(19) Europäisches Patentamt
European Patent Office
Office européen des brevets

(11) EP 2 082 743 A2

(12) EUROPEAN PATENT APPLICATION

(43) Date of publication:
**29.07.2009 Bulletin 2009/31**

(21) Application number: **09004787.9**

(22) Date of filing: **29.04.2002**

(51) Int Cl.:
***A61K 31/495** (2006.01)* ***A61K 31/50** (2006.01)*
***A61P 25/00** (2006.01)* ***C07D 487/04** (2006.01)*
*C07D 237/00 (2006.01)* *C07D 231/00 (2006.01)*

(84) Designated Contracting States:
**AT BE CH CY DE DK ES FI FR GB GR IE IT LI LU MC NL PT SE TR**

(30) Priority: **30.04.2001 US 287366 P**
**08.06.2001 US 297094 P**
**27.02.2002 US 361899 P**

(62) Document number(s) of the earlier application(s) in accordance with Art. 76 EPC:
**02729056.8 / 1 435 957**

(71) Applicant: **VERTEX PHARMACEUTICALS INCORPORATED**
**Cambridge, MA 02139-4242 (US)**

(72) Inventors:
- **Ter Haar, Ernst**
  **Roslindale, MA 02131 (US)**
- **Swenson, Lovorka**
  **Belmont, MA 02478 (US)**
- **Green, Jeremy**
  **Burlington, MA 01803 (US)**
- **Arnost, Michael J.**
  **North Andover, MA 01845 (US)**

(74) Representative: **Vossius & Partner**
**Siebertstraße 4**
**81675 München (DE)**

Remarks:
This application was filed on 31-03-2009 as a divisional application to the application mentioned under INID code 62.

# (54) Inhibitors of GSK-3 and crystal structures of GSK-3beta protein and protein complexes

(57) The present invention relates to inhibitors of GSK-3 and methods for producing these inhibitors. The invention also provides pharmaceutical compositions comprising the inhibitors and methods of utilizing those compositions in the treatment and prevention of various disorders, such as diabetes and Alzheimer's disease. In addition, the invention relates to molecules or molecular complexes which comprise binding pockets of GSK-3β or its homologues. The invention relates to a computer comprising a data storage medium encoded with the structure coordinates of such binding pockets. The invention also relates to methods of using the structure coordinates to solve the structure of homologous proteins or protein complexes. The invention relates to methods of using the structure coordinates to screen for and design compounds that bind to GSK-3β protein or homologues thereof. The invention also relates to crystallizable compositions and crystals comprising GSK-3β protein or GSK-3β protein complexes.

EP 2 082 743 A2

## Description

### TECHNICAL FIELD OF INVENTION

The present invention relates to inhibitors of glycogen synthase kinase-3 (GSK-3), a serine/threonine protein kinase, and to methods for producing them. The invention also provides pharmaceutical compositions comprising the inhibitors of the invention and methods of utilizing those compositions in the treatment and prevention of various disease states, such as diabetes and Alzheimer's disease. The present invention also relates to molecules or molecular complexes which comprise binding pockets of GSK-3β, or its homologues. The present invention provides a computer comprising a data storage medium encoded with the structure coordinates of such binding pockets. This invention also relates to methods of using the structure coordinates to solve the structure of homologous proteins or protein complexes. In addition, this invention relates to methods of using the structure coordinates to screen for and design compounds, including inhibitory compounds, that bind to GSK-3β protein or homologues thereof. The invention also relates to crystallizable compositions and crystals comprising GSK-3β protein or GSK-3β protein complexes.

### BACKGROUND OF THE INVENTION

Protein kinases mediate intracellular signal transduction by affecting a phosphoryl transfer from a nucleoside triphosphate to a protein acceptor involved in a signaling pathway. There are a number of kinases and pathways through which extracellular and other stimuli cause a variety of cellular responses to occur inside the cell. Examples of such stimuli include environmental and chemical stress signals (e.g., osmotic shock, heat shock, ultraviolet radiation, bacterial endotoxin, $H_2O_2$), cytokines (e.g., interleukin-1 (IL-1) and tumor necrosis factor α (TNF-α)), growth factors (e.g., granulocyte macrophage-colony-stimulating factor (GM-CSF), and fibroblast growth factor (FGF). An extracellular stimulus may affect one or more cellular responses related to cell growth, migration, differentiation, secretion of hormones, activation of transcription factors, muscle contraction, glucose metabolism, control of protein synthesis and regulation of cell cycle. Many disease states are associated with abnormal cellular responses triggered by protein kinase-mediated events. These diseases include autoimmune diseases, inflammatory diseases, neurological and neurodegenerative diseases, cancer, cardiovascular diseases, allergies and asthma, Alzheimer's disease and hormone-related diseases.

GSK-3 is a serine/threonine protein kinase and belongs to the superfamily of mitogen-activated protein kinases. MAP kinases are activated by phosphorylation of threonine and/or tyrosine residues in a loop adjacent to the active site. Phosphorylation of MAP kinases is carried out by specific kinases upstream. Activated MAP kinase then phosphorylates the various substrates.

Mammalian cells have α and β isoforms of GSK-3 that are each encoded by distinct genes (Coghlan et al., Chemistry & Biology, 7, pp. 793-803 (2000); Kim and Kimmel, Curr. Opinion Genetics Dev., 10, pp. 508-514 (2000)). The core kinase sequences have 97% similarity, but the protein sequences deviate substantially outside the kinase core (Woodgett, J.R., EMBO J., 9, pp. 2431-8 (1990)). GSK-3α is 63 residues longer at the N-terminal end than GSK-3β, however the N-terminal phosphorylation site in both isoforms (S21 for GSK-3α and S9 for GSK-3β) is embedded in a conserved 7 residue motif. The two isoforms are not redundant as GSK-3β deficiency is lethal in embryogenesis due to severe liver degeneration (Hoeflich, K.P., et al., Nature, 406, pp. 86-90 (2000)).

GSK-3β has multiple phosphorylation sites. The Serine 9 and Tyrosine 216 phosphorylation sites are well described in the literature. Phosphorylation of Tyrosine 216 activates GSK-3β but phosphorylation of Serine 9 inactivates it. GSK-3β is unique among kinases in that it requires prior phosphorylation of its substrates. GSK-3β does not phosphorylate its multiple substrates in the same manner and with the same efficiency but has different modes of phosphorylation. The canonical phosphorylation sequence recognized by GSK-3β, SXXXS, contains two serines separated by three amino acid residues. Multiple copies of this motif can be present in the substrate. Several protein substrates such as glycogen synthase, eIF2b and APC, are first phosphorylated by a different kinase at the P+4 serine in the $_{p+4}SXXXS_p$ motif before GSK-3β phosphorylates the serine in the P position. This is called primed phosphorylation, and is approximately 100 to 1000 times faster than the phosphorylation without priming (Thomas, G.M., et al., FEBS Lett., 458, pp. 247-51 (1999)). Glycogen synthase has multiple serines separated by four residues (residue 640, 644, 648, and 652) and those serines are phosphorylated sequentially by GSK-3β from the C-terminal end, after S656 has been phosphorylated by Casein Kinase II (Woodgett, J.R. and P. Cohen, Biochim. Biophys. Acta, 788, pp. 339-47 (1984); Kuret, J. et al, Eur. J. Biochem., 151, pp. 39-48 (1985)).

Glycogen synthase kinase-3 has been implicated in various diseases including diabetes, Alzheimer's disease, CNS disorders such as manic depressive disorder and neurodegenerative diseases, and cardiomyocete hypertrophy (WO 99/65897; WO 00/38675; and Haq et al., J. Cell Biol., 151, pp. 117 (2000)). These diseases may be caused by, or result in, the abnormal operation of certain cell signaling pathways in which GSK-3 plays a role. GSK-3 phosphorylates and modulates the activity of a number of regulatory proteins. These include glycogen synthase which is the rate limiting enzyme necessary for glycogen synthesis, the microtubule associated protein Tau, the gene transcription factor beta-catenin, the translation initiation factor e1F2B, as well as ATP citrate lyase, axin, heat shock factor-1, c-Jun, c-Myc, c-Myb, CREB and CEPBa. These diverse targets implicate GSK-3 in many aspects of cellular metabolism, proliferation, differentiation and development.

In a GSK-3 mediated pathway that is relevant for the treatment of type II diabetes, insulin-induced signaling leads to cellular glucose uptake and glycogen synthesis. Along this pathway, GSK-3 is a negative regulator of the insulin-induced signal. Insulin inactivates GSK-3β via the PKB/Akt pathway, which results in activation of glycogen synthase. (Summers, S.A., et al., J. Biol. Chem., 274, pp. 17934-40 (1999); Ross, S.E., et al., Mol. Cell. Biol., 19, pp. 8433-41 (1999)). The inhibition of GSK-3 leads to increased glycogen synthesis and glucose uptake (Klein et al., PNAS, 93, pp. 8455-9 (1996); Cross et al., Biochem. J., 303, pp. 21-26 (1994); Cohen, Biochem. Soc. Trans., 21, pp. 555-567(1993); Massillon et al., Biochem. J. 299, pp. 123-128 (1994)). However, in a diabetic patient where the insulin response is impaired, glycogen synthesis and glucose uptake fail to increase despite the presence of relatively high blood levels of insulin. This leads to abnormally high blood levels of glucose with acute and long term effects that may ultimately result in cardiovascular diseases, renal failure and blindness. In such patients, the normal insulin-induced inhibition of GSK-3 fails to occur. It has also been reported that in patients with type II diabetes, GSK-3 is overexpressed (WO 00/38675). Therapeutic inhibitors of GSK-3 are therefore potentially useful for treating diabetic patients suffering from an impaired response to insulin.

GSK-3 activity has also been associated with Alzheimer's disease. This disease is characterized by the well-known β-amyloid peptide and the formation of intracellular neurofibrillary tangles. The neurofibrillary tangles contain hyperphosphorylated Tau protein where Tau is phosphorylated on abnormal sites. GSK-3 has been shown to phosphorylate these abnormal sites in cell and animal models. Furthermore, inhibition of GSK-3 has been shown to prevent hyperphosphorylation of Tau in cells (Lovestone et al., Current Biology, 4, pp. 1077-86 (1994); Brownlees et al., Neuroreport, 8, pp. 3251-55 (1997)). Therefore, it is believed that GSK-3 activity may promote generation of the neurofibrillary tangles and the progression of Alzheimer's disease.

Another substrate of GSK-3 is beta-catenin which is degraded after phosphorylation by GSK-3. Reduced levels of beta-catenin have been reported in schizophrenic patients and have also been associated with other diseases related to increase in neuronal cell death (Zhong et al., Nature, 395, 698-702 (1998); Takashima et al., PNAS, 90, 7789-93 (1993); Pei et al., J. Neuropathol. Exp., 56, 70-78 (1997)).

GSK-3β is also a component of the Wnt signalling pathway. Activation of the Wnt pathway inhibits GSK-3β, which results in accumulation of cytosolic β-catenin (Yost, C., et al., Cell, 93, pp. 1031-41 (1998)). The cytosolic β-catenin translocates to the cell nucleus, where it associates with LEF/tcf and stimulates the expression of Wnt target genes resulting in cell proliferation (Ding, V.W., et al., J. Biol. Chem., 275, pp. 32475-81 (2000); Waltzer, L. and M. Bienz, Cancer Metastasis Rev. 18, pp. 231-46 (1999); Ikeda, S., et al., EMBO J., 17, pp. 1371-84 (1998); Thomas, G.M., et al., FEBS Lett, 458, pp. 247-51 (1999); Salic, A., et al., Mol. Cell., 5, pp. 523-32 (2000)). The activity of GSK-3β is also down regulated by 7-TM receptors that regulate cAMP levels. cAMP-dependent protein kinase A, binds, phosphorylates and inhibits GSK-3β in response to the adenyl cyclase activator forskolin, or the p-adrenergic receptor activator isoproterenol (Fang, X., et al., Proc. Natl. Acad. Sci. U S A, 97, pp. 11960-5 (2000)).

Small molecule inhibitors of GSK-3 have recently been reported (WO 99/65897 and WO 00/38675). For many of the aforementioned diseases associated with abnormal GSK-3 activity, other protein kinases have also been targeted for treating the same diseases. However, the various protein kinases often act through different biological pathways. Quinazoline derivatives have been reported recently as inhibitors of p38 kinase (WO 00/12497). The compounds are reported to be useful for treating conditions characterized by enhanced p38-α activity and/or enhanced TGF-β activity. While p38 activity has been implicated in a wide variety of diseases, including diabetes, p38 kinase is not reported to be a constituent of an insulin signaling pathway that regulates glycogen synthesis or glucose uptake. Therefore, unlike GSK-3, p38 inhibition would not be expected to enhance glycogen synthesis and/or glucose uptake.

Accordingly, there has been an interest in finding GSK-3 inhibitors that are effective as therapeutic agents due to its important role in diabetes, Alzheimer's disease and other diseases. A challenge has been to find protein kinase inhibitors that act in a selective manner. Since there are numerous protein kinases that are involved in a variety of cellular responses, non-selective inhibitors may lead to unwanted side effects.

In this regard, the three-dimensional structure of the kinase would assist in the rational design of inhibitors. Further, information provided by the X-ray crystal structure of GSK-3β-inhibitor complexes would be extremely useful in iterative drug design of various GSK-3 proteins. The determination of the amino acid residues in GSK-3β binding pockets and the determination of the shape of those binding pockets would allow one to design inhibitors that bind more favorably to this class of enzymes.

## SUMMARY OF THE INVENTION

The present invention addresses this need by providing compounds and pharmaceutical compositions thereof that are effective as protein kinase inhibitors, particularly as inhibitors of GSK-3. Applicants have also addressed this

need by providing the crystal structures of a unphosphorylated GSK-3β, a phosphorylated GSK-3β, unphosphorylated GSK-3β-inhibitor complexes, a phosphorylated GSK-3β-inhibitor complex and a phosphorylated GSK-3β-ADP-peptide complex. Solving these crystal structures has allowed the determination of the key structural features of GSK-3β, particularly the shape of its substrate and ATP-binding pockets.

The compounds of the present invention have the general formula I:

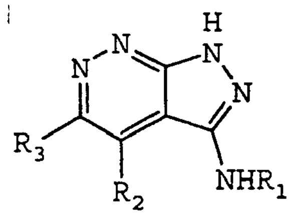

I

or a pharmaceutically acceptable derivative thereof, wherein:

$R_1$ is selected from H, aliphatic, RC(O)-, $RS(O)_n$-, ROC(O)-, carbocyclyl, heterocyclyl, aryl, aralkyl, heteroaryl, or heteroaralkyl; wherein said aliphatic, carbocyclyl, heterocyclyl, aryl, aralkyl, heteroaryl, or heteroaralkyl is optionally substituted;

$R_2$ and $R_3$ are each independently selected from H, aliphatic, carbocyclyl, heterocyclyl, aryl, aralkyl, heteroaryl, heteroaralkyl, $-N(R)_2$, -NRCOR, - $NRCO_2R$, $-NRCO_2R$, $-S(O)_nR$, $-SO_2N(R)_2$, -SR, -OR, $-CF_3$, halo, $-NO_2$, -CN, -C(O)R, $-CO_2R$, -OC(O)R, $-CON(R)_2$, or - $OC(O)N(R)_2$, wherein said aliphatic, carbocyclyl, heterocyclyl, aryl, aralkyl, heteroaryl, or heteroaralkyl is optionally substituted; or $R_2$ and $R_3$ taken together with the intervening atoms optionally form a five- to nine-membered ring that is fused to the pyridazinyl ring of formula I, said fused ring having 0-2 heteroatoms;

each R is independently selected from H, aliphatic, carbocyclyl, heterocyclyl, aryl, aralkyl, heteroaryl, or heteroaralkyl, wherein each member of R except H is optionally substituted'; and

n is 1 or 2;

provided that when $R_1$ is H, $R_2$ and $R_3$ are not both unsubstituted phenyl; and when $R_1$ is H, $R_2$ and $R_3$ are other than H, halogen, or an unsubstituted alkyl.

In another embodiment, the invention provides pharmaceutical compositions comprising a GSK-3 inhibitor of this invention. These compositions may be utilized in methods for treating or preventing a variety of GSK-3 mediated disorders, such as autoimmune diseases, inflammatory diseases, metabolic, neurological and neurodegenerative diseases, cardiovascular diseases, allergy, asthma, diabetes, Alzheimer's disease, Huntington's Disease, Parkinson's Disease, AIDS-associated dementia, amyotrophic lateral sclerosis (AML, Lou Gehrig's Disease), multiple sclerosis (MS), schizophrenia, cardiomyocyte hypertrophy, reperfusion/ischemia, and baldness.

The compositions of this invention are also useful in methods for enhancing glycogen synthesis and/or lowering blood levels of glucose and therefore are especially useful for diabetic patients. These compositions are also useful in methods for inhibiting the production of hyperphosphorylated Tau protein, which is useful in halting or slowing the progression of Alzheimer's disease. Another embodiment of this invention relates to a method for inhibiting the phosphorylation of β-catenin, which is useful for treating schizophrenia.

In another embodiment, the invention provides methods of synthesizing compounds of formula I and preparing pharmaceutical compositions comprising these compounds.

The present invention also provides molecules or molecular complexes comprising GSK-3β binding pockets, or GSK-3β-like binding pockets that have similar three-dimensional shapes. In one embodiment, the molecules or molecular complexes are GSK-3β proteins, protein complexes or homologues thereof. In another embodiment, the molecules or molecular complexes are in crystalline form.

The invention provides crystallizable compositions and crystal compositions comprising unphosphorylated GSK-3β, phosphorylated GSK-3β or their homologues with or without a chemical entity. The invention also provides a method for crystallizing a GSK-3β protein, protein complex, or homologues thereof.

The invention provides a data storage medium which comprises the structure coordinates of molecules or molecular complexes of the GSK-3β binding pockets or GSK-3β-like binding pockets. In one embodiment, the data storage medium comprises the structure coordinates of the binding pocket. The invention also provides a computer comprising the data storage medium. Such storage medium when read and utilized by a computer programmed with appropriate software can display, on a computer screen or similar viewing device, a three-dimensional graphical representation of such binding pockets.

The invention also provides methods for designing, evaluating and identifying compounds which bind to the molecules or molecular complexes or their binding pockets. Such compounds are potential inhibitors of GSK-3β or its homologues.

The invention also provides a method for determining at least a portion of the three-dimensional structure of molecules or molecular complexes which contain at least some structurally similar features to GSK-3β, particularly GSK-3β homologues. This is achieved by using at least some of the structure coordinates obtained from the unphosphorylated or phosphorylated GSK-3β protein or protein complexes.

## BRIEF DESCRIPTION OF THE FIGURES

Figure 1 lists the atomic structure coordinates for unphosphorylated GSK-3β as derived by X-ray diffraction from the crystal.

Figure 2 lists the atomic structure coordinates for phosphorylated GSK-3β.

Figure 3 lists the atomic structure coordinates for phosphorylated GSK-3β-inhibitor1 complex (inhibitor1 is 4,5-Diphenyl-1H-pyrazolo[3,4-c]pyridazin-3-ylamine).

Figure 4 lists the atomic structure coordinates for unphosphorylated GSK-3β-inhibitor2 complex (inhibitor2 is (5-Methyl-2H-pyrazol-3-yl)-(2-pyridin-4-yl-quinazolin-4-yl)-amine)).

Figure 5 lists the atomic structure coordinates for unphosphorylated GSK-3β-inhibitor3 complex (inhibitor 3 is 4-(5-Methyl-2-phenylamino-pyrimidin-4-yl)-1H-pyrrole-2-carboxylic acid (2-hydroxy-1-phenyl-ethyl)-amide).

Figure 6 lists the atomic structure coordinates for unphosphorylated GSK-3β-inhibitor4 complex (inhibitor 4 is (1H-Indazol-3-yl)-[2-(2-trifluoromethyl-phenyl)-quinazolin-4-yl]-amine).

Figure 7 lists the atomic structure coordinates for phosphorylated GSK-3β in complex with ADP and glycogen synthase peptide.

Figure 8 depicts a ribbon diagram of the overall fold of unphosphorylated GSK-3β. The N-terminal domain corresponds to the β-strand domain and encompasses residues 25 to 138. β-strand 1 was only visible in one of the two molecules in the asymmetric unit and makes hydrogen bonds with β-strand 2 although it is not part of the β-barrel. The α-helical domain corresponds to residues 139 to 349. Key features of the kinase-fold such as the hinge, glycine rich loop and activation-loop are indicated.

Figure 9 depicts a superposition of unphosphorylated GSK-3β (light shade) and activated substrate-bound CDK2 (Protein Data Bank accession number 1QMZ) (dark shade). The α-helical domains of GSK-3β and CDK2 were superimposed in QUANTA by aligning matching residues.

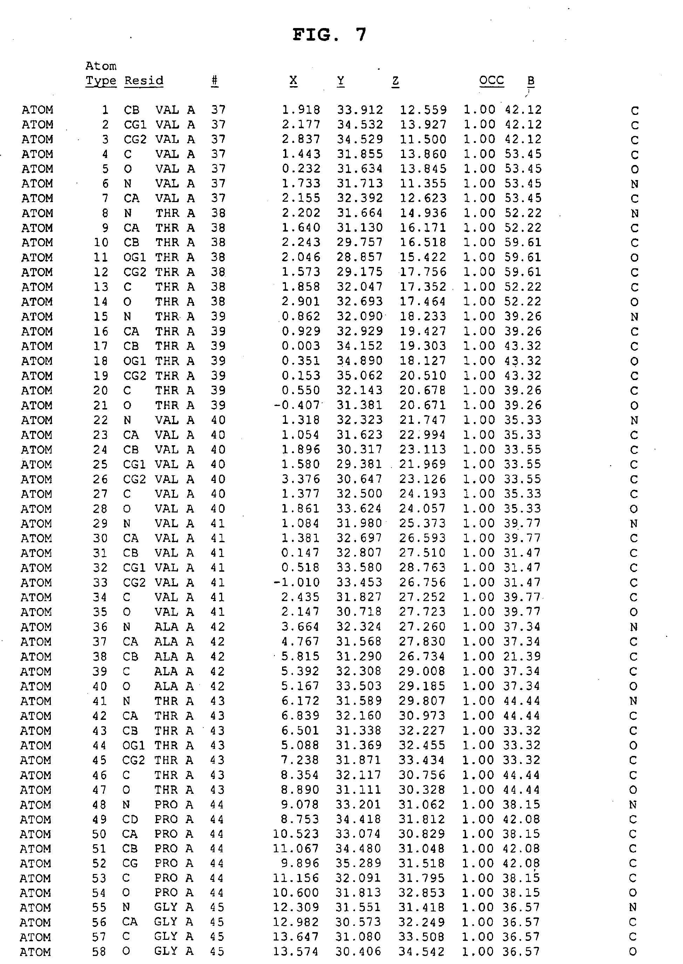

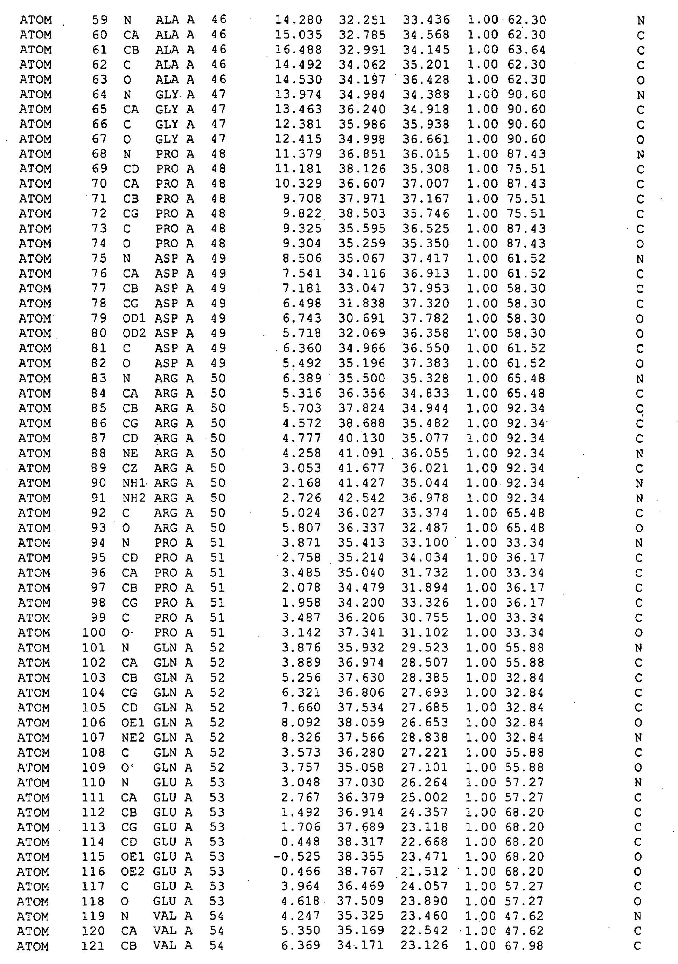

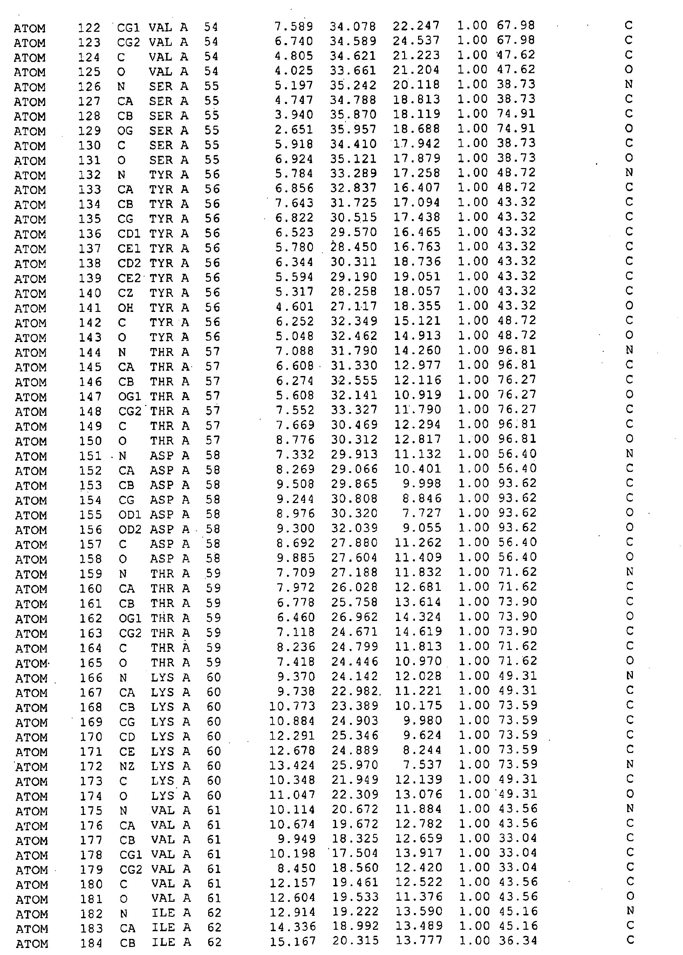

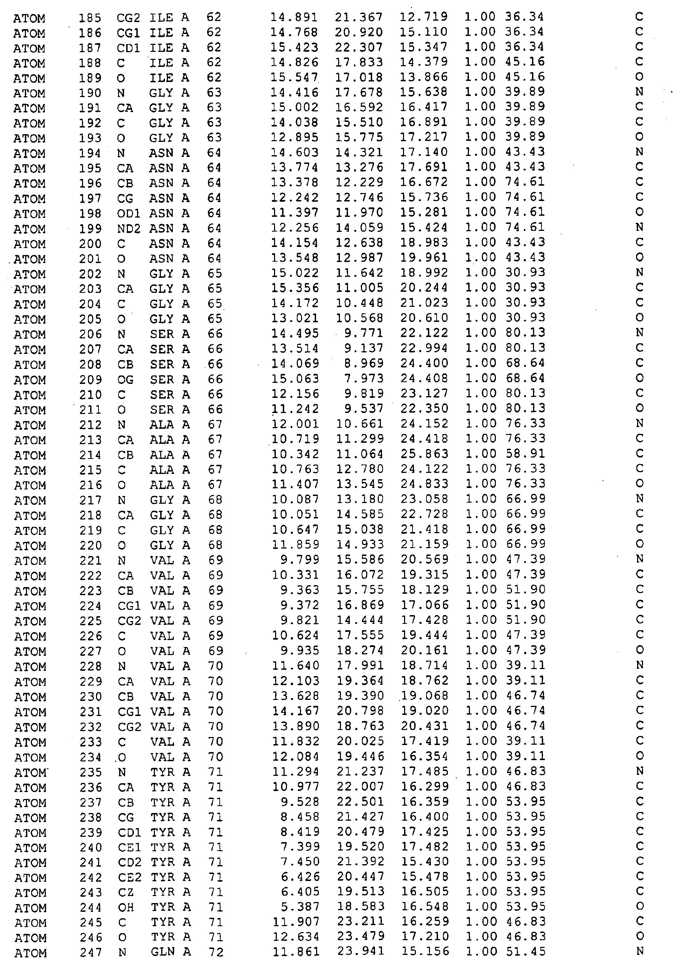

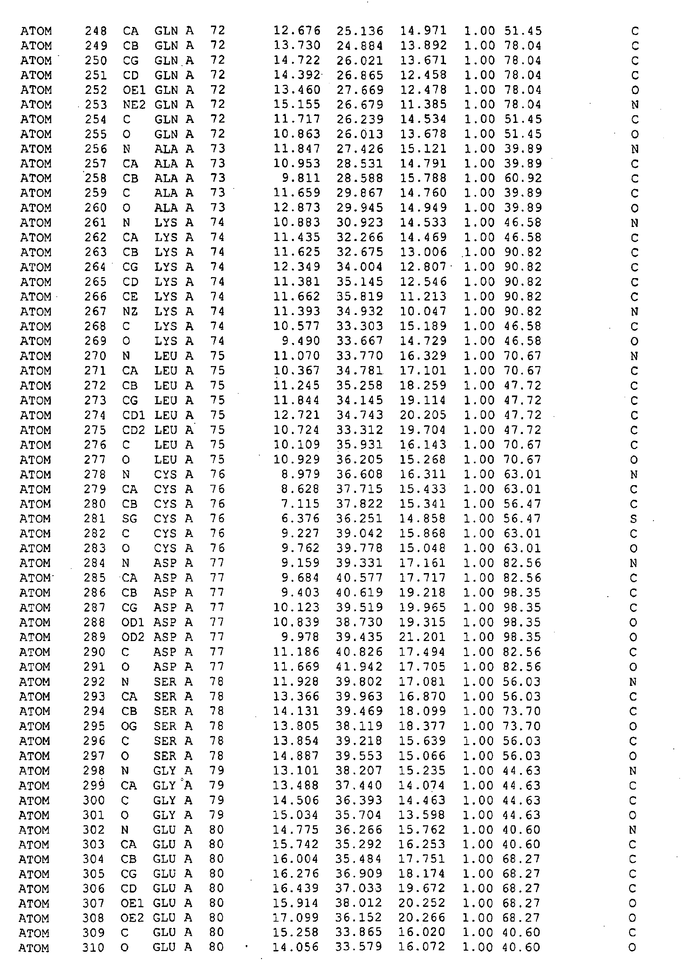

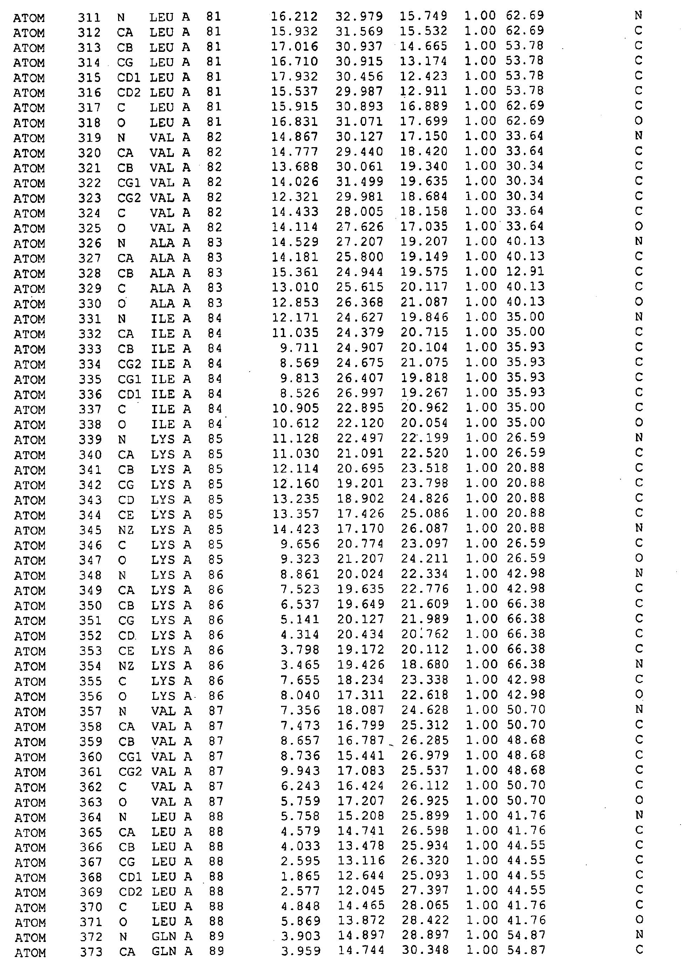

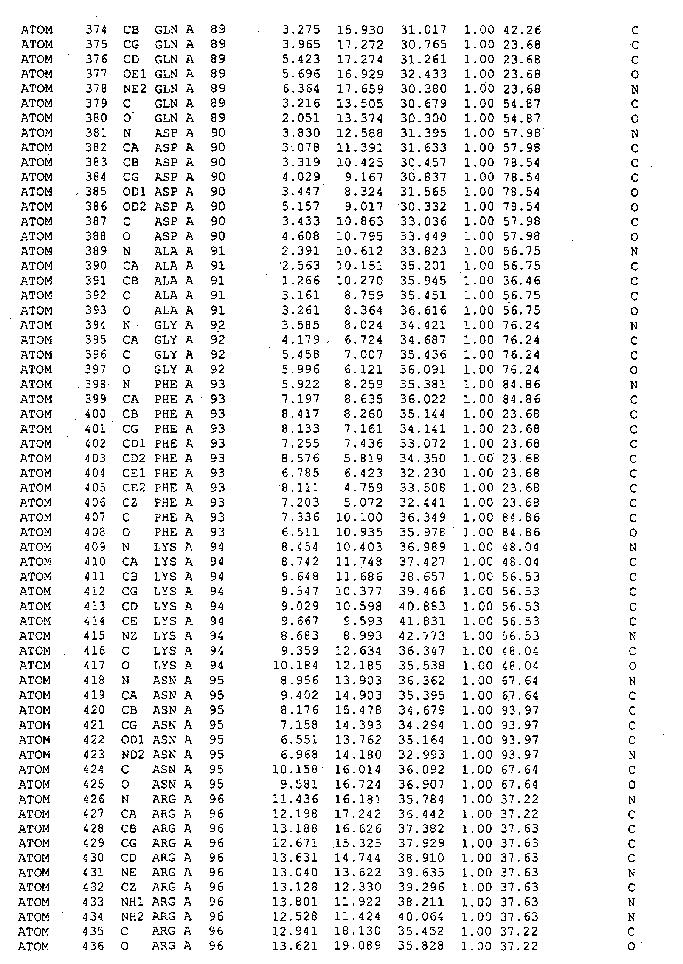

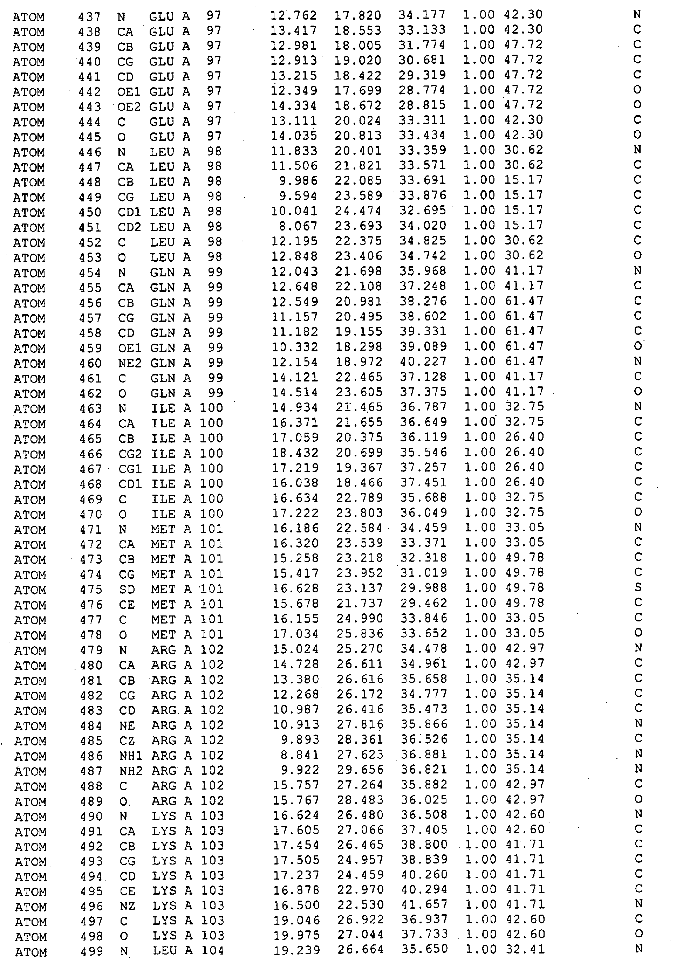

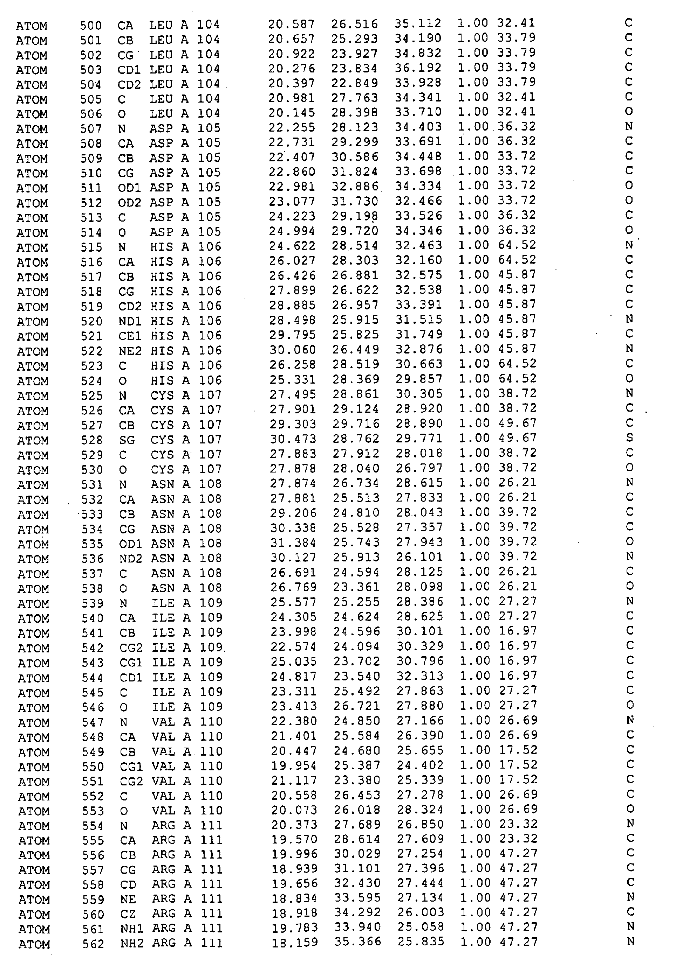

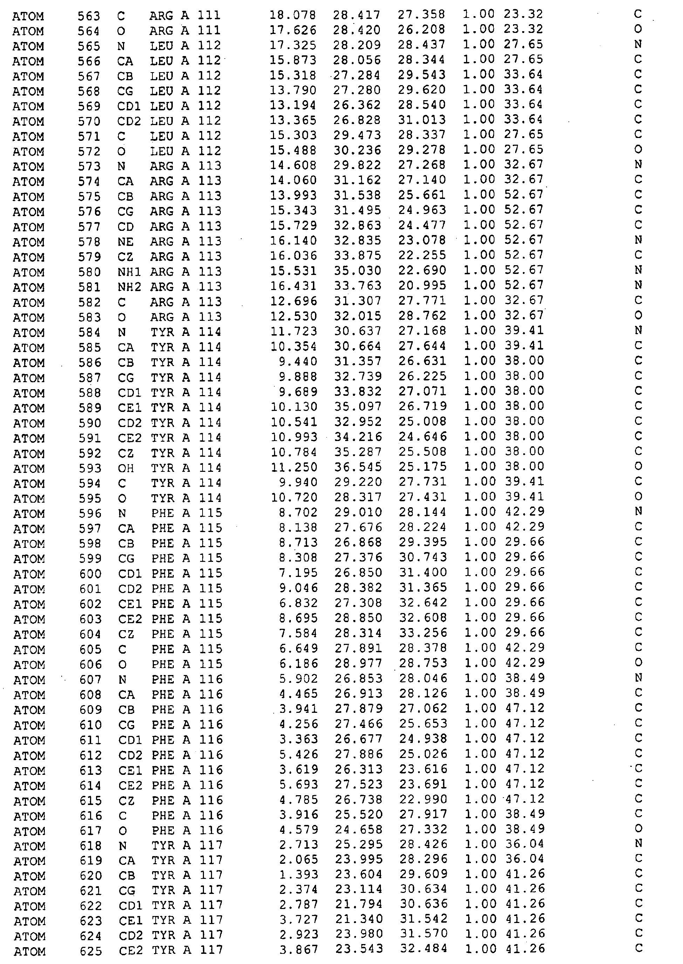

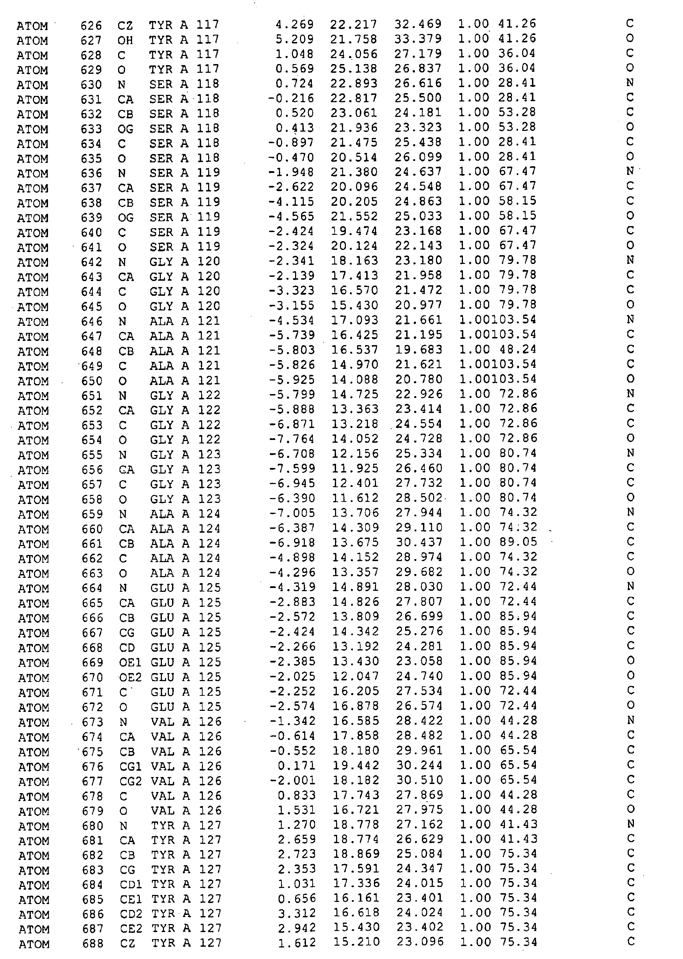

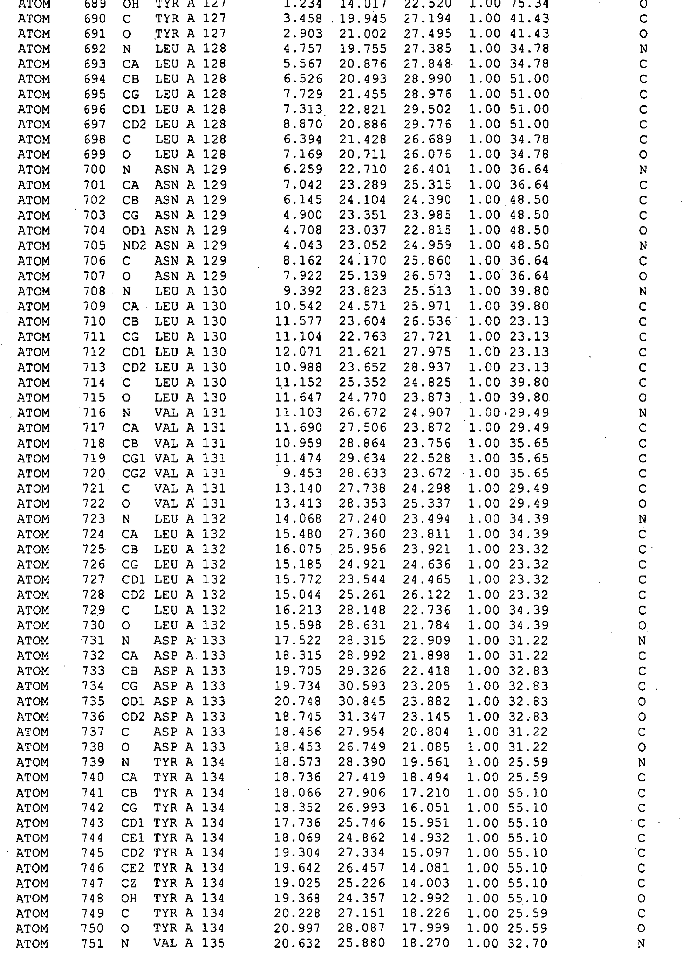

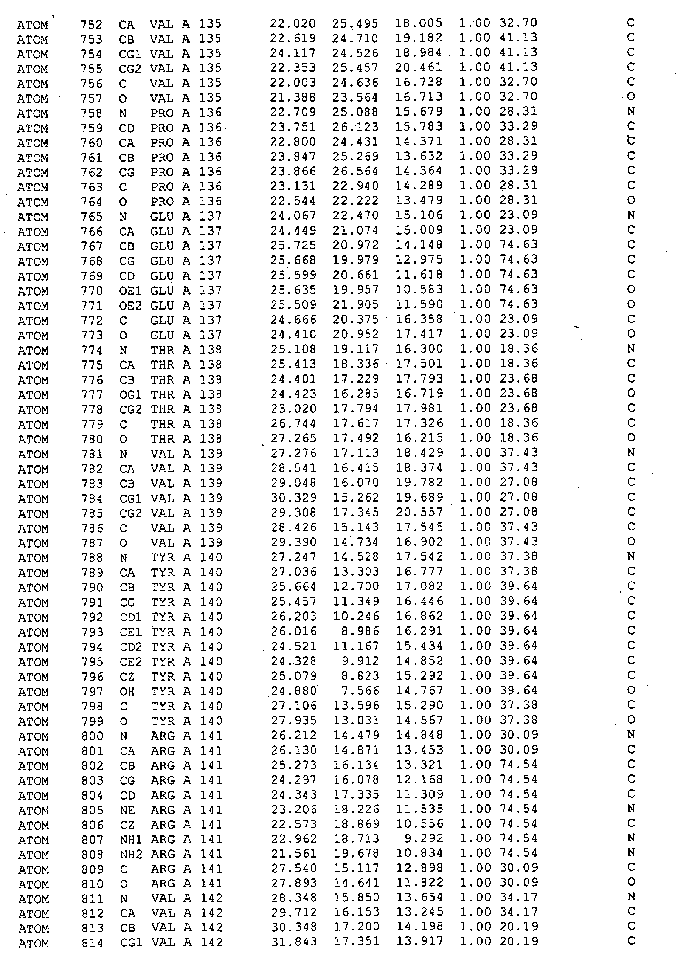

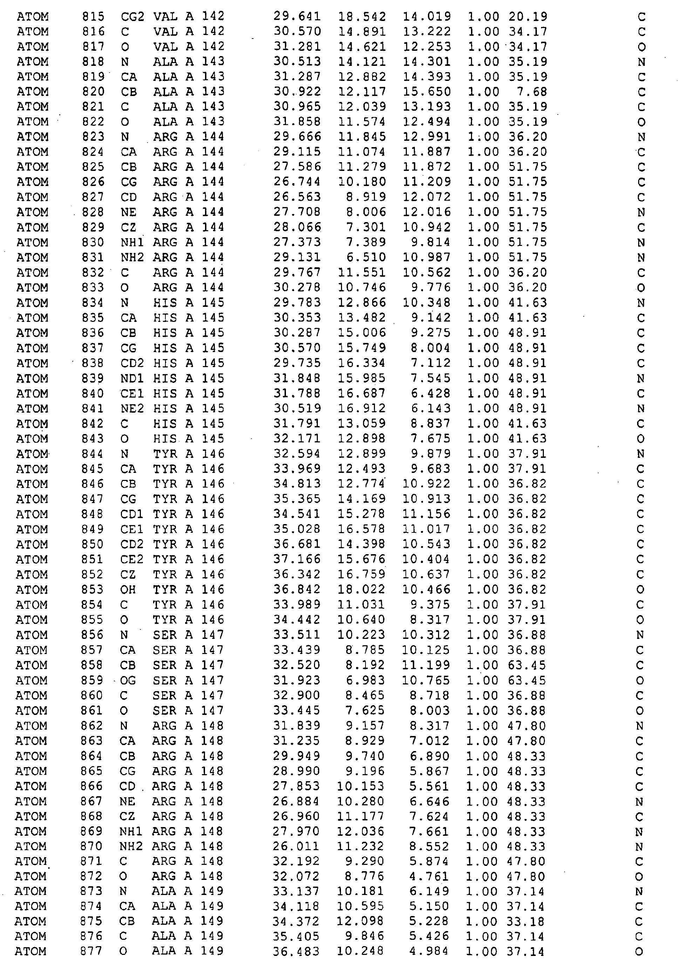

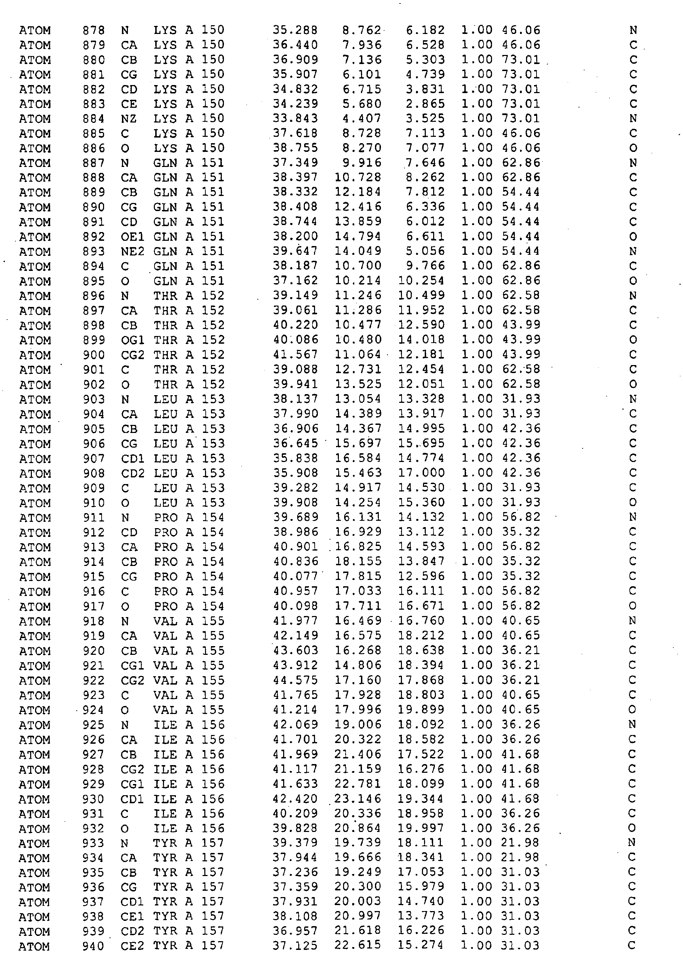

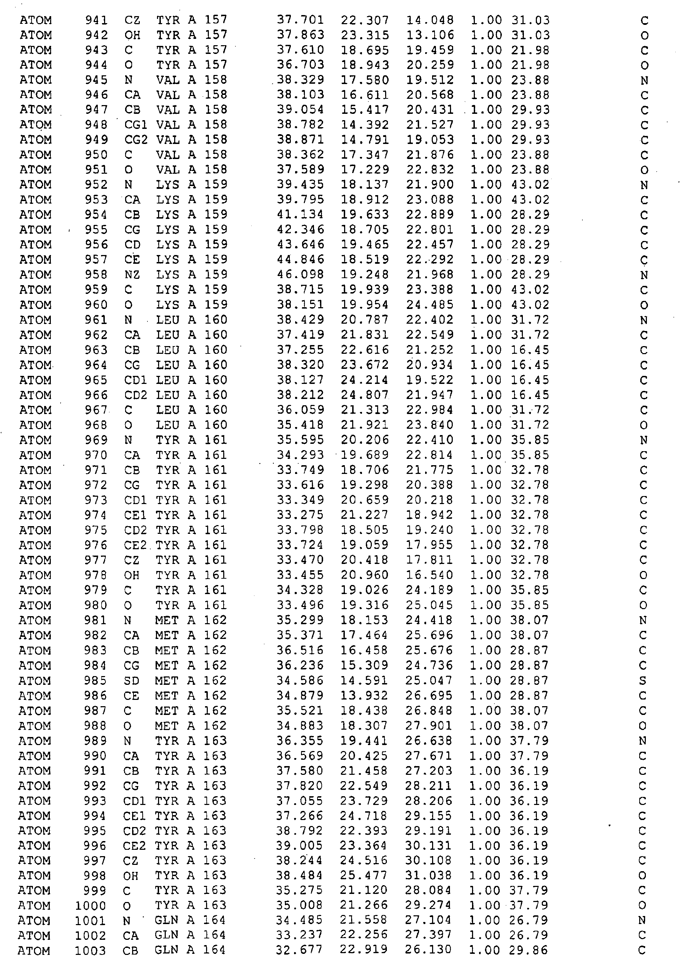

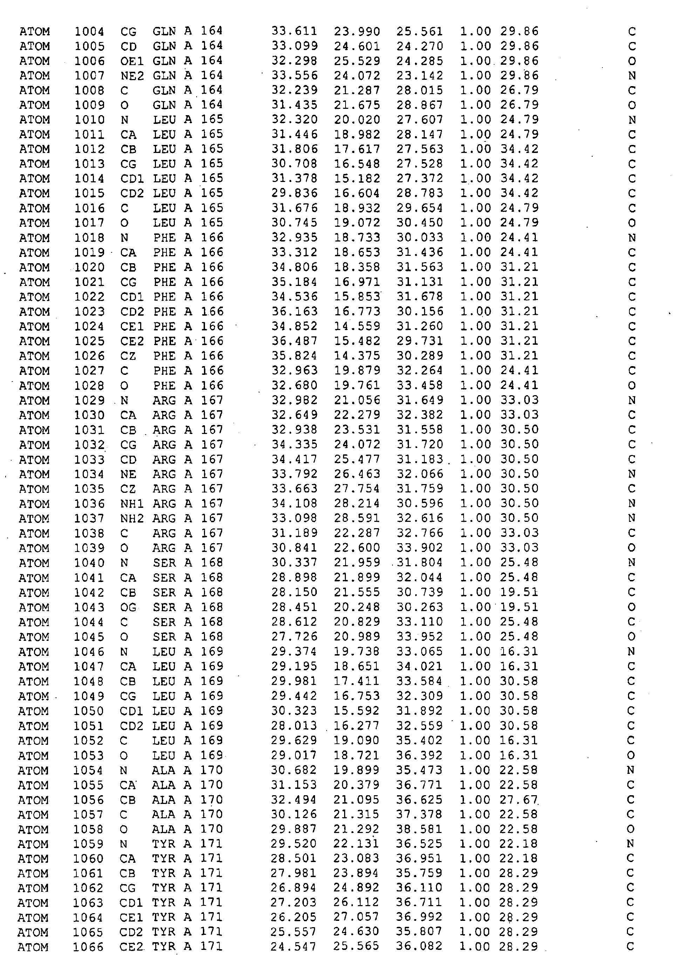

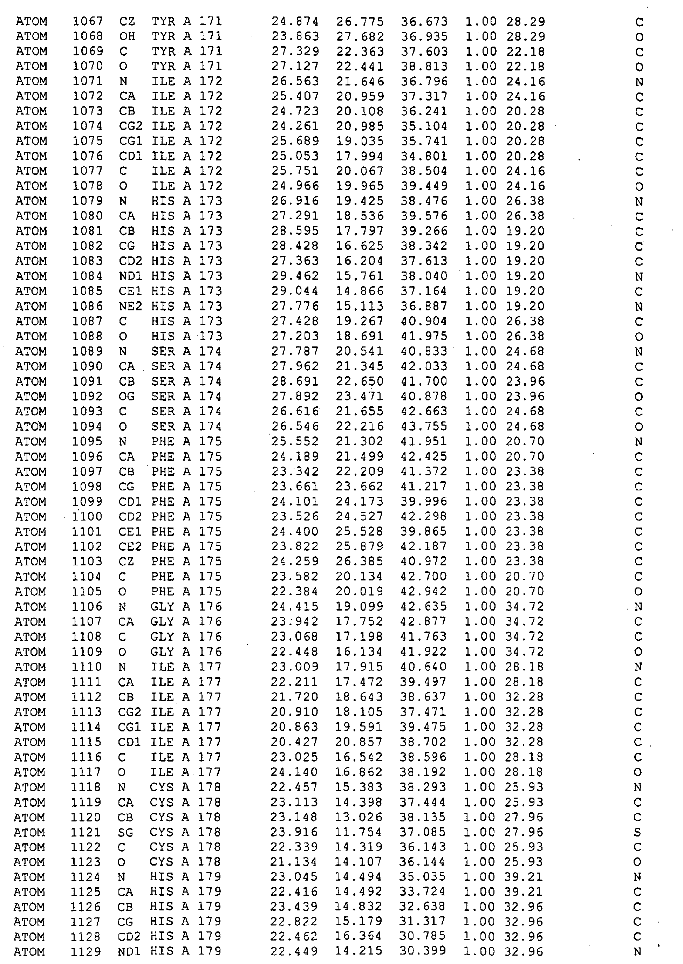

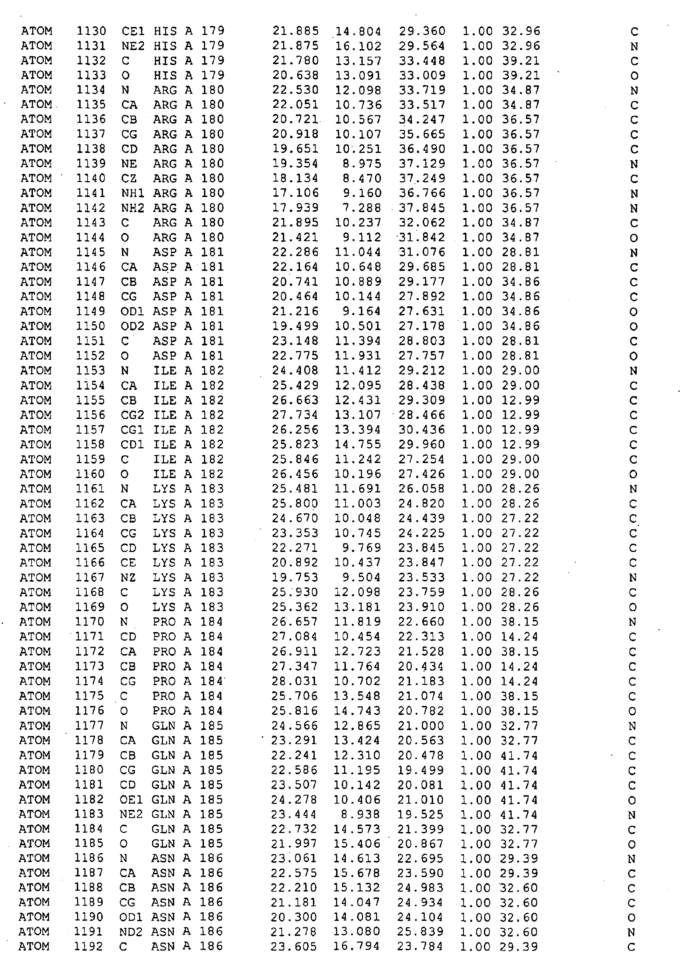

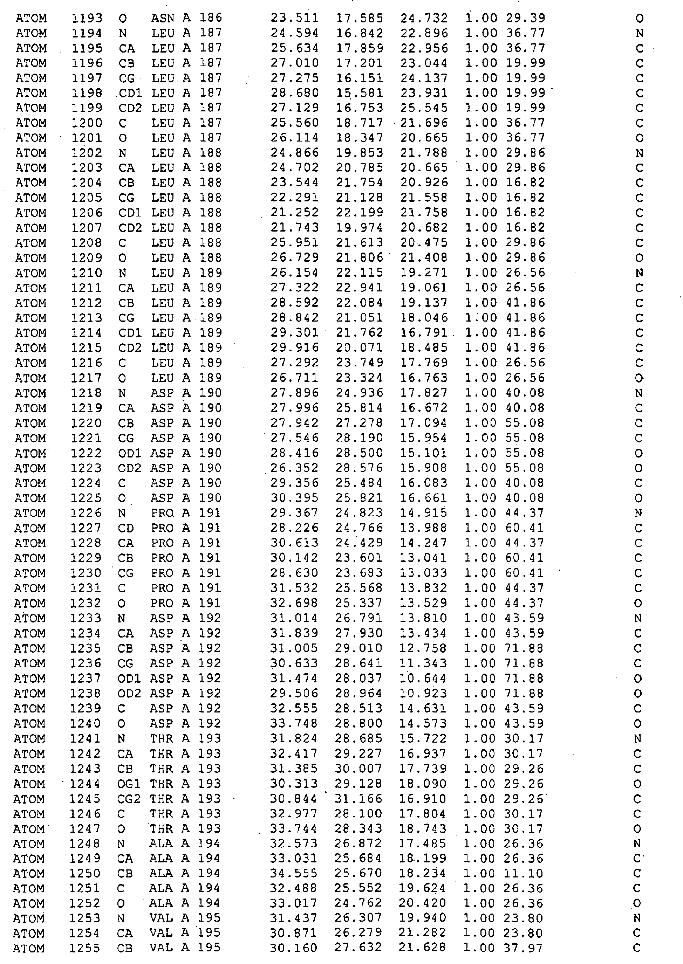

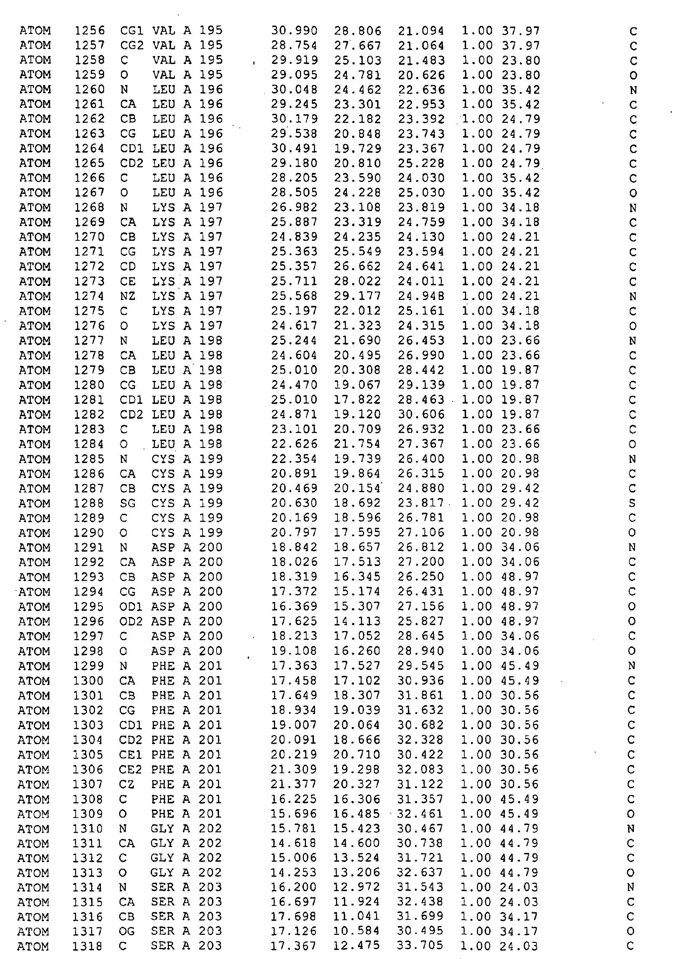

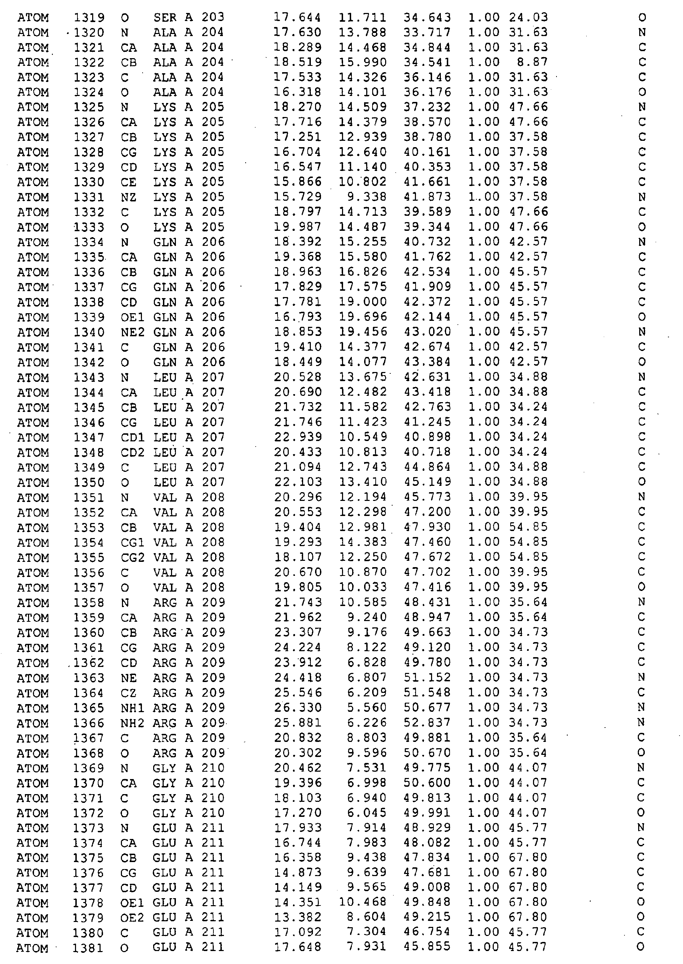

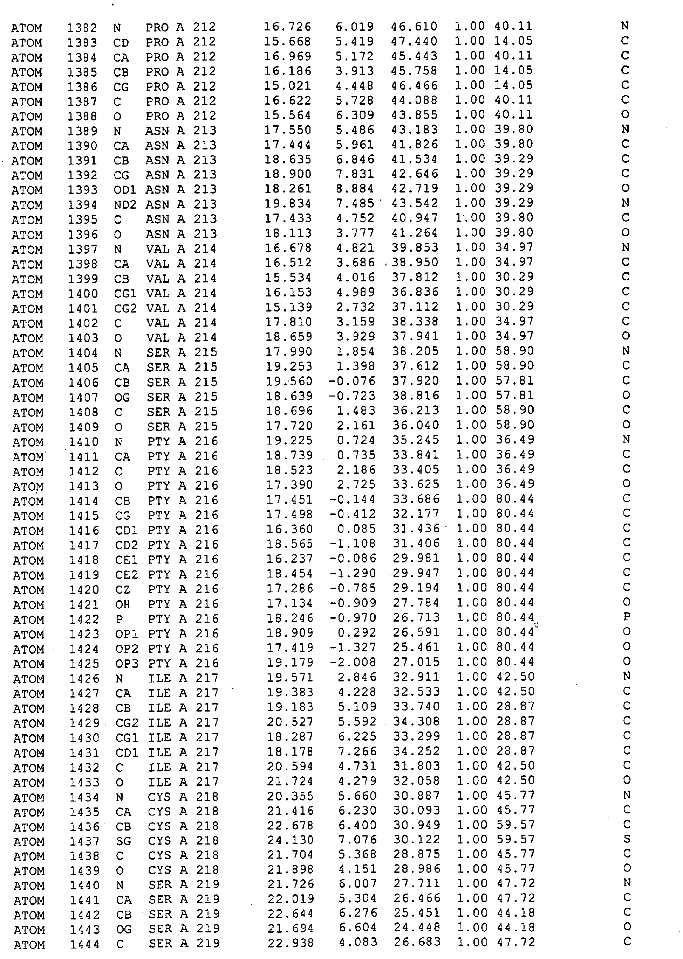

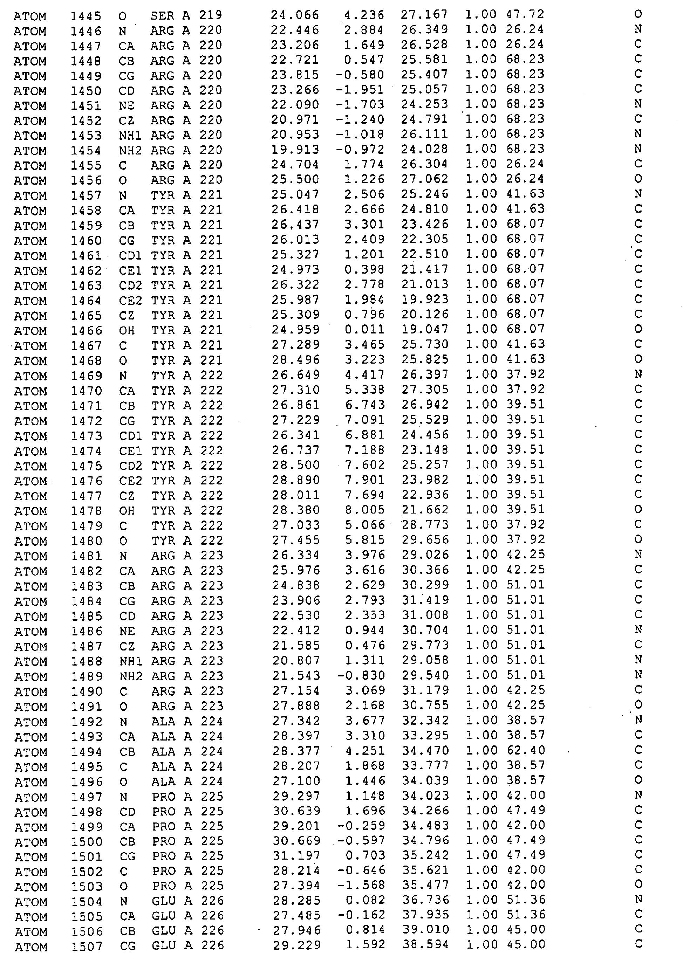

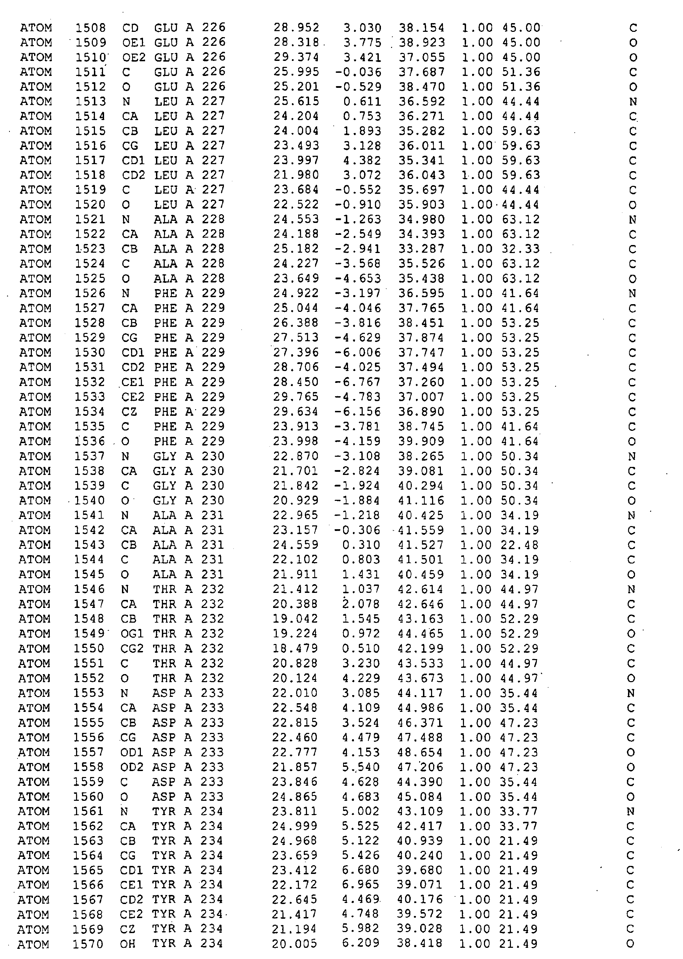

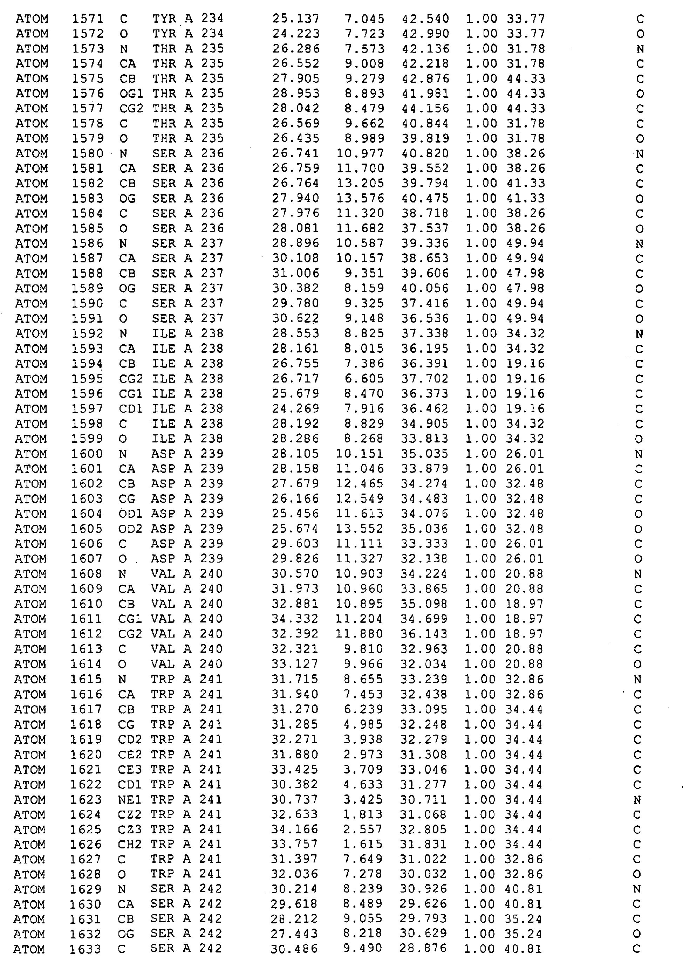

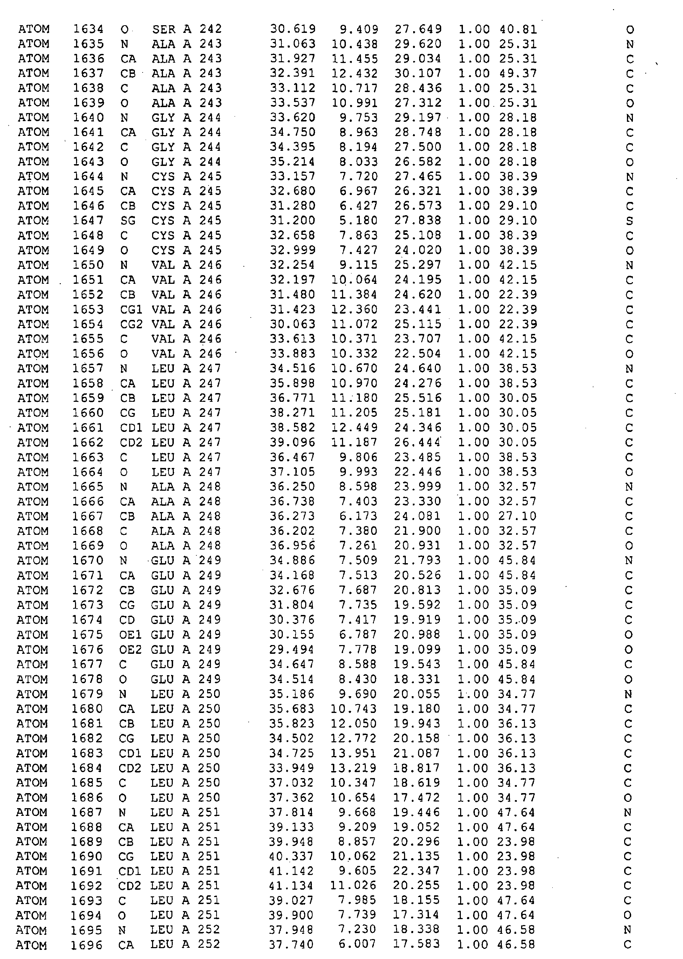

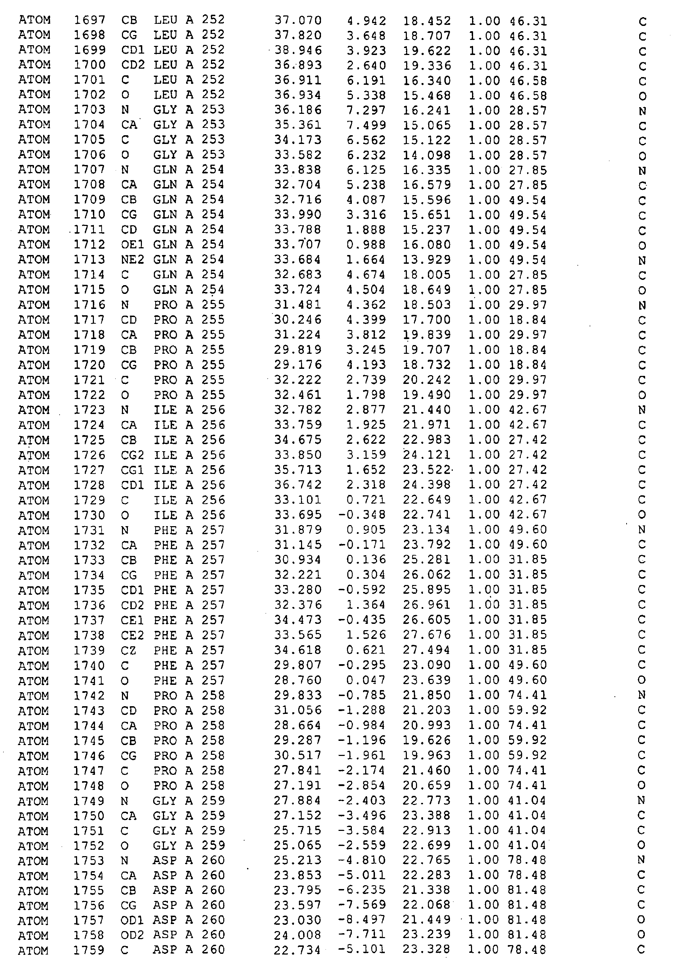

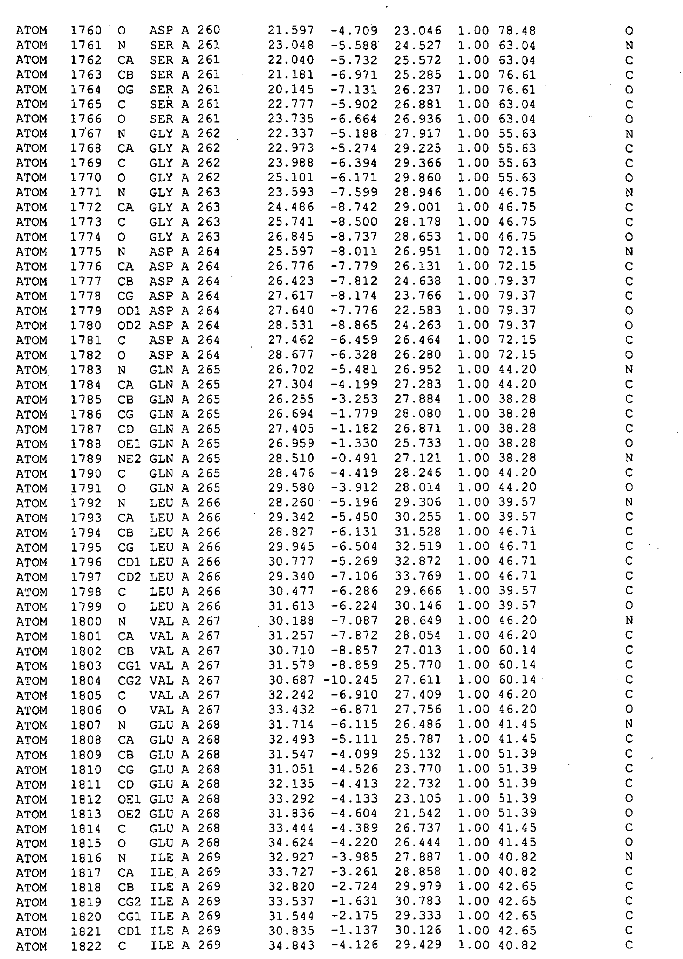

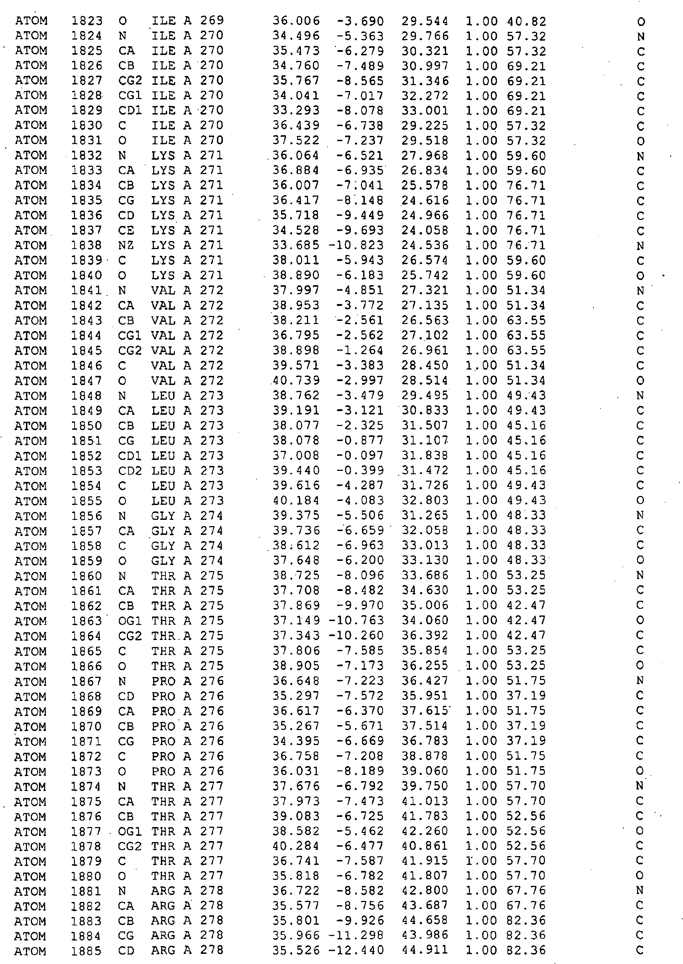

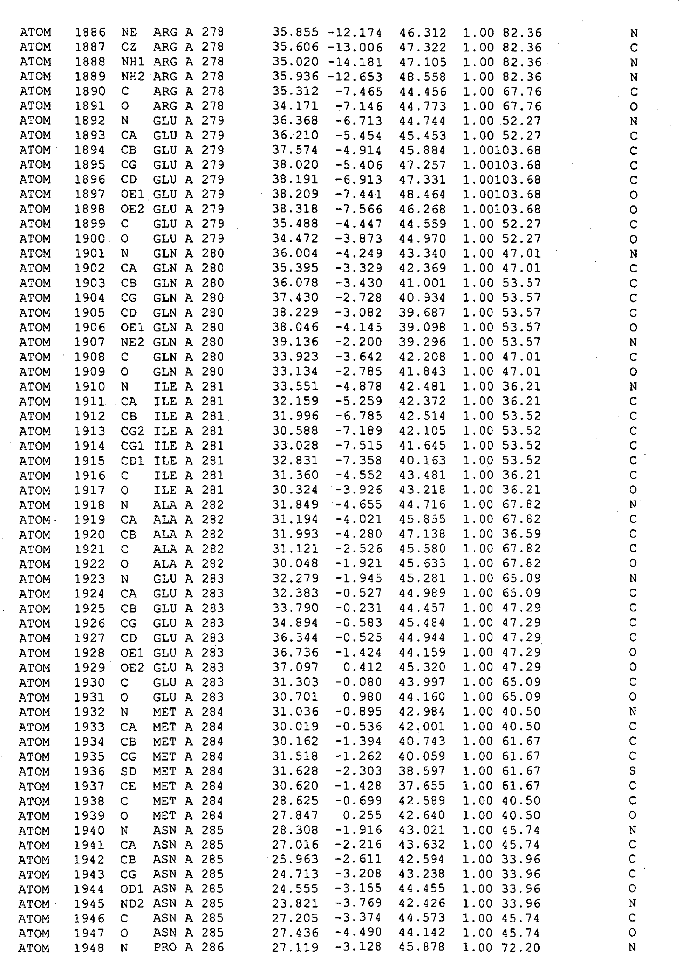

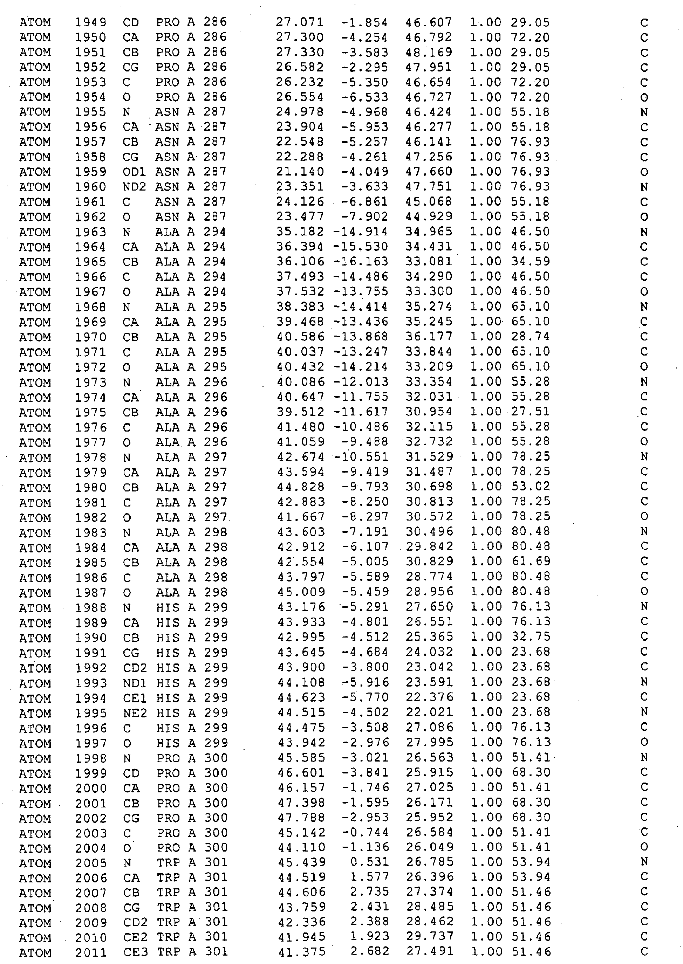

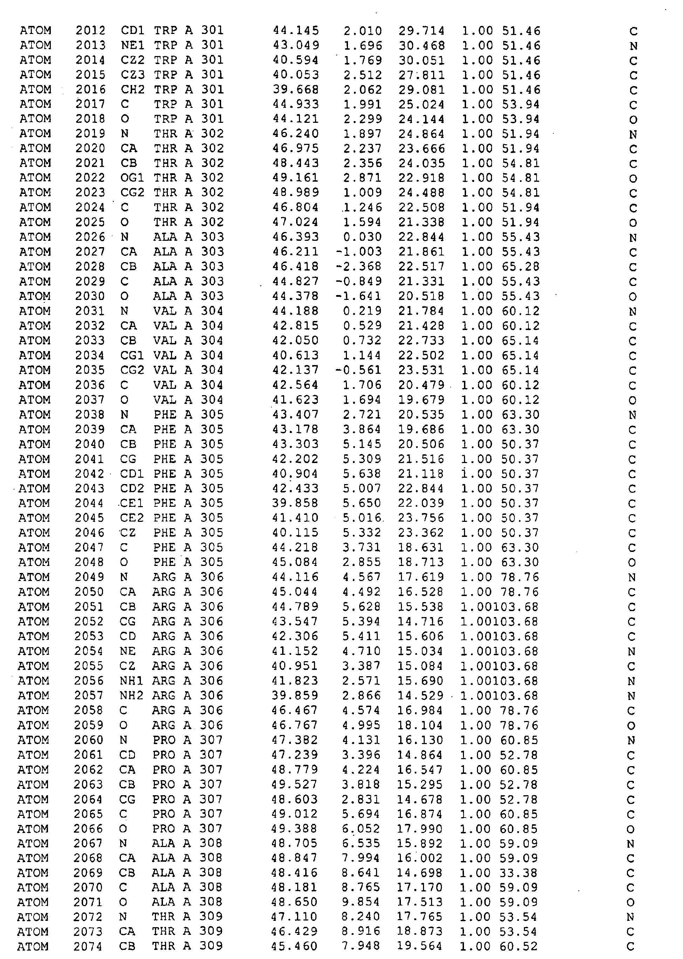

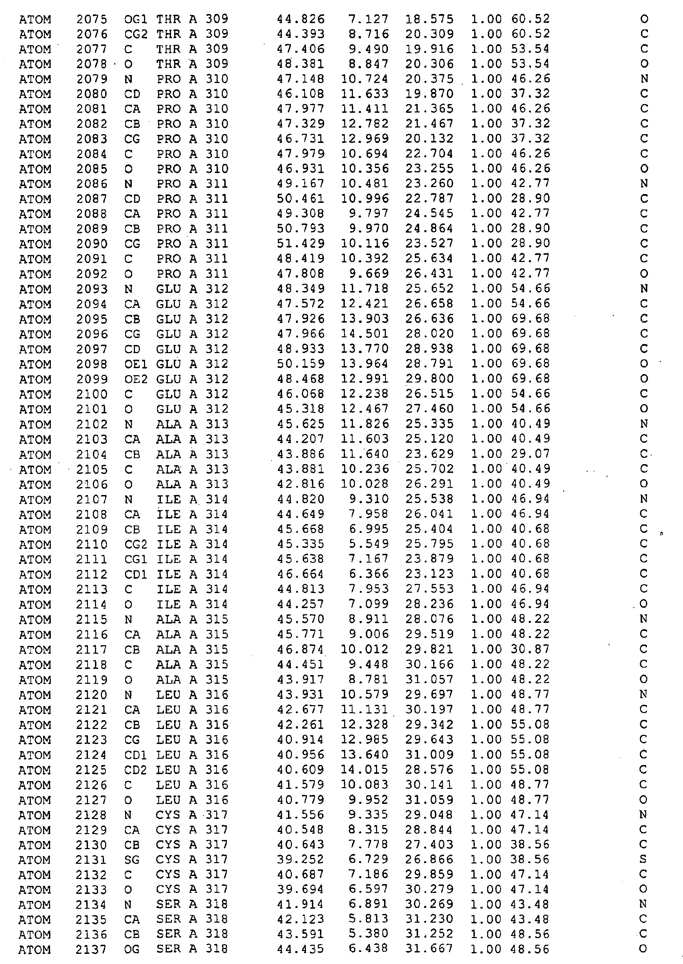

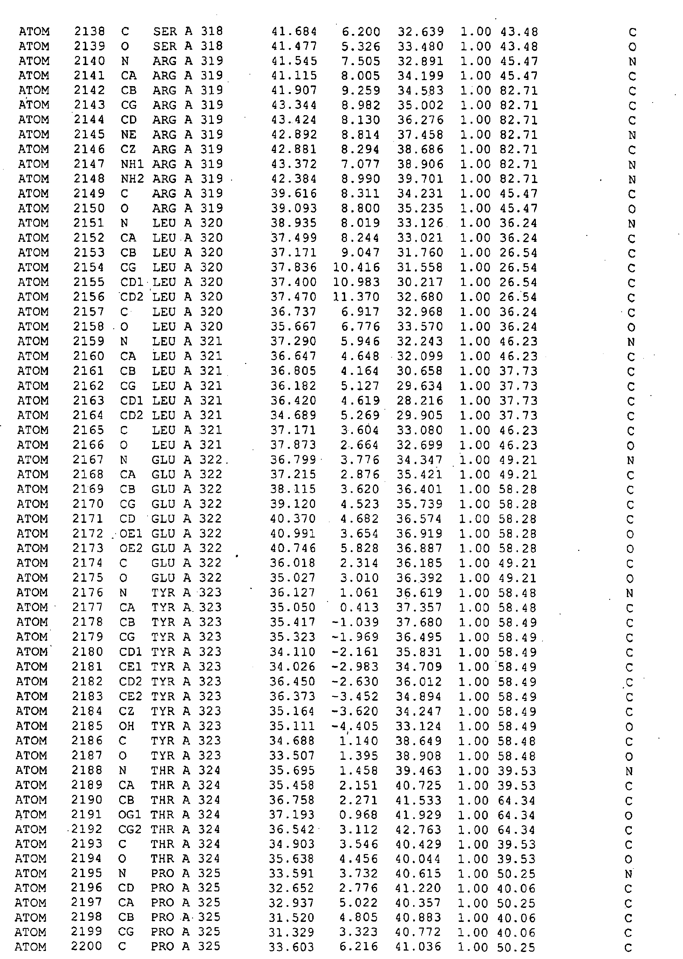

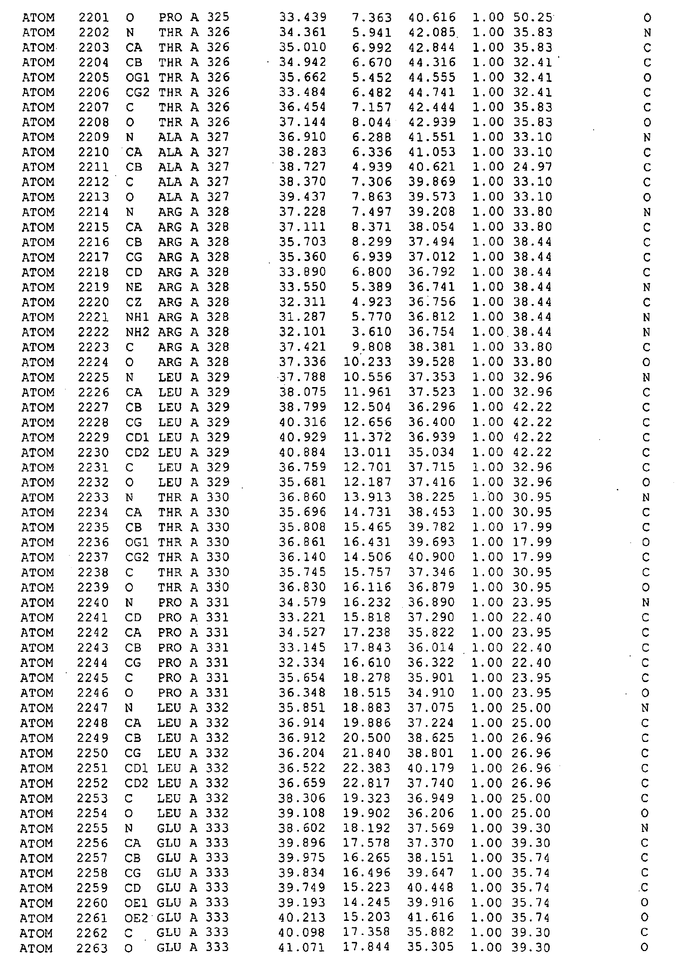

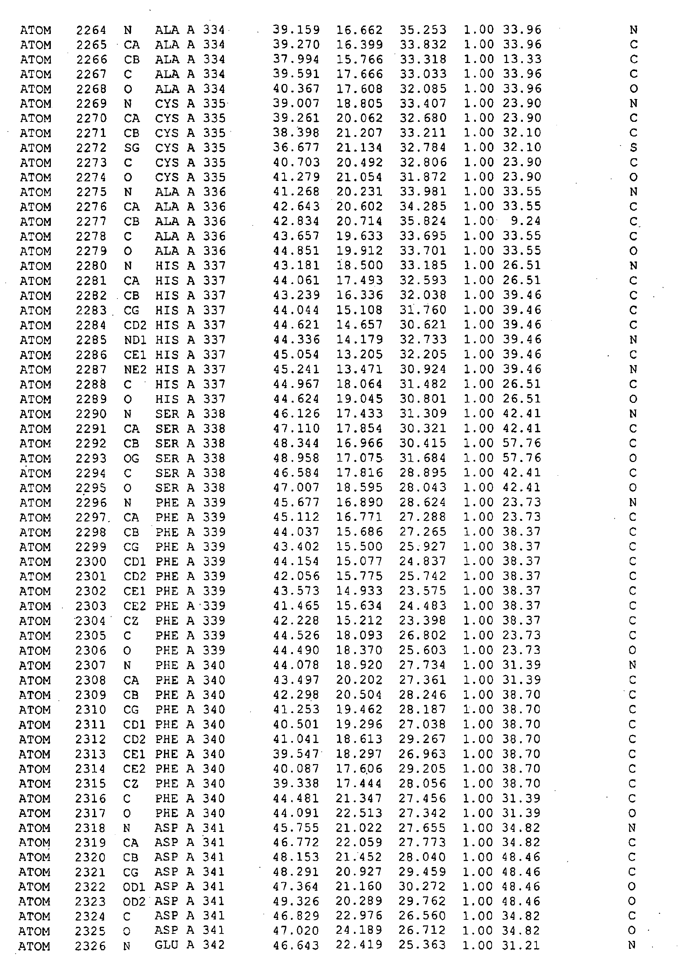

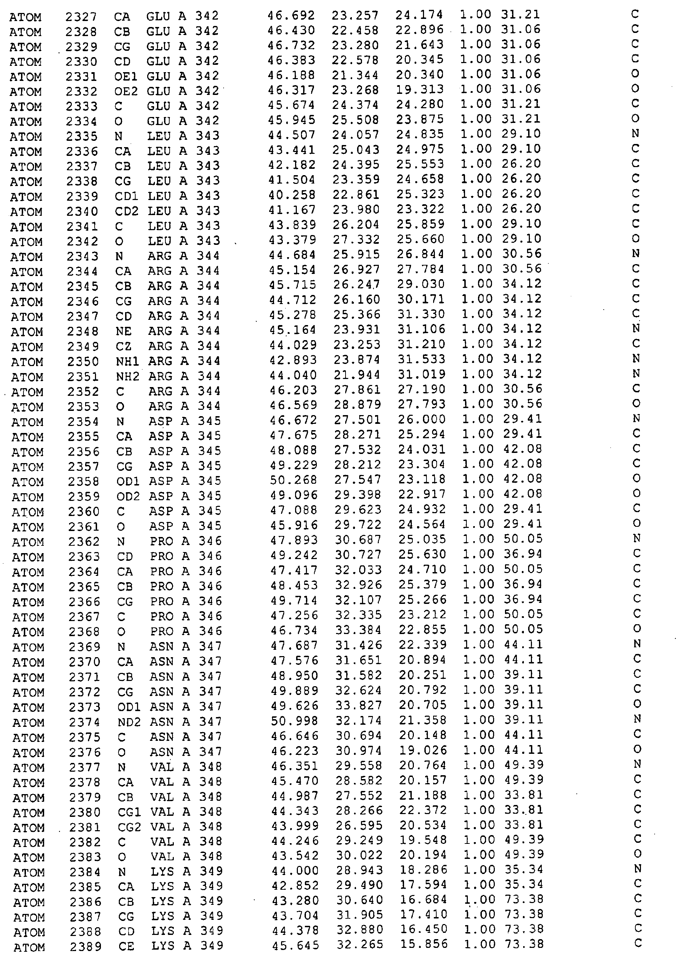

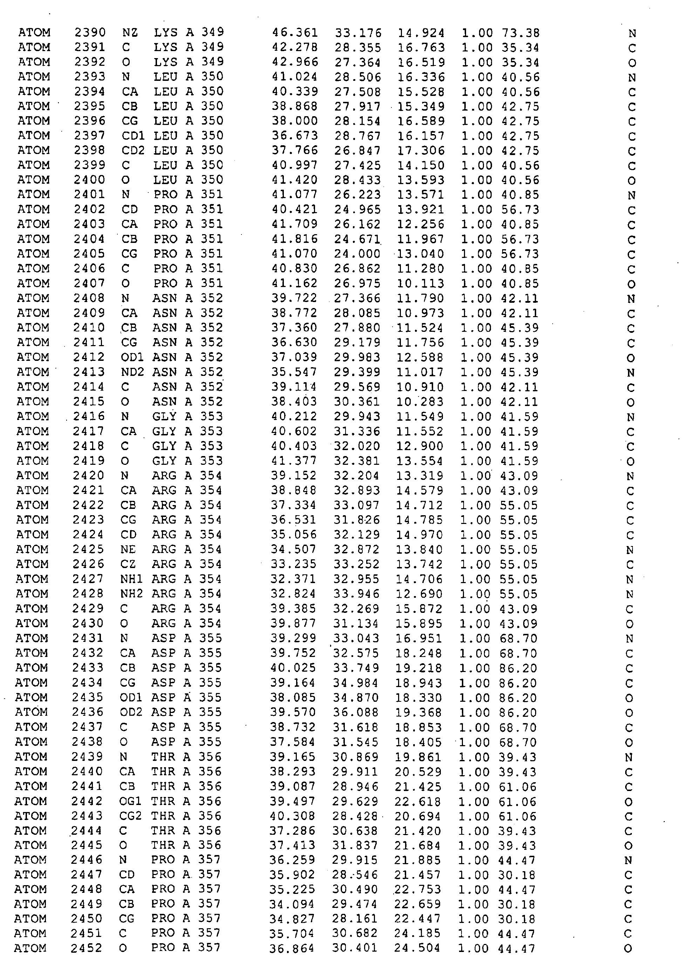

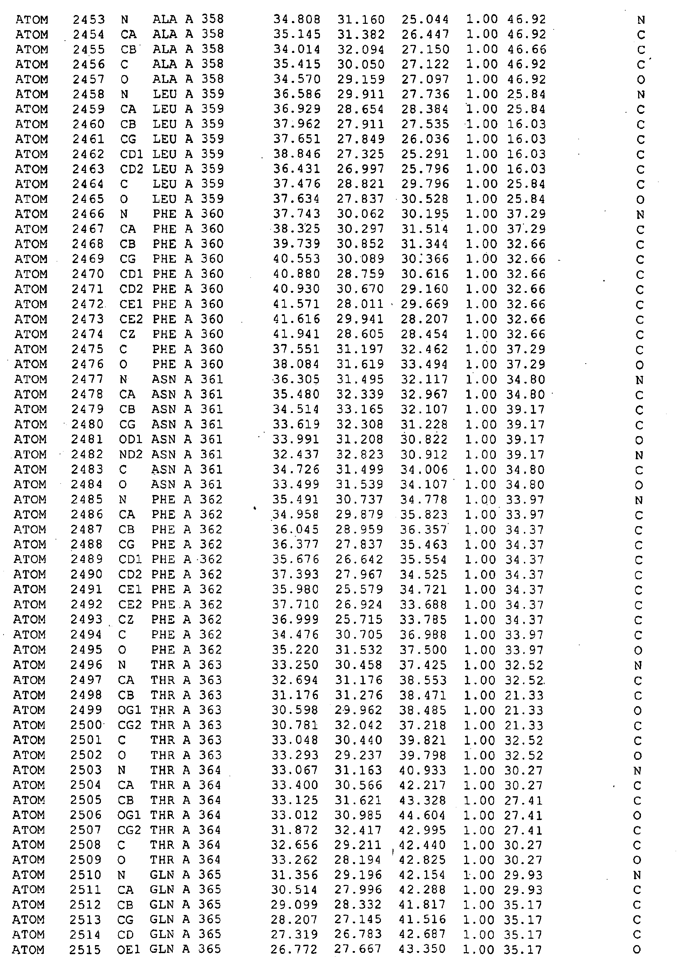

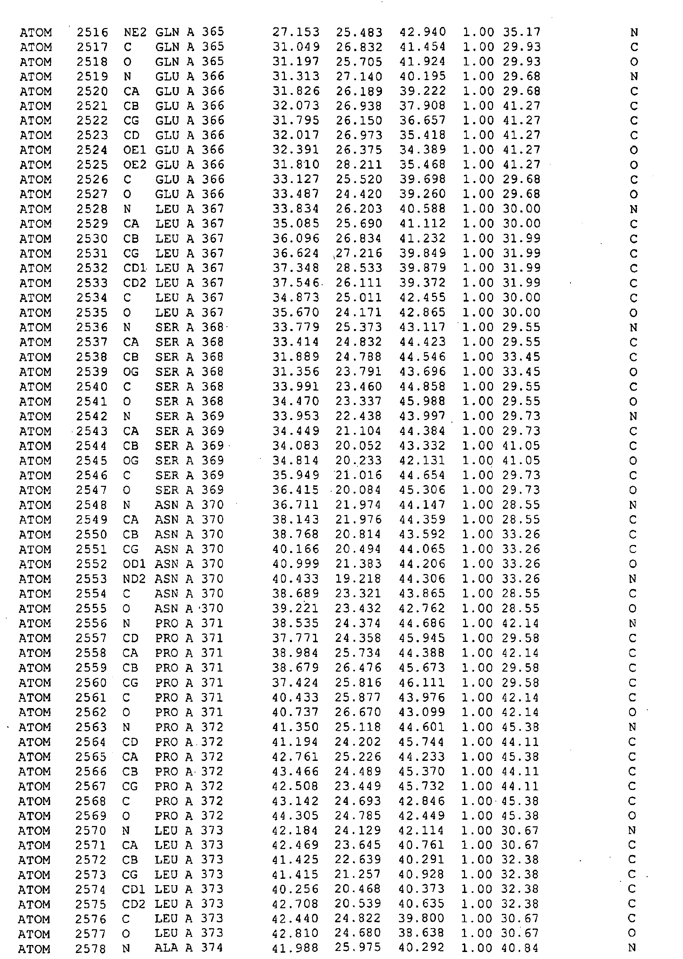

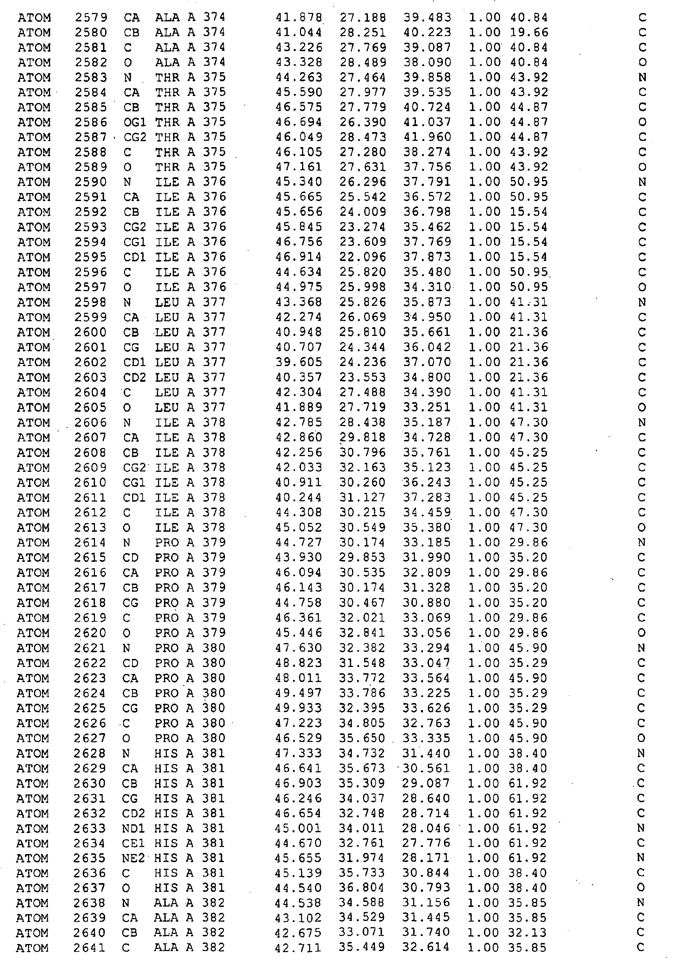

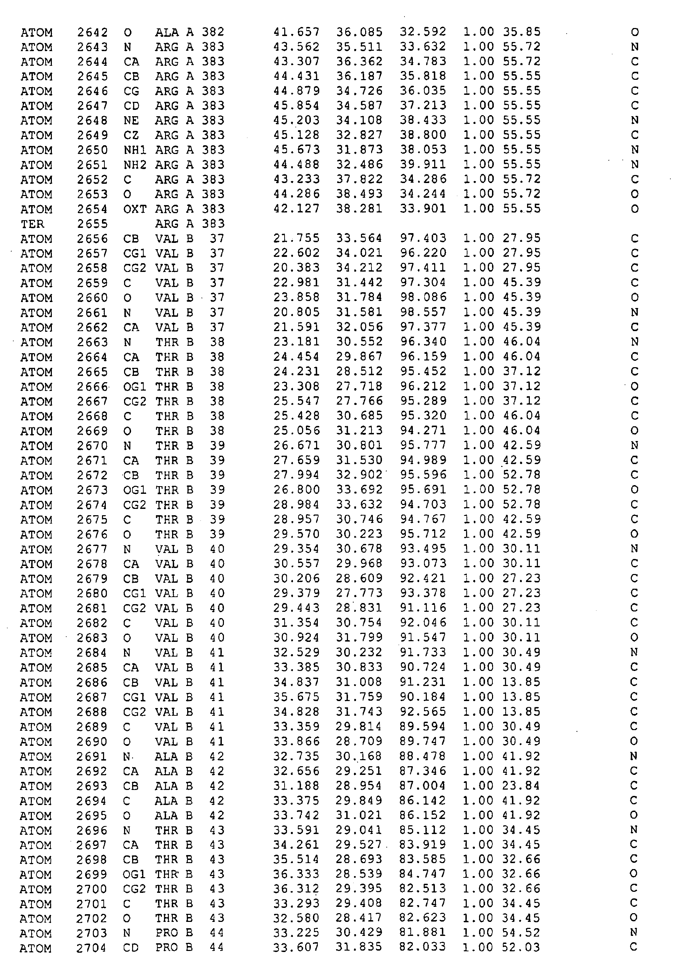

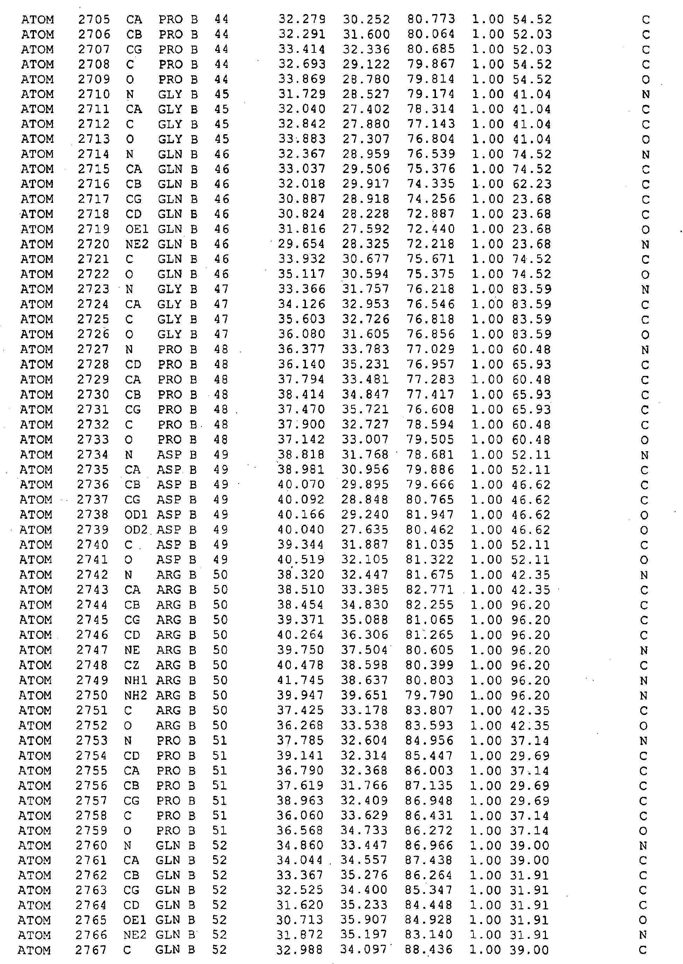

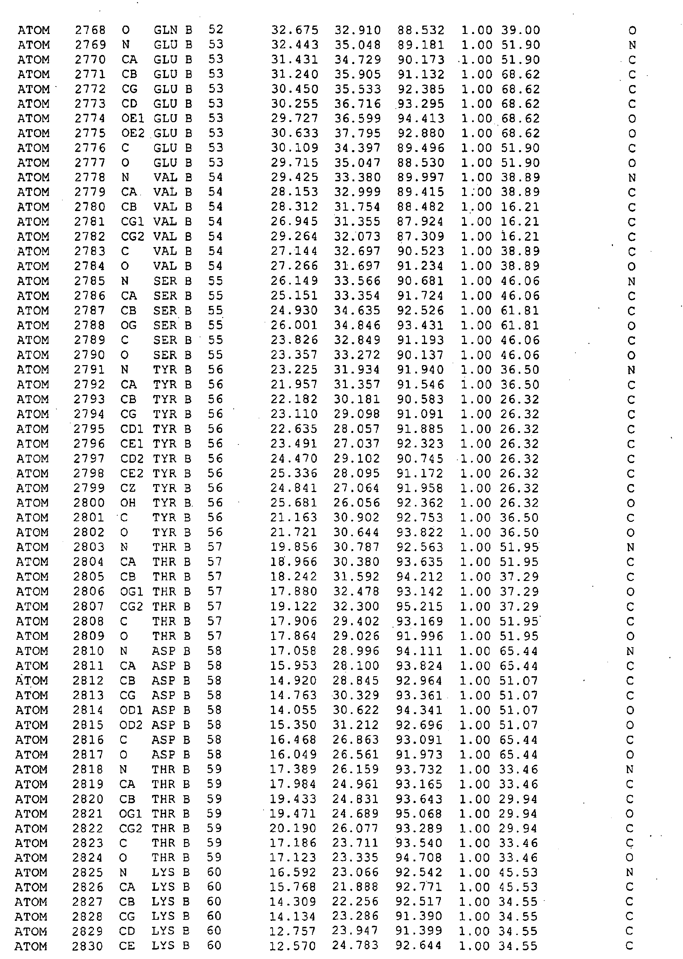

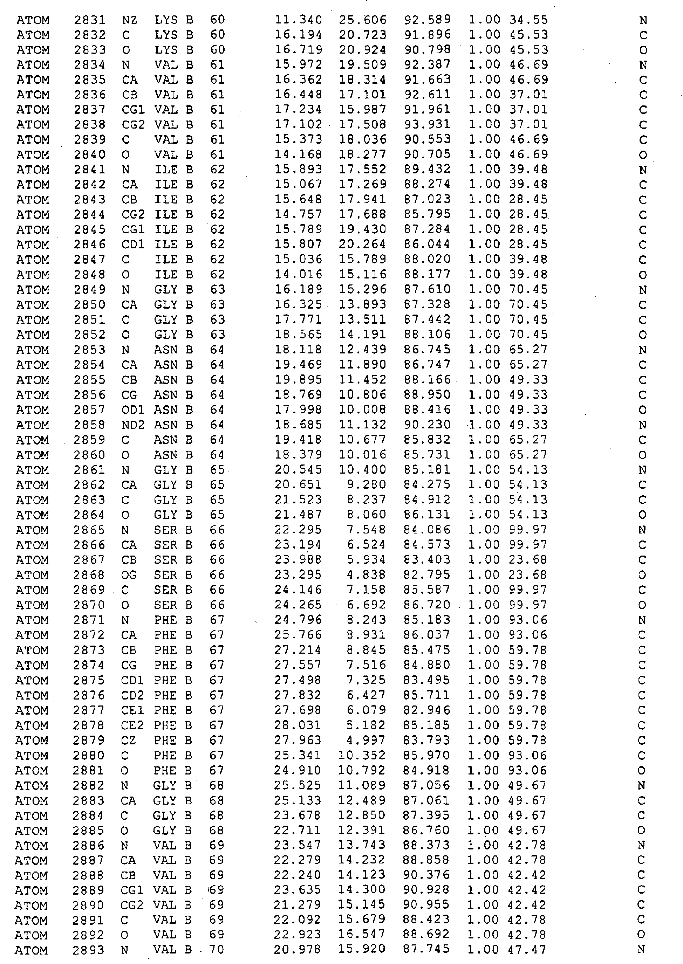

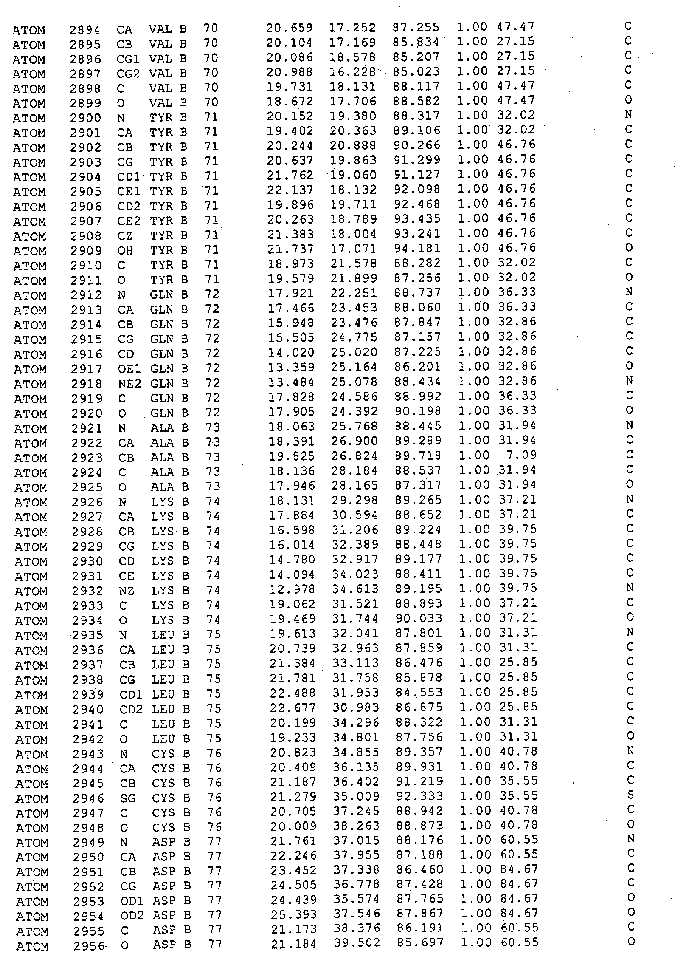

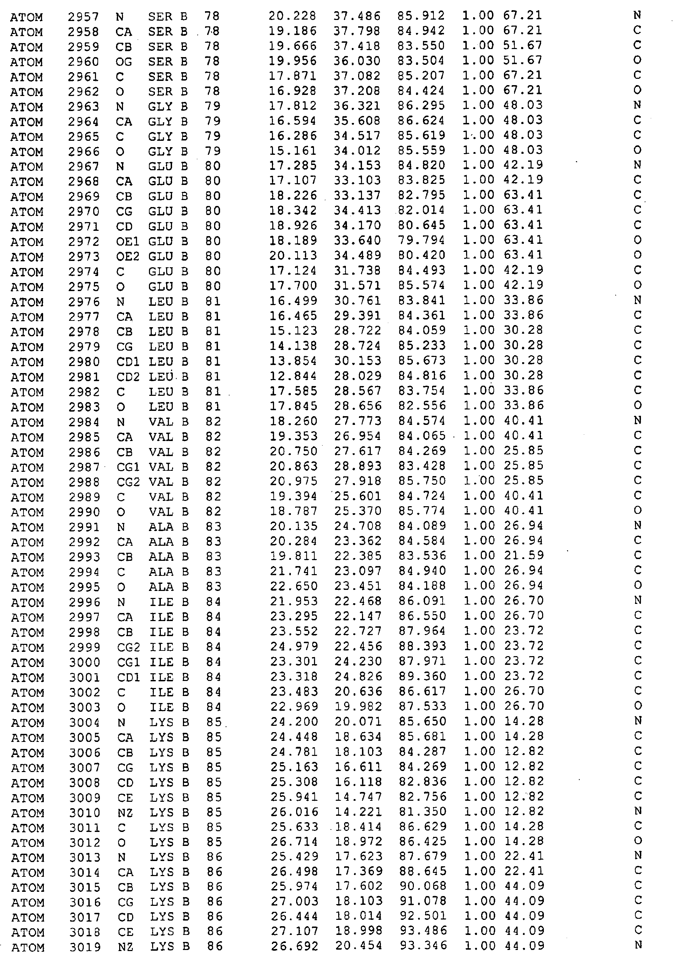

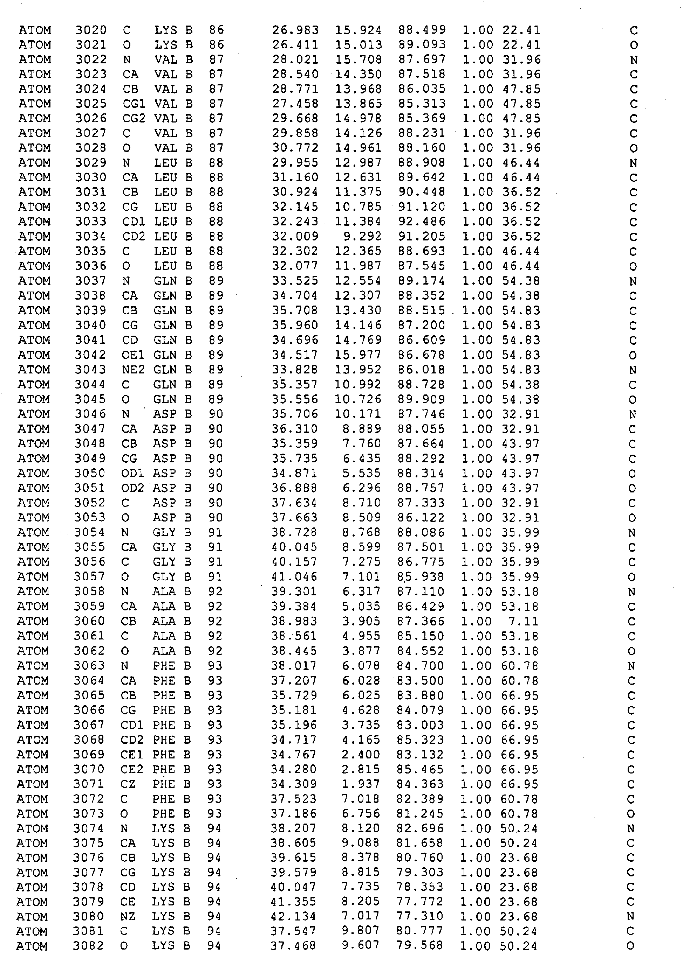

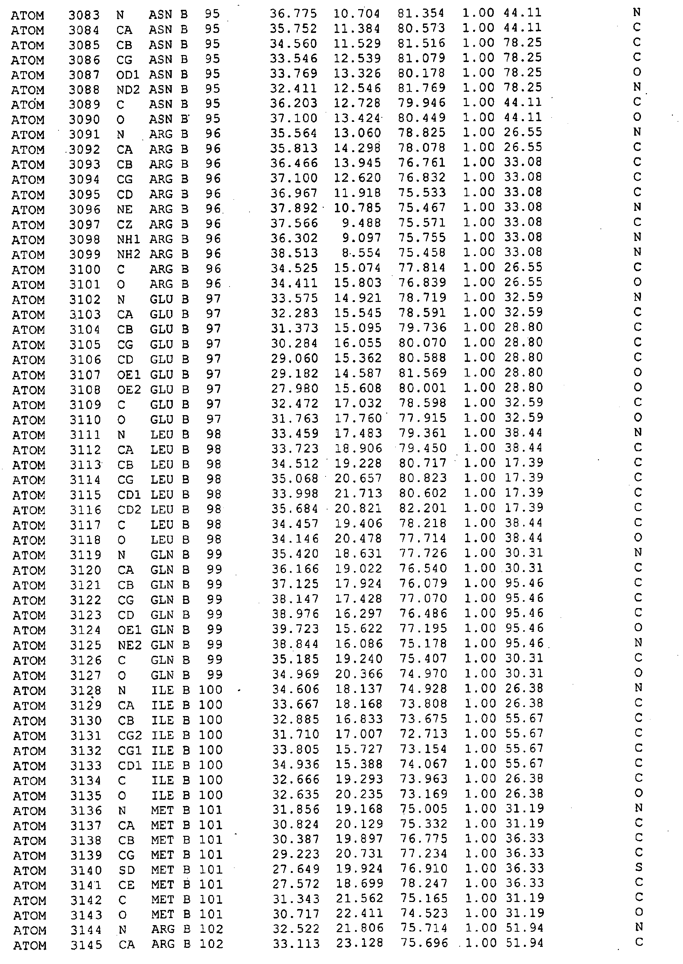

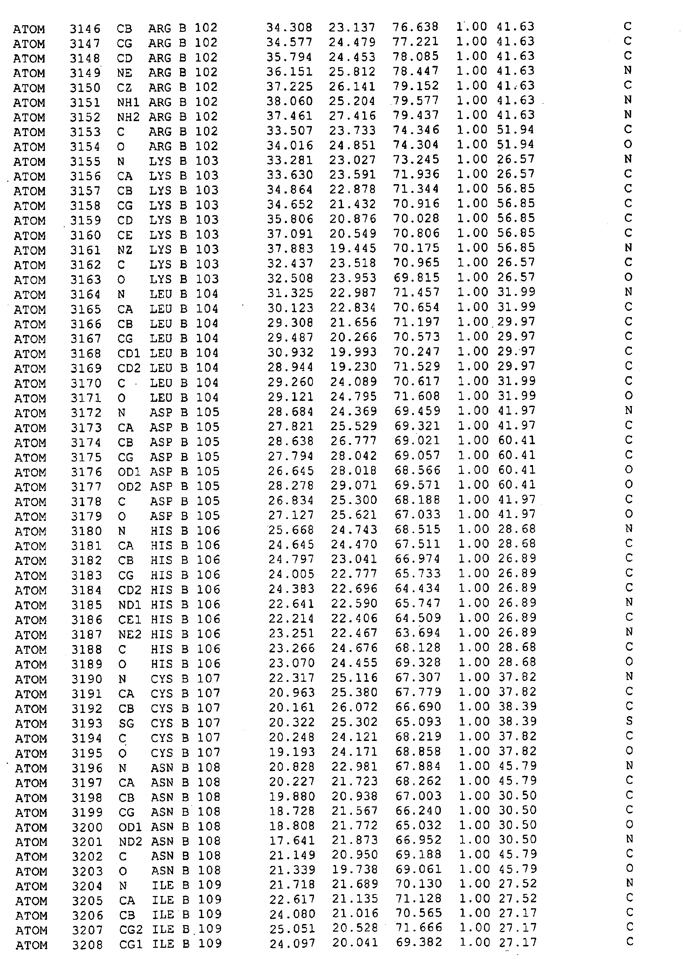

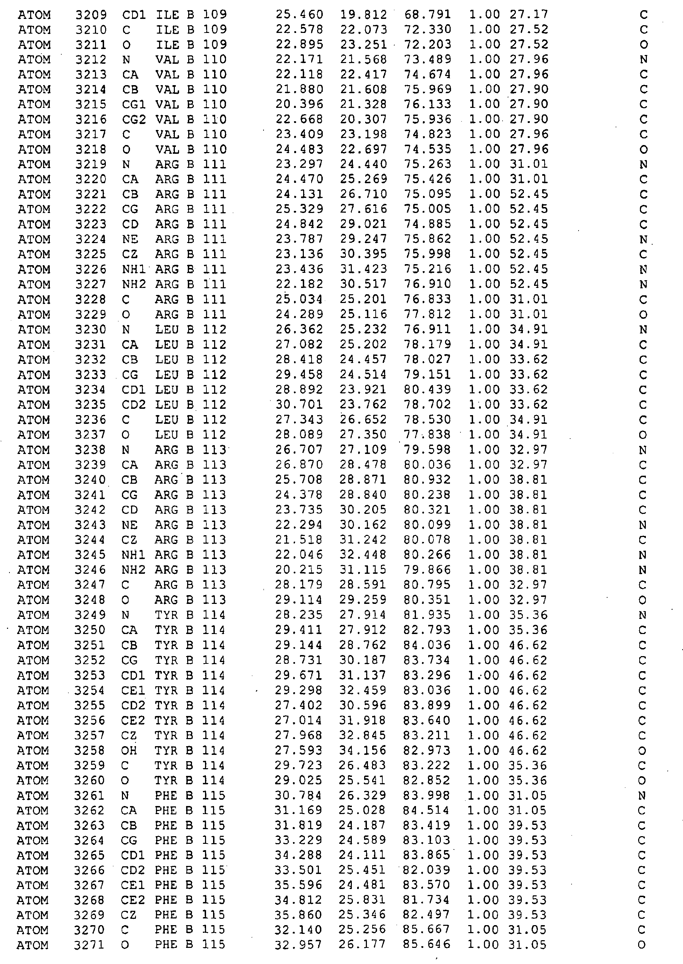

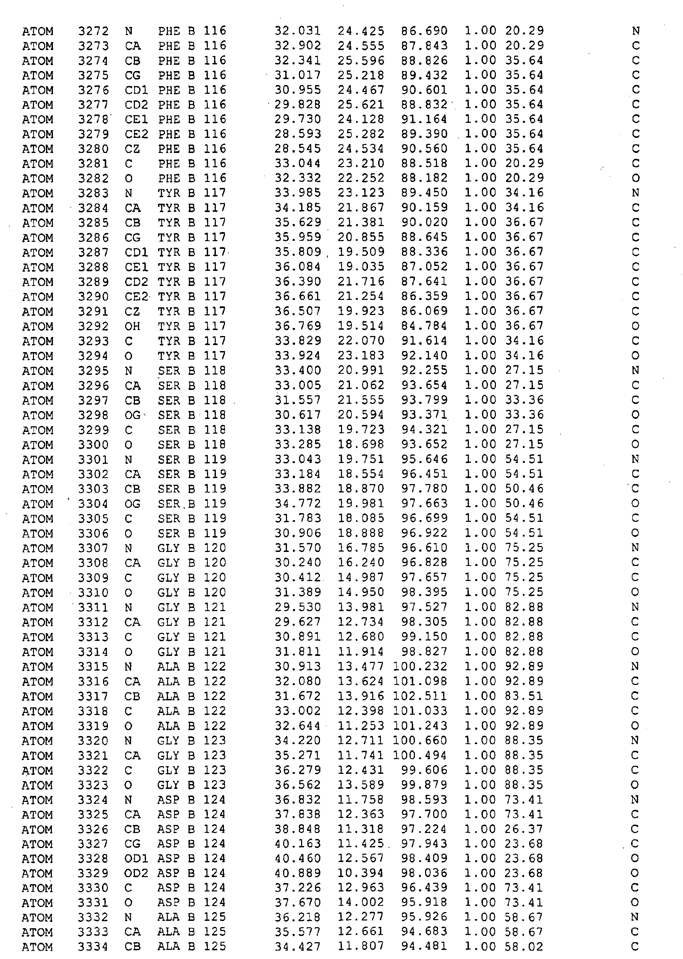

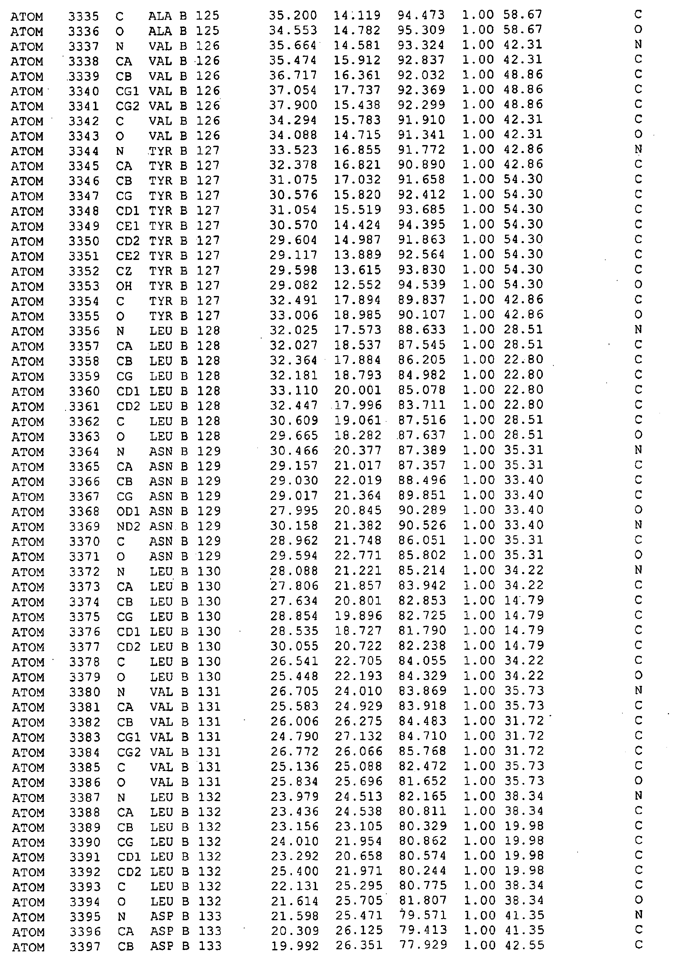

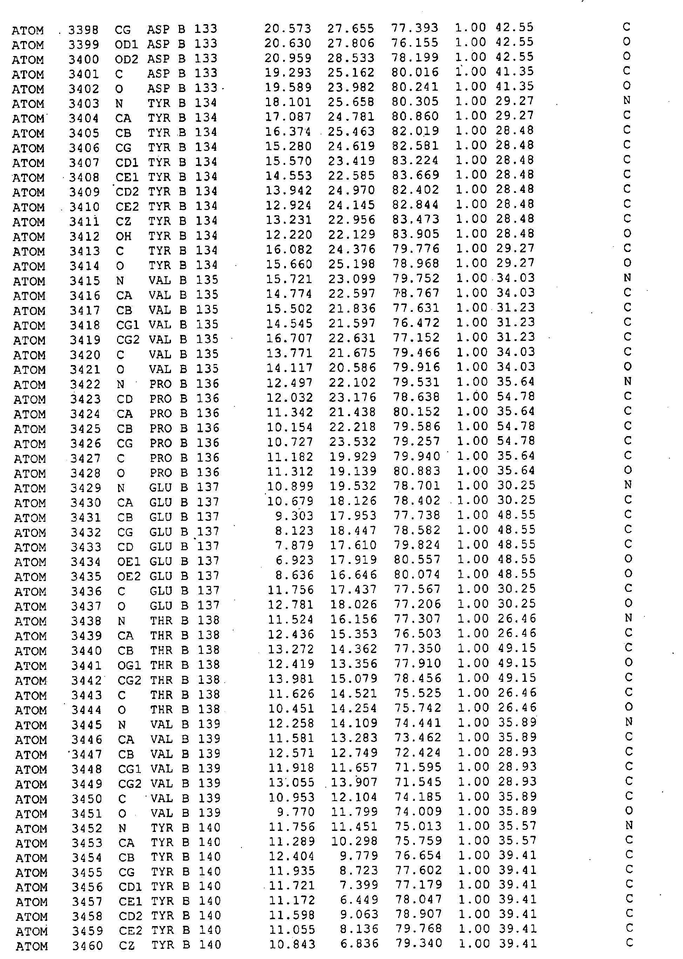

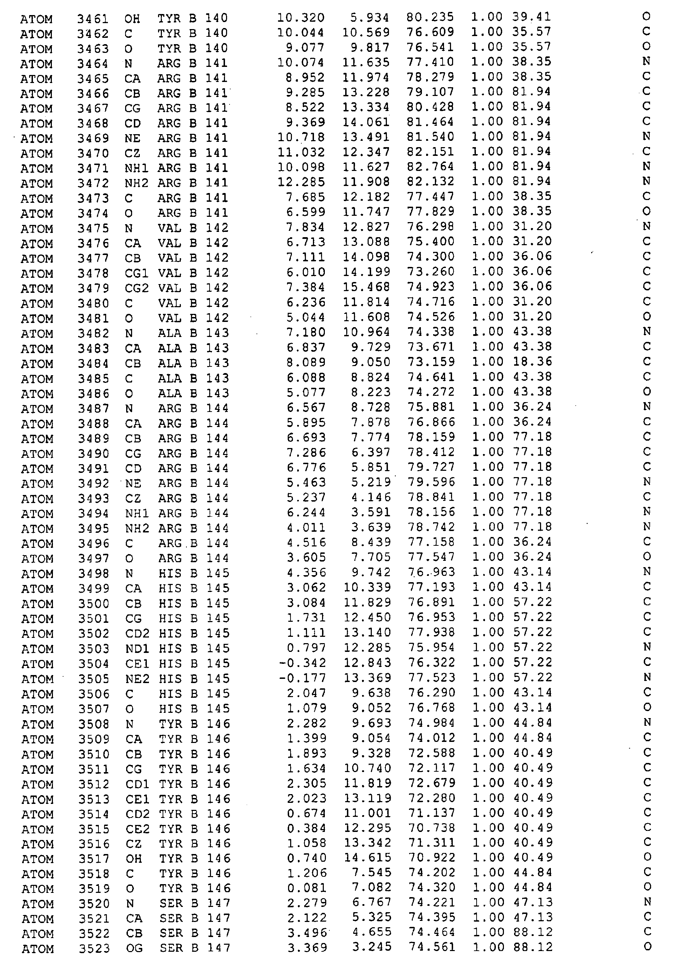

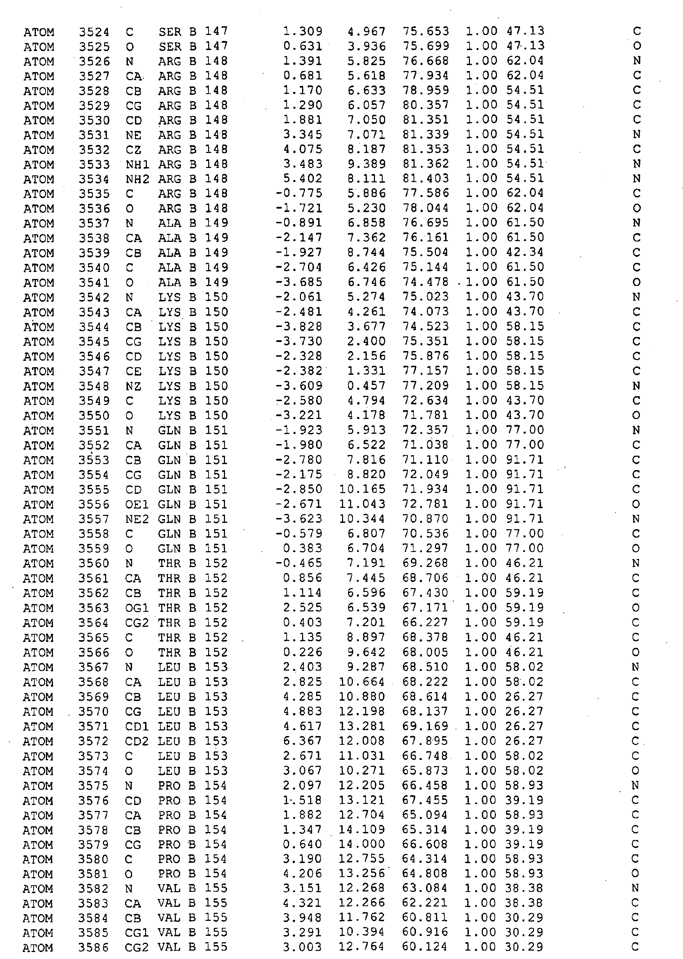

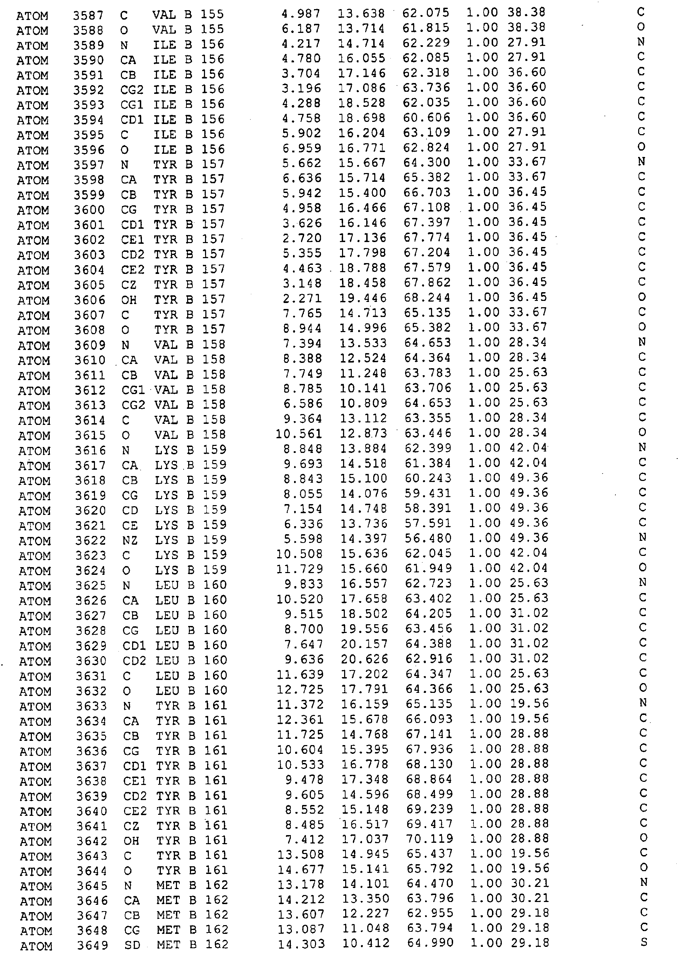

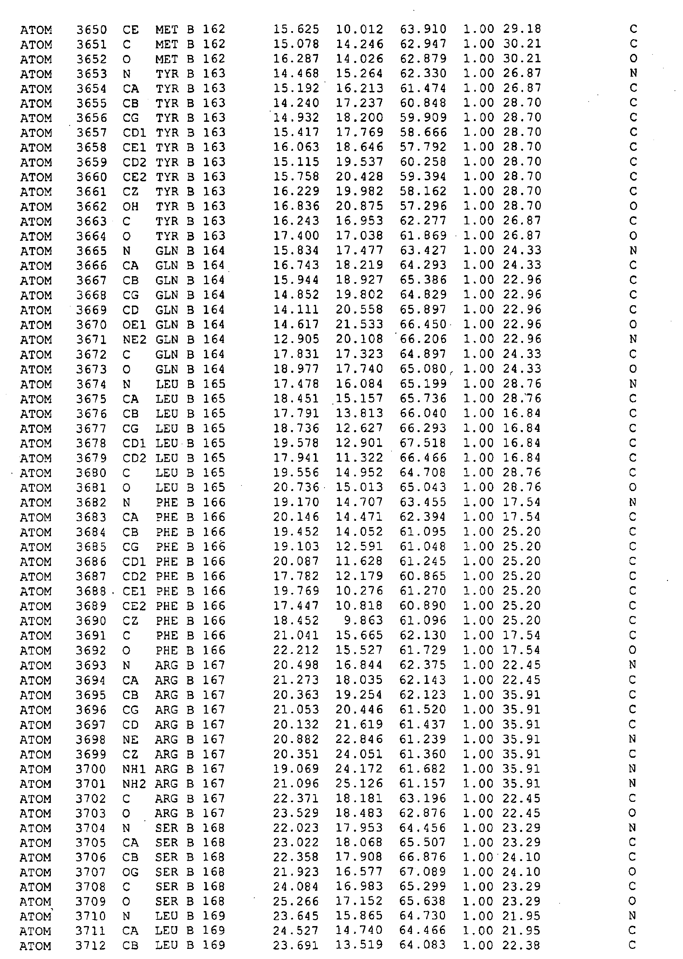

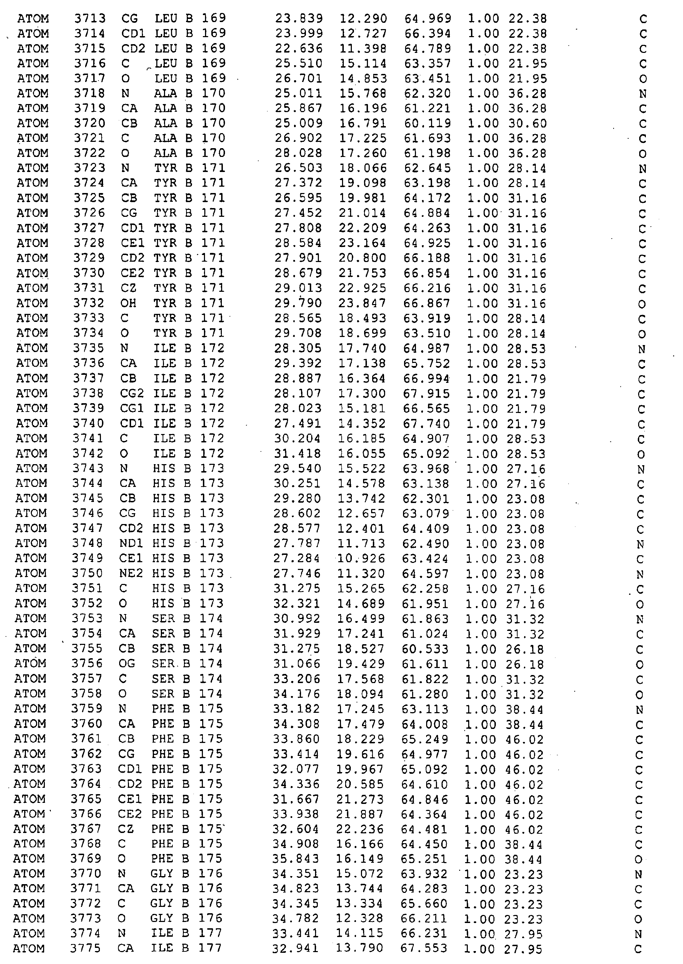

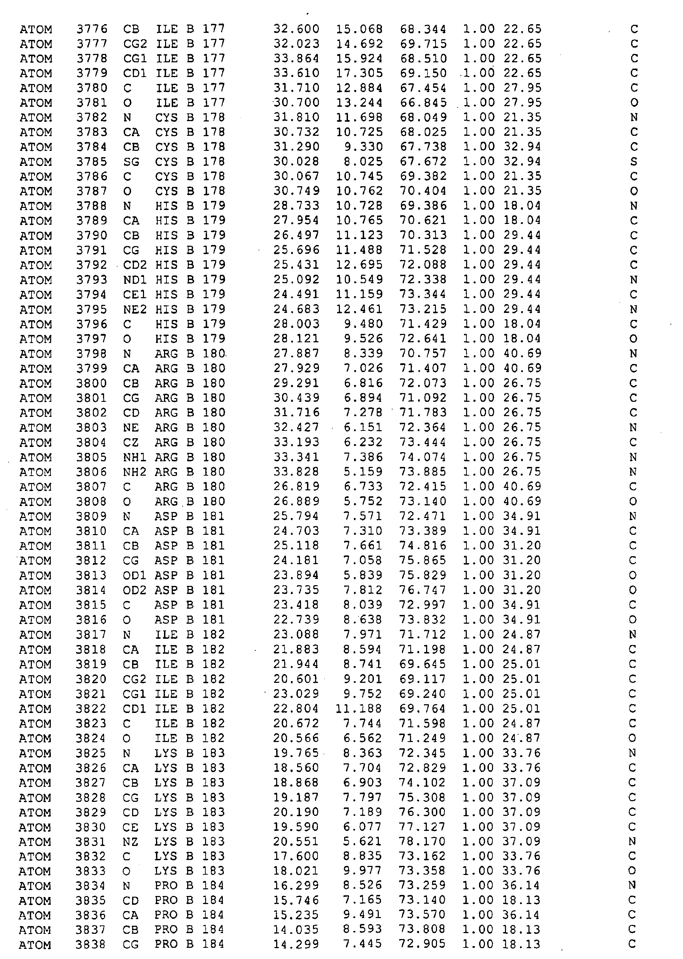

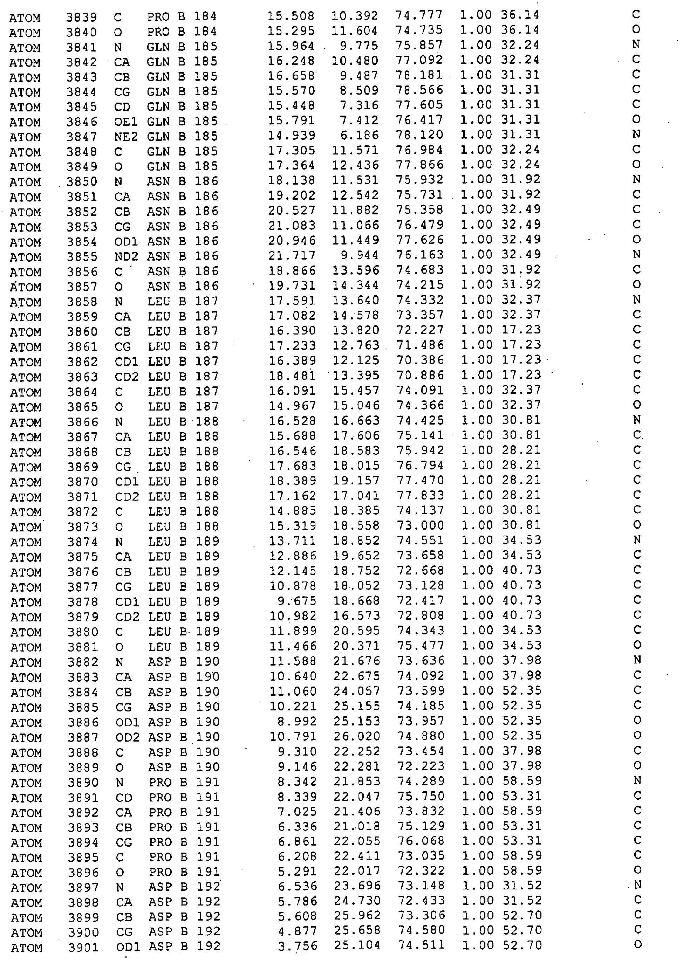

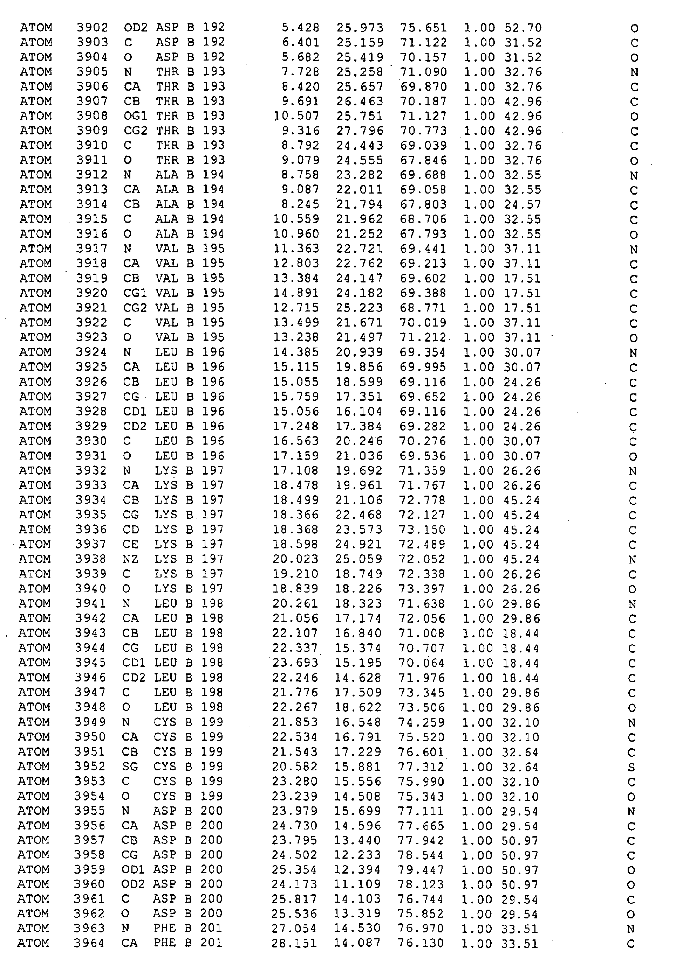

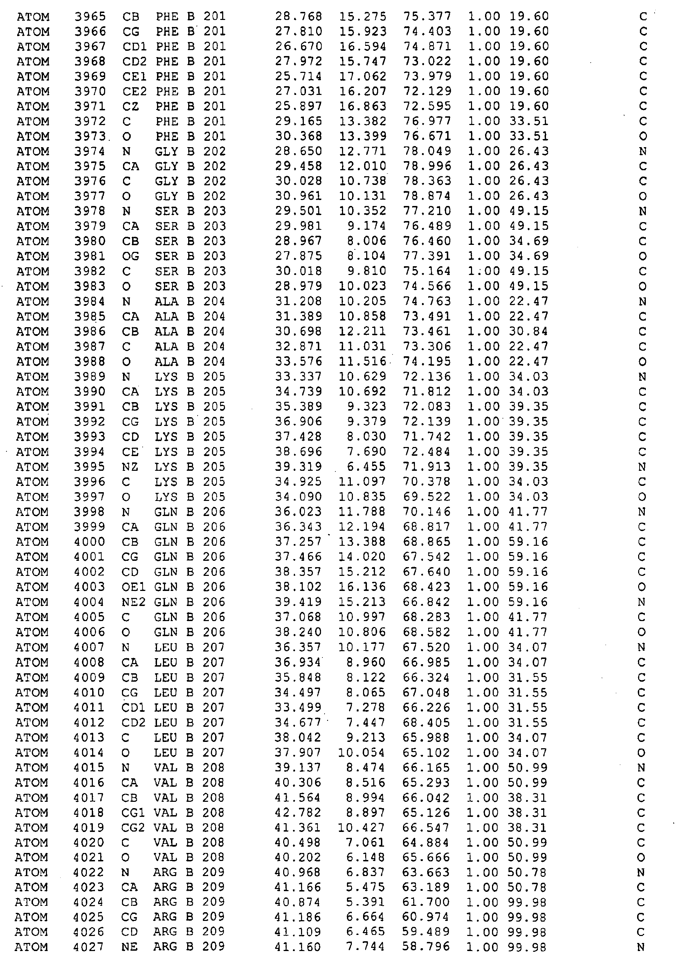

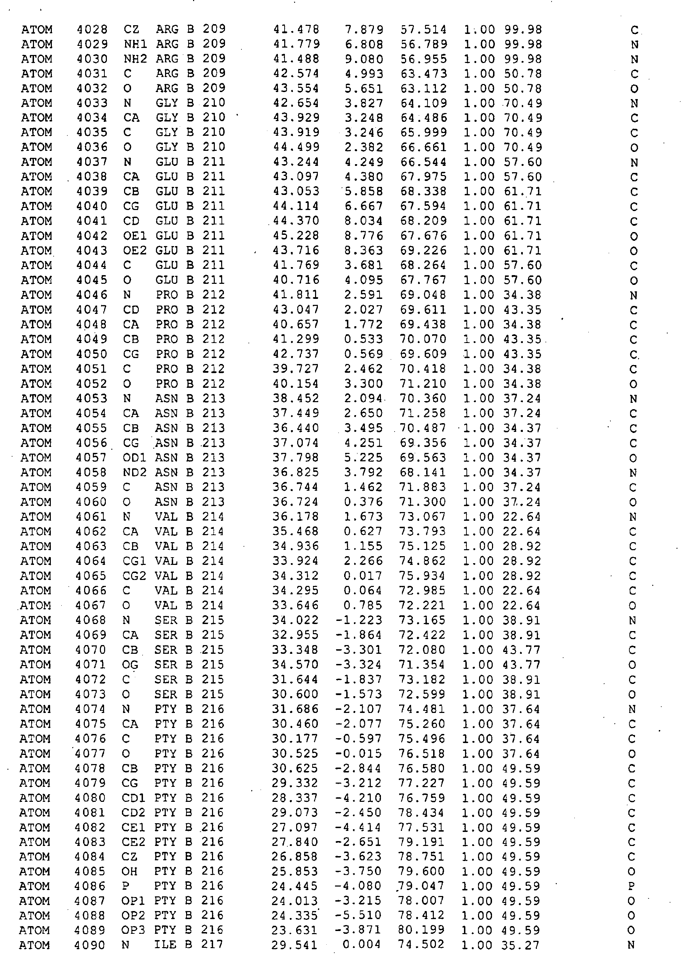

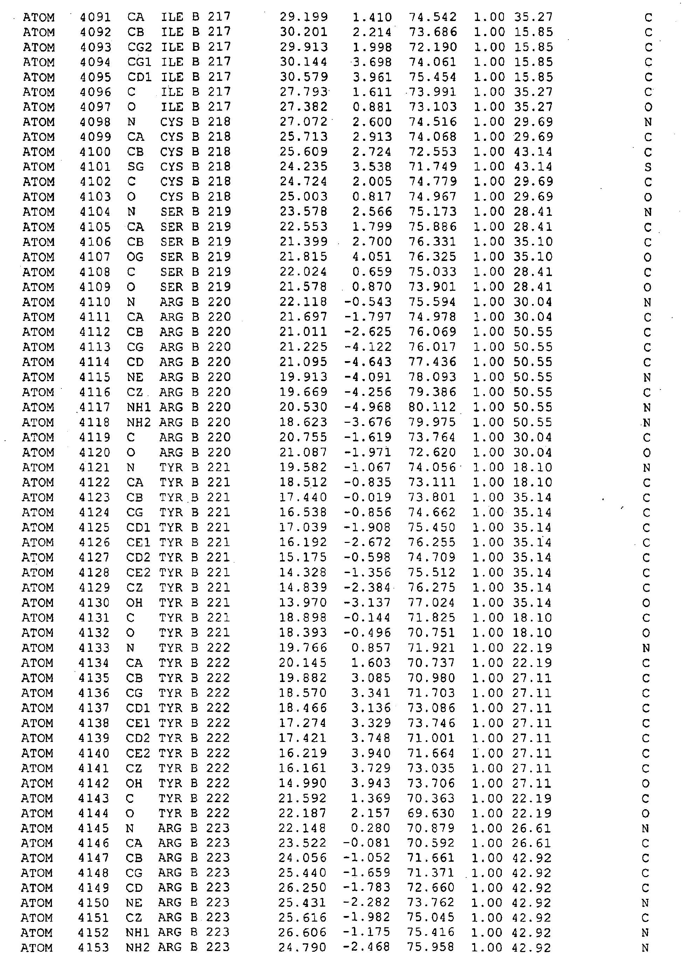

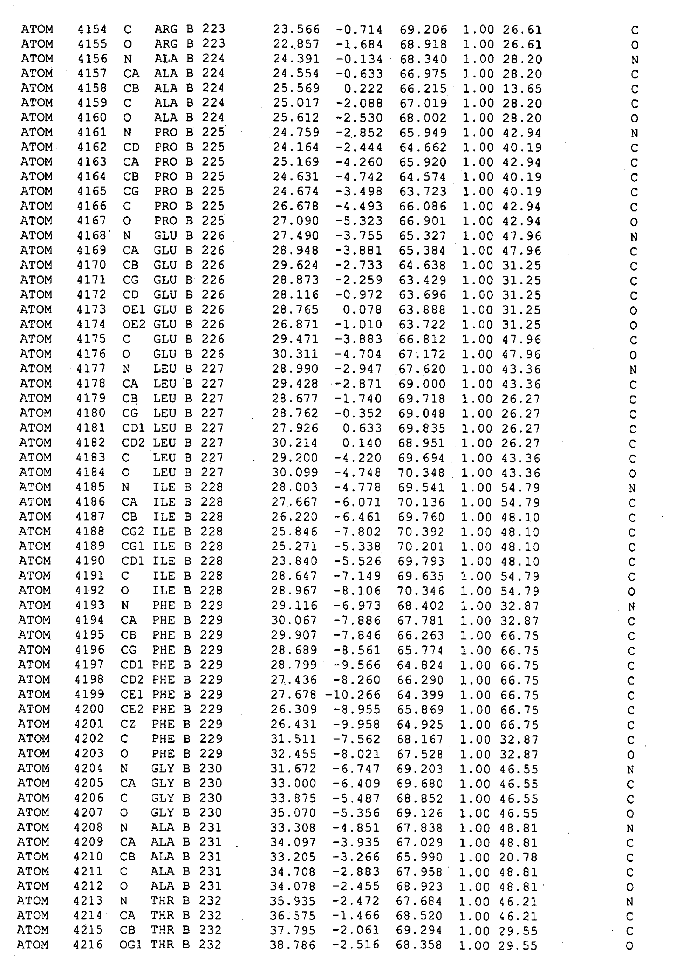

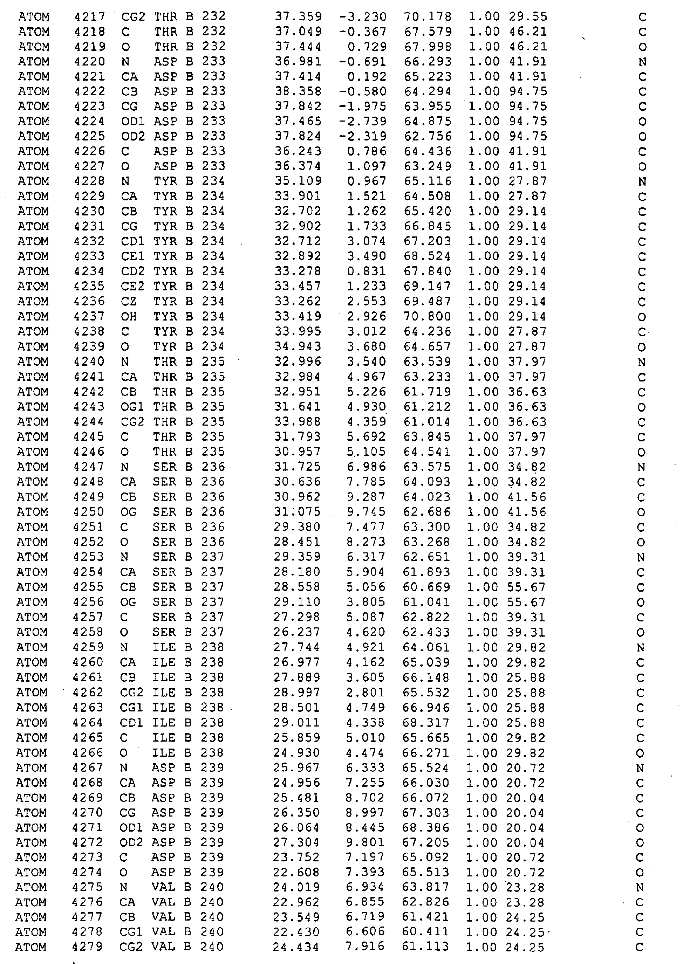

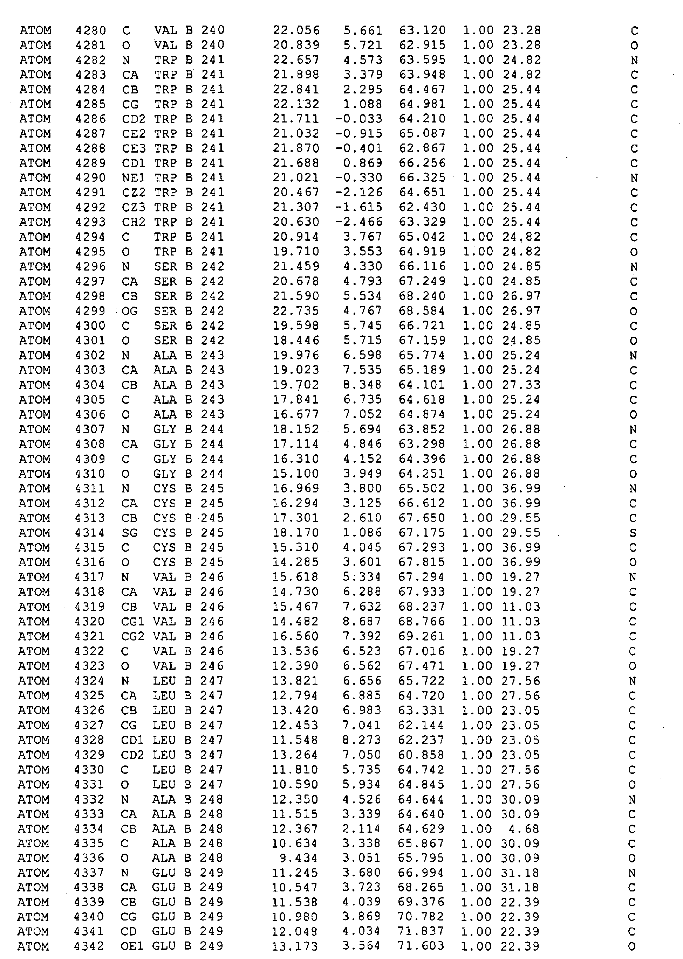

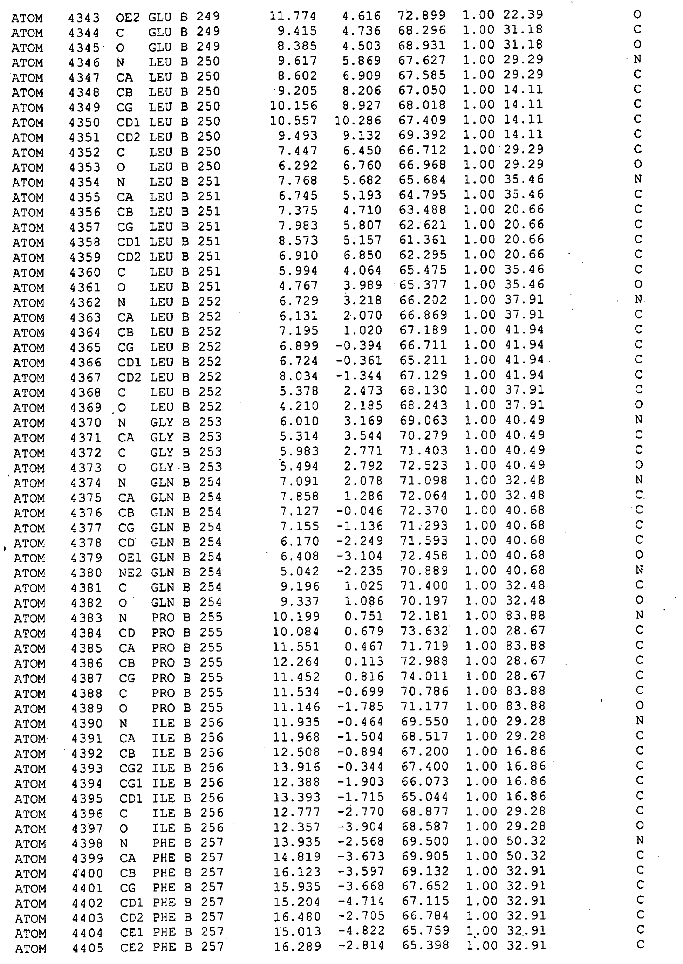

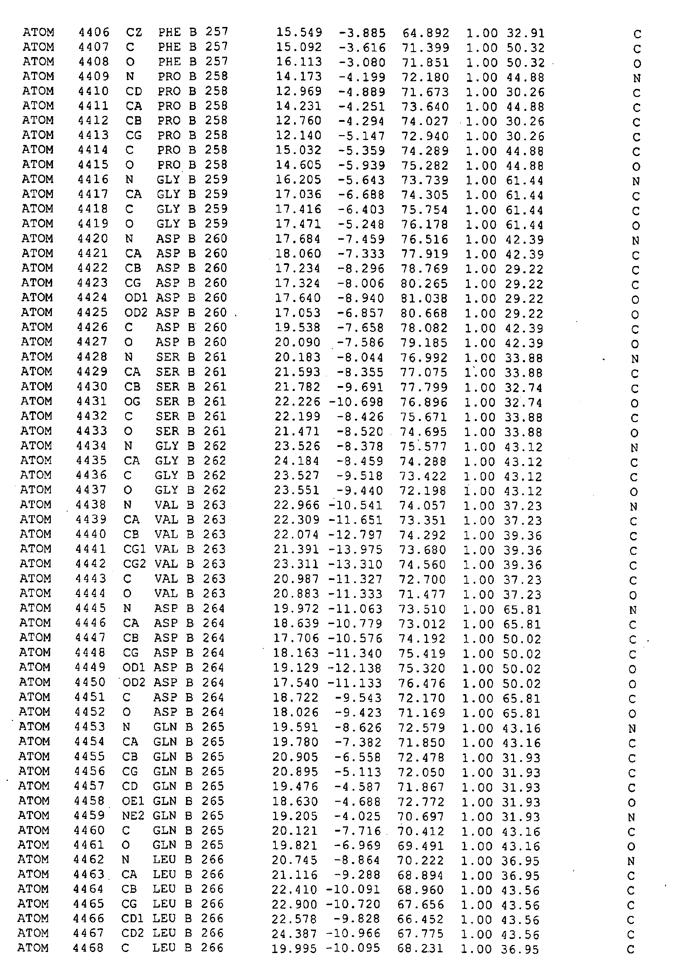

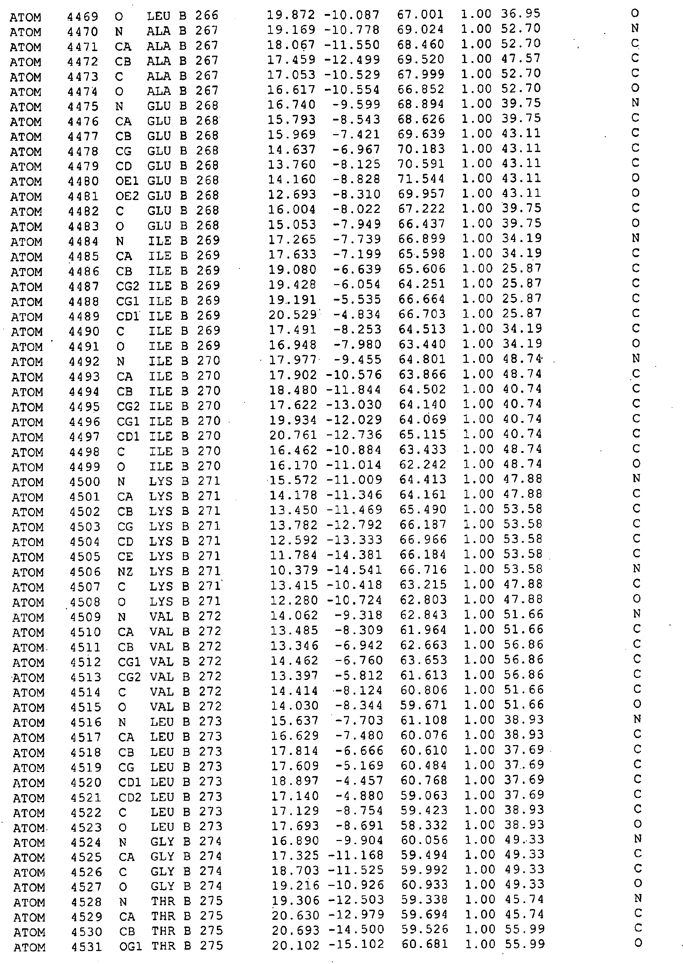

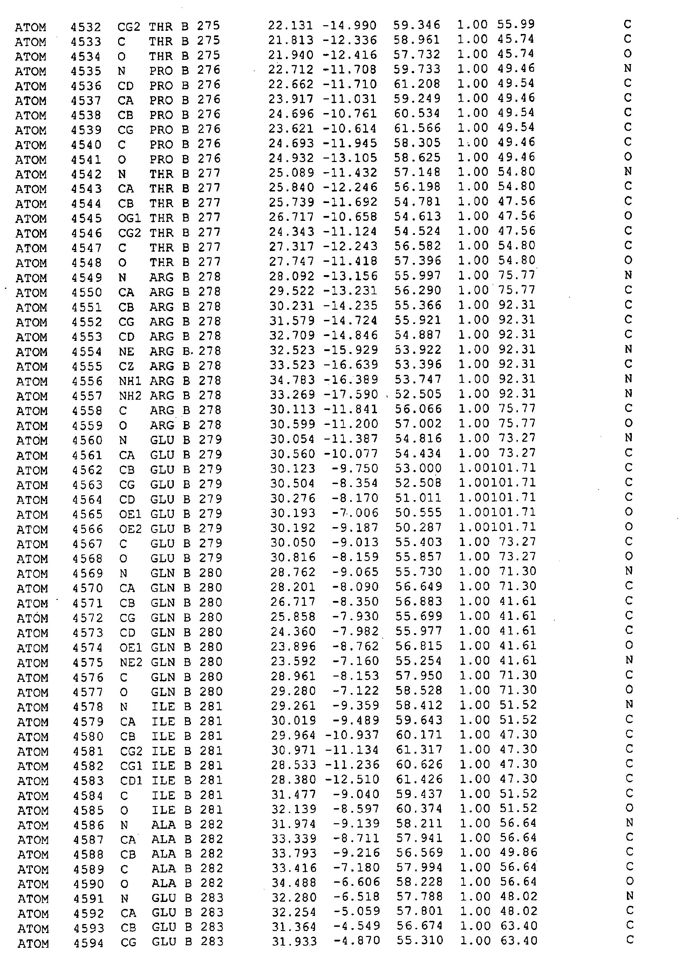

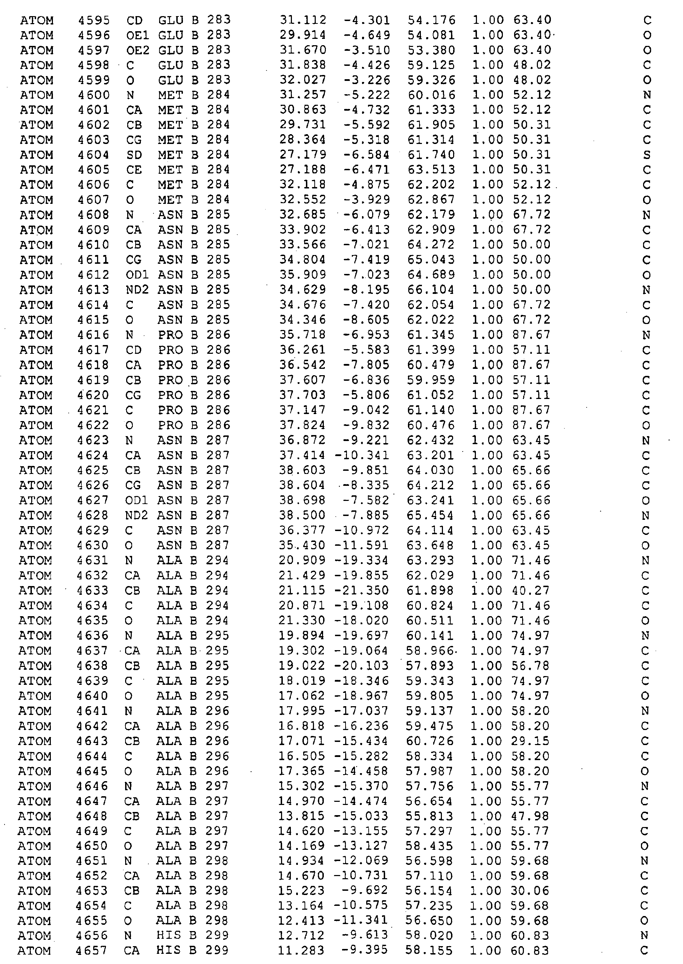

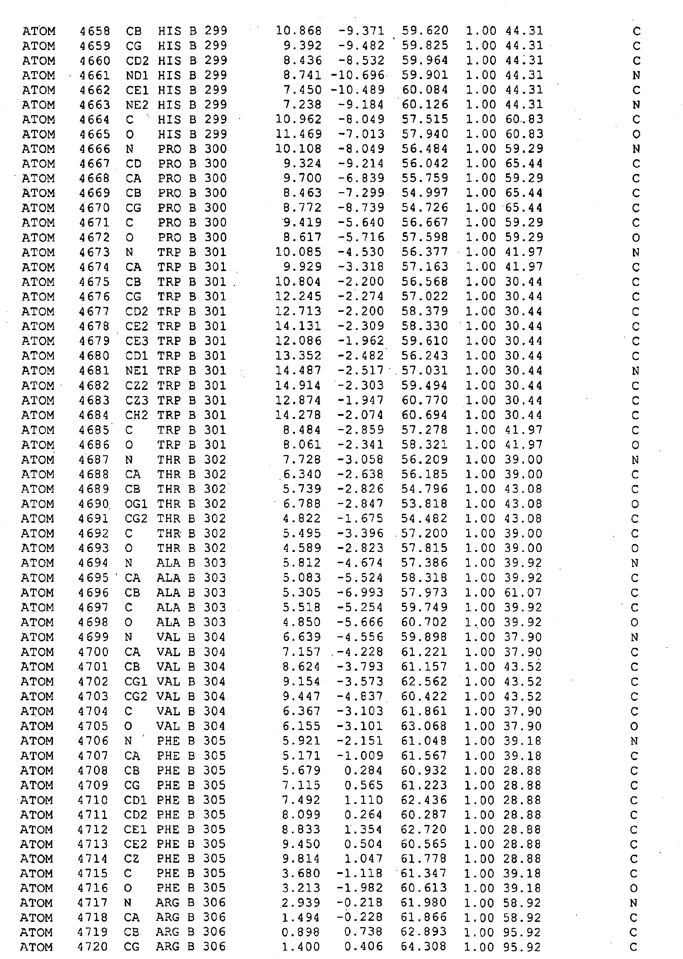

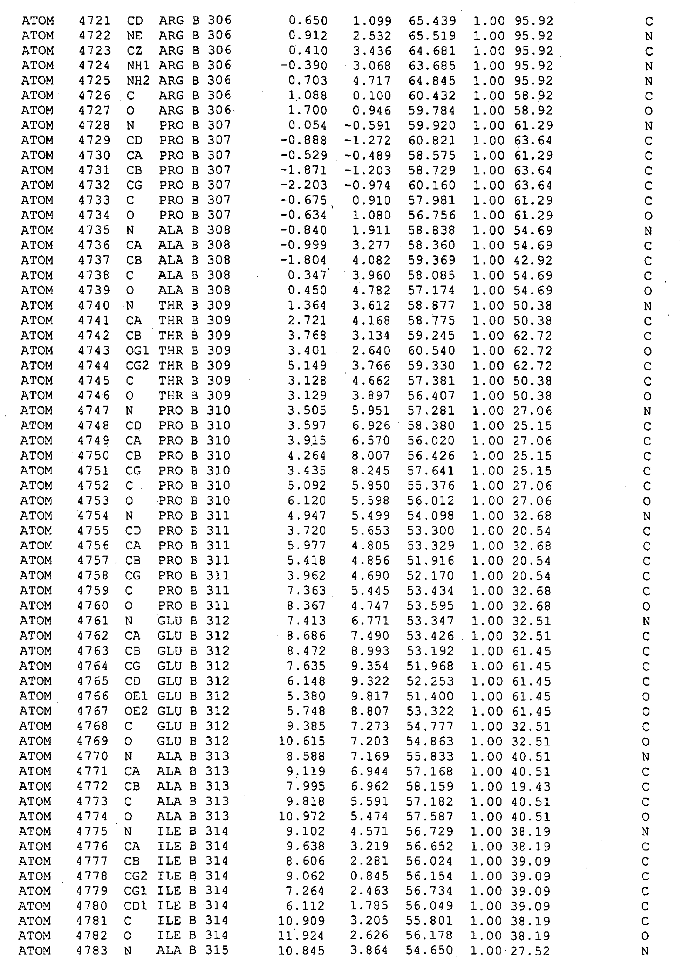

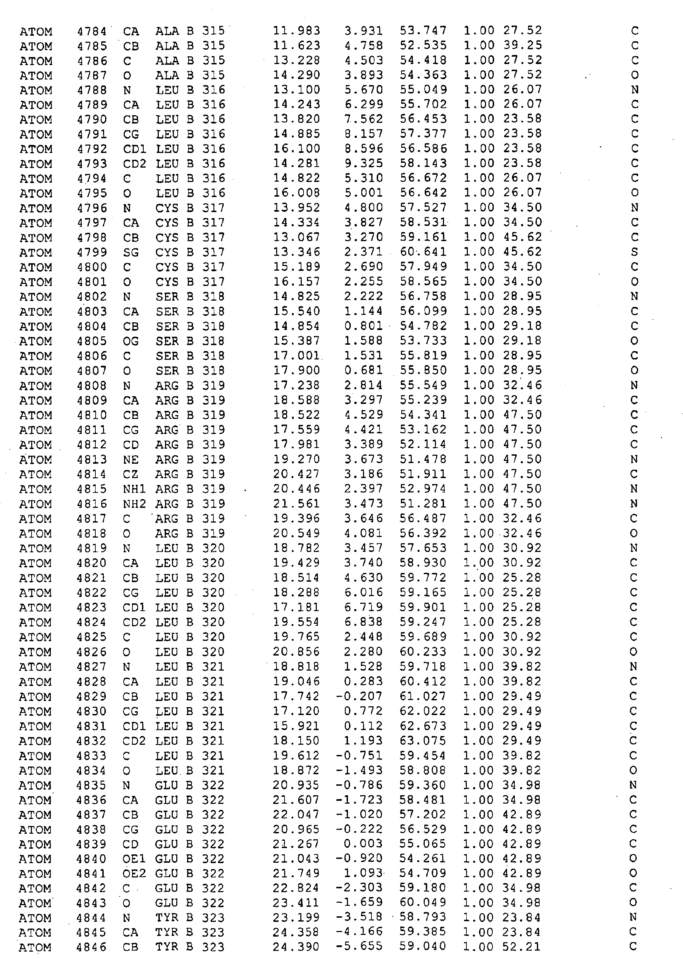

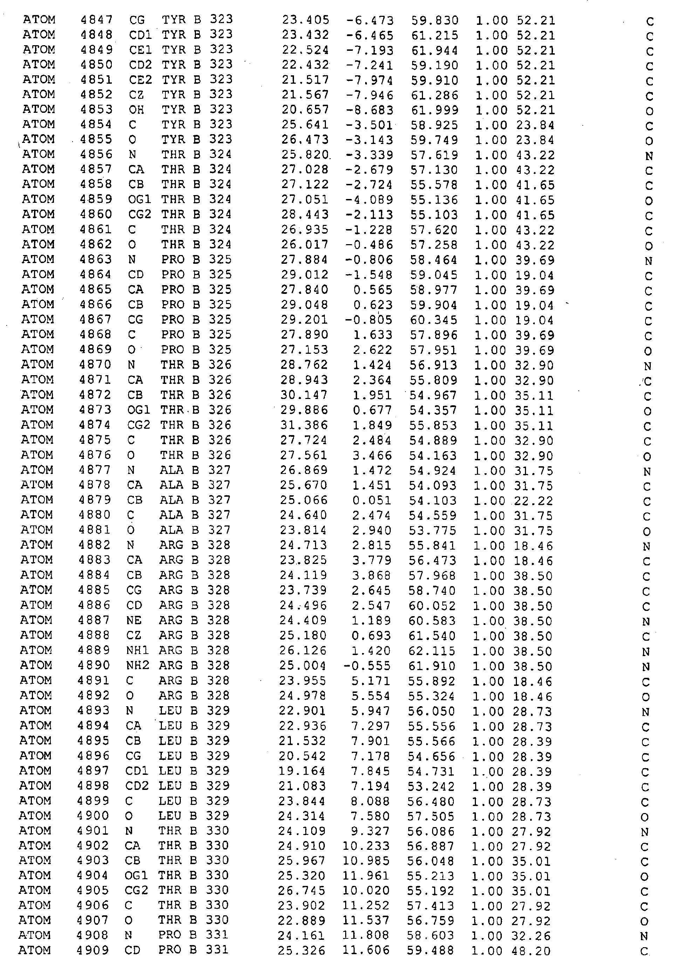

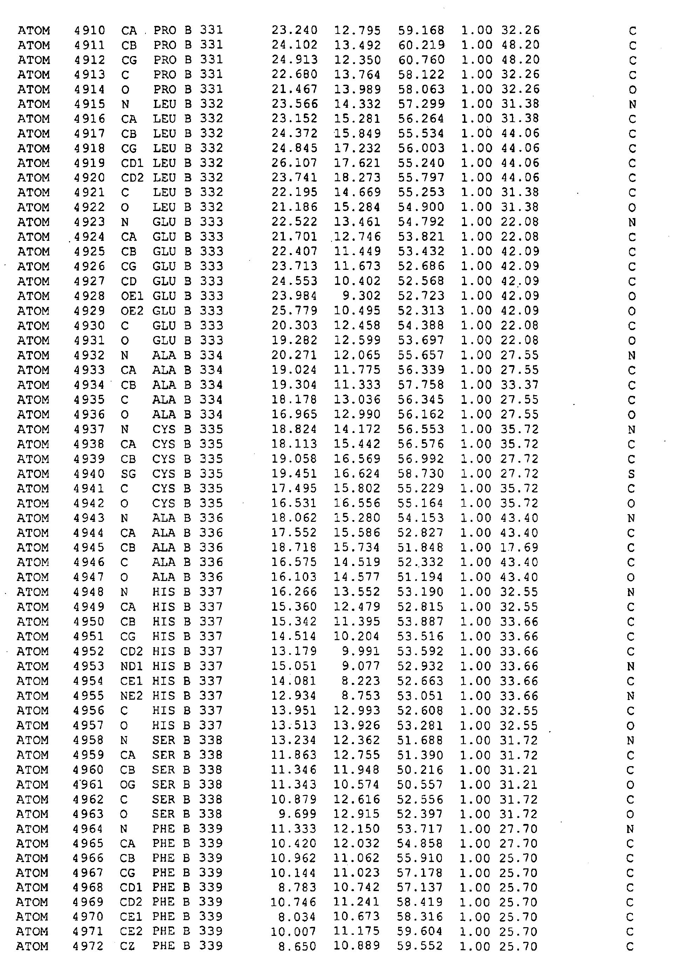

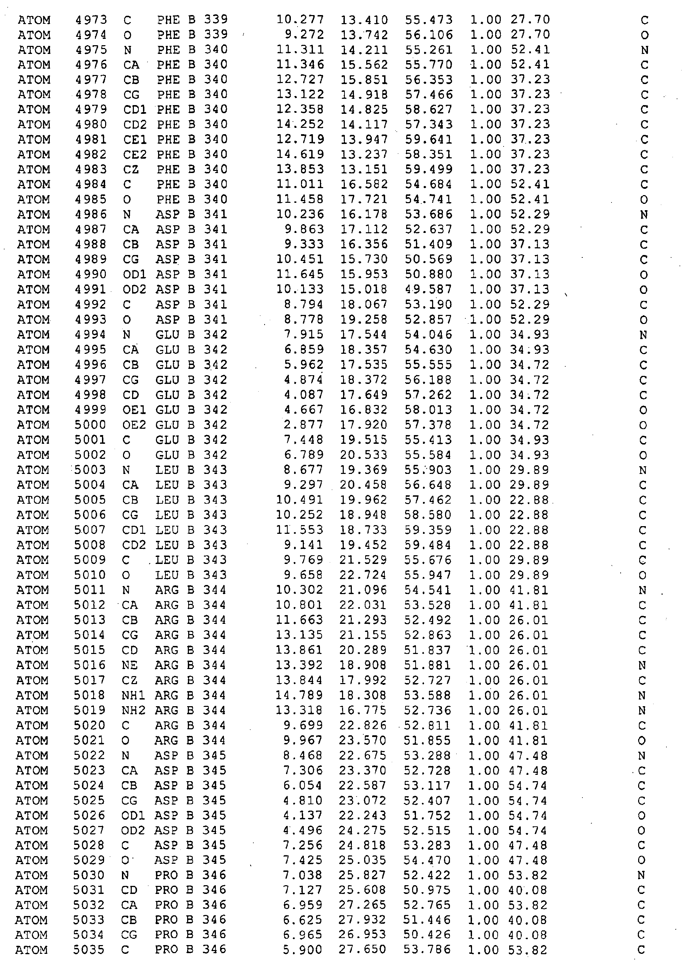

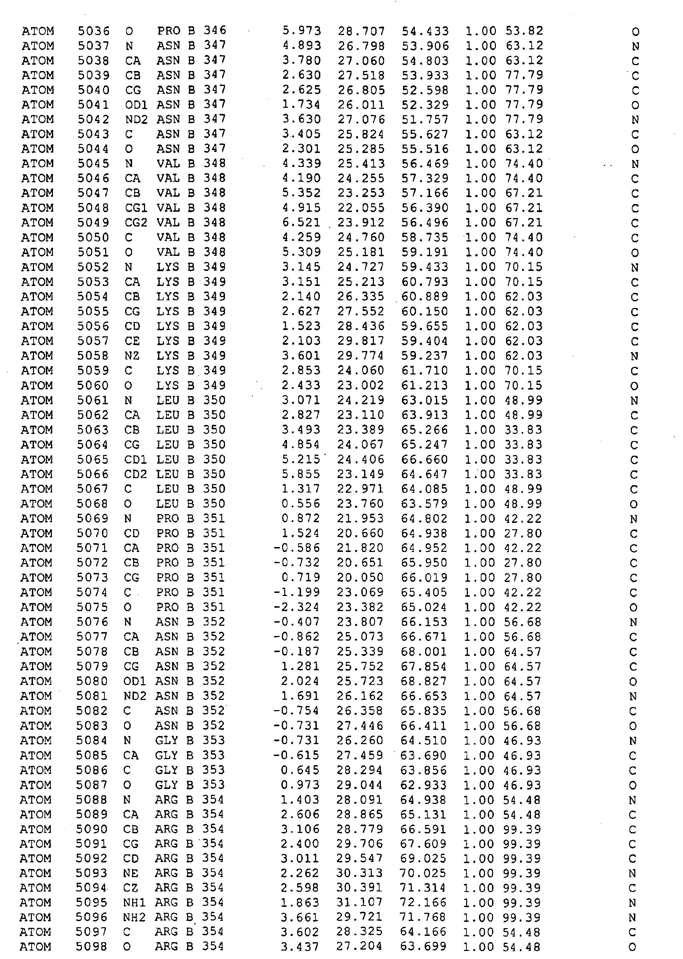

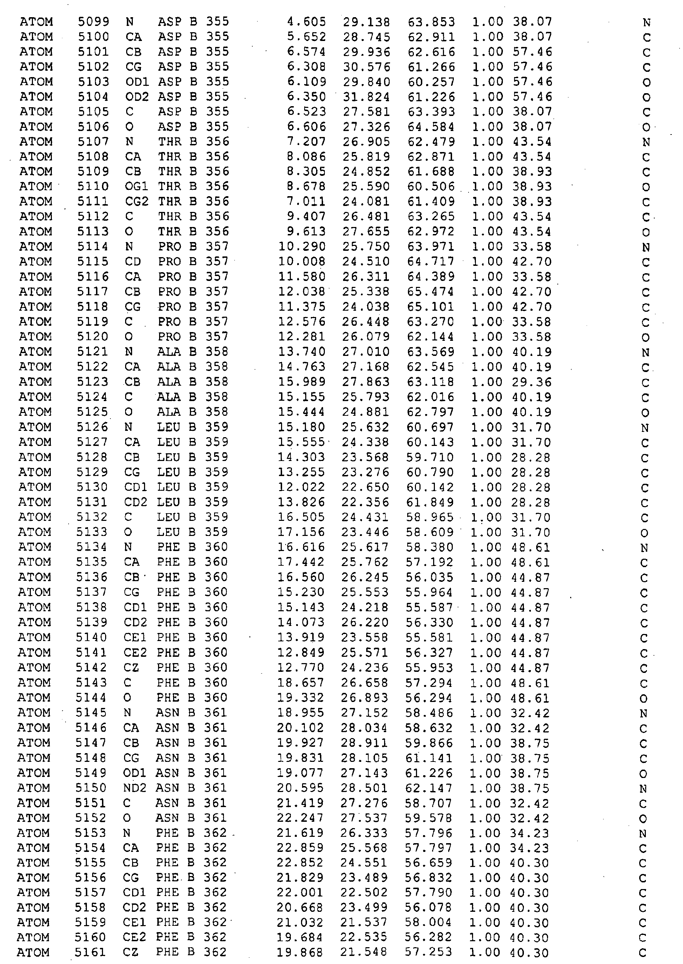

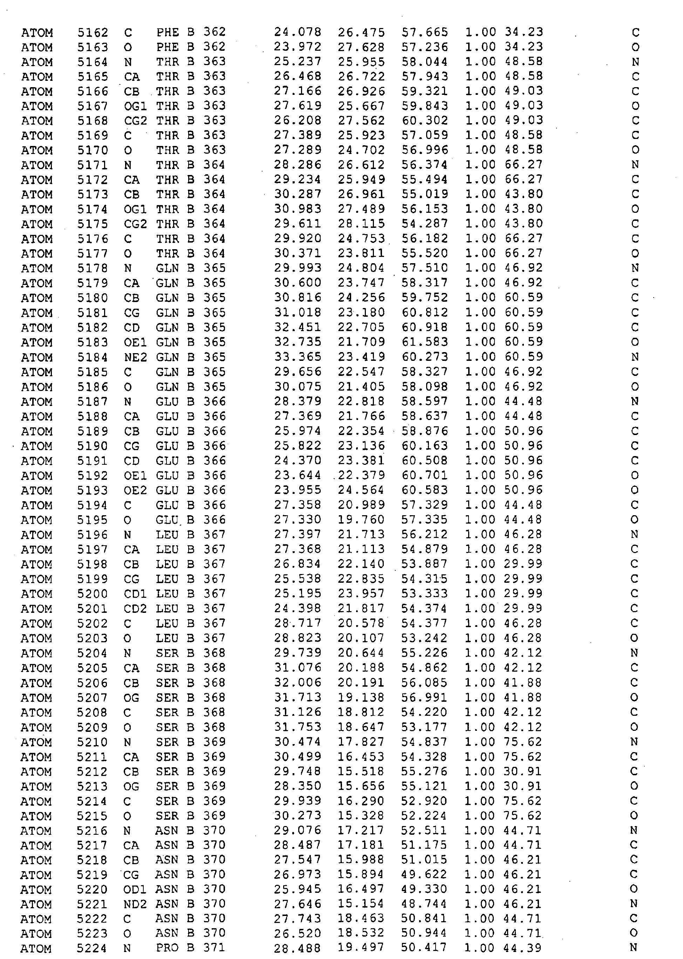

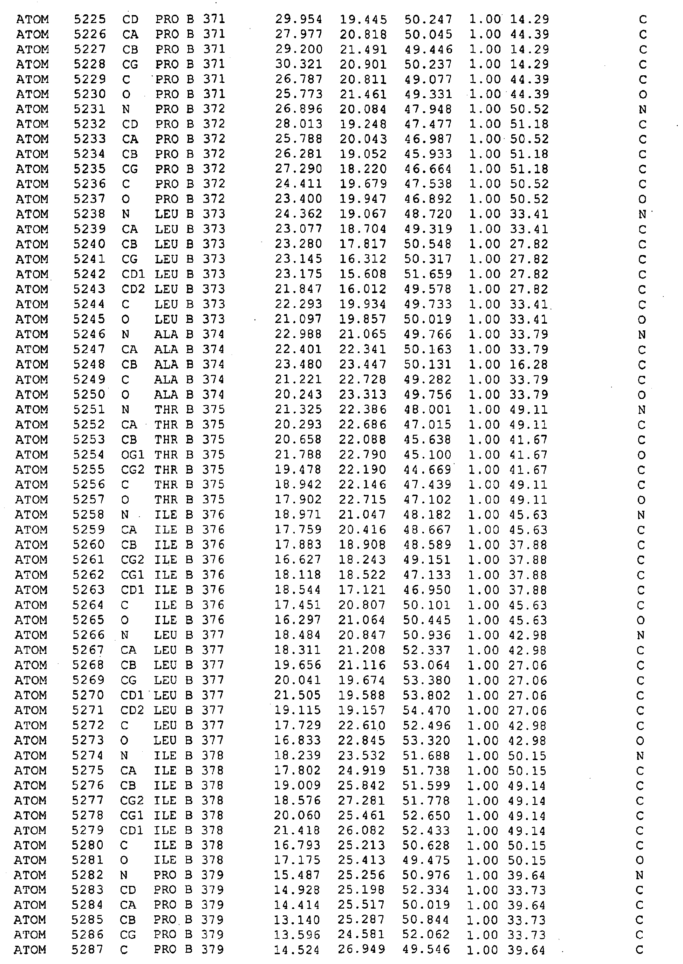

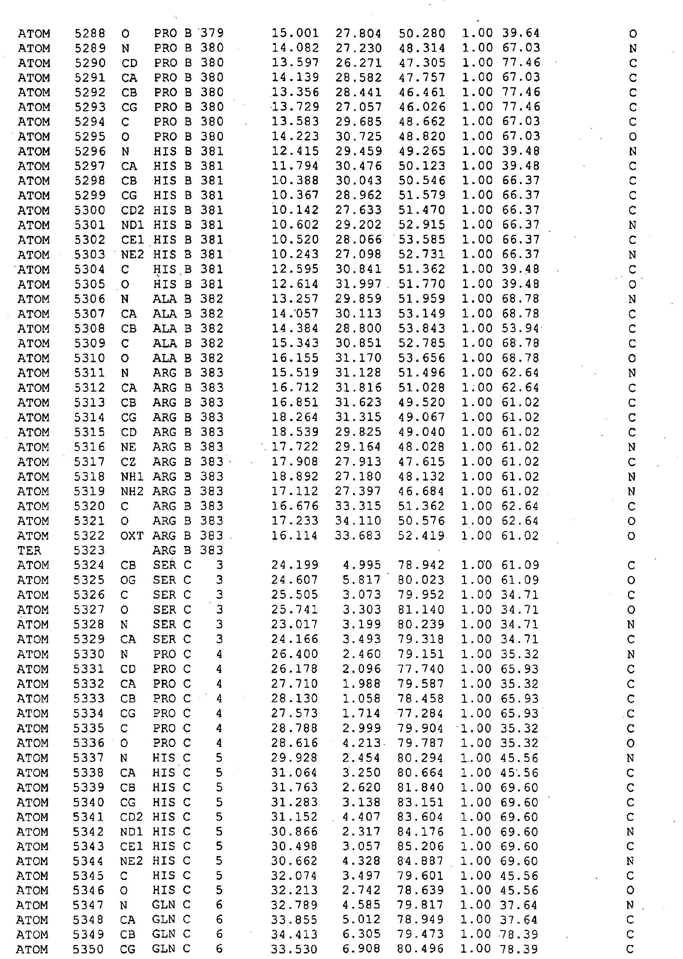

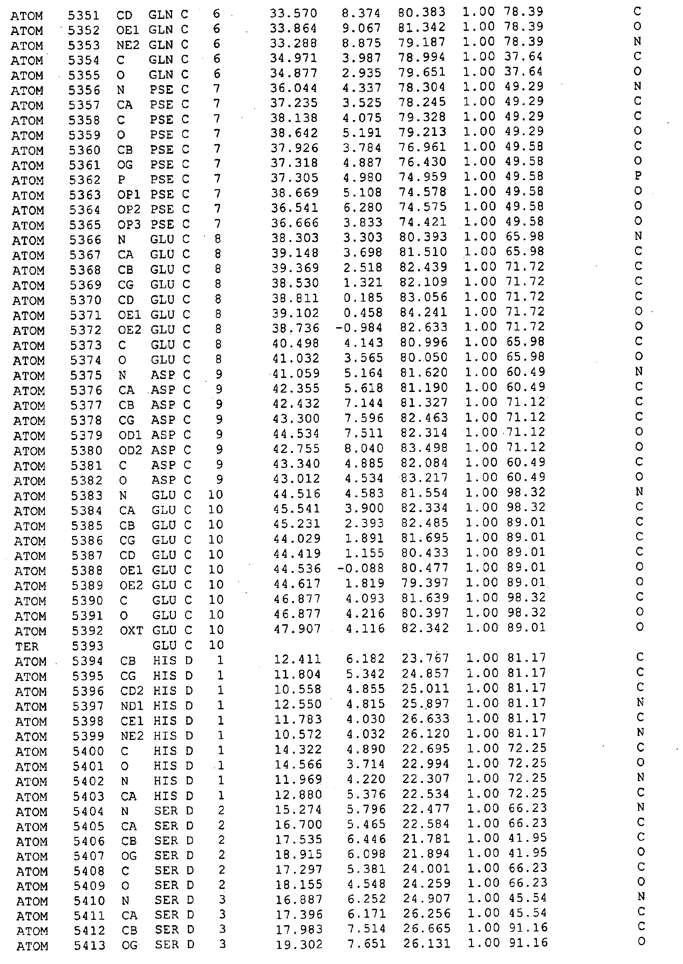

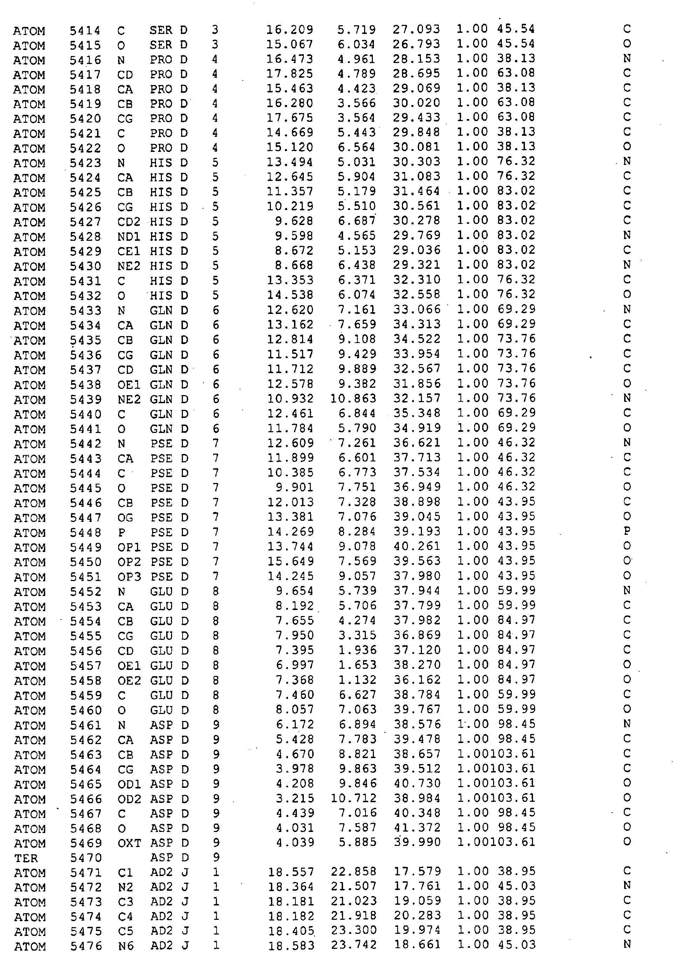

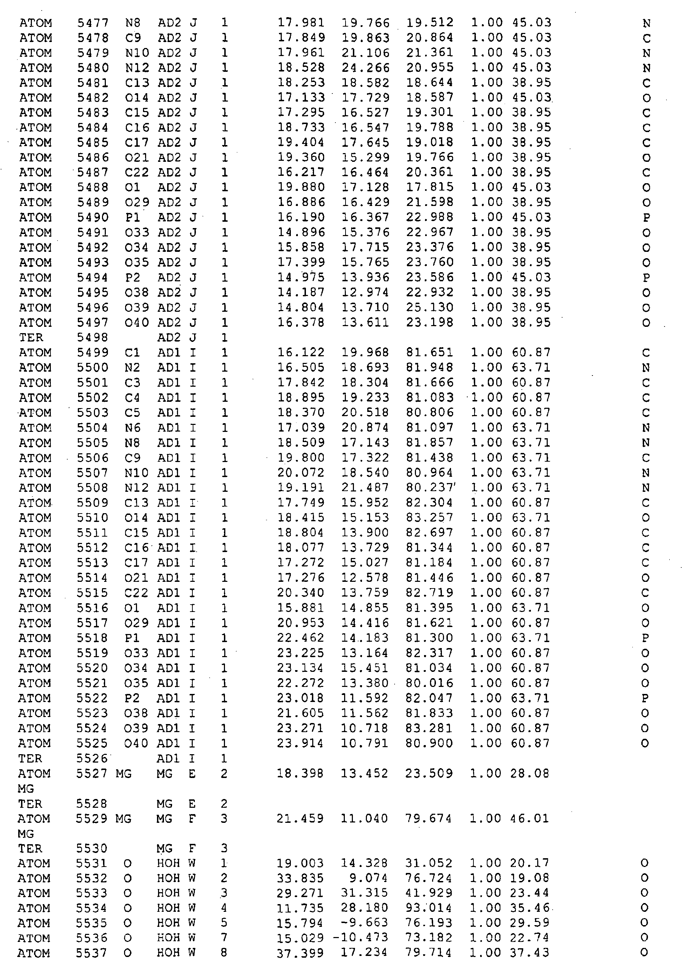

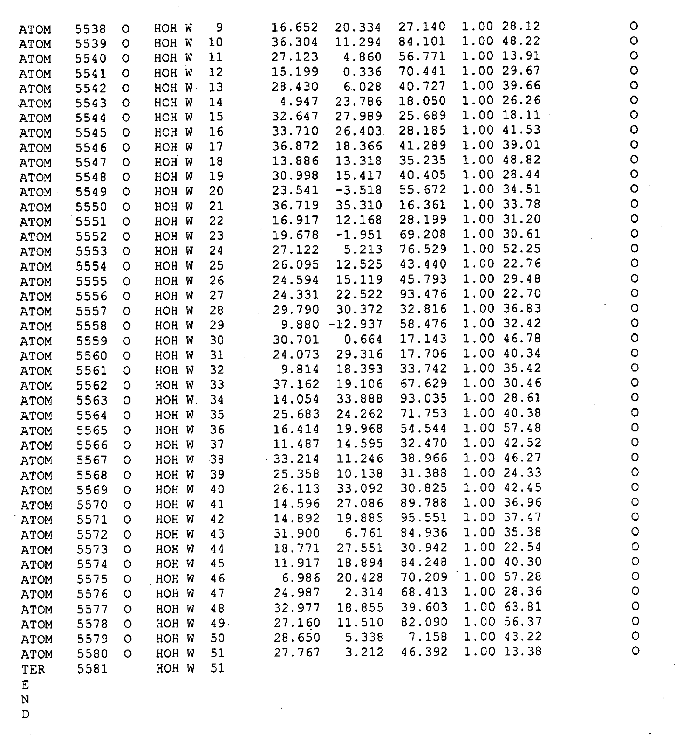

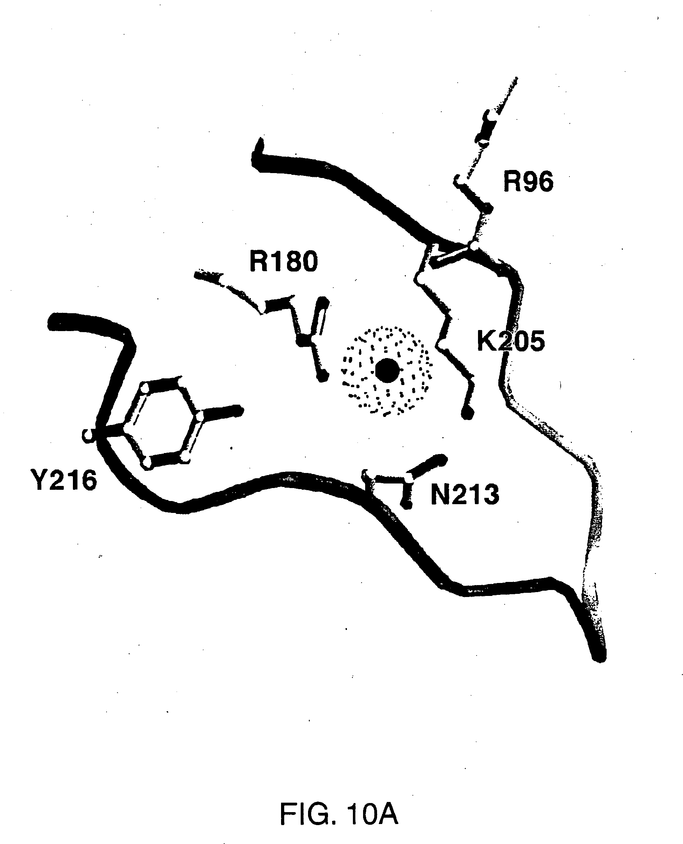

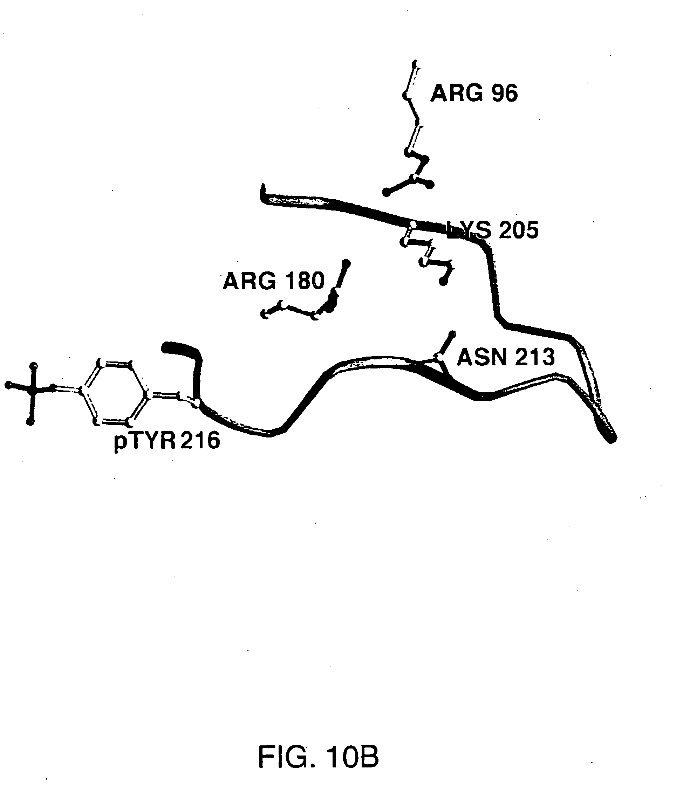

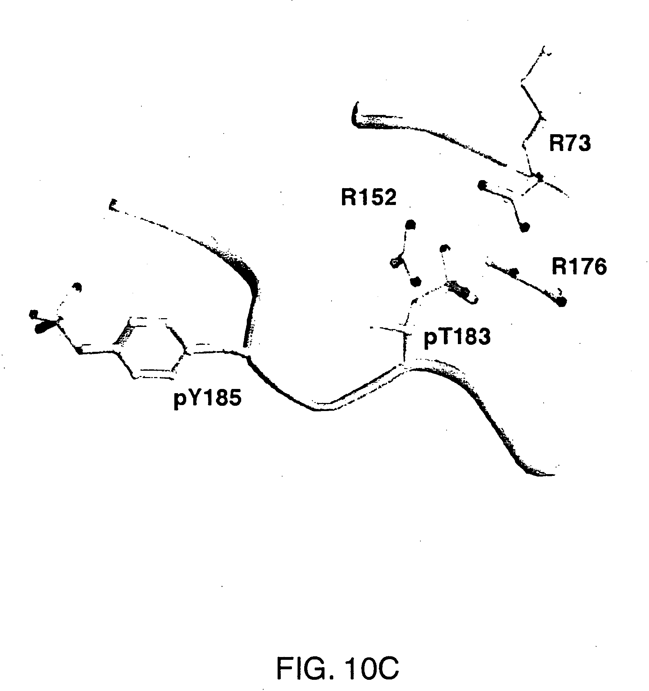

Figure 10 shows a comparison between the activation loops of unphosphorylated GSK-3β (Figure 10A), phosphorylated GSK-3β (Figure 10B), and phosphorylated p38γ (Protein Data Bank accession number 1CM8)(Figure 7C). In Figure 10A, the side chains of residues R96, R180 and K205 are pointing to a phosphate ion that occupies the same position as the phosphate group of the phospho-threonine in activated p38γ (Figure 10C), activated CDK2 and activated ERK2. In Figure 10C, the phosphorylated Y216 is flipped out of the substrate binding groove, which is similar to the position of the phosphorylated Y185 of p38γ.

Figure 11A shows that in the unphosphorylated GSK-3β structure, the GSK-3β substrate-binding groove is occupied by a phosphate ion and residues 260 to 264 of a neighboring GSK-3β molecule.

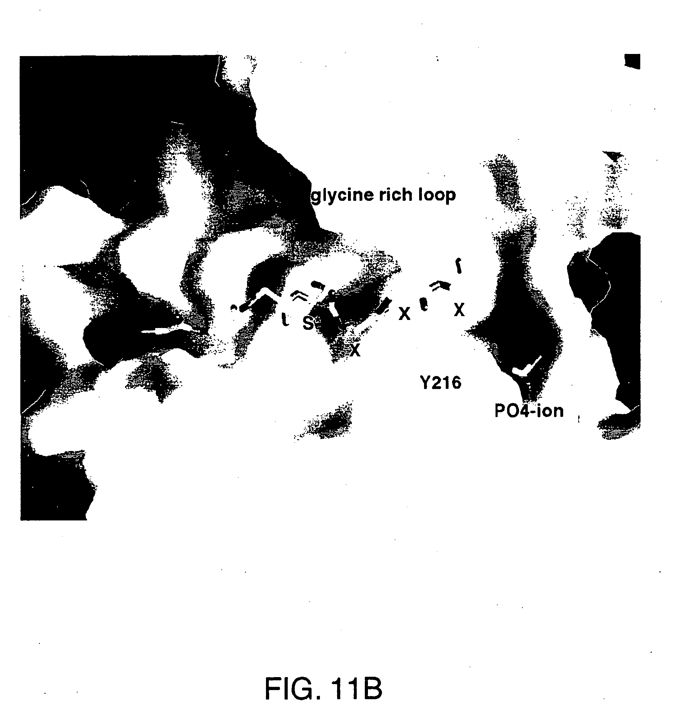

Figure 11B shows a model for the binding of the SXXXpS motif in the GSK-3β substrate-binding groove. The α-helical domains of GSK-3β and activated substrate bound CDK2 (Protein Data Bank accession number 1QMZ) were superimposed to model the positioning of a primed peptide in the GSK-3β substrate-binding groove. The side chains of the peptide residues except for the target serine S* have been removed for clarity. The phosphorylation site S* is positioned in front of the active site. The model estimates how the SXXXpS motif fits in the substrate-binding groove with the three residues bridging the gap between the P and P+4 serines. The phosphate group of the P+4 serine will occupy the same position as the phosphate ion found in the crystal structure.

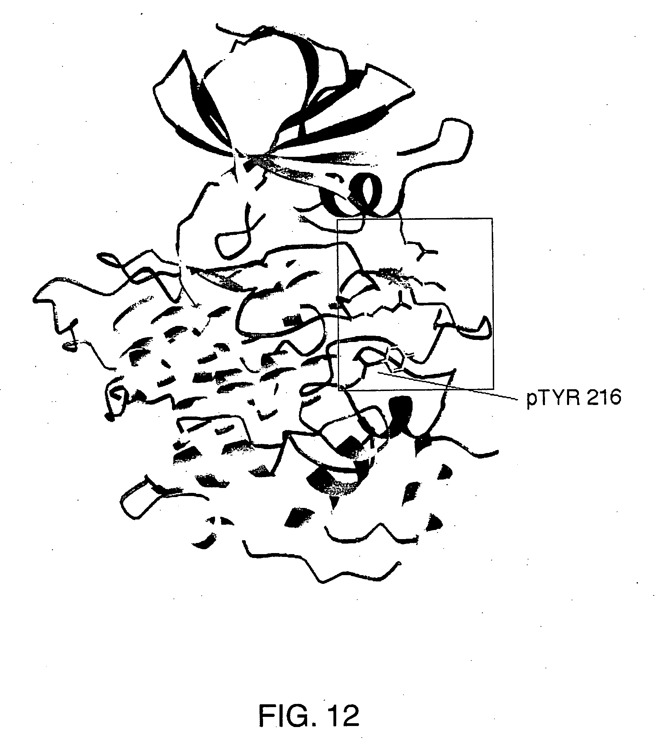

Figure 12 depicts a ribbon diagram of phosphorylated GSK-3β. The activation loop and the phosphorylated Y216 are indicated.

Figure 13 presents the inhibitor2 bound in the active site of unphosphorylated GSK-3β. The hinge and glycine rich loop are indicated.

Figure 14 presents the view of inhibitor1 bound in the active site of phosphorylated GSK-3β.

Figure 15 presents the view of inhibitor3 bound in the active site of unphosphorylated GSK-3β.

Figure 16 presents the conformation of Gln185 in the active site when bound to inhibitor4.

Figure 17 depicts the overall structure of phosphorylated GSK-3β in complex with ADP and glycogen synthase peptide. The N-terminal domain corresponds to the β-strand domain and includes residues 37 to 138. The α-helical domain of the GSK-3β kinase core corresponds to residues 139 to 343. The C-terminal 34 residues are not part of the kinase core but pack against the α-helical domain. Key features of the kinase fold, such as the glycine rich loop, the hinge and the activation loop are indicated. The ADP-Mg complex occupying the active site is shown. The glycogen

synthase peptide (residues 650 to 658) bound in the substrate binding groove is also shown here.

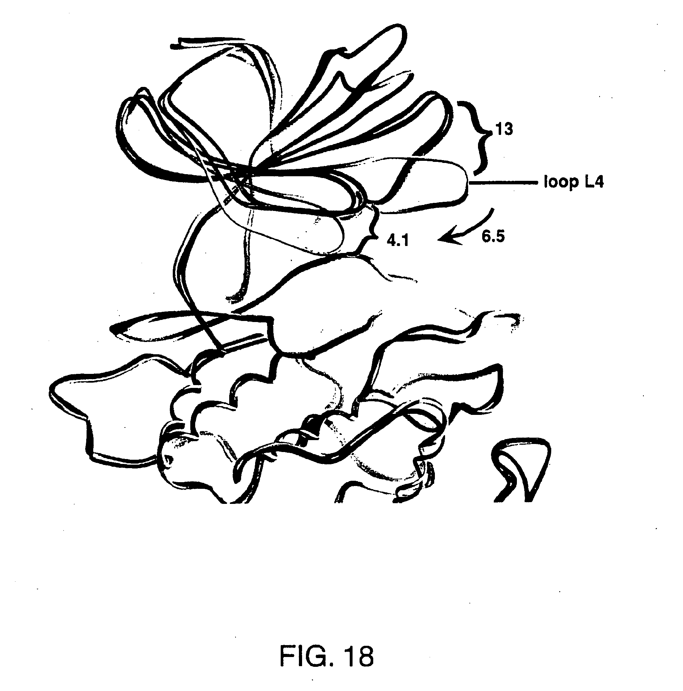

Figure 18 depicts the β-strand domain rotation induced by ADP and glycogen synthase peptide. Superposition of the α-helical domains of unphosphorylated (dark shade), phosphorylated apo-GSK3 (gray shade) and phosphorylated ADP peptide bound GSK-3β (light shade). The β-strand domain of the phosphorylated GSK-3β-ADP-peptide complex, rotated 6.5 Å in comparison to unphosphorylated and phosphorylated apo-GSK-3. The loop between β-3 and α-C (residues 87 to 95) moved 13 Å to contact the glycogen synthase peptide (not shown).

Figure 19A depicts a stereo view of the active site occupied by the ADP-Mg complex. The adenine base is surrounded by hydrophobic residues and makes two hydrogen bonds with the backbone of the hinge region. The catalytic residues involved in the phosphate transfer to the P-site serine surround the two non-transferable ADP phosphates.

Figure 19B is a schematic drawing of the hydrogen bond network that connects the ADP-Mg complex to the P-site serine via the catalytic residues.

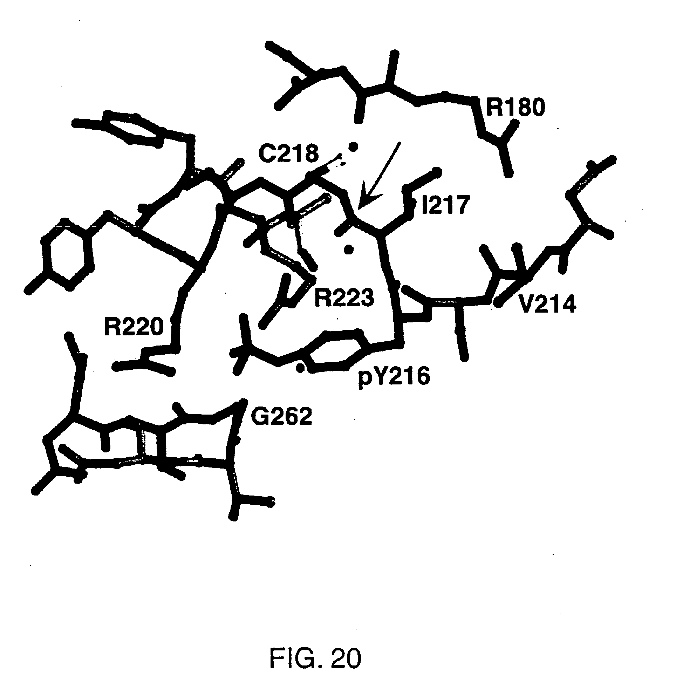

Figure 20 depicts the environment of phosphotyrosine 216 in the structure of phosphorylated GSK-3β in complex with ADP and glycogen synthase peptide. The phosphate moiety binds two arginine side chains (Arg 220 and Arg 223) resulting in charge neutralization. The arrow indicated the 180 degree flip of I217 backbone carbonyl.

Figure 21 depicts the glycogen synthase peptide in the substrate binding groove. The phosphoserine (pSer 656) binds Arg 96, Arg 180 and Lys 205, which results in the proper alignment of the α-helical and β-strand domains. pTyr 216 moves its side chain out of the substrate binding groove to make contact with the side chains of Arg 220 and Arg 223.

Figure 22 shows a diagram of a system used to carry out the instructions encoded by the storage medium of Figures 23 and 24.

Figure 23 shows a cross section of a magnetic storage medium.

Figure 24 shows a cross section of a optically-readable data storage medium.

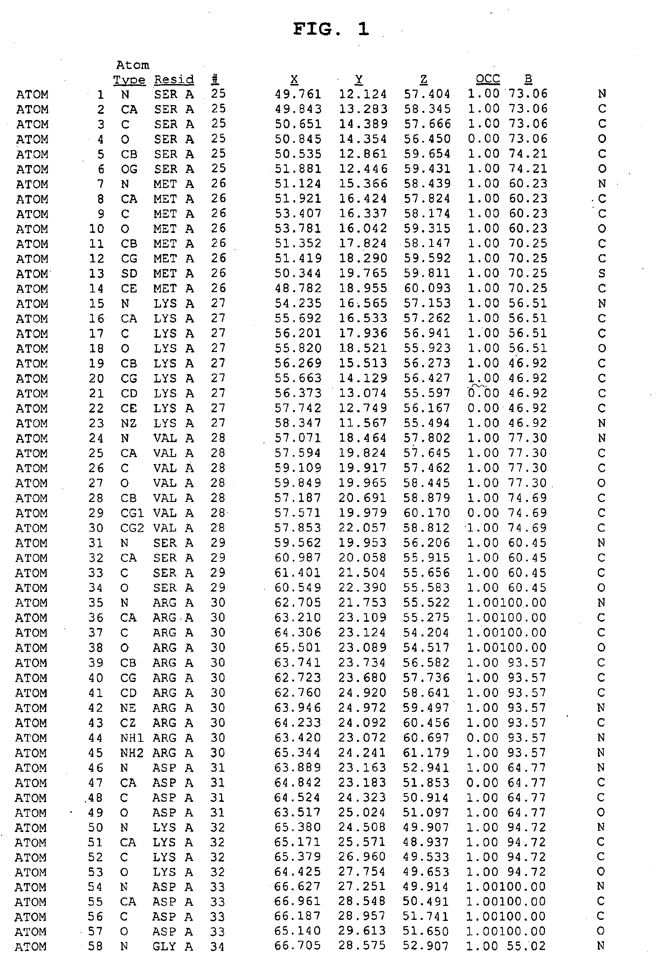

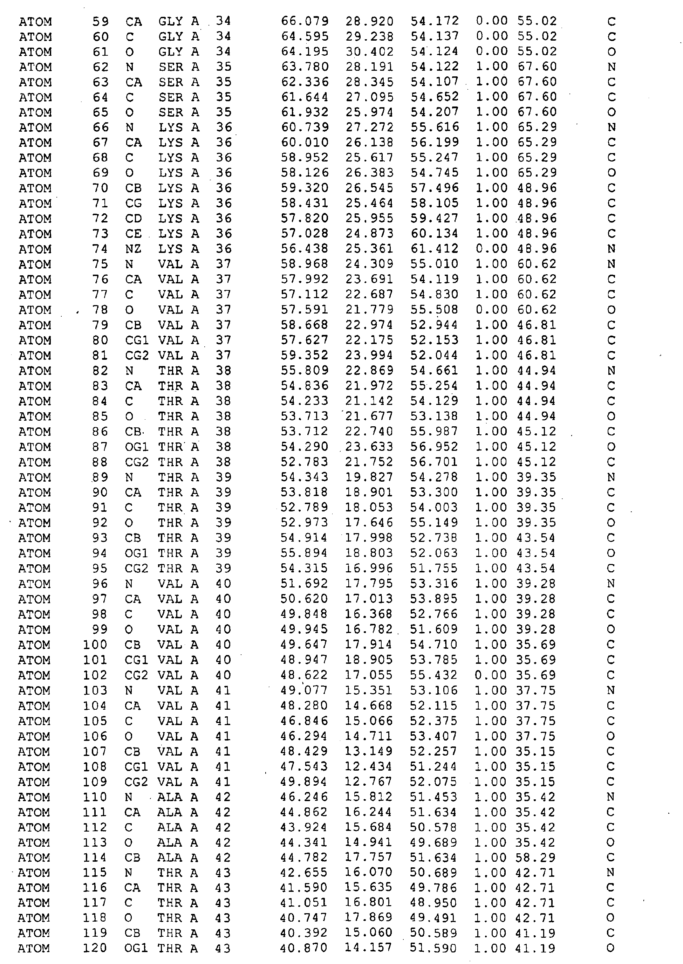

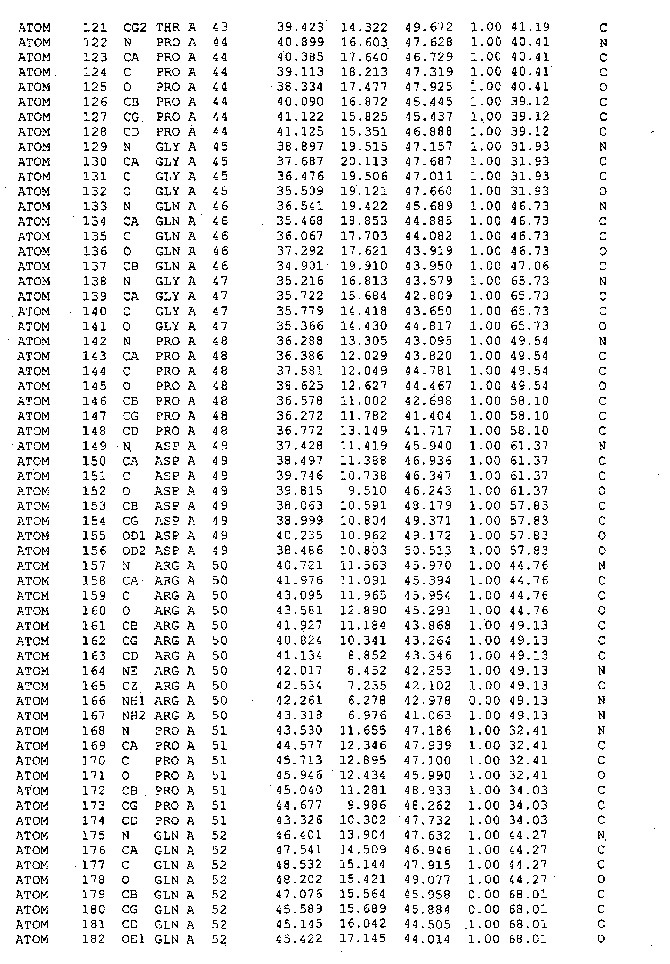

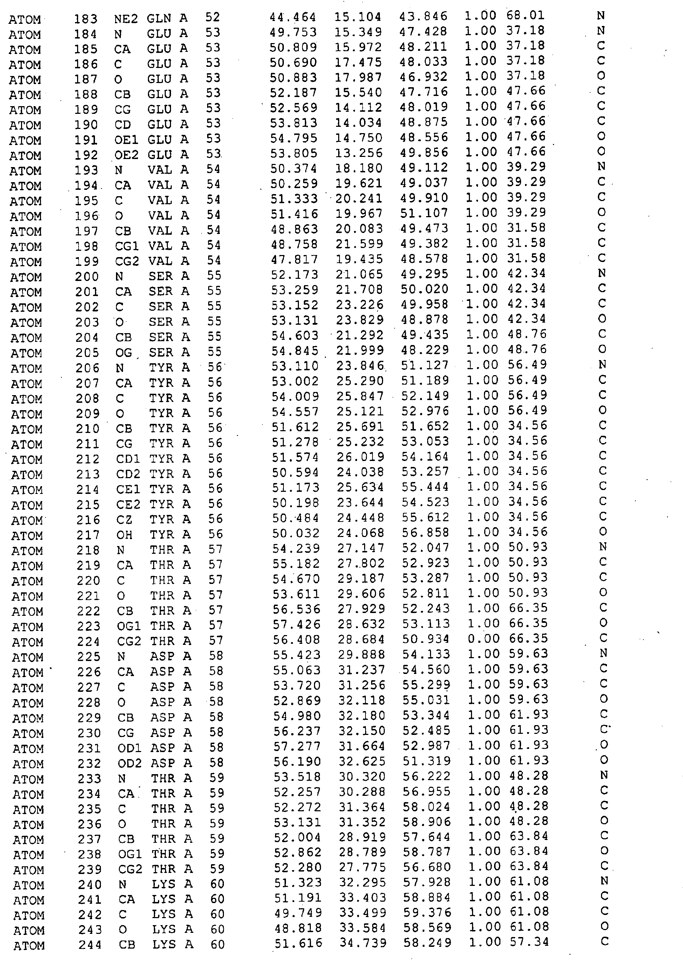

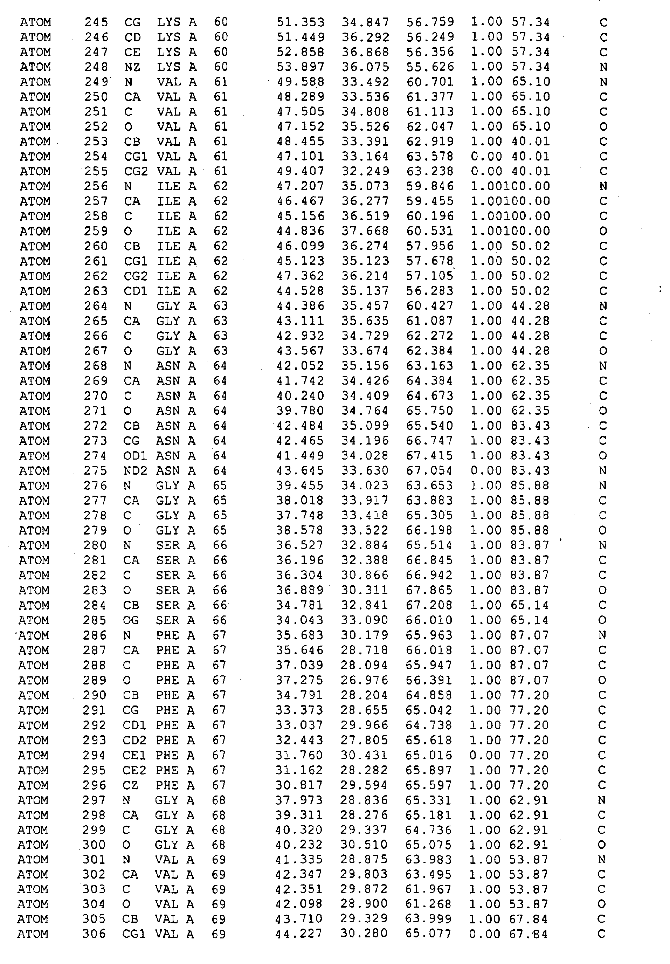

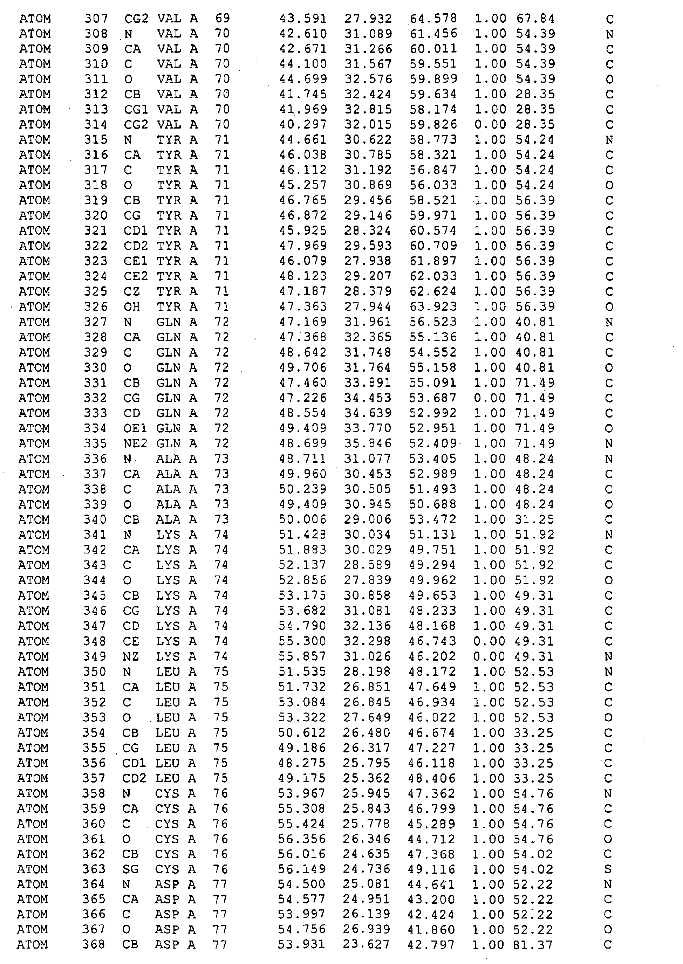

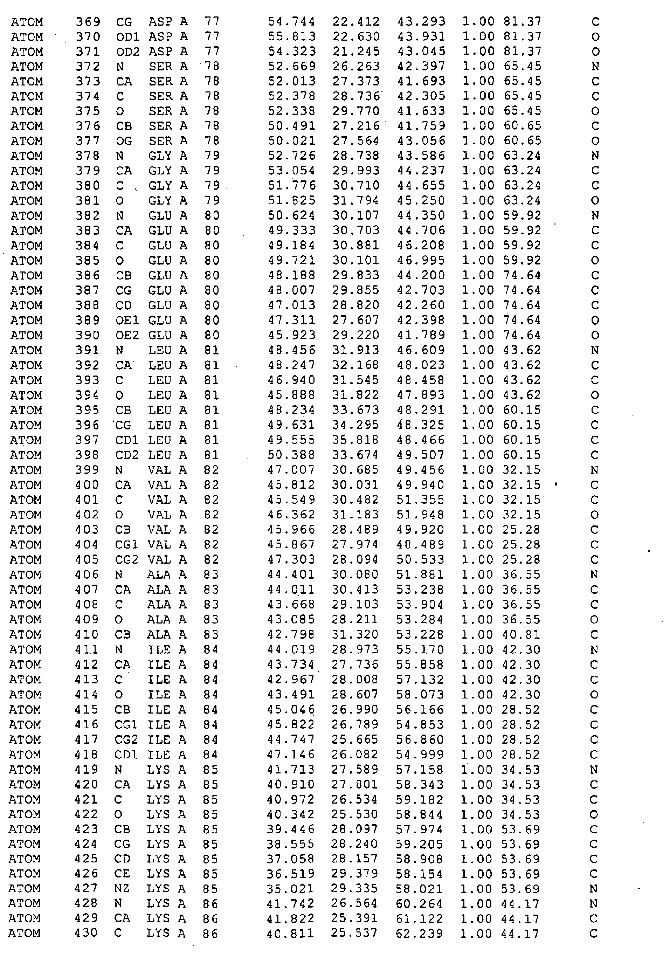

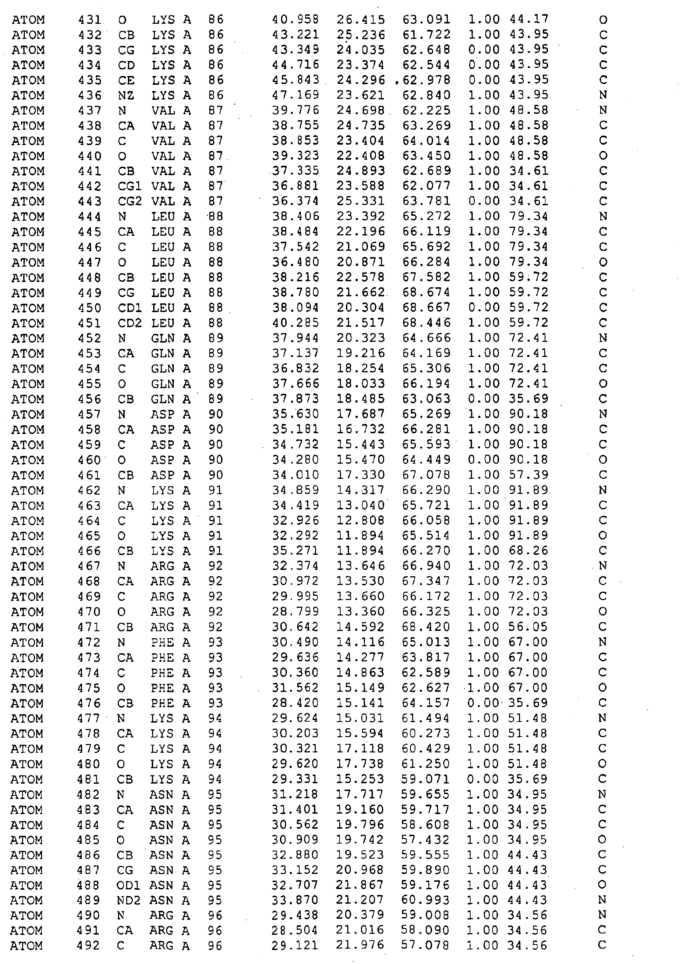

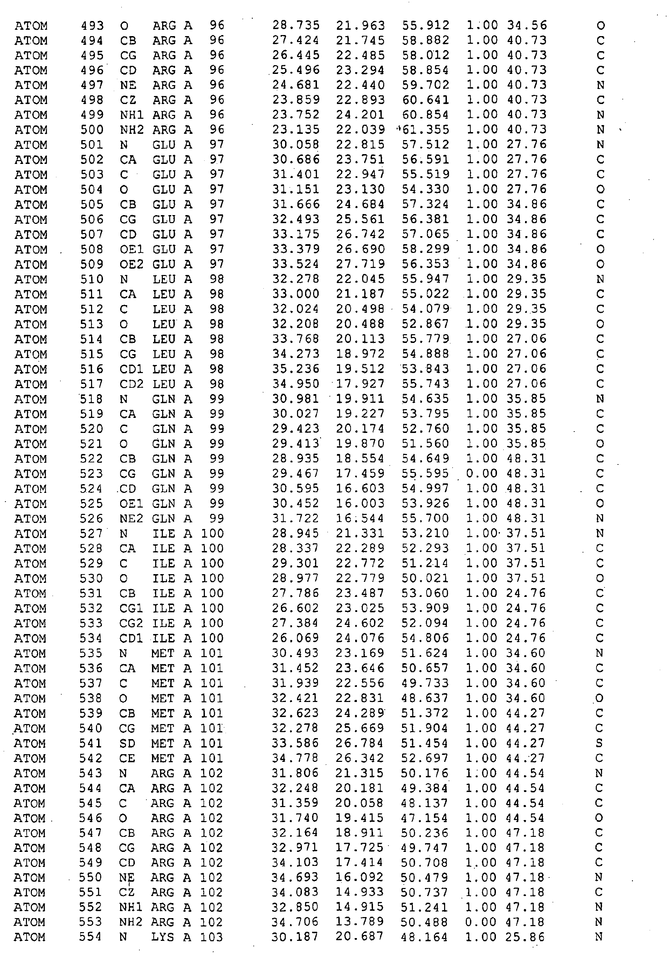

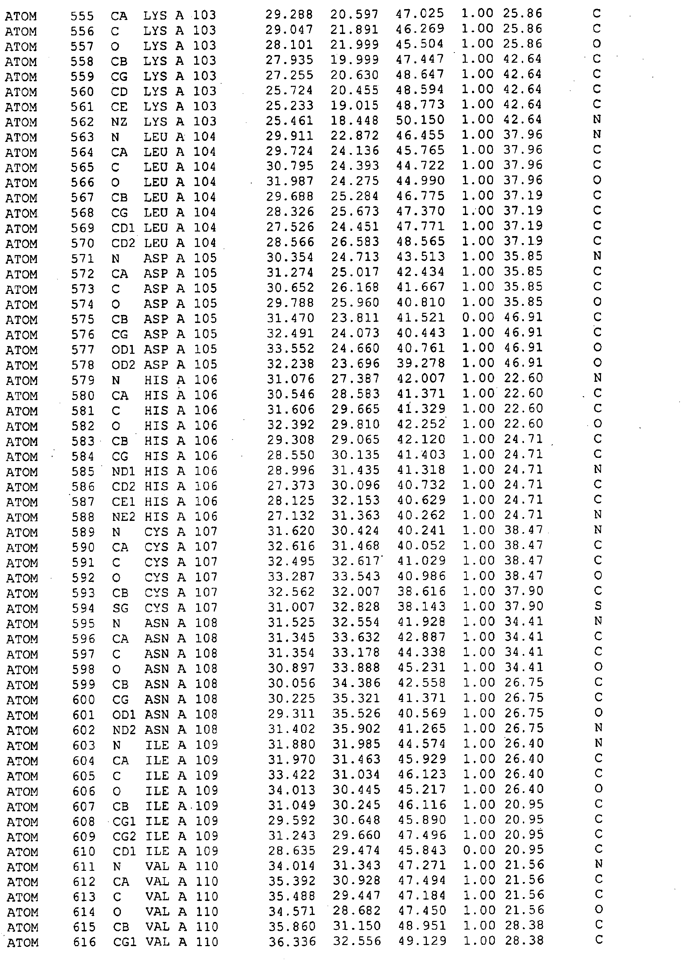

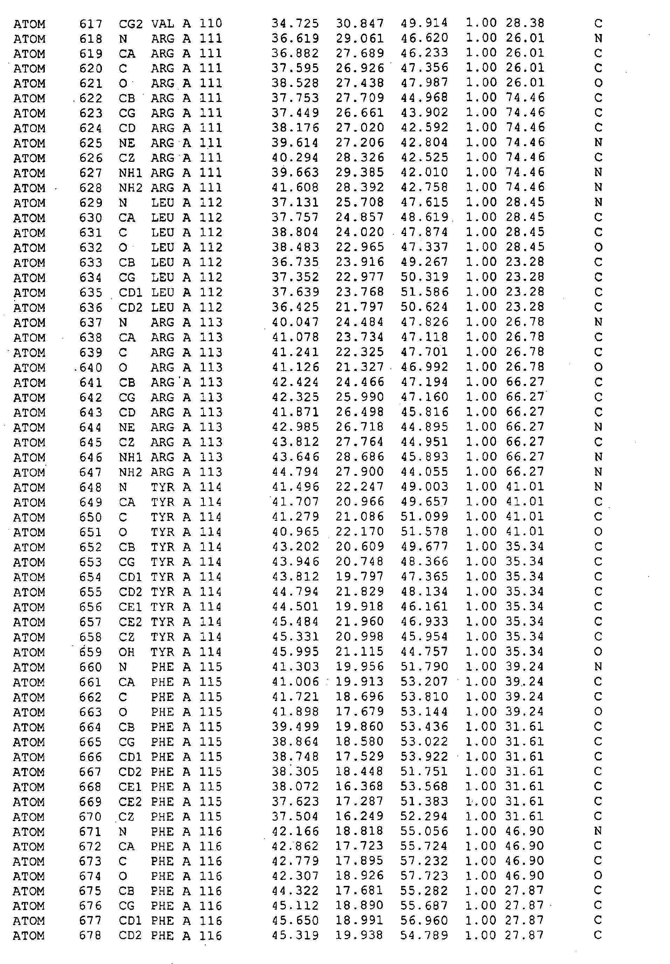

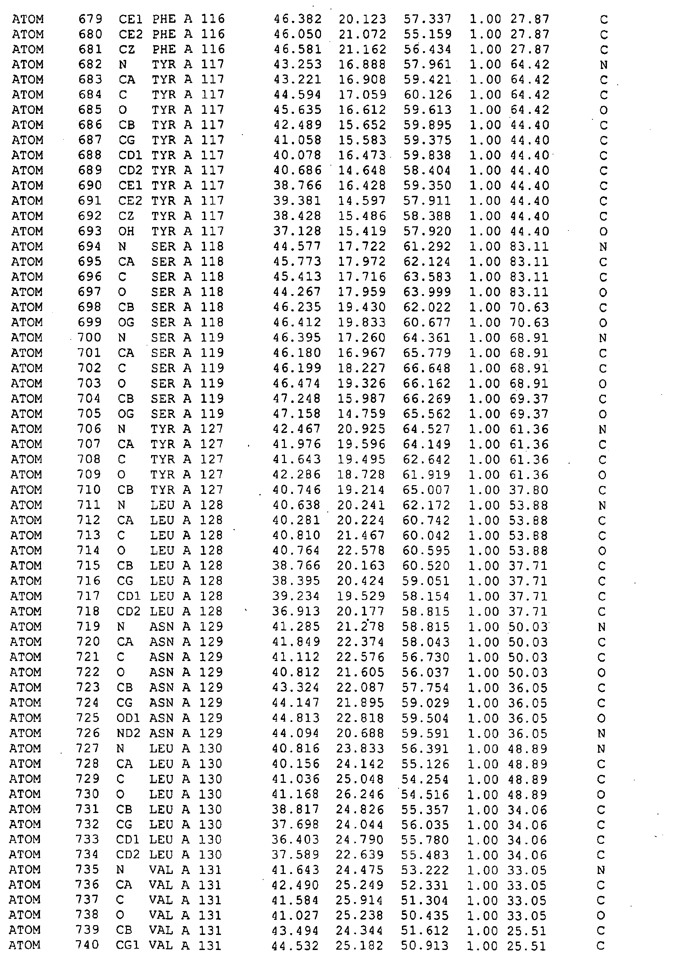

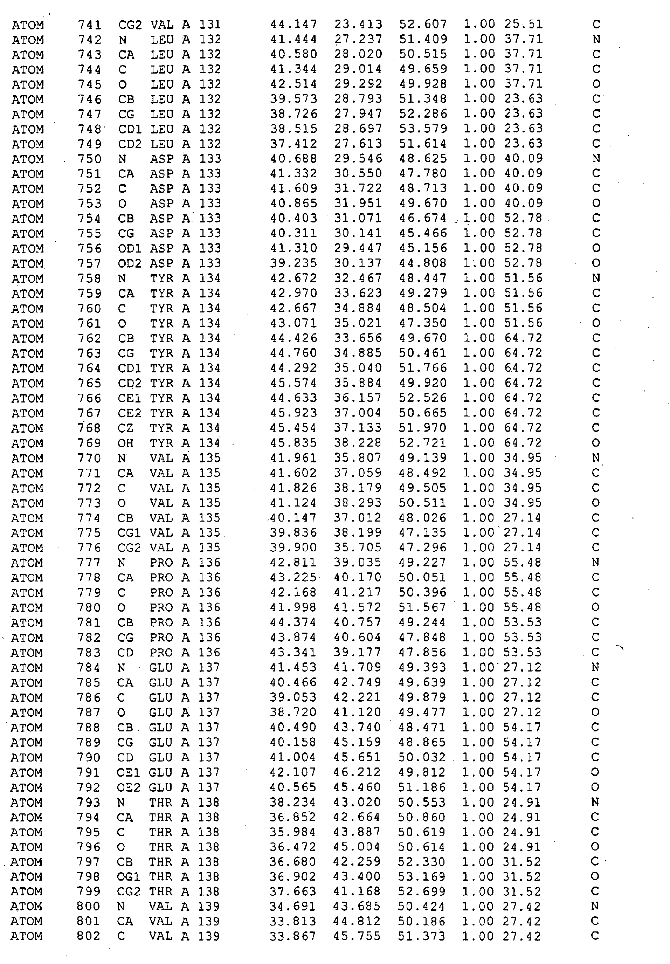

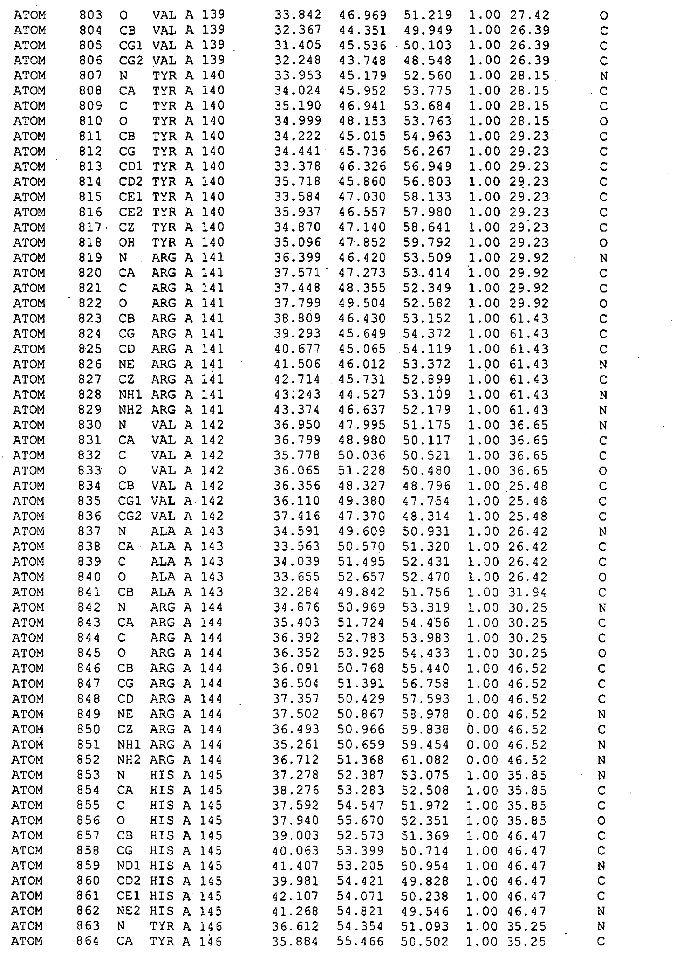

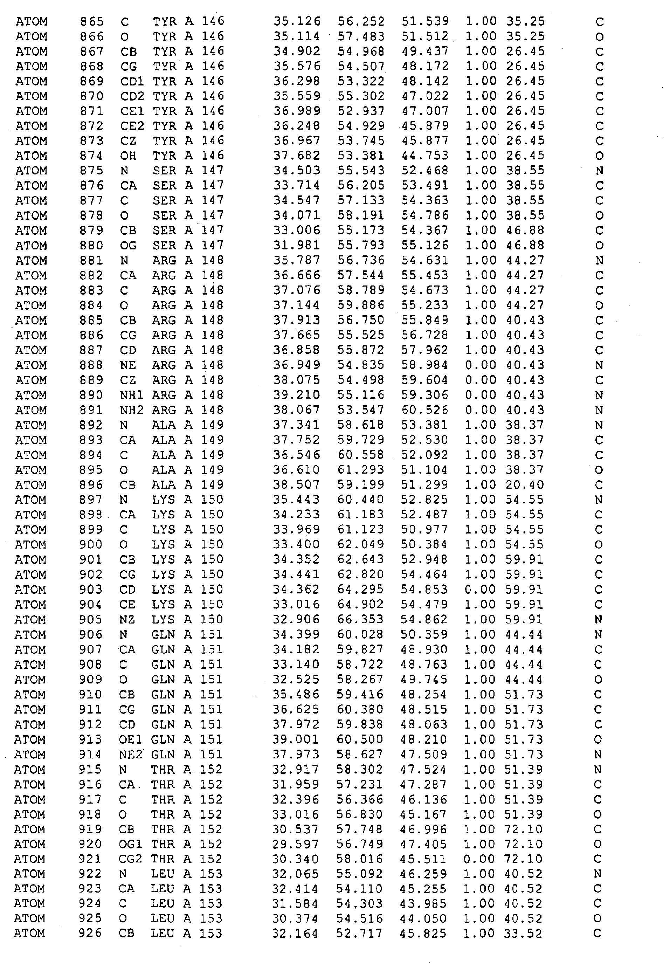

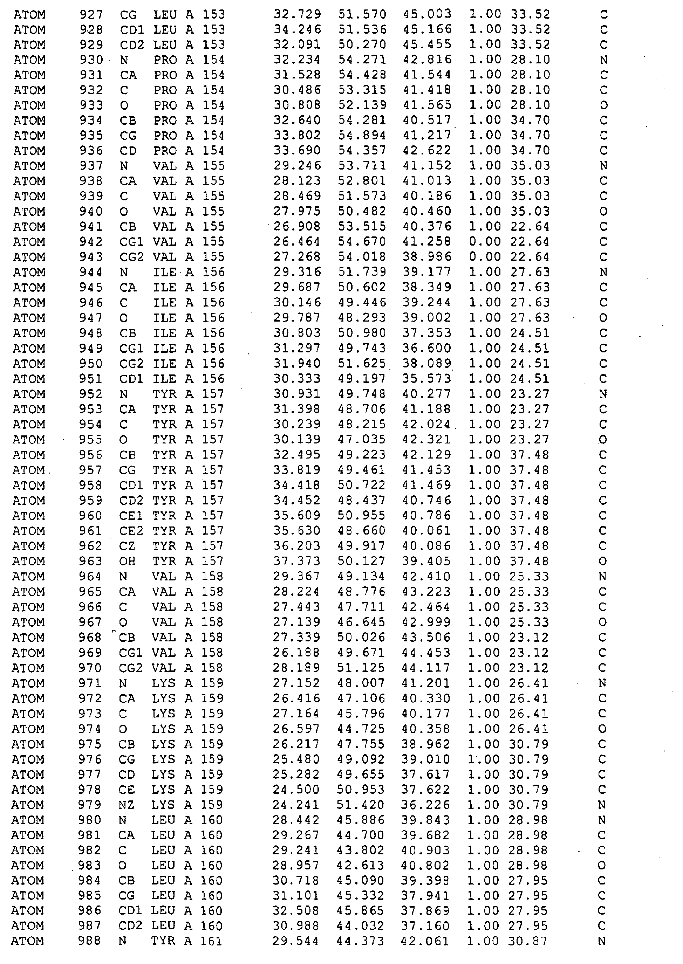

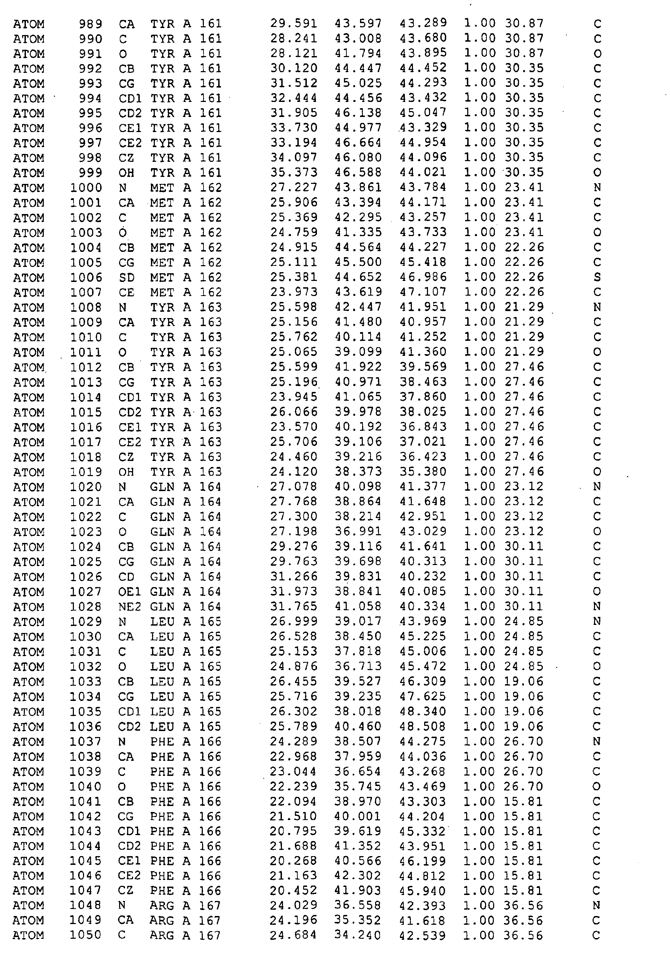

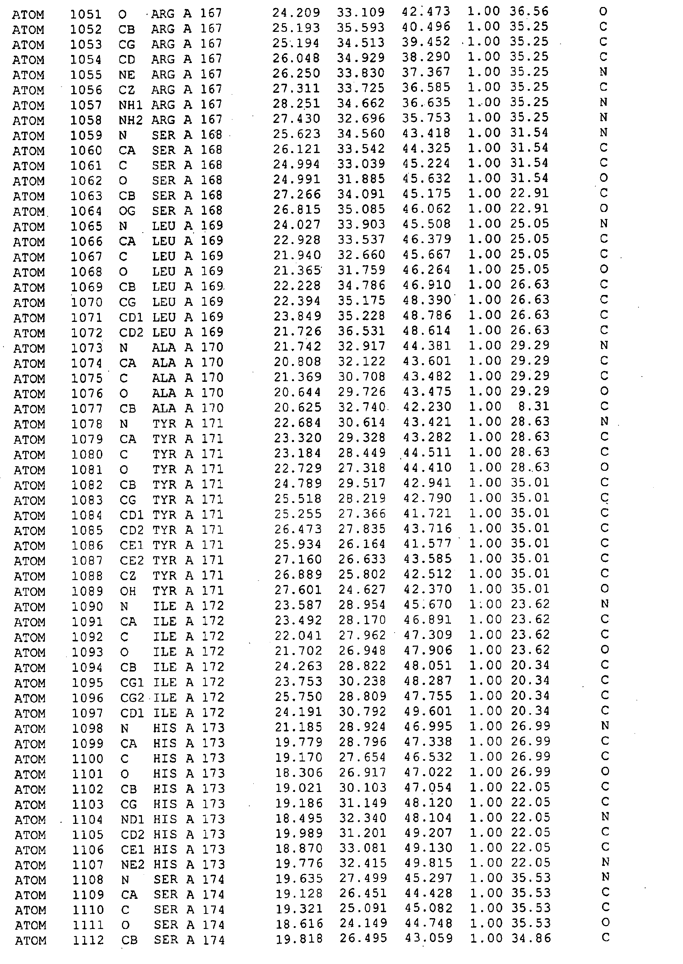

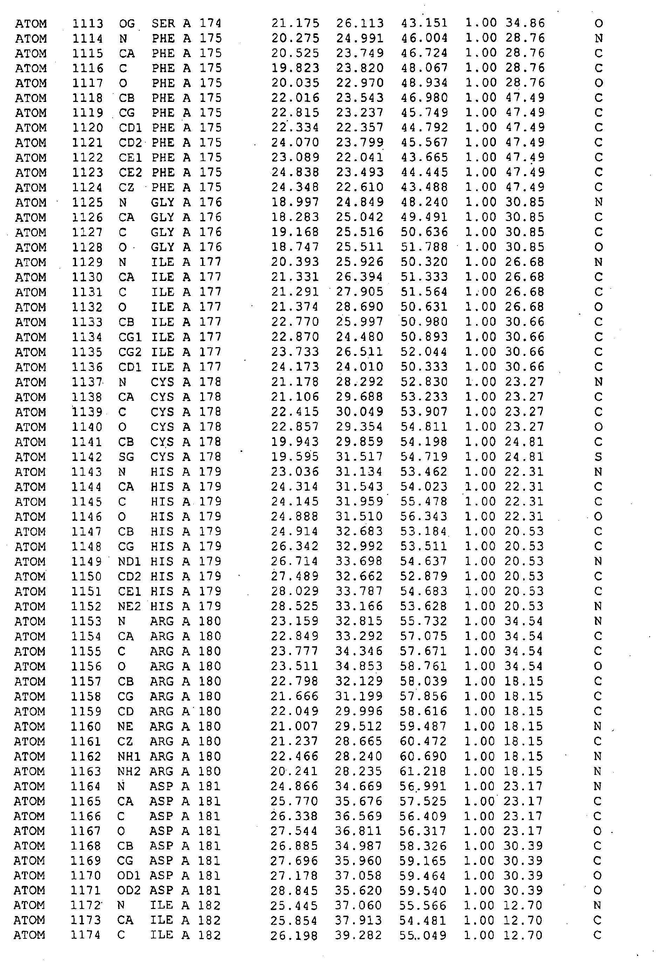

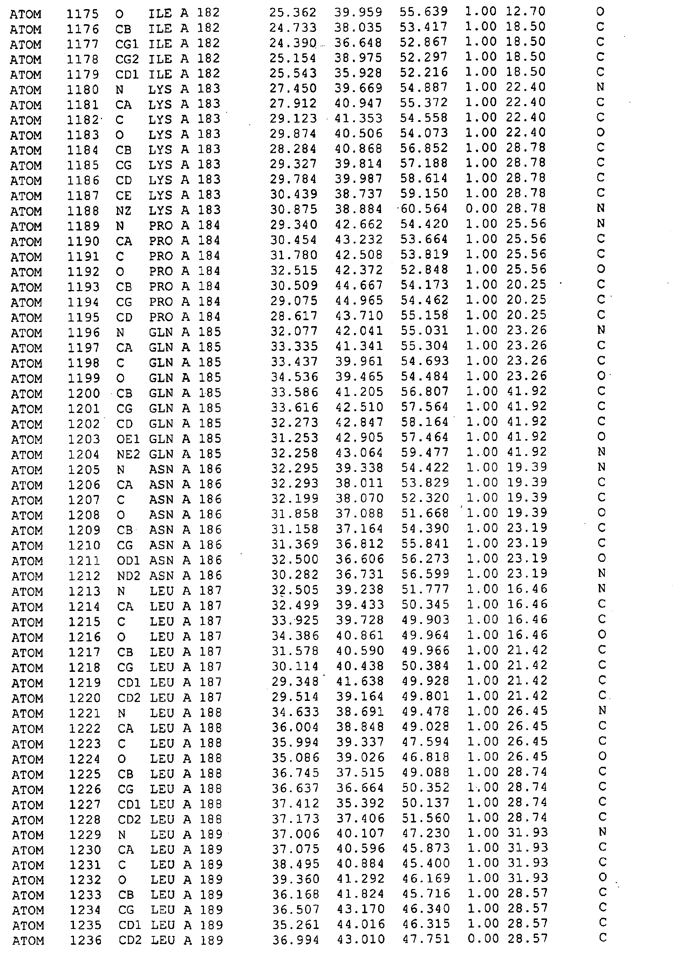

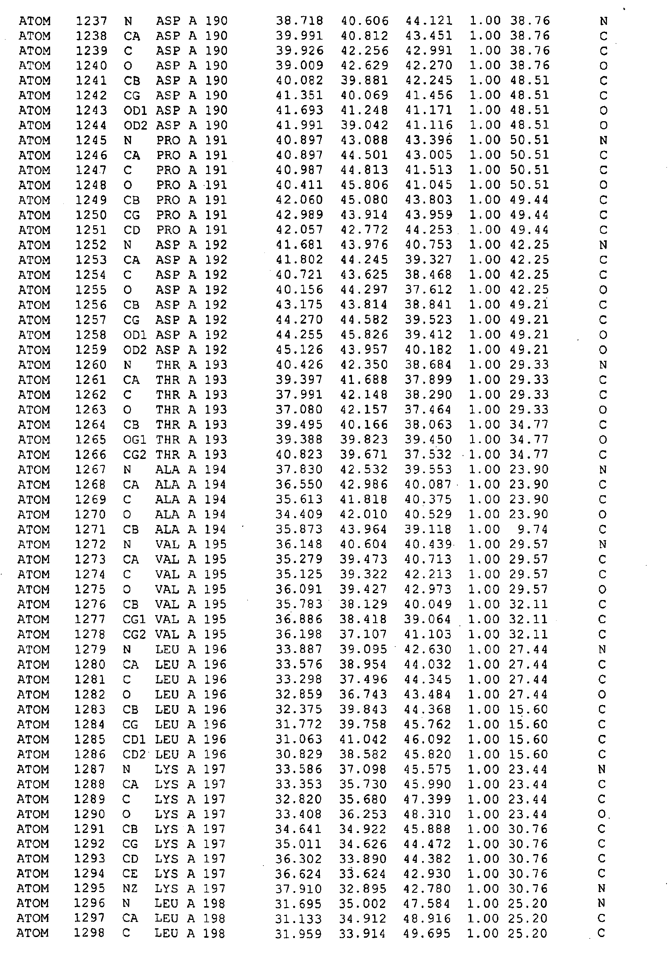

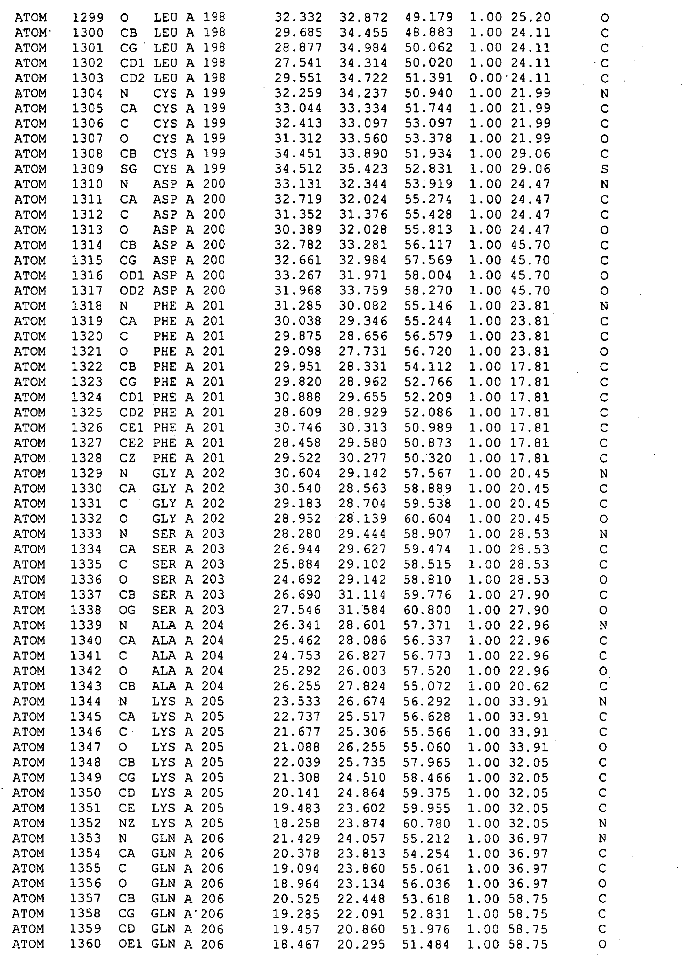

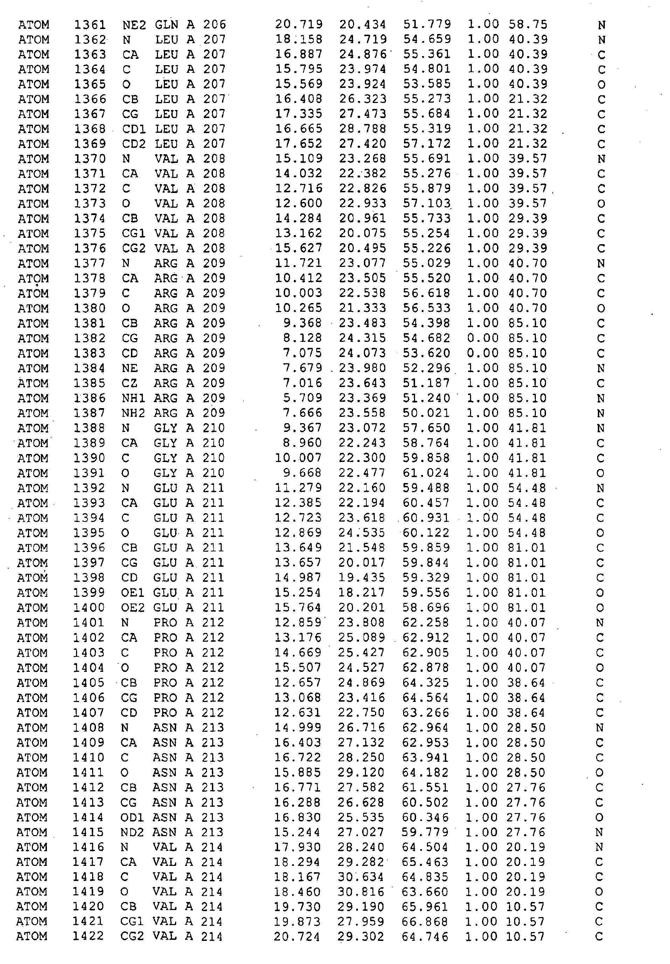

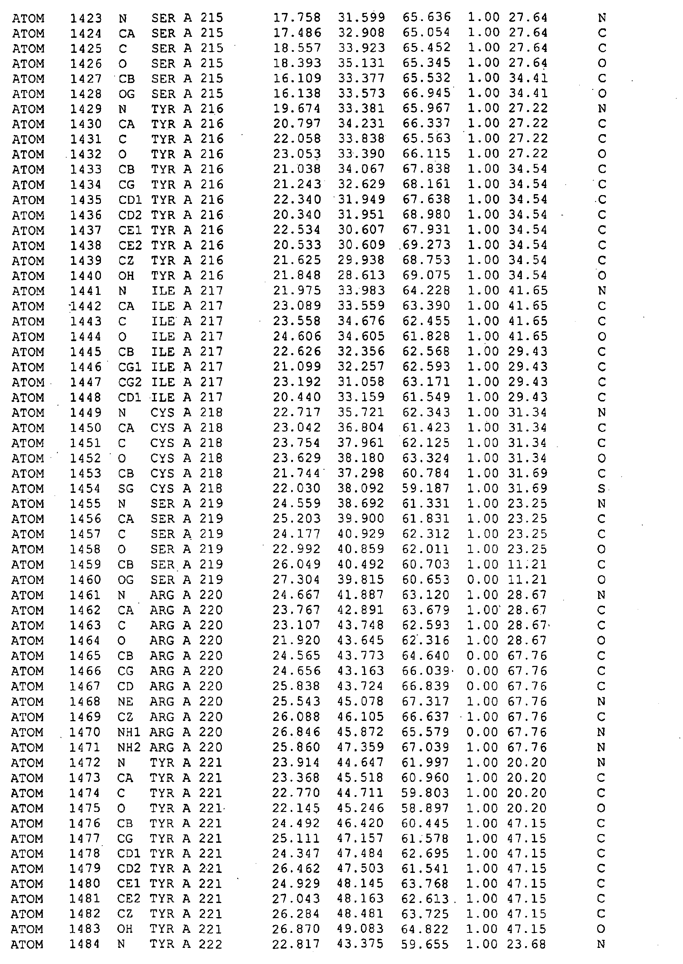

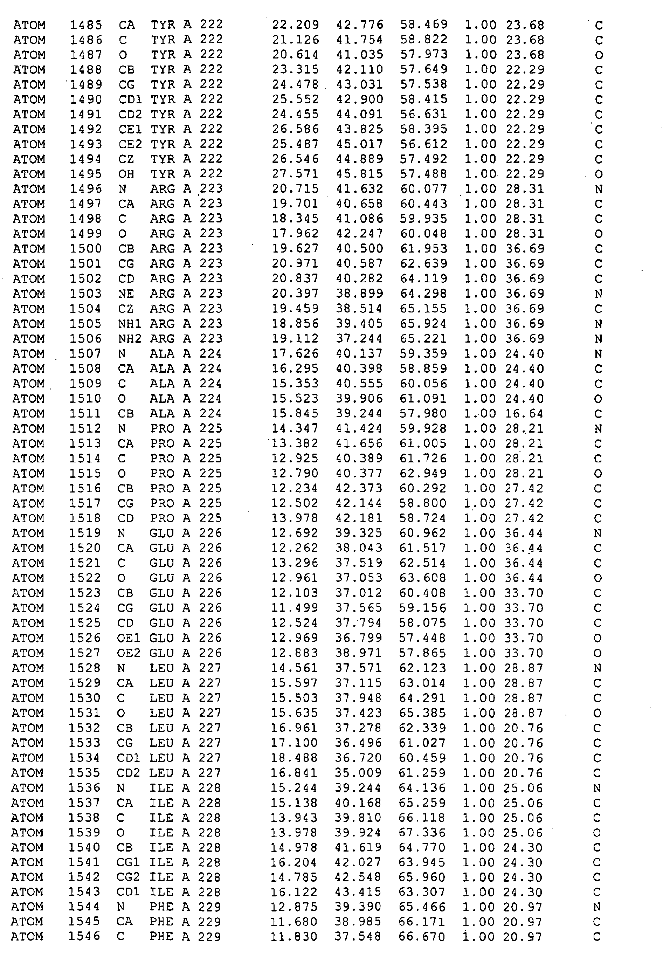

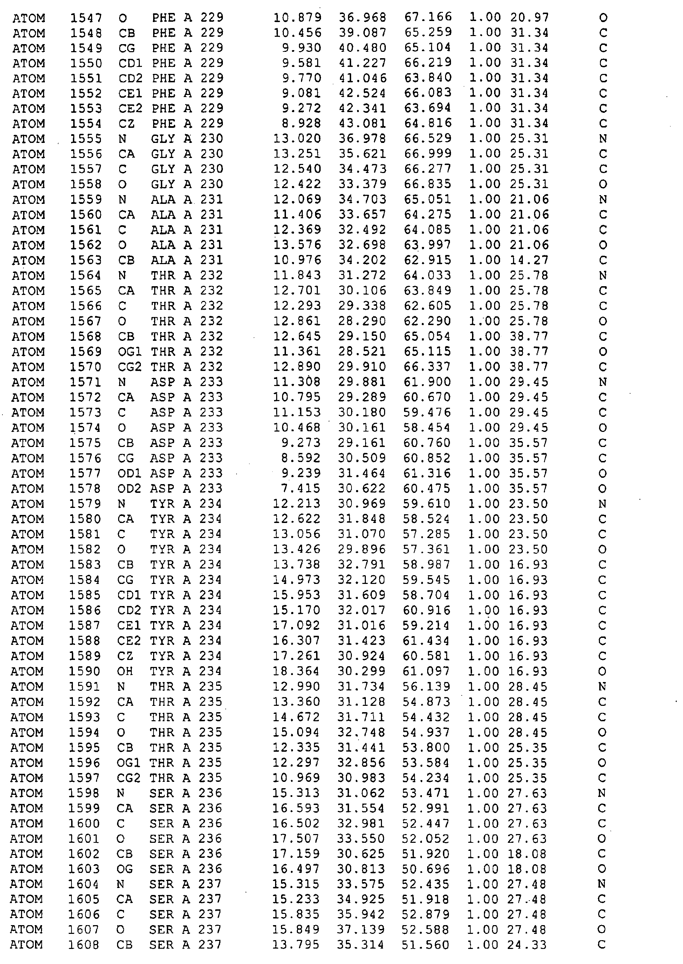

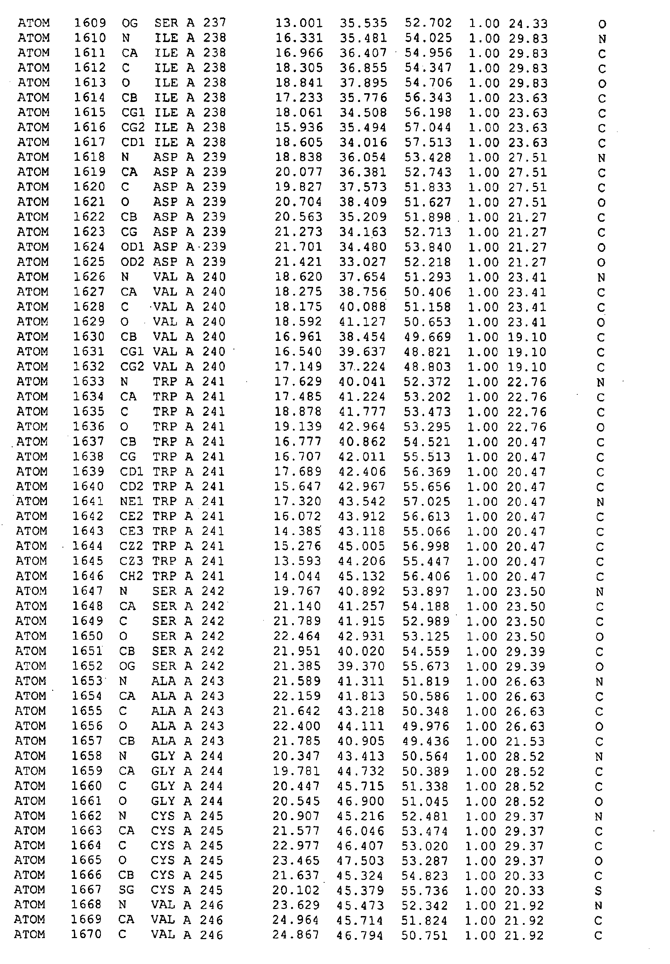

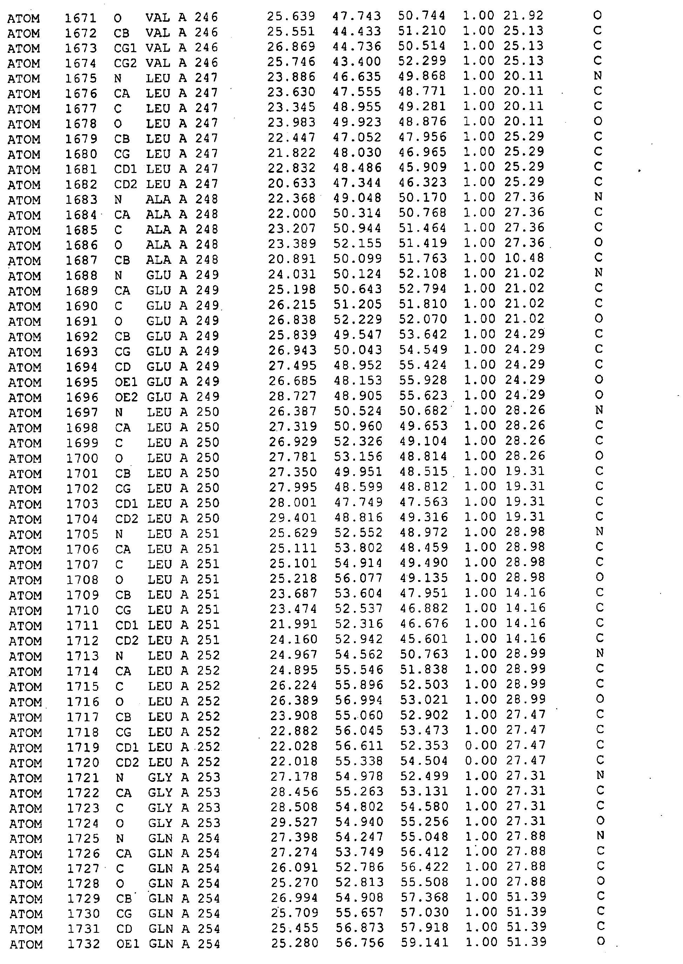

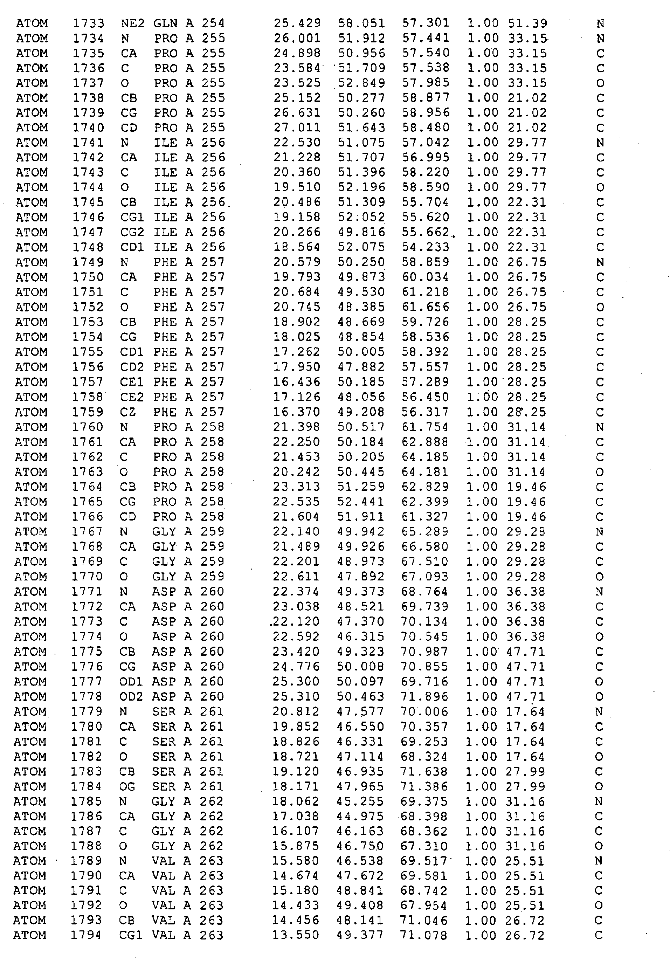

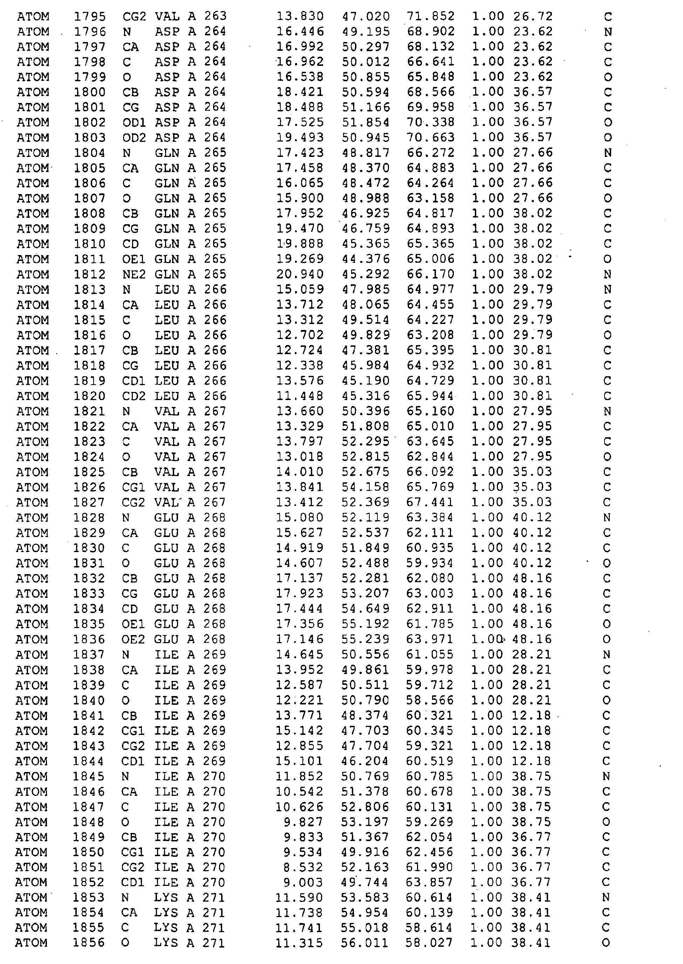

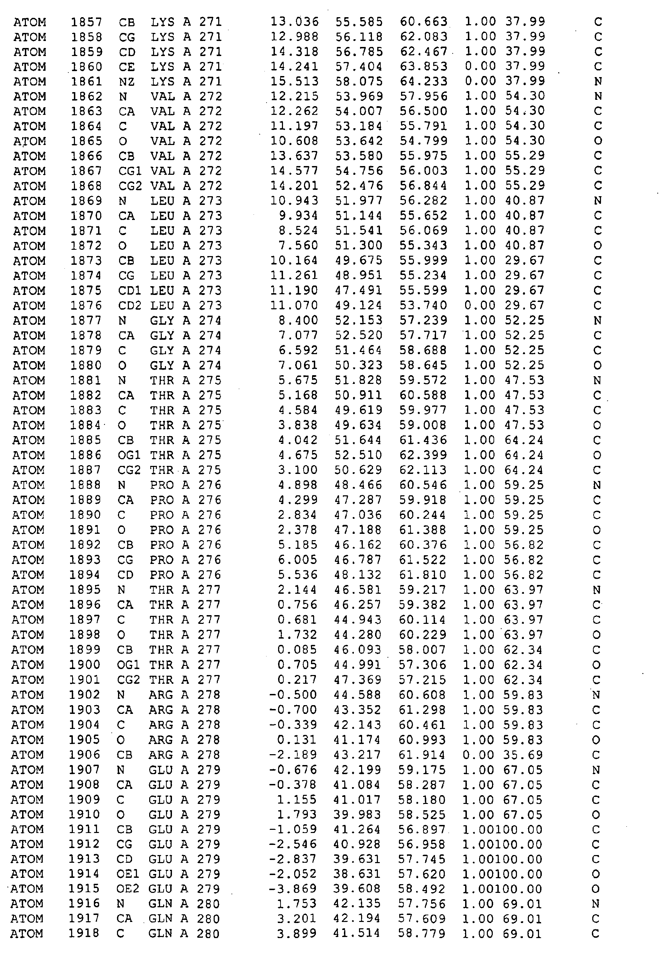

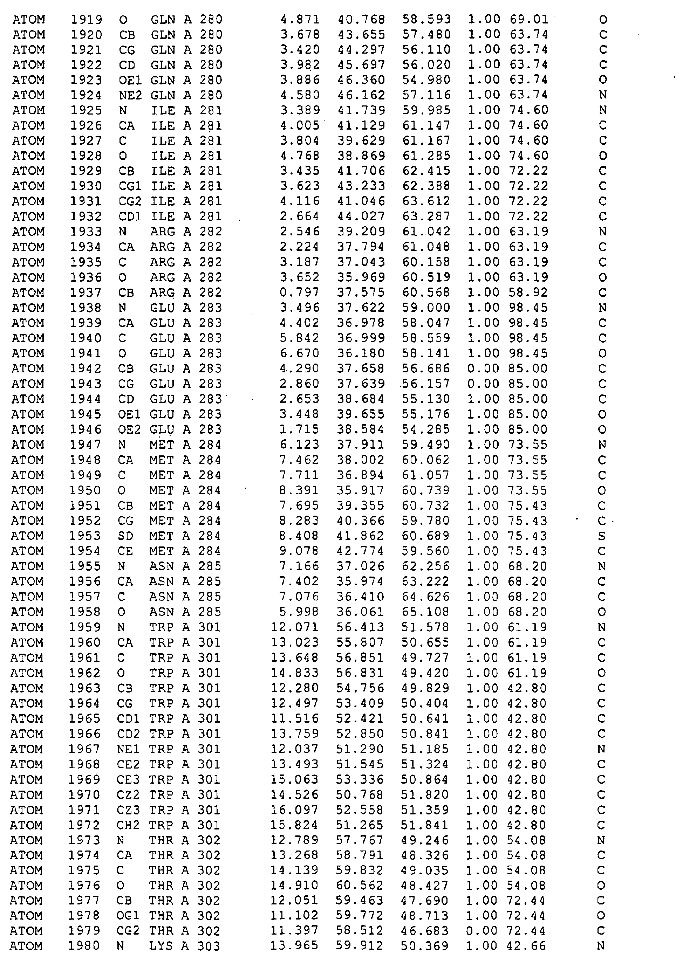

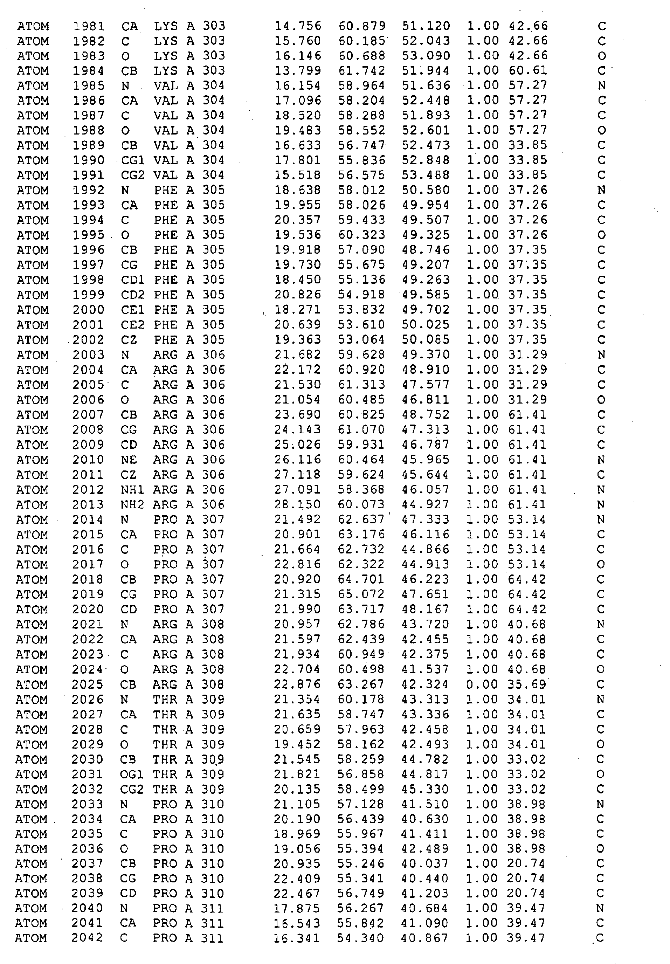

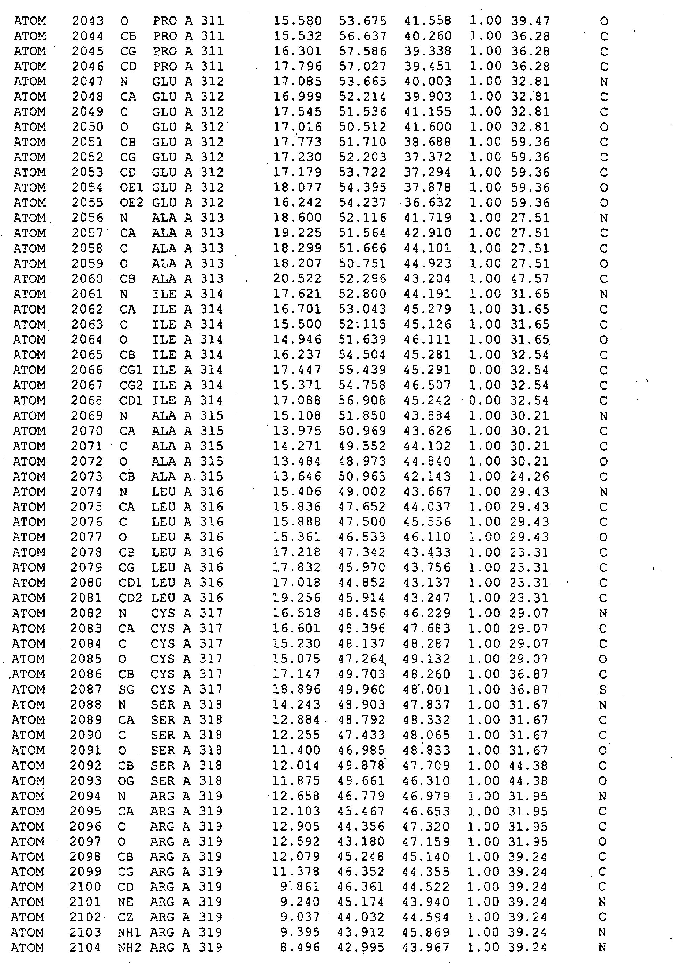

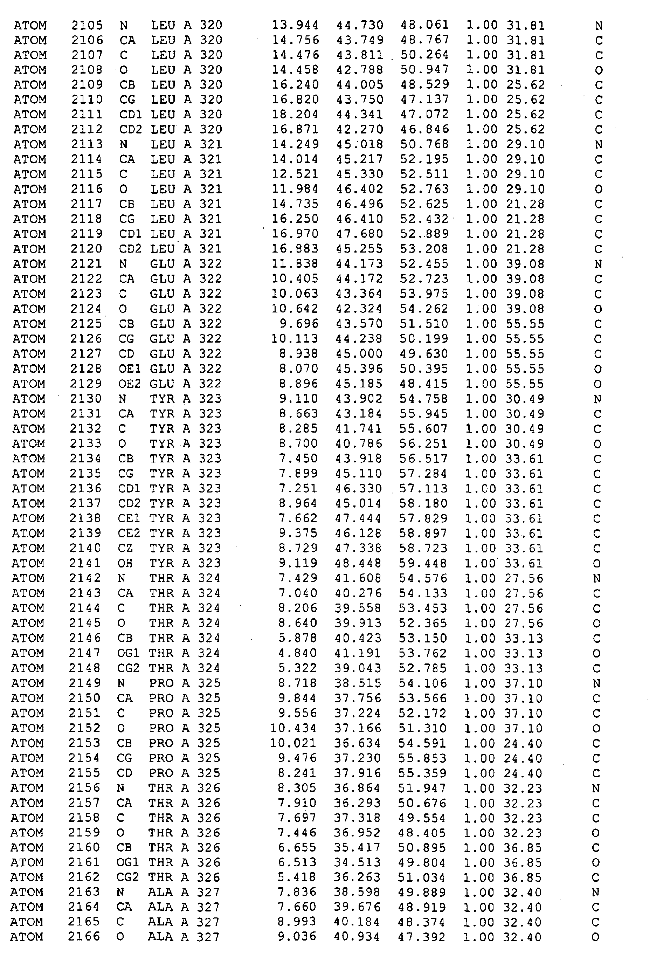

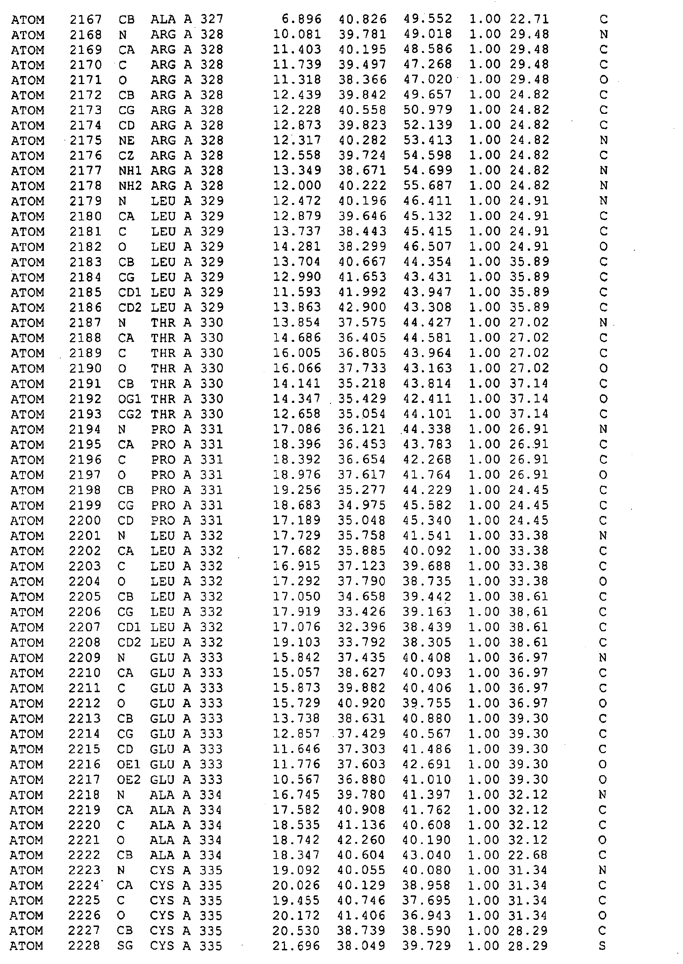

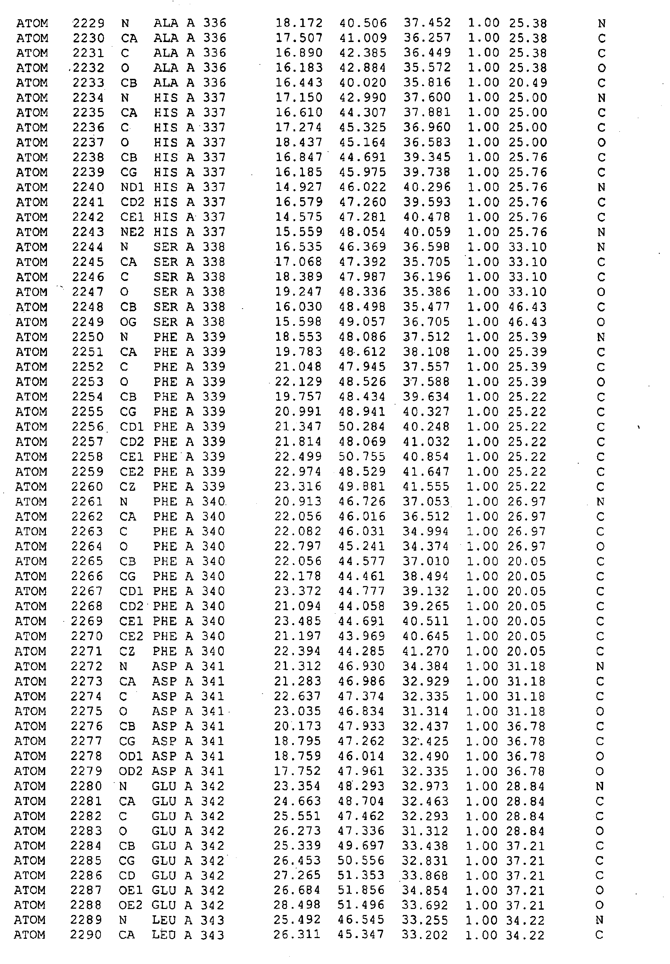

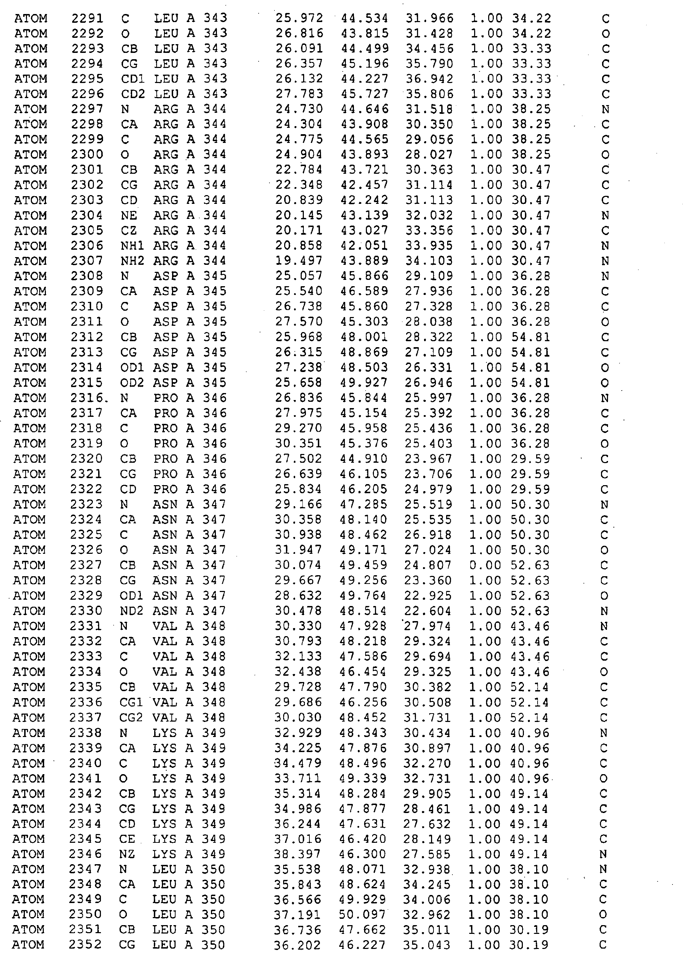

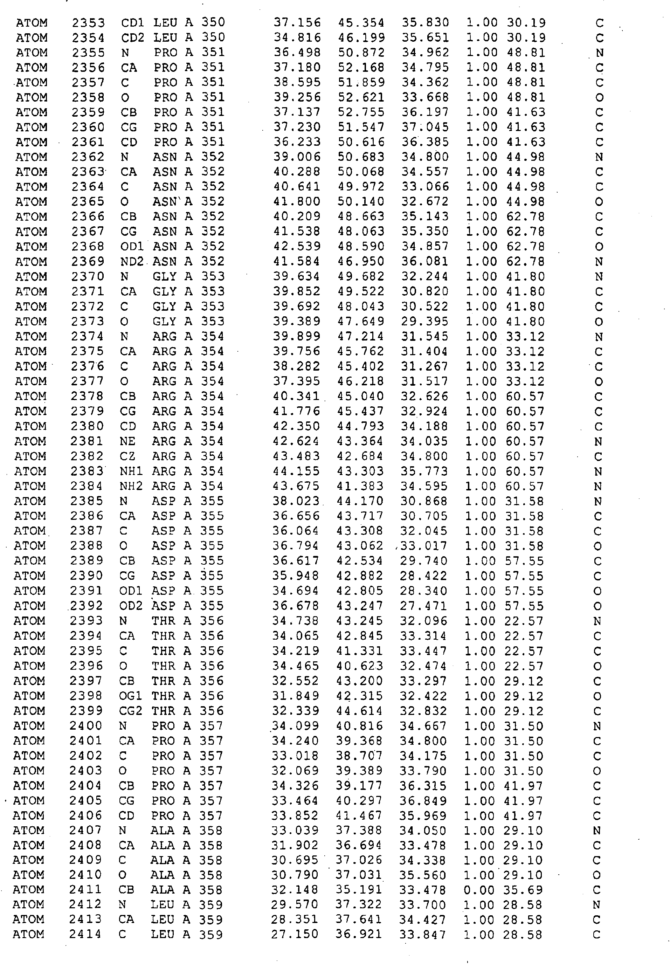

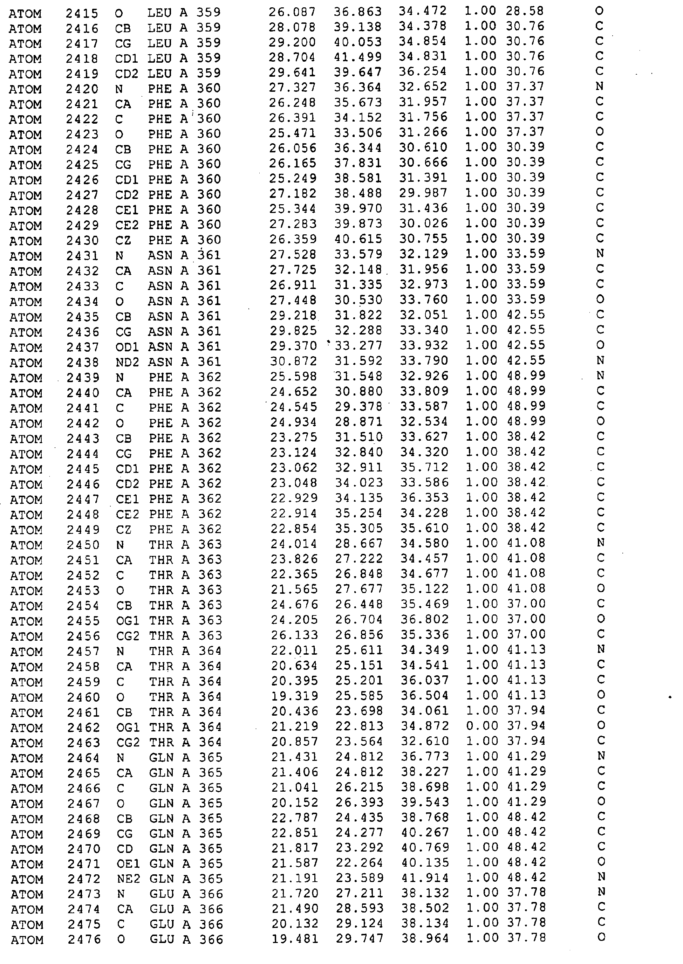

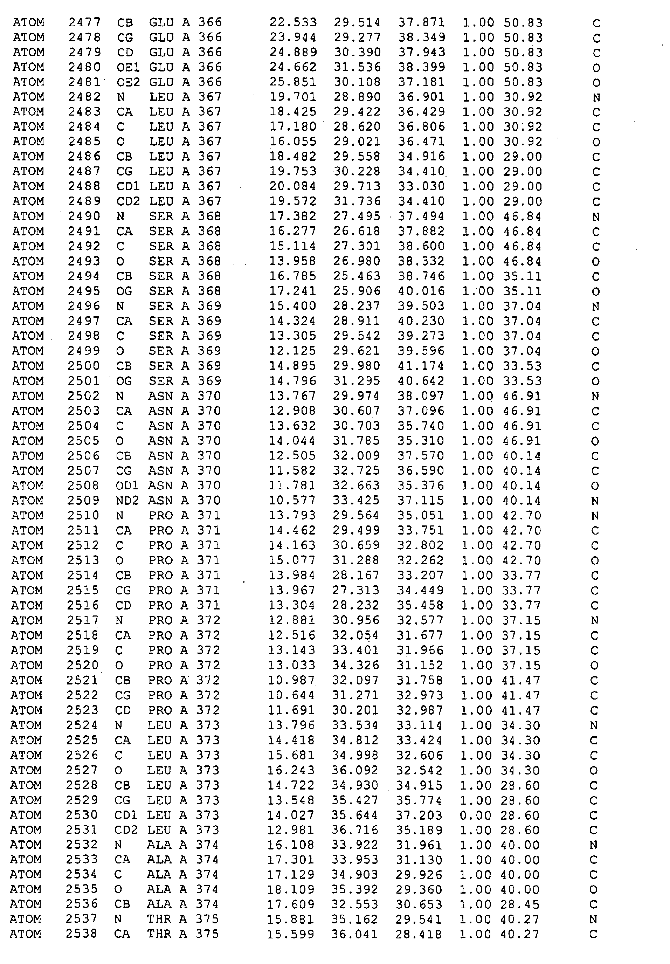

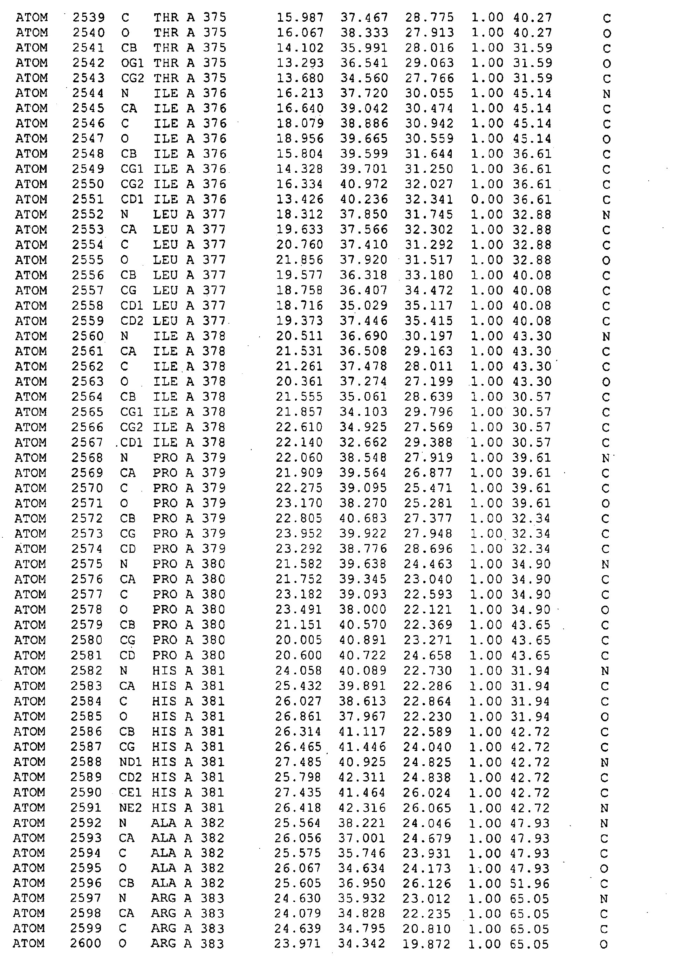

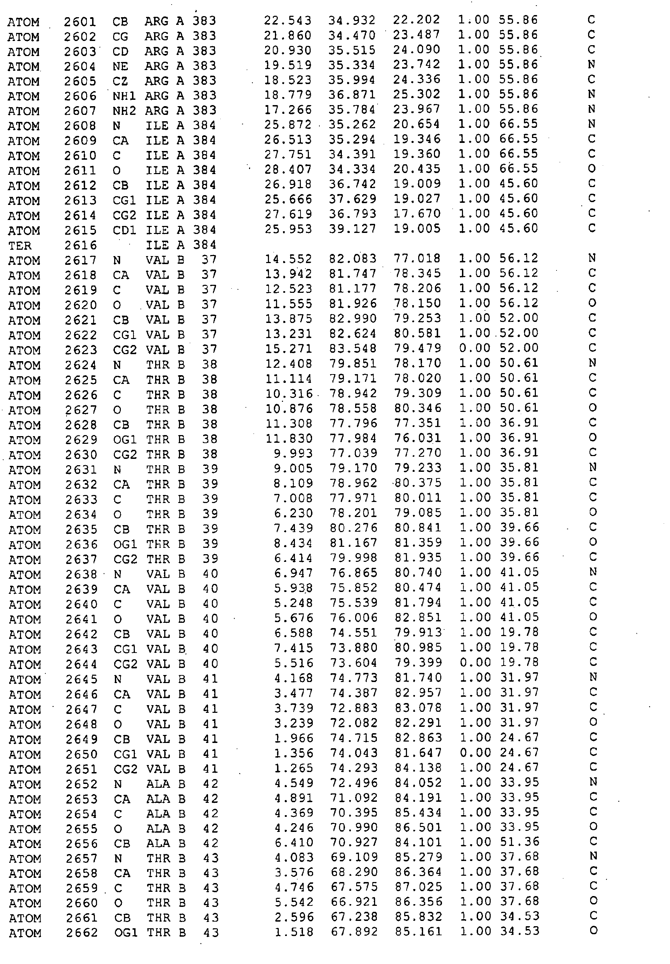

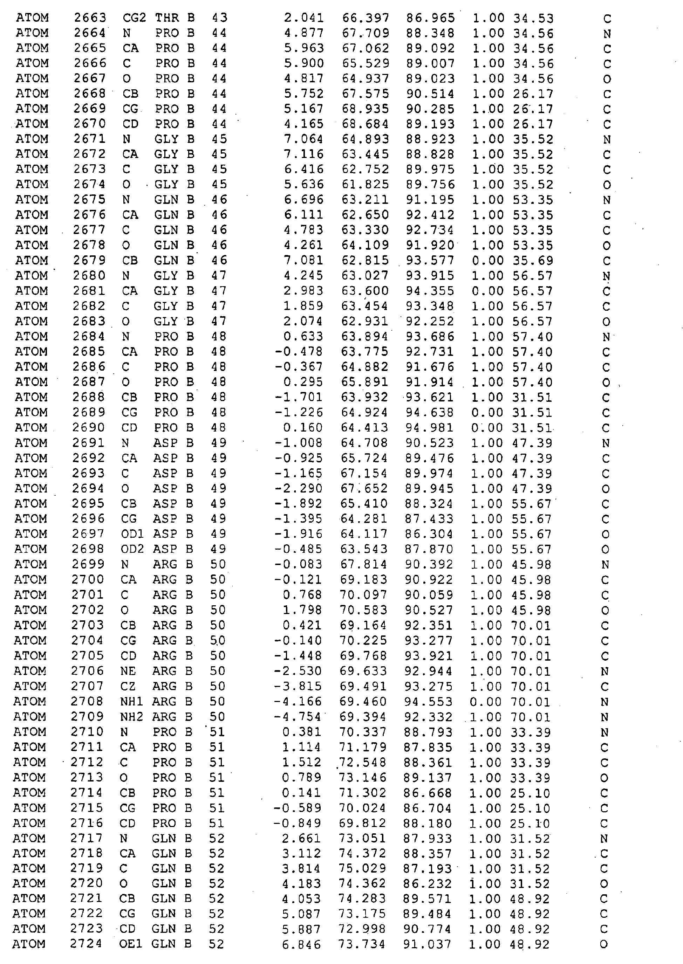

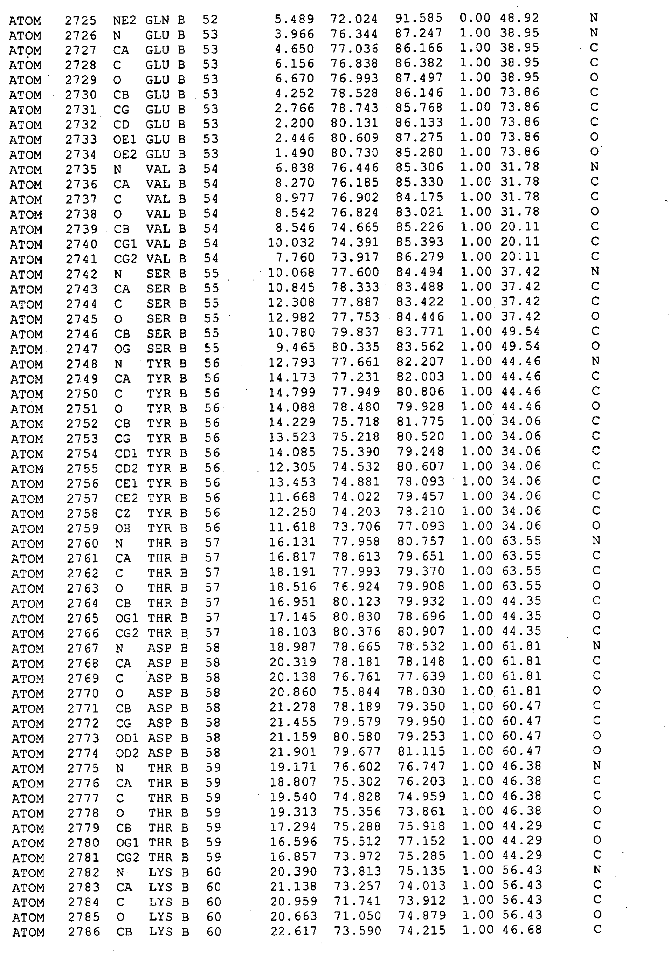

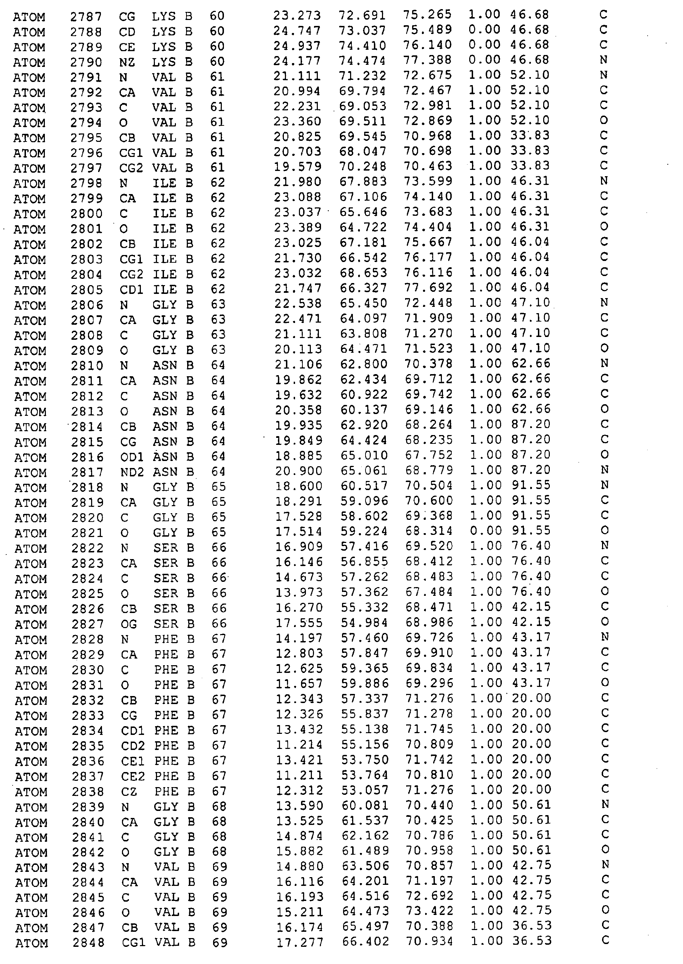

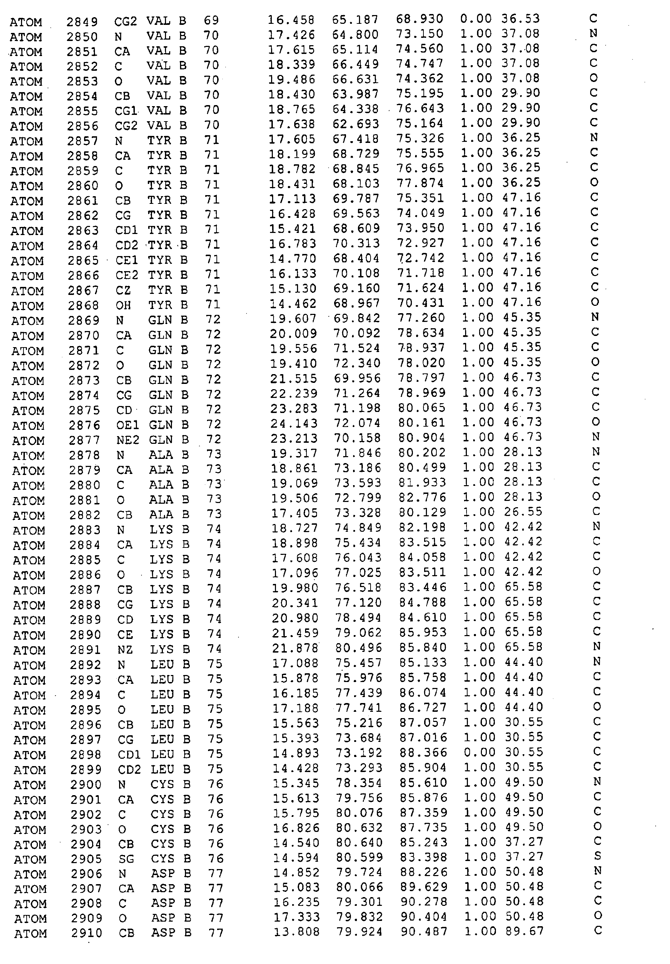

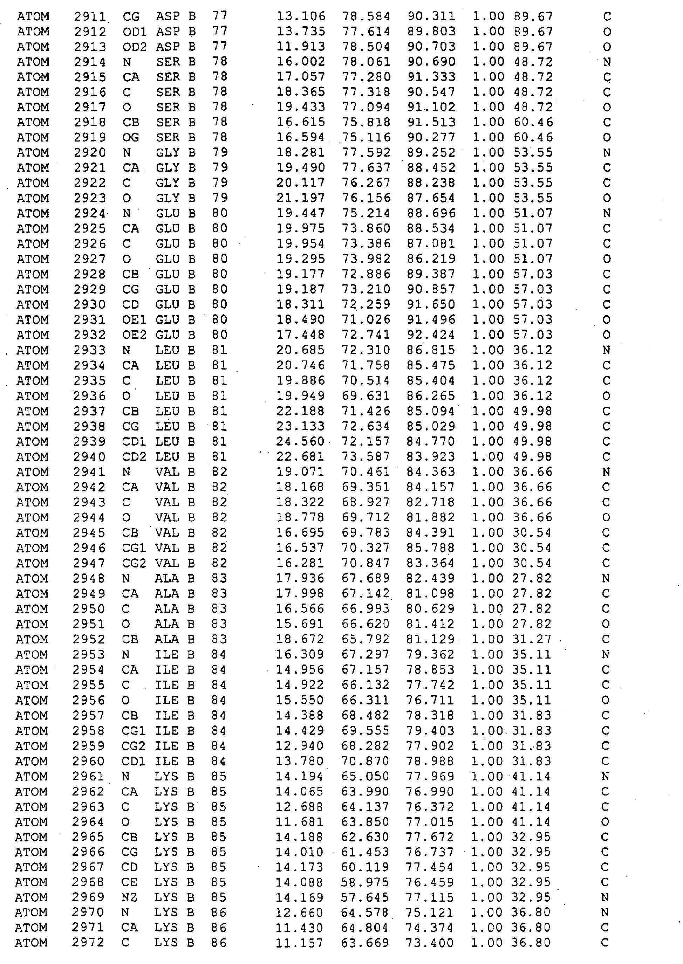

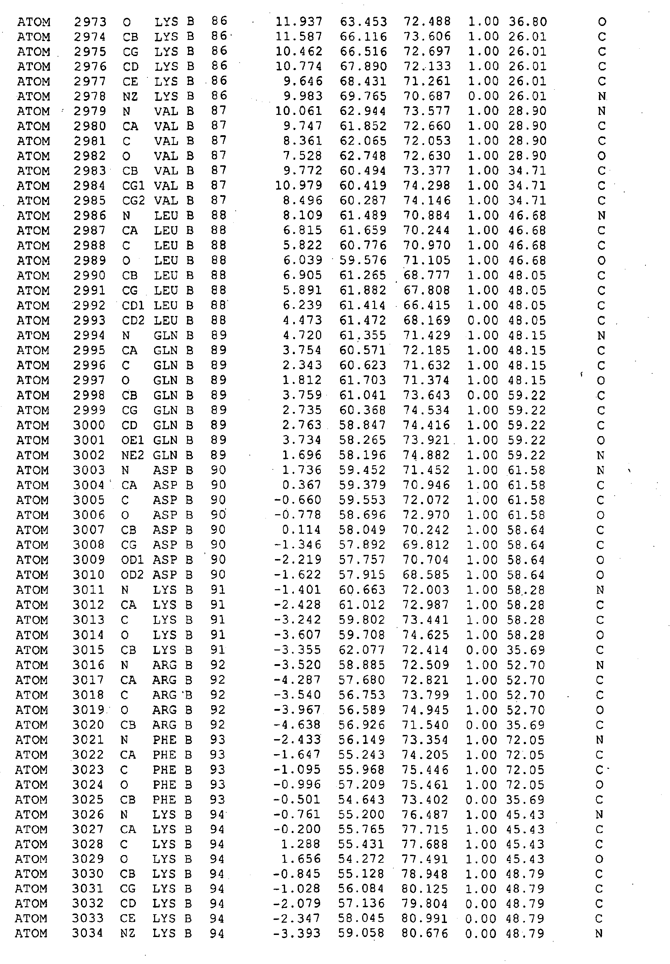

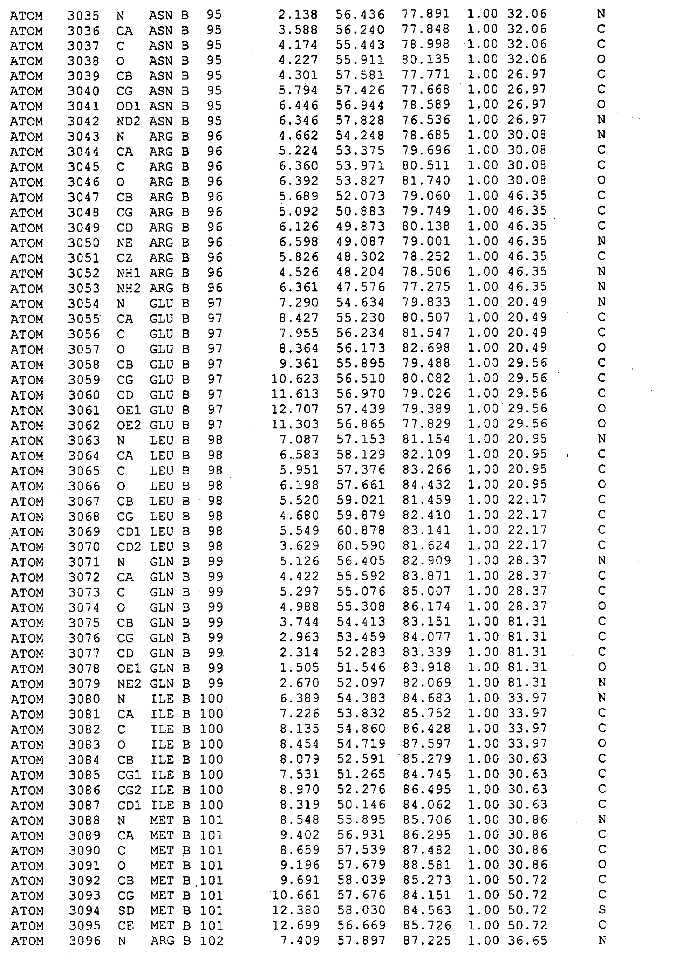

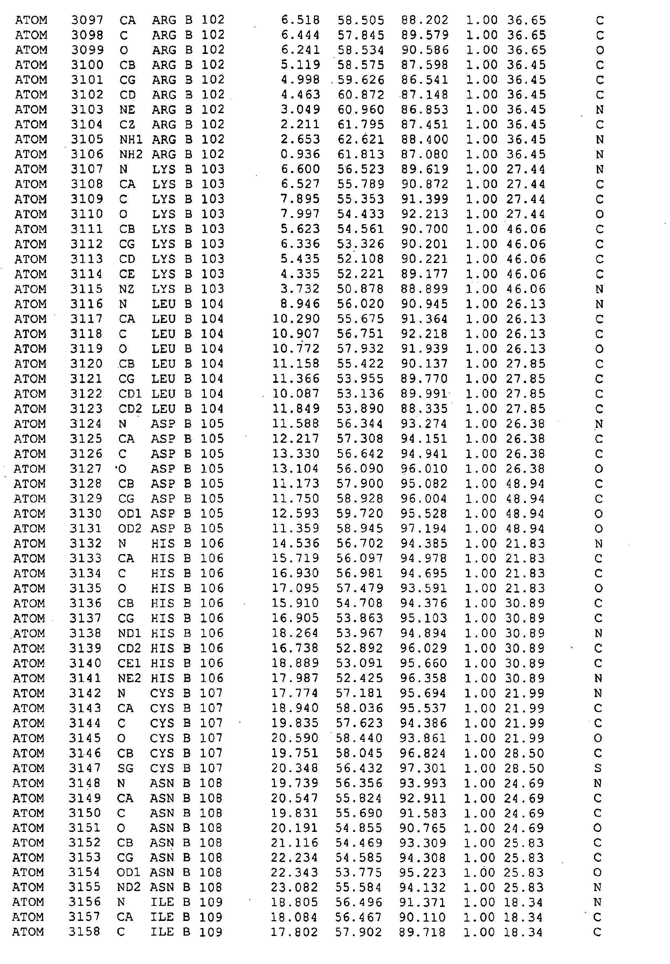

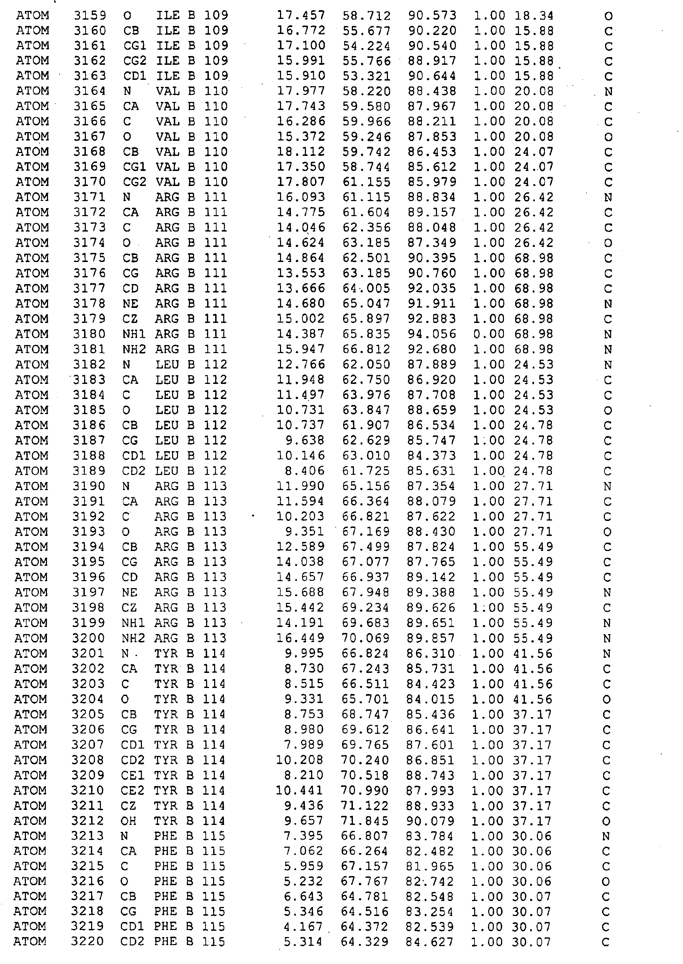

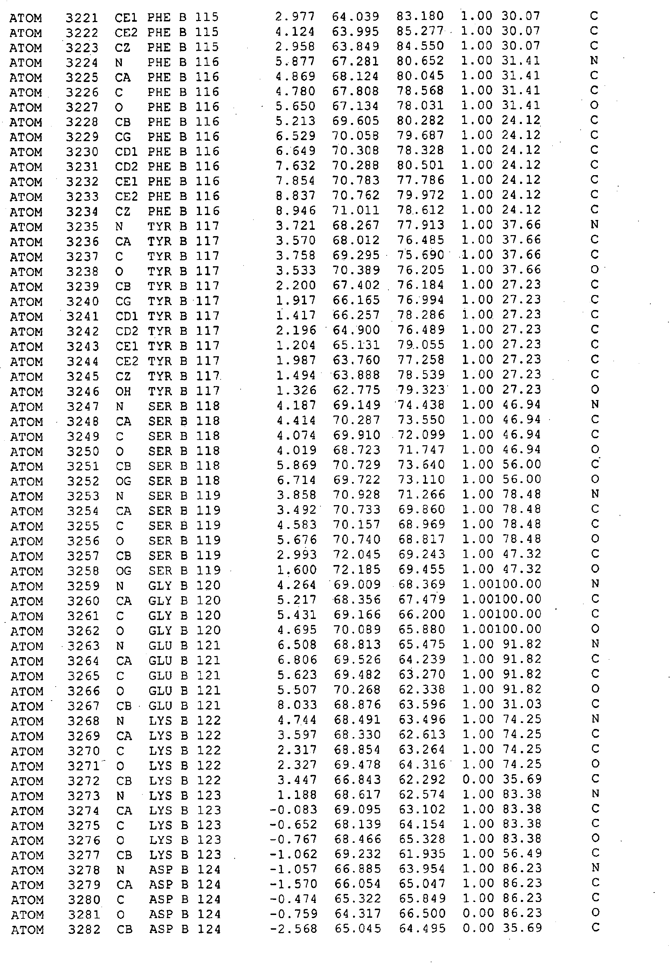

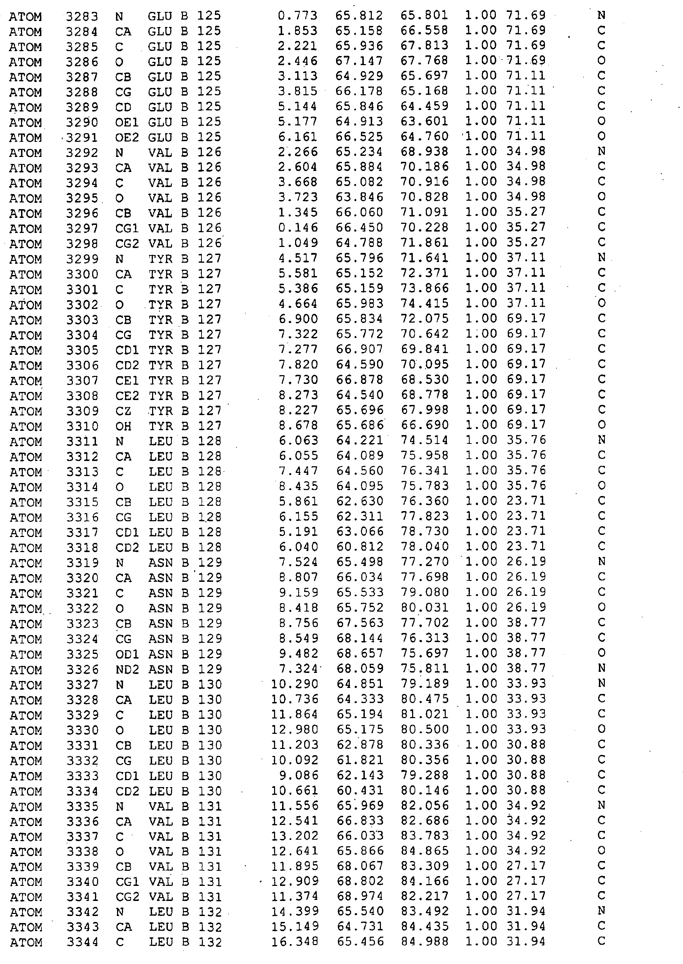

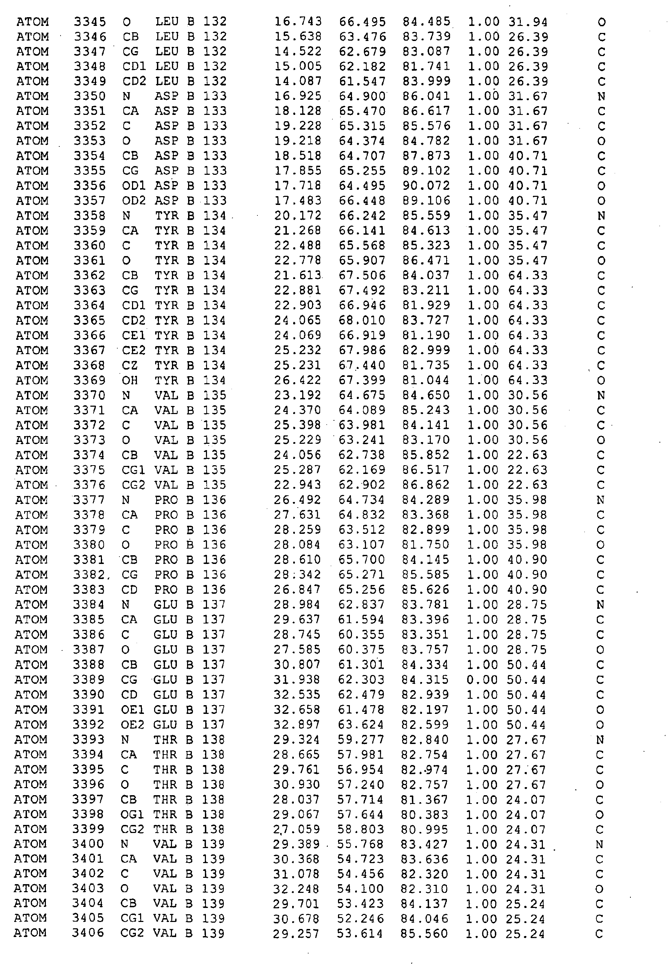

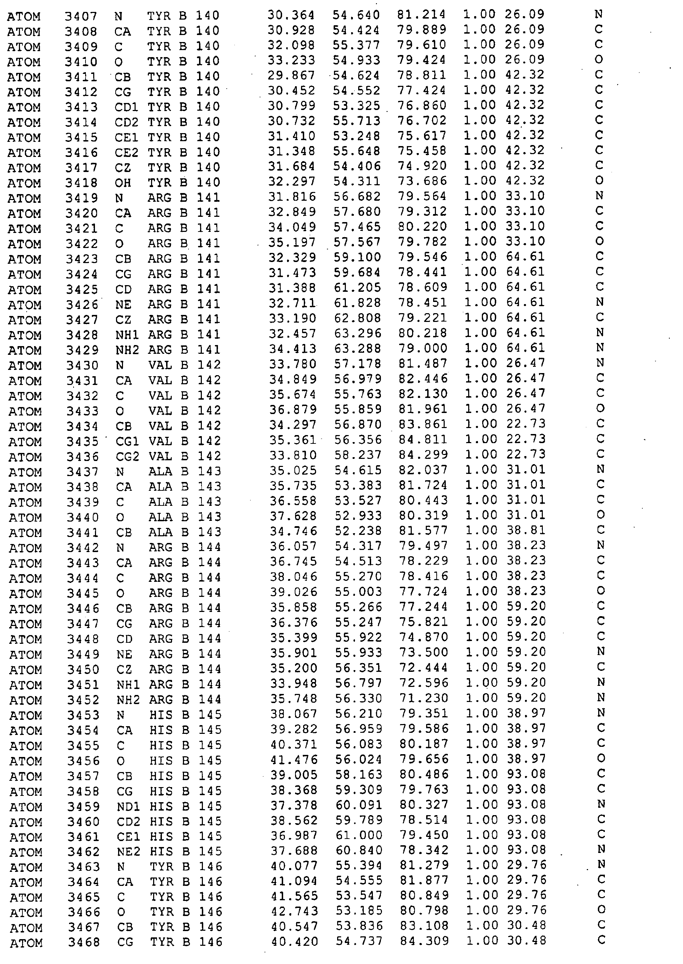

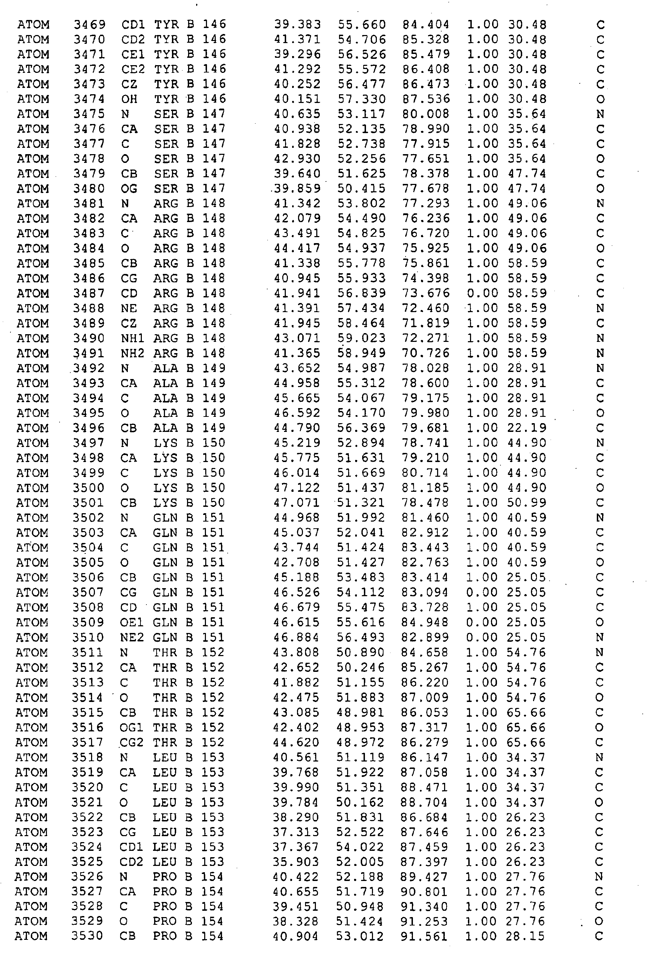

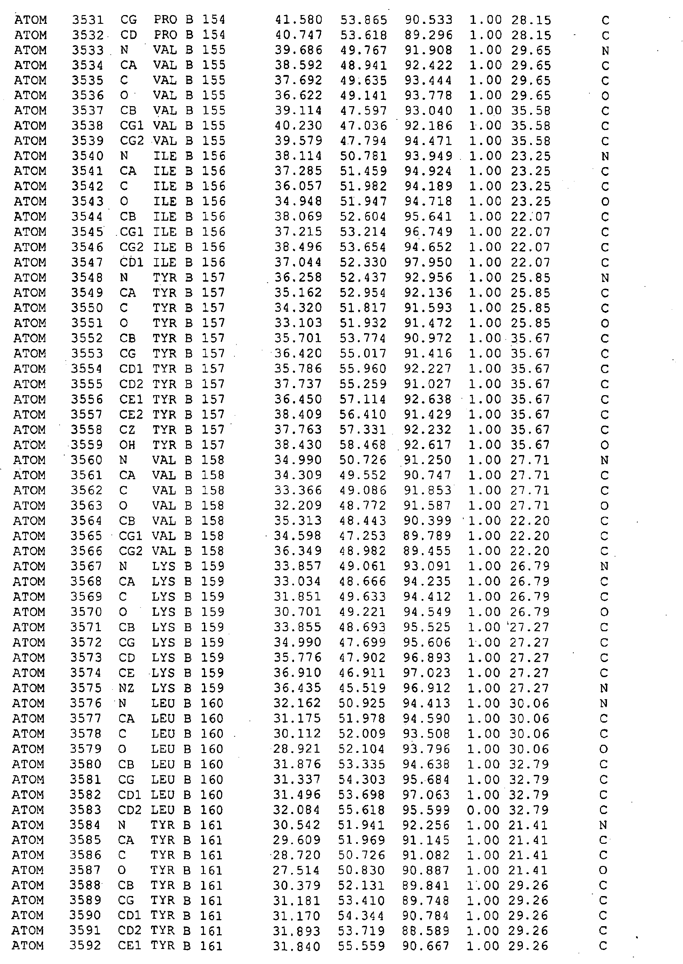

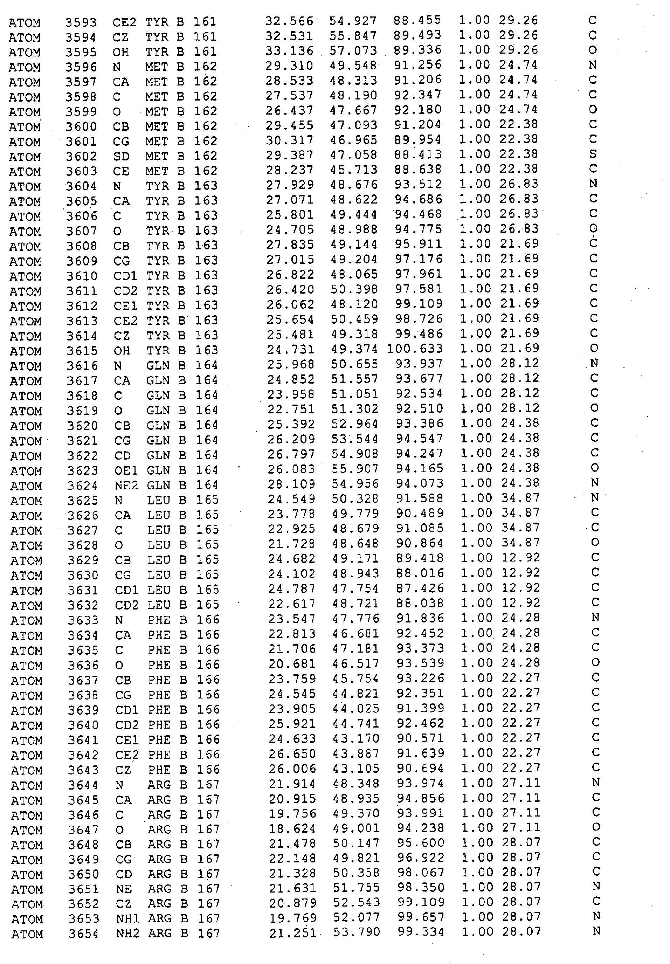

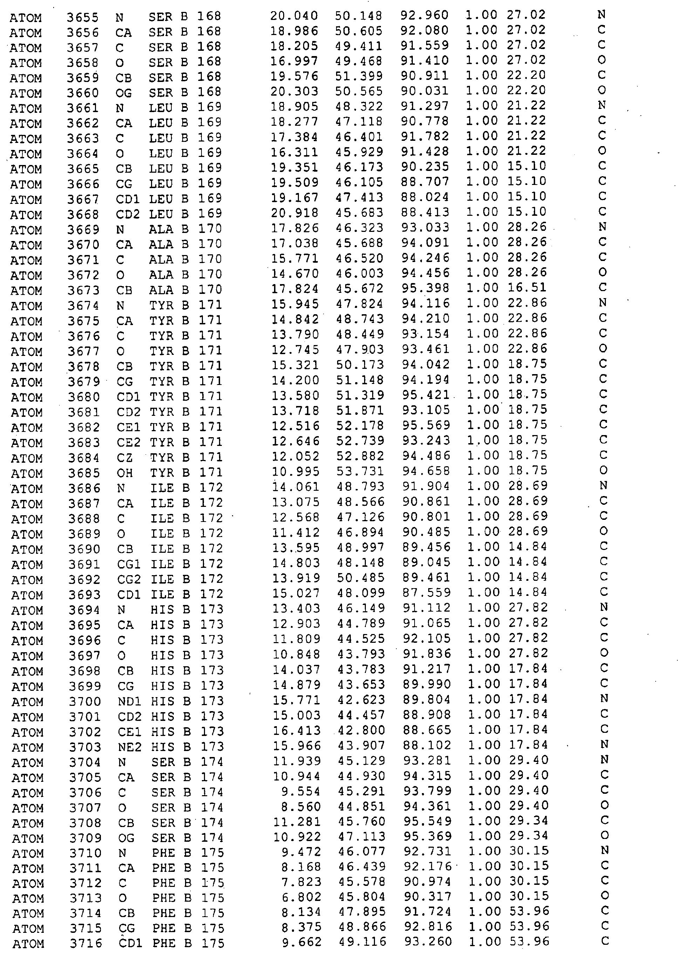

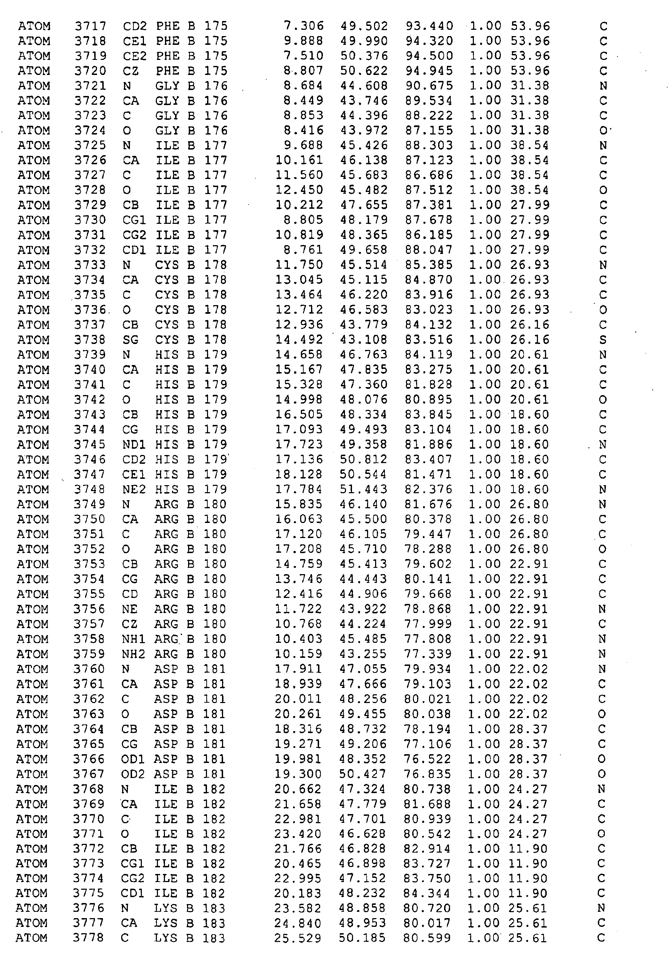

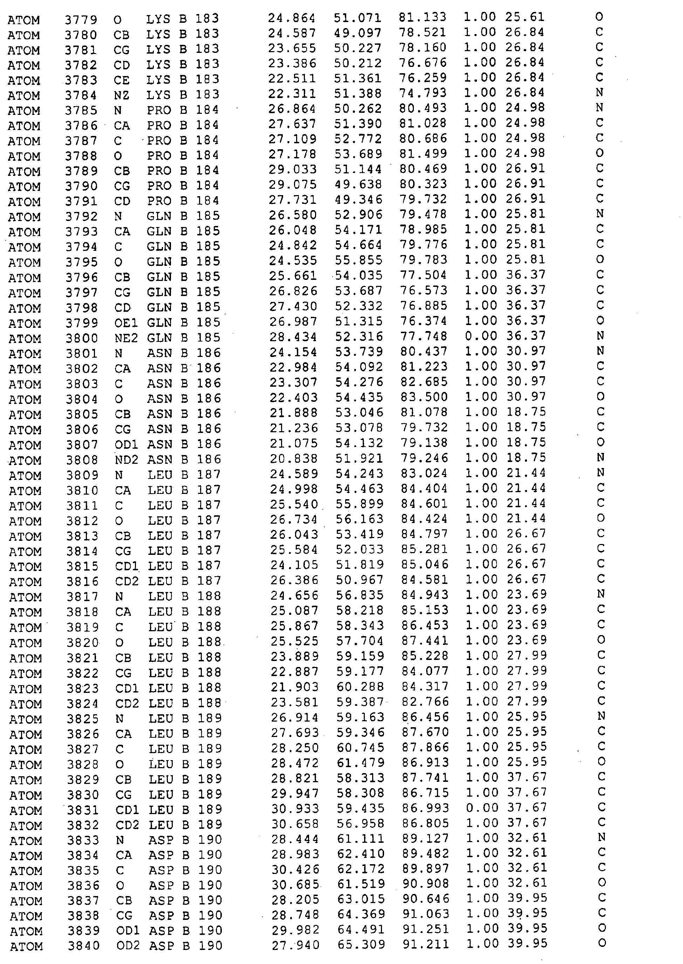

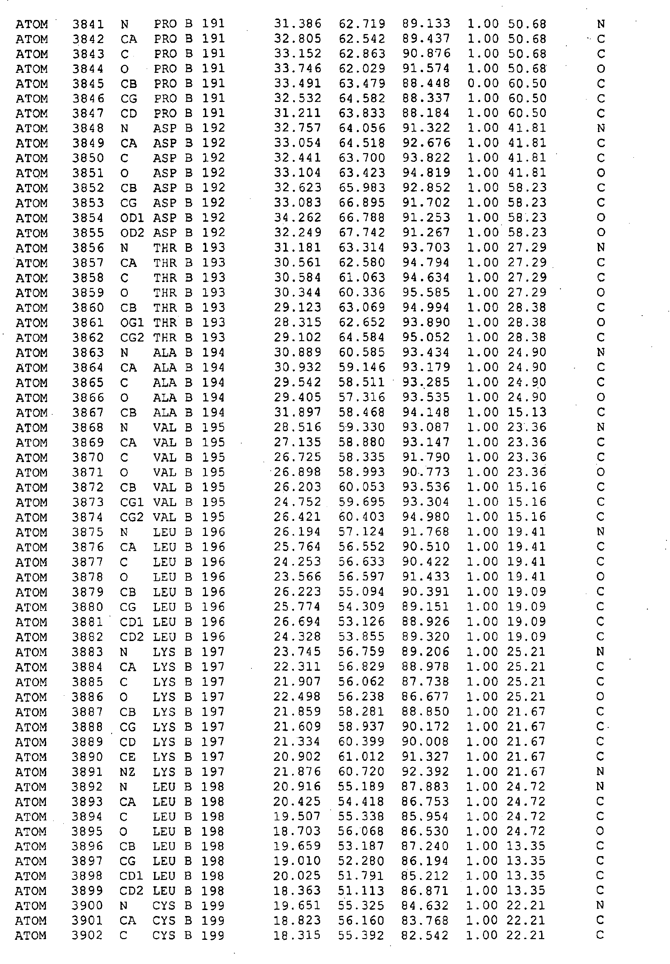

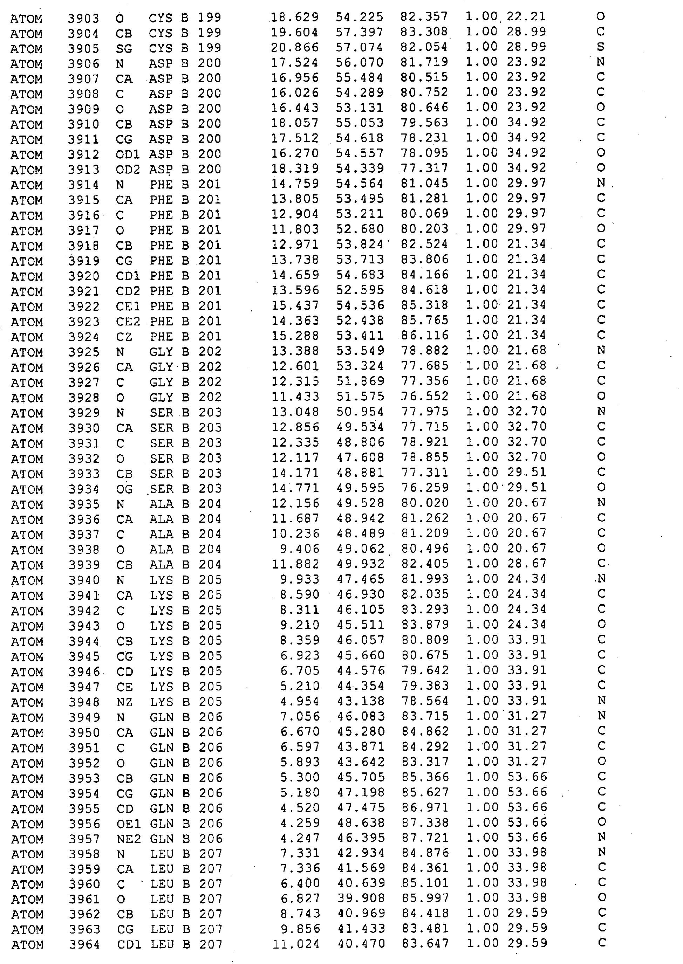

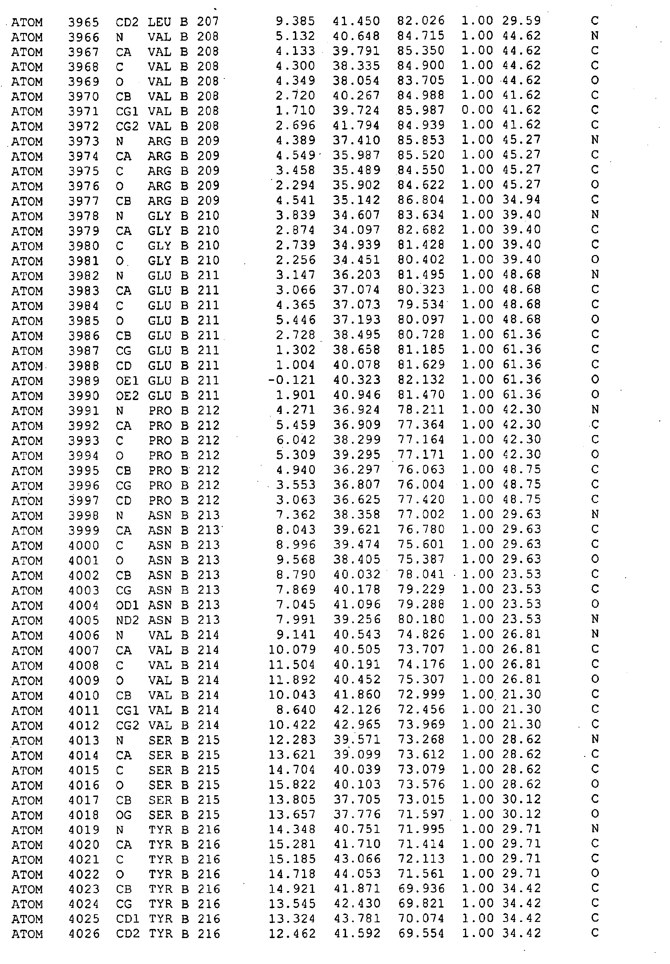

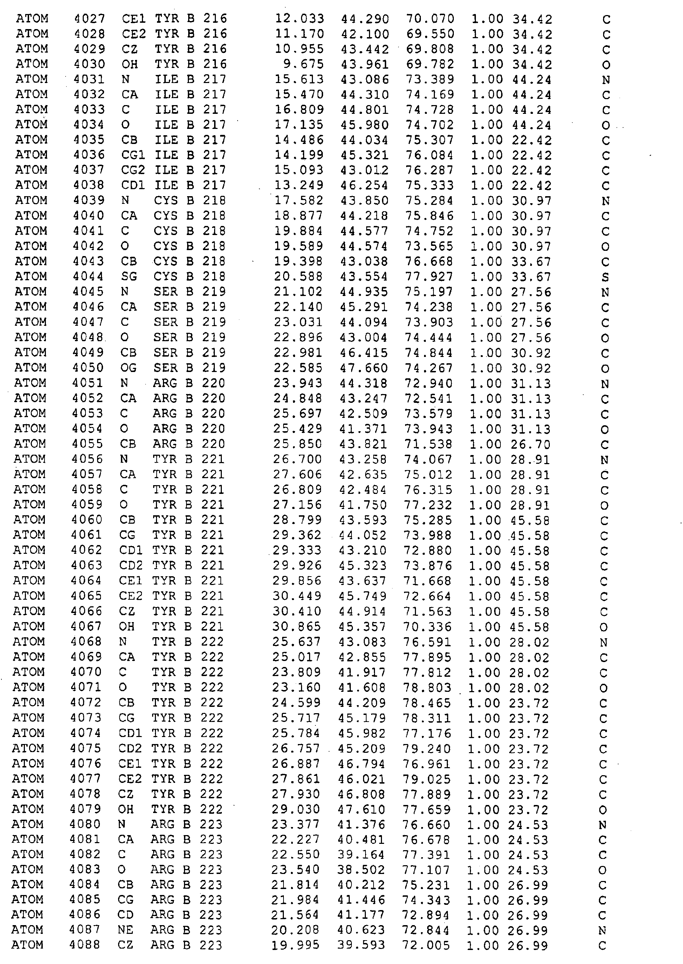

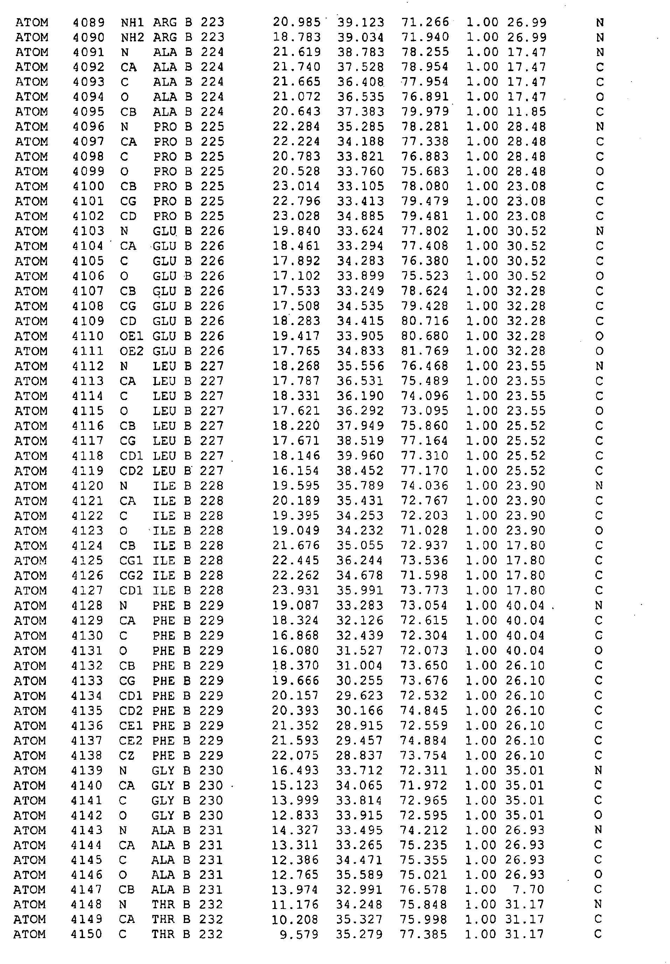

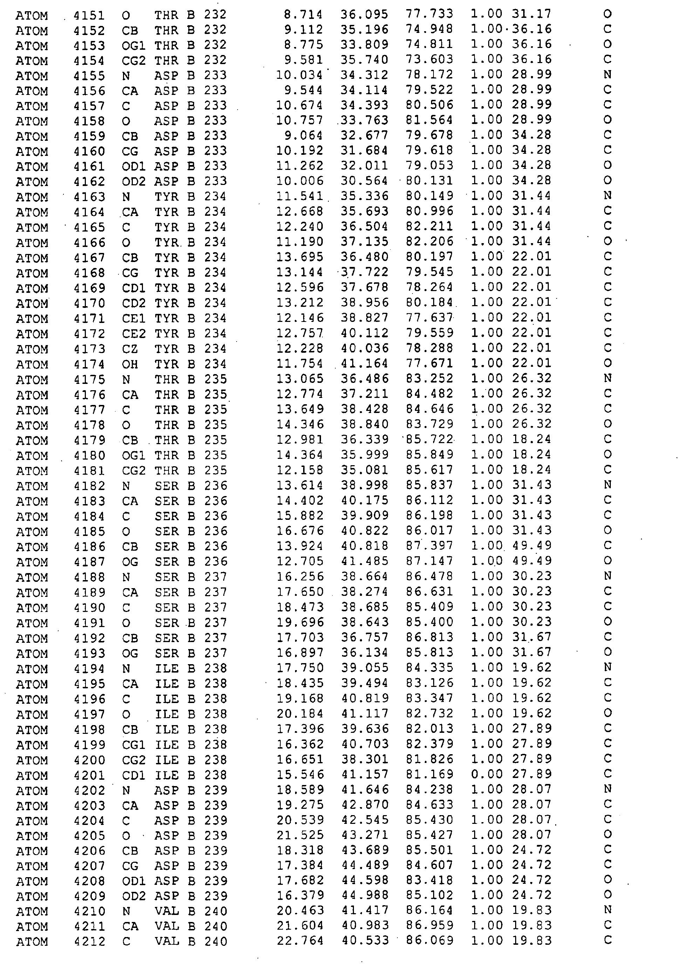

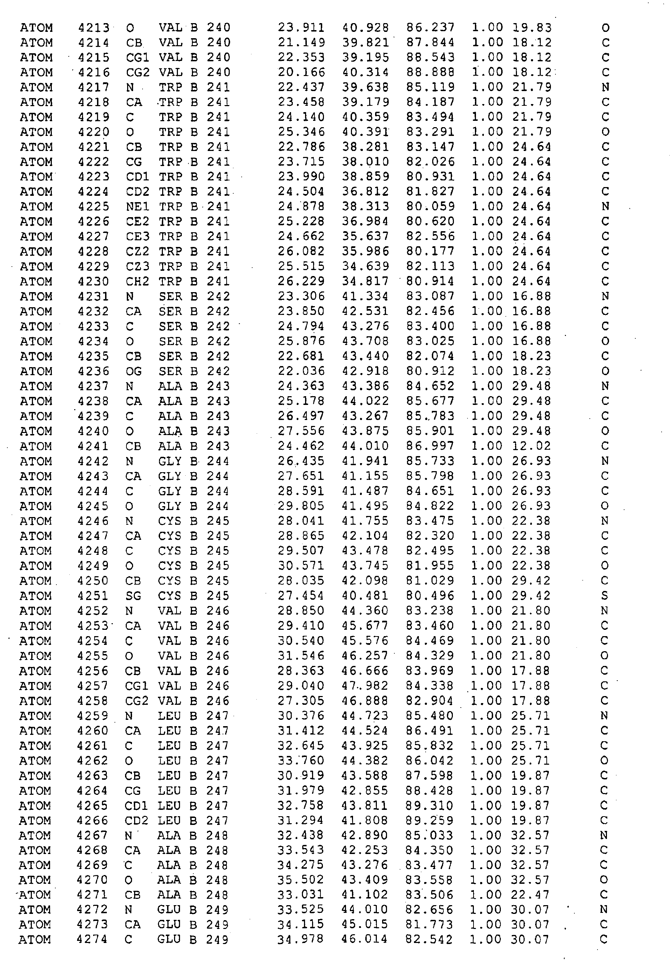

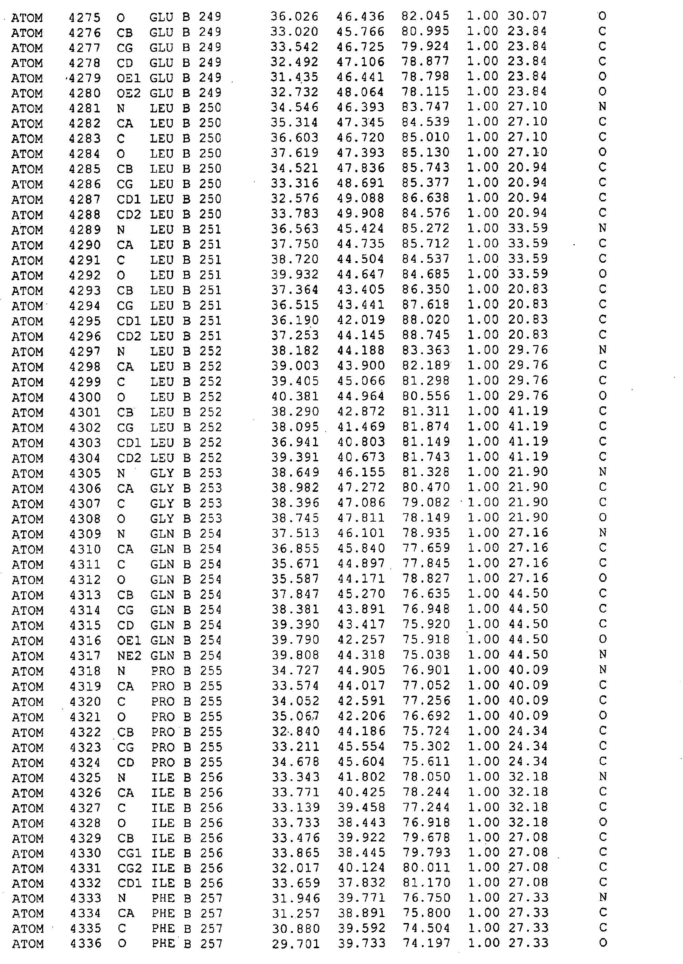

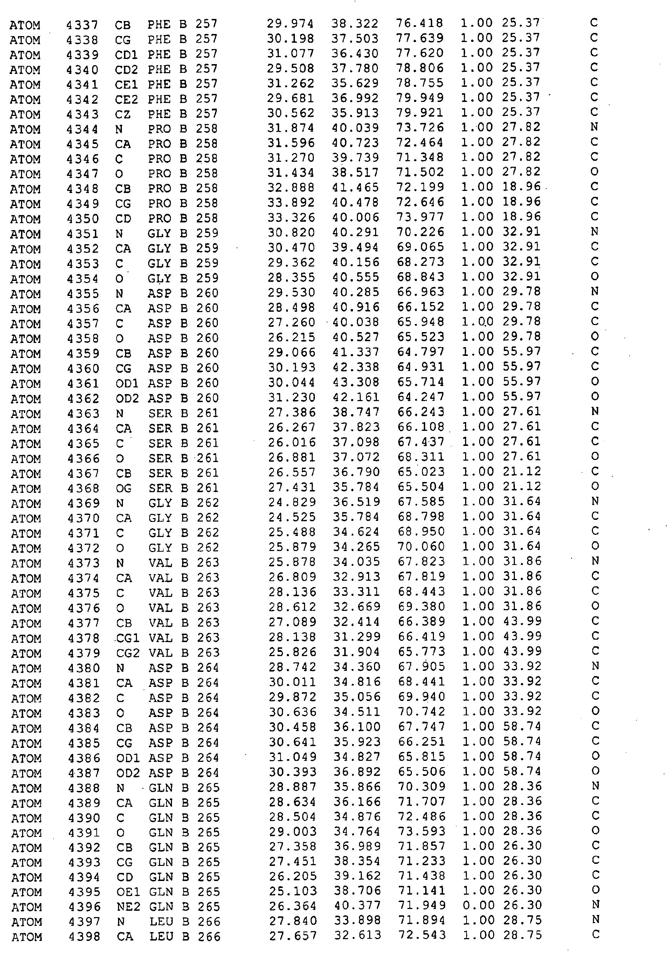

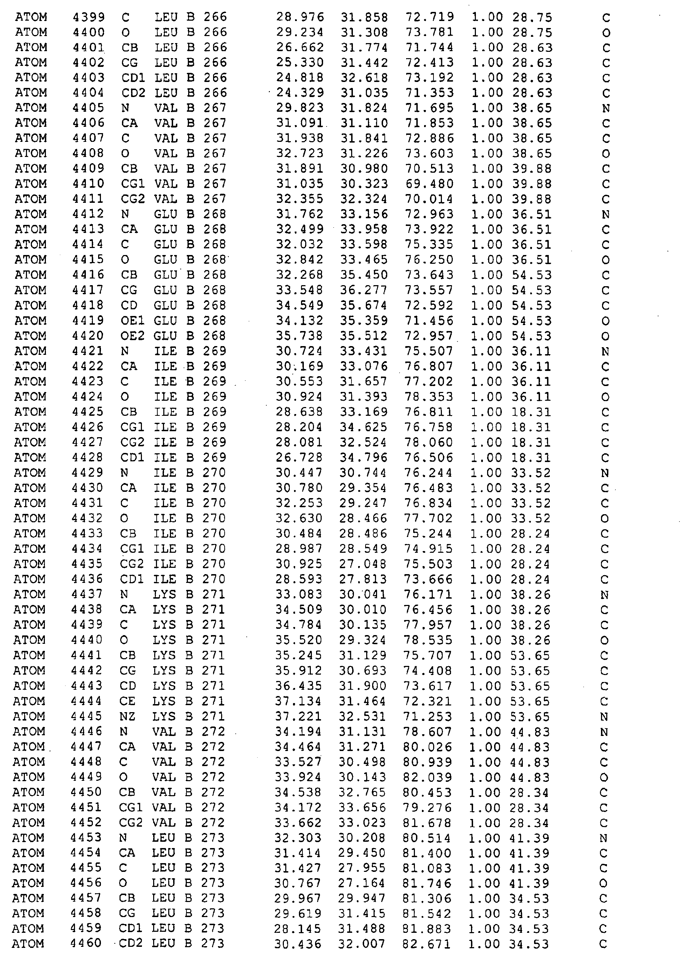

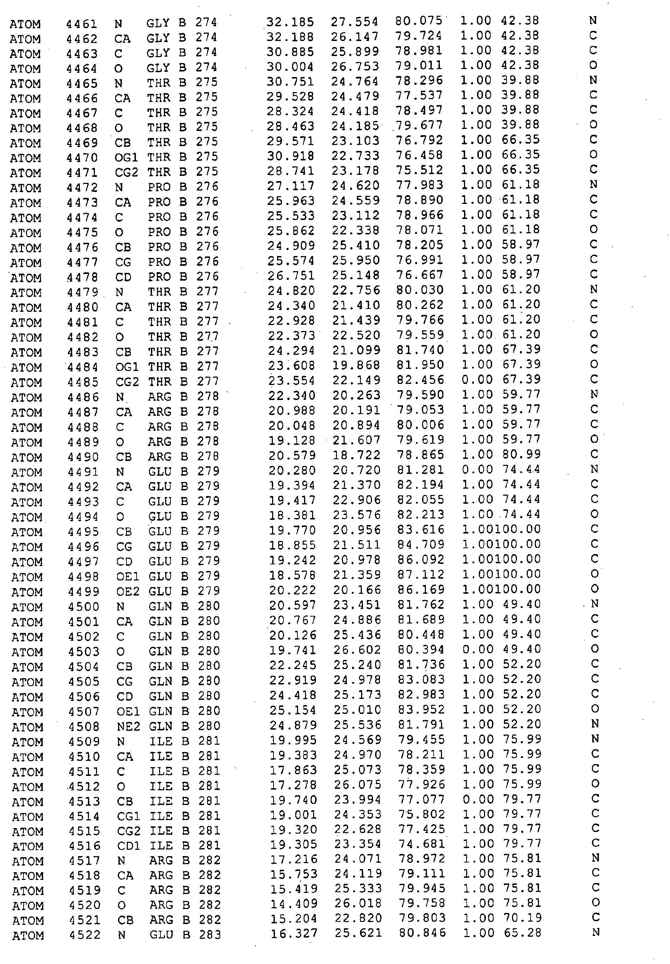

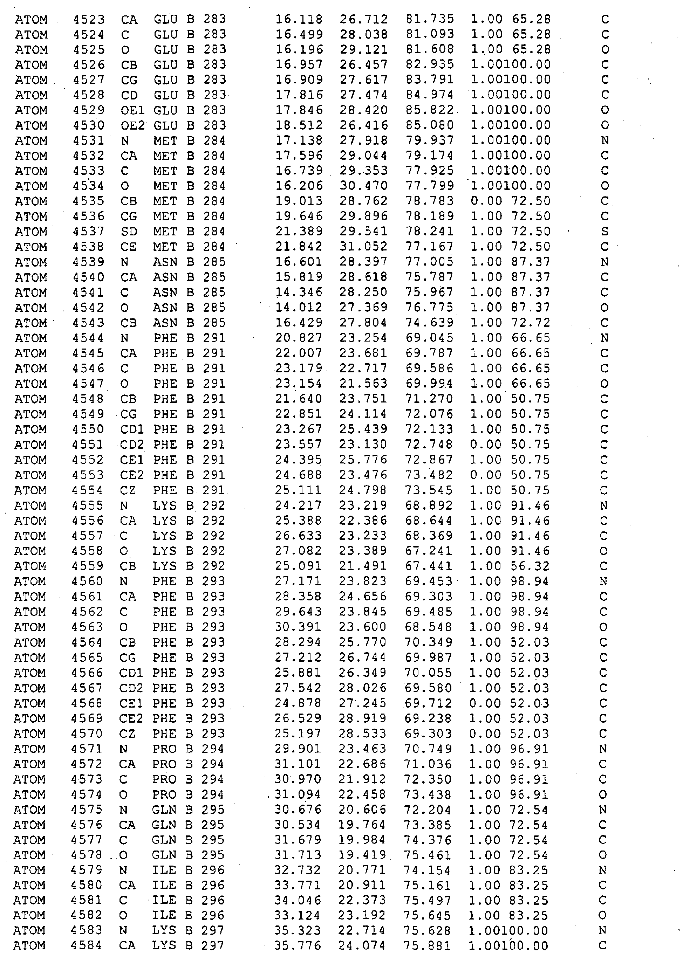

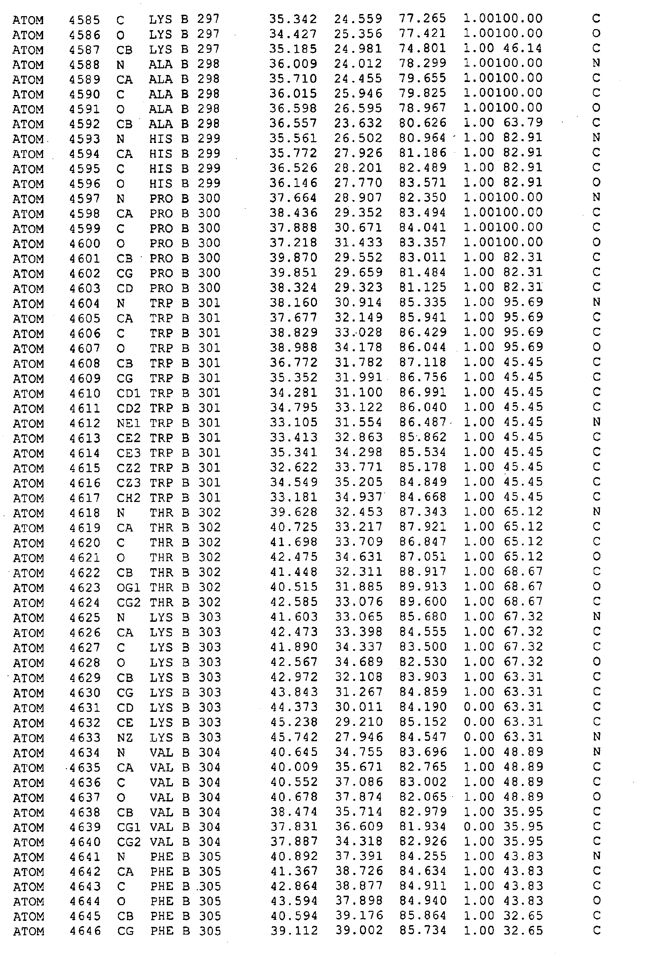

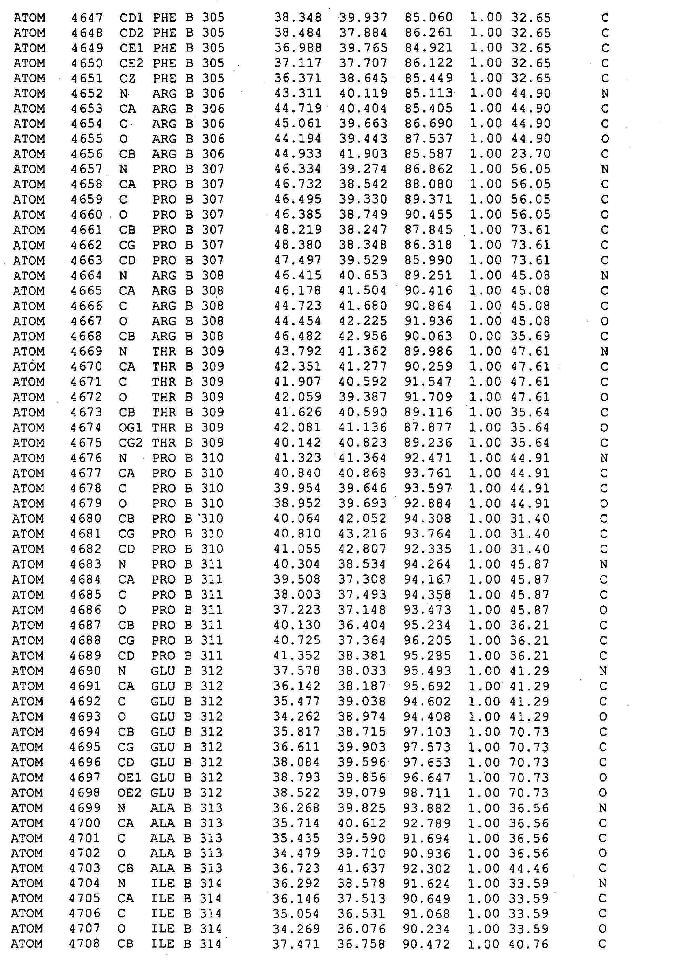

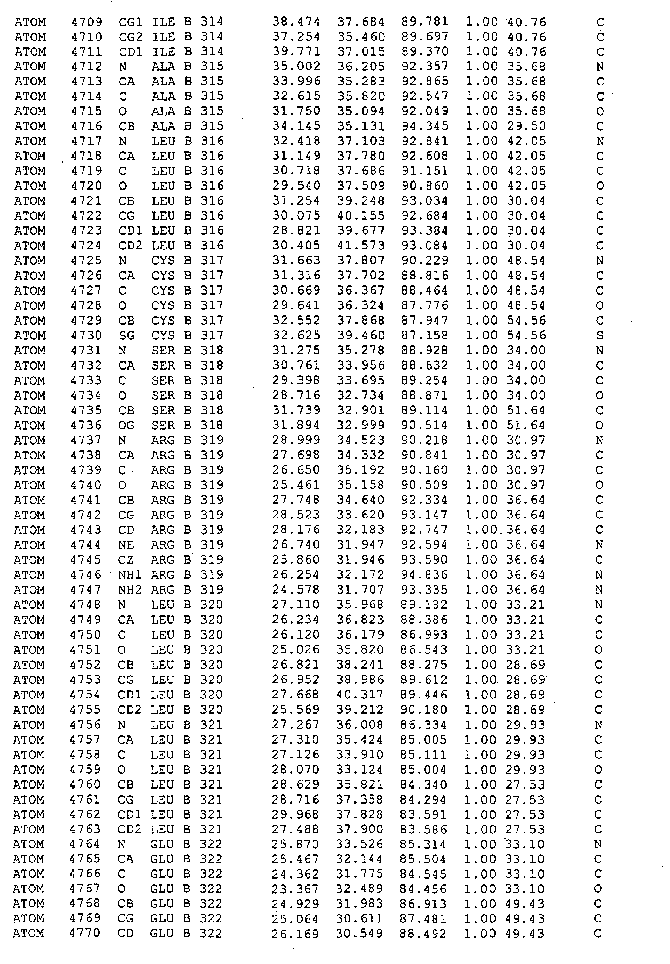

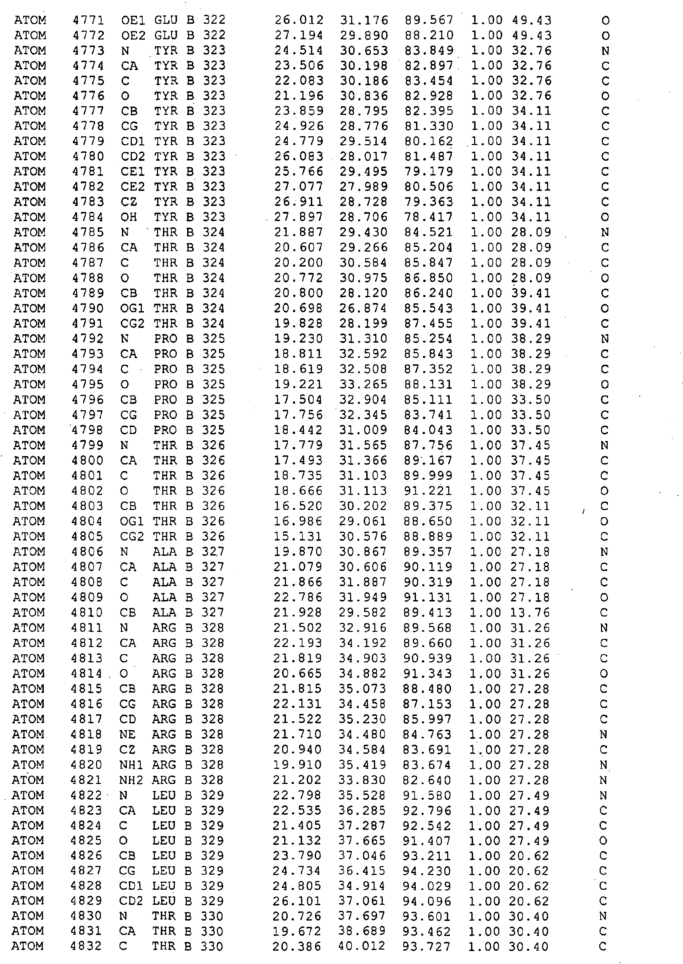

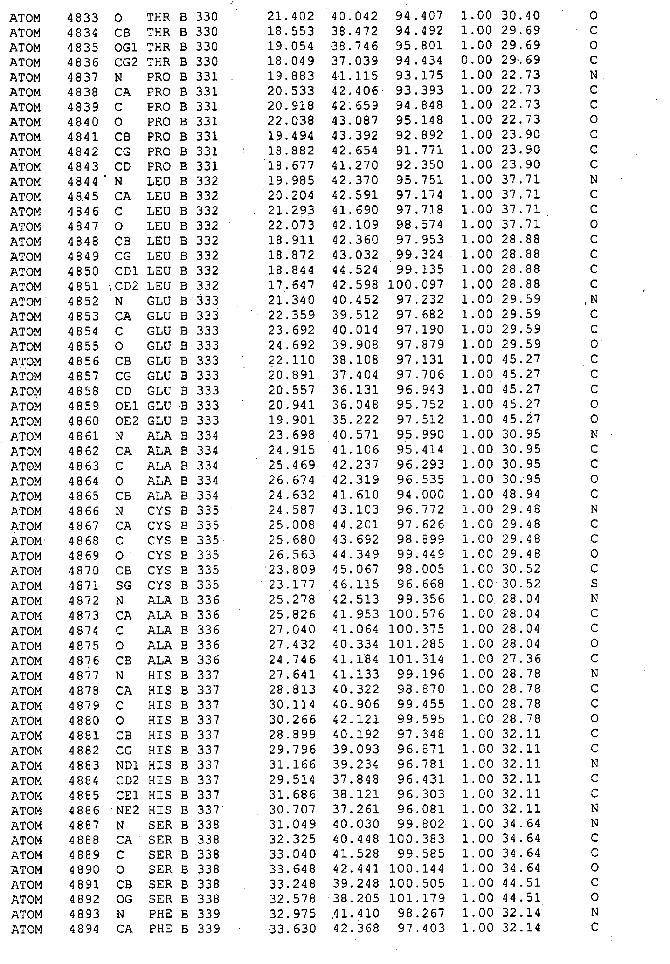

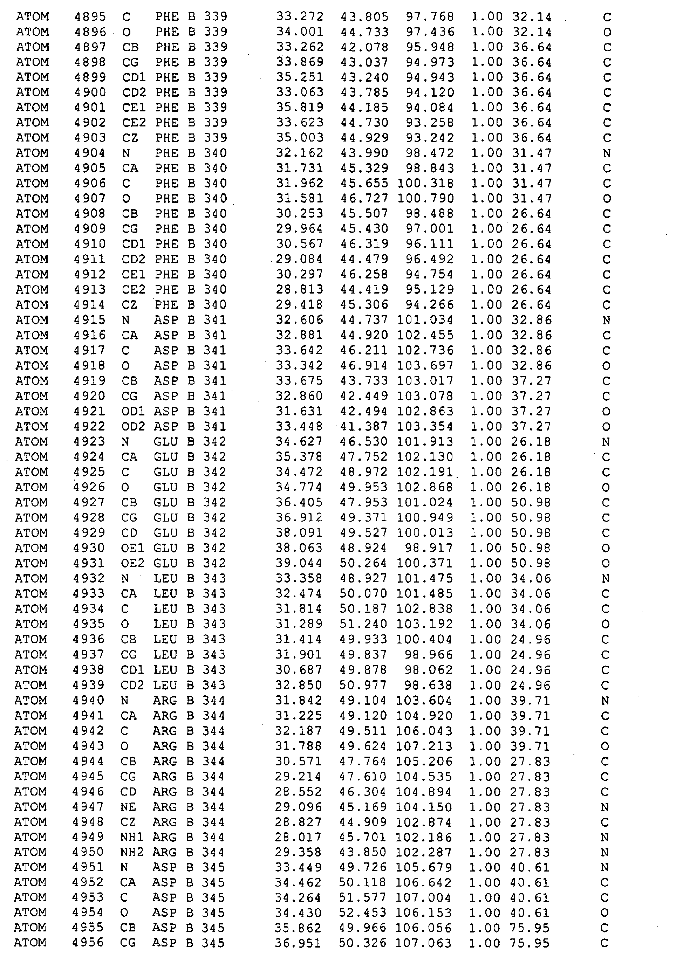

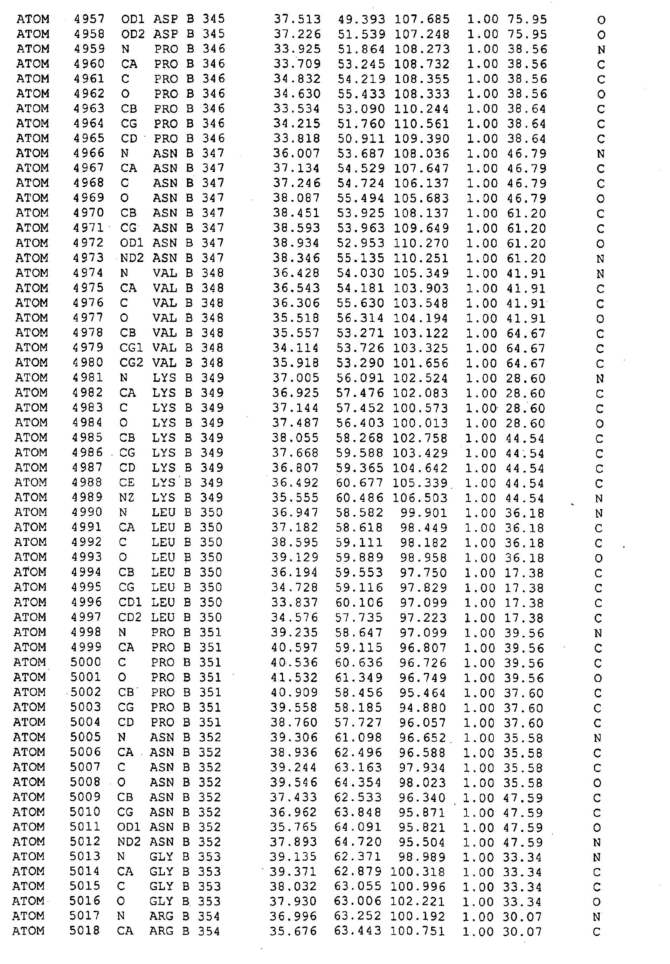

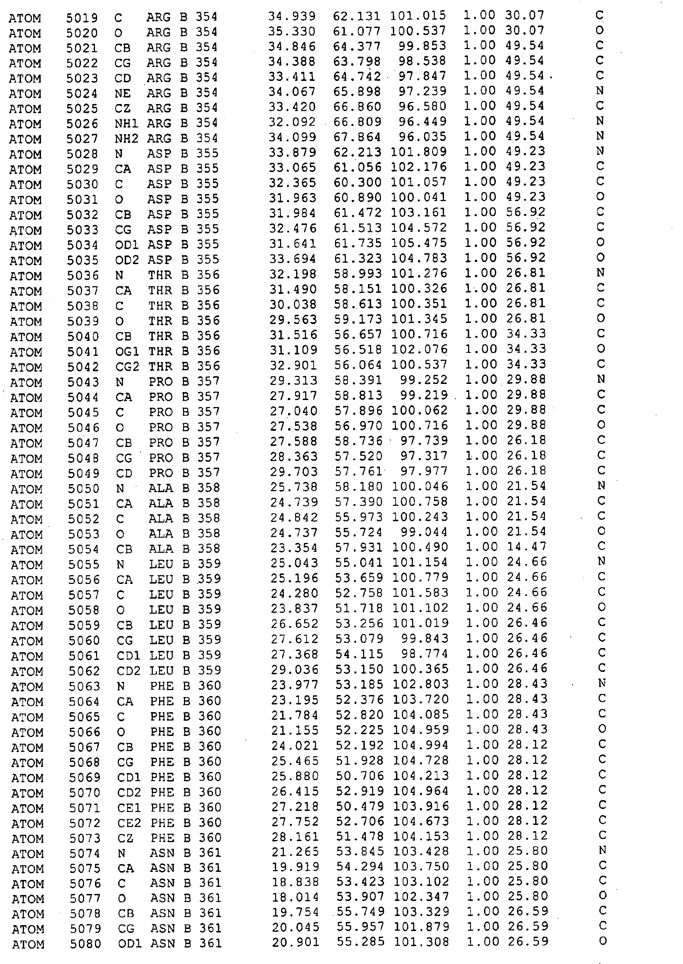

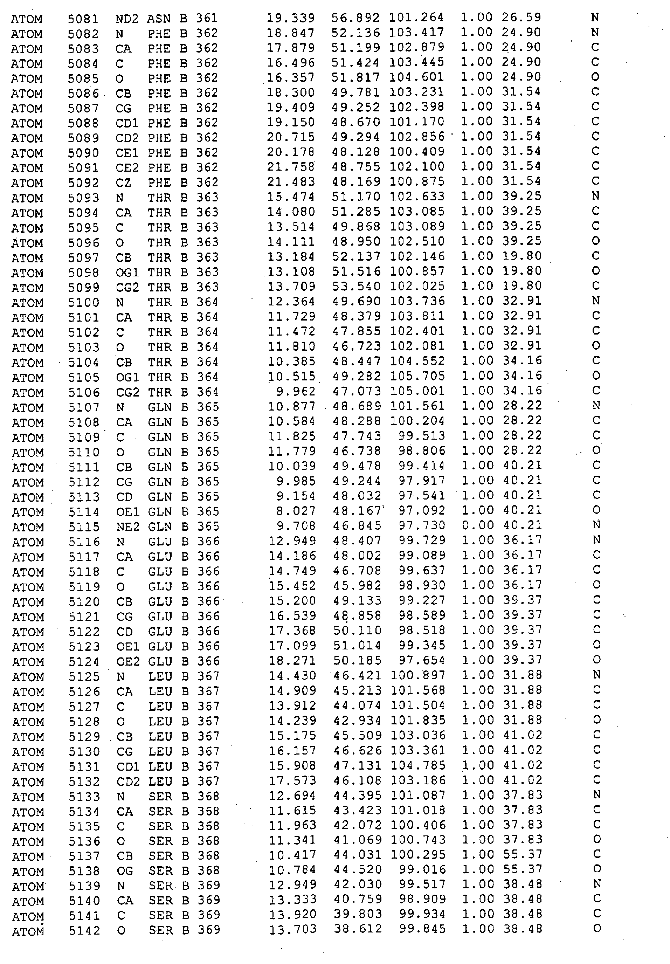

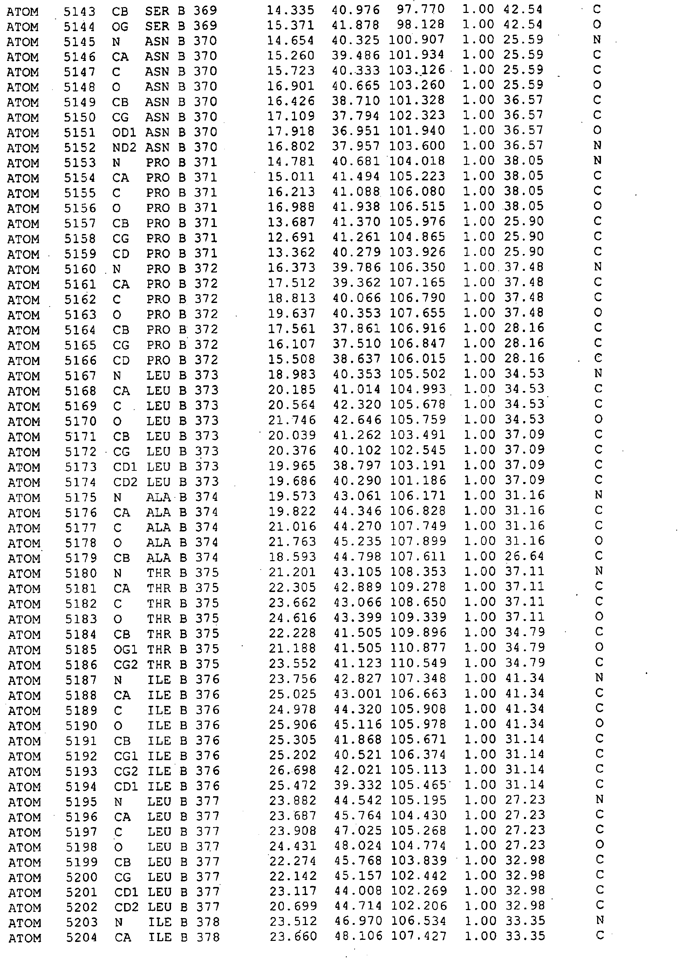

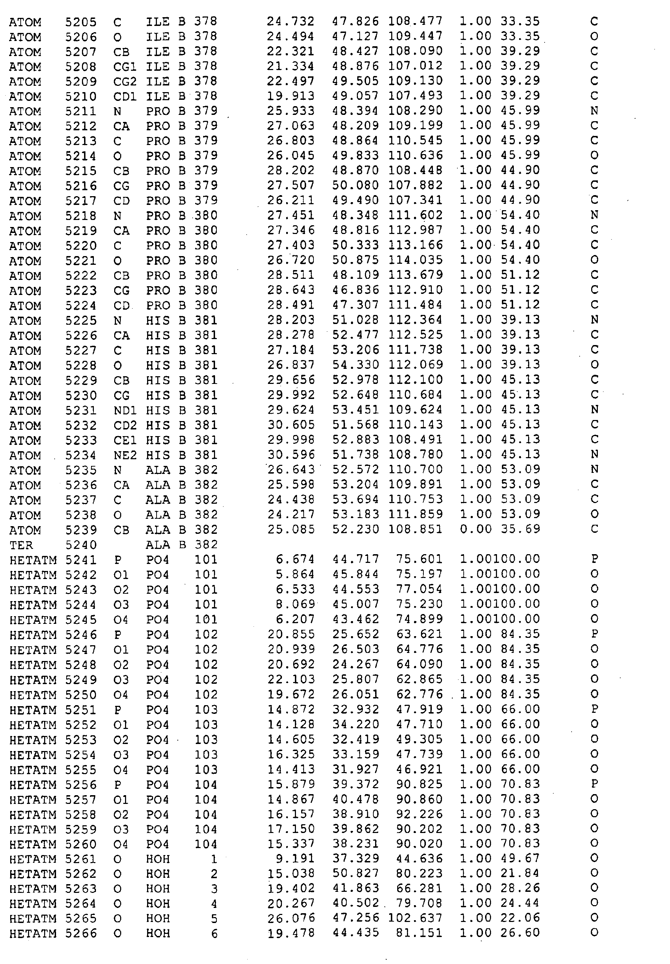

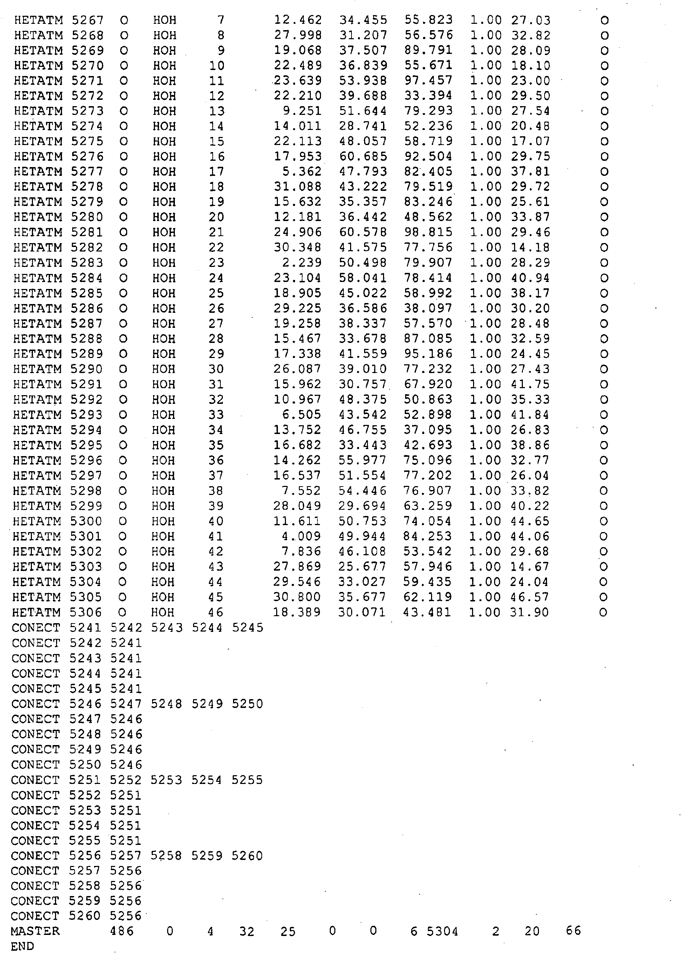

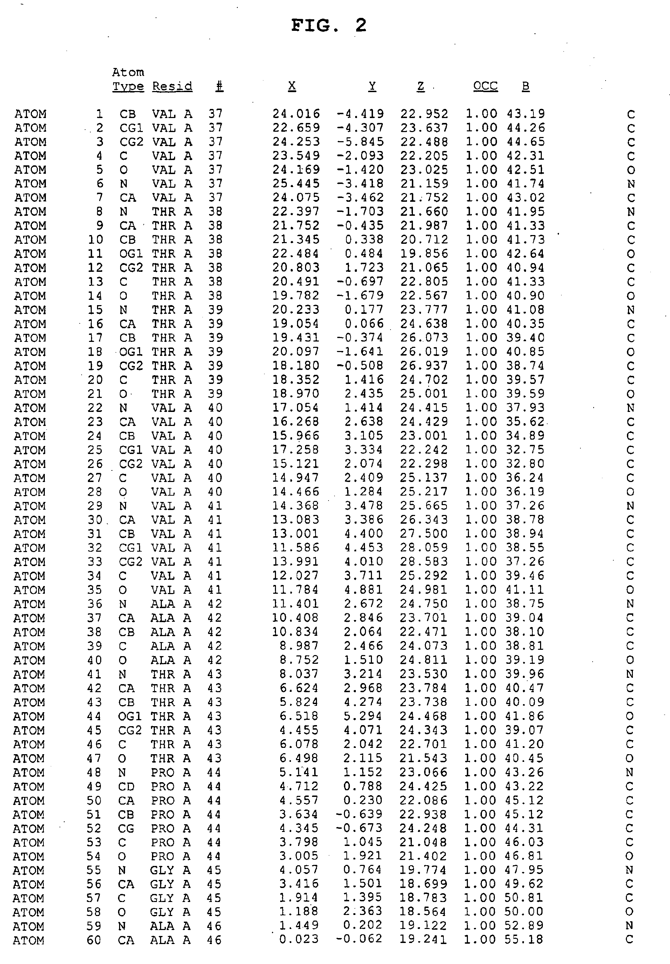

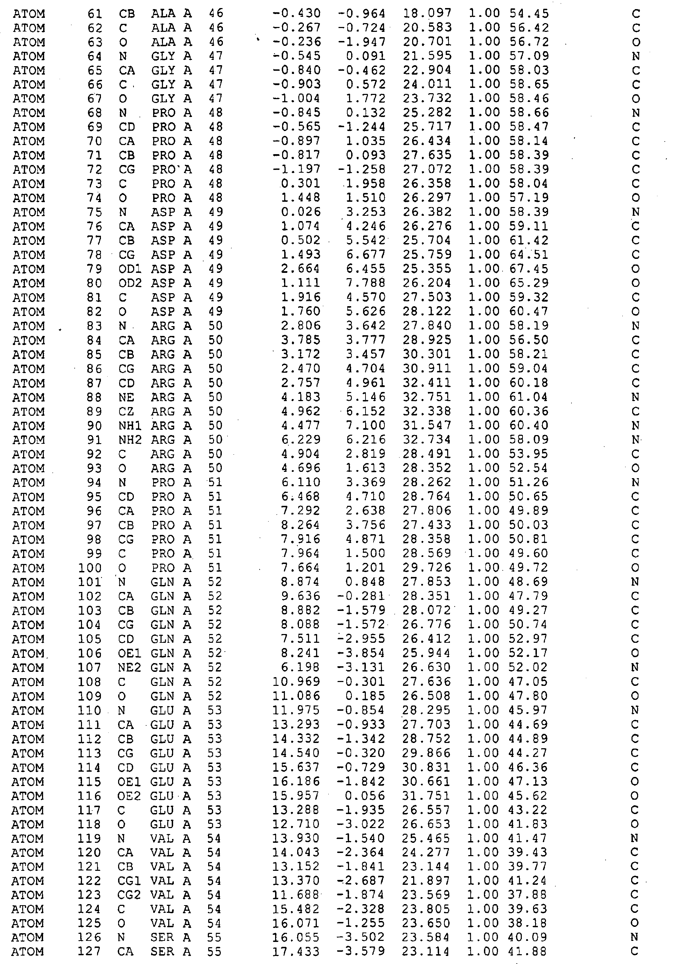

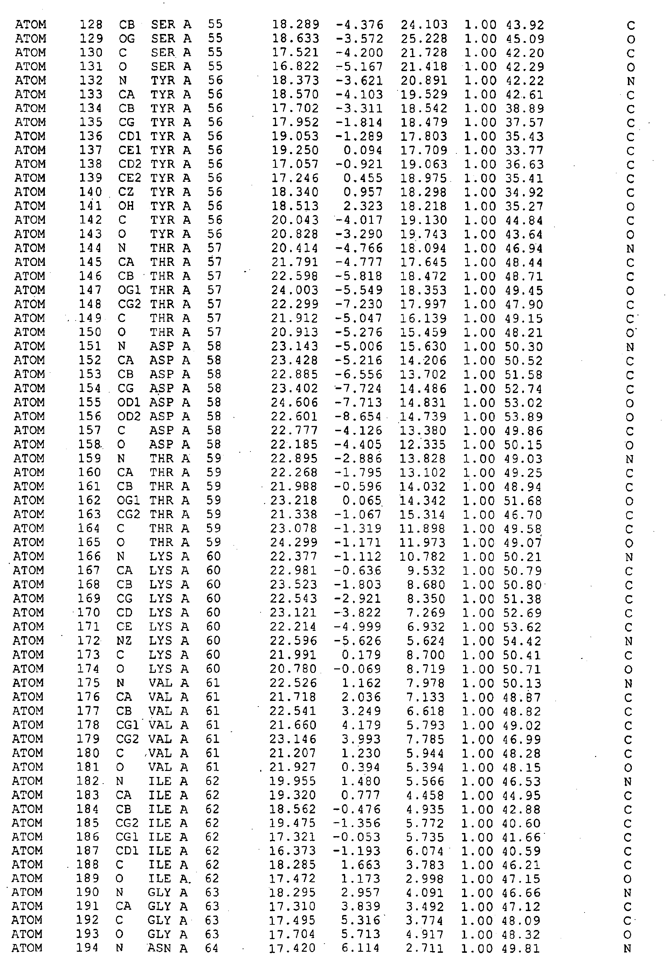

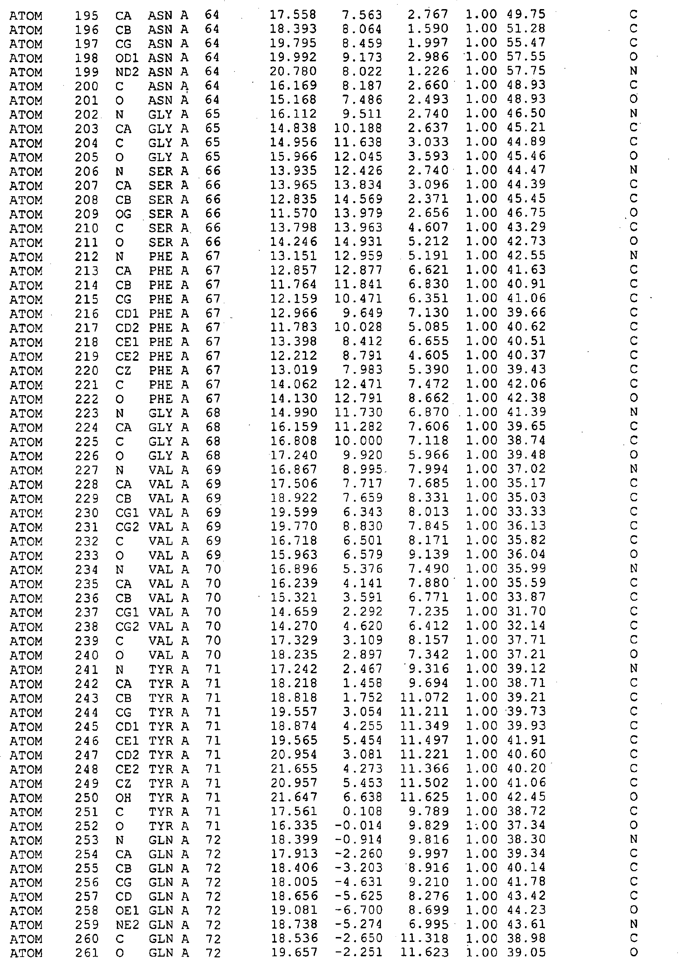

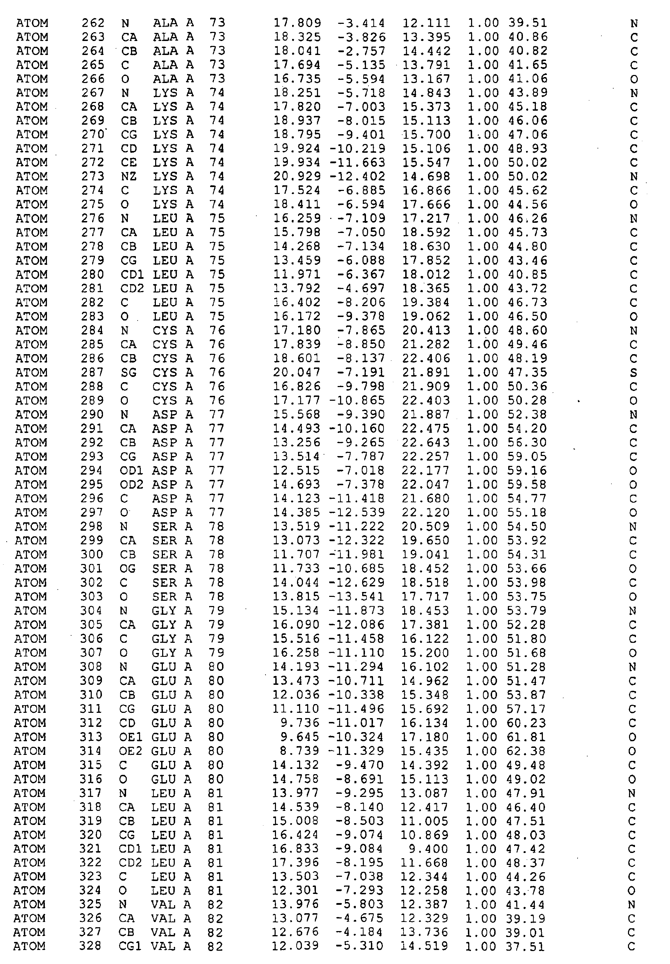

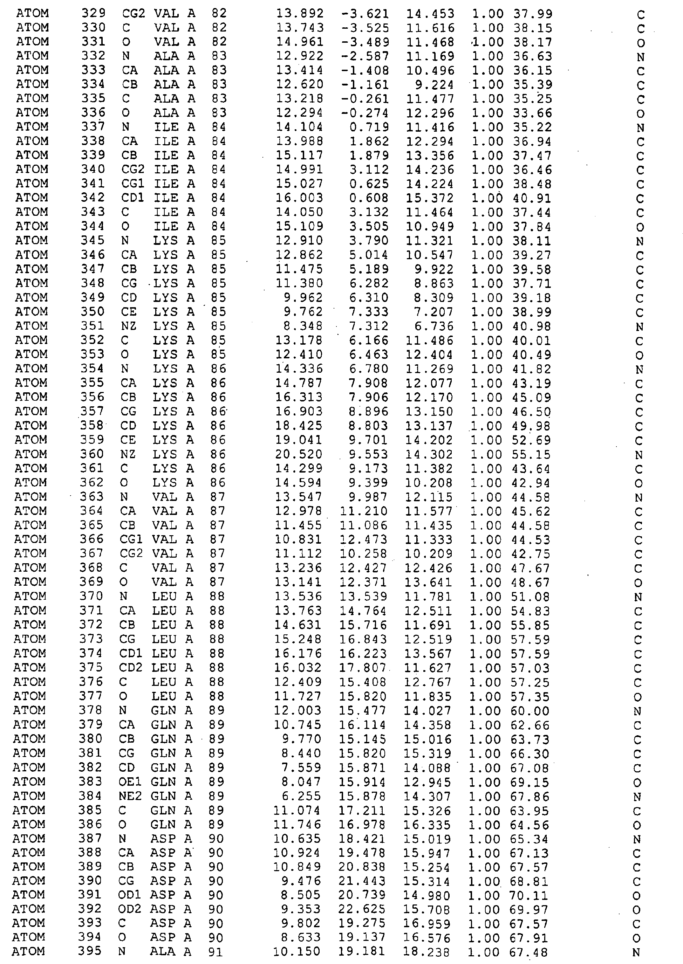

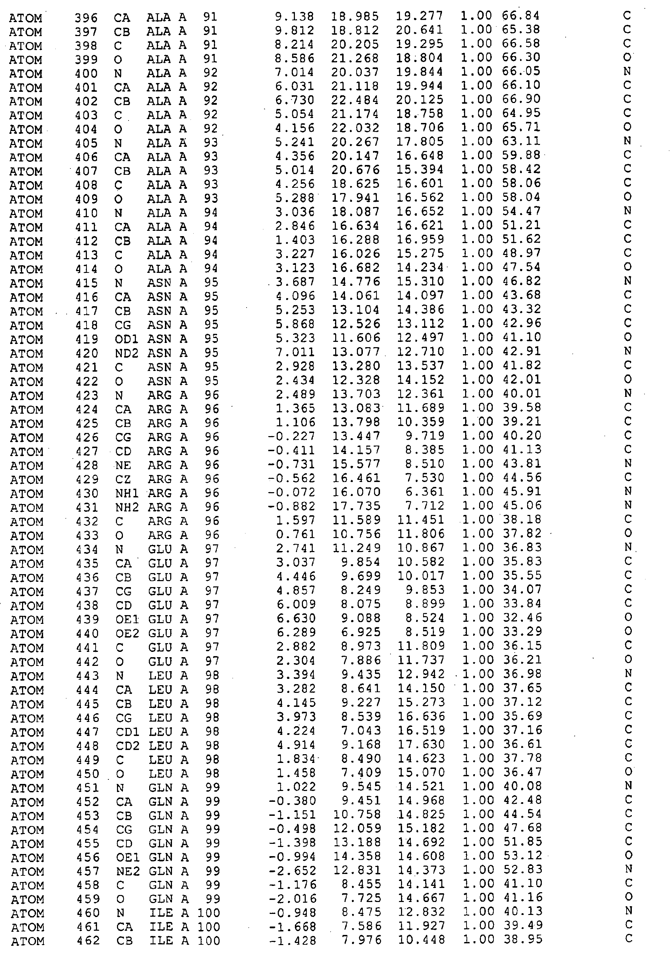

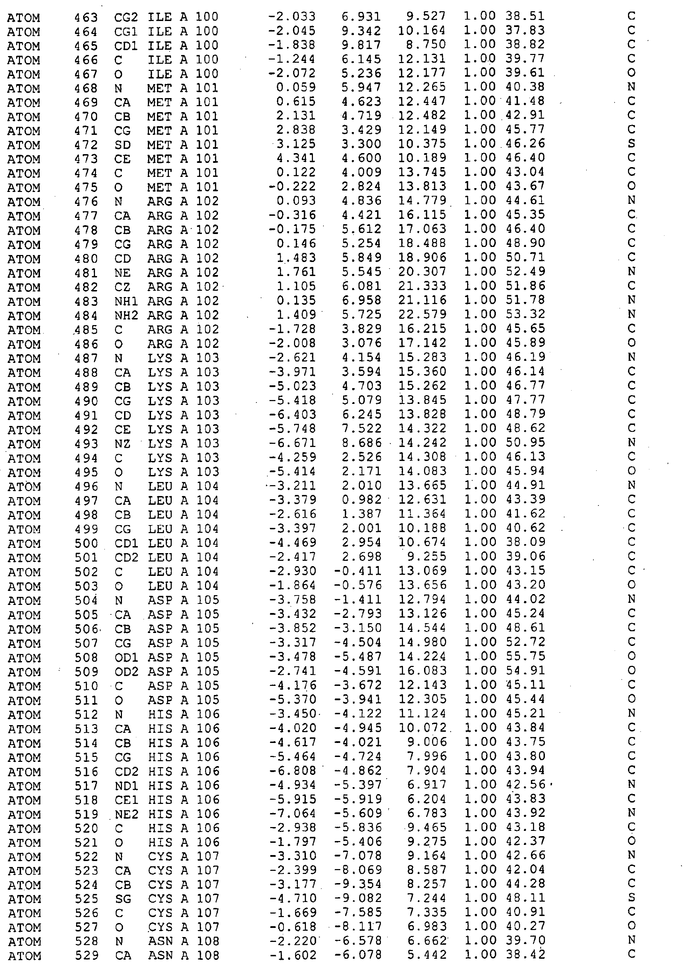

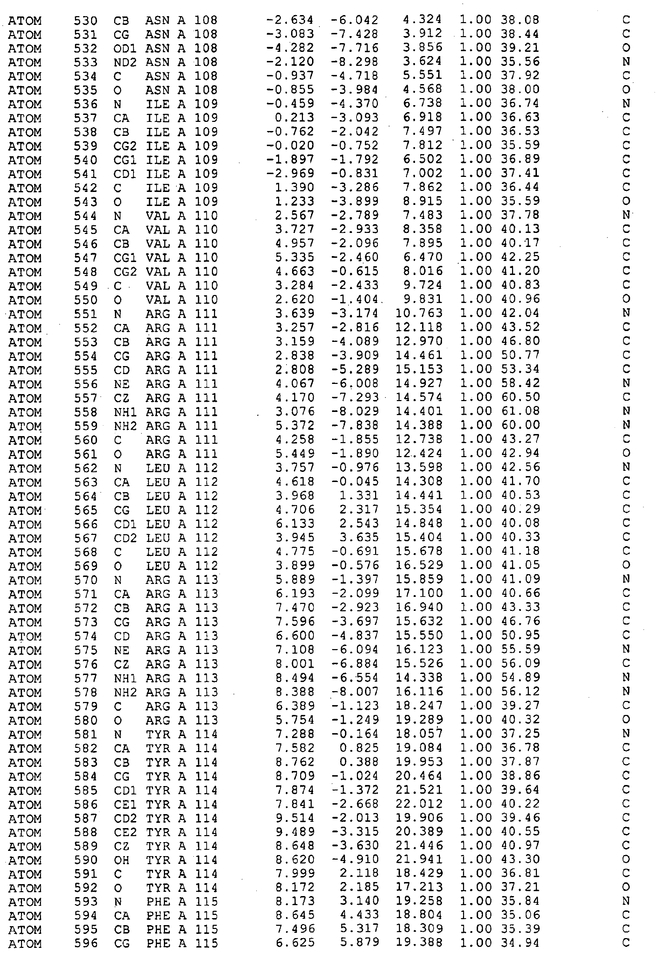

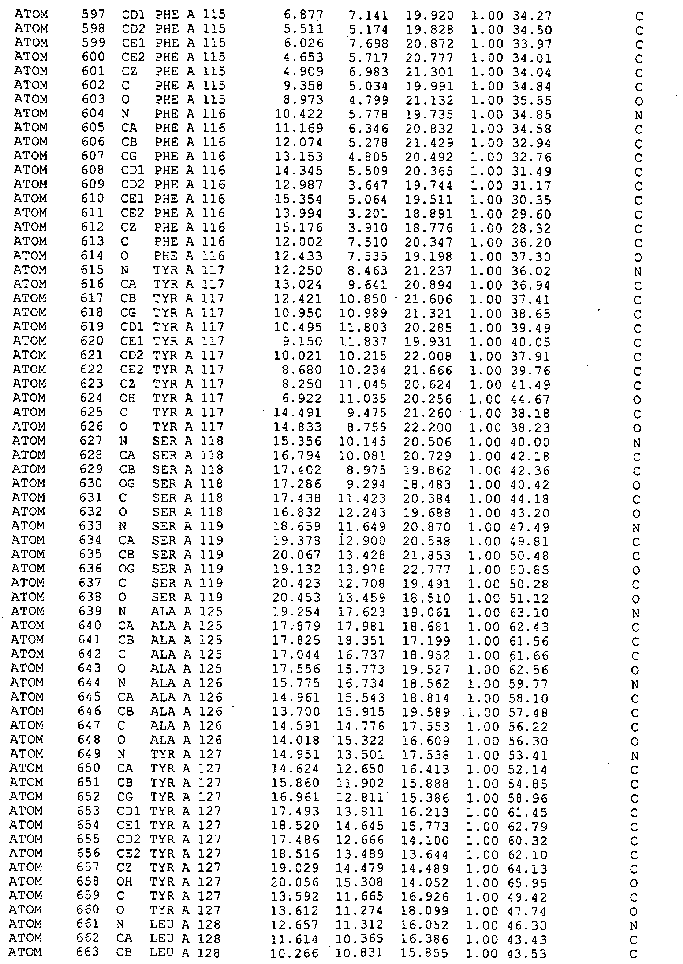

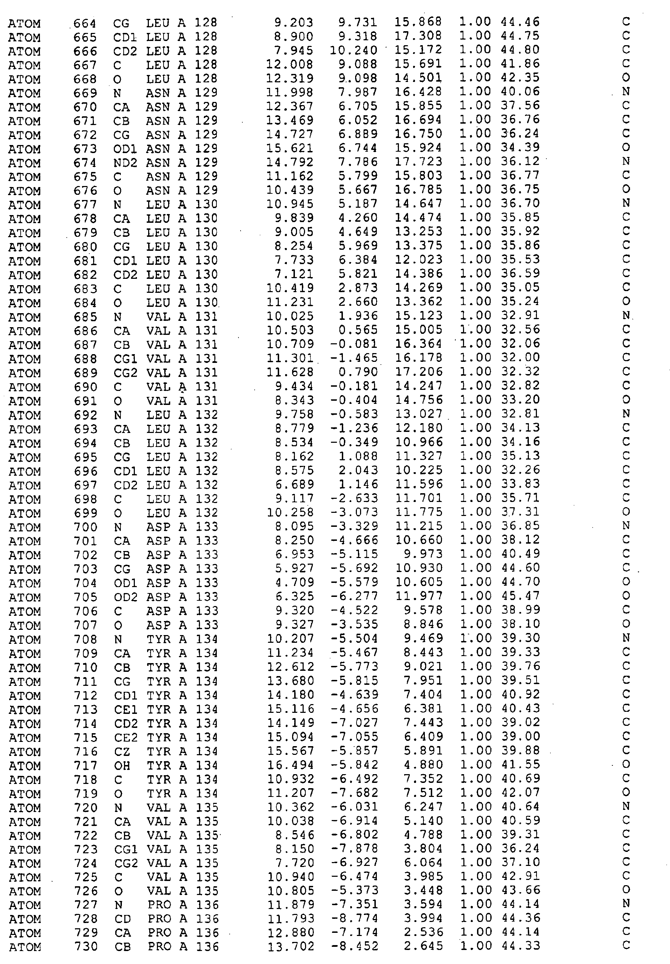

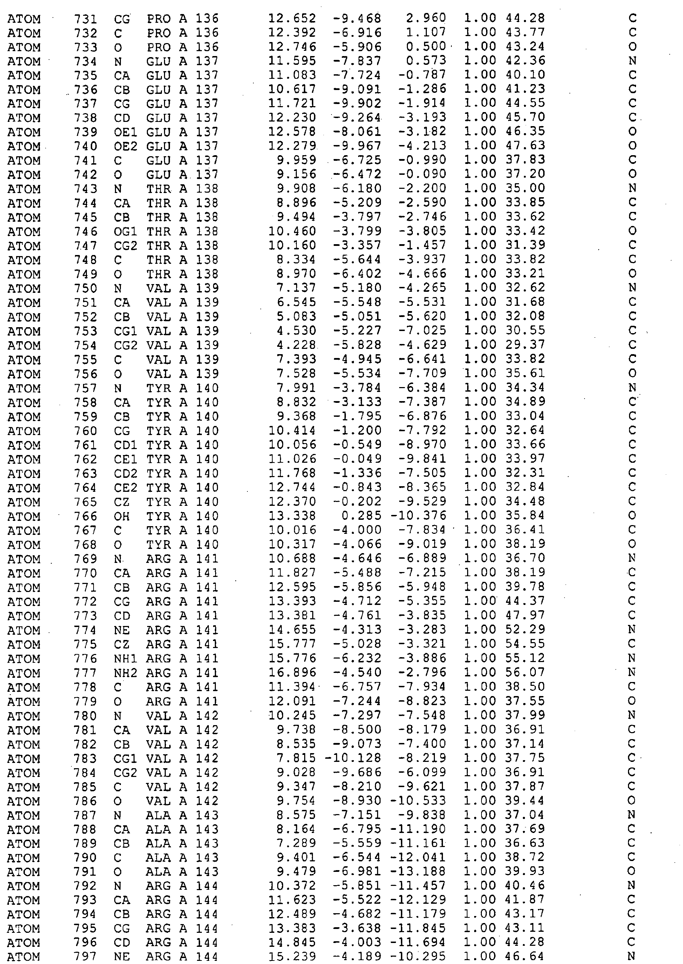

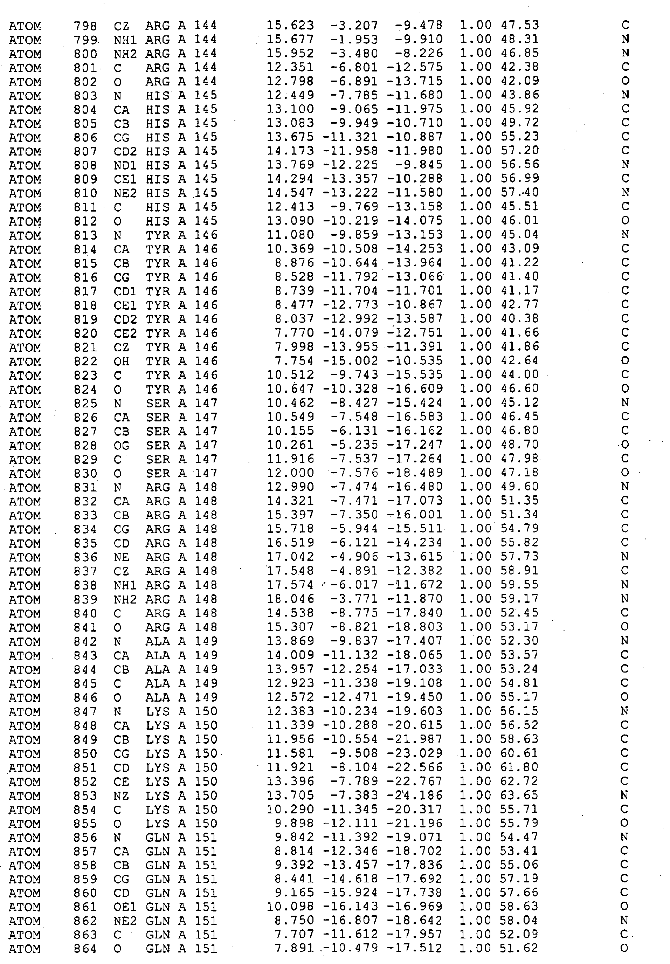

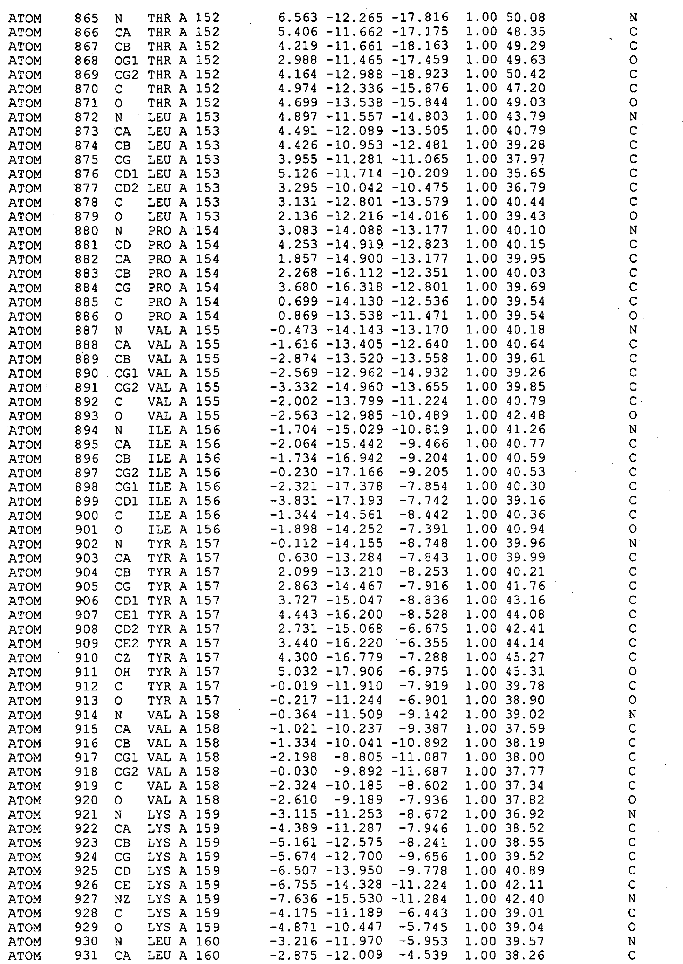

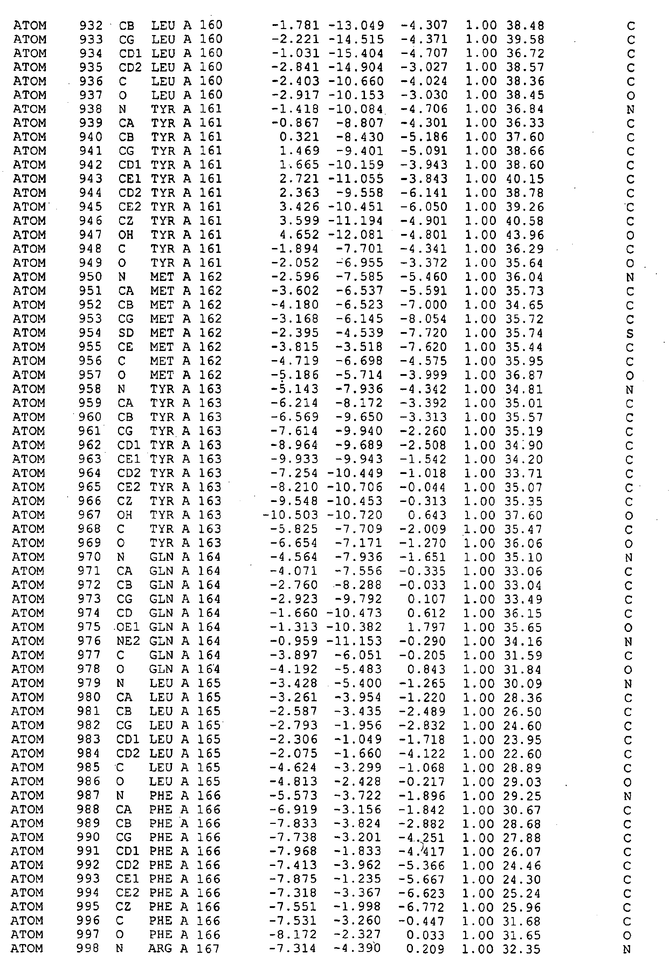

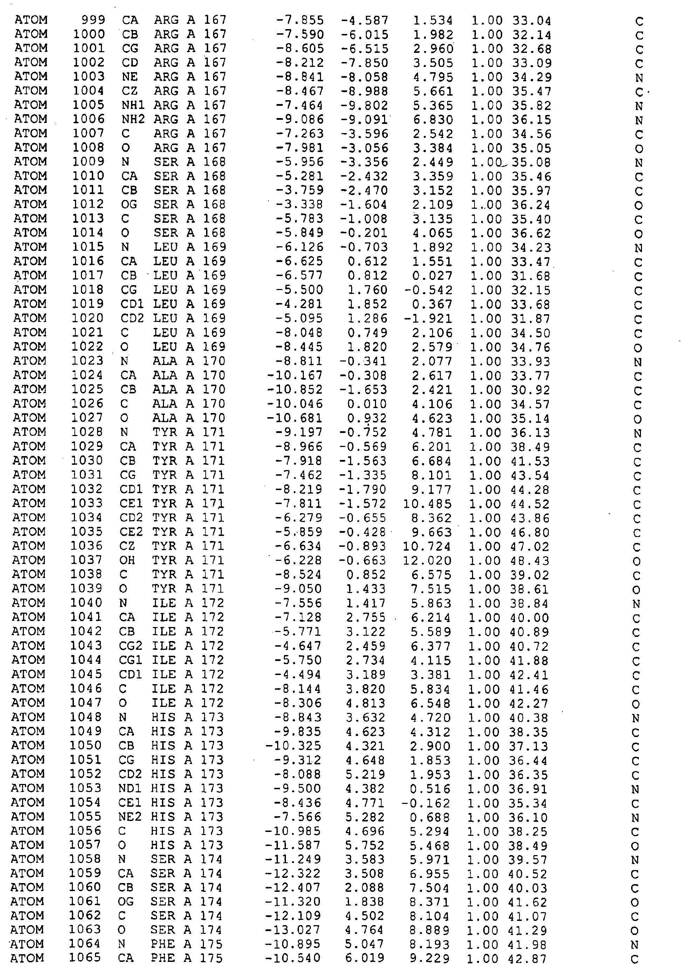

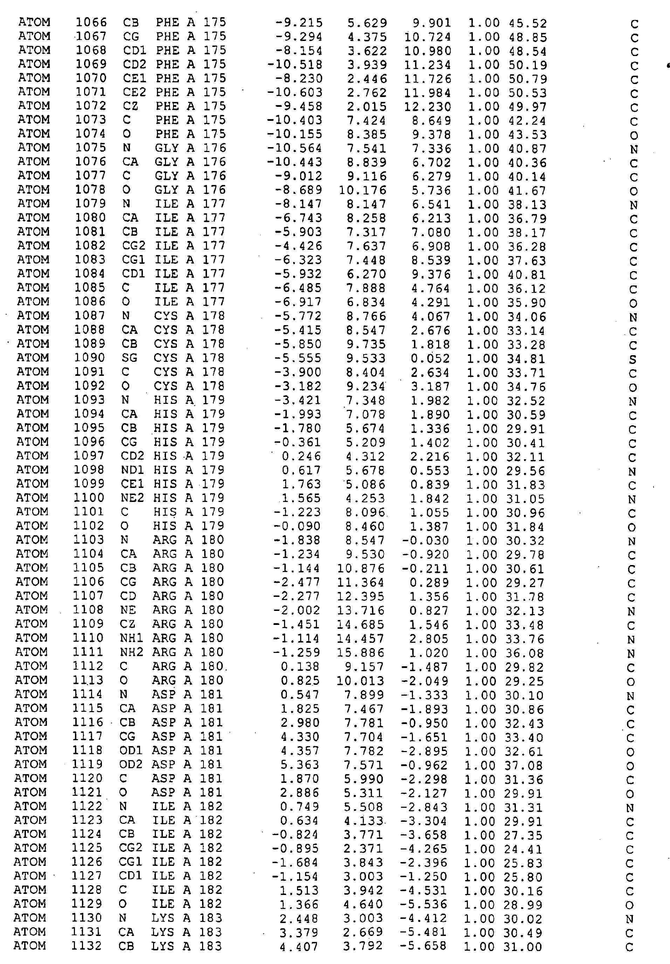

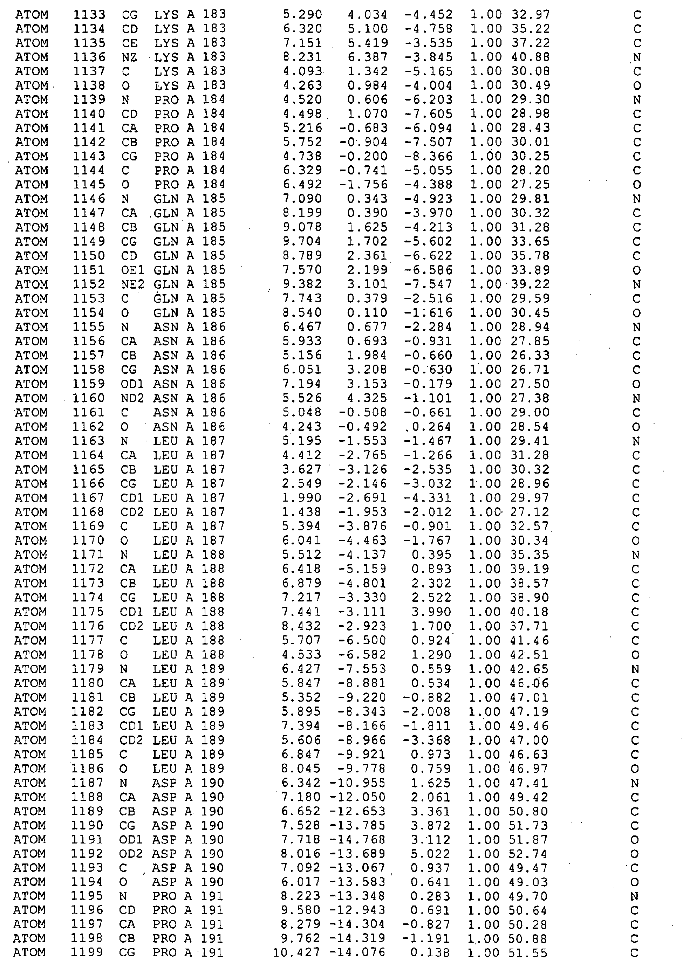

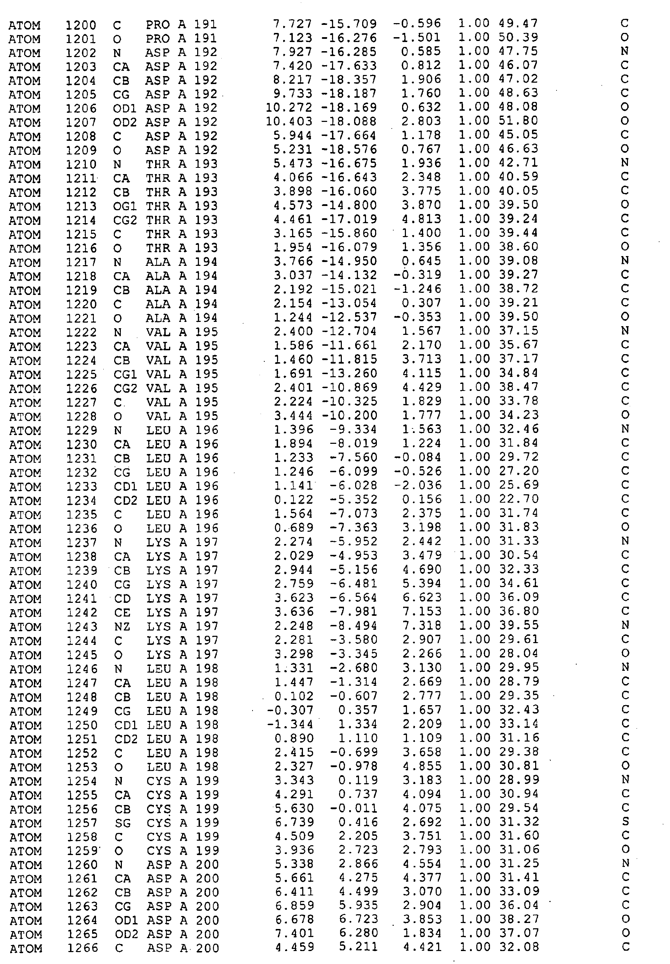

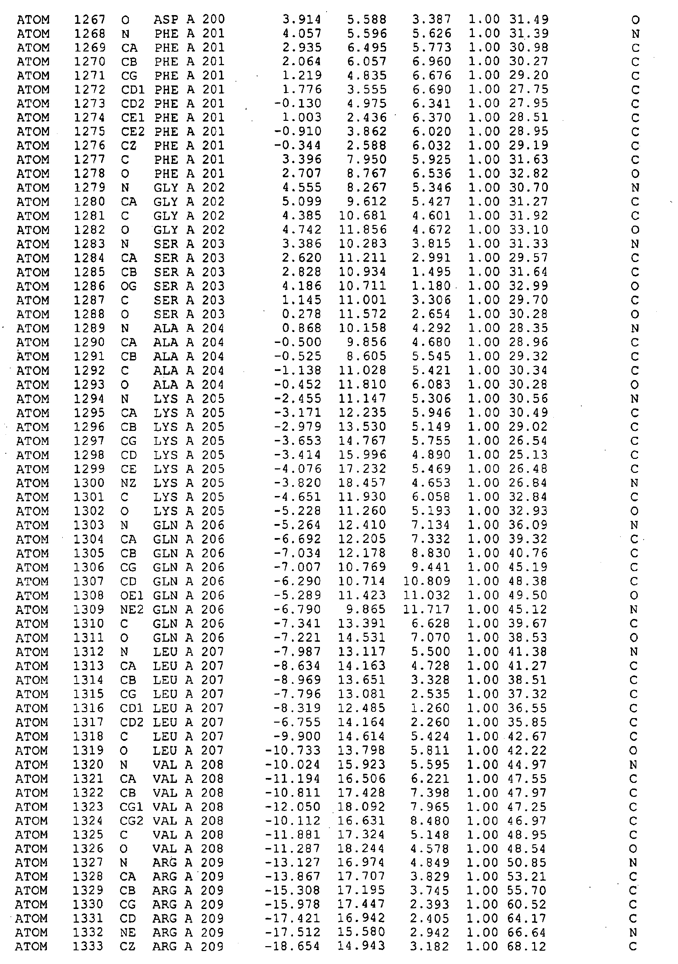

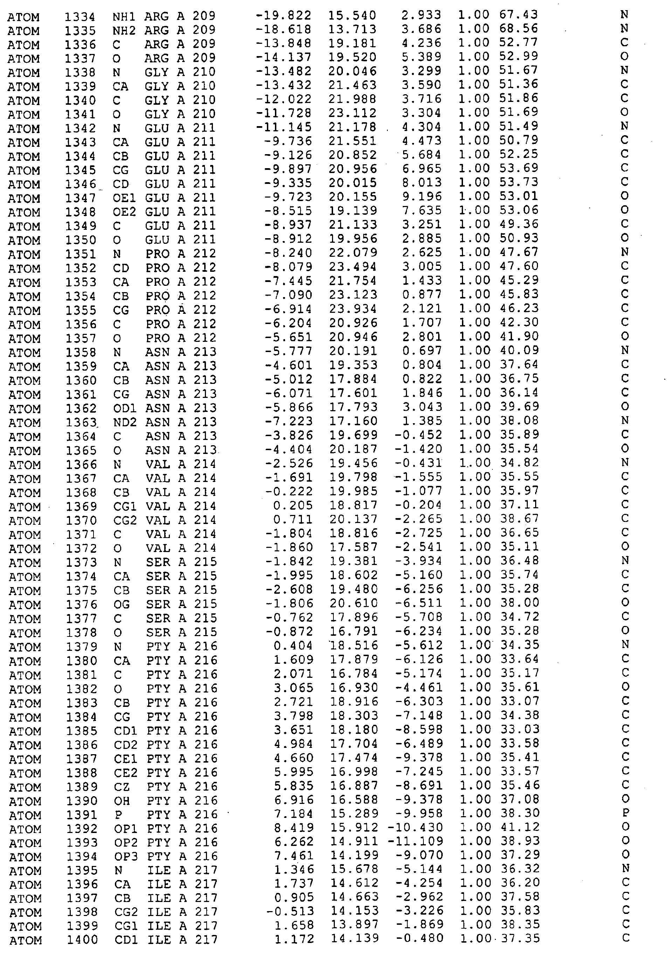

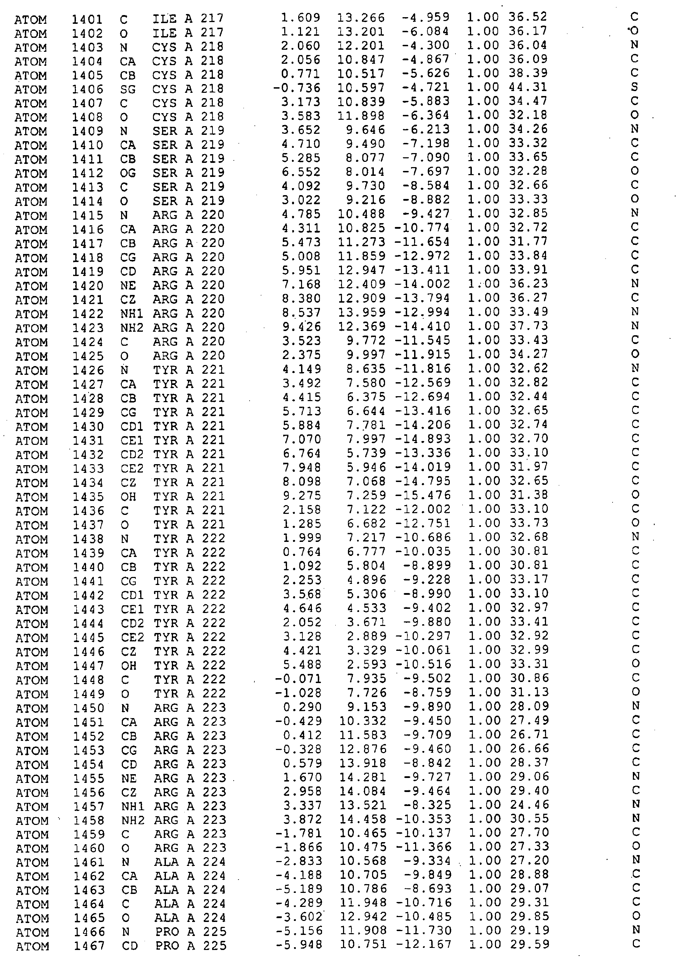

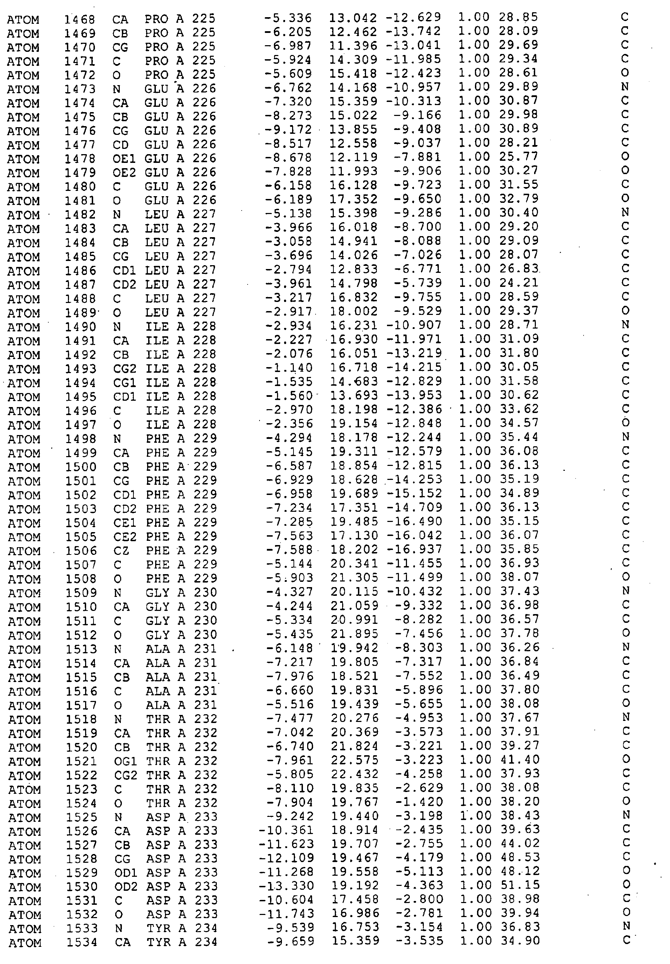

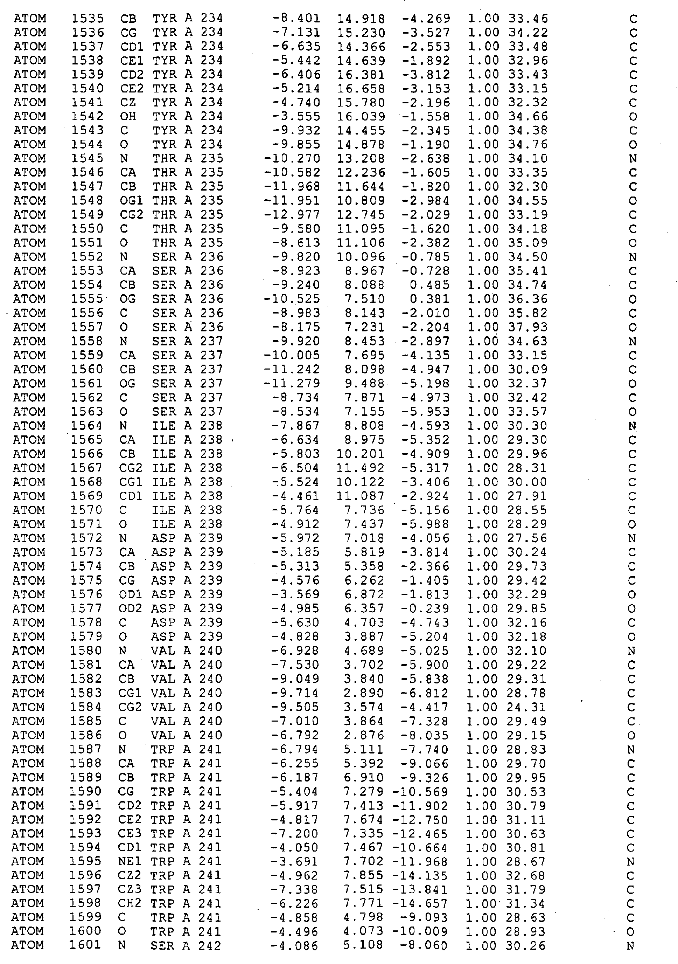

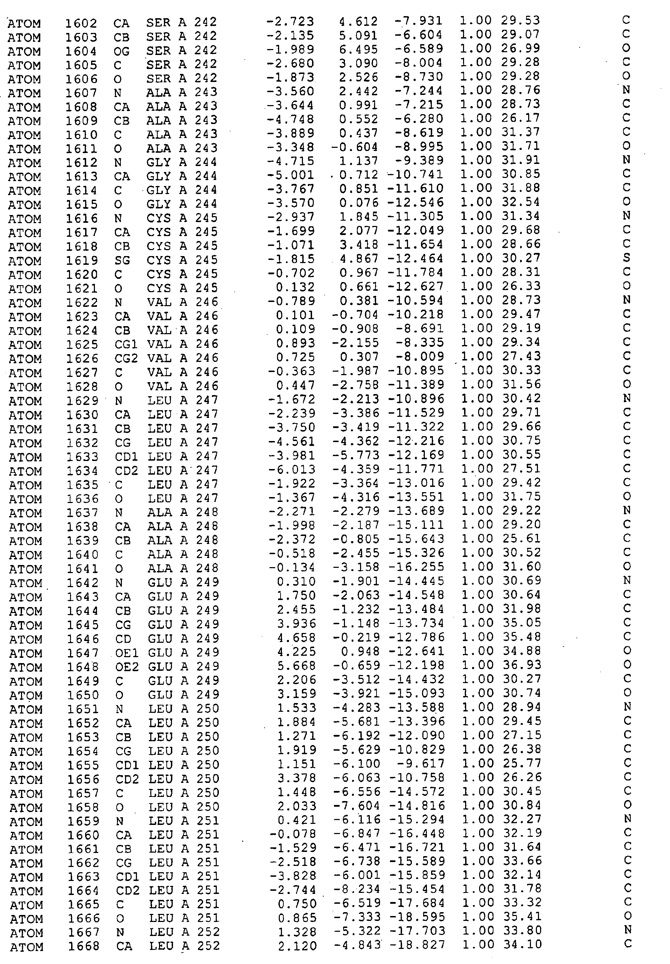

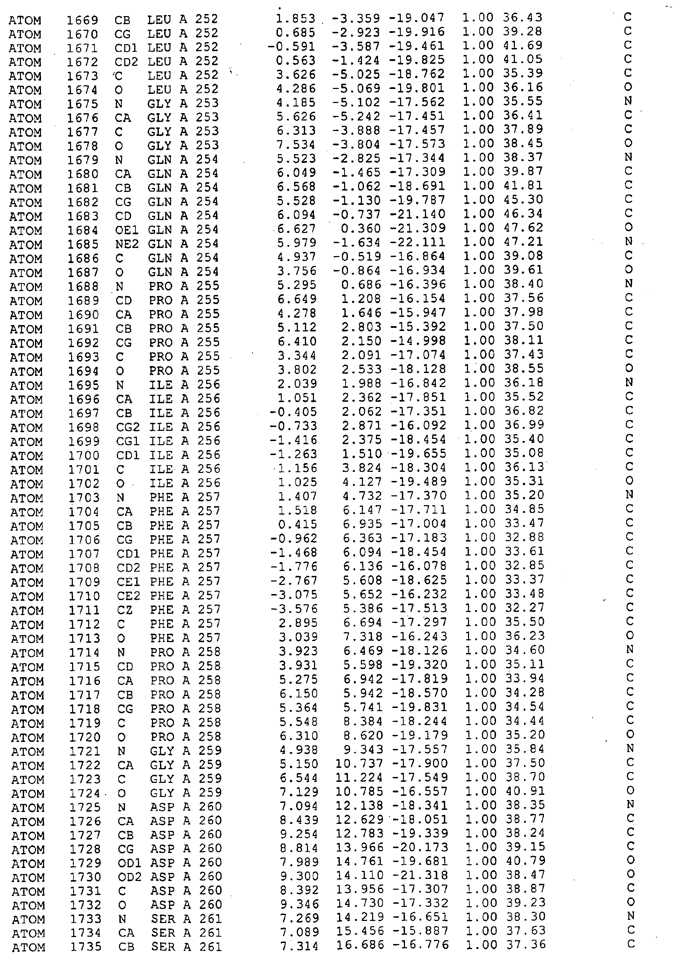

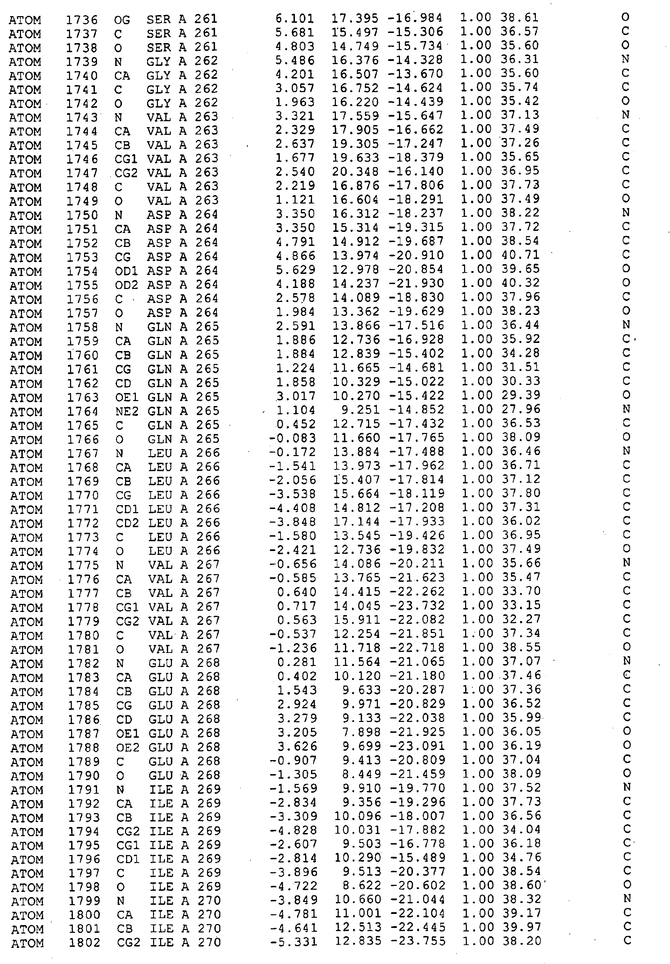

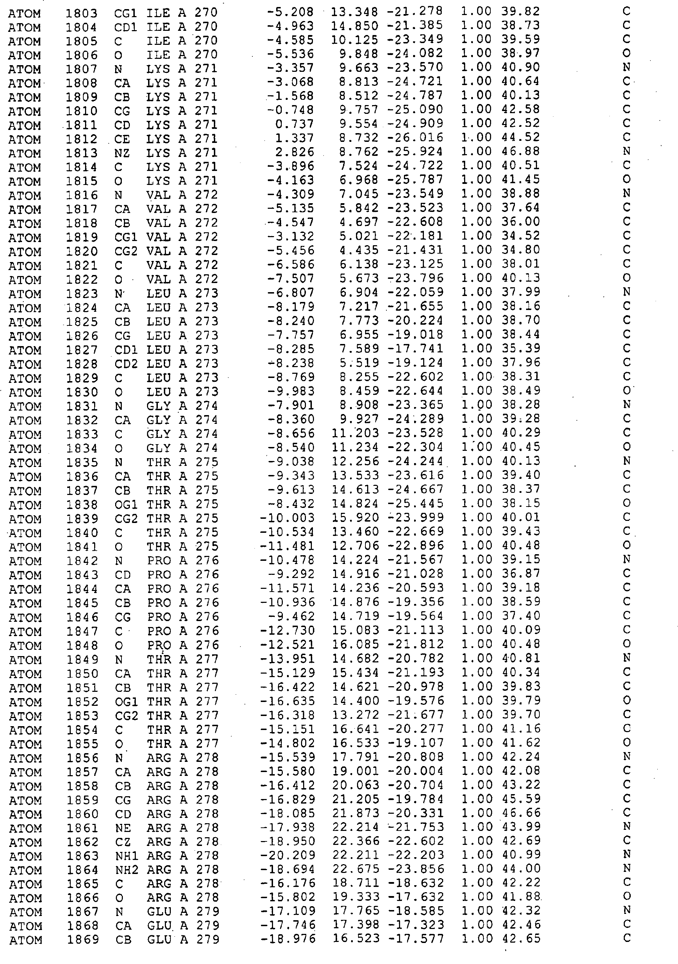

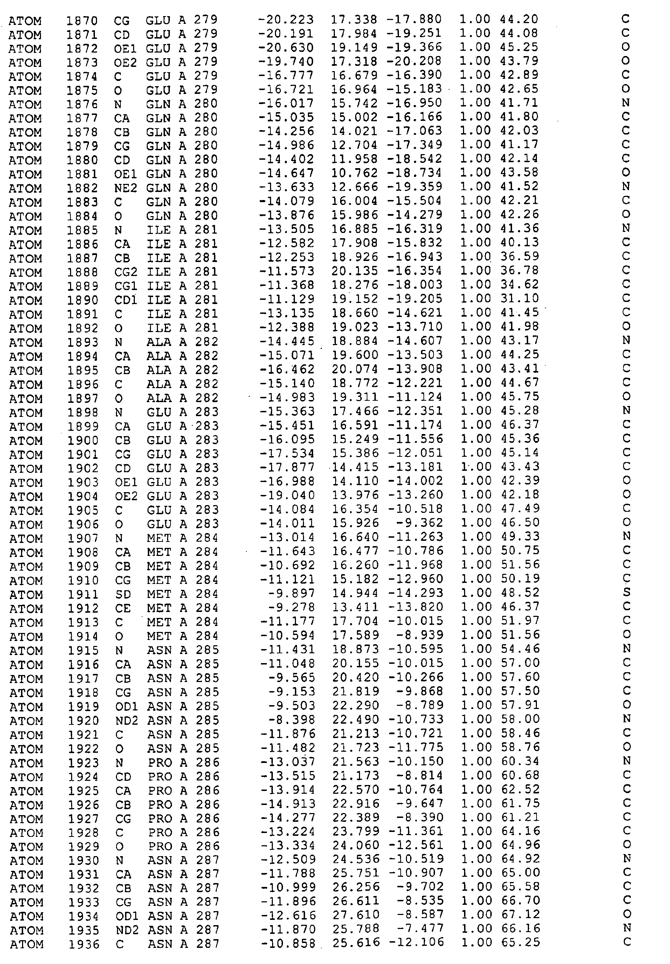

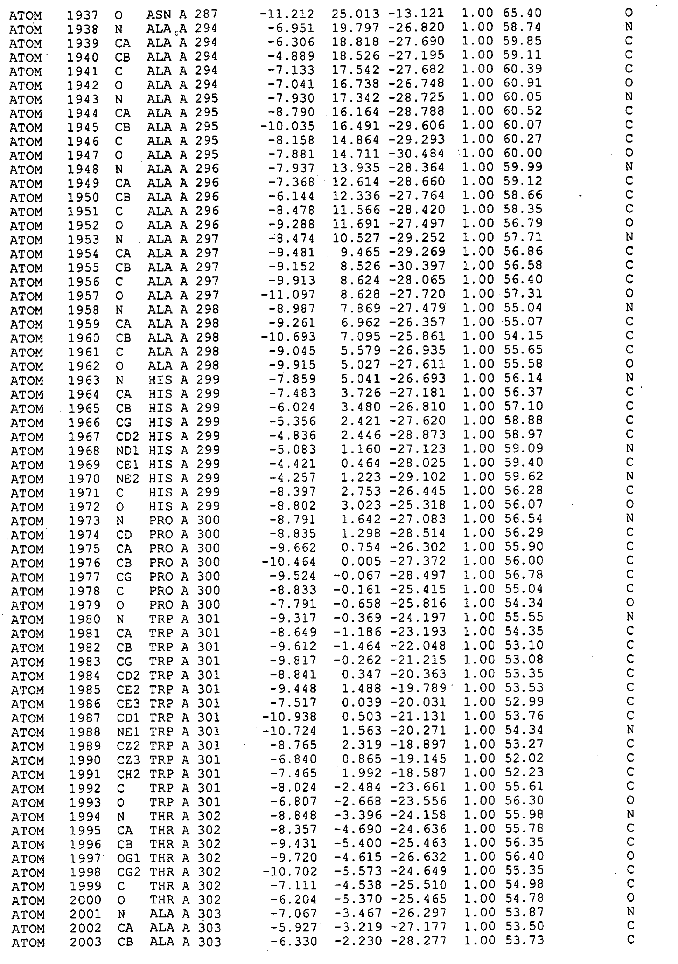

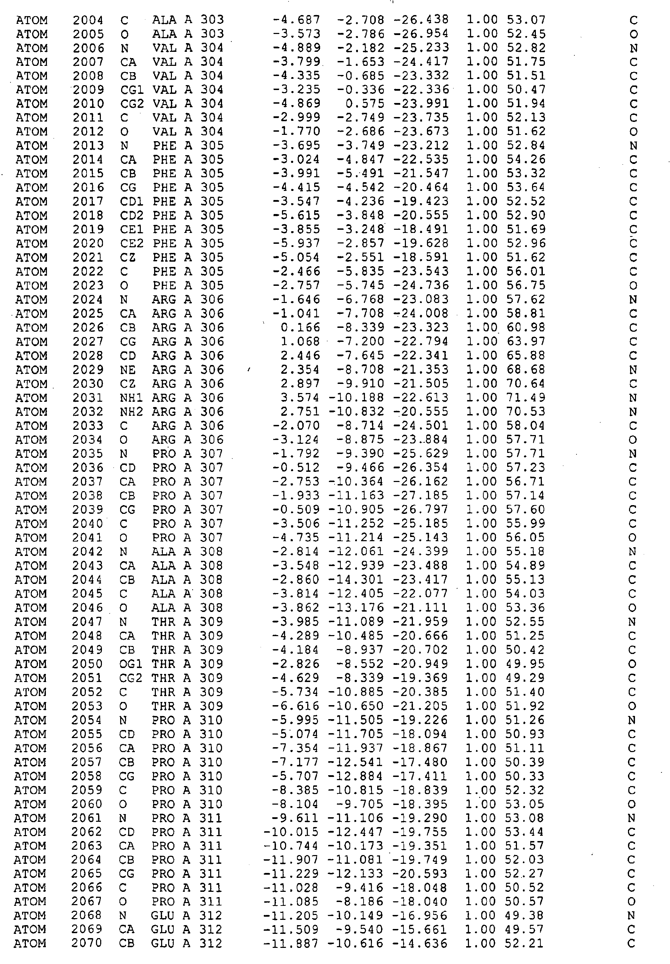

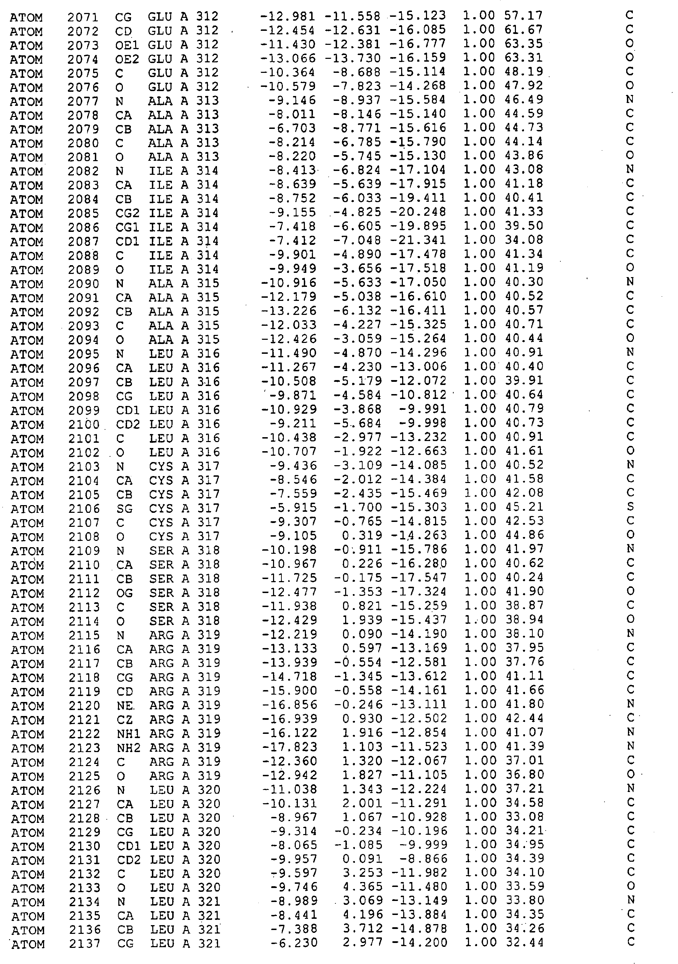

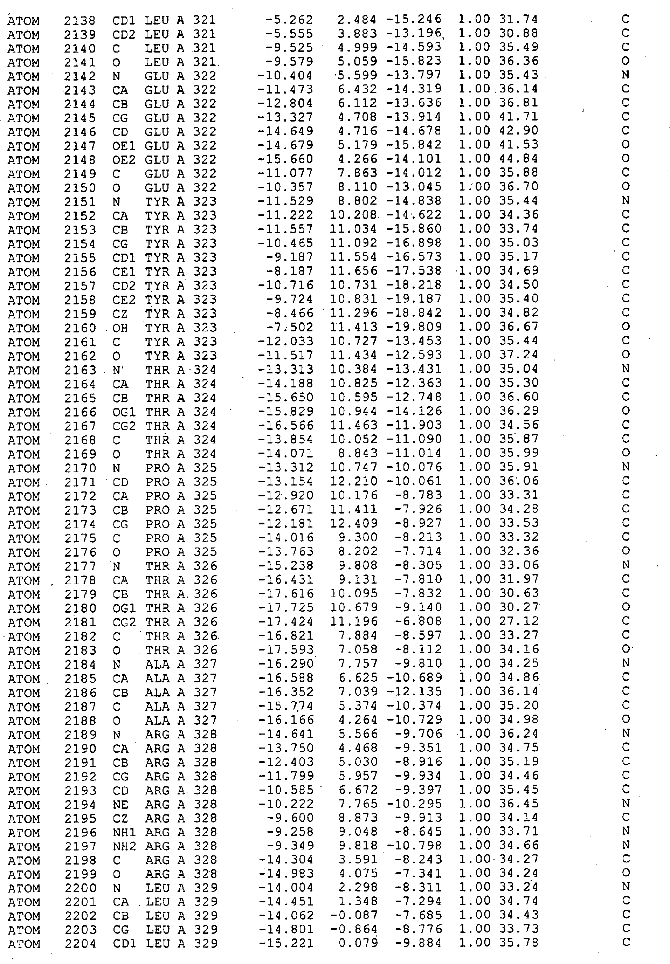

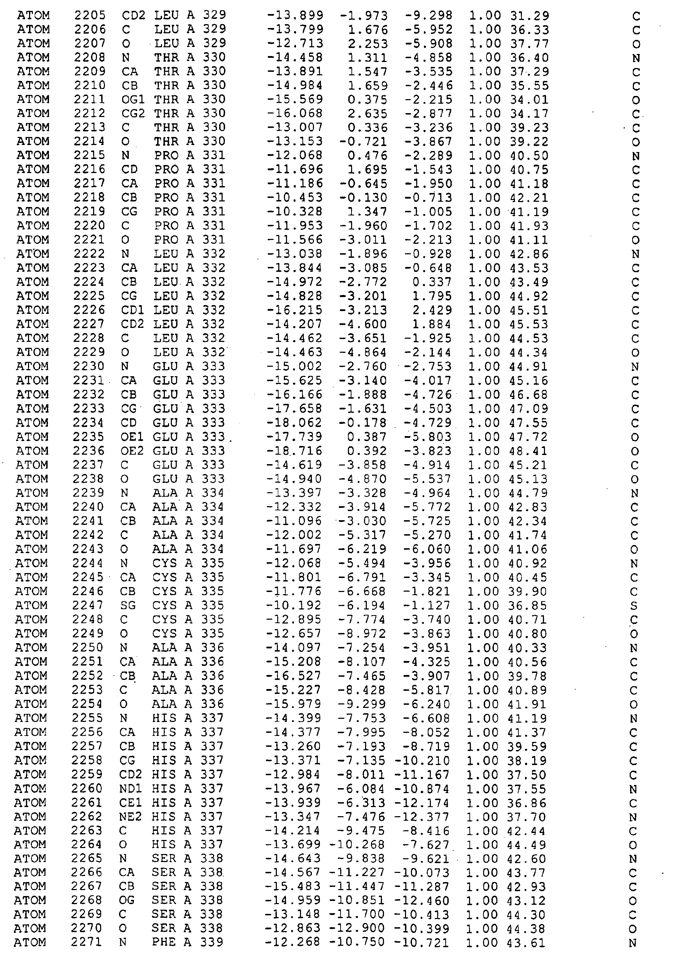

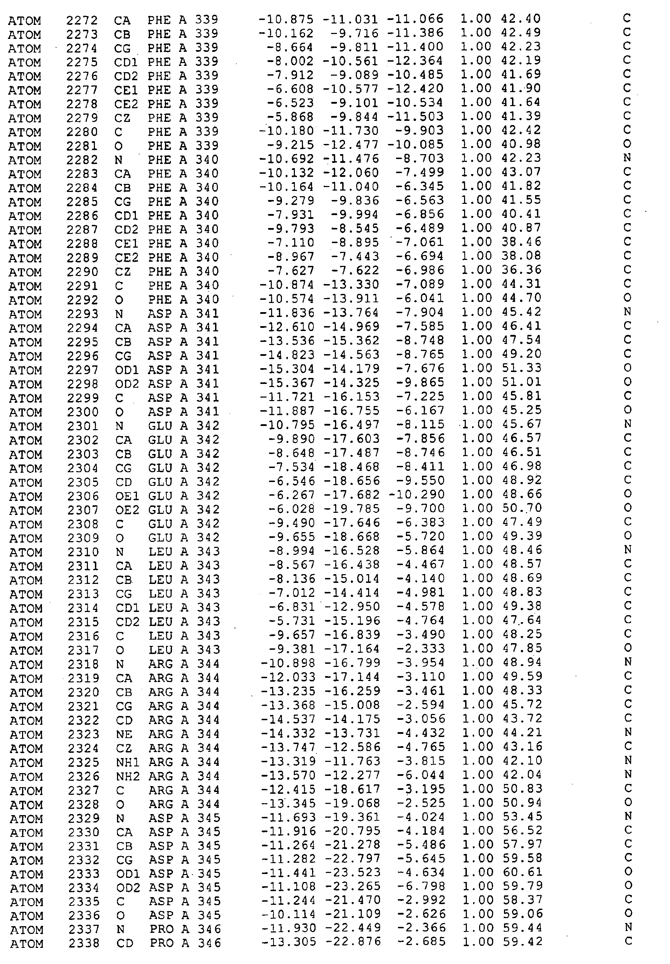

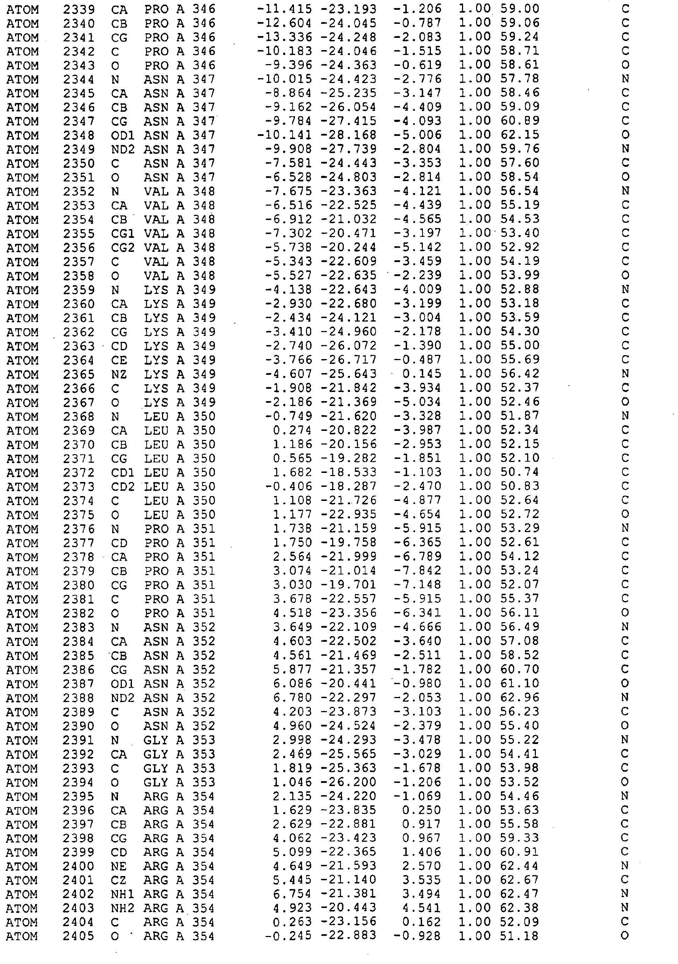

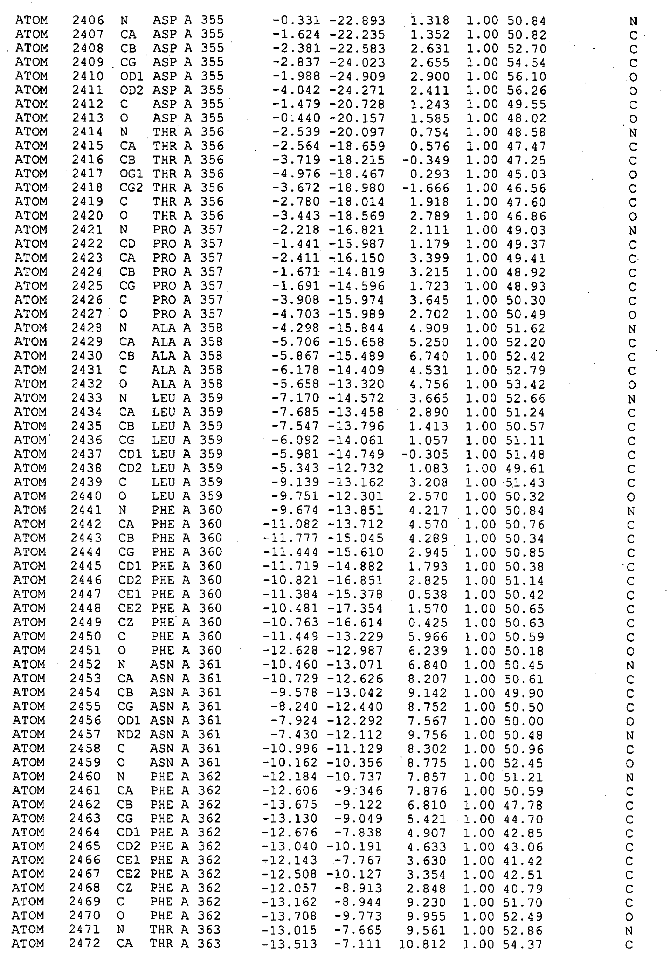

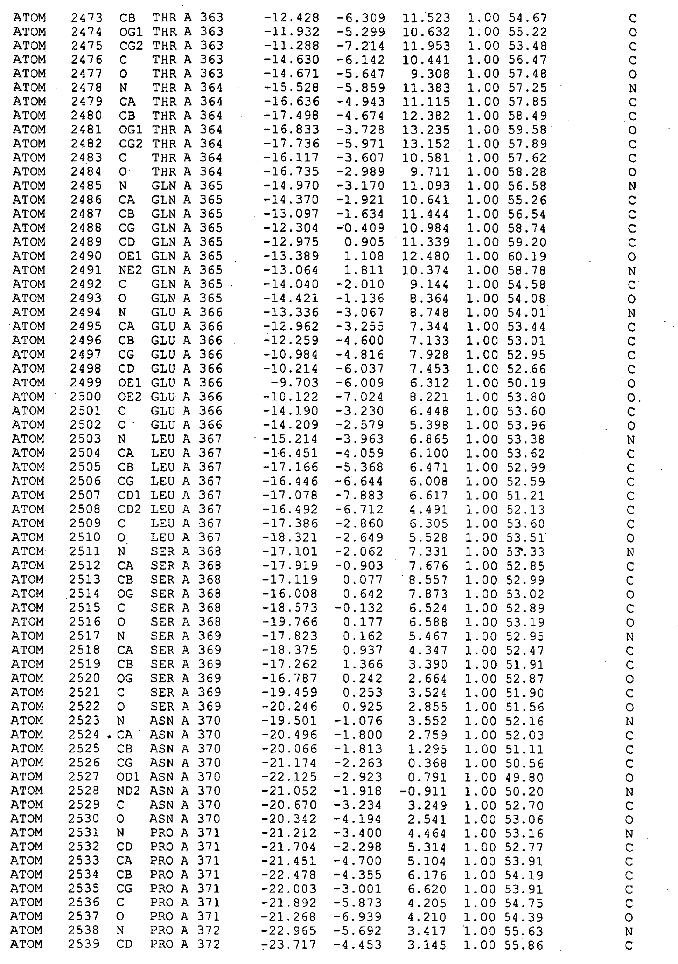

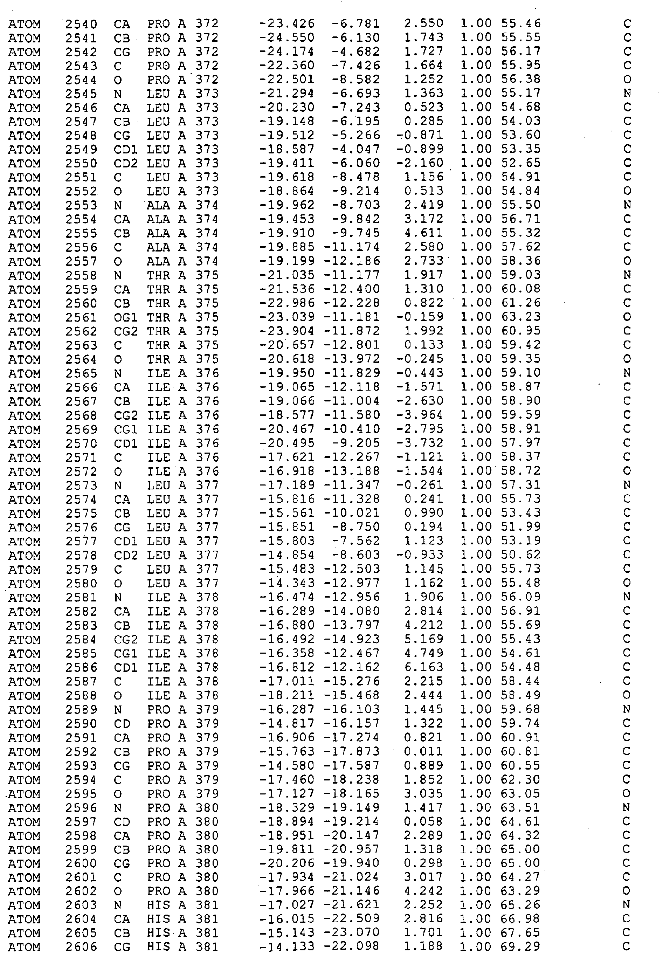

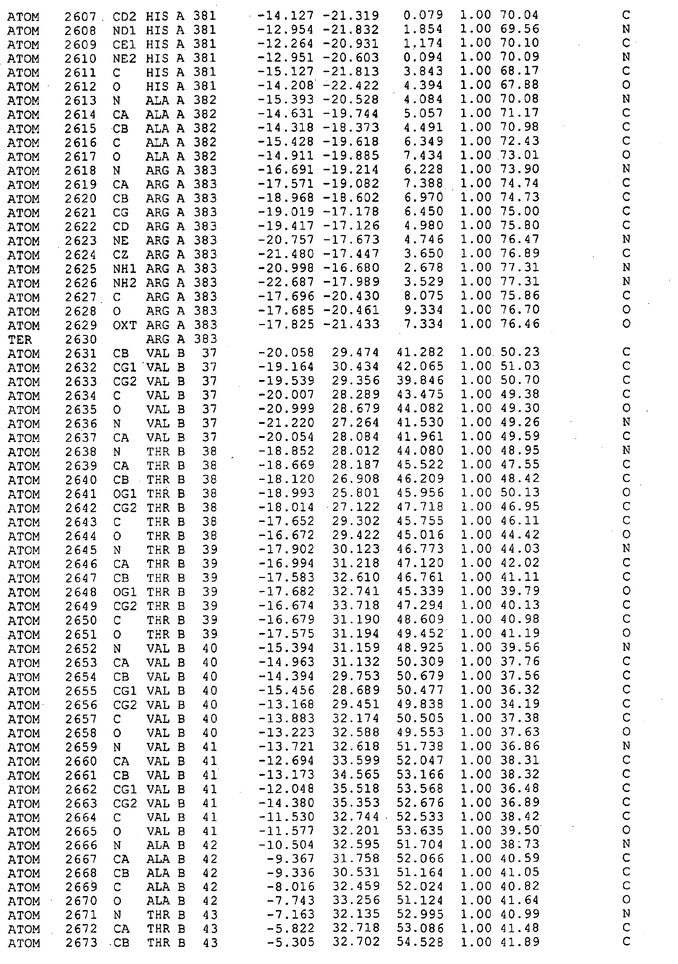

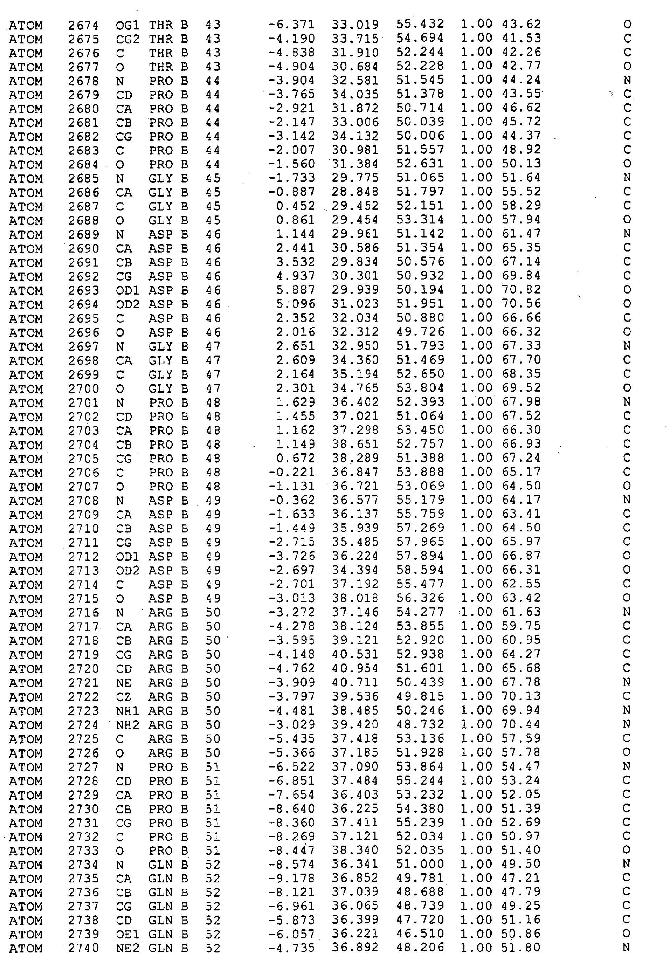

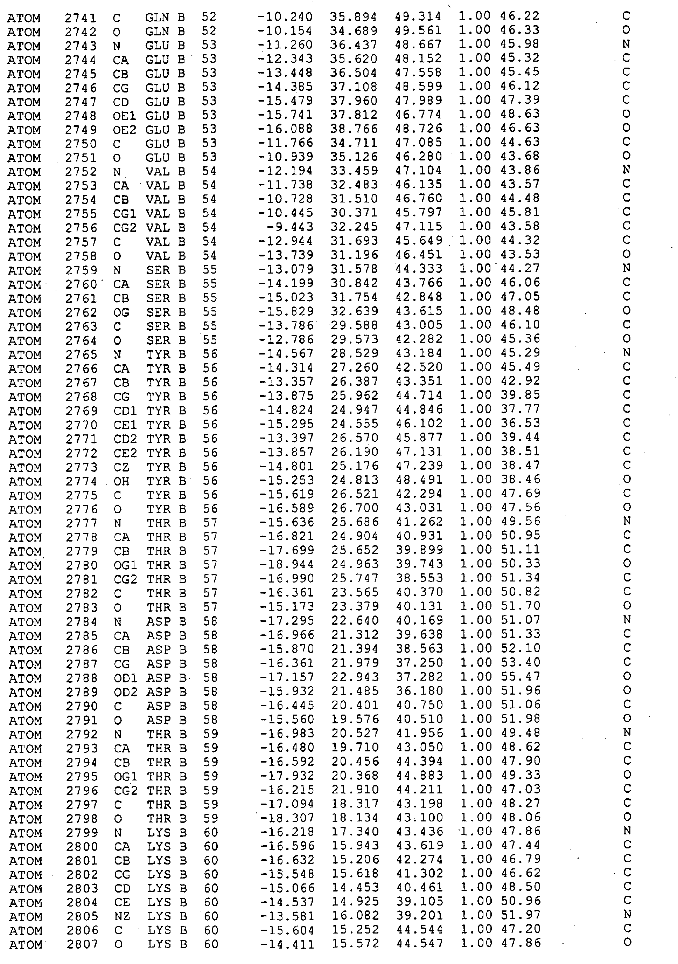

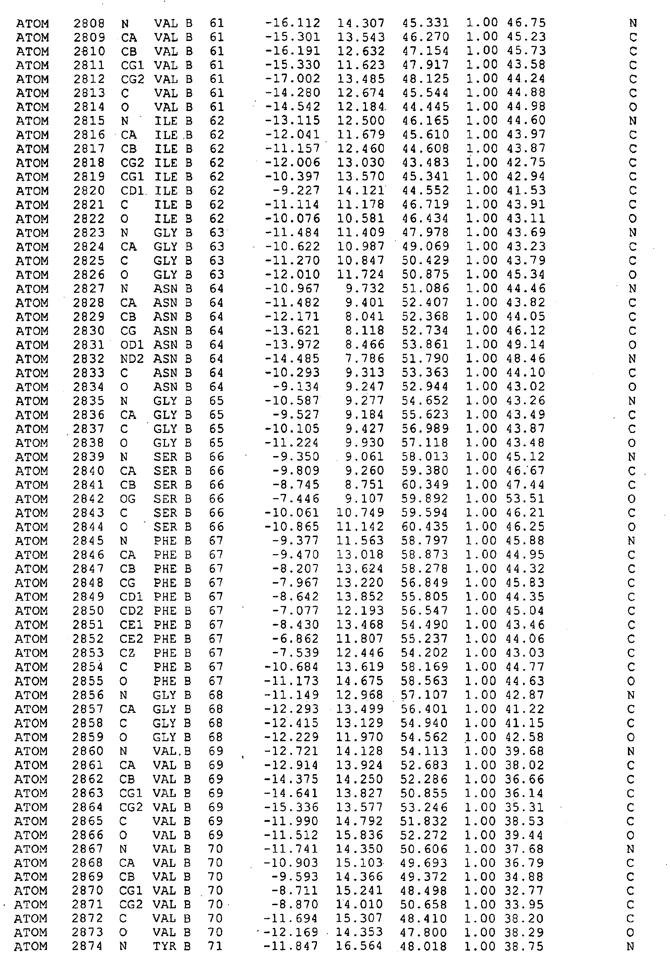

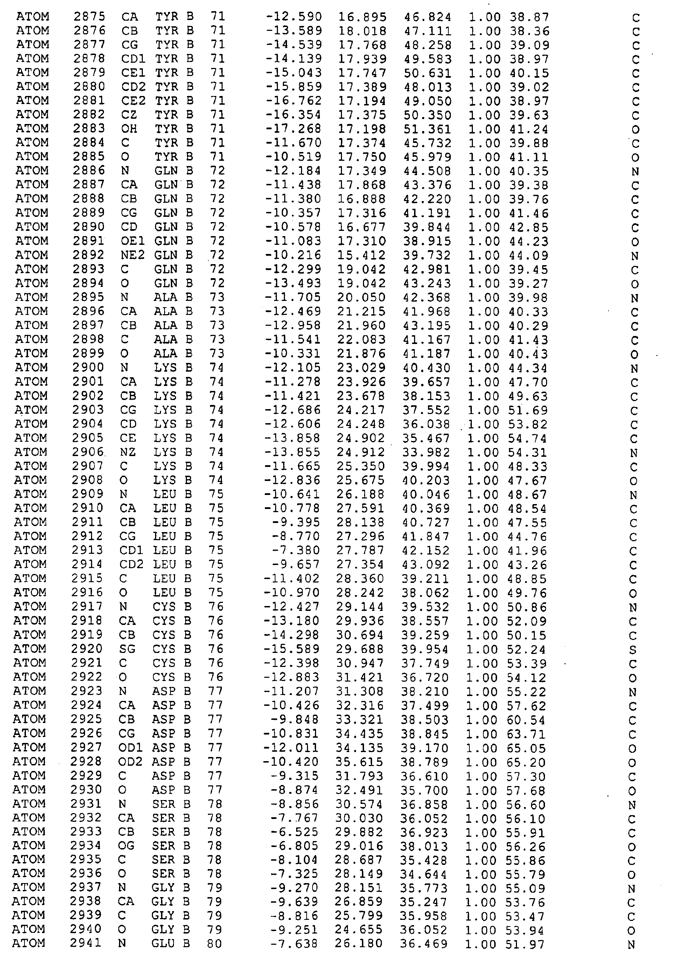

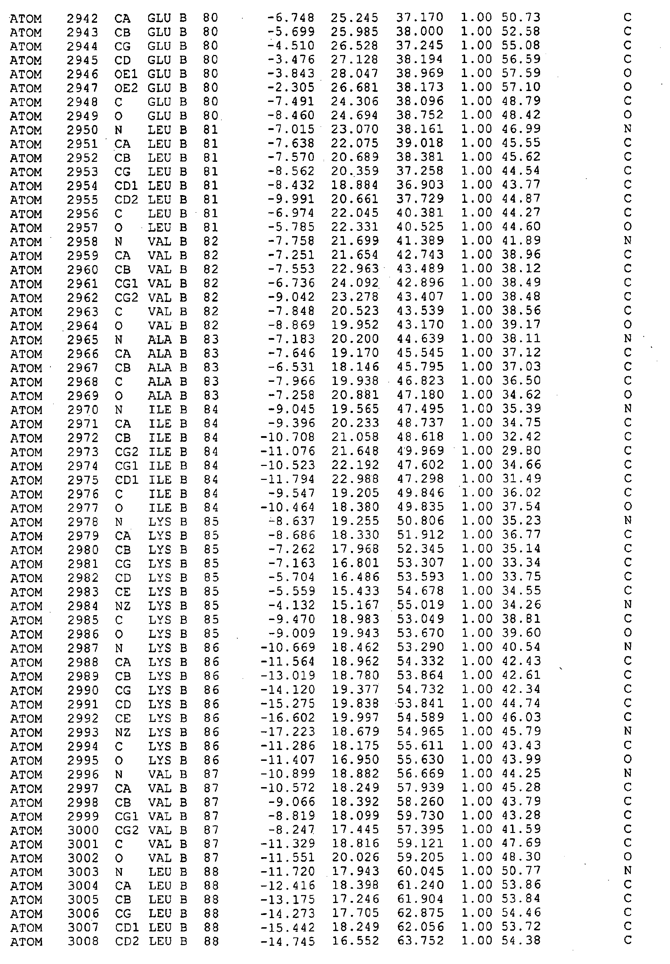

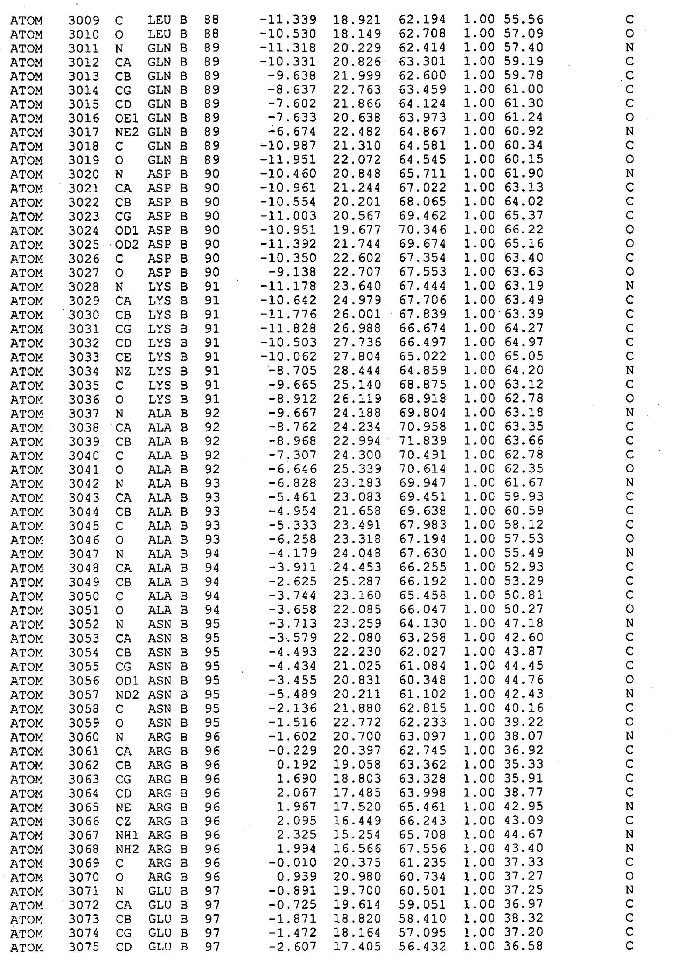

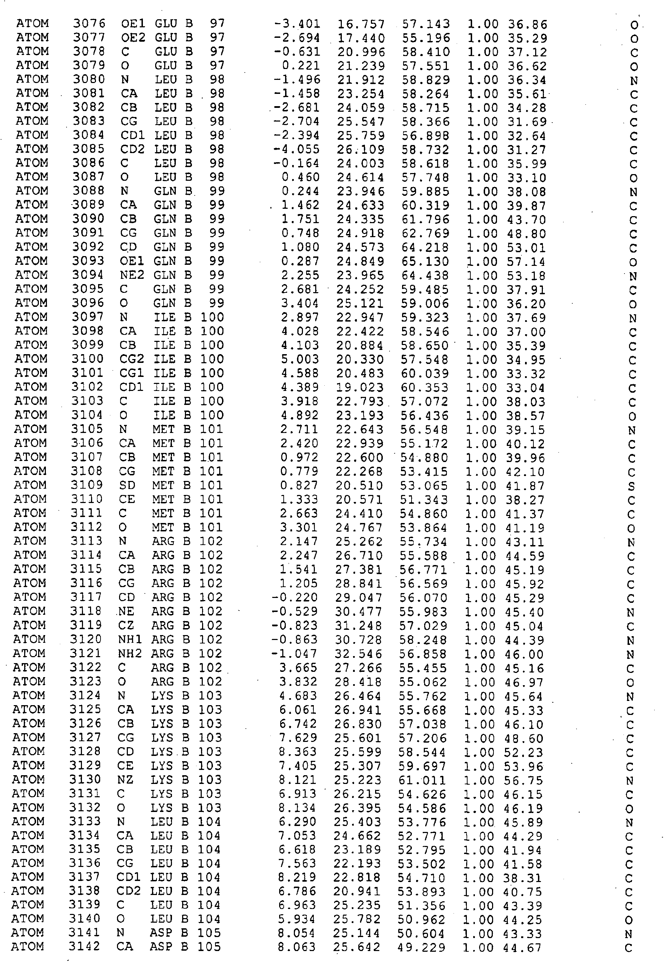

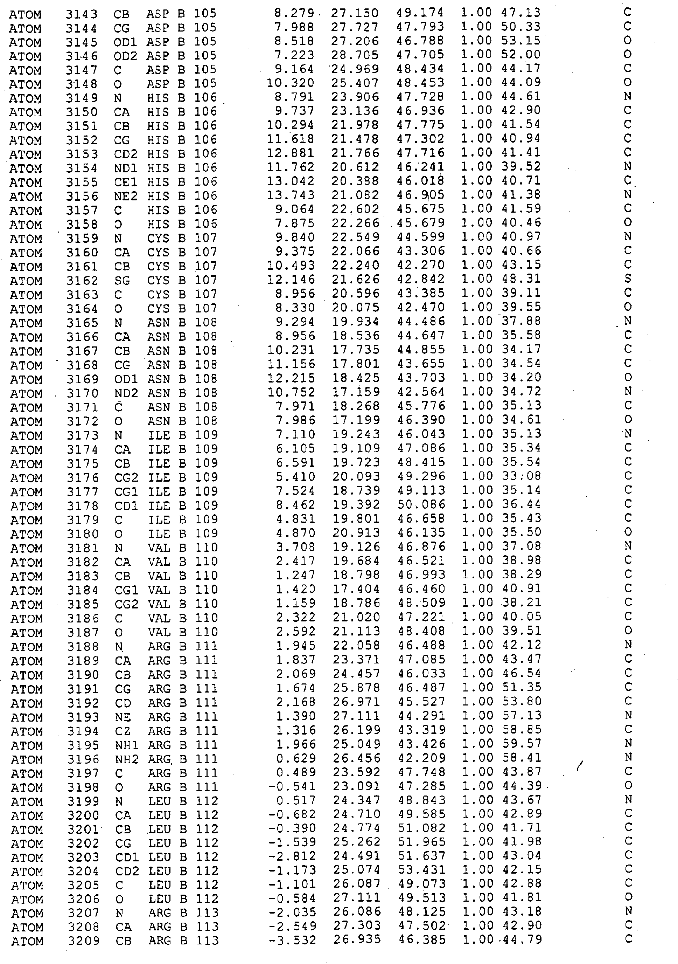

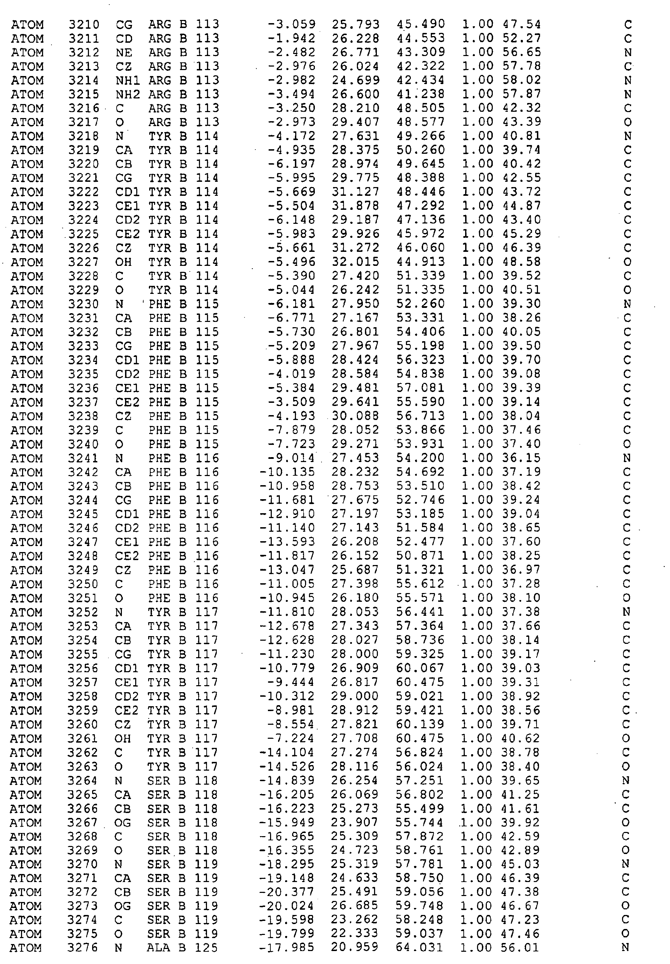

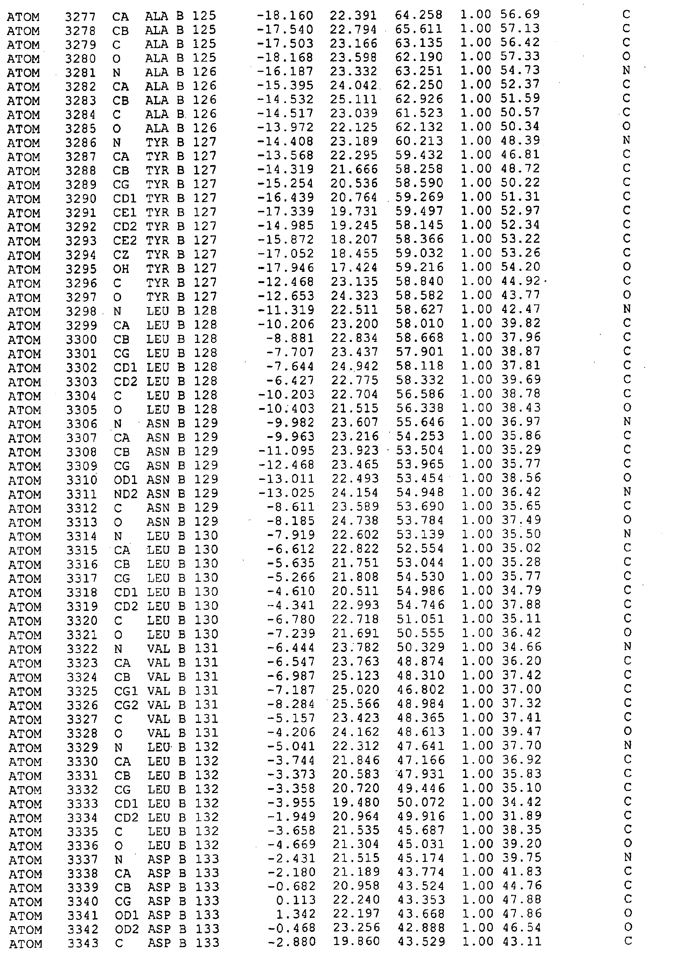

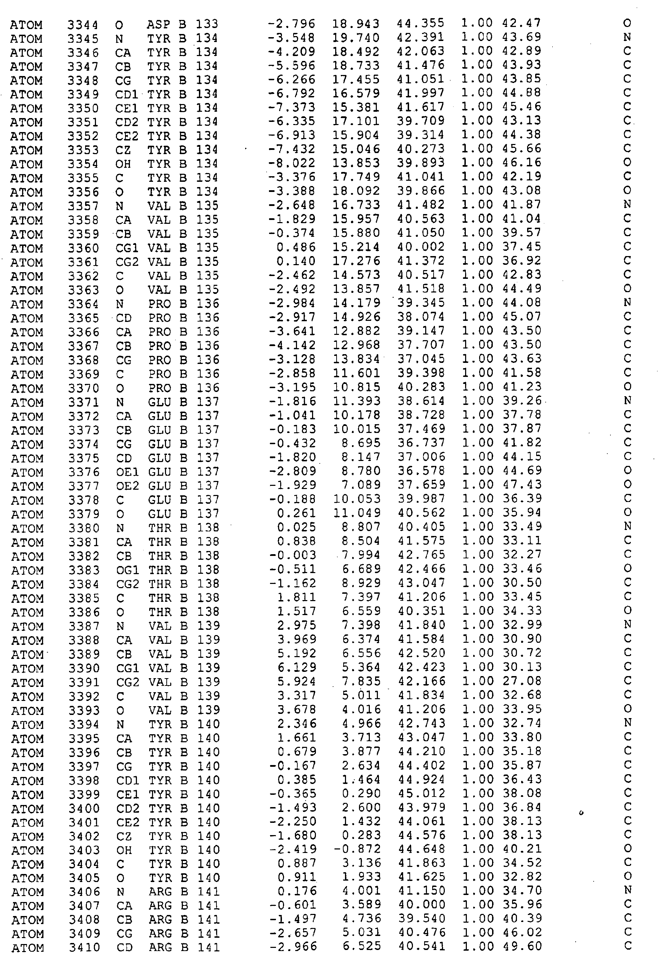

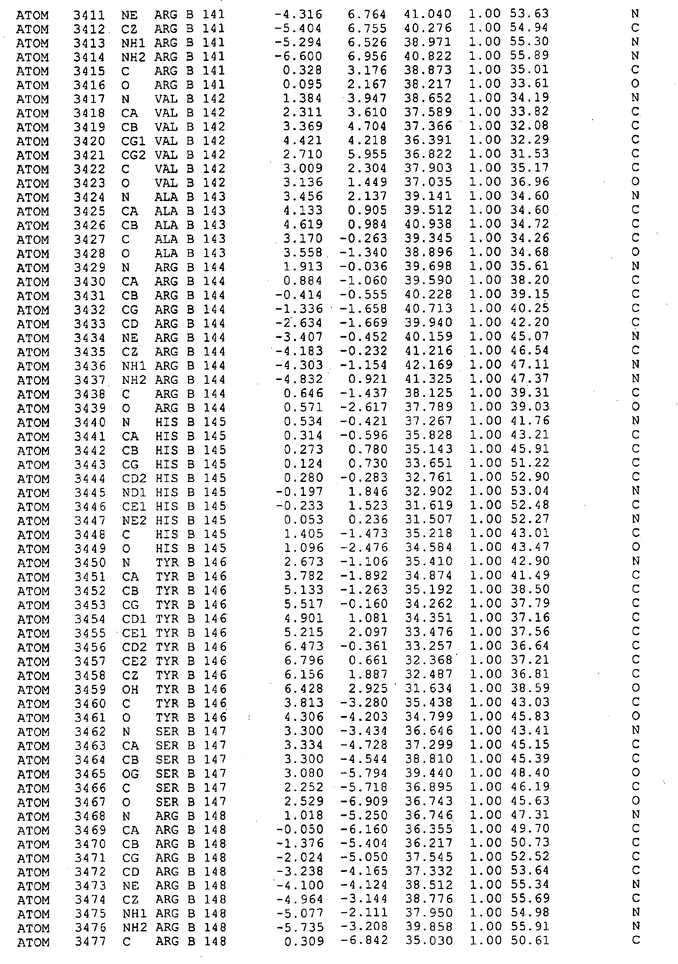

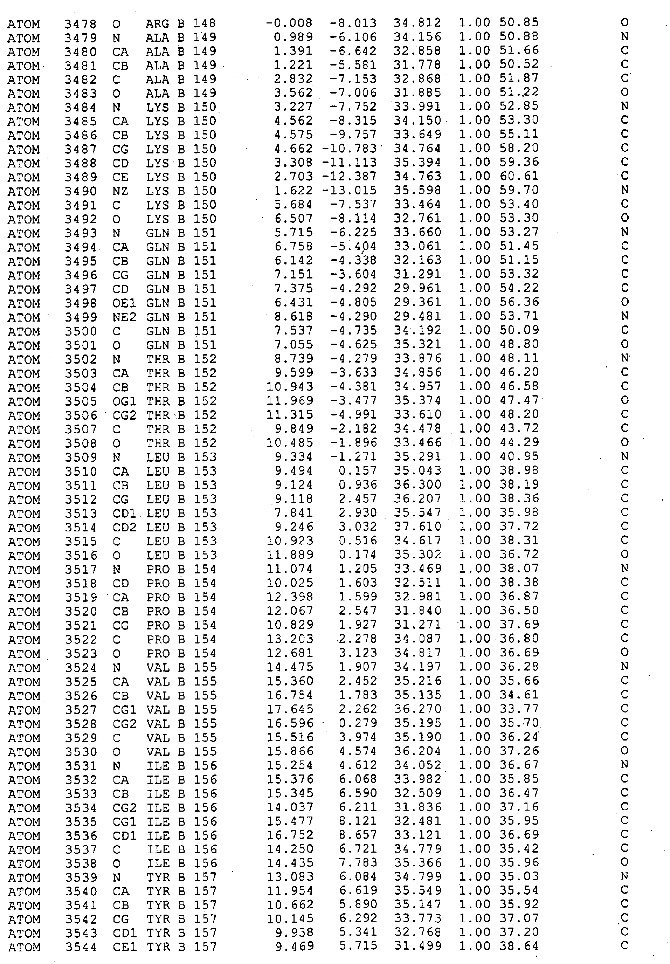

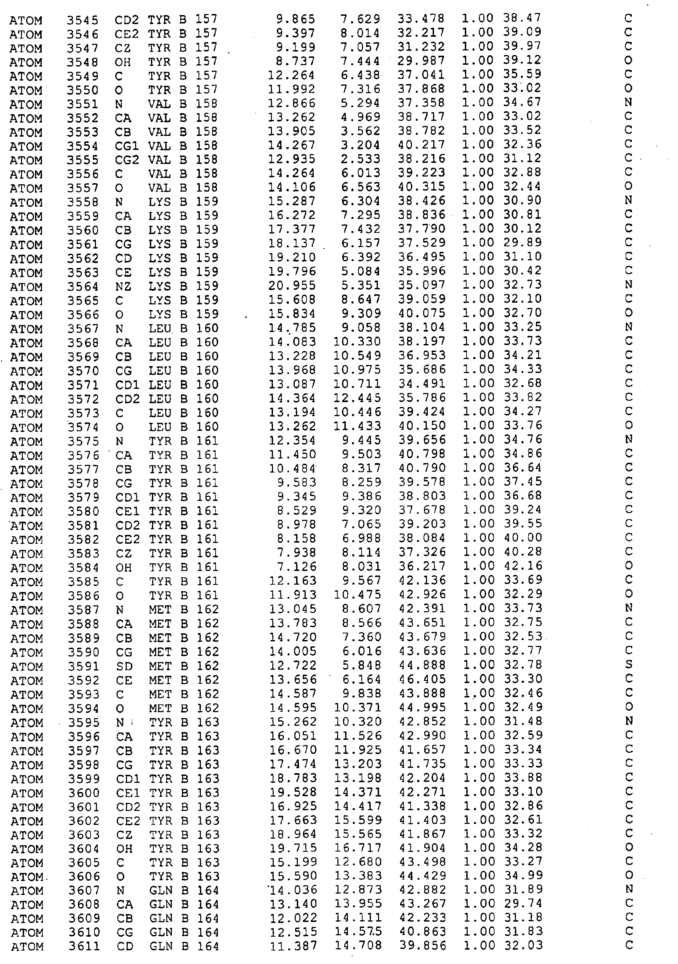

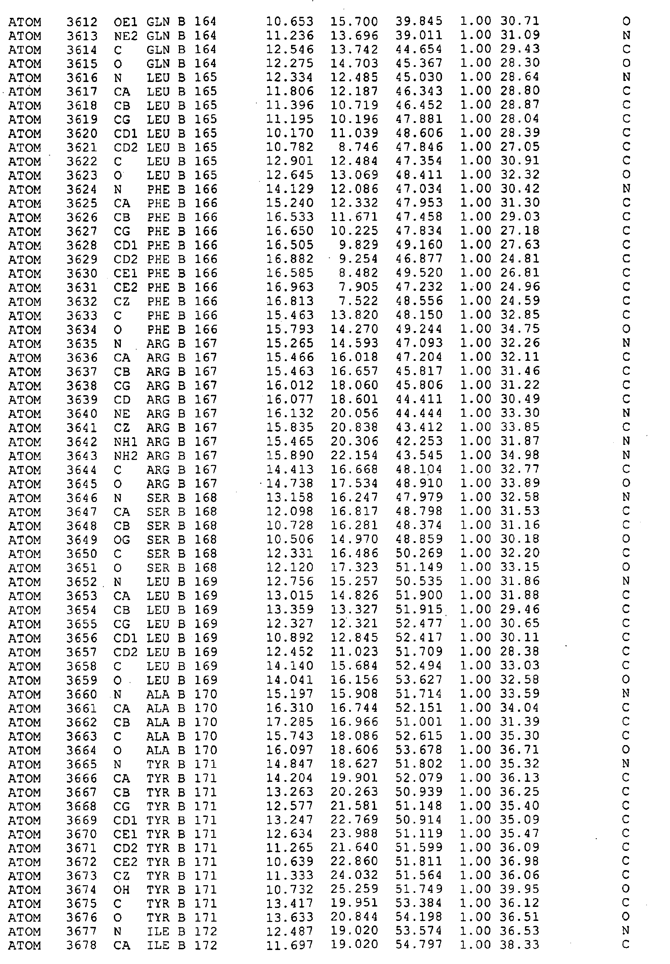

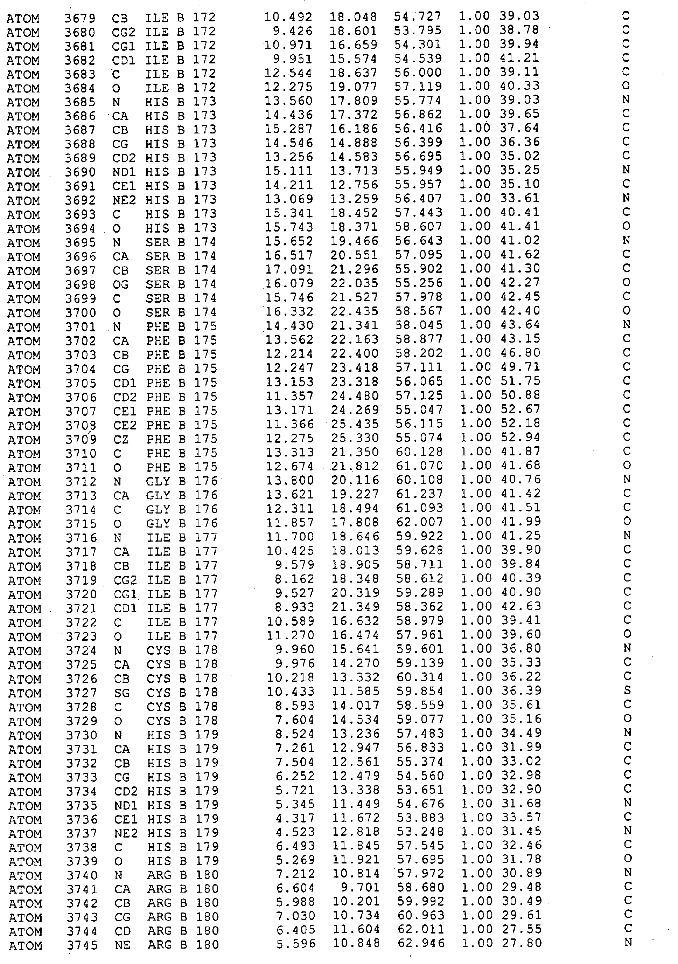

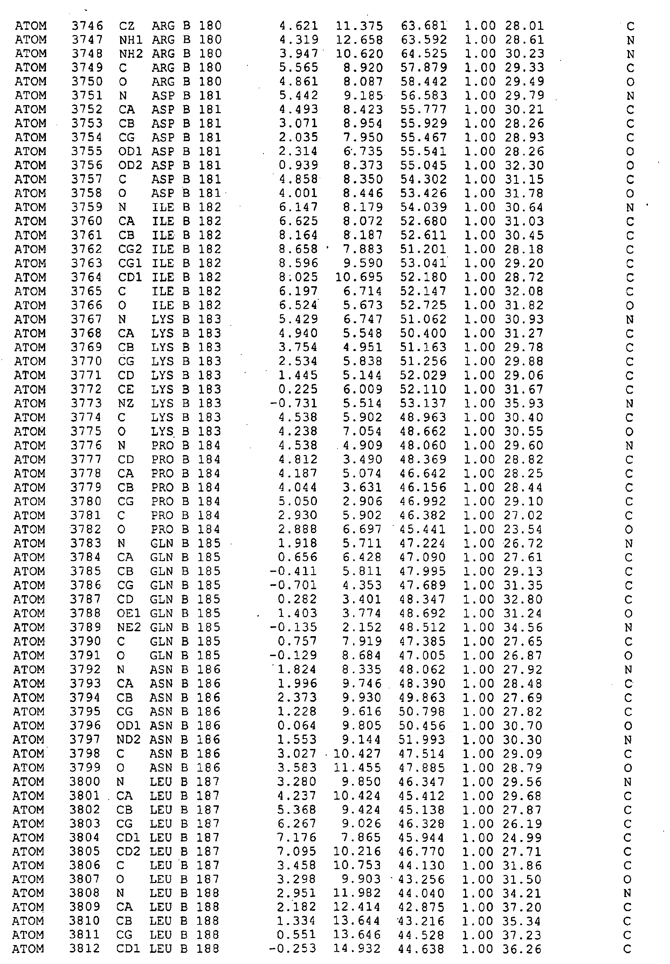

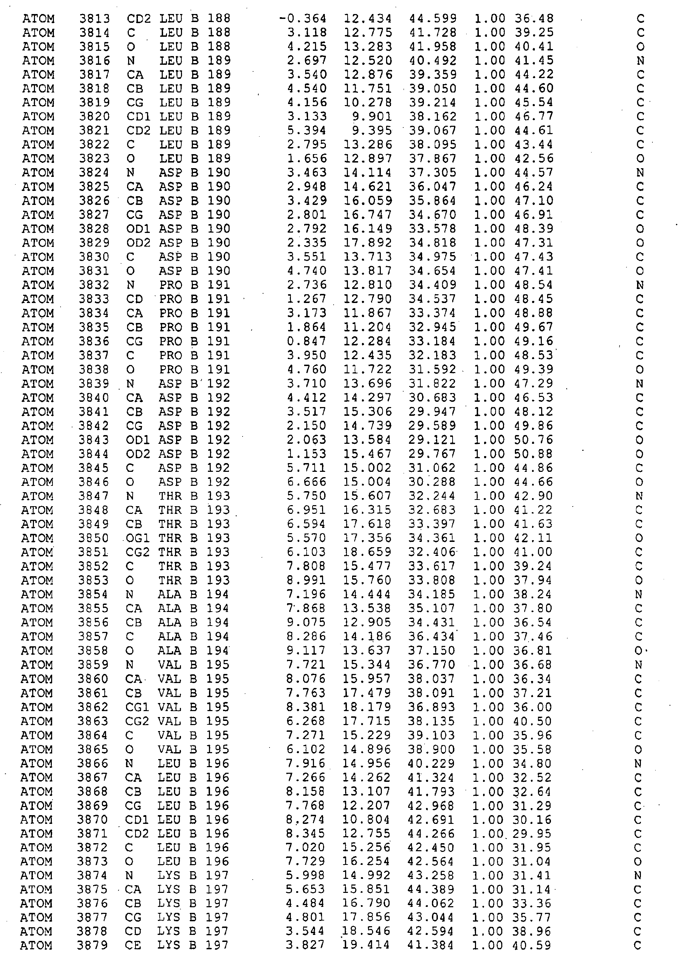

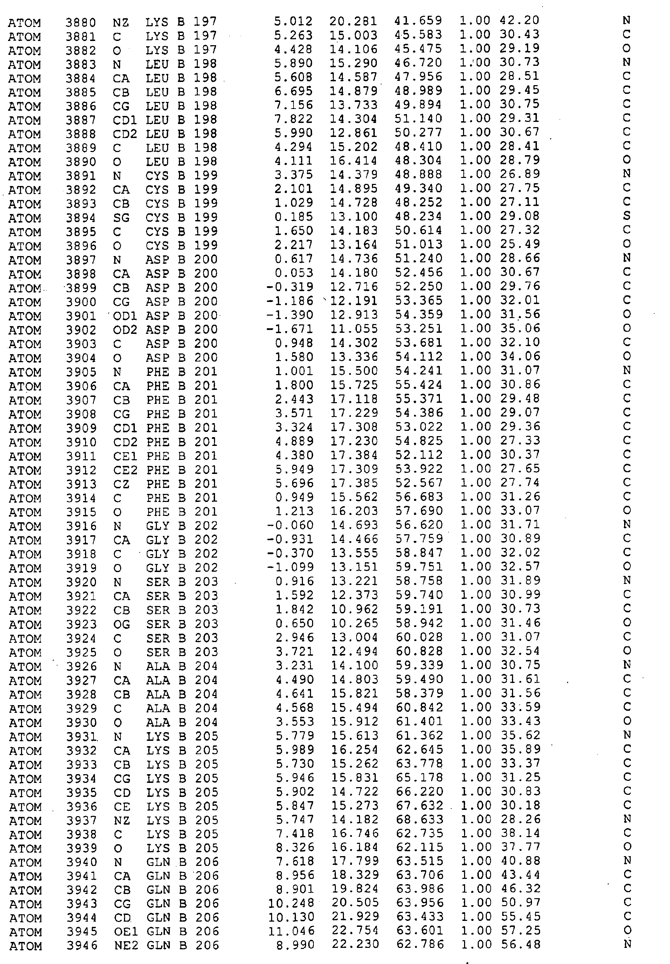

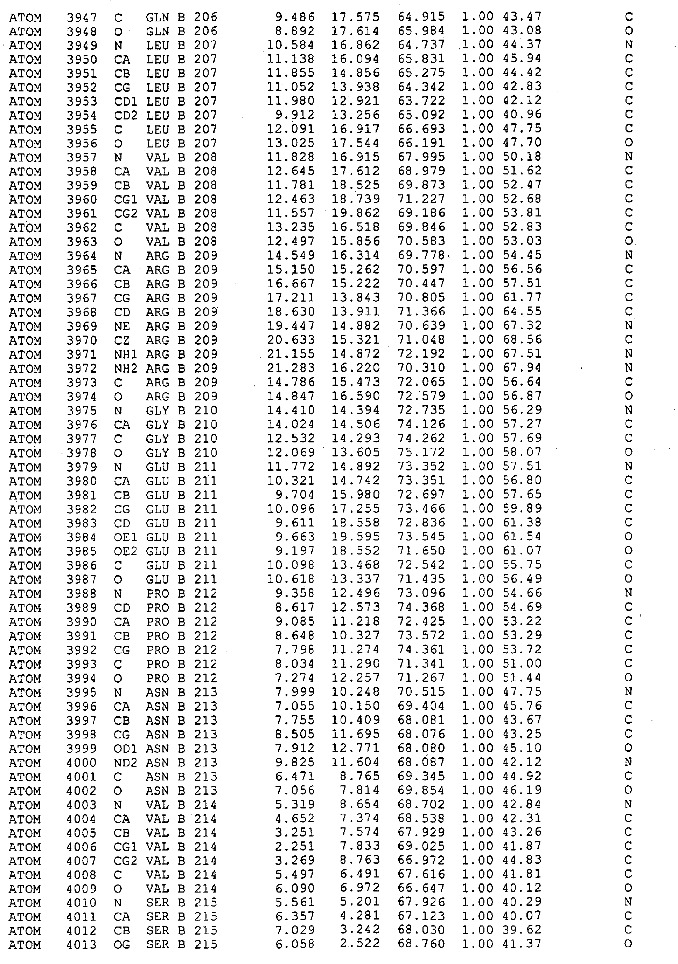

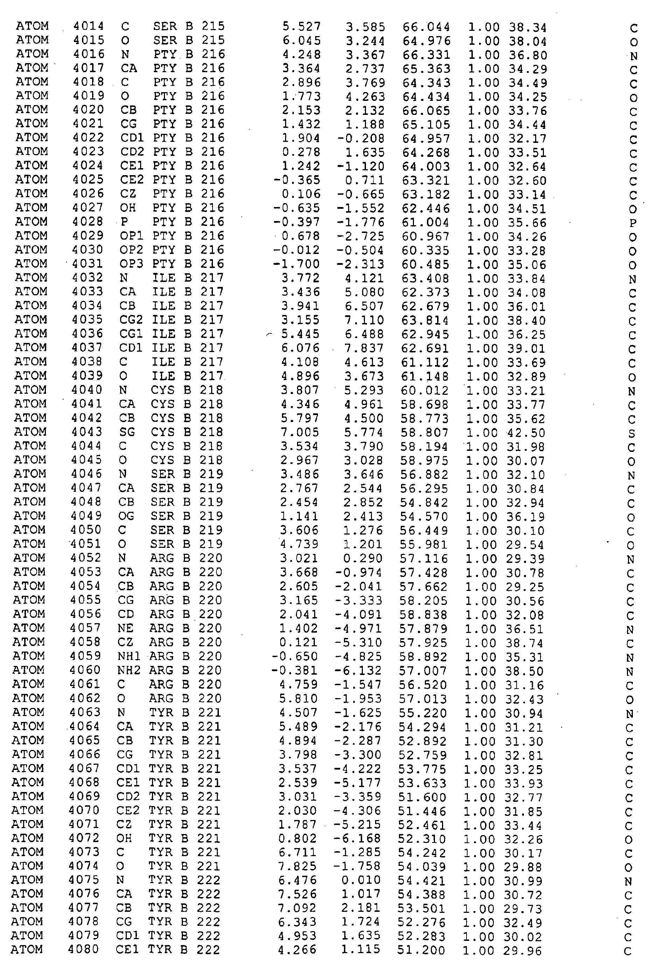

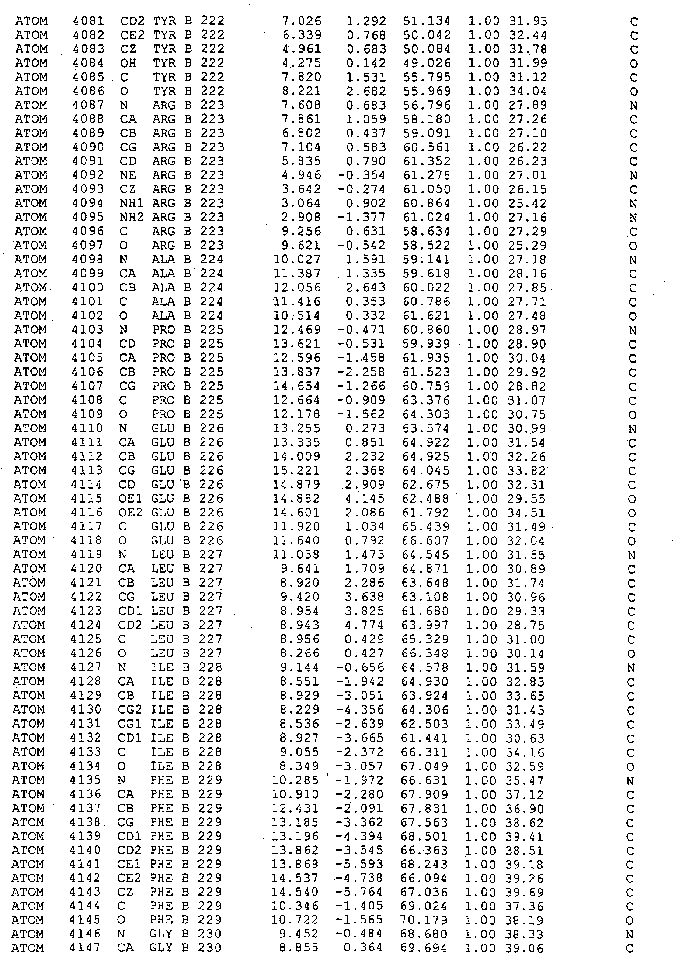

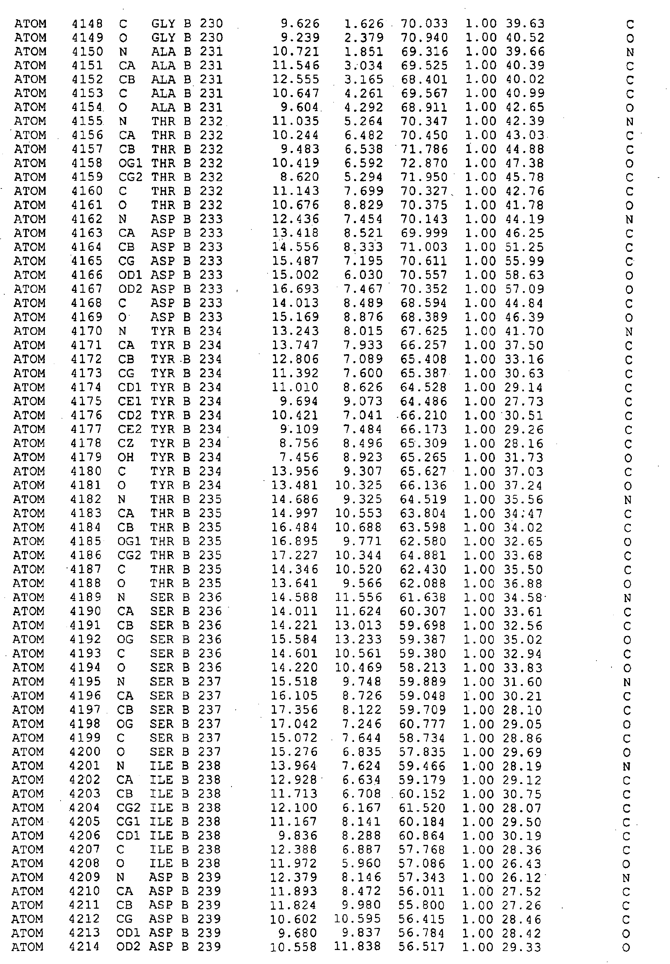

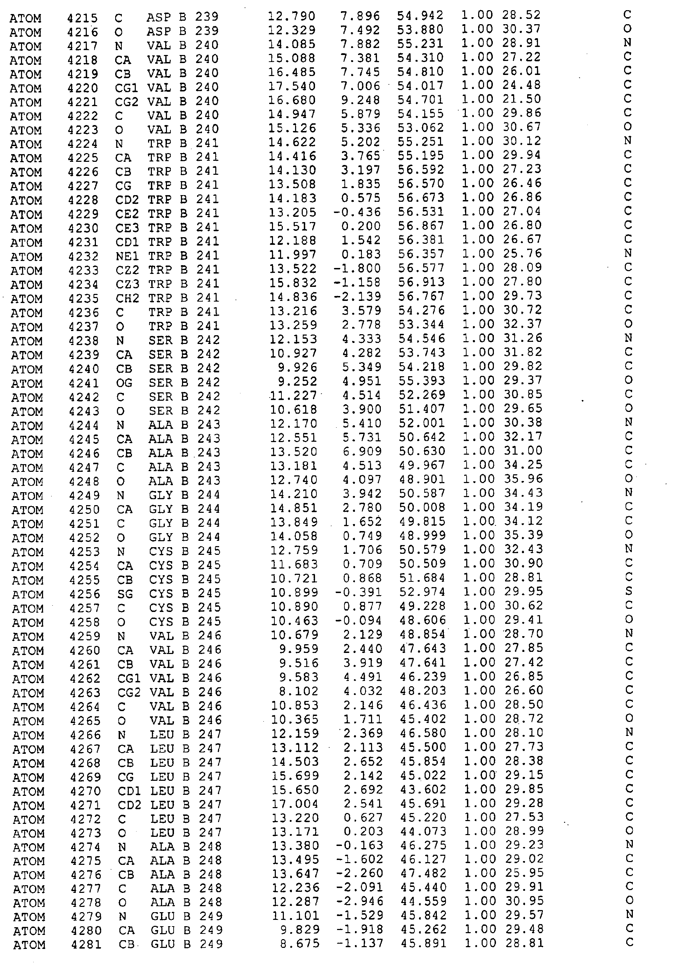

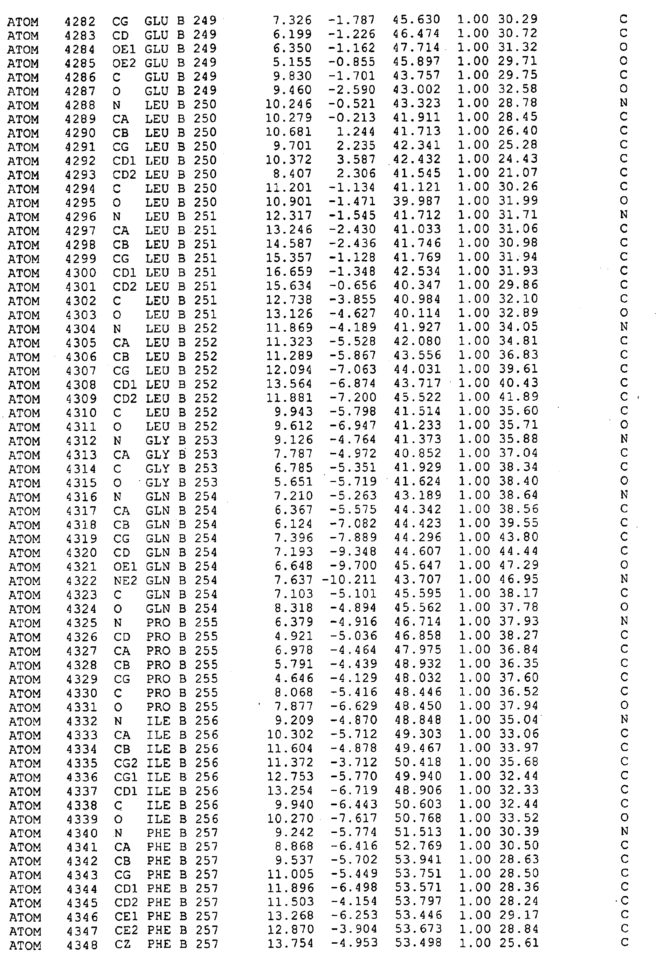

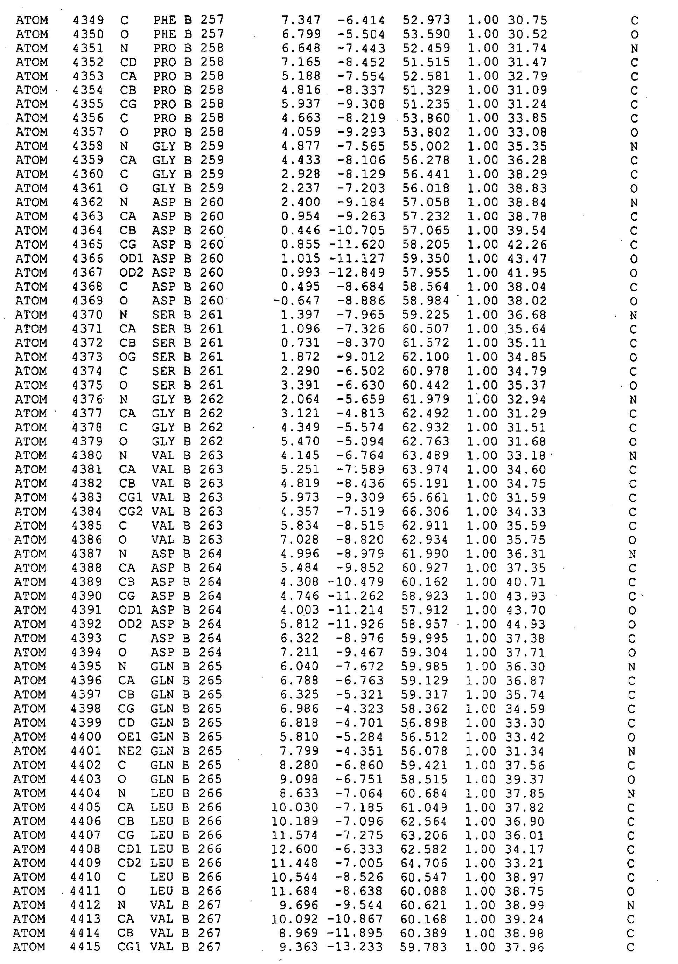

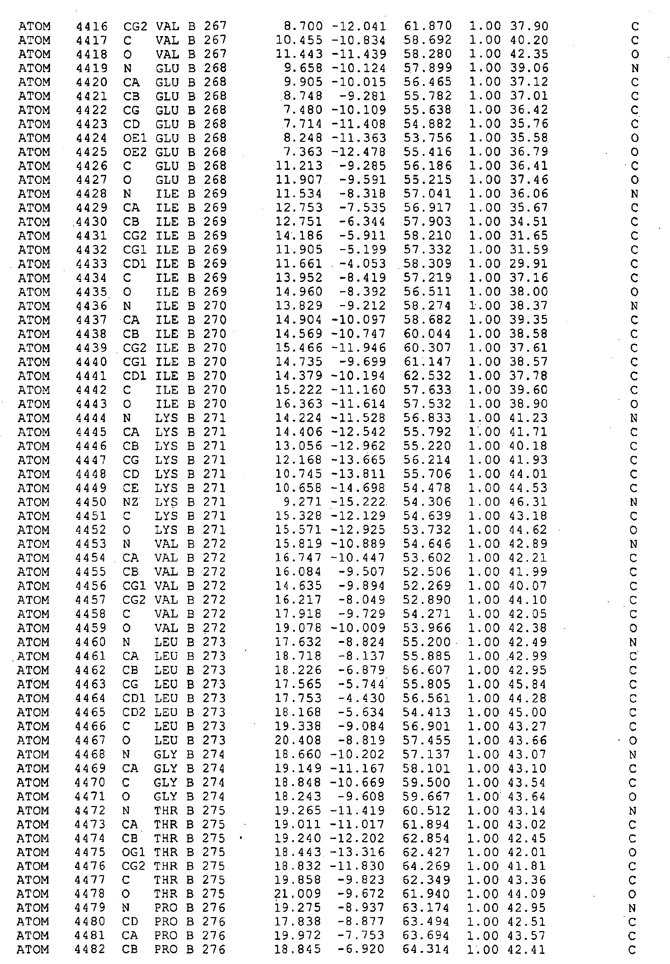

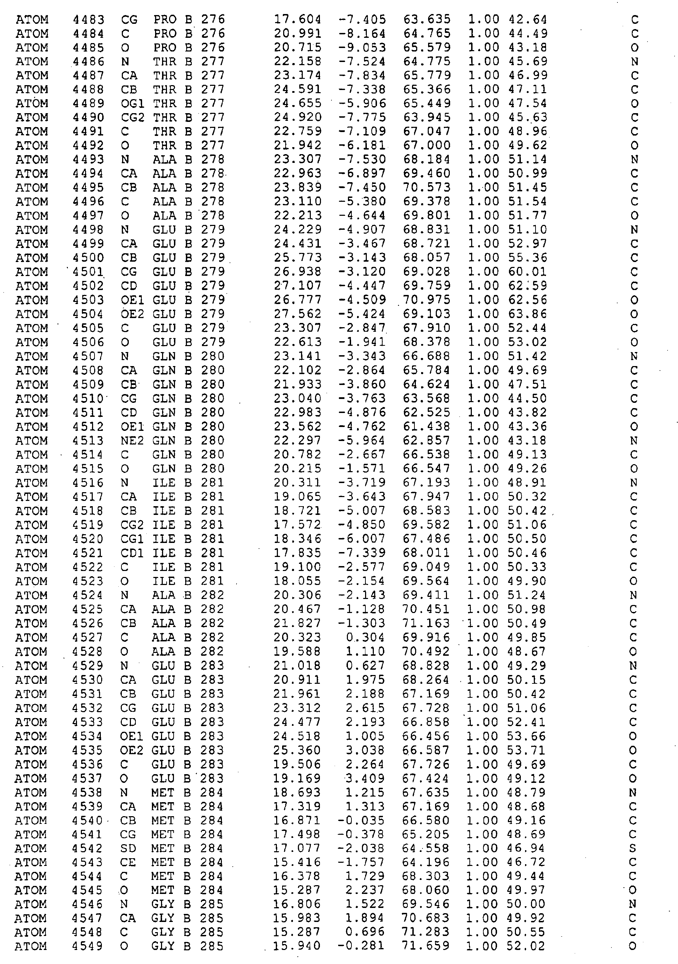

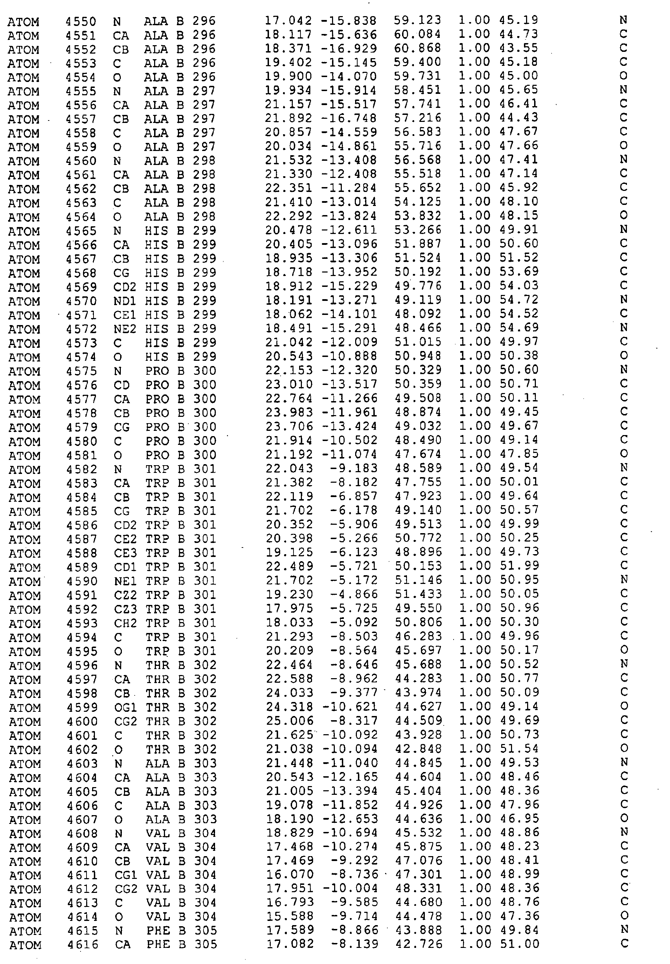

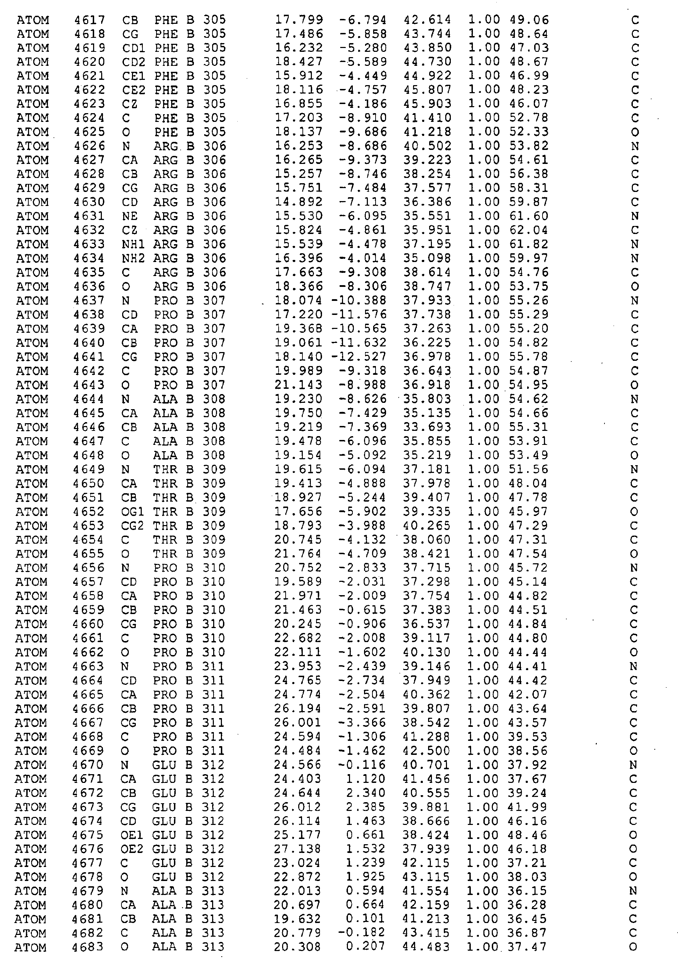

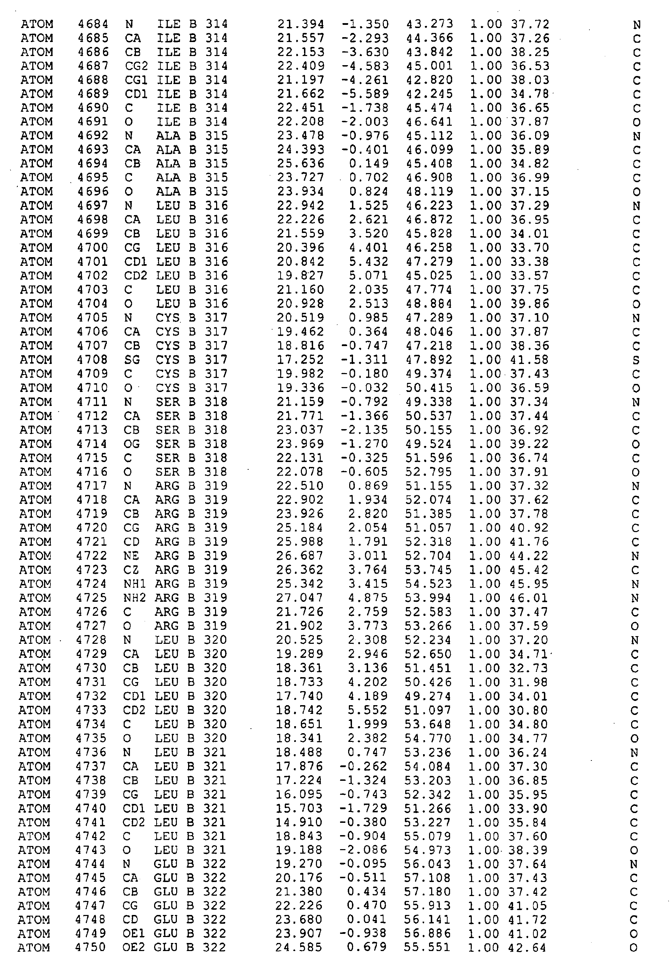

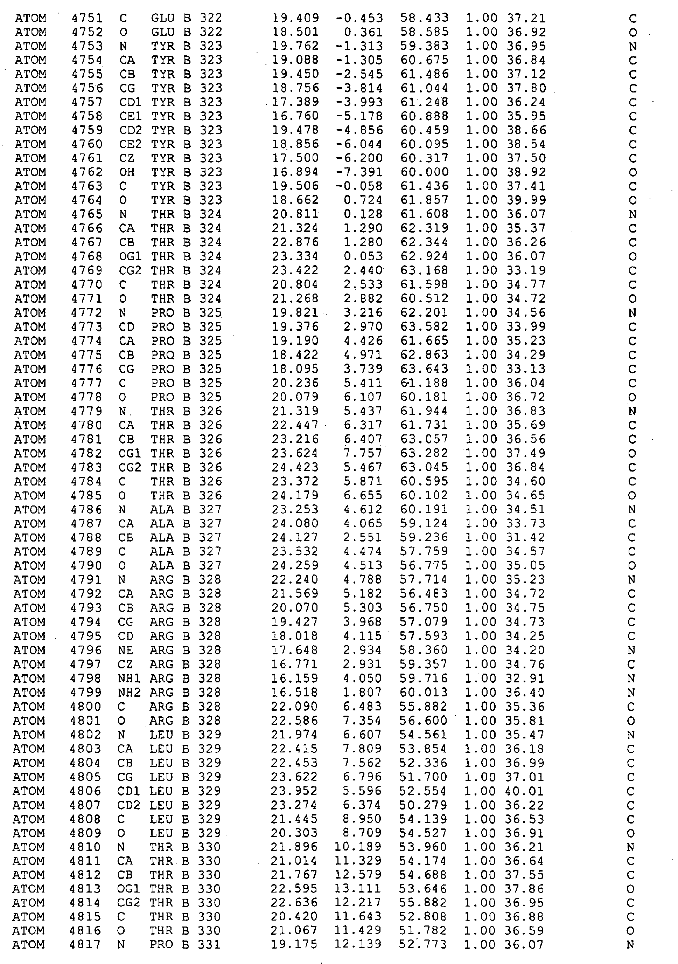

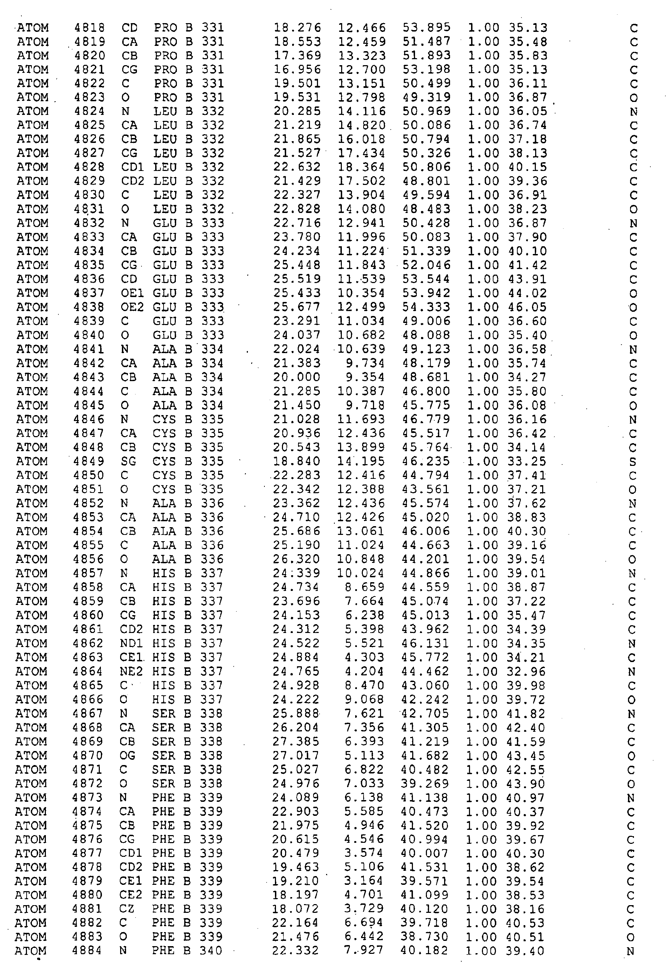

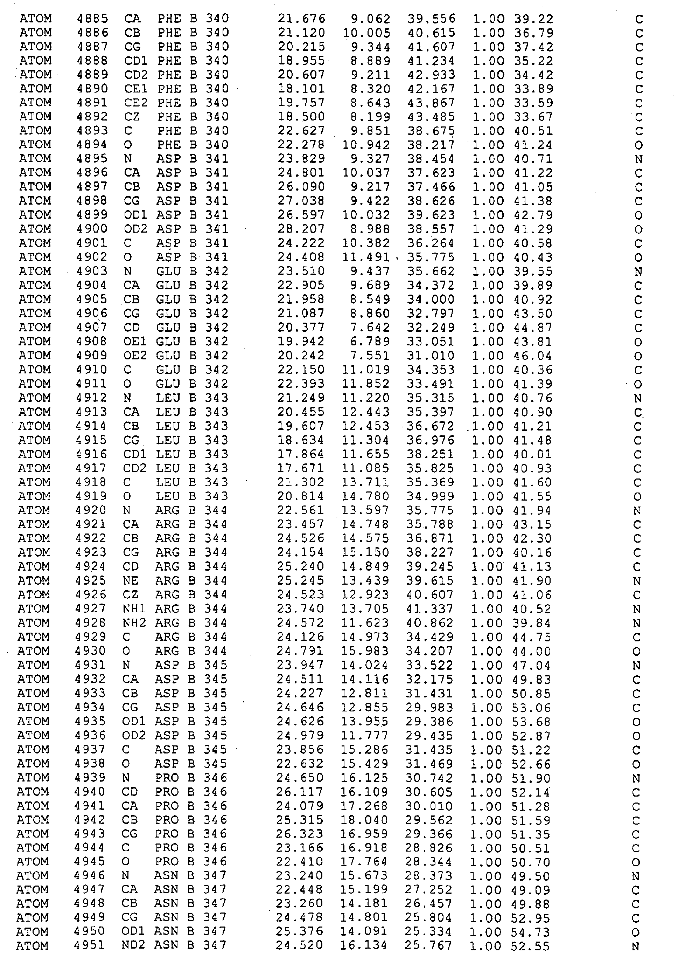

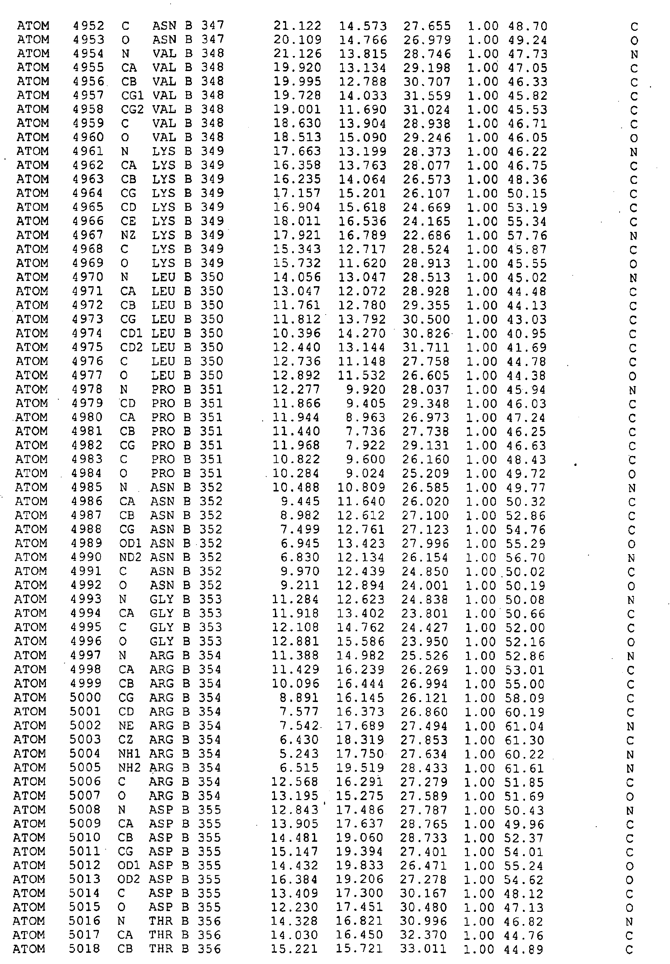

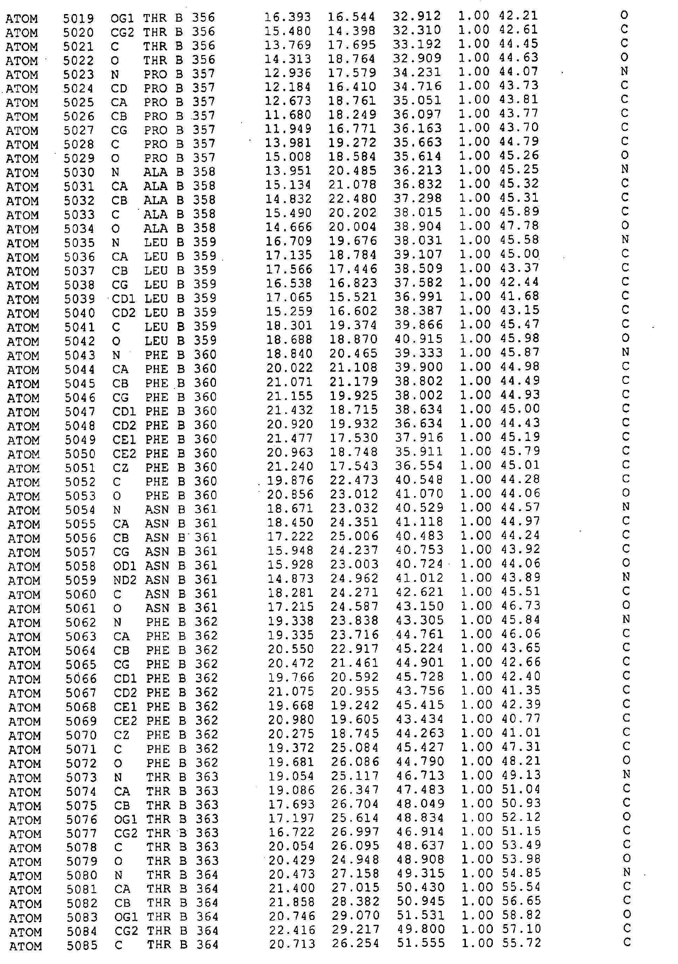

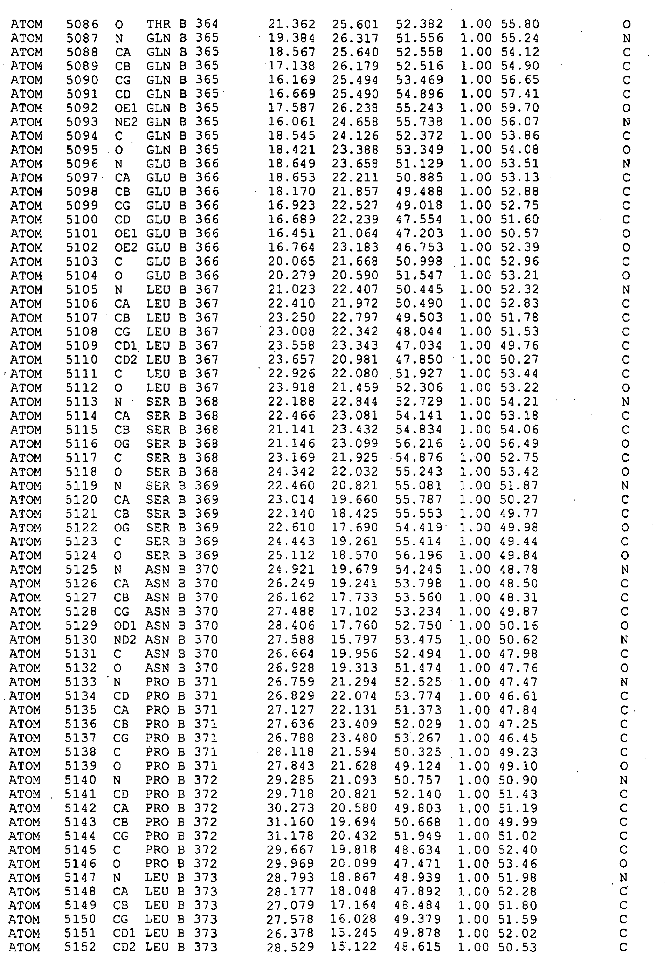

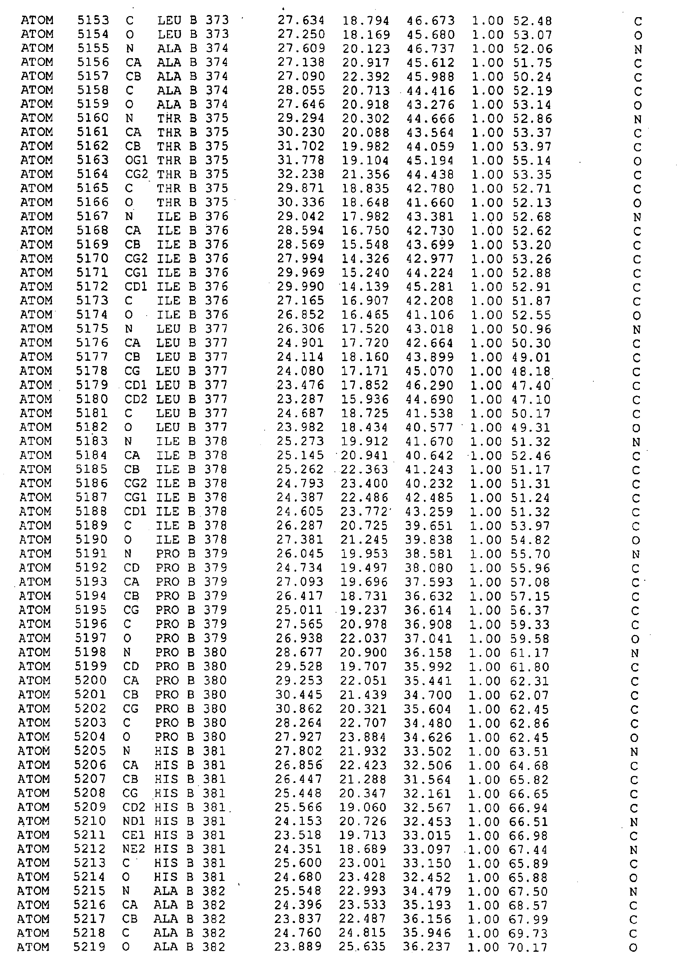

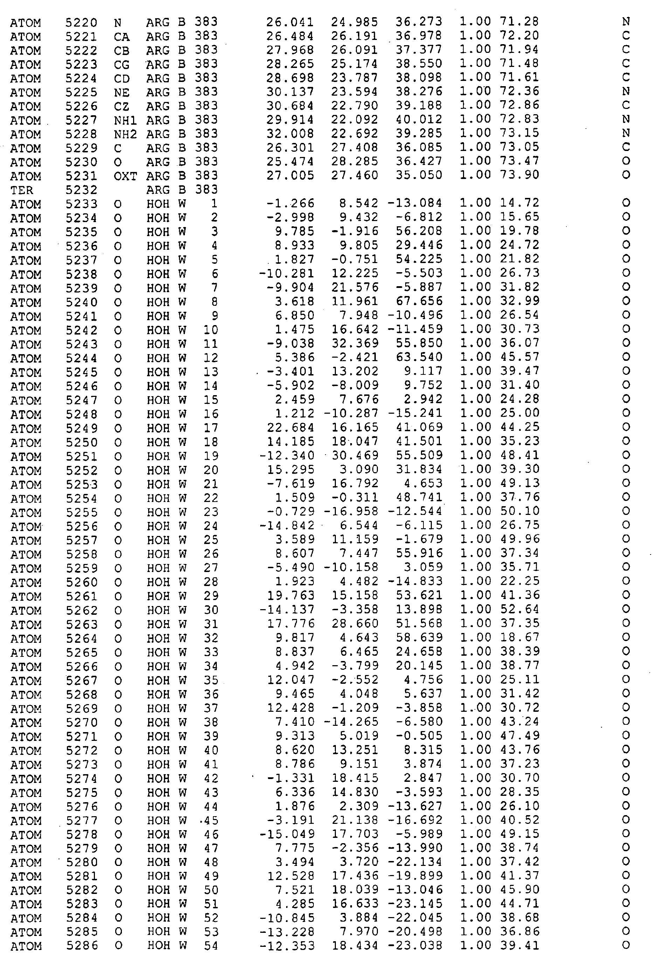

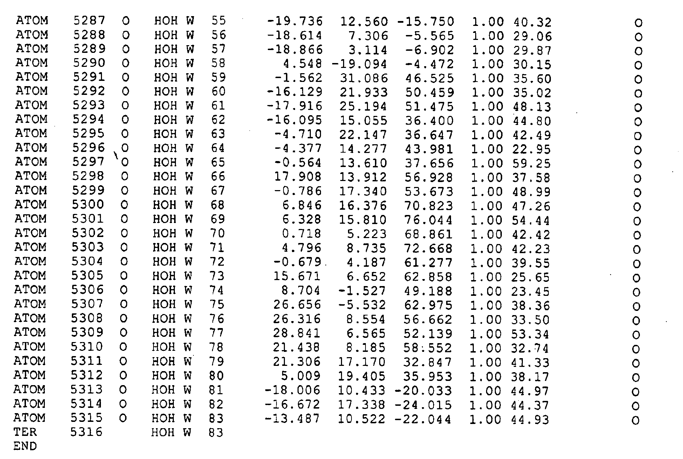

The following abbreviations are used in Figures 1-5:

"Atom type" refers to the element whose coordinates are measured. The first letter in the column defines the element.
"Res" refers to the amino acid residue in the molecular model.
"X, Y, Z" crystallographically define the atomic position of the element measured.
"B" is a thermal factor that measures movement of the atom around its atomic center.
"Occ" is an occupancy factor that refers to the fraction of the molecules in which each atom occupies the position specified by the coordinates. A value of "1" indicates that each atom has the same conformation, i.e., the same position, in all molecules of the crystal.

## DETAILED DESCRIPTION OF THE INVENTION

In order that the invention described herein may be more fully understood, the following detailed description is set forth.

Throughout the specification, the word "comprise" or variations such as "comprises" or "comprising" will be understood to imply the inclusion of a stated integer or groups of integers but not the exclusion of any other integer or groups of integers.

The following abbreviations are used throughout the application:

| | |
|---|---|
| A = Ala = Alanine | T = Thr = Threonine |
| V = Val = Valine | C = Cys = Cysteine |
| L = Leu = Leucine | Y = Tyr = Tyrosine |
| I = Ile = Isoleucine | N = Asn = Asparagine |
| P = Pro = Proline | Q = Gln = Glutamine |
| F = Phe = Phenylalanine | D = Asp = Aspartic Acid |
| W = Trp = Tryptophan | E = Glu = Glutamic Acid |
| M = Met = Methionine | K = Lys = Lysine |
| G = Gly = Glycine | R = Arg = Arginine |
| S = Ser = Serine | H= His = Histidine |
| pS= Phosphorylated Serine | pTy= Phosphorylated Tyrosine |

As used herein, the following definitions shall apply unless otherwise indicated. Also, combinations of substituents or variables are permissible only if such combinations result in stable compounds.

The term "about" when used in the context of RMSD values takes into consideration the standard error of the

RMSD value, which is ± 0.1 Å.

The term "active site" refers to the area in the protein kinase where the nucleotide binds. This site is located at the interface of the C-terminal α-helical and N-terminal β-strand domain, and is bordered by the glycine rich loop and the hinge (See, Xie. et al., Structure, 6, pp. 983-991 (1998), incorporated herein by reference).

The term "aliphatic" refers to straight chain or branched hydrocarbons that are completely saturated or that contain one or more units of unsaturation. For example, aliphatic groups include substituted or unsubstituted linear or branched alkyl, alkenyl and alkynyl groups. Unless indicated otherwise, the term "aliphatic" encompasses both substituted and unsubstituted hydrocarbons. The term "alkyl", used alone or as part of a larger moiety, refers to both straight and branched saturated chains containing one to twelve carbon atoms. The terms "alkenyl" and "alkynyl", used alone or as part of a larger moiety, encompass both straight and branched chains containing two to twelve carbon atoms and at least one unit of unsaturation. An alkenyl group contains at least one carbon-carbon double bond and an alkynyl group contains at least one carbon-carbon triple bond.

The term "correspond to" or "corresponding amino acids" when used in the context of amino acid residues that correspond to GSK-3β amino acids refers to particular amino acids or analogues thereof in a protein that correspond to amino acids in the GSK-3β protein. The corresponding amino acid may be an identical, mutated, chemically modified, conserved, conservatively substituted, functionally equivalent or homologous amino acid when compared to the GSK-3β amino acid to which it corresponds.

Methods for identifying a corresponding amino acid are known in the art and are based upon sequence, structural alignment, its functional position or a combination thereof as compared to the GSK-3β protein. For example, corresponding amino acids may be identified by superimposing the backbone atoms of the amino acids in GSK-3β and the protein using well known software applications, such as QUANTA (Molecular Simulations, Inc., San Diego, CA ©2000). The corresponding amino acids may also be identified using sequence alignment programs such as the "bestfit" program available from the Genetics Computer Group which uses the local homology algorithm described by Smith and Waterman in Advances in Applied Mathematics 2, 482 (1981), which is incorporated herein by reference.

The term "aryl", alone or in combination with other terms, refers to monocyclic or polycyclic aromatic carbon ring systems having five to fourteen members. Examples of aryl groups include, but are not limited to, phenyl (Ph), 1-naphthyl, 2-naphthyl, 1-anthracyl and 2-anthracyl. The term "aralkyl" refers to an alkyl group substituted by an aryl. Also explicitly included within the scope of the term "aralkyl" are alkenyl or alkynyl groups substituted by an aryl. Examples of aralkyl groups include benzyl and phenethyl. The term "aryl", "aryl group" or "aryl ring" also refers to rings that are optionally substituted, unless otherwise indicated.

The term "associating with" refers to a condition of proximity between a chemical entity or compound, or portions thereof, and a binding pocket or binding site on a protein. The association may be non-covalent -- wherein the juxtaposition is energetically favored by hydrogen bonding or van der Waals or electrostatic interactions -- or it may be covalent.

The term "ATP analogue" refers to a compound derived from Adenosine-5'-triphosphate(ATP). The analogue can be ADP, or non-hydralysable, for example, Adenylyl Imidodiphosphate (AMPPNP). AMPPNP can be in complex with Magnesium or Manganese ions.

The term "binding pocket" refers to a region of a molecule or molecular complex, that, as a result of its shape, favorably associates with another chemical entity or compound.

The term "biological sample" includes, without limitation, cell cultures or extracts thereof; biopsied material obtained from a mammal or extracts thereof; and blood, saliva, urine, feces, semen, tears, or other body fluids or extracts thereof.

The term "carbocylyl" or "carbocyclic", alone or in combination with any other term, refers to monocyclic or polycyclic non-aromatic carbon ring systems, which may contain a specified number of carbon atoms, preferably from 3 to 12 carbon atoms, which are completely saturated or which contain one or more units of unsaturation. A carbocyclic ring system may be monocyclic, bicyclic or tricyclic. A carbocylyl ring may be fused to another ring, such as an aryl ring or another carbocyclic ring. Examples of carbocyclic rings could include cyclohexyl, cyclopentyl, cyclobutyl, cyclopropyl, cyclohexenyl, cyclopentenyl, indanyl, tetrahydronaphthyl and the like. The term "carbocyclic" or "carbocylyl", whether saturated or unsaturated, also refers to rings that are optionally substituted unless indicated. The term "carbocyclic" or "carbocylyl" also encompasses hybrids of aliphatic and carbocyclic groups, such as (cycloalkyl)alkyl, (cycloalkenyl)alkyl and (cycloalkyl)alkenyl.

The term "chemically feasible or stable" refers to a compound structure that is sufficiently stable to allow manufacture and administration to a patient by methods known in the art. Typically, such compounds are stable at a temperature of 40˚C or less, in the absence of moisture or other chemically reactive conditions, for at least one week.

The term "chemical entity" refers to chemical compounds, complexes of at least two chemical compounds, and fragments of such compounds or complexes. The chemical entity can be, for example, a ligand, a substrate, nucleotide triphosphate, a nucleotide, an agonist, antagonist, inhibitor, antibody, peptide, protein or drug. In one embodiment, the chemical entity is selected from the group consisting of an ATP and an inhibitor for the active site. In one embodiment, the inhibitor is 4,5-Diphenyl-1H-pyrazolo[3,4-c]pyridazin-3-ylamine, (5-Methyl-2H-pyrazol-3-yl)-(2-pyridin-4-yl-quinazolin-4-yl)-amine, 4-(5-Methyl-2-phenylamino-pyrimidin-4-yl)-1H-pyrrole-2-carboxylic acid (2-hydroxy-1-phenyl-ethyl)-amide, (1H-Indazol-3-yl)-[2-(2-trifluoromethyl-phenyl)-quinazolin-4-yl]-amine and an ATP analogue such as MgAMP-PNP (adenylyl imidodiphosphate) or ADP. In one embodiment, the chemical entity is selected from the group consisting of a peptide substrate or inhibitor for the substrate binding groove.

The term "crystallization solution" refers to a solution that promotes crystallization of macromolecules. The crystallization solution may contain a precipitant, a buffer, salt, stabilizer, a polyionic agent, a detergent, a lanthanide ion or reducing agent. One of ordinary skilled in the art may adjust the components of the crystallization solution to find a condition suitable for the macromolecule of interest.

The term "conservative substitutions" refers to residues that are physically or functionally similar to the corresponding reference residues. That is, a conservative substitution and its reference residue have similar size, shape, electric charge, chemical properties including the ability to form covalent or hydrogen bonds, or the like. Preferred conservative substitutions are those fulfilling the criteria defined for an accepted point mutation in Dayhoff et al., Atlas of Protein Sequence and Structure, 5, pp. 345-352 (1978 & Supp.), which is incorporated herein by reference. Examples of conservative substitutions are substitutions including but not limited to the following groups: (a) valine, glycine; (b) glycine, alanine; (c) valine, isoleucine, leucine; (d) aspartic acid, glutamic acid; (e) asparagine, glutamine; (f) serine, threonine; (g) lysine, arginine, methionine; and (h) phenylalanine, tyrosine.

The term "complex" refers to a protein associated with a chemical entity.

The term "domain" refers to a structural unit of the GSK-3β protein or homologue. The domain can comprise a binding pocket, or a sequence or structural motif. In GSK-3β, the protein is separated into two domains, the N-terminal domain which is predominantly β strands and the C-terminal domain which is predominantly α helical.

The term "generating a three-dimensional structure" refers to plotting the structure coordinates in three-dimensional space. This can be achieved through commercially available software. The three-dimensional structure may be used to perform computer modeling, fitting operations, or displayed as a three-dimensional graphical representation.

The term "GSK-3β inhibitor-binding pocket" or "GSK-3β ATP-binding pocket" refers to a binding pocket of a molecule or molecular complex defined by the structure coordinates of a certain set of amino acid residues present in the GSK-3β structure, as described below. This binding pocket is in an area in the GSK-3β protein where the ATP or inhibitor for the active site binds.

The term "GSK-3β-like" refers to all or a portion of a molecule or molecular complex that has a commonality of shape to all or a portion of the GSK-3β protein. For example, in the GSK-3β-like inhibitor binding pocket, the commonality of shape is defined by a root mean square deviation of the structure coordinates of the backbone atoms between the amino acids in the GSK-3β-like inhibitor-binding pocket and the GSK-3β amino acids in the GSK-3β inhibitor-binding pocket (as set forth in any one of Figure 1-7). Depending on the set of GSK-3β amino acids that define the GSK-3β inhibitor-binding pocket, one skilled in the art would be able to locate the corresponding amino acids that define a GSK-3β-like inhibitor-binding pocket in a protein based on sequence or structural homology.

The term "GSK-3-mediated condition" or "state" refers to any disease or other deleterious condition or state in which GSK-3, in particular GSK-3, is known to play a role. Such diseases or conditions include, without limitation, diabetes, Alzheimer's disease, Huntington's Disease, Parkinson's Disease, AIDS-associated dementia, amyotrophic lateral sclerosis (AML), multiple sclerosis (MS), schizophrenia, cardiomycete hypertrophy, reperfusion/ischemia, and baldness.

The term "halogen" or "halo" means F, Cl, Br, or I.

The term "heteroatom" means N, O, or S and shall include any oxidized form of nitrogen and sulfur, such as N(O), S(O), $S(O)_2$ and the quaternized form of any basic nitrogen.

The term "heterocyclic" or "heterocyclyl" refers to non-aromatic saturated or unsaturated monocyclic or polycyclic ring systems containing one or more heteroatoms and with a ring size of three to fourteen. One having ordinary skill in the art will recognize that the maximum number of heteroatoms in a stable, chemically feasible heterocyclic ring is determined by the size of the ring, degree of unsaturation, and valence. In general, a heterocyclic ring may have one to four heteroatoms so long as the heterocyclic ring is chemically feasible and stable and may be fused to another ring, such as a carbocyclic, aryl or heteroaryl ring, or to another heterocyclic ring. A heterocyclic ring system may be monocyclic, bicyclic or tricyclic. Also included within the scope of within the scope of the term "heterocyclic" or "heterocyclyl", as used herein, is a group in which one or more carbocyclic rings are fused to a heteroaryl.

Examples of heterocyclic rings include, but are not limited to, 3-1H-benzimidazol-2-one, 3-1H-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, benzothiane, aziranyl, oxiranyl, azetidinyl, pyrrolinyl, dioxolanyl, imidazolinyl, imidazolidinyl, pyrazolinyl, pyrazolidinyl, pyranyl, dioxanyl, dithianyl, trithianyl, quinuclidinyl, oxepanyl, and thiepanyl. The term "heterocyclic" ring, whether saturated or unsaturated, also refers to rings that are optionally substituted, unless otherwise indicated.

The term "heteroaryl", alone or in combination with any other term, refers to monocyclic or polycyclic aromatic ring systems having five to fourteen members and one or more heteroatoms. One having ordinary skill in the art will recognize that the maximum number of heteroatoms in a stable, chemically feasible heteroaryl ring is determined by the size of the ring and valence. The term "heteroaralkyl" refers to an alkyl group substituted by a heteroaryl. Also explicitly included within the scope of the term "heteroaralkyl" are alkenyl or alkynyl groups substituted by a heteroaryl. In general, a heteroaryl ring may have one to four heteroatoms. Heteroaryl groups include, without limitation, 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, and 3-thienyl. The term "heteroaryl ring", "heteroaryl group", or "heteroaralkyl" also refers to rings that are optionally substituted.

Examples of fused polycyclic heteroaryl and aryl ring systems in which a carbocyclic aromatic ring or heteroaryl ring is fused to one or more other rings include, tetrahydronaphthyl, benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl, and the like.

An aryl, aralkyl, heteroaryl, or heteroaralkyl group may contain one or more independently selected substituents. Examples of suitable substituents on the unsaturated carbon atom of an aryl or heteroaryl group include halogen, $CF_3$, -R', -OR', -OH, -SH, -SR', protected OH (such as acyloxy), $-NO_2$, -CN, $-NH_2$, -NHR', $-N(R')_2$, -NHCOR', $-NHCONH_2$, -NHCONHR', $-NHCON(R')_2$, -NRCOR', $-NHCO_2H$, $-NHCO_2R'$, $-CO_2R'$, $-CO_2H$, -COR', $-CONH_2$, -CONHR', $-CON(R')_2$, $-S(O)_2H$, $-S(O)_2R'$, $-SO_2NH_2$, -S(O)H, -S(O)R', $-SO_2NHR'$, $-SO_2N(R')_2$, $-NHS(O)_2H$, or $-NHS(O)_2R'$, where R' is selected from H, aliphatic, carbocyclyl, heterocyclyl, aryl, aralkyl, heteroaryl, or heteroaralkyl and each R' is optionally substituted with halogen, nitro, cyano, amino, -NH-(unsubstituted aliphatic), -N-(unsubstituted aliphatic)$_2$, carboxy, carbamoyl, hydroxy, -O-(unsubstituted aliphatic), -SH, -S-(unsubstituted aliphatic), $CF_3$, $-SO_2NH_2$, unsubstituted aliphatic, unsubstituted carbocyclyl, unsubstituted heterocyclyl, unsubstituted aryl, unsubstituted aralkyl, unsubstituted heteroaryl, or unsubstituted heteroaralkyl.

An aliphatic group or a non-aromatic heterocyclic ring may contain one or more substituents. Examples of suitable substituents on the saturated carbon of an aliphatic group or of a non-aromatic heterocyclic ring include those listed above for the unsaturated carbon as well as the following: =O, =S, =NNHR', $=NN(R')_2$, =N-OR', =NNHCOR', $=NNHCO_2R'$, $=NNHSO_2R'$, =N-CN, or =NR', wherein R' is as defined above. Guided by this specification, the selection of suitable substituents is within the knowledge of one skilled in the art.

A substitutable nitrogen on a heteroaryl or a non-aromatic heterocyclic ring is optionally substituted. Suitable substituents on the nitrogen include R'', COR'', $S(O)_2R''$, and $CO_2R''$, where R'' is H, an aliphatic group or a substituted aliphatic group.

The term "motif" refers to a group of amino acids in the protein that defines a structural compartment or carries out a function in the protein, for example, catalysis, structural stabilization or phosphorylation. The motif may be conserved in sequence, structure and function when. The motif can be contiguous in primary sequence or three-dimensional space. Examples of a motif include but are not limited to SXXXS motif, phosphorylation lip or activation loop, the glycine-rich phosphate anchor loop, the catalytic loop, the DFG loop and the APE motif (See, Xie et al., Structure, 6, pp. 983-991 (1998)).

The term "homologue of GSK-3β" or "GSK-3β homologue" refers to a molecule that is homologous to GSK-3β by structure or sequence, but retains the kinase activity of GSK-3. In one embodiment, the homologue has at least 80%, 90% or 95% sequence homology to GSK-3β. The homologue can be GSK-3α, GSK-3β from another species, with conservative substitutions, conservative additions or deletions thereof; human GSK-3β with conservative substitutions, conservative additions or deletions. For example, the GSK-3β can be full length protein (amino acids 1-420 of SEQ ID NO: 1); a truncated protein with amino acids 7-420, 25-381, 37-381 of SEQ ID NO: 1; the full length protein with conservative substitutions; the truncated protein with conservative mutations.

The term "part of a binding pocket" refers to less than all of the amino acid residues that define the binding pocket. The structure coordinates of residues that constitute part of a binding pocket may be specific for defining the chemical environment of the binding pocket, or useful in designing fragments of an inhibitor that may interact with those residues. For example, the portion of residues may be key residues that play a role in ligand binding, or may be residues that are spatially related and define a three-dimensional compartment of the binding pocket. The residues may be contiguous or non-contiguous in primary sequence.

In one embodiment, part of the binding pocket is at least two amino acid residues. In one embodiment, part of the inhibitor-binding pocket is GSK-3β amino acids D133 and V135. In another embodiment, part of the inhibitor-binding pocket is GSK-3β amino acids K85, L132, D133 and V135. In another embodiment, part of the inhibitor-binding pocket is GSK-3β amino acids K85, M101, V110, L130 and L132. In another embodiment, part of the inhibitor-binding pocket is GSK-3β amino acids I62, V135, P136, T138 and L188. In another embodiment, part of the inhibitor-binding pocket is GSK-3β amino acids V70, V110, L188 and C199. In another embodiment, part of the inhibitor-binding pocket is GSK-3β amino acids F67, V70, Q185 and C199. In one embodiment, part of a substrate binding pocket is GSK-3β amino acids D90, K91, R92, F93, K94. In another embodiment, part of the substrate binding pocket is GSK-3β amino acids

R96, R180, K205, N213 and Y234. In another embodiment, part of the substrate binding pocket is GSK-3β amino acids R96, R180 and K205. In another embodiment, part of the substrate binding pocket is GSK-3β amino acids S66, F67 and F93. In another embodiment, part of the substrate binding pocket is GSK-3β amino acids Y216, I217, C218, S219, R220 and R223.

The term "part of a GSK-3β protein" refers to less than all of the amino acid residues of a GSK-3β protein. In one embodiment, part of a GSK-3β protein defines the binding pockets, domains or motifs of the protein. The structure coordinates of residues that constitute part of a GSK-3β protein may be specific for defining the chemical environment of the protein, or useful in designing fragments of an inhibitor that may interact with those residues. The portion of residues may also be residues that are spatially related and define a three-dimensional compartment of a binding pocket, motif or domain. The residues maybe contiguous or non-contiguous in primary sequence. For example, the portion of residues may be key residues that play a role in ligand or substrate binding, catalysis or structural stabilization.

The term "pharmaceutically acceptable carrier, adjuvant, or vehicle" refers to a non-toxic carrier, adjuvant, or vehicle that may be administered to a patient, together with a compound of this invention, and which does not destroy the pharmacological activity thereof.

The term "patient" includes human and veterinary subjects.

The term "peptide comprising a phosphorylation sequence" refers to a peptide comprising the ZXXXY motif. Z can be serine or threonine. Y can be serine, threonine or valine. X can be any amino acid residue. Z or Y can be phosphorylated or unphosphorylated. The phosphorylation of Y facilitates the phosphorylation of X by GSK-3β. Examples of peptide substrates comprising a phosphorylation sequence include but are not limited to, ASVPPS, PSPSLS, LSRHSS, SSPHQS, DSRAGS, LSRRPS, PTPPPT, PTPVPS, KSPVVS, VSGDTS, QSYLDS, DSGIHS, HSGATT, TTTAPS, TSANDS, DSEQQS, SSPLPS, PSSPLS and CTPTDV.

The term "pharmaceutically acceptable derivative" or "prodrug" means any pharmaceutically acceptable salt, ester, salt of an ester or other derivative of a compound of this invention which, upon administration to a recipient, is capable of providing, either directly or indirectly, a compound of this invention or an inhibitorily active metabolite or residue thereof. Particularly favored derivatives or prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

Pharmaceutically acceptable prodrugs of the compounds of this invention include, without limitation, esters, amino acid esters, phosphate esters, metal salts and sulfonate esters.

Pharmaceutically acceptable salts of the compounds of this invention include those derived from pharmaceutically acceptable inorganic and organic acids and bases. Examples of suitable acid salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, formate, fumarate, glucoheptanoate, glycerophosphate, glycolate, hemisulfate, heptanoate, hexanoate, hydrochloride, hydrobromide, hydroiodide, 2-hydroxyethanesulfonate, lactate, maleate, malonate, methanesulfonate, 2-naphthalenesulfonate, nicotinate, nitrate, oxalate, palmoate, pectinate, persulfate, 3-phenylpropionate, phosphate, picrate, pivalate, propionate, salicylate, succinate, sulfate, tartrate, thiocyanate, tosylate and undecanoate. Other acids, such as oxalic, while not in themselves pharmaceutically acceptable, may be employed in the preparation of salts useful as intermediates in obtaining the compounds of the invention and their pharmaceutically acceptable acid addition salts.

Salts derived from appropriate bases include alkali metal (e.g., sodium and potassium), alkaline earth metal (e.g., magnesium), ammonium and $N^+(C_{1-4}\ alkyl)_4$ salts. This invention also envisions the quaternization of any basic nitrogen-containing groups of the compounds disclosed herein. Water or oil-soluble or dispersible products may be obtained by such quaternization.

The term "protein kinase-mediated condition" or "state" refers to any disease or other deleterious condition or state in which a protein kinase is known to play a role. Such conditions include, without limitation, autoimmune diseases, inflammatory diseases, metabolic, neurological and neurodegenerative diseases, cardiovasclular diseases, allergy and asthma.

The term "root mean square deviation" or "RMSD" refers to the square root of the arithmetic mean of the squares of the deviations from the mean. It is a way to express the deviation or variation from a trend or object. For purposes of this invention, the "root mean square deviation" defines the variation in the backbone of a protein from the backbone of GSK-3β or a binding pocket portion thereof, as defined by the structure coordinates of GSK-3β described herein. It would be readily apparent to those skilled in the art that the calculation of RMSD involves standard error.

The term "soaked" refers to a process in which the crystal is transferred to a solution containing the compound of interest.

The term "structure coordinates" refers to Cartesian coordinates derived from mathematical equations related to the patterns obtained on diffraction of a monochromatic beam of X-rays by the atoms (scattering centers) of a protein or protein complex in crystal form. The diffraction data are used to calculate an electron density map of the repeating

unit of the crystal. The electron density maps are then used to establish the positions of the individual atoms of the enzyme or enzyme complex.

The term "substantially all of a GSK-3β binding pocket" or "substantially all of a GSK-3β protein" refers to all or almost all of the amino acids in the GSK-3β binding pocket or protein. For example, substantially all of a GSK-3β binding pocket can be 100%, 95%, 90%, 80%, 70% of the residues defining the GSK-3β binding pocket or protein.

The term "substrate binding groove" refers to an area in a protein kinase where the substrate binds. The substrate binding groove is located at the interface of the β-strand and α-helical domain, and positioned between the activation loop and β-strand domain. Examples of substrates include but are not limited to glycogen synthase, β-catenin, elongation initiation factor 2B ε subunit, cAMP-responsive element binding protein, CCAAT/enhancer binding protein α, microtuble associated protein Tau, axin, Dd-STATa and Cyclin D1.

The term "substrate binding pocket" refers to a binding pocket of a molecule or molecular complex defined by the structure coordinates of a certain set of amino' acid residues present in the GSK-3β structure, as described below. This binding pocket is in an area in the GSK-3β protein where the substrate binding groove is located.

The term "sufficiently homologous to GSK-3β" refers to a protein that has a sequence homology of at least 20% compared to GSK-3β protein. In one embodiment, the sequence homology is at least 40%.

## Inhibitors of GSK-3

One object of the instant invention is to provide compounds having formula (I):

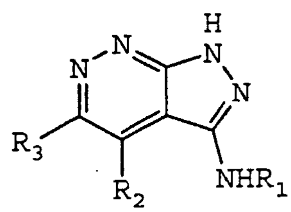

I

or a pharmaceutically acceptable derivative thereof, wherein:

$R_1$ is selected from H, aliphatic, RC(O)-, $RS(O)_n$-, ROC(O)-, carbocyclyl, heterocyclyl, aryl, aralkyl, heteroaryl, or heteroaralkyl; wherein said aliphatic, carbocyclyl, heterocyclyl, aryl, aralkyl, heteroaryl, or heteroaralkyl is optionally substituted;

$R_2$ and $R_3$ are each independently selected from H, aliphatic, carbocyclyl, heterocyclyl, aryl, aralkyl, heteroaryl, heteroaralkyl, $-N(R)_2$, -NRCOR, $-NRCO_2R$, $-NRSO_2R$, $-S(O)_nR$, $-SO_2N(R)_2$, -SR, -OR, $-CF_3$, halo, $-NO_2$, -CN, -C(O)R, $-CO_2R$, -OC(O)R, $-CON(R)_2$, or $-OC(O)N(R)_2$, wherein said aliphatic, carbocyclyl, heterocyclyl, aryl, aralkyl, heteroaryl, or heteroaralkyl is optionally substituted; or $R_2$ and $R_3$ taken together with the intervening atoms optionally form a five- to nine-membered ring that is fused to the pyridazinyl ring of formula I, said fused ring having 0-2 heteroatoms;

each R is independently selected from H, aliphatic, carbocyclyl, heterocyclyl, aryl, aralkyl, heteroaryl, or heteroaralkyl, wherein each member of R except H is optionally substituted; and

n is 1 or 2;

provided that when $R_1$ is H, $R_2$ and $R_3$ are not both unsubstituted phenyl; and when $R_1$ is H, $R_2$ and $R_3$ are other than H, halogen, or an unsubstituted alkyl.

It will be apparent to one skilled in the art that certain compounds of this invention may exist in tautomeric forms, all such tautomeric forms of the compounds being within the scope of the invention.

Unless otherwise stated, structures depicted herein are also meant to include all stereochemical forms of the structure; i.e., the R and S configurations for each asymmetric center. Therefore, single stereochemical isomers as well as enantibmeric and diastereomeric mixtures of the present compounds are within the scope of the invention. Unless otherwise stated, structures depicted herein are also meant to include compounds which differ only in the presence of one or more isotopically enriched atoms. For example, compounds having the present structures except for the replacement of a hydrogen by a deuterium or tritium, or the replacement of a carbon by a $^{13}C$- or $^{14}C$-enriched carbon are within the scope of this invention.

In a preferred embodiment of the invention, $R_1$ is H, RC(O)-, or aralkyl, wherein R is as defined above.
In a more preferred embodiment, $R_1$ is H, aliphatic-C(O)-, aryl-C(O)-, or aralkyl. In an even more preferred embodiment, $R_1$ is H, $CH_3C(O)$-, PhC(O)-, or $PhCH_2$-.

In another preferred embodiment, $R_2$ and $R_3$ are independently H, aryl or heteroaryl. In another preferred embodiment, $R_2$ and $R_3$ are independently H, aryl, carbocyclyl, heterocyclyl, or heteroaryl. In another embodiment, $R_2$ and $R_3$ are independently aryl, carbocyclyl, heterocyclyl, or heteroaryl. Preferably, $R_2$ and $R_3$ are independently H, phenyl, naphthyl, pyridyl, thienyl, furanyl, pyrimidinyl, benzodioxolyl, or cyclohexyl, any of which except H is optionally substituted. More preferably, $R_2$ and $R_3$ are independently phenyl, naphthyl, pyridyl, thienyl, furanyl, pyrimidinyl, benzodioxolyl, or cyclohexyl, any of which is optionally substituted. Even more preferably, the substituents on phenyl, naphthyl, pyridyl, thienyl, furanyl, pyrimidinyl, benzodioxolyl, or cyclohexyl are selected from halo, alkyl, -CN, $-NO_2$, $-SO_2NH_2$, $-SO_2NH$-(alkyl), $-SO_2N(alkyl)_2$, -O-alkyl, $-NH_2$, -N-alkyl, $-N(alkyl)_2$, $-CONH_2$, -CONH(alkyl), $-CONH(alkyl)_2$, -O-phenyl, or -S-alkyl.

In another preferred embodiment, when $R_1$ is a large group, $R_2$ is a small group. A small group refers to hydrogen or a moiety that contains 3 carbons or less, such as methyl, ethyl, or propyl. A large group refers to a moiety that contains 4 or more carbons.

In another preferred embodiment, $R_1$ is H, and $R_2$ and $R_3$ are independently H or an optionally substituted phenyl.

A more preferred embodiment of the invention is shown in formula Ia:

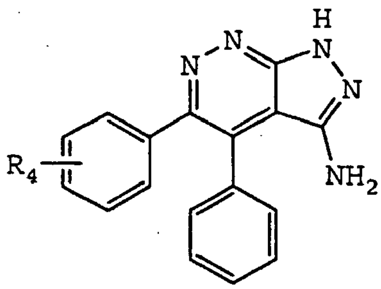

(Ia),

wherein $R_4$ is halo. In an even more preferred embodiment, $R_4$ is F.

Representative examples of compounds of the present invention are shown below in Table 1.

TABLE 1

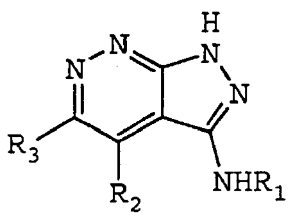

| Compound No. | $R_1$ | $R_2$ | $R_3$ |
|---|---|---|---|
| 1 | H | 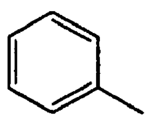 | 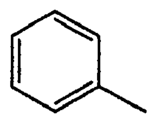 |
| 2 | H | 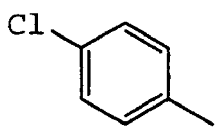 | 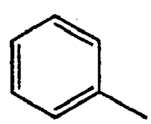 |
| 3 | H | 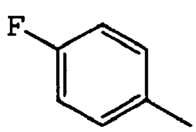 | 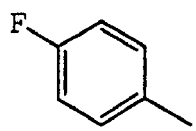 |

(continued)

| Compound No. | $R_1$ | $R_2$ | $R_3$ |
|---|---|---|---|
| 4 | H | 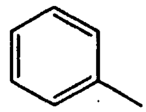 | 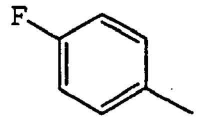 |
| 5 | H | 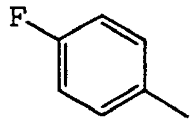 | 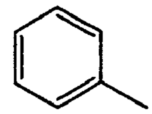 |
| 6 | H | 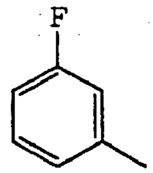 | 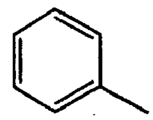 |
| 7 | H | 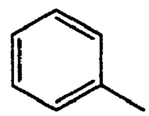 | 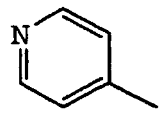 |
| 8 | H | 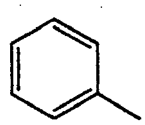 | 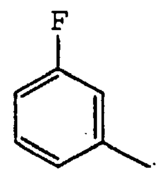 |
| 9 | 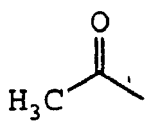 | 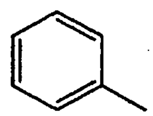 | 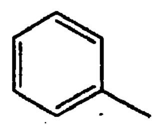 |
| 10 | 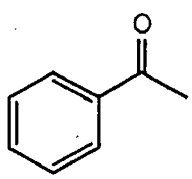 | 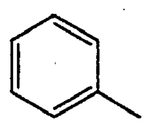 | 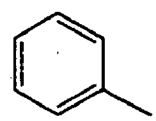 |
| 11 | H | 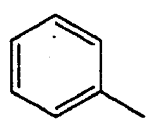 | 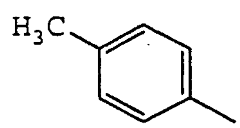 |
| 12 | H | 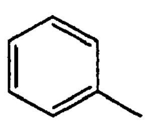 | 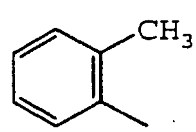 |
| 13 | H | 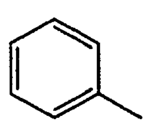 | 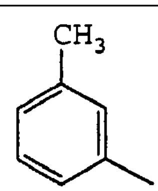 |

(continued)

| Compound No. | $R_1$ | $R_2$ | $R_3$ |
|---|---|---|---|
| 14 | H | 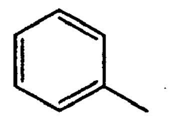 | 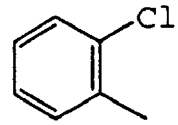 |
| 15 | H | 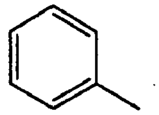 | 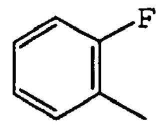 |
| 16 | H | 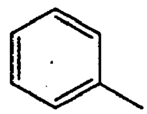 | 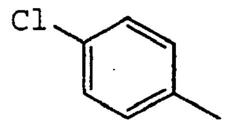 |
| 17 | H | 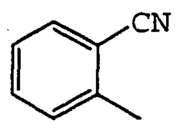 | 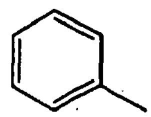 |
| 18 | H | 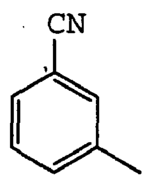 | 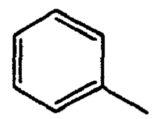 |
| 19 | H | 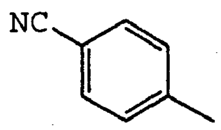 | 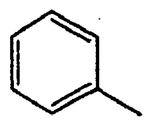 |
| 20 | H | 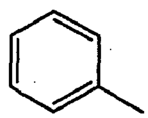 | 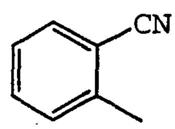 |
| 21 | H | 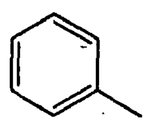 | 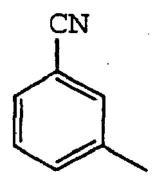 |
| 22 | H | 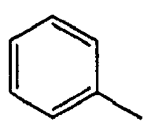 | 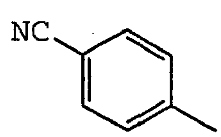 |
| 23 | H | 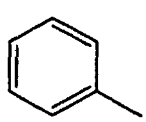 | 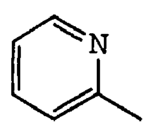 |

(continued)

| Compound No. | $R_1$ | $R_2$ | $R_3$ |
|---|---|---|---|
| 24 | H | 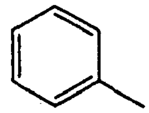 | 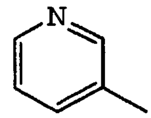 |
| 25 | 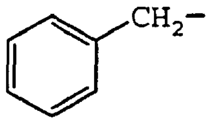 | 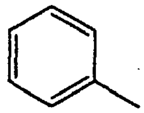 | 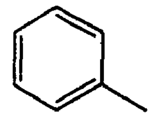 |
| 26 | H | 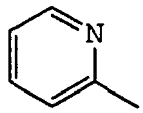 | 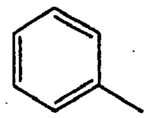 |
| 27 | H | 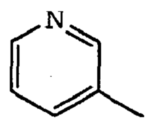 | 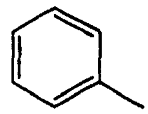 |
| 28 | H | 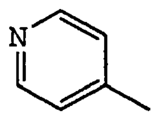 | 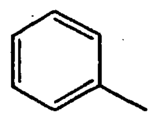 |
| 29 | H | 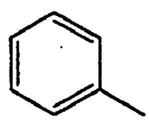 | 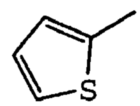 |
| 30 | H | 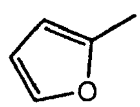 | 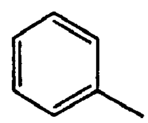 |
| 31 | H | 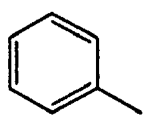 | 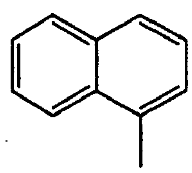 |
| 32 | H | 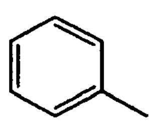 | H |
| 33 | H | H | 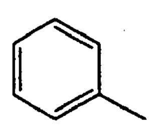 |
| 35 | H | 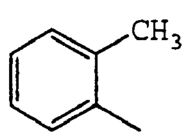 | 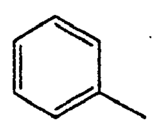 |

(continued)

| Compound No. | $R_1$ | $R_2$ | $R_3$ |
|---|---|---|---|
| 36 | H | 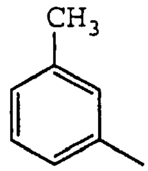 | 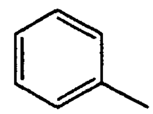 |
| 37 | H | 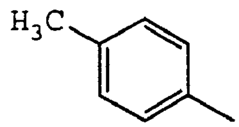 | 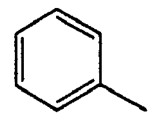 |
| 38 | H | 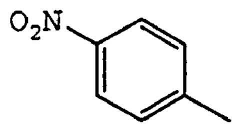 | 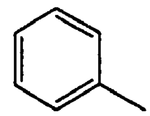 |
| 39 | H | 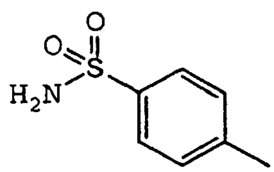 | 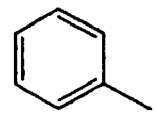 |
| 40 | H | 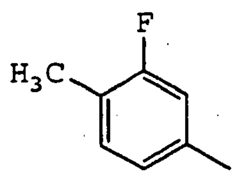 | 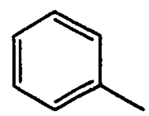 |
| 41 | H | 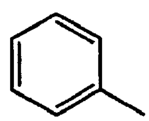 | 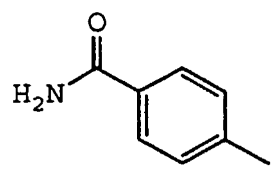 |
| 42 | H | 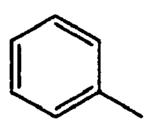 | 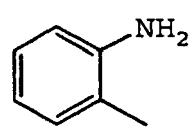 |
| 43 | H | 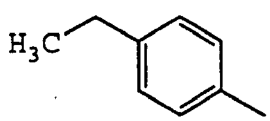 | 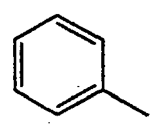 |
| 44 | H | 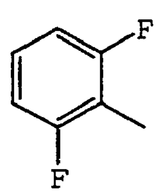 | 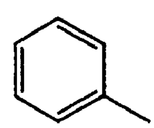 |

(continued)

| Compound No. | $R_1$ | $R_2$ | $R_3$ |
|---|---|---|---|
| 45 | H | 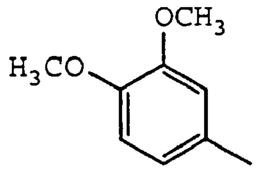 | 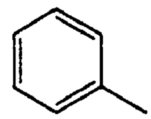 |
| 46 | H | 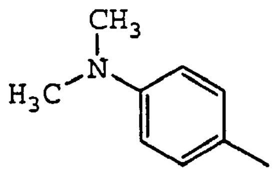 | 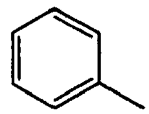 |
| 47 | H | 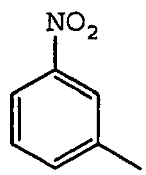 | 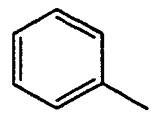 |
| 48 | H | 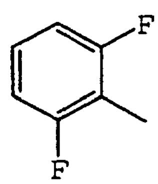 | 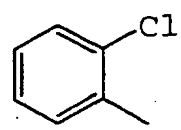 |
| 49 | H | 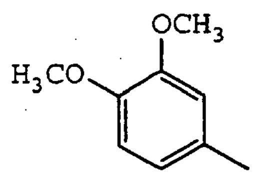 | 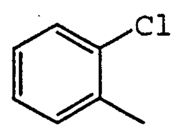 |
| 50 | H | 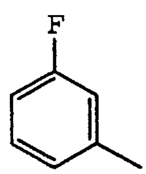 | 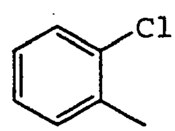 |
| 51 | H | 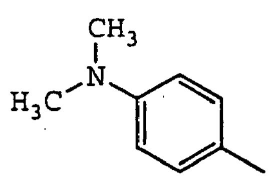 | 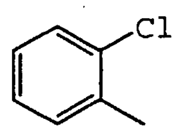 |
| 52 | H | 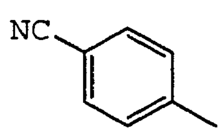 | 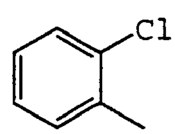 |

(continued)

| Compound No. | $R_1$ | $R_2$ | $R_3$ |
|---|---|---|---|
| 53 | H | 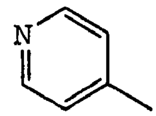 | 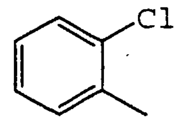 |
| 54 | H | 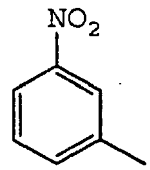 | 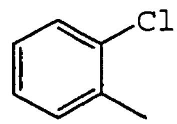 |
| 55 | H | 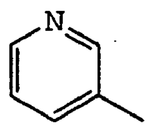 | 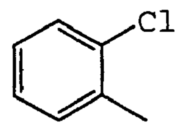 |
| 56 | H | 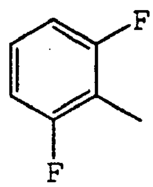 | 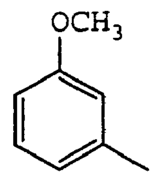 |
| 57 | H | 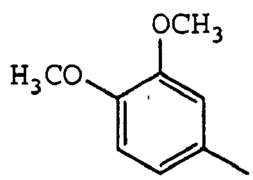 | 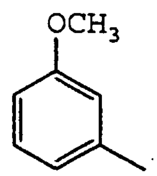 |
| 58 | H | 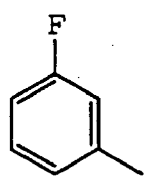 | 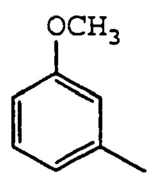 |
| 59 | H | 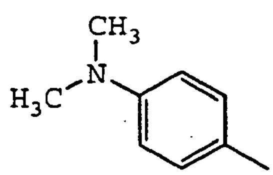 | 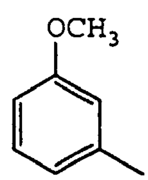 |
| 60 | H | 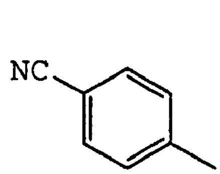 | 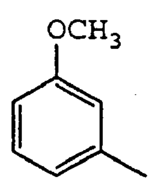 |

(continued)

| Compound No. | $R_1$ | $R_2$ | $R_3$ |
|---|---|---|---|
| 61 | H | 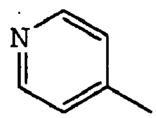 | 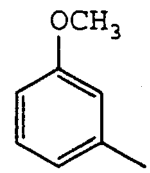 |
| 62 | H | 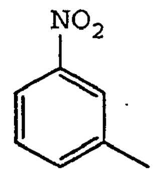 | 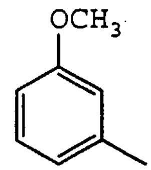 |
| 63 | H | 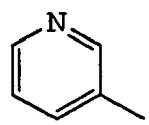 | 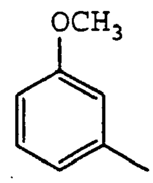 |
| 64 | H | 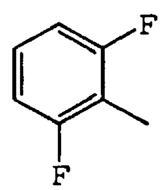 | 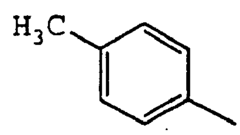 |
| 65 | H | 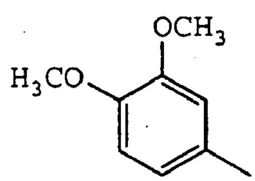 | 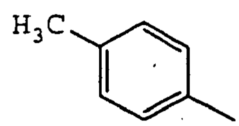 |
| 66 | H | 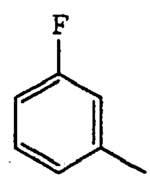 | 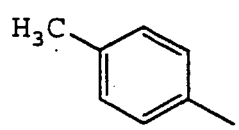 |
| 67 | H | 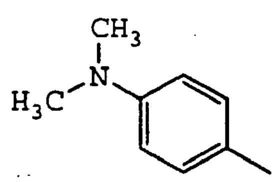 | 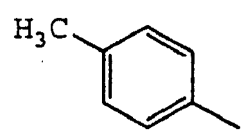 |
| 68 | H | 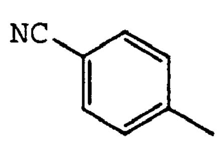 | 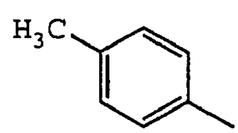 |

(continued)

| Compound No. | $R_1$ | $R_2$ | $R_3$ |
|---|---|---|---|
| 69 | H | 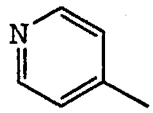 | 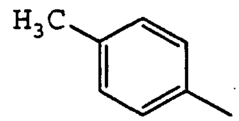 |
| 70 | H | 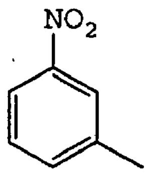 | 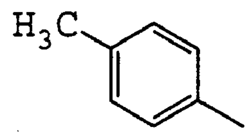 |
| 71 | H | 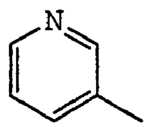 | 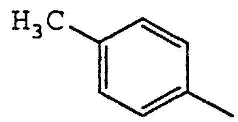 |
| 72 | H | 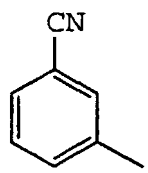 | 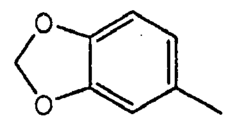 |
| 73 | H | 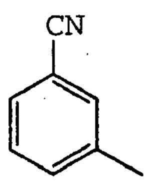 | 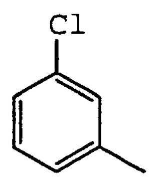 |
| 74 | H | 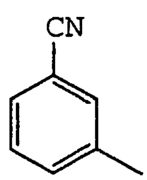 | 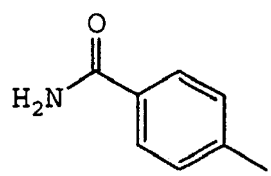 |
| 75 | H | 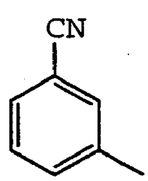 | 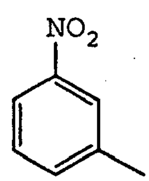 |
| 76 | H | 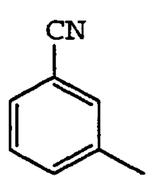 | 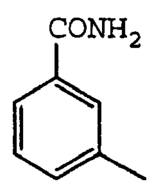 |
| 77 | H | 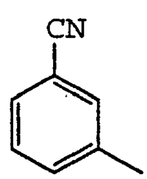 | 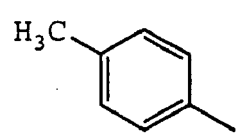 |

(continued)

| Compound No. | $R_1$ | $R_2$ | $R_3$ |
|---|---|---|---|
| 78 | H | 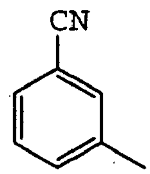 | 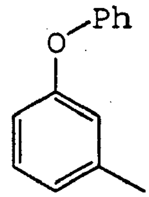 |
| 79 | H | 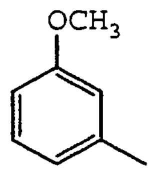 | 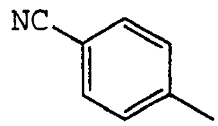 |
| 80 | H | 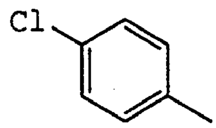 | 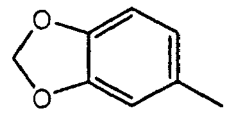 |
| 81 | H | 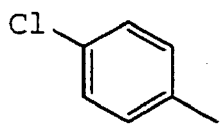 | 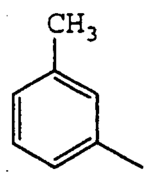 |
| 82 | H | 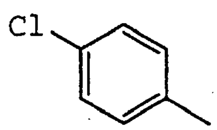 | 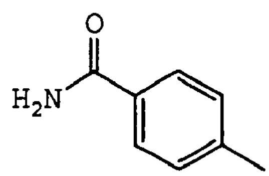 |
| 83 | H | 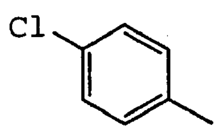 | 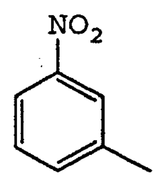 |
| 84 | H | 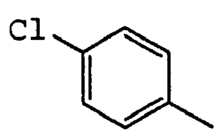 | 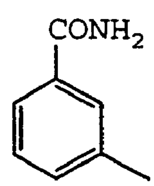 |
| 85 | H | 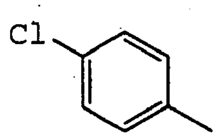 | 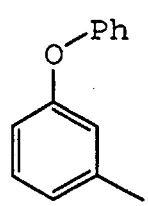 |

(continued)

| Compound No. | $R_1$ | $R_2$ | $R_3$ |
|---|---|---|---|
| 86 | H | 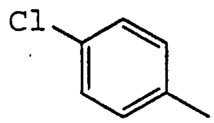 | 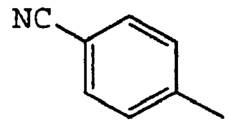 |
| 87 | H | 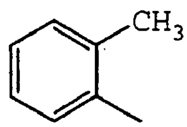 | 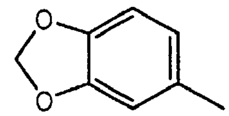 |
| 88 | H | 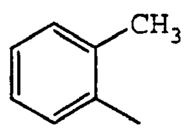 | 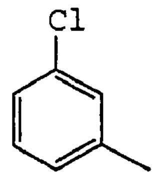 |
| 89 | H | 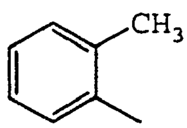 | 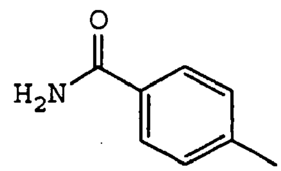 |
| 90 | H | 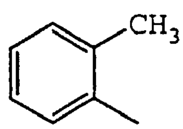 | 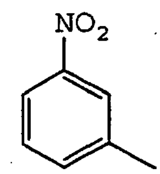 |
| 91 | H | 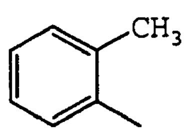 | 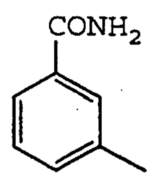 |
| 92 | H | 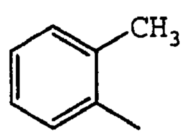 | 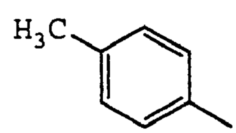 |
| 93 | H | 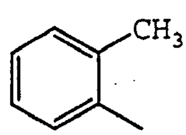 | 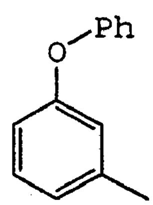 |
| 94 | H | 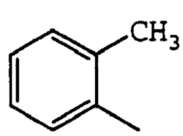 | 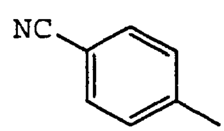 |

(continued)

| Compound No. | $R_1$ | $R_2$ | $R_3$ |
|---|---|---|---|
| 95 | H | 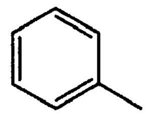 | 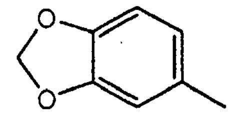 |
| 96 | H | 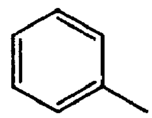 | 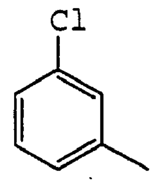 |
| 97 | H | 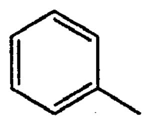 | 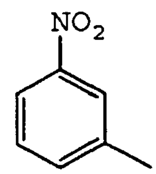 |
| 98 | H | 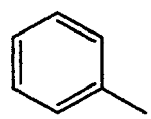 | 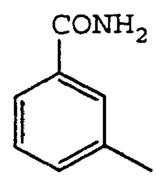 |
| 99 | H | 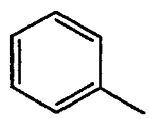 | 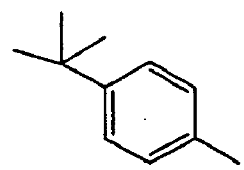 |
| 100 | H | 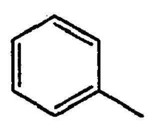 | 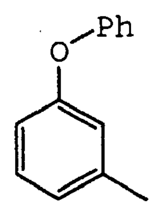 |
| 101 | H | 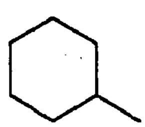 | 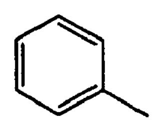 |
| 102 | H | 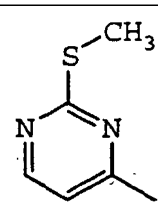 | 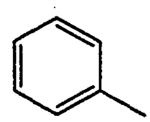 |

In a more preferred embodiment, the compound of the present invention is 3-amino-4-phenyl-5-(3-fluorophenyl)-1H-pyrazolo[3,4-c] pyridazine (Compound 8).

Methods for Producing GSK-3 Inhibitors

The compounds of this invention generally may be prepared from known starting materials, following methods known to those skilled in the art for analogous compounds, as illustrated by general Scheme I and the synthetic examples described below. References that may be useful in making the present compounds include El-Gendy, A.M. et al., Asian J. Chem., 1, 376 (1989); Deeb, A. and Said, S.A., Collect. Czech. Chem. Comm., 50, 2795 (1990); and Shalaby, A.A. J. Prakt. Chemie, 332, 104 (1990).

Scheme I

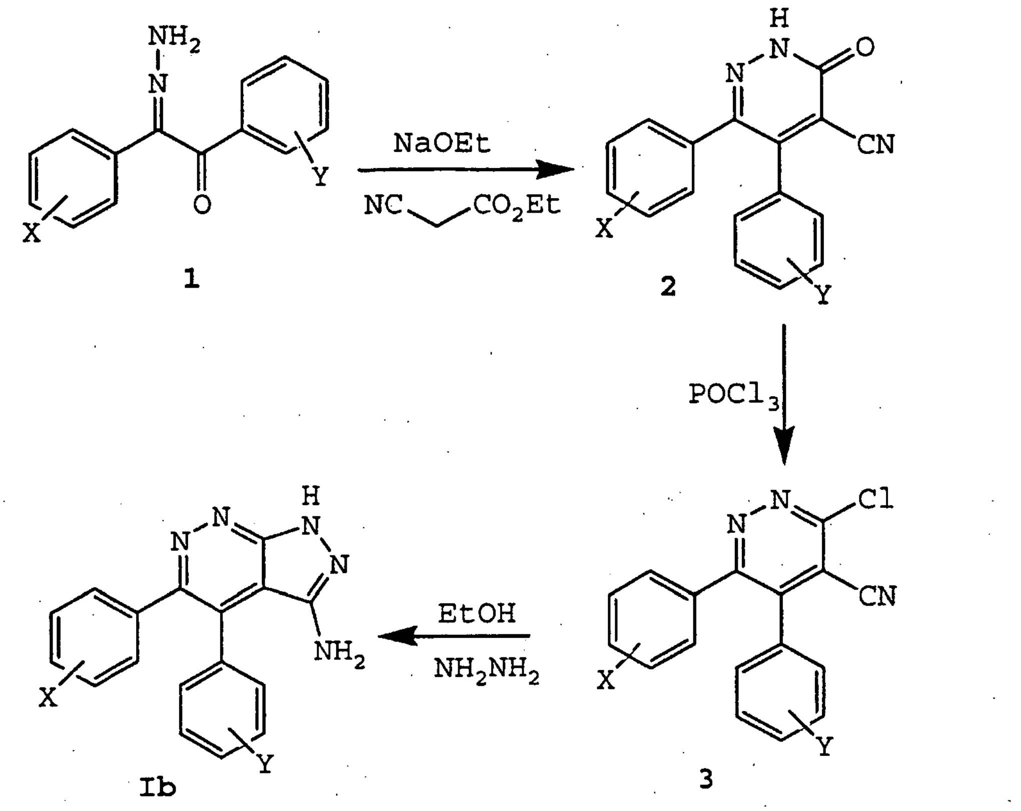


Scheme I shows a general approach for making the present compounds. The unsymmetrical diaryl keto hydrazones **(1)** were prepared from the corresponding substituted deoxybenzoins as described in United States Patent 4,009,022, incorporated herein by reference. The substituted deoxybenzoins were readily synthesized following methods known in the art, for instance, those described in Hill, D.T. et al, J. Het. Chem., 28, 1181 (1991); Rieke, R.D. et al, J. Org. Chem., 56, 1445 (1991); Fujisowa, T. et al, Chem. Lett., 1135 (1981); and Iyoda, M. et al, Tet. Lett., 26, 4777 (1985). To an ethanol solution of diaryl keto hydrazone **(1),** ethyl cyanoacetate (excess) and sodium ethoxide in tetrahydrofuran (THF) were added. The mixture was refluxed for 6 hours. After cooling, the solvent was removed under vacuum and the residue was taken up in dichloromethane ($CH_2Cl_2$), washed with 0.1 M HCl and water and dried with sodium sulfate. After filtering, the solvent was removed under vacuum and the product, 4-cyano-5,6-diaryl 2(1H) pyridazinone **(2)** was purified by chromatography on silica gel (5:95 methanol/dichloromethane).

Purified 4-cyano-5,6-diaryl 2(1H) pyridazinone **(2)** was added to $POCl_3$ and heated to 100°C for 5-6 hours. After cooling, the reaction mixture was poured onto ice and stirred for one hour. The resultant 3-chloro-4-cyano-5,6-diaryl pyridazine **(3)** was filtered off, washed with water, air dried and used in the next step without further purification. Purified 3-chloro-4-cyano-5,6-diaryl pyridazine **(3)** was further refluxed with 2 equivalents of anhydrous hydrazine in ethanol for several hours. Upon cooling, the product **Ib** would sometimes precipitate out, in which case compound **Ib** was purified by recrystallizing from ethanol. Otherwise purification of **Ib** was achieved by chromatography on silica gel (5:95 methanol/dichloromethane).

One having ordinary skill in that art may synthesize other compounds of this invention following the teachings of the specification using reagents that are readily synthesized or commercially available.

The present invention provides detailed methods of producing representative compounds of the present invention as described in Examples 1-17 below.

The activity of the compounds as protein kinase inhibitors, for example, as GSK-3 inhibitors, may be assayed *in vitro*, *in vivo* or in a cell line. *In vitro* assays include assays that determine inhibition of either the phosphorylation activity or ATPase activity of activated GSK-3. Alternate *in vitro* assays quantitate the ability of the inhibitor to bind to GSK-3. Inhibitor binding may be measured by radiolabelling the inhibitor prior to binding, isolating the inhibitor/GSK-3 complex and determining the amount of radiolabel bound. Alternatively, inhibitor binding may be determined by running a competition experiment where new inhibitors are incubated with GSK-3 bound to known radioligands.

Pharmaceutical Compositions

According to another embodiment of the invention, the protein kinase inhibitors, particularly GSK-3 inhibitors, or derivatives/salts thereof may be formulated into compositions. In a preferred embodiment, the composition is a pharmaceutical composition. In one embodiment, the composition comprises an amount of the protein kinase inhibitor effective to inhibit GSK-3 in a biological sample or in a patient. In another embodiement, the pharmaceutical compositions, which comprise an amount of the protein kinase inhibitor effective to treat or prevent a GSK-3-mediated condition and a pharmaceutically acceptable carrier, adjuvant, or vehicle, may be formulated for administration to a patient.

The amount effective to inhibit GSK-3 is one that inhibits the kinase activity of GSK-3 at least 50%, more preferably at least 60% or 70%, even more preferably at least 80% or 90%, and most preferably at least 95%, where compared to the GSK-3 activity of the enzyme in the absence of an inhibitor. Any method may be used to determine inhibition. See, e.g., Example 18.

Pharmaceutically acceptable carriers that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously.

Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutical compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added.

For oral administration in a capsule form, useful diluents include lactose and dried cornstarch. When aqueous suspensions are required for oral use, the active ingredient is combined with emulsifying and suspending agents. If desired, certain sweetening, favoring or coloring agents may also be added.

Alternatively, the pharmaceutical compositions of this invention may be administered in the form of suppositories for rectal administration. These can be prepared by mixing the agent with a suitable nonirritating excipient which is solid at room temperature but liquid at rectal temperature and therefore will melt in the rectum to release the drug. Such materials include cocoa butter, beeswax and polyethylene glycols.

The pharmaceutical compositions of this invention may also be administered topically, especially when the target of treatment includes areas or organs readily accessible by topical application, including diseases of the eye, the

skin, or the lower intestinal tract. Suitable topical formulations are readily prepared for each of these areas or organs.

Topical application for the lower intestinal tract can be effected in a rectal suppository formulation (see above) or in a suitable enema formulation.

Topically-transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum.

The pharmaceutical compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

In addition to the compounds of this invention, pharmaceutically acceptable derivatives or prodrugs of the compounds of this invention may also be employed in compositions to treat or prevent the above-identified diseases or disorders.

The amount of the protein kinase inhibitor that may be combined with the carrier materials to produce a single dosage form will vary depending upon the patient treated and the particular mode of administration. Preferably, the compositions should be formulated so that a dosage of between 0.01 - 100 mg/kg body weight/day of the inhibitor can be administered to a patient receiving these compositions.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of inhibitor will also depend upon the particular compound in the composition.

Method of Treatment and Prevention of Disease

One aspect of this invention relates to a method for treating a disease state in patients that is alleviated by treatment with a GSK-3 inhibitor, which method comprises administering to a patient in need of such a treatment a therapeutically effective amount of a compound of formula I or a pharmaceutically acceptable derivative thereof.

Another aspect of this invention relates to a method of inhibiting GSK-3 activity in a patient, comprising administering to the patient a composition comprising a compound of formula I or a pharmaceutically acceptable derivative thereof. Another method relates to enhancing glycogen synthesis and/or lowering blood levels of glucose in a patient in need thereof, which method comprises administering to the patient a therapeutically effective amount of a compound of formula I or a pharmaceutically acceptable derivative thereof. This method is especially useful for diabetic patients. Another method relates to inhibiting the production of hyperphosphorylated Tau protein, which is useful in halting or slowing the progression of Alzheimer's disease. Another method relates to inhibiting the phosphorylation of β-catenin, which is useful for treating schizophrenia.

Depending upon the particular protein kinase-mediated condition to be treated or prevented, additional drugs, which are normally administered to treat or prevent that condition, may be administered together with the inhibitors of this invention. For example, in the treatment of diabetes, other anti-diabetic agents may be combined with the GSK-3 inhibitors of this invention to treat diabetes. These agents include, without limitation, insulin, in injectable or inhalation form, glitazones, and sulfonyl ureas.

Those additional agents may be administered separately from the protein kinase inhibitor-containing composition, as part of a multiple dosage regimen. Alternatively, those agents may be part of a single dosage form, mixed together with the protein kinase inhibitor of this invention in a single composition.

Another method of this invention relates to inhibiting GSK-3 activity in a biological sample, which method comprises contacting the biological sample with the GSK-3 inhibitor of formula I or a pharmaceutically acceptable derivative or prodrug thereof, or a pharmaceutical composition thereof, in an amount effective to inhibit GSK-3.

Crystallizable Compositions and Crystals of GSK-3β Protein and Protein Complexes

According to one embodiment, the invention provides a crystallizable composition comprising unphosphorylated GSK-3β protein or its homologue and phosphate ions. In one embodiment, the crystallizable composition further comprises between about 5 to 25% v/v of precipitant polyethylene glycol, a buffer that maintains pH between about 4.0 and 8.0; and optionally, a reducing agent of 1-20 mM. In one embodiment, the crystallizable composition comprises unphosphorylated GSK-3β protein, 15% PEG 3350, 50 mM $Na/KPO_4$ at pH 4.1 and 10 mM DTT.

In another embodiment, the invention provides a crystallizable composition comprising phosphorylated GSK-3β protein or its homologue. In one embodiment, the crystallizable composition further comprises between about 5-25% v/v polyethylene glycol, a buffer that maintains pH between about 6.0 and 8.5, and 1-10% dimethyl sulfoxide(DMSO). In one embodiment, the crystallizable composition comprises phosphorylated GSK-3β protein, between about 7-10% PEG 3350, 100 mM Tris HCl and 5% DMSO.

In another embodiment, the invention provides a crystallizable composition comprising GSK-3β protein or its homologue and a chemical entity. The GSK-3β protein may be phosphorylated or unphosphorylated. In one embodiment, the chemical entity is selected from the group consisting of an inhibitor for the active site, a nucleotide triphosphate, an ATP, a substrate or inhibitor for the substrate binding groove, or a peptide comprising a phosphorylation sequence. In one embodiment, the inhibitor for the active site is selected from the group consisting of 4,5-Diphenyl-1H-pyrazolo[3,4-c]pyridazin-3-ylamine; (5-Methyl-2H-pyrazol-3-yl)-(2-pyridin-4-yl-quinazolin-4-yl)-amine; 4-(5-Methyl-2-phenylamino-pyrimidin-4-yl)-1H-pyrrole-2-carboxylic acid (2-hydroxy-1-phenyl-ethyl)-amide; (1H-Indazol-3-yl)-[2-(2-trifluoromethyl-phenyl)-quinazolin-4-yl]-amine and an ATP analogue. In one embodiment, the crystallizable composition further comprises between about 5-25% v/v polyethylene glycol, and between about 0.1-1 M ammonium fluoride, ammonium formate, potassium formate or potassium fluoride. In one embodiment, the peptide comprising a phosphorylation sequence is HSSPHQpSEDEEE. In another embodiment, the crystallizable composition comprises phosphorylated GSK-3β protein, HSSPHQpSEDEEE, between about 10-15% v/v polyethylene glycol, and 50 mM ammonium fluoride.

In one embodiment, the GSK-3β protein or its homologue is preferably 85-100% pure prior to forming the crystallizable composition.

According to another embodiment, the invention provides a crystal composition comprising unphosphorylated GSK-3β protein or its homologue and phosphate ions. In another embodiment, the invention provides a crystal composition comprising unphosphorylated GSK-3β protein or its homologue and a chemical entity. Preferably, the chemical entity is an inhibitor for the active site, an ATP analogue or nucleotide triphosphate. Preferably, the crystal has a unit cell dimension of a= 83 Å b= 86 Å c= 178 Å, $\alpha = \beta = \gamma = 90^\circ$ and belongs to space group $P2_12_12_1$. It will be readily apparent to those skilled in the art that the unit cells of the crystal compositions may deviate ± 1-2 Å from the above cell dimensions depending on the deviation in the unit cell calculations.

The invention also provides a crystal composition comprising phosphorylated GSK-3β protein or its homologue with or without a chemical entity. Preferably, the chemical entity is an inhibitor for the active site, an ATP analogue or nucleotide triphosphate. Preferably, the unit cell dimensions of the crystal is a= 64 Å b=67 Å c= 67 Å $\alpha$=100° $\beta$= 103° $\gamma$= 89.8° or a= 64 Å b=67 Å c= 67 Å $\alpha$= 80° $\beta$= 77° $\gamma$= 89.8° and belongs to the space group P1. In another embodiment, the chemical entity is a substrate or inhibitor to the substrate binding groove, or a peptide comprising a phosphorylation sequence. Preferably, the chemical entity is HSSPHQpSEDEEE and the unit cell dimensions of the crystal is a= 75 Å b=108 Å c= 121 Å $\alpha$=$\beta$=Å= 90° and belongs to the space group $P2_12_12_1$. It will be readily apparent to those skilled in the art that the unit cells of the crystal compositions may deviate ± 1-2 Å or ± 1-2° from the above cell dimensions depending on the deviation in the unit cell calculations.

As used herein, the GSK-3β protein in the crystallizable or crystal compositions can be the full length GSK-3β protein (amino acids 1-420 of SEQ ID NO: 1); a truncated GSK-3β protein with amino acids 7-420, 25-381, 37-381 of SEQ ID NO: 1; the full length protein with conservative substitutions; said truncated protein with conservative mutations. In one embodiment, the GSK-3β protein is produced from the baculovirus system. The unphosphorylated GSK-3β protein is not phosphorylated at any of the phosphorylation sites. The phosphorylated GSK-3β protein is phosphorylated at any of the phosphorylation sites, for example, at Serine 9 or Tyrosine 216. Preferably, the protein is phosphorylated at Tyrosine 216.

The GSK-3β protein or its homologue may be produced by any well-known method, including synthetic methods, such as solid phase, liquid phase and combination solid phase/liquid phase syntheses; recombinant DNA methods, including cDNA cloning, optionally combined with site directed mutagenesis; and/or purification of the natural products. In one embodiment, the protein is overexpressed from a baculovirus system or *E. Coli* system.

The invention also relates to a method of obtaining a crystal of a GSK-3β protein complex or GSK-3β homologue protein complex comprising a chemical entity that binds to the substrate-binding groove, comprising the steps of:

a) producing and purifying a GSK-3β protein;
b) mixing a crystallization solution with the protein complex to produce a crystallizable composition; and

c) subjecting the composition to conditions which promote crystallization.

Conditions for promoting crystallization include, for example, apparatuses and devices for forming crystals, for example, a hanging drop, sitting drop, dialysis or microtube batch device, will promote crystallization. (U.S. patent 4,886,646, 5,096,676, 5,130,105, 5,221,410 and 5,400,741; Pav et al., Proteins: Structure, Function, and Genetics, 20, pp. 98-102 (1994), incorporated herein by reference). The hanging drop or sitting drop methods produce crystals by vapor diffusion. The hanging drop or sitting drop which contains the crystallizable composition is equilibrated against a reservoir containing a higher concentration of precipitant. As the drop approaches equilibrium with the reservoir, the protein becomes saturated in solution and crystals form. One of ordinary skilled in the art would be able to vary the crystallization conditions disclosed above and identify other crystallization conditions that would produce crystals for GSK-3 protein or its homologues with or without a chemical entity. Such variations may include adjusting the pH, salt type or concentration, precipitant type or concentration, crystallization temperature, protein concentration. One, may also use high throughput crystallization assays to assist in finding or optimizing the crystallization condition.

<u>Binding Pockets of GSK-3β Protein, Protein Complexes or Homologues thereof</u>

As disclosed above, applicants have provided the three-dimensional X-ray crystal structures of unphosphorylated GSK-3β, phosphorylated GSK-3β, unphosphorylated GSK-3β-inhibitor complexes, phosphorylated GSK-3β-inhibitor complex and phosphorylated GSK-3β-ADP-peptide complex. The crystal structure of GSK-3β presented here is within the GSK-3 subfamily. The invention will be useful for inhibitor design. The atomic coordinate data is presented in Figures 1-7.

In order to use the structure coordinates generated for the unphosphorylated and phosphorylated GSK-3β, their complexes or one of its binding pockets or GSK-3β-like binding pocket thereof, it is often times necessary to convert them into a three-dimensional shape. This is achieved through the use of commercially available software that is capable of generating three-dimensional structures of molecules or portions thereof from a set of structure coordinates.

Binding pockets, also referred to as binding sites in the present invention, are of significant utility in fields such as drug discovery. The association of natural ligands or substrates with the binding pockets of their corresponding receptors or enzymes is the basis of many biological mechanisms of action. Similarly, many drugs exert their biological effects through association with the binding pockets of receptors and enzymes. Such associations may occur with all or part of the binding pocket. An understanding of such associations will help lead to the design of drugs having more favorable associations with their target receptor or enzyme, and thus, improved biological effects. Therefore, this information is valuable in designing potential inhibitors of the binding pockets of biologically important targets. The binding pockets of this invention will be important for drug design.

The structure coordinates described above may be used to derive the torsion angles of the side chains (S.C. Lovell et al, Proteins: Structure, Function, and Genetics, 40, 389-408, (2000)). For example, in Glutamine, χ1 defines the torsion angle between N, Cα, Cβ, Cγ; χ2 defines the torsion angle between Cα, Cβ, Cγ, Cδ; and χ3 defines the torsion angle between Cβ, Cγ, Cδ, Oε.

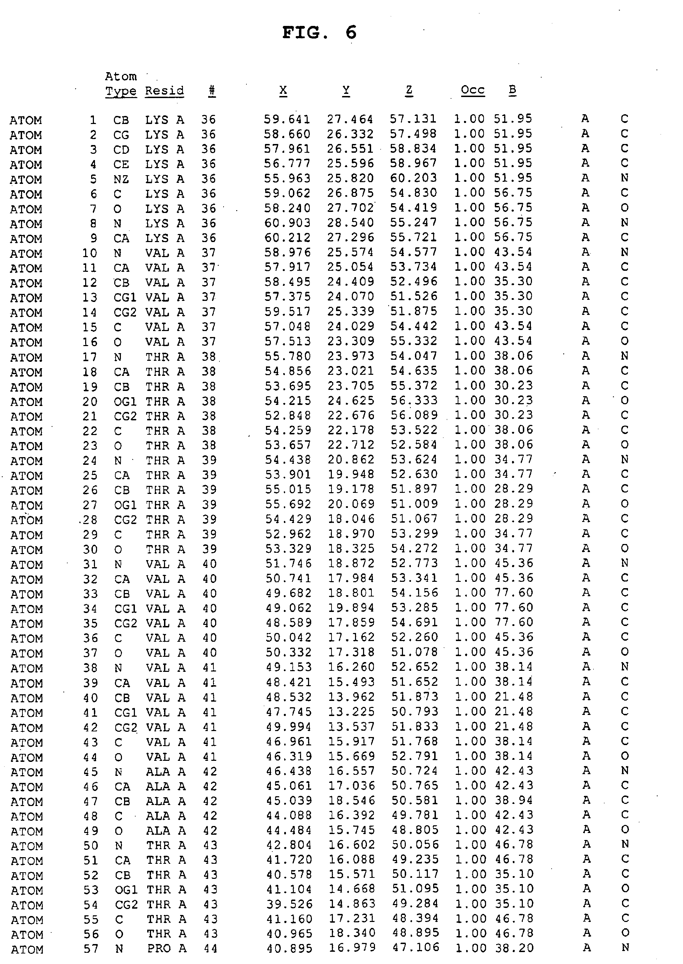

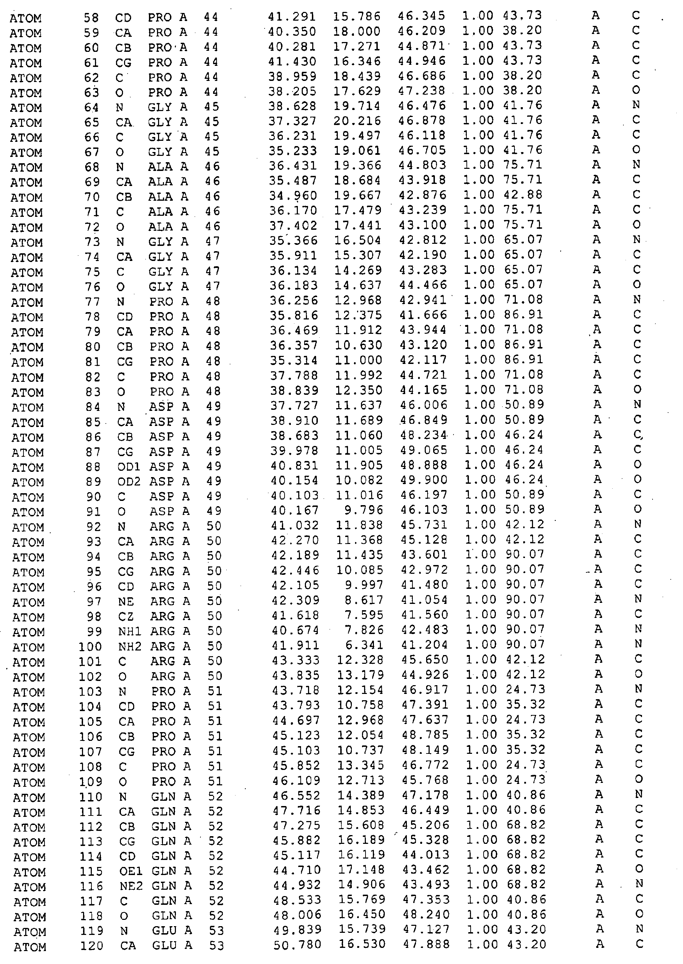

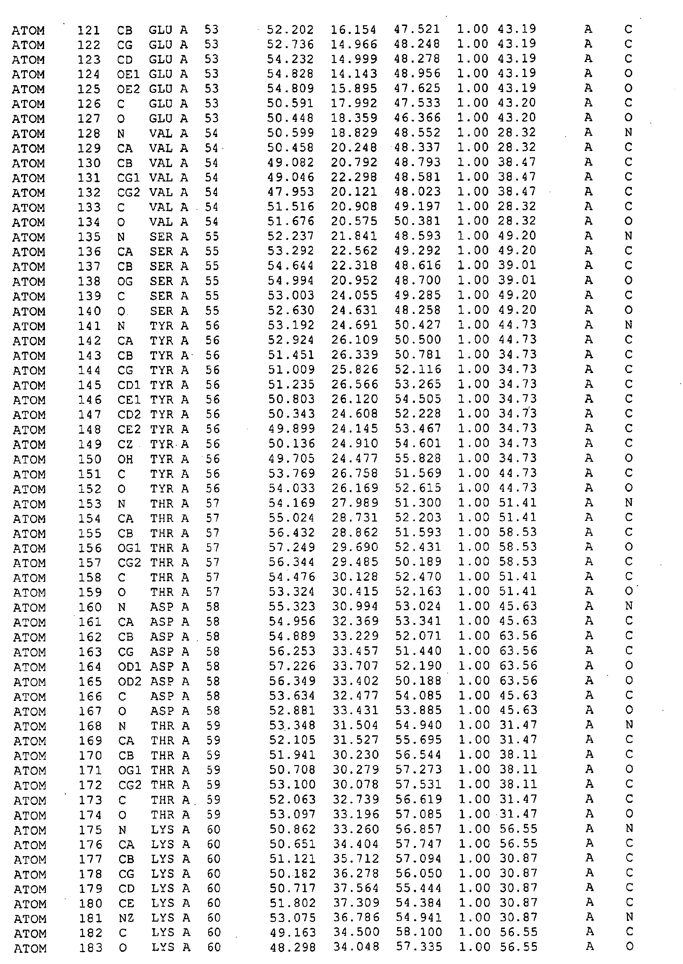

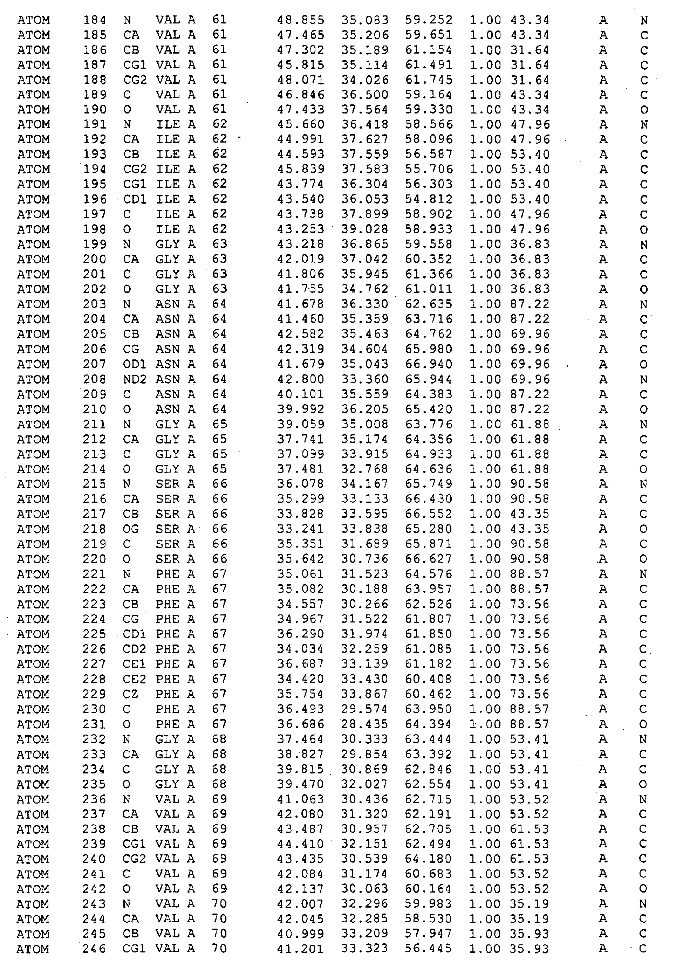

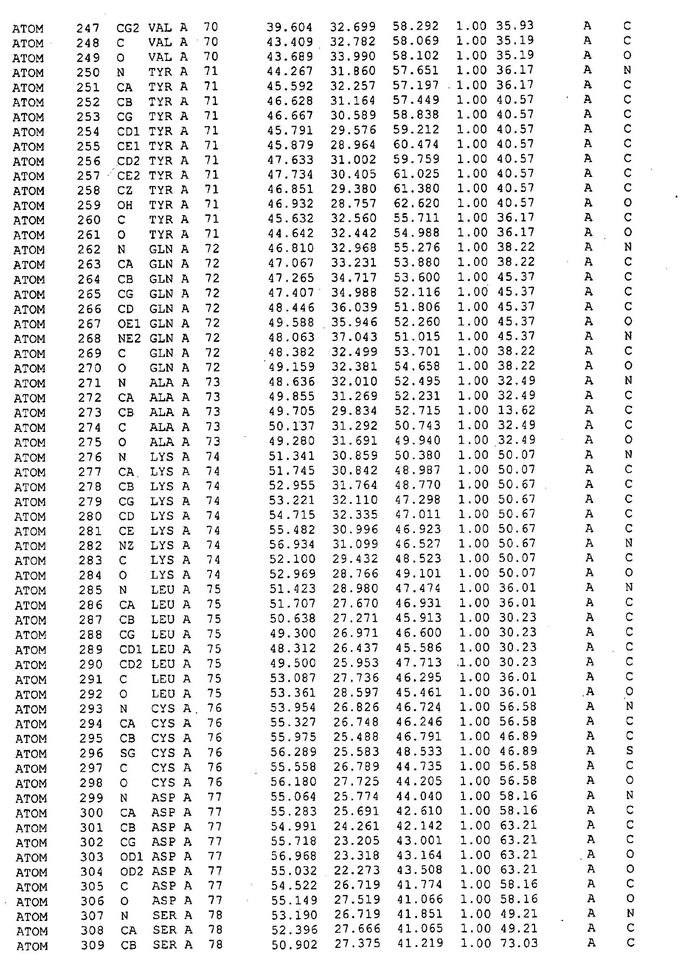

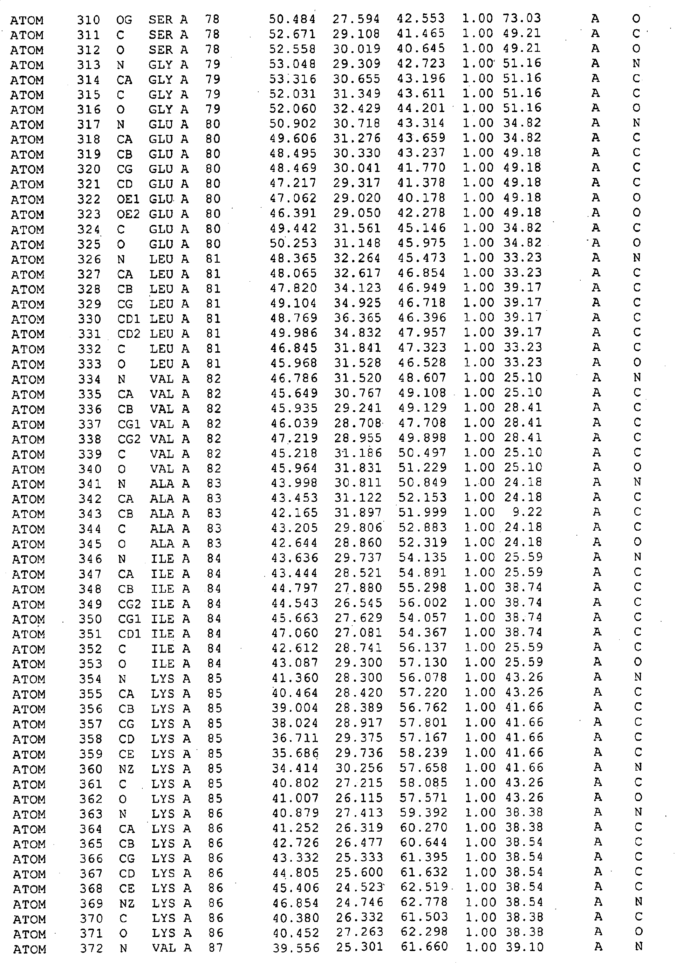

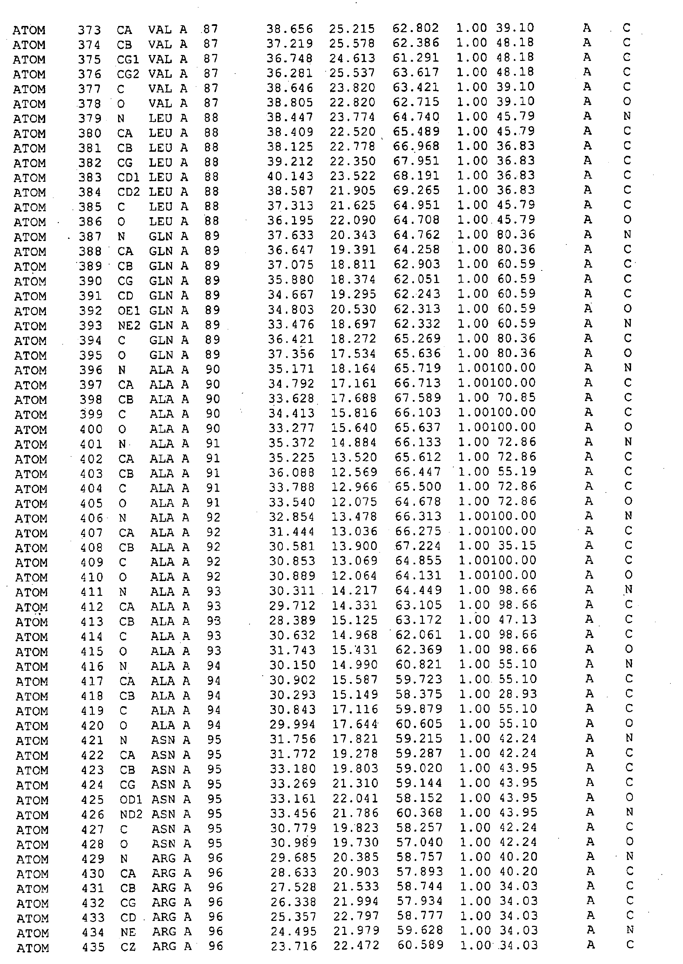

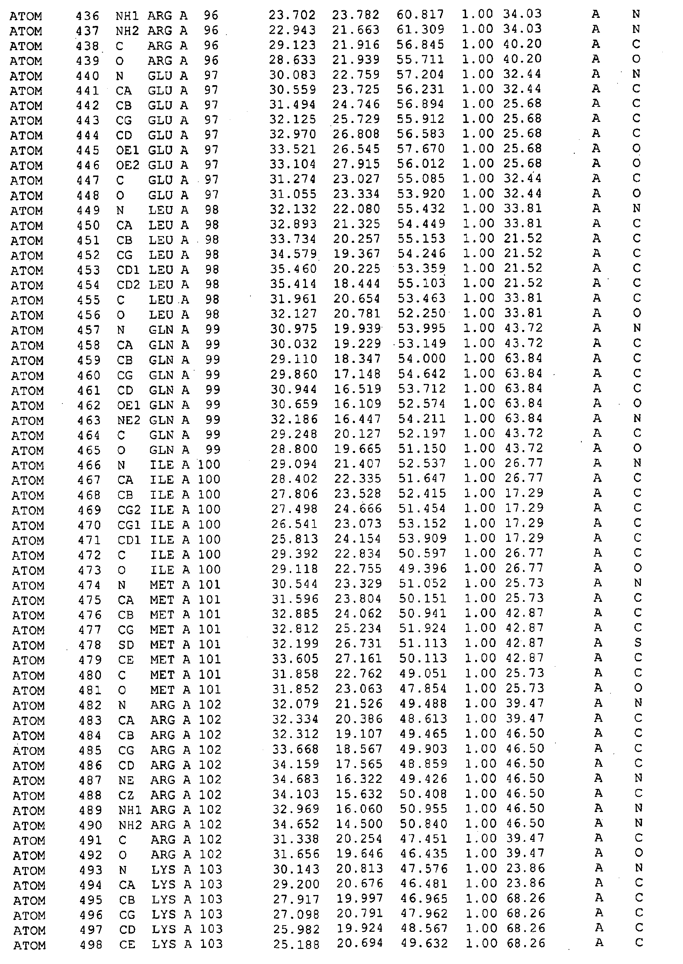

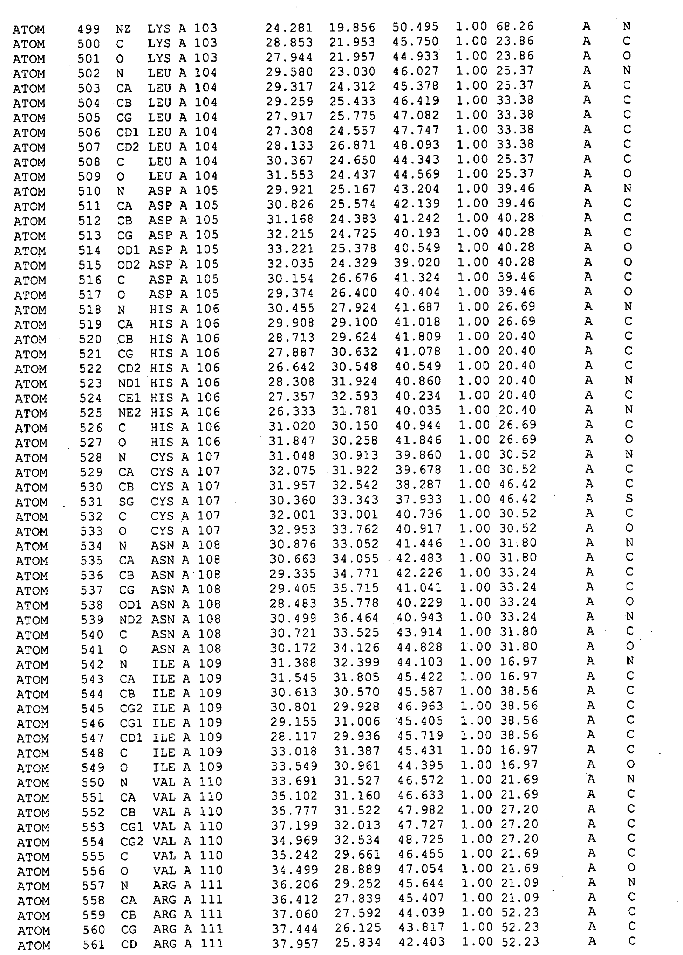

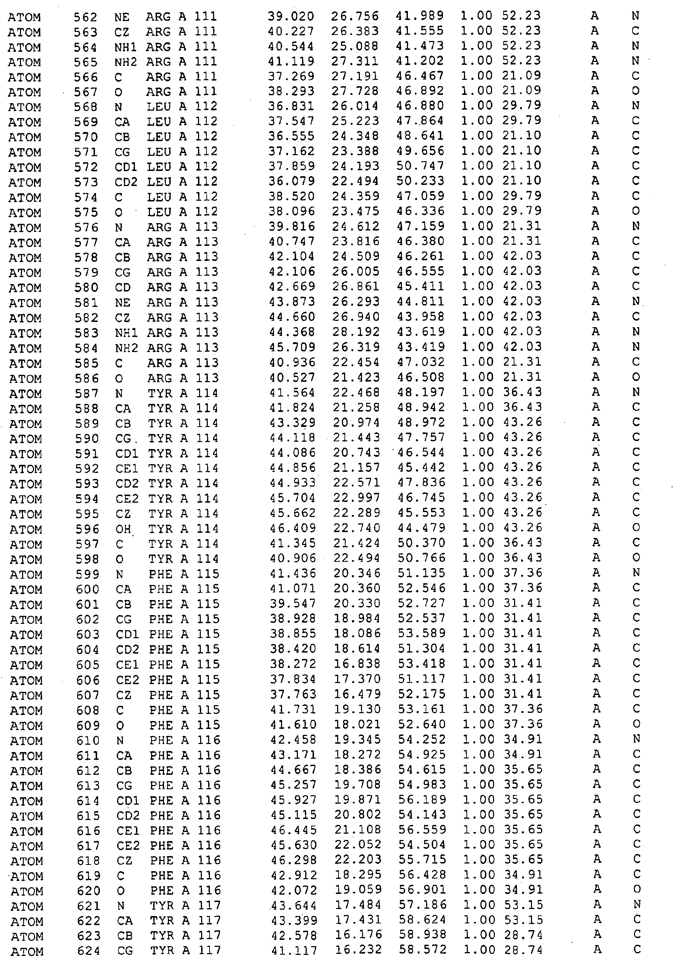

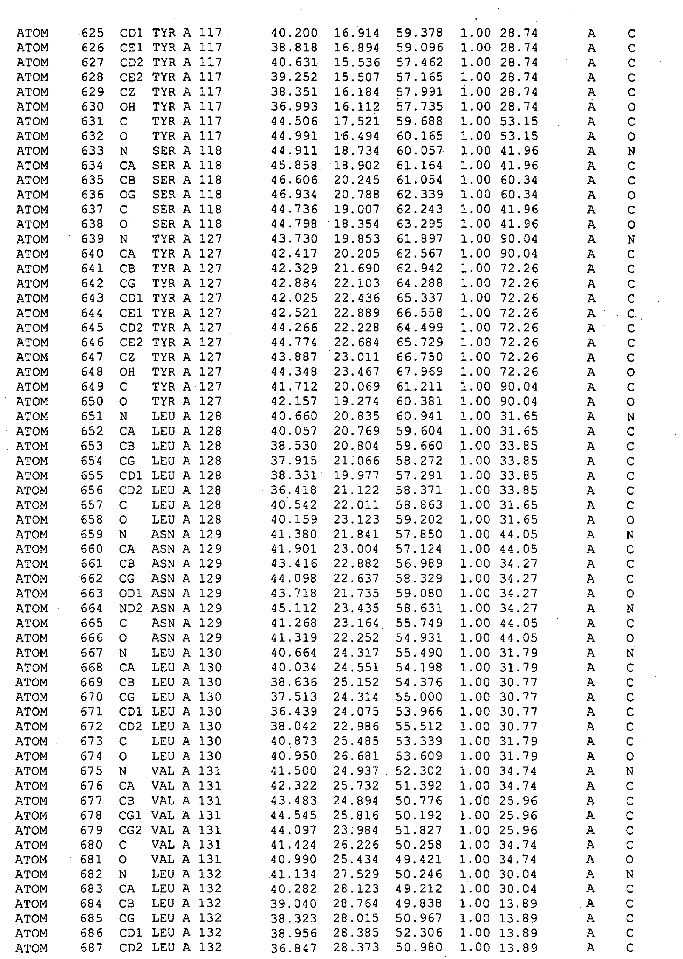

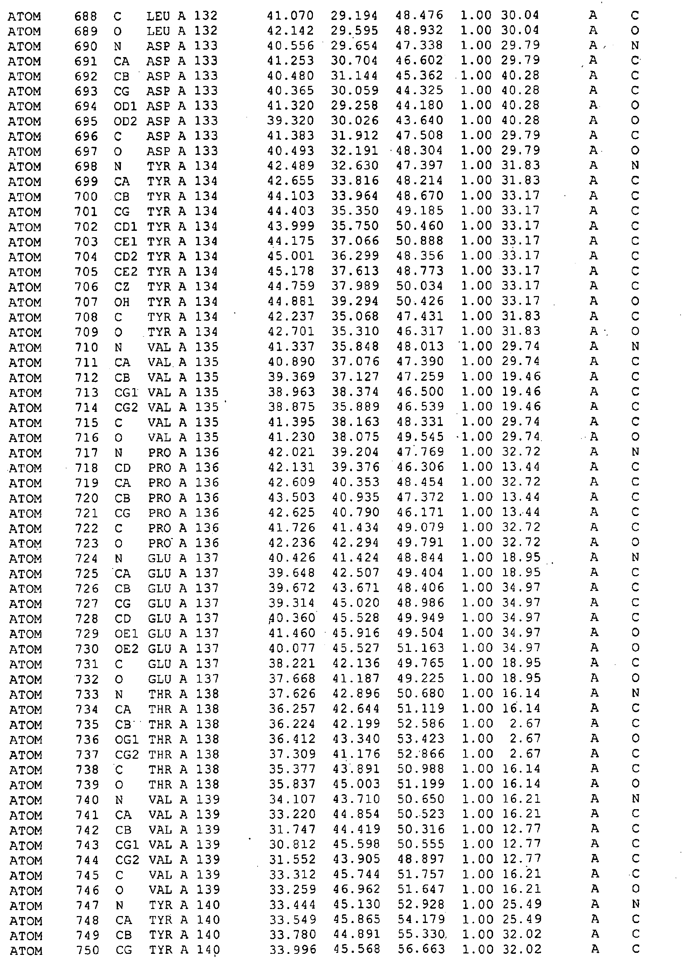

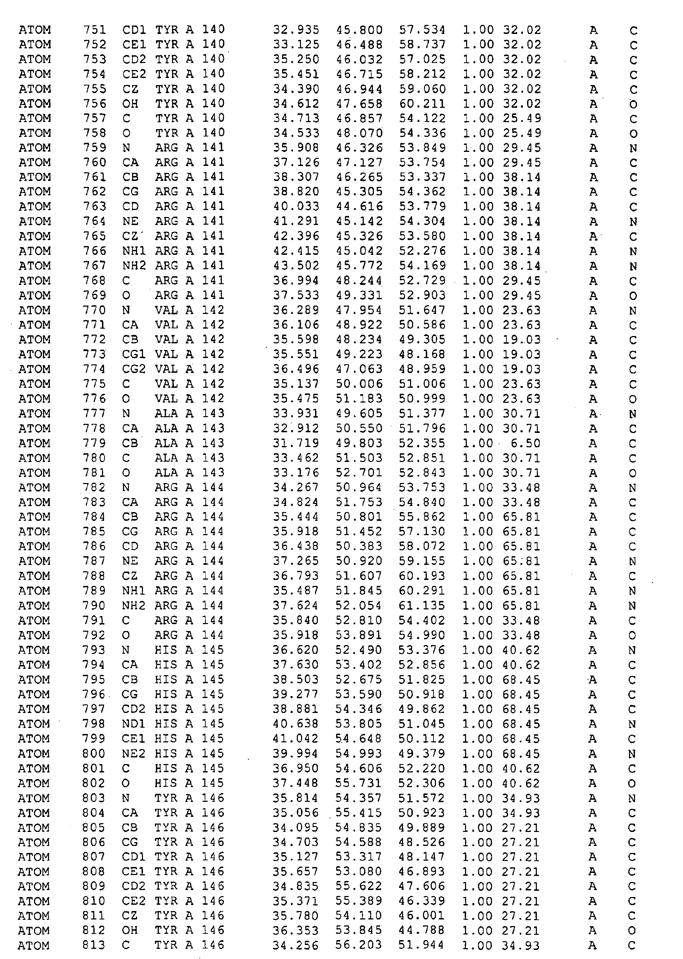

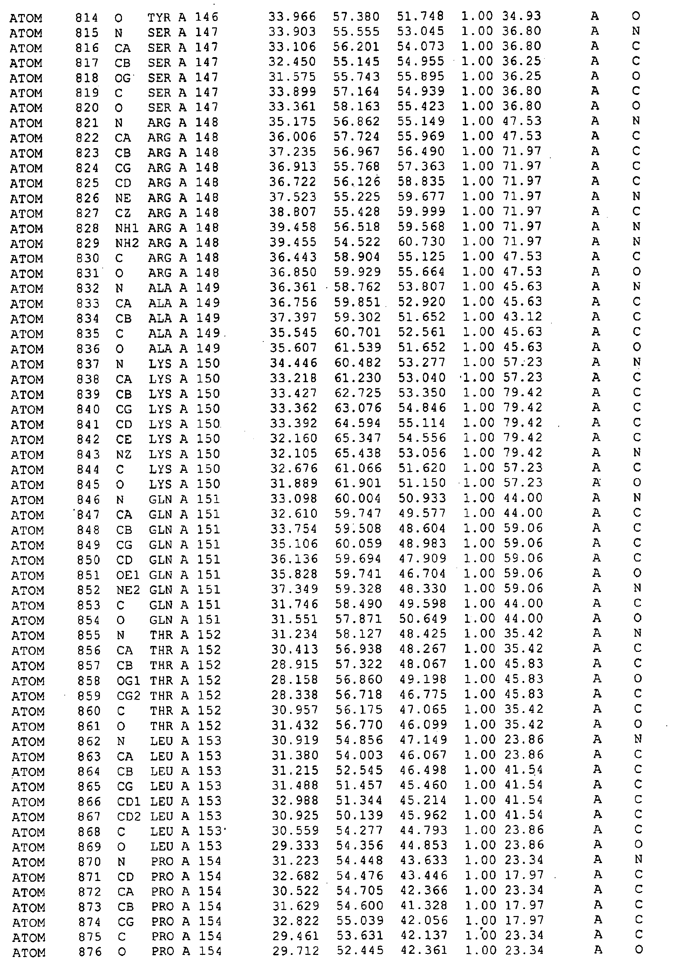

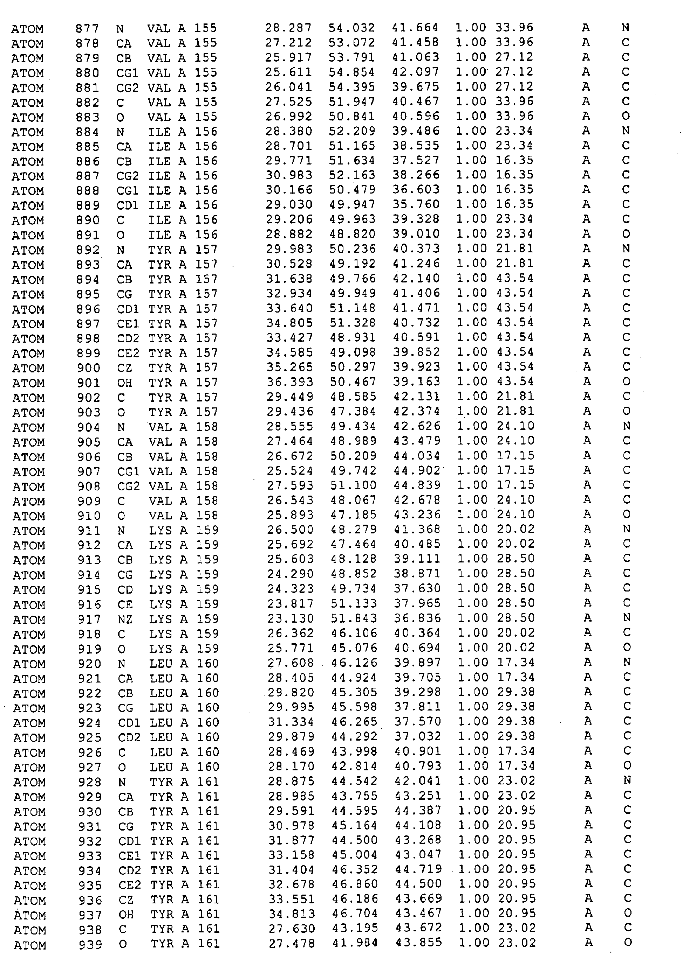

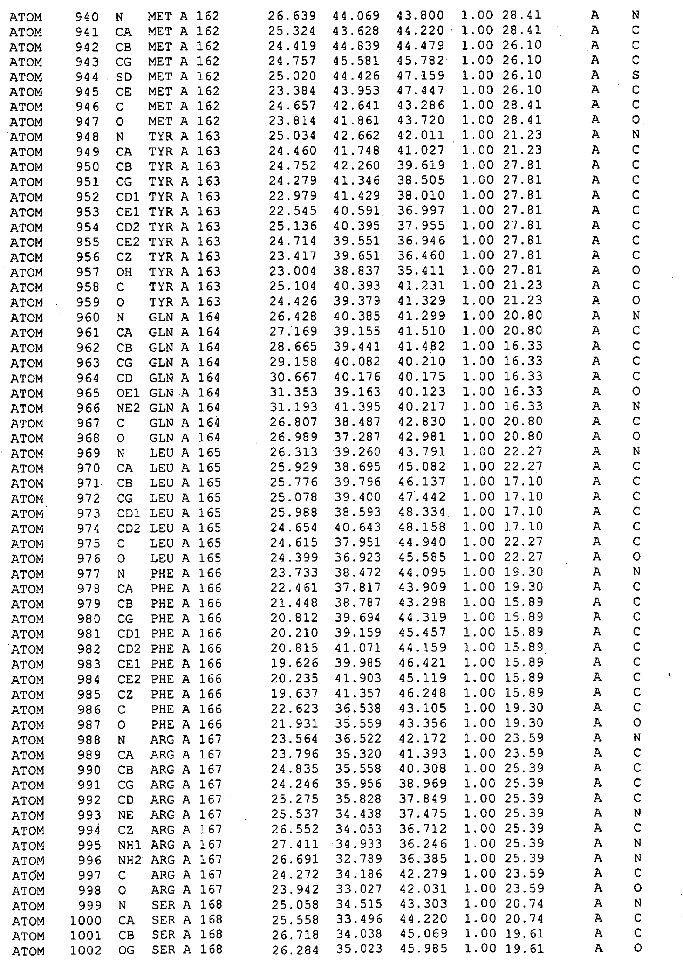

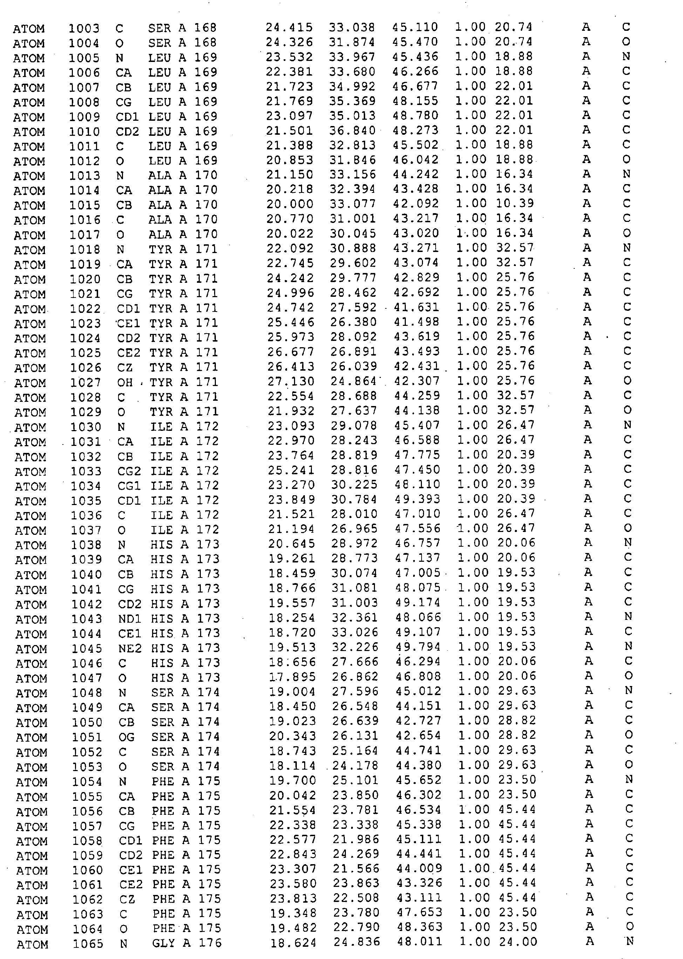

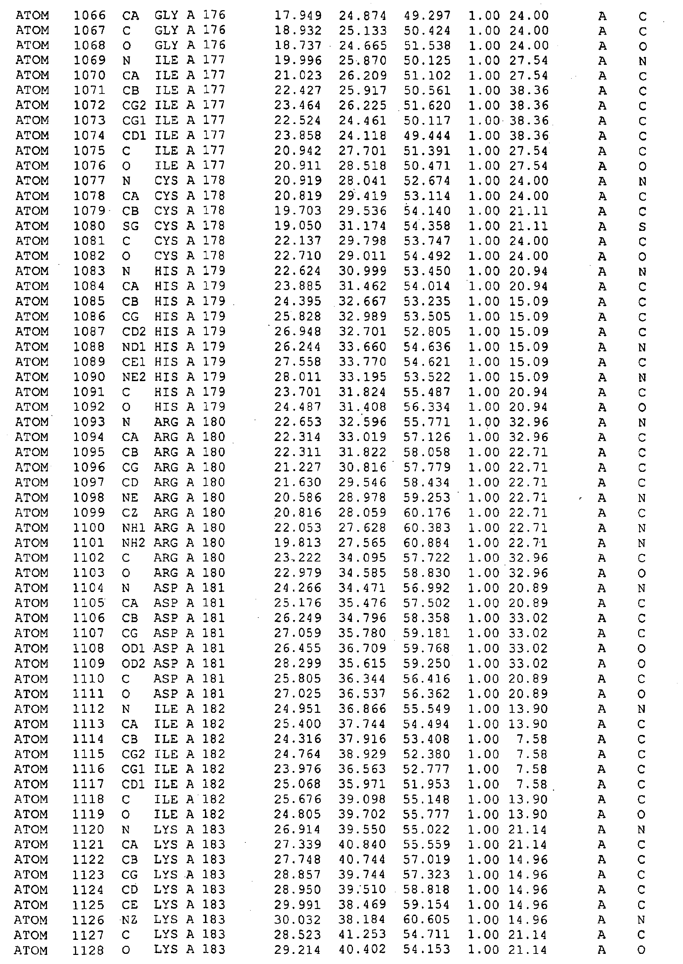

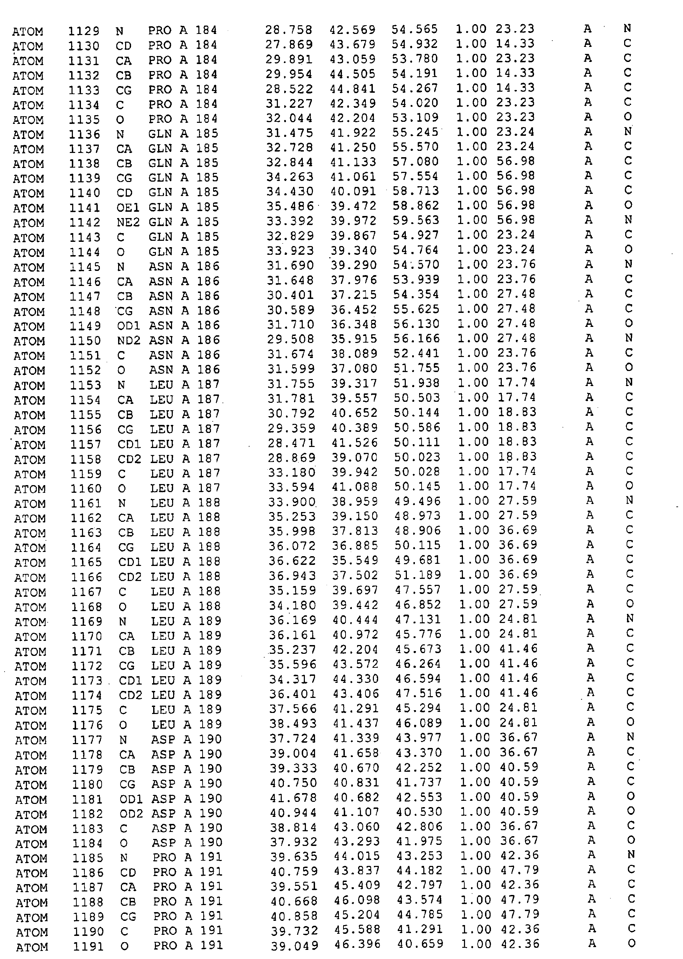

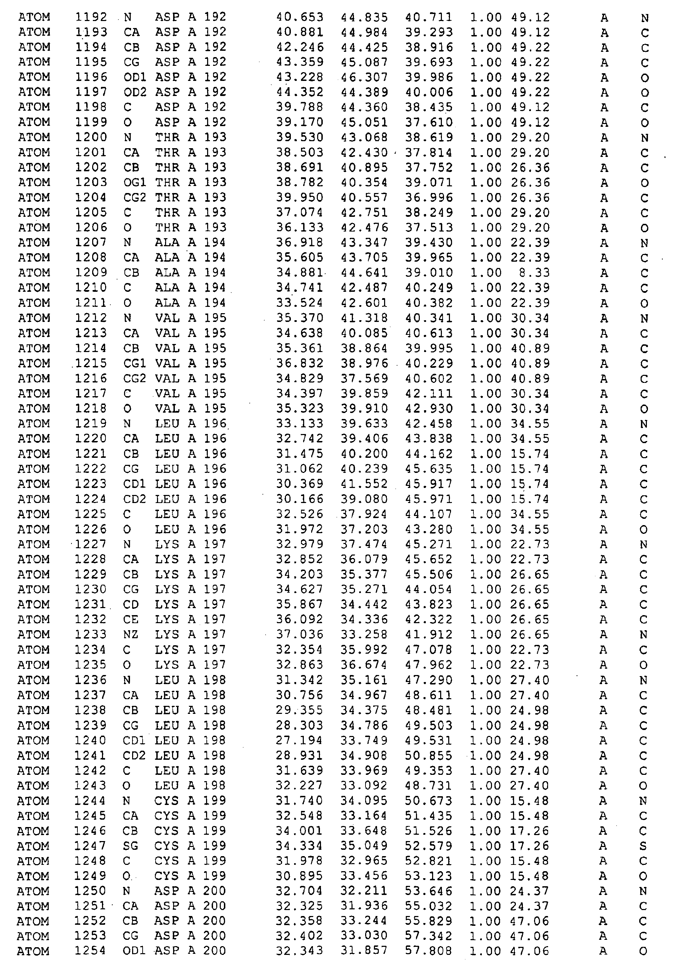

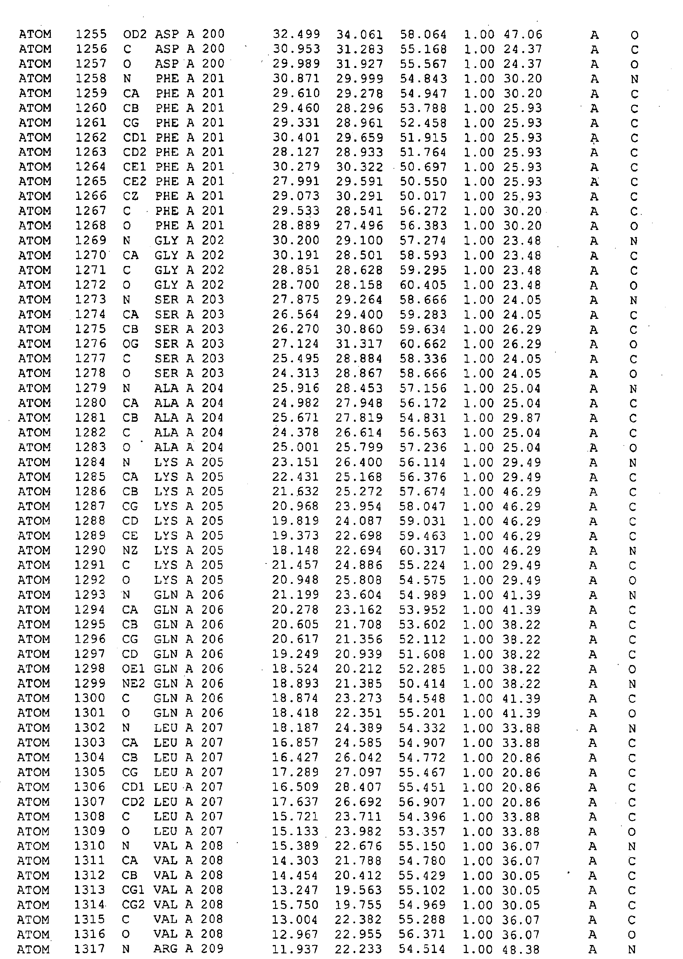

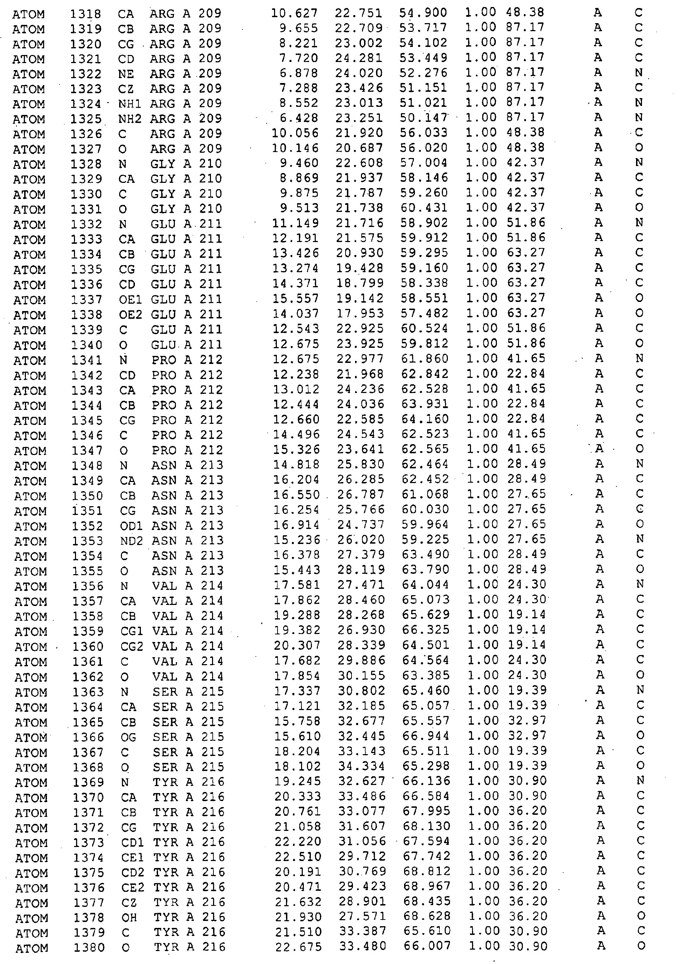

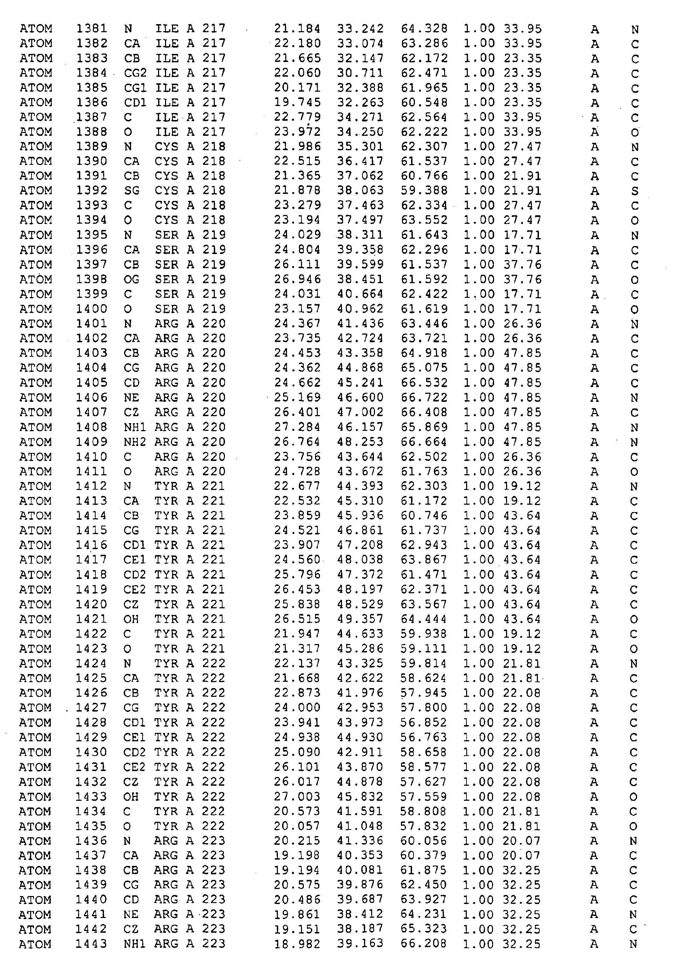

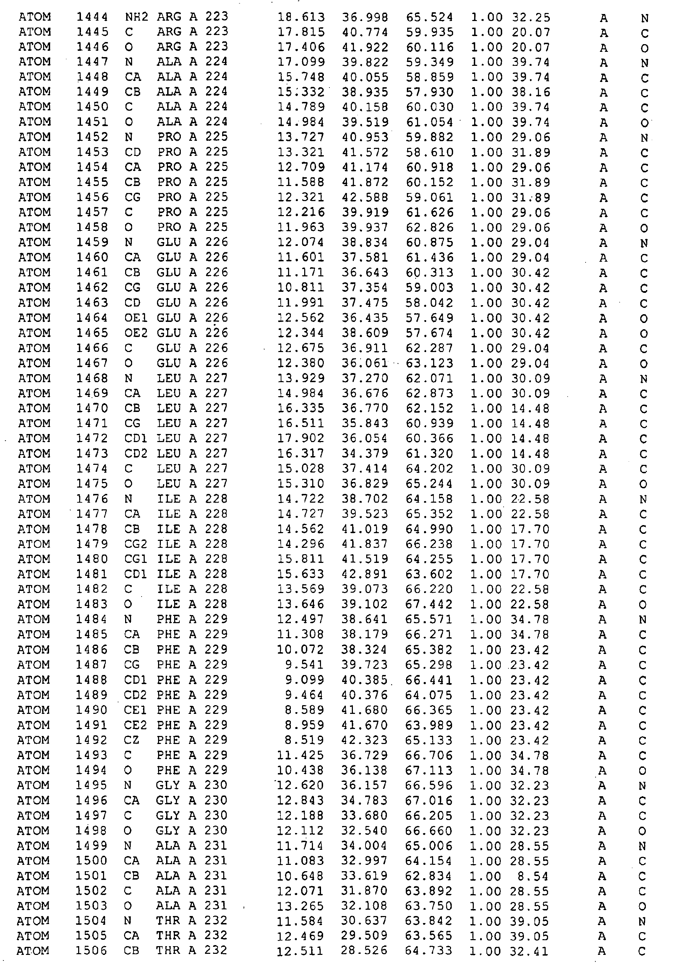

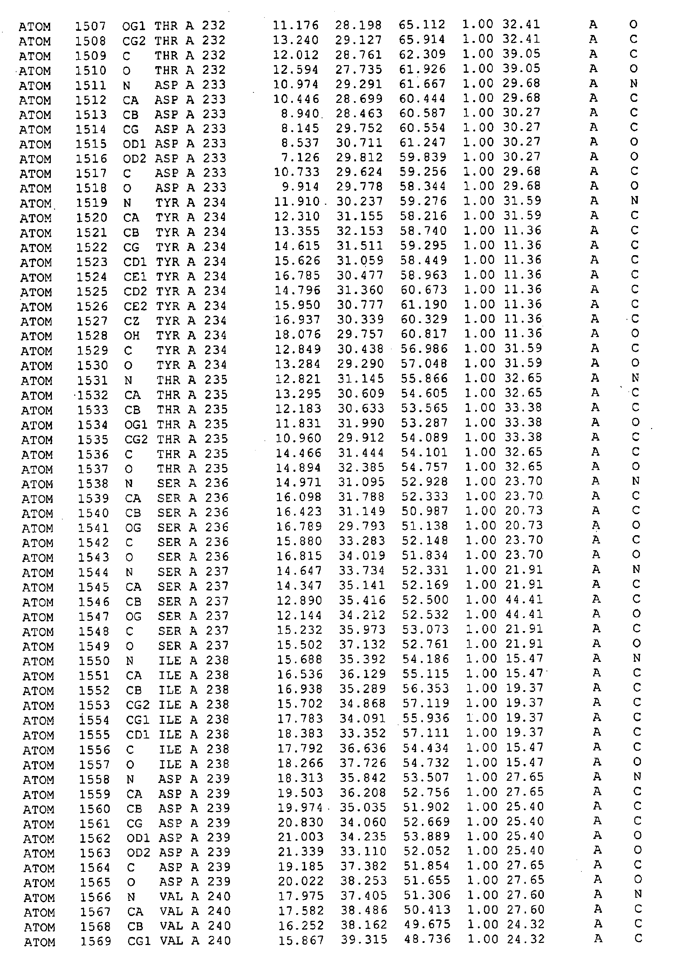

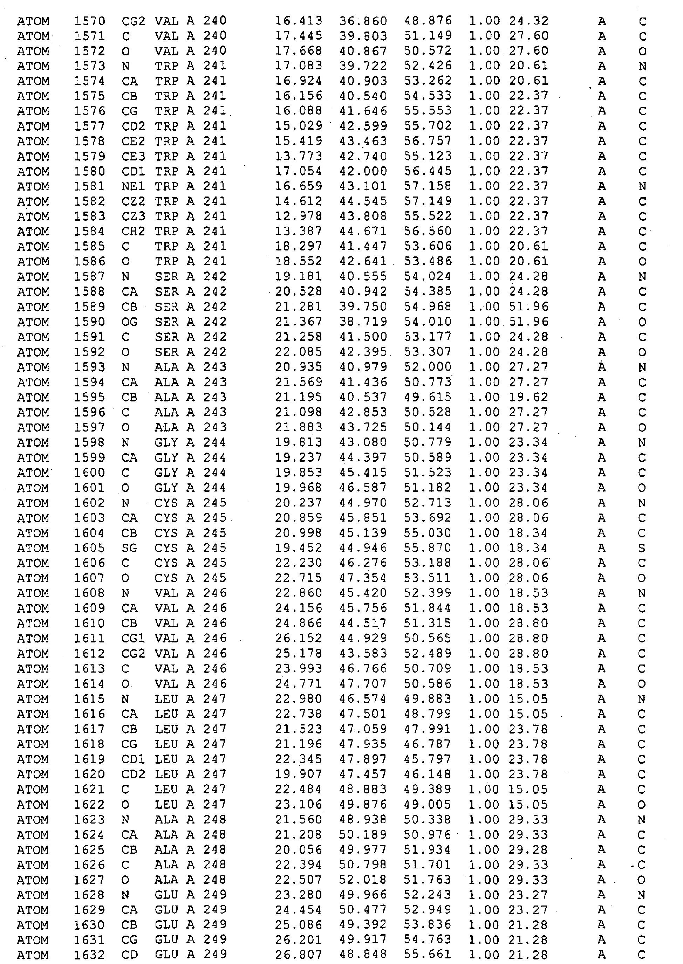

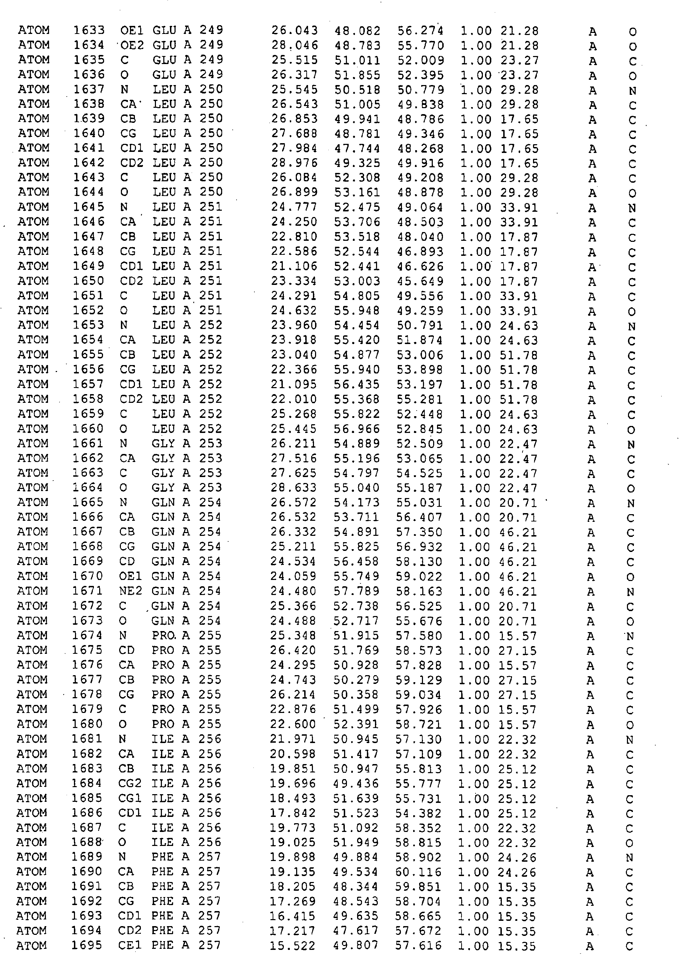

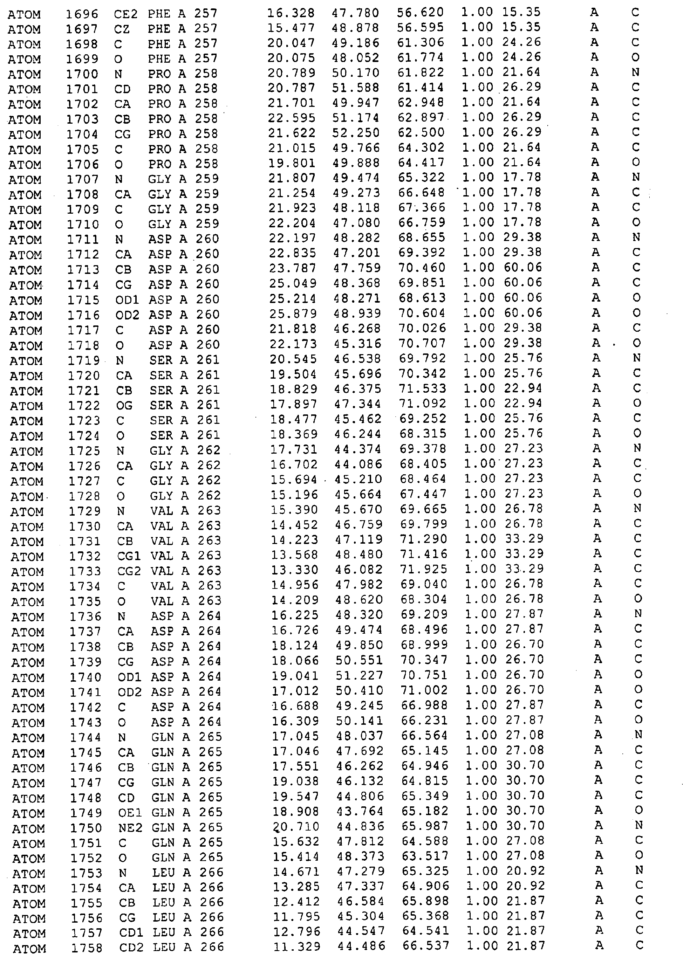

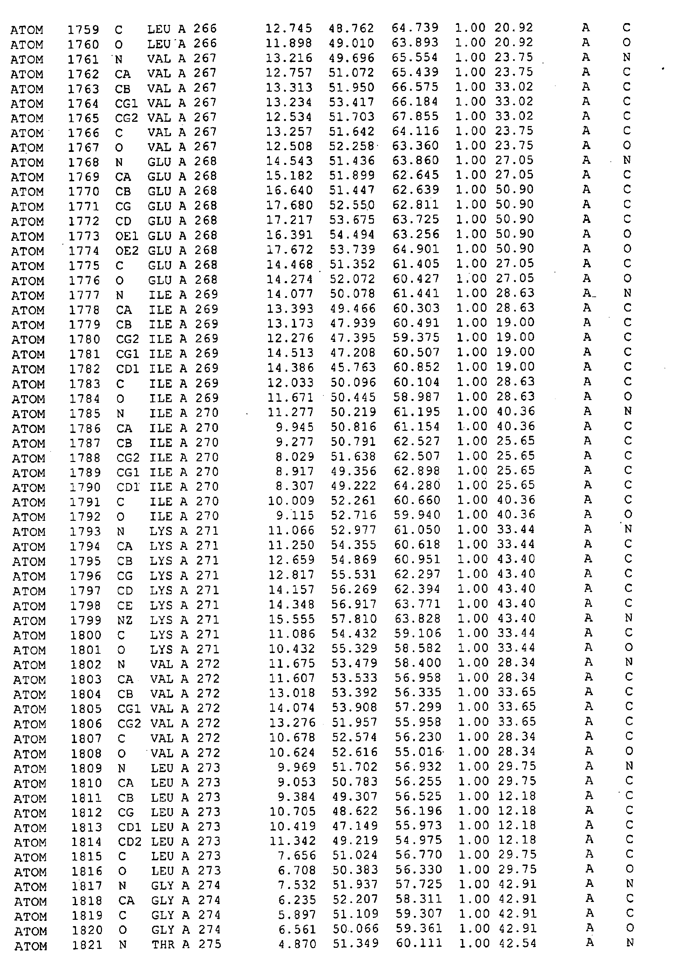

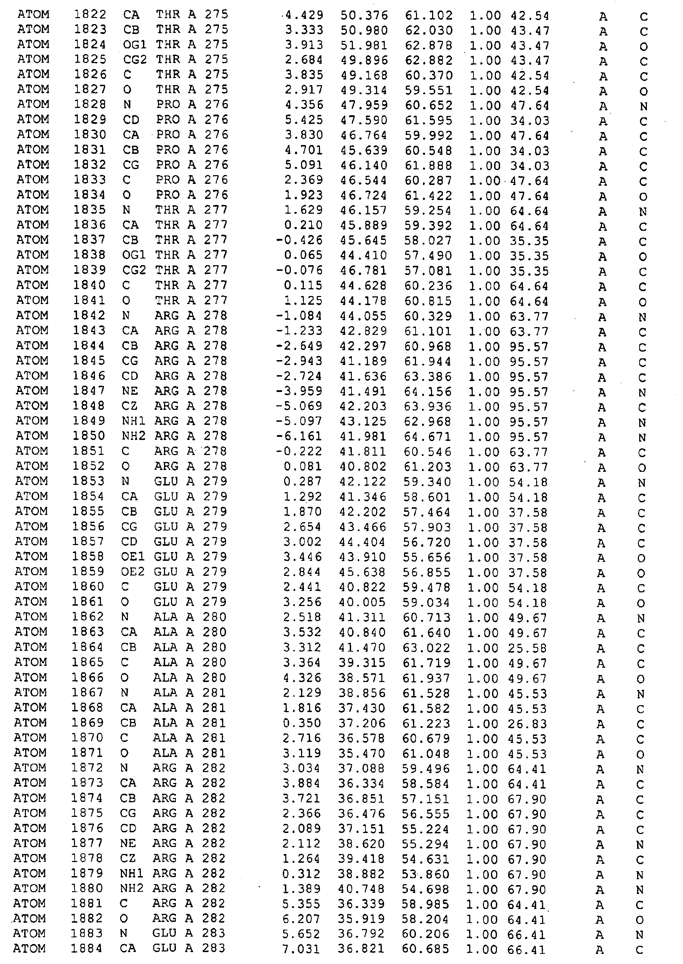

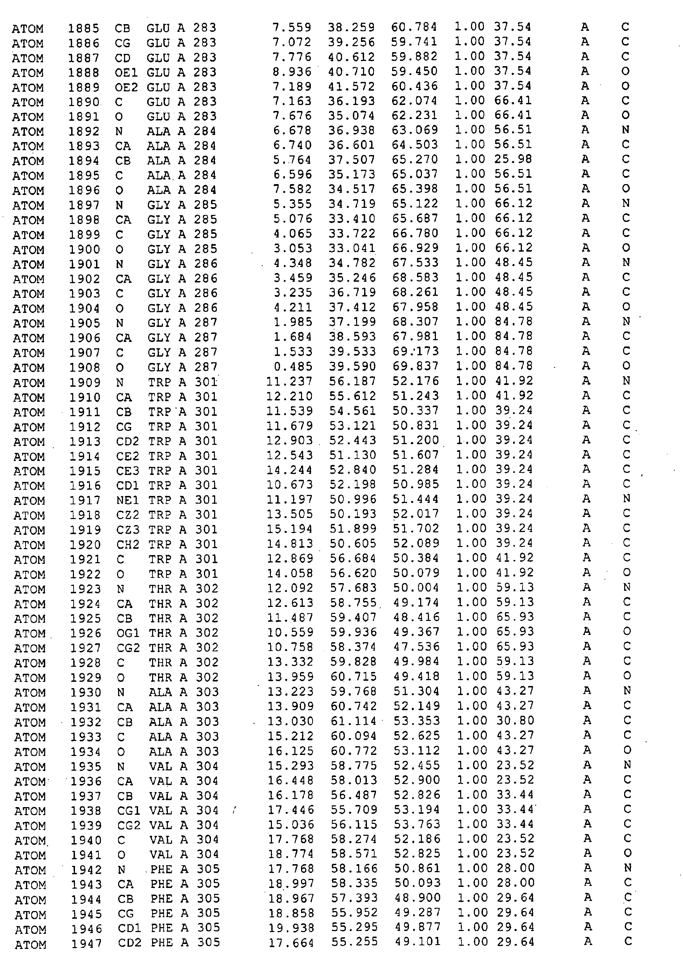

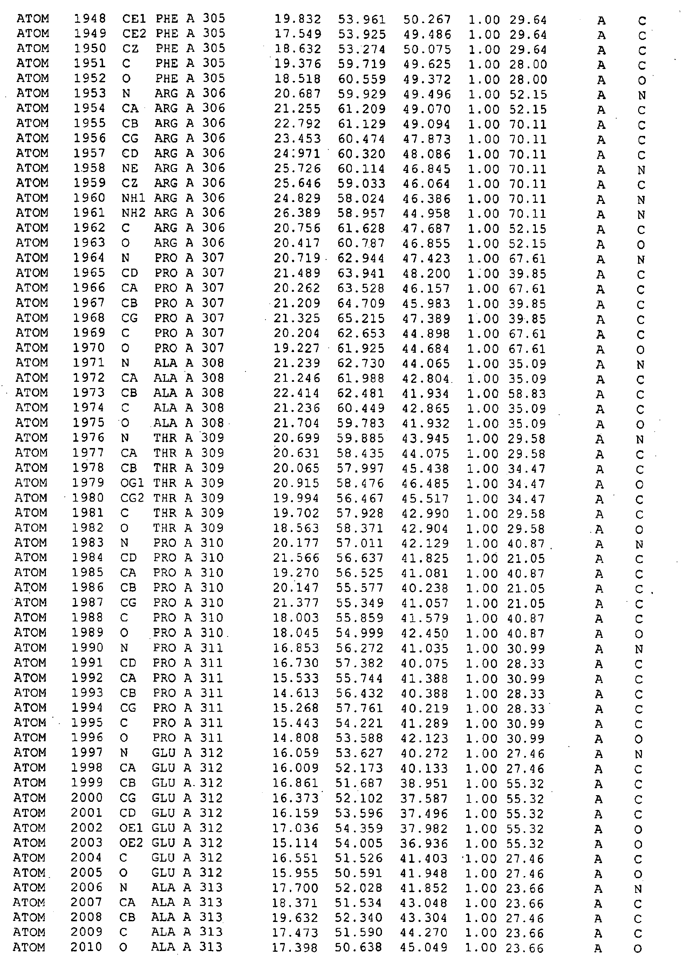

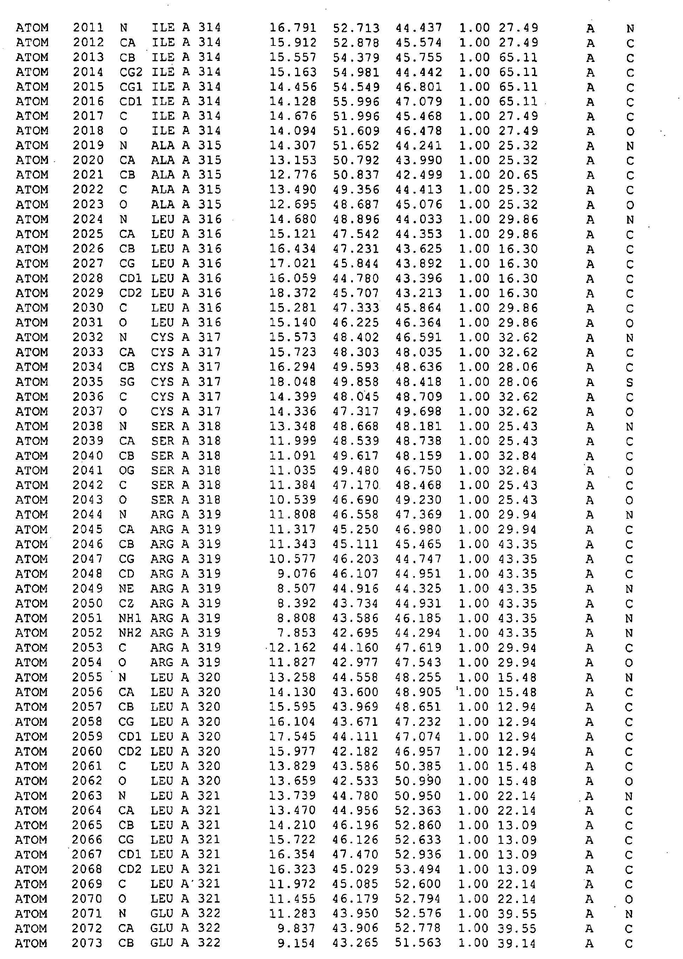

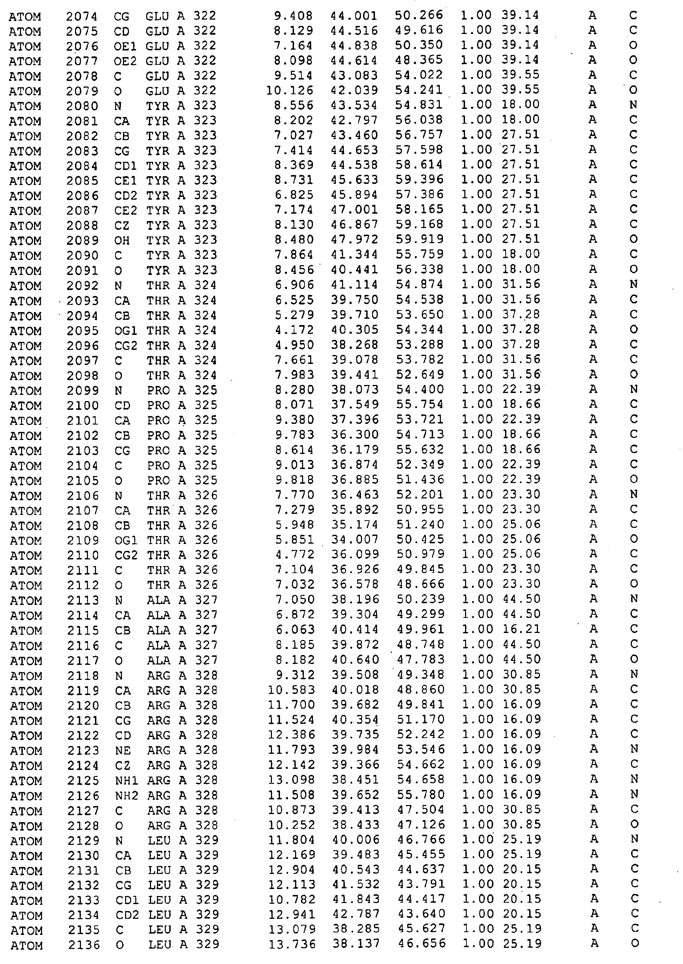

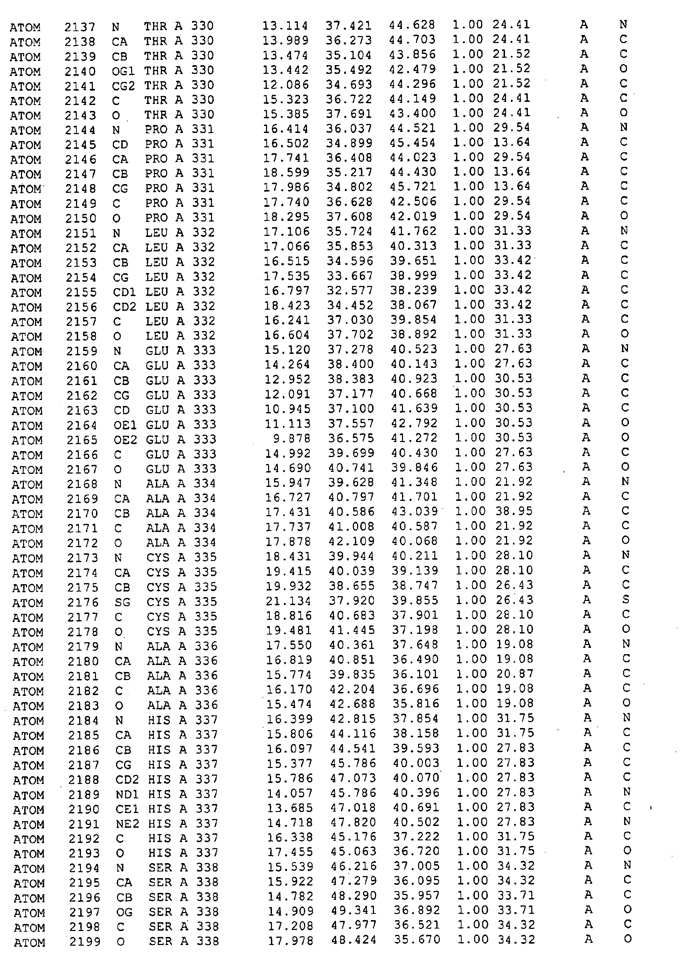

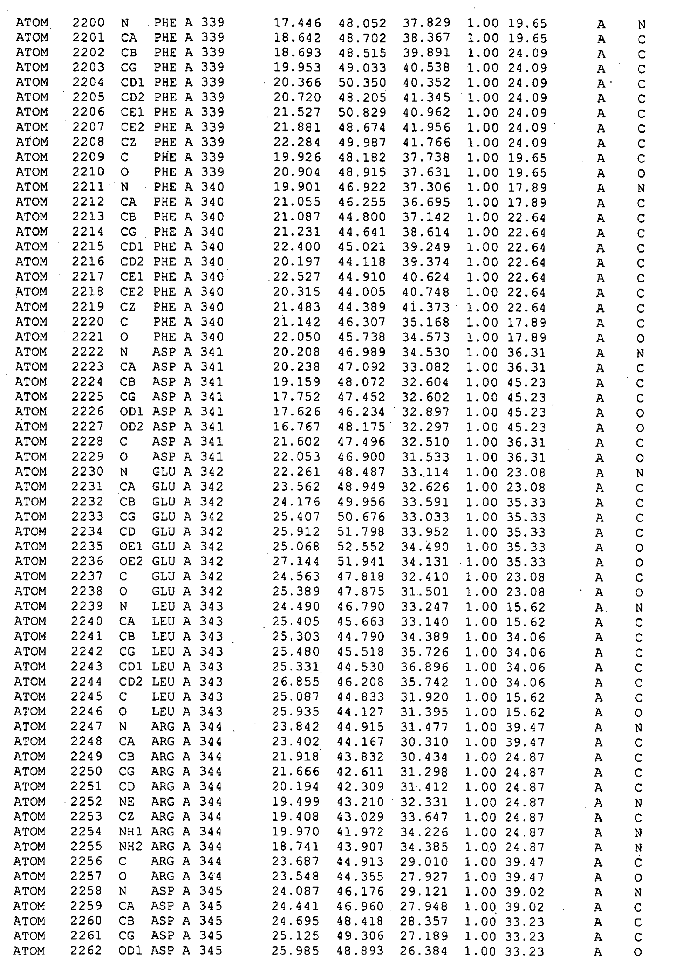

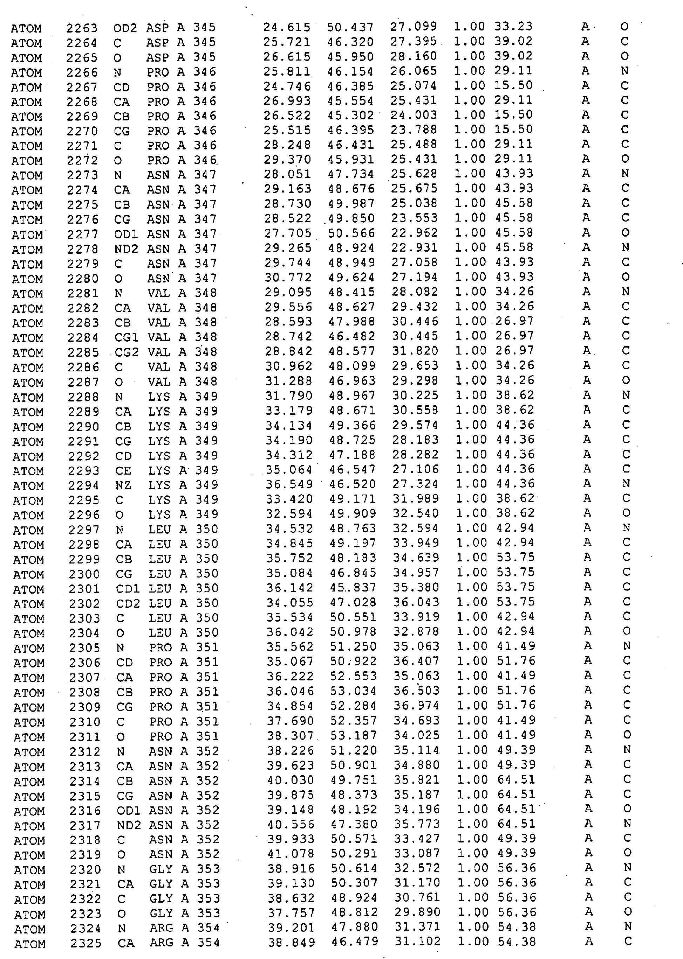

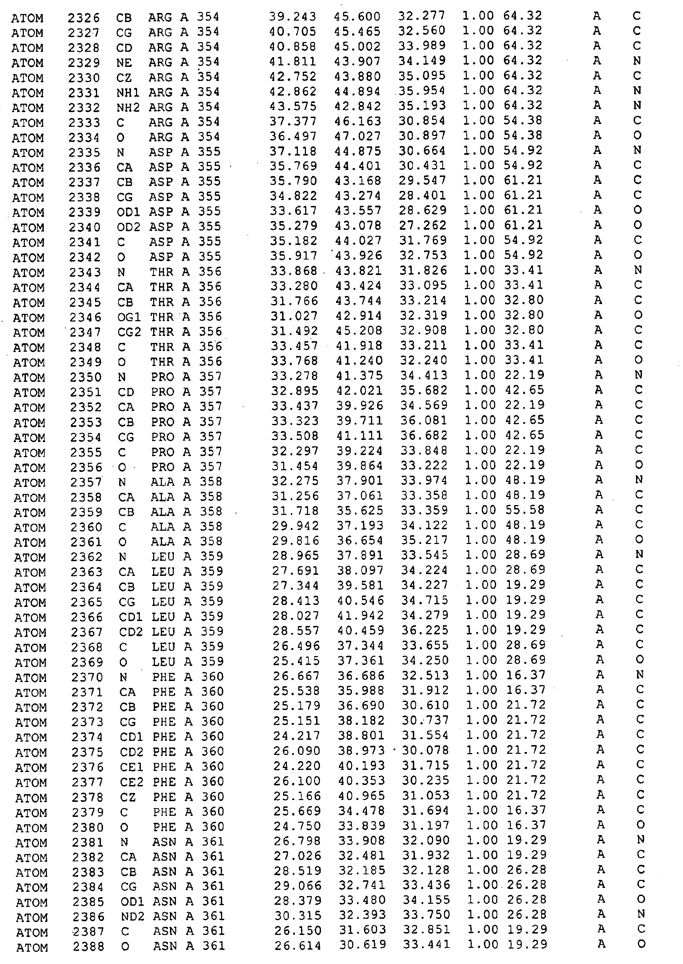

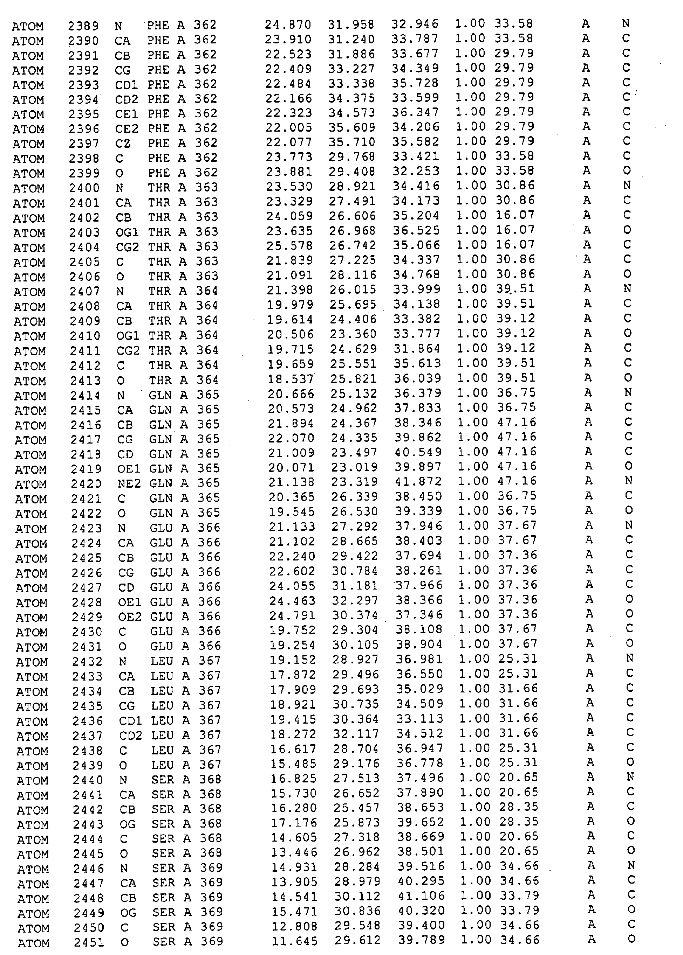

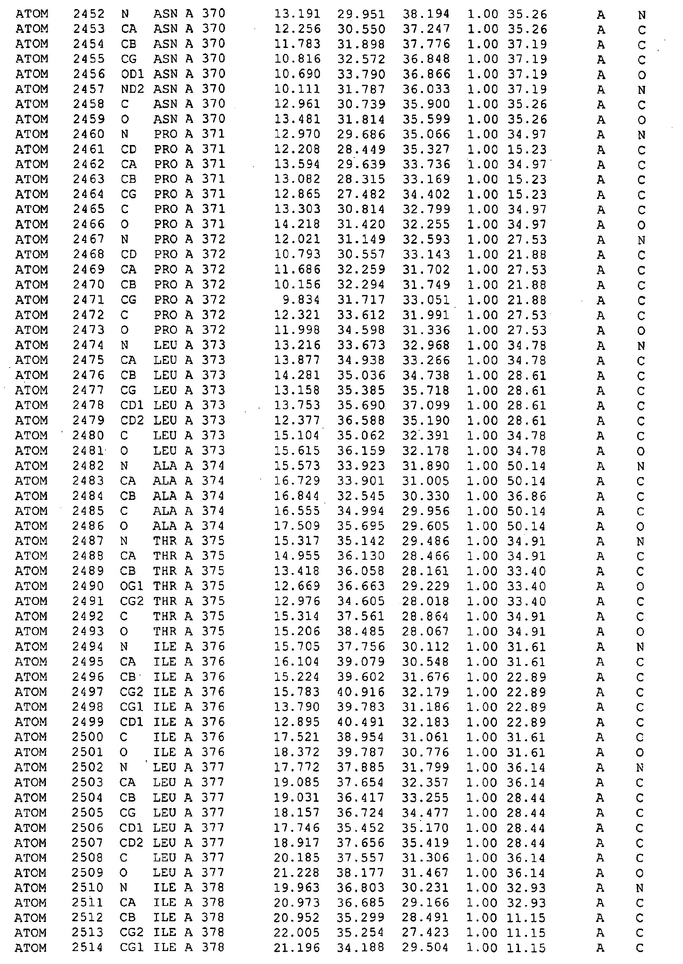

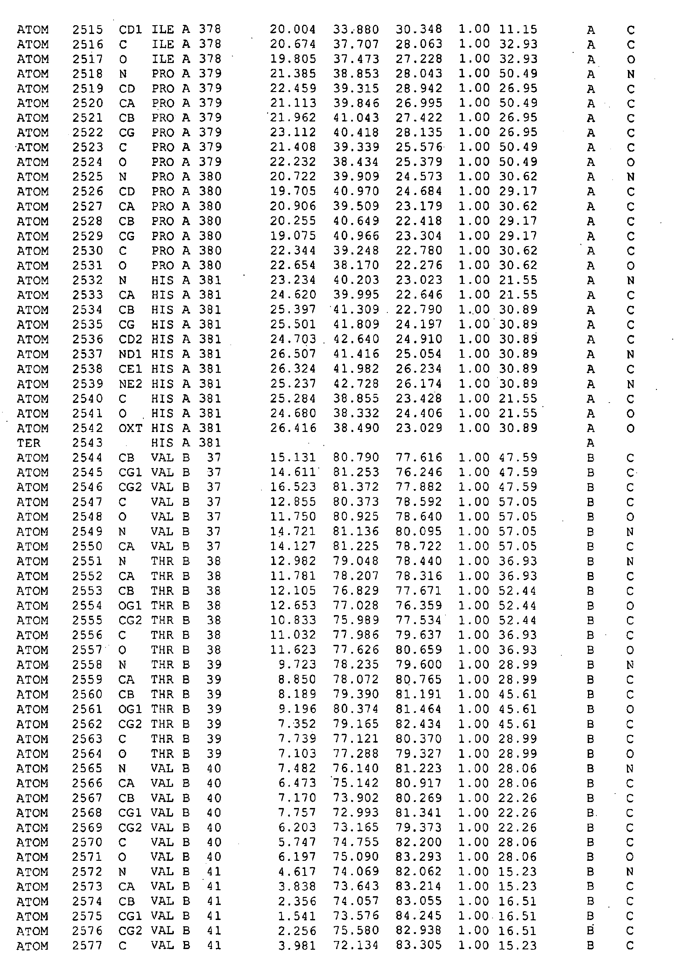

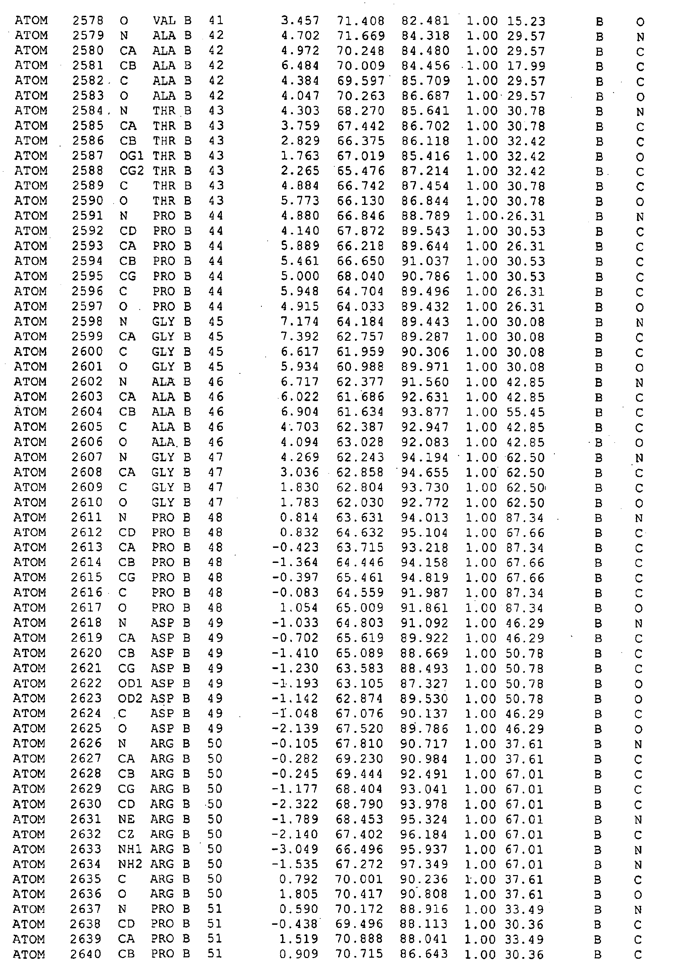

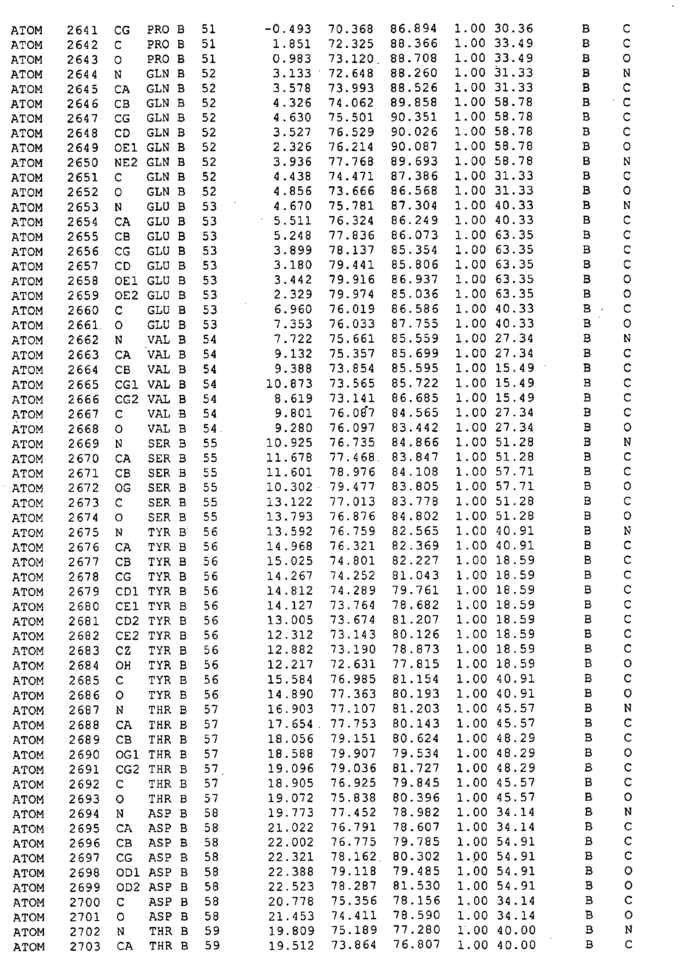

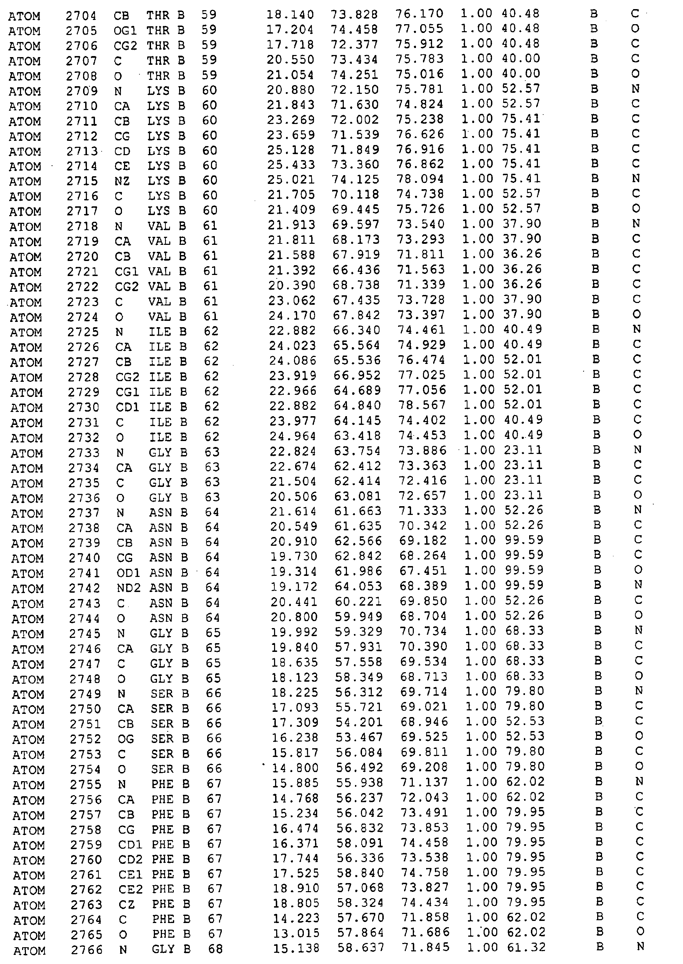

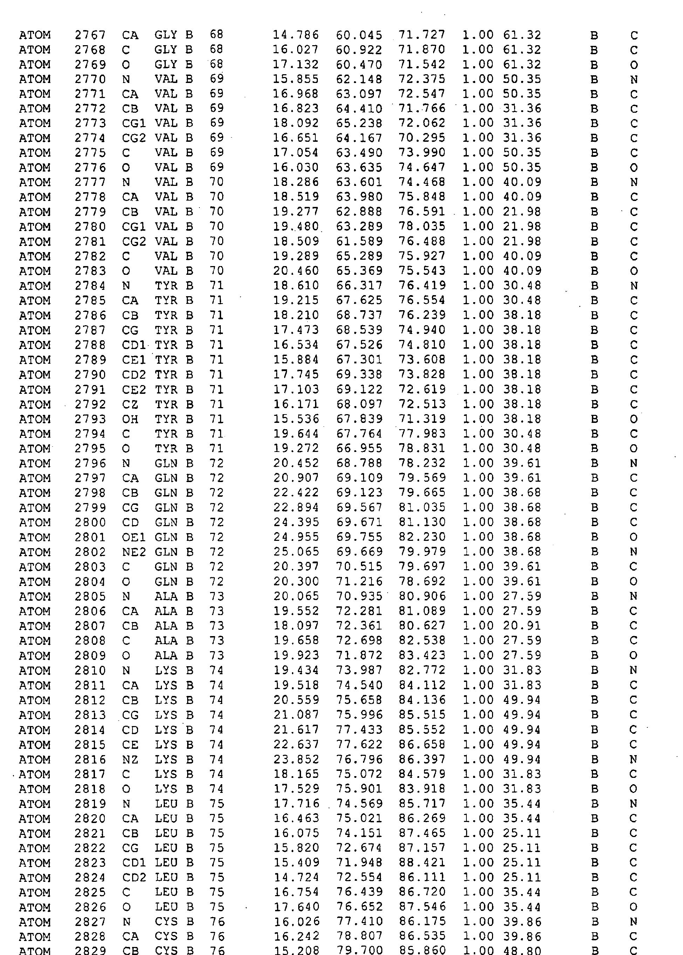

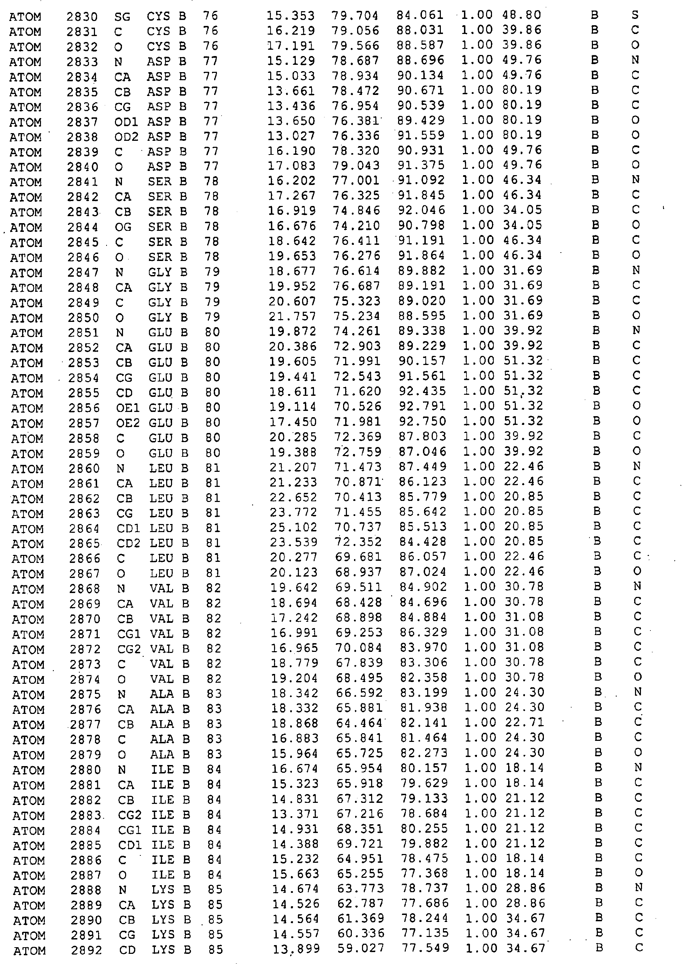

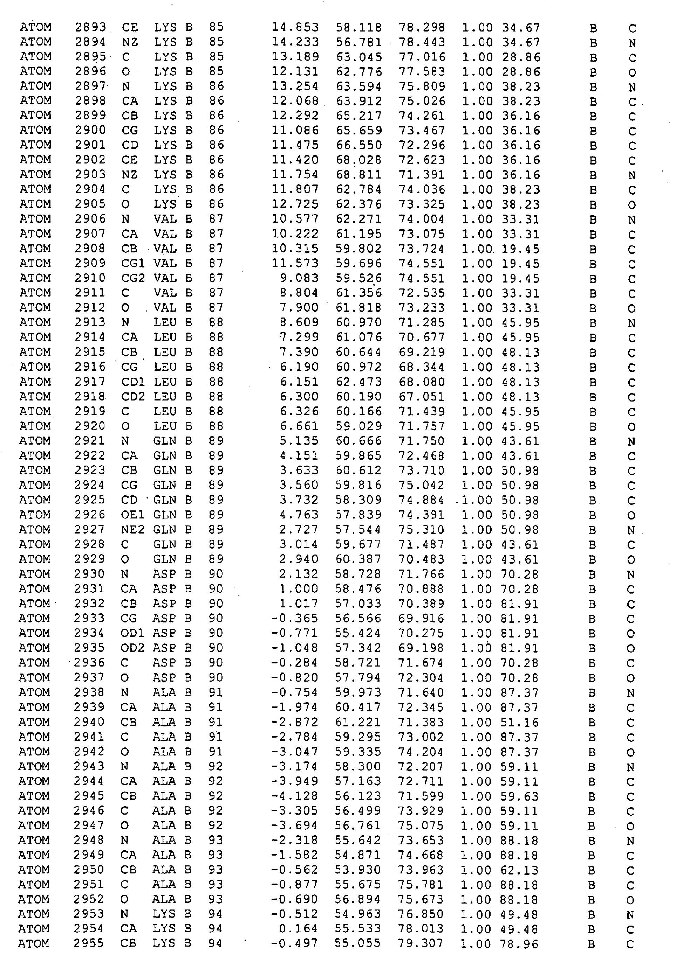

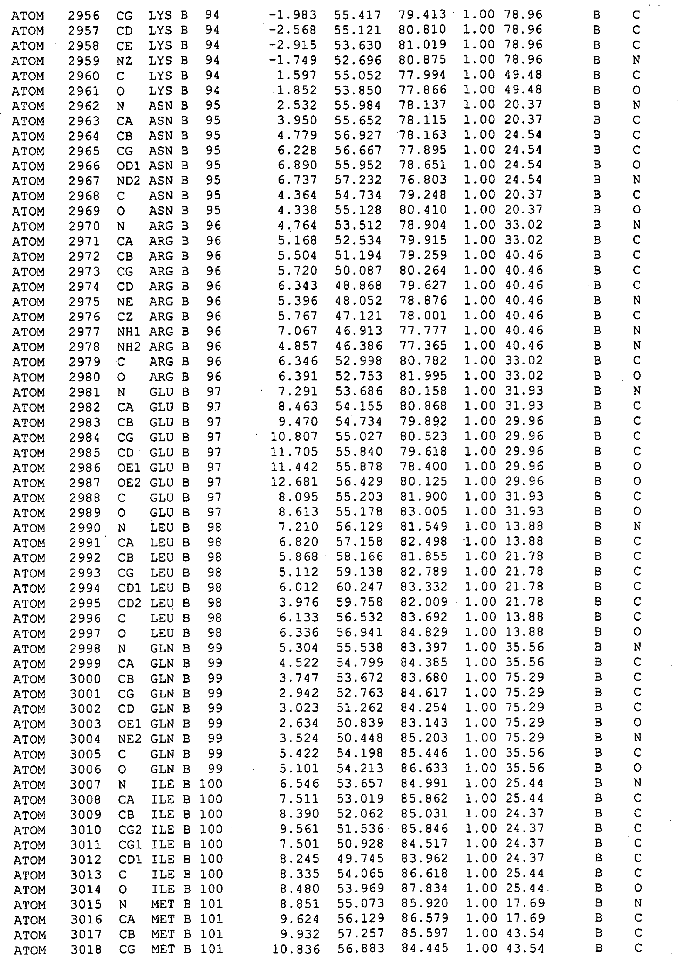

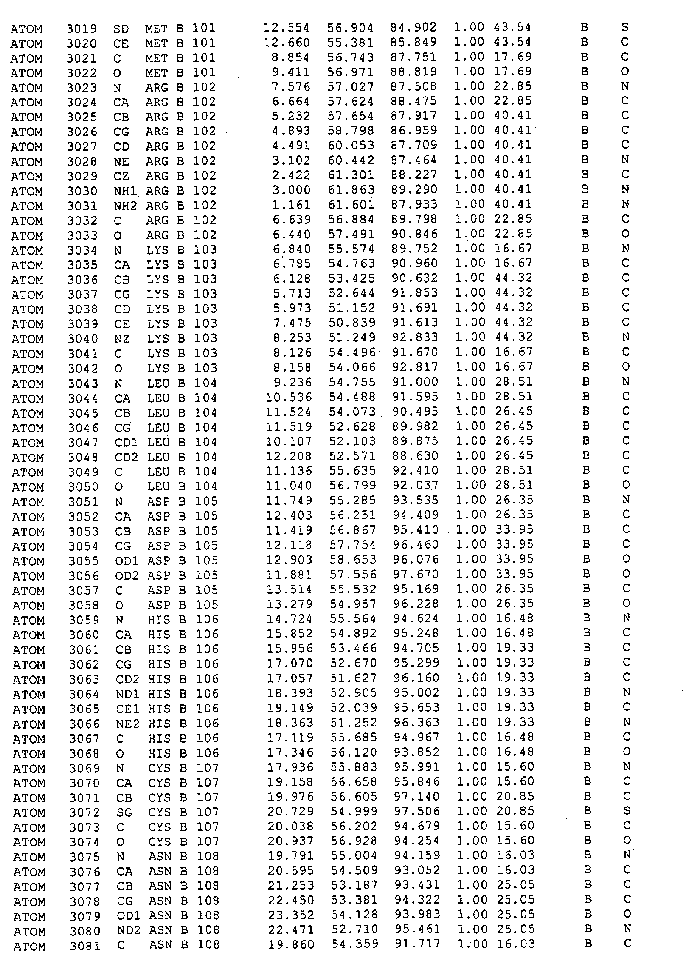

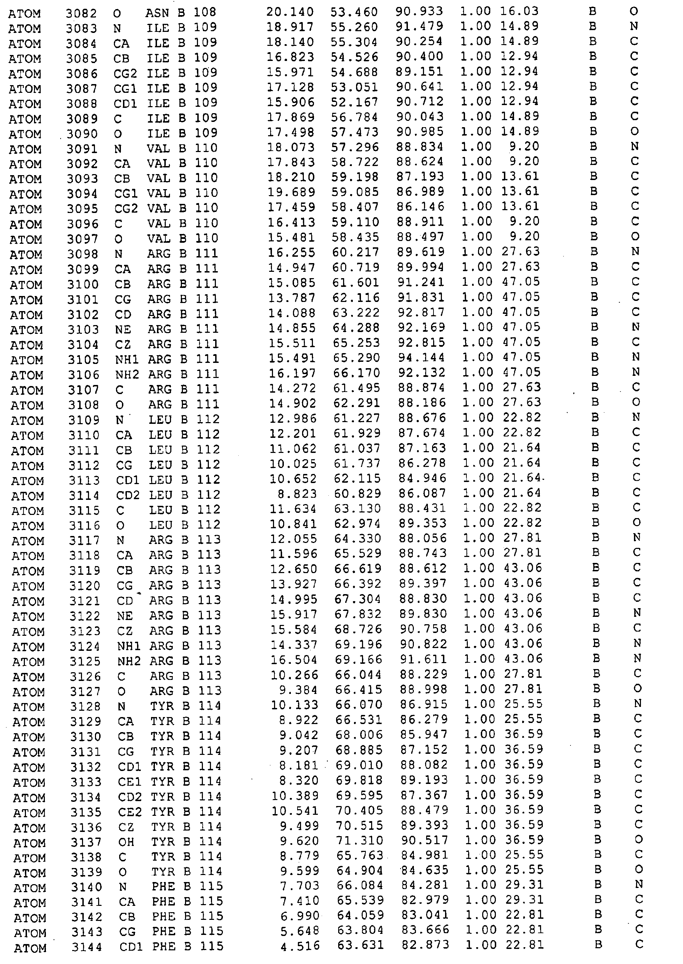

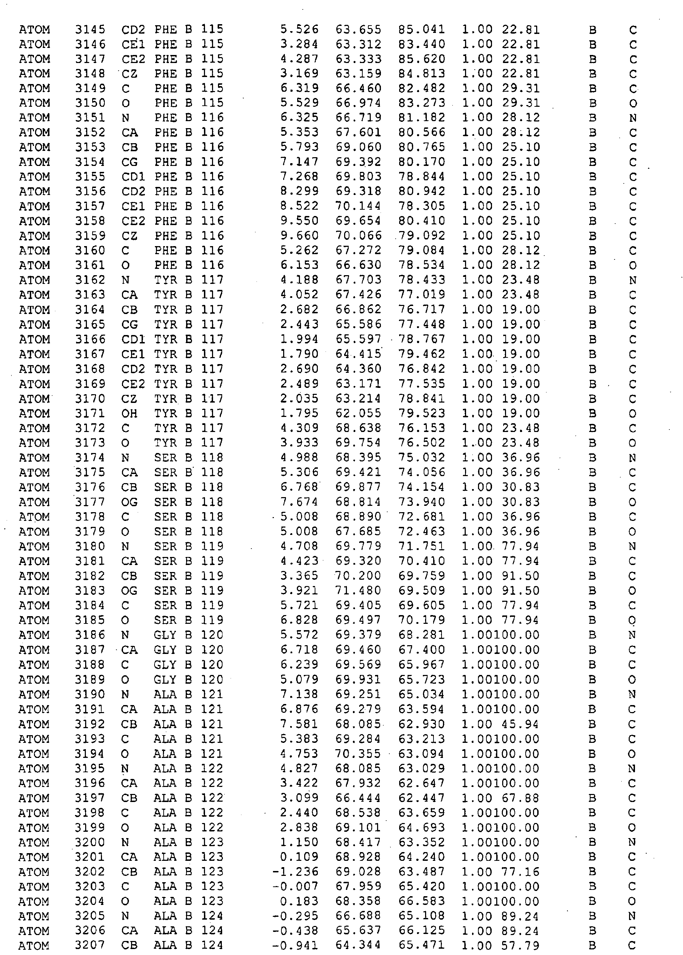

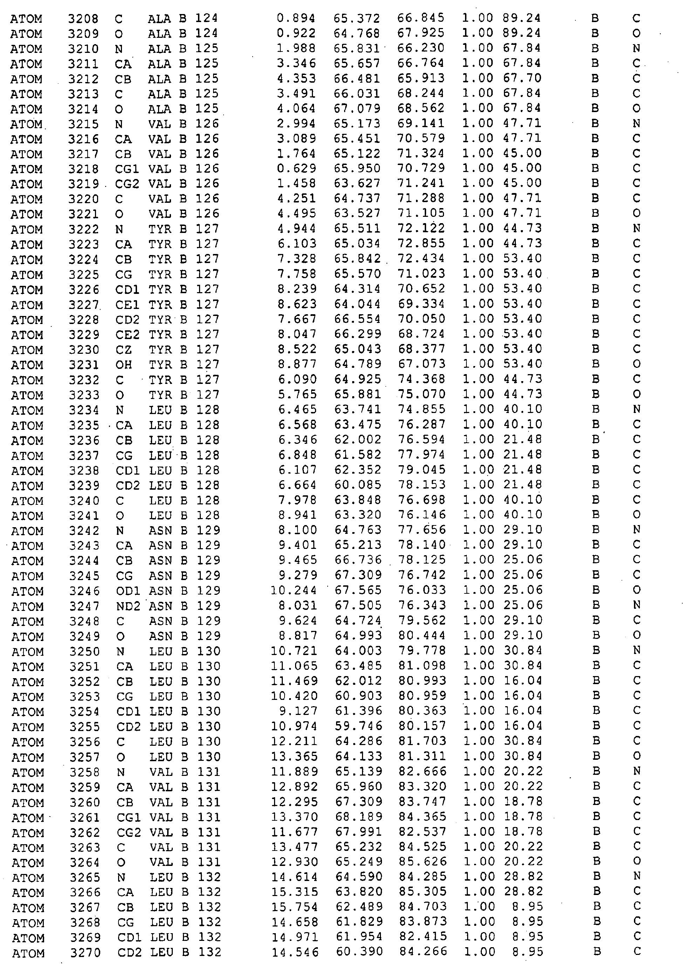

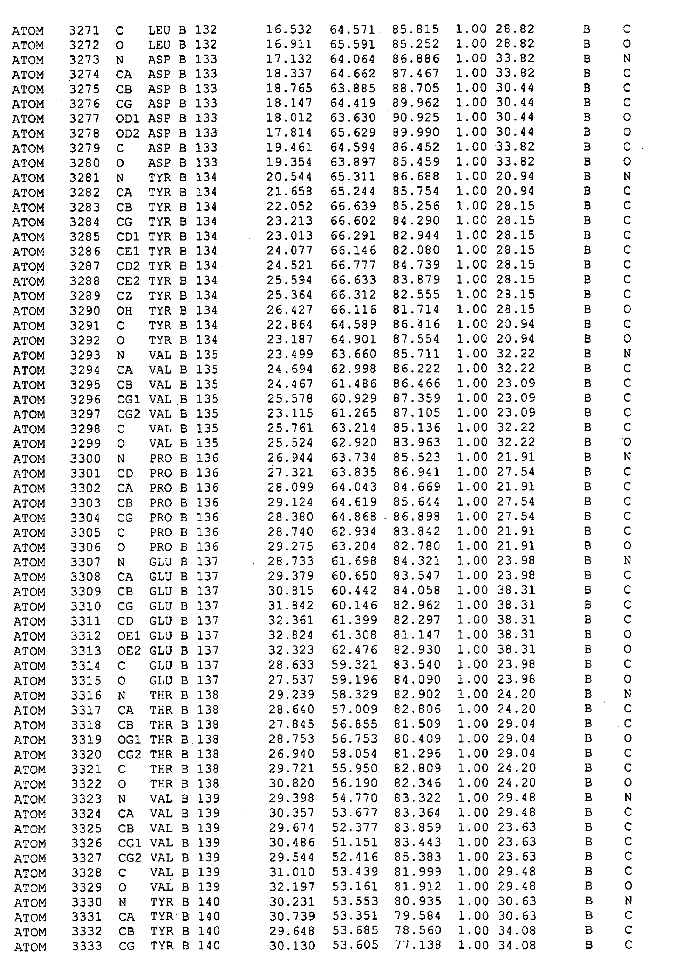

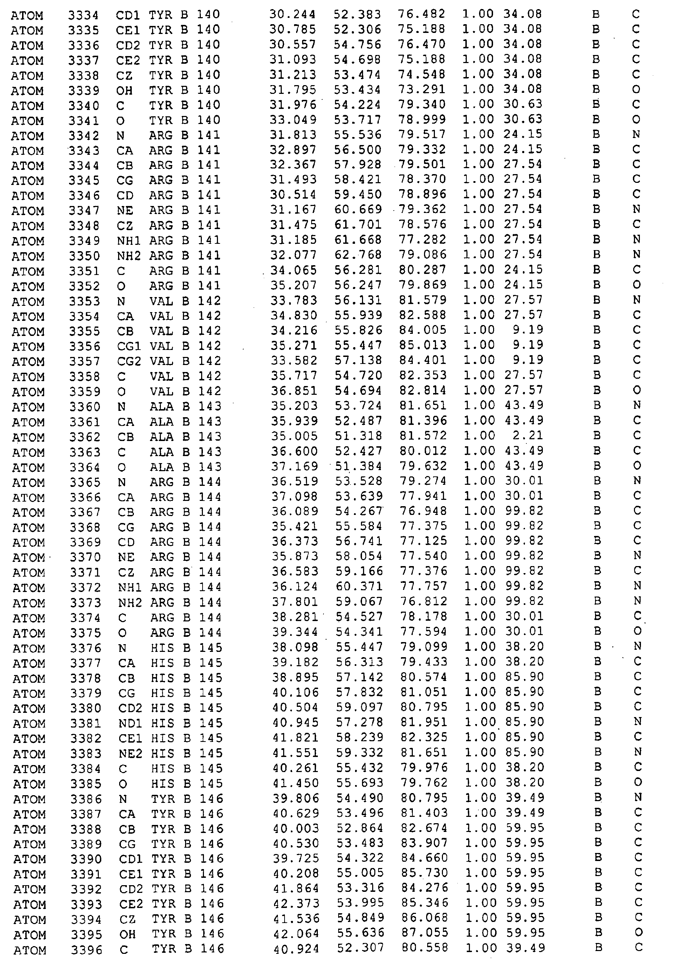

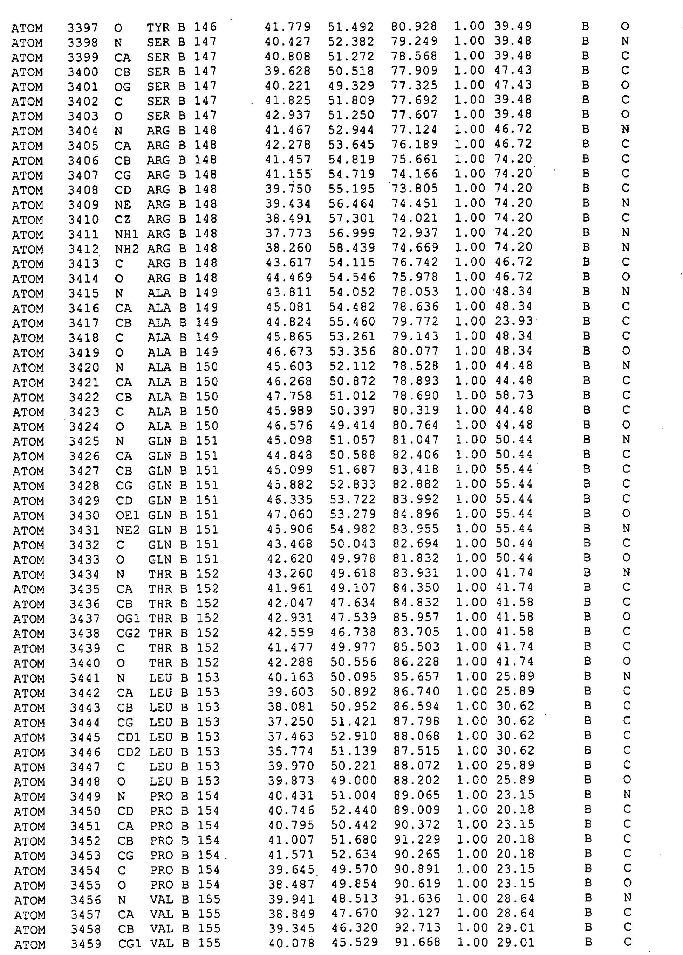

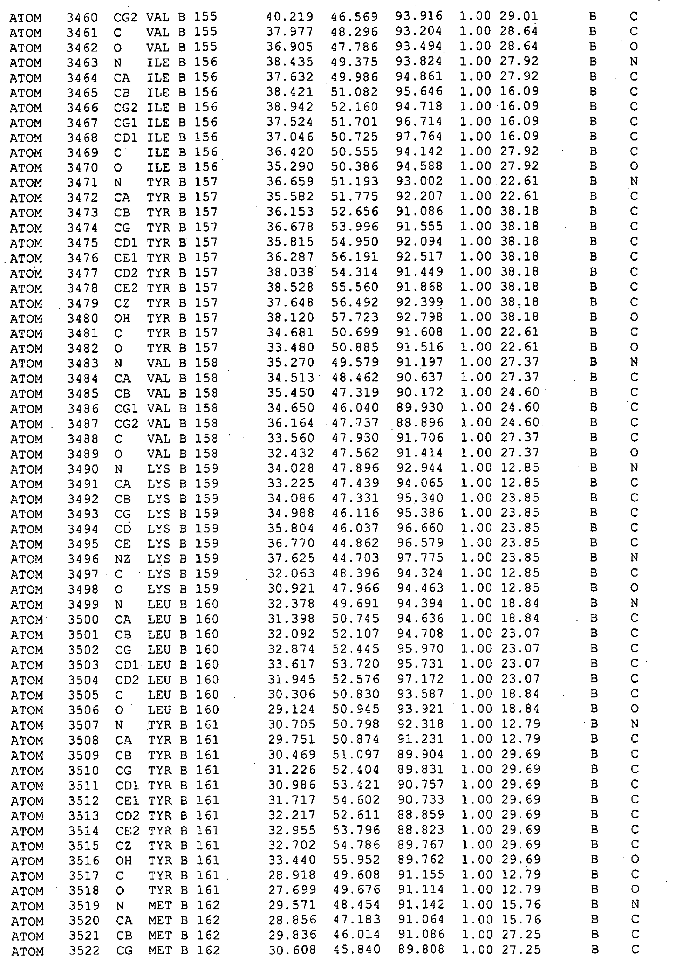

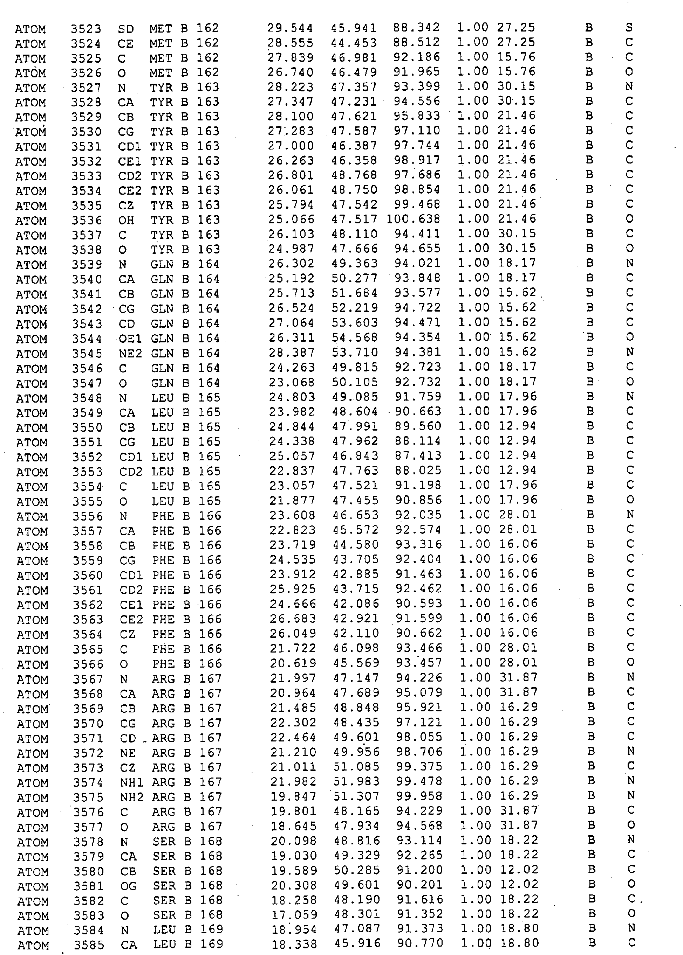

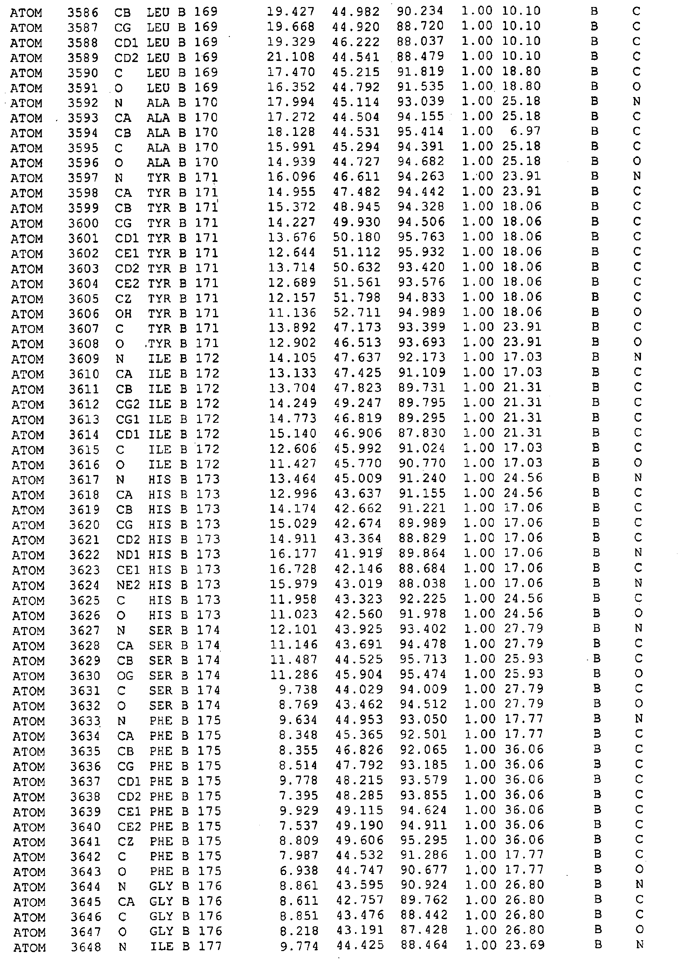

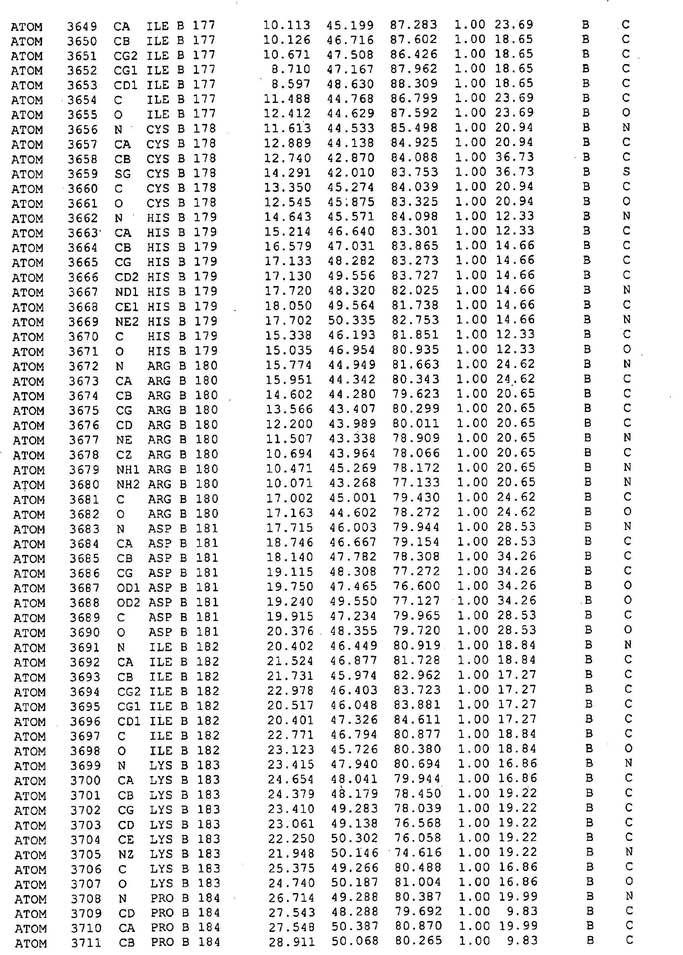

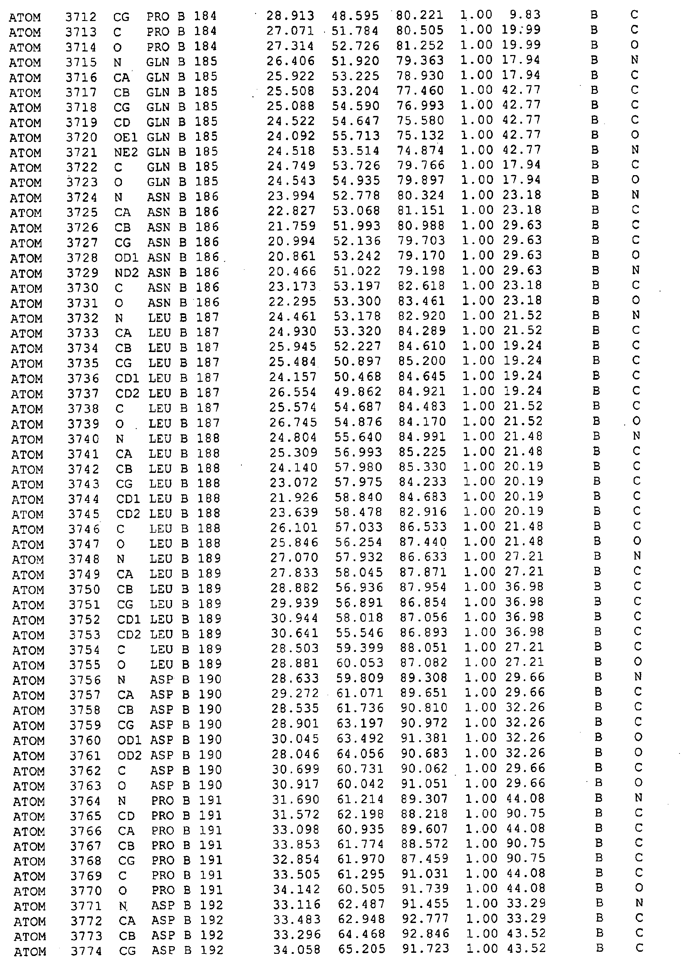

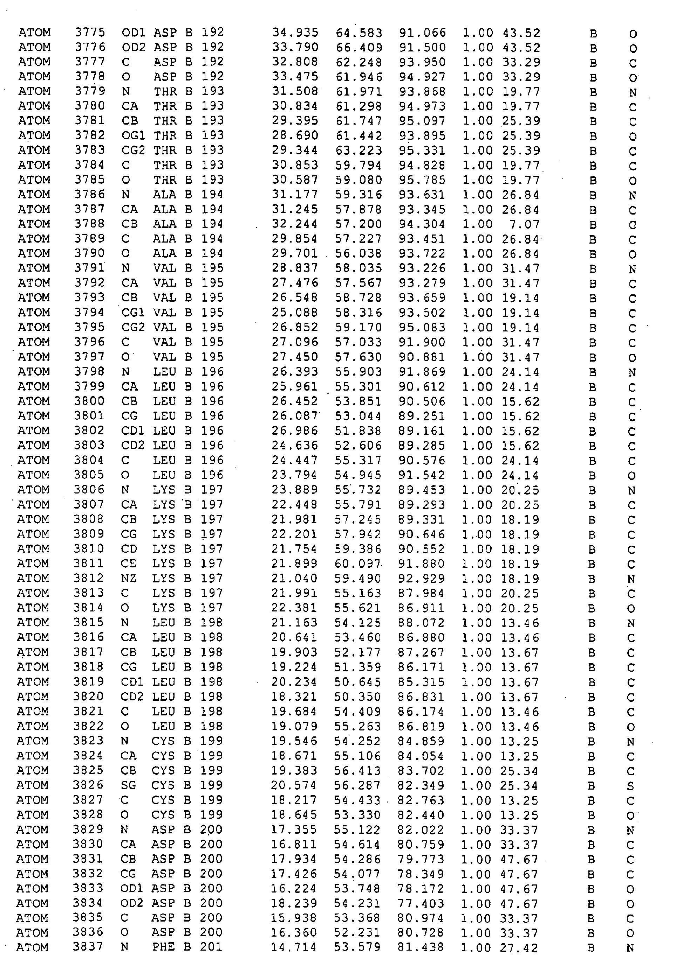

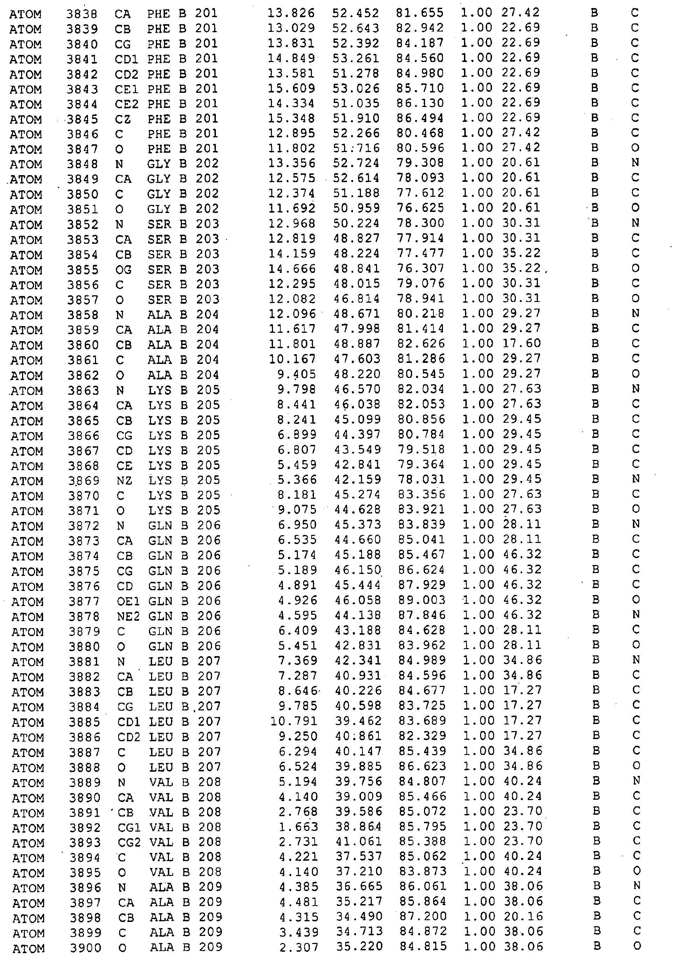

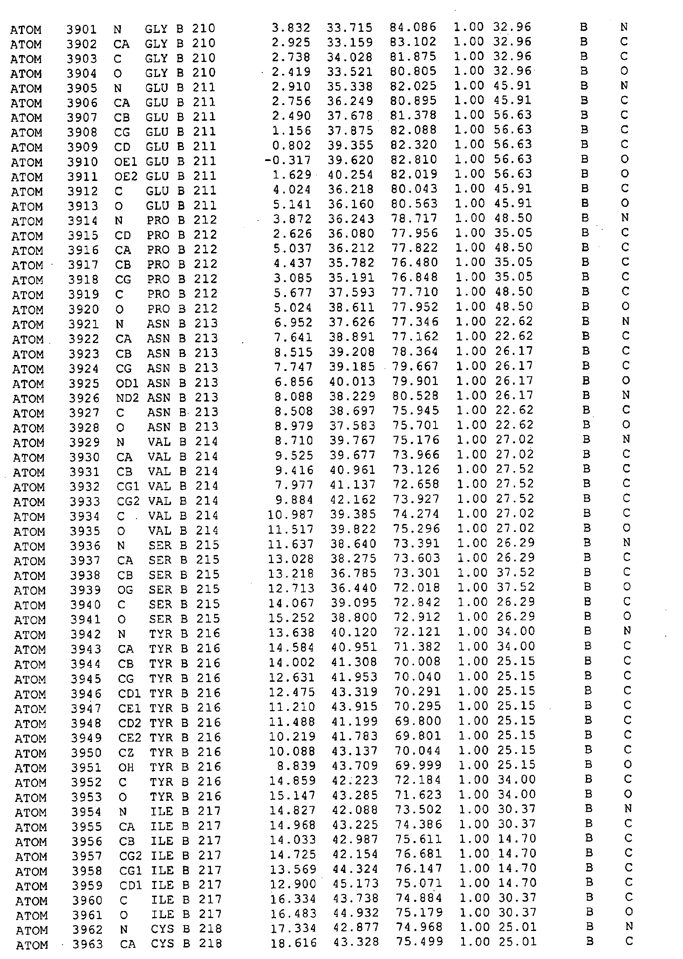

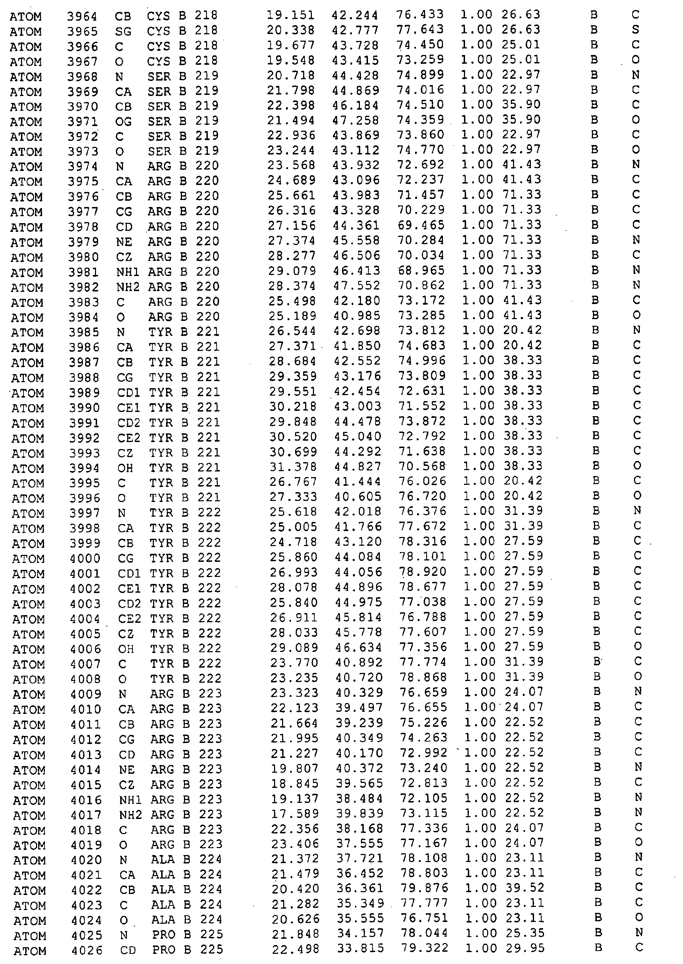

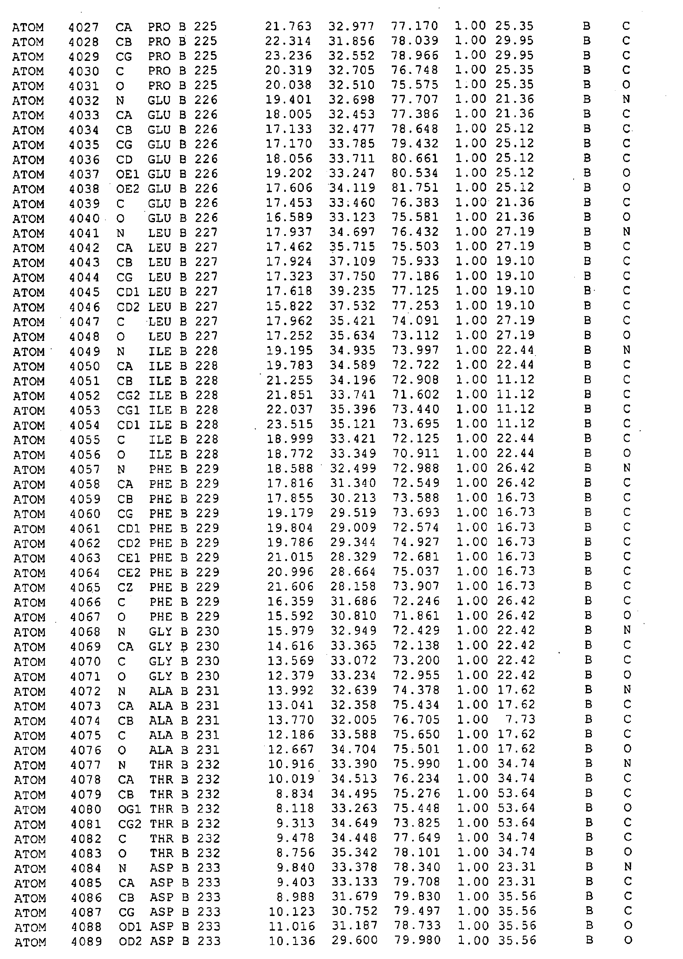

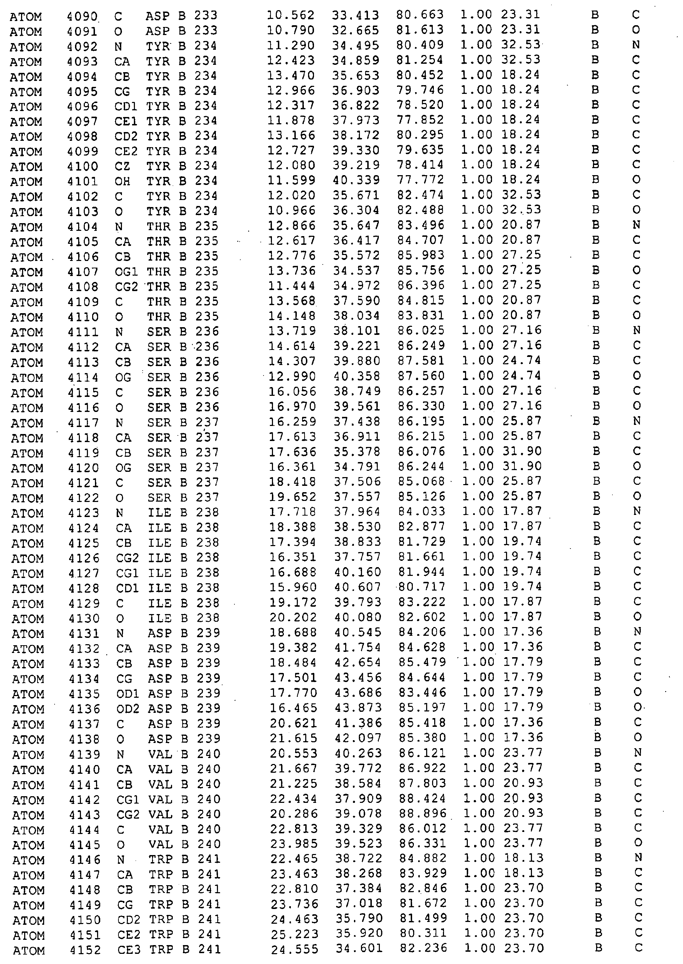

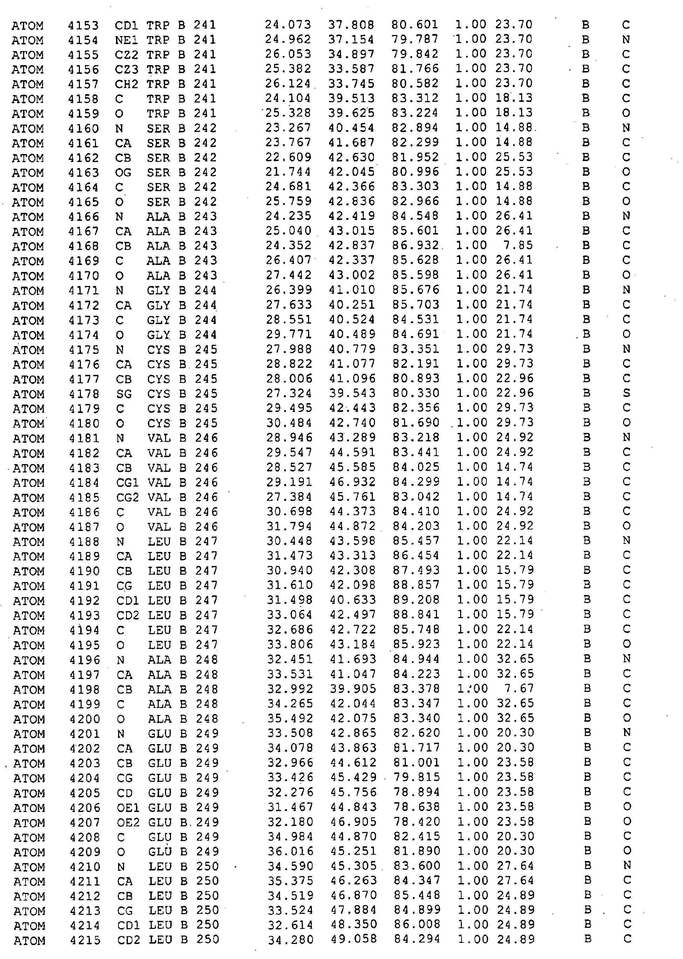

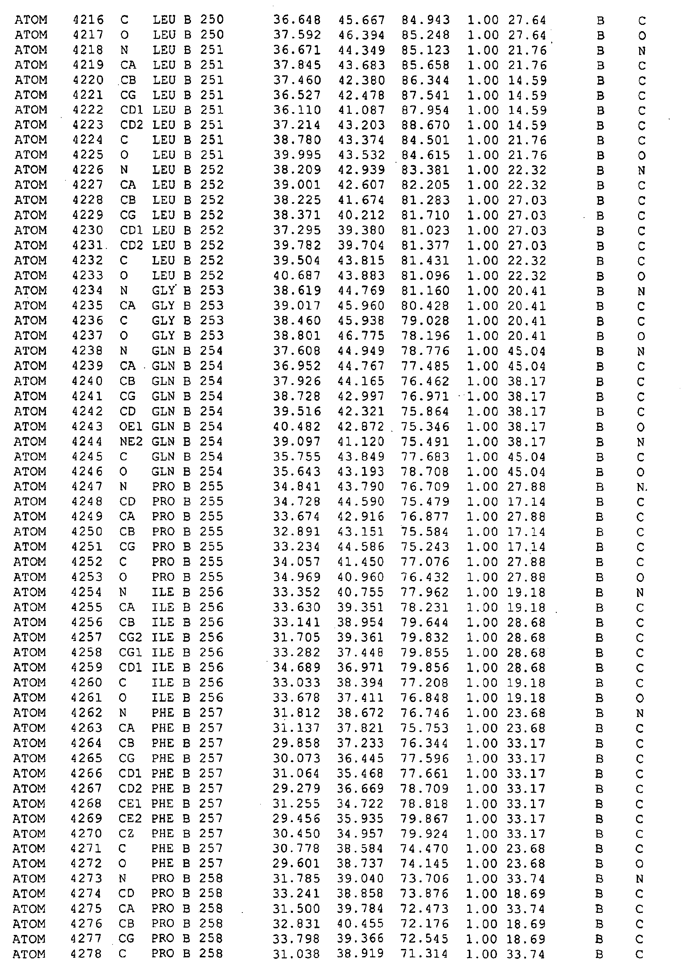

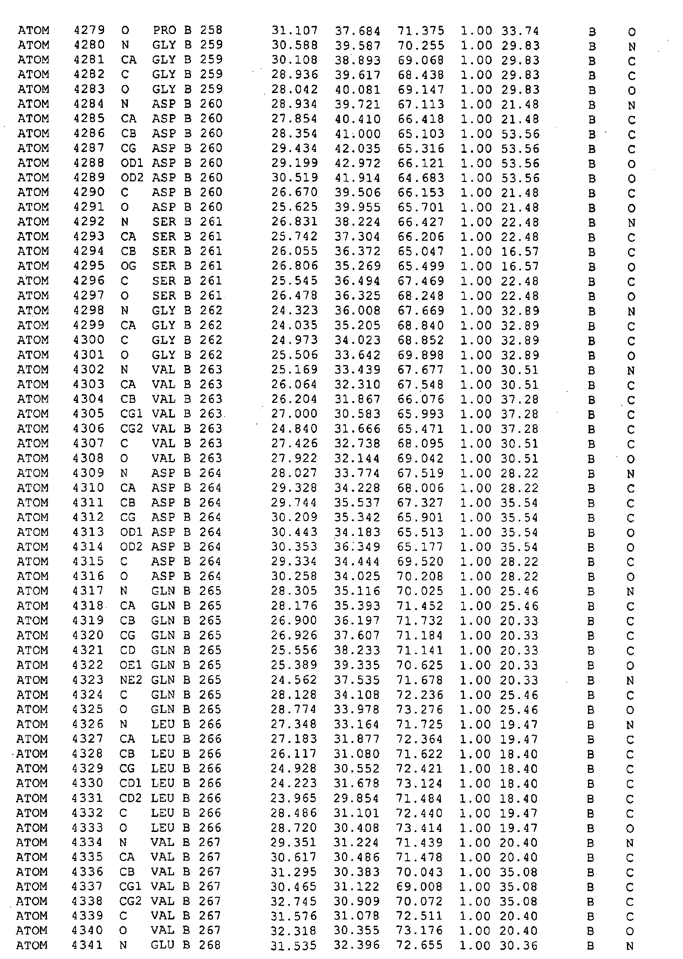

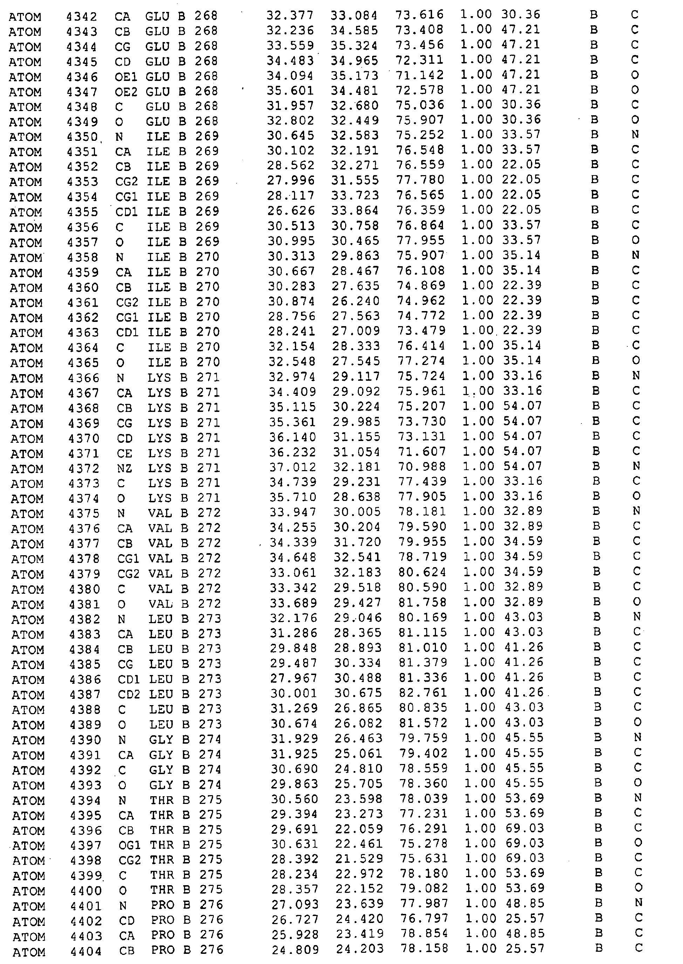

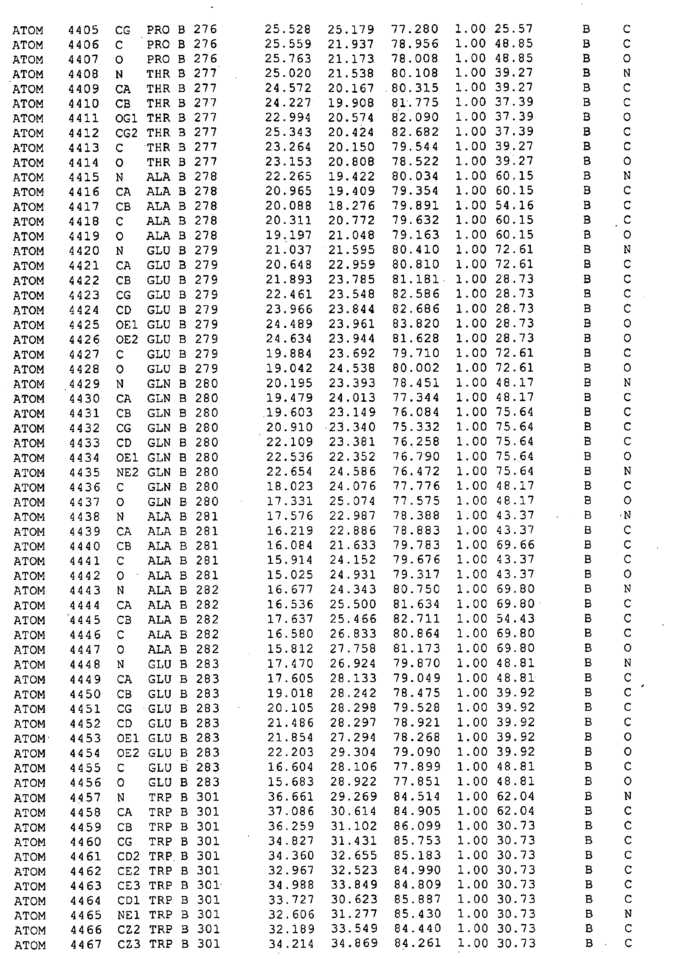

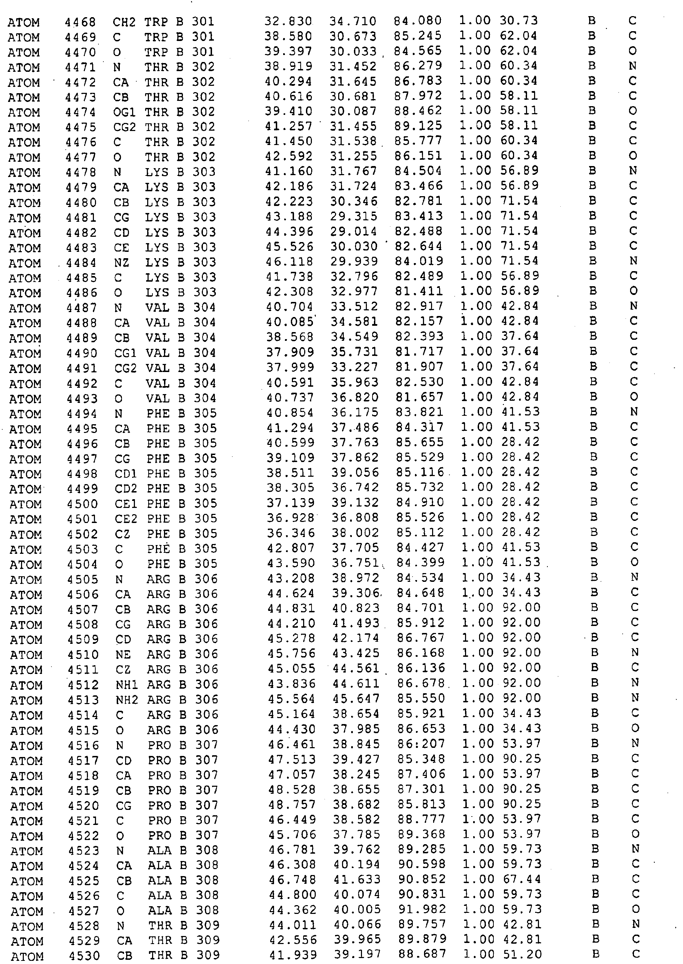

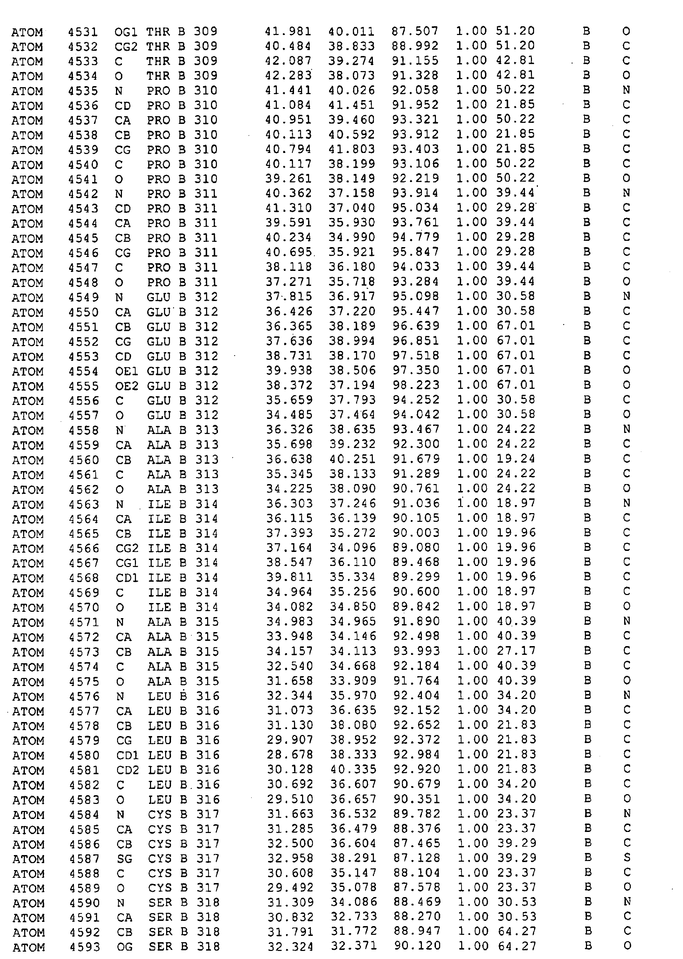

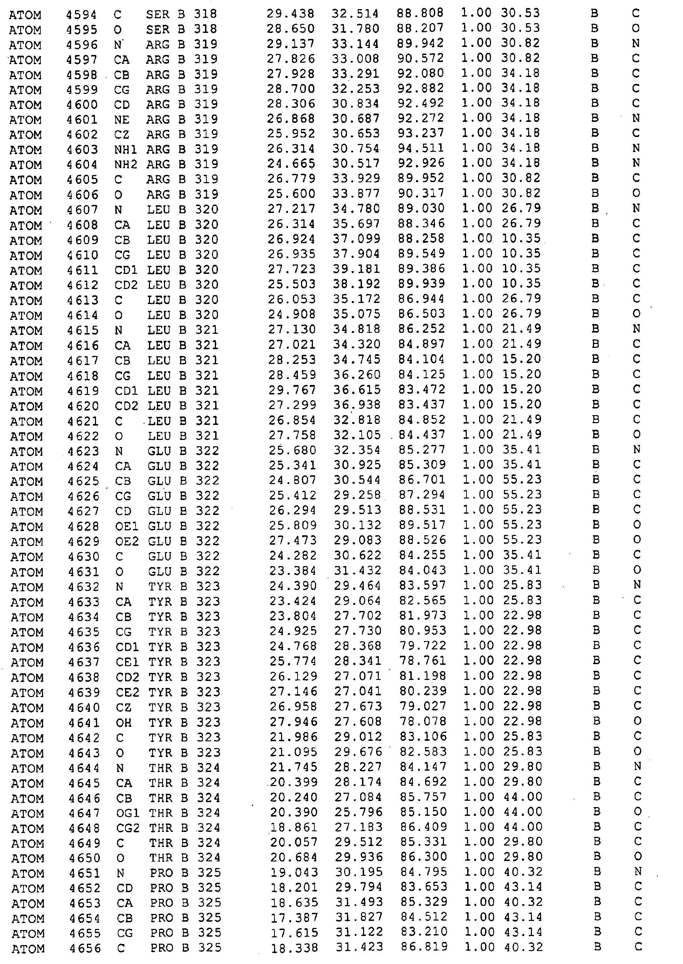

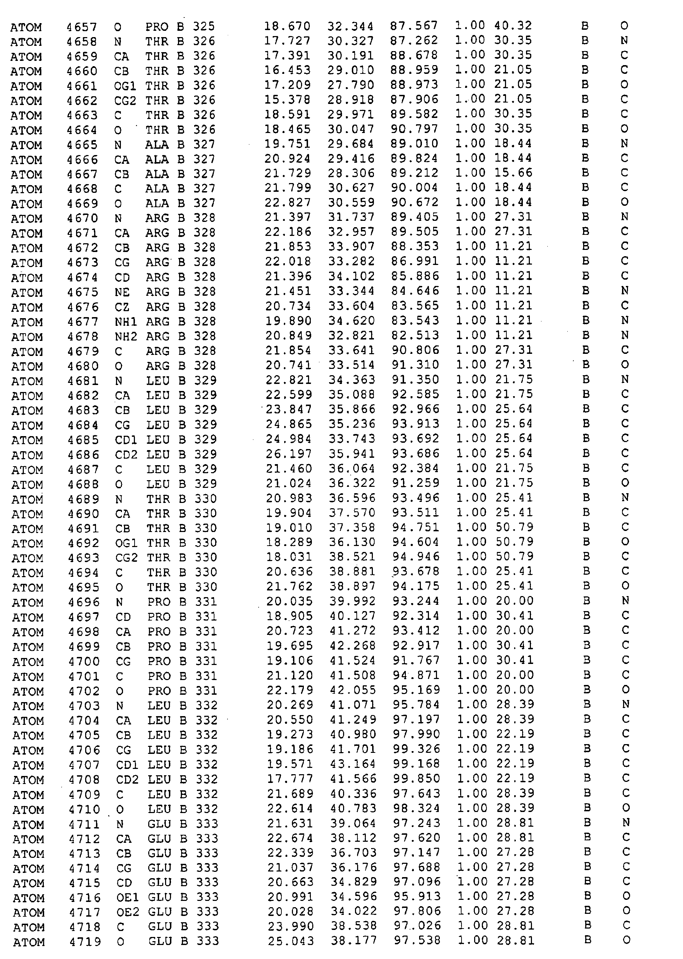

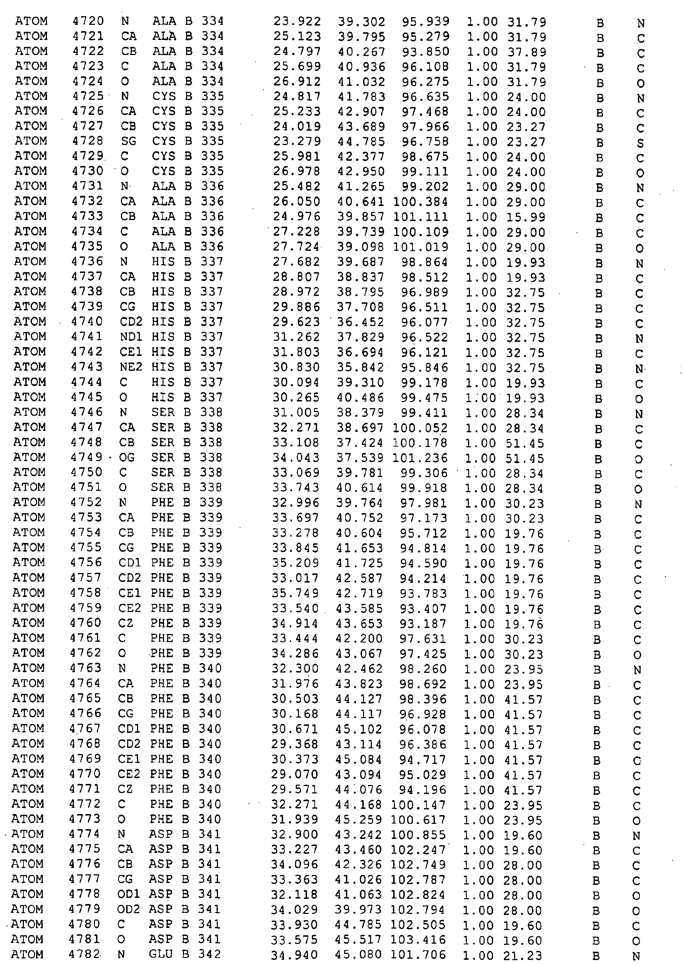

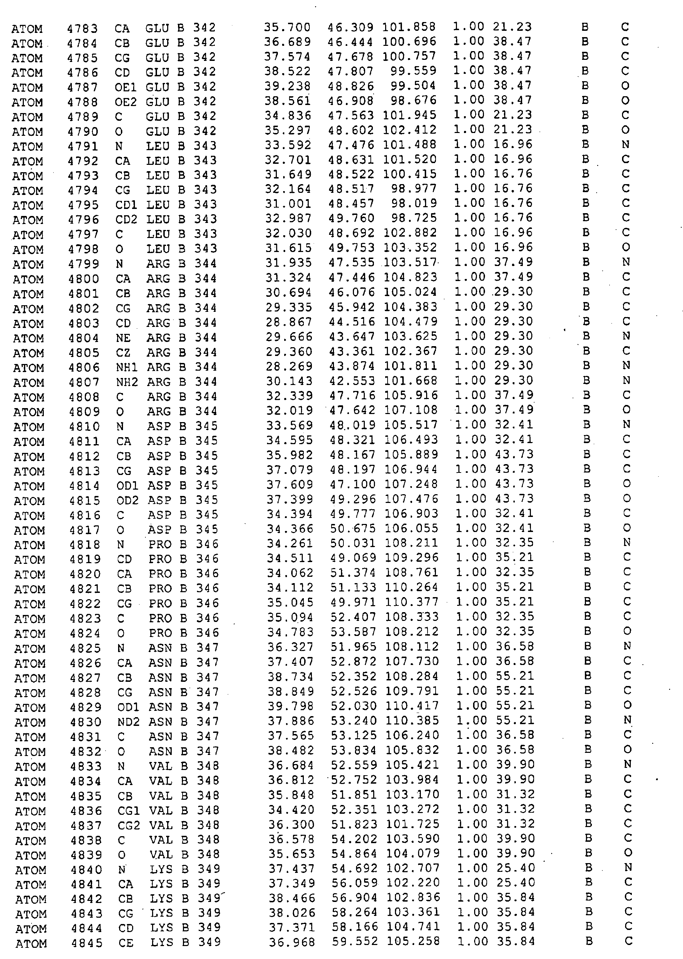

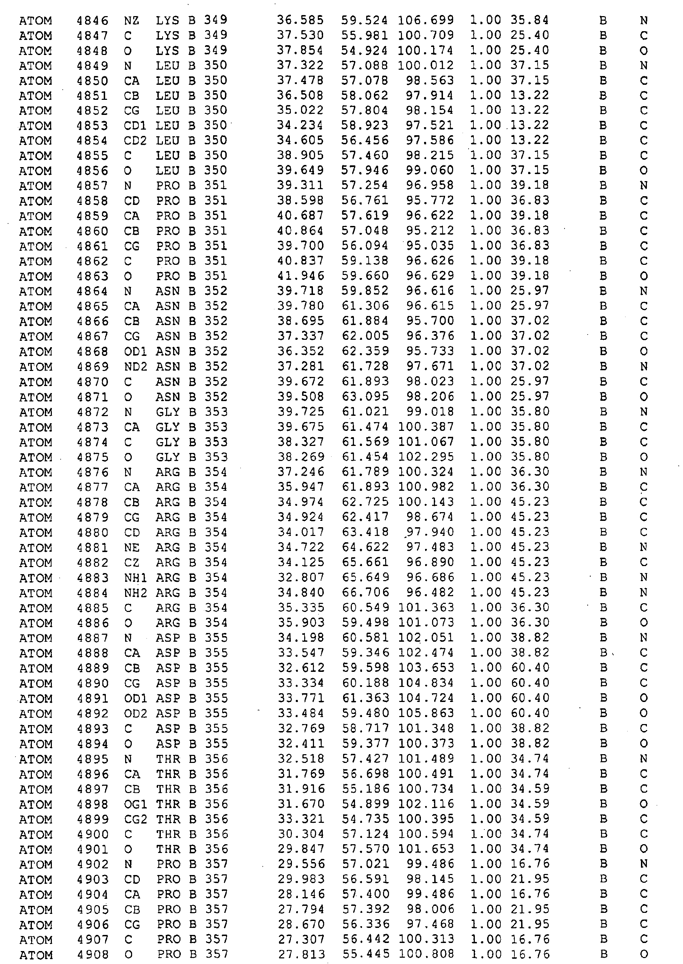

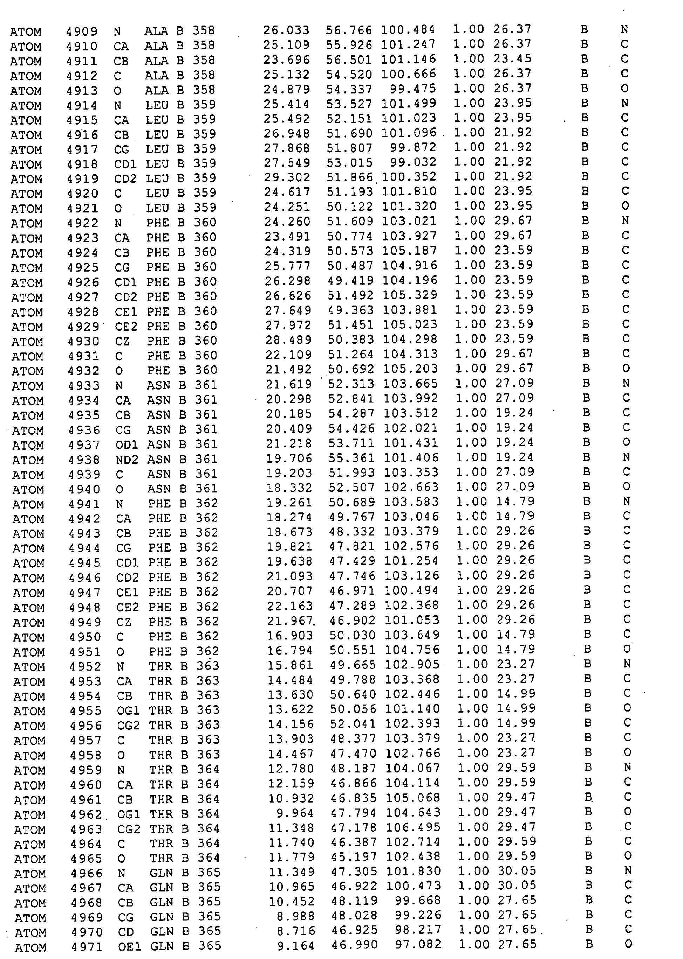

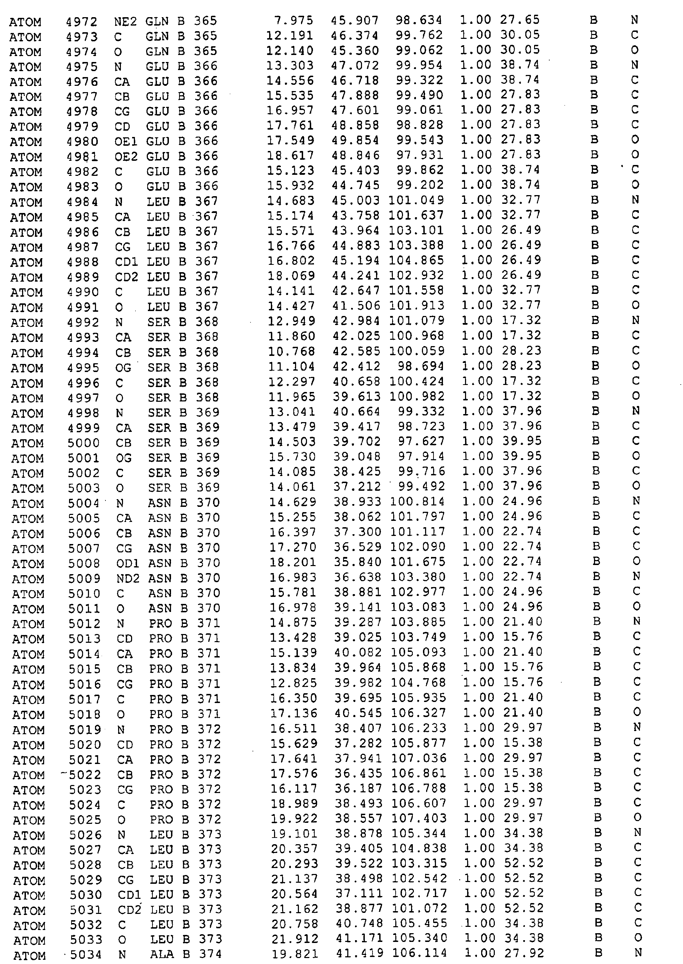

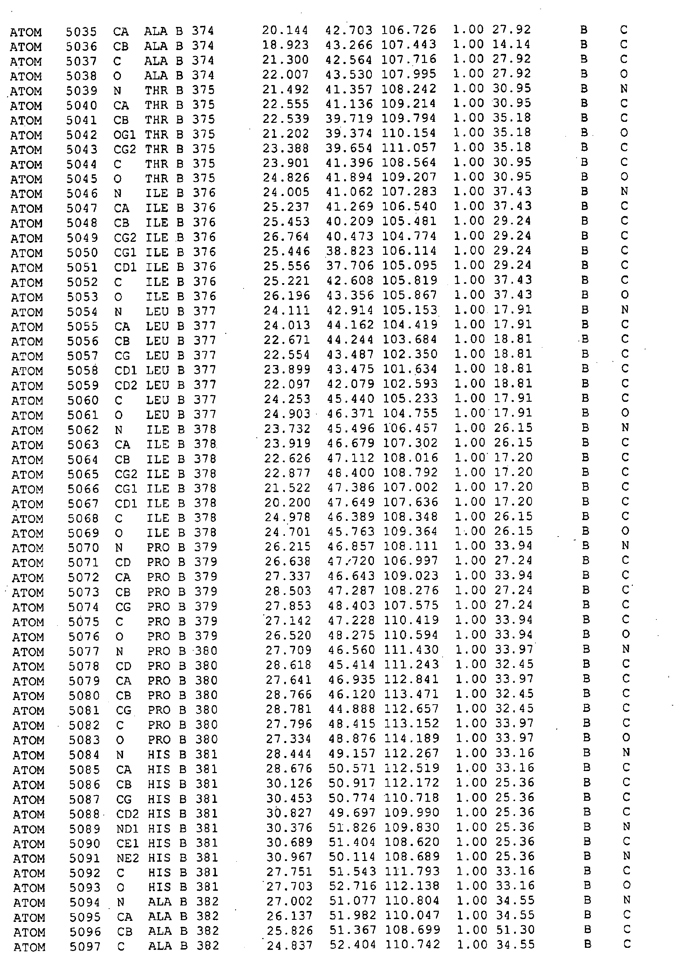

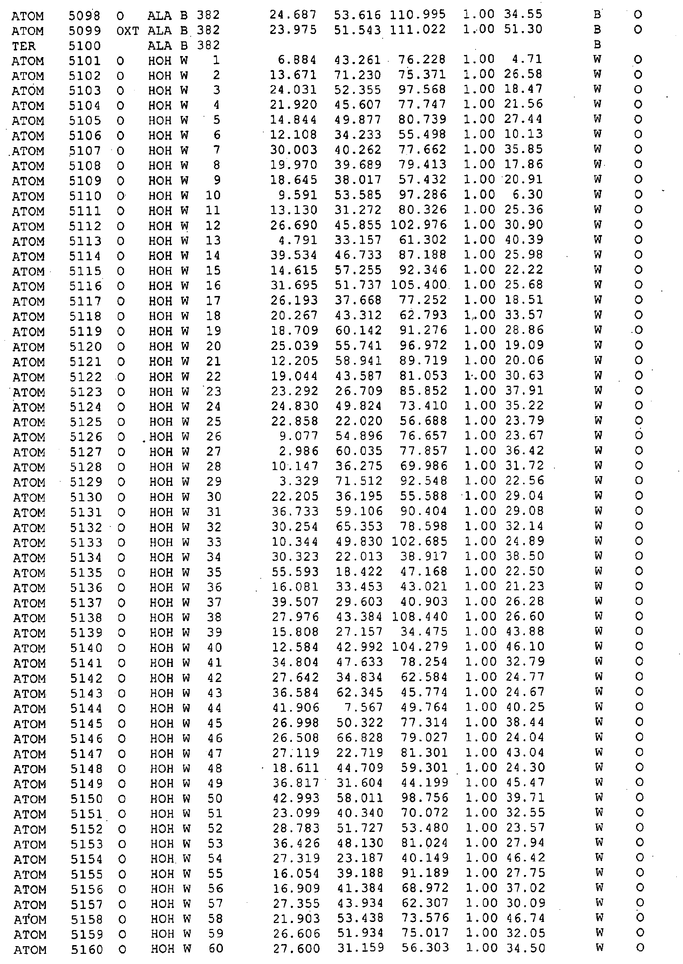

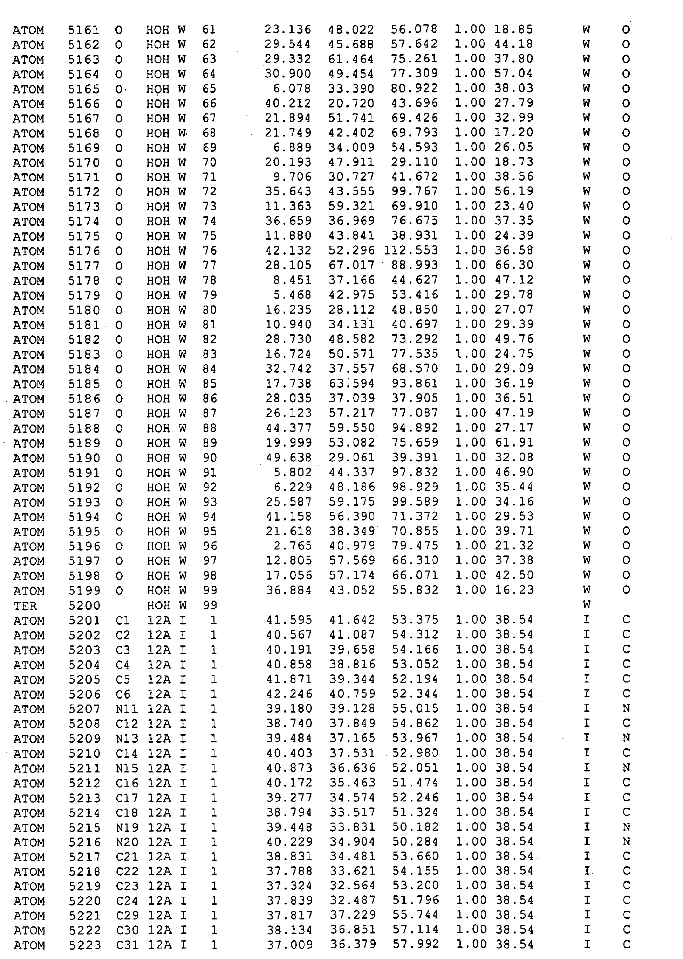

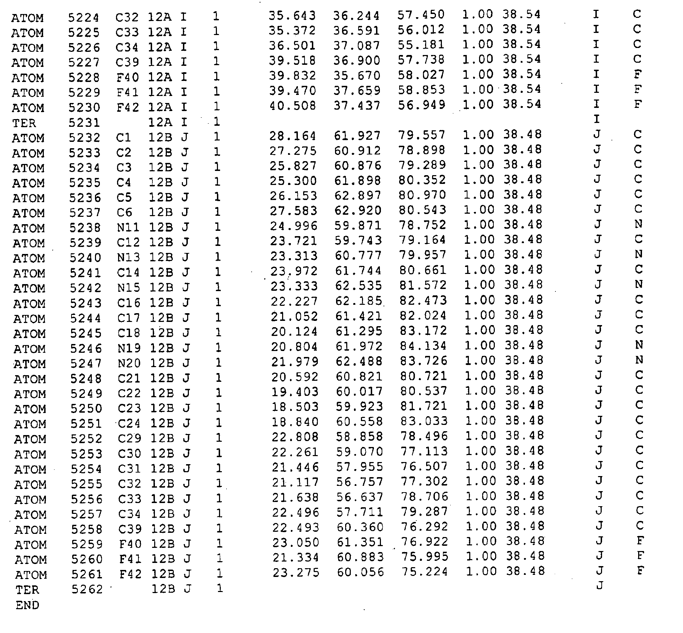

Surprisingly, it has now been found that for GSK-3β-inhibitor4 complex (Figure 6), the conformation of Gln185 is very different from the conformations reported for glutamines at this position in unphosphorylated, phosphorylated GSK-3β, GSK-3β-ADP-peptide complex and other protein kinases. A glutamine side chain is able to adopt different conformations, depending on its chemical environment. In the case of Gln185, the molecule that occupies the active site influences the conformation of the side chain of glutamine. When the molecule that occupies the GSK-3β active site contains an ortho-substituted phenyl ring and that ring is within 3.9 Å of Ile 62, Phe 67, Val 70, Asn 186 and Asp 200, the glutamine side chain adopts a conformation with a χ1 angle of -176.4° and a χ2 angle of 174°. Taking into consideration the steric hindrance of nearby residues, χ1 of Gln185 can range from 123° to 180°, χ2 can range from -174° to -180° and 106° to 180°. The χ1 can also range from -100° to -180°, and χ2 can range from -151° to -180° and 126° to 180°.

In order to compare the conformations of GSK-3β and other protein kinases at a particular amino acid site, such as Gln185, along the polypeptide backbone, well-known procedures may be used for doing sequence alignments of the amino acids. Such sequence alignments allow for the equivalent sites to be compared. One such method for doing a sequence alignment is the "bestfit" program available from Genetics Computer Group which uses the local homology algorithm described by Smith and Waterman in Advances in Applied Mathematics 2; 482 (1981).

Equivalents of the Gln185 residue of GSK-3β may also be identified by its functional position. Gln185 is located on the α-helical domain of the GSK-3β kinase domain in front of the active site. It is positioned five residues after the conserved RD motif (Arg 180, Asp 181) and just before the beginning of beta-strand β7 (Bax, B. et al. Structure, 9, pp 1143-1152, (2001)).

A comparison of the torsion angles between Gln185 in the GSK-3β or GSK-3β complexes and those of corresponding glutamines in other kinases are illustrated in Table 2. The torsion angles were determined by the program QUANTA.

TABLE 2

| Protein | χ1 (˚) | χ2 (˚) | χ3 (˚) |
|---|---|---|---|
| GSK-3β-peptide-ADP | -60.0 | 83.3 | -25.7 |
| Phosphorylated GSK-3β | -59.9 | 83.2 | -22.5 |
| Unphosphorylated GSK-3β | -60.9 | 63.0 | 88.7 |
| GSK-3β-inhibitor1 | -67.1 | 113.9 | -72.2 |
| GSK-3β-inhibitor2 | -60.7 | 90.1 | 98 |
| GSK-3β-inhibitor3 | -63.3 | -158.5 | 179.8 |
| GSK-3β-inhibitor4 | -176.4 | -174.0 | -3.3 |
| CDK2 _inhibitor [1] | 87.4 | -172.5 | 73.4 |
| CDK2_cyclin A [2] | -98.1 | -59.2 | 71.2 |
| 1: Cyclin-Dependant Kinase 2 in complex with oxindole inhibitor. Davis et al., Science, 291, 134 (2001); Protein Data Bank Accession number 1FVV.<br>2: Phosphorylated Cyclin-Dependent Kinase-2 bound to Cyclin A. Russo et al., Nat. Struct. Biol., 3, 696 (1996); Protein Data Bank Accession number 1JST. | | | |

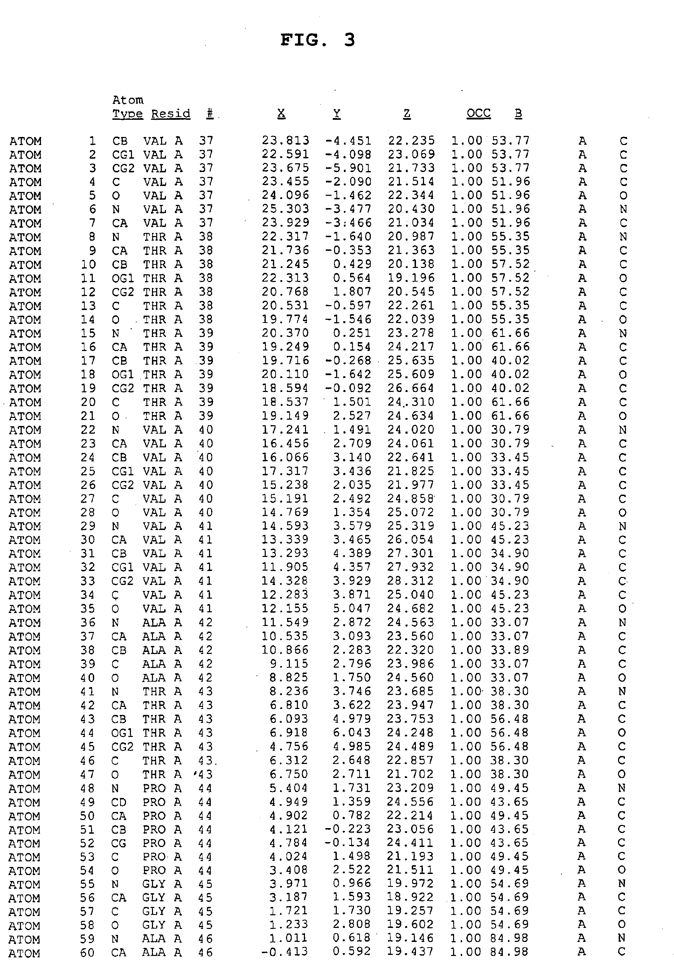

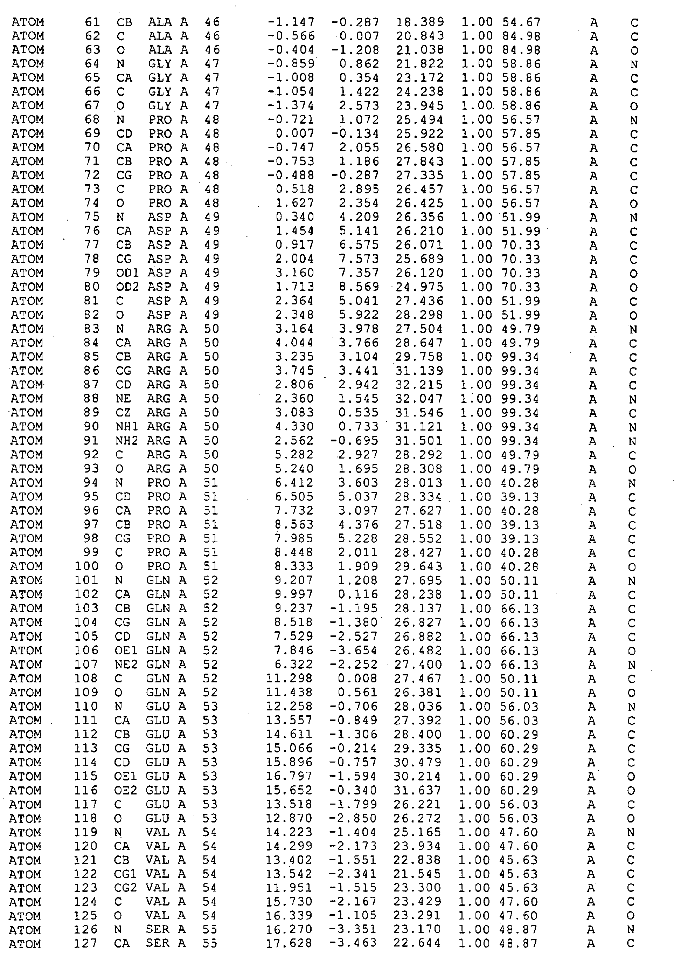

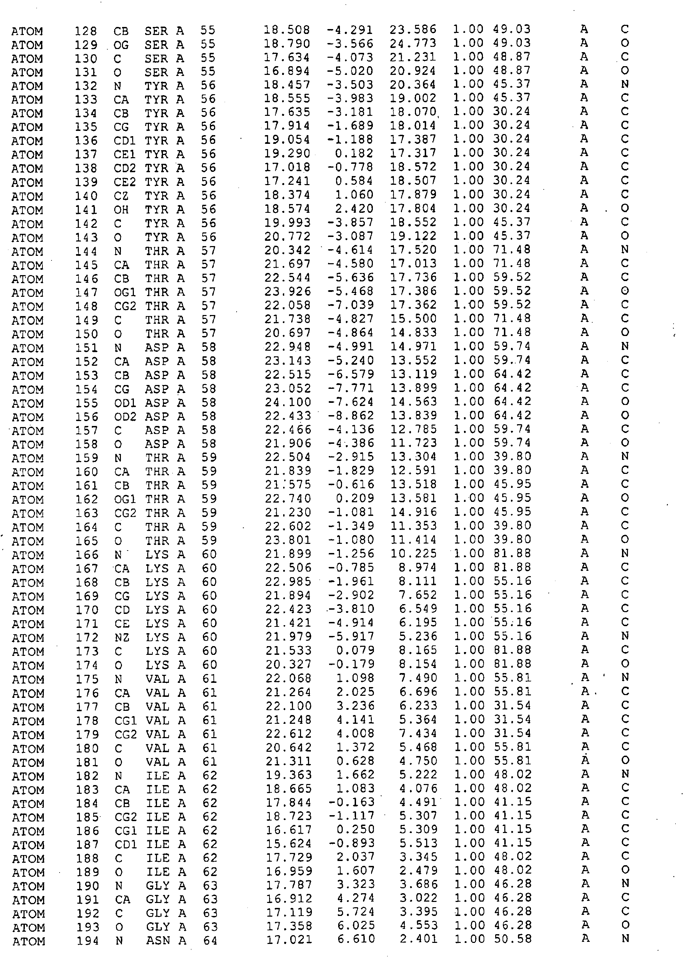

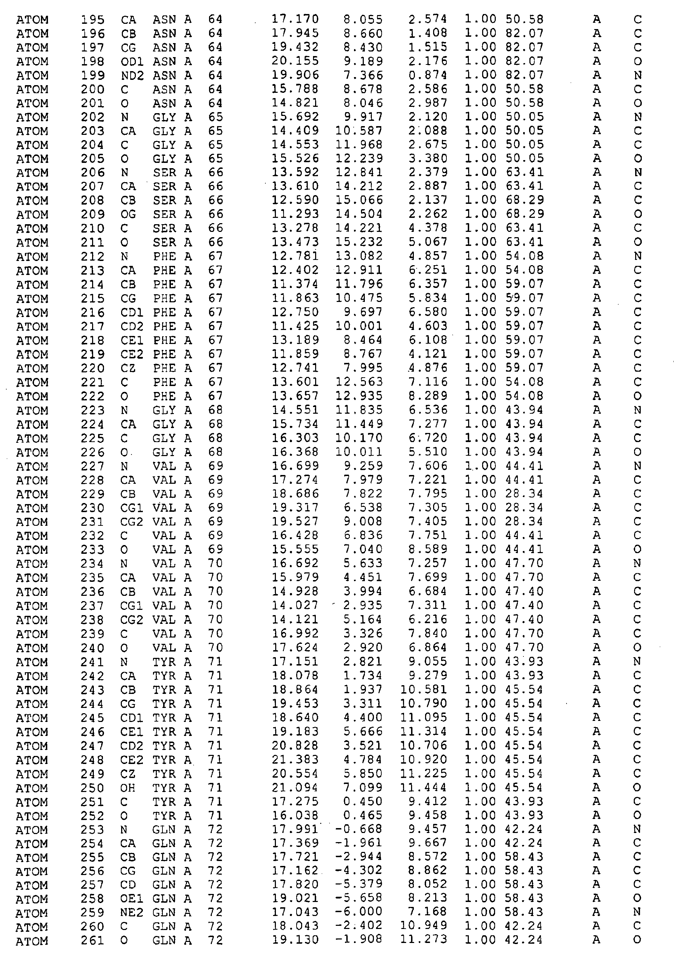

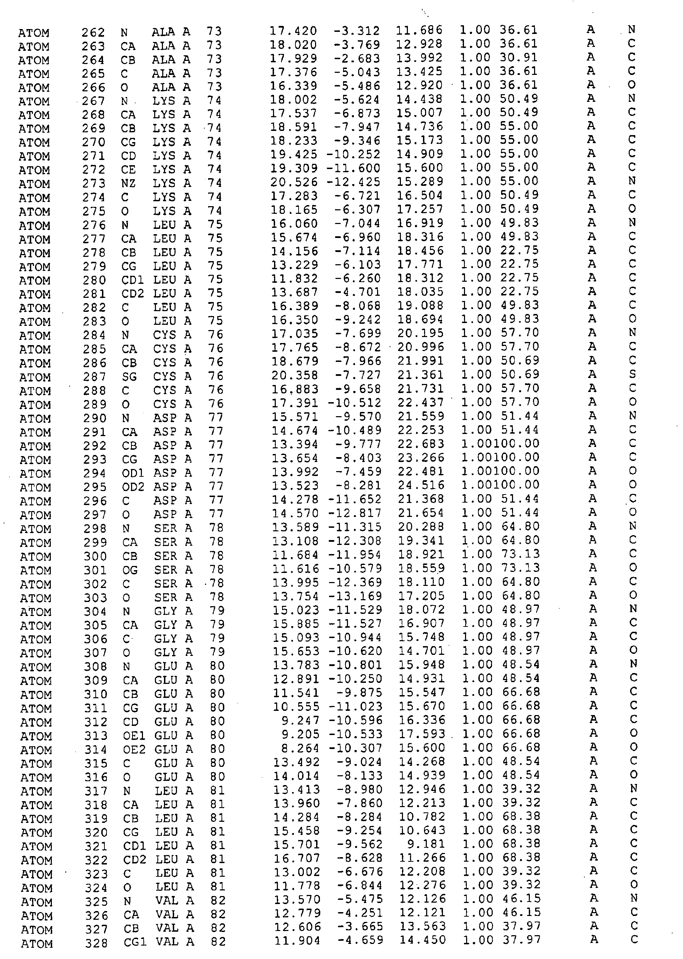

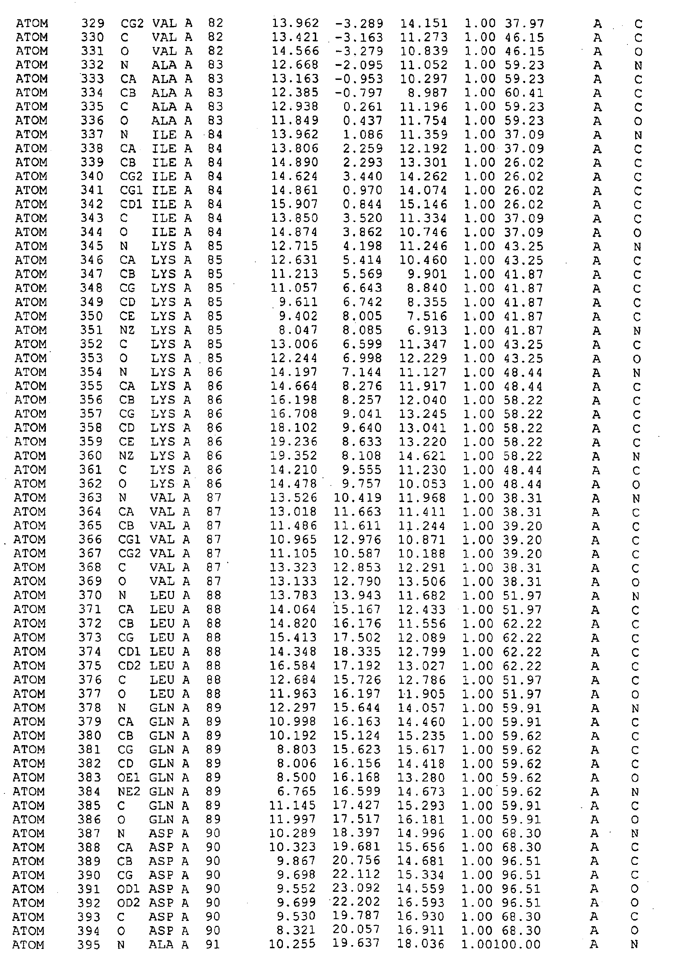

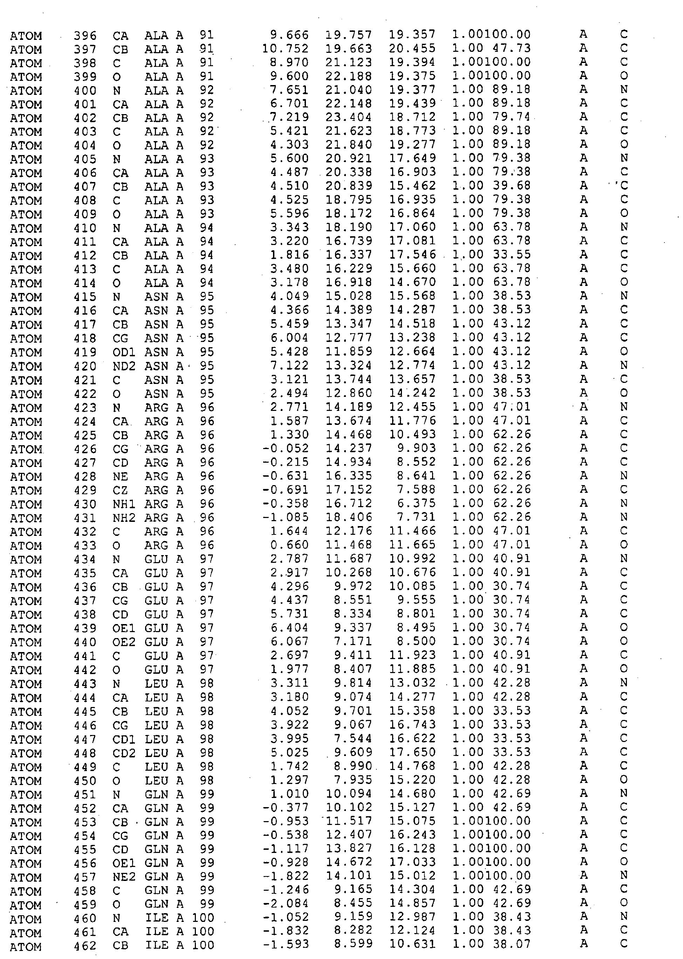

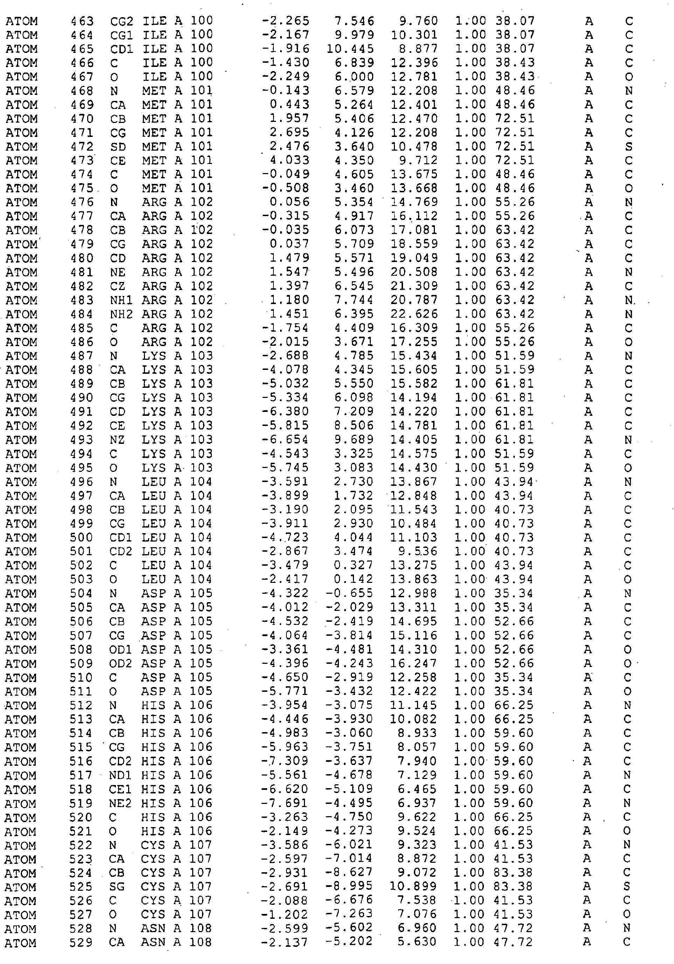

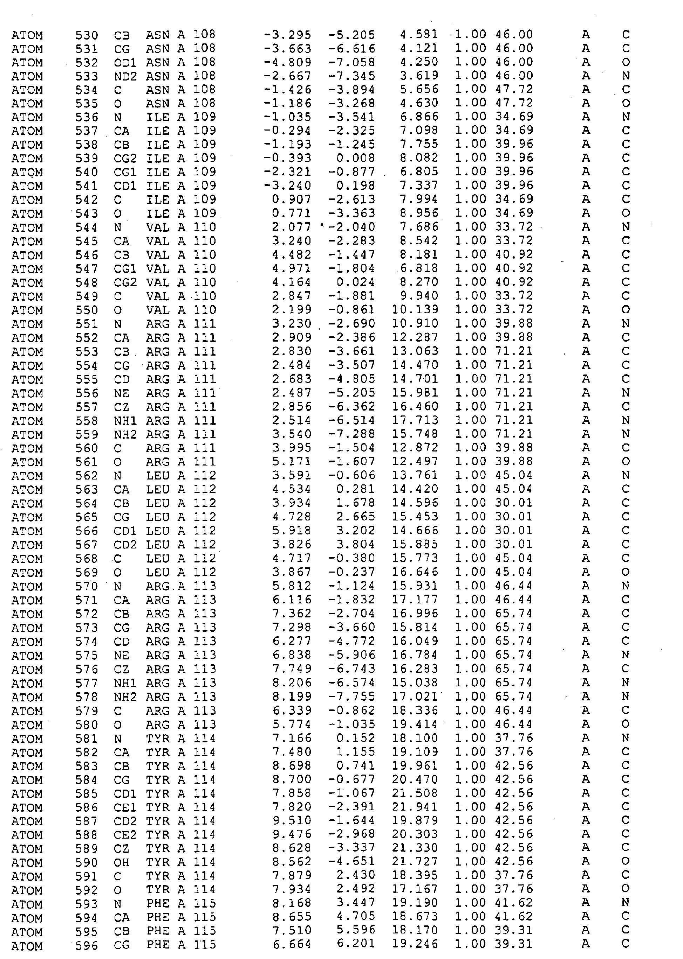

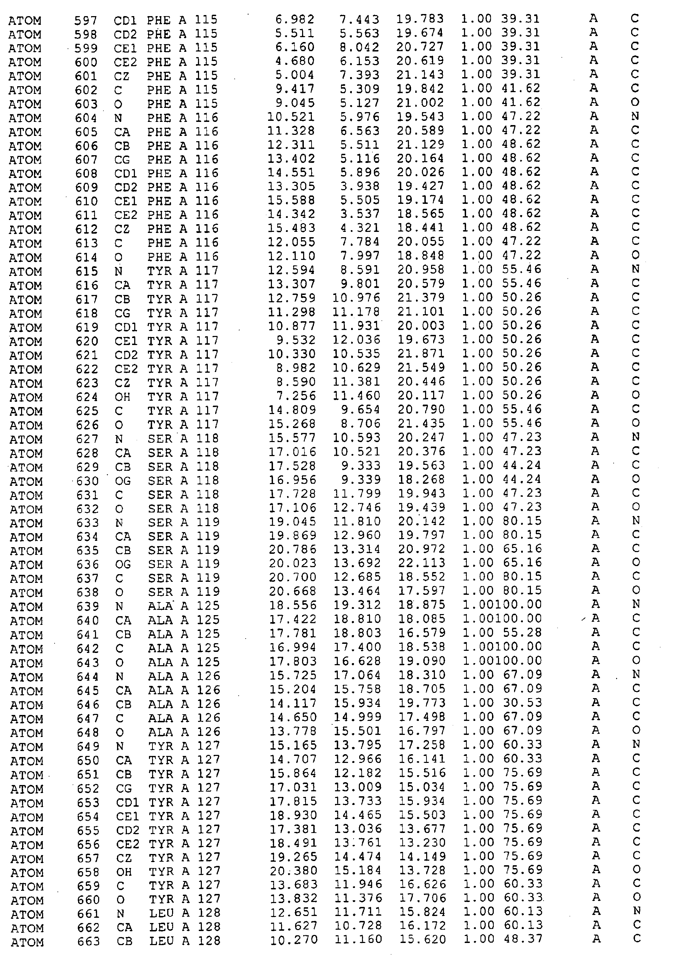

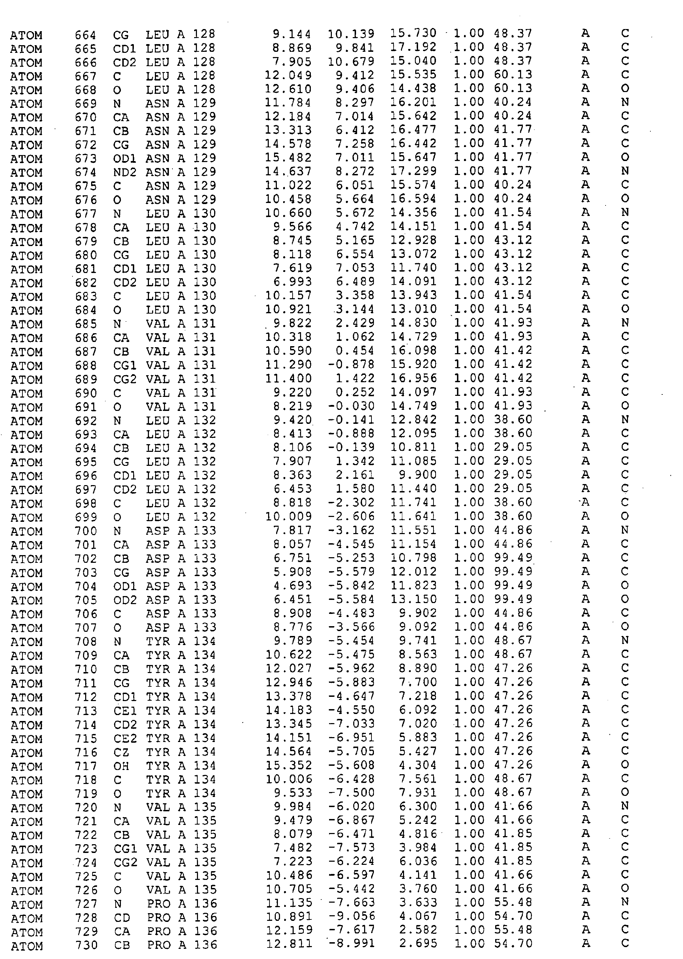

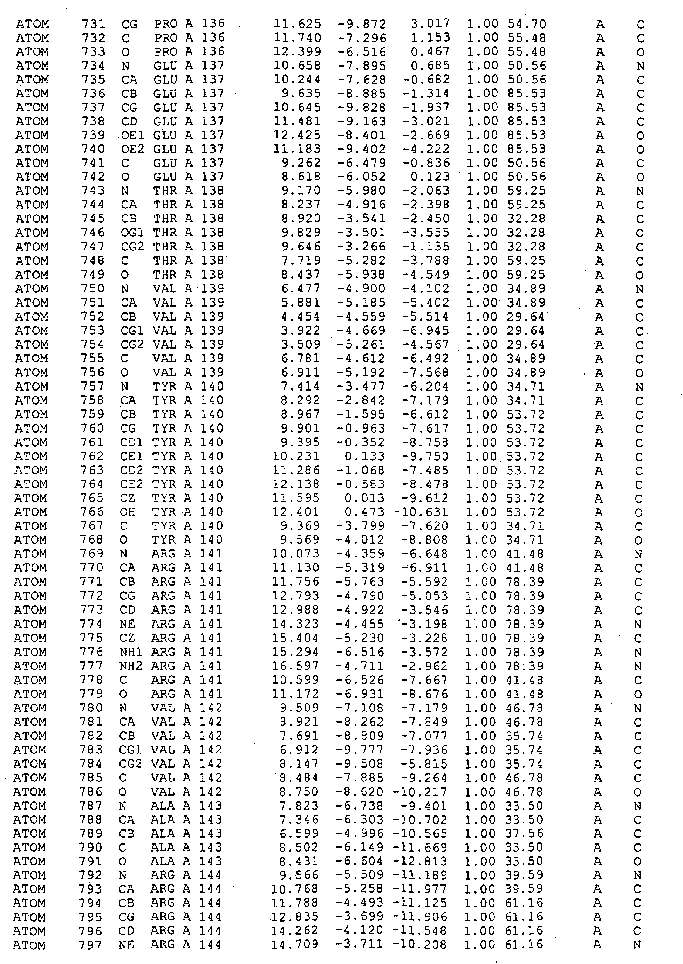

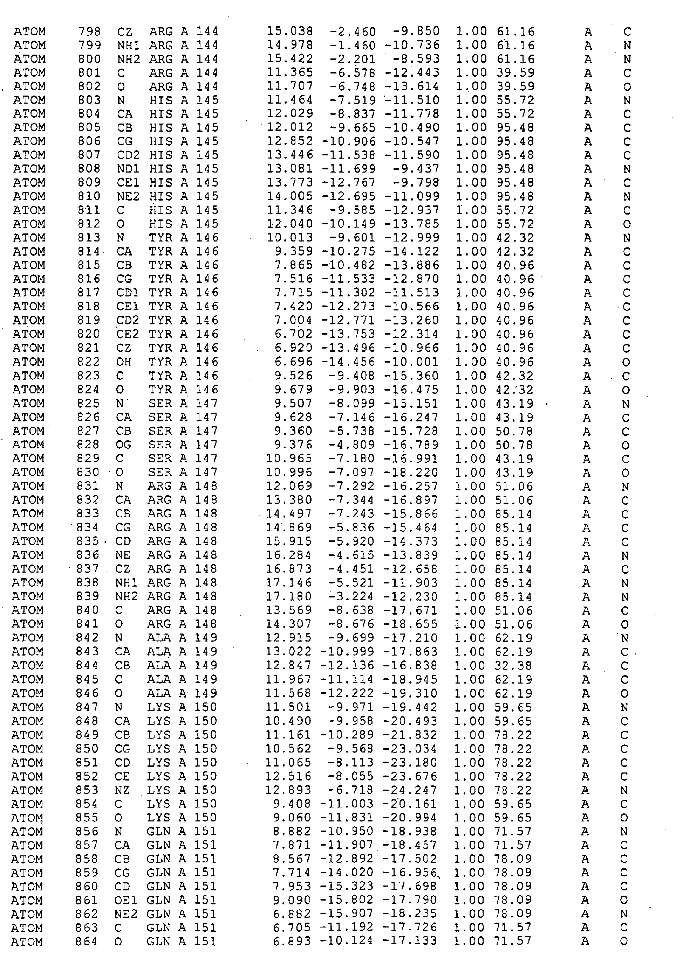

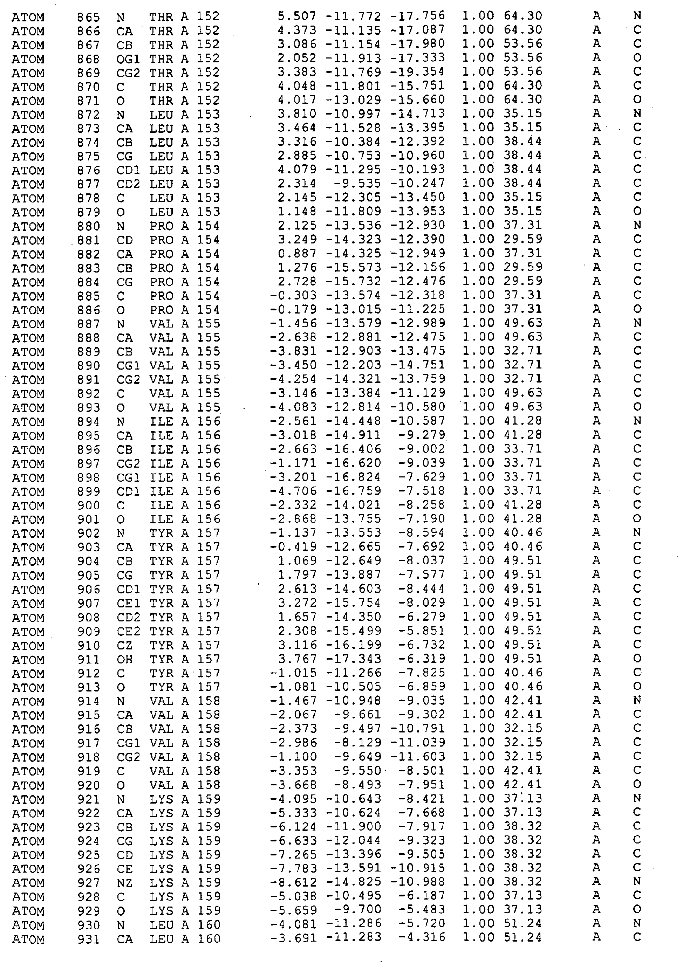

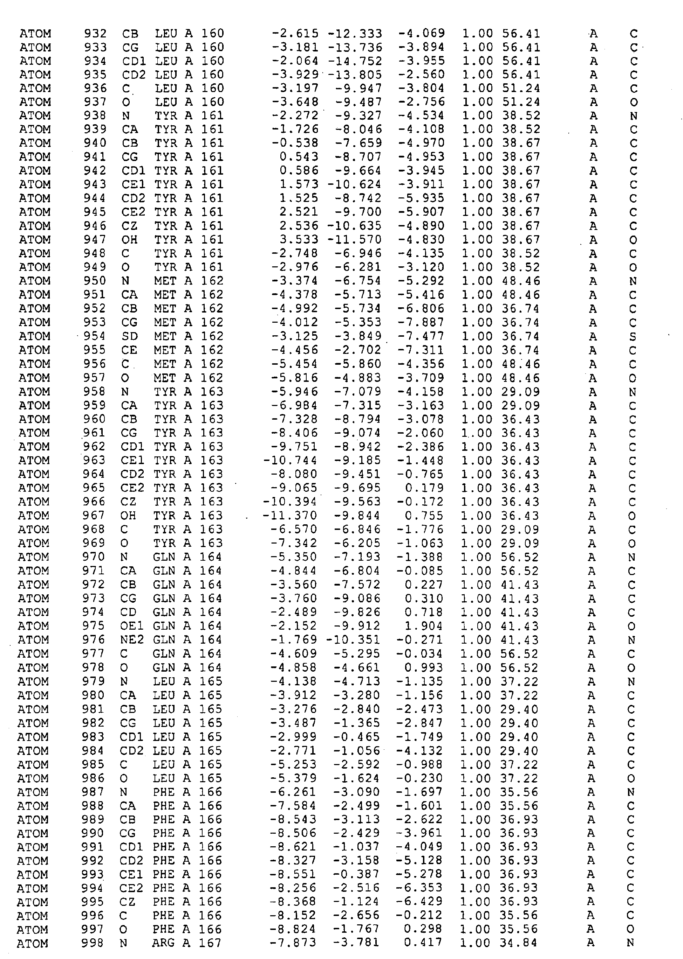

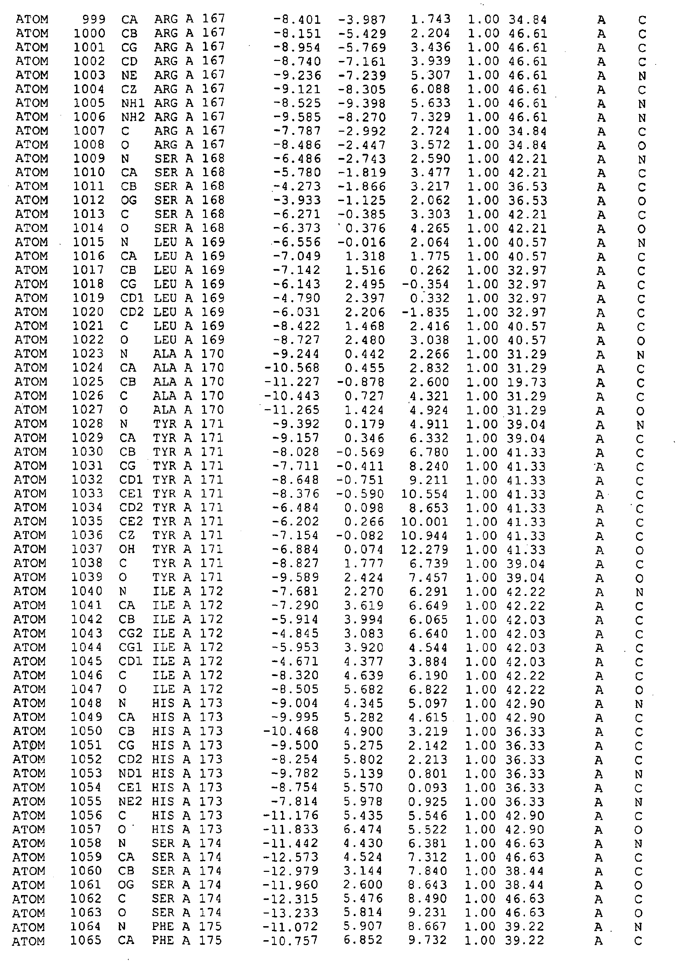

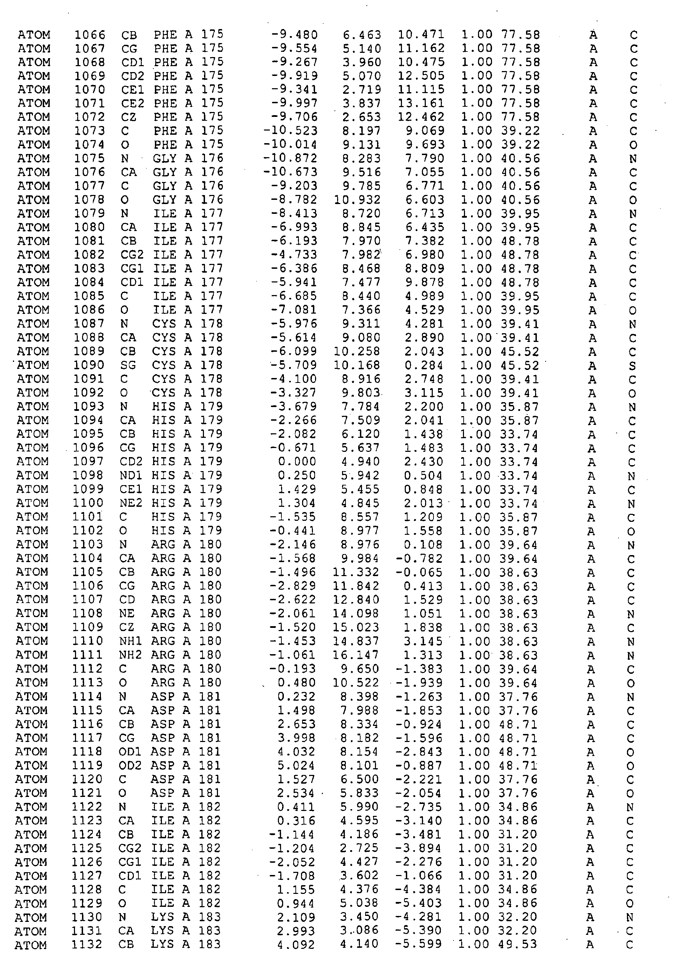

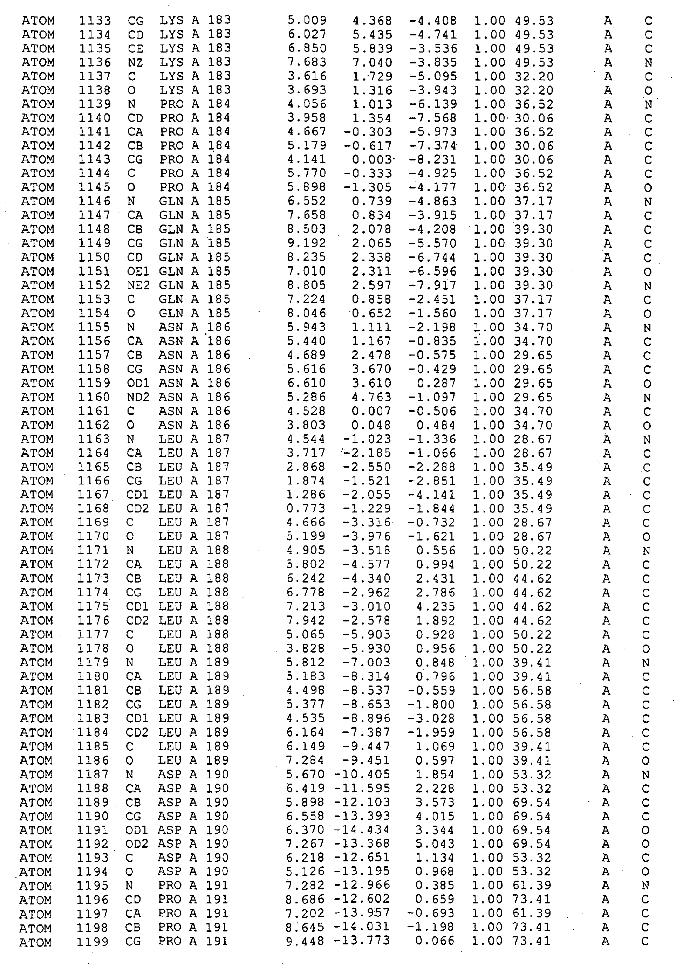

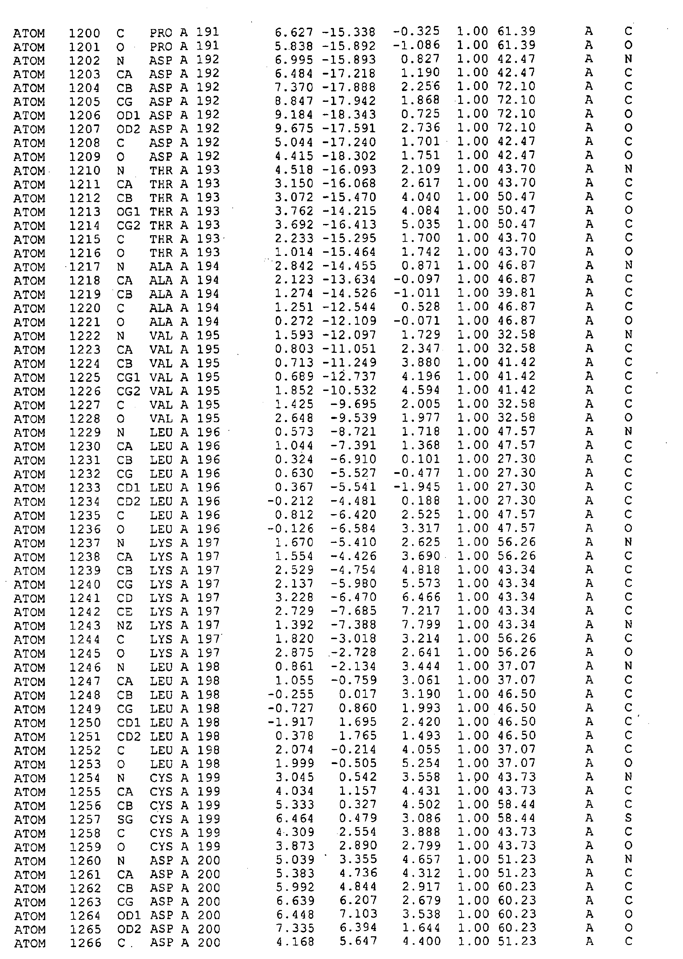

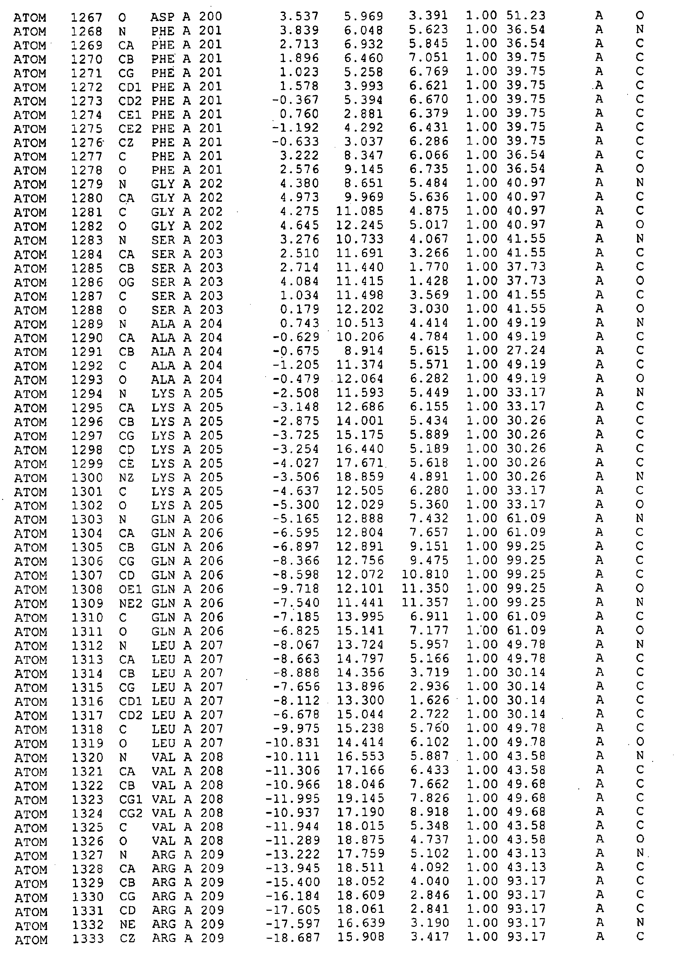

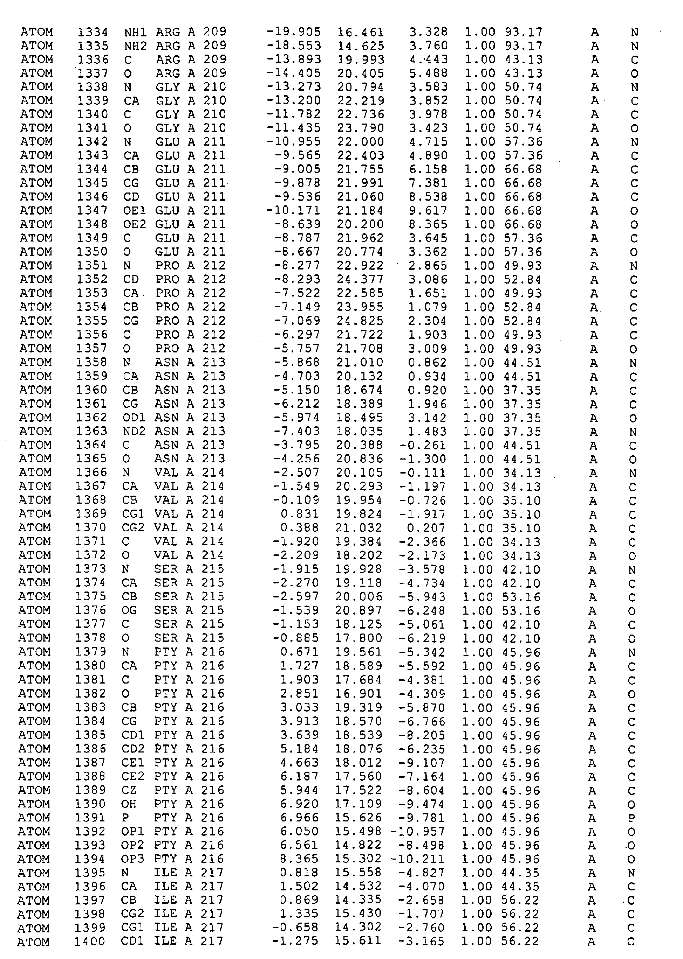

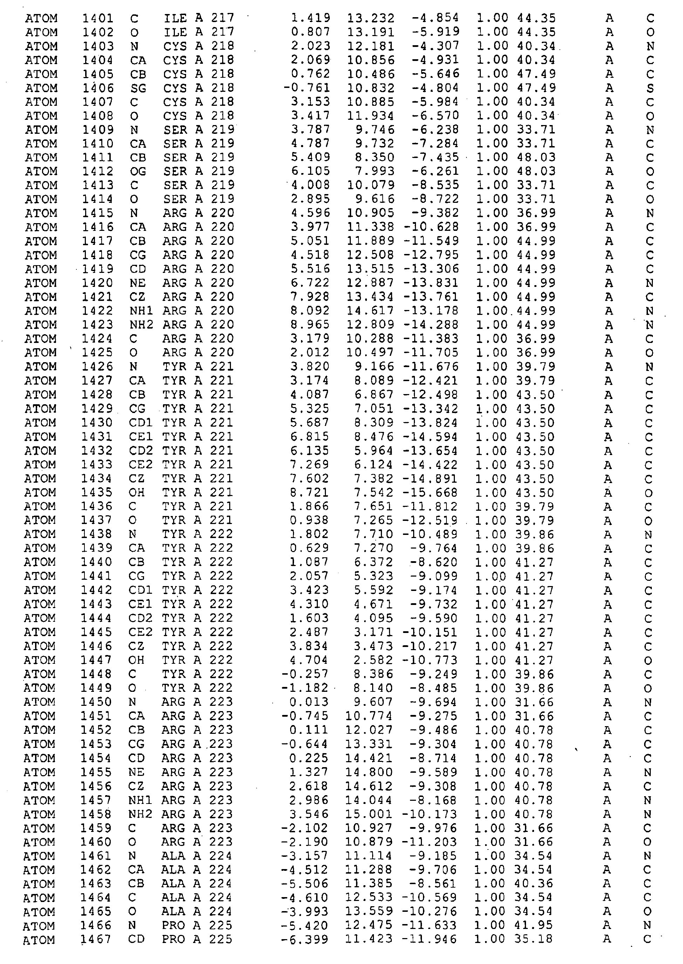

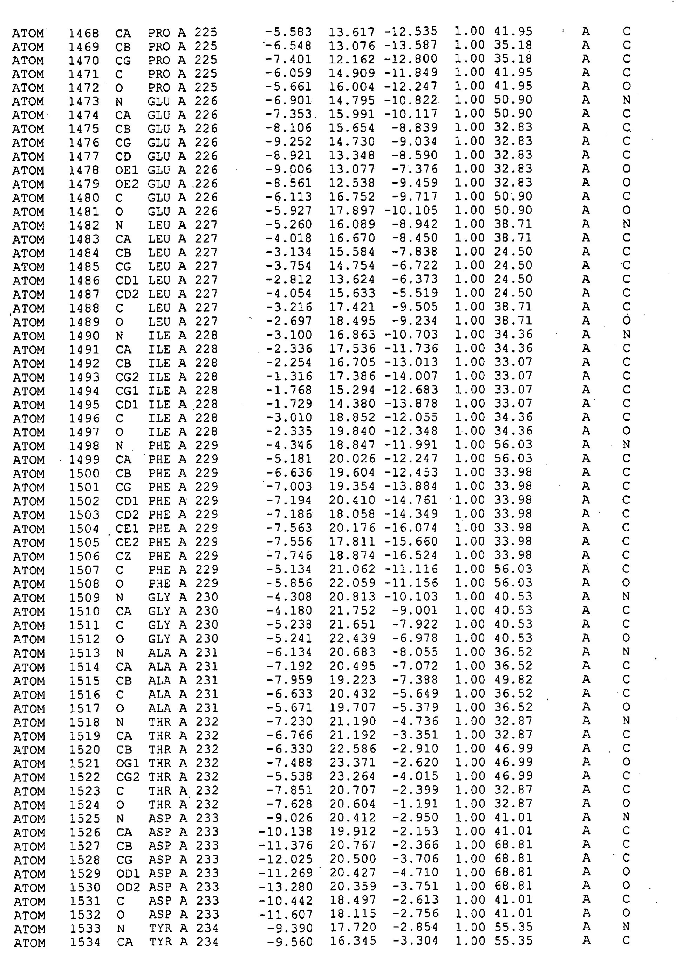

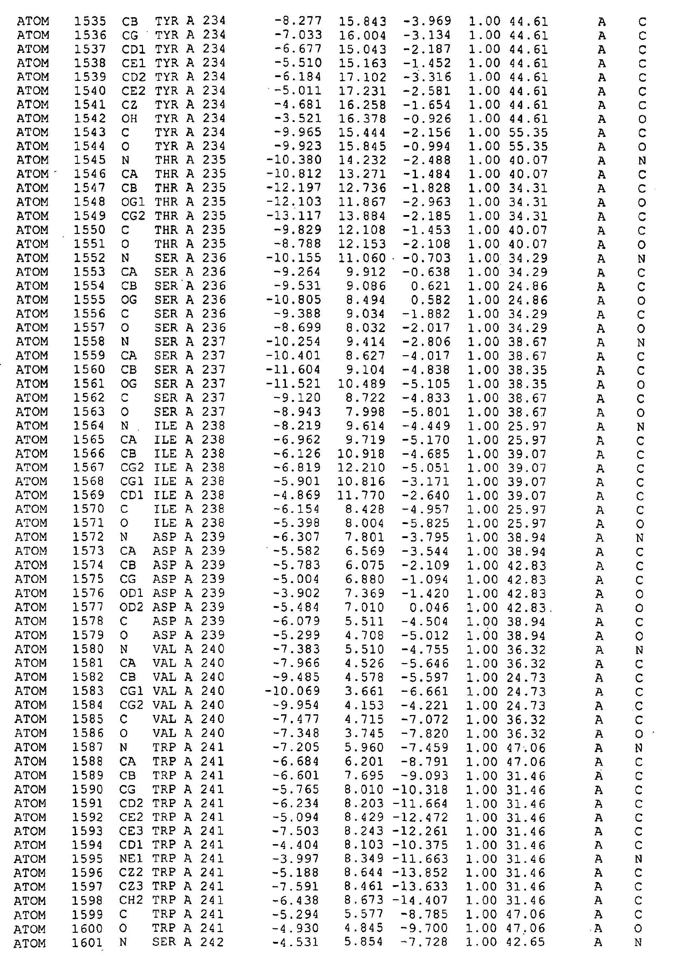

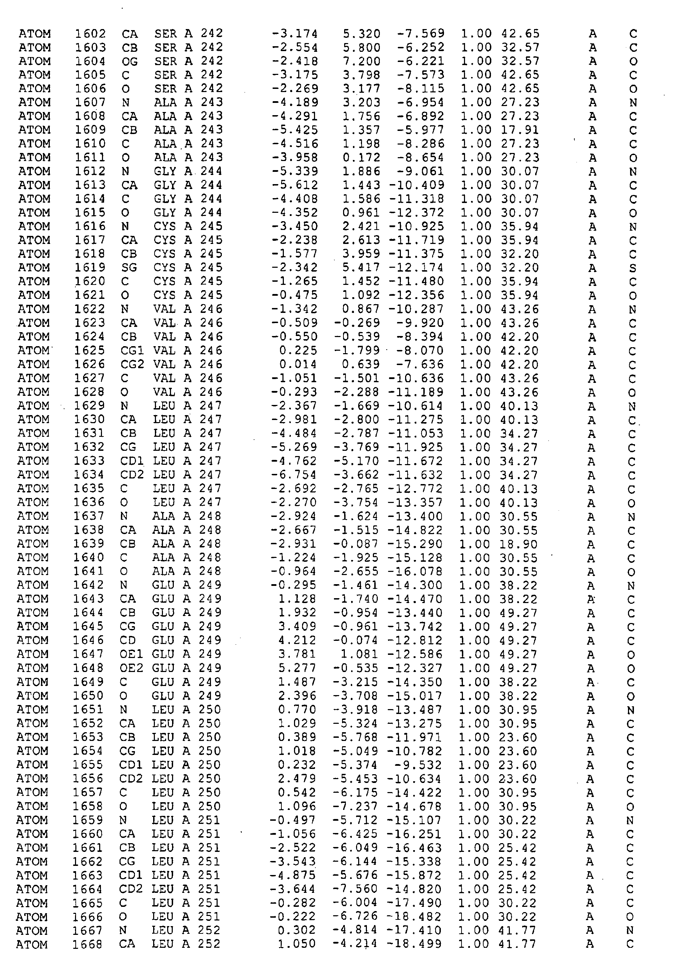

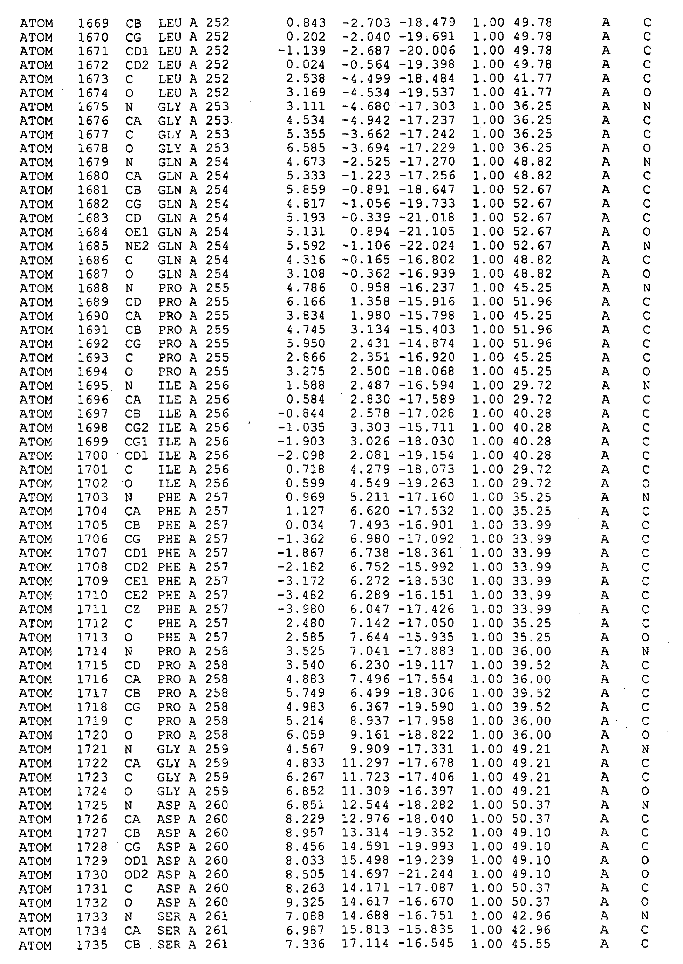

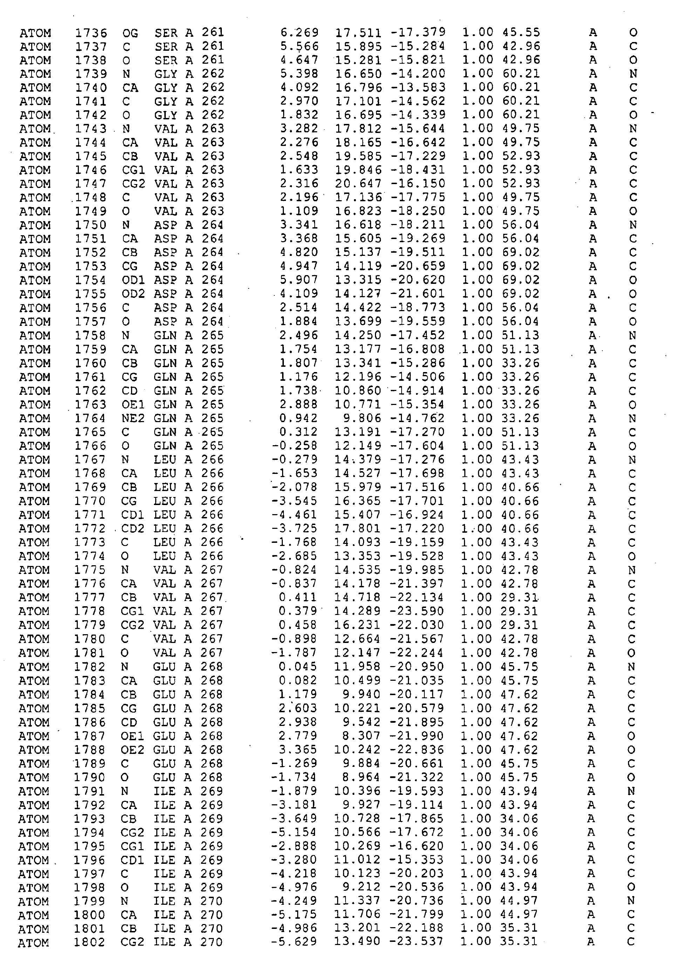

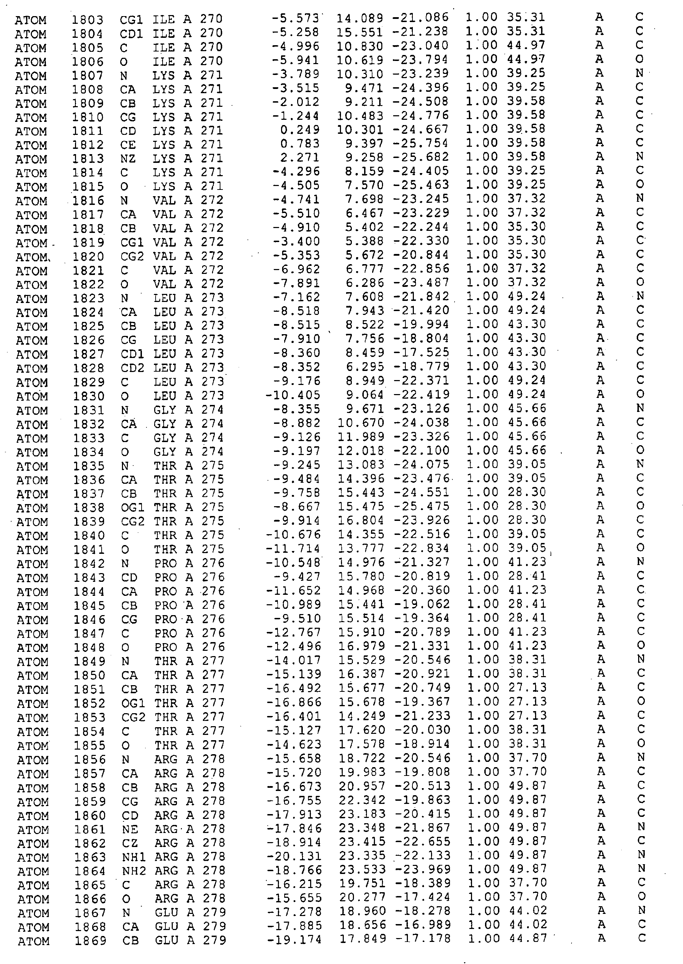

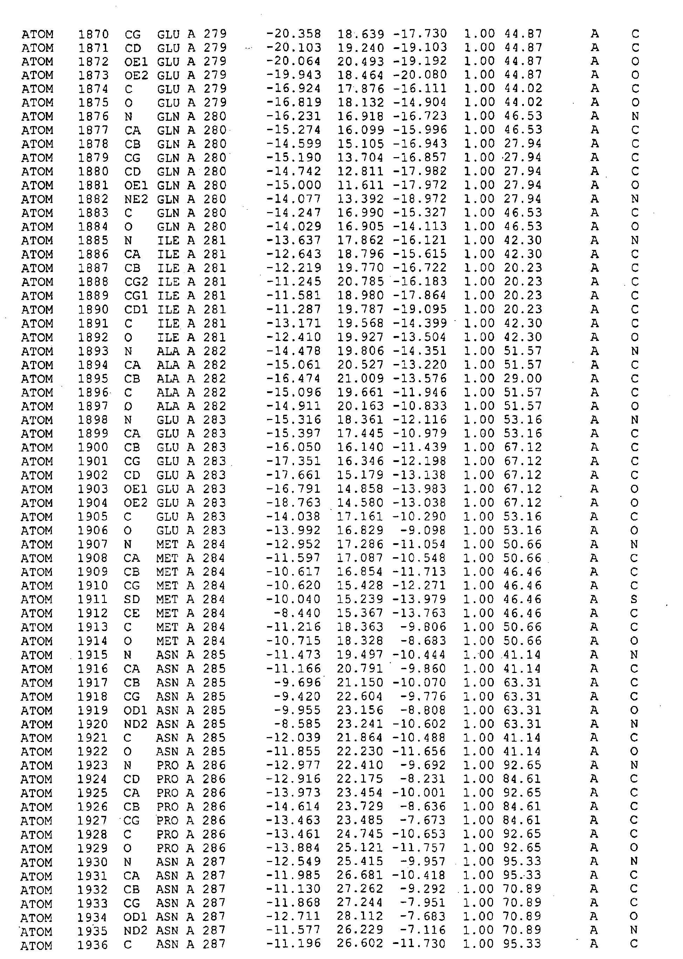

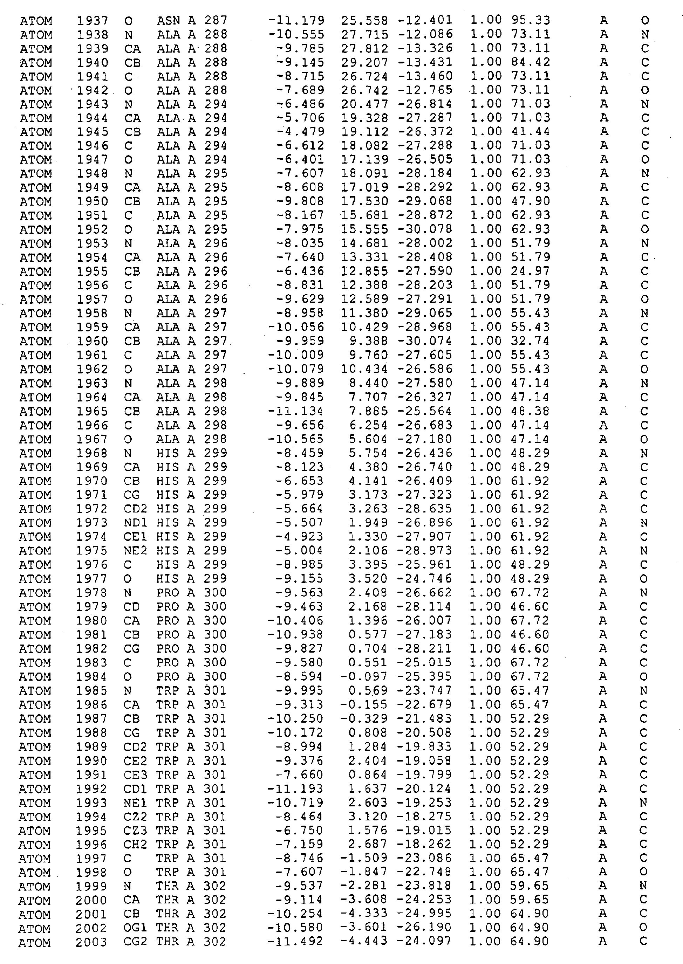

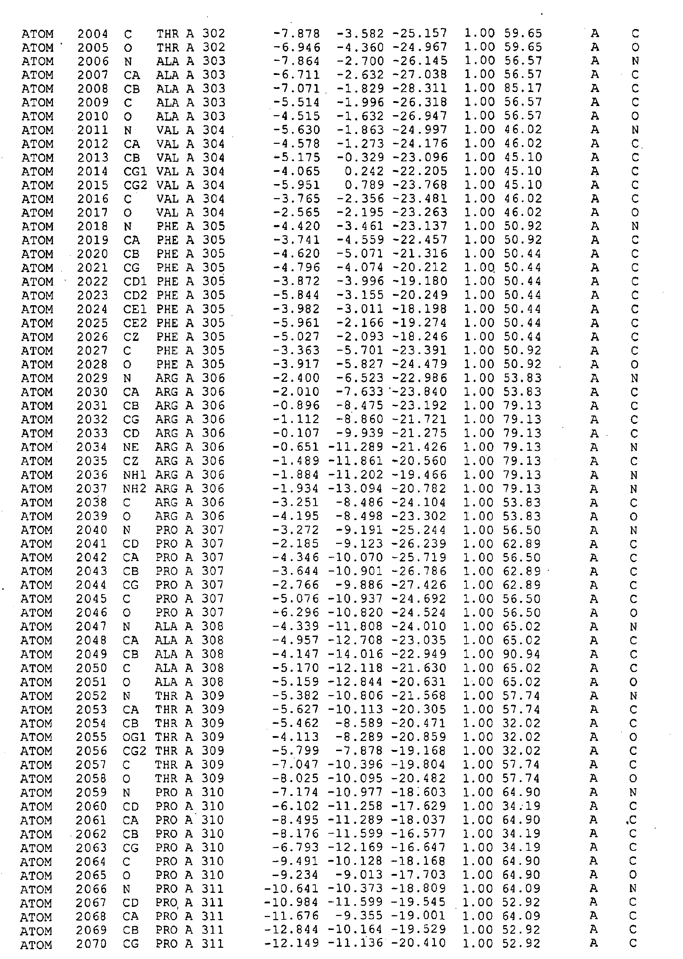

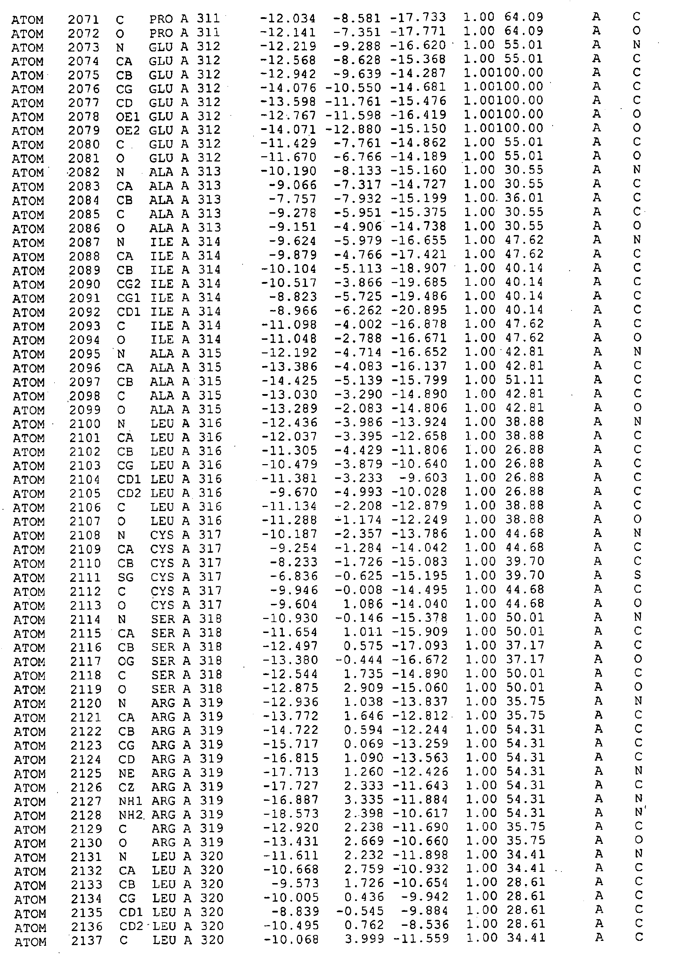

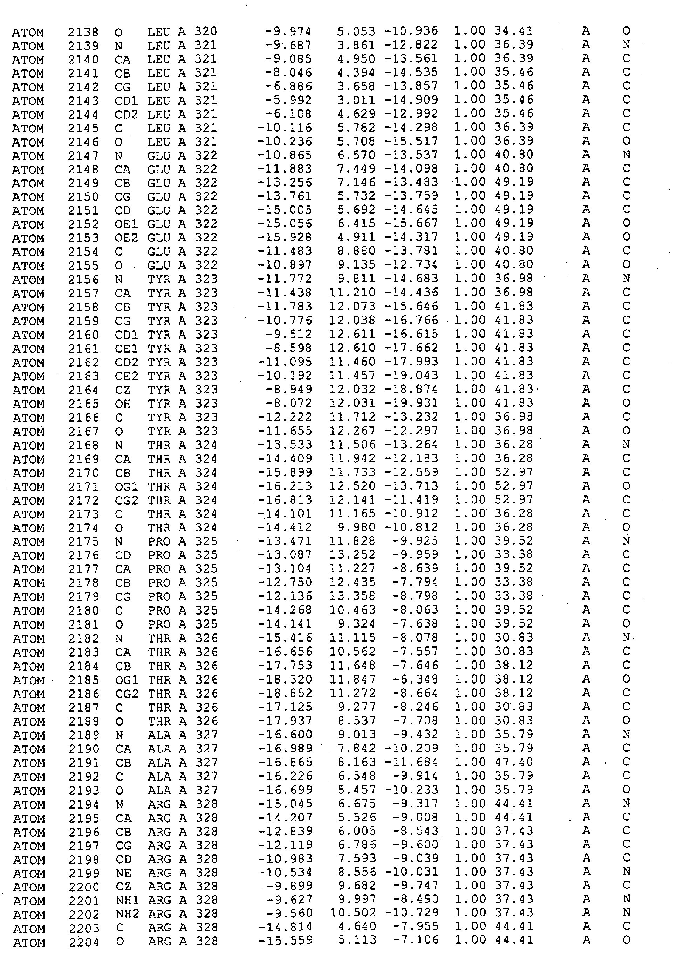

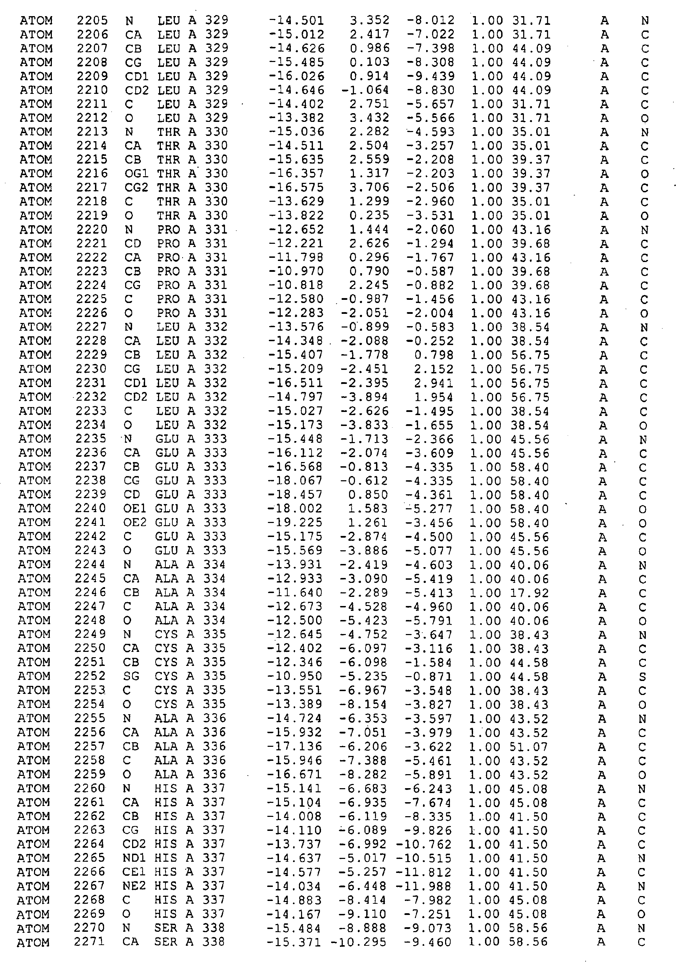

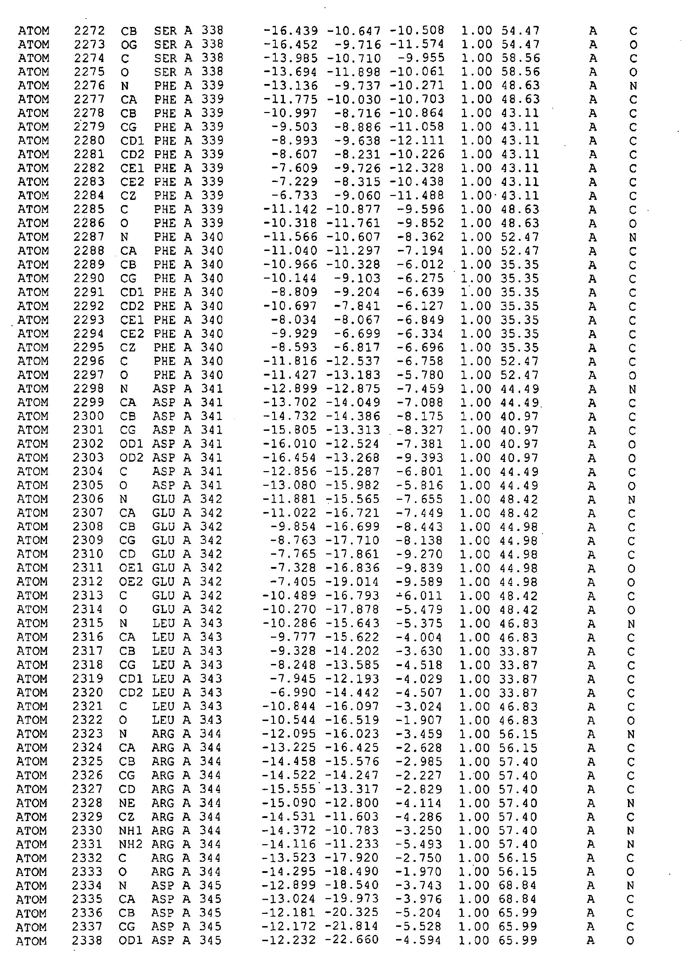

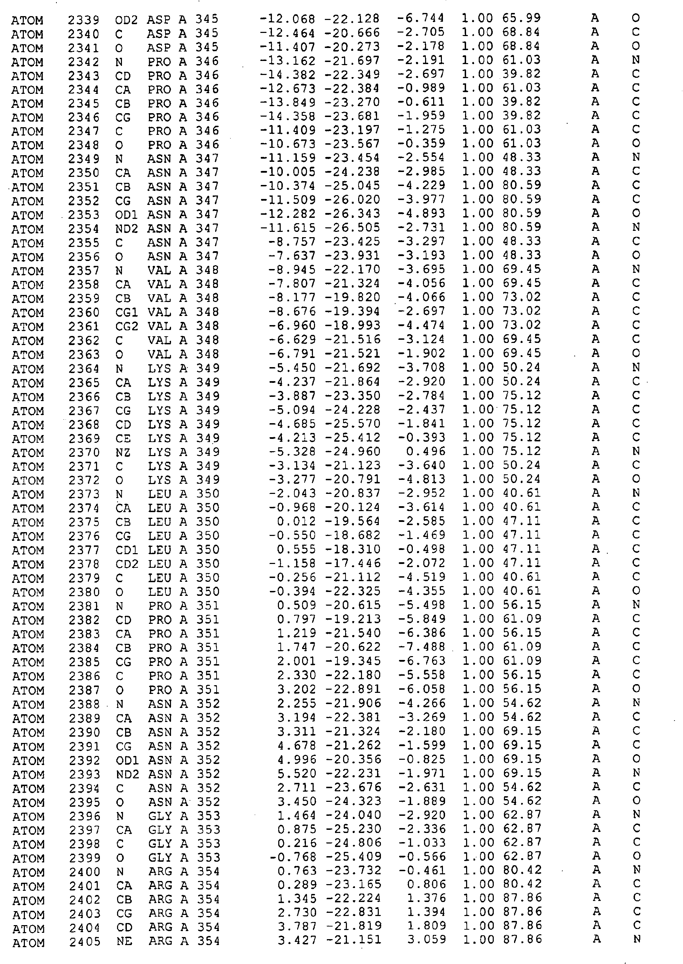

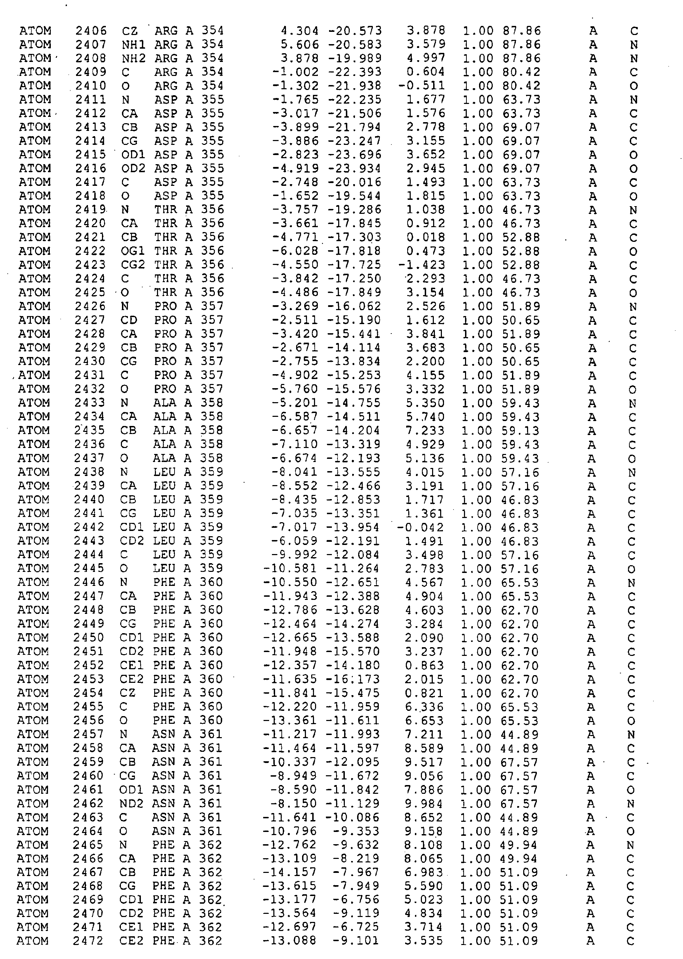

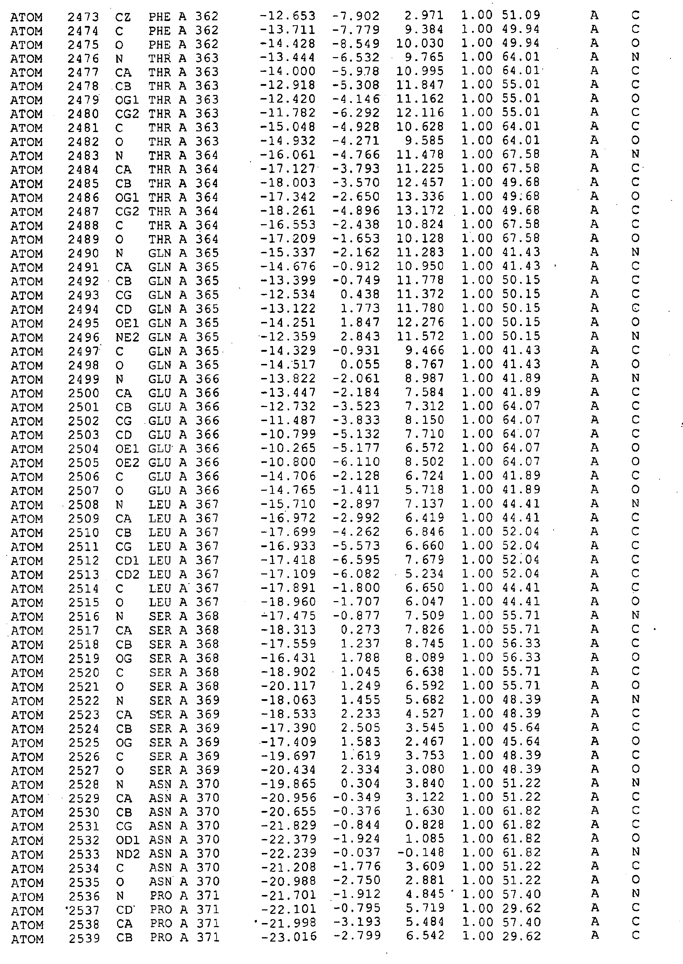

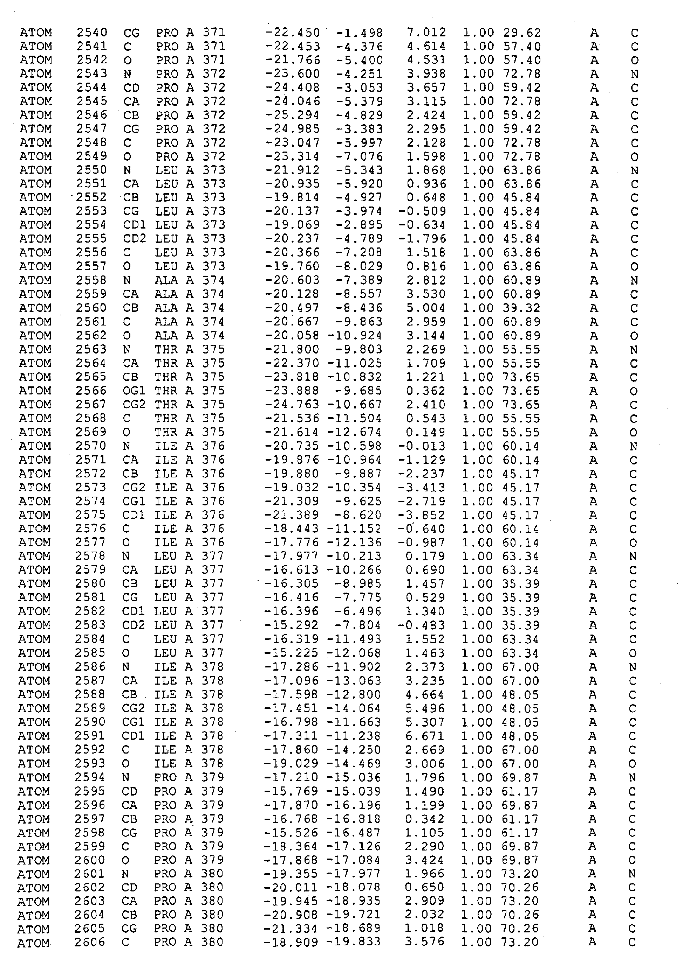

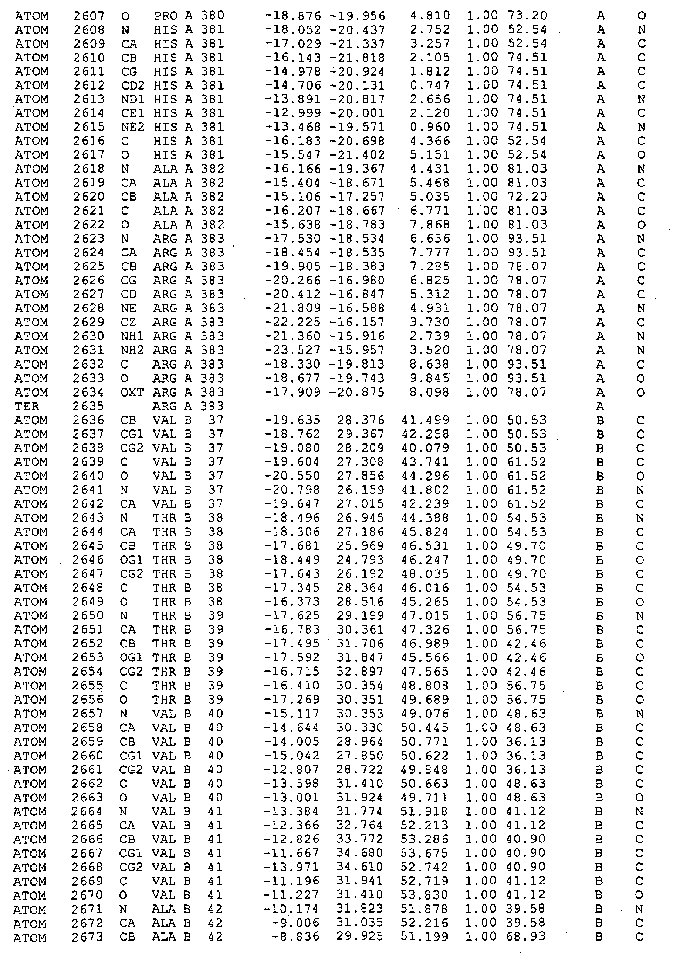

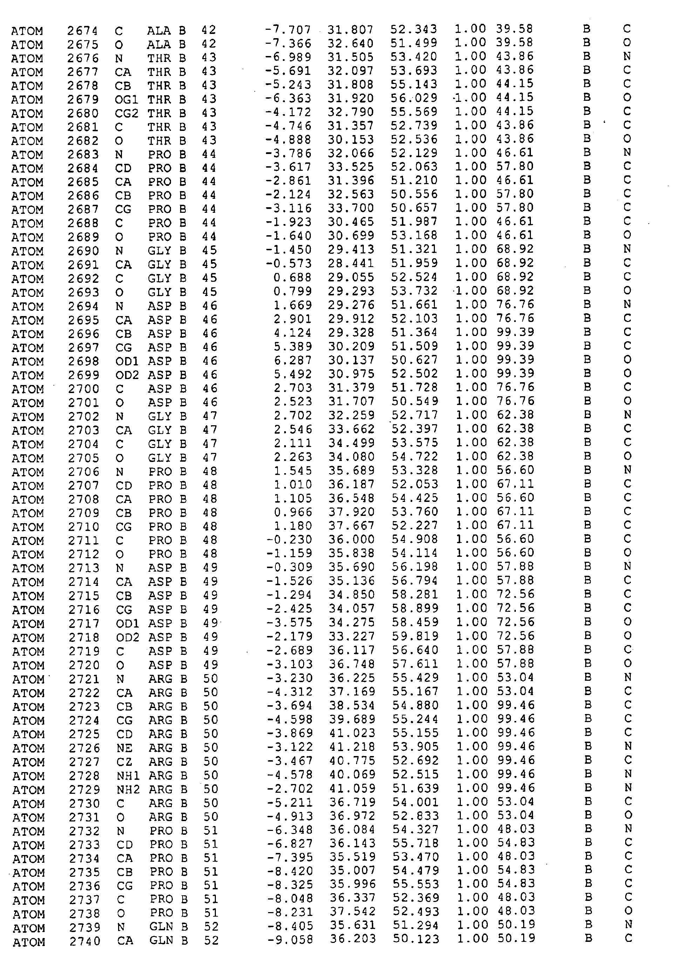

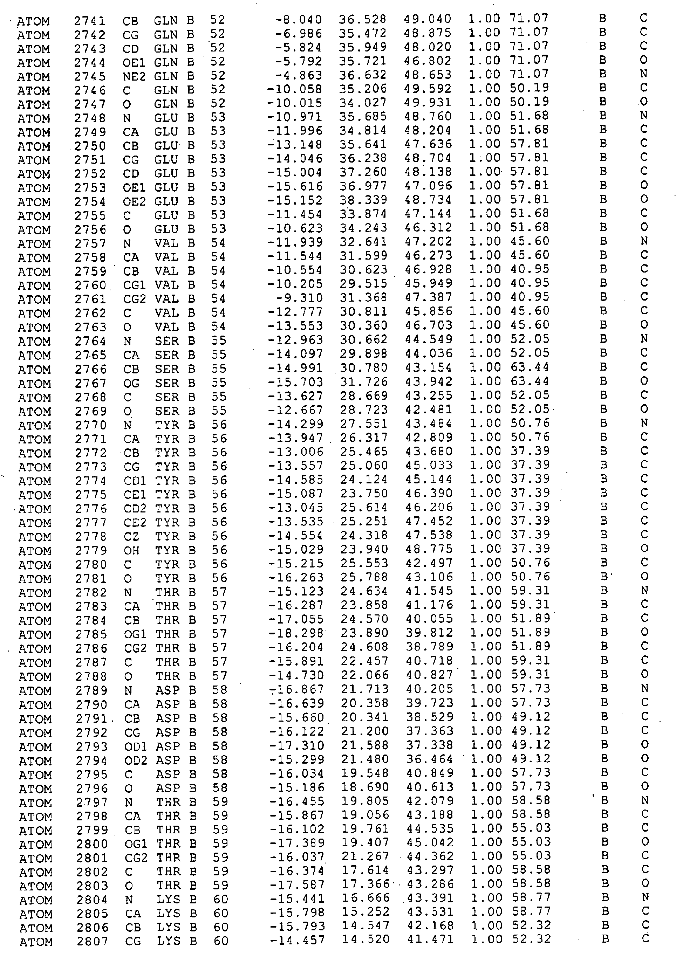

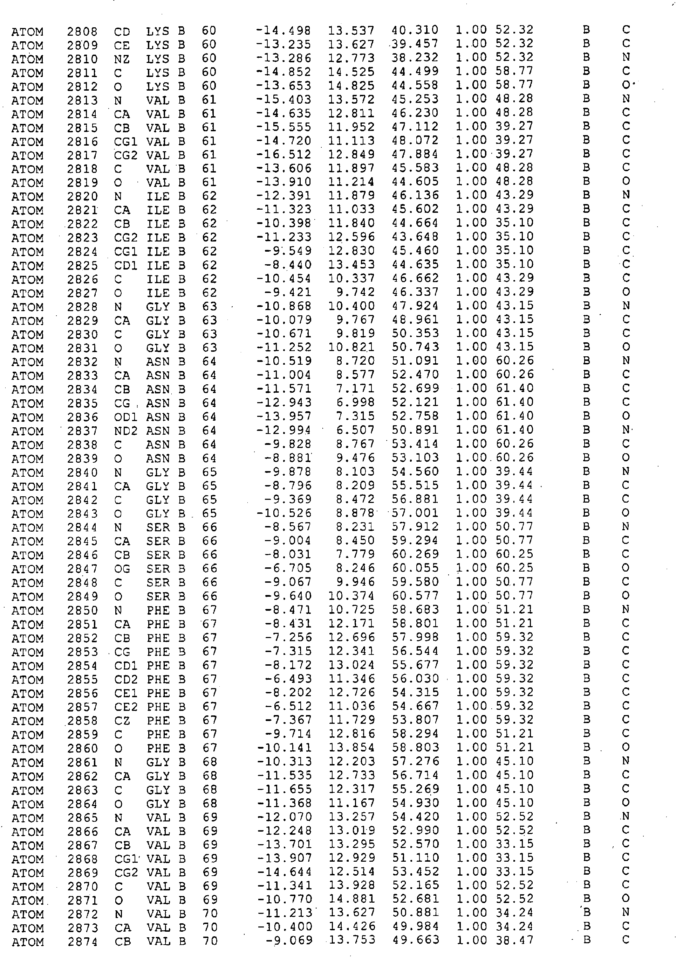

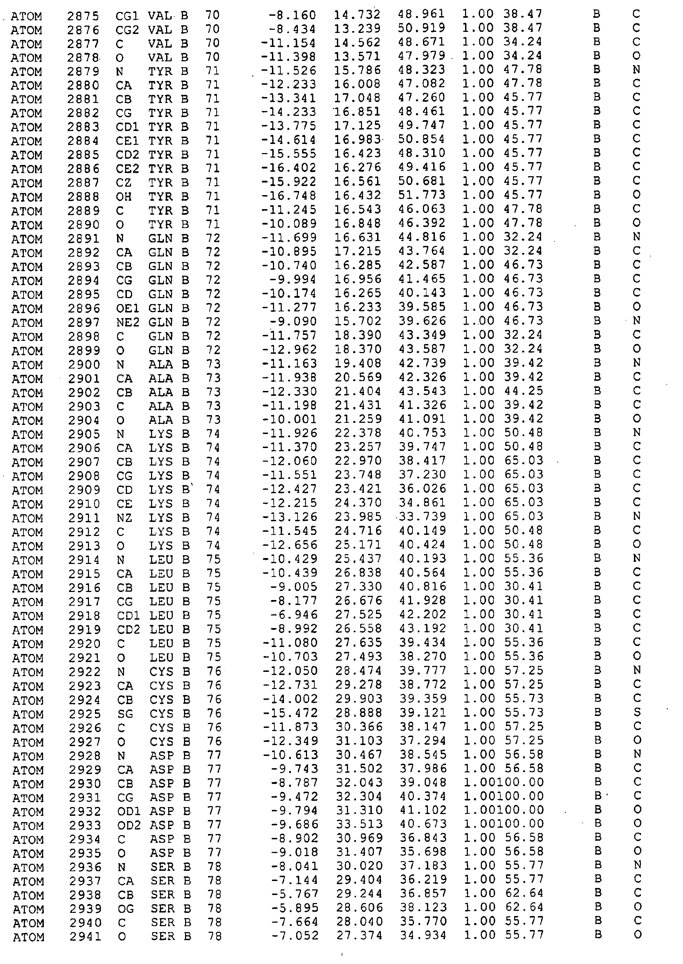

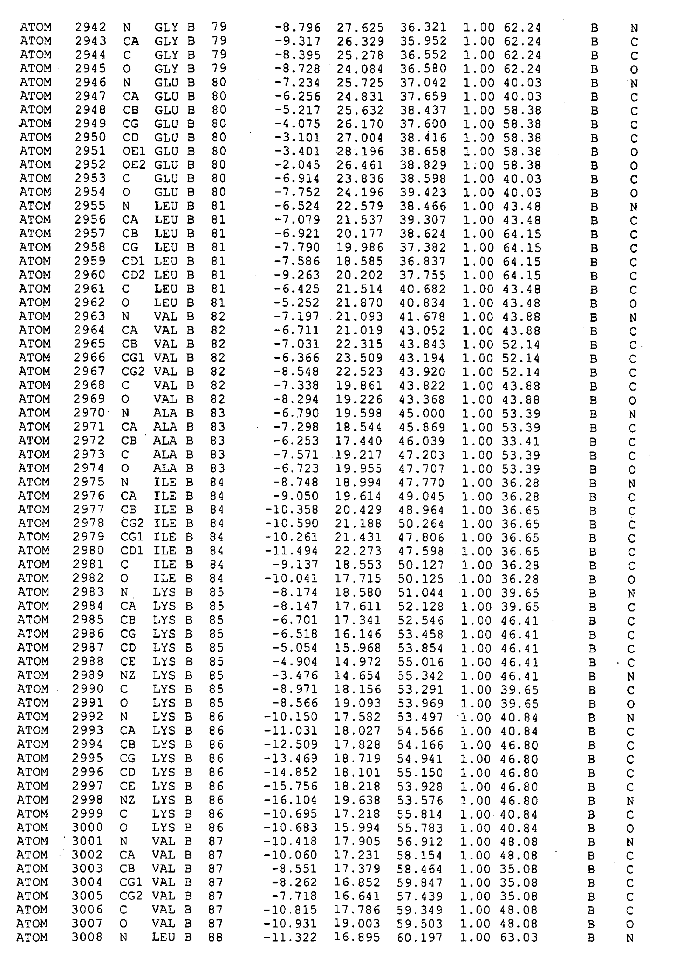

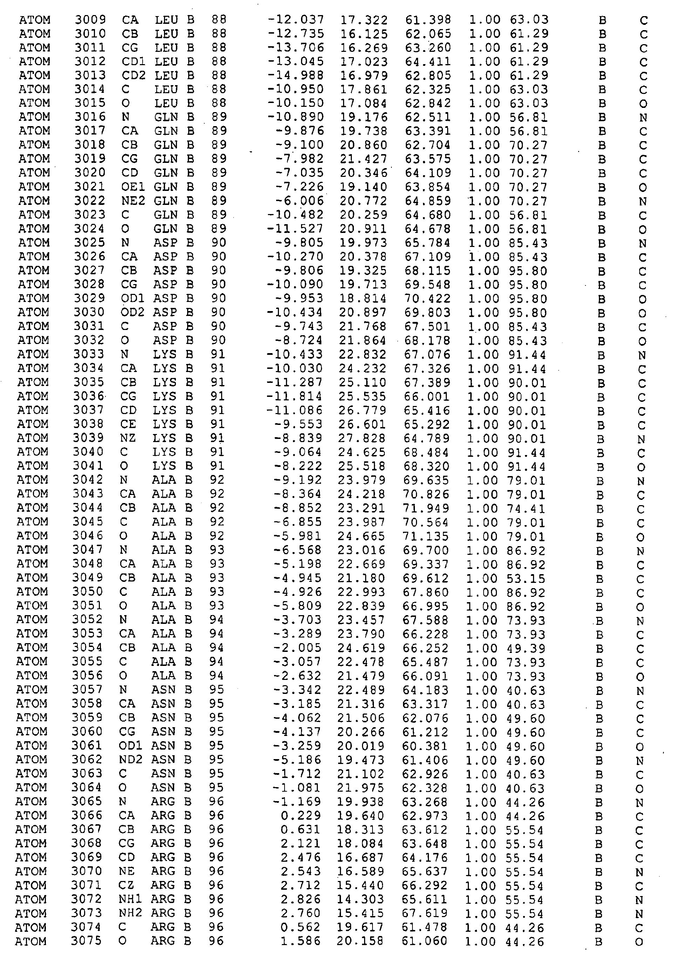

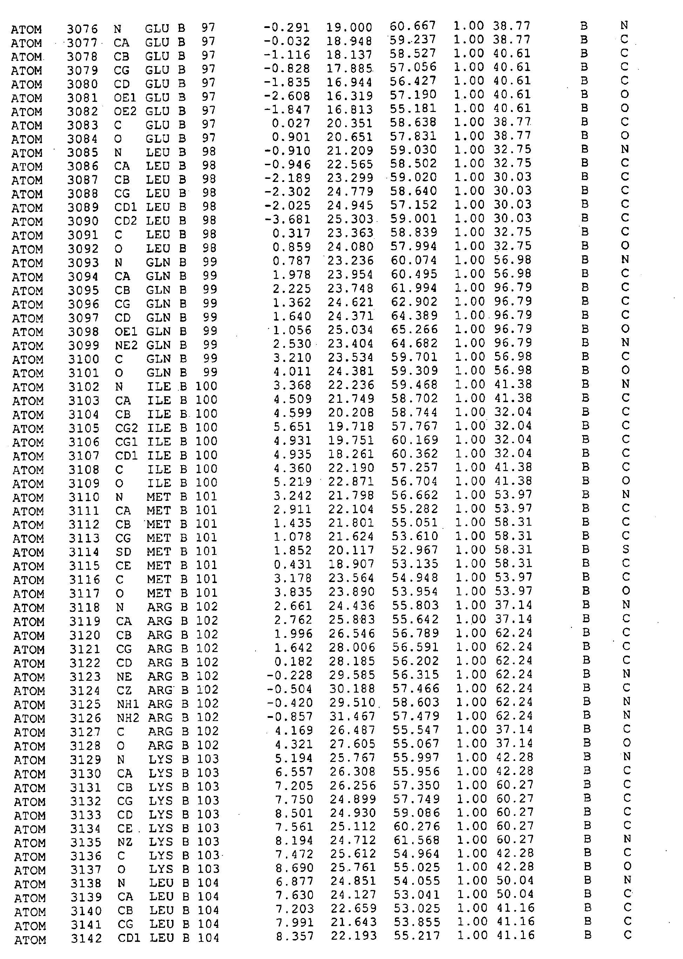

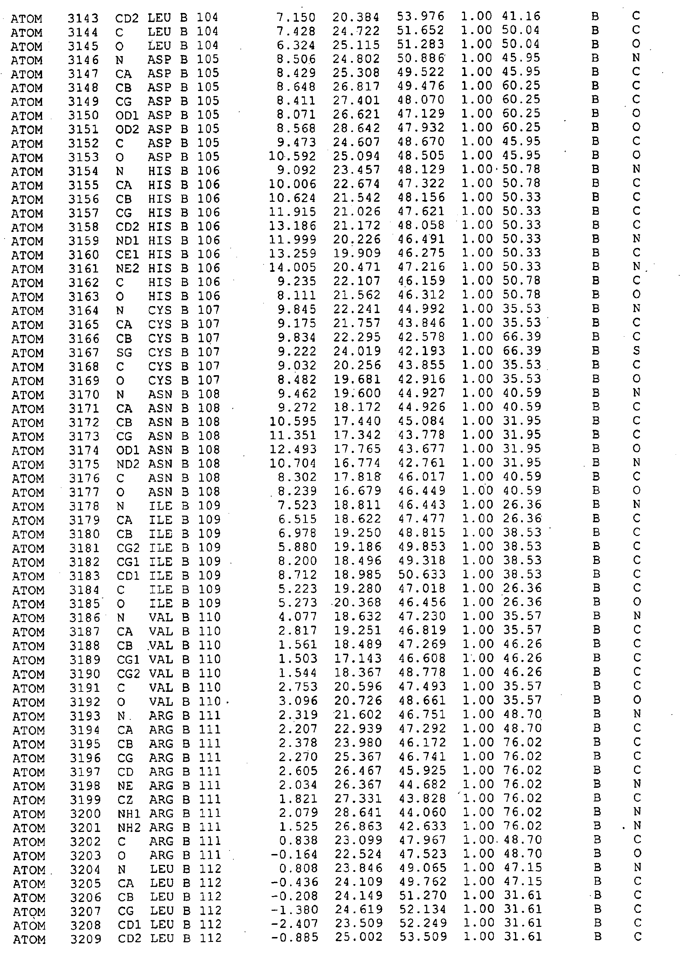

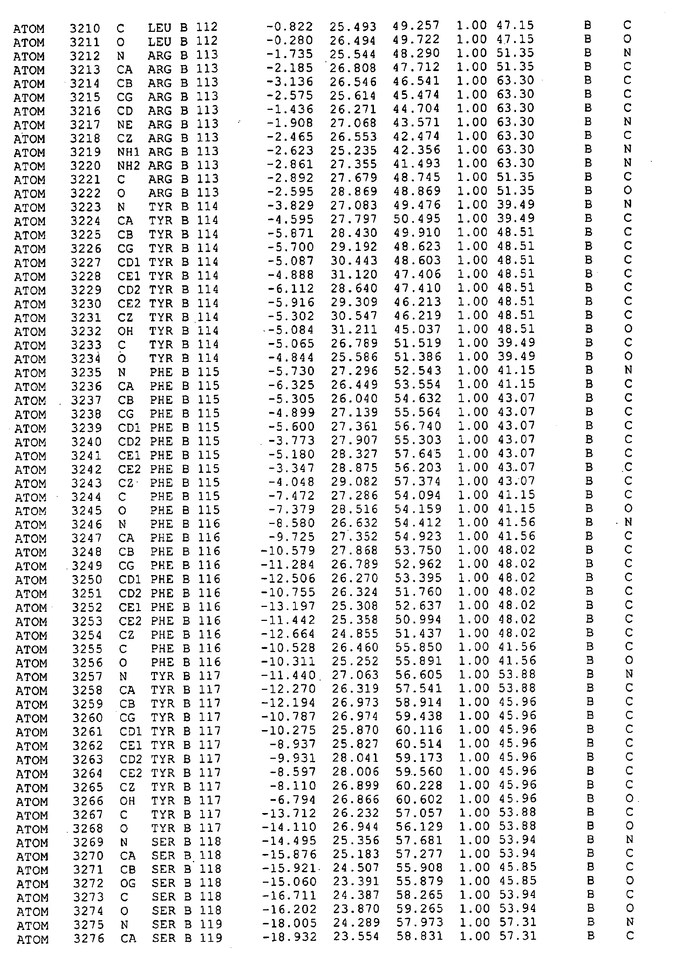

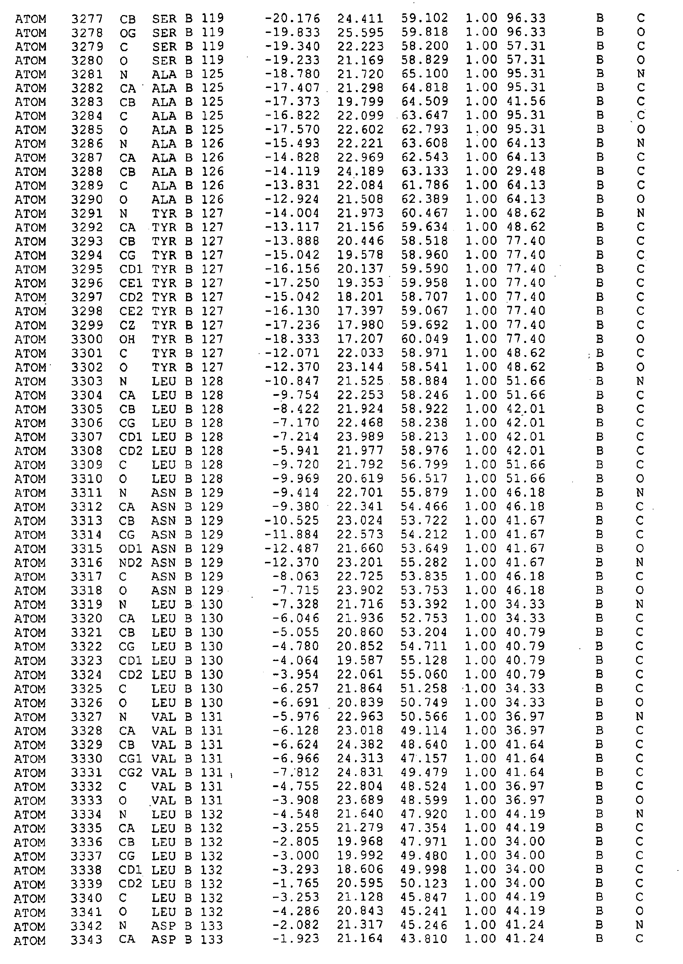

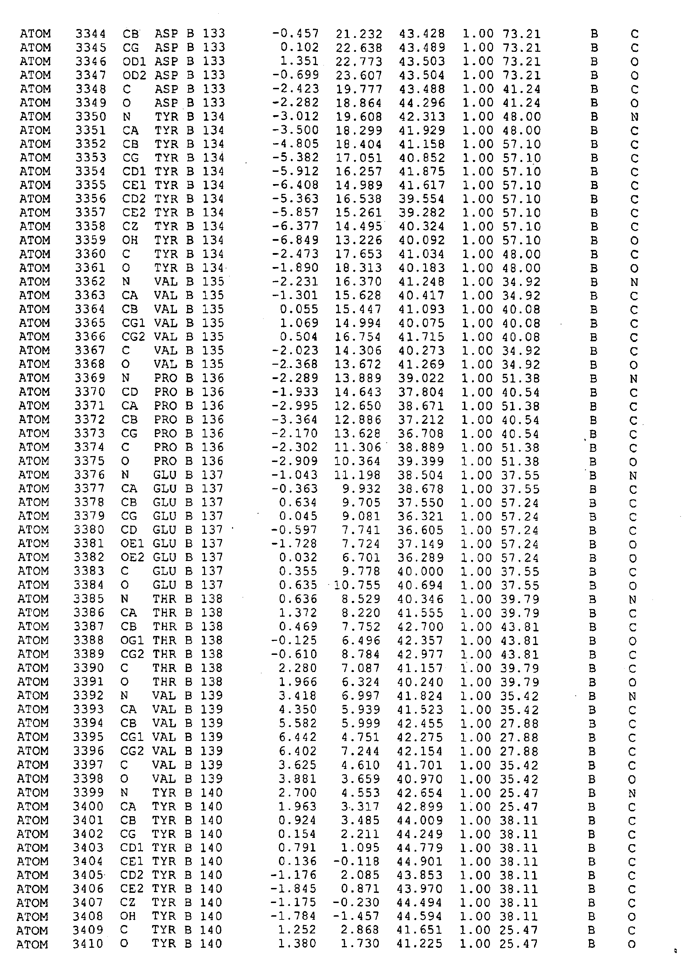

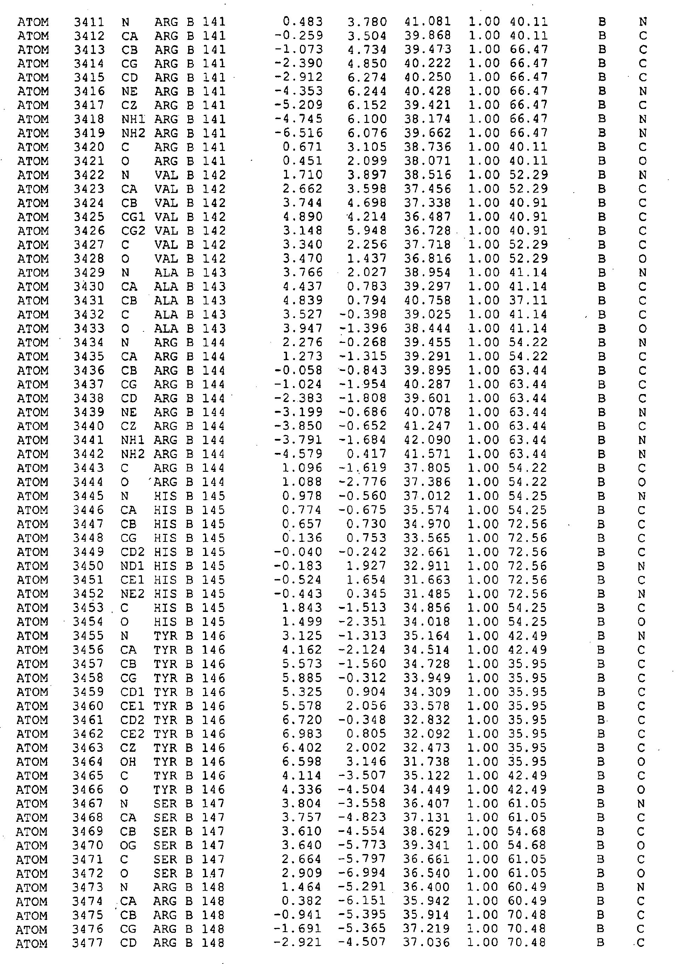

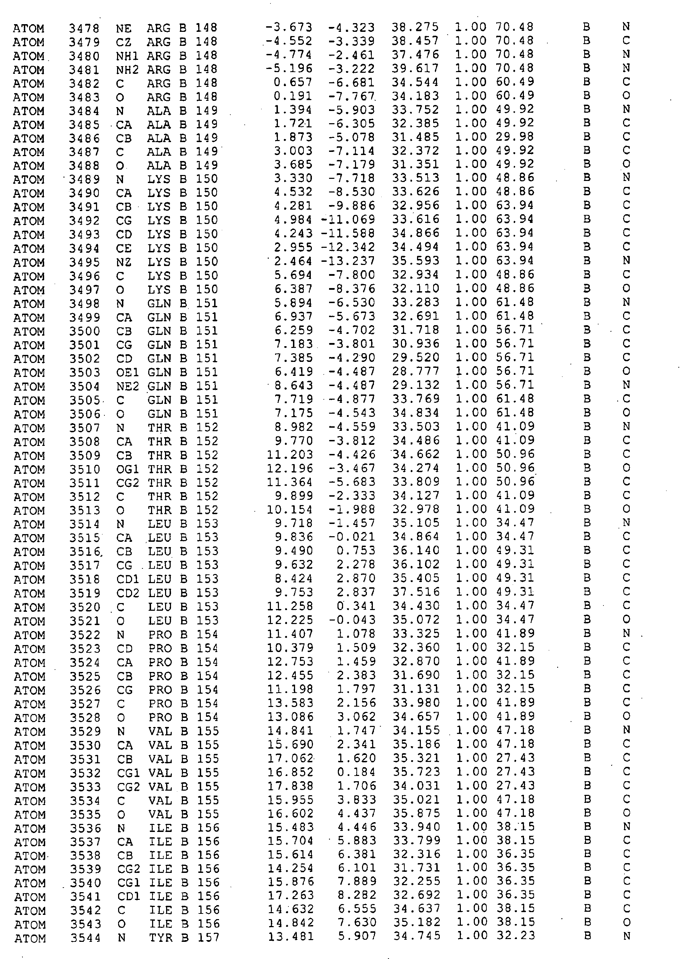

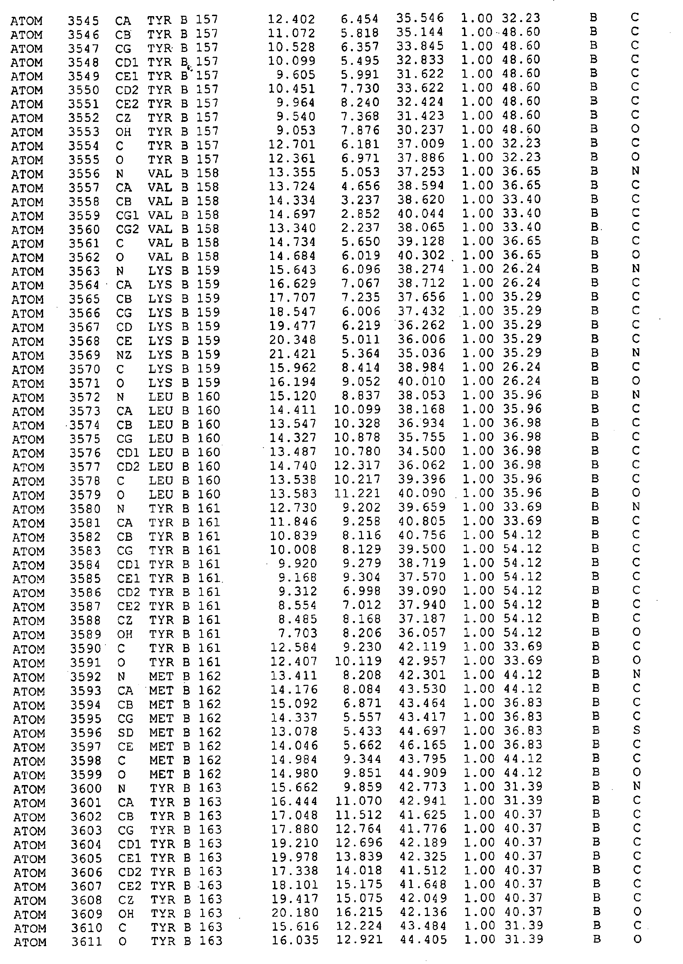

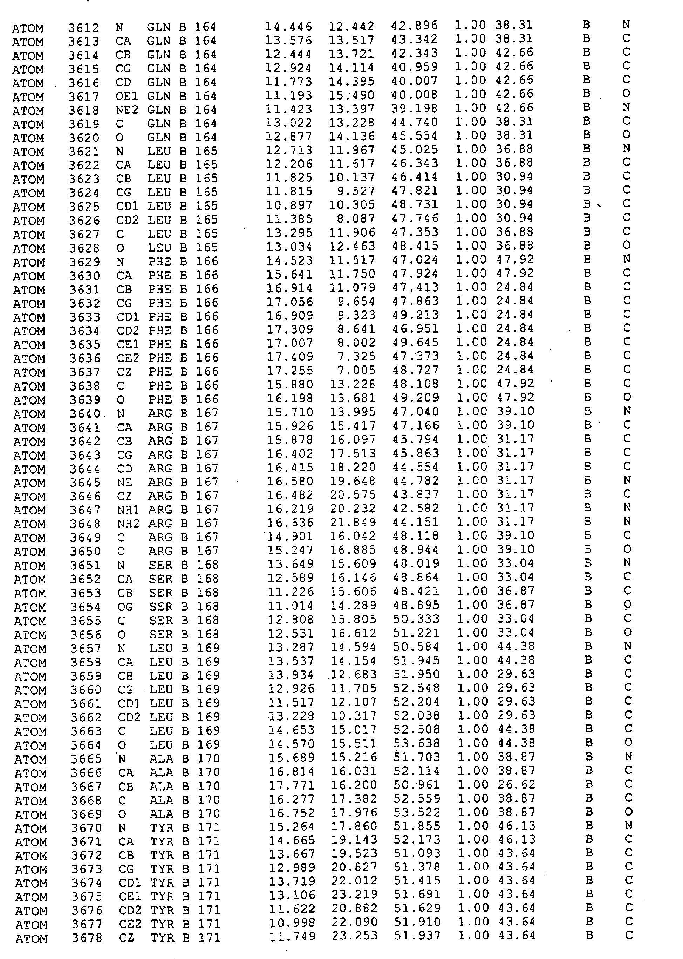

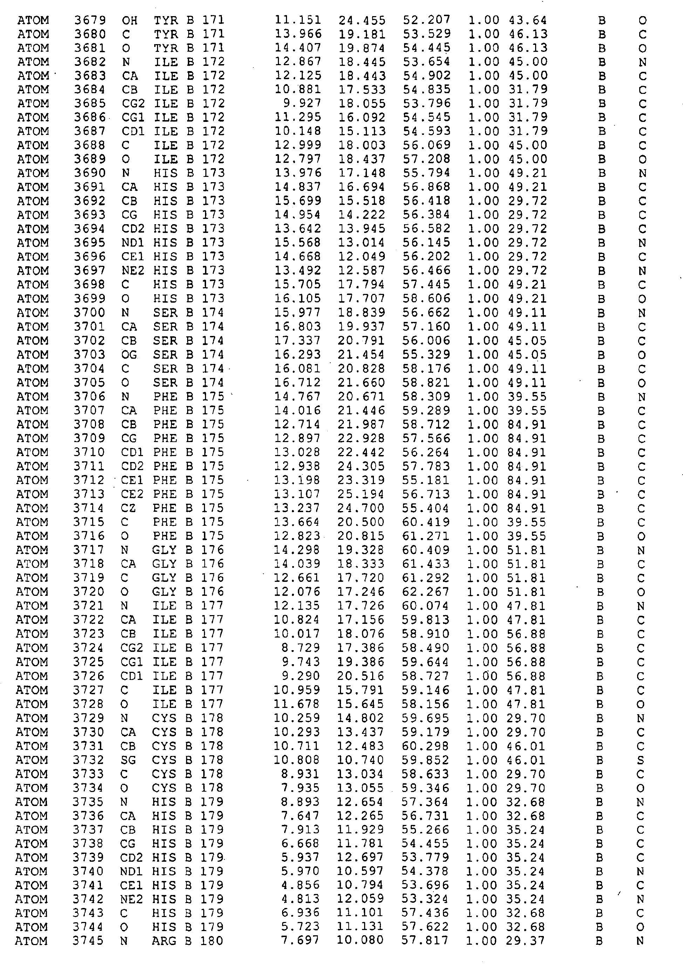

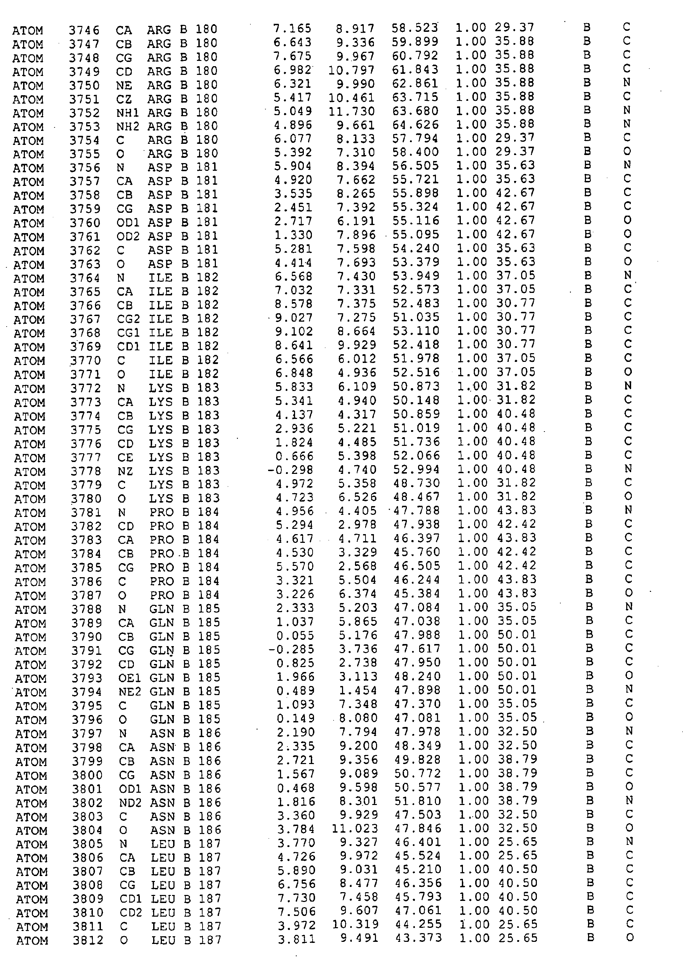

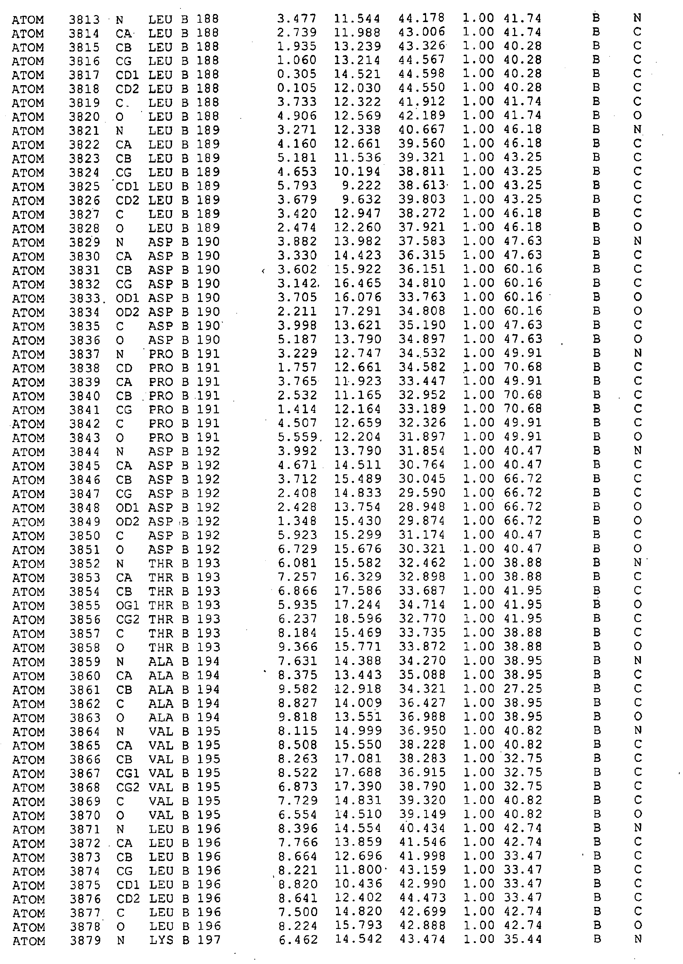

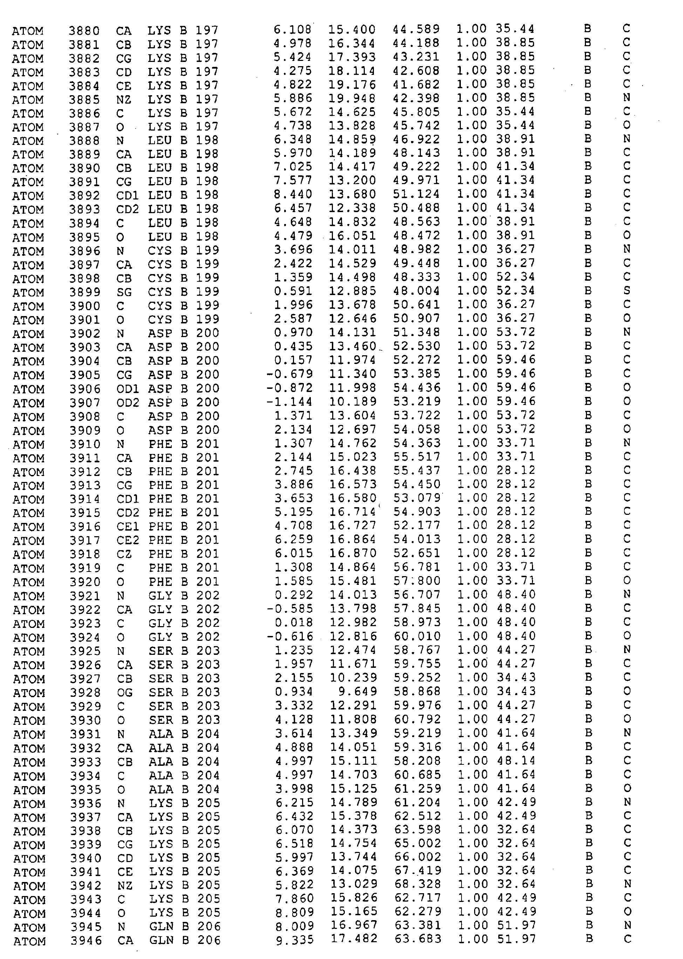

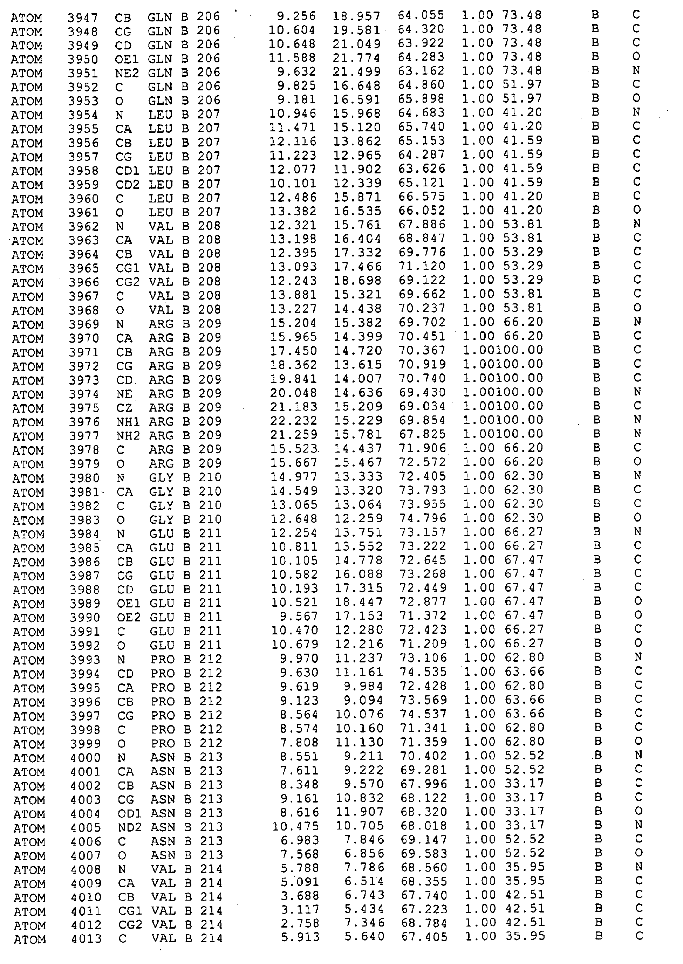

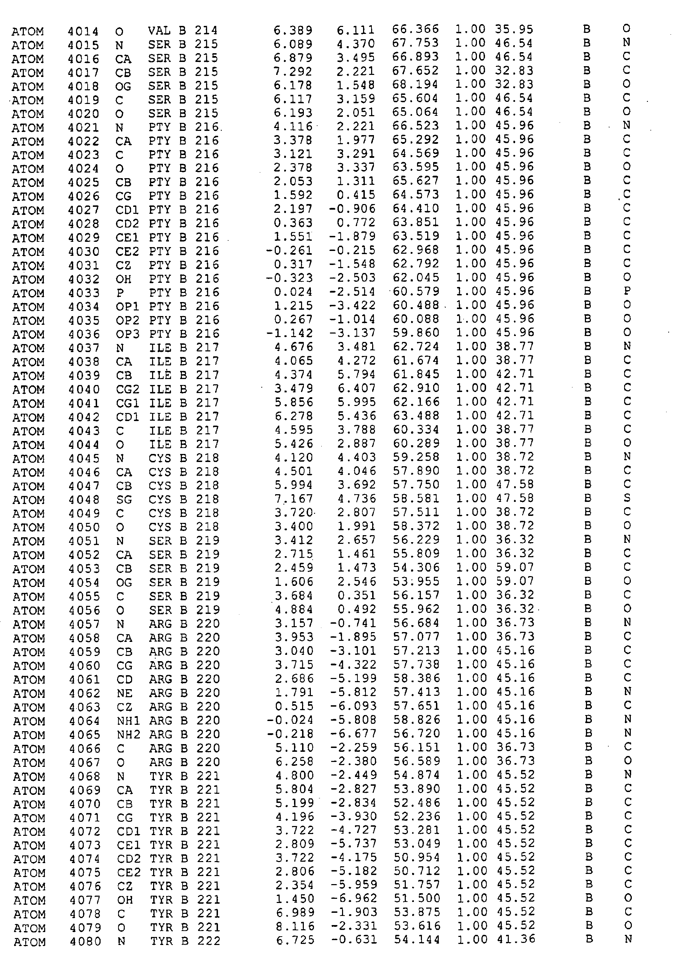

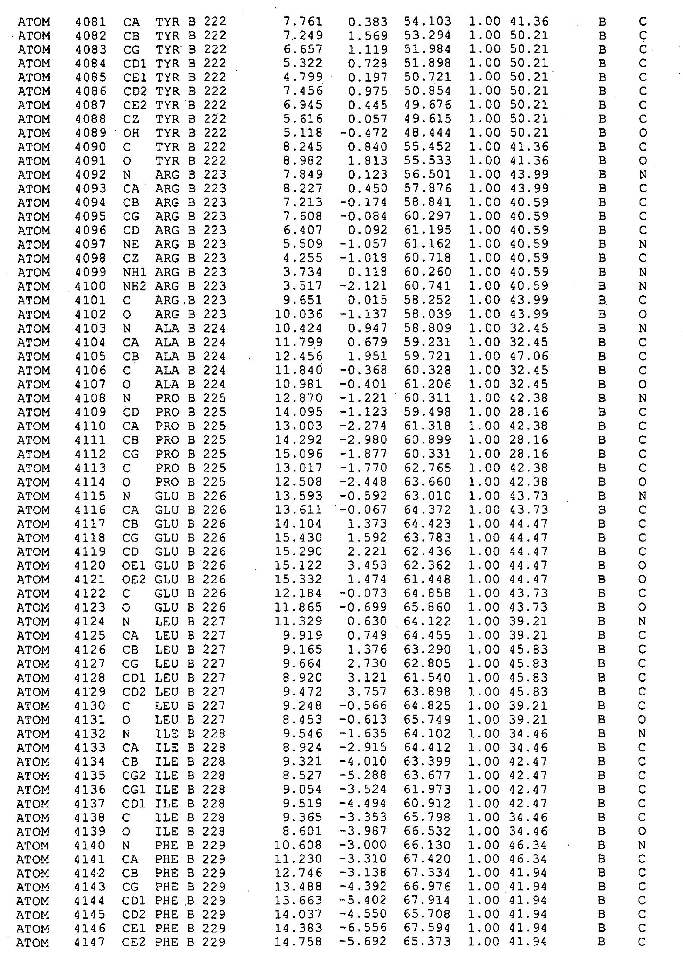

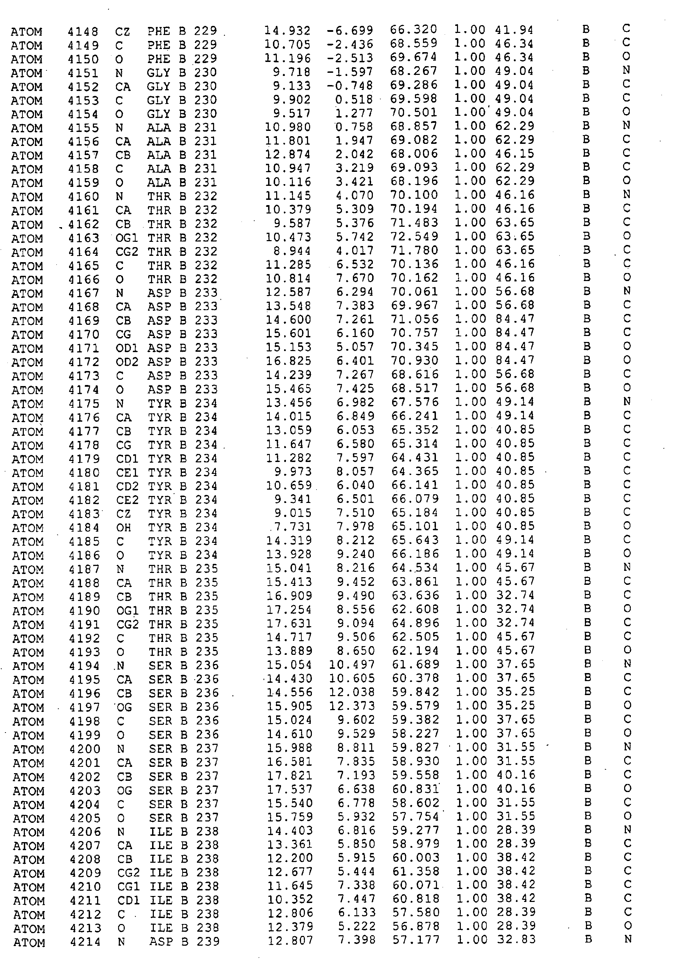

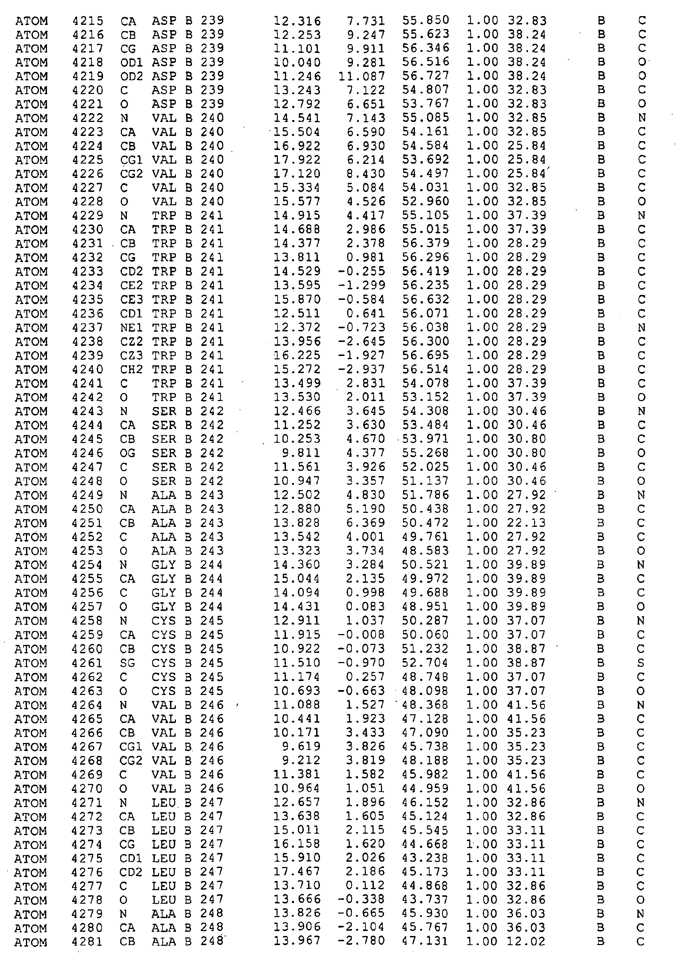

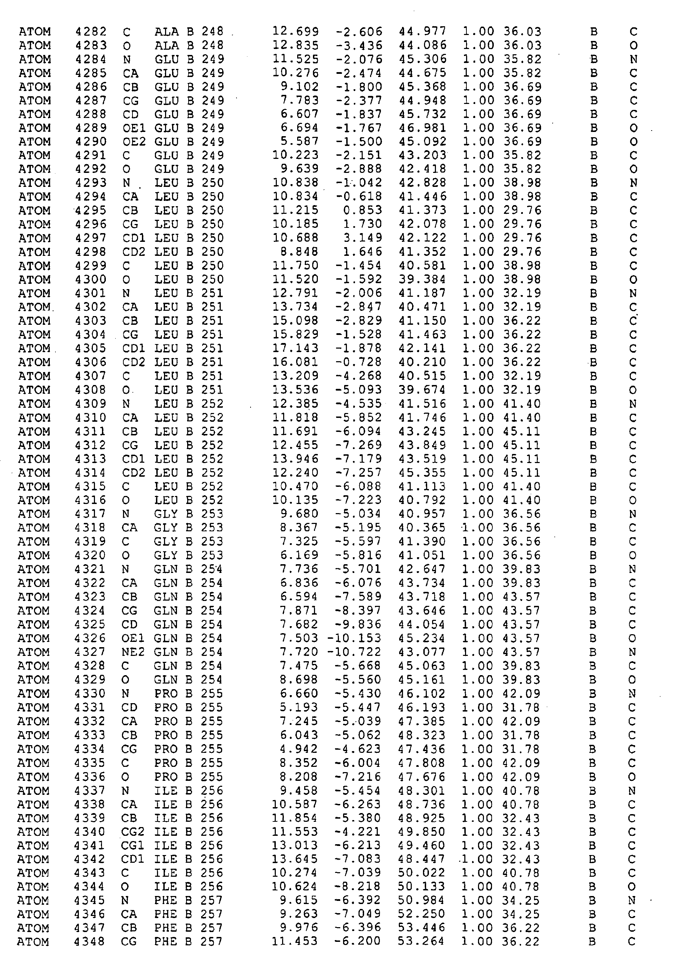

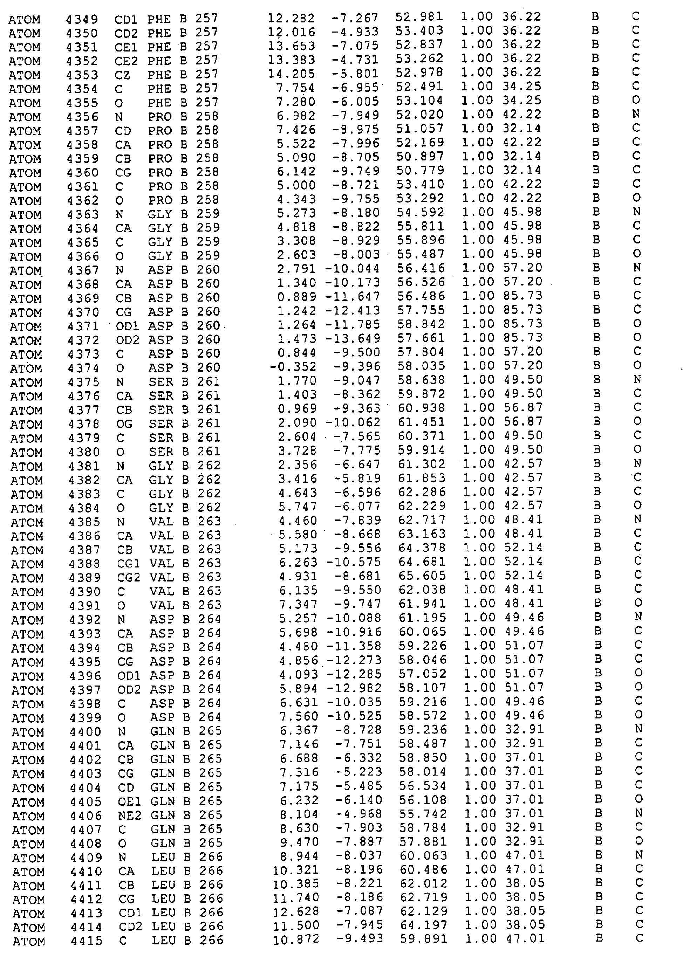

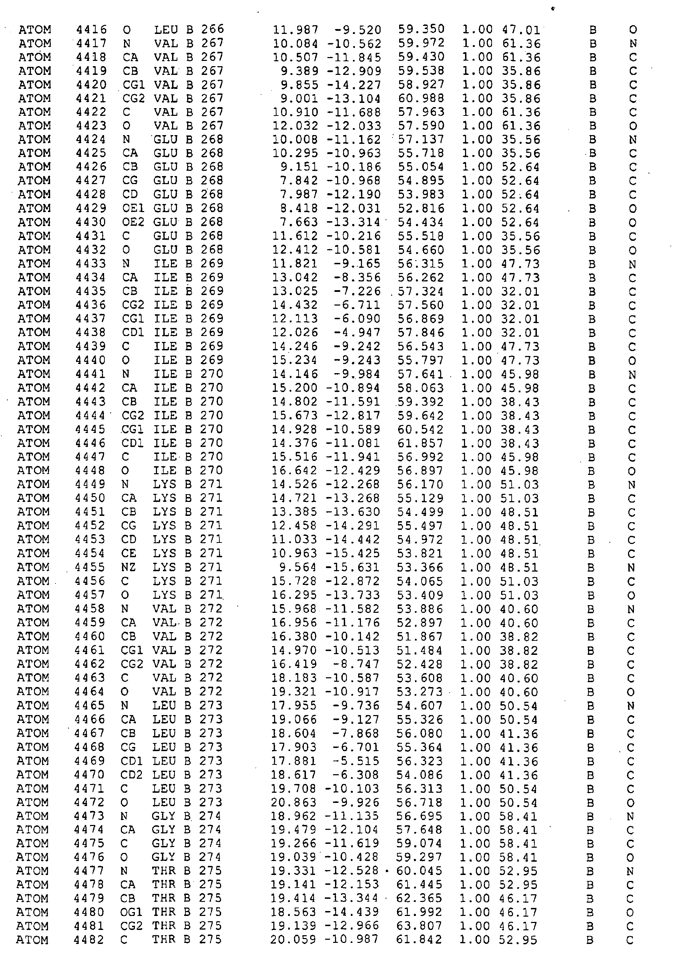

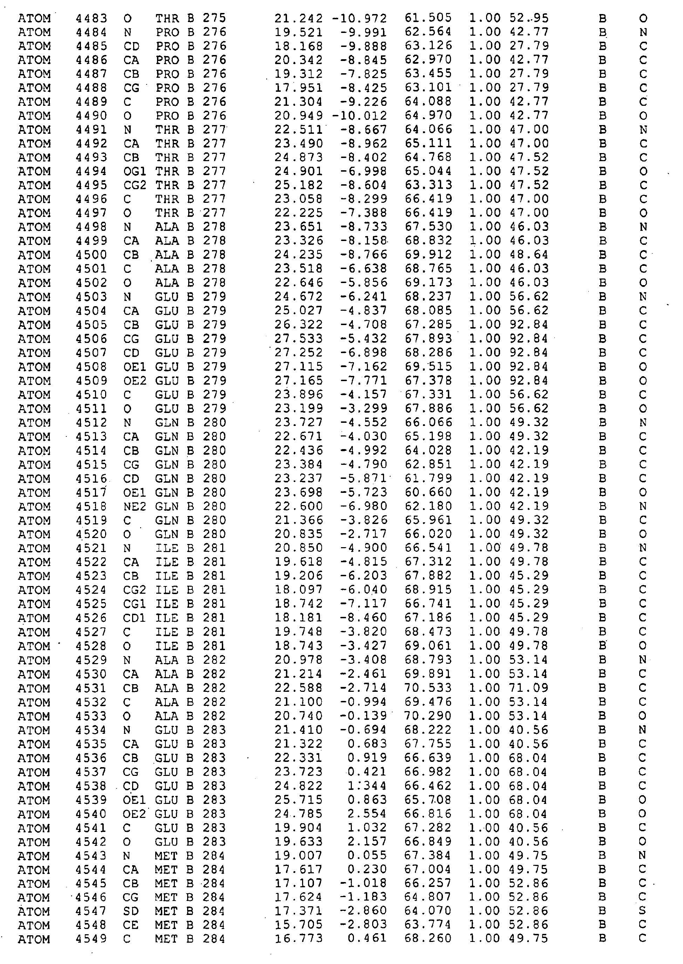

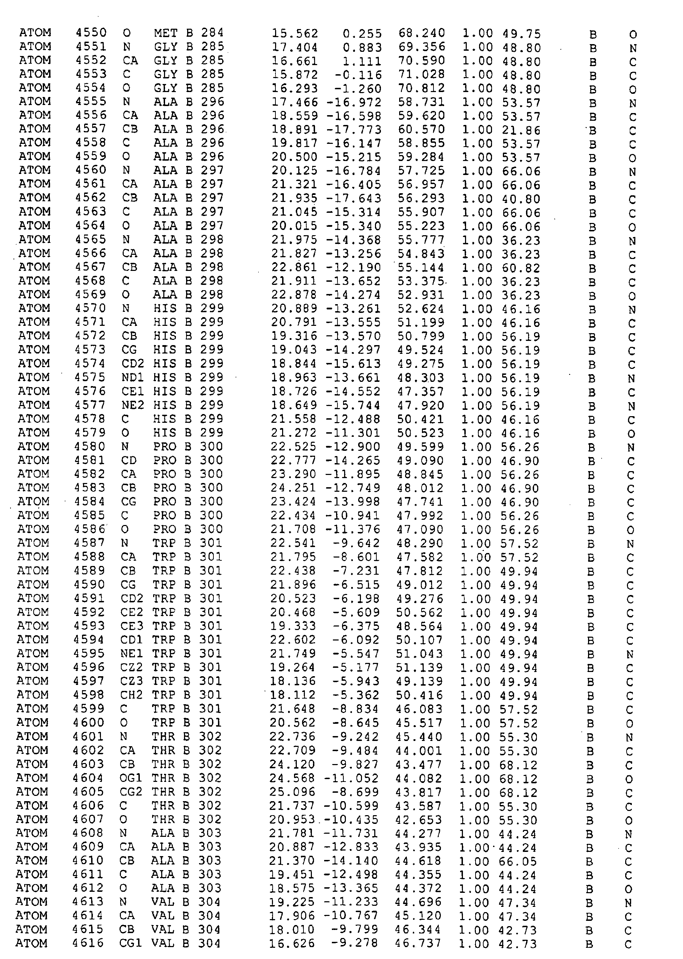

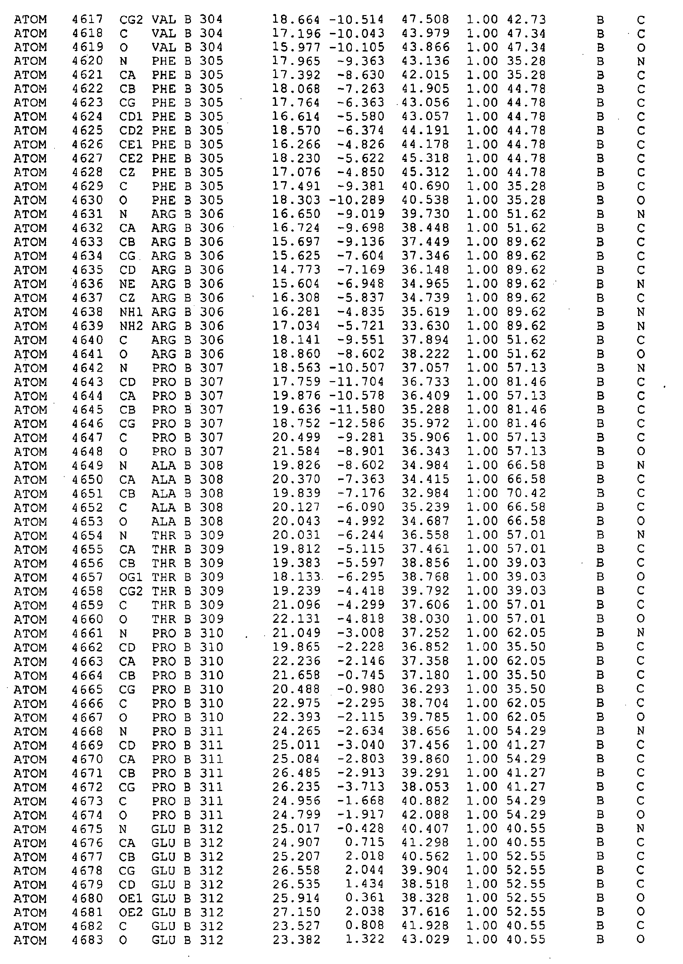

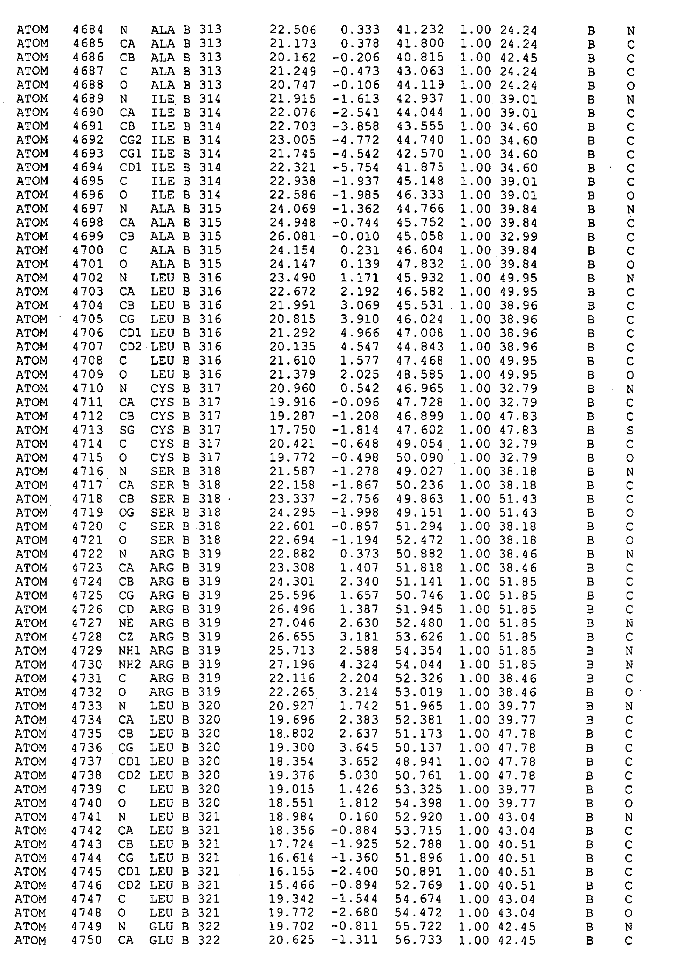

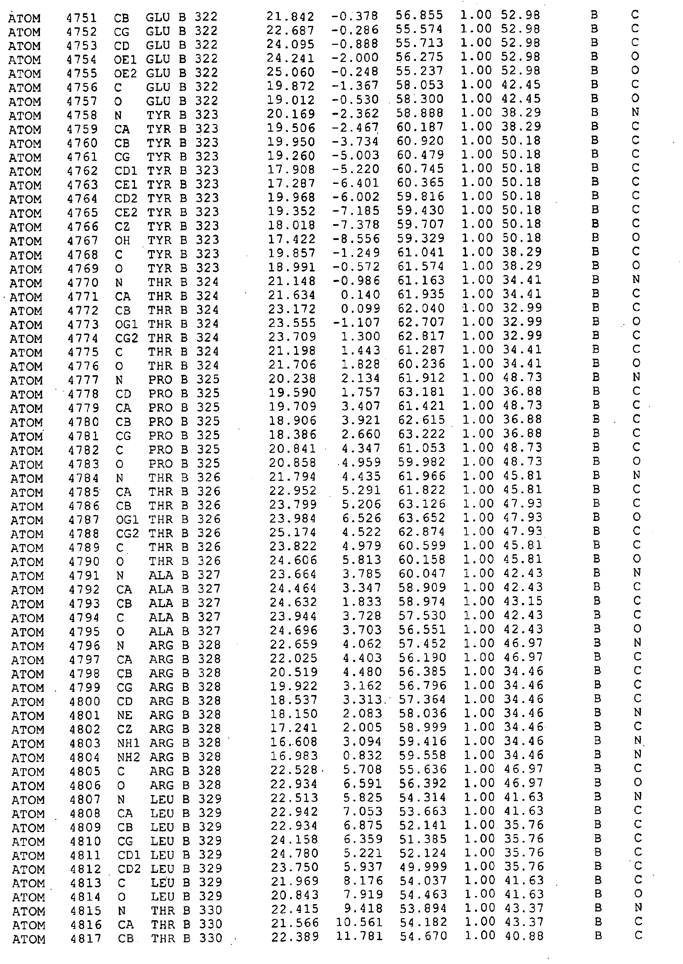

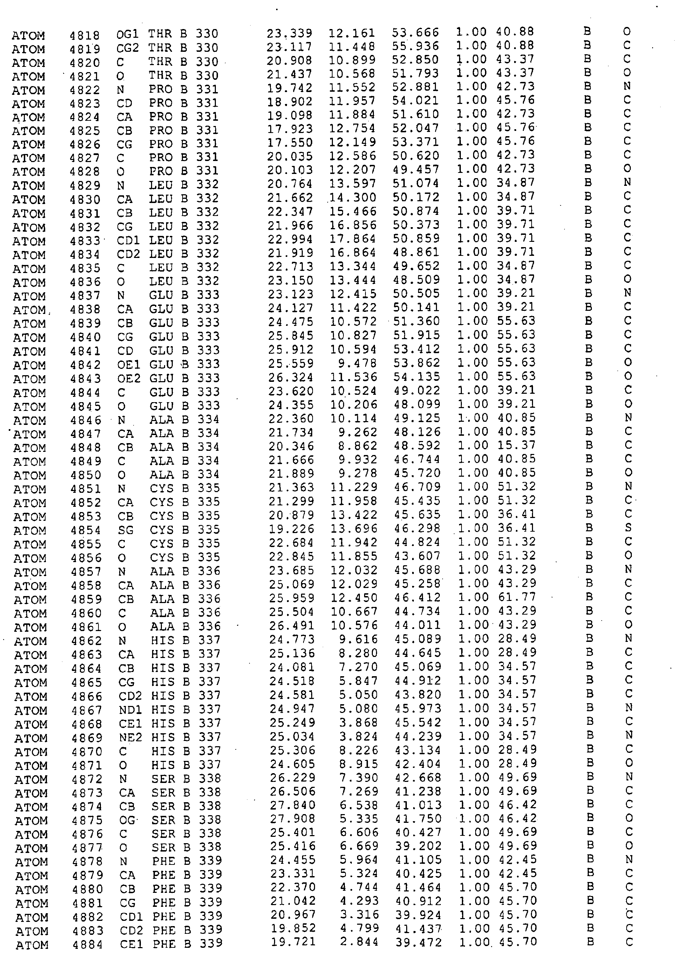

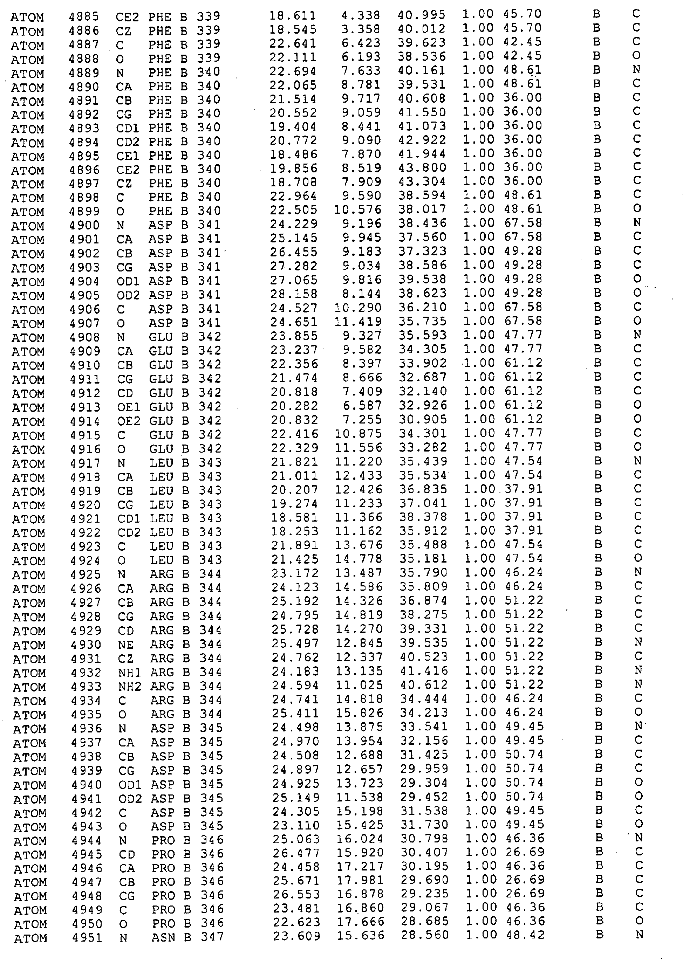

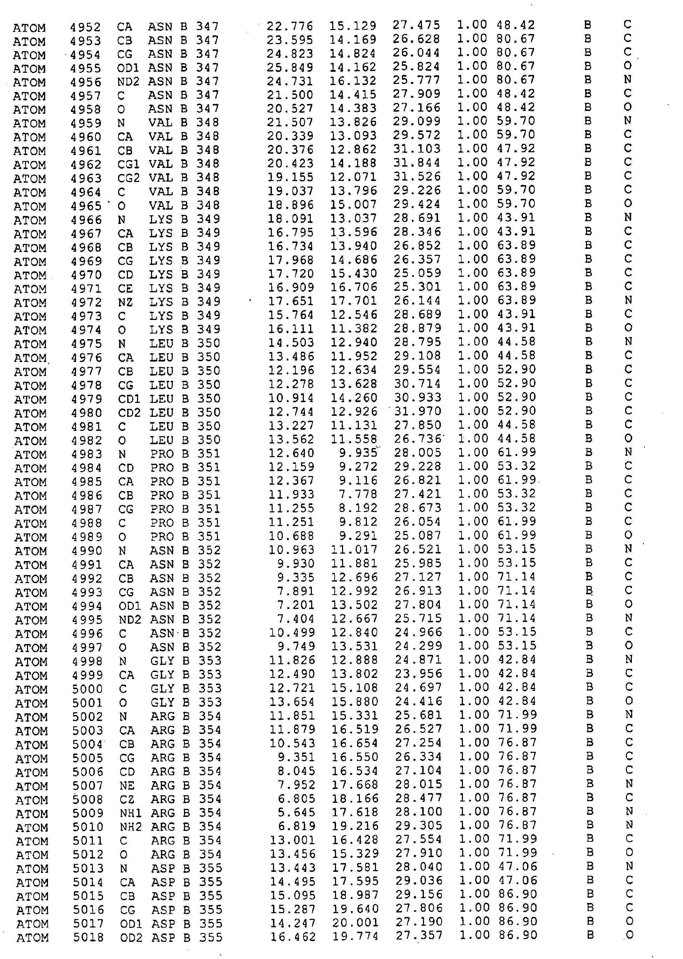

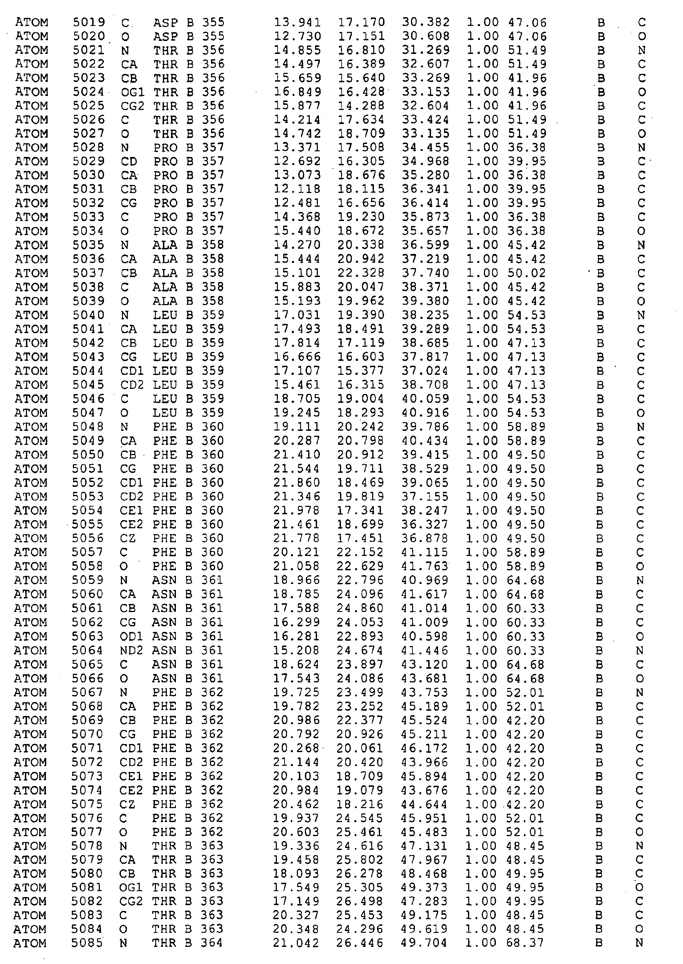

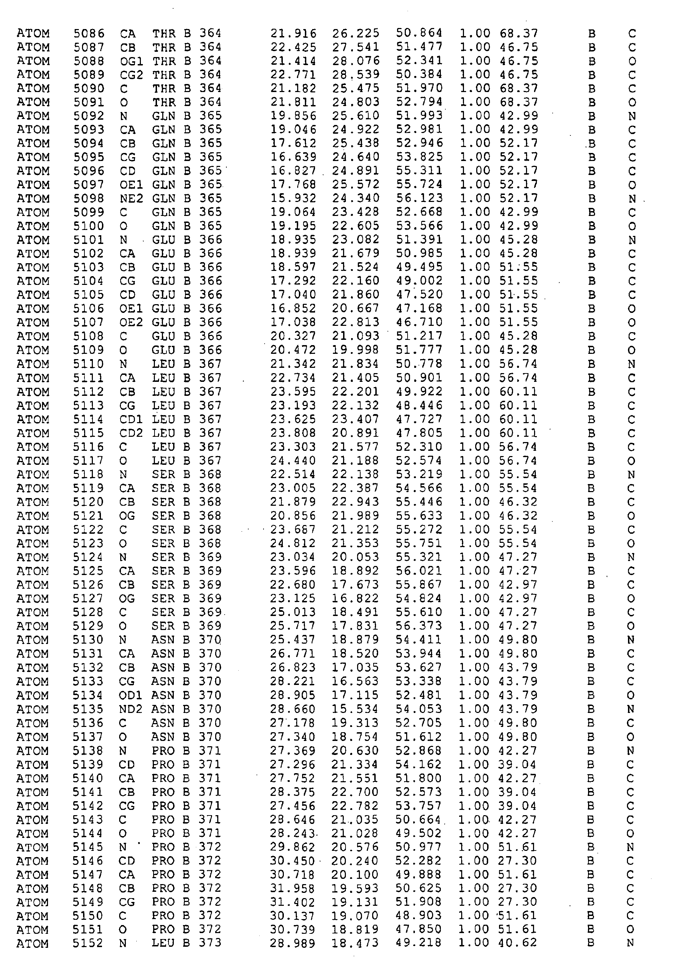

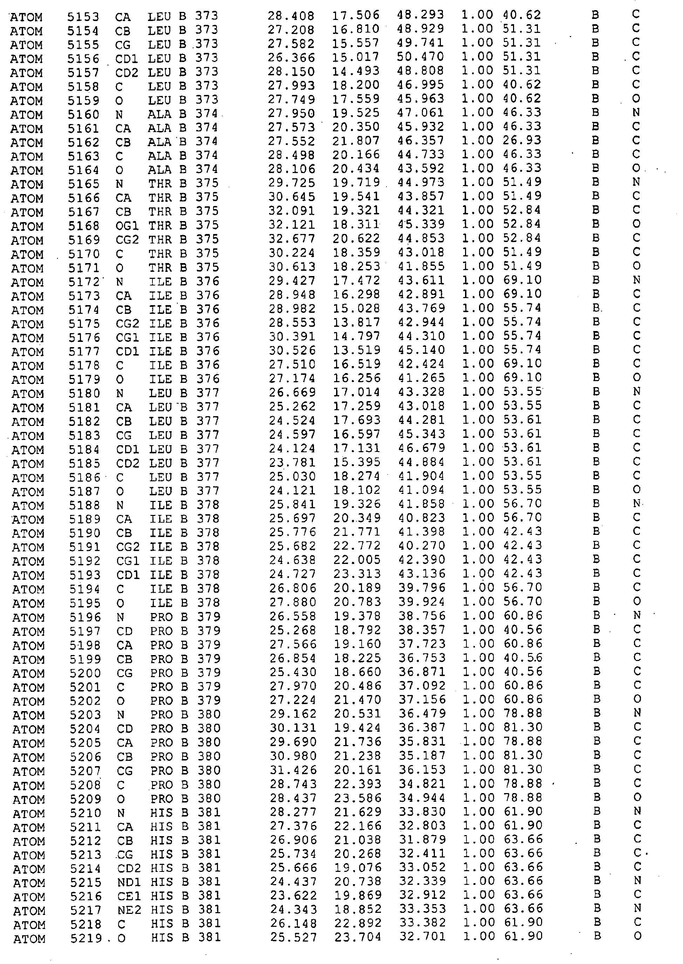

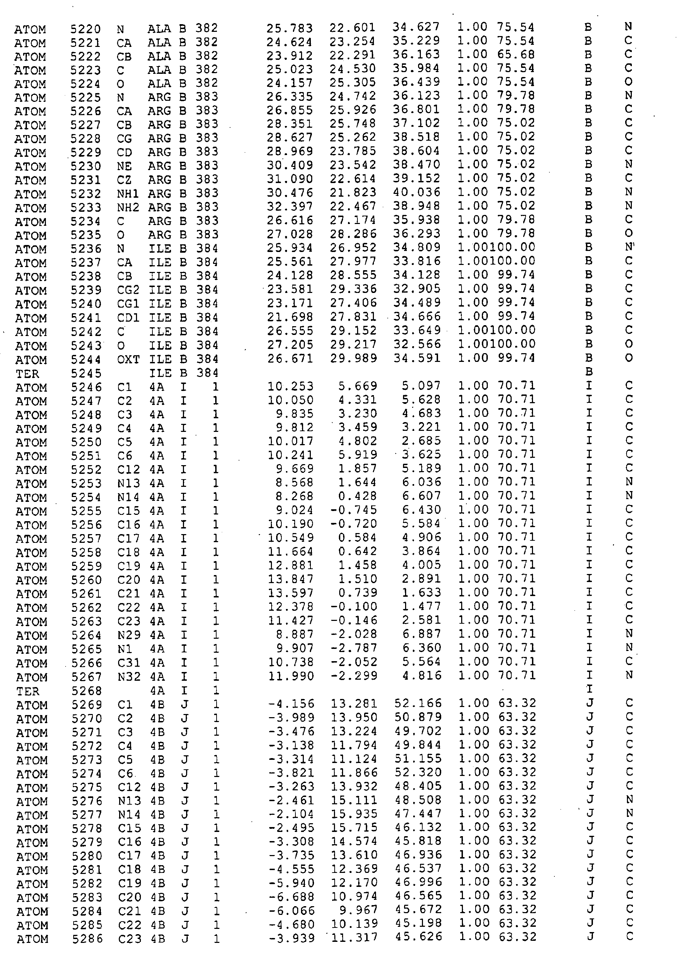

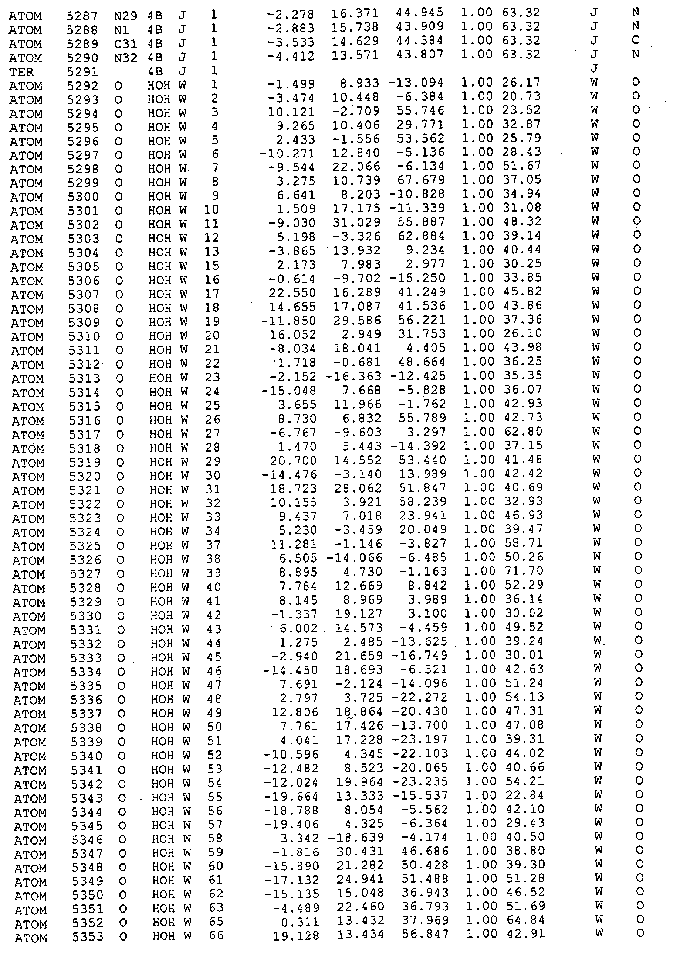

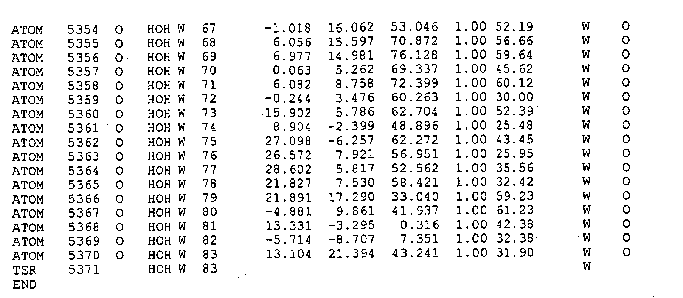

In the crystal structure of the phosphorylated GSK-3β-inhibitorl complex, amino acid residues 162, G63, F67, V70, A83, K85, V110, L132, D133, Y134, V135, T138, N186, L188, C199, and D200 according to Figure 3 were within 5 Å of the inhibitor bound in the active site. These amino acids residues were identified using the program QUANTA (Molecular Simulations, Inc., San Diego, CA ©1998), O (Jones et al., Acta Crystallogr. A47, pp. 110-119 (1991)) and RIBBONS (Carson, J. Appl. Crystallogr., 24, pp. 9589-961 (1991)). The programs allow the display and output of all residues within 5 Å from the inhibitor. In addition, amino acid residues V61, I62, G63, N64, G65, F67, V69, V70, A83, K85, K86, V87, E97, M101, V110, R111, L130, V131, L132, D133, Y134, V135, P136, E137, T138, R141, D181, K183, Q185, N186, L187, L188, L189, K197, L198, C199, D200, F201 and G202 according to Figure 3 were within 8 Å of the inhibitor bound in the active site. These amino acid residues were identified using the programs QUANTA, O and RIBBONS, supra.

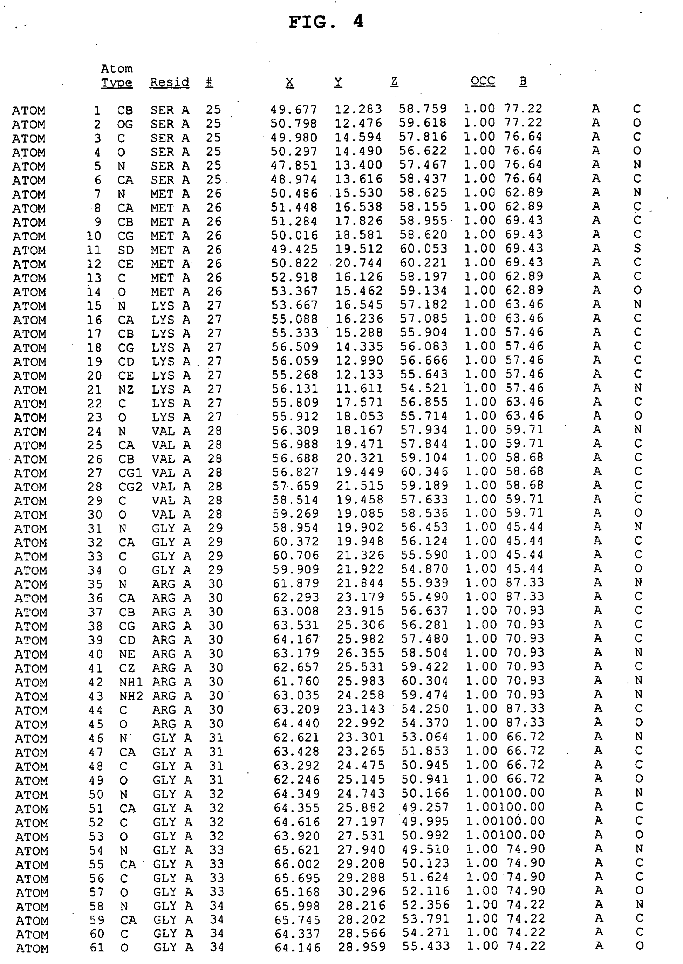

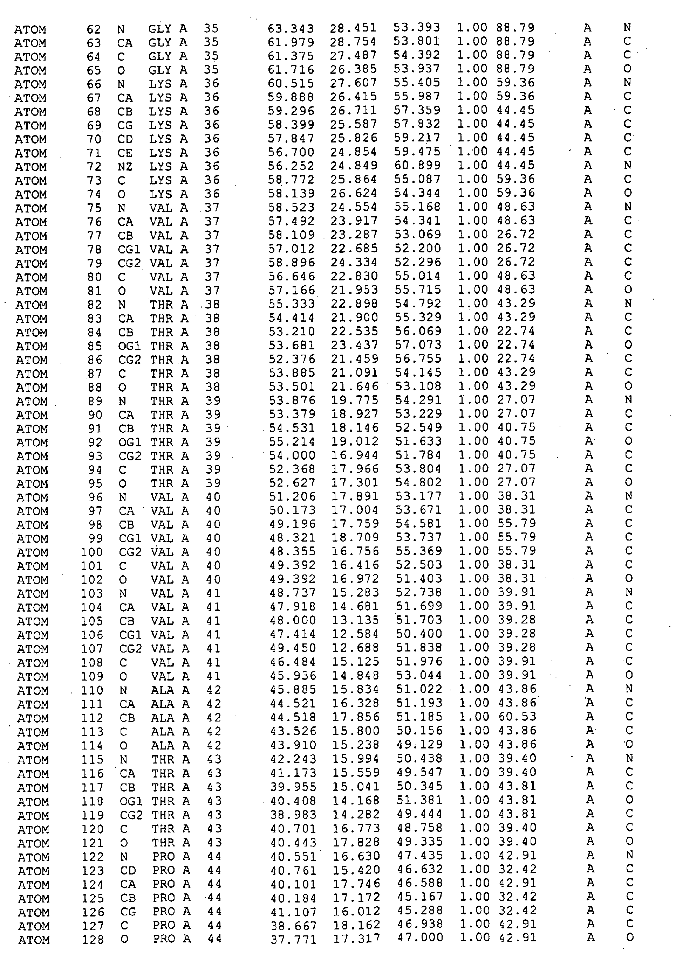

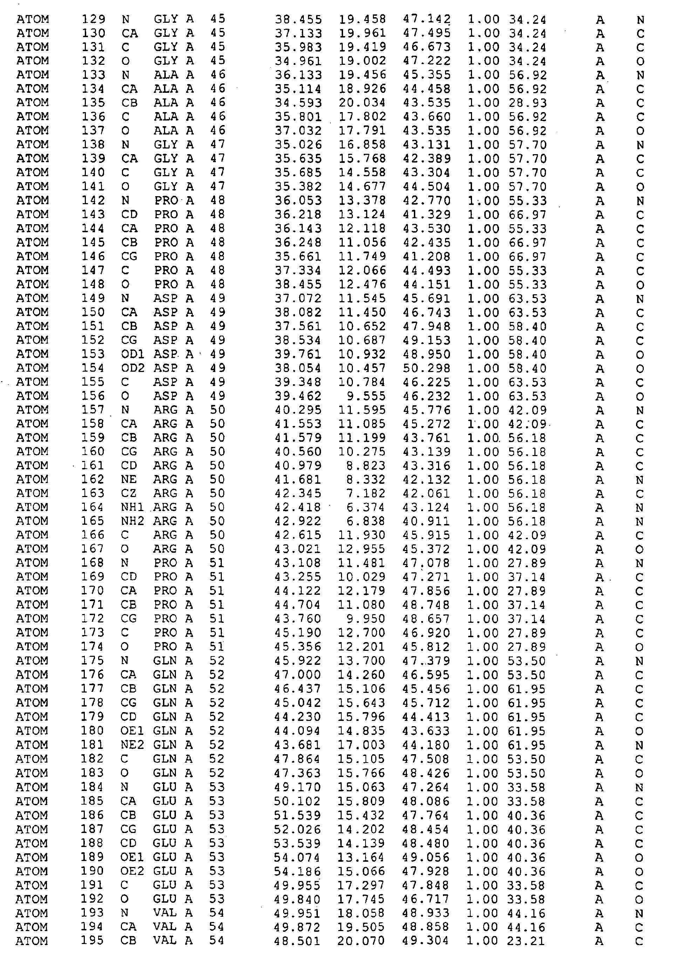

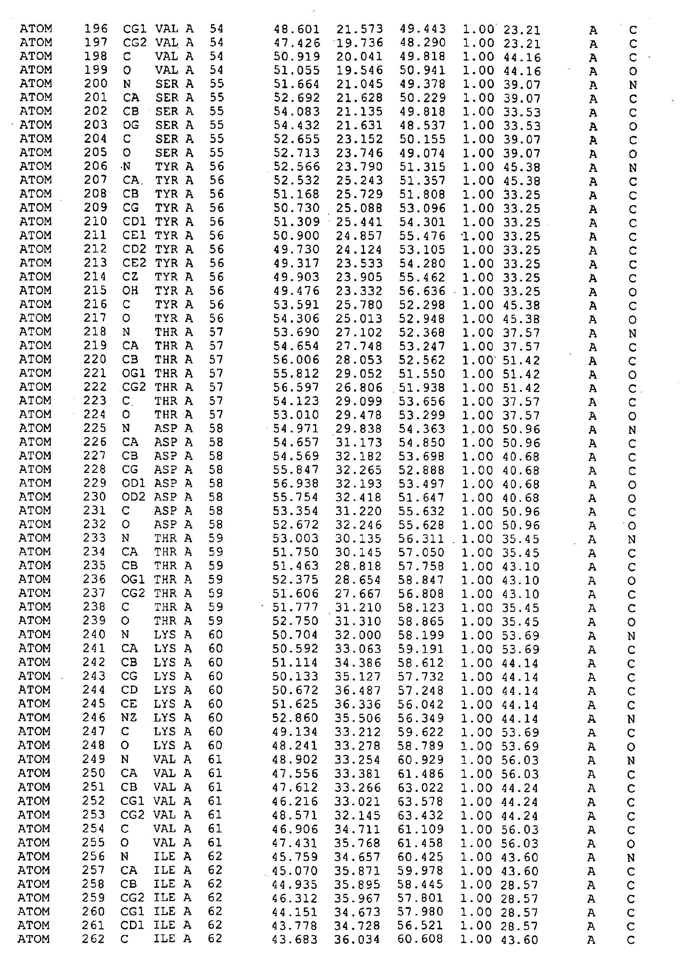

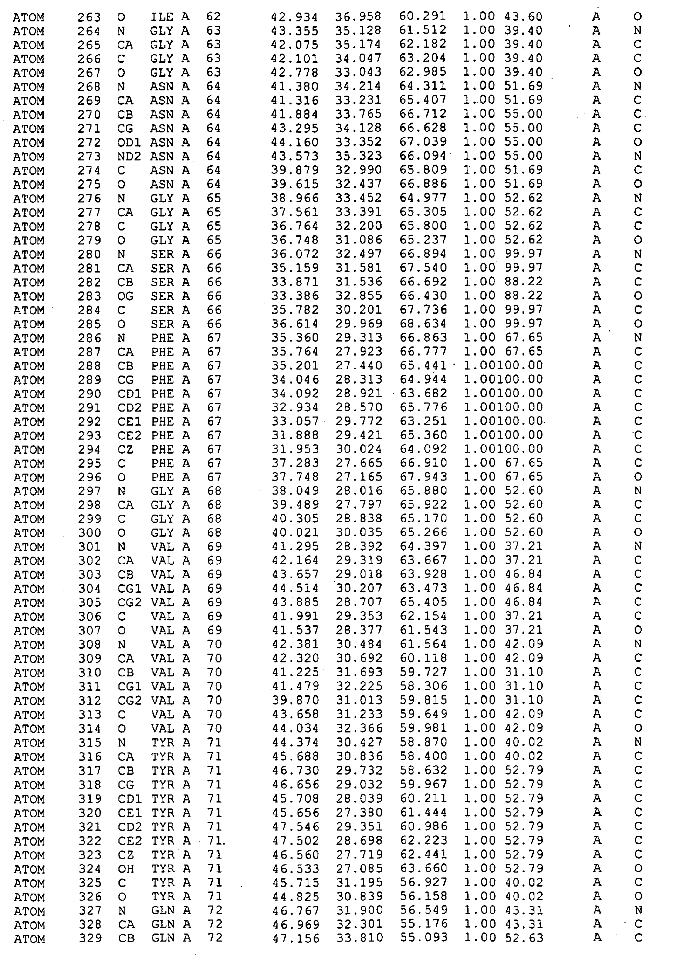

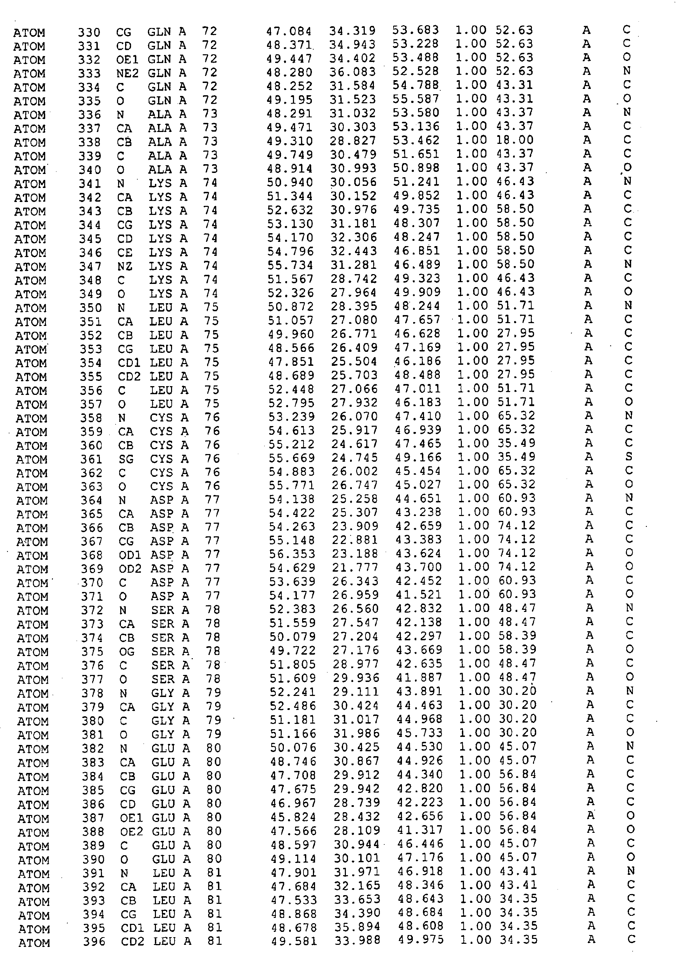

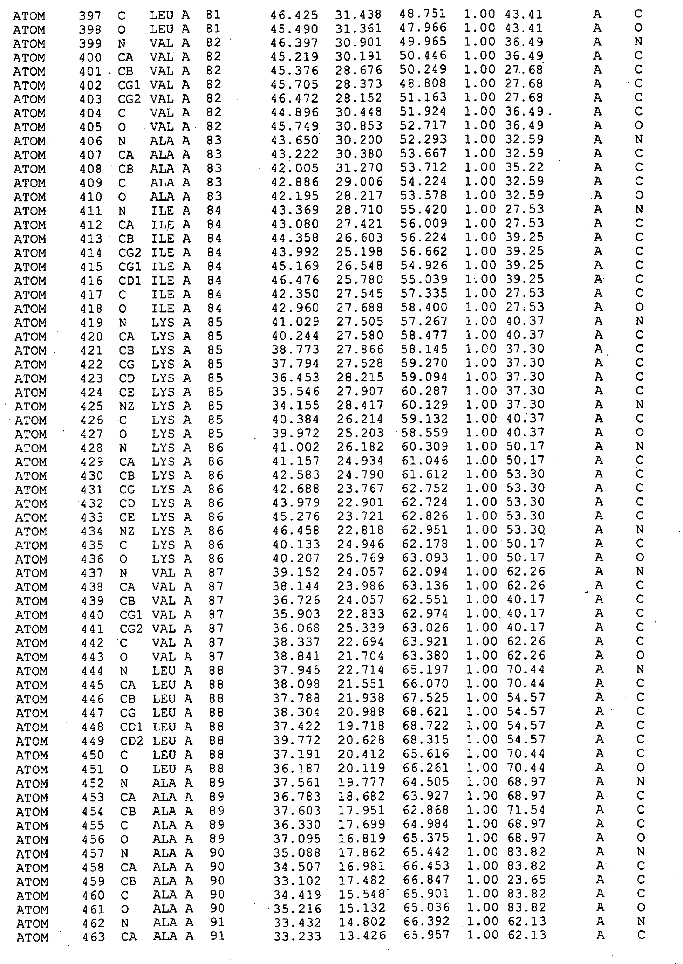

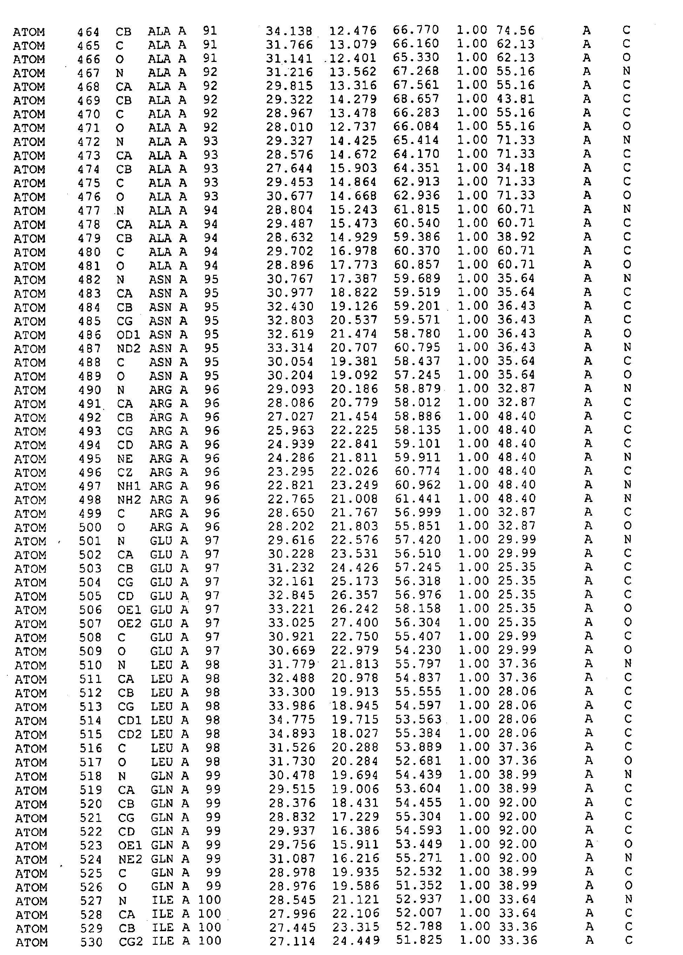

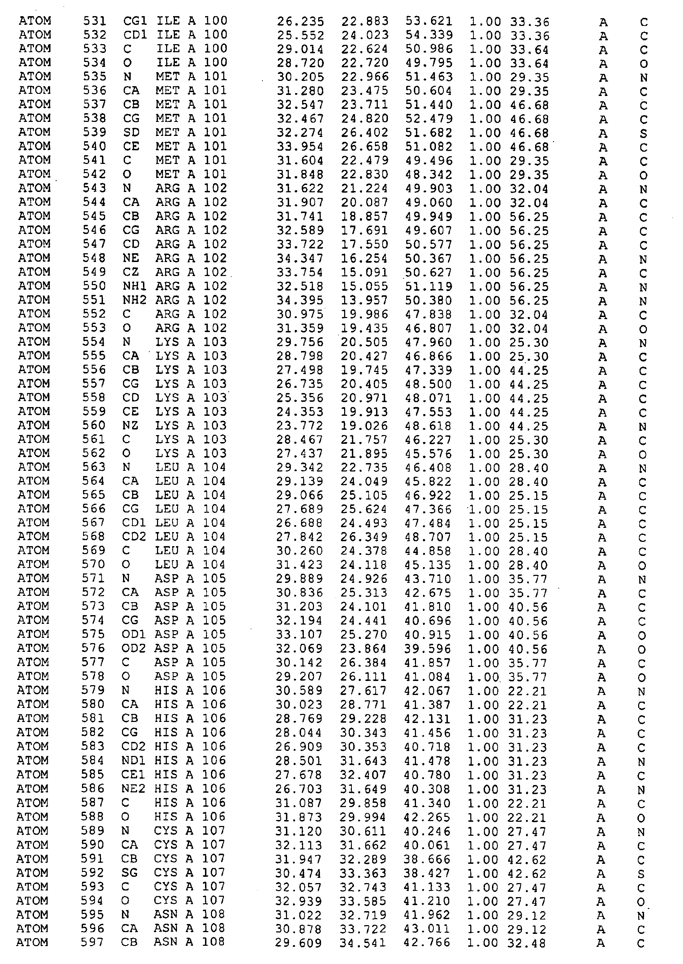

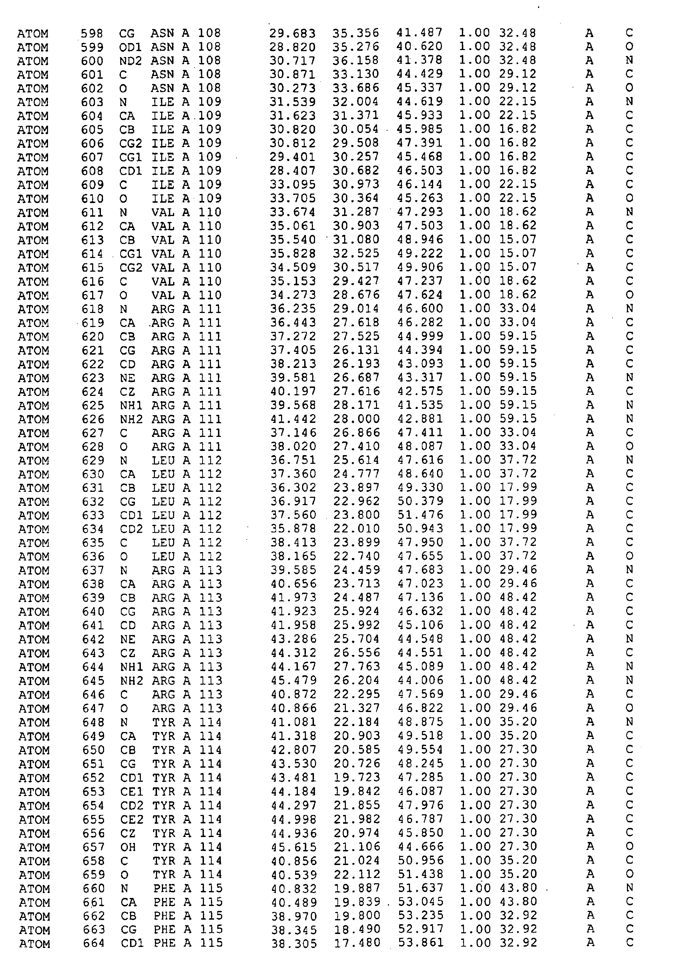

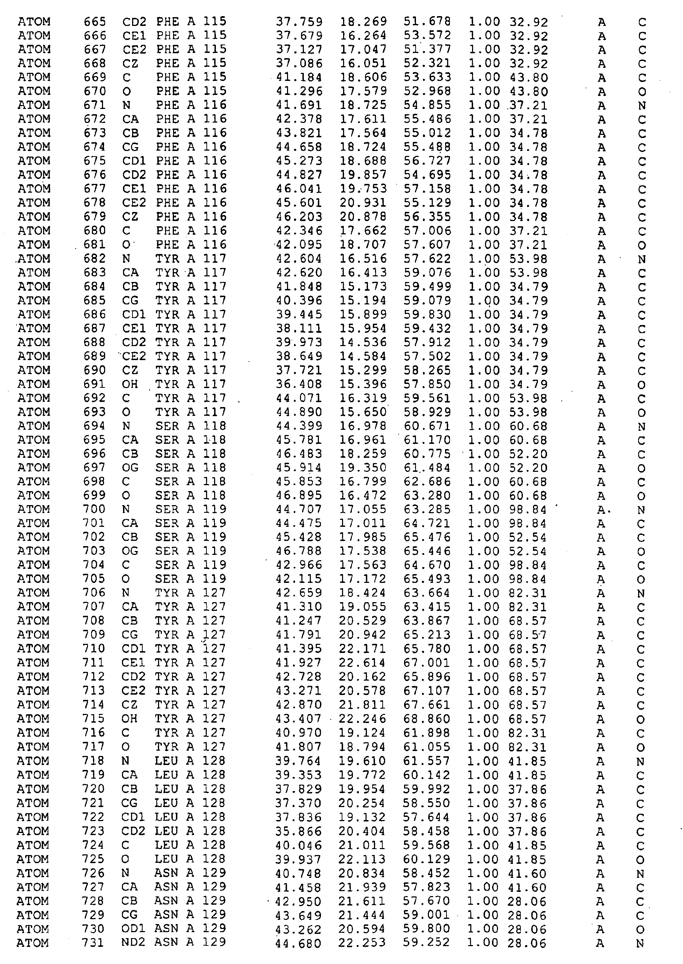

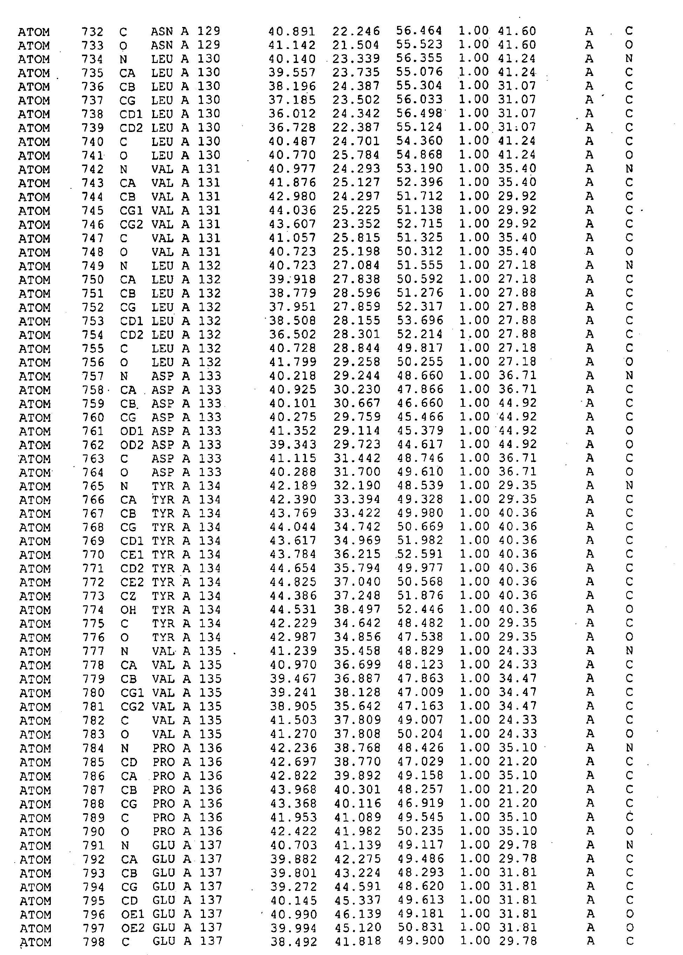

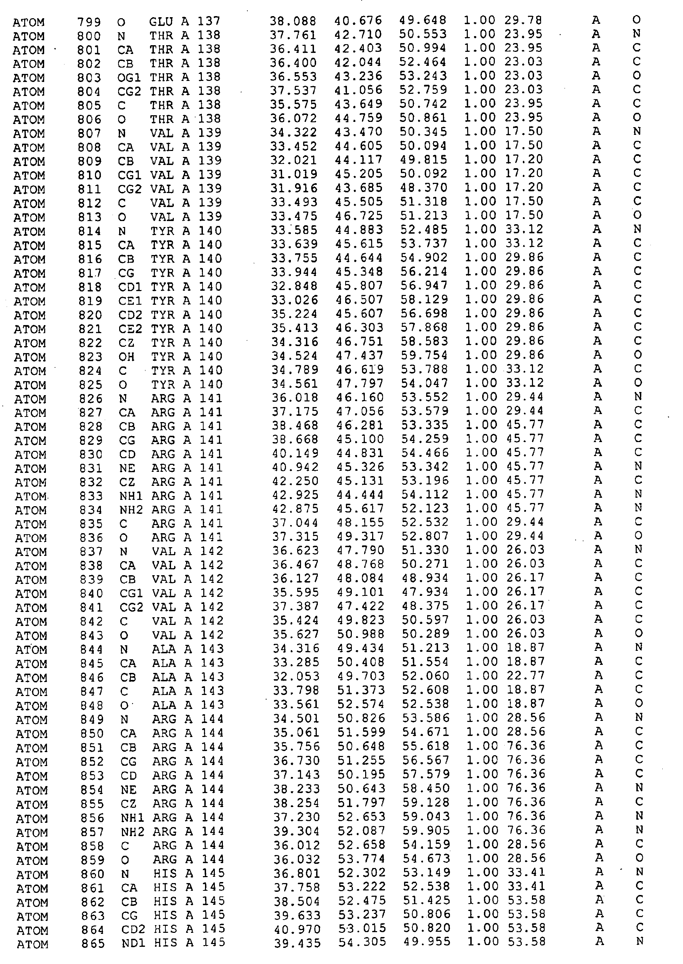

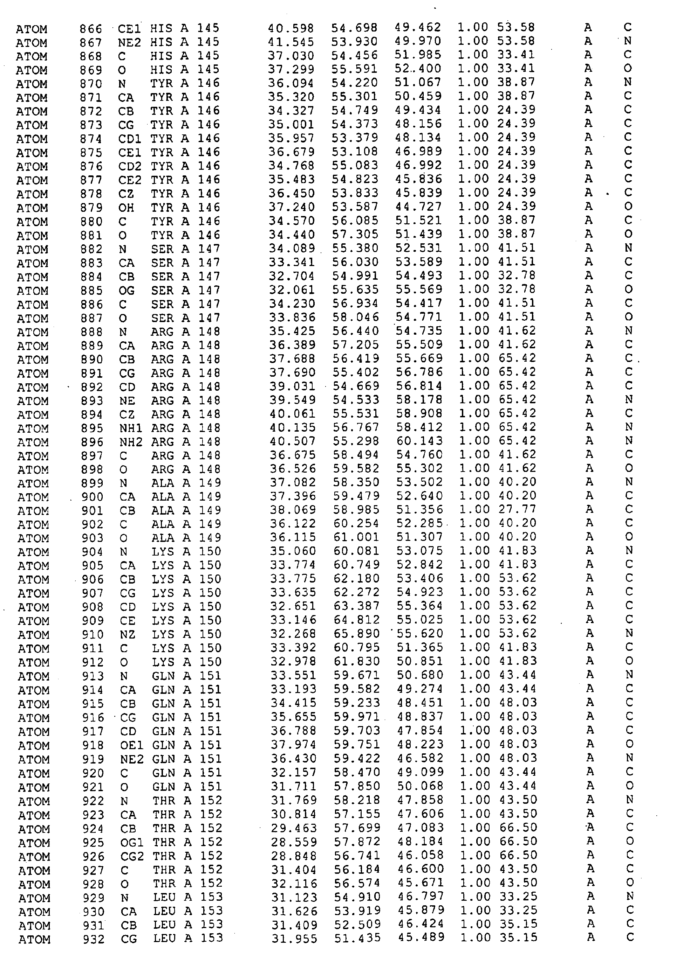

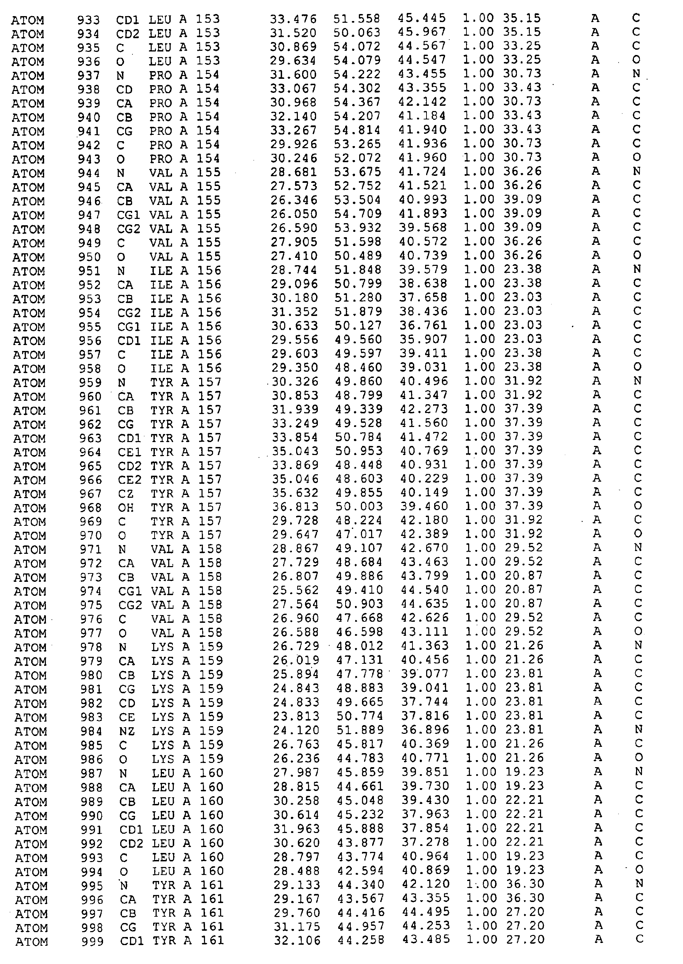

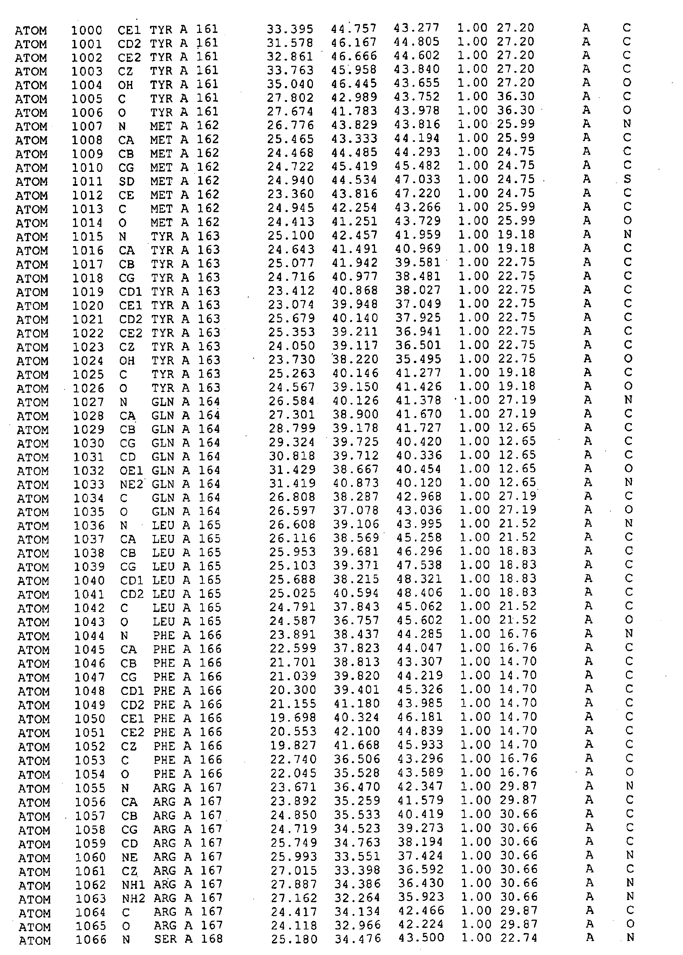

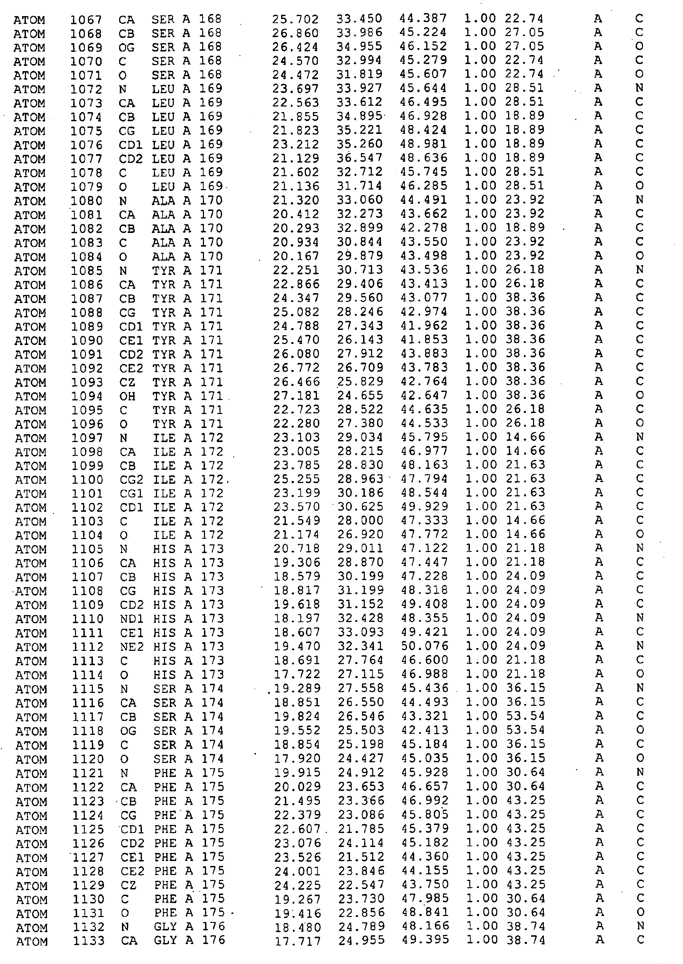

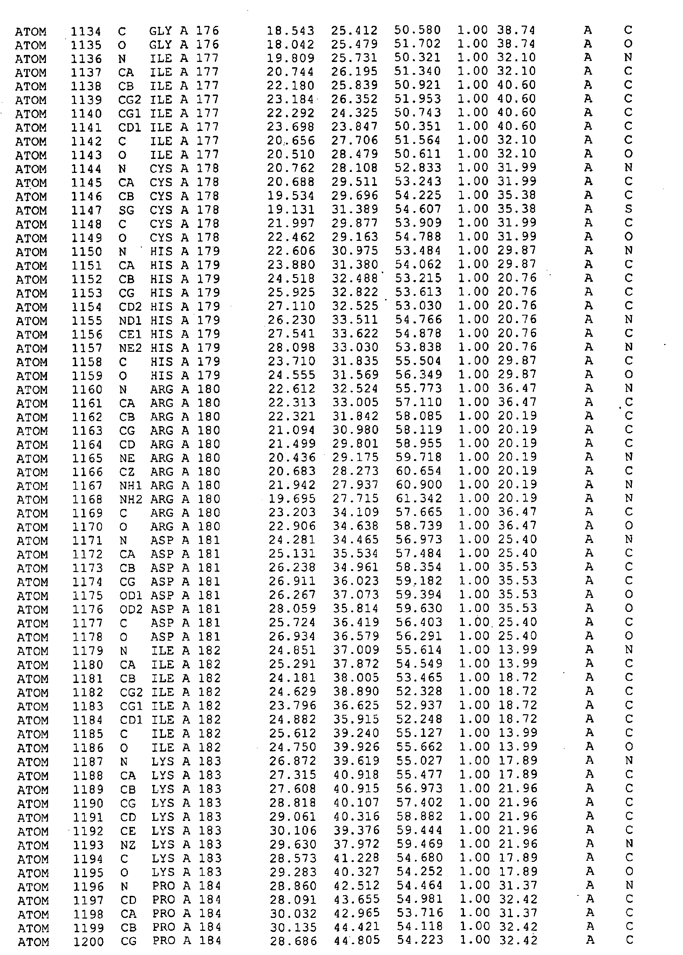

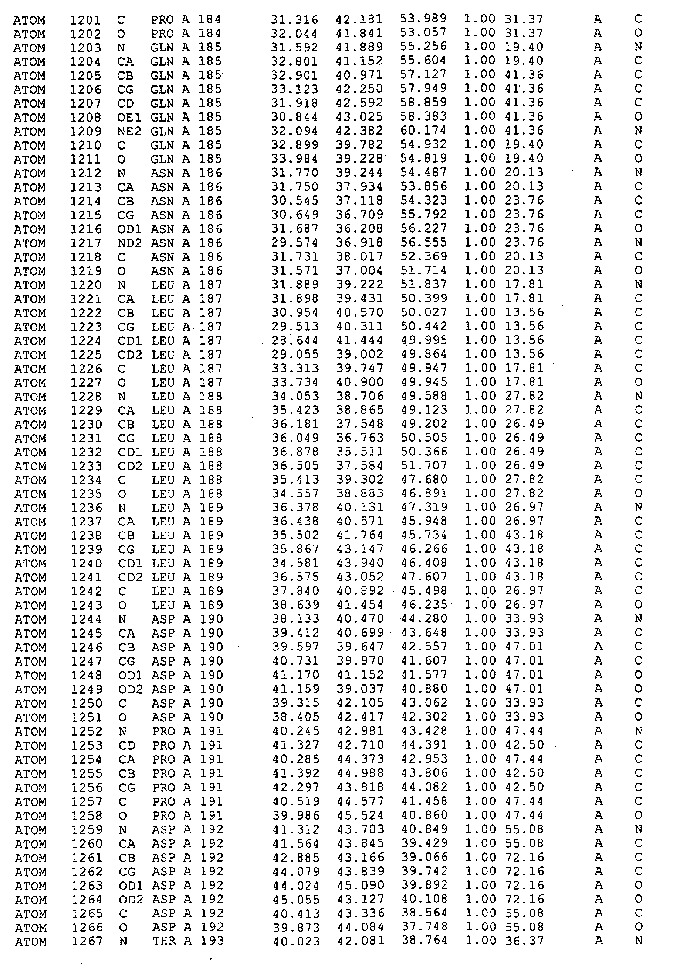

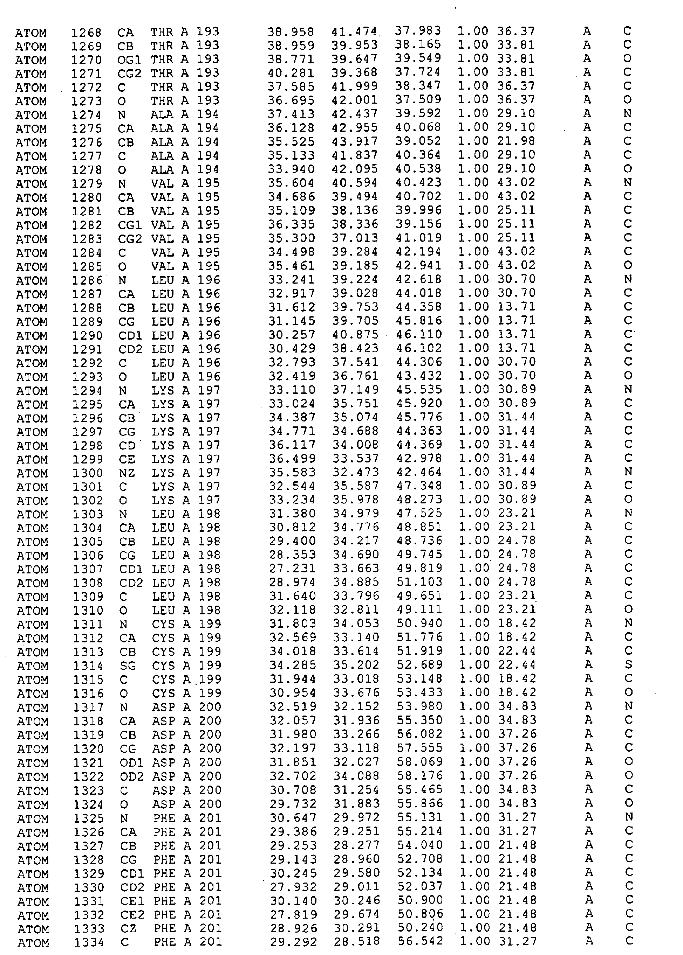

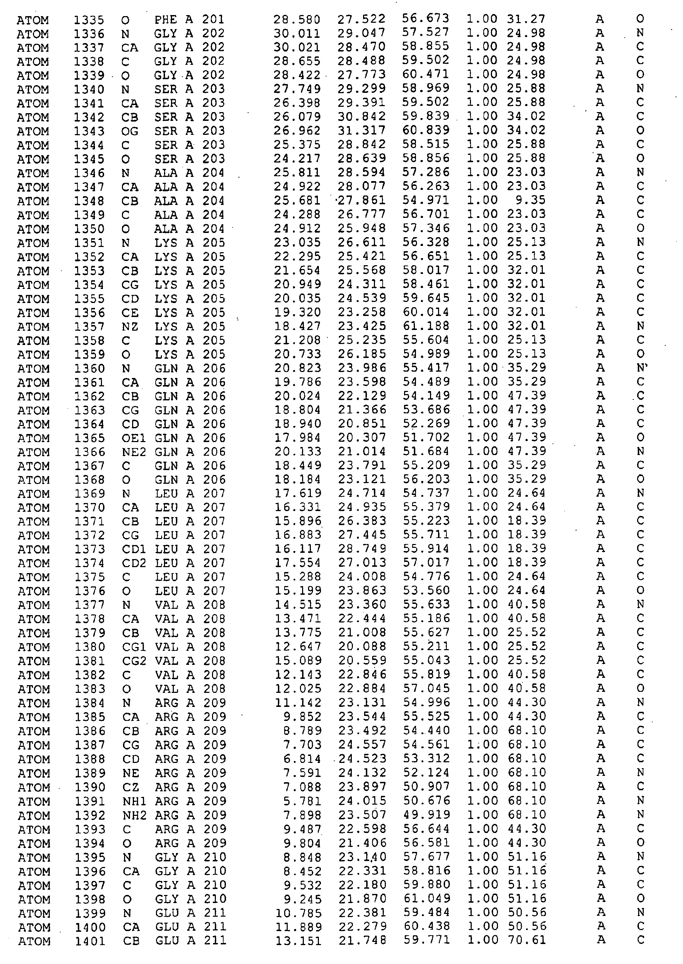

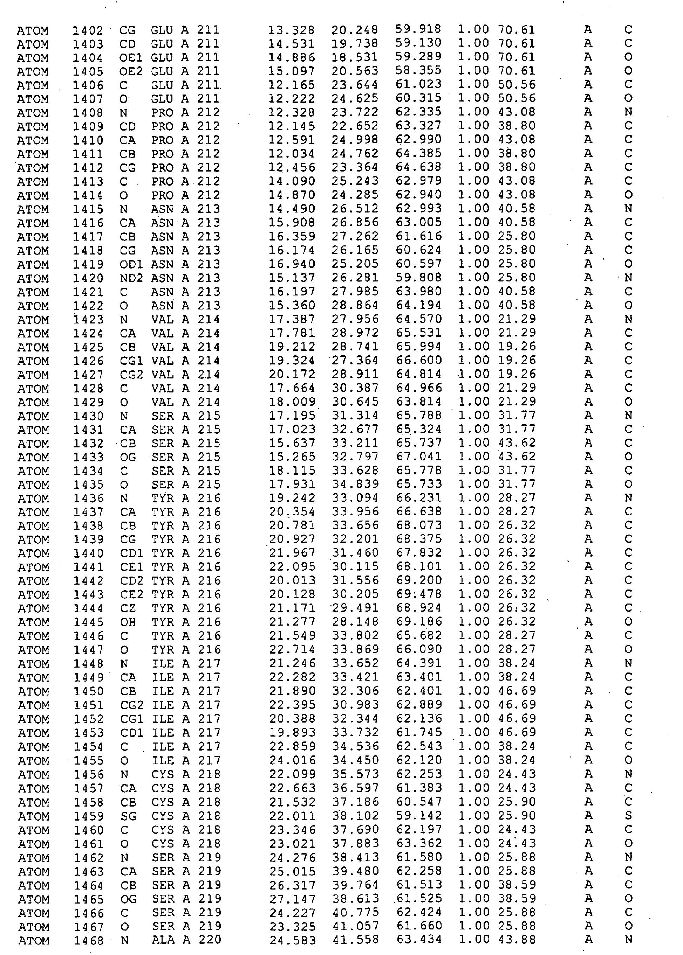

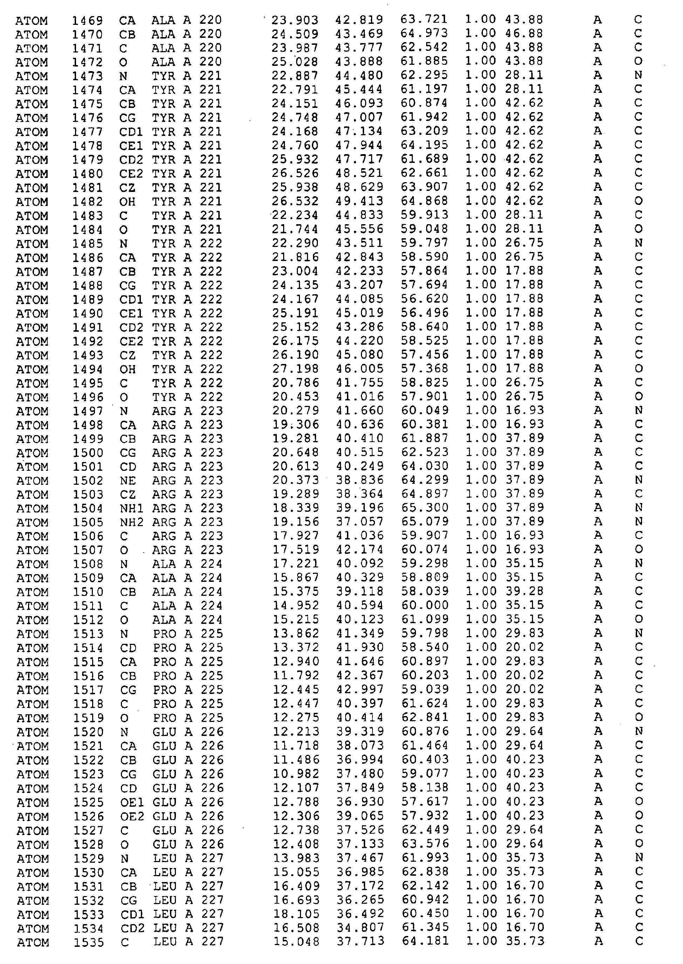

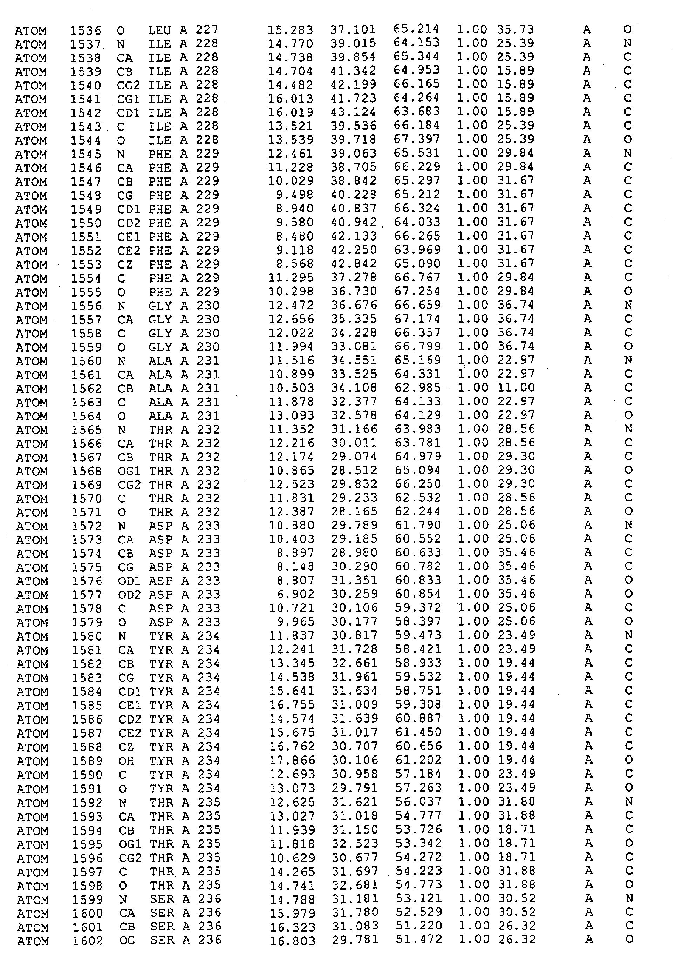

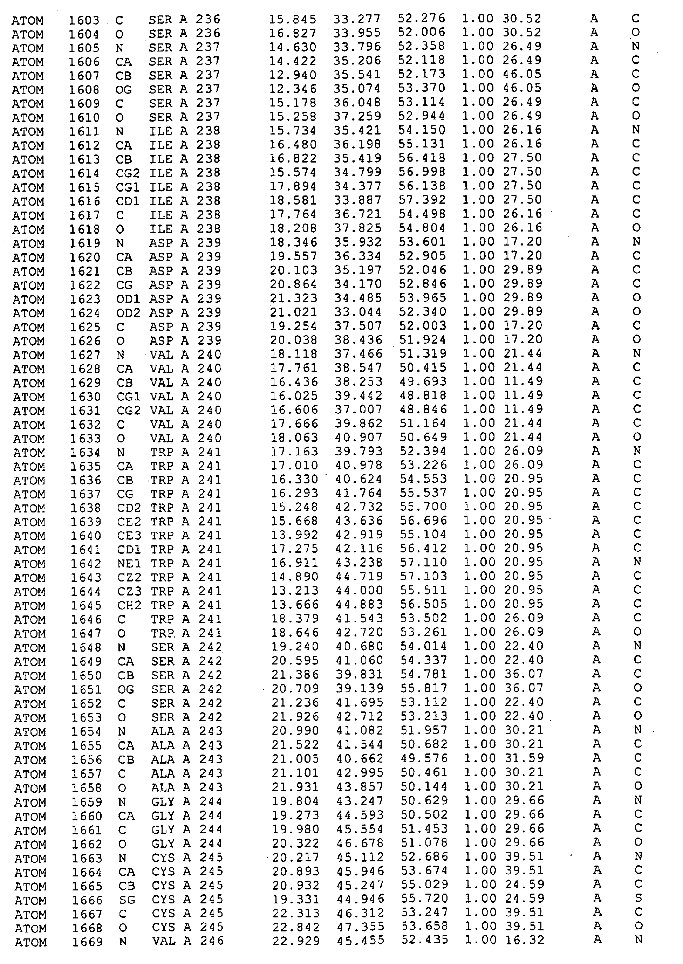

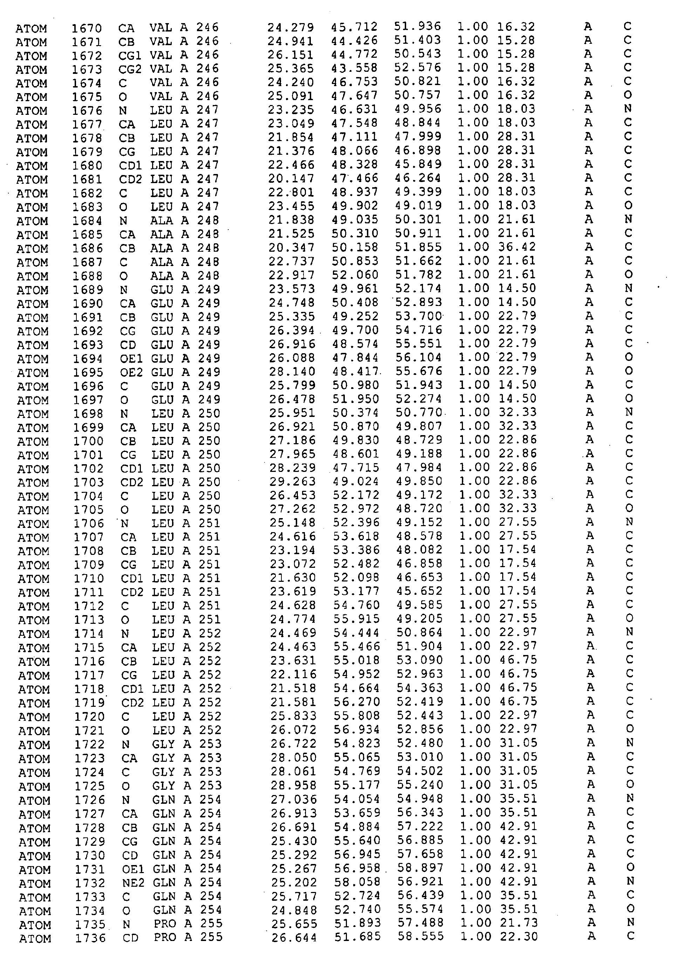

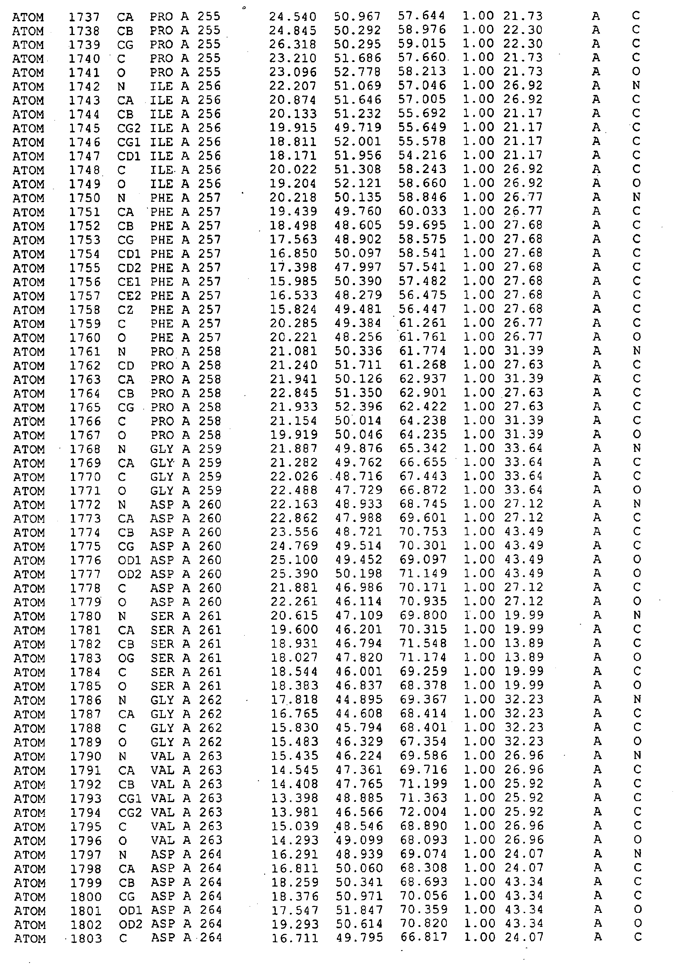

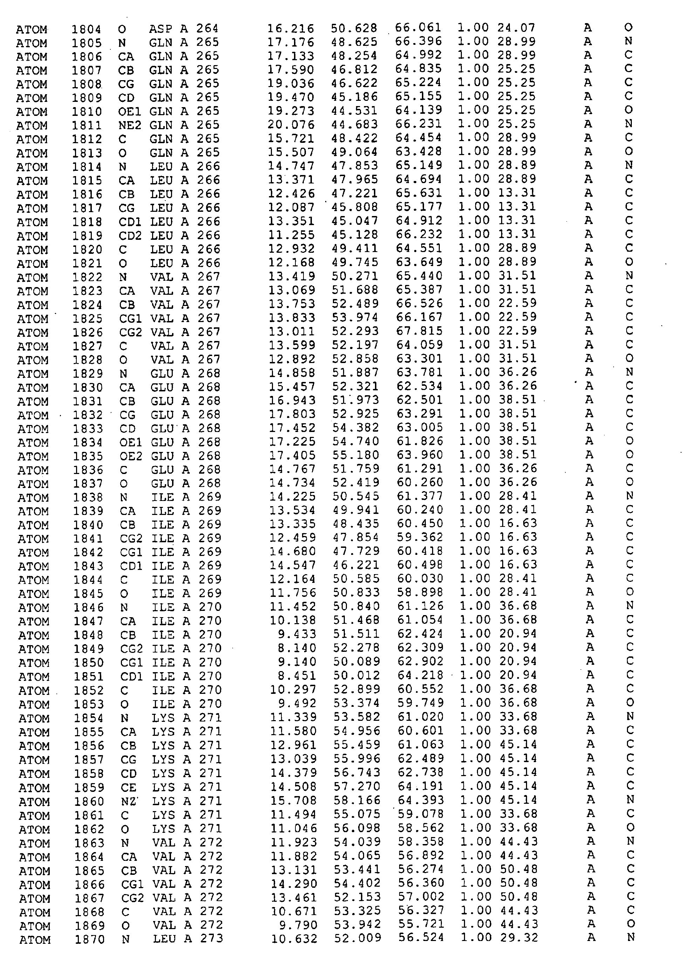

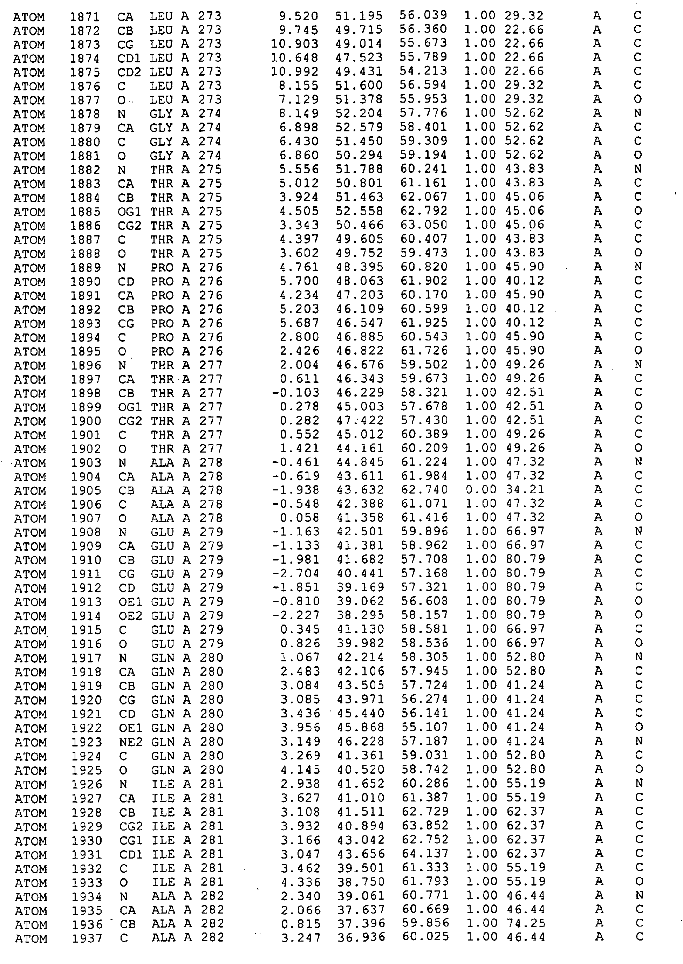

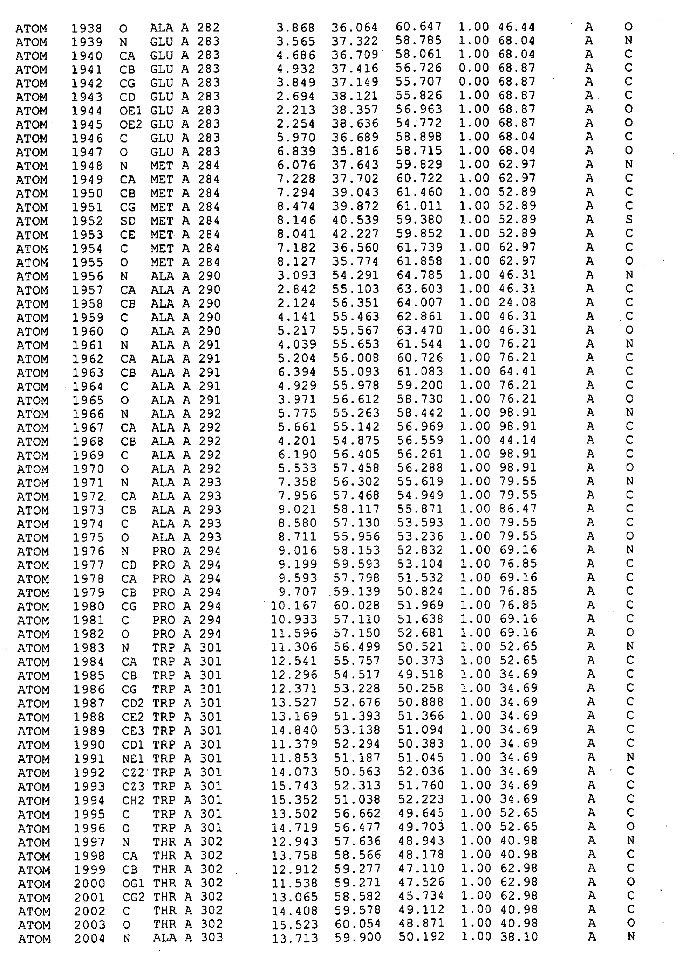

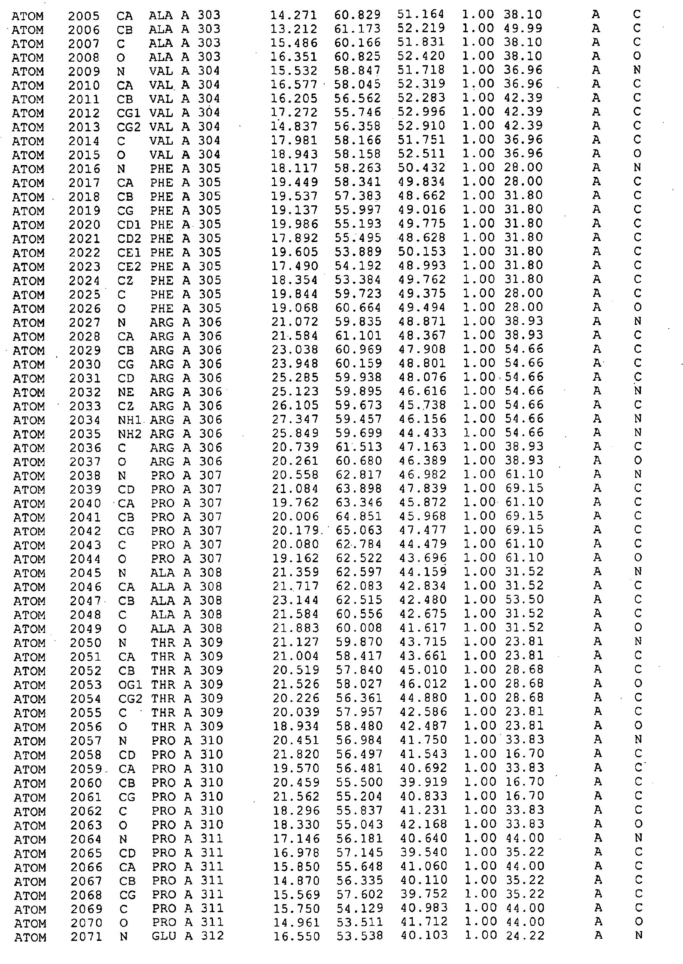

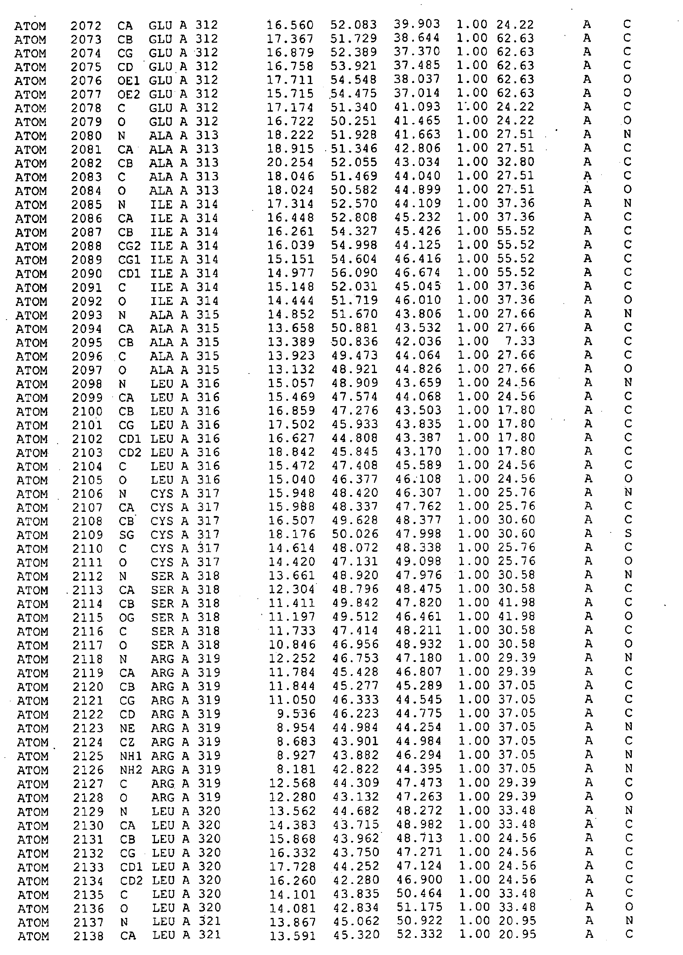

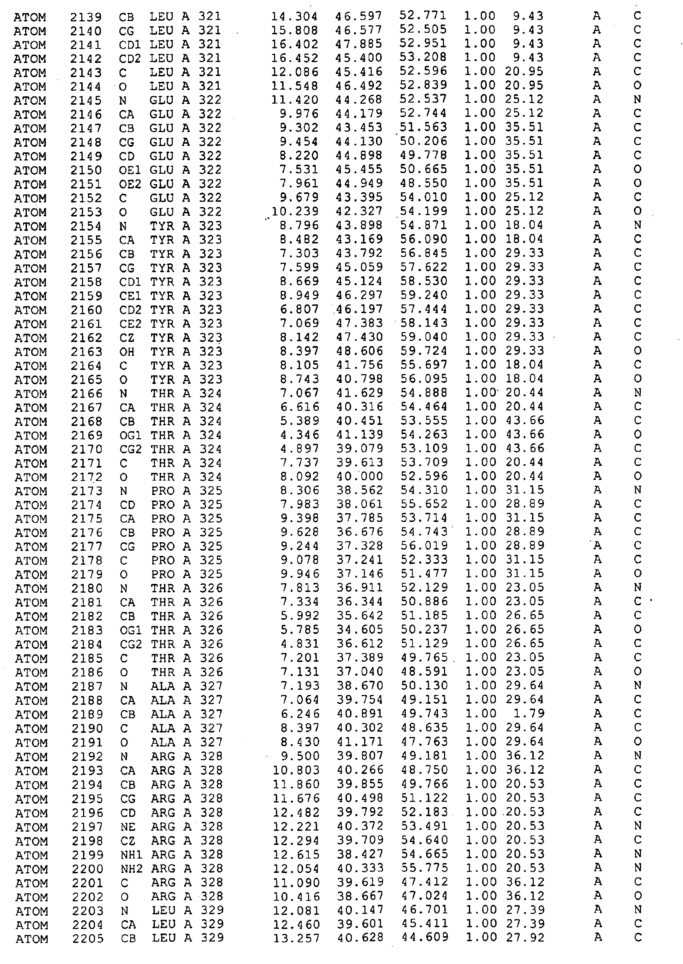

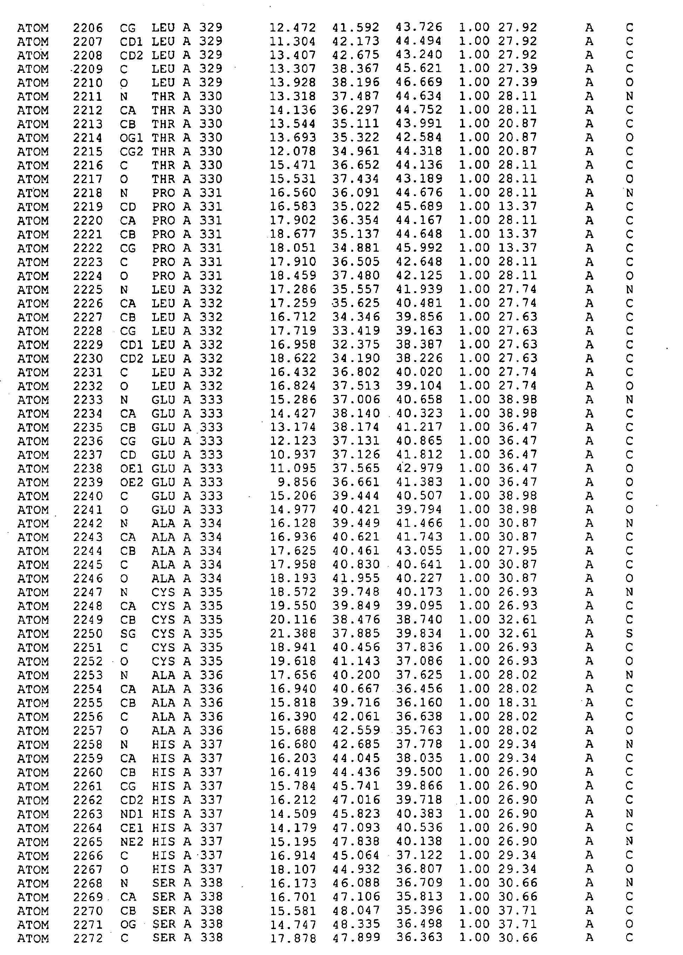

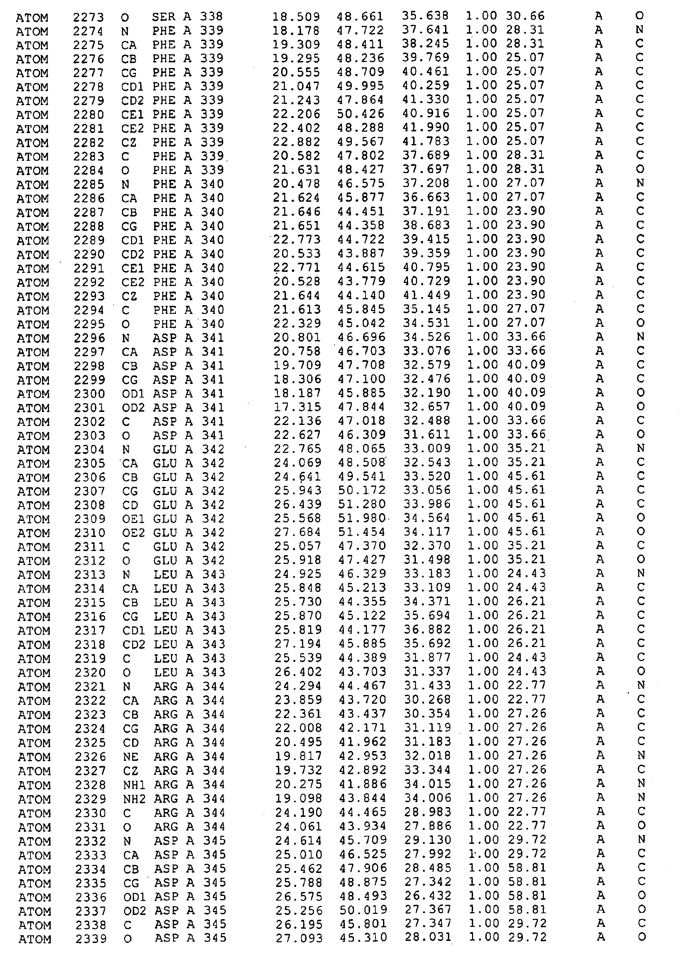

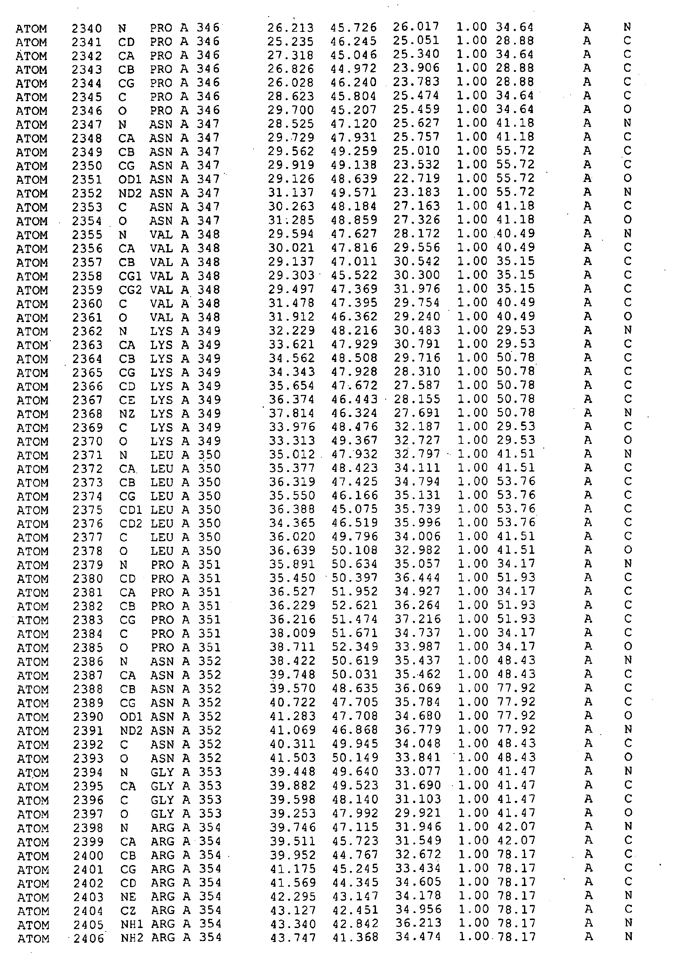

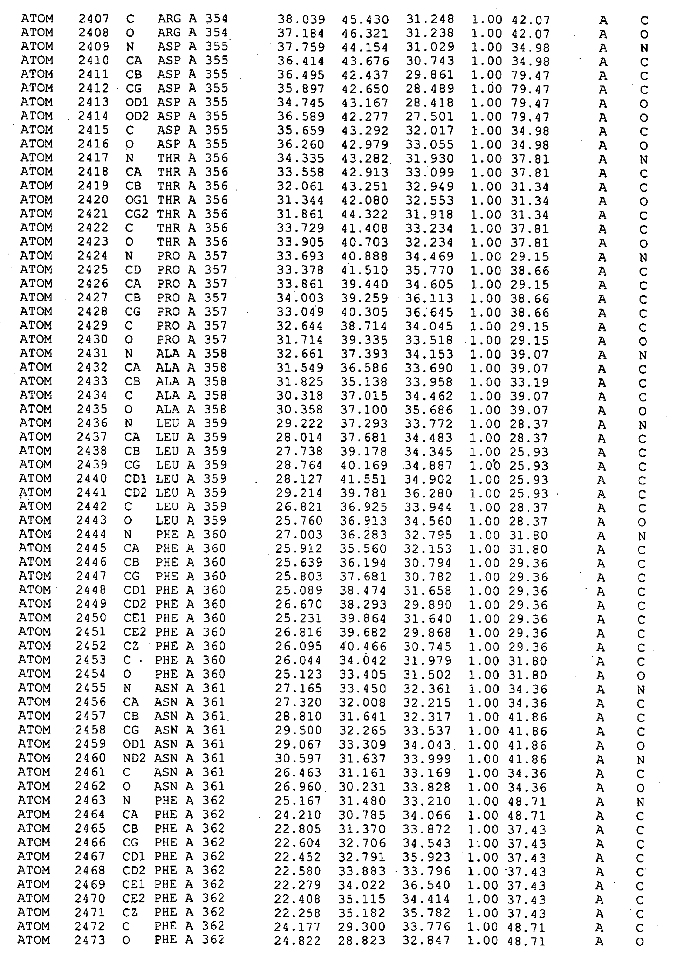

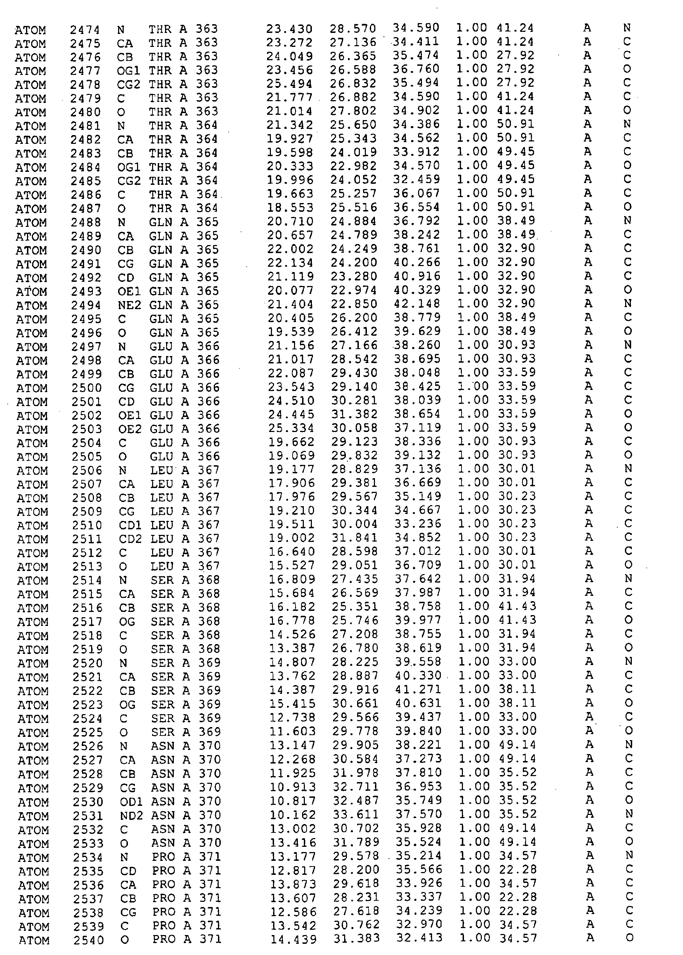

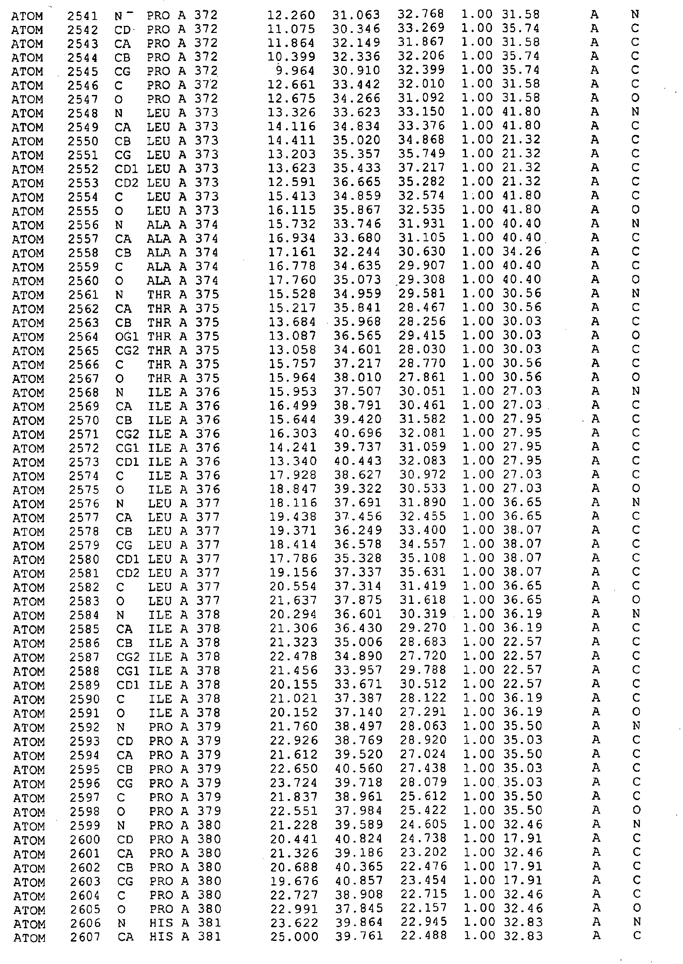

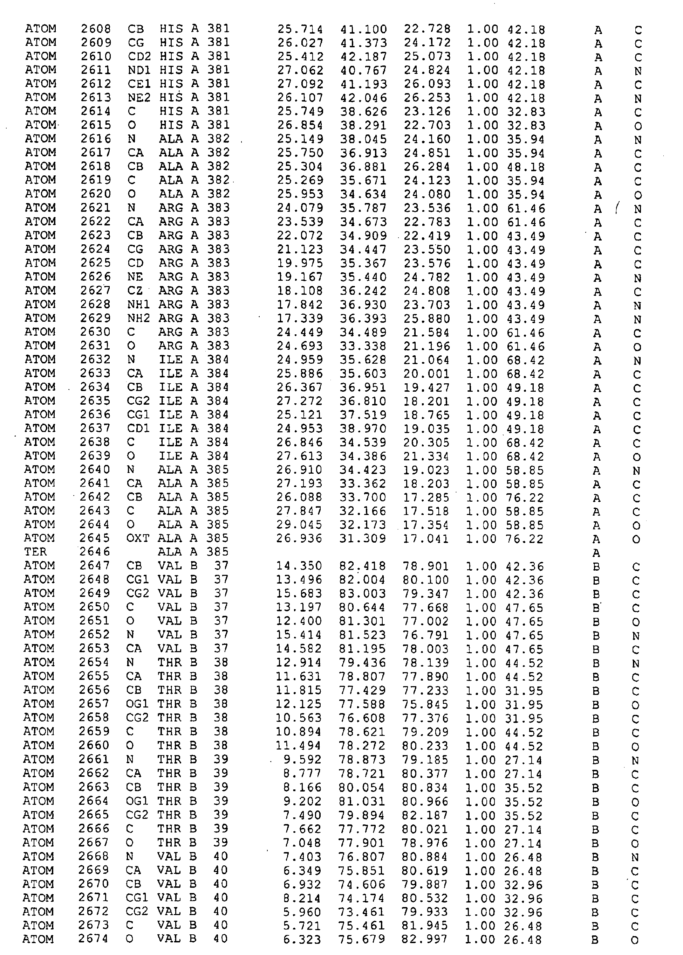

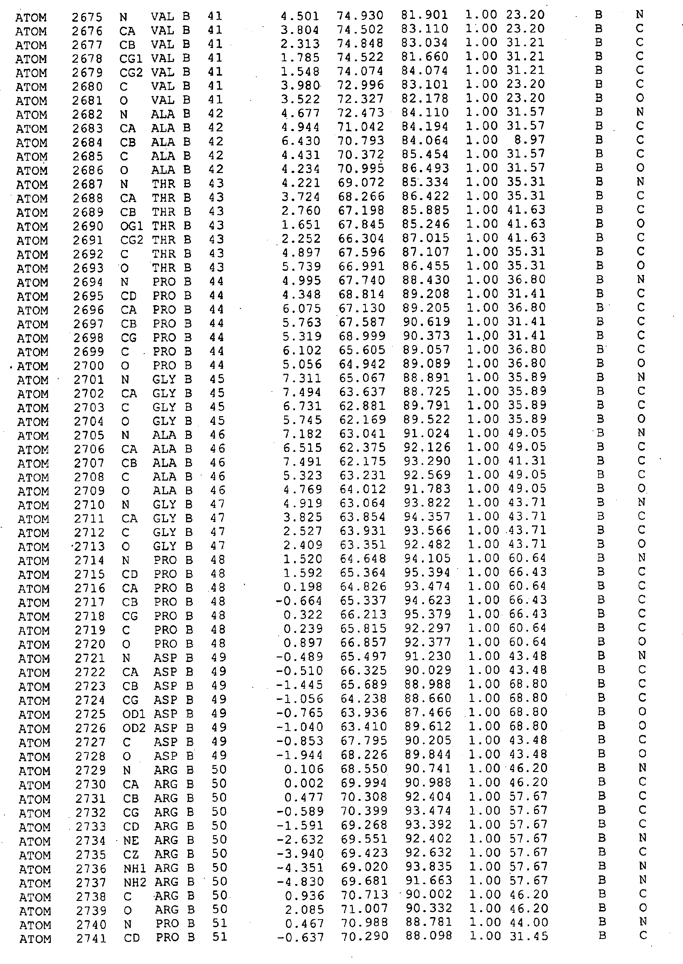

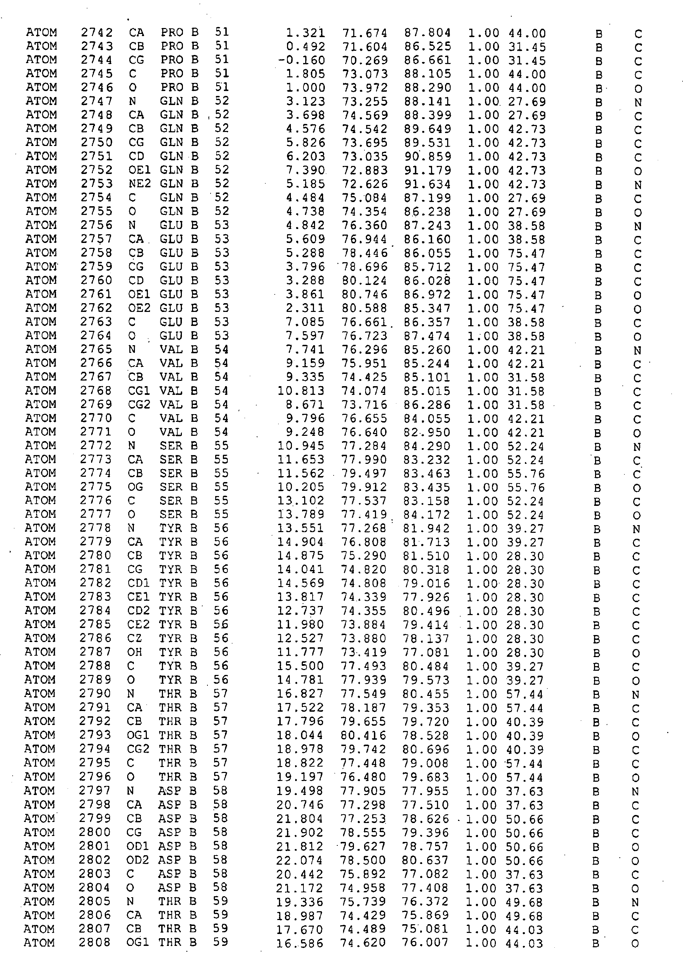

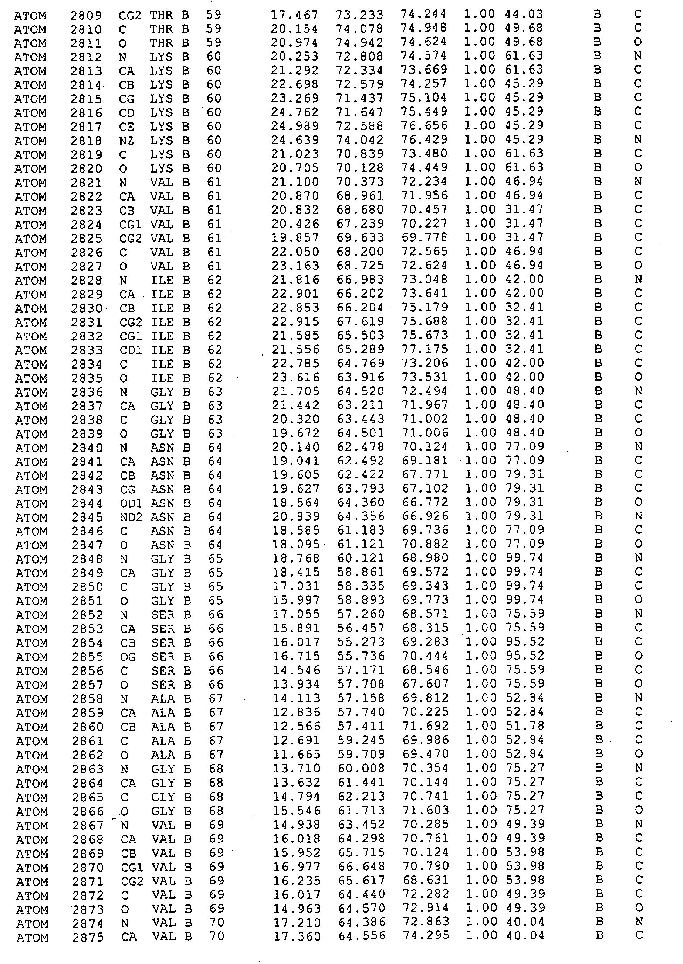

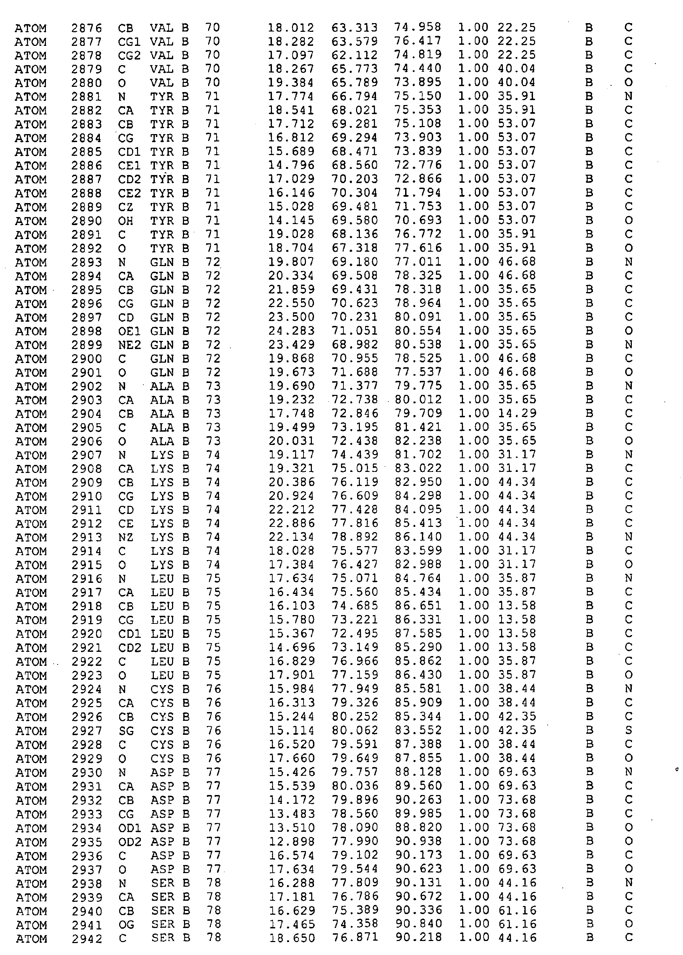

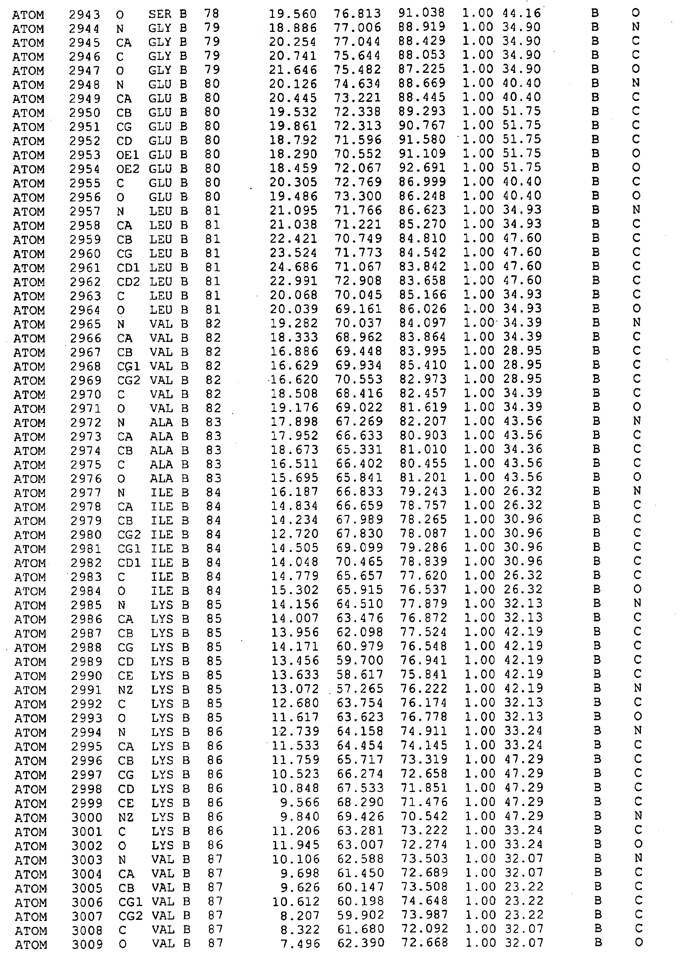

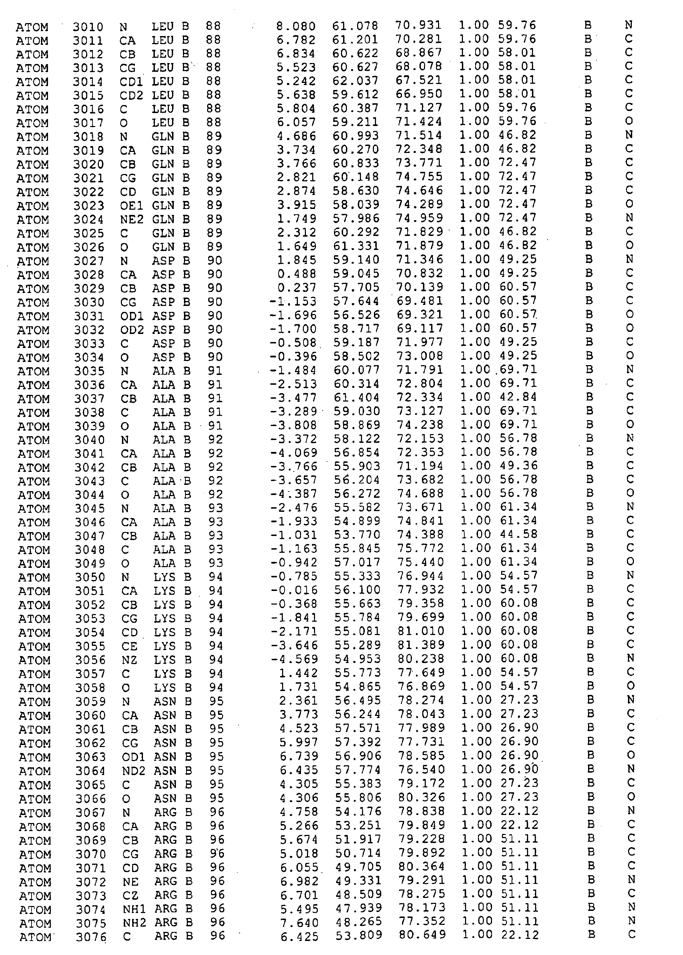

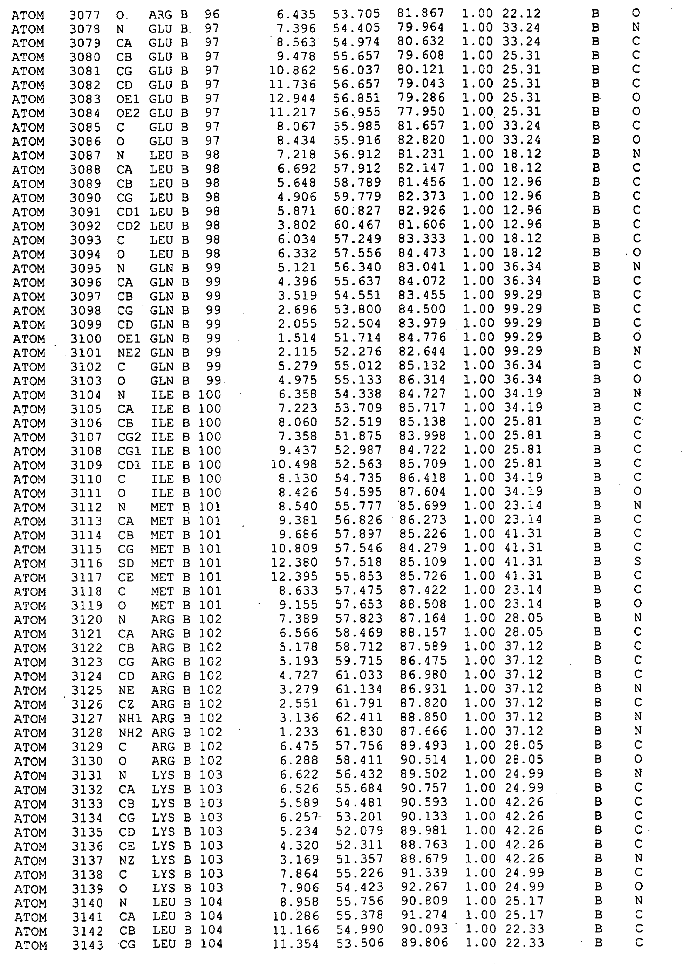

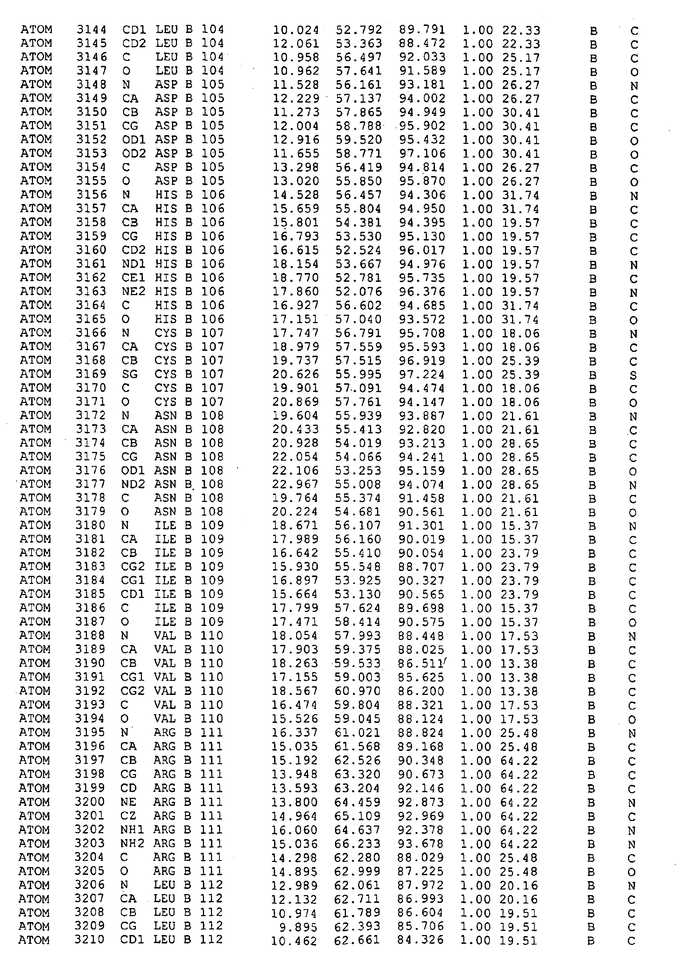

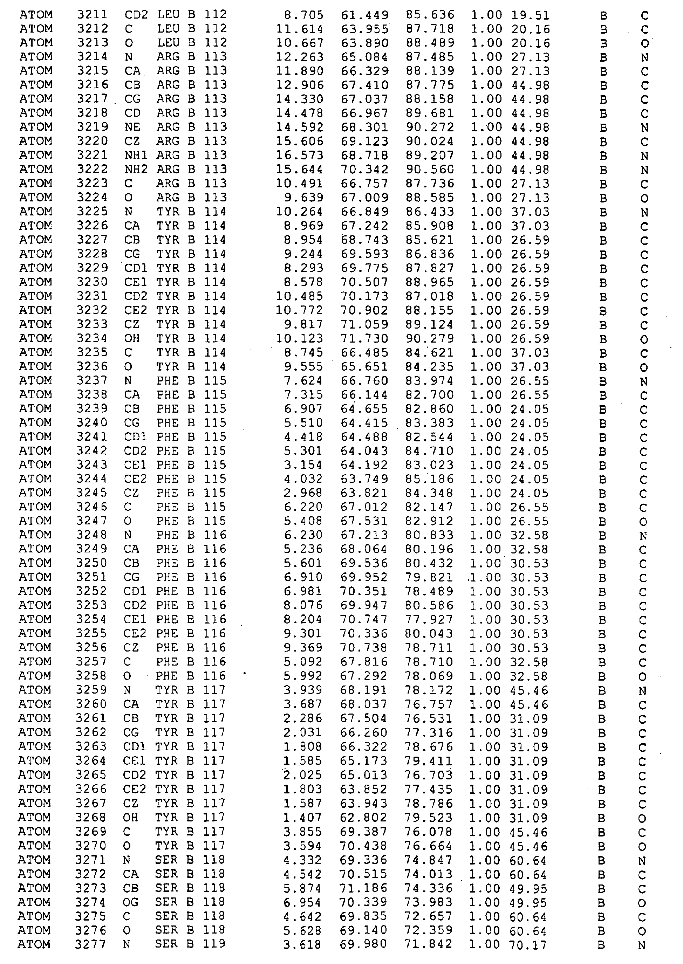

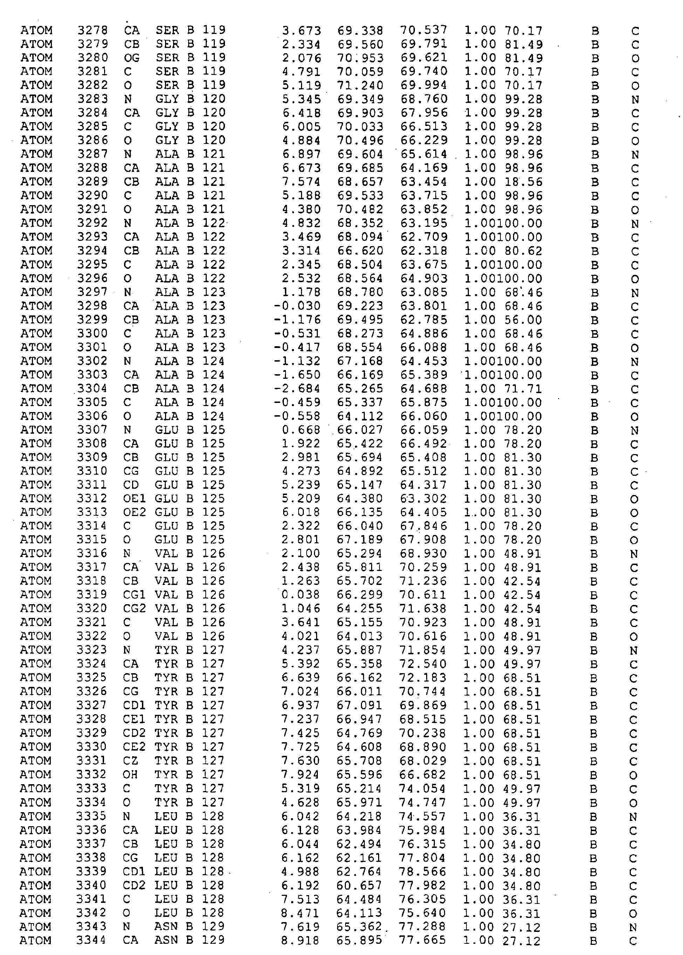

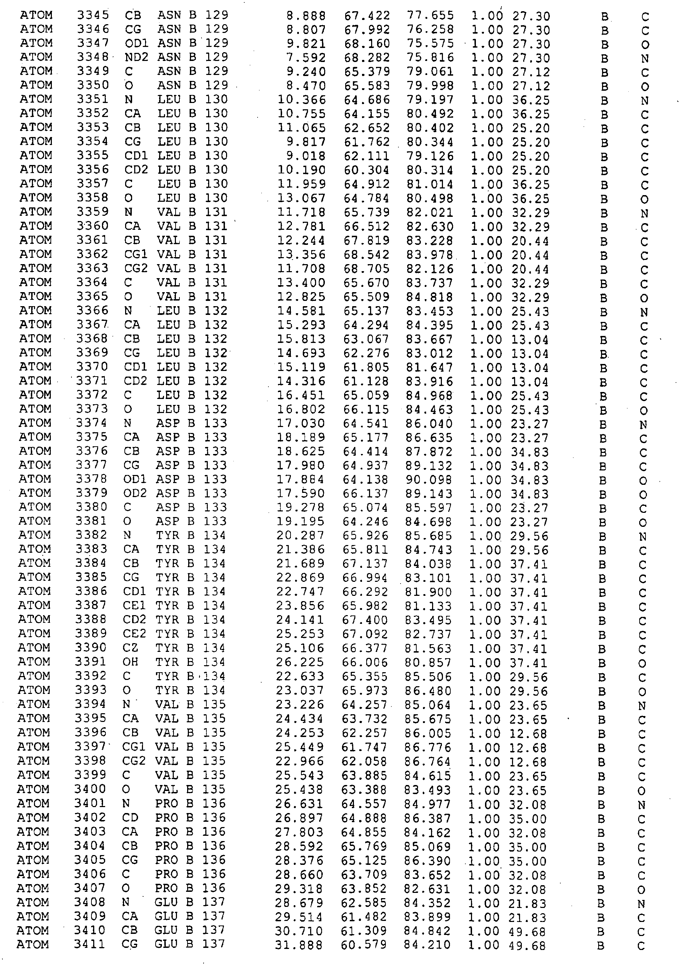

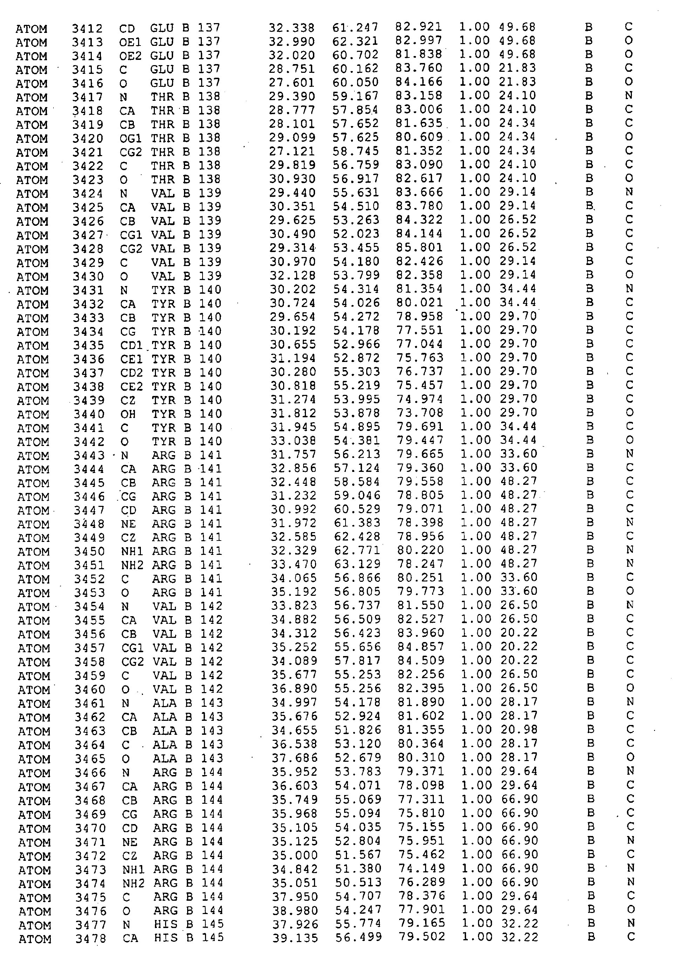

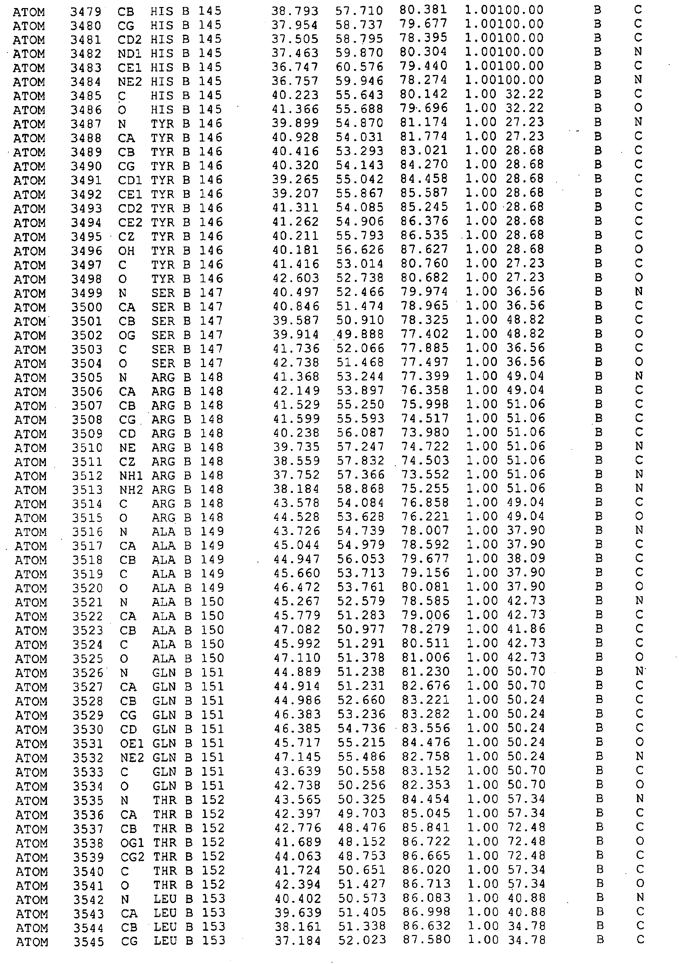

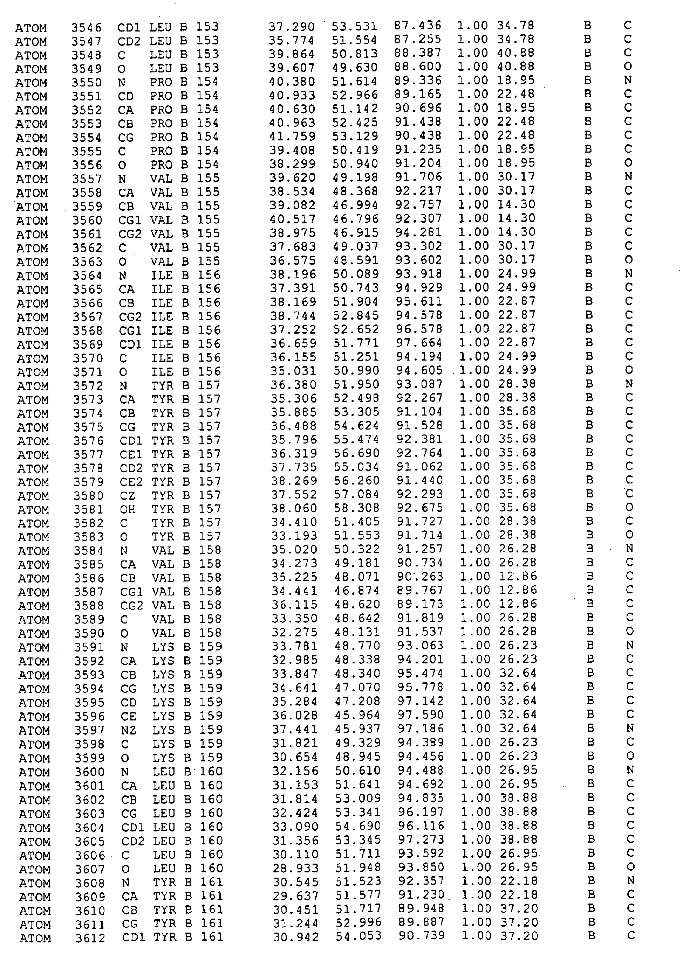

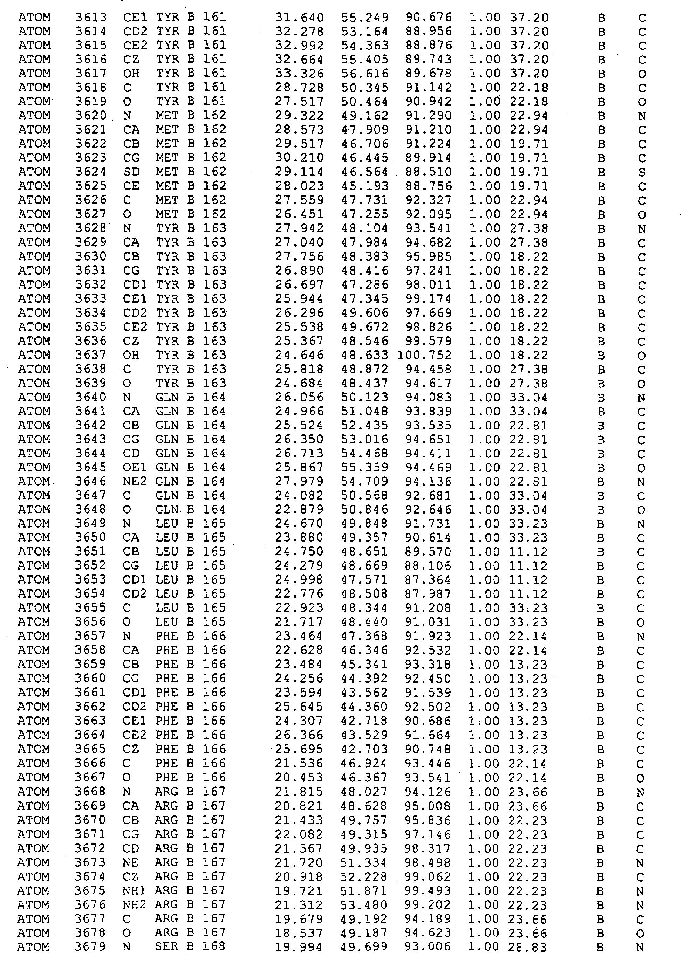

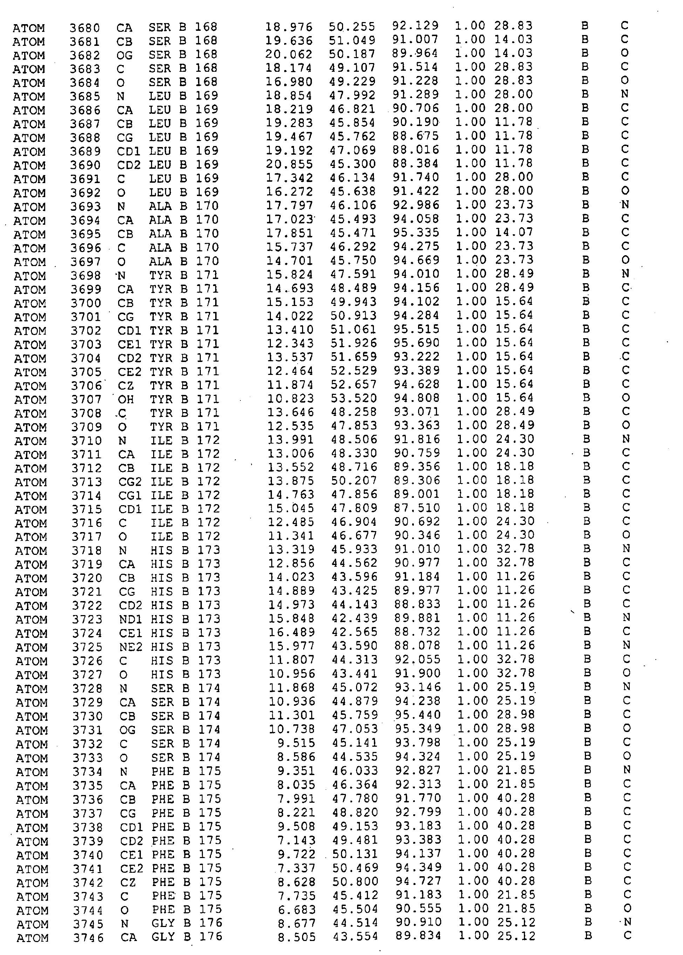

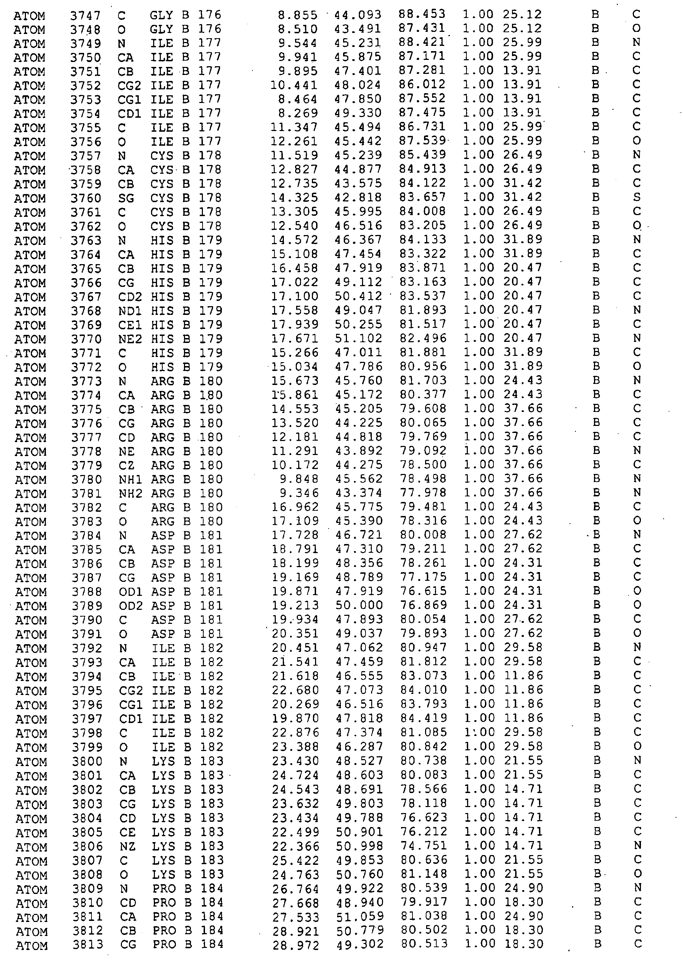

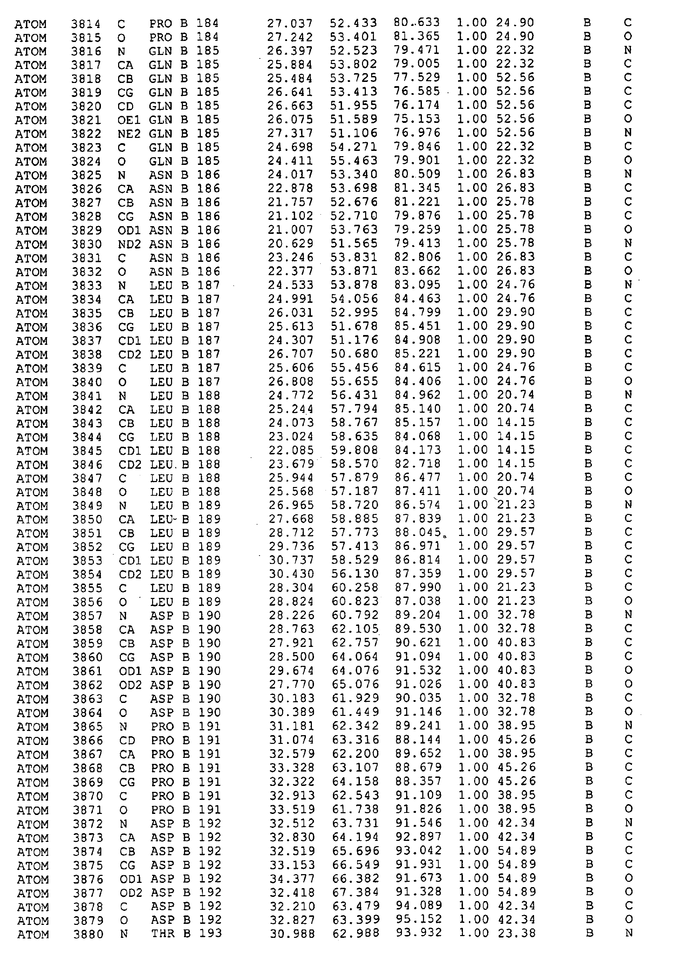

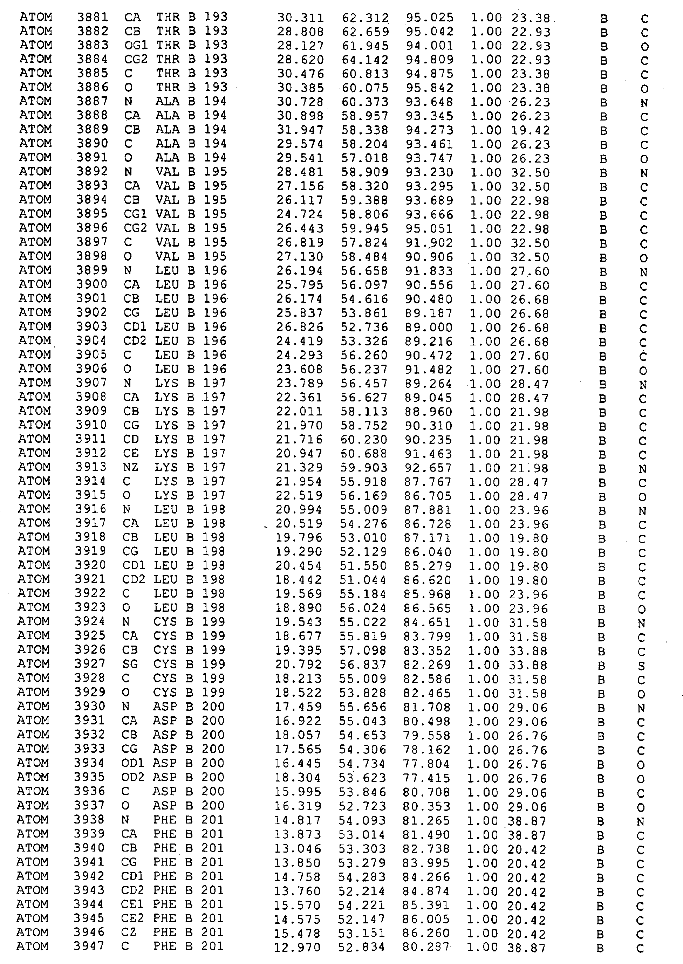

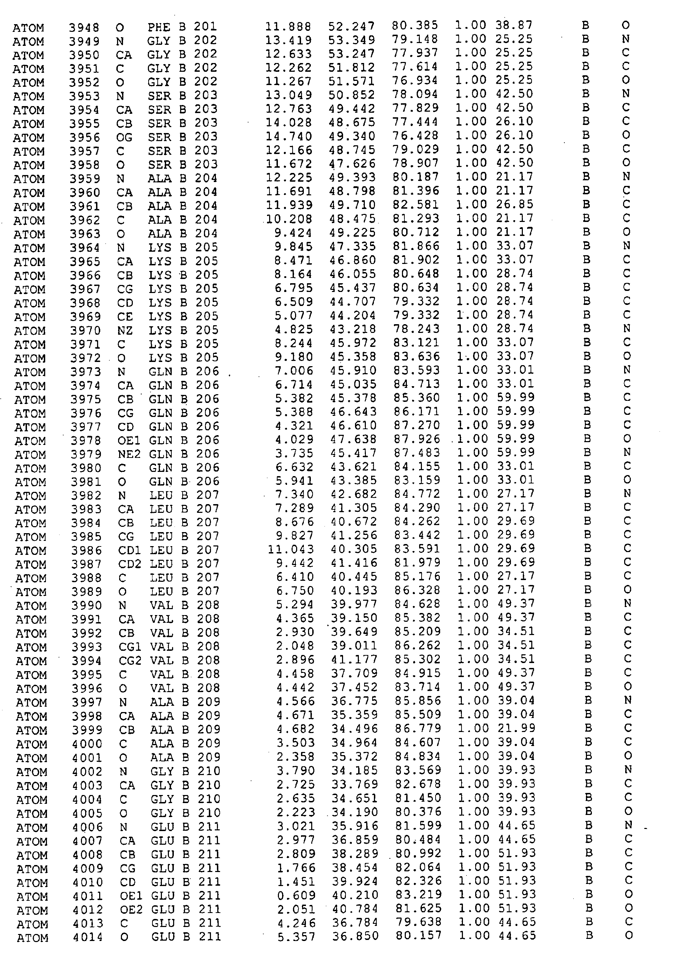

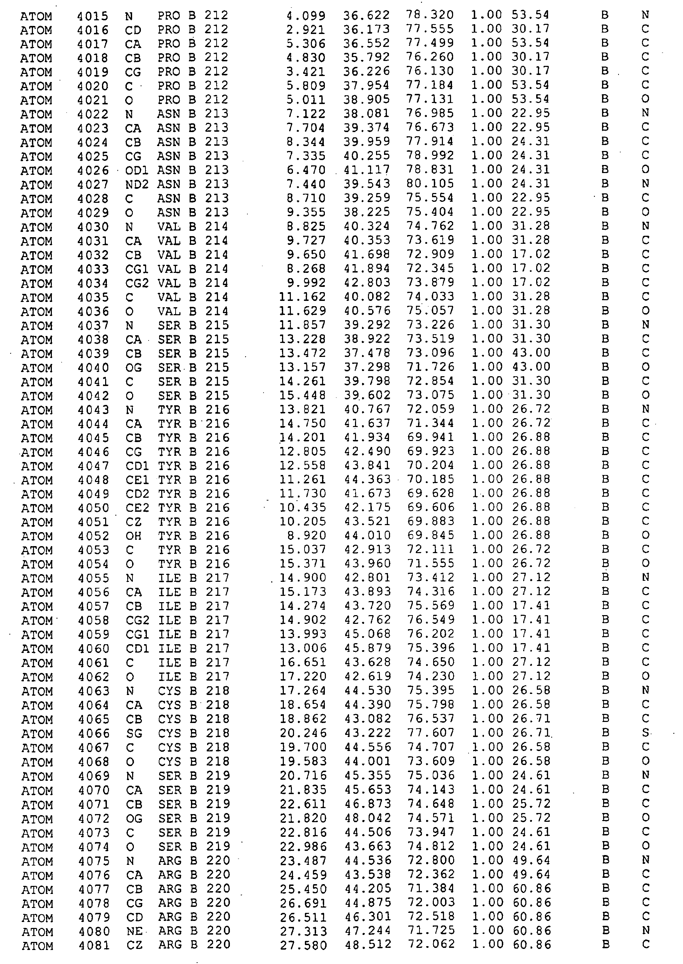

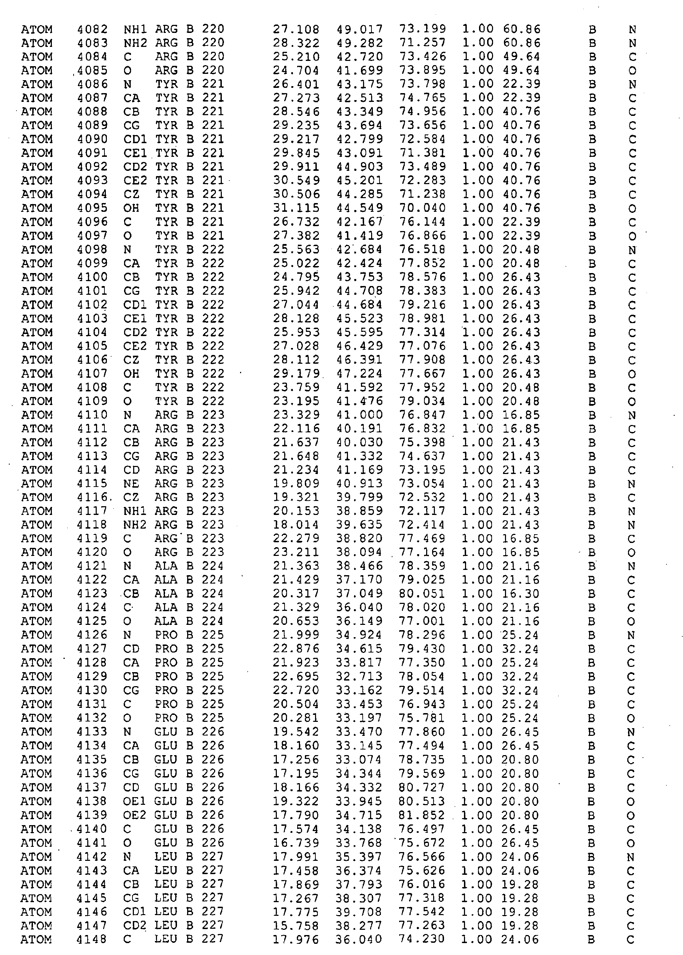

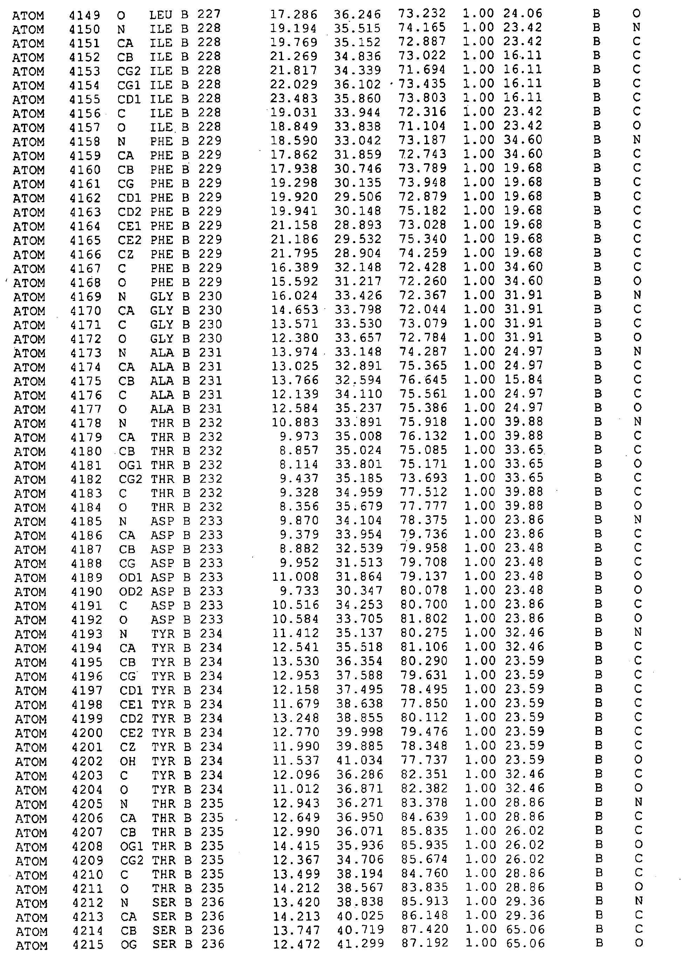

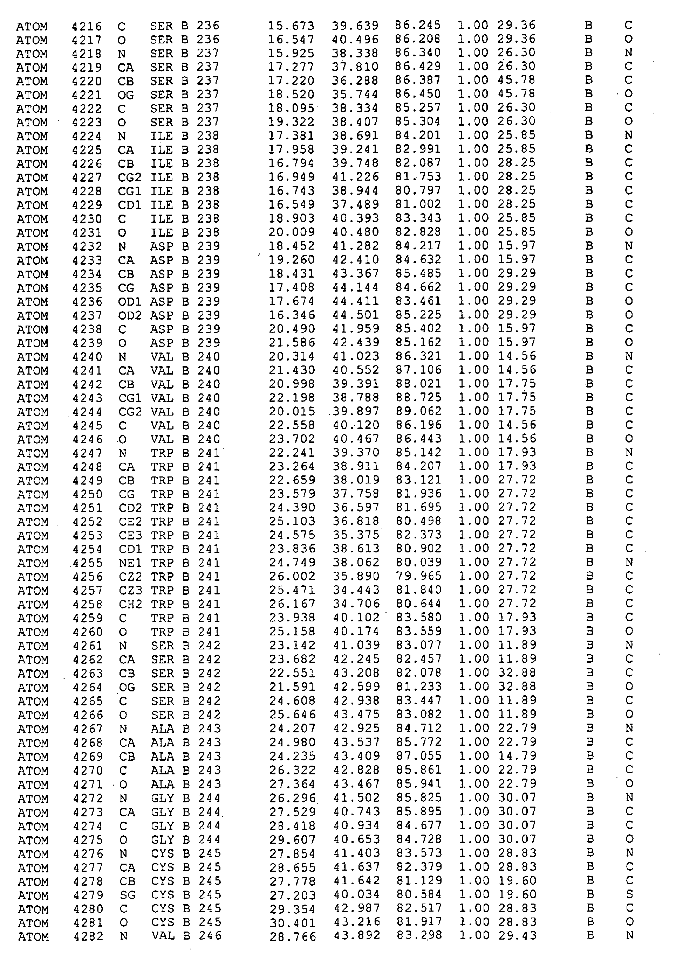

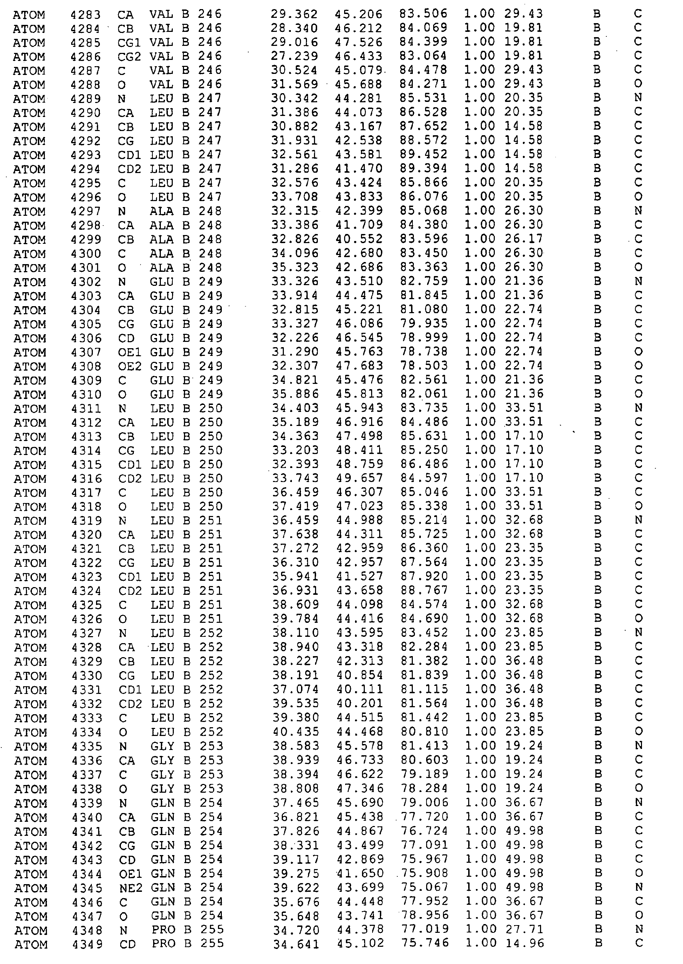

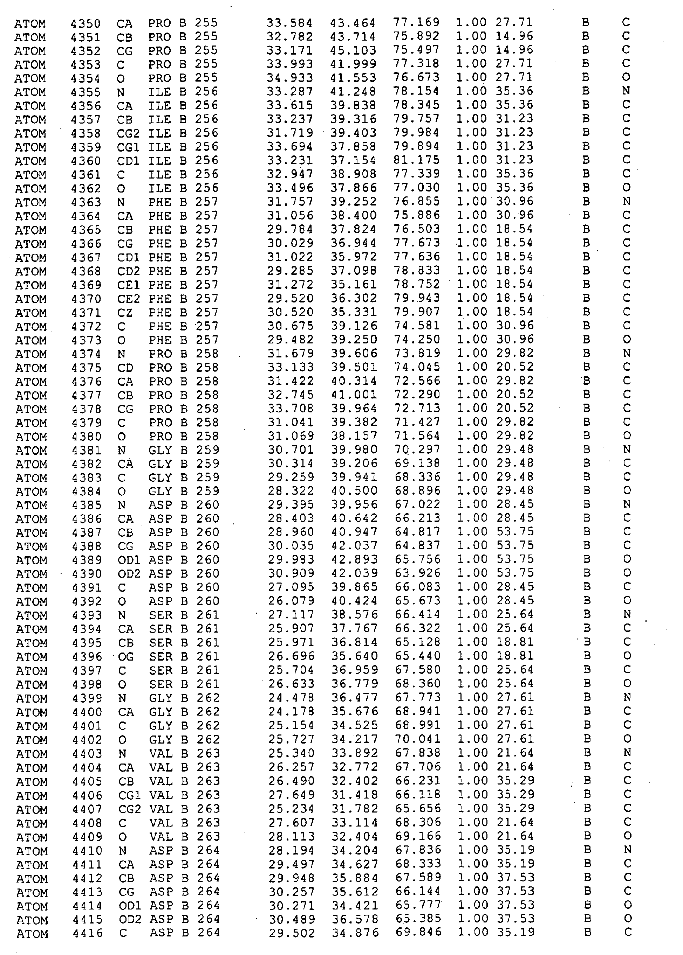

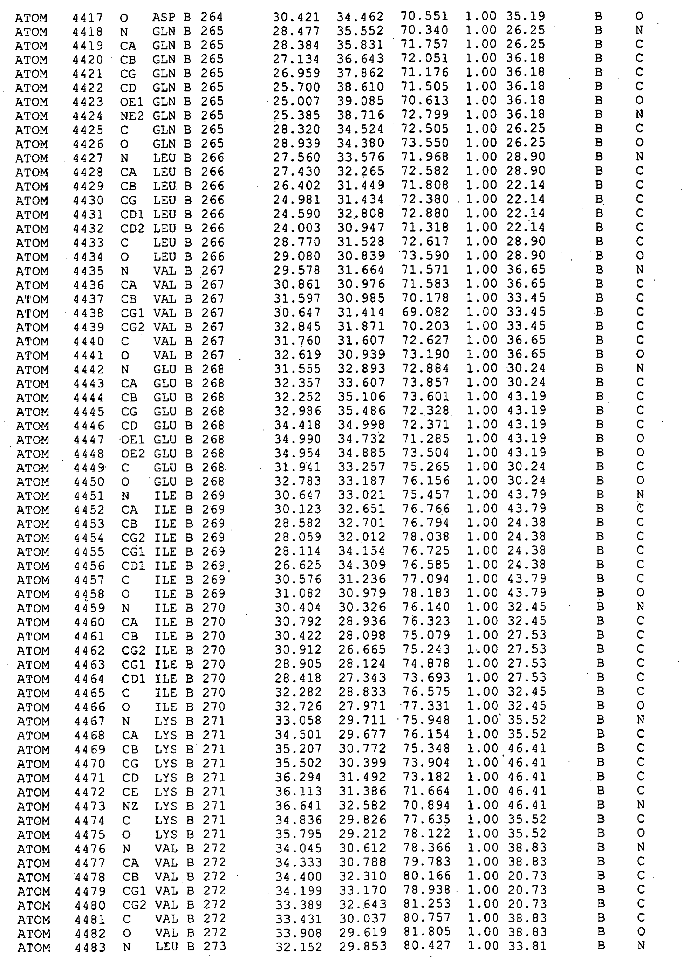

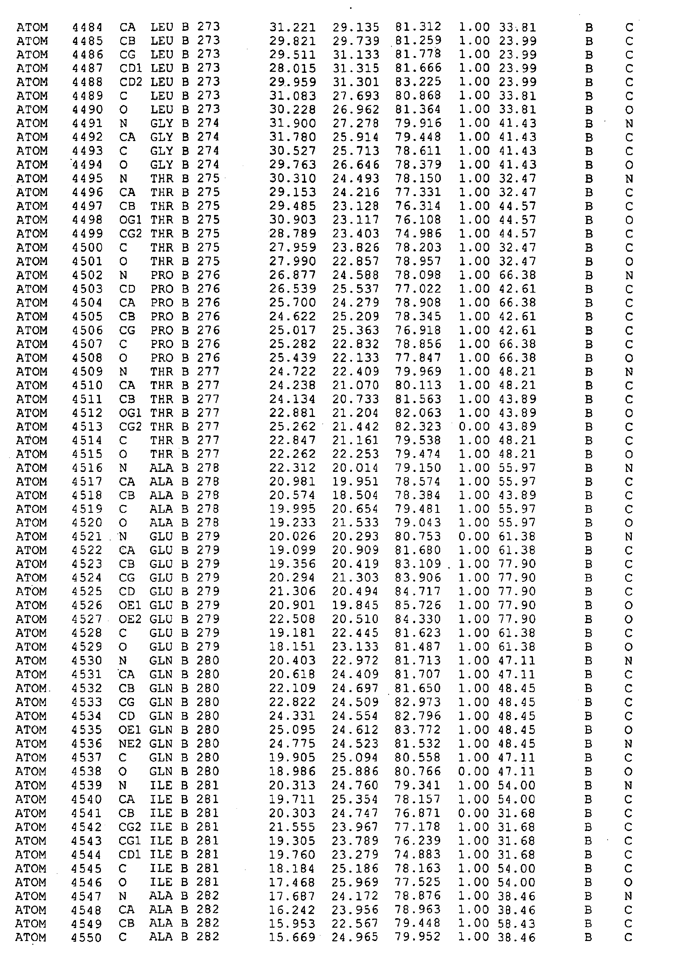

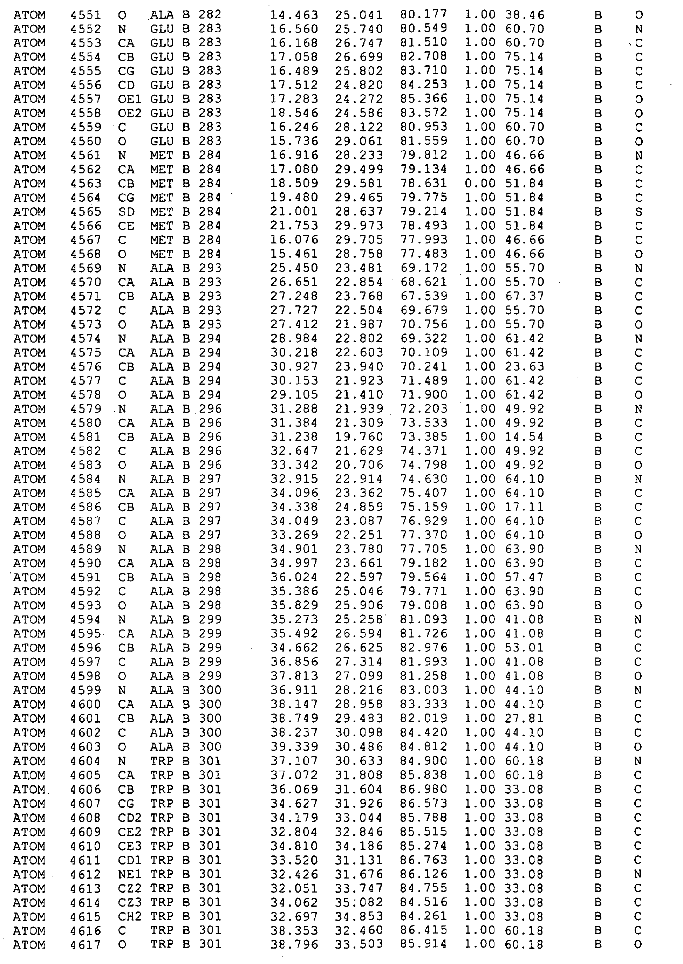

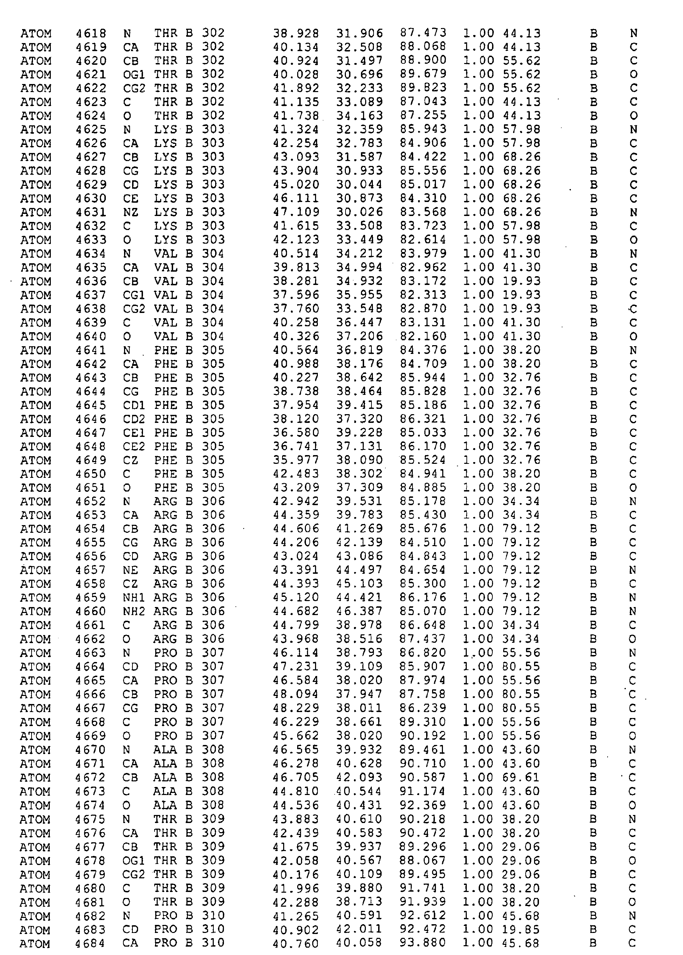

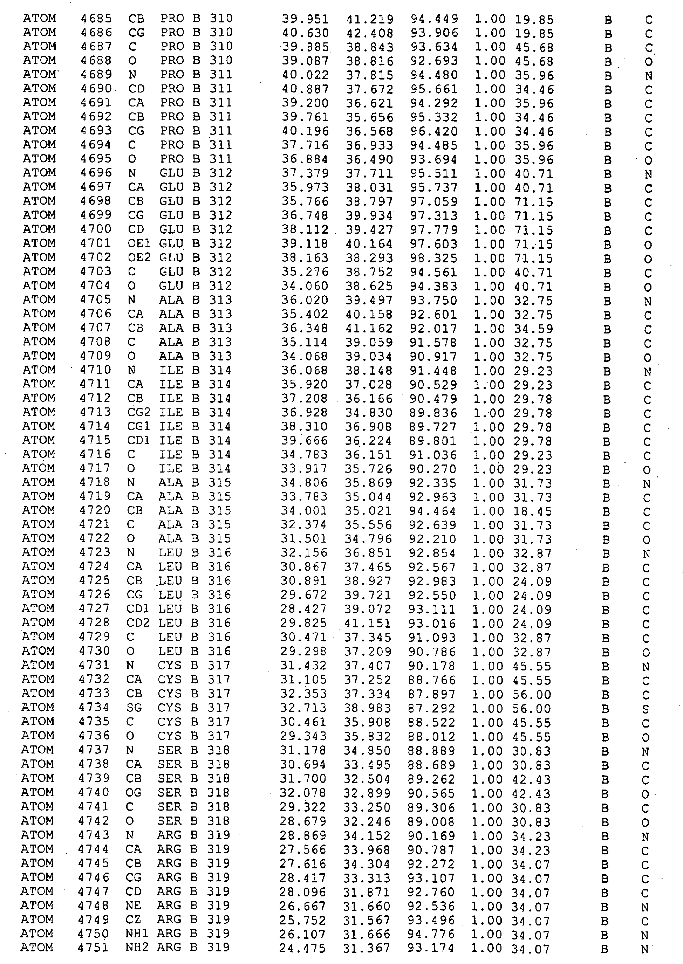

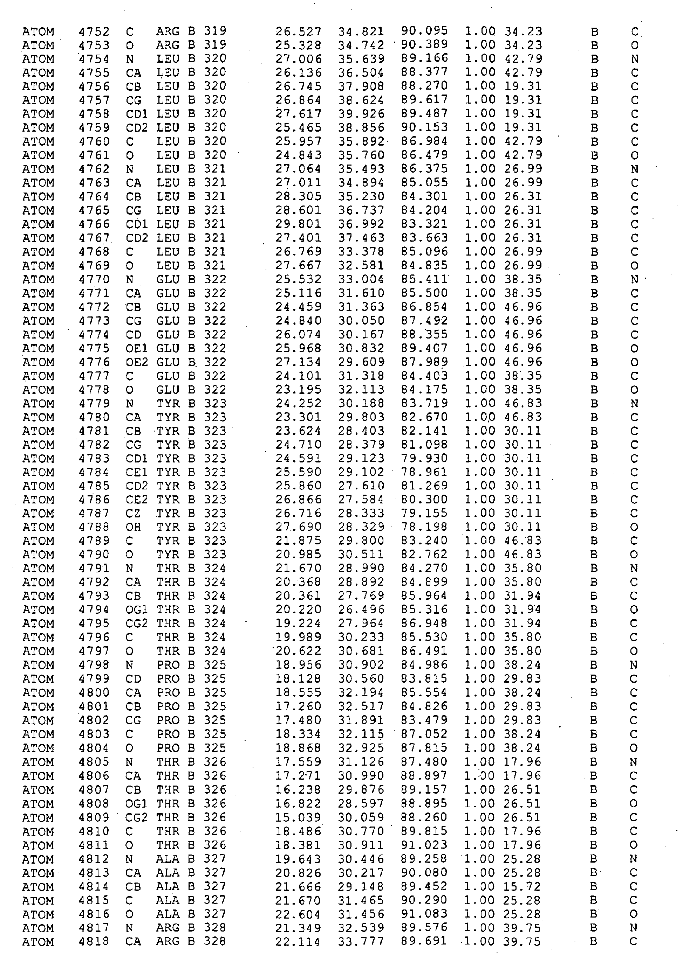

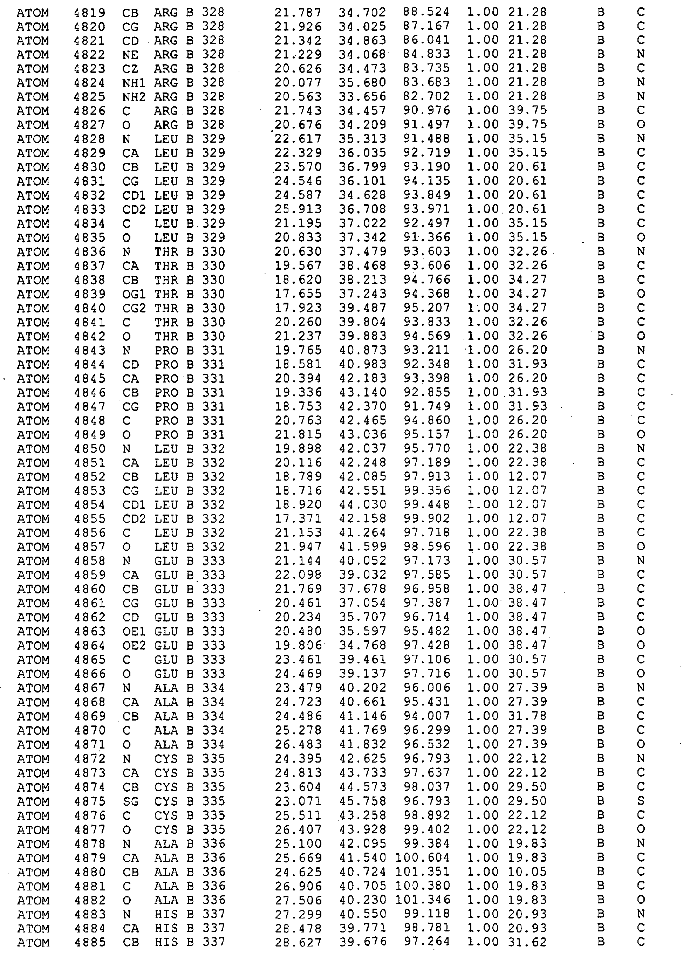

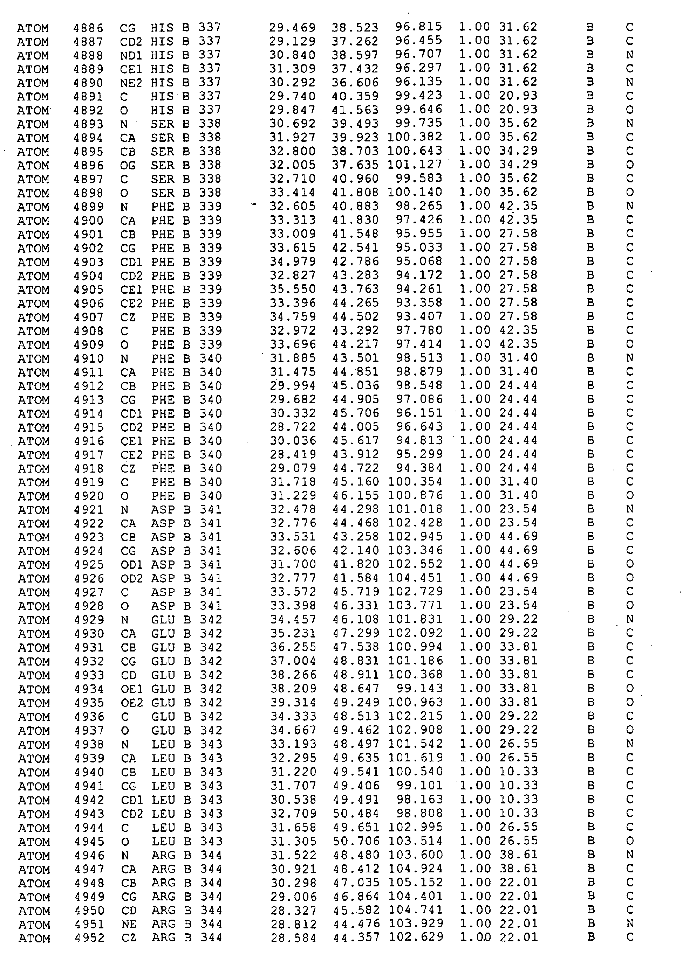

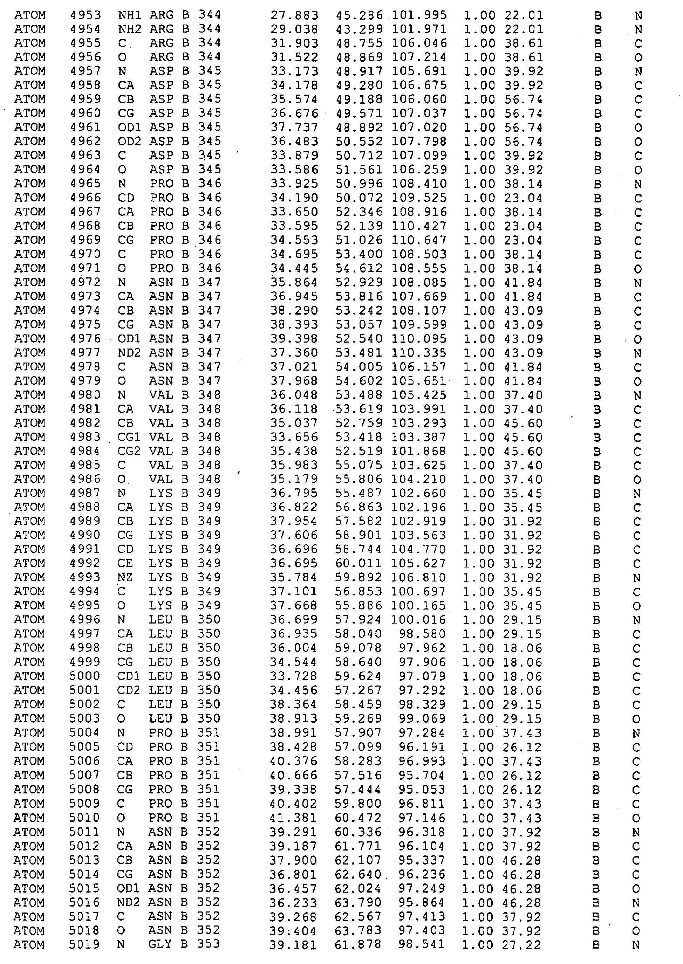

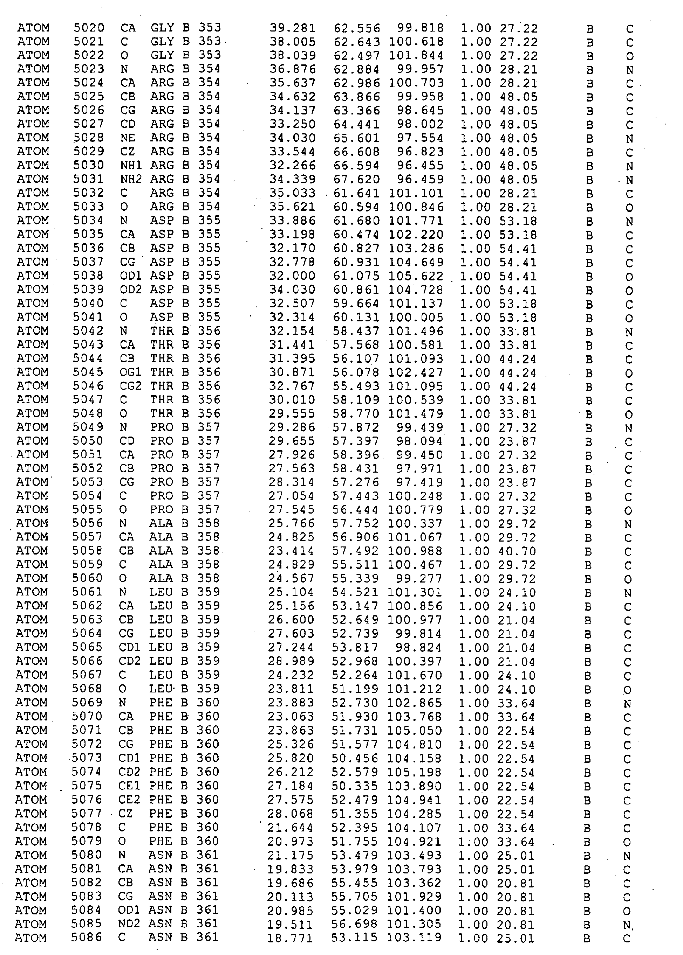

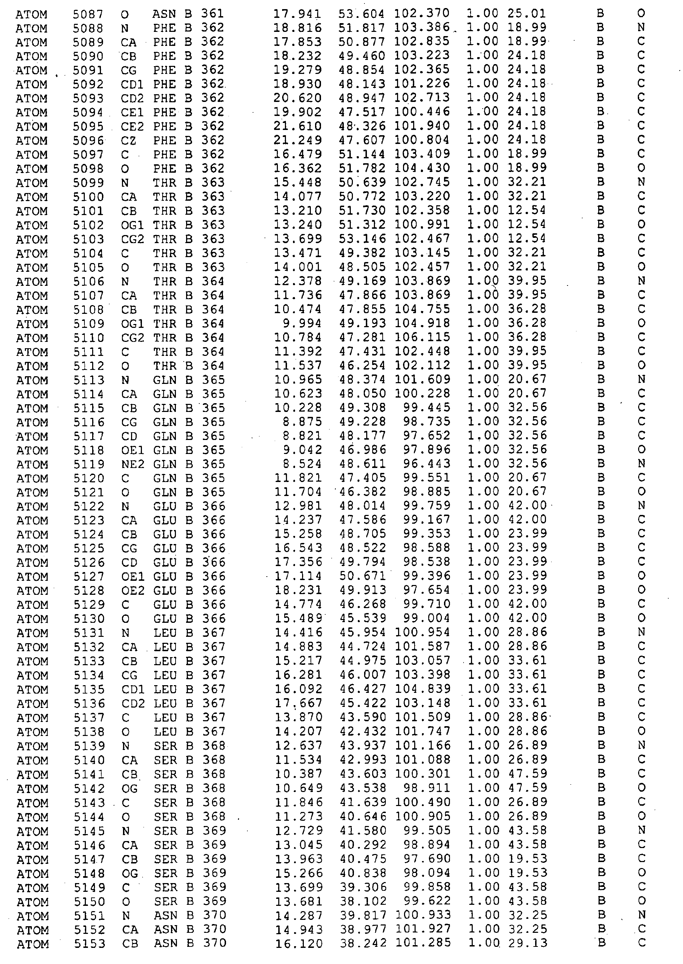

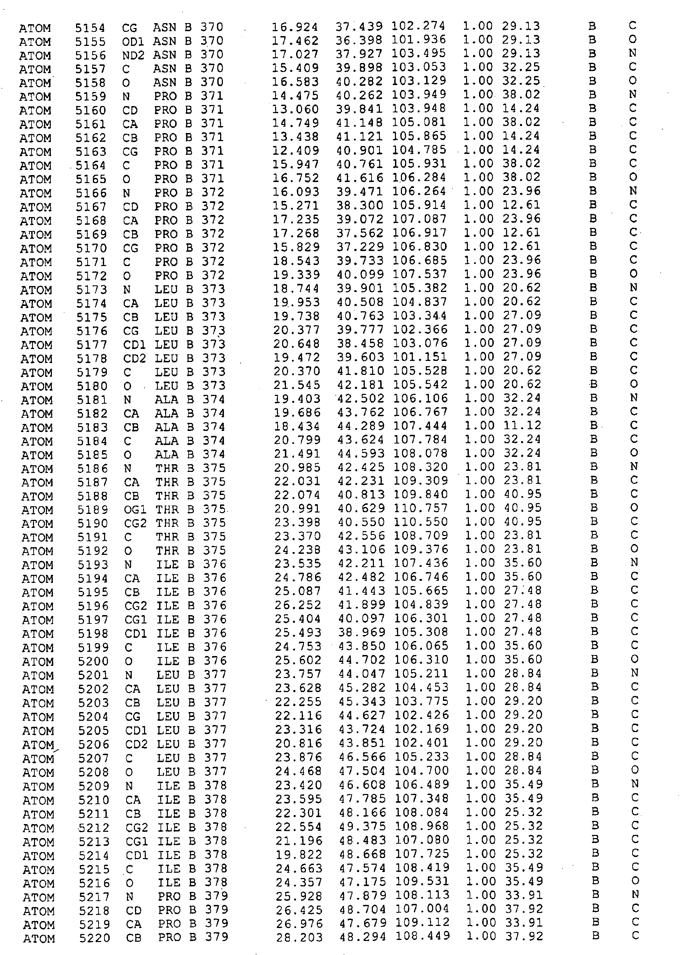

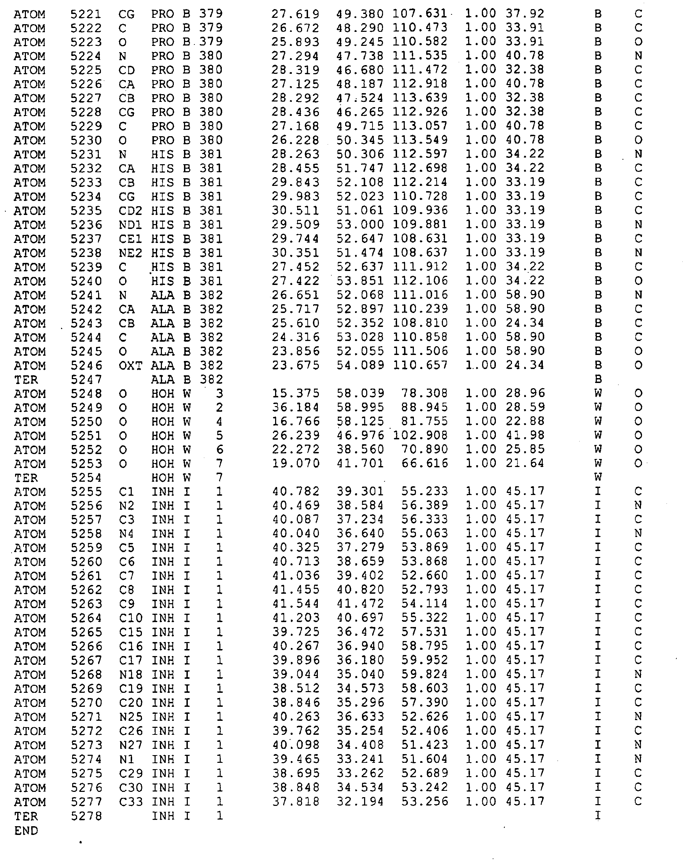

In the crystal structure of the unphosphorylated GSK-3β-inhibitor2 complex, amino acid residues 162, G63, V70, A83, V110, L132, D133, Y134, V135, P136, E137, T138, R141, L188 and C199 according to Figure 4 were within 5 Å of the inhibitor bound in the active site. These amino acid residues were identified using the programs QUANTA, O and RIBBONS. In addition, amino acid residues V61, 162, G63, N64, G65, G68, V69, V70, Y71, Q72, L81, V82, A83, I84, K85, E97, M101, V110, R111, L130, V131, L132, D133, Y134, V135, P136, E137, T138, V139, Y140, R141, Q185, N186, L187, L188, L189, K197, L198, C199, D200 and F201 according to Figure 4 were within 8 Å of the inhibitor bound in the active site. These amino acids residues were identified using the programs QUANTA, O and RIBBONS.

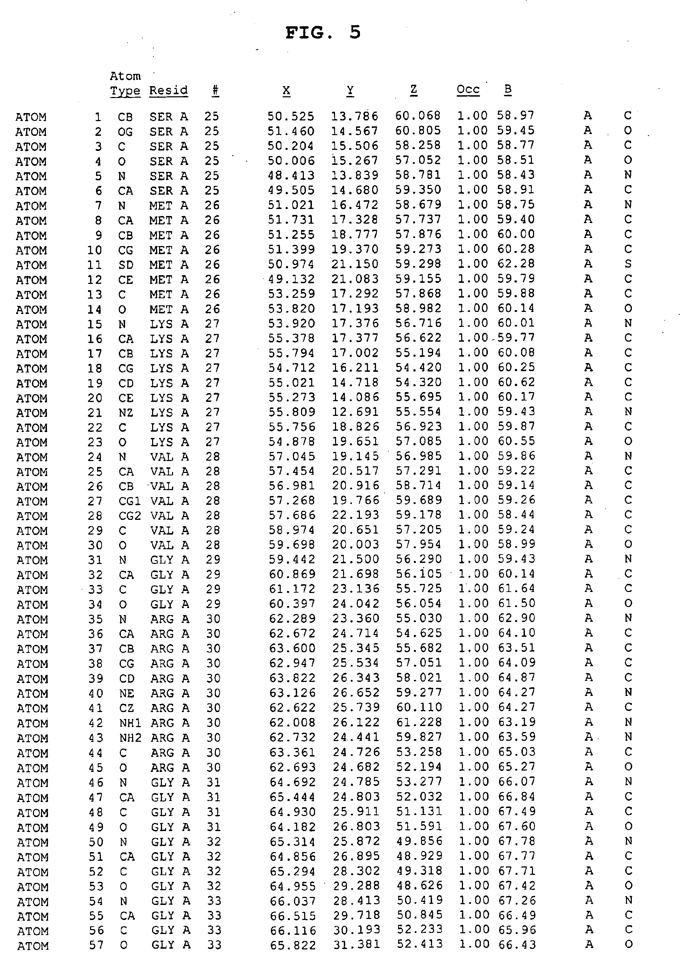

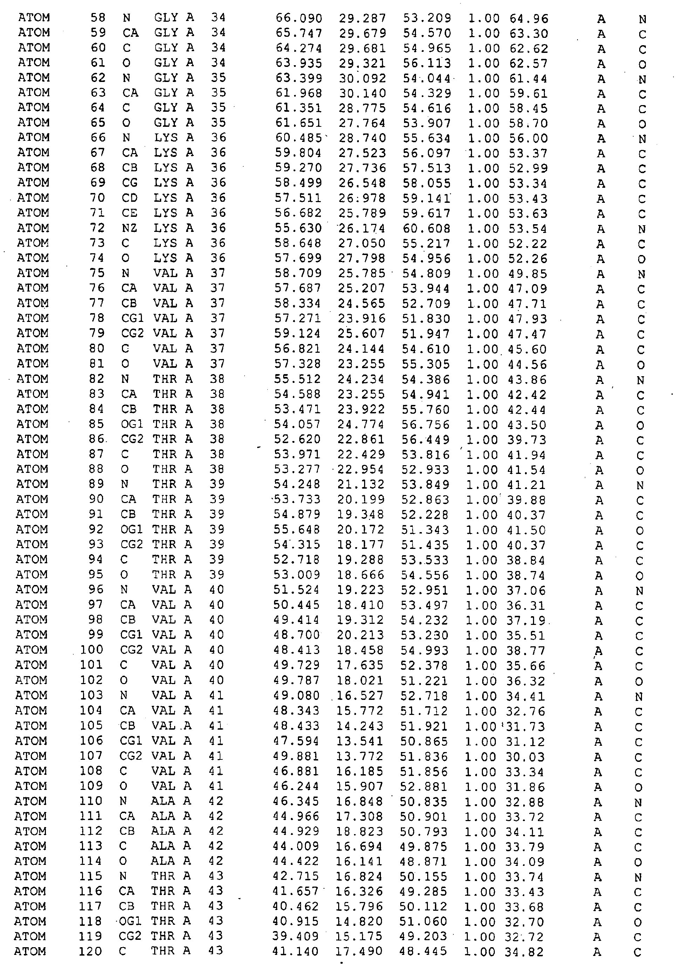

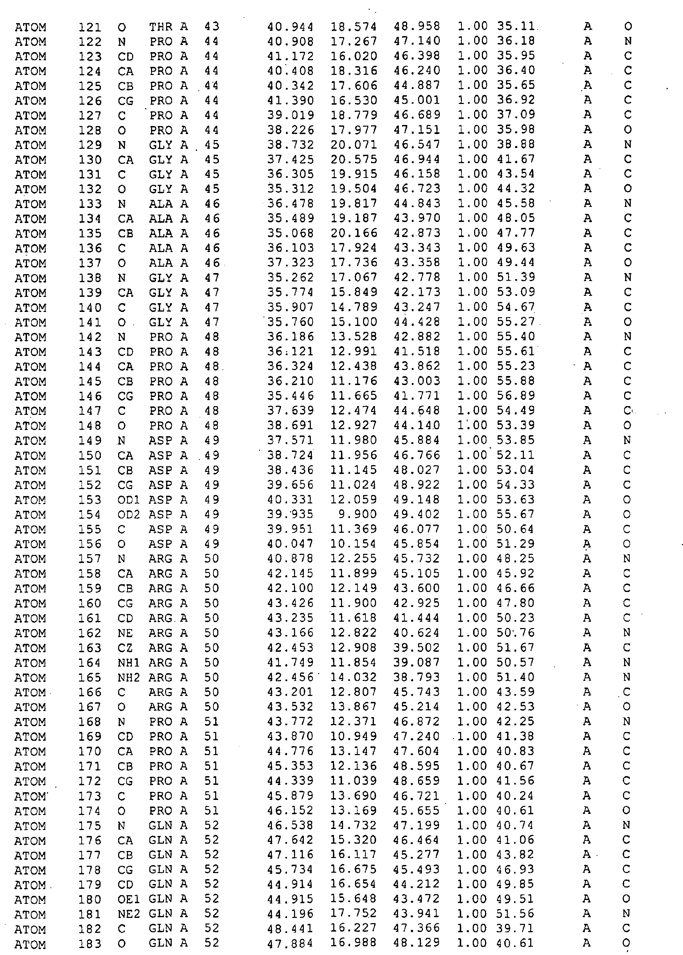

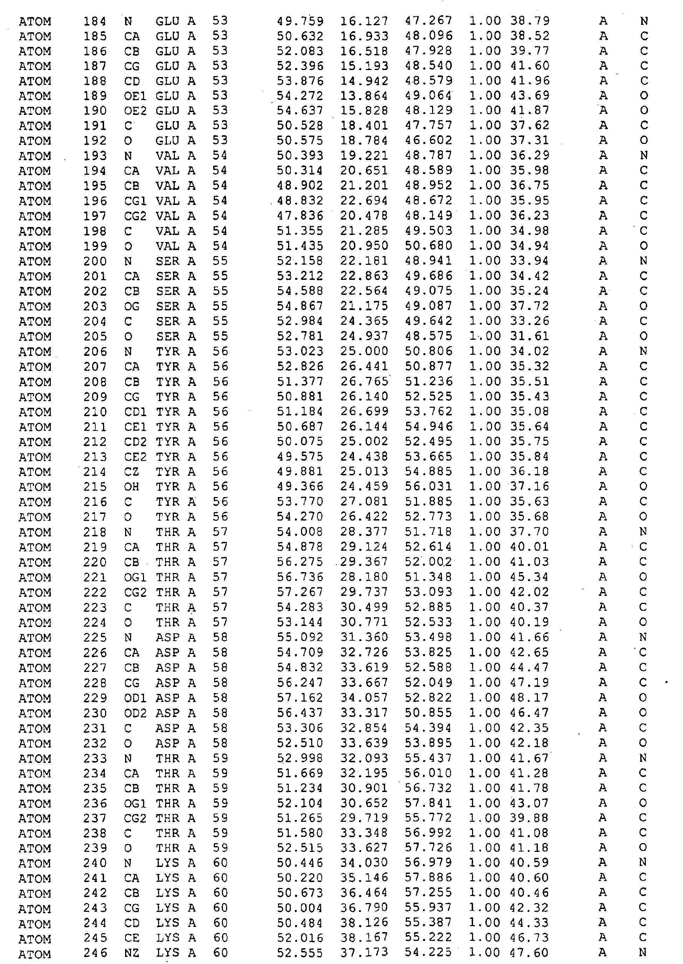

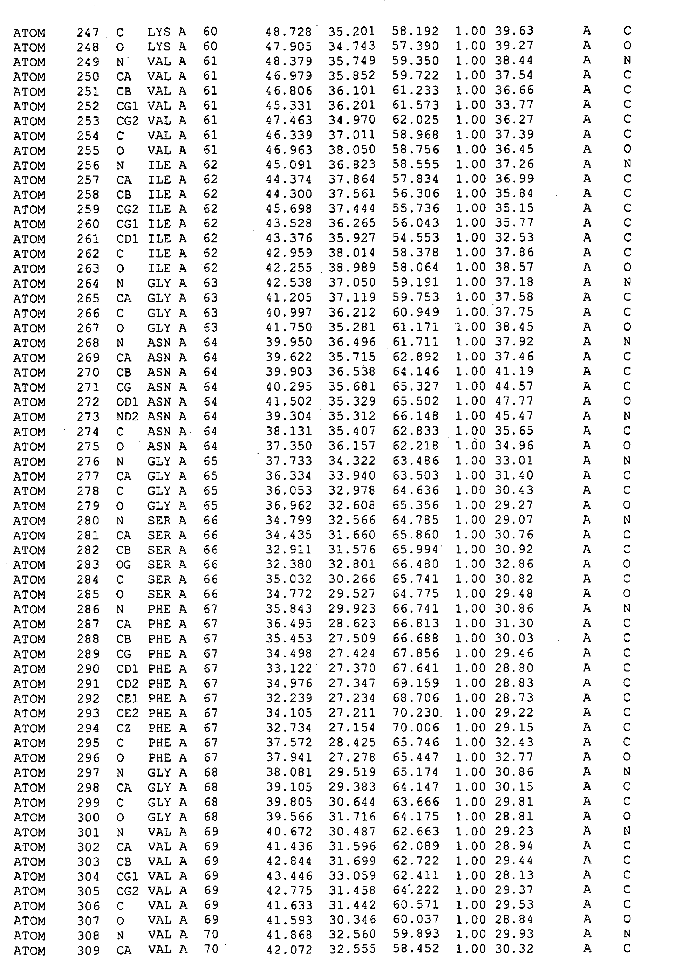

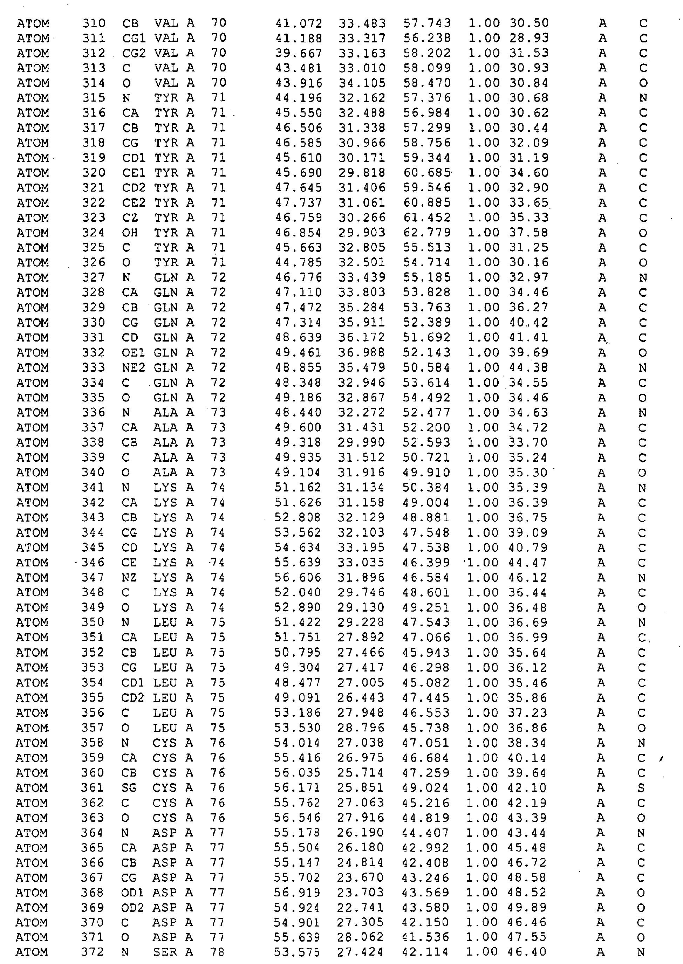

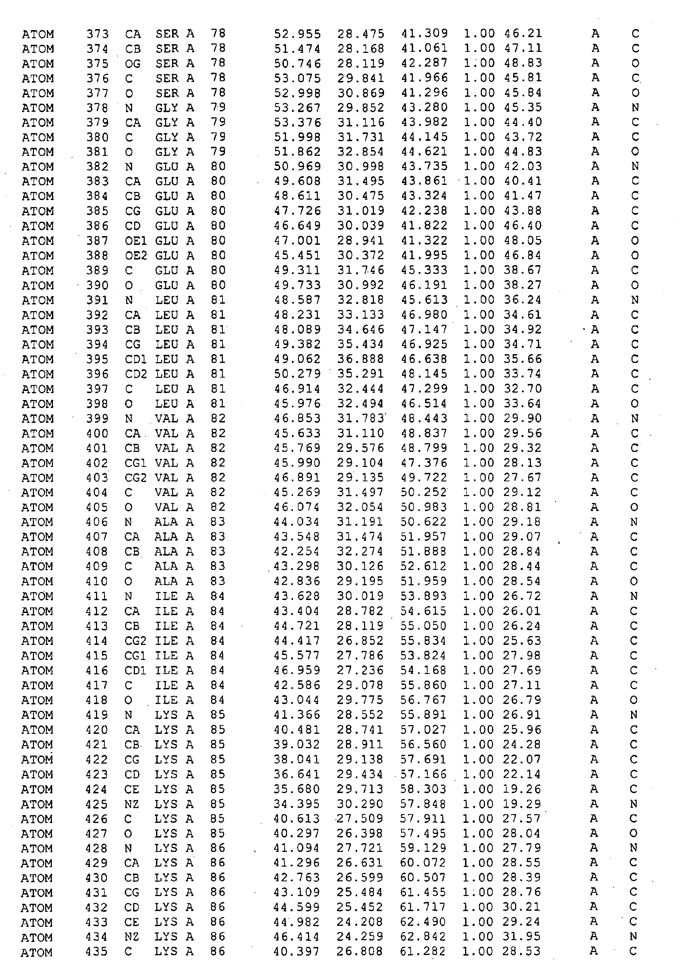

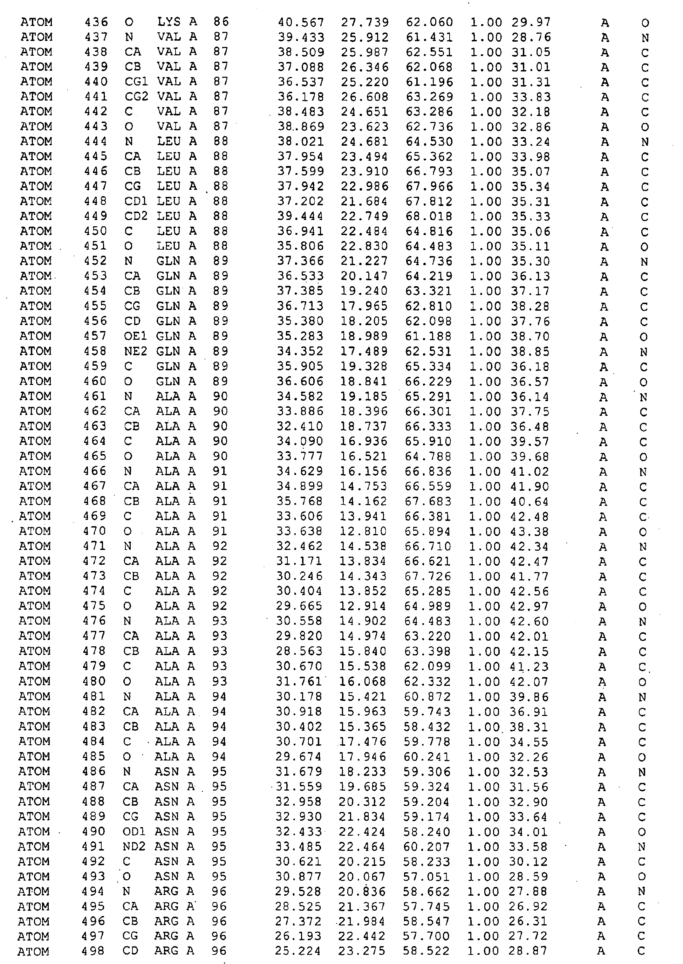

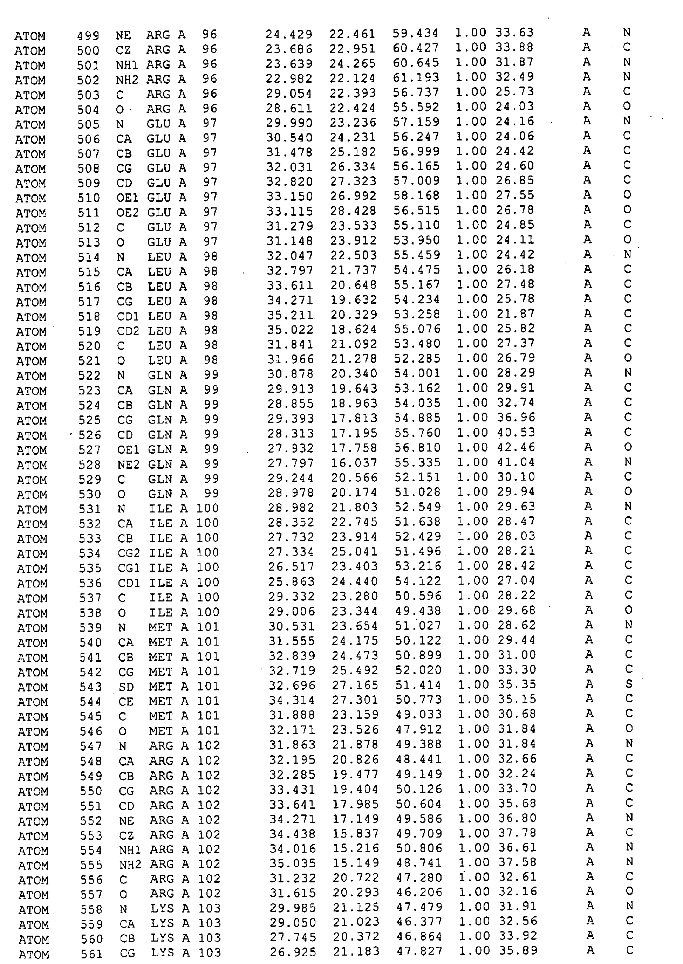

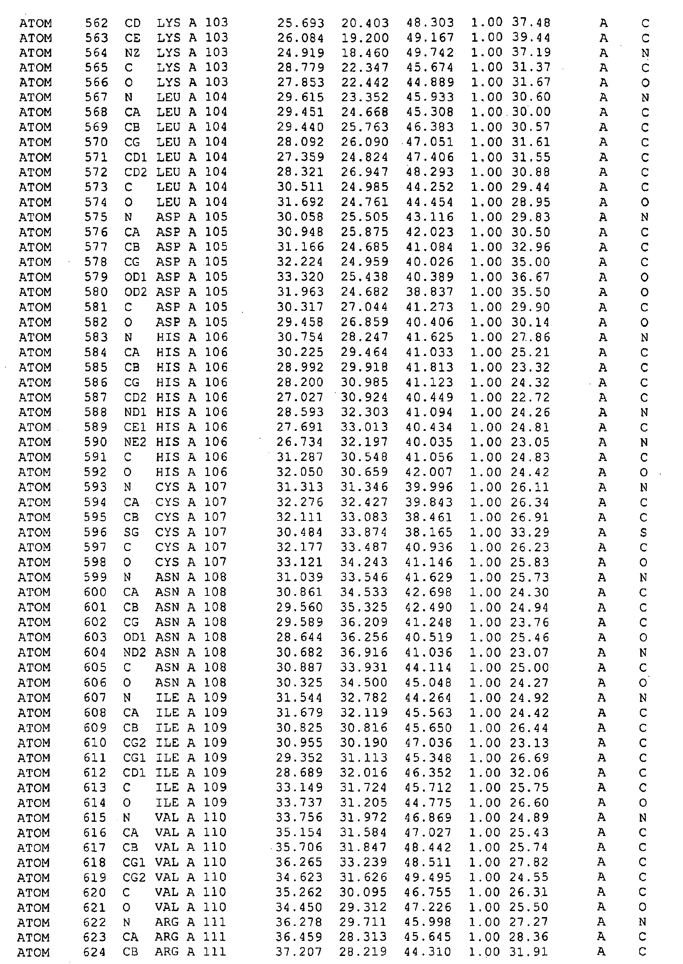

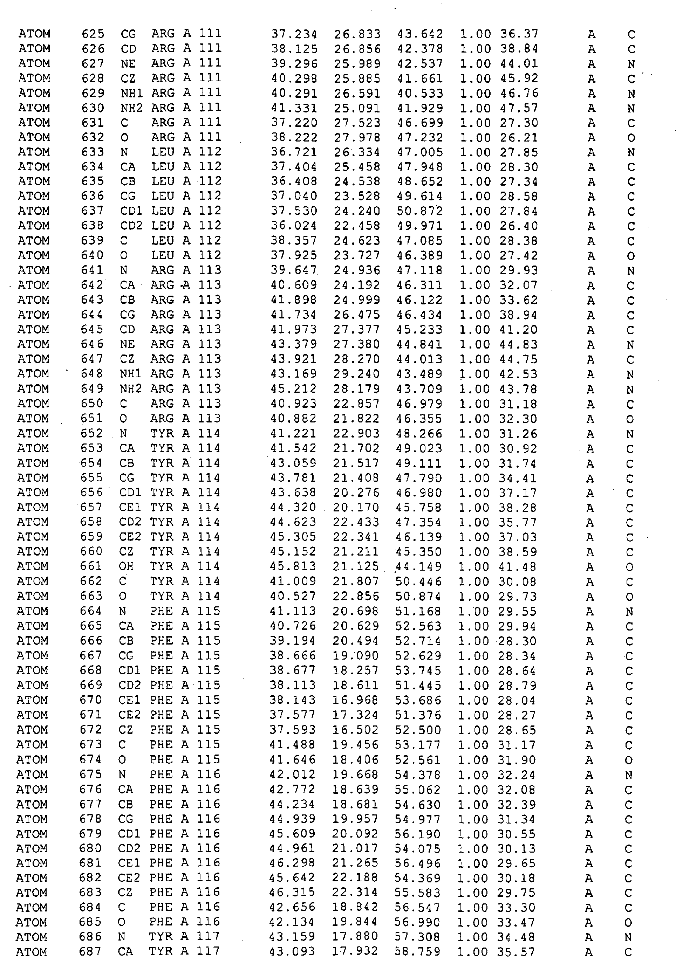

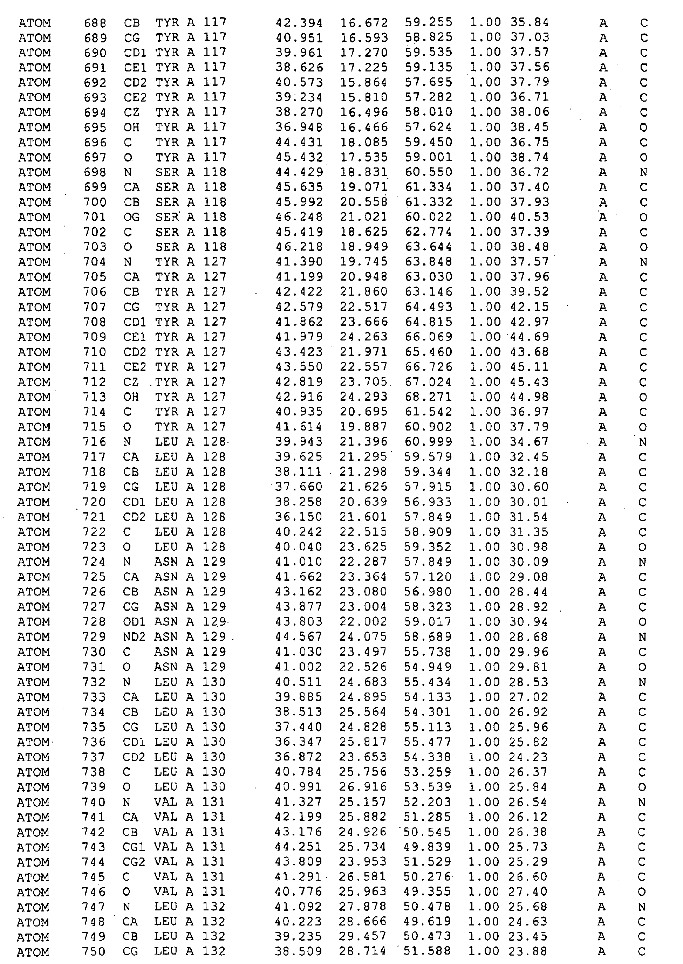

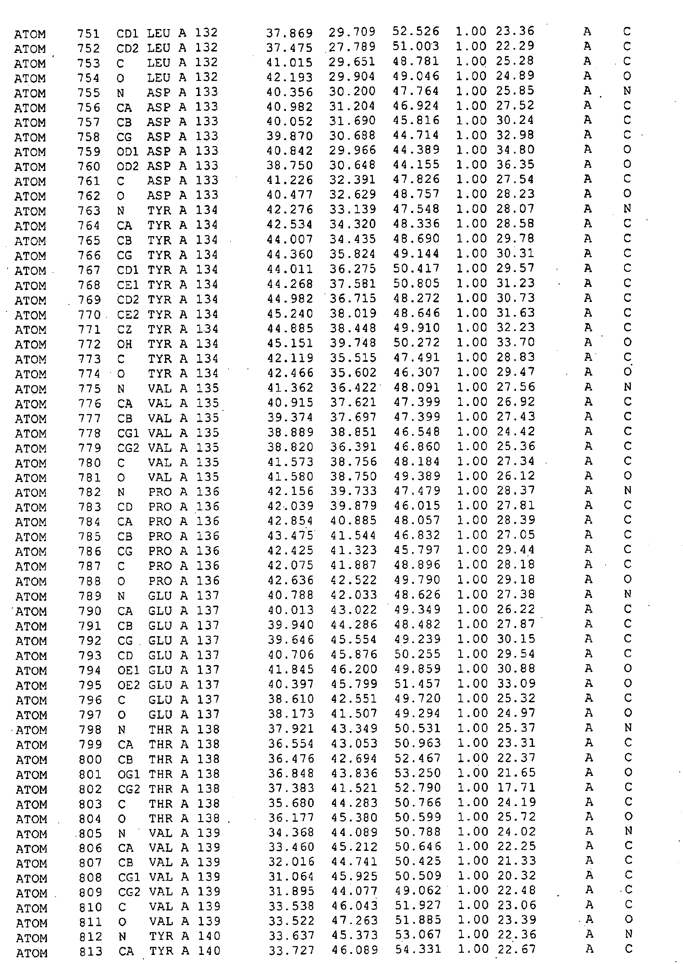

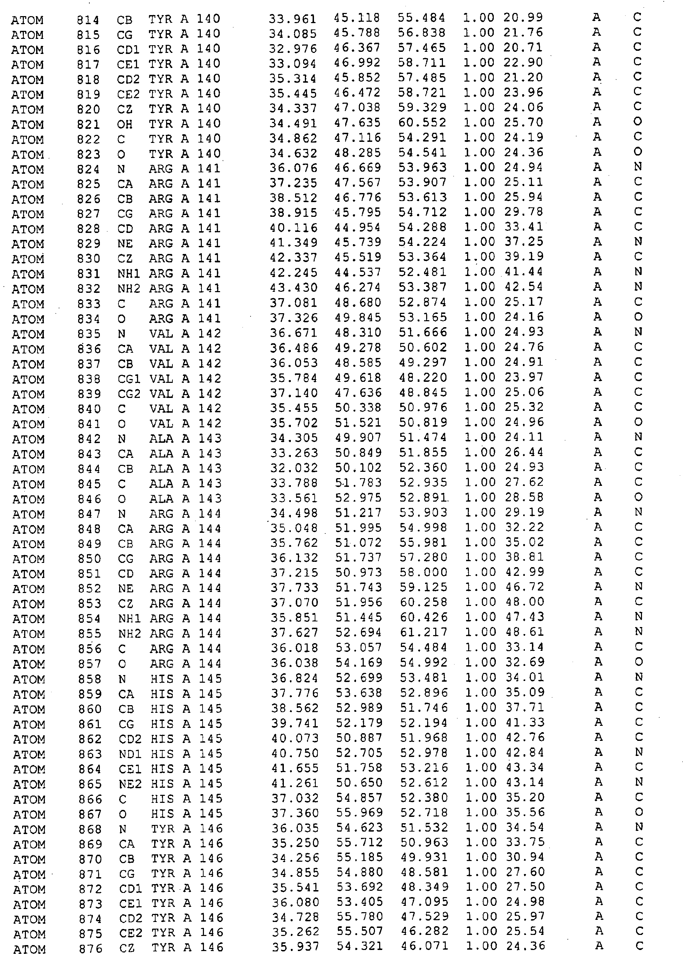

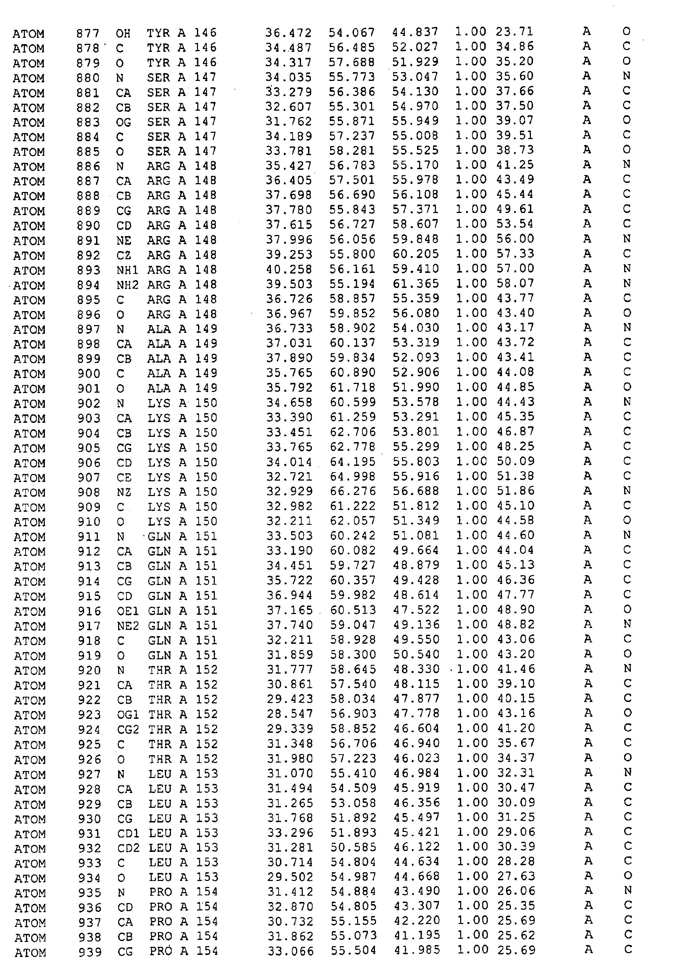

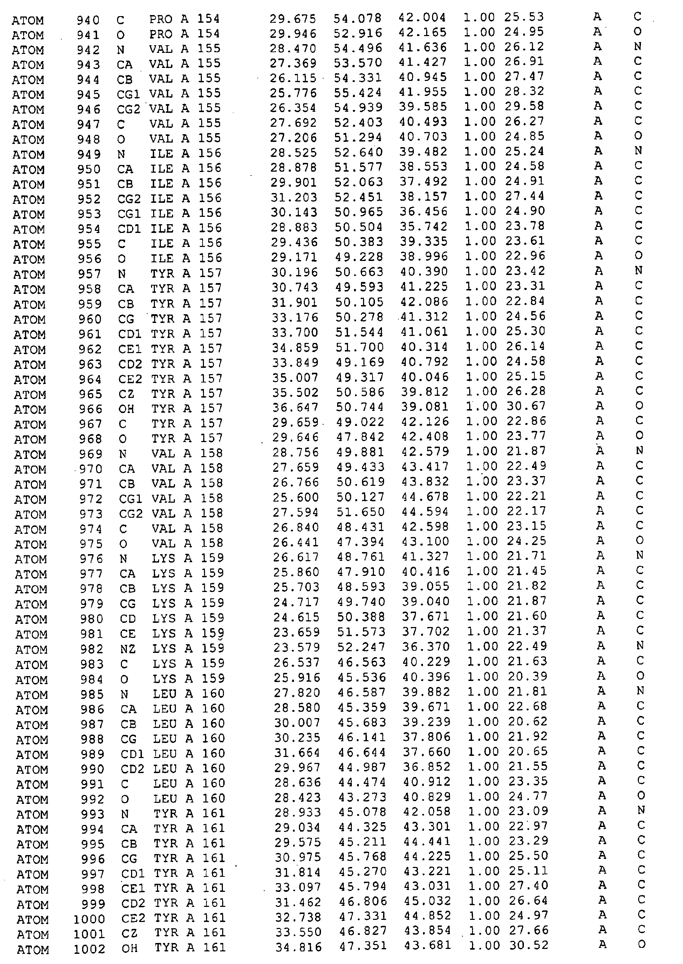

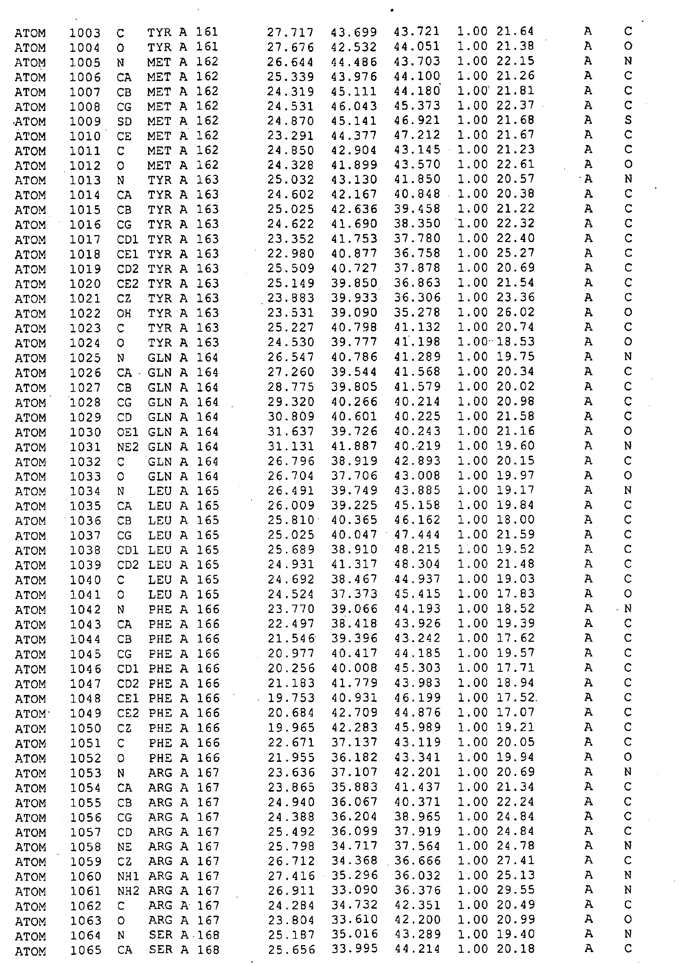

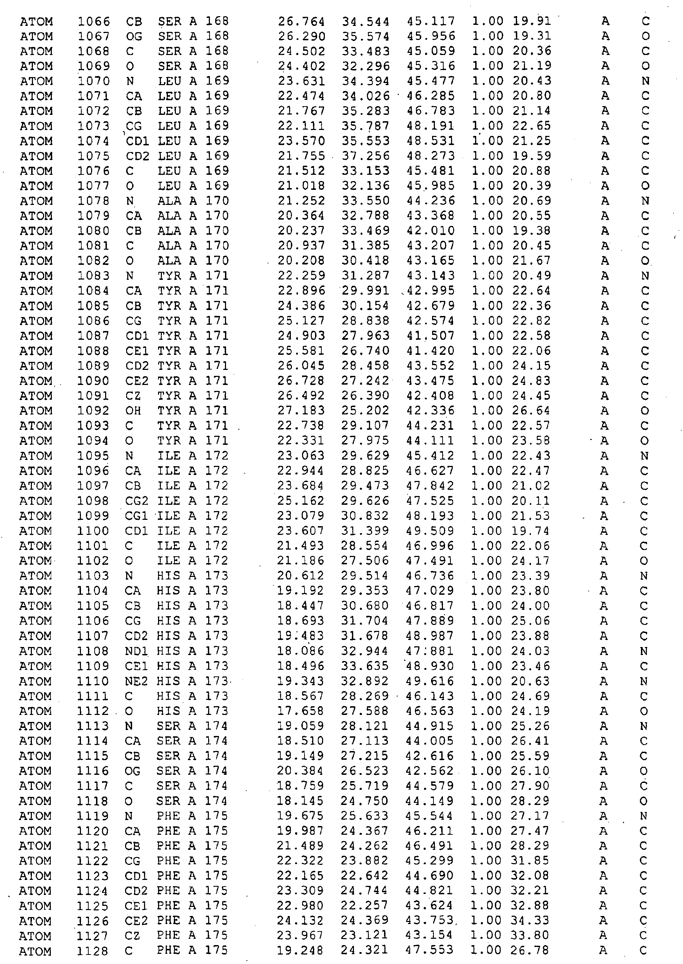

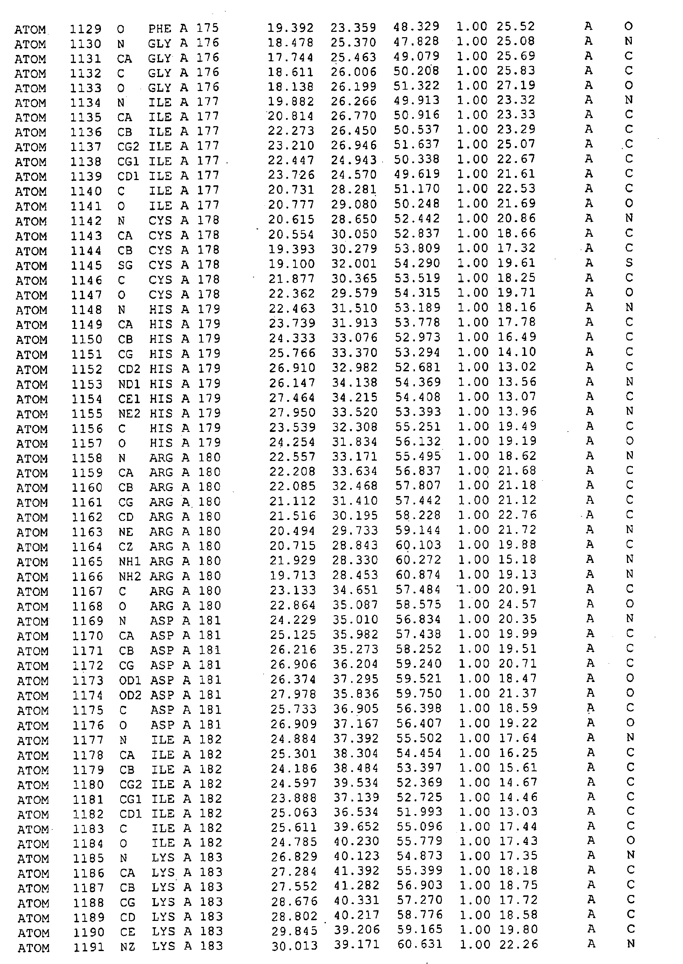

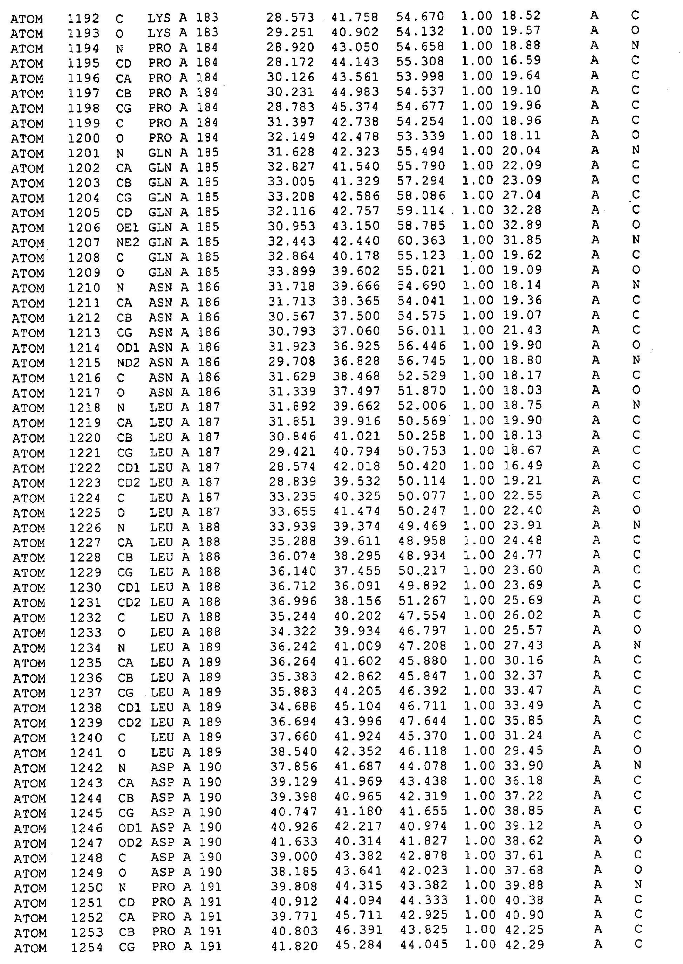

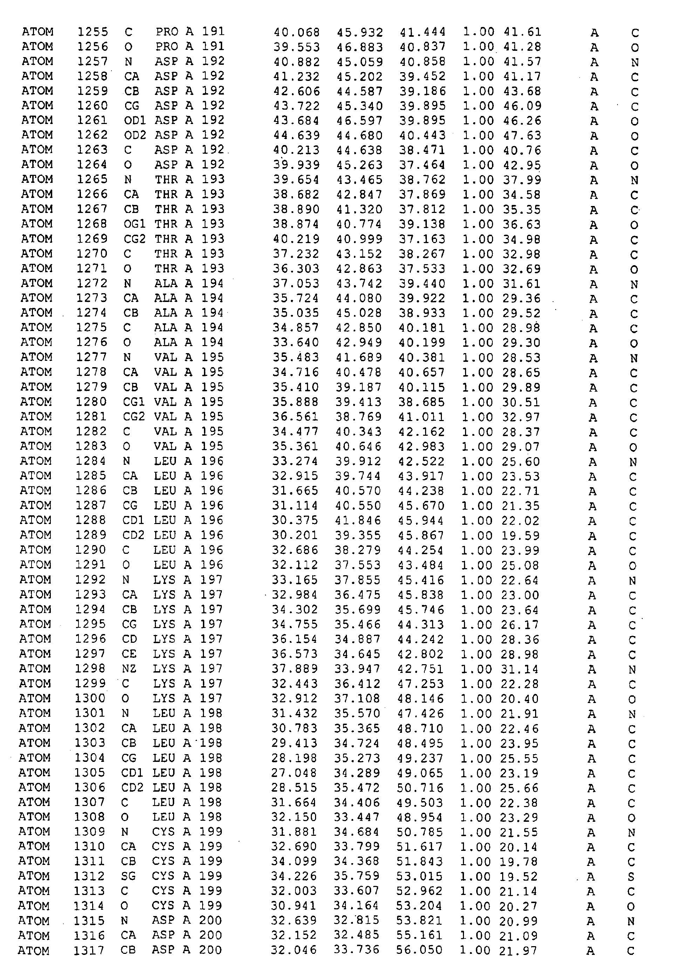

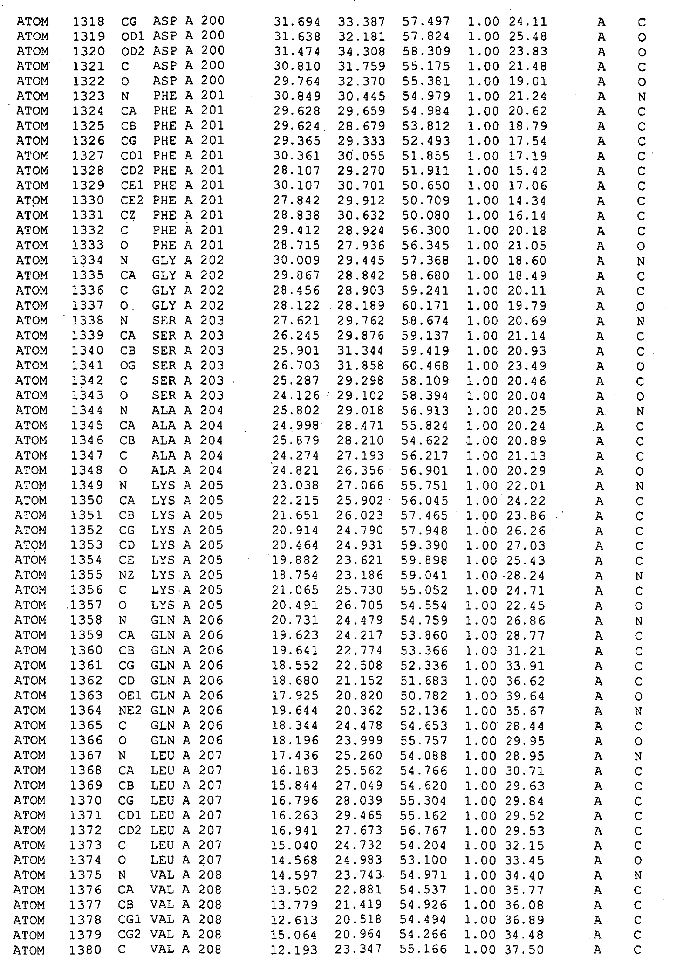

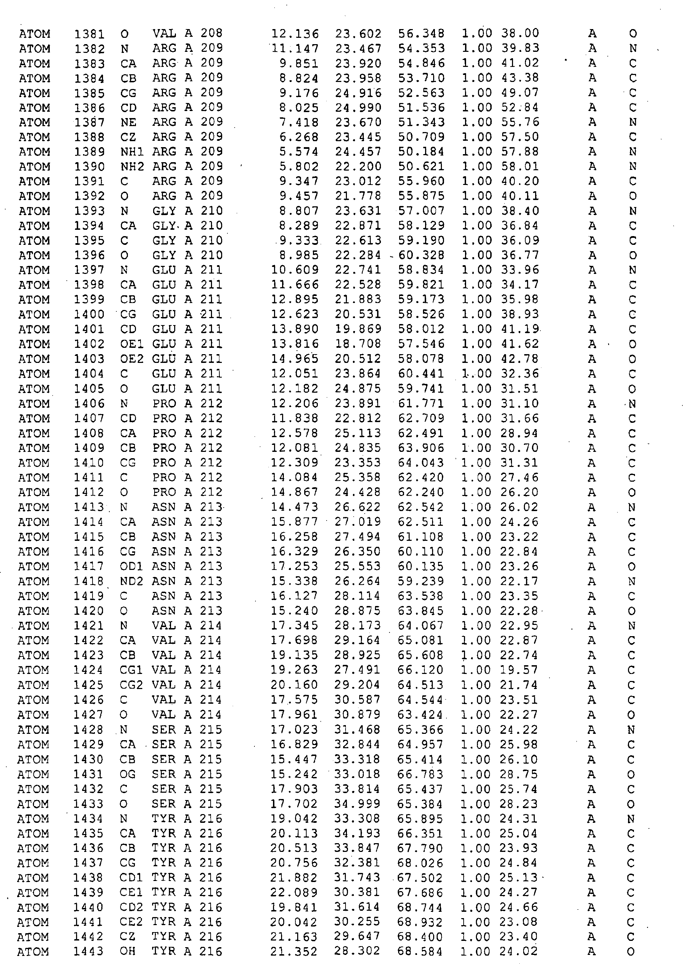

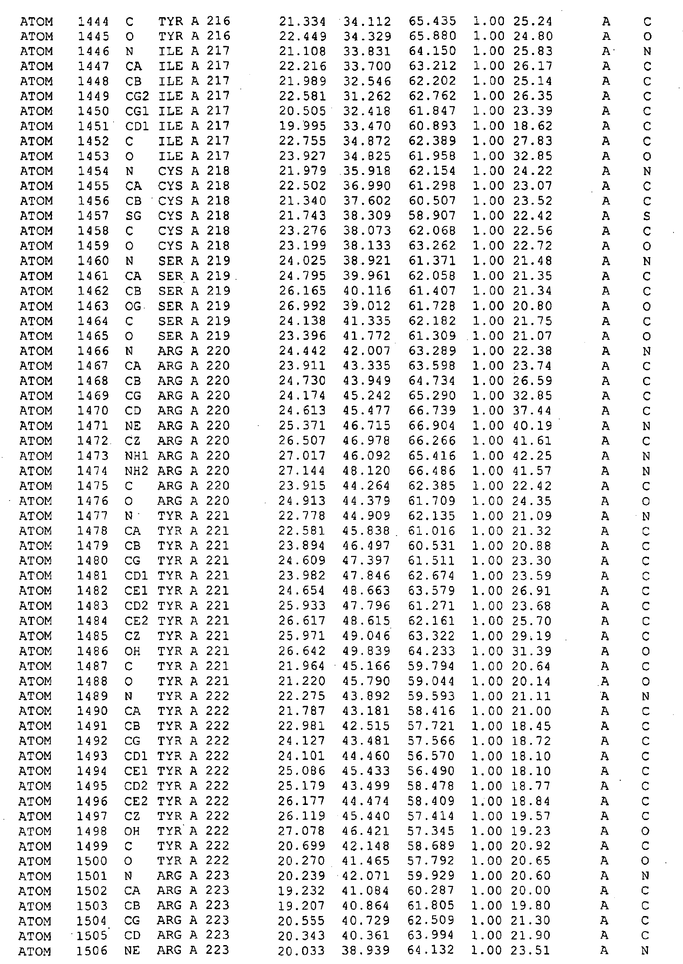

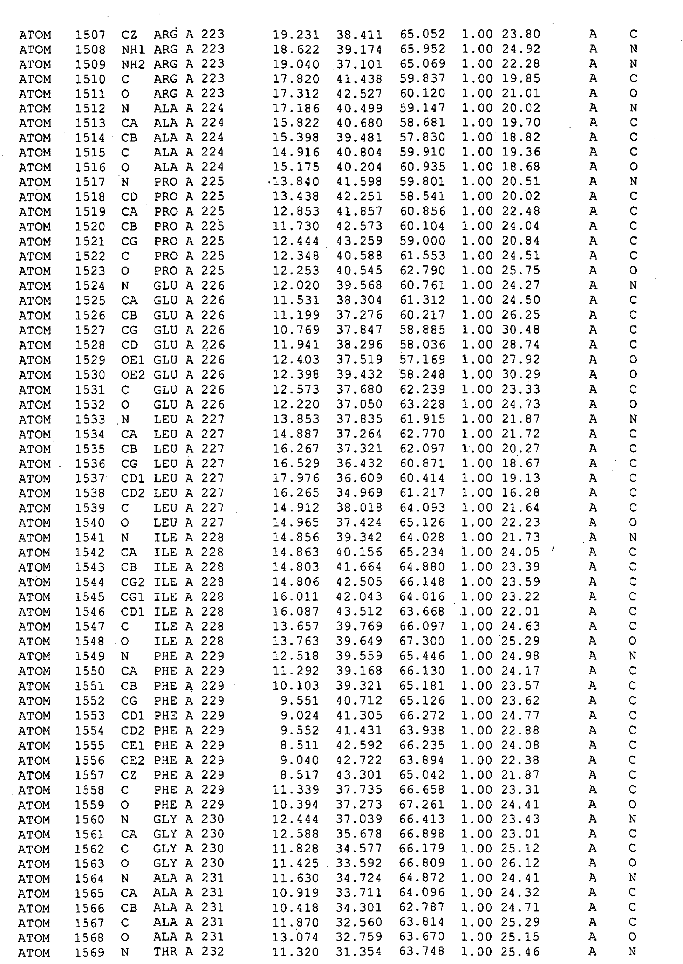

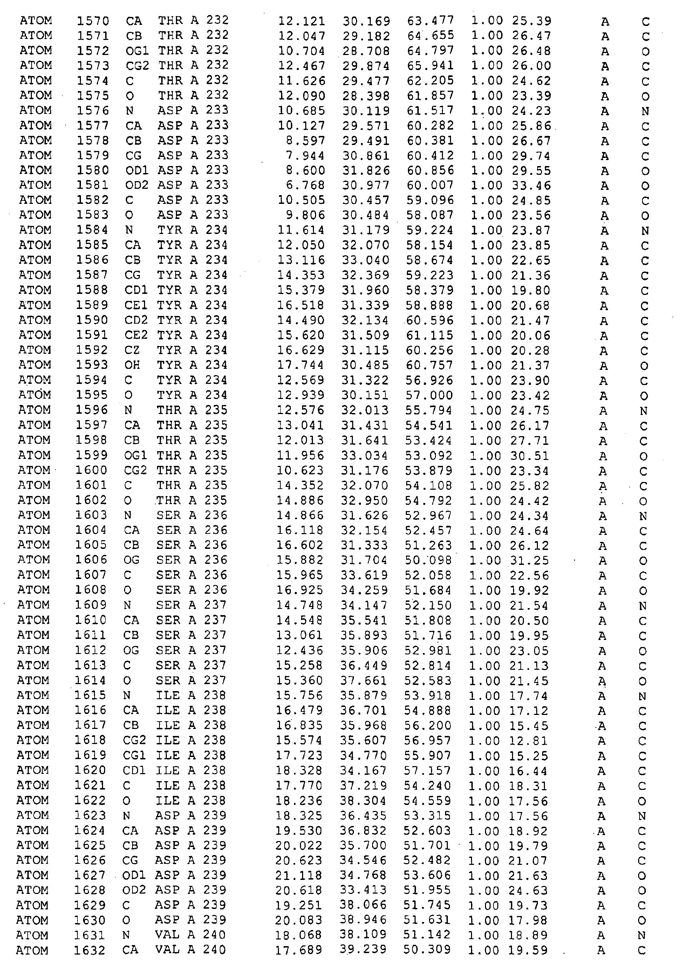

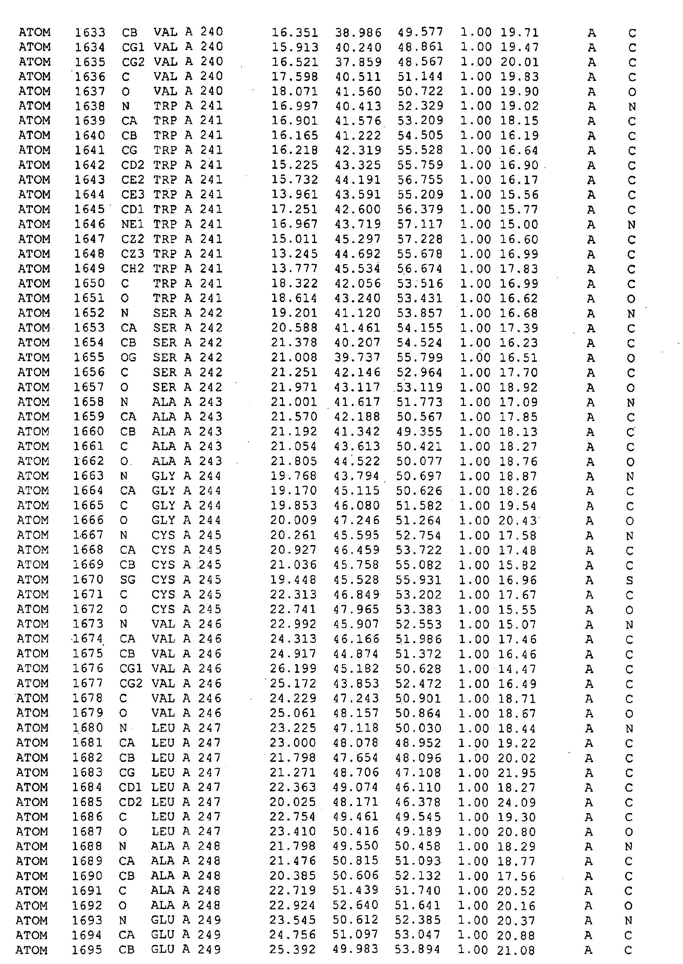

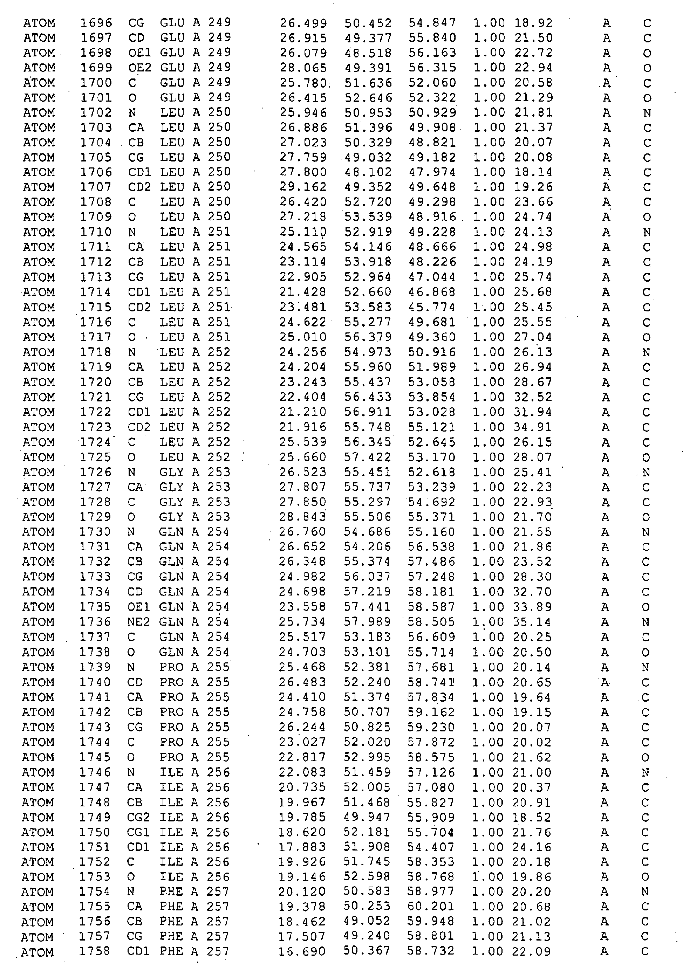

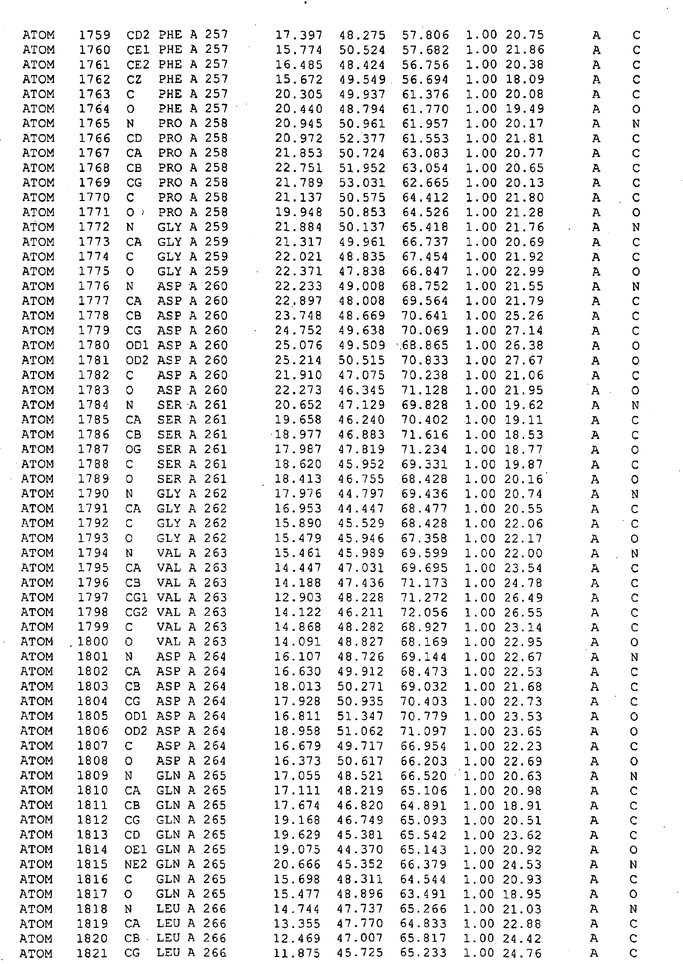

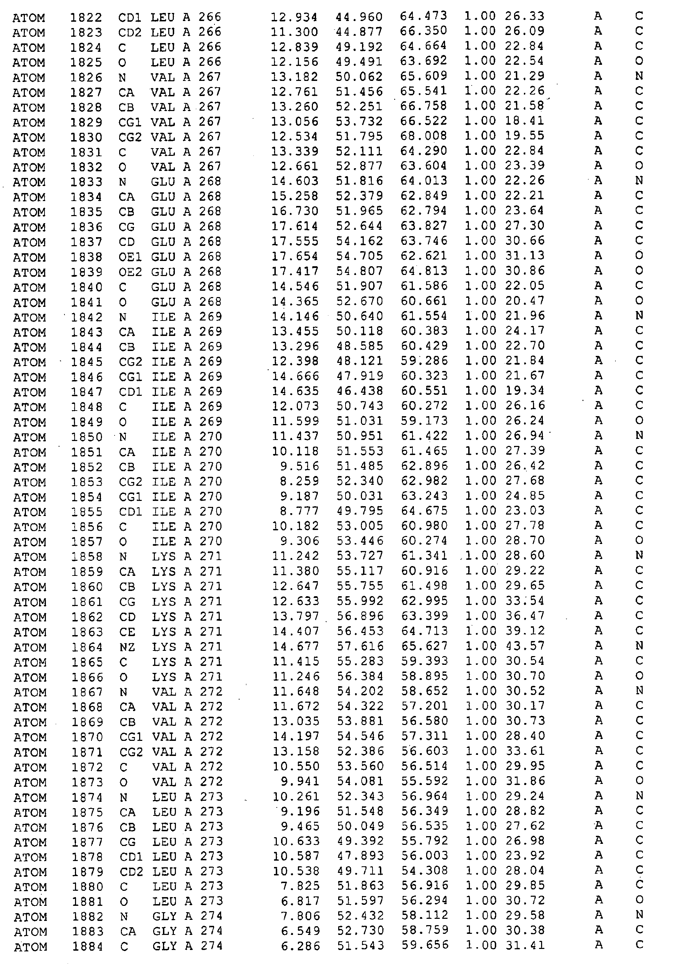

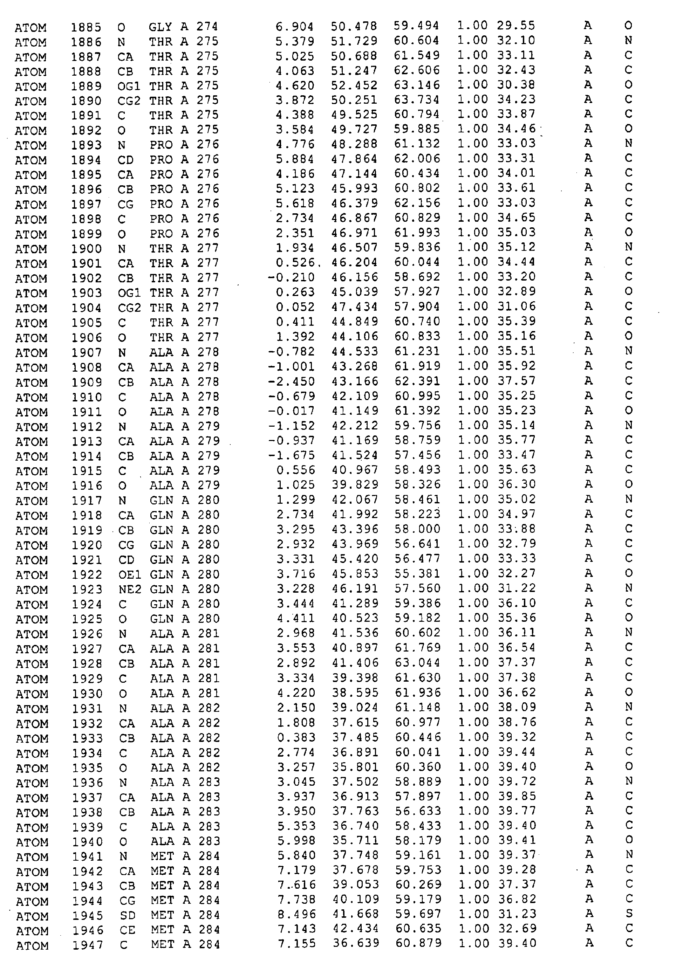

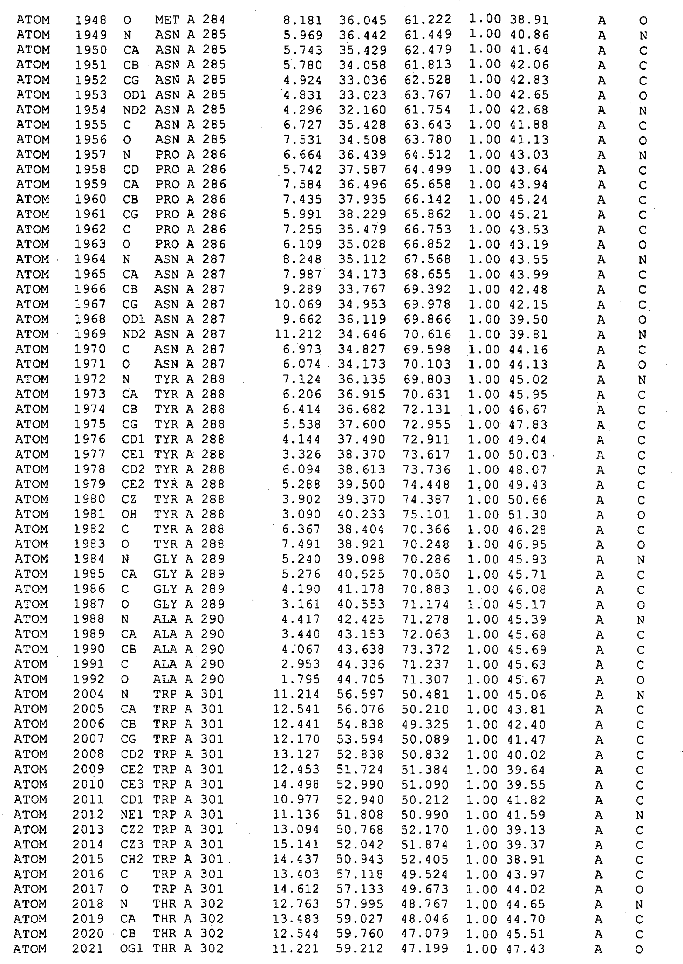

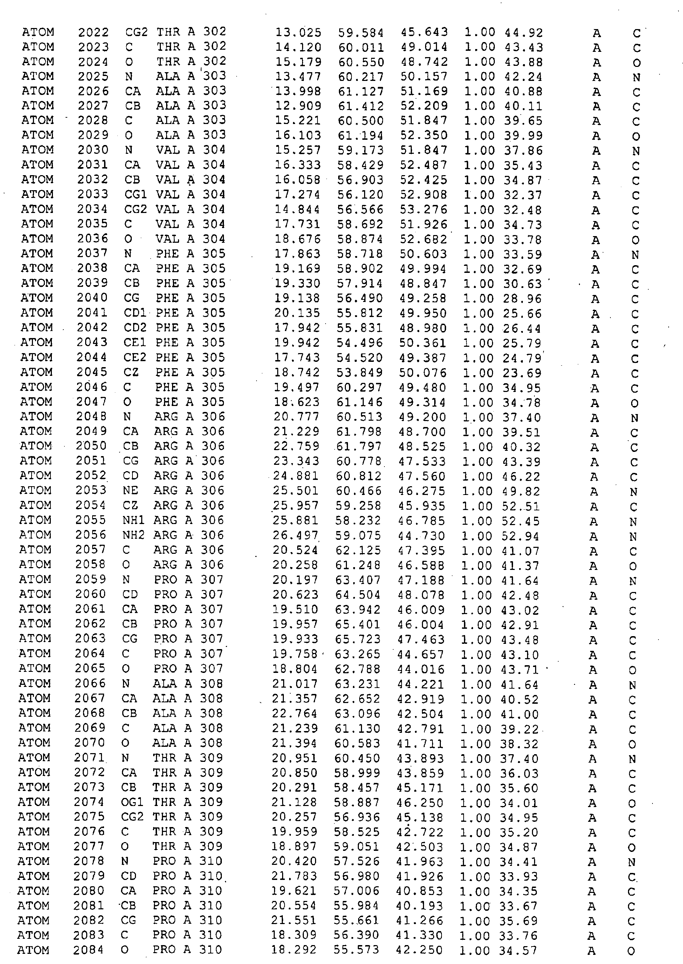

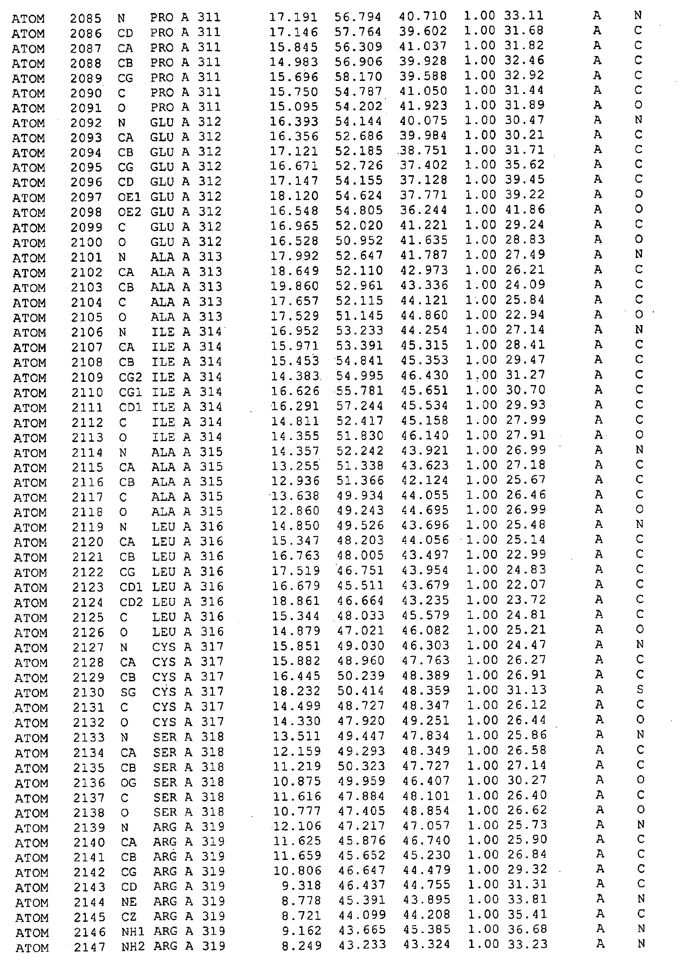

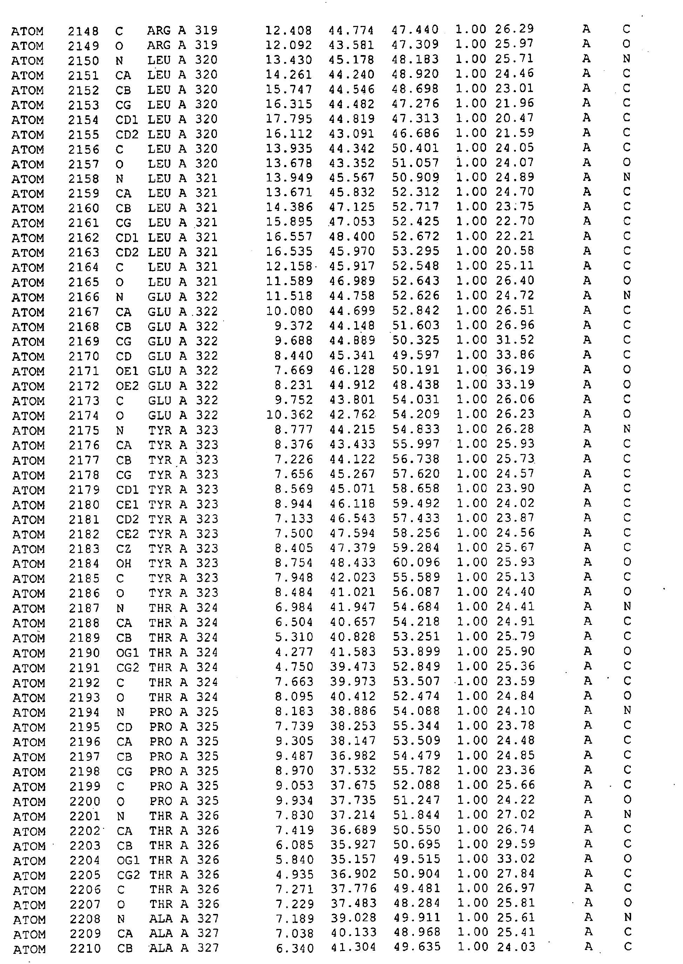

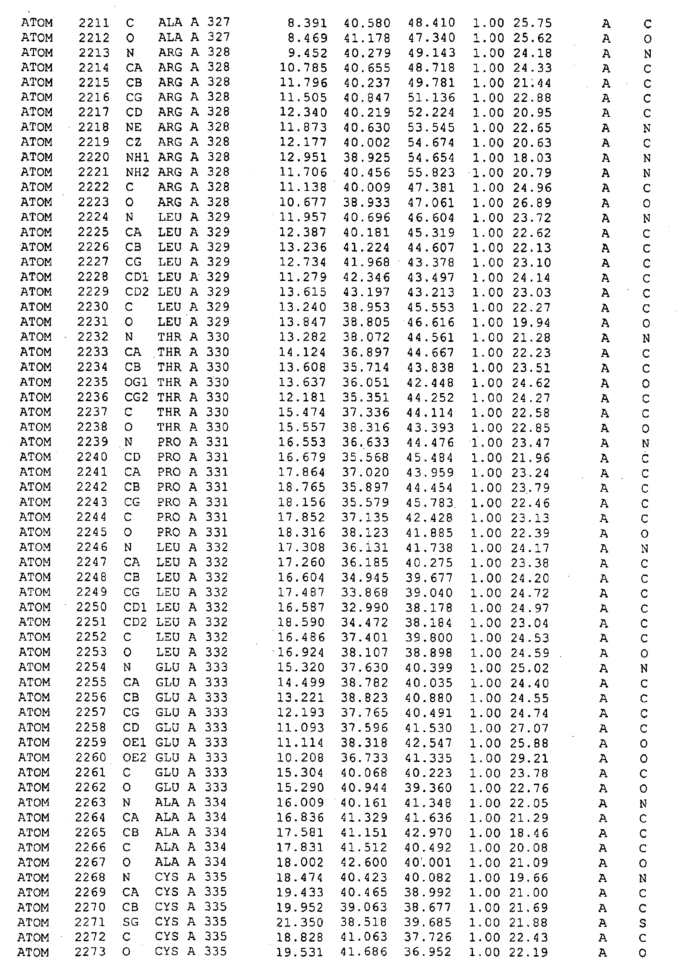

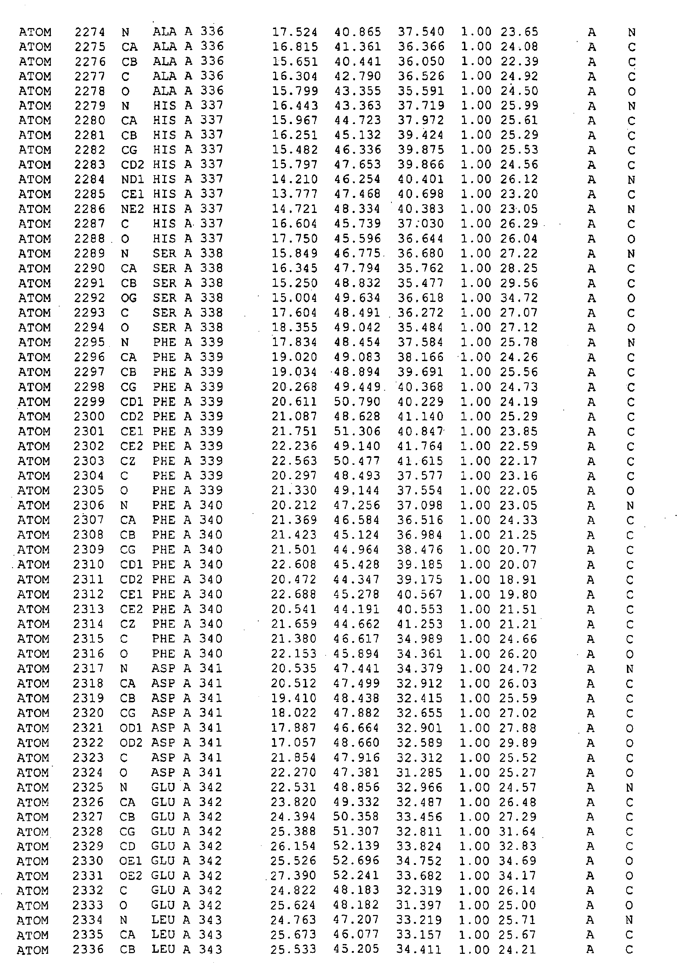

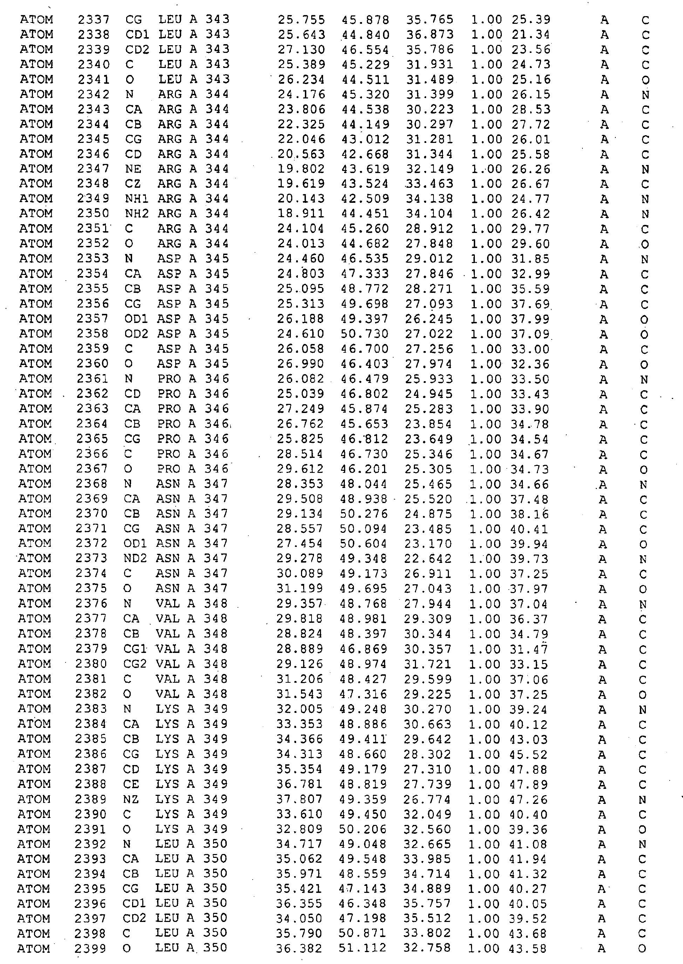

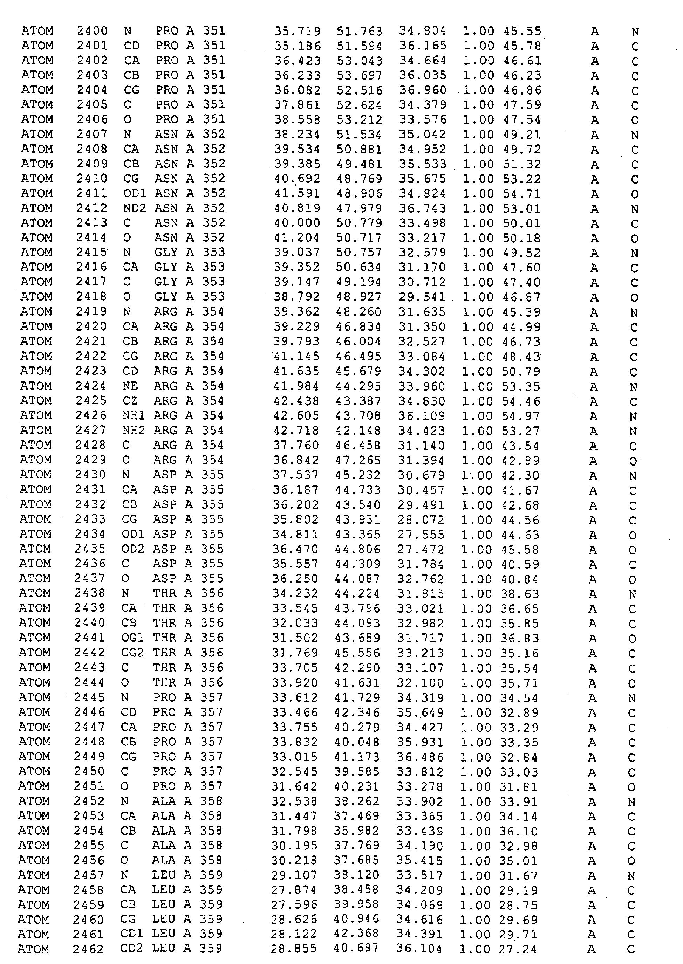

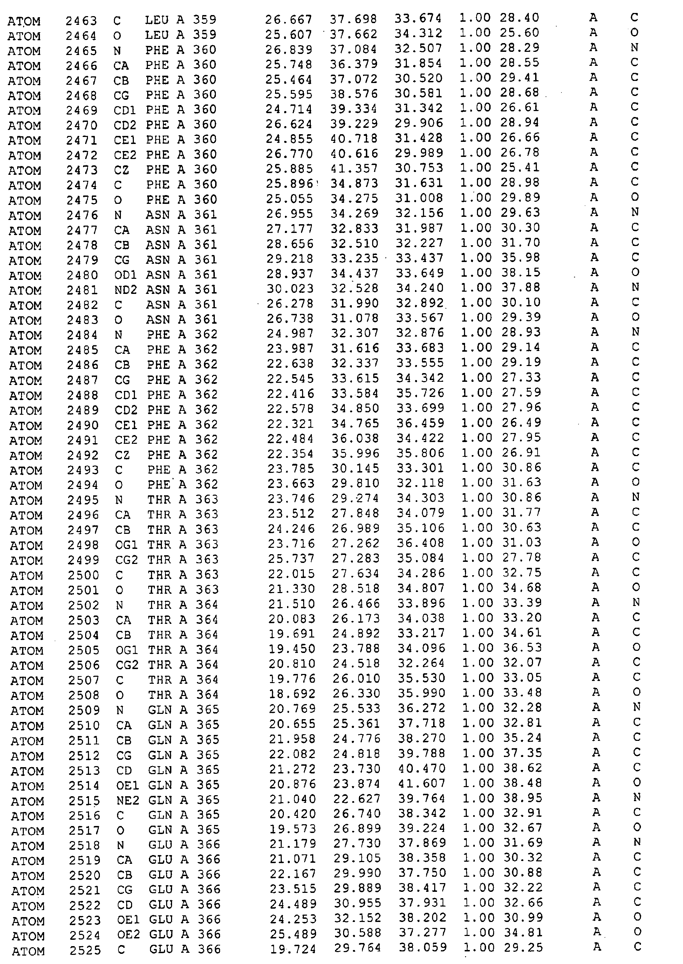

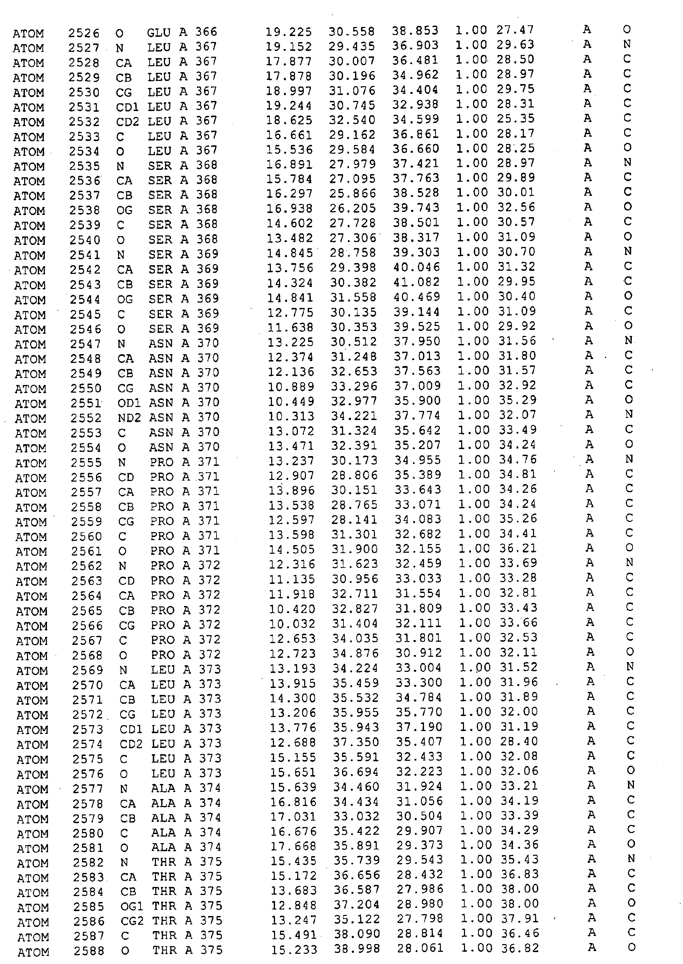

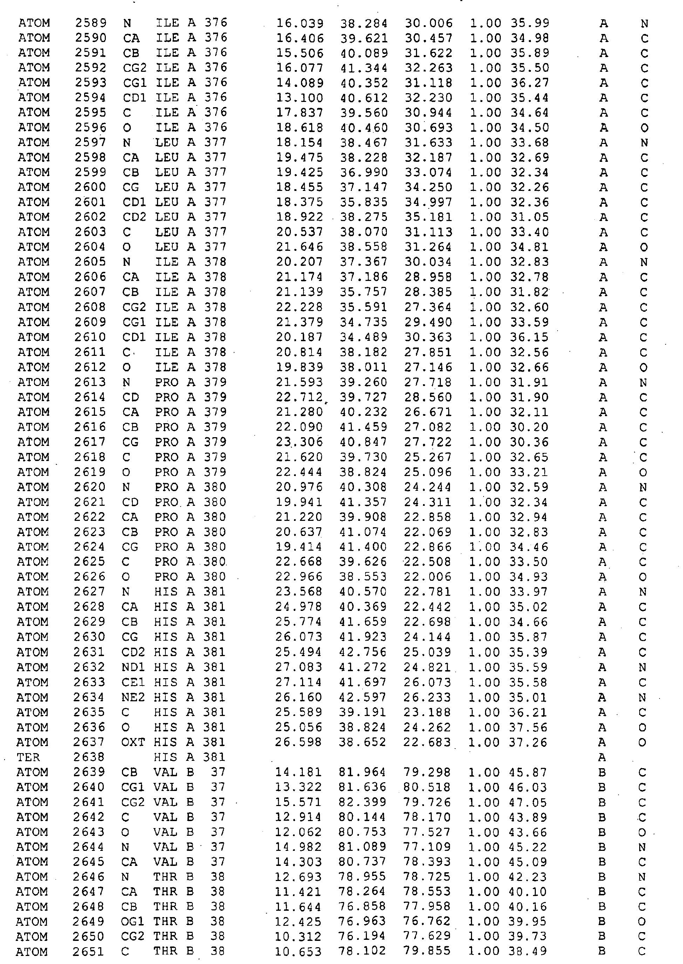

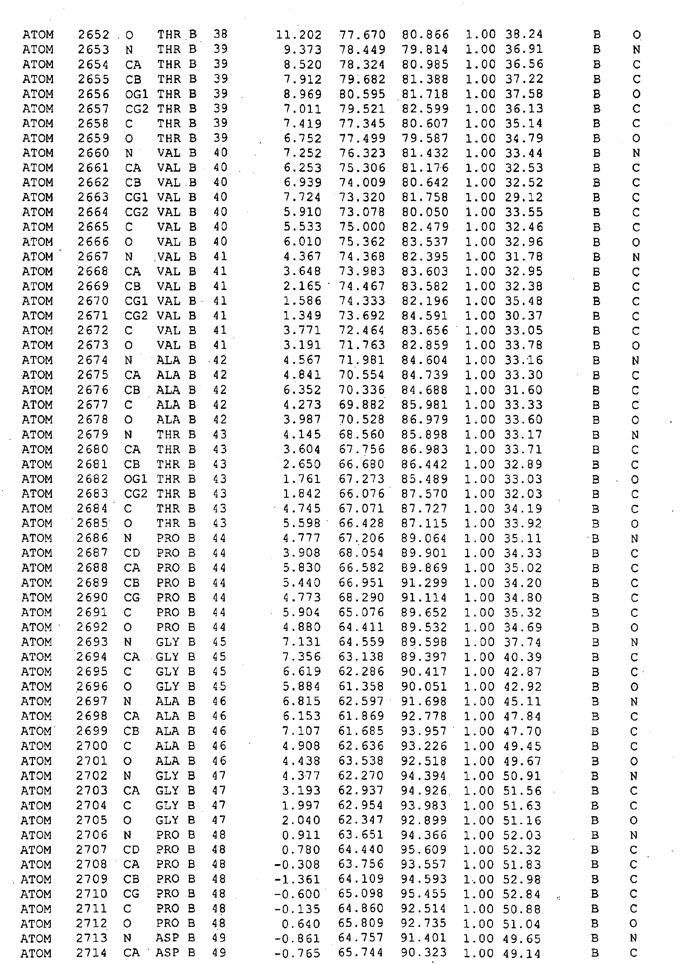

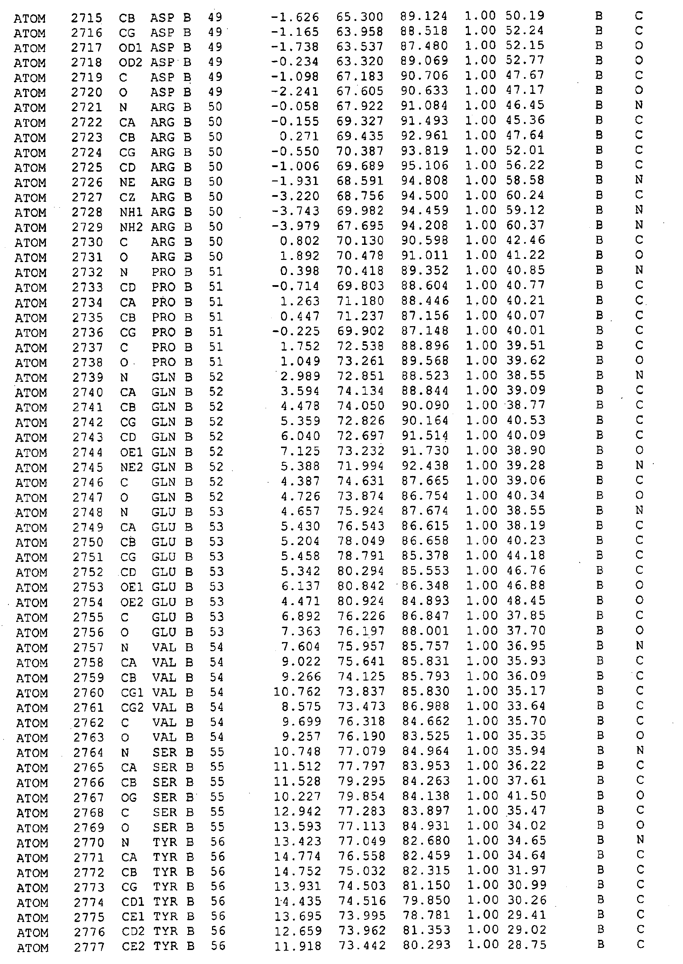

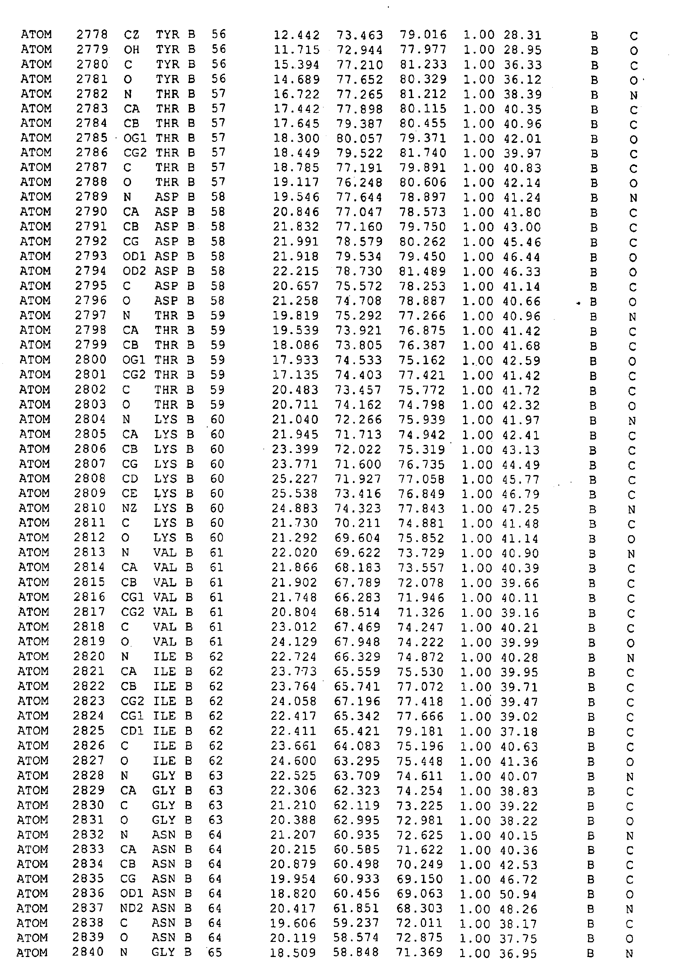

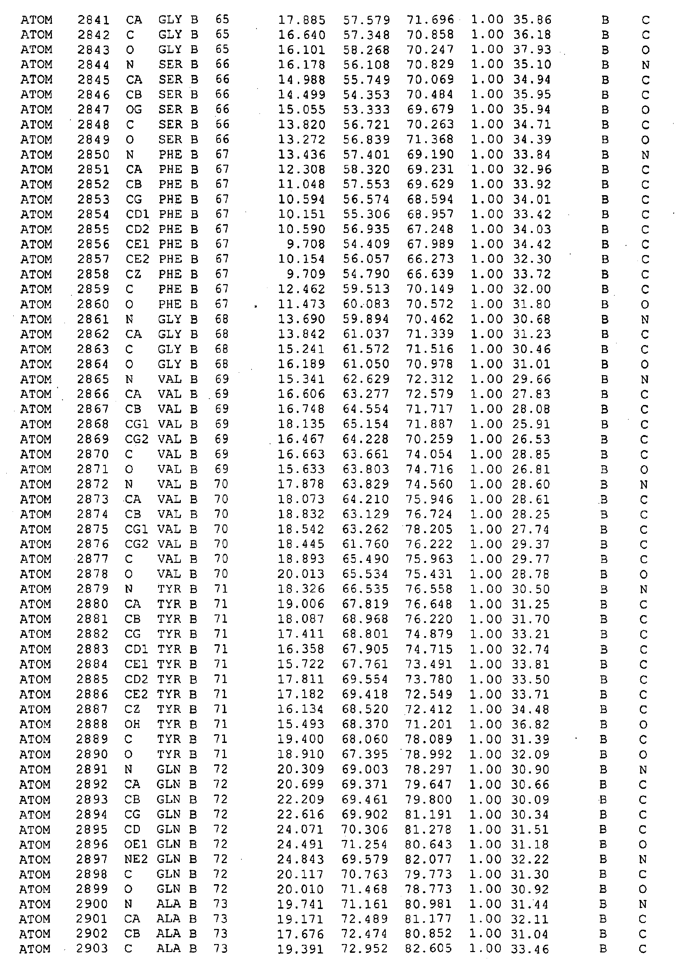

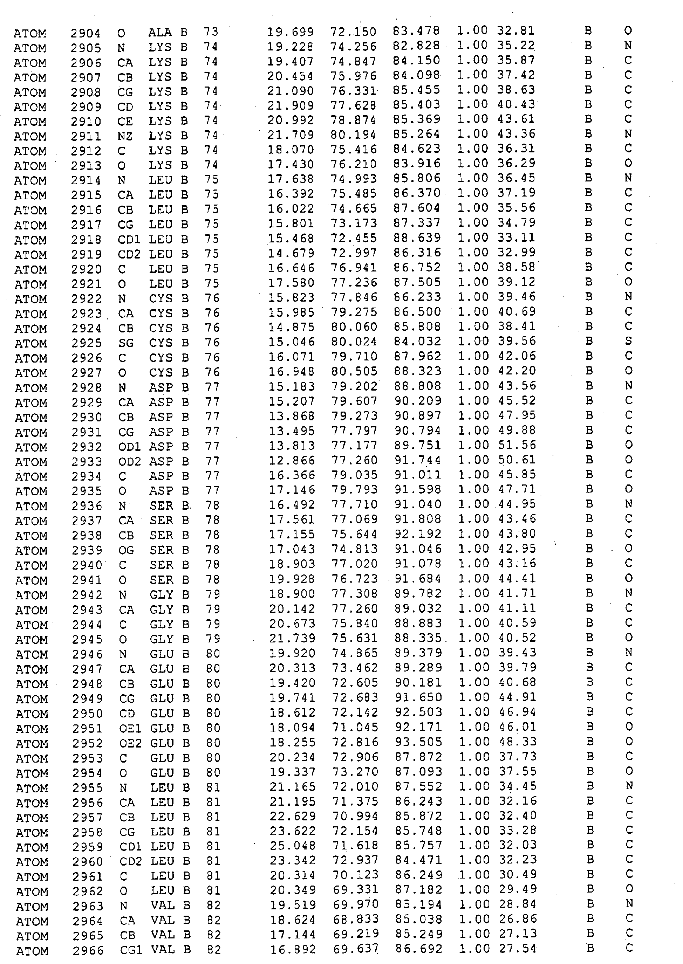

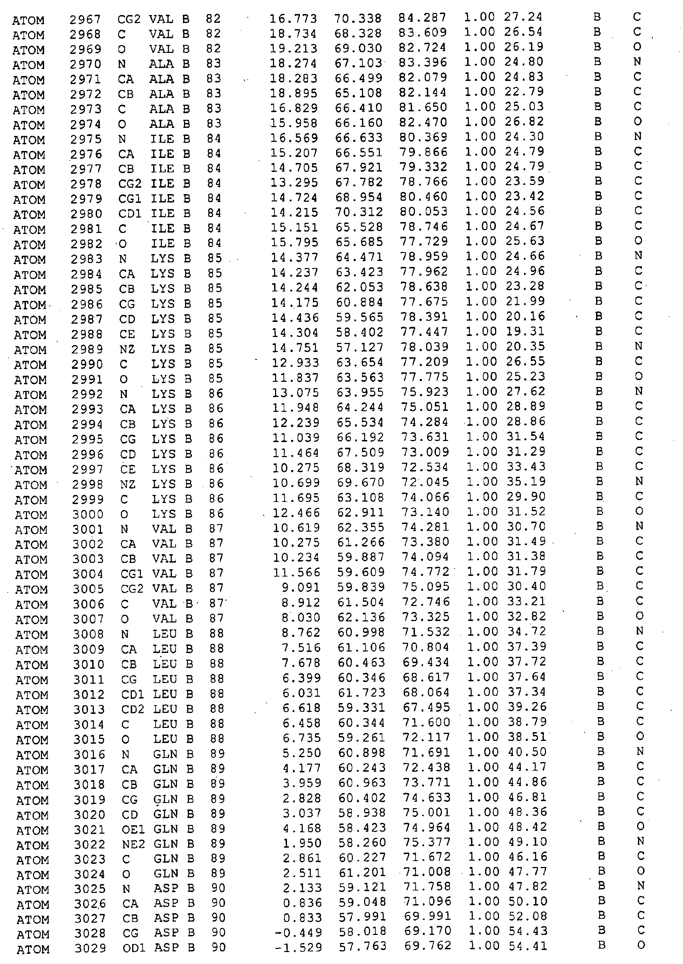

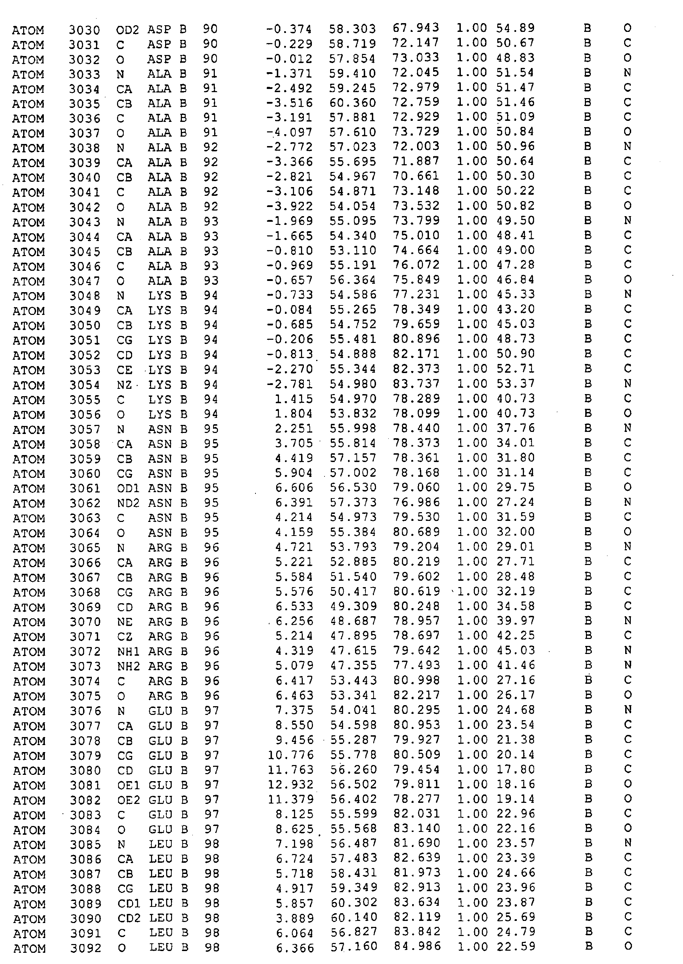

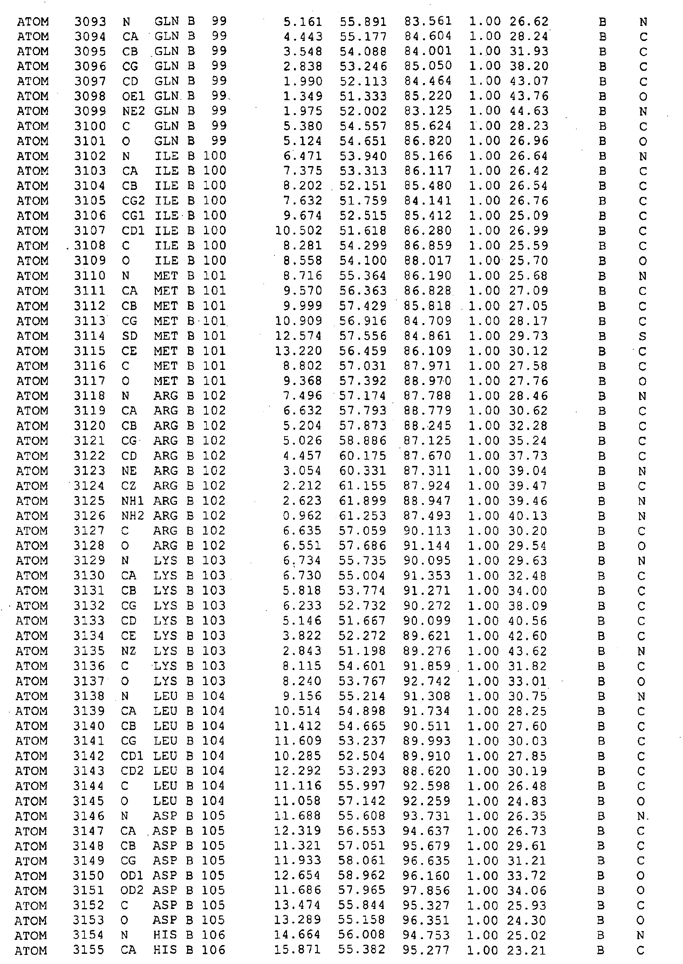

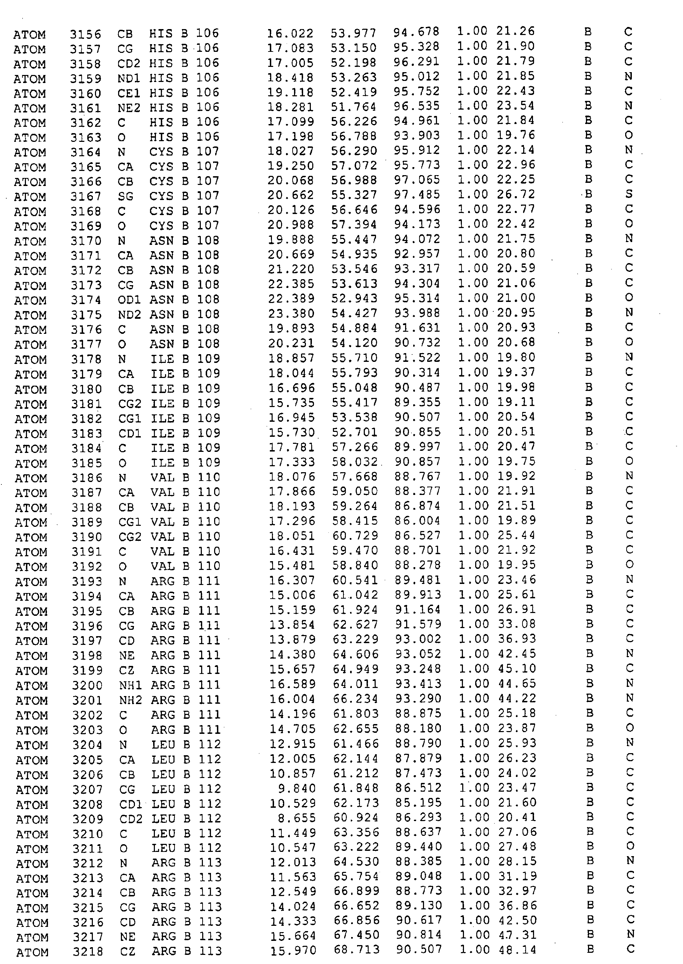

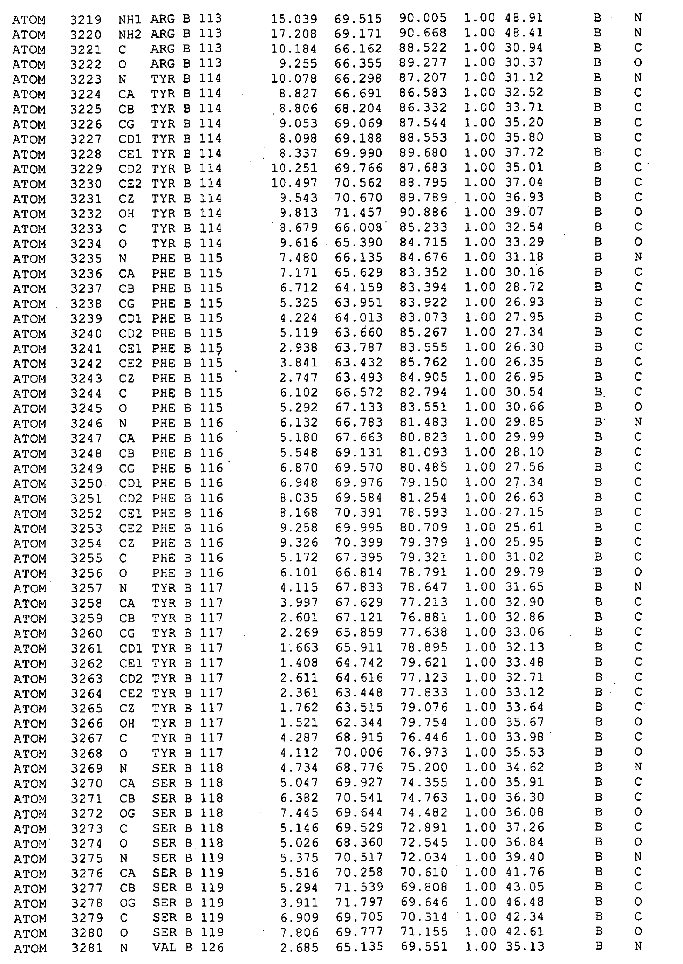

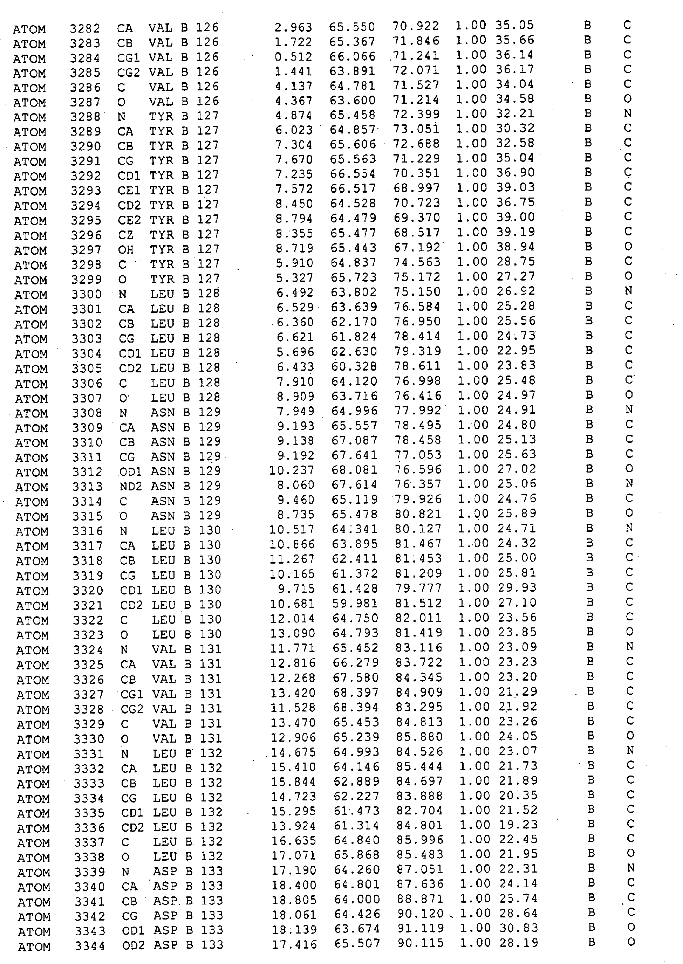

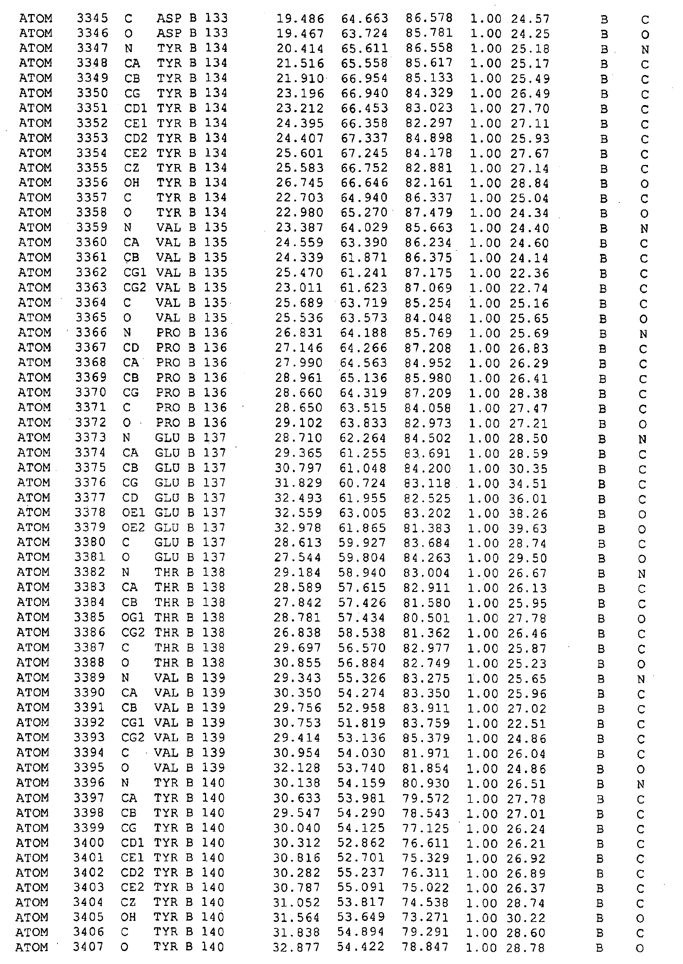

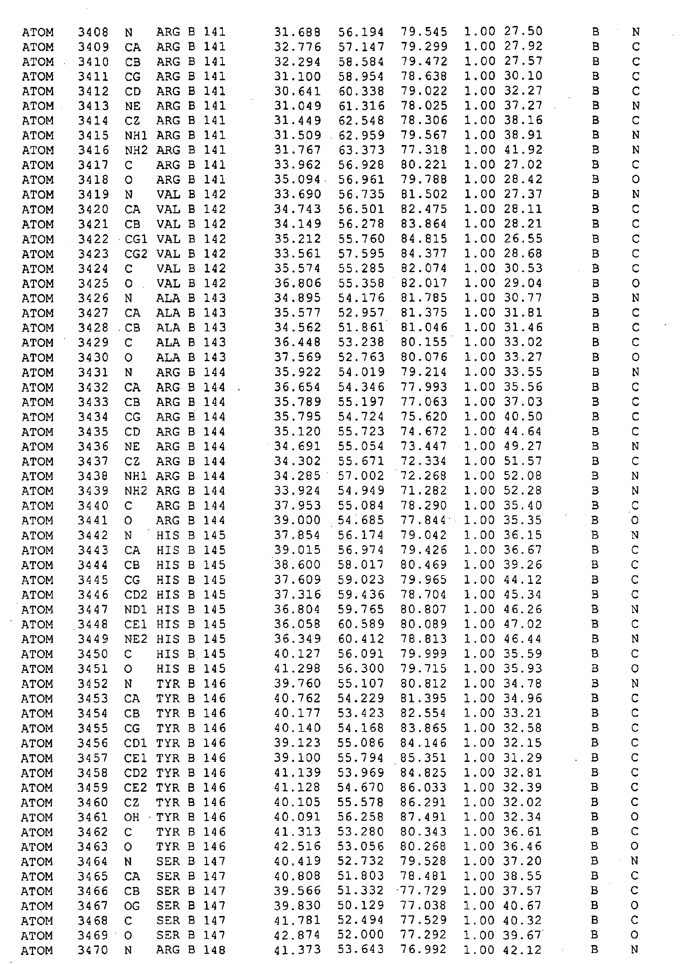

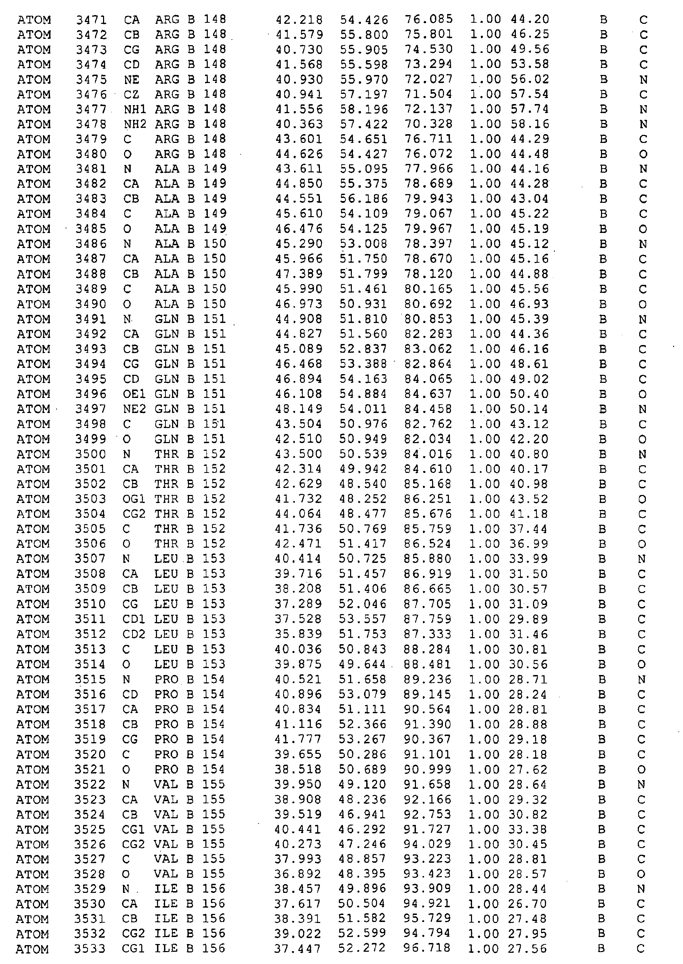

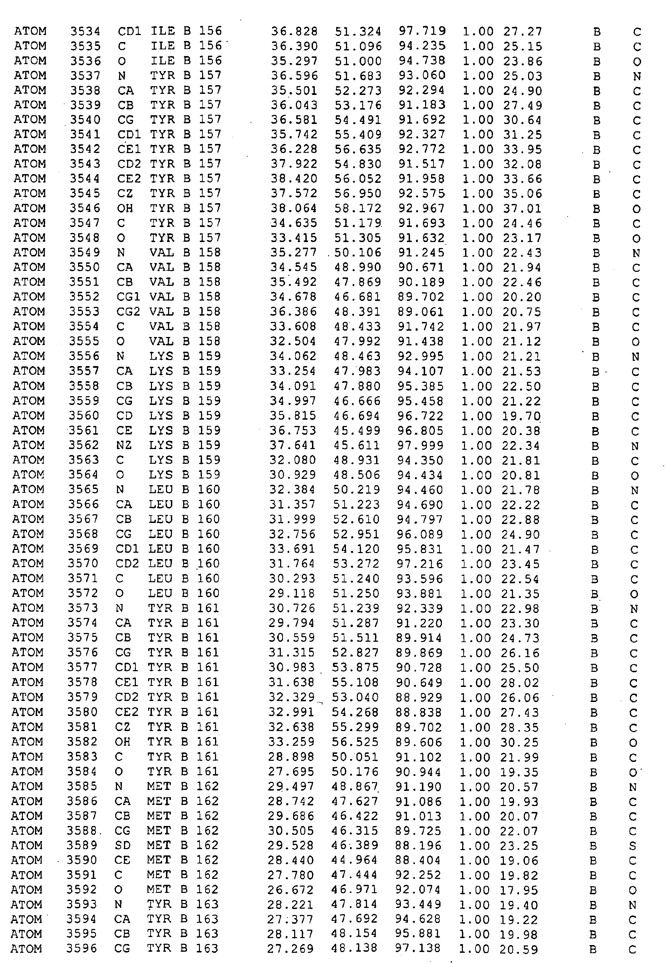

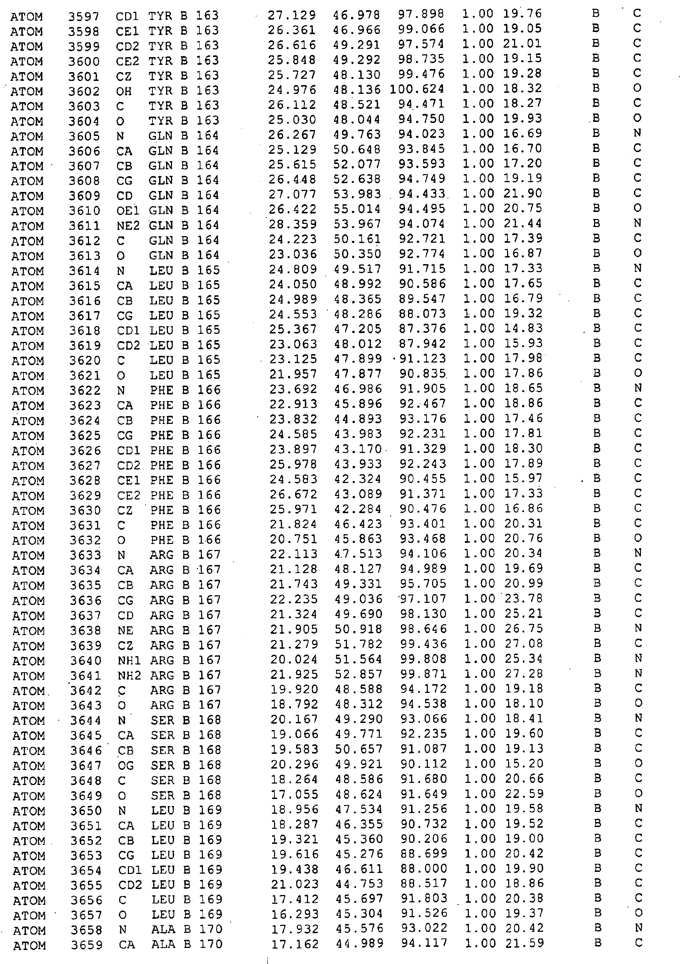

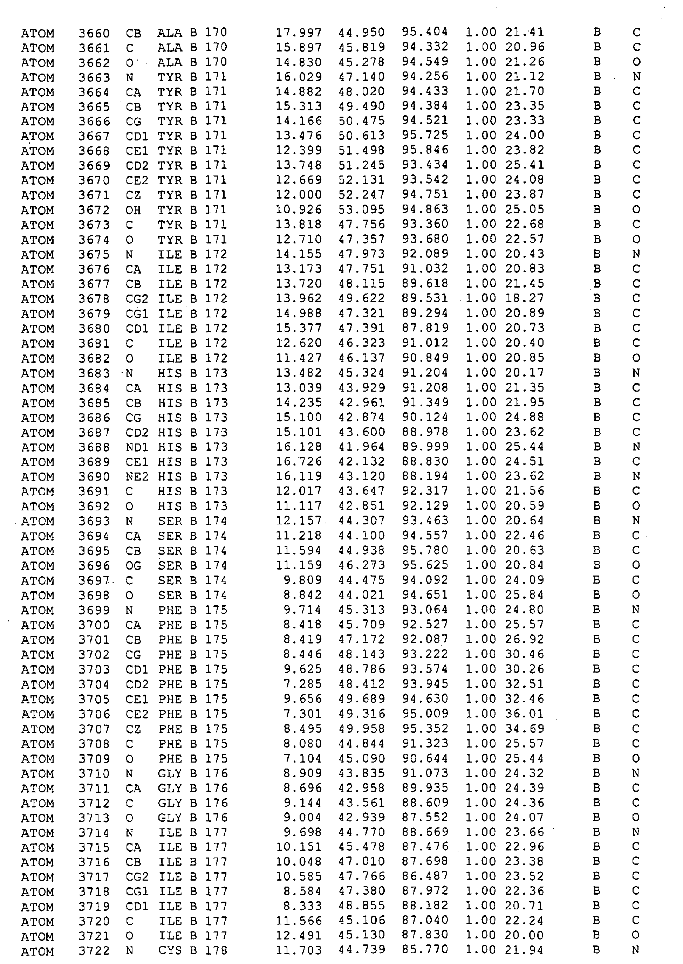

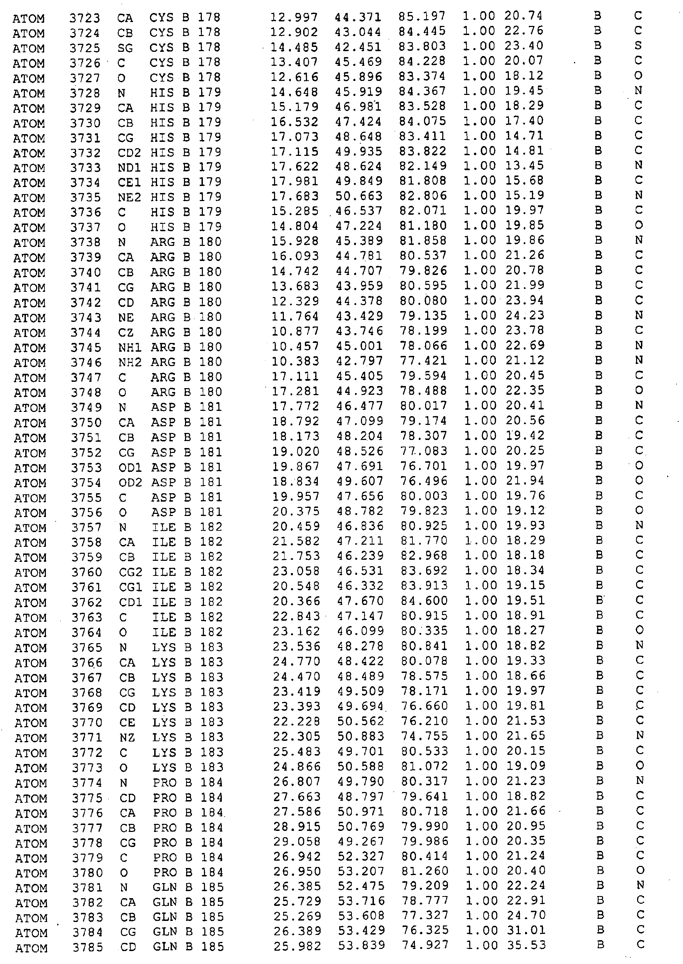

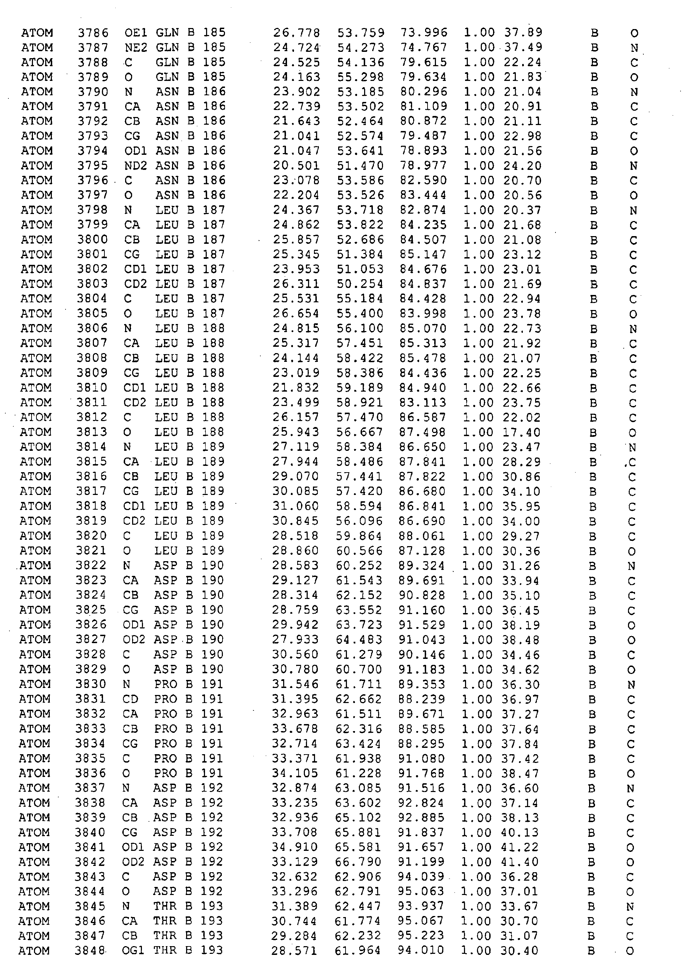

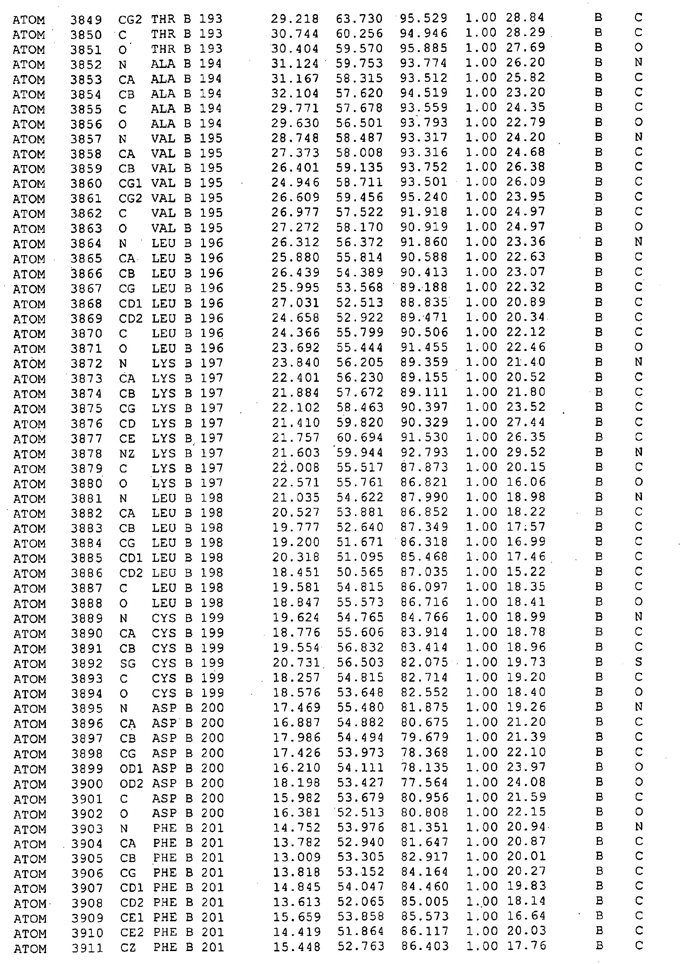

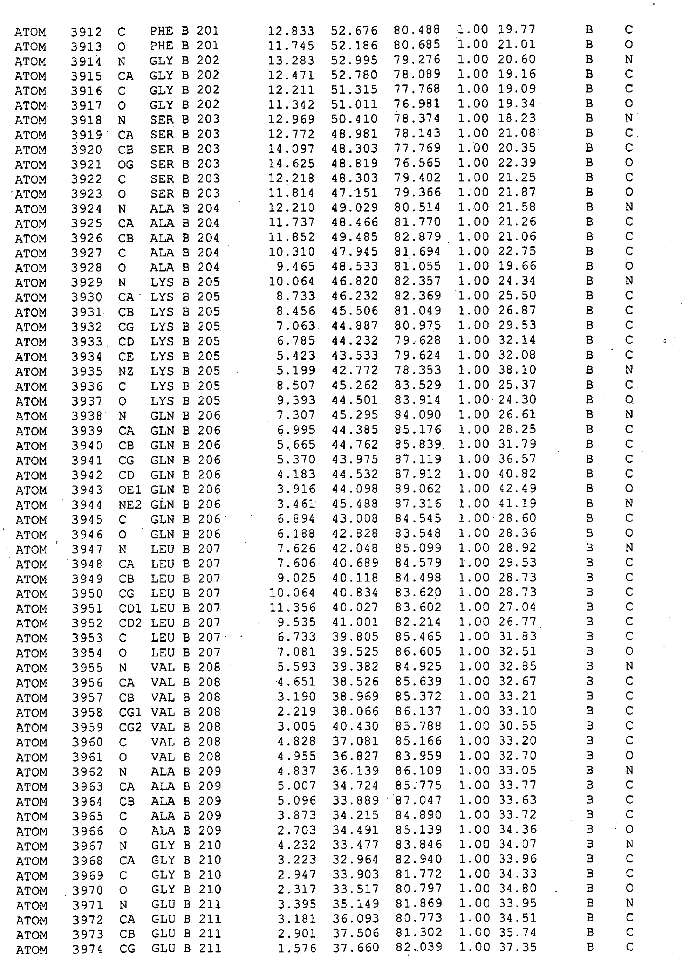

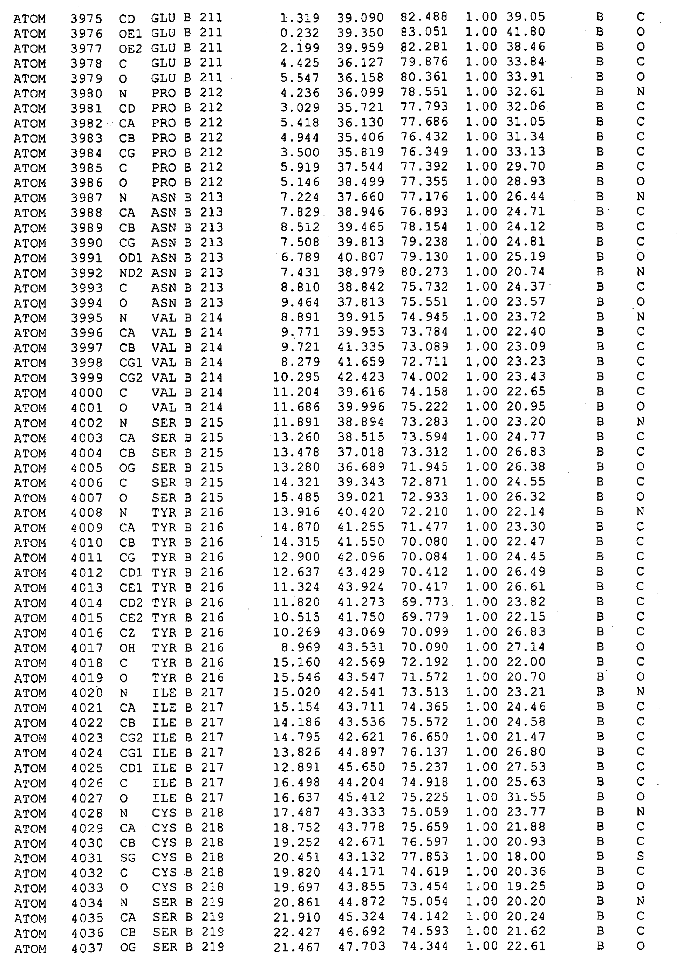

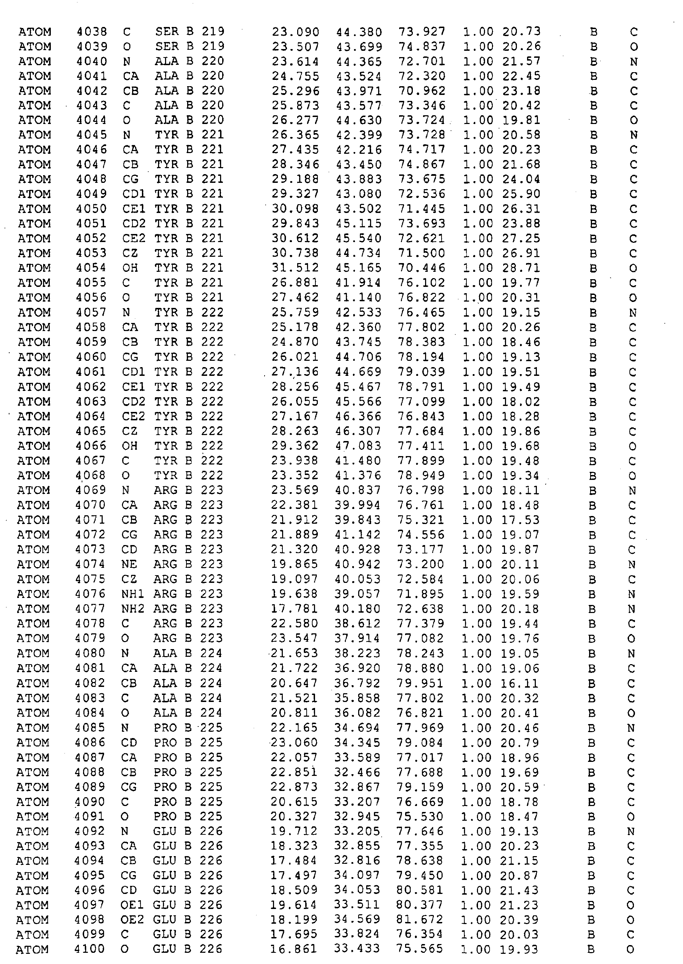

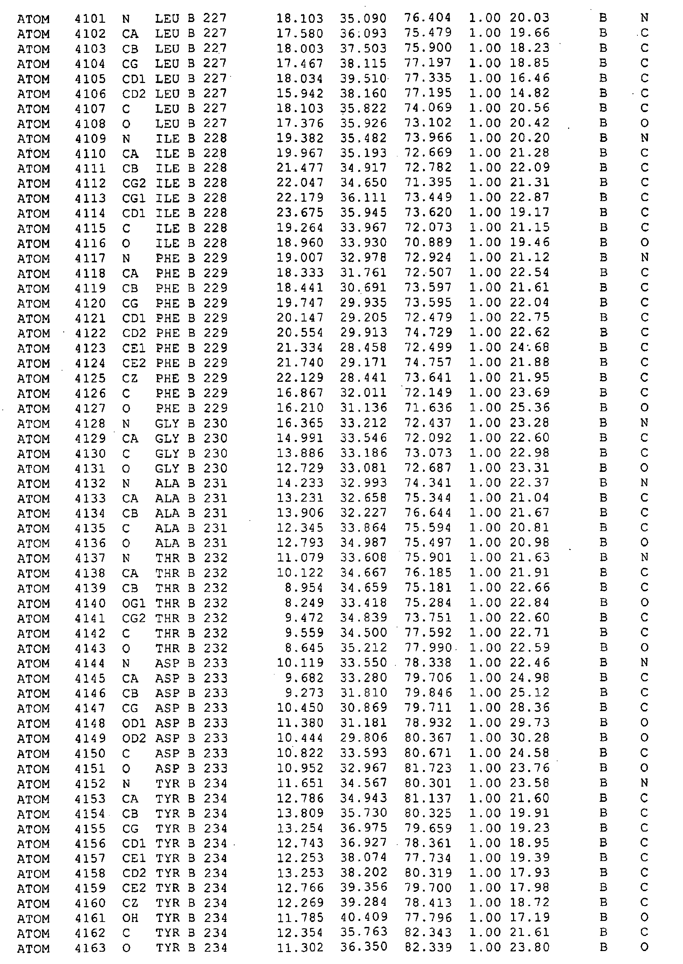

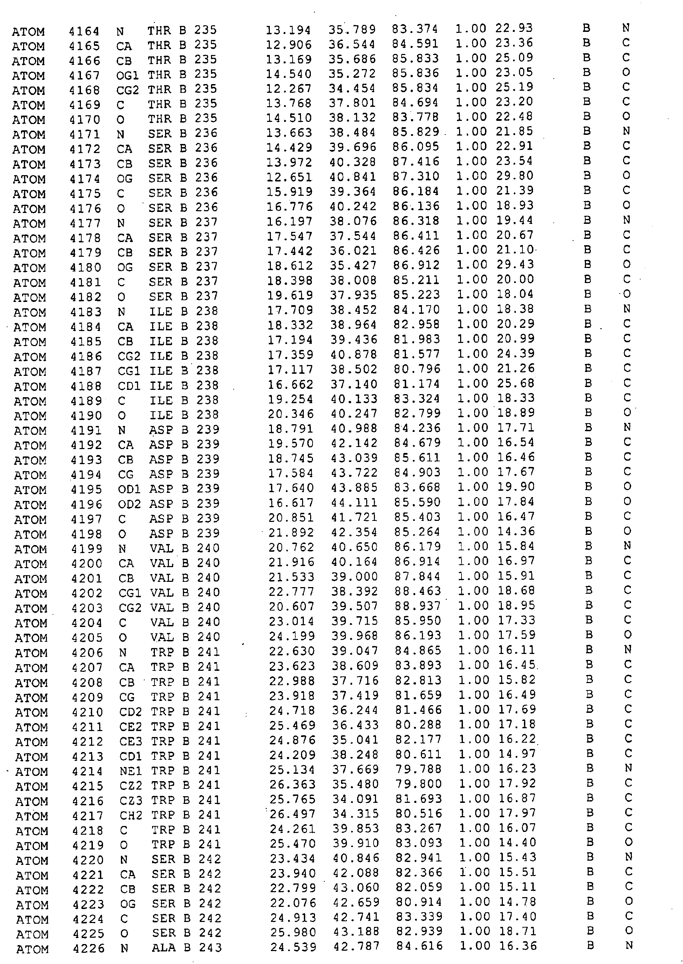

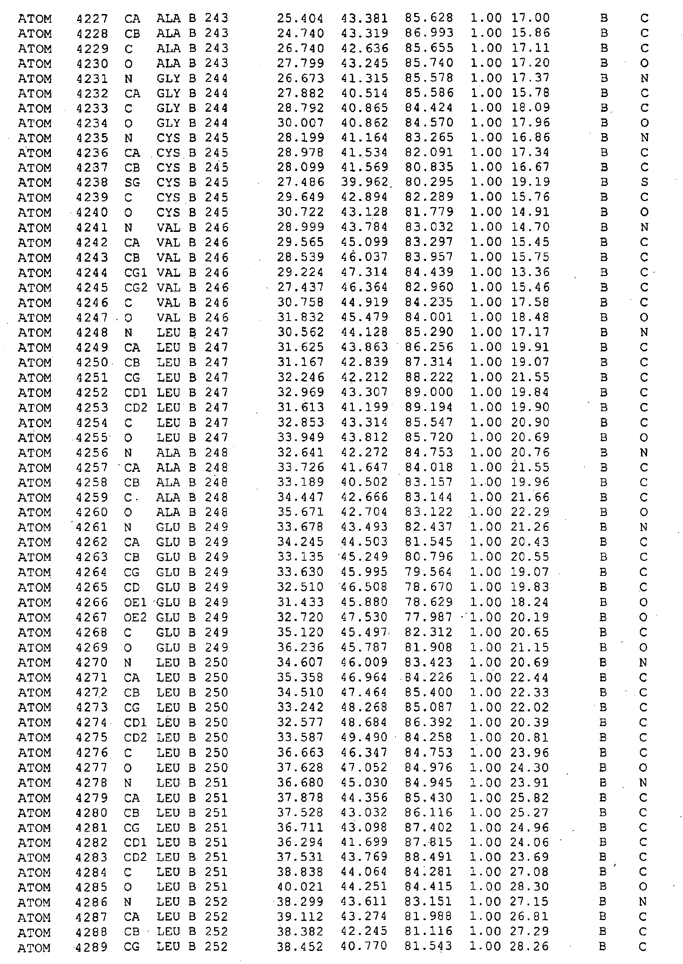

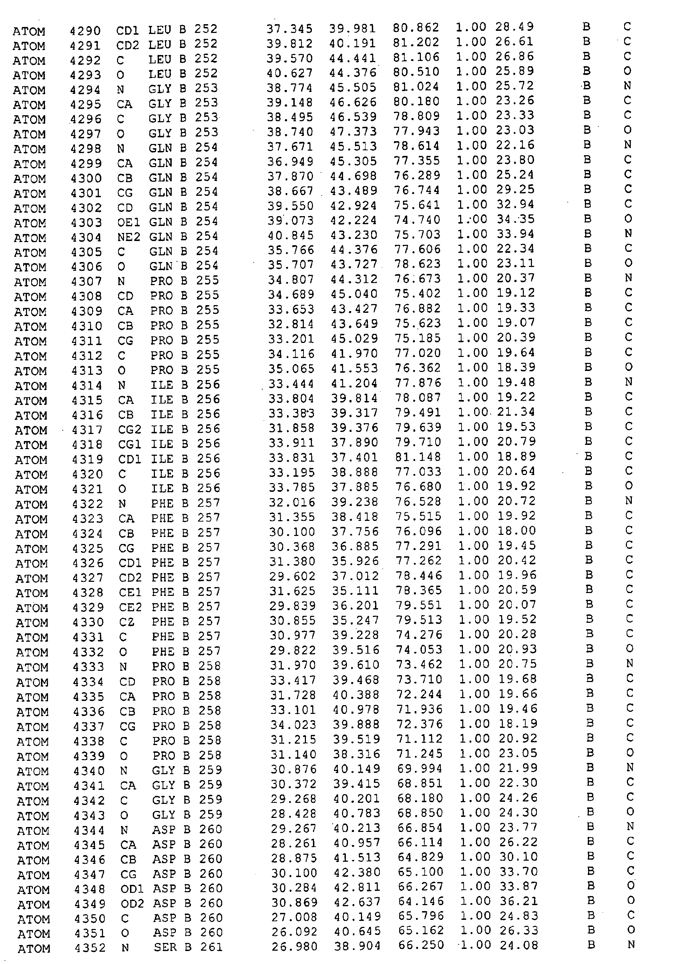

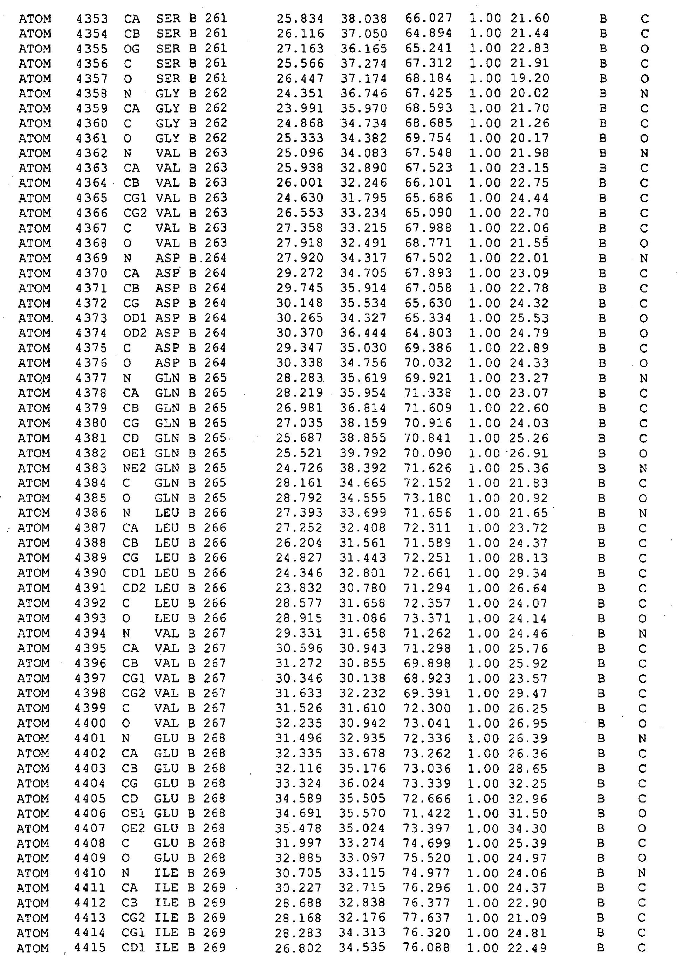

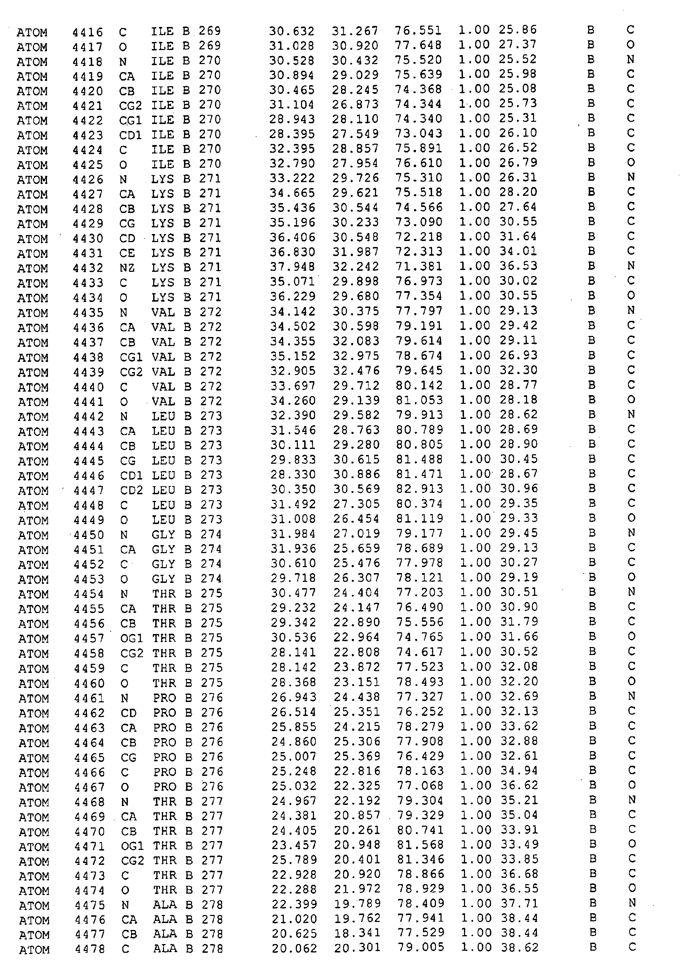

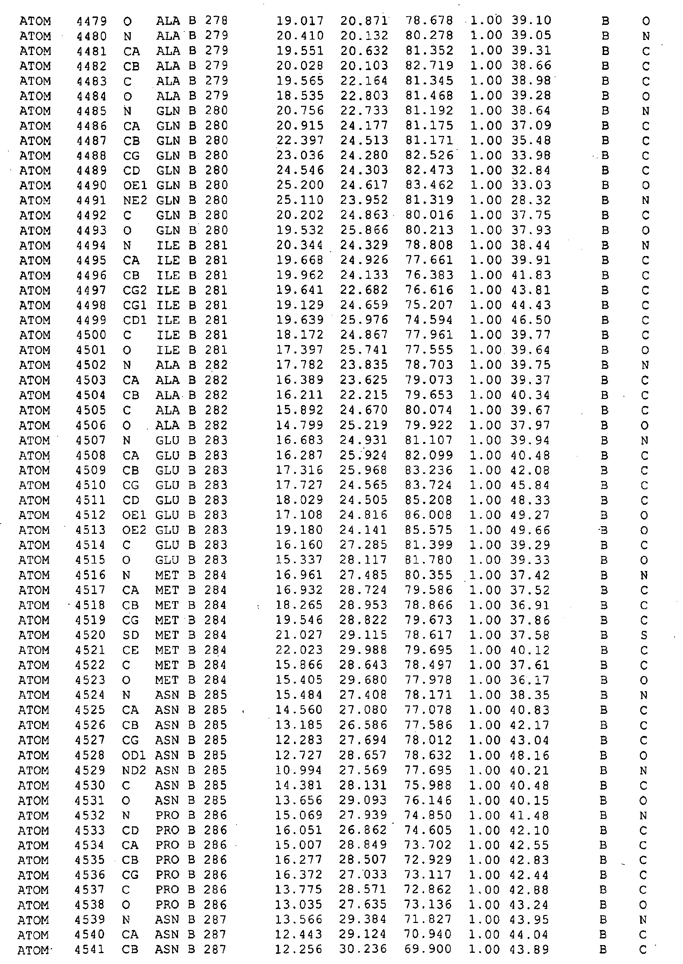

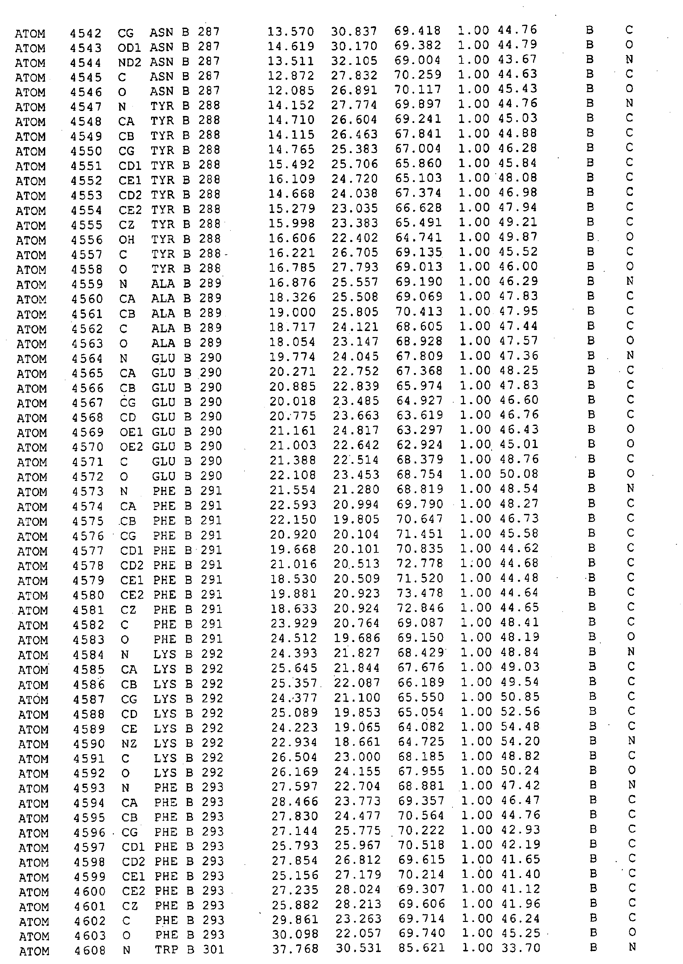

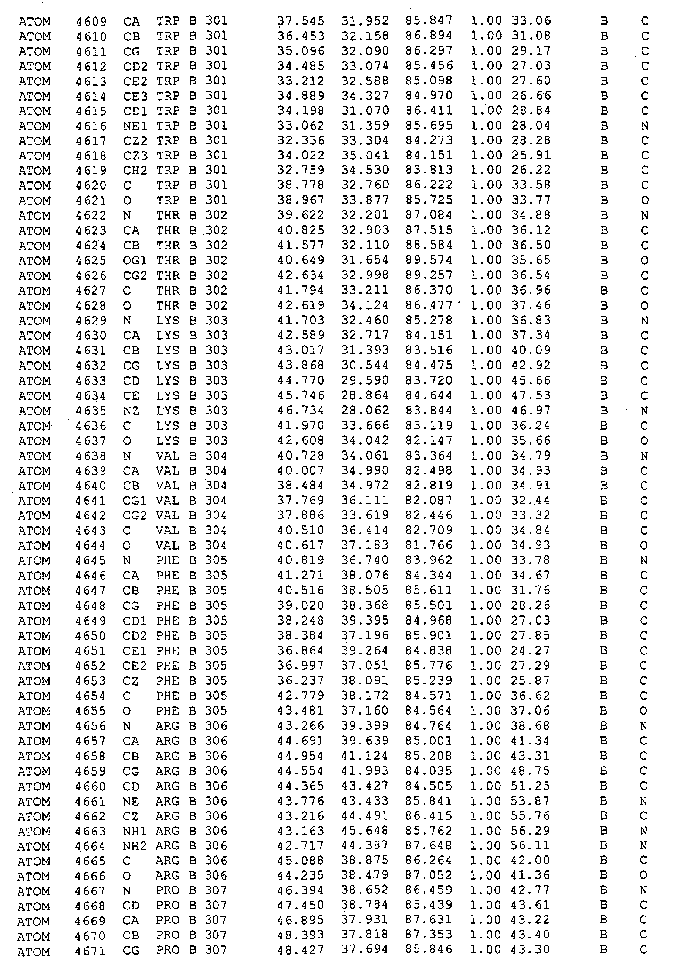

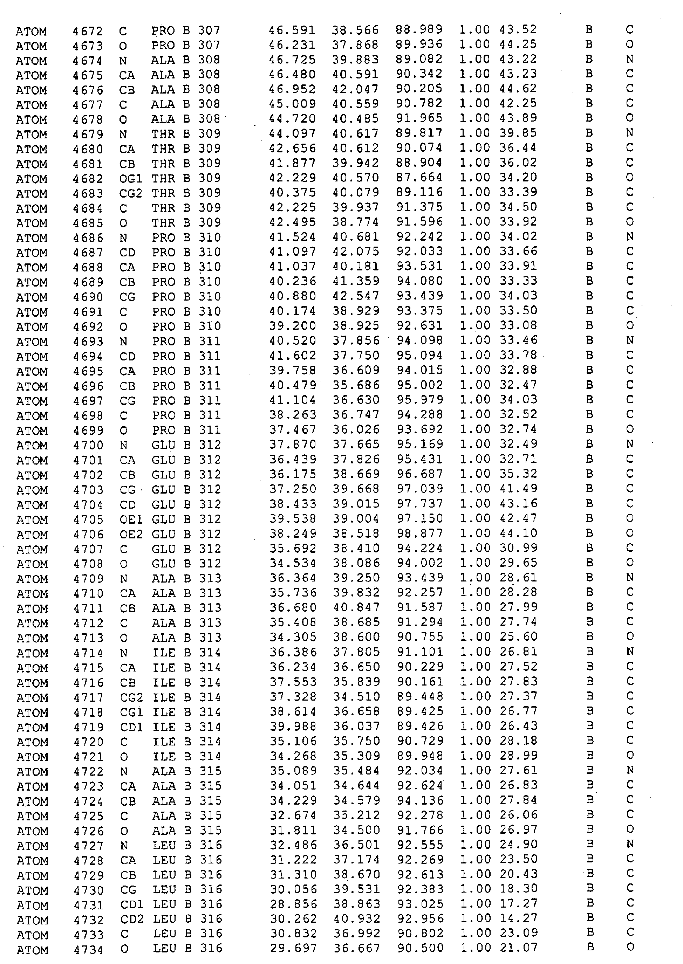

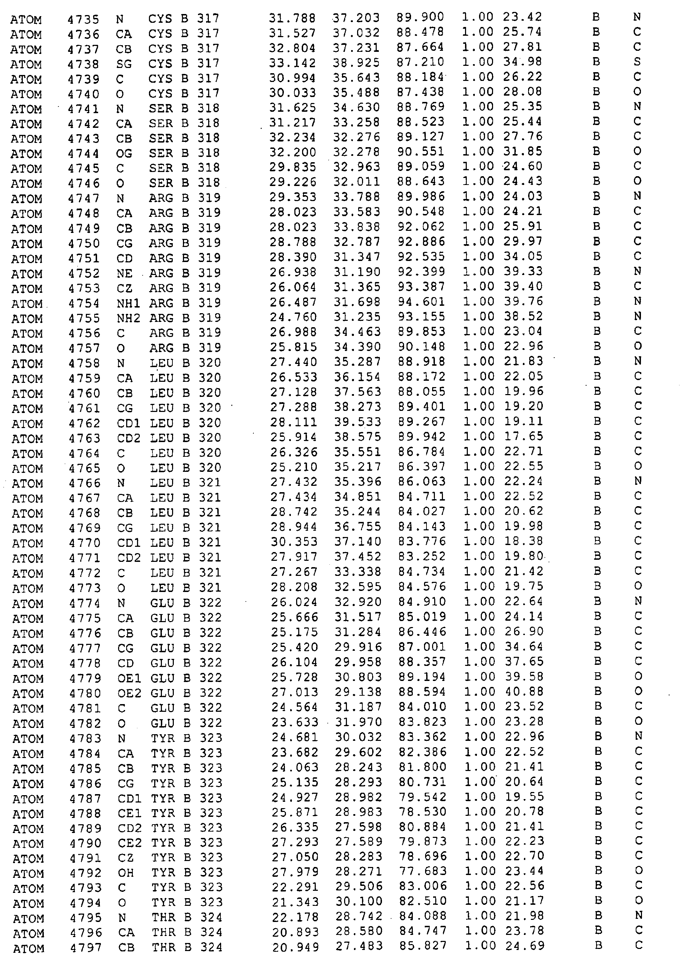

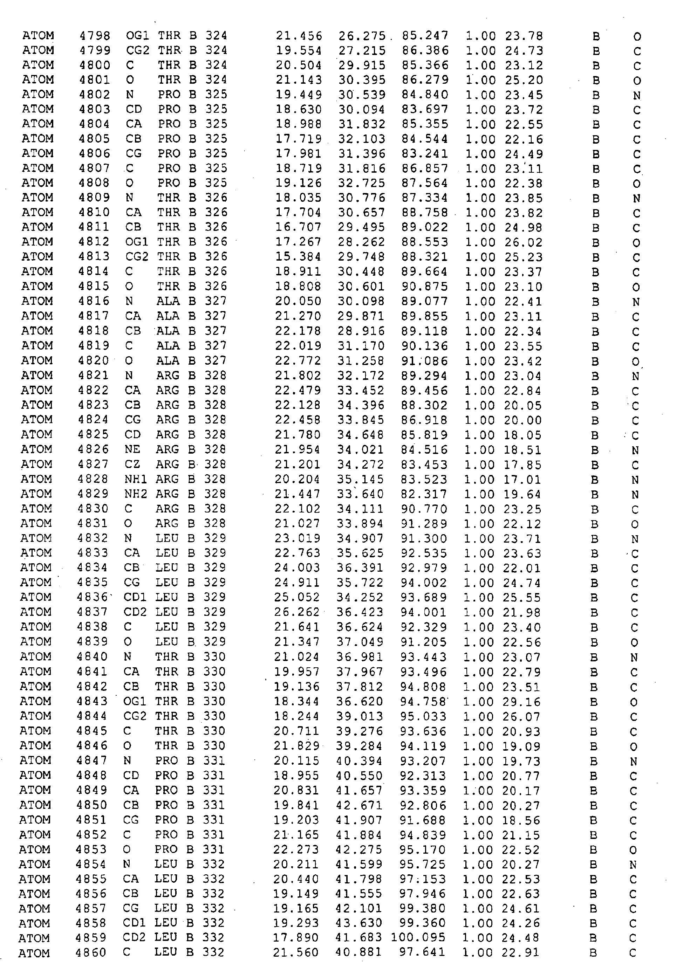

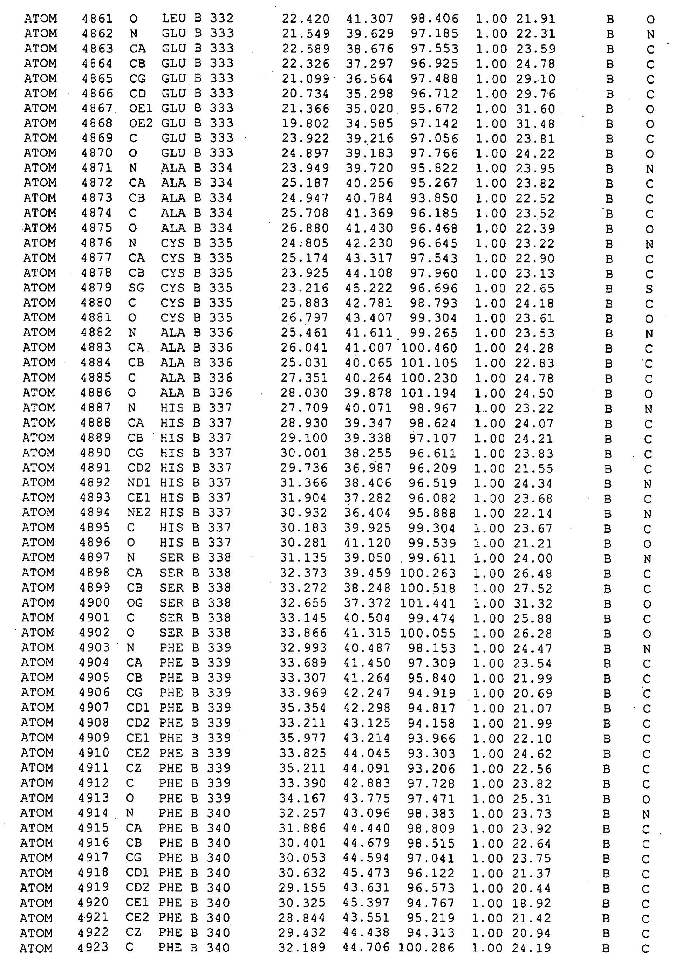

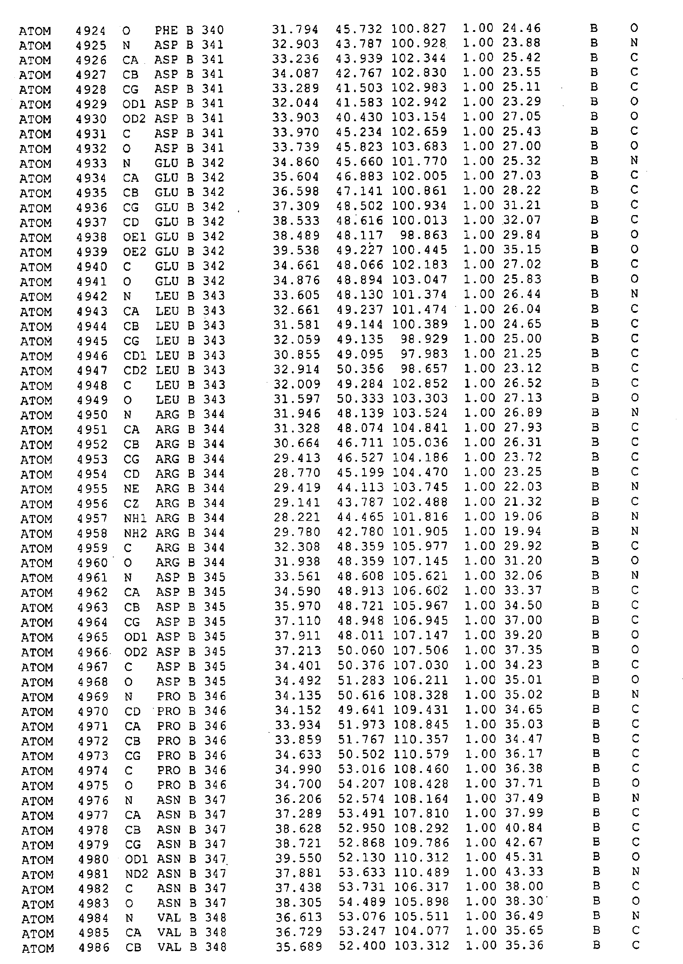

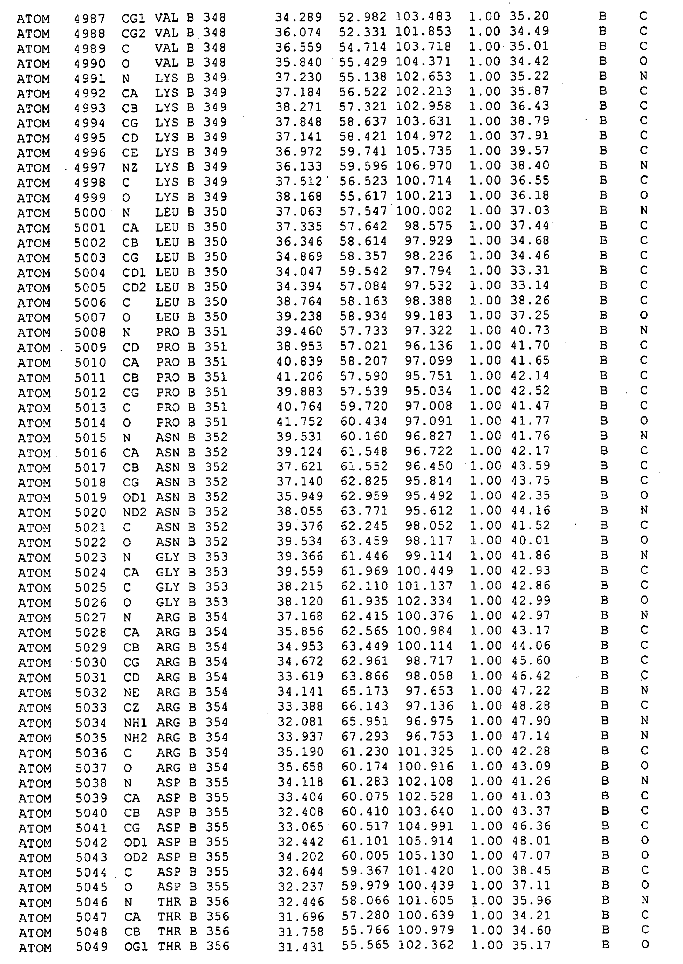

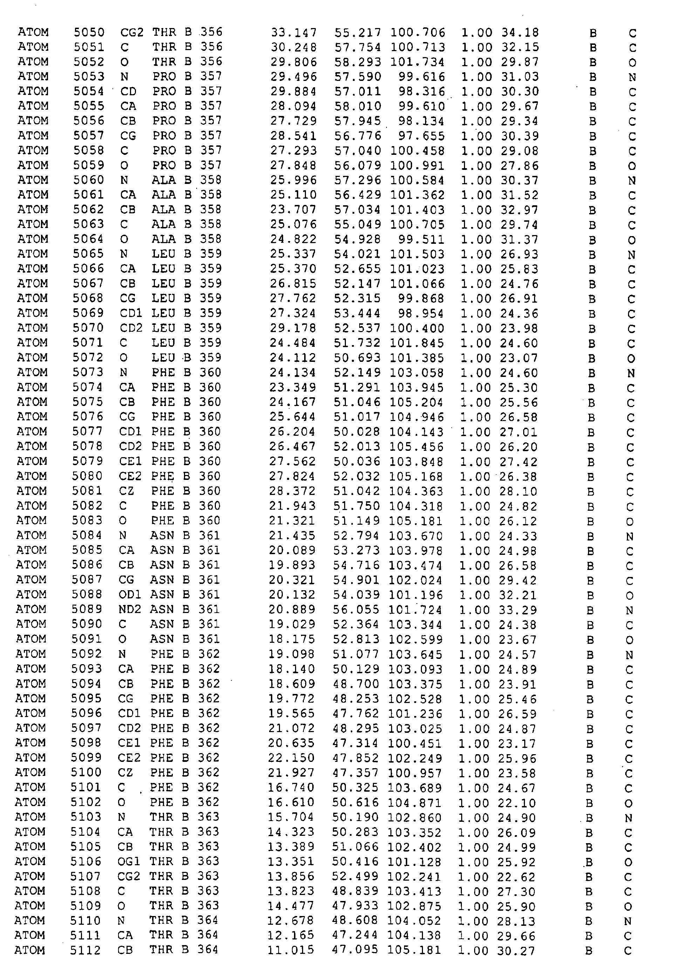

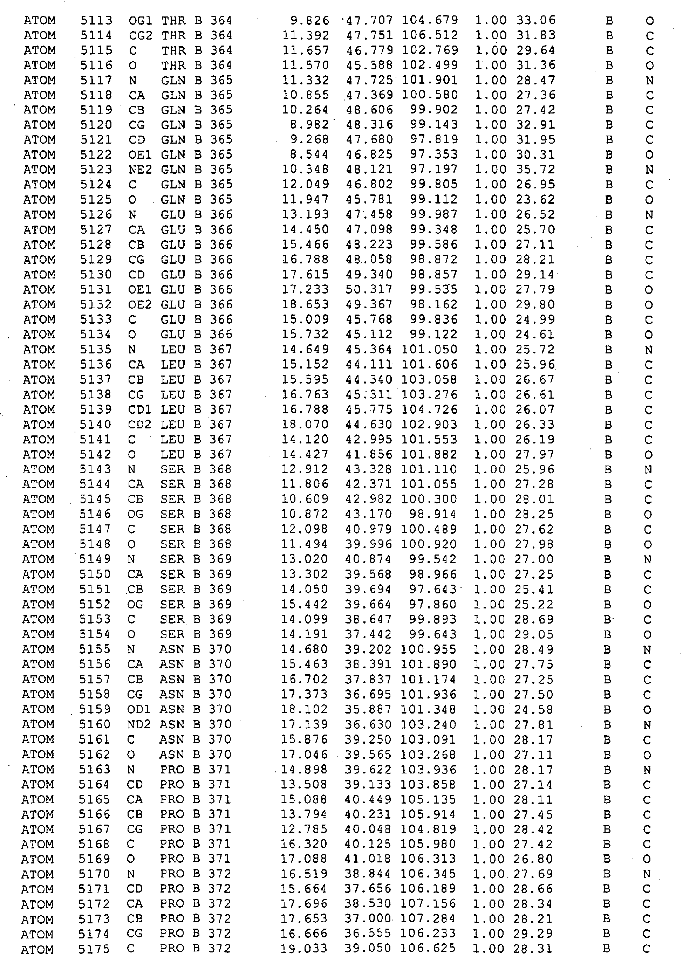

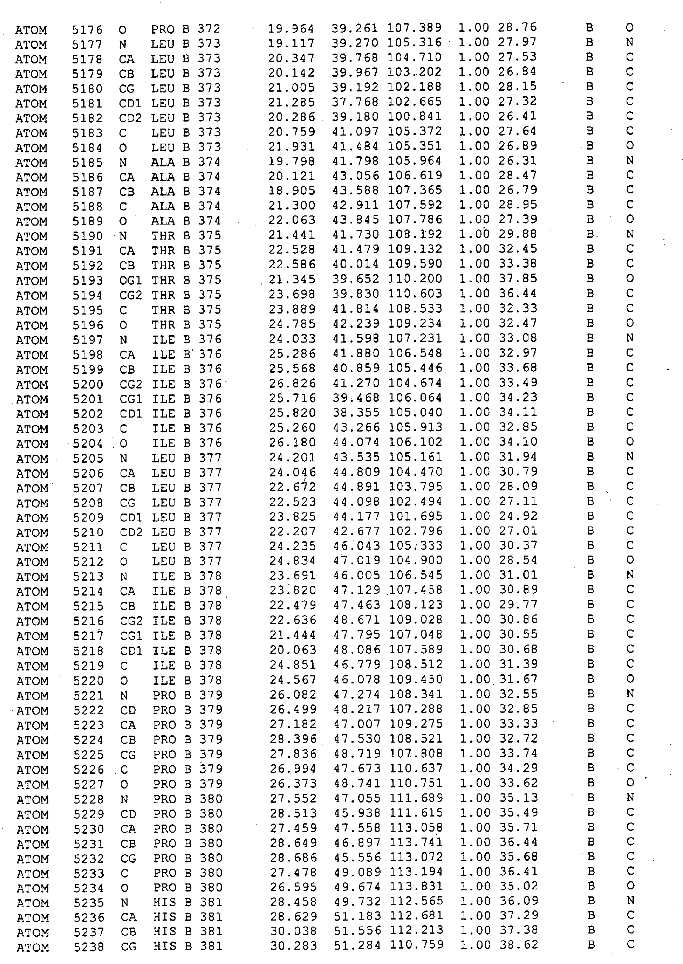

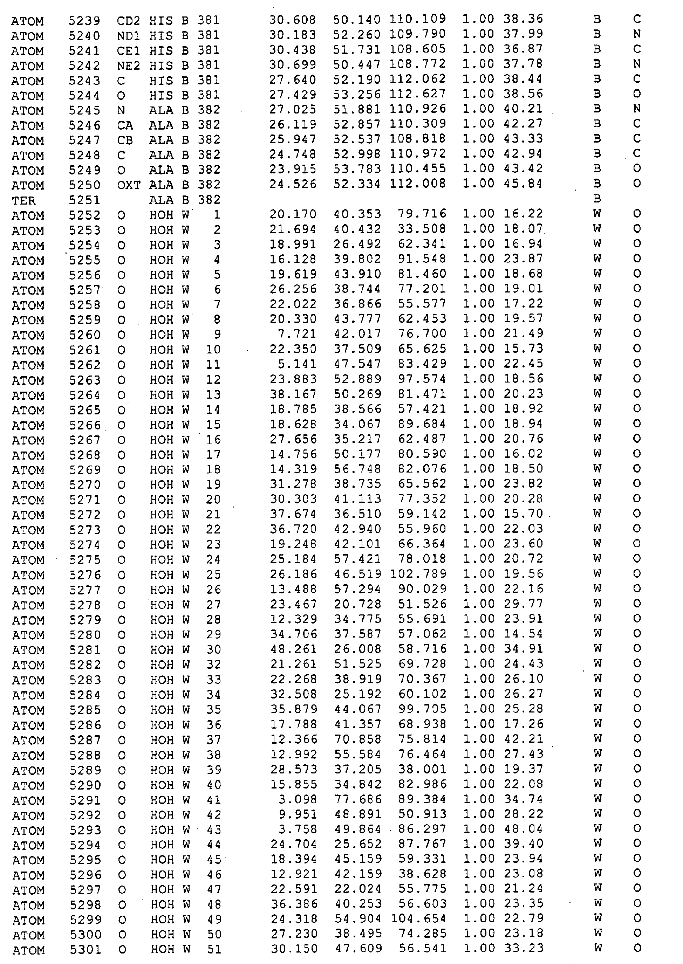

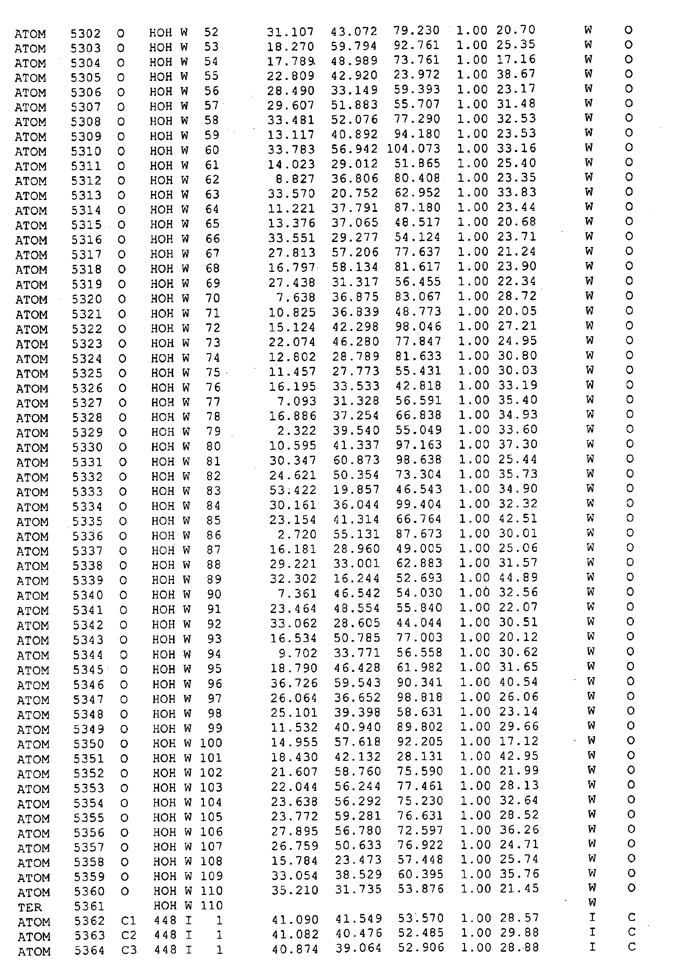

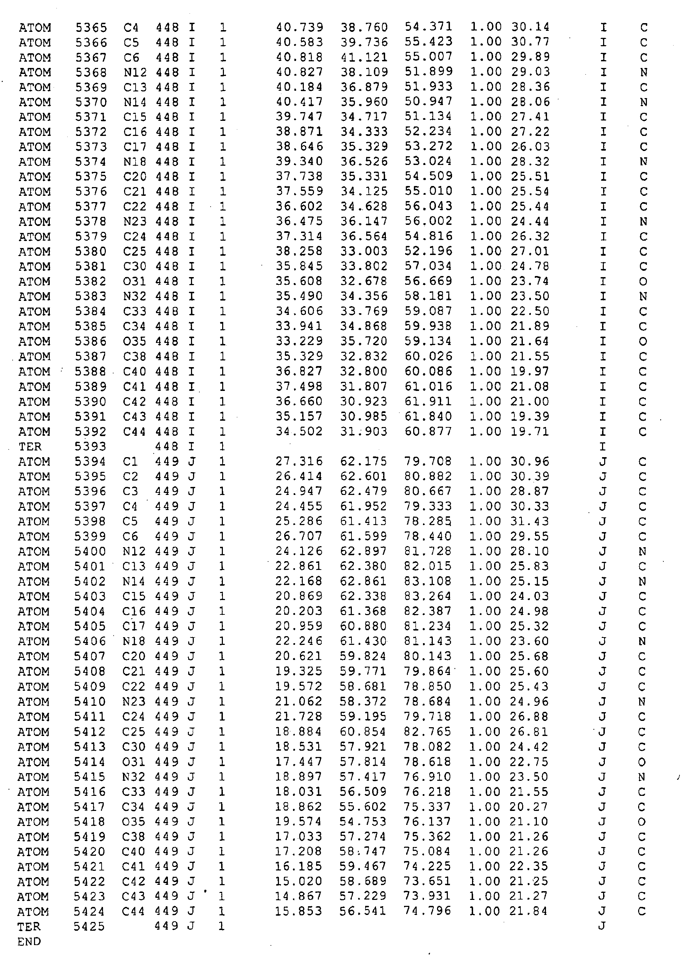

In the crystal structure of the unphosphorylated GSK-3β-inhibitor3 complex, amino acid residues I62, N64, G65, S66, F67, G68, V69, V70, A83, K85, K86, V87, E97, V110, L132, D133, Y134, V135, P136, E137, T138, R141, K183, Q185, N186, L188, C199 and D200 according to Figure 5 were within 5 Å of the inhibitor bound in the active site. These amino acid residues were identified using the programs QUANTA, O and RIBBONS. In addition, amino acid residues V61, I62, G63, N64, G65, S66, F67, G68, V69, V70, Y71, Q72, L81, V82, A83, I84, K85, K86, V87, L88, E97, M101, V110, R111, L130, V131, L132, D133, Y134, V135, P136, E137, T138, V139, Y140, R141, H179, D181, K183, Q185, N186, L187, L188, L189, K197, L198, C199, D200, F201, G202, S203 and S219 according to Figure 5 were within 8 Å of the inhibitor bound in the active site. These amino acid residues were identified using the programs QUANTA, O and RIBBON.

In the crystal structure of the unphosphorylated GSK-3β-inhibitor4 complex, amino acid residues I62, G63, N64, F67, V70, A83, V110, L132, D133, Y134, V135, P136, E137, T138, R141, Q185, N186, L188, C199 and D200 according to Figure 6 were within 5 Å of the inhibitor bound in the active site. These amino acid residues were identified using the programs QUANTA, O and RIBBONS. In addition, applicants have determined that amino acid residues V61, I62, G63, N64, G65, S66, F67, G68, V69, V70, Y71, Q72, L81, V82, A83, I84, K85, E97, M101, V110, R111, L130, V131, L132, D133, Y134, V135, P136, E137, T138, V139, Y140, R141, D181, K183, P184, Q185, N186, L187, L188, L189, K197, L198, C199, D200 and F201 according to Figure 6 were within 8 Å of the inhibitor bound in the active site.

These amino acid residues were identified using the programs QUANTA, O and RIBBONS.

Using a multiple alignment program to compare the unphosphorylated GSK-3β structure and structures of other members of the protein kinase family, amino acid residues Y56, T59, K60, V61, V69, V70, Y71, Q72, A73, K74, L75, L81, V82, A83, I84, K85, K86, L98, M101, R102, L104, H106, C107, N108, I109, V110, R111, L112, R113, Y114, F115, F116, L128, N129, L130, V131, L132, D133, Y134, V135, P136, E137, T138, V139, Y140, R141, V142, P154, V155, I156, Y157, V158, K159, L160, Y161, M162, Y163, Q164, L165, F166, R167, S168, L169, A170, Y171, I172, H173, S174, F175, G176, I177, C178, H179, R180, D181, I182, K183, P184, Q185, N186, L187, L188, L189, D190, P191, A194, V195, L196, K197, L198, C199 and D200 according to Figure 1 were identified as the ATP-binding pocket (Gerstein et al., J. Mol. Biol., 251, pp. 161-175 (1995), incorporated herein by reference). To perform this comparison, first, a sequence alignment between members of the protein kinase family was performed. Second, a putative core was constructed by superimposing a series of corresponding structures in the protein kinase family. Third, residues of high spatial variation were discarded, and the core alignment was iteratively refined. The amino acids that make up the final core structure have low structural variance and have similar local and global conformation relative to the corresponding residues in the protein family.

In the crystal structure of the phosphorylated GSK-3β-ADP-peptide complex, amino acids residues G65, S66, F67, D90, K91, R92, F93, K94, R96, R180, D181, K183, G202, S203, K205, P212, N213, V214, Y216, 1217, C218, S219, R223, Y234 according to Figure 7 were within 5 Å of the peptide bound in the substrate binding groove. These amino acid residues were identified using the programs QUANTA, O and RIBBONS, supra. Amino acid residues D90, K91, R92, F93, K94 made backbone interactions with the peptide substrate. Amino acid residues R96, R180, K205, N213, Y234 bound to pS656 of the peptide substrate. Amino acid residues R96, R180 and K205 formed a positively charged binding pocket surrounding the pS656. Amino acid residue S66 bound to the backbone of amino acid residue S652 from the peptide substrate. Amino acid residues F67, F93 form an aromatic binding pocket surrounding the peptide substrate amino acid residue H650.

In the crystal structure of the phosphorylated GSK-3β-ADP-peptide complex, amino acid residues N64, G65, S66, F67, G68, V87, L88, D90, K91, R92, F93, K94, N95, R96, E97, R180, D181, I182, K183 Q185, N186, D200, F201, G202, S203, A204, K205, Q206, L207, E211, P212, N213, V214, S215, Y216, I217, C218, S219, R220, Y221, Y222, R223, L227, T232 and Y234 according to Figure 5 were within 8 Å of the peptide bound in the substrate binding groove. These amino acid residues were identified using the programs QUANTA, O and RIBBONS, *supra*. Amino acid residues Y216, I217, C218, S219, R220 and R223 form a binding pocket that accommodates the proline side chain of the peptide substrate.

In the GSK-3β-ADP-peptide electron density map, the side chains of residues F67, K91, and R92 in the substrate binding pocket could not be located. Alanine and glycine residues were used to build the structure model at these positions. For the purpose of this invention, the structure coordinates of amino acid residues F67, K91 and R92 refer to the structure coordinates of amino acid residues A67, A91 and G92 in Figure 7, respectively. In Figures 1-7, where alanine or glycine residues were built in the model as a result of missing side chains in the electron density map, the same applies to those residues.

In one embodiment, the inhibitor-binding pocket comprises GSK-3β amino acid residues K85, M101, V110, L130 and L132 according to any one of Figures 1-7. In another embodiment, the inhibitor-binding pocket comprises GSK-3βamino acid residues I62, V135, P136, T138 and L188 according to any one of Figures 1-7. In another embodiment, the inhibitor-binding pocket comprises GSK-3β amino acid residues V70, V110, L188 and C199 according to any one of Figures 1-7. In another embodiment, the inhibitor-binding pocket comprises GSK-3β amino acids F67, V70, Q185 and C199 according to any one of Figures 1-7.

In one embodiment the inhibitor-binding pocket comprises amino acid residues V61, I62, G63, N64, G65, S66, F67, G68, V69, V70, Y71, Q72, L81, V82, A83, I84, K85, K86, V87, L88, E97, M101, V110, R111, L112, L130, V131, L132, D133, Y134, V135, P136, E137, T138, V139, Y140, R141, H179, D181, K183, P184, Q185, N186, L187, L188, L189, K197, L198, C199, D200, F201, G202, S203 and S219 according to any one of Figures 1-7.

In another embodiment, the ATP-binding pocket comprises amino acid residues Y56, T59, K60, V61, V69, V70, Y71, Q72, A73, K74, L75, L81, V82, A83, I84, K85, K86, L98, M101, R102, L104, H106, C107, N108, I109, V110, R111, L112, R113, Y114, F115, F116, L128, N129, L130, V131, L132, D133, Y134, V135, P136, E137, T138, V139, Y140, R141, V142, P154, V155, I156, Y157, V158, K159, L160, Y161, M162, Y163, Q164, L165, F166, R167, S168, L169, A170, Y171, I172, H173, S174, F175, G176, I177, C178, H179, R180, D181, I182, K183, P184, Q185, N186, L187, L188, L189, D190, P191, A194, V195, L196, K197, L198, C199 and D200 according to any one of Figures 1-7.

In another embodiment, the substrate binding pocket comprises amino acid residues S66, F67 and F93 according to any one of Figures 1-7. In another embodiment, the substrate binding pocket comprises amino acid residues G65, S66, F67 and F93 according to any one of Figures 1-7.

In another embodiment, the substrate binding pocket comprises amino acid residues D90, K91, R92, F93, K94 according to any one of Figures 1-7.

In another embodiment, the substrate binding pocket comprises amino acid residues R96, R180, K205, N213

and Y234 according to any one of Figures 1-7. In another embodiment, the substrate binding pocket comprises amino acid residues R96, R180 and K205 according to any one of Figures 1-7.

In another embodiment, the substrate binding pocket comprises amino acid residues Y216, I217, C218, S219, R220 and R223 according to any one of Figures 1-7.

In another embodiment, the substrate binding pocket comprises amino acid residues G65, S66, F67, D90, K91, R92, F93, K94, R96, R180, D181, K183, G202, S203, K205, P212, N213, V214, Y216, I217, C218, S219, R223 and Y234 according to any one of Figures 1-7.

In yet another embodiment, the substrate binding pocket comprises amino acid residues N64, G65, S66, F67, G68, V87, L88, D90, K91, R92, F93, K94, N95, R96, E97, R180, D181, I182, K183 Q185, N186, D200, F201, G202, S203, A204, K205, Q206, L207, E211, P212, N213, V214, S215, Y216, I217, C218, S219, R220, Y221, Y222, R223, L227, T232 and Y234 according to any one of Figures 1-7.

Thus, the binding pockets of this invention are defined by the structure coordinates of the above amino acids, as set forth in Figures 1-7.

It will be readily apparent to those of skill in the art that the numbering of amino acid residues in other homologues of GSK-3β may be different than that set forth for GSK-3β. Corresponding amino acid residues in homologues of GSK-3β are easily identified by visual inspection of the amino acid sequences or by using commercially available homology software programs.

Those of skill in the art understand that a set of structure coordinates for an enzyme or an enzyme-complex or a portion thereof, is a relative set of points that define a shape in three dimensions. Thus, it is possible that an entirely different set of coordinates could define a similar or identical shape. Moreover, slight variations in the individual coordinates will have little effect on overall shape. In terms of binding pockets, these variations would not be expected to significantly alter the nature of ligands that could associate with those pockets.

The variations in coordinates discussed above may be generated because of mathematical manipulations of the GSK-3β structure coordinates. For example, the structure coordinates set forth in any one of Figures 1-7 could be manipulated by crystallographic permutations of the structure coordinates, fractionalization of the structure coordinates, integer additions or subtractions to sets of the structure coordinates, inversion of the structure coordinates or any combination of the above.

Alternatively, modifications in the crystal structure due to mutations, additions, substitutions, and/or deletions of amino acids, or other changes in any of the components that make up the crystal could also account for variations in structure coordinates. If such variations are within a certain root mean square deviation as compared to the original coordinates, the resulting three-dimensional shape is considered encompassed by this invention. Thus, for example, a ligand that bound to the binding pocket of GSK-3β would also be expected to bind to another binding pocket whose structure coordinates defined a shape that fell within the acceptable root mean square deviation.

Various computational analyses may be necessary to determine whether a molecule or the binding pocket or portion thereof is sufficiently similar to the GSK-3β binding pockets described above. Such analyses may be carried out using well known software applications, such as the Molecular Similarity application of QUANTA (Molecular Simulations Inc., San Diego, CA © 1998), CCP4 (Acta Crystallogr., D50, 760-763 (1994)) or ProFit (A. C.R. Martin, ProFit version 1.8, http//www.bioinfo.org.uk/software).

The Molecular Similarity software application permits comparisons between different structures, different conformations of the same structure, and different parts of the same structure. The procedure used in Molecular Similarity to compare structures is divided into four steps: 1) load the structures to be compared; 2) define the atom equivalences in these structures; 3) perform a fitting operation; and 4) analyze the results.

Each structure in the comparison is identified by a name. One structure is identified as the target (i.e., the fixed structure); all remaining structures are working structures (i.e., moving structures). Since atom equivalency within QUANTA is defined by user input, for the purpose of this invention we will define equivalent atoms as protein backbone atoms N, C, O and Cα for all corresponding amino acids between the two structures being compared.

The corresponding amino acids may be identified by sequence alignment programs such as the "bestfit" program available from the Genetics Computer Group which uses the local homology algorithm described by Smith and Waterman in Advances in Applied Mathematics 2, 482 (1981), which is incorporated herein by reference. The identification of equivalent residues can also be assisted by secondary structure alignment, for example, aligning the α-helices, β-sheets or hinge region (residues 126-135 in GSK-3β in the structure. For programs that calculate RMSD values of the backbone atoms, an RMSD cutoff value can be used to exclude pairs of equivalent atoms with extreme individual RMSD values, or in situations where the equivalent atom can not be found in the corresponding structure.

When a rigid fitting method is used, the working structure is translated and rotated to obtain an optimum fit with the target structure. The fitting operation uses an algorithm that computes the optimum translation and rotation to be applied to the moving structure, such that the root mean square difference of the fit over the specified pairs of equivalent atom is an absolute minimum. This number, given in angstroms, is reported by QUANTA.

The RMSD values of the inhibitor and substrate-binding pockets between the GSK-3β-ADP-peptide structure

(Figure 7) and other GSK-3β structures (Figures 1-6) are illustrated in Tables 3-9. The RMSD values were calculated by the program LSQKAB in CCP4, *supra.* Backbone atoms (C, O, N and Cα) of all residues in the binding pocket were used in the calculation of the RMSD.

TABLE 3

| Inhibitor-binding pocket (K85, M101, V110, L130 and L132) | |
|---|---|
| GSK-3β structures | RMSD between inhibitor binding pockets (Å) |
| unphosphorylated GSK-3β | 0.32 |
| phosphorylated GSK-3β | 0.34 |
| GSK-3β inhibitor1 complex | 0.32 |
| GSK-3β inhibitor2 complex | 0.39 |
| GSK-3β inhibitor3 complex | 0.27 |
| GSK-3β inhibitor4 complex | 0.27 |

TABLE 4

| Inhibitor-binding pocket (162, V135, P136, T138 and L188) | |
|---|---|
| GSK-3β structures | RMSD between inhibitor binding pockets (Å) |
| unphosphorylated GSK-3β | 1.16 |
| phosphorylated GSK-3β | 1.02 |
| GSK-3β inhibitor1 complex | 0.77 |
| GSK-3β inhibitor2 complex | 0.95 |
| GSK-3β inhibitor3 complex | 0.23 |
| GSK-3β inhibitor4 complex | 0.31 |

TABLE 5

| Inhibitor-binding pocket (F67, V70, Q185, C199) | |
|---|---|
| GSK-3β structures | RMSD between inhibitor binding pockets (Å) |
| unphosphorylated GSK-3β | 1.83 |
| phosphorylated GSK-3β | 1.89 |
| GSK-3β inhibitor1 complex | 1.67 |
| GSK-3β inhibitor2 complex | 2.23 |
| GSK-3β inhibitor3 complex | 1.71 |
| GSK-3β inhibitor4 complex | 0.80 |

TABLE 6

| Inhibitor-binding pocket (V70, V110, L188 and C199) | |
|---|---|
| GSK-3β structures | RMSD between inhibitor binding pockets (Å) |
| unphosphorylated GSK-3β | 0.80 |
| phosphorylated GSK-3β | 0.88 |
| GSK-3β inhibitor1 complex | 0.81 |

(continued)

| Inhibitor-binding pocket (V70, V110, L188 and C199) | |
|---|---|
| GSK-3β structures | RMSD between inhibitor binding pockets (Å) |
| GSK-3β inhibitor2 complex | 0.92 |
| GSK-3β inhibitor3 complex | 0.38 |
| GSK-3β inhibitor4 complex | 0.30 |

TABLE 7

| Substrate-binding pocket (G65, S66, F67 and F93) | |
|---|---|
| GSK-3β structures | RMSD between inhibitor binding pockets (Å) |
| unphosphorylated GSK-3β | 1.93 |
| phosphorylated GSK-3β | 1.32 |
| GSK-3β inhibitor1 complex | 1.32 |
| GSK-3β inhibitor2 complex | 1.74 |
| GSK-3β inhibitor3 complex | 1.43 |
| GSK-3β inhibitor4 complex | 2.09 |

TABLE 8

| Substrate-binding pocket (R96, R180, K205, N213 and Y234) | |
|---|---|
| GSK-3β structures | RMSD between inhibitor binding pockets (Å) |
| unphosphorylated GSK-3β | 0.67 |
| phosphorylated GSK-3β | 0.59 |
| GSK-3β inhibitor1 complex | 0.64 |
| GSK-3β inhibitor2 complex | 0.71 |
| GSK-3β inhibitor3 complex | 0.71 |
| GSK-3β inhibitor4 complex | 0.65 |

TABLE 9

| Substrate-binding pocket (Y216, I217, C218, S219, R220 and R223) | |
|---|---|
| GSK-3β structures | RMSD between inhibitor binding pockets (Å) |
| unphosphorylated GSK-3β | 1.07 |
| phosphorylated GSK-3β | 0.36 |
| GSK-3β inhibitor1 complex | 0.46 |
| GSK-3β inhibitor2 complex | 1.35 |
| GSK-3β inhibitor3 complex | 1.39 |
| GSK-3β inhibitor4 complex | 1.21 |

The RMSD values of the overall structure between the GSK-3β-inhibitor2 structure (Figure 4) and other GSK-3β structures (Figures 1-3, and 5-7) are illustrated in Table 10. The RMSD values were calculated by the program LSQKAB in CCP4, supra. Backbone atoms (C, O, N and Cα) of all residues in the overall structure according to Figures

1-7 were used in the calculation of the RMSD.

TABLE 10

| GSK-3β structures | RMSD of overall structure(Å) |
|---|---|
| unphosphorylated GSK-3β | 0.52 |
| phosphorylated GSK-3β | 1.27 |
| GSK-3β-ADP-peptide complex | 1.94 |
| GSK-3β inhibitor1 complex | 0.79 |
| GSK-3β inhibitor3 complex | 1.77 |
| GSK-3β inhibitor4 complex | 0.90 |

For the purpose of this invention, any molecule or molecular complex or binding pocket thereof that is within a root mean square deviation for backbone atoms (C, O, N and Cα) when superimposed on the relevant backbone atoms described by structure coordinates listed in any one of Figures 1-7 are encompassed by this invention.

In one embodiment, the present invention provides a molecule or molecular complex comprising a binding pocket defined by structure coordinates of amino acid residues which are identical to GSK-3β amino acid residues K85, M101, V110, L130 and L132 according to any one of Figures 1-7, wherein the root mean square deviation of the backbone atoms between said amino acid residues and said GSK-3β amino acid residues is not greater than about 3.0 Å. In one embodiment, the RMSD is not greater than about 1.0 Å. In a preferred embodiment, the RMSD is not greater than about 0.5 Å.

In a more preferred embodiment, the root mean square deviation of the backbone atoms between said amino acid residues and said GSK-3β amino acid residues according to Figure 7 is not greater than about 0.5 Å. In one embodiment, the RMSD is not greater than about 0.2 Å.

In another embodiment, the root mean square deviation of the backbone atoms between said amino acid residues and said GSK-3β amino acid residues according to any one of Figures 1-7 is not greater than about 0.3 Å, and wherein at least one of said amino acid residues is not identical to the GSK-3β amino acid residue to which it corresponds. In one embodiment, the RMSD is not greater than about 0.2 Å.

In another embodiment, the present invention provides a molecule or molecular complex comprising a binding pocket defined by structure coordinates of amino acid residues which are identical to GSK-3β amino acid residues I62, V135, P136, T138 and L188 according to any one of Figures 1-7, wherein the root mean square deviation of the backbone atoms between said amino acid residues and said GSK-3β amino acid residues is not greater than about 3.0 Å. In one embodiment, the RMSD is not greater than about 1.5 Å. In a preferred embodiment the RMSD is not greater than about 1.0 Å.

In a more preferred embodiment, the root mean square deviation of the backbone atoms between said amino acid residues and said GSK-3β amino acid residues according to Figure 7 is not greater than about 0.8 Å. In one embodiment, the RMSD is not greater than about 0.5 Å. In one embodiment the RMSD is not greater than about 0.3 Å.

In another embodiment, the root mean square deviation of the backbone atoms between said amino acid residues and said GSK-3β amino acid residues according to any one of Figures 1-7 is not greater than about 0.2 Å, and wherein at least one of said amino acid residues is not identical to the GSK-3β amino acid residue to which it corresponds.

In another embodiment, the present invention provides a molecule or molecular complex comprising a binding pocket defined by structure coordinates of amino acid residues which are identical to GSK-3β amino acid residues V70, V110, L188 and C199 according to any one of Figures 1-7, wherein the root mean square deviation of the backbone atoms between said amino acid residues and said GSK-3β amino acid residues is not greater than about 3.0 Å. In one embodiment the RMSD is not greater than about 1.5 Å. In a preferred embodiment the RMSD is not greater than about 1.0 Å.

In a more preferred embodiment, the root mean square deviation of the backbone atoms between said amino acid residues and said GSK-3β amino acid residues according to Figure 7 is not greater than about 0.6 Å. In one embodiment, the RMSD is not greater than about 0.4 Å. In one embodiment, the RMSD is not greater than about 0.2 Å.

In another embodiment, the root mean square deviation of the backbone atoms between said amino acid residues and said GSK-3β amino acid residues according to any one of Figures 1-7 is not greater than about 0.2 Å, and wherein at least one of said amino acids is not identical to the GSK-3β amino acid to which it corresponds.

In another embodiment, the present invention provides a molecule or molecular complex comprising a binding pocket defined by structure coordinates of amino acid residues which are identical to GSK-3β amino acid residues V70, F67, Q185 and C199 according to any one of Figures 1-7, wherein the root mean square deviation of the backbone

atoms between said amino acid residues and said GSK-3β amino acid residues is not greater than about 3.0 Å. In a preferred embodiment, the RMSD is not greater than about 2.5 Å.

In a more preferred embodiment, the root mean square deviation of the backbone atoms between said amino acid residues and said GSK-3β amino acid residues according to Figure 7 is not greater than about 1.6 Å. In one embodiment, the RMSD is not greater than about 1.1 Å. In one embodiment, the RMSD is not greater than about 0.6 Å. In one embodiment, the RMSD is not greater than about 0.2 Å.

In another embodiment, the root mean square deviation of the backbone atoms between said amino acid residues and said GSK-3β amino acid residue according to any one of Figures 1-7 is not greater than about 0.3 Å, and wherein at least one of said amino acid residues is not identical to the GSK-3β amino acid residues to which it corresponds. In one embodiment, the RMSD is not greater than about 0.2 Å.

In another embodiment, the present invention provides a molecule or molecular complex comprising part of a binding pocket, said binding pocket defined by structure coordinates of amino acid residues which correspond to GSK-3β amino acid residues Y56, T59, K60, V61, I62, G63, N64, G65, S66, F67, G68, V69, V70, Y71, Q72, A73, K74, L75, L81, V82, A83, I84, K85, K86, V87, L88, E97, L98, M101, R102, L104, H106, C107, N108, I109, V110, R111, L112, R113, Y114, F115, F116, L128, N129, L130, V131, L132, D133, Y134, V135, P136, E137, T138, V139, Y140, R141, V142, R144, P154, V155, I156, Y157, V158, K159, L160, Y161, M162, Y163, Q164, L165, F166, R167, S168, L169, A170, Y171, I172, H173, S174, F175, G176, I177, C178, H179, R180, D181, I182, K183, P184, Q185, N186, L187, L188, L189, D190, P191, A194, V195, L196, K197, L198, C199, D200, F201, G202, S203 and S219 according to any one of Figures 1-7, wherein the root mean square deviation of the backbone atoms between said amino acid residues and said GSK-3β amino acid residues is not greater than about 0.2 Å. In a preferred embodiment, said amino acid residues are identical to said GSK-3β amino acid residues.

In yet another embodiment, the present invention provides a molecule or molecular complex comprising a binding pocket defined by structure coordinates of amino acid residues which are identical to GSK-3β amino acid residues G65, S66, F67 and F93 according to any one of Figures 1-7, wherein the root mean square deviation of the backbone atoms between said amino acid residues and said GSK-3β amino acid residues is not greater than about 3.0 Å. In one embodiment, the RMSD is not greater than about 2.5 Å. In a preferred embodiment, the RMSD is not greater than about 2.0 Å.

In a more preferred embodiment, the root mean square deviation of the backbone atoms between said amino acid residues and said GSK-3β amino acid residues according to Figure 7 is not greater than about 1.5 Å. In one embodiment, the RMSD is not greater than about 1.1 Å. In one embodiment, the RMSD is not greater than about 0.7 Å. In one embodiment, the RMSD is not greater than about 0.5 Å.

In another embodiment, the root mean square deviation of the backbone atoms between said amino acid residues and said GSK-3β amino acid residues according to any one of Figures 1-7 is not greater than about 1.1 Å, and wherein at least one of said amino acid residues is not identical to the GSK-3β amino acid residues to which it corresponds. In one embodiment, the RMSD is not greater than about 0.8 Å. In one embodiment, the RMSD is not greater than about 0.4 Å. In one embodiment, the RMSD is not greater than about 0.2 Å.

In another embodiment, the present invention provides a molecule or molecular complex comprising a binding pocket defined by structure coordinates of amino acid residues which correspond to GSK-3β amino acids R96, R180, K205, N213 and Y234 according to any one of Figures 1-7, wherein the root mean square deviation of the backbone atoms between said amino acid residues and said GSK-3β amino acid residues is not greater than about 3.0 Å, wherein said binding pocket comprises an amino acid residue asparagine corresponding to said GSK-3β amino acid residue N213. In another embodiment, said amino acid residues are identical to said GSK-3β amino acids. In one embodiment, the RMSD is not greater than about 1.5 Å. In a preferred embodiment, the RMSD is not greater than about 1.0 Å.

In a more preferred embodiment, the root mean square deviation of backbone atoms between said amino acid residues and said GSK-3β amino acid residues according to Figure 7 is not greater than about 0.4 Å. In one embodiment, the RMSD is not greater than about 0.2 Å.

In another embodiment, the root mean square deviation of the backbone atoms between said amino acid residues and said GSK-3β amino acid residues according to any one of Figures 1-7 is not greater than about 3.0 Å, and wherein at least one of said amino acid residues is not identical to the GSK-3β amino acid residues to which it corresponds. In one embodiment, the RMSD is not greater than about 1.2 Å. In one embodiment, the RMSD is not greater than about 0.7 Å. In one embodiment, the RMSD is not greater than about 0.3 Å.

In another embodiment, the present invention provides a molecule or molecular complex comprising a binding pocket defined by structure coordinates of amino acid residues which correspond to GSK-3β amino acid residues Y216, I217, C218, S219, R220 and R223 according to any one of Figures 1-7, wherein the root mean square deviation of the backbone atoms between said amino acid residues and said GSK-3β amino acid residues is not greater than about 3.0 Å, wherein said binding pocket comprises a cysteine amino acid residue corresponding to said GSK-3β amino acid residue C218. In another embodiment, said amino acid residues are identical to said GSK-3β amino acids. In one embodiment, the RMSD is not greater than about 2.0 Å. In a preferred embodiment, the RMSD is not greater than about

1.5 Å.

In a more preferred embodiment, the root mean square deviation of backbone atoms between said amino acid residues and said GSK-3β amino acid residues according to Figure 5 is not greater than about 1.1 Å. In one embodiment, the RMSD is not greater than about 0.5 Å. In one embodiment, the RMSD is not greater than about 0.2 Å.

In another embodiment, the root mean square deviation of the backbone atoms between said amino acid residues and said GSK-3β amino acid residues according to any one of Figures 1-7 is not greater than about 1.1 Å, and wherein at least one of said amino acid residues is not identical to the GSK-3β amino acid to which it corresponds. In one embodiment, the RMSD is not greater than about 0.8 Å. In one embodiment, the RMSD is not greater than about 0.5 Å. In one embodiment, the RMSD is not greater than about 0.2 Å.

In another embodiment, the present invention provides a molecule or molecular complex comprising part of a binding pocket, said binding pocket defined by structure coordinates of amino acid residues which correspond to GSK-3β amino acid residues N64, G65, S66, F67, G68, V87, L88, D90, K91, R92, F93, K94, N95, R96, E97, R180, D181, I182, K183 Q185, N186, D200, F201, G202, S203, A204, K205, Q206, L207, E211, P212, N213, V214, S215, Y216, I217, C218, S219, R220, Y221, Y222, R223, L227, T232 and Y234 according to any one of Figures 1-7, wherein the root mean square deviation of the backbone atoms between said amino acid residues and said GSK-3β amino acid residues is not greater than about 1.0 Å. In one embodiment, the RMSD is not greater than about 0.7 Å. In one embodiment, the RMSD is not greater than about 0.5 Å. In one embodiment, the RMSD is not greater than about 0.2 Å. In one embodiment, said amino acid residues are identical to said GSK-3β amino acid residues.

In one embodiment, the present invention provides a molecule or molecular complex comprising a GSK-3β protein defined by structure coordinates of the amino acid residues which correspond to GSK-3β amino acid residues according to any one of Figures 1-7, wherein the root mean square deviation of the backbone atoms between said amino acid residues and said GSK-3β amino acid residues is not greater than about 2.0 Å. In one embodiment, the RMSD is not greater than about 1.7 Å. In one embodiment, the RMSD is not greater than about 1.1 Å. In one embodiment, the RMSD is not greater than about 0.7 Å. In one embodiment, said amino acid residues are identical to said GSK-3β amino acid residues.

In one embodiment, the present invention provides a molecule or molecular complex comprising a protein kinase comprising a glutamine or glutamic acid residue that corresponds to Gln185 of GSK-3β protein, wherein the $\chi 1$ angle is in the range of 123° to 180°, and the $\chi 2$ angle is in the range of -174° to -180° and 106° to 180°. In another embodiment, the $\chi 1$ angle is in the range of -100° to -180° and the $\chi 2$ angle is in the range of -151° to -180° and 126° to 180°.

In one embodiment, the above molecules or molecular complexes are GSK-3β proteins or a GSK-3β homologues. In another embodiment, the above molecules or molecular complexes are in crystalline form.

<u>Computer Systems</u>

According to another embodiment, this invention provides a machine-readable data storage medium, comprising a data storage material encoded with machine-readable data, wherein said data defines the above mentioned molecules or molecular complexes. In one embodiment, the data defines the above mentioned binding pockets by comprising the structure coordinates of said amino acid residues according to any one of Figures 1-7.

To use the structure coordinates generated for the CSK-β, homologues thereof, or one of its binding pockets, it is sometimes necessary to convert them into a three-dimensional shape. This is achieved through the use of commercially available software that is capable of generating a three-dimensional structure of molecules or portions thereof from a set of structure coordinates.
The three-dimensional structure may be displayed as a graphical representation.

Therefore, according to another embodiment, this invention provides a machine-readable data storage medium comprising a data storage material encoded with machine readable data. In one embodiment, a machine programmed with instructions for using said data, is capable of generating a three-dimensional structure of any of the molecule or molecular complexes, or binding pockets thereof, that are described herein.

This invention also provides a computer comprising:

a) a machine-readable data storage medium comprising a data storage material encoded with machine-readable data, wherein said data defines any one of the above binding pockets of the molecule or molecular complex;
b) a working memory for storing instructions for processing said machine-readable data;
c) a central processing unit coupled to said working memory and to said machine-readable data storage medium for processing said machine readable data; and
d) output hardware coupled to said central processing unit for outputting information of said binding pocket or information produced by using said binding pocket.

Information of said binding pocket or information produced by using said binding pocket can be outputted

through a display terminal, printer or disk drive. The information can be in graphical or alphanumeric form. In another embodiment, the computer further comprises a commercially available software program to display the information as a graphical representation. Examples of software programs include but are not limited to QUANTA (Molecular Simulations, Inc., San Diego, CA ©2001), O (Jones et al., Acta Crystallogr. A47, pp. 110-119 (1991)) and RIBBONS (Carson, J. Appl. Crystallogr., 24, pp. 9589-961 (1991)), which are incorporated herein by reference.

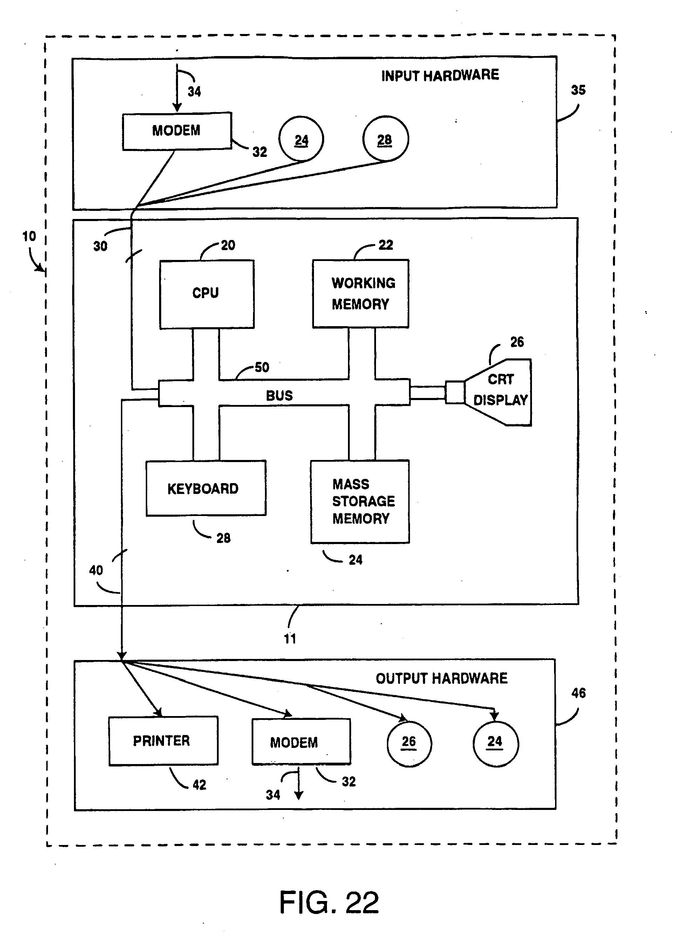

Figure 22 demonstrates one version of these embodiments. System (10) includes a computer (11) comprising a central processing unit ("CPU") (20), a working memory (22) which may be, e.g., RAM (random-access memory) or "core" memory, mass storage memory (24) (such as one or more disk drives or CD-ROM drives), one or more cathode-ray tube ("CRT") display terminals (26), one or more keyboards (28), one or more input lines (30), and one or more output lines (40), all of which are, interconnected by a conventional bi-directional system bus (50).

Input hardware (35), coupled to computer (11) by input lines (30), may be implemented in a variety of ways. Machine-readable data of this invention may be inputted via the use of a modem or modems (32) connected by a telephone line or dedicated data line (34). Alternatively or additionally, the input hardware (35) may comprise CD-ROM drives or disk drives (24). In conjunction with display terminal (26), keyboard (28) may also be used as an input device.

Output hardware (46), coupled to computer (11) by output lines (40), may similarly be implemented by conventional devices. By way of example, output hardware (46) may include CRT display terminal (26) for displaying a graphical representation of a binding pocket of this invention using a program such as QUANTA as described herein. Output hardware might also include a printer (42), so that hard copy output may be produced, or a disk drive (24), to store system output for later use.

In operation, CPU (20) coordinates the use of the various input and output devices (35), (46), coordinates data accesses from mass storage (24) and accesses to and from working memory (22), and determines the sequence of data processing steps. A number of programs may be used to process the machine-readable data of this invention. Such programs are discussed in reference to the computational methods of drug discovery as described herein. Specific references to components of the hardware system (10) are included as appropriate throughout the following description of the data storage medium.

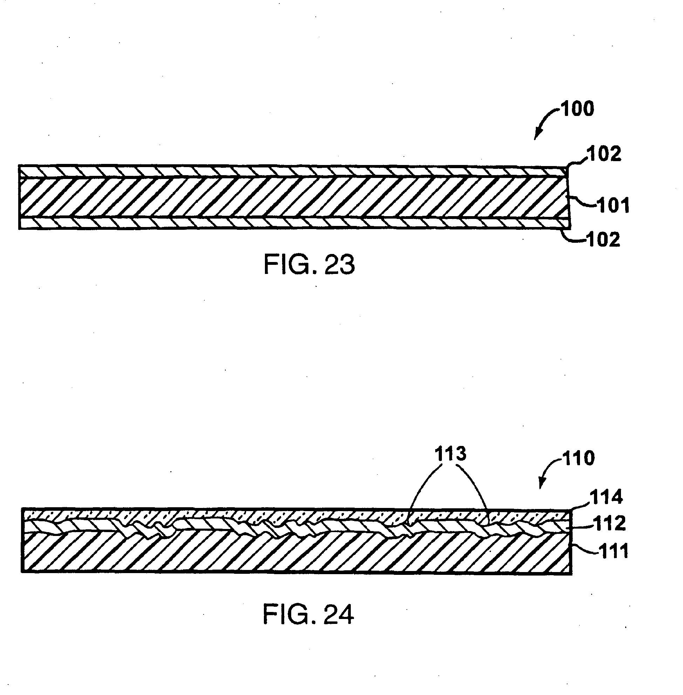

Figure 23 shows a cross section of a magnetic data storage medium (100) which can be encoded with a machine-readable data that can be carried out by a system such as system (10) of Figure 22. Medium (100) can be a conventional floppy diskette or hard disk, having a suitable substrate (101), which may be conventional, and a suitable coating (102), which may be conventional, on one or both sides, containing magnetic domains (not visible) whose polarity or orientation can be altered magnetically. Medium (100) may also have an opening (not shown) for receiving the spindle of a disk drive or other data storage device (24).

The magnetic domains of coating (102) of medium (100) are polarized or oriented so as to encode in manner which may be conventional, machine readable data such as that described herein, for execution by a system such as system (10) of Figure 22.

Figure 24 shows a cross section of an optically-readable data storage medium (110) which also can be encoded with such a machine-readable data, or set of instructions, which can be carried out by a system such as system (10) of Figure 22. Medium (110) can be a conventional compact disk read only memory (CD-ROM) or a rewritable medium such as a magneto-optical disk which is optically readable and magneto-optically writable.

Medium (100) preferably has a suitable substrate (111), which may be conventional, and a suitable coating (112), which may be conventional, usually of one side of substrate (111).

In the case of CD-ROM, as is well known, coating (112) is reflective and is impressed with a plurality of pits (113) to encode the machine-readable data. The arrangement of pits is read by reflecting laser light off the surface of coating (112). A protective coating (114), which preferably is substantially transparent, is provided on top of coating (112).

In the case of a magneto-optical disk, as is well known, coating (112) has no pits (113), but has a plurality of magnetic domains whose polarity or orientation can be changed magnetically when heated above a certain temperature, as by a laser (not shown). The orientation of the domains can be read by measuring the polarization of laser light reflected from coating (112). The arrangement of the domains encodes the data as described above.

Thus, in accordance with the present invention, data capable of generating the three dimensional structure of the above molecules or molecular complexes, or binding pockets thereof, can be stored in a machine-readable storage medium, which is capable of displaying a graphical three-dimensional representation of the structure.

## Rational Drug Design

The GSK-3β X-ray coordinate data, when used in conjunction with a computer programmed with software to generate those coordinates into the three-dimensional structure of GSK-3β may be used for a variety of purposes, such as drug discovery. The coordinate data themselves may also be used directly to perform computer modelling and fitting operations.

For example, the structure encoded by the data may be computationally evaluated for its ability to associate

with chemical entities. Chemical entities that associate with GSK-3β may inhibit GSK-3β and its homologues, and are potential drug candidates. Alternatively, the structure encoded by the data may be displayed in a graphical three-dimensional representation on a computer screen. This allows visual inspection of the structure, as well as visual inspection of the structure's association with chemical entities.

Thus, according to another embodiment, the invention relates to a method for evaluating the potential of a chemical entity to associate with a molecule or molecular complex as described previously in the different embodiments.

This method comprises the steps of: a) employing computational means to perform a fitting operation between the chemical entity and a binding pocket of the molecule or molecular complex; b) analyzing the results of said fitting operation to quantify the association between the chemical entity and the binding pocket; and optionally c) outputting said quantified association to a suitable output hardware, such as a CRT display terminal, a printer or a disk drive, as described previously. The method may further comprise the step of generating a three-dimensional graphical representation of the molecule or molecular complex or binding pocket thereof prior to step a).

Alternatively, the structure coordinates of the above binding pockets can be utilized in a method for identifying an agonist or antagonist of a molecule comprising any of the above binding pockets. This method comprises the steps of:

a) using the three-dimensional structure of said molecule or molecular complex to design or select a chemical entity;
b) contacting said chemical entity with the molecule or molecular complex and monitoring the activity of the molecule or molecular complex; and
c) classifying said chemical entity as an agonist or antagonist based on the effect of said chemical entity on the activity of the molecule or molecular complex.

In one embodiment, step a) is using the three-dimensional structure of the binding pocket of the molecule or molecular complex. In another embodiment, the three-dimensional structure is displayed as a graphical representation.

The present invention permits the use of molecular design techniques to identify, select and design chemical entities, including inhibitory compounds, capable of binding to the above binding pockets.

The elucidation of binding pockets on GSK-3β provides the necessary information for designing new chemical entities and compounds that may interact with GSK-3β substrate or ATP-binding pockets, in whole or in part. Due to the homology in the kinase core of GSK-3β and GSK-3β, compounds that inhibit GSK-3β are also expected to inhibit GSK-3β, especially those compounds that bind the ATP-binding pocket.

Throughout this section, discussions about the ability of an entity to bind to, associate with or inhibit the above binding pockets refers to features of the entity alone. Assays to determine if a compound binds to GSK-3β are well known in the art and are exemplified below.

The design of compounds that bind to or inhibit the above binding pockets according to this invention generally involves consideration of two factors. First, the entity must be capable of physically and structurally associating with parts or all of the above binding pockets. Non-covalent molecular interactions important in this association include hydrogen bonding, van der Waals interactions, hydrophobic interactions and electrostatic interactions.

Second, the entity must be able to assume a conformation that allows it to associate with the above binding pockets directly. Although certain portions of the entity will not directly participate in these associations, those portions of the entity may still influence the overall conformation of the molecule.
This, in turn, may have a significant impact on potency. Such conformational requirements include the overall three-dimensional structure and orientation of the chemical entity in relation to all or a portion of the binding pocket, or the spacing between functional groups of an entity comprising several chemical entities that directly interact with the above binding pockets.

The potential inhibitory or binding effect of a chemical entity on the above binding pockets may be analyzed prior to its actual synthesis and testing by the use of computer modeling techniques. If the theoretical structure of the given entity suggests insufficient interaction and association between it and the above binding pockets, testing of the entity is obviated. However, if computer modeling indicates a strong interaction, the molecule may then be synthesized and tested for its ability to bind to the above binding pocket. This may be achieved by testing the ability of the molecule to inhibit GSK-3β using the assays described in Example 18. In this manner, synthesis of inoperative compounds may be avoided.

A potential inhibitor of the above binding pockets may be computationally evaluated by means of a series of steps in which chemical entities or fragments are screened and selected for their ability to associate with the above binding pockets.

One skilled in the art may use one of several methods to screen chemical entities or fragments for their ability to associate with the above binding pockets. This process may begin by visual inspection of, for example, any of the above binding pockets on the computer screen based on the GSK-3β structure coordinates in any one of Figures 1-7 or other coordinates which define a similar shape generated from the machine-readable storage medium. Selected fragments or chemical entities may then be positioned in a variety of orientations, or docked, within that binding pocket

as defined supra. Docking may be accomplished using software such as QUANTA and Sybyl, followed by energy minimization and molecular dynamics with standard molecular mechanics force fields, such as CHARMM and AMBER.

Specialized computer programs may also assist in the process of selecting fragments or chemical entities. These include:

1. GRID (P. J. Goodford, "A Computational Procedure for Determining Energetically Favorable Binding Sites on Biologically Important Macromolecules", J. Med. Chem., 28, pp. 849-857 (1985)). GRID is available from Oxford University, Oxford, UK.
2. MCSS (A. Miranker et al., "Functionality Maps of Binding Sites: A Multiple Copy Simultaneous Search Method." Proteins: Structure, Function and Genetics, 11, pp. 29-34 (1991)). MCSS is available from Molecular Simulations, San Diego, CA.
3. AUTODOCK (D. S. Goodsell et al., "Automated Docking of Substrates to Proteins by Simulated Annealing", Proteins: Structure, Function, and Genetics, 8, pp. 195-202 (1990)). AUTODOCK is available from Scripps Research Institute, La Jolla, CA.
4. DOCK (I. D. Kuntz et al., "A Geometric Approach to Macromolecule-Ligand Interactions", J. Mol. Biol., 161, pp. 269-288 (1982)). DOCK is available from University of California, San Francisco, CA.

Once suitable chemical entities or fragments have been selected, they can be assembled into a single compound or complex. Assembly may be preceded by visual inspection of the relationship of the fragments to each other on the three-dimensional image displayed on a computer screen in relation to the structure coordinates of GSK-3β. This would be followed by manual model building using software such as QUANTA or Sybyl (Tripos Associates, St. Louis, MO).

Useful programs to aid one of skill in the art in connecting the individual chemical entities or fragments include:

1. CAVEAT (P. A. Bartlett et al., "CAVEAT: A Program to Facilitate the Structure-Derived Design of Biologically Active Molecules", in "Molecular Recognition in Chemical and Biological Problems", Special Pub., Royal Chem. Soc., 78, pp. 182-196 (1989); G. Lauri and P. A. Bartlett, "CAVEAT: a Program to Facilitate the Design of Organic Molecules", J. Comput. Aided Mol. Des. , 8, pp. 51-66 (1994)). CAVEAT is available from the University of California, Berkeley, CA.
2. 3D Database systems such as ISIS (MDL Information Systems, San Leandro, CA). This area is reviewed in Y. C. Martin, "3D Database Searching in Drug Design", J. Med. Chem., 35, pp. 2145-2154 (1992).
3. HOOK (M. B. Eisen et al., "HOOK: A Program for Finding Novel Molecular Architectures that Satisfy the Chemical and Steric Requirements of a Macromolecule Binding Site", Proteins: Struct., Funct., Genet., 19, pp. 199-221 (1994). HOOK is available from Molecular Simulations, San Diego, CA.

Instead of proceeding to build an inhibitor of any of the above binding pockets in a step-wise fashion, one fragment or chemical entity at a time as described above, inhibitory or other GSK-3β binding compounds may be designed as a whole or "de novo" using either an empty binding pocket or optionally including some portion(s) of a known inhibitor (s). There are many de novo ligand design methods including:

1. LUDI (H.-J. Bohm, "The Computer Program LUDI: A New Method for the De Novo Design of Enzyme Inhibitors", J. Comp. Aid. Molec. Design, 6, pp. 61-78 (1992)). LUDI is available from Molecular Simulations Incorporated, San Diego, CA.
2. LEGEND (Y. Nishibata et al., Tetrahedron, 47, p. 8985 (1991)). LEGEND is available from Molecular Simulations Incorporated, San Diego, CA.
3. LeapFrog (available from Tripos Associates, St. Louis, MO).
4. SPROUT (V. Gillet et al., "SPROUT: A Program for Structure Generation)", J. Comput. Aided Mol. Design, 7, pp. 127-153 (1993)). SPROUT is available from the University of Leeds, UK.

Other molecular modeling techniques may also be employed in accordance with this invention (see, e.g., N. C. Cohen et al., "Molecular Modeling Software and Methods for Medicinal Chemistry, J. Med. Chem., 33, pp. 883-894 (1990); see also, M. A. Navia and M. A. Murcko, "The Use of Structural Information in Drug Design", Current Opinions in Structural Biology, 2, pp. 202-210 (1992); L. M. Balbes et al., "A Perspective of Modern Methods in Computer-Aided Drug Design", in Reviews in Computational Chemistry, Vol. 5, K. B. Lipkowitz and D. B. Boyd, Eds., VCH, New York, pp. 337-380 (1994); see also, W. C. Guida, "Software For Structure-Based Drug Design", Curr. Opin. Struct. Biology, 4, pp. 777-781 (1994)).

Once a compound has been designed or selected by the above methods, the efficiency with which that entity may bind to any of the above binding pockets may be tested and optimized by computational evaluation. For example, an effective binding pocket inhibitor must preferably demonstrate a relatively small difference in energy between its

bound and free states (i.e., a small deformation energy of binding). Thus, the most efficient binding pocket inhibitors should preferably be designed with a deformation energy of binding of not greater than about 10 kcal/mole, more preferably, not greater than 7 kcal/mole. Binding pocket inhibitors may interact with the binding pocket in more than one conformation that is similar in overall binding energy. In those cases, the deformation energy of binding is taken to be the difference between the energy of the free entity and the average energy of the conformations observed when the inhibitor binds to the protein.

An entity designed or selected as binding to any one of the above binding pockets may be further computationally optimized so that in its bound state it would preferably lack repulsive electrostatic interaction with the target enzyme and with the surrounding water molecules. Such non-complementary electrostatic interactions include repulsive charge-charge, dipole-dipole and charge-dipole interactions.

Specific computer software is available in the art to evaluate compound deformation energy and electrostatic interactions. Examples of programs designed for such uses include: Gaussian 94, revision C (M. J. Frisch, Gaussian, Inc., Pittsburgh, PA ©1995); AMBER, version 4.1 (P. A. Kollman, University of California at San Francisco, ©1995); QUANTA/CHARMM (Molecular Simulations, Inc., San Diego, CA ©1998); Insight II/Discover (Molecular Simulations, Inc., San Diego, CA©1998); DelPhi (Molecular Simulations, Inc., San Diego, CA ©1998); and AMSOL (Quantum Chemistry Program Exchange, Indiana University). These programs may be implemented, for instance, using a Silicon Graphics workstation such as an Indigo2 with "IMPACT" graphics. Other hardware systems and software packages will be known to those skilled in the art.

Another approach enabled by this invention, is the computational screening of small molecule databases for chemical entities or compounds that can bind in whole, or in part, to any of the above binding pockets. In this screening, the quality of fit of such entities to the binding pocket may be judged either by shape complementarity or by estimated interaction energy (E. C. Meng et al., J. Comp. Chem., 13, pp. 505-524 (1992)).

Although the phosphorylated and unphosphorylated forms of GSK-3β have similar binding pockets, the subtle differences in water molecules, ions, position of the Y216 residue near the binding pockets as well as deviations in the overall structure may render slightly different results in the calculation of binding energies for inhibitors. By comparing the binding energies of inhibitors to the phosphorylated and unphosphorylated form, one may select inhibitors that are more suitable for one form than the other. Furthermore, the identification of inhibitors for both forms would allow the options of inhibiting GSK-3β prior to or after phosphorylation by upstream kinases *in vivo.*

Another particularly useful drug design technique enabled by this invention is iterative drug design. Iterative drug design is a method for optimizing associations between a protein and a compound by determining and evaluating the three-dimensional structures of successive sets of protein/compound complexes.

In iterative drug design, crystals of a series of protein or protein complexes are obtained and then the three-dimensional structures of each crystal is solved. Such an approach provides insight into the association between the proteins and compounds of each complex. This is accomplished by selecting compounds with inhibitory activity, obtaining crystals of this new protein/compound complex, solving the three-dimensional structure of the complex, and comparing the associations between the new protein/compound complex and previously solved protein/compound complexes. By observing how changes in the compound affect the protein/compound associations, these associations may be optimized.

In some cases, iterative drug design is carried out by forming successive protein-compound complexes and then crystallizing each new complex. Alternatively, a pre-formed protein crystal is soaked in the presence of an inhibitor, thereby forming a protein/compound complex and obviating the need to crystallize each individual protein/compound complex. The phosphorylated crystals provided by this invention may be soaked in the presence of a compound or compounds, to provide other crystal complexes.

## Structure Determination of Other Molecules

The structure coordinates set forth in any one of Figures 1-7 can also be used to aid in obtaining structural information about another crystallized molecule or molecular complex. This may be achieved by any of a number of well-known techniques, including molecular replacement.

According to an alternate embodiment, the machine-readable data storage medium comprises a data storage material encoded with a first set of machine readable data which comprises the Fourier transform of at least a portion of the structure coordinates set forth in any one of Figures 1-7, and which, when using a machine programmed with instructions for using said data, can be combined with a second set of machine readable data comprising the X-ray diffraction pattern of a molecule or molecular complex to determine at least a portion of the structure coordinates corresponding to the second set of machine readable data.

In another embodiment, the invention provides a computer for determining at least a portion of the structure coordinates corresponding to X-ray diffraction data obtained from a molecule or molecular complex, wherein said computer comprises:

a) a machine-readable data storage medium comprising a data storage material encoded with machine-readable data, wherein said data comprises at least a portion of the structure coordinates of GSK-3β according to any one of Figures 1-7;

b) a machine-readable data storage medium comprising a data storage material encoded with machine-readable data, wherein said data comprises X-ray diffraction data obtained from said molecule or molecular complex; and

c) instructions for performing a Fourier transform of the machine readable data of (a) and for processing said machine readable data of (b) into structure coordinates.

For example, the Fourier transform of at least a portion of the structure coordinates set forth in any one of Figures 1-7 may be used to determine at least a portion of the structure coordinates of GSK-3β homologues, and other sufficiently homologous kinases such as CDK2. In one embodiment, the molecule is a GSK-3β homologue. In another embodiment, the molecular complex is selected from the group consisting of a GSK-3β protein complex and a GSK-3β homologue complex.

Therefore, in another embodiment this invention provides a method of utilizing molecular replacement to obtain structural information about a molecule or molecular complex whose structure is unknown comprising the steps of:

a) crystallizing said molecule or molecular complex of unknown structure;

b) generating an X-ray diffraction pattern from said crystallized molecule or molecular complex; and

c) applying at least a portion of the GSK-3β structure coordinates set forth in any one of Figures 1-7 to the X-ray diffraction pattern to generate a three-dimensional electron density map of the molecule or molecular complex whose structure is unknown.

By using molecular replacement, all or part of the structure coordinates of the GSK-3β as provided by this invention (and set forth in any one of Figures 1-7) can be used to determine the structure of a crystallized molecule or molecular complex whose structure is unknown more quickly and efficiently than attempting to determine such information ab initio.

Molecular replacement provides an accurate estimation of the phases for an unknown structure. Phases are a factor in equations used to solve crystal structures that can not be determined directly. Obtaining accurate values for the phases, by methods other than molecular replacement, is a time-consuming process that involves iterative cycles of approximations and refinements and greatly hinders the solution of crystal structures. However, when the crystal structure of a protein containing at least a homologous portion has been solved, the phases from the known structure provide a satisfactory estimate of the phases for the unknown structure.

Thus, this method involves generating a preliminary model of a molecule or molecular complex whose structure coordinates are unknown, by orienting and positioning the relevant portion of the GSK-3β according to any one of Figures 1-7 within the unit cell of the crystal of the unknown molecule or molecular complex so as best to account for the observed X-ray diffraction pattern of the crystal of the molecule or molecular complex whose structure is unknown. Phases can then be calculated from this model and combined with the observed X-ray diffraction pattern amplitudes to generate an electron density map of the structure whose coordinates are unknown. This, in turn, can be subjected to any well-known model building and structure refinement techniques to provide a final, accurate structure of the unknown crystallized molecule or molecular complex (E. Lattman, "Use of the Rotation and Translation Functions", in Meth. Enzymol., 115, pp. 55-77 (1985); M. G. Rossmann, ed., "The Molecular Replacement Method", Int. Sci. Rev. Ser., No. 13, Gordon & Breach, New York (1972)).

The structure of any portion of any crystallized molecule or molecular complex that is sufficiently homologous to any portion of the GSK-3β can be resolved by this method.

In a preferred embodiment, the method of molecular replacement is utilized to obtain structural information about a GSK-3 homologue. The structure coordinates of GSK-3β as provided by this invention are particularly useful in solving the structure of GSK-3β complexes that are bound by ligands, substrates and inhibitors.

Furthermore, the structure coordinates of GSK-3β as provided by this invention are useful in solving the structure of GSK-3β proteins that have amino acid substitutions, additions and/or deletions (referred to collectively as "GSK-3β mutants", as compared to naturally occurring GSK-3β. These GSK-3β mutants may optionally be crystallized in co-complex with a chemical entity, such as a non-hydrolyzable ATP analogue, a suicide substrate or a inhibitor. The crystal structures of a series of such complexes may then be solved by molecular replacement and compared with that of wild-type GSK-3β. Potential sites for modification within the various binding pockets of the enzyme may thus be identified. This information provides an additional tool for determining the most efficient binding interactions, for example, increased hydrophobic interactions, between GSK-3β and a chemical entity or compound.

The structure coordinates are also particularly useful to solving the structure of crystals of GSK-3β or GSK-3β homologues co-complexed with a variety of chemical entities. This approach enables the determination of the optimal sites for interaction between chemical entities, including candidate GSK-3β inhibitors and GSK-3β. For example, high

resolution X-ray diffraction data collected from crystals exposed to different types of solvent allows the determination of where each type of solvent molecule resides. Small molecules that bind tightly to those sites can then be designed and synthesized and tested for their GSK-3β inhibition activity.

All of the complexes referred to above may be studied using well-known X-ray diffraction techniques and may be refined versus 1.5-3.4 Å resolution X-ray data to an R value of about 0.30 or less using computer software, such as X-PLOR (Yale University, ©1992, distributed by Molecular Simulations, Inc.; see, e.g., Blundell & Johnson, *supra;* Meth. Enzymol., vol. 114 & 115, H. W. Wyckoff et al., eds., Academic Press (1985)). This information may thus be used to optimize known GSK-3β inhibitors, and more importantly, to design new GSK-3β inhibitors.

In order that this invention be more fully understood, the following examples are set forth. These examples are for the purpose of illustration only and are not to be construed as limiting the scope of the invention in any way.

Example 1

3-Amino-4,5-diphenyl-1H-pyrazolo[3,4-c]pyridazine (Compound 1)

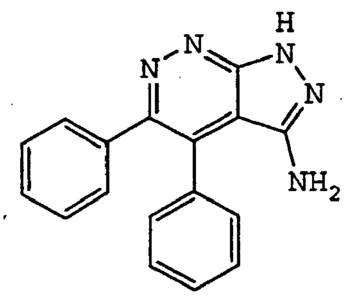

The appropriate diaryl keto hydrazone (see Scheme I, formula 1, wherein X is H and Y is H; 0.85 mmol) and ethyl cyanoacetate (0.9 mmol) were added to 3 mL ethanol. Sodium ethoxide (0.9 mmol) in THF was subsequently added and the mixture refluxed for 6 hours. After cooling, the solvent was removed under vacuum and the residue taken up in 10 mL dichloromethane. It was then washed with 0.1 M HCl, water and dried with sodium sulfate. After filtering, the solvent was removed under vacuum and the product, 4-cyano-5,6-diaryl 2(1H) pyridazinone (Scheme I, formula 2, wherein X is H and Y is H) was purified by chromatography on silica gel (5:95 methanol/dichloromethane).

Purified 4-cyano-5,6-diaryl 2(1H) pyridazinone (100 mg) was added to 2 mL $POCl_3$ and heated to 100˚C for 5-6 hours. After cooling, the reaction mixture was poured onto 10 mL ice and stirred for one hour. The resulting 3-chloro-4-cyano-5,6-diaryl pyridazine (Scheme I, formula 3, wherein X is H and Y is H) was filtered off, washed with water, air dried and used in the next step without further purification.

Crude 3-chloro-4-cyano-5,6-diaryl pyridazine was refluxed with 2 equivalents of anhydrous hydrazine in ethanol for several hours. Upon cooling, the product would sometimes precipitate out, in which case the pure title compound was obtained by recrystallizing from ethanol. Otherwise the title compound was purified by chromatography on silica gel (5:95 methanoldichloromethane): MS (ES+) m/e: 288.01 (M+H); analytical HPLC ($C_{18}$ column) 2.96 minutes.

Example 2

3-Amino-4(4-chlorophenyl)-5-phenyl-1H-pyrazolo[3,4-c]pyridazine (Compound 2)

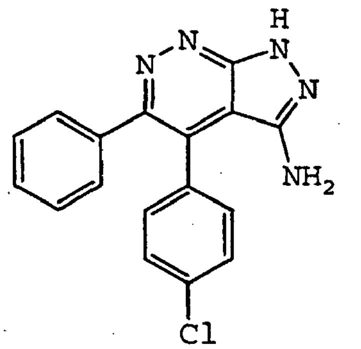

The title compound was obtained according to synthetic procedures described in EXAMPLE 1 using the appropriate diaryl keto hydrazone (X is H and Y is p-Cl): MS (ES+) m/e: 321.89 (M+H); analytical HPLC ($C_{18}$ column) 3.33 minutes.

Example 3

3-Amino-4,5-bis(4-fluorophenyl)-1H-pyrazolo[3,4-c] pyridazine (Compound 3)

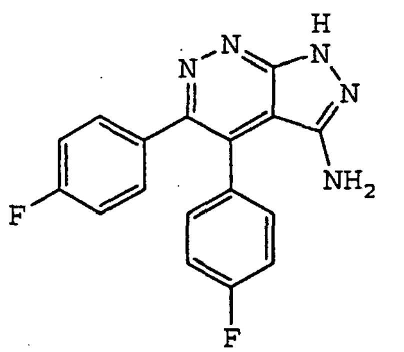

The title compound was obtained according to synthetic procedures described in EXAMPLE 1 using the appropriate diaryl keto hydrazone (X is p-F and Y is p-F): MS (138+) m/e: 324.1 (M+H); analytical HPLC ($C_{18}$ column) 3.26 minutes.

Example 4

3-Amino-4-phenyl-5-(4-fluorophenyl)-1H-pyrazolo[3,4-c] pyridazine (Compound 4)

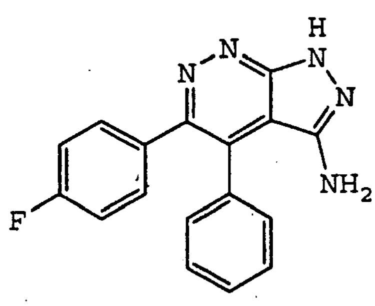

The title compound was obtained according to synthetic procedures described in EXAMPLE 1 using the appropriate diaryl keto hydrazone (X is p-F and Y is H): MS (ES+) m/e: 306.1 (M+H); analytical HPLC ($C_{18}$ column) 3.08 minutes.

Example 5

3-Amino-4-(4-fluorophenyl)-5-phenyl-1H-pyrazolo[3,4-c] pyridazine (Compound 5)

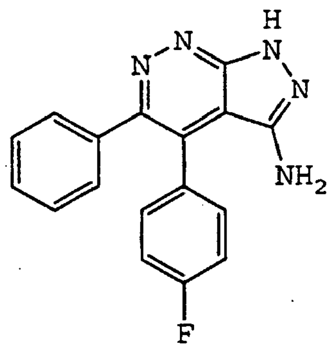

The title compound was obtained according to synthetic procedures described in EXAMPLE 1 using the appropriate diaryl keto hydrazone (X is H and Y is p-F): MS (ES+) m/e: 306.1 (M+H); analytical HPLC ($C_{18}$ column) 3.02 minutes.

Example 6

3-Amino-4-(3-fluorophenyl)-5-phenyl-1H-pyrazolo[3,4-c] pyridazine (Compound 6)

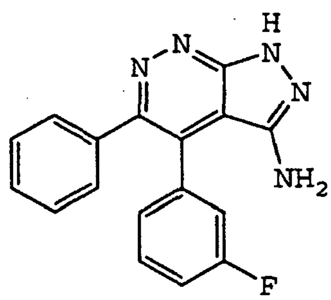

The title compound was obtained according to synthetic procedures described in EXAMPLE 1 using the appropriate diaryl keto hydrazone (X is H and Y is m-F): MS (ES+) m/e: 306.1 (M+H); analytical HPLC ($C_{18}$ column) 2.94 minutes.

Example 7

3-Amino-4-phenyl-5-(4-pyridyl)-1H-pyrazolo[3,4-c] pyridazine (Compound 7)

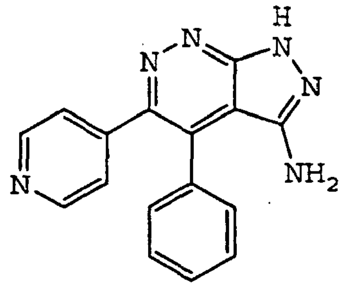

The title compound was obtained according to synthetic procedures described in EXAMPLE 1 using the appropriate heteroaryl aryl keto hydrazone: MS (ES+) m/e: 289.1 (M+H); analytical HPLC ($C_{18}$ column) 1.77 minutes.

Example 8

3-Amino-4-phenyl-5-(3-fluorophenyl)-1H-pyrazolo[3,4-c] pyridazine (Compound 8)

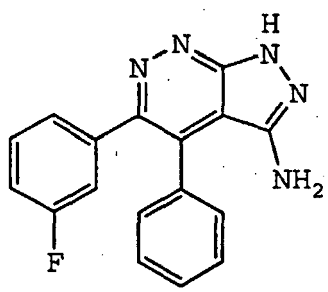

The title compound was obtained according to synthetic procedures described in EXAMPLE 1 using the appropriate diaryl keto hydrazone (X is m-F and Y is H): MS (ES+) m/e: 306.1 (M+H); analytical HPLC ($C_{18}$ column) 3.12 minutes.

Example 9

N-(4,5-Diphenyl-1H-pyrazolo[3,4-c]pyridazin-3-yl)-acetamide (Compound 9)

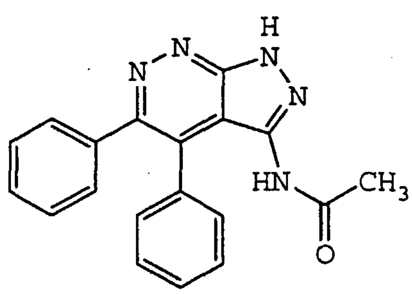

The title compound was obtained according to synthetic procedures described in EXAMPLE 1 using the appropriate diaryl keto hydrazone (X is H and Y is H), followed by amidation with $CH_3CO_2H$: MS (ES+) m/e: 330.1 (M+H); analytical HPLC ($C_{18}$ column) 2.65 minutes.

Example 10

N-(4,5-Diphenyl-1H-pyrazolo[3,4-c]pyridazin-3-yl)-benzamide (Compound 10)

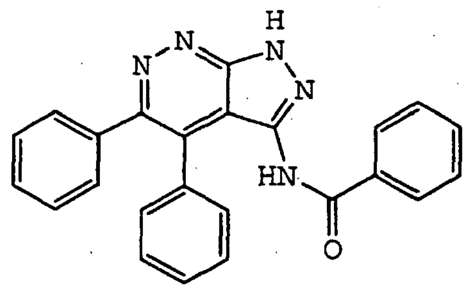

The title compound was obtained according to synthetic procedures described in EXAMPLE 1 using the appropriate diaryl keto hydrazone (X is H and Y is H), followed by amidation with benzoic acid: MS (ES+) m/e: 414.2 (M+Na+); analytical HPLC ($C_{18}$ column) 2.90 minutes.

Example 11

3-Amino-4-phenyl-5-(4-methyl-phenyl)-1H-pyrazolo[3,4-c] pyridazine (Compound 11)

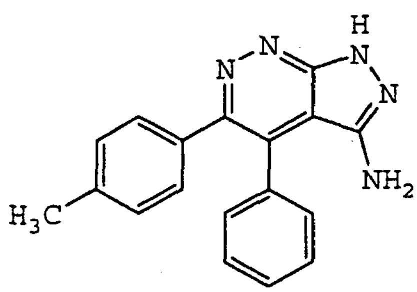

The title compound was obtained according to synthetic procedures described in EXAMPLE 1 using the appropriate diaryl keto hydrazone (X is p-$CH_3$ and Y is H) : MS (ES+) m/e: 302.1 (M+H); analytical HPLC ($C_{18}$ column) 3.07 minutes.

Example 12

3-Amino-4-phenyl-5-(2-methyl-phenyl)-1H-pyrazolo[3,4-c] pyridazine (Compound 12)

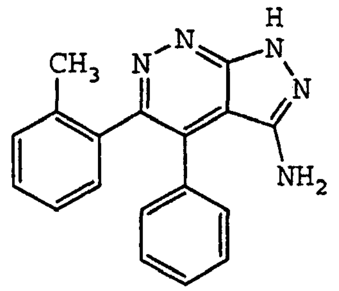

The title compound was obtained according to synthetic procedures described in EXAMPLE 1 using the appropriate diaryl keto hydrazone (X is o-$CH_3$ and Y is H): MS (ES+) m/e: 302.1 (M+H); analytical HPLC ($C_{18}$ column) 2.94 minutes.

Example 13

3-Amino-4-phenyl-5-(3-methyl-phenyl)-1H-pyrazolo[3,4-c] pyridazine (Compound 13)

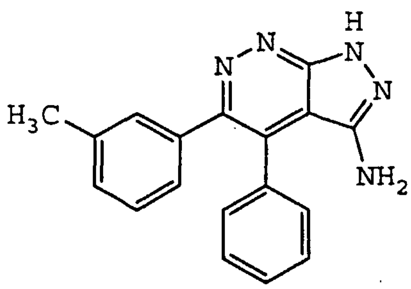

The title compound was obtained according to synthetic procedures described in EXAMPLE 1 using the appropriate diaryl keto hydrazone (X is m-$CH_3$ and Y is H): MS (ES+) m/e: 302.1 (M+H); analytical HPLC ($C_{18}$ column) 3.09 minutes.

Example 14

3-Amino-4-phenyl-5-(2-chloro-phenyl)-1H-pyrazolo[3,4-c] pyridazine (Compound 14)

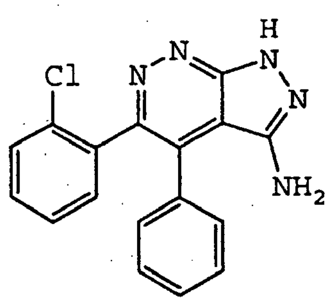

The title compound was obtained according to synthetic procedures described in EXAMPLE 1 using the appropriate diaryl keto hydrazone (X is o-Cl and Y is H): MS (ES+) m/e: 322.1 (M+H); analytical HPLC ($C_{18}$ column) 3.48 minutes.

Example 15

3-Amino-4-phenyl-5-(2-fluorophenyl)-1H-pyrazolo[3,4-c] pyridazine (Compound 15)

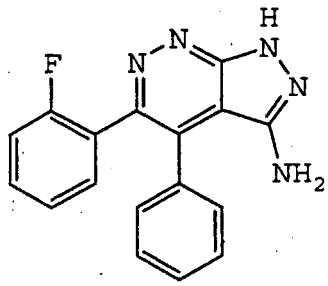

The title compound was obtained according to synthetic procedures described in EXAMPLE 1 using the appropriate diaryl keto hydrazone (X is o-F and Y is H): MS (ES+) m/e: 306.1 (M+H); analytical HPLC ($C_{18}$ column) 2.97 minutes.

Example 16

3-Amino-4-phenyl-5-(4-chloro-phenyl)-1H-pyrazolo[3,4-c] pyridazine (Compound 16)

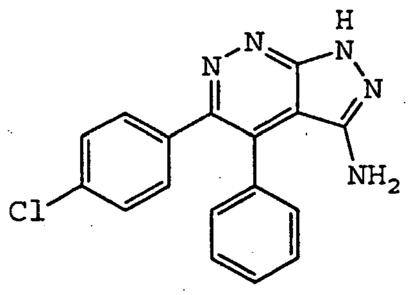

The title compound was obtained according to synthetic procedures described in EXAMPLE 1 using the appropriate diaryl keto hydrazone (X is p-Cl and Y is H): MS (ES+) m/e: 322 (M+H); analytical HPLC ($C_{18}$ column) 4.06 minutes.

Example 17

4-Phenyl-1H-pyrazolo[3,4-c]pyridazin-3-ylamine (Compound 32)

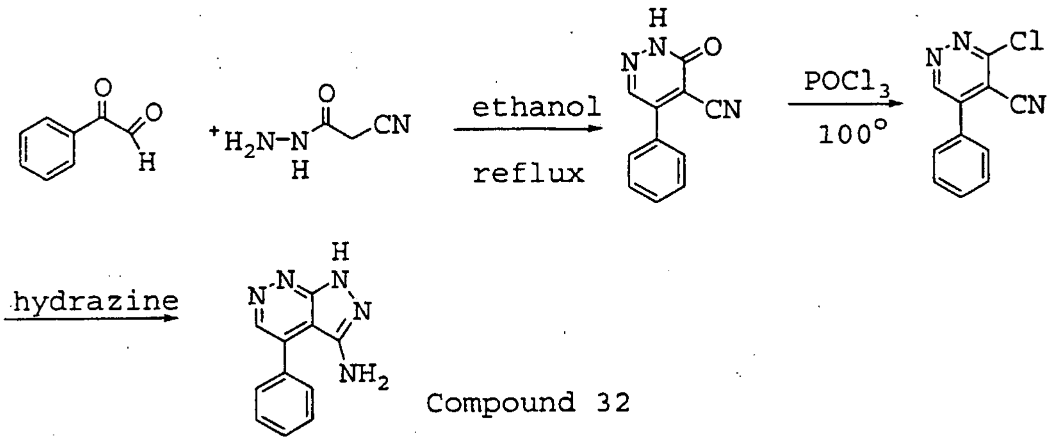


Compound 32

Step A: 3-Oxo-5-phenyl-2,3-dihydro-pyridazine-4-carbonitrile

Phenyl glyoxal (1.0 g, 7.46 mmol) and cyanoacetohydrazide (740 mg, 7.46 mmol) were heated to reflux in 10 mL ethanol for 16 hours. After cooling, the solvent was evaporated and the crude brown mixture was purified by silica chromatography (1:9 methanol/dichloromethane). The still impure product was further recrystallized from methanol affording 70 mg pure product.

Step B: 3-Chloro-5-phenyl-pyridazine-4-carbonitrile

3-Oxo-5-phenyl-2,3-dihydro-pyridazine-4-carbonitrile (70 mg) was suspended in 1 mL phosphorous oxychloride and heated to 100˚C for 6 hours. The mixture was then cooled and poured onto ice. The resulting brown solid product was filtered and air dried and used in the next step without further purification.

Step C: 4-Phenyl-1H-pyrazolo[3,4-c]pyridazin-3-ylamine

3-Chloro-5-phenyl-pyridazine-4-carbonitrile obtained from Step B was suspended in 1 mL ethanol with 23 μL hydrazine and the mixture was refluxed for several hours. The solvent was then evaporated and the title product was purified by silica gel chromatography (1:9 methanol/dichloromethane): MS (ES+) 212 (M+H); HPLC 1.121 minutes.

Example 18

$K_i$ Determination for the Inhibition of GSK-3

Compounds were screened for their ability to inhibit GSK-3β (amino acids 1-420) activity using a standard coupled enzyme system (Fox et al. (1998) *Protein Sci*. 7, 2249). Reactions were carried out in a solution containing 100 mM HEPES (pH 7.5), 10 mM $MgCl_2$, 25 mM NaCl, 300 μM NADH, 1 mM DTT and 1.5% DMSO. Final substrate concentrations in the assay were 20 μM ATP (Sigma Chemicals, St Louis, MO) and 300 μM peptide (HSSPHQS($PO_3H_2$) EDEEE, American Peptide, Sunnyvale, CA).
Reactions were carried out at 30 ˚C and 20 nM GSK-3β. Final concentrations of the components of the coupled enzyme system were 2.5 mM phosphoenolpyruvate, 300 μM NADH, 30 μg/ml pyruvate kinase and 10 μg/ml lactate dehydrogenase.

An assay stock buffer solution was prepared containing all of the reagents listed above with the exception of ATP and the test compound of interest. The assay stock buffer solution (175 μl) was incubated in a 96 well plate with 5 μl of the test compound of interest at final concentrations spanning 0.002 μM to 30 μM at 30 ˚C for 10 min. Typically, a 12 point titration was conducted by preparing serial dilutions (from 10 mM compound stocks) with DMSO of the test compounds in daughter plates. The reaction was initiated by the addition of 20 μl of ATP (final concentration 20 μM). Reaction rates were obtained using a Molecular Devices Spectramax plate reader (Sunnyvale, CA) over 10 min at 30 ˚C. The $K_i$ values were determined from the rate data as a function of inhibitor concentration.

The GSK-3 inhibitory activity of certain compounds of this invention are shown in Table 11. For GSK-3 $K_i$ values, "+++" represents ≤ 0.1 μM, "++" represents between 0.1 and 10 μM, and "+" represents ≥ 10 μM.

TABLE 11 Inhibitory Activity

| Compound No. | $K_i$ |
|---|---|
| 1 | +++ |
| 2 | + |
| 3 | ++ |
| 4 | + |
| 5 | +++ |
| 6 | +++ |
| 7 | + |
| 8 | +++ |
| 9 | ++ |
| 10 | ++ |
| 11 | +++ |
| 12 | ++ |
| 13 | ++ |
| 14 | +++ |
| 15 | +++ |
| 16 | ++ |
| 32 | ++ |

Example 19

Expression and Purification of GSK-3β

Full length human GSK-3β (1-420) (SEQ ID NO:1) with an N-terminal hexa-histidine tag and a thrombin cleavage site was overexpressed in a baculo virus expression system. GSK-3β was purified using Talon metal affinity chromatography (Clontech, Palo Alto, CA) followed by size-exclusion on a Superdex 200 column (Pharmacia, Uppsala, Sweden). The hexa-histidine tag was then removed by incubation with thrombin (Calbiochem, La Jolla, CA). In addition to the authentic thrombin site, a second cleavage product was identified 10 amino acids downstream at Threonine 7. Incubation overnight at 4˚C with 12 U $mg^{-1}$ thrombin produced more than 90% GSK-3β (7-420), which was used for crystallographic studies. The reaction was quenched with PMSF and thrombin was removed with benzamidine sepharose (Pharmacia, Uppsala, Sweden). To separate unphosphorylated GSK-3β (7-420) from the phosphorylated species and GSK-3β cleaved at the authentic thrombin cleavage site, the protein was applied to a MonoS 10/10 column (Pharmacia, Uppsala, Sweden) equilibrated in 25 mM HEPES, pH 7.2, 10% Glycerol (v/v), 2 mM DTT. The protein was eluted with a linear gradient from 0 to 300 mM NaCl in 30 column volumes. Unphosphorylated GSK-3β (7-420) eluted at 150 mM NaCl. Phosphorylated GSK-3β (7-420) eluted at around 200 mM NaCl The protein was dialyzed against 25 mM Tris pH 8.0 containing 200 mM NaCl and 2 mM DTT at 4 ˚C, concentrated to 15 mg $ml^{-1}$, and centrifuged at 100,000 x g prior to crystallization. All protein molecular weights were confirmed by electrospray mass spectrometry. Phosphorylation on Tyr 216 was confirmed by tryptic digest and MALDI-TOF spectrometry.

Example 20

Crystallization of GSK-3β

Crystallization of GSK-3β was carried out using the hanging drop vapor diffusion technique. The unphosphorylated GSK-3β formed diamond shape crystals over a reservoir containing 15% PEG 3350, 50 mM Na/KP04 pH 4.1, 10 mM DTT. The crystallization droplet contained 1 μl of 15 mg $ml^{-1}$ protein solution and 1 μl of reservoir solution. Crystals formed in less than 1 hour and were harvested in a reservoir solution after 12 hrs.

The phosphorylated form (Tyrosine 216) of GSK-3β formed plate-like crystals over a reservoir containing Solution A (7-10% PEG 3350, 100 mM Tris HCl pH 7.5, 5% Dimethylsulphoxide(DMSO)). The component DMSO was important for the crystallaization of phosphorylated GSK-3β. The crystallization droplet contained 1 μl of protein (16 mg/mL) and 1 μl of reservoir solution. The crystals formed overnight and were harvested in Solution A after a few days.

In order to obtain crystals of the ADP-peptide-GSK-3β complex, 0.3 mM protein was mixed with 1.4 mM glycogen synthase peptide (residues 650 to 661), 2 mM ADP and 2 mM MgCl. The mixture was incubated on ice for two hours. Small rod shaped crystals of the complex formed over a reservoir containing 10-15% PEG 3350 and 50 mM ammonium fluoride. Crystals large enough for data collection were obtained after repeated cycles of micro seeding.

Once the above crystals were harvested in reservoir solution, they were transferred to reservoir solutions containing increasing concentrations of glycerol, starting with 2% and increasing to 5, 10, 15, 20, 25 and 30%. After soaking the crystals in 30% glycerol for less than 5 minutes, the crystals were scooped up with a cryo-loop, frozen in liquid nitrogen and stored for data collection.

Example 21

Formation of GSK-3β-inhibitor Complex Crystals

Phosphorylated GSK-3β-inhibitorl complex crystals were formed by soaking phosphorylated GSK-3β crystals in Solution A that also contained 500 μM of inhibitor 4,5-Diphenyl-1H-pyrazolo[3,4-c]pyridazin-3-ylamine.

Crystals of unphosphorylated GSK-3β-inhibitor2-4 complexes were formed by co-crystallizing the protein with the inhibitors. The inhibitor was added to the concentrated GSK-3β protein solution right before setting up the crystallization drop. Alternatively, inhibitor could be added to a diluted protein solution, and the mixture concentrated to the required concentration. The unphosphorylated GSK-3β protein co-crystallized with inhibitors(5-Methyl-2H-pyrazol-3-yl)-(2-pyridin-4-yl-quinazolin-4-yl)-amine, 4-(5-Methyl-2-phenylamino-pyrimidin-4-yl)-1H-pyrrole-2-carboxylic acid (2-hydroxy-1-phenyl-ethyl)-amide), and (1H-Indazol-3-yl)-[2-(2-trifluoromethyl-phenyl)-quinazolin-4-yl]-amine) over a reservoir solution containing 15-20% PEG 3350, 0.1-1 M of KF, potassium formate or ammonium formate. The crystallization droplet contained 1 μl of 10-20 mg/ml protein solution containing the inhibitor and 1 μl of reservoir solution.

Once the crystals of both forms were harvested, they were transferred to reservoir solutions containing increasing concentrations of glycerol, starting with 5% and increasing to 10, 15, 20, 25 and 30%. After soaking the crystals in 30% glycerol for less than 5 minutes, the crystals were scooped up with a cryo-loop, frozen in liquid nitrogen and stored for data collection.

Example 22

X-Ray Data Collection and Structure Determination

X-ray diffraction data for the unphosphorylated GSK-3β, phosphorylated GSK-3β, phosphorylated GSK-3β-inhibitor complex, unphosphorylated GSK-3β-inhibitor complexes, phosphorylated GSK-3β-ADP-peptide complex structures were collected on a Raxis 4 image plate, with mirror-focused CuKα X-rays generated by a rotating-anode source. X-ray data used to refine the unphosphorylated GSK-3β structure was collected at beam line 5.0.2 of the Advanced Light Source Lawrence Berkeley Laboratory, Berkeley, California. Data collected on the Raxis 4 image plate was processed with DENZO and SCALEPACK (Otwinowski et al., Methods Enzymol., 180, 51-62 (1989)) Data collected at ALS were processed with the program MOSFLM and the data was scaled using SCALA (Collaborative Computational Project, N., Acta Cryst., D50, pp. 760-763 (1994)).

The data statistics of the unphosphorylated form are summarized in Table 12. The spacegroup of the unphosphorylated crystals was $P2_12_12_1$, with unit cell dimensions a= 83 b= 86 c= 178 Å, α = β = γ = 90˚. The starting phases for unphosphorylated GSK-3β were obtained by molecular replacement using coordinates of CDK2 (Protein Data Bank Accession number 1AQ1) (Lawrie, A.M., et al., Nat. Struct. Biol., 4, pp. 796-801 (1997)) as a search model in the program AMORE (Navaza, J. Acta. Cryst., 50, pp. 157-163 (1994)). The asymmetric unit contained two molecules. Multiple rounds of rebuilding with QUANTA and refinement with CNX resulted in a final model that included amino acid residues 25 to 384 for molecule A and residues 37 to 382 for molecule B, 4 phosphate ions and 46 water molecules. The α carbons in A and B chains have a root-mean-squared deviation after superposition of 0.48 Å. The refined model has a crystallographic R-factor of 23.7% and R-free of 27.4%. The coordinates of the structure have been deposited with the Protein Databank (accession code 1I09).

The data statistics of the phosphorylated form are summarized in Table 13. The spacegroup of the crystals was P1, with unit cell dimensions a= 64 Å b=67.2 Å c= 67.4 Å α= 100.1˚ β= 103.5˚ γ= 90˚ or a= 64 Å b=67 Å c= 67 Å α= 80˚ β= 77˚ γ= 89.8˚. The dimensions of the unit cell varied 1-2% from crystal to crystal. The starting phases for phosphorylated GSK-3β were obtained by molecular replacement using coordinates of the unphosphorylated form as

a search model in the program AMORE ENRfu, *supra.* The asymmetric unit contained two molecules. Multiple rounds of rebuilding with QUANTA and refinement with CNX resulted in a final model that included residues 37 to 383 for molecule A and residues 37 to 383 for molecule B and 83 water molecules. All data was included and NCS restraint was applied through out the refinement. The final step of refinement was an individual B-factor refinement. The refined model has a crystallographic R-factor of 23.8% and R-free of 27.6%.

The spacegroup of the unphosphorylated GSK-3β-inhibitor2-4 complex crystals was $P2_12_12_1$, with unit cell dimensions a= 83 b= 86 c= 178 Å, $\alpha = \beta = \gamma = 90^\circ$. The starting phases for unphosphorylated GSK-3β-inhibitor complexes were obtained by molecular replacement using coordinates of the unphosphorylated form as a search model in the program AMORE ENRfu, *supra*. The asymmetric unit contained two molecules. Multiple rounds of rebuilding with QUANTA and refinement with CNX were performed.

The data statistics of the unphosphorylated GSK-3β-inhibitor2 complex are summarized in Table 14. The structure was refined to 2.9 Å, and the R-factor was 24.1% and R-free was 28%. The final model included amino acid residues 25 to 385 for molecule A, residues 37 to 382 for molecule B, inhibitor2 and 6 water molecules.

For the unphosphorylated GSK-3β-inhibitor3 complex, the structure was refined to 2.3 Å, and the R-factor was 25.3% and R-free was 28.6%. For molecule A, residues 25 to 381 were included in the final model. For molecule B, amino acid residues 37 to 382 were included in the final model.

For the unphosphorylated GSK-3β-inhibitor4 complex, the structure was refined to 2.8 Å, and the R-factor was 24.1% and R-free was 28.3%. For molecule A, residues 36 to 381 were included in the final model. For molecule B, amino acid residues 37 to 382 were included in the final model.

The data statistics of the phosphorylated GSK-3β-inhibitor1 complex are summarized in Table 15. The spacegroup of the crystals was P1, with unit cell dimensions a= 64 Å b=67 Å c= 67 Å $\alpha$= 100° $\beta$= 103° $\gamma$= 89.8° or a= 64 Å b=67 Å c= 67 Å $\alpha$= 80° $\beta$= 77° $\gamma$= 89.8°. The starting phases for phosphorylated GSK-3β-inhibitor complex were obtained by molecular replacement using coordinates of the phosphorylated form as a search model in the program AMORE ENRfu, *supra*. The asymmetric unit contained two molecules. Multiple rounds of rebuilding with QUANTA and refinement with CNX resulted in a final model that included residues 37 to 383 for molecule A and residues 37 to 384 for molecule B, the inihibitors bound to molecule A and B, and 83 water molecules. The structure was refined to 2.8 Å, and had an R-factor of 22.6% and R-free of 26.9%.

The data statistics of the phosphorylated GSK-3β-ADP-peptide complex are summarized in Table 16. The spacegroup of the crystals was $P2_12_12_1$, with unit cell dimensions a= 75.16 Å b= 107.93 Å c= 121.2 Å $\alpha$= 90° $\beta$= 90° $\gamma$= 90°. The starting phases for phosphorylated GSK-3β were obtained by molecular replacement using coordinates of the unphosphorylated form as a search model in the program AMORE ENRfu, *supra.* The asymmetric unit contained two molecules. The glycine rich loop was well ordered and could be built in the model. Multiple rounds of rebuilding with QUANTA and refinement with CNX was performed. All data was included and NCS restraint was applied through out the refinement. The final step of refinement was a grouped B-factor refinement. The refined model has a crystallographic R-factor of 23.5% and R-free of 27.2%.

The Ramachandran plot of phosphorylated GSK-3β in complex with ADP and glycogen synthase peptide showed that 80.1% of the residues were in most favored regions and 19.6% in additionally allowed regions. The Ramachandran plot of apo-phosphorylated GSK-3β showed that 85.3% of the amino acid residues were in most favored regions and 14.3% in additionally allowed regions. Two amino acid residues (Cys 218 in molecule A and B) in both crystal structures were in disallowed regions.

In the above models, disordered residues were not included in the model. Alanine or glycine residues were used in the model if the side chains of certain residues could not be located in the electron density.

## Example 23

## Overall Structure of Unphosphorylated GSK-3β

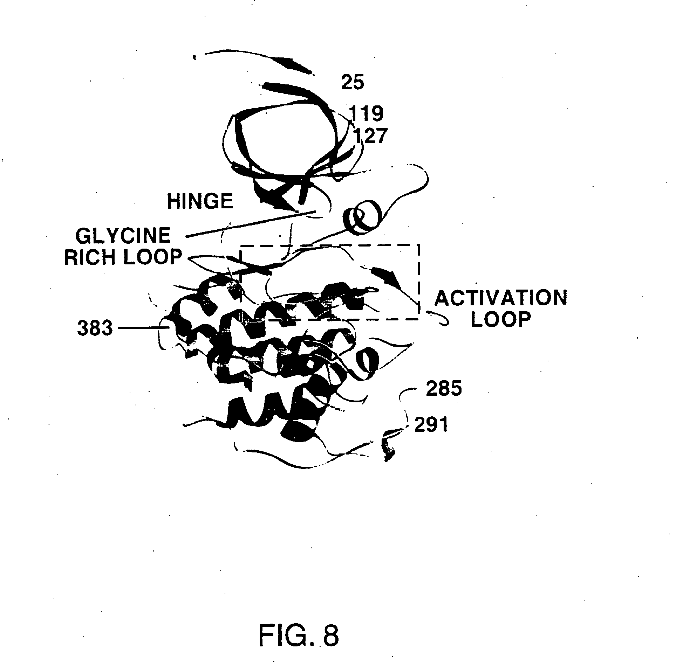

GSK-3β has the typical 2 domain kinase fold (Hanks, S.K., et al., Science, 241, pp. 42-52 (1988); Hanks, S.K. and A.M. Quinn, Methods Enzymol., 200, pp. 38-62 (1991)) with a β-strand domain (amino acid residues 25-138) at the N-terminal end and an α-helical domain at the C-terminal end (amino acid residues 139-343) (Fig. 8). The active site is at the interface of the α-helical and β-strand domain, and is bordered by the glycine rich loop and the hinge. The activation loop runs along the surface of the substrate-binding groove. The C-terminal 39 amino acid residues (amino acid residues 344-382) are outside the core kinase fold and form a small domain that packs against the α-helical domain. The β-strand domain consists of seven anti-parallel β-strands. Strands 2 to 6 form a β-barrel that is interrupted between strand 4 and 5 by a short helix (amino acid residues 96-102) which packs against the β-barrel. This helix is conserved in all kinases, and two of its residues play key roles in the catalytic activity of the enzyme. Arginine 96 is involved in the alignment of the two domains. Glutamate 97 is positioned in the active site and forms a salt bridge with lysine 85, a key residue in catalysis.

Phosphorylation of Peptides by GSK- 3β

Before a serine/threonine kinase can phosphorylate a substrate, its β-strand and α-helical domains must be aligned into a catalytically active conformation. Most kinases use one or two phosphorylated amino acid residues on the activation loop for this purpose. Polar amino acid residues, typically arginines and lysines, from the β-strand and α-helical domains, bind the phosphate group of the phosphorylated amino acid residue on the activation loop, which leads to proper alignment of the two domains. The second phosphorylated amino acid residue (if present, for example Tyr216 in GSK-3β) opens the substrate-binding groove and allows the substrate to bind.

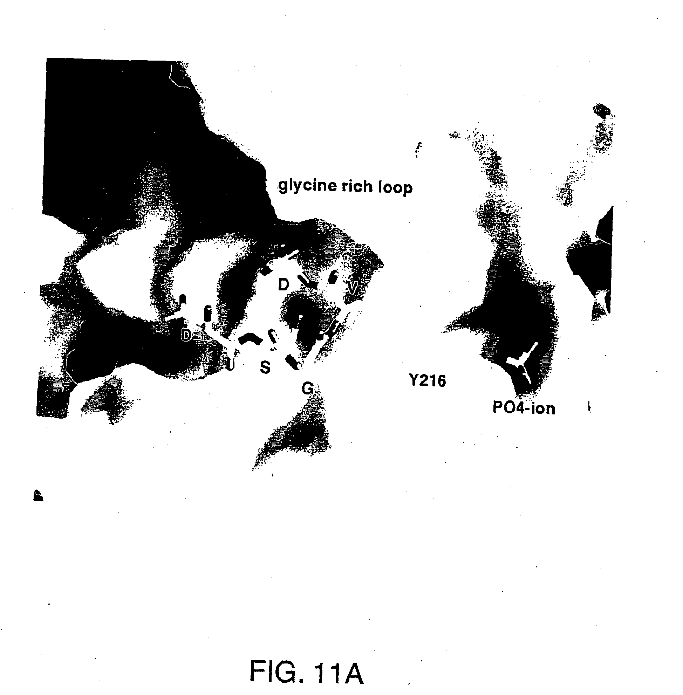

Comparison of the GSK-3β with other kinases such as CDK2, p38γ and ERK2 revealed that the structure of apo-GSK-3β resembles closely the substrate-bound, activated form of a kinase. The activation loop (amino acid residues 200 to 226) in the GSK-3β structure is well ordered, and is positioned against the α-helical domain. This orientation opens the peptide substrate binding groove (Fig. 11), and mimics the position of the activation loop of the activated substrate bound CDK2 (Fig. 10) but not apo-CDK2 (Protein Data Bank Accession number 1HCL) (Schulze-Gahmen, U., et al., Proteins, 22, pp. 378-91 (1995)). Comparison of the activation loops of GSK-3β, P38γ (Protein Data Bank Accession number 1CM8) (Bellon, S., et al., Structure Fold Des, 7, pp. 1057-65 (1999)), substrate-bound activated CDK2 (Protein Data Bank Acession number 1QMZ) (Brown, N.R., et al., Nat. Cell. Biol., 1, pp. 438-443 (1999)), and ERK2 (Protein Data Bank Accession number 2ERK) (Canagarajah, B.J., et al., Cell, 90, pp. 859-69 (1997)) shows that the backbone and side chain atoms of important residues align (Fig. 10). R96, R180 and K205 of GSK-3β (Fig. 10A) superimpose well with R73, R152 and R176 of phosphorylated P38γ, respectively (Fig. 10C). These amino acid residues also superimpose well with the corresponding residues in phosphorylated ERK2 and substrate bound activated CDK2. In p38γ, CDK2 and ERK2, these residues point to the phosphate group from the phosphorylated threonine on the activation loop, the residue that is important for aligning the N-terminal and C-terminal domains. In the GSK-3β structure, R96, R180 and K205 point to a $PO_4^-$ ion that is located in the same position as the phosphate group of the phosphorylated threonine in CDK2, ERK2 and p38γ.

The superposition of the GSK-3β and p38γ activation loops shows that the GSK-3β phosphorylation site, Y216, is located in a similar position as the phospho-tyrosine (amino acid residue 185) of p38γ. The phospho-tyrosine of p38γ acts as a gatekeeper for the substrate-binding groove. When it is phosphorylated, its side chain moves out of the groove allowing substrate peptides to bind. The side chain of Y216 of GSK-3β is also positioned to block access to the substrate-binding groove.

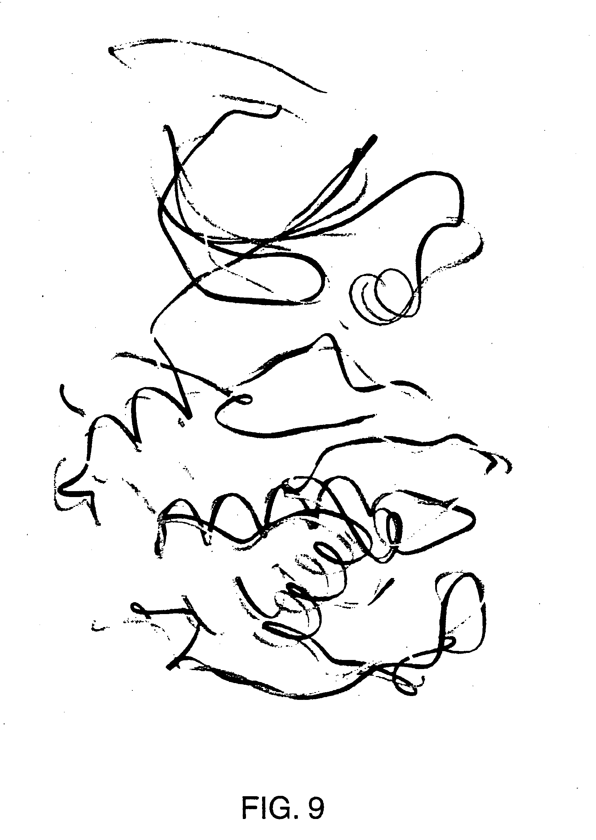

The sequence of GSK-3β is 25% identical and 41% similar to the sequence of CDK2. The structure of GSK-3β presented here superimposes well with the structure of activated, substrate-bound CDK2 (Fig. 9). Because of the similarity in sequence and fold, we can use the structure of activated, substrate-bound CDK2 as a model for the substrate binding of GSK-3β.

Primed Phosphorylation

The GSK-3β substrate-binding groove is partially occupied by a loop from a neighboring GSK-3β molecule in the crystal (Fig. 11A). The loop is positioned in front of the active site. Superposition of the α-helical domains of activated substrate-bound CDK2 and GSK-3β shows that four residues in the loop, DSGV (amino acid residues 260 to 263), are in a very similar position as the peptide in activated substrate-bound CDK2. S261 of the loop in GSK-3β occupies the same position as the target serine in peptide-bound CDK2 (compare position S261 in Fig. 11A with S* in Fig. 11B). The CDK2-bound peptide has an extended conformation, while loop 260-264 in the GSK-3β adopts a turn and occupies a small portion of the substrate-binding groove. It is likely that the natural substrate for GSK-3β also has an extended conformation, similar to the peptide bound to CDK2. If we use the CDK2-bound peptide as a model for a GSK-3β substrate, it becomes clear why GSK-3β prefers a phosphorylated serine or threonine at the P+4 position. The phosphate group at the P+4 position will occupy the same position as the phosphate ion near the activation loop of our structure, contacting R96, R180 and K205 (Fig. 11). This means that while CDK2, p38γ and ERK2 use a phospho-threonine on the activation loop to align the β-strand and α-helical domains, GSK-3β uses the phosphorylated serine at the P+4 position of the substrate to align the two domains for optimal catalytic activity.

Example 24

Active Site of GSK-3β-inhibitor Complexes

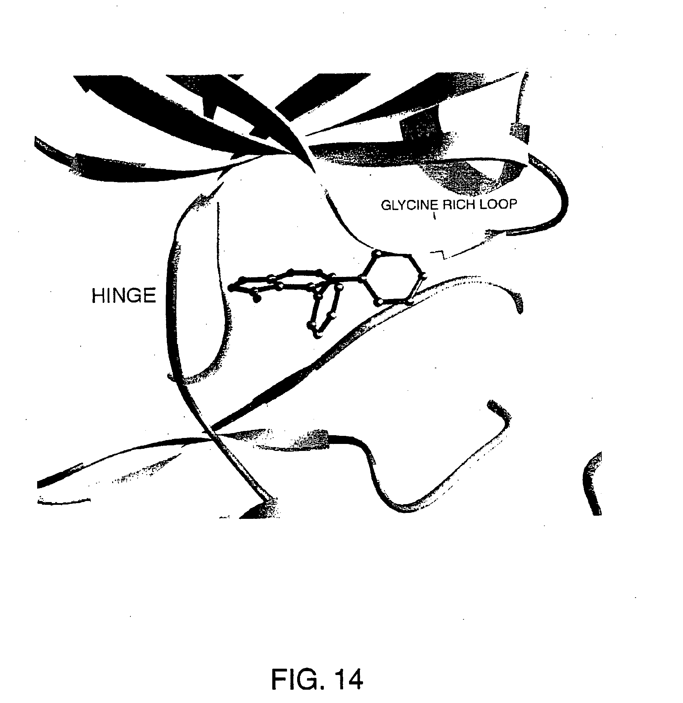

Inhibitor1 4,5-Diphenyl-1H-pyrazolo[3,4-c]pyridazin-3-ylamine is bound in the deep cleft of the active site in the phosphorylated GSK-3β structure (Figure 14). Inhibitor1 forms two hydrogen bonds with the hinge portion of the active site. The 1*H* pyrazole nitrogen shares a proton with the D133 backbone carbonyl. The other pyrazole nitrogen (position 2) accepts a proton from the V135 backbone nitrogen. The side chains of L132 and K85 are positioned inside

the active site. K85 is a catalytically important residue and forms a salt bridge with E97 (not shown).

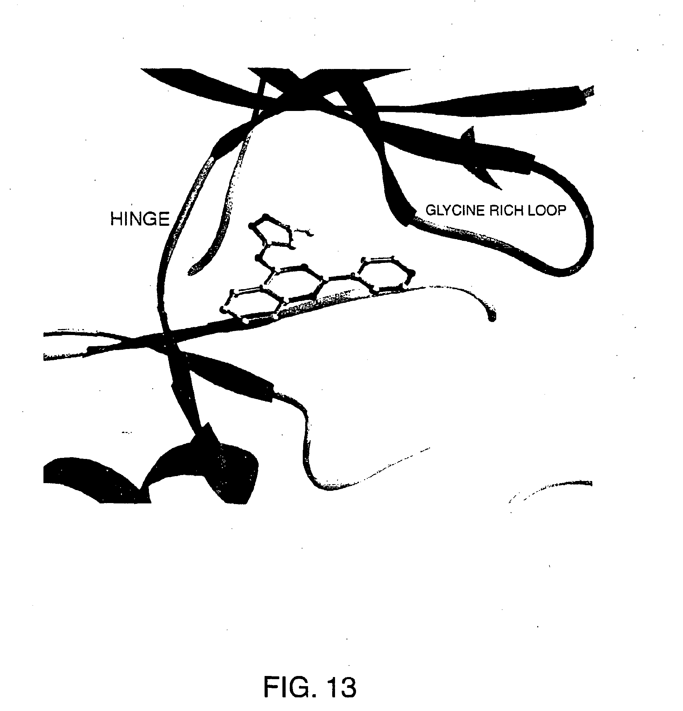

Inhibitor2 (5-Methyl-2*H*-pyrazol-3-yl)-(2-pyridin-4-yl-quinazolin-4-yl)-amine is bound in the active site in the unphosphorylated GSK-3β structure (Figure 13). The inhibitor2 forms four H-bonds with the hinge backbone. Two hydrogen bonds come from the pyrazole ring. The nitrogen in position one donates a hydrogen to the backbone carbonyl of Asp 133. The nitrogen in position 2 accepts a hydrogen from the Val 135 amide nitrogen. The backbone carbonyl of Val 135 is within hydrogen bonding range of hydrogen donating groups on the inhibitor2. It contacts the linker nitrogen and the quinazoline carbon at position 8.

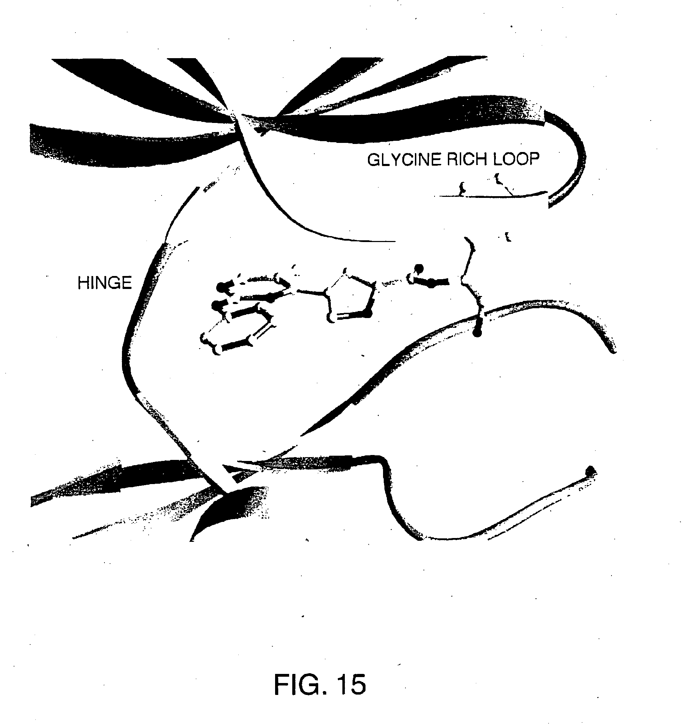

Inhibitor3 4-(5-Methyl-2-phenylamino-pyrimidin-4-yl)-1*H*-pyrrole-2-carboxylic acid (2-hydroxy-1-phenylethyl)-amide) is bound in the active site in the unphosphorylated GSK-3β structure (Figure 15). Inhibitor3 is a potent inhibitor of GSK-3β with a Ki of 4 nM. Inhibitor3 forms 5 hydrogen bonds with the GSK-3β protein. The three aminopyrimidine hydrogen bonds contact the hinge backbone. The carbonyl forms a hydrogen bond with the side chain of the catalytic lysine (K85) and the hydroxyl forms a hydrogen bond with the side chain of asparagine 186. Asn186 is a conserved residue in GSK-3β and ERK2. The 5-methyl group of the amino-pyrimidine ring points toward L132 and V110 in GSK-3β. There is limited space between these two residues suggesting that a small substituent is allowed in this position.

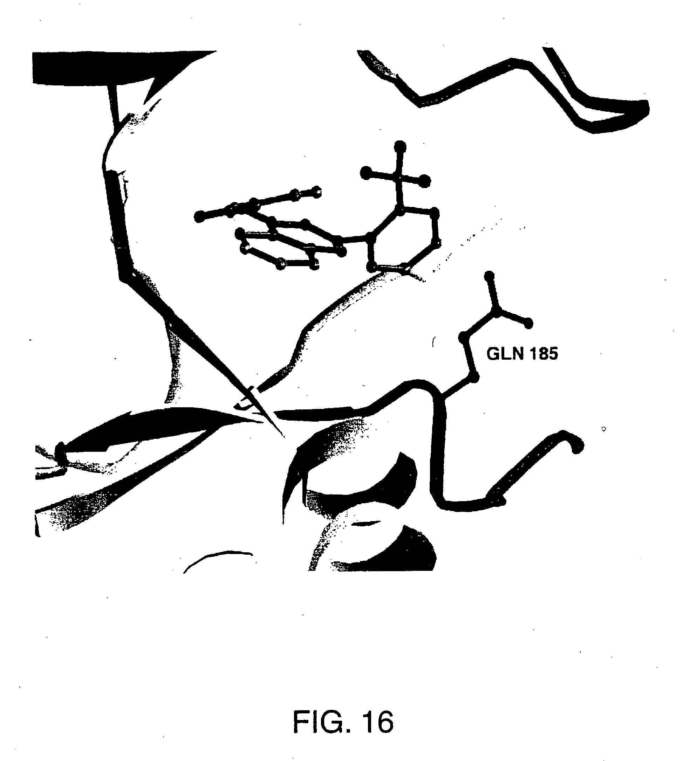

Inhibitor4 (1*H*-Indazol-3-yl)-[2-(2-trifluoromethyl-phenyl)-quinazolin-4-yl]-amine) is bound in the active site in the unphosphorylated GSK-3β structure (Figure 16). The indazole ring of inhibitor4 forms two hydrogen bonds with the hinge backbone. The nitrogen in position one donates a hydrogen to the backbone carbonyl of Asp 133. The nitrogen in position 2 accepts a hydrogen from the Val 135 amide nitrogen. The backbone carbonyl of Val 135 is within hydrogen bonding range of hydrogen donating groups from inhibitor4. It contacts the linker nitrogen and the quinazoline carbon at position 8. The trifluoromethyl phenyl ring is almost perpendicular to the quinazoline ring with the ortho trifluoromethyl substituent pointing to the glycine rich loop. The side chain of glutamine 185 packs against the trifluoromethyl phenyl ring and points to the glycine rich loop.

## Example 25

### Overall Structure of apo-Phosphorylated GSK-3β and Substrate-bound Phosphorylated GSK-3β

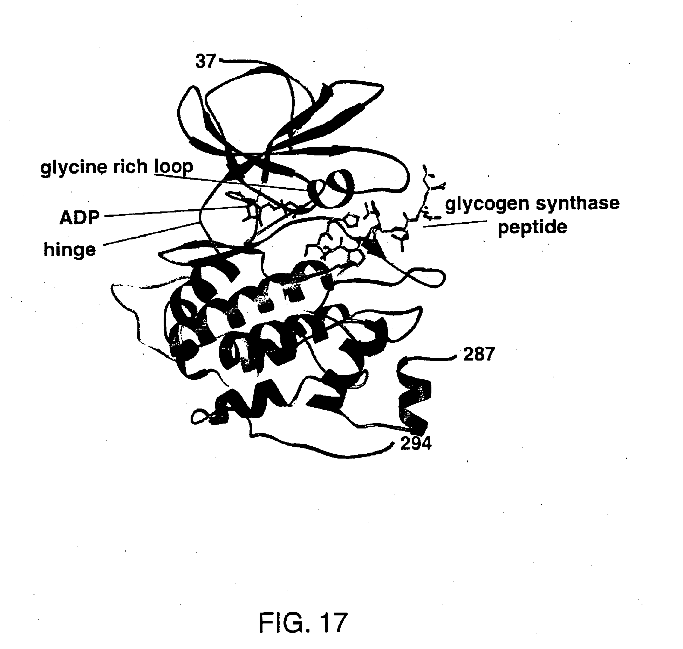

The apo-phosphorylated GSK-3β and substrate-bound phosphorylated GSK-3β structures have the typical two-domain kinase fold (residues 37 to 343) (Hanks, S.K. and Quinn, A.M., Methods Enzymol., 200, pp. 38-62 (1991); Hanks, S.K. and Hunter, T., FASEB J., 9, pp. 576-96 (1995)). The N-terminal β-strand domain (amino acid residues 37 to 138) forms a β-barrel consisting of seven β-strands (Figure 17). The C-terminal α-helical domain contains amino acid residues 139 to 343. The C-terminal 40 amino acid residues (residues 344 to 383) are not part of the kinase fold, but pack against the α-helical domain.

The active site and the substrate-binding groove are located at the interface of the β-strand and α-helical domain. The active site is bordered by the hinge and the glycine-rich loop and contains an ADP-molecule. The substrate-binding groove contains a 12 amino acid residue phosphorylated peptide derived from the sequence in glycogen synthase recognized by GSK-3β (650 HSSPHQpSEDEEE 661). The peptide is positioned between the activation loop (amino acid residues 200 to 226) and the β-strand domain (Figure 17).

Comparison between the structures of unphosphorylated, phosphorylated apo-GSK-3β and phosphorylated peptide-bound GSK-3β reveal local differences induced by the presence of the substrates (Figure'18). Superposition of the protein backbone of unphosphorylated and phosphorylated apo-GSK-3β resulted in a mean displacement of 0.7 Å and a maximum displacement of 3.7 Å of the Ile 217 backbone carbonyl. In the phosphorylated peptide-bound GSK-3β structure, the phosphorylated side chain of Tyr 216 rotated out of the substrate-binding groove and induced a 180° flip of the Ile 217 backbone carbonyl. As a consequence of this adjustment, the torsion angles of Cys 218 appeared in disallowed regions of the Ramachandran plot. In the phosphorylated apo-GSK-3β structure, the side chain of Y216 also flips out of the substrate binding groove (Fig. 10). The N-terminal domain of the phosphorylated peptide-bound GSK-3β rotated by 6.5°, with the glycine rich loop covering the ADP molecule. The reorganization of the glycine rich loop resulted in 4.1 Å translation of the Ser 66 α-carbon. The loop connecting β-3 to α-C (L4, amino acid residues 87 to 95) migrated 13 Å (amino acid residue 92) towards the substrate-binding groove, and form contacts with the backbone atoms of the glycogen synthase peptide. The αG helix (amino acid residues 262 to 273) rotated towards phosphorylated Tyr 216. The rotation of the β-strand domain and the adjustments of the glycine rich loop and L4 were induced by the presence of ADP and the substrate peptide since the α-strand domain of apo-phosphorylated GSK-3β were not rotated in comparison to unphosphorylated GSK-3β.

The loop connecting α1L14 with Trp 301 (amino acid residues 284 to 300) was poorly ordered in the apo- and ADP, peptide-bound structures. This loop is part of the Frat1 binding site (Bax, B. et al., Structure(Camb), 9, pp. 1143-52 (2001)) and covers a hydrophobic groove between αG and amino acid residues 288-294. The loop is separate from the substrate-binding groove and does not seem to influence peptide phosphorylation even when Frat1 is bound to GSK-3β (Thomas, G.M. et al., FEBS Lett., 458, pp. 247-51 (1999)).

Example 26

The Active site of the GSK-3β-ADP-peptide Complex

The active site is located at the interface of the β-strand and α-helical domain and is enclosed by the glycine rich loop and the hinge. The glycine rich loop is formed by two anti-parallel β-strands. The conformation of the glycine rich loop adjusts to the ligand that occupies the active site. The absence of the γ-phosphate in the ADP molecule allows the glycine rich loop to descend closer to the α-helical domain. A similar adjustment of the glycine rich loop was observed in the PKA-ADP complex (Protein Data Bank Accession number 1JBP) (Madhusudan, Trafny, E.A. et al., Protein Sci, 3, pp. 176-87 (1994)).

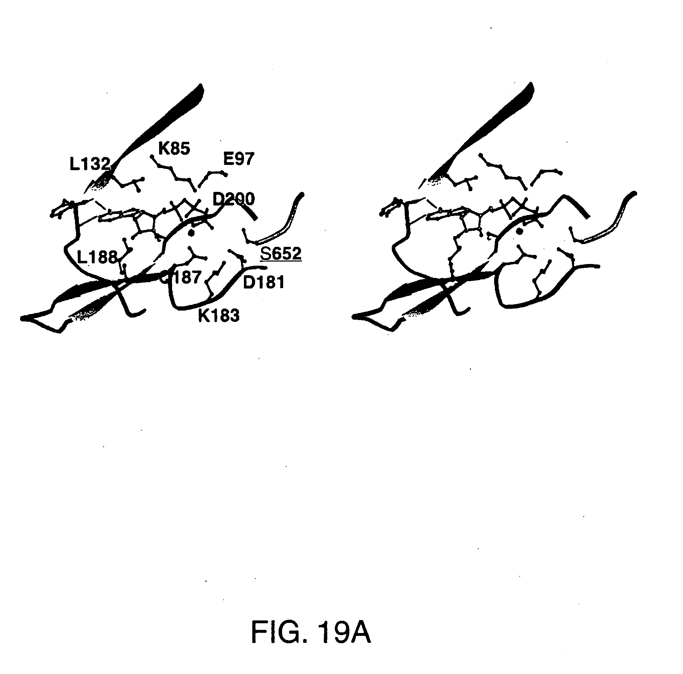

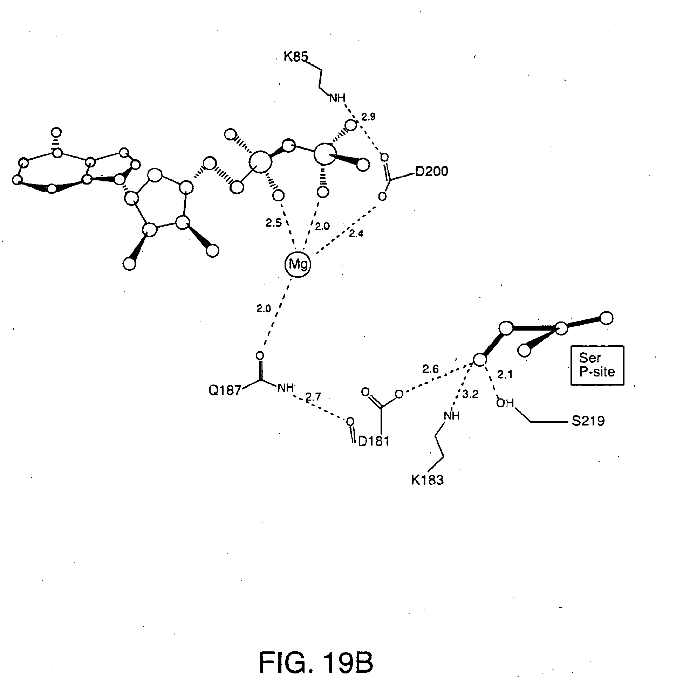

The entire β-strand domain rotated 6.5° as a result of the ADP molecule being bound in the active site. The adenine moiety of ADP is surrounded by hydrophobic amino acid residues from the glycine rich loop (Ile 62, Val 70), from the hinge (Tyr 134) and from the bottom of the active site (Leu 188, Cys 199) (Figure 19A). Two hydrogen bonds between the base and the hinge backbone were observed. The amino group on position 6 forms a hydrogen bond with the backbone carbonyl of Asp 133, and the N1 nitrogen accepts a hydrogen from Val135 amide nitrogen. The 3' hydroxyl from the ADP ribose donates a hydrogen to the backbone carbonyl of Gln 185. The active site residues that play a role in the serine phosphorylation reaction form a web of hydrogen bonds around the ADP phosphates and the glycogen synthase target serine (Figure 19B). The two ADP phosphates form a complex with one Mg ion, which in turn contacts the side chains of Asp 200 and Gln 185. Asp 200 is part of the Asp Phe Gly motif and is the first amino acid residue of the activation loop. The Asp 200 homologue in other kinase-AMPPNP complexes binds a second Mg ion (or Mn ion) that is positioned between the β- and γ- phosphate (Bellon, S. et al., Structure Fold Des., 7, pp. 1057-65 (1999); Xie, X. et al., Structure, 6, pp. 983-91 (1998); Bossemeyer, D. et al., EMBO J, 12, pp. 849-59 (1993)). However, a second Mg ion could not be located in our electron density maps. On the other hand, the structure of the PKA-ADP complex (Protein Data Bank Accession number 1JBP) (Madhusudan, Trafny, E.A. et al., Protein Sci, 3, pp. 176-87 (1994)) does not have any Mg ion associated with the phosphate groups.

Lys 85 is positioned between Glu97 and the α-and β- phosphates and likely facilitates the transfer of the γ-phosphate from ATP to the substrate serine. Asp 181 is also a conserved residue in kinases and its side chain is within hydrogen bonding range of the peptide target serine (Ser 652). The backbone carbonyl of Asp 181 forms a hydrogen bond with the Gln 185 side chain. Asp 181 prepares the serine hydroxyl for the nucleophilic attack on the γ- phosphate (Adams, J.A., Chem. Rev., 101, pp. 2271-2290 (2001)). Lys 183 makes a hydrogen bond with the peptide target serine. Lys 183 is positioned between the phosphate groups and the target serine and is involved in the phosphate transfer. When the γ-phosphate is present in the kinase active site, the Lys 183 is likely to contact the terminal phosphate group.

Example 27

The Substrate-binding Groove of the GSK-3β-AD-peptide Complex

GSK-3β has one phosphorylation site in the activation loop, Y216, which acts as a gate for the substrate-binding groove. In the crystal structure of unphosphorylated GSK-3β, the unphosphorylated tyrosyl side chain occupied the substrate-binding groove. A comparison between the unphosphorylated and phosphorylated peptide-bound GSK-3β structures showed that there would be space to accommodate the glycogen synthase peptide even if the unphosphorylated tyrosine remains in the substrate-binding groove. The P+2 residue of the peptide (His 654) is the closest amino acid residue to Tyr 216. Its imidazole ring is part of a cluster of aromatic residues formed by GSK-3β residues Phe 67, Phe 93 and the peptide substrate amino acid residue His 650.

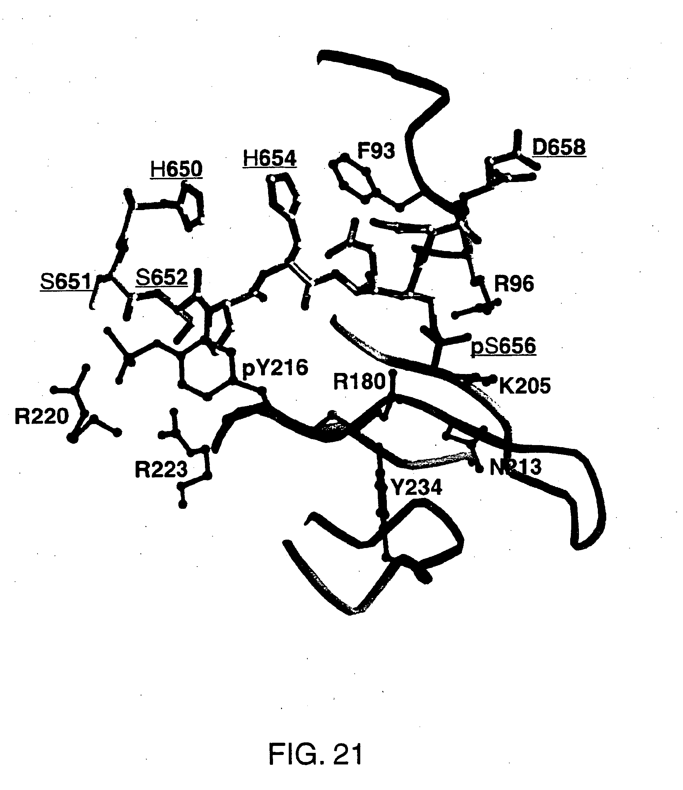

The phosphorylated Tyr216 side chain has moved out of the substrate-binding groove in the GSK-3β-ADP-peptide complex structure (Figure 21). The phospho-phenol group of Tyr 216 is bound to the side chains of Arg 220 and Arg 223, which results in neutralization of the negative charge of the phosphate group and a 180° flip of the Ile 217 backbone carbonyl. The phospho-phenol group also caused a slight adjustment of the αG helix (residues 262-272), which results in the distance between the phosphate oxygen atoms and amide nitrogen of G262 (which is the closest H-bond donor) outside the H-bonding range (4.4 Å).

Glycogen synthase is a major substrate of GSK-3β with multiple phosphorylation sites. The canonical phosphorylation site for GSK-3β is SXXXpS. The P+4 serine (or threonine) is first phosphorylated by a different kinase, which is called primed phosphorylation. In the case of glycogen synthase, GSK-3β phosphorylates four sites sequentially after it is phosphorylated by casein kinase-II. The co-crystallized peptide present in the substrate-binding groove is derived from glycogen synthase. The sequence (650-HSSPHQ(pS)EDEEE-661) contains the canonical GSK-3β phosphorylation motif and GSK-3β can phosphorylate Ser 652 under the appropriate conditions. Ten of the twelve residues (residues

650 to 659) had visible electron density and were built in the substrate-binding groove (Figure 21). The peptide has the shape of a large loop with residues 651 to 656 fitting in the substrate binding groove and residues 650, 657 to 659 exposed to solvent. The structure reveals why GSK-3β prefers a phosphorylated serine or threonine at the P+4 position of the glycogen synthase phosphorylation site. The phosphate group of Ser 656 occupies a positively surface charged pocket formed by residues Arg 96, Arg 180 and Lys 205. These amino acid residues are conserved in serine/threonine kinases and are responsible for the proper alignment of the β-strand and α-helical domains, the latter of which is required for optimal catalytic activity. Other serine/threonine kinases such as CDK2 (Schulze-Gahmen, U. et al., Proteins, 22, pp. 378-91 (1995)), ERK2 (Zhang, F. et al., Nature, 367, pp. 704-11 (1994); Canagarajah, B.J. et al., Cell, 90, pp. 859-69 (1997)) and p38γ (Bellon, S. et al., Structure Fold Des., 7, pp. 1057-65 (1999)) have a phosphorylated threonine in the activation loop for this purpose, but GSK-3 uses a phosphorylated residue from the substrate. The target serine (Ser 652) is positioned in front of the active site, 6.0 Å from the β-phosphate of the ADP molecule. The loop between residues Lys 85 and Arg 96 migrates more than 10 Å to facilitate the glycogen synthase peptide binding. Phe 93 forms a pi-stack with the peptide His 654. Ser 66, which is at the tip of the glycine rich loop, makes a hydrogen bond with the target serine backbone-nitrogen (Ser 652). The backbone carbonyl of pSer 656 forms a hydrogen bond with Lys 94.

The canonical phosphorylation motif for GSK-3β is SXXXpS. There is no sequence requirement for the three residues between the target serine and the phosphoserine. The P+1 residue in the glycogen synthase peptide used here is a proline. Although prolines are not uncommon as the P+1 residue in phosphorylation motifs, the GSK-3β phosphorylation does not require a proline. A sequence analysis of the phosphorylation motifs of the proteins that undergo primed phosphorylation shows that residues such as A, G, Q, E, S, T, R and V can replace the proline at the P+1 position. In the GSK-3β-ADP-peptide structure, the proline side chain fits in a pocket formed by the side chains of pY216, R220, R223 and the backbones of residues 217 to 219. The pocket is shallow, and the distance between the Cγ of Pro 654 and Arg 223 is 3.8 Å. This pocket can only accommodate small residues such as Ala, Ser or Val. Larger residues, such as Arg and Gln will have to point their side chains into the solvent. This might explain the absence of bulky hydrophobic residues at the P+1 position. This fact is reflected in the interactions between the glycogen synthase peptide and GSK-3β. Except for the phosphoserine side chain interactions with the basic residues, the other interactions are with the peptide backbone. The pi-stack interaction between His 654 and Phe 93 is probably specific for the phosphorylation of Ser 652, because there is no aromatic residue at the P+2 position in other motifs of glycogen synthase or eIF2b.

The crystal structure of the GSK-3β-ADP-peptide complex provides a detailed picture of the phosphorylation of the glycogen synthase peptide. Although the ADP molecule does not contain the γ-phosphate, the catalytic residues are located around the ADP molecule similar to the positions in other kinase nucleotide complexes. The phosphoserine of the peptide serves as an anchor for the peptide and is required for the proper alignment of the β-strand and α-helical domains of GSK-3β.

Biological Implications

Activation of the insulin-signaling pathway induces increased glucose uptake and conversion to glycogen. Patients with type II diabetes have decreased sensitivity towards insulin, which reduces glycogen synthesis and increased blood glucose levels. The conversion of glucose into glycogen by glycogen synthase is the rate limiting step in glycogen synthesis and the phosphorylation status of glycogen synthase determines the catalytic rate. Glycogen synthase has at least nine phosphorylation sites and the more it is phosphorylated the lower its catalytic activity. GSK-3β is one of the kinases that phosphorylates and inhibits glycogen synthase. It phosphorylates sequentially multiple sites at the C-terminal end of glycogen synthase after Casein Kinase II phosphorylates glycogen synthase. The canonical sequence recognized by GSK-3β is SXXXpS, which is four times present in glycogen synthase. GSK-3β is a potential therapeutic target for type 2 diabetes because its inhibition leads to increased glycogen synthesis and decreased blood glucose levels.

The crystal structures of apo-phosphorylated GSK-3β and GSK-3β in complex with ADP and glycogen synthase peptide provide insight on how GSK-3α phosphorylates its substrates. The ADP molecule occupies the active site and the glycogen synthase peptide occupies the substrate-binding groove. The phosphorylated serine at the P+4 position of the glycogen synthase peptide binds three well-conserved basic residues, which results in optimal alignment of the β-strand and α-helical domains of the GSK-3β kinase core. Other interactions between GSK-3β and the glycogen synthase peptide involve mostly backbone atoms of the peptide, which might explain the tolerance for different residues at position P+1, P+2 and P+3. The present invention will be helpful in understanding the role of GSK-3β in the insulin-signaling pathway and development of potential new anti-diabetic therapies.

Example 28

The Use of GSK-3β coordinates for Inhibitor Design

The coordinates of any one of Figures 1-7 are used to design compounds, including inhibitory compounds,

that associate with GSK-3β or GSK-3β homologues. This process may be aided by using a computer comprising a machine-readable data storage medium encoded with a set of machine-executable instructions, wherein the recorded instructions are capable of displaying a three-dimensional representation of the GSK-3β, the GSK-3β homologues or portions thereof. The graphical representation is used according to the methods described herein to design compounds. Such compounds associate with the GSK-3β or GSK-3β homologue at the active site or substrate binding pocket.

While we have described a number of embodiments of this invention, it is apparent that our basic constructions may be altered to provide other embodiments which utilize the products, processes and methods of this invention. Therefore, it will be appreciated that the scope of this invention is to be defined by the appended claims, rather than by the specific embodiments which have been presented by way of example.

United States provisional applications 60/287,366, 60/361,899, 60/297,094 are incorporated herein by reference in their entirety.

TABLE 12

| Summary of data collection | | |
|---|---|---|
| Source | R-Axis IV | ALS 5.0.2 |
| Wavelength (Å) | 1.54 | 1.1 |
| Resolution (Å) | 3.0 | 2.7 |
| No. of Reflections (measured/unique) | 303687/26039 | 251279/34993 |
| Completeness (%) (overall/outer shell). | 96.7/89.3 | 98.9/99.8 |
| $R_{merge}$(%) [1] (overall/outer shell) | 0.064/0.30 | 0.070/0.32 |
| Structure refinement | | |
| Resolution (Å) | 48.3-2.7 | |
| No. of reflections | 34747 | |
| R factor | 23.7 | |
| Free R factor † | 27.4 | |
| Rms deviations<br><br>Bond lengths<br>Bond angles | <br><br>0.01<br>1.5° | |

[1] $R_{merge} = 100 \times \sum_h \sum_i |I_{hi} - \langle I_h \rangle| / \sum_h \sum_i I_{hi}$.

† The Free R factor was calculated with 9.1% of the data.

TABLE 13

| Summary of data collection | |
|---|---|
| Source | R-Axis IV |
| Wavelength (Å) | 1.54 |
| Resolution (Å) | 2.5 |
| No. of Reflections (measured/unique) | 32357/3447 |
| Completeness (%) (overall/outer shell) | 87.0/61.8 |
| I/δ(I) (overall/outer shell) | 10.4/2.3 |
| $R_{merge}$(%) (overall/outer shell) | 0.064/0.30 |
| Structure refinement | |
| Resolution (Å) | 42.8-2.5 |
| No. of reflections | 32357 |

(continued)

| Structure refinement | |
|---|---|
| R factor | 23.8 |
| Free R factor [1] | 27.6 |
| Rms deviations<br><br>Bond lengths<br>Bond angles<br>Bfactor (average)[2] | <br><br>0.008<br>1.5°<br>45 Å |
| [1] The Free R factor was calculated with 10% of the data.<br>[2] The B-factor of the data (Wilson plot) was 26.4 $Å^2$. | |

TABLE 14

| Summary of data collection | |
|---|---|
| Source | R-Axis IV |
| Wavelength (Å) | 1.54 |
| Resolution (Å) | 2.9 |
| No. of Reflections (measured/unique) | 338559/26836 |
| Completeness (%) (overall/outer shell) | 93.1/85.1 |
| 1/δ(I) (overall/outer shell) | 10.2/2.7 |
| $R_{merge}$(%) (overall/outer shell) | 0.072/0.29 |
| Structure refinement | |
| Resolution (Å) | 25.7-2.9 |
| No. of reflections | 26796 |
| R factor | 24.0 |
| Free R factor [1] | 27.9 |
| Rms deviations<br><br>Bond lengths<br>Bond angles<br>Bfactor (average)[2] | <br><br>0.011<br>1.8°<br>39.3 Å |
| [1] The Free R factor was calculated with 9.4% of the data.<br>[2] The B-factor of the data (Wilson plot) was 33 $Å^2$. | |

TABLE 15

| Summary of data collection | |
|---|---|
| Source | R-Axis IV |
| Wavelength (Å) | 1.54 |
| Resolution (Å) | 2.8 |
| No. of Reflections (measured/unique) | 71493/23161 |
| Completeness (%) (overall/outer shell) | 88.3/90.3 |

(continued)

| Summary of data collection | |
|---|---|
| 1/δ(I) (overall/outer shell) | 14.7/2.2 |
| $R_{merge}$(%) (overall/outer shell) | 0.046/0.33 |
| Structure refinement | |
| Resolution (Å) | 32.2-2.8 |
| No. of reflections | 23148 |
| R factor | 22.5 |
| Free R factor [1] | 26.8 |
| Rms deviations<br><br>Bond lengths<br>Bond angles<br>Bfactor (average)[2] | <br><br>0.013<br>1.6°<br>51.1 Å |
| [1] The Free R factor was calculated with 9.4% of the data.<br>[2] The B-factor of the data (Wilson plot) was 91.3 $Å^2$. | |

TABLE 16

| Summary of data collection | |
|---|---|
| Source | R-Axis IV |
| Wavelength (Å) | 1.54 |
| Resolution (Å) | 2.8 |
| No. of Reflections (measured/unique) | 311458/24709 |
| Completeness (%) (overall/outer shell) | 99.4/99.8 |
| I/δ(I) (overall/outer shell) | 18.4/3.6 |
| $R_{merge}$(%) (overall/outer shell) | 0.075/0.33 |
| Structure refinement | |
| Resolution (Å) | 49.3-2.8 |
| No. of reflections | 24288 |
| R factor | 23.5 |
| Free R factor [1] | 27.2 |
| Rms deviations<br><br>Bond lengths<br>Bond angles | <br><br>0.013<br>1.7° |
| [1] The Free R factor was calculated with 9.4% of the data. | |

Items

1. A compound of formula I:

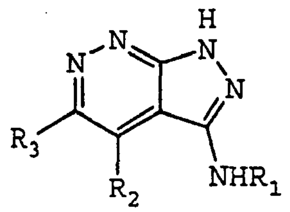

I

or a pharmaceutically acceptable derivative thereof, wherein:

$R_1$ is selected from H, aliphatic, RC(O)-, $RS(O)_n$-, ROC(O)-, carbocyclyl, heterocyclyl, aryl, aralkyl, heteroaryl, or heteroaralkyl; wherein said aliphatic, carbocyclyl, heterocyclyl, aryl, aralkyl, heteroaryl, or heteroaralkyl is optionally substituted;
$R_2$ and $R_3$ are each independently selected from H, aliphatic, carbocyclyl, heterocyclyl, aryl, aralkyl, heteroaryl, heteroaralkyl, $-N(R)_2$, -NRCOR, $-NRCO_2R$, $-NRSO_2R$, $-S(O)_nR$, $-SO_2N(R)_2$, -SR, -OR, $-CF_3$, halo, $-NO_2$, -CN, -C(O)R, $-CO_2R$, -OC(O)R, $-CON(R)_2$, or $-OC(O)N(R)_2$, wherein said aliphatic, carbocyclyl, heterocyclyl, aryl, aralkyl, heteroaryl, or heteroaralkyl is optionally substituted; or wherein $R_2$ and $R_3$ taken together with the intervening atoms optionally form a five- to nine-membered ring that is fused to the pyridazinyl ring of formula I, said fused ring having 0-2 heteroatoms;
R is selected from H, aliphatic, carbocyclyl, heterocyclyl, aryl, aralkyl, heteroaryl, or heteroaralkyl, wherein each member of R except H is optionally substituted; and
n is 1 or 2;
provided that when $R_1$ is H, $R_2$ and $R_3$ are not both unsubstituted phenyl; and when $R_1$ is H, $R_2$ and $R_3$ are other than H, halogen, or an unsubstituted alkyl.

2. The compound according to item 1, wherein $R_1$ is H, RC(O)-, or aralkyl.

3. The compound according to item 2, wherein R is aliphatic or aryl.

4. The compound according to item 2, wherein $R_1$ is H, $CH_3C(O)$-, PhC(O)-, or $PhCH_2$-.

5. The compound according to item 1, wherein $R_2$ and $R_3$ are independently H, aryl, carbocyclyl, heterocyclyl, or heteroaryl.

6. The compound according to item 1, wherein $R_2$ and $R_3$ are independently aryl, carbocyclyl, heterocyclyl, or heteroaryl.

7. The compound according to item 5, wherein $R_2$ and $R_3$ are independently H, phenyl, naphthyl, pyridyl, thienyl, furanyl, pyrimidinyl, benzodioxolyl, or cyclohexyl, any of which except H is optionally substituted.

8. The compound according to item 6, wherein $R_2$ and $R_3$ are independently phenyl, naphthyl, pyridyl, thienyl, furanyl, pyrimidinyl, benzodioxolyl, or cyclohexyl, any of which is optionally substituted.

9. The compound according to item 7 or 8, wherein the substituents are selected from halo, alkyl, -CN, $-NO_2$, $-SO_2NH_2$, $-SO_2NH$-(alkyl), $-SO_2N(alkyl)_2$, -O-alkyl,- $NH_2$, -N-alkyl, $-N-(alkyl)_2$, $-CONH_2$, -CONH(alkyl), -CONH $(alkyl)_2$, -O-phenyl, or -S-alkyl.

10. The compound according to item 1 having formula Ia:

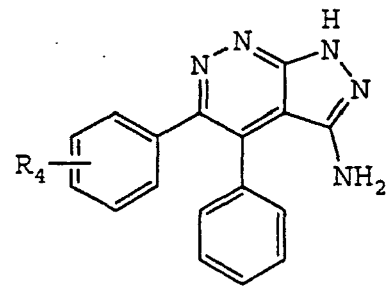

wherein $R_4$ is halo.

11. The compound according to item 10, wherein $R_4$ is F.

12. The compound according to item 1, wherein $R_1$ is H, $R_2$ and $R_3$ are independently H or an optionally substituted phenyl.

13. A compound selected from the group consisting of:

3-amino-4(4-chlorophenyl)-5-phenyl-1H-pyrazolo[3,4-c]pyridazine (Compound 2);
3-amino-4,5-bis(4-fluorophenyl)-1H-pyrazolo[3,4-c] pyridazine (Compound 3);
3-amino-4-phenyl-5-(4-fluorophenyl)-1H-pyrazolo[3,4-c] pyridazine (Compound 4);
3-amino-4-(4-fluorophenyl)-5-phenyl-1H-pyrazolo[3,4-c] pyridazine (Compound 5);
3-amino-4-(3-fluorophenyl)-5-phenyl-1H-pyrazolo[3,4-c] pyridazine (Compound 6);
3-amino-4-phenyl-5-(4-pyridyl)-1H-pyrazolo[3,4-c] pyridazine (Compound 7);
3-amino-4-phenyl-5-(3-fluorophenyl)-1H-pyrazolo[3,4-c] pyridazine (Compound 8);
N-(4,5-diphenyl-1H-pyrazolo[3,4-c] pyridazin-3-yl)-acetamide (Compound 9);
N-(4,5-diphenyl-1H-pyrazolo[3,4-c] pyridazin-3-yl)-benzamide (Compound 10);
3-amino-4-phenyl-5-(4-methyl-phenyl)-1H-pyra-zolo[3,4-c] pyridazine (Compound 11);
3-amino-4-phenyl-5-(2-methyl-phenyl)-1H-pyrazolo[3,4-c] pyridazine (Compound 12);
3-amino-4-phenyl-5-(3-methyl-phenyl)-1H-pyrazolo[3,4-c] pyridazine (Compound 13);
3-amino-4-phenyl-5-(2-chloro-phenyl)-1H-pyrazolo[3,4-c] pyridazine (Compound 14);
3-amino-4-phenyl-5-(2-fluorophenyl)-1H-pyrazolo[3,4-c] pyridazine (Compound 15);
3-amino-4-phenyl-5-(4-chloro-phenyl)-1H-pyrazolo[3,4-c] pyridazine (Compound 16);
3-amino-4(2-cyanophenyl)-5-phenyl-1H-pyrazolo[3,4-c]pyridazine (Compound 17);
3-amino-4(3-cyanophenyl)-5-phenyl-1H-pyrazolo[3,4-c]pyridazine (Compound 18);
3-amino-4(4-cyanophenyl)-5-phenyl-1H-pyrazolo[3,4-c]pyridazine (Compound 19);
3-amino-4-phenyl-5-(2-cyanophenyl)-1H-pyrazolo[3,4-c] pyridazine (Compound 20);
3-amino-4-phenyl-5-(3-cyanophenyl)-1H-pyrazolo[3,4-c] pyridazine (Compound 21);
3-amino-4-phenyl-5-(4-cyanophenyl)-1H-pyrazolo[3,4-c] pyridazine (Compound 22);
3-amino-4-phenyl-5-(2-pyridyl)-1H-pyrazolo[3,4-c] pyridazine (Compound 23);
3-amino-4-phenyl-5-(3-pyridyl)-1H-pyrazolo[3,4-c] pyridazine (Compound 24);
N-(4,5-diphenyl-1H-pyrazolo[3,4-c] pyridazin-3-yl)-benzylamine (Compound 25);
3-amino-4(2-pyridyl)-5-phenyl-1H-pyrazolo[3,4-c] pyridazine (Compound 26);
3-amino-4(3-pyridyl)-5-phenyl-1H-pyrazolo[3,4-c] pyridazine (Compound 27);
3-amino-4(4-pyridyl)-5-phenyl-1H-pyrazolo[3,4-c] pyridazine (Compound 28);
3-amino-4-phenyl-5-(2-thienyl)-1H-pyrazolo[3,4-c] pyridazine (Compound 29);
3-amino-4(2-furanyl)-5-phenyl-1H-pyrazolo[3,4-c] pyridazine (Compound 30);
3-amino-4-phenyl-5-naphthyl-1H-pyrazolo[3,4-c]pyridazine (Compound 31);
4-phenyl-1H-pyrazolo[3,4-c]pyridazin-3-ylamine (Compound 32);
5-phenyl-1H-pyrazolo[3,4-c]pyridazin-3-ylamine (Compound 33);
5-phenyl-4-o-tolyl-1H-pyrazolo[3,4-c] pyridazin-3-ylamine (Compound 35);
5-phenyl-4-m-tolyl-1H-pyrazolo[3,4-c] pyridazin-3-ylamine (Compound 36);
5-phenyl-4-p-tolyl-1H-pyrazolo[3,4-c] pyridazin-3-ylamine (Compound 37);
4-(4-nitro-phenyl)-5-phenyl-1H-pyrazolo[3,4-c]pyridazin-3-ylamine (Compound 38);
4-(3-amino-5-phenyl-1H-pyrazolo[3,4-c] pyridazin-4-yl)-benzenesulfonamide (Compound 39);

4-(3-fluoro-4-methyl-phenyl)-5-phenyl-1H-pyrazolo[3,4-c]pyridazin-3-ylamine (Compound 40);
4-(3-amino-4-phenyl-1H-pyrazolo[3,4-c] pyridazin-5-yl)-benzamide (Compound 41);
5-(2-amino-phenyl)-4-phenyl-1H-pyrazolo[3,4-c]pyridazin-3-ylamine (Compound 42);
4-(4-ethyl-phenyl)-5-phenyl-1H-pyrazolo[3,4-c]pyridazin-3-ylamine (Compound 43);
4-(2,6-difluoro-phenyl)-5-phenyl-1H-pyrazolo[3,4-c]pyridazin-3-ylamine (Compound 44);
4-(3,4-dimethoxy-phenyl)-5-phenyl-1H-pyrazolo[3,4-c]pyridazin-3-ylamine (Compound 45);
4-(3-fluoro-phenyl)-5-phenyl-1H-pyrazolo[3,4-c]pyridazin-3-ylamine (Compound 46);
4-(3-nitro-phenyl)-5-phenyl-1H-pyrazolo[3,4-c]pyridazin-3-ylamine (Compound 47);
5-(2-chloro-phenyl)-4-(2,6-difluoro-phenyl)-1H-pyrazolo[3,4-c]pyridazin-3-ylamine (Compound 48);
5-(2-chloro-phenyl)-4-(3,4-dimethoxy-phenyl)-1H-pyrazolo[3,4-c]pyridazin-3-ylamine (Compound 49);
5-(2-chloro-phenyl)-4-(3-fluoro-phenyl)-1H-pyrazolo[3,4-c]pyridazin-3-ylamine (Compound 50);
5-(2-chloro-phenyl)-4-(4-dimethylamino-phenyl)-1H-pyrazolo[3,4-c]pyridazin-3-ylamine (Compound 51);
4-[3-amino-5-(2-chloro-phenyl)-1H-pyrazolo[3,4-c]pyridazin-4-yl]-benzonitrile (Compound 52);
5-(2-chloro-phenyl)-4-pyridin-4-yl-1H-pyrazolo[3,4-c]pyridazin-3-ylamine (Compound 53);
5-(2-chloro-phenyl)-4-(3-nitro-phenyl)-1H-pyrazolo[3,4-c]pyridazin-3-ylamine (Compound 54);
5-(2-chloro-phenyl)-4-pyridin-3-yl-1H-pyrazolo[3,4-c]pyridazin-3-ylamine (Compound 55);
4-(2,6-difluoro-phenyl)-5-(3-methoxy-phenyl)-1H-pyrazolo[3,4-c]pyridazin-3-ylamine (Compoud 56);
4-(3,4-dimethoxy-phenyl)-5-(3-methoxy-phenyl)-1H-pyrazolo[3,4-c]pyridazin-3-ylamine (Compound 57);
4-(3-fluoro-phenyl)-5-(3-methoxy-phenyl)-1H-pyrazolo[3,4-c]pyridazin-3-ylamine (Compound 58);
4-(4-dimethylamino-phenyl)-5-(3-methoxy-phenyl)-1H-pyrazolo[3,4-c]pyridazin-3-ylamine (Compound 59);
4-[3-amino-5-(3-methoxy-phenyl)-1H-pyrazolo[3,4-c]pyridazin-4-yl]-benzonitrile (Compound 60) ;
5-(3-methoxy-phenyl)-4-pyridin-4-yl-1H-pyrazolo[3,4-c]pyridazin-3-ylamine (Compound 61);
5-(3-methoxy-phenyl)-4-(3-nitro-phenyl)-1H-pyrazolo[3,4-c]pyridazin-3-ylamine (Compound 62);
5-(3-methoxy-phenyl)-4-pyridin-3-yl-1H-pyrazolo[3,4-c]pyridazin-3-ylamine (Compound 63);
4-(2,6-difluoro-phenyl)-5-p-tolyl-1H-pyrazolo[3,4-c]pyridazin-3-ylamine (Compound 64);
4-(3,4-dimethoxy-phenyl)-5-p-tolyl-1H-pyrazolo[3,4-c]pyridazin-3-ylamine (Compound 65);
4-(3-fluoro-phenyl)-5-p-tolyl-1H-pyrazolo[3,4-c]pyridazin-3-ylamine (Compound 66);
4-(4-dimethylamino-phenyl)-5-p-tolyl-1H-pyrazolo[3,4-c]pyridazin-3-ylamine (Compound 67);
4-(3-amino-5-p-tolyl-1H-pyrazolo[3,4-c]pyridazin-4-yl)-benzonitrile (Compound 68);
4-pyridin-4-yl-5-p-tolyl-1H-pyrazolo[3,4-c]pyridazin-3-ylamine (Compound 69);
4-(3-nitro-phenyl)-5-p-tolyl-1H-pyrazolo[3,4-c]pyridazin-3-ylamine (Compound 70);
4-pyridin-3-yl-5-p-tolyl-1H-pyrazolo[3,4-c]pyridazin-3-ylamine (Compound 71);
3-(3-amino-5-benzo[1,3]dioxol-5-yl-1H-pyrazolo[3,4-c]pyridazin-4-yl)-benzonitrile (Compound 72);
3-[3-amino-5-(3-chloro-phenyl)-1H-pyrazolo[3,4-c]pyridazin-4-yl]-benzonitrile (Compound 73);
4-[3-amino-4-(3-cyano-phenyl)-1H-pyrazolo[3,4-c]pyridazin-5-yl]-benzamide (Compound 74);
3-[3-amino-5-(3-nitro-phenyl)-1H-pyrazolo[3,4-c]pyridazin-4-yl]-benzonitrile (Compound 75);
3-[3-amino-4-(3-cyano-phenyl)-1H-pyrazolo[3,4-c]pyridazin-5-yl]-benzamide (Compound 76);
3-(3-amino-5-p-tolyl-1H-pyrazolo[3,4-c]pyridazin-4-yl)-benzonitrile (Compound 77);
3-[3-amino-5-(3-phenoxy-phenyl)-1H-pyrazolo[3,4-c]pyridazin-4-yl]-benzonitrile (Compound 78);
4-[3-amino-4-(3-methoxy-phenyl)-1H-pyrazolo[3,4-c]pyridazin-5-yl]-benzonitrile (Compound 79);
5-benzo[1,3]dioxol-5-yl-4-(4-chloro-phenyl)-1H-pyrazolo[3,4-c]pyridazin-3-ylamine (Compound 80);
4-(4-chloro-phenyl)-5-m-tolyl-1H-pyrazolo[3,4-c]pyridazin-3-ylamine (Compound 81);
4-[3-amino-4-(4-chloro-phenyl)-1H-pyrazolo[3,4-c]pyridazin-5-yl]-benzamide (Compound 82);
4-(4-chloro-phenyl)-5-(3-nitro-phenyl)-1H-pyrazolo[3,4-c]pyridazin-3-ylamine (Compound 83);
3-[3-amino-4-(4-chloro-phenyl)-1H-pyrazolo[3,4-c]pyridazin-5-yl]-benzamide (Compound 84);
4-(4-chloro-phenyl)-5-(3-phenoxy-phenyl)-1H-pyrazolo[3,4-c]pyridazin-3-ylamine (Compound 85);
4-[3-amino-4-(4-chloro-phenyl)-1H-pyrazolo[3,4-c]pyridazin-5-yl]-benzonitrile (Compound 86);
5-benzo[1,3]dioxol-5-yl-4-o-tolyl-1H-pyrazolo[3,4-c]pyridazin-3-ylamine (Compound 87);
5-(3-chloro-phenyl)-4-o-tolyl-1H-pyrazolo[3,4-c]pyridazin-3-ylamine (Compound 88);
4-(3-amino-4-o-tolyl-1H-pyrazolo[3,4-c] pyridazin-5-yl)-benzamide (Compound 89);
5-(3-nitro-phenyl)-4-o-tolyl-1H-pyrazolo[3,4-c]pyridazin-3-ylamine (Compound 90);
3-(3-amino-4-o-tolyl-1H-pyrazolo[3,4-c]pyridazin-5-yl)-benzamide (Compound 91);
4-o-tolyl-5-p-tolyl-1H-pyrazolo[3,4-c]pyridazin-3-ylamine (Compound 92);
5-(3-phenoxy-phenyl)-4-o-tolyl-1H-pyrazolo[3,4-c]pyridazin-3-ylamine (Compound 93);
4-(3-amino-4-o-tolyl-1H-pyrazolo[3,4-c]pyridazin-5-yl)-benzonitrile (Compound 94);
5-benzo[1,3]dioxol-5-yl-4-phenyl-1H-pyrazolo[3,4-c]pyridazin-3-ylamine (Compound 95);
5-(3-chloro-phenyl)-4-phenyl-1H-pyrazolo[3,4-c]pyridazin-3-ylamine (Compound 96);
5-(3-nitro-phenyl)-4-phenyl-1H-pyrazolo[3,4-c]pyridazin-3-ylamine (Compound 97);

3-(3-amino-4-phenyl-1H-pyrazolo[3,4-c]pyridazin-5-yl)-benzamide (Compound 98);
5-(4-tert-butyl-phenyl)-4-phenyl-1H-pyrazolo[3,4-c]pyridazin-3-ylamine (Compound 99);
5-(3-phenoxy-phenyl)-4-phenyl-1H-pyrazolo[3,4-c]pyridazin-3-ylamine (Compound 100);
4-cyclohexyl-5-phenyl-1H-pyrazolo[3,4-c]pyridazin-3-ylamine (Compound 101); and
4-(2-Methylsulfanyl-pyrimidin-4-yl)-5-phenyl-1H-pyrazolo[3,4-c]pyridazin-3-ylamine (Compound 102).

14. The compound according to item 13, wherein the compound is:

3-amino-4-phenyl-5-(3-fluorophenyl)-1H-pyrazolo[3,4-c] pyridazine (Compound 8).

15. A pharmaceutical composition comprising a compound according to any one of items 1-14 and a pharmaceutically acceptable carrier, adjuvant, or vehicle.

16. A method for inhibiting GSK-3 activity in a patient comprising the step of administering to said patient:

(a) a compound of formula I:

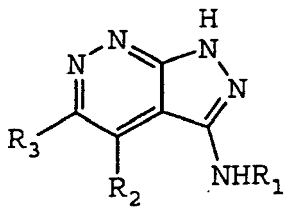

I

or a pharmaceutically acceptable derivative thereof, wherein:

$R_1$ is selected from H, aliphatic, RC(O)-, $RS(O)_n$-, ROC(O)-, carbocyclyl, heterocyclyl, aryl, aralkyl, heteroaryl, or heteroaralkyl; wherein said aliphatic, carbocyclyl, heterocyclyl, aryl, aralkyl, heteroaryl, or heteroaralkyl is optionally substituted;
$R_2$ and $R_3$ are each independently selected from H, aliphatic, carbocyclyl, heterocyclyl, aryl, aralkyl, heteroaryl, heteroaralkyl, $-N(R)_2$, -NRCOR, - $NRCO_2R$, $-NRSO_2R$, $-S(O)_nR$, $-SO_2N(R)_2$, -SR, -OR, $-CF_3$, halo, $-NO_2$, -CN, -C(O)R, $-CO_2R$, -OC(O)R, $-CON(R)_2$, or - $OC(O)N(R)_2$, wherein said aliphatic, carbocyclyl, heterocyclyl, aryl, aralkyl, heteroaryl, or heteroaralkyl is optionally substituted; or wherein $R_2$ and $R_3$ taken together with the intervening atoms optionally form a five- to nine-membered ring that is fused to the pyridazinyl ring of formula I, said fused ring having 0-2 heteroatoms;
R is selected from H, aliphatic, carbocyclyl, heterocyclyl, aryl, aralkyl, heteroaryl, or heteroaralkyl, wherein each member of R except H is optionally substituted; and
n is 1 or 2; or

(b) a pharmaceutical composition comprising said compound and a pharmaceutically acceptable carrier, adjuvant, or vehicle;
in an amount effective to inhibit GSK-3 activity.

17. A method for inhibiting GSK-3 activity in a patient comprising the step of administering to said patient:

(a) a compound according to item 1 or 13, or
(b) a pharmaceutical composition comprising said compound and a pharmaceutically acceptable carrier, adjuvant, or vehicle;
in an amount effective to inhibit GSK-3 activity.

18. A method for enhancing glycogen synthesis or lowering blood levels of glucose in a patient in need thereof, comprising the step of administering to said patient:

(a) a compound of formula I:

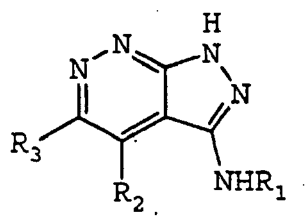

I

or a pharmaceutically acceptable derivative thereof, wherein:

$R_1$ is selected from H, aliphatic, RC(O)-, $RS(O)_n$-, ROC(O)-, carbocyclyl, heterocyclyl, aryl, aralkyl, heteroaryl, or heteroaralkyl; wherein said aliphatic, carbocyclyl, heterocyclyl, aryl, aralkyl, heteroaryl, or heteroaralkyl is optionally substituted;
$R_2$ and $R_3$ are each independently selected from H, aliphatic, carbocyclyl, heterocyclyl, aryl, aralkyl, heteroaryl, heteroaralkyl, $-N(R)_2$, -NRCOR, - $NRCO_2R$, $-NRSO_2R$, $-S(O)_nR$, $-SO_2N(R)_2$, -SR, -OR, $-CF_3$, halo, $-NO_2$, -CN, -C(O)R, $-CO_2R$, -OC(O)R, $-CON(R)_2$, or - $OC(O)N(R)_2$, wherein said aliphatic, carbocyclyl, heterocyclyl, aryl, aralkyl, heteroaryl, or heteroaralkyl is optionally substituted; or wherein $R_2$ and $R_3$ taken together with the intervening atoms optionally form a five- to nine-membered ring that is fused to the pyridazinyl ring of formula I, said fused ring having 0-2 heteroatoms;
R is selected from H, aliphatic, carbocyclyl, heterocyclyl, aryl, aralkyl, heteroaryl, or heteroaralkyl, wherein each member of R except H is optionally substituted; and
n is 1 or 2; or

(b) a pharmaceutical composition comprising said compound and a pharmaceutically acceptable carrier, adjuvant, or vehicle;
in an amount sufficient to enhance glycogen synthesis or lower blood glucose levels.

19. A method for enhancing glycogen synthesis or lowering blood levels of glucose in a patient in need thereof, comprising the step of administering to said patient:

(a) a compound according to item 1 or 13, or
(b) a pharmaceutical composition comprising said compound and a pharmaceutically acceptable carrier, adjuvant, or vehicle;
in an amount sufficient to enhance glycogen synthesis or lower blood glucose levels.

20. The method according to item 18 or 19 wherein the patient is treated for diabetes.

21. A method for inhibiting the production of hyperphosphorylated Tau protein in a patient in need thereof, comprising the step of administering to said patient:

(a) a compound of formula I:

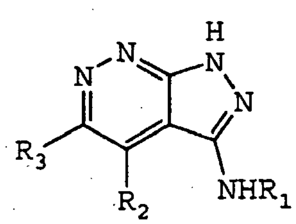

I

or a pharmaceutically acceptable derivative thereof, wherein:

$R_1$ is selected from H, aliphatic, RC(O)-, $RS(O)_n$-, ROC(O)-, carbocyclyl, heterocyclyl, aryl, aralkyl, heteroaryl, or heteroaralkyl; wherein said aliphatic, carbocyclyl, heterocyclyl, aryl, aralkyl, heteroaryl, or heter-

oaralkyl is optionally substituted;

$R_2$ and $R_3$ are each independently selected from H, aliphatic, carbocyclyl, heterocyclyl, aryl, aralkyl, heteroaryl, heteroaralkyl, -N(R)$_2$, -NRCOR, - NRCO$_2$R, -NRSO$_2$R, -S(O)$_n$R, -SO$_2$N(R)$_2$, -SR, -OR, -CF$_3$, halo, -NO$_2$, -CN, -C(O)R, -CO$_2$R, -OC(O)R, -CON(R)$_2$, or - OC(O)N(R)$_2$, wherein said aliphatic, carbocyclyl, heterocyclyl, aryl, aralkyl, heteroaryl, or heteroaralkyl is optionally substituted; or wherein $R_2$ and $R_3$ taken together with the intervening atoms optionally form a five- to nine-membered ring that is fused to the pyridazinyl ring of formula I, said fused ring having 0-2 heteroatoms;

R is selected from H, aliphatic, carbocyclyl, heterocyclyl, aryl, aralkyl, heteroaryl, or heteroaralkyl, wherein each member of R except H is optionally substituted; and

n is 1 or 2; or

(b) a pharmaceutical composition comprising said compound and a pharmaceutically acceptable carrier, adjuvant, or vehicle;

in an amount sufficient to inhibit hyperphosphorylation of Tau protein.

22. A method for inhibiting the production of hyperphosphorylated Tau protein in a patient in need thereof, comprising the step of administering to said patient:

(a) a compound according to item 1 or 13, or

(b) a pharmaceutical composition comprising said compound and a pharmaceutically acceptable carrier, adjuvant, or vehicle;

in an amount sufficient to inhibit hyperphosphorylation of Tau protein.

23. The method according to item 21 or 22, wherein the patient is treated for Alzheimer's disease.

24. A method for inhibiting the phosphorylation of β-catenin in a patient in need thereof, comprising the step of administering to said patient:

(a) a compound of formula I:

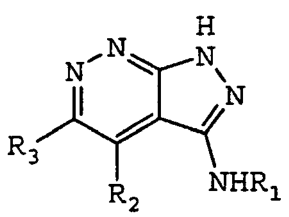

I

or a pharmaceutically acceptable derivative thereof, wherein:

$R_1$ is selected from H, aliphatic, RC(O)-, RS(O)$_n$-, ROC(O)-, carbocyclyl, heterocyclyl, aryl, aralkyl, heteroaryl, or heteroaralkyl; wherein said aliphatic, carbocyclyl, heterocyclyl, aryl, aralkyl., heteroaryl, or heteroaralkyl is optionally substituted;

$R_2$ and $R_3$ are each independently selected from H, aliphatic; carbocyclyl, heterocyclyl, aryl, aralkyl, heteroaryl, heteroaralkyl, -N(R)$_2$, -NRCOR, -NRCO$_2$R, -NRSO$_2$R, -S(O)$_n$R, -SO$_2$N(R)$_2$, -SR, -OR, -CF$_3$, halo, -NO$_2$, -CN, -C(O)R, -CO$_2$R, -OC(O)R, -CON(R)$_2$, or - OC(O)N(R)$_2$, wherein said aliphatic, carbocyclyl, heterocyclyl, aryl, aralkyl, heteroaryl, or heteroaralkyl is optionally substituted; or wherein $R_2$ and $R_3$ taken together with the intervening atoms optionally form a five- to nine-membered ring that is fused to the pyridazinyl ring of formula I, said fused ring having 0-2 heteroatoms;

R is selected from H, aliphatic, carbocyclyl, heterocyclyl, aryl, aralkyl, heteroaryl, or heteroaralkyl, wherein each member of R except H is optionally substituted; and

n is 1 or 2; or

(b) a pharmaceutical composition comprising said compound and a pharmaceutically acceptable carrier, adjuvant, or vehicle;

in an amount sufficient to inhibit phosphorylation of β-catenin.

25. A method for inhibiting the phosphorylation of β-catenin in a patient in need thereof, comprising the step of administering to said patient:

(a) a compound according to item 1 or 13, or
(b) a pharmaceutical composition comprising said compound and a pharmaceutically acceptable carrier, adjuvant, or vehicle,
in an amount sufficient to inhibit phosphorylation of β-catenin.

26. The method according to item 24 or 25, wherein the patient is treated for schizophrenia.

27. The method according to any one of items 16, 18, 21 or 24, wherein said compound or composition is administered with an additional therapeutic agent.

28. A method for inhibiting GSK-3 activity in a biological sample, comprising the step of contacting the biological sample with:

(a) a compound of formula I:

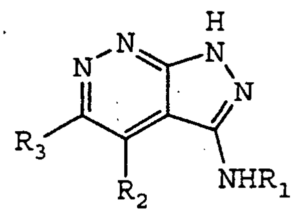

I

or a pharmaceutically acceptable derivative thereof, wherein:

$R_1$ is selected from H, aliphatic, RC(O)-, $RS(O)_n$-, ROC(O)-, carbocyclyl, heterocyclyl, aryl, aralkyl, heteroaryl, or heteroaralkyl; wherein said aliphatic, carbocyclyl, heterocyclyl, aryl, aralkyl, heteroaryl, or heteroaralkyl is optionally substituted;
$R_2$ and $R_3$ are each independently selected from H, aliphatic, carbocyclyl, heterocyclyl, aryl, aralkyl, heteroaryl, heteroaralkyl, $-N(R)_2$, -NRCOR, - $NRCO_2R$, $-NRSO_2R$, $-S(O)_nR$, $-SO_2N(R)_2$, -SR, -OR, $-CF_3$, halo, $-NO_2$, -CN, -C(O)R, $-CO_2R$, -OC(O)R, $-CON(R)_2$, or - $OC(O)N(R)_2$, wherein said aliphatic, carbocyclyl, heterocyclyl, aryl, aralkyl, heteroaryl, or heteroaralkyl is optionally substituted; or wherein $R_2$ and $R_3$ taken together with the intervening atoms optionally form a five- to nine-membered ring that is fused to the pyridazinyl ring of formula I, said fused ring having 0-2 heteroatoms;
R is selected from H, aliphatic, carbocyclyl, heterocyclyl, aryl, aralkyl, heteroaryl, or heteroaralkyl, wherein each member of R except H is optionally substituted; and
n is 1 or 2; or

(b) a composition comprising said compound and a carrier, adjuvant, or vehicle;
in an amount effective to inhibit GSK-3 activity.

29. A method for inhibiting GSK-3 activity in a biological sample, comprising the step of contacting the biological sample with:

(a) a compound according to item 1 or 13, or
(b) a composition comprising said compound and a carrier, adjuvant, or vehicle;
in an amount effective to inhibit GSK-3 activity.

30. A crystal comprising an unphosphorylated GSK-3β protein or unphosphorylated GSK-3β homologue; and phosphate ions.

31. A crystal comprising an unphosphorylated GSK-3β protein or unphosphorylated GSK-3β homologue; and a chemical entity.

32. The crystal of item 31, wherein the chemical entity binds to the active site.

33. The crystal of item 32, wherein the chemical entity is selected from the group consisting of 4,5-Diphenyl-1H-pyrazolo[3,4-c]pyridazin-3-ylamine, (5-Methyl-2H-pyrazol-3-yl)-(2-pyridin-4-yl-quinazolin-4-yl)-amine, 4-(5-Methyl-2-phenylamino-pyrimidin-4-yl)-1H-pyrrole-2-carboxylic acid (2-hydroxy-1-phenyl-ethyl)-amide, (1H-Indazol-3-yl)-[2-(2-trifluoromethyl-phenyl)-quinazolin-4-yl]-amine, ATP, an ATP analogue and a nucleotide triphosphate.

34. A crystal comprising a GSK-3β protein or GSK-3β homologue and a chemical entity that binds to the substrate binding groove.

35. The crystal of item 34, wherein the chemical entity is HSSPHQpSEDEEE.

36. The crystal of any one of items 30, 31 and 34, wherein said GSK-3β protein is selected from the group consisting of SEQ ID NO: 1; amino acid residues 7-420 of SEQ ID NO: 1; and amino acid residues 37-381 of SEQ ID NO: 1.

37. A method of obtaining a crystal of a GSK-3β protein complex or GSK-3β homologue protein complex, wherein said protein complex comprises a chemical entity that binds to the substrate-binding groove, comprising the steps of:

a) producing and purifying GSK-3β protein;
b) mixing a crystallization solution with the protein complex to produce a crystallizable composition; and
c) subjecting the composition to conditions which promote crystallization.

38. The method of item 37, wherein the GSK-3β protein is produced from a baculovirus overexpression system.

39. A molecule or molecular complex comprising a binding pocket defined by structure coordinates of amino acid residues which are identical to GSK-3β amino acid residues K85, M101, V110, L130 and L132 according to any one of Figures 1-7, wherein the root mean square deviation of the backbone atoms between said amino acid residues and said GSK-3β amino acid residues is not greater than about 3.0 Å,

40. A molecule or molecular complex comprising a binding pocket defined by structure coordinates of amino acid residues which are identical to GSK-3β amino acid residues I62, V135, P136, T138 and L188 according to any one of Figures 1-7, wherein the root mean square deviation of the backbone atoms between said amino acid residues and said GSK-3β amino acid residues is not greater than about 3.0 Å.

41. A molecule or molecular complex comprising a binding pocket defined by structure coordinates of amino acid residues which are identical to GSK-3β amino acid residues V70, V110, L188 and C199 according to any one of Figures 1-7, wherein the root mean square deviation of the backbone atoms between said amino acid residues and said GSK-3β amino acid residues is not greater than about 3.0 Å.

42. A molecule or molecular complex comprising a binding pocket defined by structure coordinates of amino acid residues which are identical to GSK-3β amino acid residues V70, F67, Q185 and C199 according to any one of Figures 1-7, wherein the root mean square deviation of the backbone atoms between said amino acid residues and said GSK-3β amino acid residues is not greater than about 3.0 Å.

43. A molecule or molecular complex comprising part of a binding pocket, said binding pocket defined by structure coordinates of amino acid residues which are identical to GSK-3β amino acid residues Y56, T59, K60, V61, I62, G63, N64, G65, S66, F67, G68, V69, V70, Y71, Q72, A73, K74, L75, L81, V82, A83, I84, K85, K86, V87, L88, E97, L98, M101, R102, L104, H106, C107, N108, I109, V110, R111, L112, R113, Y114, F115, F116, L128, N129, L130, V131, L132, D133, Y134, V135, P136, E137, T138, V139, Y140, R141, V142, R144, P154, V155, I156, Y157, V158, K159, L160, Y161, M162, Y163, Q164, L165, F166, R167, S168, L169, A170, Y171, I172, H173, S174, F175, G176, I177, C178, H179, R180, D181, I182, K183, P184, Q185, N186, L187, L188, L189, D190, P191, A194, V195, L196, K197, L198, C199, D200, F201, G202, S203 and S219 according to any one of Figures 1-7, wherein the root mean square deviation of the backbone atoms between said amino acid residues and said GSK-3β amino acid residues is not greater than about 0.2 Å.

44. A molecule or molecular complex comprising a binding pocket defined by structure coordinates of amino acid residues which are identical to GSK-3β amino acid residues G65, S66, F67 and F93 according to any one of Figures 1-7, wherein the root mean square deviation of the backbone atoms between said amino acid residues and said

GSK-3β amino acid residues is not greater than about 3.0 Å.

45. A molecule or molecular complex comprising a binding pocket defined by structure coordinates of amino acid residues which are identical to GSK-3Å amino acid residues R96, R180, K205, N213 and Y234 according to any one of Figures 1-7, wherein the root mean square deviation of the backbone atoms between said amino acid residues and said GSK-3Å amino acid residues is not greater than about 3.0 Å.

46. A molecule or molecular complex comprising a binding pocket defined by structure coordinates of amino acid residues which are identical to GSK-3β amino acid residues Y216, I217, C218, S219, R220 and R223 according to any one of Figures 1-7, wherein the root mean square deviation of the backbone atoms between said amino acid residues and said GSK-3β amino acid residues is not greater than about 3.0 Å.

47. A molecule or molecular complex comprising part of a binding pocket, said binding pocket defined by structure coordinates of amino acid residues which are identical to GSK-3β amino acid residues N64, G65, S66, F67, G68, V87, L88, D90, K91, R92, F93, K94, N95, R96, E97, R180, D181, I182, K183 Q185, N186, D200, F201, G202, S203, A204, K205, Q206, L207, E211, P212, N213, V214, S215, Y216, I217, C218, S219, R220, Y221, Y222, R223, L227, T232 and Y234 according to any one of Figures 1-7, wherein the root mean square deviation of the backbone atoms between said amino acid residues and said GSK-3β amino acid residues is not greater than about 0.2 Å.

48. A molecule or molecular complex comprising a GSK-3β protein defined by structure coordinates of amino acid residues which are identical to GSK-3β amino acid residues according to Figure 4, wherein the root mean square deviation of the backbone atoms between said amino acid residues and said GSK-3β amino acid residues is not greater than about 2.0 Å.

49. A molecule or molecular complex comprising a protein kinase comprising a glutamine or glutamic acid residue that corresponds to Gln185 of GSK-3β protein, wherein the $\chi 1$ angle is in the range of 123˚ to 180˚, and the $\chi 2$ angle is in the range of -174˚ to -180˚ and 106˚ to 180˚.

50. A molecule or molecular complex comprising a protein kinase comprising a glutamine or glutamic acid residue that corresponds to Gln185 of GSK-3β protein, wherein the $\chi 1$ angle is in the range of -100˚ to -180˚ and the $\chi 2$ angle is in the range of -151˚ to -180˚ and 126˚ to 180˚.

51. The molecule or molecular complex of any one of items 39-42, 44-46 and 49-50, wherein the molecule is a GSK-3β protein or a GSK-3β homologue.

52. The molecule or molecular complex of any one of items 39-42, 44-46 and 49-50, wherein the molecule or molecular complex is in crystalline form.

53. A computer comprising:

a) a machine-readable data storage medium comprising a data storage material encoded with machine-readable data, wherein said data defines the binding pocket according to any one of items 39-42 and 44-46;
b) a working memory for storing instructions for processing said machine-readable data;
c) a central processing unit coupled to said working memory and to said machine-readable data storage medium for processing said machine readable data; and
d) output hardware coupled to said central processing unit for outputting information of said binding pocket or information produced by using said binding pocket.

54. The computer according to item 53, wherein said output hardware is a display terminal, a printer or disk drive.

55. A computer for determining at least a portion of the structure coordinates corresponding to X-ray diffraction data obtained from a molecule or molecular complex, wherein said computer comprises:

a) a machine-readable data storage medium comprising a data storage material encoded with machine-readable data, wherein said data comprises at least a portion of the GSK-3β structure coordinates of according to any one of Figures 1-7;
b) a machine-readable data storage medium comprising a data storage material encoded with machine-readable

data, wherein said data comprises X-ray diffraction data obtained from said molecule or molecular complex; and
c) instructions for performing a Fourier transform of the machine readable data of (a) and for processing said machine readable data of (b) into structure coordinates.

56. The computer of item 55, wherein the molecule is a GSK-3β homologue.

57. The computer of item 55, wherein the molecular complex is selected from the group consisting of a GSK-3β protein complex and a GSK-3β homologue complex.

58. A method for evaluating the ability of a chemical entity to associate with the molecule or molecular complex according to any one of items 39-42 and 44-46 comprising the steps of:

a) employing computational means to perform a fitting operation between the chemical entity and the molecule or molecular complex; and
b) analyzing the results of said fitting operation to quantify the association between the chemical entity and the molecule or molecular complex.

59. The method of item 58, further comprising generating a three-dimensional graphical representation of the molecule or molecular complex prior to step (a).

60. The method of item 58, wherein the method is for evaluating the ability of a chemical entity to associate with the binding pocket of the molecule or molecular complex.

61. A method for identifying an agonist or antagonist of a molecule or molecular complex according to any one of items 39-42 and 44-46 comprising the steps of:

a) using the three-dimensional structure of said molecule or molecular complex to design or select a chemical entity;
b) contacting said chemical entity with the molecule or molecular complex and monitoring the activity of the molecule or molecular complex; and
c) classifying said chemical entity as an agonist or antagonist based on the effect of said chemical entity on the activity of the molecule or molecular complex.

62. The method of item 61, wherein step a) is using the three-dimensional structure of the binding pocket of the molecule or molecular complex.

63. The method of item ’61, wherein the three-dimensional structure is displayed as a graphical representation.

64. A method of utilizing molecular replacement to obtain structural information about a molecule or a molecular complex of unknown structure, comprising the steps of:

a) crystallizing said molecule or molecular complex;
b) generating an X-ray diffraction pattern from said crystallized molecule or molecular complex; and
c) applying at least a portion of the GSK-3β structure coordinates set forth in any one of Figures 1-7 to said X-ray diffraction pattern to generate a three-dimensional electron density map of at least a portion of the molecule or molecular complex whose structure is unknown.

65. The method of item 64, wherein the molecule is a GSK-3β homologue.

66. The method of item 64, wherein the molecular complex is selected from the group consisting of a GSK-3β protein complex and a GSK-3β homologue complex.

## Claims

1. A crystal comprising an unphosphorylated GSK-3β protein or unphosphorylated GSK-3β homologue; and phosphate ions.

2. A crystal comprising an unphosphorylated GSK-3β protein or unphosphorylated GSK-3β homologue; and a chemical entity.

3. The crystal of claim 2, wherein the chemical entity binds to the active site.

4. The crystal of claim 3, wherein the chemical entity is selected from the group consisting of 4,5-Diphenyl-1H-pyrazolo [3,4-c]pyridazin-3-ylamine, (5-Methyl-2H-pyrazol-3-yl)-(2-pyridin-4-yl-quinazolin-4-yl)-amine, 4-(5-Methyl-2-phenylamino-pyrimidin-4-yl)-1H-pyrrole-2-carboxylic acid (2-hydroxy-1-phenyl-ethyl)-amide, (1H-Indazol-3-yl)-[2-(2-trifluoromethyl-phenyl)-quinazolin-4-yl]-amine, ATP, an ATP analogue and a nucleotide triphosphate.

5. A crystal comprising a GSK-3β protein or GSK-3β homologue and a chemical entity that binds to the substrate binding groove.

6. The crystal of claim 5, wherein the chemical entity is HSSPHQpSEDEEE.

7. The crystal of any one of claims 1, 2 and 5, wherein said GSK-3β protein is selected from the group consisting of SEQ ID NO: 1; amino acid residues 7-420 of SEQ ID NO: 1; and amino acid residues 37-381 of SEQ ID NO: 1.

8. A method of obtaining a crystal of a GSK-3β protein complex or GSK-3β homologue protein complex, wherein said protein complex comprises a chemical entity that binds to the substrate-binding groove, comprising the steps of:

   (a) producing and purifying GSK-3β protein;
   (b) mixing a crystallization solution with the protein complex to produce a crystallizable composition; and
   (c) subjecting the composition to conditions which promote crystallization.

9. The method of claim 8, wherein the GSK-3β protein is produced from a baculovirus overexpression system.

# FIG. 1

| | | Atom Type | Resid | | # | X | Y | Z | OCC | B | |
|---|---|---|---|---|---|---|---|---|---|---|---|
| ATOM | 1 | N | SER | A | 25 | 49.761 | 12.124 | 57.404 | 1.00 | 73.06 | N |
| ATOM | 2 | CA | SER | A | 25 | 49.843 | 13.283 | 58.345 | 1.00 | 73.06 | C |
| ATOM | 3 | C | SER | A | 25 | 50.651 | 14.389 | 57.666 | 1.00 | 73.06 | C |
| ATOM | 4 | O | SER | A | 25 | 50.845 | 14.354 | 56.450 | 0.00 | 73.06 | O |
| ATOM | 5 | CB | SER | A | 25 | 50.535 | 12.861 | 59.654 | 1.00 | 74.21 | C |
| ATOM | 6 | OG | SER | A | 25 | 51.881 | 12.446 | 59.431 | 1.00 | 74.21 | O |
| ATOM | 7 | N | MET | A | 26 | 51.124 | 15.366 | 58.439 | 1.00 | 60.23 | N |
| ATOM | 8 | CA | MET | A | 26 | 51.921 | 16.424 | 57.824 | 1.00 | 60.23 | C |
| ATOM | 9 | C | MET | A | 26 | 53.407 | 16.337 | 58.174 | 1.00 | 60.23 | C |
| ATOM | 10 | O | MET | A | 26 | 53.781 | 16.042 | 59.315 | 1.00 | 60.23 | O |
| ATOM | 11 | CB | MET | A | 26 | 51.352 | 17.824 | 58.147 | 1.00 | 70.25 | C |
| ATOM | 12 | CG | MET | A | 26 | 51.419 | 18.290 | 59.592 | 1.00 | 70.25 | C |
| ATOM | 13 | SD | MET | A | 26 | 50.344 | 19.765 | 59.811 | 1.00 | 70.25 | S |
| ATOM | 14 | CE | MET | A | 26 | 48.782 | 18.955 | 60.093 | 1.00 | 70.25 | C |
| ATOM | 15 | N | LYS | A | 27 | 54.235 | 16.565 | 57.153 | 1.00 | 56.51 | N |
| ATOM | 16 | CA | LYS | A | 27 | 55.692 | 16.533 | 57.262 | 1.00 | 56.51 | C |
| ATOM | 17 | C | LYS | A | 27 | 56.201 | 17.936 | 56.941 | 1.00 | 56.51 | C |
| ATOM | 18 | O | LYS | A | 27 | 55.820 | 18.521 | 55.923 | 1.00 | 56.51 | O |
| ATOM | 19 | CB | LYS | A | 27 | 56.269 | 15.513 | 56.273 | 1.00 | 46.92 | C |
| ATOM | 20 | CG | LYS | A | 27 | 55.663 | 14.129 | 56.427 | 1.00 | 46.92 | C |
| ATOM | 21 | CD | LYS | A | 27 | 56.373 | 13.074 | 55.597 | 0.00 | 46.92 | C |
| ATOM | 22 | CE | LYS | A | 27 | 57.742 | 12.749 | 56.167 | 0.00 | 46.92 | C |
| ATOM | 23 | NZ | LYS | A | 27 | 58.347 | 11.567 | 55.494 | 1.00 | 46.92 | N |
| ATOM | 24 | N | VAL | A | 28 | 57.071 | 18.464 | 57.802 | 1.00 | 77.30 | N |
| ATOM | 25 | CA | VAL | A | 28 | 57.594 | 19.824 | 57.645 | 1.00 | 77.30 | C |
| ATOM | 26 | C | VAL | A | 28 | 59.109 | 19.917 | 57.462 | 1.00 | 77.30 | C |
| ATOM | 27 | O | VAL | A | 28 | 59.849 | 19.965 | 58.445 | 1.00 | 77.30 | O |
| ATOM | 28 | CB | VAL | A | 28 | 57.187 | 20.691 | 58.879 | 1.00 | 74.69 | C |
| ATOM | 29 | CG1 | VAL | A | 28 | 57.571 | 19.979 | 60.170 | 0.00 | 74.69 | C |
| ATOM | 30 | CG2 | VAL | A | 28 | 57.853 | 22.057 | 58.812 | 1.00 | 74.69 | C |
| ATOM | 31 | N | SER | A | 29 | 59.562 | 19.953 | 56.206 | 1.00 | 60.45 | N |
| ATOM | 32 | CA | SER | A | 29 | 60.987 | 20.058 | 55.915 | 1.00 | 60.45 | C |
| ATOM | 33 | C | SER | A | 29 | 61.401 | 21.504 | 55.656 | 1.00 | 60.45 | C |
| ATOM | 34 | O | SER | A | 29 | 60.549 | 22.390 | 55.583 | 1.00 | 60.45 | O |
| ATOM | 35 | N | ARG | A | 30 | 62.705 | 21.753 | 55.522 | 1.00 | 100.00 | N |
| ATOM | 36 | CA | ARG | A | 30 | 63.210 | 23.109 | 55.275 | 1.00 | 100.00 | C |
| ATOM | 37 | C | ARG | A | 30 | 64.306 | 23.124 | 54.204 | 1.00 | 100.00 | C |
| ATOM | 38 | O | ARG | A | 30 | 65.501 | 23.089 | 54.517 | 1.00 | 100.00 | O |
| ATOM | 39 | CB | ARG | A | 30 | 63.741 | 23.734 | 56.582 | 1.00 | 93.57 | C |
| ATOM | 40 | CG | ARG | A | 30 | 62.723 | 23.680 | 57.736 | 1.00 | 93.57 | C |
| ATOM | 41 | CD | ARG | A | 30 | 62.760 | 24.920 | 58.641 | 1.00 | 93.57 | C |
| ATOM | 42 | NE | ARG | A | 30 | 63.946 | 24.972 | 59.497 | 1.00 | 93.57 | N |
| ATOM | 43 | CZ | ARG | A | 30 | 64.233 | 24.092 | 60.456 | 1.00 | 93.57 | C |
| ATOM | 44 | NH1 | ARG | A | 30 | 63.420 | 23.072 | 60.697 | 0.00 | 93.57 | N |
| ATOM | 45 | NH2 | ARG | A | 30 | 65.344 | 24.241 | 61.179 | 1.00 | 93.57 | N |
| ATOM | 46 | N | ASP | A | 31 | 63.889 | 23.163 | 52.941 | 1.00 | 64.77 | N |
| ATOM | 47 | CA | ASP | A | 31 | 64.842 | 23.183 | 51.853 | 0.00 | 64.77 | C |
| ATOM | 48 | C | ASP | A | 31 | 64.524 | 24.323 | 50.914 | 1.00 | 64.77 | C |
| ATOM | 49 | O | ASP | A | 31 | 63.517 | 25.024 | 51.097 | 1.00 | 64.77 | O |
| ATOM | 50 | N | LYS | A | 32 | 65.380 | 24.508 | 49.907 | 1.00 | 94.72 | N |
| ATOM | 51 | CA | LYS | A | 32 | 65.171 | 25.571 | 48.937 | 1.00 | 94.72 | C |
| ATOM | 52 | C | LYS | A | 32 | 65.379 | 26.960 | 49.533 | 1.00 | 94.72 | C |
| ATOM | 53 | O | LYS | A | 32 | 64.425 | 27.754 | 49.653 | 1.00 | 94.72 | O |
| ATOM | 54 | N | ASP | A | 33 | 66.627 | 27.251 | 49.914 | 1.00 | 100.00 | N |
| ATOM | 55 | CA | ASP | A | 33 | 66.961 | 28.548 | 50.491 | 1.00 | 100.00 | C |
| ATOM | 56 | C | ASP | A | 33 | 66.187 | 28.957 | 51.741 | 1.00 | 100.00 | C |
| ATOM | 57 | O | ASP | A | 33 | 65.140 | 29.613 | 51.650 | 1.00 | 100.00 | O |
| ATOM | 58 | N | GLY | A | 34 | 66.705 | 28.575 | 52.907 | 1.00 | 55.02 | N |

| | | | | | | | | | | | |
|---|---|---|---|---|---|---|---|---|---|---|---|
| ATOM | 59 | CA | GLY | A | 34 | 66.079 | 28.920 | 54.172 | 0.00 | 55.02 | C |
| ATOM | 60 | C | GLY | A | 34 | 64.595 | 29.238 | 54.137 | 0.00 | 55.02 | C |
| ATOM | 61 | O | GLY | A | 34 | 64.195 | 30.402 | 54.124 | 0.00 | 55.02 | O |
| ATOM | 62 | N | SER | A | 35 | 63.780 | 28.191 | 54.122 | 1.00 | 67.60 | N |
| ATOM | 63 | CA | SER | A | 35 | 62.336 | 28.345 | 54.107 | 1.00 | 67.60 | C |
| ATOM | 64 | C | SER | A | 35 | 61.644 | 27.095 | 54.652 | 1.00 | 67.60 | C |
| ATOM | 65 | O | SER | A | 35 | 61.932 | 25.974 | 54.207 | 1.00 | 67.60 | O |
| ATOM | 66 | N | LYS | A | 36 | 60.739 | 27.272 | 55.616 | 1.00 | 65.29 | N |
| ATOM | 67 | CA | LYS | A | 36 | 60.010 | 26.138 | 56.199 | 1.00 | 65.29 | C |
| ATOM | 68 | C | LYS | A | 36 | 58.952 | 25.617 | 55.247 | 1.00 | 65.29 | C |
| ATOM | 69 | O | LYS | A | 36 | 58.126 | 26.383 | 54.745 | 1.00 | 65.29 | O |
| ATOM | 70 | CB | LYS | A | 36 | 59.320 | 26.545 | 57.496 | 1.00 | 48.96 | C |
| ATOM | 71 | CG | LYS | A | 36 | 58.431 | 25.464 | 58.105 | 1.00 | 48.96 | C |
| ATOM | 72 | CD | LYS | A | 36 | 57.820 | 25.955 | 59.427 | 1.00 | 48.96 | C |
| ATOM | 73 | CE | LYS | A | 36 | 57.028 | 24.873 | 60.134 | 1.00 | 48.96 | C |
| ATOM | 74 | NZ | LYS | A | 36 | 56.438 | 25.361 | 61.412 | 0.00 | 48.96 | N |
| ATOM | 75 | N | VAL | A | 37 | 58.968 | 24.309 | 55.010 | 1.00 | 60.62 | N |
| ATOM | 76 | CA | VAL | A | 37 | 57.992 | 23.691 | 54.119 | 1.00 | 60.62 | C |
| ATOM | 77 | C | VAL | A | 37 | 57.112 | 22.687 | 54.830 | 1.00 | 60.62 | C |
| ATOM | 78 | O | VAL | A | 37 | 57.591 | 21.779 | 55.508 | 0.00 | 60.62 | O |
| ATOM | 79 | CB | VAL | A | 37 | 58.668 | 22.974 | 52.944 | 1.00 | 46.81 | C |
| ATOM | 80 | CG1 | VAL | A | 37 | 57.627 | 22.175 | 52.153 | 1.00 | 46.81 | C |
| ATOM | 81 | CG2 | VAL | A | 37 | 59.352 | 23.994 | 52.044 | 1.00 | 46.81 | C |
| ATOM | 82 | N | THR | A | 38 | 55.809 | 22.869 | 54.661 | 1.00 | 44.94 | N |
| ATOM | 83 | CA | THR | A | 38 | 54.836 | 21.972 | 55.254 | 1.00 | 44.94 | C |
| ATOM | 84 | C | THR | A | 38 | 54.233 | 21.142 | 54.129 | 1.00 | 44.94 | C |
| ATOM | 85 | O | THR | A | 38 | 53.713 | 21.677 | 53.138 | 1.00 | 44.94 | O |
| ATOM | 86 | CB | THR | A | 38 | 53.712 | 22.740 | 55.987 | 1.00 | 45.12 | C |
| ATOM | 87 | OG1 | THR | A | 38 | 54.290 | 23.633 | 56.952 | 1.00 | 45.12 | O |
| ATOM | 88 | CG2 | THR | A | 38 | 52.783 | 21.752 | 56.701 | 1.00 | 45.12 | C |
| ATOM | 89 | N | THR | A | 39 | 54.343 | 19.827 | 54.278 | 1.00 | 39.35 | N |
| ATOM | 90 | CA | THR | A | 39 | 53.818 | 18.901 | 53.300 | 1.00 | 39.35 | C |
| ATOM | 91 | C | THR | A | 39 | 52.789 | 18.053 | 54.003 | 1.00 | 39.35 | C |
| ATOM | 92 | O | THR | A | 39 | 52.973 | 17.646 | 55.149 | 1.00 | 39.35 | O |
| ATOM | 93 | CB | THR | A | 39 | 54.914 | 17.998 | 52.738 | 1.00 | 43.54 | C |
| ATOM | 94 | OG1 | THR | A | 39 | 55.894 | 18.803 | 52.063 | 1.00 | 43.54 | O |
| ATOM | 95 | CG2 | THR | A | 39 | 54.315 | 16.996 | 51.755 | 1.00 | 43.54 | C |
| ATOM | 96 | N | VAL | A | 40 | 51.692 | 17.795 | 53.316 | 1.00 | 39.28 | N |
| ATOM | 97 | CA | VAL | A | 40 | 50.620 | 17.013 | 53.895 | 1.00 | 39.28 | C |
| ATOM | 98 | C | VAL | A | 40 | 49.848 | 16.368 | 52.766 | 1.00 | 39.28 | C |
| ATOM | 99 | O | VAL | A | 40 | 49.945 | 16.782 | 51.609 | 1.00 | 39.28 | O |
| ATOM | 100 | CB | VAL | A | 40 | 49.647 | 17.914 | 54.710 | 1.00 | 35.69 | C |
| ATOM | 101 | CG1 | VAL | A | 40 | 48.947 | 18.905 | 53.785 | 1.00 | 35.69 | C |
| ATOM | 102 | CG2 | VAL | A | 40 | 48.622 | 17.055 | 55.432 | 0.00 | 35.69 | C |
| ATOM | 103 | N | VAL | A | 41 | 49.077 | 15.351 | 53.106 | 1.00 | 37.75 | N |
| ATOM | 104 | CA | VAL | A | 41 | 48.280 | 14.668 | 52.115 | 1.00 | 37.75 | C |
| ATOM | 105 | C | VAL | A | 41 | 46.846 | 15.066 | 52.375 | 1.00 | 37.75 | C |
| ATOM | 106 | O | VAL | A | 41 | 46.294 | 14.711 | 53.407 | 1.00 | 37.75 | O |
| ATOM | 107 | CB | VAL | A | 41 | 48.429 | 13.149 | 52.257 | 1.00 | 35.15 | C |
| ATOM | 108 | CG1 | VAL | A | 41 | 47.543 | 12.434 | 51.244 | 1.00 | 35.15 | C |
| ATOM | 109 | CG2 | VAL | A | 41 | 49.894 | 12.767 | 52.075 | 1.00 | 35.15 | C |
| ATOM | 110 | N | ALA | A | 42 | 46.246 | 15.812 | 51.453 | 1.00 | 35.42 | N |
| ATOM | 111 | CA | ALA | A | 42 | 44.862 | 16.244 | 51.634 | 1.00 | 35.42 | C |
| ATOM | 112 | C | ALA | A | 42 | 43.924 | 15.684 | 50.578 | 1.00 | 35.42 | C |
| ATOM | 113 | O | ALA | A | 42 | 44.341 | 14.941 | 49.689 | 1.00 | 35.42 | O |
| ATOM | 114 | CB | ALA | A | 42 | 44.782 | 17.757 | 51.634 | 1.00 | 58.29 | C |
| ATOM | 115 | N | THR | A | 43 | 42.655 | 16.070 | 50.689 | 1.00 | 42.71 | N |
| ATOM | 116 | CA | THR | A | 43 | 41.590 | 15.635 | 49.786 | 1.00 | 42.71 | C |
| ATOM | 117 | C | THR | A | 43 | 41.051 | 16.801 | 48.950 | 1.00 | 42.71 | C |
| ATOM | 118 | O | THR | A | 43 | 40.747 | 17.869 | 49.491 | 1.00 | 42.71 | O |
| ATOM | 119 | CB | THR | A | 43 | 40.392 | 15.060 | 50.589 | 1.00 | 41.19 | C |
| ATOM | 120 | OG1 | THR | A | 43 | 40.870 | 14.157 | 51.590 | 1.00 | 41.19 | O |

| | | | | | | | | | | | |
|---|---|---|---|---|---|---|---|---|---|---|---|
| ATOM | 121 | CG2 | THR | A | 43 | 39.423 | 14.322 | 49.672 | 1.00 | 41.19 | C |
| ATOM | 122 | N | PRO | A | 44 | 40.899 | 16.603 | 47.628 | 1.00 | 40.41 | N |
| ATOM | 123 | CA | PRO | A | 44 | 40.385 | 17.640 | 46.729 | 1.00 | 40.41 | C |
| ATOM | 124 | C | PRO | A | 44 | 39.113 | 18.213 | 47.319 | 1.00 | 40.41 | C |
| ATOM | 125 | O | PRO | A | 44 | 38.334 | 17.477 | 47.925 | 1.00 | 40.41 | O |
| ATOM | 126 | CB | PRO | A | 44 | 40.090 | 16.872 | 45.445 | 1.00 | 39.12 | C |
| ATOM | 127 | CG | PRO | A | 44 | 41.122 | 15.825 | 45.437 | 1.00 | 39.12 | C |
| ATOM | 128 | CD | PRO | A | 44 | 41.125 | 15.351 | 46.888 | 1.00 | 39.12 | C |
| ATOM | 129 | N | GLY | A | 45 | 38.897 | 19.515 | 47.157 | 1.00 | 31.93 | N |
| ATOM | 130 | CA | GLY | A | 45 | 37.687 | 20.113 | 47.687 | 1.00 | 31.93 | C |
| ATOM | 131 | C | GLY | A | 45 | 36.476 | 19.506 | 47.011 | 1.00 | 31.93 | C |
| ATOM | 132 | O | GLY | A | 45 | 35.509 | 19.121 | 47.660 | 1.00 | 31.93 | O |
| ATOM | 133 | N | GLN | A | 46 | 36.541 | 19.422 | 45.689 | 1.00 | 46.73 | N |
| ATOM | 134 | CA | GLN | A | 46 | 35.468 | 18.853 | 44.885 | 1.00 | 46.73 | C |
| ATOM | 135 | C | GLN | A | 46 | 36.067 | 17.703 | 44.082 | 1.00 | 46.73 | C |
| ATOM | 136 | O | GLN | A | 46 | 37.292 | 17.621 | 43.919 | 1.00 | 46.73 | O |
| ATOM | 137 | CB | GLN | A | 46 | 34.901 | 19.910 | 43.950 | 1.00 | 47.06 | C |
| ATOM | 138 | N | GLY | A | 47 | 35.216 | 16.813 | 43.579 | 1.00 | 65.73 | N |
| ATOM | 139 | CA | GLY | A | 47 | 35.722 | 15.684 | 42.809 | 1.00 | 65.73 | C |
| ATOM | 140 | C | GLY | A | 47 | 35.779 | 14.418 | 43.650 | 1.00 | 65.73 | C |
| ATOM | 141 | O | GLY | A | 47 | 35.366 | 14.430 | 44.817 | 1.00 | 65.73 | O |
| ATOM | 142 | N | PRO | A | 48 | 36.288 | 13.305 | 43.095 | 1.00 | 49.54 | N |
| ATOM | 143 | CA | PRO | A | 48 | 36.386 | 12.029 | 43.820 | 1.00 | 49.54 | C |
| ATOM | 144 | C | PRO | A | 48 | 37.581 | 12.049 | 44.781 | 1.00 | 49.54 | C |
| ATOM | 145 | O | PRO | A | 48 | 38.625 | 12.627 | 44.467 | 1.00 | 49.54 | O |
| ATOM | 146 | CB | PRO | A | 48 | 36.578 | 11.002 | 42.698 | 1.00 | 58.10 | C |
| ATOM | 147 | CG | PRO | A | 48 | 36.272 | 11.782 | 41.404 | 1.00 | 58.10 | C |
| ATOM | 148 | CD | PRO | A | 48 | 36.772 | 13.149 | 41.717 | 1.00 | 58.10 | C |
| ATOM | 149 | N | ASP | A | 49 | 37.428 | 11.419 | 45.940 | 1.00 | 61.37 | N |
| ATOM | 150 | CA | ASP | A | 49 | 38.497 | 11.388 | 46.936 | 1.00 | 61.37 | C |
| ATOM | 151 | C | ASP | A | 49 | 39.746 | 10.738 | 46.347 | 1.00 | 61.37 | C |
| ATOM | 152 | O | ASP | A | 49 | 39.815 | 9.510 | 46.243 | 1.00 | 61.37 | O |
| ATOM | 153 | CB | ASP | A | 49 | 38.063 | 10.591 | 48.179 | 1.00 | 57.83 | C |
| ATOM | 154 | CG | ASP | A | 49 | 38.999 | 10.804 | 49.371 | 1.00 | 57.83 | C |
| ATOM | 155 | OD1 | ASP | A | 49 | 40.235 | 10.962 | 49.172 | 1.00 | 57.83 | O |
| ATOM | 156 | OD2 | ASP | A | 49 | 38.486 | 10.803 | 50.513 | 1.00 | 57.83 | O |
| ATOM | 157 | N | ARG | A | 50 | 40.721 | 11.563 | 45.970 | 1.00 | 44.76 | N |
| ATOM | 158 | CA | ARG | A | 50 | 41.976 | 11.091 | 45.394 | 1.00 | 44.76 | C |
| ATOM | 159 | C | ARG | A | 50 | 43.095 | 11.965 | 45.954 | 1.00 | 44.76 | C |
| ATOM | 160 | O | ARG | A | 50 | 43.581 | 12.890 | 45.291 | 1.00 | 44.76 | O |
| ATOM | 161 | CB | ARG | A | 50 | 41.927 | 11.184 | 43.868 | 1.00 | 49.13 | C |
| ATOM | 162 | CG | ARG | A | 50 | 40.824 | 10.341 | 43.264 | 1.00 | 49.13 | C |
| ATOM | 163 | CD | ARG | A | 50 | 41.134 | 8.852 | 43.346 | 1.00 | 49.13 | C |
| ATOM | 164 | NE | ARG | A | 50 | 42.017 | 8.452 | 42.253 | 1.00 | 49.13 | N |
| ATOM | 165 | CZ | ARG | A | 50 | 42.534 | 7.235 | 42.102 | 1.00 | 49.13 | C |
| ATOM | 166 | NH1 | ARG | A | 50 | 42.261 | 6.278 | 42.978 | 0.00 | 49.13 | N |
| ATOM | 167 | NH2 | ARG | A | 50 | 43.318 | 6.976 | 41.063 | 1.00 | 49.13 | N |
| ATOM | 168 | N | PRO | A | 51 | 43.530 | 11.655 | 47.186 | 1.00 | 32.41 | N |
| ATOM | 169 | CA | PRO | A | 51 | 44.577 | 12.346 | 47.939 | 1.00 | 32.41 | C |
| ATOM | 170 | C | PRO | A | 51 | 45.713 | 12.895 | 47.100 | 1.00 | 32.41 | C |
| ATOM | 171 | O | PRO | A | 51 | 45.946 | 12.434 | 45.990 | 1.00 | 32.41 | O |
| ATOM | 172 | CB | PRO | A | 51 | 45.040 | 11.281 | 48.933 | 1.00 | 34.03 | C |
| ATOM | 173 | CG | PRO | A | 51 | 44.677 | 9.986 | 48.262 | 1.00 | 34.03 | C |
| ATOM | 174 | CD | PRO | A | 51 | 43.326 | 10.302 | 47.732 | 1.00 | 34.03 | C |
| ATOM | 175 | N | GLN | A | 52 | 46.401 | 13.904 | 47.632 | 1.00 | 44.27 | N |
| ATOM | 176 | CA | GLN | A | 52 | 47.541 | 14.509 | 46.946 | 1.00 | 44.27 | C |
| ATOM | 177 | C | GLN | A | 52 | 48.532 | 15.144 | 47.915 | 1.00 | 44.27 | C |
| ATOM | 178 | O | GLN | A | 52 | 48.202 | 15.421 | 49.077 | 1.00 | 44.27 | O |
| ATOM | 179 | CB | GLN | A | 52 | 47.076 | 15.564 | 45.958 | 0.00 | 68.01 | C |
| ATOM | 180 | CG | GLN | A | 52 | 45.589 | 15.689 | 45.884 | 0.00 | 68.01 | C |
| ATOM | 181 | CD | GLN | A | 52 | 45.145 | 16.042 | 44.505 | 1.00 | 68.01 | C |
| ATOM | 182 | OE1 | GLN | A | 52 | 45.422 | 17.145 | 44.014 | 1.00 | 68.01 | O |

| | | | | | | | | | | | |
|---|---|---|---|---|---|---|---|---|---|---|---|
| ATOM | 183 | NE2 | GLN | A | 52 | 44.464 | 15.104 | 43.846 | 1.00 | 68.01 | N |
| ATOM | 184 | N | GLU | A | 53 | 49.753 | 15.349 | 47.428 | 1.00 | 37.18 | N |
| ATOM | 185 | CA | GLU | A | 53 | 50.809 | 15.972 | 48.211 | 1.00 | 37.18 | C |
| ATOM | 186 | C | GLU | A | 53 | 50.690 | 17.475 | 48.033 | 1.00 | 37.18 | C |
| ATOM | 187 | O | GLU | A | 53 | 50.883 | 17.987 | 46.932 | 1.00 | 37.18 | O |
| ATOM | 188 | CB | GLU | A | 53 | 52.187 | 15.540 | 47.716 | 1.00 | 47.66 | C |
| ATOM | 189 | CG | GLU | A | 53 | 52.569 | 14.112 | 48.019 | 1.00 | 47.66 | C |
| ATOM | 190 | CD | GLU | A | 53 | 53.813 | 14.034 | 48.875 | 1.00 | 47.66 | C |
| ATOM | 191 | OE1 | GLU | A | 53 | 54.795 | 14.750 | 48.556 | 1.00 | 47.66 | O |
| ATOM | 192 | OE2 | GLU | A | 53 | 53.805 | 13.256 | 49.856 | 1.00 | 47.66 | O |
| ATOM | 193 | N | VAL | A | 54 | 50.374 | 18.180 | 49.112 | 1.00 | 39.29 | N |
| ATOM | 194 | CA | VAL | A | 54 | 50.259 | 19.621 | 49.037 | 1.00 | 39.29 | C |
| ATOM | 195 | C | VAL | A | 54 | 51.333 | 20.241 | 49.910 | 1.00 | 39.29 | C |
| ATOM | 196 | O | VAL | A | 54 | 51.416 | 19.967 | 51.107 | 1.00 | 39.29 | O |
| ATOM | 197 | CB | VAL | A | 54 | 48.863 | 20.083 | 49.473 | 1.00 | 31.58 | C |
| ATOM | 198 | CG1 | VAL | A | 54 | 48.758 | 21.599 | 49.382 | 1.00 | 31.58 | C |
| ATOM | 199 | CG2 | VAL | A | 54 | 47.817 | 19.435 | 48.578 | 1.00 | 31.58 | C |
| ATOM | 200 | N | SER | A | 55 | 52.173 | 21.065 | 49.295 | 1.00 | 42.34 | N |
| ATOM | 201 | CA | SER | A | 55 | 53.259 | 21.708 | 50.020 | 1.00 | 42.34 | C |
| ATOM | 202 | C | SER | A | 55 | 53.152 | 23.226 | 49.958 | 1.00 | 42.34 | C |
| ATOM | 203 | O | SER | A | 55 | 53.131 | 23.829 | 48.878 | 1.00 | 42.34 | O |
| ATOM | 204 | CB | SER | A | 55 | 54.603 | 21.292 | 49.435 | 1.00 | 48.76 | C |
| ATOM | 205 | OG | SER | A | 55 | 54.845 | 21.999 | 48.229 | 1.00 | 48.76 | O |
| ATOM | 206 | N | TYR | A | 56 | 53.110 | 23.846 | 51.127 | 1.00 | 56.49 | N |
| ATOM | 207 | CA | TYR | A | 56 | 53.002 | 25.290 | 51.189 | 1.00 | 56.49 | C |
| ATOM | 208 | C | TYR | A | 56 | 54.009 | 25.847 | 52.149 | 1.00 | 56.49 | C |
| ATOM | 209 | O | TYR | A | 56 | 54.557 | 25.121 | 52.976 | 1.00 | 56.49 | O |
| ATOM | 210 | CB | TYR | A | 56 | 51.612 | 25.691 | 51.652 | 1.00 | 34.56 | C |
| ATOM | 211 | CG | TYR | A | 56 | 51.278 | 25.232 | 53.053 | 1.00 | 34.56 | C |
| ATOM | 212 | CD1 | TYR | A | 56 | 51.574 | 26.019 | 54.164 | 1.00 | 34.56 | C |
| ATOM | 213 | CD2 | TYR | A | 56 | 50.594 | 24.038 | 53.257 | 1.00 | 34.56 | C |
| ATOM | 214 | CE1 | TYR | A | 56 | 51.173 | 25.634 | 55.444 | 1.00 | 34.56 | C |
| ATOM | 215 | CE2 | TYR | A | 56 | 50.198 | 23.644 | 54.523 | 1.00 | 34.56 | C |
| ATOM | 216 | CZ | TYR | A | 56 | 50.484 | 24.448 | 55.612 | 1.00 | 34.56 | C |
| ATOM | 217 | OH | TYR | A | 56 | 50.032 | 24.068 | 56.858 | 1.00 | 34.56 | O |
| ATOM | 218 | N | THR | A | 57 | 54.239 | 27.147 | 52.047 | 1.00 | 50.93 | N |
| ATOM | 219 | CA | THR | A | 57 | 55.182 | 27.802 | 52.923 | 1.00 | 50.93 | C |
| ATOM | 220 | C | THR | A | 57 | 54.670 | 29.187 | 53.287 | 1.00 | 50.93 | C |
| ATOM | 221 | O | THR | A | 57 | 53.611 | 29.606 | 52.811 | 1.00 | 50.93 | O |
| ATOM | 222 | CB | THR | A | 57 | 56.536 | 27.929 | 52.243 | 1.00 | 66.35 | C |
| ATOM | 223 | OG1 | THR | A | 57 | 57.426 | 28.632 | 53.113 | 1.00 | 66.35 | O |
| ATOM | 224 | CG2 | THR | A | 57 | 56.408 | 28.684 | 50.934 | 0.00 | 66.35 | C |
| ATOM | 225 | N | ASP | A | 58 | 55.423 | 29.888 | 54.133 | 1.00 | 59.63 | N |
| ATOM | 226 | CA | ASP | A | 58 | 55.063 | 31.237 | 54.560 | 1.00 | 59.63 | C |
| ATOM | 227 | C | ASP | A | 58 | 53.720 | 31.256 | 55.299 | 1.00 | 59.63 | C |
| ATOM | 228 | O | ASP | A | 58 | 52.869 | 32.118 | 55.031 | 1.00 | 59.63 | O |
| ATOM | 229 | CB | ASP | A | 58 | 54.980 | 32.180 | 53.344 | 1.00 | 61.93 | C |
| ATOM | 230 | CG | ASP | A | 58 | 56.237 | 32.150 | 52.485 | 1.00 | 61.93 | C |
| ATOM | 231 | OD1 | ASP | A | 58 | 57.277 | 31.664 | 52.987 | 1.00 | 61.93 | O |
| ATOM | 232 | OD2 | ASP | A | 58 | 56.190 | 32.625 | 51.319 | 1.00 | 61.93 | O |
| ATOM | 233 | N | THR | A | 59 | 53.518 | 30.320 | 56.222 | 1.00 | 48.28 | N |
| ATOM | 234 | CA | THR | A | 59 | 52.257 | 30.288 | 56.955 | 1.00 | 48.28 | C |
| ATOM | 235 | C | THR | A | 59 | 52.272 | 31.364 | 58.024 | 1.00 | 48.28 | C |
| ATOM | 236 | O | THR | A | 59 | 53.131 | 31.352 | 58.906 | 1.00 | 48.28 | O |
| ATOM | 237 | CB | THR | A | 59 | 52.004 | 28.919 | 57.644 | 1.00 | 63.84 | C |
| ATOM | 238 | OG1 | THR | A | 59 | 52.862 | 28.789 | 58.787 | 1.00 | 63.84 | O |
| ATOM | 239 | CG2 | THR | A | 59 | 52.280 | 27.775 | 56.680 | 1.00 | 63.84 | C |
| ATOM | 240 | N | LYS | A | 60 | 51.323 | 32.295 | 57.928 | 1.00 | 61.08 | N |
| ATOM | 241 | CA | LYS | A | 60 | 51.191 | 33.403 | 58.884 | 1.00 | 61.08 | C |
| ATOM | 242 | C | LYS | A | 60 | 49.749 | 33.499 | 59.376 | 1.00 | 61.08 | C |
| ATOM | 243 | O | LYS | A | 60 | 48.818 | 33.584 | 58.569 | 1.00 | 61.08 | O |
| ATOM | 244 | CB | LYS | A | 60 | 51.616 | 34.739 | 58.249 | 1.00 | 57.34 | C |

| | | | | | | | | | | | |
|---|---|---|---|---|---|---|---|---|---|---|---|
| ATOM | 245 | CG | LYS | A | 60 | 51.353 | 34.847 | 56.759 | 1.00 | 57.34 | C |
| ATOM | 246 | CD | LYS | A | 60 | 51.449 | 36.292 | 56.249 | 1.00 | 57.34 | C |
| ATOM | 247 | CE | LYS | A | 60 | 52.858 | 36.868 | 56.356 | 1.00 | 57.34 | C |
| ATOM | 248 | NZ | LYS | A | 60 | 53.897 | 36.075 | 55.626 | 1.00 | 57.34 | N |
| ATOM | 249 | N | VAL | A | 61 | 49.588 | 33.492 | 60.701 | 1.00 | 65.10 | N |
| ATOM | 250 | CA | VAL | A | 61 | 48.289 | 33.536 | 61.377 | 1.00 | 65.10 | C |
| ATOM | 251 | C | VAL | A | 61 | 47.505 | 34.808 | 61.113 | 1.00 | 65.10 | C |
| ATOM | 252 | O | VAL | A | 61 | 47.152 | 35.526 | 62.047 | 1.00 | 65.10 | O |
| ATOM | 253 | CB | VAL | A | 61 | 48.455 | 33.391 | 62.919 | 1.00 | 40.01 | C |
| ATOM | 254 | CG1 | VAL | A | 61 | 47.101 | 33.164 | 63.578 | 0.00 | 40.01 | C |
| ATOM | 255 | CG2 | VAL | A | 61 | 49.407 | 32.249 | 63.238 | 0.00 | 40.01 | C |
| ATOM | 256 | N | ILE | A | 62 | 47.207 | 35.073 | 59.846 | 1.00 | 100.00 | N |
| ATOM | 257 | CA | ILE | A | 62 | 46.467 | 36.277 | 59.455 | 1.00 | 100.00 | C |
| ATOM | 258 | C | ILE | A | 62 | 45.156 | 36.519 | 60.196 | 1.00 | 100.00 | C |
| ATOM | 259 | O | ILE | A | 62 | 44.836 | 37.668 | 60.531 | 1.00 | 100.00 | O |
| ATOM | 260 | CB | ILE | A | 62 | 46.099 | 36.274 | 57.956 | 1.00 | 50.02 | C |
| ATOM | 261 | CG1 | ILE | A | 62 | 45.123 | 35.123 | 57.678 | 1.00 | 50.02 | C |
| ATOM | 262 | CG2 | ILE | A | 62 | 47.362 | 36.214 | 57.105 | 1.00 | 50.02 | C |
| ATOM | 263 | CD1 | ILE | A | 62 | 44.528 | 35.137 | 56.283 | 1.00 | 50.02 | C |
| ATOM | 264 | N | GLY | A | 63 | 44.386 | 35.457 | 60.427 | 1.00 | 44.28 | N |
| ATOM | 265 | CA | GLY | A | 63 | 43.111 | 35.635 | 61.087 | 1.00 | 44.28 | C |
| ATOM | 266 | C | GLY | A | 63 | 42.932 | 34.729 | 62.272 | 1.00 | 44.28 | C |
| ATOM | 267 | O | GLY | A | 63 | 43.567 | 33.674 | 62.384 | 1.00 | 44.28 | O |
| ATOM | 268 | N | ASN | A | 64 | 42.052 | 35.156 | 63.163 | 1.00 | 62.35 | N |
| ATOM | 269 | CA | ASN | A | 64 | 41.742 | 34.426 | 64.384 | 1.00 | 62.35 | C |
| ATOM | 270 | C | ASN | A | 64 | 40.240 | 34.409 | 64.673 | 1.00 | 62.35 | C |
| ATOM | 271 | O | ASN | A | 64 | 39.780 | 34.764 | 65.750 | 1.00 | 62.35 | O |
| ATOM | 272 | CB | ASN | A | 64 | 42.484 | 35.099 | 65.540 | 1.00 | 83.43 | C |
| ATOM | 273 | CG | ASN | A | 64 | 42.465 | 34.196 | 66.747 | 1.00 | 83.43 | C |
| ATOM | 274 | OD1 | ASN | A | 64 | 41.449 | 34.028 | 67.415 | 1.00 | 83.43 | O |
| ATOM | 275 | ND2 | ASN | A | 64 | 43.645 | 33.630 | 67.054 | 0.00 | 83.43 | N |
| ATOM | 276 | N | GLY | A | 65 | 39.455 | 34.023 | 63.653 | 1.00 | 85.88 | N |
| ATOM | 277 | CA | GLY | A | 65 | 38.018 | 33.917 | 63.883 | 1.00 | 85.88 | C |
| ATOM | 278 | C | GLY | A | 65 | 37.748 | 33.418 | 65.305 | 1.00 | 85.88 | C |
| ATOM | 279 | O | GLY | A | 65 | 38.578 | 33.522 | 66.198 | 1.00 | 85.88 | O |
| ATOM | 280 | N | SER | A | 66 | 36.527 | 32.884 | 65.514 | 1.00 | 83.87 | N |
| ATOM | 281 | CA | SER | A | 66 | 36.196 | 32.388 | 66.845 | 1.00 | 83.87 | C |
| ATOM | 282 | C | SER | A | 66 | 36.304 | 30.866 | 66.942 | 1.00 | 83.87 | C |
| ATOM | 283 | O | SER | A | 66 | 36.889 | 30.311 | 67.865 | 1.00 | 83.87 | O |
| ATOM | 284 | CB | SER | A | 66 | 34.781 | 32.841 | 67.208 | 1.00 | 65.14 | C |
| ATOM | 285 | OG | SER | A | 66 | 34.043 | 33.090 | 66.010 | 1.00 | 65.14 | O |
| ATOM | 286 | N | PHE | A | 67 | 35.683 | 30.179 | 65.963 | 1.00 | 87.07 | N |
| ATOM | 287 | CA | PHE | A | 67 | 35.646 | 28.718 | 66.018 | 1.00 | 87.07 | C |
| ATOM | 288 | C | PHE | A | 67 | 37.039 | 28.094 | 65.947 | 1.00 | 87.07 | C |
| ATOM | 289 | O | PHE | A | 67 | 37.275 | 26.976 | 66.391 | 1.00 | 87.07 | O |
| ATOM | 290 | CB | PHE | A | 67 | 34.791 | 28.204 | 64.858 | 1.00 | 77.20 | C |
| ATOM | 291 | CG | PHE | A | 67 | 33.373 | 28.655 | 65.042 | 1.00 | 77.20 | C |
| ATOM | 292 | CD1 | PHE | A | 67 | 33.037 | 29.966 | 64.738 | 1.00 | 77.20 | C |
| ATOM | 293 | CD2 | PHE | A | 67 | 32.443 | 27.805 | 65.618 | 1.00 | 77.20 | C |
| ATOM | 294 | CE1 | PHE | A | 67 | 31.760 | 30.431 | 65.016 | 0.00 | 77.20 | C |
| ATOM | 295 | CE2 | PHE | A | 67 | 31.162 | 28.282 | 65.897 | 1.00 | 77.20 | C |
| ATOM | 296 | CZ | PHE | A | 67 | 30.817 | 29.594 | 65.597 | 1.00 | 77.20 | C |
| ATOM | 297 | N | GLY | A | 68 | 37.973 | 28.836 | 65.331 | 1.00 | 62.91 | N |
| ATOM | 298 | CA | GLY | A | 68 | 39.311 | 28.276 | 65.181 | 1.00 | 62.91 | C |
| ATOM | 299 | C | GLY | A | 68 | 40.320 | 29.337 | 64.736 | 1.00 | 62.91 | C |
| ATOM | 300 | O | GLY | A | 68 | 40.232 | 30.510 | 65.075 | 1.00 | 62.91 | O |
| ATOM | 301 | N | VAL | A | 69 | 41.335 | 28.875 | 63.983 | 1.00 | 53.87 | N |
| ATOM | 302 | CA | VAL | A | 69 | 42.347 | 29.803 | 63.495 | 1.00 | 53.87 | C |
| ATOM | 303 | C | VAL | A | 69 | 42.351 | 29.872 | 61.967 | 1.00 | 53.87 | C |
| ATOM | 304 | O | VAL | A | 69 | 42.098 | 28.900 | 61.268 | 1.00 | 53.87 | O |
| ATOM | 305 | CB | VAL | A | 69 | 43.710 | 29.329 | 63.999 | 1.00 | 67.84 | C |
| ATOM | 306 | CG1 | VAL | A | 69 | 44.227 | 30.280 | 65.077 | 0.00 | 67.84 | C |

| | | | | | | | | | | | |
|---|---|---|---|---|---|---|---|---|---|---|---|
| ATOM | 307 | CG2 | VAL | A | 69 | 43.591 | 27.932 | 64.578 | 1.00 | 67.84 | C |
| ATOM | 308 | N | VAL | A | 70 | 42.610 | 31.089 | 61.456 | 1.00 | 54.39 | N |
| ATOM | 309 | CA | VAL | A | 70 | 42.671 | 31.266 | 60.011 | 1.00 | 54.39 | C |
| ATOM | 310 | C | VAL | A | 70 | 44.100 | 31.567 | 59.551 | 1.00 | 54.39 | C |
| ATOM | 311 | O | VAL | A | 70 | 44.699 | 32.576 | 59.899 | 1.00 | 54.39 | O |
| ATOM | 312 | CB | VAL | A | 70 | 41.745 | 32.424 | 59.634 | 1.00 | 28.35 | C |
| ATOM | 313 | CG1 | VAL | A | 70 | 41.969 | 32.815 | 58.174 | 1.00 | 28.35 | C |
| ATOM | 314 | CG2 | VAL | A | 70 | 40.297 | 32.015 | 59.826 | 0.00 | 28.35 | C |
| ATOM | 315 | N | TYR | A | 71 | 44.661 | 30.622 | 58.773 | 1.00 | 54.24 | N |
| ATOM | 316 | CA | TYR | A | 71 | 46.038 | 30.785 | 58.321 | 1.00 | 54.24 | C |
| ATOM | 317 | C | TYR | A | 71 | 46.112 | 31.192 | 56.847 | 1.00 | 54.24 | C |
| ATOM | 318 | O | TYR | A | 71 | 45.257 | 30.869 | 56.033 | 1.00 | 54.24 | O |
| ATOM | 319 | CB | TYR | A | 71 | 46.765 | 29.456 | 58.521 | 1.00 | 56.39 | C |
| ATOM | 320 | CG | TYR | A | 71 | 46.872 | 29.146 | 59.971 | 1.00 | 56.39 | C |
| ATOM | 321 | CD1 | TYR | A | 71 | 45.925 | 28.324 | 60.574 | 1.00 | 56.39 | C |
| ATOM | 322 | CD2 | TYR | A | 71 | 47.969 | 29.593 | 60.709 | 1.00 | 56.39 | C |
| ATOM | 323 | CE1 | TYR | A | 71 | 46.079 | 27.938 | 61.897 | 1.00 | 56.39 | C |
| ATOM | 324 | CE2 | TYR | A | 71 | 48.123 | 29.207 | 62.033 | 1.00 | 56.39 | C |
| ATOM | 325 | CZ | TYR | A | 71 | 47.187 | 28.379 | 62.624 | 1.00 | 56.39 | C |
| ATOM | 326 | OH | TYR | A | 71 | 47.363 | 27.944 | 63.923 | 1.00 | 56.39 | O |
| ATOM | 327 | N | GLN | A | 72 | 47.169 | 31.961 | 56.523 | 1.00 | 40.81 | N |
| ATOM | 328 | CA | GLN | A | 72 | 47.368 | 32.365 | 55.136 | 1.00 | 40.81 | C |
| ATOM | 329 | C | GLN | A | 72 | 48.642 | 31.748 | 54.552 | 1.00 | 40.81 | C |
| ATOM | 330 | O | GLN | A | 72 | 49.706 | 31.764 | 55.158 | 1.00 | 40.81 | O |
| ATOM | 331 | CB | GLN | A | 72 | 47.460 | 33.891 | 55.091 | 1.00 | 71.49 | C |
| ATOM | 332 | CG | GLN | A | 72 | 47.226 | 34.453 | 53.687 | 0.00 | 71.49 | C |
| ATOM | 333 | CD | GLN | A | 72 | 48.554 | 34.639 | 52.992 | 1.00 | 71.49 | C |
| ATOM | 334 | OE1 | GLN | A | 72 | 49.409 | 33.770 | 52.951 | 1.00 | 71.49 | O |
| ATOM | 335 | NE2 | GLN | A | 72 | 48.699 | 35.846 | 52.409 | 1.00 | 71.49 | N |
| ATOM | 336 | N | ALA | A | 73 | 48.711 | 31.077 | 53.405 | 1.00 | 48.24 | N |
| ATOM | 337 | CA | ALA | A | 73 | 49.960 | 30.453 | 52.989 | 1.00 | 48.24 | C |
| ATOM | 338 | C | ALA | A | 73 | 50.239 | 30.505 | 51.493 | 1.00 | 48.24 | C |
| ATOM | 339 | O | ALA | A | 73 | 49.409 | 30.945 | 50.688 | 1.00 | 48.24 | O |
| ATOM | 340 | CB | ALA | A | 73 | 50.006 | 29.006 | 53.472 | 1.00 | 31.25 | C |
| ATOM | 341 | N | LYS | A | 74 | 51.428 | 30.034 | 51.131 | 1.00 | 51.92 | N |
| ATOM | 342 | CA | LYS | A | 74 | 51.883 | 30.029 | 49.751 | 1.00 | 51.92 | C |
| ATOM | 343 | C | LYS | A | 74 | 52.137 | 28.589 | 49.294 | 1.00 | 51.92 | C |
| ATOM | 344 | O | LYS | A | 74 | 52.856 | 27.839 | 49.962 | 1.00 | 51.92 | O |
| ATOM | 345 | CB | LYS | A | 74 | 53.175 | 30.858 | 49.653 | 1.00 | 49.31 | C |
| ATOM | 346 | CG | LYS | A | 74 | 53.682 | 31.081 | 48.233 | 1.00 | 49.31 | C |
| ATOM | 347 | CD | LYS | A | 74 | 54.790 | 32.136 | 48.168 | 1.00 | 49.31 | C |
| ATOM | 348 | CE | LYS | A | 74 | 55.300 | 32.298 | 46.743 | 0.00 | 49.31 | C |
| ATOM | 349 | NZ | LYS | A | 74 | 55.857 | 31.026 | 46.202 | 0.00 | 49.31 | N |
| ATOM | 350 | N | LEU | A | 75 | 51.535 | 28.198 | 48.172 | 1.00 | 52.53 | N |
| ATOM | 351 | CA | LEU | A | 75 | 51.732 | 26.851 | 47.649 | 1.00 | 52.53 | C |
| ATOM | 352 | C | LEU | A | 75 | 53.084 | 26.845 | 46.934 | 1.00 | 52.53 | C |
| ATOM | 353 | O | LEU | A | 75 | 53.322 | 27.649 | 46.022 | 1.00 | 52.53 | O |
| ATOM | 354 | CB | LEU | A | 75 | 50.612 | 26.480 | 46.674 | 1.00 | 33.25 | C |
| ATOM | 355 | CG | LEU | A | 75 | 49.186 | 26.317 | 47.227 | 1.00 | 33.25 | C |
| ATOM | 356 | CD1 | LEU | A | 75 | 48.275 | 25.795 | 46.118 | 1.00 | 33.25 | C |
| ATOM | 357 | CD2 | LEU | A | 75 | 49.175 | 25.362 | 48.406 | 1.00 | 33.25 | C |
| ATOM | 358 | N | CYS | A | 76 | 53.967 | 25.945 | 47.362 | 1.00 | 54.76 | N |
| ATOM | 359 | CA | CYS | A | 76 | 55.308 | 25.843 | 46.799 | 1.00 | 54.76 | C |
| ATOM | 360 | C | CYS | A | 76 | 55.424 | 25.778 | 45.289 | 1.00 | 54.76 | C |
| ATOM | 361 | O | CYS | A | 76 | 56.356 | 26.346 | 44.712 | 1.00 | 54.76 | O |
| ATOM | 362 | CB | CYS | A | 76 | 56.016 | 24.635 | 47.368 | 1.00 | 54.02 | C |
| ATOM | 363 | SG | CYS | A | 76 | 56.149 | 24.736 | 49.116 | 1.00 | 54.02 | S |
| ATOM | 364 | N | ASP | A | 77 | 54.500 | 25.081 | 44.641 | 1.00 | 52.22 | N |
| ATOM | 365 | CA | ASP | A | 77 | 54.577 | 24.951 | 43.200 | 1.00 | 52.22 | C |
| ATOM | 366 | C | ASP | A | 77 | 53.997 | 26.139 | 42.424 | 1.00 | 52.22 | C |
| ATOM | 367 | O | ASP | A | 77 | 54.756 | 26.939 | 41.860 | 1.00 | 52.22 | O |
| ATOM | 368 | CB | ASP | A | 77 | 53.931 | 23.627 | 42.797 | 1.00 | 81.37 | C |

| | | | | | | | | | | | |
|---|---|---|---|---|---|---|---|---|---|---|---|
| ATOM | 369 | CG | ASP | A | 77 | 54.744 | 22.412 | 43.293 | 1.00 | 81.37 | C |
| ATOM | 370 | OD1 | ASP | A | 77 | 55.813 | 22.630 | 43.931 | 1.00 | 81.37 | O |
| ATOM | 371 | OD2 | ASP | A | 77 | 54.323 | 21.245 | 43.045 | 1.00 | 81.37 | O |
| ATOM | 372 | N | SER | A | 78 | 52.669 | 26.263 | 42.397 | 1.00 | 65.45 | N |
| ATOM | 373 | CA | SER | A | 78 | 52.013 | 27.373 | 41.693 | 1.00 | 65.45 | C |
| ATOM | 374 | C | SER | A | 78 | 52.378 | 28.736 | 42.305 | 1.00 | 65.45 | C |
| ATOM | 375 | O | SER | A | 78 | 52.338 | 29.770 | 41.633 | 1.00 | 65.45 | O |
| ATOM | 376 | CB | SER | A | 78 | 50.491 | 27.216 | 41.759 | 1.00 | 60.65 | C |
| ATOM | 377 | OG | SER | A | 78 | 50.021 | 27.564 | 43.056 | 1.00 | 60.65 | O |
| ATOM | 378 | N | GLY | A | 79 | 52.726 | 28.738 | 43.586 | 1.00 | 63.24 | N |
| ATOM | 379 | CA | GLY | A | 79 | 53.054 | 29.993 | 44.237 | 1.00 | 63.24 | C |
| ATOM | 380 | C | GLY | A | 79 | 51.776 | 30.710 | 44.655 | 1.00 | 63.24 | C |
| ATOM | 381 | O | GLY | A | 79 | 51.825 | 31.794 | 45.250 | 1.00 | 63.24 | O |
| ATOM | 382 | N | GLU | A | 80 | 50.624 | 30.107 | 44.350 | 1.00 | 59.92 | N |
| ATOM | 383 | CA | GLU | A | 80 | 49.333 | 30.703 | 44.706 | 1.00 | 59.92 | C |
| ATOM | 384 | C | GLU | A | 80 | 49.184 | 30.881 | 46.208 | 1.00 | 59.92 | C |
| ATOM | 385 | O | GLU | A | 80 | 49.721 | 30.101 | 46.995 | 1.00 | 59.92 | O |
| ATOM | 386 | CB | GLU | A | 80 | 48.188 | 29.833 | 44.200 | 1.00 | 74.64 | C |
| ATOM | 387 | CG | GLU | A | 80 | 48.007 | 29.855 | 42.703 | 1.00 | 74.64 | C |
| ATOM | 388 | CD | GLU | A | 80 | 47.013 | 28.820 | 42.260 | 1.00 | 74.64 | C |
| ATOM | 389 | OE1 | GLU | A | 80 | 47.311 | 27.607 | 42.398 | 1.00 | 74.64 | O |
| ATOM | 390 | OE2 | GLU | A | 80 | 45.923 | 29.220 | 41.789 | 1.00 | 74.64 | O |
| ATOM | 391 | N | LEU | A | 81 | 48.456 | 31.913 | 46.609 | 1.00 | 43.62 | N |
| ATOM | 392 | CA | LEU | A | 81 | 48.247 | 32.168 | 48.023 | 1.00 | 43.62 | C |
| ATOM | 393 | C | LEU | A | 81 | 46.940 | 31.545 | 48.458 | 1.00 | 43.62 | C |
| ATOM | 394 | O | LEU | A | 81 | 45.888 | 31.822 | 47.893 | 1.00 | 43.62 | O |
| ATOM | 395 | CB | LEU | A | 81 | 48.234 | 33.673 | 48.291 | 1.00 | 60.15 | C |
| ATOM | 396 | CG | LEU | A | 81 | 49.631 | 34.295 | 48.325 | 1.00 | 60.15 | C |
| ATOM | 397 | CD1 | LEU | A | 81 | 49.555 | 35.818 | 48.466 | 1.00 | 60.15 | C |
| ATOM | 398 | CD2 | LEU | A | 81 | 50.388 | 33.674 | 49.507 | 1.00 | 60.15 | C |
| ATOM | 399 | N | VAL | A | 82 | 47.007 | 30.685 | 49.456 | 1.00 | 32.15 | N |
| ATOM | 400 | CA | VAL | A | 82 | 45.812 | 30.031 | 49.940 | 1.00 | 32.15 | C |
| ATOM | 401 | C | VAL | A | 82 | 45.549 | 30.482 | 51.355 | 1.00 | 32.15 | C |
| ATOM | 402 | O | VAL | A | 82 | 46.362 | 31.183 | 51.948 | 1.00 | 32.15 | O |
| ATOM | 403 | CB | VAL | A | 82 | 45.966 | 28.489 | 49.920 | 1.00 | 25.28 | C |
| ATOM | 404 | CG1 | VAL | A | 82 | 45.867 | 27.974 | 48.489 | 1.00 | 25.28 | C |
| ATOM | 405 | CG2 | VAL | A | 82 | 47.303 | 28.094 | 50.533 | 1.00 | 25.28 | C |
| ATOM | 406 | N | ALA | A | 83 | 44.401 | 30.080 | 51.881 | 1.00 | 36.55 | N |
| ATOM | 407 | CA | ALA | A | 83 | 44.011 | 30.413 | 53.238 | 1.00 | 36.55 | C |
| ATOM | 408 | C | ALA | A | 83 | 43.668 | 29.103 | 53.904 | 1.00 | 36.55 | C |
| ATOM | 409 | O | ALA | A | 83 | 43.085 | 28.211 | 53.284 | 1.00 | 36.55 | O |
| ATOM | 410 | CB | ALA | A | 83 | 42.798 | 31.320 | 53.228 | 1.00 | 40.81 | C |
| ATOM | 411 | N | ILE | A | 84 | 44.019 | 28.973 | 55.170 | 1.00 | 42.30 | N |
| ATOM | 412 | CA | ILE | A | 84 | 43.734 | 27.736 | 55.858 | 1.00 | 42.30 | C |
| ATOM | 413 | C | ILE | A | 84 | 42.967 | 28.008 | 57.132 | 1.00 | 42.30 | C |
| ATOM | 414 | O | ILE | A | 84 | 43.491 | 28.607 | 58.073 | 1.00 | 42.30 | O |
| ATOM | 415 | CB | ILE | A | 84 | 45.046 | 26.990 | 56.166 | 1.00 | 28.52 | C |
| ATOM | 416 | CG1 | ILE | A | 84 | 45.822 | 26.789 | 54.853 | 1.00 | 28.52 | C |
| ATOM | 417 | CG2 | ILE | A | 84 | 44.747 | 25.665 | 56.860 | 1.00 | 28.52 | C |
| ATOM | 418 | CD1 | ILE | A | 84 | 47.146 | 26.082 | 54.999 | 1.00 | 28.52 | C |
| ATOM | 419 | N | LYS | A | 85 | 41.713 | 27.589 | 57.158 | 1.00 | 34.53 | N |
| ATOM | 420 | CA | LYS | A | 85 | 40.910 | 27.801 | 58.343 | 1.00 | 34.53 | C |
| ATOM | 421 | C | LYS | A | 85 | 40.972 | 26.534 | 59.182 | 1.00 | 34.53 | C |
| ATOM | 422 | O | LYS | A | 85 | 40.342 | 25.530 | 58.844 | 1.00 | 34.53 | O |
| ATOM | 423 | CB | LYS | A | 85 | 39.446 | 28.097 | 57.974 | 1.00 | 53.69 | C |
| ATOM | 424 | CG | LYS | A | 85 | 38.555 | 28.240 | 59.205 | 1.00 | 53.69 | C |
| ATOM | 425 | CD | LYS | A | 85 | 37.058 | 28.157 | 58.908 | 1.00 | 53.69 | C |
| ATOM | 426 | CE | LYS | A | 85 | 36.519 | 29.379 | 58.154 | 1.00 | 53.69 | C |
| ATOM | 427 | NZ | LYS | A | 85 | 35.021 | 29.335 | 58.021 | 1.00 | 53.69 | N |
| ATOM | 428 | N | LYS | A | 86 | 41.742 | 26.564 | 60.264 | 1.00 | 44.17 | N |
| ATOM | 429 | CA | LYS | A | 86 | 41.822 | 25.391 | 61.122 | 1.00 | 44.17 | C |
| ATOM | 430 | C | LYS | A | 86 | 40.811 | 25.537 | 62.239 | 1.00 | 44.17 | C |

| | | | | | | | | | | | |
|---|---|---|---|---|---|---|---|---|---|---|---|
| ATOM | 431 | O | LYS | A | 86 | 40.958 | 26.415 | 63.091 | 1.00 | 44.17 | O |
| ATOM | 432 | CB | LYS | A | 86 | 43.221 | 25.236 | 61.722 | 1.00 | 43.95 | C |
| ATOM | 433 | CG | LYS | A | 86 | 43.349 | 24.035 | 62.648 | 0.00 | 43.95 | C |
| ATOM | 434 | CD | LYS | A | 86 | 44.716 | 23.374 | 62.544 | 0.00 | 43.95 | C |
| ATOM | 435 | CE | LYS | A | 86 | 45.843 | 24.296 | 62.978 | 0.00 | 43.95 | C |
| ATOM | 436 | NZ | LYS | A | 86 | 47.169 | 23.621 | 62.840 | 1.00 | 43.95 | N |
| ATOM | 437 | N | VAL | A | 87 | 39.776 | 24.698 | 62.225 | 1.00 | 48.58 | N |
| ATOM | 438 | CA | VAL | A | 87 | 38.755 | 24.735 | 63.269 | 1.00 | 48.58 | C |
| ATOM | 439 | C | VAL | A | 87 | 38.853 | 23.404 | 64.014 | 1.00 | 48.58 | C |
| ATOM | 440 | O | VAL | A | 87 | 39.323 | 22.408 | 63.450 | 1.00 | 48.58 | O |
| ATOM | 441 | CB | VAL | A | 87 | 37.335 | 24.893 | 62.689 | 1.00 | 34.61 | C |
| ATOM | 442 | CG1 | VAL | A | 87 | 36.881 | 23.588 | 62.077 | 1.00 | 34.61 | C |
| ATOM | 443 | CG2 | VAL | A | 87 | 36.374 | 25.331 | 63.781 | 0.00 | 34.61 | C |
| ATOM | 444 | N | LEU | A | 88 | 38.406 | 23.392 | 65.272 | 1.00 | 79.34 | N |
| ATOM | 445 | CA | LEU | A | 88 | 38.484 | 22.196 | 66.119 | 1.00 | 79.34 | C |
| ATOM | 446 | C | LEU | A | 88 | 37.542 | 21.069 | 65.692 | 1.00 | 79.34 | C |
| ATOM | 447 | O | LEU | A | 88 | 36.480 | 20.871 | 66.284 | 1.00 | 79.34 | O |
| ATOM | 448 | CB | LEU | A | 88 | 38.216 | 22.578 | 67.582 | 1.00 | 59.72 | C |
| ATOM | 449 | CG | LEU | A | 88 | 38.780 | 21.662 | 68.674 | 1.00 | 59.72 | C |
| ATOM | 450 | CD1 | LEU | A | 88 | 38.094 | 20.304 | 68.667 | 0.00 | 59.72 | C |
| ATOM | 451 | CD2 | LEU | A | 88 | 40.285 | 21.517 | 68.446 | 1.00 | 59.72 | C |
| ATOM | 452 | N | GLN | A | 89 | 37.944 | 20.323 | 64.666 | 1.00 | 72.41 | N |
| ATOM | 453 | CA | GLN | A | 89 | 37.137 | 19.216 | 64.169 | 1.00 | 72.41 | C |
| ATOM | 454 | C | GLN | A | 89 | 36.832 | 18.254 | 65.306 | 1.00 | 72.41 | C |
| ATOM | 455 | O | GLN | A | 89 | 37.666 | 18.033 | 66.194 | 1.00 | 72.41 | O |
| ATOM | 456 | CB | GLN | A | 89 | 37.873 | 18.485 | 63.063 | 0.00 | 35.69 | C |
| ATOM | 457 | N | ASP | A | 90 | 35.630 | 17.687 | 65.269 | 1.00 | 90.18 | N |
| ATOM | 458 | CA | ASP | A | 90 | 35.181 | 16.732 | 66.281 | 1.00 | 90.18 | C |
| ATOM | 459 | C | ASP | A | 90 | 34.732 | 15.443 | 65.593 | 1.00 | 90.18 | C |
| ATOM | 460 | O | ASP | A | 90 | 34.280 | 15.470 | 64.449 | 0.00 | 90.18 | O |
| ATOM | 461 | CB | ASP | A | 90 | 34.010 | 17.330 | 67.078 | 1.00 | 57.39 | C |
| ATOM | 462 | N | LYS | A | 91 | 34.859 | 14.317 | 66.290 | 1.00 | 91.89 | N |
| ATOM | 463 | CA | LYS | A | 91 | 34.419 | 13.040 | 65.721 | 1.00 | 91.89 | C |
| ATOM | 464 | C | LYS | A | 91 | 32.926 | 12.808 | 66.058 | 1.00 | 91.89 | C |
| ATOM | 465 | O | LYS | A | 91 | 32.292 | 11.894 | 65.514 | 1.00 | 91.89 | O |
| ATOM | 466 | CB | LYS | A | 91 | 35.271 | 11.894 | 66.270 | 1.00 | 68.26 | C |
| ATOM | 467 | N | ARG | A | 92 | 32.374 | 13.646 | 66.940 | 1.00 | 72.03 | N |
| ATOM | 468 | CA | ARG | A | 92 | 30.972 | 13.530 | 67.347 | 1.00 | 72.03 | C |
| ATOM | 469 | C | ARG | A | 92 | 29.995 | 13.660 | 66.172 | 1.00 | 72.03 | C |
| ATOM | 470 | O | ARG | A | 92 | 28.799 | 13.360 | 66.325 | 1.00 | 72.03 | O |
| ATOM | 471 | CB | ARG | A | 92 | 30.642 | 14.592 | 68.420 | 1.00 | 56.05 | C |
| ATOM | 472 | N | PHE | A | 93 | 30.490 | 14.116 | 65.013 | 1.00 | 67.00 | N |
| ATOM | 473 | CA | PHE | A | 93 | 29.636 | 14.277 | 63.817 | 1.00 | 67.00 | C |
| ATOM | 474 | C | PHE | A | 93 | 30.360 | 14.863 | 62.589 | 1.00 | 67.00 | C |
| ATOM | 475 | O | PHE | A | 93 | 31.562 | 15.149 | 62.627 | 1.00 | 67.00 | O |
| ATOM | 476 | CB | PHE | A | 93 | 28.420 | 15.141 | 64.157 | 0.00 | 35.69 | C |
| ATOM | 477 | N | LYS | A | 94 | 29.624 | 15.031 | 61.494 | 1.00 | 51.48 | N |
| ATOM | 478 | CA | LYS | A | 94 | 30.203 | 15.594 | 60.273 | 1.00 | 51.48 | C |
| ATOM | 479 | C | LYS | A | 94 | 30.321 | 17.118 | 60.429 | 1.00 | 51.48 | C |
| ATOM | 480 | O | LYS | A | 94 | 29.620 | 17.738 | 61.250 | 1.00 | 51.48 | O |
| ATOM | 481 | CB | LYS | A | 94 | 29.331 | 15.253 | 59.071 | 0.00 | 35.69 | C |
| ATOM | 482 | N | ASN | A | 95 | 31.218 | 17.717 | 59.655 | 1.00 | 34.95 | N |
| ATOM | 483 | CA | ASN | A | 95 | 31.401 | 19.160 | 59.717 | 1.00 | 34.95 | C |
| ATOM | 484 | C | ASN | A | 95 | 30.562 | 19.796 | 58.608 | 1.00 | 34.95 | C |
| ATOM | 485 | O | ASN | A | 95 | 30.909 | 19.742 | 57.432 | 1.00 | 34.95 | O |
| ATOM | 486 | CB | ASN | A | 95 | 32.880 | 19.523 | 59.555 | 1.00 | 44.43 | C |
| ATOM | 487 | CG | ASN | A | 95 | 33.152 | 20.968 | 59.890 | 1.00 | 44.43 | C |
| ATOM | 488 | OD1 | ASN | A | 95 | 32.707 | 21.867 | 59.176 | 1.00 | 44.43 | O |
| ATOM | 489 | ND2 | ASN | A | 95 | 33.870 | 21.207 | 60.993 | 1.00 | 44.43 | N |
| ATOM | 490 | N | ARG | A | 96 | 29.438 | 20.379 | 59.008 | 1.00 | 34.56 | N |
| ATOM | 491 | CA | ARG | A | 96 | 28.504 | 21.016 | 58.090 | 1.00 | 34.56 | C |
| ATOM | 492 | C | ARG | A | 96 | 29.121 | 21.976 | 57.078 | 1.00 | 34.56 | C |

| | | | | | | | | | | | |
|---|---|---|---|---|---|---|---|---|---|---|---|
| ATOM | 493 | O | ARG | A | 96 | 28.735 | 21.963 | 55.912 | 1.00 | 34.56 | O |
| ATOM | 494 | CB | ARG | A | 96 | 27.424 | 21.745 | 58.882 | 1.00 | 40.73 | C |
| ATOM | 495 | CG | ARG | A | 96 | 26.445 | 22.485 | 58.012 | 1.00 | 40.73 | C |
| ATOM | 496 | CD | ARG | A | 96 | 25.496 | 23.294 | 58.854 | 1.00 | 40.73 | C |
| ATOM | 497 | NE | ARG | A | 96 | 24.681 | 22.440 | 59.702 | 1.00 | 40.73 | N |
| ATOM | 498 | CZ | ARG | A | 96 | 23.859 | 22.893 | 60.641 | 1.00 | 40.73 | C |
| ATOM | 499 | NH1 | ARG | A | 96 | 23.752 | 24.201 | 60.854 | 1.00 | 40.73 | N |
| ATOM | 500 | NH2 | ARG | A | 96 | 23.135 | 22.039 | 61.355 | 1.00 | 40.73 | N |
| ATOM | 501 | N | GLU | A | 97 | 30.058 | 22.815 | 57.512 | 1.00 | 27.76 | N |
| ATOM | 502 | CA | GLU | A | 97 | 30.686 | 23.751 | 56.591 | 1.00 | 27.76 | C |
| ATOM | 503 | C | GLU | A | 97 | 31.401 | 22.947 | 55.519 | 1.00 | 27.76 | C |
| ATOM | 504 | O | GLU | A | 97 | 31.151 | 23.130 | 54.330 | 1.00 | 27.76 | O |
| ATOM | 505 | CB | GLU | A | 97 | 31.666 | 24.684 | 57.324 | 1.00 | 34.86 | C |
| ATOM | 506 | CG | GLU | A | 97 | 32.493 | 25.561 | 56.381 | 1.00 | 34.86 | C |
| ATOM | 507 | CD | GLU | A | 97 | 33.175 | 26.742 | 57.065 | 1.00 | 34.86 | C |
| ATOM | 508 | OE1 | GLU | A | 97 | 33.379 | 26.690 | 58.299 | 1.00 | 34.86 | O |
| ATOM | 509 | OE2 | GLU | A | 97 | 33.524 | 27.719 | 56.353 | 1.00 | 34.86 | O |
| ATOM | 510 | N | LEU | A | 98 | 32.278 | 22.045 | 55.947 | 1.00 | 29.35 | N |
| ATOM | 511 | CA | LEU | A | 98 | 33.000 | 21.187 | 55.022 | 1.00 | 29.35 | C |
| ATOM | 512 | C | LEU | A | 98 | 32.024 | 20.498 | 54.079 | 1.00 | 29.35 | C |
| ATOM | 513 | O | LEU | A | 98 | 32.208 | 20.488 | 52.867 | 1.00 | 29.35 | O |
| ATOM | 514 | CB | LEU | A | 98 | 33.768 | 20.113 | 55.779 | 1.00 | 27.06 | C |
| ATOM | 515 | CG | LEU | A | 98 | 34.273 | 18.972 | 54.888 | 1.00 | 27.06 | C |
| ATOM | 516 | CD1 | LEU | A | 98 | 35.236 | 19.512 | 53.843 | 1.00 | 27.06 | C |
| ATOM | 517 | CD2 | LEU | A | 98 | 34.950 | 17.927 | 55.743 | 1.00 | 27.06 | C |
| ATOM | 518 | N | GLN | A | 99 | 30.981 | 19.911 | 54.635 | 1.00 | 35.85 | N |
| ATOM | 519 | CA | GLN | A | 99 | 30.027 | 19.227 | 53.795 | 1.00 | 35.85 | C |
| ATOM | 520 | C | GLN | A | 99 | 29.423 | 20.174 | 52.760 | 1.00 | 35.85 | C |
| ATOM | 521 | O | GLN | A | 99 | 29.413 | 19.870 | 51.560 | 1.00 | 35.85 | O |
| ATOM | 522 | CB | GLN | A | 99 | 28.935 | 18.554 | 54.649 | 1.00 | 48.31 | C |
| ATOM | 523 | CG | GLN | A | 99 | 29.467 | 17.459 | 55.595 | 0.00 | 48.31 | C |
| ATOM | 524 | CD | GLN | A | 99 | 30.595 | 16.603 | 54.997 | 1.00 | 48.31 | C |
| ATOM | 525 | OE1 | GLN | A | 99 | 30.452 | 16.003 | 53.926 | 1.00 | 48.31 | O |
| ATOM | 526 | NE2 | GLN | A | 99 | 31.722 | 16.544 | 55.700 | 1.00 | 48.31 | N |
| ATOM | 527 | N | ILE | A | 100 | 28.945 | 21.331 | 53.210 | 1.00 | 37.51 | N |
| ATOM | 528 | CA | ILE | A | 100 | 28.337 | 22.289 | 52.293 | 1.00 | 37.51 | C |
| ATOM | 529 | C | ILE | A | 100 | 29.301 | 22.772 | 51.214 | 1.00 | 37.51 | C |
| ATOM | 530 | O | ILE | A | 100 | 28.977 | 22.779 | 50.021 | 1.00 | 37.51 | O |
| ATOM | 531 | CB | ILE | A | 100 | 27.786 | 23.487 | 53.060 | 1.00 | 24.76 | C |
| ATOM | 532 | CG1 | ILE | A | 100 | 26.602 | 23.025 | 53.909 | 1.00 | 24.76 | C |
| ATOM | 533 | CG2 | ILE | A | 100 | 27.384 | 24.602 | 52.094 | 1.00 | 24.76 | C |
| ATOM | 534 | CD1 | ILE | A | 100 | 26.069 | 24.076 | 54.806 | 1.00 | 24.76 | C |
| ATOM | 535 | N | MET | A | 101 | 30.493 | 23.169 | 51.624 | 1.00 | 34.60 | N |
| ATOM | 536 | CA | MET | A | 101 | 31.452 | 23.646 | 50.657 | 1.00 | 34.60 | C |
| ATOM | 537 | C | MET | A | 101 | 31.939 | 22.556 | 49.733 | 1.00 | 34.60 | C |
| ATOM | 538 | O | MET | A | 101 | 32.421 | 22.831 | 48.637 | 1.00 | 34.60 | O |
| ATOM | 539 | CB | MET | A | 101 | 32.623 | 24.289 | 51.372 | 1.00 | 44.27 | C |
| ATOM | 540 | CG | MET | A | 101 | 32.278 | 25.669 | 51.904 | 1.00 | 44.27 | C |
| ATOM | 541 | SD | MET | A | 101 | 33.586 | 26.784 | 51.454 | 1.00 | 44.27 | S |
| ATOM | 542 | CE | MET | A | 101 | 34.778 | 26.342 | 52.697 | 1.00 | 44.27 | C |
| ATOM | 543 | N | ARG | A | 102 | 31.806 | 21.315 | 50.176 | 1.00 | 44.54 | N |
| ATOM | 544 | CA | ARG | A | 102 | 32.248 | 20.181 | 49.384 | 1.00 | 44.54 | C |
| ATOM | 545 | C | ARG | A | 102 | 31.359 | 20.058 | 48.137 | 1.00 | 44.54 | C |
| ATOM | 546 | O | ARG | A | 102 | 31.740 | 19.415 | 47.154 | 1.00 | 44.54 | O |
| ATOM | 547 | CB | ARG | A | 102 | 32.164 | 18.911 | 50.236 | 1.00 | 47.18 | C |
| ATOM | 548 | CG | ARG | A | 102 | 32.971 | 17.725 | 49.747 | 1.00 | 47.18 | C |
| ATOM | 549 | CD | ARG | A | 102 | 34.103 | 17.414 | 50.708 | 1.00 | 47.18 | C |
| ATOM | 550 | NE | ARG | A | 102 | 34.693 | 16.092 | 50.479 | 1.00 | 47.18 | N |
| ATOM | 551 | CZ | ARG | A | 102 | 34.083 | 14.933 | 50.737 | 1.00 | 47.18 | C |
| ATOM | 552 | NH1 | ARG | A | 102 | 32.850 | 14.915 | 51.241 | 1.00 | 47.18 | N |
| ATOM | 553 | NH2 | ARG | A | 102 | 34.706 | 13.789 | 50.488 | 0.00 | 47.18 | N |
| ATOM | 554 | N | LYS | A | 103 | 30.187 | 20.687 | 48.164 | 1.00 | 25.86 | N |

| | | | | | | | | | | | |
|---|---|---|---|---|---|---|---|---|---|---|---|
| ATOM | 555 | CA | LYS | A | 103 | 29.288 | 20.597 | 47.025 | 1.00 | 25.86 | C |
| ATOM | 556 | C | LYS | A | 103 | 29.047 | 21.891 | 46.269 | 1.00 | 25.86 | C |
| ATOM | 557 | O | LYS | A | 103 | 28.101 | 21.999 | 45.504 | 1.00 | 25.86 | O |
| ATOM | 558 | CB | LYS | A | 103 | 27.935 | 19.999 | 47.447 | 1.00 | 42.64 | C |
| ATOM | 559 | CG | LYS | A | 103 | 27.255 | 20.630 | 48.647 | 1.00 | 42.64 | C |
| ATOM | 560 | CD | LYS | A | 103 | 25.724 | 20.455 | 48.594 | 1.00 | 42.64 | C |
| ATOM | 561 | CE | LYS | A | 103 | 25.233 | 19.015 | 48.773 | 1.00 | 42.64 | C |
| ATOM | 562 | NZ | LYS | A | 103 | 25.461 | 18.448 | 50.150 | 1.00 | 42.64 | N |
| ATOM | 563 | N | LEU | A | 104 | 29.911 | 22.872 | 46.455 | 1.00 | 37.96 | N |
| ATOM | 564 | CA | LEU | A | 104 | 29.724 | 24.136 | 45.765 | 1.00 | 37.96 | C |
| ATOM | 565 | C | LEU | A | 104 | 30.795 | 24.393 | 44.722 | 1.00 | 37.96 | C |
| ATOM | 566 | O | LEU | A | 104 | 31.987 | 24.275 | 44.990 | 1.00 | 37.96 | O |
| ATOM | 567 | CB | LEU | A | 104 | 29.688 | 25.284 | 46.775 | 1.00 | 37.19 | C |
| ATOM | 568 | CG | LEU | A | 104 | 28.326 | 25.673 | 47.370 | 1.00 | 37.19 | C |
| ATOM | 569 | CD1 | LEU | A | 104 | 27.526 | 24.451 | 47.771 | 1.00 | 37.19 | C |
| ATOM | 570 | CD2 | LEU | A | 104 | 28.566 | 26.583 | 48.565 | 1.00 | 37.19 | C |
| ATOM | 571 | N | ASP | A | 105 | 30.354 | 24.713 | 43.513 | 1.00 | 35.85 | N |
| ATOM | 572 | CA | ASP | A | 105 | 31.274 | 25.017 | 42.434 | 1.00 | 35.85 | C |
| ATOM | 573 | C | ASP | A | 105 | 30.652 | 26.168 | 41.667 | 1.00 | 35.85 | C |
| ATOM | 574 | O | ASP | A | 105 | 29.788 | 25.960 | 40.810 | 1.00 | 35.85 | O |
| ATOM | 575 | CB | ASP | A | 105 | 31.470 | 23.811 | 41.521 | 0.00 | 46.91 | C |
| ATOM | 576 | CG | ASP | A | 105 | 32.491 | 24.073 | 40.443 | 1.00 | 46.91 | C |
| ATOM | 577 | OD1 | ASP | A | 105 | 33.552 | 24.660 | 40.761 | 1.00 | 46.91 | O |
| ATOM | 578 | OD2 | ASP | A | 105 | 32.238 | 23.696 | 39.278 | 1.00 | 46.91 | O |
| ATOM | 579 | N | HIS | A | 106 | 31.076 | 27.387 | 42.007 | 1.00 | 22.60 | N |
| ATOM | 580 | CA | HIS | A | 106 | 30.546 | 28.583 | 41.371 | 1.00 | 22.60 | C |
| ATOM | 581 | C | HIS | A | 106 | 31.606 | 29.665 | 41.329 | 1.00 | 22.60 | C |
| ATOM | 582 | O | HIS | A | 106 | 32.392 | 29.810 | 42.252 | 1.00 | 22.60 | O |
| ATOM | 583 | CB | HIS | A | 106 | 29.308 | 29.065 | 42.120 | 1.00 | 24.71 | C |
| ATOM | 584 | CG | HIS | A | 106 | 28.550 | 30.135 | 41.403 | 1.00 | 24.71 | C |
| ATOM | 585 | ND1 | HIS | A | 106 | 28.996 | 31.435 | 41.318 | 1.00 | 24.71 | N |
| ATOM | 586 | CD2 | HIS | A | 106 | 27.373 | 30.096 | 40.732 | 1.00 | 24.71 | C |
| ATOM | 587 | CE1 | HIS | A | 106 | 28.125 | 32.153 | 40.629 | 1.00 | 24.71 | C |
| ATOM | 588 | NE2 | HIS | A | 106 | 27.132 | 31.363 | 40.262 | 1.00 | 24.71 | N |
| ATOM | 589 | N | CYS | A | 107 | 31.620 | 30.424 | 40.241 | 1.00 | 38.47 | N |
| ATOM | 590 | CA | CYS | A | 107 | 32.616 | 31.468 | 40.052 | 1.00 | 38.47 | C |
| ATOM | 591 | C | CYS | A | 107 | 32.495 | 32.617 | 41.029 | 1.00 | 38.47 | C |
| ATOM | 592 | O | CYS | A | 107 | 33.287 | 33.543 | 40.986 | 1.00 | 38.47 | O |
| ATOM | 593 | CB | CYS | A | 107 | 32.562 | 32.007 | 38.616 | 1.00 | 37.90 | C |
| ATOM | 594 | SG | CYS | A | 107 | 31.007 | 32.828 | 38.143 | 1.00 | 37.90 | S |
| ATOM | 595 | N | ASN | A | 108 | 31.525 | 32.554 | 41.928 | 1.00 | 34.41 | N |
| ATOM | 596 | CA | ASN | A | 108 | 31.345 | 33.632 | 42.887 | 1.00 | 34.41 | C |
| ATOM | 597 | C | ASN | A | 108 | 31.354 | 33.178 | 44.338 | 1.00 | 34.41 | C |
| ATOM | 598 | O | ASN | A | 108 | 30.897 | 33.888 | 45.231 | 1.00 | 34.41 | O |
| ATOM | 599 | CB | ASN | A | 108 | 30.056 | 34.386 | 42.558 | 1.00 | 26.75 | C |
| ATOM | 600 | CG | ASN | A | 108 | 30.225 | 35.321 | 41.371 | 1.00 | 26.75 | C |
| ATOM | 601 | OD1 | ASN | A | 108 | 29.311 | 35.526 | 40.569 | 1.00 | 26.75 | O |
| ATOM | 602 | ND2 | ASN | A | 108 | 31.402 | 35.902 | 41.265 | 1.00 | 26.75 | N |
| ATOM | 603 | N | ILE | A | 109 | 31.880 | 31.985 | 44.574 | 1.00 | 26.40 | N |
| ATOM | 604 | CA | ILE | A | 109 | 31.970 | 31.463 | 45.929 | 1.00 | 26.40 | C |
| ATOM | 605 | C | ILE | A | 109 | 33.422 | 31.034 | 46.123 | 1.00 | 26.40 | C |
| ATOM | 606 | O | ILE | A | 109 | 34.013 | 30.445 | 45.217 | 1.00 | 26.40 | O |
| ATOM | 607 | CB | ILE | A | 109 | 31.049 | 30.245 | 46.116 | 1.00 | 20.95 | C |
| ATOM | 608 | CG1 | ILE | A | 109 | 29.592 | 30.648 | 45.890 | 1.00 | 20.95 | C |
| ATOM | 609 | CG2 | ILE | A | 109 | 31.243 | 29.660 | 47.496 | 1.00 | 20.95 | C |
| ATOM | 610 | CD1 | ILE | A | 109 | 28.635 | 29.474 | 45.843 | 0.00 | 20.95 | C |
| ATOM | 611 | N | VAL | A | 110 | 34.014 | 31.343 | 47.271 | 1.00 | 21.56 | N |
| ATOM | 612 | CA | VAL | A | 110 | 35.392 | 30.928 | 47.494 | 1.00 | 21.56 | C |
| ATOM | 613 | C | VAL | A | 110 | 35.488 | 29.447 | 47.184 | 1.00 | 21.56 | C |
| ATOM | 614 | O | VAL | A | 110 | 34.571 | 28.682 | 47.450 | 1.00 | 21.56 | O |
| ATOM | 615 | CB | VAL | A | 110 | 35.860 | 31.150 | 48.951 | 1.00 | 28.38 | C |
| ATOM | 616 | CG1 | VAL | A | 110 | 36.336 | 32.556 | 49.129 | 1.00 | 28.38 | C |

| | | | | | | | | | | | |
|---|---|---|---|---|---|---|---|---|---|---|---|
| ATOM | 617 | CG2 | VAL | A | 110 | 34.725 | 30.847 | 49.914 | 1.00 | 28.38 | C |
| ATOM | 618 | N | ARG | A | 111 | 36.619 | 29.061 | 46.620 | 1.00 | 26.01 | N |
| ATOM | 619 | CA | ARG | A | 111 | 36.882 | 27.689 | 46.233 | 1.00 | 26.01 | C |
| ATOM | 620 | C | ARG | A | 111 | 37.595 | 26.926 | 47.356 | 1.00 | 26.01 | C |
| ATOM | 621 | O | ARG | A | 111 | 38.528 | 27.438 | 47.987 | 1.00 | 26.01 | O |
| ATOM | 622 | CB | ARG | A | 111 | 37.753 | 27.709 | 44.968 | 1.00 | 74.46 | C |
| ATOM | 623 | CG | ARG | A | 111 | 37.449 | 26.661 | 43.902 | 1.00 | 74.46 | C |
| ATOM | 624 | CD | ARG | A | 111 | 38.176 | 27.020 | 42.592 | 1.00 | 74.46 | C |
| ATOM | 625 | NE | ARG | A | 111 | 39.614 | 27.206 | 42.804 | 1.00 | 74.46 | N |
| ATOM | 626 | CZ | ARG | A | 111 | 40.294 | 28.326 | 42.525 | 1.00 | 74.46 | C |
| ATOM | 627 | NH1 | ARG | A | 111 | 39.663 | 29.385 | 42.010 | 1.00 | 74.46 | N |
| ATOM | 628 | NH2 | ARG | A | 111 | 41.608 | 28.392 | 42.758 | 1.00 | 74.46 | N |
| ATOM | 629 | N | LEU | A | 112 | 37.131 | 25.708 | 47.615 | 1.00 | 28.45 | N |
| ATOM | 630 | CA | LEU | A | 112 | 37.757 | 24.857 | 48.619 | 1.00 | 28.45 | C |
| ATOM | 631 | C | LEU | A | 112 | 38.804 | 24.020 | 47.874 | 1.00 | 28.45 | C |
| ATOM | 632 | O | LEU | A | 112 | 38.483 | 22.965 | 47.337 | 1.00 | 28.45 | O |
| ATOM | 633 | CB | LEU | A | 112 | 36.735 | 23.916 | 49.267 | 1.00 | 23.28 | C |
| ATOM | 634 | CG | LEU | A | 112 | 37.352 | 22.977 | 50.319 | 1.00 | 23.28 | C |
| ATOM | 635 | CD1 | LEU | A | 112 | 37.639 | 23.768 | 51.586 | 1.00 | 23.28 | C |
| ATOM | 636 | CD2 | LEU | A | 112 | 36.425 | 21.797 | 50.624 | 1.00 | 23.28 | C |
| ATOM | 637 | N | ARG | A | 113 | 40.047 | 24.484 | 47.826 | 1.00 | 26.78 | N |
| ATOM | 638 | CA | ARG | A | 113 | 41.078 | 23.734 | 47.118 | 1.00 | 26.78 | C |
| ATOM | 639 | C | ARG | A | 113 | 41.241 | 22.325 | 47.701 | 1.00 | 26.78 | C |
| ATOM | 640 | O | ARG | A | 113 | 41.126 | 21.327 | 46.992 | 1.00 | 26.78 | O |
| ATOM | 641 | CB | ARG | A | 113 | 42.424 | 24.466 | 47.194 | 1.00 | 66.27 | C |
| ATOM | 642 | CG | ARG | A | 113 | 42.325 | 25.990 | 47.160 | 1.00 | 66.27 | C |
| ATOM | 643 | CD | ARG | A | 113 | 41.871 | 26.498 | 45.816 | 1.00 | 66.27 | C |
| ATOM | 644 | NE | ARG | A | 113 | 42.985 | 26.718 | 44.895 | 1.00 | 66.27 | N |
| ATOM | 645 | CZ | ARG | A | 113 | 43.812 | 27.764 | 44.951 | 1.00 | 66.27 | C |
| ATOM | 646 | NH1 | ARG | A | 113 | 43.646 | 28.686 | 45.893 | 1.00 | 66.27 | N |
| ATOM | 647 | NH2 | ARG | A | 113 | 44.794 | 27.900 | 44.055 | 1.00 | 66.27 | N |
| ATOM | 648 | N | TYR | A | 114 | 41.496 | 22.247 | 49.003 | 1.00 | 41.01 | N |
| ATOM | 649 | CA | TYR | A | 114 | 41.707 | 20.966 | 49.657 | 1.00 | 41.01 | C |
| ATOM | 650 | C | TYR | A | 114 | 41.279 | 21.086 | 51.099 | 1.00 | 41.01 | C |
| ATOM | 651 | O | TYR | A | 114 | 40.965 | 22.170 | 51.578 | 1.00 | 41.01 | O |
| ATOM | 652 | CB | TYR | A | 114 | 43.202 | 20.609 | 49.677 | 1.00 | 35.34 | C |
| ATOM | 653 | CG | TYR | A | 114 | 43.946 | 20.748 | 48.366 | 1.00 | 35.34 | C |
| ATOM | 654 | CD1 | TYR | A | 114 | 43.812 | 19.797 | 47.365 | 1.00 | 35.34 | C |
| ATOM | 655 | CD2 | TYR | A | 114 | 44.794 | 21.829 | 48.134 | 1.00 | 35.34 | C |
| ATOM | 656 | CE1 | TYR | A | 114 | 44.501 | 19.918 | 46.161 | 1.00 | 35.34 | C |
| ATOM | 657 | CE2 | TYR | A | 114 | 45.484 | 21.960 | 46.933 | 1.00 | 35.34 | C |
| ATOM | 658 | CZ | TYR | A | 114 | 45.331 | 20.998 | 45.954 | 1.00 | 35.34 | C |
| ATOM | 659 | OH | TYR | A | 114 | 45.995 | 21.115 | 44.757 | 1.00 | 35.34 | O |
| ATOM | 660 | N | PHE | A | 115 | 41.303 | 19.956 | 51.790 | 1.00 | 39.24 | N |
| ATOM | 661 | CA | PHE | A | 115 | 41.006 | 19.913 | 53.207 | 1.00 | 39.24 | C |
| ATOM | 662 | C | PHE | A | 115 | 41.721 | 18.696 | 53.810 | 1.00 | 39.24 | C |
| ATOM | 663 | O | PHE | A | 115 | 41.898 | 17.679 | 53.144 | 1.00 | 39.24 | O |
| ATOM | 664 | CB | PHE | A | 115 | 39.499 | 19.860 | 53.436 | 1.00 | 31.61 | C |
| ATOM | 665 | CG | PHE | A | 115 | 38.864 | 18.580 | 53.022 | 1.00 | 31.61 | C |
| ATOM | 666 | CD1 | PHE | A | 115 | 38.748 | 17.529 | 53.922 | 1.00 | 31.61 | C |
| ATOM | 667 | CD2 | PHE | A | 115 | 38.305 | 18.448 | 51.751 | 1.00 | 31.61 | C |
| ATOM | 668 | CE1 | PHE | A | 115 | 38.072 | 16.368 | 53.568 | 1.00 | 31.61 | C |
| ATOM | 669 | CE2 | PHE | A | 115 | 37.623 | 17.287 | 51.383 | 1.00 | 31.61 | C |
| ATOM | 670 | CZ | PHE | A | 115 | 37.504 | 16.249 | 52.294 | 1.00 | 31.61 | C |
| ATOM | 671 | N | PHE | A | 116 | 42.166 | 18.818 | 55.056 | 1.00 | 46.90 | N |
| ATOM | 672 | CA | PHE | A | 116 | 42.862 | 17.723 | 55.724 | 1.00 | 46.90 | C |
| ATOM | 673 | C | PHE | A | 116 | 42.779 | 17.895 | 57.232 | 1.00 | 46.90 | C |
| ATOM | 674 | O | PHE | A | 116 | 42.307 | 18.926 | 57.723 | 1.00 | 46.90 | O |
| ATOM | 675 | CB | PHE | A | 116 | 44.322 | 17.681 | 55.282 | 1.00 | 27.87 | C |
| ATOM | 676 | CG | PHE | A | 116 | 45.112 | 18.890 | 55.687 | 1.00 | 27.87 | C |
| ATOM | 677 | CD1 | PHE | A | 116 | 45.650 | 18.991 | 56.960 | 1.00 | 27.87 | C |
| ATOM | 678 | CD2 | PHE | A | 116 | 45.319 | 19.938 | 54.789 | 1.00 | 27.87 | C |

| | | | | | | | | | | |
|---|---|---|---|---|---|---|---|---|---|---|
| ATOM | 679 | CE1 | PHE | A | 116 | 46.382 | 20.123 | 57.337 | 1.00 | 27.87 | C |
| ATOM | 680 | CE2 | PHE | A | 116 | 46.050 | 21.072 | 55.159 | 1.00 | 27.87 | C |
| ATOM | 681 | CZ | PHE | A | 116 | 46.581 | 21.162 | 56.434 | 1.00 | 27.87 | C |
| ATOM | 682 | N | TYR | A | 117 | 43.253 | 16.888 | 57.961 | 1.00 | 64.42 | N |
| ATOM | 683 | CA | TYR | A | 117 | 43.221 | 16.908 | 59.421 | 1.00 | 64.42 | C |
| ATOM | 684 | C | TYR | A | 117 | 44.594 | 17.059 | 60.126 | 1.00 | 64.42 | C |
| ATOM | 685 | O | TYR | A | 117 | 45.635 | 16.612 | 59.613 | 1.00 | 64.42 | O |
| ATOM | 686 | CB | TYR | A | 117 | 42.489 | 15.652 | 59.895 | 1.00 | 44.40 | C |
| ATOM | 687 | CG | TYR | A | 117 | 41.058 | 15.583 | 59.375 | 1.00 | 44.40 | C |
| ATOM | 688 | CD1 | TYR | A | 117 | 40.078 | 16.473 | 59.838 | 1.00 | 44.40 | C |
| ATOM | 689 | CD2 | TYR | A | 117 | 40.686 | 14.648 | 58.404 | 1.00 | 44.40 | C |
| ATOM | 690 | CE1 | TYR | A | 117 | 38.766 | 16.428 | 59.350 | 1.00 | 44.40 | C |
| ATOM | 691 | CE2 | TYR | A | 117 | 39.381 | 14.597 | 57.911 | 1.00 | 44.40 | C |
| ATOM | 692 | CZ | TYR | A | 117 | 38.428 | 15.486 | 58.388 | 1.00 | 44.40 | C |
| ATOM | 693 | OH | TYR | A | 117 | 37.128 | 15.419 | 57.920 | 1.00 | 44.40 | O |
| ATOM | 694 | N | SER | A | 118 | 44.577 | 17.722 | 61.292 | 1.00 | 83.11 | N |
| ATOM | 695 | CA | SER | A | 118 | 45.773 | 17.972 | 62.124 | 1.00 | 83.11 | C |
| ATOM | 696 | C | SER | A | 118 | 45.413 | 17.716 | 63.583 | 1.00 | 83.11 | C |
| ATOM | 697 | O | SER | A | 118 | 44.267 | 17.959 | 63.999 | 1.00 | 83.11 | O |
| ATOM | 698 | CB | SER | A | 118 | 46.235 | 19.430 | 62.022 | 1.00 | 70.63 | C |
| ATOM | 699 | OG | SER | A | 118 | 46.412 | 19.833 | 60.677 | 1.00 | 70.63 | O |
| ATOM | 700 | N | SER | A | 119 | 46.395 | 17.260 | 64.361 | 1.00 | 68.91 | N |
| ATOM | 701 | CA | SER | A | 119 | 46.180 | 16.967 | 65.779 | 1.00 | 68.91 | C |
| ATOM | 702 | C | SER | A | 119 | 46.199 | 18.227 | 66.648 | 1.00 | 68.91 | C |
| ATOM | 703 | O | SER | A | 119 | 46.474 | 19.326 | 66.162 | 1.00 | 68.91 | O |
| ATOM | 704 | CB | SER | A | 119 | 47.248 | 15.987 | 66.269 | 1.00 | 69.37 | C |
| ATOM | 705 | OG | SER | A | 119 | 47.158 | 14.759 | 65.562 | 1.00 | 69.37 | O |
| ATOM | 706 | N | TYR | A | 127 | 42.467 | 20.925 | 64.527 | 1.00 | 61.36 | N |
| ATOM | 707 | CA | TYR | A | 127 | 41.976 | 19.596 | 64.149 | 1.00 | 61.36 | C |
| ATOM | 708 | C | TYR | A | 127 | 41.643 | 19.495 | 62.642 | 1.00 | 61.36 | C |
| ATOM | 709 | O | TYR | A | 127 | 42.286 | 18.728 | 61.919 | 1.00 | 61.36 | O |
| ATOM | 710 | CB | TYR | A | 127 | 40.746 | 19.214 | 65.007 | 1.00 | 37.80 | C |
| ATOM | 711 | N | LEU | A | 128 | 40.638 | 20.241 | 62.172 | 1.00 | 53.88 | N |
| ATOM | 712 | CA | LEU | A | 128 | 40.281 | 20.224 | 60.742 | 1.00 | 53.88 | C |
| ATOM | 713 | C | LEU | A | 128 | 40.810 | 21.467 | 60.042 | 1.00 | 53.88 | C |
| ATOM | 714 | O | LEU | A | 128 | 40.764 | 22.578 | 60.595 | 1.00 | 53.88 | O |
| ATOM | 715 | CB | LEU | A | 128 | 38.766 | 20.163 | 60.520 | 1.00 | 37.71 | C |
| ATOM | 716 | CG | LEU | A | 128 | 38.395 | 20.424 | 59.051 | 1.00 | 37.71 | C |
| ATOM | 717 | CD1 | LEU | A | 128 | 39.234 | 19.529 | 58.154 | 1.00 | 37.71 | C |
| ATOM | 718 | CD2 | LEU | A | 128 | 36.913 | 20.177 | 58.815 | 1.00 | 37.71 | C |
| ATOM | 719 | N | ASN | A | 129 | 41.285 | 21.278 | 58.815 | 1.00 | 50.03 | N |
| ATOM | 720 | CA | ASN | A | 129 | 41.849 | 22.374 | 58.043 | 1.00 | 50.03 | C |
| ATOM | 721 | C | ASN | A | 129 | 41.112 | 22.576 | 56.730 | 1.00 | 50.03 | C |
| ATOM | 722 | O | ASN | A | 129 | 40.812 | 21.605 | 56.037 | 1.00 | 50.03 | O |
| ATOM | 723 | CB | ASN | A | 129 | 43.324 | 22.087 | 57.754 | 1.00 | 36.05 | C |
| ATOM | 724 | CG | ASN | A | 129 | 44.147 | 21.895 | 59.029 | 1.00 | 36.05 | C |
| ATOM | 725 | OD1 | ASN | A | 129 | 44.813 | 22.818 | 59.504 | 1.00 | 36.05 | O |
| ATOM | 726 | ND2 | ASN | A | 129 | 44.094 | 20.688 | 59.591 | 1.00 | 36.05 | N |
| ATOM | 727 | N | LEU | A | 130 | 40.816 | 23.833 | 56.391 | 1.00 | 48.89 | N |
| ATOM | 728 | CA | LEU | A | 130 | 40.156 | 24.142 | 55.126 | 1.00 | 48.89 | C |
| ATOM | 729 | C | LEU | A | 130 | 41.036 | 25.048 | 54.254 | 1.00 | 48.89 | C |
| ATOM | 730 | O | LEU | A | 130 | 41.168 | 26.246 | 54.516 | 1.00 | 48.89 | O |
| ATOM | 731 | CB | LEU | A | 130 | 38.817 | 24.826 | 55.357 | 1.00 | 34.06 | C |
| ATOM | 732 | CG | LEU | A | 130 | 37.698 | 24.044 | 56.035 | 1.00 | 34.06 | C |
| ATOM | 733 | CD1 | LEU | A | 130 | 36.403 | 24.790 | 55.780 | 1.00 | 34.06 | C |
| ATOM | 734 | CD2 | LEU | A | 130 | 37.589 | 22.639 | 55.483 | 1.00 | 34.06 | C |
| ATOM | 735 | N | VAL | A | 131 | 41.643 | 24.475 | 53.222 | 1.00 | 33.05 | N |
| ATOM | 736 | CA | VAL | A | 131 | 42.490 | 25.249 | 52.331 | 1.00 | 33.05 | C |
| ATOM | 737 | C | VAL | A | 131 | 41.584 | 25.914 | 51.304 | 1.00 | 33.05 | C |
| ATOM | 738 | O | VAL | A | 131 | 41.027 | 25.238 | 50.435 | 1.00 | 33.05 | O |
| ATOM | 739 | CB | VAL | A | 131 | 43.494 | 24.344 | 51.612 | 1.00 | 25.51 | C |
| ATOM | 740 | CG1 | VAL | A | 131 | 44.532 | 25.182 | 50.913 | 1.00 | 25.51 | C |

| | | | | | | | | | | | |
|---|---|---|---|---|---|---|---|---|---|---|---|
| ATOM | 741 | CG2 | VAL | A | 131 | 44.147 | 23.413 | 52.607 | 1.00 | 25.51 | C |
| ATOM | 742 | N | LEU | A | 132 | 41.444 | 27.237 | 51.409 | 1.00 | 37.71 | N |
| ATOM | 743 | CA | LEU | A | 132 | 40.580 | 28.020 | 50.515 | 1.00 | 37.71 | C |
| ATOM | 744 | C | LEU | A | 132 | 41.344 | 29.014 | 49.659 | 1.00 | 37.71 | C |
| ATOM | 745 | O | LEU | A | 132 | 42.514 | 29.292 | 49.928 | 1.00 | 37.71 | O |
| ATOM | 746 | CB | LEU | A | 132 | 39.573 | 28.793 | 51.348 | 1.00 | 23.63 | C |
| ATOM | 747 | CG | LEU | A | 132 | 38.726 | 27.947 | 52.286 | 1.00 | 23.63 | C |
| ATOM | 748 | CD1 | LEU | A | 132 | 38.515 | 28.697 | 53.579 | 1.00 | 23.63 | C |
| ATOM | 749 | CD2 | LEU | A | 132 | 37.412 | 27.613 | 51.614 | 1.00 | 23.63 | C |
| ATOM | 750 | N | ASP | A | 133 | 40.688 | 29.546 | 48.625 | 1.00 | 40.09 | N |
| ATOM | 751 | CA | ASP | A | 133 | 41.332 | 30.550 | 47.780 | 1.00 | 40.09 | C |
| ATOM | 752 | C | ASP | A | 133 | 41.609 | 31.722 | 48.713 | 1.00 | 40.09 | C |
| ATOM | 753 | O | ASP | A | 133 | 40.865 | 31.951 | 49.670 | 1.00 | 40.09 | O |
| ATOM | 754 | CB | ASP | A | 133 | 40.403 | 31.071 | 46.674 | 1.00 | 52.78 | C |
| ATOM | 755 | CG | ASP | A | 133 | 40.311 | 30.141 | 45.466 | 1.00 | 52.78 | C |
| ATOM | 756 | OD1 | ASP | A | 133 | 41.310 | 29.447 | 45.156 | 1.00 | 52.78 | O |
| ATOM | 757 | OD2 | ASP | A | 133 | 39.235 | 30.137 | 44.808 | 1.00 | 52.78 | O |
| ATOM | 758 | N | TYR | A | 134 | 42.672 | 32.467 | 48.447 | 1.00 | 51.56 | N |
| ATOM | 759 | CA | TYR | A | 134 | 42.970 | 33.623 | 49.279 | 1.00 | 51.56 | C |
| ATOM | 760 | C | TYR | A | 134 | 42.667 | 34.884 | 48.504 | 1.00 | 51.56 | C |
| ATOM | 761 | O | TYR | A | 134 | 43.071 | 35.021 | 47.350 | 1.00 | 51.56 | O |
| ATOM | 762 | CB | TYR | A | 134 | 44.426 | 33.656 | 49.670 | 1.00 | 64.72 | C |
| ATOM | 763 | CG | TYR | A | 134 | 44.760 | 34.885 | 50.461 | 1.00 | 64.72 | C |
| ATOM | 764 | CD1 | TYR | A | 134 | 44.292 | 35.040 | 51.766 | 1.00 | 64.72 | C |
| ATOM | 765 | CD2 | TYR | A | 134 | 45.574 | 35.884 | 49.920 | 1.00 | 64.72 | C |
| ATOM | 766 | CE1 | TYR | A | 134 | 44.633 | 36.157 | 52.526 | 1.00 | 64.72 | C |
| ATOM | 767 | CE2 | TYR | A | 134 | 45.923 | 37.004 | 50.665 | 1.00 | 64.72 | C |
| ATOM | 768 | CZ | TYR | A | 134 | 45.454 | 37.133 | 51.970 | 1.00 | 64.72 | C |
| ATOM | 769 | OH | TYR | A | 134 | 45.835 | 38.228 | 52.721 | 1.00 | 64.72 | O |
| ATOM | 770 | N | VAL | A | 135 | 41.961 | 35.807 | 49.139 | 1.00 | 34.95 | N |
| ATOM | 771 | CA | VAL | A | 135 | 41.602 | 37.059 | 48.492 | 1.00 | 34.95 | C |
| ATOM | 772 | C | VAL | A | 135 | 41.826 | 38.179 | 49.505 | 1.00 | 34.95 | C |
| ATOM | 773 | O | VAL | A | 135 | 41.124 | 38.293 | 50.511 | 1.00 | 34.95 | O |
| ATOM | 774 | CB | VAL | A | 135 | 40.147 | 37.012 | 48.026 | 1.00 | 27.14 | C |
| ATOM | 775 | CG1 | VAL | A | 135 | 39.836 | 38.199 | 47.135 | 1.00 | 27.14 | C |
| ATOM | 776 | CG2 | VAL | A | 135 | 39.900 | 35.705 | 47.296 | 1.00 | 27.14 | C |
| ATOM | 777 | N | PRO | A | 136 | 42.811 | 39.035 | 49.227 | 1.00 | 55.48 | N |
| ATOM | 778 | CA | PRO | A | 136 | 43.225 | 40.170 | 50.051 | 1.00 | 55.48 | C |
| ATOM | 779 | C | PRO | A | 136 | 42.168 | 41.217 | 50.396 | 1.00 | 55.48 | C |
| ATOM | 780 | O | PRO | A | 136 | 41.998 | 41.572 | 51.567 | 1.00 | 55.48 | O |
| ATOM | 781 | CB | PRO | A | 136 | 44.374 | 40.757 | 49.244 | 1.00 | 53.53 | C |
| ATOM | 782 | CG | PRO | A | 136 | 43.874 | 40.604 | 47.848 | 1.00 | 53.53 | C |
| ATOM | 783 | CD | PRO | A | 136 | 43.341 | 39.177 | 47.856 | 1.00 | 53.53 | C |
| ATOM | 784 | N | GLU | A | 137 | 41.453 | 41.709 | 49.393 | 1.00 | 27.12 | N |
| ATOM | 785 | CA | GLU | A | 137 | 40.466 | 42.749 | 49.639 | 1.00 | 27.12 | C |
| ATOM | 786 | C | GLU | A | 137 | 39.053 | 42.221 | 49.879 | 1.00 | 27.12 | C |
| ATOM | 787 | O | GLU | A | 137 | 38.720 | 41.120 | 49.477 | 1.00 | 27.12 | O |
| ATOM | 788 | CB | GLU | A | 137 | 40.490 | 43.740 | 48.471 | 1.00 | 54.17 | C |
| ATOM | 789 | CG | GLU | A | 137 | 40.158 | 45.159 | 48.865 | 1.00 | 54.17 | C |
| ATOM | 790 | CD | GLU | A | 137 | 41.004 | 45.651 | 50.032 | 1.00 | 54.17 | C |
| ATOM | 791 | OE1 | GLU | A | 137 | 42.107 | 46.212 | 49.812 | 1.00 | 54.17 | O |
| ATOM | 792 | OE2 | GLU | A | 137 | 40.565 | 45.460 | 51.186 | 1.00 | 54.17 | O |
| ATOM | 793 | N | THR | A | 138 | 38.234 | 43.020 | 50.553 | 1.00 | 24.91 | N |
| ATOM | 794 | CA | THR | A | 138 | 36.852 | 42.664 | 50.860 | 1.00 | 24.91 | C |
| ATOM | 795 | C | THR | A | 138 | 35.984 | 43.887 | 50.619 | 1.00 | 24.91 | C |
| ATOM | 796 | O | THR | A | 138 | 36.472 | 45.004 | 50.614 | 1.00 | 24.91 | O |
| ATOM | 797 | CB | THR | A | 138 | 36.680 | 42.259 | 52.330 | 1.00 | 31.52 | C |
| ATOM | 798 | OG1 | THR | A | 138 | 36.902 | 43.400 | 53.169 | 1.00 | 31.52 | O |
| ATOM | 799 | CG2 | THR | A | 138 | 37.663 | 41.168 | 52.699 | 1.00 | 31.52 | C |
| ATOM | 800 | N | VAL | A | 139 | 34.691 | 43.685 | 50.424 | 1.00 | 27.42 | N |
| ATOM | 801 | CA | VAL | A | 139 | 33.813 | 44.812 | 50.186 | 1.00 | 27.42 | C |
| ATOM | 802 | C | VAL | A | 139 | 33.867 | 45.755 | 51.373 | 1.00 | 27.42 | C |

| | | | | | | | | | | | |
|---|---|---|---|---|---|---|---|---|---|---|---|
| ATOM | 803 | O | VAL | A | 139 | 33.842 | 46.969 | 51.219 | 1.00 | 27.42 | O |
| ATOM | 804 | CB | VAL | A | 139 | 32.367 | 44.351 | 49.949 | 1.00 | 26.39 | C |
| ATOM | 805 | CG1 | VAL | A | 139 | 31.405 | 45.536 | 50.103 | 1.00 | 26.39 | C |
| ATOM | 806 | CG2 | VAL | A | 139 | 32.248 | 43.748 | 48.548 | 1.00 | 26.39 | C |
| ATOM | 807 | N | TYR | A | 140 | 33.953 | 45.179 | 52.560 | 1.00 | 28.15 | N |
| ATOM | 808 | CA | TYR | A | 140 | 34.024 | 45.952 | 53.775 | 1.00 | 28.15 | C |
| ATOM | 809 | C | TYR | A | 140 | 35.190 | 46.941 | 53.684 | 1.00 | 28.15 | C |
| ATOM | 810 | O | TYR | A | 140 | 34.999 | 48.153 | 53.763 | 1.00 | 28.15 | O |
| ATOM | 811 | CB | TYR | A | 140 | 34.222 | 45.015 | 54.963 | 1.00 | 29.23 | C |
| ATOM | 812 | CG | TYR | A | 140 | 34.441 | 45.736 | 56.267 | 1.00 | 29.23 | C |
| ATOM | 813 | CD1 | TYR | A | 140 | 33.378 | 46.326 | 56.949 | 1.00 | 29.23 | C |
| ATOM | 814 | CD2 | TYR | A | 140 | 35.718 | 45.860 | 56.803 | 1.00 | 29.23 | C |
| ATOM | 815 | CE1 | TYR | A | 140 | 33.584 | 47.030 | 58.133 | 1.00 | 29.23 | C |
| ATOM | 816 | CE2 | TYR | A | 140 | 35.937 | 46.557 | 57.980 | 1.00 | 29.23 | C |
| ATOM | 817 | CZ | TYR | A | 140 | 34.870 | 47.140 | 58.641 | 1.00 | 29.23 | C |
| ATOM | 818 | OH | TYR | A | 140 | 35.096 | 47.852 | 59.792 | 1.00 | 29.23 | O |
| ATOM | 819 | N | ARG | A | 141 | 36.399 | 46.420 | 53.509 | 1.00 | 29.92 | N |
| ATOM | 820 | CA | ARG | A | 141 | 37.571 | 47.273 | 53.414 | 1.00 | 29.92 | C |
| ATOM | 821 | C | ARG | A | 141 | 37.448 | 48.355 | 52.349 | 1.00 | 29.92 | C |
| ATOM | 822 | O | ARG | A | 141 | 37.799 | 49.504 | 52.582 | 1.00 | 29.92 | O |
| ATOM | 823 | CB | ARG | A | 141 | 38.809 | 46.430 | 53.152 | 1.00 | 61.43 | C |
| ATOM | 824 | CG | ARG | A | 141 | 39.293 | 45.649 | 54.372 | 1.00 | 61.43 | C |
| ATOM | 825 | CD | ARG | A | 141 | 40.677 | 45.065 | 54.119 | 1.00 | 61.43 | C |
| ATOM | 826 | NE | ARG | A | 141 | 41.506 | 46.012 | 53.372 | 1.00 | 61.43 | N |
| ATOM | 827 | CZ | ARG | A | 141 | 42.714 | 45.731 | 52.899 | 1.00 | 61.43 | C |
| ATOM | 828 | NH1 | ARG | A | 141 | 43.243 | 44.527 | 53.109 | 1.00 | 61.43 | N |
| ATOM | 829 | NH2 | ARG | A | 141 | 43.374 | 46.637 | 52.179 | 1.00 | 61.43 | N |
| ATOM | 830 | N | VAL | A | 142 | 36.950 | 47.995 | 51.175 | 1.00 | 36.65 | N |
| ATOM | 831 | CA | VAL | A | 142 | 36.799 | 48.980 | 50.117 | 1.00 | 36.65 | C |
| ATOM | 832 | C | VAL | A | 142 | 35.778 | 50.036 | 50.521 | 1.00 | 36.65 | C |
| ATOM | 833 | O | VAL | A | 142 | 36.065 | 51.228 | 50.480 | 1.00 | 36.65 | O |
| ATOM | 834 | CB | VAL | A | 142 | 36.356 | 48.327 | 48.796 | 1.00 | 25.48 | C |
| ATOM | 835 | CG1 | VAL | A | 142 | 36.110 | 49.380 | 47.754 | 1.00 | 25.48 | C |
| ATOM | 836 | CG2 | VAL | A | 142 | 37.416 | 47.370 | 48.314 | 1.00 | 25.48 | C |
| ATOM | 837 | N | ALA | A | 143 | 34.591 | 49.609 | 50.931 | 1.00 | 26.42 | N |
| ATOM | 838 | CA | ALA | A | 143 | 33.563 | 50.570 | 51.320 | 1.00 | 26.42 | C |
| ATOM | 839 | C | ALA | A | 143 | 34.039 | 51.495 | 52.431 | 1.00 | 26.42 | C |
| ATOM | 840 | O | ALA | A | 143 | 33.655 | 52.657 | 52.470 | 1.00 | 26.42 | O |
| ATOM | 841 | CB | ALA | A | 143 | 32.284 | 49.842 | 51.756 | 1.00 | 31.94 | C |
| ATOM | 842 | N | ARG | A | 144 | 34.876 | 50.969 | 53.319 | 1.00 | 30.25 | N |
| ATOM | 843 | CA | ARG | A | 144 | 35.403 | 51.724 | 54.456 | 1.00 | 30.25 | C |
| ATOM | 844 | C | ARG | A | 144 | 36.392 | 52.783 | 53.983 | 1.00 | 30.25 | C |
| ATOM | 845 | O | ARG | A | 144 | 36.352 | 53.925 | 54.433 | 1.00 | 30.25 | O |
| ATOM | 846 | CB | ARG | A | 144 | 36.091 | 50.768 | 55.440 | 1.00 | 46.52 | C |
| ATOM | 847 | CG | ARG | A | 144 | 36.504 | 51.391 | 56.758 | 1.00 | 46.52 | C |
| ATOM | 848 | CD | ARG | A | 144 | 37.357 | 50.429 | 57.593 | 1.00 | 46.52 | C |
| ATOM | 849 | NE | ARG | A | 144 | 37.502 | 50.867 | 58.978 | 0.00 | 46.52 | N |
| ATOM | 850 | CZ | ARG | A | 144 | 36.493 | 50.966 | 59.838 | 0.00 | 46.52 | C |
| ATOM | 851 | NH1 | ARG | A | 144 | 35.261 | 50.659 | 59.454 | 0.00 | 46.52 | N |
| ATOM | 852 | NH2 | ARG | A | 144 | 36.712 | 51.368 | 61.082 | 0.00 | 46.52 | N |
| ATOM | 853 | N | HIS | A | 145 | 37.278 | 52.387 | 53.075 | 1.00 | 35.85 | N |
| ATOM | 854 | CA | HIS | A | 145 | 38.276 | 53.283 | 52.508 | 1.00 | 35.85 | C |
| ATOM | 855 | C | HIS | A | 145 | 37.592 | 54.547 | 51.972 | 1.00 | 35.85 | C |
| ATOM | 856 | O | HIS | A | 145 | 37.940 | 55.670 | 52.351 | 1.00 | 35.85 | O |
| ATOM | 857 | CB | HIS | A | 145 | 39.003 | 52.573 | 51.369 | 1.00 | 46.47 | C |
| ATOM | 858 | CG | HIS | A | 145 | 40.063 | 53.399 | 50.714 | 1.00 | 46.47 | C |
| ATOM | 859 | ND1 | HIS | A | 145 | 41.407 | 53.205 | 50.954 | 1.00 | 46.47 | N |
| ATOM | 860 | CD2 | HIS | A | 145 | 39.981 | 54.421 | 49.828 | 1.00 | 46.47 | C |
| ATOM | 861 | CE1 | HIS | A | 145 | 42.107 | 54.071 | 50.238 | 1.00 | 46.47 | C |
| ATOM | 862 | NE2 | HIS | A | 145 | 41.268 | 54.821 | 49.546 | 1.00 | 46.47 | N |
| ATOM | 863 | N | TYR | A | 146 | 36.612 | 54.354 | 51.093 | 1.00 | 35.25 | N |
| ATOM | 864 | CA | TYR | A | 146 | 35.884 | 55.466 | 50.502 | 1.00 | 35.25 | C |

| | | | | | | | | | | | |
|---|---|---|---|---|---|---|---|---|---|---|---|
| ATOM | 865 | C | TYR | A | 146 | 35.126 | 56.252 | 51.539 | 1.00 | 35.25 | C |
| ATOM | 866 | O | TYR | A | 146 | 35.114 | 57.483 | 51.512 | 1.00 | 35.25 | O |
| ATOM | 867 | CB | TYR | A | 146 | 34.902 | 54.968 | 49.437 | 1.00 | 26.45 | C |
| ATOM | 868 | CG | TYR | A | 146 | 35.576 | 54.507 | 48.172 | 1.00 | 26.45 | C |
| ATOM | 869 | CD1 | TYR | A | 146 | 36.298 | 53.322 | 48.142 | 1.00 | 26.45 | C |
| ATOM | 870 | CD2 | TYR | A | 146 | 35.559 | 55.302 | 47.022 | 1.00 | 26.45 | C |
| ATOM | 871 | CE1 | TYR | A | 146 | 36.989 | 52.937 | 47.007 | 1.00 | 26.45 | C |
| ATOM | 872 | CE2 | TYR | A | 146 | 36.248 | 54.929 | 45.879 | 1.00 | 26.45 | C |
| ATOM | 873 | CZ | TYR | A | 146 | 36.967 | 53.745 | 45.877 | 1.00 | 26.45 | C |
| ATOM | 874 | OH | TYR | A | 146 | 37.682 | 53.381 | 44.753 | 1.00 | 26.45 | O |
| ATOM | 875 | N | SER | A | 147 | 34.503 | 55.543 | 52.468 | 1.00 | 38.55 | N |
| ATOM | 876 | CA | SER | A | 147 | 33.714 | 56.205 | 53.491 | 1.00 | 38.55 | C |
| ATOM | 877 | C | SER | A | 147 | 34.547 | 57.133 | 54.363 | 1.00 | 38.55 | C |
| ATOM | 878 | O | SER | A | 147 | 34.071 | 58.191 | 54.786 | 1.00 | 38.55 | O |
| ATOM | 879 | CB | SER | A | 147 | 33.006 | 55.173 | 54.367 | 1.00 | 46.88 | C |
| ATOM | 880 | OG | SER | A | 147 | 31.981 | 55.793 | 55.126 | 1.00 | 46.88 | O |
| ATOM | 881 | N | ARG | A | 148 | 35.787 | 56.736 | 54.631 | 1.00 | 44.27 | N |
| ATOM | 882 | CA | ARG | A | 148 | 36.666 | 57.544 | 55.453 | 1.00 | 44.27 | C |
| ATOM | 883 | C | ARG | A | 148 | 37.076 | 58.789 | 54.673 | 1.00 | 44.27 | C |
| ATOM | 884 | O | ARG | A | 148 | 37.144 | 59.886 | 55.233 | 1.00 | 44.27 | O |
| ATOM | 885 | CB | ARG | A | 148 | 37.913 | 56.750 | 55.849 | 1.00 | 40.43 | C |
| ATOM | 886 | CG | ARG | A | 148 | 37.665 | 55.525 | 56.728 | 1.00 | 40.43 | C |
| ATOM | 887 | CD | ARG | A | 148 | 36.858 | 55.872 | 57.962 | 1.00 | 40.43 | C |
| ATOM | 888 | NE | ARG | A | 148 | 36.949 | 54.835 | 58.984 | 0.00 | 40.43 | N |
| ATOM | 889 | CZ | ARG | A | 148 | 38.075 | 54.498 | 59.604 | 0.00 | 40.43 | C |
| ATOM | 890 | NH1 | ARG | A | 148 | 39.210 | 55.116 | 59.306 | 0.00 | 40.43 | N |
| ATOM | 891 | NH2 | ARG | A | 148 | 38.067 | 53.547 | 60.526 | 0.00 | 40.43 | N |
| ATOM | 892 | N | ALA | A | 149 | 37.341 | 58.618 | 53.381 | 1.00 | 38.37 | N |
| ATOM | 893 | CA | ALA | A | 149 | 37.752 | 59.729 | 52.530 | 1.00 | 38.37 | C |
| ATOM | 894 | C | ALA | A | 149 | 36.546 | 60.558 | 52.092 | 1.00 | 38.37 | C |
| ATOM | 895 | O | ALA | A | 149 | 36.610 | 61.293 | 51.104 | 1.00 | 38.37 | O |
| ATOM | 896 | CB | ALA | A | 149 | 38.507 | 59.199 | 51.299 | 1.00 | 20.40 | C |
| ATOM | 897 | N | LYS | A | 150 | 35.443 | 60.440 | 52.825 | 1.00 | 54.55 | N |
| ATOM | 898 | CA | LYS | A | 150 | 34.233 | 61.183 | 52.487 | 1.00 | 54.55 | C |
| ATOM | 899 | C | LYS | A | 150 | 33.969 | 61.123 | 50.977 | 1.00 | 54.55 | C |
| ATOM | 900 | O | LYS | A | 150 | 33.400 | 62.049 | 50.384 | 1.00 | 54.55 | O |
| ATOM | 901 | CB | LYS | A | 150 | 34.352 | 62.643 | 52.948 | 1.00 | 59.91 | C |
| ATOM | 902 | CG | LYS | A | 150 | 34.441 | 62.820 | 54.464 | 1.00 | 59.91 | C |
| ATOM | 903 | CD | LYS | A | 150 | 34.362 | 64.295 | 54.853 | 0.00 | 59.91 | C |
| ATOM | 904 | CE | LYS | A | 150 | 33.016 | 64.902 | 54.479 | 1.00 | 59.91 | C |
| ATOM | 905 | NZ | LYS | A | 150 | 32.906 | 66.353 | 54.862 | 1.00 | 59.91 | N |
| ATOM | 906 | N | GLN | A | 151 | 34.399 | 60.028 | 50.359 | 1.00 | 44.44 | N |
| ATOM | 907 | CA | GLN | A | 151 | 34.182 | 59.827 | 48.930 | 1.00 | 44.44 | C |
| ATOM | 908 | C | GLN | A | 151 | 33.140 | 58.722 | 48.763 | 1.00 | 44.44 | C |
| ATOM | 909 | O | GLN | A | 151 | 32.525 | 58.267 | 49.745 | 1.00 | 44.44 | O |
| ATOM | 910 | CB | GLN | A | 151 | 35.486 | 59.416 | 48.254 | 1.00 | 51.73 | C |
| ATOM | 911 | CG | GLN | A | 151 | 36.625 | 60.380 | 48.515 | 1.00 | 51.73 | C |
| ATOM | 912 | CD | GLN | A | 151 | 37.972 | 59.838 | 48.063 | 1.00 | 51.73 | C |
| ATOM | 913 | OE1 | GLN | A | 151 | 39.001 | 60.500 | 48.210 | 1.00 | 51.73 | O |
| ATOM | 914 | NE2 | GLN | A | 151 | 37.973 | 58.627 | 47.509 | 1.00 | 51.73 | N |
| ATOM | 915 | N | THR | A | 152 | 32.917 | 58.302 | 47.524 | 1.00 | 51.39 | N |
| ATOM | 916 | CA | THR | A | 152 | 31.959 | 57.231 | 47.287 | 1.00 | 51.39 | C |
| ATOM | 917 | C | THR | A | 152 | 32.396 | 56.366 | 46.136 | 1.00 | 51.39 | C |
| ATOM | 918 | O | THR | A | 152 | 33.016 | 56.830 | 45.167 | 1.00 | 51.39 | O |
| ATOM | 919 | CB | THR | A | 152 | 30.537 | 57.748 | 46.996 | 1.00 | 72.10 | C |
| ATOM | 920 | OG1 | THR | A | 152 | 29.597 | 56.749 | 47.405 | 1.00 | 72.10 | O |
| ATOM | 921 | CG2 | THR | A | 152 | 30.340 | 58.016 | 45.511 | 0.00 | 72.10 | C |
| ATOM | 922 | N | LEU | A | 153 | 32.065 | 55.092 | 46.259 | 1.00 | 40.52 | N |
| ATOM | 923 | CA | LEU | A | 153 | 32.414 | 54.110 | 45.255 | 1.00 | 40.52 | C |
| ATOM | 924 | C | LEU | A | 153 | 31.584 | 54.303 | 43.985 | 1.00 | 40.52 | C |
| ATOM | 925 | O | LEU | A | 153 | 30.374 | 54.516 | 44.050 | 1.00 | 40.52 | O |
| ATOM | 926 | CB | LEU | A | 153 | 32.164 | 52.717 | 45.825 | 1.00 | 33.52 | C |

| | | | | | | | | | | | |
|---|---|---|---|---|---|---|---|---|---|---|---|
| ATOM | 927 | CG | LEU | A | 153 | 32.729 | 51.570 | 45.003 | 1.00 | 33.52 | C |
| ATOM | 928 | CD1 | LEU | A | 153 | 34.246 | 51.536 | 45.166 | 1.00 | 33.52 | C |
| ATOM | 929 | CD2 | LEU | A | 153 | 32.091 | 50.270 | 45.455 | 1.00 | 33.52 | C |
| ATOM | 930 | N | PRO | A | 154 | 32.234 | 54.271 | 42.816 | 1.00 | 28.10 | N |
| ATOM | 931 | CA | PRO | A | 154 | 31.528 | 54.428 | 41.544 | 1.00 | 28.10 | C |
| ATOM | 932 | C | PRO | A | 154 | 30.486 | 53.315 | 41.418 | 1.00 | 28.10 | C |
| ATOM | 933 | O | PRO | A | 154 | 30.808 | 52.139 | 41.565 | 1.00 | 28.10 | O |
| ATOM | 934 | CB | PRO | A | 154 | 32.640 | 54.281 | 40.517 | 1.00 | 34.70 | C |
| ATOM | 935 | CG | PRO | A | 154 | 33.802 | 54.894 | 41.217 | 1.00 | 34.70 | C |
| ATOM | 936 | CD | PRO | A | 154 | 33.690 | 54.357 | 42.622 | 1.00 | 34.70 | C |
| ATOM | 937 | N | VAL | A | 155 | 29.246 | 53.711 | 41.152 | 1.00 | 35.03 | N |
| ATOM | 938 | CA | VAL | A | 155 | 28.123 | 52.801 | 41.013 | 1.00 | 35.03 | C |
| ATOM | 939 | C | VAL | A | 155 | 28.469 | 51.573 | 40.186 | 1.00 | 35.03 | C |
| ATOM | 940 | O | VAL | A | 155 | 27.975 | 50.482 | 40.460 | 1.00 | 35.03 | O |
| ATOM | 941 | CB | VAL | A | 155 | 26.908 | 53.515 | 40.376 | 1.00 | 22.64 | C |
| ATOM | 942 | CG1 | VAL | A | 155 | 26.464 | 54.670 | 41.258 | 0.00 | 22.64 | C |
| ATOM | 943 | CG2 | VAL | A | 155 | 27.268 | 54.018 | 38.986 | 0.00 | 22.64 | C |
| ATOM | 944 | N | ILE | A | 156 | 29.316 | 51.739 | 39.177 | 1.00 | 27.63 | N |
| ATOM | 945 | CA | ILE | A | 156 | 29.687 | 50.602 | 38.349 | 1.00 | 27.63 | C |
| ATOM | 946 | C | ILE | A | 156 | 30.146 | 49.446 | 39.244 | 1.00 | 27.63 | C |
| ATOM | 947 | O | ILE | A | 156 | 29.787 | 48.293 | 39.002 | 1.00 | 27.63 | O |
| ATOM | 948 | CB | ILE | A | 156 | 30.803 | 50.980 | 37.353 | 1.00 | 24.51 | C |
| ATOM | 949 | CG1 | ILE | A | 156 | 31.297 | 49.743 | 36.600 | 1.00 | 24.51 | C |
| ATOM | 950 | CG2 | ILE | A | 156 | 31.940 | 51.625 | 38.089 | 1.00 | 24.51 | C |
| ATOM | 951 | CD1 | ILE | A | 156 | 30.333 | 49.197 | 35.573 | 1.00 | 24.51 | C |
| ATOM | 952 | N | TYR | A | 157 | 30.931 | 49.748 | 40.277 | 1.00 | 23.27 | N |
| ATOM | 953 | CA | TYR | A | 157 | 31.398 | 48.706 | 41.188 | 1.00 | 23.27 | C |
| ATOM | 954 | C | TYR | A | 157 | 30.239 | 48.215 | 42.024 | 1.00 | 23.27 | C |
| ATOM | 955 | O | TYR | A | 157 | 30.139 | 47.035 | 42.321 | 1.00 | 23.27 | O |
| ATOM | 956 | CB | TYR | A | 157 | 32.495 | 49.223 | 42.129 | 1.00 | 37.48 | C |
| ATOM | 957 | CG | TYR | A | 157 | 33.819 | 49.461 | 41.453 | 1.00 | 37.48 | C |
| ATOM | 958 | CD1 | TYR | A | 157 | 34.418 | 50.722 | 41.469 | 1.00 | 37.48 | C |
| ATOM | 959 | CD2 | TYR | A | 157 | 34.452 | 48.437 | 40.746 | 1.00 | 37.48 | C |
| ATOM | 960 | CE1 | TYR | A | 157 | 35.609 | 50.955 | 40.786 | 1.00 | 37.48 | C |
| ATOM | 961 | CE2 | TYR | A | 157 | 35.630 | 48.660 | 40.061 | 1.00 | 37.48 | C |
| ATOM | 962 | CZ | TYR | A | 157 | 36.203 | 49.917 | 40.086 | 1.00 | 37.48 | C |
| ATOM | 963 | OH | TYR | A | 157 | 37.373 | 50.127 | 39.405 | 1.00 | 37.48 | O |
| ATOM | 964 | N | VAL | A | 158 | 29.367 | 49.134 | 42.410 | 1.00 | 25.33 | N |
| ATOM | 965 | CA | VAL | A | 158 | 28.224 | 48.776 | 43.223 | 1.00 | 25.33 | C |
| ATOM | 966 | C | VAL | A | 158 | 27.443 | 47.711 | 42.464 | 1.00 | 25.33 | C |
| ATOM | 967 | O | VAL | A | 158 | 27.139 | 46.645 | 42.999 | 1.00 | 25.33 | O |
| ATOM | 968 | CB | VAL | A | 158 | 27.339 | 50.026 | 43.506 | 1.00 | 23.12 | C |
| ATOM | 969 | CG1 | VAL | A | 158 | 26.188 | 49.671 | 44.453 | 1.00 | 23.12 | C |
| ATOM | 970 | CG2 | VAL | A | 158 | 28.189 | 51.125 | 44.117 | 1.00 | 23.12 | C |
| ATOM | 971 | N | LYS | A | 159 | 27.152 | 48.007 | 41.201 | 1.00 | 26.41 | N |
| ATOM | 972 | CA | LYS | A | 159 | 26.416 | 47.106 | 40.330 | 1.00 | 26.41 | C |
| ATOM | 973 | C | LYS | A | 159 | 27.164 | 45.796 | 40.177 | 1.00 | 26.41 | C |
| ATOM | 974 | O | LYS | A | 159 | 26.597 | 44.725 | 40.358 | 1.00 | 26.41 | O |
| ATOM | 975 | CB | LYS | A | 159 | 26.217 | 47.755 | 38.962 | 1.00 | 30.79 | C |
| ATOM | 976 | CG | LYS | A | 159 | 25.480 | 49.092 | 39.010 | 1.00 | 30.79 | C |
| ATOM | 977 | CD | LYS | A | 159 | 25.282 | 49.655 | 37.617 | 1.00 | 30.79 | C |
| ATOM | 978 | CE | LYS | A | 159 | 24.500 | 50.953 | 37.622 | 1.00 | 30.79 | C |
| ATOM | 979 | NZ | LYS | A | 159 | 24.241 | 51.420 | 36.226 | 1.00 | 30.79 | N |
| ATOM | 980 | N | LEU | A | 160 | 28.442 | 45.886 | 39.843 | 1.00 | 28.98 | N |
| ATOM | 981 | CA | LEU | A | 160 | 29.267 | 44.700 | 39.682 | 1.00 | 28.98 | C |
| ATOM | 982 | C | LEU | A | 160 | 29.241 | 43.802 | 40.903 | 1.00 | 28.98 | C |
| ATOM | 983 | O | LEU | A | 160 | 28.957 | 42.613 | 40.802 | 1.00 | 28.98 | O |
| ATOM | 984 | CB | LEU | A | 160 | 30.718 | 45.090 | 39.398 | 1.00 | 27.95 | C |
| ATOM | 985 | CG | LEU | A | 160 | 31.101 | 45.332 | 37.941 | 1.00 | 27.95 | C |
| ATOM | 986 | CD1 | LEU | A | 160 | 32.508 | 45.865 | 37.869 | 1.00 | 27.95 | C |
| ATOM | 987 | CD2 | LEU | A | 160 | 30.988 | 44.032 | 37.160 | 1.00 | 27.95 | C |
| ATOM | 988 | N | TYR | A | 161 | 29.544 | 44.373 | 42.061 | 1.00 | 30.87 | N |

| | | | | | | | | | | | |
|---|---|---|---|---|---|---|---|---|---|---|---|
| ATOM | 989 | CA | TYR | A | 161 | 29.591 | 43.597 | 43.289 | 1.00 | 30.87 | C |
| ATOM | 990 | C | TYR | A | 161 | 28.241 | 43.008 | 43.680 | 1.00 | 30.87 | C |
| ATOM | 991 | O | TYR | A | 161 | 28.121 | 41.794 | 43.895 | 1.00 | 30.87 | O |
| ATOM | 992 | CB | TYR | A | 161 | 30.120 | 44.447 | 44.452 | 1.00 | 30.35 | C |
| ATOM | 993 | CG | TYR | A | 161 | 31.512 | 45.025 | 44.293 | 1.00 | 30.35 | C |
| ATOM | 994 | CD1 | TYR | A | 161 | 32.444 | 44.456 | 43.432 | 1.00 | 30.35 | C |
| ATOM | 995 | CD2 | TYR | A | 161 | 31.905 | 46.138 | 45.047 | 1.00 | 30.35 | C |
| ATOM | 996 | CE1 | TYR | A | 161 | 33.730 | 44.977 | 43.329 | 1.00 | 30.35 | C |
| ATOM | 997 | CE2 | TYR | A | 161 | 33.194 | 46.664 | 44.954 | 1.00 | 30.35 | C |
| ATOM | 998 | CZ | TYR | A | 161 | 34.097 | 46.080 | 44.096 | 1.00 | 30.35 | C |
| ATOM | 999 | OH | TYR | A | 161 | 35.373 | 46.588 | 44.021 | 1.00 | 30.35 | O |
| ATOM | 1000 | N | MET | A | 162 | 27.227 | 43.861 | 43.784 | 1.00 | 23.41 | N |
| ATOM | 1001 | CA | MET | A | 162 | 25.906 | 43.394 | 44.171 | 1.00 | 23.41 | C |
| ATOM | 1002 | C | MET | A | 162 | 25.369 | 42.295 | 43.257 | 1.00 | 23.41 | C |
| ATOM | 1003 | O | MET | A | 162 | 24.759 | 41.335 | 43.733 | 1.00 | 23.41 | O |
| ATOM | 1004 | CB | MET | A | 162 | 24.915 | 44.564 | 44.227 | 1.00 | 22.26 | C |
| ATOM | 1005 | CG | MET | A | 162 | 25.111 | 45.500 | 45.418 | 1.00 | 22.26 | C |
| ATOM | 1006 | SD | MET | A | 162 | 25.381 | 44.652 | 46.986 | 1.00 | 22.26 | S |
| ATOM | 1007 | CE | MET | A | 162 | 23.973 | 43.619 | 47.107 | 1.00 | 22.26 | C |
| ATOM | 1008 | N | TYR | A | 163 | 25.598 | 42.447 | 41.951 | 1.00 | 21.29 | N |
| ATOM | 1009 | CA | TYR | A | 163 | 25.156 | 41.480 | 40.957 | 1.00 | 21.29 | C |
| ATOM | 1010 | C | TYR | A | 163 | 25.762 | 40.114 | 41.252 | 1.00 | 21.29 | C |
| ATOM | 1011 | O | TYR | A | 163 | 25.065 | 39.099 | 41.360 | 1.00 | 21.29 | O |
| ATOM | 1012 | CB | TYR | A | 163 | 25.599 | 41.922 | 39.569 | 1.00 | 27.46 | C |
| ATOM | 1013 | CG | TYR | A | 163 | 25.196 | 40.971 | 38.463 | 1.00 | 27.46 | C |
| ATOM | 1014 | CD1 | TYR | A | 163 | 23.945 | 41.065 | 37.860 | 1.00 | 27.46 | C |
| ATOM | 1015 | CD2 | TYR | A | 163 | 26.066 | 39.978 | 38.025 | 1.00 | 27.46 | C |
| ATOM | 1016 | CE1 | TYR | A | 163 | 23.570 | 40.192 | 36.843 | 1.00 | 27.46 | C |
| ATOM | 1017 | CE2 | TYR | A | 163 | 25.706 | 39.106 | 37.021 | 1.00 | 27.46 | C |
| ATOM | 1018 | CZ | TYR | A | 163 | 24.460 | 39.216 | 36.423 | 1.00 | 27.46 | C |
| ATOM | 1019 | OH | TYR | A | 163 | 24.120 | 38.373 | 35.380 | 1.00 | 27.46 | O |
| ATOM | 1020 | N | GLN | A | 164 | 27.078 | 40.098 | 41.377 | 1.00 | 23.12 | N |
| ATOM | 1021 | CA | GLN | A | 164 | 27.768 | 38.864 | 41.648 | 1.00 | 23.12 | C |
| ATOM | 1022 | C | GLN | A | 164 | 27.300 | 38.214 | 42.951 | 1.00 | 23.12 | C |
| ATOM | 1023 | O | GLN | A | 164 | 27.198 | 36.991 | 43.029 | 1.00 | 23.12 | O |
| ATOM | 1024 | CB | GLN | A | 164 | 29.276 | 39.116 | 41.641 | 1.00 | 30.11 | C |
| ATOM | 1025 | CG | GLN | A | 164 | 29.763 | 39.698 | 40.313 | 1.00 | 30.11 | C |
| ATOM | 1026 | CD | GLN | A | 164 | 31.266 | 39.831 | 40.232 | 1.00 | 30.11 | C |
| ATOM | 1027 | OE1 | GLN | A | 164 | 31.973 | 38.841 | 40.085 | 1.00 | 30.11 | O |
| ATOM | 1028 | NE2 | GLN | A | 164 | 31.765 | 41.058 | 40.334 | 1.00 | 30.11 | N |
| ATOM | 1029 | N | LEU | A | 165 | 26.999 | 39.017 | 43.969 | 1.00 | 24.85 | N |
| ATOM | 1030 | CA | LEU | A | 165 | 26.528 | 38.450 | 45.225 | 1.00 | 24.85 | C |
| ATOM | 1031 | C | LEU | A | 165 | 25.153 | 37.818 | 45.006 | 1.00 | 24.85 | C |
| ATOM | 1032 | O | LEU | A | 165 | 24.876 | 36.713 | 45.472 | 1.00 | 24.85 | O |
| ATOM | 1033 | CB | LEU | A | 165 | 26.455 | 39.527 | 46.309 | 1.00 | 19.06 | C |
| ATOM | 1034 | CG | LEU | A | 165 | 25.716 | 39.235 | 47.625 | 1.00 | 19.06 | C |
| ATOM | 1035 | CD1 | LEU | A | 165 | 26.302 | 38.018 | 48.340 | 1.00 | 19.06 | C |
| ATOM | 1036 | CD2 | LEU | A | 165 | 25.789 | 40.460 | 48.508 | 1.00 | 19.06 | C |
| ATOM | 1037 | N | PHE | A | 166 | 24.289 | 38.507 | 44.275 | 1.00 | 26.70 | N |
| ATOM | 1038 | CA | PHE | A | 166 | 22.968 | 37.959 | 44.036 | 1.00 | 26.70 | C |
| ATOM | 1039 | C | PHE | A | 166 | 23.044 | 36.654 | 43.268 | 1.00 | 26.70 | C |
| ATOM | 1040 | O | PHE | A | 166 | 22.239 | 35.745 | 43.469 | 1.00 | 26.70 | O |
| ATOM | 1041 | CB | PHE | A | 166 | 22.094 | 38.970 | 43.303 | 1.00 | 15.81 | C |
| ATOM | 1042 | CG | PHE | A | 166 | 21.510 | 40.001 | 44.204 | 1.00 | 15.81 | C |
| ATOM | 1043 | CD1 | PHE | A | 166 | 20.795 | 39.619 | 45.332 | 1.00 | 15.81 | C |
| ATOM | 1044 | CD2 | PHE | A | 166 | 21.688 | 41.352 | 43.951 | 1.00 | 15.81 | C |
| ATOM | 1045 | CE1 | PHE | A | 166 | 20.268 | 40.566 | 46.199 | 1.00 | 15.81 | C |
| ATOM | 1046 | CE2 | PHE | A | 166 | 21.163 | 42.302 | 44.812 | 1.00 | 15.81 | C |
| ATOM | 1047 | CZ | PHE | A | 166 | 20.452 | 41.903 | 45.940 | 1.00 | 15.81 | C |
| ATOM | 1048 | N | ARG | A | 167 | 24.029 | 36.558 | 42.393 | 1.00 | 36.56 | N |
| ATOM | 1049 | CA | ARG | A | 167 | 24.196 | 35.352 | 41.618 | 1.00 | 36.56 | C |
| ATOM | 1050 | C | ARG | A | 167 | 24.684 | 34.240 | 42.539 | 1.00 | 36.56 | C |

| | | | | | | | | | | | |
|---|---|---|---|---|---|---|---|---|---|---|---|
| ATOM | 1051 | O | ARG | A | 167 | 24.209 | 33.109 | 42.473 | 1.00 | 36.56 | O |
| ATOM | 1052 | CB | ARG | A | 167 | 25.193 | 35.593 | 40.496 | 1.00 | 35.25 | C |
| ATOM | 1053 | CG | ARG | A | 167 | 25.194 | 34.513 | 39.452 | 1.00 | 35.25 | C |
| ATOM | 1054 | CD | ARG | A | 167 | 26.048 | 34.929 | 38.290 | 1.00 | 35.25 | C |
| ATOM | 1055 | NE | ARG | A | 167 | 26.250 | 33.830 | 37.367 | 1.00 | 35.25 | N |
| ATOM | 1056 | CZ | ARG | A | 167 | 27.311 | 33.725 | 36.585 | 1.00 | 35.25 | C |
| ATOM | 1057 | NH1 | ARG | A | 167 | 28.251 | 34.662 | 36.635 | 1.00 | 35.25 | N |
| ATOM | 1058 | NH2 | ARG | A | 167 | 27.430 | 32.696 | 35.753 | 1.00 | 35.25 | N |
| ATOM | 1059 | N | SER | A | 168 | 25.623 | 34.560 | 43.418 | 1.00 | 31.54 | N |
| ATOM | 1060 | CA | SER | A | 168 | 26.121 | 33.542 | 44.325 | 1.00 | 31.54 | C |
| ATOM | 1061 | C | SER | A | 168 | 24.994 | 33.039 | 45.224 | 1.00 | 31.54 | C |
| ATOM | 1062 | O | SER | A | 168 | 24.991 | 31.885 | 45.632 | 1.00 | 31.54 | O |
| ATOM | 1063 | CB | SER | A | 168 | 27.266 | 34.091 | 45.175 | 1.00 | 22.91 | C |
| ATOM | 1064 | OG | SER | A | 168 | 26.815 | 35.085 | 46.062 | 1.00 | 22.91 | O |
| ATOM | 1065 | N | LEU | A | 169 | 24.027 | 33.903 | 45.508 | 1.00 | 25.05 | N |
| ATOM | 1066 | CA | LEU | A | 169 | 22.928 | 33.537 | 46.379 | 1.00 | 25.05 | C |
| ATOM | 1067 | C | LEU | A | 169 | 21.940 | 32.660 | 45.667 | 1.00 | 25.05 | C |
| ATOM | 1068 | O | LEU | A | 169 | 21.365 | 31.759 | 46.264 | 1.00 | 25.05 | O |
| ATOM | 1069 | CB | LEU | A | 169 | 22.228 | 34.786 | 46.910 | 1.00 | 26.63 | C |
| ATOM | 1070 | CG | LEU | A | 169 | 22.394 | 35.175 | 48.390 | 1.00 | 26.63 | C |
| ATOM | 1071 | CD1 | LEU | A | 169 | 23.849 | 35.228 | 48.786 | 1.00 | 26.63 | C |
| ATOM | 1072 | CD2 | LEU | A | 169 | 21.726 | 36.531 | 48.614 | 1.00 | 26.63 | C |
| ATOM | 1073 | N | ALA | A | 170 | 21.742 | 32.917 | 44.381 | 1.00 | 29.29 | N |
| ATOM | 1074 | CA | ALA | A | 170 | 20.808 | 32.122 | 43.601 | 1.00 | 29.29 | C |
| ATOM | 1075 | C | ALA | A | 170 | 21.369 | 30.708 | 43.482 | 1.00 | 29.29 | C |
| ATOM | 1076 | O | ALA | A | 170 | 20.644 | 29.726 | 43.475 | 1.00 | 29.29 | O |
| ATOM | 1077 | CB | ALA | A | 170 | 20.625 | 32.740 | 42.230 | 1.00 | 8.31 | C |
| ATOM | 1078 | N | TYR | A | 171 | 22.684 | 30.614 | 43.421 | 1.00 | 28.63 | N |
| ATOM | 1079 | CA | TYR | A | 171 | 23.320 | 29.328 | 43.282 | 1.00 | 28.63 | C |
| ATOM | 1080 | C | TYR | A | 171 | 23.184 | 28.449 | 44.511 | 1.00 | 28.63 | C |
| ATOM | 1081 | O | TYR | A | 171 | 22.729 | 27.318 | 44.410 | 1.00 | 28.63 | O |
| ATOM | 1082 | CB | TYR | A | 171 | 24.789 | 29.517 | 42.941 | 1.00 | 35.01 | C |
| ATOM | 1083 | CG | TYR | A | 171 | 25.518 | 28.219 | 42.790 | 1.00 | 35.01 | C |
| ATOM | 1084 | CD1 | TYR | A | 171 | 25.255 | 27.366 | 41.721 | 1.00 | 35.01 | C |
| ATOM | 1085 | CD2 | TYR | A | 171 | 26.473 | 27.835 | 43.716 | 1.00 | 35.01 | C |
| ATOM | 1086 | CE1 | TYR | A | 171 | 25.934 | 26.164 | 41.577 | 1.00 | 35.01 | C |
| ATOM | 1087 | CE2 | TYR | A | 171 | 27.160 | 26.633 | 43.585 | 1.00 | 35.01 | C |
| ATOM | 1088 | CZ | TYR | A | 171 | 26.889 | 25.802 | 42.512 | 1.00 | 35.01 | C |
| ATOM | 1089 | OH | TYR | A | 171 | 27.601 | 24.627 | 42.370 | 1.00 | 35.01 | O |
| ATOM | 1090 | N | ILE | A | 172 | 23.587 | 28.954 | 45.670 | 1.00 | 23.62 | N |
| ATOM | 1091 | CA | ILE | A | 172 | 23.492 | 28.170 | 46.891 | 1.00 | 23.62 | C |
| ATOM | 1092 | C | ILE | A | 172 | 22.041 | 27.962 | 47.309 | 1.00 | 23.62 | C |
| ATOM | 1093 | O | ILE | A | 172 | 21.702 | 26.948 | 47.906 | 1.00 | 23.62 | O |
| ATOM | 1094 | CB | ILE | A | 172 | 24.263 | 28.822 | 48.051 | 1.00 | 20.34 | C |
| ATOM | 1095 | CG1 | ILE | A | 172 | 23.753 | 30.238 | 48.287 | 1.00 | 20.34 | C |
| ATOM | 1096 | CG2 | ILE | A | 172 | 25.750 | 28.809 | 47.755 | 1.00 | 20.34 | C |
| ATOM | 1097 | CD1 | ILE | A | 172 | 24.191 | 30.792 | 49.601 | 1.00 | 20.34 | C |
| ATOM | 1098 | N | HIS | A | 173 | 21.185 | 28.924 | 46.995 | 1.00 | 26.99 | N |
| ATOM | 1099 | CA | HIS | A | 173 | 19.779 | 28.796 | 47.338 | 1.00 | 26.99 | C |
| ATOM | 1100 | C | HIS | A | 173 | 19.170 | 27.654 | 46.532 | 1.00 | 26.99 | C |
| ATOM | 1101 | O | HIS | A | 173 | 18.306 | 26.917 | 47.022 | 1.00 | 26.99 | O |
| ATOM | 1102 | CB | HIS | A | 173 | 19.021 | 30.103 | 47.054 | 1.00 | 22.05 | C |
| ATOM | 1103 | CG | HIS | A | 173 | 19.186 | 31.149 | 48.120 | 1.00 | 22.05 | C |
| ATOM | 1104 | ND1 | HIS | A | 173 | 18.495 | 32.340 | 48.104 | 1.00 | 22.05 | N |
| ATOM | 1105 | CD2 | HIS | A | 173 | 19.989 | 31.201 | 49.207 | 1.00 | 22.05 | C |
| ATOM | 1106 | CE1 | HIS | A | 173 | 18.870 | 33.081 | 49.130 | 1.00 | 22.05 | C |
| ATOM | 1107 | NE2 | HIS | A | 173 | 19.776 | 32.415 | 49.815 | 1.00 | 22.05 | N |
| ATOM | 1108 | N | SER | A | 174 | 19.635 | 27.499 | 45.297 | 1.00 | 35.53 | N |
| ATOM | 1109 | CA | SER | A | 174 | 19.128 | 26.451 | 44.428 | 1.00 | 35.53 | C |
| ATOM | 1110 | C | SER | A | 174 | 19.321 | 25.091 | 45.082 | 1.00 | 35.53 | C |
| ATOM | 1111 | O | SER | A | 174 | 18.616 | 24.149 | 44.748 | 1.00 | 35.53 | O |
| ATOM | 1112 | CB | SER | A | 174 | 19.818 | 26.495 | 43.059 | 1.00 | 34.86 | C |

| | | | | | | | | | | | |
|---|---|---|---|---|---|---|---|---|---|---|---|
| ATOM | 1113 | OG | SER | A | 174 | 21.175 | 26.113 | 43.151 | 1.00 | 34.86 | O |
| ATOM | 1114 | N | PHE | A | 175 | 20.275 | 24.991 | 46.004 | 1.00 | 28.76 | N |
| ATOM | 1115 | CA | PHE | A | 175 | 20.525 | 23.749 | 46.724 | 1.00 | 28.76 | C |
| ATOM | 1116 | C | PHE | A | 175 | 19.823 | 23.820 | 48.067 | 1.00 | 28.76 | C |
| ATOM | 1117 | O | PHE | A | 175 | 20.035 | 22.970 | 48.934 | 1.00 | 28.76 | O |
| ATOM | 1118 | CB | PHE | A | 175 | 22.016 | 23.543 | 46.980 | 1.00 | 47.49 | C |
| ATOM | 1119 | CG | PHE | A | 175 | 22.815 | 23.237 | 45.749 | 1.00 | 47.49 | C |
| ATOM | 1120 | CD1 | PHE | A | 175 | 22.334 | 22.357 | 44.792 | 1.00 | 47.49 | C |
| ATOM | 1121 | CD2 | PHE | A | 175 | 24.070 | 23.799 | 45.567 | 1.00 | 47.49 | C |
| ATOM | 1122 | CE1 | PHE | A | 175 | 23.089 | 22.041 | 43.665 | 1.00 | 47.49 | C |
| ATOM | 1123 | CE2 | PHE | A | 175 | 24.838 | 23.493 | 44.445 | 1.00 | 47.49 | C |
| ATOM | 1124 | CZ | PHE | A | 175 | 24.348 | 22.610 | 43.488 | 1.00 | 47.49 | C |
| ATOM | 1125 | N | GLY | A | 176 | 18.997 | 24.849 | 48.240 | 1.00 | 30.85 | N |
| ATOM | 1126 | CA | GLY | A | 176 | 18.283 | 25.042 | 49.491 | 1.00 | 30.85 | C |
| ATOM | 1127 | C | GLY | A | 176 | 19.168 | 25.516 | 50.636 | 1.00 | 30.85 | C |
| ATOM | 1128 | O | GLY | A | 176 | 18.747 | 25.511 | 51.788 | 1.00 | 30.85 | O |
| ATOM | 1129 | N | ILE | A | 177 | 20.393 | 25.926 | 50.320 | 1.00 | 26.68 | N |
| ATOM | 1130 | CA | ILE | A | 177 | 21.331 | 26.394 | 51.333 | 1.00 | 26.68 | C |
| ATOM | 1131 | C | ILE | A | 177 | 21.291 | 27.905 | 51.564 | 1.00 | 26.68 | C |
| ATOM | 1132 | O | ILE | A | 177 | 21.374 | 28.690 | 50.631 | 1.00 | 26.68 | O |
| ATOM | 1133 | CB | ILE | A | 177 | 22.770 | 25.997 | 50.980 | 1.00 | 30.66 | C |
| ATOM | 1134 | CG1 | ILE | A | 177 | 22.870 | 24.480 | 50.893 | 1.00 | 30.66 | C |
| ATOM | 1135 | CG2 | ILE | A | 177 | 23.733 | 26.511 | 52.044 | 1.00 | 30.66 | C |
| ATOM | 1136 | CD1 | ILE | A | 177 | 24.173 | 24.010 | 50.333 | 1.00 | 30.66 | C |
| ATOM | 1137 | N | CYS | A | 178 | 21.178 | 28.292 | 52.830 | 1.00 | 23.27 | N |
| ATOM | 1138 | CA | CYS | A | 178 | 21.106 | 29.688 | 53.233 | 1.00 | 23.27 | C |
| ATOM | 1139 | C | CYS | A | 178 | 22.415 | 30.049 | 53.907 | 1.00 | 23.27 | C |
| ATOM | 1140 | O | CYS | A | 178 | 22.857 | 29.354 | 54.811 | 1.00 | 23.27 | O |
| ATOM | 1141 | CB | CYS | A | 178 | 19.943 | 29.859 | 54.198 | 1.00 | 24.81 | C |
| ATOM | 1142 | SG | CYS | A | 178 | 19.595 | 31.517 | 54.719 | 1.00 | 24.81 | S |
| ATOM | 1143 | N | HIS | A | 179 | 23.036 | 31.134 | 53.462 | 1.00 | 22.31 | N |
| ATOM | 1144 | CA | HIS | A | 179 | 24.314 | 31.543 | 54.023 | 1.00 | 22.31 | C |
| ATOM | 1145 | C | HIS | A | 179 | 24.145 | 31.959 | 55.478 | 1.00 | 22.31 | C |
| ATOM | 1146 | O | HIS | A | 179 | 24.888 | 31.510 | 56.343 | 1.00 | 22.31 | O |
| ATOM | 1147 | CB | HIS | A | 179 | 24.914 | 32.683 | 53.184 | 1.00 | 20.53 | C |
| ATOM | 1148 | CG | HIS | A | 179 | 26.342 | 32.992 | 53.511 | 1.00 | 20.53 | C |
| ATOM | 1149 | ND1 | HIS | A | 179 | 26.714 | 33.698 | 54.637 | 1.00 | 20.53 | N |
| ATOM | 1150 | CD2 | HIS | A | 179 | 27.489 | 32.662 | 52.879 | 1.00 | 20.53 | C |
| ATOM | 1151 | CE1 | HIS | A | 179 | 28.029 | 33.787 | 54.683 | 1.00 | 20.53 | C |
| ATOM | 1152 | NE2 | HIS | A | 179 | 28.525 | 33.166 | 53.628 | 1.00 | 20.53 | N |
| ATOM | 1153 | N | ARG | A | 180 | 23.159 | 32.815 | 55.732 | 1.00 | 34.54 | N |
| ATOM | 1154 | CA | ARG | A | 180 | 22.849 | 33.292 | 57.075 | 1.00 | 34.54 | C |
| ATOM | 1155 | C | ARG | A | 180 | 23.777 | 34.346 | 57.671 | 1.00 | 34.54 | C |
| ATOM | 1156 | O | ARG | A | 180 | 23.511 | 34.853 | 58.761 | 1.00 | 34.54 | O |
| ATOM | 1157 | CB | ARG | A | 180 | 22.798 | 32.129 | 58.039 | 1.00 | 18.15 | C |
| ATOM | 1158 | CG | ARG | A | 180 | 21.666 | 31.199 | 57.856 | 1.00 | 18.15 | C |
| ATOM | 1159 | CD | ARG | A | 180 | 22.049 | 29.996 | 58.616 | 1.00 | 18.15 | C |
| ATOM | 1160 | NE | ARG | A | 180 | 21.007 | 29.512 | 59.487 | 1.00 | 18.15 | N |
| ATOM | 1161 | CZ | ARG | A | 180 | 21.237 | 28.665 | 60.472 | 1.00 | 18.15 | C |
| ATOM | 1162 | NH1 | ARG | A | 180 | 22.466 | 28.240 | 60.690 | 1.00 | 18.15 | N |
| ATOM | 1163 | NH2 | ARG | A | 180 | 20.241 | 28.235 | 61.218 | 1.00 | 18.15 | N |
| ATOM | 1164 | N | ASP | A | 181 | 24.866 | 34.669 | 56.991 | 1.00 | 23.17 | N |
| ATOM | 1165 | CA | ASP | A | 181 | 25.770 | 35.676 | 57.525 | 1.00 | 23.17 | C |
| ATOM | 1166 | C | ASP | A | 181 | 26.338 | 36.569 | 56.409 | 1.00 | 23.17 | C |
| ATOM | 1167 | O | ASP | A | 181 | 27.544 | 36.811 | 56.317 | 1.00 | 23.17 | O |
| ATOM | 1168 | CB | ASP | A | 181 | 26.885 | 34.987 | 58.326 | 1.00 | 30.39 | C |
| ATOM | 1169 | CG | ASP | A | 181 | 27.696 | 35.960 | 59.165 | 1.00 | 30.39 | C |
| ATOM | 1170 | OD1 | ASP | A | 181 | 27.178 | 37.058 | 59.464 | 1.00 | 30.39 | O |
| ATOM | 1171 | OD2 | ASP | A | 181 | 28.845 | 35.620 | 59.540 | 1.00 | 30.39 | O |
| ATOM | 1172 | N | ILE | A | 182 | 25.445 | 37.060 | 55.566 | 1.00 | 12.70 | N |
| ATOM | 1173 | CA | ILE | A | 182 | 25.854 | 37.913 | 54.481 | 1.00 | 12.70 | C |
| ATOM | 1174 | C | ILE | A | 182 | 26.198 | 39.282 | 55.049 | 1.00 | 12.70 | C |

| | | | | | | | | | | | |
|---|---|---|---|---|---|---|---|---|---|---|---|
| ATOM | 1175 | O | ILE | A | 182 | 25.362 | 39.959 | 55.639 | 1.00 | 12.70 | O |
| ATOM | 1176 | CB | ILE | A | 182 | 24.733 | 38.035 | 53.417 | 1.00 | 18.50 | C |
| ATOM | 1177 | CG1 | ILE | A | 182 | 24.390 | 36.648 | 52.867 | 1.00 | 18.50 | C |
| ATOM | 1178 | CG2 | ILE | A | 182 | 25.154 | 38.975 | 52.297 | 1.00 | 18.50 | C |
| ATOM | 1179 | CD1 | ILE | A | 182 | 25.543 | 35.928 | 52.216 | 1.00 | 18.50 | C |
| ATOM | 1180 | N | LYS | A | 183 | 27.450 | 39.669 | 54.887 | 1.00 | 22.40 | N |
| ATOM | 1181 | CA | LYS | A | 183 | 27.912 | 40.947 | 55.372 | 1.00 | 22.40 | C |
| ATOM | 1182 | C | LYS | A | 183 | 29.123 | 41.353 | 54.558 | 1.00 | 22.40 | C |
| ATOM | 1183 | O | LYS | A | 183 | 29.874 | 40.506 | 54.073 | 1.00 | 22.40 | O |
| ATOM | 1184 | CB | LYS | A | 183 | 28.284 | 40.868 | 56.852 | 1.00 | 28.78 | C |
| ATOM | 1185 | CG | LYS | A | 183 | 29.327 | 39.814 | 57.188 | 1.00 | 28.78 | C |
| ATOM | 1186 | CD | LYS | A | 183 | 29.784 | 39.987 | 58.614 | 1.00 | 28.78 | C |
| ATOM | 1187 | CE | LYS | A | 183 | 30.439 | 38.737 | 59.150 | 1.00 | 28.78 | C |
| ATOM | 1188 | NZ | LYS | A | 183 | 30.875 | 38.884 | 60.564 | 0.00 | 28.78 | N |
| ATOM | 1189 | N | PRO | A | 184 | 29.340 | 42.662 | 54.420 | 1.00 | 25.56 | N |
| ATOM | 1190 | CA | PRO | A | 184 | 30.454 | 43.232 | 53.664 | 1.00 | 25.56 | C |
| ATOM | 1191 | C | PRO | A | 184 | 31.780 | 42.508 | 53.819 | 1.00 | 25.56 | C |
| ATOM | 1192 | O | PRO | A | 184 | 32.515 | 42.372 | 52.848 | 1.00 | 25.56 | O |
| ATOM | 1193 | CB | PRO | A | 184 | 30.509 | 44.667 | 54.173 | 1.00 | 20.25 | C |
| ATOM | 1194 | CG | PRO | A | 184 | 29.075 | 44.965 | 54.462 | 1.00 | 20.25 | C |
| ATOM | 1195 | CD | PRO | A | 184 | 28.617 | 43.710 | 55.158 | 1.00 | 20.25 | C |
| ATOM | 1196 | N | GLN | A | 185 | 32.077 | 42.041 | 55.031 | 1.00 | 23.26 | N |
| ATOM | 1197 | CA | GLN | A | 185 | 33.335 | 41.341 | 55.304 | 1.00 | 23.26 | C |
| ATOM | 1198 | C | GLN | A | 185 | 33.437 | 39.961 | 54.693 | 1.00 | 23.26 | C |
| ATOM | 1199 | O | GLN | A | 185 | 34.536 | 39.465 | 54.484 | 1.00 | 23.26 | O |
| ATOM | 1200 | CB | GLN | A | 185 | 33.586 | 41.205 | 56.807 | 1.00 | 41.92 | C |
| ATOM | 1201 | CG | GLN | A | 185 | 33.616 | 42.510 | 57.564 | 1.00 | 41.92 | C |
| ATOM | 1202 | CD | GLN | A | 185 | 32.273 | 42.847 | 58.164 | 1.00 | 41.92 | C |
| ATOM | 1203 | OE1 | GLN | A | 185 | 31.253 | 42.905 | 57.464 | 1.00 | 41.92 | O |
| ATOM | 1204 | NE2 | GLN | A | 185 | 32.258 | 43.064 | 59.477 | 1.00 | 41.92 | N |
| ATOM | 1205 | N | ASN | A | 186 | 32.295 | 39.338 | 54.422 | 1.00 | 19.39 | N |
| ATOM | 1206 | CA | ASN | A | 186 | 32.293 | 38.011 | 53.829 | 1.00 | 19.39 | C |
| ATOM | 1207 | C | ASN | A | 186 | 32.199 | 38.070 | 52.320 | 1.00 | 19.39 | C |
| ATOM | 1208 | O | ASN | A | 186 | 31.858 | 37.088 | 51.668 | 1.00 | 19.39 | O |
| ATOM | 1209 | CB | ASN | A | 186 | 31.158 | 37.164 | 54.390 | 1.00 | 23.19 | C |
| ATOM | 1210 | CG | ASN | A | 186 | 31.369 | 36.812 | 55.841 | 1.00 | 23.19 | C |
| ATOM | 1211 | OD1 | ASN | A | 186 | 32.500 | 36.606 | 56.273 | 1.00 | 23.19 | O |
| ATOM | 1212 | ND2 | ASN | A | 186 | 30.282 | 36.731 | 56.599 | 1.00 | 23.19 | N |
| ATOM | 1213 | N | LEU | A | 187 | 32.505 | 39.238 | 51.777 | 1.00 | 16.46 | N |
| ATOM | 1214 | CA | LEU | A | 187 | 32.499 | 39.433 | 50.345 | 1.00 | 16.46 | C |
| ATOM | 1215 | C | LEU | A | 187 | 33.925 | 39.728 | 49.903 | 1.00 | 16.46 | C |
| ATOM | 1216 | O | LEU | A | 187 | 34.386 | 40.861 | 49.964 | 1.00 | 16.46 | O |
| ATOM | 1217 | CB | LEU | A | 187 | 31.578 | 40.590 | 49.966 | 1.00 | 21.42 | C |
| ATOM | 1218 | CG | LEU | A | 187 | 30.114 | 40.438 | 50.384 | 1.00 | 21.42 | C |
| ATOM | 1219 | CD1 | LEU | A | 187 | 29.348 | 41.638 | 49.928 | 1.00 | 21.42 | C |
| ATOM | 1220 | CD2 | LEU | A | 187 | 29.514 | 39.164 | 49.801 | 1.00 | 21.42 | C |
| ATOM | 1221 | N | LEU | A | 188 | 34.633 | 38.691 | 49.478 | 1.00 | 26.45 | N |
| ATOM | 1222 | CA | LEU | A | 188 | 36.004 | 38.848 | 49.028 | 1.00 | 26.45 | C |
| ATOM | 1223 | C | LEU | A | 188 | 35.994 | 39.337 | 47.594 | 1.00 | 26.45 | C |
| ATOM | 1224 | O | LEU | A | 188 | 35.086 | 39.026 | 46.818 | 1.00 | 26.45 | O |
| ATOM | 1225 | CB | LEU | A | 188 | 36.745 | 37.515 | 49.088 | 1.00 | 28.74 | C |
| ATOM | 1226 | CG | LEU | A | 188 | 36.637 | 36.664 | 50.352 | 1.00 | 28.74 | C |
| ATOM | 1227 | CD1 | LEU | A | 188 | 37.412 | 35.392 | 50.137 | 1.00 | 28.74 | C |
| ATOM | 1228 | CD2 | LEU | A | 188 | 37.173 | 37.406 | 51.560 | 1.00 | 28.74 | C |
| ATOM | 1229 | N | LEU | A | 189 | 37.006 | 40.107 | 47.230 | 1.00 | 31.93 | N |
| ATOM | 1230 | CA | LEU | A | 189 | 37.075 | 40.596 | 45.873 | 1.00 | 31.93 | C |
| ATOM | 1231 | C | LEU | A | 189 | 38.495 | 40.884 | 45.400 | 1.00 | 31.93 | C |
| ATOM | 1232 | O | LEU | A | 189 | 39.360 | 41.292 | 46.169 | 1.00 | 31.93 | O |
| ATOM | 1233 | CB | LEU | A | 189 | 36.168 | 41.824 | 45.716 | 1.00 | 28.57 | C |
| ATOM | 1234 | CG | LEU | A | 189 | 36.507 | 43.170 | 46.340 | 1.00 | 28.57 | C |
| ATOM | 1235 | CD1 | LEU | A | 189 | 35.261 | 44.016 | 46.315 | 1.00 | 28.57 | C |
| ATOM | 1236 | CD2 | LEU | A | 189 | 36.994 | 43.010 | 47.751 | 0.00 | 28.57 | C |

| | | | | | | | | | | | |
|---|---|---|---|---|---|---|---|---|---|---|---|
| ATOM | 1237 | N | ASP | A | 190 | 38.718 | 40.606 | 44.121 | 1.00 | 38.76 | N |
| ATOM | 1238 | CA | ASP | A | 190 | 39.991 | 40.812 | 43.451 | 1.00 | 38.76 | C |
| ATOM | 1239 | C | ASP | A | 190 | 39.926 | 42.256 | 42.991 | 1.00 | 38.76 | C |
| ATOM | 1240 | O | ASP | A | 190 | 39.009 | 42.629 | 42.270 | 1.00 | 38.76 | O |
| ATOM | 1241 | CB | ASP | A | 190 | 40.082 | 39.881 | 42.245 | 1.00 | 48.51 | C |
| ATOM | 1242 | CG | ASP | A | 190 | 41.351 | 40.069 | 41.456 | 1.00 | 48.51 | C |
| ATOM | 1243 | OD1 | ASP | A | 190 | 41.693 | 41.248 | 41.171 | 1.00 | 48.51 | O |
| ATOM | 1244 | OD2 | ASP | A | 190 | 41.991 | 39.042 | 41.116 | 1.00 | 48.51 | O |
| ATOM | 1245 | N | PRO | A | 191 | 40.897 | 43.088 | 43.396 | 1.00 | 50.51 | N |
| ATOM | 1246 | CA | PRO | A | 191 | 40.897 | 44.501 | 43.005 | 1.00 | 50.51 | C |
| ATOM | 1247 | C | PRO | A | 191 | 40.987 | 44.813 | 41.513 | 1.00 | 50.51 | C |
| ATOM | 1248 | O | PRO | A | 191 | 40.411 | 45.806 | 41.045 | 1.00 | 50.51 | O |
| ATOM | 1249 | CB | PRO | A | 191 | 42.060 | 45.080 | 43.803 | 1.00 | 49.44 | C |
| ATOM | 1250 | CG | PRO | A | 191 | 42.989 | 43.914 | 43.959 | 1.00 | 49.44 | C |
| ATOM | 1251 | CD | PRO | A | 191 | 42.057 | 42.772 | 44.253 | 1.00 | 49.44 | C |
| ATOM | 1252 | N | ASP | A | 192 | 41.681 | 43.976 | 40.753 | 1.00 | 42.25 | N |
| ATOM | 1253 | CA | ASP | A | 192 | 41.802 | 44.245 | 39.327 | 1.00 | 42.25 | C |
| ATOM | 1254 | C | ASP | A | 192 | 40.721 | 43.625 | 38.468 | 1.00 | 42.25 | C |
| ATOM | 1255 | O | ASP | A | 192 | 40.156 | 44.297 | 37.612 | 1.00 | 42.25 | O |
| ATOM | 1256 | CB | ASP | A | 192 | 43.175 | 43.814 | 38.841 | 1.00 | 49.21 | C |
| ATOM | 1257 | CG | ASP | A | 192 | 44.270 | 44.582 | 39.523 | 1.00 | 49.21 | C |
| ATOM | 1258 | OD1 | ASP | A | 192 | 44.255 | 45.826 | 39.412 | 1.00 | 49.21 | O |
| ATOM | 1259 | OD2 | ASP | A | 192 | 45.126 | 43.957 | 40.182 | 1.00 | 49.21 | O |
| ATOM | 1260 | N | THR | A | 193 | 40.426 | 42.350 | 38.684 | 1.00 | 29.33 | N |
| ATOM | 1261 | CA | THR | A | 193 | 39.397 | 41.688 | 37.899 | 1.00 | 29.33 | C |
| ATOM | 1262 | C | THR | A | 193 | 37.991 | 42.148 | 38.290 | 1.00 | 29.33 | C |
| ATOM | 1263 | O | THR | A | 193 | 37.080 | 42.157 | 37.464 | 1.00 | 29.33 | O |
| ATOM | 1264 | CB | THR | A | 193 | 39.495 | 40.166 | 38.063 | 1.00 | 34.77 | C |
| ATOM | 1265 | OG1 | THR | A | 193 | 39.388 | 39.823 | 39.450 | 1.00 | 34.77 | O |
| ATOM | 1266 | CG2 | THR | A | 193 | 40.823 | 39.671 | 37.532 | 1.00 | 34.77 | C |
| ATOM | 1267 | N | ALA | A | 194 | 37.830 | 42.532 | 39.553 | 1.00 | 23.90 | N |
| ATOM | 1268 | CA | ALA | A | 194 | 36.550 | 42.986 | 40.087 | 1.00 | 23.90 | C |
| ATOM | 1269 | C | ALA | A | 194 | 35.613 | 41.818 | 40.375 | 1.00 | 23.90 | C |
| ATOM | 1270 | O | ALA | A | 194 | 34.409 | 42.010 | 40.529 | 1.00 | 23.90 | O |
| ATOM | 1271 | CB | ALA | A | 194 | 35.873 | 43.964 | 39.118 | 1.00 | 9.74 | C |
| ATOM | 1272 | N | VAL | A | 195 | 36.148 | 40.604 | 40.439 | 1.00 | 29.57 | N |
| ATOM | 1273 | CA | VAL | A | 195 | 35.279 | 39.473 | 40.713 | 1.00 | 29.57 | C |
| ATOM | 1274 | C | VAL | A | 195 | 35.125 | 39.322 | 42.213 | 1.00 | 29.57 | C |
| ATOM | 1275 | O | VAL | A | 195 | 36.091 | 39.427 | 42.973 | 1.00 | 29.57 | O |
| ATOM | 1276 | CB | VAL | A | 195 | 35.783 | 38.129 | 40.049 | 1.00 | 32.11 | C |
| ATOM | 1277 | CG1 | VAL | A | 195 | 36.886 | 38.418 | 39.064 | 1.00 | 32.11 | C |
| ATOM | 1278 | CG2 | VAL | A | 195 | 36.198 | 37.107 | 41.103 | 1.00 | 32.11 | C |
| ATOM | 1279 | N | LEU | A | 196 | 33.887 | 39.095 | 42.630 | 1.00 | 27.44 | N |
| ATOM | 1280 | CA | LEU | A | 196 | 33.576 | 38.954 | 44.032 | 1.00 | 27.44 | C |
| ATOM | 1281 | C | LEU | A | 196 | 33.298 | 37.496 | 44.345 | 1.00 | 27.44 | C |
| ATOM | 1282 | O | LEU | A | 196 | 32.859 | 36.743 | 43.484 | 1.00 | 27.44 | O |
| ATOM | 1283 | CB | LEU | A | 196 | 32.375 | 39.843 | 44.368 | 1.00 | 15.60 | C |
| ATOM | 1284 | CG | LEU | A | 196 | 31.772 | 39.758 | 45.762 | 1.00 | 15.60 | C |
| ATOM | 1285 | CD1 | LEU | A | 196 | 31.063 | 41.042 | 46.092 | 1.00 | 15.60 | C |
| ATOM | 1286 | CD2 | LEU | A | 196 | 30.829 | 38.582 | 45.820 | 1.00 | 15.60 | C |
| ATOM | 1287 | N | LYS | A | 197 | 33.586 | 37.098 | 45.575 | 1.00 | 23.44 | N |
| ATOM | 1288 | CA | LYS | A | 197 | 33.353 | 35.730 | 45.990 | 1.00 | 23.44 | C |
| ATOM | 1289 | C | LYS | A | 197 | 32.820 | 35.680 | 47.399 | 1.00 | 23.44 | C |
| ATOM | 1290 | O | LYS | A | 197 | 33.408 | 36.253 | 48.310 | 1.00 | 23.44 | O |
| ATOM | 1291 | CB | LYS | A | 197 | 34.641 | 34.922 | 45.888 | 1.00 | 30.76 | C |
| ATOM | 1292 | CG | LYS | A | 197 | 35.011 | 34.626 | 44.472 | 1.00 | 30.76 | C |
| ATOM | 1293 | CD | LYS | A | 197 | 36.302 | 33.890 | 44.382 | 1.00 | 30.76 | C |
| ATOM | 1294 | CE | LYS | A | 197 | 36.624 | 33.624 | 42.930 | 1.00 | 30.76 | C |
| ATOM | 1295 | NZ | LYS | A | 197 | 37.910 | 32.895 | 42.780 | 1.00 | 30.76 | N |
| ATOM | 1296 | N | LEU | A | 198 | 31.695 | 35.002 | 47.584 | 1.00 | 25.20 | N |
| ATOM | 1297 | CA | LEU | A | 198 | 31.133 | 34.912 | 48.916 | 1.00 | 25.20 | C |
| ATOM | 1298 | C | LEU | A | 198 | 31.959 | 33.914 | 49.695 | 1.00 | 25.20 | C |

| | | | | | | | | | | |
|---|---|---|---|---|---|---|---|---|---|---|
| ATOM | 1299 | O | LEU | A | 198 | 32.332 | 32.872 | 49.179 | 1.00 25.20 | O |
| ATOM | 1300 | CB | LEU | A | 198 | 29.685 | 34.455 | 48.883 | 1.00 24.11 | C |
| ATOM | 1301 | CG | LEU | A | 198 | 28.877 | 34.984 | 50.062 | 1.00 24.11 | C |
| ATOM | 1302 | CD1 | LEU | A | 198 | 27.541 | 34.314 | 50.020 | 1.00 24.11 | C |
| ATOM | 1303 | CD2 | LEU | A | 198 | 29.551 | 34.722 | 51.391 | 0.00 24.11 | C |
| ATOM | 1304 | N | CYS | A | 199 | 32.259 | 34.237 | 50.940 | 1.00 21.99 | N |
| ATOM | 1305 | CA | CYS | A | 199 | 33.044 | 33.334 | 51.744 | 1.00 21.99 | C |
| ATOM | 1306 | C | CYS | A | 199 | 32.413 | 33.097 | 53.097 | 1.00 21.99 | C |
| ATOM | 1307 | O | CYS | A | 199 | 31.312 | 33.560 | 53.378 | 1.00 21.99 | O |
| ATOM | 1308 | CB | CYS | A | 199 | 34.451 | 33.890 | 51.934 | 1.00 29.06 | C |
| ATOM | 1309 | SG | CYS | A | 199 | 34.512 | 35.423 | 52.831 | 1.00 29.06 | S |
| ATOM | 1310 | N | ASP | A | 200 | 33.131 | 32.344 | 53.919 | 1.00 24.47 | N |
| ATOM | 1311 | CA | ASP | A | 200 | 32.719 | 32.024 | 55.274 | 1.00 24.47 | C |
| ATOM | 1312 | C | ASP | A | 200 | 31.352 | 31.376 | 55.428 | 1.00 24.47 | C |
| ATOM | 1313 | O | ASP | A | 200 | 30.389 | 32.028 | 55.813 | 1.00 24.47 | O |
| ATOM | 1314 | CB | ASP | A | 200 | 32.782 | 33.281 | 56.117 | 1.00 45.70 | C |
| ATOM | 1315 | CG | ASP | A | 200 | 32.661 | 32.984 | 57.569 | 1.00 45.70 | C |
| ATOM | 1316 | OD1 | ASP | A | 200 | 33.267 | 31.971 | 58.004 | 1.00 45.70 | O |
| ATOM | 1317 | OD2 | ASP | A | 200 | 31.968 | 33.759 | 58.270 | 1.00 45.70 | O |
| ATOM | 1318 | N | PHE | A | 201 | 31.285 | 30.082 | 55.146 | 1.00 23.81 | N |
| ATOM | 1319 | CA | PHE | A | 201 | 30.038 | 29.346 | 55.244 | 1.00 23.81 | C |
| ATOM | 1320 | C | PHE | A | 201 | 29.875 | 28.656 | 56.579 | 1.00 23.81 | C |
| ATOM | 1321 | O | PHE | A | 201 | 29.098 | 27.731 | 56.720 | 1.00 23.81 | O |
| ATOM | 1322 | CB | PHE | A | 201 | 29.951 | 28.331 | 54.112 | 1.00 17.81 | C |
| ATOM | 1323 | CG | PHE | A | 201 | 29.820 | 28.962 | 52.766 | 1.00 17.81 | C |
| ATOM | 1324 | CD1 | PHE | A | 201 | 30.888 | 29.655 | 52.209 | 1.00 17.81 | C |
| ATOM | 1325 | CD2 | PHE | A | 201 | 28.609 | 28.929 | 52.086 | 1.00 17.81 | C |
| ATOM | 1326 | CE1 | PHE | A | 201 | 30.746 | 30.313 | 50.989 | 1.00 17.81 | C |
| ATOM | 1327 | CE2 | PHE | A | 201 | 28.458 | 29.580 | 50.873 | 1.00 17.81 | C |
| ATOM | 1328 | CZ | PHE | A | 201 | 29.522 | 30.277 | 50.320 | 1.00 17.81 | C |
| ATOM | 1329 | N | GLY | A | 202 | 30.604 | 29.142 | 57.567 | 1.00 20.45 | N |
| ATOM | 1330 | CA | GLY | A | 202 | 30.540 | 28.563 | 58.889 | 1.00 20.45 | C |
| ATOM | 1331 | C | GLY | A | 202 | 29.183 | 28.704 | 59.538 | 1.00 20.45 | C |
| ATOM | 1332 | O | GLY | A | 202 | 28.952 | 28.139 | 60.604 | 1.00 20.45 | O |
| ATOM | 1333 | N | SER | A | 203 | 28.280 | 29.444 | 58.907 | 1.00 28.53 | N |
| ATOM | 1334 | CA | SER | A | 203 | 26.944 | 29.627 | 59.474 | 1.00 28.53 | C |
| ATOM | 1335 | C | SER | A | 203 | 25.884 | 29.102 | 58.515 | 1.00 28.53 | C |
| ATOM | 1336 | O | SER | A | 203 | 24.692 | 29.142 | 58.810 | 1.00 28.53 | O |
| ATOM | 1337 | CB | SER | A | 203 | 26.690 | 31.114 | 59.776 | 1.00 27.90 | C |
| ATOM | 1338 | OG | SER | A | 203 | 27.546 | 31.584 | 60.800 | 1.00 27.90 | O |
| ATOM | 1339 | N | ALA | A | 204 | 26.341 | 28.601 | 57.371 | 1.00 22.96 | N |
| ATOM | 1340 | CA | ALA | A | 204 | 25.462 | 28.086 | 56.337 | 1.00 22.96 | C |
| ATOM | 1341 | C | ALA | A | 204 | 24.753 | 26.827 | 56.773 | 1.00 22.96 | C |
| ATOM | 1342 | O | ALA | A | 204 | 25.292 | 26.003 | 57.520 | 1.00 22.96 | O |
| ATOM | 1343 | CB | ALA | A | 204 | 26.255 | 27.824 | 55.072 | 1.00 20.62 | C |
| ATOM | 1344 | N | LYS | A | 205 | 23.533 | 26.674 | 56.292 | 1.00 33.91 | N |
| ATOM | 1345 | CA | LYS | A | 205 | 22.737 | 25.517 | 56.628 | 1.00 33.91 | C |
| ATOM | 1346 | C | LYS | A | 205 | 21.677 | 25.306 | 55.566 | 1.00 33.91 | C |
| ATOM | 1347 | O | LYS | A | 205 | 21.088 | 26.255 | 55.060 | 1.00 33.91 | O |
| ATOM | 1348 | CB | LYS | A | 205 | 22.039 | 25.735 | 57.965 | 1.00 32.05 | C |
| ATOM | 1349 | CG | LYS | A | 205 | 21.308 | 24.510 | 58.466 | 1.00 32.05 | C |
| ATOM | 1350 | CD | LYS | A | 205 | 20.141 | 24.864 | 59.375 | 1.00 32.05 | C |
| ATOM | 1351 | CE | LYS | A | 205 | 19.483 | 23.602 | 59.955 | 1.00 32.05 | C |
| ATOM | 1352 | NZ | LYS | A | 205 | 18.258 | 23.874 | 60.780 | 1.00 32.05 | N |
| ATOM | 1353 | N | GLN | A | 206 | 21.429 | 24.057 | 55.212 | 1.00 36.97 | N |
| ATOM | 1354 | CA | GLN | A | 206 | 20.378 | 23.813 | 54.254 | 1.00 36.97 | C |
| ATOM | 1355 | C | GLN | A | 206 | 19.094 | 23.860 | 55.061 | 1.00 36.97 | C |
| ATOM | 1356 | O | GLN | A | 206 | 18.964 | 23.134 | 56.036 | 1.00 36.97 | O |
| ATOM | 1357 | CB | GLN | A | 206 | 20.525 | 22.448 | 53.618 | 1.00 58.75 | C |
| ATOM | 1358 | CG | GLN | A | 206 | 19.285 | 22.091 | 52.831 | 1.00 58.75 | C |
| ATOM | 1359 | CD | GLN | A | 206 | 19.457 | 20.860 | 51.976 | 1.00 58.75 | C |
| ATOM | 1360 | OE1 | GLN | A | 206 | 18.467 | 20.295 | 51.484 | 1.00 58.75 | O |

| | | | | | | | | | | |
|---|---|---|---|---|---|---|---|---|---|---|
| ATOM | 1361 | NE2 | GLN | A | 206 | 20.719 | 20.434 | 51.779 | 1.00 | 58.75 | N |
| ATOM | 1362 | N | LEU | A | 207 | 18.158 | 24.719 | 54.659 | 1.00 | 40.39 | N |
| ATOM | 1363 | CA | LEU | A | 207 | 16.887 | 24.876 | 55.361 | 1.00 | 40.39 | C |
| ATOM | 1364 | C | LEU | A | 207 | 15.795 | 23.974 | 54.801 | 1.00 | 40.39 | C |
| ATOM | 1365 | O | LEU | A | 207 | 15.569 | 23.924 | 53.585 | 1.00 | 40.39 | O |
| ATOM | 1366 | CB | LEU | A | 207 | 16.408 | 26.323 | 55.273 | 1.00 | 21.32 | C |
| ATOM | 1367 | CG | LEU | A | 207 | 17.335 | 27.473 | 55.684 | 1.00 | 21.32 | C |
| ATOM | 1368 | CD1 | LEU | A | 207 | 16.665 | 28.788 | 55.319 | 1.00 | 21.32 | C |
| ATOM | 1369 | CD2 | LEU | A | 207 | 17.652 | 27.420 | 57.172 | 1.00 | 21.32 | C |
| ATOM | 1370 | N | VAL | A | 208 | 15.109 | 23.268 | 55.691 | 1.00 | 39.57 | N |
| ATOM | 1371 | CA | VAL | A | 208 | 14.032 | 22.382 | 55.276 | 1.00 | 39.57 | C |
| ATOM | 1372 | C | VAL | A | 208 | 12.716 | 22.826 | 55.879 | 1.00 | 39.57 | C |
| ATOM | 1373 | O | VAL | A | 208 | 12.600 | 22.933 | 57.103 | 1.00 | 39.57 | O |
| ATOM | 1374 | CB | VAL | A | 208 | 14.284 | 20.961 | 55.733 | 1.00 | 29.39 | C |
| ATOM | 1375 | CG1 | VAL | A | 208 | 13.162 | 20.075 | 55.254 | 1.00 | 29.39 | C |
| ATOM | 1376 | CG2 | VAL | A | 208 | 15.627 | 20.495 | 55.226 | 1.00 | 29.39 | C |
| ATOM | 1377 | N | ARG | A | 209 | 11.721 | 23.077 | 55.029 | 1.00 | 40.70 | N |
| ATOM | 1378 | CA | ARG | A | 209 | 10.412 | 23.505 | 55.520 | 1.00 | 40.70 | C |
| ATOM | 1379 | C | ARG | A | 209 | 10.003 | 22.538 | 56.618 | 1.00 | 40.70 | C |
| ATOM | 1380 | O | ARG | A | 209 | 10.265 | 21.333 | 56.533 | 1.00 | 40.70 | O |
| ATOM | 1381 | CB | ARG | A | 209 | 9.368 | 23.483 | 54.398 | 1.00 | 85.10 | C |
| ATOM | 1382 | CG | ARG | A | 209 | 8.128 | 24.315 | 54.682 | 0.00 | 85.10 | C |
| ATOM | 1383 | CD | ARG | A | 209 | 7.075 | 24.073 | 53.620 | 0.00 | 85.10 | C |
| ATOM | 1384 | NE | ARG | A | 209 | 7.679 | 23.980 | 52.296 | 1.00 | 85.10 | N |
| ATOM | 1385 | CZ | ARG | A | 209 | 7.016 | 23.643 | 51.187 | 1.00 | 85.10 | C |
| ATOM | 1386 | NH1 | ARG | A | 209 | 5.709 | 23.369 | 51.240 | 1.00 | 85.10 | N |
| ATOM | 1387 | NH2 | ARG | A | 209 | 7.666 | 23.558 | 50.021 | 1.00 | 85.10 | N |
| ATOM | 1388 | N | GLY | A | 210 | 9.367 | 23.072 | 57.650 | 1.00 | 41.81 | N |
| ATOM | 1389 | CA | GLY | A | 210 | 8.960 | 22.243 | 58.764 | 1.00 | 41.81 | C |
| ATOM | 1390 | C | GLY | A | 210 | 10.007 | 22.300 | 59.858 | 1.00 | 41.81 | C |
| ATOM | 1391 | O | GLY | A | 210 | 9.668 | 22.477 | 61.024 | 1.00 | 41.81 | O |
| ATOM | 1392 | N | GLU | A | 211 | 11.279 | 22.160 | 59.488 | 1.00 | 54.48 | N |
| ATOM | 1393 | CA | GLU | A | 211 | 12.385 | 22.194 | 60.457 | 1.00 | 54.48 | C |
| ATOM | 1394 | C | GLU | A | 211 | 12.723 | 23.618 | 60.931 | 1.00 | 54.48 | C |
| ATOM | 1395 | O | GLU | A | 211 | 12.869 | 24.535 | 60.122 | 1.00 | 54.48 | O |
| ATOM | 1396 | CB | GLU | A | 211 | 13.649 | 21.548 | 59.859 | 1.00 | 81.01 | C |
| ATOM | 1397 | CG | GLU | A | 211 | 13.657 | 20.017 | 59.844 | 1.00 | 81.01 | C |
| ATOM | 1398 | CD | GLU | A | 211 | 14.987 | 19.435 | 59.329 | 1.00 | 81.01 | C |
| ATOM | 1399 | OE1 | GLU | A | 211 | 15.254 | 18.217 | 59.556 | 1.00 | 81.01 | O |
| ATOM | 1400 | OE2 | GLU | A | 211 | 15.764 | 20.201 | 58.696 | 1.00 | 81.01 | O |
| ATOM | 1401 | N | PRO | A | 212 | 12.859 | 23.808 | 62.258 | 1.00 | 40.07 | N |
| ATOM | 1402 | CA | PRO | A | 212 | 13.176 | 25.089 | 62.912 | 1.00 | 40.07 | C |
| ATOM | 1403 | C | PRO | A | 212 | 14.669 | 25.427 | 62.905 | 1.00 | 40.07 | C |
| ATOM | 1404 | O | PRO | A | 212 | 15.507 | 24.527 | 62.878 | 1.00 | 40.07 | O |
| ATOM | 1405 | CB | PRO | A | 212 | 12.657 | 24.869 | 64.325 | 1.00 | 38.64 | C |
| ATOM | 1406 | CG | PRO | A | 212 | 13.068 | 23.416 | 64.564 | 1.00 | 38.64 | C |
| ATOM | 1407 | CD | PRO | A | 212 | 12.631 | 22.750 | 63.266 | 1.00 | 38.64 | C |
| ATOM | 1408 | N | ASN | A | 213 | 14.999 | 26.716 | 62.964 | 1.00 | 28.50 | N |
| ATOM | 1409 | CA | ASN | A | 213 | 16.403 | 27.132 | 62.953 | 1.00 | 28.50 | C |
| ATOM | 1410 | C | ASN | A | 213 | 16.722 | 28.250 | 63.941 | 1.00 | 28.50 | C |
| ATOM | 1411 | O | ASN | A | 213 | 15.885 | 29.120 | 64.182 | 1.00 | 28.50 | O |
| ATOM | 1412 | CB | ASN | A | 213 | 16.771 | 27.582 | 61.551 | 1.00 | 27.76 | C |
| ATOM | 1413 | CG | ASN | A | 213 | 16.288 | 26.628 | 60.502 | 1.00 | 27.76 | C |
| ATOM | 1414 | OD1 | ASN | A | 213 | 16.830 | 25.535 | 60.346 | 1.00 | 27.76 | O |
| ATOM | 1415 | ND2 | ASN | A | 213 | 15.244 | 27.027 | 59.779 | 1.00 | 27.76 | N |
| ATOM | 1416 | N | VAL | A | 214 | 17.930 | 28.240 | 64.504 | 1.00 | 20.19 | N |
| ATOM | 1417 | CA | VAL | A | 214 | 18.294 | 29.282 | 65.463 | 1.00 | 20.19 | C |
| ATOM | 1418 | C | VAL | A | 214 | 18.167 | 30.634 | 64.835 | 1.00 | 20.19 | C |
| ATOM | 1419 | O | VAL | A | 214 | 18.460 | 30.816 | 63.660 | 1.00 | 20.19 | O |
| ATOM | 1420 | CB | VAL | A | 214 | 19.730 | 29.190 | 65.961 | 1.00 | 10.57 | C |
| ATOM | 1421 | CG1 | VAL | A | 214 | 19.873 | 27.959 | 66.868 | 1.00 | 10.57 | C |
| ATOM | 1422 | CG2 | VAL | A | 214 | 20.724 | 29.302 | 64.746 | 1.00 | 10.57 | C |

| | | | | | | | | | | | |
|---|---|---|---|---|---|---|---|---|---|---|---|
| ATOM | 1423 | N | SER | A | 215 | 17.758 | 31.599 | 65.636 | 1.00 | 27.64 | N |
| ATOM | 1424 | CA | SER | A | 215 | 17.486 | 32.908 | 65.054 | 1.00 | 27.64 | C |
| ATOM | 1425 | C | SER | A | 215 | 18.557 | 33.923 | 65.452 | 1.00 | 27.64 | C |
| ATOM | 1426 | O | SER | A | 215 | 18.393 | 35.131 | 65.345 | 1.00 | 27.64 | O |
| ATOM | 1427 | CB | SER | A | 215 | 16.109 | 33.377 | 65.532 | 1.00 | 34.41 | C |
| ATOM | 1428 | OG | SER | A | 215 | 16.138 | 33.573 | 66.945 | 1.00 | 34.41 | O |
| ATOM | 1429 | N | TYR | A | 216 | 19.674 | 33.381 | 65.967 | 1.00 | 27.22 | N |
| ATOM | 1430 | CA | TYR | A | 216 | 20.797 | 34.231 | 66.337 | 1.00 | 27.22 | C |
| ATOM | 1431 | C | TYR | A | 216 | 22.058 | 33.838 | 65.563 | 1.00 | 27.22 | C |
| ATOM | 1432 | O | TYR | A | 216 | 23.053 | 33.390 | 66.115 | 1.00 | 27.22 | O |
| ATOM | 1433 | CB | TYR | A | 216 | 21.038 | 34.067 | 67.838 | 1.00 | 34.54 | C |
| ATOM | 1434 | CG | TYR | A | 216 | 21.243 | 32.629 | 68.161 | 1.00 | 34.54 | C |
| ATOM | 1435 | CD1 | TYR | A | 216 | 22.340 | 31.949 | 67.638 | 1.00 | 34.54 | C |
| ATOM | 1436 | CD2 | TYR | A | 216 | 20.340 | 31.951 | 68.980 | 1.00 | 34.54 | C |
| ATOM | 1437 | CE1 | TYR | A | 216 | 22.534 | 30.607 | 67.931 | 1.00 | 34.54 | C |
| ATOM | 1438 | CE2 | TYR | A | 216 | 20.533 | 30.609 | 69.273 | 1.00 | 34.54 | C |
| ATOM | 1439 | CZ | TYR | A | 216 | 21.625 | 29.938 | 68.753 | 1.00 | 34.54 | C |
| ATOM | 1440 | OH | TYR | A | 216 | 21.848 | 28.613 | 69.075 | 1.00 | 34.54 | O |
| ATOM | 1441 | N | ILE | A | 217 | 21.975 | 33.983 | 64.228 | 1.00 | 41.65 | N |
| ATOM | 1442 | CA | ILE | A | 217 | 23.089 | 33.559 | 63.390 | 1.00 | 41.65 | C |
| ATOM | 1443 | C | ILE | A | 217 | 23.558 | 34.676 | 62.455 | 1.00 | 41.65 | C |
| ATOM | 1444 | O | ILE | A | 217 | 24.606 | 34.605 | 61.828 | 1.00 | 41.65 | O |
| ATOM | 1445 | CB | ILE | A | 217 | 22.626 | 32.356 | 62.568 | 1.00 | 29.43 | C |
| ATOM | 1446 | CG1 | ILE | A | 217 | 21.099 | 32.257 | 62.593 | 1.00 | 29.43 | C |
| ATOM | 1447 | CG2 | ILE | A | 217 | 23.192 | 31.058 | 63.171 | 1.00 | 29.43 | C |
| ATOM | 1448 | CD1 | ILE | A | 217 | 20.440 | 33.159 | 61.549 | 1.00 | 29.43 | C |
| ATOM | 1449 | N | CYS | A | 218 | 22.717 | 35.721 | 62.343 | 1.00 | 31.34 | N |
| ATOM | 1450 | CA | CYS | A | 218 | 23.042 | 36.804 | 61.423 | 1.00 | 31.34 | C |
| ATOM | 1451 | C | CYS | A | 218 | 23.754 | 37.961 | 62.125 | 1.00 | 31.34 | C |
| ATOM | 1452 | O | CYS | A | 218 | 23.629 | 38.180 | 63.324 | 1.00 | 31.34 | O |
| ATOM | 1453 | CB | CYS | A | 218 | 21.744 | 37.298 | 60.784 | 1.00 | 31.69 | C |
| ATOM | 1454 | SG | CYS | A | 218 | 22.030 | 38.092 | 59.187 | 1.00 | 31.69 | S |
| ATOM | 1455 | N | SER | A | 219 | 24.559 | 38.692 | 61.331 | 1.00 | 23.25 | N |
| ATOM | 1456 | CA | SER | A | 219 | 25.203 | 39.900 | 61.831 | 1.00 | 23.25 | C |
| ATOM | 1457 | C | SER | A | 219 | 24.177 | 40.929 | 62.312 | 1.00 | 23.25 | C |
| ATOM | 1458 | O | SER | A | 219 | 22.992 | 40.859 | 62.011 | 1.00 | 23.25 | O |
| ATOM | 1459 | CB | SER | A | 219 | 26.049 | 40.492 | 60.703 | 1.00 | 11.21 | C |
| ATOM | 1460 | OG | SER | A | 219 | 27.304 | 39.815 | 60.653 | 0.00 | 11.21 | O |
| ATOM | 1461 | N | ARG | A | 220 | 24.667 | 41.887 | 63.120 | 1.00 | 28.67 | N |
| ATOM | 1462 | CA | ARG | A | 220 | 23.767 | 42.891 | 63.679 | 1.00 | 28.67 | C |
| ATOM | 1463 | C | ARG | A | 220 | 23.107 | 43.748 | 62.593 | 1.00 | 28.67 | C |
| ATOM | 1464 | O | ARG | A | 220 | 21.920 | 43.645 | 62.316 | 1.00 | 28.67 | O |
| ATOM | 1465 | CB | ARG | A | 220 | 24.565 | 43.773 | 64.640 | 0.00 | 67.76 | C |
| ATOM | 1466 | CG | ARG | A | 220 | 24.656 | 43.163 | 66.039 | 0.00 | 67.76 | C |
| ATOM | 1467 | CD | ARG | A | 220 | 25.838 | 43.724 | 66.839 | 0.00 | 67.76 | C |
| ATOM | 1468 | NE | ARG | A | 220 | 25.543 | 45.078 | 67.317 | 1.00 | 67.76 | N |
| ATOM | 1469 | CZ | ARG | A | 220 | 26.088 | 46.105 | 66.637 | 1.00 | 67.76 | C |
| ATOM | 1470 | NH1 | ARG | A | 220 | 26.846 | 45.872 | 65.579 | 0.00 | 67.76 | N |
| ATOM | 1471 | NH2 | ARG | A | 220 | 25.860 | 47.359 | 67.039 | 1.00 | 67.76 | N |
| ATOM | 1472 | N | TYR | A | 221 | 23.914 | 44.647 | 61.997 | 1.00 | 20.20 | N |
| ATOM | 1473 | CA | TYR | A | 221 | 23.368 | 45.518 | 60.960 | 1.00 | 20.20 | C |
| ATOM | 1474 | C | TYR | A | 221 | 22.770 | 44.711 | 59.803 | 1.00 | 20.20 | C |
| ATOM | 1475 | O | TYR | A | 221 | 22.145 | 45.246 | 58.897 | 1.00 | 20.20 | O |
| ATOM | 1476 | CB | TYR | A | 221 | 24.492 | 46.420 | 60.445 | 1.00 | 47.15 | C |
| ATOM | 1477 | CG | TYR | A | 221 | 25.111 | 47.157 | 61.578 | 1.00 | 47.15 | C |
| ATOM | 1478 | CD1 | TYR | A | 221 | 24.347 | 47.484 | 62.695 | 1.00 | 47.15 | C |
| ATOM | 1479 | CD2 | TYR | A | 221 | 26.462 | 47.503 | 61.541 | 1.00 | 47.15 | C |
| ATOM | 1480 | CE1 | TYR | A | 221 | 24.929 | 48.145 | 63.768 | 1.00 | 47.15 | C |
| ATOM | 1481 | CE2 | TYR | A | 221 | 27.043 | 48.163 | 62.613 | 1.00 | 47.15 | C |
| ATOM | 1482 | CZ | TYR | A | 221 | 26.284 | 48.481 | 63.725 | 1.00 | 47.15 | C |
| ATOM | 1483 | OH | TYR | A | 221 | 26.870 | 49.083 | 64.822 | 1.00 | 47.15 | O |
| ATOM | 1484 | N | TYR | A | 222 | 22.817 | 43.375 | 59.655 | 1.00 | 23.68 | N |

| | | | | | | | | | | | |
|---|---|---|---|---|---|---|---|---|---|---|---|
| ATOM | 1485 | CA | TYR | A | 222 | 22.209 | 42.776 | 58.469 | 1.00 | 23.68 | C |
| ATOM | 1486 | C | TYR | A | 222 | 21.126 | 41.754 | 58.822 | 1.00 | 23.68 | C |
| ATOM | 1487 | O | TYR | A | 222 | 20.614 | 41.035 | 57.973 | 1.00 | 23.68 | O |
| ATOM | 1488 | CB | TYR | A | 222 | 23.315 | 42.110 | 57.649 | 1.00 | 22.29 | C |
| ATOM | 1489 | CG | TYR | A | 222 | 24.478 | 43.031 | 57.538 | 1.00 | 22.29 | C |
| ATOM | 1490 | CD1 | TYR | A | 222 | 25.552 | 42.900 | 58.415 | 1.00 | 22.29 | C |
| ATOM | 1491 | CD2 | TYR | A | 222 | 24.455 | 44.091 | 56.631 | 1.00 | 22.29 | C |
| ATOM | 1492 | CE1 | TYR | A | 222 | 26.586 | 43.825 | 58.395 | 1.00 | 22.29 | C |
| ATOM | 1493 | CE2 | TYR | A | 222 | 25.487 | 45.017 | 56.612 | 1.00 | 22.29 | C |
| ATOM | 1494 | CZ | TYR | A | 222 | 26.546 | 44.889 | 57.492 | 1.00 | 22.29 | C |
| ATOM | 1495 | OH | TYR | A | 222 | 27.571 | 45.815 | 57.488 | 1.00 | 22.29 | O |
| ATOM | 1496 | N | ARG | A | 223 | 20.715 | 41.632 | 60.077 | 1.00 | 28.31 | N |
| ATOM | 1497 | CA | ARG | A | 223 | 19.701 | 40.658 | 60.443 | 1.00 | 28.31 | C |
| ATOM | 1498 | C | ARG | A | 223 | 18.345 | 41.086 | 59.935 | 1.00 | 28.31 | C |
| ATOM | 1499 | O | ARG | A | 223 | 17.962 | 42.247 | 60.048 | 1.00 | 28.31 | O |
| ATOM | 1500 | CB | ARG | A | 223 | 19.627 | 40.500 | 61.953 | 1.00 | 36.69 | C |
| ATOM | 1501 | CG | ARG | A | 223 | 20.971 | 40.587 | 62.639 | 1.00 | 36.69 | C |
| ATOM | 1502 | CD | ARG | A | 223 | 20.837 | 40.282 | 64.119 | 1.00 | 36.69 | C |
| ATOM | 1503 | NE | ARG | A | 223 | 20.397 | 38.899 | 64.298 | 1.00 | 36.69 | N |
| ATOM | 1504 | CZ | ARG | A | 223 | 19.459 | 38.514 | 65.155 | 1.00 | 36.69 | C |
| ATOM | 1505 | NH1 | ARG | A | 223 | 18.856 | 39.405 | 65.924 | 1.00 | 36.69 | N |
| ATOM | 1506 | NH2 | ARG | A | 223 | 19.112 | 37.244 | 65.221 | 1.00 | 36.69 | N |
| ATOM | 1507 | N | ALA | A | 224 | 17.626 | 40.137 | 59.359 | 1.00 | 24.40 | N |
| ATOM | 1508 | CA | ALA | A | 224 | 16.295 | 40.398 | 58.859 | 1.00 | 24.40 | C |
| ATOM | 1509 | C | ALA | A | 224 | 15.353 | 40.555 | 60.056 | 1.00 | 24.40 | C |
| ATOM | 1510 | O | ALA | A | 224 | 15.523 | 39.906 | 61.091 | 1.00 | 24.40 | O |
| ATOM | 1511 | CB | ALA | A | 224 | 15.845 | 39.244 | 57.980 | 1.00 | 16.64 | C |
| ATOM | 1512 | N | PRO | A | 225 | 14.347 | 41.424 | 59.928 | 1.00 | 28.21 | N |
| ATOM | 1513 | CA | PRO | A | 225 | 13.382 | 41.656 | 61.005 | 1.00 | 28.21 | C |
| ATOM | 1514 | C | PRO | A | 225 | 12.925 | 40.389 | 61.726 | 1.00 | 28.21 | C |
| ATOM | 1515 | O | PRO | A | 225 | 12.790 | 40.377 | 62.949 | 1.00 | 28.21 | O |
| ATOM | 1516 | CB | PRO | A | 225 | 12.234 | 42.373 | 60.292 | 1.00 | 27.42 | C |
| ATOM | 1517 | CG | PRO | A | 225 | 12.502 | 42.144 | 58.800 | 1.00 | 27.42 | C |
| ATOM | 1518 | CD | PRO | A | 225 | 13.978 | 42.181 | 58.724 | 1.00 | 27.42 | C |
| ATOM | 1519 | N | GLU | A | 226 | 12.692 | 39.325 | 60.962 | 1.00 | 36.44 | N |
| ATOM | 1520 | CA | GLU | A | 226 | 12.262 | 38.043 | 61.517 | 1.00 | 36.44 | C |
| ATOM | 1521 | C | GLU | A | 226 | 13.296 | 37.519 | 62.514 | 1.00 | 36.44 | C |
| ATOM | 1522 | O | GLU | A | 226 | 12.961 | 37.053 | 63.608 | 1.00 | 36.44 | O |
| ATOM | 1523 | CB | GLU | A | 226 | 12.103 | 37.012 | 60.408 | 1.00 | 33.70 | C |
| ATOM | 1524 | CG | GLU | A | 226 | 11.499 | 37.565 | 59.156 | 1.00 | 33.70 | C |
| ATOM | 1525 | CD | GLU | A | 226 | 12.524 | 37.794 | 58.075 | 1.00 | 33.70 | C |
| ATOM | 1526 | OE1 | GLU | A | 226 | 12.969 | 36.799 | 57.448 | 1.00 | 33.70 | O |
| ATOM | 1527 | OE2 | GLU | A | 226 | 12.883 | 38.971 | 57.865 | 1.00 | 33.70 | O |
| ATOM | 1528 | N | LEU | A | 227 | 14.561 | 37.571 | 62.123 | 1.00 | 28.87 | N |
| ATOM | 1529 | CA | LEU | A | 227 | 15.597 | 37.115 | 63.014 | 1.00 | 28.87 | C |
| ATOM | 1530 | C | LEU | A | 227 | 15.503 | 37.948 | 64.291 | 1.00 | 28.87 | C |
| ATOM | 1531 | O | LEU | A | 227 | 15.635 | 37.423 | 65.385 | 1.00 | 28.87 | O |
| ATOM | 1532 | CB | LEU | A | 227 | 16.961 | 37.278 | 62.339 | 1.00 | 20.76 | C |
| ATOM | 1533 | CG | LEU | A | 227 | 17.100 | 36.496 | 61.027 | 1.00 | 20.76 | C |
| ATOM | 1534 | CD1 | LEU | A | 227 | 18.488 | 36.720 | 60.459 | 1.00 | 20.76 | C |
| ATOM | 1535 | CD2 | LEU | A | 227 | 16.841 | 35.009 | 61.259 | 1.00 | 20.76 | C |
| ATOM | 1536 | N | ILE | A | 228 | 15.244 | 39.244 | 64.136 | 1.00 | 25.06 | N |
| ATOM | 1537 | CA | ILE | A | 228 | 15.138 | 40.168 | 65.259 | 1.00 | 25.06 | C |
| ATOM | 1538 | C | ILE | A | 228 | 13.943 | 39.810 | 66.118 | 1.00 | 25.06 | C |
| ATOM | 1539 | O | ILE | A | 228 | 13.978 | 39.924 | 67.336 | 1.00 | 25.06 | O |
| ATOM | 1540 | CB | ILE | A | 228 | 14.978 | 41.619 | 64.770 | 1.00 | 24.30 | C |
| ATOM | 1541 | CG1 | ILE | A | 228 | 16.204 | 42.027 | 63.945 | 1.00 | 24.30 | C |
| ATOM | 1542 | CG2 | ILE | A | 228 | 14.785 | 42.548 | 65.960 | 1.00 | 24.30 | C |
| ATOM | 1543 | CD1 | ILE | A | 228 | 16.122 | 43.415 | 63.307 | 1.00 | 24.30 | C |
| ATOM | 1544 | N | PHE | A | 229 | 12.875 | 39.390 | 65.466 | 1.00 | 20.97 | N |
| ATOM | 1545 | CA | PHE | A | 229 | 11.680 | 38.985 | 66.171 | 1.00 | 20.97 | C |
| ATOM | 1546 | C | PHE | A | 229 | 11.830 | 37.548 | 66.670 | 1.00 | 20.97 | C |

| | | | | | | | | | | | |
|---|---|---|---|---|---|---|---|---|---|---|---|
| ATOM | 1547 | O | PHE | A | 229 | 10.879 | 36.968 | 67.166 | 1.00 | 20.97 | O |
| ATOM | 1548 | CB | PHE | A | 229 | 10.456 | 39.087 | 65.259 | 1.00 | 31.34 | C |
| ATOM | 1549 | CG | PHE | A | 229 | 9.930 | 40.480 | 65.104 | 1.00 | 31.34 | C |
| ATOM | 1550 | CD1 | PHE | A | 229 | 9.581 | 41.227 | 66.219 | 1.00 | 31.34 | C |
| ATOM | 1551 | CD2 | PHE | A | 229 | 9.770 | 41.046 | 63.840 | 1.00 | 31.34 | C |
| ATOM | 1552 | CE1 | PHE | A | 229 | 9.081 | 42.524 | 66.083 | 1.00 | 31.34 | C |
| ATOM | 1553 | CE2 | PHE | A | 229 | 9.272 | 42.341 | 63.694 | 1.00 | 31.34 | C |
| ATOM | 1554 | CZ | PHE | A | 229 | 8.928 | 43.081 | 64.816 | 1.00 | 31.34 | C |
| ATOM | 1555 | N | GLY | A | 230 | 13.020 | 36.978 | 66.529 | 1.00 | 25.31 | N |
| ATOM | 1556 | CA | GLY | A | 230 | 13.251 | 35.621 | 66.999 | 1.00 | 25.31 | C |
| ATOM | 1557 | C | GLY | A | 230 | 12.540 | 34.473 | 66.277 | 1.00 | 25.31 | C |
| ATOM | 1558 | O | GLY | A | 230 | 12.422 | 33.379 | 66.835 | 1.00 | 25.31 | O |
| ATOM | 1559 | N | ALA | A | 231 | 12.069 | 34.703 | 65.051 | 1.00 | 21.06 | N |
| ATOM | 1560 | CA | ALA | A | 231 | 11.406 | 33.657 | 64.275 | 1.00 | 21.06 | C |
| ATOM | 1561 | C | ALA | A | 231 | 12.369 | 32.492 | 64.085 | 1.00 | 21.06 | C |
| ATOM | 1562 | O | ALA | A | 231 | 13.576 | 32.698 | 63.997 | 1.00 | 21.06 | O |
| ATOM | 1563 | CB | ALA | A | 231 | 10.976 | 34.202 | 62.915 | 1.00 | 14.27 | C |
| ATOM | 1564 | N | THR | A | 232 | 11.843 | 31.272 | 64.033 | 1.00 | 25.78 | N |
| ATOM | 1565 | CA | THR | A | 232 | 12.701 | 30.106 | 63.849 | 1.00 | 25.78 | C |
| ATOM | 1566 | C | THR | A | 232 | 12.293 | 29.338 | 62.605 | 1.00 | 25.78 | C |
| ATOM | 1567 | O | THR | A | 232 | 12.861 | 28.290 | 62.290 | 1.00 | 25.78 | O |
| ATOM | 1568 | CB | THR | A | 232 | 12.645 | 29.150 | 65.054 | 1.00 | 38.77 | C |
| ATOM | 1569 | OG1 | THR | A | 232 | 11.361 | 28.521 | 65.115 | 1.00 | 38.77 | O |
| ATOM | 1570 | CG2 | THR | A | 232 | 12.890 | 29.910 | 66.337 | 1.00 | 38.77 | C |
| ATOM | 1571 | N | ASP | A | 233 | 11.308 | 29.881 | 61.900 | 1.00 | 29.45 | N |
| ATOM | 1572 | CA | ASP | A | 233 | 10.795 | 29.289 | 60.670 | 1.00 | 29.45 | C |
| ATOM | 1573 | C | ASP | A | 233 | 11.153 | 30.180 | 59.476 | 1.00 | 29.45 | C |
| ATOM | 1574 | O | ASP | A | 233 | 10.468 | 30.161 | 58.454 | 1.00 | 29.45 | O |
| ATOM | 1575 | CB | ASP | A | 233 | 9.273 | 29.161 | 60.760 | 1.00 | 35.57 | C |
| ATOM | 1576 | CG | ASP | A | 233 | 8.592 | 30.509 | 60.852 | 1.00 | 35.57 | C |
| ATOM | 1577 | OD1 | ASP | A | 233 | 9.239 | 31.464 | 61.316 | 1.00 | 35.57 | O |
| ATOM | 1578 | OD2 | ASP | A | 233 | 7.415 | 30.622 | 60.475 | 1.00 | 35.57 | O |
| ATOM | 1579 | N | TYR | A | 234 | 12.213 | 30.969 | 59.610 | 1.00 | 23.50 | N |
| ATOM | 1580 | CA | TYR | A | 234 | 12.622 | 31.848 | 58.524 | 1.00 | 23.50 | C |
| ATOM | 1581 | C | TYR | A | 234 | 13.056 | 31.070 | 57.285 | 1.00 | 23.50 | C |
| ATOM | 1582 | O | TYR | A | 234 | 13.426 | 29.896 | 57.361 | 1.00 | 23.50 | O |
| ATOM | 1583 | CB | TYR | A | 234 | 13.738 | 32.791 | 58.987 | 1.00 | 16.93 | C |
| ATOM | 1584 | CG | TYR | A | 234 | 14.973 | 32.120 | 59.545 | 1.00 | 16.93 | C |
| ATOM | 1585 | CD1 | TYR | A | 234 | 15.953 | 31.609 | 58.704 | 1.00 | 16.93 | C |
| ATOM | 1586 | CD2 | TYR | A | 234 | 15.170 | 32.017 | 60.916 | 1.00 | 16.93 | C |
| ATOM | 1587 | CE1 | TYR | A | 234 | 17.092 | 31.016 | 59.214 | 1.00 | 16.93 | C |
| ATOM | 1588 | CE2 | TYR | A | 234 | 16.307 | 31.423 | 61.434 | 1.00 | 16.93 | C |
| ATOM | 1589 | CZ | TYR | A | 234 | 17.261 | 30.924 | 60.581 | 1.00 | 16.93 | C |
| ATOM | 1590 | OH | TYR | A | 234 | 18.364 | 30.299 | 61.097 | 1.00 | 16.93 | O |
| ATOM | 1591 | N | THR | A | 235 | 12.990 | 31.734 | 56.139 | 1.00 | 28.45 | N |
| ATOM | 1592 | CA | THR | A | 235 | 13.360 | 31.128 | 54.873 | 1.00 | 28.45 | C |
| ATOM | 1593 | C | THR | A | 235 | 14.672 | 31.711 | 54.432 | 1.00 | 28.45 | C |
| ATOM | 1594 | O | THR | A | 235 | 15.094 | 32.748 | 54.937 | 1.00 | 28.45 | O |
| ATOM | 1595 | CB | THR | A | 235 | 12.335 | 31.441 | 53.800 | 1.00 | 25.35 | C |
| ATOM | 1596 | OG1 | THR | A | 235 | 12.297 | 32.856 | 53.584 | 1.00 | 25.35 | O |
| ATOM | 1597 | CG2 | THR | A | 235 | 10.969 | 30.983 | 54.234 | 1.00 | 25.35 | C |
| ATOM | 1598 | N | SER | A | 236 | 15.313 | 31.062 | 53.471 | 1.00 | 27.63 | N |
| ATOM | 1599 | CA | SER | A | 236 | 16.593 | 31.554 | 52.991 | 1.00 | 27.63 | C |
| ATOM | 1600 | C | SER | A | 236 | 16.502 | 32.981 | 52.447 | 1.00 | 27.63 | C |
| ATOM | 1601 | O | SER | A | 236 | 17.507 | 33.550 | 52.052 | 1.00 | 27.63 | O |
| ATOM | 1602 | CB | SER | A | 236 | 17.159 | 30.625 | 51.920 | 1.00 | 18.08 | C |
| ATOM | 1603 | OG | SER | A | 236 | 16.497 | 30.813 | 50.696 | 1.00 | 18.08 | O |
| ATOM | 1604 | N | SER | A | 237 | 15.315 | 33.575 | 52.435 | 1.00 | 27.48 | N |
| ATOM | 1605 | CA | SER | A | 237 | 15.233 | 34.925 | 51.918 | 1.00 | 27.48 | C |
| ATOM | 1606 | C | SER | A | 237 | 15.835 | 35.942 | 52.879 | 1.00 | 27.48 | C |
| ATOM | 1607 | O | SER | A | 237 | 15.849 | 37.139 | 52.588 | 1.00 | 27.48 | O |
| ATOM | 1608 | CB | SER | A | 237 | 13.795 | 35.314 | 51.560 | 1.00 | 24.33 | C |

| | | | | | | | | | | | |
|---|---|---|---|---|---|---|---|---|---|---|---|
| ATOM | 1609 | OG | SER | A | 237 | 13.001 | 35.535 | 52.702 | 1.00 | 24.33 | O |
| ATOM | 1610 | N | ILE | A | 238 | 16.331 | 35.481 | 54.025 | 1.00 | 29.83 | N |
| ATOM | 1611 | CA | ILE | A | 238 | 16.966 | 36.407 | 54.956 | 1.00 | 29.83 | C |
| ATOM | 1612 | C | ILE | A | 238 | 18.305 | 36.855 | 54.347 | 1.00 | 29.83 | C |
| ATOM | 1613 | O | ILE | A | 238 | 18.841 | 37.895 | 54.706 | 1.00 | 29.83 | O |
| ATOM | 1614 | CB | ILE | A | 238 | 17.233 | 35.776 | 56.343 | 1.00 | 23.63 | C |
| ATOM | 1615 | CG1 | ILE | A | 238 | 18.061 | 34.508 | 56.198 | 1.00 | 23.63 | C |
| ATOM | 1616 | CG2 | ILE | A | 238 | 15.936 | 35.494 | 57.044 | 1.00 | 23.63 | C |
| ATOM | 1617 | CD1 | ILE | A | 238 | 18.605 | 34.016 | 57.513 | 1.00 | 23.63 | C |
| ATOM | 1618 | N | ASP | A | 239 | 18.838 | 36.054 | 53.428 | 1.00 | 27.51 | N |
| ATOM | 1619 | CA | ASP | A | 239 | 20.077 | 36.381 | 52.743 | 1.00 | 27.51 | C |
| ATOM | 1620 | C | ASP | A | 239 | 19.827 | 37.573 | 51.833 | 1.00 | 27.51 | C |
| ATOM | 1621 | O | ASP | A | 239 | 20.704 | 38.409 | 51.627 | 1.00 | 27.51 | O |
| ATOM | 1622 | CB | ASP | A | 239 | 20.563 | 35.209 | 51.898 | 1.00 | 21.27 | C |
| ATOM | 1623 | CG | ASP | A | 239 | 21.273 | 34.163 | 52.713 | 1.00 | 21.27 | C |
| ATOM | 1624 | OD1 | ASP | A | 239 | 21.701 | 34.480 | 53.840 | 1.00 | 21.27 | O |
| ATOM | 1625 | OD2 | ASP | A | 239 | 21.421 | 33.027 | 52.218 | 1.00 | 21.27 | O |
| ATOM | 1626 | N | VAL | A | 240 | 18.620 | 37.654 | 51.293 | 1.00 | 23.41 | N |
| ATOM | 1627 | CA | VAL | A | 240 | 18.275 | 38.756 | 50.406 | 1.00 | 23.41 | C |
| ATOM | 1628 | C | VAL | A | 240 | 18.175 | 40.088 | 51.158 | 1.00 | 23.41 | C |
| ATOM | 1629 | O | VAL | A | 240 | 18.592 | 41.127 | 50.653 | 1.00 | 23.41 | O |
| ATOM | 1630 | CB | VAL | A | 240 | 16.961 | 38.454 | 49.669 | 1.00 | 19.10 | C |
| ATOM | 1631 | CG1 | VAL | A | 240 | 16.540 | 39.637 | 48.821 | 1.00 | 19.10 | C |
| ATOM | 1632 | CG2 | VAL | A | 240 | 17.149 | 37.224 | 48.803 | 1.00 | 19.10 | C |
| ATOM | 1633 | N | TRP | A | 241 | 17.629 | 40.041 | 52.372 | 1.00 | 22.76 | N |
| ATOM | 1634 | CA | TRP | A | 241 | 17.485 | 41.224 | 53.202 | 1.00 | 22.76 | C |
| ATOM | 1635 | C | TRP | A | 241 | 18.878 | 41.777 | 53.473 | 1.00 | 22.76 | C |
| ATOM | 1636 | O | TRP | A | 241 | 19.139 | 42.964 | 53.295 | 1.00 | 22.76 | O |
| ATOM | 1637 | CB | TRP | A | 241 | 16.777 | 40.862 | 54.521 | 1.00 | 20.47 | C |
| ATOM | 1638 | CG | TRP | A | 241 | 16.707 | 42.011 | 55.513 | 1.00 | 20.47 | C |
| ATOM | 1639 | CD1 | TRP | A | 241 | 17.689 | 42.406 | 56.369 | 1.00 | 20.47 | C |
| ATOM | 1640 | CD2 | TRP | A | 241 | 15.647 | 42.967 | 55.656 | 1.00 | 20.47 | C |
| ATOM | 1641 | NE1 | TRP | A | 241 | 17.320 | 43.542 | 57.025 | 1.00 | 20.47 | N |
| ATOM | 1642 | CE2 | TRP | A | 241 | 16.072 | 43.912 | 56.613 | 1.00 | 20.47 | C |
| ATOM | 1643 | CE3 | TRP | A | 241 | 14.385 | 43.118 | 55.066 | 1.00 | 20.47 | C |
| ATOM | 1644 | CZ2 | TRP | A | 241 | 15.276 | 45.005 | 56.998 | 1.00 | 20.47 | C |
| ATOM | 1645 | CZ3 | TRP | A | 241 | 13.593 | 44.206 | 55.447 | 1.00 | 20.47 | C |
| ATOM | 1646 | CH2 | TRP | A | 241 | 14.044 | 45.132 | 56.406 | 1.00 | 20.47 | C |
| ATOM | 1647 | N | SER | A | 242 | 19.767 | 40.892 | 53.897 | 1.00 | 23.50 | N |
| ATOM | 1648 | CA | SER | A | 242 | 21.140 | 41.257 | 54.188 | 1.00 | 23.50 | C |
| ATOM | 1649 | C | SER | A | 242 | 21.789 | 41.915 | 52.989 | 1.00 | 23.50 | C |
| ATOM | 1650 | O | SER | A | 242 | 22.464 | 42.931 | 53.125 | 1.00 | 23.50 | O |
| ATOM | 1651 | CB | SER | A | 242 | 21.951 | 40.020 | 54.559 | 1.00 | 29.39 | C |
| ATOM | 1652 | OG | SER | A | 242 | 21.385 | 39.370 | 55.673 | 1.00 | 29.39 | O |
| ATOM | 1653 | N | ALA | A | 243 | 21.589 | 41.311 | 51.819 | 1.00 | 26.63 | N |
| ATOM | 1654 | CA | ALA | A | 243 | 22.159 | 41.813 | 50.586 | 1.00 | 26.63 | C |
| ATOM | 1655 | C | ALA | A | 243 | 21.642 | 43.218 | 50.348 | 1.00 | 26.63 | C |
| ATOM | 1656 | O | ALA | A | 243 | 22.400 | 44.111 | 49.976 | 1.00 | 26.63 | O |
| ATOM | 1657 | CB | ALA | A | 243 | 21.785 | 40.905 | 49.436 | 1.00 | 21.53 | C |
| ATOM | 1658 | N | GLY | A | 244 | 20.347 | 43.413 | 50.564 | 1.00 | 28.52 | N |
| ATOM | 1659 | CA | GLY | A | 244 | 19.781 | 44.732 | 50.389 | 1.00 | 28.52 | C |
| ATOM | 1660 | C | GLY | A | 244 | 20.447 | 45.715 | 51.338 | 1.00 | 28.52 | C |
| ATOM | 1661 | O | GLY | A | 244 | 20.545 | 46.900 | 51.045 | 1.00 | 28.52 | O |
| ATOM | 1662 | N | CYS | A | 245 | 20.907 | 45.216 | 52.481 | 1.00 | 29.37 | N |
| ATOM | 1663 | CA | CYS | A | 245 | 21.577 | 46.046 | 53.474 | 1.00 | 29.37 | C |
| ATOM | 1664 | C | CYS | A | 245 | 22.977 | 46.407 | 53.020 | 1.00 | 29.37 | C |
| ATOM | 1665 | O | CYS | A | 245 | 23.465 | 47.503 | 53.287 | 1.00 | 29.37 | O |
| ATOM | 1666 | CB | CYS | A | 245 | 21.637 | 45.324 | 54.823 | 1.00 | 20.33 | C |
| ATOM | 1667 | SG | CYS | A | 245 | 20.102 | 45.379 | 55.736 | 1.00 | 20.33 | S |
| ATOM | 1668 | N | VAL | A | 246 | 23.629 | 45.473 | 52.342 | 1.00 | 21.92 | N |
| ATOM | 1669 | CA | VAL | A | 246 | 24.964 | 45.714 | 51.824 | 1.00 | 21.92 | C |
| ATOM | 1670 | C | VAL | A | 246 | 24.867 | 46.794 | 50.751 | 1.00 | 21.92 | C |

| | | | | | | | | | | | |
|---|---|---|---|---|---|---|---|---|---|---|---|
| ATOM | 1671 | O | VAL | A | 246 | 25.639 | 47.743 | 50.744 | 1.00 | 21.92 | O |
| ATOM | 1672 | CB | VAL | A | 246 | 25.551 | 44.433 | 51.210 | 1.00 | 25.13 | C |
| ATOM | 1673 | CG1 | VAL | A | 246 | 26.869 | 44.736 | 50.514 | 1.00 | 25.13 | C |
| ATOM | 1674 | CG2 | VAL | A | 246 | 25.746 | 43.400 | 52.299 | 1.00 | 25.13 | C |
| ATOM | 1675 | N | LEU | A | 247 | 23.886 | 46.635 | 49.868 | 1.00 | 20.11 | N |
| ATOM | 1676 | CA | LEU | A | 247 | 23.630 | 47.555 | 48.771 | 1.00 | 20.11 | C |
| ATOM | 1677 | C | LEU | A | 247 | 23.345 | 48.955 | 49.281 | 1.00 | 20.11 | C |
| ATOM | 1678 | O | LEU | A | 247 | 23.983 | 49.923 | 48.876 | 1.00 | 20.11 | O |
| ATOM | 1679 | CB | LEU | A | 247 | 22.447 | 47.052 | 47.956 | 1.00 | 25.29 | C |
| ATOM | 1680 | CG | LEU | A | 247 | 21.822 | 48.030 | 46.965 | 1.00 | 25.29 | C |
| ATOM | 1681 | CD1 | LEU | A | 247 | 22.832 | 48.486 | 45.909 | 1.00 | 25.29 | C |
| ATOM | 1682 | CD2 | LEU | A | 247 | 20.633 | 47.344 | 46.323 | 1.00 | 25.29 | C |
| ATOM | 1683 | N | ALA | A | 248 | 22.368 | 49.048 | 50.170 | 1.00 | 27.36 | N |
| ATOM | 1684 | CA | ALA | A | 248 | 22.000 | 50.314 | 50.768 | 1.00 | 27.36 | C |
| ATOM | 1685 | C | ALA | A | 248 | 23.207 | 50.944 | 51.464 | 1.00 | 27.36 | C |
| ATOM | 1686 | O | ALA | A | 248 | 23.389 | 52.155 | 51.419 | 1.00 | 27.36 | O |
| ATOM | 1687 | CB | ALA | A | 248 | 20.891 | 50.099 | 51.763 | 1.00 | 10.48 | C |
| ATOM | 1688 | N | GLU | A | 249 | 24.031 | 50.124 | 52.108 | 1.00 | 21.02 | N |
| ATOM | 1689 | CA | GLU | A | 249 | 25.198 | 50.643 | 52.794 | 1.00 | 21.02 | C |
| ATOM | 1690 | C | GLU | A | 249 | 26.215 | 51.205 | 51.810 | 1.00 | 21.02 | C |
| ATOM | 1691 | O | GLU | A | 249 | 26.838 | 52.229 | 52.070 | 1.00 | 21.02 | O |
| ATOM | 1692 | CB | GLU | A | 249 | 25.839 | 49.547 | 53.642 | 1.00 | 24.29 | C |
| ATOM | 1693 | CG | GLU | A | 249 | 26.943 | 50.043 | 54.549 | 1.00 | 24.29 | C |
| ATOM | 1694 | CD | GLU | A | 249 | 27.495 | 48.952 | 55.424 | 1.00 | 24.29 | C |
| ATOM | 1695 | OE1 | GLU | A | 249 | 26.685 | 48.153 | 55.928 | 1.00 | 24.29 | O |
| ATOM | 1696 | OE2 | GLU | A | 249 | 28.727 | 48.905 | 55.623 | 1.00 | 24.29 | O |
| ATOM | 1697 | N | LEU | A | 250 | 26.387 | 50.524 | 50.682 | 1.00 | 28.26 | N |
| ATOM | 1698 | CA | LEU | A | 250 | 27.319 | 50.960 | 49.653 | 1.00 | 28.26 | C |
| ATOM | 1699 | C | LEU | A | 250 | 26.929 | 52.326 | 49.104 | 1.00 | 28.26 | C |
| ATOM | 1700 | O | LEU | A | 250 | 27.781 | 53.156 | 48.814 | 1.00 | 28.26 | O |
| ATOM | 1701 | CB | LEU | A | 250 | 27.350 | 49.951 | 48.515 | 1.00 | 19.31 | C |
| ATOM | 1702 | CG | LEU | A | 250 | 27.995 | 48.599 | 48.812 | 1.00 | 19.31 | C |
| ATOM | 1703 | CD1 | LEU | A | 250 | 28.001 | 47.749 | 47.563 | 1.00 | 19.31 | C |
| ATOM | 1704 | CD2 | LEU | A | 250 | 29.401 | 48.816 | 49.316 | 1.00 | 19.31 | C |
| ATOM | 1705 | N | LEU | A | 251 | 25.629 | 52.552 | 48.972 | 1.00 | 28.98 | N |
| ATOM | 1706 | CA | LEU | A | 251 | 25.111 | 53.802 | 48.459 | 1.00 | 28.98 | C |
| ATOM | 1707 | C | LEU | A | 251 | 25.101 | 54.914 | 49.490 | 1.00 | 28.98 | C |
| ATOM | 1708 | O | LEU | A | 251 | 25.218 | 56.077 | 49.135 | 1.00 | 28.98 | O |
| ATOM | 1709 | CB | LEU | A | 251 | 23.687 | 53.604 | 47.951 | 1.00 | 14.16 | C |
| ATOM | 1710 | CG | LEU | A | 251 | 23.474 | 52.537 | 46.882 | 1.00 | 14.16 | C |
| ATOM | 1711 | CD1 | LEU | A | 251 | 21.991 | 52.316 | 46.676 | 1.00 | 14.16 | C |
| ATOM | 1712 | CD2 | LEU | A | 251 | 24.160 | 52.942 | 45.601 | 1.00 | 14.16 | C |
| ATOM | 1713 | N | LEU | A | 252 | 24.967 | 54.562 | 50.763 | 1.00 | 28.99 | N |
| ATOM | 1714 | CA | LEU | A | 252 | 24.895 | 55.546 | 51.838 | 1.00 | 28.99 | C |
| ATOM | 1715 | C | LEU | A | 252 | 26.224 | 55.896 | 52.503 | 1.00 | 28.99 | C |
| ATOM | 1716 | O | LEU | A | 252 | 26.389 | 56.994 | 53.021 | 1.00 | 28.99 | O |
| ATOM | 1717 | CB | LEU | A | 252 | 23.908 | 55.060 | 52.902 | 1.00 | 27.47 | C |
| ATOM | 1718 | CG | LEU | A | 252 | 22.882 | 56.045 | 53.473 | 1.00 | 27.47 | C |
| ATOM | 1719 | CD1 | LEU | A | 252 | 22.028 | 56.611 | 52.353 | 0.00 | 27.47 | C |
| ATOM | 1720 | CD2 | LEU | A | 252 | 22.018 | 55.338 | 54.504 | 0.00 | 27.47 | C |
| ATOM | 1721 | N | GLY | A | 253 | 27.178 | 54.978 | 52.499 | 1.00 | 27.31 | N |
| ATOM | 1722 | CA | GLY | A | 253 | 28.456 | 55.263 | 53.131 | 1.00 | 27.31 | C |
| ATOM | 1723 | C | GLY | A | 253 | 28.508 | 54.802 | 54.580 | 1.00 | 27.31 | C |
| ATOM | 1724 | O | GLY | A | 253 | 29.527 | 54.940 | 55.256 | 1.00 | 27.31 | O |
| ATOM | 1725 | N | GLN | A | 254 | 27.398 | 54.247 | 55.048 | 1.00 | 27.88 | N |
| ATOM | 1726 | CA | GLN | A | 254 | 27.274 | 53.749 | 56.412 | 1.00 | 27.88 | C |
| ATOM | 1727 | C | GLN | A | 254 | 26.091 | 52.786 | 56.422 | 1.00 | 27.88 | C |
| ATOM | 1728 | O | GLN | A | 254 | 25.270 | 52.813 | 55.508 | 1.00 | 27.88 | O |
| ATOM | 1729 | CB | GLN | A | 254 | 26.994 | 54.908 | 57.368 | 1.00 | 51.39 | C |
| ATOM | 1730 | CG | GLN | A | 254 | 25.709 | 55.657 | 57.030 | 1.00 | 51.39 | C |
| ATOM | 1731 | CD | GLN | A | 254 | 25.455 | 56.873 | 57.918 | 1.00 | 51.39 | C |
| ATOM | 1732 | OE1 | GLN | A | 254 | 25.280 | 56.756 | 59.141 | 1.00 | 51.39 | O |

| | | | | | | | | | | | |
|---|---|---|---|---|---|---|---|---|---|---|---|
| ATOM | 1733 | NE2 | GLN | A | 254 | 25.429 | 58.051 | 57.301 | 1.00 | 51.39 | N |
| ATOM | 1734 | N | PRO | A | 255 | 26.001 | 51.912 | 57.441 | 1.00 | 33.15 | N |
| ATOM | 1735 | CA | PRO | A | 255 | 24.898 | 50.956 | 57.540 | 1.00 | 33.15 | C |
| ATOM | 1736 | C | PRO | A | 255 | 23.584 | 51.709 | 57.538 | 1.00 | 33.15 | C |
| ATOM | 1737 | O | PRO | A | 255 | 23.525 | 52.849 | 57.985 | 1.00 | 33.15 | O |
| ATOM | 1738 | CB | PRO | A | 255 | 25.152 | 50.277 | 58.877 | 1.00 | 21.02 | C |
| ATOM | 1739 | CG | PRO | A | 255 | 26.631 | 50.260 | 58.956 | 1.00 | 21.02 | C |
| ATOM | 1740 | CD | PRO | A | 255 | 27.011 | 51.643 | 58.480 | 1.00 | 21.02 | C |
| ATOM | 1741 | N | ILE | A | 256 | 22.530 | 51.075 | 57.042 | 1.00 | 29.77 | N |
| ATOM | 1742 | CA | ILE | A | 256 | 21.228 | 51.707 | 56.995 | 1.00 | 29.77 | C |
| ATOM | 1743 | C | ILE | A | 256 | 20.360 | 51.396 | 58.220 | 1.00 | 29.77 | C |
| ATOM | 1744 | O | ILE | A | 256 | 19.510 | 52.196 | 58.590 | 1.00 | 29.77 | O |
| ATOM | 1745 | CB | ILE | A | 256 | 20.486 | 51.309 | 55.704 | 1.00 | 22.31 | C |
| ATOM | 1746 | CG1 | ILE | A | 256 | 19.158 | 52.052 | 55.620 | 1.00 | 22.31 | C |
| ATOM | 1747 | CG2 | ILE | A | 256 | 20.266 | 49.816 | 55.662 | 1.00 | 22.31 | C |
| ATOM | 1748 | CD1 | ILE | A | 256 | 18.564 | 52.075 | 54.233 | 1.00 | 22.31 | C |
| ATOM | 1749 | N | PHE | A | 257 | 20.579 | 50.250 | 58.859 | 1.00 | 26.75 | N |
| ATOM | 1750 | CA | PHE | A | 257 | 19.793 | 49.873 | 60.034 | 1.00 | 26.75 | C |
| ATOM | 1751 | C | PHE | A | 257 | 20.684 | 49.530 | 61.218 | 1.00 | 26.75 | C |
| ATOM | 1752 | O | PHE | A | 257 | 20.745 | 48.385 | 61.656 | 1.00 | 26.75 | O |
| ATOM | 1753 | CB | PHE | A | 257 | 18.902 | 48.669 | 59.726 | 1.00 | 28.25 | C |
| ATOM | 1754 | CG | PHE | A | 257 | 18.025 | 48.854 | 58.536 | 1.00 | 28.25 | C |
| ATOM | 1755 | CD1 | PHE | A | 257 | 17.262 | 50.005 | 58.392 | 1.00 | 28.25 | C |
| ATOM | 1756 | CD2 | PHE | A | 257 | 17.950 | 47.882 | 57.557 | 1.00 | 28.25 | C |
| ATOM | 1757 | CE1 | PHE | A | 257 | 16.436 | 50.185 | 57.289 | 1.00 | 28.25 | C |
| ATOM | 1758 | CE2 | PHE | A | 257 | 17.126 | 48.056 | 56.450 | 1.00 | 28.25 | C |
| ATOM | 1759 | CZ | PHE | A | 257 | 16.370 | 49.208 | 56.317 | 1.00 | 28.25 | C |
| ATOM | 1760 | N | PRO | A | 258 | 21.398 | 50.517 | 61.754 | 1.00 | 31.14 | N |
| ATOM | 1761 | CA | PRO | A | 258 | 22.250 | 50.184 | 62.888 | 1.00 | 31.14 | C |
| ATOM | 1762 | C | PRO | A | 258 | 21.453 | 50.205 | 64.185 | 1.00 | 31.14 | C |
| ATOM | 1763 | O | PRO | A | 258 | 20.242 | 50.445 | 64.181 | 1.00 | 31.14 | O |
| ATOM | 1764 | CB | PRO | A | 258 | 23.313 | 51.259 | 62.829 | 1.00 | 19.46 | C |
| ATOM | 1765 | CG | PRO | A | 258 | 22.535 | 52.441 | 62.399 | 1.00 | 19.46 | C |
| ATOM | 1766 | CD | PRO | A | 258 | 21.604 | 51.911 | 61.327 | 1.00 | 19.46 | C |
| ATOM | 1767 | N | GLY | A | 259 | 22.140 | 49.942 | 65.289 | 1.00 | 29.28 | N |
| ATOM | 1768 | CA | GLY | A | 259 | 21.489 | 49.926 | 66.580 | 1.00 | 29.28 | C |
| ATOM | 1769 | C | GLY | A | 259 | 22.201 | 48.973 | 67.510 | 1.00 | 29.28 | C |
| ATOM | 1770 | O | GLY | A | 259 | 22.611 | 47.892 | 67.093 | 1.00 | 29.28 | O |
| ATOM | 1771 | N | ASP | A | 260 | 22.374 | 49.373 | 68.764 | 1.00 | 36.38 | N |
| ATOM | 1772 | CA | ASP | A | 260 | 23.038 | 48.521 | 69.739 | 1.00 | 36.38 | C |
| ATOM | 1773 | C | ASP | A | 260 | 22.120 | 47.370 | 70.134 | 1.00 | 36.38 | C |
| ATOM | 1774 | O | ASP | A | 260 | 22.592 | 46.315 | 70.545 | 1.00 | 36.38 | O |
| ATOM | 1775 | CB | ASP | A | 260 | 23.420 | 49.323 | 70.987 | 1.00 | 47.71 | C |
| ATOM | 1776 | CG | ASP | A | 260 | 24.776 | 50.008 | 70.855 | 1.00 | 47.71 | C |
| ATOM | 1777 | OD1 | ASP | A | 260 | 25.300 | 50.097 | 69.716 | 1.00 | 47.71 | O |
| ATOM | 1778 | OD2 | ASP | A | 260 | 25.310 | 50.463 | 71.896 | 1.00 | 47.71 | O |
| ATOM | 1779 | N | SER | A | 261 | 20.812 | 47.577 | 70.006 | 1.00 | 17.64 | N |
| ATOM | 1780 | CA | SER | A | 261 | 19.852 | 46.550 | 70.357 | 1.00 | 17.64 | C |
| ATOM | 1781 | C | SER | A | 261 | 18.826 | 46.331 | 69.253 | 1.00 | 17.64 | C |
| ATOM | 1782 | O | SER | A | 261 | 18.721 | 47.114 | 68.324 | 1.00 | 17.64 | O |
| ATOM | 1783 | CB | SER | A | 261 | 19.120 | 46.935 | 71.638 | 1.00 | 27.99 | C |
| ATOM | 1784 | OG | SER | A | 261 | 18.171 | 47.965 | 71.386 | 1.00 | 27.99 | O |
| ATOM | 1785 | N | GLY | A | 262 | 18.062 | 45.255 | 69.375 | 1.00 | 31.16 | N |
| ATOM | 1786 | CA | GLY | A | 262 | 17.038 | 44.975 | 68.398 | 1.00 | 31.16 | C |
| ATOM | 1787 | C | GLY | A | 262 | 16.107 | 46.163 | 68.362 | 1.00 | 31.16 | C |
| ATOM | 1788 | O | GLY | A | 262 | 15.875 | 46.750 | 67.310 | 1.00 | 31.16 | O |
| ATOM | 1789 | N | VAL | A | 263 | 15.580 | 46.538 | 69.517 | 1.00 | 25.51 | N |
| ATOM | 1790 | CA | VAL | A | 263 | 14.674 | 47.672 | 69.581 | 1.00 | 25.51 | C |
| ATOM | 1791 | C | VAL | A | 263 | 15.180 | 48.841 | 68.742 | 1.00 | 25.51 | C |
| ATOM | 1792 | O | VAL | A | 263 | 14.433 | 49.408 | 67.954 | 1.00 | 25.51 | O |
| ATOM | 1793 | CB | VAL | A | 263 | 14.456 | 48.141 | 71.046 | 1.00 | 26.72 | C |
| ATOM | 1794 | CG1 | VAL | A | 263 | 13.550 | 49.377 | 71.078 | 1.00 | 26.72 | C |

| | | | | | | | | | | | |
|---|---|---|---|---|---|---|---|---|---|---|---|
| ATOM | 1795 | CG2 | VAL | A | 263 | 13.830 | 47.020 | 71.852 | 1.00 | 26.72 | C |
| ATOM | 1796 | N | ASP | A | 264 | 16.446 | 49.195 | 68.902 | 1.00 | 23.62 | N |
| ATOM | 1797 | CA | ASP | A | 264 | 16.992 | 50.297 | 68.132 | 1.00 | 23.62 | C |
| ATOM | 1798 | C | ASP | A | 264 | 16.962 | 50.012 | 66.641 | 1.00 | 23.62 | C |
| ATOM | 1799 | O | ASP | A | 264 | 16.538 | 50.855 | 65.848 | 1.00 | 23.62 | O |
| ATOM | 1800 | CB | ASP | A | 264 | 18.421 | 50.594 | 68.566 | 1.00 | 36.57 | C |
| ATOM | 1801 | CG | ASP | A | 264 | 18.488 | 51.166 | 69.958 | 1.00 | 36.57 | C |
| ATOM | 1802 | OD1 | ASP | A | 264 | 17.525 | 51.854 | 70.338 | 1.00 | 36.57 | O |
| ATOM | 1803 | OD2 | ASP | A | 264 | 19.493 | 50.945 | 70.663 | 1.00 | 36.57 | O |
| ATOM | 1804 | N | GLN | A | 265 | 17.423 | 48.817 | 66.272 | 1.00 | 27.66 | N |
| ATOM | 1805 | CA | GLN | A | 265 | 17.458 | 48.370 | 64.883 | 1.00 | 27.66 | C |
| ATOM | 1806 | C | GLN | A | 265 | 16.065 | 48.472 | 64.264 | 1.00 | 27.66 | C |
| ATOM | 1807 | O | GLN | A | 265 | 15.900 | 48.988 | 63.158 | 1.00 | 27.66 | O |
| ATOM | 1808 | CB | GLN | A | 265 | 17.952 | 46.925 | 64.817 | 1.00 | 38.02 | C |
| ATOM | 1809 | CG | GLN | A | 265 | 19.470 | 46.759 | 64.893 | 1.00 | 38.02 | C |
| ATOM | 1810 | CD | GLN | A | 265 | 19.888 | 45.365 | 65.365 | 1.00 | 38.02 | C |
| ATOM | 1811 | OE1 | GLN | A | 265 | 19.269 | 44.376 | 65.006 | 1.00 | 38.02 | O |
| ATOM | 1812 | NE2 | GLN | A | 265 | 20.940 | 45.292 | 66.170 | 1.00 | 38.02 | N |
| ATOM | 1813 | N | LEU | A | 266 | 15.059 | 47.985 | 64.977 | 1.00 | 29.79 | N |
| ATOM | 1814 | CA | LEU | A | 266 | 13.712 | 48.065 | 64.455 | 1.00 | 29.79 | C |
| ATOM | 1815 | C | LEU | A | 266 | 13.312 | 49.514 | 64.227 | 1.00 | 29.79 | C |
| ATOM | 1816 | O | LEU | A | 266 | 12.702 | 49.829 | 63.208 | 1.00 | 29.79 | O |
| ATOM | 1817 | CB | LEU | A | 266 | 12.724 | 47.381 | 65.395 | 1.00 | 30.81 | C |
| ATOM | 1818 | CG | LEU | A | 266 | 12.338 | 45.984 | 64.932 | 1.00 | 30.81 | C |
| ATOM | 1819 | CD1 | LEU | A | 266 | 13.576 | 45.190 | 64.729 | 1.00 | 30.81 | C |
| ATOM | 1820 | CD2 | LEU | A | 266 | 11.448 | 45.316 | 65.944 | 1.00 | 30.81 | C |
| ATOM | 1821 | N | VAL | A | 267 | 13.660 | 50.396 | 65.160 | 1.00 | 27.95 | N |
| ATOM | 1822 | CA | VAL | A | 267 | 13.329 | 51.808 | 65.010 | 1.00 | 27.95 | C |
| ATOM | 1823 | C | VAL | A | 267 | 13.797 | 52.295 | 63.645 | 1.00 | 27.95 | C |
| ATOM | 1824 | O | VAL | A | 267 | 13.018 | 52.815 | 62.844 | 1.00 | 27.95 | O |
| ATOM | 1825 | CB | VAL | A | 267 | 14.010 | 52.675 | 66.092 | 1.00 | 35.03 | C |
| ATOM | 1826 | CG1 | VAL | A | 267 | 13.841 | 54.158 | 65.769 | 1.00 | 35.03 | C |
| ATOM | 1827 | CG2 | VAL | A | 267 | 13.412 | 52.369 | 67.441 | 1.00 | 35.03 | C |
| ATOM | 1828 | N | GLU | A | 268 | 15.080 | 52.119 | 63.384 | 1.00 | 40.12 | N |
| ATOM | 1829 | CA | GLU | A | 268 | 15.627 | 52.537 | 62.111 | 1.00 | 40.12 | C |
| ATOM | 1830 | C | GLU | A | 268 | 14.919 | 51.849 | 60.935 | 1.00 | 40.12 | C |
| ATOM | 1831 | O | GLU | A | 268 | 14.607 | 52.488 | 59.934 | 1.00 | 40.12 | O |
| ATOM | 1832 | CB | GLU | A | 268 | 17.137 | 52.281 | 62.080 | 1.00 | 48.16 | C |
| ATOM | 1833 | CG | GLU | A | 268 | 17.923 | 53.207 | 63.003 | 1.00 | 48.16 | C |
| ATOM | 1834 | CD | GLU | A | 268 | 17.444 | 54.649 | 62.911 | 1.00 | 48.16 | C |
| ATOM | 1835 | OE1 | GLU | A | 268 | 17.356 | 55.192 | 61.785 | 1.00 | 48.16 | O |
| ATOM | 1836 | OE2 | GLU | A | 268 | 17.146 | 55.239 | 63.971 | 1.00 | 48.16 | O |
| ATOM | 1837 | N | ILE | A | 269 | 14.645 | 50.556 | 61.055 | 1.00 | 28.21 | N |
| ATOM | 1838 | CA | ILE | A | 269 | 13.952 | 49.861 | 59.978 | 1.00 | 28.21 | C |
| ATOM | 1839 | C | ILE | A | 269 | 12.587 | 50.511 | 59.712 | 1.00 | 28.21 | C |
| ATOM | 1840 | O | ILE | A | 269 | 12.221 | 50.790 | 58.566 | 1.00 | 28.21 | O |
| ATOM | 1841 | CB | ILE | A | 269 | 13.771 | 48.374 | 60.321 | 1.00 | 12.18 | C |
| ATOM | 1842 | CG1 | ILE | A | 269 | 15.142 | 47.703 | 60.345 | 1.00 | 12.18 | C |
| ATOM | 1843 | CG2 | ILE | A | 269 | 12.855 | 47.704 | 59.321 | 1.00 | 12.18 | C |
| ATOM | 1844 | CD1 | ILE | A | 269 | 15.101 | 46.204 | 60.519 | 1.00 | 12.18 | C |
| ATOM | 1845 | N | ILE | A | 270 | 11.852 | 50.769 | 60.785 | 1.00 | 38.75 | N |
| ATOM | 1846 | CA | ILE | A | 270 | 10.542 | 51.378 | 60.678 | 1.00 | 38.75 | C |
| ATOM | 1847 | C | ILE | A | 270 | 10.626 | 52.806 | 60.131 | 1.00 | 38.75 | C |
| ATOM | 1848 | O | ILE | A | 270 | 9.827 | 53.197 | 59.269 | 1.00 | 38.75 | O |
| ATOM | 1849 | CB | ILE | A | 270 | 9.833 | 51.367 | 62.054 | 1.00 | 36.77 | C |
| ATOM | 1850 | CG1 | ILE | A | 270 | 9.534 | 49.916 | 62.456 | 1.00 | 36.77 | C |
| ATOM | 1851 | CG2 | ILE | A | 270 | 8.532 | 52.163 | 61.990 | 1.00 | 36.77 | C |
| ATOM | 1852 | CD1 | ILE | A | 270 | 9.003 | 49.744 | 63.857 | 1.00 | 36.77 | C |
| ATOM | 1853 | N | LYS | A | 271 | 11.590 | 53.583 | 60.614 | 1.00 | 38.41 | N |
| ATOM | 1854 | CA | LYS | A | 271 | 11.738 | 54.954 | 60.139 | 1.00 | 38.41 | C |
| ATOM | 1855 | C | LYS | A | 271 | 11.741 | 55.018 | 58.614 | 1.00 | 38.41 | C |
| ATOM | 1856 | O | LYS | A | 271 | 11.315 | 56.011 | 58.027 | 1.00 | 38.41 | O |

| | | | | | | | | | | | |
|---|---|---|---|---|---|---|---|---|---|---|---|
| ATOM | 1857 | CB | LYS | A | 271 | 13.036 | 55.585 | 60.663 | 1.00 | 37.99 | C |
| ATOM | 1858 | CG | LYS | A | 271 | 12.988 | 56.118 | 62.083 | 1.00 | 37.99 | C |
| ATOM | 1859 | CD | LYS | A | 271 | 14.318 | 56.785 | 62.467 | 1.00 | 37.99 | C |
| ATOM | 1860 | CE | LYS | A | 271 | 14.241 | 57.404 | 63.853 | 0.00 | 37.99 | C |
| ATOM | 1861 | NZ | LYS | A | 271 | 15.513 | 58.075 | 64.233 | 0.00 | 37.99 | N |
| ATOM | 1862 | N | VAL | A | 272 | 12.215 | 53.969 | 57.956 | 1.00 | 54.30 | N |
| ATOM | 1863 | CA | VAL | A | 272 | 12.262 | 54.007 | 56.500 | 1.00 | 54.30 | C |
| ATOM | 1864 | C | VAL | A | 272 | 11.197 | 53.184 | 55.791 | 1.00 | 54.30 | C |
| ATOM | 1865 | O | VAL | A | 272 | 10.608 | 53.642 | 54.799 | 1.00 | 54.30 | O |
| ATOM | 1866 | CB | VAL | A | 272 | 13.637 | 53.580 | 55.975 | 1.00 | 55.29 | C |
| ATOM | 1867 | CG1 | VAL | A | 272 | 14.577 | 54.756 | 56.003 | 1.00 | 55.29 | C |
| ATOM | 1868 | CG2 | VAL | A | 272 | 14.201 | 52.476 | 56.844 | 1.00 | 55.29 | C |
| ATOM | 1869 | N | LEU | A | 273 | 10.943 | 51.977 | 56.282 | 1.00 | 40.87 | N |
| ATOM | 1870 | CA | LEU | A | 273 | 9.934 | 51.144 | 55.652 | 1.00 | 40.87 | C |
| ATOM | 1871 | C | LEU | A | 273 | 8.524 | 51.541 | 56.069 | 1.00 | 40.87 | C |
| ATOM | 1872 | O | LEU | A | 273 | 7.560 | 51.300 | 55.343 | 1.00 | 40.87 | O |
| ATOM | 1873 | CB | LEU | A | 273 | 10.164 | 49.675 | 55.999 | 1.00 | 29.67 | C |
| ATOM | 1874 | CG | LEU | A | 273 | 11.261 | 48.951 | 55.234 | 1.00 | 29.67 | C |
| ATOM | 1875 | CD1 | LEU | A | 273 | 11.190 | 47.491 | 55.599 | 1.00 | 29.67 | C |
| ATOM | 1876 | CD2 | LEU | A | 273 | 11.070 | 49.124 | 53.740 | 0.00 | 29.67 | C |
| ATOM | 1877 | N | GLY | A | 274 | 8.400 | 52.153 | 57.239 | 1.00 | 52.25 | N |
| ATOM | 1878 | CA | GLY | A | 274 | 7.077 | 52.520 | 57.717 | 1.00 | 52.25 | C |
| ATOM | 1879 | C | GLY | A | 274 | 6.592 | 51.464 | 58.688 | 1.00 | 52.25 | C |
| ATOM | 1880 | O | GLY | A | 274 | 7.061 | 50.323 | 58.645 | 1.00 | 52.25 | O |
| ATOM | 1881 | N | THR | A | 275 | 5.675 | 51.828 | 59.572 | 1.00 | 47.53 | N |
| ATOM | 1882 | CA | THR | A | 275 | 5.168 | 50.911 | 60.588 | 1.00 | 47.53 | C |
| ATOM | 1883 | C | THR | A | 275 | 4.584 | 49.619 | 59.977 | 1.00 | 47.53 | C |
| ATOM | 1884 | O | THR | A | 275 | 3.838 | 49.634 | 59.008 | 1.00 | 47.53 | O |
| ATOM | 1885 | CB | THR | A | 275 | 4.042 | 51.644 | 61.436 | 1.00 | 64.24 | C |
| ATOM | 1886 | OG1 | THR | A | 275 | 4.675 | 52.510 | 62.399 | 1.00 | 64.24 | O |
| ATOM | 1887 | CG2 | THR | A | 275 | 3.100 | 50.629 | 62.113 | 1.00 | 64.24 | C |
| ATOM | 1888 | N | PRO | A | 276 | 4.898 | 48.466 | 60.546 | 1.00 | 59.25 | N |
| ATOM | 1889 | CA | PRO | A | 276 | 4.299 | 47.287 | 59.918 | 1.00 | 59.25 | C |
| ATOM | 1890 | C | PRO | A | 276 | 2.834 | 47.036 | 60.244 | 1.00 | 59.25 | C |
| ATOM | 1891 | O | PRO | A | 276 | 2.378 | 47.188 | 61.388 | 1.00 | 59.25 | O |
| ATOM | 1892 | CB | PRO | A | 276 | 5.185 | 46.162 | 60.376 | 1.00 | 56.82 | C |
| ATOM | 1893 | CG | PRO | A | 276 | 6.005 | 46.787 | 61.522 | 1.00 | 56.82 | C |
| ATOM | 1894 | CD | PRO | A | 276 | 5.536 | 48.132 | 61.810 | 1.00 | 56.82 | C |
| ATOM | 1895 | N | THR | A | 277 | 2.144 | 46.581 | 59.217 | 1.00 | 63.97 | N |
| ATOM | 1896 | CA | THR | A | 277 | 0.756 | 46.257 | 59.382 | 1.00 | 63.97 | C |
| ATOM | 1897 | C | THR | A | 277 | 0.681 | 44.943 | 60.114 | 1.00 | 63.97 | C |
| ATOM | 1898 | O | THR | A | 277 | 1.732 | 44.280 | 60.229 | 1.00 | 63.97 | O |
| ATOM | 1899 | CB | THR | A | 277 | 0.085 | 46.093 | 58.007 | 1.00 | 62.34 | C |
| ATOM | 1900 | OG1 | THR | A | 277 | 0.705 | 44.991 | 57.306 | 1.00 | 62.34 | O |
| ATOM | 1901 | CG2 | THR | A | 277 | 0.217 | 47.369 | 57.215 | 1.00 | 62.34 | C |
| ATOM | 1902 | N | ARG | A | 278 | -0.500 | 44.588 | 60.608 | 1.00 | 59.83 | N |
| ATOM | 1903 | CA | ARG | A | 278 | -0.700 | 43.352 | 61.298 | 1.00 | 59.83 | C |
| ATOM | 1904 | C | ARG | A | 278 | -0.339 | 42.143 | 60.461 | 1.00 | 59.83 | C |
| ATOM | 1905 | O | ARG | A | 278 | 0.131 | 41.174 | 60.993 | 1.00 | 59.83 | O |
| ATOM | 1906 | CB | ARG | A | 278 | -2.189 | 43.217 | 61.914 | 0.00 | 35.69 | C |
| ATOM | 1907 | N | GLU | A | 279 | -0.676 | 42.199 | 59.175 | 1.00 | 67.05 | N |
| ATOM | 1908 | CA | GLU | A | 279 | -0.378 | 41.084 | 58.287 | 1.00 | 67.05 | C |
| ATOM | 1909 | C | GLU | A | 279 | 1.155 | 41.017 | 58.180 | 1.00 | 67.05 | C |
| ATOM | 1910 | O | GLU | A | 279 | 1.793 | 39.983 | 58.525 | 1.00 | 67.05 | O |
| ATOM | 1911 | CB | GLU | A | 279 | -1.059 | 41.264 | 56.897 | 1.00100.00 | | C |
| ATOM | 1912 | CG | GLU | A | 279 | -2.546 | 40.928 | 56.958 | 1.00100.00 | | C |
| ATOM | 1913 | CD | GLU | A | 279 | -2.837 | 39.631 | 57.745 | 1.00100.00 | | C |
| ATOM | 1914 | OE1 | GLU | A | 279 | -2.052 | 38.631 | 57.620 | 1.00100.00 | | O |
| ATOM | 1915 | OE2 | GLU | A | 279 | -3.869 | 39.608 | 58.492 | 1.00100.00 | | O |
| ATOM | 1916 | N | GLN | A | 280 | 1.753 | 42.135 | 57.756 | 1.00 | 69.01 | N |
| ATOM | 1917 | CA | GLN | A | 280 | 3.201 | 42.194 | 57.609 | 1.00 | 69.01 | C |
| ATOM | 1918 | C | GLN | A | 280 | 3.899 | 41.514 | 58.779 | 1.00 | 69.01 | C |

| | | | | | | | | | | |
|---|---|---|---|---|---|---|---|---|---|---|
| ATOM | 1919 | O | GLN | A | 280 | 4.871 | 40.768 | 58.593 | 1.00 69.01 | O |
| ATOM | 1920 | CB | GLN | A | 280 | 3.678 | 43.655 | 57.480 | 1.00 63.74 | C |
| ATOM | 1921 | CG | GLN | A | 280 | 3.420 | 44.297 | 56.110 | 1.00 63.74 | C |
| ATOM | 1922 | CD | GLN | A | 280 | 3.982 | 45.697 | 56.020 | 1.00 63.74 | C |
| ATOM | 1923 | OE1 | GLN | A | 280 | 3.886 | 46.360 | 54.980 | 1.00 63.74 | O |
| ATOM | 1924 | NE2 | GLN | A | 280 | 4.580 | 46.162 | 57.116 | 1.00 63.74 | N |
| ATOM | 1925 | N | ILE | A | 281 | 3.389 | 41.739 | 59.985 | 1.00 74.60 | N |
| ATOM | 1926 | CA | ILE | A | 281 | 4.005 | 41.129 | 61.147 | 1.00 74.60 | C |
| ATOM | 1927 | C | ILE | A | 281 | 3.804 | 39.629 | 61.167 | 1.00 74.60 | C |
| ATOM | 1928 | O | ILE | A | 281 | 4.768 | 38.869 | 61.285 | 1.00 74.60 | O |
| ATOM | 1929 | CB | ILE | A | 281 | 3.435 | 41.706 | 62.415 | 1.00 72.22 | C |
| ATOM | 1930 | CG1 | ILE | A | 281 | 3.623 | 43.233 | 62.388 | 1.00 72.22 | C |
| ATOM | 1931 | CG2 | ILE | A | 281 | 4.116 | 41.046 | 63.612 | 1.00 72.22 | C |
| ATOM | 1932 | CD1 | ILE | A | 281 | 2.664 | 44.027 | 63.287 | 1.00 72.22 | C |
| ATOM | 1933 | N | ARG | A | 282 | 2.546 | 39.209 | 61.042 | 1.00 63.19 | N |
| ATOM | 1934 | CA | ARG | A | 282 | 2.224 | 37.794 | 61.048 | 1.00 63.19 | C |
| ATOM | 1935 | C | ARG | A | 282 | 3.187 | 37.043 | 60.158 | 1.00 63.19 | C |
| ATOM | 1936 | O | ARG | A | 282 | 3.652 | 35.969 | 60.519 | 1.00 63.19 | O |
| ATOM | 1937 | CB | ARG | A | 282 | 0.797 | 37.575 | 60.568 | 1.00 58.92 | C |
| ATOM | 1938 | N | GLU | A | 283 | 3.496 | 37.622 | 59.000 | 1.00 98.45 | N |
| ATOM | 1939 | CA | GLU | A | 283 | 4.402 | 36.978 | 58.047 | 1.00 98.45 | C |
| ATOM | 1940 | C | GLU | A | 283 | 5.842 | 36.999 | 58.559 | 1.00 98.45 | C |
| ATOM | 1941 | O | GLU | A | 283 | 6.670 | 36.180 | 58.141 | 1.00 98.45 | O |
| ATOM | 1942 | CB | GLU | A | 283 | 4.290 | 37.658 | 56.686 | 0.00 85.00 | C |
| ATOM | 1943 | CG | GLU | A | 283 | 2.860 | 37.639 | 56.157 | 0.00 85.00 | C |
| ATOM | 1944 | CD | GLU | A | 283 | 2.653 | 38.684 | 55.130 | 1.00 85.00 | C |
| ATOM | 1945 | OE1 | GLU | A | 283 | 3.448 | 39.655 | 55.176 | 1.00 85.00 | O |
| ATOM | 1946 | OE2 | GLU | A | 283 | 1.715 | 38.584 | 54.285 | 1.00 85.00 | O |
| ATOM | 1947 | N | MET | A | 284 | 6.123 | 37.911 | 59.490 | 1.00 73.55 | N |
| ATOM | 1948 | CA | MET | A | 284 | 7.462 | 38.002 | 60.062 | 1.00 73.55 | C |
| ATOM | 1949 | C | MET | A | 284 | 7.711 | 36.894 | 61.057 | 1.00 73.55 | C |
| ATOM | 1950 | O | MET | A | 284 | 8.391 | 35.917 | 60.739 | 1.00 73.55 | O |
| ATOM | 1951 | CB | MET | A | 284 | 7.695 | 39.355 | 60.732 | 1.00 75.43 | C |
| ATOM | 1952 | CG | MET | A | 284 | 8.283 | 40.366 | 59.780 | 1.00 75.43 | C |
| ATOM | 1953 | SD | MET | A | 284 | 8.408 | 41.862 | 60.689 | 1.00 75.43 | S |
| ATOM | 1954 | CE | MET | A | 284 | 9.078 | 42.774 | 59.560 | 1.00 75.43 | C |
| ATOM | 1955 | N | ASN | A | 285 | 7.166 | 37.026 | 62.256 | 1.00 68.20 | N |
| ATOM | 1956 | CA | ASN | A | 285 | 7.402 | 35.974 | 63.222 | 1.00 68.20 | C |
| ATOM | 1957 | C | ASN | A | 285 | 7.076 | 36.410 | 64.626 | 1.00 68.20 | C |
| ATOM | 1958 | O | ASN | A | 285 | 5.998 | 36.061 | 65.108 | 1.00 68.20 | O |
| ATOM | 1959 | N | TRP | A | 301 | 12.071 | 56.413 | 51.578 | 1.00 61.19 | N |
| ATOM | 1960 | CA | TRP | A | 301 | 13.023 | 55.807 | 50.655 | 1.00 61.19 | C |
| ATOM | 1961 | C | TRP | A | 301 | 13.648 | 56.851 | 49.727 | 1.00 61.19 | C |
| ATOM | 1962 | O | TRP | A | 301 | 14.833 | 56.831 | 49.420 | 1.00 61.19 | O |
| ATOM | 1963 | CB | TRP | A | 301 | 12.280 | 54.756 | 49.829 | 1.00 42.80 | C |
| ATOM | 1964 | CG | TRP | A | 301 | 12.497 | 53.409 | 50.404 | 1.00 42.80 | C |
| ATOM | 1965 | CD1 | TRP | A | 301 | 11.516 | 52.421 | 50.641 | 1.00 42.80 | C |
| ATOM | 1966 | CD2 | TRP | A | 301 | 13.759 | 52.850 | 50.841 | 1.00 42.80 | C |
| ATOM | 1967 | NE1 | TRP | A | 301 | 12.037 | 51.290 | 51.185 | 1.00 42.80 | N |
| ATOM | 1968 | CE2 | TRP | A | 301 | 13.493 | 51.545 | 51.324 | 1.00 42.80 | C |
| ATOM | 1969 | CE3 | TRP | A | 301 | 15.063 | 53.336 | 50.864 | 1.00 42.80 | C |
| ATOM | 1970 | CZ2 | TRP | A | 301 | 14.526 | 50.768 | 51.820 | 1.00 42.80 | C |
| ATOM | 1971 | CZ3 | TRP | A | 301 | 16.097 | 52.558 | 51.359 | 1.00 42.80 | C |
| ATOM | 1972 | CH2 | TRP | A | 301 | 15.824 | 51.265 | 51.841 | 1.00 42.80 | C |
| ATOM | 1973 | N | THR | A | 302 | 12.789 | 57.767 | 49.246 | 1.00 54.08 | N |
| ATOM | 1974 | CA | THR | A | 302 | 13.268 | 58.791 | 48.326 | 1.00 54.08 | C |
| ATOM | 1975 | C | THR | A | 302 | 14.139 | 59.832 | 49.035 | 1.00 54.08 | C |
| ATOM | 1976 | O | THR | A | 302 | 14.910 | 60.562 | 48.427 | 1.00 54.08 | O |
| ATOM | 1977 | CB | THR | A | 302 | 12.051 | 59.463 | 47.690 | 1.00 72.44 | C |
| ATOM | 1978 | OG1 | THR | A | 302 | 11.102 | 59.772 | 48.713 | 1.00 72.44 | O |
| ATOM | 1979 | CG2 | THR | A | 302 | 11.397 | 58.512 | 46.683 | 0.00 72.44 | C |
| ATOM | 1980 | N | LYS | A | 303 | 13.965 | 59.912 | 50.369 | 1.00 42.66 | N |

| | | | | | | | | | | | |
|---|---|---|---|---|---|---|---|---|---|---|---|
| ATOM | 1981 | CA | LYS | A | 303 | 14.756 | 60.879 | 51.120 | 1.00 | 42.66 | C |
| ATOM | 1982 | C | LYS | A | 303 | 15.760 | 60.185 | 52.043 | 1.00 | 42.66 | C |
| ATOM | 1983 | O | LYS | A | 303 | 16.146 | 60.688 | 53.090 | 1.00 | 42.66 | O |
| ATOM | 1984 | CB | LYS | A | 303 | 13.799 | 61.742 | 51.944 | 1.00 | 60.61 | C |
| ATOM | 1985 | N | VAL | A | 304 | 16.154 | 58.964 | 51.636 | 1.00 | 57.27 | N |
| ATOM | 1986 | CA | VAL | A | 304 | 17.096 | 58.204 | 52.448 | 1.00 | 57.27 | C |
| ATOM | 1987 | C | VAL | A | 304 | 18.520 | 58.288 | 51.893 | 1.00 | 57.27 | C |
| ATOM | 1988 | O | VAL | A | 304 | 19.483 | 58.552 | 52.601 | 1.00 | 57.27 | O |
| ATOM | 1989 | CB | VAL | A | 304 | 16.633 | 56.747 | 52.473 | 1.00 | 33.85 | C |
| ATOM | 1990 | CG1 | VAL | A | 304 | 17.801 | 55.836 | 52.848 | 1.00 | 33.85 | C |
| ATOM | 1991 | CG2 | VAL | A | 304 | 15.518 | 56.575 | 53.488 | 1.00 | 33.85 | C |
| ATOM | 1992 | N | PHE | A | 305 | 18.638 | 58.012 | 50.580 | 1.00 | 37.26 | N |
| ATOM | 1993 | CA | PHE | A | 305 | 19.955 | 58.026 | 49.954 | 1.00 | 37.26 | C |
| ATOM | 1994 | C | PHE | A | 305 | 20.357 | 59.433 | 49.507 | 1.00 | 37.26 | C |
| ATOM | 1995 | O | PHE | A | 305 | 19.536 | 60.323 | 49.325 | 1.00 | 37.26 | O |
| ATOM | 1996 | CB | PHE | A | 305 | 19.918 | 57.090 | 48.746 | 1.00 | 37.35 | C |
| ATOM | 1997 | CG | PHE | A | 305 | 19.730 | 55.675 | 49.207 | 1.00 | 37.35 | C |
| ATOM | 1998 | CD1 | PHE | A | 305 | 18.450 | 55.136 | 49.263 | 1.00 | 37.35 | C |
| ATOM | 1999 | CD2 | PHE | A | 305 | 20.826 | 54.918 | 49.585 | 1.00 | 37.35 | C |
| ATOM | 2000 | CE1 | PHE | A | 305 | 18.271 | 53.832 | 49.702 | 1.00 | 37.35 | C |
| ATOM | 2001 | CE2 | PHE | A | 305 | 20.639 | 53.610 | 50.025 | 1.00 | 37.35 | C |
| ATOM | 2002 | CZ | PHE | A | 305 | 19.363 | 53.064 | 50.085 | 1.00 | 37.35 | C |
| ATOM | 2003 | N | ARG | A | 306 | 21.682 | 59.628 | 49.370 | 1.00 | 31.29 | N |
| ATOM | 2004 | CA | ARG | A | 306 | 22.172 | 60.920 | 48.910 | 1.00 | 31.29 | C |
| ATOM | 2005 | C | ARG | A | 306 | 21.530 | 61.313 | 47.577 | 1.00 | 31.29 | C |
| ATOM | 2006 | O | ARG | A | 306 | 21.054 | 60.485 | 46.811 | 1.00 | 31.29 | O |
| ATOM | 2007 | CB | ARG | A | 306 | 23.690 | 60.825 | 48.752 | 1.00 | 61.41 | C |
| ATOM | 2008 | CG | ARG | A | 306 | 24.143 | 61.070 | 47.313 | 1.00 | 61.41 | C |
| ATOM | 2009 | CD | ARG | A | 306 | 25.026 | 59.931 | 46.787 | 1.00 | 61.41 | C |
| ATOM | 2010 | NE | ARG | A | 306 | 26.116 | 60.464 | 45.965 | 1.00 | 61.41 | N |
| ATOM | 2011 | CZ | ARG | A | 306 | 27.118 | 59.624 | 45.644 | 1.00 | 61.41 | C |
| ATOM | 2012 | NH1 | ARG | A | 306 | 27.091 | 58.368 | 46.057 | 1.00 | 61.41 | N |
| ATOM | 2013 | NH2 | ARG | A | 306 | 28.150 | 60.073 | 44.927 | 1.00 | 61.41 | N |
| ATOM | 2014 | N | PRO | A | 307 | 21.492 | 62.637 | 47.333 | 1.00 | 53.14 | N |
| ATOM | 2015 | CA | PRO | A | 307 | 20.901 | 63.176 | 46.116 | 1.00 | 53.14 | C |
| ATOM | 2016 | C | PRO | A | 307 | 21.664 | 62.732 | 44.866 | 1.00 | 53.14 | C |
| ATOM | 2017 | O | PRO | A | 307 | 22.816 | 62.322 | 44.913 | 1.00 | 53.14 | O |
| ATOM | 2018 | CB | PRO | A | 307 | 20.920 | 64.701 | 46.223 | 1.00 | 64.42 | C |
| ATOM | 2019 | CG | PRO | A | 307 | 21.315 | 65.072 | 47.651 | 1.00 | 64.42 | C |
| ATOM | 2020 | CD | PRO | A | 307 | 21.990 | 63.717 | 48.167 | 1.00 | 64.42 | C |
| ATOM | 2021 | N | ARG | A | 308 | 20.957 | 62.786 | 43.720 | 1.00 | 40.68 | N |
| ATOM | 2022 | CA | ARG | A | 308 | 21.597 | 62.439 | 42.455 | 1.00 | 40.68 | C |
| ATOM | 2023 | C | ARG | A | 308 | 21.934 | 60.949 | 42.375 | 1.00 | 40.68 | C |
| ATOM | 2024 | O | ARG | A | 308 | 22.704 | 60.498 | 41.537 | 1.00 | 40.68 | O |
| ATOM | 2025 | CB | ARG | A | 308 | 22.876 | 63.267 | 42.324 | 0.00 | 35.69 | C |
| ATOM | 2026 | N | THR | A | 309 | 21.354 | 60.178 | 43.313 | 1.00 | 34.01 | N |
| ATOM | 2027 | CA | THR | A | 309 | 21.635 | 58.747 | 43.336 | 1.00 | 34.01 | C |
| ATOM | 2028 | C | THR | A | 309 | 20.659 | 57.963 | 42.458 | 1.00 | 34.01 | C |
| ATOM | 2029 | O | THR | A | 309 | 19.452 | 58.162 | 42.493 | 1.00 | 34.01 | O |
| ATOM | 2030 | CB | THR | A | 309 | 21.545 | 58.259 | 44.782 | 1.00 | 33.02 | C |
| ATOM | 2031 | OG1 | THR | A | 309 | 21.821 | 56.858 | 44.817 | 1.00 | 33.02 | O |
| ATOM | 2032 | CG2 | THR | A | 309 | 20.135 | 58.499 | 45.330 | 1.00 | 33.02 | C |
| ATOM | 2033 | N | PRO | A | 310 | 21.105 | 57.128 | 41.510 | 1.00 | 38.98 | N |
| ATOM | 2034 | CA | PRO | A | 310 | 20.190 | 56.439 | 40.630 | 1.00 | 38.98 | C |
| ATOM | 2035 | C | PRO | A | 310 | 18.969 | 55.967 | 41.411 | 1.00 | 38.98 | C |
| ATOM | 2036 | O | PRO | A | 310 | 19.056 | 55.394 | 42.489 | 1.00 | 38.98 | O |
| ATOM | 2037 | CB | PRO | A | 310 | 20.935 | 55.246 | 40.037 | 1.00 | 20.74 | C |
| ATOM | 2038 | CG | PRO | A | 310 | 22.409 | 55.341 | 40.440 | 1.00 | 20.74 | C |
| ATOM | 2039 | CD | PRO | A | 310 | 22.467 | 56.749 | 41.203 | 1.00 | 20.74 | C |
| ATOM | 2040 | N | PRO | A | 311 | 17.875 | 56.267 | 40.684 | 1.00 | 39.47 | N |
| ATOM | 2041 | CA | PRO | A | 311 | 16.543 | 55.842 | 41.090 | 1.00 | 39.47 | C |
| ATOM | 2042 | C | PRO | A | 311 | 16.341 | 54.340 | 40.867 | 1.00 | 39.47 | C |

| | | | | | | | | | | | |
|---|---|---|---|---|---|---|---|---|---|---|---|
| ATOM | 2043 | O | PRO | A | 311 | 15.580 | 53.675 | 41.558 | 1.00 | 39.47 | O |
| ATOM | 2044 | CB | PRO | A | 311 | 15.532 | 56.637 | 40.260 | 1.00 | 36.28 | C |
| ATOM | 2045 | CG | PRO | A | 311 | 16.301 | 57.586 | 39.338 | 1.00 | 36.28 | C |
| ATOM | 2046 | CD | PRO | A | 311 | 17.796 | 57.027 | 39.451 | 1.00 | 36.28 | C |
| ATOM | 2047 | N | GLU | A | 312 | 17.085 | 53.665 | 40.003 | 1.00 | 32.81 | N |
| ATOM | 2048 | CA | GLU | A | 312 | 16.999 | 52.214 | 39.903 | 1.00 | 32.81 | C |
| ATOM | 2049 | C | GLU | A | 312 | 17.545 | 51.536 | 41.155 | 1.00 | 32.81 | C |
| ATOM | 2050 | O | GLU | A | 312 | 17.016 | 50.512 | 41.600 | 1.00 | 32.81 | O |
| ATOM | 2051 | CB | GLU | A | 312 | 17.773 | 51.710 | 38.688 | 1.00 | 59.36 | C |
| ATOM | 2052 | CG | GLU | A | 312 | 17.230 | 52.203 | 37.372 | 1.00 | 59.36 | C |
| ATOM | 2053 | CD | GLU | A | 312 | 17.179 | 53.722 | 37.294 | 1.00 | 59.36 | C |
| ATOM | 2054 | OE1 | GLU | A | 312 | 18.077 | 54.395 | 37.878 | 1.00 | 59.36 | O |
| ATOM | 2055 | OE2 | GLU | A | 312 | 16.242 | 54.237 | 36.632 | 1.00 | 59.36 | O |
| ATOM | 2056 | N | ALA | A | 313 | 18.600 | 52.116 | 41.719 | 1.00 | 27.51 | N |
| ATOM | 2057 | CA | ALA | A | 313 | 19.225 | 51.564 | 42.910 | 1.00 | 27.51 | C |
| ATOM | 2058 | C | ALA | A | 313 | 18.299 | 51.666 | 44.101 | 1.00 | 27.51 | C |
| ATOM | 2059 | O | ALA | A | 313 | 18.207 | 50.751 | 44.923 | 1.00 | 27.51 | O |
| ATOM | 2060 | CB | ALA | A | 313 | 20.522 | 52.296 | 43.204 | 1.00 | 47.57 | C |
| ATOM | 2061 | N | ILE | A | 314 | 17.621 | 52.800 | 44.191 | 1.00 | 31.65 | N |
| ATOM | 2062 | CA | ILE | A | 314 | 16.701 | 53.043 | 45.279 | 1.00 | 31.65 | C |
| ATOM | 2063 | C | ILE | A | 314 | 15.500 | 52.115 | 45.126 | 1.00 | 31.65 | C |
| ATOM | 2064 | O | ILE | A | 314 | 14.946 | 51.639 | 46.111 | 1.00 | 31.65 | O |
| ATOM | 2065 | CB | ILE | A | 314 | 16.237 | 54.504 | 45.281 | 1.00 | 32.54 | C |
| ATOM | 2066 | CG1 | ILE | A | 314 | 17.447 | 55.439 | 45.291 | 0.00 | 32.54 | C |
| ATOM | 2067 | CG2 | ILE | A | 314 | 15.371 | 54.758 | 46.507 | 1.00 | 32.54 | C |
| ATOM | 2068 | CD1 | ILE | A | 314 | 17.088 | 56.908 | 45.242 | 0.00 | 32.54 | C |
| ATOM | 2069 | N | ALA | A | 315 | 15.108 | 51.850 | 43.884 | 1.00 | 30.21 | N |
| ATOM | 2070 | CA | ALA | A | 315 | 13.975 | 50.969 | 43.626 | 1.00 | 30.21 | C |
| ATOM | 2071 | C | ALA | A | 315 | 14.271 | 49.552 | 44.102 | 1.00 | 30.21 | C |
| ATOM | 2072 | O | ALA | A | 315 | 13.484 | 48.973 | 44.840 | 1.00 | 30.21 | O |
| ATOM | 2073 | CB | ALA | A | 315 | 13.646 | 50.963 | 42.143 | 1.00 | 24.26 | C |
| ATOM | 2074 | N | LEU | A | 316 | 15.406 | 49.002 | 43.667 | 1.00 | 29.43 | N |
| ATOM | 2075 | CA | LEU | A | 316 | 15.836 | 47.652 | 44.037 | 1.00 | 29.43 | C |
| ATOM | 2076 | C | LEU | A | 316 | 15.888 | 47.500 | 45.556 | 1.00 | 29.43 | C |
| ATOM | 2077 | O | LEU | A | 316 | 15.361 | 46.533 | 46.110 | 1.00 | 29.43 | O |
| ATOM | 2078 | CB | LEU | A | 316 | 17.218 | 47.342 | 43.433 | 1.00 | 23.31 | C |
| ATOM | 2079 | CG | LEU | A | 316 | 17.832 | 45.970 | 43.756 | 1.00 | 23.31 | C |
| ATOM | 2080 | CD1 | LEU | A | 316 | 17.018 | 44.852 | 43.137 | 1.00 | 23.31 | C |
| ATOM | 2081 | CD2 | LEU | A | 316 | 19.256 | 45.914 | 43.247 | 1.00 | 23.31 | C |
| ATOM | 2082 | N | CYS | A | 317 | 16.518 | 48.456 | 46.229 | 1.00 | 29.07 | N |
| ATOM | 2083 | CA | CYS | A | 317 | 16.601 | 48.396 | 47.683 | 1.00 | 29.07 | C |
| ATOM | 2084 | C | CYS | A | 317 | 15.230 | 48.137 | 48.287 | 1.00 | 29.07 | C |
| ATOM | 2085 | O | CYS | A | 317 | 15.075 | 47.264 | 49.132 | 1.00 | 29.07 | O |
| ATOM | 2086 | CB | CYS | A | 317 | 17.147 | 49.703 | 48.260 | 1.00 | 36.87 | C |
| ATOM | 2087 | SG | CYS | A | 317 | 18.896 | 49.960 | 48.001 | 1.00 | 36.87 | S |
| ATOM | 2088 | N | SER | A | 318 | 14.243 | 48.903 | 47.837 | 1.00 | 31.67 | N |
| ATOM | 2089 | CA | SER | A | 318 | 12.884 | 48.792 | 48.332 | 1.00 | 31.67 | C |
| ATOM | 2090 | C | SER | A | 318 | 12.255 | 47.433 | 48.065 | 1.00 | 31.67 | C |
| ATOM | 2091 | O | SER | A | 318 | 11.400 | 46.985 | 48.833 | 1.00 | 31.67 | O |
| ATOM | 2092 | CB | SER | A | 318 | 12.014 | 49.878 | 47.709 | 1.00 | 44.38 | C |
| ATOM | 2093 | OG | SER | A | 318 | 11.875 | 49.661 | 46.310 | 1.00 | 44.38 | O |
| ATOM | 2094 | N | ARG | A | 319 | 12.658 | 46.779 | 46.979 | 1.00 | 31.95 | N |
| ATOM | 2095 | CA | ARG | A | 319 | 12.103 | 45.467 | 46.653 | 1.00 | 31.95 | C |
| ATOM | 2096 | C | ARG | A | 319 | 12.905 | 44.356 | 47.320 | 1.00 | 31.95 | C |
| ATOM | 2097 | O | ARG | A | 319 | 12.592 | 43.180 | 47.159 | 1.00 | 31.95 | O |
| ATOM | 2098 | CB | ARG | A | 319 | 12.079 | 45.248 | 45.140 | 1.00 | 39.24 | C |
| ATOM | 2099 | CG | ARG | A | 319 | 11.378 | 46.352 | 44.355 | 1.00 | 39.24 | C |
| ATOM | 2100 | CD | ARG | A | 319 | 9.861 | 46.361 | 44.522 | 1.00 | 39.24 | C |
| ATOM | 2101 | NE | ARG | A | 319 | 9.240 | 45.174 | 43.940 | 1.00 | 39.24 | N |
| ATOM | 2102 | CZ | ARG | A | 319 | 9.037 | 44.032 | 44.594 | 1.00 | 39.24 | C |
| ATOM | 2103 | NH1 | ARG | A | 319 | 9.395 | 43.912 | 45.869 | 1.00 | 39.24 | N |
| ATOM | 2104 | NH2 | ARG | A | 319 | 8.496 | 42.995 | 43.967 | 1.00 | 39.24 | N |

| | | | | | | | | | | |
|---|---|---|---|---|---|---|---|---|---|---|
| ATOM | 2105 | N | LEU | A | 320 | 13.944 | 44.730 | 48.061 | 1.00 31.81 | N |
| ATOM | 2106 | CA | LEU | A | 320 | 14.756 | 43.749 | 48.767 | 1.00 31.81 | C |
| ATOM | 2107 | C | LEU | A | 320 | 14.476 | 43.811 | 50.264 | 1.00 31.81 | C |
| ATOM | 2108 | O | LEU | A | 320 | 14.458 | 42.788 | 50.947 | 1.00 31.81 | O |
| ATOM | 2109 | CB | LEU | A | 320 | 16.240 | 44.005 | 48.529 | 1.00 25.62 | C |
| ATOM | 2110 | CG | LEU | A | 320 | 16.820 | 43.750 | 47.137 | 1.00 25.62 | C |
| ATOM | 2111 | CD1 | LEU | A | 320 | 18.204 | 44.341 | 47.072 | 1.00 25.62 | C |
| ATOM | 2112 | CD2 | LEU | A | 320 | 16.871 | 42.270 | 46.846 | 1.00 25.62 | C |
| ATOM | 2113 | N | LEU | A | 321 | 14.249 | 45.018 | 50.768 | 1.00 29.10 | N |
| ATOM | 2114 | CA | LEU | A | 321 | 14.014 | 45.217 | 52.195 | 1.00 29.10 | C |
| ATOM | 2115 | C | LEU | A | 321 | 12.521 | 45.330 | 52.511 | 1.00 29.10 | C |
| ATOM | 2116 | O | LEU | A | 321 | 11.984 | 46.402 | 52.763 | 1.00 29.10 | O |
| ATOM | 2117 | CB | LEU | A | 321 | 14.735 | 46.496 | 52.625 | 1.00 21.28 | C |
| ATOM | 2118 | CG | LEU | A | 321 | 16.250 | 46.410 | 52.432 | 1.00 21.28 | C |
| ATOM | 2119 | CD1 | LEU | A | 321 | 16.970 | 47.680 | 52.889 | 1.00 21.28 | C |
| ATOM | 2120 | CD2 | LEU | A | 321 | 16.883 | 45.255 | 53.208 | 1.00 21.28 | C |
| ATOM | 2121 | N | GLU | A | 322 | 11.838 | 44.173 | 52.455 | 1.00 39.08 | N |
| ATOM | 2122 | CA | GLU | A | 322 | 10.405 | 44.172 | 52.723 | 1.00 39.08 | C |
| ATOM | 2123 | C | GLU | A | 322 | 10.063 | 43.364 | 53.975 | 1.00 39.08 | C |
| ATOM | 2124 | O | GLU | A | 322 | 10.642 | 42.324 | 54.262 | 1.00 39.08 | O |
| ATOM | 2125 | CB | GLU | A | 322 | 9.696 | 43.570 | 51.510 | 1.00 55.55 | C |
| ATOM | 2126 | CG | GLU | A | 322 | 10.113 | 44.238 | 50.199 | 1.00 55.55 | C |
| ATOM | 2127 | CD | GLU | A | 322 | 8.938 | 45.000 | 49.630 | 1.00 55.55 | C |
| ATOM | 2128 | OE1 | GLU | A | 322 | 8.070 | 45.396 | 50.395 | 1.00 55.55 | O |
| ATOM | 2129 | OE2 | GLU | A | 322 | 8.896 | 45.185 | 48.415 | 1.00 55.55 | O |
| ATOM | 2130 | N | TYR | A | 323 | 9.110 | 43.902 | 54.758 | 1.00 30.49 | N |
| ATOM | 2131 | CA | TYR | A | 323 | 8.663 | 43.184 | 55.945 | 1.00 30.49 | C |
| ATOM | 2132 | C | TYR | A | 323 | 8.285 | 41.741 | 55.607 | 1.00 30.49 | C |
| ATOM | 2133 | O | TYR | A | 323 | 8.700 | 40.786 | 56.251 | 1.00 30.49 | O |
| ATOM | 2134 | CB | TYR | A | 323 | 7.450 | 43.918 | 56.517 | 1.00 33.61 | C |
| ATOM | 2135 | CG | TYR | A | 323 | 7.899 | 45.110 | 57.284 | 1.00 33.61 | C |
| ATOM | 2136 | CD1 | TYR | A | 323 | 7.251 | 46.330 | 57.113 | 1.00 33.61 | C |
| ATOM | 2137 | CD2 | TYR | A | 323 | 8.964 | 45.014 | 58.180 | 1.00 33.61 | C |
| ATOM | 2138 | CE1 | TYR | A | 323 | 7.662 | 47.444 | 57.829 | 1.00 33.61 | C |
| ATOM | 2139 | CE2 | TYR | A | 323 | 9.375 | 46.128 | 58.897 | 1.00 33.61 | C |
| ATOM | 2140 | CZ | TYR | A | 323 | 8.729 | 47.338 | 58.723 | 1.00 33.61 | C |
| ATOM | 2141 | OH | TYR | A | 323 | 9.119 | 48.448 | 59.448 | 1.00 33.61 | O |
| ATOM | 2142 | N | THR | A | 324 | 7.429 | 41.608 | 54.576 | 1.00 27.56 | N |
| ATOM | 2143 | CA | THR | A | 324 | 7.040 | 40.276 | 54.133 | 1.00 27.56 | C |
| ATOM | 2144 | C | THR | A | 324 | 8.206 | 39.558 | 53.453 | 1.00 27.56 | C |
| ATOM | 2145 | O | THR | A | 324 | 8.640 | 39.913 | 52.365 | 1.00 27.56 | O |
| ATOM | 2146 | CB | THR | A | 324 | 5.878 | 40.423 | 53.150 | 1.00 33.13 | C |
| ATOM | 2147 | OG1 | THR | A | 324 | 4.840 | 41.191 | 53.762 | 1.00 33.13 | O |
| ATOM | 2148 | CG2 | THR | A | 324 | 5.322 | 39.043 | 52.785 | 1.00 33.13 | C |
| ATOM | 2149 | N | PRO | A | 325 | 8.718 | 38.515 | 54.106 | 1.00 37.10 | N |
| ATOM | 2150 | CA | PRO | A | 325 | 9.844 | 37.756 | 53.566 | 1.00 37.10 | C |
| ATOM | 2151 | C | PRO | A | 325 | 9.556 | 37.224 | 52.172 | 1.00 37.10 | C |
| ATOM | 2152 | O | PRO | A | 325 | 10.434 | 37.166 | 51.310 | 1.00 37.10 | O |
| ATOM | 2153 | CB | PRO | A | 325 | 10.021 | 36.634 | 54.591 | 1.00 24.40 | C |
| ATOM | 2154 | CG | PRO | A | 325 | 9.476 | 37.230 | 55.853 | 1.00 24.40 | C |
| ATOM | 2155 | CD | PRO | A | 325 | 8.241 | 37.916 | 55.359 | 1.00 24.40 | C |
| ATOM | 2156 | N | THR | A | 326 | 8.305 | 36.864 | 51.947 | 1.00 32.23 | N |
| ATOM | 2157 | CA | THR | A | 326 | 7.910 | 36.293 | 50.676 | 1.00 32.23 | C |
| ATOM | 2158 | C | THR | A | 326 | 7.697 | 37.318 | 49.554 | 1.00 32.23 | C |
| ATOM | 2159 | O | THR | A | 326 | 7.446 | 36.952 | 48.405 | 1.00 32.23 | O |
| ATOM | 2160 | CB | THR | A | 326 | 6.655 | 35.417 | 50.895 | 1.00 36.85 | C |
| ATOM | 2161 | OG1 | THR | A | 326 | 6.513 | 34.513 | 49.804 | 1.00 36.85 | O |
| ATOM | 2162 | CG2 | THR | A | 326 | 5.418 | 36.263 | 51.034 | 1.00 36.85 | C |
| ATOM | 2163 | N | ALA | A | 327 | 7.836 | 38.598 | 49.889 | 1.00 32.40 | N |
| ATOM | 2164 | CA | ALA | A | 327 | 7.660 | 39.676 | 48.919 | 1.00 32.40 | C |
| ATOM | 2165 | C | ALA | A | 327 | 8.993 | 40.184 | 48.374 | 1.00 32.40 | C |
| ATOM | 2166 | O | ALA | A | 327 | 9.036 | 40.934 | 47.392 | 1.00 32.40 | O |

| | | | | | | | | | | | |
|---|---|---|---|---|---|---|---|---|---|---|---|
| ATOM | 2167 | CB | ALA | A | 327 | 6.896 | 40.826 | 49.552 | 1.00 | 22.71 | C |
| ATOM | 2168 | N | ARG | A | 328 | 10.081 | 39.781 | 49.018 | 1.00 | 29.48 | N |
| ATOM | 2169 | CA | ARG | A | 328 | 11.403 | 40.195 | 48.586 | 1.00 | 29.48 | C |
| ATOM | 2170 | C | ARG | A | 328 | 11.739 | 39.497 | 47.268 | 1.00 | 29.48 | C |
| ATOM | 2171 | O | ARG | A | 328 | 11.318 | 38.366 | 47.020 | 1.00 | 29.48 | O |
| ATOM | 2172 | CB | ARG | A | 328 | 12.439 | 39.842 | 49.657 | 1.00 | 24.82 | C |
| ATOM | 2173 | CG | ARG | A | 328 | 12.228 | 40.558 | 50.979 | 1.00 | 24.82 | C |
| ATOM | 2174 | CD | ARG | A | 328 | 12.873 | 39.823 | 52.139 | 1.00 | 24.82 | C |
| ATOM | 2175 | NE | ARG | A | 328 | 12.317 | 40.282 | 53.413 | 1.00 | 24.82 | N |
| ATOM | 2176 | CZ | ARG | A | 328 | 12.558 | 39.724 | 54.598 | 1.00 | 24.82 | C |
| ATOM | 2177 | NH1 | ARG | A | 328 | 13.349 | 38.671 | 54.699 | 1.00 | 24.82 | N |
| ATOM | 2178 | NH2 | ARG | A | 328 | 12.000 | 40.222 | 55.687 | 1.00 | 24.82 | N |
| ATOM | 2179 | N | LEU | A | 329 | 12.472 | 40.196 | 46.411 | 1.00 | 24.91 | N |
| ATOM | 2180 | CA | LEU | A | 329 | 12.879 | 39.646 | 45.132 | 1.00 | 24.91 | C |
| ATOM | 2181 | C | LEU | A | 329 | 13.737 | 38.443 | 45.415 | 1.00 | 24.91 | C |
| ATOM | 2182 | O | LEU | A | 329 | 14.281 | 38.299 | 46.507 | 1.00 | 24.91 | O |
| ATOM | 2183 | CB | LEU | A | 329 | 13.704 | 40.667 | 44.354 | 1.00 | 35.89 | C |
| ATOM | 2184 | CG | LEU | A | 329 | 12.990 | 41.653 | 43.431 | 1.00 | 35.89 | C |
| ATOM | 2185 | CD1 | LEU | A | 329 | 11.593 | 41.992 | 43.947 | 1.00 | 35.89 | C |
| ATOM | 2186 | CD2 | LEU | A | 329 | 13.863 | 42.900 | 43.308 | 1.00 | 35.89 | C |
| ATOM | 2187 | N | THR | A | 330 | 13.854 | 37.575 | 44.427 | 1.00 | 27.02 | N |
| ATOM | 2188 | CA | THR | A | 330 | 14.686 | 36.405 | 44.581 | 1.00 | 27.02 | C |
| ATOM | 2189 | C | THR | A | 330 | 16.005 | 36.805 | 43.964 | 1.00 | 27.02 | C |
| ATOM | 2190 | O | THR | A | 330 | 16.066 | 37.733 | 43.163 | 1.00 | 27.02 | O |
| ATOM | 2191 | CB | THR | A | 330 | 14.141 | 35.218 | 43.814 | 1.00 | 37.14 | C |
| ATOM | 2192 | OG1 | THR | A | 330 | 14.347 | 35.429 | 42.411 | 1.00 | 37.14 | O |
| ATOM | 2193 | CG2 | THR | A | 330 | 12.658 | 35.054 | 44.101 | 1.00 | 37.14 | C |
| ATOM | 2194 | N | PRO | A | 331 | 17.086 | 36.121 | 44.338 | 1.00 | 26.91 | N |
| ATOM | 2195 | CA | PRO | A | 331 | 18.396 | 36.453 | 43.783 | 1.00 | 26.91 | C |
| ATOM | 2196 | C | PRO | A | 331 | 18.392 | 36.654 | 42.268 | 1.00 | 26.91 | C |
| ATOM | 2197 | O | PRO | A | 331 | 18.976 | 37.617 | 41.764 | 1.00 | 26.91 | O |
| ATOM | 2198 | CB | PRO | A | 331 | 19.256 | 35.277 | 44.229 | 1.00 | 24.45 | C |
| ATOM | 2199 | CG | PRO | A | 331 | 18.683 | 34.975 | 45.582 | 1.00 | 24.45 | C |
| ATOM | 2200 | CD | PRO | A | 331 | 17.189 | 35.048 | 45.340 | 1.00 | 24.45 | C |
| ATOM | 2201 | N | LEU | A | 332 | 17.729 | 35.758 | 41.541 | 1.00 | 33.38 | N |
| ATOM | 2202 | CA | LEU | A | 332 | 17.682 | 35.885 | 40.092 | 1.00 | 33.38 | C |
| ATOM | 2203 | C | LEU | A | 332 | 16.915 | 37.123 | 39.688 | 1.00 | 33.38 | C |
| ATOM | 2204 | O | LEU | A | 332 | 17.292 | 37.790 | 38.735 | 1.00 | 33.38 | O |
| ATOM | 2205 | CB | LEU | A | 332 | 17.050 | 34.658 | 39.442 | 1.00 | 38.61 | C |
| ATOM | 2206 | CG | LEU | A | 332 | 17.919 | 33.426 | 39.163 | 1.00 | 38.61 | C |
| ATOM | 2207 | CD1 | LEU | A | 332 | 17.076 | 32.396 | 38.439 | 1.00 | 38.61 | C |
| ATOM | 2208 | CD2 | LEU | A | 332 | 19.103 | 33.792 | 38.305 | 1.00 | 38.61 | C |
| ATOM | 2209 | N | GLU | A | 333 | 15.842 | 37.435 | 40.408 | 1.00 | 36.97 | N |
| ATOM | 2210 | CA | GLU | A | 333 | 15.057 | 38.627 | 40.093 | 1.00 | 36.97 | C |
| ATOM | 2211 | C | GLU | A | 333 | 15.873 | 39.882 | 40.406 | 1.00 | 36.97 | C |
| ATOM | 2212 | O | GLU | A | 333 | 15.729 | 40.920 | 39.755 | 1.00 | 36.97 | O |
| ATOM | 2213 | CB | GLU | A | 333 | 13.738 | 38.631 | 40.880 | 1.00 | 39.30 | C |
| ATOM | 2214 | CG | GLU | A | 333 | 12.857 | 37.429 | 40.567 | 1.00 | 39.30 | C |
| ATOM | 2215 | CD | GLU | A | 333 | 11.646 | 37.303 | 41.486 | 1.00 | 39.30 | C |
| ATOM | 2216 | OE1 | GLU | A | 333 | 11.776 | 37.603 | 42.691 | 1.00 | 39.30 | O |
| ATOM | 2217 | OE2 | GLU | A | 333 | 10.567 | 36.880 | 41.010 | 1.00 | 39.30 | O |
| ATOM | 2218 | N | ALA | A | 334 | 16.745 | 39.780 | 41.397 | 1.00 | 32.12 | N |
| ATOM | 2219 | CA | ALA | A | 334 | 17.582 | 40.908 | 41.762 | 1.00 | 32.12 | C |
| ATOM | 2220 | C | ALA | A | 334 | 18.535 | 41.136 | 40.608 | 1.00 | 32.12 | C |
| ATOM | 2221 | O | ALA | A | 334 | 18.742 | 42.260 | 40.190 | 1.00 | 32.12 | O |
| ATOM | 2222 | CB | ALA | A | 334 | 18.347 | 40.604 | 43.040 | 1.00 | 22.68 | C |
| ATOM | 2223 | N | CYS | A | 335 | 19.092 | 40.055 | 40.080 | 1.00 | 31.34 | N |
| ATOM | 2224 | CA | CYS | A | 335 | 20.026 | 40.129 | 38.958 | 1.00 | 31.34 | C |
| ATOM | 2225 | C | CYS | A | 335 | 19.455 | 40.746 | 37.695 | 1.00 | 31.34 | C |
| ATOM | 2226 | O | CYS | A | 335 | 20.172 | 41.406 | 36.943 | 1.00 | 31.34 | O |
| ATOM | 2227 | CB | CYS | A | 335 | 20.530 | 38.739 | 38.590 | 1.00 | 28.29 | C |
| ATOM | 2228 | SG | CYS | A | 335 | 21.696 | 38.049 | 39.729 | 1.00 | 28.29 | S |

| | | | | | | | | | | | |
|---|---|---|---|---|---|---|---|---|---|---|---|
| ATOM | 2229 | N | ALA | A | 336 | 18.172 | 40.506 | 37.452 | 1.00 | 25.38 | N |
| ATOM | 2230 | CA | ALA | A | 336 | 17.507 | 41.009 | 36.257 | 1.00 | 25.38 | C |
| ATOM | 2231 | C | ALA | A | 336 | 16.890 | 42.385 | 36.449 | 1.00 | 25.38 | C |
| ATOM | 2232 | O | ALA | A | 336 | 16.183 | 42.884 | 35.572 | 1.00 | 25.38 | O |
| ATOM | 2233 | CB | ALA | A | 336 | 16.443 | 40.020 | 35.816 | 1.00 | 20.49 | C |
| ATOM | 2234 | N | HIS | A | 337 | 17.150 | 42.990 | 37.600 | 1.00 | 25.00 | N |
| ATOM | 2235 | CA | HIS | A | 337 | 16.610 | 44.307 | 37.881 | 1.00 | 25.00 | C |
| ATOM | 2236 | C | HIS | A | 337 | 17.274 | 45.325 | 36.960 | 1.00 | 25.00 | C |
| ATOM | 2237 | O | HIS | A | 337 | 18.437 | 45.164 | 36.583 | 1.00 | 25.00 | O |
| ATOM | 2238 | CB | HIS | A | 337 | 16.847 | 44.691 | 39.345 | 1.00 | 25.76 | C |
| ATOM | 2239 | CG | HIS | A | 337 | 16.185 | 45.975 | 39.738 | 1.00 | 25.76 | C |
| ATOM | 2240 | ND1 | HIS | A | 337 | 14.927 | 46.022 | 40.296 | 1.00 | 25.76 | N |
| ATOM | 2241 | CD2 | HIS | A | 337 | 16.579 | 47.260 | 39.593 | 1.00 | 25.76 | C |
| ATOM | 2242 | CE1 | HIS | A | 337 | 14.575 | 47.281 | 40.478 | 1.00 | 25.76 | C |
| ATOM | 2243 | NE2 | HIS | A | 337 | 15.559 | 48.054 | 40.059 | 1.00 | 25.76 | N |
| ATOM | 2244 | N | SER | A | 338 | 16.535 | 46.369 | 36.598 | 1.00 | 33.10 | N |
| ATOM | 2245 | CA | SER | A | 338 | 17.068 | 47.392 | 35.705 | 1.00 | 33.10 | C |
| ATOM | 2246 | C | SER | A | 338 | 18.389 | 47.987 | 36.196 | 1.00 | 33.10 | C |
| ATOM | 2247 | O | SER | A | 338 | 19.247 | 48.336 | 35.386 | 1.00 | 33.10 | O |
| ATOM | 2248 | CB | SER | A | 338 | 16.030 | 48.498 | 35.477 | 1.00 | 46.43 | C |
| ATOM | 2249 | OG | SER | A | 338 | 15.598 | 49.057 | 36.705 | 1.00 | 46.43 | O |
| ATOM | 2250 | N | PHE | A | 339 | 18.553 | 48.086 | 37.512 | 1.00 | 25.39 | N |
| ATOM | 2251 | CA | PHE | A | 339 | 19.783 | 48.612 | 38.108 | 1.00 | 25.39 | C |
| ATOM | 2252 | C | PHE | A | 339 | 21.048 | 47.945 | 37.557 | 1.00 | 25.39 | C |
| ATOM | 2253 | O | PHE | A | 339 | 22.129 | 48.526 | 37.588 | 1.00 | 25.39 | O |
| ATOM | 2254 | CB | PHE | A | 339 | 19.757 | 48.434 | 39.634 | 1.00 | 25.22 | C |
| ATOM | 2255 | CG | PHE | A | 339 | 20.991 | 48.941 | 40.327 | 1.00 | 25.22 | C |
| ATOM | 2256 | CD1 | PHE | A | 339 | 21.347 | 50.284 | 40.248 | 1.00 | 25.22 | C |
| ATOM | 2257 | CD2 | PHE | A | 339 | 21.814 | 48.069 | 41.032 | 1.00 | 25.22 | C |
| ATOM | 2258 | CE1 | PHE | A | 339 | 22.499 | 50.755 | 40.854 | 1.00 | 25.22 | C |
| ATOM | 2259 | CE2 | PHE | A | 339 | 22.974 | 48.529 | 41.647 | 1.00 | 25.22 | C |
| ATOM | 2260 | CZ | PHE | A | 339 | 23.316 | 49.881 | 41.555 | 1.00 | 25.22 | C |
| ATOM | 2261 | N | PHE | A | 340 | 20.913 | 46.726 | 37.053 | 1.00 | 26.97 | N |
| ATOM | 2262 | CA | PHE | A | 340 | 22.056 | 46.016 | 36.512 | 1.00 | 26.97 | C |
| ATOM | 2263 | C | PHE | A | 340 | 22.082 | 46.031 | 34.994 | 1.00 | 26.97 | C |
| ATOM | 2264 | O | PHE | A | 340 | 22.797 | 45.241 | 34.374 | 1.00 | 26.97 | O |
| ATOM | 2265 | CB | PHE | A | 340 | 22.056 | 44.577 | 37.010 | 1.00 | 20.05 | C |
| ATOM | 2266 | CG | PHE | A | 340 | 22.178 | 44.461 | 38.494 | 1.00 | 20.05 | C |
| ATOM | 2267 | CD1 | PHE | A | 340 | 23.372 | 44.777 | 39.132 | 1.00 | 20.05 | C |
| ATOM | 2268 | CD2 | PHE | A | 340 | 21.094 | 44.058 | 39.265 | 1.00 | 20.05 | C |
| ATOM | 2269 | CE1 | PHE | A | 340 | 23.485 | 44.691 | 40.511 | 1.00 | 20.05 | C |
| ATOM | 2270 | CE2 | PHE | A | 340 | 21.197 | 43.969 | 40.645 | 1.00 | 20.05 | C |
| ATOM | 2271 | CZ | PHE | A | 340 | 22.394 | 44.285 | 41.270 | 1.00 | 20.05 | C |
| ATOM | 2272 | N | ASP | A | 341 | 21.312 | 46.930 | 34.384 | 1.00 | 31.18 | N |
| ATOM | 2273 | CA | ASP | A | 341 | 21.283 | 46.986 | 32.929 | 1.00 | 31.18 | C |
| ATOM | 2274 | C | ASP | A | 341 | 22.637 | 47.374 | 32.335 | 1.00 | 31.18 | C |
| ATOM | 2275 | O | ASP | A | 341 | 23.035 | 46.834 | 31.314 | 1.00 | 31.18 | O |
| ATOM | 2276 | CB | ASP | A | 341 | 20.173 | 47.933 | 32.437 | 1.00 | 36.78 | C |
| ATOM | 2277 | CG | ASP | A | 341 | 18.795 | 47.262 | 32.425 | 1.00 | 36.78 | C |
| ATOM | 2278 | OD1 | ASP | A | 341 | 18.759 | 46.014 | 32.490 | 1.00 | 36.78 | O |
| ATOM | 2279 | OD2 | ASP | A | 341 | 17.752 | 47.961 | 32.335 | 1.00 | 36.78 | O |
| ATOM | 2280 | N | GLU | A | 342 | 23.354 | 48.293 | 32.973 | 1.00 | 28.84 | N |
| ATOM | 2281 | CA | GLU | A | 342 | 24.663 | 48.704 | 32.463 | 1.00 | 28.84 | C |
| ATOM | 2282 | C | GLU | A | 342 | 25.551 | 47.462 | 32.293 | 1.00 | 28.84 | C |
| ATOM | 2283 | O | GLU | A | 342 | 26.273 | 47.336 | 31.312 | 1.00 | 28.84 | O |
| ATOM | 2284 | CB | GLU | A | 342 | 25.339 | 49.697 | 33.438 | 1.00 | 37.21 | C |
| ATOM | 2285 | CG | GLU | A | 342 | 26.453 | 50.556 | 32.831 | 1.00 | 37.21 | C |
| ATOM | 2286 | CD | GLU | A | 342 | 27.265 | 51.353 | 33.868 | 1.00 | 37.21 | C |
| ATOM | 2287 | OE1 | GLU | A | 342 | 26.684 | 51.856 | 34.854 | 1.00 | 37.21 | O |
| ATOM | 2288 | OE2 | GLU | A | 342 | 28.498 | 51.496 | 33.692 | 1.00 | 37.21 | O |
| ATOM | 2289 | N | LEU | A | 343 | 25.492 | 46.545 | 33.255 | 1.00 | 34.22 | N |
| ATOM | 2290 | CA | LEU | A | 343 | 26.311 | 45.347 | 33.202 | 1.00 | 34.22 | C |

| | | | | | | | | | | | |
|---|---|---|---|---|---|---|---|---|---|---|---|
| ATOM | 2291 | C | LEU | A | 343 | 25.972 | 44.534 | 31.966 | 1.00 | 34.22 | C |
| ATOM | 2292 | O | LEU | A | 343 | 26.816 | 43.815 | 31.428 | 1.00 | 34.22 | O |
| ATOM | 2293 | CB | LEU | A | 343 | 26.091 | 44.499 | 34.456 | 1.00 | 33.33 | C |
| ATOM | 2294 | CG | LEU | A | 343 | 26.357 | 45.196 | 35.790 | 1.00 | 33.33 | C |
| ATOM | 2295 | CD1 | LEU | A | 343 | 26.132 | 44.227 | 36.942 | 1.00 | 33.33 | C |
| ATOM | 2296 | CD2 | LEU | A | 343 | 27.783 | 45.727 | 35.806 | 1.00 | 33.33 | C |
| ATOM | 2297 | N | ARG | A | 344 | 24.730 | 44.646 | 31.518 | 1.00 | 38.25 | N |
| ATOM | 2298 | CA | ARG | A | 344 | 24.304 | 43.908 | 30.350 | 1.00 | 38.25 | C |
| ATOM | 2299 | C | ARG | A | 344 | 24.775 | 44.565 | 29.056 | 1.00 | 38.25 | C |
| ATOM | 2300 | O | ARG | A | 344 | 24.904 | 43.893 | 28.027 | 1.00 | 38.25 | O |
| ATOM | 2301 | CB | ARG | A | 344 | 22.784 | 43.721 | 30.363 | 1.00 | 30.47 | C |
| ATOM | 2302 | CG | ARG | A | 344 | 22.348 | 42.457 | 31.114 | 1.00 | 30.47 | C |
| ATOM | 2303 | CD | ARG | A | 344 | 20.839 | 42.242 | 31.113 | 1.00 | 30.47 | C |
| ATOM | 2304 | NE | ARG | A | 344 | 20.145 | 43.139 | 32.032 | 1.00 | 30.47 | N |
| ATOM | 2305 | CZ | ARG | A | 344 | 20.171 | 43.027 | 33.356 | 1.00 | 30.47 | C |
| ATOM | 2306 | NH1 | ARG | A | 344 | 20.858 | 42.051 | 33.935 | 1.00 | 30.47 | N |
| ATOM | 2307 | NH2 | ARG | A | 344 | 19.497 | 43.889 | 34.103 | 1.00 | 30.47 | N |
| ATOM | 2308 | N | ASP | A | 345 | 25.057 | 45.866 | 29.109 | 1.00 | 36.28 | N |
| ATOM | 2309 | CA | ASP | A | 345 | 25.540 | 46.589 | 27.936 | 1.00 | 36.28 | C |
| ATOM | 2310 | C | ASP | A | 345 | 26.738 | 45.860 | 27.328 | 1.00 | 36.28 | C |
| ATOM | 2311 | O | ASP | A | 345 | 27.570 | 45.303 | 28.038 | 1.00 | 36.28 | O |
| ATOM | 2312 | CB | ASP | A | 345 | 25.968 | 48.001 | 28.322 | 1.00 | 54.81 | C |
| ATOM | 2313 | CG | ASP | A | 345 | 26.315 | 48.869 | 27.109 | 1.00 | 54.81 | C |
| ATOM | 2314 | OD1 | ASP | A | 345 | 27.238 | 48.503 | 26.331 | 1.00 | 54.81 | O |
| ATOM | 2315 | OD2 | ASP | A | 345 | 25.658 | 49.927 | 26.946 | 1.00 | 54.81 | O |
| ATOM | 2316. | N | PRO | A | 346 | 26.836 | 45.844 | 25.997 | 1.00 | 36.28 | N |
| ATOM | 2317 | CA | PRO | A | 346 | 27.975 | 45.154 | 25.392 | 1.00 | 36.28 | C |
| ATOM | 2318 | C | PRO | A | 346 | 29.270 | 45.958 | 25.436 | 1.00 | 36.28 | C |
| ATOM | 2319 | O | PRO | A | 346 | 30.351 | 45.376 | 25.403 | 1.00 | 36.28 | O |
| ATOM | 2320 | CB | PRO | A | 346 | 27.502 | 44.910 | 23.967 | 1.00 | 29.59 | C |
| ATOM | 2321 | CG | PRO | A | 346 | 26.639 | 46.105 | 23.706 | 1.00 | 29.59 | C |
| ATOM | 2322 | CD | PRO | A | 346 | 25.834 | 46.205 | 24.979 | 1.00 | 29.59 | C |
| ATOM | 2323 | N | ASN | A | 347 | 29.166 | 47.285 | 25.519 | 1.00 | 50.30 | N |
| ATOM | 2324 | CA | ASN | A | 347 | 30.358 | 48.140 | 25.535 | 1.00 | 50.30 | C |
| ATOM | 2325 | C | ASN | A | 347 | 30.938 | 48.462 | 26.918 | 1.00 | 50.30 | C |
| ATOM | 2326 | O | ASN | A | 347 | 31.947 | 49.171 | 27.024 | 1.00 | 50.30 | O |
| ATOM | 2327 | CB | ASN | A | 347 | 30.074 | 49.459 | 24.807 | 0.00 | 52.63 | C |
| ATOM | 2328 | CG | ASN | A | 347 | 29.667 | 49.256 | 23.360 | 1.00 | 52.63 | C |
| ATOM | 2329 | OD1 | ASN | A | 347 | 28.632 | 49.764 | 22.925 | 1.00 | 52.63 | O |
| ATOM | 2330 | ND2 | ASN | A | 347 | 30.478 | 48.514 | 22.604 | 1.00 | 52.63 | N |
| ATOM | 2331 | N | VAL | A | 348 | 30.330 | 47.928 | 27.974 | 1.00 | 43.46 | N |
| ATOM | 2332 | CA | VAL | A | 348 | 30.793 | 48.218 | 29.324 | 1.00 | 43.46 | C |
| ATOM | 2333 | C | VAL | A | 348 | 32.133 | 47.586 | 29.694 | 1.00 | 43.46 | C |
| ATOM | 2334 | O | VAL | A | 348 | 32.438 | 46.454 | 29.325 | 1.00 | 43.46 | O |
| ATOM | 2335 | CB | VAL | A | 348 | 29.728 | 47.790 | 30.382 | 1.00 | 52.14 | C |
| ATOM | 2336 | CG1 | VAL | A | 348 | 29.686 | 46.256 | 30.508 | 1.00 | 52.14 | C |
| ATOM | 2337 | CG2 | VAL | A | 348 | 30.030 | 48.452 | 31.731 | 1.00 | 52.14 | C |
| ATOM | 2338 | N | LYS | A | 349 | 32.929 | 48.343 | 30.434 | 1.00 | 40.96 | N |
| ATOM | 2339 | CA | LYS | A | 349 | 34.225 | 47.876 | 30.897 | 1.00 | 40.96 | C |
| ATOM | 2340 | C | LYS | A | 349 | 34.479 | 48.496 | 32.270 | 1.00 | 40.96 | C |
| ATOM | 2341 | O | LYS | A | 349 | 33.711 | 49.339 | 32.731 | 1.00 | 40.96 | O |
| ATOM | 2342 | CB | LYS | A | 349 | 35.314 | 48.284 | 29.905 | 1.00 | 49.14 | C |
| ATOM | 2343 | CG | LYS | A | 349 | 34.986 | 47.877 | 28.461 | 1.00 | 49.14 | C |
| ATOM | 2344 | CD | LYS | A | 349 | 36.244 | 47.631 | 27.632 | 1.00 | 49.14 | C |
| ATOM | 2345 | CE | LYS | A | 349 | 37.016 | 46.420 | 28.149 | 1.00 | 49.14 | C |
| ATOM | 2346 | NZ | LYS | A | 349 | 38.397 | 46.300 | 27.585 | 1.00 | 49.14 | N |
| ATOM | 2347 | N | LEU | A | 350 | 35.538 | 48.071 | 32.938 | 1.00 | 38.10 | N |
| ATOM | 2348 | CA | LEU | A | 350 | 35.843 | 48.624 | 34.245 | 1.00 | 38.10 | C |
| ATOM | 2349 | C | LEU | A | 350 | 36.566 | 49.929 | 34.006 | 1.00 | 38.10 | C |
| ATOM | 2350 | O | LEU | A | 350 | 37.191 | 50.097 | 32.962 | 1.00 | 38.10 | O |
| ATOM | 2351 | CB | LEU | A | 350 | 36.736 | 47.662 | 35.011 | 1.00 | 30.19 | C |
| ATOM | 2352 | CG | LEU | A | 350 | 36.202 | 46.227 | 35.043 | 1.00 | 30.19 | C |

| | | | | | | | | | | | |
|---|---|---|---|---|---|---|---|---|---|---|---|
| ATOM | 2353 | CD1 | LEU | A | 350 | 37.156 | 45.354 | 35.830 | 1.00 | 30.19 | C |
| ATOM | 2354 | CD2 | LEU | A | 350 | 34.816 | 46.199 | 35.651 | 1.00 | 30.19 | C |
| ATOM | 2355 | N | PRO | A | 351 | 36.498 | 50.872 | 34.962 | 1.00 | 48.81 | N |
| ATOM | 2356 | CA | PRO | A | 351 | 37.180 | 52.168 | 34.795 | 1.00 | 48.81 | C |
| ATOM | 2357 | C | PRO | A | 351 | 38.595 | 51.859 | 34.362 | 1.00 | 48.81 | C |
| ATOM | 2358 | O | PRO | A | 351 | 39.256 | 52.621 | 33.668 | 1.00 | 48.81 | O |
| ATOM | 2359 | CB | PRO | A | 351 | 37.137 | 52.755 | 36.197 | 1.00 | 41.63 | C |
| ATOM | 2360 | CG | PRO | A | 351 | 37.230 | 51.547 | 37.045 | 1.00 | 41.63 | C |
| ATOM | 2361 | CD | PRO | A | 351 | 36.233 | 50.616 | 36.385 | 1.00 | 41.63 | C |
| ATOM | 2362 | N | ASN | A | 352 | 39.006 | 50.683 | 34.800 | 1.00 | 44.98 | N |
| ATOM | 2363 | CA | ASN | A | 352 | 40.288 | 50.068 | 34.557 | 1.00 | 44.98 | C |
| ATOM | 2364 | C | ASN | A | 352 | 40.641 | 49.972 | 33.066 | 1.00 | 44.98 | C |
| ATOM | 2365 | O | ASN | A | 352 | 41.800 | 50.140 | 32.672 | 1.00 | 44.98 | O |
| ATOM | 2366 | CB | ASN | A | 352 | 40.209 | 48.663 | 35.143 | 1.00 | 62.78 | C |
| ATOM | 2367 | CG | ASN | A | 352 | 41.538 | 48.063 | 35.350 | 1.00 | 62.78 | C |
| ATOM | 2368 | OD1 | ASN | A | 352 | 42.539 | 48.590 | 34.857 | 1.00 | 62.78 | O |
| ATOM | 2369 | ND2 | ASN | A | 352 | 41.584 | 46.950 | 36.081 | 1.00 | 62.78 | N |
| ATOM | 2370 | N | GLY | A | 353 | 39.634 | 49.682 | 32.244 | 1.00 | 41.80 | N |
| ATOM | 2371 | CA | GLY | A | 353 | 39.852 | 49.522 | 30.820 | 1.00 | 41.80 | C |
| ATOM | 2372 | C | GLY | A | 353 | 39.692 | 48.043 | 30.522 | 1.00 | 41.80 | C |
| ATOM | 2373 | O | GLY | A | 353 | 39.389 | 47.649 | 29.395 | 1.00 | 41.80 | O |
| ATOM | 2374 | N | ARG | A | 354 | 39.899 | 47.214 | 31.545 | 1.00 | 33.12 | N |
| ATOM | 2375 | CA | ARG | A | 354 | 39.756 | 45.762 | 31.404 | 1.00 | 33.12 | C |
| ATOM | 2376 | C | ARG | A | 354 | 38.282 | 45.402 | 31.267 | 1.00 | 33.12 | C |
| ATOM | 2377 | O | ARG | A | 354 | 37.395 | 46.218 | 31.517 | 1.00 | 33.12 | O |
| ATOM | 2378 | CB | ARG | A | 354 | 40.341 | 45.040 | 32.626 | 1.00 | 60.57 | C |
| ATOM | 2379 | CG | ARG | A | 354 | 41.776 | 45.437 | 32.924 | 1.00 | 60.57 | C |
| ATOM | 2380 | CD | ARG | A | 354 | 42.350 | 44.793 | 34.188 | 1.00 | 60.57 | C |
| ATOM | 2381 | NE | ARG | A | 354 | 42.624 | 43.364 | 34.035 | 1.00 | 60.57 | N |
| ATOM | 2382 | CZ | ARG | A | 354 | 43.483 | 42.684 | 34.800 | 1.00 | 60.57 | C |
| ATOM | 2383 | NH1 | ARG | A | 354 | 44.155 | 43.303 | 35.773 | 1.00 | 60.57 | N |
| ATOM | 2384 | NH2 | ARG | A | 354 | 43.675 | 41.383 | 34.595 | 1.00 | 60.57 | N |
| ATOM | 2385 | N | ASP | A | 355 | 38.023 | 44.170 | 30.868 | 1.00 | 31.58 | N |
| ATOM | 2386 | CA | ASP | A | 355 | 36.656 | 43.717 | 30.705 | 1.00 | 31.58 | C |
| ATOM | 2387 | C | ASP | A | 355 | 36.064 | 43.308 | 32.045 | 1.00 | 31.58 | C |
| ATOM | 2388 | O | ASP | A | 355 | 36.794 | 43.062 | 33.017 | 1.00 | 31.58 | O |
| ATOM | 2389 | CB | ASP | A | 355 | 36.617 | 42.534 | 29.740 | 1.00 | 57.55 | C |
| ATOM | 2390 | CG | ASP | A | 355 | 35.948 | 42.882 | 28.422 | 1.00 | 57.55 | C |
| ATOM | 2391 | OD1 | ASP | A | 355 | 34.694 | 42.805 | 28.340 | 1.00 | 57.55 | O |
| ATOM | 2392 | OD2 | ASP | A | 355 | 36.678 | 43.247 | 27.471 | 1.00 | 57.55 | O |
| ATOM | 2393 | N | THR | A | 356 | 34.738 | 43.245 | 32.096 | 1.00 | 22.57 | N |
| ATOM | 2394 | CA | THR | A | 356 | 34.065 | 42.845 | 33.314 | 1.00 | 22.57 | C |
| ATOM | 2395 | C | THR | A | 356 | 34.219 | 41.331 | 33.447 | 1.00 | 22.57 | C |
| ATOM | 2396 | O | THR | A | 356 | 34.465 | 40.623 | 32.474 | 1.00 | 22.57 | O |
| ATOM | 2397 | CB | THR | A | 356 | 32.552 | 43.200 | 33.297 | 1.00 | 29.12 | C |
| ATOM | 2398 | OG1 | THR | A | 356 | 31.849 | 42.315 | 32.422 | 1.00 | 29.12 | O |
| ATOM | 2399 | CG2 | THR | A | 356 | 32.339 | 44.614 | 32.832 | 1.00 | 29.12 | C |
| ATOM | 2400 | N | PRO | A | 357 | 34.099 | 40.816 | 34.667 | 1.00 | 31.50 | N |
| ATOM | 2401 | CA | PRO | A | 357 | 34.240 | 39.368 | 34.800 | 1.00 | 31.50 | C |
| ATOM | 2402 | C | PRO | A | 357 | 33.018 | 38.707 | 34.175 | 1.00 | 31.50 | C |
| ATOM | 2403 | O | PRO | A | 357 | 32.069 | 39.389 | 33.790 | 1.00 | 31.50 | O |
| ATOM | 2404 | CB | PRO | A | 357 | 34.326 | 39.177 | 36.315 | 1.00 | 41.97 | C |
| ATOM | 2405 | CG | PRO | A | 357 | 33.464 | 40.297 | 36.849 | 1.00 | 41.97 | C |
| ATOM | 2406 | CD | PRO | A | 357 | 33.852 | 41.467 | 35.969 | 1.00 | 41.97 | C |
| ATOM | 2407 | N | ALA | A | 358 | 33.039 | 37.388 | 34.050 | 1.00 | 29.10 | N |
| ATOM | 2408 | CA | ALA | A | 358 | 31.902 | 36.694 | 33.478 | 1.00 | 29.10 | C |
| ATOM | 2409 | C | ALA | A | 358 | 30.695 | 37.026 | 34.338 | 1.00 | 29.10 | C |
| ATOM | 2410 | O | ALA | A | 358 | 30.790 | 37.031 | 35.560 | 1.00 | 29.10 | O |
| ATOM | 2411 | CB | ALA | A | 358 | 32.148 | 35.191 | 33.478 | 0.00 | 35.69 | C |
| ATOM | 2412 | N | LEU | A | 359 | 29.570 | 37.322 | 33.700 | 1.00 | 28.58 | N |
| ATOM | 2413 | CA | LEU | A | 359 | 28.351 | 37.641 | 34.427 | 1.00 | 28.58 | C |
| ATOM | 2414 | C | LEU | A | 359 | 27.150 | 36.921 | 33.847 | 1.00 | 28.58 | C |

| | | | | | | | | | | | |
|---|---|---|---|---|---|---|---|---|---|---|---|
| ATOM | 2415 | O | LEU | A | 359 | 26.087 | 36.863 | 34.472 | 1.00 | 28.58 | O |
| ATOM | 2416 | CB | LEU | A | 359 | 28.078 | 39.138 | 34.378 | 1.00 | 30.76 | C |
| ATOM | 2417 | CG | LEU | A | 359 | 29.200 | 40.053 | 34.854 | 1.00 | 30.76 | C |
| ATOM | 2418 | CD1 | LEU | A | 359 | 28.704 | 41.499 | 34.831 | 1.00 | 30.76 | C |
| ATOM | 2419 | CD2 | LEU | A | 359 | 29.641 | 39.647 | 36.254 | 1.00 | 30.76 | C |
| ATOM | 2420 | N | PHE | A | 360 | 27.327 | 36.364 | 32.652 | 1.00 | 37.37 | N |
| ATOM | 2421 | CA | PHE | A | 360 | 26.248 | 35.673 | 31.957 | 1.00 | 37.37 | C |
| ATOM | 2422 | C | PHE | A | 360 | 26.391 | 34.152 | 31.756 | 1.00 | 37.37 | C |
| ATOM | 2423 | O | PHE | A | 360 | 25.471 | 33.506 | 31.266 | 1.00 | 37.37 | O |
| ATOM | 2424 | CB | PHE | A | 360 | 26.056 | 36.344 | 30.610 | 1.00 | 30.39 | C |
| ATOM | 2425 | CG | PHE | A | 360 | 26.165 | 37.831 | 30.666 | 1.00 | 30.39 | C |
| ATOM | 2426 | CD1 | PHE | A | 360 | 25.249 | 38.581 | 31.391 | 1.00 | 30.39 | C |
| ATOM | 2427 | CD2 | PHE | A | 360 | 27.182 | 38.488 | 29.987 | 1.00 | 30.39 | C |
| ATOM | 2428 | CE1 | PHE | A | 360 | 25.344 | 39.970 | 31.436 | 1.00 | 30.39 | C |
| ATOM | 2429 | CE2 | PHE | A | 360 | 27.283 | 39.873 | 30.026 | 1.00 | 30.39 | C |
| ATOM | 2430 | CZ | PHE | A | 360 | 26.359 | 40.615 | 30.755 | 1.00 | 30.39 | C |
| ATOM | 2431 | N | ASN | A | 361 | 27.528 | 33.579 | 32.129 | 1.00 | 33.59 | N |
| ATOM | 2432 | CA | ASN | A | 361 | 27.725 | 32.148 | 31.956 | 1.00 | 33.59 | C |
| ATOM | 2433 | C | ASN | A | 361 | 26.911 | 31.335 | 32.973 | 1.00 | 33.59 | C |
| ATOM | 2434 | O | ASN | A | 361 | 27.448 | 30.530 | 33.760 | 1.00 | 33.59 | O |
| ATOM | 2435 | CB | ASN | A | 361 | 29.218 | 31.822 | 32.051 | 1.00 | 42.55 | C |
| ATOM | 2436 | CG | ASN | A | 361 | 29.825 | 32.288 | 33.340 | 1.00 | 42.55 | C |
| ATOM | 2437 | OD1 | ASN | A | 361 | 29.370 | 33.277 | 33.932 | 1.00 | 42.55 | O |
| ATOM | 2438 | ND2 | ASN | A | 361 | 30.872 | 31.592 | 33.790 | 1.00 | 42.55 | N |
| ATOM | 2439 | N | PHE | A | 362 | 25.598 | 31.548 | 32.926 | 1.00 | 48.99 | N |
| ATOM | 2440 | CA | PHE | A | 362 | 24.652 | 30.880 | 33.809 | 1.00 | 48.99 | C |
| ATOM | 2441 | C | PHE | A | 362 | 24.545 | 29.378 | 33.587 | 1.00 | 48.99 | C |
| ATOM | 2442 | O | PHE | A | 362 | 24.934 | 28.871 | 32.534 | 1.00 | 48.99 | O |
| ATOM | 2443 | CB | PHE | A | 362 | 23.275 | 31.510 | 33.627 | 1.00 | 38.42 | C |
| ATOM | 2444 | CG | PHE | A | 362 | 23.124 | 32.840 | 34.320 | 1.00 | 38.42 | C |
| ATOM | 2445 | CD1 | PHE | A | 362 | 23.062 | 32.911 | 35.712 | 1.00 | 38.42 | C |
| ATOM | 2446 | CD2 | PHE | A | 362 | 23.048 | 34.023 | 33.586 | 1.00 | 38.42 | C |
| ATOM | 2447 | CE1 | PHE | A | 362 | 22.929 | 34.135 | 36.353 | 1.00 | 38.42 | C |
| ATOM | 2448 | CE2 | PHE | A | 362 | 22.914 | 35.254 | 34.228 | 1.00 | 38.42 | C |
| ATOM | 2449 | CZ | PHE | A | 362 | 22.854 | 35.305 | 35.610 | 1.00 | 38.42 | C |
| ATOM | 2450 | N | THR | A | 363 | 24.014 | 28.667 | 34.580 | 1.00 | 41.08 | N |
| ATOM | 2451 | CA | THR | A | 363 | 23.826 | 27.222 | 34.457 | 1.00 | 41.08 | C |
| ATOM | 2452 | C | THR | A | 363 | 22.365 | 26.848 | 34.677 | 1.00 | 41.08 | C |
| ATOM | 2453 | O | THR | A | 363 | 21.565 | 27.677 | 35.122 | 1.00 | 41.08 | O |
| ATOM | 2454 | CB | THR | A | 363 | 24.676 | 26.448 | 35.469 | 1.00 | 37.00 | C |
| ATOM | 2455 | OG1 | THR | A | 363 | 24.205 | 26.704 | 36.802 | 1.00 | 37.00 | O |
| ATOM | 2456 | CG2 | THR | A | 363 | 26.133 | 26.856 | 35.336 | 1.00 | 37.00 | C |
| ATOM | 2457 | N | THR | A | 364 | 22.011 | 25.611 | 34.349 | 1.00 | 41.13 | N |
| ATOM | 2458 | CA | THR | A | 364 | 20.634 | 25.151 | 34.541 | 1.00 | 41.13 | C |
| ATOM | 2459 | C | THR | A | 364 | 20.395 | 25.201 | 36.037 | 1.00 | 41.13 | C |
| ATOM | 2460 | O | THR | A | 364 | 19.319 | 25.585 | 36.504 | 1.00 | 41.13 | O |
| ATOM | 2461 | CB | THR | A | 364 | 20.436 | 23.698 | 34.061 | 1.00 | 37.94 | C |
| ATOM | 2462 | OG1 | THR | A | 364 | 21.219 | 22.813 | 34.872 | 0.00 | 37.94 | O |
| ATOM | 2463 | CG2 | THR | A | 364 | 20.857 | 23.564 | 32.610 | 1.00 | 37.94 | C |
| ATOM | 2464 | N | GLN | A | 365 | 21.431 | 24.812 | 36.773 | 1.00 | 41.29 | N |
| ATOM | 2465 | CA | GLN | A | 365 | 21.406 | 24.812 | 38.227 | 1.00 | 41.29 | C |
| ATOM | 2466 | C | GLN | A | 365 | 21.041 | 26.215 | 38.698 | 1.00 | 41.29 | C |
| ATOM | 2467 | O | GLN | A | 365 | 20.152 | 26.393 | 39.543 | 1.00 | 41.29 | O |
| ATOM | 2468 | CB | GLN | A | 365 | 22.787 | 24.435 | 38.768 | 1.00 | 48.42 | C |
| ATOM | 2469 | CG | GLN | A | 365 | 22.851 | 24.277 | 40.267 | 1.00 | 48.42 | C |
| ATOM | 2470 | CD | GLN | A | 365 | 21.817 | 23.292 | 40.769 | 1.00 | 48.42 | C |
| ATOM | 2471 | OE1 | GLN | A | 365 | 21.587 | 22.264 | 40.135 | 1.00 | 48.42 | O |
| ATOM | 2472 | NE2 | GLN | A | 365 | 21.191 | 23.589 | 41.914 | 1.00 | 48.42 | N |
| ATOM | 2473 | N | GLU | A | 366 | 21.720 | 27.211 | 38.132 | 1.00 | 37.78 | N |
| ATOM | 2474 | CA | GLU | A | 366 | 21.490 | 28.593 | 38.502 | 1.00 | 37.78 | C |
| ATOM | 2475 | C | GLU | A | 366 | 20.132 | 29.124 | 38.134 | 1.00 | 37.78 | C |
| ATOM | 2476 | O | GLU | A | 366 | 19.481 | 29.747 | 38.964 | 1.00 | 37.78 | O |

| | | | | | | | | | | | |
|---|---|---|---|---|---|---|---|---|---|---|---|
| ATOM | 2477 | CB | GLU | A | 366 | 22.533 | 29.514 | 37.871 | 1.00 | 50.83 | C |
| ATOM | 2478 | CG | GLU | A | 366 | 23.944 | 29.277 | 38.349 | 1.00 | 50.83 | C |
| ATOM | 2479 | CD | GLU | A | 366 | 24.889 | 30.390 | 37.943 | 1.00 | 50.83 | C |
| ATOM | 2480 | OE1 | GLU | A | 366 | 24.662 | 31.536 | 38.399 | 1.00 | 50.83 | O |
| ATOM | 2481 | OE2 | GLU | A | 366 | 25.851 | 30.108 | 37.181 | 1.00 | 50.83 | O |
| ATOM | 2482 | N | LEU | A | 367 | 19.701 | 28.890 | 36.901 | 1.00 | 30.92 | N |
| ATOM | 2483 | CA | LEU | A | 367 | 18.425 | 29.422 | 36.429 | 1.00 | 30.92 | C |
| ATOM | 2484 | C | LEU | A | 367 | 17.180 | 28.620 | 36.806 | 1.00 | 30.92 | C |
| ATOM | 2485 | O | LEU | A | 367 | 16.055 | 29.021 | 36.471 | 1.00 | 30.92 | O |
| ATOM | 2486 | CB | LEU | A | 367 | 18.482 | 29.558 | 34.916 | 1.00 | 29.00 | C |
| ATOM | 2487 | CG | LEU | A | 367 | 19.753 | 30.228 | 34.410 | 1.00 | 29.00 | C |
| ATOM | 2488 | CD1 | LEU | A | 367 | 20.084 | 29.713 | 33.030 | 1.00 | 29.00 | C |
| ATOM | 2489 | CD2 | LEU | A | 367 | 19.572 | 31.736 | 34.410 | 1.00 | 29.00 | C |
| ATOM | 2490 | N | SER | A | 368 | 17.382 | 27.495 | 37.494 | 1.00 | 46.84 | N |
| ATOM | 2491 | CA | SER | A | 368 | 16.277 | 26.618 | 37.882 | 1.00 | 46.84 | C |
| ATOM | 2492 | C | SER | A | 368 | 15.114 | 27.301 | 38.600 | 1.00 | 46.84 | C |
| ATOM | 2493 | O | SER | A | 368 | 13.958 | 26.980 | 38.332 | 1.00 | 46.84 | O |
| ATOM | 2494 | CB | SER | A | 368 | 16.785 | 25.463 | 38.746 | 1.00 | 35.11 | C |
| ATOM | 2495 | OG | SER | A | 368 | 17.241 | 25.906 | 40.016 | 1.00 | 35.11 | O |
| ATOM | 2496 | N | SER | A | 369 | 15.400 | 28.237 | 39.503 | 1.00 | 37.04 | N |
| ATOM | 2497 | CA | SER | A | 369 | 14.324 | 28.911 | 40.230 | 1.00 | 37.04 | C |
| ATOM | 2498 | C | SER | A | 369 | 13.305 | 29.542 | 39.273 | 1.00 | 37.04 | C |
| ATOM | 2499 | O | SER | A | 369 | 12.125 | 29.621 | 39.596 | 1.00 | 37.04 | O |
| ATOM | 2500 | CB | SER | A | 369 | 14.895 | 29.980 | 41.174 | 1.00 | 33.53 | C |
| ATOM | 2501 | OG | SER | A | 369 | 14.796 | 31.295 | 40.642 | 1.00 | 33.53 | O |
| ATOM | 2502 | N | ASN | A | 370 | 13.767 | 29.974 | 38.097 | 1.00 | 46.91 | N |
| ATOM | 2503 | CA | ASN | A | 370 | 12.908 | 30.607 | 37.096 | 1.00 | 46.91 | C |
| ATOM | 2504 | C | ASN | A | 370 | 13.632 | 30.703 | 35.740 | 1.00 | 46.91 | C |
| ATOM | 2505 | O | ASN | A | 370 | 14.044 | 31.785 | 35.310 | 1.00 | 46.91 | O |
| ATOM | 2506 | CB | ASN | A | 370 | 12.505 | 32.009 | 37.570 | 1.00 | 40.14 | C |
| ATOM | 2507 | CG | ASN | A | 370 | 11.582 | 32.725 | 36.590 | 1.00 | 40.14 | C |
| ATOM | 2508 | OD1 | ASN | A | 370 | 11.781 | 32.663 | 35.376 | 1.00 | 40.14 | O |
| ATOM | 2509 | ND2 | ASN | A | 370 | 10.577 | 33.425 | 37.115 | 1.00 | 40.14 | N |
| ATOM | 2510 | N | PRO | A | 371 | 13.793 | 29.564 | 35.051 | 1.00 | 42.70 | N |
| ATOM | 2511 | CA | PRO | A | 371 | 14.462 | 29.499 | 33.751 | 1.00 | 42.70 | C |
| ATOM | 2512 | C | PRO | A | 371 | 14.163 | 30.659 | 32.802 | 1.00 | 42.70 | C |
| ATOM | 2513 | O | PRO | A | 371 | 15.077 | 31.288 | 32.262 | 1.00 | 42.70 | O |
| ATOM | 2514 | CB | PRO | A | 371 | 13.984 | 28.167 | 33.207 | 1.00 | 33.77 | C |
| ATOM | 2515 | CG | PRO | A | 371 | 13.967 | 27.313 | 34.449 | 1.00 | 33.77 | C |
| ATOM | 2516 | CD | PRO | A | 371 | 13.304 | 28.232 | 35.458 | 1.00 | 33.77 | C |
| ATOM | 2517 | N | PRO | A | 372 | 12.881 | 30.956 | 32.577 | 1.00 | 37.15 | N |
| ATOM | 2518 | CA | PRO | A | 372 | 12.516 | 32.054 | 31.677 | 1.00 | 37.15 | C |
| ATOM | 2519 | C | PRO | A | 372 | 13.143 | 33.401 | 31.966 | 1.00 | 37.15 | C |
| ATOM | 2520 | O | PRO | A | 372 | 13.033 | 34.326 | 31.152 | 1.00 | 37.15 | O |
| ATOM | 2521 | CB | PRO | A | 372 | 10.987 | 32.097 | 31.758 | 1.00 | 41.47 | C |
| ATOM | 2522 | CG | PRO | A | 372 | 10.644 | 31.271 | 32.973 | 1.00 | 41.47 | C |
| ATOM | 2523 | CD | PRO | A | 372 | 11.691 | 30.201 | 32.987 | 1.00 | 41.47 | C |
| ATOM | 2524 | N | LEU | A | 373 | 13.796 | 33.534 | 33.114 | 1.00 | 34.30 | N |
| ATOM | 2525 | CA | LEU | A | 373 | 14.418 | 34.812 | 33.424 | 1.00 | 34.30 | C |
| ATOM | 2526 | C | LEU | A | 373 | 15.681 | 34.998 | 32.606 | 1.00 | 34.30 | C |
| ATOM | 2527 | O | LEU | A | 373 | 16.243 | 36.092 | 32.542 | 1.00 | 34.30 | O |
| ATOM | 2528 | CB | LEU | A | 373 | 14.722 | 34.930 | 34.915 | 1.00 | 28.60 | C |
| ATOM | 2529 | CG | LEU | A | 373 | 13.548 | 35.427 | 35.774 | 1.00 | 28.60 | C |
| ATOM | 2530 | CD1 | LEU | A | 373 | 14.027 | 35.644 | 37.203 | 0.00 | 28.60 | C |
| ATOM | 2531 | CD2 | LEU | A | 373 | 12.981 | 36.716 | 35.189 | 1.00 | 28.60 | C |
| ATOM | 2532 | N | ALA | A | 374 | 16.108 | 33.922 | 31.961 | 1.00 | 40.00 | N |
| ATOM | 2533 | CA | ALA | A | 374 | 17.301 | 33.953 | 31.130 | 1.00 | 40.00 | C |
| ATOM | 2534 | C | ALA | A | 374 | 17.129 | 34.903 | 29.926 | 1.00 | 40.00 | C |
| ATOM | 2535 | O | ALA | A | 374 | 18.109 | 35.392 | 29.360 | 1.00 | 40.00 | O |
| ATOM | 2536 | CB | ALA | A | 374 | 17.609 | 32.553 | 30.653 | 1.00 | 28.45 | C |
| ATOM | 2537 | N | THR | A | 375 | 15.881 | 35.162 | 29.541 | 1.00 | 40.27 | N |
| ATOM | 2538 | CA | THR | A | 375 | 15.599 | 36.041 | 28.418 | 1.00 | 40.27 | C |

| | | | | | | | | | | | |
|---|---|---|---|---|---|---|---|---|---|---|---|
| ATOM | 2539 | C | THR | A | 375 | 15.987 | 37.467 | 28.775 | 1.00 | 40.27 | C |
| ATOM | 2540 | O | THR | A | 375 | 16.067 | 38.333 | 27.913 | 1.00 | 40.27 | O |
| ATOM | 2541 | CB | THR | A | 375 | 14.102 | 35.991 | 28.016 | 1.00 | 31.59 | C |
| ATOM | 2542 | OG1 | THR | A | 375 | 13.293 | 36.541 | 29.063 | 1.00 | 31.59 | O |
| ATOM | 2543 | CG2 | THR | A | 375 | 13.680 | 34.560 | 27.766 | 1.00 | 31.59 | C |
| ATOM | 2544 | N | ILE | A | 376 | 16.213 | 37.720 | 30.055 | 1.00 | 45.14 | N |
| ATOM | 2545 | CA | ILE | A | 376 | 16.640 | 39.042 | 30.474 | 1.00 | 45.14 | C |
| ATOM | 2546 | C | ILE | A | 376 | 18.079 | 38.886 | 30.942 | 1.00 | 45.14 | C |
| ATOM | 2547 | O | ILE | A | 376 | 18.956 | 39.665 | 30.559 | 1.00 | 45.14 | O |
| ATOM | 2548 | CB | ILE | A | 376 | 15.804 | 39.599 | 31.644 | 1.00 | 36.61 | C |
| ATOM | 2549 | CG1 | ILE | A | 376 | 14.328 | 39.701 | 31.250 | 1.00 | 36.61 | C |
| ATOM | 2550 | CG2 | ILE | A | 376 | 16.334 | 40.972 | 32.027 | 1.00 | 36.61 | C |
| ATOM | 2551 | CD1 | ILE | A | 376 | 13.426 | 40.236 | 32.341 | 0.00 | 36.61 | C |
| ATOM | 2552 | N | LEU | A | 377 | 18.312 | 37.850 | 31.745 | 1.00 | 32.88 | N |
| ATOM | 2553 | CA | LEU | A | 377 | 19.633 | 37.566 | 32.302 | 1.00 | 32.88 | C |
| ATOM | 2554 | C | LEU | A | 377 | 20.760 | 37.410 | 31.292 | 1.00 | 32.88 | C |
| ATOM | 2555 | O | LEU | A | 377 | 21.856 | 37.920 | 31.517 | 1.00 | 32.88 | O |
| ATOM | 2556 | CB | LEU | A | 377 | 19.577 | 36.318 | 33.180 | 1.00 | 40.08 | C |
| ATOM | 2557 | CG | LEU | A | 377 | 18.758 | 36.407 | 34.472 | 1.00 | 40.08 | C |
| ATOM | 2558 | CD1 | LEU | A | 377 | 18.716 | 35.029 | 35.117 | 1.00 | 40.08 | C |
| ATOM | 2559 | CD2 | LEU | A | 377 | 19.373 | 37.446 | 35.415 | 1.00 | 40.08 | C |
| ATOM | 2560 | N | ILE | A | 378 | 20.511 | 36.690 | 30.197 | 1.00 | 43.30 | N |
| ATOM | 2561 | CA | ILE | A | 378 | 21.531 | 36.508 | 29.163 | 1.00 | 43.30 | C |
| ATOM | 2562 | C | ILE | A | 378 | 21.261 | 37.478 | 28.011 | 1.00 | 43.30 | C |
| ATOM | 2563 | O | ILE | A | 378 | 20.361 | 37.274 | 27.199 | 1.00 | 43.30 | O |
| ATOM | 2564 | CB | ILE | A | 378 | 21.555 | 35.061 | 28.639 | 1.00 | 30.57 | C |
| ATOM | 2565 | CG1 | ILE | A | 378 | 21.857 | 34.103 | 29.796 | 1.00 | 30.57 | C |
| ATOM | 2566 | CG2 | ILE | A | 378 | 22.610 | 34.925 | 27.569 | 1.00 | 30.57 | C |
| ATOM | 2567 | CD1 | ILE | A | 378 | 22.140 | 32.662 | 29.388 | 1.00 | 30.57 | C |
| ATOM | 2568 | N | PRO | A | 379 | 22.060 | 38.548 | 27.919 | 1.00 | 39.61 | N |
| ATOM | 2569 | CA | PRO | A | 379 | 21.909 | 39.564 | 26.877 | 1.00 | 39.61 | C |
| ATOM | 2570 | C | PRO | A | 379 | 22.275 | 39.095 | 25.471 | 1.00 | 39.61 | C |
| ATOM | 2571 | O | PRO | A | 379 | 23.170 | 38.270 | 25.281 | 1.00 | 39.61 | O |
| ATOM | 2572 | CB | PRO | A | 379 | 22.805 | 40.683 | 27.377 | 1.00 | 32.34 | C |
| ATOM | 2573 | CG | PRO | A | 379 | 23.952 | 39.922 | 27.948 | 1.00 | 32.34 | C |
| ATOM | 2574 | CD | PRO | A | 379 | 23.292 | 38.776 | 28.696 | 1.00 | 32.34 | C |
| ATOM | 2575 | N | PRO | A | 380 | 21.582 | 39.638 | 24.463 | 1.00 | 34.90 | N |
| ATOM | 2576 | CA | PRO | A | 380 | 21.752 | 39.345 | 23.040 | 1.00 | 34.90 | C |
| ATOM | 2577 | C | PRO | A | 380 | 23.182 | 39.093 | 22.593 | 1.00 | 34.90 | C |
| ATOM | 2578 | O | PRO | A | 380 | 23.491 | 38.000 | 22.121 | 1.00 | 34.90 | O |
| ATOM | 2579 | CB | PRO | A | 380 | 21.151 | 40.570 | 22.369 | 1.00 | 43.65 | C |
| ATOM | 2580 | CG | PRO | A | 380 | 20.005 | 40.891 | 23.271 | 1.00 | 43.65 | C |
| ATOM | 2581 | CD | PRO | A | 380 | 20.600 | 40.722 | 24.658 | 1.00 | 43.65 | C |
| ATOM | 2582 | N | HIS | A | 381 | 24.058 | 40.089 | 22.730 | 1.00 | 31.94 | N |
| ATOM | 2583 | CA | HIS | A | 381 | 25.432 | 39.891 | 22.286 | 1.00 | 31.94 | C |
| ATOM | 2584 | C | HIS | A | 381 | 26.027 | 38.613 | 22.864 | 1.00 | 31.94 | C |
| ATOM | 2585 | O | HIS | A | 381 | 26.861 | 37.967 | 22.230 | 1.00 | 31.94 | O |
| ATOM | 2586 | CB | HIS | A | 381 | 26.314 | 41.117 | 22.589 | 1.00 | 42.72 | C |
| ATOM | 2587 | CG | HIS | A | 381 | 26.465 | 41.446 | 24.040 | 1.00 | 42.72 | C |
| ATOM | 2588 | ND1 | HIS | A | 381 | 27.485 | 40.925 | 24.825 | 1.00 | 42.72 | N |
| ATOM | 2589 | CD2 | HIS | A | 381 | 25.798 | 42.311 | 24.838 | 1.00 | 42.72 | C |
| ATOM | 2590 | CE1 | HIS | A | 381 | 27.435 | 41.464 | 26.024 | 1.00 | 42.72 | C |
| ATOM | 2591 | NE2 | HIS | A | 381 | 26.418 | 42.316 | 26.065 | 1.00 | 42.72 | N |
| ATOM | 2592 | N | ALA | A | 382 | 25.564 | 38.221 | 24.046 | 1.00 | 47.93 | N |
| ATOM | 2593 | CA | ALA | A | 382 | 26.056 | 37.001 | 24.679 | 1.00 | 47.93 | C |
| ATOM | 2594 | C | ALA | A | 382 | 25.575 | 35.746 | 23.931 | 1.00 | 47.93 | C |
| ATOM | 2595 | O | ALA | A | 382 | 26.067 | 34.634 | 24.173 | 1.00 | 47.93 | O |
| ATOM | 2596 | CB | ALA | A | 382 | 25.605 | 36.950 | 26.126 | 1.00 | 51.96 | C |
| ATOM | 2597 | N | ARG | A | 383 | 24.630 | 35.932 | 23.012 | 1.00 | 65.05 | N |
| ATOM | 2598 | CA | ARG | A | 383 | 24.079 | 34.828 | 22.235 | 1.00 | 65.05 | C |
| ATOM | 2599 | C | ARG | A | 383 | 24.639 | 34.795 | 20.810 | 1.00 | 65.05 | C |
| ATOM | 2600 | O | ARG | A | 383 | 23.971 | 34.342 | 19.872 | 1.00 | 65.05 | O |

| | | | | | | | | | | | |
|---|---|---|---|---|---|---|---|---|---|---|---|
| ATOM | 2601 | CB | ARG | A | 383 | 22.543 | 34.932 | 22.202 | 1.00 | 55.86 | C |
| ATOM | 2602 | CG | ARG | A | 383 | 21.860 | 34.470 | 23.487 | 1.00 | 55.86 | C |
| ATOM | 2603 | CD | ARG | A | 383 | 20.930 | 35.515 | 24.090 | 1.00 | 55.86 | C |
| ATOM | 2604 | NE | ARG | A | 383 | 19.519 | 35.334 | 23.742 | 1.00 | 55.86 | N |
| ATOM | 2605 | CZ | ARG | A | 383 | 18.523 | 35.994 | 24.336 | 1.00 | 55.86 | C |
| ATOM | 2606 | NH1 | ARG | A | 383 | 18.779 | 36.871 | 25.302 | 1.00 | 55.86 | N |
| ATOM | 2607 | NH2 | ARG | A | 383 | 17.266 | 35.784 | 23.967 | 1.00 | 55.86 | N |
| ATOM | 2608 | N | ILE | A | 384 | 25.872 | 35.262 | 20.654 | 1.00 | 66.55 | N |
| ATOM | 2609 | CA | ILE | A | 384 | 26.513 | 35.294 | 19.346 | 1.00 | 66.55 | C |
| ATOM | 2610 | C | ILE | A | 384 | 27.751 | 34.391 | 19.360 | 1.00 | 66.55 | C |
| ATOM | 2611 | O | ILE | A | 384 | 28.407 | 34.334 | 20.435 | 1.00 | 66.55 | O |
| ATOM | 2612 | CB | ILE | A | 384 | 26.918 | 36.742 | 19.009 | 1.00 | 45.60 | C |
| ATOM | 2613 | CG1 | ILE | A | 384 | 25.666 | 37.629 | 19.027 | 1.00 | 45.60 | C |
| ATOM | 2614 | CG2 | ILE | A | 384 | 27.619 | 36.793 | 17.670 | 1.00 | 45.60 | C |
| ATOM | 2615 | CD1 | ILE | A | 384 | 25.953 | 39.127 | 19.005 | 1.00 | 45.60 | C |
| TER | 2616 | | ILE | A | 384 | | | | | | |
| ATOM | 2617 | N | VAL | B | 37 | 14.552 | 82.083 | 77.018 | 1.00 | 56.12 | N |
| ATOM | 2618 | CA | VAL | B | 37 | 13.942 | 81.747 | 78.345 | 1.00 | 56.12 | C |
| ATOM | 2619 | C | VAL | B | 37 | 12.523 | 81.177 | 78.206 | 1.00 | 56.12 | C |
| ATOM | 2620 | O | VAL | B | 37 | 11.555 | 81.926 | 78.150 | 1.00 | 56.12 | O |
| ATOM | 2621 | CB | VAL | B | 37 | 13.875 | 82.990 | 79.253 | 1.00 | 52.00 | C |
| ATOM | 2622 | CG1 | VAL | B | 37 | 13.231 | 82.624 | 80.581 | 1.00 | 52.00 | C |
| ATOM | 2623 | CG2 | VAL | B | 37 | 15.271 | 83.548 | 79.479 | 0.00 | 52.00 | C |
| ATOM | 2624 | N | THR | B | 38 | 12.408 | 79.851 | 78.170 | 1.00 | 50.61 | N |
| ATOM | 2625 | CA | THR | B | 38 | 11.114 | 79.171 | 78.020 | 1.00 | 50.61 | C |
| ATOM | 2626 | C | THR | B | 38 | 10.316 | 78.942 | 79.309 | 1.00 | 50.61 | C |
| ATOM | 2627 | O | THR | B | 38 | 10.876 | 78.558 | 80.346 | 1.00 | 50.61 | O |
| ATOM | 2628 | CB | THR | B | 38 | 11.308 | 77.796 | 77.351 | 1.00 | 36.91 | C |
| ATOM | 2629 | OG1 | THR | B | 38 | 11.830 | 77.984 | 76.031 | 1.00 | 36.91 | O |
| ATOM | 2630 | CG2 | THR | B | 38 | 9.993 | 77.039 | 77.270 | 1.00 | 36.91 | C |
| ATOM | 2631 | N | THR | B | 39 | 9.005 | 79.170 | 79.233 | 1.00 | 35.81 | N |
| ATOM | 2632 | CA | THR | B | 39 | 8.109 | 78.962 | 80.375 | 1.00 | 35.81 | C |
| ATOM | 2633 | C | THR | B | 39 | 7.008 | 77.971 | 80.011 | 1.00 | 35.81 | C |
| ATOM | 2634 | O | THR | B | 39 | 6.230 | 78.201 | 79.085 | 1.00 | 35.81 | O |
| ATOM | 2635 | CB | THR | B | 39 | 7.439 | 80.276 | 80.841 | 1.00 | 39.66 | C |
| ATOM | 2636 | OG1 | THR | B | 39 | 8.434 | 81.167 | 81.359 | 1.00 | 39.66 | O |
| ATOM | 2637 | CG2 | THR | B | 39 | 6.414 | 79.998 | 81.935 | 1.00 | 39.66 | C |
| ATOM | 2638 | N | VAL | B | 40 | 6.947 | 76.865 | 80.740 | 1.00 | 41.05 | N |
| ATOM | 2639 | CA | VAL | B | 40 | 5.938 | 75.852 | 80.474 | 1.00 | 41.05 | C |
| ATOM | 2640 | C | VAL | B | 40 | 5.248 | 75.539 | 81.794 | 1.00 | 41.05 | C |
| ATOM | 2641 | O | VAL | B | 40 | 5.676 | 76.006 | 82.851 | 1.00 | 41.05 | O |
| ATOM | 2642 | CB | VAL | B | 40 | 6.588 | 74.551 | 79.913 | 1.00 | 19.78 | C |
| ATOM | 2643 | CG1 | VAL | B | 40 | 7.415 | 73.880 | 80.985 | 1.00 | 19.78 | C |
| ATOM | 2644 | CG2 | VAL | B | 40 | 5.516 | 73.604 | 79.399 | 0.00 | 19.78 | C |
| ATOM | 2645 | N | VAL | B | 41 | 4.168 | 74.773 | 81.740 | 1.00 | 31.97 | N |
| ATOM | 2646 | CA | VAL | B | 41 | 3.477 | 74.387 | 82.957 | 1.00 | 31.97 | C |
| ATOM | 2647 | C | VAL | B | 41 | 3.739 | 72.883 | 83.078 | 1.00 | 31.97 | C |
| ATOM | 2648 | O | VAL | B | 41 | 3.239 | 72.082 | 82.291 | 1.00 | 31.97 | O |
| ATOM | 2649 | CB | VAL | B | 41 | 1.966 | 74.715 | 82.863 | 1.00 | 24.67 | C |
| ATOM | 2650 | CG1 | VAL | B | 41 | 1.356 | 74.043 | 81.647 | 0.00 | 24.67 | C |
| ATOM | 2651 | CG2 | VAL | B | 41 | 1.265 | 74.293 | 84.138 | 1.00 | 24.67 | C |
| ATOM | 2652 | N | ALA | B | 42 | 4.549 | 72.496 | 84.052 | 1.00 | 33.95 | N |
| ATOM | 2653 | CA | ALA | B | 42 | 4.891 | 71.092 | 84.191 | 1.00 | 33.95 | C |
| ATOM | 2654 | C | ALA | B | 42 | 4.369 | 70.395 | 85.434 | 1.00 | 33.95 | C |
| ATOM | 2655 | O | ALA | B | 42 | 4.246 | 70.990 | 86.501 | 1.00 | 33.95 | O |
| ATOM | 2656 | CB | ALA | B | 42 | 6.410 | 70.927 | 84.101 | 1.00 | 51.36 | C |
| ATOM | 2657 | N | THR | B | 43 | 4.083 | 69.109 | 85.279 | 1.00 | 37.68 | N |
| ATOM | 2658 | CA | THR | B | 43 | 3.576 | 68.290 | 86.364 | 1.00 | 37.68 | C |
| ATOM | 2659 | C | THR | B | 43 | 4.746 | 67.575 | 87.025 | 1.00 | 37.68 | C |
| ATOM | 2660 | O | THR | B | 43 | 5.542 | 66.921 | 86.356 | 1.00 | 37.68 | O |
| ATOM | 2661 | CB | THR | B | 43 | 2.596 | 67.238 | 85.832 | 1.00 | 34.53 | C |
| ATOM | 2662 | OG1 | THR | B | 43 | 1.518 | 67.892 | 85.161 | 1.00 | 34.53 | O |

| | | | | | | | | | | | |
|---|---|---|---|---|---|---|---|---|---|---|---|
| ATOM | 2663 | CG2 | THR | B | 43 | 2.041 | 66.397 | 86.965 | 1.00 | 34.53 | C |
| ATOM | 2664 | N | PRO | B | 44 | 4.877 | 67.709 | 88.348 | 1.00 | 34.56 | N |
| ATOM | 2665 | CA | PRO | B | 44 | 5.963 | 67.062 | 89.092 | 1.00 | 34.56 | C |
| ATOM | 2666 | C | PRO | B | 44 | 5.900 | 65.529 | 89.007 | 1.00 | 34.56 | C |
| ATOM | 2667 | O | PRO | B | 44 | 4.817 | 64.937 | 89.023 | 1.00 | 34.56 | O |
| ATOM | 2668 | CB | PRO | B | 44 | 5.752 | 67.575 | 90.514 | 1.00 | 26.17 | C |
| ATOM | 2669 | CG | PRO | B | 44 | 5.167 | 68.935 | 90.285 | 1.00 | 26.17 | C |
| ATOM | 2670 | CD | PRO | B | 44 | 4.165 | 68.684 | 89.193 | 1.00 | 26.17 | C |
| ATOM | 2671 | N | GLY | B | 45 | 7.064 | 64.893 | 88.923 | 1.00 | 35.52 | N |
| ATOM | 2672 | CA | GLY | B | 45 | 7.116 | 63.445 | 88.828 | 1.00 | 35.52 | C |
| ATOM | 2673 | C | GLY | B | 45 | 6.416 | 62.752 | 89.975 | 1.00 | 35.52 | C |
| ATOM | 2674 | O | GLY | B | 45 | 5.636 | 61.825 | 89.756 | 1.00 | 35.52 | O |
| ATOM | 2675 | N | GLN | B | 46 | 6.696 | 63.211 | 91.195 | 1.00 | 53.35 | N |
| ATOM | 2676 | CA | GLN | B | 46 | 6.111 | 62.650 | 92.412 | 1.00 | 53.35 | C |
| ATOM | 2677 | C | GLN | B | 46 | 4.783 | 63.330 | 92.734 | 1.00 | 53.35 | C |
| ATOM | 2678 | O | GLN | B | 46 | 4.261 | 64.109 | 91.920 | 1.00 | 53.35 | O |
| ATOM | 2679 | CB | GLN | B | 46 | 7.081 | 62.815 | 93.577 | 0.00 | 35.69 | C |
| ATOM | 2680 | N | GLY | B | 47 | 4.245 | 63.027 | 93.915 | 1.00 | 56.57 | N |
| ATOM | 2681 | CA | GLY | B | 47 | 2.983 | 63.600 | 94.355 | 0.00 | 56.57 | C |
| ATOM | 2682 | C | GLY | B | 47 | 1.859 | 63.454 | 93.348 | 1.00 | 56.57 | C |
| ATOM | 2683 | O | GLY | B | 47 | 2.074 | 62.931 | 92.252 | 1.00 | 56.57 | O |
| ATOM | 2684 | N | PRO | B | 48 | 0.633 | 63.894 | 93.686 | 1.00 | 57.40 | N |
| ATOM | 2685 | CA | PRO | B | 48 | -0.478 | 63.775 | 92.731 | 1.00 | 57.40 | C |
| ATOM | 2686 | C | PRO | B | 48 | -0.367 | 64.882 | 91.676 | 1.00 | 57.40 | C |
| ATOM | 2687 | O | PRO | B | 48 | 0.295 | 65.891 | 91.914 | 1.00 | 57.40 | O |
| ATOM | 2688 | CB | PRO | B | 48 | -1.701 | 63.932 | 93.621 | 1.00 | 31.51 | C |
| ATOM | 2689 | CG | PRO | B | 48 | -1.226 | 64.924 | 94.638 | 0.00 | 31.51 | C |
| ATOM | 2690 | CD | PRO | B | 48 | 0.160 | 64.413 | 94.981 | 0.00 | 31.51 | C |
| ATOM | 2691 | N | ASP | B | 49 | -1.008 | 64.708 | 90.523 | 1.00 | 47.39 | N |
| ATOM | 2692 | CA | ASP | B | 49 | -0.925 | 65.724 | 89.476 | 1.00 | 47.39 | C |
| ATOM | 2693 | C | ASP | B | 49 | -1.165 | 67.154 | 89.974 | 1.00 | 47.39 | C |
| ATOM | 2694 | O | ASP | B | 49 | -2.290 | 67.652 | 89.945 | 1.00 | 47.39 | O |
| ATOM | 2695 | CB | ASP | B | 49 | -1.892 | 65.410 | 88.324 | 1.00 | 55.67 | C |
| ATOM | 2696 | CG | ASP | B | 49 | -1.395 | 64.281 | 87.433 | 1.00 | 55.67 | C |
| ATOM | 2697 | OD1 | ASP | B | 49 | -1.916 | 64.117 | 86.304 | 1.00 | 55.67 | O |
| ATOM | 2698 | OD2 | ASP | B | 49 | -0.485 | 63.543 | 87.870 | 1.00 | 55.67 | O |
| ATOM | 2699 | N | ARG | B | 50 | -0.083 | 67.814 | 90.392 | 1.00 | 45.98 | N |
| ATOM | 2700 | CA | ARG | B | 50 | -0.121 | 69.183 | 90.922 | 1.00 | 45.98 | C |
| ATOM | 2701 | C | ARG | B | 50 | 0.768 | 70.097 | 90.059 | 1.00 | 45.98 | C |
| ATOM | 2702 | O | ARG | B | 50 | 1.798 | 70.583 | 90.527 | 1.00 | 45.98 | O |
| ATOM | 2703 | CB | ARG | B | 50 | 0.421 | 69.164 | 92.351 | 1.00 | 70.01 | C |
| ATOM | 2704 | CG | ARG | B | 50 | -0.140 | 70.225 | 93.277 | 1.00 | 70.01 | C |
| ATOM | 2705 | CD | ARG | B | 50 | -1.448 | 69.768 | 93.921 | 1.00 | 70.01 | C |
| ATOM | 2706 | NE | ARG | B | 50 | -2.530 | 69.633 | 92.944 | 1.00 | 70.01 | N |
| ATOM | 2707 | CZ | ARG | B | 50 | -3.815 | 69.491 | 93.275 | 1.00 | 70.01 | C |
| ATOM | 2708 | NH1 | ARG | B | 50 | -4.166 | 69.460 | 94.553 | 0.00 | 70.01 | N |
| ATOM | 2709 | NH2 | ARG | B | 50 | -4.754 | 69.394 | 92.332 | 1.00 | 70.01 | N |
| ATOM | 2710 | N | PRO | B | 51 | 0.381 | 70.337 | 88.793 | 1.00 | 33.39 | N |
| ATOM | 2711 | CA | PRO | B | 51 | 1.114 | 71.179 | 87.835 | 1.00 | 33.39 | C |
| ATOM | 2712 | C | PRO | B | 51 | 1.512 | 72.548 | 88.361 | 1.00 | 33.39 | C |
| ATOM | 2713 | O | PRO | B | 51 | 0.789 | 73.146 | 89.137 | 1.00 | 33.39 | O |
| ATOM | 2714 | CB | PRO | B | 51 | 0.141 | 71.302 | 86.668 | 1.00 | 25.10 | C |
| ATOM | 2715 | CG | PRO | B | 51 | -0.589 | 70.024 | 86.704 | 1.00 | 25.10 | C |
| ATOM | 2716 | CD | PRO | B | 51 | -0.849 | 69.812 | 88.180 | 1.00 | 25.10 | C |
| ATOM | 2717 | N | GLN | B | 52 | 2.661 | 73.051 | 87.933 | 1.00 | 31.52 | N |
| ATOM | 2718 | CA | GLN | B | 52 | 3.112 | 74.372 | 88.357 | 1.00 | 31.52 | C |
| ATOM | 2719 | C | GLN | B | 52 | 3.814 | 75.029 | 87.193 | 1.00 | 31.52 | C |
| ATOM | 2720 | O | GLN | B | 52 | 4.183 | 74.362 | 86.232 | 1.00 | 31.52 | O |
| ATOM | 2721 | CB | GLN | B | 52 | 4.053 | 74.283 | 89.571 | 1.00 | 48.92 | C |
| ATOM | 2722 | CG | GLN | B | 52 | 5.087 | 73.175 | 89.484 | 1.00 | 48.92 | C |
| ATOM | 2723 | CD | GLN | B | 52 | 5.887 | 72.998 | 90.774 | 1.00 | 48.92 | C |
| ATOM | 2724 | OE1 | GLN | B | 52 | 6.846 | 73.734 | 91.037 | 1.00 | 48.92 | O |

| | | | | | | | | | | | |
|---|---|---|---|---|---|---|---|---|---|---|---|
| ATOM | 2725 | NE2 | GLN | B | 52 | 5.489 | 72.024 | 91.585 | 0.00 | 48.92 | N |
| ATOM | 2726 | N | GLU | B | 53 | 3.966 | 76.344 | 87.247 | 1.00 | 38.95 | N |
| ATOM | 2727 | CA | GLU | B | 53 | 4.650 | 77.036 | 86.166 | 1.00 | 38.95 | C |
| ATOM | 2728 | C | GLU | B | 53 | 6.156 | 76.838 | 86.382 | 1.00 | 38.95 | C |
| ATOM | 2729 | O | GLU | B | 53 | 6.670 | 76.993 | 87.497 | 1.00 | 38.95 | O |
| ATOM | 2730 | CB | GLU | B | 53 | 4.252 | 78.528 | 86.146 | 1.00 | 73.86 | C |
| ATOM | 2731 | CG | GLU | B | 53 | 2.766 | 78.743 | 85.768 | 1.00 | 73.86 | C |
| ATOM | 2732 | CD | GLU | B | 53 | 2.200 | 80.131 | 86.133 | 1.00 | 73.86 | C |
| ATOM | 2733 | OE1 | GLU | B | 53 | 2.446 | 80.609 | 87.275 | 1.00 | 73.86 | O |
| ATOM | 2734 | OE2 | GLU | B | 53 | 1.490 | 80.730 | 85.280 | 1.00 | 73.86 | O |
| ATOM | 2735 | N | VAL | B | 54 | 6.838 | 76.446 | 85.306 | 1.00 | 31.78 | N |
| ATOM | 2736 | CA | VAL | B | 54 | 8.270 | 76.185 | 85.330 | 1.00 | 31.78 | C |
| ATOM | 2737 | C | VAL | B | 54 | 8.977 | 76.902 | 84.175 | 1.00 | 31.78 | C |
| ATOM | 2738 | O | VAL | B | 54 | 8.542 | 76.824 | 83.021 | 1.00 | 31.78 | O |
| ATOM | 2739 | CB | VAL | B | 54 | 8.546 | 74.665 | 85.226 | 1.00 | 20.11 | C |
| ATOM | 2740 | CG1 | VAL | B | 54 | 10.032 | 74.391 | 85.393 | 1.00 | 20.11 | C |
| ATOM | 2741 | CG2 | VAL | B | 54 | 7.760 | 73.917 | 86.279 | 1.00 | 20.11 | C |
| ATOM | 2742 | N | SER | B | 55 | 10.068 | 77.600 | 84.494 | 1.00 | 37.42 | N |
| ATOM | 2743 | CA | SER | B | 55 | 10.845 | 78.333 | 83.488 | 1.00 | 37.42 | C |
| ATOM | 2744 | C | SER | B | 55 | 12.308 | 77.887 | 83.422 | 1.00 | 37.42 | C |
| ATOM | 2745 | O | SER | B | 55 | 12.982 | 77.753 | 84.446 | 1.00 | 37.42 | O |
| ATOM | 2746 | CB | SER | B | 55 | 10.780 | 79.837 | 83.771 | 1.00 | 49.54 | C |
| ATOM | 2747 | OG | SER | B | 55 | 9.465 | 80.335 | 83.562 | 1.00 | 49.54 | O |
| ATOM | 2748 | N | TYR | B | 56 | 12.793 | 77.661 | 82.207 | 1.00 | 44.46 | N |
| ATOM | 2749 | CA | TYR | B | 56 | 14.173 | 77.231 | 82.003 | 1.00 | 44.46 | C |
| ATOM | 2750 | C | TYR | B | 56 | 14.799 | 77.949 | 80.806 | 1.00 | 44.46 | C |
| ATOM | 2751 | O | TYR | B | 56 | 14.088 | 78.480 | 79.928 | 1.00 | 44.46 | O |
| ATOM | 2752 | CB | TYR | B | 56 | 14.229 | 75.718 | 81.775 | 1.00 | 34.06 | C |
| ATOM | 2753 | CG | TYR | B | 56 | 13.523 | 75.218 | 80.520 | 1.00 | 34.06 | C |
| ATOM | 2754 | CD1 | TYR | B | 56 | 14.085 | 75.390 | 79.248 | 1.00 | 34.06 | C |
| ATOM | 2755 | CD2 | TYR | B | 56 | 12.305 | 74.532 | 80.607 | 1.00 | 34.06 | C |
| ATOM | 2756 | CE1 | TYR | B | 56 | 13.453 | 74.881 | 78.093 | 1.00 | 34.06 | C |
| ATOM | 2757 | CE2 | TYR | B | 56 | 11.668 | 74.022 | 79.457 | 1.00 | 34.06 | C |
| ATOM | 2758 | CZ | TYR | B | 56 | 12.250 | 74.203 | 78.210 | 1.00 | 34.06 | C |
| ATOM | 2759 | OH | TYR | B | 56 | 11.618 | 73.706 | 77.093 | 1.00 | 34.06 | O |
| ATOM | 2760 | N | THR | B | 57 | 16.131 | 77.958 | 80.757 | 1.00 | 63.55 | N |
| ATOM | 2761 | CA | THR | B | 57 | 16.817 | 78.613 | 79.651 | 1.00 | 63.55 | C |
| ATOM | 2762 | C | THR | B | 57 | 18.191 | 77.993 | 79.370 | 1.00 | 63.55 | C |
| ATOM | 2763 | O | THR | B | 57 | 18.516 | 76.924 | 79.908 | 1.00 | 63.55 | O |
| ATOM | 2764 | CB | THR | B | 57 | 16.951 | 80.123 | 79.932 | 1.00 | 44.35 | C |
| ATOM | 2765 | OG1 | THR | B | 57 | 17.145 | 80.830 | 78.696 | 1.00 | 44.35 | O |
| ATOM | 2766 | CG2 | THR | B | 57 | 18.103 | 80.376 | 80.907 | 1.00 | 44.35 | C |
| ATOM | 2767 | N | ASP | B | 58 | 18.987 | 78.665 | 78.532 | 1.00 | 61.81 | N |
| ATOM | 2768 | CA | ASP | B | 58 | 20.319 | 78.181 | 78.148 | 1.00 | 61.81 | C |
| ATOM | 2769 | C | ASP | B | 58 | 20.138 | 76.761 | 77.639 | 1.00 | 61.81 | C |
| ATOM | 2770 | O | ASP | B | 58 | 20.860 | 75.844 | 78.030 | 1.00 | 61.81 | O |
| ATOM | 2771 | CB | ASP | B | 58 | 21.278 | 78.189 | 79.350 | 1.00 | 60.47 | C |
| ATOM | 2772 | CG | ASP | B | 58 | 21.455 | 79.579 | 79.950 | 1.00 | 60.47 | C |
| ATOM | 2773 | OD1 | ASP | B | 58 | 21.159 | 80.580 | 79.253 | 1.00 | 60.47 | O |
| ATOM | 2774 | OD2 | ASP | B | 58 | 21.901 | 79.677 | 81.115 | 1.00 | 60.47 | O |
| ATOM | 2775 | N | THR | B | 59 | 19.171 | 76.602 | 76.747 | 1.00 | 46.38 | N |
| ATOM | 2776 | CA | THR | B | 59 | 18.807 | 75.302 | 76.203 | 1.00 | 46.38 | C |
| ATOM | 2777 | C | THR | B | 59 | 19.540 | 74.828 | 74.959 | 1.00 | 46.38 | C |
| ATOM | 2778 | O | THR | B | 59 | 19.313 | 75.356 | 73.861 | 1.00 | 46.38 | O |
| ATOM | 2779 | CB | THR | B | 59 | 17.294 | 75.288 | 75.918 | 1.00 | 44.29 | C |
| ATOM | 2780 | OG1 | THR | B | 59 | 16.596 | 75.512 | 77.152 | 1.00 | 44.29 | O |
| ATOM | 2781 | CG2 | THR | B | 59 | 16.857 | 73.972 | 75.285 | 1.00 | 44.29 | C |
| ATOM | 2782 | N | LYS | B | 60 | 20.390 | 73.813 | 75.135 | 1.00 | 56.43 | N |
| ATOM | 2783 | CA | LYS | B | 60 | 21.138 | 73.257 | 74.013 | 1.00 | 56.43 | C |
| ATOM | 2784 | C | LYS | B | 60 | 20.959 | 71.741 | 73.912 | 1.00 | 56.43 | C |
| ATOM | 2785 | O | LYS | B | 60 | 20.663 | 71.050 | 74.879 | 1.00 | 56.43 | O |
| ATOM | 2786 | CB | LYS | B | 60 | 22.617 | 73.590 | 74.215 | 1.00 | 46.68 | C |

| | | | | | | | | | | | |
|---|---|---|---|---|---|---|---|---|---|---|---|
| ATOM | 2787 | CG | LYS | B | 60 | 23.273 | 72.691 | 75.265 | 1.00 | 46.68 | C |
| ATOM | 2788 | CD | LYS | B | 60 | 24.747 | 73.037 | 75.489 | 0.00 | 46.68 | C |
| ATOM | 2789 | CE | LYS | B | 60 | 24.937 | 74.410 | 76.140 | 0.00 | 46.68 | C |
| ATOM | 2790 | NZ | LYS | B | 60 | 24.177 | 74.474 | 77.388 | 0.00 | 46.68 | N |
| ATOM | 2791 | N | VAL | B | 61 | 21.111 | 71.232 | 72.675 | 1.00 | 52.10 | N |
| ATOM | 2792 | CA | VAL | B | 61 | 20.994 | 69.794 | 72.467 | 1.00 | 52.10 | C |
| ATOM | 2793 | C | VAL | B | 61 | 22.231 | 69.053 | 72.981 | 1.00 | 52.10 | C |
| ATOM | 2794 | O | VAL | B | 61 | 23.360 | 69.511 | 72.869 | 1.00 | 52.10 | O |
| ATOM | 2795 | CB | VAL | B | 61 | 20.825 | 69.545 | 70.968 | 1.00 | 33.83 | C |
| ATOM | 2796 | CG1 | VAL | B | 61 | 20.703 | 68.047 | 70.698 | 1.00 | 33.83 | C |
| ATOM | 2797 | CG2 | VAL | B | 61 | 19.579 | 70.248 | 70.463 | 1.00 | 33.83 | C |
| ATOM | 2798 | N | ILE | B | 62 | 21.980 | 67.883 | 73.599 | 1.00 | 46.31 | N |
| ATOM | 2799 | CA | ILE | B | 62 | 23.088 | 67.106 | 74.140 | 1.00 | 46.31 | C |
| ATOM | 2800 | C | ILE | B | 62 | 23.037 | 65.646 | 73.683 | 1.00 | 46.31 | C |
| ATOM | 2801 | O | ILE | B | 62 | 23.389 | 64.722 | 74.404 | 1.00 | 46.31 | O |
| ATOM | 2802 | CB | ILE | B | 62 | 23.025 | 67.181 | 75.667 | 1.00 | 46.04 | C |
| ATOM | 2803 | CG1 | ILE | B | 62 | 21.730 | 66.542 | 76.177 | 1.00 | 46.04 | C |
| ATOM | 2804 | CG2 | ILE | B | 62 | 23.032 | 68.653 | 76.116 | 1.00 | 46.04 | C |
| ATOM | 2805 | CD1 | ILE | B | 62 | 21.747 | 66.327 | 77.692 | 1.00 | 46.04 | C |
| ATOM | 2806 | N | GLY | B | 63 | 22.538 | 65.450 | 72.448 | 1.00 | 47.10 | N |
| ATOM | 2807 | CA | GLY | B | 63 | 22.471 | 64.097 | 71.909 | 1.00 | 47.10 | C |
| ATOM | 2808 | C | GLY | B | 63 | 21.111 | 63.808 | 71.270 | 1.00 | 47.10 | C |
| ATOM | 2809 | O | GLY | B | 63 | 20.113 | 64.471 | 71.523 | 1.00 | 47.10 | O |
| ATOM | 2810 | N | ASN | B | 64 | 21.106 | 62.800 | 70.378 | 1.00 | 62.66 | N |
| ATOM | 2811 | CA | ASN | B | 64 | 19.862 | 62.434 | 69.712 | 1.00 | 62.66 | C |
| ATOM | 2812 | C | ASN | B | 64 | 19.632 | 60.922 | 69.742 | 1.00 | 62.66 | C |
| ATOM | 2813 | O | ASN | B | 64 | 20.358 | 60.137 | 69.146 | 1.00 | 62.66 | O |
| ATOM | 2814 | CB | ASN | B | 64 | 19.935 | 62.920 | 68.264 | 1.00 | 87.20 | C |
| ATOM | 2815 | CG | ASN | B | 64 | 19.849 | 64.424 | 68.235 | 1.00 | 87.20 | C |
| ATOM | 2816 | OD1 | ASN | B | 64 | 18.885 | 65.010 | 67.752 | 1.00 | 87.20 | O |
| ATOM | 2817 | ND2 | ASN | B | 64 | 20.900 | 65.061 | 68.779 | 1.00 | 87.20 | N |
| ATOM | 2818 | N | GLY | B | 65 | 18.600 | 60.517 | 70.504 | 1.00 | 91.55 | N |
| ATOM | 2819 | CA | GLY | B | 65 | 18.291 | 59.096 | 70.600 | 1.00 | 91.55 | C |
| ATOM | 2820 | C | GLY | B | 65 | 17.528 | 58.602 | 69.368 | 1.00 | 91.55 | C |
| ATOM | 2821 | O | GLY | B | 65 | 17.514 | 59.224 | 68.314 | 0.00 | 91.55 | O |
| ATOM | 2822 | N | SER | B | 66 | 16.909 | 57.416 | 69.520 | 1.00 | 76.40 | N |
| ATOM | 2823 | CA | SER | B | 66 | 16.146 | 56.855 | 68.412 | 1.00 | 76.40 | C |
| ATOM | 2824 | C | SER | B | 66 | 14.673 | 57.262 | 68.483 | 1.00 | 76.40 | C |
| ATOM | 2825 | O | SER | B | 66 | 13.973 | 57.362 | 67.484 | 1.00 | 76.40 | O |
| ATOM | 2826 | CB | SER | B | 66 | 16.270 | 55.332 | 68.471 | 1.00 | 42.15 | C |
| ATOM | 2827 | OG | SER | B | 66 | 17.555 | 54.984 | 68.986 | 1.00 | 42.15 | O |
| ATOM | 2828 | N | PHE | B | 67 | 14.197 | 57.460 | 69.726 | 1.00 | 43.17 | N |
| ATOM | 2829 | CA | PHE | B | 67 | 12.803 | 57.847 | 69.910 | 1.00 | 43.17 | C |
| ATOM | 2830 | C | PHE | B | 67 | 12.625 | 59.365 | 69.834 | 1.00 | 43.17 | C |
| ATOM | 2831 | O | PHE | B | 67 | 11.657 | 59.886 | 69.296 | 1.00 | 43.17 | O |
| ATOM | 2832 | CB | PHE | B | 67 | 12.343 | 57.337 | 71.276 | 1.00 | 20.00 | C |
| ATOM | 2833 | CG | PHE | B | 67 | 12.326 | 55.837 | 71.278 | 1.00 | 20.00 | C |
| ATOM | 2834 | CD1 | PHE | B | 67 | 13.432 | 55.138 | 71.745 | 1.00 | 20.00 | C |
| ATOM | 2835 | CD2 | PHE | B | 67 | 11.214 | 55.156 | 70.809 | 1.00 | 20.00 | C |
| ATOM | 2836 | CE1 | PHE | B | 67 | 13.421 | 53.750 | 71.742 | 1.00 | 20.00 | C |
| ATOM | 2837 | CE2 | PHE | B | 67 | 11.211 | 53.764 | 70.810 | 1.00 | 20.00 | C |
| ATOM | 2838 | CZ | PHE | B | 67 | 12.312 | 53.057 | 71.276 | 1.00 | 20.00 | C |
| ATOM | 2839 | N | GLY | B | 68 | 13.590 | 60.081 | 70.440 | 1.00 | 50.61 | N |
| ATOM | 2840 | CA | GLY | B | 68 | 13.525 | 61.537 | 70.425 | 1.00 | 50.61 | C |
| ATOM | 2841 | C | GLY | B | 68 | 14.874 | 62.162 | 70.786 | 1.00 | 50.61 | C |
| ATOM | 2842 | O | GLY | B | 68 | 15.882 | 61.489 | 70.958 | 1.00 | 50.61 | O |
| ATOM | 2843 | N | VAL | B | 69 | 14.880 | 63.506 | 70.857 | 1.00 | 42.75 | N |
| ATOM | 2844 | CA | VAL | B | 69 | 16.116 | 64.201 | 71.197 | 1.00 | 42.75 | C |
| ATOM | 2845 | C | VAL | B | 69 | 16.193 | 64.516 | 72.692 | 1.00 | 42.75 | C |
| ATOM | 2846 | O | VAL | B | 69 | 15.211 | 64.473 | 73.422 | 1.00 | 42.75 | O |
| ATOM | 2847 | CB | VAL | B | 69 | 16.174 | 65.497 | 70.388 | 1.00 | 36.53 | C |
| ATOM | 2848 | CG1 | VAL | B | 69 | 17.277 | 66.402 | 70.934 | 1.00 | 36.53 | C |

| | | | | | | | | | | | |
|---|---|---|---|---|---|---|---|---|---|---|---|
| ATOM | 2849 | CG2 | VAL | B | 69 | 16.458 | 65.187 | 68.930 | 0.00 | 36.53 | C |
| ATOM | 2850 | N | VAL | B | 70 | 17.426 | 64.800 | 73.150 | 1.00 | 37.08 | N |
| ATOM | 2851 | CA | VAL | B | 70 | 17.615 | 65.114 | 74.560 | 1.00 | 37.08 | C |
| ATOM | 2852 | C | VAL | B | 70 | 18.339 | 66.449 | 74.747 | 1.00 | 37.08 | C |
| ATOM | 2853 | O | VAL | B | 70 | 19.486 | 66.631 | 74.362 | 1.00 | 37.08 | O |
| ATOM | 2854 | CB | VAL | B | 70 | 18.430 | 63.987 | 75.195 | 1.00 | 29.90 | C |
| ATOM | 2855 | CG1 | VAL | B | 70 | 18.765 | 64.338 | 76.643 | 1.00 | 29.90 | C |
| ATOM | 2856 | CG2 | VAL | B | 70 | 17.638 | 62.693 | 75.164 | 1.00 | 29.90 | C |
| ATOM | 2857 | N | TYR | B | 71 | 17.605 | 67.418 | 75.326 | 1.00 | 36.25 | N |
| ATOM | 2858 | CA | TYR | B | 71 | 18.199 | 68.729 | 75.555 | 1.00 | 36.25 | C |
| ATOM | 2859 | C | TYR | B | 71 | 18.782 | 68.845 | 76.965 | 1.00 | 36.25 | C |
| ATOM | 2860 | O | TYR | B | 71 | 18.431 | 68.103 | 77.874 | 1.00 | 36.25 | O |
| ATOM | 2861 | CB | TYR | B | 71 | 17.113 | 69.787 | 75.351 | 1.00 | 47.16 | C |
| ATOM | 2862 | CG | TYR | B | 71 | 16.428 | 69.563 | 74.049 | 1.00 | 47.16 | C |
| ATOM | 2863 | CD1 | TYR | B | 71 | 15.421 | 68.609 | 73.950 | 1.00 | 47.16 | C |
| ATOM | 2864 | CD2 | TYR | B | 71 | 16.783 | 70.313 | 72.927 | 1.00 | 47.16 | C |
| ATOM | 2865 | CE1 | TYR | B | 71 | 14.770 | 68.404 | 72.742 | 1.00 | 47.16 | C |
| ATOM | 2866 | CE2 | TYR | B | 71 | 16.133 | 70.108 | 71.718 | 1.00 | 47.16 | C |
| ATOM | 2867 | CZ | TYR | B | 71 | 15.130 | 69.160 | 71.624 | 1.00 | 47.16 | C |
| ATOM | 2868 | OH | TYR | B | 71 | 14.462 | 68.967 | 70.431 | 1.00 | 47.16 | O |
| ATOM | 2869 | N | GLN | B | 72 | 19.607 | 69.842 | 77.260 | 1.00 | 45.35 | N |
| ATOM | 2870 | CA | GLN | B | 72 | 20.009 | 70.092 | 78.634 | 1.00 | 45.35 | C |
| ATOM | 2871 | C | GLN | B | 72 | 19.556 | 71.524 | 78.937 | 1.00 | 45.35 | C |
| ATOM | 2872 | O | GLN | B | 72 | 19.410 | 72.340 | 78.020 | 1.00 | 45.35 | O |
| ATOM | 2873 | CB | GLN | B | 72 | 21.515 | 69.956 | 78.797 | 1.00 | 46.73 | C |
| ATOM | 2874 | CG | GLN | B | 72 | 22.239 | 71.264 | 78.969 | 1.00 | 46.73 | C |
| ATOM | 2875 | CD | GLN | B | 72 | 23.283 | 71.198 | 80.065 | 1.00 | 46.73 | C |
| ATOM | 2876 | OE1 | GLN | B | 72 | 24.143 | 72.074 | 80.161 | 1.00 | 46.73 | O |
| ATOM | 2877 | NE2 | GLN | B | 72 | 23.213 | 70.158 | 80.904 | 1.00 | 46.73 | N |
| ATOM | 2878 | N | ALA | B | 73 | 19.317 | 71.846 | 80.202 | 1.00 | 28.13 | N |
| ATOM | 2879 | CA | ALA | B | 73 | 18.861 | 73.186 | 80.499 | 1.00 | 28.13 | C |
| ATOM | 2880 | C | ALA | B | 73 | 19.069 | 73.593 | 81.933 | 1.00 | 28.13 | C |
| ATOM | 2881 | O | ALA | B | 73 | 19.506 | 72.799 | 82.776 | 1.00 | 28.13 | O |
| ATOM | 2882 | CB | ALA | B | 73 | 17.405 | 73.328 | 80.129 | 1.00 | 26.55 | C |
| ATOM | 2883 | N | LYS | B | 74 | 18.727 | 74.849 | 82.198 | 1.00 | 42.42 | N |
| ATOM | 2884 | CA | LYS | B | 74 | 18.898 | 75.434 | 83.515 | 1.00 | 42.42 | C |
| ATOM | 2885 | C | LYS | B | 74 | 17.608 | 76.043 | 84.058 | 1.00 | 42.42 | C |
| ATOM | 2886 | O | LYS | B | 74 | 17.096 | 77.025 | 83.511 | 1.00 | 42.42 | O |
| ATOM | 2887 | CB | LYS | B | 74 | 19.980 | 76.518 | 83.446 | 1.00 | 65.58 | C |
| ATOM | 2888 | CG | LYS | B | 74 | 20.341 | 77.120 | 84.788 | 1.00 | 65.58 | C |
| ATOM | 2889 | CD | LYS | B | 74 | 20.980 | 78.494 | 84.610 | 1.00 | 65.58 | C |
| ATOM | 2890 | CE | LYS | B | 74 | 21.459 | 79.062 | 85.953 | 1.00 | 65.58 | C |
| ATOM | 2891 | NZ | LYS | B | 74 | 21.878 | 80.496 | 85.840 | 1.00 | 65.58 | N |
| ATOM | 2892 | N | LEU | B | 75 | 17.088 | 75.457 | 85.133 | 1.00 | 44.40 | N |
| ATOM | 2893 | CA | LEU | B | 75 | 15.878 | 75.976 | 85.758 | 1.00 | 44.40 | C |
| ATOM | 2894 | C | LEU | B | 75 | 16.185 | 77.439 | 86.074 | 1.00 | 44.40 | C |
| ATOM | 2895 | O | LEU | B | 75 | 17.188 | 77.741 | 86.727 | 1.00 | 44.40 | O |
| ATOM | 2896 | CB | LEU | B | 75 | 15.563 | 75.216 | 87.057 | 1.00 | 30.55 | C |
| ATOM | 2897 | CG | LEU | B | 75 | 15.393 | 73.684 | 87.016 | 1.00 | 30.55 | C |
| ATOM | 2898 | CD1 | LEU | B | 75 | 14.893 | 73.192 | 88.366 | 0.00 | 30.55 | C |
| ATOM | 2899 | CD2 | LEU | B | 75 | 14.428 | 73.293 | 85.904 | 1.00 | 30.55 | C |
| ATOM | 2900 | N | CYS | B | 76 | 15.345 | 78.354 | 85.610 | 1.00 | 49.50 | N |
| ATOM | 2901 | CA | CYS | B | 76 | 15.613 | 79.756 | 85.876 | 1.00 | 49.50 | C |
| ATOM | 2902 | C | CYS | B | 76 | 15.795 | 80.076 | 87.359 | 1.00 | 49.50 | C |
| ATOM | 2903 | O | CYS | B | 76 | 16.826 | 80.632 | 87.735 | 1.00 | 49.50 | O |
| ATOM | 2904 | CB | CYS | B | 76 | 14.540 | 80.640 | 85.243 | 1.00 | 37.27 | C |
| ATOM | 2905 | SG | CYS | B | 76 | 14.594 | 80.599 | 83.398 | 1.00 | 37.27 | S |
| ATOM | 2906 | N | ASP | B | 77 | 14.852 | 79.724 | 88.226 | 1.00 | 50.48 | N |
| ATOM | 2907 | CA | ASP | B | 77 | 15.083 | 80.066 | 89.629 | 1.00 | 50.48 | C |
| ATOM | 2908 | C | ASP | B | 77 | 16.235 | 79.301 | 90.278 | 1.00 | 50.48 | C |
| ATOM | 2909 | O | ASP | B | 77 | 17.333 | 79.832 | 90.404 | 1.00 | 50.48 | O |
| ATOM | 2910 | CB | ASP | B | 77 | 13.808 | 79.924 | 90.487 | 1.00 | 89.67 | C |

| | | | | | | | | | | | |
|---|---|---|---|---|---|---|---|---|---|---|---|
| ATOM | 2911 | CG | ASP | B | 77 | 13.106 | 78.584 | 90.311 | 1.00 | 89.67 | C |
| ATOM | 2912 | OD1 | ASP | B | 77 | 13.735 | 77.614 | 89.803 | 1.00 | 89.67 | O |
| ATOM | 2913 | OD2 | ASP | B | 77 | 11.913 | 78.504 | 90.703 | 1.00 | 89.67 | O |
| ATOM | 2914 | N | SER | B | 78 | 16.002 | 78.061 | 90.690 | 1.00 | 48.72 | N |
| ATOM | 2915 | CA | SER | B | 78 | 17.057 | 77.280 | 91.333 | 1.00 | 48.72 | C |
| ATOM | 2916 | C | SER | B | 78 | 18.365 | 77.318 | 90.547 | 1.00 | 48.72 | C |
| ATOM | 2917 | O | SER | B | 78 | 19.433 | 77.094 | 91.102 | 1.00 | 48.72 | O |
| ATOM | 2918 | CB | SER | B | 78 | 16.615 | 75.818 | 91.513 | 1.00 | 60.46 | C |
| ATOM | 2919 | OG | SER | B | 78 | 16.594 | 75.116 | 90.277 | 1.00 | 60.46 | O |
| ATOM | 2920 | N | GLY | B | 79 | 18.281 | 77.592 | 89.252 | 1.00 | 53.55 | N |
| ATOM | 2921 | CA | GLY | B | 79 | 19.490 | 77.637 | 88.452 | 1.00 | 53.55 | C |
| ATOM | 2922 | C | GLY | B | 79 | 20.117 | 76.267 | 88.238 | 1.00 | 53.55 | C |
| ATOM | 2923 | O | GLY | B | 79 | 21.197 | 76.156 | 87.654 | 1.00 | 53.55 | O |
| ATOM | 2924 | N | GLU | B | 80 | 19.447 | 75.214 | 88.696 | 1.00 | 51.07 | N |
| ATOM | 2925 | CA | GLU | B | 80 | 19.975 | 73.860 | 88.534 | 1.00 | 51.07 | C |
| ATOM | 2926 | C | GLU | B | 80 | 19.954 | 73.386 | 87.081 | 1.00 | 51.07 | C |
| ATOM | 2927 | O | GLU | B | 80 | 19.295 | 73.982 | 86.219 | 1.00 | 51.07 | O |
| ATOM | 2928 | CB | GLU | B | 80 | 19.177 | 72.886 | 89.387 | 1.00 | 57.03 | C |
| ATOM | 2929 | CG | GLU | B | 80 | 19.187 | 73.210 | 90.857 | 1.00 | 57.03 | C |
| ATOM | 2930 | CD | GLU | B | 80 | 18.311 | 72.259 | 91.650 | 1.00 | 57.03 | C |
| ATOM | 2931 | OE1 | GLU | B | 80 | 18.490 | 71.026 | 91.496 | 1.00 | 57.03 | O |
| ATOM | 2932 | OE2 | GLU | B | 80 | 17.448 | 72.741 | 92.424 | 1.00 | 57.03 | O |
| ATOM | 2933 | N | LEU | B | 81 | 20.685 | 72.310 | 86.815 | 1.00 | 36.12 | N |
| ATOM | 2934 | CA | LEU | B | 81 | 20.746 | 71.758 | 85.475 | 1.00 | 36.12 | C |
| ATOM | 2935 | C | LEU | B | 81 | 19.886 | 70.514 | 85.404 | 1.00 | 36.12 | C |
| ATOM | 2936 | O | LEU | B | 81 | 19.949 | 69.631 | 86.265 | 1.00 | 36.12 | O |
| ATOM | 2937 | CB | LEU | B | 81 | 22.188 | 71.426 | 85.094 | 1.00 | 49.98 | C |
| ATOM | 2938 | CG | LEU | B | 81 | 23.133 | 72.634 | 85.029 | 1.00 | 49.98 | C |
| ATOM | 2939 | CD1 | LEU | B | 81 | 24.560 | 72.157 | 84.770 | 1.00 | 49.98 | C |
| ATOM | 2940 | CD2 | LEU | B | 81 | 22.681 | 73.587 | 83.923 | 1.00 | 49.98 | C |
| ATOM | 2941 | N | VAL | B | 82 | 19.071 | 70.461 | 84.363 | 1.00 | 36.66 | N |
| ATOM | 2942 | CA | VAL | B | 82 | 18.168 | 69.351 | 84.157 | 1.00 | 36.66 | C |
| ATOM | 2943 | C | VAL | B | 82 | 18.322 | 68.927 | 82.718 | 1.00 | 36.66 | C |
| ATOM | 2944 | O | VAL | B | 82 | 18.778 | 69.712 | 81.882 | 1.00 | 36.66 | O |
| ATOM | 2945 | CB | VAL | B | 82 | 16.695 | 69.783 | 84.391 | 1.00 | 30.54 | C |
| ATOM | 2946 | CG1 | VAL | B | 82 | 16.537 | 70.327 | 85.788 | 1.00 | 30.54 | C |
| ATOM | 2947 | CG2 | VAL | B | 82 | 16.281 | 70.847 | 83.364 | 1.00 | 30.54 | C |
| ATOM | 2948 | N | ALA | B | 83 | 17.936 | 67.689 | 82.439 | 1.00 | 27.82 | N |
| ATOM | 2949 | CA | ALA | B | 83 | 17.998 | 67.142 | 81.098 | 1.00 | 27.82 | C |
| ATOM | 2950 | C | ALA | B | 83 | 16.566 | 66.993 | 80.629 | 1.00 | 27.82 | C |
| ATOM | 2951 | O | ALA | B | 83 | 15.691 | 66.620 | 81.412 | 1.00 | 27.82 | O |
| ATOM | 2952 | CB | ALA | B | 83 | 18.672 | 65.792 | 81.129 | 1.00 | 31.27 | C |
| ATOM | 2953 | N | ILE | B | 84 | 16.309 | 67.297 | 79.362 | 1.00 | 35.11 | N |
| ATOM | 2954 | CA | ILE | B | 84 | 14.956 | 67.157 | 78.853 | 1.00 | 35.11 | C |
| ATOM | 2955 | C | ILE | B | 84 | 14.922 | 66.132 | 77.742 | 1.00 | 35.11 | C |
| ATOM | 2956 | O | ILE | B | 84 | 15.550 | 66.311 | 76.711 | 1.00 | 35.11 | O |
| ATOM | 2957 | CB | ILE | B | 84 | 14.388 | 68.482 | 78.318 | 1.00 | 31.83 | C |
| ATOM | 2958 | CG1 | ILE | B | 84 | 14.429 | 69.555 | 79.403 | 1.00 | 31.83 | C |
| ATOM | 2959 | CG2 | ILE | B | 84 | 12.940 | 68.282 | 77.902 | 1.00 | 31.83 | C |
| ATOM | 2960 | CD1 | ILE | B | 84 | 13.780 | 70.870 | 78.988 | 1.00 | 31.83 | C |
| ATOM | 2961 | N | LYS | B | 85 | 14.194 | 65.050 | 77.969 | 1.00 | 41.14 | N |
| ATOM | 2962 | CA | LYS | B | 85 | 14.065 | 63.990 | 76.990 | 1.00 | 41.14 | C |
| ATOM | 2963 | C | LYS | B | 85 | 12.688 | 64.137 | 76.372 | 1.00 | 41.14 | C |
| ATOM | 2964 | O | LYS | B | 85 | 11.681 | 63.850 | 77.015 | 1.00 | 41.14 | O |
| ATOM | 2965 | CB | LYS | B | 85 | 14.188 | 62.630 | 77.672 | 1.00 | 32.95 | C |
| ATOM | 2966 | CG | LYS | B | 85 | 14.010 | 61.453 | 76.737 | 1.00 | 32.95 | C |
| ATOM | 2967 | CD | LYS | B | 85 | 14.173 | 60.119 | 77.454 | 1.00 | 32.95 | C |
| ATOM | 2968 | CE | LYS | B | 85 | 14.088 | 58.975 | 76.459 | 1.00 | 32.95 | C |
| ATOM | 2969 | NZ | LYS | B | 85 | 14.169 | 57.645 | 77.115 | 1.00 | 32.95 | N |
| ATOM | 2970 | N | LYS | B | 86 | 12.660 | 64.578 | 75.121 | 1.00 | 36.80 | N |
| ATOM | 2971 | CA | LYS | B | 86 | 11.430 | 64.804 | 74.374 | 1.00 | 36.80 | C |
| ATOM | 2972 | C | LYS | B | 86 | 11.157 | 63.669 | 73.400 | 1.00 | 36.80 | C |

| | | | | | | | | | | | |
|---|---|---|---|---|---|---|---|---|---|---|---|
| ATOM | 2973 | O | LYS | B | 86 | 11.937 | 63.453 | 72.488 | 1.00 | 36.80 | O |
| ATOM | 2974 | CB | LYS | B | 86 | 11.587 | 66.116 | 73.606 | 1.00 | 26.01 | C |
| ATOM | 2975 | CG | LYS | B | 86 | 10.462 | 66.516 | 72.697 | 1.00 | 26.01 | C |
| ATOM | 2976 | CD | LYS | B | 86 | 10.774 | 67.890 | 72.133 | 1.00 | 26.01 | C |
| ATOM | 2977 | CE | LYS | B | 86 | 9.646 | 68.431 | 71.261 | 1.00 | 26.01 | C |
| ATOM | 2978 | NZ | LYS | B | 86 | 9.983 | 69.765 | 70.687 | 0.00 | 26.01 | N |
| ATOM | 2979 | N | VAL | B | 87 | 10.061 | 62.944 | 73.577 | 1.00 | 28.90 | N |
| ATOM | 2980 | CA | VAL | B | 87 | 9.747 | 61.852 | 72.660 | 1.00 | 28.90 | C |
| ATOM | 2981 | C | VAL | B | 87 | 8.361 | 62.065 | 72.053 | 1.00 | 28.90 | C |
| ATOM | 2982 | O | VAL | B | 87 | 7.528 | 62.748 | 72.630 | 1.00 | 28.90 | O |
| ATOM | 2983 | CB | VAL | B | 87 | 9.772 | 60.494 | 73.377 | 1.00 | 34.71 | C |
| ATOM | 2984 | CG1 | VAL | B | 87 | 10.979 | 60.419 | 74.298 | 1.00 | 34.71 | C |
| ATOM | 2985 | CG2 | VAL | B | 87 | 8.496 | 60.287 | 74.146 | 1.00 | 34.71 | C |
| ATOM | 2986 | N | LEU | B | 88 | 8.109 | 61.489 | 70.884 | 1.00 | 46.68 | N |
| ATOM | 2987 | CA | LEU | B | 88 | 6.815 | 61.659 | 70.244 | 1.00 | 46.68 | C |
| ATOM | 2988 | C | LEU | B | 88 | 5.822 | 60.776 | 70.970 | 1.00 | 46.68 | C |
| ATOM | 2989 | O | LEU | B | 88 | 6.039 | 59.576 | 71.105 | 1.00 | 46.68 | O |
| ATOM | 2990 | CB | LEU | B | 88 | 6.905 | 61.265 | 68.777 | 1.00 | 48.05 | C |
| ATOM | 2991 | CG | LEU | B | 88 | 5.891 | 61.882 | 67.808 | 1.00 | 48.05 | C |
| ATOM | 2992 | CD1 | LEU | B | 88 | 6.239 | 61.414 | 66.415 | 1.00 | 48.05 | C |
| ATOM | 2993 | CD2 | LEU | B | 88 | 4.473 | 61.472 | 68.169 | 0.00 | 48.05 | C |
| ATOM | 2994 | N | GLN | B | 89 | 4.720 | 61.355 | 71.429 | 1.00 | 48.15 | N |
| ATOM | 2995 | CA | GLN | B | 89 | 3.754 | 60.571 | 72.185 | 1.00 | 48.15 | C |
| ATOM | 2996 | C | GLN | B | 89 | 2.343 | 60.623 | 71.632 | 1.00 | 48.15 | C |
| ATOM | 2997 | O | GLN | B | 89 | 1.812 | 61.703 | 71.374 | 1.00 | 48.15 | O |
| ATOM | 2998 | CB | GLN | B | 89 | 3.759 | 61.041 | 73.643 | 0.00 | 59.22 | C |
| ATOM | 2999 | CG | GLN | B | 89 | 2.735 | 60.368 | 74.534 | 1.00 | 59.22 | C |
| ATOM | 3000 | CD | GLN | B | 89 | 2.763 | 58.847 | 74.416 | 1.00 | 59.22 | C |
| ATOM | 3001 | OE1 | GLN | B | 89 | 3.734 | 58.265 | 73.921 | 1.00 | 59.22 | O |
| ATOM | 3002 | NE2 | GLN | B | 89 | 1.696 | 58.196 | 74.882 | 1.00 | 59.22 | N |
| ATOM | 3003 | N | ASP | B | 90 | 1.736 | 59.452 | 71.452 | 1.00 | 61.58 | N |
| ATOM | 3004 | CA | ASP | B | 90 | 0.367 | 59.379 | 70.946 | 1.00 | 61.58 | C |
| ATOM | 3005 | C | ASP | B | 90 | -0.660 | 59.553 | 72.072 | 1.00 | 61.58 | C |
| ATOM | 3006 | O | ASP | B | 90 | -0.778 | 58.696 | 72.970 | 1.00 | 61.58 | O |
| ATOM | 3007 | CB | ASP | B | 90 | 0.114 | 58.049 | 70.242 | 1.00 | 58.64 | C |
| ATOM | 3008 | CG | ASP | B | 90 | -1.346 | 57.892 | 69.812 | 1.00 | 58.64 | C |
| ATOM | 3009 | OD1 | ASP | B | 90 | -2.219 | 57.757 | 70.704 | 1.00 | 58.64 | O |
| ATOM | 3010 | OD2 | ASP | B | 90 | -1.622 | 57.915 | 68.585 | 1.00 | 58.64 | O |
| ATOM | 3011 | N | LYS | B | 91 | -1.401 | 60.663 | 72.003 | 1.00 | 58.28 | N |
| ATOM | 3012 | CA | LYS | B | 91 | -2.428 | 61.012 | 72.987 | 1.00 | 58.28 | C |
| ATOM | 3013 | C | LYS | B | 91 | -3.242 | 59.802 | 73.441 | 1.00 | 58.28 | C |
| ATOM | 3014 | O | LYS | B | 91 | -3.607 | 59.708 | 74.625 | 1.00 | 58.28 | O |
| ATOM | 3015 | CB | LYS | B | 91 | -3.355 | 62.077 | 72.414 | 0.00 | 35.69 | C |
| ATOM | 3016 | N | ARG | B | 92 | -3.520 | 58.885 | 72.509 | 1.00 | 52.70 | N |
| ATOM | 3017 | CA | ARG | B | 92 | -4.287 | 57.680 | 72.821 | 1.00 | 52.70 | C |
| ATOM | 3018 | C | ARG | B | 92 | -3.540 | 56.753 | 73.799 | 1.00 | 52.70 | C |
| ATOM | 3019 | O | ARG | B | 92 | -3.967 | 56.589 | 74.945 | 1.00 | 52.70 | O |
| ATOM | 3020 | CB | ARG | B | 92 | -4.638 | 56.926 | 71.540 | 0.00 | 35.69 | C |
| ATOM | 3021 | N | PHE | B | 93 | -2.433 | 56.149 | 73.354 | 1.00 | 72.05 | N |
| ATOM | 3022 | CA | PHE | B | 93 | -1.647 | 55.243 | 74.205 | 1.00 | 72.05 | C |
| ATOM | 3023 | C | PHE | B | 93 | -1.095 | 55.968 | 75.446 | 1.00 | 72.05 | C |
| ATOM | 3024 | O | PHE | B | 93 | -0.996 | 57.209 | 75.461 | 1.00 | 72.05 | O |
| ATOM | 3025 | CB | PHE | B | 93 | -0.501 | 54.643 | 73.402 | 0.00 | 35.69 | C |
| ATOM | 3026 | N | LYS | B | 94 | -0.761 | 55.200 | 76.487 | 1.00 | 45.43 | N |
| ATOM | 3027 | CA | LYS | B | 94 | -0.200 | 55.765 | 77.715 | 1.00 | 45.43 | C |
| ATOM | 3028 | C | LYS | B | 94 | 1.288 | 55.431 | 77.688 | 1.00 | 45.43 | C |
| ATOM | 3029 | O | LYS | B | 94 | 1.656 | 54.272 | 77.491 | 1.00 | 45.43 | O |
| ATOM | 3030 | CB | LYS | B | 94 | -0.845 | 55.128 | 78.948 | 1.00 | 48.79 | C |
| ATOM | 3031 | CG | LYS | B | 94 | -1.028 | 56.084 | 80.125 | 1.00 | 48.79 | C |
| ATOM | 3032 | CD | LYS | B | 94 | -2.079 | 57.136 | 79.804 | 0.00 | 48.79 | C |
| ATOM | 3033 | CE | LYS | B | 94 | -2.347 | 58.045 | 80.991 | 0.00 | 48.79 | C |
| ATOM | 3034 | NZ | LYS | B | 94 | -3.393 | 59.058 | 80.676 | 0.00 | 48.79 | N |

| | | | | | | | | | | | |
|---|---|---|---|---|---|---|---|---|---|---|---|
| ATOM | 3035 | N | ASN | B | 95 | 2.138 | 56.436 | 77.891 | 1.00 | 32.06 | N |
| ATOM | 3036 | CA | ASN | B | 95 | 3.588 | 56.240 | 77.848 | 1.00 | 32.06 | C |
| ATOM | 3037 | C | ASN | B | 95 | 4.174 | 55.443 | 78.998 | 1.00 | 32.06 | C |
| ATOM | 3038 | O | ASN | B | 95 | 4.227 | 55.911 | 80.135 | 1.00 | 32.06 | O |
| ATOM | 3039 | CB | ASN | B | 95 | 4.301 | 57.581 | 77.771 | 1.00 | 26.97 | C |
| ATOM | 3040 | CG | ASN | B | 95 | 5.794 | 57.426 | 77.668 | 1.00 | 26.97 | C |
| ATOM | 3041 | OD1 | ASN | B | 95 | 6.446 | 56.944 | 78.589 | 1.00 | 26.97 | O |
| ATOM | 3042 | ND2 | ASN | B | 95 | 6.346 | 57.828 | 76.536 | 1.00 | 26.97 | N |
| ATOM | 3043 | N | ARG | B | 96 | 4.662 | 54.248 | 78.685 | 1.00 | 30.08 | N |
| ATOM | 3044 | CA | ARG | B | 96 | 5.224 | 53.375 | 79.696 | 1.00 | 30.08 | C |
| ATOM | 3045 | C | ARG | B | 96 | 6.360 | 53.971 | 80.511 | 1.00 | 30.08 | C |
| ATOM | 3046 | O | ARG | B | 96 | 6.392 | 53.827 | 81.740 | 1.00 | 30.08 | O |
| ATOM | 3047 | CB | ARG | B | 96 | 5.689 | 52.073 | 79.060 | 1.00 | 46.35 | C |
| ATOM | 3048 | CG | ARG | B | 96 | 5.092 | 50.883 | 79.749 | 1.00 | 46.35 | C |
| ATOM | 3049 | CD | ARG | B | 96 | 6.126 | 49.873 | 80.138 | 1.00 | 46.35 | C |
| ATOM | 3050 | NE | ARG | B | 96 | 6.598 | 49.087 | 79.001 | 1.00 | 46.35 | N |
| ATOM | 3051 | CZ | ARG | B | 96 | 5.826 | 48.302 | 78.252 | 1.00 | 46.35 | C |
| ATOM | 3052 | NH1 | ARG | B | 96 | 4.526 | 48.204 | 78.506 | 1.00 | 46.35 | N |
| ATOM | 3053 | NH2 | ARG | B | 96 | 6.361 | 47.576 | 77.275 | 1.00 | 46.35 | N |
| ATOM | 3054 | N | GLU | B | 97 | 7.290 | 54.634 | 79.833 | 1.00 | 20.49 | N |
| ATOM | 3055 | CA | GLU | B | 97 | 8.427 | 55.230 | 80.507 | 1.00 | 20.49 | C |
| ATOM | 3056 | C | GLU | B | 97 | 7.955 | 56.234 | 81.547 | 1.00 | 20.49 | C |
| ATOM | 3057 | O | GLU | B | 97 | 8.364 | 56.173 | 82.698 | 1.00 | 20.49 | O |
| ATOM | 3058 | CB | GLU | B | 97 | 9.361 | 55.895 | 79.488 | 1.00 | 29.56 | C |
| ATOM | 3059 | CG | GLU | B | 97 | 10.623 | 56.510 | 80.082 | 1.00 | 29.56 | C |
| ATOM | 3060 | CD | GLU | B | 97 | 11.613 | 56.970 | 79.026 | 1.00 | 29.56 | C |
| ATOM | 3061 | OE1 | GLU | B | 97 | 12.707 | 57.439 | 79.389 | 1.00 | 29.56 | O |
| ATOM | 3062 | OE2 | GLU | B | 97 | 11.303 | 56.865 | 77.829 | 1.00 | 29.56 | O |
| ATOM | 3063 | N | LEU | B | 98 | 7.087 | 57.153 | 81.154 | 1.00 | 20.95 | N |
| ATOM | 3064 | CA | LEU | B | 98 | 6.583 | 58.129 | 82.109 | 1.00 | 20.95 | C |
| ATOM | 3065 | C | LEU | B | 98 | 5.951 | 57.376 | 83.266 | 1.00 | 20.95 | C |
| ATOM | 3066 | O | LEU | B | 98 | 6.198 | 57.661 | 84.432 | 1.00 | 20.95 | O |
| ATOM | 3067 | CB | LEU | B | 98 | 5.520 | 59.021 | 81.459 | 1.00 | 22.17 | C |
| ATOM | 3068 | CG | LEU | B | 98 | 4.680 | 59.879 | 82.410 | 1.00 | 22.17 | C |
| ATOM | 3069 | CD1 | LEU | B | 98 | 5.549 | 60.878 | 83.141 | 1.00 | 22.17 | C |
| ATOM | 3070 | CD2 | LEU | B | 98 | 3.629 | 60.590 | 81.624 | 1.00 | 22.17 | C |
| ATOM | 3071 | N | GLN | B | 99 | 5.126 | 56.405 | 82.909 | 1.00 | 28.37 | N |
| ATOM | 3072 | CA | GLN | B | 99 | 4.422 | 55.592 | 83.871 | 1.00 | 28.37 | C |
| ATOM | 3073 | C | GLN | B | 99 | 5.297 | 55.076 | 85.007 | 1.00 | 28.37 | C |
| ATOM | 3074 | O | GLN | B | 99 | 4.988 | 55.308 | 86.174 | 1.00 | 28.37 | O |
| ATOM | 3075 | CB | GLN | B | 99 | 3.744 | 54.413 | 83.151 | 1.00 | 81.31 | C |
| ATOM | 3076 | CG | GLN | B | 99 | 2.963 | 53.459 | 84.077 | 1.00 | 81.31 | C |
| ATOM | 3077 | CD | GLN | B | 99 | 2.314 | 52.283 | 83.339 | 1.00 | 81.31 | C |
| ATOM | 3078 | OE1 | GLN | B | 99 | 1.505 | 51.546 | 83.918 | 1.00 | 81.31 | O |
| ATOM | 3079 | NE2 | GLN | B | 99 | 2.670 | 52.097 | 82.069 | 1.00 | 81.31 | N |
| ATOM | 3080 | N | ILE | B | 100 | 6.389 | 54.383 | 84.683 | 1.00 | 33.97 | N |
| ATOM | 3081 | CA | ILE | B | 100 | 7.226 | 53.832 | 85.752 | 1.00 | 33.97 | C |
| ATOM | 3082 | C | ILE | B | 100 | 8.135 | 54.860 | 86.428 | 1.00 | 33.97 | C |
| ATOM | 3083 | O | ILE | B | 100 | 8.454 | 54.719 | 87.597 | 1.00 | 33.97 | O |
| ATOM | 3084 | CB | ILE | B | 100 | 8.079 | 52.591 | 85.279 | 1.00 | 30.63 | C |
| ATOM | 3085 | CG1 | ILE | B | 100 | 7.531 | 51.265 | 84.745 | 1.00 | 30.63 | C |
| ATOM | 3086 | CG2 | ILE | B | 100 | 8.970 | 52.276 | 86.495 | 1.00 | 30.63 | C |
| ATOM | 3087 | CD1 | ILE | B | 100 | 8.319 | 50.146 | 84.062 | 1.00 | 30.63 | C |
| ATOM | 3088 | N | MET | B | 101 | 8.548 | 55.895 | 85.706 | 1.00 | 30.86 | N |
| ATOM | 3089 | CA | MET | B | 101 | 9.402 | 56.931 | 86.295 | 1.00 | 30.86 | C |
| ATOM | 3090 | C | MET | B | 101 | 8.659 | 57.539 | 87.482 | 1.00 | 30.86 | C |
| ATOM | 3091 | O | MET | B | 101 | 9.196 | 57.679 | 88.581 | 1.00 | 30.86 | O |
| ATOM | 3092 | CB | MET | B | 101 | 9.691 | 58.039 | 85.273 | 1.00 | 50.72 | C |
| ATOM | 3093 | CG | MET | B | 101 | 10.661 | 57.676 | 84.151 | 1.00 | 50.72 | C |
| ATOM | 3094 | SD | MET | B | 101 | 12.380 | 58.030 | 84.563 | 1.00 | 50.72 | S |
| ATOM | 3095 | CE | MET | B | 101 | 12.699 | 56.669 | 85.726 | 1.00 | 50.72 | C |
| ATOM | 3096 | N | ARG | B | 102 | 7.409 | 57.897 | 87.225 | 1.00 | 36.65 | N |

| | | | | | | | | | | | |
|---|---|---|---|---|---|---|---|---|---|---|---|
| ATOM | 3097 | CA | ARG | B | 102 | 6.518 | 58.505 | 88.202 | 1.00 | 36.65 | C |
| ATOM | 3098 | C | ARG | B | 102 | 6.444 | 57.845 | 89.579 | 1.00 | 36.65 | C |
| ATOM | 3099 | O | ARG | B | 102 | 6.241 | 58.534 | 90.586 | 1.00 | 36.65 | O |
| ATOM | 3100 | CB | ARG | B | 102 | 5.119 | 58.575 | 87.598 | 1.00 | 36.45 | C |
| ATOM | 3101 | CG | ARG | B | 102 | 4.998 | 59.626 | 86.541 | 1.00 | 36.45 | C |
| ATOM | 3102 | CD | ARG | B | 102 | 4.463 | 60.872 | 87.148 | 1.00 | 36.45 | C |
| ATOM | 3103 | NE | ARG | B | 102 | 3.049 | 60.960 | 86.853 | 1.00 | 36.45 | N |
| ATOM | 3104 | CZ | ARG | B | 102 | 2.211 | 61.795 | 87.451 | 1.00 | 36.45 | C |
| ATOM | 3105 | NH1 | ARG | B | 102 | 2.653 | 62.621 | 88.400 | 1.00 | 36.45 | N |
| ATOM | 3106 | NH2 | ARG | B | 102 | 0.936 | 61.813 | 87.080 | 1.00 | 36.45 | N |
| ATOM | 3107 | N | LYS | B | 103 | 6.600 | 56.523 | 89.619 | 1.00 | 27.44 | N |
| ATOM | 3108 | CA | LYS | B | 103 | 6.527 | 55.789 | 90.872 | 1.00 | 27.44 | C |
| ATOM | 3109 | C | LYS | B | 103 | 7.895 | 55.353 | 91.399 | 1.00 | 27.44 | C |
| ATOM | 3110 | O | LYS | B | 103 | 7.997 | 54.433 | 92.213 | 1.00 | 27.44 | O |
| ATOM | 3111 | CB | LYS | B | 103 | 5.623 | 54.561 | 90.700 | 1.00 | 46.06 | C |
| ATOM | 3112 | CG | LYS | B | 103 | 6.336 | 53.326 | 90.201 | 1.00 | 46.06 | C |
| ATOM | 3113 | CD | LYS | B | 103 | 5.435 | 52.108 | 90.221 | 1.00 | 46.06 | C |
| ATOM | 3114 | CE | LYS | B | 103 | 4.335 | 52.221 | 89.177 | 1.00 | 46.06 | C |
| ATOM | 3115 | NZ | LYS | B | 103 | 3.732 | 50.878 | 88.899 | 1.00 | 46.06 | N |
| ATOM | 3116 | N | LEU | B | 104 | 8.946 | 56.020 | 90.945 | 1.00 | 26.13 | N |
| ATOM | 3117 | CA | LEU | B | 104 | 10.290 | 55.675 | 91.364 | 1.00 | 26.13 | C |
| ATOM | 3118 | C | LEU | B | 104 | 10.907 | 56.751 | 92.218 | 1.00 | 26.13 | C |
| ATOM | 3119 | O | LEU | B | 104 | 10.772 | 57.932 | 91.939 | 1.00 | 26.13 | O |
| ATOM | 3120 | CB | LEU | B | 104 | 11.158 | 55.422 | 90.137 | 1.00 | 27.85 | C |
| ATOM | 3121 | CG | LEU | B | 104 | 11.366 | 53.955 | 89.770 | 1.00 | 27.85 | C |
| ATOM | 3122 | CD1 | LEU | B | 104 | 10.087 | 53.136 | 89.991 | 1.00 | 27.85 | C |
| ATOM | 3123 | CD2 | LEU | B | 104 | 11.849 | 53.890 | 88.335 | 1.00 | 27.85 | C |
| ATOM | 3124 | N | ASP | B | 105 | 11.588 | 56.344 | 93.274 | 1.00 | 26.38 | N |
| ATOM | 3125 | CA | ASP | B | 105 | 12.217 | 57.308 | 94.151 | 1.00 | 26.38 | C |
| ATOM | 3126 | C | ASP | B | 105 | 13.330 | 56.642 | 94.941 | 1.00 | 26.38 | C |
| ATOM | 3127 | O | ASP | B | 105 | 13.104 | 56.090 | 96.010 | 1.00 | 26.38 | O |
| ATOM | 3128 | CB | ASP | B | 105 | 11.173 | 57.900 | 95.082 | 1.00 | 48.94 | C |
| ATOM | 3129 | CG | ASP | B | 105 | 11.750 | 58.928 | 96.004 | 1.00 | 48.94 | C |
| ATOM | 3130 | OD1 | ASP | B | 105 | 12.593 | 59.720 | 95.528 | 1.00 | 48.94 | O |
| ATOM | 3131 | OD2 | ASP | B | 105 | 11.359 | 58.945 | 97.194 | 1.00 | 48.94 | O |
| ATOM | 3132 | N | HIS | B | 106 | 14.536 | 56.702 | 94.385 | 1.00 | 21.83 | N |
| ATOM | 3133 | CA | HIS | B | 106 | 15.719 | 56.097 | 94.978 | 1.00 | 21.83 | C |
| ATOM | 3134 | C | HIS | B | 106 | 16.930 | 56.981 | 94.695 | 1.00 | 21.83 | C |
| ATOM | 3135 | O | HIS | B | 106 | 17.095 | 57.479 | 93.591 | 1.00 | 21.83 | O |
| ATOM | 3136 | CB | HIS | B | 106 | 15.910 | 54.708 | 94.376 | 1.00 | 30.89 | C |
| ATOM | 3137 | CG | HIS | B | 106 | 16.905 | 53.863 | 95.103 | 1.00 | 30.89 | C |
| ATOM | 3138 | ND1 | HIS | B | 106 | 18.264 | 53.967 | 94.894 | 1.00 | 30.89 | N |
| ATOM | 3139 | CD2 | HIS | B | 106 | 16.738 | 52.892 | 96.029 | 1.00 | 30.89 | C |
| ATOM | 3140 | CE1 | HIS | B | 106 | 18.889 | 53.091 | 95.660 | 1.00 | 30.89 | C |
| ATOM | 3141 | NE2 | HIS | B | 106 | 17.987 | 52.425 | 96.358 | 1.00 | 30.89 | N |
| ATOM | 3142 | N | CYS | B | 107 | 17.774 | 57.181 | 95.694 | 1.00 | 21.99 | N |
| ATOM | 3143 | CA | CYS | B | 107 | 18.940 | 58.036 | 95.537 | 1.00 | 21.99 | C |
| ATOM | 3144 | C | CYS | B | 107 | 19.835 | 57.623 | 94.386 | 1.00 | 21.99 | C |
| ATOM | 3145 | O | CYS | B | 107 | 20.590 | 58.440 | 93.861 | 1.00 | 21.99 | O |
| ATOM | 3146 | CB | CYS | B | 107 | 19.751 | 58.045 | 96.824 | 1.00 | 28.50 | C |
| ATOM | 3147 | SG | CYS | B | 107 | 20.348 | 56.432 | 97.301 | 1.00 | 28.50 | S |
| ATOM | 3148 | N | ASN | B | 108 | 19.739 | 56.356 | 93.993 | 1.00 | 24.69 | N |
| ATOM | 3149 | CA | ASN | B | 108 | 20.547 | 55.824 | 92.911 | 1.00 | 24.69 | C |
| ATOM | 3150 | C | ASN | B | 108 | 19.831 | 55.690 | 91.583 | 1.00 | 24.69 | C |
| ATOM | 3151 | O | ASN | B | 108 | 20.191 | 54.855 | 90.765 | 1.00 | 24.69 | O |
| ATOM | 3152 | CB | ASN | B | 108 | 21.116 | 54.469 | 93.309 | 1.00 | 25.83 | C |
| ATOM | 3153 | CG | ASN | B | 108 | 22.234 | 54.585 | 94.308 | 1.00 | 25.83 | C |
| ATOM | 3154 | OD1 | ASN | B | 108 | 22.343 | 53.775 | 95.223 | 1.00 | 25.83 | O |
| ATOM | 3155 | ND2 | ASN | B | 108 | 23.082 | 55.584 | 94.132 | 1.00 | 25.83 | N |
| ATOM | 3156 | N | ILE | B | 109 | 18.805 | 56.496 | 91.371 | 1.00 | 18.34 | N |
| ATOM | 3157 | CA | ILE | B | 109 | 18.084 | 56.467 | 90.110 | 1.00 | 18.34 | C |
| ATOM | 3158 | C | ILE | B | 109 | 17.802 | 57.902 | 89.718 | 1.00 | 18.34 | C |

| | | | | | | | | | | | |
|---|---|---|---|---|---|---|---|---|---|---|---|
| ATOM | 3159 | O | ILE | B | 109 | 17.457 | 58.712 | 90.573 | 1.00 | 18.34 | O |
| ATOM | 3160 | CB | ILE | B | 109 | 16.772 | 55.677 | 90.220 | 1.00 | 15.88 | C |
| ATOM | 3161 | CG1 | ILE | B | 109 | 17.100 | 54.224 | 90.540 | 1.00 | 15.88 | C |
| ATOM | 3162 | CG2 | ILE | B | 109 | 15.991 | 55.766 | 88.917 | 1.00 | 15.88 | C |
| ATOM | 3163 | CD1 | ILE | B | 109 | 15.910 | 53.321 | 90.644 | 1.00 | 15.88 | C |
| ATOM | 3164 | N | VAL | B | 110 | 17.977 | 58.220 | 88.438 | 1.00 | 20.08 | N |
| ATOM | 3165 | CA | VAL | B | 110 | 17.743 | 59.580 | 87.967 | 1.00 | 20.08 | C |
| ATOM | 3166 | C | VAL | B | 110 | 16.286 | 59.966 | 88.211 | 1.00 | 20.08 | C |
| ATOM | 3167 | O | VAL | B | 110 | 15.372 | 59.246 | 87.853 | 1.00 | 20.08 | O |
| ATOM | 3168 | CB | VAL | B | 110 | 18.112 | 59.742 | 86.453 | 1.00 | 24.07 | C |
| ATOM | 3169 | CG1 | VAL | B | 110 | 17.350 | 58.744 | 85.612 | 1.00 | 24.07 | C |
| ATOM | 3170 | CG2 | VAL | B | 110 | 17.807 | 61.155 | 85.979 | 1.00 | 24.07 | C |
| ATOM | 3171 | N | ARG | B | 111 | 16.093 | 61.115 | 88.834 | 1.00 | 26.42 | N |
| ATOM | 3172 | CA | ARG | B | 111 | 14.775 | 61.604 | 89.157 | 1.00 | 26.42 | C |
| ATOM | 3173 | C | ARG | B | 111 | 14.046 | 62.356 | 88.048 | 1.00 | 26.42 | C |
| ATOM | 3174 | O | ARG | B | 111 | 14.624 | 63.185 | 87.349 | 1.00 | 26.42 | O |
| ATOM | 3175 | CB | ARG | B | 111 | 14.864 | 62.501 | 90.395 | 1.00 | 68.98 | C |
| ATOM | 3176 | CG | ARG | B | 111 | 13.553 | 63.185 | 90.760 | 1.00 | 68.98 | C |
| ATOM | 3177 | CD | ARG | B | 111 | 13.666 | 64.005 | 92.035 | 1.00 | 68.98 | C |
| ATOM | 3178 | NE | ARG | B | 111 | 14.680 | 65.047 | 91.911 | 1.00 | 68.98 | N |
| ATOM | 3179 | CZ | ARG | B | 111 | 15.002 | 65.897 | 92.883 | 1.00 | 68.98 | C |
| ATOM | 3180 | NH1 | ARG | B | 111 | 14.387 | 65.835 | 94.056 | 0.00 | 68.98 | N |
| ATOM | 3181 | NH2 | ARG | B | 111 | 15.947 | 66.812 | 92.680 | 1.00 | 68.98 | N |
| ATOM | 3182 | N | LEU | B | 112 | 12.766 | 62.050 | 87.889 | 1.00 | 24.53 | N |
| ATOM | 3183 | CA | LEU | B | 112 | 11.948 | 62.750 | 86.920 | 1.00 | 24.53 | C |
| ATOM | 3184 | C | LEU | B | 112 | 11.497 | 63.976 | 87.708 | 1.00 | 24.53 | C |
| ATOM | 3185 | O | LEU | B | 112 | 10.731 | 63.847 | 88.659 | 1.00 | 24.53 | O |
| ATOM | 3186 | CB | LEU | B | 112 | 10.737 | 61.907 | 86.534 | 1.00 | 24.78 | C |
| ATOM | 3187 | CG | LEU | B | 112 | 9.638 | 62.629 | 85.747 | 1.00 | 24.78 | C |
| ATOM | 3188 | CD1 | LEU | B | 112 | 10.146 | 63.010 | 84.373 | 1.00 | 24.78 | C |
| ATOM | 3189 | CD2 | LEU | B | 112 | 8.406 | 61.725 | 85.631 | 1.00 | 24.78 | C |
| ATOM | 3190 | N | ARG | B | 113 | 11.990 | 65.156 | 87.354 | 1.00 | 27.71 | N |
| ATOM | 3191 | CA | ARG | B | 113 | 11.594 | 66.364 | 88.079 | 1.00 | 27.71 | C |
| ATOM | 3192 | C | ARG | B | 113 | 10.203 | 66.821 | 87.622 | 1.00 | 27.71 | C |
| ATOM | 3193 | O | ARG | B | 113 | 9.351 | 67.169 | 88.430 | 1.00 | 27.71 | O |
| ATOM | 3194 | CB | ARG | B | 113 | 12.589 | 67.499 | 87.824 | 1.00 | 55.49 | C |
| ATOM | 3195 | CG | ARG | B | 113 | 14.038 | 67.077 | 87.765 | 1.00 | 55.49 | C |
| ATOM | 3196 | CD | ARG | B | 113 | 14.657 | 66.937 | 89.142 | 1.00 | 55.49 | C |
| ATOM | 3197 | NE | ARG | B | 113 | 15.688 | 67.948 | 89.388 | 1.00 | 55.49 | N |
| ATOM | 3198 | CZ | ARG | B | 113 | 15.442 | 69.234 | 89.626 | 1.00 | 55.49 | C |
| ATOM | 3199 | NH1 | ARG | B | 113 | 14.191 | 69.683 | 89.651 | 1.00 | 55.49 | N |
| ATOM | 3200 | NH2 | ARG | B | 113 | 16.449 | 70.069 | 89.857 | 1.00 | 55.49 | N |
| ATOM | 3201 | N | TYR | B | 114 | 9.995 | 66.824 | 86.310 | 1.00 | 41.56 | N |
| ATOM | 3202 | CA | TYR | B | 114 | 8.730 | 67.243 | 85.731 | 1.00 | 41.56 | C |
| ATOM | 3203 | C | TYR | B | 114 | 8.515 | 66.511 | 84.423 | 1.00 | 41.56 | C |
| ATOM | 3204 | O | TYR | B | 114 | 9.331 | 65.701 | 84.015 | 1.00 | 41.56 | O |
| ATOM | 3205 | CB | TYR | B | 114 | 8.753 | 68.747 | 85.436 | 1.00 | 37.17 | C |
| ATOM | 3206 | CG | TYR | B | 114 | 8.980 | 69.612 | 86.641 | 1.00 | 37.17 | C |
| ATOM | 3207 | CD1 | TYR | B | 114 | 7.989 | 69.765 | 87.601 | 1.00 | 37.17 | C |
| ATOM | 3208 | CD2 | TYR | B | 114 | 10.208 | 70.240 | 86.851 | 1.00 | 37.17 | C |
| ATOM | 3209 | CE1 | TYR | B | 114 | 8.210 | 70.518 | 88.743 | 1.00 | 37.17 | C |
| ATOM | 3210 | CE2 | TYR | B | 114 | 10.441 | 70.990 | 87.993 | 1.00 | 37.17 | C |
| ATOM | 3211 | CZ | TYR | B | 114 | 9.436 | 71.122 | 88.933 | 1.00 | 37.17 | C |
| ATOM | 3212 | OH | TYR | B | 114 | 9.657 | 71.845 | 90.079 | 1.00 | 37.17 | O |
| ATOM | 3213 | N | PHE | B | 115 | 7.395 | 66.807 | 83.784 | 1.00 | 30.06 | N |
| ATOM | 3214 | CA | PHE | B | 115 | 7.062 | 66.264 | 82.482 | 1.00 | 30.06 | C |
| ATOM | 3215 | C | PHE | B | 115 | 5.959 | 67.157 | 81.965 | 1.00 | 30.06 | C |
| ATOM | 3216 | O | PHE | B | 115 | 5.232 | 67.767 | 82.742 | 1.00 | 30.06 | O |
| ATOM | 3217 | CB | PHE | B | 115 | 6.643 | 64.781 | 82.548 | 1.00 | 30.07 | C |
| ATOM | 3218 | CG | PHE | B | 115 | 5.346 | 64.516 | 83.254 | 1.00 | 30.07 | C |
| ATOM | 3219 | CD1 | PHE | B | 115 | 4.167 | 64.372 | 82.539 | 1.00 | 30.07 | C |
| ATOM | 3220 | CD2 | PHE | B | 115 | 5.314 | 64.329 | 84.627 | 1.00 | 30.07 | C |

| | | | | | | | | | | | |
|---|---|---|---|---|---|---|---|---|---|---|---|
| ATOM | 3221 | CE1 | PHE | B | 115 | 2.977 | 64.039 | 83.180 | 1.00 | 30.07 | C |
| ATOM | 3222 | CE2 | PHE | B | 115 | 4.124 | 63.995 | 85.277 | 1.00 | 30.07 | C |
| ATOM | 3223 | CZ | PHE | B | 115 | 2.958 | 63.849 | 84.550 | 1.00 | 30.07 | C |
| ATOM | 3224 | N | PHE | B | 116 | 5.877 | 67.281 | 80.652 | 1.00 | 31.41 | N |
| ATOM | 3225 | CA | PHE | B | 116 | 4.869 | 68.124 | 80.045 | 1.00 | 31.41 | C |
| ATOM | 3226 | C | PHE | B | 116 | 4.780 | 67.808 | 78.568 | 1.00 | 31.41 | C |
| ATOM | 3227 | O | PHE | B | 116 | 5.650 | 67.134 | 78.031 | 1.00 | 31.41 | O |
| ATOM | 3228 | CB | PHE | B | 116 | 5.213 | 69.605 | 80.282 | 1.00 | 24.12 | C |
| ATOM | 3229 | CG | PHE | B | 116 | 6.529 | 70.058 | 79.687 | 1.00 | 24.12 | C |
| ATOM | 3230 | CD1 | PHE | B | 116 | 6.649 | 70.308 | 78.328 | 1.00 | 24.12 | C |
| ATOM | 3231 | CD2 | PHE | B | 116 | 7.632 | 70.288 | 80.501 | 1.00 | 24.12 | C |
| ATOM | 3232 | CE1 | PHE | B | 116 | 7.854 | 70.783 | 77.786 | 1.00 | 24.12 | C |
| ATOM | 3233 | CE2 | PHE | B | 116 | 8.837 | 70.762 | 79.972 | 1.00 | 24.12 | C |
| ATOM | 3234 | CZ | PHE | B | 116 | 8.946 | 71.011 | 78.612 | 1.00 | 24.12 | C |
| ATOM | 3235 | N | TYR | B | 117 | 3.721 | 68.267 | 77.913 | 1.00 | 37.66 | N |
| ATOM | 3236 | CA | TYR | B | 117 | 3.570 | 68.012 | 76.485 | 1.00 | 37.66 | C |
| ATOM | 3237 | C | TYR | B | 117 | 3.758 | 69.295 | 75.690 | 1.00 | 37.66 | C |
| ATOM | 3238 | O | TYR | B | 117 | 3.533 | 70.389 | 76.205 | 1.00 | 37.66 | O |
| ATOM | 3239 | CB | TYR | B | 117 | 2.200 | 67.402 | 76.184 | 1.00 | 27.23 | C |
| ATOM | 3240 | CG | TYR | B | 117 | 1.917 | 66.165 | 76.994 | 1.00 | 27.23 | C |
| ATOM | 3241 | CD1 | TYR | B | 117 | 1.417 | 66.257 | 78.286 | 1.00 | 27.23 | C |
| ATOM | 3242 | CD2 | TYR | B | 117 | 2.196 | 64.900 | 76.489 | 1.00 | 27.23 | C |
| ATOM | 3243 | CE1 | TYR | B | 117 | 1.204 | 65.131 | 79.055 | 1.00 | 27.23 | C |
| ATOM | 3244 | CE2 | TYR | B | 117 | 1.987 | 63.760 | 77.258 | 1.00 | 27.23 | C |
| ATOM | 3245 | CZ | TYR | B | 117 | 1.494 | 63.888 | 78.539 | 1.00 | 27.23 | C |
| ATOM | 3246 | OH | TYR | B | 117 | 1.326 | 62.775 | 79.323 | 1.00 | 27.23 | O |
| ATOM | 3247 | N | SER | B | 118 | 4.187 | 69.149 | 74.438 | 1.00 | 46.94 | N |
| ATOM | 3248 | CA | SER | B | 118 | 4.414 | 70.287 | 73.550 | 1.00 | 46.94 | C |
| ATOM | 3249 | C | SER | B | 118 | 4.074 | 69.910 | 72.099 | 1.00 | 46.94 | C |
| ATOM | 3250 | O | SER | B | 118 | 4.019 | 68.723 | 71.747 | 1.00 | 46.94 | O |
| ATOM | 3251 | CB | SER | B | 118 | 5.869 | 70.729 | 73.640 | 1.00 | 56.00 | C |
| ATOM | 3252 | OG | SER | B | 118 | 6.714 | 69.722 | 73.110 | 1.00 | 56.00 | O |
| ATOM | 3253 | N | SER | B | 119 | 3.858 | 70.928 | 71.266 | 1.00 | 78.48 | N |
| ATOM | 3254 | CA | SER | B | 119 | 3.492 | 70.733 | 69.860 | 1.00 | 78.48 | C |
| ATOM | 3255 | C | SER | B | 119 | 4.583 | 70.157 | 68.969 | 1.00 | 78.48 | C |
| ATOM | 3256 | O | SER | B | 119 | 5.676 | 70.740 | 68.817 | 1.00 | 78.48 | O |
| ATOM | 3257 | CB | SER | B | 119 | 2.993 | 72.045 | 69.243 | 1.00 | 47.32 | C |
| ATOM | 3258 | OG | SER | B | 119 | 1.600 | 72.185 | 69.455 | 1.00 | 47.32 | O |
| ATOM | 3259 | N | GLY | B | 120 | 4.264 | 69.009 | 68.369 | 1.00100.00 | | N |
| ATOM | 3260 | CA | GLY | B | 120 | 5.217 | 68.356 | 67.479 | 1.00100.00 | | C |
| ATOM | 3261 | C | GLY | B | 120 | 5.431 | 69.166 | 66.200 | 1.00100.00 | | C |
| ATOM | 3262 | O | GLY | B | 120 | 4.695 | 70.089 | 65.880 | 1.00100.00 | | O |
| ATOM | 3263 | N | GLU | B | 121 | 6.508 | 68.813 | 65.475 | 1.00 | 91.82 | N |
| ATOM | 3264 | CA | GLU | B | 121 | 6.806 | 69.526 | 64.239 | 1.00 | 91.82 | C |
| ATOM | 3265 | C | GLU | B | 121 | 5.623 | 69.482 | 63.270 | 1.00 | 91.82 | C |
| ATOM | 3266 | O | GLU | B | 121 | 5.507 | 70.268 | 62.338 | 1.00 | 91.82 | O |
| ATOM | 3267 | CB | GLU | B | 121 | 8.033 | 68.876 | 63.596 | 1.00 | 31.03 | C |
| ATOM | 3268 | N | LYS | B | 122 | 4.744 | 68.491 | 63.496 | 1.00 | 74.25 | N |
| ATOM | 3269 | CA | LYS | B | 122 | 3.597 | 68.330 | 62.613 | 1.00 | 74.25 | C |
| ATOM | 3270 | C | LYS | B | 122 | 2.317 | 68.854 | 63.264 | 1.00 | 74.25 | C |
| ATOM | 3271 | O | LYS | B | 122 | 2.327 | 69.478 | 64.316 | 1.00 | 74.25 | O |
| ATOM | 3272 | CB | LYS | B | 122 | 3.447 | 66.843 | 62.292 | 0.00 | 35.69 | C |
| ATOM | 3273 | N | LYS | B | 123 | 1.188 | 68.617 | 62.574 | 1.00 | 83.38 | N |
| ATOM | 3274 | CA | LYS | B | 123 | -0.083 | 69.095 | 63.102 | 1.00 | 83.38 | C |
| ATOM | 3275 | C | LYS | B | 123 | -0.652 | 68.139 | 64.154 | 1.00 | 83.38 | C |
| ATOM | 3276 | O | LYS | B | 123 | -0.767 | 68.466 | 65.328 | 1.00 | 83.38 | O |
| ATOM | 3277 | CB | LYS | B | 123 | -1.062 | 69.232 | 61.935 | 1.00 | 56.49 | C |
| ATOM | 3278 | N | ASP | B | 124 | -1.057 | 66.885 | 63.954 | 1.00 | 86.23 | N |
| ATOM | 3279 | CA | ASP | B | 124 | -1.570 | 66.054 | 65.047 | 1.00 | 86.23 | C |
| ATOM | 3280 | C | ASP | B | 124 | -0.474 | 65.322 | 65.849 | 1.00 | 86.23 | C |
| ATOM | 3281 | O | ASP | B | 124 | -0.759 | 64.317 | 66.500 | 0.00 | 86.23 | O |
| ATOM | 3282 | CB | ASP | B | 124 | -2.568 | 65.045 | 64.495 | 0.00 | 35.69 | C |

| | | | | | | | | | | | |
|---|---|---|---|---|---|---|---|---|---|---|---|
| ATOM | 3283 | N | GLU | B | 125 | 0.773 | 65.812 | 65.801 | 1.00 | 71.69 | N |
| ATOM | 3284 | CA | GLU | B | 125 | 1.853 | 65.158 | 66.558 | 1.00 | 71.69 | C |
| ATOM | 3285 | C | GLU | B | 125 | 2.221 | 65.936 | 67.813 | 1.00 | 71.69 | C |
| ATOM | 3286 | O | GLU | B | 125 | 2.446 | 67.147 | 67.768 | 1.00 | 71.69 | O |
| ATOM | 3287 | CB | GLU | B | 125 | 3.113 | 64.929 | 65.697 | 1.00 | 71.11 | C |
| ATOM | 3288 | CG | GLU | B | 125 | 3.815 | 66.178 | 65.168 | 1.00 | 71.11 | C |
| ATOM | 3289 | CD | GLU | B | 125 | 5.144 | 65.846 | 64.459 | 1.00 | 71.11 | C |
| ATOM | 3290 | OE1 | GLU | B | 125 | 5.177 | 64.913 | 63.601 | 1.00 | 71.11 | O |
| ATOM | 3291 | OE2 | GLU | B | 125 | 6.161 | 66.525 | 64.760 | 1.00 | 71.11 | O |
| ATOM | 3292 | N | VAL | B | 126 | 2.266 | 65.234 | 68.938 | 1.00 | 34.98 | N |
| ATOM | 3293 | CA | VAL | B | 126 | 2.604 | 65.884 | 70.186 | 1.00 | 34.98 | C |
| ATOM | 3294 | C | VAL | B | 126 | 3.668 | 65.082 | 70.916 | 1.00 | 34.98 | C |
| ATOM | 3295 | O | VAL | B | 126 | 3.723 | 63.846 | 70.828 | 1.00 | 34.98 | O |
| ATOM | 3296 | CB | VAL | B | 126 | 1.345 | 66.060 | 71.091 | 1.00 | 35.27 | C |
| ATOM | 3297 | CG1 | VAL | B | 126 | 0.146 | 66.450 | 70.228 | 1.00 | 35.27 | C |
| ATOM | 3298 | CG2 | VAL | B | 126 | 1.049 | 64.788 | 71.861 | 1.00 | 35.27 | C |
| ATOM | 3299 | N | TYR | B | 127 | 4.517 | 65.796 | 71.641 | 1.00 | 37.11 | N |
| ATOM | 3300 | CA | TYR | B | 127 | 5.581 | 65.152 | 72.371 | 1.00 | 37.11 | C |
| ATOM | 3301 | C | TYR | B | 127 | 5.386 | 65.159 | 73.866 | 1.00 | 37.11 | C |
| ATOM | 3302 | O | TYR | B | 127 | 4.664 | 65.983 | 74.415 | 1.00 | 37.11 | O |
| ATOM | 3303 | CB | TYR | B | 127 | 6.900 | 65.834 | 72.075 | 1.00 | 69.17 | C |
| ATOM | 3304 | CG | TYR | B | 127 | 7.322 | 65.772 | 70.642 | 1.00 | 69.17 | C |
| ATOM | 3305 | CD1 | TYR | B | 127 | 7.277 | 66.907 | 69.841 | 1.00 | 69.17 | C |
| ATOM | 3306 | CD2 | TYR | B | 127 | 7.820 | 64.590 | 70.095 | 1.00 | 69.17 | C |
| ATOM | 3307 | CE1 | TYR | B | 127 | 7.730 | 66.878 | 68.530 | 1.00 | 69.17 | C |
| ATOM | 3308 | CE2 | TYR | B | 127 | 8.273 | 64.540 | 68.778 | 1.00 | 69.17 | C |
| ATOM | 3309 | CZ | TYR | B | 127 | 8.227 | 65.696 | 67.998 | 1.00 | 69.17 | C |
| ATOM | 3310 | OH | TYR | B | 127 | 8.678 | 65.686 | 66.690 | 1.00 | 69.17 | O |
| ATOM | 3311 | N | LEU | B | 128 | 6.063 | 64.221 | 74.514 | 1.00 | 35.76 | N |
| ATOM | 3312 | CA | LEU | B | 128 | 6.055 | 64.089 | 75.958 | 1.00 | 35.76 | C |
| ATOM | 3313 | C | LEU | B | 128 | 7.447 | 64.560 | 76.341 | 1.00 | 35.76 | C |
| ATOM | 3314 | O | LEU | B | 128 | 8.435 | 64.095 | 75.783 | 1.00 | 35.76 | O |
| ATOM | 3315 | CB | LEU | B | 128 | 5.861 | 62.630 | 76.360 | 1.00 | 23.71 | C |
| ATOM | 3316 | CG | LEU | B | 128 | 6.155 | 62.311 | 77.823 | 1.00 | 23.71 | C |
| ATOM | 3317 | CD1 | LEU | B | 128 | 5.191 | 63.066 | 78.730 | 1.00 | 23.71 | C |
| ATOM | 3318 | CD2 | LEU | B | 128 | 6.040 | 60.812 | 78.040 | 1.00 | 23.71 | C |
| ATOM | 3319 | N | ASN | B | 129 | 7.524 | 65.498 | 77.270 | 1.00 | 26.19 | N |
| ATOM | 3320 | CA | ASN | B | 129 | 8.807 | 66.034 | 77.698 | 1.00 | 26.19 | C |
| ATOM | 3321 | C | ASN | B | 129 | 9.159 | 65.533 | 79.080 | 1.00 | 26.19 | C |
| ATOM | 3322 | O | ASN | B | 129 | 8.418 | 65.752 | 80.031 | 1.00 | 26.19 | O |
| ATOM | 3323 | CB | ASN | B | 129 | 8.756 | 67.563 | 77.702 | 1.00 | 38.77 | C |
| ATOM | 3324 | CG | ASN | B | 129 | 8.549 | 68.144 | 76.313 | 1.00 | 38.77 | C |
| ATOM | 3325 | OD1 | ASN | B | 129 | 9.482 | 68.657 | 75.697 | 1.00 | 38.77 | O |
| ATOM | 3326 | ND2 | ASN | B | 129 | 7.324 | 68.059 | 75.811 | 1.00 | 38.77 | N |
| ATOM | 3327 | N | LEU | B | 130 | 10.290 | 64.851 | 79.189 | 1.00 | 33.93 | N |
| ATOM | 3328 | CA | LEU | B | 130 | 10.736 | 64.333 | 80.475 | 1.00 | 33.93 | C |
| ATOM | 3329 | C | LEU | B | 130 | 11.864 | 65.194 | 81.021 | 1.00 | 33.93 | C |
| ATOM | 3330 | O | LEU | B | 130 | 12.980 | 65.175 | 80.500 | 1.00 | 33.93 | O |
| ATOM | 3331 | CB | LEU | B | 130 | 11.203 | 62.878 | 80.336 | 1.00 | 30.88 | C |
| ATOM | 3332 | CG | LEU | B | 130 | 10.092 | 61.821 | 80.356 | 1.00 | 30.88 | C |
| ATOM | 3333 | CD1 | LEU | B | 130 | 9.086 | 62.143 | 79.288 | 1.00 | 30.88 | C |
| ATOM | 3334 | CD2 | LEU | B | 130 | 10.661 | 60.431 | 80.146 | 1.00 | 30.88 | C |
| ATOM | 3335 | N | VAL | B | 131 | 11.556 | 65.969 | 82.056 | 1.00 | 34.92 | N |
| ATOM | 3336 | CA | VAL | B | 131 | 12.541 | 66.833 | 82.686 | 1.00 | 34.92 | C |
| ATOM | 3337 | C | VAL | B | 131 | 13.202 | 66.033 | 83.783 | 1.00 | 34.92 | C |
| ATOM | 3338 | O | VAL | B | 131 | 12.641 | 65.866 | 84.865 | 1.00 | 34.92 | O |
| ATOM | 3339 | CB | VAL | B | 131 | 11.895 | 68.067 | 83.309 | 1.00 | 27.17 | C |
| ATOM | 3340 | CG1 | VAL | B | 131 | 12.909 | 68.802 | 84.166 | 1.00 | 27.17 | C |
| ATOM | 3341 | CG2 | VAL | B | 131 | 11.374 | 68.974 | 82.217 | 1.00 | 27.17 | C |
| ATOM | 3342 | N | LEU | B | 132 | 14.399 | 65.540 | 83.492 | 1.00 | 31.94 | N |
| ATOM | 3343 | CA | LEU | B | 132 | 15.149 | 64.731 | 84.435 | 1.00 | 31.94 | C |
| ATOM | 3344 | C | LEU | B | 132 | 16.348 | 65.456 | 84.988 | 1.00 | 31.94 | C |

| | | | | | | | | | | | |
|---|---|---|---|---|---|---|---|---|---|---|---|
| ATOM | 3345 | O | LEU | B | 132 | 16.743 | 66.495 | 84.485 | 1.00 | 31.94 | O |
| ATOM | 3346 | CB | LEU | B | 132 | 15.638 | 63.476 | 83.739 | 1.00 | 26.39 | C |
| ATOM | 3347 | CG | LEU | B | 132 | 14.522 | 62.679 | 83.087 | 1.00 | 26.39 | C |
| ATOM | 3348 | CD1 | LEU | B | 132 | 15.005 | 62.182 | 81.741 | 1.00 | 26.39 | C |
| ATOM | 3349 | CD2 | LEU | B | 132 | 14.087 | 61.547 | 83.999 | 1.00 | 26.39 | C |
| ATOM | 3350 | N | ASP | B | 133 | 16.925 | 64.900 | 86.041 | 1.00 | 31.67 | N |
| ATOM | 3351 | CA | ASP | B | 133 | 18.128 | 65.470 | 86.617 | 1.00 | 31.67 | C |
| ATOM | 3352 | C | ASP | B | 133 | 19.228 | 65.315 | 85.576 | 1.00 | 31.67 | C |
| ATOM | 3353 | O | ASP | B | 133 | 19.218 | 64.374 | 84.782 | 1.00 | 31.67 | O |
| ATOM | 3354 | CB | ASP | B | 133 | 18.518 | 64.707 | 87.873 | 1.00 | 40.71 | C |
| ATOM | 3355 | CG | ASP | B | 133 | 17.855 | 65.255 | 89.102 | 1.00 | 40.71 | C |
| ATOM | 3356 | OD1 | ASP | B | 133 | 17.718 | 64.495 | 90.072 | 1.00 | 40.71 | O |
| ATOM | 3357 | OD2 | ASP | B | 133 | 17.483 | 66.448 | 89.106 | 1.00 | 40.71 | O |
| ATOM | 3358 | N | TYR | B | 134 | 20.172 | 66.242 | 85.559 | 1.00 | 35.47 | N |
| ATOM | 3359 | CA | TYR | B | 134 | 21.268 | 66.141 | 84.613 | 1.00 | 35.47 | C |
| ATOM | 3360 | C | TYR | B | 134 | 22.488 | 65.568 | 85.323 | 1.00 | 35.47 | C |
| ATOM | 3361 | O | TYR | B | 134 | 22.778 | 65.907 | 86.471 | 1.00 | 35.47 | O |
| ATOM | 3362 | CB | TYR | B | 134 | 21.613 | 67.506 | 84.037 | 1.00 | 64.33 | C |
| ATOM | 3363 | CG | TYR | B | 134 | 22.881 | 67.492 | 83.211 | 1.00 | 64.33 | C |
| ATOM | 3364 | CD1 | TYR | B | 134 | 22.903 | 66.946 | 81.929 | 1.00 | 64.33 | C |
| ATOM | 3365 | CD2 | TYR | B | 134 | 24.065 | 68.010 | 83.727 | 1.00 | 64.33 | C |
| ATOM | 3366 | CE1 | TYR | B | 134 | 24.069 | 66.919 | 81.190 | 1.00 | 64.33 | C |
| ATOM | 3367 | CE2 | TYR | B | 134 | 25.232 | 67.986 | 82.999 | 1.00 | 64.33 | C |
| ATOM | 3368 | CZ | TYR | B | 134 | 25.231 | 67.440 | 81.735 | 1.00 | 64.33 | C |
| ATOM | 3369 | OH | TYR | B | 134 | 26.422 | 67.399 | 81.044 | 1.00 | 64.33 | O |
| ATOM | 3370 | N | VAL | B | 135 | 23.192 | 64.675 | 84.650 | 1.00 | 30.56 | N |
| ATOM | 3371 | CA | VAL | B | 135 | 24.370 | 64.089 | 85.243 | 1.00 | 30.56 | C |
| ATOM | 3372 | C | VAL | B | 135 | 25.398 | 63.981 | 84.141 | 1.00 | 30.56 | C |
| ATOM | 3373 | O | VAL | B | 135 | 25.229 | 63.241 | 83.170 | 1.00 | 30.56 | O |
| ATOM | 3374 | CB | VAL | B | 135 | 24.056 | 62.738 | 85.852 | 1.00 | 22.63 | C |
| ATOM | 3375 | CG1 | VAL | B | 135 | 25.287 | 62.169 | 86.517 | 1.00 | 22.63 | C |
| ATOM | 3376 | CG2 | VAL | B | 135 | 22.943 | 62.902 | 86.862 | 1.00 | 22.63 | C |
| ATOM | 3377 | N | PRO | B | 136 | 26.492 | 64.734 | 84.289 | 1.00 | 35.98 | N |
| ATOM | 3378 | CA | PRO | B | 136 | 27.631 | 64.832 | 83.368 | 1.00 | 35.98 | C |
| ATOM | 3379 | C | PRO | B | 136 | 28.259 | 63.512 | 82.899 | 1.00 | 35.98 | C |
| ATOM | 3380 | O | PRO | B | 136 | 28.084 | 63.107 | 81.750 | 1.00 | 35.98 | O |
| ATOM | 3381 | CB | PRO | B | 136 | 28.610 | 65.700 | 84.145 | 1.00 | 40.90 | C |
| ATOM | 3382 | CG | PRO | B | 136 | 28.342 | 65.271 | 85.585 | 1.00 | 40.90 | C |
| ATOM | 3383 | CD | PRO | B | 136 | 26.847 | 65.256 | 85.626 | 1.00 | 40.90 | C |
| ATOM | 3384 | N | GLU | B | 137 | 28.984 | 62.837 | 83.781 | 1.00 | 28.75 | N |
| ATOM | 3385 | CA | GLU | B | 137 | 29.637 | 61.594 | 83.396 | 1.00 | 28.75 | C |
| ATOM | 3386 | C | GLU | B | 137 | 28.745 | 60.355 | 83.351 | 1.00 | 28.75 | C |
| ATOM | 3387 | O | GLU | B | 137 | 27.585 | 60.375 | 83.757 | 1.00 | 28.75 | O |
| ATOM | 3388 | CB | GLU | B | 137 | 30.807 | 61.301 | 84.334 | 1.00 | 50.44 | C |
| ATOM | 3389 | CG | GLU | B | 137 | 31.938 | 62.303 | 84.315 | 0.00 | 50.44 | C |
| ATOM | 3390 | CD | GLU | B | 137 | 32.535 | 62.479 | 82.939 | 1.00 | 50.44 | C |
| ATOM | 3391 | OE1 | GLU | B | 137 | 32.658 | 61.478 | 82.197 | 1.00 | 50.44 | O |
| ATOM | 3392 | OE2 | GLU | B | 137 | 32.897 | 63.624 | 82.599 | 1.00 | 50.44 | O |
| ATOM | 3393 | N | THR | B | 138 | 29.324 | 59.277 | 82.840 | 1.00 | 27.67 | N |
| ATOM | 3394 | CA | THR | B | 138 | 28.665 | 57.981 | 82.754 | 1.00 | 27.67 | C |
| ATOM | 3395 | C | THR | B | 138 | 29.761 | 56.954 | 82.974 | 1.00 | 27.67 | C |
| ATOM | 3396 | O | THR | B | 138 | 30.930 | 57.240 | 82.757 | 1.00 | 27.67 | O |
| ATOM | 3397 | CB | THR | B | 138 | 28.037 | 57.714 | 81.367 | 1.00 | 24.07 | C |
| ATOM | 3398 | OG1 | THR | B | 138 | 29.067 | 57.644 | 80.383 | 1.00 | 24.07 | O |
| ATOM | 3399 | CG2 | THR | B | 138 | 27.059 | 58.803 | 80.995 | 1.00 | 24.07 | C |
| ATOM | 3400 | N | VAL | B | 139 | 29.389 | 55.768 | 83.427 | 1.00 | 24.31 | N |
| ATOM | 3401 | CA | VAL | B | 139 | 30.368 | 54.723 | 83.636 | 1.00 | 24.31 | C |
| ATOM | 3402 | C | VAL | B | 139 | 31.078 | 54.456 | 82.320 | 1.00 | 24.31 | C |
| ATOM | 3403 | O | VAL | B | 139 | 32.248 | 54.100 | 82.310 | 1.00 | 24.31 | O |
| ATOM | 3404 | CB | VAL | B | 139 | 29.701 | 53.423 | 84.137 | 1.00 | 25.24 | C |
| ATOM | 3405 | CG1 | VAL | B | 139 | 30.678 | 52.246 | 84.046 | 1.00 | 25.24 | C |
| ATOM | 3406 | CG2 | VAL | B | 139 | 29.257 | 53.614 | 85.560 | 1.00 | 25.24 | C |

| | | | | | | | | | | | |
|---|---|---|---|---|---|---|---|---|---|---|---|
| ATOM | 3407 | N | TYR | B | 140 | 30.364 | 54.640 | 81.214 | 1.00 | 26.09 | N |
| ATOM | 3408 | CA | TYR | B | 140 | 30.928 | 54.424 | 79.889 | 1.00 | 26.09 | C |
| ATOM | 3409 | C | TYR | B | 140 | 32.098 | 55.377 | 79.610 | 1.00 | 26.09 | C |
| ATOM | 3410 | O | TYR | B | 140 | 33.233 | 54.933 | 79.424 | 1.00 | 26.09 | O |
| ATOM | 3411 | CB | TYR | B | 140 | 29.867 | 54.624 | 78.811 | 1.00 | 42.32 | C |
| ATOM | 3412 | CG | TYR | B | 140 | 30.452 | 54.552 | 77.424 | 1.00 | 42.32 | C |
| ATOM | 3413 | CD1 | TYR | B | 140 | 30.799 | 53.325 | 76.860 | 1.00 | 42.32 | C |
| ATOM | 3414 | CD2 | TYR | B | 140 | 30.732 | 55.713 | 76.702 | 1.00 | 42.32 | C |
| ATOM | 3415 | CE1 | TYR | B | 140 | 31.410 | 53.248 | 75.617 | 1.00 | 42.32 | C |
| ATOM | 3416 | CE2 | TYR | B | 140 | 31.348 | 55.648 | 75.458 | 1.00 | 42.32 | C |
| ATOM | 3417 | CZ | TYR | B | 140 | 31.684 | 54.406 | 74.920 | 1.00 | 42.32 | C |
| ATOM | 3418 | OH | TYR | B | 140 | 32.297 | 54.311 | 73.686 | 1.00 | 42.32 | O |
| ATOM | 3419 | N | ARG | B | 141 | 31.816 | 56.682 | 79.564 | 1.00 | 33.10 | N |
| ATOM | 3420 | CA | ARG | B | 141 | 32.849 | 57.680 | 79.312 | 1.00 | 33.10 | C |
| ATOM | 3421 | C | ARG | B | 141 | 34.049 | 57.465 | 80.220 | 1.00 | 33.10 | C |
| ATOM | 3422 | O | ARG | B | 141 | 35.197 | 57.567 | 79.782 | 1.00 | 33.10 | O |
| ATOM | 3423 | CB | ARG | B | 141 | 32.329 | 59.100 | 79.546 | 1.00 | 64.61 | C |
| ATOM | 3424 | CG | ARG | B | 141 | 31.473 | 59.684 | 78.441 | 1.00 | 64.61 | C |
| ATOM | 3425 | CD | ARG | B | 141 | 31.388 | 61.205 | 78.609 | 1.00 | 64.61 | C |
| ATOM | 3426 | NE | ARG | B | 141 | 32.711 | 61.828 | 78.451 | 1.00 | 64.61 | N |
| ATOM | 3427 | CZ | ARG | B | 141 | 33.190 | 62.808 | 79.221 | 1.00 | 64.61 | C |
| ATOM | 3428 | NH1 | ARG | B | 141 | 32.457 | 63.296 | 80.218 | 1.00 | 64.61 | N |
| ATOM | 3429 | NH2 | ARG | B | 141 | 34.413 | 63.288 | 79.000 | 1.00 | 64.61 | N |
| ATOM | 3430 | N | VAL | B | 142 | 33.780 | 57.178 | 81.487 | 1.00 | 26.47 | N |
| ATOM | 3431 | CA | VAL | B | 142 | 34.849 | 56.979 | 82.446 | 1.00 | 26.47 | C |
| ATOM | 3432 | C | VAL | B | 142 | 35.674 | 55.763 | 82.130 | 1.00 | 26.47 | C |
| ATOM | 3433 | O | VAL | B | 142 | 36.879 | 55.859 | 81.961 | 1.00 | 26.47 | O |
| ATOM | 3434 | CB | VAL | B | 142 | 34.297 | 56.870 | 83.861 | 1.00 | 22.73 | C |
| ATOM | 3435 | CG1 | VAL | B | 142 | 35.361 | 56.356 | 84.811 | 1.00 | 22.73 | C |
| ATOM | 3436 | CG2 | VAL | B | 142 | 33.810 | 58.237 | 84.299 | 1.00 | 22.73 | C |
| ATOM | 3437 | N | ALA | B | 143 | 35.025 | 54.615 | 82.037 | 1.00 | 31.01 | N |
| ATOM | 3438 | CA | ALA | B | 143 | 35.735 | 53.383 | 81.724 | 1.00 | 31.01 | C |
| ATOM | 3439 | C | ALA | B | 143 | 36.558 | 53.527 | 80.443 | 1.00 | 31.01 | C |
| ATOM | 3440 | O | ALA | B | 143 | 37.628 | 52.933 | 80.319 | 1.00 | 31.01 | O |
| ATOM | 3441 | CB | ALA | B | 143 | 34.746 | 52.238 | 81.577 | 1.00 | 38.81 | C |
| ATOM | 3442 | N | ARG | B | 144 | 36.057 | 54.317 | 79.497 | 1.00 | 38.23 | N |
| ATOM | 3443 | CA | ARG | B | 144 | 36.745 | 54.513 | 78.229 | 1.00 | 38.23 | C |
| ATOM | 3444 | C | ARG | B | 144 | 38.046 | 55.270 | 78.416 | 1.00 | 38.23 | C |
| ATOM | 3445 | O | ARG | B | 144 | 39.026 | 55.003 | 77.724 | 1.00 | 38.23 | O |
| ATOM | 3446 | CB | ARG | B | 144 | 35.858 | 55.266 | 77.244 | 1.00 | 59.20 | C |
| ATOM | 3447 | CG | ARG | B | 144 | 36.376 | 55.247 | 75.821 | 1.00 | 59.20 | C |
| ATOM | 3448 | CD | ARG | B | 144 | 35.399 | 55.922 | 74.870 | 1.00 | 59.20 | C |
| ATOM | 3449 | NE | ARG | B | 144 | 35.901 | 55.933 | 73.500 | 1.00 | 59.20 | N |
| ATOM | 3450 | CZ | ARG | B | 144 | 35.200 | 56.351 | 72.444 | 1.00 | 59.20 | C |
| ATOM | 3451 | NH1 | ARG | B | 144 | 33.948 | 56.797 | 72.596 | 1.00 | 59.20 | N |
| ATOM | 3452 | NH2 | ARG | B | 144 | 35.748 | 56.330 | 71.230 | 1.00 | 59.20 | N |
| ATOM | 3453 | N | HIS | B | 145 | 38.067 | 56.210 | 79.351 | 1.00 | 38.97 | N |
| ATOM | 3454 | CA | HIS | B | 145 | 39.282 | 56.959 | 79.586 | 1.00 | 38.97 | C |
| ATOM | 3455 | C | HIS | B | 145 | 40.371 | 56.083 | 80.187 | 1.00 | 38.97 | C |
| ATOM | 3456 | O | HIS | B | 145 | 41.476 | 56.024 | 79.656 | 1.00 | 38.97 | O |
| ATOM | 3457 | CB | HIS | B | 145 | 39.005 | 58.163 | 80.486 | 1.00 | 93.08 | C |
| ATOM | 3458 | CG | HIS | B | 145 | 38.368 | 59.309 | 79.763 | 1.00 | 93.08 | C |
| ATOM | 3459 | ND1 | HIS | B | 145 | 37.378 | 60.091 | 80.327 | 1.00 | 93.08 | N |
| ATOM | 3460 | CD2 | HIS | B | 145 | 38.562 | 59.789 | 78.514 | 1.00 | 93.08 | C |
| ATOM | 3461 | CE1 | HIS | B | 145 | 36.987 | 61.000 | 79.450 | 1.00 | 93.08 | C |
| ATOM | 3462 | NE2 | HIS | B | 145 | 37.688 | 60.840 | 78.342 | 1.00 | 93.08 | N |
| ATOM | 3463 | N | TYR | B | 146 | 40.077 | 55.394 | 81.279 | 1.00 | 29.76 | N |
| ATOM | 3464 | CA | TYR | B | 146 | 41.094 | 54.555 | 81.877 | 1.00 | 29.76 | C |
| ATOM | 3465 | C | TYR | B | 146 | 41.565 | 53.547 | 80.849 | 1.00 | 29.76 | C |
| ATOM | 3466 | O | TYR | B | 146 | 42.743 | 53.185 | 80.798 | 1.00 | 29.76 | O |
| ATOM | 3467 | CB | TYR | B | 146 | 40.547 | 53.836 | 83.108 | 1.00 | 30.48 | C |
| ATOM | 3468 | CG | TYR | B | 146 | 40.420 | 54.737 | 84.309 | 1.00 | 30.48 | C |

| | | | | | | | | | | | |
|---|---|---|---|---|---|---|---|---|---|---|---|
| ATOM | 3469 | CD1 | TYR | B | 146 | 39.383 | 55.660 | 84.404 | 1.00 | 30.48 | C |
| ATOM | 3470 | CD2 | TYR | B | 146 | 41.371 | 54.706 | 85.328 | 1.00 | 30.48 | C |
| ATOM | 3471 | CE1 | TYR | B | 146 | 39.296 | 56.526 | 85.479 | 1.00 | 30.48 | C |
| ATOM | 3472 | CE2 | TYR | B | 146 | 41.292 | 55.572 | 86.408 | 1.00 | 30.48 | C |
| ATOM | 3473 | CZ | TYR | B | 146 | 40.252 | 56.477 | 86.473 | 1.00 | 30.48 | C |
| ATOM | 3474 | OH | TYR | B | 146 | 40.151 | 57.330 | 87.536 | 1.00 | 30.48 | O |
| ATOM | 3475 | N | SER | B | 147 | 40.635 | 53.117 | 80.008 | 1.00 | 35.64 | N |
| ATOM | 3476 | CA | SER | B | 147 | 40.938 | 52.135 | 78.990 | 1.00 | 35.64 | C |
| ATOM | 3477 | C | SER | B | 147 | 41.828 | 52.738 | 77.915 | 1.00 | 35.64 | C |
| ATOM | 3478 | O | SER | B | 147 | 42.930 | 52.256 | 77.651 | 1.00 | 35.64 | O |
| ATOM | 3479 | CB | SER | B | 147 | 39.640 | 51.625 | 78.378 | 1.00 | 47.74 | C |
| ATOM | 3480 | OG | SER | B | 147 | 39.859 | 50.415 | 77.678 | 1.00 | 47.74 | O |
| ATOM | 3481 | N | ARG | B | 148 | 41.342 | 53.802 | 77.293 | 1.00 | 49.06 | N |
| ATOM | 3482 | CA | ARG | B | 148 | 42.079 | 54.490 | 76.236 | 1.00 | 49.06 | C |
| ATOM | 3483 | C | ARG | B | 148 | 43.491 | 54.825 | 76.720 | 1.00 | 49.06 | C |
| ATOM | 3484 | O | ARG | B | 148 | 44.417 | 54.937 | 75.925 | 1.00 | 49.06 | O |
| ATOM | 3485 | CB | ARG | B | 148 | 41.338 | 55.778 | 75.861 | 1.00 | 58.59 | C |
| ATOM | 3486 | CG | ARG | B | 148 | 40.945 | 55.933 | 74.398 | 1.00 | 58.59 | C |
| ATOM | 3487 | CD | ARG | B | 148 | 41.941 | 56.839 | 73.676 | 0.00 | 58.59 | C |
| ATOM | 3488 | NE | ARG | B | 148 | 41.391 | 57.434 | 72.460 | 1.00 | 58.59 | N |
| ATOM | 3489 | CZ | ARG | B | 148 | 41.945 | 58.464 | 71.819 | 1.00 | 58.59 | C |
| ATOM | 3490 | NH1 | ARG | B | 148 | 43.071 | 59.023 | 72.271 | 1.00 | 58.59 | N |
| ATOM | 3491 | NH2 | ARG | B | 148 | 41.365 | 58.949 | 70.726 | 1.00 | 58.59 | N |
| ATOM | 3492 | N | ALA | B | 149 | 43.652 | 54.987 | 78.028 | 1.00 | 28.91 | N |
| ATOM | 3493 | CA | ALA | B | 149 | 44.958 | 55.312 | 78.600 | 1.00 | 28.91 | C |
| ATOM | 3494 | C | ALA | B | 149 | 45.665 | 54.067 | 79.175 | 1.00 | 28.91 | C |
| ATOM | 3495 | O | ALA | B | 149 | 46.592 | 54.170 | 79.980 | 1.00 | 28.91 | O |
| ATOM | 3496 | CB | ALA | B | 149 | 44.790 | 56.369 | 79.681 | 1.00 | 22.19 | C |
| ATOM | 3497 | N | LYS | B | 150 | 45.219 | 52.894 | 78.741 | 1.00 | 44.90 | N |
| ATOM | 3498 | CA | LYS | B | 150 | 45.775 | 51.631 | 79.210 | 1.00 | 44.90 | C |
| ATOM | 3499 | C | LYS | B | 150 | 46.014 | 51.669 | 80.714 | 1.00 | 44.90 | C |
| ATOM | 3500 | O | LYS | B | 150 | 47.122 | 51.437 | 81.185 | 1.00 | 44.90 | O |
| ATOM | 3501 | CB | LYS | B | 150 | 47.071 | 51.321 | 78.478 | 1.00 | 50.99 | C |
| ATOM | 3502 | N | GLN | B | 151 | 44.968 | 51.992 | 81.460 | 1.00 | 40.59 | N |
| ATOM | 3503 | CA | GLN | B | 151 | 45.037 | 52.041 | 82.912 | 1.00 | 40.59 | C |
| ATOM | 3504 | C | GLN | B | 151 | 43.744 | 51.424 | 83.443 | 1.00 | 40.59 | C |
| ATOM | 3505 | O | GLN | B | 151 | 42.708 | 51.427 | 82.763 | 1.00 | 40.59 | O |
| ATOM | 3506 | CB | GLN | B | 151 | 45.188 | 53.483 | 83.414 | 1.00 | 25.05 | C |
| ATOM | 3507 | CG | GLN | B | 151 | 46.526 | 54.112 | 83.094 | 0.00 | 25.05 | C |
| ATOM | 3508 | CD | GLN | B | 151 | 46.679 | 55.475 | 83.728 | 1.00 | 25.05 | C |
| ATOM | 3509 | OE1 | GLN | B | 151 | 46.615 | 55.616 | 84.948 | 0.00 | 25.05 | O |
| ATOM | 3510 | NE2 | GLN | B | 151 | 46.884 | 56.493 | 82.899 | 0.00 | 25.05 | N |
| ATOM | 3511 | N | THR | B | 152 | 43.808 | 50.890 | 84.658 | 1.00 | 54.76 | N |
| ATOM | 3512 | CA | THR | B | 152 | 42.652 | 50.246 | 85.267 | 1.00 | 54.76 | C |
| ATOM | 3513 | C | THR | B | 152 | 41.882 | 51.155 | 86.220 | 1.00 | 54.76 | C |
| ATOM | 3514 | O | THR | B | 152 | 42.475 | 51.883 | 87.009 | 1.00 | 54.76 | O |
| ATOM | 3515 | CB | THR | B | 152 | 43.085 | 48.981 | 86.053 | 1.00 | 65.66 | C |
| ATOM | 3516 | OG1 | THR | B | 152 | 42.402 | 48.953 | 87.317 | 1.00 | 65.66 | O |
| ATOM | 3517 | CG2 | THR | B | 152 | 44.620 | 48.972 | 86.279 | 1.00 | 65.66 | C |
| ATOM | 3518 | N | LEU | B | 153 | 40.561 | 51.119 | 86.147 | 1.00 | 34.37 | N |
| ATOM | 3519 | CA | LEU | B | 153 | 39.768 | 51.922 | 87.058 | 1.00 | 34.37 | C |
| ATOM | 3520 | C | LEU | B | 153 | 39.990 | 51.351 | 88.471 | 1.00 | 34.37 | C |
| ATOM | 3521 | O | LEU | B | 153 | 39.784 | 50.162 | 88.704 | 1.00 | 34.37 | O |
| ATOM | 3522 | CB | LEU | B | 153 | 38.290 | 51.831 | 86.684 | 1.00 | 26.23 | C |
| ATOM | 3523 | CG | LEU | B | 153 | 37.313 | 52.522 | 87.646 | 1.00 | 26.23 | C |
| ATOM | 3524 | CD1 | LEU | B | 153 | 37.367 | 54.022 | 87.459 | 1.00 | 26.23 | C |
| ATOM | 3525 | CD2 | LEU | B | 153 | 35.903 | 52.005 | 87.397 | 1.00 | 26.23 | C |
| ATOM | 3526 | N | PRO | B | 154 | 40.422 | 52.188 | 89.427 | 1.00 | 27.76 | N |
| ATOM | 3527 | CA | PRO | B | 154 | 40.655 | 51.719 | 90.801 | 1.00 | 27.76 | C |
| ATOM | 3528 | C | PRO | B | 154 | 39.451 | 50.948 | 91.340 | 1.00 | 27.76 | C |
| ATOM | 3529 | O | PRO | B | 154 | 38.328 | 51.424 | 91.253 | 1.00 | 27.76 | O |
| ATOM | 3530 | CB | PRO | B | 154 | 40.904 | 53.012 | 91.561 | 1.00 | 28.15 | C |

| | | | | | | | | | | | |
|---|---|---|---|---|---|---|---|---|---|---|---|
| ATOM | 3531 | CG | PRO | B | 154 | 41.580 | 53.865 | 90.533 | 1.00 | 28.15 | C |
| ATOM | 3532 | CD | PRO | B | 154 | 40.747 | 53.618 | 89.296 | 1.00 | 28.15 | C |
| ATOM | 3533 | N | VAL | B | 155 | 39.686 | 49.767 | 91.908 | 1.00 | 29.65 | N |
| ATOM | 3534 | CA | VAL | B | 155 | 38.592 | 48.941 | 92.422 | 1.00 | 29.65 | C |
| ATOM | 3535 | C | VAL | B | 155 | 37.692 | 49.635 | 93.444 | 1.00 | 29.65 | C |
| ATOM | 3536 | O | VAL | B | 155 | 36.622 | 49.141 | 93.778 | 1.00 | 29.65 | O |
| ATOM | 3537 | CB | VAL | B | 155 | 39.114 | 47.597 | 93.040 | 1.00 | 35.58 | C |
| ATOM | 3538 | CG1 | VAL | B | 155 | 40.230 | 47.036 | 92.186 | 1.00 | 35.58 | C |
| ATOM | 3539 | CG2 | VAL | B | 155 | 39.579 | 47.794 | 94.471 | 1.00 | 35.58 | C |
| ATOM | 3540 | N | ILE | B | 156 | 38.114 | 50.781 | 93.949 | 1.00 | 23.25 | N |
| ATOM | 3541 | CA | ILE | B | 156 | 37.285 | 51.459 | 94.924 | 1.00 | 23.25 | C |
| ATOM | 3542 | C | ILE | B | 156 | 36.057 | 51.982 | 94.189 | 1.00 | 23.25 | C |
| ATOM | 3543 | O | ILE | B | 156 | 34.948 | 51.947 | 94.718 | 1.00 | 23.25 | O |
| ATOM | 3544 | CB | ILE | B | 156 | 38.069 | 52.604 | 95.641 | 1.00 | 22.07 | C |
| ATOM | 3545 | CG1 | ILE | B | 156 | 37.215 | 53.214 | 96.749 | 1.00 | 22.07 | C |
| ATOM | 3546 | CG2 | ILE | B | 156 | 38.496 | 53.654 | 94.652 | 1.00 | 22.07 | C |
| ATOM | 3547 | CD1 | ILE | B | 156 | 37.044 | 52.330 | 97.950 | 1.00 | 22.07 | C |
| ATOM | 3548 | N | TYR | B | 157 | 36.258 | 52.437 | 92.956 | 1.00 | 25.85 | N |
| ATOM | 3549 | CA | TYR | B | 157 | 35.162 | 52.954 | 92.136 | 1.00 | 25.85 | C |
| ATOM | 3550 | C | TYR | B | 157 | 34.320 | 51.817 | 91.593 | 1.00 | 25.85 | C |
| ATOM | 3551 | O | TYR | B | 157 | 33.103 | 51.932 | 91.472 | 1.00 | 25.85 | O |
| ATOM | 3552 | CB | TYR | B | 157 | 35.701 | 53.774 | 90.972 | 1.00 | 35.67 | C |
| ATOM | 3553 | CG | TYR | B | 157 | 36.420 | 55.017 | 91.416 | 1.00 | 35.67 | C |
| ATOM | 3554 | CD1 | TYR | B | 157 | 35.786 | 55.960 | 92.227 | 1.00 | 35.67 | C |
| ATOM | 3555 | CD2 | TYR | B | 157 | 37.737 | 55.259 | 91.027 | 1.00 | 35.67 | C |
| ATOM | 3556 | CE1 | TYR | B | 157 | 36.450 | 57.114 | 92.638 | 1.00 | 35.67 | C |
| ATOM | 3557 | CE2 | TYR | B | 157 | 38.409 | 56.410 | 91.429 | 1.00 | 35.67 | C |
| ATOM | 3558 | CZ | TYR | B | 157 | 37.763 | 57.331 | 92.232 | 1.00 | 35.67 | C |
| ATOM | 3559 | OH | TYR | B | 157 | 38.430 | 58.468 | 92.617 | 1.00 | 35.67 | O |
| ATOM | 3560 | N | VAL | B | 158 | 34.990 | 50.726 | 91.250 | 1.00 | 27.71 | N |
| ATOM | 3561 | CA | VAL | B | 158 | 34.309 | 49.552 | 90.747 | 1.00 | 27.71 | C |
| ATOM | 3562 | C | VAL | B | 158 | 33.366 | 49.086 | 91.853 | 1.00 | 27.71 | C |
| ATOM | 3563 | O | VAL | B | 158 | 32.209 | 48.772 | 91.587 | 1.00 | 27.71 | O |
| ATOM | 3564 | CB | VAL | B | 158 | 35.313 | 48.443 | 90.399 | 1.00 | 22.20 | C |
| ATOM | 3565 | CG1 | VAL | B | 158 | 34.598 | 47.253 | 89.789 | 1.00 | 22.20 | C |
| ATOM | 3566 | CG2 | VAL | B | 158 | 36.349 | 48.982 | 89.455 | 1.00 | 22.20 | C |
| ATOM | 3567 | N | LYS | B | 159 | 33.857 | 49.061 | 93.091 | 1.00 | 26.79 | N |
| ATOM | 3568 | CA | LYS | B | 159 | 33.034 | 48.666 | 94.235 | 1.00 | 26.79 | C |
| ATOM | 3569 | C | LYS | B | 159 | 31.851 | 49.633 | 94.412 | 1.00 | 26.79 | C |
| ATOM | 3570 | O | LYS | B | 159 | 30.701 | 49.221 | 94.549 | 1.00 | 26.79 | O |
| ATOM | 3571 | CB | LYS | B | 159 | 33.855 | 48.693 | 95.525 | 1.00 | 27.27 | C |
| ATOM | 3572 | CG | LYS | B | 159 | 34.990 | 47.699 | 95.606 | 1.00 | 27.27 | C |
| ATOM | 3573 | CD | LYS | B | 159 | 35.776 | 47.902 | 96.893 | 1.00 | 27.27 | C |
| ATOM | 3574 | CE | LYS | B | 159 | 36.910 | 46.911 | 97.023 | 1.00 | 27.27 | C |
| ATOM | 3575 | NZ | LYS | B | 159 | 36.435 | 45.519 | 96.912 | 1.00 | 27.27 | N |
| ATOM | 3576 | N | LEU | B | 160 | 32.162 | 50.925 | 94.413 | 1.00 | 30.06 | N |
| ATOM | 3577 | CA | LEU | B | 160 | 31.175 | 51.978 | 94.590 | 1.00 | 30.06 | C |
| ATOM | 3578 | C | LEU | B | 160 | 30.112 | 52.009 | 93.508 | 1.00 | 30.06 | C |
| ATOM | 3579 | O | LEU | B | 160 | 28.921 | 52.104 | 93.796 | 1.00 | 30.06 | O |
| ATOM | 3580 | CB | LEU | B | 160 | 31.876 | 53.335 | 94.638 | 1.00 | 32.79 | C |
| ATOM | 3581 | CG | LEU | B | 160 | 31.337 | 54.303 | 95.684 | 1.00 | 32.79 | C |
| ATOM | 3582 | CD1 | LEU | B | 160 | 31.496 | 53.698 | 97.063 | 1.00 | 32.79 | C |
| ATOM | 3583 | CD2 | LEU | B | 160 | 32.084 | 55.618 | 95.599 | 0.00 | 32.79 | C |
| ATOM | 3584 | N | TYR | B | 161 | 30.542 | 51.941 | 92.256 | 1.00 | 21.41 | N |
| ATOM | 3585 | CA | TYR | B | 161 | 29.609 | 51.969 | 91.145 | 1.00 | 21.41 | C |
| ATOM | 3586 | C | TYR | B | 161 | 28.720 | 50.726 | 91.082 | 1.00 | 21.41 | C |
| ATOM | 3587 | O | TYR | B | 161 | 27.514 | 50.830 | 90.887 | 1.00 | 21.41 | O |
| ATOM | 3588 | CB | TYR | B | 161 | 30.379 | 52.131 | 89.841 | 1.00 | 29.26 | C |
| ATOM | 3589 | CG | TYR | B | 161 | 31.181 | 53.410 | 89.748 | 1.00 | 29.26 | C |
| ATOM | 3590 | CD1 | TYR | B | 161 | 31.170 | 54.344 | 90.784 | 1.00 | 29.26 | C |
| ATOM | 3591 | CD2 | TYR | B | 161 | 31.893 | 53.719 | 88.589 | 1.00 | 29.26 | C |
| ATOM | 3592 | CE1 | TYR | B | 161 | 31.840 | 55.559 | 90.667 | 1.00 | 29.26 | C |

| | | | | | | | | | | | |
|---|---|---|---|---|---|---|---|---|---|---|---|
| ATOM | 3593 | CE2 | TYR | B | 161 | 32.566 | 54.927 | 88.455 | 1.00 | 29.26 | C |
| ATOM | 3594 | CZ | TYR | B | 161 | 32.531 | 55.847 | 89.493 | 1.00 | 29.26 | C |
| ATOM | 3595 | OH | TYR | B | 161 | 33.136 | 57.073 | 89.336 | 1.00 | 29.26 | O |
| ATOM | 3596 | N | MET | B | 162 | 29.310 | 49.548 | 91.256 | 1.00 | 24.74 | N |
| ATOM | 3597 | CA | MET | B | 162 | 28.533 | 48.313 | 91.206 | 1.00 | 24.74 | C |
| ATOM | 3598 | C | MET | B | 162 | 27.537 | 48.190 | 92.347 | 1.00 | 24.74 | C |
| ATOM | 3599 | O | MET | B | 162 | 26.437 | 47.667 | 92.180 | 1.00 | 24.74 | O |
| ATOM | 3600 | CB | MET | B | 162 | 29.455 | 47.093 | 91.204 | 1.00 | 22.38 | C |
| ATOM | 3601 | CG | MET | B | 162 | 30.317 | 46.965 | 89.954 | 1.00 | 22.38 | C |
| ATOM | 3602 | SD | MET | B | 162 | 29.387 | 47.058 | 88.413 | 1.00 | 22.38 | S |
| ATOM | 3603 | CE | MET | B | 162 | 28.237 | 45.713 | 88.638 | 1.00 | 22.38 | C |
| ATOM | 3604 | N | TYR | B | 163 | 27.929 | 48.676 | 93.512 | 1.00 | 26.83 | N |
| ATOM | 3605 | CA | TYR | B | 163 | 27.071 | 48.622 | 94.686 | 1.00 | 26.83 | C |
| ATOM | 3606 | C | TYR | B | 163 | 25.801 | 49.444 | 94.468 | 1.00 | 26.83 | C |
| ATOM | 3607 | O | TYR | B | 163 | 24.705 | 48.988 | 94.775 | 1.00 | 26.83 | O |
| ATOM | 3608 | CB | TYR | B | 163 | 27.835 | 49.144 | 95.911 | 1.00 | 21.69 | C |
| ATOM | 3609 | CG | TYR | B | 163 | 27.015 | 49.204 | 97.176 | 1.00 | 21.69 | C |
| ATOM | 3610 | CD1 | TYR | B | 163 | 26.822 | 48.065 | 97.961 | 1.00 | 21.69 | C |
| ATOM | 3611 | CD2 | TYR | B | 163 | 26.420 | 50.398 | 97.581 | 1.00 | 21.69 | C |
| ATOM | 3612 | CE1 | TYR | B | 163 | 26.062 | 48.120 | 99.109 | 1.00 | 21.69 | C |
| ATOM | 3613 | CE2 | TYR | B | 163 | 25.654 | 50.459 | 98.726 | 1.00 | 21.69 | C |
| ATOM | 3614 | CZ | TYR | B | 163 | 25.481 | 49.318 | 99.486 | 1.00 | 21.69 | C |
| ATOM | 3615 | OH | TYR | B | 163 | 24.731 | 49.374 | 100.633 | 1.00 | 21.69 | O |
| ATOM | 3616 | N | GLN | B | 164 | 25.968 | 50.655 | 93.937 | 1.00 | 28.12 | N |
| ATOM | 3617 | CA | GLN | B | 164 | 24.852 | 51.557 | 93.677 | 1.00 | 28.12 | C |
| ATOM | 3618 | C | GLN | B | 164 | 23.958 | 51.051 | 92.534 | 1.00 | 28.12 | C |
| ATOM | 3619 | O | GLN | B | 164 | 22.751 | 51.302 | 92.510 | 1.00 | 28.12 | O |
| ATOM | 3620 | CB | GLN | B | 164 | 25.392 | 52.964 | 93.386 | 1.00 | 24.38 | C |
| ATOM | 3621 | CG | GLN | B | 164 | 26.209 | 53.544 | 94.547 | 1.00 | 24.38 | C |
| ATOM | 3622 | CD | GLN | B | 164 | 26.797 | 54.908 | 94.247 | 1.00 | 24.38 | C |
| ATOM | 3623 | OE1 | GLN | B | 164 | 26.083 | 55.907 | 94.165 | 1.00 | 24.38 | O |
| ATOM | 3624 | NE2 | GLN | B | 164 | 28.109 | 54.956 | 94.073 | 1.00 | 24.38 | N |
| ATOM | 3625 | N | LEU | B | 165 | 24.549 | 50.328 | 91.588 | 1.00 | 34.87 | N |
| ATOM | 3626 | CA | LEU | B | 165 | 23.778 | 49.779 | 90.489 | 1.00 | 34.87 | C |
| ATOM | 3627 | C | LEU | B | 165 | 22.925 | 48.679 | 91.085 | 1.00 | 34.87 | C |
| ATOM | 3628 | O | LEU | B | 165 | 21.728 | 48.648 | 90.864 | 1.00 | 34.87 | O |
| ATOM | 3629 | CB | LEU | B | 165 | 24.682 | 49.171 | 89.418 | 1.00 | 12.92 | C |
| ATOM | 3630 | CG | LEU | B | 165 | 24.102 | 48.943 | 88.016 | 1.00 | 12.92 | C |
| ATOM | 3631 | CD1 | LEU | B | 165 | 24.787 | 47.754 | 87.426 | 1.00 | 12.92 | C |
| ATOM | 3632 | CD2 | LEU | B | 165 | 22.617 | 48.721 | 88.038 | 1.00 | 12.92 | C |
| ATOM | 3633 | N | PHE | B | 166 | 23.547 | 47.776 | 91.836 | 1.00 | 24.28 | N |
| ATOM | 3634 | CA | PHE | B | 166 | 22.813 | 46.681 | 92.452 | 1.00 | 24.28 | C |
| ATOM | 3635 | C | PHE | B | 166 | 21.706 | 47.181 | 93.373 | 1.00 | 24.28 | C |
| ATOM | 3636 | O | PHE | B | 166 | 20.681 | 46.517 | 93.539 | 1.00 | 24.28 | O |
| ATOM | 3637 | CB | PHE | B | 166 | 23.759 | 45.754 | 93.226 | 1.00 | 22.27 | C |
| ATOM | 3638 | CG | PHE | B | 166 | 24.545 | 44.821 | 92.351 | 1.00 | 22.27 | C |
| ATOM | 3639 | CD1 | PHE | B | 166 | 23.905 | 44.025 | 91.399 | 1.00 | 22.27 | C |
| ATOM | 3640 | CD2 | PHE | B | 166 | 25.921 | 44.741 | 92.462 | 1.00 | 22.27 | C |
| ATOM | 3641 | CE1 | PHE | B | 166 | 24.633 | 43.170 | 90.571 | 1.00 | 22.27 | C |
| ATOM | 3642 | CE2 | PHE | B | 166 | 26.650 | 43.887 | 91.639 | 1.00 | 22.27 | C |
| ATOM | 3643 | CZ | PHE | B | 166 | 26.006 | 43.105 | 90.694 | 1.00 | 22.27 | C |
| ATOM | 3644 | N | ARG | B | 167 | 21.914 | 48.348 | 93.974 | 1.00 | 27.11 | N |
| ATOM | 3645 | CA | ARG | B | 167 | 20.915 | 48.935 | 94.856 | 1.00 | 27.11 | C |
| ATOM | 3646 | C | ARG | B | 167 | 19.756 | 49.370 | 93.991 | 1.00 | 27.11 | C |
| ATOM | 3647 | O | ARG | B | 167 | 18.624 | 49.001 | 94.238 | 1.00 | 27.11 | O |
| ATOM | 3648 | CB | ARG | B | 167 | 21.478 | 50.147 | 95.600 | 1.00 | 28.07 | C |
| ATOM | 3649 | CG | ARG | B | 167 | 22.148 | 49.821 | 96.922 | 1.00 | 28.07 | C |
| ATOM | 3650 | CD | ARG | B | 167 | 21.328 | 50.358 | 98.067 | 1.00 | 28.07 | C |
| ATOM | 3651 | NE | ARG | B | 167 | 21.631 | 51.755 | 98.350 | 1.00 | 28.07 | N |
| ATOM | 3652 | CZ | ARG | B | 167 | 20.879 | 52.543 | 99.109 | 1.00 | 28.07 | C |
| ATOM | 3653 | NH1 | ARG | B | 167 | 19.769 | 52.077 | 99.657 | 1.00 | 28.07 | N |
| ATOM | 3654 | NH2 | ARG | B | 167 | 21.251 | 53.790 | 99.334 | 1.00 | 28.07 | N |

| | | | | | | | | | | | |
|---|---|---|---|---|---|---|---|---|---|---|---|
| ATOM | 3655 | N | SER | B | 168 | 20.040 | 50.148 | 92.960 | 1.00 | 27.02 | N |
| ATOM | 3656 | CA | SER | B | 168 | 18.986 | 50.605 | 92.080 | 1.00 | 27.02 | C |
| ATOM | 3657 | C | SER | B | 168 | 18.205 | 49.411 | 91.559 | 1.00 | 27.02 | C |
| ATOM | 3658 | O | SER | B | 168 | 16.997 | 49.468 | 91.410 | 1.00 | 27.02 | O |
| ATOM | 3659 | CB | SER | B | 168 | 19.576 | 51.399 | 90.911 | 1.00 | 22.20 | C |
| ATOM | 3660 | OG | SER | B | 168 | 20.303 | 50.565 | 90.031 | 1.00 | 22.20 | O |
| ATOM | 3661 | N | LEU | B | 169 | 18.905 | 48.322 | 91.297 | 1.00 | 21.22 | N |
| ATOM | 3662 | CA | LEU | B | 169 | 18.277 | 47.118 | 90.778 | 1.00 | 21.22 | C |
| ATOM | 3663 | C | LEU | B | 169 | 17.384 | 46.401 | 91.782 | 1.00 | 21.22 | C |
| ATOM | 3664 | O | LEU | B | 169 | 16.311 | 45.929 | 91.428 | 1.00 | 21.22 | O |
| ATOM | 3665 | CB | LEU | B | 169 | 19.351 | 46.173 | 90.235 | 1.00 | 15.10 | C |
| ATOM | 3666 | CG | LEU | B | 169 | 19.509 | 46.105 | 88.707 | 1.00 | 15.10 | C |
| ATOM | 3667 | CD1 | LEU | B | 169 | 19.167 | 47.413 | 88.024 | 1.00 | 15.10 | C |
| ATOM | 3668 | CD2 | LEU | B | 169 | 20.918 | 45.683 | 88.413 | 1.00 | 15.10 | C |
| ATOM | 3669 | N | ALA | B | 170 | 17.826 | 46.323 | 93.033 | 1.00 | 28.26 | N |
| ATOM | 3670 | CA | ALA | B | 170 | 17.038 | 45.688 | 94.091 | 1.00 | 28.26 | C |
| ATOM | 3671 | C | ALA | B | 170 | 15.771 | 46.520 | 94.246 | 1.00 | 28.26 | C |
| ATOM | 3672 | O | ALA | B | 170 | 14.670 | 46.003 | 94.456 | 1.00 | 28.26 | O |
| ATOM | 3673 | CB | ALA | B | 170 | 17.824 | 45.672 | 95.398 | 1.00 | 16.51 | C |
| ATOM | 3674 | N | TYR | B | 171 | 15.945 | 47.824 | 94.116 | 1.00 | 22.86 | N |
| ATOM | 3675 | CA | TYR | B | 171 | 14.842 | 48.743 | 94.210 | 1.00 | 22.86 | C |
| ATOM | 3676 | C | TYR | B | 171 | 13.790 | 48.449 | 93.154 | 1.00 | 22.86 | C |
| ATOM | 3677 | O | TYR | B | 171 | 12.745 | 47.903 | 93.461 | 1.00 | 22.86 | O |
| ATOM | 3678 | CB | TYR | B | 171 | 15.321 | 50.173 | 94.042 | 1.00 | 18.75 | C |
| ATOM | 3679 | CG | TYR | B | 171 | 14.200 | 51.148 | 94.194 | 1.00 | 18.75 | C |
| ATOM | 3680 | CD1 | TYR | B | 171 | 13.580 | 51.319 | 95.421 | 1.00 | 18.75 | C |
| ATOM | 3681 | CD2 | TYR | B | 171 | 13.718 | 51.871 | 93.105 | 1.00 | 18.75 | C |
| ATOM | 3682 | CE1 | TYR | B | 171 | 12.516 | 52.178 | 95.569 | 1.00 | 18.75 | C |
| ATOM | 3683 | CE2 | TYR | B | 171 | 12.646 | 52.739 | 93.243 | 1.00 | 18.75 | C |
| ATOM | 3684 | CZ | TYR | B | 171 | 12.052 | 52.882 | 94.486 | 1.00 | 18.75 | C |
| ATOM | 3685 | OH | TYR | B | 171 | 10.995 | 53.731 | 94.658 | 1.00 | 18.75 | O |
| ATOM | 3686 | N | ILE | B | 172 | 14.061 | 48.793 | 91.904 | 1.00 | 28.69 | N |
| ATOM | 3687 | CA | ILE | B | 172 | 13.075 | 48.566 | 90.861 | 1.00 | 28.69 | C |
| ATOM | 3688 | C | ILE | B | 172 | 12.568 | 47.126 | 90.801 | 1.00 | 28.69 | C |
| ATOM | 3689 | O | ILE | B | 172 | 11.412 | 46.894 | 90.485 | 1.00 | 28.69 | O |
| ATOM | 3690 | CB | ILE | B | 172 | 13.595 | 48.997 | 89.456 | 1.00 | 14.84 | C |
| ATOM | 3691 | CG1 | ILE | B | 172 | 14.803 | 48.148 | 89.045 | 1.00 | 14.84 | C |
| ATOM | 3692 | CG2 | ILE | B | 172 | 13.919 | 50.485 | 89.461 | 1.00 | 14.84 | C |
| ATOM | 3693 | CD1 | ILE | B | 172 | 15.027 | 48.099 | 87.559 | 1.00 | 14.84 | C |
| ATOM | 3694 | N | HIS | B | 173 | 13.403 | 46.149 | 91.112 | 1.00 | 27.82 | N |
| ATOM | 3695 | CA | HIS | B | 173 | 12.903 | 44.789 | 91.065 | 1.00 | 27.82 | C |
| ATOM | 3696 | C | HIS | B | 173 | 11.809 | 44.525 | 92.105 | 1.00 | 27.82 | C |
| ATOM | 3697 | O | HIS | B | 173 | 10.848 | 43.793 | 91.836 | 1.00 | 27.82 | O |
| ATOM | 3698 | CB | HIS | B | 173 | 14.037 | 43.783 | 91.217 | 1.00 | 17.84 | C |
| ATOM | 3699 | CG | HIS | B | 173 | 14.879 | 43.653 | 89.990 | 1.00 | 17.84 | C |
| ATOM | 3700 | ND1 | HIS | B | 173 | 15.771 | 42.623 | 89.804 | 1.00 | 17.84 | N |
| ATOM | 3701 | CD2 | HIS | B | 173 | 15.003 | 44.457 | 88.908 | 1.00 | 17.84 | C |
| ATOM | 3702 | CE1 | HIS | B | 173 | 16.413 | 42.800 | 88.665 | 1.00 | 17.84 | C |
| ATOM | 3703 | NE2 | HIS | B | 173 | 15.966 | 43.907 | 88.102 | 1.00 | 17.84 | N |
| ATOM | 3704 | N | SER | B | 174 | 11.939 | 45.129 | 93.281 | 1.00 | 29.40 | N |
| ATOM | 3705 | CA | SER | B | 174 | 10.944 | 44.930 | 94.315 | 1.00 | 29.40 | C |
| ATOM | 3706 | C | SER | B | 174 | 9.554 | 45.291 | 93.799 | 1.00 | 29.40 | C |
| ATOM | 3707 | O | SER | B | 174 | 8.560 | 44.851 | 94.361 | 1.00 | 29.40 | O |
| ATOM | 3708 | CB | SER | B | 174 | 11.281 | 45.760 | 95.549 | 1.00 | 29.34 | C |
| ATOM | 3709 | OG | SER | B | 174 | 10.922 | 47.113 | 95.369 | 1.00 | 29.34 | O |
| ATOM | 3710 | N | PHE | B | 175 | 9.472 | 46.077 | 92.731 | 1.00 | 30.15 | N |
| ATOM | 3711 | CA | PHE | B | 175 | 8.168 | 46.439 | 92.176 | 1.00 | 30.15 | C |
| ATOM | 3712 | C | PHE | B | 175 | 7.823 | 45.578 | 90.974 | 1.00 | 30.15 | C |
| ATOM | 3713 | O | PHE | B | 175 | 6.802 | 45.804 | 90.317 | 1.00 | 30.15 | O |
| ATOM | 3714 | CB | PHE | B | 175 | 8.134 | 47.895 | 91.724 | 1.00 | 53.96 | C |
| ATOM | 3715 | CG | PHE | B | 175 | 8.375 | 48.866 | 92.816 | 1.00 | 53.96 | C |
| ATOM | 3716 | CD1 | PHE | B | 175 | 9.662 | 49.116 | 93.260 | 1.00 | 53.96 | C |

| | | | | | | | | | | |
|---|---|---|---|---|---|---|---|---|---|---|
| ATOM | 3717 | CD2 | PHE | B | 175 | 7.306 | 49.502 | 93.440 | 1.00 | 53.96 | C |
| ATOM | 3718 | CE1 | PHE | B | 175 | 9.888 | 49.990 | 94.320 | 1.00 | 53.96 | C |
| ATOM | 3719 | CE2 | PHE | B | 175 | 7.510 | 50.376 | 94.500 | 1.00 | 53.96 | C |
| ATOM | 3720 | CZ | PHE | B | 175 | 8.807 | 50.622 | 94.945 | 1.00 | 53.96 | C |
| ATOM | 3721 | N | GLY | B | 176 | 8.684 | 44.608 | 90.675 | 1.00 | 31.38 | N |
| ATOM | 3722 | CA | GLY | B | 176 | 8.449 | 43.746 | 89.534 | 1.00 | 31.38 | C |
| ATOM | 3723 | C | GLY | B | 176 | 8.853 | 44.396 | 88.222 | 1.00 | 31.38 | C |
| ATOM | 3724 | O | GLY | B | 176 | 8.416 | 43.972 | 87.155 | 1.00 | 31.38 | O |
| ATOM | 3725 | N | ILE | B | 177 | 9.688 | 45.426 | 88.303 | 1.00 | 38.54 | N |
| ATOM | 3726 | CA | ILE | B | 177 | 10.161 | 46.138 | 87.123 | 1.00 | 38.54 | C |
| ATOM | 3727 | C | ILE | B | 177 | 11.560 | 45.683 | 86.686 | 1.00 | 38.54 | C |
| ATOM | 3728 | O | ILE | B | 177 | 12.450 | 45.482 | 87.512 | 1.00 | 38.54 | O |
| ATOM | 3729 | CB | ILE | B | 177 | 10.212 | 47.655 | 87.381 | 1.00 | 27.99 | C |
| ATOM | 3730 | CG1 | ILE | B | 177 | 8.805 | 48.179 | 87.678 | 1.00 | 27.99 | C |
| ATOM | 3731 | CG2 | ILE | B | 177 | 10.819 | 48.365 | 86.185 | 1.00 | 27.99 | C |
| ATOM | 3732 | CD1 | ILE | B | 177 | 8.761 | 49.658 | 88.047 | 1.00 | 27.99 | C |
| ATOM | 3733 | N | CYS | B | 178 | 11.750 | 45.514 | 85.385 | 1.00 | 26.93 | N |
| ATOM | 3734 | CA | CYS | B | 178 | 13.045 | 45.115 | 84.870 | 1.00 | 26.93 | C |
| ATOM | 3735 | C | CYS | B | 178 | 13.464 | 46.220 | 83.916 | 1.00 | 26.93 | C |
| ATOM | 3736 | O | CYS | B | 178 | 12.712 | 46.583 | 83.023 | 1.00 | 26.93 | O |
| ATOM | 3737 | CB | CYS | B | 178 | 12.936 | 43.779 | 84.132 | 1.00 | 26.16 | C |
| ATOM | 3738 | SG | CYS | B | 178 | 14.492 | 43.108 | 83.516 | 1.00 | 26.16 | S |
| ATOM | 3739 | N | HIS | B | 179 | 14.658 | 46.763 | 84.119 | 1.00 | 20.61 | N |
| ATOM | 3740 | CA | HIS | B | 179 | 15.167 | 47.835 | 83.275 | 1.00 | 20.61 | C |
| ATOM | 3741 | C | HIS | B | 179 | 15.328 | 47.360 | 81.828 | 1.00 | 20.61 | C |
| ATOM | 3742 | O | HIS | B | 179 | 14.998 | 48.076 | 80.895 | 1.00 | 20.61 | O |
| ATOM | 3743 | CB | HIS | B | 179 | 16.505 | 48.334 | 83.845 | 1.00 | 18.60 | C |
| ATOM | 3744 | CG | HIS | B | 179 | 17.093 | 49.493 | 83.104 | 1.00 | 18.60 | C |
| ATOM | 3745 | ND1 | HIS | B | 179 | 17.723 | 49.358 | 81.886 | 1.00 | 18.60 | N |
| ATOM | 3746 | CD2 | HIS | B | 179 | 17.136 | 50.812 | 83.407 | 1.00 | 18.60 | C |
| ATOM | 3747 | CE1 | HIS | B | 179 | 18.128 | 50.544 | 81.471 | 1.00 | 18.60 | C |
| ATOM | 3748 | NE2 | HIS | B | 179 | 17.784 | 51.443 | 82.376 | 1.00 | 18.60 | N |
| ATOM | 3749 | N | ARG | B | 180 | 15.835 | 46.140 | 81.676 | 1.00 | 26.80 | N |
| ATOM | 3750 | CA | ARG | B | 180 | 16.063 | 45.500 | 80.378 | 1.00 | 26.80 | C |
| ATOM | 3751 | C | ARG | B | 180 | 17.120 | 46.105 | 79.447 | 1.00 | 26.80 | C |
| ATOM | 3752 | O | ARG | B | 180 | 17.208 | 45.710 | 78.288 | 1.00 | 26.80 | O |
| ATOM | 3753 | CB | ARG | B | 180 | 14.759 | 45.413 | 79.602 | 1.00 | 22.91 | C |
| ATOM | 3754 | CG | ARG | B | 180 | 13.746 | 44.443 | 80.141 | 1.00 | 22.91 | C |
| ATOM | 3755 | CD | ARG | B | 180 | 12.416 | 44.906 | 79.668 | 1.00 | 22.91 | C |
| ATOM | 3756 | NE | ARG | B | 180 | 11.722 | 43.922 | 78.868 | 1.00 | 22.91 | N |
| ATOM | 3757 | CZ | ARG | B | 180 | 10.768 | 44.224 | 77.999 | 1.00 | 22.91 | C |
| ATOM | 3758 | NH1 | ARG | B | 180 | 10.403 | 45.485 | 77.808 | 1.00 | 22.91 | N |
| ATOM | 3759 | NH2 | ARG | B | 180 | 10.159 | 43.255 | 77.339 | 1.00 | 22.91 | N |
| ATOM | 3760 | N | ASP | B | 181 | 17.911 | 47.055 | 79.934 | 1.00 | 22.02 | N |
| ATOM | 3761 | CA | ASP | B | 181 | 18.939 | 47.666 | 79.103 | 1.00 | 22.02 | C |
| ATOM | 3762 | C | ASP | B | 181 | 20.011 | 48.256 | 80.021 | 1.00 | 22.02 | C |
| ATOM | 3763 | O | ASP | B | 181 | 20.261 | 49.455 | 80.038 | 1.00 | 22.02 | O |
| ATOM | 3764 | CB | ASP | B | 181 | 18.316 | 48.732 | 78.194 | 1.00 | 28.37 | C |
| ATOM | 3765 | CG | ASP | B | 181 | 19.271 | 49.206 | 77.106 | 1.00 | 28.37 | C |
| ATOM | 3766 | OD1 | ASP | B | 181 | 19.981 | 48.352 | 76.522 | 1.00 | 28.37 | O |
| ATOM | 3767 | OD2 | ASP | B | 181 | 19.300 | 50.427 | 76.835 | 1.00 | 28.37 | O |
| ATOM | 3768 | N | ILE | B | 182 | 20.662 | 47.324 | 80.738 | 1.00 | 24.27 | N |
| ATOM | 3769 | CA | ILE | B | 182 | 21.658 | 47.779 | 81.688 | 1.00 | 24.27 | C |
| ATOM | 3770 | C | ILE | B | 182 | 22.981 | 47.701 | 80.939 | 1.00 | 24.27 | C |
| ATOM | 3771 | O | ILE | B | 182 | 23.420 | 46.628 | 80.542 | 1.00 | 24.27 | O |
| ATOM | 3772 | CB | ILE | B | 182 | 21.766 | 46.828 | 82.914 | 1.00 | 11.90 | C |
| ATOM | 3773 | CG1 | ILE | B | 182 | 20.465 | 46.898 | 83.727 | 1.00 | 11.90 | C |
| ATOM | 3774 | CG2 | ILE | B | 182 | 22.995 | 47.152 | 83.750 | 1.00 | 11.90 | C |
| ATOM | 3775 | CD1 | ILE | B | 182 | 20.183 | 48.232 | 84.344 | 1.00 | 11.90 | C |
| ATOM | 3776 | N | LYS | B | 183 | 23.582 | 48.858 | 80.720 | 1.00 | 25.61 | N |
| ATOM | 3777 | CA | LYS | B | 183 | 24.840 | 48.953 | 80.017 | 1.00 | 25.61 | C |
| ATOM | 3778 | C | LYS | B | 183 | 25.529 | 50.185 | 80.599 | 1.00 | 25.61 | C |

| | | | | | | | | | | | |
|---|---|---|---|---|---|---|---|---|---|---|---|
| ATOM | 3779 | O | LYS | B | 183 | 24.864 | 51.071 | 81.133 | 1.00 | 25.61 | O |
| ATOM | 3780 | CB | LYS | B | 183 | 24.587 | 49.097 | 78.521 | 1.00 | 26.84 | C |
| ATOM | 3781 | CG | LYS | B | 183 | 23.655 | 50.227 | 78.160 | 1.00 | 26.84 | C |
| ATOM | 3782 | CD | LYS | B | 183 | 23.386 | 50.212 | 76.676 | 1.00 | 26.84 | C |
| ATOM | 3783 | CE | LYS | B | 183 | 22.511 | 51.361 | 76.259 | 1.00 | 26.84 | C |
| ATOM | 3784 | NZ | LYS | B | 183 | 22.311 | 51.388 | 74.793 | 1.00 | 26.84 | N |
| ATOM | 3785 | N | PRO | B | 184 | 26.864 | 50.262 | 80.493 | 1.00 | 24.98 | N |
| ATOM | 3786 | CA | PRO | B | 184 | 27.637 | 51.390 | 81.028 | 1.00 | 24.98 | C |
| ATOM | 3787 | C | PRO | B | 184 | 27.109 | 52.772 | 80.686 | 1.00 | 24.98 | C |
| ATOM | 3788 | O | PRO | B | 184 | 27.178 | 53.689 | 81.499 | 1.00 | 24.98 | O |
| ATOM | 3789 | CB | PRO | B | 184 | 29.033 | 51.144 | 80.469 | 1.00 | 26.91 | C |
| ATOM | 3790 | CG | PRO | B | 184 | 29.075 | 49.638 | 80.323 | 1.00 | 26.91 | C |
| ATOM | 3791 | CD | PRO | B | 184 | 27.731 | 49.346 | 79.732 | 1.00 | 26.91 | C |
| ATOM | 3792 | N | GLN | B | 185 | 26.580 | 52.906 | 79.478 | 1.00 | 25.81 | N |
| ATOM | 3793 | CA | GLN | B | 185 | 26.048 | 54.171 | 78.985 | 1.00 | 25.81 | C |
| ATOM | 3794 | C | GLN | B | 185 | 24.842 | 54.664 | 79.776 | 1.00 | 25.81 | C |
| ATOM | 3795 | O | GLN | B | 185 | 24.535 | 55.855 | 79.783 | 1.00 | 25.81 | O |
| ATOM | 3796 | CB | GLN | B | 185 | 25.661 | 54.035 | 77.504 | 1.00 | 36.37 | C |
| ATOM | 3797 | CG | GLN | B | 185 | 26.826 | 53.687 | 76.573 | 1.00 | 36.37 | C |
| ATOM | 3798 | CD | GLN | B | 185 | 27.430 | 52.332 | 76.885 | 1.00 | 36.37 | C |
| ATOM | 3799 | OE1 | GLN | B | 185 | 26.987 | 51.315 | 76.374 | 1.00 | 36.37 | O |
| ATOM | 3800 | NE2 | GLN | B | 185 | 28.434 | 52.316 | 77.748 | 0.00 | 36.37 | N |
| ATOM | 3801 | N | ASN | B | 186 | 24.154 | 53.739 | 80.437 | 1.00 | 30.97 | N |
| ATOM | 3802 | CA | ASN | B | 186 | 22.984 | 54.092 | 81.223 | 1.00 | 30.97 | C |
| ATOM | 3803 | C | ASN | B | 186 | 23.307 | 54.276 | 82.685 | 1.00 | 30.97 | C |
| ATOM | 3804 | O | ASN | B | 186 | 22.403 | 54.435 | 83.500 | 1.00 | 30.97 | O |
| ATOM | 3805 | CB | ASN | B | 186 | 21.888 | 53.046 | 81.078 | 1.00 | 18.75 | C |
| ATOM | 3806 | CG | ASN | B | 186 | 21.236 | 53.078 | 79.732 | 1.00 | 18.75 | C |
| ATOM | 3807 | OD1 | ASN | B | 186 | 21.075 | 54.132 | 79.138 | 1.00 | 18.75 | O |
| ATOM | 3808 | ND2 | ASN | B | 186 | 20.838 | 51.921 | 79.246 | 1.00 | 18.75 | N |
| ATOM | 3809 | N | LEU | B | 187 | 24.589 | 54.243 | 83.024 | 1.00 | 21.44 | N |
| ATOM | 3810 | CA | LEU | B | 187 | 24.998 | 54.463 | 84.404 | 1.00 | 21.44 | C |
| ATOM | 3811 | C | LEU | B | 187 | 25.540 | 55.899 | 84.601 | 1.00 | 21.44 | C |
| ATOM | 3812 | O | LEU | B | 187 | 26.734 | 56.163 | 84.424 | 1.00 | 21.44 | O |
| ATOM | 3813 | CB | LEU | B | 187 | 26.043 | 53.419 | 84.797 | 1.00 | 26.67 | C |
| ATOM | 3814 | CG | LEU | B | 187 | 25.584 | 52.033 | 85.281 | 1.00 | 26.67 | C |
| ATOM | 3815 | CD1 | LEU | B | 187 | 24.105 | 51.819 | 85.046 | 1.00 | 26.67 | C |
| ATOM | 3816 | CD2 | LEU | B | 187 | 26.386 | 50.967 | 84.581 | 1.00 | 26.67 | C |
| ATOM | 3817 | N | LEU | B | 188 | 24.656 | 56.835 | 84.943 | 1.00 | 23.69 | N |
| ATOM | 3818 | CA | LEU | B | 188 | 25.087 | 58.218 | 85.153 | 1.00 | 23.69 | C |
| ATOM | 3819 | C | LEU | B | 188 | 25.867 | 58.343 | 86.453 | 1.00 | 23.69 | C |
| ATOM | 3820 | O | LEU | B | 188 | 25.525 | 57.704 | 87.441 | 1.00 | 23.69 | O |
| ATOM | 3821 | CB | LEU | B | 188 | 23.889 | 59.159 | 85.228 | 1.00 | 27.99 | C |
| ATOM | 3822 | CG | LEU | B | 188 | 22.887 | 59.177 | 84.077 | 1.00 | 27.99 | C |
| ATOM | 3823 | CD1 | LEU | B | 188 | 21.903 | 60.288 | 84.317 | 1.00 | 27.99 | C |
| ATOM | 3824 | CD2 | LEU | B | 188 | 23.581 | 59.387 | 82.766 | 1.00 | 27.99 | C |
| ATOM | 3825 | N | LEU | B | 189 | 26.914 | 59.163 | 86.456 | 1.00 | 25.95 | N |
| ATOM | 3826 | CA | LEU | B | 189 | 27.693 | 59.346 | 87.670 | 1.00 | 25.95 | C |
| ATOM | 3827 | C | LEU | B | 189 | 28.250 | 60.745 | 87.866 | 1.00 | 25.95 | C |
| ATOM | 3828 | O | LEU | B | 189 | 28.472 | 61.479 | 86.913 | 1.00 | 25.95 | O |
| ATOM | 3829 | CB | LEU | B | 189 | 28.821 | 58.313 | 87.741 | 1.00 | 37.67 | C |
| ATOM | 3830 | CG | LEU | B | 189 | 29.947 | 58.308 | 86.715 | 1.00 | 37.67 | C |
| ATOM | 3831 | CD1 | LEU | B | 189 | 30.933 | 59.435 | 86.993 | 0.00 | 37.67 | C |
| ATOM | 3832 | CD2 | LEU | B | 189 | 30.658 | 56.958 | 86.805 | 1.00 | 37.67 | C |
| ATOM | 3833 | N | ASP | B | 190 | 28.444 | 61.111 | 89.127 | 1.00 | 32.61 | N |
| ATOM | 3834 | CA | ASP | B | 190 | 28.983 | 62.410 | 89.482 | 1.00 | 32.61 | C |
| ATOM | 3835 | C | ASP | B | 190 | 30.426 | 62.172 | 89.897 | 1.00 | 32.61 | C |
| ATOM | 3836 | O | ASP | B | 190 | 30.685 | 61.519 | 90.908 | 1.00 | 32.61 | O |
| ATOM | 3837 | CB | ASP | B | 190 | 28.205 | 63.015 | 90.646 | 1.00 | 39.95 | C |
| ATOM | 3838 | CG | ASP | B | 190 | 28.748 | 64.369 | 91.063 | 1.00 | 39.95 | C |
| ATOM | 3839 | OD1 | ASP | B | 190 | 29.982 | 64.491 | 91.251 | 1.00 | 39.95 | O |
| ATOM | 3840 | OD2 | ASP | B | 190 | 27.940 | 65.309 | 91.211 | 1.00 | 39.95 | O |

| | | | | | | | | | | | |
|---|---|---|---|---|---|---|---|---|---|---|---|
| ATOM | 3841 | N | PRO | B | 191 | 31.386 | 62.719 | 89.133 | 1.00 | 50.68 | N |
| ATOM | 3842 | CA | PRO | B | 191 | 32.805 | 62.542 | 89.437 | 1.00 | 50.68 | C |
| ATOM | 3843 | C | PRO | B | 191 | 33.152 | 62.863 | 90.876 | 1.00 | 50.68 | C |
| ATOM | 3844 | O | PRO | B | 191 | 33.746 | 62.029 | 91.574 | 1.00 | 50.68 | O |
| ATOM | 3845 | CB | PRO | B | 191 | 33.491 | 63.479 | 88.448 | 0.00 | 60.50 | C |
| ATOM | 3846 | CG | PRO | B | 191 | 32.532 | 64.582 | 88.337 | 1.00 | 60.50 | C |
| ATOM | 3847 | CD | PRO | B | 191 | 31.211 | 63.833 | 88.184 | 1.00 | 60.50 | C |
| ATOM | 3848 | N | ASP | B | 192 | 32.757 | 64.056 | 91.322 | 1.00 | 41.81 | N |
| ATOM | 3849 | CA | ASP | B | 192 | 33.054 | 64.518 | 92.676 | 1.00 | 41.81 | C |
| ATOM | 3850 | C | ASP | B | 192 | 32.441 | 63.700 | 93.822 | 1.00 | 41.81 | C |
| ATOM | 3851 | O | ASP | B | 192 | 33.104 | 63.423 | 94.819 | 1.00 | 41.81 | O |
| ATOM | 3852 | CB | ASP | B | 192 | 32.623 | 65.983 | 92.852 | 1.00 | 58.23 | C |
| ATOM | 3853 | CG | ASP | B | 192 | 33.083 | 66.895 | 91.702 | 1.00 | 58.23 | C |
| ATOM | 3854 | OD1 | ASP | B | 192 | 34.262 | 66.788 | 91.253 | 1.00 | 58.23 | O |
| ATOM | 3855 | OD2 | ASP | B | 192 | 32.249 | 67.742 | 91.267 | 1.00 | 58.23 | O |
| ATOM | 3856 | N | THR | B | 193 | 31.181 | 63.314 | 93.703 | 1.00 | 27.29 | N |
| ATOM | 3857 | CA | THR | B | 193 | 30.561 | 62.580 | 94.794 | 1.00 | 27.29 | C |
| ATOM | 3858 | C | THR | B | 193 | 30.584 | 61.063 | 94.634 | 1.00 | 27.29 | C |
| ATOM | 3859 | O | THR | B | 193 | 30.344 | 60.336 | 95.585 | 1.00 | 27.29 | O |
| ATOM | 3860 | CB | THR | B | 193 | 29.123 | 63.069 | 94.994 | 1.00 | 28.38 | C |
| ATOM | 3861 | OG1 | THR | B | 193 | 28.315 | 62.652 | 93.890 | 1.00 | 28.38 | O |
| ATOM | 3862 | CG2 | THR | B | 193 | 29.102 | 64.584 | 95.052 | 1.00 | 28.38 | C |
| ATOM | 3863 | N | ALA | B | 194 | 30.889 | 60.585 | 93.434 | 1.00 | 24.90 | N |
| ATOM | 3864 | CA | ALA | B | 194 | 30.932 | 59.146 | 93.179 | 1.00 | 24.90 | C |
| ATOM | 3865 | C | ALA | B | 194 | 29.542 | 58.511 | 93.285 | 1.00 | 24.90 | C |
| ATOM | 3866 | O | ALA | B | 194 | 29.405 | 57.316 | 93.535 | 1.00 | 24.90 | O |
| ATOM | 3867 | CB | ALA | B | 194 | 31.897 | 58.468 | 94.148 | 1.00 | 15.13 | C |
| ATOM | 3868 | N | VAL | B | 195 | 28.516 | 59.330 | 93.087 | 1.00 | 23.36 | N |
| ATOM | 3869 | CA | VAL | B | 195 | 27.135 | 58.880 | 93.147 | 1.00 | 23.36 | C |
| ATOM | 3870 | C | VAL | B | 195 | 26.725 | 58.335 | 91.790 | 1.00 | 23.36 | C |
| ATOM | 3871 | O | VAL | B | 195 | 26.898 | 58.993 | 90.773 | 1.00 | 23.36 | O |
| ATOM | 3872 | CB | VAL | B | 195 | 26.203 | 60.053 | 93.536 | 1.00 | 15.16 | C |
| ATOM | 3873 | CG1 | VAL | B | 195 | 24.752 | 59.695 | 93.304 | 1.00 | 15.16 | C |
| ATOM | 3874 | CG2 | VAL | B | 195 | 26.421 | 60.403 | 94.980 | 1.00 | 15.16 | C |
| ATOM | 3875 | N | LEU | B | 196 | 26.194 | 57.124 | 91.768 | 1.00 | 19.41 | N |
| ATOM | 3876 | CA | LEU | B | 196 | 25.764 | 56.552 | 90.510 | 1.00 | 19.41 | C |
| ATOM | 3877 | C | LEU | B | 196 | 24.253 | 56.633 | 90.422 | 1.00 | 19.41 | C |
| ATOM | 3878 | O | LEU | B | 196 | 23.566 | 56.597 | 91.433 | 1.00 | 19.41 | O |
| ATOM | 3879 | CB | LEU | B | 196 | 26.223 | 55.094 | 90.391 | 1.00 | 19.09 | C |
| ATOM | 3880 | CG | LEU | B | 196 | 25.774 | 54.309 | 89.151 | 1.00 | 19.09 | C |
| ATOM | 3881 | CD1 | LEU | B | 196 | 26.694 | 53.126 | 88.926 | 1.00 | 19.09 | C |
| ATOM | 3882 | CD2 | LEU | B | 196 | 24.328 | 53.855 | 89.320 | 1.00 | 19.09 | C |
| ATOM | 3883 | N | LYS | B | 197 | 23.745 | 56.759 | 89.206 | 1.00 | 25.21 | N |
| ATOM | 3884 | CA | LYS | B | 197 | 22.311 | 56.829 | 88.978 | 1.00 | 25.21 | C |
| ATOM | 3885 | C | LYS | B | 197 | 21.907 | 56.062 | 87.738 | 1.00 | 25.21 | C |
| ATOM | 3886 | O | LYS | B | 197 | 22.498 | 56.238 | 86.677 | 1.00 | 25.21 | O |
| ATOM | 3887 | CB | LYS | B | 197 | 21.859 | 58.281 | 88.850 | 1.00 | 21.67 | C |
| ATOM | 3888 | CG | LYS | B | 197 | 21.609 | 58.937 | 90.172 | 1.00 | 21.67 | C |
| ATOM | 3889 | CD | LYS | B | 197 | 21.334 | 60.399 | 90.008 | 1.00 | 21.67 | C |
| ATOM | 3890 | CE | LYS | B | 197 | 20.902 | 61.012 | 91.327 | 1.00 | 21.67 | C |
| ATOM | 3891 | NZ | LYS | B | 197 | 21.876 | 60.720 | 92.392 | 1.00 | 21.67 | N |
| ATOM | 3892 | N | LEU | B | 198 | 20.916 | 55.189 | 87.883 | 1.00 | 24.72 | N |
| ATOM | 3893 | CA | LEU | B | 198 | 20.425 | 54.418 | 86.753 | 1.00 | 24.72 | C |
| ATOM | 3894 | C | LEU | B | 198 | 19.507 | 55.338 | 85.954 | 1.00 | 24.72 | C |
| ATOM | 3895 | O | LEU | B | 198 | 18.703 | 56.068 | 86.530 | 1.00 | 24.72 | O |
| ATOM | 3896 | CB | LEU | B | 198 | 19.659 | 53.187 | 87.240 | 1.00 | 13.35 | C |
| ATOM | 3897 | CG | LEU | B | 198 | 19.010 | 52.280 | 86.194 | 1.00 | 13.35 | C |
| ATOM | 3898 | CD1 | LEU | B | 198 | 20.025 | 51.791 | 85.212 | 1.00 | 13.35 | C |
| ATOM | 3899 | CD2 | LEU | B | 198 | 18.363 | 51.113 | 86.871 | 1.00 | 13.35 | C |
| ATOM | 3900 | N | CYS | B | 199 | 19.651 | 55.325 | 84.632 | 1.00 | 22.21 | N |
| ATOM | 3901 | CA | CYS | B | 199 | 18.823 | 56.160 | 83.768 | 1.00 | 22.21 | C |
| ATOM | 3902 | C | CYS | B | 199 | 18.315 | 55.392 | 82.542 | 1.00 | 22.21 | C |

| | | | | | | | | | | | |
|---|---|---|---|---|---|---|---|---|---|---|---|
| ATOM | 3903 | O | CYS | B | 199 | 18.629 | 54.225 | 82.357 | 1.00 | 22.21 | O |
| ATOM | 3904 | CB | CYS | B | 199 | 19.604 | 57.397 | 83.308 | 1.00 | 28.99 | C |
| ATOM | 3905 | SG | CYS | B | 199 | 20.866 | 57.074 | 82.054 | 1.00 | 28.99 | S |
| ATOM | 3906 | N | ASP | B | 200 | 17.524 | 56.070 | 81.719 | 1.00 | 23.92 | N |
| ATOM | 3907 | CA | ASP | B | 200 | 16.956 | 55.484 | 80.515 | 1.00 | 23.92 | C |
| ATOM | 3908 | C | ASP | B | 200 | 16.026 | 54.289 | 80.752 | 1.00 | 23.92 | C |
| ATOM | 3909 | O | ASP | B | 200 | 16.443 | 53.131 | 80.646 | 1.00 | 23.92 | O |
| ATOM | 3910 | CB | ASP | B | 200 | 18.057 | 55.053 | 79.563 | 1.00 | 34.92 | C |
| ATOM | 3911 | CG | ASP | B | 200 | 17.512 | 54.618 | 78.231 | 1.00 | 34.92 | C |
| ATOM | 3912 | OD1 | ASP | B | 200 | 16.270 | 54.557 | 78.095 | 1.00 | 34.92 | O |
| ATOM | 3913 | OD2 | ASP | B | 200 | 18.319 | 54.339 | 77.317 | 1.00 | 34.92 | O |
| ATOM | 3914 | N | PHE | B | 201 | 14.759 | 54.564 | 81.045 | 1.00 | 29.97 | N |
| ATOM | 3915 | CA | PHE | B | 201 | 13.805 | 53.495 | 81.281 | 1.00 | 29.97 | C |
| ATOM | 3916 | C | PHE | B | 201 | 12.904 | 53.211 | 80.069 | 1.00 | 29.97 | C |
| ATOM | 3917 | O | PHE | B | 201 | 11.803 | 52.680 | 80.203 | 1.00 | 29.97 | O |
| ATOM | 3918 | CB | PHE | B | 201 | 12.971 | 53.824 | 82.524 | 1.00 | 21.34 | C |
| ATOM | 3919 | CG | PHE | B | 201 | 13.738 | 53.713 | 83.806 | 1.00 | 21.34 | C |
| ATOM | 3920 | CD1 | PHE | B | 201 | 14.659 | 54.683 | 84.166 | 1.00 | 21.34 | C |
| ATOM | 3921 | CD2 | PHE | B | 201 | 13.596 | 52.595 | 84.618 | 1.00 | 21.34 | C |
| ATOM | 3922 | CE1 | PHE | B | 201 | 15.437 | 54.536 | 85.318 | 1.00 | 21.34 | C |
| ATOM | 3923 | CE2 | PHE | B | 201 | 14.363 | 52.438 | 85.765 | 1.00 | 21.34 | C |
| ATOM | 3924 | CZ | PHE | B | 201 | 15.288 | 53.411 | 86.116 | 1.00 | 21.34 | C |
| ATOM | 3925 | N | GLY | B | 202 | 13.388 | 53.549 | 78.882 | 1.00 | 21.68 | N |
| ATOM | 3926 | CA | GLY | B | 202 | 12.601 | 53.324 | 77.685 | 1.00 | 21.68 | C |
| ATOM | 3927 | C | GLY | B | 202 | 12.315 | 51.869 | 77.356 | 1.00 | 21.68 | C |
| ATOM | 3928 | O | GLY | B | 202 | 11.433 | 51.575 | 76.552 | 1.00 | 21.68 | O |
| ATOM | 3929 | N | SER | B | 203 | 13.048 | 50.954 | 77.975 | 1.00 | 32.70 | N |
| ATOM | 3930 | CA | SER | B | 203 | 12.856 | 49.534 | 77.715 | 1.00 | 32.70 | C |
| ATOM | 3931 | C | SER | B | 203 | 12.335 | 48.806 | 78.921 | 1.00 | 32.70 | C |
| ATOM | 3932 | O | SER | B | 203 | 12.117 | 47.608 | 78.855 | 1.00 | 32.70 | O |
| ATOM | 3933 | CB | SER | B | 203 | 14.171 | 48.881 | 77.311 | 1.00 | 29.51 | C |
| ATOM | 3934 | OG | SER | B | 203 | 14.771 | 49.595 | 76.259 | 1.00 | 29.51 | O |
| ATOM | 3935 | N | ALA | B | 204 | 12.156 | 49.528 | 80.020 | 1.00 | 20.67 | N |
| ATOM | 3936 | CA | ALA | B | 204 | 11.687 | 48.942 | 81.262 | 1.00 | 20.67 | C |
| ATOM | 3937 | C | ALA | B | 204 | 10.236 | 48.489 | 81.209 | 1.00 | 20.67 | C |
| ATOM | 3938 | O | ALA | B | 204 | 9.406 | 49.062 | 80.496 | 1.00 | 20.67 | O |
| ATOM | 3939 | CB | ALA | B | 204 | 11.882 | 49.932 | 82.405 | 1.00 | 28.67 | C |
| ATOM | 3940 | N | LYS | B | 205 | 9.933 | 47.465 | 81.993 | 1.00 | 24.34 | N |
| ATOM | 3941 | CA | LYS | B | 205 | 8.590 | 46.930 | 82.035 | 1.00 | 24.34 | C |
| ATOM | 3942 | C | LYS | B | 205 | 8.311 | 46.105 | 83.293 | 1.00 | 24.34 | C |
| ATOM | 3943 | O | LYS | B | 205 | 9.210 | 45.511 | 83.879 | 1.00 | 24.34 | O |
| ATOM | 3944 | CB | LYS | B | 205 | 8.359 | 46.057 | 80.809 | 1.00 | 33.91 | C |
| ATOM | 3945 | CG | LYS | B | 205 | 6.923 | 45.660 | 80.675 | 1.00 | 33.91 | C |
| ATOM | 3946 | CD | LYS | B | 205 | 6.705 | 44.576 | 79.642 | 1.00 | 33.91 | C |
| ATOM | 3947 | CE | LYS | B | 205 | 5.210 | 44.354 | 79.383 | 1.00 | 33.91 | C |
| ATOM | 3948 | NZ | LYS | B | 205 | 4.954 | 43.138 | 78.564 | 1.00 | 33.91 | N |
| ATOM | 3949 | N | GLN | B | 206 | 7.056 | 46.083 | 83.715 | 1.00 | 31.27 | N |
| ATOM | 3950 | CA | GLN | B | 206 | 6.670 | 45.280 | 84.862 | 1.00 | 31.27 | C |
| ATOM | 3951 | C | GLN | B | 206 | 6.597 | 43.871 | 84.292 | 1.00 | 31.27 | C |
| ATOM | 3952 | O | GLN | B | 206 | 5.893 | 43.642 | 83.317 | 1.00 | 31.27 | O |
| ATOM | 3953 | CB | GLN | B | 206 | 5.300 | 45.705 | 85.366 | 1.00 | 53.66 | C |
| ATOM | 3954 | CG | GLN | B | 206 | 5.180 | 47.198 | 85.627 | 1.00 | 53.66 | C |
| ATOM | 3955 | CD | GLN | B | 206 | 4.520 | 47.475 | 86.971 | 1.00 | 53.66 | C |
| ATOM | 3956 | OE1 | GLN | B | 206 | 4.259 | 48.638 | 87.338 | 1.00 | 53.66 | O |
| ATOM | 3957 | NE2 | GLN | B | 206 | 4.247 | 46.395 | 87.721 | 1.00 | 53.66 | N |
| ATOM | 3958 | N | LEU | B | 207 | 7.331 | 42.934 | 84.876 | 1.00 | 33.98 | N |
| ATOM | 3959 | CA | LEU | B | 207 | 7.336 | 41.569 | 84.361 | 1.00 | 33.98 | C |
| ATOM | 3960 | C | LEU | B | 207 | 6.400 | 40.639 | 85.101 | 1.00 | 33.98 | C |
| ATOM | 3961 | O | LEU | B | 207 | 6.827 | 39.908 | 85.997 | 1.00 | 33.98 | O |
| ATOM | 3962 | CB | LEU | B | 207 | 8.743 | 40.969 | 84.418 | 1.00 | 29.59 | C |
| ATOM | 3963 | CG | LEU | B | 207 | 9.856 | 41.433 | 83.481 | 1.00 | 29.59 | C |
| ATOM | 3964 | CD1 | LEU | B | 207 | 11.024 | 40.470 | 83.647 | 1.00 | 29.59 | C |

| | | | | | | | | | | | |
|---|---|---|---|---|---|---|---|---|---|---|---|
| ATOM | 3965 | CD2 | LEU | B | 207 | 9.385 | 41.450 | 82.026 | 1.00 | 29.59 | C |
| ATOM | 3966 | N | VAL | B | 208 | 5.132 | 40.648 | 84.715 | 1.00 | 44.62 | N |
| ATOM | 3967 | CA | VAL | B | 208 | 4.133 | 39.791 | 85.350 | 1.00 | 44.62 | C |
| ATOM | 3968 | C | VAL | B | 208 | 4.300 | 38.335 | 84.900 | 1.00 | 44.62 | C |
| ATOM | 3969 | O | VAL | B | 208 | 4.349 | 38.054 | 83.705 | 1.00 | 44.62 | O |
| ATOM | 3970 | CB | VAL | B | 208 | 2.720 | 40.267 | 84.988 | 1.00 | 41.62 | C |
| ATOM | 3971 | CG1 | VAL | B | 208 | 1.710 | 39.724 | 85.987 | 0.00 | 41.62 | C |
| ATOM | 3972 | CG2 | VAL | B | 208 | 2.696 | 41.794 | 84.939 | 1.00 | 41.62 | C |
| ATOM | 3973 | N | ARG | B | 209 | 4.389 | 37.410 | 85.853 | 1.00 | 45.27 | N |
| ATOM | 3974 | CA | ARG | B | 209 | 4.549 | 35.987 | 85.520 | 1.00 | 45.27 | C |
| ATOM | 3975 | C | ARG | B | 209 | 3.458 | 35.489 | 84.550 | 1.00 | 45.27 | C |
| ATOM | 3976 | O | ARG | B | 209 | 2.294 | 35.902 | 84.622 | 1.00 | 45.27 | O |
| ATOM | 3977 | CB | ARG | B | 209 | 4.541 | 35.142 | 86.804 | 1.00 | 34.94 | C |
| ATOM | 3978 | N | GLY | B | 210 | 3.839 | 34.607 | 83.634 | 1.00 | 39.40 | N |
| ATOM | 3979 | CA | GLY | B | 210 | 2.874 | 34.097 | 82.682 | 1.00 | 39.40 | C |
| ATOM | 3980 | C | GLY | B | 210 | 2.739 | 34.939 | 81.428 | 1.00 | 39.40 | C |
| ATOM | 3981 | O | GLY | B | 210 | 2.256 | 34.451 | 80.402 | 1.00 | 39.40 | O |
| ATOM | 3982 | N | GLU | B | 211 | 3.147 | 36.203 | 81.495 | 1.00 | 48.68 | N |
| ATOM | 3983 | CA | GLU | B | 211 | 3.066 | 37.074 | 80.323 | 1.00 | 48.68 | C |
| ATOM | 3984 | C | GLU | B | 211 | 4.365 | 37.073 | 79.534 | 1.00 | 48.68 | C |
| ATOM | 3985 | O | GLU | B | 211 | 5.446 | 37.193 | 80.097 | 1.00 | 48.68 | O |
| ATOM | 3986 | CB | GLU | B | 211 | 2.728 | 38.495 | 80.728 | 1.00 | 61.36 | C |
| ATOM | 3987 | CG | GLU | B | 211 | 1.302 | 38.658 | 81.185 | 1.00 | 61.36 | C |
| ATOM | 3988 | CD | GLU | B | 211 | 1.004 | 40.078 | 81.629 | 1.00 | 61.36 | C |
| ATOM | 3989 | OE1 | GLU | B | 211 | -0.121 | 40.323 | 82.132 | 1.00 | 61.36 | O |
| ATOM | 3990 | OE2 | GLU | B | 211 | 1.901 | 40.946 | 81.470 | 1.00 | 61.36 | O |
| ATOM | 3991 | N | PRO | B | 212 | 4.271 | 36.924 | 78.211 | 1.00 | 42.30 | N |
| ATOM | 3992 | CA | PRO | B | 212 | 5.459 | 36.909 | 77.364 | 1.00 | 42.30 | C |
| ATOM | 3993 | C | PRO | B | 212 | 6.042 | 38.299 | 77.164 | 1.00 | 42.30 | C |
| ATOM | 3994 | O | PRO | B | 212 | 5.309 | 39.295 | 77.171 | 1.00 | 42.30 | O |
| ATOM | 3995 | CB | PRO | B | 212 | 4.940 | 36.297 | 76.063 | 1.00 | 48.75 | C |
| ATOM | 3996 | CG | PRO | B | 212 | 3.553 | 36.807 | 76.004 | 1.00 | 48.75 | C |
| ATOM | 3997 | CD | PRO | B | 212 | 3.063 | 36.625 | 77.420 | 1.00 | 48.75 | C |
| ATOM | 3998 | N | ASN | B | 213 | 7.362 | 38.358 | 77.002 | 1.00 | 29.63 | N |
| ATOM | 3999 | CA | ASN | B | 213 | 8.043 | 39.621 | 76.780 | 1.00 | 29.63 | C |
| ATOM | 4000 | C | ASN | B | 213 | 8.996 | 39.474 | 75.601 | 1.00 | 29.63 | C |
| ATOM | 4001 | O | ASN | B | 213 | 9.568 | 38.405 | 75.387 | 1.00 | 29.63 | O |
| ATOM | 4002 | CB | ASN | B | 213 | 8.790 | 40.032 | 78.041 | 1.00 | 23.53 | C |
| ATOM | 4003 | CG | ASN | B | 213 | 7.869 | 40.178 | 79.229 | 1.00 | 23.53 | C |
| ATOM | 4004 | OD1 | ASN | B | 213 | 7.045 | 41.096 | 79.288 | 1.00 | 23.53 | O |
| ATOM | 4005 | ND2 | ASN | B | 213 | 7.991 | 39.256 | 80.180 | 1.00 | 23.53 | N |
| ATOM | 4006 | N | VAL | B | 214 | 9.141 | 40.543 | 74.826 | 1.00 | 26.81 | N |
| ATOM | 4007 | CA | VAL | B | 214 | 10.079 | 40.505 | 73.707 | 1.00 | 26.81 | C |
| ATOM | 4008 | C | VAL | B | 214 | 11.504 | 40.191 | 74.176 | 1.00 | 26.81 | C |
| ATOM | 4009 | O | VAL | B | 214 | 11.892 | 40.452 | 75.307 | 1.00 | 26.81 | O |
| ATOM | 4010 | CB | VAL | B | 214 | 10.043 | 41.860 | 72.999 | 1.00 | 21.30 | C |
| ATOM | 4011 | CG1 | VAL | B | 214 | 8.640 | 42.126 | 72.456 | 1.00 | 21.30 | C |
| ATOM | 4012 | CG2 | VAL | B | 214 | 10.422 | 42.965 | 73.969 | 1.00 | 21.30 | C |
| ATOM | 4013 | N | SER | B | 215 | 12.283 | 39.571 | 73.268 | 1.00 | 28.62 | N |
| ATOM | 4014 | CA | SER | B | 215 | 13.621 | 39.099 | 73.612 | 1.00 | 28.62 | C |
| ATOM | 4015 | C | SER | B | 215 | 14.704 | 40.039 | 73.079 | 1.00 | 28.62 | C |
| ATOM | 4016 | O | SER | B | 215 | 15.822 | 40.103 | 73.576 | 1.00 | 28.62 | O |
| ATOM | 4017 | CB | SER | B | 215 | 13.805 | 37.705 | 73.015 | 1.00 | 30.12 | C |
| ATOM | 4018 | OG | SER | B | 215 | 13.657 | 37.776 | 71.597 | 1.00 | 30.12 | O |
| ATOM | 4019 | N | TYR | B | 216 | 14.348 | 40.751 | 71.995 | 1.00 | 29.71 | N |
| ATOM | 4020 | CA | TYR | B | 216 | 15.281 | 41.710 | 71.414 | 1.00 | 29.71 | C |
| ATOM | 4021 | C | TYR | B | 216 | 15.185 | 43.066 | 72.113 | 1.00 | 29.71 | C |
| ATOM | 4022 | O | TYR | B | 216 | 14.718 | 44.053 | 71.561 | 1.00 | 29.71 | O |
| ATOM | 4023 | CB | TYR | B | 216 | 14.921 | 41.871 | 69.936 | 1.00 | 34.42 | C |
| ATOM | 4024 | CG | TYR | B | 216 | 13.545 | 42.430 | 69.821 | 1.00 | 34.42 | C |
| ATOM | 4025 | CD1 | TYR | B | 216 | 13.324 | 43.781 | 70.074 | 1.00 | 34.42 | C |
| ATOM | 4026 | CD2 | TYR | B | 216 | 12.462 | 41.592 | 69.554 | 1.00 | 34.42 | C |

| | | | | | | | | | | | |
|---|---|---|---|---|---|---|---|---|---|---|---|
| ATOM | 4027 | CE1 | TYR | B | 216 | 12.033 | 44.290 | 70.070 | 1.00 | 34.42 | C |
| ATOM | 4028 | CE2 | TYR | B | 216 | 11.170 | 42.100 | 69.550 | 1.00 | 34.42 | C |
| ATOM | 4029 | CZ | TYR | B | 216 | 10.955 | 43.442 | 69.808 | 1.00 | 34.42 | C |
| ATOM | 4030 | OH | TYR | B | 216 | 9.675 | 43.961 | 69.782 | 1.00 | 34.42 | O |
| ATOM | 4031 | N | ILE | B | 217 | 15.613 | 43.086 | 73.389 | 1.00 | 44.24 | N |
| ATOM | 4032 | CA | ILE | B | 217 | 15.470 | 44.310 | 74.169 | 1.00 | 44.24 | C |
| ATOM | 4033 | C | ILE | B | 217 | 16.809 | 44.801 | 74.728 | 1.00 | 44.24 | C |
| ATOM | 4034 | O | ILE | B | 217 | 17.135 | 45.980 | 74.702 | 1.00 | 44.24 | O |
| ATOM | 4035 | CB | ILE | B | 217 | 14.486 | 44.034 | 75.307 | 1.00 | 22.42 | C |
| ATOM | 4036 | CG1 | ILE | B | 217 | 14.199 | 45.321 | 76.084 | 1.00 | 22.42 | C |
| ATOM | 4037 | CG2 | ILE | B | 217 | 15.093 | 43.012 | 76.287 | 1.00 | 22.42 | C |
| ATOM | 4038 | CD1 | ILE | B | 217 | 13.249 | 46.254 | 75.333 | 1.00 | 22.42 | C |
| ATOM | 4039 | N | CYS | B | 218 | 17.582 | 43.850 | 75.284 | 1.00 | 30.97 | N |
| ATOM | 4040 | CA | CYS | B | 218 | 18.877 | 44.218 | 75.846 | 1.00 | 30.97 | C |
| ATOM | 4041 | C | CYS | B | 218 | 19.884 | 44.577 | 74.752 | 1.00 | 30.97 | C |
| ATOM | 4042 | O | CYS | B | 218 | 19.589 | 44.574 | 73.565 | 1.00 | 30.97 | O |
| ATOM | 4043 | CB | CYS | B | 218 | 19.398 | 43.038 | 76.668 | 1.00 | 33.67 | C |
| ATOM | 4044 | SG | CYS | B | 218 | 20.588 | 43.554 | 77.927 | 1.00 | 33.67 | S |
| ATOM | 4045 | N | SER | B | 219 | 21.102 | 44.935 | 75.197 | 1.00 | 27.56 | N |
| ATOM | 4046 | CA | SER | B | 219 | 22.140 | 45.291 | 74.238 | 1.00 | 27.56 | C |
| ATOM | 4047 | C | SER | B | 219 | 23.031 | 44.094 | 73.903 | 1.00 | 27.56 | C |
| ATOM | 4048 | O | SER | B | 219 | 22.896 | 43.004 | 74.444 | 1.00 | 27.56 | O |
| ATOM | 4049 | CB | SER | B | 219 | 22.981 | 46.415 | 74.844 | 1.00 | 30.92 | C |
| ATOM | 4050 | OG | SER | B | 219 | 22.585 | 47.660 | 74.267 | 1.00 | 30.92 | O |
| ATOM | 4051 | N | ARG | B | 220 | 23.943 | 44.318 | 72.940 | 1.00 | 31.13 | N |
| ATOM | 4052 | CA | ARG | B | 220 | 24.848 | 43.247 | 72.541 | 1.00 | 31.13 | C |
| ATOM | 4053 | C | ARG | B | 220 | 25.697 | 42.509 | 73.579 | 1.00 | 31.13 | C |
| ATOM | 4054 | O | ARG | B | 220 | 25.429 | 41.371 | 73.943 | 1.00 | 31.13 | O |
| ATOM | 4055 | CB | ARG | B | 220 | 25.850 | 43.821 | 71.538 | 1.00 | 26.70 | C |
| ATOM | 4056 | N | TYR | B | 221 | 26.700 | 43.258 | 74.067 | 1.00 | 28.91 | N |
| ATOM | 4057 | CA | TYR | B | 221 | 27.606 | 42.635 | 75.012 | 1.00 | 28.91 | C |
| ATOM | 4058 | C | TYR | B | 221 | 26.809 | 42.484 | 76.315 | 1.00 | 28.91 | C |
| ATOM | 4059 | O | TYR | B | 221 | 27.156 | 41.750 | 77.232 | 1.00 | 28.91 | O |
| ATOM | 4060 | CB | TYR | B | 221 | 28.799 | 43.593 | 75.285 | 1.00 | 45.58 | C |
| ATOM | 4061 | CG | TYR | B | 221 | 29.362 | 44.052 | 73.988 | 1.00 | 45.58 | C |
| ATOM | 4062 | CD1 | TYR | B | 221 | 29.333 | 43.210 | 72.880 | 1.00 | 45.58 | C |
| ATOM | 4063 | CD2 | TYR | B | 221 | 29.926 | 45.323 | 73.876 | 1.00 | 45.58 | C |
| ATOM | 4064 | CE1 | TYR | B | 221 | 29.856 | 43.637 | 71.668 | 1.00 | 45.58 | C |
| ATOM | 4065 | CE2 | TYR | B | 221 | 30.449 | 45.749 | 72.664 | 1.00 | 45.58 | C |
| ATOM | 4066 | CZ | TYR | B | 221 | 30.410 | 44.914 | 71.563 | 1.00 | 45.58 | C |
| ATOM | 4067 | OH | TYR | B | 221 | 30.865 | 45.357 | 70.336 | 1.00 | 45.58 | O |
| ATOM | 4068 | N | TYR | B | 222 | 25.637 | 43.083 | 76.591 | 1.00 | 28.02 | N |
| ATOM | 4069 | CA | TYR | B | 222 | 25.017 | 42.855 | 77.895 | 1.00 | 28.02 | C |
| ATOM | 4070 | C | TYR | B | 222 | 23.809 | 41.917 | 77.812 | 1.00 | 28.02 | C |
| ATOM | 4071 | O | TYR | B | 222 | 23.160 | 41.608 | 78.803 | 1.00 | 28.02 | O |
| ATOM | 4072 | CB | TYR | B | 222 | 24.599 | 44.209 | 78.465 | 1.00 | 23.72 | C |
| ATOM | 4073 | CG | TYR | B | 222 | 25.717 | 45.179 | 78.311 | 1.00 | 23.72 | C |
| ATOM | 4074 | CD1 | TYR | B | 222 | 25.784 | 45.982 | 77.176 | 1.00 | 23.72 | C |
| ATOM | 4075 | CD2 | TYR | B | 222 | 26.757 | 45.209 | 79.240 | 1.00 | 23.72 | C |
| ATOM | 4076 | CE1 | TYR | B | 222 | 26.887 | 46.794 | 76.961 | 1.00 | 23.72 | C |
| ATOM | 4077 | CE2 | TYR | B | 222 | 27.861 | 46.021 | 79.025 | 1.00 | 23.72 | C |
| ATOM | 4078 | CZ | TYR | B | 222 | 27.930 | 46.808 | 77.889 | 1.00 | 23.72 | C |
| ATOM | 4079 | OH | TYR | B | 222 | 29.030 | 47.610 | 77.659 | 1.00 | 23.72 | O |
| ATOM | 4080 | N | ARG | B | 223 | 23.377 | 41.376 | 76.660 | 1.00 | 24.53 | N |
| ATOM | 4081 | CA | ARG | B | 223 | 22.227 | 40.481 | 76.678 | 1.00 | 24.53 | C |
| ATOM | 4082 | C | ARG | B | 223 | 22.550 | 39.164 | 77.391 | 1.00 | 24.53 | C |
| ATOM | 4083 | O | ARG | B | 223 | 23.540 | 38.502 | 77.107 | 1.00 | 24.53 | O |
| ATOM | 4084 | CB | ARG | B | 223 | 21.814 | 40.212 | 75.231 | 1.00 | 26.99 | C |
| ATOM | 4085 | CG | ARG | B | 223 | 21.984 | 41.446 | 74.343 | 1.00 | 26.99 | C |
| ATOM | 4086 | CD | ARG | B | 223 | 21.564 | 41.177 | 72.894 | 1.00 | 26.99 | C |
| ATOM | 4087 | NE | ARG | B | 223 | 20.208 | 40.623 | 72.844 | 1.00 | 26.99 | N |
| ATOM | 4088 | CZ | ARG | B | 223 | 19.995 | 39.593 | 72.005 | 1.00 | 26.99 | C |

| | | | | | | | | | | | |
|---|---|---|---|---|---|---|---|---|---|---|---|
| ATOM | 4089 | NH1 | ARG | B | 223 | 20.985 | 39.123 | 71.266 | 1.00 | 26.99 | N |
| ATOM | 4090 | NH2 | ARG | B | 223 | 18.783 | 39.034 | 71.940 | 1.00 | 26.99 | N |
| ATOM | 4091 | N | ALA | B | 224 | 21.619 | 38.783 | 78.255 | 1.00 | 17.47 | N |
| ATOM | 4092 | CA | ALA | B | 224 | 21.740 | 37.528 | 78.954 | 1.00 | 17.47 | C |
| ATOM | 4093 | C | ALA | B | 224 | 21.665 | 36.408 | 77.954 | 1.00 | 17.47 | C |
| ATOM | 4094 | O | ALA | B | 224 | 21.072 | 36.535 | 76.891 | 1.00 | 17.47 | O |
| ATOM | 4095 | CB | ALA | B | 224 | 20.643 | 37.383 | 79.979 | 1.00 | 11.85 | C |
| ATOM | 4096 | N | PRO | B | 225 | 22.284 | 35.285 | 78.281 | 1.00 | 28.48 | N |
| ATOM | 4097 | CA | PRO | B | 225 | 22.224 | 34.188 | 77.338 | 1.00 | 28.48 | C |
| ATOM | 4098 | C | PRO | B | 225 | 20.783 | 33.821 | 76.883 | 1.00 | 28.48 | C |
| ATOM | 4099 | O | PRO | B | 225 | 20.528 | 33.760 | 75.683 | 1.00 | 28.48 | O |
| ATOM | 4100 | CB | PRO | B | 225 | 23.014 | 33.105 | 78.080 | 1.00 | 23.08 | C |
| ATOM | 4101 | CG | PRO | B | 225 | 22.796 | 33.413 | 79.479 | 1.00 | 23.08 | C |
| ATOM | 4102 | CD | PRO | B | 225 | 23.028 | 34.885 | 79.481 | 1.00 | 23.08 | C |
| ATOM | 4103 | N | GLU | B | 226 | 19.840 | 33.624 | 77.802 | 1.00 | 30.52 | N |
| ATOM | 4104 | CA | GLU | B | 226 | 18.461 | 33.294 | 77.408 | 1.00 | 30.52 | C |
| ATOM | 4105 | C | GLU | B | 226 | 17.892 | 34.283 | 76.380 | 1.00 | 30.52 | C |
| ATOM | 4106 | O | GLU | B | 226 | 17.102 | 33.899 | 75.523 | 1.00 | 30.52 | O |
| ATOM | 4107 | CB | GLU | B | 226 | 17.533 | 33.249 | 78.624 | 1.00 | 32.28 | C |
| ATOM | 4108 | CG | GLU | B | 226 | 17.508 | 34.535 | 79.428 | 1.00 | 32.28 | C |
| ATOM | 4109 | CD | GLU | B | 226 | 18.283 | 34.415 | 80.716 | 1.00 | 32.28 | C |
| ATOM | 4110 | OE1 | GLU | B | 226 | 19.417 | 33.905 | 80.680 | 1.00 | 32.28 | O |
| ATOM | 4111 | OE2 | GLU | B | 226 | 17.765 | 34.833 | 81.769 | 1.00 | 32.28 | O |
| ATOM | 4112 | N | LEU | B | 227 | 18.268 | 35.556 | 76.468 | 1.00 | 23.55 | N |
| ATOM | 4113 | CA | LEU | B | 227 | 17.787 | 36.531 | 75.489 | 1.00 | 23.55 | C |
| ATOM | 4114 | C | LEU | B | 227 | 18.331 | 36.190 | 74.096 | 1.00 | 23.55 | C |
| ATOM | 4115 | O | LEU | B | 227 | 17.621 | 36.292 | 73.095 | 1.00 | 23.55 | O |
| ATOM | 4116 | CB | LEU | B | 227 | 18.220 | 37.949 | 75.860 | 1.00 | 25.52 | C |
| ATOM | 4117 | CG | LEU | B | 227 | 17.671 | 38.519 | 77.164 | 1.00 | 25.52 | C |
| ATOM | 4118 | CD1 | LEU | B | 227 | 18.146 | 39.960 | 77.310 | 1.00 | 25.52 | C |
| ATOM | 4119 | CD2 | LEU | B | 227 | 16.154 | 38.452 | 77.170 | 1.00 | 25.52 | C |
| ATOM | 4120 | N | ILE | B | 228 | 19.595 | 35.789 | 74.036 | 1.00 | 23.90 | N |
| ATOM | 4121 | CA | ILE | B | 228 | 20.189 | 35.431 | 72.767 | 1.00 | 23.90 | C |
| ATOM | 4122 | C | ILE | B | 228 | 19.395 | 34.253 | 72.203 | 1.00 | 23.90 | C |
| ATOM | 4123 | O | ILE | B | 228 | 19.049 | 34.232 | 71.028 | 1.00 | 23.90 | O |
| ATOM | 4124 | CB | ILE | B | 228 | 21.676 | 35.055 | 72.937 | 1.00 | 17.80 | C |
| ATOM | 4125 | CG1 | ILE | B | 228 | 22.445 | 36.244 | 73.536 | 1.00 | 17.80 | C |
| ATOM | 4126 | CG2 | ILE | B | 228 | 22.262 | 34.678 | 71.598 | 1.00 | 17.80 | C |
| ATOM | 4127 | CD1 | ILE | B | 228 | 23.931 | 35.991 | 73.773 | 1.00 | 17.80 | C |
| ATOM | 4128 | N | PHE | B | 229 | 19.087 | 33.283 | 73.054 | 1.00 | 40.04 | N |
| ATOM | 4129 | CA | PHE | B | 229 | 18.324 | 32.126 | 72.615 | 1.00 | 40.04 | C |
| ATOM | 4130 | C | PHE | B | 229 | 16.868 | 32.439 | 72.304 | 1.00 | 40.04 | C |
| ATOM | 4131 | O | PHE | B | 229 | 16.080 | 31.527 | 72.073 | 1.00 | 40.04 | O |
| ATOM | 4132 | CB | PHE | B | 229 | 18.370 | 31.004 | 73.650 | 1.00 | 26.10 | C |
| ATOM | 4133 | CG | PHE | B | 229 | 19.666 | 30.255 | 73.676 | 1.00 | 26.10 | C |
| ATOM | 4134 | CD1 | PHE | B | 229 | 20.157 | 29.623 | 72.532 | 1.00 | 26.10 | C |
| ATOM | 4135 | CD2 | PHE | B | 229 | 20.393 | 30.166 | 74.845 | 1.00 | 26.10 | C |
| ATOM | 4136 | CE1 | PHE | B | 229 | 21.352 | 28.915 | 72.559 | 1.00 | 26.10 | C |
| ATOM | 4137 | CE2 | PHE | B | 229 | 21.593 | 29.457 | 74.884 | 1.00 | 26.10 | C |
| ATOM | 4138 | CZ | PHE | B | 229 | 22.075 | 28.837 | 73.754 | 1.00 | 26.10 | C |
| ATOM | 4139 | N | GLY | B | 230 | 16.493 | 33.712 | 72.311 | 1.00 | 35.01 | N |
| ATOM | 4140 | CA | GLY | B | 230 | 15.123 | 34.065 | 71.972 | 1.00 | 35.01 | C |
| ATOM | 4141 | C | GLY | B | 230 | 13.999 | 33.814 | 72.965 | 1.00 | 35.01 | C |
| ATOM | 4142 | O | GLY | B | 230 | 12.833 | 33.915 | 72.595 | 1.00 | 35.01 | O |
| ATOM | 4143 | N | ALA | B | 231 | 14.327 | 33.495 | 74.212 | 1.00 | 26.93 | N |
| ATOM | 4144 | CA | ALA | B | 231 | 13.311 | 33.265 | 75.235 | 1.00 | 26.93 | C |
| ATOM | 4145 | C | ALA | B | 231 | 12.386 | 34.471 | 75.355 | 1.00 | 26.93 | C |
| ATOM | 4146 | O | ALA | B | 231 | 12.765 | 35.589 | 75.021 | 1.00 | 26.93 | O |
| ATOM | 4147 | CB | ALA | B | 231 | 13.974 | 32.991 | 76.578 | 1.00 | 7.70 | C |
| ATOM | 4148 | N | THR | B | 232 | 11.176 | 34.248 | 75.848 | 1.00 | 31.17 | N |
| ATOM | 4149 | CA | THR | B | 232 | 10.208 | 35.327 | 75.998 | 1.00 | 31.17 | C |
| ATOM | 4150 | C | THR | B | 232 | 9.579 | 35.279 | 77.385 | 1.00 | 31.17 | C |

| | | | | | | | | | | | |
|---|---|---|---|---|---|---|---|---|---|---|---|
| ATOM | 4151 | O | THR | B | 232 | 8.714 | 36.095 | 77.733 | 1.00 | 31.17 | O |
| ATOM | 4152 | CB | THR | B | 232 | 9.112 | 35.196 | 74.948 | 1.00 | 36.16 | C |
| ATOM | 4153 | OG1 | THR | B | 232 | 8.775 | 33.809 | 74.811 | 1.00 | 36.16 | O |
| ATOM | 4154 | CG2 | THR | B | 232 | 9.581 | 35.740 | 73.603 | 1.00 | 36.16 | C |
| ATOM | 4155 | N | ASP | B | 233 | 10.034 | 34.312 | 78.172 | 1.00 | 28.99 | N |
| ATOM | 4156 | CA | ASP | B | 233 | 9.544 | 34.114 | 79.522 | 1.00 | 28.99 | C |
| ATOM | 4157 | C | ASP | B | 233 | 10.674 | 34.393 | 80.506 | 1.00 | 28.99 | C |
| ATOM | 4158 | O | ASP | B | 233 | 10.757 | 33.763 | 81.564 | 1.00 | 28.99 | O |
| ATOM | 4159 | CB | ASP | B | 233 | 9.064 | 32.677 | 79.678 | 1.00 | 34.28 | C |
| ATOM | 4160 | CG | ASP | B | 233 | 10.192 | 31.684 | 79.618 | 1.00 | 34.28 | C |
| ATOM | 4161 | OD1 | ASP | B | 233 | 11.262 | 32.011 | 79.053 | 1.00 | 34.28 | O |
| ATOM | 4162 | OD2 | ASP | B | 233 | 10.006 | 30.564 | 80.131 | 1.00 | 34.28 | O |
| ATOM | 4163 | N | TYR | B | 234 | 11.541 | 35.336 | 80.149 | 1.00 | 31.44 | N |
| ATOM | 4164 | CA | TYR | B | 234 | 12.668 | 35.693 | 80.996 | 1.00 | 31.44 | C |
| ATOM | 4165 | C | TYR | B | 234 | 12.240 | 36.504 | 82.211 | 1.00 | 31.44 | C |
| ATOM | 4166 | O | TYR | B | 234 | 11.190 | 37.135 | 82.206 | 1.00 | 31.44 | O |
| ATOM | 4167 | CB | TYR | B | 234 | 13.695 | 36.480 | 80.197 | 1.00 | 22.01 | C |
| ATOM | 4168 | CG | TYR | B | 234 | 13.144 | 37.722 | 79.545 | 1.00 | 22.01 | C |
| ATOM | 4169 | CD1 | TYR | B | 234 | 12.596 | 37.678 | 78.264 | 1.00 | 22.01 | C |
| ATOM | 4170 | CD2 | TYR | B | 234 | 13.212 | 38.956 | 80.184 | 1.00 | 22.01 | C |
| ATOM | 4171 | CE1 | TYR | B | 234 | 12.146 | 38.827 | 77.637 | 1.00 | 22.01 | C |
| ATOM | 4172 | CE2 | TYR | B | 234 | 12.757 | 40.112 | 79.559 | 1.00 | 22.01 | C |
| ATOM | 4173 | CZ | TYR | B | 234 | 12.228 | 40.036 | 78.288 | 1.00 | 22.01 | C |
| ATOM | 4174 | OH | TYR | B | 234 | 11.754 | 41.164 | 77.671 | 1.00 | 22.01 | O |
| ATOM | 4175 | N | THR | B | 235 | 13.065 | 36.486 | 83.252 | 1.00 | 26.32 | N |
| ATOM | 4176 | CA | THR | B | 235 | 12.774 | 37.211 | 84.482 | 1.00 | 26.32 | C |
| ATOM | 4177 | C | THR | B | 235 | 13.649 | 38.428 | 84.646 | 1.00 | 26.32 | C |
| ATOM | 4178 | O | THR | B | 235 | 14.346 | 38.840 | 83.729 | 1.00 | 26.32 | O |
| ATOM | 4179 | CB | THR | B | 235 | 12.981 | 36.339 | 85.722 | 1.00 | 18.24 | C |
| ATOM | 4180 | OG1 | THR | B | 235 | 14.364 | 35.999 | 85.849 | 1.00 | 18.24 | O |
| ATOM | 4181 | CG2 | THR | B | 235 | 12.158 | 35.081 | 85.617 | 1.00 | 18.24 | C |
| ATOM | 4182 | N | SER | B | 236 | 13.614 | 38.998 | 85.837 | 1.00 | 31.43 | N |
| ATOM | 4183 | CA | SER | B | 236 | 14.402 | 40.175 | 86.112 | 1.00 | 31.43 | C |
| ATOM | 4184 | C | SER | B | 236 | 15.882 | 39.909 | 86.198 | 1.00 | 31.43 | C |
| ATOM | 4185 | O | SER | B | 236 | 16.676 | 40.822 | 86.017 | 1.00 | 31.43 | O |
| ATOM | 4186 | CB | SER | B | 236 | 13.924 | 40.818 | 87.397 | 1.00 | 49.49 | C |
| ATOM | 4187 | OG | SER | B | 236 | 12.705 | 41.485 | 87.147 | 1.00 | 49.49 | O |
| ATOM | 4188 | N | SER | B | 237 | 16.256 | 38.664 | 86.478 | 1.00 | 30.23 | N |
| ATOM | 4189 | CA | SER | B | 237 | 17.650 | 38.274 | 86.631 | 1.00 | 30.23 | C |
| ATOM | 4190 | C | SER | B | 237 | 18.473 | 38.685 | 85.409 | 1.00 | 30.23 | C |
| ATOM | 4191 | O | SER | B | 237 | 19.696 | 38.643 | 85.400 | 1.00 | 30.23 | O |
| ATOM | 4192 | CB | SER | B | 237 | 17.703 | 36.757 | 86.813 | 1.00 | 31.67 | C |
| ATOM | 4193 | OG | SER | B | 237 | 16.897 | 36.134 | 85.813 | 1.00 | 31.67 | O |
| ATOM | 4194 | N | ILE | B | 238 | 17.750 | 39.055 | 84.335 | 1.00 | 19.62 | N |
| ATOM | 4195 | CA | ILE | B | 238 | 18.435 | 39.494 | 83.126 | 1.00 | 19.62 | C |
| ATOM | 4196 | C | ILE | B | 238 | 19.168 | 40.819 | 83.347 | 1.00 | 19.62 | C |
| ATOM | 4197 | O | ILE | B | 238 | 20.184 | 41.117 | 82.732 | 1.00 | 19.62 | O |
| ATOM | 4198 | CB | ILE | B | 238 | 17.396 | 39.636 | 82.013 | 1.00 | 27.89 | C |
| ATOM | 4199 | CG1 | ILE | B | 238 | 16.362 | 40.703 | 82.379 | 1.00 | 27.89 | C |
| ATOM | 4200 | CG2 | ILE | B | 238 | 16.651 | 38.301 | 81.826 | 1.00 | 27.89 | C |
| ATOM | 4201 | CD1 | ILE | B | 238 | 15.546 | 41.157 | 81.169 | 0.00 | 27.89 | C |
| ATOM | 4202 | N | ASP | B | 239 | 18.589 | 41.646 | 84.238 | 1.00 | 28.07 | N |
| ATOM | 4203 | CA | ASP | B | 239 | 19.275 | 42.870 | 84.633 | 1.00 | 28.07 | C |
| ATOM | 4204 | C | ASP | B | 239 | 20.539 | 42.545 | 85.430 | 1.00 | 28.07 | C |
| ATOM | 4205 | O | ASP | B | 239 | 21.525 | 43.271 | 85.427 | 1.00 | 28.07 | O |
| ATOM | 4206 | CB | ASP | B | 239 | 18.318 | 43.689 | 85.501 | 1.00 | 24.72 | C |
| ATOM | 4207 | CG | ASP | B | 239 | 17.384 | 44.489 | 84.607 | 1.00 | 24.72 | C |
| ATOM | 4208 | OD1 | ASP | B | 239 | 17.682 | 44.598 | 83.418 | 1.00 | 24.72 | O |
| ATOM | 4209 | OD2 | ASP | B | 239 | 16.379 | 44.988 | 85.102 | 1.00 | 24.72 | O |
| ATOM | 4210 | N | VAL | B | 240 | 20.463 | 41.417 | 86.164 | 1.00 | 19.83 | N |
| ATOM | 4211 | CA | VAL | B | 240 | 21.604 | 40.983 | 86.959 | 1.00 | 19.83 | C |
| ATOM | 4212 | C | VAL | B | 240 | 22.764 | 40.533 | 86.069 | 1.00 | 19.83 | C |

| | | | | | | | | | | | |
|---|---|---|---|---|---|---|---|---|---|---|---|
| ATOM | 4213 | O | VAL | B | 240 | 23.911 | 40.928 | 86.237 | 1.00 | 19.83 | O |
| ATOM | 4214 | CB | VAL | B | 240 | 21.149 | 39.821 | 87.844 | 1.00 | 18.12 | C |
| ATOM | 4215 | CG1 | VAL | B | 240 | 22.353 | 39.195 | 88.543 | 1.00 | 18.12 | C |
| ATOM | 4216 | CG2 | VAL | B | 240 | 20.166 | 40.314 | 88.888 | 1.00 | 18.12 | C |
| ATOM | 4217 | N | TRP | B | 241 | 22.437 | 39.638 | 85.119 | 1.00 | 21.79 | N |
| ATOM | 4218 | CA | TRP | B | 241 | 23.458 | 39.179 | 84.187 | 1.00 | 21.79 | C |
| ATOM | 4219 | C | TRP | B | 241 | 24.140 | 40.359 | 83.494 | 1.00 | 21.79 | C |
| ATOM | 4220 | O | TRP | B | 241 | 25.346 | 40.391 | 83.291 | 1.00 | 21.79 | O |
| ATOM | 4221 | CB | TRP | B | 241 | 22.786 | 38.281 | 83.147 | 1.00 | 24.64 | C |
| ATOM | 4222 | CG | TRP | B | 241 | 23.715 | 38.010 | 82.026 | 1.00 | 24.64 | C |
| ATOM | 4223 | CD1 | TRP | B | 241 | 23.990 | 38.859 | 80.931 | 1.00 | 24.64 | C |
| ATOM | 4224 | CD2 | TRP | B | 241 | 24.504 | 36.812 | 81.827 | 1.00 | 24.64 | C |
| ATOM | 4225 | NE1 | TRP | B | 241 | 24.878 | 38.313 | 80.059 | 1.00 | 24.64 | N |
| ATOM | 4226 | CE2 | TRP | B | 241 | 25.228 | 36.984 | 80.620 | 1.00 | 24.64 | C |
| ATOM | 4227 | CE3 | TRP | B | 241 | 24.662 | 35.637 | 82.556 | 1.00 | 24.64 | C |
| ATOM | 4228 | CZ2 | TRP | B | 241 | 26.082 | 35.986 | 80.177 | 1.00 | 24.64 | C |
| ATOM | 4229 | CZ3 | TRP | B | 241 | 25.515 | 34.639 | 82.113 | 1.00 | 24.64 | C |
| ATOM | 4230 | CH2 | TRP | B | 241 | 26.229 | 34.817 | 80.914 | 1.00 | 24.64 | C |
| ATOM | 4231 | N | SER | B | 242 | 23.306 | 41.334 | 83.087 | 1.00 | 16.88 | N |
| ATOM | 4232 | CA | SER | B | 242 | 23.850 | 42.531 | 82.456 | 1.00 | 16.88 | C |
| ATOM | 4233 | C | SER | B | 242 | 24.794 | 43.276 | 83.400 | 1.00 | 16.88 | C |
| ATOM | 4234 | O | SER | B | 242 | 25.876 | 43.708 | 83.025 | 1.00 | 16.88 | O |
| ATOM | 4235 | CB | SER | B | 242 | 22.681 | 43.440 | 82.074 | 1.00 | 18.23 | C |
| ATOM | 4236 | OG | SER | B | 242 | 22.036 | 42.918 | 80.912 | 1.00 | 18.23 | O |
| ATOM | 4237 | N | ALA | B | 243 | 24.363 | 43.386 | 84.652 | 1.00 | 29.48 | N |
| ATOM | 4238 | CA | ALA | B | 243 | 25.178 | 44.022 | 85.677 | 1.00 | 29.48 | C |
| ATOM | 4239 | C | ALA | B | 243 | 26.497 | 43.267 | 85.783 | 1.00 | 29.48 | C |
| ATOM | 4240 | O | ALA | B | 243 | 27.556 | 43.875 | 85.901 | 1.00 | 29.48 | O |
| ATOM | 4241 | CB | ALA | B | 243 | 24.462 | 44.010 | 86.997 | 1.00 | 12.02 | C |
| ATOM | 4242 | N | GLY | B | 244 | 26.435 | 41.941 | 85.733 | 1.00 | 26.93 | N |
| ATOM | 4243 | CA | GLY | B | 244 | 27.651 | 41.155 | 85.798 | 1.00 | 26.93 | C |
| ATOM | 4244 | C | GLY | B | 244 | 28.591 | 41.487 | 84.651 | 1.00 | 26.93 | C |
| ATOM | 4245 | O | GLY | B | 244 | 29.805 | 41.495 | 84.822 | 1.00 | 26.93 | O |
| ATOM | 4246 | N | CYS | B | 245 | 28.041 | 41.755 | 83.475 | 1.00 | 22.38 | N |
| ATOM | 4247 | CA | CYS | B | 245 | 28.865 | 42.104 | 82.320 | 1.00 | 22.38 | C |
| ATOM | 4248 | C | CYS | B | 245 | 29.507 | 43.478 | 82.495 | 1.00 | 22.38 | C |
| ATOM | 4249 | O | CYS | B | 245 | 30.571 | 43.745 | 81.955 | 1.00 | 22.38 | O |
| ATOM | 4250 | CB | CYS | B | 245 | 28.035 | 42.098 | 81.029 | 1.00 | 29.42 | C |
| ATOM | 4251 | SG | CYS | B | 245 | 27.454 | 40.481 | 80.496 | 1.00 | 29.42 | S |
| ATOM | 4252 | N | VAL | B | 246 | 28.850 | 44.360 | 83.238 | 1.00 | 21.80 | N |
| ATOM | 4253 | CA | VAL | B | 246 | 29.410 | 45.677 | 83.460 | 1.00 | 21.80 | C |
| ATOM | 4254 | C | VAL | B | 246 | 30.540 | 45.576 | 84.469 | 1.00 | 21.80 | C |
| ATOM | 4255 | O | VAL | B | 246 | 31.546 | 46.257 | 84.329 | 1.00 | 21.80 | O |
| ATOM | 4256 | CB | VAL | B | 246 | 28.363 | 46.666 | 83.969 | 1.00 | 17.88 | C |
| ATOM | 4257 | CG1 | VAL | B | 246 | 29.040 | 47.982 | 84.338 | 1.00 | 17.88 | C |
| ATOM | 4258 | CG2 | VAL | B | 246 | 27.305 | 46.888 | 82.904 | 1.00 | 17.88 | C |
| ATOM | 4259 | N | LEU | B | 247 | 30.376 | 44.723 | 85.480 | 1.00 | 25.71 | N |
| ATOM | 4260 | CA | LEU | B | 247 | 31.412 | 44.524 | 86.491 | 1.00 | 25.71 | C |
| ATOM | 4261 | C | LEU | B | 247 | 32.645 | 43.925 | 85.832 | 1.00 | 25.71 | C |
| ATOM | 4262 | O | LEU | B | 247 | 33.760 | 44.382 | 86.042 | 1.00 | 25.71 | O |
| ATOM | 4263 | CB | LEU | B | 247 | 30.919 | 43.588 | 87.598 | 1.00 | 19.87 | C |
| ATOM | 4264 | CG | LEU | B | 247 | 31.979 | 42.855 | 88.428 | 1.00 | 19.87 | C |
| ATOM | 4265 | CD1 | LEU | B | 247 | 32.758 | 43.811 | 89.310 | 1.00 | 19.87 | C |
| ATOM | 4266 | CD2 | LEU | B | 247 | 31.294 | 41.808 | 89.259 | 1.00 | 19.87 | C |
| ATOM | 4267 | N | ALA | B | 248 | 32.438 | 42.890 | 85.033 | 1.00 | 32.57 | N |
| ATOM | 4268 | CA | ALA | B | 248 | 33.543 | 42.253 | 84.350 | 1.00 | 32.57 | C |
| ATOM | 4269 | C | ALA | B | 248 | 34.275 | 43.276 | 83.477 | 1.00 | 32.57 | C |
| ATOM | 4270 | O | ALA | B | 248 | 35.502 | 43.409 | 83.558 | 1.00 | 32.57 | O |
| ATOM | 4271 | CB | ALA | B | 248 | 33.031 | 41.102 | 83.506 | 1.00 | 22.47 | C |
| ATOM | 4272 | N | GLU | B | 249 | 33.525 | 44.010 | 82.656 | 1.00 | 30.07 | N |
| ATOM | 4273 | CA | GLU | B | 249 | 34.115 | 45.015 | 81.773 | 1.00 | 30.07 | C |
| ATOM | 4274 | C | GLU | B | 249 | 34.978 | 46.014 | 82.542 | 1.00 | 30.07 | C |

| | | | | | | | | | | | |
|---|---|---|---|---|---|---|---|---|---|---|---|
| ATOM | 4275 | O | GLU | B | 249 | 36.026 | 46.436 | 82.045 | 1.00 | 30.07 | O |
| ATOM | 4276 | CB | GLU | B | 249 | 33.020 | 45.766 | 80.995 | 1.00 | 23.84 | C |
| ATOM | 4277 | CG | GLU | B | 249 | 33.542 | 46.725 | 79.924 | 1.00 | 23.84 | C |
| ATOM | 4278 | CD | GLU | B | 249 | 32.492 | 47.106 | 78.877 | 1.00 | 23.84 | C |
| ATOM | 4279 | OE1 | GLU | B | 249 | 31.435 | 46.441 | 78.798 | 1.00 | 23.84 | O |
| ATOM | 4280 | OE2 | GLU | B | 249 | 32.732 | 48.064 | 78.115 | 1.00 | 23.84 | O |
| ATOM | 4281 | N | LEU | B | 250 | 34.546 | 46.393 | 83.747 | 1.00 | 27.10 | N |
| ATOM | 4282 | CA | LEU | B | 250 | 35.314 | 47.345 | 84.539 | 1.00 | 27.10 | C |
| ATOM | 4283 | C | LEU | B | 250 | 36.603 | 46.720 | 85.010 | 1.00 | 27.10 | C |
| ATOM | 4284 | O | LEU | B | 250 | 37.619 | 47.393 | 85.130 | 1.00 | 27.10 | O |
| ATOM | 4285 | CB | LEU | B | 250 | 34.521 | 47.836 | 85.743 | 1.00 | 20.94 | C |
| ATOM | 4286 | CG | LEU | B | 250 | 33.316 | 48.691 | 85.377 | 1.00 | 20.94 | C |
| ATOM | 4287 | CD1 | LEU | B | 250 | 32.576 | 49.088 | 86.638 | 1.00 | 20.94 | C |
| ATOM | 4288 | CD2 | LEU | B | 250 | 33.783 | 49.908 | 84.576 | 1.00 | 20.94 | C |
| ATOM | 4289 | N | LEU | B | 251 | 36.563 | 45.424 | 85.272 | 1.00 | 33.59 | N |
| ATOM | 4290 | CA | LEU | B | 251 | 37.750 | 44.735 | 85.712 | 1.00 | 33.59 | C |
| ATOM | 4291 | C | LEU | B | 251 | 38.720 | 44.504 | 84.537 | 1.00 | 33.59 | C |
| ATOM | 4292 | O | LEU | B | 251 | 39.932 | 44.647 | 84.685 | 1.00 | 33.59 | O |
| ATOM | 4293 | CB | LEU | B | 251 | 37.364 | 43.405 | 86.350 | 1.00 | 20.83 | C |
| ATOM | 4294 | CG | LEU | B | 251 | 36.515 | 43.441 | 87.618 | 1.00 | 20.83 | C |
| ATOM | 4295 | CD1 | LEU | B | 251 | 36.190 | 42.019 | 88.020 | 1.00 | 20.83 | C |
| ATOM | 4296 | CD2 | LEU | B | 251 | 37.253 | 44.145 | 88.745 | 1.00 | 20.83 | C |
| ATOM | 4297 | N | LEU | B | 252 | 38.182 | 44.188 | 83.363 | 1.00 | 29.76 | N |
| ATOM | 4298 | CA | LEU | B | 252 | 39.003 | 43.900 | 82.189 | 1.00 | 29.76 | C |
| ATOM | 4299 | C | LEU | B | 252 | 39.405 | 45.066 | 81.298 | 1.00 | 29.76 | C |
| ATOM | 4300 | O | LEU | B | 252 | 40.381 | 44.964 | 80.556 | 1.00 | 29.76 | O |
| ATOM | 4301 | CB | LEU | B | 252 | 38.290 | 42.872 | 81.311 | 1.00 | 41.19 | C |
| ATOM | 4302 | CG | LEU | B | 252 | 38.095 | 41.469 | 81.874 | 1.00 | 41.19 | C |
| ATOM | 4303 | CD1 | LEU | B | 252 | 36.941 | 40.803 | 81.149 | 1.00 | 41.19 | C |
| ATOM | 4304 | CD2 | LEU | B | 252 | 39.391 | 40.673 | 81.743 | 1.00 | 41.19 | C |
| ATOM | 4305 | N | GLY | B | 253 | 38.649 | 46.155 | 81.328 | 1.00 | 21.90 | N |
| ATOM | 4306 | CA | GLY | B | 253 | 38.982 | 47.272 | 80.470 | 1.00 | 21.90 | C |
| ATOM | 4307 | C | GLY | B | 253 | 38.396 | 47.086 | 79.082 | 1.00 | 21.90 | C |
| ATOM | 4308 | O | GLY | B | 253 | 38.745 | 47.811 | 78.149 | 1.00 | 21.90 | O |
| ATOM | 4309 | N | GLN | B | 254 | 37.513 | 46.101 | 78.935 | 1.00 | 27.16 | N |
| ATOM | 4310 | CA | GLN | B | 254 | 36.855 | 45.840 | 77.659 | 1.00 | 27.16 | C |
| ATOM | 4311 | C | GLN | B | 254 | 35.671 | 44.897 | 77.845 | 1.00 | 27.16 | C |
| ATOM | 4312 | O | GLN | B | 254 | 35.587 | 44.171 | 78.827 | 1.00 | 27.16 | O |
| ATOM | 4313 | CB | GLN | B | 254 | 37.847 | 45.270 | 76.635 | 1.00 | 44.50 | C |
| ATOM | 4314 | CG | GLN | B | 254 | 38.381 | 43.891 | 76.948 | 1.00 | 44.50 | C |
| ATOM | 4315 | CD | GLN | B | 254 | 39.390 | 43.417 | 75.920 | 1.00 | 44.50 | C |
| ATOM | 4316 | OE1 | GLN | B | 254 | 39.790 | 42.257 | 75.918 | 1.00 | 44.50 | O |
| ATOM | 4317 | NE2 | GLN | B | 254 | 39.808 | 44.318 | 75.038 | 1.00 | 44.50 | N |
| ATOM | 4318 | N | PRO | B | 255 | 34.727 | 44.905 | 76.901 | 1.00 | 40.09 | N |
| ATOM | 4319 | CA | PRO | B | 255 | 33.574 | 44.017 | 77.052 | 1.00 | 40.09 | C |
| ATOM | 4320 | C | PRO | B | 255 | 34.052 | 42.591 | 77.256 | 1.00 | 40.09 | C |
| ATOM | 4321 | O | PRO | B | 255 | 35.067 | 42.206 | 76.692 | 1.00 | 40.09 | O |
| ATOM | 4322 | CB | PRO | B | 255 | 32.840 | 44.186 | 75.724 | 1.00 | 24.34 | C |
| ATOM | 4323 | CG | PRO | B | 255 | 33.211 | 45.554 | 75.302 | 1.00 | 24.34 | C |
| ATOM | 4324 | CD | PRO | B | 255 | 34.678 | 45.604 | 75.611 | 1.00 | 24.34 | C |
| ATOM | 4325 | N | ILE | B | 256 | 33.343 | 41.802 | 78.050 | 1.00 | 32.18 | N |
| ATOM | 4326 | CA | ILE | B | 256 | 33.771 | 40.425 | 78.244 | 1.00 | 32.18 | C |
| ATOM | 4327 | C | ILE | B | 256 | 33.139 | 39.458 | 77.244 | 1.00 | 32.18 | C |
| ATOM | 4328 | O | ILE | B | 256 | 33.733 | 38.443 | 76.918 | 1.00 | 32.18 | O |
| ATOM | 4329 | CB | ILE | B | 256 | 33.476 | 39.922 | 79.678 | 1.00 | 27.08 | C |
| ATOM | 4330 | CG1 | ILE | B | 256 | 33.865 | 38.445 | 79.793 | 1.00 | 27.08 | C |
| ATOM | 4331 | CG2 | ILE | B | 256 | 32.017 | 40.124 | 80.011 | 1.00 | 27.08 | C |
| ATOM | 4332 | CD1 | ILE | B | 256 | 33.659 | 37.832 | 81.170 | 1.00 | 27.08 | C |
| ATOM | 4333 | N | PHE | B | 257 | 31.946 | 39.771 | 76.750 | 1.00 | 27.33 | N |
| ATOM | 4334 | CA | PHE | B | 257 | 31.257 | 38.891 | 75.800 | 1.00 | 27.33 | C |
| ATOM | 4335 | C | PHE | B | 257 | 30.880 | 39.592 | 74.504 | 1.00 | 27.33 | C |
| ATOM | 4336 | O | PHE | B | 257 | 29.701 | 39.733 | 74.197 | 1.00 | 27.33 | O |

| | | | | | | | | | | | |
|---|---|---|---|---|---|---|---|---|---|---|---|
| ATOM | 4337 | CB | PHE | B | 257 | 29.974 | 38.322 | 76.418 | 1.00 | 25.37 | C |
| ATOM | 4338 | CG | PHE | B | 257 | 30.198 | 37.503 | 77.639 | 1.00 | 25.37 | C |
| ATOM | 4339 | CD1 | PHE | B | 257 | 31.077 | 36.430 | 77.620 | 1.00 | 25.37 | C |
| ATOM | 4340 | CD2 | PHE | B | 257 | 29.508 | 37.780 | 78.806 | 1.00 | 25.37 | C |
| ATOM | 4341 | CE1 | PHE | B | 257 | 31.262 | 35.629 | 78.755 | 1.00 | 25.37 | C |
| ATOM | 4342 | CE2 | PHE | B | 257 | 29.681 | 36.992 | 79.949 | 1.00 | 25.37 | C |
| ATOM | 4343 | CZ | PHE | B | 257 | 30.562 | 35.913 | 79.921 | 1.00 | 25.37 | C |
| ATOM | 4344 | N | PRO | B | 258 | 31.874 | 40.039 | 73.726 | 1.00 | 27.82 | N |
| ATOM | 4345 | CA | PRO | B | 258 | 31.596 | 40.723 | 72.464 | 1.00 | 27.82 | C |
| ATOM | 4346 | C | PRO | B | 258 | 31.270 | 39.739 | 71.348 | 1.00 | 27.82 | C |
| ATOM | 4347 | O | PRO | B | 258 | 31.434 | 38.517 | 71.502 | 1.00 | 27.82 | O |
| ATOM | 4348 | CB | PRO | B | 258 | 32.888 | 41.465 | 72.199 | 1.00 | 18.96 | C |
| ATOM | 4349 | CG | PRO | B | 258 | 33.892 | 40.478 | 72.646 | 1.00 | 18.96 | C |
| ATOM | 4350 | CD | PRO | B | 258 | 33.326 | 40.006 | 73.977 | 1.00 | 18.96 | C |
| ATOM | 4351 | N | GLY | B | 259 | 30.820 | 40.291 | 70.226 | 1.00 | 32.91 | N |
| ATOM | 4352 | CA | GLY | B | 259 | 30.470 | 39.494 | 69.065 | 1.00 | 32.91 | C |
| ATOM | 4353 | C | GLY | B | 259 | 29.362 | 40.156 | 68.273 | 1.00 | 32.91 | C |
| ATOM | 4354 | O | GLY | B | 259 | 28.355 | 40.555 | 68.843 | 1.00 | 32.91 | O |
| ATOM | 4355 | N | ASP | B | 260 | 29.530 | 40.285 | 66.963 | 1.00 | 29.78 | N |
| ATOM | 4356 | CA | ASP | B | 260 | 28.498 | 40.916 | 66.152 | 1.00 | 29.78 | C |
| ATOM | 4357 | C | ASP | B | 260 | 27.260 | 40.038 | 65.948 | 1.00 | 29.78 | C |
| ATOM | 4358 | O | ASP | B | 260 | 26.215 | 40.527 | 65.523 | 1.00 | 29.78 | O |
| ATOM | 4359 | CB | ASP | B | 260 | 29.066 | 41.337 | 64.797 | 1.00 | 55.97 | C |
| ATOM | 4360 | CG | ASP | B | 260 | 30.193 | 42.338 | 64.931 | 1.00 | 55.97 | C |
| ATOM | 4361 | OD1 | ASP | B | 260 | 30.044 | 43.308 | 65.714 | 1.00 | 55.97 | O |
| ATOM | 4362 | OD2 | ASP | B | 260 | 31.230 | 42.161 | 64.247 | 1.00 | 55.97 | O |
| ATOM | 4363 | N | SER | B | 261 | 27.386 | 38.747 | 66.243 | 1.00 | 27.61 | N |
| ATOM | 4364 | CA | SER | B | 261 | 26.267 | 37.823 | 66.108 | 1.00 | 27.61 | C |
| ATOM | 4365 | C | SER | B | 261 | 26.016 | 37.098 | 67.437 | 1.00 | 27.61 | C |
| ATOM | 4366 | O | SER | B | 261 | 26.881 | 37.072 | 68.311 | 1.00 | 27.61 | O |
| ATOM | 4367 | CB | SER | B | 261 | 26.557 | 36.790 | 65.023 | 1.00 | 21.12 | C |
| ATOM | 4368 | OG | SER | B | 261 | 27.431 | 35.784 | 65.504 | 1.00 | 21.12 | O |
| ATOM | 4369 | N | GLY | B | 262 | 24.829 | 36.519 | 67.585 | 1.00 | 31.64 | N |
| ATOM | 4370 | CA | GLY | B | 262 | 24.525 | 35.784 | 68.798 | 1.00 | 31.64 | C |
| ATOM | 4371 | C | GLY | B | 262 | 25.488 | 34.624 | 68.950 | 1.00 | 31.64 | C |
| ATOM | 4372 | O | GLY | B | 262 | 25.879 | 34.265 | 70.060 | 1.00 | 31.64 | O |
| ATOM | 4373 | N | VAL | B | 263 | 25.878 | 34.035 | 67.823 | 1.00 | 31.86 | N |
| ATOM | 4374 | CA | VAL | B | 263 | 26.809 | 32.913 | 67.819 | 1.00 | 31.86 | C |
| ATOM | 4375 | C | VAL | B | 263 | 28.136 | 33.311 | 68.443 | 1.00 | 31.86 | C |
| ATOM | 4376 | O | VAL | B | 263 | 28.612 | 32.669 | 69.380 | 1.00 | 31.86 | O |
| ATOM | 4377 | CB | VAL | B | 263 | 27.089 | 32.414 | 66.389 | 1.00 | 43.99 | C |
| ATOM | 4378 | CG1 | VAL | B | 263 | 28.138 | 31.299 | 66.419 | 1.00 | 43.99 | C |
| ATOM | 4379 | CG2 | VAL | B | 263 | 25.826 | 31.904 | 65.773 | 1.00 | 43.99 | C |
| ATOM | 4380 | N | ASP | B | 264 | 28.742 | 34.360 | 67.905 | 1.00 | 33.92 | N |
| ATOM | 4381 | CA | ASP | B | 264 | 30.011 | 34.816 | 68.441 | 1.00 | 33.92 | C |
| ATOM | 4382 | C | ASP | B | 264 | 29.872 | 35.056 | 69.940 | 1.00 | 33.92 | C |
| ATOM | 4383 | O | ASP | B | 264 | 30.636 | 34.511 | 70.742 | 1.00 | 33.92 | O |
| ATOM | 4384 | CB | ASP | B | 264 | 30.458 | 36.100 | 67.747 | 1.00 | 58.74 | C |
| ATOM | 4385 | CG | ASP | B | 264 | 30.641 | 35.923 | 66.251 | 1.00 | 58.74 | C |
| ATOM | 4386 | OD1 | ASP | B | 264 | 31.049 | 34.827 | 65.815 | 1.00 | 58.74 | O |
| ATOM | 4387 | OD2 | ASP | B | 264 | 30.393 | 36.892 | 65.506 | 1.00 | 58.74 | O |
| ATOM | 4388 | N | GLN | B | 265 | 28.887 | 35.866 | 70.309 | 1.00 | 28.36 | N |
| ATOM | 4389 | CA | GLN | B | 265 | 28.634 | 36.166 | 71.707 | 1.00 | 28.36 | C |
| ATOM | 4390 | C | GLN | B | 265 | 28.504 | 34.876 | 72.486 | 1.00 | 28.36 | C |
| ATOM | 4391 | O | GLN | B | 265 | 29.003 | 34.764 | 73.593 | 1.00 | 28.36 | O |
| ATOM | 4392 | CB | GLN | B | 265 | 27.358 | 36.989 | 71.857 | 1.00 | 26.30 | C |
| ATOM | 4393 | CG | GLN | B | 265 | 27.451 | 38.354 | 71.233 | 1.00 | 26.30 | C |
| ATOM | 4394 | CD | GLN | B | 265 | 26.205 | 39.162 | 71.438 | 1.00 | 26.30 | C |
| ATOM | 4395 | OE1 | GLN | B | 265 | 25.103 | 38.706 | 71.141 | 1.00 | 26.30 | O |
| ATOM | 4396 | NE2 | GLN | B | 265 | 26.364 | 40.377 | 71.949 | 0.00 | 26.30 | N |
| ATOM | 4397 | N | LEU | B | 266 | 27.840 | 33.898 | 71.894 | 1.00 | 28.75 | N |
| ATOM | 4398 | CA | LEU | B | 266 | 27.657 | 32.613 | 72.543 | 1.00 | 28.75 | C |

| | | | | | | | | | | | |
|---|---|---|---|---|---|---|---|---|---|---|---|
| ATOM | 4399 | C | LEU | B | 266 | 28.976 | 31.858 | 72.719 | 1.00 | 28.75 | C |
| ATOM | 4400 | O | LEU | B | 266 | 29.234 | 31.308 | 73.781 | 1.00 | 28.75 | O |
| ATOM | 4401 | CB | LEU | B | 266 | 26.662 | 31.774 | 71.744 | 1.00 | 28.63 | C |
| ATOM | 4402 | CG | LEU | B | 266 | 25.330 | 31.442 | 72.413 | 1.00 | 28.63 | C |
| ATOM | 4403 | CD1 | LEU | B | 266 | 24.818 | 32.618 | 73.192 | 1.00 | 28.63 | C |
| ATOM | 4404 | CD2 | LEU | B | 266 | 24.329 | 31.035 | 71.353 | 1.00 | 28.63 | C |
| ATOM | 4405 | N | VAL | B | 267 | 29.823 | 31.824 | 71.695 | 1.00 | 38.65 | N |
| ATOM | 4406 | CA | VAL | B | 267 | 31.091 | 31.110 | 71.853 | 1.00 | 38.65 | C |
| ATOM | 4407 | C | VAL | B | 267 | 31.938 | 31.841 | 72.886 | 1.00 | 38.65 | C |
| ATOM | 4408 | O | VAL | B | 267 | 32.723 | 31.226 | 73.603 | 1.00 | 38.65 | O |
| ATOM | 4409 | CB | VAL | B | 267 | 31.891 | 30.980 | 70.513 | 1.00 | 39.88 | C |
| ATOM | 4410 | CG1 | VAL | B | 267 | 31.035 | 30.323 | 69.480 | 1.00 | 39.88 | C |
| ATOM | 4411 | CG2 | VAL | B | 267 | 32.355 | 32.324 | 70.014 | 1.00 | 39.88 | C |
| ATOM | 4412 | N | GLU | B | 268 | 31.762 | 33.156 | 72.963 | 1.00 | 36.51 | N |
| ATOM | 4413 | CA | GLU | B | 268 | 32.499 | 33.958 | 73.922 | 1.00 | 36.51 | C |
| ATOM | 4414 | C | GLU | B | 268 | 32.032 | 33.598 | 75.335 | 1.00 | 36.51 | C |
| ATOM | 4415 | O | GLU | B | 268 | 32.842 | 33.465 | 76.250 | 1.00 | 36.51 | O |
| ATOM | 4416 | CB | GLU | B | 268 | 32.268 | 35.450 | 73.643 | 1.00 | 54.53 | C |
| ATOM | 4417 | CG | GLU | B | 268 | 33.548 | 36.277 | 73.557 | 1.00 | 54.53 | C |
| ATOM | 4418 | CD | GLU | B | 268 | 34.549 | 35.674 | 72.592 | 1.00 | 54.53 | C |
| ATOM | 4419 | OE1 | GLU | B | 268 | 34.132 | 35.359 | 71.456 | 1.00 | 54.53 | O |
| ATOM | 4420 | OE2 | GLU | B | 268 | 35.738 | 35.512 | 72.957 | 1.00 | 54.53 | O |
| ATOM | 4421 | N | ILE | B | 269 | 30.724 | 33.431 | 75.507 | 1.00 | 36.11 | N |
| ATOM | 4422 | CA | ILE | B | 269 | 30.169 | 33.076 | 76.807 | 1.00 | 36.11 | C |
| ATOM | 4423 | C | ILE | B | 269 | 30.553 | 31.657 | 77.202 | 1.00 | 36.11 | C |
| ATOM | 4424 | O | ILE | B | 269 | 30.924 | 31.393 | 78.353 | 1.00 | 36.11 | O |
| ATOM | 4425 | CB | ILE | B | 269 | 28.638 | 33.169 | 76.811 | 1.00 | 18.31 | C |
| ATOM | 4426 | CG1 | ILE | B | 269 | 28.204 | 34.625 | 76.758 | 1.00 | 18.31 | C |
| ATOM | 4427 | CG2 | ILE | B | 269 | 28.081 | 32.524 | 78.060 | 1.00 | 18.31 | C |
| ATOM | 4428 | CD1 | ILE | B | 269 | 26.728 | 34.796 | 76.506 | 1.00 | 18.31 | C |
| ATOM | 4429 | N | ILE | B | 270 | 30.447 | 30.744 | 76.244 | 1.00 | 33.52 | N |
| ATOM | 4430 | CA | ILE | B | 270 | 30.780 | 29.354 | 76.483 | 1.00 | 33.52 | C |
| ATOM | 4431 | C | ILE | B | 270 | 32.253 | 29.247 | 76.834 | 1.00 | 33.52 | C |
| ATOM | 4432 | O | ILE | B | 270 | 32.630 | 28.466 | 77.702 | 1.00 | 33.52 | O |
| ATOM | 4433 | CB | ILE | B | 270 | 30.484 | 28.486 | 75.244 | 1.00 | 28.24 | C |
| ATOM | 4434 | CG1 | ILE | B | 270 | 28.987 | 28.549 | 74.915 | 1.00 | 28.24 | C |
| ATOM | 4435 | CG2 | ILE | B | 270 | 30.925 | 27.048 | 75.503 | 1.00 | 28.24 | C |
| ATOM | 4436 | CD1 | ILE | B | 270 | 28.593 | 27.813 | 73.666 | 1.00 | 28.24 | C |
| ATOM | 4437 | N | LYS | B | 271 | 33.083 | 30.041 | 76.171 | 1.00 | 38.26 | N |
| ATOM | 4438 | CA | LYS | B | 271 | 34.509 | 30.010 | 76.456 | 1.00 | 38.26 | C |
| ATOM | 4439 | C | LYS | B | 271 | 34.784 | 30.135 | 77.957 | 1.00 | 38.26 | C |
| ATOM | 4440 | O | LYS | B | 271 | 35.520 | 29.324 | 78.535 | 1.00 | 38.26 | O |
| ATOM | 4441 | CB | LYS | B | 271 | 35.245 | 31.129 | 75.707 | 1.00 | 53.65 | C |
| ATOM | 4442 | CG | LYS | B | 271 | 35.912 | 30.693 | 74.408 | 1.00 | 53.65 | C |
| ATOM | 4443 | CD | LYS | B | 271 | 36.435 | 31.900 | 73.617 | 1.00 | 53.65 | C |
| ATOM | 4444 | CE | LYS | B | 271 | 37.134 | 31.464 | 72.321 | 1.00 | 53.65 | C |
| ATOM | 4445 | NZ | LYS | B | 271 | 37.221 | 32.531 | 71.253 | 1.00 | 53.65 | N |
| ATOM | 4446 | N | VAL | B | 272 | 34.194 | 31.131 | 78.607 | 1.00 | 44.83 | N |
| ATOM | 4447 | CA | VAL | B | 272 | 34.464 | 31.271 | 80.026 | 1.00 | 44.83 | C |
| ATOM | 4448 | C | VAL | B | 272 | 33.527 | 30.498 | 80.939 | 1.00 | 44.83 | C |
| ATOM | 4449 | O | VAL | B | 272 | 33.924 | 30.143 | 82.039 | 1.00 | 44.83 | O |
| ATOM | 4450 | CB | VAL | B | 272 | 34.538 | 32.765 | 80.453 | 1.00 | 28.34 | C |
| ATOM | 4451 | CG1 | VAL | B | 272 | 34.172 | 33.656 | 79.276 | 1.00 | 28.34 | C |
| ATOM | 4452 | CG2 | VAL | B | 272 | 33.662 | 33.023 | 81.678 | 1.00 | 28.34 | C |
| ATOM | 4453 | N | LEU | B | 273 | 32.303 | 30.208 | 80.514 | 1.00 | 41.39 | N |
| ATOM | 4454 | CA | LEU | B | 273 | 31.414 | 29.450 | 81.400 | 1.00 | 41.39 | C |
| ATOM | 4455 | C | LEU | B | 273 | 31.427 | 27.955 | 81.083 | 1.00 | 41.39 | C |
| ATOM | 4456 | O | LEU | B | 273 | 30.767 | 27.164 | 81.746 | 1.00 | 41.39 | O |
| ATOM | 4457 | CB | LEU | B | 273 | 29.967 | 29.947 | 81.306 | 1.00 | 34.53 | C |
| ATOM | 4458 | CG | LEU | B | 273 | 29.619 | 31.415 | 81.542 | 1.00 | 34.53 | C |
| ATOM | 4459 | CD1 | LEU | B | 273 | 28.145 | 31.488 | 81.883 | 1.00 | 34.53 | C |
| ATOM | 4460 | CD2 | LEU | B | 273 | 30.436 | 32.007 | 82.671 | 1.00 | 34.53 | C |

| | | | | | | | | | | | |
|---|---|---|---|---|---|---|---|---|---|---|---|
| ATOM | 4461 | N | GLY | B | 274 | 32.185 | 27.554 | 80.075 | 1.00 | 42.38 | N |
| ATOM | 4462 | CA | GLY | B | 274 | 32.188 | 26.147 | 79.724 | 1.00 | 42.38 | C |
| ATOM | 4463 | C | GLY | B | 274 | 30.885 | 25.899 | 78.981 | 1.00 | 42.38 | C |
| ATOM | 4464 | O | GLY | B | 274 | 30.004 | 26.753 | 79.011 | 1.00 | 42.38 | O |
| ATOM | 4465 | N | THR | B | 275 | 30.751 | 24.764 | 78.296 | 1.00 | 39.88 | N |
| ATOM | 4466 | CA | THR | B | 275 | 29.528 | 24.479 | 77.537 | 1.00 | 39.88 | C |
| ATOM | 4467 | C | THR | B | 275 | 28.324 | 24.418 | 78.497 | 1.00 | 39.88 | C |
| ATOM | 4468 | O | THR | B | 275 | 28.463 | 24.185 | 79.677 | 1.00 | 39.88 | O |
| ATOM | 4469 | CB | THR | B | 275 | 29.571 | 23.103 | 76.792 | 1.00 | 66.35 | C |
| ATOM | 4470 | OG1 | THR | B | 275 | 30.918 | 22.733 | 76.458 | 1.00 | 66.35 | O |
| ATOM | 4471 | CG2 | THR | B | 275 | 28.741 | 23.178 | 75.512 | 1.00 | 66.35 | C |
| ATOM | 4472 | N | PRO | B | 276 | 27.117 | 24.620 | 77.983 | 1.00 | 61.18 | N |
| ATOM | 4473 | CA | PRO | B | 276 | 25.963 | 24.559 | 78.890 | 1.00 | 61.18 | C |
| ATOM | 4474 | C | PRO | B | 276 | 25.533 | 23.112 | 78.966 | 1.00 | 61.18 | C |
| ATOM | 4475 | O | PRO | B | 276 | 25.862 | 22.338 | 78.071 | 1.00 | 61.18 | O |
| ATOM | 4476 | CB | PRO | B | 276 | 24.909 | 25.410 | 78.205 | 1.00 | 58.97 | C |
| ATOM | 4477 | CG | PRO | B | 276 | 25.574 | 25.950 | 76.991 | 1.00 | 58.97 | C |
| ATOM | 4478 | CD | PRO | B | 276 | 26.751 | 25.148 | 76.667 | 1.00 | 58.97 | C |
| ATOM | 4479 | N | THR | B | 277 | 24.820 | 22.756 | 80.030 | 1.00 | 61.20 | N |
| ATOM | 4480 | CA | THR | B | 277 | 24.340 | 21.410 | 80.262 | 1.00 | 61.20 | C |
| ATOM | 4481 | C | THR | B | 277 | 22.928 | 21.439 | 79.766 | 1.00 | 61.20 | C |
| ATOM | 4482 | O | THR | B | 277 | 22.373 | 22.520 | 79.559 | 1.00 | 61.20 | O |
| ATOM | 4483 | CB | THR | B | 277 | 24.294 | 21.099 | 81.740 | 1.00 | 67.39 | C |
| ATOM | 4484 | OG1 | THR | B | 277 | 23.608 | 19.868 | 81.950 | 1.00 | 67.39 | O |
| ATOM | 4485 | CG2 | THR | B | 277 | 23.554 | 22.149 | 82.456 | 0.00 | 67.39 | C |
| ATOM | 4486 | N | ARG | B | 278 | 22.340 | 20.263 | 79.590 | 1.00 | 59.77 | N |
| ATOM | 4487 | CA | ARG | B | 278 | 20.988 | 20.191 | 79.053 | 1.00 | 59.77 | C |
| ATOM | 4488 | C | ARG | B | 278 | 20.048 | 20.894 | 80.006 | 1.00 | 59.77 | C |
| ATOM | 4489 | O | ARG | B | 278 | 19.128 | 21.607 | 79.619 | 1.00 | 59.77 | O |
| ATOM | 4490 | CB | ARG | B | 278 | 20.579 | 18.722 | 78.865 | 1.00 | 80.99 | C |
| ATOM | 4491 | N | GLU | B | 279 | 20.280 | 20.720 | 81.281 | 0.00 | 74.44 | N |
| ATOM | 4492 | CA | GLU | B | 279 | 19.394 | 21.370 | 82.194 | 1.00 | 74.44 | C |
| ATOM | 4493 | C | GLU | B | 279 | 19.417 | 22.906 | 82.055 | 1.00 | 74.44 | C |
| ATOM | 4494 | O | GLU | B | 279 | 18.381 | 23.576 | 82.213 | 1.00 | 74.44 | O |
| ATOM | 4495 | CB | GLU | B | 279 | 19.770 | 20.956 | 83.616 | 1.00100.00 | | C |
| ATOM | 4496 | CG | GLU | B | 279 | 18.855 | 21.511 | 84.709 | 1.00100.00 | | C |
| ATOM | 4497 | CD | GLU | B | 279 | 19.242 | 20.978 | 86.092 | 1.00100.00 | | C |
| ATOM | 4498 | OE1 | GLU | B | 279 | 18.578 | 21.359 | 87.112 | 1.00100.00 | | O |
| ATOM | 4499 | OE2 | GLU | B | 279 | 20.222 | 20.166 | 86.169 | 1.00100.00 | | O |
| ATOM | 4500 | N | GLN | B | 280 | 20.597 | 23.451 | 81.762 | 1.00 | 49.40 | N |
| ATOM | 4501 | CA | GLN | B | 280 | 20.767 | 24.886 | 81.689 | 1.00 | 49.40 | C |
| ATOM | 4502 | C | GLN | B | 280 | 20.126 | 25.436 | 80.448 | 1.00 | 49.40 | C |
| ATOM | 4503 | O | GLN | B | 280 | 19.741 | 26.602 | 80.394 | 0.00 | 49.40 | O |
| ATOM | 4504 | CB | GLN | B | 280 | 22.245 | 25.240 | 81.736 | 1.00 | 52.20 | C |
| ATOM | 4505 | CG | GLN | B | 280 | 22.919 | 24.978 | 83.083 | 1.00 | 52.20 | C |
| ATOM | 4506 | CD | GLN | B | 280 | 24.418 | 25.173 | 82.983 | 1.00 | 52.20 | C |
| ATOM | 4507 | OE1 | GLN | B | 280 | 25.154 | 25.010 | 83.952 | 1.00 | 52.20 | O |
| ATOM | 4508 | NE2 | GLN | B | 280 | 24.879 | 25.536 | 81.791 | 1.00 | 52.20 | N |
| ATOM | 4509 | N | ILE | B | 281 | 19.995 | 24.569 | 79.455 | 1.00 | 75.99 | N |
| ATOM | 4510 | CA | ILE | B | 281 | 19.383 | 24.970 | 78.211 | 1.00 | 75.99 | C |
| ATOM | 4511 | C | ILE | B | 281 | 17.863 | 25.073 | 78.359 | 1.00 | 75.99 | C |
| ATOM | 4512 | O | ILE | B | 281 | 17.278 | 26.075 | 77.926 | 1.00 | 75.99 | O |
| ATOM | 4513 | CB | ILE | B | 281 | 19.740 | 23.994 | 77.077 | 0.00 | 79.77 | C |
| ATOM | 4514 | CG1 | ILE | B | 281 | 19.001 | 24.353 | 75.802 | 1.00 | 79.77 | C |
| ATOM | 4515 | CG2 | ILE | B | 281 | 19.320 | 22.628 | 77.425 | 1.00 | 79.77 | C |
| ATOM | 4516 | CD1 | ILE | B | 281 | 19.305 | 23.354 | 74.681 | 1.00 | 79.77 | C |
| ATOM | 4517 | N | ARG | B | 282 | 17.216 | 24.071 | 78.972 | 1.00 | 75.81 | N |
| ATOM | 4518 | CA | ARG | B | 282 | 15.753 | 24.119 | 79.111 | 1.00 | 75.81 | C |
| ATOM | 4519 | C | ARG | B | 282 | 15.419 | 25.333 | 79.945 | 1.00 | 75.81 | C |
| ATOM | 4520 | O | ARG | B | 282 | 14.409 | 26.018 | 79.758 | 1.00 | 75.81 | O |
| ATOM | 4521 | CB | ARG | B | 282 | 15.204 | 22.820 | 79.803 | 1.00 | 70.19 | C |
| ATOM | 4522 | N | GLU | B | 283 | 16.327 | 25.621 | 80.846 | 1.00 | 65.28 | N |

| | | | | | | | | | | | |
|---|---|---|---|---|---|---|---|---|---|---|---|
| ATOM | 4523 | CA | GLU | B | 283 | 16.118 | 26.712 | 81.735 | 1.00 | 65.28 | C |
| ATOM | 4524 | C | GLU | B | 283 | 16.499 | 28.038 | 81.093 | 1.00 | 65.28 | C |
| ATOM | 4525 | O | GLU | B | 283 | 16.196 | 29.121 | 81.608 | 1.00 | 65.28 | O |
| ATOM | 4526 | CB | GLU | B | 283 | 16.957 | 26.457 | 82.935 | 1.00 | 100.00 | C |
| ATOM | 4527 | CG | GLU | B | 283 | 16.909 | 27.617 | 83.791 | 1.00 | 100.00 | C |
| ATOM | 4528 | CD | GLU | B | 283 | 17.816 | 27.474 | 84.974 | 1.00 | 100.00 | C |
| ATOM | 4529 | OE1 | GLU | B | 283 | 17.846 | 28.420 | 85.822 | 1.00 | 100.00 | O |
| ATOM | 4530 | OE2 | GLU | B | 283 | 18.512 | 26.416 | 85.080 | 1.00 | 100.00 | O |
| ATOM | 4531 | N | MET | B | 284 | 17.138 | 27.918 | 79.937 | 1.00 | 100.00 | N |
| ATOM | 4532 | CA | MET | B | 284 | 17.596 | 29.044 | 79.174 | 1.00 | 100.00 | C |
| ATOM | 4533 | C | MET | B | 284 | 16.739 | 29.353 | 77.925 | 1.00 | 100.00 | C |
| ATOM | 4534 | O | MET | B | 284 | 16.206 | 30.470 | 77.799 | 1.00 | 100.00 | O |
| ATOM | 4535 | CB | MET | B | 284 | 19.013 | 28.762 | 78.783 | 0.00 | 72.50 | C |
| ATOM | 4536 | CG | MET | B | 284 | 19.646 | 29.896 | 78.189 | 1.00 | 72.50 | C |
| ATOM | 4537 | SD | MET | B | 284 | 21.389 | 29.541 | 78.241 | 1.00 | 72.50 | S |
| ATOM | 4538 | CE | MET | B | 284 | 21.842 | 31.052 | 77.167 | 1.00 | 72.50 | C |
| ATOM | 4539 | N | ASN | B | 285 | 16.601 | 28.397 | 77.005 | 1.00 | 87.37 | N |
| ATOM | 4540 | CA | ASN | B | 285 | 15.819 | 28.618 | 75.787 | 1.00 | 87.37 | C |
| ATOM | 4541 | C | ASN | B | 285 | 14.346 | 28.250 | 75.967 | 1.00 | 87.37 | C |
| ATOM | 4542 | O | ASN | B | 285 | 14.012 | 27.369 | 76.775 | 1.00 | 87.37 | O |
| ATOM | 4543 | CB | ASN | B | 285 | 16.429 | 27.804 | 74.639 | 1.00 | 72.72 | C |
| ATOM | 4544 | N | PHE | B | 291 | 20.827 | 23.254 | 69.045 | 1.00 | 66.65 | N |
| ATOM | 4545 | CA | PHE | B | 291 | 22.007 | 23.681 | 69.787 | 1.00 | 66.65 | C |
| ATOM | 4546 | C | PHE | B | 291 | 23.179 | 22.717 | 69.586 | 1.00 | 66.65 | C |
| ATOM | 4547 | O | PHE | B | 291 | 23.154 | 21.563 | 69.994 | 1.00 | 66.65 | O |
| ATOM | 4548 | CB | PHE | B | 291 | 21.640 | 23.751 | 71.270 | 1.00 | 50.75 | C |
| ATOM | 4549 | CG | PHE | B | 291 | 22.851 | 24.114 | 72.076 | 1.00 | 50.75 | C |
| ATOM | 4550 | CD1 | PHE | B | 291 | 23.267 | 25.439 | 72.133 | 1.00 | 50.75 | C |
| ATOM | 4551 | CD2 | PHE | B | 291 | 23.557 | 23.130 | 72.748 | 0.00 | 50.75 | C |
| ATOM | 4552 | CE1 | PHE | B | 291 | 24.395 | 25.776 | 72.867 | 1.00 | 50.75 | C |
| ATOM | 4553 | CE2 | PHE | B | 291 | 24.688 | 23.476 | 73.482 | 0.00 | 50.75 | C |
| ATOM | 4554 | CZ | PHE | B | 291 | 25.111 | 24.798 | 73.545 | 1.00 | 50.75 | C |
| ATOM | 4555 | N | LYS | B | 292 | 24.217 | 23.219 | 68.892 | 1.00 | 91.46 | N |
| ATOM | 4556 | CA | LYS | B | 292 | 25.388 | 22.386 | 68.644 | 1.00 | 91.46 | C |
| ATOM | 4557 | C | LYS | B | 292 | 26.633 | 23.233 | 68.369 | 1.00 | 91.46 | C |
| ATOM | 4558 | O | LYS | B | 292 | 27.082 | 23.389 | 67.241 | 1.00 | 91.46 | O |
| ATOM | 4559 | CB | LYS | B | 292 | 25.091 | 21.491 | 67.441 | 1.00 | 56.32 | C |
| ATOM | 4560 | N | PHE | B | 293 | 27.171 | 23.823 | 69.453 | 1.00 | 98.94 | N |
| ATOM | 4561 | CA | PHE | B | 293 | 28.358 | 24.656 | 69.303 | 1.00 | 98.94 | C |
| ATOM | 4562 | C | PHE | B | 293 | 29.643 | 23.845 | 69.485 | 1.00 | 98.94 | C |
| ATOM | 4563 | O | PHE | B | 293 | 30.391 | 23.600 | 68.548 | 1.00 | 98.94 | O |
| ATOM | 4564 | CB | PHE | B | 293 | 28.294 | 25.770 | 70.349 | 1.00 | 52.03 | C |
| ATOM | 4565 | CG | PHE | B | 293 | 27.212 | 26.744 | 69.987 | 1.00 | 52.03 | C |
| ATOM | 4566 | CD1 | PHE | B | 293 | 25.881 | 26.349 | 70.055 | 1.00 | 52.03 | C |
| ATOM | 4567 | CD2 | PHE | B | 293 | 27.542 | 28.026 | 69.580 | 1.00 | 52.03 | C |
| ATOM | 4568 | CE1 | PHE | B | 293 | 24.878 | 27.245 | 69.712 | 0.00 | 52.03 | C |
| ATOM | 4569 | CE2 | PHE | B | 293 | 26.529 | 28.919 | 69.238 | 1.00 | 52.03 | C |
| ATOM | 4570 | CZ | PHE | B | 293 | 25.197 | 28.533 | 69.303 | 0.00 | 52.03 | C |
| ATOM | 4571 | N | PRO | B | 294 | 29.901 | 23.463 | 70.749 | 1.00 | 96.91 | N |
| ATOM | 4572 | CA | PRO | B | 294 | 31.101 | 22.686 | 71.036 | 1.00 | 96.91 | C |
| ATOM | 4573 | C | PRO | B | 294 | 30.970 | 21.912 | 72.350 | 1.00 | 96.91 | C |
| ATOM | 4574 | O | PRO | B | 294 | 31.094 | 22.458 | 73.438 | 1.00 | 96.91 | O |
| ATOM | 4575 | N | GLN | B | 295 | 30.676 | 20.606 | 72.204 | 1.00 | 72.54 | N |
| ATOM | 4576 | CA | GLN | B | 295 | 30.534 | 19.764 | 73.385 | 1.00 | 72.54 | C |
| ATOM | 4577 | C | GLN | B | 295 | 31.679 | 19.984 | 74.376 | 1.00 | 72.54 | C |
| ATOM | 4578 | O | GLN | B | 295 | 31.713 | 19.419 | 75.461 | 1.00 | 72.54 | O |
| ATOM | 4579 | N | ILE | B | 296 | 32.732 | 20.771 | 74.154 | 1.00 | 83.25 | N |
| ATOM | 4580 | CA | ILE | B | 296 | 33.771 | 20.911 | 75.161 | 1.00 | 83.25 | C |
| ATOM | 4581 | C | ILE | B | 296 | 34.046 | 22.373 | 75.497 | 1.00 | 83.25 | C |
| ATOM | 4582 | O | ILE | B | 296 | 33.124 | 23.192 | 75.645 | 1.00 | 83.25 | O |
| ATOM | 4583 | N | LYS | B | 297 | 35.323 | 22.714 | 75.628 | 1.00 | 100.00 | N |
| ATOM | 4584 | CA | LYS | B | 297 | 35.776 | 24.074 | 75.881 | 1.00 | 100.00 | C |

| | | | | | | | | | | | |
|---|---|---|---|---|---|---|---|---|---|---|---|
| ATOM | 4585 | C | LYS | B | 297 | 35.342 | 24.559 | 77.265 | 1.00 | 100.00 | C |
| ATOM | 4586 | O | LYS | B | 297 | 34.427 | 25.356 | 77.421 | 1.00 | 100.00 | O |
| ATOM | 4587 | CB | LYS | B | 297 | 35.185 | 24.981 | 74.801 | 1.00 | 46.14 | C |
| ATOM | 4588 | N | ALA | B | 298 | 36.009 | 24.012 | 78.299 | 1.00 | 100.00 | N |
| ATOM | 4589 | CA | ALA | B | 298 | 35.710 | 24.455 | 79.655 | 1.00 | 100.00 | C |
| ATOM | 4590 | C | ALA | B | 298 | 36.015 | 25.946 | 79.825 | 1.00 | 100.00 | C |
| ATOM | 4591 | O | ALA | B | 298 | 36.598 | 26.595 | 78.967 | 1.00 | 100.00 | O |
| ATOM | 4592 | CB | ALA | B | 298 | 36.557 | 23.632 | 80.626 | 1.00 | 63.79 | C |
| ATOM | 4593 | N | HIS | B | 299 | 35.561 | 26.502 | 80.964 | 1.00 | 82.91 | N |
| ATOM | 4594 | CA | HIS | B | 299 | 35.772 | 27.926 | 81.186 | 1.00 | 82.91 | C |
| ATOM | 4595 | C | HIS | B | 299 | 36.526 | 28.201 | 82.489 | 1.00 | 82.91 | C |
| ATOM | 4596 | O | HIS | B | 299 | 36.146 | 27.770 | 83.571 | 1.00 | 82.91 | O |
| ATOM | 4597 | N | PRO | B | 300 | 37.664 | 28.907 | 82.350 | 1.00 | 100.00 | N |
| ATOM | 4598 | CA | PRO | B | 300 | 38.436 | 29.352 | 83.494 | 1.00 | 100.00 | C |
| ATOM | 4599 | C | PRO | B | 300 | 37.888 | 30.671 | 84.041 | 1.00 | 100.00 | C |
| ATOM | 4600 | O | PRO | B | 300 | 37.218 | 31.433 | 83.357 | 1.00 | 100.00 | O |
| ATOM | 4601 | CB | PRO | B | 300 | 39.870 | 29.552 | 83.011 | 1.00 | 82.31 | C |
| ATOM | 4602 | CG | PRO | B | 300 | 39.851 | 29.659 | 81.484 | 1.00 | 82.31 | C |
| ATOM | 4603 | CD | PRO | B | 300 | 38.324 | 29.323 | 81.125 | 1.00 | 82.31 | C |
| ATOM | 4604 | N | TRP | B | 301 | 38.160 | 30.914 | 85.335 | 1.00 | 95.69 | N |
| ATOM | 4605 | CA | TRP | B | 301 | 37.677 | 32.149 | 85.941 | 1.00 | 95.69 | C |
| ATOM | 4606 | C | TRP | B | 301 | 38.829 | 33.028 | 86.429 | 1.00 | 95.69 | C |
| ATOM | 4607 | O | TRP | B | 301 | 38.988 | 34.178 | 86.044 | 1.00 | 95.69 | O |
| ATOM | 4608 | CB | TRP | B | 301 | 36.772 | 31.782 | 87.118 | 1.00 | 45.45 | C |
| ATOM | 4609 | CG | TRP | B | 301 | 35.352 | 31.991 | 86.756 | 1.00 | 45.45 | C |
| ATOM | 4610 | CD1 | TRP | B | 301 | 34.281 | 31.100 | 86.991 | 1.00 | 45.45 | C |
| ATOM | 4611 | CD2 | TRP | B | 301 | 34.795 | 33.122 | 86.040 | 1.00 | 45.45 | C |
| ATOM | 4612 | NE1 | TRP | B | 301 | 33.105 | 31.554 | 86.487 | 1.00 | 45.45 | N |
| ATOM | 4613 | CE2 | TRP | B | 301 | 33.413 | 32.863 | 85.862 | 1.00 | 45.45 | C |
| ATOM | 4614 | CE3 | TRP | B | 301 | 35.341 | 34.298 | 85.534 | 1.00 | 45.45 | C |
| ATOM | 4615 | CZ2 | TRP | B | 301 | 32.622 | 33.771 | 85.178 | 1.00 | 45.45 | C |
| ATOM | 4616 | CZ3 | TRP | B | 301 | 34.549 | 35.205 | 84.849 | 1.00 | 45.45 | C |
| ATOM | 4617 | CH2 | TRP | B | 301 | 33.181 | 34.937 | 84.668 | 1.00 | 45.45 | C |
| ATOM | 4618 | N | THR | B | 302 | 39.628 | 32.453 | 87.343 | 1.00 | 65.12 | N |
| ATOM | 4619 | CA | THR | B | 302 | 40.725 | 33.217 | 87.921 | 1.00 | 65.12 | C |
| ATOM | 4620 | C | THR | B | 302 | 41.698 | 33.709 | 86.847 | 1.00 | 65.12 | C |
| ATOM | 4621 | O | THR | B | 302 | 42.475 | 34.631 | 87.051 | 1.00 | 65.12 | O |
| ATOM | 4622 | CB | THR | B | 302 | 41.448 | 32.311 | 88.917 | 1.00 | 68.67 | C |
| ATOM | 4623 | OG1 | THR | B | 302 | 40.515 | 31.885 | 89.913 | 1.00 | 68.67 | O |
| ATOM | 4624 | CG2 | THR | B | 302 | 42.585 | 33.076 | 89.600 | 1.00 | 68.67 | C |
| ATOM | 4625 | N | LYS | B | 303 | 41.603 | 33.065 | 85.680 | 1.00 | 67.32 | N |
| ATOM | 4626 | CA | LYS | B | 303 | 42.473 | 33.398 | 84.555 | 1.00 | 67.32 | C |
| ATOM | 4627 | C | LYS | B | 303 | 41.890 | 34.337 | 83.500 | 1.00 | 67.32 | C |
| ATOM | 4628 | O | LYS | B | 303 | 42.567 | 34.689 | 82.530 | 1.00 | 67.32 | O |
| ATOM | 4629 | CB | LYS | B | 303 | 42.972 | 32.108 | 83.903 | 1.00 | 63.31 | C |
| ATOM | 4630 | CG | LYS | B | 303 | 43.843 | 31.267 | 84.859 | 1.00 | 63.31 | C |
| ATOM | 4631 | CD | LYS | B | 303 | 44.373 | 30.011 | 84.190 | 0.00 | 63.31 | C |
| ATOM | 4632 | CE | LYS | B | 303 | 45.238 | 29.210 | 85.152 | 0.00 | 63.31 | C |
| ATOM | 4633 | NZ | LYS | B | 303 | 45.742 | 27.946 | 84.547 | 0.00 | 63.31 | N |
| ATOM | 4634 | N | VAL | B | 304 | 40.645 | 34.755 | 83.696 | 1.00 | 48.89 | N |
| ATOM | 4635 | CA | VAL | B | 304 | 40.009 | 35.671 | 82.765 | 1.00 | 48.89 | C |
| ATOM | 4636 | C | VAL | B | 304 | 40.552 | 37.086 | 83.002 | 1.00 | 48.89 | C |
| ATOM | 4637 | O | VAL | B | 304 | 40.678 | 37.874 | 82.065 | 1.00 | 48.89 | O |
| ATOM | 4638 | CB | VAL | B | 304 | 38.474 | 35.714 | 82.979 | 1.00 | 35.95 | C |
| ATOM | 4639 | CG1 | VAL | B | 304 | 37.831 | 36.609 | 81.934 | 0.00 | 35.95 | C |
| ATOM | 4640 | CG2 | VAL | B | 304 | 37.887 | 34.318 | 82.926 | 1.00 | 35.95 | C |
| ATOM | 4641 | N | PHE | B | 305 | 40.892 | 37.391 | 84.255 | 1.00 | 43.83 | N |
| ATOM | 4642 | CA | PHE | B | 305 | 41.367 | 38.726 | 84.634 | 1.00 | 43.83 | C |
| ATOM | 4643 | C | PHE | B | 305 | 42.864 | 38.877 | 84.911 | 1.00 | 43.83 | C |
| ATOM | 4644 | O | PHE | B | 305 | 43.594 | 37.898 | 84.940 | 1.00 | 43.83 | O |
| ATOM | 4645 | CB | PHE | B | 305 | 40.594 | 39.176 | 85.864 | 1.00 | 32.65 | C |
| ATOM | 4646 | CG | PHE | B | 305 | 39.112 | 39.002 | 85.734 | 1.00 | 32.65 | C |

| | | | | | | | | | | | |
|---|---|---|---|---|---|---|---|---|---|---|---|
| ATOM | 4647 | CD1 | PHE | B | 305 | 38.348 | 39.937 | 85.060 | 1.00 | 32.65 | C |
| ATOM | 4648 | CD2 | PHE | B | 305 | 38.484 | 37.884 | 86.261 | 1.00 | 32.65 | C |
| ATOM | 4649 | CE1 | PHE | B | 305 | 36.988 | 39.765 | 84.921 | 1.00 | 32.65 | C |
| ATOM | 4650 | CE2 | PHE | B | 305 | 37.117 | 37.707 | 86.122 | 1.00 | 32.65 | C |
| ATOM | 4651 | CZ | PHE | B | 305 | 36.371 | 38.645 | 85.449 | 1.00 | 32.65 | C |
| ATOM | 4652 | N | ARG | B | 306 | 43.311 | 40.119 | 85.113 | 1.00 | 44.90 | N |
| ATOM | 4653 | CA | ARG | B | 306 | 44.719 | 40.404 | 85.405 | 1.00 | 44.90 | C |
| ATOM | 4654 | C | ARG | B | 306 | 45.061 | 39.663 | 86.690 | 1.00 | 44.90 | C |
| ATOM | 4655 | O | ARG | B | 306 | 44.194 | 39.443 | 87.537 | 1.00 | 44.90 | O |
| ATOM | 4656 | CB | ARG | B | 306 | 44.933 | 41.903 | 85.587 | 1.00 | 23.70 | C |
| ATOM | 4657 | N | PRO | B | 307 | 46.334 | 39.274 | 86.862 | 1.00 | 56.05 | N |
| ATOM | 4658 | CA | PRO | B | 307 | 46.732 | 38.542 | 88.080 | 1.00 | 56.05 | C |
| ATOM | 4659 | C | PRO | B | 307 | 46.495 | 39.330 | 89.371 | 1.00 | 56.05 | C |
| ATOM | 4660 | O | PRO | B | 307 | 46.385 | 38.749 | 90.455 | 1.00 | 56.05 | O |
| ATOM | 4661 | CB | PRO | B | 307 | 48.219 | 38.247 | 87.845 | 1.00 | 73.61 | C |
| ATOM | 4662 | CG | PRO | B | 307 | 48.380 | 38.348 | 86.318 | 1.00 | 73.61 | C |
| ATOM | 4663 | CD | PRO | B | 307 | 47.497 | 39.529 | 85.990 | 1.00 | 73.61 | C |
| ATOM | 4664 | N | ARG | B | 308 | 46.415 | 40.653 | 89.251 | 1.00 | 45.08 | N |
| ATOM | 4665 | CA | ARG | B | 308 | 46.178 | 41.504 | 90.416 | 1.00 | 45.08 | C |
| ATOM | 4666 | C | ARG | B | 308 | 44.723 | 41.680 | 90.864 | 1.00 | 45.08 | C |
| ATOM | 4667 | O | ARG | B | 308 | 44.454 | 42.225 | 91.936 | 1.00 | 45.08 | O |
| ATOM | 4668 | CB | ARG | B | 308 | 46.482 | 42.956 | 90.063 | 0.00 | 35.69 | C |
| ATOM | 4669 | N | THR | B | 309 | 43.792 | 41.362 | 89.986 | 1.00 | 47.61 | N |
| ATOM | 4670 | CA | THR | B | 309 | 42.351 | 41.277 | 90.259 | 1.00 | 47.61 | C |
| ATOM | 4671 | C | THR | B | 309 | 41.907 | 40.592 | 91.547 | 1.00 | 47.61 | C |
| ATOM | 4672 | O | THR | B | 309 | 42.059 | 39.387 | 91.709 | 1.00 | 47.61 | O |
| ATOM | 4673 | CB | THR | B | 309 | 41.626 | 40.590 | 89.116 | 1.00 | 35.64 | C |
| ATOM | 4674 | OG1 | THR | B | 309 | 42.081 | 41.136 | 87.877 | 1.00 | 35.64 | O |
| ATOM | 4675 | CG2 | THR | B | 309 | 40.142 | 40.823 | 89.236 | 1.00 | 35.64 | C |
| ATOM | 4676 | N | PRO | B | 310 | 41.323 | 41.364 | 92.471 | 1.00 | 44.91 | N |
| ATOM | 4677 | CA | PRO | B | 310 | 40.840 | 40.868 | 93.761 | 1.00 | 44.91 | C |
| ATOM | 4678 | C | PRO | B | 310 | 39.954 | 39.646 | 93.597 | 1.00 | 44.91 | C |
| ATOM | 4679 | O | PRO | B | 310 | 38.952 | 39.693 | 92.884 | 1.00 | 44.91 | O |
| ATOM | 4680 | CB | PRO | B | 310 | 40.064 | 42.052 | 94.308 | 1.00 | 31.40 | C |
| ATOM | 4681 | CG | PRO | B | 310 | 40.810 | 43.216 | 93.764 | 1.00 | 31.40 | C |
| ATOM | 4682 | CD | PRO | B | 310 | 41.055 | 42.807 | 92.335 | 1.00 | 31.40 | C |
| ATOM | 4683 | N | PRO | B | 311 | 40.304 | 38.534 | 94.264 | 1.00 | 45.87 | N |
| ATOM | 4684 | CA | PRO | B | 311 | 39.508 | 37.308 | 94.167 | 1.00 | 45.87 | C |
| ATOM | 4685 | C | PRO | B | 311 | 38.003 | 37.493 | 94.358 | 1.00 | 45.87 | C |
| ATOM | 4686 | O | PRO | B | 311 | 37.223 | 37.148 | 93.473 | 1.00 | 45.87 | O |
| ATOM | 4687 | CB | PRO | B | 311 | 40.130 | 36.404 | 95.234 | 1.00 | 36.21 | C |
| ATOM | 4688 | CG | PRO | B | 311 | 40.725 | 37.364 | 96.205 | 1.00 | 36.21 | C |
| ATOM | 4689 | CD | PRO | B | 311 | 41.352 | 38.381 | 95.285 | 1.00 | 36.21 | C |
| ATOM | 4690 | N | GLU | B | 312 | 37.578 | 38.033 | 95.493 | 1.00 | 41.29 | N |
| ATOM | 4691 | CA | GLU | B | 312 | 36.142 | 38.187 | 95.692 | 1.00 | 41.29 | C |
| ATOM | 4692 | C | GLU | B | 312 | 35.477 | 39.038 | 94.602 | 1.00 | 41.29 | C |
| ATOM | 4693 | O | GLU | B | 312 | 34.262 | 38.974 | 94.408 | 1.00 | 41.29 | O |
| ATOM | 4694 | CB | GLU | B | 312 | 35.817 | 38.715 | 97.103 | 1.00 | 70.73 | C |
| ATOM | 4695 | CG | GLU | B | 312 | 36.611 | 39.903 | 97.573 | 1.00 | 70.73 | C |
| ATOM | 4696 | CD | GLU | B | 312 | 38.084 | 39.596 | 97.653 | 1.00 | 70.73 | C |
| ATOM | 4697 | OE1 | GLU | B | 312 | 38.793 | 39.856 | 96.647 | 1.00 | 70.73 | O |
| ATOM | 4698 | OE2 | GLU | B | 312 | 38.522 | 39.079 | 98.711 | 1.00 | 70.73 | O |
| ATOM | 4699 | N | ALA | B | 313 | 36.268 | 39.825 | 93.882 | 1.00 | 36.56 | N |
| ATOM | 4700 | CA | ALA | B | 313 | 35.714 | 40.612 | 92.789 | 1.00 | 36.56 | C |
| ATOM | 4701 | C | ALA | B | 313 | 35.435 | 39.590 | 91.694 | 1.00 | 36.56 | C |
| ATOM | 4702 | O | ALA | B | 313 | 34.479 | 39.710 | 90.936 | 1.00 | 36.56 | O |
| ATOM | 4703 | CB | ALA | B | 313 | 36.723 | 41.637 | 92.302 | 1.00 | 44.46 | C |
| ATOM | 4704 | N | ILE | B | 314 | 36.292 | 38.578 | 91.624 | 1.00 | 33.59 | N |
| ATOM | 4705 | CA | ILE | B | 314 | 36.146 | 37.513 | 90.649 | 1.00 | 33.59 | C |
| ATOM | 4706 | C | ILE | B | 314 | 35.054 | 36.531 | 91.068 | 1.00 | 33.59 | C |
| ATOM | 4707 | O | ILE | B | 314 | 34.269 | 36.076 | 90.234 | 1.00 | 33.59 | O |
| ATOM | 4708 | CB | ILE | B | 314 | 37.471 | 36.758 | 90.472 | 1.00 | 40.76 | C |

| | | | | | | | | | | | |
|---|---|---|---|---|---|---|---|---|---|---|---|
| ATOM | 4709 | CG1 | ILE | B | 314 | 38.474 | 37.684 | 89.781 | 1.00 | 40.76 | C |
| ATOM | 4710 | CG2 | ILE | B | 314 | 37.254 | 35.460 | 89.697 | 1.00 | 40.76 | C |
| ATOM | 4711 | CD1 | ILE | B | 314 | 39.771 | 37.015 | 89.370 | 1.00 | 40.76 | C |
| ATOM | 4712 | N | ALA | B | 315 | 35.002 | 36.205 | 92.357 | 1.00 | 35.68 | N |
| ATOM | 4713 | CA | ALA | B | 315 | 33.996 | 35.283 | 92.865 | 1.00 | 35.68 | C |
| ATOM | 4714 | C | ALA | B | 315 | 32.615 | 35.820 | 92.547 | 1.00 | 35.68 | C |
| ATOM | 4715 | O | ALA | B | 315 | 31.750 | 35.094 | 92.049 | 1.00 | 35.68 | O |
| ATOM | 4716 | CB | ALA | B | 315 | 34.145 | 35.131 | 94.345 | 1.00 | 29.50 | C |
| ATOM | 4717 | N | LEU | B | 316 | 32.418 | 37.103 | 92.841 | 1.00 | 42.05 | N |
| ATOM | 4718 | CA | LEU | B | 316 | 31.149 | 37.780 | 92.608 | 1.00 | 42.05 | C |
| ATOM | 4719 | C | LEU | B | 316 | 30.718 | 37.686 | 91.151 | 1.00 | 42.05 | C |
| ATOM | 4720 | O | LEU | B | 316 | 29.540 | 37.509 | 90.860 | 1.00 | 42.05 | O |
| ATOM | 4721 | CB | LEU | B | 316 | 31.254 | 39.248 | 93.034 | 1.00 | 30.04 | C |
| ATOM | 4722 | CG | LEU | B | 316 | 30.075 | 40.155 | 92.684 | 1.00 | 30.04 | C |
| ATOM | 4723 | CD1 | LEU | B | 316 | 28.821 | 39.677 | 93.384 | 1.00 | 30.04 | C |
| ATOM | 4724 | CD2 | LEU | B | 316 | 30.405 | 41.573 | 93.084 | 1.00 | 30.04 | C |
| ATOM | 4725 | N | CYS | B | 317 | 31.663 | 37.807 | 90.229 | 1.00 | 48.54 | N |
| ATOM | 4726 | CA | CYS | B | 317 | 31.316 | 37.702 | 88.816 | 1.00 | 48.54 | C |
| ATOM | 4727 | C | CYS | B | 317 | 30.669 | 36.367 | 88.464 | 1.00 | 48.54 | C |
| ATOM | 4728 | O | CYS | B | 317 | 29.641 | 36.324 | 87.776 | 1.00 | 48.54 | O |
| ATOM | 4729 | CB | CYS | B | 317 | 32.552 | 37.868 | 87.947 | 1.00 | 54.56 | C |
| ATOM | 4730 | SG | CYS | B | 317 | 32.625 | 39.460 | 87.158 | 1.00 | 54.56 | S |
| ATOM | 4731 | N | SER | B | 318 | 31.275 | 35.278 | 88.928 | 1.00 | 34.00 | N |
| ATOM | 4732 | CA | SER | B | 318 | 30.761 | 33.956 | 88.632 | 1.00 | 34.00 | C |
| ATOM | 4733 | C | SER | B | 318 | 29.398 | 33.695 | 89.254 | 1.00 | 34.00 | C |
| ATOM | 4734 | O | SER | B | 318 | 28.716 | 32.734 | 88.871 | 1.00 | 34.00 | O |
| ATOM | 4735 | CB | SER | B | 318 | 31.739 | 32.901 | 89.114 | 1.00 | 51.64 | C |
| ATOM | 4736 | OG | SER | B | 318 | 31.894 | 32.999 | 90.514 | 1.00 | 51.64 | O |
| ATOM | 4737 | N | ARG | B | 319 | 28.999 | 34.523 | 90.218 | 1.00 | 30.97 | N |
| ATOM | 4738 | CA | ARG | B | 319 | 27.698 | 34.332 | 90.841 | 1.00 | 30.97 | C |
| ATOM | 4739 | C | ARG | B | 319 | 26.650 | 35.192 | 90.160 | 1.00 | 30.97 | C |
| ATOM | 4740 | O | ARG | B | 319 | 25.461 | 35.158 | 90.509 | 1.00 | 30.97 | O |
| ATOM | 4741 | CB | ARG | B | 319 | 27.748 | 34.640 | 92.334 | 1.00 | 36.64 | C |
| ATOM | 4742 | CG | ARG | B | 319 | 28.523 | 33.620 | 93.147 | 1.00 | 36.64 | C |
| ATOM | 4743 | CD | ARG | B | 319 | 28.176 | 32.183 | 92.747 | 1.00 | 36.64 | C |
| ATOM | 4744 | NE | ARG | B | 319 | 26.740 | 31.947 | 92.594 | 1.00 | 36.64 | N |
| ATOM | 4745 | CZ | ARG | B | 319 | 25.860 | 31.946 | 93.590 | 1.00 | 36.64 | C |
| ATOM | 4746 | NH1 | ARG | B | 319 | 26.254 | 32.172 | 94.836 | 1.00 | 36.64 | N |
| ATOM | 4747 | NH2 | ARG | B | 319 | 24.578 | 31.707 | 93.335 | 1.00 | 36.64 | N |
| ATOM | 4748 | N | LEU | B | 320 | 27.110 | 35.968 | 89.182 | 1.00 | 33.21 | N |
| ATOM | 4749 | CA | LEU | B | 320 | 26.234 | 36.823 | 88.386 | 1.00 | 33.21 | C |
| ATOM | 4750 | C | LEU | B | 320 | 26.120 | 36.179 | 86.993 | 1.00 | 33.21 | C |
| ATOM | 4751 | O | LEU | B | 320 | 25.026 | 35.820 | 86.543 | 1.00 | 33.21 | O |
| ATOM | 4752 | CB | LEU | B | 320 | 26.821 | 38.241 | 88.275 | 1.00 | 28.69 | C |
| ATOM | 4753 | CG | LEU | B | 320 | 26.952 | 38.986 | 89.612 | 1.00 | 28.69 | C |
| ATOM | 4754 | CD1 | LEU | B | 320 | 27.668 | 40.317 | 89.446 | 1.00 | 28.69 | C |
| ATOM | 4755 | CD2 | LEU | B | 320 | 25.569 | 39.212 | 90.180 | 1.00 | 28.69 | C |
| ATOM | 4756 | N | LEU | B | 321 | 27.267 | 36.008 | 86.334 | 1.00 | 29.93 | N |
| ATOM | 4757 | CA | LEU | B | 321 | 27.310 | 35.424 | 85.005 | 1.00 | 29.93 | C |
| ATOM | 4758 | C | LEU | B | 321 | 27.126 | 33.910 | 85.111 | 1.00 | 29.93 | C |
| ATOM | 4759 | O | LEU | B | 321 | 28.070 | 33.124 | 85.004 | 1.00 | 29.93 | O |
| ATOM | 4760 | CB | LEU | B | 321 | 28.629 | 35.821 | 84.340 | 1.00 | 27.53 | C |
| ATOM | 4761 | CG | LEU | B | 321 | 28.716 | 37.358 | 84.294 | 1.00 | 27.53 | C |
| ATOM | 4762 | CD1 | LEU | B | 321 | 29.968 | 37.828 | 83.591 | 1.00 | 27.53 | C |
| ATOM | 4763 | CD2 | LEU | B | 321 | 27.488 | 37.900 | 83.586 | 1.00 | 27.53 | C |
| ATOM | 4764 | N | GLU | B | 322 | 25.870 | 33.526 | 85.314 | 1.00 | 33.10 | N |
| ATOM | 4765 | CA | GLU | B | 322 | 25.467 | 32.144 | 85.504 | 1.00 | 33.10 | C |
| ATOM | 4766 | C | GLU | B | 322 | 24.362 | 31.775 | 84.545 | 1.00 | 33.10 | C |
| ATOM | 4767 | O | GLU | B | 322 | 23.367 | 32.489 | 84.456 | 1.00 | 33.10 | O |
| ATOM | 4768 | CB | GLU | B | 322 | 24.929 | 31.983 | 86.913 | 1.00 | 49.43 | C |
| ATOM | 4769 | CG | GLU | B | 322 | 25.064 | 30.611 | 87.481 | 1.00 | 49.43 | C |
| ATOM | 4770 | CD | GLU | B | 322 | 26.169 | 30.549 | 88.492 | 1.00 | 49.43 | C |

| | | | | | | | | | | | |
|---|---|---|---|---|---|---|---|---|---|---|---|
| ATOM | 4771 | OE1 | GLU | B | 322 | 26.012 | 31.176 | 89.567 | 1.00 | 49.43 | O |
| ATOM | 4772 | OE2 | GLU | B | 322 | 27.194 | 29.890 | 88.210 | 1.00 | 49.43 | O |
| ATOM | 4773 | N | TYR | B | 323 | 24.514 | 30.653 | 83.849 | 1.00 | 32.76 | N |
| ATOM | 4774 | CA | TYR | B | 323 | 23.506 | 30.198 | 82.897 | 1.00 | 32.76 | C |
| ATOM | 4775 | C | TYR | B | 323 | 22.083 | 30.186 | 83.454 | 1.00 | 32.76 | C |
| ATOM | 4776 | O | TYR | B | 323 | 21.196 | 30.836 | 82.928 | 1.00 | 32.76 | O |
| ATOM | 4777 | CB | TYR | B | 323 | 23.859 | 28.795 | 82.395 | 1.00 | 34.11 | C |
| ATOM | 4778 | CG | TYR | B | 323 | 24.926 | 28.776 | 81.330 | 1.00 | 34.11 | C |
| ATOM | 4779 | CD1 | TYR | B | 323 | 24.779 | 29.514 | 80.162 | 1.00 | 34.11 | C |
| ATOM | 4780 | CD2 | TYR | B | 323 | 26.083 | 28.017 | 81.487 | 1.00 | 34.11 | C |
| ATOM | 4781 | CE1 | TYR | B | 323 | 25.766 | 29.495 | 79.179 | 1.00 | 34.11 | C |
| ATOM | 4782 | CE2 | TYR | B | 323 | 27.077 | 27.989 | 80.506 | 1.00 | 34.11 | C |
| ATOM | 4783 | CZ | TYR | B | 323 | 26.911 | 28.728 | 79.363 | 1.00 | 34.11 | C |
| ATOM | 4784 | OH | TYR | B | 323 | 27.897 | 28.706 | 78.417 | 1.00 | 34.11 | O |
| ATOM | 4785 | N | THR | B | 324 | 21.887 | 29.430 | 84.521 | 1.00 | 28.09 | N |
| ATOM | 4786 | CA | THR | B | 324 | 20.607 | 29.266 | 85.204 | 1.00 | 28.09 | C |
| ATOM | 4787 | C | THR | B | 324 | 20.200 | 30.584 | 85.847 | 1.00 | 28.09 | C |
| ATOM | 4788 | O | THR | B | 324 | 20.772 | 30.975 | 86.850 | 1.00 | 28.09 | O |
| ATOM | 4789 | CB | THR | B | 324 | 20.800 | 28.120 | 86.240 | 1.00 | 39.41 | C |
| ATOM | 4790 | OG1 | THR | B | 324 | 20.698 | 26.874 | 85.543 | 1.00 | 39.41 | O |
| ATOM | 4791 | CG2 | THR | B | 324 | 19.828 | 28.199 | 87.455 | 1.00 | 39.41 | C |
| ATOM | 4792 | N | PRO | B | 325 | 19.230 | 31.310 | 85.254 | 1.00 | 38.29 | N |
| ATOM | 4793 | CA | PRO | B | 325 | 18.811 | 32.592 | 85.843 | 1.00 | 38.29 | C |
| ATOM | 4794 | C | PRO | B | 325 | 18.619 | 32.508 | 87.352 | 1.00 | 38.29 | C |
| ATOM | 4795 | O | PRO | B | 325 | 19.221 | 33.265 | 88.131 | 1.00 | 38.29 | O |
| ATOM | 4796 | CB | PRO | B | 325 | 17.504 | 32.904 | 85.111 | 1.00 | 33.50 | C |
| ATOM | 4797 | CG | PRO | B | 325 | 17.756 | 32.345 | 83.741 | 1.00 | 33.50 | C |
| ATOM | 4798 | CD | PRO | B | 325 | 18.442 | 31.009 | 84.043 | 1.00 | 33.50 | C |
| ATOM | 4799 | N | THR | B | 326 | 17.779 | 31.565 | 87.756 | 1.00 | 37.45 | N |
| ATOM | 4800 | CA | THR | B | 326 | 17.493 | 31.366 | 89.167 | 1.00 | 37.45 | C |
| ATOM | 4801 | C | THR | B | 326 | 18.735 | 31.103 | 89.999 | 1.00 | 37.45 | C |
| ATOM | 4802 | O | THR | B | 326 | 18.666 | 31.113 | 91.221 | 1.00 | 37.45 | O |
| ATOM | 4803 | CB | THR | B | 326 | 16.520 | 30.202 | 89.375 | 1.00 | 32.11 | C |
| ATOM | 4804 | OG1 | THR | B | 326 | 16.986 | 29.061 | 88.650 | 1.00 | 32.11 | O |
| ATOM | 4805 | CG2 | THR | B | 326 | 15.131 | 30.576 | 88.889 | 1.00 | 32.11 | C |
| ATOM | 4806 | N | ALA | B | 327 | 19.870 | 30.867 | 89.357 | 1.00 | 27.18 | N |
| ATOM | 4807 | CA | ALA | B | 327 | 21.079 | 30.606 | 90.119 | 1.00 | 27.18 | C |
| ATOM | 4808 | C | ALA | B | 327 | 21.866 | 31.887 | 90.319 | 1.00 | 27.18 | C |
| ATOM | 4809 | O | ALA | B | 327 | 22.786 | 31.949 | 91.131 | 1.00 | 27.18 | O |
| ATOM | 4810 | CB | ALA | B | 327 | 21.928 | 29.582 | 89.413 | 1.00 | 13.76 | C |
| ATOM | 4811 | N | ARG | B | 328 | 21.502 | 32.916 | 89.568 | 1.00 | 31.26 | N |
| ATOM | 4812 | CA | ARG | B | 328 | 22.193 | 34.192 | 89.660 | 1.00 | 31.26 | C |
| ATOM | 4813 | C | ARG | B | 328 | 21.819 | 34.903 | 90.939 | 1.00 | 31.26 | C |
| ATOM | 4814 | O | ARG | B | 328 | 20.665 | 34.882 | 91.343 | 1.00 | 31.26 | O |
| ATOM | 4815 | CB | ARG | B | 328 | 21.815 | 35.073 | 88.480 | 1.00 | 27.28 | C |
| ATOM | 4816 | CG | ARG | B | 328 | 22.131 | 34.458 | 87.153 | 1.00 | 27.28 | C |
| ATOM | 4817 | CD | ARG | B | 328 | 21.522 | 35.230 | 85.997 | 1.00 | 27.28 | C |
| ATOM | 4818 | NE | ARG | B | 328 | 21.710 | 34.480 | 84.763 | 1.00 | 27.28 | N |
| ATOM | 4819 | CZ | ARG | B | 328 | 20.940 | 34.584 | 83.691 | 1.00 | 27.28 | C |
| ATOM | 4820 | NH1 | ARG | B | 328 | 19.910 | 35.419 | 83.674 | 1.00 | 27.28 | N |
| ATOM | 4821 | NH2 | ARG | B | 328 | 21.202 | 33.830 | 82.640 | 1.00 | 27.28 | N |
| ATOM | 4822 | N | LEU | B | 329 | 22.798 | 35.528 | 91.580 | 1.00 | 27.49 | N |
| ATOM | 4823 | CA | LEU | B | 329 | 22.535 | 36.285 | 92.796 | 1.00 | 27.49 | C |
| ATOM | 4824 | C | LEU | B | 329 | 21.405 | 37.287 | 92.542 | 1.00 | 27.49 | C |
| ATOM | 4825 | O | LEU | B | 329 | 21.132 | 37.665 | 91.407 | 1.00 | 27.49 | O |
| ATOM | 4826 | CB | LEU | B | 329 | 23.790 | 37.046 | 93.211 | 1.00 | 20.62 | C |
| ATOM | 4827 | CG | LEU | B | 329 | 24.734 | 36.415 | 94.230 | 1.00 | 20.62 | C |
| ATOM | 4828 | CD1 | LEU | B | 329 | 24.805 | 34.914 | 94.029 | 1.00 | 20.62 | C |
| ATOM | 4829 | CD2 | LEU | B | 329 | 26.101 | 37.061 | 94.096 | 1.00 | 20.62 | C |
| ATOM | 4830 | N | THR | B | 330 | 20.726 | 37.697 | 93.601 | 1.00 | 30.40 | N |
| ATOM | 4831 | CA | THR | B | 330 | 19.672 | 38.689 | 93.462 | 1.00 | 30.40 | C |
| ATOM | 4832 | C | THR | B | 330 | 20.386 | 40.012 | 93.727 | 1.00 | 30.40 | C |

| | | | | | | | | | | | |
|---|---|---|---|---|---|---|---|---|---|---|---|
| ATOM | 4833 | O | THR | B | 330 | 21.402 | 40.042 | 94.407 | 1.00 | 30.40 | O |
| ATOM | 4834 | CB | THR | B | 330 | 18.553 | 38.472 | 94.492 | 1.00 | 29.69 | C |
| ATOM | 4835 | OG1 | THR | B | 330 | 19.054 | 38.746 | 95.801 | 1.00 | 29.69 | O |
| ATOM | 4836 | CG2 | THR | B | 330 | 18.049 | 37.039 | 94.434 | 0.00 | 29.69 | C |
| ATOM | 4837 | N | PRO | B | 331 | 19.883 | 41.115 | 93.175 | 1.00 | 22.73 | N |
| ATOM | 4838 | CA | PRO | B | 331 | 20.533 | 42.406 | 93.393 | 1.00 | 22.73 | C |
| ATOM | 4839 | C | PRO | B | 331 | 20.918 | 42.659 | 94.848 | 1.00 | 22.73 | C |
| ATOM | 4840 | O | PRO | B | 331 | 22.038 | 43.087 | 95.148 | 1.00 | 22.73 | O |
| ATOM | 4841 | CB | PRO | B | 331 | 19.494 | 43.392 | 92.892 | 1.00 | 23.90 | C |
| ATOM | 4842 | CG | PRO | B | 331 | 18.882 | 42.654 | 91.771 | 1.00 | 23.90 | C |
| ATOM | 4843 | CD | PRO | B | 331 | 18.677 | 41.270 | 92.350 | 1.00 | 23.90 | C |
| ATOM | 4844 | N | LEU | B | 332 | 19.985 | 42.370 | 95.751 | 1.00 | 37.71 | N |
| ATOM | 4845 | CA | LEU | B | 332 | 20.204 | 42.591 | 97.174 | 1.00 | 37.71 | C |
| ATOM | 4846 | C | LEU | B | 332 | 21.293 | 41.690 | 97.718 | 1.00 | 37.71 | C |
| ATOM | 4847 | O | LEU | B | 332 | 22.073 | 42.109 | 98.574 | 1.00 | 37.71 | O |
| ATOM | 4848 | CB | LEU | B | 332 | 18.911 | 42.360 | 97.953 | 1.00 | 28.88 | C |
| ATOM | 4849 | CG | LEU | B | 332 | 18.872 | 43.032 | 99.324 | 1.00 | 28.88 | C |
| ATOM | 4850 | CD1 | LEU | B | 332 | 18.844 | 44.524 | 99.135 | 1.00 | 28.88 | C |
| ATOM | 4851 | CD2 | LEU | B | 332 | 17.647 | 42.598 | 100.097 | 1.00 | 28.88 | C |
| ATOM | 4852 | N | GLU | B | 333 | 21.340 | 40.452 | 97.232 | 1.00 | 29.59 | N |
| ATOM | 4853 | CA | GLU | B | 333 | 22.359 | 39.512 | 97.682 | 1.00 | 29.59 | C |
| ATOM | 4854 | C | GLU | B | 333 | 23.692 | 40.014 | 97.190 | 1.00 | 29.59 | C |
| ATOM | 4855 | O | GLU | B | 333 | 24.692 | 39.908 | 97.879 | 1.00 | 29.59 | O |
| ATOM | 4856 | CB | GLU | B | 333 | 22.110 | 38.108 | 97.131 | 1.00 | 45.27 | C |
| ATOM | 4857 | CG | GLU | B | 333 | 20.891 | 37.404 | 97.706 | 1.00 | 45.27 | C |
| ATOM | 4858 | CD | GLU | B | 333 | 20.557 | 36.131 | 96.943 | 1.00 | 45.27 | C |
| ATOM | 4859 | OE1 | GLU | B | 333 | 20.941 | 36.048 | 95.752 | 1.00 | 45.27 | O |
| ATOM | 4860 | OE2 | GLU | B | 333 | 19.901 | 35.222 | 97.512 | 1.00 | 45.27 | O |
| ATOM | 4861 | N | ALA | B | 334 | 23.698 | 40.571 | 95.990 | 1.00 | 30.95 | N |
| ATOM | 4862 | CA | ALA | B | 334 | 24.915 | 41.106 | 95.414 | 1.00 | 30.95 | C |
| ATOM | 4863 | C | ALA | B | 334 | 25.469 | 42.237 | 96.293 | 1.00 | 30.95 | C |
| ATOM | 4864 | O | ALA | B | 334 | 26.674 | 42.319 | 96.535 | 1.00 | 30.95 | O |
| ATOM | 4865 | CB | ALA | B | 334 | 24.632 | 41.610 | 94.000 | 1.00 | 48.94 | C |
| ATOM | 4866 | N | CYS | B | 335 | 24.587 | 43.103 | 96.772 | 1.00 | 29.48 | N |
| ATOM | 4867 | CA | CYS | B | 335 | 25.008 | 44.201 | 97.626 | 1.00 | 29.48 | C |
| ATOM | 4868 | C | CYS | B | 335 | 25.680 | 43.692 | 98.899 | 1.00 | 29.48 | C |
| ATOM | 4869 | O | CYS | B | 335 | 26.563 | 44.349 | 99.449 | 1.00 | 29.48 | O |
| ATOM | 4870 | CB | CYS | B | 335 | 23.809 | 45.067 | 98.005 | 1.00 | 30.52 | C |
| ATOM | 4871 | SG | CYS | B | 335 | 23.177 | 46.115 | 96.668 | 1.00 | 30.52 | S |
| ATOM | 4872 | N | ALA | B | 336 | 25.278 | 42.513 | 99.356 | 1.00 | 28.04 | N |
| ATOM | 4873 | CA | ALA | B | 336 | 25.826 | 41.953 | 100.576 | 1.00 | 28.04 | C |
| ATOM | 4874 | C | ALA | B | 336 | 27.040 | 41.064 | 100.375 | 1.00 | 28.04 | C |
| ATOM | 4875 | O | ALA | B | 336 | 27.432 | 40.334 | 101.285 | 1.00 | 28.04 | O |
| ATOM | 4876 | CB | ALA | B | 336 | 24.746 | 41.184 | 101.314 | 1.00 | 27.36 | C |
| ATOM | 4877 | N | HIS | B | 337 | 27.641 | 41.133 | 99.196 | 1.00 | 28.78 | N |
| ATOM | 4878 | CA | HIS | B | 337 | 28.813 | 40.322 | 98.870 | 1.00 | 28.78 | C |
| ATOM | 4879 | C | HIS | B | 337 | 30.114 | 40.906 | 99.455 | 1.00 | 28.78 | C |
| ATOM | 4880 | O | HIS | B | 337 | 30.266 | 42.121 | 99.595 | 1.00 | 28.78 | O |
| ATOM | 4881 | CB | HIS | B | 337 | 28.899 | 40.192 | 97.348 | 1.00 | 32.11 | C |
| ATOM | 4882 | CG | HIS | B | 337 | 29.796 | 39.093 | 96.871 | 1.00 | 32.11 | C |
| ATOM | 4883 | ND1 | HIS | B | 337 | 31.166 | 39.234 | 96.781 | 1.00 | 32.11 | N |
| ATOM | 4884 | CD2 | HIS | B | 337 | 29.514 | 37.848 | 96.431 | 1.00 | 32.11 | C |
| ATOM | 4885 | CE1 | HIS | B | 337 | 31.686 | 38.121 | 96.303 | 1.00 | 32.11 | C |
| ATOM | 4886 | NE2 | HIS | B | 337 | 30.707 | 37.261 | 96.081 | 1.00 | 32.11 | N |
| ATOM | 4887 | N | SER | B | 338 | 31.049 | 40.030 | 99.802 | 1.00 | 34.64 | N |
| ATOM | 4888 | CA | SER | B | 338 | 32.325 | 40.448 | 100.383 | 1.00 | 34.64 | C |
| ATOM | 4889 | C | SER | B | 338 | 33.040 | 41.528 | 99.585 | 1.00 | 34.64 | C |
| ATOM | 4890 | O | SER | B | 338 | 33.648 | 42.441 | 100.144 | 1.00 | 34.64 | O |
| ATOM | 4891 | CB | SER | B | 338 | 33.248 | 39.248 | 100.505 | 1.00 | 44.51 | C |
| ATOM | 4892 | OG | SER | B | 338 | 32.578 | 38.205 | 101.179 | 1.00 | 44.51 | O |
| ATOM | 4893 | N | PHE | B | 339 | 32.975 | 41.410 | 98.267 | 1.00 | 32.14 | N |
| ATOM | 4894 | CA | PHE | B | 339 | 33.630 | 42.368 | 97.403 | 1.00 | 32.14 | C |

| | | | | | | | | | | | |
|---|---|---|---|---|---|---|---|---|---|---|---|
| ATOM | 4895 | C | PHE | B | 339 | 33.272 | 43.805 | 97.768 | 1.00 | 32.14 | C |
| ATOM | 4896 | O | PHE | B | 339 | 34.001 | 44.733 | 97.436 | 1.00 | 32.14 | O |
| ATOM | 4897 | CB | PHE | B | 339 | 33.262 | 42.078 | 95.948 | 1.00 | 36.64 | C |
| ATOM | 4898 | CG | PHE | B | 339 | 33.869 | 43.037 | 94.973 | 1.00 | 36.64 | C |
| ATOM | 4899 | CD1 | PHE | B | 339 | 35.251 | 43.240 | 94.943 | 1.00 | 36.64 | C |
| ATOM | 4900 | CD2 | PHE | B | 339 | 33.063 | 43.785 | 94.120 | 1.00 | 36.64 | C |
| ATOM | 4901 | CE1 | PHE | B | 339 | 35.819 | 44.185 | 94.084 | 1.00 | 36.64 | C |
| ATOM | 4902 | CE2 | PHE | B | 339 | 33.623 | 44.730 | 93.258 | 1.00 | 36.64 | C |
| ATOM | 4903 | CZ | PHE | B | 339 | 35.003 | 44.929 | 93.242 | 1.00 | 36.64 | C |
| ATOM | 4904 | N | PHE | B | 340 | 32.162 | 43.990 | 98.472 | 1.00 | 31.47 | N |
| ATOM | 4905 | CA | PHE | B | 340 | 31.731 | 45.329 | 98.843 | 1.00 | 31.47 | C |
| ATOM | 4906 | C | PHE | B | 340 | 31.962 | 45.655 | 100.318 | 1.00 | 31.47 | C |
| ATOM | 4907 | O | PHE | B | 340 | 31.581 | 46.727 | 100.790 | 1.00 | 31.47 | O |
| ATOM | 4908 | CB | PHE | B | 340 | 30.253 | 45.507 | 98.488 | 1.00 | 26.64 | C |
| ATOM | 4909 | CG | PHE | B | 340 | 29.964 | 45.430 | 97.001 | 1.00 | 26.64 | C |
| ATOM | 4910 | CD1 | PHE | B | 340 | 30.567 | 46.319 | 96.111 | 1.00 | 26.64 | C |
| ATOM | 4911 | CD2 | PHE | B | 340 | 29.084 | 44.479 | 96.492 | 1.00 | 26.64 | C |
| ATOM | 4912 | CE1 | PHE | B | 340 | 30.297 | 46.258 | 94.754 | 1.00 | 26.64 | C |
| ATOM | 4913 | CE2 | PHE | B | 340 | 28.813 | 44.419 | 95.129 | 1.00 | 26.64 | C |
| ATOM | 4914 | CZ | PHE | B | 340 | 29.418 | 45.306 | 94.266 | 1.00 | 26.64 | C |
| ATOM | 4915 | N | ASP | B | 341 | 32.606 | 44.737 | 101.034 | 1.00 | 32.86 | N |
| ATOM | 4916 | CA | ASP | B | 341 | 32.881 | 44.920 | 102.455 | 1.00 | 32.86 | C |
| ATOM | 4917 | C | ASP | B | 341 | 33.642 | 46.211 | 102.736 | 1.00 | 32.86 | C |
| ATOM | 4918 | O | ASP | B | 341 | 33.342 | 46.914 | 103.697 | 1.00 | 32.86 | O |
| ATOM | 4919 | CB | ASP | B | 341 | 33.675 | 43.733 | 103.017 | 1.00 | 37.27 | C |
| ATOM | 4920 | CG | ASP | B | 341 | 32.860 | 42.449 | 103.078 | 1.00 | 37.27 | C |
| ATOM | 4921 | OD1 | ASP | B | 341 | 31.631 | 42.494 | 102.863 | 1.00 | 37.27 | O |
| ATOM | 4922 | OD2 | ASP | B | 341 | 33.448 | 41.387 | 103.354 | 1.00 | 37.27 | O |
| ATOM | 4923 | N | GLU | B | 342 | 34.627 | 46.530 | 101.913 | 1.00 | 26.18 | N |
| ATOM | 4924 | CA | GLU | B | 342 | 35.378 | 47.752 | 102.130 | 1.00 | 26.18 | C |
| ATOM | 4925 | C | GLU | B | 342 | 34.472 | 48.972 | 102.191 | 1.00 | 26.18 | C |
| ATOM | 4926 | O | GLU | B | 342 | 34.774 | 49.953 | 102.868 | 1.00 | 26.18 | O |
| ATOM | 4927 | CB | GLU | B | 342 | 36.405 | 47.953 | 101.024 | 1.00 | 50.98 | C |
| ATOM | 4928 | CG | GLU | B | 342 | 36.912 | 49.371 | 100.949 | 1.00 | 50.98 | C |
| ATOM | 4929 | CD | GLU | B | 342 | 38.091 | 49.527 | 100.013 | 1.00 | 50.98 | C |
| ATOM | 4930 | OE1 | GLU | B | 342 | 38.063 | 48.924 | 98.917 | 1.00 | 50.98 | O |
| ATOM | 4931 | OE2 | GLU | B | 342 | 39.044 | 50.264 | 100.371 | 1.00 | 50.98 | O |
| ATOM | 4932 | N | LEU | B | 343 | 33.358 | 48.927 | 101.475 | 1.00 | 34.06 | N |
| ATOM | 4933 | CA | LEU | B | 343 | 32.474 | 50.070 | 101.485 | 1.00 | 34.06 | C |
| ATOM | 4934 | C | LEU | B | 343 | 31.814 | 50.187 | 102.838 | 1.00 | 34.06 | C |
| ATOM | 4935 | O | LEU | B | 343 | 31.289 | 51.240 | 103.192 | 1.00 | 34.06 | O |
| ATOM | 4936 | CB | LEU | B | 343 | 31.414 | 49.933 | 100.404 | 1.00 | 24.96 | C |
| ATOM | 4937 | CG | LEU | B | 343 | 31.901 | 49.837 | 98.966 | 1.00 | 24.96 | C |
| ATOM | 4938 | CD1 | LEU | B | 343 | 30.687 | 49.878 | 98.062 | 1.00 | 24.96 | C |
| ATOM | 4939 | CD2 | LEU | B | 343 | 32.850 | 50.977 | 98.638 | 1.00 | 24.96 | C |
| ATOM | 4940 | N | ARG | B | 344 | 31.842 | 49.104 | 103.604 | 1.00 | 39.71 | N |
| ATOM | 4941 | CA | ARG | B | 344 | 31.225 | 49.120 | 104.920 | 1.00 | 39.71 | C |
| ATOM | 4942 | C | ARG | B | 344 | 32.187 | 49.511 | 106.043 | 1.00 | 39.71 | C |
| ATOM | 4943 | O | ARG | B | 344 | 31.788 | 49.624 | 107.213 | 1.00 | 39.71 | O |
| ATOM | 4944 | CB | ARG | B | 344 | 30.571 | 47.764 | 105.206 | 1.00 | 27.83 | C |
| ATOM | 4945 | CG | ARG | B | 344 | 29.214 | 47.610 | 104.535 | 1.00 | 27.83 | C |
| ATOM | 4946 | CD | ARG | B | 344 | 28.552 | 46.304 | 104.894 | 1.00 | 27.83 | C |
| ATOM | 4947 | NE | ARG | B | 344 | 29.096 | 45.169 | 104.150 | 1.00 | 27.83 | N |
| ATOM | 4948 | CZ | ARG | B | 344 | 28.827 | 44.909 | 102.874 | 1.00 | 27.83 | C |
| ATOM | 4949 | NH1 | ARG | B | 344 | 28.017 | 45.701 | 102.186 | 1.00 | 27.83 | N |
| ATOM | 4950 | NH2 | ARG | B | 344 | 29.358 | 43.850 | 102.287 | 1.00 | 27.83 | N |
| ATOM | 4951 | N | ASP | B | 345 | 33.449 | 49.726 | 105.679 | 1.00 | 40.61 | N |
| ATOM | 4952 | CA | ASP | B | 345 | 34.462 | 50.118 | 106.642 | 1.00 | 40.61 | C |
| ATOM | 4953 | C | ASP | B | 345 | 34.264 | 51.577 | 107.004 | 1.00 | 40.61 | C |
| ATOM | 4954 | O | ASP | B | 345 | 34.430 | 52.453 | 106.153 | 1.00 | 40.61 | O |
| ATOM | 4955 | CB | ASP | B | 345 | 35.862 | 49.966 | 106.056 | 1.00 | 75.95 | C |
| ATOM | 4956 | CG | ASP | B | 345 | 36.951 | 50.326 | 107.063 | 1.00 | 75.95 | C |

| | | | | | | | | | | | |
|---|---|---|---|---|---|---|---|---|---|---|---|
| ATOM | 4957 | OD1 | ASP | B | 345 | 37.513 | 49.393 | 107.685 | 1.00 | 75.95 | O |
| ATOM | 4958 | OD2 | ASP | B | 345 | 37.226 | 51.539 | 107.248 | 1.00 | 75.95 | O |
| ATOM | 4959 | N | PRO | B | 346 | 33.925 | 51.864 | 108.273 | 1.00 | 38.56 | N |
| ATOM | 4960 | CA | PRO | B | 346 | 33.709 | 53.245 | 108.732 | 1.00 | 38.56 | C |
| ATOM | 4961 | C | PRO | B | 346 | 34.832 | 54.219 | 108.355 | 1.00 | 38.56 | C |
| ATOM | 4962 | O | PRO | B | 346 | 34.630 | 55.433 | 108.333 | 1.00 | 38.56 | O |
| ATOM | 4963 | CB | PRO | B | 346 | 33.534 | 53.090 | 110.244 | 1.00 | 38.64 | C |
| ATOM | 4964 | CG | PRO | B | 346 | 34.215 | 51.760 | 110.561 | 1.00 | 38.64 | C |
| ATOM | 4965 | CD | PRO | B | 346 | 33.818 | 50.911 | 109.390 | 1.00 | 38.64 | C |
| ATOM | 4966 | N | ASN | B | 347 | 36.007 | 53.687 | 108.036 | 1.00 | 46.79 | N |
| ATOM | 4967 | CA | ASN | B | 347 | 37.134 | 54.529 | 107.647 | 1.00 | 46.79 | C |
| ATOM | 4968 | C | ASN | B | 347 | 37.246 | 54.724 | 106.137 | 1.00 | 46.79 | C |
| ATOM | 4969 | O | ASN | B | 347 | 38.087 | 55.494 | 105.683 | 1.00 | 46.79 | O |
| ATOM | 4970 | CB | ASN | B | 347 | 38.451 | 53.925 | 108.137 | 1.00 | 61.20 | C |
| ATOM | 4971 | CG | ASN | B | 347 | 38.593 | 53.963 | 109.649 | 1.00 | 61.20 | C |
| ATOM | 4972 | OD1 | ASN | B | 347 | 38.934 | 52.953 | 110.270 | 1.00 | 61.20 | O |
| ATOM | 4973 | ND2 | ASN | B | 347 | 38.346 | 55.135 | 110.251 | 1.00 | 61.20 | N |
| ATOM | 4974 | N | VAL | B | 348 | 36.428 | 54.030 | 105.349 | 1.00 | 41.91 | N |
| ATOM | 4975 | CA | VAL | B | 348 | 36.543 | 54.181 | 103.903 | 1.00 | 41.91 | C |
| ATOM | 4976 | C | VAL | B | 348 | 36.306 | 55.630 | 103.548 | 1.00 | 41.91 | C |
| ATOM | 4977 | O | VAL | B | 348 | 35.518 | 56.314 | 104.194 | 1.00 | 41.91 | O |
| ATOM | 4978 | CB | VAL | B | 348 | 35.557 | 53.271 | 103.122 | 1.00 | 64.67 | C |
| ATOM | 4979 | CG1 | VAL | B | 348 | 34.114 | 53.726 | 103.325 | 1.00 | 64.67 | C |
| ATOM | 4980 | CG2 | VAL | B | 348 | 35.918 | 53.290 | 101.656 | 1.00 | 64.67 | C |
| ATOM | 4981 | N | LYS | B | 349 | 37.005 | 56.091 | 102.524 | 1.00 | 28.60 | N |
| ATOM | 4982 | CA | LYS | B | 349 | 36.925 | 57.476 | 102.083 | 1.00 | 28.60 | C |
| ATOM | 4983 | C | LYS | B | 349 | 37.144 | 57.452 | 100.573 | 1.00 | 28.60 | C |
| ATOM | 4984 | O | LYS | B | 349 | 37.487 | 56.403 | 100.013 | 1.00 | 28.60 | O |
| ATOM | 4985 | CB | LYS | B | 349 | 38.055 | 58.268 | 102.758 | 1.00 | 44.54 | C |
| ATOM | 4986 | CG | LYS | B | 349 | 37.668 | 59.588 | 103.429 | 1.00 | 44.54 | C |
| ATOM | 4987 | CD | LYS | B | 349 | 36.807 | 59.365 | 104.642 | 1.00 | 44.54 | C |
| ATOM | 4988 | CE | LYS | B | 349 | 36.492 | 60.677 | 105.339 | 1.00 | 44.54 | C |
| ATOM | 4989 | NZ | LYS | B | 349 | 35.555 | 60.486 | 106.503 | 1.00 | 44.54 | N |
| ATOM | 4990 | N | LEU | B | 350 | 36.947 | 58.582 | 99.901 | 1.00 | 36.18 | N |
| ATOM | 4991 | CA | LEU | B | 350 | 37.182 | 58.618 | 98.449 | 1.00 | 36.18 | C |
| ATOM | 4992 | C | LEU | B | 350 | 38.595 | 59.111 | 98.182 | 1.00 | 36.18 | C |
| ATOM | 4993 | O | LEU | B | 350 | 39.129 | 59.889 | 98.958 | 1.00 | 36.18 | O |
| ATOM | 4994 | CB | LEU | B | 350 | 36.194 | 59.553 | 97.750 | 1.00 | 17.38 | C |
| ATOM | 4995 | CG | LEU | B | 350 | 34.728 | 59.116 | 97.829 | 1.00 | 17.38 | C |
| ATOM | 4996 | CD1 | LEU | B | 350 | 33.837 | 60.106 | 97.099 | 1.00 | 17.38 | C |
| ATOM | 4997 | CD2 | LEU | B | 350 | 34.576 | 57.735 | 97.223 | 1.00 | 17.38 | C |
| ATOM | 4998 | N | PRO | B | 351 | 39.235 | 58.647 | 97.099 | 1.00 | 39.56 | N |
| ATOM | 4999 | CA | PRO | B | 351 | 40.597 | 59.115 | 96.807 | 1.00 | 39.56 | C |
| ATOM | 5000 | C | PRO | B | 351 | 40.536 | 60.636 | 96.726 | 1.00 | 39.56 | C |
| ATOM | 5001 | O | PRO | B | 351 | 41.532 | 61.349 | 96.749 | 1.00 | 39.56 | O |
| ATOM | 5002 | CB | PRO | B | 351 | 40.909 | 58.456 | 95.464 | 1.00 | 37.60 | C |
| ATOM | 5003 | CG | PRO | B | 351 | 39.558 | 58.185 | 94.880 | 1.00 | 37.60 | C |
| ATOM | 5004 | CD | PRO | B | 351 | 38.760 | 57.727 | 96.057 | 1.00 | 37.60 | C |
| ATOM | 5005 | N | ASN | B | 352 | 39.306 | 61.098 | 96.652 | 1.00 | 35.58 | N |
| ATOM | 5006 | CA | ASN | B | 352 | 38.936 | 62.496 | 96.588 | 1.00 | 35.58 | C |
| ATOM | 5007 | C | ASN | B | 352 | 39.244 | 63.163 | 97.934 | 1.00 | 35.58 | C |
| ATOM | 5008 | O | ASN | B | 352 | 39.546 | 64.354 | 98.023 | 1.00 | 35.58 | O |
| ATOM | 5009 | CB | ASN | B | 352 | 37.433 | 62.533 | 96.340 | 1.00 | 47.59 | C |
| ATOM | 5010 | CG | ASN | B | 352 | 36.962 | 63.848 | 95.871 | 1.00 | 47.59 | C |
| ATOM | 5011 | OD1 | ASN | B | 352 | 35.765 | 64.091 | 95.821 | 1.00 | 47.59 | O |
| ATOM | 5012 | ND2 | ASN | B | 352 | 37.893 | 64.720 | 95.504 | 1.00 | 47.59 | N |
| ATOM | 5013 | N | GLY | B | 353 | 39.135 | 62.371 | 98.989 | 1.00 | 33.34 | N |
| ATOM | 5014 | CA | GLY | B | 353 | 39.371 | 62.879 | 100.318 | 1.00 | 33.34 | C |
| ATOM | 5015 | C | GLY | B | 353 | 38.032 | 63.055 | 100.996 | 1.00 | 33.34 | C |
| ATOM | 5016 | O | GLY | B | 353 | 37.930 | 63.006 | 102.221 | 1.00 | 33.34 | O |
| ATOM | 5017 | N | ARG | B | 354 | 36.996 | 63.252 | 100.192 | 1.00 | 30.07 | N |
| ATOM | 5018 | CA | ARG | B | 354 | 35.676 | 63.443 | 100.751 | 1.00 | 30.07 | C |

| | | | | | | | | | | | |
|---|---|---|---|---|---|---|---|---|---|---|---|
| ATOM | 5019 | C | ARG | B | 354 | 34.939 | 62.131 | 101.015 | 1.00 | 30.07 | C |
| ATOM | 5020 | O | ARG | B | 354 | 35.330 | 61.077 | 100.537 | 1.00 | 30.07 | O |
| ATOM | 5021 | CB | ARG | B | 354 | 34.846 | 64.377 | 99.853 | 1.00 | 49.54 | C |
| ATOM | 5022 | CG | ARG | B | 354 | 34.388 | 63.798 | 98.538 | 1.00 | 49.54 | C |
| ATOM | 5023 | CD | ARG | B | 354 | 33.411 | 64.742 | 97.847 | 1.00 | 49.54 | C |
| ATOM | 5024 | NE | ARG | B | 354 | 34.067 | 65.898 | 97.239 | 1.00 | 49.54 | N |
| ATOM | 5025 | CZ | ARG | B | 354 | 33.420 | 66.860 | 96.580 | 1.00 | 49.54 | C |
| ATOM | 5026 | NH1 | ARG | B | 354 | 32.092 | 66.809 | 96.449 | 1.00 | 49.54 | N |
| ATOM | 5027 | NH2 | ARG | B | 354 | 34.099 | 67.864 | 96.035 | 1.00 | 49.54 | N |
| ATOM | 5028 | N | ASP | B | 355 | 33.879 | 62.213 | 101.809 | 1.00 | 49.23 | N |
| ATOM | 5029 | CA | ASP | B | 355 | 33.065 | 61.056 | 102.176 | 1.00 | 49.23 | C |
| ATOM | 5030 | C | ASP | B | 355 | 32.365 | 60.300 | 101.057 | 1.00 | 49.23 | C |
| ATOM | 5031 | O | ASP | B | 355 | 31.963 | 60.890 | 100.041 | 1.00 | 49.23 | O |
| ATOM | 5032 | CB | ASP | B | 355 | 31.984 | 61.472 | 103.161 | 1.00 | 56.92 | C |
| ATOM | 5033 | CG | ASP | B | 355 | 32.476 | 61.513 | 104.572 | 1.00 | 56.92 | C |
| ATOM | 5034 | OD1 | ASP | B | 355 | 31.641 | 61.735 | 105.475 | 1.00 | 56.92 | O |
| ATOM | 5035 | OD2 | ASP | B | 355 | 33.694 | 61.323 | 104.783 | 1.00 | 56.92 | O |
| ATOM | 5036 | N | THR | B | 356 | 32.198 | 58.993 | 101.276 | 1.00 | 26.81 | N |
| ATOM | 5037 | CA | THR | B | 356 | 31.490 | 58.151 | 100.326 | 1.00 | 26.81 | C |
| ATOM | 5038 | C | THR | B | 356 | 30.038 | 58.613 | 100.351 | 1.00 | 26.81 | C |
| ATOM | 5039 | O | THR | B | 356 | 29.563 | 59.173 | 101.345 | 1.00 | 26.81 | O |
| ATOM | 5040 | CB | THR | B | 356 | 31.516 | 56.657 | 100.716 | 1.00 | 34.33 | C |
| ATOM | 5041 | OG1 | THR | B | 356 | 31.109 | 56.518 | 102.076 | 1.00 | 34.33 | O |
| ATOM | 5042 | CG2 | THR | B | 356 | 32.901 | 56.064 | 100.537 | 1.00 | 34.33 | C |
| ATOM | 5043 | N | PRO | B | 357 | 29.313 | 58.391 | 99.252 | 1.00 | 29.88 | N |
| ATOM | 5044 | CA | PRO | B | 357 | 27.917 | 58.813 | 99.219 | 1.00 | 29.88 | C |
| ATOM | 5045 | C | PRO | B | 357 | 27.040 | 57.896 | 100.062 | 1.00 | 29.88 | C |
| ATOM | 5046 | O | PRO | B | 357 | 27.538 | 56.970 | 100.716 | 1.00 | 29.88 | O |
| ATOM | 5047 | CB | PRO | B | 357 | 27.588 | 58.736 | 97.739 | 1.00 | 26.18 | C |
| ATOM | 5048 | CG | PRO | B | 357 | 28.363 | 57.520 | 97.317 | 1.00 | 26.18 | C |
| ATOM | 5049 | CD | PRO | B | 357 | 29.703 | 57.761 | 97.977 | 1.00 | 26.18 | C |
| ATOM | 5050 | N | ALA | B | 358 | 25.738 | 58.180 | 100.046 | 1.00 | 21.54 | N |
| ATOM | 5051 | CA | ALA | B | 358 | 24.739 | 57.390 | 100.758 | 1.00 | 21.54 | C |
| ATOM | 5052 | C | ALA | B | 358 | 24.842 | 55.973 | 100.243 | 1.00 | 21.54 | C |
| ATOM | 5053 | O | ALA | B | 358 | 24.737 | 55.724 | 99.044 | 1.00 | 21.54 | O |
| ATOM | 5054 | CB | ALA | B | 358 | 23.354 | 57.931 | 100.490 | 1.00 | 14.47 | C |
| ATOM | 5055 | N | LEU | B | 359 | 25.043 | 55.041 | 101.154 | 1.00 | 24.66 | N |
| ATOM | 5056 | CA | LEU | B | 359 | 25.196 | 53.659 | 100.779 | 1.00 | 24.66 | C |
| ATOM | 5057 | C | LEU | B | 359 | 24.280 | 52.758 | 101.583 | 1.00 | 24.66 | C |
| ATOM | 5058 | O | LEU | B | 359 | 23.837 | 51.718 | 101.102 | 1.00 | 24.66 | O |
| ATOM | 5059 | CB | LEU | B | 359 | 26.652 | 53.256 | 101.019 | 1.00 | 26.46 | C |
| ATOM | 5060 | CG | LEU | B | 359 | 27.612 | 53.079 | 99.843 | 1.00 | 26.46 | C |
| ATOM | 5061 | CD1 | LEU | B | 359 | 27.368 | 54.115 | 98.774 | 1.00 | 26.46 | C |
| ATOM | 5062 | CD2 | LEU | B | 359 | 29.036 | 53.150 | 100.365 | 1.00 | 26.46 | C |
| ATOM | 5063 | N | PHE | B | 360 | 23.977 | 53.185 | 102.803 | 1.00 | 28.43 | N |
| ATOM | 5064 | CA | PHE | B | 360 | 23.195 | 52.376 | 103.720 | 1.00 | 28.43 | C |
| ATOM | 5065 | C | PHE | B | 360 | 21.784 | 52.820 | 104.085 | 1.00 | 28.43 | C |
| ATOM | 5066 | O | PHE | B | 360 | 21.155 | 52.225 | 104.959 | 1.00 | 28.43 | O |
| ATOM | 5067 | CB | PHE | B | 360 | 24.021 | 52.192 | 104.994 | 1.00 | 28.12 | C |
| ATOM | 5068 | CG | PHE | B | 360 | 25.465 | 51.928 | 104.728 | 1.00 | 28.12 | C |
| ATOM | 5069 | CD1 | PHE | B | 360 | 25.880 | 50.706 | 104.213 | 1.00 | 28.12 | C |
| ATOM | 5070 | CD2 | PHE | B | 360 | 26.415 | 52.919 | 104.964 | 1.00 | 28.12 | C |
| ATOM | 5071 | CE1 | PHE | B | 360 | 27.218 | 50.479 | 103.916 | 1.00 | 28.12 | C |
| ATOM | 5072 | CE2 | PHE | B | 360 | 27.752 | 52.706 | 104.673 | 1.00 | 28.12 | C |
| ATOM | 5073 | CZ | PHE | B | 360 | 28.161 | 51.478 | 104.153 | 1.00 | 28.12 | C |
| ATOM | 5074 | N | ASN | B | 361 | 21.265 | 53.845 | 103.428 | 1.00 | 25.80 | N |
| ATOM | 5075 | CA | ASN | B | 361 | 19.919 | 54.294 | 103.750 | 1.00 | 25.80 | C |
| ATOM | 5076 | C | ASN | B | 361 | 18.838 | 53.423 | 103.102 | 1.00 | 25.80 | C |
| ATOM | 5077 | O | ASN | B | 361 | 18.014 | 53.907 | 102.347 | 1.00 | 25.80 | O |
| ATOM | 5078 | CB | ASN | B | 361 | 19.754 | 55.749 | 103.329 | 1.00 | 26.59 | C |
| ATOM | 5079 | CG | ASN | B | 361 | 20.045 | 55.957 | 101.879 | 1.00 | 26.59 | C |
| ATOM | 5080 | OD1 | ASN | B | 361 | 20.901 | 55.285 | 101.308 | 1.00 | 26.59 | O |

| | | | | | | | | | | | |
|---|---|---|---|---|---|---|---|---|---|---|---|
| ATOM | 5081 | ND2 | ASN | B | 361 | 19.339 | 56.892 | 101.264 | 1.00 | 26.59 | N |
| ATOM | 5082 | N | PHE | B | 362 | 18.847 | 52.136 | 103.417 | 1.00 | 24.90 | N |
| ATOM | 5083 | CA | PHE | B | 362 | 17.879 | 51.199 | 102.879 | 1.00 | 24.90 | C |
| ATOM | 5084 | C | PHE | B | 362 | 16.496 | 51.424 | 103.445 | 1.00 | 24.90 | C |
| ATOM | 5085 | O | PHE | B | 362 | 16.357 | 51.817 | 104.601 | 1.00 | 24.90 | O |
| ATOM | 5086 | CB | PHE | B | 362 | 18.300 | 49.781 | 103.231 | 1.00 | 31.54 | C |
| ATOM | 5087 | CG | PHE | B | 362 | 19.409 | 49.252 | 102.398 | 1.00 | 31.54 | C |
| ATOM | 5088 | CD1 | PHE | B | 362 | 19.150 | 48.670 | 101.170 | 1.00 | 31.54 | C |
| ATOM | 5089 | CD2 | PHE | B | 362 | 20.715 | 49.294 | 102.856 | 1.00 | 31.54 | C |
| ATOM | 5090 | CE1 | PHE | B | 362 | 20.178 | 48.128 | 100.409 | 1.00 | 31.54 | C |
| ATOM | 5091 | CE2 | PHE | B | 362 | 21.758 | 48.755 | 102.100 | 1.00 | 31.54 | C |
| ATOM | 5092 | CZ | PHE | B | 362 | 21.483 | 48.169 | 100.875 | 1.00 | 31.54 | C |
| ATOM | 5093 | N | THR | B | 363 | 15.474 | 51.170 | 102.633 | 1.00 | 39.25 | N |
| ATOM | 5094 | CA | THR | B | 363 | 14.080 | 51.285 | 103.085 | 1.00 | 39.25 | C |
| ATOM | 5095 | C | THR | B | 363 | 13.514 | 49.868 | 103.089 | 1.00 | 39.25 | C |
| ATOM | 5096 | O | THR | B | 363 | 14.111 | 48.950 | 102.510 | 1.00 | 39.25 | O |
| ATOM | 5097 | CB | THR | B | 363 | 13.184 | 52.137 | 102.146 | 1.00 | 19.80 | C |
| ATOM | 5098 | OG1 | THR | B | 363 | 13.108 | 51.516 | 100.857 | 1.00 | 19.80 | O |
| ATOM | 5099 | CG2 | THR | B | 363 | 13.709 | 53.540 | 102.025 | 1.00 | 19.80 | C |
| ATOM | 5100 | N | THR | B | 364 | 12.364 | 49.690 | 103.736 | 1.00 | 32.91 | N |
| ATOM | 5101 | CA | THR | B | 364 | 11.729 | 48.379 | 103.811 | 1.00 | 32.91 | C |
| ATOM | 5102 | C | THR | B | 364 | 11.472 | 47.855 | 102.401 | 1.00 | 32.91 | C |
| ATOM | 5103 | O | THR | B | 364 | 11.810 | 46.723 | 102.081 | 1.00 | 32.91 | O |
| ATOM | 5104 | CB | THR | B | 364 | 10.385 | 48.447 | 104.552 | 1.00 | 34.16 | C |
| ATOM | 5105 | OG1 | THR | B | 364 | 10.515 | 49.282 | 105.705 | 1.00 | 34.16 | O |
| ATOM | 5106 | CG2 | THR | B | 364 | 9.962 | 47.073 | 105.001 | 1.00 | 34.16 | C |
| ATOM | 5107 | N | GLN | B | 365 | 10.877 | 48.689 | 101.561 | 1.00 | 28.22 | N |
| ATOM | 5108 | CA | GLN | B | 365 | 10.584 | 48.288 | 100.204 | 1.00 | 28.22 | C |
| ATOM | 5109 | C | GLN | B | 365 | 11.825 | 47.743 | 99.513 | 1.00 | 28.22 | C |
| ATOM | 5110 | O | GLN | B | 365 | 11.779 | 46.738 | 98.806 | 1.00 | 28.22 | O |
| ATOM | 5111 | CB | GLN | B | 365 | 10.039 | 49.478 | 99.414 | 1.00 | 40.21 | C |
| ATOM | 5112 | CG | GLN | B | 365 | 9.985 | 49.244 | 97.917 | 1.00 | 40.21 | C |
| ATOM | 5113 | CD | GLN | B | 365 | 9.154 | 48.032 | 97.541 | 1.00 | 40.21 | C |
| ATOM | 5114 | OE1 | GLN | B | 365 | 8.027 | 48.167 | 97.092 | 1.00 | 40.21 | O |
| ATOM | 5115 | NE2 | GLN | B | 365 | 9.708 | 46.845 | 97.730 | 0.00 | 40.21 | N |
| ATOM | 5116 | N | GLU | B | 366 | 12.949 | 48.407 | 99.729 | 1.00 | 36.17 | N |
| ATOM | 5117 | CA | GLU | B | 366 | 14.186 | 48.002 | 99.089 | 1.00 | 36.17 | C |
| ATOM | 5118 | C | GLU | B | 366 | 14.749 | 46.708 | 99.637 | 1.00 | 36.17 | C |
| ATOM | 5119 | O | GLU | B | 366 | 15.452 | 45.982 | 98.930 | 1.00 | 36.17 | O |
| ATOM | 5120 | CB | GLU | B | 366 | 15.200 | 49.133 | 99.227 | 1.00 | 39.37 | C |
| ATOM | 5121 | CG | GLU | B | 366 | 16.539 | 48.858 | 98.589 | 1.00 | 39.37 | C |
| ATOM | 5122 | CD | GLU | B | 366 | 17.368 | 50.110 | 98.518 | 1.00 | 39.37 | C |
| ATOM | 5123 | OE1 | GLU | B | 366 | 17.099 | 51.014 | 99.345 | 1.00 | 39.37 | O |
| ATOM | 5124 | OE2 | GLU | B | 366 | 18.271 | 50.185 | 97.654 | 1.00 | 39.37 | O |
| ATOM | 5125 | N | LEU | B | 367 | 14.430 | 46.421 | 100.897 | 1.00 | 31.88 | N |
| ATOM | 5126 | CA | LEU | B | 367 | 14.909 | 45.213 | 101.568 | 1.00 | 31.88 | C |
| ATOM | 5127 | C | LEU | B | 367 | 13.912 | 44.074 | 101.504 | 1.00 | 31.88 | C |
| ATOM | 5128 | O | LEU | B | 367 | 14.239 | 42.934 | 101.835 | 1.00 | 31.88 | O |
| ATOM | 5129 | CB | LEU | B | 367 | 15.175 | 45.509 | 103.036 | 1.00 | 41.02 | C |
| ATOM | 5130 | CG | LEU | B | 367 | 16.157 | 46.626 | 103.361 | 1.00 | 41.02 | C |
| ATOM | 5131 | CD1 | LEU | B | 367 | 15.908 | 47.131 | 104.785 | 1.00 | 41.02 | C |
| ATOM | 5132 | CD2 | LEU | B | 367 | 17.573 | 46.108 | 103.186 | 1.00 | 41.02 | C |
| ATOM | 5133 | N | SER | B | 368 | 12.694 | 44.395 | 101.087 | 1.00 | 37.83 | N |
| ATOM | 5134 | CA | SER | B | 368 | 11.615 | 43.423 | 101.018 | 1.00 | 37.83 | C |
| ATOM | 5135 | C | SER | B | 368 | 11.963 | 42.072 | 100.406 | 1.00 | 37.83 | C |
| ATOM | 5136 | O | SER | B | 368 | 11.341 | 41.069 | 100.743 | 1.00 | 37.83 | O |
| ATOM | 5137 | CB | SER | B | 368 | 10.417 | 44.031 | 100.295 | 1.00 | 55.37 | C |
| ATOM | 5138 | OG | SER | B | 368 | 10.784 | 44.520 | 99.016 | 1.00 | 55.37 | O |
| ATOM | 5139 | N | SER | B | 369 | 12.949 | 42.030 | 99.517 | 1.00 | 38.48 | N |
| ATOM | 5140 | CA | SER | B | 369 | 13.333 | 40.759 | 98.909 | 1.00 | 38.48 | C |
| ATOM | 5141 | C | SER | B | 369 | 13.920 | 39.803 | 99.934 | 1.00 | 38.48 | C |
| ATOM | 5142 | O | SER | B | 369 | 13.703 | 38.612 | 99.845 | 1.00 | 38.48 | O |

| | | | | | | | | | | | |
|---|---|---|---|---|---|---|---|---|---|---|---|
| ATOM | 5143 | CB | SER | B | 369 | 14.335 | 40.976 | 97.770 | 1.00 | 42.54 | C |
| ATOM | 5144 | OG | SER | B | 369 | 15.371 | 41.878 | 98.128 | 1.00 | 42.54 | O |
| ATOM | 5145 | N | ASN | B | 370 | 14.654 | 40.325 | 100.907 | 1.00 | 25.59 | N |
| ATOM | 5146 | CA | ASN | B | 370 | 15.260 | 39.486 | 101.934 | 1.00 | 25.59 | C |
| ATOM | 5147 | C | ASN | B | 370 | 15.723 | 40.333 | 103.126 | 1.00 | 25.59 | C |
| ATOM | 5148 | O | ASN | B | 370 | 16.901 | 40.665 | 103.260 | 1.00 | 25.59 | O |
| ATOM | 5149 | CB | ASN | B | 370 | 16.426 | 38.710 | 101.328 | 1.00 | 36.57 | C |
| ATOM | 5150 | CG | ASN | B | 370 | 17.109 | 37.794 | 102.323 | 1.00 | 36.57 | C |
| ATOM | 5151 | OD1 | ASN | B | 370 | 17.918 | 36.951 | 101.940 | 1.00 | 36.57 | O |
| ATOM | 5152 | ND2 | ASN | B | 370 | 16.802 | 37.957 | 103.600 | 1.00 | 36.57 | N |
| ATOM | 5153 | N | PRO | B | 371 | 14.781 | 40.681 | 104.018 | 1.00 | 38.05 | N |
| ATOM | 5154 | CA | PRO | B | 371 | 15.011 | 41.494 | 105.223 | 1.00 | 38.05 | C |
| ATOM | 5155 | C | PRO | B | 371 | 16.213 | 41.088 | 106.080 | 1.00 | 38.05 | C |
| ATOM | 5156 | O | PRO | B | 371 | 16.988 | 41.938 | 106.515 | 1.00 | 38.05 | O |
| ATOM | 5157 | CB | PRO | B | 371 | 13.687 | 41.370 | 105.976 | 1.00 | 25.90 | C |
| ATOM | 5158 | CG | PRO | B | 371 | 12.691 | 41.261 | 104.865 | 1.00 | 25.90 | C |
| ATOM | 5159 | CD | PRO | B | 371 | 13.362 | 40.279 | 103.926 | 1.00 | 25.90 | C |
| ATOM | 5160 | N | PRO | B | 372 | 16.373 | 39.786 | 106.350 | 1.00 | 37.48 | N |
| ATOM | 5161 | CA | PRO | B | 372 | 17.512 | 39.362 | 107.165 | 1.00 | 37.48 | C |
| ATOM | 5162 | C | PRO | B | 372 | 18.813 | 40.066 | 106.790 | 1.00 | 37.48 | C |
| ATOM | 5163 | O | PRO | B | 372 | 19.637 | 40.353 | 107.655 | 1.00 | 37.48 | O |
| ATOM | 5164 | CB | PRO | B | 372 | 17.561 | 37.861 | 106.916 | 1.00 | 28.16 | C |
| ATOM | 5165 | CG | PRO | B | 372 | 16.107 | 37.510 | 106.847 | 1.00 | 28.16 | C |
| ATOM | 5166 | CD | PRO | B | 372 | 15.508 | 38.637 | 106.015 | 1.00 | 28.16 | C |
| ATOM | 5167 | N | LEU | B | 373 | 18.983 | 40.353 | 105.502 | 1.00 | 34.53 | N |
| ATOM | 5168 | CA | LEU | B | 373 | 20.185 | 41.014 | 104.993 | 1.00 | 34.53 | C |
| ATOM | 5169 | C | LEU | B | 373 | 20.564 | 42.320 | 105.678 | 1.00 | 34.53 | C |
| ATOM | 5170 | O | LEU | B | 373 | 21.746 | 42.646 | 105.759 | 1.00 | 34.53 | O |
| ATOM | 5171 | CB | LEU | B | 373 | 20.039 | 41.262 | 103.491 | 1.00 | 37.09 | C |
| ATOM | 5172 | CG | LEU | B | 373 | 20.376 | 40.102 | 102.545 | 1.00 | 37.09 | C |
| ATOM | 5173 | CD1 | LEU | B | 373 | 19.965 | 38.797 | 103.191 | 1.00 | 37.09 | C |
| ATOM | 5174 | CD2 | LEU | B | 373 | 19.686 | 40.290 | 101.186 | 1.00 | 37.09 | C |
| ATOM | 5175 | N | ALA | B | 374 | 19.573 | 43.061 | 106.171 | 1.00 | 31.16 | N |
| ATOM | 5176 | CA | ALA | B | 374 | 19.822 | 44.346 | 106.828 | 1.00 | 31.16 | C |
| ATOM | 5177 | C | ALA | B | 374 | 21.016 | 44.270 | 107.749 | 1.00 | 31.16 | C |
| ATOM | 5178 | O | ALA | B | 374 | 21.763 | 45.235 | 107.899 | 1.00 | 31.16 | O |
| ATOM | 5179 | CB | ALA | B | 374 | 18.593 | 44.798 | 107.611 | 1.00 | 26.64 | C |
| ATOM | 5180 | N | THR | B | 375 | 21.201 | 43.105 | 108.353 | 1.00 | 37.11 | N |
| ATOM | 5181 | CA | THR | B | 375 | 22.305 | 42.889 | 109.278 | 1.00 | 37.11 | C |
| ATOM | 5182 | C | THR | B | 375 | 23.662 | 43.066 | 108.650 | 1.00 | 37.11 | C |
| ATOM | 5183 | O | THR | B | 375 | 24.616 | 43.399 | 109.339 | 1.00 | 37.11 | O |
| ATOM | 5184 | CB | THR | B | 375 | 22.228 | 41.505 | 109.896 | 1.00 | 34.79 | C |
| ATOM | 5185 | OG1 | THR | B | 375 | 21.188 | 41.505 | 110.877 | 1.00 | 34.79 | O |
| ATOM | 5186 | CG2 | THR | B | 375 | 23.552 | 41.123 | 110.549 | 1.00 | 34.79 | C |
| ATOM | 5187 | N | ILE | B | 376 | 23.756 | 42.827 | 107.348 | 1.00 | 41.34 | N |
| ATOM | 5188 | CA | ILE | B | 376 | 25.025 | 43.001 | 106.663 | 1.00 | 41.34 | C |
| ATOM | 5189 | C | ILE | B | 376 | 24.978 | 44.320 | 105.908 | 1.00 | 41.34 | C |
| ATOM | 5190 | O | ILE | B | 376 | 25.906 | 45.116 | 105.978 | 1.00 | 41.34 | O |
| ATOM | 5191 | CB | ILE | B | 376 | 25.305 | 41.868 | 105.671 | 1.00 | 31.14 | C |
| ATOM | 5192 | CG1 | ILE | B | 376 | 25.202 | 40.521 | 106.374 | 1.00 | 31.14 | C |
| ATOM | 5193 | CG2 | ILE | B | 376 | 26.698 | 42.021 | 105.113 | 1.00 | 31.14 | C |
| ATOM | 5194 | CD1 | ILE | B | 376 | 25.472 | 39.332 | 105.465 | 1.00 | 31.14 | C |
| ATOM | 5195 | N | LEU | B | 377 | 23.882 | 44.542 | 105.195 | 1.00 | 27.23 | N |
| ATOM | 5196 | CA | LEU | B | 377 | 23.687 | 45.764 | 104.430 | 1.00 | 27.23 | C |
| ATOM | 5197 | C | LEU | B | 377 | 23.908 | 47.025 | 105.268 | 1.00 | 27.23 | C |
| ATOM | 5198 | O | LEU | B | 377 | 24.431 | 48.024 | 104.774 | 1.00 | 27.23 | O |
| ATOM | 5199 | CB | LEU | B | 377 | 22.274 | 45.768 | 103.839 | 1.00 | 32.98 | C |
| ATOM | 5200 | CG | LEU | B | 377 | 22.142 | 45.157 | 102.442 | 1.00 | 32.98 | C |
| ATOM | 5201 | CD1 | LEU | B | 377 | 23.117 | 44.008 | 102.269 | 1.00 | 32.98 | C |
| ATOM | 5202 | CD2 | LEU | B | 377 | 20.699 | 44.714 | 102.206 | 1.00 | 32.98 | C |
| ATOM | 5203 | N | ILE | B | 378 | 23.512 | 46.970 | 106.534 | 1.00 | 33.35 | N |
| ATOM | 5204 | CA | ILE | B | 378 | 23.660 | 48.106 | 107.427 | 1.00 | 33.35 | C |

| | | | | | | | | | | | |
|---|---|---|---|---|---|---|---|---|---|---|---|
| ATOM | 5205 | C | ILE | B | 378 | 24.732 | 47.826 | 108.477 | 1.00 | 33.35 | C |
| ATOM | 5206 | O | ILE | B | 378 | 24.494 | 47.127 | 109.447 | 1.00 | 33.35 | O |
| ATOM | 5207 | CB | ILE | B | 378 | 22.321 | 48.427 | 108.090 | 1.00 | 39.29 | C |
| ATOM | 5208 | CG1 | ILE | B | 378 | 21.334 | 48.876 | 107.012 | 1.00 | 39.29 | C |
| ATOM | 5209 | CG2 | ILE | B | 378 | 22.497 | 49.505 | 109.130 | 1.00 | 39.29 | C |
| ATOM | 5210 | CD1 | ILE | B | 378 | 19.913 | 49.057 | 107.493 | 1.00 | 39.29 | C |
| ATOM | 5211 | N | PRO | B | 379 | 25.933 | 48.394 | 108.290 | 1.00 | 45.99 | N |
| ATOM | 5212 | CA | PRO | B | 379 | 27.063 | 48.209 | 109.199 | 1.00 | 45.99 | C |
| ATOM | 5213 | C | PRO | B | 379 | 26.803 | 48.864 | 110.545 | 1.00 | 45.99 | C |
| ATOM | 5214 | O | PRO | B | 379 | 26.045 | 49.833 | 110.636 | 1.00 | 45.99 | O |
| ATOM | 5215 | CB | PRO | B | 379 | 28.202 | 48.870 | 108.448 | 1.00 | 44.90 | C |
| ATOM | 5216 | CG | PRO | B | 379 | 27.507 | 50.080 | 107.882 | 1.00 | 44.90 | C |
| ATOM | 5217 | CD | PRO | B | 379 | 26.211 | 49.490 | 107.341 | 1.00 | 44.90 | C |
| ATOM | 5218 | N | PRO | B | 380 | 27.451 | 48.348 | 111.602 | 1.00 | 54.40 | N |
| ATOM | 5219 | CA | PRO | B | 380 | 27.346 | 48.816 | 112.987 | 1.00 | 54.40 | C |
| ATOM | 5220 | C | PRO | B | 380 | 27.403 | 50.333 | 113.166 | 1.00 | 54.40 | C |
| ATOM | 5221 | O | PRO | B | 380 | 26.720 | 50.875 | 114.035 | 1.00 | 54.40 | O |
| ATOM | 5222 | CB | PRO | B | 380 | 28.511 | 48.109 | 113.679 | 1.00 | 51.12 | C |
| ATOM | 5223 | CG | PRO | B | 380 | 28.643 | 46.836 | 112.910 | 1.00 | 51.12 | C |
| ATOM | 5224 | CD | PRO | B | 380 | 28.491 | 47.307 | 111.484 | 1.00 | 51.12 | C |
| ATOM | 5225 | N | HIS | B | 381 | 28.203 | 51.028 | 112.364 | 1.00 | 39.13 | N |
| ATOM | 5226 | CA | HIS | B | 381 | 28.278 | 52.477 | 112.525 | 1.00 | 39.13 | C |
| ATOM | 5227 | C | HIS | B | 381 | 27.184 | 53.206 | 111.738 | 1.00 | 39.13 | C |
| ATOM | 5228 | O | HIS | B | 381 | 26.837 | 54.330 | 112.069 | 1.00 | 39.13 | O |
| ATOM | 5229 | CB | HIS | B | 381 | 29.656 | 52.978 | 112.100 | 1.00 | 45.13 | C |
| ATOM | 5230 | CG | HIS | B | 381 | 29.992 | 52.648 | 110.684 | 1.00 | 45.13 | C |
| ATOM | 5231 | ND1 | HIS | B | 381 | 29.624 | 53.451 | 109.624 | 1.00 | 45.13 | N |
| ATOM | 5232 | CD2 | HIS | B | 381 | 30.605 | 51.568 | 110.143 | 1.00 | 45.13 | C |
| ATOM | 5233 | CE1 | HIS | B | 381 | 29.998 | 52.883 | 108.491 | 1.00 | 45.13 | C |
| ATOM | 5234 | NE2 | HIS | B | 381 | 30.596 | 51.738 | 108.780 | 1.00 | 45.13 | N |
| ATOM | 5235 | N | ALA | B | 382 | 26.643 | 52.572 | 110.700 | 1.00 | 53.09 | N |
| ATOM | 5236 | CA | ALA | B | 382 | 25.598 | 53.204 | 109.891 | 1.00 | 53.09 | C |
| ATOM | 5237 | C | ALA | B | 382 | 24.438 | 53.694 | 110.753 | 1.00 | 53.09 | C |
| ATOM | 5238 | O | ALA | B | 382 | 24.217 | 53.183 | 111.859 | 1.00 | 53.09 | O |
| ATOM | 5239 | CB | ALA | B | 382 | 25.085 | 52.230 | 108.851 | 0.00 | 35.69 | C |
| TER | 5240 | | ALA | B | 382 | | | | | | |
| HETATM | 5241 | P | PO4 | | 101 | 6.674 | 44.717 | 75.601 | 1.00100.00 | | P |
| HETATM | 5242 | O1 | PO4 | | 101 | 5.864 | 45.844 | 75.197 | 1.00100.00 | | O |
| HETATM | 5243 | O2 | PO4 | | 101 | 6.533 | 44.553 | 77.054 | 1.00100.00 | | O |
| HETATM | 5244 | O3 | PO4 | | 101 | 8.069 | 45.007 | 75.230 | 1.00100.00 | | O |
| HETATM | 5245 | O4 | PO4 | | 101 | 6.207 | 43.462 | 74.899 | 1.00100.00 | | O |
| HETATM | 5246 | P | PO4 | | 102 | 20.855 | 25.652 | 63.621 | 1.00 | 84.35 | P |
| HETATM | 5247 | O1 | PO4 | | 102 | 20.939 | 26.503 | 64.776 | 1.00 | 84.35 | O |
| HETATM | 5248 | O2 | PO4 | | 102 | 20.692 | 24.267 | 64.090 | 1.00 | 84.35 | O |
| HETATM | 5249 | O3 | PO4 | | 102 | 22.103 | 25.807 | 62.865 | 1.00 | 84.35 | O |
| HETATM | 5250 | O4 | PO4 | | 102 | 19.672 | 26.051 | 62.776 | 1.00 | 84.35 | O |
| HETATM | 5251 | P | PO4 | | 103 | 14.872 | 32.932 | 47.919 | 1.00 | 66.00 | P |
| HETATM | 5252 | O1 | PO4 | | 103 | 14.128 | 34.220 | 47.710 | 1.00 | 66.00 | O |
| HETATM | 5253 | O2 | PO4 | | 103 | 14.605 | 32.419 | 49.305 | 1.00 | 66.00 | O |
| HETATM | 5254 | O3 | PO4 | | 103 | 16.325 | 33.159 | 47.739 | 1.00 | 66.00 | O |
| HETATM | 5255 | O4 | PO4 | | 103 | 14.413 | 31.927 | 46.921 | 1.00 | 66.00 | O |
| HETATM | 5256 | P | PO4 | | 104 | 15.879 | 39.372 | 90.825 | 1.00 | 70.83 | P |
| HETATM | 5257 | O1 | PO4 | | 104 | 14.867 | 40.478 | 90.860 | 1.00 | 70.83 | O |
| HETATM | 5258 | O2 | PO4 | | 104 | 16.157 | 38.910 | 92.226 | 1.00 | 70.83 | O |
| HETATM | 5259 | O3 | PO4 | | 104 | 17.150 | 39.862 | 90.202 | 1.00 | 70.83 | O |
| HETATM | 5260 | O4 | PO4 | | 104 | 15.337 | 38.231 | 90.020 | 1.00 | 70.83 | O |
| HETATM | 5261 | O | HOH | | 1 | 9.191 | 37.329 | 44.636 | 1.00 | 49.67 | O |
| HETATM | 5262 | O | HOH | | 2 | 15.038 | 50.827 | 80.223 | 1.00 | 21.84 | O |
| HETATM | 5263 | O | HOH | | 3 | 19.402 | 41.863 | 66.281 | 1.00 | 28.26 | O |
| HETATM | 5264 | O | HOH | | 4 | 20.267 | 40.502 | 79.708 | 1.00 | 24.44 | O |
| HETATM | 5265 | O | HOH | | 5 | 26.076 | 47.256 | 102.637 | 1.00 | 22.06 | O |
| HETATM | 5266 | O | HOH | | 6 | 19.478 | 44.435 | 81.151 | 1.00 | 26.60 | O |

```
HETATM 5267  O   HOH     7      12.462  34.455  55.823  1.00 27.03           O
HETATM 5268  O   HOH     8      27.998  31.207  56.576  1.00 32.82           O
HETATM 5269  O   HOH     9      19.068  37.507  89.791  1.00 28.09           O
HETATM 5270  O   HOH    10      22.489  36.839  55.671  1.00 18.10           O
HETATM 5271  O   HOH    11      23.639  53.938  97.457  1.00 23.00           O
HETATM 5272  O   HOH    12      22.210  39.688  33.394  1.00 29.50           O
HETATM 5273  O   HOH    13       9.251  51.644  79.293  1.00 27.54           O
HETATM 5274  O   HOH    14      14.011  28.741  52.236  1.00 20.48           O
HETATM 5275  O   HOH    15      22.113  48.057  58.719  1.00 17.07           O
HETATM 5276  O   HOH    16      17.953  60.685  92.504  1.00 29.75           O
HETATM 5277  O   HOH    17       5.362  47.793  82.405  1.00 37.81           O
HETATM 5278  O   HOH    18      31.088  43.222  79.519  1.00 29.72           O
HETATM 5279  O   HOH    19      15.632  35.357  83.246  1.00 25.61           O
HETATM 5280  O   HOH    20      12.181  36.442  48.562  1.00 33.87           O
HETATM 5281  O   HOH    21      24.906  60.578  98.815  1.00 29.46           O
HETATM 5282  O   HOH    22      30.348  41.575  77.756  1.00 14.18           O
HETATM 5283  O   HOH    23       2.239  50.498  79.907  1.00 28.29           O
HETATM 5284  O   HOH    24      23.104  58.041  78.414  1.00 40.94           O
HETATM 5285  O   HOH    25      18.905  45.022  58.992  1.00 38.17           O
HETATM 5286  O   HOH    26      29.225  36.586  38.097  1.00 30.20           O
HETATM 5287  O   HOH    27      19.258  38.337  57.570  1.00 28.48           O
HETATM 5288  O   HOH    28      15.467  33.678  87.085  1.00 32.59           O
HETATM 5289  O   HOH    29      17.338  41.559  95.186  1.00 24.45           O
HETATM 5290  O   HOH    30      26.087  39.010  77.232  1.00 27.43           O
HETATM 5291  O   HOH    31      15.962  30.757  67.920  1.00 41.75           O
HETATM 5292  O   HOH    32      10.967  48.375  50.863  1.00 35.33           O
HETATM 5293  O   HOH    33       6.505  43.542  52.898  1.00 41.84           O
HETATM 5294  O   HOH    34      13.752  46.755  37.095  1.00 26.83           O
HETATM 5295  O   HOH    35      16.682  33.443  42.693  1.00 38.86           O
HETATM 5296  O   HOH    36      14.262  55.977  75.096  1.00 32.77           O
HETATM 5297  O   HOH    37      16.537  51.554  77.202  1.00 26.04           O
HETATM 5298  O   HOH    38       7.552  54.446  76.907  1.00 33.82           O
HETATM 5299  O   HOH    39      28.049  29.694  63.259  1.00 40.22           O
HETATM 5300  O   HOH    40      11.611  50.753  74.054  1.00 44.65           O
HETATM 5301  O   HOH    41       4.009  49.944  84.253  1.00 44.06           O
HETATM 5302  O   HOH    42       7.836  46.108  53.542  1.00 29.68           O
HETATM 5303  O   HOH    43      27.869  25.677  57.946  1.00 14.67           O
HETATM 5304  O   HOH    44      29.546  33.027  59.435  1.00 24.04           O
HETATM 5305  O   HOH    45      30.800  35.677  62.119  1.00 46.57           O
HETATM 5306  O   HOH    46      18.389  30.071  43.481  1.00 31.90           O
CONECT 5241 5242 5243 5244 5245
CONECT 5242 5241
CONECT 5243 5241
CONECT 5244 5241
CONECT 5245 5241
CONECT 5246 5247 5248 5249 5250
CONECT 5247 5246
CONECT 5248 5246
CONECT 5249 5246
CONECT 5250 5246
CONECT 5251 5252 5253 5254 5255
CONECT 5252 5251
CONECT 5253 5251
CONECT 5254 5251
CONECT 5255 5251
CONECT 5256 5257 5258 5259 5260
CONECT 5257 5256
CONECT 5258 5256
CONECT 5259 5256
CONECT 5260 5256
MASTER      486    0    4   32   25    0    0    6 5304    2   20   66
END
```

# FIG. 2

| | | Atom Type | Resid | | # | X | Y | Z | OCC | B | |
|---|---|---|---|---|---|---|---|---|---|---|---|
| ATOM | 1 | CB | VAL | A | 37 | 24.016 | -4.419 | 22.952 | 1.00 | 43.19 | C |
| ATOM | 2 | CG1 | VAL | A | 37 | 22.659 | -4.307 | 23.637 | 1.00 | 44.26 | C |
| ATOM | 3 | CG2 | VAL | A | 37 | 24.253 | -5.845 | 22.488 | 1.00 | 44.65 | C |
| ATOM | 4 | C | VAL | A | 37 | 23.549 | -2.093 | 22.205 | 1.00 | 42.31 | C |
| ATOM | 5 | O | VAL | A | 37 | 24.169 | -1.420 | 23.025 | 1.00 | 42.51 | O |
| ATOM | 6 | N | VAL | A | 37 | 25.445 | -3.418 | 21.159 | 1.00 | 41.74 | N |
| ATOM | 7 | CA | VAL | A | 37 | 24.075 | -3.462 | 21.752 | 1.00 | 43.02 | C |
| ATOM | 8 | N | THR | A | 38 | 22.397 | -1.703 | 21.660 | 1.00 | 41.95 | N |
| ATOM | 9 | CA | THR | A | 38 | 21.752 | -0.435 | 21.987 | 1.00 | 41.33 | C |
| ATOM | 10 | CB | THR | A | 38 | 21.345 | 0.338 | 20.712 | 1.00 | 41.73 | C |
| ATOM | 11 | OG1 | THR | A | 38 | 22.484 | 0.484 | 19.856 | 1.00 | 42.64 | O |
| ATOM | 12 | CG2 | THR | A | 38 | 20.803 | 1.723 | 21.065 | 1.00 | 40.94 | C |
| ATOM | 13 | C | THR | A | 38 | 20.491 | -0.697 | 22.805 | 1.00 | 41.33 | C |
| ATOM | 14 | O | THR | A | 38 | 19.782 | -1.679 | 22.567 | 1.00 | 40.90 | O |
| ATOM | 15 | N | THR | A | 39 | 20.233 | 0.177 | 23.777 | 1.00 | 41.08 | N |
| ATOM | 16 | CA | THR | A | 39 | 19.054 | 0.066 | 24.638 | 1.00 | 40.35 | C |
| ATOM | 17 | CB | THR | A | 39 | 19.431 | -0.374 | 26.073 | 1.00 | 39.40 | C |
| ATOM | 18 | OG1 | THR | A | 39 | 20.097 | -1.641 | 26.019 | 1.00 | 40.85 | O |
| ATOM | 19 | CG2 | THR | A | 39 | 18.180 | -0.508 | 26.937 | 1.00 | 38.74 | C |
| ATOM | 20 | C | THR | A | 39 | 18.352 | 1.416 | 24.702 | 1.00 | 39.57 | C |
| ATOM | 21 | O | THR | A | 39 | 18.970 | 2.435 | 25.001 | 1.00 | 39.59 | O |
| ATOM | 22 | N | VAL | A | 40 | 17.054 | 1.414 | 24.415 | 1.00 | 37.93 | N |
| ATOM | 23 | CA | VAL | A | 40 | 16.268 | 2.638 | 24.429 | 1.00 | 35.62 | C |
| ATOM | 24 | CB | VAL | A | 40 | 15.966 | 3.105 | 23.001 | 1.00 | 34.89 | C |
| ATOM | 25 | CG1 | VAL | A | 40 | 17.258 | 3.334 | 22.242 | 1.00 | 32.75 | C |
| ATOM | 26 | CG2 | VAL | A | 40 | 15.121 | 2.074 | 22.298 | 1.00 | 32.80 | C |
| ATOM | 27 | C | VAL | A | 40 | 14.947 | 2.409 | 25.137 | 1.00 | 36.24 | C |
| ATOM | 28 | O | VAL | A | 40 | 14.466 | 1.284 | 25.217 | 1.00 | 36.19 | O |
| ATOM | 29 | N | VAL | A | 41 | 14.368 | 3.478 | 25.665 | 1.00 | 37.26 | N |
| ATOM | 30 | CA | VAL | A | 41 | 13.083 | 3.386 | 26.343 | 1.00 | 38.78 | C |
| ATOM | 31 | CB | VAL | A | 41 | 13.001 | 4.400 | 27.500 | 1.00 | 38.94 | C |
| ATOM | 32 | CG1 | VAL | A | 41 | 11.586 | 4.453 | 28.059 | 1.00 | 38.55 | C |
| ATOM | 33 | CG2 | VAL | A | 41 | 13.991 | 4.010 | 28.583 | 1.00 | 37.26 | C |
| ATOM | 34 | C | VAL | A | 41 | 12.027 | 3.711 | 25.292 | 1.00 | 39.46 | C |
| ATOM | 35 | O | VAL | A | 41 | 11.784 | 4.881 | 24.981 | 1.00 | 41.11 | O |
| ATOM | 36 | N | ALA | A | 42 | 11.401 | 2.672 | 24.750 | 1.00 | 38.75 | N |
| ATOM | 37 | CA | ALA | A | 42 | 10.408 | 2.846 | 23.701 | 1.00 | 39.04 | C |
| ATOM | 38 | CB | ALA | A | 42 | 10.834 | 2.064 | 22.471 | 1.00 | 38.10 | C |
| ATOM | 39 | C | ALA | A | 42 | 8.987 | 2.466 | 24.073 | 1.00 | 38.81 | C |
| ATOM | 40 | O | ALA | A | 42 | 8.752 | 1.510 | 24.811 | 1.00 | 39.19 | O |
| ATOM | 41 | N | THR | A | 43 | 8.037 | 3.214 | 23.530 | 1.00 | 39.96 | N |
| ATOM | 42 | CA | THR | A | 43 | 6.624 | 2.968 | 23.784 | 1.00 | 40.47 | C |
| ATOM | 43 | CB | THR | A | 43 | 5.824 | 4.274 | 23.738 | 1.00 | 40.09 | C |
| ATOM | 44 | OG1 | THR | A | 43 | 6.518 | 5.294 | 24.468 | 1.00 | 41.86 | O |
| ATOM | 45 | CG2 | THR | A | 43 | 4.455 | 4.071 | 24.343 | 1.00 | 39.07 | C |
| ATOM | 46 | C | THR | A | 43 | 6.078 | 2.042 | 22.701 | 1.00 | 41.20 | C |
| ATOM | 47 | O | THR | A | 43 | 6.498 | 2.115 | 21.543 | 1.00 | 40.45 | O |
| ATOM | 48 | N | PRO | A | 44 | 5.141 | 1.152 | 23.066 | 1.00 | 43.26 | N |
| ATOM | 49 | CD | PRO | A | 44 | 4.712 | 0.788 | 24.425 | 1.00 | 43.22 | C |
| ATOM | 50 | CA | PRO | A | 44 | 4.557 | 0.230 | 22.086 | 1.00 | 45.12 | C |
| ATOM | 51 | CB | PRO | A | 44 | 3.634 | -0.639 | 22.938 | 1.00 | 45.12 | C |
| ATOM | 52 | CG | PRO | A | 44 | 4.345 | -0.673 | 24.248 | 1.00 | 44.31 | C |
| ATOM | 53 | C | PRO | A | 44 | 3.798 | 1.045 | 21.048 | 1.00 | 46.03 | C |
| ATOM | 54 | O | PRO | A | 44 | 3.005 | 1.921 | 21.402 | 1.00 | 46.81 | O |
| ATOM | 55 | N | GLY | A | 45 | 4.057 | 0.764 | 19.774 | 1.00 | 47.95 | N |
| ATOM | 56 | CA | GLY | A | 45 | 3.416 | 1.501 | 18.699 | 1.00 | 49.62 | C |
| ATOM | 57 | C | GLY | A | 45 | 1.914 | 1.395 | 18.783 | 1.00 | 50.81 | C |
| ATOM | 58 | O | GLY | A | 45 | 1.188 | 2.363 | 18.564 | 1.00 | 50.00 | O |
| ATOM | 59 | N | ALA | A | 46 | 1.449 | 0.202 | 19.122 | 1.00 | 52.89 | N |
| ATOM | 60 | CA | ALA | A | 46 | 0.023 | -0.062 | 19.241 | 1.00 | 55.18 | C |

| | | | | | | | | | | | |
|---|---|---|---|---|---|---|---|---|---|---|---|
| ATOM | 61 | CB | ALA | A | 46 | -0.430 | -0.964 | 18.097 | 1.00 | 54.45 | C |
| ATOM | 62 | C | ALA | A | 46 | -0.267 | -0.724 | 20.583 | 1.00 | 56.42 | C |
| ATOM | 63 | O | ALA | A | 46 | -0.236 | -1.947 | 20.701 | 1.00 | 56.72 | O |
| ATOM | 64 | N | GLY | A | 47 | -0.545 | 0.091 | 21.595 | 1.00 | 57.09 | N |
| ATOM | 65 | CA | GLY | A | 47 | -0.840 | -0.462 | 22.904 | 1.00 | 58.03 | C |
| ATOM | 66 | C | GLY | A | 47 | -0.903 | 0.572 | 24.011 | 1.00 | 58.65 | C |
| ATOM | 67 | O | GLY | A | 47 | -1.004 | 1.772 | 23.732 | 1.00 | 58.46 | O |
| ATOM | 68 | N | PRO | A | 48 | -0.845 | 0.132 | 25.282 | 1.00 | 58.66 | N |
| ATOM | 69 | CD | PRO | A | 48 | -0.565 | -1.244 | 25.717 | 1.00 | 58.47 | C |
| ATOM | 70 | CA | PRO | A | 48 | -0.897 | 1.035 | 26.434 | 1.00 | 58.14 | C |
| ATOM | 71 | CB | PRO | A | 48 | -0.817 | 0.093 | 27.635 | 1.00 | 58.39 | C |
| ATOM | 72 | CG | PRO | A | 48 | -1.197 | -1.258 | 27.072 | 1.00 | 58.39 | C |
| ATOM | 73 | C | PRO | A | 48 | 0.301 | 1.958 | 26.358 | 1.00 | 58.04 | C |
| ATOM | 74 | O | PRO | A | 48 | 1.448 | 1.510 | 26.297 | 1.00 | 57.19 | O |
| ATOM | 75 | N | ASP | A | 49 | 0.026 | 3.253 | 26.382 | 1.00 | 58.39 | N |
| ATOM | 76 | CA | ASP | A | 49 | 1.074 | 4.246 | 26.276 | 1.00 | 59.11 | C |
| ATOM | 77 | CB | ASP | A | 49 | 0.502 | 5.542 | 25.704 | 1.00 | 61.42 | C |
| ATOM | 78 | CG | ASP | A | 49 | 1.493 | 6.677 | 25.759 | 1.00 | 64.51 | C |
| ATOM | 79 | OD1 | ASP | A | 49 | 2.664 | 6.455 | 25.355 | 1.00 | 67.45 | O |
| ATOM | 80 | OD2 | ASP | A | 49 | 1.111 | 7.788 | 26.204 | 1.00 | 65.29 | O |
| ATOM | 81 | C | ASP | A | 49 | 1.916 | 4.570 | 27.503 | 1.00 | 59.32 | C |
| ATOM | 82 | O | ASP | A | 49 | 1.760 | 5.626 | 28.122 | 1.00 | 60.47 | O |
| ATOM | 83 | N | ARG | A | 50 | 2.806 | 3.642 | 27.840 | 1.00 | 58.19 | N |
| ATOM | 84 | CA | ARG | A | 50 | 3.785 | 3.777 | 28.925 | 1.00 | 56.50 | C |
| ATOM | 85 | CB | ARG | A | 50 | 3.172 | 3.457 | 30.301 | 1.00 | 58.21 | C |
| ATOM | 86 | CG | ARG | A | 50 | 2.470 | 4.704 | 30.911 | 1.00 | 59.04 | C |
| ATOM | 87 | CD | ARG | A | 50 | 2.757 | 4.961 | 32.411 | 1.00 | 60.18 | C |
| ATOM | 88 | NE | ARG | A | 50 | 4.183 | 5.146 | 32.751 | 1.00 | 61.04 | N |
| ATOM | 89 | CZ | ARG | A | 50 | 4.962 | 6.152 | 32.338 | 1.00 | 60.36 | C |
| ATOM | 90 | NH1 | ARG | A | 50 | 4.477 | 7.100 | 31.547 | 1.00 | 60.40 | N |
| ATOM | 91 | NH2 | ARG | A | 50 | 6.229 | 6.216 | 32.734 | 1.00 | 58.09 | N |
| ATOM | 92 | C | ARG | A | 50 | 4.904 | 2.819 | 28.491 | 1.00 | 53.95 | C |
| ATOM | 93 | O | ARG | A | 50 | 4.696 | 1.613 | 28.352 | 1.00 | 52.54 | O |
| ATOM | 94 | N | PRO | A | 51 | 6.110 | 3.369 | 28.262 | 1.00 | 51.26 | N |
| ATOM | 95 | CD | PRO | A | 51 | 6.468 | 4.710 | 28.764 | 1.00 | 50.65 | C |
| ATOM | 96 | CA | PRO | A | 51 | 7.292 | 2.638 | 27.806 | 1.00 | 49.89 | C |
| ATOM | 97 | CB | PRO | A | 51 | 8.264 | 3.756 | 27.433 | 1.00 | 50.03 | C |
| ATOM | 98 | CG | PRO | A | 51 | 7.916 | 4.871 | 28.358 | 1.00 | 50.81 | C |
| ATOM | 99 | C | PRO | A | 51 | 7.964 | 1.500 | 28.569 | 1.00 | 49.60 | C |
| ATOM | 100 | O | PRO | A | 51 | 7.664 | 1.201 | 29.726 | 1.00 | 49.72 | O |
| ATOM | 101 | N | GLN | A | 52 | 8.874 | 0.848 | 27.853 | 1.00 | 48.69 | N |
| ATOM | 102 | CA | GLN | A | 52 | 9.636 | -0.281 | 28.351 | 1.00 | 47.79 | C |
| ATOM | 103 | CB | GLN | A | 52 | 8.882 | -1.579 | 28.072 | 1.00 | 49.27 | C |
| ATOM | 104 | CG | GLN | A | 52 | 8.088 | -1.572 | 26.776 | 1.00 | 50.74 | C |
| ATOM | 105 | CD | GLN | A | 52 | 7.511 | -2.955 | 26.412 | 1.00 | 52.97 | C |
| ATOM | 106 | OE1 | GLN | A | 52 | 8.241 | -3.854 | 25.944 | 1.00 | 52.17 | O |
| ATOM | 107 | NE2 | GLN | A | 52 | 6.198 | -3.131 | 26.630 | 1.00 | 52.02 | N |
| ATOM | 108 | C | GLN | A | 52 | 10.969 | -0.301 | 27.636 | 1.00 | 47.05 | C |
| ATOM | 109 | O | GLN | A | 52 | 11.086 | 0.185 | 26.508 | 1.00 | 47.80 | O |
| ATOM | 110 | N | GLU | A | 53 | 11.975 | -0.854 | 28.295 | 1.00 | 45.97 | N |
| ATOM | 111 | CA | GLU | A | 53 | 13.293 | -0.933 | 27.703 | 1.00 | 44.69 | C |
| ATOM | 112 | CB | GLU | A | 53 | 14.332 | -1.342 | 28.752 | 1.00 | 44.89 | C |
| ATOM | 113 | CG | GLU | A | 53 | 14.540 | -0.320 | 29.866 | 1.00 | 44.27 | C |
| ATOM | 114 | CD | GLU | A | 53 | 15.637 | -0.729 | 30.831 | 1.00 | 46.36 | C |
| ATOM | 115 | OE1 | GLU | A | 53 | 16.186 | -1.842 | 30.661 | 1.00 | 47.13 | O |
| ATOM | 116 | OE2 | GLU | A | 53 | 15.957 | 0.056 | 31.751 | 1.00 | 45.62 | O |
| ATOM | 117 | C | GLU | A | 53 | 13.288 | -1.935 | 26.557 | 1.00 | 43.22 | C |
| ATOM | 118 | O | GLU | A | 53 | 12.710 | -3.022 | 26.653 | 1.00 | 41.83 | O |
| ATOM | 119 | N | VAL | A | 54 | 13.930 | -1.540 | 25.465 | 1.00 | 41.47 | N |
| ATOM | 120 | CA | VAL | A | 54 | 14.043 | -2.364 | 24.277 | 1.00 | 39.43 | C |
| ATOM | 121 | CB | VAL | A | 54 | 13.152 | -1.841 | 23.144 | 1.00 | 39.77 | C |
| ATOM | 122 | CG1 | VAL | A | 54 | 13.370 | -2.687 | 21.897 | 1.00 | 41.24 | C |
| ATOM | 123 | CG2 | VAL | A | 54 | 11.688 | -1.874 | 23.569 | 1.00 | 37.88 | C |
| ATOM | 124 | C | VAL | A | 54 | 15.482 | -2.328 | 23.805 | 1.00 | 39.63 | C |
| ATOM | 125 | O | VAL | A | 54 | 16.071 | -1.255 | 23.650 | 1.00 | 38.18 | O |
| ATOM | 126 | N | SER | A | 55 | 16.055 | -3.502 | 23.584 | 1.00 | 40.09 | N |
| ATOM | 127 | CA | SER | A | 55 | 17.433 | -3.579 | 23.114 | 1.00 | 41.88 | C |

| | | | | | | | | | | | |
|---|---|---|---|---|---|---|---|---|---|---|---|
| ATOM | 128 | CB | SER | A | 55 | 18.289 | -4.376 | 24.103 | 1.00 | 43.92 | C |
| ATOM | 129 | OG | SER | A | 55 | 18.633 | -3.572 | 25.228 | 1.00 | 45.09 | O |
| ATOM | 130 | C | SER | A | 55 | 17.521 | -4.200 | 21.728 | 1.00 | 42.20 | C |
| ATOM | 131 | O | SER | A | 55 | 16.822 | -5.167 | 21.418 | 1.00 | 42.29 | O |
| ATOM | 132 | N | TYR | A | 56 | 18.373 | -3.621 | 20.891 | 1.00 | 42.22 | N |
| ATOM | 133 | CA | TYR | A | 56 | 18.570 | -4.103 | 19.529 | 1.00 | 42.61 | C |
| ATOM | 134 | CB | TYR | A | 56 | 17.702 | -3.311 | 18.542 | 1.00 | 38.89 | C |
| ATOM | 135 | CG | TYR | A | 56 | 17.952 | -1.814 | 18.479 | 1.00 | 37.57 | C |
| ATOM | 136 | CD1 | TYR | A | 56 | 19.053 | -1.289 | 17.803 | 1.00 | 35.43 | C |
| ATOM | 137 | CE1 | TYR | A | 56 | 19.250 | 0.094 | 17.709 | 1.00 | 33.77 | C |
| ATOM | 138 | CD2 | TYR | A | 56 | 17.057 | -0.921 | 19.063 | 1.00 | 36.63 | C |
| ATOM | 139 | CE2 | TYR | A | 56 | 17.246 | 0.455 | 18.975 | 1.00 | 35.41 | C |
| ATOM | 140 | CZ | TYR | A | 56 | 18.340 | 0.957 | 18.298 | 1.00 | 34.92 | C |
| ATOM | 141 | OH | TYR | A | 56 | 18.513 | 2.323 | 18.218 | 1.00 | 35.27 | O |
| ATOM | 142 | C | TYR | A | 56 | 20.043 | -4.017 | 19.130 | 1.00 | 44.84 | C |
| ATOM | 143 | O | TYR | A | 56 | 20.828 | -3.290 | 19.743 | 1.00 | 43.64 | O |
| ATOM | 144 | N | THR | A | 57 | 20.414 | -4.766 | 18.094 | 1.00 | 46.94 | N |
| ATOM | 145 | CA | THR | A | 57 | 21.791 | -4.777 | 17.645 | 1.00 | 48.44 | C |
| ATOM | 146 | CB | THR | A | 57 | 22.598 | -5.818 | 18.472 | 1.00 | 48.71 | C |
| ATOM | 147 | OG1 | THR | A | 57 | 24.003 | -5.549 | 18.353 | 1.00 | 49.45 | O |
| ATOM | 148 | CG2 | THR | A | 57 | 22.299 | -7.230 | 17.997 | 1.00 | 47.90 | C |
| ATOM | 149 | C | THR | A | 57 | 21.912 | -5.047 | 16.139 | 1.00 | 49.15 | C |
| ATOM | 150 | O | THR | A | 57 | 20.913 | -5.276 | 15.459 | 1.00 | 48.21 | O |
| ATOM | 151 | N | ASP | A | 58 | 23.143 | -5.006 | 15.630 | 1.00 | 50.30 | N |
| ATOM | 152 | CA | ASP | A | 58 | 23.428 | -5.216 | 14.206 | 1.00 | 50.52 | C |
| ATOM | 153 | CB | ASP | A | 58 | 22.885 | -6.556 | 13.702 | 1.00 | 51.58 | C |
| ATOM | 154 | CG | ASP | A | 58 | 23.402 | -7.724 | 14.486 | 1.00 | 52.74 | C |
| ATOM | 155 | OD1 | ASP | A | 58 | 24.606 | -7.713 | 14.831 | 1.00 | 53.02 | O |
| ATOM | 156 | OD2 | ASP | A | 58 | 22.601 | -8.654 | 14.739 | 1.00 | 53.89 | O |
| ATOM | 157 | C | ASP | A | 58 | 22.777 | -4.126 | 13.380 | 1.00 | 49.86 | C |
| ATOM | 158 | O | ASP | A | 58 | 22.185 | -4.405 | 12.335 | 1.00 | 50.15 | O |
| ATOM | 159 | N | THR | A | 59 | 22.895 | -2.886 | 13.828 | 1.00 | 49.03 | N |
| ATOM | 160 | CA | THR | A | 59 | 22.268 | -1.795 | 13.102 | 1.00 | 49.25 | C |
| ATOM | 161 | CB | THR | A | 59 | 21.988 | -0.596 | 14.032 | 1.00 | 48.94 | C |
| ATOM | 162 | OG1 | THR | A | 59 | 23.218 | 0.065 | 14.342 | 1.00 | 51.68 | O |
| ATOM | 163 | CG2 | THR | A | 59 | 21.338 | -1.067 | 15.314 | 1.00 | 46.70 | C |
| ATOM | 164 | C | THR | A | 59 | 23.078 | -1.319 | 11.898 | 1.00 | 49.58 | C |
| ATOM | 165 | O | THR | A | 59 | 24.299 | -1.171 | 11.973 | 1.00 | 49.07 | O |
| ATOM | 166 | N | LYS | A | 60 | 22.377 | -1.112 | 10.782 | 1.00 | 50.21 | N |
| ATOM | 167 | CA | LYS | A | 60 | 22.981 | -0.636 | 9.532 | 1.00 | 50.79 | C |
| ATOM | 168 | CB | LYS | A | 60 | 23.523 | -1.803 | 8.680 | 1.00 | 50.80 | C |
| ATOM | 169 | CG | LYS | A | 60 | 22.543 | -2.921 | 8.350 | 1.00 | 51.38 | C |
| ATOM | 170 | CD | LYS | A | 60 | 23.121 | -3.822 | 7.269 | 1.00 | 52.69 | C |
| ATOM | 171 | CE | LYS | A | 60 | 22.214 | -4.999 | 6.932 | 1.00 | 53.62 | C |
| ATOM | 172 | NZ | LYS | A | 60 | 22.596 | -5.626 | 5.624 | 1.00 | 54.42 | N |
| ATOM | 173 | C | LYS | A | 60 | 21.991 | 0.179 | 8.700 | 1.00 | 50.41 | C |
| ATOM | 174 | O | LYS | A | 60 | 20.780 | -0.069 | 8.719 | 1.00 | 50.71 | O |
| ATOM | 175 | N | VAL | A | 61 | 22.526 | 1.162 | 7.978 | 1.00 | 50.13 | N |
| ATOM | 176 | CA | VAL | A | 61 | 21.718 | 2.036 | 7.133 | 1.00 | 48.87 | C |
| ATOM | 177 | CB | VAL | A | 61 | 22.541 | 3.249 | 6.618 | 1.00 | 48.82 | C |
| ATOM | 178 | CG1 | VAL | A | 61 | 21.660 | 4.179 | 5.793 | 1.00 | 49.02 | C |
| ATOM | 179 | CG2 | VAL | A | 61 | 23.146 | 3.993 | 7.785 | 1.00 | 46.99 | C |
| ATOM | 180 | C | VAL | A | 61 | 21.207 | 1.230 | 5.944 | 1.00 | 48.28 | C |
| ATOM | 181 | O | VAL | A | 61 | 21.927 | 0.394 | 5.394 | 1.00 | 48.15 | O |
| ATOM | 182 | N | ILE | A | 62 | 19.955 | 1.480 | 5.566 | 1.00 | 46.53 | N |
| ATOM | 183 | CA | ILE | A | 62 | 19.320 | 0.777 | 4.458 | 1.00 | 44.95 | C |
| ATOM | 184 | CB | ILE | A | 62 | 18.562 | -0.476 | 4.935 | 1.00 | 42.88 | C |
| ATOM | 185 | CG2 | ILE | A | 62 | 19.475 | -1.356 | 5.772 | 1.00 | 40.60 | C |
| ATOM | 186 | CG1 | ILE | A | 62 | 17.321 | -0.053 | 5.735 | 1.00 | 41.66 | C |
| ATOM | 187 | CD1 | ILE | A | 62 | 16.373 | -1.193 | 6.074 | 1.00 | 40.59 | C |
| ATOM | 188 | C | ILE | A | 62 | 18.285 | 1.663 | 3.783 | 1.00 | 46.21 | C |
| ATOM | 189 | O | ILE | A | 62 | 17.472 | 1.173 | 2.998 | 1.00 | 47.15 | O |
| ATOM | 190 | N | GLY | A | 63 | 18.295 | 2.957 | 4.091 | 1.00 | 46.66 | N |
| ATOM | 191 | CA | GLY | A | 63 | 17.310 | 3.839 | 3.492 | 1.00 | 47.12 | C |
| ATOM | 192 | C | GLY | A | 63 | 17.495 | 5.316 | 3.774 | 1.00 | 48.09 | C |
| ATOM | 193 | O | GLY | A | 63 | 17.704 | 5.713 | 4.917 | 1.00 | 48.32 | O |
| ATOM | 194 | N | ASN | A | 64 | 17.420 | 6.114 | 2.711 | 1.00 | 49.81 | N |

| | | | | | | | | | | | |
|---|---|---|---|---|---|---|---|---|---|---|---|
| ATOM | 195 | CA | ASN | A | 64 | 17.558 | 7.563 | 2.767 | 1.00 | 49.75 | C |
| ATOM | 196 | CB | ASN | A | 64 | 18.393 | 8.064 | 1.590 | 1.00 | 51.28 | C |
| ATOM | 197 | CG | ASN | A | 64 | 19.795 | 8.459 | 1.997 | 1.00 | 55.47 | C |
| ATOM | 198 | OD1 | ASN | A | 64 | 19.992 | 9.173 | 2.986 | 1.00 | 57.55 | O |
| ATOM | 199 | ND2 | ASN | A | 64 | 20.780 | 8.022 | 1.226 | 1.00 | 57.75 | N |
| ATOM | 200 | C | ASN | A | 64 | 16.169 | 8.187 | 2.660 | 1.00 | 48.93 | C |
| ATOM | 201 | O | ASN | A | 64 | 15.168 | 7.486 | 2.493 | 1.00 | 48.93 | O |
| ATOM | 202 | N | GLY | A | 65 | 16.112 | 9.511 | 2.740 | 1.00 | 46.50 | N |
| ATOM | 203 | CA | GLY | A | 65 | 14.838 | 10.188 | 2.637 | 1.00 | 45.21 | C |
| ATOM | 204 | C | GLY | A | 65 | 14.956 | 11.638 | 3.033 | 1.00 | 44.89 | C |
| ATOM | 205 | O | GLY | A | 65 | 15.966 | 12.045 | 3.593 | 1.00 | 45.46 | O |
| ATOM | 206 | N | SER | A | 66 | 13.935 | 12.426 | 2.740 | 1.00 | 44.47 | N |
| ATOM | 207 | CA | SER | A | 66 | 13.965 | 13.834 | 3.096 | 1.00 | 44.39 | C |
| ATOM | 208 | CB | SER | A | 66 | 12.835 | 14.569 | 2.371 | 1.00 | 45.45 | C |
| ATOM | 209 | OG | SER | A | 66 | 11.570 | 13.979 | 2.656 | 1.00 | 46.75 | O |
| ATOM | 210 | C | SER | A | 66 | 13.798 | 13.963 | 4.607 | 1.00 | 43.29 | C |
| ATOM | 211 | O | SER | A | 66 | 14.246 | 14.931 | 5.212 | 1.00 | 42.73 | O |
| ATOM | 212 | N | PHE | A | 67 | 13.151 | 12.959 | 5.191 | 1.00 | 42.55 | N |
| ATOM | 213 | CA | PHE | A | 67 | 12.857 | 12.877 | 6.621 | 1.00 | 41.63 | C |
| ATOM | 214 | CB | PHE | A | 67 | 11.764 | 11.841 | 6.830 | 1.00 | 40.91 | C |
| ATOM | 215 | CG | PHE | A | 67 | 12.159 | 10.471 | 6.351 | 1.00 | 41.06 | C |
| ATOM | 216 | CD1 | PHE | A | 67 | 12.966 | 9.649 | 7.130 | 1.00 | 39.66 | C |
| ATOM | 217 | CD2 | PHE | A | 67 | 11.783 | 10.028 | 5.085 | 1.00 | 40.62 | C |
| ATOM | 218 | CE1 | PHE | A | 67 | 13.398 | 8.412 | 6.655 | 1.00 | 40.51 | C |
| ATOM | 219 | CE2 | PHE | A | 67 | 12.212 | 8.791 | 4.605 | 1.00 | 40.37 | C |
| ATOM | 220 | CZ | PHE | A | 67 | 13.019 | 7.983 | 5.390 | 1.00 | 39.43 | C |
| ATOM | 221 | C | PHE | A | 67 | 14.062 | 12.471 | 7.472 | 1.00 | 42.06 | C |
| ATOM | 222 | O | PHE | A | 67 | 14.130 | 12.791 | 8.662 | 1.00 | 42.38 | O |
| ATOM | 223 | N | GLY | A | 68 | 14.990 | 11.730 | 6.870 | 1.00 | 41.39 | N |
| ATOM | 224 | CA | GLY | A | 68 | 16.159 | 11.282 | 7.606 | 1.00 | 39.65 | C |
| ATOM | 225 | C | GLY | A | 68 | 16.808 | 10.000 | 7.118 | 1.00 | 38.74 | C |
| ATOM | 226 | O | GLY | A | 68 | 17.240 | 9.920 | 5.966 | 1.00 | 39.48 | O |
| ATOM | 227 | N | VAL | A | 69 | 16.867 | 8.995 | 7.994 | 1.00 | 37.02 | N |
| ATOM | 228 | CA | VAL | A | 69 | 17.506 | 7.717 | 7.685 | 1.00 | 35.17 | C |
| ATOM | 229 | CB | VAL | A | 69 | 18.922 | 7.659 | 8.331 | 1.00 | 35.03 | C |
| ATOM | 230 | CG1 | VAL | A | 69 | 19.599 | 6.343 | 8.013 | 1.00 | 33.33 | C |
| ATOM | 231 | CG2 | VAL | A | 69 | 19.770 | 8.830 | 7.845 | 1.00 | 36.13 | C |
| ATOM | 232 | C | VAL | A | 69 | 16.718 | 6.501 | 8.171 | 1.00 | 35.82 | C |
| ATOM | 233 | O | VAL | A | 69 | 15.963 | 6.579 | 9.139 | 1.00 | 36.04 | O |
| ATOM | 234 | N | VAL | A | 70 | 16.896 | 5.376 | 7.490 | 1.00 | 35.99 | N |
| ATOM | 235 | CA | VAL | A | 70 | 16.239 | 4.141 | 7.880 | 1.00 | 35.59 | C |
| ATOM | 236 | CB | VAL | A | 70 | 15.321 | 3.591 | 6.771 | 1.00 | 33.87 | C |
| ATOM | 237 | CG1 | VAL | A | 70 | 14.659 | 2.292 | 7.235 | 1.00 | 31.70 | C |
| ATOM | 238 | CG2 | VAL | A | 70 | 14.270 | 4.620 | 6.412 | 1.00 | 32.14 | C |
| ATOM | 239 | C | VAL | A | 70 | 17.329 | 3.109 | 8.157 | 1.00 | 37.71 | C |
| ATOM | 240 | O | VAL | A | 70 | 18.235 | 2.897 | 7.342 | 1.00 | 37.21 | O |
| ATOM | 241 | N | TYR | A | 71 | 17.242 | 2.467 | 9.316 | 1.00 | 39.12 | N |
| ATOM | 242 | CA | TYR | A | 71 | 18.218 | 1.458 | 9.694 | 1.00 | 38.71 | C |
| ATOM | 243 | CB | TYR | A | 71 | 18.818 | 1.752 | 11.072 | 1.00 | 39.21 | C |
| ATOM | 244 | CG | TYR | A | 71 | 19.557 | 3.054 | 11.211 | 1.00 | 39.73 | C |
| ATOM | 245 | CD1 | TYR | A | 71 | 18.874 | 4.255 | 11.349 | 1.00 | 39.93 | C |
| ATOM | 246 | CE1 | TYR | A | 71 | 19.565 | 5.454 | 11.497 | 1.00 | 41.91 | C |
| ATOM | 247 | CD2 | TYR | A | 71 | 20.954 | 3.081 | 11.221 | 1.00 | 40.60 | C |
| ATOM | 248 | CE2 | TYR | A | 71 | 21.655 | 4.273 | 11.366 | 1.00 | 40.20 | C |
| ATOM | 249 | CZ | TYR | A | 71 | 20.957 | 5.453 | 11.502 | 1.00 | 41.06 | C |
| ATOM | 250 | OH | TYR | A | 71 | 21.647 | 6.638 | 11.625 | 1.00 | 42.45 | O |
| ATOM | 251 | C | TYR | A | 71 | 17.561 | 0.108 | 9.789 | 1.00 | 38.72 | C |
| ATOM | 252 | O | TYR | A | 71 | 16.335 | -0.014 | 9.829 | 1.00 | 37.34 | O |
| ATOM | 253 | N | GLN | A | 72 | 18.399 | -0.914 | 9.816 | 1.00 | 38.30 | N |
| ATOM | 254 | CA | GLN | A | 72 | 17.913 | -2.260 | 9.997 | 1.00 | 39.34 | C |
| ATOM | 255 | CB | GLN | A | 72 | 18.406 | -3.203 | 8.916 | 1.00 | 40.14 | C |
| ATOM | 256 | CG | GLN | A | 72 | 18.005 | -4.631 | 9.210 | 1.00 | 41.78 | C |
| ATOM | 257 | CD | GLN | A | 72 | 18.656 | -5.625 | 8.276 | 1.00 | 43.42 | C |
| ATOM | 258 | OE1 | GLN | A | 72 | 19.081 | -6.700 | 8.699 | 1.00 | 44.23 | O |
| ATOM | 259 | NE2 | GLN | A | 72 | 18.738 | -5.274 | 6.995 | 1.00 | 43.61 | N |
| ATOM | 260 | C | GLN | A | 72 | 18.536 | -2.650 | 11.318 | 1.00 | 38.98 | C |
| ATOM | 261 | O | GLN | A | 72 | 19.657 | -2.251 | 11.623 | 1.00 | 39.05 | O |

| | | | | | | | | | | | |
|---|---|---|---|---|---|---|---|---|---|---|---|
| ATOM | 262 | N | ALA | A | 73 | 17.809 | -3.414 | 12.111 | 1.00 | 39.51 | N |
| ATOM | 263 | CA | ALA | A | 73 | 18.325 | -3.826 | 13.395 | 1.00 | 40.86 | C |
| ATOM | 264 | CB | ALA | A | 73 | 18.041 | -2.757 | 14.442 | 1.00 | 40.82 | C |
| ATOM | 265 | C | ALA | A | 73 | 17.694 | -5.135 | 13.791 | 1.00 | 41.65 | C |
| ATOM | 266 | O | ALA | A | 73 | 16.735 | -5.594 | 13.167 | 1.00 | 41.06 | O |
| ATOM | 267 | N | LYS | A | 74 | 18.251 | -5.718 | 14.843 | 1.00 | 43.89 | N |
| ATOM | 268 | CA | LYS | A | 74 | 17.820 | -7.003 | 15.373 | 1.00 | 45.18 | C |
| ATOM | 269 | CB | LYS | A | 74 | 18.937 | -8.015 | 15.113 | 1.00 | 46.06 | C |
| ATOM | 270 | CG | LYS | A | 74 | 18.795 | -9.401 | 15.700 | 1.00 | 47.06 | C |
| ATOM | 271 | CD | LYS | A | 74 | 19.924 | -10.219 | 15.106 | 1.00 | 48.93 | C |
| ATOM | 272 | CE | LYS | A | 74 | 19.934 | -11.663 | 15.547 | 1.00 | 50.02 | C |
| ATOM | 273 | NZ | LYS | A | 74 | 20.929 | -12.402 | 14.698 | 1.00 | 50.02 | N |
| ATOM | 274 | C | LYS | A | 74 | 17.524 | -6.885 | 16.866 | 1.00 | 45.62 | C |
| ATOM | 275 | O | LYS | A | 74 | 18.411 | -6.594 | 17.666 | 1.00 | 44.56 | O |
| ATOM | 276 | N | LEU | A | 75 | 16.259 | -7.109 | 17.217 | 1.00 | 46.26 | N |
| ATOM | 277 | CA | LEU | A | 75 | 15.798 | -7.050 | 18.592 | 1.00 | 45.73 | C |
| ATOM | 278 | CB | LEU | A | 75 | 14.268 | -7.134 | 18.630 | 1.00 | 44.80 | C |
| ATOM | 279 | CG | LEU | A | 75 | 13.459 | -6.088 | 17.852 | 1.00 | 43.46 | C |
| ATOM | 280 | CD1 | LEU | A | 75 | 11.971 | -6.367 | 18.012 | 1.00 | 40.85 | C |
| ATOM | 281 | CD2 | LEU | A | 75 | 13.792 | -4.697 | 18.365 | 1.00 | 43.72 | C |
| ATOM | 282 | C | LEU | A | 75 | 16.402 | -8.206 | 19.384 | 1.00 | 46.73 | C |
| ATOM | 283 | O | LEU | A | 75 | 16.172 | -9.378 | 19.062 | 1.00 | 46.50 | O |
| ATOM | 284 | N | CYS | A | 76 | 17.180 | -7.865 | 20.413 | 1.00 | 48.60 | N |
| ATOM | 285 | CA | CYS | A | 76 | 17.839 | -8.850 | 21.282 | 1.00 | 49.46 | C |
| ATOM | 286 | CB | CYS | A | 76 | 18.601 | -8.137 | 22.406 | 1.00 | 48.19 | C |
| ATOM | 287 | SG | CYS | A | 76 | 20.047 | -7.191 | 21.891 | 1.00 | 47.35 | S |
| ATOM | 288 | C | CYS | A | 76 | 16.826 | -9.798 | 21.909 | 1.00 | 50.36 | C |
| ATOM | 289 | O | CYS | A | 76 | 17.177 | -10.865 | 22.403 | 1.00 | 50.28 | O |
| ATOM | 290 | N | ASP | A | 77 | 15.568 | -9.390 | 21.887 | 1.00 | 52.38 | N |
| ATOM | 291 | CA | ASP | A | 77 | 14.493 | -10.160 | 22.475 | 1.00 | 54.20 | C |
| ATOM | 292 | CB | ASP | A | 77 | 13.256 | -9.265 | 22.643 | 1.00 | 56.30 | C |
| ATOM | 293 | CG | ASP | A | 77 | 13.514 | -7.787 | 22.257 | 1.00 | 59.05 | C |
| ATOM | 294 | OD1 | ASP | A | 77 | 12.515 | -7.018 | 22.177 | 1.00 | 59.16 | O |
| ATOM | 295 | OD2 | ASP | A | 77 | 14.693 | -7.378 | 22.047 | 1.00 | 59.58 | O |
| ATOM | 296 | C | ASP | A | 77 | 14.123 | -11.418 | 21.680 | 1.00 | 54.77 | C |
| ATOM | 297 | O | ASP | A | 77 | 14.385 | -12.539 | 22.120 | 1.00 | 55.18 | O |
| ATOM | 298 | N | SER | A | 78 | 13.519 | -11.222 | 20.509 | 1.00 | 54.50 | N |
| ATOM | 299 | CA | SER | A | 78 | 13.073 | -12.322 | 19.650 | 1.00 | 53.92 | C |
| ATOM | 300 | CB | SER | A | 78 | 11.707 | -11.981 | 19.041 | 1.00 | 54.31 | C |
| ATOM | 301 | OG | SER | A | 78 | 11.733 | -10.685 | 18.452 | 1.00 | 53.66 | O |
| ATOM | 302 | C | SER | A | 78 | 14.044 | -12.629 | 18.518 | 1.00 | 53.98 | C |
| ATOM | 303 | O | SER | A | 78 | 13.815 | -13.541 | 17.717 | 1.00 | 53.75 | O |
| ATOM | 304 | N | GLY | A | 79 | 15.134 | -11.873 | 18.453 | 1.00 | 53.79 | N |
| ATOM | 305 | CA | GLY | A | 79 | 16.090 | -12.086 | 17.381 | 1.00 | 52.28 | C |
| ATOM | 306 | C | GLY | A | 79 | 15.516 | -11.458 | 16.122 | 1.00 | 51.80 | C |
| ATOM | 307 | O | GLY | A | 79 | 16.258 | -11.110 | 15.200 | 1.00 | 51.68 | O |
| ATOM | 308 | N | GLU | A | 80 | 14.193 | -11.294 | 16.102 | 1.00 | 51.28 | N |
| ATOM | 309 | CA | GLU | A | 80 | 13.473 | -10.711 | 14.962 | 1.00 | 51.47 | C |
| ATOM | 310 | CB | GLU | A | 80 | 12.036 | -10.338 | 15.348 | 1.00 | 53.87 | C |
| ATOM | 311 | CG | GLU | A | 80 | 11.110 | -11.496 | 15.692 | 1.00 | 57.17 | C |
| ATOM | 312 | CD | GLU | A | 80 | 9.736 | -11.017 | 16.134 | 1.00 | 60.23 | C |
| ATOM | 313 | OE1 | GLU | A | 80 | 9.645 | -10.324 | 17.180 | 1.00 | 61.81 | O |
| ATOM | 314 | OE2 | GLU | A | 80 | 8.739 | -11.329 | 15.435 | 1.00 | 62.38 | O |
| ATOM | 315 | C | GLU | A | 80 | 14.132 | -9.470 | 14.392 | 1.00 | 49.48 | C |
| ATOM | 316 | O | GLU | A | 80 | 14.758 | -8.691 | 15.113 | 1.00 | 49.02 | O |
| ATOM | 317 | N | LEU | A | 81 | 13.977 | -9.295 | 13.087 | 1.00 | 47.91 | N |
| ATOM | 318 | CA | LEU | A | 81 | 14.539 | -8.140 | 12.417 | 1.00 | 46.40 | C |
| ATOM | 319 | CB | LEU | A | 81 | 15.008 | -8.503 | 11.005 | 1.00 | 47.51 | C |
| ATOM | 320 | CG | LEU | A | 81 | 16.424 | -9.074 | 10.869 | 1.00 | 48.03 | C |
| ATOM | 321 | CD1 | LEU | A | 81 | 16.833 | -9.084 | 9.400 | 1.00 | 47.42 | C |
| ATOM | 322 | CD2 | LEU | A | 81 | 17.396 | -8.195 | 11.668 | 1.00 | 48.37 | C |
| ATOM | 323 | C | LEU | A | 81 | 13.503 | -7.038 | 12.344 | 1.00 | 44.26 | C |
| ATOM | 324 | O | LEU | A | 81 | 12.301 | -7.293 | 12.258 | 1.00 | 43.78 | O |
| ATOM | 325 | N | VAL | A | 82 | 13.976 | -5.803 | 12.387 | 1.00 | 41.44 | N |
| ATOM | 326 | CA | VAL | A | 82 | 13.077 | -4.675 | 12.329 | 1.00 | 39.19 | C |
| ATOM | 327 | CB | VAL | A | 82 | 12.676 | -4.184 | 13.736 | 1.00 | 39.01 | C |
| ATOM | 328 | CG1 | VAL | A | 82 | 12.039 | -5.310 | 14.519 | 1.00 | 37.51 | C |

| | | | | | | | | | | | |
|---|---|---|---|---|---|---|---|---|---|---|---|
| ATOM | 329 | CG2 | VAL | A | 82 | 13.892 | -3.621 | 14.453 | 1.00 | 37.99 | C |
| ATOM | 330 | C | VAL | A | 82 | 13.743 | -3.525 | 11.616 | 1.00 | 38.15 | C |
| ATOM | 331 | O | VAL | A | 82 | 14.961 | -3.489 | 11.468 | 1.00 | 38.17 | O |
| ATOM | 332 | N | ALA | A | 83 | 12.922 | -2.587 | 11.169 | 1.00 | 36.63 | N |
| ATOM | 333 | CA | ALA | A | 83 | 13.414 | -1.408 | 10.496 | 1.00 | 36.15 | C |
| ATOM | 334 | CB | ALA | A | 83 | 12.620 | -1.161 | 9.224 | 1.00 | 35.39 | C |
| ATOM | 335 | C | ALA | A | 83 | 13.218 | -0.261 | 11.477 | 1.00 | 35.25 | C |
| ATOM | 336 | O | ALA | A | 83 | 12.294 | -0.274 | 12.296 | 1.00 | 33.66 | O |
| ATOM | 337 | N | ILE | A | 84 | 14.104 | 0.719 | 11.416 | 1.00 | 35.22 | N |
| ATOM | 338 | CA | ILE | A | 84 | 13.988 | 1.862 | 12.294 | 1.00 | 36.94 | C |
| ATOM | 339 | CB | ILE | A | 84 | 15.117 | 1.879 | 13.356 | 1.00 | 37.47 | C |
| ATOM | 340 | CG2 | ILE | A | 84 | 14.991 | 3.112 | 14.236 | 1.00 | 36.46 | C |
| ATOM | 341 | CG1 | ILE | A | 84 | 15.027 | 0.625 | 14.224 | 1.00 | 38.48 | C |
| ATOM | 342 | CD1 | ILE | A | 84 | 16.003 | 0.608 | 15.372 | 1.00 | 40.91 | C |
| ATOM | 343 | C | ILE | A | 84 | 14.050 | 3.132 | 11.464 | 1.00 | 37.44 | C |
| ATOM | 344 | O | ILE | A | 84 | 15.109 | 3.505 | 10.949 | 1.00 | 37.84 | O |
| ATOM | 345 | N | LYS | A | 85 | 12.910 | 3.790 | 11.321 | 1.00 | 38.11 | N |
| ATOM | 346 | CA | LYS | A | 85 | 12.862 | 5.014 | 10.547 | 1.00 | 39.27 | C |
| ATOM | 347 | CB | LYS | A | 85 | 11.475 | 5.189 | 9.922 | 1.00 | 39.58 | C |
| ATOM | 348 | CG | LYS | A | 85 | 11.380 | 6.282 | 8.863 | 1.00 | 37.71 | C |
| ATOM | 349 | CD | LYS | A | 85 | 9.962 | 6.310 | 8.309 | 1.00 | 39.18 | C |
| ATOM | 350 | CE | LYS | A | 85 | 9.762 | 7.333 | 7.207 | 1.00 | 38.99 | C |
| ATOM | 351 | NZ | LYS | A | 85 | 8.348 | 7.312 | 6.736 | 1.00 | 40.98 | N |
| ATOM | 352 | C | LYS | A | 85 | 13.178 | 6.166 | 11.486 | 1.00 | 40.01 | C |
| ATOM | 353 | O | LYS | A | 85 | 12.410 | 6.463 | 12.404 | 1.00 | 40.49 | O |
| ATOM | 354 | N | LYS | A | 86 | 14.336 | 6.780 | 11.269 | 1.00 | 41.82 | N |
| ATOM | 355 | CA | LYS | A | 86 | 14.787 | 7.908 | 12.077 | 1.00 | 43.19 | C |
| ATOM | 356 | CB | LYS | A | 86 | 16.313 | 7.906 | 12.170 | 1.00 | 45.09 | C |
| ATOM | 357 | CG | LYS | A | 86 | 16.903 | 8.896 | 13.150 | 1.00 | 46.50 | C |
| ATOM | 358 | CD | LYS | A | 86 | 18.425 | 8.803 | 13.137 | 1.00 | 49.98 | C |
| ATOM | 359 | CE | LYS | A | 86 | 19.041 | 9.701 | 14.202 | 1.00 | 52.69 | C |
| ATOM | 360 | NZ | LYS | A | 86 | 20.520 | 9.553 | 14.302 | 1.00 | 55.15 | N |
| ATOM | 361 | C | LYS | A | 86 | 14.299 | 9.173 | 11.382 | 1.00 | 43.64 | C |
| ATOM | 362 | O | LYS | A | 86 | 14.594 | 9.399 | 10.208 | 1.00 | 42.94 | O |
| ATOM | 363 | N | VAL | A | 87 | 13.547 | 9.987 | 12.115 | 1.00 | 44.58 | N |
| ATOM | 364 | CA | VAL | A | 87 | 12.978 | 11.210 | 11.577 | 1.00 | 45.62 | C |
| ATOM | 365 | CB | VAL | A | 87 | 11.455 | 11.086 | 11.435 | 1.00 | 44.58 | C |
| ATOM | 366 | CG1 | VAL | A | 87 | 10.831 | 12.473 | 11.333 | 1.00 | 44.53 | C |
| ATOM | 367 | CG2 | VAL | A | 87 | 11.112 | 10.258 | 10.209 | 1.00 | 42.75 | C |
| ATOM | 368 | C | VAL | A | 87 | 13.236 | 12.427 | 12.426 | 1.00 | 47.67 | C |
| ATOM | 369 | O | VAL | A | 87 | 13.141 | 12.371 | 13.641 | 1.00 | 48.67 | O |
| ATOM | 370 | N | LEU | A | 88 | 13.536 | 13.539 | 11.781 | 1.00 | 51.08 | N |
| ATOM | 371 | CA | LEU | A | 88 | 13.763 | 14.764 | 12.511 | 1.00 | 54.83 | C |
| ATOM | 372 | CB | LEU | A | 88 | 14.631 | 15.716 | 11.691 | 1.00 | 55.85 | C |
| ATOM | 373 | CG | LEU | A | 88 | 15.248 | 16.843 | 12.519 | 1.00 | 57.59 | C |
| ATOM | 374 | CD1 | LEU | A | 88 | 16.176 | 16.223 | 13.567 | 1.00 | 57.59 | C |
| ATOM | 375 | CD2 | LEU | A | 88 | 16.032 | 17.807 | 11.627 | 1.00 | 57.03 | C |
| ATOM | 376 | C | LEU | A | 88 | 12.409 | 15.408 | 12.767 | 1.00 | 57.25 | C |
| ATOM | 377 | O | LEU | A | 88 | 11.727 | 15.820 | 11.835 | 1.00 | 57.35 | O |
| ATOM | 378 | N | GLN | A | 89 | 12.003 | 15.477 | 14.027 | 1.00 | 60.00 | N |
| ATOM | 379 | CA | GLN | A | 89 | 10.745 | 16.114 | 14.358 | 1.00 | 62.66 | C |
| ATOM | 380 | CB | GLN | A | 89 | 9.770 | 15.145 | 15.016 | 1.00 | 63.73 | C |
| ATOM | 381 | CG | GLN | A | 89 | 8.440 | 15.820 | 15.319 | 1.00 | 66.30 | C |
| ATOM | 382 | CD | GLN | A | 89 | 7.559 | 15.871 | 14.088 | 1.00 | 67.08 | C |
| ATOM | 383 | OE1 | GLN | A | 89 | 8.047 | 15.914 | 12.945 | 1.00 | 69.15 | O |
| ATOM | 384 | NE2 | GLN | A | 89 | 6.255 | 15.878 | 14.307 | 1.00 | 67.86 | N |
| ATOM | 385 | C | GLN | A | 89 | 11.074 | 17.211 | 15.326 | 1.00 | 63.95 | C |
| ATOM | 386 | O | GLN | A | 89 | 11.746 | 16.978 | 16.335 | 1.00 | 64.56 | O |
| ATOM | 387 | N | ASP | A | 90 | 10.635 | 18.421 | 15.019 | 1.00 | 65.34 | N |
| ATOM | 388 | CA | ASP | A | 90 | 10.924 | 19.478 | 15.947 | 1.00 | 67.13 | C |
| ATOM | 389 | CB | ASP | A | 90 | 10.849 | 20.838 | 15.254 | 1.00 | 67.57 | C |
| ATOM | 390 | CG | ASP | A | 90 | 9.476 | 21.443 | 15.314 | 1.00 | 68.81 | C |
| ATOM | 391 | OD1 | ASP | A | 90 | 8.505 | 20.739 | 14.980 | 1.00 | 70.11 | O |
| ATOM | 392 | OD2 | ASP | A | 90 | 9.353 | 22.625 | 15.708 | 1.00 | 69.97 | O |
| ATOM | 393 | C | ASP | A | 90 | 9.802 | 19.275 | 16.959 | 1.00 | 67.57 | C |
| ATOM | 394 | O | ASP | A | 90 | 8.633 | 19.137 | 16.576 | 1.00 | 67.91 | O |
| ATOM | 395 | N | ALA | A | 91 | 10.150 | 19.181 | 18.238 | 1.00 | 67.48 | N |

| | | | | | | | | | | | |
|---|---|---|---|---|---|---|---|---|---|---|---|
| ATOM | 396 | CA | ALA | A | 91 | 9.138 | 18.985 | 19.277 | 1.00 | 66.84 | C |
| ATOM | 397 | CB | ALA | A | 91 | 9.812 | 18.812 | 20.641 | 1.00 | 65.38 | C |
| ATOM | 398 | C | ALA | A | 91 | 8.214 | 20.205 | 19.295 | 1.00 | 66.58 | C |
| ATOM | 399 | O | ALA | A | 91 | 8.586 | 21.268 | 18.804 | 1.00 | 66.30 | O |
| ATOM | 400 | N | ALA | A | 92 | 7.014 | 20.037 | 19.844 | 1.00 | 66.05 | N |
| ATOM | 401 | CA | ALA | A | 92 | 6.031 | 21.118 | 19.944 | 1.00 | 66.10 | C |
| ATOM | 402 | CB | ALA | A | 92 | 6.730 | 22.484 | 20.125 | 1.00 | 66.90 | C |
| ATOM | 403 | C | ALA | A | 92 | 5.054 | 21.174 | 18.758 | 1.00 | 64.95 | C |
| ATOM | 404 | O | ALA | A | 92 | 4.156 | 22.032 | 18.706 | 1.00 | 65.71 | O |
| ATOM | 405 | N | ALA | A | 93 | 5.241 | 20.267 | 17.805 | 1.00 | 63.11 | N |
| ATOM | 406 | CA | ALA | A | 93 | 4.356 | 20.147 | 16.648 | 1.00 | 59.88 | C |
| ATOM | 407 | CB | ALA | A | 93 | 5.014 | 20.676 | 15.394 | 1.00 | 58.42 | C |
| ATOM | 408 | C | ALA | A | 93 | 4.256 | 18.625 | 16.601 | 1.00 | 58.06 | C |
| ATOM | 409 | O | ALA | A | 93 | 5.288 | 17.941 | 16.562 | 1.00 | 58.04 | O |
| ATOM | 410 | N | ALA | A | 94 | 3.036 | 18.087 | 16.652 | 1.00 | 54.47 | N |
| ATOM | 411 | CA | ALA | A | 94 | 2.846 | 16.634 | 16.621 | 1.00 | 51.21 | C |
| ATOM | 412 | CB | ALA | A | 94 | 1.403 | 16.288 | 16.959 | 1.00 | 51.62 | C |
| ATOM | 413 | C | ALA | A | 94 | 3.227 | 16.026 | 15.275 | 1.00 | 48.97 | C |
| ATOM | 414 | O | ALA | A | 94 | 3.123 | 16.682 | 14.234 | 1.00 | 47.54 | O |
| ATOM | 415 | N | ASN | A | 95 | 3.687 | 14.776 | 15.310 | 1.00 | 46.82 | N |
| ATOM | 416 | CA | ASN | A | 95 | 4.096 | 14.061 | 14.097 | 1.00 | 43.68 | C |
| ATOM | 417 | CB | ASN | A | 95 | 5.253 | 13.104 | 14.386 | 1.00 | 43.32 | C |
| ATOM | 418 | CG | ASN | A | 95 | 5.868 | 12.526 | 13.112 | 1.00 | 42.96 | C |
| ATOM | 419 | OD1 | ASN | A | 95 | 5.323 | 11.606 | 12.497 | 1.00 | 41.10 | O |
| ATOM | 420 | ND2 | ASN | A | 95 | 7.011 | 13.077 | 12.710 | 1.00 | 42.91 | N |
| ATOM | 421 | C | ASN | A | 95 | 2.928 | 13.280 | 13.537 | 1.00 | 41.82 | C |
| ATOM | 422 | O | ASN | A | 95 | 2.434 | 12.328 | 14.152 | 1.00 | 42.01 | O |
| ATOM | 423 | N | ARG | A | 96 | 2.489 | 13.703 | 12.361 | 1.00 | 40.01 | N |
| ATOM | 424 | CA | ARG | A | 96 | 1.365 | 13.083 | 11.689 | 1.00 | 39.58 | C |
| ATOM | 425 | CB | ARG | A | 96 | 1.106 | 13.798 | 10.359 | 1.00 | 39.21 | C |
| ATOM | 426 | CG | ARG | A | 96 | -0.227 | 13.447 | 9.719 | 1.00 | 40.20 | C |
| ATOM | 427 | CD | ARG | A | 96 | -0.411 | 14.157 | 8.385 | 1.00 | 41.13 | C |
| ATOM | 428 | NE | ARG | A | 96 | -0.731 | 15.577 | 8.510 | 1.00 | 43.81 | N |
| ATOM | 429 | CZ | ARG | A | 96 | -0.562 | 16.461 | 7.530 | 1.00 | 44.56 | C |
| ATOM | 430 | NH1 | ARG | A | 96 | -0.072 | 16.070 | 6.361 | 1.00 | 45.91 | N |
| ATOM | 431 | NH2 | ARG | A | 96 | -0.882 | 17.735 | 7.712 | 1.00 | 45.06 | N |
| ATOM | 432 | C | ARG | A | 96 | 1.597 | 11.589 | 11.451 | 1.00 | 38.18 | C |
| ATOM | 433 | O | ARG | A | 96 | 0.761 | 10.756 | 11.806 | 1.00 | 37.82 | O |
| ATOM | 434 | N | GLU | A | 97 | 2.741 | 11.249 | 10.867 | 1.00 | 36.83 | N |
| ATOM | 435 | CA | GLU | A | 97 | 3.037 | 9.854 | 10.582 | 1.00 | 35.83 | C |
| ATOM | 436 | CB | GLU | A | 97 | 4.446 | 9.699 | 10.017 | 1.00 | 35.55 | C |
| ATOM | 437 | CG | GLU | A | 97 | 4.857 | 8.249 | 9.853 | 1.00 | 34.07 | C |
| ATOM | 438 | CD | GLU | A | 97 | 6.009 | 8.075 | 8.899 | 1.00 | 33.84 | C |
| ATOM | 439 | OE1 | GLU | A | 97 | 6.630 | 9.088 | 8.524 | 1.00 | 32.46 | O |
| ATOM | 440 | OE2 | GLU | A | 97 | 6.289 | 6.925 | 8.519 | 1.00 | 33.29 | O |
| ATOM | 441 | C | GLU | A | 97 | 2.882 | 8.973 | 11.809 | 1.00 | 36.15 | C |
| ATOM | 442 | O | GLU | A | 97 | 2.304 | 7.886 | 11.737 | 1.00 | 36.21 | O |
| ATOM | 443 | N | LEU | A | 98 | 3.394 | 9.435 | 12.942 | 1.00 | 36.98 | N |
| ATOM | 444 | CA | LEU | A | 98 | 3.282 | 8.641 | 14.150 | 1.00 | 37.65 | C |
| ATOM | 445 | CB | LEU | A | 98 | 4.145 | 9.227 | 15.273 | 1.00 | 37.12 | C |
| ATOM | 446 | CG | LEU | A | 98 | 3.973 | 8.539 | 16.636 | 1.00 | 35.69 | C |
| ATOM | 447 | CD1 | LEU | A | 98 | 4.224 | 7.043 | 16.519 | 1.00 | 37.16 | C |
| ATOM | 448 | CD2 | LEU | A | 98 | 4.914 | 9.168 | 17.630 | 1.00 | 36.61 | C |
| ATOM | 449 | C | LEU | A | 98 | 1.834 | 8.490 | 14.623 | 1.00 | 37.78 | C |
| ATOM | 450 | O | LEU | A | 98 | 1.458 | 7.409 | 15.070 | 1.00 | 36.47 | O |
| ATOM | 451 | N | GLN | A | 99 | 1.022 | 9.545 | 14.521 | 1.00 | 40.08 | N |
| ATOM | 452 | CA | GLN | A | 99 | -0.380 | 9.451 | 14.968 | 1.00 | 42.48 | C |
| ATOM | 453 | CB | GLN | A | 99 | -1.151 | 10.758 | 14.825 | 1.00 | 44.54 | C |
| ATOM | 454 | CG | GLN | A | 99 | -0.498 | 12.059 | 15.182 | 1.00 | 47.68 | C |
| ATOM | 455 | CD | GLN | A | 99 | -1.398 | 13.188 | 14.692 | 1.00 | 51.85 | C |
| ATOM | 456 | OE1 | GLN | A | 99 | -0.994 | 14.358 | 14.608 | 1.00 | 53.12 | O |
| ATOM | 457 | NE2 | GLN | A | 99 | -2.652 | 12.831 | 14.373 | 1.00 | 52.83 | N |
| ATOM | 458 | C | GLN | A | 99 | -1.176 | 8.455 | 14.141 | 1.00 | 41.10 | C |
| ATOM | 459 | O | GLN | A | 99 | -2.016 | 7.725 | 14.667 | 1.00 | 41.16 | O |
| ATOM | 460 | N | ILE | A | 100 | -0.948 | 8.475 | 12.832 | 1.00 | 40.13 | N |
| ATOM | 461 | CA | ILE | A | 100 | -1.668 | 7.586 | 11.927 | 1.00 | 39.49 | C |
| ATOM | 462 | CB | ILE | A | 100 | -1.428 | 7.976 | 10.448 | 1.00 | 38.95 | C |

| | | | | | | | | | | | |
|---|---|---|---|---|---|---|---|---|---|---|---|
| ATOM | 463 | CG2 | ILE | A | 100 | -2.033 | 6.931 | 9.527 | 1.00 | 38.51 | C |
| ATOM | 464 | CG1 | ILE | A | 100 | -2.045 | 9.342 | 10.164 | 1.00 | 37.83 | C |
| ATOM | 465 | CD1 | ILE | A | 100 | -1.838 | 9.817 | 8.750 | 1.00 | 38.82 | C |
| ATOM | 466 | C | ILE | A | 100 | -1.244 | 6.145 | 12.131 | 1.00 | 39.77 | C |
| ATOM | 467 | O | ILE | A | 100 | -2.072 | 5.236 | 12.177 | 1.00 | 39.61 | O |
| ATOM | 468 | N | MET | A | 101 | 0.059 | 5.947 | 12.265 | 1.00 | 40.38 | N |
| ATOM | 469 | CA | MET | A | 101 | 0.615 | 4.623 | 12.447 | 1.00 | 41.48 | C |
| ATOM | 470 | CB | MET | A | 101 | 2.131 | 4.719 | 12.482 | 1.00 | 42.91 | C |
| ATOM | 471 | CG | MET | A | 101 | 2.838 | 3.429 | 12.149 | 1.00 | 45.77 | C |
| ATOM | 472 | SD | MET | A | 101 | 3.125 | 3.300 | 10.375 | 1.00 | 46.26 | S |
| ATOM | 473 | CE | MET | A | 101 | 4.341 | 4.600 | 10.189 | 1.00 | 46.40 | C |
| ATOM | 474 | C | MET | A | 101 | 0.122 | 4.009 | 13.745 | 1.00 | 43.04 | C |
| ATOM | 475 | O | MET | A | 101 | -0.222 | 2.824 | 13.813 | 1.00 | 43.67 | O |
| ATOM | 476 | N | ARG | A | 102 | 0.093 | 4.836 | 14.779 | 1.00 | 44.61 | N |
| ATOM | 477 | CA | ARG | A | 102 | -0.316 | 4.421 | 16.115 | 1.00 | 45.35 | C |
| ATOM | 478 | CB | ARG | A | 102 | -0.175 | 5.612 | 17.063 | 1.00 | 46.40 | C |
| ATOM | 479 | CG | ARG | A | 102 | 0.146 | 5.254 | 18.488 | 1.00 | 48.90 | C |
| ATOM | 480 | CD | ARG | A | 102 | 1.483 | 5.849 | 18.906 | 1.00 | 50.71 | C |
| ATOM | 481 | NE | ARG | A | 102 | 1.761 | 5.545 | 20.307 | 1.00 | 52.49 | N |
| ATOM | 482 | CZ | ARG | A | 102 | 1.105 | 6.081 | 21.333 | 1.00 | 51.86 | C |
| ATOM | 483 | NH1 | ARG | A | 102 | 0.135 | 6.958 | 21.116 | 1.00 | 51.78 | N |
| ATOM | 484 | NH2 | ARG | A | 102 | 1.409 | 5.725 | 22.579 | 1.00 | 53.32 | N |
| ATOM | 485 | C | ARG | A | 102 | -1.728 | 3.829 | 16.215 | 1.00 | 45.65 | C |
| ATOM | 486 | O | ARG | A | 102 | -2.008 | 3.076 | 17.142 | 1.00 | 45.89 | O |
| ATOM | 487 | N | LYS | A | 103 | -2.621 | 4.154 | 15.283 | 1.00 | 46.19 | N |
| ATOM | 488 | CA | LYS | A | 103 | -3.971 | 3.594 | 15.360 | 1.00 | 46.14 | C |
| ATOM | 489 | CB | LYS | A | 103 | -5.023 | 4.703 | 15.262 | 1.00 | 46.77 | C |
| ATOM | 490 | CG | LYS | A | 103 | -5.418 | 5.079 | 13.845 | 1.00 | 47.77 | C |
| ATOM | 491 | CD | LYS | A | 103 | -6.403 | 6.245 | 13.828 | 1.00 | 48.79 | C |
| ATOM | 492 | CE | LYS | A | 103 | -5.748 | 7.522 | 14.322 | 1.00 | 48.62 | C |
| ATOM | 493 | NZ | LYS | A | 103 | -6.671 | 8.686 | 14.242 | 1.00 | 50.95 | N |
| ATOM | 494 | C | LYS | A | 103 | -4.259 | 2.526 | 14.308 | 1.00 | 46.13 | C |
| ATOM | 495 | O | LYS | A | 103 | -5.414 | 2.171 | 14.083 | 1.00 | 45.94 | O |
| ATOM | 496 | N | LEU | A | 104 | -3.211 | 2.010 | 13.665 | 1.00 | 44.91 | N |
| ATOM | 497 | CA | LEU | A | 104 | -3.379 | 0.982 | 12.631 | 1.00 | 43.39 | C |
| ATOM | 498 | CB | LEU | A | 104 | -2.616 | 1.387 | 11.364 | 1.00 | 41.62 | C |
| ATOM | 499 | CG | LEU | A | 104 | -3.397 | 2.001 | 10.188 | 1.00 | 40.62 | C |
| ATOM | 500 | CD1 | LEU | A | 104 | -4.469 | 2.954 | 10.674 | 1.00 | 38.09 | C |
| ATOM | 501 | CD2 | LEU | A | 104 | -2.417 | 2.698 | 9.255 | 1.00 | 39.06 | C |
| ATOM | 502 | C | LEU | A | 104 | -2.930 | -0.411 | 13.069 | 1.00 | 43.15 | C |
| ATOM | 503 | O | LEU | A | 104 | -1.864 | -0.576 | 13.656 | 1.00 | 43.20 | O |
| ATOM | 504 | N | ASP | A | 105 | -3.758 | -1.411 | 12.794 | 1.00 | 44.02 | N |
| ATOM | 505 | CA | ASP | A | 105 | -3.432 | -2.793 | 13.126 | 1.00 | 45.24 | C |
| ATOM | 506 | CB | ASP | A | 105 | -3.852 | -3.150 | 14.544 | 1.00 | 48.61 | C |
| ATOM | 507 | CG | ASP | A | 105 | -3.317 | -4.504 | 14.980 | 1.00 | 52.72 | C |
| ATOM | 508 | OD1 | ASP | A | 105 | -3.478 | -5.487 | 14.224 | 1.00 | 55.75 | O |
| ATOM | 509 | OD2 | ASP | A | 105 | -2.741 | -4.591 | 16.083 | 1.00 | 54.91 | O |
| ATOM | 510 | C | ASP | A | 105 | -4.176 | -3.672 | 12.143 | 1.00 | 45.11 | C |
| ATOM | 511 | O | ASP | A | 105 | -5.370 | -3.941 | 12.305 | 1.00 | 45.44 | O |
| ATOM | 512 | N | HIS | A | 106 | -3.450 | -4.122 | 11.124 | 1.00 | 45.21 | N |
| ATOM | 513 | CA | HIS | A | 106 | -4.020 | -4.945 | 10.072 | 1.00 | 43.84 | C |
| ATOM | 514 | CB | HIS | A | 106 | -4.617 | -4.021 | 9.006 | 1.00 | 43.75 | C |
| ATOM | 515 | CG | HIS | A | 106 | -5.464 | -4.724 | 7.996 | 1.00 | 43.80 | C |
| ATOM | 516 | CD2 | HIS | A | 106 | -6.808 | -4.862 | 7.904 | 1.00 | 43.94 | C |
| ATOM | 517 | ND1 | HIS | A | 106 | -4.934 | -5.397 | 6.917 | 1.00 | 42.56 | N |
| ATOM | 518 | CE1 | HIS | A | 106 | -5.915 | -5.919 | 6.204 | 1.00 | 43.83 | C |
| ATOM | 519 | NE2 | HIS | A | 106 | -7.064 | -5.609 | 6.783 | 1.00 | 43.92 | N |
| ATOM | 520 | C | HIS | A | 106 | -2.938 | -5.836 | 9.465 | 1.00 | 43.18 | C |
| ATOM | 521 | O | HIS | A | 106 | -1.797 | -5.406 | 9.275 | 1.00 | 42.37 | O |
| ATOM | 522 | N | CYS | A | 107 | -3.310 | -7.078 | 9.164 | 1.00 | 42.66 | N |
| ATOM | 523 | CA | CYS | A | 107 | -2.399 | -8.069 | 8.587 | 1.00 | 42.04 | C |
| ATOM | 524 | CB | CYS | A | 107 | -3.177 | -9.354 | 8.257 | 1.00 | 44.28 | C |
| ATOM | 525 | SG | CYS | A | 107 | -4.710 | -9.082 | 7.244 | 1.00 | 48.11 | S |
| ATOM | 526 | C | CYS | A | 107 | -1.669 | -7.585 | 7.335 | 1.00 | 40.91 | C |
| ATOM | 527 | O | CYS | A | 107 | -0.618 | -8.117 | 6.983 | 1.00 | 40.27 | O |
| ATOM | 528 | N | ASN | A | 108 | -2.220 | -6.578 | 6.662 | 1.00 | 39.70 | N |
| ATOM | 529 | CA | ASN | A | 108 | -1.602 | -6.078 | 5.442 | 1.00 | 38.42 | C |

| | | | | | | | | | | | |
|---|---|---|---|---|---|---|---|---|---|---|---|
| ATOM | 530 | CB | ASN | A | 108 | -2.634 | -6.042 | 4.324 | 1.00 | 38.08 | C |
| ATOM | 531 | CG | ASN | A | 108 | -3.083 | -7.428 | 3.912 | 1.00 | 38.44 | C |
| ATOM | 532 | OD1 | ASN | A | 108 | -4.282 | -7.716 | 3.856 | 1.00 | 39.21 | O |
| ATOM | 533 | ND2 | ASN | A | 108 | -2.120 | -8.298 | 3.624 | 1.00 | 35.56 | N |
| ATOM | 534 | C | ASN | A | 108 | -0.937 | -4.718 | 5.551 | 1.00 | 37.92 | C |
| ATOM | 535 | O | ASN | A | 108 | -0.855 | -3.984 | 4.568 | 1.00 | 38.00 | O |
| ATOM | 536 | N | ILE | A | 109 | -0.459 | -4.370 | 6.738 | 1.00 | 36.74 | N |
| ATOM | 537 | CA | ILE | A | 109 | 0.213 | -3.093 | 6.918 | 1.00 | 36.63 | C |
| ATOM | 538 | CB | ILE | A | 109 | -0.762 | -2.042 | 7.497 | 1.00 | 36.53 | C |
| ATOM | 539 | CG2 | ILE | A | 109 | -0.020 | -0.752 | 7.812 | 1.00 | 35.59 | C |
| ATOM | 540 | CG1 | ILE | A | 109 | -1.897 | -1.792 | 6.502 | 1.00 | 36.89 | C |
| ATOM | 541 | CD1 | ILE | A | 109 | -2.969 | -0.831 | 7.002 | 1.00 | 37.41 | C |
| ATOM | 542 | C | ILE | A | 109 | 1.390 | -3.286 | 7.862 | 1.00 | 36.44 | C |
| ATOM | 543 | O | ILE | A | 109 | 1.233 | -3.899 | 8.915 | 1.00 | 35.59 | O |
| ATOM | 544 | N | VAL | A | 110 | 2.567 | -2.789 | 7.483 | 1.00 | 37.78 | N |
| ATOM | 545 | CA | VAL | A | 110 | 3.727 | -2.933 | 8.358 | 1.00 | 40.13 | C |
| ATOM | 546 | CB | VAL | A | 110 | 4.957 | -2.096 | 7.895 | 1.00 | 40.17 | C |
| ATOM | 547 | CG1 | VAL | A | 110 | 5.335 | -2.460 | 6.470 | 1.00 | 42.25 | C |
| ATOM | 548 | CG2 | VAL | A | 110 | 4.663 | -0.615 | 8.016 | 1.00 | 41.20 | C |
| ATOM | 549 | C | VAL | A | 110 | 3.284 | -2.433 | 9.724 | 1.00 | 40.83 | C |
| ATOM | 550 | O | VAL | A | 110 | 2.620 | -1.404 | 9.831 | 1.00 | 40.96 | O |
| ATOM | 551 | N | ARG | A | 111 | 3.639 | -3.174 | 10.763 | 1.00 | 42.04 | N |
| ATOM | 552 | CA | ARG | A | 111 | 3.257 | -2.816 | 12.118 | 1.00 | 43.52 | C |
| ATOM | 553 | CB | ARG | A | 111 | 3.159 | -4.089 | 12.970 | 1.00 | 46.80 | C |
| ATOM | 554 | CG | ARG | A | 111 | 2.838 | -3.909 | 14.461 | 1.00 | 50.77 | C |
| ATOM | 555 | CD | ARG | A | 111 | 2.808 | -5.289 | 15.153 | 1.00 | 53.34 | C |
| ATOM | 556 | NE | ARG | A | 111 | 4.067 | -6.008 | 14.927 | 1.00 | 58.42 | N |
| ATOM | 557 | CZ | ARG | A | 111 | 4.170 | -7.293 | 14.574 | 1.00 | 60.50 | C |
| ATOM | 558 | NH1 | ARG | A | 111 | 3.076 | -8.029 | 14.401 | 1.00 | 61.08 | N |
| ATOM | 559 | NH2 | ARG | A | 111 | 5.372 | -7.838 | 14.388 | 1.00 | 60.00 | N |
| ATOM | 560 | C | ARG | A | 111 | 4.258 | -1.855 | 12.738 | 1.00 | 43.27 | C |
| ATOM | 561 | O | ARG | A | 111 | 5.449 | -1.890 | 12.424 | 1.00 | 42.94 | O |
| ATOM | 562 | N | LEU | A | 112 | 3.757 | -0.976 | 13.598 | 1.00 | 42.56 | N |
| ATOM | 563 | CA | LEU | A | 112 | 4.618 | -0.045 | 14.308 | 1.00 | 41.70 | C |
| ATOM | 564 | CB | LEU | A | 112 | 3.968 | 1.331 | 14.441 | 1.00 | 40.53 | C |
| ATOM | 565 | CG | LEU | A | 112 | 4.706 | 2.317 | 15.354 | 1.00 | 40.29 | C |
| ATOM | 566 | CD1 | LEU | A | 112 | 6.133 | 2.543 | 14.848 | 1.00 | 40.08 | C |
| ATOM | 567 | CD2 | LEU | A | 112 | 3.945 | 3.635 | 15.404 | 1.00 | 40.33 | C |
| ATOM | 568 | C | LEU | A | 112 | 4.775 | -0.691 | 15.678 | 1.00 | 41.18 | C |
| ATOM | 569 | O | LEU | A | 112 | 3.899 | -0.576 | 16.529 | 1.00 | 41.05 | O |
| ATOM | 570 | N | ARG | A | 113 | 5.889 | -1.397 | 15.859 | 1.00 | 41.09 | N |
| ATOM | 571 | CA | ARG | A | 113 | 6.193 | -2.099 | 17.100 | 1.00 | 40.66 | C |
| ATOM | 572 | CB | ARG | A | 113 | 7.470 | -2.923 | 16.940 | 1.00 | 43.33 | C |
| ATOM | 573 | CG | ARG | A | 113 | 7.596 | -3.697 | 15.632 | 1.00 | 46.76 | C |
| ATOM | 574 | CD | ARG | A | 113 | 6.600 | -4.837 | 15.550 | 1.00 | 50.95 | C |
| ATOM | 575 | NE | ARG | A | 113 | 7.108 | -6.094 | 16.123 | 1.00 | 55.59 | N |
| ATOM | 576 | CZ | ARG | A | 113 | 8.001 | -6.884 | 15.526 | 1.00 | 56.09 | C |
| ATOM | 577 | NH1 | ARG | A | 113 | 8.494 | -6.554 | 14.338 | 1.00 | 54.89 | N |
| ATOM | 578 | NH2 | ARG | A | 113 | 8.388 | -8.007 | 16.116 | 1.00 | 56.12 | N |
| ATOM | 579 | C | ARG | A | 113 | 6.389 | -1.123 | 18.247 | 1.00 | 39.27 | C |
| ATOM | 580 | O | ARG | A | 113 | 5.754 | -1.249 | 19.289 | 1.00 | 40.32 | O |
| ATOM | 581 | N | TYR | A | 114 | 7.288 | -0.164 | 18.057 | 1.00 | 37.25 | N |
| ATOM | 582 | CA | TYR | A | 114 | 7.582 | 0.825 | 19.084 | 1.00 | 36.78 | C |
| ATOM | 583 | CB | TYR | A | 114 | 8.762 | 0.388 | 19.953 | 1.00 | 37.87 | C |
| ATOM | 584 | CG | TYR | A | 114 | 8.709 | -1.024 | 20.464 | 1.00 | 38.86 | C |
| ATOM | 585 | CD1 | TYR | A | 114 | 7.874 | -1.372 | 21.521 | 1.00 | 39.64 | C |
| ATOM | 586 | CE1 | TYR | A | 114 | 7.841 | -2.668 | 22.012 | 1.00 | 40.22 | C |
| ATOM | 587 | CD2 | TYR | A | 114 | 9.514 | -2.013 | 19.906 | 1.00 | 39.46 | C |
| ATOM | 588 | CE2 | TYR | A | 114 | 9.489 | -3.315 | 20.389 | 1.00 | 40.55 | C |
| ATOM | 589 | CZ | TYR | A | 114 | 8.648 | -3.630 | 21.446 | 1.00 | 40.97 | C |
| ATOM | 590 | OH | TYR | A | 114 | 8.620 | -4.910 | 21.941 | 1.00 | 43.30 | O |
| ATOM | 591 | C | TYR | A | 114 | 7.999 | 2.118 | 18.429 | 1.00 | 36.81 | C |
| ATOM | 592 | O | TYR | A | 114 | 8.172 | 2.185 | 17.213 | 1.00 | 37.21 | O |
| ATOM | 593 | N | PHE | A | 115 | 8.173 | 3.140 | 19.258 | 1.00 | 35.84 | N |
| ATOM | 594 | CA | PHE | A | 115 | 8.645 | 4.433 | 18.804 | 1.00 | 35.06 | C |
| ATOM | 595 | CB | PHE | A | 115 | 7.496 | 5.317 | 18.309 | 1.00 | 35.39 | C |
| ATOM | 596 | CG | PHE | A | 115 | 6.625 | 5.879 | 19.388 | 1.00 | 34.94 | C |

| | | | | | | | | | | | |
|---|---|---|---|---|---|---|---|---|---|---|---|
| ATOM | 597 | CD1 | PHE | A | 115 | 6.877 | 7.141 | 19.920 | 1.00 | 34.27 | C |
| ATOM | 598 | CD2 | PHE | A | 115 | 5.511 | 5.174 | 19.828 | 1.00 | 34.50 | C |
| ATOM | 599 | CE1 | PHE | A | 115 | 6.026 | 7.698 | 20.872 | 1.00 | 33.97 | C |
| ATOM | 600 | CE2 | PHE | A | 115 | 4.653 | 5.717 | 20.777 | 1.00 | 34.01 | C |
| ATOM | 601 | CZ | PHE | A | 115 | 4.909 | 6.983 | 21.301 | 1.00 | 34.04 | C |
| ATOM | 602 | C | PHE | A | 115 | 9.358 | 5.034 | 19.991 | 1.00 | 34.84 | C |
| ATOM | 603 | O | PHE | A | 115 | 8.973 | 4.799 | 21.132 | 1.00 | 35.55 | O |
| ATOM | 604 | N | PHE | A | 116 | 10.422 | 5.778 | 19.735 | 1.00 | 34.85 | N |
| ATOM | 605 | CA | PHE | A | 116 | 11.169 | 6.346 | 20.832 | 1.00 | 34.58 | C |
| ATOM | 606 | CB | PHE | A | 116 | 12.074 | 5.278 | 21.429 | 1.00 | 32.94 | C |
| ATOM | 607 | CG | PHE | A | 116 | 13.153 | 4.805 | 20.492 | 1.00 | 32.76 | C |
| ATOM | 608 | CD1 | PHE | A | 116 | 14.345 | 5.509 | 20.365 | 1.00 | 31.49 | C |
| ATOM | 609 | CD2 | PHE | A | 116 | 12.987 | 3.647 | 19.744 | 1.00 | 31.17 | C |
| ATOM | 610 | CE1 | PHE | A | 116 | 15.354 | 5.064 | 19.511 | 1.00 | 30.35 | C |
| ATOM | 611 | CE2 | PHE | A | 116 | 13.994 | 3.201 | 18.891 | 1.00 | 29.60 | C |
| ATOM | 612 | CZ | PHE | A | 116 | 15.176 | 3.910 | 18.776 | 1.00 | 28.32 | C |
| ATOM | 613 | C | PHE | A | 116 | 12.002 | 7.510 | 20.347 | 1.00 | 36.20 | C |
| ATOM | 614 | O | PHE | A | 116 | 12.433 | 7.535 | 19.198 | 1.00 | 37.30 | O |
| ATOM | 615 | N | TYR | A | 117 | 12.250 | 8.463 | 21.237 | 1.00 | 36.02 | N |
| ATOM | 616 | CA | TYR | A | 117 | 13.024 | 9.641 | 20.894 | 1.00 | 36.94 | C |
| ATOM | 617 | CB | TYR | A | 117 | 12.421 | 10.850 | 21.606 | 1.00 | 37.41 | C |
| ATOM | 618 | CG | TYR | A | 117 | 10.950 | 10.989 | 21.321 | 1.00 | 38.65 | C |
| ATOM | 619 | CD1 | TYR | A | 117 | 10.495 | 11.803 | 20.285 | 1.00 | 39.49 | C |
| ATOM | 620 | CE1 | TYR | A | 117 | 9.150 | 11.837 | 19.931 | 1.00 | 40.05 | C |
| ATOM | 621 | CD2 | TYR | A | 117 | 10.021 | 10.215 | 22.008 | 1.00 | 37.91 | C |
| ATOM | 622 | CE2 | TYR | A | 117 | 8.680 | 10.234 | 21.666 | 1.00 | 39.76 | C |
| ATOM | 623 | CZ | TYR | A | 117 | 8.250 | 11.045 | 20.624 | 1.00 | 41.49 | C |
| ATOM | 624 | OH | TYR | A | 117 | 6.922 | 11.035 | 20.256 | 1.00 | 44.67 | O |
| ATOM | 625 | C | TYR | A | 117 | 14.491 | 9.475 | 21.260 | 1.00 | 38.18 | C |
| ATOM | 626 | O | TYR | A | 117 | 14.833 | 8.755 | 22.200 | 1.00 | 38.23 | O |
| ATOM | 627 | N | SER | A | 118 | 15.356 | 10.145 | 20.506 | 1.00 | 40.00 | N |
| ATOM | 628 | CA | SER | A | 118 | 16.794 | 10.081 | 20.729 | 1.00 | 42.18 | C |
| ATOM | 629 | CB | SER | A | 118 | 17.402 | 8.975 | 19.862 | 1.00 | 42.36 | C |
| ATOM | 630 | OG | SER | A | 118 | 17.286 | 9.294 | 18.483 | 1.00 | 40.42 | O |
| ATOM | 631 | C | SER | A | 118 | 17.438 | 11.423 | 20.384 | 1.00 | 44.18 | C |
| ATOM | 632 | O | SER | A | 118 | 16.832 | 12.243 | 19.688 | 1.00 | 43.20 | O |
| ATOM | 633 | N | SER | A | 119 | 18.659 | 11.649 | 20.870 | 1.00 | 47.49 | N |
| ATOM | 634 | CA | SER | A | 119 | 19.378 | 12.900 | 20.588 | 1.00 | 49.81 | C |
| ATOM | 635 | CB | SER | A | 119 | 20.067 | 13.428 | 21.853 | 1.00 | 50.48 | C |
| ATOM | 636 | OG | SER | A | 119 | 19.132 | 13.978 | 22.777 | 1.00 | 50.85 | O |
| ATOM | 637 | C | SER | A | 119 | 20.423 | 12.708 | 19.491 | 1.00 | 50.28 | C |
| ATOM | 638 | O | SER | A | 119 | 20.453 | 13.459 | 18.510 | 1.00 | 51.12 | O |
| ATOM | 639 | N | ALA | A | 125 | 19.254 | 17.623 | 19.061 | 1.00 | 63.10 | N |
| ATOM | 640 | CA | ALA | A | 125 | 17.879 | 17.981 | 18.681 | 1.00 | 62.43 | C |
| ATOM | 641 | CB | ALA | A | 125 | 17.825 | 18.351 | 17.199 | 1.00 | 61.56 | C |
| ATOM | 642 | C | ALA | A | 125 | 17.044 | 16.737 | 18.952 | 1.00 | 61.66 | C |
| ATOM | 643 | O | ALA | A | 125 | 17.556 | 15.773 | 19.527 | 1.00 | 62.56 | O |
| ATOM | 644 | N | ALA | A | 126 | 15.775 | 16.734 | 18.562 | 1.00 | 59.77 | N |
| ATOM | 645 | CA | ALA | A | 126 | 14.961 | 15.543 | 18.814 | 1.00 | 58.10 | C |
| ATOM | 646 | CB | ALA | A | 126 | 13.700 | 15.915 | 19.589 | 1.00 | 57.48 | C |
| ATOM | 647 | C | ALA | A | 126 | 14.591 | 14.776 | 17.553 | 1.00 | 56.22 | C |
| ATOM | 648 | O | ALA | A | 126 | 14.018 | 15.322 | 16.609 | 1.00 | 56.30 | O |
| ATOM | 649 | N | TYR | A | 127 | 14.951 | 13.501 | 17.538 | 1.00 | 53.41 | N |
| ATOM | 650 | CA | TYR | A | 127 | 14.624 | 12.650 | 16.413 | 1.00 | 52.14 | C |
| ATOM | 651 | CB | TYR | A | 127 | 15.860 | 11.902 | 15.888 | 1.00 | 54.85 | C |
| ATOM | 652 | CG | TYR | A | 127 | 16.961 | 12.811 | 15.386 | 1.00 | 58.96 | C |
| ATOM | 653 | CD1 | TYR | A | 127 | 17.493 | 13.811 | 16.213 | 1.00 | 61.45 | C |
| ATOM | 654 | CE1 | TYR | A | 127 | 18.520 | 14.645 | 15.773 | 1.00 | 62.79 | C |
| ATOM | 655 | CD2 | TYR | A | 127 | 17.486 | 12.666 | 14.100 | 1.00 | 60.32 | C |
| ATOM | 656 | CE2 | TYR | A | 127 | 18.516 | 13.489 | 13.644 | 1.00 | 62.10 | C |
| ATOM | 657 | CZ | TYR | A | 127 | 19.029 | 14.479 | 14.489 | 1.00 | 64.13 | C |
| ATOM | 658 | OH | TYR | A | 127 | 20.056 | 15.308 | 14.052 | 1.00 | 65.95 | O |
| ATOM | 659 | C | TYR | A | 127 | 13.592 | 11.665 | 16.926 | 1.00 | 49.42 | C |
| ATOM | 660 | O | TYR | A | 127 | 13.612 | 11.274 | 18.099 | 1.00 | 47.74 | O |
| ATOM | 661 | N | LEU | A | 128 | 12.657 | 11.312 | 16.052 | 1.00 | 46.30 | N |
| ATOM | 662 | CA | LEU | A | 128 | 11.614 | 10.365 | 16.386 | 1.00 | 43.43 | C |
| ATOM | 663 | CB | LEU | A | 128 | 10.266 | 10.831 | 15.855 | 1.00 | 43.53 | C |

| | | | | | | | | | | | |
|---|---|---|---|---|---|---|---|---|---|---|---|
| ATOM | 664 | CG | LEU | A | 128 | 9.203 | 9.731 | 15.868 | 1.00 | 44.46 | C |
| ATOM | 665 | CD1 | LEU | A | 128 | 8.900 | 9.318 | 17.308 | 1.00 | 44.75 | C |
| ATOM | 666 | CD2 | LEU | A | 128 | 7.945 | 10.240 | 15.172 | 1.00 | 44.80 | C |
| ATOM | 667 | C | LEU | A | 128 | 12.008 | 9.088 | 15.691 | 1.00 | 41.86 | C |
| ATOM | 668 | O | LEU | A | 128 | 12.319 | 9.098 | 14.501 | 1.00 | 42.35 | O |
| ATOM | 669 | N | ASN | A | 129 | 11.998 | 7.987 | 16.428 | 1.00 | 40.06 | N |
| ATOM | 670 | CA | ASN | A | 129 | 12.367 | 6.705 | 15.855 | 1.00 | 37.56 | C |
| ATOM | 671 | CB | ASN | A | 129 | 13.469 | 6.052 | 16.694 | 1.00 | 36.76 | C |
| ATOM | 672 | CG | ASN | A | 129 | 14.727 | 6.889 | 16.750 | 1.00 | 36.24 | C |
| ATOM | 673 | OD1 | ASN | A | 129 | 15.621 | 6.744 | 15.924 | 1.00 | 34.39 | O |
| ATOM | 674 | ND2 | ASN | A | 129 | 14.792 | 7.786 | 17.723 | 1.00 | 36.12 | N |
| ATOM | 675 | C | ASN | A | 129 | 11.162 | 5.799 | 15.803 | 1.00 | 36.77 | C |
| ATOM | 676 | O | ASN | A | 129 | 10.439 | 5.667 | 16.785 | 1.00 | 36.75 | O |
| ATOM | 677 | N | LEU | A | 130 | 10.945 | 5.187 | 14.647 | 1.00 | 36.70 | N |
| ATOM | 678 | CA | LEU | A | 130 | 9.839 | 4.260 | 14.474 | 1.00 | 35.85 | C |
| ATOM | 679 | CB | LEU | A | 130 | 9.005 | 4.649 | 13.253 | 1.00 | 35.92 | C |
| ATOM | 680 | CG | LEU | A | 130 | 8.254 | 5.969 | 13.375 | 1.00 | 35.86 | C |
| ATOM | 681 | CD1 | LEU | A | 130 | 7.733 | 6.384 | 12.023 | 1.00 | 35.53 | C |
| ATOM | 682 | CD2 | LEU | A | 130 | 7.121 | 5.821 | 14.386 | 1.00 | 36.59 | C |
| ATOM | 683 | C | LEU | A | 130 | 10.419 | 2.873 | 14.269 | 1.00 | 35.05 | C |
| ATOM | 684 | O | LEU | A | 130 | 11.231 | 2.660 | 13.362 | 1.00 | 35.24 | O |
| ATOM | 685 | N | VAL | A | 131 | 10.025 | 1.936 | 15.123 | 1.00 | 32.91 | N |
| ATOM | 686 | CA | VAL | A | 131 | 10.503 | 0.565 | 15.005 | 1.00 | 32.56 | C |
| ATOM | 687 | CB | VAL | A | 131 | 10.709 | -0.081 | 16.364 | 1.00 | 32.06 | C |
| ATOM | 688 | CG1 | VAL | A | 131 | 11.301 | -1.465 | 16.178 | 1.00 | 32.00 | C |
| ATOM | 689 | CG2 | VAL | A | 131 | 11.628 | 0.790 | 17.206 | 1.00 | 32.32 | C |
| ATOM | 690 | C | VAL | A | 131 | 9.434 | -0.181 | 14.247 | 1.00 | 32.82 | C |
| ATOM | 691 | O | VAL | A | 131 | 8.343 | -0.404 | 14.756 | 1.00 | 33.20 | O |
| ATOM | 692 | N | LEU | A | 132 | 9.758 | -0.583 | 13.027 | 1.00 | 32.81 | N |
| ATOM | 693 | CA | LEU | A | 132 | 8.779 | -1.236 | 12.180 | 1.00 | 34.13 | C |
| ATOM | 694 | CB | LEU | A | 132 | 8.534 | -0.349 | 10.966 | 1.00 | 34.16 | C |
| ATOM | 695 | CG | LEU | A | 132 | 8.162 | 1.088 | 11.327 | 1.00 | 35.13 | C |
| ATOM | 696 | CD1 | LEU | A | 132 | 8.575 | 2.043 | 10.225 | 1.00 | 32.26 | C |
| ATOM | 697 | CD2 | LEU | A | 132 | 6.689 | 1.146 | 11.596 | 1.00 | 33.83 | C |
| ATOM | 698 | C | LEU | A | 132 | 9.117 | -2.633 | 11.701 | 1.00 | 35.71 | C |
| ATOM | 699 | O | LEU | A | 132 | 10.258 | -3.073 | 11.775 | 1.00 | 37.31 | O |
| ATOM | 700 | N | ASP | A | 133 | 8.095 | -3.329 | 11.215 | 1.00 | 36.85 | N |
| ATOM | 701 | CA | ASP | A | 133 | 8.250 | -4.666 | 10.660 | 1.00 | 38.12 | C |
| ATOM | 702 | CB | ASP | A | 133 | 6.953 | -5.115 | 9.973 | 1.00 | 40.49 | C |
| ATOM | 703 | CG | ASP | A | 133 | 5.927 | -5.692 | 10.930 | 1.00 | 44.60 | C |
| ATOM | 704 | OD1 | ASP | A | 133 | 4.709 | -5.579 | 10.605 | 1.00 | 44.70 | O |
| ATOM | 705 | OD2 | ASP | A | 133 | 6.325 | -6.277 | 11.977 | 1.00 | 45.47 | O |
| ATOM | 706 | C | ASP | A | 133 | 9.320 | -4.522 | 9.578 | 1.00 | 38.99 | C |
| ATOM | 707 | O | ASP | A | 133 | 9.327 | -3.535 | 8.846 | 1.00 | 38.10 | O |
| ATOM | 708 | N | TYR | A | 134 | 10.207 | -5.504 | 9.469 | 1.00 | 39.30 | N |
| ATOM | 709 | CA | TYR | A | 134 | 11.234 | -5.467 | 8.443 | 1.00 | 39.33 | C |
| ATOM | 710 | CB | TYR | A | 134 | 12.612 | -5.773 | 9.021 | 1.00 | 39.76 | C |
| ATOM | 711 | CG | TYR | A | 134 | 13.680 | -5.815 | 7.951 | 1.00 | 39.51 | C |
| ATOM | 712 | CD1 | TYR | A | 134 | 14.180 | -4.639 | 7.404 | 1.00 | 40.92 | C |
| ATOM | 713 | CE1 | TYR | A | 134 | 15.116 | -4.656 | 6.381 | 1.00 | 40.43 | C |
| ATOM | 714 | CD2 | TYR | A | 134 | 14.149 | -7.027 | 7.443 | 1.00 | 39.02 | C |
| ATOM | 715 | CE2 | TYR | A | 134 | 15.094 | -7.055 | 6.409 | 1.00 | 39.00 | C |
| ATOM | 716 | CZ | TYR | A | 134 | 15.567 | -5.857 | 5.891 | 1.00 | 39.88 | C |
| ATOM | 717 | OH | TYR | A | 134 | 16.494 | -5.842 | 4.880 | 1.00 | 41.55 | O |
| ATOM | 718 | C | TYR | A | 134 | 10.932 | -6.492 | 7.352 | 1.00 | 40.69 | C |
| ATOM | 719 | O | TYR | A | 134 | 11.207 | -7.682 | 7.512 | 1.00 | 42.07 | O |
| ATOM | 720 | N | VAL | A | 135 | 10.362 | -6.031 | 6.247 | 1.00 | 40.64 | N |
| ATOM | 721 | CA | VAL | A | 135 | 10.038 | -6.914 | 5.140 | 1.00 | 40.59 | C |
| ATOM | 722 | CB | VAL | A | 135 | 8.546 | -6.802 | 4.788 | 1.00 | 39.31 | C |
| ATOM | 723 | CG1 | VAL | A | 135 | 8.150 | -7.878 | 3.804 | 1.00 | 36.24 | C |
| ATOM | 724 | CG2 | VAL | A | 135 | 7.720 | -6.927 | 6.064 | 1.00 | 37.10 | C |
| ATOM | 725 | C | VAL | A | 135 | 10.940 | -6.474 | 3.985 | 1.00 | 42.91 | C |
| ATOM | 726 | O | VAL | A | 135 | 10.805 | -5.373 | 3.448 | 1.00 | 43.66 | O |
| ATOM | 727 | N | PRO | A | 136 | 11.879 | -7.351 | 3.594 | 1.00 | 44.14 | N |
| ATOM | 728 | CD | PRO | A | 136 | 11.793 | -8.774 | 3.994 | 1.00 | 44.36 | C |
| ATOM | 729 | CA | PRO | A | 136 | 12.880 | -7.174 | 2.536 | 1.00 | 44.14 | C |
| ATOM | 730 | CB | PRO | A | 136 | 13.702 | -8.452 | 2.645 | 1.00 | 44.33 | C |

| | | | | | | | | | | | |
|---|---|---|---|---|---|---|---|---|---|---|---|
| ATOM | 731 | CG | PRO | A | 136 | 12.652 | -9.468 | 2.960 | 1.00 | 44.28 | C |
| ATOM | 732 | C | PRO | A | 136 | 12.392 | -6.916 | 1.107 | 1.00 | 43.77 | C |
| ATOM | 733 | O | PRO | A | 136 | 12.746 | -5.906 | 0.500 | 1.00 | 43.24 | O |
| ATOM | 734 | N | GLU | A | 137 | 11.595 | -7.837 | 0.573 | 1.00 | 42.36 | N |
| ATOM | 735 | CA | GLU | A | 137 | 11.083 | -7.724 | -0.787 | 1.00 | 40.10 | C |
| ATOM | 736 | CB | GLU | A | 137 | 10.617 | -9.091 | -1.286 | 1.00 | 41.23 | C |
| ATOM | 737 | CG | GLU | A | 137 | 11.721 | -9.902 | -1.914 | 1.00 | 44.55 | C |
| ATOM | 738 | CD | GLU | A | 137 | 12.230 | -9.264 | -3.193 | 1.00 | 45.70 | C |
| ATOM | 739 | OE1 | GLU | A | 137 | 12.578 | -8.061 | -3.182 | 1.00 | 46.35 | O |
| ATOM | 740 | OE2 | GLU | A | 137 | 12.279 | -9.967 | -4.213 | 1.00 | 47.63 | O |
| ATOM | 741 | C | GLU | A | 137 | 9.959 | -6.725 | -0.990 | 1.00 | 37.83 | C |
| ATOM | 742 | O | GLU | A | 137 | 9.156 | -6.472 | -0.090 | 1.00 | 37.20 | O |
| ATOM | 743 | N | THR | A | 138 | 9.908 | -6.180 | -2.200 | 1.00 | 35.00 | N |
| ATOM | 744 | CA | THR | A | 138 | 8.896 | -5.209 | -2.590 | 1.00 | 33.85 | C |
| ATOM | 745 | CB | THR | A | 138 | 9.494 | -3.797 | -2.746 | 1.00 | 33.62 | C |
| ATOM | 746 | OG1 | THR | A | 138 | 10.460 | -3.799 | -3.805 | 1.00 | 33.42 | O |
| ATOM | 747 | CG2 | THR | A | 138 | 10.160 | -3.357 | -1.457 | 1.00 | 31.39 | C |
| ATOM | 748 | C | THR | A | 138 | 8.334 | -5.644 | -3.937 | 1.00 | 33.82 | C |
| ATOM | 749 | O | THR | A | 138 | 8.970 | -6.402 | -4.666 | 1.00 | 33.21 | O |
| ATOM | 750 | N | VAL | A | 139 | 7.137 | -5.180 | -4.265 | 1.00 | 32.62 | N |
| ATOM | 751 | CA | VAL | A | 139 | 6.545 | -5.548 | -5.531 | 1.00 | 31.68 | C |
| ATOM | 752 | CB | VAL | A | 139 | 5.083 | -5.051 | -5.620 | 1.00 | 32.08 | C |
| ATOM | 753 | CG1 | VAL | A | 139 | 4.530 | -5.227 | -7.025 | 1.00 | 30.55 | C |
| ATOM | 754 | CG2 | VAL | A | 139 | 4.228 | -5.828 | -4.629 | 1.00 | 29.37 | C |
| ATOM | 755 | C | VAL | A | 139 | 7.393 | -4.945 | -6.641 | 1.00 | 33.82 | C |
| ATOM | 756 | O | VAL | A | 139 | 7.528 | -5.534 | -7.709 | 1.00 | 35.61 | O |
| ATOM | 757 | N | TYR | A | 140 | 7.991 | -3.784 | -6.384 | 1.00 | 34.34 | N |
| ATOM | 758 | CA | TYR | A | 140 | 8.832 | -3.133 | -7.387 | 1.00 | 34.89 | C |
| ATOM | 759 | CB | TYR | A | 140 | 9.368 | -1.795 | -6.876 | 1.00 | 33.04 | C |
| ATOM | 760 | CG | TYR | A | 140 | 10.414 | -1.200 | -7.792 | 1.00 | 32.64 | C |
| ATOM | 761 | CD1 | TYR | A | 140 | 10.056 | -0.549 | -8.970 | 1.00 | 33.66 | C |
| ATOM | 762 | CE1 | TYR | A | 140 | 11.026 | -0.049 | -9.841 | 1.00 | 33.97 | C |
| ATOM | 763 | CD2 | TYR | A | 140 | 11.768 | -1.336 | -7.505 | 1.00 | 32.31 | C |
| ATOM | 764 | CE2 | TYR | A | 140 | 12.744 | -0.843 | -8.365 | 1.00 | 32.84 | C |
| ATOM | 765 | CZ | TYR | A | 140 | 12.370 | -0.202 | -9.529 | 1.00 | 34.48 | C |
| ATOM | 766 | OH | TYR | A | 140 | 13.338 | 0.285 | -10.376 | 1.00 | 35.84 | O |
| ATOM | 767 | C | TYR | A | 140 | 10.016 | -4.000 | -7.834 | 1.00 | 36.41 | C |
| ATOM | 768 | O | TYR | A | 140 | 10.317 | -4.066 | -9.019 | 1.00 | 38.19 | O |
| ATOM | 769 | N | ARG | A | 141 | 10.688 | -4.646 | -6.889 | 1.00 | 36.70 | N |
| ATOM | 770 | CA | ARG | A | 141 | 11.827 | -5.488 | -7.215 | 1.00 | 38.19 | C |
| ATOM | 771 | CB | ARG | A | 141 | 12.595 | -5.856 | -5.948 | 1.00 | 39.78 | C |
| ATOM | 772 | CG | ARG | A | 141 | 13.393 | -4.712 | -5.355 | 1.00 | 44.37 | C |
| ATOM | 773 | CD | ARG | A | 141 | 13.381 | -4.761 | -3.835 | 1.00 | 47.97 | C |
| ATOM | 774 | NE | ARG | A | 141 | 14.655 | -4.313 | -3.283 | 1.00 | 52.29 | N |
| ATOM | 775 | CZ | ARG | A | 141 | 15.777 | -5.028 | -3.321 | 1.00 | 54.55 | C |
| ATOM | 776 | NH1 | ARG | A | 141 | 15.776 | -6.232 | -3.886 | 1.00 | 55.12 | N |
| ATOM | 777 | NH2 | ARG | A | 141 | 16.896 | -4.540 | -2.796 | 1.00 | 56.07 | N |
| ATOM | 778 | C | ARG | A | 141 | 11.394 | -6.757 | -7.934 | 1.00 | 38.50 | C |
| ATOM | 779 | O | ARG | A | 141 | 12.091 | -7.244 | -8.823 | 1.00 | 37.55 | O |
| ATOM | 780 | N | VAL | A | 142 | 10.245 | -7.297 | -7.548 | 1.00 | 37.99 | N |
| ATOM | 781 | CA | VAL | A | 142 | 9.738 | -8.500 | -8.179 | 1.00 | 36.91 | C |
| ATOM | 782 | CB | VAL | A | 142 | 8.535 | -9.073 | -7.400 | 1.00 | 37.14 | C |
| ATOM | 783 | CG1 | VAL | A | 142 | 7.815 | -10.128 | -8.219 | 1.00 | 37.75 | C |
| ATOM | 784 | CG2 | VAL | A | 142 | 9.028 | -9.686 | -6.099 | 1.00 | 36.91 | C |
| ATOM | 785 | C | VAL | A | 142 | 9.347 | -8.210 | -9.621 | 1.00 | 37.87 | C |
| ATOM | 786 | O | VAL | A | 142 | 9.754 | -8.930 | -10.533 | 1.00 | 39.44 | O |
| ATOM | 787 | N | ALA | A | 143 | 8.575 | -7.151 | -9.838 | 1.00 | 37.04 | N |
| ATOM | 788 | CA | ALA | A | 143 | 8.164 | -6.795 | -11.190 | 1.00 | 37.69 | C |
| ATOM | 789 | CB | ALA | A | 143 | 7.289 | -5.559 | -11.161 | 1.00 | 36.63 | C |
| ATOM | 790 | C | ALA | A | 143 | 9.401 | -6.544 | -12.041 | 1.00 | 38.72 | C |
| ATOM | 791 | O | ALA | A | 143 | 9.479 | -6.981 | -13.188 | 1.00 | 39.93 | O |
| ATOM | 792 | N | ARG | A | 144 | 10.372 | -5.851 | -11.457 | 1.00 | 40.46 | N |
| ATOM | 793 | CA | ARG | A | 144 | 11.623 | -5.522 | -12.129 | 1.00 | 41.87 | C |
| ATOM | 794 | CB | ARG | A | 144 | 12.489 | -4.682 | -11.179 | 1.00 | 43.17 | C |
| ATOM | 795 | CG | ARG | A | 144 | 13.383 | -3.638 | -11.845 | 1.00 | 43.11 | C |
| ATOM | 796 | CD | ARG | A | 144 | 14.845 | -4.003 | -11.694 | 1.00 | 44.28 | C |
| ATOM | 797 | NE | ARG | A | 144 | 15.239 | -4.189 | -10.295 | 1.00 | 46.64 | N |

| | | | | | | | | | | | |
|---|---|---|---|---|---|---|---|---|---|---|---|
| ATOM | 798 | CZ | ARG | A | 144 | 15.623 | -3.207 | -9.478 | 1.00 | 47.53 | C |
| ATOM | 799 | NH1 | ARG | A | 144 | 15.677 | -1.953 | -9.910 | 1.00 | 48.31 | N |
| ATOM | 800 | NH2 | ARG | A | 144 | 15.952 | -3.480 | -8.226 | 1.00 | 46.85 | N |
| ATOM | 801 | C | ARG | A | 144 | 12.351 | -6.801 | -12.575 | 1.00 | 42.38 | C |
| ATOM | 802 | O | ARG | A | 144 | 12.798 | -6.891 | -13.715 | 1.00 | 42.09 | O |
| ATOM | 803 | N | HIS | A | 145 | 12.449 | -7.785 | -11.680 | 1.00 | 43.86 | N |
| ATOM | 804 | CA | HIS | A | 145 | 13.100 | -9.065 | -11.975 | 1.00 | 45.92 | C |
| ATOM | 805 | CB | HIS | A | 145 | 13.083 | -9.949 | -10.710 | 1.00 | 49.72 | C |
| ATOM | 806 | CG | HIS | A | 145 | 13.675 | -11.321 | -10.887 | 1.00 | 55.23 | C |
| ATOM | 807 | CD2 | HIS | A | 145 | 14.173 | -11.958 | -11.980 | 1.00 | 57.20 | C |
| ATOM | 808 | ND1 | HIS | A | 145 | 13.769 | -12.225 | -9.845 | 1.00 | 56.56 | N |
| ATOM | 809 | CE1 | HIS | A | 145 | 14.294 | -13.357 | -10.288 | 1.00 | 56.99 | C |
| ATOM | 810 | NE2 | HIS | A | 145 | 14.547 | -13.222 | -11.580 | 1.00 | 57.40 | N |
| ATOM | 811 | C | HIS | A | 145 | 12.413 | -9.769 | -13.158 | 1.00 | 45.51 | C |
| ATOM | 812 | O | HIS | A | 145 | 13.090 | -10.219 | -14.075 | 1.00 | 46.01 | O |
| ATOM | 813 | N | TYR | A | 146 | 11.080 | -9.859 | -13.153 | 1.00 | 45.04 | N |
| ATOM | 814 | CA | TYR | A | 146 | 10.369 | -10.508 | -14.253 | 1.00 | 43.09 | C |
| ATOM | 815 | CB | TYR | A | 146 | 8.876 | -10.644 | -13.964 | 1.00 | 41.22 | C |
| ATOM | 816 | CG | TYR | A | 146 | 8.528 | -11.792 | -13.066 | 1.00 | 41.40 | C |
| ATOM | 817 | CD1 | TYR | A | 146 | 8.739 | -11.704 | -11.701 | 1.00 | 41.17 | C |
| ATOM | 818 | CE1 | TYR | A | 146 | 8.477 | -12.773 | -10.867 | 1.00 | 42.77 | C |
| ATOM | 819 | CD2 | TYR | A | 146 | 8.037 | -12.992 | -13.587 | 1.00 | 40.38 | C |
| ATOM | 820 | CE2 | TYR | A | 146 | 7.770 | -14.079 | -12.751 | 1.00 | 41.66 | C |
| ATOM | 821 | CZ | TYR | A | 146 | 7.998 | -13.955 | -11.391 | 1.00 | 41.86 | C |
| ATOM | 822 | OH | TYR | A | 146 | 7.754 | -15.002 | -10.535 | 1.00 | 42.64 | O |
| ATOM | 823 | C | TYR | A | 146 | 10.512 | -9.743 | -15.535 | 1.00 | 44.00 | C |
| ATOM | 824 | O | TYR | A | 146 | 10.647 | -10.328 | -16.609 | 1.00 | 46.60 | O |
| ATOM | 825 | N | SER | A | 147 | 10.462 | -8.427 | -15.424 | 1.00 | 45.12 | N |
| ATOM | 826 | CA | SER | A | 147 | 10.549 | -7.548 | -16.583 | 1.00 | 46.45 | C |
| ATOM | 827 | CB | SER | A | 147 | 10.155 | -6.131 | -16.162 | 1.00 | 46.80 | C |
| ATOM | 828 | OG | SER | A | 147 | 10.261 | -5.235 | -17.247 | 1.00 | 48.70 | O |
| ATOM | 829 | C | SER | A | 147 | 11.916 | -7.537 | -17.264 | 1.00 | 47.98 | C |
| ATOM | 830 | O | SER | A | 147 | 12.000 | -7.576 | -18.489 | 1.00 | 47.18 | O |
| ATOM | 831 | N | ARG | A | 148 | 12.990 | -7.474 | -16.480 | 1.00 | 49.60 | N |
| ATOM | 832 | CA | ARG | A | 148 | 14.321 | -7.471 | -17.073 | 1.00 | 51.35 | C |
| ATOM | 833 | CB | ARG | A | 148 | 15.397 | -7.350 | -16.001 | 1.00 | 51.34 | C |
| ATOM | 834 | CG | ARG | A | 148 | 15.718 | -5.944 | -15.511 | 1.00 | 54.79 | C |
| ATOM | 835 | CD | ARG | A | 148 | 16.519 | -6.121 | -14.234 | 1.00 | 55.82 | C |
| ATOM | 836 | NE | ARG | A | 148 | 17.042 | -4.906 | -13.615 | 1.00 | 57.73 | N |
| ATOM | 837 | CZ | ARG | A | 148 | 17.548 | -4.891 | -12.382 | 1.00 | 58.91 | C |
| ATOM | 838 | NH1 | ARG | A | 148 | 17.574 | -6.017 | -11.672 | 1.00 | 59.55 | N |
| ATOM | 839 | NH2 | ARG | A | 148 | 18.046 | -3.771 | -11.870 | 1.00 | 59.17 | N |
| ATOM | 840 | C | ARG | A | 148 | 14.538 | -8.775 | -17.840 | 1.00 | 52.45 | C |
| ATOM | 841 | O | ARG | A | 148 | 15.307 | -8.821 | -18.803 | 1.00 | 53.17 | O |
| ATOM | 842 | N | ALA | A | 149 | 13.869 | -9.837 | -17.407 | 1.00 | 52.30 | N |
| ATOM | 843 | CA | ALA | A | 149 | 14.009 | -11.132 | -18.065 | 1.00 | 53.57 | C |
| ATOM | 844 | CB | ALA | A | 149 | 13.957 | -12.254 | -17.033 | 1.00 | 53.24 | C |
| ATOM | 845 | C | ALA | A | 149 | 12.923 | -11.338 | -19.108 | 1.00 | 54.81 | C |
| ATOM | 846 | O | ALA | A | 149 | 12.572 | -12.471 | -19.450 | 1.00 | 55.17 | O |
| ATOM | 847 | N | LYS | A | 150 | 12.383 | -10.234 | -19.603 | 1.00 | 56.15 | N |
| ATOM | 848 | CA | LYS | A | 150 | 11.339 | -10.288 | -20.615 | 1.00 | 56.52 | C |
| ATOM | 849 | CB | LYS | A | 150 | 11.956 | -10.554 | -21.987 | 1.00 | 58.63 | C |
| ATOM | 850 | CG | LYS | A | 150 | 11.581 | -9.508 | -23.029 | 1.00 | 60.61 | C |
| ATOM | 851 | CD | LYS | A | 150 | 11.921 | -8.104 | -22.566 | 1.00 | 61.80 | C |
| ATOM | 852 | CE | LYS | A | 150 | 13.396 | -7.789 | -22.767 | 1.00 | 62.72 | C |
| ATOM | 853 | NZ | LYS | A | 150 | 13.705 | -7.383 | -24.186 | 1.00 | 63.65 | N |
| ATOM | 854 | C | LYS | A | 150 | 10.290 | -11.345 | -20.317 | 1.00 | 55.71 | C |
| ATOM | 855 | O | LYS | A | 150 | 9.898 | -12.111 | -21.196 | 1.00 | 55.79 | O |
| ATOM | 856 | N | GLN | A | 151 | 9.842 | -11.392 | -19.071 | 1.00 | 54.47 | N |
| ATOM | 857 | CA | GLN | A | 151 | 8.814 | -12.346 | -18.702 | 1.00 | 53.41 | C |
| ATOM | 858 | CB | GLN | A | 151 | 9.392 | -13.457 | -17.836 | 1.00 | 55.06 | C |
| ATOM | 859 | CG | GLN | A | 151 | 8.441 | -14.618 | -17.692 | 1.00 | 57.19 | C |
| ATOM | 860 | CD | GLN | A | 151 | 9.165 | -15.924 | -17.738 | 1.00 | 57.66 | C |
| ATOM | 861 | OE1 | GLN | A | 151 | 10.098 | -16.143 | -16.969 | 1.00 | 58.63 | O |
| ATOM | 862 | NE2 | GLN | A | 151 | 8.750 | -16.807 | -18.642 | 1.00 | 58.04 | N |
| ATOM | 863 | C | GLN | A | 151 | 7.707 | -11.612 | -17.957 | 1.00 | 52.09 | C |
| ATOM | 864 | O | GLN | A | 151 | 7.891 | -10.479 | -17.512 | 1.00 | 51.62 | O |

| | | | | | | | | | | | |
|---|---|---|---|---|---|---|---|---|---|---|---|
| ATOM | 865 | N | THR | A | 152 | 6.563 | -12.265 | -17.816 | 1.00 | 50.08 | N |
| ATOM | 866 | CA | THR | A | 152 | 5.406 | -11.662 | -17.175 | 1.00 | 48.35 | C |
| ATOM | 867 | CB | THR | A | 152 | 4.219 | -11.661 | -18.163 | 1.00 | 49.29 | C |
| ATOM | 868 | OG1 | THR | A | 152 | 2.988 | -11.465 | -17.459 | 1.00 | 49.63 | O |
| ATOM | 869 | CG2 | THR | A | 152 | 4.164 | -12.988 | -18.923 | 1.00 | 50.42 | C |
| ATOM | 870 | C | THR | A | 152 | 4.974 | -12.336 | -15.876 | 1.00 | 47.20 | C |
| ATOM | 871 | O | THR | A | 152 | 4.699 | -13.538 | -15.844 | 1.00 | 49.03 | O |
| ATOM | 872 | N | LEU | A | 153 | 4.897 | -11.557 | -14.803 | 1.00 | 43.79 | N |
| ATOM | 873 | CA | LEU | A | 153 | 4.491 | -12.089 | -13.505 | 1.00 | 40.79 | C |
| ATOM | 874 | CB | LEU | A | 153 | 4.426 | -10.953 | -12.481 | 1.00 | 39.28 | C |
| ATOM | 875 | CG | LEU | A | 153 | 3.955 | -11.281 | -11.065 | 1.00 | 37.97 | C |
| ATOM | 876 | CD1 | LEU | A | 153 | 5.126 | -11.714 | -10.209 | 1.00 | 35.65 | C |
| ATOM | 877 | CD2 | LEU | A | 153 | 3.295 | -10.042 | -10.475 | 1.00 | 36.79 | C |
| ATOM | 878 | C | LEU | A | 153 | 3.131 | -12.801 | -13.579 | 1.00 | 40.44 | C |
| ATOM | 879 | O | LEU | A | 153 | 2.136 | -12.216 | -14.016 | 1.00 | 39.43 | O |
| ATOM | 880 | N | PRO | A | 154 | 3.083 | -14.088 | -13.177 | 1.00 | 40.10 | N |
| ATOM | 881 | CD | PRO | A | 154 | 4.253 | -14.919 | -12.823 | 1.00 | 40.15 | C |
| ATOM | 882 | CA | PRO | A | 154 | 1.857 | -14.900 | -13.177 | 1.00 | 39.95 | C |
| ATOM | 883 | CB | PRO | A | 154 | 2.268 | -16.112 | -12.351 | 1.00 | 40.03 | C |
| ATOM | 884 | CG | PRO | A | 154 | 3.680 | -16.318 | -12.801 | 1.00 | 39.69 | C |
| ATOM | 885 | C | PRO | A | 154 | 0.699 | -14.130 | -12.536 | 1.00 | 39.54 | C |
| ATOM | 886 | O | PRO | A | 154 | 0.869 | -13.538 | -11.471 | 1.00 | 39.54 | O |
| ATOM | 887 | N | VAL | A | 155 | -0.473 | -14.143 | -13.170 | 1.00 | 40.18 | N |
| ATOM | 888 | CA | VAL | A | 155 | -1.616 | -13.405 | -12.640 | 1.00 | 40.64 | C |
| ATOM | 889 | CB | VAL | A | 155 | -2.874 | -13.520 | -13.558 | 1.00 | 39.61 | C |
| ATOM | 890 | CG1 | VAL | A | 155 | -2.569 | -12.962 | -14.932 | 1.00 | 39.26 | C |
| ATOM | 891 | CG2 | VAL | A | 155 | -3.332 | -14.960 | -13.655 | 1.00 | 39.85 | C |
| ATOM | 892 | C | VAL | A | 155 | -2.002 | -13.799 | -11.224 | 1.00 | 40.79 | C |
| ATOM | 893 | O | VAL | A | 155 | -2.563 | -12.985 | -10.489 | 1.00 | 42.48 | O |
| ATOM | 894 | N | ILE | A | 156 | -1.704 | -15.029 | -10.819 | 1.00 | 41.26 | N |
| ATOM | 895 | CA | ILE | A | 156 | -2.064 | -15.442 | -9.466 | 1.00 | 40.77 | C |
| ATOM | 896 | CB | ILE | A | 156 | -1.734 | -16.942 | -9.204 | 1.00 | 40.59 | C |
| ATOM | 897 | CG2 | ILE | A | 156 | -0.230 | -17.166 | -9.205 | 1.00 | 40.53 | C |
| ATOM | 898 | CG1 | ILE | A | 156 | -2.321 | -17.378 | -7.854 | 1.00 | 40.30 | C |
| ATOM | 899 | CD1 | ILE | A | 156 | -3.831 | -17.193 | -7.742 | 1.00 | 39.16 | C |
| ATOM | 900 | C | ILE | A | 156 | -1.344 | -14.561 | -8.442 | 1.00 | 40.36 | C |
| ATOM | 901 | O | ILE | A | 156 | -1.898 | -14.252 | -7.391 | 1.00 | 40.94 | O |
| ATOM | 902 | N | TYR | A | 157 | -0.112 | -14.155 | -8.748 | 1.00 | 39.96 | N |
| ATOM | 903 | CA | TYR | A | 157 | 0.630 | -13.284 | -7.843 | 1.00 | 39.99 | C |
| ATOM | 904 | CB | TYR | A | 157 | 2.099 | -13.210 | -8.253 | 1.00 | 40.21 | C |
| ATOM | 905 | CG | TYR | A | 157 | 2.863 | -14.467 | -7.916 | 1.00 | 41.76 | C |
| ATOM | 906 | CD1 | TYR | A | 157 | 3.727 | -15.047 | -8.836 | 1.00 | 43.16 | C |
| ATOM | 907 | CE1 | TYR | A | 157 | 4.443 | -16.200 | -8.528 | 1.00 | 44.08 | C |
| ATOM | 908 | CD2 | TYR | A | 157 | 2.731 | -15.068 | -6.675 | 1.00 | 42.41 | C |
| ATOM | 909 | CE2 | TYR | A | 157 | 3.440 | -16.220 | -6.355 | 1.00 | 44.14 | C |
| ATOM | 910 | CZ | TYR | A | 157 | 4.300 | -16.779 | -7.288 | 1.00 | 45.27 | C |
| ATOM | 911 | OH | TYR | A | 157 | 5.032 | -17.906 | -6.975 | 1.00 | 45.31 | O |
| ATOM | 912 | C | TYR | A | 157 | -0.019 | -11.910 | -7.919 | 1.00 | 39.78 | C |
| ATOM | 913 | O | TYR | A | 157 | -0.217 | -11.244 | -6.901 | 1.00 | 38.90 | O |
| ATOM | 914 | N | VAL | A | 158 | -0.364 | -11.509 | -9.142 | 1.00 | 39.02 | N |
| ATOM | 915 | CA | VAL | A | 158 | -1.021 | -10.237 | -9.387 | 1.00 | 37.59 | C |
| ATOM | 916 | CB | VAL | A | 158 | -1.334 | -10.041 | -10.892 | 1.00 | 38.19 | C |
| ATOM | 917 | CG1 | VAL | A | 158 | -2.198 | -8.805 | -11.087 | 1.00 | 38.00 | C |
| ATOM | 918 | CG2 | VAL | A | 158 | -0.030 | -9.892 | -11.687 | 1.00 | 37.77 | C |
| ATOM | 919 | C | VAL | A | 158 | -2.324 | -10.185 | -8.602 | 1.00 | 37.34 | C |
| ATOM | 920 | O | VAL | A | 158 | -2.610 | -9.189 | -7.936 | 1.00 | 37.82 | O |
| ATOM | 921 | N | LYS | A | 159 | -3.115 | -11.253 | -8.672 | 1.00 | 36.92 | N |
| ATOM | 922 | CA | LYS | A | 159 | -4.389 | -11.287 | -7.946 | 1.00 | 38.52 | C |
| ATOM | 923 | CB | LYS | A | 159 | -5.161 | -12.575 | -8.241 | 1.00 | 38.55 | C |
| ATOM | 924 | CG | LYS | A | 159 | -5.674 | -12.700 | -9.656 | 1.00 | 39.52 | C |
| ATOM | 925 | CD | LYS | A | 159 | -6.507 | -13.950 | -9.778 | 1.00 | 40.89 | C |
| ATOM | 926 | CE | LYS | A | 159 | -6.755 | -14.328 | -11.224 | 1.00 | 42.11 | C |
| ATOM | 927 | NZ | LYS | A | 159 | -7.636 | -15.530 | -11.284 | 1.00 | 42.40 | N |
| ATOM | 928 | C | LYS | A | 159 | -4.175 | -11.189 | -6.443 | 1.00 | 39.01 | C |
| ATOM | 929 | O | LYS | A | 159 | -4.871 | -10.447 | -5.745 | 1.00 | 39.04 | O |
| ATOM | 930 | N | LEU | A | 160 | -3.216 | -11.970 | -5.953 | 1.00 | 39.57 | N |
| ATOM | 931 | CA | LEU | A | 160 | -2.875 | -12.009 | -4.539 | 1.00 | 38.26 | C |

| | | | | | | | | | | | |
|---|---|---|---|---|---|---|---|---|---|---|---|
| ATOM | 932 | CB | LEU | A | 160 | -1.781 | -13.049 | -4.307 | 1.00 | 38.48 | C |
| ATOM | 933 | CG | LEU | A | 160 | -2.221 | -14.515 | -4.371 | 1.00 | 39.58 | C |
| ATOM | 934 | CD1 | LEU | A | 160 | -1.031 | -15.404 | -4.707 | 1.00 | 36.72 | C |
| ATOM | 935 | CD2 | LEU | A | 160 | -2.841 | -14.904 | -3.027 | 1.00 | 38.57 | C |
| ATOM | 936 | C | LEU | A | 160 | -2.403 | -10.660 | -4.024 | 1.00 | 38.36 | C |
| ATOM | 937 | O | LEU | A | 160 | -2.917 | -10.153 | -3.030 | 1.00 | 38.45 | O |
| ATOM | 938 | N | TYR | A | 161 | -1.418 | -10.084 | -4.706 | 1.00 | 36.84 | N |
| ATOM | 939 | CA | TYR | A | 161 | -0.867 | -8.807 | -4.301 | 1.00 | 36.33 | C |
| ATOM | 940 | CB | TYR | A | 161 | 0.321 | -8.430 | -5.186 | 1.00 | 37.60 | C |
| ATOM | 941 | CG | TYR | A | 161 | 1.469 | -9.401 | -5.091 | 1.00 | 38.66 | C |
| ATOM | 942 | CD1 | TYR | A | 161 | 1.665 | -10.159 | -3.943 | 1.00 | 38.60 | C |
| ATOM | 943 | CE1 | TYR | A | 161 | 2.721 | -11.055 | -3.843 | 1.00 | 40.15 | C |
| ATOM | 944 | CD2 | TYR | A | 161 | 2.363 | -9.558 | -6.141 | 1.00 | 38.78 | C |
| ATOM | 945 | CE2 | TYR | A | 161 | 3.426 | -10.451 | -6.050 | 1.00 | 39.26 | C |
| ATOM | 946 | CZ | TYR | A | 161 | 3.599 | -11.194 | -4.901 | 1.00 | 40.58 | C |
| ATOM | 947 | OH | TYR | A | 161 | 4.652 | -12.081 | -4.801 | 1.00 | 43.96 | O |
| ATOM | 948 | C | TYR | A | 161 | -1.894 | -7.701 | -4.341 | 1.00 | 36.29 | C |
| ATOM | 949 | O | TYR | A | 161 | -2.052 | -6.955 | -3.372 | 1.00 | 35.64 | O |
| ATOM | 950 | N | MET | A | 162 | -2.596 | -7.585 | -5.460 | 1.00 | 36.04 | N |
| ATOM | 951 | CA | MET | A | 162 | -3.602 | -6.537 | -5.591 | 1.00 | 35.73 | C |
| ATOM | 952 | CB | MET | A | 162 | -4.180 | -6.523 | -7.000 | 1.00 | 34.65 | C |
| ATOM | 953 | CG | MET | A | 162 | -3.168 | -6.145 | -8.054 | 1.00 | 35.72 | C |
| ATOM | 954 | SD | MET | A | 162 | -2.395 | -4.539 | -7.720 | 1.00 | 35.74 | S |
| ATOM | 955 | CE | MET | A | 162 | -3.815 | -3.518 | -7.620 | 1.00 | 35.44 | C |
| ATOM | 956 | C | MET | A | 162 | -4.719 | -6.698 | -4.575 | 1.00 | 35.95 | C |
| ATOM | 957 | O | MET | A | 162 | -5.186 | -5.714 | -3.999 | 1.00 | 36.87 | O |
| ATOM | 958 | N | TYR | A | 163 | -5.143 | -7.936 | -4.342 | 1.00 | 34.81 | N |
| ATOM | 959 | CA | TYR | A | 163 | -6.214 | -8.172 | -3.392 | 1.00 | 35.01 | C |
| ATOM | 960 | CB | TYR | A | 163 | -6.569 | -9.650 | -3.313 | 1.00 | 35.57 | C |
| ATOM | 961 | CG | TYR | A | 163 | -7.614 | -9.940 | -2.260 | 1.00 | 35.19 | C |
| ATOM | 962 | CD1 | TYR | A | 163 | -8.964 | -9.689 | -2.508 | 1.00 | 34.90 | C |
| ATOM | 963 | CE1 | TYR | A | 163 | -9.933 | -9.943 | -1.542 | 1.00 | 34.20 | C |
| ATOM | 964 | CD2 | TYR | A | 163 | -7.254 | -10.449 | -1.018 | 1.00 | 33.71 | C |
| ATOM | 965 | CE2 | TYR | A | 163 | -8.210 | -10.706 | -0.044 | 1.00 | 35.07 | C |
| ATOM | 966 | CZ | TYR | A | 163 | -9.548 | -10.453 | -0.313 | 1.00 | 35.35 | C |
| ATOM | 967 | OH | TYR | A | 163 | -10.503 | -10.720 | 0.643 | 1.00 | 37.60 | O |
| ATOM | 968 | C | TYR | A | 163 | -5.825 | -7.709 | -2.009 | 1.00 | 35.47 | C |
| ATOM | 969 | O | TYR | A | 163 | -6.654 | -7.171 | -1.270 | 1.00 | 36.06 | O |
| ATOM | 970 | N | GLN | A | 164 | -4.564 | -7.936 | -1.651 | 1.00 | 35.10 | N |
| ATOM | 971 | CA | GLN | A | 164 | -4.071 | -7.556 | -0.335 | 1.00 | 33.06 | C |
| ATOM | 972 | CB | GLN | A | 164 | -2.760 | -8.288 | -0.033 | 1.00 | 33.04 | C |
| ATOM | 973 | CG | GLN | A | 164 | -2.923 | -9.792 | 0.107 | 1.00 | 33.49 | C |
| ATOM | 974 | CD | GLN | A | 164 | -1.660 | -10.473 | 0.612 | 1.00 | 36.15 | C |
| ATOM | 975 | OE1 | GLN | A | 164 | -1.313 | -10.382 | 1.797 | 1.00 | 35.65 | O |
| ATOM | 976 | NE2 | GLN | A | 164 | -0.959 | -11.153 | -0.290 | 1.00 | 34.16 | N |
| ATOM | 977 | C | GLN | A | 164 | -3.897 | -6.051 | -0.205 | 1.00 | 31.59 | C |
| ATOM | 978 | O | GLN | A | 164 | -4.192 | -5.483 | 0.843 | 1.00 | 31.84 | O |
| ATOM | 979 | N | LEU | A | 165 | -3.428 | -5.400 | -1.265 | 1.00 | 30.09 | N |
| ATOM | 980 | CA | LEU | A | 165 | -3.261 | -3.954 | -1.220 | 1.00 | 28.36 | C |
| ATOM | 981 | CB | LEU | A | 165 | -2.587 | -3.435 | -2.489 | 1.00 | 26.50 | C |
| ATOM | 982 | CG | LEU | A | 165 | -2.793 | -1.956 | -2.832 | 1.00 | 24.60 | C |
| ATOM | 983 | CD1 | LEU | A | 165 | -2.306 | -1.049 | -1.718 | 1.00 | 23.95 | C |
| ATOM | 984 | CD2 | LEU | A | 165 | -2.075 | -1.660 | -4.122 | 1.00 | 22.60 | C |
| ATOM | 985 | C | LEU | A | 165 | -4.624 | -3.299 | -1.068 | 1.00 | 28.89 | C |
| ATOM | 986 | O | LEU | A | 165 | -4.813 | -2.428 | -0.217 | 1.00 | 29.03 | O |
| ATOM | 987 | N | PHE | A | 166 | -5.573 | -3.722 | -1.896 | 1.00 | 29.25 | N |
| ATOM | 988 | CA | PHE | A | 166 | -6.919 | -3.156 | -1.842 | 1.00 | 30.67 | C |
| ATOM | 989 | CB | PHE | A | 166 | -7.833 | -3.824 | -2.882 | 1.00 | 28.68 | C |
| ATOM | 990 | CG | PHE | A | 166 | -7.738 | -3.201 | -4.251 | 1.00 | 27.88 | C |
| ATOM | 991 | CD1 | PHE | A | 166 | -7.968 | -1.833 | -4.417 | 1.00 | 26.07 | C |
| ATOM | 992 | CD2 | PHE | A | 166 | -7.413 | -3.962 | -5.366 | 1.00 | 24.46 | C |
| ATOM | 993 | CE1 | PHE | A | 166 | -7.875 | -1.235 | -5.667 | 1.00 | 24.30 | C |
| ATOM | 994 | CE2 | PHE | A | 166 | -7.318 | -3.367 | -6.623 | 1.00 | 25.24 | C |
| ATOM | 995 | CZ | PHE | A | 166 | -7.551 | -1.998 | -6.772 | 1.00 | 25.96 | C |
| ATOM | 996 | C | PHE | A | 166 | -7.531 | -3.260 | -0.447 | 1.00 | 31.68 | C |
| ATOM | 997 | O | PHE | A | 166 | -8.172 | -2.327 | 0.033 | 1.00 | 31.65 | O |
| ATOM | 998 | N | ARG | A | 167 | -7.314 | -4.390 | 0.209 | 1.00 | 32.35 | N |

| | | | | | | | | | | | |
|---|---|---|---|---|---|---|---|---|---|---|---|
| ATOM | 999 | CA | ARG | A | 167 | -7.855 | -4.587 | 1.534 | 1.00 | 33.04 | C |
| ATOM | 1000 | CB | ARG | A | 167 | -7.590 | -6.015 | 1.982 | 1.00 | 32.14 | C |
| ATOM | 1001 | CG | ARG | A | 167 | -8.605 | -6.515 | 2.960 | 1.00 | 32.68 | C |
| ATOM | 1002 | CD | ARG | A | 167 | -8.212 | -7.850 | 3.505 | 1.00 | 33.09 | C |
| ATOM | 1003 | NE | ARG | A | 167 | -8.841 | -8.058 | 4.795 | 1.00 | 34.29 | N |
| ATOM | 1004 | CZ | ARG | A | 167 | -8.467 | -8.988 | 5.661 | 1.00 | 35.47 | C |
| ATOM | 1005 | NH1 | ARG | A | 167 | -7.464 | -9.802 | 5.365 | 1.00 | 35.82 | N |
| ATOM | 1006 | NH2 | ARG | A | 167 | -9.086 | -9.091 | 6.830 | 1.00 | 36.15 | N |
| ATOM | 1007 | C | ARG | A | 167 | -7.263 | -3.596 | 2.542 | 1.00 | 34.56 | C |
| ATOM | 1008 | O | ARG | A | 167 | -7.981 | -3.056 | 3.384 | 1.00 | 35.05 | O |
| ATOM | 1009 | N | SER | A | 168 | -5.956 | -3.356 | 2.449 | 1.00 | 35.08 | N |
| ATOM | 1010 | CA | SER | A | 168 | -5.281 | -2.432 | 3.359 | 1.00 | 35.46 | C |
| ATOM | 1011 | CB | SER | A | 168 | -3.759 | -2.470 | 3.152 | 1.00 | 35.97 | C |
| ATOM | 1012 | OG | SER | A | 168 | -3.338 | -1.604 | 2.109 | 1.00 | 36.24 | O |
| ATOM | 1013 | C | SER | A | 168 | -5.783 | -1.008 | 3.135 | 1.00 | 35.40 | C |
| ATOM | 1014 | O | SER | A | 168 | -5.849 | -0.201 | 4.065 | 1.00 | 36.62 | O |
| ATOM | 1015 | N | LEU | A | 169 | -6.126 | -0.703 | 1.892 | 1.00 | 34.23 | N |
| ATOM | 1016 | CA | LEU | A | 169 | -6.625 | 0.612 | 1.551 | 1.00 | 33.47 | C |
| ATOM | 1017 | CB | LEU | A | 169 | -6.577 | 0.812 | 0.027 | 1.00 | 31.68 | C |
| ATOM | 1018 | CG | LEU | A | 169 | -5.500 | 1.760 | -0.542 | 1.00 | 32.15 | C |
| ATOM | 1019 | CD1 | LEU | A | 169 | -4.281 | 1.852 | 0.367 | 1.00 | 33.68 | C |
| ATOM | 1020 | CD2 | LEU | A | 169 | -5.095 | 1.286 | -1.921 | 1.00 | 31.87 | C |
| ATOM | 1021 | C | LEU | A | 169 | -8.048 | 0.749 | 2.106 | 1.00 | 34.50 | C |
| ATOM | 1022 | O | LEU | A | 169 | -8.445 | 1.820 | 2.579 | 1.00 | 34.76 | O |
| ATOM | 1023 | N | ALA | A | 170 | -8.811 | -0.341 | 2.077 | 1.00 | 33.93 | N |
| ATOM | 1024 | CA | ALA | A | 170 | -10.167 | -0.308 | 2.617 | 1.00 | 33.77 | C |
| ATOM | 1025 | CB | ALA | A | 170 | -10.852 | -1.653 | 2.421 | 1.00 | 30.92 | C |
| ATOM | 1026 | C | ALA | A | 170 | -10.046 | 0.010 | 4.106 | 1.00 | 34.57 | C |
| ATOM | 1027 | O | ALA | A | 170 | -10.681 | 0.932 | 4.623 | 1.00 | 35.14 | O |
| ATOM | 1028 | N | TYR | A | 171 | -9.197 | -0.752 | 4.781 | 1.00 | 36.13 | N |
| ATOM | 1029 | CA | TYR | A | 171 | -8.966 | -0.569 | 6.201 | 1.00 | 38.49 | C |
| ATOM | 1030 | CB | TYR | A | 171 | -7.918 | -1.563 | 6.684 | 1.00 | 41.53 | C |
| ATOM | 1031 | CG | TYR | A | 171 | -7.462 | -1.335 | 8.101 | 1.00 | 43.54 | C |
| ATOM | 1032 | CD1 | TYR | A | 171 | -8.219 | -1.790 | 9.177 | 1.00 | 44.28 | C |
| ATOM | 1033 | CE1 | TYR | A | 171 | -7.811 | -1.572 | 10.485 | 1.00 | 44.52 | C |
| ATOM | 1034 | CD2 | TYR | A | 171 | -6.279 | -0.655 | 8.362 | 1.00 | 43.86 | C |
| ATOM | 1035 | CE2 | TYR | A | 171 | -5.859 | -0.428 | 9.663 | 1.00 | 46.80 | C |
| ATOM | 1036 | CZ | TYR | A | 171 | -6.634 | -0.893 | 10.724 | 1.00 | 47.02 | C |
| ATOM | 1037 | OH | TYR | A | 171 | -6.228 | -0.663 | 12.020 | 1.00 | 48.43 | O |
| ATOM | 1038 | C | TYR | A | 171 | -8.524 | 0.852 | 6.575 | 1.00 | 39.02 | C |
| ATOM | 1039 | O | TYR | A | 171 | -9.050 | 1.433 | 7.515 | 1.00 | 38.61 | O |
| ATOM | 1040 | N | ILE | A | 172 | -7.556 | 1.417 | 5.863 | 1.00 | 38.84 | N |
| ATOM | 1041 | CA | ILE | A | 172 | -7.128 | 2.755 | 6.214 | 1.00 | 40.00 | C |
| ATOM | 1042 | CB | ILE | A | 172 | -5.771 | 3.122 | 5.589 | 1.00 | 40.89 | C |
| ATOM | 1043 | CG2 | ILE | A | 172 | -4.647 | 2.459 | 6.377 | 1.00 | 40.72 | C |
| ATOM | 1044 | CG1 | ILE | A | 172 | -5.750 | 2.734 | 4.115 | 1.00 | 41.88 | C |
| ATOM | 1045 | CD1 | ILE | A | 172 | -4.494 | 3.189 | 3.381 | 1.00 | 42.41 | C |
| ATOM | 1046 | C | ILE | A | 172 | -8.144 | 3.820 | 5.834 | 1.00 | 41.46 | C |
| ATOM | 1047 | O | ILE | A | 172 | -8.306 | 4.813 | 6.548 | 1.00 | 42.27 | O |
| ATOM | 1048 | N | HIS | A | 173 | -8.843 | 3.632 | 4.720 | 1.00 | 40.38 | N |
| ATOM | 1049 | CA | HIS | A | 173 | -9.835 | 4.623 | 4.312 | 1.00 | 38.35 | C |
| ATOM | 1050 | CB | HIS | A | 173 | -10.325 | 4.321 | 2.900 | 1.00 | 37.13 | C |
| ATOM | 1051 | CG | HIS | A | 173 | -9.312 | 4.648 | 1.853 | 1.00 | 36.44 | C |
| ATOM | 1052 | CD2 | HIS | A | 173 | -8.088 | 5.219 | 1.953 | 1.00 | 36.35 | C |
| ATOM | 1053 | ND1 | HIS | A | 173 | -9.500 | 4.382 | 0.516 | 1.00 | 36.91 | N |
| ATOM | 1054 | CE1 | HIS | A | 173 | -8.436 | 4.771 | -0.162 | 1.00 | 35.34 | C |
| ATOM | 1055 | NE2 | HIS | A | 173 | -7.566 | 5.282 | 0.688 | 1.00 | 36.10 | N |
| ATOM | 1056 | C | HIS | A | 173 | -10.985 | 4.696 | 5.294 | 1.00 | 38.25 | C |
| ATOM | 1057 | O | HIS | A | 173 | -11.587 | 5.752 | 5.468 | 1.00 | 38.49 | O |
| ATOM | 1058 | N | SER | A | 174 | -11.249 | 3.583 | 5.971 | 1.00 | 39.57 | N |
| ATOM | 1059 | CA | SER | A | 174 | -12.322 | 3.508 | 6.955 | 1.00 | 40.52 | C |
| ATOM | 1060 | CB | SER | A | 174 | -12.407 | 2.088 | 7.504 | 1.00 | 40.03 | C |
| ATOM | 1061 | OG | SER | A | 174 | -11.320 | 1.838 | 8.371 | 1.00 | 41.62 | O |
| ATOM | 1062 | C | SER | A | 174 | -12.109 | 4.502 | 8.104 | 1.00 | 41.07 | C |
| ATOM | 1063 | O | SER | A | 174 | -13.027 | 4.764 | 8.889 | 1.00 | 41.29 | O |
| ATOM | 1064 | N | PHE | A | 175 | -10.895 | 5.047 | 8.193 | 1.00 | 41.98 | N |
| ATOM | 1065 | CA | PHE | A | 175 | -10.540 | 6.019 | 9.229 | 1.00 | 42.87 | C |

| | | | | | | | | | | | |
|---|---|---|---|---|---|---|---|---|---|---|---|
| ATOM | 1066 | CB | PHE | A | 175 | -9.215 | 5.629 | 9.901 | 1.00 | 45.52 | C |
| ATOM | 1067 | CG | PHE | A | 175 | -9.294 | 4.375 | 10.724 | 1.00 | 48.85 | C |
| ATOM | 1068 | CD1 | PHE | A | 175 | -8.154 | 3.622 | 10.980 | 1.00 | 48.54 | C |
| ATOM | 1069 | CD2 | PHE | A | 175 | -10.518 | 3.939 | 11.234 | 1.00 | 50.19 | C |
| ATOM | 1070 | CE1 | PHE | A | 175 | -8.230 | 2.446 | 11.726 | 1.00 | 50.79 | C |
| ATOM | 1071 | CE2 | PHE | A | 175 | -10.603 | 2.762 | 11.984 | 1.00 | 50.53 | C |
| ATOM | 1072 | CZ | PHE | A | 175 | -9.458 | 2.015 | 12.230 | 1.00 | 49.97 | C |
| ATOM | 1073 | C | PHE | A | 175 | -10.403 | 7.424 | 8.649 | 1.00 | 42.24 | C |
| ATOM | 1074 | O | PHE | A | 175 | -10.155 | 8.385 | 9.378 | 1.00 | 43.53 | O |
| ATOM | 1075 | N | GLY | A | 176 | -10.564 | 7.541 | 7.336 | 1.00 | 40.87 | N |
| ATOM | 1076 | CA | GLY | A | 176 | -10.443 | 8.839 | 6.702 | 1.00 | 40.36 | C |
| ATOM | 1077 | C | GLY | A | 176 | -9.012 | 9.116 | 6.279 | 1.00 | 40.14 | C |
| ATOM | 1078 | O | GLY | A | 176 | -8.689 | 10.176 | 5.736 | 1.00 | 41.67 | O |
| ATOM | 1079 | N | ILE | A | 177 | -8.147 | 8.147 | 6.541 | 1.00 | 38.13 | N |
| ATOM | 1080 | CA | ILE | A | 177 | -6.743 | 8.258 | 6.213 | 1.00 | 36.79 | C |
| ATOM | 1081 | CB | ILE | A | 177 | -5.903 | 7.317 | 7.080 | 1.00 | 38.17 | C |
| ATOM | 1082 | CG2 | ILE | A | 177 | -4.426 | 7.637 | 6.908 | 1.00 | 36.28 | C |
| ATOM | 1083 | CG1 | ILE | A | 177 | -6.323 | 7.448 | 8.539 | 1.00 | 37.63 | C |
| ATOM | 1084 | CD1 | ILE | A | 177 | -5.932 | 6.270 | 9.376 | 1.00 | 40.81 | C |
| ATOM | 1085 | C | ILE | A | 177 | -6.485 | 7.888 | 4.764 | 1.00 | 36.12 | C |
| ATOM | 1086 | O | ILE | A | 177 | -6.917 | 6.834 | 4.291 | 1.00 | 35.90 | O |
| ATOM | 1087 | N | CYS | A | 178 | -5.772 | 8.766 | 4.067 | 1.00 | 34.06 | N |
| ATOM | 1088 | CA | CYS | A | 178 | -5.415 | 8.547 | 2.676 | 1.00 | 33.14 | C |
| ATOM | 1089 | CB | CYS | A | 178 | -5.850 | 9.735 | 1.818 | 1.00 | 33.28 | C |
| ATOM | 1090 | SG | CYS | A | 178 | -5.555 | 9.533 | 0.052 | 1.00 | 34.81 | S |
| ATOM | 1091 | C | CYS | A | 178 | -3.900 | 8.404 | 2.634 | 1.00 | 33.71 | C |
| ATOM | 1092 | O | CYS | A | 178 | -3.182 | 9.234 | 3.187 | 1.00 | 34.76 | O |
| ATOM | 1093 | N | HIS | A | 179 | -3.421 | 7.348 | 1.982 | 1.00 | 32.52 | N |
| ATOM | 1094 | CA | HIS | A | 179 | -1.993 | 7.078 | 1.890 | 1.00 | 30.59 | C |
| ATOM | 1095 | CB | HIS | A | 179 | -1.780 | 5.674 | 1.336 | 1.00 | 29.91 | C |
| ATOM | 1096 | CG | HIS | A | 179 | -0.361 | 5.209 | 1.402 | 1.00 | 30.41 | C |
| ATOM | 1097 | CD2 | HIS | A | 179 | 0.246 | 4.312 | 2.216 | 1.00 | 32.11 | C |
| ATOM | 1098 | ND1 | HIS | A | 179 | 0.617 | 5.678 | 0.553 | 1.00 | 29.56 | N |
| ATOM | 1099 | CE1 | HIS | A | 179 | 1.763 | 5.086 | 0.839 | 1.00 | 31.83 | C |
| ATOM | 1100 | NE2 | HIS | A | 179 | 1.565 | 4.253 | 1.842 | 1.00 | 31.05 | N |
| ATOM | 1101 | C | HIS | A | 179 | -1.223 | 8.096 | 1.055 | 1.00 | 30.96 | C |
| ATOM | 1102 | O | HIS | A | 179 | -0.090 | 8.460 | 1.387 | 1.00 | 31.84 | O |
| ATOM | 1103 | N | ARG | A | 180 | -1.838 | 8.547 | -0.030 | 1.00 | 30.32 | N |
| ATOM | 1104 | CA | ARG | A | 180 | -1.234 | 9.530 | -0.920 | 1.00 | 29.78 | C |
| ATOM | 1105 | CB | ARG | A | 180 | -1.144 | 10.876 | -0.211 | 1.00 | 30.61 | C |
| ATOM | 1106 | CG | ARG | A | 180 | -2.477 | 11.364 | 0.289 | 1.00 | 29.27 | C |
| ATOM | 1107 | CD | ARG | A | 180 | -2.277 | 12.395 | 1.356 | 1.00 | 31.78 | C |
| ATOM | 1108 | NE | ARG | A | 180 | -2.002 | 13.716 | 0.827 | 1.00 | 32.13 | N |
| ATOM | 1109 | CZ | ARG | A | 180 | -1.451 | 14.685 | 1.546 | 1.00 | 33.48 | C |
| ATOM | 1110 | NH1 | ARG | A | 180 | -1.114 | 14.457 | 2.805 | 1.00 | 33.76 | N |
| ATOM | 1111 | NH2 | ARG | A | 180 | -1.259 | 15.886 | 1.020 | 1.00 | 36.08 | N |
| ATOM | 1112 | C | ARG | A | 180 | 0.138 | 9.157 | -1.487 | 1.00 | 29.82 | C |
| ATOM | 1113 | O | ARG | A | 180 | 0.825 | 10.013 | -2.049 | 1.00 | 29.25 | O |
| ATOM | 1114 | N | ASP | A | 181 | 0.547 | 7.899 | -1.333 | 1.00 | 30.10 | N |
| ATOM | 1115 | CA | ASP | A | 181 | 1.825 | 7.467 | -1.893 | 1.00 | 30.86 | C |
| ATOM | 1116 | CB | ASP | A | 181 | 2.980 | 7.781 | -0.950 | 1.00 | 32.43 | C |
| ATOM | 1117 | CG | ASP | A | 181 | 4.330 | 7.704 | -1.651 | 1.00 | 33.40 | C |
| ATOM | 1118 | OD1 | ASP | A | 181 | 4.357 | 7.782 | -2.895 | 1.00 | 32.61 | O |
| ATOM | 1119 | OD2 | ASP | A | 181 | 5.363 | 7.571 | -0.962 | 1.00 | 37.08 | O |
| ATOM | 1120 | C | ASP | A | 181 | 1.870 | 5.990 | -2.298 | 1.00 | 31.36 | C |
| ATOM | 1121 | O | ASP | A | 181 | 2.886 | 5.311 | -2.127 | 1.00 | 29.91 | O |
| ATOM | 1122 | N | ILE | A | 182 | 0.749 | 5.508 | -2.843 | 1.00 | 31.31 | N |
| ATOM | 1123 | CA | ILE | A | 182 | 0.634 | 4.133 | -3.304 | 1.00 | 29.91 | C |
| ATOM | 1124 | CB | ILE | A | 182 | -0.824 | 3.771 | -3.658 | 1.00 | 27.35 | C |
| ATOM | 1125 | CG2 | ILE | A | 182 | -0.895 | 2.371 | -4.265 | 1.00 | 24.41 | C |
| ATOM | 1126 | CG1 | ILE | A | 182 | -1.684 | 3.843 | -2.396 | 1.00 | 25.83 | C |
| ATOM | 1127 | CD1 | ILE | A | 182 | -1.154 | 3.003 | -1.250 | 1.00 | 25.80 | C |
| ATOM | 1128 | C | ILE | A | 182 | 1.513 | 3.942 | -4.531 | 1.00 | 30.16 | C |
| ATOM | 1129 | O | ILE | A | 182 | 1.366 | 4.640 | -5.536 | 1.00 | 28.99 | O |
| ATOM | 1130 | N | LYS | A | 183 | 2.448 | 3.003 | -4.412 | 1.00 | 30.02 | N |
| ATOM | 1131 | CA | LYS | A | 183 | 3.379 | 2.669 | -5.481 | 1.00 | 30.49 | C |
| ATOM | 1132 | CB | LYS | A | 183 | 4.407 | 3.792 | -5.658 | 1.00 | 31.00 | C |

| | | | | | | | | | | | |
|---|---|---|---|---|---|---|---|---|---|---|---|
| ATOM | 1133 | CG | LYS | A | 183 | 5.290 | 4.034 | -4.452 | 1.00 | 32.97 | C |
| ATOM | 1134 | CD | LYS | A | 183 | 6.320 | 5.100 | -4.758 | 1.00 | 35.22 | C |
| ATOM | 1135 | CE | LYS | A | 183 | 7.151 | 5.419 | -3.535 | 1.00 | 37.22 | C |
| ATOM | 1136 | NZ | LYS | A | 183 | 8.231 | 6.387 | -3.845 | 1.00 | 40.88 | N |
| ATOM | 1137 | C | LYS | A | 183 | 4.093 | 1.342 | -5.165 | 1.00 | 30.08 | C |
| ATOM | 1138 | O | LYS | A | 183 | 4.263 | 0.984 | -4.004 | 1.00 | 30.49 | O |
| ATOM | 1139 | N | PRO | A | 184 | 4.520 | 0.606 | -6.203 | 1.00 | 29.30 | N |
| ATOM | 1140 | CD | PRO | A | 184 | 4.498 | 1.070 | -7.605 | 1.00 | 28.98 | C |
| ATOM | 1141 | CA | PRO | A | 184 | 5.216 | -0.683 | -6.094 | 1.00 | 28.43 | C |
| ATOM | 1142 | CB | PRO | A | 184 | 5.752 | -0.904 | -7.507 | 1.00 | 30.01 | C |
| ATOM | 1143 | CG | PRO | A | 184 | 4.738 | -0.200 | -8.366 | 1.00 | 30.25 | C |
| ATOM | 1144 | C | PRO | A | 184 | 6.329 | -0.741 | -5.055 | 1.00 | 28.20 | C |
| ATOM | 1145 | O | PRO | A | 184 | 6.492 | -1.756 | -4.388 | 1.00 | 27.25 | O |
| ATOM | 1146 | N | GLN | A | 185 | 7.090 | 0.343 | -4.923 | 1.00 | 29.81 | N |
| ATOM | 1147 | CA | GLN | A | 185 | 8.199 | 0.390 | -3.970 | 1.00 | 30.32 | C |
| ATOM | 1148 | CB | GLN | A | 185 | 9.078 | 1.625 | -4.213 | 1.00 | 31.28 | C |
| ATOM | 1149 | CG | GLN | A | 185 | 9.704 | 1.702 | -5.602 | 1.00 | 33.65 | C |
| ATOM | 1150 | CD | GLN | A | 185 | 8.789 | 2.361 | -6.622 | 1.00 | 35.78 | C |
| ATOM | 1151 | OE1 | GLN | A | 185 | 7.570 | 2.199 | -6.586 | 1.00 | 33.89 | O |
| ATOM | 1152 | NE2 | GLN | A | 185 | 9.382 | 3.101 | -7.547 | 1.00 | 39.22 | N |
| ATOM | 1153 | C | GLN | A | 185 | 7.743 | 0.379 | -2.516 | 1.00 | 29.59 | C |
| ATOM | 1154 | O | GLN | A | 185 | 8.540 | 0.110 | -1.616 | 1.00 | 30.45 | O |
| ATOM | 1155 | N | ASN | A | 186 | 6.467 | 0.677 | -2.284 | 1.00 | 28.94 | N |
| ATOM | 1156 | CA | ASN | A | 186 | 5.933 | 0.693 | -0.931 | 1.00 | 27.85 | C |
| ATOM | 1157 | CB | ASN | A | 186 | 5.156 | 1.984 | -0.660 | 1.00 | 26.33 | C |
| ATOM | 1158 | CG | ASN | A | 186 | 6.051 | 3.208 | -0.630 | 1.00 | 26.71 | C |
| ATOM | 1159 | OD1 | ASN | A | 186 | 7.194 | 3.153 | -0.179 | 1.00 | 27.50 | O |
| ATOM | 1160 | ND2 | ASN | A | 186 | 5.526 | 4.325 | -1.101 | 1.00 | 27.38 | N |
| ATOM | 1161 | C | ASN | A | 186 | 5.048 | -0.508 | -0.661 | 1.00 | 29.00 | C |
| ATOM | 1162 | O | ASN | A | 186 | 4.243 | -0.492 | 0.264 | 1.00 | 28.54 | O |
| ATOM | 1163 | N | LEU | A | 187 | 5.195 | -1.553 | -1.467 | 1.00 | 29.41 | N |
| ATOM | 1164 | CA | LEU | A | 187 | 4.412 | -2.765 | -1.266 | 1.00 | 31.28 | C |
| ATOM | 1165 | CB | LEU | A | 187 | 3.627 | -3.126 | -2.535 | 1.00 | 30.32 | C |
| ATOM | 1166 | CG | LEU | A | 187 | 2.549 | -2.146 | -3.032 | 1.00 | 28.96 | C |
| ATOM | 1167 | CD1 | LEU | A | 187 | 1.990 | -2.691 | -4.331 | 1.00 | 29.97 | C |
| ATOM | 1168 | CD2 | LEU | A | 187 | 1.438 | -1.953 | -2.012 | 1.00 | 27.12 | C |
| ATOM | 1169 | C | LEU | A | 187 | 5.394 | -3.876 | -0.901 | 1.00 | 32.57 | C |
| ATOM | 1170 | O | LEU | A | 187 | 6.041 | -4.463 | -1.767 | 1.00 | 30.34 | O |
| ATOM | 1171 | N | LEU | A | 188 | 5.512 | -4.137 | 0.395 | 1.00 | 35.35 | N |
| ATOM | 1172 | CA | LEU | A | 188 | 6.418 | -5.159 | 0.893 | 1.00 | 39.19 | C |
| ATOM | 1173 | CB | LEU | A | 188 | 6.879 | -4.801 | 2.302 | 1.00 | 38.57 | C |
| ATOM | 1174 | CG | LEU | A | 188 | 7.217 | -3.330 | 2.522 | 1.00 | 38.90 | C |
| ATOM | 1175 | CD1 | LEU | A | 188 | 7.441 | -3.111 | 3.990 | 1.00 | 40.18 | C |
| ATOM | 1176 | CD2 | LEU | A | 188 | 8.432 | -2.923 | 1.700 | 1.00 | 37.71 | C |
| ATOM | 1177 | C | LEU | A | 188 | 5.707 | -6.500 | 0.924 | 1.00 | 41.46 | C |
| ATOM | 1178 | O | LEU | A | 188 | 4.533 | -6.582 | 1.290 | 1.00 | 42.51 | O |
| ATOM | 1179 | N | LEU | A | 189 | 6.427 | -7.553 | 0.559 | 1.00 | 42.65 | N |
| ATOM | 1180 | CA | LEU | A | 189 | 5.847 | -8.881 | 0.534 | 1.00 | 46.06 | C |
| ATOM | 1181 | CB | LEU | A | 189 | 5.352 | -9.220 | -0.882 | 1.00 | 47.01 | C |
| ATOM | 1182 | CG | LEU | A | 189 | 5.895 | -8.343 | -2.008 | 1.00 | 47.19 | C |
| ATOM | 1183 | CD1 | LEU | A | 189 | 7.394 | -8.166 | -1.811 | 1.00 | 49.46 | C |
| ATOM | 1184 | CD2 | LEU | A | 189 | 5.606 | -8.966 | -3.368 | 1.00 | 47.00 | C |
| ATOM | 1185 | C | LEU | A | 189 | 6.847 | -9.921 | 0.973 | 1.00 | 46.63 | C |
| ATOM | 1186 | O | LEU | A | 189 | 8.045 | -9.778 | 0.759 | 1.00 | 46.97 | O |
| ATOM | 1187 | N | ASP | A | 190 | 6.342 | -10.955 | 1.625 | 1.00 | 47.41 | N |
| ATOM | 1188 | CA | ASP | A | 190 | 7.180 | -12.050 | 2.061 | 1.00 | 49.42 | C |
| ATOM | 1189 | CB | ASP | A | 190 | 6.652 | -12.653 | 3.361 | 1.00 | 50.80 | C |
| ATOM | 1190 | CG | ASP | A | 190 | 7.528 | -13.785 | 3.872 | 1.00 | 51.73 | C |
| ATOM | 1191 | OD1 | ASP | A | 190 | 7.718 | -14.768 | 3.112 | 1.00 | 51.87 | O |
| ATOM | 1192 | OD2 | ASP | A | 190 | 8.016 | -13.689 | 5.022 | 1.00 | 52.74 | O |
| ATOM | 1193 | C | ASP | A | 190 | 7.092 | -13.067 | 0.937 | 1.00 | 49.47 | C |
| ATOM | 1194 | O | ASP | A | 190 | 6.017 | -13.583 | 0.641 | 1.00 | 49.03 | O |
| ATOM | 1195 | N | PRO | A | 191 | 8.223 | -13.348 | 0.283 | 1.00 | 49.70 | N |
| ATOM | 1196 | CD | PRO | A | 191 | 9.580 | -12.943 | 0.691 | 1.00 | 50.64 | C |
| ATOM | 1197 | CA | PRO | A | 191 | 8.279 | -14.304 | -0.827 | 1.00 | 50.28 | C |
| ATOM | 1198 | CB | PRO | A | 191 | 9.762 | -14.319 | -1.191 | 1.00 | 50.88 | C |
| ATOM | 1199 | CG | PRO | A | 191 | 10.427 | -14.076 | 0.138 | 1.00 | 51.55 | C |

| | | | | | | | | | | | |
|---|---|---|---|---|---|---|---|---|---|---|---|
| ATOM | 1200 | C | PRO | A | 191 | 7.727 | -15.709 | -0.596 | 1.00 | 49.47 | C |
| ATOM | 1201 | O | PRO | A | 191 | 7.123 | -16.276 | -1.501 | 1.00 | 50.39 | O |
| ATOM | 1202 | N | ASP | A | 192 | 7.927 | -16.285 | 0.585 | 1.00 | 47.75 | N |
| ATOM | 1203 | CA | ASP | A | 192 | 7.420 | -17.633 | 0.812 | 1.00 | 46.07 | C |
| ATOM | 1204 | CB | ASP | A | 192 | 8.217 | -18.357 | 1.906 | 1.00 | 47.02 | C |
| ATOM | 1205 | CG | ASP | A | 192 | 9.733 | -18.187 | 1.760 | 1.00 | 48.63 | C |
| ATOM | 1206 | OD1 | ASP | A | 192 | 10.272 | -18.169 | 0.632 | 1.00 | 48.08 | O |
| ATOM | 1207 | OD2 | ASP | A | 192 | 10.403 | -18.088 | 2.803 | 1.00 | 51.80 | O |
| ATOM | 1208 | C | ASP | A | 192 | 5.944 | -17.664 | 1.178 | 1.00 | 45.05 | C |
| ATOM | 1209 | O | ASP | A | 192 | 5.231 | -18.576 | 0.767 | 1.00 | 46.63 | O |
| ATOM | 1210 | N | THR | A | 193 | 5.473 | -16.675 | 1.936 | 1.00 | 42.71 | N |
| ATOM | 1211 | CA | THR | A | 193 | 4.066 | -16.643 | 2.348 | 1.00 | 40.59 | C |
| ATOM | 1212 | CB | THR | A | 193 | 3.898 | -16.060 | 3.775 | 1.00 | 40.05 | C |
| ATOM | 1213 | OG1 | THR | A | 193 | 4.573 | -14.800 | 3.870 | 1.00 | 39.50 | O |
| ATOM | 1214 | CG2 | THR | A | 193 | 4.461 | -17.019 | 4.813 | 1.00 | 39.24 | C |
| ATOM | 1215 | C | THR | A | 193 | 3.165 | -15.860 | 1.400 | 1.00 | 39.44 | C |
| ATOM | 1216 | O | THR | A | 193 | 1.954 | -16.079 | 1.356 | 1.00 | 38.60 | O |
| ATOM | 1217 | N | ALA | A | 194 | 3.766 | -14.950 | 0.645 | 1.00 | 39.08 | N |
| ATOM | 1218 | CA | ALA | A | 194 | 3.037 | -14.132 | -0.319 | 1.00 | 39.27 | C |
| ATOM | 1219 | CB | ALA | A | 194 | 2.192 | -15.021 | -1.246 | 1.00 | 38.72 | C |
| ATOM | 1220 | C | ALA | A | 194 | 2.154 | -13.054 | 0.307 | 1.00 | 39.21 | C |
| ATOM | 1221 | O | ALA | A | 194 | 1.244 | -12.537 | -0.353 | 1.00 | 39.50 | O |
| ATOM | 1222 | N | VAL | A | 195 | 2.400 | -12.704 | 1.567 | 1.00 | 37.15 | N |
| ATOM | 1223 | CA | VAL | A | 195 | 1.586 | -11.661 | 2.170 | 1.00 | 35.67 | C |
| ATOM | 1224 | CB | VAL | A | 195 | 1.460 | -11.815 | 3.713 | 1.00 | 37.17 | C |
| ATOM | 1225 | CG1 | VAL | A | 195 | 1.691 | -13.260 | 4.115 | 1.00 | 34.84 | C |
| ATOM | 1226 | CG2 | VAL | A | 195 | 2.401 | -10.869 | 4.429 | 1.00 | 38.47 | C |
| ATOM | 1227 | C | VAL | A | 195 | 2.224 | -10.325 | 1.829 | 1.00 | 33.78 | C |
| ATOM | 1228 | O | VAL | A | 195 | 3.444 | -10.200 | 1.777 | 1.00 | 34.23 | O |
| ATOM | 1229 | N | LEU | A | 196 | 1.396 | -9.334 | 1.563 | 1.00 | 32.46 | N |
| ATOM | 1230 | CA | LEU | A | 196 | 1.894 | -8.019 | 1.224 | 1.00 | 31.84 | C |
| ATOM | 1231 | CB | LEU | A | 196 | 1.233 | -7.560 | -0.084 | 1.00 | 29.72 | C |
| ATOM | 1232 | CG | LEU | A | 196 | 1.246 | -6.099 | -0.526 | 1.00 | 27.20 | C |
| ATOM | 1233 | CD1 | LEU | A | 196 | 1.141 | -6.028 | -2.036 | 1.00 | 25.69 | C |
| ATOM | 1234 | CD2 | LEU | A | 196 | 0.122 | -5.352 | 0.156 | 1.00 | 22.70 | C |
| ATOM | 1235 | C | LEU | A | 196 | 1.564 | -7.073 | 2.375 | 1.00 | 31.74 | C |
| ATOM | 1236 | O | LEU | A | 196 | 0.689 | -7.363 | 3.198 | 1.00 | 31.83 | O |
| ATOM | 1237 | N | LYS | A | 197 | 2.274 | -5.952 | 2.442 | 1.00 | 31.33 | N |
| ATOM | 1238 | CA | LYS | A | 197 | 2.029 | -4.953 | 3.479 | 1.00 | 30.54 | C |
| ATOM | 1239 | CB | LYS | A | 197 | 2.944 | -5.156 | 4.690 | 1.00 | 32.33 | C |
| ATOM | 1240 | CG | LYS | A | 197 | 2.759 | -6.481 | 5.394 | 1.00 | 34.61 | C |
| ATOM | 1241 | CD | LYS | A | 197 | 3.623 | -6.564 | 6.623 | 1.00 | 36.09 | C |
| ATOM | 1242 | CE | LYS | A | 197 | 3.636 | -7.981 | 7.153 | 1.00 | 36.80 | C |
| ATOM | 1243 | NZ | LYS | A | 197 | 2.248 | -8.494 | 7.318 | 1.00 | 39.55 | N |
| ATOM | 1244 | C | LYS | A | 197 | 2.281 | -3.580 | 2.907 | 1.00 | 29.61 | C |
| ATOM | 1245 | O | LYS | A | 197 | 3.298 | -3.345 | 2.266 | 1.00 | 28.04 | O |
| ATOM | 1246 | N | LEU | A | 198 | 1.331 | -2.680 | 3.130 | 1.00 | 29.95 | N |
| ATOM | 1247 | CA | LEU | A | 198 | 1.447 | -1.314 | 2.669 | 1.00 | 28.79 | C |
| ATOM | 1248 | CB | LEU | A | 198 | 0.102 | -0.607 | 2.777 | 1.00 | 29.35 | C |
| ATOM | 1249 | CG | LEU | A | 198 | -0.307 | 0.357 | 1.657 | 1.00 | 32.43 | C |
| ATOM | 1250 | CD1 | LEU | A | 198 | -1.344 | 1.334 | 2.209 | 1.00 | 33.14 | C |
| ATOM | 1251 | CD2 | LEU | A | 198 | 0.890 | 1.110 | 1.109 | 1.00 | 31.16 | C |
| ATOM | 1252 | C | LEU | A | 198 | 2.415 | -0.699 | 3.658 | 1.00 | 29.38 | C |
| ATOM | 1253 | O | LEU | A | 198 | 2.327 | -0.978 | 4.855 | 1.00 | 30.81 | O |
| ATOM | 1254 | N | CYS | A | 199 | 3.343 | 0.119 | 3.183 | 1.00 | 28.99 | N |
| ATOM | 1255 | CA | CYS | A | 199 | 4.291 | 0.737 | 4.094 | 1.00 | 30.94 | C |
| ATOM | 1256 | CB | CYS | A | 199 | 5.630 | -0.011 | 4.075 | 1.00 | 29.54 | C |
| ATOM | 1257 | SG | CYS | A | 199 | 6.739 | 0.416 | 2.692 | 1.00 | 31.32 | S |
| ATOM | 1258 | C | CYS | A | 199 | 4.509 | 2.205 | 3.751 | 1.00 | 31.60 | C |
| ATOM | 1259 | O | CYS | A | 199 | 3.936 | 2.723 | 2.793 | 1.00 | 31.06 | O |
| ATOM | 1260 | N | ASP | A | 200 | 5.338 | 2.866 | 4.554 | 1.00 | 31.25 | N |
| ATOM | 1261 | CA | ASP | A | 200 | 5.661 | 4.275 | 4.377 | 1.00 | 31.41 | C |
| ATOM | 1262 | CB | ASP | A | 200 | 6.411 | 4.499 | 3.070 | 1.00 | 33.09 | C |
| ATOM | 1263 | CG | ASP | A | 200 | 6.859 | 5.935 | 2.904 | 1.00 | 36.04 | C |
| ATOM | 1264 | OD1 | ASP | A | 200 | 6.678 | 6.723 | 3.853 | 1.00 | 38.27 | O |
| ATOM | 1265 | OD2 | ASP | A | 200 | 7.401 | 6.280 | 1.834 | 1.00 | 37.07 | O |
| ATOM | 1266 | C | ASP | A | 200 | 4.459 | 5.211 | 4.421 | 1.00 | 32.08 | C |

| | | | | | | | | | | | |
|---|---|---|---|---|---|---|---|---|---|---|---|
| ATOM | 1267 | O | ASP | A | 200 | 3.914 | 5.588 | 3.387 | 1.00 | 31.49 | O |
| ATOM | 1268 | N | PHE | A | 201 | 4.057 | 5.596 | 5.626 | 1.00 | 31.39 | N |
| ATOM | 1269 | CA | PHE | A | 201 | 2.935 | 6.495 | 5.773 | 1.00 | 30.98 | C |
| ATOM | 1270 | CB | PHE | A | 201 | 2.064 | 6.057 | 6.960 | 1.00 | 30.27 | C |
| ATOM | 1271 | CG | PHE | A | 201 | 1.219 | 4.835 | 6.676 | 1.00 | 29.20 | C |
| ATOM | 1272 | CD1 | PHE | A | 201 | 1.776 | 3.555 | 6.690 | 1.00 | 27.75 | C |
| ATOM | 1273 | CD2 | PHE | A | 201 | -0.130 | 4.975 | 6.341 | 1.00 | 27.95 | C |
| ATOM | 1274 | CE1 | PHE | A | 201 | 1.003 | 2.436 | 6.370 | 1.00 | 28.51 | C |
| ATOM | 1275 | CE2 | PHE | A | 201 | -0.910 | 3.862 | 6.020 | 1.00 | 28.95 | C |
| ATOM | 1276 | CZ | PHE | A | 201 | -0.344 | 2.588 | 6.032 | 1.00 | 29.19 | C |
| ATOM | 1277 | C | PHE | A | 201 | 3.396 | 7.950 | 5.925 | 1.00 | 31.63 | C |
| ATOM | 1278 | O | PHE | A | 201 | 2.707 | 8.767 | 6.536 | 1.00 | 32.82 | O |
| ATOM | 1279 | N | GLY | A | 202 | 4.555 | 8.267 | 5.346 | 1.00 | 30.70 | N |
| ATOM | 1280 | CA | GLY | A | 202 | 5.099 | 9.612 | 5.427 | 1.00 | 31.27 | C |
| ATOM | 1281 | C | GLY | A | 202 | 4.385 | 10.681 | 4.601 | 1.00 | 31.92 | C |
| ATOM | 1282 | O | GLY | A | 202 | 4.742 | 11.856 | 4.672 | 1.00 | 33.10 | O |
| ATOM | 1283 | N | SER | A | 203 | 3.386 | 10.283 | 3.815 | 1.00 | 31.33 | N |
| ATOM | 1284 | CA | SER | A | 203 | 2.620 | 11.211 | 2.991 | 1.00 | 29.57 | C |
| ATOM | 1285 | CB | SER | A | 203 | 2.828 | 10.934 | 1.495 | 1.00 | 31.64 | C |
| ATOM | 1286 | OG | SER | A | 203 | 4.186 | 10.711 | 1.180 | 1.00 | 32.99 | O |
| ATOM | 1287 | C | SER | A | 203 | 1.145 | 11.001 | 3.306 | 1.00 | 29.70 | C |
| ATOM | 1288 | O | SER | A | 203 | 0.278 | 11.572 | 2.654 | 1.00 | 30.28 | O |
| ATOM | 1289 | N | ALA | A | 204 | 0.868 | 10.158 | 4.292 | 1.00 | 28.35 | N |
| ATOM | 1290 | CA | ALA | A | 204 | -0.500 | 9.856 | 4.680 | 1.00 | 28.96 | C |
| ATOM | 1291 | CB | ALA | A | 204 | -0.525 | 8.605 | 5.545 | 1.00 | 29.32 | C |
| ATOM | 1292 | C | ALA | A | 204 | -1.138 | 11.028 | 5.421 | 1.00 | 30.34 | C |
| ATOM | 1293 | O | ALA | A | 204 | -0.452 | 11.810 | 6.083 | 1.00 | 30.28 | O |
| ATOM | 1294 | N | LYS | A | 205 | -2.455 | 11.147 | 5.306 | 1.00 | 30.56 | N |
| ATOM | 1295 | CA | LYS | A | 205 | -3.171 | 12.235 | 5.946 | 1.00 | 30.49 | C |
| ATOM | 1296 | CB | LYS | A | 205 | -2.979 | 13.530 | 5.149 | 1.00 | 29.02 | C |
| ATOM | 1297 | CG | LYS | A | 205 | -3.653 | 14.767 | 5.755 | 1.00 | 26.54 | C |
| ATOM | 1298 | CD | LYS | A | 205 | -3.414 | 15.996 | 4.890 | 1.00 | 25.13 | C |
| ATOM | 1299 | CE | LYS | A | 205 | -4.076 | 17.232 | 5.469 | 1.00 | 26.48 | C |
| ATOM | 1300 | NZ | LYS | A | 205 | -3.820 | 18.457 | 4.653 | 1.00 | 26.84 | N |
| ATOM | 1301 | C | LYS | A | 205 | -4.651 | 11.930 | 6.058 | 1.00 | 32.84 | C |
| ATOM | 1302 | O | LYS | A | 205 | -5.228 | 11.260 | 5.193 | 1.00 | 32.93 | O |
| ATOM | 1303 | N | GLN | A | 206 | -5.264 | 12.410 | 7.134 | 1.00 | 36.09 | N |
| ATOM | 1304 | CA | GLN | A | 206 | -6.692 | 12.205 | 7.332 | 1.00 | 39.32 | C |
| ATOM | 1305 | CB | GLN | A | 206 | -7.034 | 12.178 | 8.830 | 1.00 | 40.76 | C |
| ATOM | 1306 | CG | GLN | A | 206 | -7.007 | 10.769 | 9.441 | 1.00 | 45.19 | C |
| ATOM | 1307 | CD | GLN | A | 206 | -6.290 | 10.714 | 10.809 | 1.00 | 48.38 | C |
| ATOM | 1308 | OE1 | GLN | A | 206 | -5.289 | 11.423 | 11.032 | 1.00 | 49.50 | O |
| ATOM | 1309 | NE2 | GLN | A | 206 | -6.790 | 9.865 | 11.717 | 1.00 | 45.12 | N |
| ATOM | 1310 | C | GLN | A | 206 | -7.341 | 13.391 | 6.628 | 1.00 | 39.67 | C |
| ATOM | 1311 | O | GLN | A | 206 | -7.221 | 14.531 | 7.070 | 1.00 | 38.53 | O |
| ATOM | 1312 | N | LEU | A | 207 | -7.987 | 13.117 | 5.500 | 1.00 | 41.38 | N |
| ATOM | 1313 | CA | LEU | A | 207 | -8.634 | 14.163 | 4.728 | 1.00 | 41.27 | C |
| ATOM | 1314 | CB | LEU | A | 207 | -8.969 | 13.651 | 3.328 | 1.00 | 38.51 | C |
| ATOM | 1315 | CG | LEU | A | 207 | -7.796 | 13.081 | 2.535 | 1.00 | 37.32 | C |
| ATOM | 1316 | CD1 | LEU | A | 207 | -8.319 | 12.485 | 1.260 | 1.00 | 36.55 | C |
| ATOM | 1317 | CD2 | LEU | A | 207 | -6.755 | 14.164 | 2.260 | 1.00 | 35.85 | C |
| ATOM | 1318 | C | LEU | A | 207 | -9.900 | 14.614 | 5.424 | 1.00 | 42.67 | C |
| ATOM | 1319 | O | LEU | A | 207 | -10.733 | 13.798 | 5.811 | 1.00 | 42.22 | O |
| ATOM | 1320 | N | VAL | A | 208 | -10.024 | 15.923 | 5.595 | 1.00 | 44.97 | N |
| ATOM | 1321 | CA | VAL | A | 208 | -11.194 | 16.506 | 6.221 | 1.00 | 47.55 | C |
| ATOM | 1322 | CB | VAL | A | 208 | -10.811 | 17.428 | 7.398 | 1.00 | 47.97 | C |
| ATOM | 1323 | CG1 | VAL | A | 208 | -12.050 | 18.092 | 7.965 | 1.00 | 47.25 | C |
| ATOM | 1324 | CG2 | VAL | A | 208 | -10.112 | 16.631 | 8.480 | 1.00 | 46.97 | C |
| ATOM | 1325 | C | VAL | A | 208 | -11.881 | 17.324 | 5.148 | 1.00 | 48.95 | C |
| ATOM | 1326 | O | VAL | A | 208 | -11.287 | 18.244 | 4.578 | 1.00 | 48.54 | O |
| ATOM | 1327 | N | ARG | A | 209 | -13.127 | 16.974 | 4.849 | 1.00 | 50.85 | N |
| ATOM | 1328 | CA | ARG | A | 209 | -13.867 | 17.707 | 3.829 | 1.00 | 53.21 | C |
| ATOM | 1329 | CB | ARG | A | 209 | -15.308 | 17.195 | 3.745 | 1.00 | 55.70 | C |
| ATOM | 1330 | CG | ARG | A | 209 | -15.978 | 17.447 | 2.393 | 1.00 | 60.52 | C |
| ATOM | 1331 | CD | ARG | A | 209 | -17.421 | 16.942 | 2.405 | 1.00 | 64.17 | C |
| ATOM | 1332 | NE | ARG | A | 209 | -17.512 | 15.580 | 2.942 | 1.00 | 66.64 | N |
| ATOM | 1333 | CZ | ARG | A | 209 | -18.654 | 14.943 | 3.182 | 1.00 | 68.12 | C |

| | | | | | | | | | | | |
|---|---|---|---|---|---|---|---|---|---|---|---|
| ATOM | 1334 | NH1 | ARG | A | 209 | -19.822 | 15.540 | 2.933 | 1.00 | 67.43 | N |
| ATOM | 1335 | NH2 | ARG | A | 209 | -18.618 | 13.713 | 3.686 | 1.00 | 68.56 | N |
| ATOM | 1336 | C | ARG | A | 209 | -13.848 | 19.181 | 4.236 | 1.00 | 52.77 | C |
| ATOM | 1337 | O | ARG | A | 209 | -14.137 | 19.520 | 5.389 | 1.00 | 52.99 | O |
| ATOM | 1338 | N | GLY | A | 210 | -13.482 | 20.046 | 3.299 | 1.00 | 51.67 | N |
| ATOM | 1339 | CA | GLY | A | 210 | -13.432 | 21.463 | 3.590 | 1.00 | 51.36 | C |
| ATOM | 1340 | C | GLY | A | 210 | -12.022 | 21.988 | 3.716 | 1.00 | 51.86 | C |
| ATOM | 1341 | O | GLY | A | 210 | -11.728 | 23.112 | 3.304 | 1.00 | 51.69 | O |
| ATOM | 1342 | N | GLU | A | 211 | -11.145 | 21.178 | 4.304 | 1.00 | 51.49 | N |
| ATOM | 1343 | CA | GLU | A | 211 | -9.736 | 21.551 | 4.473 | 1.00 | 50.79 | C |
| ATOM | 1344 | CB | GLU | A | 211 | -9.126 | 20.852 | 5.684 | 1.00 | 52.25 | C |
| ATOM | 1345 | CG | GLU | A | 211 | -9.897 | 20.956 | 6.965 | 1.00 | 53.69 | C |
| ATOM | 1346 | CD | GLU | A | 211 | -9.335 | 20.015 | 8.013 | 1.00 | 53.73 | C |
| ATOM | 1347 | OE1 | GLU | A | 211 | -9.723 | 20.155 | 9.196 | 1.00 | 53.01 | O |
| ATOM | 1348 | OE2 | GLU | A | 211 | -8.515 | 19.139 | 7.635 | 1.00 | 53.06 | O |
| ATOM | 1349 | C | GLU | A | 211 | -8.937 | 21.133 | 3.251 | 1.00 | 49.36 | C |
| ATOM | 1350 | O | GLU | A | 211 | -8.912 | 19.956 | 2.885 | 1.00 | 50.93 | O |
| ATOM | 1351 | N | PRO | A | 212 | -8.240 | 22.079 | 2.625 | 1.00 | 47.67 | N |
| ATOM | 1352 | CD | PRO | A | 212 | -8.079 | 23.494 | 3.005 | 1.00 | 47.60 | C |
| ATOM | 1353 | CA | PRO | A | 212 | -7.445 | 21.754 | 1.433 | 1.00 | 45.29 | C |
| ATOM | 1354 | CB | PRO | A | 212 | -7.090 | 23.123 | 0.877 | 1.00 | 45.83 | C |
| ATOM | 1355 | CG | PRO | A | 212 | -6.914 | 23.934 | 2.121 | 1.00 | 46.23 | C |
| ATOM | 1356 | C | PRO | A | 212 | -6.204 | 20.926 | 1.707 | 1.00 | 42.30 | C |
| ATOM | 1357 | O | PRO | A | 212 | -5.651 | 20.946 | 2.801 | 1.00 | 41.90 | O |
| ATOM | 1358 | N | ASN | A | 213 | -5.777 | 20.191 | 0.697 | 1.00 | 40.09 | N |
| ATOM | 1359 | CA | ASN | A | 213 | -4.601 | 19.353 | 0.804 | 1.00 | 37.64 | C |
| ATOM | 1360 | CB | ASN | A | 213 | -5.012 | 17.884 | 0.822 | 1.00 | 36.75 | C |
| ATOM | 1361 | CG | ASN | A | 213 | -6.071 | 17.601 | 1.846 | 1.00 | 36.14 | C |
| ATOM | 1362 | OD1 | ASN | A | 213 | -5.866 | 17.793 | 3.043 | 1.00 | 39.69 | O |
| ATOM | 1363 | ND2 | ASN | A | 213 | -7.223 | 17.160 | 1.385 | 1.00 | 38.08 | N |
| ATOM | 1364 | C | ASN | A | 213 | -3.826 | 19.699 | -0.452 | 1.00 | 35.89 | C |
| ATOM | 1365 | O | ASN | A | 213 | -4.404 | 20.187 | -1.420 | 1.00 | 35.54 | O |
| ATOM | 1366 | N | VAL | A | 214 | -2.526 | 19.456 | -0.431 | 1.00 | 34.82 | N |
| ATOM | 1367 | CA | VAL | A | 214 | -1.691 | 19.798 | -1.555 | 1.00 | 35.55 | C |
| ATOM | 1368 | CB | VAL | A | 214 | -0.222 | 19.985 | -1.077 | 1.00 | 35.97 | C |
| ATOM | 1369 | CG1 | VAL | A | 214 | 0.205 | 18.817 | -0.204 | 1.00 | 37.11 | C |
| ATOM | 1370 | CG2 | VAL | A | 214 | 0.711 | 20.137 | -2.265 | 1.00 | 38.67 | C |
| ATOM | 1371 | C | VAL | A | 214 | -1.804 | 18.816 | -2.725 | 1.00 | 36.65 | C |
| ATOM | 1372 | O | VAL | A | 214 | -1.860 | 17.587 | -2.541 | 1.00 | 35.11 | O |
| ATOM | 1373 | N | SER | A | 215 | -1.842 | 19.381 | -3.934 | 1.00 | 36.48 | N |
| ATOM | 1374 | CA | SER | A | 215 | -1.995 | 18.602 | -5.160 | 1.00 | 35.74 | C |
| ATOM | 1375 | CB | SER | A | 215 | -2.608 | 19.480 | -6.256 | 1.00 | 35.28 | C |
| ATOM | 1376 | OG | SER | A | 215 | -1.806 | 20.610 | -6.511 | 1.00 | 38.00 | O |
| ATOM | 1377 | C | SER | A | 215 | -0.762 | 17.896 | -5.708 | 1.00 | 34.72 | C |
| ATOM | 1378 | O | SER | A | 215 | -0.872 | 16.791 | -6.234 | 1.00 | 35.28 | O |
| ATOM | 1379 | N | PTY | A | 216 | 0.404 | 18.516 | -5.612 | 1.00 | 34.35 | N |
| ATOM | 1380 | CA | PTY | A | 216 | 1.609 | 17.879 | -6.126 | 1.00 | 33.64 | C |
| ATOM | 1381 | C | PTY | A | 216 | 2.071 | 16.784 | -5.174 | 1.00 | 35.17 | C |
| ATOM | 1382 | O | PTY | A | 216 | 3.065 | 16.930 | -4.461 | 1.00 | 35.61 | O |
| ATOM | 1383 | CB | PTY | A | 216 | 2.721 | 18.916 | -6.303 | 1.00 | 33.07 | C |
| ATOM | 1384 | CG | PTY | A | 216 | 3.798 | 18.303 | -7.148 | 1.00 | 34.38 | C |
| ATOM | 1385 | CD1 | PTY | A | 216 | 3.651 | 18.180 | -8.598 | 1.00 | 33.03 | C |
| ATOM | 1386 | CD2 | PTY | A | 216 | 4.984 | 17.704 | -6.489 | 1.00 | 33.58 | C |
| ATOM | 1387 | CE1 | PTY | A | 216 | 4.660 | 17.474 | -9.378 | 1.00 | 35.41 | C |
| ATOM | 1388 | CE2 | PTY | A | 216 | 5.995 | 16.998 | -7.245 | 1.00 | 33.57 | C |
| ATOM | 1389 | CZ | PTY | A | 216 | 5.835 | 16.887 | -8.691 | 1.00 | 35.46 | C |
| ATOM | 1390 | OH | PTY | A | 216 | 6.916 | 16.588 | -9.378 | 1.00 | 37.08 | O |
| ATOM | 1391 | P | PTY | A | 216 | 7.184 | 15.289 | -9.958 | 1.00 | 38.30 | P |
| ATOM | 1392 | OP1 | PTY | A | 216 | 8.419 | 15.912 | -10.430 | 1.00 | 41.12 | O |
| ATOM | 1393 | OP2 | PTY | A | 216 | 6.262 | 14.911 | -11.109 | 1.00 | 38.93 | O |
| ATOM | 1394 | OP3 | PTY | A | 216 | 7.461 | 14.199 | -9.070 | 1.00 | 37.29 | O |
| ATOM | 1395 | N | ILE | A | 217 | 1.346 | 15.678 | -5.144 | 1.00 | 36.32 | N |
| ATOM | 1396 | CA | ILE | A | 217 | 1.737 | 14.612 | -4.254 | 1.00 | 36.20 | C |
| ATOM | 1397 | CB | ILE | A | 217 | 0.905 | 14.663 | -2.962 | 1.00 | 37.58 | C |
| ATOM | 1398 | CG2 | ILE | A | 217 | -0.513 | 14.153 | -3.226 | 1.00 | 35.83 | C |
| ATOM | 1399 | CG1 | ILE | A | 217 | 1.658 | 13.897 | -1.869 | 1.00 | 38.35 | C |
| ATOM | 1400 | CD1 | ILE | A | 217 | 1.172 | 14.139 | -0.480 | 1.00 | 37.35 | C |

| | | | | | | | | | | | |
|---|---|---|---|---|---|---|---|---|---|---|---|
| ATOM | 1401 | C | ILE | A | 217 | 1.609 | 13.266 | -4.959 | 1.00 | 36.52 | C |
| ATOM | 1402 | O | ILE | A | 217 | 1.121 | 13.201 | -6.084 | 1.00 | 36.17 | O |
| ATOM | 1403 | N | CYS | A | 218 | 2.060 | 12.201 | -4.300 | 1.00 | 36.04 | N |
| ATOM | 1404 | CA | CYS | A | 218 | 2.056 | 10.847 | -4.867 | 1.00 | 36.09 | C |
| ATOM | 1405 | CB | CYS | A | 218 | 0.771 | 10.517 | -5.626 | 1.00 | 38.39 | C |
| ATOM | 1406 | SG | CYS | A | 218 | -0.736 | 10.597 | -4.721 | 1.00 | 44.31 | S |
| ATOM | 1407 | C | CYS | A | 218 | 3.173 | 10.839 | -5.883 | 1.00 | 34.47 | C |
| ATOM | 1408 | O | CYS | A | 218 | 3.583 | 11.898 | -6.364 | 1.00 | 32.18 | O |
| ATOM | 1409 | N | SER | A | 219 | 3.652 | 9.646 | -6.213 | 1.00 | 34.26 | N |
| ATOM | 1410 | CA | SER | A | 219 | 4.710 | 9.490 | -7.198 | 1.00 | 33.32 | C |
| ATOM | 1411 | CB | SER | A | 219 | 5.285 | 8.077 | -7.090 | 1.00 | 33.65 | C |
| ATOM | 1412 | OG | SER | A | 219 | 6.552 | 8.014 | -7.697 | 1.00 | 32.28 | O |
| ATOM | 1413 | C | SER | A | 219 | 4.092 | 9.730 | -8.584 | 1.00 | 32.66 | C |
| ATOM | 1414 | O | SER | A | 219 | 3.022 | 9.216 | -8.882 | 1.00 | 33.33 | O |
| ATOM | 1415 | N | ARG | A | 220 | 4.785 | 10.488 | -9.427 | 1.00 | 32.85 | N |
| ATOM | 1416 | CA | ARG | A | 220 | 4.311 | 10.825 | -10.774 | 1.00 | 32.72 | C |
| ATOM | 1417 | CB | ARG | A | 220 | 5.473 | 11.273 | -11.654 | 1.00 | 31.77 | C |
| ATOM | 1418 | CG | ARG | A | 220 | 5.008 | 11.859 | -12.972 | 1.00 | 33.84 | C |
| ATOM | 1419 | CD | ARG | A | 220 | 5.951 | 12.947 | -13.411 | 1.00 | 33.91 | C |
| ATOM | 1420 | NE | ARG | A | 220 | 7.168 | 12.409 | -14.002 | 1.00 | 36.23 | N |
| ATOM | 1421 | CZ | ARG | A | 220 | 8.380 | 12.909 | -13.794 | 1.00 | 36.27 | C |
| ATOM | 1422 | NH1 | ARG | A | 220 | 8.537 | 13.959 | -12.994 | 1.00 | 33.49 | N |
| ATOM | 1423 | NH2 | ARG | A | 220 | 9.426 | 12.369 | -14.410 | 1.00 | 37.73 | N |
| ATOM | 1424 | C | ARG | A | 220 | 3.523 | 9.772 | -11.545 | 1.00 | 33.43 | C |
| ATOM | 1425 | O | ARG | A | 220 | 2.375 | 9.997 | -11.915 | 1.00 | 34.27 | O |
| ATOM | 1426 | N | TYR | A | 221 | 4.149 | 8.635 | -11.816 | 1.00 | 32.62 | N |
| ATOM | 1427 | CA | TYR | A | 221 | 3.492 | 7.580 | -12.569 | 1.00 | 32.82 | C |
| ATOM | 1428 | CB | TYR | A | 221 | 4.415 | 6.375 | -12.694 | 1.00 | 32.44 | C |
| ATOM | 1429 | CG | TYR | A | 221 | 5.713 | 6.644 | -13.416 | 1.00 | 32.65 | C |
| ATOM | 1430 | CD1 | TYR | A | 221 | 5.884 | 7.781 | -14.206 | 1.00 | 32.74 | C |
| ATOM | 1431 | CE1 | TYR | A | 221 | 7.070 | 7.997 | -14.893 | 1.00 | 32.70 | C |
| ATOM | 1432 | CD2 | TYR | A | 221 | 6.764 | 5.739 | -13.336 | 1.00 | 33.10 | C |
| ATOM | 1433 | CE2 | TYR | A | 221 | 7.948 | 5.946 | -14.019 | 1.00 | 31.97 | C |
| ATOM | 1434 | CZ | TYR | A | 221 | 8.098 | 7.068 | -14.795 | 1.00 | 32.65 | C |
| ATOM | 1435 | OH | TYR | A | 221 | 9.275 | 7.259 | -15.476 | 1.00 | 31.38 | O |
| ATOM | 1436 | C | TYR | A | 221 | 2.158 | 7.122 | -12.002 | 1.00 | 33.10 | C |
| ATOM | 1437 | O | TYR | A | 221 | 1.285 | 6.682 | -12.751 | 1.00 | 33.73 | O |
| ATOM | 1438 | N | TYR | A | 222 | 1.999 | 7.217 | -10.686 | 1.00 | 32.68 | N |
| ATOM | 1439 | CA | TYR | A | 222 | 0.764 | 6.777 | -10.035 | 1.00 | 30.81 | C |
| ATOM | 1440 | CB | TYR | A | 222 | 1.092 | 5.804 | -8.899 | 1.00 | 30.81 | C |
| ATOM | 1441 | CG | TYR | A | 222 | 2.253 | 4.896 | -9.228 | 1.00 | 33.17 | C |
| ATOM | 1442 | CD1 | TYR | A | 222 | 3.568 | 5.306 | -8.990 | 1.00 | 33.10 | C |
| ATOM | 1443 | CE1 | TYR | A | 222 | 4.646 | 4.533 | -9.402 | 1.00 | 32.97 | C |
| ATOM | 1444 | CD2 | TYR | A | 222 | 2.052 | 3.671 | -9.880 | 1.00 | 33.41 | C |
| ATOM | 1445 | CE2 | TYR | A | 222 | 3.128 | 2.889 | -10.297 | 1.00 | 32.92 | C |
| ATOM | 1446 | CZ | TYR | A | 222 | 4.421 | 3.329 | -10.061 | 1.00 | 32.99 | C |
| ATOM | 1447 | OH | TYR | A | 222 | 5.488 | 2.593 | -10.516 | 1.00 | 33.31 | O |
| ATOM | 1448 | C | TYR | A | 222 | -0.071 | 7.935 | -9.502 | 1.00 | 30.86 | C |
| ATOM | 1449 | O | TYR | A | 222 | -1.028 | 7.726 | -8.759 | 1.00 | 31.13 | O |
| ATOM | 1450 | N | ARG | A | 223 | 0.290 | 9.153 | -9.890 | 1.00 | 28.09 | N |
| ATOM | 1451 | CA | ARG | A | 223 | -0.429 | 10.332 | -9.450 | 1.00 | 27.49 | C |
| ATOM | 1452 | CB | ARG | A | 223 | 0.412 | 11.583 | -9.709 | 1.00 | 26.71 | C |
| ATOM | 1453 | CG | ARG | A | 223 | -0.328 | 12.876 | -9.460 | 1.00 | 26.66 | C |
| ATOM | 1454 | CD | ARG | A | 223 | 0.579 | 13.918 | -8.842 | 1.00 | 28.37 | C |
| ATOM | 1455 | NE | ARG | A | 223 | 1.670 | 14.281 | -9.727 | 1.00 | 29.06 | N |
| ATOM | 1456 | CZ | ARG | A | 223 | 2.958 | 14.084 | -9.464 | 1.00 | 29.40 | C |
| ATOM | 1457 | NH1 | ARG | A | 223 | 3.337 | 13.521 | -8.325 | 1.00 | 24.46 | N |
| ATOM | 1458 | NH2 | ARG | A | 223 | 3.872 | 14.458 | -10.353 | 1.00 | 30.55 | N |
| ATOM | 1459 | C | ARG | A | 223 | -1.781 | 10.465 | -10.137 | 1.00 | 27.70 | C |
| ATOM | 1460 | O | ARG | A | 223 | -1.866 | 10.475 | -11.366 | 1.00 | 27.33 | O |
| ATOM | 1461 | N | ALA | A | 224 | -2.833 | 10.568 | -9.334 | 1.00 | 27.20 | N |
| ATOM | 1462 | CA | ALA | A | 224 | -4.188 | 10.705 | -9.849 | 1.00 | 28.88 | C |
| ATOM | 1463 | CB | ALA | A | 224 | -5.189 | 10.786 | -8.693 | 1.00 | 29.07 | C |
| ATOM | 1464 | C | ALA | A | 224 | -4.289 | 11.948 | -10.716 | 1.00 | 29.31 | C |
| ATOM | 1465 | O | ALA | A | 224 | -3.602 | 12.942 | -10.485 | 1.00 | 29.85 | O |
| ATOM | 1466 | N | PRO | A | 225 | -5.156 | 11.908 | -11.730 | 1.00 | 29.19 | N |
| ATOM | 1467 | CD | PRO | A | 225 | -5.948 | 10.751 | -12.167 | 1.00 | 29.59 | C |

| | | | | | | | | | | | |
|---|---|---|---|---|---|---|---|---|---|---|---|
| ATOM | 1468 | CA | PRO | A | 225 | -5.336 | 13.042 | -12.629 | 1.00 | 28.85 | C |
| ATOM | 1469 | CB | PRO | A | 225 | -6.205 | 12.462 | -13.742 | 1.00 | 28.09 | C |
| ATOM | 1470 | CG | PRO | A | 225 | -6.987 | 11.396 | -13.041 | 1.00 | 29.69 | C |
| ATOM | 1471 | C | PRO | A | 225 | -5.924 | 14.309 | -11.985 | 1.00 | 29.34 | C |
| ATOM | 1472 | O | PRO | A | 225 | -5.609 | 15.418 | -12.423 | 1.00 | 28.61 | O |
| ATOM | 1473 | N | GLU | A | 226 | -6.762 | 14.168 | -10.957 | 1.00 | 29.89 | N |
| ATOM | 1474 | CA | GLU | A | 226 | -7.320 | 15.359 | -10.313 | 1.00 | 30.87 | C |
| ATOM | 1475 | CB | GLU | A | 226 | -8.273 | 15.022 | -9.166 | 1.00 | 29.98 | C |
| ATOM | 1476 | CG | GLU | A | 226 | -9.172 | 13.855 | -9.408 | 1.00 | 30.89 | C |
| ATOM | 1477 | CD | GLU | A | 226 | -8.517 | 12.558 | -9.037 | 1.00 | 28.21 | C |
| ATOM | 1478 | OE1 | GLU | A | 226 | -8.678 | 12.119 | -7.881 | 1.00 | 25.77 | O |
| ATOM | 1479 | OE2 | GLU | A | 226 | -7.828 | 11.993 | -9.906 | 1.00 | 30.27 | O |
| ATOM | 1480 | C | GLU | A | 226 | -6.158 | 16.128 | -9.723 | 1.00 | 31.55 | C |
| ATOM | 1481 | O | GLU | A | 226 | -6.189 | 17.352 | -9.650 | 1.00 | 32.79 | O |
| ATOM | 1482 | N | LEU | A | 227 | -5.138 | 15.398 | -9.286 | 1.00 | 30.40 | N |
| ATOM | 1483 | CA | LEU | A | 227 | -3.966 | 16.018 | -8.700 | 1.00 | 29.20 | C |
| ATOM | 1484 | CB | LEU | A | 227 | -3.058 | 14.941 | -8.088 | 1.00 | 29.09 | C |
| ATOM | 1485 | CG | LEU | A | 227 | -3.696 | 14.026 | -7.026 | 1.00 | 28.07 | C |
| ATOM | 1486 | CD1 | LEU | A | 227 | -2.794 | 12.833 | -6.771 | 1.00 | 26.83 | C |
| ATOM | 1487 | CD2 | LEU | A | 227 | -3.961 | 14.798 | -5.739 | 1.00 | 24.21 | C |
| ATOM | 1488 | C | LEU | A | 227 | -3.217 | 16.832 | -9.755 | 1.00 | 28.59 | C |
| ATOM | 1489 | O | LEU | A | 227 | -2.917 | 18.002 | -9.529 | 1.00 | 29.37 | O |
| ATOM | 1490 | N | ILE | A | 228 | -2.934 | 16.231 | -10.907 | 1.00 | 28.71 | N |
| ATOM | 1491 | CA | ILE | A | 228 | -2.227 | 16.930 | -11.971 | 1.00 | 31.09 | C |
| ATOM | 1492 | CB | ILE | A | 228 | -2.076 | 16.051 | -13.219 | 1.00 | 31.80 | C |
| ATOM | 1493 | CG2 | ILE | A | 228 | -1.140 | 16.718 | -14.215 | 1.00 | 30.05 | C |
| ATOM | 1494 | CG1 | ILE | A | 228 | -1.535 | 14.683 | -12.829 | 1.00 | 31.58 | C |
| ATOM | 1495 | CD1 | ILE | A | 228 | -1.560 | 13.693 | -13.953 | 1.00 | 30.62 | C |
| ATOM | 1496 | C | ILE | A | 228 | -2.970 | 18.198 | -12.386 | 1.00 | 33.62 | C |
| ATOM | 1497 | O | ILE | A | 228 | -2.356 | 19.154 | -12.848 | 1.00 | 34.57 | O |
| ATOM | 1498 | N | PHE | A | 229 | -4.294 | 18.178 | -12.244 | 1.00 | 35.44 | N |
| ATOM | 1499 | CA | PHE | A | 229 | -5.145 | 19.311 | -12.579 | 1.00 | 36.08 | C |
| ATOM | 1500 | CB | PHE | A | 229 | -6.587 | 18.854 | -12.815 | 1.00 | 36.13 | C |
| ATOM | 1501 | CG | PHE | A | 229 | -6.929 | 18.628 | -14.253 | 1.00 | 35.19 | C |
| ATOM | 1502 | CD1 | PHE | A | 229 | -6.958 | 19.689 | -15.152 | 1.00 | 34.89 | C |
| ATOM | 1503 | CD2 | PHE | A | 229 | -7.234 | 17.351 | -14.709 | 1.00 | 36.13 | C |
| ATOM | 1504 | CE1 | PHE | A | 229 | -7.285 | 19.485 | -16.490 | 1.00 | 35.15 | C |
| ATOM | 1505 | CE2 | PHE | A | 229 | -7.563 | 17.130 | -16.042 | 1.00 | 36.07 | C |
| ATOM | 1506 | CZ | PHE | A | 229 | -7.588 | 18.202 | -16.937 | 1.00 | 35.85 | C |
| ATOM | 1507 | C | PHE | A | 229 | -5.144 | 20.341 | -11.455 | 1.00 | 36.93 | C |
| ATOM | 1508 | O | PHE | A | 229 | -5.903 | 21.305 | -11.499 | 1.00 | 38.07 | O |
| ATOM | 1509 | N | GLY | A | 230 | -4.327 | 20.115 | -10.432 | 1.00 | 37.43 | N |
| ATOM | 1510 | CA | GLY | A | 230 | -4.244 | 21.059 | -9.332 | 1.00 | 36.98 | C |
| ATOM | 1511 | C | GLY | A | 230 | -5.334 | 20.991 | -8.282 | 1.00 | 36.57 | C |
| ATOM | 1512 | O | GLY | A | 230 | -5.435 | 21.895 | -7.456 | 1.00 | 37.78 | O |
| ATOM | 1513 | N | ALA | A | 231 | -6.148 | 19.942 | -8.303 | 1.00 | 36.26 | N |
| ATOM | 1514 | CA | ALA | A | 231 | -7.217 | 19.805 | -7.317 | 1.00 | 36.84 | C |
| ATOM | 1515 | CB | ALA | A | 231 | -7.976 | 18.521 | -7.552 | 1.00 | 36.49 | C |
| ATOM | 1516 | C | ALA | A | 231 | -6.660 | 19.831 | -5.896 | 1.00 | 37.80 | C |
| ATOM | 1517 | O | ALA | A | 231 | -5.516 | 19.439 | -5.655 | 1.00 | 38.08 | O |
| ATOM | 1518 | N | THR | A | 232 | -7.477 | 20.276 | -4.953 | 1.00 | 37.67 | N |
| ATOM | 1519 | CA | THR | A | 232 | -7.042 | 20.369 | -3.573 | 1.00 | 37.91 | C |
| ATOM | 1520 | CB | THR | A | 232 | -6.740 | 21.824 | -3.221 | 1.00 | 39.27 | C |
| ATOM | 1521 | OG1 | THR | A | 232 | -7.961 | 22.575 | -3.223 | 1.00 | 41.40 | O |
| ATOM | 1522 | CG2 | THR | A | 232 | -5.805 | 22.432 | -4.258 | 1.00 | 37.93 | C |
| ATOM | 1523 | C | THR | A | 232 | -8.110 | 19.835 | -2.629 | 1.00 | 38.08 | C |
| ATOM | 1524 | O | THR | A | 232 | -7.904 | 19.767 | -1.420 | 1.00 | 38.20 | O |
| ATOM | 1525 | N | ASP | A | 233 | -9.242 | 19.440 | -3.198 | 1.00 | 38.43 | N |
| ATOM | 1526 | CA | ASP | A | 233 | -10.361 | 18.914 | -2.435 | 1.00 | 39.63 | C |
| ATOM | 1527 | CB | ASP | A | 233 | -11.623 | 19.707 | -2.755 | 1.00 | 44.02 | C |
| ATOM | 1528 | CG | ASP | A | 233 | -12.109 | 19.467 | -4.179 | 1.00 | 48.53 | C |
| ATOM | 1529 | OD1 | ASP | A | 233 | -11.268 | 19.558 | -5.113 | 1.00 | 48.12 | O |
| ATOM | 1530 | OD2 | ASP | A | 233 | -13.330 | 19.192 | -4.363 | 1.00 | 51.15 | O |
| ATOM | 1531 | C | ASP | A | 233 | -10.604 | 17.458 | -2.800 | 1.00 | 38.98 | C |
| ATOM | 1532 | O | ASP | A | 233 | -11.743 | 16.986 | -2.781 | 1.00 | 39.94 | O |
| ATOM | 1533 | N | TYR | A | 234 | -9.539 | 16.753 | -3.154 | 1.00 | 36.83 | N |
| ATOM | 1534 | CA | TYR | A | 234 | -9.659 | 15.359 | -3.535 | 1.00 | 34.90 | C |

| | | | | | | | | | | | |
|---|---|---|---|---|---|---|---|---|---|---|---|
| ATOM | 1535 | CB | TYR | A | 234 | -8.401 | 14.918 | -4.269 | 1.00 | 33.46 | C |
| ATOM | 1536 | CG | TYR | A | 234 | -7.131 | 15.230 | -3.527 | 1.00 | 34.22 | C |
| ATOM | 1537 | CD1 | TYR | A | 234 | -6.635 | 14.366 | -2.553 | 1.00 | 33.48 | C |
| ATOM | 1538 | CE1 | TYR | A | 234 | -5.442 | 14.639 | -1.892 | 1.00 | 32.96 | C |
| ATOM | 1539 | CD2 | TYR | A | 234 | -6.406 | 16.381 | -3.812 | 1.00 | 33.43 | C |
| ATOM | 1540 | CE2 | TYR | A | 234 | -5.214 | 16.658 | -3.153 | 1.00 | 33.15 | C |
| ATOM | 1541 | CZ | TYR | A | 234 | -4.740 | 15.780 | -2.196 | 1.00 | 32.32 | C |
| ATOM | 1542 | OH | TYR | A | 234 | -3.555 | 16.039 | -1.558 | 1.00 | 34.66 | O |
| ATOM | 1543 | C | TYR | A | 234 | -9.932 | 14.455 | -2.345 | 1.00 | 34.38 | C |
| ATOM | 1544 | O | TYR | A | 234 | -9.855 | 14.878 | -1.190 | 1.00 | 34.76 | O |
| ATOM | 1545 | N | THR | A | 235 | -10.270 | 13.208 | -2.638 | 1.00 | 34.10 | N |
| ATOM | 1546 | CA | THR | A | 235 | -10.582 | 12.236 | -1.605 | 1.00 | 33.35 | C |
| ATOM | 1547 | CB | THR | A | 235 | -11.968 | 11.644 | -1.820 | 1.00 | 32.30 | C |
| ATOM | 1548 | OG1 | THR | A | 235 | -11.951 | 10.809 | -2.984 | 1.00 | 34.55 | O |
| ATOM | 1549 | CG2 | THR | A | 235 | -12.977 | 12.745 | -2.029 | 1.00 | 33.19 | C |
| ATOM | 1550 | C | THR | A | 235 | -9.580 | 11.095 | -1.620 | 1.00 | 34.18 | C |
| ATOM | 1551 | O | THR | A | 235 | -8.613 | 11.106 | -2.382 | 1.00 | 35.09 | O |
| ATOM | 1552 | N | SER | A | 236 | -9.820 | 10.096 | -0.785 | 1.00 | 34.50 | N |
| ATOM | 1553 | CA | SER | A | 236 | -8.923 | 8.967 | -0.728 | 1.00 | 35.41 | C |
| ATOM | 1554 | CB | SER | A | 236 | -9.240 | 8.088 | 0.485 | 1.00 | 34.74 | C |
| ATOM | 1555 | OG | SER | A | 236 | -10.525 | 7.510 | 0.381 | 1.00 | 36.36 | O |
| ATOM | 1556 | C | SER | A | 236 | -8.983 | 8.143 | -2.010 | 1.00 | 35.82 | C |
| ATOM | 1557 | O | SER | A | 236 | -8.175 | 7.231 | -2.204 | 1.00 | 37.93 | O |
| ATOM | 1558 | N | SER | A | 237 | -9.920 | 8.453 | -2.897 | 1.00 | 34.63 | N |
| ATOM | 1559 | CA | SER | A | 237 | -10.005 | 7.695 | -4.135 | 1.00 | 33.15 | C |
| ATOM | 1560 | CB | SER | A | 237 | -11.242 | 8.098 | -4.947 | 1.00 | 30.09 | C |
| ATOM | 1561 | OG | SER | A | 237 | -11.279 | 9.488 | -5.198 | 1.00 | 32.37 | O |
| ATOM | 1562 | C | SER | A | 237 | -8.734 | 7.871 | -4.973 | 1.00 | 32.42 | C |
| ATOM | 1563 | O | SER | A | 237 | -8.534 | 7.155 | -5.953 | 1.00 | 33.57 | O |
| ATOM | 1564 | N | ILE | A | 238 | -7.867 | 8.808 | -4.593 | 1.00 | 30.30 | N |
| ATOM | 1565 | CA | ILE | A | 238 | -6.634 | 8.975 | -5.352 | 1.00 | 29.30 | C |
| ATOM | 1566 | CB | ILE | A | 238 | -5.803 | 10.201 | -4.909 | 1.00 | 29.96 | C |
| ATOM | 1567 | CG2 | ILE | A | 238 | -6.504 | 11.492 | -5.317 | 1.00 | 28.31 | C |
| ATOM | 1568 | CG1 | ILE | A | 238 | -5.524 | 10.122 | -3.406 | 1.00 | 30.00 | C |
| ATOM | 1569 | CD1 | ILE | A | 238 | -4.461 | 11.087 | -2.924 | 1.00 | 27.91 | C |
| ATOM | 1570 | C | ILE | A | 238 | -5.764 | 7.736 | -5.156 | 1.00 | 28.55 | C |
| ATOM | 1571 | O | ILE | A | 238 | -4.912 | 7.437 | -5.988 | 1.00 | 28.29 | O |
| ATOM | 1572 | N | ASP | A | 239 | -5.972 | 7.018 | -4.056 | 1.00 | 27.56 | N |
| ATOM | 1573 | CA | ASP | A | 239 | -5.185 | 5.819 | -3.814 | 1.00 | 30.24 | C |
| ATOM | 1574 | CB | ASP | A | 239 | -5.313 | 5.358 | -2.366 | 1.00 | 29.73 | C |
| ATOM | 1575 | CG | ASP | A | 239 | -4.576 | 6.262 | -1.405 | 1.00 | 29.42 | C |
| ATOM | 1576 | OD1 | ASP | A | 239 | -3.569 | 6.872 | -1.813 | 1.00 | 32.29 | O |
| ATOM | 1577 | OD2 | ASP | A | 239 | -4.985 | 6.357 | -0.239 | 1.00 | 29.85 | O |
| ATOM | 1578 | C | ASP | A | 239 | -5.630 | 4.703 | -4.743 | 1.00 | 32.16 | C |
| ATOM | 1579 | O | ASP | A | 239 | -4.828 | 3.887 | -5.204 | 1.00 | 32.18 | O |
| ATOM | 1580 | N | VAL | A | 240 | -6.928 | 4.689 | -5.025 | 1.00 | 32.10 | N |
| ATOM | 1581 | CA | VAL | A | 240 | -7.530 | 3.702 | -5.900 | 1.00 | 29.22 | C |
| ATOM | 1582 | CB | VAL | A | 240 | -9.049 | 3.840 | -5.838 | 1.00 | 29.31 | C |
| ATOM | 1583 | CG1 | VAL | A | 240 | -9.714 | 2.890 | -6.812 | 1.00 | 28.78 | C |
| ATOM | 1584 | CG2 | VAL | A | 240 | -9.505 | 3.574 | -4.417 | 1.00 | 24.31 | C |
| ATOM | 1585 | C | VAL | A | 240 | -7.010 | 3.864 | -7.328 | 1.00 | 29.49 | C |
| ATOM | 1586 | O | VAL | A | 240 | -6.792 | 2.876 | -8.035 | 1.00 | 29.15 | O |
| ATOM | 1587 | N | TRP | A | 241 | -6.794 | 5.111 | -7.740 | 1.00 | 28.83 | N |
| ATOM | 1588 | CA | TRP | A | 241 | -6.255 | 5.392 | -9.066 | 1.00 | 29.70 | C |
| ATOM | 1589 | CB | TRP | A | 241 | -6.187 | 6.910 | -9.326 | 1.00 | 29.95 | C |
| ATOM | 1590 | CG | TRP | A | 241 | -5.404 | 7.279 | -10.569 | 1.00 | 30.53 | C |
| ATOM | 1591 | CD2 | TRP | A | 241 | -5.917 | 7.413 | -11.902 | 1.00 | 30.79 | C |
| ATOM | 1592 | CE2 | TRP | A | 241 | -4.817 | 7.674 | -12.750 | 1.00 | 31.11 | C |
| ATOM | 1593 | CE3 | TRP | A | 241 | -7.200 | 7.335 | -12.465 | 1.00 | 30.63 | C |
| ATOM | 1594 | CD1 | TRP | A | 241 | -4.050 | 7.467 | -10.664 | 1.00 | 30.81 | C |
| ATOM | 1595 | NE1 | TRP | A | 241 | -3.691 | 7.702 | -11.968 | 1.00 | 28.67 | N |
| ATOM | 1596 | CZ2 | TRP | A | 241 | -4.962 | 7.855 | -14.135 | 1.00 | 32.68 | C |
| ATOM | 1597 | CZ3 | TRP | A | 241 | -7.338 | 7.515 | -13.841 | 1.00 | 31.79 | C |
| ATOM | 1598 | CH2 | TRP | A | 241 | -6.226 | 7.771 | -14.657 | 1.00 | 31.34 | C |
| ATOM | 1599 | C | TRP | A | 241 | -4.858 | 4.798 | -9.093 | 1.00 | 28.63 | C |
| ATOM | 1600 | O | TRP | A | 241 | -4.496 | 4.073 | -10.009 | 1.00 | 28.93 | O |
| ATOM | 1601 | N | SER | A | 242 | -4.086 | 5.108 | -8.060 | 1.00 | 30.26 | N |

| | | | | | | | | | | | |
|---|---|---|---|---|---|---|---|---|---|---|---|
| ATOM | 1602 | CA | SER | A | 242 | -2.723 | 4.612 | -7.931 | 1.00 | 29.53 | C |
| ATOM | 1603 | CB | SER | A | 242 | -2.135 | 5.091 | -6.604 | 1.00 | 29.07 | C |
| ATOM | 1604 | OG | SER | A | 242 | -1.989 | 6.495 | -6.589 | 1.00 | 26.99 | O |
| ATOM | 1605 | C | SER | A | 242 | -2.680 | 3.090 | -8.004 | 1.00 | 29.28 | C |
| ATOM | 1606 | O | SER | A | 242 | -1.873 | 2.526 | -8.730 | 1.00 | 29.28 | O |
| ATOM | 1607 | N | ALA | A | 243 | -3.560 | 2.442 | -7.244 | 1.00 | 28.76 | N |
| ATOM | 1608 | CA | ALA | A | 243 | -3.644 | 0.991 | -7.215 | 1.00 | 28.73 | C |
| ATOM | 1609 | CB | ALA | A | 243 | -4.748 | 0.552 | -6.280 | 1.00 | 26.17 | C |
| ATOM | 1610 | C | ALA | A | 243 | -3.889 | 0.437 | -8.619 | 1.00 | 31.37 | C |
| ATOM | 1611 | O | ALA | A | 243 | -3.348 | -0.604 | -8.995 | 1.00 | 31.71 | O |
| ATOM | 1612 | N | GLY | A | 244 | -4.715 | 1.137 | -9.389 | 1.00 | 31.91 | N |
| ATOM | 1613 | CA | GLY | A | 244 | -5.001 | 0.712 | -10.741 | 1.00 | 30.85 | C |
| ATOM | 1614 | C | GLY | A | 244 | -3.767 | 0.851 | -11.610 | 1.00 | 31.88 | C |
| ATOM | 1615 | O | GLY | A | 244 | -3.570 | 0.076 | -12.546 | 1.00 | 32.54 | O |
| ATOM | 1616 | N | CYS | A | 245 | -2.937 | 1.845 | -11.305 | 1.00 | 31.34 | N |
| ATOM | 1617 | CA | CYS | A | 245 | -1.699 | 2.077 | -12.049 | 1.00 | 29.68 | C |
| ATOM | 1618 | CB | CYS | A | 245 | -1.071 | 3.418 | -11.654 | 1.00 | 28.66 | C |
| ATOM | 1619 | SG | CYS | A | 245 | -1.815 | 4.867 | -12.464 | 1.00 | 30.27 | S |
| ATOM | 1620 | C | CYS | A | 245 | -0.702 | 0.967 | -11.784 | 1.00 | 28.31 | C |
| ATOM | 1621 | O | CYS | A | 245 | 0.132 | 0.661 | -12.627 | 1.00 | 26.33 | O |
| ATOM | 1622 | N | VAL | A | 246 | -0.789 | 0.381 | -10.594 | 1.00 | 28.73 | N |
| ATOM | 1623 | CA | VAL | A | 246 | 0.101 | -0.704 | -10.218 | 1.00 | 29.47 | C |
| ATOM | 1624 | CB | VAL | A | 246 | 0.109 | -0.908 | -8.691 | 1.00 | 29.19 | C |
| ATOM | 1625 | CG1 | VAL | A | 246 | 0.893 | -2.155 | -8.335 | 1.00 | 29.34 | C |
| ATOM | 1626 | CG2 | VAL | A | 246 | 0.725 | 0.307 | -8.009 | 1.00 | 27.43 | C |
| ATOM | 1627 | C | VAL | A | 246 | -0.363 | -1.987 | -10.895 | 1.00 | 30.33 | C |
| ATOM | 1628 | O | VAL | A | 246 | 0.447 | -2.758 | -11.389 | 1.00 | 31.56 | O |
| ATOM | 1629 | N | LEU | A | 247 | -1.672 | -2.213 | -10.896 | 1.00 | 30.42 | N |
| ATOM | 1630 | CA | LEU | A | 247 | -2.239 | -3.386 | -11.529 | 1.00 | 29.71 | C |
| ATOM | 1631 | CB | LEU | A | 247 | -3.750 | -3.419 | -11.322 | 1.00 | 29.66 | C |
| ATOM | 1632 | CG | LEU | A | 247 | -4.561 | -4.362 | -12.216 | 1.00 | 30.75 | C |
| ATOM | 1633 | CD1 | LEU | A | 247 | -3.981 | -5.773 | -12.169 | 1.00 | 30.55 | C |
| ATOM | 1634 | CD2 | LEU | A | 247 | -6.013 | -4.359 | -11.771 | 1.00 | 27.51 | C |
| ATOM | 1635 | C | LEU | A | 247 | -1.922 | -3.364 | -13.016 | 1.00 | 29.42 | C |
| ATOM | 1636 | O | LEU | A | 247 | -1.367 | -4.316 | -13.551 | 1.00 | 31.75 | O |
| ATOM | 1637 | N | ALA | A | 248 | -2.271 | -2.279 | -13.689 | 1.00 | 29.22 | N |
| ATOM | 1638 | CA | ALA | A | 248 | -1.998 | -2.187 | -15.111 | 1.00 | 29.20 | C |
| ATOM | 1639 | CB | ALA | A | 248 | -2.372 | -0.805 | -15.643 | 1.00 | 25.61 | C |
| ATOM | 1640 | C | ALA | A | 248 | -0.518 | -2.455 | -15.326 | 1.00 | 30.52 | C |
| ATOM | 1641 | O | ALA | A | 248 | -0.134 | -3.158 | -16.255 | 1.00 | 31.60 | O |
| ATOM | 1642 | N | GLU | A | 249 | 0.310 | -1.901 | -14.445 | 1.00 | 30.69 | N |
| ATOM | 1643 | CA | GLU | A | 249 | 1.750 | -2.063 | -14.548 | 1.00 | 30.64 | C |
| ATOM | 1644 | CB | GLU | A | 249 | 2.455 | -1.232 | -13.484 | 1.00 | 31.98 | C |
| ATOM | 1645 | CG | GLU | A | 249 | 3.936 | -1.148 | -13.734 | 1.00 | 35.05 | C |
| ATOM | 1646 | CD | GLU | A | 249 | 4.658 | -0.219 | -12.786 | 1.00 | 35.48 | C |
| ATOM | 1647 | OE1 | GLU | A | 249 | 4.225 | 0.948 | -12.641 | 1.00 | 34.88 | O |
| ATOM | 1648 | OE2 | GLU | A | 249 | 5.668 | -0.659 | -12.198 | 1.00 | 36.93 | O |
| ATOM | 1649 | C | GLU | A | 249 | 2.206 | -3.512 | -14.432 | 1.00 | 30.27 | C |
| ATOM | 1650 | O | GLU | A | 249 | 3.159 | -3.921 | -15.093 | 1.00 | 30.74 | O |
| ATOM | 1651 | N | LEU | A | 250 | 1.533 | -4.283 | -13.588 | 1.00 | 28.94 | N |
| ATOM | 1652 | CA | LEU | A | 250 | 1.884 | -5.681 | -13.396 | 1.00 | 29.45 | C |
| ATOM | 1653 | CB | LEU | A | 250 | 1.271 | -6.192 | -12.090 | 1.00 | 27.15 | C |
| ATOM | 1654 | CG | LEU | A | 250 | 1.919 | -5.629 | -10.829 | 1.00 | 26.38 | C |
| ATOM | 1655 | CD1 | LEU | A | 250 | 1.151 | -6.100 | -9.617 | 1.00 | 25.77 | C |
| ATOM | 1656 | CD2 | LEU | A | 250 | 3.378 | -6.063 | -10.758 | 1.00 | 26.26 | C |
| ATOM | 1657 | C | LEU | A | 250 | 1.448 | -6.556 | -14.572 | 1.00 | 30.45 | C |
| ATOM | 1658 | O | LEU | A | 250 | 2.033 | -7.604 | -14.816 | 1.00 | 30.84 | O |
| ATOM | 1659 | N | LEU | A | 251 | 0.421 | -6.116 | -15.294 | 1.00 | 32.27 | N |
| ATOM | 1660 | CA | LEU | A | 251 | -0.078 | -6.847 | -16.448 | 1.00 | 32.19 | C |
| ATOM | 1661 | CB | LEU | A | 251 | -1.529 | -6.471 | -16.721 | 1.00 | 31.64 | C |
| ATOM | 1662 | CG | LEU | A | 251 | -2.518 | -6.738 | -15.589 | 1.00 | 33.66 | C |
| ATOM | 1663 | CD1 | LEU | A | 251 | -3.828 | -6.001 | -15.859 | 1.00 | 32.14 | C |
| ATOM | 1664 | CD2 | LEU | A | 251 | -2.744 | -8.234 | -15.454 | 1.00 | 31.78 | C |
| ATOM | 1665 | C | LEU | A | 251 | 0.750 | -6.519 | -17.684 | 1.00 | 33.32 | C |
| ATOM | 1666 | O | LEU | A | 251 | 0.865 | -7.333 | -18.595 | 1.00 | 35.41 | O |
| ATOM | 1667 | N | LEU | A | 252 | 1.328 | -5.322 | -17.703 | 1.00 | 33.80 | N |
| ATOM | 1668 | CA | LEU | A | 252 | 2.120 | -4.843 | -18.827 | 1.00 | 34.10 | C |

| | | | | | | | | | | | |
|---|---|---|---|---|---|---|---|---|---|---|---|
| ATOM | 1669 | CB | LEU | A | 252 | 1.853 | -3.359 | -19.047 | 1.00 | 36.43 | C |
| ATOM | 1670 | CG | LEU | A | 252 | 0.685 | -2.923 | -19.916 | 1.00 | 39.28 | C |
| ATOM | 1671 | CD1 | LEU | A | 252 | -0.591 | -3.587 | -19.461 | 1.00 | 41.69 | C |
| ATOM | 1672 | CD2 | LEU | A | 252 | 0.563 | -1.424 | -19.825 | 1.00 | 41.05 | C |
| ATOM | 1673 | C | LEU | A | 252 | 3.626 | -5.025 | -18.762 | 1.00 | 35.39 | C |
| ATOM | 1674 | O | LEU | A | 252 | 4.286 | -5.069 | -19.801 | 1.00 | 36.16 | O |
| ATOM | 1675 | N | GLY | A | 253 | 4.185 | -5.102 | -17.562 | 1.00 | 35.55 | N |
| ATOM | 1676 | CA | GLY | A | 253 | 5.626 | -5.242 | -17.451 | 1.00 | 36.41 | C |
| ATOM | 1677 | C | GLY | A | 253 | 6.313 | -3.888 | -17.457 | 1.00 | 37.89 | C |
| ATOM | 1678 | O | GLY | A | 253 | 7.534 | -3.804 | -17.573 | 1.00 | 38.45 | O |
| ATOM | 1679 | N | GLN | A | 254 | 5.523 | -2.825 | -17.344 | 1.00 | 38.37 | N |
| ATOM | 1680 | CA | GLN | A | 254 | 6.049 | -1.465 | -17.309 | 1.00 | 39.87 | C |
| ATOM | 1681 | CB | GLN | A | 254 | 6.568 | -1.062 | -18.691 | 1.00 | 41.81 | C |
| ATOM | 1682 | CG | GLN | A | 254 | 5.528 | -1.130 | -19.787 | 1.00 | 45.30 | C |
| ATOM | 1683 | CD | GLN | A | 254 | 6.094 | -0.737 | -21.140 | 1.00 | 46.34 | C |
| ATOM | 1684 | OE1 | GLN | A | 254 | 6.627 | 0.360 | -21.309 | 1.00 | 47.62 | O |
| ATOM | 1685 | NE2 | GLN | A | 254 | 5.979 | -1.634 | -22.111 | 1.00 | 47.21 | N |
| ATOM | 1686 | C | GLN | A | 254 | 4.937 | -0.519 | -16.864 | 1.00 | 39.08 | C |
| ATOM | 1687 | O | GLN | A | 254 | 3.756 | -0.864 | -16.934 | 1.00 | 39.61 | O |
| ATOM | 1688 | N | PRO | A | 255 | 5.295 | 0.686 | -16.396 | 1.00 | 38.40 | N |
| ATOM | 1689 | CD | PRO | A | 255 | 6.649 | 1.208 | -16.154 | 1.00 | 37.56 | C |
| ATOM | 1690 | CA | PRO | A | 255 | 4.278 | 1.646 | -15.947 | 1.00 | 37.98 | C |
| ATOM | 1691 | CB | PRO | A | 255 | 5.112 | 2.803 | -15.392 | 1.00 | 37.50 | C |
| ATOM | 1692 | CG | PRO | A | 255 | 6.410 | 2.150 | -14.998 | 1.00 | 38.11 | C |
| ATOM | 1693 | C | PRO | A | 255 | 3.344 | 2.091 | -17.074 | 1.00 | 37.43 | C |
| ATOM | 1694 | O | PRO | A | 255 | 3.802 | 2.533 | -18.128 | 1.00 | 38.55 | O |
| ATOM | 1695 | N | ILE | A | 256 | 2.039 | 1.988 | -16.842 | 1.00 | 36.18 | N |
| ATOM | 1696 | CA | ILE | A | 256 | 1.051 | 2.362 | -17.851 | 1.00 | 35.52 | C |
| ATOM | 1697 | CB | ILE | A | 256 | -0.405 | 2.062 | -17.351 | 1.00 | 36.82 | C |
| ATOM | 1698 | CG2 | ILE | A | 256 | -0.733 | 2.871 | -16.092 | 1.00 | 36.99 | C |
| ATOM | 1699 | CG1 | ILE | A | 256 | -1.416 | 2.375 | -18.454 | 1.00 | 35.40 | C |
| ATOM | 1700 | CD1 | ILE | A | 256 | -1.263 | 1.510 | -19.655 | 1.00 | 35.08 | C |
| ATOM | 1701 | C | ILE | A | 256 | 1.156 | 3.824 | -18.304 | 1.00 | 36.13 | C |
| ATOM | 1702 | O | ILE | A | 256 | 1.025 | 4.127 | -19.489 | 1.00 | 35.31 | O |
| ATOM | 1703 | N | PHE | A | 257 | 1.407 | 4.732 | -17.370 | 1.00 | 35.20 | N |
| ATOM | 1704 | CA | PHE | A | 257 | 1.518 | 6.147 | -17.711 | 1.00 | 34.85 | C |
| ATOM | 1705 | CB | PHE | A | 257 | 0.415 | 6.935 | -17.004 | 1.00 | 33.47 | C |
| ATOM | 1706 | CG | PHE | A | 257 | -0.962 | 6.363 | -17.183 | 1.00 | 32.88 | C |
| ATOM | 1707 | CD1 | PHE | A | 257 | -1.468 | 6.094 | -18.454 | 1.00 | 33.61 | C |
| ATOM | 1708 | CD2 | PHE | A | 257 | -1.776 | 6.136 | -16.078 | 1.00 | 32.85 | C |
| ATOM | 1709 | CE1 | PHE | A | 257 | -2.767 | 5.608 | -18.625 | 1.00 | 33.37 | C |
| ATOM | 1710 | CE2 | PHE | A | 257 | -3.075 | 5.652 | -16.232 | 1.00 | 33.48 | C |
| ATOM | 1711 | CZ | PHE | A | 257 | -3.576 | 5.386 | -17.513 | 1.00 | 32.27 | C |
| ATOM | 1712 | C | PHE | A | 257 | 2.895 | 6.694 | -17.297 | 1.00 | 35.50 | C |
| ATOM | 1713 | O | PHE | A | 257 | 3.039 | 7.318 | -16.243 | 1.00 | 36.23 | O |
| ATOM | 1714 | N | PRO | A | 258 | 3.923 | 6.469 | -18.126 | 1.00 | 34.60 | N |
| ATOM | 1715 | CD | PRO | A | 258 | 3.931 | 5.598 | -19.320 | 1.00 | 35.11 | C |
| ATOM | 1716 | CA | PRO | A | 258 | 5.275 | 6.942 | -17.819 | 1.00 | 33.94 | C |
| ATOM | 1717 | CB | PRO | A | 258 | 6.150 | 5.942 | -18.570 | 1.00 | 34.28 | C |
| ATOM | 1718 | CG | PRO | A | 258 | 5.364 | 5.741 | -19.831 | 1.00 | 34.54 | C |
| ATOM | 1719 | C | PRO | A | 258 | 5.548 | 8.384 | -18.244 | 1.00 | 34.44 | C |
| ATOM | 1720 | O | PRO | A | 258 | 6.310 | 8.620 | -19.179 | 1.00 | 35.20 | O |
| ATOM | 1721 | N | GLY | A | 259 | 4.938 | 9.343 | -17.557 | 1.00 | 35.84 | N |
| ATOM | 1722 | CA | GLY | A | 259 | 5.150 | 10.737 | -17.900 | 1.00 | 37.50 | C |
| ATOM | 1723 | C | GLY | A | 259 | 6.544 | 11.224 | -17.549 | 1.00 | 38.70 | C |
| ATOM | 1724 | O | GLY | A | 259 | 7.129 | 10.785 | -16.557 | 1.00 | 40.91 | O |
| ATOM | 1725 | N | ASP | A | 260 | 7.094 | 12.138 | -18.341 | 1.00 | 38.35 | N |
| ATOM | 1726 | CA | ASP | A | 260 | 8.439 | 12.629 | -18.051 | 1.00 | 38.77 | C |
| ATOM | 1727 | CB | ASP | A | 260 | 9.254 | 12.783 | -19.339 | 1.00 | 38.24 | C |
| ATOM | 1728 | CG | ASP | A | 260 | 8.814 | 13.966 | -20.173 | 1.00 | 39.15 | C |
| ATOM | 1729 | OD1 | ASP | A | 260 | 7.989 | 14.761 | -19.681 | 1.00 | 40.79 | O |
| ATOM | 1730 | OD2 | ASP | A | 260 | 9.300 | 14.110 | -21.318 | 1.00 | 38.47 | O |
| ATOM | 1731 | C | ASP | A | 260 | 8.392 | 13.956 | -17.307 | 1.00 | 38.87 | C |
| ATOM | 1732 | O | ASP | A | 260 | 9.346 | 14.730 | -17.332 | 1.00 | 39.23 | O |
| ATOM | 1733 | N | SER | A | 261 | 7.269 | 14.219 | -16.651 | 1.00 | 38.30 | N |
| ATOM | 1734 | CA | SER | A | 261 | 7.089 | 15.456 | -15.887 | 1.00 | 37.63 | C |
| ATOM | 1735 | CB | SER | A | 261 | 7.314 | 16.686 | -16.776 | 1.00 | 37.36 | C |

| | | | | | | | | | | | |
|---|---|---|---|---|---|---|---|---|---|---|---|
| ATOM | 1736 | OG | SER | A | 261 | 6.101 | 17.395 | -16.984 | 1.00 | 38.61 | O |
| ATOM | 1737 | C | SER | A | 261 | 5.681 | 15.497 | -15.306 | 1.00 | 36.57 | C |
| ATOM | 1738 | O | SER | A | 261 | 4.803 | 14.749 | -15.734 | 1.00 | 35.60 | O |
| ATOM | 1739 | N | GLY | A | 262 | 5.486 | 16.376 | -14.328 | 1.00 | 36.31 | N |
| ATOM | 1740 | CA | GLY | A | 262 | 4.201 | 16.507 | -13.670 | 1.00 | 35.60 | C |
| ATOM | 1741 | C | GLY | A | 262 | 3.057 | 16.752 | -14.624 | 1.00 | 35.74 | C |
| ATOM | 1742 | O | GLY | A | 262 | 1.963 | 16.220 | -14.439 | 1.00 | 35.42 | O |
| ATOM | 1743 | N | VAL | A | 263 | 3.321 | 17.559 | -15.647 | 1.00 | 37.13 | N |
| ATOM | 1744 | CA | VAL | A | 263 | 2.329 | 17.905 | -16.662 | 1.00 | 37.49 | C |
| ATOM | 1745 | CB | VAL | A | 263 | 2.637 | 19.305 | -17.247 | 1.00 | 37.26 | C |
| ATOM | 1746 | CG1 | VAL | A | 263 | 1.677 | 19.633 | -18.379 | 1.00 | 35.65 | C |
| ATOM | 1747 | CG2 | VAL | A | 263 | 2.540 | 20.348 | -16.140 | 1.00 | 36.95 | C |
| ATOM | 1748 | C | VAL | A | 263 | 2.219 | 16.876 | -17.806 | 1.00 | 37.73 | C |
| ATOM | 1749 | O | VAL | A | 263 | 1.121 | 16.604 | -18.291 | 1.00 | 37.49 | O |
| ATOM | 1750 | N | ASP | A | 264 | 3.350 | 16.312 | -18.237 | 1.00 | 38.22 | N |
| ATOM | 1751 | CA | ASP | A | 264 | 3.350 | 15.314 | -19.315 | 1.00 | 37.72 | C |
| ATOM | 1752 | CB | ASP | A | 264 | 4.791 | 14.912 | -19.687 | 1.00 | 38.54 | C |
| ATOM | 1753 | CG | ASP | A | 264 | 4.866 | 13.974 | -20.910 | 1.00 | 40.71 | C |
| ATOM | 1754 | OD1 | ASP | A | 264 | 5.629 | 12.978 | -20.854 | 1.00 | 39.65 | O |
| ATOM | 1755 | OD2 | ASP | A | 264 | 4.188 | 14.237 | -21.930 | 1.00 | 40.32 | O |
| ATOM | 1756 | C | ASP | A | 264 | 2.578 | 14.089 | -18.830 | 1.00 | 37.96 | C |
| ATOM | 1757 | O | ASP | A | 264 | 1.984 | 13.362 | -19.629 | 1.00 | 38.23 | O |
| ATOM | 1758 | N | GLN | A | 265 | 2.591 | 13.866 | -17.516 | 1.00 | 36.44 | N |
| ATOM | 1759 | CA | GLN | A | 265 | 1.886 | 12.736 | -16.928 | 1.00 | 35.92 | C |
| ATOM | 1760 | CB | GLN | A | 265 | 1.884 | 12.839 | -15.402 | 1.00 | 34.28 | C |
| ATOM | 1761 | CG | GLN | A | 265 | 1.224 | 11.665 | -14.681 | 1.00 | 31.51 | C |
| ATOM | 1762 | CD | GLN | A | 265 | 1.858 | 10.329 | -15.022 | 1.00 | 30.33 | C |
| ATOM | 1763 | OE1 | GLN | A | 265 | 3.017 | 10.270 | -15.422 | 1.00 | 29.39 | O |
| ATOM | 1764 | NE2 | GLN | A | 265 | 1.104 | 9.251 | -14.852 | 1.00 | 27.96 | N |
| ATOM | 1765 | C | GLN | A | 265 | 0.452 | 12.715 | -17.432 | 1.00 | 36.53 | C |
| ATOM | 1766 | O | GLN | A | 265 | -0.083 | 11.660 | -17.765 | 1.00 | 38.09 | O |
| ATOM | 1767 | N | LEU | A | 266 | -0.172 | 13.884 | -17.488 | 1.00 | 36.46 | N |
| ATOM | 1768 | CA | LEU | A | 266 | -1.541 | 13.973 | -17.962 | 1.00 | 36.71 | C |
| ATOM | 1769 | CB | LEU | A | 266 | -2.056 | 15.407 | -17.814 | 1.00 | 37.12 | C |
| ATOM | 1770 | CG | LEU | A | 266 | -3.538 | 15.664 | -18.119 | 1.00 | 37.80 | C |
| ATOM | 1771 | CD1 | LEU | A | 266 | -4.408 | 14.812 | -17.208 | 1.00 | 37.31 | C |
| ATOM | 1772 | CD2 | LEU | A | 266 | -3.848 | 17.144 | -17.933 | 1.00 | 36.02 | C |
| ATOM | 1773 | C | LEU | A | 266 | -1.580 | 13.545 | -19.426 | 1.00 | 36.95 | C |
| ATOM | 1774 | O | LEU | A | 266 | -2.421 | 12.736 | -19.832 | 1.00 | 37.49 | O |
| ATOM | 1775 | N | VAL | A | 267 | -0.656 | 14.086 | -20.211 | 1.00 | 35.66 | N |
| ATOM | 1776 | CA | VAL | A | 267 | -0.585 | 13.765 | -21.623 | 1.00 | 35.47 | C |
| ATOM | 1777 | CB | VAL | A | 267 | 0.640 | 14.415 | -22.262 | 1.00 | 33.70 | C |
| ATOM | 1778 | CG1 | VAL | A | 267 | 0.717 | 14.045 | -23.732 | 1.00 | 33.15 | C |
| ATOM | 1779 | CG2 | VAL | A | 267 | 0.563 | 15.911 | -22.082 | 1.00 | 32.27 | C |
| ATOM | 1780 | C | VAL | A | 267 | -0.537 | 12.254 | -21.851 | 1.00 | 37.34 | C |
| ATOM | 1781 | O | VAL | A | 267 | -1.236 | 11.718 | -22.718 | 1.00 | 38.55 | O |
| ATOM | 1782 | N | GLU | A | 268 | 0.281 | 11.564 | -21.065 | 1.00 | 37.07 | N |
| ATOM | 1783 | CA | GLU | A | 268 | 0.402 | 10.120 | -21.180 | 1.00 | 37.46 | C |
| ATOM | 1784 | CB | GLU | A | 268 | 1.543 | 9.633 | -20.287 | 1.00 | 37.36 | C |
| ATOM | 1785 | CG | GLU | A | 268 | 2.924 | 9.971 | -20.829 | 1.00 | 36.52 | C |
| ATOM | 1786 | CD | GLU | A | 268 | 3.279 | 9.133 | -22.038 | 1.00 | 35.99 | C |
| ATOM | 1787 | OE1 | GLU | A | 268 | 3.205 | 7.898 | -21.925 | 1.00 | 36.05 | O |
| ATOM | 1788 | OE2 | GLU | A | 268 | 3.626 | 9.699 | -23.091 | 1.00 | 36.19 | O |
| ATOM | 1789 | C | GLU | A | 268 | -0.907 | 9.413 | -20.809 | 1.00 | 37.04 | C |
| ATOM | 1790 | O | GLU | A | 268 | -1.305 | 8.449 | -21.459 | 1.00 | 38.09 | O |
| ATOM | 1791 | N | ILE | A | 269 | -1.569 | 9.910 | -19.770 | 1.00 | 37.52 | N |
| ATOM | 1792 | CA | ILE | A | 269 | -2.834 | 9.356 | -19.296 | 1.00 | 37.73 | C |
| ATOM | 1793 | CB | ILE | A | 269 | -3.309 | 10.096 | -18.007 | 1.00 | 36.56 | C |
| ATOM | 1794 | CG2 | ILE | A | 269 | -4.828 | 10.031 | -17.882 | 1.00 | 34.04 | C |
| ATOM | 1795 | CG1 | ILE | A | 269 | -2.607 | 9.503 | -16.778 | 1.00 | 36.18 | C |
| ATOM | 1796 | CD1 | ILE | A | 269 | -2.814 | 10.290 | -15.489 | 1.00 | 34.76 | C |
| ATOM | 1797 | C | ILE | A | 269 | -3.896 | 9.513 | -20.377 | 1.00 | 38.54 | C |
| ATOM | 1798 | O | ILE | A | 269 | -4.722 | 8.622 | -20.602 | 1.00 | 38.60 | O |
| ATOM | 1799 | N | ILE | A | 270 | -3.849 | 10.660 | -21.044 | 1.00 | 38.32 | N |
| ATOM | 1800 | CA | ILE | A | 270 | -4.781 | 11.001 | -22.104 | 1.00 | 39.17 | C |
| ATOM | 1801 | CB | ILE | A | 270 | -4.641 | 12.513 | -22.445 | 1.00 | 39.97 | C |
| ATOM | 1802 | CG2 | ILE | A | 270 | -5.331 | 12.835 | -23.755 | 1.00 | 38.20 | C |

| | | | | | | | | | | | |
|---|---|---|---|---|---|---|---|---|---|---|---|
| ATOM | 1803 | CG1 | ILE | A | 270 | -5.208 | 13.348 | -21.278 | 1.00 | 39.82 | C |
| ATOM | 1804 | CD1 | ILE | A | 270 | -4.963 | 14.850 | -21.385 | 1.00 | 38.73 | C |
| ATOM | 1805 | C | ILE | A | 270 | -4.585 | 10.125 | -23.349 | 1.00 | 39.59 | C |
| ATOM | 1806 | O | ILE | A | 270 | -5.536 | 9.848 | -24.082 | 1.00 | 38.97 | O |
| ATOM | 1807 | N | LYS | A | 271 | -3.357 | 9.663 | -23.570 | 1.00 | 40.90 | N |
| ATOM | 1808 | CA | LYS | A | 271 | -3.068 | 8.813 | -24.721 | 1.00 | 40.64 | C |
| ATOM | 1809 | CB | LYS | A | 271 | -1.568 | 8.512 | -24.787 | 1.00 | 40.13 | C |
| ATOM | 1810 | CG | LYS | A | 271 | -0.748 | 9.757 | -25.090 | 1.00 | 42.58 | C |
| ATOM | 1811 | CD | LYS | A | 271 | 0.737 | 9.554 | -24.909 | 1.00 | 42.52 | C |
| ATOM | 1812 | CE | LYS | A | 271 | 1.337 | 8.732 | -26.016 | 1.00 | 44.52 | C |
| ATOM | 1813 | NZ | LYS | A | 271 | 2.826 | 8.762 | -25.924 | 1.00 | 46.88 | N |
| ATOM | 1814 | C | LYS | A | 271 | -3.896 | 7.524 | -24.722 | 1.00 | 40.51 | C |
| ATOM | 1815 | O | LYS | A | 271 | -4.163 | 6.968 | -25.787 | 1.00 | 41.45 | O |
| ATOM | 1816 | N | VAL | A | 272 | -4.309 | 7.045 | -23.549 | 1.00 | 38.88 | N |
| ATOM | 1817 | CA | VAL | A | 272 | -5.135 | 5.842 | -23.523 | 1.00 | 37.64 | C |
| ATOM | 1818 | CB | VAL | A | 272 | -4.547 | 4.697 | -22.608 | 1.00 | 36.00 | C |
| ATOM | 1819 | CG1 | VAL | A | 272 | -3.132 | 5.021 | -22.181 | 1.00 | 34.52 | C |
| ATOM | 1820 | CG2 | VAL | A | 272 | -5.456 | 4.435 | -21.431 | 1.00 | 34.80 | C |
| ATOM | 1821 | C | VAL | A | 272 | -6.586 | 6.138 | -23.125 | 1.00 | 38.01 | C |
| ATOM | 1822 | O | VAL | A | 272 | -7.507 | 5.673 | -23.796 | 1.00 | 40.13 | O |
| ATOM | 1823 | N | LEU | A | 273 | -6.807 | 6.904 | -22.059 | 1.00 | 37.99 | N |
| ATOM | 1824 | CA | LEU | A | 273 | -8.179 | 7.217 | -21.655 | 1.00 | 38.16 | C |
| ATOM | 1825 | CB | LEU | A | 273 | -8.240 | 7.773 | -20.224 | 1.00 | 38.70 | C |
| ATOM | 1826 | CG | LEU | A | 273 | -7.757 | 6.955 | -19.018 | 1.00 | 38.44 | C |
| ATOM | 1827 | CD1 | LEU | A | 273 | -8.285 | 7.589 | -17.741 | 1.00 | 35.39 | C |
| ATOM | 1828 | CD2 | LEU | A | 273 | -8.238 | 5.519 | -19.124 | 1.00 | 37.96 | C |
| ATOM | 1829 | C | LEU | A | 273 | -8.769 | 8.255 | -22.602 | 1.00 | 38.31 | C |
| ATOM | 1830 | O | LEU | A | 273 | -9.983 | 8.459 | -22.644 | 1.00 | 38.49 | O |
| ATOM | 1831 | N | GLY | A | 274 | -7.901 | 8.908 | -23.365 | 1.00 | 38.28 | N |
| ATOM | 1832 | CA | GLY | A | 274 | -8.360 | 9.927 | -24.289 | 1.00 | 39.28 | C |
| ATOM | 1833 | C | GLY | A | 274 | -8.656 | 11.203 | -23.528 | 1.00 | 40.29 | C |
| ATOM | 1834 | O | GLY | A | 274 | -8.540 | 11.234 | -22.304 | 1.00 | 40.45 | O |
| ATOM | 1835 | N | THR | A | 275 | -9.038 | 12.256 | -24.244 | 1.00 | 40.13 | N |
| ATOM | 1836 | CA | THR | A | 275 | -9.343 | 13.533 | -23.616 | 1.00 | 39.40 | C |
| ATOM | 1837 | CB | THR | A | 275 | -9.613 | 14.613 | -24.667 | 1.00 | 38.37 | C |
| ATOM | 1838 | OG1 | THR | A | 275 | -8.432 | 14.824 | -25.445 | 1.00 | 38.15 | O |
| ATOM | 1839 | CG2 | THR | A | 275 | -10.003 | 15.920 | -23.999 | 1.00 | 40.01 | C |
| ATOM | 1840 | C | THR | A | 275 | -10.534 | 13.460 | -22.669 | 1.00 | 39.43 | C |
| ATOM | 1841 | O | THR | A | 275 | -11.481 | 12.706 | -22.896 | 1.00 | 40.48 | O |
| ATOM | 1842 | N | PRO | A | 276 | -10.478 | 14.224 | -21.567 | 1.00 | 39.15 | N |
| ATOM | 1843 | CD | PRO | A | 276 | -9.292 | 14.916 | -21.028 | 1.00 | 36.87 | C |
| ATOM | 1844 | CA | PRO | A | 276 | -11.571 | 14.236 | -20.593 | 1.00 | 39.18 | C |
| ATOM | 1845 | CB | PRO | A | 276 | -10.936 | 14.876 | -19.356 | 1.00 | 38.59 | C |
| ATOM | 1846 | CG | PRO | A | 276 | -9.462 | 14.719 | -19.564 | 1.00 | 37.40 | C |
| ATOM | 1847 | C | PRO | A | 276 | -12.730 | 15.083 | -21.113 | 1.00 | 40.09 | C |
| ATOM | 1848 | O | PRO | A | 276 | -12.521 | 16.085 | -21.812 | 1.00 | 40.48 | O |
| ATOM | 1849 | N | THR | A | 277 | -13.951 | 14.682 | -20.782 | 1.00 | 40.81 | N |
| ATOM | 1850 | CA | THR | A | 277 | -15.129 | 15.434 | -21.193 | 1.00 | 40.34 | C |
| ATOM | 1851 | CB | THR | A | 277 | -16.422 | 14.621 | -20.978 | 1.00 | 39.83 | C |
| ATOM | 1852 | OG1 | THR | A | 277 | -16.635 | 14.400 | -19.576 | 1.00 | 39.79 | O |
| ATOM | 1853 | CG2 | THR | A | 277 | -16.318 | 13.272 | -21.677 | 1.00 | 39.70 | C |
| ATOM | 1854 | C | THR | A | 277 | -15.151 | 16.641 | -20.277 | 1.00 | 41.16 | C |
| ATOM | 1855 | O | THR | A | 277 | -14.802 | 16.533 | -19.107 | 1.00 | 41.62 | O |
| ATOM | 1856 | N | ARG | A | 278 | -15.539 | 17.791 | -20.808 | 1.00 | 42.24 | N |
| ATOM | 1857 | CA | ARG | A | 278 | -15.580 | 19.001 | -20.004 | 1.00 | 42.08 | C |
| ATOM | 1858 | CB | ARG | A | 278 | -16.412 | 20.063 | -20.704 | 1.00 | 43.22 | C |
| ATOM | 1859 | CG | ARG | A | 278 | -16.829 | 21.205 | -19.784 | 1.00 | 45.59 | C |
| ATOM | 1860 | CD | ARG | A | 278 | -18.085 | 21.873 | -20.331 | 1.00 | 46.66 | C |
| ATOM | 1861 | NE | ARG | A | 278 | -17.938 | 22.214 | -21.753 | 1.00 | 43.99 | N |
| ATOM | 1862 | CZ | ARG | A | 278 | -18.950 | 22.366 | -22.602 | 1.00 | 42.69 | C |
| ATOM | 1863 | NH1 | ARG | A | 278 | -20.209 | 22.211 | -22.203 | 1.00 | 40.99 | N |
| ATOM | 1864 | NH2 | ARG | A | 278 | -18.694 | 22.675 | -23.856 | 1.00 | 44.00 | N |
| ATOM | 1865 | C | ARG | A | 278 | -16.176 | 18.711 | -18.632 | 1.00 | 42.22 | C |
| ATOM | 1866 | O | ARG | A | 278 | -15.802 | 19.333 | -17.632 | 1.00 | 41.88 | O |
| ATOM | 1867 | N | GLU | A | 279 | -17.109 | 17.765 | -18.585 | 1.00 | 42.32 | N |
| ATOM | 1868 | CA | GLU | A | 279 | -17.746 | 17.398 | -17.323 | 1.00 | 42.46 | C |
| ATOM | 1869 | CB | GLU | A | 279 | -18.976 | 16.523 | -17.577 | 1.00 | 42.65 | C |

| | | | | | | | | | | | |
|---|---|---|---|---|---|---|---|---|---|---|---|
| ATOM | 1870 | CG | GLU | A | 279 | -20.223 | 17.338 | -17.880 | 1.00 | 44.20 | C |
| ATOM | 1871 | CD | GLU | A | 279 | -20.191 | 17.984 | -19.251 | 1.00 | 44.08 | C |
| ATOM | 1872 | OE1 | GLU | A | 279 | -20.630 | 19.149 | -19.366 | 1.00 | 45.25 | O |
| ATOM | 1873 | OE2 | GLU | A | 279 | -19.740 | 17.318 | -20.208 | 1.00 | 43.79 | O |
| ATOM | 1874 | C | GLU | A | 279 | -16.777 | 16.679 | -16.390 | 1.00 | 42.89 | C |
| ATOM | 1875 | O | GLU | A | 279 | -16.721 | 16.964 | -15.183 | 1.00 | 42.65 | O |
| ATOM | 1876 | N | GLN | A | 280 | -16.017 | 15.742 | -16.950 | 1.00 | 41.71 | N |
| ATOM | 1877 | CA | GLN | A | 280 | -15.035 | 15.002 | -16.166 | 1.00 | 41.80 | C |
| ATOM | 1878 | CB | GLN | A | 280 | -14.256 | 14.021 | -17.063 | 1.00 | 42.03 | C |
| ATOM | 1879 | CG | GLN | A | 280 | -14.986 | 12.704 | -17.349 | 1.00 | 41.17 | C |
| ATOM | 1880 | CD | GLN | A | 280 | -14.402 | 11.958 | -18.542 | 1.00 | 42.14 | C |
| ATOM | 1881 | OE1 | GLN | A | 280 | -14.647 | 10.762 | -18.734 | 1.00 | 43.58 | O |
| ATOM | 1882 | NE2 | GLN | A | 280 | -13.633 | 12.666 | -19.359 | 1.00 | 41.52 | N |
| ATOM | 1883 | C | GLN | A | 280 | -14.079 | 16.004 | -15.504 | 1.00 | 42.21 | C |
| ATOM | 1884 | O | GLN | A | 280 | -13.876 | 15.986 | -14.279 | 1.00 | 42.26 | O |
| ATOM | 1885 | N | ILE | A | 281 | -13.505 | 16.885 | -16.319 | 1.00 | 41.36 | N |
| ATOM | 1886 | CA | ILE | A | 281 | -12.582 | 17.908 | -15.832 | 1.00 | 40.13 | C |
| ATOM | 1887 | CB | ILE | A | 281 | -12.253 | 18.926 | -16.943 | 1.00 | 36.59 | C |
| ATOM | 1888 | CG2 | ILE | A | 281 | -11.573 | 20.135 | -16.354 | 1.00 | 36.78 | C |
| ATOM | 1889 | CG1 | ILE | A | 281 | -11.368 | 18.276 | -18.003 | 1.00 | 34.62 | C |
| ATOM | 1890 | CD1 | ILE | A | 281 | -11.129 | 19.152 | -19.205 | 1.00 | 31.10 | C |
| ATOM | 1891 | C | ILE | A | 281 | -13.135 | 18.660 | -14.621 | 1.00 | 41.45 | C |
| ATOM | 1892 | O | ILE | A | 281 | -12.388 | 19.023 | -13.710 | 1.00 | 41.98 | O |
| ATOM | 1893 | N | ALA | A | 282 | -14.445 | 18.884 | -14.607 | 1.00 | 43.17 | N |
| ATOM | 1894 | CA | ALA | A | 282 | -15.071 | 19.600 | -13.503 | 1.00 | 44.25 | C |
| ATOM | 1895 | CB | ALA | A | 282 | -16.462 | 20.074 | -13.908 | 1.00 | 43.41 | C |
| ATOM | 1896 | C | ALA | A | 282 | -15.140 | 18.772 | -12.221 | 1.00 | 44.67 | C |
| ATOM | 1897 | O | ALA | A | 282 | -14.983 | 19.311 | -11.124 | 1.00 | 45.75 | O |
| ATOM | 1898 | N | GLU | A | 283 | -15.363 | 17.466 | -12.351 | 1.00 | 45.28 | N |
| ATOM | 1899 | CA | GLU | A | 283 | -15.451 | 16.591 | -11.174 | 1.00 | 46.37 | C |
| ATOM | 1900 | CB | GLU | A | 283 | -16.095 | 15.249 | -11.556 | 1.00 | 45.36 | C |
| ATOM | 1901 | CG | GLU | A | 283 | -17.534 | 15.386 | -12.051 | 1.00 | 45.14 | C |
| ATOM | 1902 | CD | GLU | A | 283 | -17.877 | 14.415 | -13.181 | 1.00 | 43.43 | C |
| ATOM | 1903 | OE1 | GLU | A | 283 | -16.988 | 14.110 | -14.002 | 1.00 | 42.39 | O |
| ATOM | 1904 | OE2 | GLU | A | 283 | -19.040 | 13.976 | -13.260 | 1.00 | 42.18 | O |
| ATOM | 1905 | C | GLU | A | 283 | -14.084 | 16.354 | -10.518 | 1.00 | 47.49 | C |
| ATOM | 1906 | O | GLU | A | 283 | -14.011 | 15.926 | -9.362 | 1.00 | 46.50 | O |
| ATOM | 1907 | N | MET | A | 284 | -13.014 | 16.640 | -11.263 | 1.00 | 49.33 | N |
| ATOM | 1908 | CA | MET | A | 284 | -11.643 | 16.477 | -10.786 | 1.00 | 50.75 | C |
| ATOM | 1909 | CB | MET | A | 284 | -10.692 | 16.260 | -11.968 | 1.00 | 51.56 | C |
| ATOM | 1910 | CG | MET | A | 284 | -11.121 | 15.182 | -12.960 | 1.00 | 50.19 | C |
| ATOM | 1911 | SD | MET | A | 284 | -9.897 | 14.944 | -14.293 | 1.00 | 48.52 | S |
| ATOM | 1912 | CE | MET | A | 284 | -9.278 | 13.411 | -13.820 | 1.00 | 46.37 | C |
| ATOM | 1913 | C | MET | A | 284 | -11.177 | 17.704 | -10.015 | 1.00 | 51.97 | C |
| ATOM | 1914 | O | MET | A | 284 | -10.594 | 17.589 | -8.939 | 1.00 | 51.56 | O |
| ATOM | 1915 | N | ASN | A | 285 | -11.431 | 18.873 | -10.595 | 1.00 | 54.46 | N |
| ATOM | 1916 | CA | ASN | A | 285 | -11.048 | 20.155 | -10.015 | 1.00 | 57.00 | C |
| ATOM | 1917 | CB | ASN | A | 285 | -9.565 | 20.420 | -10.266 | 1.00 | 57.60 | C |
| ATOM | 1918 | CG | ASN | A | 285 | -9.153 | 21.819 | -9.868 | 1.00 | 57.50 | C |
| ATOM | 1919 | OD1 | ASN | A | 285 | -9.503 | 22.290 | -8.789 | 1.00 | 57.91 | O |
| ATOM | 1920 | ND2 | ASN | A | 285 | -8.398 | 22.490 | -10.733 | 1.00 | 58.00 | N |
| ATOM | 1921 | C | ASN | A | 285 | -11.876 | 21.213 | -10.721 | 1.00 | 58.46 | C |
| ATOM | 1922 | O | ASN | A | 285 | -11.482 | 21.723 | -11.775 | 1.00 | 58.76 | O |
| ATOM | 1923 | N | PRO | A | 286 | -13.037 | 21.563 | -10.150 | 1.00 | 60.34 | N |
| ATOM | 1924 | CD | PRO | A | 286 | -13.515 | 21.173 | -8.814 | 1.00 | 60.68 | C |
| ATOM | 1925 | CA | PRO | A | 286 | -13.914 | 22.570 | -10.764 | 1.00 | 62.52 | C |
| ATOM | 1926 | CB | PRO | A | 286 | -14.913 | 22.916 | -9.647 | 1.00 | 61.75 | C |
| ATOM | 1927 | CG | PRO | A | 286 | -14.277 | 22.389 | -8.390 | 1.00 | 61.21 | C |
| ATOM | 1928 | C | PRO | A | 286 | -13.224 | 23.799 | -11.361 | 1.00 | 64.16 | C |
| ATOM | 1929 | O | PRO | A | 286 | -13.334 | 24.060 | -12.561 | 1.00 | 64.96 | O |
| ATOM | 1930 | N | ASN | A | 287 | -12.509 | 24.536 | -10.519 | 1.00 | 64.92 | N |
| ATOM | 1931 | CA | ASN | A | 287 | -11.788 | 25.751 | -10.907 | 1.00 | 65.00 | C |
| ATOM | 1932 | CB | ASN | A | 287 | -10.999 | 26.256 | -9.702 | 1.00 | 65.58 | C |
| ATOM | 1933 | CG | ASN | A | 287 | -11.896 | 26.611 | -8.535 | 1.00 | 66.70 | C |
| ATOM | 1934 | OD1 | ASN | A | 287 | -12.616 | 27.610 | -8.587 | 1.00 | 67.12 | O |
| ATOM | 1935 | ND2 | ASN | A | 287 | -11.870 | 25.788 | -7.477 | 1.00 | 66.16 | N |
| ATOM | 1936 | C | ASN | A | 287 | -10.858 | 25.616 | -12.106 | 1.00 | 65.25 | C |

| | | | | | | | | | | | |
|---|---|---|---|---|---|---|---|---|---|---|---|
| ATOM | 1937 | O | ASN | A | 287 | -11.212 | 25.013 | -13.121 | 1.00 | 65.40 | O |
| ATOM | 1938 | N | ALA | A | 294 | -6.951 | 19.797 | -26.820 | 1.00 | 58.74 | N |
| ATOM | 1939 | CA | ALA | A | 294 | -6.306 | 18.818 | -27.690 | 1.00 | 59.85 | C |
| ATOM | 1940 | CB | ALA | A | 294 | -4.889 | 18.526 | -27.195 | 1.00 | 59.11 | C |
| ATOM | 1941 | C | ALA | A | 294 | -7.133 | 17.542 | -27.682 | 1.00 | 60.39 | C |
| ATOM | 1942 | O | ALA | A | 294 | -7.041 | 16.738 | -26.748 | 1.00 | 60.91 | O |
| ATOM | 1943 | N | ALA | A | 295 | -7.930 | 17.342 | -28.725 | 1.00 | 60.05 | N |
| ATOM | 1944 | CA | ALA | A | 295 | -8.790 | 16.164 | -28.788 | 1.00 | 60.52 | C |
| ATOM | 1945 | CB | ALA | A | 295 | -10.035 | 16.491 | -29.606 | 1.00 | 60.07 | C |
| ATOM | 1946 | C | ALA | A | 295 | -8.158 | 14.864 | -29.293 | 1.00 | 60.27 | C |
| ATOM | 1947 | O | ALA | A | 295 | -7.881 | 14.711 | -30.484 | 1.00 | 60.00 | O |
| ATOM | 1948 | N | ALA | A | 296 | -7.937 | 13.935 | -28.364 | 1.00 | 59.99 | N |
| ATOM | 1949 | CA | ALA | A | 296 | -7.368 | 12.614 | -28.660 | 1.00 | 59.12 | C |
| ATOM | 1950 | CB | ALA | A | 296 | -6.144 | 12.336 | -27.764 | 1.00 | 58.66 | C |
| ATOM | 1951 | C | ALA | A | 296 | -8.478 | 11.566 | -28.420 | 1.00 | 58.35 | C |
| ATOM | 1952 | O | ALA | A | 296 | -9.288 | 11.691 | -27.497 | 1.00 | 56.79 | O |
| ATOM | 1953 | N | ALA | A | 297 | -8.474 | 10.527 | -29.252 | 1.00 | 57.71 | N |
| ATOM | 1954 | CA | ALA | A | 297 | -9.481 | 9.465 | -29.269 | 1.00 | 56.86 | C |
| ATOM | 1955 | CB | ALA | A | 297 | -9.152 | 8.526 | -30.397 | 1.00 | 56.58 | C |
| ATOM | 1956 | C | ALA | A | 297 | -9.913 | 8.624 | -28.065 | 1.00 | 56.40 | C |
| ATOM | 1957 | O | ALA | A | 297 | -11.097 | 8.628 | -27.720 | 1.00 | 57.31 | O |
| ATOM | 1958 | N | ALA | A | 298 | -8.987 | 7.869 | -27.479 | 1.00 | 55.04 | N |
| ATOM | 1959 | CA | ALA | A | 298 | -9.261 | 6.962 | -26.357 | 1.00 | 55.07 | C |
| ATOM | 1960 | CB | ALA | A | 298 | -10.693 | 7.095 | -25.861 | 1.00 | 54.15 | C |
| ATOM | 1961 | C | ALA | A | 298 | -9.045 | 5.579 | -26.935 | 1.00 | 55.65 | C |
| ATOM | 1962 | O | ALA | A | 298 | -9.915 | 5.027 | -27.611 | 1.00 | 55.58 | O |
| ATOM | 1963 | N | HIS | A | 299 | -7.859 | 5.041 | -26.693 | 1.00 | 56.14 | N |
| ATOM | 1964 | CA | HIS | A | 299 | -7.483 | 3.726 | -27.181 | 1.00 | 56.37 | C |
| ATOM | 1965 | CB | HIS | A | 299 | -6.024 | 3.480 | -26.810 | 1.00 | 57.10 | C |
| ATOM | 1966 | CG | HIS | A | 299 | -5.356 | 2.421 | -27.620 | 1.00 | 58.88 | C |
| ATOM | 1967 | CD2 | HIS | A | 299 | -4.836 | 2.446 | -28.873 | 1.00 | 58.97 | C |
| ATOM | 1968 | ND1 | HIS | A | 299 | -5.083 | 1.160 | -27.123 | 1.00 | 59.09 | N |
| ATOM | 1969 | CE1 | HIS | A | 299 | -4.421 | 0.464 | -28.025 | 1.00 | 59.40 | C |
| ATOM | 1970 | NE2 | HIS | A | 299 | -4.257 | 1.223 | -29.102 | 1.00 | 59.62 | N |
| ATOM | 1971 | C | HIS | A | 299 | -8.397 | 2.753 | -26.445 | 1.00 | 56.28 | C |
| ATOM | 1972 | O | HIS | A | 299 | -8.802 | 3.023 | -25.318 | 1.00 | 56.07 | O |
| ATOM | 1973 | N | PRO | A | 300 | -8.791 | 1.642 | -27.083 | 1.00 | 56.54 | N |
| ATOM | 1974 | CD | PRO | A | 300 | -8.835 | 1.298 | -28.514 | 1.00 | 56.29 | C |
| ATOM | 1975 | CA | PRO | A | 300 | -9.662 | 0.754 | -26.302 | 1.00 | 55.90 | C |
| ATOM | 1976 | CB | PRO | A | 300 | -10.464 | 0.005 | -27.372 | 1.00 | 56.00 | C |
| ATOM | 1977 | CG | PRO | A | 300 | -9.524 | -0.067 | -28.497 | 1.00 | 56.78 | C |
| ATOM | 1978 | C | PRO | A | 300 | -8.833 | -0.161 | -25.415 | 1.00 | 55.04 | C |
| ATOM | 1979 | O | PRO | A | 300 | -7.791 | -0.658 | -25.816 | 1.00 | 54.34 | O |
| ATOM | 1980 | N | TRP | A | 301 | -9.317 | -0.369 | -24.197 | 1.00 | 55.55 | N |
| ATOM | 1981 | CA | TRP | A | 301 | -8.649 | -1.186 | -23.193 | 1.00 | 54.35 | C |
| ATOM | 1982 | CB | TRP | A | 301 | -9.612 | -1.464 | -22.048 | 1.00 | 53.10 | C |
| ATOM | 1983 | CG | TRP | A | 301 | -9.817 | -0.262 | -21.215 | 1.00 | 53.08 | C |
| ATOM | 1984 | CD2 | TRP | A | 301 | -8.841 | 0.347 | -20.363 | 1.00 | 53.35 | C |
| ATOM | 1985 | CE2 | TRP | A | 301 | -9.448 | 1.488 | -19.789 | 1.00 | 53.53 | C |
| ATOM | 1986 | CE3 | TRP | A | 301 | -7.517 | 0.039 | -20.031 | 1.00 | 52.99 | C |
| ATOM | 1987 | CD1 | TRP | A | 301 | -10.938 | 0.503 | -21.131 | 1.00 | 53.76 | C |
| ATOM | 1988 | NE1 | TRP | A | 301 | -10.724 | 1.563 | -20.271 | 1.00 | 54.34 | N |
| ATOM | 1989 | CZ2 | TRP | A | 301 | -8.765 | 2.319 | -18.897 | 1.00 | 53.27 | C |
| ATOM | 1990 | CZ3 | TRP | A | 301 | -6.840 | 0.865 | -19.145 | 1.00 | 52.02 | C |
| ATOM | 1991 | CH2 | TRP | A | 301 | -7.465 | 1.992 | -18.587 | 1.00 | 52.23 | C |
| ATOM | 1992 | C | TRP | A | 301 | -8.024 | -2.484 | -23.661 | 1.00 | 55.61 | C |
| ATOM | 1993 | O | TRP | A | 301 | -6.807 | -2.668 | -23.556 | 1.00 | 56.30 | O |
| ATOM | 1994 | N | THR | A | 302 | -8.848 | -3.396 | -24.158 | 1.00 | 55.98 | N |
| ATOM | 1995 | CA | THR | A | 302 | -8.357 | -4.690 | -24.636 | 1.00 | 55.78 | C |
| ATOM | 1996 | CB | THR | A | 302 | -9.431 | -5.400 | -25.463 | 1.00 | 56.35 | C |
| ATOM | 1997 | OG1 | THR | A | 302 | -9.720 | -4.615 | -26.632 | 1.00 | 56.40 | O |
| ATOM | 1998 | CG2 | THR | A | 302 | -10.702 | -5.573 | -24.649 | 1.00 | 55.35 | C |
| ATOM | 1999 | C | THR | A | 302 | -7.111 | -4.538 | -25.510 | 1.00 | 54.98 | C |
| ATOM | 2000 | O | THR | A | 302 | -6.204 | -5.370 | -25.465 | 1.00 | 54.78 | O |
| ATOM | 2001 | N | ALA | A | 303 | -7.067 | -3.467 | -26.297 | 1.00 | 53.87 | N |
| ATOM | 2002 | CA | ALA | A | 303 | -5.927 | -3.219 | -27.177 | 1.00 | 53.50 | C |
| ATOM | 2003 | CB | ALA | A | 303 | -6.330 | -2.230 | -28.277 | 1.00 | 53.73 | C |

| | | | | | | | | | | | |
|---|---|---|---|---|---|---|---|---|---|---|---|
| ATOM | 2004 | C | ALA | A | 303 | -4.687 | -2.708 | -26.438 | 1.00 | 53.07 | C |
| ATOM | 2005 | O | ALA | A | 303 | -3.573 | -2.786 | -26.954 | 1.00 | 52.45 | O |
| ATOM | 2006 | N | VAL | A | 304 | -4.889 | -2.182 | -25.233 | 1.00 | 52.82 | N |
| ATOM | 2007 | CA | VAL | A | 304 | -3.799 | -1.653 | -24.417 | 1.00 | 51.75 | C |
| ATOM | 2008 | CB | VAL | A | 304 | -4.335 | -0.685 | -23.332 | 1.00 | 51.51 | C |
| ATOM | 2009 | CG1 | VAL | A | 304 | -3.235 | -0.336 | -22.336 | 1.00 | 50.47 | C |
| ATOM | 2010 | CG2 | VAL | A | 304 | -4.869 | 0.575 | -23.991 | 1.00 | 51.94 | C |
| ATOM | 2011 | C | VAL | A | 304 | -2.999 | -2.749 | -23.735 | 1.00 | 52.13 | C |
| ATOM | 2012 | O | VAL | A | 304 | -1.770 | -2.686 | -23.673 | 1.00 | 51.62 | O |
| ATOM | 2013 | N | PHE | A | 305 | -3.695 | -3.749 | -23.212 | 1.00 | 52.84 | N |
| ATOM | 2014 | CA | PHE | A | 305 | -3.024 | -4.847 | -22.535 | 1.00 | 54.26 | C |
| ATOM | 2015 | CB | PHE | A | 305 | -3.991 | -5.491 | -21.547 | 1.00 | 53.32 | C |
| ATOM | 2016 | CG | PHE | A | 305 | -4.415 | -4.542 | -20.464 | 1.00 | 53.64 | C |
| ATOM | 2017 | CD1 | PHE | A | 305 | -3.547 | -4.236 | -19.423 | 1.00 | 52.52 | C |
| ATOM | 2018 | CD2 | PHE | A | 305 | -5.615 | -3.848 | -20.555 | 1.00 | 52.90 | C |
| ATOM | 2019 | CE1 | PHE | A | 305 | -3.855 | -3.248 | -18.491 | 1.00 | 51.69 | C |
| ATOM | 2020 | CE2 | PHE | A | 305 | -5.937 | -2.857 | -19.628 | 1.00 | 52.96 | C |
| ATOM | 2021 | CZ | PHE | A | 305 | -5.054 | -2.551 | -18.591 | 1.00 | 51.62 | C |
| ATOM | 2022 | C | PHE | A | 305 | -2.466 | -5.835 | -23.543 | 1.00 | 56.01 | C |
| ATOM | 2023 | O | PHE | A | 305 | -2.757 | -5.745 | -24.736 | 1.00 | 56.75 | O |
| ATOM | 2024 | N | ARG | A | 306 | -1.646 | -6.768 | -23.083 | 1.00 | 57.62 | N |
| ATOM | 2025 | CA | ARG | A | 306 | -1.041 | -7.708 | -24.008 | 1.00 | 58.81 | C |
| ATOM | 2026 | CB | ARG | A | 306 | 0.166 | -8.339 | -23.323 | 1.00 | 60.98 | C |
| ATOM | 2027 | CG | ARG | A | 306 | 1.068 | -7.200 | -22.794 | 1.00 | 63.97 | C |
| ATOM | 2028 | CD | ARG | A | 306 | 2.446 | -7.645 | -22.341 | 1.00 | 65.88 | C |
| ATOM | 2029 | NE | ARG | A | 306 | 2.354 | -8.708 | -21.353 | 1.00 | 68.68 | N |
| ATOM | 2030 | CZ | ARG | A | 306 | 2.897 | -9.910 | -21.505 | 1.00 | 70.64 | C |
| ATOM | 2031 | NH1 | ARG | A | 306 | 3.574 | -10.188 | -22.613 | 1.00 | 71.49 | N |
| ATOM | 2032 | NH2 | ARG | A | 306 | 2.751 | -10.832 | -20.555 | 1.00 | 70.53 | N |
| ATOM | 2033 | C | ARG | A | 306 | -2.070 | -8.714 | -24.501 | 1.00 | 58.04 | C |
| ATOM | 2034 | O | ARG | A | 306 | -3.124 | -8.875 | -23.884 | 1.00 | 57.71 | O |
| ATOM | 2035 | N | PRO | A | 307 | -1.792 | -9.390 | -25.629 | 1.00 | 57.71 | N |
| ATOM | 2036 | CD | PRO | A | 307 | -0.512 | -9.466 | -26.354 | 1.00 | 57.23 | C |
| ATOM | 2037 | CA | PRO | A | 307 | -2.753 | -10.364 | -26.162 | 1.00 | 56.71 | C |
| ATOM | 2038 | CB | PRO | A | 307 | -1.933 | -11.163 | -27.185 | 1.00 | 57.14 | C |
| ATOM | 2039 | CG | PRO | A | 307 | -0.509 | -10.905 | -26.797 | 1.00 | 57.60 | C |
| ATOM | 2040 | C | PRO | A | 307 | -3.506 | -11.252 | -25.185 | 1.00 | 55.99 | C |
| ATOM | 2041 | O | PRO | A | 307 | -4.735 | -11.214 | -25.143 | 1.00 | 56.05 | O |
| ATOM | 2042 | N | ALA | A | 308 | -2.814 | -12.061 | -24.399 | 1.00 | 55.18 | N |
| ATOM | 2043 | CA | ALA | A | 308 | -3.548 | -12.939 | -23.488 | 1.00 | 54.89 | C |
| ATOM | 2044 | CB | ALA | A | 308 | -2.860 | -14.301 | -23.417 | 1.00 | 55.13 | C |
| ATOM | 2045 | C | ALA | A | 308 | -3.814 | -12.405 | -22.077 | 1.00 | 54.03 | C |
| ATOM | 2046 | O | ALA | A | 308 | -3.862 | -13.176 | -21.111 | 1.00 | 53.36 | O |
| ATOM | 2047 | N | THR | A | 309 | -3.985 | -11.089 | -21.959 | 1.00 | 52.55 | N |
| ATOM | 2048 | CA | THR | A | 309 | -4.289 | -10.485 | -20.666 | 1.00 | 51.25 | C |
| ATOM | 2049 | CB | THR | A | 309 | -4.184 | -8.937 | -20.702 | 1.00 | 50.42 | C |
| ATOM | 2050 | OG1 | THR | A | 309 | -2.826 | -8.552 | -20.949 | 1.00 | 49.95 | O |
| ATOM | 2051 | CG2 | THR | A | 309 | -4.629 | -8.339 | -19.369 | 1.00 | 49.29 | C |
| ATOM | 2052 | C | THR | A | 309 | -5.734 | -10.885 | -20.385 | 1.00 | 51.40 | C |
| ATOM | 2053 | O | THR | A | 309 | -6.616 | -10.650 | -21.205 | 1.00 | 51.92 | O |
| ATOM | 2054 | N | PRO | A | 310 | -5.995 | -11.505 | -19.226 | 1.00 | 51.26 | N |
| ATOM | 2055 | CD | PRO | A | 310 | -5.074 | -11.705 | -18.094 | 1.00 | 50.93 | C |
| ATOM | 2056 | CA | PRO | A | 310 | -7.354 | -11.937 | -18.867 | 1.00 | 51.11 | C |
| ATOM | 2057 | CB | PRO | A | 310 | -7.177 | -12.541 | -17.480 | 1.00 | 50.39 | C |
| ATOM | 2058 | CG | PRO | A | 310 | -5.707 | -12.884 | -17.411 | 1.00 | 50.33 | C |
| ATOM | 2059 | C | PRO | A | 310 | -8.385 | -10.815 | -18.839 | 1.00 | 52.32 | C |
| ATOM | 2060 | O | PRO | A | 310 | -8.104 | -9.705 | -18.395 | 1.00 | 53.05 | O |
| ATOM | 2061 | N | PRO | A | 311 | -9.611 | -11.106 | -19.290 | 1.00 | 53.08 | N |
| ATOM | 2062 | CD | PRO | A | 311 | -10.015 | -12.447 | -19.755 | 1.00 | 53.44 | C |
| ATOM | 2063 | CA | PRO | A | 311 | -10.744 | -10.173 | -19.351 | 1.00 | 51.57 | C |
| ATOM | 2064 | CB | PRO | A | 311 | -11.907 | -11.081 | -19.749 | 1.00 | 52.03 | C |
| ATOM | 2065 | CG | PRO | A | 311 | -11.229 | -12.133 | -20.593 | 1.00 | 52.27 | C |
| ATOM | 2066 | C | PRO | A | 311 | -11.028 | -9.416 | -18.048 | 1.00 | 50.52 | C |
| ATOM | 2067 | O | PRO | A | 311 | -11.085 | -8.186 | -18.040 | 1.00 | 50.57 | O |
| ATOM | 2068 | N | GLU | A | 312 | -11.205 | -10.149 | -16.956 | 1.00 | 49.38 | N |
| ATOM | 2069 | CA | GLU | A | 312 | -11.509 | -9.540 | -15.661 | 1.00 | 49.57 | C |
| ATOM | 2070 | CB | GLU | A | 312 | -11.887 | -10.616 | -14.636 | 1.00 | 52.21 | C |

| | | | | | | | | | | | |
|---|---|---|---|---|---|---|---|---|---|---|---|
| ATOM | 2071 | CG | GLU | A | 312 | -12.981 | -11.558 | -15.123 | 1.00 | 57.17 | C |
| ATOM | 2072 | CD | GLU | A | 312 | -12.454 | -12.631 | -16.085 | 1.00 | 61.67 | C |
| ATOM | 2073 | OE1 | GLU | A | 312 | -11.430 | -12.381 | -16.777 | 1.00 | 63.35 | O |
| ATOM | 2074 | OE2 | GLU | A | 312 | -13.066 | -13.730 | -16.159 | 1.00 | 63.31 | O |
| ATOM | 2075 | C | GLU | A | 312 | -10.364 | -8.688 | -15.114 | 1.00 | 48.19 | C |
| ATOM | 2076 | O | GLU | A | 312 | -10.579 | -7.823 | -14.268 | 1.00 | 47.92 | O |
| ATOM | 2077 | N | ALA | A | 313 | -9.146 | -8.937 | -15.584 | 1.00 | 46.49 | N |
| ATOM | 2078 | CA | ALA | A | 313 | -8.011 | -8.146 | -15.140 | 1.00 | 44.59 | C |
| ATOM | 2079 | CB | ALA | A | 313 | -6.703 | -8.771 | -15.616 | 1.00 | 44.73 | C |
| ATOM | 2080 | C | ALA | A | 313 | -8.214 | -6.785 | -15.790 | 1.00 | 44.14 | C |
| ATOM | 2081 | O | ALA | A | 313 | -8.220 | -5.745 | -15.130 | 1.00 | 43.86 | O |
| ATOM | 2082 | N | ILE | A | 314 | -8.413 | -6.824 | -17.104 | 1.00 | 43.08 | N |
| ATOM | 2083 | CA | ILE | A | 314 | -8.639 | -5.639 | -17.915 | 1.00 | 41.18 | C |
| ATOM | 2084 | CB | ILE | A | 314 | -8.752 | -6.033 | -19.411 | 1.00 | 40.41 | C |
| ATOM | 2085 | CG2 | ILE | A | 314 | -9.155 | -4.825 | -20.248 | 1.00 | 41.33 | C |
| ATOM | 2086 | CG1 | ILE | A | 314 | -7.418 | -6.605 | -19.895 | 1.00 | 39.50 | C |
| ATOM | 2087 | CD1 | ILE | A | 314 | -7.412 | -7.048 | -21.341 | 1.00 | 34.08 | C |
| ATOM | 2088 | C | ILE | A | 314 | -9.901 | -4.890 | -17.478 | 1.00 | 41.34 | C |
| ATOM | 2089 | O | ILE | A | 314 | -9.949 | -3.656 | -17.518 | 1.00 | 41.19 | O |
| ATOM | 2090 | N | ALA | A | 315 | -10.916 | -5.633 | -17.050 | 1.00 | 40.30 | N |
| ATOM | 2091 | CA | ALA | A | 315 | -12.179 | -5.038 | -16.610 | 1.00 | 40.52 | C |
| ATOM | 2092 | CB | ALA | A | 315 | -13.226 | -6.132 | -16.411 | 1.00 | 40.57 | C |
| ATOM | 2093 | C | ALA | A | 315 | -12.033 | -4.227 | -15.325 | 1.00 | 40.71 | C |
| ATOM | 2094 | O | ALA | A | 315 | -12.426 | -3.059 | -15.264 | 1.00 | 40.44 | O |
| ATOM | 2095 | N | LEU | A | 316 | -11.490 | -4.870 | -14.296 | 1.00 | 40.91 | N |
| ATOM | 2096 | CA | LEU | A | 316 | -11.267 | -4.230 | -13.006 | 1.00 | 40.40 | C |
| ATOM | 2097 | CB | LEU | A | 316 | -10.508 | -5.179 | -12.072 | 1.00 | 39.91 | C |
| ATOM | 2098 | CG | LEU | A | 316 | -9.871 | -4.584 | -10.812 | 1.00 | 40.64 | C |
| ATOM | 2099 | CD1 | LEU | A | 316 | -10.929 | -3.868 | -9.991 | 1.00 | 40.79 | C |
| ATOM | 2100 | CD2 | LEU | A | 316 | -9.211 | -5.684 | -9.998 | 1.00 | 40.73 | C |
| ATOM | 2101 | C | LEU | A | 316 | -10.438 | -2.977 | -13.232 | 1.00 | 40.91 | C |
| ATOM | 2102 | O | LEU | A | 316 | -10.707 | -1.922 | -12.663 | 1.00 | 41.61 | O |
| ATOM | 2103 | N | CYS | A | 317 | -9.436 | -3.109 | -14.085 | 1.00 | 40.52 | N |
| ATOM | 2104 | CA | CYS | A | 317 | -8.546 | -2.012 | -14.384 | 1.00 | 41.58 | C |
| ATOM | 2105 | CB | CYS | A | 317 | -7.559 | -2.435 | -15.469 | 1.00 | 42.08 | C |
| ATOM | 2106 | SG | CYS | A | 317 | -5.915 | -1.700 | -15.303 | 1.00 | 45.21 | S |
| ATOM | 2107 | C | CYS | A | 317 | -9.307 | -0.765 | -14.815 | 1.00 | 42.53 | C |
| ATOM | 2108 | O | CYS | A | 317 | -9.105 | 0.319 | -14.263 | 1.00 | 44.86 | O |
| ATOM | 2109 | N | SER | A | 318 | -10.198 | -0.911 | -15.786 | 1.00 | 41.97 | N |
| ATOM | 2110 | CA | SER | A | 318 | -10.967 | 0.226 | -16.280 | 1.00 | 40.62 | C |
| ATOM | 2111 | CB | SER | A | 318 | -11.725 | -0.175 | -17.547 | 1.00 | 40.24 | C |
| ATOM | 2112 | OG | SER | A | 318 | -12.477 | -1.353 | -17.324 | 1.00 | 41.90 | O |
| ATOM | 2113 | C | SER | A | 318 | -11.938 | 0.821 | -15.259 | 1.00 | 38.87 | C |
| ATOM | 2114 | O | SER | A | 318 | -12.429 | 1.939 | -15.437 | 1.00 | 38.94 | O |
| ATOM | 2115 | N | ARG | A | 319 | -12.219 | 0.090 | -14.190 | 1.00 | 38.10 | N |
| ATOM | 2116 | CA | ARG | A | 319 | -13.133 | 0.597 | -13.169 | 1.00 | 37.95 | C |
| ATOM | 2117 | CB | ARG | A | 319 | -13.939 | -0.554 | -12.581 | 1.00 | 37.76 | C |
| ATOM | 2118 | CG | ARG | A | 319 | -14.718 | -1.345 | -13.612 | 1.00 | 41.11 | C |
| ATOM | 2119 | CD | ARG | A | 319 | -15.900 | -0.558 | -14.161 | 1.00 | 41.66 | C |
| ATOM | 2120 | NE | ARG | A | 319 | -16.856 | -0.246 | -13.111 | 1.00 | 41.80 | N |
| ATOM | 2121 | CZ | ARG | A | 319 | -16.939 | 0.930 | -12.502 | 1.00 | 42.44 | C |
| ATOM | 2122 | NH1 | ARG | A | 319 | -16.122 | 1.916 | -12.854 | 1.00 | 41.07 | N |
| ATOM | 2123 | NH2 | ARG | A | 319 | -17.823 | 1.103 | -11.523 | 1.00 | 41.39 | N |
| ATOM | 2124 | C | ARG | A | 319 | -12.360 | 1.320 | -12.067 | 1.00 | 37.01 | C |
| ATOM | 2125 | O | ARG | A | 319 | -12.942 | 1.827 | -11.105 | 1.00 | 36.80 | O |
| ATOM | 2126 | N | LEU | A | 320 | -11.038 | 1.343 | -12.224 | 1.00 | 37.21 | N |
| ATOM | 2127 | CA | LEU | A | 320 | -10.131 | 2.001 | -11.291 | 1.00 | 34.58 | C |
| ATOM | 2128 | CB | LEU | A | 320 | -8.967 | 1.067 | -10.928 | 1.00 | 33.08 | C |
| ATOM | 2129 | CG | LEU | A | 320 | -9.314 | -0.234 | -10.196 | 1.00 | 34.21 | C |
| ATOM | 2130 | CD1 | LEU | A | 320 | -8.065 | -1.085 | -9.999 | 1.00 | 34.95 | C |
| ATOM | 2131 | CD2 | LEU | A | 320 | -9.957 | 0.091 | -8.866 | 1.00 | 34.39 | C |
| ATOM | 2132 | C | LEU | A | 320 | -9.597 | 3.253 | -11.982 | 1.00 | 34.10 | C |
| ATOM | 2133 | O | LEU | A | 320 | -9.746 | 4.365 | -11.480 | 1.00 | 33.59 | O |
| ATOM | 2134 | N | LEU | A | 321 | -8.989 | 3.069 | -13.149 | 1.00 | 33.80 | N |
| ATOM | 2135 | CA | LEU | A | 321 | -8.441 | 4.196 | -13.884 | 1.00 | 34.35 | C |
| ATOM | 2136 | CB | LEU | A | 321 | -7.388 | 3.712 | -14.878 | 1.00 | 34.26 | C |
| ATOM | 2137 | CG | LEU | A | 321 | -6.230 | 2.977 | -14.200 | 1.00 | 32.44 | C |

| | | | | | | | | | | | |
|---|---|---|---|---|---|---|---|---|---|---|---|
| ATOM | 2138 | CD1 | LEU | A | 321 | -5.262 | 2.484 | -15.246 | 1.00 | 31.74 | C |
| ATOM | 2139 | CD2 | LEU | A | 321 | -5.555 | 3.883 | -13.196 | 1.00 | 30.88 | C |
| ATOM | 2140 | C | LEU | A | 321 | -9.525 | 4.999 | -14.593 | 1.00 | 35.49 | C |
| ATOM | 2141 | O | LEU | A | 321 | -9.579 | 5.059 | -15.823 | 1.00 | 36.36 | O |
| ATOM | 2142 | N | GLU | A | 322 | -10.404 | 5.599 | -13.797 | 1.00 | 35.43 | N |
| ATOM | 2143 | CA | GLU | A | 322 | -11.473 | 6.432 | -14.319 | 1.00 | 36.14 | C |
| ATOM | 2144 | CB | GLU | A | 322 | -12.804 | 6.112 | -13.636 | 1.00 | 36.81 | C |
| ATOM | 2145 | CG | GLU | A | 322 | -13.327 | 4.708 | -13.914 | 1.00 | 41.71 | C |
| ATOM | 2146 | CD | GLU | A | 322 | -14.649 | 4.716 | -14.678 | 1.00 | 42.90 | C |
| ATOM | 2147 | OE1 | GLU | A | 322 | -14.679 | 5.179 | -15.842 | 1.00 | 41.53 | O |
| ATOM | 2148 | OE2 | GLU | A | 322 | -15.660 | 4.266 | -14.101 | 1.00 | 44.84 | O |
| ATOM | 2149 | C | GLU | A | 322 | -11.077 | 7.863 | -14.012 | 1.00 | 35.88 | C |
| ATOM | 2150 | O | GLU | A | 322 | -10.357 | 8.110 | -13.045 | 1.00 | 36.70 | O |
| ATOM | 2151 | N | TYR | A | 323 | -11.529 | 8.802 | -14.838 | 1.00 | 35.44 | N |
| ATOM | 2152 | CA | TYR | A | 323 | -11.222 | 10.208 | -14.622 | 1.00 | 34.36 | C |
| ATOM | 2153 | CB | TYR | A | 323 | -11.557 | 11.034 | -15.860 | 1.00 | 33.74 | C |
| ATOM | 2154 | CG | TYR | A | 323 | -10.465 | 11.092 | -16.898 | 1.00 | 35.03 | C |
| ATOM | 2155 | CD1 | TYR | A | 323 | -9.187 | 11.554 | -16.573 | 1.00 | 35.17 | C |
| ATOM | 2156 | CE1 | TYR | A | 323 | -8.187 | 11.656 | -17.538 | 1.00 | 34.69 | C |
| ATOM | 2157 | CD2 | TYR | A | 323 | -10.716 | 10.731 | -18.218 | 1.00 | 34.50 | C |
| ATOM | 2158 | CE2 | TYR | A | 323 | -9.724 | 10.831 | -19.187 | 1.00 | 35.40 | C |
| ATOM | 2159 | CZ | TYR | A | 323 | -8.466 | 11.296 | -18.842 | 1.00 | 34.82 | C |
| ATOM | 2160 | OH | TYR | A | 323 | -7.502 | 11.413 | -19.809 | 1.00 | 36.67 | O |
| ATOM | 2161 | C | TYR | A | 323 | -12.033 | 10.727 | -13.453 | 1.00 | 35.44 | C |
| ATOM | 2162 | O | TYR | A | 323 | -11.517 | 11.434 | -12.593 | 1.00 | 37.24 | O |
| ATOM | 2163 | N | THR | A | 324 | -13.313 | 10.384 | -13.431 | 1.00 | 35.04 | N |
| ATOM | 2164 | CA | THR | A | 324 | -14.188 | 10.825 | -12.363 | 1.00 | 35.30 | C |
| ATOM | 2165 | CB | THR | A | 324 | -15.650 | 10.595 | -12.748 | 1.00 | 36.60 | C |
| ATOM | 2166 | OG1 | THR | A | 324 | -15.829 | 10.944 | -14.126 | 1.00 | 36.29 | O |
| ATOM | 2167 | CG2 | THR | A | 324 | -16.566 | 11.463 | -11.903 | 1.00 | 34.56 | C |
| ATOM | 2168 | C | THR | A | 324 | -13.854 | 10.052 | -11.090 | 1.00 | 35.87 | C |
| ATOM | 2169 | O | THR | A | 324 | -14.071 | 8.843 | -11.014 | 1.00 | 35.99 | O |
| ATOM | 2170 | N | PRO | A | 325 | -13.312 | 10.747 | -10.076 | 1.00 | 35.91 | N |
| ATOM | 2171 | CD | PRO | A | 325 | -13.154 | 12.210 | -10.061 | 1.00 | 36.06 | C |
| ATOM | 2172 | CA | PRO | A | 325 | -12.920 | 10.176 | -8.783 | 1.00 | 33.31 | C |
| ATOM | 2173 | CB | PRO | A | 325 | -12.671 | 11.411 | -7.926 | 1.00 | 34.28 | C |
| ATOM | 2174 | CG | PRO | A | 325 | -12.181 | 12.409 | -8.927 | 1.00 | 33.53 | C |
| ATOM | 2175 | C | PRO | A | 325 | -14.016 | 9.300 | -8.213 | 1.00 | 33.32 | C |
| ATOM | 2176 | O | PRO | A | 325 | -13.763 | 8.202 | -7.714 | 1.00 | 32.36 | O |
| ATOM | 2177 | N | THR | A | 326 | -15.238 | 9.808 | -8.305 | 1.00 | 33.06 | N |
| ATOM | 2178 | CA | THR | A | 326 | -16.431 | 9.131 | -7.810 | 1.00 | 31.97 | C |
| ATOM | 2179 | CB | THR | A | 326 | -17.616 | 10.095 | -7.832 | 1.00 | 30.63 | C |
| ATOM | 2180 | OG1 | THR | A | 326 | -17.725 | 10.679 | -9.140 | 1.00 | 30.27 | O |
| ATOM | 2181 | CG2 | THR | A | 326 | -17.424 | 11.196 | -6.808 | 1.00 | 27.12 | C |
| ATOM | 2182 | C | THR | A | 326 | -16.821 | 7.884 | -8.597 | 1.00 | 33.27 | C |
| ATOM | 2183 | O | THR | A | 326 | -17.593 | 7.058 | -8.112 | 1.00 | 34.16 | O |
| ATOM | 2184 | N | ALA | A | 327 | -16.290 | 7.757 | -9.810 | 1.00 | 34.25 | N |
| ATOM | 2185 | CA | ALA | A | 327 | -16.588 | 6.625 | -10.689 | 1.00 | 34.86 | C |
| ATOM | 2186 | CB | ALA | A | 327 | -16.352 | 7.039 | -12.135 | 1.00 | 36.14 | C |
| ATOM | 2187 | C | ALA | A | 327 | -15.774 | 5.374 | -10.374 | 1.00 | 35.20 | C |
| ATOM | 2188 | O | ALA | A | 327 | -16.166 | 4.264 | -10.729 | 1.00 | 34.98 | O |
| ATOM | 2189 | N | ARG | A | 328 | -14.641 | 5.566 | -9.706 | 1.00 | 36.24 | N |
| ATOM | 2190 | CA | ARG | A | 328 | -13.750 | 4.468 | -9.351 | 1.00 | 34.75 | C |
| ATOM | 2191 | CB | ARG | A | 328 | -12.403 | 5.030 | -8.916 | 1.00 | 35.19 | C |
| ATOM | 2192 | CG | ARG | A | 328 | -11.799 | 5.957 | -9.934 | 1.00 | 34.46 | C |
| ATOM | 2193 | CD | ARG | A | 328 | -10.585 | 6.672 | -9.397 | 1.00 | 35.45 | C |
| ATOM | 2194 | NE | ARG | A | 328 | -10.222 | 7.765 | -10.295 | 1.00 | 36.45 | N |
| ATOM | 2195 | CZ | ARG | A | 328 | -9.600 | 8.873 | -9.913 | 1.00 | 34.14 | C |
| ATOM | 2196 | NH1 | ARG | A | 328 | -9.258 | 9.048 | -8.645 | 1.00 | 33.71 | N |
| ATOM | 2197 | NH2 | ARG | A | 328 | -9.349 | 9.818 | -10.798 | 1.00 | 34.66 | N |
| ATOM | 2198 | C | ARG | A | 328 | -14.304 | 3.591 | -8.243 | 1.00 | 34.27 | C |
| ATOM | 2199 | O | ARG | A | 328 | -14.983 | 4.075 | -7.341 | 1.00 | 34.24 | O |
| ATOM | 2200 | N | LEU | A | 329 | -14.004 | 2.298 | -8.311 | 1.00 | 33.24 | N |
| ATOM | 2201 | CA | LEU | A | 329 | -14.451 | 1.348 | -7.294 | 1.00 | 34.74 | C |
| ATOM | 2202 | CB | LEU | A | 329 | -14.062 | -0.087 | -7.685 | 1.00 | 34.43 | C |
| ATOM | 2203 | CG | LEU | A | 329 | -14.801 | -0.864 | -8.776 | 1.00 | 33.73 | C |
| ATOM | 2204 | CD1 | LEU | A | 329 | -15.221 | 0.079 | -9.884 | 1.00 | 35.78 | C |

| | | | | | | | | | | | |
|---|---|---|---|---|---|---|---|---|---|---|---|
| ATOM | 2205 | CD2 | LEU | A | 329 | -13.899 | -1.973 | -9.298 | 1.00 | 31.29 | C |
| ATOM | 2206 | C | LEU | A | 329 | -13.799 | 1.676 | -5.952 | 1.00 | 36.33 | C |
| ATOM | 2207 | O | LEU | A | 329 | -12.713 | 2.253 | -5.908 | 1.00 | 37.77 | O |
| ATOM | 2208 | N | THR | A | 330 | -14.458 | 1.311 | -4.858 | 1.00 | 36.40 | N |
| ATOM | 2209 | CA | THR | A | 330 | -13.891 | 1.547 | -3.535 | 1.00 | 37.29 | C |
| ATOM | 2210 | CB | THR | A | 330 | -14.984 | 1.659 | -2.446 | 1.00 | 35.55 | C |
| ATOM | 2211 | OG1 | THR | A | 330 | -15.569 | 0.375 | -2.215 | 1.00 | 34.01 | O |
| ATOM | 2212 | CG2 | THR | A | 330 | -16.068 | 2.635 | -2.877 | 1.00 | 34.17 | C |
| ATOM | 2213 | C | THR | A | 330 | -13.007 | 0.336 | -3.236 | 1.00 | 39.23 | C |
| ATOM | 2214 | O | THR | A | 330 | -13.153 | -0.721 | -3.867 | 1.00 | 39.22 | O |
| ATOM | 2215 | N | PRO | A | 331 | -12.068 | 0.476 | -2.289 | 1.00 | 40.50 | N |
| ATOM | 2216 | CD | PRO | A | 331 | -11.696 | 1.695 | -1.543 | 1.00 | 40.75 | C |
| ATOM | 2217 | CA | PRO | A | 331 | -11.186 | -0.645 | -1.950 | 1.00 | 41.18 | C |
| ATOM | 2218 | CB | PRO | A | 331 | -10.453 | -0.130 | -0.713 | 1.00 | 42.21 | C |
| ATOM | 2219 | CG | PRO | A | 331 | -10.328 | 1.347 | -1.005 | 1.00 | 41.19 | C |
| ATOM | 2220 | C | PRO | A | 331 | -11.953 | -1.960 | -1.702 | 1.00 | 41.93 | C |
| ATOM | 2221 | O | PRO | A | 331 | -11.566 | -3.011 | -2.213 | 1.00 | 41.11 | O |
| ATOM | 2222 | N | LEU | A | 332 | -13.038 | -1.896 | -0.928 | 1.00 | 42.86 | N |
| ATOM | 2223 | CA | LEU | A | 332 | -13.844 | -3.085 | -0.648 | 1.00 | 43.53 | C |
| ATOM | 2224 | CB | LEU | A | 332 | -14.972 | -2.772 | 0.337 | 1.00 | 43.49 | C |
| ATOM | 2225 | CG | LEU | A | 332 | -14.828 | -3.201 | 1.795 | 1.00 | 44.92 | C |
| ATOM | 2226 | CD1 | LEU | A | 332 | -16.215 | -3.213 | 2.429 | 1.00 | 45.51 | C |
| ATOM | 2227 | CD2 | LEU | A | 332 | -14.207 | -4.600 | 1.884 | 1.00 | 45.53 | C |
| ATOM | 2228 | C | LEU | A | 332 | -14.462 | -3.651 | -1.925 | 1.00 | 44.53 | C |
| ATOM | 2229 | O | LEU | A | 332 | -14.463 | -4.864 | -2.144 | 1.00 | 44.34 | O |
| ATOM | 2230 | N | GLU | A | 333 | -15.002 | -2.760 | -2.753 | 1.00 | 44.91 | N |
| ATOM | 2231 | CA | GLU | A | 333 | -15.625 | -3.140 | -4.017 | 1.00 | 45.16 | C |
| ATOM | 2232 | CB | GLU | A | 333 | -16.166 | -1.888 | -4.726 | 1.00 | 46.68 | C |
| ATOM | 2233 | CG | GLU | A | 333 | -17.658 | -1.631 | -4.503 | 1.00 | 47.09 | C |
| ATOM | 2234 | CD | GLU | A | 333 | -18.062 | -0.178 | -4.729 | 1.00 | 47.55 | C |
| ATOM | 2235 | OE1 | GLU | A | 333 | -17.739 | 0.387 | -5.803 | 1.00 | 47.72 | O |
| ATOM | 2236 | OE2 | GLU | A | 333 | -18.716 | 0.392 | -3.823 | 1.00 | 48.41 | O |
| ATOM | 2237 | C | GLU | A | 333 | -14.619 | -3.858 | -4.914 | 1.00 | 45.21 | C |
| ATOM | 2238 | O | GLU | A | 333 | -14.940 | -4.870 | -5.537 | 1.00 | 45.13 | O |
| ATOM | 2239 | N | ALA | A | 334 | -13.397 | -3.328 | -4.964 | 1.00 | 44.79 | N |
| ATOM | 2240 | CA | ALA | A | 334 | -12.332 | -3.914 | -5.772 | 1.00 | 42.83 | C |
| ATOM | 2241 | CB | ALA | A | 334 | -11.096 | -3.030 | -5.725 | 1.00 | 42.34 | C |
| ATOM | 2242 | C | ALA | A | 334 | -12.002 | -5.317 | -5.270 | 1.00 | 41.74 | C |
| ATOM | 2243 | O | ALA | A | 334 | -11.697 | -6.219 | -6.060 | 1.00 | 41.06 | O |
| ATOM | 2244 | N | CYS | A | 335 | -12.068 | -5.494 | -3.956 | 1.00 | 40.92 | N |
| ATOM | 2245 | CA | CYS | A | 335 | -11.801 | -6.791 | -3.345 | 1.00 | 40.45 | C |
| ATOM | 2246 | CB | CYS | A | 335 | -11.776 | -6.668 | -1.821 | 1.00 | 39.90 | C |
| ATOM | 2247 | SG | CYS | A | 335 | -10.192 | -6.194 | -1.127 | 1.00 | 36.85 | S |
| ATOM | 2248 | C | CYS | A | 335 | -12.895 | -7.774 | -3.740 | 1.00 | 40.71 | C |
| ATOM | 2249 | O | CYS | A | 335 | -12.657 | -8.972 | -3.863 | 1.00 | 40.80 | O |
| ATOM | 2250 | N | ALA | A | 336 | -14.097 | -7.254 | -3.951 | 1.00 | 40.33 | N |
| ATOM | 2251 | CA | ALA | A | 336 | -15.208 | -8.107 | -4.325 | 1.00 | 40.56 | C |
| ATOM | 2252 | CB | ALA | A | 336 | -16.527 | -7.465 | -3.907 | 1.00 | 39.78 | C |
| ATOM | 2253 | C | ALA | A | 336 | -15.227 | -8.428 | -5.817 | 1.00 | 40.89 | C |
| ATOM | 2254 | O | ALA | A | 336 | -15.979 | -9.299 | -6.240 | 1.00 | 41.91 | O |
| ATOM | 2255 | N | HIS | A | 337 | -14.399 | -7.753 | -6.608 | 1.00 | 41.19 | N |
| ATOM | 2256 | CA | HIS | A | 337 | -14.377 | -7.995 | -8.052 | 1.00 | 41.37 | C |
| ATOM | 2257 | CB | HIS | A | 337 | -13.260 | -7.193 | -8.719 | 1.00 | 39.59 | C |
| ATOM | 2258 | CG | HIS | A | 337 | -13.371 | -7.135 | -10.210 | 1.00 | 38.19 | C |
| ATOM | 2259 | CD2 | HIS | A | 337 | -12.984 | -8.011 | -11.167 | 1.00 | 37.50 | C |
| ATOM | 2260 | ND1 | HIS | A | 337 | -13.967 | -6.084 | -10.874 | 1.00 | 37.55 | N |
| ATOM | 2261 | CE1 | HIS | A | 337 | -13.939 | -6.313 | -12.174 | 1.00 | 36.86 | C |
| ATOM | 2262 | NE2 | HIS | A | 337 | -13.347 | -7.476 | -12.377 | 1.00 | 37.70 | N |
| ATOM | 2263 | C | HIS | A | 337 | -14.214 | -9.475 | -8.416 | 1.00 | 42.44 | C |
| ATOM | 2264 | O | HIS | A | 337 | -13.699 | -10.268 | -7.627 | 1.00 | 44.49 | O |
| ATOM | 2265 | N | SER | A | 338 | -14.643 | -9.838 | -9.621 | 1.00 | 42.60 | N |
| ATOM | 2266 | CA | SER | A | 338 | -14.567 | -11.227 | -10.073 | 1.00 | 43.77 | C |
| ATOM | 2267 | CB | SER | A | 338 | -15.483 | -11.447 | -11.287 | 1.00 | 42.93 | C |
| ATOM | 2268 | OG | SER | A | 338 | -14.959 | -10.851 | -12.460 | 1.00 | 43.12 | O |
| ATOM | 2269 | C | SER | A | 338 | -13.148 | -11.700 | -10.413 | 1.00 | 44.30 | C |
| ATOM | 2270 | O | SER | A | 338 | -12.863 | -12.900 | -10.399 | 1.00 | 44.38 | O |
| ATOM | 2271 | N | PHE | A | 339 | -12.268 | -10.750 | -10.721 | 1.00 | 43.61 | N |

| | | | | | | | | | | | |
|---|---|---|---|---|---|---|---|---|---|---|---|
| ATOM | 2272 | CA | PHE | A | 339 | -10.875 | -11.031 | -11.066 | 1.00 | 42.40 | C |
| ATOM | 2273 | CB | PHE | A | 339 | -10.162 | -9.716 | -11.386 | 1.00 | 42.49 | C |
| ATOM | 2274 | CG | PHE | A | 339 | -8.664 | -9.811 | -11.400 | 1.00 | 42.23 | C |
| ATOM | 2275 | CD1 | PHE | A | 339 | -8.002 | -10.561 | -12.364 | 1.00 | 42.19 | C |
| ATOM | 2276 | CD2 | PHE | A | 339 | -7.912 | -9.089 | -10.485 | 1.00 | 41.69 | C |
| ATOM | 2277 | CE1 | PHE | A | 339 | -6.608 | -10.577 | -12.420 | 1.00 | 41.90 | C |
| ATOM | 2278 | CE2 | PHE | A | 339 | -6.523 | -9.101 | -10.534 | 1.00 | 41.64 | C |
| ATOM | 2279 | CZ | PHE | A | 339 | -5.868 | -9.844 | -11.503 | 1.00 | 41.39 | C |
| ATOM | 2280 | C | PHE | A | 339 | -10.180 | -11.730 | -9.903 | 1.00 | 42.42 | C |
| ATOM | 2281 | O | PHE | A | 339 | -9.215 | -12.477 | -10.085 | 1.00 | 40.98 | O |
| ATOM | 2282 | N | PHE | A | 340 | -10.692 | -11.476 | -8.703 | 1.00 | 42.23 | N |
| ATOM | 2283 | CA | PHE | A | 340 | -10.132 | -12.060 | -7.499 | 1.00 | 43.07 | C |
| ATOM | 2284 | CB | PHE | A | 340 | -10.164 | -11.040 | -6.345 | 1.00 | 41.82 | C |
| ATOM | 2285 | CG | PHE | A | 340 | -9.279 | -9.836 | -6.563 | 1.00 | 41.55 | C |
| ATOM | 2286 | CD1 | PHE | A | 340 | -7.931 | -9.994 | -6.856 | 1.00 | 40.41 | C |
| ATOM | 2287 | CD2 | PHE | A | 340 | -9.793 | -8.545 | -6.489 | 1.00 | 40.87 | C |
| ATOM | 2288 | CE1 | PHE | A | 340 | -7.110 | -8.895 | -7.061 | 1.00 | 38.46 | C |
| ATOM | 2289 | CE2 | PHE | A | 340 | -8.967 | -7.443 | -6.694 | 1.00 | 38.08 | C |
| ATOM | 2290 | CZ | PHE | A | 340 | -7.627 | -7.622 | -6.986 | 1.00 | 36.36 | C |
| ATOM | 2291 | C | PHE | A | 340 | -10.874 | -13.330 | -7.089 | 1.00 | 44.31 | C |
| ATOM | 2292 | O | PHE | A | 340 | -10.574 | -13.911 | -6.041 | 1.00 | 44.70 | O |
| ATOM | 2293 | N | ASP | A | 341 | -11.836 | -13.764 | -7.904 | 1.00 | 45.42 | N |
| ATOM | 2294 | CA | ASP | A | 341 | -12.610 | -14.969 | -7.585 | 1.00 | 46.41 | C |
| ATOM | 2295 | CB | ASP | A | 341 | -13.536 | -15.362 | -8.748 | 1.00 | 47.54 | C |
| ATOM | 2296 | CG | ASP | A | 341 | -14.823 | -14.563 | -8.765 | 1.00 | 49.20 | C |
| ATOM | 2297 | OD1 | ASP | A | 341 | -15.304 | -14.179 | -7.676 | 1.00 | 51.33 | O |
| ATOM | 2298 | OD2 | ASP | A | 341 | -15.367 | -14.325 | -9.865 | 1.00 | 51.01 | O |
| ATOM | 2299 | C | ASP | A | 341 | -11.721 | -16.153 | -7.225 | 1.00 | 45.81 | C |
| ATOM | 2300 | O | ASP | A | 341 | -11.887 | -16.755 | -6.167 | 1.00 | 45.25 | O |
| ATOM | 2301 | N | GLU | A | 342 | -10.795 | -16.497 | -8.115 | 1.00 | 45.67 | N |
| ATOM | 2302 | CA | GLU | A | 342 | -9.890 | -17.603 | -7.856 | 1.00 | 46.57 | C |
| ATOM | 2303 | CB | GLU | A | 342 | -8.648 | -17.487 | -8.746 | 1.00 | 46.51 | C |
| ATOM | 2304 | CG | GLU | A | 342 | -7.534 | -18.468 | -8.411 | 1.00 | 46.98 | C |
| ATOM | 2305 | CD | GLU | A | 342 | -6.546 | -18.656 | -9.550 | 1.00 | 48.92 | C |
| ATOM | 2306 | OE1 | GLU | A | 342 | -6.267 | -17.682 | -10.290 | 1.00 | 48.66 | O |
| ATOM | 2307 | OE2 | GLU | A | 342 | -6.028 | -19.785 | -9.700 | 1.00 | 50.70 | O |
| ATOM | 2308 | C | GLU | A | 342 | -9.490 | -17.646 | -6.383 | 1.00 | 47.49 | C |
| ATOM | 2309 | O | GLU | A | 342 | -9.655 | -18.668 | -5.720 | 1.00 | 49.39 | O |
| ATOM | 2310 | N | LEU | A | 343 | -8.994 | -16.528 | -5.864 | 1.00 | 48.46 | N |
| ATOM | 2311 | CA | LEU | A | 343 | -8.567 | -16.438 | -4.467 | 1.00 | 48.57 | C |
| ATOM | 2312 | CB | LEU | A | 343 | -8.136 | -15.014 | -4.140 | 1.00 | 48.69 | C |
| ATOM | 2313 | CG | LEU | A | 343 | -7.012 | -14.414 | -4.981 | 1.00 | 48.83 | C |
| ATOM | 2314 | CD1 | LEU | A | 343 | -6.831 | -12.950 | -4.578 | 1.00 | 49.38 | C |
| ATOM | 2315 | CD2 | LEU | A | 343 | -5.731 | -15.196 | -4.764 | 1.00 | 47.64 | C |
| ATOM | 2316 | C | LEU | A | 343 | -9.657 | -16.839 | -3.490 | 1.00 | 48.25 | C |
| ATOM | 2317 | O | LEU | A | 343 | -9.381 | -17.164 | -2.333 | 1.00 | 47.85 | O |
| ATOM | 2318 | N | ARG | A | 344 | -10.898 | -16.799 | -3.954 | 1.00 | 48.94 | N |
| ATOM | 2319 | CA | ARG | A | 344 | -12.033 | -17.144 | -3.110 | 1.00 | 49.59 | C |
| ATOM | 2320 | CB | ARG | A | 344 | -13.235 | -16.259 | -3.461 | 1.00 | 48.33 | C |
| ATOM | 2321 | CG | ARG | A | 344 | -13.368 | -15.008 | -2.594 | 1.00 | 45.72 | C |
| ATOM | 2322 | CD | ARG | A | 344 | -14.537 | -14.175 | -3.056 | 1.00 | 43.72 | C |
| ATOM | 2323 | NE | ARG | A | 344 | -14.332 | -13.731 | -4.432 | 1.00 | 44.21 | N |
| ATOM | 2324 | CZ | ARG | A | 344 | -13.747 | -12.586 | -4.765 | 1.00 | 43.16 | C |
| ATOM | 2325 | NH1 | ARG | A | 344 | -13.319 | -11.763 | -3.815 | 1.00 | 42.10 | N |
| ATOM | 2326 | NH2 | ARG | A | 344 | -13.570 | -12.277 | -6.044 | 1.00 | 42.04 | N |
| ATOM | 2327 | C | ARG | A | 344 | -12.415 | -18.617 | -3.195 | 1.00 | 50.83 | C |
| ATOM | 2328 | O | ARG | A | 344 | -13.345 | -19.068 | -2.525 | 1.00 | 50.94 | O |
| ATOM | 2329 | N | ASP | A | 345 | -11.693 | -19.361 | -4.024 | 1.00 | 53.45 | N |
| ATOM | 2330 | CA | ASP | A | 345 | -11.916 | -20.795 | -4.184 | 1.00 | 56.52 | C |
| ATOM | 2331 | CB | ASP | A | 345 | -11.264 | -21.278 | -5.486 | 1.00 | 57.97 | C |
| ATOM | 2332 | CG | ASP | A | 345 | -11.282 | -22.797 | -5.645 | 1.00 | 59.58 | C |
| ATOM | 2333 | OD1 | ASP | A | 345 | -11.441 | -23.523 | -4.634 | 1.00 | 60.61 | O |
| ATOM | 2334 | OD2 | ASP | A | 345 | -11.108 | -23.265 | -6.798 | 1.00 | 59.79 | O |
| ATOM | 2335 | C | ASP | A | 345 | -11.244 | -21.470 | -2.992 | 1.00 | 58.37 | C |
| ATOM | 2336 | O | ASP | A | 345 | -10.114 | -21.109 | -2.626 | 1.00 | 59.06 | O |
| ATOM | 2337 | N | PRO | A | 346 | -11.930 | -22.449 | -2.366 | 1.00 | 59.44 | N |
| ATOM | 2338 | CD | PRO | A | 346 | -13.305 | -22.876 | -2.685 | 1.00 | 59.42 | C |

| | | | | | | | | | | | |
|---|---|---|---|---|---|---|---|---|---|---|---|
| ATOM | 2339 | CA | PRO | A | 346 | -11.415 | -23.193 | -1.206 | 1.00 | 59.00 | C |
| ATOM | 2340 | CB | PRO | A | 346 | -12.604 | -24.045 | -0.787 | 1.00 | 59.06 | C |
| ATOM | 2341 | CG | PRO | A | 346 | -13.336 | -24.248 | -2.083 | 1.00 | 59.24 | C |
| ATOM | 2342 | C | PRO | A | 346 | -10.183 | -24.046 | -1.515 | 1.00 | 58.71 | C |
| ATOM | 2343 | O | PRO | A | 346 | -9.396 | -24.363 | -0.619 | 1.00 | 58.61 | O |
| ATOM | 2344 | N | ASN | A | 347 | -10.015 | -24.423 | -2.776 | 1.00 | 57.78 | N |
| ATOM | 2345 | CA | ASN | A | 347 | -8.864 | -25.235 | -3.147 | 1.00 | 58.46 | C |
| ATOM | 2346 | CB | ASN | A | 347 | -9.162 | -26.054 | -4.409 | 1.00 | 59.09 | C |
| ATOM | 2347 | CG | ASN | A | 347 | -9.784 | -27.415 | -4.093 | 1.00 | 60.89 | C |
| ATOM | 2348 | OD1 | ASN | A | 347 | -10.141 | -28.168 | -5.006 | 1.00 | 62.15 | O |
| ATOM | 2349 | ND2 | ASN | A | 347 | -9.908 | -27.739 | -2.804 | 1.00 | 59.76 | N |
| ATOM | 2350 | C | ASN | A | 347 | -7.581 | -24.443 | -3.353 | 1.00 | 57.60 | C |
| ATOM | 2351 | O | ASN | A | 347 | -6.528 | -24.803 | -2.814 | 1.00 | 58.54 | O |
| ATOM | 2352 | N | VAL | A | 348 | -7.675 | -23.363 | -4.121 | 1.00 | 56.54 | N |
| ATOM | 2353 | CA | VAL | A | 348 | -6.516 | -22.525 | -4.439 | 1.00 | 55.19 | C |
| ATOM | 2354 | CB | VAL | A | 348 | -6.912 | -21.032 | -4.565 | 1.00 | 54.53 | C |
| ATOM | 2355 | CG1 | VAL | A | 348 | -7.302 | -20.471 | -3.197 | 1.00 | 53.40 | C |
| ATOM | 2356 | CG2 | VAL | A | 348 | -5.738 | -20.244 | -5.142 | 1.00 | 52.92 | C |
| ATOM | 2357 | C | VAL | A | 348 | -5.343 | -22.609 | -3.459 | 1.00 | 54.19 | C |
| ATOM | 2358 | O | VAL | A | 348 | -5.527 | -22.635 | -2.239 | 1.00 | 53.99 | O |
| ATOM | 2359 | N | LYS | A | 349 | -4.138 | -22.643 | -4.009 | 1.00 | 52.88 | N |
| ATOM | 2360 | CA | LYS | A | 349 | -2.930 | -22.680 | -3.199 | 1.00 | 53.18 | C |
| ATOM | 2361 | CB | LYS | A | 349 | -2.434 | -24.121 | -3.004 | 1.00 | 53.59 | C |
| ATOM | 2362 | CG | LYS | A | 349 | -3.410 | -24.960 | -2.178 | 1.00 | 54.30 | C |
| ATOM | 2363 | CD | LYS | A | 349 | -2.740 | -26.072 | -1.390 | 1.00 | 55.00 | C |
| ATOM | 2364 | CE | LYS | A | 349 | -3.766 | -26.717 | -0.487 | 1.00 | 55.69 | C |
| ATOM | 2365 | NZ | LYS | A | 349 | -4.607 | -25.643 | 0.145 | 1.00 | 56.42 | N |
| ATOM | 2366 | C | LYS | A | 349 | -1.908 | -21.842 | -3.934 | 1.00 | 52.37 | C |
| ATOM | 2367 | O | LYS | A | 349 | -2.186 | -21.369 | -5.034 | 1.00 | 52.46 | O |
| ATOM | 2368 | N | LEU | A | 350 | -0.749 | -21.620 | -3.328 | 1.00 | 51.87 | N |
| ATOM | 2369 | CA | LEU | A | 350 | 0.274 | -20.822 | -3.987 | 1.00 | 52.34 | C |
| ATOM | 2370 | CB | LEU | A | 350 | 1.186 | -20.156 | -2.953 | 1.00 | 52.15 | C |
| ATOM | 2371 | CG | LEU | A | 350 | 0.565 | -19.282 | -1.851 | 1.00 | 52.10 | C |
| ATOM | 2372 | CD1 | LEU | A | 350 | 1.682 | -18.533 | -1.103 | 1.00 | 50.74 | C |
| ATOM | 2373 | CD2 | LEU | A | 350 | -0.406 | -18.287 | -2.470 | 1.00 | 50.83 | C |
| ATOM | 2374 | C | LEU | A | 350 | 1.108 | -21.726 | -4.877 | 1.00 | 52.64 | C |
| ATOM | 2375 | O | LEU | A | 350 | 1.177 | -22.935 | -4.654 | 1.00 | 52.72 | O |
| ATOM | 2376 | N | PRO | A | 351 | 1.738 | -21.159 | -5.915 | 1.00 | 53.29 | N |
| ATOM | 2377 | CD | PRO | A | 351 | 1.750 | -19.758 | -6.365 | 1.00 | 52.61 | C |
| ATOM | 2378 | CA | PRO | A | 351 | 2.564 | -21.999 | -6.789 | 1.00 | 54.12 | C |
| ATOM | 2379 | CB | PRO | A | 351 | 3.074 | -21.014 | -7.842 | 1.00 | 53.24 | C |
| ATOM | 2380 | CG | PRO | A | 351 | 3.030 | -19.701 | -7.148 | 1.00 | 52.07 | C |
| ATOM | 2381 | C | PRO | A | 351 | 3.678 | -22.557 | -5.915 | 1.00 | 55.37 | C |
| ATOM | 2382 | O | PRO | A | 351 | 4.518 | -23.356 | -6.341 | 1.00 | 56.11 | O |
| ATOM | 2383 | N | ASN | A | 352 | 3.649 | -22.109 | -4.666 | 1.00 | 56.49 | N |
| ATOM | 2384 | CA | ASN | A | 352 | 4.603 | -22.502 | -3.640 | 1.00 | 57.08 | C |
| ATOM | 2385 | CB | ASN | A | 352 | 4.561 | -21.469 | -2.511 | 1.00 | 58.52 | C |
| ATOM | 2386 | CG | ASN | A | 352 | 5.877 | -21.357 | -1.782 | 1.00 | 60.70 | C |
| ATOM | 2387 | OD1 | ASN | A | 352 | 6.086 | -20.441 | -0.980 | 1.00 | 61.10 | O |
| ATOM | 2388 | ND2 | ASN | A | 352 | 6.780 | -22.297 | -2.053 | 1.00 | 62.96 | N |
| ATOM | 2389 | C | ASN | A | 352 | 4.203 | -23.873 | -3.103 | 1.00 | 56.23 | C |
| ATOM | 2390 | O | ASN | A | 352 | 4.960 | -24.524 | -2.379 | 1.00 | 55.40 | O |
| ATOM | 2391 | N | GLY | A | 353 | 2.998 | -24.293 | -3.478 | 1.00 | 55.22 | N |
| ATOM | 2392 | CA | GLY | A | 353 | 2.469 | -25.565 | -3.029 | 1.00 | 54.41 | C |
| ATOM | 2393 | C | GLY | A | 353 | 1.819 | -25.363 | -1.678 | 1.00 | 53.98 | C |
| ATOM | 2394 | O | GLY | A | 353 | 1.046 | -26.200 | -1.206 | 1.00 | 53.52 | O |
| ATOM | 2395 | N | ARG | A | 354 | 2.135 | -24.220 | -1.069 | 1.00 | 54.46 | N |
| ATOM | 2396 | CA | ARG | A | 354 | 1.629 | -23.835 | 0.250 | 1.00 | 53.63 | C |
| ATOM | 2397 | CB | ARG | A | 354 | 2.629 | -22.881 | 0.917 | 1.00 | 55.58 | C |
| ATOM | 2398 | CG | ARG | A | 354 | 4.062 | -23.423 | 0.967 | 1.00 | 59.33 | C |
| ATOM | 2399 | CD | ARG | A | 354 | 5.099 | -22.365 | 1.406 | 1.00 | 60.91 | C |
| ATOM | 2400 | NE | ARG | A | 354 | 4.649 | -21.593 | 2.570 | 1.00 | 62.44 | N |
| ATOM | 2401 | CZ | ARG | A | 354 | 5.445 | -21.140 | 3.535 | 1.00 | 62.67 | C |
| ATOM | 2402 | NH1 | ARG | A | 354 | 6.754 | -21.381 | 3.494 | 1.00 | 62.47 | N |
| ATOM | 2403 | NH2 | ARG | A | 354 | 4.923 | -20.443 | 4.541 | 1.00 | 62.38 | N |
| ATOM | 2404 | C | ARG | A | 354 | 0.263 | -23.156 | 0.162 | 1.00 | 52.09 | C |
| ATOM | 2405 | O | ARG | A | 354 | -0.245 | -22.883 | -0.928 | 1.00 | 51.18 | O |

| | | | | | | | | | | | |
|---|---|---|---|---|---|---|---|---|---|---|---|
| ATOM | 2406 | N | ASP | A | 355 | -0.331 | -22.893 | 1.318 | 1.00 | 50.84 | N |
| ATOM | 2407 | CA | ASP | A | 355 | -1.624 | -22.235 | 1.352 | 1.00 | 50.82 | C |
| ATOM | 2408 | CB | ASP | A | 355 | -2.381 | -22.583 | 2.631 | 1.00 | 52.70 | C |
| ATOM | 2409 | CG | ASP | A | 355 | -2.837 | -24.023 | 2.655 | 1.00 | 54.54 | C |
| ATOM | 2410 | OD1 | ASP | A | 355 | -1.988 | -24.909 | 2.900 | 1.00 | 56.10 | O |
| ATOM | 2411 | OD2 | ASP | A | 355 | -4.042 | -24.271 | 2.411 | 1.00 | 56.26 | O |
| ATOM | 2412 | C | ASP | A | 355 | -1.479 | -20.728 | 1.243 | 1.00 | 49.55 | C |
| ATOM | 2413 | O | ASP | A | 355 | -0.440 | -20.157 | 1.585 | 1.00 | 48.02 | O |
| ATOM | 2414 | N | THR | A | 356 | -2.539 | -20.097 | 0.754 | 1.00 | 48.58 | N |
| ATOM | 2415 | CA | THR | A | 356 | -2.564 | -18.659 | 0.576 | 1.00 | 47.47 | C |
| ATOM | 2416 | CB | THR | A | 356 | -3.719 | -18.215 | -0.349 | 1.00 | 47.25 | C |
| ATOM | 2417 | OG1 | THR | A | 356 | -4.976 | -18.467 | 0.293 | 1.00 | 45.03 | O |
| ATOM | 2418 | CG2 | THR | A | 356 | -3.672 | -18.980 | -1.666 | 1.00 | 46.56 | C |
| ATOM | 2419 | C | THR | A | 356 | -2.780 | -18.014 | 1.918 | 1.00 | 47.60 | C |
| ATOM | 2420 | O | THR | A | 356 | -3.443 | -18.569 | 2.789 | 1.00 | 46.86 | O |
| ATOM | 2421 | N | PRO | A | 357 | -2.218 | -16.821 | 2.111 | 1.00 | 49.03 | N |
| ATOM | 2422 | CD | PRO | A | 357 | -1.441 | -15.987 | 1.179 | 1.00 | 49.37 | C |
| ATOM | 2423 | CA | PRO | A | 357 | -2.411 | -16.150 | 3.399 | 1.00 | 49.41 | C |
| ATOM | 2424 | CB | PRO | A | 357 | -1.671 | -14.819 | 3.215 | 1.00 | 48.92 | C |
| ATOM | 2425 | CG | PRO | A | 357 | -1.691 | -14.596 | 1.723 | 1.00 | 48.93 | C |
| ATOM | 2426 | C | PRO | A | 357 | -3.908 | -15.974 | 3.645 | 1.00 | 50.30 | C |
| ATOM | 2427 | O | PRO | A | 357 | -4.703 | -15.989 | 2.702 | 1.00 | 50.49 | O |
| ATOM | 2428 | N | ALA | A | 358 | -4.298 | -15.844 | 4.909 | 1.00 | 51.62 | N |
| ATOM | 2429 | CA | ALA | A | 358 | -5.706 | -15.658 | 5.250 | 1.00 | 52.20 | C |
| ATOM | 2430 | CB | ALA | A | 358 | -5.867 | -15.489 | 6.740 | 1.00 | 52.42 | C |
| ATOM | 2431 | C | ALA | A | 358 | -6.178 | -14.409 | 4.531 | 1.00 | 52.79 | C |
| ATOM | 2432 | O | ALA | A | 358 | -5.658 | -13.320 | 4.756 | 1.00 | 53.42 | O |
| ATOM | 2433 | N | LEU | A | 359 | -7.170 | -14.572 | 3.665 | 1.00 | 52.66 | N |
| ATOM | 2434 | CA | LEU | A | 359 | -7.685 | -13.458 | 2.890 | 1.00 | 51.24 | C |
| ATOM | 2435 | CB | LEU | A | 359 | -7.547 | -13.796 | 1.413 | 1.00 | 50.57 | C |
| ATOM | 2436 | CG | LEU | A | 359 | -6.092 | -14.061 | 1.057 | 1.00 | 51.11 | C |
| ATOM | 2437 | CD1 | LEU | A | 359 | -5.981 | -14.749 | -0.305 | 1.00 | 51.48 | C |
| ATOM | 2438 | CD2 | LEU | A | 359 | -5.343 | -12.732 | 1.083 | 1.00 | 49.61 | C |
| ATOM | 2439 | C | LEU | A | 359 | -9.139 | -13.162 | 3.208 | 1.00 | 51.43 | C |
| ATOM | 2440 | O | LEU | A | 359 | -9.751 | -12.301 | 2.570 | 1.00 | 50.32 | O |
| ATOM | 2441 | N | PHE | A | 360 | -9.674 | -13.851 | 4.217 | 1.00 | 50.84 | N |
| ATOM | 2442 | CA | PHE | A | 360 | -11.082 | -13.712 | 4.570 | 1.00 | 50.76 | C |
| ATOM | 2443 | CB | PHE | A | 360 | -11.777 | -15.045 | 4.289 | 1.00 | 50.34 | C |
| ATOM | 2444 | CG | PHE | A | 360 | -11.444 | -15.610 | 2.945 | 1.00 | 50.85 | C |
| ATOM | 2445 | CD1 | PHE | A | 360 | -11.719 | -14.882 | 1.793 | 1.00 | 50.38 | C |
| ATOM | 2446 | CD2 | PHE | A | 360 | -10.821 | -16.851 | 2.825 | 1.00 | 51.14 | C |
| ATOM | 2447 | CE1 | PHE | A | 360 | -11.384 | -15.378 | 0.538 | 1.00 | 50.42 | C |
| ATOM | 2448 | CE2 | PHE | A | 360 | -10.481 | -17.354 | 1.570 | 1.00 | 50.65 | C |
| ATOM | 2449 | CZ | PHE | A | 360 | -10.763 | -16.614 | 0.425 | 1.00 | 50.63 | C |
| ATOM | 2450 | C | PHE | A | 360 | -11.449 | -13.229 | 5.966 | 1.00 | 50.59 | C |
| ATOM | 2451 | O | PHE | A | 360 | -12.628 | -12.987 | 6.239 | 1.00 | 50.18 | O |
| ATOM | 2452 | N | ASN | A | 361 | -10.460 | -13.071 | 6.840 | 1.00 | 50.45 | N |
| ATOM | 2453 | CA | ASN | A | 361 | -10.729 | -12.626 | 8.207 | 1.00 | 50.61 | C |
| ATOM | 2454 | CB | ASN | A | 361 | -9.578 | -13.042 | 9.142 | 1.00 | 49.90 | C |
| ATOM | 2455 | CG | ASN | A | 361 | -8.240 | -12.440 | 8.752 | 1.00 | 50.50 | C |
| ATOM | 2456 | OD1 | ASN | A | 361 | -7.924 | -12.292 | 7.567 | 1.00 | 50.00 | O |
| ATOM | 2457 | ND2 | ASN | A | 361 | -7.430 | -12.112 | 9.756 | 1.00 | 50.48 | N |
| ATOM | 2458 | C | ASN | A | 361 | -10.996 | -11.129 | 8.302 | 1.00 | 50.96 | C |
| ATOM | 2459 | O | ASN | A | 361 | -10.162 | -10.356 | 8.775 | 1.00 | 52.45 | O |
| ATOM | 2460 | N | PHE | A | 362 | -12.184 | -10.737 | 7.857 | 1.00 | 51.21 | N |
| ATOM | 2461 | CA | PHE | A | 362 | -12.606 | -9.346 | 7.876 | 1.00 | 50.59 | C |
| ATOM | 2462 | CB | PHE | A | 362 | -13.675 | -9.122 | 6.810 | 1.00 | 47.78 | C |
| ATOM | 2463 | CG | PHE | A | 362 | -13.130 | -9.049 | 5.421 | 1.00 | 44.70 | C |
| ATOM | 2464 | CD1 | PHE | A | 362 | -12.676 | -7.838 | 4.907 | 1.00 | 42.85 | C |
| ATOM | 2465 | CD2 | PHE | A | 362 | -13.040 | -10.191 | 4.633 | 1.00 | 43.06 | C |
| ATOM | 2466 | CE1 | PHE | A | 362 | -12.143 | -7.767 | 3.630 | 1.00 | 41.42 | C |
| ATOM | 2467 | CE2 | PHE | A | 362 | -12.508 | -10.127 | 3.354 | 1.00 | 42.51 | C |
| ATOM | 2468 | CZ | PHE | A | 362 | -12.057 | -8.913 | 2.848 | 1.00 | 40.79 | C |
| ATOM | 2469 | C | PHE | A | 362 | -13.162 | -8.944 | 9.230 | 1.00 | 51.70 | C |
| ATOM | 2470 | O | PHE | A | 362 | -13.708 | -9.773 | 9.955 | 1.00 | 52.49 | O |
| ATOM | 2471 | N | THR | A | 363 | -13.015 | -7.665 | 9.561 | 1.00 | 52.86 | N |
| ATOM | 2472 | CA | THR | A | 363 | -13.513 | -7.111 | 10.812 | 1.00 | 54.37 | C |

| | | | | | | | | | | | |
|---|---|---|---|---|---|---|---|---|---|---|---|
| ATOM | 2473 | CB | THR | A | 363 | -12.428 | -6.309 | 11.523 | 1.00 | 54.67 | C |
| ATOM | 2474 | OG1 | THR | A | 363 | -11.932 | -5.299 | 10.632 | 1.00 | 55.22 | O |
| ATOM | 2475 | CG2 | THR | A | 363 | -11.288 | -7.214 | 11.953 | 1.00 | 53.48 | C |
| ATOM | 2476 | C | THR | A | 363 | -14.630 | -6.142 | 10.441 | 1.00 | 56.47 | C |
| ATOM | 2477 | O | THR | A | 363 | -14.671 | -5.647 | 9.308 | 1.00 | 57.48 | O |
| ATOM | 2478 | N | THR | A | 364 | -15.528 | -5.859 | 11.383 | 1.00 | 57.25 | N |
| ATOM | 2479 | CA | THR | A | 364 | -16.636 | -4.943 | 11.115 | 1.00 | 57.85 | C |
| ATOM | 2480 | CB | THR | A | 364 | -17.498 | -4.674 | 12.382 | 1.00 | 58.49 | C |
| ATOM | 2481 | OG1 | THR | A | 364 | -16.833 | -3.728 | 13.235 | 1.00 | 59.58 | O |
| ATOM | 2482 | CG2 | THR | A | 364 | -17.736 | -5.971 | 13.152 | 1.00 | 57.89 | C |
| ATOM | 2483 | C | THR | A | 364 | -16.117 | -3.607 | 10.581 | 1.00 | 57.62 | C |
| ATOM | 2484 | O | THR | A | 364 | -16.735 | -2.989 | 9.711 | 1.00 | 58.28 | O |
| ATOM | 2485 | N | GLN | A | 365 | -14.970 | -3.170 | 11.093 | 1.00 | 56.58 | N |
| ATOM | 2486 | CA | GLN | A | 365 | -14.370 | -1.921 | 10.641 | 1.00 | 55.26 | C |
| ATOM | 2487 | CB | GLN | A | 365 | -13.097 | -1.634 | 11.444 | 1.00 | 56.54 | C |
| ATOM | 2488 | CG | GLN | A | 365 | -12.304 | -0.409 | 10.984 | 1.00 | 58.74 | C |
| ATOM | 2489 | CD | GLN | A | 365 | -12.975 | 0.905 | 11.339 | 1.00 | 59.20 | C |
| ATOM | 2490 | OE1 | GLN | A | 365 | -13.389 | 1.108 | 12.480 | 1.00 | 60.19 | O |
| ATOM | 2491 | NE2 | GLN | A | 365 | -13.064 | 1.811 | 10.374 | 1.00 | 58.78 | N |
| ATOM | 2492 | C | GLN | A | 365 | -14.040 | -2.010 | 9.144 | 1.00 | 54.58 | C |
| ATOM | 2493 | O | GLN | A | 365 | -14.421 | -1.136 | 8.364 | 1.00 | 54.08 | O |
| ATOM | 2494 | N | GLU | A | 366 | -13.336 | -3.067 | 8.748 | 1.00 | 54.01 | N |
| ATOM | 2495 | CA | GLU | A | 366 | -12.962 | -3.255 | 7.344 | 1.00 | 53.44 | C |
| ATOM | 2496 | CB | GLU | A | 366 | -12.259 | -4.600 | 7.133 | 1.00 | 53.01 | C |
| ATOM | 2497 | CG | GLU | A | 366 | -10.984 | -4.816 | 7.928 | 1.00 | 52.95 | C |
| ATOM | 2498 | CD | GLU | A | 366 | -10.214 | -6.037 | 7.453 | 1.00 | 52.66 | C |
| ATOM | 2499 | OE1 | GLU | A | 366 | -9.703 | -6.009 | 6.312 | 1.00 | 50.19 | O |
| ATOM | 2500 | OE2 | GLU | A | 366 | -10.122 | -7.024 | 8.221 | 1.00 | 53.80 | O |
| ATOM | 2501 | C | GLU | A | 366 | -14.190 | -3.230 | 6.448 | 1.00 | 53.60 | C |
| ATOM | 2502 | O | GLU | A | 366 | -14.209 | -2.579 | 5.398 | 1.00 | 53.96 | O |
| ATOM | 2503 | N | LEU | A | 367 | -15.214 | -3.963 | 6.865 | 1.00 | 53.38 | N |
| ATOM | 2504 | CA | LEU | A | 367 | -16.451 | -4.059 | 6.100 | 1.00 | 53.62 | C |
| ATOM | 2505 | CB | LEU | A | 367 | -17.166 | -5.368 | 6.471 | 1.00 | 52.99 | C |
| ATOM | 2506 | CG | LEU | A | 367 | -16.446 | -6.644 | 6.008 | 1.00 | 52.59 | C |
| ATOM | 2507 | CD1 | LEU | A | 367 | -17.078 | -7.883 | 6.617 | 1.00 | 51.21 | C |
| ATOM | 2508 | CD2 | LEU | A | 367 | -16.492 | -6.712 | 4.491 | 1.00 | 52.13 | C |
| ATOM | 2509 | C | LEU | A | 367 | -17.386 | -2.860 | 6.305 | 1.00 | 53.60 | C |
| ATOM | 2510 | O | LEU | A | 367 | -18.321 | -2.649 | 5.528 | 1.00 | 53.51 | O |
| ATOM | 2511 | N | SER | A | 368 | -17.101 | -2.062 | 7.331 | 1.00 | 53.33 | N |
| ATOM | 2512 | CA | SER | A | 368 | -17.919 | -0.903 | 7.676 | 1.00 | 52.85 | C |
| ATOM | 2513 | CB | SER | A | 368 | -17.119 | 0.077 | 8.557 | 1.00 | 52.99 | C |
| ATOM | 2514 | OG | SER | A | 368 | -16.008 | 0.642 | 7.873 | 1.00 | 53.02 | O |
| ATOM | 2515 | C | SER | A | 368 | -18.573 | -0.132 | 6.524 | 1.00 | 52.89 | C |
| ATOM | 2516 | O | SER | A | 368 | -19.766 | 0.177 | 6.588 | 1.00 | 53.19 | O |
| ATOM | 2517 | N | SER | A | 369 | -17.823 | 0.162 | 5.467 | 1.00 | 52.95 | N |
| ATOM | 2518 | CA | SER | A | 369 | -18.375 | 0.937 | 4.347 | 1.00 | 52.47 | C |
| ATOM | 2519 | CB | SER | A | 369 | -17.262 | 1.366 | 3.390 | 1.00 | 51.91 | C |
| ATOM | 2520 | OG | SER | A | 369 | -16.787 | 0.242 | 2.664 | 1.00 | 52.87 | O |
| ATOM | 2521 | C | SER | A | 369 | -19.459 | 0.253 | 3.524 | 1.00 | 51.90 | C |
| ATOM | 2522 | O | SER | A | 369 | -20.246 | 0.925 | 2.855 | 1.00 | 51.56 | O |
| ATOM | 2523 | N | ASN | A | 370 | -19.501 | -1.076 | 3.552 | 1.00 | 52.16 | N |
| ATOM | 2524 | CA | ASN | A | 370 | -20.496 | -1.800 | 2.759 | 1.00 | 52.03 | C |
| ATOM | 2525 | CB | ASN | A | 370 | -20.066 | -1.813 | 1.295 | 1.00 | 51.11 | C |
| ATOM | 2526 | CG | ASN | A | 370 | -21.174 | -2.263 | 0.368 | 1.00 | 50.56 | C |
| ATOM | 2527 | OD1 | ASN | A | 370 | -22.125 | -2.923 | 0.791 | 1.00 | 49.80 | O |
| ATOM | 2528 | ND2 | ASN | A | 370 | -21.052 | -1.918 | -0.911 | 1.00 | 50.20 | N |
| ATOM | 2529 | C | ASN | A | 370 | -20.670 | -3.234 | 3.249 | 1.00 | 52.70 | C |
| ATOM | 2530 | O | ASN | A | 370 | -20.342 | -4.194 | 2.541 | 1.00 | 53.06 | O |
| ATOM | 2531 | N | PRO | A | 371 | -21.212 | -3.400 | 4.464 | 1.00 | 53.16 | N |
| ATOM | 2532 | CD | PRO | A | 371 | -21.704 | -2.298 | 5.314 | 1.00 | 52.77 | C |
| ATOM | 2533 | CA | PRO | A | 371 | -21.451 | -4.700 | 5.104 | 1.00 | 53.91 | C |
| ATOM | 2534 | CB | PRO | A | 371 | -22.478 | -4.355 | 6.176 | 1.00 | 54.19 | C |
| ATOM | 2535 | CG | PRO | A | 371 | -22.003 | -3.001 | 6.620 | 1.00 | 53.91 | C |
| ATOM | 2536 | C | PRO | A | 371 | -21.892 | -5.873 | 4.205 | 1.00 | 54.75 | C |
| ATOM | 2537 | O | PRO | A | 371 | -21.268 | -6.939 | 4.210 | 1.00 | 54.39 | O |
| ATOM | 2538 | N | PRO | A | 372 | -22.965 | -5.692 | 3.417 | 1.00 | 55.63 | N |
| ATOM | 2539 | CD | PRO | A | 372 | -23.717 | -4.453 | 3.145 | 1.00 | 55.86 | C |

| | | | | | | | | | | | |
|---|---|---|---|---|---|---|---|---|---|---|---|
| ATOM | 2540 | CA | PRO | A | 372 | -23.426 | -6.781 | 2.550 | 1.00 | 55.46 | C |
| ATOM | 2541 | CB | PRO | A | 372 | -24.550 | -6.130 | 1.743 | 1.00 | 55.55 | C |
| ATOM | 2542 | CG | PRO | A | 372 | -24.174 | -4.682 | 1.727 | 1.00 | 56.17 | C |
| ATOM | 2543 | C | PRO | A | 372 | -22.360 | -7.426 | 1.664 | 1.00 | 55.95 | C |
| ATOM | 2544 | O | PRO | A | 372 | -22.501 | -8.582 | 1.252 | 1.00 | 56.38 | O |
| ATOM | 2545 | N | LEU | A | 373 | -21.294 | -6.693 | 1.363 | 1.00 | 55.17 | N |
| ATOM | 2546 | CA | LEU | A | 373 | -20.230 | -7.243 | 0.523 | 1.00 | 54.68 | C |
| ATOM | 2547 | CB | LEU | A | 373 | -19.148 | -6.195 | 0.285 | 1.00 | 54.03 | C |
| ATOM | 2548 | CG | LEU | A | 373 | -19.512 | -5.266 | -0.871 | 1.00 | 53.60 | C |
| ATOM | 2549 | CD1 | LEU | A | 373 | -18.587 | -4.047 | -0.899 | 1.00 | 53.35 | C |
| ATOM | 2550 | CD2 | LEU | A | 373 | -19.411 | -6.060 | -2.160 | 1.00 | 52.65 | C |
| ATOM | 2551 | C | LEU | A | 373 | -19.618 | -8.478 | 1.156 | 1.00 | 54.91 | C |
| ATOM | 2552 | O | LEU | A | 373 | -18.864 | -9.214 | 0.513 | 1.00 | 54.84 | O |
| ATOM | 2553 | N | ALA | A | 374 | -19.962 | -8.703 | 2.419 | 1.00 | 55.50 | N |
| ATOM | 2554 | CA | ALA | A | 374 | -19.453 | -9.842 | 3.172 | 1.00 | 56.71 | C |
| ATOM | 2555 | CB | ALA | A | 374 | -19.910 | -9.745 | 4.611 | 1.00 | 55.32 | C |
| ATOM | 2556 | C | ALA | A | 374 | -19.885 | -11.174 | 2.580 | 1.00 | 57.62 | C |
| ATOM | 2557 | O | ALA | A | 374 | -19.199 | -12.186 | 2.733 | 1.00 | 58.36 | O |
| ATOM | 2558 | N | THR | A | 375 | -21.035 | -11.177 | 1.917 | 1.00 | 59.03 | N |
| ATOM | 2559 | CA | THR | A | 375 | -21.536 | -12.400 | 1.310 | 1.00 | 60.08 | C |
| ATOM | 2560 | CB | THR | A | 375 | -22.986 | -12.228 | 0.822 | 1.00 | 61.26 | C |
| ATOM | 2561 | OG1 | THR | A | 375 | -23.039 | -11.181 | -0.159 | 1.00 | 63.23 | O |
| ATOM | 2562 | CG2 | THR | A | 375 | -23.904 | -11.872 | 1.992 | 1.00 | 60.95 | C |
| ATOM | 2563 | C | THR | A | 375 | -20.657 | -12.801 | 0.133 | 1.00 | 59.42 | C |
| ATOM | 2564 | O | THR | A | 375 | -20.618 | -13.972 | -0.245 | 1.00 | 59.35 | O |
| ATOM | 2565 | N | ILE | A | 376 | -19.950 | -11.829 | -0.443 | 1.00 | 59.10 | N |
| ATOM | 2566 | CA | ILE | A | 376 | -19.065 | -12.118 | -1.571 | 1.00 | 58.87 | C |
| ATOM | 2567 | CB | ILE | A | 376 | -19.066 | -11.004 | -2.630 | 1.00 | 58.90 | C |
| ATOM | 2568 | CG2 | ILE | A | 376 | -18.577 | -11.580 | -3.964 | 1.00 | 59.59 | C |
| ATOM | 2569 | CG1 | ILE | A | 376 | -20.467 | -10.410 | -2.795 | 1.00 | 58.91 | C |
| ATOM | 2570 | CD1 | ILE | A | 376 | -20.495 | -9.205 | -3.732 | 1.00 | 57.97 | C |
| ATOM | 2571 | C | ILE | A | 376 | -17.621 | -12.267 | -1.121 | 1.00 | 58.37 | C |
| ATOM | 2572 | O | ILE | A | 376 | -16.918 | -13.188 | -1.544 | 1.00 | 58.72 | O |
| ATOM | 2573 | N | LEU | A | 377 | -17.189 | -11.347 | -0.261 | 1.00 | 57.31 | N |
| ATOM | 2574 | CA | LEU | A | 377 | -15.816 | -11.328 | 0.241 | 1.00 | 55.73 | C |
| ATOM | 2575 | CB | LEU | A | 377 | -15.561 | -10.021 | 0.990 | 1.00 | 53.43 | C |
| ATOM | 2576 | CG | LEU | A | 377 | -15.851 | -8.750 | 0.194 | 1.00 | 51.99 | C |
| ATOM | 2577 | CD1 | LEU | A | 377 | -15.803 | -7.562 | 1.123 | 1.00 | 53.19 | C |
| ATOM | 2578 | CD2 | LEU | A | 377 | -14.854 | -8.603 | -0.933 | 1.00 | 50.62 | C |
| ATOM | 2579 | C | LEU | A | 377 | -15.483 | -12.503 | 1.145 | 1.00 | 55.73 | C |
| ATOM | 2580 | O | LEU | A | 377 | -14.343 | -12.977 | 1.162 | 1.00 | 55.48 | O |
| ATOM | 2581 | N | ILE | A | 378 | -16.474 | -12.956 | 1.906 | 1.00 | 56.09 | N |
| ATOM | 2582 | CA | ILE | A | 378 | -16.289 | -14.080 | 2.814 | 1.00 | 56.91 | C |
| ATOM | 2583 | CB | ILE | A | 378 | -16.880 | -13.797 | 4.212 | 1.00 | 55.69 | C |
| ATOM | 2584 | CG2 | ILE | A | 378 | -16.492 | -14.923 | 5.169 | 1.00 | 55.43 | C |
| ATOM | 2585 | CG1 | ILE | A | 378 | -16.358 | -12.467 | 4.749 | 1.00 | 54.61 | C |
| ATOM | 2586 | CD1 | ILE | A | 378 | -16.812 | -12.162 | 6.163 | 1.00 | 54.48 | C |
| ATOM | 2587 | C | ILE | A | 378 | -17.011 | -15.276 | 2.215 | 1.00 | 58.44 | C |
| ATOM | 2588 | O | ILE | A | 378 | -18.211 | -15.468 | 2.444 | 1.00 | 58.49 | O |
| ATOM | 2589 | N | PRO | A | 379 | -16.287 | -16.103 | 1.445 | 1.00 | 59.68 | N |
| ATOM | 2590 | CD | PRO | A | 379 | -14.817 | -16.157 | 1.322 | 1.00 | 59.74 | C |
| ATOM | 2591 | CA | PRO | A | 379 | -16.906 | -17.274 | 0.821 | 1.00 | 60.91 | C |
| ATOM | 2592 | CB | PRO | A | 379 | -15.763 | -17.873 | 0.011 | 1.00 | 60.81 | C |
| ATOM | 2593 | CG | PRO | A | 379 | -14.580 | -17.587 | 0.889 | 1.00 | 60.55 | C |
| ATOM | 2594 | C | PRO | A | 379 | -17.460 | -18.238 | 1.852 | 1.00 | 62.30 | C |
| ATOM | 2595 | O | PRO | A | 379 | -17.127 | -18.165 | 3.035 | 1.00 | 63.05 | O |
| ATOM | 2596 | N | PRO | A | 380 | -18.329 | -19.149 | 1.417 | 1.00 | 63.51 | N |
| ATOM | 2597 | CD | PRO | A | 380 | -18.894 | -19.214 | 0.058 | 1.00 | 64.61 | C |
| ATOM | 2598 | CA | PRO | A | 380 | -18.951 | -20.147 | 2.289 | 1.00 | 64.32 | C |
| ATOM | 2599 | CB | PRO | A | 380 | -19.811 | -20.957 | 1.318 | 1.00 | 65.00 | C |
| ATOM | 2600 | CG | PRO | A | 380 | -20.206 | -19.940 | 0.298 | 1.00 | 65.00 | C |
| ATOM | 2601 | C | PRO | A | 380 | -17.934 | -21.024 | 3.017 | 1.00 | 64.27 | C |
| ATOM | 2602 | O | PRO | A | 380 | -17.966 | -21.146 | 4.242 | 1.00 | 63.29 | O |
| ATOM | 2603 | N | HIS | A | 381 | -17.027 | -21.621 | 2.252 | 1.00 | 65.26 | N |
| ATOM | 2604 | CA | HIS | A | 381 | -16.015 | -22.509 | 2.816 | 1.00 | 66.98 | C |
| ATOM | 2605 | CB | HIS | A | 381 | -15.143 | -23.070 | 1.701 | 1.00 | 67.65 | C |
| ATOM | 2606 | CG | HIS | A | 381 | -14.133 | -22.098 | 1.188 | 1.00 | 69.29 | C |

| | | | | | | | | | | | |
|---|---|---|---|---|---|---|---|---|---|---|---|
| ATOM | 2607 | CD2 | HIS | A | 381 | -14.127 | -21.319 | 0.079 | 1.00 | 70.04 | C |
| ATOM | 2608 | ND1 | HIS | A | 381 | -12.954 | -21.832 | 1.854 | 1.00 | 69.56 | N |
| ATOM | 2609 | CE1 | HIS | A | 381 | -12.264 | -20.931 | 1.174 | 1.00 | 70.10 | C |
| ATOM | 2610 | NE2 | HIS | A | 381 | -12.951 | -20.603 | 0.094 | 1.00 | 70.09 | N |
| ATOM | 2611 | C | HIS | A | 381 | -15.127 | -21.813 | 3.843 | 1.00 | 68.17 | C |
| ATOM | 2612 | O | HIS | A | 381 | -14.208 | -22.422 | 4.394 | 1.00 | 67.88 | O |
| ATOM | 2613 | N | ALA | A | 382 | -15.393 | -20.528 | 4.084 | 1.00 | 70.08 | N |
| ATOM | 2614 | CA | ALA | A | 382 | -14.631 | -19.744 | 5.057 | 1.00 | 71.17 | C |
| ATOM | 2615 | CB | ALA | A | 382 | -14.318 | -18.373 | 4.491 | 1.00 | 70.98 | C |
| ATOM | 2616 | C | ALA | A | 382 | -15.428 | -19.618 | 6.349 | 1.00 | 72.43 | C |
| ATOM | 2617 | O | ALA | A | 382 | -14.911 | -19.885 | 7.434 | 1.00 | 73.01 | O |
| ATOM | 2618 | N | ARG | A | 383 | -16.691 | -19.214 | 6.228 | 1.00 | 73.90 | N |
| ATOM | 2619 | CA | ARG | A | 383 | -17.571 | -19.082 | 7.388 | 1.00 | 74.74 | C |
| ATOM | 2620 | CB | ARG | A | 383 | -18.968 | -18.602 | 6.970 | 1.00 | 74.73 | C |
| ATOM | 2621 | CG | ARG | A | 383 | -19.019 | -17.178 | 6.450 | 1.00 | 75.00 | C |
| ATOM | 2622 | CD | ARG | A | 383 | -19.417 | -17.126 | 4.980 | 1.00 | 75.80 | C |
| ATOM | 2623 | NE | ARG | A | 383 | -20.757 | -17.673 | 4.746 | 1.00 | 76.47 | N |
| ATOM | 2624 | CZ | ARG | A | 383 | -21.480 | -17.447 | 3.650 | 1.00 | 76.89 | C |
| ATOM | 2625 | NH1 | ARG | A | 383 | -20.998 | -16.680 | 2.678 | 1.00 | 77.31 | N |
| ATOM | 2626 | NH2 | ARG | A | 383 | -22.687 | -17.989 | 3.529 | 1.00 | 77.31 | N |
| ATOM | 2627 | C | ARG | A | 383 | -17.696 | -20.430 | 8.075 | 1.00 | 75.86 | C |
| ATOM | 2628 | O | ARG | A | 383 | -17.685 | -20.461 | 9.334 | 1.00 | 76.70 | O |
| ATOM | 2629 | OXT | ARG | A | 383 | -17.825 | -21.433 | 7.334 | 1.00 | 76.46 | O |
| TER | 2630 | | ARG | A | 383 | | | | | | |
| ATOM | 2631 | CB | VAL | B | 37 | -20.058 | 29.474 | 41.282 | 1.00 | 50.23 | C |
| ATOM | 2632 | CG1 | VAL | B | 37 | -19.164 | 30.434 | 42.065 | 1.00 | 51.03 | C |
| ATOM | 2633 | CG2 | VAL | B | 37 | -19.539 | 29.356 | 39.846 | 1.00 | 50.70 | C |
| ATOM | 2634 | C | VAL | B | 37 | -20.007 | 28.289 | 43.475 | 1.00 | 49.38 | C |
| ATOM | 2635 | O | VAL | B | 37 | -20.999 | 28.679 | 44.082 | 1.00 | 49.30 | O |
| ATOM | 2636 | N | VAL | B | 37 | -21.220 | 27.264 | 41.530 | 1.00 | 49.26 | N |
| ATOM | 2637 | CA | VAL | B | 37 | -20.054 | 28.084 | 41.961 | 1.00 | 49.59 | C |
| ATOM | 2638 | N | THR | B | 38 | -18.852 | 28.012 | 44.080 | 1.00 | 48.95 | N |
| ATOM | 2639 | CA | THR | B | 38 | -18.669 | 28.187 | 45.522 | 1.00 | 47.55 | C |
| ATOM | 2640 | CB | THR | B | 38 | -18.120 | 26.908 | 46.209 | 1.00 | 48.42 | C |
| ATOM | 2641 | OG1 | THR | B | 38 | -18.993 | 25.801 | 45.956 | 1.00 | 50.13 | O |
| ATOM | 2642 | CG2 | THR | B | 38 | -18.014 | 27.122 | 47.718 | 1.00 | 46.95 | C |
| ATOM | 2643 | C | THR | B | 38 | -17.652 | 29.302 | 45.755 | 1.00 | 46.11 | C |
| ATOM | 2644 | O | THR | B | 38 | -16.672 | 29.422 | 45.016 | 1.00 | 44.42 | O |
| ATOM | 2645 | N | THR | B | 39 | -17.902 | 30.123 | 46.773 | 1.00 | 44.03 | N |
| ATOM | 2646 | CA | THR | B | 39 | -16.994 | 31.218 | 47.120 | 1.00 | 42.02 | C |
| ATOM | 2647 | CB | THR | B | 39 | -17.583 | 32.610 | 46.761 | 1.00 | 41.11 | C |
| ATOM | 2648 | OG1 | THR | B | 39 | -17.682 | 32.741 | 45.339 | 1.00 | 39.79 | O |
| ATOM | 2649 | CG2 | THR | B | 39 | -16.674 | 33.718 | 47.294 | 1.00 | 40.13 | C |
| ATOM | 2650 | C | THR | B | 39 | -16.679 | 31.190 | 48.609 | 1.00 | 40.98 | C |
| ATOM | 2651 | O | THR | B | 39 | -17.575 | 31.194 | 49.452 | 1.00 | 41.19 | O |
| ATOM | 2652 | N | VAL | B | 40 | -15.394 | 31.159 | 48.925 | 1.00 | 39.56 | N |
| ATOM | 2653 | CA | VAL | B | 40 | -14.963 | 31.132 | 50.309 | 1.00 | 37.76 | C |
| ATOM | 2654 | CB | VAL | B | 40 | -14.394 | 29.753 | 50.679 | 1.00 | 37.56 | C |
| ATOM | 2655 | CG1 | VAL | B | 40 | -15.456 | 28.689 | 50.477 | 1.00 | 36.32 | C |
| ATOM | 2656 | CG2 | VAL | B | 40 | -13.168 | 29.451 | 49.838 | 1.00 | 34.19 | C |
| ATOM | 2657 | C | VAL | B | 40 | -13.883 | 32.174 | 50.505 | 1.00 | 37.38 | C |
| ATOM | 2658 | O | VAL | B | 40 | -13.223 | 32.588 | 49.553 | 1.00 | 37.63 | O |
| ATOM | 2659 | N | VAL | B | 41 | -13.721 | 32.618 | 51.738 | 1.00 | 36.86 | N |
| ATOM | 2660 | CA | VAL | B | 41 | -12.694 | 33.599 | 52.047 | 1.00 | 38.31 | C |
| ATOM | 2661 | CB | VAL | B | 41 | -13.173 | 34.565 | 53.166 | 1.00 | 38.32 | C |
| ATOM | 2662 | CG1 | VAL | B | 41 | -12.048 | 35.518 | 53.568 | 1.00 | 36.48 | C |
| ATOM | 2663 | CG2 | VAL | B | 41 | -14.380 | 35.353 | 52.676 | 1.00 | 36.89 | C |
| ATOM | 2664 | C | VAL | B | 41 | -11.530 | 32.744 | 52.533 | 1.00 | 38.42 | C |
| ATOM | 2665 | O | VAL | B | 41 | -11.577 | 32.201 | 53.635 | 1.00 | 39.50 | O |
| ATOM | 2666 | N | ALA | B | 42 | -10.504 | 32.595 | 51.704 | 1.00 | 38.73 | N |
| ATOM | 2667 | CA | ALA | B | 42 | -9.367 | 31.758 | 52.066 | 1.00 | 40.59 | C |
| ATOM | 2668 | CB | ALA | B | 42 | -9.336 | 30.531 | 51.164 | 1.00 | 41.05 | C |
| ATOM | 2669 | C | ALA | B | 42 | -8.016 | 32.459 | 52.024 | 1.00 | 40.82 | C |
| ATOM | 2670 | O | ALA | B | 42 | -7.743 | 33.256 | 51.124 | 1.00 | 41.64 | O |
| ATOM | 2671 | N | THR | B | 43 | -7.163 | 32.135 | 52.995 | 1.00 | 40.99 | N |
| ATOM | 2672 | CA | THR | B | 43 | -5.822 | 32.718 | 53.086 | 1.00 | 41.48 | C |
| ATOM | 2673 | CB | THR | B | 43 | -5.305 | 32.702 | 54.528 | 1.00 | 41.89 | C |

| | | | | | | | | | | | |
|---|---|---|---|---|---|---|---|---|---|---|---|
| ATOM | 2674 | OG1 | THR | B | 43 | -6.371 | 33.019 | 55.432 | 1.00 | 43.62 | O |
| ATOM | 2675 | CG2 | THR | B | 43 | -4.190 | 33.715 | 54.694 | 1.00 | 41.53 | C |
| ATOM | 2676 | C | THR | B | 43 | -4.838 | 31.910 | 52.244 | 1.00 | 42.26 | C |
| ATOM | 2677 | O | THR | B | 43 | -4.904 | 30.684 | 52.228 | 1.00 | 42.77 | O |
| ATOM | 2678 | N | PRO | B | 44 | -3.904 | 32.581 | 51.545 | 1.00 | 44.24 | N |
| ATOM | 2679 | CD | PRO | B | 44 | -3.765 | 34.035 | 51.378 | 1.00 | 43.55 | C |
| ATOM | 2680 | CA | PRO | B | 44 | -2.921 | 31.872 | 50.714 | 1.00 | 46.62 | C |
| ATOM | 2681 | CB | PRO | B | 44 | -2.147 | 33.006 | 50.039 | 1.00 | 45.72 | C |
| ATOM | 2682 | CG | PRO | B | 44 | -3.142 | 34.132 | 50.006 | 1.00 | 44.37 | C |
| ATOM | 2683 | C | PRO | B | 44 | -2.007 | 30.981 | 51.557 | 1.00 | 48.92 | C |
| ATOM | 2684 | O | PRO | B | 44 | -1.560 | 31.384 | 52.631 | 1.00 | 50.13 | O |
| ATOM | 2685 | N | GLY | B | 45 | -1.733 | 29.775 | 51.065 | 1.00 | 51.64 | N |
| ATOM | 2686 | CA | GLY | B | 45 | -0.887 | 28.848 | 51.797 | 1.00 | 55.52 | C |
| ATOM | 2687 | C | GLY | B | 45 | 0.452 | 29.452 | 52.151 | 1.00 | 58.29 | C |
| ATOM | 2688 | O | GLY | B | 45 | 0.861 | 29.454 | 53.314 | 1.00 | 57.94 | O |
| ATOM | 2689 | N | ASP | B | 46 | 1.144 | 29.961 | 51.142 | 1.00 | 61.47 | N |
| ATOM | 2690 | CA | ASP | B | 46 | 2.441 | 30.586 | 51.354 | 1.00 | 65.35 | C |
| ATOM | 2691 | CB | ASP | B | 46 | 3.532 | 29.834 | 50.576 | 1.00 | 67.14 | C |
| ATOM | 2692 | CG | ASP | B | 46 | 4.937 | 30.301 | 50.932 | 1.00 | 69.84 | C |
| ATOM | 2693 | OD1 | ASP | B | 46 | 5.887 | 29.939 | 50.194 | 1.00 | 70.82 | O |
| ATOM | 2694 | OD2 | ASP | B | 46 | 5.096 | 31.023 | 51.951 | 1.00 | 70.56 | O |
| ATOM | 2695 | C | ASP | B | 46 | 2.352 | 32.034 | 50.880 | 1.00 | 66.66 | C |
| ATOM | 2696 | O | ASP | B | 46 | 2.016 | 32.312 | 49.726 | 1.00 | 66.32 | O |
| ATOM | 2697 | N | GLY | B | 47 | 2.651 | 32.950 | 51.793 | 1.00 | 67.33 | N |
| ATOM | 2698 | CA | GLY | B | 47 | 2.609 | 34.360 | 51.469 | 1.00 | 67.70 | C |
| ATOM | 2699 | C | GLY | B | 47 | 2.164 | 35.194 | 52.650 | 1.00 | 68.35 | C |
| ATOM | 2700 | O | GLY | B | 47 | 2.301 | 34.765 | 53.804 | 1.00 | 69.52 | O |
| ATOM | 2701 | N | PRO | B | 48 | 1.629 | 36.402 | 52.393 | 1.00 | 67.98 | N |
| ATOM | 2702 | CD | PRO | B | 48 | 1.455 | 37.021 | 51.064 | 1.00 | 67.52 | C |
| ATOM | 2703 | CA | PRO | B | 48 | 1.162 | 37.298 | 53.450 | 1.00 | 66.30 | C |
| ATOM | 2704 | CB | PRO | B | 48 | 1.149 | 38.651 | 52.757 | 1.00 | 66.93 | C |
| ATOM | 2705 | CG | PRO | B | 48 | 0.672 | 38.289 | 51.388 | 1.00 | 67.24 | C |
| ATOM | 2706 | C | PRO | B | 48 | -0.221 | 36.847 | 53.888 | 1.00 | 65.17 | C |
| ATOM | 2707 | O | PRO | B | 48 | -1.131 | 36.721 | 53.069 | 1.00 | 64.50 | O |
| ATOM | 2708 | N | ASP | B | 49 | -0.362 | 36.577 | 55.179 | 1.00 | 64.17 | N |
| ATOM | 2709 | CA | ASP | B | 49 | -1.633 | 36.137 | 55.759 | 1.00 | 63.41 | C |
| ATOM | 2710 | CB | ASP | B | 49 | -1.449 | 35.939 | 57.269 | 1.00 | 64.50 | C |
| ATOM | 2711 | CG | ASP | B | 49 | -2.715 | 35.485 | 57.965 | 1.00 | 65.97 | C |
| ATOM | 2712 | OD1 | ASP | B | 49 | -3.726 | 36.224 | 57.894 | 1.00 | 66.87 | O |
| ATOM | 2713 | OD2 | ASP | B | 49 | -2.697 | 34.394 | 58.594 | 1.00 | 66.31 | O |
| ATOM | 2714 | C | ASP | B | 49 | -2.701 | 37.192 | 55.477 | 1.00 | 62.55 | C |
| ATOM | 2715 | O | ASP | B | 49 | -3.013 | 38.018 | 56.326 | 1.00 | 63.42 | O |
| ATOM | 2716 | N | ARG | B | 50 | -3.272 | 37.146 | 54.277 | 1.00 | 61.63 | N |
| ATOM | 2717 | CA | ARG | B | 50 | -4.278 | 38.124 | 53.855 | 1.00 | 59.75 | C |
| ATOM | 2718 | CB | ARG | B | 50 | -3.595 | 39.121 | 52.920 | 1.00 | 60.95 | C |
| ATOM | 2719 | CG | ARG | B | 50 | -4.148 | 40.531 | 52.938 | 1.00 | 64.27 | C |
| ATOM | 2720 | CD | ARG | B | 50 | -4.762 | 40.954 | 51.601 | 1.00 | 65.68 | C |
| ATOM | 2721 | NE | ARG | B | 50 | -3.909 | 40.711 | 50.439 | 1.00 | 67.78 | N |
| ATOM | 2722 | CZ | ARG | B | 50 | -3.797 | 39.536 | 49.815 | 1.00 | 70.13 | C |
| ATOM | 2723 | NH1 | ARG | B | 50 | -4.481 | 38.485 | 50.246 | 1.00 | 69.94 | N |
| ATOM | 2724 | NH2 | ARG | B | 50 | -3.029 | 39.420 | 48.732 | 1.00 | 70.44 | N |
| ATOM | 2725 | C | ARG | B | 50 | -5.435 | 37.418 | 53.136 | 1.00 | 57.59 | C |
| ATOM | 2726 | O | ARG | B | 50 | -5.366 | 37.185 | 51.928 | 1.00 | 57.78 | O |
| ATOM | 2727 | N | PRO | B | 51 | -6.522 | 37.090 | 53.864 | 1.00 | 54.47 | N |
| ATOM | 2728 | CD | PRO | B | 51 | -6.851 | 37.484 | 55.244 | 1.00 | 53.24 | C |
| ATOM | 2729 | CA | PRO | B | 51 | -7.654 | 36.403 | 53.232 | 1.00 | 52.05 | C |
| ATOM | 2730 | CB | PRO | B | 51 | -8.640 | 36.225 | 54.380 | 1.00 | 51.39 | C |
| ATOM | 2731 | CG | PRO | B | 51 | -8.360 | 37.411 | 55.239 | 1.00 | 52.69 | C |
| ATOM | 2732 | C | PRO | B | 51 | -8.269 | 37.121 | 52.034 | 1.00 | 50.97 | C |
| ATOM | 2733 | O | PRO | B | 51 | -8.447 | 38.340 | 52.035 | 1.00 | 51.40 | O |
| ATOM | 2734 | N | GLN | B | 52 | -8.574 | 36.341 | 51.000 | 1.00 | 49.50 | N |
| ATOM | 2735 | CA | GLN | B | 52 | -9.178 | 36.852 | 49.781 | 1.00 | 47.21 | C |
| ATOM | 2736 | CB | GLN | B | 52 | -8.121 | 37.039 | 48.688 | 1.00 | 47.79 | C |
| ATOM | 2737 | CG | GLN | B | 52 | -6.961 | 36.065 | 48.739 | 1.00 | 49.25 | C |
| ATOM | 2738 | CD | GLN | B | 52 | -5.873 | 36.399 | 47.720 | 1.00 | 51.16 | C |
| ATOM | 2739 | OE1 | GLN | B | 52 | -6.057 | 36.221 | 46.510 | 1.00 | 50.86 | O |
| ATOM | 2740 | NE2 | GLN | B | 52 | -4.735 | 36.892 | 48.206 | 1.00 | 51.80 | N |

| | | | | | | | | | | | |
|---|---|---|---|---|---|---|---|---|---|---|---|
| ATOM | 2741 | C | GLN | B | 52 | -10.240 | 35.894 | 49.314 | 1.00 | 46.22 | C |
| ATOM | 2742 | O | GLN | B | 52 | -10.154 | 34.689 | 49.561 | 1.00 | 46.33 | O |
| ATOM | 2743 | N | GLU | B | 53 | -11.260 | 36.437 | 48.667 | 1.00 | 45.98 | N |
| ATOM | 2744 | CA | GLU | B | 53 | -12.343 | 35.620 | 48.152 | 1.00 | 45.32 | C |
| ATOM | 2745 | CB | GLU | B | 53 | -13.448 | 36.504 | 47.558 | 1.00 | 45.45 | C |
| ATOM | 2746 | CG | GLU | B | 53 | -14.385 | 37.108 | 48.599 | 1.00 | 46.12 | C |
| ATOM | 2747 | CD | GLU | B | 53 | -15.479 | 37.960 | 47.989 | 1.00 | 47.39 | C |
| ATOM | 2748 | OE1 | GLU | B | 53 | -15.741 | 37.812 | 46.774 | 1.00 | 48.63 | O |
| ATOM | 2749 | OE2 | GLU | B | 53 | -16.088 | 38.766 | 48.726 | 1.00 | 46.63 | O |
| ATOM | 2750 | C | GLU | B | 53 | -11.766 | 34.711 | 47.085 | 1.00 | 44.63 | C |
| ATOM | 2751 | O | GLU | B | 53 | -10.939 | 35.126 | 46.280 | 1.00 | 43.68 | O |
| ATOM | 2752 | N | VAL | B | 54 | -12.194 | 33.459 | 47.104 | 1.00 | 43.86 | N |
| ATOM | 2753 | CA | VAL | B | 54 | -11.738 | 32.483 | 46.135 | 1.00 | 43.57 | C |
| ATOM | 2754 | CB | VAL | B | 54 | -10.728 | 31.510 | 46.760 | 1.00 | 44.48 | C |
| ATOM | 2755 | CG1 | VAL | B | 54 | -10.445 | 30.371 | 45.797 | 1.00 | 45.81 | C |
| ATOM | 2756 | CG2 | VAL | B | 54 | -9.443 | 32.245 | 47.115 | 1.00 | 43.58 | C |
| ATOM | 2757 | C | VAL | B | 54 | -12.944 | 31.693 | 45.649 | 1.00 | 44.32 | C |
| ATOM | 2758 | O | VAL | B | 54 | -13.739 | 31.196 | 46.451 | 1.00 | 43.53 | O |
| ATOM | 2759 | N | SER | B | 55 | -13.079 | 31.578 | 44.333 | 1.00 | 44.27 | N |
| ATOM | 2760 | CA | SER | B | 55 | -14.199 | 30.842 | 43.766 | 1.00 | 46.06 | C |
| ATOM | 2761 | CB | SER | B | 55 | -15.023 | 31.754 | 42.848 | 1.00 | 47.05 | C |
| ATOM | 2762 | OG | SER | B | 55 | -15.829 | 32.639 | 43.615 | 1.00 | 48.48 | O |
| ATOM | 2763 | C | SER | B | 55 | -13.786 | 29.588 | 43.005 | 1.00 | 46.10 | C |
| ATOM | 2764 | O | SER | B | 55 | -12.786 | 29.573 | 42.282 | 1.00 | 45.36 | O |
| ATOM | 2765 | N | TYR | B | 56 | -14.567 | 28.529 | 43.184 | 1.00 | 45.29 | N |
| ATOM | 2766 | CA | TYR | B | 56 | -14.314 | 27.260 | 42.520 | 1.00 | 45.49 | C |
| ATOM | 2767 | CB | TYR | B | 56 | -13.357 | 26.387 | 43.351 | 1.00 | 42.92 | C |
| ATOM | 2768 | CG | TYR | B | 56 | -13.875 | 25.962 | 44.714 | 1.00 | 39.85 | C |
| ATOM | 2769 | CD1 | TYR | B | 56 | -14.824 | 24.947 | 44.846 | 1.00 | 37.77 | C |
| ATOM | 2770 | CE1 | TYR | B | 56 | -15.295 | 24.555 | 46.102 | 1.00 | 36.53 | C |
| ATOM | 2771 | CD2 | TYR | B | 56 | -13.397 | 26.570 | 45.877 | 1.00 | 39.44 | C |
| ATOM | 2772 | CE2 | TYR | B | 56 | -13.857 | 26.190 | 47.131 | 1.00 | 38.51 | C |
| ATOM | 2773 | CZ | TYR | B | 56 | -14.801 | 25.176 | 47.239 | 1.00 | 38.47 | C |
| ATOM | 2774 | OH | TYR | B | 56 | -15.253 | 24.813 | 48.491 | 1.00 | 38.46 | O |
| ATOM | 2775 | C | TYR | B | 56 | -15.619 | 26.521 | 42.294 | 1.00 | 47.69 | C |
| ATOM | 2776 | O | TYR | B | 56 | -16.589 | 26.700 | 43.031 | 1.00 | 47.56 | O |
| ATOM | 2777 | N | THR | B | 57 | -15.636 | 25.686 | 41.262 | 1.00 | 49.56 | N |
| ATOM | 2778 | CA | THR | B | 57 | -16.821 | 24.904 | 40.931 | 1.00 | 50.95 | C |
| ATOM | 2779 | CB | THR | B | 57 | -17.699 | 25.652 | 39.899 | 1.00 | 51.11 | C |
| ATOM | 2780 | OG1 | THR | B | 57 | -18.944 | 24.963 | 39.743 | 1.00 | 50.33 | O |
| ATOM | 2781 | CG2 | THR | B | 57 | -16.990 | 25.747 | 38.553 | 1.00 | 51.34 | C |
| ATOM | 2782 | C | THR | B | 57 | -16.361 | 23.565 | 40.370 | 1.00 | 50.82 | C |
| ATOM | 2783 | O | THR | B | 57 | -15.173 | 23.379 | 40.131 | 1.00 | 51.70 | O |
| ATOM | 2784 | N | ASP | B | 58 | -17.295 | 22.640 | 40.169 | 1.00 | 51.07 | N |
| ATOM | 2785 | CA | ASP | B | 58 | -16.966 | 21.312 | 39.638 | 1.00 | 51.33 | C |
| ATOM | 2786 | CB | ASP | B | 58 | -15.870 | 21.394 | 38.563 | 1.00 | 52.10 | C |
| ATOM | 2787 | CG | ASP | B | 58 | -16.361 | 21.979 | 37.250 | 1.00 | 53.40 | C |
| ATOM | 2788 | OD1 | ASP | B | 58 | -17.157 | 22.943 | 37.282 | 1.00 | 55.47 | O |
| ATOM | 2789 | OD2 | ASP | B | 58 | -15.932 | 21.485 | 36.180 | 1.00 | 51.96 | O |
| ATOM | 2790 | C | ASP | B | 58 | -16.445 | 20.401 | 40.750 | 1.00 | 51.06 | C |
| ATOM | 2791 | O | ASP | B | 58 | -15.560 | 19.576 | 40.510 | 1.00 | 51.98 | O |
| ATOM | 2792 | N | THR | B | 59 | -16.983 | 20.527 | 41.956 | 1.00 | 49.48 | N |
| ATOM | 2793 | CA | THR | B | 59 | -16.480 | 19.710 | 43.050 | 1.00 | 48.62 | C |
| ATOM | 2794 | CB | THR | B | 59 | -16.592 | 20.456 | 44.394 | 1.00 | 47.90 | C |
| ATOM | 2795 | OG1 | THR | B | 59 | -17.932 | 20.368 | 44.883 | 1.00 | 49.33 | O |
| ATOM | 2796 | CG2 | THR | B | 59 | -16.215 | 21.910 | 44.211 | 1.00 | 47.03 | C |
| ATOM | 2797 | C | THR | B | 59 | -17.094 | 18.317 | 43.198 | 1.00 | 48.27 | C |
| ATOM | 2798 | O | THR | B | 59 | -18.307 | 18.134 | 43.100 | 1.00 | 48.06 | O |
| ATOM | 2799 | N | LYS | B | 60 | -16.218 | 17.340 | 43.436 | 1.00 | 47.86 | N |
| ATOM | 2800 | CA | LYS | B | 60 | -16.596 | 15.943 | 43.619 | 1.00 | 47.44 | C |
| ATOM | 2801 | CB | LYS | B | 60 | -16.632 | 15.206 | 42.274 | 1.00 | 46.79 | C |
| ATOM | 2802 | CG | LYS | B | 60 | -15.548 | 15.618 | 41.302 | 1.00 | 46.62 | C |
| ATOM | 2803 | CD | LYS | B | 60 | -15.066 | 14.453 | 40.461 | 1.00 | 48.50 | C |
| ATOM | 2804 | CE | LYS | B | 60 | -14.537 | 14.925 | 39.105 | 1.00 | 50.96 | C |
| ATOM | 2805 | NZ | LYS | B | 60 | -13.581 | 16.082 | 39.201 | 1.00 | 51.97 | N |
| ATOM | 2806 | C | LYS | B | 60 | -15.604 | 15.252 | 44.544 | 1.00 | 47.20 | C |
| ATOM | 2807 | O | LYS | B | 60 | -14.411 | 15.572 | 44.547 | 1.00 | 47.86 | O |

| | | | | | | | | | | | |
|---|---|---|---|---|---|---|---|---|---|---|---|
| ATOM | 2808 | N | VAL | B | 61 | -16.112 | 14.307 | 45.331 | 1.00 | 46.75 | N |
| ATOM | 2809 | CA | VAL | B | 61 | -15.301 | 13.543 | 46.270 | 1.00 | 45.23 | C |
| ATOM | 2810 | CB | VAL | B | 61 | -16.191 | 12.632 | 47.154 | 1.00 | 45.73 | C |
| ATOM | 2811 | CG1 | VAL | B | 61 | -15.330 | 11.623 | 47.917 | 1.00 | 43.58 | C |
| ATOM | 2812 | CG2 | VAL | B | 61 | -17.002 | 13.485 | 48.125 | 1.00 | 44.24 | C |
| ATOM | 2813 | C | VAL | B | 61 | -14.280 | 12.674 | 45.544 | 1.00 | 44.88 | C |
| ATOM | 2814 | O | VAL | B | 61 | -14.542 | 12.184 | 44.445 | 1.00 | 44.98 | O |
| ATOM | 2815 | N | ILE | B | 62 | -13.115 | 12.500 | 46.165 | 1.00 | 44.60 | N |
| ATOM | 2816 | CA | ILE | B | 62 | -12.041 | 11.679 | 45.610 | 1.00 | 43.97 | C |
| ATOM | 2817 | CB | ILE | B | 62 | -11.157 | 12.460 | 44.608 | 1.00 | 43.87 | C |
| ATOM | 2818 | CG2 | ILE | B | 62 | -12.006 | 13.030 | 43.483 | 1.00 | 42.75 | C |
| ATOM | 2819 | CG1 | ILE | B | 62 | -10.397 | 13.570 | 45.341 | 1.00 | 42.94 | C |
| ATOM | 2820 | CD1 | ILE | B | 62 | -9.227 | 14.121 | 44.552 | 1.00 | 41.53 | C |
| ATOM | 2821 | C | ILE | B | 62 | -11.114 | 11.178 | 46.719 | 1.00 | 43.91 | C |
| ATOM | 2822 | O | ILE | B | 62 | -10.076 | 10.581 | 46.434 | 1.00 | 43.11 | O |
| ATOM | 2823 | N | GLY | B | 63 | -11.484 | 11.409 | 47.978 | 1.00 | 43.69 | N |
| ATOM | 2824 | CA | GLY | B | 63 | -10.622 | 10.987 | 49.069 | 1.00 | 43.23 | C |
| ATOM | 2825 | C | GLY | B | 63 | -11.270 | 10.847 | 50.429 | 1.00 | 43.79 | C |
| ATOM | 2826 | O | GLY | B | 63 | -12.010 | 11.724 | 50.875 | 1.00 | 45.34 | O |
| ATOM | 2827 | N | ASN | B | 64 | -10.967 | 9.732 | 51.086 | 1.00 | 44.46 | N |
| ATOM | 2828 | CA | ASN | B | 64 | -11.482 | 9.401 | 52.407 | 1.00 | 43.82 | C |
| ATOM | 2829 | CB | ASN | B | 64 | -12.171 | 8.041 | 52.368 | 1.00 | 44.05 | C |
| ATOM | 2830 | CG | ASN | B | 64 | -13.621 | 8.118 | 52.734 | 1.00 | 46.12 | C |
| ATOM | 2831 | OD1 | ASN | B | 64 | -13.972 | 8.466 | 53.861 | 1.00 | 49.14 | O |
| ATOM | 2832 | ND2 | ASN | B | 64 | -14.485 | 7.786 | 51.790 | 1.00 | 48.46 | N |
| ATOM | 2833 | C | ASN | B | 64 | -10.293 | 9.313 | 53.363 | 1.00 | 44.10 | C |
| ATOM | 2834 | O | ASN | B | 64 | -9.134 | 9.247 | 52.944 | 1.00 | 43.02 | O |
| ATOM | 2835 | N | GLY | B | 65 | -10.587 | 9.277 | 54.652 | 1.00 | 43.26 | N |
| ATOM | 2836 | CA | GLY | B | 65 | -9.527 | 9.184 | 55.623 | 1.00 | 43.49 | C |
| ATOM | 2837 | C | GLY | B | 65 | -10.105 | 9.427 | 56.989 | 1.00 | 43.87 | C |
| ATOM | 2838 | O | GLY | B | 65 | -11.224 | 9.930 | 57.118 | 1.00 | 43.48 | O |
| ATOM | 2839 | N | SER | B | 66 | -9.350 | 9.061 | 58.013 | 1.00 | 45.12 | N |
| ATOM | 2840 | CA | SER | B | 66 | -9.809 | 9.260 | 59.380 | 1.00 | 46.67 | C |
| ATOM | 2841 | CB | SER | B | 66 | -8.745 | 8.751 | 60.349 | 1.00 | 47.44 | C |
| ATOM | 2842 | OG | SER | B | 66 | -7.446 | 9.107 | 59.892 | 1.00 | 53.51 | O |
| ATOM | 2843 | C | SER | B | 66 | -10.061 | 10.749 | 59.594 | 1.00 | 46.21 | C |
| ATOM | 2844 | O | SER | B | 66 | -10.865 | 11.142 | 60.435 | 1.00 | 46.25 | O |
| ATOM | 2845 | N | PHE | B | 67 | -9.377 | 11.563 | 58.797 | 1.00 | 45.88 | N |
| ATOM | 2846 | CA | PHE | B | 67 | -9.470 | 13.018 | 58.873 | 1.00 | 44.95 | C |
| ATOM | 2847 | CB | PHE | B | 67 | -8.207 | 13.624 | 58.278 | 1.00 | 44.32 | C |
| ATOM | 2848 | CG | PHE | B | 67 | -7.967 | 13.220 | 56.849 | 1.00 | 45.83 | C |
| ATOM | 2849 | CD1 | PHE | B | 67 | -8.642 | 13.852 | 55.805 | 1.00 | 44.35 | C |
| ATOM | 2850 | CD2 | PHE | B | 67 | -7.077 | 12.193 | 56.547 | 1.00 | 45.04 | C |
| ATOM | 2851 | CE1 | PHE | B | 67 | -8.430 | 13.468 | 54.490 | 1.00 | 43.46 | C |
| ATOM | 2852 | CE2 | PHE | B | 67 | -6.862 | 11.807 | 55.237 | 1.00 | 44.06 | C |
| ATOM | 2853 | CZ | PHE | B | 67 | -7.539 | 12.446 | 54.202 | 1.00 | 43.03 | C |
| ATOM | 2854 | C | PHE | B | 67 | -10.684 | 13.619 | 58.169 | 1.00 | 44.77 | C |
| ATOM | 2855 | O | PHE | B | 67 | -11.173 | 14.675 | 58.563 | 1.00 | 44.63 | O |
| ATOM | 2856 | N | GLY | B | 68 | -11.149 | 12.968 | 57.107 | 1.00 | 42.87 | N |
| ATOM | 2857 | CA | GLY | B | 68 | -12.293 | 13.499 | 56.401 | 1.00 | 41.22 | C |
| ATOM | 2858 | C | GLY | B | 68 | -12.415 | 13.129 | 54.940 | 1.00 | 41.15 | C |
| ATOM | 2859 | O | GLY | B | 68 | -12.229 | 11.970 | 54.562 | 1.00 | 42.58 | O |
| ATOM | 2860 | N | VAL | B | 69 | -12.721 | 14.128 | 54.113 | 1.00 | 39.68 | N |
| ATOM | 2861 | CA | VAL | B | 69 | -12.914 | 13.924 | 52.683 | 1.00 | 38.02 | C |
| ATOM | 2862 | CB | VAL | B | 69 | -14.375 | 14.250 | 52.286 | 1.00 | 36.66 | C |
| ATOM | 2863 | CG1 | VAL | B | 69 | -14.641 | 13.827 | 50.855 | 1.00 | 36.14 | C |
| ATOM | 2864 | CG2 | VAL | B | 69 | -15.336 | 13.577 | 53.246 | 1.00 | 35.31 | C |
| ATOM | 2865 | C | VAL | B | 69 | -11.990 | 14.792 | 51.832 | 1.00 | 38.53 | C |
| ATOM | 2866 | O | VAL | B | 69 | -11.512 | 15.836 | 52.272 | 1.00 | 39.44 | O |
| ATOM | 2867 | N | VAL | B | 70 | -11.741 | 14.350 | 50.606 | 1.00 | 37.68 | N |
| ATOM | 2868 | CA | VAL | B | 70 | -10.903 | 15.103 | 49.693 | 1.00 | 36.79 | C |
| ATOM | 2869 | CB | VAL | B | 70 | -9.593 | 14.366 | 49.372 | 1.00 | 34.88 | C |
| ATOM | 2870 | CG1 | VAL | B | 70 | -8.711 | 15.241 | 48.498 | 1.00 | 32.77 | C |
| ATOM | 2871 | CG2 | VAL | B | 70 | -8.870 | 14.010 | 50.658 | 1.00 | 33.95 | C |
| ATOM | 2872 | C | VAL | B | 70 | -11.694 | 15.307 | 48.410 | 1.00 | 38.20 | C |
| ATOM | 2873 | O | VAL | B | 70 | -12.169 | 14.353 | 47.800 | 1.00 | 38.29 | O |
| ATOM | 2874 | N | TYR | B | 71 | -11.847 | 16.564 | 48.018 | 1.00 | 38.75 | N |

| | | | | | | | | | | | |
|---|---|---|---|---|---|---|---|---|---|---|---|
| ATOM | 2875 | CA | TYR | B | 71 | -12.590 | 16.895 | 46.824 | 1.00 | 38.87 | C |
| ATOM | 2876 | CB | TYR | B | 71 | -13.589 | 18.018 | 47.111 | 1.00 | 38.36 | C |
| ATOM | 2877 | CG | TYR | B | 71 | -14.539 | 17.768 | 48.258 | 1.00 | 39.09 | C |
| ATOM | 2878 | CD1 | TYR | B | 71 | -14.139 | 17.939 | 49.583 | 1.00 | 38.97 | C |
| ATOM | 2879 | CE1 | TYR | B | 71 | -15.043 | 17.747 | 50.631 | 1.00 | 40.15 | C |
| ATOM | 2880 | CD2 | TYR | B | 71 | -15.859 | 17.389 | 48.013 | 1.00 | 39.02 | C |
| ATOM | 2881 | CE2 | TYR | B | 71 | -16.762 | 17.194 | 49.050 | 1.00 | 38.97 | C |
| ATOM | 2882 | CZ | TYR | B | 71 | -16.354 | 17.375 | 50.350 | 1.00 | 39.63 | C |
| ATOM | 2883 | OH | TYR | B | 71 | -17.268 | 17.198 | 51.361 | 1.00 | 41.24 | O |
| ATOM | 2884 | C | TYR | B | 71 | -11.670 | 17.374 | 45.732 | 1.00 | 39.88 | C |
| ATOM | 2885 | O | TYR | B | 71 | -10.519 | 17.750 | 45.979 | 1.00 | 41.11 | O |
| ATOM | 2886 | N | GLN | B | 72 | -12.184 | 17.349 | 44.508 | 1.00 | 40.35 | N |
| ATOM | 2887 | CA | GLN | B | 72 | -11.438 | 17.868 | 43.376 | 1.00 | 39.38 | C |
| ATOM | 2888 | CB | GLN | B | 72 | -11.380 | 16.888 | 42.220 | 1.00 | 39.76 | C |
| ATOM | 2889 | CG | GLN | B | 72 | -10.357 | 17.316 | 41.191 | 1.00 | 41.46 | C |
| ATOM | 2890 | CD | GLN | B | 72 | -10.578 | 16.677 | 39.844 | 1.00 | 42.85 | C |
| ATOM | 2891 | OE1 | GLN | B | 72 | -11.083 | 17.310 | 38.915 | 1.00 | 44.23 | O |
| ATOM | 2892 | NE2 | GLN | B | 72 | -10.216 | 15.412 | 39.732 | 1.00 | 44.09 | N |
| ATOM | 2893 | C | GLN | B | 72 | -12.299 | 19.042 | 42.981 | 1.00 | 39.45 | C |
| ATOM | 2894 | O | GLN | B | 72 | -13.493 | 19.042 | 43.243 | 1.00 | 39.27 | O |
| ATOM | 2895 | N | ALA | B | 73 | -11.705 | 20.050 | 42.368 | 1.00 | 39.98 | N |
| ATOM | 2896 | CA | ALA | B | 73 | -12.469 | 21.215 | 41.968 | 1.00 | 40.33 | C |
| ATOM | 2897 | CB | ALA | B | 73 | -12.958 | 21.960 | 43.195 | 1.00 | 40.29 | C |
| ATOM | 2898 | C | ALA | B | 73 | -11.541 | 22.083 | 41.167 | 1.00 | 41.43 | C |
| ATOM | 2899 | O | ALA | B | 73 | -10.331 | 21.876 | 41.187 | 1.00 | 40.43 | O |
| ATOM | 2900 | N | LYS | B | 74 | -12.105 | 23.029 | 40.430 | 1.00 | 44.34 | N |
| ATOM | 2901 | CA | LYS | B | 74 | -11.278 | 23.926 | 39.657 | 1.00 | 47.70 | C |
| ATOM | 2902 | CB | LYS | B | 74 | -11.421 | 23.678 | 38.153 | 1.00 | 49.63 | C |
| ATOM | 2903 | CG | LYS | B | 74 | -12.686 | 24.217 | 37.552 | 1.00 | 51.69 | C |
| ATOM | 2904 | CD | LYS | B | 74 | -12.606 | 24.248 | 36.038 | 1.00 | 53.82 | C |
| ATOM | 2905 | CE | LYS | B | 74 | -13.858 | 24.902 | 35.467 | 1.00 | 54.74 | C |
| ATOM | 2906 | NZ | LYS | B | 74 | -13.855 | 24.912 | 33.982 | 1.00 | 54.31 | N |
| ATOM | 2907 | C | LYS | B | 74 | -11.665 | 25.350 | 39.994 | 1.00 | 48.33 | C |
| ATOM | 2908 | O | LYS | B | 74 | -12.836 | 25.675 | 40.203 | 1.00 | 47.67 | O |
| ATOM | 2909 | N | LEU | B | 75 | -10.641 | 26.188 | 40.046 | 1.00 | 48.67 | N |
| ATOM | 2910 | CA | LEU | B | 75 | -10.778 | 27.591 | 40.369 | 1.00 | 48.54 | C |
| ATOM | 2911 | CB | LEU | B | 75 | -9.395 | 28.138 | 40.727 | 1.00 | 47.55 | C |
| ATOM | 2912 | CG | LEU | B | 75 | -8.770 | 27.296 | 41.847 | 1.00 | 44.76 | C |
| ATOM | 2913 | CD1 | LEU | B | 75 | -7.380 | 27.787 | 42.152 | 1.00 | 41.96 | C |
| ATOM | 2914 | CD2 | LEU | B | 75 | -9.657 | 27.354 | 43.092 | 1.00 | 43.26 | C |
| ATOM | 2915 | C | LEU | B | 75 | -11.402 | 28.360 | 39.211 | 1.00 | 48.85 | C |
| ATOM | 2916 | O | LEU | B | 75 | -10.970 | 28.242 | 38.062 | 1.00 | 49.76 | O |
| ATOM | 2917 | N | CYS | B | 76 | -12.427 | 29.144 | 39.532 | 1.00 | 50.86 | N |
| ATOM | 2918 | CA | CYS | B | 76 | -13.180 | 29.936 | 38.557 | 1.00 | 52.09 | C |
| ATOM | 2919 | CB | CYS | B | 76 | -14.298 | 30.694 | 39.259 | 1.00 | 50.15 | C |
| ATOM | 2920 | SG | CYS | B | 76 | -15.589 | 29.688 | 39.954 | 1.00 | 52.24 | S |
| ATOM | 2921 | C | CYS | B | 76 | -12.398 | 30.947 | 37.749 | 1.00 | 53.39 | C |
| ATOM | 2922 | O | CYS | B | 76 | -12.883 | 31.421 | 36.720 | 1.00 | 54.12 | O |
| ATOM | 2923 | N | ASP | B | 77 | -11.207 | 31.308 | 38.210 | 1.00 | 55.22 | N |
| ATOM | 2924 | CA | ASP | B | 77 | -10.426 | 32.316 | 37.499 | 1.00 | 57.62 | C |
| ATOM | 2925 | CB | ASP | B | 77 | -9.848 | 33.321 | 38.503 | 1.00 | 60.54 | C |
| ATOM | 2926 | CG | ASP | B | 77 | -10.831 | 34.435 | 38.845 | 1.00 | 63.71 | C |
| ATOM | 2927 | OD1 | ASP | B | 77 | -12.011 | 34.135 | 39.170 | 1.00 | 65.05 | O |
| ATOM | 2928 | OD2 | ASP | B | 77 | -10.420 | 35.615 | 38.789 | 1.00 | 65.20 | O |
| ATOM | 2929 | C | ASP | B | 77 | -9.315 | 31.793 | 36.610 | 1.00 | 57.30 | C |
| ATOM | 2930 | O | ASP | B | 77 | -8.874 | 32.491 | 35.700 | 1.00 | 57.68 | O |
| ATOM | 2931 | N | SER | B | 78 | -8.856 | 30.574 | 36.858 | 1.00 | 56.60 | N |
| ATOM | 2932 | CA | SER | B | 78 | -7.767 | 30.030 | 36.052 | 1.00 | 56.10 | C |
| ATOM | 2933 | CB | SER | B | 78 | -6.525 | 29.882 | 36.923 | 1.00 | 55.91 | C |
| ATOM | 2934 | OG | SER | B | 78 | -6.805 | 29.016 | 38.013 | 1.00 | 56.26 | O |
| ATOM | 2935 | C | SER | B | 78 | -8.104 | 28.687 | 35.428 | 1.00 | 55.86 | C |
| ATOM | 2936 | O | SER | B | 78 | -7.325 | 28.149 | 34.644 | 1.00 | 55.79 | O |
| ATOM | 2937 | N | GLY | B | 79 | -9.270 | 28.151 | 35.773 | 1.00 | 55.09 | N |
| ATOM | 2938 | CA | GLY | B | 79 | -9.639 | 26.859 | 35.247 | 1.00 | 53.76 | C |
| ATOM | 2939 | C | GLY | B | 79 | -8.816 | 25.799 | 35.958 | 1.00 | 53.47 | C |
| ATOM | 2940 | O | GLY | B | 79 | -9.251 | 24.655 | 36.052 | 1.00 | 53.94 | O |
| ATOM | 2941 | N | GLU | B | 80 | -7.638 | 26.180 | 36.469 | 1.00 | 51.97 | N |

| | | | | | | | | | | | |
|---|---|---|---|---|---|---|---|---|---|---|---|
| ATOM | 2942 | CA | GLU | B | 80 | -6.748 | 25.245 | 37.170 | 1.00 | 50.73 | C |
| ATOM | 2943 | CB | GLU | B | 80 | -5.699 | 25.985 | 38.000 | 1.00 | 52.58 | C |
| ATOM | 2944 | CG | GLU | B | 80 | -4.510 | 26.528 | 37.245 | 1.00 | 55.08 | C |
| ATOM | 2945 | CD | GLU | B | 80 | -3.476 | 27.128 | 38.194 | 1.00 | 56.59 | C |
| ATOM | 2946 | OE1 | GLU | B | 80 | -3.843 | 28.047 | 38.969 | 1.00 | 57.59 | O |
| ATOM | 2947 | OE2 | GLU | B | 80 | -2.305 | 26.681 | 38.173 | 1.00 | 57.10 | O |
| ATOM | 2948 | C | GLU | B | 80 | -7.491 | 24.306 | 38.096 | 1.00 | 48.79 | C |
| ATOM | 2949 | O | GLU | B | 80 | -8.460 | 24.694 | 38.752 | 1.00 | 48.42 | O |
| ATOM | 2950 | N | LEU | B | 81 | -7.015 | 23.070 | 38.161 | 1.00 | 46.99 | N |
| ATOM | 2951 | CA | LEU | B | 81 | -7.638 | 22.075 | 39.018 | 1.00 | 45.55 | C |
| ATOM | 2952 | CB | LEU | B | 81 | -7.570 | 20.689 | 38.381 | 1.00 | 45.62 | C |
| ATOM | 2953 | CG | LEU | B | 81 | -8.562 | 20.359 | 37.258 | 1.00 | 44.54 | C |
| ATOM | 2954 | CD1 | LEU | B | 81 | -8.432 | 18.884 | 36.903 | 1.00 | 43.77 | C |
| ATOM | 2955 | CD2 | LEU | B | 81 | -9.991 | 20.661 | 37.729 | 1.00 | 44.87 | C |
| ATOM | 2956 | C | LEU | B | 81 | -6.974 | 22.045 | 40.381 | 1.00 | 44.27 | C |
| ATOM | 2957 | O | LEU | B | 81 | -5.785 | 22.331 | 40.525 | 1.00 | 44.60 | O |
| ATOM | 2958 | N | VAL | B | 82 | -7.758 | 21.699 | 41.389 | 1.00 | 41.89 | N |
| ATOM | 2959 | CA | VAL | B | 82 | -7.251 | 21.654 | 42.743 | 1.00 | 38.96 | C |
| ATOM | 2960 | CB | VAL | B | 82 | -7.553 | 22.963 | 43.489 | 1.00 | 38.12 | C |
| ATOM | 2961 | CG1 | VAL | B | 82 | -6.736 | 24.092 | 42.896 | 1.00 | 38.49 | C |
| ATOM | 2962 | CG2 | VAL | B | 82 | -9.042 | 23.278 | 43.407 | 1.00 | 38.48 | C |
| ATOM | 2963 | C | VAL | B | 82 | -7.848 | 20.523 | 43.539 | 1.00 | 38.56 | C |
| ATOM | 2964 | O | VAL | B | 82 | -8.869 | 19.952 | 43.170 | 1.00 | 39.17 | O |
| ATOM | 2965 | N | ALA | B | 83 | -7.183 | 20.200 | 44.639 | 1.00 | 38.11 | N |
| ATOM | 2966 | CA | ALA | B | 83 | -7.646 | 19.170 | 45.545 | 1.00 | 37.12 | C |
| ATOM | 2967 | CB | ALA | B | 83 | -6.531 | 18.146 | 45.795 | 1.00 | 37.03 | C |
| ATOM | 2968 | C | ALA | B | 83 | -7.966 | 19.938 | 46.823 | 1.00 | 36.50 | C |
| ATOM | 2969 | O | ALA | B | 83 | -7.258 | 20.881 | 47.180 | 1.00 | 34.62 | O |
| ATOM | 2970 | N | ILE | B | 84 | -9.045 | 19.565 | 47.495 | 1.00 | 35.39 | N |
| ATOM | 2971 | CA | ILE | B | 84 | -9.396 | 20.233 | 48.737 | 1.00 | 34.75 | C |
| ATOM | 2972 | CB | ILE | B | 84 | -10.708 | 21.058 | 48.618 | 1.00 | 32.42 | C |
| ATOM | 2973 | CG2 | ILE | B | 84 | -11.076 | 21.648 | 49.969 | 1.00 | 29.80 | C |
| ATOM | 2974 | CG1 | ILE | B | 84 | -10.523 | 22.192 | 47.602 | 1.00 | 34.66 | C |
| ATOM | 2975 | CD1 | ILE | B | 84 | -11.794 | 22.988 | 47.298 | 1.00 | 31.49 | C |
| ATOM | 2976 | C | ILE | B | 84 | -9.547 | 19.205 | 49.846 | 1.00 | 36.02 | C |
| ATOM | 2977 | O | ILE | B | 84 | -10.464 | 18.380 | 49.835 | 1.00 | 37.54 | O |
| ATOM | 2978 | N | LYS | B | 85 | -8.637 | 19.255 | 50.806 | 1.00 | 35.23 | N |
| ATOM | 2979 | CA | LYS | B | 85 | -8.686 | 18.330 | 51.912 | 1.00 | 36.77 | C |
| ATOM | 2980 | CB | LYS | B | 85 | -7.262 | 17.968 | 52.345 | 1.00 | 35.14 | C |
| ATOM | 2981 | CG | LYS | B | 85 | -7.163 | 16.801 | 53.307 | 1.00 | 33.34 | C |
| ATOM | 2982 | CD | LYS | B | 85 | -5.704 | 16.486 | 53.593 | 1.00 | 33.75 | C |
| ATOM | 2983 | CE | LYS | B | 85 | -5.559 | 15.433 | 54.678 | 1.00 | 34.55 | C |
| ATOM | 2984 | NZ | LYS | B | 85 | -4.132 | 15.167 | 55.019 | 1.00 | 34.26 | N |
| ATOM | 2985 | C | LYS | B | 85 | -9.470 | 18.983 | 53.049 | 1.00 | 38.81 | C |
| ATOM | 2986 | O | LYS | B | 85 | -9.009 | 19.943 | 53.670 | 1.00 | 39.60 | O |
| ATOM | 2987 | N | LYS | B | 86 | -10.669 | 18.462 | 53.290 | 1.00 | 40.54 | N |
| ATOM | 2988 | CA | LYS | B | 86 | -11.564 | 18.962 | 54.332 | 1.00 | 42.43 | C |
| ATOM | 2989 | CB | LYS | B | 86 | -13.019 | 18.780 | 53.864 | 1.00 | 42.61 | C |
| ATOM | 2990 | CG | LYS | B | 86 | -14.120 | 19.377 | 54.732 | 1.00 | 42.34 | C |
| ATOM | 2991 | CD | LYS | B | 86 | -15.275 | 19.838 | 53.841 | 1.00 | 44.74 | C |
| ATOM | 2992 | CE | LYS | B | 86 | -16.602 | 19.997 | 54.589 | 1.00 | 46.03 | C |
| ATOM | 2993 | NZ | LYS | B | 86 | -17.223 | 18.679 | 54.965 | 1.00 | 45.79 | N |
| ATOM | 2994 | C | LYS | B | 86 | -11.286 | 18.175 | 55.611 | 1.00 | 43.43 | C |
| ATOM | 2995 | O | LYS | B | 86 | -11.407 | 16.950 | 55.630 | 1.00 | 43.99 | O |
| ATOM | 2996 | N | VAL | B | 87 | -10.899 | 18.882 | 56.669 | 1.00 | 44.25 | N |
| ATOM | 2997 | CA | VAL | B | 87 | -10.572 | 18.249 | 57.939 | 1.00 | 45.28 | C |
| ATOM | 2998 | CB | VAL | B | 87 | -9.066 | 18.392 | 58.260 | 1.00 | 43.79 | C |
| ATOM | 2999 | CG1 | VAL | B | 87 | -8.819 | 18.099 | 59.730 | 1.00 | 43.28 | C |
| ATOM | 3000 | CG2 | VAL | B | 87 | -8.247 | 17.445 | 57.395 | 1.00 | 41.59 | C |
| ATOM | 3001 | C | VAL | B | 87 | -11.329 | 18.816 | 59.121 | 1.00 | 47.69 | C |
| ATOM | 3002 | O | VAL | B | 87 | -11.551 | 20.026 | 59.205 | 1.00 | 48.30 | O |
| ATOM | 3003 | N | LEU | B | 88 | -11.720 | 17.943 | 60.045 | 1.00 | 50.77 | N |
| ATOM | 3004 | CA | LEU | B | 88 | -12.416 | 18.398 | 61.240 | 1.00 | 53.86 | C |
| ATOM | 3005 | CB | LEU | B | 88 | -13.175 | 17.246 | 61.904 | 1.00 | 53.84 | C |
| ATOM | 3006 | CG | LEU | B | 88 | -14.273 | 17.705 | 62.875 | 1.00 | 54.46 | C |
| ATOM | 3007 | CD1 | LEU | B | 88 | -15.442 | 18.249 | 62.056 | 1.00 | 53.72 | C |
| ATOM | 3008 | CD2 | LEU | B | 88 | -14.745 | 16.552 | 63.752 | 1.00 | 54.38 | C |

| | | | | | | | | | | | |
|---|---|---|---|---|---|---|---|---|---|---|---|
| ATOM | 3009 | C | LEU | B | 88 | -11.339 | 18.921 | 62.194 | 1.00 | 55.56 | C |
| ATOM | 3010 | O | LEU | B | 88 | -10.530 | 18.149 | 62.708 | 1.00 | 57.09 | O |
| ATOM | 3011 | N | GLN | B | 89 | -11.318 | 20.229 | 62.414 | 1.00 | 57.40 | N |
| ATOM | 3012 | CA | GLN | B | 89 | -10.331 | 20.826 | 63.301 | 1.00 | 59.19 | C |
| ATOM | 3013 | CB | GLN | B | 89 | -9.638 | 21.999 | 62.600 | 1.00 | 59.78 | C |
| ATOM | 3014 | CG | GLN | B | 89 | -8.637 | 22.763 | 63.459 | 1.00 | 61.00 | C |
| ATOM | 3015 | CD | GLN | B | 89 | -7.602 | 21.866 | 64.124 | 1.00 | 61.30 | C |
| ATOM | 3016 | OE1 | GLN | B | 89 | -7.633 | 20.638 | 63.973 | 1.00 | 61.24 | O |
| ATOM | 3017 | NE2 | GLN | B | 89 | -6.674 | 22.482 | 64.867 | 1.00 | 60.92 | N |
| ATOM | 3018 | C | GLN | B | 89 | -10.987 | 21.310 | 64.581 | 1.00 | 60.34 | C |
| ATOM | 3019 | O | GLN | B | 89 | -11.951 | 22.072 | 64.545 | 1.00 | 60.15 | O |
| ATOM | 3020 | N | ASP | B | 90 | -10.460 | 20.848 | 65.711 | 1.00 | 61.90 | N |
| ATOM | 3021 | CA | ASP | B | 90 | -10.961 | 21.244 | 67.022 | 1.00 | 63.13 | C |
| ATOM | 3022 | CB | ASP | B | 90 | -10.554 | 20.201 | 68.065 | 1.00 | 64.02 | C |
| ATOM | 3023 | CG | ASP | B | 90 | -11.003 | 20.567 | 69.462 | 1.00 | 65.37 | C |
| ATOM | 3024 | OD1 | ASP | B | 90 | -10.951 | 19.677 | 70.346 | 1.00 | 66.22 | O |
| ATOM | 3025 | OD2 | ASP | B | 90 | -11.392 | 21.744 | 69.674 | 1.00 | 65.16 | O |
| ATOM | 3026 | C | ASP | B | 90 | -10.350 | 22.602 | 67.354 | 1.00 | 63.40 | C |
| ATOM | 3027 | O | ASP | B | 90 | -9.138 | 22.707 | 67.553 | 1.00 | 63.63 | O |
| ATOM | 3028 | N | LYS | B | 91 | -11.178 | 23.640 | 67.444 | 1.00 | 63.19 | N |
| ATOM | 3029 | CA | LYS | B | 91 | -10.642 | 24.979 | 67.706 | 1.00 | 63.49 | C |
| ATOM | 3030 | CB | LYS | B | 91 | -11.776 | 26.001 | 67.839 | 1.00 | 63.39 | C |
| ATOM | 3031 | CG | LYS | B | 91 | -11.828 | 26.988 | 66.674 | 1.00 | 64.27 | C |
| ATOM | 3032 | CD | LYS | B | 91 | -10.503 | 27.736 | 66.497 | 1.00 | 64.97 | C |
| ATOM | 3033 | CE | LYS | B | 91 | -10.062 | 27.804 | 65.022 | 1.00 | 65.05 | C |
| ATOM | 3034 | NZ | LYS | B | 91 | -8.705 | 28.444 | 64.859 | 1.00 | 64.20 | N |
| ATOM | 3035 | C | LYS | B | 91 | -9.665 | 25.140 | 68.875 | 1.00 | 63.12 | C |
| ATOM | 3036 | O | LYS | B | 91 | -8.912 | 26.119 | 68.918 | 1.00 | 62.78 | O |
| ATOM | 3037 | N | ALA | B | 92 | -9.667 | 24.188 | 69.804 | 1.00 | 63.18 | N |
| ATOM | 3038 | CA | ALA | B | 92 | -8.762 | 24.234 | 70.958 | 1.00 | 63.35 | C |
| ATOM | 3039 | CB | ALA | B | 92 | -8.968 | 22.994 | 71.839 | 1.00 | 63.66 | C |
| ATOM | 3040 | C | ALA | B | 92 | -7.307 | 24.300 | 70.491 | 1.00 | 62.78 | C |
| ATOM | 3041 | O | ALA | B | 92 | -6.646 | 25.339 | 70.614 | 1.00 | 62.35 | O |
| ATOM | 3042 | N | ALA | B | 93 | -6.828 | 23.183 | 69.947 | 1.00 | 61.67 | N |
| ATOM | 3043 | CA | ALA | B | 93 | -5.461 | 23.083 | 69.451 | 1.00 | 59.93 | C |
| ATOM | 3044 | CB | ALA | B | 93 | -4.954 | 21.658 | 69.638 | 1.00 | 60.59 | C |
| ATOM | 3045 | C | ALA | B | 93 | -5.333 | 23.491 | 67.983 | 1.00 | 58.12 | C |
| ATOM | 3046 | O | ALA | B | 93 | -6.258 | 23.318 | 67.194 | 1.00 | 57.53 | O |
| ATOM | 3047 | N | ALA | B | 94 | -4.179 | 24.048 | 67.630 | 1.00 | 55.49 | N |
| ATOM | 3048 | CA | ALA | B | 94 | -3.911 | 24.453 | 66.255 | 1.00 | 52.93 | C |
| ATOM | 3049 | CB | ALA | B | 94 | -2.625 | 25.287 | 66.192 | 1.00 | 53.29 | C |
| ATOM | 3050 | C | ALA | B | 94 | -3.744 | 23.160 | 65.458 | 1.00 | 50.81 | C |
| ATOM | 3051 | O | ALA | B | 94 | -3.658 | 22.085 | 66.047 | 1.00 | 50.27 | O |
| ATOM | 3052 | N | ASN | B | 95 | -3.713 | 23.259 | 64.130 | 1.00 | 47.18 | N |
| ATOM | 3053 | CA | ASN | B | 95 | -3.579 | 22.080 | 63.258 | 1.00 | 42.60 | C |
| ATOM | 3054 | CB | ASN | B | 95 | -4.493 | 22.230 | 62.027 | 1.00 | 43.87 | C |
| ATOM | 3055 | CG | ASN | B | 95 | -4.434 | 21.025 | 61.084 | 1.00 | 44.45 | C |
| ATOM | 3056 | OD1 | ASN | B | 95 | -3.455 | 20.831 | 60.348 | 1.00 | 44.76 | O |
| ATOM | 3057 | ND2 | ASN | B | 95 | -5.489 | 20.211 | 61.102 | 1.00 | 42.43 | N |
| ATOM | 3058 | C | ASN | B | 95 | -2.136 | 21.880 | 62.815 | 1.00 | 40.16 | C |
| ATOM | 3059 | O | ASN | B | 95 | -1.516 | 22.772 | 62.233 | 1.00 | 39.22 | O |
| ATOM | 3060 | N | ARG | B | 96 | -1.602 | 20.700 | 63.097 | 1.00 | 38.07 | N |
| ATOM | 3061 | CA | ARG | B | 96 | -0.229 | 20.397 | 62.745 | 1.00 | 36.92 | C |
| ATOM | 3062 | CB | ARG | B | 96 | 0.192 | 19.058 | 63.362 | 1.00 | 35.33 | C |
| ATOM | 3063 | CG | ARG | B | 96 | 1.690 | 18.803 | 63.328 | 1.00 | 35.91 | C |
| ATOM | 3064 | CD | ARG | B | 96 | 2.067 | 17.485 | 63.998 | 1.00 | 38.77 | C |
| ATOM | 3065 | NE | ARG | B | 96 | 1.967 | 17.520 | 65.461 | 1.00 | 42.95 | N |
| ATOM | 3066 | CZ | ARG | B | 96 | 2.095 | 16.449 | 66.243 | 1.00 | 43.09 | C |
| ATOM | 3067 | NH1 | ARG | B | 96 | 2.325 | 15.254 | 65.708 | 1.00 | 44.67 | N |
| ATOM | 3068 | NH2 | ARG | B | 96 | 1.994 | 16.566 | 67.556 | 1.00 | 43.40 | N |
| ATOM | 3069 | C | ARG | B | 96 | -0.010 | 20.375 | 61.235 | 1.00 | 37.33 | C |
| ATOM | 3070 | O | ARG | B | 96 | 0.939 | 20.980 | 60.734 | 1.00 | 37.27 | O |
| ATOM | 3071 | N | GLU | B | 97 | -0.891 | 19.700 | 60.501 | 1.00 | 37.25 | N |
| ATOM | 3072 | CA | GLU | B | 97 | -0.725 | 19.614 | 59.051 | 1.00 | 36.97 | C |
| ATOM | 3073 | CB | GLU | B | 97 | -1.871 | 18.820 | 58.410 | 1.00 | 38.32 | C |
| ATOM | 3074 | CG | GLU | B | 97 | -1.472 | 18.164 | 57.095 | 1.00 | 37.20 | C |
| ATOM | 3075 | CD | GLU | B | 97 | -2.607 | 17.405 | 56.432 | 1.00 | 36.58 | C |

| | | | | | | | | | | | |
|---|---|---|---|---|---|---|---|---|---|---|---|
| ATOM | 3076 | OE1 | GLU | B | 97 | -3.401 | 16.757 | 57.143 | 1.00 | 36.86 | O |
| ATOM | 3077 | OE2 | GLU | B | 97 | -2.694 | 17.440 | 55.196 | 1.00 | 35.29 | O |
| ATOM | 3078 | C | GLU | B | 97 | -0.631 | 20.996 | 58.410 | 1.00 | 37.12 | C |
| ATOM | 3079 | O | GLU | B | 97 | 0.221 | 21.239 | 57.551 | 1.00 | 36.62 | O |
| ATOM | 3080 | N | LEU | B | 98 | -1.496 | 21.912 | 58.829 | 1.00 | 36.34 | N |
| ATOM | 3081 | CA | LEU | B | 98 | -1.458 | 23.254 | 58.264 | 1.00 | 35.61 | C |
| ATOM | 3082 | CB | LEU | B | 98 | -2.681 | 24.059 | 58.715 | 1.00 | 34.28 | C |
| ATOM | 3083 | CG | LEU | B | 98 | -2.704 | 25.547 | 58.366 | 1.00 | 31.69 | C |
| ATOM | 3084 | CD1 | LEU | B | 98 | -2.394 | 25.759 | 56.898 | 1.00 | 32.64 | C |
| ATOM | 3085 | CD2 | LEU | B | 98 | -4.055 | 26.109 | 58.732 | 1.00 | 31.27 | C |
| ATOM | 3086 | C | LEU | B | 98 | -0.164 | 24.003 | 58.618 | 1.00 | 35.99 | C |
| ATOM | 3087 | O | LEU | B | 98 | 0.460 | 24.614 | 57.748 | 1.00 | 33.10 | O |
| ATOM | 3088 | N | GLN | B | 99 | 0.244 | 23.946 | 59.885 | 1.00 | 38.08 | N |
| ATOM | 3089 | CA | GLN | B | 99 | 1.462 | 24.633 | 60.319 | 1.00 | 39.87 | C |
| ATOM | 3090 | CB | GLN | B | 99 | 1.751 | 24.335 | 61.796 | 1.00 | 43.70 | C |
| ATOM | 3091 | CG | GLN | B | 99 | 0.748 | 24.918 | 62.769 | 1.00 | 48.80 | C |
| ATOM | 3092 | CD | GLN | B | 99 | 1.080 | 24.573 | 64.218 | 1.00 | 53.01 | C |
| ATOM | 3093 | OE1 | GLN | B | 99 | 0.287 | 24.849 | 65.130 | 1.00 | 57.14 | O |
| ATOM | 3094 | NE2 | GLN | B | 99 | 2.255 | 23.965 | 64.438 | 1.00 | 53.18 | N |
| ATOM | 3095 | C | GLN | B | 99 | 2.681 | 24.252 | 59.485 | 1.00 | 37.91 | C |
| ATOM | 3096 | O | GLN | B | 99 | 3.404 | 25.121 | 59.006 | 1.00 | 36.20 | O |
| ATOM | 3097 | N | ILE | B | 100 | 2.897 | 22.947 | 59.323 | 1.00 | 37.69 | N |
| ATOM | 3098 | CA | ILE | B | 100 | 4.028 | 22.422 | 58.546 | 1.00 | 37.00 | C |
| ATOM | 3099 | CB | ILE | B | 100 | 4.103 | 20.884 | 58.650 | 1.00 | 35.39 | C |
| ATOM | 3100 | CG2 | ILE | B | 100 | 5.003 | 20.330 | 57.548 | 1.00 | 34.95 | C |
| ATOM | 3101 | CG1 | ILE | B | 100 | 4.588 | 20.483 | 60.039 | 1.00 | 33.32 | C |
| ATOM | 3102 | CD1 | ILE | B | 100 | 4.389 | 19.023 | 60.353 | 1.00 | 33.04 | C |
| ATOM | 3103 | C | ILE | B | 100 | 3.918 | 22.793 | 57.072 | 1.00 | 38.03 | C |
| ATOM | 3104 | O | ILE | B | 100 | 4.892 | 23.193 | 56.436 | 1.00 | 38.57 | O |
| ATOM | 3105 | N | MET | B | 101 | 2.711 | 22.643 | 56.548 | 1.00 | 39.15 | N |
| ATOM | 3106 | CA | MET | B | 101 | 2.420 | 22.939 | 55.172 | 1.00 | 40.12 | C |
| ATOM | 3107 | CB | MET | B | 101 | 0.972 | 22.600 | 54.880 | 1.00 | 39.96 | C |
| ATOM | 3108 | CG | MET | B | 101 | 0.779 | 22.268 | 53.415 | 1.00 | 42.10 | C |
| ATOM | 3109 | SD | MET | B | 101 | 0.827 | 20.510 | 53.065 | 1.00 | 41.87 | S |
| ATOM | 3110 | CE | MET | B | 101 | 1.333 | 20.571 | 51.343 | 1.00 | 38.27 | C |
| ATOM | 3111 | C | MET | B | 101 | 2.663 | 24.410 | 54.860 | 1.00 | 41.37 | C |
| ATOM | 3112 | O | MET | B | 101 | 3.301 | 24.767 | 53.864 | 1.00 | 41.19 | O |
| ATOM | 3113 | N | ARG | B | 102 | 2.147 | 25.262 | 55.734 | 1.00 | 43.11 | N |
| ATOM | 3114 | CA | ARG | B | 102 | 2.247 | 26.710 | 55.588 | 1.00 | 44.59 | C |
| ATOM | 3115 | CB | ARG | B | 102 | 1.541 | 27.381 | 56.771 | 1.00 | 45.19 | C |
| ATOM | 3116 | CG | ARG | B | 102 | 1.205 | 28.841 | 56.569 | 1.00 | 45.92 | C |
| ATOM | 3117 | CD | ARG | B | 102 | -0.220 | 29.047 | 56.070 | 1.00 | 45.29 | C |
| ATOM | 3118 | NE | ARG | B | 102 | -0.529 | 30.477 | 55.983 | 1.00 | 45.40 | N |
| ATOM | 3119 | CZ | ARG | B | 102 | -0.823 | 31.248 | 57.029 | 1.00 | 45.04 | C |
| ATOM | 3120 | NH1 | ARG | B | 102 | -0.863 | 30.728 | 58.248 | 1.00 | 44.39 | N |
| ATOM | 3121 | NH2 | ARG | B | 102 | -1.047 | 32.546 | 56.858 | 1.00 | 46.00 | N |
| ATOM | 3122 | C | ARG | B | 102 | 3.665 | 27.266 | 55.455 | 1.00 | 45.16 | C |
| ATOM | 3123 | O | ARG | B | 102 | 3.832 | 28.418 | 55.062 | 1.00 | 46.97 | O |
| ATOM | 3124 | N | LYS | B | 103 | 4.683 | 26.464 | 55.762 | 1.00 | 45.64 | N |
| ATOM | 3125 | CA | LYS | B | 103 | 6.061 | 26.941 | 55.668 | 1.00 | 45.33 | C |
| ATOM | 3126 | CB | LYS | B | 103 | 6.742 | 26.830 | 57.038 | 1.00 | 46.10 | C |
| ATOM | 3127 | CG | LYS | B | 103 | 7.629 | 25.601 | 57.206 | 1.00 | 48.60 | C |
| ATOM | 3128 | CD | LYS | B | 103 | 8.363 | 25.599 | 58.544 | 1.00 | 52.23 | C |
| ATOM | 3129 | CE | LYS | B | 103 | 7.405 | 25.307 | 59.697 | 1.00 | 53.96 | C |
| ATOM | 3130 | NZ | LYS | B | 103 | 8.121 | 25.223 | 61.011 | 1.00 | 56.75 | N |
| ATOM | 3131 | C | LYS | B | 103 | 6.913 | 26.215 | 54.626 | 1.00 | 46.15 | C |
| ATOM | 3132 | O | LYS | B | 103 | 8.134 | 26.395 | 54.586 | 1.00 | 46.19 | O |
| ATOM | 3133 | N | LEU | B | 104 | 6.290 | 25.403 | 53.776 | 1.00 | 45.89 | N |
| ATOM | 3134 | CA | LEU | B | 104 | 7.053 | 24.662 | 52.771 | 1.00 | 44.29 | C |
| ATOM | 3135 | CB | LEU | B | 104 | 6.618 | 23.189 | 52.795 | 1.00 | 41.94 | C |
| ATOM | 3136 | CG | LEU | B | 104 | 7.563 | 22.193 | 53.502 | 1.00 | 41.58 | C |
| ATOM | 3137 | CD1 | LEU | B | 104 | 8.219 | 22.818 | 54.710 | 1.00 | 38.31 | C |
| ATOM | 3138 | CD2 | LEU | B | 104 | 6.786 | 20.941 | 53.893 | 1.00 | 40.75 | C |
| ATOM | 3139 | C | LEU | B | 104 | 6.963 | 25.235 | 51.356 | 1.00 | 43.39 | C |
| ATOM | 3140 | O | LEU | B | 104 | 5.934 | 25.782 | 50.962 | 1.00 | 44.25 | O |
| ATOM | 3141 | N | ASP | B | 105 | 8.054 | 25.144 | 50.604 | 1.00 | 43.33 | N |
| ATOM | 3142 | CA | ASP | B | 105 | 8.063 | 25.642 | 49.229 | 1.00 | 44.67 | C |

| | | | | | | | | | | | |
|---|---|---|---|---|---|---|---|---|---|---|---|
| ATOM | 3143 | CB | ASP | B | 105 | 8.279 | 27.150 | 49.174 | 1.00 | 47.13 | C |
| ATOM | 3144 | CG | ASP | B | 105 | 7.988 | 27.727 | 47.793 | 1.00 | 50.33 | C |
| ATOM | 3145 | OD1 | ASP | B | 105 | 8.518 | 27.206 | 46.788 | 1.00 | 53.15 | O |
| ATOM | 3146 | OD2 | ASP | B | 105 | 7.223 | 28.705 | 47.705 | 1.00 | 52.00 | O |
| ATOM | 3147 | C | ASP | B | 105 | 9.164 | 24.969 | 48.434 | 1.00 | 44.17 | C |
| ATOM | 3148 | O | ASP | B | 105 | 10.320 | 25.407 | 48.453 | 1.00 | 44.09 | O |
| ATOM | 3149 | N | HIS | B | 106 | 8.791 | 23.906 | 47.728 | 1.00 | 44.61 | N |
| ATOM | 3150 | CA | HIS | B | 106 | 9.737 | 23.136 | 46.936 | 1.00 | 42.90 | C |
| ATOM | 3151 | CB | HIS | B | 106 | 10.294 | 21.978 | 47.775 | 1.00 | 41.54 | C |
| ATOM | 3152 | CG | HIS | B | 106 | 11.618 | 21.478 | 47.302 | 1.00 | 40.94 | C |
| ATOM | 3153 | CD2 | HIS | B | 106 | 12.881 | 21.766 | 47.716 | 1.00 | 41.41 | C |
| ATOM | 3154 | ND1 | HIS | B | 106 | 11.762 | 20.612 | 46.241 | 1.00 | 39.52 | N |
| ATOM | 3155 | CE1 | HIS | B | 106 | 13.042 | 20.388 | 46.018 | 1.00 | 40.71 | C |
| ATOM | 3156 | NE2 | HIS | B | 106 | 13.743 | 21.082 | 46.905 | 1.00 | 41.38 | N |
| ATOM | 3157 | C | HIS | B | 106 | 9.064 | 22.602 | 45.675 | 1.00 | 41.59 | C |
| ATOM | 3158 | O | HIS | B | 106 | 7.875 | 22.266 | 45.679 | 1.00 | 40.46 | O |
| ATOM | 3159 | N | CYS | B | 107 | 9.840 | 22.549 | 44.599 | 1.00 | 40.97 | N |
| ATOM | 3160 | CA | CYS | B | 107 | 9.375 | 22.066 | 43.306 | 1.00 | 40.66 | C |
| ATOM | 3161 | CB | CYS | B | 107 | 10.493 | 22.240 | 42.270 | 1.00 | 43.15 | C |
| ATOM | 3162 | SG | CYS | B | 107 | 12.146 | 21.626 | 42.842 | 1.00 | 48.31 | S |
| ATOM | 3163 | C | CYS | B | 107 | 8.956 | 20.596 | 43.385 | 1.00 | 39.11 | C |
| ATOM | 3164 | O | CYS | B | 107 | 8.330 | 20.075 | 42.470 | 1.00 | 39.55 | O |
| ATOM | 3165 | N | ASN | B | 108 | 9.294 | 19.934 | 44.486 | 1.00 | 37.88 | N |
| ATOM | 3166 | CA | ASN | B | 108 | 8.956 | 18.536 | 44.647 | 1.00 | 35.58 | C |
| ATOM | 3167 | CB | ASN | B | 108 | 10.231 | 17.735 | 44.855 | 1.00 | 34.17 | C |
| ATOM | 3168 | CG | ASN | B | 108 | 11.156 | 17.801 | 43.655 | 1.00 | 34.54 | C |
| ATOM | 3169 | OD1 | ASN | B | 108 | 12.215 | 18.425 | 43.703 | 1.00 | 34.20 | O |
| ATOM | 3170 | ND2 | ASN | B | 108 | 10.752 | 17.159 | 42.564 | 1.00 | 34.72 | N |
| ATOM | 3171 | C | ASN | B | 108 | 7.971 | 18.268 | 45.776 | 1.00 | 35.13 | C |
| ATOM | 3172 | O | ASN | B | 108 | 7.986 | 17.199 | 46.390 | 1.00 | 34.61 | O |
| ATOM | 3173 | N | ILE | B | 109 | 7.110 | 19.243 | 46.043 | 1.00 | 35.13 | N |
| ATOM | 3174 | CA | ILE | B | 109 | 6.105 | 19.109 | 47.086 | 1.00 | 35.34 | C |
| ATOM | 3175 | CB | ILE | B | 109 | 6.591 | 19.723 | 48.415 | 1.00 | 35.54 | C |
| ATOM | 3176 | CG2 | ILE | B | 109 | 5.410 | 20.093 | 49.296 | 1.00 | 33.08 | C |
| ATOM | 3177 | CG1 | ILE | B | 109 | 7.524 | 18.739 | 49.113 | 1.00 | 35.14 | C |
| ATOM | 3178 | CD1 | ILE | B | 109 | 8.462 | 19.392 | 50.086 | 1.00 | 36.44 | C |
| ATOM | 3179 | C | ILE | B | 109 | 4.831 | 19.801 | 46.658 | 1.00 | 35.43 | C |
| ATOM | 3180 | O | ILE | B | 109 | 4.870 | 20.913 | 46.135 | 1.00 | 35.50 | O |
| ATOM | 3181 | N | VAL | B | 110 | 3.708 | 19.126 | 46.876 | 1.00 | 37.08 | N |
| ATOM | 3182 | CA | VAL | B | 110 | 2.417 | 19.684 | 46.521 | 1.00 | 38.98 | C |
| ATOM | 3183 | CB | VAL | B | 110 | 1.247 | 18.798 | 46.993 | 1.00 | 38.29 | C |
| ATOM | 3184 | CG1 | VAL | B | 110 | 1.420 | 17.404 | 46.460 | 1.00 | 40.91 | C |
| ATOM | 3185 | CG2 | VAL | B | 110 | 1.159 | 18.786 | 48.509 | 1.00 | 38.21 | C |
| ATOM | 3186 | C | VAL | B | 110 | 2.322 | 21.020 | 47.221 | 1.00 | 40.05 | C |
| ATOM | 3187 | O | VAL | B | 110 | 2.592 | 21.113 | 48.408 | 1.00 | 39.51 | O |
| ATOM | 3188 | N | ARG | B | 111 | 1.945 | 22.058 | 46.488 | 1.00 | 42.12 | N |
| ATOM | 3189 | CA | ARG | B | 111 | 1.837 | 23.371 | 47.085 | 1.00 | 43.47 | C |
| ATOM | 3190 | CB | ARG | B | 111 | 2.069 | 24.457 | 46.033 | 1.00 | 46.54 | C |
| ATOM | 3191 | CG | ARG | B | 111 | 1.674 | 25.878 | 46.487 | 1.00 | 51.35 | C |
| ATOM | 3192 | CD | ARG | B | 111 | 2.168 | 26.971 | 45.527 | 1.00 | 53.80 | C |
| ATOM | 3193 | NE | ARG | B | 111 | 1.390 | 27.111 | 44.291 | 1.00 | 57.13 | N |
| ATOM | 3194 | CZ | ARG | B | 111 | 1.316 | 26.199 | 43.319 | 1.00 | 58.85 | C |
| ATOM | 3195 | NH1 | ARG | B | 111 | 1.966 | 25.049 | 43.426 | 1.00 | 59.57 | N |
| ATOM | 3196 | NH2 | ARG | B | 111 | 0.629 | 26.456 | 42.209 | 1.00 | 58.41 | N |
| ATOM | 3197 | C | ARG | B | 111 | 0.489 | 23.592 | 47.748 | 1.00 | 43.87 | C |
| ATOM | 3198 | O | ARG | B | 111 | -0.541 | 23.091 | 47.285 | 1.00 | 44.39 | O |
| ATOM | 3199 | N | LEU | B | 112 | 0.517 | 24.347 | 48.843 | 1.00 | 43.67 | N |
| ATOM | 3200 | CA | LEU | B | 112 | -0.682 | 24.710 | 49.585 | 1.00 | 42.89 | C |
| ATOM | 3201 | CB | LEU | B | 112 | -0.390 | 24.774 | 51.082 | 1.00 | 41.71 | C |
| ATOM | 3202 | CG | LEU | B | 112 | -1.539 | 25.262 | 51.965 | 1.00 | 41.98 | C |
| ATOM | 3203 | CD1 | LEU | B | 112 | -2.812 | 24.491 | 51.637 | 1.00 | 43.04 | C |
| ATOM | 3204 | CD2 | LEU | B | 112 | -1.173 | 25.074 | 53.431 | 1.00 | 42.15 | C |
| ATOM | 3205 | C | LEU | B | 112 | -1.101 | 26.087 | 49.073 | 1.00 | 42.88 | C |
| ATOM | 3206 | O | LEU | B | 112 | -0.584 | 27.111 | 49.513 | 1.00 | 41.81 | O |
| ATOM | 3207 | N | ARG | B | 113 | -2.035 | 26.086 | 48.125 | 1.00 | 43.18 | N |
| ATOM | 3208 | CA | ARG | B | 113 | -2.549 | 27.303 | 47.502 | 1.00 | 42.90 | C |
| ATOM | 3209 | CB | ARG | B | 113 | -3.532 | 26.935 | 46.385 | 1.00 | 44.79 | C |

| | | | | | | | | | | | |
|---|---|---|---|---|---|---|---|---|---|---|---|
| ATOM | 3210 | CG | ARG | B | 113 | -3.059 | 25.793 | 45.490 | 1.00 | 47.54 | C |
| ATOM | 3211 | CD | ARG | B | 113 | -1.942 | 26.228 | 44.553 | 1.00 | 52.27 | C |
| ATOM | 3212 | NE | ARG | B | 113 | -2.482 | 26.771 | 43.309 | 1.00 | 56.65 | N |
| ATOM | 3213 | CZ | ARG | B | 113 | -2.976 | 26.024 | 42.322 | 1.00 | 57.78 | C |
| ATOM | 3214 | NH1 | ARG | B | 113 | -2.982 | 24.699 | 42.434 | 1.00 | 58.02 | N |
| ATOM | 3215 | NH2 | ARG | B | 113 | -3.494 | 26.600 | 41.238 | 1.00 | 57.87 | N |
| ATOM | 3216 | C | ARG | B | 113 | -3.250 | 28.210 | 48.505 | 1.00 | 42.32 | C |
| ATOM | 3217 | O | ARG | B | 113 | -2.973 | 29.407 | 48.577 | 1.00 | 43.39 | O |
| ATOM | 3218 | N | TYR | B | 114 | -4.172 | 27.631 | 49.266 | 1.00 | 40.81 | N |
| ATOM | 3219 | CA | TYR | B | 114 | -4.935 | 28.375 | 50.260 | 1.00 | 39.74 | C |
| ATOM | 3220 | CB | TYR | B | 114 | -6.197 | 28.974 | 49.645 | 1.00 | 40.42 | C |
| ATOM | 3221 | CG | TYR | B | 114 | -5.995 | 29.775 | 48.388 | 1.00 | 42.55 | C |
| ATOM | 3222 | CD1 | TYR | B | 114 | -5.669 | 31.127 | 48.446 | 1.00 | 43.72 | C |
| ATOM | 3223 | CE1 | TYR | B | 114 | -5.504 | 31.878 | 47.292 | 1.00 | 44.87 | C |
| ATOM | 3224 | CD2 | TYR | B | 114 | -6.148 | 29.187 | 47.136 | 1.00 | 43.40 | C |
| ATOM | 3225 | CE2 | TYR | B | 114 | -5.983 | 29.926 | 45.972 | 1.00 | 45.29 | C |
| ATOM | 3226 | CZ | TYR | B | 114 | -5.661 | 31.272 | 46.060 | 1.00 | 46.39 | C |
| ATOM | 3227 | OH | TYR | B | 114 | -5.496 | 32.015 | 44.913 | 1.00 | 48.58 | O |
| ATOM | 3228 | C | TYR | B | 114 | -5.390 | 27.420 | 51.339 | 1.00 | 39.52 | C |
| ATOM | 3229 | O | TYR | B | 114 | -5.044 | 26.242 | 51.335 | 1.00 | 40.51 | O |
| ATOM | 3230 | N | PHE | B | 115 | -6.181 | 27.950 | 52.260 | 1.00 | 39.30 | N |
| ATOM | 3231 | CA | PHE | B | 115 | -6.771 | 27.167 | 53.331 | 1.00 | 38.26 | C |
| ATOM | 3232 | CB | PHE | B | 115 | -5.730 | 26.801 | 54.406 | 1.00 | 40.05 | C |
| ATOM | 3233 | CG | PHE | B | 115 | -5.209 | 27.967 | 55.198 | 1.00 | 39.50 | C |
| ATOM | 3234 | CD1 | PHE | B | 115 | -5.888 | 28.424 | 56.323 | 1.00 | 39.70 | C |
| ATOM | 3235 | CD2 | PHE | B | 115 | -4.019 | 28.584 | 54.838 | 1.00 | 39.08 | C |
| ATOM | 3236 | CE1 | PHE | B | 115 | -5.384 | 29.481 | 57.081 | 1.00 | 39.39 | C |
| ATOM | 3237 | CE2 | PHE | B | 115 | -3.509 | 29.641 | 55.590 | 1.00 | 39.14 | C |
| ATOM | 3238 | CZ | PHE | B | 115 | -4.193 | 30.088 | 56.713 | 1.00 | 38.04 | C |
| ATOM | 3239 | C | PHE | B | 115 | -7.879 | 28.052 | 53.866 | 1.00 | 37.46 | C |
| ATOM | 3240 | O | PHE | B | 115 | -7.723 | 29.271 | 53.931 | 1.00 | 37.40 | O |
| ATOM | 3241 | N | PHE | B | 116 | -9.014 | 27.453 | 54.200 | 1.00 | 36.15 | N |
| ATOM | 3242 | CA | PHE | B | 116 | -10.135 | 28.232 | 54.692 | 1.00 | 37.19 | C |
| ATOM | 3243 | CB | PHE | B | 116 | -10.958 | 28.753 | 53.510 | 1.00 | 38.42 | C |
| ATOM | 3244 | CG | PHE | B | 116 | -11.681 | 27.675 | 52.746 | 1.00 | 39.24 | C |
| ATOM | 3245 | CD1 | PHE | B | 116 | -12.910 | 27.197 | 53.185 | 1.00 | 39.04 | C |
| ATOM | 3246 | CD2 | PHE | B | 116 | -11.140 | 27.143 | 51.584 | 1.00 | 38.65 | C |
| ATOM | 3247 | CE1 | PHE | B | 116 | -13.593 | 26.208 | 52.477 | 1.00 | 37.60 | C |
| ATOM | 3248 | CE2 | PHE | B | 116 | -11.817 | 26.152 | 50.871 | 1.00 | 38.25 | C |
| ATOM | 3249 | CZ | PHE | B | 116 | -13.047 | 25.687 | 51.321 | 1.00 | 36.97 | C |
| ATOM | 3250 | C | PHE | B | 116 | -11.005 | 27.398 | 55.612 | 1.00 | 37.28 | C |
| ATOM | 3251 | O | PHE | B | 116 | -10.945 | 26.180 | 55.571 | 1.00 | 38.10 | O |
| ATOM | 3252 | N | TYR | B | 117 | -11.810 | 28.053 | 56.441 | 1.00 | 37.38 | N |
| ATOM | 3253 | CA | TYR | B | 117 | -12.678 | 27.343 | 57.364 | 1.00 | 37.66 | C |
| ATOM | 3254 | CB | TYR | B | 117 | -12.628 | 28.027 | 58.736 | 1.00 | 38.14 | C |
| ATOM | 3255 | CG | TYR | B | 117 | -11.230 | 28.000 | 59.325 | 1.00 | 39.17 | C |
| ATOM | 3256 | CD1 | TYR | B | 117 | -10.779 | 26.909 | 60.067 | 1.00 | 39.03 | C |
| ATOM | 3257 | CE1 | TYR | B | 117 | -9.444 | 26.817 | 60.475 | 1.00 | 39.31 | C |
| ATOM | 3258 | CD2 | TYR | B | 117 | -10.312 | 29.000 | 59.021 | 1.00 | 38.92 | C |
| ATOM | 3259 | CE2 | TYR | B | 117 | -8.981 | 28.912 | 59.421 | 1.00 | 38.56 | C |
| ATOM | 3260 | CZ | TYR | B | 117 | -8.554 | 27.821 | 60.139 | 1.00 | 39.71 | C |
| ATOM | 3261 | OH | TYR | B | 117 | -7.224 | 27.708 | 60.475 | 1.00 | 40.62 | O |
| ATOM | 3262 | C | TYR | B | 117 | -14.104 | 27.274 | 56.824 | 1.00 | 38.78 | C |
| ATOM | 3263 | O | TYR | B | 117 | -14.526 | 28.116 | 56.024 | 1.00 | 38.40 | O |
| ATOM | 3264 | N | SER | B | 118 | -14.839 | 26.254 | 57.251 | 1.00 | 39.65 | N |
| ATOM | 3265 | CA | SER | B | 118 | -16.205 | 26.069 | 56.802 | 1.00 | 41.25 | C |
| ATOM | 3266 | CB | SER | B | 118 | -16.223 | 25.273 | 55.499 | 1.00 | 41.61 | C |
| ATOM | 3267 | OG | SER | B | 118 | -15.949 | 23.907 | 55.744 | 1.00 | 39.92 | O |
| ATOM | 3268 | C | SER | B | 118 | -16.965 | 25.309 | 57.872 | 1.00 | 42.59 | C |
| ATOM | 3269 | O | SER | B | 118 | -16.355 | 24.723 | 58.761 | 1.00 | 42.89 | O |
| ATOM | 3270 | N | SER | B | 119 | -18.295 | 25.319 | 57.781 | 1.00 | 45.03 | N |
| ATOM | 3271 | CA | SER | B | 119 | -19.148 | 24.633 | 58.750 | 1.00 | 46.39 | C |
| ATOM | 3272 | CB | SER | B | 119 | -20.377 | 25.491 | 59.056 | 1.00 | 47.38 | C |
| ATOM | 3273 | OG | SER | B | 119 | -20.024 | 26.685 | 59.748 | 1.00 | 46.67 | O |
| ATOM | 3274 | C | SER | B | 119 | -19.598 | 23.262 | 58.248 | 1.00 | 47.23 | C |
| ATOM | 3275 | O | SER | B | 119 | -19.799 | 22.333 | 59.037 | 1.00 | 47.46 | O |
| ATOM | 3276 | N | ALA | B | 125 | -17.985 | 20.959 | 64.031 | 1.00 | 56.01 | N |

| | | | | | | | | | | | |
|---|---|---|---|---|---|---|---|---|---|---|---|
| ATOM | 3277 | CA | ALA | B | 125 | -18.160 | 22.391 | 64.258 | 1.00 | 56.69 | C |
| ATOM | 3278 | CB | ALA | B | 125 | -17.540 | 22.794 | 65.611 | 1.00 | 57.13 | C |
| ATOM | 3279 | C | ALA | B | 125 | -17.503 | 23.166 | 63.135 | 1.00 | 56.42 | C |
| ATOM | 3280 | O | ALA | B | 125 | -18.168 | 23.598 | 62.190 | 1.00 | 57.33 | O |
| ATOM | 3281 | N | ALA | B | 126 | -16.187 | 23.332 | 63.251 | 1.00 | 54.73 | N |
| ATOM | 3282 | CA | ALA | B | 126 | -15.395 | 24.042 | 62.250 | 1.00 | 52.37 | C |
| ATOM | 3283 | CB | ALA | B | 126 | -14.532 | 25.111 | 62.926 | 1.00 | 51.59 | C |
| ATOM | 3284 | C | ALA | B | 126 | -14.517 | 23.039 | 61.523 | 1.00 | 50.57 | C |
| ATOM | 3285 | O | ALA | B | 126 | -13.972 | 22.125 | 62.132 | 1.00 | 50.34 | O |
| ATOM | 3286 | N | TYR | B | 127 | -14.408 | 23.189 | 60.213 | 1.00 | 48.39 | N |
| ATOM | 3287 | CA | TYR | B | 127 | -13.568 | 22.295 | 59.432 | 1.00 | 46.81 | C |
| ATOM | 3288 | CB | TYR | B | 127 | -14.319 | 21.666 | 58.258 | 1.00 | 48.72 | C |
| ATOM | 3289 | CG | TYR | B | 127 | -15.254 | 20.536 | 58.590 | 1.00 | 50.22 | C |
| ATOM | 3290 | CD1 | TYR | B | 127 | -16.439 | 20.764 | 59.269 | 1.00 | 51.31 | C |
| ATOM | 3291 | CE1 | TYR | B | 127 | -17.339 | 19.731 | 59.497 | 1.00 | 52.97 | C |
| ATOM | 3292 | CD2 | TYR | B | 127 | -14.985 | 19.245 | 58.145 | 1.00 | 52.34 | C |
| ATOM | 3293 | CE2 | TYR | B | 127 | -15.872 | 18.207 | 58.366 | 1.00 | 53.22 | C |
| ATOM | 3294 | CZ | TYR | B | 127 | -17.052 | 18.455 | 59.032 | 1.00 | 53.26 | C |
| ATOM | 3295 | OH | TYR | B | 127 | -17.946 | 17.424 | 59.216 | 1.00 | 54.20 | O |
| ATOM | 3296 | C | TYR | B | 127 | -12.468 | 23.135 | 58.840 | 1.00 | 44.92 | C |
| ATOM | 3297 | O | TYR | B | 127 | -12.653 | 24.323 | 58.582 | 1.00 | 43.77 | O |
| ATOM | 3298 | N | LEU | B | 128 | -11.319 | 22.511 | 58.627 | 1.00 | 42.47 | N |
| ATOM | 3299 | CA | LEU | B | 128 | -10.206 | 23.200 | 58.010 | 1.00 | 39.82 | C |
| ATOM | 3300 | CB | LEU | B | 128 | -8.881 | 22.834 | 58.668 | 1.00 | 37.96 | C |
| ATOM | 3301 | CG | LEU | B | 128 | -7.707 | 23.437 | 57.901 | 1.00 | 38.87 | C |
| ATOM | 3302 | CD1 | LEU | B | 128 | -7.644 | 24.942 | 58.118 | 1.00 | 37.81 | C |
| ATOM | 3303 | CD2 | LEU | B | 128 | -6.427 | 22.775 | 58.332 | 1.00 | 39.69 | C |
| ATOM | 3304 | C | LEU | B | 128 | -10.203 | 22.704 | 56.586 | 1.00 | 38.78 | C |
| ATOM | 3305 | O | LEU | B | 128 | -10.403 | 21.515 | 56.338 | 1.00 | 38.43 | O |
| ATOM | 3306 | N | ASN | B | 129 | -9.982 | 23.607 | 55.646 | 1.00 | 36.97 | N |
| ATOM | 3307 | CA | ASN | B | 129 | -9.963 | 23.216 | 54.253 | 1.00 | 35.86 | C |
| ATOM | 3308 | CB | ASN | B | 129 | -11.095 | 23.923 | 53.504 | 1.00 | 35.29 | C |
| ATOM | 3309 | CG | ASN | B | 129 | -12.468 | 23.465 | 53.965 | 1.00 | 35.77 | C |
| ATOM | 3310 | OD1 | ASN | B | 129 | -13.011 | 22.493 | 53.454 | 1.00 | 38.56 | O |
| ATOM | 3311 | ND2 | ASN | B | 129 | -13.025 | 24.154 | 54.948 | 1.00 | 36.42 | N |
| ATOM | 3312 | C | ASN | B | 129 | -8.611 | 23.589 | 53.690 | 1.00 | 35.65 | C |
| ATOM | 3313 | O | ASN | B | 129 | -8.185 | 24.738 | 53.784 | 1.00 | 37.49 | O |
| ATOM | 3314 | N | LEU | B | 130 | -7.919 | 22.602 | 53.139 | 1.00 | 35.50 | N |
| ATOM | 3315 | CA | LEU | B | 130 | -6.612 | 22.822 | 52.554 | 1.00 | 35.02 | C |
| ATOM | 3316 | CB | LEU | B | 130 | -5.635 | 21.751 | 53.044 | 1.00 | 35.28 | C |
| ATOM | 3317 | CG | LEU | B | 130 | -5.266 | 21.808 | 54.530 | 1.00 | 35.77 | C |
| ATOM | 3318 | CD1 | LEU | B | 130 | -4.610 | 20.511 | 54.986 | 1.00 | 34.79 | C |
| ATOM | 3319 | CD2 | LEU | B | 130 | -4.341 | 22.993 | 54.746 | 1.00 | 37.88 | C |
| ATOM | 3320 | C | LEU | B | 130 | -6.780 | 22.718 | 51.051 | 1.00 | 35.11 | C |
| ATOM | 3321 | O | LEU | B | 130 | -7.239 | 21.691 | 50.555 | 1.00 | 36.42 | O |
| ATOM | 3322 | N | VAL | B | 131 | -6.444 | 23.782 | 50.329 | 1.00 | 34.66 | N |
| ATOM | 3323 | CA | VAL | B | 131 | -6.547 | 23.763 | 48.874 | 1.00 | 36.20 | C |
| ATOM | 3324 | CB | VAL | B | 131 | -6.987 | 25.123 | 48.310 | 1.00 | 37.42 | C |
| ATOM | 3325 | CG1 | VAL | B | 131 | -7.187 | 25.020 | 46.802 | 1.00 | 37.00 | C |
| ATOM | 3326 | CG2 | VAL | B | 131 | -8.284 | 25.566 | 48.984 | 1.00 | 37.32 | C |
| ATOM | 3327 | C | VAL | B | 131 | -5.157 | 23.423 | 48.365 | 1.00 | 37.41 | C |
| ATOM | 3328 | O | VAL | B | 131 | -4.206 | 24.162 | 48.613 | 1.00 | 39.47 | O |
| ATOM | 3329 | N | LEU | B | 132 | -5.041 | 22.312 | 47.641 | 1.00 | 37.70 | N |
| ATOM | 3330 | CA | LEU | B | 132 | -3.744 | 21.846 | 47.166 | 1.00 | 36.92 | C |
| ATOM | 3331 | CB | LEU | B | 132 | -3.373 | 20.583 | 47.931 | 1.00 | 35.83 | C |
| ATOM | 3332 | CG | LEU | B | 132 | -3.358 | 20.720 | 49.446 | 1.00 | 35.10 | C |
| ATOM | 3333 | CD1 | LEU | B | 132 | -3.955 | 19.480 | 50.072 | 1.00 | 34.42 | C |
| ATOM | 3334 | CD2 | LEU | B | 132 | -1.949 | 20.964 | 49.916 | 1.00 | 31.89 | C |
| ATOM | 3335 | C | LEU | B | 132 | -3.658 | 21.535 | 45.687 | 1.00 | 38.35 | C |
| ATOM | 3336 | O | LEU | B | 132 | -4.669 | 21.304 | 45.031 | 1.00 | 39.20 | O |
| ATOM | 3337 | N | ASP | B | 133 | -2.431 | 21.515 | 45.174 | 1.00 | 39.75 | N |
| ATOM | 3338 | CA | ASP | B | 133 | -2.180 | 21.189 | 43.774 | 1.00 | 41.83 | C |
| ATOM | 3339 | CB | ASP | B | 133 | -0.682 | 20.958 | 43.524 | 1.00 | 44.76 | C |
| ATOM | 3340 | CG | ASP | B | 133 | 0.113 | 22.240 | 43.353 | 1.00 | 47.88 | C |
| ATOM | 3341 | OD1 | ASP | B | 133 | 1.342 | 22.197 | 43.668 | 1.00 | 47.86 | O |
| ATOM | 3342 | OD2 | ASP | B | 133 | -0.468 | 23.256 | 42.888 | 1.00 | 46.54 | O |
| ATOM | 3343 | C | ASP | B | 133 | -2.880 | 19.860 | 43.529 | 1.00 | 43.11 | C |

| | | | | | | | | | | | |
|---|---|---|---|---|---|---|---|---|---|---|---|
| ATOM | 3344 | O | ASP | B | 133 | -2.796 | 18.943 | 44.355 | 1.00 | 42.47 | O |
| ATOM | 3345 | N | TYR | B | 134 | -3.548 | 19.740 | 42.391 | 1.00 | 43.69 | N |
| ATOM | 3346 | CA | TYR | B | 134 | -4.209 | 18.492 | 42.063 | 1.00 | 42.89 | C |
| ATOM | 3347 | CB | TYR | B | 134 | -5.596 | 18.733 | 41.476 | 1.00 | 43.93 | C |
| ATOM | 3348 | CG | TYR | B | 134 | -6.266 | 17.455 | 41.051 | 1.00 | 43.85 | C |
| ATOM | 3349 | CD1 | TYR | B | 134 | -6.792 | 16.579 | 41.997 | 1.00 | 44.88 | C |
| ATOM | 3350 | CE1 | TYR | B | 134 | -7.373 | 15.381 | 41.617 | 1.00 | 45.46 | C |
| ATOM | 3351 | CD2 | TYR | B | 134 | -6.335 | 17.101 | 39.709 | 1.00 | 43.13 | C |
| ATOM | 3352 | CE2 | TYR | B | 134 | -6.913 | 15.904 | 39.314 | 1.00 | 44.38 | C |
| ATOM | 3353 | CZ | TYR | B | 134 | -7.432 | 15.046 | 40.273 | 1.00 | 45.66 | C |
| ATOM | 3354 | OH | TYR | B | 134 | -8.022 | 13.853 | 39.893 | 1.00 | 46.16 | O |
| ATOM | 3355 | C | TYR | B | 134 | -3.376 | 17.749 | 41.041 | 1.00 | 42.19 | C |
| ATOM | 3356 | O | TYR | B | 134 | -3.388 | 18.092 | 39.866 | 1.00 | 43.08 | O |
| ATOM | 3357 | N | VAL | B | 135 | -2.648 | 16.733 | 41.482 | 1.00 | 41.87 | N |
| ATOM | 3358 | CA | VAL | B | 135 | -1.829 | 15.957 | 40.563 | 1.00 | 41.04 | C |
| ATOM | 3359 | CB | VAL | B | 135 | -0.374 | 15.880 | 41.050 | 1.00 | 39.57 | C |
| ATOM | 3360 | CG1 | VAL | B | 135 | 0.486 | 15.214 | 40.002 | 1.00 | 37.45 | C |
| ATOM | 3361 | CG2 | VAL | B | 135 | 0.140 | 17.276 | 41.372 | 1.00 | 36.92 | C |
| ATOM | 3362 | C | VAL | B | 135 | -2.462 | 14.573 | 40.517 | 1.00 | 42.83 | C |
| ATOM | 3363 | O | VAL | B | 135 | -2.492 | 13.857 | 41.518 | 1.00 | 44.49 | O |
| ATOM | 3364 | N | PRO | B | 136 | -2.984 | 14.179 | 39.345 | 1.00 | 44.08 | N |
| ATOM | 3365 | CD | PRO | B | 136 | -2.917 | 14.926 | 38.074 | 1.00 | 45.07 | C |
| ATOM | 3366 | CA | PRO | B | 136 | -3.641 | 12.882 | 39.147 | 1.00 | 43.50 | C |
| ATOM | 3367 | CB | PRO | B | 136 | -4.142 | 12.968 | 37.707 | 1.00 | 43.50 | C |
| ATOM | 3368 | CG | PRO | B | 136 | -3.128 | 13.834 | 37.045 | 1.00 | 43.63 | C |
| ATOM | 3369 | C | PRO | B | 136 | -2.858 | 11.601 | 39.398 | 1.00 | 41.58 | C |
| ATOM | 3370 | O | PRO | B | 136 | -3.195 | 10.815 | 40.283 | 1.00 | 41.23 | O |
| ATOM | 3371 | N | GLU | B | 137 | -1.816 | 11.393 | 38.614 | 1.00 | 39.26 | N |
| ATOM | 3372 | CA | GLU | B | 137 | -1.041 | 10.178 | 38.728 | 1.00 | 37.78 | C |
| ATOM | 3373 | CB | GLU | B | 137 | -0.183 | 10.015 | 37.469 | 1.00 | 37.87 | C |
| ATOM | 3374 | CG | GLU | B | 137 | -0.432 | 8.695 | 36.737 | 1.00 | 41.82 | C |
| ATOM | 3375 | CD | GLU | B | 137 | -1.820 | 8.147 | 37.006 | 1.00 | 44.15 | C |
| ATOM | 3376 | OE1 | GLU | B | 137 | -2.809 | 8.780 | 36.578 | 1.00 | 44.69 | O |
| ATOM | 3377 | OE2 | GLU | B | 137 | -1.929 | 7.089 | 37.659 | 1.00 | 47.43 | O |
| ATOM | 3378 | C | GLU | B | 137 | -0.188 | 10.053 | 39.987 | 1.00 | 36.39 | C |
| ATOM | 3379 | O | GLU | B | 137 | 0.261 | 11.049 | 40.562 | 1.00 | 35.94 | O |
| ATOM | 3380 | N | THR | B | 138 | 0.025 | 8.807 | 40.405 | 1.00 | 33.49 | N |
| ATOM | 3381 | CA | THR | B | 138 | 0.838 | 8.504 | 41.575 | 1.00 | 33.11 | C |
| ATOM | 3382 | CB | THR | B | 138 | -0.003 | 7.994 | 42.765 | 1.00 | 32.27 | C |
| ATOM | 3383 | OG1 | THR | B | 138 | -0.511 | 6.689 | 42.466 | 1.00 | 33.46 | O |
| ATOM | 3384 | CG2 | THR | B | 138 | -1.162 | 8.929 | 43.047 | 1.00 | 30.50 | C |
| ATOM | 3385 | C | THR | B | 138 | 1.811 | 7.397 | 41.206 | 1.00 | 33.45 | C |
| ATOM | 3386 | O | THR | B | 138 | 1.517 | 6.559 | 40.351 | 1.00 | 34.33 | O |
| ATOM | 3387 | N | VAL | B | 139 | 2.975 | 7.398 | 41.840 | 1.00 | 32.99 | N |
| ATOM | 3388 | CA | VAL | B | 139 | 3.969 | 6.374 | 41.584 | 1.00 | 30.90 | C |
| ATOM | 3389 | CB | VAL | B | 139 | 5.192 | 6.556 | 42.520 | 1.00 | 30.72 | C |
| ATOM | 3390 | CG1 | VAL | B | 139 | 6.129 | 5.364 | 42.423 | 1.00 | 30.13 | C |
| ATOM | 3391 | CG2 | VAL | B | 139 | 5.924 | 7.835 | 42.166 | 1.00 | 27.08 | C |
| ATOM | 3392 | C | VAL | B | 139 | 3.317 | 5.011 | 41.834 | 1.00 | 32.68 | C |
| ATOM | 3393 | O | VAL | B | 139 | 3.678 | 4.016 | 41.206 | 1.00 | 33.95 | O |
| ATOM | 3394 | N | TYR | B | 140 | 2.346 | 4.966 | 42.743 | 1.00 | 32.74 | N |
| ATOM | 3395 | CA | TYR | B | 140 | 1.661 | 3.713 | 43.047 | 1.00 | 33.80 | C |
| ATOM | 3396 | CB | TYR | B | 140 | 0.679 | 3.877 | 44.210 | 1.00 | 35.18 | C |
| ATOM | 3397 | CG | TYR | B | 140 | -0.167 | 2.634 | 44.402 | 1.00 | 35.87 | C |
| ATOM | 3398 | CD1 | TYR | B | 140 | 0.385 | 1.464 | 44.924 | 1.00 | 36.43 | C |
| ATOM | 3399 | CE1 | TYR | B | 140 | -0.365 | 0.290 | 45.012 | 1.00 | 38.08 | C |
| ATOM | 3400 | CD2 | TYR | B | 140 | -1.493 | 2.600 | 43.979 | 1.00 | 36.84 | C |
| ATOM | 3401 | CE2 | TYR | B | 140 | -2.250 | 1.432 | 44.061 | 1.00 | 38.13 | C |
| ATOM | 3402 | CZ | TYR | B | 140 | -1.680 | 0.283 | 44.576 | 1.00 | 38.13 | C |
| ATOM | 3403 | OH | TYR | B | 140 | -2.419 | -0.872 | 44.648 | 1.00 | 40.21 | O |
| ATOM | 3404 | C | TYR | B | 140 | 0.887 | 3.136 | 41.863 | 1.00 | 34.52 | C |
| ATOM | 3405 | O | TYR | B | 140 | 0.911 | 1.933 | 41.625 | 1.00 | 32.82 | O |
| ATOM | 3406 | N | ARG | B | 141 | 0.176 | 4.001 | 41.150 | 1.00 | 34.70 | N |
| ATOM | 3407 | CA | ARG | B | 141 | -0.601 | 3.589 | 40.000 | 1.00 | 35.96 | C |
| ATOM | 3408 | CB | ARG | B | 141 | -1.497 | 4.736 | 39.540 | 1.00 | 40.39 | C |
| ATOM | 3409 | CG | ARG | B | 141 | -2.657 | 5.031 | 40.476 | 1.00 | 46.02 | C |
| ATOM | 3410 | CD | ARG | B | 141 | -2.966 | 6.525 | 40.541 | 1.00 | 49.60 | C |

| | | | | | | | | | | | |
|---|---|---|---|---|---|---|---|---|---|---|---|
| ATOM | 3411 | NE | ARG | B | 141 | -4.316 | 6.764 | 41.040 | 1.00 | 53.63 | N |
| ATOM | 3412 | CZ | ARG | B | 141 | -5.404 | 6.755 | 40.276 | 1.00 | 54.94 | C |
| ATOM | 3413 | NH1 | ARG | B | 141 | -5.294 | 6.526 | 38.971 | 1.00 | 55.30 | N |
| ATOM | 3414 | NH2 | ARG | B | 141 | -6.600 | 6.956 | 40.822 | 1.00 | 55.89 | N |
| ATOM | 3415 | C | ARG | B | 141 | 0.328 | 3.176 | 38.873 | 1.00 | 35.01 | C |
| ATOM | 3416 | O | ARG | B | 141 | 0.095 | 2.167 | 38.217 | 1.00 | 33.61 | O |
| ATOM | 3417 | N | VAL | B | 142 | 1.384 | 3.947 | 38.652 | 1.00 | 34.19 | N |
| ATOM | 3418 | CA | VAL | B | 142 | 2.311 | 3.610 | 37.589 | 1.00 | 33.82 | C |
| ATOM | 3419 | CB | VAL | B | 142 | 3.369 | 4.704 | 37.366 | 1.00 | 32.08 | C |
| ATOM | 3420 | CG1 | VAL | B | 142 | 4.421 | 4.218 | 36.391 | 1.00 | 32.29 | C |
| ATOM | 3421 | CG2 | VAL | B | 142 | 2.710 | 5.955 | 36.822 | 1.00 | 31.53 | C |
| ATOM | 3422 | C | VAL | B | 142 | 3.009 | 2.304 | 37.903 | 1.00 | 35.17 | C |
| ATOM | 3423 | O | VAL | B | 142 | 3.136 | 1.449 | 37.035 | 1.00 | 36.96 | O |
| ATOM | 3424 | N | ALA | B | 143 | 3.456 | 2.137 | 39.141 | 1.00 | 34.60 | N |
| ATOM | 3425 | CA | ALA | B | 143 | 4.133 | 0.905 | 39.512 | 1.00 | 34.60 | C |
| ATOM | 3426 | CB | ALA | B | 143 | 4.619 | 0.984 | 40.938 | 1.00 | 34.72 | C |
| ATOM | 3427 | C | ALA | B | 143 | 3.170 | -0.263 | 39.345 | 1.00 | 34.26 | C |
| ATOM | 3428 | O | ALA | B | 143 | 3.558 | -1.340 | 38.896 | 1.00 | 34.68 | O |
| ATOM | 3429 | N | ARG | B | 144 | 1.913 | -0.036 | 39.698 | 1.00 | 35.61 | N |
| ATOM | 3430 | CA | ARG | B | 144 | 0.884 | -1.060 | 39.590 | 1.00 | 38.20 | C |
| ATOM | 3431 | CB | ARG | B | 144 | -0.414 | -0.555 | 40.228 | 1.00 | 39.15 | C |
| ATOM | 3432 | CG | ARG | B | 144 | -1.336 | -1.658 | 40.713 | 1.00 | 40.25 | C |
| ATOM | 3433 | CD | ARG | B | 144 | -2.634 | -1.669 | 39.940 | 1.00 | 42.20 | C |
| ATOM | 3434 | NE | ARG | B | 144 | -3.407 | -0.452 | 40.159 | 1.00 | 45.07 | N |
| ATOM | 3435 | CZ | ARG | B | 144 | -4.183 | -0.232 | 41.216 | 1.00 | 46.54 | C |
| ATOM | 3436 | NH1 | ARG | B | 144 | -4.303 | -1.154 | 42.169 | 1.00 | 47.11 | N |
| ATOM | 3437 | NH2 | ARG | B | 144 | -4.832 | 0.921 | 41.325 | 1.00 | 47.37 | N |
| ATOM | 3438 | C | ARG | B | 144 | 0.646 | -1.437 | 38.125 | 1.00 | 39.31 | C |
| ATOM | 3439 | O | ARG | B | 144 | 0.571 | -2.617 | 37.789 | 1.00 | 39.03 | O |
| ATOM | 3440 | N | HIS | B | 145 | 0.534 | -0.421 | 37.267 | 1.00 | 41.76 | N |
| ATOM | 3441 | CA | HIS | B | 145 | 0.314 | -0.596 | 35.828 | 1.00 | 43.21 | C |
| ATOM | 3442 | CB | HIS | B | 145 | 0.273 | 0.780 | 35.143 | 1.00 | 45.91 | C |
| ATOM | 3443 | CG | HIS | B | 145 | 0.124 | 0.730 | 33.651 | 1.00 | 51.22 | C |
| ATOM | 3444 | CD2 | HIS | B | 145 | 0.280 | -0.283 | 32.761 | 1.00 | 52.90 | C |
| ATOM | 3445 | ND1 | HIS | B | 145 | -0.197 | 1.846 | 32.902 | 1.00 | 53.04 | N |
| ATOM | 3446 | CE1 | HIS | B | 145 | -0.233 | 1.523 | 31.619 | 1.00 | 52.48 | C |
| ATOM | 3447 | NE2 | HIS | B | 145 | 0.053 | 0.236 | 31.507 | 1.00 | 52.27 | N |
| ATOM | 3448 | C | HIS | B | 145 | 1.405 | -1.473 | 35.218 | 1.00 | 43.01 | C |
| ATOM | 3449 | O | HIS | B | 145 | 1.096 | -2.476 | 34.584 | 1.00 | 43.47 | O |
| ATOM | 3450 | N | TYR | B | 146 | 2.673 | -1.106 | 35.410 | 1.00 | 42.90 | N |
| ATOM | 3451 | CA | TYR | B | 146 | 3.782 | -1.892 | 34.874 | 1.00 | 41.49 | C |
| ATOM | 3452 | CB | TYR | B | 146 | 5.133 | -1.263 | 35.192 | 1.00 | 38.50 | C |
| ATOM | 3453 | CG | TYR | B | 146 | 5.517 | -0.160 | 34.262 | 1.00 | 37.79 | C |
| ATOM | 3454 | CD1 | TYR | B | 146 | 4.901 | 1.081 | 34.351 | 1.00 | 37.16 | C |
| ATOM | 3455 | CE1 | TYR | B | 146 | 5.215 | 2.097 | 33.476 | 1.00 | 37.56 | C |
| ATOM | 3456 | CD2 | TYR | B | 146 | 6.473 | -0.361 | 33.257 | 1.00 | 36.64 | C |
| ATOM | 3457 | CE2 | TYR | B | 146 | 6.796 | 0.661 | 32.368 | 1.00 | 37.21 | C |
| ATOM | 3458 | CZ | TYR | B | 146 | 6.156 | 1.887 | 32.487 | 1.00 | 36.81 | C |
| ATOM | 3459 | OH | TYR | B | 146 | 6.428 | 2.925 | 31.634 | 1.00 | 38.59 | O |
| ATOM | 3460 | C | TYR | B | 146 | 3.813 | -3.280 | 35.438 | 1.00 | 43.03 | C |
| ATOM | 3461 | O | TYR | B | 146 | 4.306 | -4.203 | 34.799 | 1.00 | 45.83 | O |
| ATOM | 3462 | N | SER | B | 147 | 3.300 | -3.434 | 36.646 | 1.00 | 43.41 | N |
| ATOM | 3463 | CA | SER | B | 147 | 3.334 | -4.728 | 37.299 | 1.00 | 45.15 | C |
| ATOM | 3464 | CB | SER | B | 147 | 3.300 | -4.544 | 38.810 | 1.00 | 45.39 | C |
| ATOM | 3465 | OG | SER | B | 147 | 3.080 | -5.794 | 39.440 | 1.00 | 48.40 | O |
| ATOM | 3466 | C | SER | B | 147 | 2.252 | -5.718 | 36.895 | 1.00 | 46.19 | C |
| ATOM | 3467 | O | SER | B | 147 | 2.529 | -6.909 | 36.743 | 1.00 | 45.63 | O |
| ATOM | 3468 | N | ARG | B | 148 | 1.018 | -5.250 | 36.746 | 1.00 | 47.31 | N |
| ATOM | 3469 | CA | ARG | B | 148 | -0.050 | -6.160 | 36.355 | 1.00 | 49.70 | C |
| ATOM | 3470 | CB | ARG | B | 148 | -1.376 | -5.404 | 36.217 | 1.00 | 50.73 | C |
| ATOM | 3471 | CG | ARG | B | 148 | -2.024 | -5.050 | 37.545 | 1.00 | 52.52 | C |
| ATOM | 3472 | CD | ARG | B | 148 | -3.238 | -4.165 | 37.332 | 1.00 | 53.64 | C |
| ATOM | 3473 | NE | ARG | B | 148 | -4.100 | -4.124 | 38.512 | 1.00 | 55.34 | N |
| ATOM | 3474 | CZ | ARG | B | 148 | -4.964 | -3.144 | 38.776 | 1.00 | 55.69 | C |
| ATOM | 3475 | NH1 | ARG | B | 148 | -5.077 | -2.111 | 37.950 | 1.00 | 54.98 | N |
| ATOM | 3476 | NH2 | ARG | B | 148 | -5.735 | -3.208 | 39.858 | 1.00 | 55.91 | N |
| ATOM | 3477 | C | ARG | B | 148 | 0.309 | -6.842 | 35.030 | 1.00 | 50.61 | C |

| | | | | | | | | | | | |
|---|---|---|---|---|---|---|---|---|---|---|---|
| ATOM | 3478 | O | ARG | B | 148 | -0.008 | -8.013 | 34.812 | 1.00 | 50.85 | O |
| ATOM | 3479 | N | ALA | B | 149 | 0.989 | -6.106 | 34.156 | 1.00 | 50.88 | N |
| ATOM | 3480 | CA | ALA | B | 149 | 1.391 | -6.642 | 32.858 | 1.00 | 51.66 | C |
| ATOM | 3481 | CB | ALA | B | 149 | 1.221 | -5.581 | 31.778 | 1.00 | 50.52 | C |
| ATOM | 3482 | C | ALA | B | 149 | 2.832 | -7.153 | 32.868 | 1.00 | 51.87 | C |
| ATOM | 3483 | O | ALA | B | 149 | 3.562 | -7.006 | 31.885 | 1.00 | 51.22 | O |
| ATOM | 3484 | N | LYS | B | 150 | 3.227 | -7.752 | 33.991 | 1.00 | 52.85 | N |
| ATOM | 3485 | CA | LYS | B | 150 | 4.562 | -8.315 | 34.150 | 1.00 | 53.30 | C |
| ATOM | 3486 | CB | LYS | B | 150 | 4.575 | -9.757 | 33.649 | 1.00 | 55.11 | C |
| ATOM | 3487 | CG | LYS | B | 150 | 4.662 | -10.783 | 34.764 | 1.00 | 58.20 | C |
| ATOM | 3488 | CD | LYS | B | 150 | 3.308 | -11.113 | 35.394 | 1.00 | 59.36 | C |
| ATOM | 3489 | CE | LYS | B | 150 | 2.703 | -12.387 | 34.763 | 1.00 | 60.61 | C |
| ATOM | 3490 | NZ | LYS | B | 150 | 1.622 | -13.015 | 35.598 | 1.00 | 59.70 | N |
| ATOM | 3491 | C | LYS | B | 150 | 5.684 | -7.537 | 33.464 | 1.00 | 53.40 | C |
| ATOM | 3492 | O | LYS | B | 150 | 6.507 | -8.114 | 32.761 | 1.00 | 53.30 | O |
| ATOM | 3493 | N | GLN | B | 151 | 5.715 | -6.225 | 33.660 | 1.00 | 53.27 | N |
| ATOM | 3494 | CA | GLN | B | 151 | 6.758 | -5.404 | 33.061 | 1.00 | 51.45 | C |
| ATOM | 3495 | CB | GLN | B | 151 | 6.142 | -4.338 | 32.163 | 1.00 | 51.15 | C |
| ATOM | 3496 | CG | GLN | B | 151 | 7.151 | -3.604 | 31.291 | 1.00 | 53.32 | C |
| ATOM | 3497 | CD | GLN | B | 151 | 7.375 | -4.292 | 29.961 | 1.00 | 54.22 | C |
| ATOM | 3498 | OE1 | GLN | B | 151 | 6.431 | -4.805 | 29.361 | 1.00 | 56.36 | O |
| ATOM | 3499 | NE2 | GLN | B | 151 | 8.618 | -4.290 | 29.481 | 1.00 | 53.71 | N |
| ATOM | 3500 | C | GLN | B | 151 | 7.537 | -4.735 | 34.192 | 1.00 | 50.09 | C |
| ATOM | 3501 | O | GLN | B | 151 | 7.055 | -4.625 | 35.321 | 1.00 | 48.80 | O |
| ATOM | 3502 | N | THR | B | 152 | 8.739 | -4.279 | 33.876 | 1.00 | 48.11 | N |
| ATOM | 3503 | CA | THR | B | 152 | 9.599 | -3.633 | 34.856 | 1.00 | 46.20 | C |
| ATOM | 3504 | CB | THR | B | 152 | 10.943 | -4.381 | 34.957 | 1.00 | 46.58 | C |
| ATOM | 3505 | OG1 | THR | B | 152 | 11.969 | -3.477 | 35.374 | 1.00 | 47.47 | O |
| ATOM | 3506 | CG2 | THR | B | 152 | 11.315 | -4.991 | 33.610 | 1.00 | 48.20 | C |
| ATOM | 3507 | C | THR | B | 152 | 9.849 | -2.182 | 34.478 | 1.00 | 43.72 | C |
| ATOM | 3508 | O | THR | B | 152 | 10.485 | -1.896 | 33.466 | 1.00 | 44.29 | O |
| ATOM | 3509 | N | LEU | B | 153 | 9.334 | -1.271 | 35.291 | 1.00 | 40.95 | N |
| ATOM | 3510 | CA | LEU | B | 153 | 9.494 | 0.157 | 35.043 | 1.00 | 38.98 | C |
| ATOM | 3511 | CB | LEU | B | 153 | 9.124 | 0.936 | 36.300 | 1.00 | 38.19 | C |
| ATOM | 3512 | CG | LEU | B | 153 | 9.118 | 2.457 | 36.207 | 1.00 | 38.36 | C |
| ATOM | 3513 | CD1 | LEU | B | 153 | 7.841 | 2.930 | 35.547 | 1.00 | 35.98 | C |
| ATOM | 3514 | CD2 | LEU | B | 153 | 9.246 | 3.032 | 37.610 | 1.00 | 37.72 | C |
| ATOM | 3515 | C | LEU | B | 153 | 10.923 | 0.516 | 34.617 | 1.00 | 38.31 | C |
| ATOM | 3516 | O | LEU | B | 153 | 11.889 | 0.174 | 35.302 | 1.00 | 36.72 | O |
| ATOM | 3517 | N | PRO | B | 154 | 11.074 | 1.205 | 33.469 | 1.00 | 38.07 | N |
| ATOM | 3518 | CD | PRO | B | 154 | 10.025 | 1.603 | 32.511 | 1.00 | 38.38 | C |
| ATOM | 3519 | CA | PRO | B | 154 | 12.398 | 1.599 | 32.981 | 1.00 | 36.87 | C |
| ATOM | 3520 | CB | PRO | B | 154 | 12.067 | 2.547 | 31.840 | 1.00 | 36.50 | C |
| ATOM | 3521 | CG | PRO | B | 154 | 10.829 | 1.927 | 31.271 | 1.00 | 37.69 | C |
| ATOM | 3522 | C | PRO | B | 154 | 13.203 | 2.278 | 34.087 | 1.00 | 36.80 | C |
| ATOM | 3523 | O | PRO | B | 154 | 12.681 | 3.123 | 34.817 | 1.00 | 36.69 | O |
| ATOM | 3524 | N | VAL | B | 155 | 14.475 | 1.907 | 34.197 | 1.00 | 36.28 | N |
| ATOM | 3525 | CA | VAL | B | 155 | 15.360 | 2.452 | 35.216 | 1.00 | 35.66 | C |
| ATOM | 3526 | CB | VAL | B | 155 | 16.754 | 1.783 | 35.135 | 1.00 | 34.61 | C |
| ATOM | 3527 | CG1 | VAL | B | 155 | 17.645 | 2.262 | 36.270 | 1.00 | 33.77 | C |
| ATOM | 3528 | CG2 | VAL | B | 155 | 16.596 | 0.279 | 35.195 | 1.00 | 35.70 | C |
| ATOM | 3529 | C | VAL | B | 155 | 15.516 | 3.974 | 35.190 | 1.00 | 36.24 | C |
| ATOM | 3530 | O | VAL | B | 155 | 15.866 | 4.574 | 36.204 | 1.00 | 37.26 | O |
| ATOM | 3531 | N | ILE | B | 156 | 15.254 | 4.612 | 34.052 | 1.00 | 36.67 | N |
| ATOM | 3532 | CA | ILE | B | 156 | 15.376 | 6.068 | 33.982 | 1.00 | 35.85 | C |
| ATOM | 3533 | CB | ILE | B | 156 | 15.345 | 6.590 | 32.509 | 1.00 | 36.47 | C |
| ATOM | 3534 | CG2 | ILE | B | 156 | 14.037 | 6.211 | 31.836 | 1.00 | 37.16 | C |
| ATOM | 3535 | CG1 | ILE | B | 156 | 15.477 | 8.121 | 32.481 | 1.00 | 35.95 | C |
| ATOM | 3536 | CD1 | ILE | B | 156 | 16.752 | 8.657 | 33.121 | 1.00 | 36.69 | C |
| ATOM | 3537 | C | ILE | B | 156 | 14.250 | 6.721 | 34.779 | 1.00 | 35.42 | C |
| ATOM | 3538 | O | ILE | B | 156 | 14.435 | 7.783 | 35.366 | 1.00 | 35.96 | O |
| ATOM | 3539 | N | TYR | B | 157 | 13.083 | 6.084 | 34.799 | 1.00 | 35.03 | N |
| ATOM | 3540 | CA | TYR | B | 157 | 11.954 | 6.619 | 35.549 | 1.00 | 35.54 | C |
| ATOM | 3541 | CB | TYR | B | 157 | 10.662 | 5.890 | 35.147 | 1.00 | 35.92 | C |
| ATOM | 3542 | CG | TYR | B | 157 | 10.145 | 6.292 | 33.773 | 1.00 | 37.07 | C |
| ATOM | 3543 | CD1 | TYR | B | 157 | 9.938 | 5.341 | 32.768 | 1.00 | 37.20 | C |
| ATOM | 3544 | CE1 | TYR | B | 157 | 9.469 | 5.715 | 31.499 | 1.00 | 38.64 | C |

| | | | | | | | | | | | |
|---|---|---|---|---|---|---|---|---|---|---|---|
| ATOM | 3545 | CD2 | TYR | B | 157 | 9.865 | 7.629 | 33.478 | 1.00 | 38.47 | C |
| ATOM | 3546 | CE2 | TYR | B | 157 | 9.397 | 8.014 | 32.217 | 1.00 | 39.09 | C |
| ATOM | 3547 | CZ | TYR | B | 157 | 9.199 | 7.057 | 31.232 | 1.00 | 39.97 | C |
| ATOM | 3548 | OH | TYR | B | 157 | 8.737 | 7.444 | 29.987 | 1.00 | 39.12 | O |
| ATOM | 3549 | C | TYR | B | 157 | 12.264 | 6.438 | 37.041 | 1.00 | 35.59 | C |
| ATOM | 3550 | O | TYR | B | 157 | 11.992 | 7.316 | 37.868 | 1.00 | 33.02 | O |
| ATOM | 3551 | N | VAL | B | 158 | 12.866 | 5.294 | 37.358 | 1.00 | 34.67 | N |
| ATOM | 3552 | CA | VAL | B | 158 | 13.262 | 4.969 | 38.717 | 1.00 | 33.02 | C |
| ATOM | 3553 | CB | VAL | B | 158 | 13.905 | 3.562 | 38.782 | 1.00 | 33.52 | C |
| ATOM | 3554 | CG1 | VAL | B | 158 | 14.267 | 3.204 | 40.217 | 1.00 | 32.36 | C |
| ATOM | 3555 | CG2 | VAL | B | 158 | 12.935 | 2.533 | 38.216 | 1.00 | 31.12 | C |
| ATOM | 3556 | C | VAL | B | 158 | 14.264 | 6.013 | 39.223 | 1.00 | 32.88 | C |
| ATOM | 3557 | O | VAL | B | 158 | 14.106 | 6.563 | 40.315 | 1.00 | 32.44 | O |
| ATOM | 3558 | N | LYS | B | 159 | 15.287 | 6.304 | 38.426 | 1.00 | 30.90 | N |
| ATOM | 3559 | CA | LYS | B | 159 | 16.272 | 7.295 | 38.836 | 1.00 | 30.81 | C |
| ATOM | 3560 | CB | LYS | B | 159 | 17.377 | 7.432 | 37.790 | 1.00 | 30.12 | C |
| ATOM | 3561 | CG | LYS | B | 159 | 18.137 | 6.157 | 37.529 | 1.00 | 29.89 | C |
| ATOM | 3562 | CD | LYS | B | 159 | 19.210 | 6.392 | 36.495 | 1.00 | 31.10 | C |
| ATOM | 3563 | CE | LYS | B | 159 | 19.796 | 5.084 | 35.996 | 1.00 | 30.42 | C |
| ATOM | 3564 | NZ | LYS | B | 159 | 20.955 | 5.351 | 35.097 | 1.00 | 32.73 | N |
| ATOM | 3565 | C | LYS | B | 159 | 15.608 | 8.647 | 39.059 | 1.00 | 32.10 | C |
| ATOM | 3566 | O | LYS | B | 159 | 15.834 | 9.309 | 40.075 | 1.00 | 32.70 | O |
| ATOM | 3567 | N | LEU | B | 160 | 14.785 | 9.058 | 38.104 | 1.00 | 33.25 | N |
| ATOM | 3568 | CA | LEU | B | 160 | 14.083 | 10.330 | 38.197 | 1.00 | 33.73 | C |
| ATOM | 3569 | CB | LEU | B | 160 | 13.228 | 10.549 | 36.953 | 1.00 | 34.21 | C |
| ATOM | 3570 | CG | LEU | B | 160 | 13.968 | 10.975 | 35.686 | 1.00 | 34.33 | C |
| ATOM | 3571 | CD1 | LEU | B | 160 | 13.087 | 10.711 | 34.491 | 1.00 | 32.68 | C |
| ATOM | 3572 | CD2 | LEU | B | 160 | 14.364 | 12.445 | 35.786 | 1.00 | 33.82 | C |
| ATOM | 3573 | C | LEU | B | 160 | 13.194 | 10.446 | 39.424 | 1.00 | 34.27 | C |
| ATOM | 3574 | O | LEU | B | 160 | 13.262 | 11.433 | 40.150 | 1.00 | 33.76 | O |
| ATOM | 3575 | N | TYR | B | 161 | 12.354 | 9.445 | 39.656 | 1.00 | 34.76 | N |
| ATOM | 3576 | CA | TYR | B | 161 | 11.450 | 9.503 | 40.798 | 1.00 | 34.86 | C |
| ATOM | 3577 | CB | TYR | B | 161 | 10.484 | 8.317 | 40.790 | 1.00 | 36.64 | C |
| ATOM | 3578 | CG | TYR | B | 161 | 9.583 | 8.259 | 39.578 | 1.00 | 37.45 | C |
| ATOM | 3579 | CD1 | TYR | B | 161 | 9.345 | 9.386 | 38.803 | 1.00 | 36.68 | C |
| ATOM | 3580 | CE1 | TYR | B | 161 | 8.529 | 9.320 | 37.678 | 1.00 | 39.24 | C |
| ATOM | 3581 | CD2 | TYR | B | 161 | 8.978 | 7.065 | 39.203 | 1.00 | 39.55 | C |
| ATOM | 3582 | CE2 | TYR | B | 161 | 8.158 | 6.988 | 38.084 | 1.00 | 40.00 | C |
| ATOM | 3583 | CZ | TYR | B | 161 | 7.938 | 8.114 | 37.326 | 1.00 | 40.28 | C |
| ATOM | 3584 | OH | TYR | B | 161 | 7.126 | 8.031 | 36.217 | 1.00 | 42.16 | O |
| ATOM | 3585 | C | TYR | B | 161 | 12.163 | 9.567 | 42.136 | 1.00 | 33.69 | C |
| ATOM | 3586 | O | TYR | B | 161 | 11.913 | 10.475 | 42.926 | 1.00 | 32.29 | O |
| ATOM | 3587 | N | MET | B | 162 | 13.045 | 8.607 | 42.391 | 1.00 | 33.73 | N |
| ATOM | 3588 | CA | MET | B | 162 | 13.783 | 8.566 | 43.651 | 1.00 | 32.75 | C |
| ATOM | 3589 | CB | MET | B | 162 | 14.720 | 7.360 | 43.679 | 1.00 | 32.53 | C |
| ATOM | 3590 | CG | MET | B | 162 | 14.005 | 6.016 | 43.636 | 1.00 | 32.77 | C |
| ATOM | 3591 | SD | MET | B | 162 | 12.722 | 5.848 | 44.888 | 1.00 | 32.78 | S |
| ATOM | 3592 | CE | MET | B | 162 | 13.656 | 6.164 | 46.405 | 1.00 | 33.30 | C |
| ATOM | 3593 | C | MET | B | 162 | 14.587 | 9.838 | 43.888 | 1.00 | 32.46 | C |
| ATOM | 3594 | O | MET | B | 162 | 14.595 | 10.371 | 44.995 | 1.00 | 32.49 | O |
| ATOM | 3595 | N | TYR | B | 163 | 15.262 | 10.320 | 42.852 | 1.00 | 31.48 | N |
| ATOM | 3596 | CA | TYR | B | 163 | 16.051 | 11.526 | 42.990 | 1.00 | 32.59 | C |
| ATOM | 3597 | CB | TYR | B | 163 | 16.670 | 11.925 | 41.657 | 1.00 | 33.34 | C |
| ATOM | 3598 | CG | TYR | B | 163 | 17.474 | 13.203 | 41.735 | 1.00 | 33.33 | C |
| ATOM | 3599 | CD1 | TYR | B | 163 | 18.783 | 13.198 | 42.204 | 1.00 | 33.88 | C |
| ATOM | 3600 | CE1 | TYR | B | 163 | 19.528 | 14.371 | 42.271 | 1.00 | 33.10 | C |
| ATOM | 3601 | CD2 | TYR | B | 163 | 16.925 | 14.417 | 41.338 | 1.00 | 32.86 | C |
| ATOM | 3602 | CE2 | TYR | B | 163 | 17.663 | 15.599 | 41.403 | 1.00 | 32.61 | C |
| ATOM | 3603 | CZ | TYR | B | 163 | 18.964 | 15.565 | 41.867 | 1.00 | 33.32 | C |
| ATOM | 3604 | OH | TYR | B | 163 | 19.715 | 16.717 | 41.904 | 1.00 | 34.28 | O |
| ATOM | 3605 | C | TYR | B | 163 | 15.199 | 12.680 | 43.498 | 1.00 | 33.27 | C |
| ATOM | 3606 | O | TYR | B | 163 | 15.590 | 13.383 | 44.429 | 1.00 | 34.99 | O |
| ATOM | 3607 | N | GLN | B | 164 | 14.036 | 12.873 | 42.882 | 1.00 | 31.89 | N |
| ATOM | 3608 | CA | GLN | B | 164 | 13.140 | 13.955 | 43.267 | 1.00 | 29.74 | C |
| ATOM | 3609 | CB | GLN | B | 164 | 12.022 | 14.111 | 42.233 | 1.00 | 31.18 | C |
| ATOM | 3610 | CG | GLN | B | 164 | 12.515 | 14.575 | 40.863 | 1.00 | 31.83 | C |
| ATOM | 3611 | CD | GLN | B | 164 | 11.387 | 14.708 | 39.856 | 1.00 | 32.03 | C |

| | | | | | | | | | | | |
|---|---|---|---|---|---|---|---|---|---|---|---|
| ATOM | 3612 | OE1 | GLN | B | 164 | 10.653 | 15.700 | 39.845 | 1.00 | 30.71 | O |
| ATOM | 3613 | NE2 | GLN | B | 164 | 11.236 | 13.696 | 39.011 | 1.00 | 31.09 | N |
| ATOM | 3614 | C | GLN | B | 164 | 12.546 | 13.742 | 44.654 | 1.00 | 29.43 | C |
| ATOM | 3615 | O | GLN | B | 164 | 12.275 | 14.703 | 45.367 | 1.00 | 28.30 | O |
| ATOM | 3616 | N | LEU | B | 165 | 12.334 | 12.485 | 45.030 | 1.00 | 28.64 | N |
| ATOM | 3617 | CA | LEU | B | 165 | 11.806 | 12.187 | 46.343 | 1.00 | 28.80 | C |
| ATOM | 3618 | CB | LEU | B | 165 | 11.396 | 10.719 | 46.452 | 1.00 | 28.87 | C |
| ATOM | 3619 | CG | LEU | B | 165 | 11.195 | 10.196 | 47.881 | 1.00 | 28.04 | C |
| ATOM | 3620 | CD1 | LEU | B | 165 | 10.170 | 11.039 | 48.606 | 1.00 | 28.39 | C |
| ATOM | 3621 | CD2 | LEU | B | 165 | 10.782 | 8.746 | 47.846 | 1.00 | 27.05 | C |
| ATOM | 3622 | C | LEU | B | 165 | 12.901 | 12.484 | 47.354 | 1.00 | 30.91 | C |
| ATOM | 3623 | O | LEU | B | 165 | 12.645 | 13.069 | 48.411 | 1.00 | 32.32 | O |
| ATOM | 3624 | N | PHE | B | 166 | 14.129 | 12.086 | 47.034 | 1.00 | 30.42 | N |
| ATOM | 3625 | CA | PHE | B | 166 | 15.240 | 12.332 | 47.953 | 1.00 | 31.30 | C |
| ATOM | 3626 | CB | PHE | B | 166 | 16.533 | 11.671 | 47.458 | 1.00 | 29.03 | C |
| ATOM | 3627 | CG | PHE | B | 166 | 16.650 | 10.225 | 47.834 | 1.00 | 27.18 | C |
| ATOM | 3628 | CD1 | PHE | B | 166 | 16.505 | 9.829 | 49.160 | 1.00 | 27.63 | C |
| ATOM | 3629 | CD2 | PHE | B | 166 | 16.882 | 9.254 | 46.877 | 1.00 | 24.81 | C |
| ATOM | 3630 | CE1 | PHE | B | 166 | 16.585 | 8.482 | 49.520 | 1.00 | 26.81 | C |
| ATOM | 3631 | CE2 | PHE | B | 166 | 16.963 | 7.905 | 47.232 | 1.00 | 24.96 | C |
| ATOM | 3632 | CZ | PHE | B | 166 | 16.813 | 7.522 | 48.556 | 1.00 | 24.59 | C |
| ATOM | 3633 | C | PHE | B | 166 | 15.463 | 13.820 | 48.150 | 1.00 | 32.85 | C |
| ATOM | 3634 | O | PHE | B | 166 | 15.793 | 14.270 | 49.244 | 1.00 | 34.75 | O |
| ATOM | 3635 | N | ARG | B | 167 | 15.265 | 14.593 | 47.093 | 1.00 | 32.26 | N |
| ATOM | 3636 | CA | ARG | B | 167 | 15.466 | 16.018 | 47.204 | 1.00 | 32.11 | C |
| ATOM | 3637 | CB | ARG | B | 167 | 15.463 | 16.657 | 45.817 | 1.00 | 31.46 | C |
| ATOM | 3638 | CG | ARG | B | 167 | 16.012 | 18.060 | 45.806 | 1.00 | 31.22 | C |
| ATOM | 3639 | CD | ARG | B | 167 | 16.077 | 18.601 | 44.411 | 1.00 | 30.49 | C |
| ATOM | 3640 | NE | ARG | B | 167 | 16.132 | 20.056 | 44.444 | 1.00 | 33.30 | N |
| ATOM | 3641 | CZ | ARG | B | 167 | 15.835 | 20.838 | 43.412 | 1.00 | 33.85 | C |
| ATOM | 3642 | NH1 | ARG | B | 167 | 15.465 | 20.306 | 42.253 | 1.00 | 31.87 | N |
| ATOM | 3643 | NH2 | ARG | B | 167 | 15.890 | 22.154 | 43.545 | 1.00 | 34.98 | N |
| ATOM | 3644 | C | ARG | B | 167 | 14.413 | 16.668 | 48.104 | 1.00 | 32.77 | C |
| ATOM | 3645 | O | ARG | B | 167 | 14.738 | 17.534 | 48.910 | 1.00 | 33.89 | O |
| ATOM | 3646 | N | SER | B | 168 | 13.158 | 16.247 | 47.979 | 1.00 | 32.58 | N |
| ATOM | 3647 | CA | SER | B | 168 | 12.098 | 16.817 | 48.798 | 1.00 | 31.53 | C |
| ATOM | 3648 | CB | SER | B | 168 | 10.728 | 16.281 | 48.374 | 1.00 | 31.16 | C |
| ATOM | 3649 | OG | SER | B | 168 | 10.506 | 14.970 | 48.859 | 1.00 | 30.18 | O |
| ATOM | 3650 | C | SER | B | 168 | 12.331 | 16.486 | 50.269 | 1.00 | 32.20 | C |
| ATOM | 3651 | O | SER | B | 168 | 12.120 | 17.323 | 51.149 | 1.00 | 33.15 | O |
| ATOM | 3652 | N | LEU | B | 169 | 12.756 | 15.257 | 50.535 | 1.00 | 31.86 | N |
| ATOM | 3653 | CA | LEU | B | 169 | 13.015 | 14.826 | 51.900 | 1.00 | 31.88 | C |
| ATOM | 3654 | CB | LEU | B | 169 | 13.359 | 13.327 | 51.915 | 1.00 | 29.46 | C |
| ATOM | 3655 | CG | LEU | B | 169 | 12.327 | 12.321 | 52.477 | 1.00 | 30.65 | C |
| ATOM | 3656 | CD1 | LEU | B | 169 | 10.892 | 12.845 | 52.417 | 1.00 | 30.11 | C |
| ATOM | 3657 | CD2 | LEU | B | 169 | 12.452 | 11.023 | 51.709 | 1.00 | 28.38 | C |
| ATOM | 3658 | C | LEU | B | 169 | 14.140 | 15.684 | 52.494 | 1.00 | 33.03 | C |
| ATOM | 3659 | O | LEU | B | 169 | 14.041 | 16.156 | 53.627 | 1.00 | 32.58 | O |
| ATOM | 3660 | N | ALA | B | 170 | 15.197 | 15.908 | 51.714 | 1.00 | 33.59 | N |
| ATOM | 3661 | CA | ALA | B | 170 | 16.310 | 16.744 | 52.151 | 1.00 | 34.04 | C |
| ATOM | 3662 | CB | ALA | B | 170 | 17.285 | 16.966 | 51.001 | 1.00 | 31.39 | C |
| ATOM | 3663 | C | ALA | B | 170 | 15.743 | 18.086 | 52.615 | 1.00 | 35.30 | C |
| ATOM | 3664 | O | ALA | B | 170 | 16.097 | 18.606 | 53.678 | 1.00 | 36.71 | O |
| ATOM | 3665 | N | TYR | B | 171 | 14.847 | 18.627 | 51.802 | 1.00 | 35.32 | N |
| ATOM | 3666 | CA | TYR | B | 171 | 14.204 | 19.901 | 52.079 | 1.00 | 36.13 | C |
| ATOM | 3667 | CB | TYR | B | 171 | 13.263 | 20.263 | 50.939 | 1.00 | 36.25 | C |
| ATOM | 3668 | CG | TYR | B | 171 | 12.577 | 21.581 | 51.148 | 1.00 | 35.40 | C |
| ATOM | 3669 | CD1 | TYR | B | 171 | 13.247 | 22.769 | 50.914 | 1.00 | 35.09 | C |
| ATOM | 3670 | CE1 | TYR | B | 171 | 12.634 | 23.988 | 51.119 | 1.00 | 35.47 | C |
| ATOM | 3671 | CD2 | TYR | B | 171 | 11.265 | 21.640 | 51.599 | 1.00 | 36.09 | C |
| ATOM | 3672 | CE2 | TYR | B | 171 | 10.639 | 22.860 | 51.811 | 1.00 | 36.98 | C |
| ATOM | 3673 | CZ | TYR | B | 171 | 11.333 | 24.032 | 51.564 | 1.00 | 36.06 | C |
| ATOM | 3674 | OH | TYR | B | 171 | 10.732 | 25.259 | 51.749 | 1.00 | 39.95 | O |
| ATOM | 3675 | C | TYR | B | 171 | 13.417 | 19.951 | 53.384 | 1.00 | 36.12 | C |
| ATOM | 3676 | O | TYR | B | 171 | 13.633 | 20.844 | 54.198 | 1.00 | 36.51 | O |
| ATOM | 3677 | N | ILE | B | 172 | 12.487 | 19.020 | 53.574 | 1.00 | 36.53 | N |
| ATOM | 3678 | CA | ILE | B | 172 | 11.697 | 19.020 | 54.797 | 1.00 | 38.33 | C |

| | | | | | | | | | | | |
|---|---|---|---|---|---|---|---|---|---|---|---|
| ATOM | 3679 | CB | ILE | B | 172 | 10.492 | 18.048 | 54.727 | 1.00 | 39.03 | C |
| ATOM | 3680 | CG2 | ILE | B | 172 | 9.426 | 18.601 | 53.795 | 1.00 | 38.78 | C |
| ATOM | 3681 | CG1 | ILE | B | 172 | 10.971 | 16.659 | 54.301 | 1.00 | 39.94 | C |
| ATOM | 3682 | CD1 | ILE | B | 172 | 9.951 | 15.574 | 54.539 | 1.00 | 41.21 | C |
| ATOM | 3683 | C | ILE | B | 172 | 12.544 | 18.637 | 56.000 | 1.00 | 39.11 | C |
| ATOM | 3684 | O | ILE | B | 172 | 12.275 | 19.077 | 57.119 | 1.00 | 40.33 | O |
| ATOM | 3685 | N | HIS | B | 173 | 13.560 | 17.809 | 55.774 | 1.00 | 39.03 | N |
| ATOM | 3686 | CA | HIS | B | 173 | 14.436 | 17.372 | 56.862 | 1.00 | 39.65 | C |
| ATOM | 3687 | CB | HIS | B | 173 | 15.287 | 16.186 | 56.416 | 1.00 | 37.64 | C |
| ATOM | 3688 | CG | HIS | B | 173 | 14.546 | 14.888 | 56.399 | 1.00 | 36.36 | C |
| ATOM | 3689 | CD2 | HIS | B | 173 | 13.256 | 14.583 | 56.695 | 1.00 | 35.02 | C |
| ATOM | 3690 | ND1 | HIS | B | 173 | 15.111 | 13.713 | 55.949 | 1.00 | 35.25 | N |
| ATOM | 3691 | CE1 | HIS | B | 173 | 14.211 | 12.756 | 55.957 | 1.00 | 35.10 | C |
| ATOM | 3692 | NE2 | HIS | B | 173 | 13.069 | 13.259 | 56.407 | 1.00 | 33.61 | N |
| ATOM | 3693 | C | HIS | B | 173 | 15.341 | 18.452 | 57.443 | 1.00 | 40.41 | C |
| ATOM | 3694 | O | HIS | B | 173 | 15.743 | 18.371 | 58.607 | 1.00 | 41.41 | O |
| ATOM | 3695 | N | SER | B | 174 | 15.652 | 19.466 | 56.643 | 1.00 | 41.02 | N |
| ATOM | 3696 | CA | SER | B | 174 | 16.517 | 20.551 | 57.095 | 1.00 | 41.62 | C |
| ATOM | 3697 | CB | SER | B | 174 | 17.091 | 21.296 | 55.902 | 1.00 | 41.30 | C |
| ATOM | 3698 | OG | SER | B | 174 | 16.079 | 22.035 | 55.256 | 1.00 | 42.27 | O |
| ATOM | 3699 | C | SER | B | 174 | 15.746 | 21.527 | 57.978 | 1.00 | 42.45 | C |
| ATOM | 3700 | O | SER | B | 174 | 16.332 | 22.435 | 58.567 | 1.00 | 42.40 | O |
| ATOM | 3701 | N | PHE | B | 175 | 14.430 | 21.341 | 58.045 | 1.00 | 43.64 | N |
| ATOM | 3702 | CA | PHE | B | 175 | 13.562 | 22.163 | 58.877 | 1.00 | 43.15 | C |
| ATOM | 3703 | CB | PHE | B | 175 | 12.214 | 22.400 | 58.202 | 1.00 | 46.80 | C |
| ATOM | 3704 | CG | PHE | B | 175 | 12.247 | 23.418 | 57.111 | 1.00 | 49.71 | C |
| ATOM | 3705 | CD1 | PHE | B | 175 | 13.153 | 23.318 | 56.065 | 1.00 | 51.75 | C |
| ATOM | 3706 | CD2 | PHE | B | 175 | 11.357 | 24.480 | 57.125 | 1.00 | 50.88 | C |
| ATOM | 3707 | CE1 | PHE | B | 175 | 13.171 | 24.269 | 55.047 | 1.00 | 52.67 | C |
| ATOM | 3708 | CE2 | PHE | B | 175 | 11.366 | 25.435 | 56.115 | 1.00 | 52.18 | C |
| ATOM | 3709 | CZ | PHE | B | 175 | 12.275 | 25.330 | 55.074 | 1.00 | 52.94 | C |
| ATOM | 3710 | C | PHE | B | 175 | 13.313 | 21.350 | 60.128 | 1.00 | 41.87 | C |
| ATOM | 3711 | O | PHE | B | 175 | 12.674 | 21.812 | 61.070 | 1.00 | 41.68 | O |
| ATOM | 3712 | N | GLY | B | 176 | 13.800 | 20.116 | 60.108 | 1.00 | 40.76 | N |
| ATOM | 3713 | CA | GLY | B | 176 | 13.621 | 19.227 | 61.237 | 1.00 | 41.42 | C |
| ATOM | 3714 | C | GLY | B | 176 | 12.311 | 18.494 | 61.093 | 1.00 | 41.51 | C |
| ATOM | 3715 | O | GLY | B | 176 | 11.857 | 17.808 | 62.007 | 1.00 | 41.99 | O |
| ATOM | 3716 | N | ILE | B | 177 | 11.700 | 18.646 | 59.922 | 1.00 | 41.25 | N |
| ATOM | 3717 | CA | ILE | B | 177 | 10.425 | 18.013 | 59.628 | 1.00 | 39.90 | C |
| ATOM | 3718 | CB | ILE | B | 177 | 9.579 | 18.905 | 58.711 | 1.00 | 39.84 | C |
| ATOM | 3719 | CG2 | ILE | B | 177 | 8.162 | 18.348 | 58.612 | 1.00 | 40.39 | C |
| ATOM | 3720 | CG1 | ILE | B | 177 | 9.527 | 20.319 | 59.289 | 1.00 | 40.90 | C |
| ATOM | 3721 | CD1 | ILE | B | 177 | 8.933 | 21.349 | 58.362 | 1.00 | 42.63 | C |
| ATOM | 3722 | C | ILE | B | 177 | 10.589 | 16.632 | 58.979 | 1.00 | 39.41 | C |
| ATOM | 3723 | O | ILE | B | 177 | 11.270 | 16.474 | 57.961 | 1.00 | 39.60 | O |
| ATOM | 3724 | N | CYS | B | 178 | 9.960 | 15.641 | 59.601 | 1.00 | 36.80 | N |
| ATOM | 3725 | CA | CYS | B | 178 | 9.976 | 14.270 | 59.139 | 1.00 | 35.33 | C |
| ATOM | 3726 | CB | CYS | B | 178 | 10.218 | 13.332 | 60.314 | 1.00 | 36.22 | C |
| ATOM | 3727 | SG | CYS | B | 178 | 10.433 | 11.585 | 59.854 | 1.00 | 36.39 | S |
| ATOM | 3728 | C | CYS | B | 178 | 8.593 | 14.017 | 58.559 | 1.00 | 35.61 | C |
| ATOM | 3729 | O | CYS | B | 178 | 7.604 | 14.534 | 59.077 | 1.00 | 35.16 | O |
| ATOM | 3730 | N | HIS | B | 179 | 8.524 | 13.236 | 57.483 | 1.00 | 34.49 | N |
| ATOM | 3731 | CA | HIS | B | 179 | 7.261 | 12.947 | 56.833 | 1.00 | 31.99 | C |
| ATOM | 3732 | CB | HIS | B | 179 | 7.504 | 12.561 | 55.374 | 1.00 | 33.02 | C |
| ATOM | 3733 | CG | HIS | B | 179 | 6.252 | 12.479 | 54.560 | 1.00 | 32.98 | C |
| ATOM | 3734 | CD2 | HIS | B | 179 | 5.721 | 13.338 | 53.651 | 1.00 | 32.90 | C |
| ATOM | 3735 | ND1 | HIS | B | 179 | 5.345 | 11.449 | 54.676 | 1.00 | 31.68 | N |
| ATOM | 3736 | CE1 | HIS | B | 179 | 4.317 | 11.672 | 53.883 | 1.00 | 33.57 | C |
| ATOM | 3737 | NE2 | HIS | B | 179 | 4.523 | 12.818 | 53.248 | 1.00 | 31.45 | N |
| ATOM | 3738 | C | HIS | B | 179 | 6.493 | 11.845 | 57.545 | 1.00 | 32.46 | C |
| ATOM | 3739 | O | HIS | B | 179 | 5.269 | 11.921 | 57.695 | 1.00 | 31.78 | O |
| ATOM | 3740 | N | ARG | B | 180 | 7.212 | 10.814 | 57.972 | 1.00 | 30.89 | N |
| ATOM | 3741 | CA | ARG | B | 180 | 6.604 | 9.701 | 58.680 | 1.00 | 29.48 | C |
| ATOM | 3742 | CB | ARG | B | 180 | 5.988 | 10.201 | 59.992 | 1.00 | 30.49 | C |
| ATOM | 3743 | CG | ARG | B | 180 | 7.030 | 10.734 | 60.963 | 1.00 | 29.61 | C |
| ATOM | 3744 | CD | ARG | B | 180 | 6.405 | 11.604 | 62.011 | 1.00 | 27.55 | C |
| ATOM | 3745 | NE | ARG | B | 180 | 5.596 | 10.848 | 62.946 | 1.00 | 27.80 | N |

| | | | | | | | | | | | |
|---|---|---|---|---|---|---|---|---|---|---|---|
| ATOM | 3746 | CZ | ARG | B | 180 | 4.621 | 11.375 | 63.681 | 1.00 | 28.01 | C |
| ATOM | 3747 | NH1 | ARG | B | 180 | 4.319 | 12.658 | 63.592 | 1.00 | 28.61 | N |
| ATOM | 3748 | NH2 | ARG | B | 180 | 3.947 | 10.620 | 64.525 | 1.00 | 30.23 | N |
| ATOM | 3749 | C | ARG | B | 180 | 5.565 | 8.920 | 57.879 | 1.00 | 29.33 | C |
| ATOM | 3750 | O | ARG | B | 180 | 4.861 | 8.087 | 58.442 | 1.00 | 29.49 | O |
| ATOM | 3751 | N | ASP | B | 181 | 5.442 | 9.185 | 56.583 | 1.00 | 29.79 | N |
| ATOM | 3752 | CA | ASP | B | 181 | 4.493 | 8.423 | 55.777 | 1.00 | 30.21 | C |
| ATOM | 3753 | CB | ASP | B | 181 | 3.071 | 8.954 | 55.929 | 1.00 | 28.26 | C |
| ATOM | 3754 | CG | ASP | B | 181 | 2.035 | 7.950 | 55.467 | 1.00 | 28.93 | C |
| ATOM | 3755 | OD1 | ASP | B | 181 | 2.314 | 6.735 | 55.541 | 1.00 | 28.26 | O |
| ATOM | 3756 | OD2 | ASP | B | 181 | 0.939 | 8.373 | 55.045 | 1.00 | 32.30 | O |
| ATOM | 3757 | C | ASP | B | 181 | 4.858 | 8.350 | 54.302 | 1.00 | 31.15 | C |
| ATOM | 3758 | O | ASP | B | 181 | 4.001 | 8.446 | 53.426 | 1.00 | 31.78 | O |
| ATOM | 3759 | N | ILE | B | 182 | 6.147 | 8.179 | 54.039 | 1.00 | 30.64 | N |
| ATOM | 3760 | CA | ILE | B | 182 | 6.625 | 8.072 | 52.680 | 1.00 | 31.03 | C |
| ATOM | 3761 | CB | ILE | B | 182 | 8.164 | 8.187 | 52.611 | 1.00 | 30.45 | C |
| ATOM | 3762 | CG2 | ILE | B | 182 | 8.658 | 7.883 | 51.201 | 1.00 | 28.18 | C |
| ATOM | 3763 | CG1 | ILE | B | 182 | 8.596 | 9.590 | 53.041 | 1.00 | 29.20 | C |
| ATOM | 3764 | CD1 | ILE | B | 182 | 8.025 | 10.695 | 52.180 | 1.00 | 28.72 | C |
| ATOM | 3765 | C | ILE | B | 182 | 6.197 | 6.714 | 52.147 | 1.00 | 32.08 | C |
| ATOM | 3766 | O | ILE | B | 182 | 6.524 | 5.673 | 52.725 | 1.00 | 31.82 | O |
| ATOM | 3767 | N | LYS | B | 183 | 5.429 | 6.747 | 51.062 | 1.00 | 30.93 | N |
| ATOM | 3768 | CA | LYS | B | 183 | 4.940 | 5.548 | 50.400 | 1.00 | 31.27 | C |
| ATOM | 3769 | CB | LYS | B | 183 | 3.754 | 4.951 | 51.163 | 1.00 | 29.78 | C |
| ATOM | 3770 | CG | LYS | B | 183 | 2.534 | 5.838 | 51.256 | 1.00 | 29.88 | C |
| ATOM | 3771 | CD | LYS | B | 183 | 1.445 | 5.144 | 52.029 | 1.00 | 29.06 | C |
| ATOM | 3772 | CE | LYS | B | 183 | 0.225 | 6.009 | 52.110 | 1.00 | 31.67 | C |
| ATOM | 3773 | NZ | LYS | B | 183 | -0.731 | 5.514 | 53.137 | 1.00 | 35.93 | N |
| ATOM | 3774 | C | LYS | B | 183 | 4.538 | 5.902 | 48.963 | 1.00 | 30.40 | C |
| ATOM | 3775 | O | LYS | B | 183 | 4.238 | 7.054 | 48.662 | 1.00 | 30.55 | O |
| ATOM | 3776 | N | PRO | B | 184 | 4.538 | 4.909 | 48.060 | 1.00 | 29.60 | N |
| ATOM | 3777 | CD | PRO | B | 184 | 4.812 | 3.490 | 48.369 | 1.00 | 28.82 | C |
| ATOM | 3778 | CA | PRO | B | 184 | 4.187 | 5.074 | 46.642 | 1.00 | 28.25 | C |
| ATOM | 3779 | CB | PRO | B | 184 | 4.044 | 3.631 | 46.156 | 1.00 | 28.44 | C |
| ATOM | 3780 | CG | PRO | B | 184 | 5.050 | 2.906 | 46.992 | 1.00 | 29.10 | C |
| ATOM | 3781 | C | PRO | B | 184 | 2.930 | 5.902 | 46.382 | 1.00 | 27.02 | C |
| ATOM | 3782 | O | PRO | B | 184 | 2.888 | 6.697 | 45.441 | 1.00 | 23.54 | O |
| ATOM | 3783 | N | GLN | B | 185 | 1.918 | 5.711 | 47.224 | 1.00 | 26.72 | N |
| ATOM | 3784 | CA | GLN | B | 185 | 0.656 | 6.428 | 47.090 | 1.00 | 27.61 | C |
| ATOM | 3785 | CB | GLN | B | 185 | -0.411 | 5.811 | 47.995 | 1.00 | 29.13 | C |
| ATOM | 3786 | CG | GLN | B | 185 | -0.701 | 4.353 | 47.689 | 1.00 | 31.35 | C |
| ATOM | 3787 | CD | GLN | B | 185 | 0.282 | 3.401 | 48.347 | 1.00 | 32.80 | C |
| ATOM | 3788 | OE1 | GLN | B | 185 | 1.403 | 3.774 | 48.692 | 1.00 | 31.24 | O |
| ATOM | 3789 | NE2 | GLN | B | 185 | -0.135 | 2.152 | 48.512 | 1.00 | 34.56 | N |
| ATOM | 3790 | C | GLN | B | 185 | 0.757 | 7.919 | 47.385 | 1.00 | 27.65 | C |
| ATOM | 3791 | O | GLN | B | 185 | -0.129 | 8.684 | 47.005 | 1.00 | 26.87 | O |
| ATOM | 3792 | N | ASN | B | 186 | 1.824 | 8.335 | 48.062 | 1.00 | 27.92 | N |
| ATOM | 3793 | CA | ASN | B | 186 | 1.996 | 9.746 | 48.390 | 1.00 | 28.48 | C |
| ATOM | 3794 | CB | ASN | B | 186 | 2.373 | 9.930 | 49.863 | 1.00 | 27.69 | C |
| ATOM | 3795 | CG | ASN | B | 186 | 1.228 | 9.616 | 50.798 | 1.00 | 27.82 | C |
| ATOM | 3796 | OD1 | ASN | B | 186 | 0.064 | 9.805 | 50.456 | 1.00 | 30.70 | O |
| ATOM | 3797 | ND2 | ASN | B | 186 | 1.553 | 9.144 | 51.993 | 1.00 | 30.30 | N |
| ATOM | 3798 | C | ASN | B | 186 | 3.027 | 10.427 | 47.514 | 1.00 | 29.09 | C |
| ATOM | 3799 | O | ASN | B | 186 | 3.583 | 11.455 | 47.885 | 1.00 | 28.79 | O |
| ATOM | 3800 | N | LEU | B | 187 | 3.280 | 9.850 | 46.347 | 1.00 | 29.56 | N |
| ATOM | 3801 | CA | LEU | B | 187 | 4.237 | 10.424 | 45.412 | 1.00 | 29.68 | C |
| ATOM | 3802 | CB | LEU | B | 187 | 5.368 | 9.424 | 45.138 | 1.00 | 27.87 | C |
| ATOM | 3803 | CG | LEU | B | 187 | 6.267 | 9.026 | 46.328 | 1.00 | 26.19 | C |
| ATOM | 3804 | CD1 | LEU | B | 187 | 7.176 | 7.865 | 45.944 | 1.00 | 24.99 | C |
| ATOM | 3805 | CD2 | LEU | B | 187 | 7.095 | 10.216 | 46.770 | 1.00 | 27.71 | C |
| ATOM | 3806 | C | LEU | B | 187 | 3.458 | 10.753 | 44.130 | 1.00 | 31.86 | C |
| ATOM | 3807 | O | LEU | B | 187 | 3.298 | 9.903 | 43.256 | 1.00 | 31.50 | O |
| ATOM | 3808 | N | LEU | B | 188 | 2.951 | 11.982 | 44.040 | 1.00 | 34.21 | N |
| ATOM | 3809 | CA | LEU | B | 188 | 2.182 | 12.414 | 42.875 | 1.00 | 37.20 | C |
| ATOM | 3810 | CB | LEU | B | 188 | 1.334 | 13.644 | 43.216 | 1.00 | 35.34 | C |
| ATOM | 3811 | CG | LEU | B | 188 | 0.551 | 13.646 | 44.528 | 1.00 | 37.23 | C |
| ATOM | 3812 | CD1 | LEU | B | 188 | -0.253 | 14.932 | 44.638 | 1.00 | 36.26 | C |

| | | | | | | | | | | | |
|---|---|---|---|---|---|---|---|---|---|---|---|
| ATOM | 3813 | CD2 | LEU | B | 188 | -0.364 | 12.434 | 44.599 | 1.00 | 36.48 | C |
| ATOM | 3814 | C | LEU | B | 188 | 3.118 | 12.775 | 41.728 | 1.00 | 39.25 | C |
| ATOM | 3815 | O | LEU | B | 188 | 4.215 | 13.283 | 41.958 | 1.00 | 40.41 | O |
| ATOM | 3816 | N | LEU | B | 189 | 2.697 | 12.520 | 40.492 | 1.00 | 41.45 | N |
| ATOM | 3817 | CA | LEU | B | 189 | 3.540 | 12.876 | 39.359 | 1.00 | 44.22 | C |
| ATOM | 3818 | CB | LEU | B | 189 | 4.540 | 11.751 | 39.050 | 1.00 | 44.60 | C |
| ATOM | 3819 | CG | LEU | B | 189 | 4.156 | 10.278 | 39.214 | 1.00 | 45.54 | C |
| ATOM | 3820 | CD1 | LEU | B | 189 | 3.133 | 9.901 | 38.162 | 1.00 | 46.77 | C |
| ATOM | 3821 | CD2 | LEU | B | 189 | 5.394 | 9.395 | 39.067 | 1.00 | 44.61 | C |
| ATOM | 3822 | C | LEU | B | 189 | 2.795 | 13.286 | 38.095 | 1.00 | 43.44 | C |
| ATOM | 3823 | O | LEU | B | 189 | 1.656 | 12.897 | 37.867 | 1.00 | 42.56 | O |
| ATOM | 3824 | N | ASP | B | 190 | 3.463 | 14.114 | 37.305 | 1.00 | 44.57 | N |
| ATOM | 3825 | CA | ASP | B | 190 | 2.948 | 14.621 | 36.047 | 1.00 | 46.24 | C |
| ATOM | 3826 | CB | ASP | B | 190 | 3.429 | 16.059 | 35.864 | 1.00 | 47.10 | C |
| ATOM | 3827 | CG | ASP | B | 190 | 2.801 | 16.747 | 34.670 | 1.00 | 46.91 | C |
| ATOM | 3828 | OD1 | ASP | B | 190 | 2.792 | 16.149 | 33.578 | 1.00 | 48.39 | O |
| ATOM | 3829 | OD2 | ASP | B | 190 | 2.335 | 17.892 | 34.818 | 1.00 | 47.31 | O |
| ATOM | 3830 | C | ASP | B | 190 | 3.551 | 13.713 | 34.975 | 1.00 | 47.43 | C |
| ATOM | 3831 | O | ASP | B | 190 | 4.740 | 13.817 | 34.654 | 1.00 | 47.41 | O |
| ATOM | 3832 | N | PRO | B | 191 | 2.736 | 12.810 | 34.409 | 1.00 | 48.54 | N |
| ATOM | 3833 | CD | PRO | B | 191 | 1.267 | 12.790 | 34.537 | 1.00 | 48.45 | C |
| ATOM | 3834 | CA | PRO | B | 191 | 3.173 | 11.867 | 33.374 | 1.00 | 48.88 | C |
| ATOM | 3835 | CB | PRO | B | 191 | 1.864 | 11.204 | 32.945 | 1.00 | 49.67 | C |
| ATOM | 3836 | CG | PRO | B | 191 | 0.847 | 12.284 | 33.184 | 1.00 | 49.16 | C |
| ATOM | 3837 | C | PRO | B | 191 | 3.950 | 12.435 | 32.183 | 1.00 | 48.53 | C |
| ATOM | 3838 | O | PRO | B | 191 | 4.760 | 11.722 | 31.592 | 1.00 | 49.39 | O |
| ATOM | 3839 | N | ASP | B | 192 | 3.710 | 13.696 | 31.822 | 1.00 | 47.29 | N |
| ATOM | 3840 | CA | ASP | B | 192 | 4.412 | 14.297 | 30.683 | 1.00 | 46.53 | C |
| ATOM | 3841 | CB | ASP | B | 192 | 3.517 | 15.306 | 29.947 | 1.00 | 48.12 | C |
| ATOM | 3842 | CG | ASP | B | 192 | 2.150 | 14.739 | 29.589 | 1.00 | 49.86 | C |
| ATOM | 3843 | OD1 | ASP | B | 192 | 2.063 | 13.584 | 29.121 | 1.00 | 50.76 | O |
| ATOM | 3844 | OD2 | ASP | B | 192 | 1.153 | 15.467 | 29.767 | 1.00 | 50.88 | O |
| ATOM | 3845 | C | ASP | B | 192 | 5.711 | 15.002 | 31.062 | 1.00 | 44.86 | C |
| ATOM | 3846 | O | ASP | B | 192 | 6.666 | 15.004 | 30.288 | 1.00 | 44.66 | O |
| ATOM | 3847 | N | THR | B | 193 | 5.750 | 15.607 | 32.244 | 1.00 | 42.90 | N |
| ATOM | 3848 | CA | THR | B | 193 | 6.951 | 16.315 | 32.683 | 1.00 | 41.22 | C |
| ATOM | 3849 | CB | THR | B | 193 | 6.594 | 17.618 | 33.397 | 1.00 | 41.63 | C |
| ATOM | 3850 | OG1 | THR | B | 193 | 5.570 | 17.356 | 34.361 | 1.00 | 42.11 | O |
| ATOM | 3851 | CG2 | THR | B | 193 | 6.103 | 18.659 | 32.406 | 1.00 | 41.00 | C |
| ATOM | 3852 | C | THR | B | 193 | 7.808 | 15.477 | 33.617 | 1.00 | 39.24 | C |
| ATOM | 3853 | O | THR | B | 193 | 8.991 | 15.760 | 33.808 | 1.00 | 37.94 | O |
| ATOM | 3854 | N | ALA | B | 194 | 7.196 | 14.444 | 34.185 | 1.00 | 38.24 | N |
| ATOM | 3855 | CA | ALA | B | 194 | 7.868 | 13.538 | 35.107 | 1.00 | 37.80 | C |
| ATOM | 3856 | CB | ALA | B | 194 | 9.075 | 12.905 | 34.431 | 1.00 | 36.54 | C |
| ATOM | 3857 | C | ALA | B | 194 | 8.286 | 14.186 | 36.434 | 1.00 | 37.46 | C |
| ATOM | 3858 | O | ALA | B | 194 | 9.117 | 13.637 | 37.150 | 1.00 | 36.81 | O |
| ATOM | 3859 | N | VAL | B | 195 | 7.721 | 15.344 | 36.770 | 1.00 | 36.68 | N |
| ATOM | 3860 | CA | VAL | B | 195 | 8.076 | 15.957 | 38.037 | 1.00 | 36.34 | C |
| ATOM | 3861 | CB | VAL | B | 195 | 7.763 | 17.479 | 38.091 | 1.00 | 37.21 | C |
| ATOM | 3862 | CG1 | VAL | B | 195 | 8.381 | 18.179 | 36.893 | 1.00 | 36.00 | C |
| ATOM | 3863 | CG2 | VAL | B | 195 | 6.268 | 17.715 | 38.135 | 1.00 | 40.50 | C |
| ATOM | 3864 | C | VAL | B | 195 | 7.271 | 15.229 | 39.103 | 1.00 | 35.96 | C |
| ATOM | 3865 | O | VAL | B | 195 | 6.102 | 14.896 | 38.900 | 1.00 | 35.58 | O |
| ATOM | 3866 | N | LEU | B | 196 | 7.916 | 14.956 | 40.229 | 1.00 | 34.80 | N |
| ATOM | 3867 | CA | LEU | B | 196 | 7.266 | 14.262 | 41.324 | 1.00 | 32.52 | C |
| ATOM | 3868 | CB | LEU | B | 196 | 8.158 | 13.107 | 41.793 | 1.00 | 32.64 | C |
| ATOM | 3869 | CG | LEU | B | 196 | 7.768 | 12.207 | 42.968 | 1.00 | 31.29 | C |
| ATOM | 3870 | CD1 | LEU | B | 196 | 8.274 | 10.804 | 42.691 | 1.00 | 30.16 | C |
| ATOM | 3871 | CD2 | LEU | B | 196 | 8.345 | 12.755 | 44.266 | 1.00 | 29.95 | C |
| ATOM | 3872 | C | LEU | B | 196 | 7.020 | 15.256 | 42.450 | 1.00 | 31.95 | C |
| ATOM | 3873 | O | LEU | B | 196 | 7.729 | 16.254 | 42.564 | 1.00 | 31.04 | O |
| ATOM | 3874 | N | LYS | B | 197 | 5.998 | 14.992 | 43.258 | 1.00 | 31.41 | N |
| ATOM | 3875 | CA | LYS | B | 197 | 5.653 | 15.851 | 44.389 | 1.00 | 31.14 | C |
| ATOM | 3876 | CB | LYS | B | 197 | 4.484 | 16.790 | 44.062 | 1.00 | 33.36 | C |
| ATOM | 3877 | CG | LYS | B | 197 | 4.801 | 17.856 | 43.044 | 1.00 | 35.77 | C |
| ATOM | 3878 | CD | LYS | B | 197 | 3.544 | 18.546 | 42.594 | 1.00 | 38.96 | C |
| ATOM | 3879 | CE | LYS | B | 197 | 3.827 | 19.414 | 41.384 | 1.00 | 40.59 | C |

| | | | | | | | | | | | |
|---|---|---|---|---|---|---|---|---|---|---|---|
| ATOM | 3880 | NZ | LYS | B | 197 | 5.012 | 20.281 | 41.659 | 1.00 | 42.20 | N |
| ATOM | 3881 | C | LYS | B | 197 | 5.263 | 15.003 | 45.583 | 1.00 | 30.43 | C |
| ATOM | 3882 | O | LYS | B | 197 | 4.428 | 14.106 | 45.475 | 1.00 | 29.19 | O |
| ATOM | 3883 | N | LEU | B | 198 | 5.890 | 15.290 | 46.720 | 1.00 | 30.73 | N |
| ATOM | 3884 | CA | LEU | B | 198 | 5.608 | 14.587 | 47.956 | 1.00 | 28.51 | C |
| ATOM | 3885 | CB | LEU | B | 198 | 6.695 | 14.879 | 48.989 | 1.00 | 29.45 | C |
| ATOM | 3886 | CG | LEU | B | 198 | 7.156 | 13.733 | 49.894 | 1.00 | 30.75 | C |
| ATOM | 3887 | CD1 | LEU | B | 198 | 7.822 | 14.304 | 51.140 | 1.00 | 29.31 | C |
| ATOM | 3888 | CD2 | LEU | B | 198 | 5.990 | 12.861 | 50.277 | 1.00 | 30.67 | C |
| ATOM | 3889 | C | LEU | B | 198 | 4.294 | 15.202 | 48.410 | 1.00 | 28.41 | C |
| ATOM | 3890 | O | LEU | B | 198 | 4.111 | 16.414 | 48.304 | 1.00 | 28.79 | O |
| ATOM | 3891 | N | CYS | B | 199 | 3.375 | 14.379 | 48.888 | 1.00 | 26.89 | N |
| ATOM | 3892 | CA | CYS | B | 199 | 2.101 | 14.895 | 49.340 | 1.00 | 27.75 | C |
| ATOM | 3893 | CB | CYS | B | 199 | 1.029 | 14.728 | 48.252 | 1.00 | 27.11 | C |
| ATOM | 3894 | SG | CYS | B | 199 | 0.185 | 13.100 | 48.234 | 1.00 | 29.08 | S |
| ATOM | 3895 | C | CYS | B | 199 | 1.650 | 14.183 | 50.614 | 1.00 | 27.32 | C |
| ATOM | 3896 | O | CYS | B | 199 | 2.217 | 13.164 | 51.013 | 1.00 | 25.49 | O |
| ATOM | 3897 | N | ASP | B | 200 | 0.617 | 14.736 | 51.240 | 1.00 | 28.66 | N |
| ATOM | 3898 | CA | ASP | B | 200 | 0.053 | 14.180 | 52.456 | 1.00 | 30.67 | C |
| ATOM | 3899 | CB | ASP | B | 200 | -0.319 | 12.716 | 52.250 | 1.00 | 29.76 | C |
| ATOM | 3900 | CG | ASP | B | 200 | -1.186 | 12.191 | 53.365 | 1.00 | 32.01 | C |
| ATOM | 3901 | OD1 | ASP | B | 200 | -1.390 | 12.913 | 54.359 | 1.00 | 31.56 | O |
| ATOM | 3902 | OD2 | ASP | B | 200 | -1.671 | 11.055 | 53.251 | 1.00 | 35.06 | O |
| ATOM | 3903 | C | ASP | B | 200 | 0.948 | 14.302 | 53.681 | 1.00 | 32.10 | C |
| ATOM | 3904 | O | ASP | B | 200 | 1.580 | 13.336 | 54.112 | 1.00 | 34.06 | O |
| ATOM | 3905 | N | PHE | B | 201 | 1.001 | 15.500 | 54.241 | 1.00 | 31.07 | N |
| ATOM | 3906 | CA | PHE | B | 201 | 1.800 | 15.725 | 55.424 | 1.00 | 30.86 | C |
| ATOM | 3907 | CB | PHE | B | 201 | 2.443 | 17.118 | 55.371 | 1.00 | 29.48 | C |
| ATOM | 3908 | CG | PHE | B | 201 | 3.571 | 17.229 | 54.386 | 1.00 | 29.07 | C |
| ATOM | 3909 | CD1 | PHE | B | 201 | 3.324 | 17.308 | 53.022 | 1.00 | 29.36 | C |
| ATOM | 3910 | CD2 | PHE | B | 201 | 4.889 | 17.230 | 54.825 | 1.00 | 27.33 | C |
| ATOM | 3911 | CE1 | PHE | B | 201 | 4.380 | 17.384 | 52.112 | 1.00 | 30.37 | C |
| ATOM | 3912 | CE2 | PHE | B | 201 | 5.949 | 17.309 | 53.922 | 1.00 | 27.65 | C |
| ATOM | 3913 | CZ | PHE | B | 201 | 5.696 | 17.385 | 52.567 | 1.00 | 27.74 | C |
| ATOM | 3914 | C | PHE | B | 201 | 0.949 | 15.562 | 56.683 | 1.00 | 31.26 | C |
| ATOM | 3915 | O | PHE | B | 201 | 1.213 | 16.203 | 57.690 | 1.00 | 33.07 | O |
| ATOM | 3916 | N | GLY | B | 202 | -0.060 | 14.693 | 56.620 | 1.00 | 31.71 | N |
| ATOM | 3917 | CA | GLY | B | 202 | -0.931 | 14.466 | 57.759 | 1.00 | 30.89 | C |
| ATOM | 3918 | C | GLY | B | 202 | -0.370 | 13.555 | 58.847 | 1.00 | 32.02 | C |
| ATOM | 3919 | O | GLY | B | 202 | -1.099 | 13.151 | 59.751 | 1.00 | 32.57 | O |
| ATOM | 3920 | N | SER | B | 203 | 0.916 | 13.221 | 58.758 | 1.00 | 31.89 | N |
| ATOM | 3921 | CA | SER | B | 203 | 1.592 | 12.373 | 59.740 | 1.00 | 30.99 | C |
| ATOM | 3922 | CB | SER | B | 203 | 1.842 | 10.962 | 59.191 | 1.00 | 30.73 | C |
| ATOM | 3923 | OG | SER | B | 203 | 0.650 | 10.265 | 58.942 | 1.00 | 31.46 | O |
| ATOM | 3924 | C | SER | B | 203 | 2.946 | 13.004 | 60.028 | 1.00 | 31.07 | C |
| ATOM | 3925 | O | SER | B | 203 | 3.721 | 12.494 | 60.828 | 1.00 | 32.54 | O |
| ATOM | 3926 | N | ALA | B | 204 | 3.231 | 14.100 | 59.339 | 1.00 | 30.75 | N |
| ATOM | 3927 | CA | ALA | B | 204 | 4.490 | 14.803 | 59.490 | 1.00 | 31.61 | C |
| ATOM | 3928 | CB | ALA | B | 204 | 4.641 | 15.821 | 58.379 | 1.00 | 31.56 | C |
| ATOM | 3929 | C | ALA | B | 204 | 4.568 | 15.494 | 60.842 | 1.00 | 33.59 | C |
| ATOM | 3930 | O | ALA | B | 204 | 3.553 | 15.912 | 61.401 | 1.00 | 33.43 | O |
| ATOM | 3931 | N | LYS | B | 205 | 5.779 | 15.613 | 61.362 | 1.00 | 35.62 | N |
| ATOM | 3932 | CA | LYS | B | 205 | 5.989 | 16.254 | 62.645 | 1.00 | 35.89 | C |
| ATOM | 3933 | CB | LYS | B | 205 | 5.730 | 15.262 | 63.778 | 1.00 | 33.37 | C |
| ATOM | 3934 | CG | LYS | B | 205 | 5.946 | 15.831 | 65.178 | 1.00 | 31.25 | C |
| ATOM | 3935 | CD | LYS | B | 205 | 5.902 | 14.722 | 66.220 | 1.00 | 30.83 | C |
| ATOM | 3936 | CE | LYS | B | 205 | 5.847 | 15.273 | 67.632 | 1.00 | 30.18 | C |
| ATOM | 3937 | NZ | LYS | B | 205 | 5.747 | 14.182 | 68.633 | 1.00 | 28.26 | N |
| ATOM | 3938 | C | LYS | B | 205 | 7.418 | 16.746 | 62.735 | 1.00 | 38.14 | C |
| ATOM | 3939 | O | LYS | B | 205 | 8.326 | 16.184 | 62.115 | 1.00 | 37.77 | O |
| ATOM | 3940 | N | GLN | B | 206 | 7.618 | 17.799 | 63.515 | 1.00 | 40.88 | N |
| ATOM | 3941 | CA | GLN | B | 206 | 8.956 | 18.329 | 63.706 | 1.00 | 43.44 | C |
| ATOM | 3942 | CB | GLN | B | 206 | 8.901 | 19.824 | 63.986 | 1.00 | 46.32 | C |
| ATOM | 3943 | CG | GLN | B | 206 | 10.248 | 20.505 | 63.956 | 1.00 | 50.97 | C |
| ATOM | 3944 | CD | GLN | B | 206 | 10.130 | 21.929 | 63.433 | 1.00 | 55.45 | C |
| ATOM | 3945 | OE1 | GLN | B | 206 | 11.046 | 22.754 | 63.601 | 1.00 | 57.25 | O |
| ATOM | 3946 | NE2 | GLN | B | 206 | 8.990 | 22.230 | 62.786 | 1.00 | 56.48 | N |

| | | | | | | | | | | | |
|---|---|---|---|---|---|---|---|---|---|---|---|
| ATOM | 3947 | C | GLN | B | 206 | 9.486 | 17.575 | 64.915 | 1.00 | 43.47 | C |
| ATOM | 3948 | O | GLN | B | 206 | 8.892 | 17.614 | 65.984 | 1.00 | 43.08 | O |
| ATOM | 3949 | N | LEU | B | 207 | 10.584 | 16.862 | 64.737 | 1.00 | 44.37 | N |
| ATOM | 3950 | CA | LEU | B | 207 | 11.138 | 16.094 | 65.831 | 1.00 | 45.94 | C |
| ATOM | 3951 | CB | LEU | B | 207 | 11.855 | 14.856 | 65.275 | 1.00 | 44.42 | C |
| ATOM | 3952 | CG | LEU | B | 207 | 11.052 | 13.938 | 64.342 | 1.00 | 42.83 | C |
| ATOM | 3953 | CD1 | LEU | B | 207 | 11.980 | 12.921 | 63.722 | 1.00 | 42.12 | C |
| ATOM | 3954 | CD2 | LEU | B | 207 | 9.912 | 13.256 | 65.092 | 1.00 | 40.96 | C |
| ATOM | 3955 | C | LEU | B | 207 | 12.091 | 16.917 | 66.693 | 1.00 | 47.75 | C |
| ATOM | 3956 | O | LEU | B | 207 | 13.025 | 17.544 | 66.191 | 1.00 | 47.70 | O |
| ATOM | 3957 | N | VAL | B | 208 | 11.828 | 16.915 | 67.995 | 1.00 | 50.18 | N |
| ATOM | 3958 | CA | VAL | B | 208 | 12.645 | 17.612 | 68.979 | 1.00 | 51.62 | C |
| ATOM | 3959 | CB | VAL | B | 208 | 11.781 | 18.525 | 69.873 | 1.00 | 52.47 | C |
| ATOM | 3960 | CG1 | VAL | B | 208 | 12.463 | 18.739 | 71.227 | 1.00 | 52.68 | C |
| ATOM | 3961 | CG2 | VAL | B | 208 | 11.557 | 19.862 | 69.186 | 1.00 | 53.81 | C |
| ATOM | 3962 | C | VAL | B | 208 | 13.235 | 16.518 | 69.846 | 1.00 | 52.83 | C |
| ATOM | 3963 | O | VAL | B | 208 | 12.497 | 15.856 | 70.583 | 1.00 | 53.03 | O |
| ATOM | 3964 | N | ARG | B | 209 | 14.549 | 16.314 | 69.778 | 1.00 | 54.45 | N |
| ATOM | 3965 | CA | ARG | B | 209 | 15.150 | 15.262 | 70.597 | 1.00 | 56.56 | C |
| ATOM | 3966 | CB | ARG | B | 209 | 16.667 | 15.222 | 70.447 | 1.00 | 57.51 | C |
| ATOM | 3967 | CG | ARG | B | 209 | 17.211 | 13.843 | 70.805 | 1.00 | 61.77 | C |
| ATOM | 3968 | CD | ARG | B | 209 | 18.630 | 13.911 | 71.366 | 1.00 | 64.55 | C |
| ATOM | 3969 | NE | ARG | B | 209 | 19.447 | 14.882 | 70.639 | 1.00 | 67.32 | N |
| ATOM | 3970 | CZ | ARG | B | 209 | 20.633 | 15.321 | 71.048 | 1.00 | 68.56 | C |
| ATOM | 3971 | NH1 | ARG | B | 209 | 21.155 | 14.872 | 72.192 | 1.00 | 67.51 | N |
| ATOM | 3972 | NH2 | ARG | B | 209 | 21.283 | 16.220 | 70.310 | 1.00 | 67.94 | N |
| ATOM | 3973 | C | ARG | B | 209 | 14.786 | 15.473 | 72.065 | 1.00 | 56.64 | C |
| ATOM | 3974 | O | ARG | B | 209 | 14.847 | 16.590 | 72.579 | 1.00 | 56.87 | O |
| ATOM | 3975 | N | GLY | B | 210 | 14.410 | 14.394 | 72.735 | 1.00 | 56.29 | N |
| ATOM | 3976 | CA | GLY | B | 210 | 14.024 | 14.506 | 74.126 | 1.00 | 57.27 | C |
| ATOM | 3977 | C | GLY | B | 210 | 12.532 | 14.293 | 74.262 | 1.00 | 57.69 | C |
| ATOM | 3978 | O | GLY | B | 210 | 12.069 | 13.605 | 75.172 | 1.00 | 58.07 | O |
| ATOM | 3979 | N | GLU | B | 211 | 11.772 | 14.892 | 73.352 | 1.00 | 57.51 | N |
| ATOM | 3980 | CA | GLU | B | 211 | 10.321 | 14.742 | 73.351 | 1.00 | 56.80 | C |
| ATOM | 3981 | CB | GLU | B | 211 | 9.704 | 15.980 | 72.697 | 1.00 | 57.65 | C |
| ATOM | 3982 | CG | GLU | B | 211 | 10.096 | 17.255 | 73.466 | 1.00 | 59.89 | C |
| ATOM | 3983 | CD | GLU | B | 211 | 9.611 | 18.558 | 72.836 | 1.00 | 61.38 | C |
| ATOM | 3984 | OE1 | GLU | B | 211 | 9.663 | 19.595 | 73.545 | 1.00 | 61.54 | O |
| ATOM | 3985 | OE2 | GLU | B | 211 | 9.197 | 18.552 | 71.650 | 1.00 | 61.07 | O |
| ATOM | 3986 | C | GLU | B | 211 | 10.098 | 13.468 | 72.542 | 1.00 | 55.75 | C |
| ATOM | 3987 | O | GLU | B | 211 | 10.618 | 13.337 | 71.435 | 1.00 | 56.49 | O |
| ATOM | 3988 | N | PRO | B | 212 | 9.358 | 12.496 | 73.096 | 1.00 | 54.66 | N |
| ATOM | 3989 | CD | PRO | B | 212 | 8.617 | 12.573 | 74.368 | 1.00 | 54.69 | C |
| ATOM | 3990 | CA | PRO | B | 212 | 9.085 | 11.218 | 72.425 | 1.00 | 53.22 | C |
| ATOM | 3991 | CB | PRO | B | 212 | 8.648 | 10.327 | 73.572 | 1.00 | 53.29 | C |
| ATOM | 3992 | CG | PRO | B | 212 | 7.798 | 11.274 | 74.361 | 1.00 | 53.72 | C |
| ATOM | 3993 | C | PRO | B | 212 | 8.034 | 11.290 | 71.341 | 1.00 | 51.00 | C |
| ATOM | 3994 | O | PRO | B | 212 | 7.274 | 12.257 | 71.267 | 1.00 | 51.44 | O |
| ATOM | 3995 | N | ASN | B | 213 | 7.999 | 10.248 | 70.515 | 1.00 | 47.75 | N |
| ATOM | 3996 | CA | ASN | B | 213 | 7.055 | 10.150 | 69.404 | 1.00 | 45.76 | C |
| ATOM | 3997 | CB | ASN | B | 213 | 7.755 | 10.409 | 68.081 | 1.00 | 43.67 | C |
| ATOM | 3998 | CG | ASN | B | 213 | 8.505 | 11.695 | 68.076 | 1.00 | 43.25 | C |
| ATOM | 3999 | OD1 | ASN | B | 213 | 7.912 | 12.771 | 68.080 | 1.00 | 45.10 | O |
| ATOM | 4000 | ND2 | ASN | B | 213 | 9.825 | 11.604 | 68.087 | 1.00 | 42.12 | N |
| ATOM | 4001 | C | ASN | B | 213 | 6.471 | 8.765 | 69.345 | 1.00 | 44.92 | C |
| ATOM | 4002 | O | ASN | B | 213 | 7.056 | 7.814 | 69.854 | 1.00 | 46.19 | O |
| ATOM | 4003 | N | VAL | B | 214 | 5.319 | 8.654 | 68.702 | 1.00 | 42.84 | N |
| ATOM | 4004 | CA | VAL | B | 214 | 4.652 | 7.374 | 68.538 | 1.00 | 42.31 | C |
| ATOM | 4005 | CB | VAL | B | 214 | 3.251 | 7.574 | 67.929 | 1.00 | 43.26 | C |
| ATOM | 4006 | CG1 | VAL | B | 214 | 2.251 | 7.833 | 69.025 | 1.00 | 41.87 | C |
| ATOM | 4007 | CG2 | VAL | B | 214 | 3.269 | 8.763 | 66.972 | 1.00 | 44.83 | C |
| ATOM | 4008 | C | VAL | B | 214 | 5.497 | 6.491 | 67.616 | 1.00 | 41.81 | C |
| ATOM | 4009 | O | VAL | B | 214 | 6.090 | 6.972 | 66.647 | 1.00 | 40.12 | O |
| ATOM | 4010 | N | SER | B | 215 | 5.561 | 5.201 | 67.926 | 1.00 | 40.29 | N |
| ATOM | 4011 | CA | SER | B | 215 | 6.357 | 4.281 | 67.123 | 1.00 | 40.07 | C |
| ATOM | 4012 | CB | SER | B | 215 | 7.029 | 3.242 | 68.030 | 1.00 | 39.62 | C |
| ATOM | 4013 | OG | SER | B | 215 | 6.058 | 2.522 | 68.760 | 1.00 | 41.37 | O |

| | | | | | | | | | | | |
|---|---|---|---|---|---|---|---|---|---|---|---|
| ATOM | 4014 | C | SER | B | 215 | 5.527 | 3.585 | 66.044 | 1.00 | 38.34 | C |
| ATOM | 4015 | O | SER | B | 215 | 6.045 | 3.244 | 64.976 | 1.00 | 38.04 | O |
| ATOM | 4016 | N | PTY | B | 216 | 4.248 | 3.367 | 66.331 | 1.00 | 36.80 | N |
| ATOM | 4017 | CA | PTY | B | 216 | 3.364 | 2.737 | 65.363 | 1.00 | 34.29 | C |
| ATOM | 4018 | C | PTY | B | 216 | 2.896 | 3.769 | 64.343 | 1.00 | 34.49 | C |
| ATOM | 4019 | O | PTY | B | 216 | 1.773 | 4.263 | 64.434 | 1.00 | 34.25 | O |
| ATOM | 4020 | CB | PTY | B | 216 | 2.153 | 2.132 | 66.065 | 1.00 | 33.76 | C |
| ATOM | 4021 | CG | PTY | B | 216 | 1.432 | 1.188 | 65.105 | 1.00 | 34.44 | C |
| ATOM | 4022 | CD1 | PTY | B | 216 | 1.904 | -0.208 | 64.957 | 1.00 | 32.17 | C |
| ATOM | 4023 | CD2 | PTY | B | 216 | 0.278 | 1.635 | 64.268 | 1.00 | 33.51 | C |
| ATOM | 4024 | CE1 | PTY | B | 216 | 1.242 | -1.120 | 64.003 | 1.00 | 32.64 | C |
| ATOM | 4025 | CE2 | PTY | B | 216 | -0.365 | 0.711 | 63.321 | 1.00 | 32.60 | C |
| ATOM | 4026 | CZ | PTY | B | 216 | 0.106 | -0.665 | 63.182 | 1.00 | 33.14 | C |
| ATOM | 4027 | OH | PTY | B | 216 | -0.635 | -1.552 | 62.446 | 1.00 | 34.51 | O |
| ATOM | 4028 | P | PTY | B | 216 | -0.397 | -1.776 | 61.004 | 1.00 | 35.66 | P |
| ATOM | 4029 | OP1 | PTY | B | 216 | 0.678 | -2.725 | 60.967 | 1.00 | 34.26 | O |
| ATOM | 4030 | OP2 | PTY | B | 216 | -0.012 | -0.504 | 60.335 | 1.00 | 33.28 | O |
| ATOM | 4031 | OP3 | PTY | B | 216 | -1.700 | -2.313 | 60.485 | 1.00 | 35.06 | O |
| ATOM | 4032 | N | ILE | B | 217 | 3.772 | 4.121 | 63.408 | 1.00 | 33.84 | N |
| ATOM | 4033 | CA | ILE | B | 217 | 3.436 | 5.080 | 62.373 | 1.00 | 34.08 | C |
| ATOM | 4034 | CB | ILE | B | 217 | 3.941 | 6.507 | 62.679 | 1.00 | 36.01 | C |
| ATOM | 4035 | CG2 | ILE | B | 217 | 3.155 | 7.110 | 63.814 | 1.00 | 38.40 | C |
| ATOM | 4036 | CG1 | ILE | B | 217 | 5.445 | 6.488 | 62.945 | 1.00 | 36.25 | C |
| ATOM | 4037 | CD1 | ILE | B | 217 | 6.076 | 7.837 | 62.691 | 1.00 | 39.01 | C |
| ATOM | 4038 | C | ILE | B | 217 | 4.108 | 4.613 | 61.112 | 1.00 | 33.69 | C |
| ATOM | 4039 | O | ILE | B | 217 | 4.896 | 3.673 | 61.148 | 1.00 | 32.89 | O |
| ATOM | 4040 | N | CYS | B | 218 | 3.807 | 5.293 | 60.012 | 1.00 | 33.21 | N |
| ATOM | 4041 | CA | CYS | B | 218 | 4.346 | 4.961 | 58.698 | 1.00 | 33.77 | C |
| ATOM | 4042 | CB | CYS | B | 218 | 5.797 | 4.500 | 58.773 | 1.00 | 35.62 | C |
| ATOM | 4043 | SG | CYS | B | 218 | 7.005 | 5.774 | 58.807 | 1.00 | 42.50 | S |
| ATOM | 4044 | C | CYS | B | 218 | 3.534 | 3.790 | 58.194 | 1.00 | 31.98 | C |
| ATOM | 4045 | O | CYS | B | 218 | 2.967 | 3.028 | 58.975 | 1.00 | 30.07 | O |
| ATOM | 4046 | N | SER | B | 219 | 3.486 | 3.646 | 56.882 | 1.00 | 32.10 | N |
| ATOM | 4047 | CA | SER | B | 219 | 2.767 | 2.544 | 56.295 | 1.00 | 30.84 | C |
| ATOM | 4048 | CB | SER | B | 219 | 2.454 | 2.852 | 54.842 | 1.00 | 32.94 | C |
| ATOM | 4049 | OG | SER | B | 219 | 1.141 | 2.413 | 54.570 | 1.00 | 36.19 | O |
| ATOM | 4050 | C | SER | B | 219 | 3.606 | 1.276 | 56.449 | 1.00 | 30.10 | C |
| ATOM | 4051 | O | SER | B | 219 | 4.739 | 1.201 | 55.981 | 1.00 | 29.54 | O |
| ATOM | 4052 | N | ARG | B | 220 | 3.021 | 0.290 | 57.116 | 1.00 | 29.39 | N |
| ATOM | 4053 | CA | ARG | B | 220 | 3.668 | -0.974 | 57.428 | 1.00 | 30.78 | C |
| ATOM | 4054 | CB | ARG | B | 220 | 2.605 | -2.041 | 57.662 | 1.00 | 29.25 | C |
| ATOM | 4055 | CG | ARG | B | 220 | 3.165 | -3.333 | 58.205 | 1.00 | 30.56 | C |
| ATOM | 4056 | CD | ARG | B | 220 | 2.041 | -4.091 | 58.838 | 1.00 | 32.08 | C |
| ATOM | 4057 | NE | ARG | B | 220 | 1.402 | -4.971 | 57.879 | 1.00 | 36.51 | N |
| ATOM | 4058 | CZ | ARG | B | 220 | 0.121 | -5.310 | 57.925 | 1.00 | 38.74 | C |
| ATOM | 4059 | NH1 | ARG | B | 220 | -0.650 | -4.825 | 58.892 | 1.00 | 35.31 | N |
| ATOM | 4060 | NH2 | ARG | B | 220 | -0.381 | -6.132 | 57.007 | 1.00 | 38.50 | N |
| ATOM | 4061 | C | ARG | B | 220 | 4.759 | -1.547 | 56.520 | 1.00 | 31.16 | C |
| ATOM | 4062 | O | ARG | B | 220 | 5.810 | -1.953 | 57.013 | 1.00 | 32.43 | O |
| ATOM | 4063 | N | TYR | B | 221 | 4.507 | -1.625 | 55.220 | 1.00 | 30.94 | N |
| ATOM | 4064 | CA | TYR | B | 221 | 5.489 | -2.176 | 54.294 | 1.00 | 31.21 | C |
| ATOM | 4065 | CB | TYR | B | 221 | 4.894 | -2.287 | 52.892 | 1.00 | 31.30 | C |
| ATOM | 4066 | CG | TYR | B | 221 | 3.798 | -3.300 | 52.759 | 1.00 | 32.81 | C |
| ATOM | 4067 | CD1 | TYR | B | 221 | 3.537 | -4.222 | 53.775 | 1.00 | 33.25 | C |
| ATOM | 4068 | CE1 | TYR | B | 221 | 2.539 | -5.177 | 53.633 | 1.00 | 33.93 | C |
| ATOM | 4069 | CD2 | TYR | B | 221 | 3.031 | -3.359 | 51.600 | 1.00 | 32.77 | C |
| ATOM | 4070 | CE2 | TYR | B | 221 | 2.030 | -4.306 | 51.446 | 1.00 | 31.85 | C |
| ATOM | 4071 | CZ | TYR | B | 221 | 1.787 | -5.215 | 52.461 | 1.00 | 33.44 | C |
| ATOM | 4072 | OH | TYR | B | 221 | 0.802 | -6.168 | 52.310 | 1.00 | 32.26 | O |
| ATOM | 4073 | C | TYR | B | 221 | 6.711 | -1.285 | 54.242 | 1.00 | 30.17 | C |
| ATOM | 4074 | O | TYR | B | 221 | 7.825 | -1.758 | 54.039 | 1.00 | 29.88 | O |
| ATOM | 4075 | N | TYR | B | 222 | 6.476 | 0.010 | 54.421 | 1.00 | 30.99 | N |
| ATOM | 4076 | CA | TYR | B | 222 | 7.526 | 1.017 | 54.388 | 1.00 | 30.72 | C |
| ATOM | 4077 | CB | TYR | B | 222 | 7.092 | 2.181 | 53.501 | 1.00 | 29.73 | C |
| ATOM | 4078 | CG | TYR | B | 222 | 6.343 | 1.724 | 52.276 | 1.00 | 32.49 | C |
| ATOM | 4079 | CD1 | TYR | B | 222 | 4.953 | 1.635 | 52.283 | 1.00 | 30.02 | C |
| ATOM | 4080 | CE1 | TYR | B | 222 | 4.266 | 1.115 | 51.200 | 1.00 | 29.96 | C |

| | | | | | | | | | | | |
|---|---|---|---|---|---|---|---|---|---|---|---|
| ATOM | 4081 | CD2 | TYR | B | 222 | 7.026 | 1.292 | 51.134 | 1.00 | 31.93 | C |
| ATOM | 4082 | CE2 | TYR | B | 222 | 6.339 | 0.768 | 50.042 | 1.00 | 32.44 | C |
| ATOM | 4083 | CZ | TYR | B | 222 | 4.961 | 0.683 | 50.084 | 1.00 | 31.78 | C |
| ATOM | 4084 | OH | TYR | B | 222 | 4.275 | 0.142 | 49.026 | 1.00 | 31.99 | O |
| ATOM | 4085 | C | TYR | B | 222 | 7.820 | 1.531 | 55.795 | 1.00 | 31.12 | C |
| ATOM | 4086 | O | TYR | B | 222 | 8.221 | 2.682 | 55.969 | 1.00 | 34.04 | O |
| ATOM | 4087 | N | ARG | B | 223 | 7.608 | 0.683 | 56.796 | 1.00 | 27.89 | N |
| ATOM | 4088 | CA | ARG | B | 223 | 7.861 | 1.059 | 58.180 | 1.00 | 27.26 | C |
| ATOM | 4089 | CB | ARG | B | 223 | 6.802 | 0.437 | 59.091 | 1.00 | 27.10 | C |
| ATOM | 4090 | CG | ARG | B | 223 | 7.104 | 0.583 | 60.561 | 1.00 | 26.22 | C |
| ATOM | 4091 | CD | ARG | B | 223 | 5.835 | 0.790 | 61.352 | 1.00 | 26.23 | C |
| ATOM | 4092 | NE | ARG | B | 223 | 4.946 | -0.354 | 61.278 | 1.00 | 27.01 | N |
| ATOM | 4093 | CZ | ARG | B | 223 | 3.642 | -0.274 | 61.050 | 1.00 | 26.15 | C |
| ATOM | 4094 | NH1 | ARG | B | 223 | 3.064 | 0.902 | 60.864 | 1.00 | 25.42 | N |
| ATOM | 4095 | NH2 | ARG | B | 223 | 2.908 | -1.377 | 61.024 | 1.00 | 27.16 | N |
| ATOM | 4096 | C | ARG | B | 223 | 9.256 | 0.631 | 58.634 | 1.00 | 27.29 | C |
| ATOM | 4097 | O | ARG | B | 223 | 9.621 | -0.542 | 58.522 | 1.00 | 25.29 | O |
| ATOM | 4098 | N | ALA | B | 224 | 10.027 | 1.591 | 59.141 | 1.00 | 27.18 | N |
| ATOM | 4099 | CA | ALA | B | 224 | 11.387 | 1.335 | 59.618 | 1.00 | 28.16 | C |
| ATOM | 4100 | CB | ALA | B | 224 | 12.056 | 2.643 | 60.022 | 1.00 | 27.85 | C |
| ATOM | 4101 | C | ALA | B | 224 | 11.416 | 0.353 | 60.786 | 1.00 | 27.71 | C |
| ATOM | 4102 | O | ALA | B | 224 | 10.514 | 0.332 | 61.621 | 1.00 | 27.48 | O |
| ATOM | 4103 | N | PRO | B | 225 | 12.469 | -0.471 | 60.860 | 1.00 | 28.97 | N |
| ATOM | 4104 | CD | PRO | B | 225 | 13.621 | -0.531 | 59.939 | 1.00 | 28.90 | C |
| ATOM | 4105 | CA | PRO | B | 225 | 12.596 | -1.458 | 61.935 | 1.00 | 30.04 | C |
| ATOM | 4106 | CB | PRO | B | 225 | 13.837 | -2.258 | 61.523 | 1.00 | 29.92 | C |
| ATOM | 4107 | CG | PRO | B | 225 | 14.654 | -1.266 | 60.759 | 1.00 | 28.82 | C |
| ATOM | 4108 | C | PRO | B | 225 | 12.664 | -0.909 | 63.376 | 1.00 | 31.07 | C |
| ATOM | 4109 | O | PRO | B | 225 | 12.178 | -1.562 | 64.303 | 1.00 | 30.75 | O |
| ATOM | 4110 | N | GLU | B | 226 | 13.255 | 0.273 | 63.574 | 1.00 | 30.99 | N |
| ATOM | 4111 | CA | GLU | B | 226 | 13.335 | 0.851 | 64.922 | 1.00 | 31.54 | C |
| ATOM | 4112 | CB | GLU | B | 226 | 14.009 | 2.232 | 64.925 | 1.00 | 32.26 | C |
| ATOM | 4113 | CG | GLU | B | 226 | 15.221 | 2.368 | 64.045 | 1.00 | 33.82 | C |
| ATOM | 4114 | CD | GLU | B | 226 | 14.879 | 2.909 | 62.675 | 1.00 | 32.31 | C |
| ATOM | 4115 | OE1 | GLU | B | 226 | 14.882 | 4.145 | 62.488 | 1.00 | 29.55 | O |
| ATOM | 4116 | OE2 | GLU | B | 226 | 14.601 | 2.086 | 61.792 | 1.00 | 34.51 | O |
| ATOM | 4117 | C | GLU | B | 226 | 11.920 | 1.034 | 65.439 | 1.00 | 31.49 | C |
| ATOM | 4118 | O | GLU | B | 226 | 11.640 | 0.792 | 66.607 | 1.00 | 32.04 | O |
| ATOM | 4119 | N | LEU | B | 227 | 11.038 | 1.473 | 64.545 | 1.00 | 31.55 | N |
| ATOM | 4120 | CA | LEU | B | 227 | 9.641 | 1.709 | 64.871 | 1.00 | 30.89 | C |
| ATOM | 4121 | CB | LEU | B | 227 | 8.920 | 2.286 | 63.648 | 1.00 | 31.74 | C |
| ATOM | 4122 | CG | LEU | B | 227 | 9.420 | 3.638 | 63.108 | 1.00 | 30.96 | C |
| ATOM | 4123 | CD1 | LEU | B | 227 | 8.954 | 3.825 | 61.680 | 1.00 | 29.33 | C |
| ATOM | 4124 | CD2 | LEU | B | 227 | 8.943 | 4.774 | 63.997 | 1.00 | 28.75 | C |
| ATOM | 4125 | C | LEU | B | 227 | 8.956 | 0.429 | 65.329 | 1.00 | 31.00 | C |
| ATOM | 4126 | O | LEU | B | 227 | 8.266 | 0.427 | 66.348 | 1.00 | 30.14 | O |
| ATOM | 4127 | N | ILE | B | 228 | 9.144 | -0.656 | 64.578 | 1.00 | 31.59 | N |
| ATOM | 4128 | CA | ILE | B | 228 | 8.551 | -1.942 | 64.930 | 1.00 | 32.83 | C |
| ATOM | 4129 | CB | ILE | B | 228 | 8.929 | -3.051 | 63.924 | 1.00 | 33.65 | C |
| ATOM | 4130 | CG2 | ILE | B | 228 | 8.229 | -4.356 | 64.306 | 1.00 | 31.43 | C |
| ATOM | 4131 | CG1 | ILE | B | 228 | 8.536 | -2.639 | 62.503 | 1.00 | 33.49 | C |
| ATOM | 4132 | CD1 | ILE | B | 228 | 8.927 | -3.665 | 61.441 | 1.00 | 30.63 | C |
| ATOM | 4133 | C | ILE | B | 228 | 9.055 | -2.372 | 66.311 | 1.00 | 34.16 | C |
| ATOM | 4134 | O | ILE | B | 228 | 8.349 | -3.057 | 67.049 | 1.00 | 32.59 | O |
| ATOM | 4135 | N | PHE | B | 229 | 10.285 | -1.972 | 66.631 | 1.00 | 35.47 | N |
| ATOM | 4136 | CA | PHE | B | 229 | 10.910 | -2.280 | 67.909 | 1.00 | 37.12 | C |
| ATOM | 4137 | CB | PHE | B | 229 | 12.431 | -2.091 | 67.831 | 1.00 | 36.90 | C |
| ATOM | 4138 | CG | PHE | B | 229 | 13.185 | -3.362 | 67.563 | 1.00 | 38.62 | C |
| ATOM | 4139 | CD1 | PHE | B | 229 | 13.196 | -4.394 | 68.501 | 1.00 | 39.41 | C |
| ATOM | 4140 | CD2 | PHE | B | 229 | 13.862 | -3.545 | 66.363 | 1.00 | 38.51 | C |
| ATOM | 4141 | CE1 | PHE | B | 229 | 13.869 | -5.593 | 68.243 | 1.00 | 39.18 | C |
| ATOM | 4142 | CE2 | PHE | B | 229 | 14.537 | -4.738 | 66.094 | 1.00 | 39.26 | C |
| ATOM | 4143 | CZ | PHE | B | 229 | 14.540 | -5.764 | 67.036 | 1.00 | 39.69 | C |
| ATOM | 4144 | C | PHE | B | 229 | 10.346 | -1.405 | 69.024 | 1.00 | 37.36 | C |
| ATOM | 4145 | O | PHE | B | 229 | 10.722 | -1.565 | 70.179 | 1.00 | 38.19 | O |
| ATOM | 4146 | N | GLY | B | 230 | 9.452 | -0.484 | 68.680 | 1.00 | 38.33 | N |
| ATOM | 4147 | CA | GLY | B | 230 | 8.855 | 0.364 | 69.694 | 1.00 | 39.06 | C |

| | | | | | | | | | | | |
|---|---|---|---|---|---|---|---|---|---|---|---|
| ATOM | 4148 | C | GLY | B | 230 | 9.626 | 1.626 | 70.033 | 1.00 | 39.63 | C |
| ATOM | 4149 | O | GLY | B | 230 | 9.239 | 2.379 | 70.940 | 1.00 | 40.52 | O |
| ATOM | 4150 | N | ALA | B | 231 | 10.721 | 1.851 | 69.316 | 1.00 | 39.66 | N |
| ATOM | 4151 | CA | ALA | B | 231 | 11.546 | 3.034 | 69.525 | 1.00 | 40.39 | C |
| ATOM | 4152 | CB | ALA | B | 231 | 12.555 | 3.165 | 68.401 | 1.00 | 40.02 | C |
| ATOM | 4153 | C | ALA | B | 231 | 10.647 | 4.261 | 69.567 | 1.00 | 40.99 | C |
| ATOM | 4154 | O | ALA | B | 231 | 9.604 | 4.292 | 68.911 | 1.00 | 42.65 | O |
| ATOM | 4155 | N | THR | B | 232 | 11.035 | 5.264 | 70.347 | 1.00 | 42.39 | N |
| ATOM | 4156 | CA | THR | B | 232 | 10.244 | 6.482 | 70.450 | 1.00 | 43.03 | C |
| ATOM | 4157 | CB | THR | B | 232 | 9.483 | 6.538 | 71.786 | 1.00 | 44.88 | C |
| ATOM | 4158 | OG1 | THR | B | 232 | 10.419 | 6.592 | 72.870 | 1.00 | 47.38 | O |
| ATOM | 4159 | CG2 | THR | B | 232 | 8.620 | 5.294 | 71.950 | 1.00 | 45.78 | C |
| ATOM | 4160 | C | THR | B | 232 | 11.143 | 7.699 | 70.327 | 1.00 | 42.76 | C |
| ATOM | 4161 | O | THR | B | 232 | 10.676 | 8.829 | 70.375 | 1.00 | 41.78 | O |
| ATOM | 4162 | N | ASP | B | 233 | 12.436 | 7.454 | 70.143 | 1.00 | 44.19 | N |
| ATOM | 4163 | CA | ASP | B | 233 | 13.418 | 8.521 | 69.999 | 1.00 | 46.25 | C |
| ATOM | 4164 | CB | ASP | B | 233 | 14.556 | 8.333 | 71.003 | 1.00 | 51.25 | C |
| ATOM | 4165 | CG | ASP | B | 233 | 15.487 | 7.195 | 70.611 | 1.00 | 55.99 | C |
| ATOM | 4166 | OD1 | ASP | B | 233 | 15.002 | 6.030 | 70.557 | 1.00 | 58.63 | O |
| ATOM | 4167 | OD2 | ASP | B | 233 | 16.693 | 7.467 | 70.352 | 1.00 | 57.09 | O |
| ATOM | 4168 | C | ASP | B | 233 | 14.013 | 8.489 | 68.594 | 1.00 | 44.84 | C |
| ATOM | 4169 | O | ASP | B | 233 | 15.169 | 8.876 | 68.389 | 1.00 | 46.39 | O |
| ATOM | 4170 | N | TYR | B | 234 | 13.243 | 8.015 | 67.625 | 1.00 | 41.70 | N |
| ATOM | 4171 | CA | TYR | B | 234 | 13.747 | 7.933 | 66.257 | 1.00 | 37.50 | C |
| ATOM | 4172 | CB | TYR | B | 234 | 12.806 | 7.089 | 65.408 | 1.00 | 33.16 | C |
| ATOM | 4173 | CG | TYR | B | 234 | 11.392 | 7.600 | 65.387 | 1.00 | 30.63 | C |
| ATOM | 4174 | CD1 | TYR | B | 234 | 11.010 | 8.626 | 64.528 | 1.00 | 29.14 | C |
| ATOM | 4175 | CE1 | TYR | B | 234 | 9.694 | 9.073 | 64.486 | 1.00 | 27.73 | C |
| ATOM | 4176 | CD2 | TYR | B | 234 | 10.421 | 7.041 | 66.210 | 1.00 | 30.51 | C |
| ATOM | 4177 | CE2 | TYR | B | 234 | 9.109 | 7.484 | 66.173 | 1.00 | 29.26 | C |
| ATOM | 4178 | CZ | TYR | B | 234 | 8.756 | 8.496 | 65.309 | 1.00 | 28.16 | C |
| ATOM | 4179 | OH | TYR | B | 234 | 7.456 | 8.923 | 65.265 | 1.00 | 31.73 | O |
| ATOM | 4180 | C | TYR | B | 234 | 13.956 | 9.307 | 65.627 | 1.00 | 37.03 | C |
| ATOM | 4181 | O | TYR | B | 234 | 13.481 | 10.325 | 66.136 | 1.00 | 37.24 | O |
| ATOM | 4182 | N | THR | B | 235 | 14.686 | 9.325 | 64.519 | 1.00 | 35.56 | N |
| ATOM | 4183 | CA | THR | B | 235 | 14.997 | 10.553 | 63.804 | 1.00 | 34.47 | C |
| ATOM | 4184 | CB | THR | B | 235 | 16.484 | 10.688 | 63.598 | 1.00 | 34.02 | C |
| ATOM | 4185 | OG1 | THR | B | 235 | 16.895 | 9.771 | 62.580 | 1.00 | 32.65 | O |
| ATOM | 4186 | CG2 | THR | B | 235 | 17.227 | 10.344 | 64.881 | 1.00 | 33.68 | C |
| ATOM | 4187 | C | THR | B | 235 | 14.346 | 10.520 | 62.430 | 1.00 | 35.50 | C |
| ATOM | 4188 | O | THR | B | 235 | 13.641 | 9.566 | 62.088 | 1.00 | 36.88 | O |
| ATOM | 4189 | N | SER | B | 236 | 14.588 | 11.556 | 61.638 | 1.00 | 34.58 | N |
| ATOM | 4190 | CA | SER | B | 236 | 14.011 | 11.624 | 60.307 | 1.00 | 33.61 | C |
| ATOM | 4191 | CB | SER | B | 236 | 14.221 | 13.013 | 59.698 | 1.00 | 32.56 | C |
| ATOM | 4192 | OG | SER | B | 236 | 15.584 | 13.233 | 59.387 | 1.00 | 35.02 | O |
| ATOM | 4193 | C | SER | B | 236 | 14.601 | 10.561 | 59.380 | 1.00 | 32.94 | C |
| ATOM | 4194 | O | SER | B | 236 | 14.220 | 10.469 | 58.213 | 1.00 | 33.83 | O |
| ATOM | 4195 | N | SER | B | 237 | 15.518 | 9.748 | 59.889 | 1.00 | 31.60 | N |
| ATOM | 4196 | CA | SER | B | 237 | 16.105 | 8.726 | 59.048 | 1.00 | 30.21 | C |
| ATOM | 4197 | CB | SER | B | 237 | 17.356 | 8.122 | 59.709 | 1.00 | 28.10 | C |
| ATOM | 4198 | OG | SER | B | 237 | 17.042 | 7.246 | 60.777 | 1.00 | 29.05 | O |
| ATOM | 4199 | C | SER | B | 237 | 15.072 | 7.644 | 58.734 | 1.00 | 28.86 | C |
| ATOM | 4200 | O | SER | B | 237 | 15.276 | 6.835 | 57.835 | 1.00 | 29.69 | O |
| ATOM | 4201 | N | ILE | B | 238 | 13.964 | 7.624 | 59.466 | 1.00 | 28.19 | N |
| ATOM | 4202 | CA | ILE | B | 238 | 12.928 | 6.634 | 59.179 | 1.00 | 29.12 | C |
| ATOM | 4203 | CB | ILE | B | 238 | 11.713 | 6.708 | 60.152 | 1.00 | 30.75 | C |
| ATOM | 4204 | CG2 | ILE | B | 238 | 12.100 | 6.167 | 61.520 | 1.00 | 28.07 | C |
| ATOM | 4205 | CG1 | ILE | B | 238 | 11.167 | 8.141 | 60.184 | 1.00 | 29.50 | C |
| ATOM | 4206 | CD1 | ILE | B | 238 | 9.836 | 8.288 | 60.864 | 1.00 | 30.19 | C |
| ATOM | 4207 | C | ILE | B | 238 | 12.388 | 6.887 | 57.768 | 1.00 | 28.36 | C |
| ATOM | 4208 | O | ILE | B | 238 | 11.972 | 5.960 | 57.086 | 1.00 | 26.43 | O |
| ATOM | 4209 | N | ASP | B | 239 | 12.379 | 8.146 | 57.343 | 1.00 | 26.12 | N |
| ATOM | 4210 | CA | ASP | B | 239 | 11.893 | 8.472 | 56.011 | 1.00 | 27.52 | C |
| ATOM | 4211 | CB | ASP | B | 239 | 11.824 | 9.980 | 55.800 | 1.00 | 27.26 | C |
| ATOM | 4212 | CG | ASP | B | 239 | 10.602 | 10.595 | 56.415 | 1.00 | 28.46 | C |
| ATOM | 4213 | OD1 | ASP | B | 239 | 9.680 | 9.837 | 56.784 | 1.00 | 28.42 | O |
| ATOM | 4214 | OD2 | ASP | B | 239 | 10.558 | 11.838 | 56.517 | 1.00 | 29.33 | O |

| | | | | | | | | | | | |
|---|---|---|---|---|---|---|---|---|---|---|---|
| ATOM | 4215 | C | ASP | B | 239 | 12.790 | 7.896 | 54.942 | 1.00 | 28.52 | C |
| ATOM | 4216 | O | ASP | B | 239 | 12.329 | 7.492 | 53.880 | 1.00 | 30.37 | O |
| ATOM | 4217 | N | VAL | B | 240 | 14.085 | 7.882 | 55.231 | 1.00 | 28.91 | N |
| ATOM | 4218 | CA | VAL | B | 240 | 15.088 | 7.381 | 54.310 | 1.00 | 27.22 | C |
| ATOM | 4219 | CB | VAL | B | 240 | 16.485 | 7.745 | 54.810 | 1.00 | 26.01 | C |
| ATOM | 4220 | CG1 | VAL | B | 240 | 17.540 | 7.006 | 54.017 | 1.00 | 24.48 | C |
| ATOM | 4221 | CG2 | VAL | B | 240 | 16.680 | 9.248 | 54.701 | 1.00 | 21.50 | C |
| ATOM | 4222 | C | VAL | B | 240 | 14.947 | 5.879 | 54.155 | 1.00 | 29.86 | C |
| ATOM | 4223 | O | VAL | B | 240 | 15.126 | 5.336 | 53.062 | 1.00 | 30.67 | O |
| ATOM | 4224 | N | TRP | B | 241 | 14.622 | 5.202 | 55.251 | 1.00 | 30.12 | N |
| ATOM | 4225 | CA | TRP | B | 241 | 14.416 | 3.765 | 55.195 | 1.00 | 29.94 | C |
| ATOM | 4226 | CB | TRP | B | 241 | 14.130 | 3.197 | 56.592 | 1.00 | 27.23 | C |
| ATOM | 4227 | CG | TRP | B | 241 | 13.508 | 1.835 | 56.570 | 1.00 | 26.46 | C |
| ATOM | 4228 | CD2 | TRP | B | 241 | 14.183 | 0.575 | 56.673 | 1.00 | 26.86 | C |
| ATOM | 4229 | CE2 | TRP | B | 241 | 13.205 | -0.436 | 56.531 | 1.00 | 27.04 | C |
| ATOM | 4230 | CE3 | TRP | B | 241 | 15.517 | 0.200 | 56.867 | 1.00 | 26.80 | C |
| ATOM | 4231 | CD1 | TRP | B | 241 | 12.188 | 1.542 | 56.381 | 1.00 | 26.67 | C |
| ATOM | 4232 | NE1 | TRP | B | 241 | 11.997 | 0.183 | 56.357 | 1.00 | 25.76 | N |
| ATOM | 4233 | CZ2 | TRP | B | 241 | 13.522 | -1.800 | 56.577 | 1.00 | 28.09 | C |
| ATOM | 4234 | CZ3 | TRP | B | 241 | 15.832 | -1.158 | 56.913 | 1.00 | 27.80 | C |
| ATOM | 4235 | CH2 | TRP | B | 241 | 14.836 | -2.139 | 56.767 | 1.00 | 29.73 | C |
| ATOM | 4236 | C | TRP | B | 241 | 13.216 | 3.579 | 54.276 | 1.00 | 30.72 | C |
| ATOM | 4237 | O | TRP | B | 241 | 13.259 | 2.778 | 53.344 | 1.00 | 32.37 | O |
| ATOM | 4238 | N | SER | B | 242 | 12.153 | 4.333 | 54.546 | 1.00 | 31.26 | N |
| ATOM | 4239 | CA | SER | B | 242 | 10.927 | 4.282 | 53.743 | 1.00 | 31.82 | C |
| ATOM | 4240 | CB | SER | B | 242 | 9.926 | 5.349 | 54.218 | 1.00 | 29.82 | C |
| ATOM | 4241 | OG | SER | B | 242 | 9.252 | 4.951 | 55.393 | 1.00 | 29.37 | O |
| ATOM | 4242 | C | SER | B | 242 | 11.227 | 4.514 | 52.269 | 1.00 | 30.85 | C |
| ATOM | 4243 | O | SER | B | 242 | 10.618 | 3.900 | 51.407 | 1.00 | 29.65 | O |
| ATOM | 4244 | N | ALA | B | 243 | 12.170 | 5.410 | 52.001 | 1.00 | 30.38 | N |
| ATOM | 4245 | CA | ALA | B | 243 | 12.551 | 5.731 | 50.642 | 1.00 | 32.17 | C |
| ATOM | 4246 | CB | ALA | B | 243 | 13.520 | 6.909 | 50.630 | 1.00 | 31.00 | C |
| ATOM | 4247 | C | ALA | B | 243 | 13.181 | 4.513 | 49.967 | 1.00 | 34.25 | C |
| ATOM | 4248 | O | ALA | B | 243 | 12.740 | 4.097 | 48.901 | 1.00 | 35.96 | O |
| ATOM | 4249 | N | GLY | B | 244 | 14.210 | 3.942 | 50.587 | 1.00 | 34.43 | N |
| ATOM | 4250 | CA | GLY | B | 244 | 14.851 | 2.780 | 50.008 | 1.00 | 34.19 | C |
| ATOM | 4251 | C | GLY | B | 244 | 13.849 | 1.652 | 49.815 | 1.00 | 34.12 | C |
| ATOM | 4252 | O | GLY | B | 244 | 14.058 | 0.749 | 48.999 | 1.00 | 35.39 | O |
| ATOM | 4253 | N | CYS | B | 245 | 12.759 | 1.706 | 50.579 | 1.00 | 32.43 | N |
| ATOM | 4254 | CA | CYS | B | 245 | 11.683 | 0.709 | 50.509 | 1.00 | 30.90 | C |
| ATOM | 4255 | CB | CYS | B | 245 | 10.721 | 0.868 | 51.684 | 1.00 | 28.81 | C |
| ATOM | 4256 | SG | CYS | B | 245 | 10.899 | -0.391 | 52.974 | 1.00 | 29.95 | S |
| ATOM | 4257 | C | CYS | B | 245 | 10.890 | 0.877 | 49.228 | 1.00 | 30.62 | C |
| ATOM | 4258 | O | CYS | B | 245 | 10.463 | -0.094 | 48.606 | 1.00 | 29.41 | O |
| ATOM | 4259 | N | VAL | B | 246 | 10.679 | 2.129 | 48.854 | 1.00 | 28.70 | N |
| ATOM | 4260 | CA | VAL | B | 246 | 9.959 | 2.440 | 47.643 | 1.00 | 27.85 | C |
| ATOM | 4261 | CB | VAL | B | 246 | 9.516 | 3.919 | 47.641 | 1.00 | 27.42 | C |
| ATOM | 4262 | CG1 | VAL | B | 246 | 9.583 | 4.491 | 46.239 | 1.00 | 26.85 | C |
| ATOM | 4263 | CG2 | VAL | B | 246 | 8.102 | 4.032 | 48.203 | 1.00 | 26.60 | C |
| ATOM | 4264 | C | VAL | B | 246 | 10.853 | 2.146 | 46.436 | 1.00 | 28.50 | C |
| ATOM | 4265 | O | VAL | B | 246 | 10.365 | 1.711 | 45.402 | 1.00 | 28.72 | O |
| ATOM | 4266 | N | LEU | B | 247 | 12.159 | 2.369 | 46.580 | 1.00 | 28.10 | N |
| ATOM | 4267 | CA | LEU | B | 247 | 13.112 | 2.113 | 45.500 | 1.00 | 27.73 | C |
| ATOM | 4268 | CB | LEU | B | 247 | 14.503 | 2.652 | 45.854 | 1.00 | 28.38 | C |
| ATOM | 4269 | CG | LEU | B | 247 | 15.699 | 2.142 | 45.022 | 1.00 | 29.15 | C |
| ATOM | 4270 | CD1 | LEU | B | 247 | 15.650 | 2.692 | 43.602 | 1.00 | 29.85 | C |
| ATOM | 4271 | CD2 | LEU | B | 247 | 17.004 | 2.541 | 45.691 | 1.00 | 29.28 | C |
| ATOM | 4272 | C | LEU | B | 247 | 13.220 | 0.627 | 45.220 | 1.00 | 27.53 | C |
| ATOM | 4273 | O | LEU | B | 247 | 13.171 | 0.203 | 44.073 | 1.00 | 28.99 | O |
| ATOM | 4274 | N | ALA | B | 248 | 13.380 | -0.163 | 46.275 | 1.00 | 29.23 | N |
| ATOM | 4275 | CA | ALA | B | 248 | 13.495 | -1.602 | 46.127 | 1.00 | 29.02 | C |
| ATOM | 4276 | CB | ALA | B | 248 | 13.647 | -2.260 | 47.482 | 1.00 | 25.95 | C |
| ATOM | 4277 | C | ALA | B | 248 | 12.236 | -2.091 | 45.440 | 1.00 | 29.91 | C |
| ATOM | 4278 | O | ALA | B | 248 | 12.287 | -2.946 | 44.559 | 1.00 | 30.95 | O |
| ATOM | 4279 | N | GLU | B | 249 | 11.101 | -1.529 | 45.842 | 1.00 | 29.57 | N |
| ATOM | 4280 | CA | GLU | B | 249 | 9.829 | -1.918 | 45.262 | 1.00 | 29.48 | C |
| ATOM | 4281 | CB | GLU | B | 249 | 8.675 | -1.137 | 45.891 | 1.00 | 28.81 | C |

| | | | | | | | | | | | |
|---|---|---|---|---|---|---|---|---|---|---|---|
| ATOM | 4282 | CG | GLU | B | 249 | 7.326 | -1.787 | 45.630 | 1.00 | 30.29 | C |
| ATOM | 4283 | CD | GLU | B | 249 | 6.199 | -1.226 | 46.474 | 1.00 | 30.72 | C |
| ATOM | 4284 | OE1 | GLU | B | 249 | 6.350 | -1.162 | 47.714 | 1.00 | 31.32 | O |
| ATOM | 4285 | OE2 | GLU | B | 249 | 5.155 | -0.855 | 45.897 | 1.00 | 29.71 | O |
| ATOM | 4286 | C | GLU | B | 249 | 9.830 | -1.701 | 43.757 | 1.00 | 29.75 | C |
| ATOM | 4287 | O | GLU | B | 249 | 9.460 | -2.590 | 43.002 | 1.00 | 32.58 | O |
| ATOM | 4288 | N | LEU | B | 250 | 10.246 | -0.521 | 43.323 | 1.00 | 28.78 | N |
| ATOM | 4289 | CA | LEU | B | 250 | 10.279 | -0.213 | 41.911 | 1.00 | 28.45 | C |
| ATOM | 4290 | CB | LEU | B | 250 | 10.681 | 1.244 | 41.713 | 1.00 | 26.40 | C |
| ATOM | 4291 | CG | LEU | B | 250 | 9.701 | 2.235 | 42.341 | 1.00 | 25.28 | C |
| ATOM | 4292 | CD1 | LEU | B | 250 | 10.372 | 3.587 | 42.432 | 1.00 | 24.43 | C |
| ATOM | 4293 | CD2 | LEU | B | 250 | 8.407 | 2.306 | 41.545 | 1.00 | 21.07 | C |
| ATOM | 4294 | C | LEU | B | 250 | 11.201 | -1.134 | 41.121 | 1.00 | 30.26 | C |
| ATOM | 4295 | O | LEU | B | 250 | 10.901 | -1.471 | 39.987 | 1.00 | 31.99 | O |
| ATOM | 4296 | N | LEU | B | 251 | 12.317 | -1.545 | 41.712 | 1.00 | 31.71 | N |
| ATOM | 4297 | CA | LEU | B | 251 | 13.246 | -2.430 | 41.033 | 1.00 | 31.06 | C |
| ATOM | 4298 | CB | LEU | B | 251 | 14.587 | -2.436 | 41.746 | 1.00 | 30.98 | C |
| ATOM | 4299 | CG | LEU | B | 251 | 15.357 | -1.128 | 41.769 | 1.00 | 31.94 | C |
| ATOM | 4300 | CD1 | LEU | B | 251 | 16.659 | -1.348 | 42.534 | 1.00 | 31.93 | C |
| ATOM | 4301 | CD2 | LEU | B | 251 | 15.634 | -0.656 | 40.347 | 1.00 | 29.86 | C |
| ATOM | 4302 | C | LEU | B | 251 | 12.738 | -3.855 | 40.984 | 1.00 | 32.10 | C |
| ATOM | 4303 | O | LEU | B | 251 | 13.126 | -4.627 | 40.114 | 1.00 | 32.89 | O |
| ATOM | 4304 | N | LEU | B | 252 | 11.869 | -4.189 | 41.927 | 1.00 | 34.05 | N |
| ATOM | 4305 | CA | LEU | B | 252 | 11.323 | -5.528 | 42.080 | 1.00 | 34.81 | C |
| ATOM | 4306 | CB | LEU | B | 252 | 11.289 | -5.867 | 43.556 | 1.00 | 36.83 | C |
| ATOM | 4307 | CG | LEU | B | 252 | 12.094 | -7.063 | 44.031 | 1.00 | 39.61 | C |
| ATOM | 4308 | CD1 | LEU | B | 252 | 13.564 | -6.874 | 43.717 | 1.00 | 40.43 | C |
| ATOM | 4309 | CD2 | LEU | B | 252 | 11.881 | -7.200 | 45.522 | 1.00 | 41.89 | C |
| ATOM | 4310 | C | LEU | B | 252 | 9.943 | -5.798 | 41.514 | 1.00 | 35.60 | C |
| ATOM | 4311 | O | LEU | B | 252 | 9.612 | -6.947 | 41.233 | 1.00 | 35.71 | O |
| ATOM | 4312 | N | GLY | B | 253 | 9.126 | -4.764 | 41.373 | 1.00 | 35.88 | N |
| ATOM | 4313 | CA | GLY | B | 253 | 7.787 | -4.972 | 40.852 | 1.00 | 37.04 | C |
| ATOM | 4314 | C | GLY | B | 253 | 6.785 | -5.351 | 41.929 | 1.00 | 38.34 | C |
| ATOM | 4315 | O | GLY | B | 253 | 5.651 | -5.719 | 41.624 | 1.00 | 38.40 | O |
| ATOM | 4316 | N | GLN | B | 254 | 7.210 | -5.263 | 43.189 | 1.00 | 38.64 | N |
| ATOM | 4317 | CA | GLN | B | 254 | 6.367 | -5.575 | 44.342 | 1.00 | 38.56 | C |
| ATOM | 4318 | CB | GLN | B | 254 | 6.124 | -7.082 | 44.423 | 1.00 | 39.55 | C |
| ATOM | 4319 | CG | GLN | B | 254 | 7.396 | -7.889 | 44.296 | 1.00 | 43.80 | C |
| ATOM | 4320 | CD | GLN | B | 254 | 7.193 | -9.348 | 44.607 | 1.00 | 44.44 | C |
| ATOM | 4321 | OE1 | GLN | B | 254 | 6.648 | -9.700 | 45.647 | 1.00 | 47.29 | O |
| ATOM | 4322 | NE2 | GLN | B | 254 | 7.637 | -10.211 | 43.707 | 1.00 | 46.95 | N |
| ATOM | 4323 | C | GLN | B | 254 | 7.103 | -5.101 | 45.595 | 1.00 | 38.17 | C |
| ATOM | 4324 | O | GLN | B | 254 | 8.318 | -4.894 | 45.562 | 1.00 | 37.78 | O |
| ATOM | 4325 | N | PRO | B | 255 | 6.379 | -4.916 | 46.714 | 1.00 | 37.93 | N |
| ATOM | 4326 | CD | PRO | B | 255 | 4.921 | -5.036 | 46.858 | 1.00 | 38.27 | C |
| ATOM | 4327 | CA | PRO | B | 255 | 6.978 | -4.464 | 47.975 | 1.00 | 36.84 | C |
| ATOM | 4328 | CB | PRO | B | 255 | 5.791 | -4.439 | 48.932 | 1.00 | 36.35 | C |
| ATOM | 4329 | CG | PRO | B | 255 | 4.646 | -4.129 | 48.032 | 1.00 | 37.60 | C |
| ATOM | 4330 | C | PRO | B | 255 | 8.068 | -5.416 | 48.446 | 1.00 | 36.52 | C |
| ATOM | 4331 | O | PRO | B | 255 | 7.877 | -6.629 | 48.450 | 1.00 | 37.94 | O |
| ATOM | 4332 | N | ILE | B | 256 | 9.209 | -4.870 | 48.848 | 1.00 | 35.04 | N |
| ATOM | 4333 | CA | ILE | B | 256 | 10.302 | -5.712 | 49.303 | 1.00 | 33.06 | C |
| ATOM | 4334 | CB | ILE | B | 256 | 11.604 | -4.878 | 49.467 | 1.00 | 33.97 | C |
| ATOM | 4335 | CG2 | ILE | B | 256 | 11.372 | -3.712 | 50.418 | 1.00 | 35.68 | C |
| ATOM | 4336 | CG1 | ILE | B | 256 | 12.753 | -5.770 | 49.940 | 1.00 | 32.44 | C |
| ATOM | 4337 | CD1 | ILE | B | 256 | 13.254 | -6.719 | 48.906 | 1.00 | 32.33 | C |
| ATOM | 4338 | C | ILE | B | 256 | 9.940 | -6.443 | 50.603 | 1.00 | 32.44 | C |
| ATOM | 4339 | O | ILE | B | 256 | 10.270 | -7.617 | 50.768 | 1.00 | 33.52 | O |
| ATOM | 4340 | N | PHE | B | 257 | 9.242 | -5.774 | 51.513 | 1.00 | 30.39 | N |
| ATOM | 4341 | CA | PHE | B | 257 | 8.868 | -6.416 | 52.769 | 1.00 | 30.50 | C |
| ATOM | 4342 | CB | PHE | B | 257 | 9.537 | -5.702 | 53.941 | 1.00 | 28.63 | C |
| ATOM | 4343 | CG | PHE | B | 257 | 11.005 | -5.449 | 53.751 | 1.00 | 28.50 | C |
| ATOM | 4344 | CD1 | PHE | B | 257 | 11.896 | -6.498 | 53.571 | 1.00 | 28.36 | C |
| ATOM | 4345 | CD2 | PHE | B | 257 | 11.503 | -4.154 | 53.797 | 1.00 | 28.24 | C |
| ATOM | 4346 | CE1 | PHE | B | 257 | 13.268 | -6.253 | 53.446 | 1.00 | 29.17 | C |
| ATOM | 4347 | CE2 | PHE | B | 257 | 12.870 | -3.904 | 53.673 | 1.00 | 28.84 | C |
| ATOM | 4348 | CZ | PHE | B | 257 | 13.754 | -4.953 | 53.498 | 1.00 | 25.61 | C |

| | | | | | | | | | | | |
|---|---|---|---|---|---|---|---|---|---|---|---|
| ATOM | 4349 | C | PHE | B | 257 | 7.347 | -6.414 | 52.973 | 1.00 | 30.75 | C |
| ATOM | 4350 | O | PHE | B | 257 | 6.799 | -5.504 | 53.590 | 1.00 | 30.52 | O |
| ATOM | 4351 | N | PRO | B | 258 | 6.648 | -7.443 | 52.459 | 1.00 | 31.74 | N |
| ATOM | 4352 | CD | PRO | B | 258 | 7.165 | -8.452 | 51.515 | 1.00 | 31.47 | C |
| ATOM | 4353 | CA | PRO | B | 258 | 5.188 | -7.554 | 52.581 | 1.00 | 32.79 | C |
| ATOM | 4354 | CB | PRO | B | 258 | 4.816 | -8.337 | 51.329 | 1.00 | 31.09 | C |
| ATOM | 4355 | CG | PRO | B | 258 | 5.937 | -9.308 | 51.235 | 1.00 | 31.24 | C |
| ATOM | 4356 | C | PRO | B | 258 | 4.663 | -8.219 | 53.860 | 1.00 | 33.85 | C |
| ATOM | 4357 | O | PRO | B | 258 | 4.059 | -9.293 | 53.802 | 1.00 | 33.08 | O |
| ATOM | 4358 | N | GLY | B | 259 | 4.877 | -7.565 | 55.002 | 1.00 | 35.35 | N |
| ATOM | 4359 | CA | GLY | B | 259 | 4.433 | -8.106 | 56.278 | 1.00 | 36.28 | C |
| ATOM | 4360 | C | GLY | B | 259 | 2.928 | -8.129 | 56.441 | 1.00 | 38.29 | C |
| ATOM | 4361 | O | GLY | B | 259 | 2.237 | -7.203 | 56.018 | 1.00 | 38.83 | O |
| ATOM | 4362 | N | ASP | B | 260 | 2.400 | -9.184 | 57.058 | 1.00 | 38.84 | N |
| ATOM | 4363 | CA | ASP | B | 260 | 0.954 | -9.263 | 57.232 | 1.00 | 38.78 | C |
| ATOM | 4364 | CB | ASP | B | 260 | 0.446 | -10.705 | 57.065 | 1.00 | 39.54 | C |
| ATOM | 4365 | CG | ASP | B | 260 | 0.855 | -11.620 | 58.205 | 1.00 | 42.26 | C |
| ATOM | 4366 | OD1 | ASP | B | 260 | 1.015 | -11.127 | 59.350 | 1.00 | 43.47 | O |
| ATOM | 4367 | OD2 | ASP | B | 260 | 0.993 | -12.849 | 57.955 | 1.00 | 41.95 | O |
| ATOM | 4368 | C | ASP | B | 260 | 0.495 | -8.684 | 58.564 | 1.00 | 38.04 | C |
| ATOM | 4369 | O | ASP | B | 260 | -0.647 | -8.886 | 58.984 | 1.00 | 38.02 | O |
| ATOM | 4370 | N | SER | B | 261 | 1.397 | -7.965 | 59.225 | 1.00 | 36.68 | N |
| ATOM | 4371 | CA | SER | B | 261 | 1.096 | -7.326 | 60.507 | 1.00 | 35.64 | C |
| ATOM | 4372 | CB | SER | B | 261 | 0.731 | -8.370 | 61.572 | 1.00 | 35.11 | C |
| ATOM | 4373 | OG | SER | B | 261 | 1.872 | -9.012 | 62.100 | 1.00 | 34.85 | O |
| ATOM | 4374 | C | SER | B | 261 | 2.290 | -6.502 | 60.978 | 1.00 | 34.79 | C |
| ATOM | 4375 | O | SER | B | 261 | 3.391 | -6.630 | 60.442 | 1.00 | 35.37 | O |
| ATOM | 4376 | N | GLY | B | 262 | 2.064 | -5.659 | 61.979 | 1.00 | 32.94 | N |
| ATOM | 4377 | CA | GLY | B | 262 | 3.121 | -4.813 | 62.492 | 1.00 | 31.29 | C |
| ATOM | 4378 | C | GLY | B | 262 | 4.349 | -5.574 | 62.932 | 1.00 | 31.51 | C |
| ATOM | 4379 | O | GLY | B | 262 | 5.470 | -5.094 | 62.763 | 1.00 | 31.68 | O |
| ATOM | 4380 | N | VAL | B | 263 | 4.145 | -6.764 | 63.489 | 1.00 | 33.18 | N |
| ATOM | 4381 | CA | VAL | B | 263 | 5.251 | -7.589 | 63.974 | 1.00 | 34.60 | C |
| ATOM | 4382 | CB | VAL | B | 263 | 4.819 | -8.436 | 65.191 | 1.00 | 34.75 | C |
| ATOM | 4383 | CG1 | VAL | B | 263 | 5.973 | -9.309 | 65.661 | 1.00 | 31.59 | C |
| ATOM | 4384 | CG2 | VAL | B | 263 | 4.357 | -7.519 | 66.306 | 1.00 | 34.33 | C |
| ATOM | 4385 | C | VAL | B | 263 | 5.834 | -8.515 | 62.911 | 1.00 | 35.59 | C |
| ATOM | 4386 | O | VAL | B | 263 | 7.028 | -8.820 | 62.934 | 1.00 | 35.75 | O |
| ATOM | 4387 | N | ASP | B | 264 | 4.996 | -8.979 | 61.990 | 1.00 | 36.31 | N |
| ATOM | 4388 | CA | ASP | B | 264 | 5.484 | -9.852 | 60.927 | 1.00 | 37.35 | C |
| ATOM | 4389 | CB | ASP | B | 264 | 4.308 | -10.479 | 60.162 | 1.00 | 40.71 | C |
| ATOM | 4390 | CG | ASP | B | 264 | 4.746 | -11.262 | 58.923 | 1.00 | 43.93 | C |
| ATOM | 4391 | OD1 | ASP | B | 264 | 4.003 | -11.214 | 57.912 | 1.00 | 43.70 | O |
| ATOM | 4392 | OD2 | ASP | B | 264 | 5.812 | -11.926 | 58.957 | 1.00 | 44.93 | O |
| ATOM | 4393 | C | ASP | B | 264 | 6.322 | -8.976 | 59.995 | 1.00 | 37.38 | C |
| ATOM | 4394 | O | ASP | B | 264 | 7.211 | -9.467 | 59.304 | 1.00 | 37.71 | O |
| ATOM | 4395 | N | GLN | B | 265 | 6.040 | -7.672 | 59.985 | 1.00 | 36.30 | N |
| ATOM | 4396 | CA | GLN | B | 265 | 6.788 | -6.763 | 59.129 | 1.00 | 36.87 | C |
| ATOM | 4397 | CB | GLN | B | 265 | 6.325 | -5.321 | 59.317 | 1.00 | 35.74 | C |
| ATOM | 4398 | CG | GLN | B | 265 | 6.986 | -4.323 | 58.362 | 1.00 | 34.59 | C |
| ATOM | 4399 | CD | GLN | B | 265 | 6.818 | -4.701 | 56.898 | 1.00 | 33.30 | C |
| ATOM | 4400 | OE1 | GLN | B | 265 | 5.810 | -5.284 | 56.512 | 1.00 | 33.42 | O |
| ATOM | 4401 | NE2 | GLN | B | 265 | 7.799 | -4.351 | 56.078 | 1.00 | 31.34 | N |
| ATOM | 4402 | C | GLN | B | 265 | 8.280 | -6.860 | 59.421 | 1.00 | 37.56 | C |
| ATOM | 4403 | O | GLN | B | 265 | 9.098 | -6.751 | 58.515 | 1.00 | 39.37 | O |
| ATOM | 4404 | N | LEU | B | 266 | 8.633 | -7.064 | 60.684 | 1.00 | 37.85 | N |
| ATOM | 4405 | CA | LEU | B | 266 | 10.030 | -7.185 | 61.049 | 1.00 | 37.82 | C |
| ATOM | 4406 | CB | LEU | B | 266 | 10.189 | -7.096 | 62.564 | 1.00 | 36.90 | C |
| ATOM | 4407 | CG | LEU | B | 266 | 11.574 | -7.275 | 63.206 | 1.00 | 36.01 | C |
| ATOM | 4408 | CD1 | LEU | B | 266 | 12.600 | -6.333 | 62.582 | 1.00 | 34.17 | C |
| ATOM | 4409 | CD2 | LEU | B | 266 | 11.448 | -7.005 | 64.706 | 1.00 | 33.21 | C |
| ATOM | 4410 | C | LEU | B | 266 | 10.544 | -8.526 | 60.547 | 1.00 | 38.97 | C |
| ATOM | 4411 | O | LEU | B | 266 | 11.684 | -8.638 | 60.088 | 1.00 | 38.75 | O |
| ATOM | 4412 | N | VAL | B | 267 | 9.696 | -9.544 | 60.621 | 1.00 | 38.99 | N |
| ATOM | 4413 | CA | VAL | B | 267 | 10.092 | -10.867 | 60.168 | 1.00 | 39.24 | C |
| ATOM | 4414 | CB | VAL | B | 267 | 8.969 | -11.895 | 60.389 | 1.00 | 38.98 | C |
| ATOM | 4415 | CG1 | VAL | B | 267 | 9.363 | -13.233 | 59.783 | 1.00 | 37.96 | C |

| | | | | | | | | | | | |
|---|---|---|---|---|---|---|---|---|---|---|---|
| ATOM | 4416 | CG2 | VAL | B | 267 | 8.700 | -12.041 | 61.870 | 1.00 | 37.90 | C |
| ATOM | 4417 | C | VAL | B | 267 | 10.455 | -10.834 | 58.692 | 1.00 | 40.20 | C |
| ATOM | 4418 | O | VAL | B | 267 | 11.443 | -11.439 | 58.280 | 1.00 | 42.35 | O |
| ATOM | 4419 | N | GLU | B | 268 | 9.658 | -10.124 | 57.899 | 1.00 | 39.06 | N |
| ATOM | 4420 | CA | GLU | B | 268 | 9.905 | -10.015 | 56.465 | 1.00 | 37.12 | C |
| ATOM | 4421 | CB | GLU | B | 268 | 8.748 | -9.281 | 55.782 | 1.00 | 37.01 | C |
| ATOM | 4422 | CG | GLU | B | 268 | 7.480 | -10.109 | 55.638 | 1.00 | 36.42 | C |
| ATOM | 4423 | CD | GLU | B | 268 | 7.714 | -11.408 | 54.882 | 1.00 | 35.76 | C |
| ATOM | 4424 | OE1 | GLU | B | 268 | 8.248 | -11.363 | 53.756 | 1.00 | 35.58 | O |
| ATOM | 4425 | OE2 | GLU | B | 268 | 7.363 | -12.478 | 55.416 | 1.00 | 36.79 | O |
| ATOM | 4426 | C | GLU | B | 268 | 11.213 | -9.285 | 56.186 | 1.00 | 36.41 | C |
| ATOM | 4427 | O | GLU | B | 268 | 11.907 | -9.591 | 55.215 | 1.00 | 37.46 | O |
| ATOM | 4428 | N | ILE | B | 269 | 11.534 | -8.318 | 57.041 | 1.00 | 36.06 | N |
| ATOM | 4429 | CA | ILE | B | 269 | 12.753 | -7.535 | 56.917 | 1.00 | 35.67 | C |
| ATOM | 4430 | CB | ILE | B | 269 | 12.751 | -6.344 | 57.903 | 1.00 | 34.51 | C |
| ATOM | 4431 | CG2 | ILE | B | 269 | 14.186 | -5.911 | 58.210 | 1.00 | 31.65 | C |
| ATOM | 4432 | CG1 | ILE | B | 269 | 11.905 | -5.199 | 57.332 | 1.00 | 31.59 | C |
| ATOM | 4433 | CD1 | ILE | B | 269 | 11.661 | -4.053 | 58.309 | 1.00 | 29.91 | C |
| ATOM | 4434 | C | ILE | B | 269 | 13.952 | -8.419 | 57.219 | 1.00 | 37.16 | C |
| ATOM | 4435 | O | ILE | B | 269 | 14.960 | -8.392 | 56.511 | 1.00 | 38.00 | O |
| ATOM | 4436 | N | ILE | B | 270 | 13.829 | -9.212 | 58.274 | 1.00 | 38.37 | N |
| ATOM | 4437 | CA | ILE | B | 270 | 14.904 | -10.097 | 58.682 | 1.00 | 39.35 | C |
| ATOM | 4438 | CB | ILE | B | 270 | 14.569 | -10.747 | 60.044 | 1.00 | 38.58 | C |
| ATOM | 4439 | CG2 | ILE | B | 270 | 15.466 | -11.946 | 60.307 | 1.00 | 37.61 | C |
| ATOM | 4440 | CG1 | ILE | B | 270 | 14.735 | -9.699 | 61.147 | 1.00 | 38.57 | C |
| ATOM | 4441 | CD1 | ILE | B | 270 | 14.379 | -10.194 | 62.532 | 1.00 | 37.78 | C |
| ATOM | 4442 | C | ILE | B | 270 | 15.222 | -11.160 | 57.633 | 1.00 | 39.60 | C |
| ATOM | 4443 | O | ILE | B | 270 | 16.363 | -11.614 | 57.532 | 1.00 | 38.90 | O |
| ATOM | 4444 | N | LYS | B | 271 | 14.224 | -11.528 | 56.833 | 1.00 | 41.23 | N |
| ATOM | 4445 | CA | LYS | B | 271 | 14.406 | -12.542 | 55.792 | 1.00 | 41.71 | C |
| ATOM | 4446 | CB | LYS | B | 271 | 13.056 | -12.962 | 55.220 | 1.00 | 40.18 | C |
| ATOM | 4447 | CG | LYS | B | 271 | 12.168 | -13.665 | 56.214 | 1.00 | 41.93 | C |
| ATOM | 4448 | CD | LYS | B | 271 | 10.745 | -13.811 | 55.706 | 1.00 | 44.01 | C |
| ATOM | 4449 | CE | LYS | B | 271 | 10.658 | -14.698 | 54.478 | 1.00 | 44.53 | C |
| ATOM | 4450 | NZ | LYS | B | 271 | 9.271 | -15.222 | 54.306 | 1.00 | 46.31 | N |
| ATOM | 4451 | C | LYS | B | 271 | 15.328 | -12.129 | 54.639 | 1.00 | 43.18 | C |
| ATOM | 4452 | O | LYS | B | 271 | 15.571 | -12.925 | 53.732 | 1.00 | 44.62 | O |
| ATOM | 4453 | N | VAL | B | 272 | 15.819 | -10.889 | 54.646 | 1.00 | 42.89 | N |
| ATOM | 4454 | CA | VAL | B | 272 | 16.747 | -10.447 | 53.602 | 1.00 | 42.21 | C |
| ATOM | 4455 | CB | VAL | B | 272 | 16.084 | -9.507 | 52.506 | 1.00 | 41.99 | C |
| ATOM | 4456 | CG1 | VAL | B | 272 | 14.635 | -9.894 | 52.269 | 1.00 | 40.07 | C |
| ATOM | 4457 | CG2 | VAL | B | 272 | 16.217 | -8.049 | 52.890 | 1.00 | 44.10 | C |
| ATOM | 4458 | C | VAL | B | 272 | 17.918 | -9.729 | 54.271 | 1.00 | 42.05 | C |
| ATOM | 4459 | O | VAL | B | 272 | 19.078 | -10.009 | 53.966 | 1.00 | 42.38 | O |
| ATOM | 4460 | N | LEU | B | 273 | 17.632 | -8.824 | 55.200 | 1.00 | 42.49 | N |
| ATOM | 4461 | CA | LEU | B | 273 | 18.718 | -8.137 | 55.885 | 1.00 | 42.99 | C |
| ATOM | 4462 | CB | LEU | B | 273 | 18.226 | -6.879 | 56.607 | 1.00 | 42.95 | C |
| ATOM | 4463 | CG | LEU | B | 273 | 17.565 | -5.744 | 55.805 | 1.00 | 45.84 | C |
| ATOM | 4464 | CD1 | LEU | B | 273 | 17.753 | -4.430 | 56.561 | 1.00 | 44.28 | C |
| ATOM | 4465 | CD2 | LEU | B | 273 | 18.168 | -5.634 | 54.413 | 1.00 | 45.00 | C |
| ATOM | 4466 | C | LEU | B | 273 | 19.338 | -9.084 | 56.901 | 1.00 | 43.27 | C |
| ATOM | 4467 | O | LEU | B | 273 | 20.408 | -8.819 | 57.455 | 1.00 | 43.66 | O |
| ATOM | 4468 | N | GLY | B | 274 | 18.660 | -10.202 | 57.137 | 1.00 | 43.07 | N |
| ATOM | 4469 | CA | GLY | B | 274 | 19.149 | -11.167 | 58.101 | 1.00 | 43.10 | C |
| ATOM | 4470 | C | GLY | B | 274 | 18.848 | -10.669 | 59.500 | 1.00 | 43.54 | C |
| ATOM | 4471 | O | GLY | B | 274 | 18.243 | -9.608 | 59.667 | 1.00 | 43.64 | O |
| ATOM | 4472 | N | THR | B | 275 | 19.265 | -11.419 | 60.512 | 1.00 | 43.14 | N |
| ATOM | 4473 | CA | THR | B | 275 | 19.011 | -11.017 | 61.894 | 1.00 | 43.02 | C |
| ATOM | 4474 | CB | THR | B | 275 | 19.240 | -12.202 | 62.854 | 1.00 | 42.45 | C |
| ATOM | 4475 | OG1 | THR | B | 275 | 18.443 | -13.316 | 62.427 | 1.00 | 42.01 | O |
| ATOM | 4476 | CG2 | THR | B | 275 | 18.832 | -11.830 | 64.269 | 1.00 | 41.81 | C |
| ATOM | 4477 | C | THR | B | 275 | 19.858 | -9.823 | 62.349 | 1.00 | 43.36 | C |
| ATOM | 4478 | O | THR | B | 275 | 21.009 | -9.672 | 61.940 | 1.00 | 44.09 | O |
| ATOM | 4479 | N | PRO | B | 276 | 19.275 | -8.937 | 63.174 | 1.00 | 42.95 | N |
| ATOM | 4480 | CD | PRO | B | 276 | 17.838 | -8.877 | 63.494 | 1.00 | 42.51 | C |
| ATOM | 4481 | CA | PRO | B | 276 | 19.972 | -7.753 | 63.694 | 1.00 | 43.57 | C |
| ATOM | 4482 | CB | PRO | B | 276 | 18.845 | -6.920 | 64.314 | 1.00 | 42.41 | C |

| | | | | | | | | | | | |
|---|---|---|---|---|---|---|---|---|---|---|---|
| ATOM | 4483 | CG | PRO | B | 276 | 17.604 | -7.405 | 63.635 | 1.00 | 42.64 | C |
| ATOM | 4484 | C | PRO | B | 276 | 20.991 | -8.164 | 64.765 | 1.00 | 44.49 | C |
| ATOM | 4485 | O | PRO | B | 276 | 20.715 | -9.053 | 65.579 | 1.00 | 43.18 | O |
| ATOM | 4486 | N | THR | B | 277 | 22.158 | -7.524 | 64.775 | 1.00 | 45.69 | N |
| ATOM | 4487 | CA | THR | B | 277 | 23.174 | -7.834 | 65.779 | 1.00 | 46.99 | C |
| ATOM | 4488 | CB | THR | B | 277 | 24.591 | -7.338 | 65.366 | 1.00 | 47.11 | C |
| ATOM | 4489 | OG1 | THR | B | 277 | 24.655 | -5.906 | 65.449 | 1.00 | 47.54 | O |
| ATOM | 4490 | CG2 | THR | B | 277 | 24.920 | -7.775 | 63.945 | 1.00 | 45.63 | C |
| ATOM | 4491 | C | THR | B | 277 | 22.759 | -7.109 | 67.047 | 1.00 | 48.96 | C |
| ATOM | 4492 | O | THR | B | 277 | 21.942 | -6.181 | 67.000 | 1.00 | 49.62 | O |
| ATOM | 4493 | N | ALA | B | 278 | 23.307 | -7.530 | 68.184 | 1.00 | 51.14 | N |
| ATOM | 4494 | CA | ALA | B | 278 | 22.963 | -6.897 | 69.460 | 1.00 | 50.99 | C |
| ATOM | 4495 | CB | ALA | B | 278 | 23.839 | -7.450 | 70.573 | 1.00 | 51.45 | C |
| ATOM | 4496 | C | ALA | B | 278 | 23.110 | -5.380 | 69.378 | 1.00 | 51.54 | C |
| ATOM | 4497 | O | ALA | B | 278 | 22.213 | -4.644 | 69.801 | 1.00 | 51.77 | O |
| ATOM | 4498 | N | GLU | B | 279 | 24.229 | -4.907 | 68.831 | 1.00 | 51.10 | N |
| ATOM | 4499 | CA | GLU | B | 279 | 24.431 | -3.467 | 68.721 | 1.00 | 52.97 | C |
| ATOM | 4500 | CB | GLU | B | 279 | 25.773 | -3.143 | 68.057 | 1.00 | 55.36 | C |
| ATOM | 4501 | CG | GLU | B | 279 | 26.938 | -3.120 | 69.028 | 1.00 | 60.01 | C |
| ATOM | 4502 | CD | GLU | B | 279 | 27.107 | -4.447 | 69.759 | 1.00 | 62.59 | C |
| ATOM | 4503 | OE1 | GLU | B | 279 | 26.777 | -4.509 | 70.975 | 1.00 | 62.56 | O |
| ATOM | 4504 | OE2 | GLU | B | 279 | 27.562 | -5.424 | 69.103 | 1.00 | 63.86 | O |
| ATOM | 4505 | C | GLU | B | 279 | 23.307 | -2.847 | 67.910 | 1.00 | 52.44 | C |
| ATOM | 4506 | O | GLU | B | 279 | 22.613 | -1.941 | 68.378 | 1.00 | 53.02 | O |
| ATOM | 4507 | N | GLN | B | 280 | 23.141 | -3.343 | 66.688 | 1.00 | 51.42 | N |
| ATOM | 4508 | CA | GLN | B | 280 | 22.102 | -2.864 | 65.784 | 1.00 | 49.69 | C |
| ATOM | 4509 | CB | GLN | B | 280 | 21.933 | -3.860 | 64.624 | 1.00 | 47.51 | C |
| ATOM | 4510 | CG | GLN | B | 280 | 23.040 | -3.763 | 63.568 | 1.00 | 44.50 | C |
| ATOM | 4511 | CD | GLN | B | 280 | 22.983 | -4.876 | 62.525 | 1.00 | 43.82 | C |
| ATOM | 4512 | OE1 | GLN | B | 280 | 23.562 | -4.762 | 61.438 | 1.00 | 43.36 | O |
| ATOM | 4513 | NE2 | GLN | B | 280 | 22.297 | -5.964 | 62.857 | 1.00 | 43.18 | N |
| ATOM | 4514 | C | GLN | B | 280 | 20.782 | -2.667 | 66.538 | 1.00 | 49.13 | C |
| ATOM | 4515 | O | GLN | B | 280 | 20.215 | -1.571 | 66.547 | 1.00 | 49.26 | O |
| ATOM | 4516 | N | ILE | B | 281 | 20.311 | -3.719 | 67.193 | 1.00 | 48.91 | N |
| ATOM | 4517 | CA | ILE | B | 281 | 19.065 | -3.643 | 67.947 | 1.00 | 50.32 | C |
| ATOM | 4518 | CB | ILE | B | 281 | 18.721 | -5.007 | 68.583 | 1.00 | 50.42 | C |
| ATOM | 4519 | CG2 | ILE | B | 281 | 17.572 | -4.850 | 69.582 | 1.00 | 51.06 | C |
| ATOM | 4520 | CG1 | ILE | B | 281 | 18.346 | -6.007 | 67.486 | 1.00 | 50.50 | C |
| ATOM | 4521 | CD1 | ILE | B | 281 | 17.835 | -7.339 | 68.011 | 1.00 | 50.46 | C |
| ATOM | 4522 | C | ILE | B | 281 | 19.100 | -2.577 | 69.049 | 1.00 | 50.33 | C |
| ATOM | 4523 | O | ILE | B | 281 | 18.055 | -2.154 | 69.564 | 1.00 | 49.90 | O |
| ATOM | 4524 | N | ALA | B | 282 | 20.306 | -2.143 | 69.411 | 1.00 | 51.24 | N |
| ATOM | 4525 | CA | ALA | B | 282 | 20.467 | -1.128 | 70.451 | 1.00 | 50.98 | C |
| ATOM | 4526 | CB | ALA | B | 282 | 21.827 | -1.303 | 71.163 | 1.00 | 50.49 | C |
| ATOM | 4527 | C | ALA | B | 282 | 20.323 | 0.304 | 69.916 | 1.00 | 49.85 | C |
| ATOM | 4528 | O | ALA | B | 282 | 19.588 | 1.110 | 70.492 | 1.00 | 48.67 | O |
| ATOM | 4529 | N | GLU | B | 283 | 21.018 | 0.627 | 68.828 | 1.00 | 49.29 | N |
| ATOM | 4530 | CA | GLU | B | 283 | 20.911 | 1.975 | 68.264 | 1.00 | 50.15 | C |
| ATOM | 4531 | CB | GLU | B | 283 | 21.961 | 2.188 | 67.169 | 1.00 | 50.42 | C |
| ATOM | 4532 | CG | GLU | B | 283 | 23.312 | 2.615 | 67.728 | 1.00 | 51.06 | C |
| ATOM | 4533 | CD | GLU | B | 283 | 24.477 | 2.193 | 66.858 | 1.00 | 52.41 | C |
| ATOM | 4534 | OE1 | GLU | B | 283 | 24.518 | 1.005 | 66.456 | 1.00 | 53.66 | O |
| ATOM | 4535 | OE2 | GLU | B | 283 | 25.360 | 3.038 | 66.587 | 1.00 | 53.71 | O |
| ATOM | 4536 | C | GLU | B | 283 | 19.506 | 2.264 | 67.726 | 1.00 | 49.69 | C |
| ATOM | 4537 | O | GLU | B | 283 | 19.169 | 3.409 | 67.424 | 1.00 | 49.12 | O |
| ATOM | 4538 | N | MET | B | 284 | 18.693 | 1.215 | 67.635 | 1.00 | 48.79 | N |
| ATOM | 4539 | CA | MET | B | 284 | 17.319 | 1.313 | 67.169 | 1.00 | 48.68 | C |
| ATOM | 4540 | CB | MET | B | 284 | 16.871 | -0.035 | 66.580 | 1.00 | 49.16 | C |
| ATOM | 4541 | CG | MET | B | 284 | 17.498 | -0.378 | 65.205 | 1.00 | 48.69 | C |
| ATOM | 4542 | SD | MET | B | 284 | 17.077 | -2.038 | 64.558 | 1.00 | 46.94 | S |
| ATOM | 4543 | CE | MET | B | 284 | 15.416 | -1.757 | 64.196 | 1.00 | 46.72 | C |
| ATOM | 4544 | C | MET | B | 284 | 16.378 | 1.729 | 68.303 | 1.00 | 49.44 | C |
| ATOM | 4545 | O | MET | B | 284 | 15.287 | 2.237 | 68.060 | 1.00 | 49.97 | O |
| ATOM | 4546 | N | GLY | B | 285 | 16.806 | 1.522 | 69.546 | 1.00 | 50.00 | N |
| ATOM | 4547 | CA | GLY | B | 285 | 15.983 | 1.894 | 70.683 | 1.00 | 49.92 | C |
| ATOM | 4548 | C | GLY | B | 285 | 15.287 | 0.696 | 71.283 | 1.00 | 50.55 | C |
| ATOM | 4549 | O | GLY | B | 285 | 15.940 | -0.281 | 71.659 | 1.00 | 52.02 | O |

| | | | | | | | | | | | |
|---|---|---|---|---|---|---|---|---|---|---|---|
| ATOM | 4550 | N | ALA | B | 296 | 17.042 | -15.838 | 59.123 | 1.00 | 45.19 | N |
| ATOM | 4551 | CA | ALA | B | 296 | 18.117 | -15.636 | 60.084 | 1.00 | 44.73 | C |
| ATOM | 4552 | CB | ALA | B | 296 | 18.371 | -16.929 | 60.868 | 1.00 | 43.55 | C |
| ATOM | 4553 | C | ALA | B | 296 | 19.402 | -15.145 | 59.400 | 1.00 | 45.18 | C |
| ATOM | 4554 | O | ALA | B | 296 | 19.900 | -14.070 | 59.731 | 1.00 | 45.00 | O |
| ATOM | 4555 | N | ALA | B | 297 | 19.934 | -15.914 | 58.451 | 1.00 | 45.65 | N |
| ATOM | 4556 | CA | ALA | B | 297 | 21.157 | -15.517 | 57.741 | 1.00 | 46.41 | C |
| ATOM | 4557 | CB | ALA | B | 297 | 21.892 | -16.748 | 57.216 | 1.00 | 44.43 | C |
| ATOM | 4558 | C | ALA | B | 297 | 20.857 | -14.559 | 56.583 | 1.00 | 47.67 | C |
| ATOM | 4559 | O | ALA | B | 297 | 20.034 | -14.861 | 55.716 | 1.00 | 47.66 | O |
| ATOM | 4560 | N | ALA | B | 298 | 21.532 | -13.408 | 56.568 | 1.00 | 47.41 | N |
| ATOM | 4561 | CA | ALA | B | 298 | 21.330 | -12.408 | 55.518 | 1.00 | 47.14 | C |
| ATOM | 4562 | CB | ALA | B | 298 | 22.351 | -11.284 | 55.652 | 1.00 | 45.92 | C |
| ATOM | 4563 | C | ALA | B | 298 | 21.410 | -13.014 | 54.125 | 1.00 | 48.10 | C |
| ATOM | 4564 | O | ALA | B | 298 | 22.292 | -13.824 | 53.832 | 1.00 | 48.15 | O |
| ATOM | 4565 | N | HIS | B | 299 | 20.478 | -12.611 | 53.266 | 1.00 | 49.91 | N |
| ATOM | 4566 | CA | HIS | B | 299 | 20.405 | -13.096 | 51.887 | 1.00 | 50.60 | C |
| ATOM | 4567 | CB | HIS | B | 299 | 18.935 | -13.306 | 51.524 | 1.00 | 51.52 | C |
| ATOM | 4568 | CG | HIS | B | 299 | 18.718 | -13.952 | 50.192 | 1.00 | 53.69 | C |
| ATOM | 4569 | CD2 | HIS | B | 299 | 18.912 | -15.229 | 49.776 | 1.00 | 54.03 | C |
| ATOM | 4570 | ND1 | HIS | B | 299 | 18.191 | -13.271 | 49.119 | 1.00 | 54.72 | N |
| ATOM | 4571 | CE1 | HIS | B | 299 | 18.062 | -14.101 | 48.092 | 1.00 | 54.52 | C |
| ATOM | 4572 | NE2 | HIS | B | 299 | 18.491 | -15.291 | 48.466 | 1.00 | 54.69 | N |
| ATOM | 4573 | C | HIS | B | 299 | 21.042 | -12.009 | 51.015 | 1.00 | 49.97 | C |
| ATOM | 4574 | O | HIS | B | 299 | 20.543 | -10.888 | 50.948 | 1.00 | 50.38 | O |
| ATOM | 4575 | N | PRO | B | 300 | 22.153 | -12.320 | 50.329 | 1.00 | 50.60 | N |
| ATOM | 4576 | CD | PRO | B | 300 | 23.010 | -13.517 | 50.359 | 1.00 | 50.71 | C |
| ATOM | 4577 | CA | PRO | B | 300 | 22.764 | -11.266 | 49.508 | 1.00 | 50.11 | C |
| ATOM | 4578 | CB | PRO | B | 300 | 23.983 | -11.961 | 48.874 | 1.00 | 49.45 | C |
| ATOM | 4579 | CG | PRO | B | 300 | 23.706 | -13.424 | 49.032 | 1.00 | 49.67 | C |
| ATOM | 4580 | C | PRO | B | 300 | 21.914 | -10.502 | 48.490 | 1.00 | 49.14 | C |
| ATOM | 4581 | O | PRO | B | 300 | 21.192 | -11.074 | 47.674 | 1.00 | 47.85 | O |
| ATOM | 4582 | N | TRP | B | 301 | 22.043 | -9.183 | 48.589 | 1.00 | 49.54 | N |
| ATOM | 4583 | CA | TRP | B | 301 | 21.382 | -8.182 | 47.755 | 1.00 | 50.01 | C |
| ATOM | 4584 | CB | TRP | B | 301 | 22.119 | -6.857 | 47.923 | 1.00 | 49.64 | C |
| ATOM | 4585 | CG | TRP | B | 301 | 21.702 | -6.178 | 49.140 | 1.00 | 50.57 | C |
| ATOM | 4586 | CD2 | TRP | B | 301 | 20.352 | -5.906 | 49.513 | 1.00 | 49.99 | C |
| ATOM | 4587 | CE2 | TRP | B | 301 | 20.398 | -5.266 | 50.772 | 1.00 | 50.25 | C |
| ATOM | 4588 | CE3 | TRP | B | 301 | 19.125 | -6.123 | 48.896 | 1.00 | 49.73 | C |
| ATOM | 4589 | CD1 | TRP | B | 301 | 22.489 | -5.721 | 50.153 | 1.00 | 51.99 | C |
| ATOM | 4590 | NE1 | TRP | B | 301 | 21.702 | -5.172 | 51.146 | 1.00 | 50.95 | N |
| ATOM | 4591 | CZ2 | TRP | B | 301 | 19.230 | -4.866 | 51.433 | 1.00 | 50.05 | C |
| ATOM | 4592 | CZ3 | TRP | B | 301 | 17.975 | -5.725 | 49.550 | 1.00 | 50.96 | C |
| ATOM | 4593 | CH2 | TRP | B | 301 | 18.033 | -5.092 | 50.806 | 1.00 | 50.30 | C |
| ATOM | 4594 | C | TRP | B | 301 | 21.293 | -8.503 | 46.283 | 1.00 | 49.96 | C |
| ATOM | 4595 | O | TRP | B | 301 | 20.209 | -8.564 | 45.697 | 1.00 | 50.17 | O |
| ATOM | 4596 | N | THR | B | 302 | 22.464 | -8.646 | 45.688 | 1.00 | 50.52 | N |
| ATOM | 4597 | CA | THR | B | 302 | 22.588 | -8.962 | 44.283 | 1.00 | 50.77 | C |
| ATOM | 4598 | CB | THR | B | 302 | 24.033 | -9.377 | 43.974 | 1.00 | 50.09 | C |
| ATOM | 4599 | OG1 | THR | B | 302 | 24.318 | -10.621 | 44.627 | 1.00 | 49.14 | O |
| ATOM | 4600 | CG2 | THR | B | 302 | 25.006 | -8.317 | 44.509 | 1.00 | 49.69 | C |
| ATOM | 4601 | C | THR | B | 302 | 21.625 | -10.092 | 43.928 | 1.00 | 50.73 | C |
| ATOM | 4602 | O | THR | B | 302 | 21.038 | -10.094 | 42.848 | 1.00 | 51.54 | O |
| ATOM | 4603 | N | ALA | B | 303 | 21.448 | -11.040 | 44.845 | 1.00 | 49.53 | N |
| ATOM | 4604 | CA | ALA | B | 303 | 20.543 | -12.165 | 44.604 | 1.00 | 48.46 | C |
| ATOM | 4605 | CB | ALA | B | 303 | 21.005 | -13.394 | 45.404 | 1.00 | 48.36 | C |
| ATOM | 4606 | C | ALA | B | 303 | 19.078 | -11.852 | 44.926 | 1.00 | 47.96 | C |
| ATOM | 4607 | O | ALA | B | 303 | 18.190 | -12.653 | 44.636 | 1.00 | 46.95 | O |
| ATOM | 4608 | N | VAL | B | 304 | 18.829 | -10.694 | 45.532 | 1.00 | 48.86 | N |
| ATOM | 4609 | CA | VAL | B | 304 | 17.468 | -10.274 | 45.875 | 1.00 | 48.23 | C |
| ATOM | 4610 | CB | VAL | B | 304 | 17.469 | -9.292 | 47.076 | 1.00 | 48.41 | C |
| ATOM | 4611 | CG1 | VAL | B | 304 | 16.070 | -8.736 | 47.301 | 1.00 | 48.99 | C |
| ATOM | 4612 | CG2 | VAL | B | 304 | 17.951 | -10.004 | 48.331 | 1.00 | 48.36 | C |
| ATOM | 4613 | C | VAL | B | 304 | 16.793 | -9.585 | 44.680 | 1.00 | 48.76 | C |
| ATOM | 4614 | O | VAL | B | 304 | 15.588 | -9.714 | 44.478 | 1.00 | 47.36 | O |
| ATOM | 4615 | N | PHE | B | 305 | 17.589 | -8.866 | 43.888 | 1.00 | 49.84 | N |
| ATOM | 4616 | CA | PHE | B | 305 | 17.082 | -8.139 | 42.726 | 1.00 | 51.00 | C |

| | | | | | | | | | | | |
|---|---|---|---|---|---|---|---|---|---|---|---|
| ATOM | 4617 | CB | PHE | B | 305 | 17.799 | -6.794 | 42.614 | 1.00 | 49.06 | C |
| ATOM | 4618 | CG | PHE | B | 305 | 17.486 | -5.858 | 43.744 | 1.00 | 48.64 | C |
| ATOM | 4619 | CD1 | PHE | B | 305 | 16.232 | -5.280 | 43.850 | 1.00 | 47.03 | C |
| ATOM | 4620 | CD2 | PHE | B | 305 | 18.427 | -5.589 | 44.730 | 1.00 | 48.67 | C |
| ATOM | 4621 | CE1 | PHE | B | 305 | 15.912 | -4.449 | 44.922 | 1.00 | 46.99 | C |
| ATOM | 4622 | CE2 | PHE | B | 305 | 18.116 | -4.757 | 45.807 | 1.00 | 48.23 | C |
| ATOM | 4623 | CZ | PHE | B | 305 | 16.855 | -4.186 | 45.903 | 1.00 | 46.07 | C |
| ATOM | 4624 | C | PHE | B | 305 | 17.203 | -8.910 | 41.410 | 1.00 | 52.78 | C |
| ATOM | 4625 | O | PHE | B | 305 | 18.137 | -9.686 | 41.218 | 1.00 | 52.33 | O |
| ATOM | 4626 | N | ARG | B | 306 | 16.253 | -8.686 | 40.502 | 1.00 | 53.82 | N |
| ATOM | 4627 | CA | ARG | B | 306 | 16.265 | -9.373 | 39.223 | 1.00 | 54.61 | C |
| ATOM | 4628 | CB | ARG | B | 306 | 15.257 | -8.746 | 38.254 | 1.00 | 56.38 | C |
| ATOM | 4629 | CG | ARG | B | 306 | 15.751 | -7.484 | 37.577 | 1.00 | 58.31 | C |
| ATOM | 4630 | CD | ARG | B | 306 | 14.892 | -7.113 | 36.386 | 1.00 | 59.87 | C |
| ATOM | 4631 | NE | ARG | B | 306 | 15.530 | -6.095 | 35.551 | 1.00 | 61.60 | N |
| ATOM | 4632 | CZ | ARG | B | 306 | 15.824 | -4.861 | 35.951 | 1.00 | 62.04 | C |
| ATOM | 4633 | NH1 | ARG | B | 306 | 15.539 | -4.478 | 37.195 | 1.00 | 61.82 | N |
| ATOM | 4634 | NH2 | ARG | B | 306 | 16.396 | -4.014 | 35.098 | 1.00 | 59.97 | N |
| ATOM | 4635 | C | ARG | B | 306 | 17.663 | -9.308 | 38.614 | 1.00 | 54.76 | C |
| ATOM | 4636 | O | ARG | B | 306 | 18.366 | -8.306 | 38.747 | 1.00 | 53.75 | O |
| ATOM | 4637 | N | PRO | B | 307 | 18.074 | -10.388 | 37.933 | 1.00 | 55.26 | N |
| ATOM | 4638 | CD | PRO | B | 307 | 17.220 | -11.576 | 37.738 | 1.00 | 55.29 | C |
| ATOM | 4639 | CA | PRO | B | 307 | 19.368 | -10.565 | 37.263 | 1.00 | 55.20 | C |
| ATOM | 4640 | CB | PRO | B | 307 | 19.061 | -11.632 | 36.225 | 1.00 | 54.82 | C |
| ATOM | 4641 | CG | PRO | B | 307 | 18.140 | -12.527 | 36.978 | 1.00 | 55.78 | C |
| ATOM | 4642 | C | PRO | B | 307 | 19.989 | -9.318 | 36.643 | 1.00 | 54.87 | C |
| ATOM | 4643 | O | PRO | B | 307 | 21.143 | -8.988 | 36.918 | 1.00 | 54.95 | O |
| ATOM | 4644 | N | ALA | B | 308 | 19.230 | -8.626 | 35.803 | 1.00 | 54.62 | N |
| ATOM | 4645 | CA | ALA | B | 308 | 19.750 | -7.429 | 35.135 | 1.00 | 54.66 | C |
| ATOM | 4646 | CB | ALA | B | 308 | 19.219 | -7.369 | 33.693 | 1.00 | 55.31 | C |
| ATOM | 4647 | C | ALA | B | 308 | 19.478 | -6.096 | 35.855 | 1.00 | 53.91 | C |
| ATOM | 4648 | O | ALA | B | 308 | 19.154 | -5.092 | 35.219 | 1.00 | 53.49 | O |
| ATOM | 4649 | N | THR | B | 309 | 19.615 | -6.094 | 37.181 | 1.00 | 51.56 | N |
| ATOM | 4650 | CA | THR | B | 309 | 19.413 | -4.888 | 37.978 | 1.00 | 48.04 | C |
| ATOM | 4651 | CB | THR | B | 309 | 18.927 | -5.244 | 39.407 | 1.00 | 47.78 | C |
| ATOM | 4652 | OG1 | THR | B | 309 | 17.656 | -5.902 | 39.335 | 1.00 | 45.97 | O |
| ATOM | 4653 | CG2 | THR | B | 309 | 18.793 | -3.988 | 40.265 | 1.00 | 47.29 | C |
| ATOM | 4654 | C | THR | B | 309 | 20.745 | -4.132 | 38.060 | 1.00 | 47.31 | C |
| ATOM | 4655 | O | THR | B | 309 | 21.764 | -4.709 | 38.421 | 1.00 | 47.54 | O |
| ATOM | 4656 | N | PRO | B | 310 | 20.752 | -2.833 | 37.715 | 1.00 | 45.72 | N |
| ATOM | 4657 | CD | PRO | B | 310 | 19.589 | -2.031 | 37.298 | 1.00 | 45.14 | C |
| ATOM | 4658 | CA | PRO | B | 310 | 21.971 | -2.009 | 37.754 | 1.00 | 44.82 | C |
| ATOM | 4659 | CB | PRO | B | 310 | 21.463 | -0.615 | 37.383 | 1.00 | 44.51 | C |
| ATOM | 4660 | CG | PRO | B | 310 | 20.245 | -0.906 | 36.537 | 1.00 | 44.84 | C |
| ATOM | 4661 | C | PRO | B | 310 | 22.682 | -2.008 | 39.117 | 1.00 | 44.80 | C |
| ATOM | 4662 | O | PRO | B | 310 | 22.111 | -1.602 | 40.130 | 1.00 | 44.44 | O |
| ATOM | 4663 | N | PRO | B | 311 | 23.953 | -2.439 | 39.146 | 1.00 | 44.41 | N |
| ATOM | 4664 | CD | PRO | B | 311 | 24.765 | -2.734 | 37.949 | 1.00 | 44.42 | C |
| ATOM | 4665 | CA | PRO | B | 311 | 24.774 | -2.504 | 40.362 | 1.00 | 42.07 | C |
| ATOM | 4666 | CB | PRO | B | 311 | 26.194 | -2.591 | 39.807 | 1.00 | 43.64 | C |
| ATOM | 4667 | CG | PRO | B | 311 | 26.001 | -3.366 | 38.542 | 1.00 | 43.57 | C |
| ATOM | 4668 | C | PRO | B | 311 | 24.594 | -1.306 | 41.288 | 1.00 | 39.53 | C |
| ATOM | 4669 | O | PRO | B | 311 | 24.484 | -1.462 | 42.500 | 1.00 | 38.56 | O |
| ATOM | 4670 | N | GLU | B | 312 | 24.566 | -0.116 | 40.701 | 1.00 | 37.92 | N |
| ATOM | 4671 | CA | GLU | B | 312 | 24.403 | 1.120 | 41.456 | 1.00 | 37.67 | C |
| ATOM | 4672 | CB | GLU | B | 312 | 24.644 | 2.340 | 40.555 | 1.00 | 39.24 | C |
| ATOM | 4673 | CG | GLU | B | 312 | 26.012 | 2.385 | 39.881 | 1.00 | 41.99 | C |
| ATOM | 4674 | CD | GLU | B | 312 | 26.114 | 1.463 | 38.666 | 1.00 | 46.16 | C |
| ATOM | 4675 | OE1 | GLU | B | 312 | 25.177 | 0.661 | 38.424 | 1.00 | 48.46 | O |
| ATOM | 4676 | OE2 | GLU | B | 312 | 27.138 | 1.532 | 37.939 | 1.00 | 46.18 | O |
| ATOM | 4677 | C | GLU | B | 312 | 23.024 | 1.239 | 42.115 | 1.00 | 37.21 | C |
| ATOM | 4678 | O | GLU | B | 312 | 22.872 | 1.925 | 43.115 | 1.00 | 38.03 | O |
| ATOM | 4679 | N | ALA | B | 313 | 22.013 | 0.594 | 41.554 | 1.00 | 36.15 | N |
| ATOM | 4680 | CA | ALA | B | 313 | 20.697 | 0.664 | 42.159 | 1.00 | 36.28 | C |
| ATOM | 4681 | CB | ALA | B | 313 | 19.632 | 0.101 | 41.213 | 1.00 | 36.45 | C |
| ATOM | 4682 | C | ALA | B | 313 | 20.779 | -0.182 | 43.415 | 1.00 | 36.87 | C |
| ATOM | 4683 | O | ALA | B | 313 | 20.308 | 0.207 | 44.483 | 1.00 | 37.47 | O |

| | | | | | | | | | | | |
|---|---|---|---|---|---|---|---|---|---|---|---|
| ATOM | 4684 | N | ILE | B | 314 | 21.394 | -1.350 | 43.273 | 1.00 | 37.72 | N |
| ATOM | 4685 | CA | ILE | B | 314 | 21.557 | -2.293 | 44.366 | 1.00 | 37.26 | C |
| ATOM | 4686 | CB | ILE | B | 314 | 22.153 | -3.630 | 43.842 | 1.00 | 38.25 | C |
| ATOM | 4687 | CG2 | ILE | B | 314 | 22.409 | -4.583 | 45.001 | 1.00 | 36.53 | C |
| ATOM | 4688 | CG1 | ILE | B | 314 | 21.197 | -4.261 | 42.820 | 1.00 | 38.03 | C |
| ATOM | 4689 | CD1 | ILE | B | 314 | 21.662 | -5.589 | 42.245 | 1.00 | 34.78 | C |
| ATOM | 4690 | C | ILE | B | 314 | 22.451 | -1.738 | 45.474 | 1.00 | 36.65 | C |
| ATOM | 4691 | O | ILE | B | 314 | 22.208 | -2.003 | 46.641 | 1.00 | 37.87 | O |
| ATOM | 4692 | N | ALA | B | 315 | 23.478 | -0.976 | 45.112 | 1.00 | 36.09 | N |
| ATOM | 4693 | CA | ALA | B | 315 | 24.393 | -0.401 | 46.099 | 1.00 | 35.89 | C |
| ATOM | 4694 | CB | ALA | B | 315 | 25.636 | 0.149 | 45.408 | 1.00 | 34.82 | C |
| ATOM | 4695 | C | ALA | B | 315 | 23.727 | 0.702 | 46.908 | 1.00 | 36.99 | C |
| ATOM | 4696 | O | ALA | B | 315 | 23.934 | 0.824 | 48.119 | 1.00 | 37.15 | O |
| ATOM | 4697 | N | LEU | B | 316 | 22.942 | 1.525 | 46.223 | 1.00 | 37.29 | N |
| ATOM | 4698 | CA | LEU | B | 316 | 22.226 | 2.621 | 46.872 | 1.00 | 36.95 | C |
| ATOM | 4699 | CB | LEU | B | 316 | 21.559 | 3.520 | 45.828 | 1.00 | 34.01 | C |
| ATOM | 4700 | CG | LEU | B | 316 | 20.396 | 4.401 | 46.258 | 1.00 | 33.70 | C |
| ATOM | 4701 | CD1 | LEU | B | 316 | 20.842 | 5.432 | 47.279 | 1.00 | 33.38 | C |
| ATOM | 4702 | CD2 | LEU | B | 316 | 19.827 | 5.071 | 45.025 | 1.00 | 33.57 | C |
| ATOM | 4703 | C | LEU | B | 316 | 21.160 | 2.035 | 47.774 | 1.00 | 37.75 | C |
| ATOM | 4704 | O | LEU | B | 316 | 20.928 | 2.513 | 48.884 | 1.00 | 39.86 | O |
| ATOM | 4705 | N | CYS | B | 317 | 20.519 | 0.985 | 47.289 | 1.00 | 37.10 | N |
| ATOM | 4706 | CA | CYS | B | 317 | 19.462 | 0.364 | 48.046 | 1.00 | 37.87 | C |
| ATOM | 4707 | CB | CYS | B | 317 | 18.816 | -0.747 | 47.218 | 1.00 | 38.36 | C |
| ATOM | 4708 | SG | CYS | B | 317 | 17.252 | -1.311 | 47.892 | 1.00 | 41.58 | S |
| ATOM | 4709 | C | CYS | B | 317 | 19.982 | -0.180 | 49.374 | 1.00 | 37.43 | C |
| ATOM | 4710 | O | CYS | B | 317 | 19.336 | -0.032 | 50.415 | 1.00 | 36.59 | O |
| ATOM | 4711 | N | SER | B | 318 | 21.159 | -0.792 | 49.338 | 1.00 | 37.34 | N |
| ATOM | 4712 | CA | SER | B | 318 | 21.771 | -1.366 | 50.537 | 1.00 | 37.44 | C |
| ATOM | 4713 | CB | SER | B | 318 | 23.037 | -2.135 | 50.155 | 1.00 | 36.92 | C |
| ATOM | 4714 | OG | SER | B | 318 | 23.969 | -1.270 | 49.524 | 1.00 | 39.22 | O |
| ATOM | 4715 | C | SER | B | 318 | 22.131 | -0.325 | 51.596 | 1.00 | 36.74 | C |
| ATOM | 4716 | O | SER | B | 318 | 22.078 | -0.605 | 52.795 | 1.00 | 37.91 | O |
| ATOM | 4717 | N | ARG | B | 319 | 22.510 | 0.869 | 51.155 | 1.00 | 37.32 | N |
| ATOM | 4718 | CA | ARG | B | 319 | 22.902 | 1.934 | 52.074 | 1.00 | 37.62 | C |
| ATOM | 4719 | CB | ARG | B | 319 | 23.926 | 2.820 | 51.385 | 1.00 | 37.78 | C |
| ATOM | 4720 | CG | ARG | B | 319 | 25.184 | 2.054 | 51.057 | 1.00 | 40.92 | C |
| ATOM | 4721 | CD | ARG | B | 319 | 25.988 | 1.791 | 52.318 | 1.00 | 41.76 | C |
| ATOM | 4722 | NE | ARG | B | 319 | 26.687 | 3.011 | 52.704 | 1.00 | 44.22 | N |
| ATOM | 4723 | CZ | ARG | B | 319 | 26.362 | 3.764 | 53.745 | 1.00 | 45.42 | C |
| ATOM | 4724 | NH1 | ARG | B | 319 | 25.342 | 3.415 | 54.523 | 1.00 | 45.95 | N |
| ATOM | 4725 | NH2 | ARG | B | 319 | 27.047 | 4.875 | 53.994 | 1.00 | 46.01 | N |
| ATOM | 4726 | C | ARG | B | 319 | 21.726 | 2.759 | 52.583 | 1.00 | 37.47 | C |
| ATOM | 4727 | O | ARG | B | 319 | 21.902 | 3.773 | 53.266 | 1.00 | 37.59 | O |
| ATOM | 4728 | N | LEU | B | 320 | 20.525 | 2.308 | 52.234 | 1.00 | 37.20 | N |
| ATOM | 4729 | CA | LEU | B | 320 | 19.289 | 2.946 | 52.650 | 1.00 | 34.71 | C |
| ATOM | 4730 | CB | LEU | B | 320 | 18.361 | 3.136 | 51.451 | 1.00 | 32.73 | C |
| ATOM | 4731 | CG | LEU | B | 320 | 18.733 | 4.202 | 50.426 | 1.00 | 31.98 | C |
| ATOM | 4732 | CD1 | LEU | B | 320 | 17.740 | 4.189 | 49.274 | 1.00 | 34.01 | C |
| ATOM | 4733 | CD2 | LEU | B | 320 | 18.742 | 5.552 | 51.097 | 1.00 | 30.80 | C |
| ATOM | 4734 | C | LEU | B | 320 | 18.651 | 1.999 | 53.648 | 1.00 | 34.80 | C |
| ATOM | 4735 | O | LEU | B | 320 | 18.341 | 2.382 | 54.770 | 1.00 | 34.77 | O |
| ATOM | 4736 | N | LEU | B | 321 | 18.488 | 0.747 | 53.236 | 1.00 | 36.24 | N |
| ATOM | 4737 | CA | LEU | B | 321 | 17.876 | -0.262 | 54.084 | 1.00 | 37.30 | C |
| ATOM | 4738 | CB | LEU | B | 321 | 17.224 | -1.324 | 53.203 | 1.00 | 36.85 | C |
| ATOM | 4739 | CG | LEU | B | 321 | 16.095 | -0.743 | 52.342 | 1.00 | 35.95 | C |
| ATOM | 4740 | CD1 | LEU | B | 321 | 15.703 | -1.729 | 51.266 | 1.00 | 33.90 | C |
| ATOM | 4741 | CD2 | LEU | B | 321 | 14.910 | -0.380 | 53.227 | 1.00 | 35.84 | C |
| ATOM | 4742 | C | LEU | B | 321 | 18.843 | -0.904 | 55.079 | 1.00 | 37.60 | C |
| ATOM | 4743 | O | LEU | B | 321 | 19.188 | -2.086 | 54.973 | 1.00 | 38.39 | O |
| ATOM | 4744 | N | GLU | B | 322 | 19.270 | -0.095 | 56.043 | 1.00 | 37.64 | N |
| ATOM | 4745 | CA | GLU | B | 322 | 20.176 | -0.511 | 57.108 | 1.00 | 37.43 | C |
| ATOM | 4746 | CB | GLU | B | 322 | 21.380 | 0.434 | 57.180 | 1.00 | 37.42 | C |
| ATOM | 4747 | CG | GLU | B | 322 | 22.226 | 0.470 | 55.913 | 1.00 | 41.05 | C |
| ATOM | 4748 | CD | GLU | B | 322 | 23.680 | 0.041 | 56.141 | 1.00 | 41.72 | C |
| ATOM | 4749 | OE1 | GLU | B | 322 | 23.907 | -0.938 | 56.886 | 1.00 | 41.02 | O |
| ATOM | 4750 | OE2 | GLU | B | 322 | 24.585 | 0.679 | 55.551 | 1.00 | 42.64 | O |

| | | | | | | | | | | | |
|---|---|---|---|---|---|---|---|---|---|---|---|
| ATOM | 4751 | C | GLU | B | 322 | 19.409 | -0.453 | 58.433 | 1.00 | 37.21 | C |
| ATOM | 4752 | O | GLU | B | 322 | 18.501 | 0.361 | 58.585 | 1.00 | 36.92 | O |
| ATOM | 4753 | N | TYR | B | 323 | 19.762 | -1.313 | 59.383 | 1.00 | 36.95 | N |
| ATOM | 4754 | CA | TYR | B | 323 | 19.088 | -1.305 | 60.675 | 1.00 | 36.84 | C |
| ATOM | 4755 | CB | TYR | B | 323 | 19.450 | -2.545 | 61.486 | 1.00 | 37.12 | C |
| ATOM | 4756 | CG | TYR | B | 323 | 18.756 | -3.814 | 61.044 | 1.00 | 37.80 | C |
| ATOM | 4757 | CD1 | TYR | B | 323 | 17.389 | -3.993 | 61.248 | 1.00 | 36.24 | C |
| ATOM | 4758 | CE1 | TYR | B | 323 | 16.760 | -5.178 | 60.888 | 1.00 | 35.95 | C |
| ATOM | 4759 | CD2 | TYR | B | 323 | 19.478 | -4.856 | 60.459 | 1.00 | 38.66 | C |
| ATOM | 4760 | CE2 | TYR | B | 323 | 18.856 | -6.044 | 60.095 | 1.00 | 38.54 | C |
| ATOM | 4761 | CZ | TYR | B | 323 | 17.500 | -6.200 | 60.317 | 1.00 | 37.50 | C |
| ATOM | 4762 | OH | TYR | B | 323 | 16.894 | -7.391 | 60.000 | 1.00 | 38.92 | O |
| ATOM | 4763 | C | TYR | B | 323 | 19.506 | -0.058 | 61.436 | 1.00 | 37.41 | C |
| ATOM | 4764 | O | TYR | B | 323 | 18.662 | 0.724 | 61.857 | 1.00 | 39.99 | O |
| ATOM | 4765 | N | THR | B | 324 | 20.811 | 0.128 | 61.608 | 1.00 | 36.07 | N |
| ATOM | 4766 | CA | THR | B | 324 | 21.324 | 1.290 | 62.319 | 1.00 | 35.37 | C |
| ATOM | 4767 | CB | THR | B | 324 | 22.876 | 1.280 | 62.344 | 1.00 | 36.26 | C |
| ATOM | 4768 | OG1 | THR | B | 324 | 23.334 | 0.053 | 62.924 | 1.00 | 36.07 | O |
| ATOM | 4769 | CG2 | THR | B | 324 | 23.422 | 2.440 | 63.168 | 1.00 | 33.19 | C |
| ATOM | 4770 | C | THR | B | 324 | 20.804 | 2.533 | 61.598 | 1.00 | 34.77 | C |
| ATOM | 4771 | O | THR | B | 324 | 21.268 | 2.882 | 60.512 | 1.00 | 34.72 | O |
| ATOM | 4772 | N | PRO | B | 325 | 19.821 | 3.216 | 62.201 | 1.00 | 34.56 | N |
| ATOM | 4773 | CD | PRO | B | 325 | 19.376 | 2.970 | 63.582 | 1.00 | 33.99 | C |
| ATOM | 4774 | CA | PRO | B | 325 | 19.190 | 4.426 | 61.665 | 1.00 | 35.23 | C |
| ATOM | 4775 | CB | PRO | B | 325 | 18.422 | 4.971 | 62.863 | 1.00 | 34.29 | C |
| ATOM | 4776 | CG | PRO | B | 325 | 18.095 | 3.739 | 63.643 | 1.00 | 33.13 | C |
| ATOM | 4777 | C | PRO | B | 325 | 20.236 | 5.411 | 61.188 | 1.00 | 36.04 | C |
| ATOM | 4778 | O | PRO | B | 325 | 20.079 | 6.107 | 60.181 | 1.00 | 36.72 | O |
| ATOM | 4779 | N | THR | B | 326 | 21.319 | 5.437 | 61.944 | 1.00 | 36.83 | N |
| ATOM | 4780 | CA | THR | B | 326 | 22.447 | 6.317 | 61.731 | 1.00 | 35.69 | C |
| ATOM | 4781 | CB | THR | B | 326 | 23.216 | 6.407 | 63.057 | 1.00 | 36.56 | C |
| ATOM | 4782 | OG1 | THR | B | 326 | 23.624 | 7.757 | 63.282 | 1.00 | 37.49 | O |
| ATOM | 4783 | CG2 | THR | B | 326 | 24.423 | 5.467 | 63.045 | 1.00 | 36.84 | C |
| ATOM | 4784 | C | THR | B | 326 | 23.372 | 5.871 | 60.595 | 1.00 | 34.60 | C |
| ATOM | 4785 | O | THR | B | 326 | 24.179 | 6.655 | 60.102 | 1.00 | 34.65 | O |
| ATOM | 4786 | N | ALA | B | 327 | 23.253 | 4.612 | 60.191 | 1.00 | 34.51 | N |
| ATOM | 4787 | CA | ALA | B | 327 | 24.080 | 4.065 | 59.124 | 1.00 | 33.73 | C |
| ATOM | 4788 | CB | ALA | B | 327 | 24.127 | 2.551 | 59.236 | 1.00 | 31.42 | C |
| ATOM | 4789 | C | ALA | B | 327 | 23.532 | 4.474 | 57.759 | 1.00 | 34.57 | C |
| ATOM | 4790 | O | ALA | B | 327 | 24.259 | 4.513 | 56.775 | 1.00 | 35.05 | O |
| ATOM | 4791 | N | ARG | B | 328 | 22.240 | 4.788 | 57.714 | 1.00 | 35.23 | N |
| ATOM | 4792 | CA | ARG | B | 328 | 21.569 | 5.182 | 56.483 | 1.00 | 34.72 | C |
| ATOM | 4793 | CB | ARG | B | 328 | 20.070 | 5.303 | 56.750 | 1.00 | 34.75 | C |
| ATOM | 4794 | CG | ARG | B | 328 | 19.427 | 3.968 | 57.079 | 1.00 | 34.73 | C |
| ATOM | 4795 | CD | ARG | B | 328 | 18.018 | 4.115 | 57.593 | 1.00 | 34.25 | C |
| ATOM | 4796 | NE | ARG | B | 328 | 17.648 | 2.934 | 58.360 | 1.00 | 34.20 | N |
| ATOM | 4797 | CZ | ARG | B | 328 | 16.771 | 2.931 | 59.357 | 1.00 | 34.76 | C |
| ATOM | 4798 | NH1 | ARG | B | 328 | 16.159 | 4.050 | 59.716 | 1.00 | 32.91 | N |
| ATOM | 4799 | NH2 | ARG | B | 328 | 16.518 | 1.807 | 60.013 | 1.00 | 36.40 | N |
| ATOM | 4800 | C | ARG | B | 328 | 22.090 | 6.483 | 55.882 | 1.00 | 35.36 | C |
| ATOM | 4801 | O | ARG | B | 328 | 22.586 | 7.354 | 56.600 | 1.00 | 35.81 | O |
| ATOM | 4802 | N | LEU | B | 329 | 21.974 | 6.607 | 54.561 | 1.00 | 35.47 | N |
| ATOM | 4803 | CA | LEU | B | 329 | 22.415 | 7.809 | 53.854 | 1.00 | 36.18 | C |
| ATOM | 4804 | CB | LEU | B | 329 | 22.453 | 7.562 | 52.336 | 1.00 | 36.99 | C |
| ATOM | 4805 | CG | LEU | B | 329 | 23.622 | 6.796 | 51.700 | 1.00 | 37.01 | C |
| ATOM | 4806 | CD1 | LEU | B | 329 | 23.952 | 5.596 | 52.554 | 1.00 | 40.01 | C |
| ATOM | 4807 | CD2 | LEU | B | 329 | 23.274 | 6.374 | 50.279 | 1.00 | 36.22 | C |
| ATOM | 4808 | C | LEU | B | 329 | 21.445 | 8.950 | 54.139 | 1.00 | 36.53 | C |
| ATOM | 4809 | O | LEU | B | 329 | 20.303 | 8.709 | 54.527 | 1.00 | 36.91 | O |
| ATOM | 4810 | N | THR | B | 330 | 21.896 | 10.189 | 53.960 | 1.00 | 36.21 | N |
| ATOM | 4811 | CA | THR | B | 330 | 21.014 | 11.329 | 54.174 | 1.00 | 36.64 | C |
| ATOM | 4812 | CB | THR | B | 330 | 21.767 | 12.579 | 54.688 | 1.00 | 37.55 | C |
| ATOM | 4813 | OG1 | THR | B | 330 | 22.595 | 13.111 | 53.646 | 1.00 | 37.86 | O |
| ATOM | 4814 | CG2 | THR | B | 330 | 22.636 | 12.217 | 55.882 | 1.00 | 36.95 | C |
| ATOM | 4815 | C | THR | B | 330 | 20.420 | 11.643 | 52.808 | 1.00 | 36.88 | C |
| ATOM | 4816 | O | THR | B | 330 | 21.067 | 11.429 | 51.782 | 1.00 | 36.59 | O |
| ATOM | 4817 | N | PRO | B | 331 | 19.175 | 12.139 | 52.773 | 1.00 | 36.07 | N |

| | | | | | | | | | | | |
|---|---|---|---|---|---|---|---|---|---|---|---|
| ATOM | 4818 | CD | PRO | B | 331 | 18.276 | 12.466 | 53.895 | 1.00 | 35.13 | C |
| ATOM | 4819 | CA | PRO | B | 331 | 18.553 | 12.459 | 51.487 | 1.00 | 35.48 | C |
| ATOM | 4820 | CB | PRO | B | 331 | 17.369 | 13.323 | 51.893 | 1.00 | 35.83 | C |
| ATOM | 4821 | CG | PRO | B | 331 | 16.956 | 12.700 | 53.198 | 1.00 | 35.13 | C |
| ATOM | 4822 | C | PRO | B | 331 | 19.501 | 13.151 | 50.499 | 1.00 | 36.11 | C |
| ATOM | 4823 | O | PRO | B | 331 | 19.531 | 12.798 | 49.319 | 1.00 | 36.87 | O |
| ATOM | 4824 | N | LEU | B | 332 | 20.285 | 14.116 | 50.969 | 1.00 | 36.05 | N |
| ATOM | 4825 | CA | LEU | B | 332 | 21.219 | 14.820 | 50.086 | 1.00 | 36.74 | C |
| ATOM | 4826 | CB | LEU | B | 332 | 21.865 | 16.018 | 50.794 | 1.00 | 37.18 | C |
| ATOM | 4827 | CG | LEU | B | 332 | 21.527 | 17.434 | 50.326 | 1.00 | 38.13 | C |
| ATOM | 4828 | CD1 | LEU | B | 332 | 22.632 | 18.364 | 50.806 | 1.00 | 40.15 | C |
| ATOM | 4829 | CD2 | LEU | B | 332 | 21.429 | 17.502 | 48.801 | 1.00 | 39.36 | C |
| ATOM | 4830 | C | LEU | B | 332 | 22.327 | 13.904 | 49.594 | 1.00 | 36.91 | C |
| ATOM | 4831 | O | LEU | B | 332 | 22.828 | 14.080 | 48.483 | 1.00 | 38.23 | O |
| ATOM | 4832 | N | GLU | B | 333 | 22.716 | 12.941 | 50.428 | 1.00 | 36.87 | N |
| ATOM | 4833 | CA | GLU | B | 333 | 23.780 | 11.996 | 50.083 | 1.00 | 37.90 | C |
| ATOM | 4834 | CB | GLU | B | 333 | 24.234 | 11.224 | 51.339 | 1.00 | 40.10 | C |
| ATOM | 4835 | CG | GLU | B | 333 | 25.448 | 11.843 | 52.046 | 1.00 | 41.42 | C |
| ATOM | 4836 | CD | GLU | B | 333 | 25.519 | 11.539 | 53.544 | 1.00 | 43.91 | C |
| ATOM | 4837 | OE1 | GLU | B | 333 | 25.433 | 10.354 | 53.942 | 1.00 | 44.02 | O |
| ATOM | 4838 | OE2 | GLU | B | 333 | 25.677 | 12.499 | 54.333 | 1.00 | 46.05 | O |
| ATOM | 4839 | C | GLU | B | 333 | 23.291 | 11.034 | 49.006 | 1.00 | 36.60 | C |
| ATOM | 4840 | O | GLU | B | 333 | 24.037 | 10.682 | 48.088 | 1.00 | 35.40 | O |
| ATOM | 4841 | N | ALA | B | 334 | 22.024 | 10.639 | 49.123 | 1.00 | 36.58 | N |
| ATOM | 4842 | CA | ALA | B | 334 | 21.383 | 9.734 | 48.179 | 1.00 | 35.74 | C |
| ATOM | 4843 | CB | ALA | B | 334 | 20.000 | 9.354 | 48.681 | 1.00 | 34.27 | C |
| ATOM | 4844 | C | ALA | B | 334 | 21.285 | 10.387 | 46.800 | 1.00 | 35.80 | C |
| ATOM | 4845 | O | ALA | B | 334 | 21.450 | 9.718 | 45.775 | 1.00 | 36.08 | O |
| ATOM | 4846 | N | CYS | B | 335 | 21.028 | 11.693 | 46.779 | 1.00 | 36.16 | N |
| ATOM | 4847 | CA | CYS | B | 335 | 20.936 | 12.436 | 45.517 | 1.00 | 36.42 | C |
| ATOM | 4848 | CB | CYS | B | 335 | 20.543 | 13.899 | 45.764 | 1.00 | 34.14 | C |
| ATOM | 4849 | SG | CYS | B | 335 | 18.840 | 14.195 | 46.235 | 1.00 | 33.25 | S |
| ATOM | 4850 | C | CYS | B | 335 | 22.283 | 12.416 | 44.794 | 1.00 | 37.41 | C |
| ATOM | 4851 | O | CYS | B | 335 | 22.342 | 12.388 | 43.561 | 1.00 | 37.21 | O |
| ATOM | 4852 | N | ALA | B | 336 | 23.362 | 12.436 | 45.574 | 1.00 | 37.62 | N |
| ATOM | 4853 | CA | ALA | B | 336 | 24.710 | 12.426 | 45.020 | 1.00 | 38.83 | C |
| ATOM | 4854 | CB | ALA | B | 336 | 25.686 | 13.061 | 46.006 | 1.00 | 40.30 | C |
| ATOM | 4855 | C | ALA | B | 336 | 25.190 | 11.024 | 44.663 | 1.00 | 39.16 | C |
| ATOM | 4856 | O | ALA | B | 336 | 26.320 | 10.848 | 44.201 | 1.00 | 39.54 | O |
| ATOM | 4857 | N | HIS | B | 337 | 24.339 | 10.024 | 44.866 | 1.00 | 39.01 | N |
| ATOM | 4858 | CA | HIS | B | 337 | 24.734 | 8.659 | 44.559 | 1.00 | 38.87 | C |
| ATOM | 4859 | CB | HIS | B | 337 | 23.696 | 7.664 | 45.074 | 1.00 | 37.22 | C |
| ATOM | 4860 | CG | HIS | B | 337 | 24.153 | 6.238 | 45.013 | 1.00 | 35.47 | C |
| ATOM | 4861 | CD2 | HIS | B | 337 | 24.312 | 5.398 | 43.962 | 1.00 | 34.39 | C |
| ATOM | 4862 | ND1 | HIS | B | 337 | 24.522 | 5.521 | 46.131 | 1.00 | 34.35 | N |
| ATOM | 4863 | CE1 | HIS | B | 337 | 24.884 | 4.303 | 45.772 | 1.00 | 34.21 | C |
| ATOM | 4864 | NE2 | HIS | B | 337 | 24.765 | 4.204 | 44.462 | 1.00 | 32.96 | N |
| ATOM | 4865 | C | HIS | B | 337 | 24.928 | 8.470 | 43.060 | 1.00 | 39.98 | C |
| ATOM | 4866 | O | HIS | B | 337 | 24.222 | 9.068 | 42.242 | 1.00 | 39.72 | O |
| ATOM | 4867 | N | SER | B | 338 | 25.888 | 7.621 | 42.705 | 1.00 | 41.82 | N |
| ATOM | 4868 | CA | SER | B | 338 | 26.204 | 7.356 | 41.305 | 1.00 | 42.40 | C |
| ATOM | 4869 | CB | SER | B | 338 | 27.385 | 6.393 | 41.219 | 1.00 | 41.59 | C |
| ATOM | 4870 | OG | SER | B | 338 | 27.017 | 5.113 | 41.682 | 1.00 | 43.45 | O |
| ATOM | 4871 | C | SER | B | 338 | 25.027 | 6.822 | 40.482 | 1.00 | 42.55 | C |
| ATOM | 4872 | O | SER | B | 338 | 24.976 | 7.033 | 39.269 | 1.00 | 43.90 | O |
| ATOM | 4873 | N | PHE | B | 339 | 24.089 | 6.138 | 41.138 | 1.00 | 40.97 | N |
| ATOM | 4874 | CA | PHE | B | 339 | 22.903 | 5.585 | 40.473 | 1.00 | 40.37 | C |
| ATOM | 4875 | CB | PHE | B | 339 | 21.975 | 4.946 | 41.520 | 1.00 | 39.92 | C |
| ATOM | 4876 | CG | PHE | B | 339 | 20.615 | 4.546 | 40.994 | 1.00 | 39.67 | C |
| ATOM | 4877 | CD1 | PHE | B | 339 | 20.479 | 3.574 | 40.007 | 1.00 | 40.30 | C |
| ATOM | 4878 | CD2 | PHE | B | 339 | 19.463 | 5.106 | 41.531 | 1.00 | 38.62 | C |
| ATOM | 4879 | CE1 | PHE | B | 339 | 19.210 | 3.164 | 39.571 | 1.00 | 39.54 | C |
| ATOM | 4880 | CE2 | PHE | B | 339 | 18.197 | 4.701 | 41.099 | 1.00 | 38.53 | C |
| ATOM | 4881 | CZ | PHE | B | 339 | 18.072 | 3.729 | 40.120 | 1.00 | 38.16 | C |
| ATOM | 4882 | C | PHE | B | 339 | 22.164 | 6.694 | 39.718 | 1.00 | 40.53 | C |
| ATOM | 4883 | O | PHE | B | 339 | 21.476 | 6.442 | 38.730 | 1.00 | 40.51 | O |
| ATOM | 4884 | N | PHE | B | 340 | 22.332 | 7.927 | 40.182 | 1.00 | 39.40 | N |

| | | | | | | | | | | | |
|---|---|---|---|---|---|---|---|---|---|---|---|
| ATOM | 4885 | CA | PHE | B | 340 | 21.676 | 9.062 | 39.556 | 1.00 | 39.22 | C |
| ATOM | 4886 | CB | PHE | B | 340 | 21.120 | 10.005 | 40.615 | 1.00 | 36.79 | C |
| ATOM | 4887 | CG | PHE | B | 340 | 20.215 | 9.344 | 41.607 | 1.00 | 37.42 | C |
| ATOM | 4888 | CD1 | PHE | B | 340 | 18.955 | 8.889 | 41.234 | 1.00 | 35.22 | C |
| ATOM | 4889 | CD2 | PHE | B | 340 | 20.607 | 9.211 | 42.933 | 1.00 | 34.42 | C |
| ATOM | 4890 | CE1 | PHE | B | 340 | 18.101 | 8.320 | 42.167 | 1.00 | 33.89 | C |
| ATOM | 4891 | CE2 | PHE | B | 340 | 19.757 | 8.643 | 43.867 | 1.00 | 33.59 | C |
| ATOM | 4892 | CZ | PHE | B | 340 | 18.500 | 8.199 | 43.485 | 1.00 | 33.67 | C |
| ATOM | 4893 | C | PHE | B | 340 | 22.627 | 9.851 | 38.675 | 1.00 | 40.51 | C |
| ATOM | 4894 | O | PHE | B | 340 | 22.278 | 10.942 | 38.217 | 1.00 | 41.24 | O |
| ATOM | 4895 | N | ASP | B | 341 | 23.829 | 9.327 | 38.454 | 1.00 | 40.71 | N |
| ATOM | 4896 | CA | ASP | B | 341 | 24.801 | 10.037 | 37.623 | 1.00 | 41.22 | C |
| ATOM | 4897 | CB | ASP | B | 341 | 26.090 | 9.217 | 37.466 | 1.00 | 41.05 | C |
| ATOM | 4898 | CG | ASP | B | 341 | 27.038 | 9.422 | 38.626 | 1.00 | 41.38 | C |
| ATOM | 4899 | OD1 | ASP | B | 341 | 26.597 | 10.032 | 39.623 | 1.00 | 42.79 | O |
| ATOM | 4900 | OD2 | ASP | B | 341 | 28.207 | 8.988 | 38.557 | 1.00 | 41.29 | O |
| ATOM | 4901 | C | ASP | B | 341 | 24.222 | 10.382 | 36.264 | 1.00 | 40.58 | C |
| ATOM | 4902 | O | ASP | B | 341 | 24.408 | 11.491 | 35.775 | 1.00 | 40.43 | O |
| ATOM | 4903 | N | GLU | B | 342 | 23.510 | 9.437 | 35.662 | 1.00 | 39.55 | N |
| ATOM | 4904 | CA | GLU | B | 342 | 22.905 | 9.689 | 34.372 | 1.00 | 39.89 | C |
| ATOM | 4905 | CB | GLU | B | 342 | 21.958 | 8.549 | 34.000 | 1.00 | 40.92 | C |
| ATOM | 4906 | CG | GLU | B | 342 | 21.087 | 8.860 | 32.797 | 1.00 | 43.50 | C |
| ATOM | 4907 | CD | GLU | B | 342 | 20.377 | 7.642 | 32.249 | 1.00 | 44.87 | C |
| ATOM | 4908 | OE1 | GLU | B | 342 | 19.942 | 6.789 | 33.051 | 1.00 | 43.81 | O |
| ATOM | 4909 | OE2 | GLU | B | 342 | 20.242 | 7.551 | 31.010 | 1.00 | 46.04 | O |
| ATOM | 4910 | C | GLU | B | 342 | 22.150 | 11.019 | 34.353 | 1.00 | 40.36 | C |
| ATOM | 4911 | O | GLU | B | 342 | 22.393 | 11.852 | 33.491 | 1.00 | 41.39 | O |
| ATOM | 4912 | N | LEU | B | 343 | 21.249 | 11.220 | 35.315 | 1.00 | 40.76 | N |
| ATOM | 4913 | CA | LEU | B | 343 | 20.455 | 12.443 | 35.397 | 1.00 | 40.90 | C |
| ATOM | 4914 | CB | LEU | B | 343 | 19.607 | 12.453 | 36.672 | 1.00 | 41.21 | C |
| ATOM | 4915 | CG | LEU | B | 343 | 18.634 | 11.304 | 36.976 | 1.00 | 41.48 | C |
| ATOM | 4916 | CD1 | LEU | B | 343 | 17.864 | 11.655 | 38.251 | 1.00 | 40.01 | C |
| ATOM | 4917 | CD2 | LEU | B | 343 | 17.671 | 11.085 | 35.825 | 1.00 | 40.93 | C |
| ATOM | 4918 | C | LEU | B | 343 | 21.302 | 13.711 | 35.369 | 1.00 | 41.60 | C |
| ATOM | 4919 | O | LEU | B | 343 | 20.814 | 14.780 | 34.999 | 1.00 | 41.55 | O |
| ATOM | 4920 | N | ARG | B | 344 | 22.561 | 13.597 | 35.775 | 1.00 | 41.94 | N |
| ATOM | 4921 | CA | ARG | B | 344 | 23.457 | 14.748 | 35.788 | 1.00 | 43.15 | C |
| ATOM | 4922 | CB | ARG | B | 344 | 24.526 | 14.575 | 36.871 | 1.00 | 42.30 | C |
| ATOM | 4923 | CG | ARG | B | 344 | 24.154 | 15.150 | 38.227 | 1.00 | 40.16 | C |
| ATOM | 4924 | CD | ARG | B | 344 | 25.240 | 14.849 | 39.245 | 1.00 | 41.13 | C |
| ATOM | 4925 | NE | ARG | B | 344 | 25.245 | 13.439 | 39.615 | 1.00 | 41.90 | N |
| ATOM | 4926 | CZ | ARG | B | 344 | 24.523 | 12.923 | 40.607 | 1.00 | 41.06 | C |
| ATOM | 4927 | NH1 | ARG | B | 344 | 23.740 | 13.705 | 41.337 | 1.00 | 40.52 | N |
| ATOM | 4928 | NH2 | ARG | B | 344 | 24.572 | 11.623 | 40.862 | 1.00 | 39.84 | N |
| ATOM | 4929 | C | ARG | B | 344 | 24.126 | 14.973 | 34.429 | 1.00 | 44.75 | C |
| ATOM | 4930 | O | ARG | B | 344 | 24.791 | 15.983 | 34.207 | 1.00 | 44.00 | O |
| ATOM | 4931 | N | ASP | B | 345 | 23.947 | 14.024 | 33.522 | 1.00 | 47.04 | N |
| ATOM | 4932 | CA | ASP | B | 345 | 24.511 | 14.116 | 32.175 | 1.00 | 49.83 | C |
| ATOM | 4933 | CB | ASP | B | 345 | 24.227 | 12.811 | 31.431 | 1.00 | 50.85 | C |
| ATOM | 4934 | CG | ASP | B | 345 | 24.646 | 12.855 | 29.983 | 1.00 | 53.06 | C |
| ATOM | 4935 | OD1 | ASP | B | 345 | 24.626 | 13.955 | 29.386 | 1.00 | 53.68 | O |
| ATOM | 4936 | OD2 | ASP | B | 345 | 24.979 | 11.777 | 29.435 | 1.00 | 52.87 | O |
| ATOM | 4937 | C | ASP | B | 345 | 23.856 | 15.286 | 31.435 | 1.00 | 51.22 | C |
| ATOM | 4938 | O | ASP | B | 345 | 22.632 | 15.429 | 31.469 | 1.00 | 52.66 | O |
| ATOM | 4939 | N | PRO | B | 346 | 24.650 | 16.125 | 30.742 | 1.00 | 51.90 | N |
| ATOM | 4940 | CD | PRO | B | 346 | 26.117 | 16.109 | 30.605 | 1.00 | 52.14 | C |
| ATOM | 4941 | CA | PRO | B | 346 | 24.079 | 17.268 | 30.010 | 1.00 | 51.28 | C |
| ATOM | 4942 | CB | PRO | B | 346 | 25.315 | 18.040 | 29.562 | 1.00 | 51.59 | C |
| ATOM | 4943 | CG | PRO | B | 346 | 26.323 | 16.959 | 29.366 | 1.00 | 51.35 | C |
| ATOM | 4944 | C | PRO | B | 346 | 23.166 | 16.918 | 28.826 | 1.00 | 50.51 | C |
| ATOM | 4945 | O | PRO | B | 346 | 22.410 | 17.764 | 28.344 | 1.00 | 50.70 | O |
| ATOM | 4946 | N | ASN | B | 347 | 23.240 | 15.673 | 28.373 | 1.00 | 49.50 | N |
| ATOM | 4947 | CA | ASN | B | 347 | 22.448 | 15.199 | 27.252 | 1.00 | 49.09 | C |
| ATOM | 4948 | CB | ASN | B | 347 | 23.260 | 14.181 | 26.457 | 1.00 | 49.88 | C |
| ATOM | 4949 | CG | ASN | B | 347 | 24.478 | 14.801 | 25.804 | 1.00 | 52.95 | C |
| ATOM | 4950 | OD1 | ASN | B | 347 | 25.376 | 14.091 | 25.334 | 1.00 | 54.73 | O |
| ATOM | 4951 | ND2 | ASN | B | 347 | 24.520 | 16.134 | 25.767 | 1.00 | 52.55 | N |

| | | | | | | | | | | | |
|---|---|---|---|---|---|---|---|---|---|---|---|
| ATOM | 4952 | C | ASN | B | 347 | 21.122 | 14.573 | 27.655 | 1.00 | 48.70 | C |
| ATOM | 4953 | O | ASN | B | 347 | 20.109 | 14.766 | 26.979 | 1.00 | 49.24 | O |
| ATOM | 4954 | N | VAL | B | 348 | 21.126 | 13.815 | 28.746 | 1.00 | 47.73 | N |
| ATOM | 4955 | CA | VAL | B | 348 | 19.920 | 13.134 | 29.198 | 1.00 | 47.05 | C |
| ATOM | 4956 | CB | VAL | B | 348 | 19.995 | 12.788 | 30.707 | 1.00 | 46.33 | C |
| ATOM | 4957 | CG1 | VAL | B | 348 | 19.728 | 14.033 | 31.559 | 1.00 | 45.82 | C |
| ATOM | 4958 | CG2 | VAL | B | 348 | 19.001 | 11.690 | 31.024 | 1.00 | 45.53 | C |
| ATOM | 4959 | C | VAL | B | 348 | 18.630 | 13.904 | 28.938 | 1.00 | 46.71 | C |
| ATOM | 4960 | O | VAL | B | 348 | 18.513 | 15.090 | 29.246 | 1.00 | 46.05 | O |
| ATOM | 4961 | N | LYS | B | 349 | 17.663 | 13.199 | 28.373 | 1.00 | 46.22 | N |
| ATOM | 4962 | CA | LYS | B | 349 | 16.358 | 13.763 | 28.077 | 1.00 | 46.75 | C |
| ATOM | 4963 | CB | LYS | B | 349 | 16.235 | 14.064 | 26.573 | 1.00 | 48.36 | C |
| ATOM | 4964 | CG | LYS | B | 349 | 17.157 | 15.201 | 26.107 | 1.00 | 50.15 | C |
| ATOM | 4965 | CD | LYS | B | 349 | 16.904 | 15.618 | 24.669 | 1.00 | 53.19 | C |
| ATOM | 4966 | CE | LYS | B | 349 | 18.011 | 16.536 | 24.165 | 1.00 | 55.34 | C |
| ATOM | 4967 | NZ | LYS | B | 349 | 17.921 | 16.789 | 22.686 | 1.00 | 57.76 | N |
| ATOM | 4968 | C | LYS | B | 349 | 15.343 | 12.717 | 28.524 | 1.00 | 45.87 | C |
| ATOM | 4969 | O | LYS | B | 349 | 15.732 | 11.620 | 28.913 | 1.00 | 45.55 | O |
| ATOM | 4970 | N | LEU | B | 350 | 14.056 | 13.047 | 28.513 | 1.00 | 45.02 | N |
| ATOM | 4971 | CA | LEU | B | 350 | 13.047 | 12.072 | 28.928 | 1.00 | 44.48 | C |
| ATOM | 4972 | CB | LEU | B | 350 | 11.761 | 12.780 | 29.355 | 1.00 | 44.13 | C |
| ATOM | 4973 | CG | LEU | B | 350 | 11.812 | 13.792 | 30.500 | 1.00 | 43.03 | C |
| ATOM | 4974 | CD1 | LEU | B | 350 | 10.396 | 14.270 | 30.826 | 1.00 | 40.95 | C |
| ATOM | 4975 | CD2 | LEU | B | 350 | 12.440 | 13.144 | 31.711 | 1.00 | 41.69 | C |
| ATOM | 4976 | C | LEU | B | 350 | 12.736 | 11.148 | 27.758 | 1.00 | 44.78 | C |
| ATOM | 4977 | O | LEU | B | 350 | 12.892 | 11.532 | 26.605 | 1.00 | 44.38 | O |
| ATOM | 4978 | N | PRO | B | 351 | 12.277 | 9.920 | 28.037 | 1.00 | 45.94 | N |
| ATOM | 4979 | CD | PRO | B | 351 | 11.866 | 9.405 | 29.348 | 1.00 | 46.03 | C |
| ATOM | 4980 | CA | PRO | B | 351 | 11.944 | 8.963 | 26.973 | 1.00 | 47.24 | C |
| ATOM | 4981 | CB | PRO | B | 351 | 11.440 | 7.736 | 27.738 | 1.00 | 46.25 | C |
| ATOM | 4982 | CG | PRO | B | 351 | 11.968 | 7.922 | 29.131 | 1.00 | 46.63 | C |
| ATOM | 4983 | C | PRO | B | 351 | 10.822 | 9.600 | 26.160 | 1.00 | 48.43 | C |
| ATOM | 4984 | O | PRO | B | 351 | 10.284 | 9.024 | 25.209 | 1.00 | 49.72 | O |
| ATOM | 4985 | N | ASN | B | 352 | 10.488 | 10.809 | 26.585 | 1.00 | 49.77 | N |
| ATOM | 4986 | CA | ASN | B | 352 | 9.445 | 11.640 | 26.020 | 1.00 | 50.32 | C |
| ATOM | 4987 | CB | ASN | B | 352 | 8.982 | 12.612 | 27.100 | 1.00 | 52.86 | C |
| ATOM | 4988 | CG | ASN | B | 352 | 7.499 | 12.761 | 27.123 | 1.00 | 54.76 | C |
| ATOM | 4989 | OD1 | ASN | B | 352 | 6.945 | 13.423 | 27.996 | 1.00 | 55.29 | O |
| ATOM | 4990 | ND2 | ASN | B | 352 | 6.830 | 12.134 | 26.154 | 1.00 | 56.70 | N |
| ATOM | 4991 | C | ASN | B | 352 | 9.970 | 12.439 | 24.850 | 1.00 | 50.02 | C |
| ATOM | 4992 | O | ASN | B | 352 | 9.211 | 12.894 | 24.001 | 1.00 | 50.19 | O |
| ATOM | 4993 | N | GLY | B | 353 | 11.284 | 12.623 | 24.838 | 1.00 | 50.08 | N |
| ATOM | 4994 | CA | GLY | B | 353 | 11.918 | 13.402 | 23.801 | 1.00 | 50.66 | C |
| ATOM | 4995 | C | GLY | B | 353 | 12.108 | 14.762 | 24.427 | 1.00 | 52.00 | C |
| ATOM | 4996 | O | GLY | B | 353 | 12.881 | 15.586 | 23.950 | 1.00 | 52.16 | O |
| ATOM | 4997 | N | ARG | B | 354 | 11.388 | 14.982 | 25.526 | 1.00 | 52.86 | N |
| ATOM | 4998 | CA | ARG | B | 354 | 11.429 | 16.239 | 26.269 | 1.00 | 53.01 | C |
| ATOM | 4999 | CB | ARG | B | 354 | 10.096 | 16.444 | 26.994 | 1.00 | 55.00 | C |
| ATOM | 5000 | CG | ARG | B | 354 | 8.891 | 16.145 | 26.121 | 1.00 | 58.09 | C |
| ATOM | 5001 | CD | ARG | B | 354 | 7.577 | 16.373 | 26.860 | 1.00 | 60.19 | C |
| ATOM | 5002 | NE | ARG | B | 354 | 7.542 | 17.689 | 27.494 | 1.00 | 61.04 | N |
| ATOM | 5003 | CZ | ARG | B | 354 | 6.430 | 18.319 | 27.853 | 1.00 | 61.30 | C |
| ATOM | 5004 | NH1 | ARG | B | 354 | 5.243 | 17.750 | 27.634 | 1.00 | 60.22 | N |
| ATOM | 5005 | NH2 | ARG | B | 354 | 6.515 | 19.519 | 28.433 | 1.00 | 61.61 | N |
| ATOM | 5006 | C | ARG | B | 354 | 12.568 | 16.291 | 27.279 | 1.00 | 51.85 | C |
| ATOM | 5007 | O | ARG | B | 354 | 13.195 | 15.275 | 27.589 | 1.00 | 51.69 | O |
| ATOM | 5008 | N | ASP | B | 355 | 12.843 | 17.486 | 27.787 | 1.00 | 50.43 | N |
| ATOM | 5009 | CA | ASP | B | 355 | 13.905 | 17.637 | 28.765 | 1.00 | 49.96 | C |
| ATOM | 5010 | CB | ASP | B | 355 | 14.481 | 19.060 | 28.733 | 1.00 | 52.37 | C |
| ATOM | 5011 | CG | ASP | B | 355 | 15.147 | 19.394 | 27.401 | 1.00 | 54.01 | C |
| ATOM | 5012 | OD1 | ASP | B | 355 | 14.432 | 19.833 | 26.471 | 1.00 | 55.24 | O |
| ATOM | 5013 | OD2 | ASP | B | 355 | 16.384 | 19.206 | 27.278 | 1.00 | 54.62 | O |
| ATOM | 5014 | C | ASP | B | 355 | 13.409 | 17.300 | 30.167 | 1.00 | 48.12 | C |
| ATOM | 5015 | O | ASP | B | 355 | 12.230 | 17.451 | 30.480 | 1.00 | 47.13 | O |
| ATOM | 5016 | N | THR | B | 356 | 14.328 | 16.821 | 30.996 | 1.00 | 46.82 | N |
| ATOM | 5017 | CA | THR | B | 356 | 14.030 | 16.450 | 32.370 | 1.00 | 44.76 | C |
| ATOM | 5018 | CB | THR | B | 356 | 15.221 | 15.721 | 33.011 | 1.00 | 44.89 | C |

| | | | | | | | | | | | |
|---|---|---|---|---|---|---|---|---|---|---|---|
| ATOM | 5019 | OG1 | THR | B | 356 | 16.393 | 16.544 | 32.912 | 1.00 | 42.21 | O |
| ATOM | 5020 | CG2 | THR | B | 356 | 15.480 | 14.398 | 32.310 | 1.00 | 42.61 | C |
| ATOM | 5021 | C | THR | B | 356 | 13.769 | 17.695 | 33.192 | 1.00 | 44.45 | C |
| ATOM | 5022 | O | THR | B | 356 | 14.313 | 18.764 | 32.909 | 1.00 | 44.63 | O |
| ATOM | 5023 | N | PRO | B | 357 | 12.936 | 17.579 | 34.231 | 1.00 | 44.07 | N |
| ATOM | 5024 | CD | PRO | B | 357 | 12.184 | 16.410 | 34.716 | 1.00 | 43.73 | C |
| ATOM | 5025 | CA | PRO | B | 357 | 12.673 | 18.761 | 35.051 | 1.00 | 43.81 | C |
| ATOM | 5026 | CB | PRO | B | 357 | 11.680 | 18.249 | 36.097 | 1.00 | 43.77 | C |
| ATOM | 5027 | CG | PRO | B | 357 | 11.949 | 16.771 | 36.163 | 1.00 | 43.70 | C |
| ATOM | 5028 | C | PRO | B | 357 | 13.981 | 19.272 | 35.663 | 1.00 | 44.79 | C |
| ATOM | 5029 | O | PRO | B | 357 | 15.008 | 18.584 | 35.614 | 1.00 | 45.26 | O |
| ATOM | 5030 | N | ALA | B | 358 | 13.951 | 20.485 | 36.213 | 1.00 | 45.25 | N |
| ATOM | 5031 | CA | ALA | B | 358 | 15.134 | 21.078 | 36.832 | 1.00 | 45.32 | C |
| ATOM | 5032 | CB | ALA | B | 358 | 14.832 | 22.480 | 37.298 | 1.00 | 45.31 | C |
| ATOM | 5033 | C | ALA | B | 358 | 15.490 | 20.202 | 38.015 | 1.00 | 45.89 | C |
| ATOM | 5034 | O | ALA | B | 358 | 14.666 | 20.004 | 38.904 | 1.00 | 47.78 | O |
| ATOM | 5035 | N | LEU | B | 359 | 16.709 | 19.676 | 38.031 | 1.00 | 45.58 | N |
| ATOM | 5036 | CA | LEU | B | 359 | 17.135 | 18.784 | 39.107 | 1.00 | 45.00 | C |
| ATOM | 5037 | CB | LEU | B | 359 | 17.566 | 17.446 | 38.509 | 1.00 | 43.37 | C |
| ATOM | 5038 | CG | LEU | B | 359 | 16.538 | 16.823 | 37.582 | 1.00 | 42.44 | C |
| ATOM | 5039 | CD1 | LEU | B | 359 | 17.065 | 15.521 | 36.991 | 1.00 | 41.68 | C |
| ATOM | 5040 | CD2 | LEU | B | 359 | 15.259 | 16.602 | 38.387 | 1.00 | 43.15 | C |
| ATOM | 5041 | C | LEU | B | 359 | 18.301 | 19.374 | 39.866 | 1.00 | 45.47 | C |
| ATOM | 5042 | O | LEU | B | 359 | 18.688 | 18.870 | 40.915 | 1.00 | 45.98 | O |
| ATOM | 5043 | N | PHE | B | 360 | 18.840 | 20.465 | 39.333 | 1.00 | 45.87 | N |
| ATOM | 5044 | CA | PHE | B | 360 | 20.022 | 21.108 | 39.900 | 1.00 | 44.98 | C |
| ATOM | 5045 | CB | PHE | B | 360 | 21.071 | 21.179 | 38.802 | 1.00 | 44.49 | C |
| ATOM | 5046 | CG | PHE | B | 360 | 21.155 | 19.925 | 38.002 | 1.00 | 44.93 | C |
| ATOM | 5047 | CD1 | PHE | B | 360 | 21.432 | 18.715 | 38.634 | 1.00 | 45.00 | C |
| ATOM | 5048 | CD2 | PHE | B | 360 | 20.920 | 19.932 | 36.634 | 1.00 | 44.43 | C |
| ATOM | 5049 | CE1 | PHE | B | 360 | 21.477 | 17.530 | 37.916 | 1.00 | 45.19 | C |
| ATOM | 5050 | CE2 | PHE | B | 360 | 20.963 | 18.748 | 35.911 | 1.00 | 45.79 | C |
| ATOM | 5051 | CZ | PHE | B | 360 | 21.240 | 17.543 | 36.554 | 1.00 | 45.01 | C |
| ATOM | 5052 | C | PHE | B | 360 | 19.876 | 22.473 | 40.548 | 1.00 | 44.28 | C |
| ATOM | 5053 | O | PHE | B | 360 | 20.856 | 23.012 | 41.070 | 1.00 | 44.06 | O |
| ATOM | 5054 | N | ASN | B | 361 | 18.671 | 23.032 | 40.529 | 1.00 | 44.57 | N |
| ATOM | 5055 | CA | ASN | B | 361 | 18.450 | 24.351 | 41.118 | 1.00 | 44.97 | C |
| ATOM | 5056 | CB | ASN | B | 361 | 17.222 | 25.006 | 40.483 | 1.00 | 44.24 | C |
| ATOM | 5057 | CG | ASN | B | 361 | 15.948 | 24.237 | 40.753 | 1.00 | 43.92 | C |
| ATOM | 5058 | OD1 | ASN | B | 361 | 15.928 | 23.003 | 40.724 | 1.00 | 44.06 | O |
| ATOM | 5059 | ND2 | ASN | B | 361 | 14.873 | 24.962 | 41.012 | 1.00 | 43.89 | N |
| ATOM | 5060 | C | ASN | B | 361 | 18.281 | 24.271 | 42.621 | 1.00 | 45.51 | C |
| ATOM | 5061 | O | ASN | B | 361 | 17.215 | 24.587 | 43.150 | 1.00 | 46.73 | O |
| ATOM | 5062 | N | PHE | B | 362 | 19.338 | 23.838 | 43.305 | 1.00 | 45.84 | N |
| ATOM | 5063 | CA | PHE | B | 362 | 19.335 | 23.716 | 44.761 | 1.00 | 46.06 | C |
| ATOM | 5064 | CB | PHE | B | 362 | 20.550 | 22.917 | 45.224 | 1.00 | 43.65 | C |
| ATOM | 5065 | CG | PHE | B | 362 | 20.472 | 21.461 | 44.901 | 1.00 | 42.66 | C |
| ATOM | 5066 | CD1 | PHE | B | 362 | 19.766 | 20.592 | 45.728 | 1.00 | 42.40 | C |
| ATOM | 5067 | CD2 | PHE | B | 362 | 21.075 | 20.955 | 43.756 | 1.00 | 41.35 | C |
| ATOM | 5068 | CE1 | PHE | B | 362 | 19.668 | 19.242 | 45.415 | 1.00 | 42.39 | C |
| ATOM | 5069 | CE2 | PHE | B | 362 | 20.980 | 19.605 | 43.434 | 1.00 | 40.77 | C |
| ATOM | 5070 | CZ | PHE | B | 362 | 20.275 | 18.745 | 44.263 | 1.00 | 41.01 | C |
| ATOM | 5071 | C | PHE | B | 362 | 19.372 | 25.084 | 45.427 | 1.00 | 47.31 | C |
| ATOM | 5072 | O | PHE | B | 362 | 19.681 | 26.086 | 44.790 | 1.00 | 48.21 | O |
| ATOM | 5073 | N | THR | B | 363 | 19.054 | 25.117 | 46.713 | 1.00 | 49.13 | N |
| ATOM | 5074 | CA | THR | B | 363 | 19.086 | 26.347 | 47.483 | 1.00 | 51.04 | C |
| ATOM | 5075 | CB | THR | B | 363 | 17.693 | 26.704 | 48.049 | 1.00 | 50.93 | C |
| ATOM | 5076 | OG1 | THR | B | 363 | 17.197 | 25.614 | 48.834 | 1.00 | 52.12 | O |
| ATOM | 5077 | CG2 | THR | B | 363 | 16.722 | 26.997 | 46.914 | 1.00 | 51.15 | C |
| ATOM | 5078 | C | THR | B | 363 | 20.054 | 26.095 | 48.637 | 1.00 | 53.49 | C |
| ATOM | 5079 | O | THR | B | 363 | 20.429 | 24.948 | 48.908 | 1.00 | 53.98 | O |
| ATOM | 5080 | N | THR | B | 364 | 20.473 | 27.158 | 49.315 | 1.00 | 54.85 | N |
| ATOM | 5081 | CA | THR | B | 364 | 21.400 | 27.015 | 50.430 | 1.00 | 55.54 | C |
| ATOM | 5082 | CB | THR | B | 364 | 21.858 | 28.382 | 50.945 | 1.00 | 56.65 | C |
| ATOM | 5083 | OG1 | THR | B | 364 | 20.746 | 29.070 | 51.531 | 1.00 | 58.82 | O |
| ATOM | 5084 | CG2 | THR | B | 364 | 22.416 | 29.217 | 49.800 | 1.00 | 57.10 | C |
| ATOM | 5085 | C | THR | B | 364 | 20.713 | 26.254 | 51.555 | 1.00 | 55.72 | C |

| | | | | | | | | | | | |
|---|---|---|---|---|---|---|---|---|---|---|---|
| ATOM | 5086 | O | THR | B | 364 | 21.362 | 25.601 | 52.382 | 1.00 | 55.80 | O |
| ATOM | 5087 | N | GLN | B | 365 | 19.384 | 26.317 | 51.556 | 1.00 | 55.24 | N |
| ATOM | 5088 | CA | GLN | B | 365 | 18.567 | 25.640 | 52.558 | 1.00 | 54.12 | C |
| ATOM | 5089 | CB | GLN | B | 365 | 17.138 | 26.179 | 52.516 | 1.00 | 54.90 | C |
| ATOM | 5090 | CG | GLN | B | 365 | 16.169 | 25.494 | 53.469 | 1.00 | 56.65 | C |
| ATOM | 5091 | CD | GLN | B | 365 | 16.669 | 25.490 | 54.896 | 1.00 | 57.41 | C |
| ATOM | 5092 | OE1 | GLN | B | 365 | 17.587 | 26.238 | 55.243 | 1.00 | 59.70 | O |
| ATOM | 5093 | NE2 | GLN | B | 365 | 16.061 | 24.658 | 55.738 | 1.00 | 56.07 | N |
| ATOM | 5094 | C | GLN | B | 365 | 18.545 | 24.126 | 52.372 | 1.00 | 53.86 | C |
| ATOM | 5095 | O | GLN | B | 365 | 18.421 | 23.388 | 53.349 | 1.00 | 54.08 | O |
| ATOM | 5096 | N | GLU | B | 366 | 18.649 | 23.658 | 51.129 | 1.00 | 53.51 | N |
| ATOM | 5097 | CA | GLU | B | 366 | 18.653 | 22.211 | 50.885 | 1.00 | 53.13 | C |
| ATOM | 5098 | CB | GLU | B | 366 | 18.170 | 21.857 | 49.488 | 1.00 | 52.88 | C |
| ATOM | 5099 | CG | GLU | B | 366 | 16.923 | 22.527 | 49.018 | 1.00 | 52.75 | C |
| ATOM | 5100 | CD | GLU | B | 366 | 16.689 | 22.239 | 47.554 | 1.00 | 51.60 | C |
| ATOM | 5101 | OE1 | GLU | B | 366 | 16.451 | 21.064 | 47.203 | 1.00 | 50.57 | O |
| ATOM | 5102 | OE2 | GLU | B | 366 | 16.764 | 23.183 | 46.753 | 1.00 | 52.39 | O |
| ATOM | 5103 | C | GLU | B | 366 | 20.065 | 21.668 | 50.998 | 1.00 | 52.96 | C |
| ATOM | 5104 | O | GLU | B | 366 | 20.279 | 20.590 | 51.547 | 1.00 | 53.21 | O |
| ATOM | 5105 | N | LEU | B | 367 | 21.023 | 22.407 | 50.445 | 1.00 | 52.32 | N |
| ATOM | 5106 | CA | LEU | B | 367 | 22.410 | 21.972 | 50.490 | 1.00 | 52.83 | C |
| ATOM | 5107 | CB | LEU | B | 367 | 23.250 | 22.797 | 49.503 | 1.00 | 51.78 | C |
| ATOM | 5108 | CG | LEU | B | 367 | 23.008 | 22.342 | 48.044 | 1.00 | 51.53 | C |
| ATOM | 5109 | CD1 | LEU | B | 367 | 23.558 | 23.343 | 47.034 | 1.00 | 49.76 | C |
| ATOM | 5110 | CD2 | LEU | B | 367 | 23.657 | 20.981 | 47.850 | 1.00 | 50.27 | C |
| ATOM | 5111 | C | LEU | B | 367 | 22.926 | 22.080 | 51.927 | 1.00 | 53.44 | C |
| ATOM | 5112 | O | LEU | B | 367 | 23.918 | 21.459 | 52.306 | 1.00 | 53.22 | O |
| ATOM | 5113 | N | SER | B | 368 | 22.188 | 22.844 | 52.729 | 1.00 | 54.21 | N |
| ATOM | 5114 | CA | SER | B | 368 | 22.466 | 23.081 | 54.141 | 1.00 | 53.18 | C |
| ATOM | 5115 | CB | SER | B | 368 | 21.141 | 23.432 | 54.834 | 1.00 | 54.06 | C |
| ATOM | 5116 | OG | SER | B | 368 | 21.146 | 23.099 | 56.216 | 1.00 | 56.49 | O |
| ATOM | 5117 | C | SER | B | 368 | 23.169 | 21.925 | 54.876 | 1.00 | 52.75 | C |
| ATOM | 5118 | O | SER | B | 368 | 24.342 | 22.032 | 55.243 | 1.00 | 53.42 | O |
| ATOM | 5119 | N | SER | B | 369 | 22.460 | 20.821 | 55.081 | 1.00 | 51.87 | N |
| ATOM | 5120 | CA | SER | B | 369 | 23.014 | 19.660 | 55.787 | 1.00 | 50.27 | C |
| ATOM | 5121 | CB | SER | B | 369 | 22.140 | 18.425 | 55.553 | 1.00 | 49.77 | C |
| ATOM | 5122 | OG | SER | B | 369 | 22.610 | 17.690 | 54.419 | 1.00 | 49.98 | O |
| ATOM | 5123 | C | SER | B | 369 | 24.443 | 19.261 | 55.414 | 1.00 | 49.44 | C |
| ATOM | 5124 | O | SER | B | 369 | 25.112 | 18.570 | 56.196 | 1.00 | 49.84 | O |
| ATOM | 5125 | N | ASN | B | 370 | 24.921 | 19.679 | 54.245 | 1.00 | 48.78 | N |
| ATOM | 5126 | CA | ASN | B | 370 | 26.249 | 19.241 | 53.798 | 1.00 | 48.50 | C |
| ATOM | 5127 | CB | ASN | B | 370 | 26.162 | 17.733 | 53.560 | 1.00 | 48.31 | C |
| ATOM | 5128 | CG | ASN | B | 370 | 27.488 | 17.102 | 53.234 | 1.00 | 49.87 | C |
| ATOM | 5129 | OD1 | ASN | B | 370 | 28.406 | 17.760 | 52.750 | 1.00 | 50.16 | O |
| ATOM | 5130 | ND2 | ASN | B | 370 | 27.588 | 15.797 | 53.475 | 1.00 | 50.62 | N |
| ATOM | 5131 | C | ASN | B | 370 | 26.664 | 19.956 | 52.494 | 1.00 | 47.98 | C |
| ATOM | 5132 | O | ASN | B | 370 | 26.928 | 19.313 | 51.474 | 1.00 | 47.76 | O |
| ATOM | 5133 | N | PRO | B | 371 | 26.759 | 21.294 | 52.525 | 1.00 | 47.47 | N |
| ATOM | 5134 | CD | PRO | B | 371 | 26.829 | 22.074 | 53.774 | 1.00 | 46.61 | C |
| ATOM | 5135 | CA | PRO | B | 371 | 27.127 | 22.131 | 51.373 | 1.00 | 47.84 | C |
| ATOM | 5136 | CB | PRO | B | 371 | 27.636 | 23.409 | 52.029 | 1.00 | 47.25 | C |
| ATOM | 5137 | CG | PRO | B | 371 | 26.788 | 23.480 | 53.267 | 1.00 | 46.45 | C |
| ATOM | 5138 | C | PRO | B | 371 | 28.118 | 21.594 | 50.325 | 1.00 | 49.23 | C |
| ATOM | 5139 | O | PRO | B | 371 | 27.843 | 21.628 | 49.124 | 1.00 | 49.10 | O |
| ATOM | 5140 | N | PRO | B | 372 | 29.285 | 21.093 | 50.757 | 1.00 | 50.90 | N |
| ATOM | 5141 | CD | PRO | B | 372 | 29.718 | 20.821 | 52.140 | 1.00 | 51.43 | C |
| ATOM | 5142 | CA | PRO | B | 372 | 30.273 | 20.580 | 49.803 | 1.00 | 51.19 | C |
| ATOM | 5143 | CB | PRO | B | 372 | 31.160 | 19.694 | 50.668 | 1.00 | 49.99 | C |
| ATOM | 5144 | CG | PRO | B | 372 | 31.178 | 20.432 | 51.949 | 1.00 | 51.02 | C |
| ATOM | 5145 | C | PRO | B | 372 | 29.667 | 19.818 | 48.634 | 1.00 | 52.40 | C |
| ATOM | 5146 | O | PRO | B | 372 | 29.969 | 20.099 | 47.471 | 1.00 | 53.46 | O |
| ATOM | 5147 | N | LEU | B | 373 | 28.793 | 18.867 | 48.939 | 1.00 | 51.98 | N |
| ATOM | 5148 | CA | LEU | B | 373 | 28.177 | 18.048 | 47.892 | 1.00 | 52.28 | C |
| ATOM | 5149 | CB | LEU | B | 373 | 27.079 | 17.164 | 48.484 | 1.00 | 51.80 | C |
| ATOM | 5150 | CG | LEU | B | 373 | 27.578 | 16.028 | 49.379 | 1.00 | 51.59 | C |
| ATOM | 5151 | CD1 | LEU | B | 373 | 26.378 | 15.245 | 49.878 | 1.00 | 52.02 | C |
| ATOM | 5152 | CD2 | LEU | B | 373 | 28.529 | 15.122 | 48.615 | 1.00 | 50.53 | C |

| | | | | | | | | | | | |
|---|---|---|---|---|---|---|---|---|---|---|---|
| ATOM | 5153 | C | LEU | B | 373 | 27.634 | 18.794 | 46.673 | 1.00 | 52.48 | C |
| ATOM | 5154 | O | LEU | B | 373 | 27.250 | 18.169 | 45.680 | 1.00 | 53.07 | O |
| ATOM | 5155 | N | ALA | B | 374 | 27.609 | 20.123 | 46.737 | 1.00 | 52.06 | N |
| ATOM | 5156 | CA | ALA | B | 374 | 27.138 | 20.917 | 45.612 | 1.00 | 51.75 | C |
| ATOM | 5157 | CB | ALA | B | 374 | 27.090 | 22.392 | 45.988 | 1.00 | 50.24 | C |
| ATOM | 5158 | C | ALA | B | 374 | 28.055 | 20.713 | 44.416 | 1.00 | 52.19 | C |
| ATOM | 5159 | O | ALA | B | 374 | 27.646 | 20.918 | 43.276 | 1.00 | 53.14 | O |
| ATOM | 5160 | N | THR | B | 375 | 29.294 | 20.302 | 44.666 | 1.00 | 52.86 | N |
| ATOM | 5161 | CA | THR | B | 375 | 30.230 | 20.088 | 43.564 | 1.00 | 53.37 | C |
| ATOM | 5162 | CB | THR | B | 375 | 31.702 | 19.982 | 44.059 | 1.00 | 53.97 | C |
| ATOM | 5163 | OG1 | THR | B | 375 | 31.778 | 19.104 | 45.194 | 1.00 | 55.14 | O |
| ATOM | 5164 | CG2 | THR | B | 375 | 32.238 | 21.356 | 44.438 | 1.00 | 53.35 | C |
| ATOM | 5165 | C | THR | B | 375 | 29.871 | 18.835 | 42.780 | 1.00 | 52.71 | C |
| ATOM | 5166 | O | THR | B | 375 | 30.336 | 18.648 | 41.660 | 1.00 | 52.13 | O |
| ATOM | 5167 | N | ILE | B | 376 | 29.042 | 17.982 | 43.381 | 1.00 | 52.68 | N |
| ATOM | 5168 | CA | ILE | B | 376 | 28.594 | 16.750 | 42.730 | 1.00 | 52.62 | C |
| ATOM | 5169 | CB | ILE | B | 376 | 28.569 | 15.548 | 43.699 | 1.00 | 53.20 | C |
| ATOM | 5170 | CG2 | ILE | B | 376 | 27.994 | 14.326 | 42.977 | 1.00 | 53.26 | C |
| ATOM | 5171 | CG1 | ILE | B | 376 | 29.969 | 15.240 | 44.224 | 1.00 | 52.88 | C |
| ATOM | 5172 | CD1 | ILE | B | 376 | 29.990 | 14.139 | 45.281 | 1.00 | 52.91 | C |
| ATOM | 5173 | C | ILE | B | 376 | 27.165 | 16.907 | 42.208 | 1.00 | 51.87 | C |
| ATOM | 5174 | O | ILE | B | 376 | 26.852 | 16.465 | 41.106 | 1.00 | 52.55 | O |
| ATOM | 5175 | N | LEU | B | 377 | 26.306 | 17.520 | 43.018 | 1.00 | 50.96 | N |
| ATOM | 5176 | CA | LEU | B | 377 | 24.901 | 17.720 | 42.664 | 1.00 | 50.30 | C |
| ATOM | 5177 | CB | LEU | B | 377 | 24.114 | 18.160 | 43.899 | 1.00 | 49.01 | C |
| ATOM | 5178 | CG | LEU | B | 377 | 24.080 | 17.171 | 45.070 | 1.00 | 48.18 | C |
| ATOM | 5179 | CD1 | LEU | B | 377 | 23.476 | 17.852 | 46.290 | 1.00 | 47.40 | C |
| ATOM | 5180 | CD2 | LEU | B | 377 | 23.287 | 15.936 | 44.690 | 1.00 | 47.10 | C |
| ATOM | 5181 | C | LEU | B | 377 | 24.687 | 18.725 | 41.538 | 1.00 | 50.17 | C |
| ATOM | 5182 | O | LEU | B | 377 | 23.982 | 18.434 | 40.577 | 1.00 | 49.31 | O |
| ATOM | 5183 | N | ILE | B | 378 | 25.273 | 19.912 | 41.670 | 1.00 | 51.32 | N |
| ATOM | 5184 | CA | ILE | B | 378 | 25.145 | 20.941 | 40.642 | 1.00 | 52.46 | C |
| ATOM | 5185 | CB | ILE | B | 378 | 25.262 | 22.363 | 41.243 | 1.00 | 51.17 | C |
| ATOM | 5186 | CG2 | ILE | B | 378 | 24.793 | 23.400 | 40.232 | 1.00 | 51.31 | C |
| ATOM | 5187 | CG1 | ILE | B | 378 | 24.387 | 22.486 | 42.485 | 1.00 | 51.24 | C |
| ATOM | 5188 | CD1 | ILE | B | 378 | 24.605 | 23.772 | 43.259 | 1.00 | 51.32 | C |
| ATOM | 5189 | C | ILE | B | 378 | 26.287 | 20.725 | 39.651 | 1.00 | 53.97 | C |
| ATOM | 5190 | O | ILE | B | 378 | 27.381 | 21.245 | 39.838 | 1.00 | 54.82 | O |
| ATOM | 5191 | N | PRO | B | 379 | 26.045 | 19.953 | 38.581 | 1.00 | 55.70 | N |
| ATOM | 5192 | CD | PRO | B | 379 | 24.734 | 19.497 | 38.080 | 1.00 | 55.96 | C |
| ATOM | 5193 | CA | PRO | B | 379 | 27.093 | 19.696 | 37.593 | 1.00 | 57.08 | C |
| ATOM | 5194 | CB | PRO | B | 379 | 26.417 | 18.731 | 36.632 | 1.00 | 57.15 | C |
| ATOM | 5195 | CG | PRO | B | 379 | 25.011 | 19.237 | 36.614 | 1.00 | 56.37 | C |
| ATOM | 5196 | C | PRO | B | 379 | 27.565 | 20.978 | 36.908 | 1.00 | 59.33 | C |
| ATOM | 5197 | O | PRO | B | 379 | 26.938 | 22.037 | 37.041 | 1.00 | 59.58 | O |
| ATOM | 5198 | N | PRO | B | 380 | 28.677 | 20.900 | 36.158 | 1.00 | 61.17 | N |
| ATOM | 5199 | CD | PRO | B | 380 | 29.528 | 19.707 | 35.992 | 1.00 | 61.80 | C |
| ATOM | 5200 | CA | PRO | B | 380 | 29.253 | 22.051 | 35.441 | 1.00 | 62.31 | C |
| ATOM | 5201 | CB | PRO | B | 380 | 30.445 | 21.439 | 34.700 | 1.00 | 62.07 | C |
| ATOM | 5202 | CG | PRO | B | 380 | 30.862 | 20.321 | 35.604 | 1.00 | 62.45 | C |
| ATOM | 5203 | C | PRO | B | 380 | 28.264 | 22.707 | 34.480 | 1.00 | 62.86 | C |
| ATOM | 5204 | O | PRO | B | 380 | 27.927 | 23.884 | 34.626 | 1.00 | 62.45 | O |
| ATOM | 5205 | N | HIS | B | 381 | 27.802 | 21.932 | 33.502 | 1.00 | 63.51 | N |
| ATOM | 5206 | CA | HIS | B | 381 | 26.856 | 22.423 | 32.506 | 1.00 | 64.68 | C |
| ATOM | 5207 | CB | HIS | B | 381 | 26.447 | 21.288 | 31.564 | 1.00 | 65.82 | C |
| ATOM | 5208 | CG | HIS | B | 381 | 25.448 | 20.347 | 32.161 | 1.00 | 66.65 | C |
| ATOM | 5209 | CD2 | HIS | B | 381 | 25.566 | 19.060 | 32.567 | 1.00 | 66.94 | C |
| ATOM | 5210 | ND1 | HIS | B | 381 | 24.153 | 20.726 | 32.453 | 1.00 | 66.51 | N |
| ATOM | 5211 | CE1 | HIS | B | 381 | 23.518 | 19.713 | 33.015 | 1.00 | 66.98 | C |
| ATOM | 5212 | NE2 | HIS | B | 381 | 24.351 | 18.689 | 33.097 | 1.00 | 67.44 | N |
| ATOM | 5213 | C | HIS | B | 381 | 25.600 | 23.001 | 33.150 | 1.00 | 65.89 | C |
| ATOM | 5214 | O | HIS | B | 381 | 24.680 | 23.428 | 32.452 | 1.00 | 65.88 | O |
| ATOM | 5215 | N | ALA | B | 382 | 25.548 | 22.993 | 34.479 | 1.00 | 67.50 | N |
| ATOM | 5216 | CA | ALA | B | 382 | 24.396 | 23.533 | 35.193 | 1.00 | 68.57 | C |
| ATOM | 5217 | CB | ALA | B | 382 | 23.837 | 22.487 | 36.156 | 1.00 | 67.99 | C |
| ATOM | 5218 | C | ALA | B | 382 | 24.760 | 24.815 | 35.946 | 1.00 | 69.73 | C |
| ATOM | 5219 | O | ALA | B | 382 | 23.889 | 25.635 | 36.237 | 1.00 | 70.17 | O |

| | | | | | | | | | | | |
|---|---|---|---|---|---|---|---|---|---|---|---|
| ATOM | 5220 | N | ARG | B | 383 | 26.041 | 24.985 | 36.273 | 1.00 | 71.28 | N |
| ATOM | 5221 | CA | ARG | B | 383 | 26.484 | 26.191 | 36.978 | 1.00 | 72.20 | C |
| ATOM | 5222 | CB | ARG | B | 383 | 27.968 | 26.091 | 37.377 | 1.00 | 71.94 | C |
| ATOM | 5223 | CG | ARG | B | 383 | 28.265 | 25.174 | 38.550 | 1.00 | 71.48 | C |
| ATOM | 5224 | CD | ARG | B | 383 | 28.698 | 23.787 | 38.098 | 1.00 | 71.61 | C |
| ATOM | 5225 | NE | ARG | B | 383 | 30.137 | 23.594 | 38.276 | 1.00 | 72.36 | N |
| ATOM | 5226 | CZ | ARG | B | 383 | 30.684 | 22.790 | 39.188 | 1.00 | 72.86 | C |
| ATOM | 5227 | NH1 | ARG | B | 383 | 29.914 | 22.092 | 40.012 | 1.00 | 72.83 | N |
| ATOM | 5228 | NH2 | ARG | B | 383 | 32.008 | 22.692 | 39.285 | 1.00 | 73.15 | N |
| ATOM | 5229 | C | ARG | B | 383 | 26.301 | 27.408 | 36.085 | 1.00 | 73.05 | C |
| ATOM | 5230 | O | ARG | B | 383 | 25.474 | 28.285 | 36.427 | 1.00 | 73.47 | O |
| ATOM | 5231 | OXT | ARG | B | 383 | 27.005 | 27.460 | 35.050 | 1.00 | 73.90 | O |
| TER | 5232 | | ARG | B | 383 | | | | | | |
| ATOM | 5233 | O | HOH | W | 1 | -1.266 | 8.542 | -13.084 | 1.00 | 14.72 | O |
| ATOM | 5234 | O | HOH | W | 2 | -2.998 | 9.432 | -6.812 | 1.00 | 15.65 | O |
| ATOM | 5235 | O | HOH | W | 3 | 9.785 | -1.916 | 56.208 | 1.00 | 19.78 | O |
| ATOM | 5236 | O | HOH | W | 4 | 8.933 | 9.805 | 29.446 | 1.00 | 24.72 | O |
| ATOM | 5237 | O | HOH | W | 5 | 1.827 | -0.751 | 54.225 | 1.00 | 21.82 | O |
| ATOM | 5238 | O | HOH | W | 6 | -10.281 | 12.225 | -5.503 | 1.00 | 26.73 | O |
| ATOM | 5239 | O | HOH | W | 7 | -9.904 | 21.576 | -5.887 | 1.00 | 31.82 | O |
| ATOM | 5240 | O | HOH | W | 8 | 3.618 | 11.961 | 67.656 | 1.00 | 32.99 | O |
| ATOM | 5241 | O | HOH | W | 9 | 6.850 | 7.948 | -10.496 | 1.00 | 26.54 | O |
| ATOM | 5242 | O | HOH | W | 10 | 1.475 | 16.642 | -11.459 | 1.00 | 30.73 | O |
| ATOM | 5243 | O | HOH | W | 11 | -9.038 | 32.369 | 55.850 | 1.00 | 36.07 | O |
| ATOM | 5244 | O | HOH | W | 12 | 5.386 | -2.421 | 63.540 | 1.00 | 45.57 | O |
| ATOM | 5245 | O | HOH | W | 13 | -3.401 | 13.202 | 9.117 | 1.00 | 39.47 | O |
| ATOM | 5246 | O | HOH | W | 14 | -5.902 | -8.009 | 9.752 | 1.00 | 31.40 | O |
| ATOM | 5247 | O | HOH | W | 15 | 2.459 | 7.676 | 2.942 | 1.00 | 24.28 | O |
| ATOM | 5248 | O | HOH | W | 16 | 1.212 | -10.287 | -15.241 | 1.00 | 25.00 | O |
| ATOM | 5249 | O | HOH | W | 17 | 22.684 | 16.165 | 41.069 | 1.00 | 44.25 | O |
| ATOM | 5250 | O | HOH | W | 18 | 14.185 | 18.047 | 41.501 | 1.00 | 35.23 | O |
| ATOM | 5251 | O | HOH | W | 19 | -12.340 | 30.469 | 55.509 | 1.00 | 48.41 | O |
| ATOM | 5252 | O | HOH | W | 20 | 15.295 | 3.090 | 31.834 | 1.00 | 39.30 | O |
| ATOM | 5253 | O | HOH | W | 21 | -7.619 | 16.792 | 4.653 | 1.00 | 49.13 | O |
| ATOM | 5254 | O | HOH | W | 22 | 1.509 | -0.311 | 48.741 | 1.00 | 37.76 | O |
| ATOM | 5255 | O | HOH | W | 23 | -0.729 | -16.958 | -12.544 | 1.00 | 50.10 | O |
| ATOM | 5256 | O | HOH | W | 24 | -14.842 | 6.544 | -6.115 | 1.00 | 26.75 | O |
| ATOM | 5257 | O | HOH | W | 25 | 3.589 | 11.159 | -1.679 | 1.00 | 49.96 | O |
| ATOM | 5258 | O | HOH | W | 26 | 8.607 | 7.447 | 55.916 | 1.00 | 37.34 | O |
| ATOM | 5259 | O | HOH | W | 27 | -5.490 | -10.158 | 3.059 | 1.00 | 35.71 | O |
| ATOM | 5260 | O | HOH | W | 28 | 1.923 | 4.482 | -14.833 | 1.00 | 22.25 | O |
| ATOM | 5261 | O | HOH | W | 29 | 19.763 | 15.158 | 53.621 | 1.00 | 41.36 | O |
| ATOM | 5262 | O | HOH | W | 30 | -14.137 | -3.358 | 13.898 | 1.00 | 52.64 | O |
| ATOM | 5263 | O | HOH | W | 31 | 17.776 | 28.660 | 51.568 | 1.00 | 37.35 | O |
| ATOM | 5264 | O | HOH | W | 32 | 9.817 | 4.643 | 58.639 | 1.00 | 18.67 | O |
| ATOM | 5265 | O | HOH | W | 33 | 8.837 | 6.465 | 24.658 | 1.00 | 38.39 | O |
| ATOM | 5266 | O | HOH | W | 34 | 4.942 | -3.799 | 20.145 | 1.00 | 38.77 | O |
| ATOM | 5267 | O | HOH | W | 35 | 12.047 | -2.552 | 4.756 | 1.00 | 25.11 | O |
| ATOM | 5268 | O | HOH | W | 36 | 9.465 | 4.048 | 5.637 | 1.00 | 31.42 | O |
| ATOM | 5269 | O | HOH | W | 37 | 12.428 | -1.209 | -3.858 | 1.00 | 30.72 | O |
| ATOM | 5270 | O | HOH | W | 38 | 7.410 | -14.265 | -6.580 | 1.00 | 43.24 | O |
| ATOM | 5271 | O | HOH | W | 39 | 9.313 | 5.019 | -0.505 | 1.00 | 47.49 | O |
| ATOM | 5272 | O | HOH | W | 40 | 8.620 | 13.251 | 8.315 | 1.00 | 43.76 | O |
| ATOM | 5273 | O | HOH | W | 41 | 8.786 | 9.151 | 3.874 | 1.00 | 37.23 | O |
| ATOM | 5274 | O | HOH | W | 42 | -1.331 | 18.415 | 2.847 | 1.00 | 30.70 | O |
| ATOM | 5275 | O | HOH | W | 43 | 6.336 | 14.830 | -3.593 | 1.00 | 28.35 | O |
| ATOM | 5276 | O | HOH | W | 44 | 1.876 | 2.309 | -13.627 | 1.00 | 26.10 | O |
| ATOM | 5277 | O | HOH | W | 45 | -3.191 | 21.138 | -16.692 | 1.00 | 40.52 | O |
| ATOM | 5278 | O | HOH | W | 46 | -15.049 | 17.703 | -5.989 | 1.00 | 49.15 | O |
| ATOM | 5279 | O | HOH | W | 47 | 7.775 | -2.356 | -13.990 | 1.00 | 38.74 | O |
| ATOM | 5280 | O | HOH | W | 48 | 3.494 | 3.720 | -22.134 | 1.00 | 37.42 | O |
| ATOM | 5281 | O | HOH | W | 49 | 12.528 | 17.436 | -19.899 | 1.00 | 41.37 | O |
| ATOM | 5282 | O | HOH | W | 50 | 7.521 | 18.039 | -13.046 | 1.00 | 45.90 | O |
| ATOM | 5283 | O | HOH | W | 51 | 4.285 | 16.633 | -23.145 | 1.00 | 44.71 | O |
| ATOM | 5284 | O | HOH | W | 52 | -10.845 | 3.884 | -22.045 | 1.00 | 38.68 | O |
| ATOM | 5285 | O | HOH | W | 53 | -13.228 | 7.970 | -20.498 | 1.00 | 36.86 | O |
| ATOM | 5286 | O | HOH | W | 54 | -12.353 | 18.434 | -23.038 | 1.00 | 39.41 | O |

| | | | | | | | | | | | |
|---|---|---|---|---|---|---|---|---|---|---|---|
| ATOM | 5287 | O | HOH | W | 55 | -19.736 | 12.560 | -15.750 | 1.00 | 40.32 | O |
| ATOM | 5288 | O | HOH | W | 56 | -18.614 | 7.306 | -5.565 | 1.00 | 29.06 | O |
| ATOM | 5289 | O | HOH | W | 57 | -18.866 | 3.114 | -6.902 | 1.00 | 29.87 | O |
| ATOM | 5290 | O | HOH | W | 58 | 4.548 | -19.094 | -4.472 | 1.00 | 30.15 | O |
| ATOM | 5291 | O | HOH | W | 59 | -1.562 | 31.086 | 46.525 | 1.00 | 35.60 | O |
| ATOM | 5292 | O | HOH | W | 60 | -16.129 | 21.933 | 50.459 | 1.00 | 35.02 | O |
| ATOM | 5293 | O | HOH | W | 61 | -17.916 | 25.194 | 51.475 | 1.00 | 48.13 | O |
| ATOM | 5294 | O | HOH | W | 62 | -16.095 | 15.055 | 36.400 | 1.00 | 44.80 | O |
| ATOM | 5295 | O | HOH | W | 63 | -4.710 | 22.147 | 36.647 | 1.00 | 42.49 | O |
| ATOM | 5296 | O | HOH | W | 64 | -4.377 | 14.277 | 43.981 | 1.00 | 22.95 | O |
| ATOM | 5297 | O | HOH | W | 65 | -0.564 | 13.610 | 37.656 | 1.00 | 59.25 | O |
| ATOM | 5298 | O | HOH | W | 66 | 17.908 | 13.912 | 56.928 | 1.00 | 37.58 | O |
| ATOM | 5299 | O | HOH | W | 67 | -0.786 | 17.340 | 53.673 | 1.00 | 48.99 | O |
| ATOM | 5300 | O | HOH | W | 68 | 6.846 | 16.376 | 70.823 | 1.00 | 47.26 | O |
| ATOM | 5301 | O | HOH | W | 69 | 6.328 | 15.810 | 76.044 | 1.00 | 54.44 | O |
| ATOM | 5302 | O | HOH | W | 70 | 0.718 | 5.223 | 68.861 | 1.00 | 42.42 | O |
| ATOM | 5303 | O | HOH | W | 71 | 4.796 | 8.735 | 72.668 | 1.00 | 42.23 | O |
| ATOM | 5304 | O | HOH | W | 72 | -0.679 | 4.187 | 61.277 | 1.00 | 39.55 | O |
| ATOM | 5305 | O | HOH | W | 73 | 15.671 | 6.652 | 62.858 | 1.00 | 25.65 | O |
| ATOM | 5306 | O | HOH | W | 74 | 8.704 | -1.527 | 49.188 | 1.00 | 23.45 | O |
| ATOM | 5307 | O | HOH | W | 75 | 26.656 | -5.532 | 62.975 | 1.00 | 38.36 | O |
| ATOM | 5308 | O | HOH | W | 76 | 26.316 | 8.554 | 56.662 | 1.00 | 33.50 | O |
| ATOM | 5309 | O | HOH | W | 77 | 28.841 | 6.565 | 52.139 | 1.00 | 53.34 | O |
| ATOM | 5310 | O | HOH | W | 78 | 21.438 | 8.185 | 58.552 | 1.00 | 32.74 | O |
| ATOM | 5311 | O | HOH | W | 79 | 21.306 | 17.170 | 32.847 | 1.00 | 41.33 | O |
| ATOM | 5312 | O | HOH | W | 80 | 5.009 | 19.405 | 35.953 | 1.00 | 38.17 | O |
| ATOM | 5313 | O | HOH | W | 81 | -18.006 | 10.433 | -20.033 | 1.00 | 44.97 | O |
| ATOM | 5314 | O | HOH | W | 82 | -16.672 | 17.338 | -24.015 | 1.00 | 44.37 | O |
| ATOM | 5315 | O | HOH | W | 83 | -13.487 | 10.522 | -22.044 | 1.00 | 44.93 | O |
| TER | 5316 | | HOH | W | 83 | | | | | | |
| END | | | | | | | | | | | |

# FIG. 3

| | | Atom Type | Resid | | # | X | Y | Z | OCC | B | | |
|---|---|---|---|---|---|---|---|---|---|---|---|---|
| ATOM | 1 | CB | VAL | A | 37 | 23.813 | -4.451 | 22.235 | 1.00 | 53.77 | A | C |
| ATOM | 2 | CG1 | VAL | A | 37 | 22.591 | -4.098 | 23.069 | 1.00 | 53.77 | A | C |
| ATOM | 3 | CG2 | VAL | A | 37 | 23.675 | -5.901 | 21.733 | 1.00 | 53.77 | A | C |
| ATOM | 4 | C | VAL | A | 37 | 23.455 | -2.090 | 21.514 | 1.00 | 51.96 | A | C |
| ATOM | 5 | O | VAL | A | 37 | 24.096 | -1.462 | 22.344 | 1.00 | 51.96 | A | O |
| ATOM | 6 | N | VAL | A | 37 | 25.303 | -3.477 | 20.430 | 1.00 | 51.96 | A | N |
| ATOM | 7 | CA | VAL | A | 37 | 23.929 | -3.466 | 21.034 | 1.00 | 51.96 | A | C |
| ATOM | 8 | N | THR | A | 38 | 22.317 | -1.640 | 20.987 | 1.00 | 55.35 | A | N |
| ATOM | 9 | CA | THR | A | 38 | 21.736 | -0.353 | 21.363 | 1.00 | 55.35 | A | C |
| ATOM | 10 | CB | THR | A | 38 | 21.245 | 0.429 | 20.138 | 1.00 | 57.52 | A | C |
| ATOM | 11 | OG1 | THR | A | 38 | 22.313 | 0.564 | 19.196 | 1.00 | 57.52 | A | O |
| ATOM | 12 | CG2 | THR | A | 38 | 20.768 | 1.807 | 20.545 | 1.00 | 57.52 | A | C |
| ATOM | 13 | C | THR | A | 38 | 20.531 | -0.597 | 22.261 | 1.00 | 55.35 | A | C |
| ATOM | 14 | O | THR | A | 38 | 19.774 | -1.546 | 22.039 | 1.00 | 55.35 | A | O |
| ATOM | 15 | N | THR | A | 39 | 20.370 | 0.251 | 23.278 | 1.00 | 61.66 | A | N |
| ATOM | 16 | CA | THR | A | 39 | 19.249 | 0.154 | 24.217 | 1.00 | 61.66 | A | C |
| ATOM | 17 | CB | THR | A | 39 | 19.716 | -0.268 | 25.635 | 1.00 | 40.02 | A | C |
| ATOM | 18 | OG1 | THR | A | 39 | 20.110 | -1.642 | 25.609 | 1.00 | 40.02 | A | O |
| ATOM | 19 | CG2 | THR | A | 39 | 18.594 | -0.092 | 26.664 | 1.00 | 40.02 | A | C |
| ATOM | 20 | C | THR | A | 39 | 18.537 | 1.501 | 24.310 | 1.00 | 61.66 | A | C |
| ATOM | 21 | O | THR | A | 39 | 19.149 | 2.527 | 24.634 | 1.00 | 61.66 | A | O |
| ATOM | 22 | N | VAL | A | 40 | 17.241 | 1.491 | 24.020 | 1.00 | 30.79 | A | N |
| ATOM | 23 | CA | VAL | A | 40 | 16.456 | 2.709 | 24.061 | 1.00 | 30.79 | A | C |
| ATOM | 24 | CB | VAL | A | 40 | 16.066 | 3.140 | 22.641 | 1.00 | 33.45 | A | C |
| ATOM | 25 | CG1 | VAL | A | 40 | 17.317 | 3.436 | 21.825 | 1.00 | 33.45 | A | C |
| ATOM | 26 | CG2 | VAL | A | 40 | 15.238 | 2.035 | 21.977 | 1.00 | 33.45 | A | C |
| ATOM | 27 | C | VAL | A | 40 | 15.191 | 2.492 | 24.858 | 1.00 | 30.79 | A | C |
| ATOM | 28 | O | VAL | A | 40 | 14.769 | 1.354 | 25.072 | 1.00 | 30.79 | A | O |
| ATOM | 29 | N | VAL | A | 41 | 14.593 | 3.579 | 25.319 | 1.00 | 45.23 | A | N |
| ATOM | 30 | CA | VAL | A | 41 | 13.339 | 3.465 | 26.054 | 1.00 | 45.23 | A | C |
| ATOM | 31 | CB | VAL | A | 41 | 13.293 | 4.389 | 27.301 | 1.00 | 34.90 | A | C |
| ATOM | 32 | CG1 | VAL | A | 41 | 11.905 | 4.357 | 27.932 | 1.00 | 34.90 | A | C |
| ATOM | 33 | CG2 | VAL | A | 41 | 14.328 | 3.929 | 28.312 | 1.00 | 34.90 | A | C |
| ATOM | 34 | C | VAL | A | 41 | 12.283 | 3.871 | 25.040 | 1.00 | 45.23 | A | C |
| ATOM | 35 | O | VAL | A | 41 | 12.155 | 5.047 | 24.682 | 1.00 | 45.23 | A | O |
| ATOM | 36 | N | ALA | A | 42 | 11.549 | 2.872 | 24.563 | 1.00 | 33.07 | A | N |
| ATOM | 37 | CA | ALA | A | 42 | 10.535 | 3.093 | 23.560 | 1.00 | 33.07 | A | C |
| ATOM | 38 | CB | ALA | A | 42 | 10.866 | 2.283 | 22.320 | 1.00 | 33.89 | A | C |
| ATOM | 39 | C | ALA | A | 42 | 9.115 | 2.796 | 23.986 | 1.00 | 33.07 | A | C |
| ATOM | 40 | O | ALA | A | 42 | 8.825 | 1.750 | 24.560 | 1.00 | 33.07 | A | O |
| ATOM | 41 | N | THR | A | 43 | 8.236 | 3.746 | 23.685 | 1.00 | 38.30 | A | N |
| ATOM | 42 | CA | THR | A | 43 | 6.810 | 3.622 | 23.947 | 1.00 | 38.30 | A | C |
| ATOM | 43 | CB | THR | A | 43 | 6.093 | 4.979 | 23.753 | 1.00 | 56.48 | A | C |
| ATOM | 44 | OG1 | THR | A | 43 | 6.918 | 6.043 | 24.248 | 1.00 | 56.48 | A | O |
| ATOM | 45 | CG2 | THR | A | 43 | 4.756 | 4.985 | 24.489 | 1.00 | 56.48 | A | C |
| ATOM | 46 | C | THR | A | 43 | 6.312 | 2.648 | 22.857 | 1.00 | 38.30 | A | C |
| ATOM | 47 | O | THR | A | 43 | 6.750 | 2.711 | 21.702 | 1.00 | 38.30 | A | O |
| ATOM | 48 | N | PRO | A | 44 | 5.404 | 1.731 | 23.209 | 1.00 | 49.45 | A | N |
| ATOM | 49 | CD | PRO | A | 44 | 4.949 | 1.359 | 24.556 | 1.00 | 43.65 | A | C |
| ATOM | 50 | CA | PRO | A | 44 | 4.902 | 0.782 | 22.214 | 1.00 | 49.45 | A | C |
| ATOM | 51 | CB | PRO | A | 44 | 4.121 | -0.223 | 23.056 | 1.00 | 43.65 | A | C |
| ATOM | 52 | CG | PRO | A | 44 | 4.784 | -0.134 | 24.411 | 1.00 | 43.65 | A | C |
| ATOM | 53 | C | PRO | A | 44 | 4.024 | 1.498 | 21.193 | 1.00 | 49.45 | A | C |
| ATOM | 54 | O | PRO | A | 44 | 3.408 | 2.522 | 21.511 | 1.00 | 49.45 | A | O |
| ATOM | 55 | N | GLY | A | 45 | 3.971 | 0.966 | 19.972 | 1.00 | 54.69 | A | N |
| ATOM | 56 | CA | GLY | A | 45 | 3.187 | 1.593 | 18.922 | 1.00 | 54.69 | A | C |
| ATOM | 57 | C | GLY | A | 45 | 1.721 | 1.730 | 19.257 | 1.00 | 54.69 | A | C |
| ATOM | 58 | O | GLY | A | 45 | 1.233 | 2.808 | 19.602 | 1.00 | 54.69 | A | O |
| ATOM | 59 | N | ALA | A | 46 | 1.011 | 0.618 | 19.146 | 1.00 | 84.98 | A | N |
| ATOM | 60 | CA | ALA | A | 46 | -0.413 | 0.592 | 19.437 | 1.00 | 84.98 | A | C |

| | | | | | | | | | | | | |
|---|---|---|---|---|---|---|---|---|---|---|---|---|
| ATOM | 61 | CB | ALA | A | 46 | -1.147 | -0.287 | 18.389 | 1.00 | 54.67 | A | C |
| ATOM | 62 | C | ALA | A | 46 | -0.566 | 0.007 | 20.843 | 1.00 | 84.98 | A | C |
| ATOM | 63 | O | ALA | A | 46 | -0.404 | -1.208 | 21.038 | 1.00 | 84.98 | A | O |
| ATOM | 64 | N | GLY | A | 47 | -0.859 | 0.862 | 21.822 | 1.00 | 58.86 | A | N |
| ATOM | 65 | CA | GLY | A | 47 | -1.008 | 0.354 | 23.172 | 1.00 | 58.86 | A | C |
| ATOM | 66 | C | GLY | A | 47 | -1.054 | 1.422 | 24.238 | 1.00 | 58.86 | A | C |
| ATOM | 67 | O | GLY | A | 47 | -1.374 | 2.573 | 23.945 | 1.00 | 58.86 | A | O |
| ATOM | 68 | N | PRO | A | 48 | -0.721 | 1.072 | 25.494 | 1.00 | 56.57 | A | N |
| ATOM | 69 | CD | PRO | A | 48 | 0.007 | -0.134 | 25.922 | 1.00 | 57.85 | A | C |
| ATOM | 70 | CA | PRO | A | 48 | -0.747 | 2.055 | 26.580 | 1.00 | 56.57 | A | C |
| ATOM | 71 | CB | PRO | A | 48 | -0.753 | 1.186 | 27.843 | 1.00 | 57.85 | A | C |
| ATOM | 72 | CG | PRO | A | 48 | -0.488 | -0.287 | 27.335 | 1.00 | 57.85 | A | C |
| ATOM | 73 | C | PRO | A | 48 | 0.518 | 2.895 | 26.457 | 1.00 | 56.57 | A | C |
| ATOM | 74 | O | PRO | A | 48 | 1.627 | 2.354 | 26.425 | 1.00 | 56.57 | A | O |
| ATOM | 75 | N | ASP | A | 49 | 0.340 | 4.209 | 26.356 | 1.00 | 51.99 | A | N |
| ATOM | 76 | CA | ASP | A | 49 | 1.454 | 5.141 | 26.210 | 1.00 | 51.99 | A | C |
| ATOM | 77 | CB | ASP | A | 49 | 0.917 | 6.575 | 26.071 | 1.00 | 70.33 | A | C |
| ATOM | 78 | CG | ASP | A | 49 | 2.004 | 7.573 | 25.689 | 1.00 | 70.33 | A | C |
| ATOM | 79 | OD1 | ASP | A | 49 | 3.160 | 7.357 | 26.120 | 1.00 | 70.33 | A | O |
| ATOM | 80 | OD2 | ASP | A | 49 | 1.713 | 8.569 | 24.975 | 1.00 | 70.33 | A | O |
| ATOM | 81 | C | ASP | A | 49 | 2.364 | 5.041 | 27.436 | 1.00 | 51.99 | A | C |
| ATOM | 82 | O | ASP | A | 49 | 2.348 | 5.922 | 28.298 | 1.00 | 51.99 | A | O |
| ATOM | 83 | N | ARG | A | 50 | 3.164 | 3.978 | 27.504 | 1.00 | 49.79 | A | N |
| ATOM | 84 | CA | ARG | A | 50 | 4.044 | 3.766 | 28.647 | 1.00 | 49.79 | A | C |
| ATOM | 85 | CB | ARG | A | 50 | 3.235 | 3.104 | 29.758 | 1.00 | 99.34 | A | C |
| ATOM | 86 | CG | ARG | A | 50 | 3.745 | 3.441 | 31.139 | 1.00 | 99.34 | A | C |
| ATOM | 87 | CD | ARG | A | 50 | 2.806 | 2.942 | 32.215 | 1.00 | 99.34 | A | C |
| ATOM | 88 | NE | ARG | A | 50 | 2.360 | 1.545 | 32.047 | 1.00 | 99.34 | A | N |
| ATOM | 89 | CZ | ARG | A | 50 | 3.083 | 0.535 | 31.546 | 1.00 | 99.34 | A | C |
| ATOM | 90 | NH1 | ARG | A | 50 | 4.330 | 0.733 | 31.121 | 1.00 | 99.34 | A | N |
| ATOM | 91 | NH2 | ARG | A | 50 | 2.562 | -0.695 | 31.501 | 1.00 | 99.34 | A | N |
| ATOM | 92 | C | ARG | A | 50 | 5.282 | 2.927 | 28.292 | 1.00 | 49.79 | A | C |
| ATOM | 93 | O | ARG | A | 50 | 5.240 | 1.695 | 28.308 | 1.00 | 49.79 | A | O |
| ATOM | 94 | N | PRO | A | 51 | 6.412 | 3.603 | 28.013 | 1.00 | 40.28 | A | N |
| ATOM | 95 | CD | PRO | A | 51 | 6.505 | 5.037 | 28.334 | 1.00 | 39.13 | A | C |
| ATOM | 96 | CA | PRO | A | 51 | 7.732 | 3.097 | 27.627 | 1.00 | 40.28 | A | C |
| ATOM | 97 | CB | PRO | A | 51 | 8.563 | 4.376 | 27.518 | 1.00 | 39.13 | A | C |
| ATOM | 98 | CG | PRO | A | 51 | 7.985 | 5.228 | 28.552 | 1.00 | 39.13 | A | C |
| ATOM | 99 | C | PRO | A | 51 | 8.448 | 2.011 | 28.427 | 1.00 | 40.28 | A | C |
| ATOM | 100 | O | PRO | A | 51 | 8.333 | 1.909 | 29.643 | 1.00 | 40.28 | A | O |
| ATOM | 101 | N | GLN | A | 52 | 9.207 | 1.208 | 27.695 | 1.00 | 50.11 | A | N |
| ATOM | 102 | CA | GLN | A | 52 | 9.997 | 0.116 | 28.238 | 1.00 | 50.11 | A | C |
| ATOM | 103 | CB | GLN | A | 52 | 9.237 | -1.195 | 28.137 | 1.00 | 66.13 | A | C |
| ATOM | 104 | CG | GLN | A | 52 | 8.518 | -1.380 | 26.827 | 1.00 | 66.13 | A | C |
| ATOM | 105 | CD | GLN | A | 52 | 7.529 | -2.527 | 26.882 | 1.00 | 66.13 | A | C |
| ATOM | 106 | OE1 | GLN | A | 52 | 7.846 | -3.654 | 26.482 | 1.00 | 66.13 | A | O |
| ATOM | 107 | NE2 | GLN | A | 52 | 6.322 | -2.252 | 27.400 | 1.00 | 66.13 | A | N |
| ATOM | 108 | C | GLN | A | 52 | 11.298 | 0.008 | 27.467 | 1.00 | 50.11 | A | C |
| ATOM | 109 | O | GLN | A | 52 | 11.438 | 0.561 | 26.381 | 1.00 | 50.11 | A | O |
| ATOM | 110 | N | GLU | A | 53 | 12.258 | -0.706 | 28.036 | 1.00 | 56.03 | A | N |
| ATOM | 111 | CA | GLU | A | 53 | 13.557 | -0.849 | 27.392 | 1.00 | 56.03 | A | C |
| ATOM | 112 | CB | GLU | A | 53 | 14.611 | -1.306 | 28.400 | 1.00 | 60.29 | A | C |
| ATOM | 113 | CG | GLU | A | 53 | 15.066 | -0.214 | 29.335 | 1.00 | 60.29 | A | C |
| ATOM | 114 | CD | GLU | A | 53 | 15.896 | -0.757 | 30.479 | 1.00 | 60.29 | A | C |
| ATOM | 115 | OE1 | GLU | A | 53 | 16.797 | -1.594 | 30.214 | 1.00 | 60.29 | A | O |
| ATOM | 116 | OE2 | GLU | A | 53 | 15.652 | -0.340 | 31.637 | 1.00 | 60.29 | A | O |
| ATOM | 117 | C | GLU | A | 53 | 13.518 | -1.799 | 26.221 | 1.00 | 56.03 | A | C |
| ATOM | 118 | O | GLU | A | 53 | 12.870 | -2.850 | 26.272 | 1.00 | 56.03 | A | O |
| ATOM | 119 | N | VAL | A | 54 | 14.223 | -1.404 | 25.165 | 1.00 | 47.60 | A | N |
| ATOM | 120 | CA | VAL | A | 54 | 14.299 | -2.173 | 23.934 | 1.00 | 47.60 | A | C |
| ATOM | 121 | CB | VAL | A | 54 | 13.402 | -1.551 | 22.838 | 1.00 | 45.63 | A | C |
| ATOM | 122 | CG1 | VAL | A | 54 | 13.542 | -2.341 | 21.545 | 1.00 | 45.63 | A | C |
| ATOM | 123 | CG2 | VAL | A | 54 | 11.951 | -1.515 | 23.300 | 1.00 | 45.63 | A | C |
| ATOM | 124 | C | VAL | A | 54 | 15.730 | -2.167 | 23.429 | 1.00 | 47.60 | A | C |
| ATOM | 125 | O | VAL | A | 54 | 16.339 | -1.105 | 23.291 | 1.00 | 47.60 | A | O |
| ATOM | 126 | N | SER | A | 55 | 16.270 | -3.351 | 23.170 | 1.00 | 48.87 | A | N |
| ATOM | 127 | CA | SER | A | 55 | 17.628 | -3.463 | 22.644 | 1.00 | 48.87 | A | C |

| | | | | | | | | | | | | |
|---|---|---|---|---|---|---|---|---|---|---|---|---|
| ATOM | 128 | CB | SER | A | 55 | 18.508 | -4.291 | 23.586 | 1.00 | 49.03 | A | C |
| ATOM | 129 | OG | SER | A | 55 | 18.790 | -3.566 | 24.773 | 1.00 | 49.03 | A | O |
| ATOM | 130 | C | SER | A | 55 | 17.634 | -4.073 | 21.231 | 1.00 | 48.87 | A | C |
| ATOM | 131 | O | SER | A | 55 | 16.894 | -5.020 | 20.924 | 1.00 | 48.87 | A | O |
| ATOM | 132 | N | TYR | A | 56 | 18.457 | -3.503 | 20.364 | 1.00 | 45.37 | A | N |
| ATOM | 133 | CA | TYR | A | 56 | 18.555 | -3.983 | 19.002 | 1.00 | 45.37 | A | C |
| ATOM | 134 | CB | TYR | A | 56 | 17.635 | -3.181 | 18.070 | 1.00 | 30.24 | A | C |
| ATOM | 135 | CG | TYR | A | 56 | 17.914 | -1.689 | 18.014 | 1.00 | 30.24 | A | C |
| ATOM | 136 | CD1 | TYR | A | 56 | 19.054 | -1.188 | 17.387 | 1.00 | 30.24 | A | C |
| ATOM | 137 | CE1 | TYR | A | 56 | 19.290 | 0.182 | 17.317 | 1.00 | 30.24 | A | C |
| ATOM | 138 | CD2 | TYR | A | 56 | 17.018 | -0.778 | 18.572 | 1.00 | 30.24 | A | C |
| ATOM | 139 | CE2 | TYR | A | 56 | 17.241 | 0.584 | 18.507 | 1.00 | 30.24 | A | C |
| ATOM | 140 | CZ | TYR | A | 56 | 18.374 | 1.060 | 17.879 | 1.00 | 30.24 | A | C |
| ATOM | 141 | OH | TYR | A | 56 | 18.574 | 2.420 | 17.804 | 1.00 | 30.24 | A | O |
| ATOM | 142 | C | TYR | A | 56 | 19.993 | -3.857 | 18.552 | 1.00 | 45.37 | A | C |
| ATOM | 143 | O | TYR | A | 56 | 20.772 | -3.087 | 19.122 | 1.00 | 45.37 | A | O |
| ATOM | 144 | N | THR | A | 57 | 20.342 | -4.614 | 17.520 | 1.00 | 71.48 | A | N |
| ATOM | 145 | CA | THR | A | 57 | 21.697 | -4.580 | 17.013 | 1.00 | 71.48 | A | C |
| ATOM | 146 | CB | THR | A | 57 | 22.544 | -5.636 | 17.736 | 1.00 | 59.52 | A | C |
| ATOM | 147 | OG1 | THR | A | 57 | 23.926 | -5.468 | 17.386 | 1.00 | 59.52 | A | O |
| ATOM | 148 | CG2 | THR | A | 57 | 22.058 | -7.039 | 17.362 | 1.00 | 59.52 | A | C |
| ATOM | 149 | C | THR | A | 57 | 21.738 | -4.827 | 15.500 | 1.00 | 71.48 | A | C |
| ATOM | 150 | O | THR | A | 57 | 20.697 | -4.864 | 14.833 | 1.00 | 71.48 | A | O |
| ATOM | 151 | N | ASP | A | 58 | 22.948 | -4.991 | 14.971 | 1.00 | 59.74 | A | N |
| ATOM | 152 | CA | ASP | A | 58 | 23.143 | -5.240 | 13.552 | 1.00 | 59.74 | A | C |
| ATOM | 153 | CB | ASP | A | 58 | 22.515 | -6.579 | 13.119 | 1.00 | 64.42 | A | C |
| ATOM | 154 | CG | ASP | A | 58 | 23.052 | -7.771 | 13.899 | 1.00 | 64.42 | A | C |
| ATOM | 155 | OD1 | ASP | A | 58 | 24.100 | -7.624 | 14.563 | 1.00 | 64.42 | A | O |
| ATOM | 156 | OD2 | ASP | A | 58 | 22.433 | -8.862 | 13.839 | 1.00 | 64.42 | A | O |
| ATOM | 157 | C | ASP | A | 58 | 22.466 | -4.136 | 12.785 | 1.00 | 59.74 | A | C |
| ATOM | 158 | O | ASP | A | 58 | 21.906 | -4.386 | 11.723 | 1.00 | 59.74 | A | O |
| ATOM | 159 | N | THR | A | 59 | 22.504 | -2.915 | 13.304 | 1.00 | 39.80 | A | N |
| ATOM | 160 | CA | THR | A | 59 | 21.839 | -1.829 | 12.591 | 1.00 | 39.80 | A | C |
| ATOM | 161 | CB | THR | A | 59 | 21.575 | -0.616 | 13.518 | 1.00 | 45.95 | A | C |
| ATOM | 162 | OG1 | THR | A | 59 | 22.740 | 0.209 | 13.581 | 1.00 | 45.95 | A | O |
| ATOM | 163 | CG2 | THR | A | 59 | 21.230 | -1.081 | 14.916 | 1.00 | 45.95 | A | C |
| ATOM | 164 | C | THR | A | 59 | 22.602 | -1.349 | 11.353 | 1.00 | 39.80 | A | C |
| ATOM | 165 | O | THR | A | 59 | 23.801 | -1.080 | 11.414 | 1.00 | 39.80 | A | O |
| ATOM | 166 | N | LYS | A | 60 | 21.899 | -1.256 | 10.225 | 1.00 | 81.88 | A | N |
| ATOM | 167 | CA | LYS | A | 60 | 22.506 | -0.785 | 8.974 | 1.00 | 81.88 | A | C |
| ATOM | 168 | CB | LYS | A | 60 | 22.985 | -1.961 | 8.111 | 1.00 | 55.16 | A | C |
| ATOM | 169 | CG | LYS | A | 60 | 21.894 | -2.902 | 7.652 | 1.00 | 55.16 | A | C |
| ATOM | 170 | CD | LYS | A | 60 | 22.423 | -3.810 | 6.549 | 1.00 | 55.16 | A | C |
| ATOM | 171 | CE | LYS | A | 60 | 21.421 | -4.914 | 6.195 | 1.00 | 55.16 | A | C |
| ATOM | 172 | NZ | LYS | A | 60 | 21.979 | -5.917 | 5.236 | 1.00 | 55.16 | A | N |
| ATOM | 173 | C | LYS | A | 60 | 21.533 | 0.079 | 8.165 | 1.00 | 81.88 | A | C |
| ATOM | 174 | O | LYS | A | 60 | 20.327 | -0.179 | 8.154 | 1.00 | 81.88 | A | O |
| ATOM | 175 | N | VAL | A | 61 | 22.068 | 1.098 | 7.490 | 1.00 | 55.81 | A | N |
| ATOM | 176 | CA | VAL | A | 61 | 21.264 | 2.025 | 6.696 | 1.00 | 55.81 | A | C |
| ATOM | 177 | CB | VAL | A | 61 | 22.100 | 3.236 | 6.233 | 1.00 | 31.54 | A | C |
| ATOM | 178 | CG1 | VAL | A | 61 | 21.248 | 4.141 | 5.364 | 1.00 | 31.54 | A | C |
| ATOM | 179 | CG2 | VAL | A | 61 | 22.612 | 4.008 | 7.434 | 1.00 | 31.54 | A | C |
| ATOM | 180 | C | VAL | A | 61 | 20.642 | 1.372 | 5.468 | 1.00 | 55.81 | A | C |
| ATOM | 181 | O | VAL | A | 61 | 21.311 | 0.628 | 4.750 | 1.00 | 55.81 | A | O |
| ATOM | 182 | N | ILE | A | 62 | 19.363 | 1.662 | 5.222 | 1.00 | 48.02 | A | N |
| ATOM | 183 | CA | ILE | A | 62 | 18.665 | 1.083 | 4.076 | 1.00 | 48.02 | A | C |
| ATOM | 184 | CB | ILE | A | 62 | 17.844 | -0.163 | 4.491 | 1.00 | 41.15 | A | C |
| ATOM | 185 | CG2 | ILE | A | 62 | 18.723 | -1.117 | 5.307 | 1.00 | 41.15 | A | C |
| ATOM | 186 | CG1 | ILE | A | 62 | 16.617 | 0.250 | 5.309 | 1.00 | 41.15 | A | C |
| ATOM | 187 | CD1 | ILE | A | 62 | 15.624 | -0.893 | 5.513 | 1.00 | 41.15 | A | C |
| ATOM | 188 | C | ILE | A | 62 | 17.729 | 2.037 | 3.345 | 1.00 | 48.02 | A | C |
| ATOM | 189 | O | ILE | A | 62 | 16.959 | 1.607 | 2.479 | 1.00 | 48.02 | A | O |
| ATOM | 190 | N | GLY | A | 63 | 17.787 | 3.323 | 3.686 | 1.00 | 46.28 | A | N |
| ATOM | 191 | CA | GLY | A | 63 | 16.912 | 4.274 | 3.022 | 1.00 | 46.28 | A | C |
| ATOM | 192 | C | GLY | A | 63 | 17.119 | 5.724 | 3.395 | 1.00 | 46.28 | A | C |
| ATOM | 193 | O | GLY | A | 63 | 17.358 | 6.025 | 4.553 | 1.00 | 46.28 | A | O |
| ATOM | 194 | N | ASN | A | 64 | 17.021 | 6.610 | 2.401 | 1.00 | 50.58 | A | N |

| | | | | | | | | | | | | |
|---|---|---|---|---|---|---|---|---|---|---|---|---|
| ATOM | 195 | CA | ASN | A | 64 | 17.170 | 8.055 | 2.574 | 1.00 | 50.58 | A | C |
| ATOM | 196 | CB | ASN | A | 64 | 17.945 | 8.660 | 1.408 | 1.00 | 82.07 | A | C |
| ATOM | 197 | CG | ASN | A | 64 | 19.432 | 8.430 | 1.515 | 1.00 | 82.07 | A | C |
| ATOM | 198 | OD1 | ASN | A | 64 | 20.155 | 9.189 | 2.176 | 1.00 | 82.07 | A | O |
| ATOM | 199 | ND2 | ASN | A | 64 | 19.906 | 7.366 | 0.874 | 1.00 | 82.07 | A | N |
| ATOM | 200 | C | ASN | A | 64 | 15.788 | 8.678 | 2.586 | 1.00 | 50.58 | A | C |
| ATOM | 201 | O | ASN | A | 64 | 14.821 | 8.046 | 2.987 | 1.00 | 50.58 | A | O |
| ATOM | 202 | N | GLY | A | 65 | 15.692 | 9.917 | 2.120 | 1.00 | 50.05 | A | N |
| ATOM | 203 | CA | GLY | A | 65 | 14.409 | 10.587 | 2.088 | 1.00 | 50.05 | A | C |
| ATOM | 204 | C | GLY | A | 65 | 14.553 | 11.968 | 2.675 | 1.00 | 50.05 | A | C |
| ATOM | 205 | O | GLY | A | 65 | 15.526 | 12.239 | 3.380 | 1.00 | 50.05 | A | O |
| ATOM | 206 | N | SER | A | 66 | 13.592 | 12.841 | 2.379 | 1.00 | 63.41 | A | N |
| ATOM | 207 | CA | SER | A | 66 | 13.610 | 14.212 | 2.887 | 1.00 | 63.41 | A | C |
| ATOM | 208 | CB | SER | A | 66 | 12.590 | 15.066 | 2.137 | 1.00 | 68.29 | A | C |
| ATOM | 209 | OG | SER | A | 66 | 11.293 | 14.504 | 2.262 | 1.00 | 68.29 | A | O |
| ATOM | 210 | C | SER | A | 66 | 13.278 | 14.221 | 4.378 | 1.00 | 63.41 | A | C |
| ATOM | 211 | O | SER | A | 66 | 13.473 | 15.232 | 5.067 | 1.00 | 63.41 | A | O |
| ATOM | 212 | N | PHE | A | 67 | 12.781 | 13.082 | 4.857 | 1.00 | 54.08 | A | N |
| ATOM | 213 | CA | PHE | A | 67 | 12.402 | 12.911 | 6.251 | 1.00 | 54.08 | A | C |
| ATOM | 214 | CB | PHE | A | 67 | 11.374 | 11.796 | 6.357 | 1.00 | 59.07 | A | C |
| ATOM | 215 | CG | PHE | A | 67 | 11.863 | 10.475 | 5.834 | 1.00 | 59.07 | A | C |
| ATOM | 216 | CD1 | PHE | A | 67 | 12.750 | 9.697 | 6.580 | 1.00 | 59.07 | A | C |
| ATOM | 217 | CD2 | PHE | A | 67 | 11.425 | 10.001 | 4.603 | 1.00 | 59.07 | A | C |
| ATOM | 218 | CE1 | PHE | A | 67 | 13.189 | 8.464 | 6.108 | 1.00 | 59.07 | A | C |
| ATOM | 219 | CE2 | PHE | A | 67 | 11.859 | 8.767 | 4.121 | 1.00 | 59.07 | A | C |
| ATOM | 220 | CZ | PHE | A | 67 | 12.741 | 7.995 | 4.876 | 1.00 | 59.07 | A | C |
| ATOM | 221 | C | PHE | A | 67 | 13.601 | 12.563 | 7.116 | 1.00 | 54.08 | A | C |
| ATOM | 222 | O | PHE | A | 67 | 13.657 | 12.935 | 8.289 | 1.00 | 54.08 | A | O |
| ATOM | 223 | N | GLY | A | 68 | 14.551 | 11.835 | 6.536 | 1.00 | 43.94 | A | N |
| ATOM | 224 | CA | GLY | A | 68 | 15.734 | 11.449 | 7.277 | 1.00 | 43.94 | A | C |
| ATOM | 225 | C | GLY | A | 68 | 16.303 | 10.170 | 6.720 | 1.00 | 43.94 | A | C |
| ATOM | 226 | O | GLY | A | 68 | 16.368 | 10.011 | 5.510 | 1.00 | 43.94 | A | O |
| ATOM | 227 | N | VAL | A | 69 | 16.699 | 9.259 | 7.606 | 1.00 | 44.41 | A | N |
| ATOM | 228 | CA | VAL | A | 69 | 17.274 | 7.979 | 7.221 | 1.00 | 44.41 | A | C |
| ATOM | 229 | CB | VAL | A | 69 | 18.686 | 7.822 | 7.795 | 1.00 | 28.34 | A | C |
| ATOM | 230 | CG1 | VAL | A | 69 | 19.317 | 6.538 | 7.305 | 1.00 | 28.34 | A | C |
| ATOM | 231 | CG2 | VAL | A | 69 | 19.527 | 9.008 | 7.405 | 1.00 | 28.34 | A | C |
| ATOM | 232 | C | VAL | A | 69 | 16.428 | 6.836 | 7.751 | 1.00 | 44.41 | A | C |
| ATOM | 233 | O | VAL | A | 69 | 15.555 | 7.040 | 8.589 | 1.00 | 44.41 | A | O |
| ATOM | 234 | N | VAL | A | 70 | 16.692 | 5.633 | 7.257 | 1.00 | 47.70 | A | N |
| ATOM | 235 | CA | VAL | A | 70 | 15.979 | 4.451 | 7.699 | 1.00 | 47.70 | A | C |
| ATOM | 236 | CB | VAL | A | 70 | 14.928 | 3.994 | 6.684 | 1.00 | 47.40 | A | C |
| ATOM | 237 | CG1 | VAL | A | 70 | 14.027 | 2.935 | 7.311 | 1.00 | 47.40 | A | C |
| ATOM | 238 | CG2 | VAL | A | 70 | 14.121 | 5.164 | 6.216 | 1.00 | 47.40 | A | C |
| ATOM | 239 | C | VAL | A | 70 | 16.992 | 3.326 | 7.840 | 1.00 | 47.70 | A | C |
| ATOM | 240 | O | VAL | A | 70 | 17.624 | 2.920 | 6.864 | 1.00 | 47.70 | A | O |
| ATOM | 241 | N | TYR | A | 71 | 17.151 | 2.821 | 9.055 | 1.00 | 43.93 | A | N |
| ATOM | 242 | CA | TYR | A | 71 | 18.078 | 1.734 | 9.279 | 1.00 | 43.93 | A | C |
| ATOM | 243 | CB | TYR | A | 71 | 18.864 | 1.937 | 10.581 | 1.00 | 45.54 | A | C |
| ATOM | 244 | CG | TYR | A | 71 | 19.453 | 3.311 | 10.790 | 1.00 | 45.54 | A | C |
| ATOM | 245 | CD1 | TYR | A | 71 | 18.640 | 4.400 | 11.095 | 1.00 | 45.54 | A | C |
| ATOM | 246 | CE1 | TYR | A | 71 | 19.183 | 5.666 | 11.314 | 1.00 | 45.54 | A | C |
| ATOM | 247 | CD2 | TYR | A | 71 | 20.828 | 3.521 | 10.706 | 1.00 | 45.54 | A | C |
| ATOM | 248 | CE2 | TYR | A | 71 | 21.383 | 4.784 | 10.920 | 1.00 | 45.54 | A | C |
| ATOM | 249 | CZ | TYR | A | 71 | 20.554 | 5.850 | 11.225 | 1.00 | 45.54 | A | C |
| ATOM | 250 | OH | TYR | A | 71 | 21.094 | 7.099 | 11.444 | 1.00 | 45.54 | A | O |
| ATOM | 251 | C | TYR | A | 71 | 17.275 | 0.450 | 9.412 | 1.00 | 43.93 | A | C |
| ATOM | 252 | O | TYR | A | 71 | 16.038 | 0.465 | 9.458 | 1.00 | 43.93 | A | O |
| ATOM | 253 | N | GLN | A | 72 | 17.991 | -0.668 | 9.457 | 1.00 | 42.24 | A | N |
| ATOM | 254 | CA | GLN | A | 72 | 17.369 | -1.961 | 9.667 | 1.00 | 42.24 | A | C |
| ATOM | 255 | CB | GLN | A | 72 | 17.721 | -2.944 | 8.572 | 1.00 | 58.43 | A | C |
| ATOM | 256 | CG | GLN | A | 72 | 17.162 | -4.302 | 8.862 | 1.00 | 58.43 | A | C |
| ATOM | 257 | CD | GLN | A | 72 | 17.820 | -5.379 | 8.052 | 1.00 | 58.43 | A | C |
| ATOM | 258 | OE1 | GLN | A | 72 | 19.021 | -5.658 | 8.213 | 1.00 | 58.43 | A | O |
| ATOM | 259 | NE2 | GLN | A | 72 | 17.043 | -6.000 | 7.168 | 1.00 | 58.43 | A | N |
| ATOM | 260 | C | GLN | A | 72 | 18.043 | -2.402 | 10.949 | 1.00 | 42.24 | A | C |
| ATOM | 261 | O | GLN | A | 72 | 19.130 | -1.908 | 11.273 | 1.00 | 42.24 | A | O |

| | | | | | | | | | | | | |
|---|---|---|---|---|---|---|---|---|---|---|---|---|
| ATOM | 262 | N | ALA | A | 73 | 17.420 | -3.312 | 11.686 | 1.00 | 36.61 | A | N |
| ATOM | 263 | CA | ALA | A | 73 | 18.020 | -3.769 | 12.928 | 1.00 | 36.61 | A | C |
| ATOM | 264 | CB | ALA | A | 73 | 17.929 | -2.683 | 13.992 | 1.00 | 30.91 | A | C |
| ATOM | 265 | C | ALA | A | 73 | 17.376 | -5.043 | 13.425 | 1.00 | 36.61 | A | C |
| ATOM | 266 | O | ALA | A | 73 | 16.339 | -5.486 | 12.920 | 1.00 | 36.61 | A | O |
| ATOM | 267 | N | LYS | A | 74 | 18.002 | -5.624 | 14.438 | 1.00 | 50.49 | A | N |
| ATOM | 268 | CA | LYS | A | 74 | 17.537 | -6.873 | 15.007 | 1.00 | 50.49 | A | C |
| ATOM | 269 | CB | LYS | A | 74 | 18.591 | -7.947 | 14.736 | 1.00 | 55.00 | A | C |
| ATOM | 270 | CG | LYS | A | 74 | 18.233 | -9.346 | 15.173 | 1.00 | 55.00 | A | C |
| ATOM | 271 | CD | LYS | A | 74 | 19.425 | -10.252 | 14.909 | 1.00 | 55.00 | A | C |
| ATOM | 272 | CE | LYS | A | 74 | 19.309 | -11.600 | 15.600 | 1.00 | 55.00 | A | C |
| ATOM | 273 | NZ | LYS | A | 74 | 20.526 | -12.425 | 15.289 | 1.00 | 55.00 | A | N |
| ATOM | 274 | C | LYS | A | 74 | 17.283 | -6.721 | 16.504 | 1.00 | 50.49 | A | C |
| ATOM | 275 | O | LYS | A | 74 | 18.165 | -6.307 | 17.257 | 1.00 | 50.49 | A | O |
| ATOM | 276 | N | LEU | A | 75 | 16.060 | -7.044 | 16.919 | 1.00 | 49.83 | A | N |
| ATOM | 277 | CA | LEU | A | 75 | 15.674 | -6.960 | 18.316 | 1.00 | 49.83 | A | C |
| ATOM | 278 | CB | LEU | A | 75 | 14.156 | -7.114 | 18.456 | 1.00 | 22.75 | A | C |
| ATOM | 279 | CG | LEU | A | 75 | 13.229 | -6.103 | 17.771 | 1.00 | 22.75 | A | C |
| ATOM | 280 | CD1 | LEU | A | 75 | 11.832 | -6.260 | 18.312 | 1.00 | 22.75 | A | C |
| ATOM | 281 | CD2 | LEU | A | 75 | 13.687 | -4.701 | 18.035 | 1.00 | 22.75 | A | C |
| ATOM | 282 | C | LEU | A | 75 | 16.389 | -8.068 | 19.088 | 1.00 | 49.83 | A | C |
| ATOM | 283 | O | LEU | A | 75 | 16.350 | -9.242 | 18.694 | 1.00 | 49.83 | A | O |
| ATOM | 284 | N | CYS | A | 76 | 17.035 | -7.699 | 20.195 | 1.00 | 57.70 | A | N |
| ATOM | 285 | CA | CYS | A | 76 | 17.765 | -8.672 | 20.996 | 1.00 | 57.70 | A | C |
| ATOM | 286 | CB | CYS | A | 76 | 18.679 | -7.966 | 21.991 | 1.00 | 50.69 | A | C |
| ATOM | 287 | SG | CYS | A | 76 | 20.358 | -7.727 | 21.361 | 1.00 | 50.69 | A | S |
| ATOM | 288 | C | CYS | A | 76 | 16.883 | -9.658 | 21.731 | 1.00 | 57.70 | A | C |
| ATOM | 289 | O | CYS | A | 76 | 17.391 | -10.512 | 22.437 | 1.00 | 57.70 | A | O |
| ATOM | 290 | N | ASP | A | 77 | 15.571 | -9.570 | 21.559 | 1.00 | 51.44 | A | N |
| ATOM | 291 | CA | ASP | A | 77 | 14.674 | -10.489 | 22.253 | 1.00 | 51.44 | A | C |
| ATOM | 292 | CB | ASP | A | 77 | 13.394 | -9.777 | 22.683 | 1.00100.00 | | A | C |
| ATOM | 293 | CG | ASP | A | 77 | 13.654 | -8.403 | 23.266 | 1.00100.00 | | A | C |
| ATOM | 294 | OD1 | ASP | A | 77 | 13.992 | -7.459 | 22.481 | 1.00100.00 | | A | O |
| ATOM | 295 | OD2 | ASP | A | 77 | 13.523 | -8.281 | 24.516 | 1.00100.00 | | A | O |
| ATOM | 296 | C | ASP | A | 77 | 14.278 | -11.652 | 21.368 | 1.00 | 51.44 | A | C |
| ATOM | 297 | O | ASP | A | 77 | 14.570 | -12.817 | 21.654 | 1.00 | 51.44 | A | O |
| ATOM | 298 | N | SER | A | 78 | 13.589 | -11.315 | 20.288 | 1.00 | 64.80 | A | N |
| ATOM | 299 | CA | SER | A | 78 | 13.108 | -12.308 | 19.341 | 1.00 | 64.80 | A | C |
| ATOM | 300 | CB | SER | A | 78 | 11.684 | -11.954 | 18.921 | 1.00 | 73.13 | A | C |
| ATOM | 301 | OG | SER | A | 78 | 11.616 | -10.579 | 18.559 | 1.00 | 73.13 | A | O |
| ATOM | 302 | C | SER | A | 78 | 13.995 | -12.369 | 18.110 | 1.00 | 64.80 | A | C |
| ATOM | 303 | O | SER | A | 78 | 13.754 | -13.169 | 17.205 | 1.00 | 64.80 | A | O |
| ATOM | 304 | N | GLY | A | 79 | 15.023 | -11.529 | 18.072 | 1.00 | 48.97 | A | N |
| ATOM | 305 | CA | GLY | A | 79 | 15.885 | -11.527 | 16.907 | 1.00 | 48.97 | A | C |
| ATOM | 306 | C | GLY | A | 79 | 15.093 | -10.944 | 15.748 | 1.00 | 48.97 | A | C |
| ATOM | 307 | O | GLY | A | 79 | 15.653 | -10.620 | 14.701 | 1.00 | 48.97 | A | O |
| ATOM | 308 | N | GLU | A | 80 | 13.783 | -10.801 | 15.948 | 1.00 | 48.54 | A | N |
| ATOM | 309 | CA | GLU | A | 80 | 12.891 | -10.250 | 14.931 | 1.00 | 48.54 | A | C |
| ATOM | 310 | CB | GLU | A | 80 | 11.541 | -9.875 | 15.547 | 1.00 | 66.68 | A | C |
| ATOM | 311 | CG | GLU | A | 80 | 10.555 | -11.023 | 15.670 | 1.00 | 66.68 | A | C |
| ATOM | 312 | CD | GLU | A | 80 | 9.247 | -10.596 | 16.336 | 1.00 | 66.68 | A | C |
| ATOM | 313 | OE1 | GLU | A | 80 | 9.205 | -10.533 | 17.593 | 1.00 | 66.68 | A | O |
| ATOM | 314 | OE2 | GLU | A | 80 | 8.264 | -10.307 | 15.600 | 1.00 | 66.68 | A | O |
| ATOM | 315 | C | GLU | A | 80 | 13.492 | -9.024 | 14.268 | 1.00 | 48.54 | A | C |
| ATOM | 316 | O | GLU | A | 80 | 14.014 | -8.133 | 14.939 | 1.00 | 48.54 | A | O |
| ATOM | 317 | N | LEU | A | 81 | 13.413 | -8.980 | 12.946 | 1.00 | 39.32 | A | N |
| ATOM | 318 | CA | LEU | A | 81 | 13.960 | -7.860 | 12.213 | 1.00 | 39.32 | A | C |
| ATOM | 319 | CB | LEU | A | 81 | 14.284 | -8.284 | 10.782 | 1.00 | 68.38 | A | C |
| ATOM | 320 | CG | LEU | A | 81 | 15.458 | -9.254 | 10.643 | 1.00 | 68.38 | A | C |
| ATOM | 321 | CD1 | LEU | A | 81 | 15.701 | -9.562 | 9.181 | 1.00 | 68.38 | A | C |
| ATOM | 322 | CD2 | LEU | A | 81 | 16.707 | -8.628 | 11.266 | 1.00 | 68.38 | A | C |
| ATOM | 323 | C | LEU | A | 81 | 13.002 | -6.676 | 12.208 | 1.00 | 39.32 | A | C |
| ATOM | 324 | O | LEU | A | 81 | 11.778 | -6.844 | 12.276 | 1.00 | 39.32 | A | O |
| ATOM | 325 | N | VAL | A | 82 | 13.570 | -5.475 | 12.126 | 1.00 | 46.15 | A | N |
| ATOM | 326 | CA | VAL | A | 82 | 12.779 | -4.251 | 12.121 | 1.00 | 46.15 | A | C |
| ATOM | 327 | CB | VAL | A | 82 | 12.606 | -3.665 | 13.563 | 1.00 | 37.97 | A | C |
| ATOM | 328 | CG1 | VAL | A | 82 | 11.904 | -4.659 | 14.450 | 1.00 | 37.97 | A | C |

| | | | | | | | | | | | | |
|---|---|---|---|---|---|---|---|---|---|---|---|---|
| ATOM | 329 | CG2 | VAL | A | 82 | 13.962 | -3.289 | 14.151 | 1.00 | 37.97 | A | C |
| ATOM | 330 | C | VAL | A | 82 | 13.421 | -3.163 | 11.273 | 1.00 | 46.15 | A | C |
| ATOM | 331 | O | VAL | A | 82 | 14.566 | -3.279 | 10.839 | 1.00 | 46.15 | A | O |
| ATOM | 332 | N | ALA | A | 83 | 12.668 | -2.095 | 11.052 | 1.00 | 59.23 | A | N |
| ATOM | 333 | CA | ALA | A | 83 | 13.163 | -0.953 | 10.297 | 1.00 | 59.23 | A | C |
| ATOM | 334 | CB | ALA | A | 83 | 12.385 | -0.797 | 8.987 | 1.00 | 60.41 | A | C |
| ATOM | 335 | C | ALA | A | 83 | 12.938 | 0.261 | 11.196 | 1.00 | 59.23 | A | C |
| ATOM | 336 | O | ALA | A | 83 | 11.849 | 0.437 | 11.754 | 1.00 | 59.23 | A | O |
| ATOM | 337 | N | ILE | A | 84 | 13.962 | 1.086 | 11.359 | 1.00 | 37.09 | A | N |
| ATOM | 338 | CA | ILE | A | 84 | 13.806 | 2.259 | 12.192 | 1.00 | 37.09 | A | C |
| ATOM | 339 | CB | ILE | A | 84 | 14.890 | 2.293 | 13.301 | 1.00 | 26.02 | A | C |
| ATOM | 340 | CG2 | ILE | A | 84 | 14.624 | 3.440 | 14.262 | 1.00 | 26.02 | A | C |
| ATOM | 341 | CG1 | ILE | A | 84 | 14.861 | 0.970 | 14.074 | 1.00 | 26.02 | A | C |
| ATOM | 342 | CD1 | ILE | A | 84 | 15.907 | 0.844 | 15.146 | 1.00 | 26.02 | A | C |
| ATOM | 343 | C | ILE | A | 84 | 13.850 | 3.520 | 11.334 | 1.00 | 37.09 | A | C |
| ATOM | 344 | O | ILE | A | 84 | 14.874 | 3.862 | 10.746 | 1.00 | 37.09 | A | O |
| ATOM | 345 | N | LYS | A | 85 | 12.715 | 4.198 | 11.246 | 1.00 | 43.25 | A | N |
| ATOM | 346 | CA | LYS | A | 85 | 12.631 | 5.414 | 10.460 | 1.00 | 43.25 | A | C |
| ATOM | 347 | CB | LYS | A | 85 | 11.213 | 5.569 | 9.901 | 1.00 | 41.87 | A | C |
| ATOM | 348 | CG | LYS | A | 85 | 11.057 | 6.643 | 8.840 | 1.00 | 41.87 | A | C |
| ATOM | 349 | CD | LYS | A | 85 | 9.611 | 6.742 | 8.355 | 1.00 | 41.87 | A | C |
| ATOM | 350 | CE | LYS | A | 85 | 9.402 | 8.005 | 7.516 | 1.00 | 41.87 | A | C |
| ATOM | 351 | NZ | LYS | A | 85 | 8.047 | 8.085 | 6.913 | 1.00 | 41.87 | A | N |
| ATOM | 352 | C | LYS | A | 85 | 13.006 | 6.599 | 11.347 | 1.00 | 43.25 | A | C |
| ATOM | 353 | O | LYS | A | 85 | 12.244 | 6.998 | 12.229 | 1.00 | 43.25 | A | O |
| ATOM | 354 | N | LYS | A | 86 | 14.197 | 7.144 | 11.127 | 1.00 | 48.44 | A | N |
| ATOM | 355 | CA | LYS | A | 86 | 14.664 | 8.276 | 11.917 | 1.00 | 48.44 | A | C |
| ATOM | 356 | CB | LYS | A | 86 | 16.198 | 8.257 | 12.040 | 1.00 | 58.22 | A | C |
| ATOM | 357 | CG | LYS | A | 86 | 16.708 | 9.041 | 13.245 | 1.00 | 58.22 | A | C |
| ATOM | 358 | CD | LYS | A | 86 | 18.102 | 9.640 | 13.041 | 1.00 | 58.22 | A | C |
| ATOM | 359 | CE | LYS | A | 86 | 19.236 | 8.633 | 13.220 | 1.00 | 58.22 | A | C |
| ATOM | 360 | NZ | LYS | A | 86 | 19.352 | 8.108 | 14.621 | 1.00 | 58.22 | A | N |
| ATOM | 361 | C | LYS | A | 86 | 14.210 | 9.555 | 11.230 | 1.00 | 48.44 | A | C |
| ATOM | 362 | O | LYS | A | 86 | 14.478 | 9.757 | 10.053 | 1.00 | 48.44 | A | O |
| ATOM | 363 | N | VAL | A | 87 | 13.526 | 10.419 | 11.968 | 1.00 | 38.31 | A | N |
| ATOM | 364 | CA | VAL | A | 87 | 13.018 | 11.663 | 11.411 | 1.00 | 38.31 | A | C |
| ATOM | 365 | CB | VAL | A | 87 | 11.486 | 11.611 | 11.244 | 1.00 | 39.20 | A | C |
| ATOM | 366 | CG1 | VAL | A | 87 | 10.965 | 12.976 | 10.871 | 1.00 | 39.20 | A | C |
| ATOM | 367 | CG2 | VAL | A | 87 | 11.105 | 10.587 | 10.188 | 1.00 | 39.20 | A | C |
| ATOM | 368 | C | VAL | A | 87 | 13.323 | 12.853 | 12.291 | 1.00 | 38.31 | A | C |
| ATOM | 369 | O | VAL | A | 87 | 13.133 | 12.790 | 13.506 | 1.00 | 38.31 | A | O |
| ATOM | 370 | N | LEU | A | 88 | 13.783 | 13.943 | 11.682 | 1.00 | 51.97 | A | N |
| ATOM | 371 | CA | LEU | A | 88 | 14.064 | 15.167 | 12.433 | 1.00 | 51.97 | A | C |
| ATOM | 372 | CB | LEU | A | 88 | 14.820 | 16.176 | 11.556 | 1.00 | 62.22 | A | C |
| ATOM | 373 | CG | LEU | A | 88 | 15.413 | 17.502 | 12.089 | 1.00 | 62.22 | A | C |
| ATOM | 374 | CD1 | LEU | A | 88 | 14.348 | 18.335 | 12.799 | 1.00 | 62.22 | A | C |
| ATOM | 375 | CD2 | LEU | A | 88 | 16.584 | 17.192 | 13.027 | 1.00 | 62.22 | A | C |
| ATOM | 376 | C | LEU | A | 88 | 12.684 | 15.726 | 12.786 | 1.00 | 51.97 | A | C |
| ATOM | 377 | O | LEU | A | 88 | 11.963 | 16.197 | 11.905 | 1.00 | 51.97 | A | O |
| ATOM | 378 | N | GLN | A | 89 | 12.297 | 15.644 | 14.057 | 1.00 | 59.91 | A | N |
| ATOM | 379 | CA | GLN | A | 89 | 10.998 | 16.163 | 14.460 | 1.00 | 59.91 | A | C |
| ATOM | 380 | CB | GLN | A | 89 | 10.192 | 15.124 | 15.235 | 1.00 | 59.62 | A | C |
| ATOM | 381 | CG | GLN | A | 89 | 8.803 | 15.623 | 15.617 | 1.00 | 59.62 | A | C |
| ATOM | 382 | CD | GLN | A | 89 | 8.006 | 16.156 | 14.418 | 1.00 | 59.62 | A | C |
| ATOM | 383 | OE1 | GLN | A | 89 | 8.500 | 16.168 | 13.280 | 1.00 | 59.62 | A | O |
| ATOM | 384 | NE2 | GLN | A | 89 | 6.765 | 16.599 | 14.673 | 1.00 | 59.62 | A | N |
| ATOM | 385 | C | GLN | A | 89 | 11.145 | 17.427 | 15.293 | 1.00 | 59.91 | A | C |
| ATOM | 386 | O | GLN | A | 89 | 11.997 | 17.517 | 16.181 | 1.00 | 59.91 | A | O |
| ATOM | 387 | N | ASP | A | 90 | 10.289 | 18.397 | 14.996 | 1.00 | 68.30 | A | N |
| ATOM | 388 | CA | ASP | A | 90 | 10.323 | 19.681 | 15.656 | 1.00 | 68.30 | A | C |
| ATOM | 389 | CB | ASP | A | 90 | 9.867 | 20.756 | 14.681 | 1.00 | 96.51 | A | C |
| ATOM | 390 | CG | ASP | A | 90 | 9.698 | 22.112 | 15.334 | 1.00 | 96.51 | A | C |
| ATOM | 391 | OD1 | ASP | A | 90 | 9.552 | 23.092 | 14.559 | 1.00 | 96.51 | A | O |
| ATOM | 392 | OD2 | ASP | A | 90 | 9.699 | 22.202 | 16.593 | 1.00 | 96.51 | A | O |
| ATOM | 393 | C | ASP | A | 90 | 9.530 | 19.787 | 16.930 | 1.00 | 68.30 | A | C |
| ATOM | 394 | O | ASP | A | 90 | 8.321 | 20.057 | 16.911 | 1.00 | 68.30 | A | O |
| ATOM | 395 | N | ALA | A | 91 | 10.255 | 19.637 | 18.036 | 1.00100.00 | | A | N |

| | | | | | | | | | | | | |
|---|---|---|---|---|---|---|---|---|---|---|---|---|
| ATOM | 396 | CA | ALA | A | 91 | 9.666 | 19.757 | 19.357 | 1.00 | 100.00 | A | C |
| ATOM | 397 | CB | ALA | A | 91 | 10.752 | 19.663 | 20.455 | 1.00 | 47.73 | A | C |
| ATOM | 398 | C | ALA | A | 91 | 8.970 | 21.123 | 19.394 | 1.00 | 100.00 | A | C |
| ATOM | 399 | O | ALA | A | 91 | 9.600 | 22.188 | 19.375 | 1.00 | 100.00 | A | O |
| ATOM | 400 | N | ALA | A | 92 | 7.651 | 21.040 | 19.377 | 1.00 | 89.18 | A | N |
| ATOM | 401 | CA | ALA | A | 92 | 6.701 | 22.148 | 19.439 | 1.00 | 89.18 | A | C |
| ATOM | 402 | CB | ALA | A | 92 | 7.219 | 23.404 | 18.712 | 1.00 | 79.74 | A | C |
| ATOM | 403 | C | ALA | A | 92 | 5.421 | 21.623 | 18.773 | 1.00 | 89.18 | A | C |
| ATOM | 404 | O | ALA | A | 92 | 4.303 | 21.840 | 19.277 | 1.00 | 89.18 | A | O |
| ATOM | 405 | N | ALA | A | 93 | 5.600 | 20.921 | 17.649 | 1.00 | 79.38 | A | N |
| ATOM | 406 | CA | ALA | A | 93 | 4.487 | 20.338 | 16.903 | 1.00 | 79.38 | A | C |
| ATOM | 407 | CB | ALA | A | 93 | 4.510 | 20.839 | 15.462 | 1.00 | 39.68 | A | C |
| ATOM | 408 | C | ALA | A | 93 | 4.525 | 18.795 | 16.935 | 1.00 | 79.38 | A | C |
| ATOM | 409 | O | ALA | A | 93 | 5.596 | 18.172 | 16.864 | 1.00 | 79.38 | A | O |
| ATOM | 410 | N | ALA | A | 94 | 3.343 | 18.190 | 17.060 | 1.00 | 63.78 | A | N |
| ATOM | 411 | CA | ALA | A | 94 | 3.220 | 16.739 | 17.081 | 1.00 | 63.78 | A | C |
| ATOM | 412 | CB | ALA | A | 94 | 1.816 | 16.337 | 17.546 | 1.00 | 33.55 | A | C |
| ATOM | 413 | C | ALA | A | 94 | 3.480 | 16.229 | 15.660 | 1.00 | 63.78 | A | C |
| ATOM | 414 | O | ALA | A | 94 | 3.178 | 16.918 | 14.670 | 1.00 | 63.78 | A | O |
| ATOM | 415 | N | ASN | A | 95 | 4.049 | 15.028 | 15.568 | 1.00 | 38.53 | A | N |
| ATOM | 416 | CA | ASN | A | 95 | 4.366 | 14.389 | 14.287 | 1.00 | 38.53 | A | C |
| ATOM | 417 | CB | ASN | A | 95 | 5.459 | 13.347 | 14.518 | 1.00 | 43.12 | A | C |
| ATOM | 418 | CG | ASN | A | 95 | 6.004 | 12.777 | 13.238 | 1.00 | 43.12 | A | C |
| ATOM | 419 | OD1 | ASN | A | 95 | 5.428 | 11.859 | 12.664 | 1.00 | 43.12 | A | O |
| ATOM | 420 | ND2 | ASN | A | 95 | 7.122 | 13.324 | 12.774 | 1.00 | 43.12 | A | N |
| ATOM | 421 | C | ASN | A | 95 | 3.121 | 13.744 | 13.657 | 1.00 | 38.53 | A | C |
| ATOM | 422 | O | ASN | A | 95 | 2.494 | 12.860 | 14.242 | 1.00 | 38.53 | A | O |
| ATOM | 423 | N | ARG | A | 96 | 2.771 | 14.189 | 12.455 | 1.00 | 47.01 | A | N |
| ATOM | 424 | CA | ARG | A | 96 | 1.587 | 13.674 | 11.776 | 1.00 | 47.01 | A | C |
| ATOM | 425 | CB | ARG | A | 96 | 1.330 | 14.468 | 10.493 | 1.00 | 62.26 | A | C |
| ATOM | 426 | CG | ARG | A | 96 | -0.052 | 14.237 | 9.903 | 1.00 | 62.26 | A | C |
| ATOM | 427 | CD | ARG | A | 96 | -0.215 | 14.934 | 8.552 | 1.00 | 62.26 | A | C |
| ATOM | 428 | NE | ARG | A | 96 | -0.631 | 16.335 | 8.641 | 1.00 | 62.26 | A | N |
| ATOM | 429 | CZ | ARG | A | 96 | -0.691 | 17.152 | 7.588 | 1.00 | 62.26 | A | C |
| ATOM | 430 | NH1 | ARG | A | 96 | -0.358 | 16.712 | 6.375 | 1.00 | 62.26 | A | N |
| ATOM | 431 | NH2 | ARG | A | 96 | -1.085 | 18.406 | 7.731 | 1.00 | 62.26 | A | N |
| ATOM | 432 | C | ARG | A | 96 | 1.644 | 12.176 | 11.466 | 1.00 | 47.01 | A | C |
| ATOM | 433 | O | ARG | A | 96 | 0.660 | 11.468 | 11.665 | 1.00 | 47.01 | A | O |
| ATOM | 434 | N | GLU | A | 97 | 2.787 | 11.687 | 10.992 | 1.00 | 40.91 | A | N |
| ATOM | 435 | CA | GLU | A | 97 | 2.917 | 10.268 | 10.676 | 1.00 | 40.91 | A | C |
| ATOM | 436 | CB | GLU | A | 97 | 4.296 | 9.972 | 10.085 | 1.00 | 30.74 | A | C |
| ATOM | 437 | CG | GLU | A | 97 | 4.437 | 8.551 | 9.555 | 1.00 | 30.74 | A | C |
| ATOM | 438 | CD | GLU | A | 97 | 5.731 | 8.334 | 8.801 | 1.00 | 30.74 | A | C |
| ATOM | 439 | OE1 | GLU | A | 97 | 6.404 | 9.337 | 8.495 | 1.00 | 30.74 | A | O |
| ATOM | 440 | OE2 | GLU | A | 97 | 6.067 | 7.171 | 8.500 | 1.00 | 30.74 | A | O |
| ATOM | 441 | C | GLU | A | 97 | 2.697 | 9.411 | 11.923 | 1.00 | 40.91 | A | C |
| ATOM | 442 | O | GLU | A | 97 | 1.977 | 8.407 | 11.885 | 1.00 | 40.91 | A | O |
| ATOM | 443 | N | LEU | A | 98 | 3.311 | 9.814 | 13.032 | 1.00 | 42.28 | A | N |
| ATOM | 444 | CA | LEU | A | 98 | 3.180 | 9.074 | 14.277 | 1.00 | 42.28 | A | C |
| ATOM | 445 | CB | LEU | A | 98 | 4.052 | 9.701 | 15.358 | 1.00 | 33.53 | A | C |
| ATOM | 446 | CG | LEU | A | 98 | 3.922 | 9.067 | 16.743 | 1.00 | 33.53 | A | C |
| ATOM | 447 | CD1 | LEU | A | 98 | 3.995 | 7.544 | 16.622 | 1.00 | 33.53 | A | C |
| ATOM | 448 | CD2 | LEU | A | 98 | 5.025 | 9.609 | 17.650 | 1.00 | 33.53 | A | C |
| ATOM | 449 | C | LEU | A | 98 | 1.742 | 8.990 | 14.768 | 1.00 | 42.28 | A | C |
| ATOM | 450 | O | LEU | A | 98 | 1.297 | 7.935 | 15.220 | 1.00 | 42.28 | A | O |
| ATOM | 451 | N | GLN | A | 99 | 1.010 | 10.094 | 14.680 | 1.00 | 42.69 | A | N |
| ATOM | 452 | CA | GLN | A | 99 | -0.377 | 10.102 | 15.127 | 1.00 | 42.69 | A | C |
| ATOM | 453 | CB | GLN | A | 99 | -0.953 | 11.517 | 15.075 | 1.00 | 100.00 | A | C |
| ATOM | 454 | CG | GLN | A | 99 | -0.538 | 12.407 | 16.243 | 1.00 | 100.00 | A | C |
| ATOM | 455 | CD | GLN | A | 99 | -1.117 | 13.827 | 16.128 | 1.00 | 100.00 | A | C |
| ATOM | 456 | OE1 | GLN | A | 99 | -0.928 | 14.672 | 17.033 | 1.00 | 100.00 | A | O |
| ATOM | 457 | NE2 | GLN | A | 99 | -1.822 | 14.101 | 15.012 | 1.00 | 100.00 | A | N |
| ATOM | 458 | C | GLN | A | 99 | -1.246 | 9.165 | 14.304 | 1.00 | 42.69 | A | C |
| ATOM | 459 | O | GLN | A | 99 | -2.084 | 8.455 | 14.857 | 1.00 | 42.69 | A | O |
| ATOM | 460 | N | ILE | A | 100 | -1.052 | 9.159 | 12.987 | 1.00 | 38.43 | A | N |
| ATOM | 461 | CA | ILE | A | 100 | -1.832 | 8.282 | 12.124 | 1.00 | 38.43 | A | C |
| ATOM | 462 | CB | ILE | A | 100 | -1.593 | 8.599 | 10.631 | 1.00 | 38.07 | A | C |

| | | | | | | | | | | | | |
|---|---|---|---|---|---|---|---|---|---|---|---|---|
| ATOM | 463 | CG2 | ILE | A | 100 | -2.265 | 7.546 | 9.760 | 1.00 | 38.07 | A | C |
| ATOM | 464 | CG1 | ILE | A | 100 | -2.167 | 9.979 | 10.301 | 1.00 | 38.07 | A | C |
| ATOM | 465 | CD1 | ILE | A | 100 | -1.916 | 10.445 | 8.877 | 1.00 | 38.07 | A | C |
| ATOM | 466 | C | ILE | A | 100 | -1.430 | 6.839 | 12.396 | 1.00 | 38.43 | A | C |
| ATOM | 467 | O | ILE | A | 100 | -2.249 | 6.000 | 12.781 | 1.00 | 38.43 | A | O |
| ATOM | 468 | N | MET | A | 101 | -0.143 | 6.579 | 12.208 | 1.00 | 48.46 | A | N |
| ATOM | 469 | CA | MET | A | 101 | 0.443 | 5.264 | 12.401 | 1.00 | 48.46 | A | C |
| ATOM | 470 | CB | MET | A | 101 | 1.957 | 5.406 | 12.470 | 1.00 | 72.51 | A | C |
| ATOM | 471 | CG | MET | A | 101 | 2.695 | 4.126 | 12.208 | 1.00 | 72.51 | A | C |
| ATOM | 472 | SD | MET | A | 101 | 2.476 | 3.640 | 10.478 | 1.00 | 72.51 | A | S |
| ATOM | 473 | CE | MET | A | 101 | 4.033 | 4.350 | 9.712 | 1.00 | 72.51 | A | C |
| ATOM | 474 | C | MET | A | 101 | -0.049 | 4.605 | 13.675 | 1.00 | 48.46 | A | C |
| ATOM | 475 | O | MET | A | 101 | -0.508 | 3.460 | 13.668 | 1.00 | 48.46 | A | O |
| ATOM | 476 | N | ARG | A | 102 | 0.056 | 5.354 | 14.769 | 1.00 | 55.26 | A | N |
| ATOM | 477 | CA | ARG | A | 102 | -0.315 | 4.917 | 16.112 | 1.00 | 55.26 | A | C |
| ATOM | 478 | CB | ARG | A | 102 | -0.035 | 6.073 | 17.081 | 1.00 | 63.42 | A | C |
| ATOM | 479 | CG | ARG | A | 102 | 0.037 | 5.709 | 18.559 | 1.00 | 63.42 | A | C |
| ATOM | 480 | CD | ARG | A | 102 | 1.479 | 5.571 | 19.049 | 1.00 | 63.42 | A | C |
| ATOM | 481 | NE | ARG | A | 102 | 1.547 | 5.496 | 20.508 | 1.00 | 63.42 | A | N |
| ATOM | 482 | CZ | ARG | A | 102 | 1.397 | 6.545 | 21.309 | 1.00 | 63.42 | A | C |
| ATOM | 483 | NH1 | ARG | A | 102 | 1.180 | 7.744 | 20.787 | 1.00 | 63.42 | A | N |
| ATOM | 484 | NH2 | ARG | A | 102 | 1.451 | 6.395 | 22.626 | 1.00 | 63.42 | A | N |
| ATOM | 485 | C | ARG | A | 102 | -1.754 | 4.409 | 16.309 | 1.00 | 55.26 | A | C |
| ATOM | 486 | O | ARG | A | 102 | -2.015 | 3.671 | 17.255 | 1.00 | 55.26 | A | O |
| ATOM | 487 | N | LYS | A | 103 | -2.688 | 4.785 | 15.434 | 1.00 | 51.59 | A | N |
| ATOM | 488 | CA | LYS | A | 103 | -4.078 | 4.345 | 15.605 | 1.00 | 51.59 | A | C |
| ATOM | 489 | CB | LYS | A | 103 | -5.032 | 5.550 | 15.582 | 1.00 | 61.81 | A | C |
| ATOM | 490 | CG | LYS | A | 103 | -5.334 | 6.098 | 14.194 | 1.00 | 61.81 | A | C |
| ATOM | 491 | CD | LYS | A | 103 | -6.380 | 7.209 | 14.220 | 1.00 | 61.81 | A | C |
| ATOM | 492 | CE | LYS | A | 103 | -5.815 | 8.506 | 14.781 | 1.00 | 61.81 | A | C |
| ATOM | 493 | NZ | LYS | A | 103 | -6.654 | 9.689 | 14.405 | 1.00 | 61.81 | A | N |
| ATOM | 494 | C | LYS | A | 103 | -4.543 | 3.325 | 14.575 | 1.00 | 51.59 | A | C |
| ATOM | 495 | O | LYS | A | 103 | -5.745 | 3.083 | 14.430 | 1.00 | 51.59 | A | O |
| ATOM | 496 | N | LEU | A | 104 | -3.591 | 2.730 | 13.867 | 1.00 | 43.94 | A | N |
| ATOM | 497 | CA | LEU | A | 104 | -3.899 | 1.732 | 12.848 | 1.00 | 43.94 | A | C |
| ATOM | 498 | CB | LEU | A | 104 | -3.190 | 2.095 | 11.543 | 1.00 | 40.73 | A | C |
| ATOM | 499 | CG | LEU | A | 104 | -3.911 | 2.930 | 10.484 | 1.00 | 40.73 | A | C |
| ATOM | 500 | CD1 | LEU | A | 104 | -4.723 | 4.044 | 11.103 | 1.00 | 40.73 | A | C |
| ATOM | 501 | CD2 | LEU | A | 104 | -2.867 | 3.474 | 9.536 | 1.00 | 40.73 | A | C |
| ATOM | 502 | C | LEU | A | 104 | -3.479 | 0.327 | 13.275 | 1.00 | 43.94 | A | C |
| ATOM | 503 | O | LEU | A | 104 | -2.417 | 0.142 | 13.863 | 1.00 | 43.94 | A | O |
| ATOM | 504 | N | ASP | A | 105 | -4.322 | -0.655 | 12.988 | 1.00 | 35.34 | A | N |
| ATOM | 505 | CA | ASP | A | 105 | -4.012 | -2.029 | 13.311 | 1.00 | 35.34 | A | C |
| ATOM | 506 | CB | ASP | A | 105 | -4.532 | -2.419 | 14.695 | 1.00 | 52.66 | A | C |
| ATOM | 507 | CG | ASP | A | 105 | -4.064 | -3.814 | 15.116 | 1.00 | 52.66 | A | C |
| ATOM | 508 | OD1 | ASP | A | 105 | -3.361 | -4.481 | 14.310 | 1.00 | 52.66 | A | O |
| ATOM | 509 | OD2 | ASP | A | 105 | -4.396 | -4.243 | 16.247 | 1.00 | 52.66 | A | O |
| ATOM | 510 | C | ASP | A | 105 | -4.650 | -2.919 | 12.258 | 1.00 | 35.34 | A | C |
| ATOM | 511 | O | ASP | A | 105 | -5.771 | -3.432 | 12.422 | 1.00 | 35.34 | A | O |
| ATOM | 512 | N | HIS | A | 106 | -3.954 | -3.075 | 11.145 | 1.00 | 66.25 | A | N |
| ATOM | 513 | CA | HIS | A | 106 | -4.446 | -3.930 | 10.082 | 1.00 | 66.25 | A | C |
| ATOM | 514 | CB | HIS | A | 106 | -4.983 | -3.060 | 8.933 | 1.00 | 59.60 | A | C |
| ATOM | 515 | CG | HIS | A | 106 | -5.963 | -3.751 | 8.057 | 1.00 | 59.60 | A | C |
| ATOM | 516 | CD2 | HIS | A | 106 | -7.309 | -3.637 | 7.940 | 1.00 | 59.60 | A | C |
| ATOM | 517 | ND1 | HIS | A | 106 | -5.561 | -4.678 | 7.129 | 1.00 | 59.60 | A | N |
| ATOM | 518 | CE1 | HIS | A | 106 | -6.620 | -5.109 | 6.465 | 1.00 | 59.60 | A | C |
| ATOM | 519 | NE2 | HIS | A | 106 | -7.691 | -4.495 | 6.937 | 1.00 | 59.60 | A | N |
| ATOM | 520 | C | HIS | A | 106 | -3.263 | -4.750 | 9.622 | 1.00 | 66.25 | A | C |
| ATOM | 521 | O | HIS | A | 106 | -2.149 | -4.273 | 9.524 | 1.00 | 66.25 | A | O |
| ATOM | 522 | N | CYS | A | 107 | -3.586 | -6.021 | 9.323 | 1.00 | 41.53 | A | N |
| ATOM | 523 | CA | CYS | A | 107 | -2.597 | -7.014 | 8.872 | 1.00 | 41.53 | A | C |
| ATOM | 524 | CB | CYS | A | 107 | -2.931 | -8.627 | 9.072 | 1.00 | 83.38 | A | C |
| ATOM | 525 | SG | CYS | A | 107 | -2.691 | -8.995 | 10.899 | 1.00 | 83.38 | A | S |
| ATOM | 526 | C | CYS | A | 107 | -2.088 | -6.676 | 7.538 | 1.00 | 41.53 | A | C |
| ATOM | 527 | O | CYS | A | 107 | -1.202 | -7.263 | 7.076 | 1.00 | 41.53 | A | O |
| ATOM | 528 | N | ASN | A | 108 | -2.599 | -5.602 | 6.960 | 1.00 | 47.72 | A | N |
| ATOM | 529 | CA | ASN | A | 108 | -2.137 | -5.202 | 5.630 | 1.00 | 47.72 | A | C |

| | | | | | | | | | | | | |
|---|---|---|---|---|---|---|---|---|---|---|---|---|
| ATOM | 530 | CB | ASN | A | 108 | -3.295 | -5.205 | 4.581 | 1.00 | 46.00 | A | C |
| ATOM | 531 | CG | ASN | A | 108 | -3.663 | -6.616 | 4.121 | 1.00 | 46.00 | A | C |
| ATOM | 532 | OD1 | ASN | A | 108 | -4.809 | -7.058 | 4.250 | 1.00 | 46.00 | A | O |
| ATOM | 533 | ND2 | ASN | A | 108 | -2.667 | -7.345 | 3.619 | 1.00 | 46.00 | A | N |
| ATOM | 534 | C | ASN | A | 108 | -1.426 | -3.894 | 5.656 | 1.00 | 47.72 | A | C |
| ATOM | 535 | O | ASN | A | 108 | -1.186 | -3.268 | 4.630 | 1.00 | 47.72 | A | O |
| ATOM | 536 | N | ILE | A | 109 | -1.035 | -3.541 | 6.866 | 1.00 | 34.69 | A | N |
| ATOM | 537 | CA | ILE | A | 109 | -0.294 | -2.325 | 7.098 | 1.00 | 34.69 | A | C |
| ATOM | 538 | CB | ILE | A | 109 | -1.193 | -1.245 | 7.755 | 1.00 | 39.96 | A | C |
| ATOM | 539 | CG2 | ILE | A | 109 | -0.393 | 0.008 | 8.082 | 1.00 | 39.96 | A | C |
| ATOM | 540 | CG1 | ILE | A | 109 | -2.321 | -0.877 | 6.805 | 1.00 | 39.96 | A | C |
| ATOM | 541 | CD1 | ILE | A | 109 | -3.240 | 0.198 | 7.337 | 1.00 | 39.96 | A | C |
| ATOM | 542 | C | ILE | A | 109 | 0.907 | -2.613 | 7.994 | 1.00 | 34.69 | A | C |
| ATOM | 543 | O | ILE | A | 109 | 0.771 | -3.363 | 8.956 | 1.00 | 34.69 | A | O |
| ATOM | 544 | N | VAL | A | 110 | 2.077 | -2.040 | 7.686 | 1.00 | 33.72 | A | N |
| ATOM | 545 | CA | VAL | A | 110 | 3.240 | -2.283 | 8.542 | 1.00 | 33.72 | A | C |
| ATOM | 546 | CB | VAL | A | 110 | 4.482 | -1.447 | 8.181 | 1.00 | 40.92 | A | C |
| ATOM | 547 | CG1 | VAL | A | 110 | 4.971 | -1.804 | 6.818 | 1.00 | 40.92 | A | C |
| ATOM | 548 | CG2 | VAL | A | 110 | 4.164 | 0.024 | 8.270 | 1.00 | 40.92 | A | C |
| ATOM | 549 | C | VAL | A | 110 | 2.847 | -1.881 | 9.940 | 1.00 | 33.72 | A | C |
| ATOM | 550 | O | VAL | A | 110 | 2.199 | -0.861 | 10.139 | 1.00 | 33.72 | A | O |
| ATOM | 551 | N | ARG | A | 111 | 3.230 | -2.690 | 10.910 | 1.00 | 39.88 | A | N |
| ATOM | 552 | CA | ARG | A | 111 | 2.909 | -2.386 | 12.287 | 1.00 | 39.88 | A | C |
| ATOM | 553 | CB | ARG | A | 111 | 2.830 | -3.661 | 13.063 | 1.00 | 71.21 | A | C |
| ATOM | 554 | CG | ARG | A | 111 | 2.484 | -3.507 | 14.470 | 1.00 | 71.21 | A | C |
| ATOM | 555 | CD | ARG | A | 111 | 2.683 | -4.805 | 14.701 | 1.00 | 71.21 | A | C |
| ATOM | 556 | NE | ARG | A | 111 | 2.487 | -5.205 | 15.981 | 1.00 | 71.21 | A | N |
| ATOM | 557 | CZ | ARG | A | 111 | 2.856 | -6.362 | 16.460 | 1.00 | 71.21 | A | C |
| ATOM | 558 | NH1 | ARG | A | 111 | 2.514 | -6.514 | 17.713 | 1.00 | 71.21 | A | N |
| ATOM | 559 | NH2 | ARG | A | 111 | 3.540 | -7.288 | 15.748 | 1.00 | 71.21 | A | N |
| ATOM | 560 | C | ARG | A | 111 | 3.995 | -1.504 | 12.872 | 1.00 | 39.88 | A | C |
| ATOM | 561 | O | ARG | A | 111 | 5.171 | -1.607 | 12.497 | 1.00 | 39.88 | A | O |
| ATOM | 562 | N | LEU | A | 112 | 3.591 | -0.606 | 13.761 | 1.00 | 45.04 | A | N |
| ATOM | 563 | CA | LEU | A | 112 | 4.534 | 0.281 | 14.420 | 1.00 | 45.04 | A | C |
| ATOM | 564 | CB | LEU | A | 112 | 3.934 | 1.678 | 14.596 | 1.00 | 30.01 | A | C |
| ATOM | 565 | CG | LEU | A | 112 | 4.728 | 2.665 | 15.453 | 1.00 | 30.01 | A | C |
| ATOM | 566 | CD1 | LEU | A | 112 | 5.918 | 3.202 | 14.666 | 1.00 | 30.01 | A | C |
| ATOM | 567 | CD2 | LEU | A | 112 | 3.826 | 3.804 | 15.885 | 1.00 | 30.01 | A | C |
| ATOM | 568 | C | LEU | A | 112 | 4.717 | -0.380 | 15.773 | 1.00 | 45.04 | A | C |
| ATOM | 569 | O | LEU | A | 112 | 3.867 | -0.237 | 16.646 | 1.00 | 45.04 | A | O |
| ATOM | 570 | N | ARG | A | 113 | 5.812 | -1.124 | 15.931 | 1.00 | 46.44 | A | N |
| ATOM | 571 | CA | ARG | A | 113 | 6.116 | -1.832 | 17.177 | 1.00 | 46.44 | A | C |
| ATOM | 572 | CB | ARG | A | 113 | 7.362 | -2.704 | 16.996 | 1.00 | 65.74 | A | C |
| ATOM | 573 | CG | ARG | A | 113 | 7.298 | -3.660 | 15.814 | 1.00 | 65.74 | A | C |
| ATOM | 574 | CD | ARG | A | 113 | 6.277 | -4.772 | 16.049 | 1.00 | 65.74 | A | C |
| ATOM | 575 | NE | ARG | A | 113 | 6.838 | -5.906 | 16.784 | 1.00 | 65.74 | A | N |
| ATOM | 576 | CZ | ARG | A | 113 | 7.749 | -6.743 | 16.283 | 1.00 | 65.74 | A | C |
| ATOM | 577 | NH1 | ARG | A | 113 | 8.206 | -6.574 | 15.038 | 1.00 | 65.74 | A | N |
| ATOM | 578 | NH2 | ARG | A | 113 | 8.199 | -7.755 | 17.021 | 1.00 | 65.74 | A | N |
| ATOM | 579 | C | ARG | A | 113 | 6.339 | -0.862 | 18.336 | 1.00 | 46.44 | A | C |
| ATOM | 580 | O | ARG | A | 113 | 5.774 | -1.035 | 19.414 | 1.00 | 46.44 | A | O |
| ATOM | 581 | N | TYR | A | 114 | 7.166 | 0.152 | 18.100 | 1.00 | 37.76 | A | N |
| ATOM | 582 | CA | TYR | A | 114 | 7.480 | 1.155 | 19.109 | 1.00 | 37.76 | A | C |
| ATOM | 583 | CB | TYR | A | 114 | 8.698 | 0.741 | 19.961 | 1.00 | 42.56 | A | C |
| ATOM | 584 | CG | TYR | A | 114 | 8.700 | -0.677 | 20.470 | 1.00 | 42.56 | A | C |
| ATOM | 585 | CD1 | TYR | A | 114 | 7.858 | -1.067 | 21.508 | 1.00 | 42.56 | A | C |
| ATOM | 586 | CE1 | TYR | A | 114 | 7.820 | -2.391 | 21.941 | 1.00 | 42.56 | A | C |
| ATOM | 587 | CD2 | TYR | A | 114 | 9.510 | -1.644 | 19.879 | 1.00 | 42.56 | A | C |
| ATOM | 588 | CE2 | TYR | A | 114 | 9.476 | -2.968 | 20.303 | 1.00 | 42.56 | A | C |
| ATOM | 589 | CZ | TYR | A | 114 | 8.628 | -3.337 | 21.330 | 1.00 | 42.56 | A | C |
| ATOM | 590 | OH | TYR | A | 114 | 8.562 | -4.651 | 21.727 | 1.00 | 42.56 | A | O |
| ATOM | 591 | C | TYR | A | 114 | 7.879 | 2.430 | 18.395 | 1.00 | 37.76 | A | C |
| ATOM | 592 | O | TYR | A | 114 | 7.934 | 2.492 | 17.167 | 1.00 | 37.76 | A | O |
| ATOM | 593 | N | PHE | A | 115 | 8.168 | 3.447 | 19.190 | 1.00 | 41.62 | A | N |
| ATOM | 594 | CA | PHE | A | 115 | 8.655 | 4.705 | 18.673 | 1.00 | 41.62 | A | C |
| ATOM | 595 | CB | PHE | A | 115 | 7.510 | 5.596 | 18.170 | 1.00 | 39.31 | A | C |
| ATOM | 596 | CG | PHE | A | 115 | 6.664 | 6.201 | 19.246 | 1.00 | 39.31 | A | C |

| | | | | | | | | | | | | |
|---|---|---|---|---|---|---|---|---|---|---|---|---|
| ATOM | 597 | CD1 | PHE | A | 115 | 6.982 | 7.443 | 19.783 | 1.00 | 39.31 | A | C |
| ATOM | 598 | CD2 | PHE | A | 115 | 5.511 | 5.563 | 19.674 | 1.00 | 39.31 | A | C |
| ATOM | 599 | CE1 | PHE | A | 115 | 6.160 | 8.042 | 20.727 | 1.00 | 39.31 | A | C |
| ATOM | 600 | CE2 | PHE | A | 115 | 4.680 | 6.153 | 20.619 | 1.00 | 39.31 | A | C |
| ATOM | 601 | CZ | PHE | A | 115 | 5.004 | 7.393 | 21.143 | 1.00 | 39.31 | A | C |
| ATOM | 602 | C | PHE | A | 115 | 9.417 | 5.309 | 19.842 | 1.00 | 41.62 | A | C |
| ATOM | 603 | O | PHE | A | 115 | 9.045 | 5.127 | 21.002 | 1.00 | 41.62 | A | O |
| ATOM | 604 | N | PHE | A | 116 | 10.521 | 5.976 | 19.543 | 1.00 | 47.22 | A | N |
| ATOM | 605 | CA | PHE | A | 116 | 11.328 | 6.563 | 20.589 | 1.00 | 47.22 | A | C |
| ATOM | 606 | CB | PHE | A | 116 | 12.311 | 5.511 | 21.129 | 1.00 | 48.62 | A | C |
| ATOM | 607 | CG | PHE | A | 116 | 13.402 | 5.116 | 20.164 | 1.00 | 48.62 | A | C |
| ATOM | 608 | CD1 | PHE | A | 116 | 14.551 | 5.896 | 20.026 | 1.00 | 48.62 | A | C |
| ATOM | 609 | CD2 | PHE | A | 116 | 13.305 | 3.938 | 19.427 | 1.00 | 48.62 | A | C |
| ATOM | 610 | CE1 | PHE | A | 116 | 15.588 | 5.505 | 19.174 | 1.00 | 48.62 | A | C |
| ATOM | 611 | CE2 | PHE | A | 116 | 14.342 | 3.537 | 18.565 | 1.00 | 48.62 | A | C |
| ATOM | 612 | CZ | PHE | A | 116 | 15.483 | 4.321 | 18.441 | 1.00 | 48.62 | A | C |
| ATOM | 613 | C | PHE | A | 116 | 12.055 | 7.784 | 20.055 | 1.00 | 47.22 | A | C |
| ATOM | 614 | O | PHE | A | 116 | 12.110 | 7.997 | 18.848 | 1.00 | 47.22 | A | O |
| ATOM | 615 | N | TYR | A | 117 | 12.594 | 8.591 | 20.958 | 1.00 | 55.46 | A | N |
| ATOM | 616 | CA | TYR | A | 117 | 13.307 | 9.801 | 20.579 | 1.00 | 55.46 | A | C |
| ATOM | 617 | CB | TYR | A | 117 | 12.759 | 10.976 | 21.379 | 1.00 | 50.26 | A | C |
| ATOM | 618 | CG | TYR | A | 117 | 11.298 | 11.178 | 21.101 | 1.00 | 50.26 | A | C |
| ATOM | 619 | CD1 | TYR | A | 117 | 10.877 | 11.931 | 20.003 | 1.00 | 50.26 | A | C |
| ATOM | 620 | CE1 | TYR | A | 117 | 9.532 | 12.036 | 19.673 | 1.00 | 50.26 | A | C |
| ATOM | 621 | CD2 | TYR | A | 117 | 10.330 | 10.535 | 21.871 | 1.00 | 50.26 | A | C |
| ATOM | 622 | CE2 | TYR | A | 117 | 8.982 | 10.629 | 21.549 | 1.00 | 50.26 | A | C |
| ATOM | 623 | CZ | TYR | A | 117 | 8.590 | 11.381 | 20.446 | 1.00 | 50.26 | A | C |
| ATOM | 624 | OH | TYR | A | 117 | 7.256 | 11.460 | 20.117 | 1.00 | 50.26 | A | O |
| ATOM | 625 | C | TYR | A | 117 | 14.809 | 9.654 | 20.790 | 1.00 | 55.46 | A | C |
| ATOM | 626 | O | TYR | A | 117 | 15.268 | 8.706 | 21.435 | 1.00 | 55.46 | A | O |
| ATOM | 627 | N | SER | A | 118 | 15.577 | 10.593 | 20.247 | 1.00 | 47.23 | A | N |
| ATOM | 628 | CA | SER | A | 118 | 17.016 | 10.521 | 20.376 | 1.00 | 47.23 | A | C |
| ATOM | 629 | CB | SER | A | 118 | 17.528 | 9.333 | 19.563 | 1.00 | 44.24 | A | C |
| ATOM | 630 | OG | SER | A | 118 | 16.956 | 9.339 | 18.268 | 1.00 | 44.24 | A | O |
| ATOM | 631 | C | SER | A | 118 | 17.728 | 11.799 | 19.943 | 1.00 | 47.23 | A | C |
| ATOM | 632 | O | SER | A | 118 | 17.106 | 12.746 | 19.439 | 1.00 | 47.23 | A | O |
| ATOM | 633 | N | SER | A | 119 | 19.045 | 11.810 | 20.142 | 1.00 | 80.15 | A | N |
| ATOM | 634 | CA | SER | A | 119 | 19.869 | 12.960 | 19.797 | 1.00 | 80.15 | A | C |
| ATOM | 635 | CB | SER | A | 119 | 20.786 | 13.314 | 20.972 | 1.00 | 65.16 | A | C |
| ATOM | 636 | OG | SER | A | 119 | 20.023 | 13.692 | 22.113 | 1.00 | 65.16 | A | O |
| ATOM | 637 | C | SER | A | 119 | 20.700 | 12.685 | 18.552 | 1.00 | 80.15 | A | C |
| ATOM | 638 | O | SER | A | 119 | 20.668 | 13.464 | 17.597 | 1.00 | 80.15 | A | O |
| ATOM | 639 | N | ALA | A | 125 | 18.556 | 19.312 | 18.875 | 1.00100.00 | | A | N |
| ATOM | 640 | CA | ALA | A | 125 | 17.422 | 18.810 | 18.085 | 1.00100.00 | | A | C |
| ATOM | 641 | CB | ALA | A | 125 | 17.781 | 18.803 | 16.579 | 1.00 | 55.28 | A | C |
| ATOM | 642 | C | ALA | A | 125 | 16.994 | 17.400 | 18.538 | 1.00100.00 | | A | C |
| ATOM | 643 | O | ALA | A | 125 | 17.803 | 16.628 | 19.090 | 1.00100.00 | | A | O |
| ATOM | 644 | N | ALA | A | 126 | 15.725 | 17.064 | 18.310 | 1.00 | 67.09 | A | N |
| ATOM | 645 | CA | ALA | A | 126 | 15.204 | 15.758 | 18.705 | 1.00 | 67.09 | A | C |
| ATOM | 646 | CB | ALA | A | 126 | 14.117 | 15.934 | 19.773 | 1.00 | 30.53 | A | C |
| ATOM | 647 | C | ALA | A | 126 | 14.650 | 14.999 | 17.498 | 1.00 | 67.09 | A | C |
| ATOM | 648 | O | ALA | A | 126 | 13.778 | 15.501 | 16.797 | 1.00 | 67.09 | A | O |
| ATOM | 649 | N | TYR | A | 127 | 15.165 | 13.795 | 17.258 | 1.00 | 60.33 | A | N |
| ATOM | 650 | CA | TYR | A | 127 | 14.707 | 12.966 | 16.141 | 1.00 | 60.33 | A | C |
| ATOM | 651 | CB | TYR | A | 127 | 15.864 | 12.182 | 15.516 | 1.00 | 75.69 | A | C |
| ATOM | 652 | CG | TYR | A | 127 | 17.031 | 13.009 | 15.034 | 1.00 | 75.69 | A | C |
| ATOM | 653 | CD1 | TYR | A | 127 | 17.815 | 13.733 | 15.934 | 1.00 | 75.69 | A | C |
| ATOM | 654 | CE1 | TYR | A | 127 | 18.930 | 14.465 | 15.503 | 1.00 | 75.69 | A | C |
| ATOM | 655 | CD2 | TYR | A | 127 | 17.381 | 13.036 | 13.677 | 1.00 | 75.69 | A | C |
| ATOM | 656 | CE2 | TYR | A | 127 | 18.491 | 13.761 | 13.230 | 1.00 | 75.69 | A | C |
| ATOM | 657 | CZ | TYR | A | 127 | 19.265 | 14.474 | 14.149 | 1.00 | 75.69 | A | C |
| ATOM | 658 | OH | TYR | A | 127 | 20.380 | 15.184 | 13.728 | 1.00 | 75.69 | A | O |
| ATOM | 659 | C | TYR | A | 127 | 13.683 | 11.946 | 16.626 | 1.00 | 60.33 | A | C |
| ATOM | 660 | O | TYR | A | 127 | 13.832 | 11.376 | 17.706 | 1.00 | 60.33 | A | O |
| ATOM | 661 | N | LEU | A | 128 | 12.651 | 11.711 | 15.824 | 1.00 | 60.13 | A | N |
| ATOM | 662 | CA | LEU | A | 128 | 11.627 | 10.728 | 16.172 | 1.00 | 60.13 | A | C |
| ATOM | 663 | CB | LEU | A | 128 | 10.270 | 11.160 | 15.620 | 1.00 | 48.37 | A | C |

| | | | | | | | | | | | | |
|---|---|---|---|---|---|---|---|---|---|---|---|---|
| ATOM | 664 | CG | LEU | A | 128 | 9.144 | 10.139 | 15.730 | 1.00 | 48.37 | A | C |
| ATOM | 665 | CD1 | LEU | A | 128 | 8.869 | 9.841 | 17.192 | 1.00 | 48.37 | A | C |
| ATOM | 666 | CD2 | LEU | A | 128 | 7.905 | 10.679 | 15.040 | 1.00 | 48.37 | A | C |
| ATOM | 667 | C | LEU | A | 128 | 12.049 | 9.412 | 15.535 | 1.00 | 60.13 | A | C |
| ATOM | 668 | O | LEU | A | 128 | 12.610 | 9.406 | 14.438 | 1.00 | 60.13 | A | O |
| ATOM | 669 | N | ASN | A | 129 | 11.784 | 8.297 | 16.201 | 1.00 | 40.24 | A | N |
| ATOM | 670 | CA | ASN | A | 129 | 12.184 | 7.014 | 15.642 | 1.00 | 40.24 | A | C |
| ATOM | 671 | CB | ASN | A | 129 | 13.313 | 6.412 | 16.477 | 1.00 | 41.77 | A | C |
| ATOM | 672 | CG | ASN | A | 129 | 14.578 | 7.258 | 16.442 | 1.00 | 41.77 | A | C |
| ATOM | 673 | OD1 | ASN | A | 129 | 15.482 | 7.011 | 15.647 | 1.00 | 41.77 | A | O |
| ATOM | 674 | ND2 | ASN | A | 129 | 14.637 | 8.272 | 17.299 | 1.00 | 41.77 | A | N |
| ATOM | 675 | C | ASN | A | 129 | 11.022 | 6.051 | 15.574 | 1.00 | 40.24 | A | C |
| ATOM | 676 | O | ASN | A | 129 | 10.458 | 5.664 | 16.594 | 1.00 | 40.24 | A | O |
| ATOM | 677 | N | LEU | A | 130 | 10.660 | 5.672 | 14.356 | 1.00 | 41.54 | A | N |
| ATOM | 678 | CA | LEU | A | 130 | 9.566 | 4.742 | 14.151 | 1.00 | 41.54 | A | C |
| ATOM | 679 | CB | LEU | A | 130 | 8.745 | 5.165 | 12.928 | 1.00 | 43.12 | A | C |
| ATOM | 680 | CG | LEU | A | 130 | 8.118 | 6.554 | 13.072 | 1.00 | 43.12 | A | C |
| ATOM | 681 | CD1 | LEU | A | 130 | 7.619 | 7.053 | 11.740 | 1.00 | 43.12 | A | C |
| ATOM | 682 | CD2 | LEU | A | 130 | 6.993 | 6.489 | 14.091 | 1.00 | 43.12 | A | C |
| ATOM | 683 | C | LEU | A | 130 | 10.157 | 3.358 | 13.943 | 1.00 | 41.54 | A | C |
| ATOM | 684 | O | LEU | A | 130 | 10.921 | 3.144 | 13.010 | 1.00 | 41.54 | A | O |
| ATOM | 685 | N | VAL | A | 131 | 9.822 | 2.429 | 14.830 | 1.00 | 41.93 | A | N |
| ATOM | 686 | CA | VAL | A | 131 | 10.318 | 1.062 | 14.729 | 1.00 | 41.93 | A | C |
| ATOM | 687 | CB | VAL | A | 131 | 10.590 | 0.454 | 16.098 | 1.00 | 41.42 | A | C |
| ATOM | 688 | CG1 | VAL | A | 131 | 11.290 | -0.878 | 15.920 | 1.00 | 41.42 | A | C |
| ATOM | 689 | CG2 | VAL | A | 131 | 11.400 | 1.422 | 16.956 | 1.00 | 41.42 | A | C |
| ATOM | 690 | C | VAL | A | 131 | 9.220 | 0.252 | 14.097 | 1.00 | 41.93 | A | C |
| ATOM | 691 | O | VAL | A | 131 | 8.219 | -0.030 | 14.749 | 1.00 | 41.93 | A | O |
| ATOM | 692 | N | LEU | A | 132 | 9.420 | -0.141 | 12.842 | 1.00 | 38.60 | A | N |
| ATOM | 693 | CA | LEU | A | 132 | 8.413 | -0.888 | 12.095 | 1.00 | 38.60 | A | C |
| ATOM | 694 | CB | LEU | A | 132 | 8.106 | -0.139 | 10.811 | 1.00 | 29.05 | A | C |
| ATOM | 695 | CG | LEU | A | 132 | 7.907 | 1.342 | 11.085 | 1.00 | 29.05 | A | C |
| ATOM | 696 | CD1 | LEU | A | 132 | 8.363 | 2.161 | 9.900 | 1.00 | 29.05 | A | C |
| ATOM | 697 | CD2 | LEU | A | 132 | 6.453 | 1.580 | 11.440 | 1.00 | 29.05 | A | C |
| ATOM | 698 | C | LEU | A | 132 | 8.818 | -2.302 | 11.741 | 1.00 | 38.60 | A | C |
| ATOM | 699 | O | LEU | A | 132 | 10.009 | -2.606 | 11.641 | 1.00 | 38.60 | A | O |
| ATOM | 700 | N | ASP | A | 133 | 7.817 | -3.162 | 11.551 | 1.00 | 44.86 | A | N |
| ATOM | 701 | CA | ASP | A | 133 | 8.057 | -4.545 | 11.154 | 1.00 | 44.86 | A | C |
| ATOM | 702 | CB | ASP | A | 133 | 6.751 | -5.253 | 10.798 | 1.00 | 99.49 | A | C |
| ATOM | 703 | CG | ASP | A | 133 | 5.908 | -5.579 | 12.012 | 1.00 | 99.49 | A | C |
| ATOM | 704 | OD1 | ASP | A | 133 | 4.693 | -5.842 | 11.823 | 1.00 | 99.49 | A | O |
| ATOM | 705 | OD2 | ASP | A | 133 | 6.451 | -5.584 | 13.150 | 1.00 | 99.49 | A | O |
| ATOM | 706 | C | ASP | A | 133 | 8.908 | -4.483 | 9.902 | 1.00 | 44.86 | A | C |
| ATOM | 707 | O | ASP | A | 133 | 8.776 | -3.566 | 9.092 | 1.00 | 44.86 | A | O |
| ATOM | 708 | N | TYR | A | 134 | 9.789 | -5.454 | 9.741 | 1.00 | 48.67 | A | N |
| ATOM | 709 | CA | TYR | A | 134 | 10.622 | -5.475 | 8.563 | 1.00 | 48.67 | A | C |
| ATOM | 710 | CB | TYR | A | 134 | 12.027 | -5.962 | 8.890 | 1.00 | 47.26 | A | C |
| ATOM | 711 | CG | TYR | A | 134 | 12.946 | -5.883 | 7.700 | 1.00 | 47.26 | A | C |
| ATOM | 712 | CD1 | TYR | A | 134 | 13.378 | -4.647 | 7.218 | 1.00 | 47.26 | A | C |
| ATOM | 713 | CE1 | TYR | A | 134 | 14.183 | -4.550 | 6.092 | 1.00 | 47.26 | A | C |
| ATOM | 714 | CD2 | TYR | A | 134 | 13.345 | -7.033 | 7.020 | 1.00 | 47.26 | A | C |
| ATOM | 715 | CE2 | TYR | A | 134 | 14.151 | -6.951 | 5.883 | 1.00 | 47.26 | A | C |
| ATOM | 716 | CZ | TYR | A | 134 | 14.564 | -5.705 | 5.427 | 1.00 | 47.26 | A | C |
| ATOM | 717 | OH | TYR | A | 134 | 15.352 | -5.608 | 4.304 | 1.00 | 47.26 | A | O |
| ATOM | 718 | C | TYR | A | 134 | 10.006 | -6.428 | 7.561 | 1.00 | 48.67 | A | C |
| ATOM | 719 | O | TYR | A | 134 | 9.533 | -7.500 | 7.931 | 1.00 | 48.67 | A | O |
| ATOM | 720 | N | VAL | A | 135 | 9.984 | -6.020 | 6.300 | 1.00 | 41.66 | A | N |
| ATOM | 721 | CA | VAL | A | 135 | 9.479 | -6.867 | 5.242 | 1.00 | 41.66 | A | C |
| ATOM | 722 | CB | VAL | A | 135 | 8.079 | -6.471 | 4.816 | 1.00 | 41.85 | A | C |
| ATOM | 723 | CG1 | VAL | A | 135 | 7.482 | -7.573 | 3.984 | 1.00 | 41.85 | A | C |
| ATOM | 724 | CG2 | VAL | A | 135 | 7.223 | -6.224 | 6.036 | 1.00 | 41.85 | A | C |
| ATOM | 725 | C | VAL | A | 135 | 10.486 | -6.597 | 4.141 | 1.00 | 41.66 | A | C |
| ATOM | 726 | O | VAL | A | 135 | 10.705 | -5.442 | 3.760 | 1.00 | 41.66 | A | O |
| ATOM | 727 | N | PRO | A | 136 | 11.135 | -7.663 | 3.633 | 1.00 | 55.48 | A | N |
| ATOM | 728 | CD | PRO | A | 136 | 10.891 | -9.056 | 4.067 | 1.00 | 54.70 | A | C |
| ATOM | 729 | CA | PRO | A | 136 | 12.159 | -7.617 | 2.582 | 1.00 | 55.48 | A | C |
| ATOM | 730 | CB | PRO | A | 136 | 12.811 | -8.991 | 2.695 | 1.00 | 54.70 | A | C |

| | | | | | | | | | | | | |
|---|---|---|---|---|---|---|---|---|---|---|---|---|
| ATOM | 731 | CG | PRO | A | 136 | 11.625 | -9.872 | 3.017 | 1.00 | 54.70 | A | C |
| ATOM | 732 | C | PRO | A | 136 | 11.740 | -7.296 | 1.153 | 1.00 | 55.48 | A | C |
| ATOM | 733 | O | PRO | A | 136 | 12.399 | -6.516 | 0.467 | 1.00 | 55.48 | A | O |
| ATOM | 734 | N | GLU | A | 137 | 10.658 | -7.895 | 0.685 | 1.00 | 50.56 | A | N |
| ATOM | 735 | CA | GLU | A | 137 | 10.244 | -7.628 | -0.682 | 1.00 | 50.56 | A | C |
| ATOM | 736 | CB | GLU | A | 137 | 9.635 | -8.885 | -1.314 | 1.00 | 85.53 | A | C |
| ATOM | 737 | CG | GLU | A | 137 | 10.645 | -9.828 | -1.937 | 1.00 | 85.53 | A | C |
| ATOM | 738 | CD | GLU | A | 137 | 11.481 | -9.163 | -3.021 | 1.00 | 85.53 | A | C |
| ATOM | 739 | OE1 | GLU | A | 137 | 12.425 | -8.401 | -2.669 | 1.00 | 85.53 | A | O |
| ATOM | 740 | OE2 | GLU | A | 137 | 11.183 | -9.402 | -4.222 | 1.00 | 85.53 | A | O |
| ATOM | 741 | C | GLU | A | 137 | 9.262 | -6.479 | -0.836 | 1.00 | 50.56 | A | C |
| ATOM | 742 | O | GLU | A | 137 | 8.618 | -6.052 | 0.123 | 1.00 | 50.56 | A | O |
| ATOM | 743 | N | THR | A | 138 | 9.170 | -5.980 | -2.063 | 1.00 | 59.25 | A | N |
| ATOM | 744 | CA | THR | A | 138 | 8.237 | -4.916 | -2.398 | 1.00 | 59.25 | A | C |
| ATOM | 745 | CB | THR | A | 138 | 8.920 | -3.541 | -2.450 | 1.00 | 32.28 | A | C |
| ATOM | 746 | OG1 | THR | A | 138 | 9.829 | -3.501 | -3.555 | 1.00 | 32.28 | A | O |
| ATOM | 747 | CG2 | THR | A | 138 | 9.646 | -3.266 | -1.135 | 1.00 | 32.28 | A | C |
| ATOM | 748 | C | THR | A | 138 | 7.719 | -5.282 | -3.788 | 1.00 | 59.25 | A | C |
| ATOM | 749 | O | THR | A | 138 | 8.437 | -5.938 | -4.549 | 1.00 | 59.25 | A | O |
| ATOM | 750 | N | VAL | A | 139 | 6.477 | -4.900 | -4.102 | 1.00 | 34.89 | A | N |
| ATOM | 751 | CA | VAL | A | 139 | 5.881 | -5.185 | -5.402 | 1.00 | 34.89 | A | C |
| ATOM | 752 | CB | VAL | A | 139 | 4.454 | -4.559 | -5.514 | 1.00 | 29.64 | A | C |
| ATOM | 753 | CG1 | VAL | A | 139 | 3.922 | -4.669 | -6.945 | 1.00 | 29.64 | A | C |
| ATOM | 754 | CG2 | VAL | A | 139 | 3.509 | -5.261 | -4.567 | 1.00 | 29.64 | A | C |
| ATOM | 755 | C | VAL | A | 139 | 6.781 | -4.612 | -6.492 | 1.00 | 34.89 | A | C |
| ATOM | 756 | O | VAL | A | 139 | 6.911 | -5.192 | -7.568 | 1.00 | 34.89 | A | O |
| ATOM | 757 | N | TYR | A | 140 | 7.414 | -3.477 | -6.204 | 1.00 | 34.71 | A | N |
| ATOM | 758 | CA | TYR | A | 140 | 8.292 | -2.842 | -7.179 | 1.00 | 34.71 | A | C |
| ATOM | 759 | CB | TYR | A | 140 | 8.967 | -1.595 | -6.612 | 1.00 | 53.72 | A | C |
| ATOM | 760 | CG | TYR | A | 140 | 9.901 | -0.963 | -7.617 | 1.00 | 53.72 | A | C |
| ATOM | 761 | CD1 | TYR | A | 140 | 9.395 | -0.352 | -8.758 | 1.00 | 53.72 | A | C |
| ATOM | 762 | CE1 | TYR | A | 140 | 10.231 | 0.133 | -9.750 | 1.00 | 53.72 | A | C |
| ATOM | 763 | CD2 | TYR | A | 140 | 11.286 | -1.068 | -7.485 | 1.00 | 53.72 | A | C |
| ATOM | 764 | CE2 | TYR | A | 140 | 12.138 | -0.583 | -8.478 | 1.00 | 53.72 | A | C |
| ATOM | 765 | CZ | TYR | A | 140 | 11.595 | 0.013 | -9.612 | 1.00 | 53.72 | A | C |
| ATOM | 766 | OH | TYR | A | 140 | 12.401 | 0.473 | -10.631 | 1.00 | 53.72 | A | O |
| ATOM | 767 | C | TYR | A | 140 | 9.369 | -3.799 | -7.620 | 1.00 | 34.71 | A | C |
| ATOM | 768 | O | TYR | A | 140 | 9.569 | -4.012 | -8.808 | 1.00 | 34.71 | A | O |
| ATOM | 769 | N | ARG | A | 141 | 10.073 | -4.359 | -6.648 | 1.00 | 41.48 | A | N |
| ATOM | 770 | CA | ARG | A | 141 | 11.130 | -5.319 | -6.911 | 1.00 | 41.48 | A | C |
| ATOM | 771 | CB | ARG | A | 141 | 11.756 | -5.763 | -5.592 | 1.00 | 78.39 | A | C |
| ATOM | 772 | CG | ARG | A | 141 | 12.793 | -4.790 | -5.053 | 1.00 | 78.39 | A | C |
| ATOM | 773 | CD | ARG | A | 141 | 12.988 | -4.922 | -3.546 | 1.00 | 78.39 | A | C |
| ATOM | 774 | NE | ARG | A | 141 | 14.323 | -4.455 | -3.198 | 1.00 | 78.39 | A | N |
| ATOM | 775 | CZ | ARG | A | 141 | 15.404 | -5.230 | -3.228 | 1.00 | 78.39 | A | C |
| ATOM | 776 | NH1 | ARG | A | 141 | 15.294 | -6.516 | -3.572 | 1.00 | 78.39 | A | N |
| ATOM | 777 | NH2 | ARG | A | 141 | 16.597 | -4.711 | -2.962 | 1.00 | 78.39 | A | N |
| ATOM | 778 | C | ARG | A | 141 | 10.599 | -6.526 | -7.667 | 1.00 | 41.48 | A | C |
| ATOM | 779 | O | ARG | A | 141 | 11.172 | -6.931 | -8.676 | 1.00 | 41.48 | A | O |
| ATOM | 780 | N | VAL | A | 142 | 9.509 | -7.108 | -7.179 | 1.00 | 46.78 | A | N |
| ATOM | 781 | CA | VAL | A | 142 | 8.921 | -8.262 | -7.849 | 1.00 | 46.78 | A | C |
| ATOM | 782 | CB | VAL | A | 142 | 7.691 | -8.809 | -7.077 | 1.00 | 35.74 | A | C |
| ATOM | 783 | CG1 | VAL | A | 142 | 6.912 | -9.777 | -7.936 | 1.00 | 35.74 | A | C |
| ATOM | 784 | CG2 | VAL | A | 142 | 8.147 | -9.508 | -5.815 | 1.00 | 35.74 | A | C |
| ATOM | 785 | C | VAL | A | 142 | 8.484 | -7.885 | -9.264 | 1.00 | 46.78 | A | C |
| ATOM | 786 | O | VAL | A | 142 | 8.750 | -8.620 | -10.217 | 1.00 | 46.78 | A | O |
| ATOM | 787 | N | ALA | A | 143 | 7.823 | -6.738 | -9.401 | 1.00 | 33.50 | A | N |
| ATOM | 788 | CA | ALA | A | 143 | 7.346 | -6.303 | -10.702 | 1.00 | 33.50 | A | C |
| ATOM | 789 | CB | ALA | A | 143 | 6.599 | -4.996 | -10.565 | 1.00 | 37.56 | A | C |
| ATOM | 790 | C | ALA | A | 143 | 8.502 | -6.149 | -11.669 | 1.00 | 33.50 | A | C |
| ATOM | 791 | O | ALA | A | 143 | 8.431 | -6.604 | -12.813 | 1.00 | 33.50 | A | O |
| ATOM | 792 | N | ARG | A | 144 | 9.566 | -5.509 | -11.189 | 1.00 | 39.59 | A | N |
| ATOM | 793 | CA | ARG | A | 144 | 10.768 | -5.258 | -11.977 | 1.00 | 39.59 | A | C |
| ATOM | 794 | CB | ARG | A | 144 | 11.788 | -4.493 | -11.125 | 1.00 | 61.16 | A | C |
| ATOM | 795 | CG | ARG | A | 144 | 12.835 | -3.699 | -11.906 | 1.00 | 61.16 | A | C |
| ATOM | 796 | CD | ARG | A | 144 | 14.262 | -4.120 | -11.548 | 1.00 | 61.16 | A | C |
| ATOM | 797 | NE | ARG | A | 144 | 14.709 | -3.711 | -10.208 | 1.00 | 61.16 | A | N |

| | | | | | | | | | | | | |
|---|---|---|---|---|---|---|---|---|---|---|---|---|
| ATOM | 798 | CZ | ARG | A | 144 | 15.038 | -2.460 | -9.850 | 1.00 | 61.16 | A | C |
| ATOM | 799 | NH1 | ARG | A | 144 | 14.978 | -1.460 | -10.736 | 1.00 | 61.16 | A | N |
| ATOM | 800 | NH2 | ARG | A | 144 | 15.422 | -2.201 | -8.593 | 1.00 | 61.16 | A | N |
| ATOM | 801 | C | ARG | A | 144 | 11.365 | -6.578 | -12.443 | 1.00 | 39.59 | A | C |
| ATOM | 802 | O | ARG | A | 144 | 11.707 | -6.748 | -13.614 | 1.00 | 39.59 | A | O |
| ATOM | 803 | N | HIS | A | 145 | 11.464 | -7.519 | -11.510 | 1.00 | 55.72 | A | N |
| ATOM | 804 | CA | HIS | A | 145 | 12.029 | -8.837 | -11.778 | 1.00 | 55.72 | A | C |
| ATOM | 805 | CB | HIS | A | 145 | 12.012 | -9.665 | -10.490 | 1.00 | 95.48 | A | C |
| ATOM | 806 | CG | HIS | A | 145 | 12.852 | -10.906 | -10.547 | 1.00 | 95.48 | A | C |
| ATOM | 807 | CD2 | HIS | A | 145 | 13.446 | -11.538 | -11.590 | 1.00 | 95.48 | A | C |
| ATOM | 808 | ND1 | HIS | A | 145 | 13.081 | -11.699 | -9.437 | 1.00 | 95.48 | A | N |
| ATOM | 809 | CE1 | HIS | A | 145 | 13.773 | -12.767 | -9.798 | 1.00 | 95.48 | A | C |
| ATOM | 810 | NE2 | HIS | A | 145 | 14.005 | -12.695 | -11.099 | 1.00 | 95.48 | A | N |
| ATOM | 811 | C | HIS | A | 145 | 11.346 | -9.585 | -12.937 | 1.00 | 55.72 | A | C |
| ATOM | 812 | O | HIS | A | 145 | 12.040 | -10.149 | -13.785 | 1.00 | 55.72 | A | O |
| ATOM | 813 | N | TYR | A | 146 | 10.013 | -9.601 | -12.999 | 1.00 | 42.32 | A | N |
| ATOM | 814 | CA | TYR | A | 146 | 9.359 | -10.275 | -14.122 | 1.00 | 42.32 | A | C |
| ATOM | 815 | CB | TYR | A | 146 | 7.865 | -10.482 | -13.886 | 1.00 | 40.96 | A | C |
| ATOM | 816 | CG | TYR | A | 146 | 7.516 | -11.533 | -12.870 | 1.00 | 40.96 | A | C |
| ATOM | 817 | CD1 | TYR | A | 146 | 7.715 | -11.302 | -11.513 | 1.00 | 40.96 | A | C |
| ATOM | 818 | CE1 | TYR | A | 146 | 7.420 | -12.273 | -10.566 | 1.00 | 40.96 | A | C |
| ATOM | 819 | CD2 | TYR | A | 146 | 7.004 | -12.771 | -13.260 | 1.00 | 40.96 | A | C |
| ATOM | 820 | CE2 | TYR | A | 146 | 6.702 | -13.753 | -12.314 | 1.00 | 40.96 | A | C |
| ATOM | 821 | CZ | TYR | A | 146 | 6.920 | -13.496 | -10.966 | 1.00 | 40.96 | A | C |
| ATOM | 822 | OH | TYR | A | 146 | 6.696 | -14.456 | -10.001 | 1.00 | 40.96 | A | O |
| ATOM | 823 | C | TYR | A | 146 | 9.526 | -9.408 | -15.360 | 1.00 | 42.32 | A | C |
| ATOM | 824 | O | TYR | A | 146 | 9.679 | -9.903 | -16.475 | 1.00 | 42.32 | A | O |
| ATOM | 825 | N | SER | A | 147 | 9.507 | -8.099 | -15.151 | 1.00 | 43.19 | A | N |
| ATOM | 826 | CA | SER | A | 147 | 9.628 | -7.146 | -16.247 | 1.00 | 43.19 | A | C |
| ATOM | 827 | CB | SER | A | 147 | 9.360 | -5.738 | -15.728 | 1.00 | 50.78 | A | C |
| ATOM | 828 | OG | SER | A | 147 | 9.376 | -4.809 | -16.789 | 1.00 | 50.78 | A | O |
| ATOM | 829 | C | SER | A | 147 | 10.965 | -7.180 | -16.991 | 1.00 | 43.19 | A | C |
| ATOM | 830 | O | SER | A | 147 | 10.996 | -7.097 | -18.220 | 1.00 | 43.19 | A | O |
| ATOM | 831 | N | ARG | A | 148 | 12.069 | -7.292 | -16.257 | 1.00 | 51.06 | A | N |
| ATOM | 832 | CA | ARG | A | 148 | 13.380 | -7.344 | -16.897 | 1.00 | 51.06 | A | C |
| ATOM | 833 | CB | ARG | A | 148 | 14.497 | -7.243 | -15.866 | 1.00 | 85.14 | A | C |
| ATOM | 834 | CG | ARG | A | 148 | 14.869 | -5.836 | -15.464 | 1.00 | 85.14 | A | C |
| ATOM | 835 | CD | ARG | A | 148 | 15.915 | -5.920 | -14.373 | 1.00 | 85.14 | A | C |
| ATOM | 836 | NE | ARG | A | 148 | 16.284 | -4.615 | -13.839 | 1.00 | 85.14 | A | N |
| ATOM | 837 | CZ | ARG | A | 148 | 16.873 | -4.451 | -12.658 | 1.00 | 85.14 | A | C |
| ATOM | 838 | NH1 | ARG | A | 148 | 17.146 | -5.521 | -11.903 | 1.00 | 85.14 | A | N |
| ATOM | 839 | NH2 | ARG | A | 148 | 17.180 | -3.224 | -12.230 | 1.00 | 85.14 | A | N |
| ATOM | 840 | C | ARG | A | 148 | 13.569 | -8.638 | -17.671 | 1.00 | 51.06 | A | C |
| ATOM | 841 | O | ARG | A | 148 | 14.307 | -8.676 | -18.655 | 1.00 | 51.06 | A | O |
| ATOM | 842 | N | ALA | A | 149 | 12.915 | -9.699 | -17.210 | 1.00 | 62.19 | A | N |
| ATOM | 843 | CA | ALA | A | 149 | 13.022 | -10.999 | -17.863 | 1.00 | 62.19 | A | C |
| ATOM | 844 | CB | ALA | A | 149 | 12.847 | -12.136 | -16.838 | 1.00 | 32.38 | A | C |
| ATOM | 845 | C | ALA | A | 149 | 11.967 | -11.114 | -18.945 | 1.00 | 62.19 | A | C |
| ATOM | 846 | O | ALA | A | 149 | 11.568 | -12.222 | -19.310 | 1.00 | 62.19 | A | O |
| ATOM | 847 | N | LYS | A | 150 | 11.501 | -9.971 | -19.442 | 1.00 | 59.65 | A | N |
| ATOM | 848 | CA | LYS | A | 150 | 10.490 | -9.958 | -20.493 | 1.00 | 59.65 | A | C |
| ATOM | 849 | CB | LYS | A | 150 | 11.161 | -10.289 | -21.832 | 1.00 | 78.22 | A | C |
| ATOM | 850 | CG | LYS | A | 150 | 10.562 | -9.568 | -23.034 | 1.00 | 78.22 | A | C |
| ATOM | 851 | CD | LYS | A | 150 | 11.065 | -8.113 | -23.180 | 1.00 | 78.22 | A | C |
| ATOM | 852 | CE | LYS | A | 150 | 12.516 | -8.055 | -23.676 | 1.00 | 78.22 | A | C |
| ATOM | 853 | NZ | LYS | A | 150 | 12.893 | -6.718 | -24.247 | 1.00 | 78.22 | A | N |
| ATOM | 854 | C | LYS | A | 150 | 9.408 | -11.003 | -20.161 | 1.00 | 59.65 | A | C |
| ATOM | 855 | O | LYS | A | 150 | 9.060 | -11.831 | -20.994 | 1.00 | 59.65 | A | O |
| ATOM | 856 | N | GLN | A | 151 | 8.882 | -10.950 | -18.938 | 1.00 | 71.57 | A | N |
| ATOM | 857 | CA | GLN | A | 151 | 7.871 | -11.907 | -18.457 | 1.00 | 71.57 | A | C |
| ATOM | 858 | CB | GLN | A | 151 | 8.567 | -12.892 | -17.502 | 1.00 | 78.09 | A | C |
| ATOM | 859 | CG | GLN | A | 151 | 7.714 | -14.020 | -16.956 | 1.00 | 78.09 | A | C |
| ATOM | 860 | CD | GLN | A | 151 | 7.953 | -15.323 | -17.698 | 1.00 | 78.09 | A | C |
| ATOM | 861 | OE1 | GLN | A | 151 | 9.090 | -15.802 | -17.790 | 1.00 | 78.09 | A | O |
| ATOM | 862 | NE2 | GLN | A | 151 | 6.882 | -15.907 | -18.235 | 1.00 | 78.09 | A | N |
| ATOM | 863 | C | GLN | A | 151 | 6.705 | -11.192 | -17.726 | 1.00 | 71.57 | A | C |
| ATOM | 864 | O | GLN | A | 151 | 6.893 | -10.124 | -17.133 | 1.00 | 71.57 | A | O |

| | | | | | | | | | | | | |
|---|---|---|---|---|---|---|---|---|---|---|---|---|
| ATOM | 865 | N | THR | A | 152 | 5.507 | -11.772 | -17.756 | 1.00 | 64.30 | A | N |
| ATOM | 866 | CA | THR | A | 152 | 4.373 | -11.135 | -17.087 | 1.00 | 64.30 | A | C |
| ATOM | 867 | CB | THR | A | 152 | 3.086 | -11.154 | -17.980 | 1.00 | 53.56 | A | C |
| ATOM | 868 | OG1 | THR | A | 152 | 2.052 | -11.913 | -17.333 | 1.00 | 53.56 | A | O |
| ATOM | 869 | CG2 | THR | A | 152 | 3.383 | -11.769 | -19.354 | 1.00 | 53.56 | A | C |
| ATOM | 870 | C | THR | A | 152 | 4.048 | -11.801 | -15.751 | 1.00 | 64.30 | A | C |
| ATOM | 871 | O | THR | A | 152 | 4.017 | -13.029 | -15.660 | 1.00 | 64.30 | A | O |
| ATOM | 872 | N | LEU | A | 153 | 3.810 | -10.997 | -14.713 | 1.00 | 35.15 | A | N |
| ATOM | 873 | CA | LEU | A | 153 | 3.464 | -11.528 | -13.395 | 1.00 | 35.15 | A | C |
| ATOM | 874 | CB | LEU | A | 153 | 3.316 | -10.384 | -12.392 | 1.00 | 38.44 | A | C |
| ATOM | 875 | CG | LEU | A | 153 | 2.885 | -10.753 | -10.960 | 1.00 | 38.44 | A | C |
| ATOM | 876 | CD1 | LEU | A | 153 | 4.079 | -11.295 | -10.193 | 1.00 | 38.44 | A | C |
| ATOM | 877 | CD2 | LEU | A | 153 | 2.314 | -9.535 | -10.247 | 1.00 | 38.44 | A | C |
| ATOM | 878 | C | LEU | A | 153 | 2.145 | -12.305 | -13.450 | 1.00 | 35.15 | A | C |
| ATOM | 879 | O | LEU | A | 153 | 1.148 | -11.809 | -13.953 | 1.00 | 35.15 | A | O |
| ATOM | 880 | N | PRO | A | 154 | 2.125 | -13.536 | -12.930 | 1.00 | 37.31 | A | N |
| ATOM | 881 | CD | PRO | A | 154 | 3.249 | -14.323 | -12.390 | 1.00 | 29.59 | A | C |
| ATOM | 882 | CA | PRO | A | 154 | 0.887 | -14.325 | -12.949 | 1.00 | 37.31 | A | C |
| ATOM | 883 | CB | PRO | A | 154 | 1.276 | -15.573 | -12.156 | 1.00 | 29.59 | A | C |
| ATOM | 884 | CG | PRO | A | 154 | 2.728 | -15.732 | -12.476 | 1.00 | 29.59 | A | C |
| ATOM | 885 | C | PRO | A | 154 | -0.303 | -13.574 | -12.318 | 1.00 | 37.31 | A | C |
| ATOM | 886 | O | PRO | A | 154 | -0.179 | -13.015 | -11.225 | 1.00 | 37.31 | A | O |
| ATOM | 887 | N | VAL | A | 155 | -1.456 | -13.579 | -12.989 | 1.00 | 49.63 | A | N |
| ATOM | 888 | CA | VAL | A | 155 | -2.638 | -12.881 | -12.475 | 1.00 | 49.63 | A | C |
| ATOM | 889 | CB | VAL | A | 155 | -3.831 | -12.903 | -13.475 | 1.00 | 32.71 | A | C |
| ATOM | 890 | CG1 | VAL | A | 155 | -3.450 | -12.203 | -14.751 | 1.00 | 32.71 | A | C |
| ATOM | 891 | CG2 | VAL | A | 155 | -4.254 | -14.321 | -13.759 | 1.00 | 32.71 | A | C |
| ATOM | 892 | C | VAL | A | 155 | -3.146 | -13.384 | -11.129 | 1.00 | 49.63 | A | C |
| ATOM | 893 | O | VAL | A | 155 | -4.083 | -12.814 | -10.580 | 1.00 | 49.63 | A | O |
| ATOM | 894 | N | ILE | A | 156 | -2.561 | -14.448 | -10.587 | 1.00 | 41.28 | A | N |
| ATOM | 895 | CA | ILE | A | 156 | -3.018 | -14.911 | -9.279 | 1.00 | 41.28 | A | C |
| ATOM | 896 | CB | ILE | A | 156 | -2.663 | -16.406 | -9.002 | 1.00 | 33.71 | A | C |
| ATOM | 897 | CG2 | ILE | A | 156 | -1.171 | -16.620 | -9.039 | 1.00 | 33.71 | A | C |
| ATOM | 898 | CG1 | ILE | A | 156 | -3.201 | -16.824 | -7.629 | 1.00 | 33.71 | A | C |
| ATOM | 899 | CD1 | ILE | A | 156 | -4.706 | -16.759 | -7.518 | 1.00 | 33.71 | A | C |
| ATOM | 900 | C | ILE | A | 156 | -2.332 | -14.021 | -8.258 | 1.00 | 41.28 | A | C |
| ATOM | 901 | O | ILE | A | 156 | -2.868 | -13.755 | -7.190 | 1.00 | 41.28 | A | O |
| ATOM | 902 | N | TYR | A | 157 | -1.137 | -13.553 | -8.594 | 1.00 | 40.46 | A | N |
| ATOM | 903 | CA | TYR | A | 157 | -0.419 | -12.665 | -7.692 | 1.00 | 40.46 | A | C |
| ATOM | 904 | CB | TYR | A | 157 | 1.069 | -12.649 | -8.037 | 1.00 | 49.51 | A | C |
| ATOM | 905 | CG | TYR | A | 157 | 1.797 | -13.887 | -7.577 | 1.00 | 49.51 | A | C |
| ATOM | 906 | CD1 | TYR | A | 157 | 2.613 | -14.603 | -8.444 | 1.00 | 49.51 | A | C |
| ATOM | 907 | CE1 | TYR | A | 157 | 3.272 | -15.754 | -8.029 | 1.00 | 49.51 | A | C |
| ATOM | 908 | CD2 | TYR | A | 157 | 1.657 | -14.350 | -6.279 | 1.00 | 49.51 | A | C |
| ATOM | 909 | CE2 | TYR | A | 157 | 2.308 | -15.499 | -5.851 | 1.00 | 49.51 | A | C |
| ATOM | 910 | CZ | TYR | A | 157 | 3.116 | -16.199 | -6.732 | 1.00 | 49.51 | A | C |
| ATOM | 911 | OH | TYR | A | 157 | 3.767 | -17.343 | -6.319 | 1.00 | 49.51 | A | O |
| ATOM | 912 | C | TYR | A | 157 | -1.015 | -11.266 | -7.825 | 1.00 | 40.46 | A | C |
| ATOM | 913 | O | TYR | A | 157 | -1.081 | -10.505 | -6.859 | 1.00 | 40.46 | A | O |
| ATOM | 914 | N | VAL | A | 158 | -1.467 | -10.948 | -9.035 | 1.00 | 42.41 | A | N |
| ATOM | 915 | CA | VAL | A | 158 | -2.067 | -9.661 | -9.302 | 1.00 | 42.41 | A | C |
| ATOM | 916 | CB | VAL | A | 158 | -2.373 | -9.497 | -10.791 | 1.00 | 32.15 | A | C |
| ATOM | 917 | CG1 | VAL | A | 158 | -2.986 | -8.129 | -11.039 | 1.00 | 32.15 | A | C |
| ATOM | 918 | CG2 | VAL | A | 158 | -1.100 | -9.649 | -11.603 | 1.00 | 32.15 | A | C |
| ATOM | 919 | C | VAL | A | 158 | -3.353 | -9.550 | -8.501 | 1.00 | 42.41 | A | C |
| ATOM | 920 | O | VAL | A | 158 | -3.668 | -8.493 | -7.951 | 1.00 | 42.41 | A | O |
| ATOM | 921 | N | LYS | A | 159 | -4.095 | -10.643 | -8.421 | 1.00 | 37.13 | A | N |
| ATOM | 922 | CA | LYS | A | 159 | -5.333 | -10.624 | -7.668 | 1.00 | 37.13 | A | C |
| ATOM | 923 | CB | LYS | A | 159 | -6.124 | -11.900 | -7.917 | 1.00 | 38.32 | A | C |
| ATOM | 924 | CG | LYS | A | 159 | -6.633 | -12.044 | -9.323 | 1.00 | 38.32 | A | C |
| ATOM | 925 | CD | LYS | A | 159 | -7.265 | -13.396 | -9.505 | 1.00 | 38.32 | A | C |
| ATOM | 926 | CE | LYS | A | 159 | -7.783 | -13.591 | -10.915 | 1.00 | 38.32 | A | C |
| ATOM | 927 | NZ | LYS | A | 159 | -8.612 | -14.825 | -10.988 | 1.00 | 38.32 | A | N |
| ATOM | 928 | C | LYS | A | 159 | -5.038 | -10.495 | -6.187 | 1.00 | 37.13 | A | C |
| ATOM | 929 | O | LYS | A | 159 | -5.659 | -9.700 | -5.483 | 1.00 | 37.13 | A | O |
| ATOM | 930 | N | LEU | A | 160 | -4.081 | -11.286 | -5.720 | 1.00 | 51.24 | A | N |
| ATOM | 931 | CA | LEU | A | 160 | -3.691 | -11.283 | -4.316 | 1.00 | 51.24 | A | C |

| | | | | | | | | | | | | |
|---|---|---|---|---|---|---|---|---|---|---|---|---|
| ATOM | 932 | CB | LEU | A | 160 | -2.615 | -12.333 | -4.069 | 1.00 | 56.41 | A | C |
| ATOM | 933 | CG | LEU | A | 160 | -3.181 | -13.736 | -3.894 | 1.00 | 56.41 | A | C |
| ATOM | 934 | CD1 | LEU | A | 160 | -2.064 | -14.752 | -3.955 | 1.00 | 56.41 | A | C |
| ATOM | 935 | CD2 | LEU | A | 160 | -3.929 | -13.805 | -2.560 | 1.00 | 56.41 | A | C |
| ATOM | 936 | C | LEU | A | 160 | -3.197 | -9.947 | -3.804 | 1.00 | 51.24 | A | C |
| ATOM | 937 | O | LEU | A | 160 | -3.648 | -9.487 | -2.756 | 1.00 | 51.24 | A | O |
| ATOM | 938 | N | TYR | A | 161 | -2.272 | -9.327 | -4.534 | 1.00 | 38.52 | A | N |
| ATOM | 939 | CA | TYR | A | 161 | -1.726 | -8.046 | -4.108 | 1.00 | 38.52 | A | C |
| ATOM | 940 | CB | TYR | A | 161 | -0.538 | -7.659 | -4.970 | 1.00 | 38.67 | A | C |
| ATOM | 941 | CG | TYR | A | 161 | 0.543 | -8.707 | -4.953 | 1.00 | 38.67 | A | C |
| ATOM | 942 | CD1 | TYR | A | 161 | 0.586 | -9.664 | -3.945 | 1.00 | 38.67 | A | C |
| ATOM | 943 | CE1 | TYR | A | 161 | 1.573 | -10.624 | -3.911 | 1.00 | 38.67 | A | C |
| ATOM | 944 | CD2 | TYR | A | 161 | 1.525 | -8.742 | -5.935 | 1.00 | 38.67 | A | C |
| ATOM | 945 | CE2 | TYR | A | 161 | 2.521 | -9.700 | -5.907 | 1.00 | 38.67 | A | C |
| ATOM | 946 | CZ | TYR | A | 161 | 2.536 | -10.635 | -4.890 | 1.00 | 38.67 | A | C |
| ATOM | 947 | OH | TYR | A | 161 | 3.533 | -11.570 | -4.830 | 1.00 | 38.67 | A | O |
| ATOM | 948 | C | TYR | A | 161 | -2.748 | -6.946 | -4.135 | 1.00 | 38.52 | A | C |
| ATOM | 949 | O | TYR | A | 161 | -2.976 | -6.281 | -3.120 | 1.00 | 38.52 | A | O |
| ATOM | 950 | N | MET | A | 162 | -3.374 | -6.754 | -5.292 | 1.00 | 48.46 | A | N |
| ATOM | 951 | CA | MET | A | 162 | -4.378 | -5.713 | -5.416 | 1.00 | 48.46 | A | C |
| ATOM | 952 | CB | MET | A | 162 | -4.992 | -5.734 | -6.806 | 1.00 | 36.74 | A | C |
| ATOM | 953 | CG | MET | A | 162 | -4.012 | -5.353 | -7.887 | 1.00 | 36.74 | A | C |
| ATOM | 954 | SD | MET | A | 162 | -3.125 | -3.849 | -7.477 | 1.00 | 36.74 | A | S |
| ATOM | 955 | CE | MET | A | 162 | -4.456 | -2.702 | -7.311 | 1.00 | 36.74 | A | C |
| ATOM | 956 | C | MET | A | 162 | -5.454 | -5.860 | -4.356 | 1.00 | 48.46 | A | C |
| ATOM | 957 | O | MET | A | 162 | -5.816 | -4.883 | -3.709 | 1.00 | 48.46 | A | O |
| ATOM | 958 | N | TYR | A | 163 | -5.946 | -7.079 | -4.158 | 1.00 | 29.09 | A | N |
| ATOM | 959 | CA | TYR | A | 163 | -6.984 | -7.315 | -3.163 | 1.00 | 29.09 | A | C |
| ATOM | 960 | CB | TYR | A | 163 | -7.328 | -8.794 | -3.078 | 1.00 | 36.43 | A | C |
| ATOM | 961 | CG | TYR | A | 163 | -8.406 | -9.074 | -2.060 | 1.00 | 36.43 | A | C |
| ATOM | 962 | CD1 | TYR | A | 163 | -9.751 | -8.942 | -2.386 | 1.00 | 36.43 | A | C |
| ATOM | 963 | CE1 | TYR | A | 163 | -10.744 | -9.185 | -1.448 | 1.00 | 36.43 | A | C |
| ATOM | 964 | CD2 | TYR | A | 163 | -8.080 | -9.451 | -0.765 | 1.00 | 36.43 | A | C |
| ATOM | 965 | CE2 | TYR | A | 163 | -9.065 | -9.695 | 0.179 | 1.00 | 36.43 | A | C |
| ATOM | 966 | CZ | TYR | A | 163 | -10.394 | -9.563 | -0.172 | 1.00 | 36.43 | A | C |
| ATOM | 967 | OH | TYR | A | 163 | -11.370 | -9.844 | 0.755 | 1.00 | 36.43 | A | O |
| ATOM | 968 | C | TYR | A | 163 | -6.570 | -6.846 | -1.776 | 1.00 | 29.09 | A | C |
| ATOM | 969 | O | TYR | A | 163 | -7.342 | -6.205 | -1.063 | 1.00 | 29.09 | A | O |
| ATOM | 970 | N | GLN | A | 164 | -5.350 | -7.193 | -1.388 | 1.00 | 56.52 | A | N |
| ATOM | 971 | CA | GLN | A | 164 | -4.844 | -6.804 | -0.085 | 1.00 | 56.52 | A | C |
| ATOM | 972 | CB | GLN | A | 164 | -3.560 | -7.572 | 0.227 | 1.00 | 41.43 | A | C |
| ATOM | 973 | CG | GLN | A | 164 | -3.760 | -9.086 | 0.310 | 1.00 | 41.43 | A | C |
| ATOM | 974 | CD | GLN | A | 164 | -2.489 | -9.826 | 0.718 | 1.00 | 41.43 | A | C |
| ATOM | 975 | OE1 | GLN | A | 164 | -2.152 | -9.912 | 1.904 | 1.00 | 41.43 | A | O |
| ATOM | 976 | NE2 | GLN | A | 164 | -1.769 | -10.351 | -0.271 | 1.00 | 41.43 | A | N |
| ATOM | 977 | C | GLN | A | 164 | -4.609 | -5.295 | -0.034 | 1.00 | 56.52 | A | C |
| ATOM | 978 | O | GLN | A | 164 | -4.858 | -4.661 | 0.993 | 1.00 | 56.52 | A | O |
| ATOM | 979 | N | LEU | A | 165 | -4.138 | -4.713 | -1.135 | 1.00 | 37.22 | A | N |
| ATOM | 980 | CA | LEU | A | 165 | -3.912 | -3.280 | -1.156 | 1.00 | 37.22 | A | C |
| ATOM | 981 | CB | LEU | A | 165 | -3.276 | -2.840 | -2.473 | 1.00 | 29.40 | A | C |
| ATOM | 982 | CG | LEU | A | 165 | -3.487 | -1.365 | -2.847 | 1.00 | 29.40 | A | C |
| ATOM | 983 | CD1 | LEU | A | 165 | -2.999 | -0.465 | -1.749 | 1.00 | 29.40 | A | C |
| ATOM | 984 | CD2 | LEU | A | 165 | -2.771 | -1.056 | -4.132 | 1.00 | 29.40 | A | C |
| ATOM | 985 | C | LEU | A | 165 | -5.253 | -2.592 | -0.988 | 1.00 | 37.22 | A | C |
| ATOM | 986 | O | LEU | A | 165 | -5.379 | -1.624 | -0.230 | 1.00 | 37.22 | A | O |
| ATOM | 987 | N | PHE | A | 166 | -6.261 | -3.090 | -1.697 | 1.00 | 35.56 | A | N |
| ATOM | 988 | CA | PHE | A | 166 | -7.584 | -2.499 | -1.601 | 1.00 | 35.56 | A | C |
| ATOM | 989 | CB | PHE | A | 166 | -8.543 | -3.113 | -2.622 | 1.00 | 36.93 | A | C |
| ATOM | 990 | CG | PHE | A | 166 | -8.506 | -2.429 | -3.961 | 1.00 | 36.93 | A | C |
| ATOM | 991 | CD1 | PHE | A | 166 | -8.621 | -1.037 | -4.049 | 1.00 | 36.93 | A | C |
| ATOM | 992 | CD2 | PHE | A | 166 | -8.327 | -3.158 | -5.128 | 1.00 | 36.93 | A | C |
| ATOM | 993 | CE1 | PHE | A | 166 | -8.551 | -0.387 | -5.278 | 1.00 | 36.93 | A | C |
| ATOM | 994 | CE2 | PHE | A | 166 | -8.256 | -2.516 | -6.353 | 1.00 | 36.93 | A | C |
| ATOM | 995 | CZ | PHE | A | 166 | -8.368 | -1.124 | -6.429 | 1.00 | 36.93 | A | C |
| ATOM | 996 | C | PHE | A | 166 | -8.152 | -2.656 | -0.212 | 1.00 | 35.56 | A | C |
| ATOM | 997 | O | PHE | A | 166 | -8.824 | -1.767 | 0.298 | 1.00 | 35.56 | A | O |
| ATOM | 998 | N | ARG | A | 167 | -7.873 | -3.781 | 0.417 | 1.00 | 34.84 | A | N |

| | | | | | | | | | | | | |
|---|---|---|---|---|---|---|---|---|---|---|---|---|
| ATOM | 999 | CA | ARG | A | 167 | -8.401 | -3.987 | 1.743 | 1.00 | 34.84 | A | C |
| ATOM | 1000 | CB | ARG | A | 167 | -8.151 | -5.429 | 2.204 | 1.00 | 46.61 | A | C |
| ATOM | 1001 | CG | ARG | A | 167 | -8.954 | -5.769 | 3.436 | 1.00 | 46.61 | A | C |
| ATOM | 1002 | CD | ARG | A | 167 | -8.740 | -7.161 | 3.939 | 1.00 | 46.61 | A | C |
| ATOM | 1003 | NE | ARG | A | 167 | -9.236 | -7.239 | 5.307 | 1.00 | 46.61 | A | N |
| ATOM | 1004 | CZ | ARG | A | 167 | -9.121 | -8.305 | 6.088 | 1.00 | 46.61 | A | C |
| ATOM | 1005 | NH1 | ARG | A | 167 | -8.525 | -9.398 | 5.633 | 1.00 | 46.61 | A | N |
| ATOM | 1006 | NH2 | ARG | A | 167 | -9.585 | -8.270 | 7.329 | 1.00 | 46.61 | A | N |
| ATOM | 1007 | C | ARG | A | 167 | -7.787 | -2.992 | 2.724 | 1.00 | 34.84 | A | C |
| ATOM | 1008 | O | ARG | A | 167 | -8.486 | -2.447 | 3.572 | 1.00 | 34.84 | A | O |
| ATOM | 1009 | N | SER | A | 168 | -6.486 | -2.743 | 2.590 | 1.00 | 42.21 | A | N |
| ATOM | 1010 | CA | SER | A | 168 | -5.780 | -1.819 | 3.477 | 1.00 | 42.21 | A | C |
| ATOM | 1011 | CB | SER | A | 168 | -4.273 | -1.866 | 3.217 | 1.00 | 36.53 | A | C |
| ATOM | 1012 | OG | SER | A | 168 | -3.933 | -1.125 | 2.062 | 1.00 | 36.53 | A | O |
| ATOM | 1013 | C | SER | A | 168 | -6.271 | -0.385 | 3.303 | 1.00 | 42.21 | A | C |
| ATOM | 1014 | O | SER | A | 168 | -6.373 | 0.376 | 4.265 | 1.00 | 42.21 | A | O |
| ATOM | 1015 | N | LEU | A | 169 | -6.556 | -0.016 | 2.064 | 1.00 | 40.57 | A | N |
| ATOM | 1016 | CA | LEU | A | 169 | -7.049 | 1.318 | 1.775 | 1.00 | 40.57 | A | C |
| ATOM | 1017 | CB | LEU | A | 169 | -7.142 | 1.516 | 0.262 | 1.00 | 32.97 | A | C |
| ATOM | 1018 | CG | LEU | A | 169 | -6.143 | 2.495 | -0.354 | 1.00 | 32.97 | A | C |
| ATOM | 1019 | CD1 | LEU | A | 169 | -4.790 | 2.397 | 0.332 | 1.00 | 32.97 | A | C |
| ATOM | 1020 | CD2 | LEU | A | 169 | -6.031 | 2.206 | -1.835 | 1.00 | 32.97 | A | C |
| ATOM | 1021 | C | LEU | A | 169 | -8.422 | 1.468 | 2.416 | 1.00 | 40.57 | A | C |
| ATOM | 1022 | O | LEU | A | 169 | -8.727 | 2.480 | 3.038 | 1.00 | 40.57 | A | O |
| ATOM | 1023 | N | ALA | A | 170 | -9.244 | 0.442 | 2.266 | 1.00 | 31.29 | A | N |
| ATOM | 1024 | CA | ALA | A | 170 | -10.568 | 0.455 | 2.832 | 1.00 | 31.29 | A | C |
| ATOM | 1025 | CB | ALA | A | 170 | -11.227 | -0.878 | 2.600 | 1.00 | 19.73 | A | C |
| ATOM | 1026 | C | ALA | A | 170 | -10.443 | 0.727 | 4.321 | 1.00 | 31.29 | A | C |
| ATOM | 1027 | O | ALA | A | 170 | -11.265 | 1.424 | 4.924 | 1.00 | 31.29 | A | O |
| ATOM | 1028 | N | TYR | A | 171 | -9.392 | 0.179 | 4.911 | 1.00 | 39.04 | A | N |
| ATOM | 1029 | CA | TYR | A | 171 | -9.157 | 0.346 | 6.332 | 1.00 | 39.04 | A | C |
| ATOM | 1030 | CB | TYR | A | 171 | -8.028 | -0.569 | 6.780 | 1.00 | 41.33 | A | C |
| ATOM | 1031 | CG | TYR | A | 171 | -7.711 | -0.411 | 8.240 | 1.00 | 41.33 | A | C |
| ATOM | 1032 | CD1 | TYR | A | 171 | -8.648 | -0.751 | 9.211 | 1.00 | 41.33 | A | C |
| ATOM | 1033 | CE1 | TYR | A | 171 | -8.376 | -0.590 | 10.554 | 1.00 | 41.33 | A | C |
| ATOM | 1034 | CD2 | TYR | A | 171 | -6.484 | 0.098 | 8.653 | 1.00 | 41.33 | A | C |
| ATOM | 1035 | CE2 | TYR | A | 171 | -6.202 | 0.266 | 10.001 | 1.00 | 41.33 | A | C |
| ATOM | 1036 | CZ | TYR | A | 171 | -7.154 | -0.082 | 10.944 | 1.00 | 41.33 | A | C |
| ATOM | 1037 | OH | TYR | A | 171 | -6.884 | 0.074 | 12.279 | 1.00 | 41.33 | A | O |
| ATOM | 1038 | C | TYR | A | 171 | -8.827 | 1.777 | 6.739 | 1.00 | 39.04 | A | C |
| ATOM | 1039 | O | TYR | A | 171 | -9.589 | 2.424 | 7.457 | 1.00 | 39.04 | A | O |
| ATOM | 1040 | N | ILE | A | 172 | -7.681 | 2.270 | 6.291 | 1.00 | 42.22 | A | N |
| ATOM | 1041 | CA | ILE | A | 172 | -7.290 | 3.619 | 6.649 | 1.00 | 42.22 | A | C |
| ATOM | 1042 | CB | ILE | A | 172 | -5.914 | 3.994 | 6.065 | 1.00 | 42.03 | A | C |
| ATOM | 1043 | CG2 | ILE | A | 172 | -4.845 | 3.083 | 6.640 | 1.00 | 42.03 | A | C |
| ATOM | 1044 | CG1 | ILE | A | 172 | -5.953 | 3.920 | 4.544 | 1.00 | 42.03 | A | C |
| ATOM | 1045 | CD1 | ILE | A | 172 | -4.671 | 4.377 | 3.884 | 1.00 | 42.03 | A | C |
| ATOM | 1046 | C | ILE | A | 172 | -8.320 | 4.639 | 6.190 | 1.00 | 42.22 | A | C |
| ATOM | 1047 | O | ILE | A | 172 | -8.505 | 5.682 | 6.822 | 1.00 | 42.22 | A | O |
| ATOM | 1048 | N | HIS | A | 173 | -9.004 | 4.345 | 5.097 | 1.00 | 42.90 | A | N |
| ATOM | 1049 | CA | HIS | A | 173 | -9.995 | 5.282 | 4.615 | 1.00 | 42.90 | A | C |
| ATOM | 1050 | CB | HIS | A | 173 | -10.468 | 4.900 | 3.219 | 1.00 | 36.33 | A | C |
| ATOM | 1051 | CG | HIS | A | 173 | -9.500 | 5.275 | 2.142 | 1.00 | 36.33 | A | C |
| ATOM | 1052 | CD2 | HIS | A | 173 | -8.254 | 5.802 | 2.213 | 1.00 | 36.33 | A | C |
| ATOM | 1053 | ND1 | HIS | A | 173 | -9.782 | 5.139 | 0.801 | 1.00 | 36.33 | A | N |
| ATOM | 1054 | CE1 | HIS | A | 173 | -8.754 | 5.570 | 0.093 | 1.00 | 36.33 | A | C |
| ATOM | 1055 | NE2 | HIS | A | 173 | -7.814 | 5.978 | 0.925 | 1.00 | 36.33 | A | N |
| ATOM | 1056 | C | HIS | A | 173 | -11.176 | 5.435 | 5.546 | 1.00 | 42.90 | A | C |
| ATOM | 1057 | O | HIS | A | 173 | -11.833 | 6.474 | 5.522 | 1.00 | 42.90 | A | O |
| ATOM | 1058 | N | SER | A | 174 | -11.442 | 4.430 | 6.381 | 1.00 | 46.63 | A | N |
| ATOM | 1059 | CA | SER | A | 174 | -12.573 | 4.524 | 7.312 | 1.00 | 46.63 | A | C |
| ATOM | 1060 | CB | SER | A | 174 | -12.979 | 3.144 | 7.840 | 1.00 | 38.44 | A | C |
| ATOM | 1061 | OG | SER | A | 174 | -11.960 | 2.600 | 8.643 | 1.00 | 38.44 | A | O |
| ATOM | 1062 | C | SER | A | 174 | -12.315 | 5.476 | 8.490 | 1.00 | 46.63 | A | C |
| ATOM | 1063 | O | SER | A | 174 | -13.233 | 5.814 | 9.231 | 1.00 | 46.63 | A | O |
| ATOM | 1064 | N | PHE | A | 175 | -11.072 | 5.907 | 8.667 | 1.00 | 39.22 | A | N |
| ATOM | 1065 | CA | PHE | A | 175 | -10.757 | 6.852 | 9.732 | 1.00 | 39.22 | A | C |

| | | | | | | | | | | | | |
|---|---|---|---|---|---|---|---|---|---|---|---|---|
| ATOM | 1066 | CB | PHE | A | 175 | -9.480 | 6.463 | 10.471 | 1.00 | 77.58 | A | C |
| ATOM | 1067 | CG | PHE | A | 175 | -9.554 | 5.140 | 11.162 | 1.00 | 77.58 | A | C |
| ATOM | 1068 | CD1 | PHE | A | 175 | -9.267 | 3.960 | 10.475 | 1.00 | 77.58 | A | C |
| ATOM | 1069 | CD2 | PHE | A | 175 | -9.919 | 5.070 | 12.505 | 1.00 | 77.58 | A | C |
| ATOM | 1070 | CE1 | PHE | A | 175 | -9.341 | 2.719 | 11.115 | 1.00 | 77.58 | A | C |
| ATOM | 1071 | CE2 | PHE | A | 175 | -9.997 | 3.837 | 13.161 | 1.00 | 77.58 | A | C |
| ATOM | 1072 | CZ | PHE | A | 175 | -9.706 | 2.653 | 12.462 | 1.00 | 77.58 | A | C |
| ATOM | 1073 | C | PHE | A | 175 | -10.523 | 8.197 | 9.069 | 1.00 | 39.22 | A | C |
| ATOM | 1074 | O | PHE | A | 175 | -10.014 | 9.131 | 9.693 | 1.00 | 39.22 | A | O |
| ATOM | 1075 | N | GLY | A | 176 | -10.872 | 8.283 | 7.790 | 1.00 | 40.56 | A | N |
| ATOM | 1076 | CA | GLY | A | 176 | -10.673 | 9.516 | 7.055 | 1.00 | 40.56 | A | C |
| ATOM | 1077 | C | GLY | A | 176 | -9.203 | 9.785 | 6.771 | 1.00 | 40.56 | A | C |
| ATOM | 1078 | O | GLY | A | 176 | -8.782 | 10.932 | 6.603 | 1.00 | 40.56 | A | O |
| ATOM | 1079 | N | ILE | A | 177 | -8.413 | 8.720 | 6.713 | 1.00 | 39.95 | A | N |
| ATOM | 1080 | CA | ILE | A | 177 | -6.993 | 8.845 | 6.435 | 1.00 | 39.95 | A | C |
| ATOM | 1081 | CB | ILE | A | 177 | -6.193 | 7.970 | 7.382 | 1.00 | 48.78 | A | C |
| ATOM | 1082 | CG2 | ILE | A | 177 | -4.733 | 7.982 | 6.980 | 1.00 | 48.78 | A | C |
| ATOM | 1083 | CG1 | ILE | A | 177 | -6.386 | 8.468 | 8.809 | 1.00 | 48.78 | A | C |
| ATOM | 1084 | CD1 | ILE | A | 177 | -5.941 | 7.477 | 9.878 | 1.00 | 48.78 | A | C |
| ATOM | 1085 | C | ILE | A | 177 | -6.685 | 8.440 | 4.989 | 1.00 | 39.95 | A | C |
| ATOM | 1086 | O | ILE | A | 177 | -7.081 | 7.366 | 4.529 | 1.00 | 39.95 | A | O |
| ATOM | 1087 | N | CYS | A | 178 | -5.976 | 9.311 | 4.281 | 1.00 | 39.41 | A | N |
| ATOM | 1088 | CA | CYS | A | 178 | -5.614 | 9.080 | 2.890 | 1.00 | 39.41 | A | C |
| ATOM | 1089 | CB | CYS | A | 178 | -6.099 | 10.258 | 2.043 | 1.00 | 45.52 | A | C |
| ATOM | 1090 | SG | CYS | A | 178 | -5.709 | 10.168 | 0.284 | 1.00 | 45.52 | A | S |
| ATOM | 1091 | C | CYS | A | 178 | -4.100 | 8.916 | 2.748 | 1.00 | 39.41 | A | C |
| ATOM | 1092 | O | CYS | A | 178 | -3.327 | 9.803 | 3.115 | 1.00 | 39.41 | A | O |
| ATOM | 1093 | N | HIS | A | 179 | -3.679 | 7.784 | 2.200 | 1.00 | 35.87 | A | N |
| ATOM | 1094 | CA | HIS | A | 179 | -2.266 | 7.509 | 2.041 | 1.00 | 35.87 | A | C |
| ATOM | 1095 | CB | HIS | A | 179 | -2.082 | 6.120 | 1.438 | 1.00 | 33.74 | A | C |
| ATOM | 1096 | CG | HIS | A | 179 | -0.671 | 5.637 | 1.483 | 1.00 | 33.74 | A | C |
| ATOM | 1097 | CD2 | HIS | A | 179 | 0.000 | 4.940 | 2.430 | 1.00 | 33.74 | A | C |
| ATOM | 1098 | ND1 | HIS | A | 179 | 0.250 | 5.942 | 0.504 | 1.00 | 33.74 | A | N |
| ATOM | 1099 | CE1 | HIS | A | 179 | 1.429 | 5.455 | 0.848 | 1.00 | 33.74 | A | C |
| ATOM | 1100 | NE2 | HIS | A | 179 | 1.304 | 4.845 | 2.013 | 1.00 | 33.74 | A | N |
| ATOM | 1101 | C | HIS | A | 179 | -1.535 | 8.557 | 1.209 | 1.00 | 35.87 | A | C |
| ATOM | 1102 | O | HIS | A | 179 | -0.441 | 8.977 | 1.558 | 1.00 | 35.87 | A | O |
| ATOM | 1103 | N | ARG | A | 180 | -2.146 | 8.976 | 0.108 | 1.00 | 39.64 | A | N |
| ATOM | 1104 | CA | ARG | A | 180 | -1.568 | 9.984 | -0.782 | 1.00 | 39.64 | A | C |
| ATOM | 1105 | CB | ARG | A | 180 | -1.496 | 11.332 | -0.065 | 1.00 | 38.63 | A | C |
| ATOM | 1106 | CG | ARG | A | 180 | -2.829 | 11.842 | 0.413 | 1.00 | 38.63 | A | C |
| ATOM | 1107 | CD | ARG | A | 180 | -2.622 | 12.840 | 1.529 | 1.00 | 38.63 | A | C |
| ATOM | 1108 | NE | ARG | A | 180 | -2.061 | 14.098 | 1.051 | 1.00 | 38.63 | A | N |
| ATOM | 1109 | CZ | ARG | A | 180 | -1.520 | 15.023 | 1.838 | 1.00 | 38.63 | A | C |
| ATOM | 1110 | NH1 | ARG | A | 180 | -1.453 | 14.837 | 3.145 | 1.00 | 38.63 | A | N |
| ATOM | 1111 | NH2 | ARG | A | 180 | -1.061 | 16.147 | 1.313 | 1.00 | 38.63 | A | N |
| ATOM | 1112 | C | ARG | A | 180 | -0.193 | 9.650 | -1.383 | 1.00 | 39.64 | A | C |
| ATOM | 1113 | O | ARG | A | 180 | 0.480 | 10.522 | -1.939 | 1.00 | 39.64 | A | O |
| ATOM | 1114 | N | ASP | A | 181 | 0.232 | 8.398 | -1.263 | 1.00 | 37.76 | A | N |
| ATOM | 1115 | CA | ASP | A | 181 | 1.498 | 7.988 | -1.853 | 1.00 | 37.76 | A | C |
| ATOM | 1116 | CB | ASP | A | 181 | 2.653 | 8.334 | -0.924 | 1.00 | 48.71 | A | C |
| ATOM | 1117 | CG | ASP | A | 181 | 3.998 | 8.182 | -1.596 | 1.00 | 48.71 | A | C |
| ATOM | 1118 | OD1 | ASP | A | 181 | 4.032 | 8.154 | -2.843 | 1.00 | 48.71 | A | O |
| ATOM | 1119 | OD2 | ASP | A | 181 | 5.024 | 8.101 | -0.887 | 1.00 | 48.71 | A | O |
| ATOM | 1120 | C | ASP | A | 181 | 1.527 | 6.500 | -2.221 | 1.00 | 37.76 | A | C |
| ATOM | 1121 | O | ASP | A | 181 | 2.534 | 5.833 | -2.054 | 1.00 | 37.76 | A | O |
| ATOM | 1122 | N | ILE | A | 182 | 0.411 | 5.990 | -2.735 | 1.00 | 34.86 | A | N |
| ATOM | 1123 | CA | ILE | A | 182 | 0.316 | 4.595 | -3.140 | 1.00 | 34.86 | A | C |
| ATOM | 1124 | CB | ILE | A | 182 | -1.144 | 4.186 | -3.481 | 1.00 | 31.20 | A | C |
| ATOM | 1125 | CG2 | ILE | A | 182 | -1.204 | 2.725 | -3.894 | 1.00 | 31.20 | A | C |
| ATOM | 1126 | CG1 | ILE | A | 182 | -2.052 | 4.427 | -2.276 | 1.00 | 31.20 | A | C |
| ATOM | 1127 | CD1 | ILE | A | 182 | -1.708 | 3.602 | -1.066 | 1.00 | 31.20 | A | C |
| ATOM | 1128 | C | ILE | A | 182 | 1.155 | 4.376 | -4.384 | 1.00 | 34.86 | A | C |
| ATOM | 1129 | O | ILE | A | 182 | 0.944 | 5.038 | -5.403 | 1.00 | 34.86 | A | O |
| ATOM | 1130 | N | LYS | A | 183 | 2.109 | 3.450 | -4.281 | 1.00 | 32.20 | A | N |
| ATOM | 1131 | CA | LYS | A | 183 | 2.993 | 3.086 | -5.390 | 1.00 | 32.20 | A | C |
| ATOM | 1132 | CB | LYS | A | 183 | 4.092 | 4.140 | -5.599 | 1.00 | 49.53 | A | C |

| | | | | | | | | | | | | |
|---|---|---|---|---|---|---|---|---|---|---|---|---|
| ATOM | 1133 | CG | LYS | A | 183 | 5.009 | 4.368 | -4.408 | 1.00 | 49.53 | A | C |
| ATOM | 1134 | CD | LYS | A | 183 | 6.027 | 5.435 | -4.741 | 1.00 | 49.53 | A | C |
| ATOM | 1135 | CE | LYS | A | 183 | 6.850 | 5.839 | -3.536 | 1.00 | 49.53 | A | C |
| ATOM | 1136 | NZ | LYS | A | 183 | 7.683 | 7.040 | -3.835 | 1.00 | 49.53 | A | N |
| ATOM | 1137 | C | LYS | A | 183 | 3.616 | 1.729 | -5.095 | 1.00 | 32.20 | A | C |
| ATOM | 1138 | O | LYS | A | 183 | 3.693 | 1.316 | -3.943 | 1.00 | 32.20 | A | O |
| ATOM | 1139 | N | PRO | A | 184 | 4.056 | 1.013 | -6.139 | 1.00 | 36.52 | A | N |
| ATOM | 1140 | CD | PRO | A | 184 | 3.958 | 1.354 | -7.568 | 1.00 | 30.06 | A | C |
| ATOM | 1141 | CA | PRO | A | 184 | 4.667 | -0.303 | -5.973 | 1.00 | 36.52 | A | C |
| ATOM | 1142 | CB | PRO | A | 184 | 5.179 | -0.617 | -7.374 | 1.00 | 30.06 | A | C |
| ATOM | 1143 | CG | PRO | A | 184 | 4.141 | 0.003 | -8.231 | 1.00 | 30.06 | A | C |
| ATOM | 1144 | C | PRO | A | 184 | 5.770 | -0.333 | -4.925 | 1.00 | 36.52 | A | C |
| ATOM | 1145 | O | PRO | A | 184 | 5.898 | -1.305 | -4.177 | 1.00 | 36.52 | A | O |
| ATOM | 1146 | N | GLN | A | 185 | 6.552 | 0.739 | -4.863 | 1.00 | 37.17 | A | N |
| ATOM | 1147 | CA | GLN | A | 185 | 7.658 | 0.834 | -3.915 | 1.00 | 37.17 | A | C |
| ATOM | 1148 | CB | GLN | A | 185 | 8.503 | 2.078 | -4.208 | 1.00 | 39.30 | A | C |
| ATOM | 1149 | CG | GLN | A | 185 | 9.192 | 2.065 | -5.570 | 1.00 | 39.30 | A | C |
| ATOM | 1150 | CD | GLN | A | 185 | 8.235 | 2.338 | -6.744 | 1.00 | 39.30 | A | C |
| ATOM | 1151 | OE1 | GLN | A | 185 | 7.010 | 2.311 | -6.596 | 1.00 | 39.30 | A | O |
| ATOM | 1152 | NE2 | GLN | A | 185 | 8.805 | 2.597 | -7.917 | 1.00 | 39.30 | A | N |
| ATOM | 1153 | C | GLN | A | 185 | 7.224 | 0.858 | -2.451 | 1.00 | 37.17 | A | C |
| ATOM | 1154 | O | GLN | A | 185 | 8.046 | 0.652 | -1.560 | 1.00 | 37.17 | A | O |
| ATOM | 1155 | N | ASN | A | 186 | 5.943 | 1.111 | -2.198 | 1.00 | 34.70 | A | N |
| ATOM | 1156 | CA | ASN | A | 186 | 5.440 | 1.167 | -0.835 | 1.00 | 34.70 | A | C |
| ATOM | 1157 | CB | ASN | A | 186 | 4.689 | 2.478 | -0.575 | 1.00 | 29.65 | A | C |
| ATOM | 1158 | CG | ASN | A | 186 | 5.616 | 3.670 | -0.429 | 1.00 | 29.65 | A | C |
| ATOM | 1159 | OD1 | ASN | A | 186 | 6.610 | 3.610 | 0.287 | 1.00 | 29.65 | A | O |
| ATOM | 1160 | ND2 | ASN | A | 186 | 5.286 | 4.763 | -1.097 | 1.00 | 29.65 | A | N |
| ATOM | 1161 | C | ASN | A | 186 | 4.528 | 0.007 | -0.506 | 1.00 | 34.70 | A | C |
| ATOM | 1162 | O | ASN | A | 186 | 3.803 | 0.048 | 0.484 | 1.00 | 34.70 | A | O |
| ATOM | 1163 | N | LEU | A | 187 | 4.544 | -1.023 | -1.336 | 1.00 | 28.67 | A | N |
| ATOM | 1164 | CA | LEU | A | 187 | 3.717 | -2.185 | -1.066 | 1.00 | 28.67 | A | C |
| ATOM | 1165 | CB | LEU | A | 187 | 2.868 | -2.550 | -2.288 | 1.00 | 35.49 | A | C |
| ATOM | 1166 | CG | LEU | A | 187 | 1.874 | -1.521 | -2.851 | 1.00 | 35.49 | A | C |
| ATOM | 1167 | CD1 | LEU | A | 187 | 1.286 | -2.055 | -4.141 | 1.00 | 35.49 | A | C |
| ATOM | 1168 | CD2 | LEU | A | 187 | 0.773 | -1.229 | -1.844 | 1.00 | 35.49 | A | C |
| ATOM | 1169 | C | LEU | A | 187 | 4.666 | -3.316 | -0.732 | 1.00 | 28.67 | A | C |
| ATOM | 1170 | O | LEU | A | 187 | 5.199 | -3.976 | -1.621 | 1.00 | 28.67 | A | O |
| ATOM | 1171 | N | LEU | A | 188 | 4.905 | -3.518 | 0.556 | 1.00 | 50.22 | A | N |
| ATOM | 1172 | CA | LEU | A | 188 | 5.802 | -4.577 | 0.994 | 1.00 | 50.22 | A | C |
| ATOM | 1173 | CB | LEU | A | 188 | 6.242 | -4.340 | 2.431 | 1.00 | 44.62 | A | C |
| ATOM | 1174 | CG | LEU | A | 188 | 6.778 | -2.962 | 2.786 | 1.00 | 44.62 | A | C |
| ATOM | 1175 | CD1 | LEU | A | 188 | 7.213 | -3.010 | 4.235 | 1.00 | 44.62 | A | C |
| ATOM | 1176 | CD2 | LEU | A | 188 | 7.942 | -2.578 | 1.892 | 1.00 | 44.62 | A | C |
| ATOM | 1177 | C | LEU | A | 188 | 5.065 | -5.903 | 0.928 | 1.00 | 50.22 | A | C |
| ATOM | 1178 | O | LEU | A | 188 | 3.828 | -5.930 | 0.956 | 1.00 | 50.22 | A | O |
| ATOM | 1179 | N | LEU | A | 189 | 5.812 | -7.003 | 0.848 | 1.00 | 39.41 | A | N |
| ATOM | 1180 | CA | LEU | A | 189 | 5.183 | -8.314 | 0.796 | 1.00 | 39.41 | A | C |
| ATOM | 1181 | CB | LEU | A | 189 | 4.498 | -8.537 | -0.559 | 1.00 | 56.58 | A | C |
| ATOM | 1182 | CG | LEU | A | 189 | 5.377 | -8.653 | -1.800 | 1.00 | 56.58 | A | C |
| ATOM | 1183 | CD1 | LEU | A | 189 | 4.535 | -8.896 | -3.028 | 1.00 | 56.58 | A | C |
| ATOM | 1184 | CD2 | LEU | A | 189 | 6.164 | -7.387 | -1.959 | 1.00 | 56.58 | A | C |
| ATOM | 1185 | C | LEU | A | 189 | 6.149 | -9.447 | 1.069 | 1.00 | 39.41 | A | C |
| ATOM | 1186 | O | LEU | A | 189 | 7.284 | -9.451 | 0.597 | 1.00 | 39.41 | A | O |
| ATOM | 1187 | N | ASP | A | 190 | 5.670 | -10.405 | 1.854 | 1.00 | 53.32 | A | N |
| ATOM | 1188 | CA | ASP | A | 190 | 6.419 | -11.595 | 2.228 | 1.00 | 53.32 | A | C |
| ATOM | 1189 | CB | ASP | A | 190 | 5.898 | -12.103 | 3.573 | 1.00 | 69.54 | A | C |
| ATOM | 1190 | CG | ASP | A | 190 | 6.558 | -13.393 | 4.015 | 1.00 | 69.54 | A | C |
| ATOM | 1191 | OD1 | ASP | A | 190 | 6.370 | -14.434 | 3.344 | 1.00 | 69.54 | A | O |
| ATOM | 1192 | OD2 | ASP | A | 190 | 7.267 | -13.368 | 5.043 | 1.00 | 69.54 | A | O |
| ATOM | 1193 | C | ASP | A | 190 | 6.218 | -12.651 | 1.134 | 1.00 | 53.32 | A | C |
| ATOM | 1194 | O | ASP | A | 190 | 5.126 | -13.195 | 0.968 | 1.00 | 53.32 | A | O |
| ATOM | 1195 | N | PRO | A | 191 | 7.282 | -12.966 | 0.385 | 1.00 | 61.39 | A | N |
| ATOM | 1196 | CD | PRO | A | 191 | 8.686 | -12.602 | 0.659 | 1.00 | 73.41 | A | C |
| ATOM | 1197 | CA | PRO | A | 191 | 7.202 | -13.957 | -0.693 | 1.00 | 61.39 | A | C |
| ATOM | 1198 | CB | PRO | A | 191 | 8.645 | -14.031 | -1.198 | 1.00 | 73.41 | A | C |
| ATOM | 1199 | CG | PRO | A | 191 | 9.448 | -13.773 | 0.066 | 1.00 | 73.41 | A | C |

| | | | | | | | | | | | | |
|---|---|---|---|---|---|---|---|---|---|---|---|---|
| ATOM | 1200 | C | PRO | A | 191 | 6.627 | -15.338 | -0.325 | 1.00 | 61.39 | A | C |
| ATOM | 1201 | O | PRO | A | 191 | 5.838 | -15.892 | -1.086 | 1.00 | 61.39 | A | O |
| ATOM | 1202 | N | ASP | A | 192 | 6.995 | -15.893 | 0.827 | 1.00 | 42.47 | A | N |
| ATOM | 1203 | CA | ASP | A | 192 | 6.484 | -17.218 | 1.190 | 1.00 | 42.47 | A | C |
| ATOM | 1204 | CB | ASP | A | 192 | 7.370 | -17.888 | 2.256 | 1.00 | 72.10 | A | C |
| ATOM | 1205 | CG | ASP | A | 192 | 8.847 | -17.942 | 1.868 | 1.00 | 72.10 | A | C |
| ATOM | 1206 | OD1 | ASP | A | 192 | 9.184 | -18.343 | 0.725 | 1.00 | 72.10 | A | O |
| ATOM | 1207 | OD2 | ASP | A | 192 | 9.675 | -17.591 | 2.736 | 1.00 | 72.10 | A | O |
| ATOM | 1208 | C | ASP | A | 192 | 5.044 | -17.240 | 1.701 | 1.00 | 42.47 | A | C |
| ATOM | 1209 | O | ASP | A | 192 | 4.415 | -18.302 | 1.751 | 1.00 | 42.47 | A | O |
| ATOM | 1210 | N | THR | A | 193 | 4.518 | -16.093 | 2.109 | 1.00 | 43.70 | A | N |
| ATOM | 1211 | CA | THR | A | 193 | 3.150 | -16.068 | 2.617 | 1.00 | 43.70 | A | C |
| ATOM | 1212 | CB | THR | A | 193 | 3.072 | -15.470 | 4.040 | 1.00 | 50.47 | A | C |
| ATOM | 1213 | OG1 | THR | A | 193 | 3.762 | -14.215 | 4.084 | 1.00 | 50.47 | A | O |
| ATOM | 1214 | CG2 | THR | A | 193 | 3.692 | -16.413 | 5.035 | 1.00 | 50.47 | A | C |
| ATOM | 1215 | C | THR | A | 193 | 2.233 | -15.295 | 1.700 | 1.00 | 43.70 | A | C |
| ATOM | 1216 | O | THR | A | 193 | 1.014 | -15.464 | 1.742 | 1.00 | 43.70 | A | O |
| ATOM | 1217 | N | ALA | A | 194 | 2.842 | -14.455 | 0.871 | 1.00 | 46.87 | A | N |
| ATOM | 1218 | CA | ALA | A | 194 | 2.123 | -13.634 | -0.097 | 1.00 | 46.87 | A | C |
| ATOM | 1219 | CB | ALA | A | 194 | 1.274 | -14.526 | -1.011 | 1.00 | 39.81 | A | C |
| ATOM | 1220 | C | ALA | A | 194 | 1.251 | -12.544 | 0.528 | 1.00 | 46.87 | A | C |
| ATOM | 1221 | O | ALA | A | 194 | 0.272 | -12.109 | -0.071 | 1.00 | 46.87 | A | O |
| ATOM | 1222 | N | VAL | A | 195 | 1.593 | -12.097 | 1.729 | 1.00 | 32.58 | A | N |
| ATOM | 1223 | CA | VAL | A | 195 | 0.803 | -11.051 | 2.347 | 1.00 | 32.58 | A | C |
| ATOM | 1224 | CB | VAL | A | 195 | 0.713 | -11.249 | 3.880 | 1.00 | 41.42 | A | C |
| ATOM | 1225 | CG1 | VAL | A | 195 | 0.689 | -12.737 | 4.196 | 1.00 | 41.42 | A | C |
| ATOM | 1226 | CG2 | VAL | A | 195 | 1.852 | -10.532 | 4.594 | 1.00 | 41.42 | A | C |
| ATOM | 1227 | C | VAL | A | 195 | 1.425 | -9.695 | 2.005 | 1.00 | 32.58 | A | C |
| ATOM | 1228 | O | VAL | A | 195 | 2.648 | -9.539 | 1.977 | 1.00 | 32.58 | A | O |
| ATOM | 1229 | N | LEU | A | 196 | 0.573 | -8.721 | 1.718 | 1.00 | 47.57 | A | N |
| ATOM | 1230 | CA | LEU | A | 196 | 1.044 | -7.391 | 1.368 | 1.00 | 47.57 | A | C |
| ATOM | 1231 | CB | LEU | A | 196 | 0.324 | -6.910 | 0.101 | 1.00 | 27.30 | A | C |
| ATOM | 1232 | CG | LEU | A | 196 | 0.630 | -5.527 | -0.477 | 1.00 | 27.30 | A | C |
| ATOM | 1233 | CD1 | LEU | A | 196 | 0.367 | -5.541 | -1.945 | 1.00 | 27.30 | A | C |
| ATOM | 1234 | CD2 | LEU | A | 196 | -0.212 | -4.481 | 0.188 | 1.00 | 27.30 | A | C |
| ATOM | 1235 | C | LEU | A | 196 | 0.812 | -6.420 | 2.525 | 1.00 | 47.57 | A | C |
| ATOM | 1236 | O | LEU | A | 196 | -0.126 | -6.584 | 3.317 | 1.00 | 47.57 | A | O |
| ATOM | 1237 | N | LYS | A | 197 | 1.670 | -5.410 | 2.625 | 1.00 | 56.26 | A | N |
| ATOM | 1238 | CA | LYS | A | 197 | 1.554 | -4.426 | 3.690 | 1.00 | 56.26 | A | C |
| ATOM | 1239 | CB | LYS | A | 197 | 2.529 | -4.754 | 4.818 | 1.00 | 43.34 | A | C |
| ATOM | 1240 | CG | LYS | A | 197 | 2.137 | -5.980 | 5.573 | 1.00 | 43.34 | A | C |
| ATOM | 1241 | CD | LYS | A | 197 | 3.228 | -6.470 | 6.466 | 1.00 | 43.34 | A | C |
| ATOM | 1242 | CE | LYS | A | 197 | 2.729 | -7.685 | 7.217 | 1.00 | 43.34 | A | C |
| ATOM | 1243 | NZ | LYS | A | 197 | 1.392 | -7.388 | 7.799 | 1.00 | 43.34 | A | N |
| ATOM | 1244 | C | LYS | A | 197 | 1.820 | -3.018 | 3.214 | 1.00 | 56.26 | A | C |
| ATOM | 1245 | O | LYS | A | 197 | 2.875 | -2.728 | 2.641 | 1.00 | 56.26 | A | O |
| ATOM | 1246 | N | LEU | A | 198 | 0.861 | -2.134 | 3.444 | 1.00 | 37.07 | A | N |
| ATOM | 1247 | CA | LEU | A | 198 | 1.055 | -0.759 | 3.061 | 1.00 | 37.07 | A | C |
| ATOM | 1248 | CB | LEU | A | 198 | -0.255 | 0.017 | 3.190 | 1.00 | 46.50 | A | C |
| ATOM | 1249 | CG | LEU | A | 198 | -0.727 | 0.860 | 1.993 | 1.00 | 46.50 | A | C |
| ATOM | 1250 | CD1 | LEU | A | 198 | -1.917 | 1.695 | 2.420 | 1.00 | 46.50 | A | C |
| ATOM | 1251 | CD2 | LEU | A | 198 | 0.378 | 1.765 | 1.493 | 1.00 | 46.50 | A | C |
| ATOM | 1252 | C | LEU | A | 198 | 2.074 | -0.214 | 4.055 | 1.00 | 37.07 | A | C |
| ATOM | 1253 | O | LEU | A | 198 | 1.999 | -0.505 | 5.254 | 1.00 | 37.07 | A | O |
| ATOM | 1254 | N | CYS | A | 199 | 3.045 | 0.542 | 3.558 | 1.00 | 43.73 | A | N |
| ATOM | 1255 | CA | CYS | A | 199 | 4.034 | 1.157 | 4.431 | 1.00 | 43.73 | A | C |
| ATOM | 1256 | CB | CYS | A | 199 | 5.333 | 0.327 | 4.502 | 1.00 | 58.44 | A | C |
| ATOM | 1257 | SG | CYS | A | 199 | 6.464 | 0.479 | 3.086 | 1.00 | 58.44 | A | S |
| ATOM | 1258 | C | CYS | A | 199 | 4.309 | 2.554 | 3.888 | 1.00 | 43.73 | A | C |
| ATOM | 1259 | O | CYS | A | 199 | 3.873 | 2.890 | 2.799 | 1.00 | 43.73 | A | O |
| ATOM | 1260 | N | ASP | A | 200 | 5.039 | 3.355 | 4.657 | 1.00 | 51.23 | A | N |
| ATOM | 1261 | CA | ASP | A | 200 | 5.383 | 4.736 | 4.312 | 1.00 | 51.23 | A | C |
| ATOM | 1262 | CB | ASP | A | 200 | 5.992 | 4.844 | 2.917 | 1.00 | 60.23 | A | C |
| ATOM | 1263 | CG | ASP | A | 200 | 6.639 | 6.207 | 2.679 | 1.00 | 60.23 | A | C |
| ATOM | 1264 | OD1 | ASP | A | 200 | 6.448 | 7.103 | 3.538 | 1.00 | 60.23 | A | O |
| ATOM | 1265 | OD2 | ASP | A | 200 | 7.335 | 6.394 | 1.644 | 1.00 | 60.23 | A | O |
| ATOM | 1266 | C | ASP | A | 200 | 4.168 | 5.647 | 4.400 | 1.00 | 51.23 | A | C |

| | | | | | | | | | | | |
|---|---|---|---|---|---|---|---|---|---|---|---|
| ATOM | 1267 | O | ASP | A | 200 | 3.537 | 5.969 | 3.391 | 1.00 | 51.23 | A | O |
| ATOM | 1268 | N | PHE | A | 201 | 3.839 | 6.048 | 5.623 | 1.00 | 36.54 | A | N |
| ATOM | 1269 | CA | PHE | A | 201 | 2.713 | 6.932 | 5.845 | 1.00 | 36.54 | A | C |
| ATOM | 1270 | CB | PHE | A | 201 | 1.896 | 6.460 | 7.051 | 1.00 | 39.75 | A | C |
| ATOM | 1271 | CG | PHE | A | 201 | 1.023 | 5.258 | 6.769 | 1.00 | 39.75 | A | C |
| ATOM | 1272 | CD1 | PHE | A | 201 | 1.578 | 3.993 | 6.621 | 1.00 | 39.75 | A | C |
| ATOM | 1273 | CD2 | PHE | A | 201 | -0.367 | 5.394 | 6.670 | 1.00 | 39.75 | A | C |
| ATOM | 1274 | CE1 | PHE | A | 201 | 0.760 | 2.881 | 6.379 | 1.00 | 39.75 | A | C |
| ATOM | 1275 | CE2 | PHE | A | 201 | -1.192 | 4.292 | 6.431 | 1.00 | 39.75 | A | C |
| ATOM | 1276 | CZ | PHE | A | 201 | -0.633 | 3.037 | 6.286 | 1.00 | 39.75 | A | C |
| ATOM | 1277 | C | PHE | A | 201 | 3.222 | 8.347 | 6.066 | 1.00 | 36.54 | A | C |
| ATOM | 1278 | O | PHE | A | 201 | 2.576 | 9.145 | 6.735 | 1.00 | 36.54 | A | O |
| ATOM | 1279 | N | GLY | A | 202 | 4.380 | 8.651 | 5.484 | 1.00 | 40.97 | A | N |
| ATOM | 1280 | CA | GLY | A | 202 | 4.973 | 9.969 | 5.636 | 1.00 | 40.97 | A | C |
| ATOM | 1281 | C | GLY | A | 202 | 4.275 | 11.085 | 4.875 | 1.00 | 40.97 | A | C |
| ATOM | 1282 | O | GLY | A | 202 | 4.645 | 12.245 | 5.017 | 1.00 | 40.97 | A | O |
| ATOM | 1283 | N | SER | A | 203 | 3.276 | 10.733 | 4.067 | 1.00 | 41.55 | A | N |
| ATOM | 1284 | CA | SER | A | 203 | 2.510 | 11.691 | 3.266 | 1.00 | 41.55 | A | C |
| ATOM | 1285 | CB | SER | A | 203 | 2.714 | 11.440 | 1.770 | 1.00 | 37.73 | A | C |
| ATOM | 1286 | OG | SER | A | 203 | 4.084 | 11.415 | 1.428 | 1.00 | 37.73 | A | O |
| ATOM | 1287 | C | SER | A | 203 | 1.034 | 11.498 | 3.569 | 1.00 | 41.55 | A | C |
| ATOM | 1288 | O | SER | A | 203 | 0.179 | 12.202 | 3.030 | 1.00 | 41.55 | A | O |
| ATOM | 1289 | N | ALA | A | 204 | 0.743 | 10.513 | 4.414 | 1.00 | 49.19 | A | N |
| ATOM | 1290 | CA | ALA | A | 204 | -0.629 | 10.206 | 4.784 | 1.00 | 49.19 | A | C |
| ATOM | 1291 | CB | ALA | A | 204 | -0.675 | 8.914 | 5.615 | 1.00 | 27.24 | A | C |
| ATOM | 1292 | C | ALA | A | 204 | -1.205 | 11.374 | 5.571 | 1.00 | 49.19 | A | C |
| ATOM | 1293 | O | ALA | A | 204 | -0.479 | 12.064 | 6.282 | 1.00 | 49.19 | A | O |
| ATOM | 1294 | N | LYS | A | 205 | -2.508 | 11.593 | 5.449 | 1.00 | 33.17 | A | N |
| ATOM | 1295 | CA | LYS | A | 205 | -3.148 | 12.686 | 6.155 | 1.00 | 33.17 | A | C |
| ATOM | 1296 | CB | LYS | A | 205 | -2.875 | 14.001 | 5.434 | 1.00 | 30.26 | A | C |
| ATOM | 1297 | CG | LYS | A | 205 | -3.725 | 15.175 | 5.889 | 1.00 | 30.26 | A | C |
| ATOM | 1298 | CD | LYS | A | 205 | -3.254 | 16.440 | 5.189 | 1.00 | 30.26 | A | C |
| ATOM | 1299 | CE | LYS | A | 205 | -4.027 | 17.671 | 5.618 | 1.00 | 30.26 | A | C |
| ATOM | 1300 | NZ | LYS | A | 205 | -3.506 | 18.859 | 4.891 | 1.00 | 30.26 | A | N |
| ATOM | 1301 | C | LYS | A | 205 | -4.637 | 12.505 | 6.280 | 1.00 | 33.17 | A | C |
| ATOM | 1302 | O | LYS | A | 205 | -5.300 | 12.029 | 5.360 | 1.00 | 33.17 | A | O |
| ATOM | 1303 | N | GLN | A | 206 | -5.165 | 12.888 | 7.432 | 1.00 | 61.09 | A | N |
| ATOM | 1304 | CA | GLN | A | 206 | -6.595 | 12.804 | 7.657 | 1.00 | 61.09 | A | C |
| ATOM | 1305 | CB | GLN | A | 206 | -6.897 | 12.891 | 9.151 | 1.00 | 99.25 | A | C |
| ATOM | 1306 | CG | GLN | A | 206 | -8.366 | 12.756 | 9.475 | 1.00 | 99.25 | A | C |
| ATOM | 1307 | CD | GLN | A | 206 | -8.598 | 12.072 | 10.810 | 1.00 | 99.25 | A | C |
| ATOM | 1308 | OE1 | GLN | A | 206 | -9.718 | 12.101 | 11.350 | 1.00 | 99.25 | A | O |
| ATOM | 1309 | NE2 | GLN | A | 206 | -7.540 | 11.441 | 11.357 | 1.00 | 99.25 | A | N |
| ATOM | 1310 | C | GLN | A | 206 | -7.185 | 13.995 | 6.911 | 1.00 | 61.09 | A | C |
| ATOM | 1311 | O | GLN | A | 206 | -6.825 | 15.141 | 7.177 | 1.00 | 61.09 | A | O |
| ATOM | 1312 | N | LEU | A | 207 | -8.067 | 13.724 | 5.957 | 1.00 | 49.78 | A | N |
| ATOM | 1313 | CA | LEU | A | 207 | -8.663 | 14.797 | 5.166 | 1.00 | 49.78 | A | C |
| ATOM | 1314 | CB | LEU | A | 207 | -8.888 | 14.356 | 3.719 | 1.00 | 30.14 | A | C |
| ATOM | 1315 | CG | LEU | A | 207 | -7.656 | 13.896 | 2.936 | 1.00 | 30.14 | A | C |
| ATOM | 1316 | CD1 | LEU | A | 207 | -8.112 | 13.300 | 1.626 | 1.00 | 30.14 | A | C |
| ATOM | 1317 | CD2 | LEU | A | 207 | -6.678 | 15.044 | 2.722 | 1.00 | 30.14 | A | C |
| ATOM | 1318 | C | LEU | A | 207 | -9.975 | 15.238 | 5.760 | 1.00 | 49.78 | A | C |
| ATOM | 1319 | O | LEU | A | 207 | -10.831 | 14.414 | 6.102 | 1.00 | 49.78 | A | O |
| ATOM | 1320 | N | VAL | A | 208 | -10.111 | 16.553 | 5.887 | 1.00 | 43.58 | A | N |
| ATOM | 1321 | CA | VAL | A | 208 | -11.306 | 17.166 | 6.433 | 1.00 | 43.58 | A | C |
| ATOM | 1322 | CB | VAL | A | 208 | -10.966 | 18.046 | 7.662 | 1.00 | 49.68 | A | C |
| ATOM | 1323 | CG1 | VAL | A | 208 | -11.995 | 19.145 | 7.826 | 1.00 | 49.68 | A | C |
| ATOM | 1324 | CG2 | VAL | A | 208 | -10.937 | 17.190 | 8.918 | 1.00 | 49.68 | A | C |
| ATOM | 1325 | C | VAL | A | 208 | -11.944 | 18.015 | 5.348 | 1.00 | 43.58 | A | C |
| ATOM | 1326 | O | VAL | A | 208 | -11.289 | 18.875 | 4.737 | 1.00 | 43.58 | A | O |
| ATOM | 1327 | N | ARG | A | 209 | -13.222 | 17.759 | 5.102 | 1.00 | 43.13 | A | N |
| ATOM | 1328 | CA | ARG | A | 209 | -13.945 | 18.511 | 4.092 | 1.00 | 43.13 | A | C |
| ATOM | 1329 | CB | ARG | A | 209 | -15.400 | 18.052 | 4.040 | 1.00 | 93.17 | A | C |
| ATOM | 1330 | CG | ARG | A | 209 | -16.184 | 18.609 | 2.846 | 1.00 | 93.17 | A | C |
| ATOM | 1331 | CD | ARG | A | 209 | -17.605 | 18.061 | 2.841 | 1.00 | 93.17 | A | C |
| ATOM | 1332 | NE | ARG | A | 209 | -17.597 | 16.639 | 3.190 | 1.00 | 93.17 | A | N |
| ATOM | 1333 | CZ | ARG | A | 209 | -18.687 | 15.908 | 3.417 | 1.00 | 93.17 | A | C |

| | | | | | | | | | | | | |
|---|---|---|---|---|---|---|---|---|---|---|---|---|
| ATOM | 1334 | NH1 | ARG | A | 209 | -19.905 | 16.461 | 3.328 | 1.00 | 93.17 | A | N |
| ATOM | 1335 | NH2 | ARG | A | 209 | -18.553 | 14.625 | 3.760 | 1.00 | 93.17 | A | N |
| ATOM | 1336 | C | ARG | A | 209 | -13.893 | 19.993 | 4.443 | 1.00 | 43.13 | A | C |
| ATOM | 1337 | O | ARG | A | 209 | -14.405 | 20.405 | 5.488 | 1.00 | 43.13 | A | O |
| ATOM | 1338 | N | GLY | A | 210 | -13.273 | 20.794 | 3.583 | 1.00 | 50.74 | A | N |
| ATOM | 1339 | CA | GLY | A | 210 | -13.200 | 22.219 | 3.852 | 1.00 | 50.74 | A | C |
| ATOM | 1340 | C | GLY | A | 210 | -11.782 | 22.736 | 3.978 | 1.00 | 50.74 | A | C |
| ATOM | 1341 | O | GLY | A | 210 | -11.435 | 23.790 | 3.423 | 1.00 | 50.74 | A | O |
| ATOM | 1342 | N | GLU | A | 211 | -10.955 | 22.000 | 4.715 | 1.00 | 57.36 | A | N |
| ATOM | 1343 | CA | GLU | A | 211 | -9.565 | 22.403 | 4.890 | 1.00 | 57.36 | A | C |
| ATOM | 1344 | CB | GLU | A | 211 | -9.005 | 21.755 | 6.158 | 1.00 | 66.68 | A | C |
| ATOM | 1345 | CG | GLU | A | 211 | -9.878 | 21.991 | 7.381 | 1.00 | 66.68 | A | C |
| ATOM | 1346 | CD | GLU | A | 211 | -9.536 | 21.060 | 8.538 | 1.00 | 66.68 | A | C |
| ATOM | 1347 | OE1 | GLU | A | 211 | -10.171 | 21.184 | 9.617 | 1.00 | 66.68 | A | O |
| ATOM | 1348 | OE2 | GLU | A | 211 | -8.639 | 20.200 | 8.365 | 1.00 | 66.68 | A | O |
| ATOM | 1349 | C | GLU | A | 211 | -8.787 | 21.962 | 3.645 | 1.00 | 57.36 | A | C |
| ATOM | 1350 | O | GLU | A | 211 | -8.667 | 20.774 | 3.362 | 1.00 | 57.36 | A | O |
| ATOM | 1351 | N | PRO | A | 212 | -8.277 | 22.922 | 2.865 | 1.00 | 49.93 | A | N |
| ATOM | 1352 | CD | PRO | A | 212 | -8.293 | 24.377 | 3.086 | 1.00 | 52.84 | A | C |
| ATOM | 1353 | CA | PRO | A | 212 | -7.522 | 22.585 | 1.651 | 1.00 | 49.93 | A | C |
| ATOM | 1354 | CB | PRO | A | 212 | -7.149 | 23.955 | 1.079 | 1.00 | 52.84 | A | C |
| ATOM | 1355 | CG | PRO | A | 212 | -7.069 | 24.825 | 2.304 | 1.00 | 52.84 | A | C |
| ATOM | 1356 | C | PRO | A | 212 | -6.297 | 21.722 | 1.903 | 1.00 | 49.93 | A | C |
| ATOM | 1357 | O | PRO | A | 212 | -5.757 | 21.708 | 3.009 | 1.00 | 49.93 | A | O |
| ATOM | 1358 | N | ASN | A | 213 | -5.868 | 21.010 | 0.862 | 1.00 | 44.51 | A | N |
| ATOM | 1359 | CA | ASN | A | 213 | -4.703 | 20.132 | 0.934 | 1.00 | 44.51 | A | C |
| ATOM | 1360 | CB | ASN | A | 213 | -5.150 | 18.674 | 0.920 | 1.00 | 37.35 | A | C |
| ATOM | 1361 | CG | ASN | A | 213 | -6.212 | 18.389 | 1.946 | 1.00 | 37.35 | A | C |
| ATOM | 1362 | OD1 | ASN | A | 213 | -5.974 | 18.495 | 3.142 | 1.00 | 37.35 | A | O |
| ATOM | 1363 | ND2 | ASN | A | 213 | -7.403 | 18.035 | 1.483 | 1.00 | 37.35 | A | N |
| ATOM | 1364 | C | ASN | A | 213 | -3.795 | 20.388 | -0.261 | 1.00 | 44.51 | A | C |
| ATOM | 1365 | O | ASN | A | 213 | -4.256 | 20.836 | -1.300 | 1.00 | 44.51 | A | O |
| ATOM | 1366 | N | VAL | A | 214 | -2.507 | 20.105 | -0.111 | 1.00 | 34.13 | A | N |
| ATOM | 1367 | CA | VAL | A | 214 | -1.549 | 20.293 | -1.197 | 1.00 | 34.13 | A | C |
| ATOM | 1368 | CB | VAL | A | 214 | -0.109 | 19.954 | -0.726 | 1.00 | 35.10 | A | C |
| ATOM | 1369 | CG1 | VAL | A | 214 | 0.831 | 19.824 | -1.917 | 1.00 | 35.10 | A | C |
| ATOM | 1370 | CG2 | VAL | A | 214 | 0.388 | 21.032 | 0.207 | 1.00 | 35.10 | A | C |
| ATOM | 1371 | C | VAL | A | 214 | -1.920 | 19.384 | -2.366 | 1.00 | 34.13 | A | C |
| ATOM | 1372 | O | VAL | A | 214 | -2.209 | 18.202 | -2.173 | 1.00 | 34.13 | A | O |
| ATOM | 1373 | N | SER | A | 215 | -1.915 | 19.928 | -3.578 | 1.00 | 42.10 | A | N |
| ATOM | 1374 | CA | SER | A | 215 | -2.270 | 19.118 | -4.734 | 1.00 | 42.10 | A | C |
| ATOM | 1375 | CB | SER | A | 215 | -2.597 | 20.006 | -5.943 | 1.00 | 53.16 | A | C |
| ATOM | 1376 | OG | SER | A | 215 | -1.539 | 20.897 | -6.248 | 1.00 | 53.16 | A | O |
| ATOM | 1377 | C | SER | A | 215 | -1.153 | 18.125 | -5.061 | 1.00 | 42.10 | A | C |
| ATOM | 1378 | O | SER | A | 215 | -0.885 | 17.800 | -6.219 | 1.00 | 42.10 | A | O |
| ATOM | 1379 | N | PTY | A | 216 | 0.671 | 19.561 | -5.342 | 1.00 | 45.96 | A | N |
| ATOM | 1380 | CA | PTY | A | 216 | 1.727 | 18.589 | -5.592 | 1.00 | 45.96 | A | C |
| ATOM | 1381 | C | PTY | A | 216 | 1.903 | 17.684 | -4.381 | 1.00 | 45.96 | A | C |
| ATOM | 1382 | O | PTY | A | 216 | 2.851 | 16.901 | -4.309 | 1.00 | 45.96 | A | O |
| ATOM | 1383 | CB | PTY | A | 216 | 3.033 | 19.319 | -5.870 | 1.00 | 45.96 | A | C |
| ATOM | 1384 | CG | PTY | A | 216 | 3.913 | 18.570 | -6.766 | 1.00 | 45.96 | A | C |
| ATOM | 1385 | CD1 | PTY | A | 216 | 3.639 | 18.539 | -8.205 | 1.00 | 45.96 | A | C |
| ATOM | 1386 | CD2 | PTY | A | 216 | 5.184 | 18.076 | -6.235 | 1.00 | 45.96 | A | C |
| ATOM | 1387 | CE1 | PTY | A | 216 | 4.663 | 18.012 | -9.107 | 1.00 | 45.96 | A | C |
| ATOM | 1388 | CE2 | PTY | A | 216 | 6.187 | 17.560 | -7.164 | 1.00 | 45.96 | A | C |
| ATOM | 1389 | CZ | PTY | A | 216 | 5.944 | 17.522 | -8.604 | 1.00 | 45.96 | A | C |
| ATOM | 1390 | OH | PTY | A | 216 | 6.920 | 17.109 | -9.474 | 1.00 | 45.96 | A | O |
| ATOM | 1391 | P | PTY | A | 216 | 6.966 | 15.626 | -9.781 | 1.00 | 45.96 | A | P |
| ATOM | 1392 | OP1 | PTY | A | 216 | 6.050 | 15.498 | -10.957 | 1.00 | 45.96 | A | O |
| ATOM | 1393 | OP2 | PTY | A | 216 | 6.561 | 14.822 | -8.498 | 1.00 | 45.96 | A | O |
| ATOM | 1394 | OP3 | PTY | A | 216 | 8.365 | 15.302 | -10.211 | 1.00 | 45.96 | A | O |
| ATOM | 1395 | N | ILE | A | 217 | 0.818 | 15.558 | -4.827 | 1.00 | 44.35 | A | N |
| ATOM | 1396 | CA | ILE | A | 217 | 1.502 | 14.532 | -4.070 | 1.00 | 44.35 | A | C |
| ATOM | 1397 | CB | ILE | A | 217 | 0.869 | 14.335 | -2.658 | 1.00 | 56.22 | A | C |
| ATOM | 1398 | CG2 | ILE | A | 217 | 1.335 | 15.430 | -1.707 | 1.00 | 56.22 | A | C |
| ATOM | 1399 | CG1 | ILE | A | 217 | -0.658 | 14.302 | -2.760 | 1.00 | 56.22 | A | C |
| ATOM | 1400 | CD1 | ILE | A | 217 | -1.275 | 15.611 | -3.165 | 1.00 | 56.22 | A | C |

| | | | | | | | | | | | | |
|---|---|---|---|---|---|---|---|---|---|---|---|---|
| ATOM | 1401 | C | ILE | A | 217 | 1.419 | 13.232 | -4.854 | 1.00 | 44.35 | A | C |
| ATOM | 1402 | O | ILE | A | 217 | 0.807 | 13.191 | -5.919 | 1.00 | 44.35 | A | O |
| ATOM | 1403 | N | CYS | A | 218 | 2.023 | 12.181 | -4.307 | 1.00 | 40.34 | A | N |
| ATOM | 1404 | CA | CYS | A | 218 | 2.069 | 10.856 | -4.931 | 1.00 | 40.34 | A | C |
| ATOM | 1405 | CB | CYS | A | 218 | 0.762 | 10.486 | -5.646 | 1.00 | 47.49 | A | C |
| ATOM | 1406 | SG | CYS | A | 218 | -0.761 | 10.832 | -4.804 | 1.00 | 47.49 | A | S |
| ATOM | 1407 | C | CYS | A | 218 | 3.153 | 10.885 | -5.984 | 1.00 | 40.34 | A | C |
| ATOM | 1408 | O | CYS | A | 218 | 3.417 | 11.934 | -6.570 | 1.00 | 40.34 | A | O |
| ATOM | 1409 | N | SER | A | 219 | 3.787 | 9.746 | -6.238 | 1.00 | 33.71 | A | N |
| ATOM | 1410 | CA | SER | A | 219 | 4.787 | 9.732 | -7.284 | 1.00 | 33.71 | A | C |
| ATOM | 1411 | CB | SER | A | 219 | 5.409 | 8.350 | -7.435 | 1.00 | 48.03 | A | C |
| ATOM | 1412 | OG | SER | A | 219 | 6.105 | 7.993 | -6.261 | 1.00 | 48.03 | A | O |
| ATOM | 1413 | C | SER | A | 219 | 4.008 | 10.079 | -8.535 | 1.00 | 33.71 | A | C |
| ATOM | 1414 | O | SER | A | 219 | 2.895 | 9.616 | -8.722 | 1.00 | 33.71 | A | O |
| ATOM | 1415 | N | ARG | A | 220 | 4.596 | 10.905 | -9.382 | 1.00 | 36.99 | A | N |
| ATOM | 1416 | CA | ARG | A | 220 | 3.977 | 11.338 | -10.628 | 1.00 | 36.99 | A | C |
| ATOM | 1417 | CB | ARG | A | 220 | 5.051 | 11.889 | -11.549 | 1.00 | 44.99 | A | C |
| ATOM | 1418 | CG | ARG | A | 220 | 4.518 | 12.508 | -12.795 | 1.00 | 44.99 | A | C |
| ATOM | 1419 | CD | ARG | A | 220 | 5.516 | 13.515 | -13.306 | 1.00 | 44.99 | A | C |
| ATOM | 1420 | NE | ARG | A | 220 | 6.722 | 12.887 | -13.831 | 1.00 | 44.99 | A | N |
| ATOM | 1421 | CZ | ARG | A | 220 | 7.928 | 13.434 | -13.761 | 1.00 | 44.99 | A | C |
| ATOM | 1422 | NH1 | ARG | A | 220 | 8.092 | 14.617 | -13.178 | 1.00 | 44.99 | A | N |
| ATOM | 1423 | NH2 | ARG | A | 220 | 8.965 | 12.809 | -14.288 | 1.00 | 44.99 | A | N |
| ATOM | 1424 | C | ARG | A | 220 | 3.179 | 10.288 | -11.383 | 1.00 | 36.99 | A | C |
| ATOM | 1425 | O | ARG | A | 220 | 2.012 | 10.497 | -11.705 | 1.00 | 36.99 | A | O |
| ATOM | 1426 | N | TYR | A | 221 | 3.820 | 9.166 | -11.676 | 1.00 | 39.79 | A | N |
| ATOM | 1427 | CA | TYR | A | 221 | 3.174 | 8.089 | -12.421 | 1.00 | 39.79 | A | C |
| ATOM | 1428 | CB | TYR | A | 221 | 4.087 | 6.867 | -12.498 | 1.00 | 43.50 | A | C |
| ATOM | 1429 | CG | TYR | A | 221 | 5.325 | 7.051 | -13.342 | 1.00 | 43.50 | A | C |
| ATOM | 1430 | CD1 | TYR | A | 221 | 5.687 | 8.309 | -13.824 | 1.00 | 43.50 | A | C |
| ATOM | 1431 | CE1 | TYR | A | 221 | 6.815 | 8.476 | -14.594 | 1.00 | 43.50 | A | C |
| ATOM | 1432 | CD2 | TYR | A | 221 | 6.135 | 5.964 | -13.654 | 1.00 | 43.50 | A | C |
| ATOM | 1433 | CE2 | TYR | A | 221 | 7.269 | 6.124 | -14.422 | 1.00 | 43.50 | A | C |
| ATOM | 1434 | CZ | TYR | A | 221 | 7.602 | 7.382 | -14.891 | 1.00 | 43.50 | A | C |
| ATOM | 1435 | OH | TYR | A | 221 | 8.721 | 7.542 | -15.668 | 1.00 | 43.50 | A | O |
| ATOM | 1436 | C | TYR | A | 221 | 1.866 | 7.651 | -11.812 | 1.00 | 39.79 | A | C |
| ATOM | 1437 | O | TYR | A | 221 | 0.938 | 7.265 | -12.519 | 1.00 | 39.79 | A | O |
| ATOM | 1438 | N | TYR | A | 222 | 1.802 | 7.710 | -10.489 | 1.00 | 39.86 | A | N |
| ATOM | 1439 | CA | TYR | A | 222 | 0.629 | 7.270 | -9.764 | 1.00 | 39.86 | A | C |
| ATOM | 1440 | CB | TYR | A | 222 | 1.087 | 6.372 | -8.620 | 1.00 | 41.27 | A | C |
| ATOM | 1441 | CG | TYR | A | 222 | 2.057 | 5.323 | -9.099 | 1.00 | 41.27 | A | C |
| ATOM | 1442 | CD1 | TYR | A | 222 | 3.423 | 5.592 | -9.174 | 1.00 | 41.27 | A | C |
| ATOM | 1443 | CE1 | TYR | A | 222 | 4.310 | 4.671 | -9.732 | 1.00 | 41.27 | A | C |
| ATOM | 1444 | CD2 | TYR | A | 222 | 1.603 | 4.095 | -9.590 | 1.00 | 41.27 | A | C |
| ATOM | 1445 | CE2 | TYR | A | 222 | 2.487 | 3.171 | -10.151 | 1.00 | 41.27 | A | C |
| ATOM | 1446 | CZ | TYR | A | 222 | 3.834 | 3.473 | -10.217 | 1.00 | 41.27 | A | C |
| ATOM | 1447 | OH | TYR | A | 222 | 4.704 | 2.582 | -10.773 | 1.00 | 41.27 | A | O |
| ATOM | 1448 | C | TYR | A | 222 | -0.257 | 8.386 | -9.249 | 1.00 | 39.86 | A | C |
| ATOM | 1449 | O | TYR | A | 222 | -1.182 | 8.140 | -8.485 | 1.00 | 39.86 | A | O |
| ATOM | 1450 | N | ARG | A | 223 | 0.013 | 9.607 | -9.694 | 1.00 | 31.66 | A | N |
| ATOM | 1451 | CA | ARG | A | 223 | -0.745 | 10.774 | -9.275 | 1.00 | 31.66 | A | C |
| ATOM | 1452 | CB | ARG | A | 223 | 0.111 | 12.027 | -9.486 | 1.00 | 40.78 | A | C |
| ATOM | 1453 | CG | ARG | A | 223 | -0.644 | 13.331 | -9.304 | 1.00 | 40.78 | A | C |
| ATOM | 1454 | CD | ARG | A | 223 | 0.225 | 14.421 | -8.714 | 1.00 | 40.78 | A | C |
| ATOM | 1455 | NE | ARG | A | 223 | 1.327 | 14.800 | -9.589 | 1.00 | 40.78 | A | N |
| ATOM | 1456 | CZ | ARG | A | 223 | 2.618 | 14.612 | -9.308 | 1.00 | 40.78 | A | C |
| ATOM | 1457 | NH1 | ARG | A | 223 | 2.986 | 14.044 | -8.168 | 1.00 | 40.78 | A | N |
| ATOM | 1458 | NH2 | ARG | A | 223 | 3.546 | 15.001 | -10.173 | 1.00 | 40.78 | A | N |
| ATOM | 1459 | C | ARG | A | 223 | -2.102 | 10.927 | -9.976 | 1.00 | 31.66 | A | C |
| ATOM | 1460 | O | ARG | A | 223 | -2.190 | 10.879 | -11.203 | 1.00 | 31.66 | A | O |
| ATOM | 1461 | N | ALA | A | 224 | -3.157 | 11.114 | -9.185 | 1.00 | 34.54 | A | N |
| ATOM | 1462 | CA | ALA | A | 224 | -4.512 | 11.288 | -9.706 | 1.00 | 34.54 | A | C |
| ATOM | 1463 | CB | ALA | A | 224 | -5.506 | 11.385 | -8.561 | 1.00 | 40.36 | A | C |
| ATOM | 1464 | C | ALA | A | 224 | -4.610 | 12.533 | -10.569 | 1.00 | 34.54 | A | C |
| ATOM | 1465 | O | ALA | A | 224 | -3.993 | 13.559 | -10.276 | 1.00 | 34.54 | A | O |
| ATOM | 1466 | N | PRO | A | 225 | -5.420 | 12.475 | -11.633 | 1.00 | 41.95 | A | N |
| ATOM | 1467 | CD | PRO | A | 225 | -6.399 | 11.423 | -11.946 | 1.00 | 35.18 | A | C |

| | | | | | | | | | | | | |
|---|---|---|---|---|---|---|---|---|---|---|---|---|
| ATOM | 1468 | CA | PRO | A | 225 | -5.583 | 13.617 | -12.535 | 1.00 | 41.95 | A | C |
| ATOM | 1469 | CB | PRO | A | 225 | -6.548 | 13.076 | -13.587 | 1.00 | 35.18 | A | C |
| ATOM | 1470 | CG | PRO | A | 225 | -7.401 | 12.162 | -12.800 | 1.00 | 35.18 | A | C |
| ATOM | 1471 | C | PRO | A | 225 | -6.059 | 14.909 | -11.849 | 1.00 | 41.95 | A | C |
| ATOM | 1472 | O | PRO | A | 225 | -5.661 | 16.004 | -12.247 | 1.00 | 41.95 | A | O |
| ATOM | 1473 | N | GLU | A | 226 | -6.901 | 14.795 | -10.822 | 1.00 | 50.90 | A | N |
| ATOM | 1474 | CA | GLU | A | 226 | -7.353 | 15.991 | -10.117 | 1.00 | 50.90 | A | C |
| ATOM | 1475 | CB | GLU | A | 226 | -8.106 | 15.654 | -8.839 | 1.00 | 32.83 | A | C |
| ATOM | 1476 | CG | GLU | A | 226 | -9.252 | 14.730 | -9.034 | 1.00 | 32.83 | A | C |
| ATOM | 1477 | CD | GLU | A | 226 | -8.921 | 13.348 | -8.590 | 1.00 | 32.83 | A | C |
| ATOM | 1478 | OE1 | GLU | A | 226 | -9.006 | 13.077 | -7.376 | 1.00 | 32.83 | A | O |
| ATOM | 1479 | OE2 | GLU | A | 226 | -8.561 | 12.538 | -9.459 | 1.00 | 32.83 | A | O |
| ATOM | 1480 | C | GLU | A | 226 | -6.113 | 16.752 | -9.717 | 1.00 | 50.90 | A | C |
| ATOM | 1481 | O | GLU | A | 226 | -5.927 | 17.897 | -10.105 | 1.00 | 50.90 | A | O |
| ATOM | 1482 | N | LEU | A | 227 | -5.260 | 16.089 | -8.942 | 1.00 | 38.71 | A | N |
| ATOM | 1483 | CA | LEU | A | 227 | -4.018 | 16.670 | -8.450 | 1.00 | 38.71 | A | C |
| ATOM | 1484 | CB | LEU | A | 227 | -3.134 | 15.584 | -7.838 | 1.00 | 24.50 | A | C |
| ATOM | 1485 | CG | LEU | A | 227 | -3.754 | 14.754 | -6.722 | 1.00 | 24.50 | A | C |
| ATOM | 1486 | CD1 | LEU | A | 227 | -2.812 | 13.624 | -6.373 | 1.00 | 24.50 | A | C |
| ATOM | 1487 | CD2 | LEU | A | 227 | -4.054 | 15.633 | -5.519 | 1.00 | 24.50 | A | C |
| ATOM | 1488 | C | LEU | A | 227 | -3.216 | 17.421 | -9.505 | 1.00 | 38.71 | A | C |
| ATOM | 1489 | O | LEU | A | 227 | -2.697 | 18.495 | -9.234 | 1.00 | 38.71 | A | O |
| ATOM | 1490 | N | ILE | A | 228 | -3.100 | 16.863 | -10.703 | 1.00 | 34.36 | A | N |
| ATOM | 1491 | CA | ILE | A | 228 | -2.336 | 17.536 | -11.736 | 1.00 | 34.36 | A | C |
| ATOM | 1492 | CB | ILE | A | 228 | -2.254 | 16.705 | -13.013 | 1.00 | 33.07 | A | C |
| ATOM | 1493 | CG2 | ILE | A | 228 | -1.316 | 17.386 | -14.007 | 1.00 | 33.07 | A | C |
| ATOM | 1494 | CG1 | ILE | A | 228 | -1.768 | 15.294 | -12.683 | 1.00 | 33.07 | A | C |
| ATOM | 1495 | CD1 | ILE | A | 228 | -1.729 | 14.380 | -13.878 | 1.00 | 33.07 | A | C |
| ATOM | 1496 | C | ILE | A | 228 | -3.010 | 18.852 | -12.055 | 1.00 | 34.36 | A | C |
| ATOM | 1497 | O | ILE | A | 228 | -2.335 | 19.840 | -12.348 | 1.00 | 34.36 | A | O |
| ATOM | 1498 | N | PHE | A | 229 | -4.346 | 18.847 | -11.991 | 1.00 | 56.03 | A | N |
| ATOM | 1499 | CA | PHE | A | 229 | -5.181 | 20.026 | -12.247 | 1.00 | 56.03 | A | C |
| ATOM | 1500 | CB | PHE | A | 229 | -6.636 | 19.604 | -12.453 | 1.00 | 33.98 | A | C |
| ATOM | 1501 | CG | PHE | A | 229 | -7.003 | 19.354 | -13.884 | 1.00 | 33.98 | A | C |
| ATOM | 1502 | CD1 | PHE | A | 229 | -7.194 | 20.410 | -14.761 | 1.00 | 33.98 | A | C |
| ATOM | 1503 | CD2 | PHE | A | 229 | -7.186 | 18.058 | -14.349 | 1.00 | 33.98 | A | C |
| ATOM | 1504 | CE1 | PHE | A | 229 | -7.563 | 20.176 | -16.074 | 1.00 | 33.98 | A | C |
| ATOM | 1505 | CE2 | PHE | A | 229 | -7.556 | 17.811 | -15.660 | 1.00 | 33.98 | A | C |
| ATOM | 1506 | CZ | PHE | A | 229 | -7.746 | 18.874 | -16.524 | 1.00 | 33.98 | A | C |
| ATOM | 1507 | C | PHE | A | 229 | -5.134 | 21.062 | -11.116 | 1.00 | 56.03 | A | C |
| ATOM | 1508 | O | PHE | A | 229 | -5.856 | 22.059 | -11.156 | 1.00 | 56.03 | A | O |
| ATOM | 1509 | N | GLY | A | 230 | -4.308 | 20.813 | -10.103 | 1.00 | 40.53 | A | N |
| ATOM | 1510 | CA | GLY | A | 230 | -4.180 | 21.752 | -9.001 | 1.00 | 40.53 | A | C |
| ATOM | 1511 | C | GLY | A | 230 | -5.238 | 21.651 | -7.922 | 1.00 | 40.53 | A | C |
| ATOM | 1512 | O | GLY | A | 230 | -5.241 | 22.439 | -6.978 | 1.00 | 40.53 | A | O |
| ATOM | 1513 | N | ALA | A | 231 | -6.134 | 20.683 | -8.055 | 1.00 | 36.52 | A | N |
| ATOM | 1514 | CA | ALA | A | 231 | -7.192 | 20.495 | -7.072 | 1.00 | 36.52 | A | C |
| ATOM | 1515 | CB | ALA | A | 231 | -7.959 | 19.223 | -7.388 | 1.00 | 49.82 | A | C |
| ATOM | 1516 | C | ALA | A | 231 | -6.633 | 20.432 | -5.649 | 1.00 | 36.52 | A | C |
| ATOM | 1517 | O | ALA | A | 231 | -5.671 | 19.707 | -5.379 | 1.00 | 36.52 | A | O |
| ATOM | 1518 | N | THR | A | 232 | -7.230 | 21.190 | -4.736 | 1.00 | 32.87 | A | N |
| ATOM | 1519 | CA | THR | A | 232 | -6.766 | 21.192 | -3.351 | 1.00 | 32.87 | A | C |
| ATOM | 1520 | CB | THR | A | 232 | -6.330 | 22.586 | -2.910 | 1.00 | 46.99 | A | C |
| ATOM | 1521 | OG1 | THR | A | 232 | -7.488 | 23.371 | -2.620 | 1.00 | 46.99 | A | O |
| ATOM | 1522 | CG2 | THR | A | 232 | -5.538 | 23.264 | -4.015 | 1.00 | 46.99 | A | C |
| ATOM | 1523 | C | THR | A | 232 | -7.851 | 20.707 | -2.399 | 1.00 | 32.87 | A | C |
| ATOM | 1524 | O | THR | A | 232 | -7.628 | 20.604 | -1.191 | 1.00 | 32.87 | A | O |
| ATOM | 1525 | N | ASP | A | 233 | -9.026 | 20.412 | -2.950 | 1.00 | 41.01 | A | N |
| ATOM | 1526 | CA | ASP | A | 233 | -10.138 | 19.912 | -2.153 | 1.00 | 41.01 | A | C |
| ATOM | 1527 | CB | ASP | A | 233 | -11.376 | 20.767 | -2.366 | 1.00 | 68.81 | A | C |
| ATOM | 1528 | CG | ASP | A | 233 | -12.025 | 20.500 | -3.706 | 1.00 | 68.81 | A | C |
| ATOM | 1529 | OD1 | ASP | A | 233 | -11.269 | 20.427 | -4.710 | 1.00 | 68.81 | A | O |
| ATOM | 1530 | OD2 | ASP | A | 233 | -13.280 | 20.359 | -3.751 | 1.00 | 68.81 | A | O |
| ATOM | 1531 | C | ASP | A | 233 | -10.442 | 18.497 | -2.613 | 1.00 | 41.01 | A | C |
| ATOM | 1532 | O | ASP | A | 233 | -11.607 | 18.115 | -2.756 | 1.00 | 41.01 | A | O |
| ATOM | 1533 | N | TYR | A | 234 | -9.390 | 17.720 | -2.854 | 1.00 | 55.35 | A | N |
| ATOM | 1534 | CA | TYR | A | 234 | -9.560 | 16.345 | -3.304 | 1.00 | 55.35 | A | C |

| | | | | | | | | | | | | |
|---|---|---|---|---|---|---|---|---|---|---|---|---|
| ATOM | 1535 | CB | TYR | A | 234 | -8.277 | 15.843 | -3.969 | 1.00 | 44.61 | A | C |
| ATOM | 1536 | CG | TYR | A | 234 | -7.033 | 16.004 | -3.134 | 1.00 | 44.61 | A | C |
| ATOM | 1537 | CD1 | TYR | A | 234 | -6.677 | 15.043 | -2.187 | 1.00 | 44.61 | A | C |
| ATOM | 1538 | CE1 | TYR | A | 234 | -5.510 | 15.163 | -1.452 | 1.00 | 44.61 | A | C |
| ATOM | 1539 | CD2 | TYR | A | 234 | -6.184 | 17.102 | -3.316 | 1.00 | 44.61 | A | C |
| ATOM | 1540 | CE2 | TYR | A | 234 | -5.011 | 17.231 | -2.581 | 1.00 | 44.61 | A | C |
| ATOM | 1541 | CZ | TYR | A | 234 | -4.681 | 16.258 | -1.654 | 1.00 | 44.61 | A | C |
| ATOM | 1542 | OH | TYR | A | 234 | -3.521 | 16.378 | -0.926 | 1.00 | 44.61 | A | O |
| ATOM | 1543 | C | TYR | A | 234 | -9.965 | 15.444 | -2.156 | 1.00 | 55.35 | A | C |
| ATOM | 1544 | O | TYR | A | 234 | -9.923 | 15.845 | -0.994 | 1.00 | 55.35 | A | O |
| ATOM | 1545 | N | THR | A | 235 | -10.380 | 14.232 | -2.488 | 1.00 | 40.07 | A | N |
| ATOM | 1546 | CA | THR | A | 235 | -10.812 | 13.271 | -1.484 | 1.00 | 40.07 | A | C |
| ATOM | 1547 | CB | THR | A | 235 | -12.197 | 12.736 | -1.828 | 1.00 | 34.31 | A | C |
| ATOM | 1548 | OG1 | THR | A | 235 | -12.103 | 11.867 | -2.963 | 1.00 | 34.31 | A | O |
| ATOM | 1549 | CG2 | THR | A | 235 | -13.117 | 13.884 | -2.185 | 1.00 | 34.31 | A | C |
| ATOM | 1550 | C | THR | A | 235 | -9.829 | 12.108 | -1.453 | 1.00 | 40.07 | A | C |
| ATOM | 1551 | O | THR | A | 235 | -8.788 | 12.153 | -2.108 | 1.00 | 40.07 | A | O |
| ATOM | 1552 | N | SER | A | 236 | -10.155 | 11.060 | -0.703 | 1.00 | 34.29 | A | N |
| ATOM | 1553 | CA | SER | A | 236 | -9.264 | 9.912 | -0.638 | 1.00 | 34.29 | A | C |
| ATOM | 1554 | CB | SER | A | 236 | -9.531 | 9.086 | 0.621 | 1.00 | 24.86 | A | C |
| ATOM | 1555 | OG | SER | A | 236 | -10.805 | 8.494 | 0.582 | 1.00 | 24.86 | A | O |
| ATOM | 1556 | C | SER | A | 236 | -9.388 | 9.034 | -1.882 | 1.00 | 34.29 | A | C |
| ATOM | 1557 | O | SER | A | 236 | -8.699 | 8.032 | -2.017 | 1.00 | 34.29 | A | O |
| ATOM | 1558 | N | SER | A | 237 | -10.254 | 9.414 | -2.806 | 1.00 | 38.67 | A | N |
| ATOM | 1559 | CA | SER | A | 237 | -10.401 | 8.627 | -4.017 | 1.00 | 38.67 | A | C |
| ATOM | 1560 | CB | SER | A | 237 | -11.604 | 9.104 | -4.838 | 1.00 | 38.35 | A | C |
| ATOM | 1561 | OG | SER | A | 237 | -11.521 | 10.489 | -5.105 | 1.00 | 38.35 | A | O |
| ATOM | 1562 | C | SER | A | 237 | -9.120 | 8.722 | -4.833 | 1.00 | 38.67 | A | C |
| ATOM | 1563 | O | SER | A | 237 | -8.943 | 7.998 | -5.801 | 1.00 | 38.67 | A | O |
| ATOM | 1564 | N | ILE | A | 238 | -8.219 | 9.614 | -4.449 | 1.00 | 25.97 | A | N |
| ATOM | 1565 | CA | ILE | A | 238 | -6.962 | 9.719 | -5.170 | 1.00 | 25.97 | A | C |
| ATOM | 1566 | CB | ILE | A | 238 | -6.126 | 10.918 | -4.685 | 1.00 | 39.07 | A | C |
| ATOM | 1567 | CG2 | ILE | A | 238 | -6.819 | 12.210 | -5.051 | 1.00 | 39.07 | A | C |
| ATOM | 1568 | CG1 | ILE | A | 238 | -5.901 | 10.816 | -3.171 | 1.00 | 39.07 | A | C |
| ATOM | 1569 | CD1 | ILE | A | 238 | -4.869 | 11.770 | -2.640 | 1.00 | 39.07 | A | C |
| ATOM | 1570 | C | ILE | A | 238 | -6.154 | 8.428 | -4.957 | 1.00 | 25.97 | A | C |
| ATOM | 1571 | O | ILE | A | 238 | -5.398 | 8.004 | -5.825 | 1.00 | 25.97 | A | O |
| ATOM | 1572 | N | ASP | A | 239 | -6.307 | 7.801 | -3.795 | 1.00 | 38.94 | A | N |
| ATOM | 1573 | CA | ASP | A | 239 | -5.582 | 6.569 | -3.544 | 1.00 | 38.94 | A | C |
| ATOM | 1574 | CB | ASP | A | 239 | -5.783 | 6.075 | -2.109 | 1.00 | 42.83 | A | C |
| ATOM | 1575 | CG | ASP | A | 239 | -5.004 | 6.880 | -1.094 | 1.00 | 42.83 | A | C |
| ATOM | 1576 | OD1 | ASP | A | 239 | -3.902 | 7.369 | -1.420 | 1.00 | 42.83 | A | O |
| ATOM | 1577 | OD2 | ASP | A | 239 | -5.484 | 7.010 | 0.046 | 1.00 | 42.83 | A | O |
| ATOM | 1578 | C | ASP | A | 239 | -6.079 | 5.511 | -4.504 | 1.00 | 38.94 | A | C |
| ATOM | 1579 | O | ASP | A | 239 | -5.299 | 4.708 | -5.012 | 1.00 | 38.94 | A | O |
| ATOM | 1580 | N | VAL | A | 240 | -7.383 | 5.510 | -4.755 | 1.00 | 36.32 | A | N |
| ATOM | 1581 | CA | VAL | A | 240 | -7.966 | 4.526 | -5.646 | 1.00 | 36.32 | A | C |
| ATOM | 1582 | CB | VAL | A | 240 | -9.485 | 4.578 | -5.597 | 1.00 | 24.73 | A | C |
| ATOM | 1583 | CG1 | VAL | A | 240 | -10.069 | 3.661 | -6.661 | 1.00 | 24.73 | A | C |
| ATOM | 1584 | CG2 | VAL | A | 240 | -9.954 | 4.153 | -4.221 | 1.00 | 24.73 | A | C |
| ATOM | 1585 | C | VAL | A | 240 | -7.477 | 4.715 | -7.072 | 1.00 | 36.32 | A | C |
| ATOM | 1586 | O | VAL | A | 240 | -7.348 | 3.745 | -7.820 | 1.00 | 36.32 | A | O |
| ATOM | 1587 | N | TRP | A | 241 | -7.205 | 5.960 | -7.459 | 1.00 | 47.06 | A | N |
| ATOM | 1588 | CA | TRP | A | 241 | -6.684 | 6.201 | -8.791 | 1.00 | 47.06 | A | C |
| ATOM | 1589 | CB | TRP | A | 241 | -6.601 | 7.695 | -9.093 | 1.00 | 31.46 | A | C |
| ATOM | 1590 | CG | TRP | A | 241 | -5.765 | 8.010 | -10.318 | 1.00 | 31.46 | A | C |
| ATOM | 1591 | CD2 | TRP | A | 241 | -6.234 | 8.203 | -11.664 | 1.00 | 31.46 | A | C |
| ATOM | 1592 | CE2 | TRP | A | 241 | -5.094 | 8.429 | -12.472 | 1.00 | 31.46 | A | C |
| ATOM | 1593 | CE3 | TRP | A | 241 | -7.503 | 8.243 | -12.261 | 1.00 | 31.46 | A | C |
| ATOM | 1594 | CD1 | TRP | A | 241 | -4.404 | 8.103 | -10.375 | 1.00 | 31.46 | A | C |
| ATOM | 1595 | NE1 | TRP | A | 241 | -3.997 | 8.349 | -11.663 | 1.00 | 31.46 | A | N |
| ATOM | 1596 | CZ2 | TRP | A | 241 | -5.188 | 8.644 | -13.852 | 1.00 | 31.46 | A | C |
| ATOM | 1597 | CZ3 | TRP | A | 241 | -7.591 | 8.461 | -13.633 | 1.00 | 31.46 | A | C |
| ATOM | 1598 | CH2 | TRP | A | 241 | -6.438 | 8.673 | -14.407 | 1.00 | 31.46 | A | C |
| ATOM | 1599 | C | TRP | A | 241 | -5.294 | 5.577 | -8.785 | 1.00 | 47.06 | A | C |
| ATOM | 1600 | O | TRP | A | 241 | -4.930 | 4.845 | -9.700 | 1.00 | 47.06 | A | O |
| ATOM | 1601 | N | SER | A | 242 | -4.531 | 5.854 | -7.728 | 1.00 | 42.65 | A | N |

| | | | | | | | | | | | | |
|---|---|---|---|---|---|---|---|---|---|---|---|---|
| ATOM | 1602 | CA | SER | A | 242 | -3.174 | 5.320 | -7.569 | 1.00 | 42.65 | A | C |
| ATOM | 1603 | CB | SER | A | 242 | -2.554 | 5.800 | -6.252 | 1.00 | 32.57 | A | C |
| ATOM | 1604 | OG | SER | A | 242 | -2.418 | 7.200 | -6.221 | 1.00 | 32.57 | A | O |
| ATOM | 1605 | C | SER | A | 242 | -3.175 | 3.798 | -7.573 | 1.00 | 42.65 | A | C |
| ATOM | 1606 | O | SER | A | 242 | -2.269 | 3.177 | -8.115 | 1.00 | 42.65 | A | O |
| ATOM | 1607 | N | ALA | A | 243 | -4.189 | 3.203 | -6.954 | 1.00 | 27.23 | A | N |
| ATOM | 1608 | CA | ALA | A | 243 | -4.291 | 1.756 | -6.892 | 1.00 | 27.23 | A | C |
| ATOM | 1609 | CB | ALA | A | 243 | -5.425 | 1.357 | -5.977 | 1.00 | 17.91 | A | C |
| ATOM | 1610 | C | ALA | A | 243 | -4.516 | 1.198 | -8.286 | 1.00 | 27.23 | A | C |
| ATOM | 1611 | O | ALA | A | 243 | -3.958 | 0.172 | -8.654 | 1.00 | 27.23 | A | O |
| ATOM | 1612 | N | GLY | A | 244 | -5.339 | 1.886 | -9.061 | 1.00 | 30.07 | A | N |
| ATOM | 1613 | CA | GLY | A | 244 | -5.612 | 1.443 | -10.409 | 1.00 | 30.07 | A | C |
| ATOM | 1614 | C | GLY | A | 244 | -4.408 | 1.586 | -11.318 | 1.00 | 30.07 | A | C |
| ATOM | 1615 | O | GLY | A | 244 | -4.352 | 0.961 | -12.372 | 1.00 | 30.07 | A | O |
| ATOM | 1616 | N | CYS | A | 245 | -3.450 | 2.421 | -10.925 | 1.00 | 35.94 | A | N |
| ATOM | 1617 | CA | CYS | A | 245 | -2.238 | 2.613 | -11.719 | 1.00 | 35.94 | A | C |
| ATOM | 1618 | CB | CYS | A | 245 | -1.577 | 3.959 | -11.375 | 1.00 | 32.20 | A | C |
| ATOM | 1619 | SG | CYS | A | 245 | -2.342 | 5.417 | -12.174 | 1.00 | 32.20 | A | S |
| ATOM | 1620 | C | CYS | A | 245 | -1.265 | 1.452 | -11.480 | 1.00 | 35.94 | A | C |
| ATOM | 1621 | O | CYS | A | 245 | -0.475 | 1.092 | -12.356 | 1.00 | 35.94 | A | O |
| ATOM | 1622 | N | VAL | A | 246 | -1.342 | 0.867 | -10.287 | 1.00 | 43.26 | A | N |
| ATOM | 1623 | CA | VAL | A | 246 | -0.509 | -0.269 | -9.920 | 1.00 | 43.26 | A | C |
| ATOM | 1624 | CB | VAL | A | 246 | -0.550 | -0.539 | -8.394 | 1.00 | 42.20 | A | C |
| ATOM | 1625 | CG1 | VAL | A | 246 | 0.225 | -1.799 | -8.070 | 1.00 | 42.20 | A | C |
| ATOM | 1626 | CG2 | VAL | A | 246 | 0.014 | 0.639 | -7.636 | 1.00 | 42.20 | A | C |
| ATOM | 1627 | C | VAL | A | 246 | -1.051 | -1.501 | -10.636 | 1.00 | 43.26 | A | C |
| ATOM | 1628 | O | VAL | A | 246 | -0.293 | -2.288 | -11.189 | 1.00 | 43.26 | A | O |
| ATOM | 1629 | N | LEU | A | 247 | -2.367 | -1.669 | -10.614 | 1.00 | 40.13 | A | N |
| ATOM | 1630 | CA | LEU | A | 247 | -2.981 | -2.800 | -11.275 | 1.00 | 40.13 | A | C |
| ATOM | 1631 | CB | LEU | A | 247 | -4.484 | -2.787 | -11.053 | 1.00 | 34.27 | A | C |
| ATOM | 1632 | CG | LEU | A | 247 | -5.269 | -3.769 | -11.925 | 1.00 | 34.27 | A | C |
| ATOM | 1633 | CD1 | LEU | A | 247 | -4.762 | -5.170 | -11.672 | 1.00 | 34.27 | A | C |
| ATOM | 1634 | CD2 | LEU | A | 247 | -6.754 | -3.662 | -11.632 | 1.00 | 34.27 | A | C |
| ATOM | 1635 | C | LEU | A | 247 | -2.692 | -2.765 | -12.772 | 1.00 | 40.13 | A | C |
| ATOM | 1636 | O | LEU | A | 247 | -2.270 | -3.754 | -13.357 | 1.00 | 40.13 | A | O |
| ATOM | 1637 | N | ALA | A | 248 | -2.924 | -1.624 | -13.400 | 1.00 | 30.55 | A | N |
| ATOM | 1638 | CA | ALA | A | 248 | -2.667 | -1.515 | -14.822 | 1.00 | 30.55 | A | C |
| ATOM | 1639 | CB | ALA | A | 248 | -2.931 | -0.087 | -15.290 | 1.00 | 18.90 | A | C |
| ATOM | 1640 | C | ALA | A | 248 | -1.224 | -1.925 | -15.128 | 1.00 | 30.55 | A | C |
| ATOM | 1641 | O | ALA | A | 248 | -0.964 | -2.655 | -16.078 | 1.00 | 30.55 | A | O |
| ATOM | 1642 | N | GLU | A | 249 | -0.295 | -1.461 | -14.300 | 1.00 | 38.22 | A | N |
| ATOM | 1643 | CA | GLU | A | 249 | 1.128 | -1.740 | -14.470 | 1.00 | 38.22 | A | C |
| ATOM | 1644 | CB | GLU | A | 249 | 1.932 | -0.954 | -13.440 | 1.00 | 49.27 | A | C |
| ATOM | 1645 | CG | GLU | A | 249 | 3.409 | -0.961 | -13.742 | 1.00 | 49.27 | A | C |
| ATOM | 1646 | CD | GLU | A | 249 | 4.212 | -0.074 | -12.812 | 1.00 | 49.27 | A | C |
| ATOM | 1647 | OE1 | GLU | A | 249 | 3.781 | 1.081 | -12.586 | 1.00 | 49.27 | A | O |
| ATOM | 1648 | OE2 | GLU | A | 249 | 5.277 | -0.535 | -12.327 | 1.00 | 49.27 | A | O |
| ATOM | 1649 | C | GLU | A | 249 | 1.487 | -3.215 | -14.350 | 1.00 | 38.22 | A | C |
| ATOM | 1650 | O | GLU | A | 249 | 2.396 | -3.708 | -15.017 | 1.00 | 38.22 | A | O |
| ATOM | 1651 | N | LEU | A | 250 | 0.770 | -3.918 | -13.487 | 1.00 | 30.95 | A | N |
| ATOM | 1652 | CA | LEU | A | 250 | 1.029 | -5.324 | -13.275 | 1.00 | 30.95 | A | C |
| ATOM | 1653 | CB | LEU | A | 250 | 0.389 | -5.768 | -11.971 | 1.00 | 23.60 | A | C |
| ATOM | 1654 | CG | LEU | A | 250 | 1.018 | -5.049 | -10.782 | 1.00 | 23.60 | A | C |
| ATOM | 1655 | CD1 | LEU | A | 250 | 0.232 | -5.374 | -9.532 | 1.00 | 23.60 | A | C |
| ATOM | 1656 | CD2 | LEU | A | 250 | 2.479 | -5.453 | -10.634 | 1.00 | 23.60 | A | C |
| ATOM | 1657 | C | LEU | A | 250 | 0.542 | -6.175 | -14.422 | 1.00 | 30.95 | A | C |
| ATOM | 1658 | O | LEU | A | 250 | 1.096 | -7.237 | -14.678 | 1.00 | 30.95 | A | O |
| ATOM | 1659 | N | LEU | A | 251 | -0.497 | -5.712 | -15.107 | 1.00 | 30.22 | A | N |
| ATOM | 1660 | CA | LEU | A | 251 | -1.056 | -6.425 | -16.251 | 1.00 | 30.22 | A | C |
| ATOM | 1661 | CB | LEU | A | 251 | -2.522 | -6.049 | -16.463 | 1.00 | 25.42 | A | C |
| ATOM | 1662 | CG | LEU | A | 251 | -3.543 | -6.144 | -15.338 | 1.00 | 25.42 | A | C |
| ATOM | 1663 | CD1 | LEU | A | 251 | -4.875 | -5.676 | -15.872 | 1.00 | 25.42 | A | C |
| ATOM | 1664 | CD2 | LEU | A | 251 | -3.644 | -7.560 | -14.820 | 1.00 | 25.42 | A | C |
| ATOM | 1665 | C | LEU | A | 251 | -0.282 | -6.004 | -17.490 | 1.00 | 30.22 | A | C |
| ATOM | 1666 | O | LEU | A | 251 | -0.222 | -6.726 | -18.482 | 1.00 | 30.22 | A | O |
| ATOM | 1667 | N | LEU | A | 252 | 0.302 | -4.814 | -17.410 | 1.00 | 41.77 | A | N |
| ATOM | 1668 | CA | LEU | A | 252 | 1.050 | -4.214 | -18.499 | 1.00 | 41.77 | A | C |

| | | | | | | | | | | | | |
|---|---|---|---|---|---|---|---|---|---|---|---|---|
| ATOM | 1669 | CB | LEU | A | 252 | 0.843 | -2.703 | -18.479 | 1.00 | 49.78 | A | C |
| ATOM | 1670 | CG | LEU | A | 252 | 0.202 | -2.040 | -19.691 | 1.00 | 49.78 | A | C |
| ATOM | 1671 | CD1 | LEU | A | 252 | -1.139 | -2.687 | -20.006 | 1.00 | 49.78 | A | C |
| ATOM | 1672 | CD2 | LEU | A | 252 | 0.024 | -0.564 | -19.398 | 1.00 | 49.78 | A | C |
| ATOM | 1673 | C | LEU | A | 252 | 2.538 | -4.499 | -18.484 | 1.00 | 41.77 | A | C |
| ATOM | 1674 | O | LEU | A | 252 | 3.169 | -4.534 | -19.537 | 1.00 | 41.77 | A | O |
| ATOM | 1675 | N | GLY | A | 253 | 3.111 | -4.680 | -17.303 | 1.00 | 36.25 | A | N |
| ATOM | 1676 | CA | GLY | A | 253 | 4.534 | -4.942 | -17.237 | 1.00 | 36.25 | A | C |
| ATOM | 1677 | C | GLY | A | 253 | 5.355 | -3.662 | -17.242 | 1.00 | 36.25 | A | C |
| ATOM | 1678 | O | GLY | A | 253 | 6.585 | -3.694 | -17.229 | 1.00 | 36.25 | A | O |
| ATOM | 1679 | N | GLN | A | 254 | 4.673 | -2.525 | -17.270 | 1.00 | 48.82 | A | N |
| ATOM | 1680 | CA | GLN | A | 254 | 5.333 | -1.223 | -17.256 | 1.00 | 48.82 | A | C |
| ATOM | 1681 | CB | GLN | A | 254 | 5.859 | -0.891 | -18.647 | 1.00 | 52.67 | A | C |
| ATOM | 1682 | CG | GLN | A | 254 | 4.817 | -1.056 | -19.733 | 1.00 | 52.67 | A | C |
| ATOM | 1683 | CD | GLN | A | 254 | 5.193 | -0.339 | -21.018 | 1.00 | 52.67 | A | C |
| ATOM | 1684 | OE1 | GLN | A | 254 | 5.131 | 0.894 | -21.105 | 1.00 | 52.67 | A | O |
| ATOM | 1685 | NE2 | GLN | A | 254 | 5.592 | -1.106 | -22.024 | 1.00 | 52.67 | A | N |
| ATOM | 1686 | C | GLN | A | 254 | 4.316 | -0.165 | -16.802 | 1.00 | 48.82 | A | C |
| ATOM | 1687 | O | GLN | A | 254 | 3.108 | -0.362 | -16.939 | 1.00 | 48.82 | A | O |
| ATOM | 1688 | N | PRO | A | 255 | 4.786 | 0.958 | -16.237 | 1.00 | 45.25 | A | N |
| ATOM | 1689 | CD | PRO | A | 255 | 6.166 | 1.358 | -15.916 | 1.00 | 51.96 | A | C |
| ATOM | 1690 | CA | PRO | A | 255 | 3.834 | 1.980 | -15.798 | 1.00 | 45.25 | A | C |
| ATOM | 1691 | CB | PRO | A | 255 | 4.745 | 3.134 | -15.403 | 1.00 | 51.96 | A | C |
| ATOM | 1692 | CG | PRO | A | 255 | 5.950 | 2.431 | -14.874 | 1.00 | 51.96 | A | C |
| ATOM | 1693 | C | PRO | A | 255 | 2.866 | 2.351 | -16.920 | 1.00 | 45.25 | A | C |
| ATOM | 1694 | O | PRO | A | 255 | 3.275 | 2.500 | -18.068 | 1.00 | 45.25 | A | O |
| ATOM | 1695 | N | ILE | A | 256 | 1.588 | 2.487 | -16.594 | 1.00 | 29.72 | A | N |
| ATOM | 1696 | CA | ILE | A | 256 | 0.584 | 2.830 | -17.589 | 1.00 | 29.72 | A | C |
| ATOM | 1697 | CB | ILE | A | 256 | -0.844 | 2.578 | -17.028 | 1.00 | 40.28 | A | C |
| ATOM | 1698 | CG2 | ILE | A | 256 | -1.035 | 3.303 | -15.711 | 1.00 | 40.28 | A | C |
| ATOM | 1699 | CG1 | ILE | A | 256 | -1.903 | 3.026 | -18.030 | 1.00 | 40.28 | A | C |
| ATOM | 1700 | CD1 | ILE | A | 256 | -2.098 | 2.081 | -19.154 | 1.00 | 40.28 | A | C |
| ATOM | 1701 | C | ILE | A | 256 | 0.718 | 4.279 | -18.073 | 1.00 | 29.72 | A | C |
| ATOM | 1702 | O | ILE | A | 256 | 0.599 | 4.549 | -19.263 | 1.00 | 29.72 | A | O |
| ATOM | 1703 | N | PHE | A | 257 | 0.969 | 5.211 | -17.160 | 1.00 | 35.25 | A | N |
| ATOM | 1704 | CA | PHE | A | 257 | 1.127 | 6.620 | -17.532 | 1.00 | 35.25 | A | C |
| ATOM | 1705 | CB | PHE | A | 257 | 0.034 | 7.493 | -16.901 | 1.00 | 33.99 | A | C |
| ATOM | 1706 | CG | PHE | A | 257 | -1.362 | 6.980 | -17.092 | 1.00 | 33.99 | A | C |
| ATOM | 1707 | CD1 | PHE | A | 257 | -1.867 | 6.738 | -18.361 | 1.00 | 33.99 | A | C |
| ATOM | 1708 | CD2 | PHE | A | 257 | -2.182 | 6.752 | -15.992 | 1.00 | 33.99 | A | C |
| ATOM | 1709 | CE1 | PHE | A | 257 | -3.172 | 6.272 | -18.530 | 1.00 | 33.99 | A | C |
| ATOM | 1710 | CE2 | PHE | A | 257 | -3.482 | 6.289 | -16.151 | 1.00 | 33.99 | A | C |
| ATOM | 1711 | CZ | PHE | A | 257 | -3.980 | 6.047 | -17.426 | 1.00 | 33.99 | A | C |
| ATOM | 1712 | C | PHE | A | 257 | 2.480 | 7.142 | -17.050 | 1.00 | 35.25 | A | C |
| ATOM | 1713 | O | PHE | A | 257 | 2.585 | 7.644 | -15.935 | 1.00 | 35.25 | A | O |
| ATOM | 1714 | N | PRO | A | 258 | 3.525 | 7.041 | -17.883 | 1.00 | 36.00 | A | N |
| ATOM | 1715 | CD | PRO | A | 258 | 3.540 | 6.230 | -19.117 | 1.00 | 39.52 | A | C |
| ATOM | 1716 | CA | PRO | A | 258 | 4.883 | 7.496 | -17.554 | 1.00 | 36.00 | A | C |
| ATOM | 1717 | CB | PRO | A | 258 | 5.749 | 6.499 | -18.306 | 1.00 | 39.52 | A | C |
| ATOM | 1718 | CG | PRO | A | 258 | 4.983 | 6.367 | -19.590 | 1.00 | 39.52 | A | C |
| ATOM | 1719 | C | PRO | A | 258 | 5.214 | 8.937 | -17.958 | 1.00 | 36.00 | A | C |
| ATOM | 1720 | O | PRO | A | 258 | 6.059 | 9.161 | -18.822 | 1.00 | 36.00 | A | O |
| ATOM | 1721 | N | GLY | A | 259 | 4.567 | 9.909 | -17.331 | 1.00 | 49.21 | A | N |
| ATOM | 1722 | CA | GLY | A | 259 | 4.833 | 11.297 | -17.678 | 1.00 | 49.21 | A | C |
| ATOM | 1723 | C | GLY | A | 259 | 6.267 | 11.723 | -17.406 | 1.00 | 49.21 | A | C |
| ATOM | 1724 | O | GLY | A | 259 | 6.852 | 11.309 | -16.397 | 1.00 | 49.21 | A | O |
| ATOM | 1725 | N | ASP | A | 260 | 6.851 | 12.544 | -18.282 | 1.00 | 50.37 | A | N |
| ATOM | 1726 | CA | ASP | A | 260 | 8.229 | 12.976 | -18.040 | 1.00 | 50.37 | A | C |
| ATOM | 1727 | CB | ASP | A | 260 | 8.957 | 13.314 | -19.352 | 1.00 | 49.10 | A | C |
| ATOM | 1728 | CG | ASP | A | 260 | 8.456 | 14.591 | -19.993 | 1.00 | 49.10 | A | C |
| ATOM | 1729 | OD1 | ASP | A | 260 | 8.033 | 15.498 | -19.239 | 1.00 | 49.10 | A | O |
| ATOM | 1730 | OD2 | ASP | A | 260 | 8.505 | 14.697 | -21.244 | 1.00 | 49.10 | A | O |
| ATOM | 1731 | C | ASP | A | 260 | 8.263 | 14.171 | -17.087 | 1.00 | 50.37 | A | C |
| ATOM | 1732 | O | ASP | A | 260 | 9.325 | 14.617 | -16.670 | 1.00 | 50.37 | A | O |
| ATOM | 1733 | N | SER | A | 261 | 7.088 | 14.688 | -16.751 | 1.00 | 42.96 | A | N |
| ATOM | 1734 | CA | SER | A | 261 | 6.987 | 15.813 | -15.835 | 1.00 | 42.96 | A | C |
| ATOM | 1735 | CB | SER | A | 261 | 7.336 | 17.114 | -16.545 | 1.00 | 45.55 | A | C |

| | | | | | | | | | | | | |
|---|---|---|---|---|---|---|---|---|---|---|---|---|
| ATOM | 1736 | OG | SER | A | 261 | 6.269 | 17.511 | -17.379 | 1.00 | 45.55 | A | O |
| ATOM | 1737 | C | SER | A | 261 | 5.566 | 15.895 | -15.284 | 1.00 | 42.96 | A | C |
| ATOM | 1738 | O | SER | A | 261 | 4.647 | 15.281 | -15.821 | 1.00 | 42.96 | A | O |
| ATOM | 1739 | N | GLY | A | 262 | 5.398 | 16.650 | -14.200 | 1.00 | 60.21 | A | N |
| ATOM | 1740 | CA | GLY | A | 262 | 4.092 | 16.796 | -13.583 | 1.00 | 60.21 | A | C |
| ATOM | 1741 | C | GLY | A | 262 | 2.970 | 17.101 | -14.562 | 1.00 | 60.21 | A | C |
| ATOM | 1742 | O | GLY | A | 262 | 1.832 | 16.695 | -14.339 | 1.00 | 60.21 | A | O |
| ATOM | 1743 | N | VAL | A | 263 | 3.282 | 17.812 | -15.644 | 1.00 | 49.75 | A | N |
| ATOM | 1744 | CA | VAL | A | 263 | 2.276 | 18.165 | -16.642 | 1.00 | 49.75 | A | C |
| ATOM | 1745 | CB | VAL | A | 263 | 2.548 | 19.585 | -17.229 | 1.00 | 52.93 | A | C |
| ATOM | 1746 | CG1 | VAL | A | 263 | 1.633 | 19.846 | -18.431 | 1.00 | 52.93 | A | C |
| ATOM | 1747 | CG2 | VAL | A | 263 | 2.316 | 20.647 | -16.150 | 1.00 | 52.93 | A | C |
| ATOM | 1748 | C | VAL | A | 263 | 2.196 | 17.136 | -17.775 | 1.00 | 49.75 | A | C |
| ATOM | 1749 | O | VAL | A | 263 | 1.109 | 16.823 | -18.250 | 1.00 | 49.75 | A | O |
| ATOM | 1750 | N | ASP | A | 264 | 3.341 | 16.618 | -18.211 | 1.00 | 56.04 | A | N |
| ATOM | 1751 | CA | ASP | A | 264 | 3.368 | 15.605 | -19.269 | 1.00 | 56.04 | A | C |
| ATOM | 1752 | CB | ASP | A | 264 | 4.820 | 15.137 | -19.511 | 1.00 | 69.02 | A | C |
| ATOM | 1753 | CG | ASP | A | 264 | 4.947 | 14.119 | -20.659 | 1.00 | 69.02 | A | C |
| ATOM | 1754 | OD1 | ASP | A | 264 | 5.907 | 13.315 | -20.620 | 1.00 | 69.02 | A | O |
| ATOM | 1755 | OD2 | ASP | A | 264 | 4.109 | 14.127 | -21.601 | 1.00 | 69.02 | A | O |
| ATOM | 1756 | C | ASP | A | 264 | 2.514 | 14.422 | -18.773 | 1.00 | 56.04 | A | C |
| ATOM | 1757 | O | ASP | A | 264 | 1.884 | 13.699 | -19.559 | 1.00 | 56.04 | A | O |
| ATOM | 1758 | N | GLN | A | 265 | 2.496 | 14.250 | -17.452 | 1.00 | 51.13 | A | N |
| ATOM | 1759 | CA | GLN | A | 265 | 1.754 | 13.177 | -16.808 | 1.00 | 51.13 | A | C |
| ATOM | 1760 | CB | GLN | A | 265 | 1.807 | 13.341 | -15.286 | 1.00 | 33.26 | A | C |
| ATOM | 1761 | CG | GLN | A | 265 | 1.176 | 12.196 | -14.506 | 1.00 | 33.26 | A | C |
| ATOM | 1762 | CD | GLN | A | 265 | 1.738 | 10.860 | -14.914 | 1.00 | 33.26 | A | C |
| ATOM | 1763 | OE1 | GLN | A | 265 | 2.888 | 10.771 | -15.354 | 1.00 | 33.26 | A | O |
| ATOM | 1764 | NE2 | GLN | A | 265 | 0.942 | 9.806 | -14.762 | 1.00 | 33.26 | A | N |
| ATOM | 1765 | C | GLN | A | 265 | 0.312 | 13.191 | -17.270 | 1.00 | 51.13 | A | C |
| ATOM | 1766 | O | GLN | A | 265 | -0.258 | 12.149 | -17.604 | 1.00 | 51.13 | A | O |
| ATOM | 1767 | N | LEU | A | 266 | -0.279 | 14.379 | -17.276 | 1.00 | 43.43 | A | N |
| ATOM | 1768 | CA | LEU | A | 266 | -1.653 | 14.527 | -17.698 | 1.00 | 43.43 | A | C |
| ATOM | 1769 | CB | LEU | A | 266 | -2.078 | 15.979 | -17.516 | 1.00 | 40.66 | A | C |
| ATOM | 1770 | CG | LEU | A | 266 | -3.545 | 16.365 | -17.701 | 1.00 | 40.66 | A | C |
| ATOM | 1771 | CD1 | LEU | A | 266 | -4.461 | 15.407 | -16.924 | 1.00 | 40.66 | A | C |
| ATOM | 1772 | CD2 | LEU | A | 266 | -3.725 | 17.801 | -17.220 | 1.00 | 40.66 | A | C |
| ATOM | 1773 | C | LEU | A | 266 | -1.768 | 14.093 | -19.159 | 1.00 | 43.43 | A | C |
| ATOM | 1774 | O | LEU | A | 266 | -2.685 | 13.353 | -19.528 | 1.00 | 43.43 | A | O |
| ATOM | 1775 | N | VAL | A | 267 | -0.824 | 14.535 | -19.985 | 1.00 | 42.78 | A | N |
| ATOM | 1776 | CA | VAL | A | 267 | -0.837 | 14.178 | -21.397 | 1.00 | 42.78 | A | C |
| ATOM | 1777 | CB | VAL | A | 267 | 0.411 | 14.718 | -22.134 | 1.00 | 29.31 | A | C |
| ATOM | 1778 | CG1 | VAL | A | 267 | 0.379 | 14.289 | -23.590 | 1.00 | 29.31 | A | C |
| ATOM | 1779 | CG2 | VAL | A | 267 | 0.458 | 16.231 | -22.030 | 1.00 | 29.31 | A | C |
| ATOM | 1780 | C | VAL | A | 267 | -0.898 | 12.664 | -21.567 | 1.00 | 42.78 | A | C |
| ATOM | 1781 | O | VAL | A | 267 | -1.787 | 12.147 | -22.244 | 1.00 | 42.78 | A | O |
| ATOM | 1782 | N | GLU | A | 268 | 0.045 | 11.958 | -20.950 | 1.00 | 45.75 | A | N |
| ATOM | 1783 | CA | GLU | A | 268 | 0.082 | 10.499 | -21.035 | 1.00 | 45.75 | A | C |
| ATOM | 1784 | CB | GLU | A | 268 | 1.179 | 9.940 | -20.117 | 1.00 | 47.62 | A | C |
| ATOM | 1785 | CG | GLU | A | 268 | 2.603 | 10.221 | -20.579 | 1.00 | 47.62 | A | C |
| ATOM | 1786 | CD | GLU | A | 268 | 2.938 | 9.542 | -21.895 | 1.00 | 47.62 | A | C |
| ATOM | 1787 | OE1 | GLU | A | 268 | 2.779 | 8.307 | -21.990 | 1.00 | 47.62 | A | O |
| ATOM | 1788 | OE2 | GLU | A | 268 | 3.365 | 10.242 | -22.836 | 1.00 | 47.62 | A | O |
| ATOM | 1789 | C | GLU | A | 268 | -1.269 | 9.884 | -20.661 | 1.00 | 45.75 | A | C |
| ATOM | 1790 | O | GLU | A | 268 | -1.734 | 8.964 | -21.322 | 1.00 | 45.75 | A | O |
| ATOM | 1791 | N | ILE | A | 269 | -1.879 | 10.396 | -19.593 | 1.00 | 43.94 | A | N |
| ATOM | 1792 | CA | ILE | A | 269 | -3.181 | 9.927 | -19.114 | 1.00 | 43.94 | A | C |
| ATOM | 1793 | CB | ILE | A | 269 | -3.649 | 10.728 | -17.865 | 1.00 | 34.06 | A | C |
| ATOM | 1794 | CG2 | ILE | A | 269 | -5.154 | 10.566 | -17.672 | 1.00 | 34.06 | A | C |
| ATOM | 1795 | CG1 | ILE | A | 269 | -2.888 | 10.269 | -16.620 | 1.00 | 34.06 | A | C |
| ATOM | 1796 | CD1 | ILE | A | 269 | -3.280 | 11.012 | -15.353 | 1.00 | 34.06 | A | C |
| ATOM | 1797 | C | ILE | A | 269 | -4.218 | 10.123 | -20.203 | 1.00 | 43.94 | A | C |
| ATOM | 1798 | O | ILE | A | 269 | -4.976 | 9.212 | -20.536 | 1.00 | 43.94 | A | O |
| ATOM | 1799 | N | ILE | A | 270 | -4.249 | 11.337 | -20.736 | 1.00 | 44.97 | A | N |
| ATOM | 1800 | CA | ILE | A | 270 | -5.175 | 11.706 | -21.799 | 1.00 | 44.97 | A | C |
| ATOM | 1801 | CB | ILE | A | 270 | -4.986 | 13.201 | -22.188 | 1.00 | 35.31 | A | C |
| ATOM | 1802 | CG2 | ILE | A | 270 | -5.629 | 13.490 | -23.537 | 1.00 | 35.31 | A | C |

| | | | | | | | | | | | | |
|---|---|---|---|---|---|---|---|---|---|---|---|---|
| ATOM | 1803 | CG1 | ILE | A | 270 | -5.573 | 14.089 | -21.086 | 1.00 | 35.31 | A | C |
| ATOM | 1804 | CD1 | ILE | A | 270 | -5.258 | 15.551 | -21.238 | 1.00 | 35.31 | A | C |
| ATOM | 1805 | C | ILE | A | 270 | -4.996 | 10.830 | -23.040 | 1.00 | 44.97 | A | C |
| ATOM | 1806 | O | ILE | A | 270 | -5.941 | 10.619 | -23.794 | 1.00 | 44.97 | A | O |
| ATOM | 1807 | N | LYS | A | 271 | -3.789 | 10.310 | -23.239 | 1.00 | 39.25 | A | N |
| ATOM | 1808 | CA | LYS | A | 271 | -3.515 | 9.471 | -24.396 | 1.00 | 39.25 | A | C |
| ATOM | 1809 | CB | LYS | A | 271 | -2.012 | 9.211 | -24.508 | 1.00 | 39.58 | A | C |
| ATOM | 1810 | CG | LYS | A | 271 | -1.244 | 10.483 | -24.776 | 1.00 | 39.58 | A | C |
| ATOM | 1811 | CD | LYS | A | 271 | 0.249 | 10.301 | -24.667 | 1.00 | 39.58 | A | C |
| ATOM | 1812 | CE | LYS | A | 271 | 0.783 | 9.397 | -25.754 | 1.00 | 39.58 | A | C |
| ATOM | 1813 | NZ | LYS | A | 271 | 2.271 | 9.258 | -25.682 | 1.00 | 39.58 | A | N |
| ATOM | 1814 | C | LYS | A | 271 | -4.296 | 8.159 | -24.405 | 1.00 | 39.25 | A | C |
| ATOM | 1815 | O | LYS | A | 271 | -4.505 | 7.570 | -25.463 | 1.00 | 39.25 | A | O |
| ATOM | 1816 | N | VAL | A | 272 | -4.741 | 7.698 | -23.245 | 1.00 | 37.32 | A | N |
| ATOM | 1817 | CA | VAL | A | 272 | -5.510 | 6.467 | -23.229 | 1.00 | 37.32 | A | C |
| ATOM | 1818 | CB | VAL | A | 272 | -4.910 | 5.402 | -22.244 | 1.00 | 35.30 | A | C |
| ATOM | 1819 | CG1 | VAL | A | 272 | -3.400 | 5.388 | -22.330 | 1.00 | 35.30 | A | C |
| ATOM | 1820 | CG2 | VAL | A | 272 | -5.353 | 5.672 | -20.844 | 1.00 | 35.30 | A | C |
| ATOM | 1821 | C | VAL | A | 272 | -6.962 | 6.777 | -22.856 | 1.00 | 37.32 | A | C |
| ATOM | 1822 | O | VAL | A | 272 | -7.891 | 6.286 | -23.487 | 1.00 | 37.32 | A | O |
| ATOM | 1823 | N | LEU | A | 273 | -7.162 | 7.608 | -21.842 | 1.00 | 49.24 | A | N |
| ATOM | 1824 | CA | LEU | A | 273 | -8.518 | 7.943 | -21.420 | 1.00 | 49.24 | A | C |
| ATOM | 1825 | CB | LEU | A | 273 | -8.515 | 8.522 | -19.994 | 1.00 | 43.30 | A | C |
| ATOM | 1826 | CG | LEU | A | 273 | -7.910 | 7.756 | -18.804 | 1.00 | 43.30 | A | C |
| ATOM | 1827 | CD1 | LEU | A | 273 | -8.360 | 8.459 | -17.525 | 1.00 | 43.30 | A | C |
| ATOM | 1828 | CD2 | LEU | A | 273 | -8.352 | 6.295 | -18.779 | 1.00 | 43.30 | A | C |
| ATOM | 1829 | C | LEU | A | 273 | -9.176 | 8.949 | -22.371 | 1.00 | 49.24 | A | C |
| ATOM | 1830 | O | LEU | A | 273 | -10.405 | 9.064 | -22.419 | 1.00 | 49.24 | A | O |
| ATOM | 1831 | N | GLY | A | 274 | -8.355 | 9.671 | -23.126 | 1.00 | 45.66 | A | N |
| ATOM | 1832 | CA | GLY | A | 274 | -8.882 | 10.670 | -24.038 | 1.00 | 45.66 | A | C |
| ATOM | 1833 | C | GLY | A | 274 | -9.126 | 11.989 | -23.326 | 1.00 | 45.66 | A | C |
| ATOM | 1834 | O | GLY | A | 274 | -9.197 | 12.018 | -22.100 | 1.00 | 45.66 | A | O |
| ATOM | 1835 | N | THR | A | 275 | -9.245 | 13.083 | -24.075 | 1.00 | 39.05 | A | N |
| ATOM | 1836 | CA | THR | A | 275 | -9.484 | 14.396 | -23.476 | 1.00 | 39.05 | A | C |
| ATOM | 1837 | CB | THR | A | 275 | -9.758 | 15.443 | -24.551 | 1.00 | 28.30 | A | C |
| ATOM | 1838 | OG1 | THR | A | 275 | -8.667 | 15.475 | -25.475 | 1.00 | 28.30 | A | O |
| ATOM | 1839 | CG2 | THR | A | 275 | -9.914 | 16.804 | -23.926 | 1.00 | 28.30 | A | C |
| ATOM | 1840 | C | THR | A | 275 | -10.676 | 14.355 | -22.516 | 1.00 | 39.05 | A | C |
| ATOM | 1841 | O | THR | A | 275 | -11.714 | 13.777 | -22.834 | 1.00 | 39.05 | A | O |
| ATOM | 1842 | N | PRO | A | 276 | -10.548 | 14.976 | -21.327 | 1.00 | 41.23 | A | N |
| ATOM | 1843 | CD | PRO | A | 276 | -9.427 | 15.780 | -20.819 | 1.00 | 28.41 | A | C |
| ATOM | 1844 | CA | PRO | A | 276 | -11.652 | 14.968 | -20.360 | 1.00 | 41.23 | A | C |
| ATOM | 1845 | CB | PRO | A | 276 | -10.989 | 15.441 | -19.062 | 1.00 | 28.41 | A | C |
| ATOM | 1846 | CG | PRO | A | 276 | -9.510 | 15.514 | -19.364 | 1.00 | 28.41 | A | C |
| ATOM | 1847 | C | PRO | A | 276 | -12.767 | 15.910 | -20.789 | 1.00 | 41.23 | A | C |
| ATOM | 1848 | O | PRO | A | 276 | -12.496 | 16.979 | -21.331 | 1.00 | 41.23 | A | O |
| ATOM | 1849 | N | THR | A | 277 | -14.017 | 15.529 | -20.546 | 1.00 | 38.31 | A | N |
| ATOM | 1850 | CA | THR | A | 277 | -15.139 | 16.387 | -20.921 | 1.00 | 38.31 | A | C |
| ATOM | 1851 | CB | THR | A | 277 | -16.492 | 15.677 | -20.749 | 1.00 | 27.13 | A | C |
| ATOM | 1852 | OG1 | THR | A | 277 | -16.866 | 15.678 | -19.367 | 1.00 | 27.13 | A | O |
| ATOM | 1853 | CG2 | THR | A | 277 | -16.401 | 14.249 | -21.233 | 1.00 | 27.13 | A | C |
| ATOM | 1854 | C | THR | A | 277 | -15.127 | 17.620 | -20.030 | 1.00 | 38.31 | A | C |
| ATOM | 1855 | O | THR | A | 277 | -14.623 | 17.578 | -18.914 | 1.00 | 38.31 | A | O |
| ATOM | 1856 | N | ARG | A | 278 | -15.658 | 18.722 | -20.546 | 1.00 | 37.70 | A | N |
| ATOM | 1857 | CA | ARG | A | 278 | -15.720 | 19.983 | -19.808 | 1.00 | 37.70 | A | C |
| ATOM | 1858 | CB | ARG | A | 278 | -16.673 | 20.957 | -20.513 | 1.00 | 49.87 | A | C |
| ATOM | 1859 | CG | ARG | A | 278 | -16.755 | 22.342 | -19.863 | 1.00 | 49.87 | A | C |
| ATOM | 1860 | CD | ARG | A | 278 | -17.913 | 23.183 | -20.415 | 1.00 | 49.87 | A | C |
| ATOM | 1861 | NE | ARG | A | 278 | -17.846 | 23.348 | -21.867 | 1.00 | 49.87 | A | N |
| ATOM | 1862 | CZ | ARG | A | 278 | -18.914 | 23.415 | -22.655 | 1.00 | 49.87 | A | C |
| ATOM | 1863 | NH1 | ARG | A | 278 | -20.131 | 23.335 | -22.133 | 1.00 | 49.87 | A | N |
| ATOM | 1864 | NH2 | ARG | A | 278 | -18.766 | 23.533 | -23.969 | 1.00 | 49.87 | A | N |
| ATOM | 1865 | C | ARG | A | 278 | -16.215 | 19.751 | -18.389 | 1.00 | 37.70 | A | C |
| ATOM | 1866 | O | ARG | A | 278 | -15.655 | 20.277 | -17.424 | 1.00 | 37.70 | A | O |
| ATOM | 1867 | N | GLU | A | 279 | -17.278 | 18.960 | -18.278 | 1.00 | 44.02 | A | N |
| ATOM | 1868 | CA | GLU | A | 279 | -17.885 | 18.656 | -16.989 | 1.00 | 44.02 | A | C |
| ATOM | 1869 | CB | GLU | A | 279 | -19.174 | 17.849 | -17.178 | 1.00 | 44.87 | A | C |

| | | | | | | | | | | | | |
|---|---|---|---|---|---|---|---|---|---|---|---|---|
| ATOM | 1870 | CG | GLU | A | 279 | -20.358 | 18.639 | -17.730 | 1.00 | 44.87 | A | C |
| ATOM | 1871 | CD | GLU | A | 279 | -20.103 | 19.240 | -19.103 | 1.00 | 44.87 | A | C |
| ATOM | 1872 | OE1 | GLU | A | 279 | -20.064 | 20.493 | -19.192 | 1.00 | 44.87 | A | O |
| ATOM | 1873 | OE2 | GLU | A | 279 | -19.943 | 18.464 | -20.080 | 1.00 | 44.87 | A | O |
| ATOM | 1874 | C | GLU | A | 279 | -16.924 | 17.876 | -16.111 | 1.00 | 44.02 | A | C |
| ATOM | 1875 | O | GLU | A | 279 | -16.819 | 18.132 | -14.904 | 1.00 | 44.02 | A | O |
| ATOM | 1876 | N | GLN | A | 280 | -16.231 | 16.918 | -16.723 | 1.00 | 46.53 | A | N |
| ATOM | 1877 | CA | GLN | A | 280 | -15.274 | 16.099 | -15.996 | 1.00 | 46.53 | A | C |
| ATOM | 1878 | CB | GLN | A | 280 | -14.599 | 15.105 | -16.943 | 1.00 | 27.94 | A | C |
| ATOM | 1879 | CG | GLN | A | 280 | -15.190 | 13.704 | -16.857 | 1.00 | 27.94 | A | C |
| ATOM | 1880 | CD | GLN | A | 280 | -14.742 | 12.811 | -17.982 | 1.00 | 27.94 | A | C |
| ATOM | 1881 | OE1 | GLN | A | 280 | -15.000 | 11.611 | -17.972 | 1.00 | 27.94 | A | O |
| ATOM | 1882 | NE2 | GLN | A | 280 | -14.077 | 13.392 | -18.972 | 1.00 | 27.94 | A | N |
| ATOM | 1883 | C | GLN | A | 280 | -14.247 | 16.990 | -15.327 | 1.00 | 46.53 | A | C |
| ATOM | 1884 | O | GLN | A | 280 | -14.029 | 16.905 | -14.113 | 1.00 | 46.53 | A | O |
| ATOM | 1885 | N | ILE | A | 281 | -13.637 | 17.862 | -16.121 | 1.00 | 42.30 | A | N |
| ATOM | 1886 | CA | ILE | A | 281 | -12.643 | 18.796 | -15.615 | 1.00 | 42.30 | A | C |
| ATOM | 1887 | CB | ILE | A | 281 | -12.219 | 19.770 | -16.722 | 1.00 | 20.23 | A | C |
| ATOM | 1888 | CG2 | ILE | A | 281 | -11.245 | 20.785 | -16.183 | 1.00 | 20.23 | A | C |
| ATOM | 1889 | CG1 | ILE | A | 281 | -11.581 | 18.980 | -17.864 | 1.00 | 20.23 | A | C |
| ATOM | 1890 | CD1 | ILE | A | 281 | -11.287 | 19.787 | -19.095 | 1.00 | 20.23 | A | C |
| ATOM | 1891 | C | ILE | A | 281 | -13.171 | 19.568 | -14.399 | 1.00 | 42.30 | A | C |
| ATOM | 1892 | O | ILE | A | 281 | -12.410 | 19.927 | -13.504 | 1.00 | 42.30 | A | O |
| ATOM | 1893 | N | ALA | A | 282 | -14.478 | 19.806 | -14.351 | 1.00 | 51.57 | A | N |
| ATOM | 1894 | CA | ALA | A | 282 | -15.061 | 20.527 | -13.220 | 1.00 | 51.57 | A | C |
| ATOM | 1895 | CB | ALA | A | 282 | -16.474 | 21.009 | -13.576 | 1.00 | 29.00 | A | C |
| ATOM | 1896 | C | ALA | A | 282 | -15.096 | 19.661 | -11.946 | 1.00 | 51.57 | A | C |
| ATOM | 1897 | O | ALA | A | 282 | -14.911 | 20.163 | -10.833 | 1.00 | 51.57 | A | O |
| ATOM | 1898 | N | GLU | A | 283 | -15.316 | 18.361 | -12.116 | 1.00 | 53.16 | A | N |
| ATOM | 1899 | CA | GLU | A | 283 | -15.397 | 17.445 | -10.979 | 1.00 | 53.16 | A | C |
| ATOM | 1900 | CB | GLU | A | 283 | -16.050 | 16.140 | -11.439 | 1.00 | 67.12 | A | C |
| ATOM | 1901 | CG | GLU | A | 283 | -17.351 | 16.346 | -12.198 | 1.00 | 67.12 | A | C |
| ATOM | 1902 | CD | GLU | A | 283 | -17.661 | 15.179 | -13.138 | 1.00 | 67.12 | A | C |
| ATOM | 1903 | OE1 | GLU | A | 283 | -16.791 | 14.858 | -13.983 | 1.00 | 67.12 | A | O |
| ATOM | 1904 | OE2 | GLU | A | 283 | -18.763 | 14.580 | -13.038 | 1.00 | 67.12 | A | O |
| ATOM | 1905 | C | GLU | A | 283 | -14.038 | 17.161 | -10.290 | 1.00 | 53.16 | A | C |
| ATOM | 1906 | O | GLU | A | 283 | -13.992 | 16.829 | -9.098 | 1.00 | 53.16 | A | O |
| ATOM | 1907 | N | MET | A | 284 | -12.952 | 17.286 | -11.054 | 1.00 | 50.66 | A | N |
| ATOM | 1908 | CA | MET | A | 284 | -11.597 | 17.087 | -10.548 | 1.00 | 50.66 | A | C |
| ATOM | 1909 | CB | MET | A | 284 | -10.617 | 16.854 | -11.713 | 1.00 | 46.46 | A | C |
| ATOM | 1910 | CG | MET | A | 284 | -10.620 | 15.428 | -12.271 | 1.00 | 46.46 | A | C |
| ATOM | 1911 | SD | MET | A | 284 | -10.040 | 15.239 | -13.979 | 1.00 | 46.46 | A | S |
| ATOM | 1912 | CE | MET | A | 284 | -8.440 | 15.367 | -13.763 | 1.00 | 46.46 | A | C |
| ATOM | 1913 | C | MET | A | 284 | -11.216 | 18.363 | -9.806 | 1.00 | 50.66 | A | C |
| ATOM | 1914 | O | MET | A | 284 | -10.715 | 18.328 | -8.683 | 1.00 | 50.66 | A | O |
| ATOM | 1915 | N | ASN | A | 285 | -11.473 | 19.497 | -10.444 | 1.00 | 41.14 | A | N |
| ATOM | 1916 | CA | ASN | A | 285 | -11.166 | 20.791 | -9.860 | 1.00 | 41.14 | A | C |
| ATOM | 1917 | CB | ASN | A | 285 | -9.696 | 21.150 | -10.070 | 1.00 | 63.31 | A | C |
| ATOM | 1918 | CG | ASN | A | 285 | -9.420 | 22.604 | -9.776 | 1.00 | 63.31 | A | C |
| ATOM | 1919 | OD1 | ASN | A | 285 | -9.955 | 23.156 | -8.808 | 1.00 | 63.31 | A | O |
| ATOM | 1920 | ND2 | ASN | A | 285 | -8.585 | 23.241 | -10.602 | 1.00 | 63.31 | A | N |
| ATOM | 1921 | C | ASN | A | 285 | -12.039 | 21.864 | -10.488 | 1.00 | 41.14 | A | C |
| ATOM | 1922 | O | ASN | A | 285 | -11.855 | 22.230 | -11.656 | 1.00 | 41.14 | A | O |
| ATOM | 1923 | N | PRO | A | 286 | -12.977 | 22.410 | -9.692 | 1.00 | 92.65 | A | N |
| ATOM | 1924 | CD | PRO | A | 286 | -12.916 | 22.175 | -8.231 | 1.00 | 84.61 | A | C |
| ATOM | 1925 | CA | PRO | A | 286 | -13.973 | 23.454 | -10.001 | 1.00 | 92.65 | A | C |
| ATOM | 1926 | CB | PRO | A | 286 | -14.614 | 23.729 | -8.636 | 1.00 | 84.61 | A | C |
| ATOM | 1927 | CG | PRO | A | 286 | -13.463 | 23.485 | -7.673 | 1.00 | 84.61 | A | C |
| ATOM | 1928 | C | PRO | A | 286 | -13.461 | 24.745 | -10.653 | 1.00 | 92.65 | A | C |
| ATOM | 1929 | O | PRO | A | 286 | -13.884 | 25.121 | -11.757 | 1.00 | 92.65 | A | O |
| ATOM | 1930 | N | ASN | A | 287 | -12.549 | 25.415 | -9.957 | 1.00 | 95.33 | A | N |
| ATOM | 1931 | CA | ASN | A | 287 | -11.985 | 26.681 | -10.418 | 1.00 | 95.33 | A | C |
| ATOM | 1932 | CB | ASN | A | 287 | -11.130 | 27.262 | -9.292 | 1.00 | 70.89 | A | C |
| ATOM | 1933 | CG | ASN | A | 287 | -11.868 | 27.244 | -7.951 | 1.00 | 70.89 | A | C |
| ATOM | 1934 | OD1 | ASN | A | 287 | -12.711 | 28.112 | -7.683 | 1.00 | 70.89 | A | O |
| ATOM | 1935 | ND2 | ASN | A | 287 | -11.577 | 26.229 | -7.116 | 1.00 | 70.89 | A | N |
| ATOM | 1936 | C | ASN | A | 287 | -11.196 | 26.602 | -11.730 | 1.00 | 95.33 | A | C |

| | | | | | | | | | | | | |
|---|---|---|---|---|---|---|---|---|---|---|---|---|
| ATOM | 1937 | O | ASN | A | 287 | -11.179 | 25.558 | -12.401 | 1.00 | 95.33 | A | O |
| ATOM | 1938 | N | ALA | A | 288 | -10.555 | 27.715 | -12.086 | 1.00 | 73.11 | A | N |
| ATOM | 1939 | CA | ALA | A | 288 | -9.785 | 27.812 | -13.326 | 1.00 | 73.11 | A | C |
| ATOM | 1940 | CB | ALA | A | 288 | -9.145 | 29.207 | -13.431 | 1.00 | 84.42 | A | C |
| ATOM | 1941 | C | ALA | A | 288 | -8.715 | 26.724 | -13.460 | 1.00 | 73.11 | A | C |
| ATOM | 1942 | O | ALA | A | 288 | -7.689 | 26.742 | -12.765 | 1.00 | 73.11 | A | O |
| ATOM | 1943 | N | ALA | A | 294 | -6.486 | 20.477 | -26.814 | 1.00 | 71.03 | A | N |
| ATOM | 1944 | CA | ALA | A | 294 | -5.706 | 19.328 | -27.287 | 1.00 | 71.03 | A | C |
| ATOM | 1945 | CB | ALA | A | 294 | -4.479 | 19.112 | -26.372 | 1.00 | 41.44 | A | C |
| ATOM | 1946 | C | ALA | A | 294 | -6.612 | 18.082 | -27.288 | 1.00 | 71.03 | A | C |
| ATOM | 1947 | O | ALA | A | 294 | -6.401 | 17.139 | -26.505 | 1.00 | 71.03 | A | O |
| ATOM | 1948 | N | ALA | A | 295 | -7.607 | 18.091 | -28.184 | 1.00 | 62.93 | A | N |
| ATOM | 1949 | CA | ALA | A | 295 | -8.608 | 17.019 | -28.292 | 1.00 | 62.93 | A | C |
| ATOM | 1950 | CB | ALA | A | 295 | -9.808 | 17.530 | -29.068 | 1.00 | 47.90 | A | C |
| ATOM | 1951 | C | ALA | A | 295 | -8.167 | 15.681 | -28.872 | 1.00 | 62.93 | A | C |
| ATOM | 1952 | O | ALA | A | 295 | -7.975 | 15.555 | -30.078 | 1.00 | 62.93 | A | O |
| ATOM | 1953 | N | ALA | A | 296 | -8.035 | 14.681 | -28.002 | 1.00 | 51.79 | A | N |
| ATOM | 1954 | CA | ALA | A | 296 | -7.640 | 13.331 | -28.408 | 1.00 | 51.79 | A | C |
| ATOM | 1955 | CB | ALA | A | 296 | -6.436 | 12.855 | -27.590 | 1.00 | 24.97 | A | C |
| ATOM | 1956 | C | ALA | A | 296 | -8.831 | 12.388 | -28.203 | 1.00 | 51.79 | A | C |
| ATOM | 1957 | O | ALA | A | 296 | -9.629 | 12.589 | -27.291 | 1.00 | 51.79 | A | O |
| ATOM | 1958 | N | ALA | A | 297 | -8.958 | 11.380 | -29.065 | 1.00 | 55.43 | A | N |
| ATOM | 1959 | CA | ALA | A | 297 | -10.056 | 10.429 | -28.968 | 1.00 | 55.43 | A | C |
| ATOM | 1960 | CB | ALA | A | 297 | -9.959 | 9.388 | -30.074 | 1.00 | 32.74 | A | C |
| ATOM | 1961 | C | ALA | A | 297 | -10.009 | 9.760 | -27.605 | 1.00 | 55.43 | A | C |
| ATOM | 1962 | O | ALA | A | 297 | -10.079 | 10.434 | -26.586 | 1.00 | 55.43 | A | O |
| ATOM | 1963 | N | ALA | A | 298 | -9.889 | 8.440 | -27.580 | 1.00 | 47.14 | A | N |
| ATOM | 1964 | CA | ALA | A | 298 | -9.845 | 7.707 | -26.327 | 1.00 | 47.14 | A | C |
| ATOM | 1965 | CB | ALA | A | 298 | -11.134 | 7.885 | -25.564 | 1.00 | 48.38 | A | C |
| ATOM | 1966 | C | ALA | A | 298 | -9.656 | 6.254 | -26.683 | 1.00 | 47.14 | A | C |
| ATOM | 1967 | O | ALA | A | 298 | -10.565 | 5.604 | -27.180 | 1.00 | 47.14 | A | O |
| ATOM | 1968 | N | HIS | A | 299 | -8.459 | 5.754 | -26.436 | 1.00 | 48.29 | A | N |
| ATOM | 1969 | CA | HIS | A | 299 | -8.123 | 4.380 | -26.740 | 1.00 | 48.29 | A | C |
| ATOM | 1970 | CB | HIS | A | 299 | -6.653 | 4.141 | -26.409 | 1.00 | 61.92 | A | C |
| ATOM | 1971 | CG | HIS | A | 299 | -5.979 | 3.173 | -27.323 | 1.00 | 61.92 | A | C |
| ATOM | 1972 | CD2 | HIS | A | 299 | -5.664 | 3.263 | -28.635 | 1.00 | 61.92 | A | C |
| ATOM | 1973 | ND1 | HIS | A | 299 | -5.507 | 1.949 | -26.896 | 1.00 | 61.92 | A | N |
| ATOM | 1974 | CE1 | HIS | A | 299 | -4.923 | 1.330 | -27.907 | 1.00 | 61.92 | A | C |
| ATOM | 1975 | NE2 | HIS | A | 299 | -5.004 | 2.106 | -28.973 | 1.00 | 61.92 | A | N |
| ATOM | 1976 | C | HIS | A | 299 | -8.985 | 3.395 | -25.961 | 1.00 | 48.29 | A | C |
| ATOM | 1977 | O | HIS | A | 299 | -9.155 | 3.520 | -24.746 | 1.00 | 48.29 | A | O |
| ATOM | 1978 | N | PRO | A | 300 | -9.563 | 2.408 | -26.662 | 1.00 | 67.72 | A | N |
| ATOM | 1979 | CD | PRO | A | 300 | -9.463 | 2.168 | -28.114 | 1.00 | 46.60 | A | C |
| ATOM | 1980 | CA | PRO | A | 300 | -10.406 | 1.396 | -26.007 | 1.00 | 67.72 | A | C |
| ATOM | 1981 | CB | PRO | A | 300 | -10.938 | 0.577 | -27.183 | 1.00 | 46.60 | A | C |
| ATOM | 1982 | CG | PRO | A | 300 | -9.827 | 0.704 | -28.211 | 1.00 | 46.60 | A | C |
| ATOM | 1983 | C | PRO | A | 300 | -9.580 | 0.551 | -25.015 | 1.00 | 67.72 | A | C |
| ATOM | 1984 | O | PRO | A | 300 | -8.594 | -0.097 | -25.395 | 1.00 | 67.72 | A | O |
| ATOM | 1985 | N | TRP | A | 301 | -9.995 | 0.569 | -23.747 | 1.00 | 65.47 | A | N |
| ATOM | 1986 | CA | TRP | A | 301 | -9.313 | -0.155 | -22.679 | 1.00 | 65.47 | A | C |
| ATOM | 1987 | CB | TRP | A | 301 | -10.250 | -0.329 | -21.483 | 1.00 | 52.29 | A | C |
| ATOM | 1988 | CG | TRP | A | 301 | -10.172 | 0.808 | -20.508 | 1.00 | 52.29 | A | C |
| ATOM | 1989 | CD2 | TRP | A | 301 | -8.994 | 1.284 | -19.833 | 1.00 | 52.29 | A | C |
| ATOM | 1990 | CE2 | TRP | A | 301 | -9.376 | 2.404 | -19.058 | 1.00 | 52.29 | A | C |
| ATOM | 1991 | CE3 | TRP | A | 301 | -7.660 | 0.864 | -19.799 | 1.00 | 52.29 | A | C |
| ATOM | 1992 | CD1 | TRP | A | 301 | -11.193 | 1.637 | -20.124 | 1.00 | 52.29 | A | C |
| ATOM | 1993 | NE1 | TRP | A | 301 | -10.719 | 2.603 | -19.253 | 1.00 | 52.29 | A | N |
| ATOM | 1994 | CZ2 | TRP | A | 301 | -8.464 | 3.120 | -18.275 | 1.00 | 52.29 | A | C |
| ATOM | 1995 | CZ3 | TRP | A | 301 | -6.750 | 1.576 | -19.015 | 1.00 | 52.29 | A | C |
| ATOM | 1996 | CH2 | TRP | A | 301 | -7.159 | 2.687 | -18.262 | 1.00 | 52.29 | A | C |
| ATOM | 1997 | C | TRP | A | 301 | -8.746 | -1.509 | -23.086 | 1.00 | 65.47 | A | C |
| ATOM | 1998 | O | TRP | A | 301 | -7.607 | -1.847 | -22.748 | 1.00 | 65.47 | A | O |
| ATOM | 1999 | N | THR | A | 302 | -9.537 | -2.281 | -23.818 | 1.00 | 59.65 | A | N |
| ATOM | 2000 | CA | THR | A | 302 | -9.114 | -3.608 | -24.253 | 1.00 | 59.65 | A | C |
| ATOM | 2001 | CB | THR | A | 302 | -10.254 | -4.333 | -24.995 | 1.00 | 64.90 | A | C |
| ATOM | 2002 | OG1 | THR | A | 302 | -10.580 | -3.601 | -26.190 | 1.00 | 64.90 | A | O |
| ATOM | 2003 | CG2 | THR | A | 302 | -11.492 | -4.443 | -24.097 | 1.00 | 64.90 | A | C |

| | | | | | | | | | | | | |
|---|---|---|---|---|---|---|---|---|---|---|---|---|
| ATOM | 2004 | C | THR | A | 302 | -7.878 | -3.582 | -25.157 | 1.00 | 59.65 | A | C |
| ATOM | 2005 | O | THR | A | 302 | -6.946 | -4.360 | -24.967 | 1.00 | 59.65 | A | O |
| ATOM | 2006 | N | ALA | A | 303 | -7.864 | -2.700 | -26.145 | 1.00 | 56.57 | A | N |
| ATOM | 2007 | CA | ALA | A | 303 | -6.711 | -2.632 | -27.038 | 1.00 | 56.57 | A | C |
| ATOM | 2008 | CB | ALA | A | 303 | -7.071 | -1.829 | -28.311 | 1.00 | 85.17 | A | C |
| ATOM | 2009 | C | ALA | A | 303 | -5.514 | -1.996 | -26.318 | 1.00 | 56.57 | A | C |
| ATOM | 2010 | O | ALA | A | 303 | -4.515 | -1.632 | -26.947 | 1.00 | 56.57 | A | O |
| ATOM | 2011 | N | VAL | A | 304 | -5.630 | -1.863 | -24.997 | 1.00 | 46.02 | A | N |
| ATOM | 2012 | CA | VAL | A | 304 | -4.578 | -1.273 | -24.176 | 1.00 | 46.02 | A | C |
| ATOM | 2013 | CB | VAL | A | 304 | -5.175 | -0.329 | -23.096 | 1.00 | 45.10 | A | C |
| ATOM | 2014 | CG1 | VAL | A | 304 | -4.065 | 0.242 | -22.205 | 1.00 | 45.10 | A | C |
| ATOM | 2015 | CG2 | VAL | A | 304 | -5.951 | 0.789 | -23.768 | 1.00 | 45.10 | A | C |
| ATOM | 2016 | C | VAL | A | 304 | -3.765 | -2.356 | -23.481 | 1.00 | 46.02 | A | C |
| ATOM | 2017 | O | VAL | A | 304 | -2.565 | -2.195 | -23.263 | 1.00 | 46.02 | A | O |
| ATOM | 2018 | N | PHE | A | 305 | -4.420 | -3.461 | -23.137 | 1.00 | 50.92 | A | N |
| ATOM | 2019 | CA | PHE | A | 305 | -3.741 | -4.559 | -22.457 | 1.00 | 50.92 | A | C |
| ATOM | 2020 | CB | PHE | A | 305 | -4.620 | -5.071 | -21.316 | 1.00 | 50.44 | A | C |
| ATOM | 2021 | CG | PHE | A | 305 | -4.796 | -4.074 | -20.212 | 1.00 | 50.44 | A | C |
| ATOM | 2022 | CD1 | PHE | A | 305 | -3.872 | -3.996 | -19.180 | 1.00 | 50.44 | A | C |
| ATOM | 2023 | CD2 | PHE | A | 305 | -5.844 | -3.155 | -20.249 | 1.00 | 50.44 | A | C |
| ATOM | 2024 | CE1 | PHE | A | 305 | -3.982 | -3.011 | -18.198 | 1.00 | 50.44 | A | C |
| ATOM | 2025 | CE2 | PHE | A | 305 | -5.961 | -2.166 | -19.274 | 1.00 | 50.44 | A | C |
| ATOM | 2026 | CZ | PHE | A | 305 | -5.027 | -2.093 | -18.246 | 1.00 | 50.44 | A | C |
| ATOM | 2027 | C | PHE | A | 305 | -3.363 | -5.701 | -23.391 | 1.00 | 50.92 | A | C |
| ATOM | 2028 | O | PHE | A | 305 | -3.917 | -5.827 | -24.479 | 1.00 | 50.92 | A | O |
| ATOM | 2029 | N | ARG | A | 306 | -2.400 | -6.523 | -22.986 | 1.00 | 53.83 | A | N |
| ATOM | 2030 | CA | ARG | A | 306 | -2.010 | -7.633 | -23.840 | 1.00 | 53.83 | A | C |
| ATOM | 2031 | CB | ARG | A | 306 | -0.896 | -8.475 | -23.192 | 1.00 | 79.13 | A | C |
| ATOM | 2032 | CG | ARG | A | 306 | -1.112 | -8.860 | -21.721 | 1.00 | 79.13 | A | C |
| ATOM | 2033 | CD | ARG | A | 306 | -0.107 | -9.939 | -21.275 | 1.00 | 79.13 | A | C |
| ATOM | 2034 | NE | ARG | A | 306 | -0.651 | -11.289 | -21.426 | 1.00 | 79.13 | A | N |
| ATOM | 2035 | CZ | ARG | A | 306 | -1.489 | -11.861 | -20.560 | 1.00 | 79.13 | A | C |
| ATOM | 2036 | NH1 | ARG | A | 306 | -1.884 | -11.202 | -19.466 | 1.00 | 79.13 | A | N |
| ATOM | 2037 | NH2 | ARG | A | 306 | -1.934 | -13.094 | -20.782 | 1.00 | 79.13 | A | N |
| ATOM | 2038 | C | ARG | A | 306 | -3.251 | -8.486 | -24.104 | 1.00 | 53.83 | A | C |
| ATOM | 2039 | O | ARG | A | 306 | -4.195 | -8.498 | -23.302 | 1.00 | 53.83 | A | O |
| ATOM | 2040 | N | PRO | A | 307 | -3.272 | -9.191 | -25.244 | 1.00 | 56.50 | A | N |
| ATOM | 2041 | CD | PRO | A | 307 | -2.185 | -9.123 | -26.239 | 1.00 | 62.89 | A | C |
| ATOM | 2042 | CA | PRO | A | 307 | -4.346 | -10.070 | -25.719 | 1.00 | 56.50 | A | C |
| ATOM | 2043 | CB | PRO | A | 307 | -3.644 | -10.901 | -26.786 | 1.00 | 62.89 | A | C |
| ATOM | 2044 | CG | PRO | A | 307 | -2.766 | -9.886 | -27.426 | 1.00 | 62.89 | A | C |
| ATOM | 2045 | C | PRO | A | 307 | -5.076 | -10.937 | -24.692 | 1.00 | 56.50 | A | C |
| ATOM | 2046 | O | PRO | A | 307 | -6.296 | -10.820 | -24.524 | 1.00 | 56.50 | A | O |
| ATOM | 2047 | N | ALA | A | 308 | -4.339 | -11.808 | -24.010 | 1.00 | 65.02 | A | N |
| ATOM | 2048 | CA | ALA | A | 308 | -4.957 | -12.708 | -23.035 | 1.00 | 65.02 | A | C |
| ATOM | 2049 | CB | ALA | A | 308 | -4.147 | -14.016 | -22.949 | 1.00 | 90.94 | A | C |
| ATOM | 2050 | C | ALA | A | 308 | -5.170 | -12.118 | -21.630 | 1.00 | 65.02 | A | C |
| ATOM | 2051 | O | ALA | A | 308 | -5.159 | -12.844 | -20.631 | 1.00 | 65.02 | A | O |
| ATOM | 2052 | N | THR | A | 309 | -5.382 | -10.806 | -21.568 | 1.00 | 57.74 | A | N |
| ATOM | 2053 | CA | THR | A | 309 | -5.627 | -10.113 | -20.305 | 1.00 | 57.74 | A | C |
| ATOM | 2054 | CB | THR | A | 309 | -5.462 | -8.589 | -20.471 | 1.00 | 32.02 | A | C |
| ATOM | 2055 | OG1 | THR | A | 309 | -4.113 | -8.289 | -20.859 | 1.00 | 32.02 | A | O |
| ATOM | 2056 | CG2 | THR | A | 309 | -5.799 | -7.878 | -19.168 | 1.00 | 32.02 | A | C |
| ATOM | 2057 | C | THR | A | 309 | -7.047 | -10.396 | -19.804 | 1.00 | 57.74 | A | C |
| ATOM | 2058 | O | THR | A | 309 | -8.025 | -10.095 | -20.482 | 1.00 | 57.74 | A | O |
| ATOM | 2059 | N | PRO | A | 310 | -7.174 | -10.977 | -18.603 | 1.00 | 64.90 | A | N |
| ATOM | 2060 | CD | PRO | A | 310 | -6.102 | -11.258 | -17.629 | 1.00 | 34.19 | A | C |
| ATOM | 2061 | CA | PRO | A | 310 | -8.495 | -11.289 | -18.037 | 1.00 | 64.90 | A | C |
| ATOM | 2062 | CB | PRO | A | 310 | -8.176 | -11.599 | -16.577 | 1.00 | 34.19 | A | C |
| ATOM | 2063 | CG | PRO | A | 310 | -6.793 | -12.169 | -16.647 | 1.00 | 34.19 | A | C |
| ATOM | 2064 | C | PRO | A | 310 | -9.491 | -10.128 | -18.168 | 1.00 | 64.90 | A | C |
| ATOM | 2065 | O | PRO | A | 310 | -9.234 | -9.013 | -17.703 | 1.00 | 64.90 | A | O |
| ATOM | 2066 | N | PRO | A | 311 | -10.641 | -10.373 | -18.809 | 1.00 | 64.09 | A | N |
| ATOM | 2067 | CD | PRO | A | 311 | -10.984 | -11.599 | -19.545 | 1.00 | 52.92 | A | C |
| ATOM | 2068 | CA | PRO | A | 311 | -11.676 | -9.355 | -19.001 | 1.00 | 64.09 | A | C |
| ATOM | 2069 | CB | PRO | A | 311 | -12.844 | -10.164 | -19.529 | 1.00 | 52.92 | A | C |
| ATOM | 2070 | CG | PRO | A | 311 | -12.149 | -11.136 | -20.410 | 1.00 | 52.92 | A | C |

| | | | | | | | | | | | | |
|---|---|---|---|---|---|---|---|---|---|---|---|---|
| ATOM | 2071 | C | PRO | A | 311 | -12.034 | -8.581 | -17.733 | 1.00 | 64.09 | A | C |
| ATOM | 2072 | O | PRO | A | 311 | -12.141 | -7.351 | -17.771 | 1.00 | 64.09 | A | O |
| ATOM | 2073 | N | GLU | A | 312 | -12.219 | -9.288 | -16.620 | 1.00 | 55.01 | A | N |
| ATOM | 2074 | CA | GLU | A | 312 | -12.568 | -8.628 | -15.368 | 1.00 | 55.01 | A | C |
| ATOM | 2075 | CB | GLU | A | 312 | -12.942 | -9.639 | -14.287 | 1.00100.00 | | A | C |
| ATOM | 2076 | CG | GLU | A | 312 | -14.076 | -10.550 | -14.681 | 1.00100.00 | | A | C |
| ATOM | 2077 | CD | GLU | A | 312 | -13.598 | -11.761 | -15.476 | 1.00100.00 | | A | C |
| ATOM | 2078 | OE1 | GLU | A | 312 | -12.767 | -11.598 | -16.419 | 1.00100.00 | | A | O |
| ATOM | 2079 | OE2 | GLU | A | 312 | -14.071 | -12.880 | -15.150 | 1.00100.00 | | A | O |
| ATOM | 2080 | C | GLU | A | 312 | -11.429 | -7.761 | -14.862 | 1.00 | 55.01 | A | C |
| ATOM | 2081 | O | GLU | A | 312 | -11.670 | -6.766 | -14.189 | 1.00 | 55.01 | A | O |
| ATOM | 2082 | N | ALA | A | 313 | -10.190 | -8.133 | -15.160 | 1.00 | 30.55 | A | N |
| ATOM | 2083 | CA | ALA | A | 313 | -9.066 | -7.317 | -14.727 | 1.00 | 30.55 | A | C |
| ATOM | 2084 | CB | ALA | A | 313 | -7.757 | -7.932 | -15.199 | 1.00 | 36.01 | A | C |
| ATOM | 2085 | C | ALA | A | 313 | -9.278 | -5.951 | -15.375 | 1.00 | 30.55 | A | C |
| ATOM | 2086 | O | ALA | A | 313 | -9.151 | -4.906 | -14.738 | 1.00 | 30.55 | A | O |
| ATOM | 2087 | N | ILE | A | 314 | -9.624 | -5.979 | -16.655 | 1.00 | 47.62 | A | N |
| ATOM | 2088 | CA | ILE | A | 314 | -9.879 | -4.766 | -17.421 | 1.00 | 47.62 | A | C |
| ATOM | 2089 | CB | ILE | A | 314 | -10.104 | -5.113 | -18.907 | 1.00 | 40.14 | A | C |
| ATOM | 2090 | CG2 | ILE | A | 314 | -10.517 | -3.866 | -19.685 | 1.00 | 40.14 | A | C |
| ATOM | 2091 | CG1 | ILE | A | 314 | -8.823 | -5.725 | -19.486 | 1.00 | 40.14 | A | C |
| ATOM | 2092 | CD1 | ILE | A | 314 | -8.966 | -6.262 | -20.895 | 1.00 | 40.14 | A | C |
| ATOM | 2093 | C | ILE | A | 314 | -11.098 | -4.002 | -16.878 | 1.00 | 47.62 | A | C |
| ATOM | 2094 | O | ILE | A | 314 | -11.048 | -2.788 | -16.671 | 1.00 | 47.62 | A | O |
| ATOM | 2095 | N | ALA | A | 315 | -12.192 | -4.714 | -16.652 | 1.00 | 42.81 | A | N |
| ATOM | 2096 | CA | ALA | A | 315 | -13.386 | -4.083 | -16.137 | 1.00 | 42.81 | A | C |
| ATOM | 2097 | CB | ALA | A | 315 | -14.425 | -5.139 | -15.799 | 1.00 | 51.11 | A | C |
| ATOM | 2098 | C | ALA | A | 315 | -13.030 | -3.290 | -14.890 | 1.00 | 42.81 | A | C |
| ATOM | 2099 | O | ALA | A | 315 | -13.289 | -2.083 | -14.806 | 1.00 | 42.81 | A | O |
| ATOM | 2100 | N | LEU | A | 316 | -12.436 | -3.986 | -13.924 | 1.00 | 38.88 | A | N |
| ATOM | 2101 | CA | LEU | A | 316 | -12.037 | -3.395 | -12.658 | 1.00 | 38.88 | A | C |
| ATOM | 2102 | CB | LEU | A | 316 | -11.305 | -4.429 | -11.806 | 1.00 | 26.88 | A | C |
| ATOM | 2103 | CG | LEU | A | 316 | -10.479 | -3.879 | -10.640 | 1.00 | 26.88 | A | C |
| ATOM | 2104 | CD1 | LEU | A | 316 | -11.381 | -3.233 | -9.603 | 1.00 | 26.88 | A | C |
| ATOM | 2105 | CD2 | LEU | A | 316 | -9.670 | -4.993 | -10.028 | 1.00 | 26.88 | A | C |
| ATOM | 2106 | C | LEU | A | 316 | -11.134 | -2.208 | -12.879 | 1.00 | 38.88 | A | C |
| ATOM | 2107 | O | LEU | A | 316 | -11.288 | -1.174 | -12.249 | 1.00 | 38.88 | A | O |
| ATOM | 2108 | N | CYS | A | 317 | -10.187 | -2.357 | -13.786 | 1.00 | 44.68 | A | N |
| ATOM | 2109 | CA | CYS | A | 317 | -9.254 | -1.284 | -14.042 | 1.00 | 44.68 | A | C |
| ATOM | 2110 | CB | CYS | A | 317 | -8.233 | -1.726 | -15.083 | 1.00 | 39.70 | A | C |
| ATOM | 2111 | SG | CYS | A | 317 | -6.836 | -0.625 | -15.195 | 1.00 | 39.70 | A | S |
| ATOM | 2112 | C | CYS | A | 317 | -9.946 | -0.008 | -14.495 | 1.00 | 44.68 | A | C |
| ATOM | 2113 | O | CYS | A | 317 | -9.604 | 1.086 | -14.040 | 1.00 | 44.68 | A | O |
| ATOM | 2114 | N | SER | A | 318 | -10.930 | -0.146 | -15.378 | 1.00 | 50.01 | A | N |
| ATOM | 2115 | CA | SER | A | 318 | -11.654 | 1.011 | -15.909 | 1.00 | 50.01 | A | C |
| ATOM | 2116 | CB | SER | A | 318 | -12.497 | 0.575 | -17.093 | 1.00 | 37.17 | A | C |
| ATOM | 2117 | OG | SER | A | 318 | -13.380 | -0.444 | -16.672 | 1.00 | 37.17 | A | O |
| ATOM | 2118 | C | SER | A | 318 | -12.544 | 1.735 | -14.890 | 1.00 | 50.01 | A | C |
| ATOM | 2119 | O | SER | A | 318 | -12.875 | 2.909 | -15.060 | 1.00 | 50.01 | A | O |
| ATOM | 2120 | N | ARG | A | 319 | -12.936 | 1.038 | -13.837 | 1.00 | 35.75 | A | N |
| ATOM | 2121 | CA | ARG | A | 319 | -13.772 | 1.646 | -12.812 | 1.00 | 35.75 | A | C |
| ATOM | 2122 | CB | ARG | A | 319 | -14.722 | 0.594 | -12.244 | 1.00 | 54.31 | A | C |
| ATOM | 2123 | CG | ARG | A | 319 | -15.717 | 0.069 | -13.259 | 1.00 | 54.31 | A | C |
| ATOM | 2124 | CD | ARG | A | 319 | -16.815 | 1.090 | -13.563 | 1.00 | 54.31 | A | C |
| ATOM | 2125 | NE | ARG | A | 319 | -17.713 | 1.260 | -12.426 | 1.00 | 54.31 | A | N |
| ATOM | 2126 | CZ | ARG | A | 319 | -17.727 | 2.333 | -11.643 | 1.00 | 54.31 | A | C |
| ATOM | 2127 | NH1 | ARG | A | 319 | -16.887 | 3.335 | -11.884 | 1.00 | 54.31 | A | N |
| ATOM | 2128 | NH2 | ARG | A | 319 | -18.573 | 2.398 | -10.617 | 1.00 | 54.31 | A | N |
| ATOM | 2129 | C | ARG | A | 319 | -12.920 | 2.238 | -11.690 | 1.00 | 35.75 | A | C |
| ATOM | 2130 | O | ARG | A | 319 | -13.431 | 2.669 | -10.660 | 1.00 | 35.75 | A | O |
| ATOM | 2131 | N | LEU | A | 320 | -11.611 | 2.232 | -11.898 | 1.00 | 34.41 | A | N |
| ATOM | 2132 | CA | LEU | A | 320 | -10.668 | 2.759 | -10.932 | 1.00 | 34.41 | A | C |
| ATOM | 2133 | CB | LEU | A | 320 | -9.573 | 1.726 | -10.654 | 1.00 | 28.61 | A | C |
| ATOM | 2134 | CG | LEU | A | 320 | -10.005 | 0.436 | -9.942 | 1.00 | 28.61 | A | C |
| ATOM | 2135 | CD1 | LEU | A | 320 | -8.839 | -0.545 | -9.884 | 1.00 | 28.61 | A | C |
| ATOM | 2136 | CD2 | LEU | A | 320 | -10.495 | 0.762 | -8.536 | 1.00 | 28.61 | A | C |
| ATOM | 2137 | C | LEU | A | 320 | -10.068 | 3.999 | -11.559 | 1.00 | 34.41 | A | C |

| | | | | | | | | | | | | |
|---|---|---|---|---|---|---|---|---|---|---|---|---|
| ATOM | 2138 | O | LEU | A | 320 | -9.974 | 5.053 | -10.936 | 1.00 | 34.41 | A | O |
| ATOM | 2139 | N | LEU | A | 321 | -9.687 | 3.861 | -12.822 | 1.00 | 36.39 | A | N |
| ATOM | 2140 | CA | LEU | A | 321 | -9.085 | 4.950 | -13.561 | 1.00 | 36.39 | A | C |
| ATOM | 2141 | CB | LEU | A | 321 | -8.046 | 4.394 | -14.535 | 1.00 | 35.46 | A | C |
| ATOM | 2142 | CG | LEU | A | 321 | -6.886 | 3.658 | -13.857 | 1.00 | 35.46 | A | C |
| ATOM | 2143 | CD1 | LEU | A | 321 | -5.992 | 3.011 | -14.909 | 1.00 | 35.46 | A | C |
| ATOM | 2144 | CD2 | LEU | A | 321 | -6.108 | 4.629 | -12.992 | 1.00 | 35.46 | A | C |
| ATOM | 2145 | C | LEU | A | 321 | -10.116 | 5.782 | -14.298 | 1.00 | 36.39 | A | C |
| ATOM | 2146 | O | LEU | A | 321 | -10.236 | 5.708 | -15.517 | 1.00 | 36.39 | A | O |
| ATOM | 2147 | N | GLU | A | 322 | -10.865 | 6.570 | -13.537 | 1.00 | 40.80 | A | N |
| ATOM | 2148 | CA | GLU | A | 322 | -11.883 | 7.449 | -14.098 | 1.00 | 40.80 | A | C |
| ATOM | 2149 | CB | GLU | A | 322 | -13.256 | 7.146 | -13.483 | 1.00 | 49.19 | A | C |
| ATOM | 2150 | CG | GLU | A | 322 | -13.761 | 5.732 | -13.759 | 1.00 | 49.19 | A | C |
| ATOM | 2151 | CD | GLU | A | 322 | -15.005 | 5.692 | -14.645 | 1.00 | 49.19 | A | C |
| ATOM | 2152 | OE1 | GLU | A | 322 | -15.056 | 6.415 | -15.667 | 1.00 | 49.19 | A | O |
| ATOM | 2153 | OE2 | GLU | A | 322 | -15.928 | 4.911 | -14.317 | 1.00 | 49.19 | A | O |
| ATOM | 2154 | C | GLU | A | 322 | -11.483 | 8.880 | -13.781 | 1.00 | 40.80 | A | C |
| ATOM | 2155 | O | GLU | A | 322 | -10.897 | 9.135 | -12.734 | 1.00 | 40.80 | A | O |
| ATOM | 2156 | N | TYR | A | 323 | -11.772 | 9.811 | -14.683 | 1.00 | 36.98 | A | N |
| ATOM | 2157 | CA | TYR | A | 323 | -11.438 | 11.210 | -14.436 | 1.00 | 36.98 | A | C |
| ATOM | 2158 | CB | TYR | A | 323 | -11.783 | 12.073 | -15.646 | 1.00 | 41.83 | A | C |
| ATOM | 2159 | CG | TYR | A | 323 | -10.776 | 12.038 | -16.766 | 1.00 | 41.83 | A | C |
| ATOM | 2160 | CD1 | TYR | A | 323 | -9.512 | 12.611 | -16.615 | 1.00 | 41.83 | A | C |
| ATOM | 2161 | CE1 | TYR | A | 323 | -8.598 | 12.610 | -17.662 | 1.00 | 41.83 | A | C |
| ATOM | 2162 | CD2 | TYR | A | 323 | -11.095 | 11.460 | -17.993 | 1.00 | 41.83 | A | C |
| ATOM | 2163 | CE2 | TYR | A | 323 | -10.192 | 11.457 | -19.043 | 1.00 | 41.83 | A | C |
| ATOM | 2164 | CZ | TYR | A | 323 | -8.949 | 12.032 | -18.874 | 1.00 | 41.83 | A | C |
| ATOM | 2165 | OH | TYR | A | 323 | -8.072 | 12.031 | -19.931 | 1.00 | 41.83 | A | O |
| ATOM | 2166 | C | TYR | A | 323 | -12.222 | 11.712 | -13.232 | 1.00 | 36.98 | A | C |
| ATOM | 2167 | O | TYR | A | 323 | -11.655 | 12.267 | -12.297 | 1.00 | 36.98 | A | O |
| ATOM | 2168 | N | THR | A | 324 | -13.533 | 11.506 | -13.264 | 1.00 | 36.28 | A | N |
| ATOM | 2169 | CA | THR | A | 324 | -14.409 | 11.942 | -12.183 | 1.00 | 36.28 | A | C |
| ATOM | 2170 | CB | THR | A | 324 | -15.899 | 11.733 | -12.559 | 1.00 | 52.97 | A | C |
| ATOM | 2171 | OG1 | THR | A | 324 | -16.213 | 12.520 | -13.713 | 1.00 | 52.97 | A | O |
| ATOM | 2172 | CG2 | THR | A | 324 | -16.813 | 12.141 | -11.419 | 1.00 | 52.97 | A | C |
| ATOM | 2173 | C | THR | A | 324 | -14.101 | 11.165 | -10.912 | 1.00 | 36.28 | A | C |
| ATOM | 2174 | O | THR | A | 324 | -14.412 | 9.980 | -10.812 | 1.00 | 36.28 | A | O |
| ATOM | 2175 | N | PRO | A | 325 | -13.471 | 11.828 | -9.925 | 1.00 | 39.52 | A | N |
| ATOM | 2176 | CD | PRO | A | 325 | -13.087 | 13.252 | -9.959 | 1.00 | 33.38 | A | C |
| ATOM | 2177 | CA | PRO | A | 325 | -13.104 | 11.227 | -8.639 | 1.00 | 39.52 | A | C |
| ATOM | 2178 | CB | PRO | A | 325 | -12.750 | 12.435 | -7.794 | 1.00 | 33.38 | A | C |
| ATOM | 2179 | CG | PRO | A | 325 | -12.136 | 13.358 | -8.798 | 1.00 | 33.38 | A | C |
| ATOM | 2180 | C | PRO | A | 325 | -14.268 | 10.463 | -8.063 | 1.00 | 39.52 | A | C |
| ATOM | 2181 | O | PRO | A | 325 | -14.141 | 9.324 | -7.638 | 1.00 | 39.52 | A | O |
| ATOM | 2182 | N | THR | A | 326 | -15.416 | 11.115 | -8.078 | 1.00 | 30.83 | A | N |
| ATOM | 2183 | CA | THR | A | 326 | -16.656 | 10.562 | -7.557 | 1.00 | 30.83 | A | C |
| ATOM | 2184 | CB | THR | A | 326 | -17.753 | 11.648 | -7.646 | 1.00 | 38.12 | A | C |
| ATOM | 2185 | OG1 | THR | A | 326 | -18.320 | 11.847 | -6.348 | 1.00 | 38.12 | A | O |
| ATOM | 2186 | CG2 | THR | A | 326 | -18.852 | 11.272 | -8.664 | 1.00 | 38.12 | A | C |
| ATOM | 2187 | C | THR | A | 326 | -17.125 | 9.277 | -8.246 | 1.00 | 30.83 | A | C |
| ATOM | 2188 | O | THR | A | 326 | -17.937 | 8.537 | -7.708 | 1.00 | 30.83 | A | O |
| ATOM | 2189 | N | ALA | A | 327 | -16.600 | 9.013 | -9.432 | 1.00 | 35.79 | A | N |
| ATOM | 2190 | CA | ALA | A | 327 | -16.989 | 7.842 | -10.209 | 1.00 | 35.79 | A | C |
| ATOM | 2191 | CB | ALA | A | 327 | -16.865 | 8.163 | -11.684 | 1.00 | 47.40 | A | C |
| ATOM | 2192 | C | ALA | A | 327 | -16.226 | 6.548 | -9.914 | 1.00 | 35.79 | A | C |
| ATOM | 2193 | O | ALA | A | 327 | -16.699 | 5.457 | -10.233 | 1.00 | 35.79 | A | O |
| ATOM | 2194 | N | ARG | A | 328 | -15.045 | 6.675 | -9.317 | 1.00 | 44.41 | A | N |
| ATOM | 2195 | CA | ARG | A | 328 | -14.207 | 5.526 | -9.008 | 1.00 | 44.41 | A | C |
| ATOM | 2196 | CB | ARG | A | 328 | -12.839 | 6.005 | -8.543 | 1.00 | 37.43 | A | C |
| ATOM | 2197 | CG | ARG | A | 328 | -12.119 | 6.786 | -9.600 | 1.00 | 37.43 | A | C |
| ATOM | 2198 | CD | ARG | A | 328 | -10.983 | 7.593 | -9.039 | 1.00 | 37.43 | A | C |
| ATOM | 2199 | NE | ARG | A | 328 | -10.534 | 8.556 | -10.031 | 1.00 | 37.43 | A | N |
| ATOM | 2200 | CZ | ARG | A | 328 | -9.899 | 9.682 | -9.747 | 1.00 | 37.43 | A | C |
| ATOM | 2201 | NH1 | ARG | A | 328 | -9.627 | 9.997 | -8.490 | 1.00 | 37.43 | A | N |
| ATOM | 2202 | NH2 | ARG | A | 328 | -9.560 | 10.502 | -10.729 | 1.00 | 37.43 | A | N |
| ATOM | 2203 | C | ARG | A | 328 | -14.814 | 4.640 | -7.955 | 1.00 | 44.41 | A | C |
| ATOM | 2204 | O | ARG | A | 328 | -15.559 | 5.113 | -7.106 | 1.00 | 44.41 | A | O |

| | | | | | | | | | | | | |
|---|---|---|---|---|---|---|---|---|---|---|---|---|
| ATOM | 2205 | N | LEU | A | 329 | -14.501 | 3.352 | -8.012 | 1.00 | 31.71 | A | N |
| ATOM | 2206 | CA | LEU | A | 329 | -15.012 | 2.417 | -7.022 | 1.00 | 31.71 | A | C |
| ATOM | 2207 | CB | LEU | A | 329 | -14.626 | 0.986 | -7.398 | 1.00 | 44.09 | A | C |
| ATOM | 2208 | CG | LEU | A | 329 | -15.485 | 0.103 | -8.308 | 1.00 | 44.09 | A | C |
| ATOM | 2209 | CD1 | LEU | A | 329 | -16.026 | 0.914 | -9.439 | 1.00 | 44.09 | A | C |
| ATOM | 2210 | CD2 | LEU | A | 329 | -14.646 | -1.064 | -8.830 | 1.00 | 44.09 | A | C |
| ATOM | 2211 | C | LEU | A | 329 | -14.402 | 2.751 | -5.657 | 1.00 | 31.71 | A | C |
| ATOM | 2212 | O | LEU | A | 329 | -13.382 | 3.432 | -5.566 | 1.00 | 31.71 | A | O |
| ATOM | 2213 | N | THR | A | 330 | -15.036 | 2.282 | -4.593 | 1.00 | 35.01 | A | N |
| ATOM | 2214 | CA | THR | A | 330 | -14.511 | 2.504 | -3.257 | 1.00 | 35.01 | A | C |
| ATOM | 2215 | CB | THR | A | 330 | -15.635 | 2.559 | -2.208 | 1.00 | 39.37 | A | C |
| ATOM | 2216 | OG1 | THR | A | 330 | -16.357 | 1.317 | -2.203 | 1.00 | 39.37 | A | O |
| ATOM | 2217 | CG2 | THR | A | 330 | -16.575 | 3.706 | -2.506 | 1.00 | 39.37 | A | C |
| ATOM | 2218 | C | THR | A | 330 | -13.629 | 1.299 | -2.960 | 1.00 | 35.01 | A | C |
| ATOM | 2219 | O | THR | A | 330 | -13.822 | 0.235 | -3.531 | 1.00 | 35.01 | A | O |
| ATOM | 2220 | N | PRO | A | 331 | -12.652 | 1.444 | -2.060 | 1.00 | 43.16 | A | N |
| ATOM | 2221 | CD | PRO | A | 331 | -12.221 | 2.626 | -1.294 | 1.00 | 39.68 | A | C |
| ATOM | 2222 | CA | PRO | A | 331 | -11.798 | 0.296 | -1.767 | 1.00 | 43.16 | A | C |
| ATOM | 2223 | CB | PRO | A | 331 | -10.970 | 0.790 | -0.587 | 1.00 | 39.68 | A | C |
| ATOM | 2224 | CG | PRO | A | 331 | -10.818 | 2.245 | -0.882 | 1.00 | 39.68 | A | C |
| ATOM | 2225 | C | PRO | A | 331 | -12.580 | -0.987 | -1.456 | 1.00 | 43.16 | A | C |
| ATOM | 2226 | O | PRO | A | 331 | -12.283 | -2.051 | -2.004 | 1.00 | 43.16 | A | O |
| ATOM | 2227 | N | LEU | A | 332 | -13.576 | -0.899 | -0.583 | 1.00 | 38.54 | A | N |
| ATOM | 2228 | CA | LEU | A | 332 | -14.348 | -2.088 | -0.252 | 1.00 | 38.54 | A | C |
| ATOM | 2229 | CB | LEU | A | 332 | -15.407 | -1.778 | 0.798 | 1.00 | 56.75 | A | C |
| ATOM | 2230 | CG | LEU | A | 332 | -15.209 | -2.451 | 2.152 | 1.00 | 56.75 | A | C |
| ATOM | 2231 | CD1 | LEU | A | 332 | -16.511 | -2.395 | 2.941 | 1.00 | 56.75 | A | C |
| ATOM | 2232 | CD2 | LEU | A | 332 | -14.797 | -3.894 | 1.954 | 1.00 | 56.75 | A | C |
| ATOM | 2233 | C | LEU | A | 332 | -15.027 | -2.626 | -1.495 | 1.00 | 38.54 | A | C |
| ATOM | 2234 | O | LEU | A | 332 | -15.173 | -3.833 | -1.655 | 1.00 | 38.54 | A | O |
| ATOM | 2235 | N | GLU | A | 333 | -15.448 | -1.713 | -2.366 | 1.00 | 45.56 | A | N |
| ATOM | 2236 | CA | GLU | A | 333 | -16.112 | -2.074 | -3.609 | 1.00 | 45.56 | A | C |
| ATOM | 2237 | CB | GLU | A | 333 | -16.568 | -0.813 | -4.335 | 1.00 | 58.40 | A | C |
| ATOM | 2238 | CG | GLU | A | 333 | -18.067 | -0.612 | -4.335 | 1.00 | 58.40 | A | C |
| ATOM | 2239 | CD | GLU | A | 333 | -18.457 | 0.850 | -4.361 | 1.00 | 58.40 | A | C |
| ATOM | 2240 | OE1 | GLU | A | 333 | -18.002 | 1.583 | -5.277 | 1.00 | 58.40 | A | O |
| ATOM | 2241 | OE2 | GLU | A | 333 | -19.225 | 1.261 | -3.456 | 1.00 | 58.40 | A | O |
| ATOM | 2242 | C | GLU | A | 333 | -15.175 | -2.874 | -4.500 | 1.00 | 45.56 | A | C |
| ATOM | 2243 | O | GLU | A | 333 | -15.569 | -3.886 | -5.077 | 1.00 | 45.56 | A | O |
| ATOM | 2244 | N | ALA | A | 334 | -13.931 | -2.419 | -4.603 | 1.00 | 40.06 | A | N |
| ATOM | 2245 | CA | ALA | A | 334 | -12.933 | -3.090 | -5.419 | 1.00 | 40.06 | A | C |
| ATOM | 2246 | CB | ALA | A | 334 | -11.640 | -2.289 | -5.413 | 1.00 | 17.92 | A | C |
| ATOM | 2247 | C | ALA | A | 334 | -12.673 | -4.528 | -4.960 | 1.00 | 40.06 | A | C |
| ATOM | 2248 | O | ALA | A | 334 | -12.500 | -5.423 | -5.791 | 1.00 | 40.06 | A | O |
| ATOM | 2249 | N | CYS | A | 335 | -12.645 | -4.752 | -3.647 | 1.00 | 38.43 | A | N |
| ATOM | 2250 | CA | CYS | A | 335 | -12.402 | -6.097 | -3.116 | 1.00 | 38.43 | A | C |
| ATOM | 2251 | CB | CYS | A | 335 | -12.346 | -6.098 | -1.584 | 1.00 | 44.58 | A | C |
| ATOM | 2252 | SG | CYS | A | 335 | -10.950 | -5.235 | -0.871 | 1.00 | 44.58 | A | S |
| ATOM | 2253 | C | CYS | A | 335 | -13.551 | -6.967 | -3.548 | 1.00 | 38.43 | A | C |
| ATOM | 2254 | O | CYS | A | 335 | -13.389 | -8.154 | -3.827 | 1.00 | 38.43 | A | O |
| ATOM | 2255 | N | ALA | A | 336 | -14.724 | -6.353 | -3.597 | 1.00 | 43.52 | A | N |
| ATOM | 2256 | CA | ALA | A | 336 | -15.932 | -7.051 | -3.979 | 1.00 | 43.52 | A | C |
| ATOM | 2257 | CB | ALA | A | 336 | -17.136 | -6.206 | -3.622 | 1.00 | 51.07 | A | C |
| ATOM | 2258 | C | ALA | A | 336 | -15.946 | -7.388 | -5.461 | 1.00 | 43.52 | A | C |
| ATOM | 2259 | O | ALA | A | 336 | -16.671 | -8.282 | -5.891 | 1.00 | 43.52 | A | O |
| ATOM | 2260 | N | HIS | A | 337 | -15.141 | -6.683 | -6.243 | 1.00 | 45.08 | A | N |
| ATOM | 2261 | CA | HIS | A | 337 | -15.104 | -6.935 | -7.674 | 1.00 | 45.08 | A | C |
| ATOM | 2262 | CB | HIS | A | 337 | -14.008 | -6.119 | -8.335 | 1.00 | 41.50 | A | C |
| ATOM | 2263 | CG | HIS | A | 337 | -14.110 | -6.089 | -9.826 | 1.00 | 41.50 | A | C |
| ATOM | 2264 | CD2 | HIS | A | 337 | -13.737 | -6.992 | -10.762 | 1.00 | 41.50 | A | C |
| ATOM | 2265 | ND1 | HIS | A | 337 | -14.637 | -5.017 | -10.515 | 1.00 | 41.50 | A | N |
| ATOM | 2266 | CE1 | HIS | A | 337 | -14.577 | -5.257 | -11.812 | 1.00 | 41.50 | A | C |
| ATOM | 2267 | NE2 | HIS | A | 337 | -14.034 | -6.448 | -11.988 | 1.00 | 41.50 | A | N |
| ATOM | 2268 | C | HIS | A | 337 | -14.883 | -8.414 | -7.982 | 1.00 | 45.08 | A | C |
| ATOM | 2269 | O | HIS | A | 337 | -14.167 | -9.110 | -7.251 | 1.00 | 45.08 | A | O |
| ATOM | 2270 | N | SER | A | 338 | -15.484 | -8.888 | -9.073 | 1.00 | 58.56 | A | N |
| ATOM | 2271 | CA | SER | A | 338 | -15.371 | -10.295 | -9.460 | 1.00 | 58.56 | A | C |

| | | | | | | | | | | | | |
|---|---|---|---|---|---|---|---|---|---|---|---|---|
| ATOM | 2272 | CB | SER | A | 338 | -16.439 | -10.647 | -10.508 | 1.00 | 54.47 | A | C |
| ATOM | 2273 | OG | SER | A | 338 | -16.452 | -9.716 | -11.574 | 1.00 | 54.47 | A | O |
| ATOM | 2274 | C | SER | A | 338 | -13.985 | -10.710 | -9.955 | 1.00 | 58.56 | A | C |
| ATOM | 2275 | O | SER | A | 338 | -13.694 | -11.898 | -10.061 | 1.00 | 58.56 | A | O |
| ATOM | 2276 | N | PHE | A | 339 | -13.136 | -9.737 | -10.271 | 1.00 | 48.63 | A | N |
| ATOM | 2277 | CA | PHE | A | 339 | -11.775 | -10.030 | -10.703 | 1.00 | 48.63 | A | C |
| ATOM | 2278 | CB | PHE | A | 339 | -10.997 | -8.716 | -10.864 | 1.00 | 43.11 | A | C |
| ATOM | 2279 | CG | PHE | A | 339 | -9.503 | -8.886 | -11.058 | 1.00 | 43.11 | A | C |
| ATOM | 2280 | CD1 | PHE | A | 339 | -8.993 | -9.638 | -12.111 | 1.00 | 43.11 | A | C |
| ATOM | 2281 | CD2 | PHE | A | 339 | -8.607 | -8.231 | -10.226 | 1.00 | 43.11 | A | C |
| ATOM | 2282 | CE1 | PHE | A | 339 | -7.609 | -9.726 | -12.328 | 1.00 | 43.11 | A | C |
| ATOM | 2283 | CE2 | PHE | A | 339 | -7.229 | -8.315 | -10.438 | 1.00 | 43.11 | A | C |
| ATOM | 2284 | CZ | PHE | A | 339 | -6.733 | -9.060 | -11.488 | 1.00 | 43.11 | A | C |
| ATOM | 2285 | C | PHE | A | 339 | -11.142 | -10.877 | -9.596 | 1.00 | 48.63 | A | C |
| ATOM | 2286 | O | PHE | A | 339 | -10.318 | -11.761 | -9.852 | 1.00 | 48.63 | A | O |
| ATOM | 2287 | N | PHE | A | 340 | -11.566 | -10.607 | -8.362 | 1.00 | 52.47 | A | N |
| ATOM | 2288 | CA | PHE | A | 340 | -11.040 | -11.297 | -7.194 | 1.00 | 52.47 | A | C |
| ATOM | 2289 | CB | PHE | A | 340 | -10.966 | -10.328 | -6.012 | 1.00 | 35.35 | A | C |
| ATOM | 2290 | CG | PHE | A | 340 | -10.144 | -9.103 | -6.275 | 1.00 | 35.35 | A | C |
| ATOM | 2291 | CD1 | PHE | A | 340 | -8.809 | -9.204 | -6.639 | 1.00 | 35.35 | A | C |
| ATOM | 2292 | CD2 | PHE | A | 340 | -10.697 | -7.841 | -6.127 | 1.00 | 35.35 | A | C |
| ATOM | 2293 | CE1 | PHE | A | 340 | -8.034 | -8.067 | -6.849 | 1.00 | 35.35 | A | C |
| ATOM | 2294 | CE2 | PHE | A | 340 | -9.929 | -6.699 | -6.334 | 1.00 | 35.35 | A | C |
| ATOM | 2295 | CZ | PHE | A | 340 | -8.593 | -6.817 | -6.696 | 1.00 | 35.35 | A | C |
| ATOM | 2296 | C | PHE | A | 340 | -11.816 | -12.537 | -6.758 | 1.00 | 52.47 | A | C |
| ATOM | 2297 | O | PHE | A | 340 | -11.427 | -13.183 | -5.780 | 1.00 | 52.47 | A | O |
| ATOM | 2298 | N | ASP | A | 341 | -12.899 | -12.875 | -7.459 | 1.00 | 44.49 | A | N |
| ATOM | 2299 | CA | ASP | A | 341 | -13.702 | -14.049 | -7.088 | 1.00 | 44.49 | A | C |
| ATOM | 2300 | CB | ASP | A | 341 | -14.732 | -14.386 | -8.175 | 1.00 | 40.97 | A | C |
| ATOM | 2301 | CG | ASP | A | 341 | -15.805 | -13.313 | -8.327 | 1.00 | 40.97 | A | C |
| ATOM | 2302 | OD1 | ASP | A | 341 | -16.010 | -12.524 | -7.381 | 1.00 | 40.97 | A | O |
| ATOM | 2303 | OD2 | ASP | A | 341 | -16.454 | -13.268 | -9.393 | 1.00 | 40.97 | A | O |
| ATOM | 2304 | C | ASP | A | 341 | -12.856 | -15.287 | -6.801 | 1.00 | 44.49 | A | C |
| ATOM | 2305 | O | ASP | A | 341 | -13.080 | -15.982 | -5.816 | 1.00 | 44.49 | A | O |
| ATOM | 2306 | N | GLU | A | 342 | -11.881 | -15.565 | -7.655 | 1.00 | 48.42 | A | N |
| ATOM | 2307 | CA | GLU | A | 342 | -11.022 | -16.721 | -7.449 | 1.00 | 48.42 | A | C |
| ATOM | 2308 | CB | GLU | A | 342 | -9.854 | -16.699 | -8.443 | 1.00 | 44.98 | A | C |
| ATOM | 2309 | CG | GLU | A | 342 | -8.763 | -17.710 | -8.138 | 1.00 | 44.98 | A | C |
| ATOM | 2310 | CD | GLU | A | 342 | -7.765 | -17.861 | -9.270 | 1.00 | 44.98 | A | C |
| ATOM | 2311 | OE1 | GLU | A | 342 | -7.328 | -16.836 | -9.839 | 1.00 | 44.98 | A | O |
| ATOM | 2312 | OE2 | GLU | A | 342 | -7.405 | -19.014 | -9.589 | 1.00 | 44.98 | A | O |
| ATOM | 2313 | C | GLU | A | 342 | -10.489 | -16.793 | -6.011 | 1.00 | 48.42 | A | C |
| ATOM | 2314 | O | GLU | A | 342 | -10.270 | -17.878 | -5.479 | 1.00 | 48.42 | A | O |
| ATOM | 2315 | N | LEU | A | 343 | -10.286 | -15.643 | -5.375 | 1.00 | 46.83 | A | N |
| ATOM | 2316 | CA | LEU | A | 343 | -9.777 | -15.622 | -4.004 | 1.00 | 46.83 | A | C |
| ATOM | 2317 | CB | LEU | A | 343 | -9.328 | -14.202 | -3.630 | 1.00 | 33.87 | A | C |
| ATOM | 2318 | CG | LEU | A | 343 | -8.248 | -13.585 | -4.518 | 1.00 | 33.87 | A | C |
| ATOM | 2319 | CD1 | LEU | A | 343 | -7.945 | -12.193 | -4.029 | 1.00 | 33.87 | A | C |
| ATOM | 2320 | CD2 | LEU | A | 343 | -6.990 | -14.442 | -4.507 | 1.00 | 33.87 | A | C |
| ATOM | 2321 | C | LEU | A | 343 | -10.844 | -16.097 | -3.024 | 1.00 | 46.83 | A | C |
| ATOM | 2322 | O | LEU | A | 343 | -10.544 | -16.519 | -1.907 | 1.00 | 46.83 | A | O |
| ATOM | 2323 | N | ARG | A | 344 | -12.095 | -16.023 | -3.459 | 1.00 | 56.15 | A | N |
| ATOM | 2324 | CA | ARG | A | 344 | -13.225 | -16.425 | -2.628 | 1.00 | 56.15 | A | C |
| ATOM | 2325 | CB | ARG | A | 344 | -14.458 | -15.576 | -2.985 | 1.00 | 57.40 | A | C |
| ATOM | 2326 | CG | ARG | A | 344 | -14.522 | -14.247 | -2.227 | 1.00 | 57.40 | A | C |
| ATOM | 2327 | CD | ARG | A | 344 | -15.555 | -13.317 | -2.829 | 1.00 | 57.40 | A | C |
| ATOM | 2328 | NE | ARG | A | 344 | -15.090 | -12.800 | -4.114 | 1.00 | 57.40 | A | N |
| ATOM | 2329 | CZ | ARG | A | 344 | -14.531 | -11.603 | -4.286 | 1.00 | 57.40 | A | C |
| ATOM | 2330 | NH1 | ARG | A | 344 | -14.372 | -10.783 | -3.250 | 1.00 | 57.40 | A | N |
| ATOM | 2331 | NH2 | ARG | A | 344 | -14.116 | -11.233 | -5.493 | 1.00 | 57.40 | A | N |
| ATOM | 2332 | C | ARG | A | 344 | -13.523 | -17.920 | -2.750 | 1.00 | 56.15 | A | C |
| ATOM | 2333 | O | ARG | A | 344 | -14.295 | -18.490 | -1.970 | 1.00 | 56.15 | A | O |
| ATOM | 2334 | N | ASP | A | 345 | -12.899 | -18.540 | -3.743 | 1.00 | 68.84 | A | N |
| ATOM | 2335 | CA | ASP | A | 345 | -13.024 | -19.973 | -3.976 | 1.00 | 68.84 | A | C |
| ATOM | 2336 | CB | ASP | A | 345 | -12.181 | -20.325 | -5.204 | 1.00 | 65.99 | A | C |
| ATOM | 2337 | CG | ASP | A | 345 | -12.172 | -21.814 | -5.528 | 1.00 | 65.99 | A | C |
| ATOM | 2338 | OD1 | ASP | A | 345 | -12.232 | -22.660 | -4.594 | 1.00 | 65.99 | A | O |

| | | | | | | | | | | | | |
|---|---|---|---|---|---|---|---|---|---|---|---|---|
| ATOM | 2339 | OD2 | ASP | A | 345 | -12.068 | -22.128 | -6.744 | 1.00 | 65.99 | A | O |
| ATOM | 2340 | C | ASP | A | 345 | -12.464 | -20.666 | -2.705 | 1.00 | 68.84 | A | C |
| ATOM | 2341 | O | ASP | A | 345 | -11.407 | -20.273 | -2.178 | 1.00 | 68.84 | A | O |
| ATOM | 2342 | N | PRO | A | 346 | -13.162 | -21.697 | -2.191 | 1.00 | 61.03 | A | N |
| ATOM | 2343 | CD | PRO | A | 346 | -14.382 | -22.349 | -2.697 | 1.00 | 39.82 | A | C |
| ATOM | 2344 | CA | PRO | A | 346 | -12.673 | -22.384 | -0.989 | 1.00 | 61.03 | A | C |
| ATOM | 2345 | CB | PRO | A | 346 | -13.849 | -23.270 | -0.611 | 1.00 | 39.82 | A | C |
| ATOM | 2346 | CG | PRO | A | 346 | -14.358 | -23.681 | -1.959 | 1.00 | 39.82 | A | C |
| ATOM | 2347 | C | PRO | A | 346 | -11.409 | -23.197 | -1.275 | 1.00 | 61.03 | A | C |
| ATOM | 2348 | O | PRO | A | 346 | -10.673 | -23.567 | -0.359 | 1.00 | 61.03 | A | O |
| ATOM | 2349 | N | ASN | A | 347 | -11.159 | -23.454 | -2.554 | 1.00 | 48.33 | A | N |
| ATOM | 2350 | CA | ASN | A | 347 | -10.005 | -24.238 | -2.985 | 1.00 | 48.33 | A | C |
| ATOM | 2351 | CB | ASN | A | 347 | -10.374 | -25.045 | -4.229 | 1.00 | 80.59 | A | C |
| ATOM | 2352 | CG | ASN | A | 347 | -11.509 | -26.020 | -3.977 | 1.00 | 80.59 | A | C |
| ATOM | 2353 | OD1 | ASN | A | 347 | -12.282 | -26.343 | -4.893 | 1.00 | 80.59 | A | O |
| ATOM | 2354 | ND2 | ASN | A | 347 | -11.615 | -26.505 | -2.731 | 1.00 | 80.59 | A | N |
| ATOM | 2355 | C | ASN | A | 347 | -8.757 | -23.425 | -3.297 | 1.00 | 48.33 | A | C |
| ATOM | 2356 | O | ASN | A | 347 | -7.637 | -23.931 | -3.193 | 1.00 | 48.33 | A | O |
| ATOM | 2357 | N | VAL | A | 348 | -8.945 | -22.170 | -3.695 | 1.00 | 69.45 | A | N |
| ATOM | 2358 | CA | VAL | A | 348 | -7.807 | -21.324 | -4.056 | 1.00 | 69.45 | A | C |
| ATOM | 2359 | CB | VAL | A | 348 | -8.177 | -19.820 | -4.066 | 1.00 | 73.02 | A | C |
| ATOM | 2360 | CG1 | VAL | A | 348 | -8.676 | -19.394 | -2.697 | 1.00 | 73.02 | A | C |
| ATOM | 2361 | CG2 | VAL | A | 348 | -6.960 | -18.993 | -4.474 | 1.00 | 73.02 | A | C |
| ATOM | 2362 | C | VAL | A | 348 | -6.629 | -21.516 | -3.124 | 1.00 | 69.45 | A | C |
| ATOM | 2363 | O | VAL | A | 348 | -6.791 | -21.521 | -1.902 | 1.00 | 69.45 | A | O |
| ATOM | 2364 | N | LYS | A | 349 | -5.450 | -21.692 | -3.708 | 1.00 | 50.24 | A | N |
| ATOM | 2365 | CA | LYS | A | 349 | -4.237 | -21.864 | -2.920 | 1.00 | 50.24 | A | C |
| ATOM | 2366 | CB | LYS | A | 349 | -3.887 | -23.350 | -2.784 | 1.00 | 75.12 | A | C |
| ATOM | 2367 | CG | LYS | A | 349 | -5.094 | -24.228 | -2.437 | 1.00 | 75.12 | A | C |
| ATOM | 2368 | CD | LYS | A | 349 | -4.685 | -25.570 | -1.841 | 1.00 | 75.12 | A | C |
| ATOM | 2369 | CE | LYS | A | 349 | -4.213 | -25.412 | -0.393 | 1.00 | 75.12 | A | C |
| ATOM | 2370 | NZ | LYS | A | 349 | -5.328 | -24.960 | 0.496 | 1.00 | 75.12 | A | N |
| ATOM | 2371 | C | LYS | A | 349 | -3.134 | -21.123 | -3.640 | 1.00 | 50.24 | A | C |
| ATOM | 2372 | O | LYS | A | 349 | -3.277 | -20.791 | -4.813 | 1.00 | 50.24 | A | O |
| ATOM | 2373 | N | LEU | A | 350 | -2.043 | -20.837 | -2.952 | 1.00 | 40.61 | A | N |
| ATOM | 2374 | CA | LEU | A | 350 | -0.968 | -20.124 | -3.614 | 1.00 | 40.61 | A | C |
| ATOM | 2375 | CB | LEU | A | 350 | 0.012 | -19.564 | -2.585 | 1.00 | 47.11 | A | C |
| ATOM | 2376 | CG | LEU | A | 350 | -0.550 | -18.682 | -1.469 | 1.00 | 47.11 | A | C |
| ATOM | 2377 | CD1 | LEU | A | 350 | 0.555 | -18.310 | -0.498 | 1.00 | 47.11 | A | C |
| ATOM | 2378 | CD2 | LEU | A | 350 | -1.158 | -17.446 | -2.072 | 1.00 | 47.11 | A | C |
| ATOM | 2379 | C | LEU | A | 350 | -0.256 | -21.112 | -4.519 | 1.00 | 40.61 | A | C |
| ATOM | 2380 | O | LEU | A | 350 | -0.394 | -22.325 | -4.355 | 1.00 | 40.61 | A | O |
| ATOM | 2381 | N | PRO | A | 351 | 0.509 | -20.615 | -5.498 | 1.00 | 56.15 | A | N |
| ATOM | 2382 | CD | PRO | A | 351 | 0.797 | -19.213 | -5.849 | 1.00 | 61.09 | A | C |
| ATOM | 2383 | CA | PRO | A | 351 | 1.219 | -21.540 | -6.386 | 1.00 | 56.15 | A | C |
| ATOM | 2384 | CB | PRO | A | 351 | 1.747 | -20.622 | -7.488 | 1.00 | 61.09 | A | C |
| ATOM | 2385 | CG | PRO | A | 351 | 2.001 | -19.345 | -6.763 | 1.00 | 61.09 | A | C |
| ATOM | 2386 | C | PRO | A | 351 | 2.330 | -22.180 | -5.558 | 1.00 | 56.15 | A | C |
| ATOM | 2387 | O | PRO | A | 351 | 3.202 | -22.891 | -6.058 | 1.00 | 56.15 | A | O |
| ATOM | 2388 | N | ASN | A | 352 | 2.255 | -21.906 | -4.266 | 1.00 | 54.62 | A | N |
| ATOM | 2389 | CA | ASN | A | 352 | 3.194 | -22.381 | -3.269 | 1.00 | 54.62 | A | C |
| ATOM | 2390 | CB | ASN | A | 352 | 3.311 | -21.324 | -2.180 | 1.00 | 69.15 | A | C |
| ATOM | 2391 | CG | ASN | A | 352 | 4.678 | -21.262 | -1.599 | 1.00 | 69.15 | A | C |
| ATOM | 2392 | OD1 | ASN | A | 352 | 4.996 | -20.356 | -0.825 | 1.00 | 69.15 | A | O |
| ATOM | 2393 | ND2 | ASN | A | 352 | 5.520 | -22.231 | -1.971 | 1.00 | 69.15 | A | N |
| ATOM | 2394 | C | ASN | A | 352 | 2.711 | -23.676 | -2.631 | 1.00 | 54.62 | A | C |
| ATOM | 2395 | O | ASN | A | 352 | 3.450 | -24.323 | -1.889 | 1.00 | 54.62 | A | O |
| ATOM | 2396 | N | GLY | A | 353 | 1.464 | -24.040 | -2.920 | 1.00 | 62.87 | A | N |
| ATOM | 2397 | CA | GLY | A | 353 | 0.875 | -25.230 | -2.336 | 1.00 | 62.87 | A | C |
| ATOM | 2398 | C | GLY | A | 353 | 0.216 | -24.806 | -1.033 | 1.00 | 62.87 | A | C |
| ATOM | 2399 | O | GLY | A | 353 | -0.768 | -25.409 | -0.566 | 1.00 | 62.87 | A | O |
| ATOM | 2400 | N | ARG | A | 354 | 0.763 | -23.732 | -0.461 | 1.00 | 80.42 | A | N |
| ATOM | 2401 | CA | ARG | A | 354 | 0.289 | -23.165 | 0.806 | 1.00 | 80.42 | A | C |
| ATOM | 2402 | CB | ARG | A | 354 | 1.345 | -22.224 | 1.376 | 1.00 | 87.86 | A | C |
| ATOM | 2403 | CG | ARG | A | 354 | 2.730 | -22.831 | 1.394 | 1.00 | 87.86 | A | C |
| ATOM | 2404 | CD | ARG | A | 354 | 3.787 | -21.819 | 1.809 | 1.00 | 87.86 | A | C |
| ATOM | 2405 | NE | ARG | A | 354 | 3.427 | -21.151 | 3.059 | 1.00 | 87.86 | A | N |

| | | | | | | | | | | | | |
|---|---|---|---|---|---|---|---|---|---|---|---|---|
| ATOM | 2406 | CZ | ARG | A | 354 | 4.304 | -20.573 | 3.878 | 1.00 | 87.86 | A | C |
| ATOM | 2407 | NH1 | ARG | A | 354 | 5.606 | -20.583 | 3.579 | 1.00 | 87.86 | A | N |
| ATOM | 2408 | NH2 | ARG | A | 354 | 3.878 | -19.989 | 4.997 | 1.00 | 87.86 | A | N |
| ATOM | 2409 | C | ARG | A | 354 | -1.002 | -22.393 | 0.604 | 1.00 | 80.42 | A | C |
| ATOM | 2410 | O | ARG | A | 354 | -1.302 | -21.938 | -0.511 | 1.00 | 80.42 | A | O |
| ATOM | 2411 | N | ASP | A | 355 | -1.765 | -22.235 | 1.677 | 1.00 | 63.73 | A | N |
| ATOM | 2412 | CA | ASP | A | 355 | -3.017 | -21.506 | 1.576 | 1.00 | 63.73 | A | C |
| ATOM | 2413 | CB | ASP | A | 355 | -3.899 | -21.794 | 2.778 | 1.00 | 69.07 | A | C |
| ATOM | 2414 | CG | ASP | A | 355 | -3.886 | -23.247 | 3.155 | 1.00 | 69.07 | A | C |
| ATOM | 2415 | OD1 | ASP | A | 355 | -2.823 | -23.696 | 3.652 | 1.00 | 69.07 | A | O |
| ATOM | 2416 | OD2 | ASP | A | 355 | -4.919 | -23.934 | 2.945 | 1.00 | 69.07 | A | O |
| ATOM | 2417 | C | ASP | A | 355 | -2.748 | -20.016 | 1.493 | 1.00 | 63.73 | A | C |
| ATOM | 2418 | O | ASP | A | 355 | -1.652 | -19.544 | 1.815 | 1.00 | 63.73 | A | O |
| ATOM | 2419 | N | THR | A | 356 | -3.757 | -19.286 | 1.038 | 1.00 | 46.73 | A | N |
| ATOM | 2420 | CA | THR | A | 356 | -3.661 | -17.845 | 0.912 | 1.00 | 46.73 | A | C |
| ATOM | 2421 | CB | THR | A | 356 | -4.771 | -17.303 | 0.018 | 1.00 | 52.88 | A | C |
| ATOM | 2422 | OG1 | THR | A | 356 | -6.028 | -17.818 | 0.473 | 1.00 | 52.88 | A | O |
| ATOM | 2423 | CG2 | THR | A | 356 | -4.550 | -17.725 | -1.423 | 1.00 | 52.88 | A | C |
| ATOM | 2424 | C | THR | A | 356 | -3.842 | -17.250 | 2.293 | 1.00 | 46.73 | A | C |
| ATOM | 2425 | O | THR | A | 356 | -4.486 | -17.849 | 3.154 | 1.00 | 46.73 | A | O |
| ATOM | 2426 | N | PRO | A | 357 | -3.269 | -16.062 | 2.526 | 1.00 | 51.89 | A | N |
| ATOM | 2427 | CD | PRO | A | 357 | -2.511 | -15.190 | 1.612 | 1.00 | 50.65 | A | C |
| ATOM | 2428 | CA | PRO | A | 357 | -3.420 | -15.441 | 3.841 | 1.00 | 51.89 | A | C |
| ATOM | 2429 | CB | PRO | A | 357 | -2.671 | -14.114 | 3.683 | 1.00 | 50.65 | A | C |
| ATOM | 2430 | CG | PRO | A | 357 | -2.755 | -13.834 | 2.200 | 1.00 | 50.65 | A | C |
| ATOM | 2431 | C | PRO | A | 357 | -4.902 | -15.253 | 4.155 | 1.00 | 51.89 | A | C |
| ATOM | 2432 | O | PRO | A | 357 | -5.760 | -15.576 | 3.332 | 1.00 | 51.89 | A | O |
| ATOM | 2433 | N | ALA | A | 358 | -5.201 | -14.755 | 5.350 | 1.00 | 59.43 | A | N |
| ATOM | 2434 | CA | ALA | A | 358 | -6.587 | -14.511 | 5.740 | 1.00 | 59.43 | A | C |
| ATOM | 2435 | CB | ALA | A | 358 | -6.657 | -14.204 | 7.233 | 1.00 | 59.13 | A | C |
| ATOM | 2436 | C | ALA | A | 358 | -7.110 | -13.319 | 4.929 | 1.00 | 59.43 | A | C |
| ATOM | 2437 | O | ALA | A | 358 | -6.674 | -12.193 | 5.136 | 1.00 | 59.43 | A | O |
| ATOM | 2438 | N | LEU | A | 359 | -8.041 | -13.555 | 4.015 | 1.00 | 57.16 | A | N |
| ATOM | 2439 | CA | LEU | A | 359 | -8.552 | -12.466 | 3.191 | 1.00 | 57.16 | A | C |
| ATOM | 2440 | CB | LEU | A | 359 | -8.435 | -12.853 | 1.717 | 1.00 | 46.83 | A | C |
| ATOM | 2441 | CG | LEU | A | 359 | -7.035 | -13.351 | 1.361 | 1.00 | 46.83 | A | C |
| ATOM | 2442 | CD1 | LEU | A | 359 | -7.017 | -13.954 | -0.042 | 1.00 | 46.83 | A | C |
| ATOM | 2443 | CD2 | LEU | A | 359 | -6.059 | -12.191 | 1.491 | 1.00 | 46.83 | A | C |
| ATOM | 2444 | C | LEU | A | 359 | -9.992 | -12.084 | 3.498 | 1.00 | 57.16 | A | C |
| ATOM | 2445 | O | LEU | A | 359 | -10.581 | -11.264 | 2.783 | 1.00 | 57.16 | A | O |
| ATOM | 2446 | N | PHE | A | 360 | -10.550 | -12.651 | 4.567 | 1.00 | 65.53 | A | N |
| ATOM | 2447 | CA | PHE | A | 360 | -11.943 | -12.388 | 4.904 | 1.00 | 65.53 | A | C |
| ATOM | 2448 | CB | PHE | A | 360 | -12.786 | -13.628 | 4.603 | 1.00 | 62.70 | A | C |
| ATOM | 2449 | CG | PHE | A | 360 | -12.464 | -14.274 | 3.284 | 1.00 | 62.70 | A | C |
| ATOM | 2450 | CD1 | PHE | A | 360 | -12.665 | -13.588 | 2.090 | 1.00 | 62.70 | A | C |
| ATOM | 2451 | CD2 | PHE | A | 360 | -11.948 | -15.570 | 3.237 | 1.00 | 62.70 | A | C |
| ATOM | 2452 | CE1 | PHE | A | 360 | -12.357 | -14.180 | 0.863 | 1.00 | 62.70 | A | C |
| ATOM | 2453 | CE2 | PHE | A | 360 | -11.635 | -16.173 | 2.015 | 1.00 | 62.70 | A | C |
| ATOM | 2454 | CZ | PHE | A | 360 | -11.841 | -15.475 | 0.821 | 1.00 | 62.70 | A | C |
| ATOM | 2455 | C | PHE | A | 360 | -12.220 | -11.959 | 6.336 | 1.00 | 65.53 | A | C |
| ATOM | 2456 | O | PHE | A | 360 | -13.361 | -11.611 | 6.653 | 1.00 | 65.53 | A | O |
| ATOM | 2457 | N | ASN | A | 361 | -11.217 | -11.993 | 7.211 | 1.00 | 44.89 | A | N |
| ATOM | 2458 | CA | ASN | A | 361 | -11.464 | -11.597 | 8.589 | 1.00 | 44.89 | A | C |
| ATOM | 2459 | CB | ASN | A | 361 | -10.337 | -12.095 | 9.517 | 1.00 | 67.57 | A | C |
| ATOM | 2460 | CG | ASN | A | 361 | -8.949 | -11.672 | 9.056 | 1.00 | 67.57 | A | C |
| ATOM | 2461 | OD1 | ASN | A | 361 | -8.590 | -11.842 | 7.886 | 1.00 | 67.57 | A | O |
| ATOM | 2462 | ND2 | ASN | A | 361 | -8.150 | -11.129 | 9.984 | 1.00 | 67.57 | A | N |
| ATOM | 2463 | C | ASN | A | 361 | -11.641 | -10.086 | 8.652 | 1.00 | 44.89 | A | C |
| ATOM | 2464 | O | ASN | A | 361 | -10.796 | -9.353 | 9.158 | 1.00 | 44.89 | A | O |
| ATOM | 2465 | N | PHE | A | 362 | -12.762 | -9.632 | 8.108 | 1.00 | 49.94 | A | N |
| ATOM | 2466 | CA | PHE | A | 362 | -13.109 | -8.219 | 8.065 | 1.00 | 49.94 | A | C |
| ATOM | 2467 | CB | PHE | A | 362 | -14.157 | -7.967 | 6.983 | 1.00 | 51.09 | A | C |
| ATOM | 2468 | CG | PHE | A | 362 | -13.615 | -7.949 | 5.590 | 1.00 | 51.09 | A | C |
| ATOM | 2469 | CD1 | PHE | A | 362 | -13.177 | -6.756 | 5.023 | 1.00 | 51.09 | A | C |
| ATOM | 2470 | CD2 | PHE | A | 362 | -13.564 | -9.119 | 4.834 | 1.00 | 51.09 | A | C |
| ATOM | 2471 | CE1 | PHE | A | 362 | -12.697 | -6.725 | 3.714 | 1.00 | 51.09 | A | C |
| ATOM | 2472 | CE2 | PHE | A | 362 | -13.088 | -9.101 | 3.535 | 1.00 | 51.09 | A | C |

| | | | | | | | | | | | | |
|---|---|---|---|---|---|---|---|---|---|---|---|---|
| ATOM | 2473 | CZ | PHE | A | 362 | -12.653 | -7.902 | 2.971 | 1.00 | 51.09 | A | C |
| ATOM | 2474 | C | PHE | A | 362 | -13.711 | -7.779 | 9.384 | 1.00 | 49.94 | A | C |
| ATOM | 2475 | O | PHE | A | 362 | -14.428 | -8.549 | 10.030 | 1.00 | 49.94 | A | O |
| ATOM | 2476 | N | THR | A | 363 | -13.444 | -6.532 | 9.765 | 1.00 | 64.01 | A | N |
| ATOM | 2477 | CA | THR | A | 363 | -14.000 | -5.978 | 10.995 | 1.00 | 64.01 | A | C |
| ATOM | 2478 | CB | THR | A | 363 | -12.918 | -5.308 | 11.847 | 1.00 | 55.01 | A | C |
| ATOM | 2479 | OG1 | THR | A | 363 | -12.420 | -4.146 | 11.162 | 1.00 | 55.01 | A | O |
| ATOM | 2480 | CG2 | THR | A | 363 | -11.782 | -6.292 | 12.116 | 1.00 | 55.01 | A | C |
| ATOM | 2481 | C | THR | A | 363 | -15.048 | -4.928 | 10.628 | 1.00 | 64.01 | A | C |
| ATOM | 2482 | O | THR | A | 363 | -14.932 | -4.271 | 9.585 | 1.00 | 64.01 | A | O |
| ATOM | 2483 | N | THR | A | 364 | -16.061 | -4.766 | 11.478 | 1.00 | 67.58 | A | N |
| ATOM | 2484 | CA | THR | A | 364 | -17.127 | -3.793 | 11.225 | 1.00 | 67.58 | A | C |
| ATOM | 2485 | CB | THR | A | 364 | -18.003 | -3.570 | 12.457 | 1.00 | 49.68 | A | C |
| ATOM | 2486 | OG1 | THR | A | 364 | -17.342 | -2.650 | 13.336 | 1.00 | 49.68 | A | O |
| ATOM | 2487 | CG2 | THR | A | 364 | -18.261 | -4.896 | 13.172 | 1.00 | 49.68 | A | C |
| ATOM | 2488 | C | THR | A | 364 | -16.553 | -2.438 | 10.824 | 1.00 | 67.58 | A | C |
| ATOM | 2489 | O | THR | A | 364 | -17.209 | -1.653 | 10.128 | 1.00 | 67.58 | A | O |
| ATOM | 2490 | N | GLN | A | 365 | -15.337 | -2.162 | 11.283 | 1.00 | 41.43 | A | N |
| ATOM | 2491 | CA | GLN | A | 365 | -14.676 | -0.912 | 10.950 | 1.00 | 41.43 | A | C |
| ATOM | 2492 | CB | GLN | A | 365 | -13.399 | -0.749 | 11.778 | 1.00 | 50.15 | A | C |
| ATOM | 2493 | CG | GLN | A | 365 | -12.534 | 0.438 | 11.372 | 1.00 | 50.15 | A | C |
| ATOM | 2494 | CD | GLN | A | 365 | -13.122 | 1.773 | 11.780 | 1.00 | 50.15 | A | C |
| ATOM | 2495 | OE1 | GLN | A | 365 | -14.251 | 1.847 | 12.276 | 1.00 | 50.15 | A | O |
| ATOM | 2496 | NE2 | GLN | A | 365 | -12.359 | 2.843 | 11.572 | 1.00 | 50.15 | A | N |
| ATOM | 2497 | C | GLN | A | 365 | -14.329 | -0.931 | 9.466 | 1.00 | 41.43 | A | C |
| ATOM | 2498 | O | GLN | A | 365 | -14.517 | 0.055 | 8.767 | 1.00 | 41.43 | A | O |
| ATOM | 2499 | N | GLU | A | 366 | -13.822 | -2.061 | 8.987 | 1.00 | 41.89 | A | N |
| ATOM | 2500 | CA | GLU | A | 366 | -13.447 | -2.184 | 7.584 | 1.00 | 41.89 | A | C |
| ATOM | 2501 | CB | GLU | A | 366 | -12.732 | -3.523 | 7.312 | 1.00 | 64.07 | A | C |
| ATOM | 2502 | CG | GLU | A | 366 | -11.487 | -3.833 | 8.150 | 1.00 | 64.07 | A | C |
| ATOM | 2503 | CD | GLU | A | 366 | -10.799 | -5.132 | 7.710 | 1.00 | 64.07 | A | C |
| ATOM | 2504 | OE1 | GLU | A | 366 | -10.265 | -5.177 | 6.572 | 1.00 | 64.07 | A | O |
| ATOM | 2505 | OE2 | GLU | A | 366 | -10.800 | -6.110 | 8.502 | 1.00 | 64.07 | A | O |
| ATOM | 2506 | C | GLU | A | 366 | -14.706 | -2.128 | 6.724 | 1.00 | 41.89 | A | C |
| ATOM | 2507 | O | GLU | A | 366 | -14.765 | -1.411 | 5.718 | 1.00 | 41.89 | A | O |
| ATOM | 2508 | N | LEU | A | 367 | -15.710 | -2.897 | 7.137 | 1.00 | 44.41 | A | N |
| ATOM | 2509 | CA | LEU | A | 367 | -16.972 | -2.992 | 6.419 | 1.00 | 44.41 | A | C |
| ATOM | 2510 | CB | LEU | A | 367 | -17.699 | -4.262 | 6.846 | 1.00 | 52.04 | A | C |
| ATOM | 2511 | CG | LEU | A | 367 | -16.933 | -5.573 | 6.660 | 1.00 | 52.04 | A | C |
| ATOM | 2512 | CD1 | LEU | A | 367 | -17.418 | -6.595 | 7.679 | 1.00 | 52.04 | A | C |
| ATOM | 2513 | CD2 | LEU | A | 367 | -17.109 | -6.082 | 5.234 | 1.00 | 52.04 | A | C |
| ATOM | 2514 | C | LEU | A | 367 | -17.891 | -1.800 | 6.650 | 1.00 | 44.41 | A | C |
| ATOM | 2515 | O | LEU | A | 367 | -18.960 | -1.707 | 6.047 | 1.00 | 44.41 | A | O |
| ATOM | 2516 | N | SER | A | 368 | -17.475 | -0.877 | 7.509 | 1.00 | 55.71 | A | N |
| ATOM | 2517 | CA | SER | A | 368 | -18.313 | 0.273 | 7.826 | 1.00 | 55.71 | A | C |
| ATOM | 2518 | CB | SER | A | 368 | -17.559 | 1.237 | 8.745 | 1.00 | 56.33 | A | C |
| ATOM | 2519 | OG | SER | A | 368 | -16.431 | 1.788 | 8.089 | 1.00 | 56.33 | A | O |
| ATOM | 2520 | C | SER | A | 368 | -18.902 | 1.045 | 6.638 | 1.00 | 55.71 | A | C |
| ATOM | 2521 | O | SER | A | 368 | -20.117 | 1.249 | 6.592 | 1.00 | 55.71 | A | O |
| ATOM | 2522 | N | SER | A | 369 | -18.063 | 1.455 | 5.682 | 1.00 | 48.39 | A | N |
| ATOM | 2523 | CA | SER | A | 369 | -18.533 | 2.233 | 4.527 | 1.00 | 48.39 | A | C |
| ATOM | 2524 | CB | SER | A | 369 | -17.390 | 2.505 | 3.545 | 1.00 | 45.64 | A | C |
| ATOM | 2525 | OG | SER | A | 369 | -17.409 | 1.583 | 2.467 | 1.00 | 45.64 | A | O |
| ATOM | 2526 | C | SER | A | 369 | -19.697 | 1.619 | 3.753 | 1.00 | 48.39 | A | C |
| ATOM | 2527 | O | SER | A | 369 | -20.434 | 2.334 | 3.080 | 1.00 | 48.39 | A | O |
| ATOM | 2528 | N | ASN | A | 370 | -19.865 | 0.304 | 3.840 | 1.00 | 51.22 | A | N |
| ATOM | 2529 | CA | ASN | A | 370 | -20.956 | -0.349 | 3.122 | 1.00 | 51.22 | A | C |
| ATOM | 2530 | CB | ASN | A | 370 | -20.655 | -0.376 | 1.630 | 1.00 | 61.82 | A | C |
| ATOM | 2531 | CG | ASN | A | 370 | -21.829 | -0.844 | 0.828 | 1.00 | 61.82 | A | C |
| ATOM | 2532 | OD1 | ASN | A | 370 | -22.379 | -1.924 | 1.085 | 1.00 | 61.82 | A | O |
| ATOM | 2533 | ND2 | ASN | A | 370 | -22.239 | -0.037 | -0.148 | 1.00 | 61.82 | A | N |
| ATOM | 2534 | C | ASN | A | 370 | -21.208 | -1.776 | 3.609 | 1.00 | 51.22 | A | C |
| ATOM | 2535 | O | ASN | A | 370 | -20.988 | -2.750 | 2.881 | 1.00 | 51.22 | A | O |
| ATOM | 2536 | N | PRO | A | 371 | -21.701 | -1.912 | 4.845 | 1.00 | 57.40 | A | N |
| ATOM | 2537 | CD | PRO | A | 371 | -22.101 | -0.795 | 5.719 | 1.00 | 29.62 | A | C |
| ATOM | 2538 | CA | PRO | A | 371 | -21.998 | -3.193 | 5.484 | 1.00 | 57.40 | A | C |
| ATOM | 2539 | CB | PRO | A | 371 | -23.016 | -2.799 | 6.542 | 1.00 | 29.62 | A | C |

| | | | | | | | | | | | | |
|---|---|---|---|---|---|---|---|---|---|---|---|---|
| ATOM | 2540 | CG | PRO | A | 371 | -22.450 | -1.498 | 7.012 | 1.00 | 29.62 | A | C |
| ATOM | 2541 | C | PRO | A | 371 | -22.453 | -4.376 | 4.614 | 1.00 | 57.40 | A | C |
| ATOM | 2542 | O | PRO | A | 371 | -21.766 | -5.400 | 4.531 | 1.00 | 57.40 | A | O |
| ATOM | 2543 | N | PRO | A | 372 | -23.600 | -4.251 | 3.938 | 1.00 | 72.78 | A | N |
| ATOM | 2544 | CD | PRO | A | 372 | -24.408 | -3.053 | 3.657 | 1.00 | 59.42 | A | C |
| ATOM | 2545 | CA | PRO | A | 372 | -24.046 | -5.379 | 3.115 | 1.00 | 72.78 | A | C |
| ATOM | 2546 | CB | PRO | A | 372 | -25.294 | -4.829 | 2.424 | 1.00 | 59.42 | A | C |
| ATOM | 2547 | CG | PRO | A | 372 | -24.985 | -3.383 | 2.295 | 1.00 | 59.42 | A | C |
| ATOM | 2548 | C | PRO | A | 372 | -23.047 | -5.997 | 2.128 | 1.00 | 72.78 | A | C |
| ATOM | 2549 | O | PRO | A | 372 | -23.314 | -7.076 | 1.598 | 1.00 | 72.78 | A | O |
| ATOM | 2550 | N | LEU | A | 373 | -21.912 | -5.343 | 1.868 | 1.00 | 63.86 | A | N |
| ATOM | 2551 | CA | LEU | A | 373 | -20.935 | -5.920 | 0.936 | 1.00 | 63.86 | A | C |
| ATOM | 2552 | CB | LEU | A | 373 | -19.814 | -4.927 | 0.648 | 1.00 | 45.84 | A | C |
| ATOM | 2553 | CG | LEU | A | 373 | -20.137 | -3.974 | -0.509 | 1.00 | 45.84 | A | C |
| ATOM | 2554 | CD1 | LEU | A | 373 | -19.069 | -2.895 | -0.634 | 1.00 | 45.84 | A | C |
| ATOM | 2555 | CD2 | LEU | A | 373 | -20.237 | -4.789 | -1.796 | 1.00 | 45.84 | A | C |
| ATOM | 2556 | C | LEU | A | 373 | -20.366 | -7.208 | 1.518 | 1.00 | 63.86 | A | C |
| ATOM | 2557 | O | LEU | A | 373 | -19.760 | -8.029 | 0.816 | 1.00 | 63.86 | A | O |
| ATOM | 2558 | N | ALA | A | 374 | -20.603 | -7.389 | 2.812 | 1.00 | 60.89 | A | N |
| ATOM | 2559 | CA | ALA | A | 374 | -20.128 | -8.557 | 3.530 | 1.00 | 60.89 | A | C |
| ATOM | 2560 | CB | ALA | A | 374 | -20.497 | -8.436 | 5.004 | 1.00 | 39.32 | A | C |
| ATOM | 2561 | C | ALA | A | 374 | -20.667 | -9.863 | 2.959 | 1.00 | 60.89 | A | C |
| ATOM | 2562 | O | ALA | A | 374 | -20.058 | -10.924 | 3.144 | 1.00 | 60.89 | A | O |
| ATOM | 2563 | N | THR | A | 375 | -21.800 | -9.803 | 2.269 | 1.00 | 55.55 | A | N |
| ATOM | 2564 | CA | THR | A | 375 | -22.370 | -11.025 | 1.709 | 1.00 | 55.55 | A | C |
| ATOM | 2565 | CB | THR | A | 375 | -23.818 | -10.832 | 1.221 | 1.00 | 73.65 | A | C |
| ATOM | 2566 | OG1 | THR | A | 375 | -23.888 | -9.685 | 0.362 | 1.00 | 73.65 | A | O |
| ATOM | 2567 | CG2 | THR | A | 375 | -24.763 | -10.667 | 2.410 | 1.00 | 73.65 | A | C |
| ATOM | 2568 | C | THR | A | 375 | -21.536 | -11.504 | 0.543 | 1.00 | 55.55 | A | C |
| ATOM | 2569 | O | THR | A | 375 | -21.614 | -12.674 | 0.149 | 1.00 | 55.55 | A | O |
| ATOM | 2570 | N | ILE | A | 376 | -20.735 | -10.598 | -0.013 | 1.00 | 60.14 | A | N |
| ATOM | 2571 | CA | ILE | A | 376 | -19.876 | -10.964 | -1.129 | 1.00 | 60.14 | A | C |
| ATOM | 2572 | CB | ILE | A | 376 | -19.880 | -9.887 | -2.237 | 1.00 | 45.17 | A | C |
| ATOM | 2573 | CG2 | ILE | A | 376 | -19.032 | -10.354 | -3.413 | 1.00 | 45.17 | A | C |
| ATOM | 2574 | CG1 | ILE | A | 376 | -21.309 | -9.625 | -2.719 | 1.00 | 45.17 | A | C |
| ATOM | 2575 | CD1 | ILE | A | 376 | -21.389 | -8.620 | -3.852 | 1.00 | 45.17 | A | C |
| ATOM | 2576 | C | ILE | A | 376 | -18.443 | -11.152 | -0.640 | 1.00 | 60.14 | A | C |
| ATOM | 2577 | O | ILE | A | 376 | -17.776 | -12.136 | -0.987 | 1.00 | 60.14 | A | O |
| ATOM | 2578 | N | LEU | A | 377 | -17.977 | -10.213 | 0.179 | 1.00 | 63.34 | A | N |
| ATOM | 2579 | CA | LEU | A | 377 | -16.613 | -10.266 | 0.690 | 1.00 | 63.34 | A | C |
| ATOM | 2580 | CB | LEU | A | 377 | -16.305 | -8.985 | 1.457 | 1.00 | 35.39 | A | C |
| ATOM | 2581 | CG | LEU | A | 377 | -16.416 | -7.775 | 0.529 | 1.00 | 35.39 | A | C |
| ATOM | 2582 | CD1 | LEU | A | 377 | -16.396 | -6.496 | 1.340 | 1.00 | 35.39 | A | C |
| ATOM | 2583 | CD2 | LEU | A | 377 | -15.292 | -7.804 | -0.483 | 1.00 | 35.39 | A | C |
| ATOM | 2584 | C | LEU | A | 377 | -16.319 | -11.493 | 1.552 | 1.00 | 63.34 | A | C |
| ATOM | 2585 | O | LEU | A | 377 | -15.225 | -12.068 | 1.463 | 1.00 | 63.34 | A | O |
| ATOM | 2586 | N | ILE | A | 378 | -17.286 | -11.902 | 2.373 | 1.00 | 67.00 | A | N |
| ATOM | 2587 | CA | ILE | A | 378 | -17.096 | -13.063 | 3.235 | 1.00 | 67.00 | A | C |
| ATOM | 2588 | CB | ILE | A | 378 | -17.598 | -12.800 | 4.664 | 1.00 | 48.05 | A | C |
| ATOM | 2589 | CG2 | ILE | A | 378 | -17.451 | -14.064 | 5.496 | 1.00 | 48.05 | A | C |
| ATOM | 2590 | CG1 | ILE | A | 378 | -16.798 | -11.663 | 5.307 | 1.00 | 48.05 | A | C |
| ATOM | 2591 | CD1 | ILE | A | 378 | -17.311 | -11.238 | 6.671 | 1.00 | 48.05 | A | C |
| ATOM | 2592 | C | ILE | A | 378 | -17.860 | -14.250 | 2.669 | 1.00 | 67.00 | A | C |
| ATOM | 2593 | O | ILE | A | 378 | -19.029 | -14.469 | 3.006 | 1.00 | 67.00 | A | O |
| ATOM | 2594 | N | PRO | A | 379 | -17.210 | -15.036 | 1.796 | 1.00 | 69.87 | A | N |
| ATOM | 2595 | CD | PRO | A | 379 | -15.769 | -15.039 | 1.490 | 1.00 | 61.17 | A | C |
| ATOM | 2596 | CA | PRO | A | 379 | -17.870 | -16.196 | 1.199 | 1.00 | 69.87 | A | C |
| ATOM | 2597 | CB | PRO | A | 379 | -16.768 | -16.818 | 0.342 | 1.00 | 61.17 | A | C |
| ATOM | 2598 | CG | PRO | A | 379 | -15.526 | -16.487 | 1.105 | 1.00 | 61.17 | A | C |
| ATOM | 2599 | C | PRO | A | 379 | -18.364 | -17.126 | 2.290 | 1.00 | 69.87 | A | C |
| ATOM | 2600 | O | PRO | A | 379 | -17.868 | -17.084 | 3.424 | 1.00 | 69.87 | A | O |
| ATOM | 2601 | N | PRO | A | 380 | -19.355 | -17.977 | 1.966 | 1.00 | 73.20 | A | N |
| ATOM | 2602 | CD | PRO | A | 380 | -20.011 | -18.078 | 0.650 | 1.00 | 70.26 | A | C |
| ATOM | 2603 | CA | PRO | A | 380 | -19.945 | -18.935 | 2.909 | 1.00 | 73.20 | A | C |
| ATOM | 2604 | CB | PRO | A | 380 | -20.908 | -19.721 | 2.032 | 1.00 | 70.26 | A | C |
| ATOM | 2605 | CG | PRO | A | 380 | -21.334 | -18.689 | 1.018 | 1.00 | 70.26 | A | C |
| ATOM | 2606 | C | PRO | A | 380 | -18.909 | -19.833 | 3.576 | 1.00 | 73.20 | A | C |

| | | | | | | | | | | | | |
|---|---|---|---|---|---|---|---|---|---|---|---|---|
| ATOM | 2607 | O | PRO | A | 380 | -18.876 | -19.956 | 4.810 | 1.00 | 73.20 | A | O |
| ATOM | 2608 | N | HIS | A | 381 | -18.052 | -20.437 | 2.752 | 1.00 | 52.54 | A | N |
| ATOM | 2609 | CA | HIS | A | 381 | -17.029 | -21.337 | 3.257 | 1.00 | 52.54 | A | C |
| ATOM | 2610 | CB | HIS | A | 381 | -16.143 | -21.818 | 2.105 | 1.00 | 74.51 | A | C |
| ATOM | 2611 | CG | HIS | A | 381 | -14.978 | -20.924 | 1.812 | 1.00 | 74.51 | A | C |
| ATOM | 2612 | CD2 | HIS | A | 381 | -14.706 | -20.131 | 0.747 | 1.00 | 74.51 | A | C |
| ATOM | 2613 | ND1 | HIS | A | 381 | -13.891 | -20.817 | 2.656 | 1.00 | 74.51 | A | N |
| ATOM | 2614 | CE1 | HIS | A | 381 | -12.999 | -20.001 | 2.120 | 1.00 | 74.51 | A | C |
| ATOM | 2615 | NE2 | HIS | A | 381 | -13.468 | -19.571 | 0.960 | 1.00 | 74.51 | A | N |
| ATOM | 2616 | C | HIS | A | 381 | -16.183 | -20.698 | 4.366 | 1.00 | 52.54 | A | C |
| ATOM | 2617 | O | HIS | A | 381 | -15.547 | -21.402 | 5.151 | 1.00 | 52.54 | A | O |
| ATOM | 2618 | N | ALA | A | 382 | -16.166 | -19.367 | 4.431 | 1.00 | 81.03 | A | N |
| ATOM | 2619 | CA | ALA | A | 382 | -15.404 | -18.671 | 5.468 | 1.00 | 81.03 | A | C |
| ATOM | 2620 | CB | ALA | A | 382 | -15.106 | -17.257 | 5.035 | 1.00 | 72.20 | A | C |
| ATOM | 2621 | C | ALA | A | 382 | -16.207 | -18.667 | 6.771 | 1.00 | 81.03 | A | C |
| ATOM | 2622 | O | ALA | A | 382 | -15.638 | -18.783 | 7.868 | 1.00 | 81.03 | A | O |
| ATOM | 2623 | N | ARG | A | 383 | -17.530 | -18.534 | 6.636 | 1.00 | 93.51 | A | N |
| ATOM | 2624 | CA | ARG | A | 383 | -18.454 | -18.535 | 7.777 | 1.00 | 93.51 | A | C |
| ATOM | 2625 | CB | ARG | A | 383 | -19.905 | -18.383 | 7.285 | 1.00 | 78.07 | A | C |
| ATOM | 2626 | CG | ARG | A | 383 | -20.266 | -16.980 | 6.825 | 1.00 | 78.07 | A | C |
| ATOM | 2627 | CD | ARG | A | 383 | -20.412 | -16.847 | 5.312 | 1.00 | 78.07 | A | C |
| ATOM | 2628 | NE | ARG | A | 383 | -21.809 | -16.588 | 4.931 | 1.00 | 78.07 | A | N |
| ATOM | 2629 | CZ | ARG | A | 383 | -22.225 | -16.157 | 3.730 | 1.00 | 78.07 | A | C |
| ATOM | 2630 | NH1 | ARG | A | 383 | -21.360 | -15.916 | 2.739 | 1.00 | 78.07 | A | N |
| ATOM | 2631 | NH2 | ARG | A | 383 | -23.527 | -15.957 | 3.520 | 1.00 | 78.07 | A | N |
| ATOM | 2632 | C | ARG | A | 383 | -18.330 | -19.813 | 8.638 | 1.00 | 93.51 | A | C |
| ATOM | 2633 | O | ARG | A | 383 | -18.677 | -19.743 | 9.845 | 1.00 | 93.51 | A | O |
| ATOM | 2634 | OXT | ARG | A | 383 | -17.909 | -20.875 | 8.098 | 1.00 | 78.07 | A | O |
| TER | 2635 | | ARG | A | 383 | | | | | | A | |
| ATOM | 2636 | CB | VAL | B | 37 | -19.635 | 28.376 | 41.499 | 1.00 | 50.53 | B | C |
| ATOM | 2637 | CG1 | VAL | B | 37 | -18.762 | 29.367 | 42.258 | 1.00 | 50.53 | B | C |
| ATOM | 2638 | CG2 | VAL | B | 37 | -19.080 | 28.209 | 40.079 | 1.00 | 50.53 | B | C |
| ATOM | 2639 | C | VAL | B | 37 | -19.604 | 27.308 | 43.741 | 1.00 | 61.52 | B | C |
| ATOM | 2640 | O | VAL | B | 37 | -20.550 | 27.856 | 44.296 | 1.00 | 61.52 | B | O |
| ATOM | 2641 | N | VAL | B | 37 | -20.798 | 26.159 | 41.802 | 1.00 | 61.52 | B | N |
| ATOM | 2642 | CA | VAL | B | 37 | -19.647 | 27.015 | 42.239 | 1.00 | 61.52 | B | C |
| ATOM | 2643 | N | THR | B | 38 | -18.496 | 26.945 | 44.388 | 1.00 | 54.53 | B | N |
| ATOM | 2644 | CA | THR | B | 38 | -18.306 | 27.186 | 45.824 | 1.00 | 54.53 | B | C |
| ATOM | 2645 | CB | THR | B | 38 | -17.681 | 25.969 | 46.531 | 1.00 | 49.70 | B | C |
| ATOM | 2646 | OG1 | THR | B | 38 | -18.449 | 24.793 | 46.247 | 1.00 | 49.70 | B | O |
| ATOM | 2647 | CG2 | THR | B | 38 | -17.643 | 26.192 | 48.035 | 1.00 | 49.70 | B | C |
| ATOM | 2648 | C | THR | B | 38 | -17.345 | 28.364 | 46.016 | 1.00 | 54.53 | B | C |
| ATOM | 2649 | O | THR | B | 38 | -16.373 | 28.516 | 45.265 | 1.00 | 54.53 | B | O |
| ATOM | 2650 | N | THR | B | 39 | -17.625 | 29.199 | 47.015 | 1.00 | 56.75 | B | N |
| ATOM | 2651 | CA | THR | B | 39 | -16.783 | 30.361 | 47.326 | 1.00 | 56.75 | B | C |
| ATOM | 2652 | CB | THR | B | 39 | -17.495 | 31.706 | 46.989 | 1.00 | 42.46 | B | C |
| ATOM | 2653 | OG1 | THR | B | 39 | -17.592 | 31.847 | 45.566 | 1.00 | 42.46 | B | O |
| ATOM | 2654 | CG2 | THR | B | 39 | -16.715 | 32.897 | 47.565 | 1.00 | 42.46 | B | C |
| ATOM | 2655 | C | THR | B | 39 | -16.410 | 30.354 | 48.808 | 1.00 | 56.75 | B | C |
| ATOM | 2656 | O | THR | B | 39 | -17.269 | 30.351 | 49.689 | 1.00 | 56.75 | B | O |
| ATOM | 2657 | N | VAL | B | 40 | -15.117 | 30.353 | 49.076 | 1.00 | 48.63 | B | N |
| ATOM | 2658 | CA | VAL | B | 40 | -14.644 | 30.330 | 50.445 | 1.00 | 48.63 | B | C |
| ATOM | 2659 | CB | VAL | B | 40 | -14.005 | 28.964 | 50.771 | 1.00 | 36.13 | B | C |
| ATOM | 2660 | CG1 | VAL | B | 40 | -15.042 | 27.850 | 50.622 | 1.00 | 36.13 | B | C |
| ATOM | 2661 | CG2 | VAL | B | 40 | -12.807 | 28.722 | 49.848 | 1.00 | 36.13 | B | C |
| ATOM | 2662 | C | VAL | B | 40 | -13.598 | 31.410 | 50.663 | 1.00 | 48.63 | B | C |
| ATOM | 2663 | O | VAL | B | 40 | -13.001 | 31.924 | 49.711 | 1.00 | 48.63 | B | O |
| ATOM | 2664 | N | VAL | B | 41 | -13.384 | 31.774 | 51.918 | 1.00 | 41.12 | B | N |
| ATOM | 2665 | CA | VAL | B | 41 | -12.366 | 32.764 | 52.213 | 1.00 | 41.12 | B | C |
| ATOM | 2666 | CB | VAL | B | 41 | -12.826 | 33.772 | 53.286 | 1.00 | 40.90 | B | C |
| ATOM | 2667 | CG1 | VAL | B | 41 | -11.667 | 34.680 | 53.675 | 1.00 | 40.90 | B | C |
| ATOM | 2668 | CG2 | VAL | B | 41 | -13.971 | 34.610 | 52.742 | 1.00 | 40.90 | B | C |
| ATOM | 2669 | C | VAL | B | 41 | -11.196 | 31.941 | 52.719 | 1.00 | 41.12 | B | C |
| ATOM | 2670 | O | VAL | B | 41 | -11.227 | 31.410 | 53.830 | 1.00 | 41.12 | B | O |
| ATOM | 2671 | N | ALA | B | 42 | -10.174 | 31.823 | 51.878 | 1.00 | 39.58 | B | N |
| ATOM | 2672 | CA | ALA | B | 42 | -9.006 | 31.035 | 52.216 | 1.00 | 39.58 | B | C |
| ATOM | 2673 | CB | ALA | B | 42 | -8.836 | 29.925 | 51.199 | 1.00 | 68.93 | B | C |

| | | | | | | | | | | | | |
|---|---|---|---|---|---|---|---|---|---|---|---|---|
| ATOM | 2674 | C | ALA | B | 42 | -7.707 | 31.807 | 52.343 | 1.00 | 39.58 | B | C |
| ATOM | 2675 | O | ALA | B | 42 | -7.366 | 32.640 | 51.499 | 1.00 | 39.58 | B | O |
| ATOM | 2676 | N | THR | B | 43 | -6.989 | 31.505 | 53.420 | 1.00 | 43.86 | B | N |
| ATOM | 2677 | CA | THR | B | 43 | -5.691 | 32.097 | 53.693 | 1.00 | 43.86 | B | C |
| ATOM | 2678 | CB | THR | B | 43 | -5.243 | 31.808 | 55.143 | 1.00 | 44.15 | B | C |
| ATOM | 2679 | OG1 | THR | B | 43 | -6.363 | 31.920 | 56.029 | 1.00 | 44.15 | B | O |
| ATOM | 2680 | CG2 | THR | B | 43 | -4.172 | 32.790 | 55.569 | 1.00 | 44.15 | B | C |
| ATOM | 2681 | C | THR | B | 43 | -4.746 | 31.357 | 52.739 | 1.00 | 43.86 | B | C |
| ATOM | 2682 | O | THR | B | 43 | -4.888 | 30.153 | 52.536 | 1.00 | 43.86 | B | O |
| ATOM | 2683 | N | PRO | B | 44 | -3.786 | 32.066 | 52.129 | 1.00 | 46.61 | B | N |
| ATOM | 2684 | CD | PRO | B | 44 | -3.617 | 33.525 | 52.063 | 1.00 | 57.80 | B | C |
| ATOM | 2685 | CA | PRO | B | 44 | -2.861 | 31.396 | 51.210 | 1.00 | 46.61 | B | C |
| ATOM | 2686 | CB | PRO | B | 44 | -2.124 | 32.563 | 50.556 | 1.00 | 57.80 | B | C |
| ATOM | 2687 | CG | PRO | B | 44 | -3.116 | 33.700 | 50.657 | 1.00 | 57.80 | B | C |
| ATOM | 2688 | C | PRO | B | 44 | -1.923 | 30.465 | 51.987 | 1.00 | 46.61 | B | C |
| ATOM | 2689 | O | PRO | B | 44 | -1.640 | 30.699 | 53.168 | 1.00 | 46.61 | B | O |
| ATOM | 2690 | N | GLY | B | 45 | -1.450 | 29.413 | 51.321 | 1.00 | 68.92 | B | N |
| ATOM | 2691 | CA | GLY | B | 45 | -0.573 | 28.441 | 51.959 | 1.00 | 68.92 | B | C |
| ATOM | 2692 | C | GLY | B | 45 | 0.688 | 29.055 | 52.524 | 1.00 | 68.92 | B | C |
| ATOM | 2693 | O | GLY | B | 45 | 0.799 | 29.293 | 53.732 | 1.00 | 68.92 | B | O |
| ATOM | 2694 | N | ASP | B | 46 | 1.669 | 29.276 | 51.661 | 1.00 | 76.76 | B | N |
| ATOM | 2695 | CA | ASP | B | 46 | 2.901 | 29.912 | 52.103 | 1.00 | 76.76 | B | C |
| ATOM | 2696 | CB | ASP | B | 46 | 4.124 | 29.328 | 51.364 | 1.00 | 99.39 | B | C |
| ATOM | 2697 | CG | ASP | B | 46 | 5.389 | 30.209 | 51.509 | 1.00 | 99.39 | B | C |
| ATOM | 2698 | OD1 | ASP | B | 46 | 6.287 | 30.137 | 50.627 | 1.00 | 99.39 | B | O |
| ATOM | 2699 | OD2 | ASP | B | 46 | 5.492 | 30.975 | 52.502 | 1.00 | 99.39 | B | O |
| ATOM | 2700 | C | ASP | B | 46 | 2.703 | 31.379 | 51.728 | 1.00 | 76.76 | B | C |
| ATOM | 2701 | O | ASP | B | 46 | 2.523 | 31.707 | 50.549 | 1.00 | 76.76 | B | O |
| ATOM | 2702 | N | GLY | B | 47 | 2.702 | 32.259 | 52.717 | 1.00 | 62.38 | B | N |
| ATOM | 2703 | CA | GLY | B | 47 | 2.546 | 33.662 | 52.397 | 1.00 | 62.38 | B | C |
| ATOM | 2704 | C | GLY | B | 47 | 2.111 | 34.499 | 53.575 | 1.00 | 62.38 | B | C |
| ATOM | 2705 | O | GLY | B | 47 | 2.263 | 34.080 | 54.722 | 1.00 | 62.38 | B | O |
| ATOM | 2706 | N | PRO | B | 48 | 1.545 | 35.689 | 53.328 | 1.00 | 56.60 | B | N |
| ATOM | 2707 | CD | PRO | B | 48 | 1.010 | 36.187 | 52.053 | 1.00 | 67.11 | B | C |
| ATOM | 2708 | CA | PRO | B | 48 | 1.105 | 36.548 | 54.425 | 1.00 | 56.60 | B | C |
| ATOM | 2709 | CB | PRO | B | 48 | 0.966 | 37.920 | 53.760 | 1.00 | 67.11 | B | C |
| ATOM | 2710 | CG | PRO | B | 48 | 1.180 | 37.667 | 52.227 | 1.00 | 67.11 | B | C |
| ATOM | 2711 | C | PRO | B | 48 | -0.230 | 36.000 | 54.908 | 1.00 | 56.60 | B | C |
| ATOM | 2712 | O | PRO | B | 48 | -1.159 | 35.838 | 54.114 | 1.00 | 56.60 | B | O |
| ATOM | 2713 | N | ASP | B | 49 | -0.309 | 35.690 | 56.198 | 1.00 | 57.88 | B | N |
| ATOM | 2714 | CA | ASP | B | 49 | -1.526 | 35.136 | 56.794 | 1.00 | 57.88 | B | C |
| ATOM | 2715 | CB | ASP | B | 49 | -1.294 | 34.850 | 58.281 | 1.00 | 72.56 | B | C |
| ATOM | 2716 | CG | ASP | B | 49 | -2.425 | 34.057 | 58.899 | 1.00 | 72.56 | B | C |
| ATOM | 2717 | OD1 | ASP | B | 49 | -3.575 | 34.275 | 58.459 | 1.00 | 72.56 | B | O |
| ATOM | 2718 | OD2 | ASP | B | 49 | -2.179 | 33.227 | 59.819 | 1.00 | 72.56 | B | O |
| ATOM | 2719 | C | ASP | B | 49 | -2.689 | 36.117 | 56.640 | 1.00 | 57.88 | B | C |
| ATOM | 2720 | O | ASP | B | 49 | -3.103 | 36.748 | 57.611 | 1.00 | 57.88 | B | O |
| ATOM | 2721 | N | ARG | B | 50 | -3.230 | 36.225 | 55.429 | 1.00 | 53.04 | B | N |
| ATOM | 2722 | CA | ARG | B | 50 | -4.312 | 37.169 | 55.167 | 1.00 | 53.04 | B | C |
| ATOM | 2723 | CB | ARG | B | 50 | -3.694 | 38.534 | 54.880 | 1.00 | 99.46 | B | C |
| ATOM | 2724 | CG | ARG | B | 50 | -4.598 | 39.689 | 55.244 | 1.00 | 99.46 | B | C |
| ATOM | 2725 | CD | ARG | B | 50 | -3.869 | 41.023 | 55.155 | 1.00 | 99.46 | B | C |
| ATOM | 2726 | NE | ARG | B | 50 | -3.122 | 41.218 | 53.905 | 1.00 | 99.46 | B | N |
| ATOM | 2727 | CZ | ARG | B | 50 | -3.467 | 40.775 | 52.692 | 1.00 | 99.46 | B | C |
| ATOM | 2728 | NH1 | ARG | B | 50 | -4.578 | 40.069 | 52.515 | 1.00 | 99.46 | B | N |
| ATOM | 2729 | NH2 | ARG | B | 50 | -2.702 | 41.059 | 51.639 | 1.00 | 99.46 | B | N |
| ATOM | 2730 | C | ARG | B | 50 | -5.211 | 36.719 | 54.001 | 1.00 | 53.04 | B | C |
| ATOM | 2731 | O | ARG | B | 50 | -4.913 | 36.972 | 52.833 | 1.00 | 53.04 | B | O |
| ATOM | 2732 | N | PRO | B | 51 | -6.348 | 36.084 | 54.327 | 1.00 | 48.03 | B | N |
| ATOM | 2733 | CD | PRO | B | 51 | -6.827 | 36.143 | 55.718 | 1.00 | 54.83 | B | C |
| ATOM | 2734 | CA | PRO | B | 51 | -7.395 | 35.519 | 53.470 | 1.00 | 48.03 | B | C |
| ATOM | 2735 | CB | PRO | B | 51 | -8.420 | 35.007 | 54.479 | 1.00 | 54.83 | B | C |
| ATOM | 2736 | CG | PRO | B | 51 | -8.325 | 35.996 | 55.553 | 1.00 | 54.83 | B | C |
| ATOM | 2737 | C | PRO | B | 51 | -8.048 | 36.337 | 52.369 | 1.00 | 48.03 | B | C |
| ATOM | 2738 | O | PRO | B | 51 | -8.231 | 37.542 | 52.493 | 1.00 | 48.03 | B | O |
| ATOM | 2739 | N | GLN | B | 52 | -8.405 | 35.631 | 51.294 | 1.00 | 50.19 | B | N |
| ATOM | 2740 | CA | GLN | B | 52 | -9.058 | 36.203 | 50.123 | 1.00 | 50.19 | B | C |

| | | | | | | | | | | | | |
|---|---|---|---|---|---|---|---|---|---|---|---|---|
| ATOM | 2741 | CB | GLN | B | 52 | -8.040 | 36.528 | 49.040 | 1.00 | 71.07 | B | C |
| ATOM | 2742 | CG | GLN | B | 52 | -6.986 | 35.472 | 48.875 | 1.00 | 71.07 | B | C |
| ATOM | 2743 | CD | GLN | B | 52 | -5.824 | 35.949 | 48.020 | 1.00 | 71.07 | B | C |
| ATOM | 2744 | OE1 | GLN | B | 52 | -5.792 | 35.721 | 46.802 | 1.00 | 71.07 | B | O |
| ATOM | 2745 | NE2 | GLN | B | 52 | -4.863 | 36.632 | 48.653 | 1.00 | 71.07 | B | N |
| ATOM | 2746 | C | GLN | B | 52 | -10.058 | 35.206 | 49.592 | 1.00 | 50.19 | B | C |
| ATOM | 2747 | O | GLN | B | 52 | -10.015 | 34.027 | 49.931 | 1.00 | 50.19 | B | O |
| ATOM | 2748 | N | GLU | B | 53 | -10.971 | 35.685 | 48.760 | 1.00 | 51.68 | B | N |
| ATOM | 2749 | CA | GLU | B | 53 | -11.996 | 34.814 | 48.204 | 1.00 | 51.68 | B | C |
| ATOM | 2750 | CB | GLU | B | 53 | -13.148 | 35.641 | 47.636 | 1.00 | 57.81 | B | C |
| ATOM | 2751 | CG | GLU | B | 53 | -14.046 | 36.238 | 48.704 | 1.00 | 57.81 | B | C |
| ATOM | 2752 | CD | GLU | B | 53 | -15.004 | 37.260 | 48.138 | 1.00 | 57.81 | B | C |
| ATOM | 2753 | OE1 | GLU | B | 53 | -15.616 | 36.977 | 47.096 | 1.00 | 57.81 | B | O |
| ATOM | 2754 | OE2 | GLU | B | 53 | -15.152 | 38.339 | 48.734 | 1.00 | 57.81 | B | O |
| ATOM | 2755 | C | GLU | B | 53 | -11.454 | 33.874 | 47.144 | 1.00 | 51.68 | B | C |
| ATOM | 2756 | O | GLU | B | 53 | -10.623 | 34.243 | 46.312 | 1.00 | 51.68 | B | O |
| ATOM | 2757 | N | VAL | B | 54 | -11.939 | 32.641 | 47.202 | 1.00 | 45.60 | B | N |
| ATOM | 2758 | CA | VAL | B | 54 | -11.544 | 31.599 | 46.273 | 1.00 | 45.60 | B | C |
| ATOM | 2759 | CB | VAL | B | 54 | -10.554 | 30.623 | 46.928 | 1.00 | 40.95 | B | C |
| ATOM | 2760 | CG1 | VAL | B | 54 | -10.205 | 29.515 | 45.949 | 1.00 | 40.95 | B | C |
| ATOM | 2761 | CG2 | VAL | B | 54 | -9.310 | 31.368 | 47.387 | 1.00 | 40.95 | B | C |
| ATOM | 2762 | C | VAL | B | 54 | -12.777 | 30.811 | 45.856 | 1.00 | 45.60 | B | C |
| ATOM | 2763 | O | VAL | B | 54 | -13.553 | 30.360 | 46.703 | 1.00 | 45.60 | B | O |
| ATOM | 2764 | N | SER | B | 55 | -12.963 | 30.662 | 44.549 | 1.00 | 52.05 | B | N |
| ATOM | 2765 | CA | SER | B | 55 | -14.097 | 29.898 | 44.036 | 1.00 | 52.05 | B | C |
| ATOM | 2766 | CB | SER | B | 55 | -14.991 | 30.780 | 43.154 | 1.00 | 63.44 | B | C |
| ATOM | 2767 | OG | SER | B | 55 | -15.703 | 31.726 | 43.942 | 1.00 | 63.44 | B | O |
| ATOM | 2768 | C | SER | B | 55 | -13.627 | 28.669 | 43.255 | 1.00 | 52.05 | B | C |
| ATOM | 2769 | O | SER | B | 55 | -12.667 | 28.723 | 42.481 | 1.00 | 52.05 | B | O |
| ATOM | 2770 | N | TYR | B | 56 | -14.299 | 27.551 | 43.484 | 1.00 | 50.76 | B | N |
| ATOM | 2771 | CA | TYR | B | 56 | -13.947 | 26.317 | 42.809 | 1.00 | 50.76 | B | C |
| ATOM | 2772 | CB | TYR | B | 56 | -13.006 | 25.465 | 43.680 | 1.00 | 37.39 | B | C |
| ATOM | 2773 | CG | TYR | B | 56 | -13.557 | 25.060 | 45.033 | 1.00 | 37.39 | B | C |
| ATOM | 2774 | CD1 | TYR | B | 56 | -14.585 | 24.124 | 45.144 | 1.00 | 37.39 | B | C |
| ATOM | 2775 | CE1 | TYR | B | 56 | -15.087 | 23.750 | 46.390 | 1.00 | 37.39 | B | C |
| ATOM | 2776 | CD2 | TYR | B | 56 | -13.045 | 25.614 | 46.206 | 1.00 | 37.39 | B | C |
| ATOM | 2777 | CE2 | TYR | B | 56 | -13.535 | 25.251 | 47.452 | 1.00 | 37.39 | B | C |
| ATOM | 2778 | CZ | TYR | B | 56 | -14.554 | 24.318 | 47.538 | 1.00 | 37.39 | B | C |
| ATOM | 2779 | OH | TYR | B | 56 | -15.029 | 23.940 | 48.775 | 1.00 | 37.39 | B | O |
| ATOM | 2780 | C | TYR | B | 56 | -15.215 | 25.553 | 42.497 | 1.00 | 50.76 | B | C |
| ATOM | 2781 | O | TYR | B | 56 | -16.263 | 25.788 | 43.106 | 1.00 | 50.76 | B | O |
| ATOM | 2782 | N | THR | B | 57 | -15.123 | 24.634 | 41.545 | 1.00 | 59.31 | B | N |
| ATOM | 2783 | CA | THR | B | 57 | -16.287 | 23.858 | 41.176 | 1.00 | 59.31 | B | C |
| ATOM | 2784 | CB | THR | B | 57 | -17.055 | 24.570 | 40.055 | 1.00 | 51.89 | B | C |
| ATOM | 2785 | OG1 | THR | B | 57 | -18.298 | 23.890 | 39.812 | 1.00 | 51.89 | B | O |
| ATOM | 2786 | CG2 | THR | B | 57 | -16.204 | 24.608 | 38.789 | 1.00 | 51.89 | B | C |
| ATOM | 2787 | C | THR | B | 57 | -15.891 | 22.457 | 40.718 | 1.00 | 59.31 | B | C |
| ATOM | 2788 | O | THR | B | 57 | -14.730 | 22.066 | 40.827 | 1.00 | 59.31 | B | O |
| ATOM | 2789 | N | ASP | B | 58 | -16.867 | 21.713 | 40.205 | 1.00 | 57.73 | B | N |
| ATOM | 2790 | CA | ASP | B | 58 | -16.639 | 20.358 | 39.723 | 1.00 | 57.73 | B | C |
| ATOM | 2791 | CB | ASP | B | 58 | -15.660 | 20.341 | 38.529 | 1.00 | 49.12 | B | C |
| ATOM | 2792 | CG | ASP | B | 58 | -16.122 | 21.200 | 37.363 | 1.00 | 49.12 | B | C |
| ATOM | 2793 | OD1 | ASP | B | 58 | -17.310 | 21.588 | 37.338 | 1.00 | 49.12 | B | O |
| ATOM | 2794 | OD2 | ASP | B | 58 | -15.299 | 21.480 | 36.464 | 1.00 | 49.12 | B | O |
| ATOM | 2795 | C | ASP | B | 58 | -16.034 | 19.548 | 40.849 | 1.00 | 57.73 | B | C |
| ATOM | 2796 | O | ASP | B | 58 | -15.186 | 18.690 | 40.613 | 1.00 | 57.73 | B | O |
| ATOM | 2797 | N | THR | B | 59 | -16.455 | 19.805 | 42.079 | 1.00 | 58.58 | B | N |
| ATOM | 2798 | CA | THR | B | 59 | -15.867 | 19.056 | 43.188 | 1.00 | 58.58 | B | C |
| ATOM | 2799 | CB | THR | B | 59 | -16.102 | 19.761 | 44.535 | 1.00 | 55.03 | B | C |
| ATOM | 2800 | OG1 | THR | B | 59 | -17.389 | 19.407 | 45.042 | 1.00 | 55.03 | B | O |
| ATOM | 2801 | CG2 | THR | B | 59 | -16.037 | 21.267 | 44.362 | 1.00 | 55.03 | B | C |
| ATOM | 2802 | C | THR | B | 59 | -16.374 | 17.614 | 43.297 | 1.00 | 58.58 | B | C |
| ATOM | 2803 | O | THR | B | 59 | -17.587 | 17.366 | 43.286 | 1.00 | 58.58 | B | O |
| ATOM | 2804 | N | LYS | B | 60 | -15.441 | 16.666 | 43.391 | 1.00 | 58.77 | B | N |
| ATOM | 2805 | CA | LYS | B | 60 | -15.798 | 15.252 | 43.531 | 1.00 | 58.77 | B | C |
| ATOM | 2806 | CB | LYS | B | 60 | -15.793 | 14.547 | 42.168 | 1.00 | 52.32 | B | C |
| ATOM | 2807 | CG | LYS | B | 60 | -14.457 | 14.520 | 41.471 | 1.00 | 52.32 | B | C |

| | | | | | | | | | | | | |
|---|---|---|---|---|---|---|---|---|---|---|---|---|
| ATOM | 2808 | CD | LYS | B | 60 | -14.498 | 13.537 | 40.310 | 1.00 | 52.32 | B | C |
| ATOM | 2809 | CE | LYS | B | 60 | -13.235 | 13.627 | 39.457 | 1.00 | 52.32 | B | C |
| ATOM | 2810 | NZ | LYS | B | 60 | -13.286 | 12.773 | 38.232 | 1.00 | 52.32 | B | N |
| ATOM | 2811 | C | LYS | B | 60 | -14.852 | 14.525 | 44.499 | 1.00 | 58.77 | B | C |
| ATOM | 2812 | O | LYS | B | 60 | -13.653 | 14.825 | 44.558 | 1.00 | 58.77 | B | O |
| ATOM | 2813 | N | VAL | B | 61 | -15.403 | 13.572 | 45.253 | 1.00 | 48.28 | B | N |
| ATOM | 2814 | CA | VAL | B | 61 | -14.635 | 12.811 | 46.230 | 1.00 | 48.28 | B | C |
| ATOM | 2815 | CB | VAL | B | 61 | -15.555 | 11.952 | 47.112 | 1.00 | 39.27 | B | C |
| ATOM | 2816 | CG1 | VAL | B | 61 | -14.720 | 11.113 | 48.072 | 1.00 | 39.27 | B | C |
| ATOM | 2817 | CG2 | VAL | B | 61 | -16.512 | 12.849 | 47.884 | 1.00 | 39.27 | B | C |
| ATOM | 2818 | C | VAL | B | 61 | -13.606 | 11.897 | 45.583 | 1.00 | 48.28 | B | C |
| ATOM | 2819 | O | VAL | B | 61 | -13.910 | 11.214 | 44.605 | 1.00 | 48.28 | B | O |
| ATOM | 2820 | N | ILE | B | 62 | -12.391 | 11.879 | 46.136 | 1.00 | 43.29 | B | N |
| ATOM | 2821 | CA | ILE | B | 62 | -11.323 | 11.033 | 45.602 | 1.00 | 43.29 | B | C |
| ATOM | 2822 | CB | ILE | B | 62 | -10.398 | 11.840 | 44.664 | 1.00 | 35.10 | B | C |
| ATOM | 2823 | CG2 | ILE | B | 62 | -11.233 | 12.596 | 43.648 | 1.00 | 35.10 | B | C |
| ATOM | 2824 | CG1 | ILE | B | 62 | -9.549 | 12.830 | 45.460 | 1.00 | 35.10 | B | C |
| ATOM | 2825 | CD1 | ILE | B | 62 | -8.440 | 13.453 | 44.635 | 1.00 | 35.10 | B | C |
| ATOM | 2826 | C | ILE | B | 62 | -10.454 | 10.337 | 46.662 | 1.00 | 43.29 | B | C |
| ATOM | 2827 | O | ILE | B | 62 | -9.421 | 9.742 | 46.337 | 1.00 | 43.29 | B | O |
| ATOM | 2828 | N | GLY | B | 63 | -10.868 | 10.400 | 47.924 | 1.00 | 43.15 | B | N |
| ATOM | 2829 | CA | GLY | B | 63 | -10.079 | 9.767 | 48.961 | 1.00 | 43.15 | B | C |
| ATOM | 2830 | C | GLY | B | 63 | -10.671 | 9.819 | 50.353 | 1.00 | 43.15 | B | C |
| ATOM | 2831 | O | GLY | B | 63 | -11.252 | 10.821 | 50.743 | 1.00 | 43.15 | B | O |
| ATOM | 2832 | N | ASN | B | 64 | -10.519 | 8.720 | 51.091 | 1.00 | 60.26 | B | N |
| ATOM | 2833 | CA | ASN | B | 64 | -11.004 | 8.577 | 52.470 | 1.00 | 60.26 | B | C |
| ATOM | 2834 | CB | ASN | B | 64 | -11.571 | 7.171 | 52.699 | 1.00 | 61.40 | B | C |
| ATOM | 2835 | CG | ASN | B | 64 | -12.943 | 6.998 | 52.121 | 1.00 | 61.40 | B | C |
| ATOM | 2836 | OD1 | ASN | B | 64 | -13.957 | 7.315 | 52.758 | 1.00 | 61.40 | B | O |
| ATOM | 2837 | ND2 | ASN | B | 64 | -12.994 | 6.507 | 50.891 | 1.00 | 61.40 | B | N |
| ATOM | 2838 | C | ASN | B | 64 | -9.828 | 8.767 | 53.414 | 1.00 | 60.26 | B | C |
| ATOM | 2839 | O | ASN | B | 64 | -8.881 | 9.476 | 53.103 | 1.00 | 60.26 | B | O |
| ATOM | 2840 | N | GLY | B | 65 | -9.878 | 8.103 | 54.560 | 1.00 | 39.44 | B | N |
| ATOM | 2841 | CA | GLY | B | 65 | -8.796 | 8.209 | 55.515 | 1.00 | 39.44 | B | C |
| ATOM | 2842 | C | GLY | B | 65 | -9.369 | 8.472 | 56.881 | 1.00 | 39.44 | B | C |
| ATOM | 2843 | O | GLY | B | 65 | -10.526 | 8.878 | 57.001 | 1.00 | 39.44 | B | O |
| ATOM | 2844 | N | SER | B | 66 | -8.567 | 8.231 | 57.912 | 1.00 | 50.77 | B | N |
| ATOM | 2845 | CA | SER | B | 66 | -9.004 | 8.450 | 59.294 | 1.00 | 50.77 | B | C |
| ATOM | 2846 | CB | SER | B | 66 | -8.031 | 7.779 | 60.269 | 1.00 | 60.25 | B | C |
| ATOM | 2847 | OG | SER | B | 66 | -6.705 | 8.246 | 60.055 | 1.00 | 60.25 | B | O |
| ATOM | 2848 | C | SER | B | 66 | -9.067 | 9.946 | 59.580 | 1.00 | 50.77 | B | C |
| ATOM | 2849 | O | SER | B | 66 | -9.640 | 10.374 | 60.577 | 1.00 | 50.77 | B | O |
| ATOM | 2850 | N | PHE | B | 67 | -8.471 | 10.725 | 58.683 | 1.00 | 51.21 | B | N |
| ATOM | 2851 | CA | PHE | B | 67 | -8.431 | 12.171 | 58.801 | 1.00 | 51.21 | B | C |
| ATOM | 2852 | CB | PHE | B | 67 | -7.256 | 12.696 | 57.998 | 1.00 | 59.32 | B | C |
| ATOM | 2853 | CG | PHE | B | 67 | -7.315 | 12.341 | 56.544 | 1.00 | 59.32 | B | C |
| ATOM | 2854 | CD1 | PHE | B | 67 | -8.172 | 13.024 | 55.677 | 1.00 | 59.32 | B | C |
| ATOM | 2855 | CD2 | PHE | B | 67 | -6.493 | 11.346 | 56.030 | 1.00 | 59.32 | B | C |
| ATOM | 2856 | CE1 | PHE | B | 67 | -8.202 | 12.726 | 54.315 | 1.00 | 59.32 | B | C |
| ATOM | 2857 | CE2 | PHE | B | 67 | -6.512 | 11.036 | 54.667 | 1.00 | 59.32 | B | C |
| ATOM | 2858 | CZ | PHE | B | 67 | -7.367 | 11.729 | 53.807 | 1.00 | 59.32 | B | C |
| ATOM | 2859 | C | PHE | B | 67 | -9.714 | 12.816 | 58.294 | 1.00 | 51.21 | B | C |
| ATOM | 2860 | O | PHE | B | 67 | -10.141 | 13.854 | 58.803 | 1.00 | 51.21 | B | O |
| ATOM | 2861 | N | GLY | B | 68 | -10.313 | 12.203 | 57.276 | 1.00 | 45.10 | B | N |
| ATOM | 2862 | CA | GLY | B | 68 | -11.535 | 12.733 | 56.714 | 1.00 | 45.10 | B | C |
| ATOM | 2863 | C | GLY | B | 68 | -11.655 | 12.317 | 55.269 | 1.00 | 45.10 | B | C |
| ATOM | 2864 | O | GLY | B | 68 | -11.368 | 11.167 | 54.930 | 1.00 | 45.10 | B | O |
| ATOM | 2865 | N | VAL | B | 69 | -12.070 | 13.257 | 54.420 | 1.00 | 52.52 | B | N |
| ATOM | 2866 | CA | VAL | B | 69 | -12.248 | 13.019 | 52.990 | 1.00 | 52.52 | B | C |
| ATOM | 2867 | CB | VAL | B | 69 | -13.701 | 13.295 | 52.570 | 1.00 | 33.15 | B | C |
| ATOM | 2868 | CG1 | VAL | B | 69 | -13.907 | 12.929 | 51.110 | 1.00 | 33.15 | B | C |
| ATOM | 2869 | CG2 | VAL | B | 69 | -14.644 | 12.514 | 53.452 | 1.00 | 33.15 | B | C |
| ATOM | 2870 | C | VAL | B | 69 | -11.341 | 13.928 | 52.165 | 1.00 | 52.52 | B | C |
| ATOM | 2871 | O | VAL | B | 69 | -10.770 | 14.881 | 52.681 | 1.00 | 52.52 | B | O |
| ATOM | 2872 | N | VAL | B | 70 | -11.213 | 13.627 | 50.881 | 1.00 | 34.24 | B | N |
| ATOM | 2873 | CA | VAL | B | 70 | -10.400 | 14.426 | 49.984 | 1.00 | 34.24 | B | C |
| ATOM | 2874 | CB | VAL | B | 70 | -9.069 | 13.753 | 49.663 | 1.00 | 38.47 | B | C |

| | | | | | | | | | | | | |
|---|---|---|---|---|---|---|---|---|---|---|---|---|
| ATOM | 2875 | CG1 | VAL | B | 70 | -8.160 | 14.732 | 48.961 | 1.00 | 38.47 | B | C |
| ATOM | 2876 | CG2 | VAL | B | 70 | -8.434 | 13.239 | 50.919 | 1.00 | 38.47 | B | C |
| ATOM | 2877 | C | VAL | B | 70 | -11.154 | 14.562 | 48.671 | 1.00 | 34.24 | B | C |
| ATOM | 2878 | O | VAL | B | 70 | -11.398 | 13.571 | 47.979 | 1.00 | 34.24 | B | O |
| ATOM | 2879 | N | TYR | B | 71 | -11.526 | 15.786 | 48.323 | 1.00 | 47.78 | B | N |
| ATOM | 2880 | CA | TYR | B | 71 | -12.233 | 16.008 | 47.082 | 1.00 | 47.78 | B | C |
| ATOM | 2881 | CB | TYR | B | 71 | -13.341 | 17.048 | 47.260 | 1.00 | 45.77 | B | C |
| ATOM | 2882 | CG | TYR | B | 71 | -14.233 | 16.851 | 48.461 | 1.00 | 45.77 | B | C |
| ATOM | 2883 | CD1 | TYR | B | 71 | -13.775 | 17.125 | 49.747 | 1.00 | 45.77 | B | C |
| ATOM | 2884 | CE1 | TYR | B | 71 | -14.614 | 16.983 | 50.854 | 1.00 | 45.77 | B | C |
| ATOM | 2885 | CD2 | TYR | B | 71 | -15.555 | 16.423 | 48.310 | 1.00 | 45.77 | B | C |
| ATOM | 2886 | CE2 | TYR | B | 71 | -16.402 | 16.276 | 49.416 | 1.00 | 45.77 | B | C |
| ATOM | 2887 | CZ | TYR | B | 71 | -15.922 | 16.561 | 50.681 | 1.00 | 45.77 | B | C |
| ATOM | 2888 | OH | TYR | B | 71 | -16.748 | 16.432 | 51.773 | 1.00 | 45.77 | B | O |
| ATOM | 2889 | C | TYR | B | 71 | -11.245 | 16.543 | 46.063 | 1.00 | 47.78 | B | C |
| ATOM | 2890 | O | TYR | B | 71 | -10.089 | 16.848 | 46.392 | 1.00 | 47.78 | B | O |
| ATOM | 2891 | N | GLN | B | 72 | -11.699 | 16.631 | 44.816 | 1.00 | 32.24 | B | N |
| ATOM | 2892 | CA | GLN | B | 72 | -10.895 | 17.215 | 43.764 | 1.00 | 32.24 | B | C |
| ATOM | 2893 | CB | GLN | B | 72 | -10.740 | 16.285 | 42.587 | 1.00 | 46.73 | B | C |
| ATOM | 2894 | CG | GLN | B | 72 | -9.994 | 16.956 | 41.465 | 1.00 | 46.73 | B | C |
| ATOM | 2895 | CD | GLN | B | 72 | -10.174 | 16.265 | 40.143 | 1.00 | 46.73 | B | C |
| ATOM | 2896 | OE1 | GLN | B | 72 | -11.277 | 16.233 | 39.585 | 1.00 | 46.73 | B | O |
| ATOM | 2897 | NE2 | GLN | B | 72 | -9.090 | 15.702 | 39.626 | 1.00 | 46.73 | B | N |
| ATOM | 2898 | C | GLN | B | 72 | -11.757 | 18.390 | 43.349 | 1.00 | 32.24 | B | C |
| ATOM | 2899 | O | GLN | B | 72 | -12.962 | 18.370 | 43.587 | 1.00 | 32.24 | B | O |
| ATOM | 2900 | N | ALA | B | 73 | -11.163 | 19.408 | 42.739 | 1.00 | 39.42 | B | N |
| ATOM | 2901 | CA | ALA | B | 73 | -11.938 | 20.569 | 42.326 | 1.00 | 39.42 | B | C |
| ATOM | 2902 | CB | ALA | B | 73 | -12.330 | 21.404 | 43.543 | 1.00 | 44.25 | B | C |
| ATOM | 2903 | C | ALA | B | 73 | -11.198 | 21.431 | 41.326 | 1.00 | 39.42 | B | C |
| ATOM | 2904 | O | ALA | B | 73 | -10.001 | 21.259 | 41.091 | 1.00 | 39.42 | B | O |
| ATOM | 2905 | N | LYS | B | 74 | -11.926 | 22.378 | 40.753 | 1.00 | 50.48 | B | N |
| ATOM | 2906 | CA | LYS | B | 74 | -11.370 | 23.257 | 39.747 | 1.00 | 50.48 | B | C |
| ATOM | 2907 | CB | LYS | B | 74 | -12.060 | 22.970 | 38.417 | 1.00 | 65.03 | B | C |
| ATOM | 2908 | CG | LYS | B | 74 | -11.551 | 23.748 | 37.230 | 1.00 | 65.03 | B | C |
| ATOM | 2909 | CD | LYS | B | 74 | -12.427 | 23.421 | 36.026 | 1.00 | 65.03 | B | C |
| ATOM | 2910 | CE | LYS | B | 74 | -12.215 | 24.370 | 34.861 | 1.00 | 65.03 | B | C |
| ATOM | 2911 | NZ | LYS | B | 74 | -13.126 | 23.985 | 33.739 | 1.00 | 65.03 | B | N |
| ATOM | 2912 | C | LYS | B | 74 | -11.545 | 24.716 | 40.149 | 1.00 | 50.48 | B | C |
| ATOM | 2913 | O | LYS | B | 74 | -12.656 | 25.171 | 40.424 | 1.00 | 50.48 | B | O |
| ATOM | 2914 | N | LEU | B | 75 | -10.429 | 25.437 | 40.193 | 1.00 | 55.36 | B | N |
| ATOM | 2915 | CA | LEU | B | 75 | -10.439 | 26.838 | 40.564 | 1.00 | 55.36 | B | C |
| ATOM | 2916 | CB | LEU | B | 75 | -9.005 | 27.330 | 40.816 | 1.00 | 30.41 | B | C |
| ATOM | 2917 | CG | LEU | B | 75 | -8.177 | 26.676 | 41.928 | 1.00 | 30.41 | B | C |
| ATOM | 2918 | CD1 | LEU | B | 75 | -6.946 | 27.525 | 42.202 | 1.00 | 30.41 | B | C |
| ATOM | 2919 | CD2 | LEU | B | 75 | -8.992 | 26.558 | 43.192 | 1.00 | 30.41 | B | C |
| ATOM | 2920 | C | LEU | B | 75 | -11.080 | 27.635 | 39.434 | 1.00 | 55.36 | B | C |
| ATOM | 2921 | O | LEU | B | 75 | -10.703 | 27.493 | 38.270 | 1.00 | 55.36 | B | O |
| ATOM | 2922 | N | CYS | B | 76 | -12.050 | 28.474 | 39.777 | 1.00 | 57.25 | B | N |
| ATOM | 2923 | CA | CYS | B | 76 | -12.731 | 29.278 | 38.772 | 1.00 | 57.25 | B | C |
| ATOM | 2924 | CB | CYS | B | 76 | -14.002 | 29.903 | 39.359 | 1.00 | 55.73 | B | C |
| ATOM | 2925 | SG | CYS | B | 76 | -15.472 | 28.888 | 39.121 | 1.00 | 55.73 | B | S |
| ATOM | 2926 | C | CYS | B | 76 | -11.873 | 30.366 | 38.147 | 1.00 | 57.25 | B | C |
| ATOM | 2927 | O | CYS | B | 76 | -12.349 | 31.103 | 37.294 | 1.00 | 57.25 | B | O |
| ATOM | 2928 | N | ASP | B | 77 | -10.613 | 30.467 | 38.545 | 1.00 | 56.58 | B | N |
| ATOM | 2929 | CA | ASP | B | 77 | -9.743 | 31.502 | 37.986 | 1.00 | 56.58 | B | C |
| ATOM | 2930 | CB | ASP | B | 77 | -8.787 | 32.043 | 39.048 | 1.00100.00 | | B | C |
| ATOM | 2931 | CG | ASP | B | 77 | -9.472 | 32.304 | 40.374 | 1.00100.00 | | B | C |
| ATOM | 2932 | OD1 | ASP | B | 77 | -9.794 | 31.310 | 41.102 | 1.00100.00 | | B | O |
| ATOM | 2933 | OD2 | ASP | B | 77 | -9.686 | 33.513 | 40.673 | 1.00100.00 | | B | O |
| ATOM | 2934 | C | ASP | B | 77 | -8.902 | 30.969 | 36.843 | 1.00 | 56.58 | B | C |
| ATOM | 2935 | O | ASP | B | 77 | -9.018 | 31.407 | 35.698 | 1.00 | 56.58 | B | O |
| ATOM | 2936 | N | SER | B | 78 | -8.041 | 30.020 | 37.183 | 1.00 | 55.77 | B | N |
| ATOM | 2937 | CA | SER | B | 78 | -7.144 | 29.404 | 36.219 | 1.00 | 55.77 | B | C |
| ATOM | 2938 | CB | SER | B | 78 | -5.767 | 29.244 | 36.857 | 1.00 | 62.64 | B | C |
| ATOM | 2939 | OG | SER | B | 78 | -5.895 | 28.606 | 38.123 | 1.00 | 62.64 | B | O |
| ATOM | 2940 | C | SER | B | 78 | -7.664 | 28.040 | 35.770 | 1.00 | 55.77 | B | C |
| ATOM | 2941 | O | SER | B | 78 | -7.052 | 27.374 | 34.934 | 1.00 | 55.77 | B | O |

| | | | | | | | | | | | | |
|---|---|---|---|---|---|---|---|---|---|---|---|---|
| ATOM | 2942 | N | GLY | B | 79 | -8.796 | 27.625 | 36.321 | 1.00 | 62.24 | B | N |
| ATOM | 2943 | CA | GLY | B | 79 | -9.317 | 26.329 | 35.952 | 1.00 | 62.24 | B | C |
| ATOM | 2944 | C | GLY | B | 79 | -8.395 | 25.278 | 36.552 | 1.00 | 62.24 | B | C |
| ATOM | 2945 | O | GLY | B | 79 | -8.728 | 24.084 | 36.580 | 1.00 | 62.24 | B | O |
| ATOM | 2946 | N | GLU | B | 80 | -7.234 | 25.725 | 37.042 | 1.00 | 40.03 | B | N |
| ATOM | 2947 | CA | GLU | B | 80 | -6.256 | 24.831 | 37.659 | 1.00 | 40.03 | B | C |
| ATOM | 2948 | CB | GLU | B | 80 | -5.217 | 25.632 | 38.437 | 1.00 | 58.38 | B | C |
| ATOM | 2949 | CG | GLU | B | 80 | -4.075 | 26.170 | 37.600 | 1.00 | 58.38 | B | C |
| ATOM | 2950 | CD | GLU | B | 80 | -3.101 | 27.004 | 38.416 | 1.00 | 58.38 | B | C |
| ATOM | 2951 | OE1 | GLU | B | 80 | -3.401 | 28.196 | 38.658 | 1.00 | 58.38 | B | O |
| ATOM | 2952 | OE2 | GLU | B | 80 | -2.045 | 26.461 | 38.829 | 1.00 | 58.38 | B | O |
| ATOM | 2953 | C | GLU | B | 80 | -6.914 | 23.836 | 38.598 | 1.00 | 40.03 | B | C |
| ATOM | 2954 | O | GLU | B | 80 | -7.752 | 24.196 | 39.423 | 1.00 | 40.03 | B | O |
| ATOM | 2955 | N | LEU | B | 81 | -6.524 | 22.579 | 38.466 | 1.00 | 43.48 | B | N |
| ATOM | 2956 | CA | LEU | B | 81 | -7.079 | 21.537 | 39.307 | 1.00 | 43.48 | B | C |
| ATOM | 2957 | CB | LEU | B | 81 | -6.921 | 20.177 | 38.624 | 1.00 | 64.15 | B | C |
| ATOM | 2958 | CG | LEU | B | 81 | -7.790 | 19.986 | 37.382 | 1.00 | 64.15 | B | C |
| ATOM | 2959 | CD1 | LEU | B | 81 | -7.586 | 18.585 | 36.837 | 1.00 | 64.15 | B | C |
| ATOM | 2960 | CD2 | LEU | B | 81 | -9.263 | 20.202 | 37.755 | 1.00 | 64.15 | B | C |
| ATOM | 2961 | C | LEU | B | 81 | -6.425 | 21.514 | 40.682 | 1.00 | 43.48 | B | C |
| ATOM | 2962 | O | LEU | B | 81 | -5.252 | 21.870 | 40.834 | 1.00 | 43.48 | B | O |
| ATOM | 2963 | N | VAL | B | 82 | -7.197 | 21.093 | 41.678 | 1.00 | 43.88 | B | N |
| ATOM | 2964 | CA | VAL | B | 82 | -6.711 | 21.019 | 43.052 | 1.00 | 43.88 | B | C |
| ATOM | 2965 | CB | VAL | B | 82 | -7.031 | 22.315 | 43.843 | 1.00 | 52.14 | B | C |
| ATOM | 2966 | CG1 | VAL | B | 82 | -6.366 | 23.509 | 43.194 | 1.00 | 52.14 | B | C |
| ATOM | 2967 | CG2 | VAL | B | 82 | -8.548 | 22.523 | 43.920 | 1.00 | 52.14 | B | C |
| ATOM | 2968 | C | VAL | B | 82 | -7.338 | 19.861 | 43.822 | 1.00 | 43.88 | B | C |
| ATOM | 2969 | O | VAL | B | 82 | -8.294 | 19.226 | 43.368 | 1.00 | 43.88 | B | O |
| ATOM | 2970 | N | ALA | B | 83 | -6.790 | 19.598 | 45.000 | 1.00 | 53.39 | B | N |
| ATOM | 2971 | CA | ALA | B | 83 | -7.298 | 18.544 | 45.869 | 1.00 | 53.39 | B | C |
| ATOM | 2972 | CB | ALA | B | 83 | -6.253 | 17.440 | 46.039 | 1.00 | 33.41 | B | C |
| ATOM | 2973 | C | ALA | B | 83 | -7.571 | 19.217 | 47.203 | 1.00 | 53.39 | B | C |
| ATOM | 2974 | O | ALA | B | 83 | -6.723 | 19.955 | 47.707 | 1.00 | 53.39 | B | O |
| ATOM | 2975 | N | ILE | B | 84 | -8.748 | 18.994 | 47.770 | 1.00 | 36.28 | B | N |
| ATOM | 2976 | CA | ILE | B | 84 | -9.050 | 19.614 | 49.045 | 1.00 | 36.28 | B | C |
| ATOM | 2977 | CB | ILE | B | 84 | -10.358 | 20.429 | 48.964 | 1.00 | 36.65 | B | C |
| ATOM | 2978 | CG2 | ILE | B | 84 | -10.590 | 21.188 | 50.264 | 1.00 | 36.65 | B | C |
| ATOM | 2979 | CG1 | ILE | B | 84 | -10.261 | 21.431 | 47.806 | 1.00 | 36.65 | B | C |
| ATOM | 2980 | CD1 | ILE | B | 84 | -11.494 | 22.273 | 47.598 | 1.00 | 36.65 | B | C |
| ATOM | 2981 | C | ILE | B | 84 | -9.137 | 18.553 | 50.127 | 1.00 | 36.28 | B | C |
| ATOM | 2982 | O | ILE | B | 84 | -10.041 | 17.715 | 50.125 | 1.00 | 36.28 | B | O |
| ATOM | 2983 | N | LYS | B | 85 | -8.174 | 18.580 | 51.044 | 1.00 | 39.65 | B | N |
| ATOM | 2984 | CA | LYS | B | 85 | -8.147 | 17.611 | 52.128 | 1.00 | 39.65 | B | C |
| ATOM | 2985 | CB | LYS | B | 85 | -6.701 | 17.341 | 52.546 | 1.00 | 46.41 | B | C |
| ATOM | 2986 | CG | LYS | B | 85 | -6.518 | 16.146 | 53.458 | 1.00 | 46.41 | B | C |
| ATOM | 2987 | CD | LYS | B | 85 | -5.054 | 15.968 | 53.854 | 1.00 | 46.41 | B | C |
| ATOM | 2988 | CE | LYS | B | 85 | -4.904 | 14.972 | 55.016 | 1.00 | 46.41 | B | C |
| ATOM | 2989 | NZ | LYS | B | 85 | -3.476 | 14.654 | 55.342 | 1.00 | 46.41 | B | N |
| ATOM | 2990 | C | LYS | B | 85 | -8.971 | 18.156 | 53.291 | 1.00 | 39.65 | B | C |
| ATOM | 2991 | O | LYS | B | 85 | -8.566 | 19.093 | 53.969 | 1.00 | 39.65 | B | O |
| ATOM | 2992 | N | LYS | B | 86 | -10.150 | 17.582 | 53.497 | 1.00 | 40.84 | B | N |
| ATOM | 2993 | CA | LYS | B | 86 | -11.031 | 18.027 | 54.566 | 1.00 | 40.84 | B | C |
| ATOM | 2994 | CB | LYS | B | 86 | -12.509 | 17.828 | 54.166 | 1.00 | 46.80 | B | C |
| ATOM | 2995 | CG | LYS | B | 86 | -13.469 | 18.719 | 54.941 | 1.00 | 46.80 | B | C |
| ATOM | 2996 | CD | LYS | B | 86 | -14.852 | 18.101 | 55.150 | 1.00 | 46.80 | B | C |
| ATOM | 2997 | CE | LYS | B | 86 | -15.756 | 18.218 | 53.928 | 1.00 | 46.80 | B | C |
| ATOM | 2998 | NZ | LYS | B | 86 | -16.104 | 19.638 | 53.576 | 1.00 | 46.80 | B | N |
| ATOM | 2999 | C | LYS | B | 86 | -10.695 | 17.218 | 55.814 | 1.00 | 40.84 | B | C |
| ATOM | 3000 | O | LYS | B | 86 | -10.683 | 15.994 | 55.783 | 1.00 | 40.84 | B | O |
| ATOM | 3001 | N | VAL | B | 87 | -10.418 | 17.905 | 56.912 | 1.00 | 48.08 | B | N |
| ATOM | 3002 | CA | VAL | B | 87 | -10.060 | 17.231 | 58.154 | 1.00 | 48.08 | B | C |
| ATOM | 3003 | CB | VAL | B | 87 | -8.551 | 17.379 | 58.464 | 1.00 | 35.08 | B | C |
| ATOM | 3004 | CG1 | VAL | B | 87 | -8.262 | 16.852 | 59.847 | 1.00 | 35.08 | B | C |
| ATOM | 3005 | CG2 | VAL | B | 87 | -7.718 | 16.641 | 57.439 | 1.00 | 35.08 | B | C |
| ATOM | 3006 | C | VAL | B | 87 | -10.815 | 17.786 | 59.349 | 1.00 | 48.08 | B | C |
| ATOM | 3007 | O | VAL | B | 87 | -10.931 | 19.003 | 59.503 | 1.00 | 48.08 | B | O |
| ATOM | 3008 | N | LEU | B | 88 | -11.322 | 16.895 | 60.197 | 1.00 | 63.03 | B | N |

| | | | | | | | | | | | | |
|---|---|---|---|---|---|---|---|---|---|---|---|---|
| ATOM | 3009 | CA | LEU | B | 88 | -12.037 | 17.322 | 61.398 | 1.00 | 63.03 | B | C |
| ATOM | 3010 | CB | LEU | B | 88 | -12.735 | 16.125 | 62.065 | 1.00 | 61.29 | B | C |
| ATOM | 3011 | CG | LEU | B | 88 | -13.706 | 16.269 | 63.260 | 1.00 | 61.29 | B | C |
| ATOM | 3012 | CD1 | LEU | B | 88 | -13.045 | 17.023 | 64.411 | 1.00 | 61.29 | B | C |
| ATOM | 3013 | CD2 | LEU | B | 88 | -14.988 | 16.979 | 62.805 | 1.00 | 61.29 | B | C |
| ATOM | 3014 | C | LEU | B | 88 | -10.950 | 17.861 | 62.325 | 1.00 | 63.03 | B | C |
| ATOM | 3015 | O | LEU | B | 88 | -10.150 | 17.084 | 62.842 | 1.00 | 63.03 | B | O |
| ATOM | 3016 | N | GLN | B | 89 | -10.890 | 19.176 | 62.511 | 1.00 | 56.81 | B | N |
| ATOM | 3017 | CA | GLN | B | 89 | -9.876 | 19.738 | 63.391 | 1.00 | 56.81 | B | C |
| ATOM | 3018 | CB | GLN | B | 89 | -9.100 | 20.860 | 62.704 | 1.00 | 70.27 | B | C |
| ATOM | 3019 | CG | GLN | B | 89 | -7.982 | 21.427 | 63.575 | 1.00 | 70.27 | B | C |
| ATOM | 3020 | CD | GLN | B | 89 | -7.035 | 20.346 | 64.109 | 1.00 | 70.27 | B | C |
| ATOM | 3021 | OE1 | GLN | B | 89 | -7.226 | 19.140 | 63.854 | 1.00 | 70.27 | B | O |
| ATOM | 3022 | NE2 | GLN | B | 89 | -6.006 | 20.772 | 64.859 | 1.00 | 70.27 | B | N |
| ATOM | 3023 | C | GLN | B | 89 | -10.482 | 20.259 | 64.680 | 1.00 | 56.81 | B | C |
| ATOM | 3024 | O | GLN | B | 89 | -11.527 | 20.911 | 64.678 | 1.00 | 56.81 | B | O |
| ATOM | 3025 | N | ASP | B | 90 | -9.805 | 19.973 | 65.784 | 1.00 | 85.43 | B | N |
| ATOM | 3026 | CA | ASP | B | 90 | -10.270 | 20.378 | 67.109 | 1.00 | 85.43 | B | C |
| ATOM | 3027 | CB | ASP | B | 90 | -9.806 | 19.325 | 68.115 | 1.00 | 95.80 | B | C |
| ATOM | 3028 | CG | ASP | B | 90 | -10.090 | 19.713 | 69.548 | 1.00 | 95.80 | B | C |
| ATOM | 3029 | OD1 | ASP | B | 90 | -9.953 | 18.814 | 70.422 | 1.00 | 95.80 | B | O |
| ATOM | 3030 | OD2 | ASP | B | 90 | -10.434 | 20.897 | 69.803 | 1.00 | 95.80 | B | O |
| ATOM | 3031 | C | ASP | B | 90 | -9.743 | 21.768 | 67.501 | 1.00 | 85.43 | B | C |
| ATOM | 3032 | O | ASP | B | 90 | -8.724 | 21.864 | 68.178 | 1.00 | 85.43 | B | O |
| ATOM | 3033 | N | LYS | B | 91 | -10.433 | 22.832 | 67.076 | 1.00 | 91.44 | B | N |
| ATOM | 3034 | CA | LYS | B | 91 | -10.030 | 24.232 | 67.326 | 1.00 | 91.44 | B | C |
| ATOM | 3035 | CB | LYS | B | 91 | -11.287 | 25.110 | 67.389 | 1.00 | 90.01 | B | C |
| ATOM | 3036 | CG | LYS | B | 91 | -11.814 | 25.535 | 66.001 | 1.00 | 90.01 | B | C |
| ATOM | 3037 | CD | LYS | B | 91 | -11.086 | 26.779 | 65.416 | 1.00 | 90.01 | B | C |
| ATOM | 3038 | CE | LYS | B | 91 | -9.553 | 26.601 | 65.292 | 1.00 | 90.01 | B | C |
| ATOM | 3039 | NZ | LYS | B | 91 | -8.839 | 27.828 | 64.789 | 1.00 | 90.01 | B | N |
| ATOM | 3040 | C | LYS | B | 91 | -9.064 | 24.625 | 68.484 | 1.00 | 91.44 | B | C |
| ATOM | 3041 | O | LYS | B | 91 | -8.222 | 25.518 | 68.320 | 1.00 | 91.44 | B | O |
| ATOM | 3042 | N | ALA | B | 92 | -9.192 | 23.979 | 69.635 | 1.00 | 79.01 | B | N |
| ATOM | 3043 | CA | ALA | B | 92 | -8.364 | 24.218 | 70.826 | 1.00 | 79.01 | B | C |
| ATOM | 3044 | CB | ALA | B | 92 | -8.852 | 23.291 | 71.949 | 1.00 | 74.41 | B | C |
| ATOM | 3045 | C | ALA | B | 92 | -6.855 | 23.987 | 70.564 | 1.00 | 79.01 | B | C |
| ATOM | 3046 | O | ALA | B | 92 | -5.981 | 24.665 | 71.135 | 1.00 | 79.01 | B | O |
| ATOM | 3047 | N | ALA | B | 93 | -6.568 | 23.016 | 69.700 | 1.00 | 86.92 | B | N |
| ATOM | 3048 | CA | ALA | B | 93 | -5.198 | 22.669 | 69.337 | 1.00 | 86.92 | B | C |
| ATOM | 3049 | CB | ALA | B | 93 | -4.945 | 21.180 | 69.612 | 1.00 | 53.15 | B | C |
| ATOM | 3050 | C | ALA | B | 93 | -4.926 | 22.993 | 67.860 | 1.00 | 86.92 | B | C |
| ATOM | 3051 | O | ALA | B | 93 | -5.809 | 22.839 | 66.995 | 1.00 | 86.92 | B | O |
| ATOM | 3052 | N | ALA | B | 94 | -3.703 | 23.457 | 67.588 | 1.00 | 73.93 | B | N |
| ATOM | 3053 | CA | ALA | B | 94 | -3.289 | 23.790 | 66.228 | 1.00 | 73.93 | B | C |
| ATOM | 3054 | CB | ALA | B | 94 | -2.005 | 24.619 | 66.252 | 1.00 | 49.39 | B | C |
| ATOM | 3055 | C | ALA | B | 94 | -3.057 | 22.478 | 65.487 | 1.00 | 73.93 | B | C |
| ATOM | 3056 | O | ALA | B | 94 | -2.632 | 21.479 | 66.091 | 1.00 | 73.93 | B | O |
| ATOM | 3057 | N | ASN | B | 95 | -3.342 | 22.489 | 64.183 | 1.00 | 40.63 | B | N |
| ATOM | 3058 | CA | ASN | B | 95 | -3.185 | 21.316 | 63.317 | 1.00 | 40.63 | B | C |
| ATOM | 3059 | CB | ASN | B | 95 | -4.062 | 21.506 | 62.076 | 1.00 | 49.60 | B | C |
| ATOM | 3060 | CG | ASN | B | 95 | -4.137 | 20.266 | 61.212 | 1.00 | 49.60 | B | C |
| ATOM | 3061 | OD1 | ASN | B | 95 | -3.259 | 20.019 | 60.381 | 1.00 | 49.60 | B | O |
| ATOM | 3062 | ND2 | ASN | B | 95 | -5.186 | 19.473 | 61.406 | 1.00 | 49.60 | B | N |
| ATOM | 3063 | C | ASN | B | 95 | -1.712 | 21.102 | 62.926 | 1.00 | 40.63 | B | C |
| ATOM | 3064 | O | ASN | B | 95 | -1.081 | 21.975 | 62.328 | 1.00 | 40.63 | B | O |
| ATOM | 3065 | N | ARG | B | 96 | -1.169 | 19.938 | 63.268 | 1.00 | 44.26 | B | N |
| ATOM | 3066 | CA | ARG | B | 96 | 0.229 | 19.640 | 62.973 | 1.00 | 44.26 | B | C |
| ATOM | 3067 | CB | ARG | B | 96 | 0.631 | 18.313 | 63.612 | 1.00 | 55.54 | B | C |
| ATOM | 3068 | CG | ARG | B | 96 | 2.121 | 18.084 | 63.648 | 1.00 | 55.54 | B | C |
| ATOM | 3069 | CD | ARG | B | 96 | 2.476 | 16.687 | 64.176 | 1.00 | 55.54 | B | C |
| ATOM | 3070 | NE | ARG | B | 96 | 2.543 | 16.589 | 65.637 | 1.00 | 55.54 | B | N |
| ATOM | 3071 | CZ | ARG | B | 96 | 2.712 | 15.440 | 66.292 | 1.00 | 55.54 | B | C |
| ATOM | 3072 | NH1 | ARG | B | 96 | 2.826 | 14.303 | 65.611 | 1.00 | 55.54 | B | N |
| ATOM | 3073 | NH2 | ARG | B | 96 | 2.760 | 15.415 | 67.619 | 1.00 | 55.54 | B | N |
| ATOM | 3074 | C | ARG | B | 96 | 0.562 | 19.617 | 61.478 | 1.00 | 44.26 | B | C |
| ATOM | 3075 | O | ARG | B | 96 | 1.586 | 20.158 | 61.060 | 1.00 | 44.26 | B | O |

| | | | | | | | | | | | | |
|---|---|---|---|---|---|---|---|---|---|---|---|---|
| ATOM | 3076 | N | GLU | B | 97 | -0.291 | 19.000 | 60.667 | 1.00 | 38.77 | B | N |
| ATOM | 3077 | CA | GLU | B | 97 | -0.032 | 18.948 | 59.237 | 1.00 | 38.77 | B | C |
| ATOM | 3078 | CB | GLU | B | 97 | -1.116 | 18.137 | 58.527 | 1.00 | 40.61 | B | C |
| ATOM | 3079 | CG | GLU | B | 97 | -0.828 | 17.885 | 57.056 | 1.00 | 40.61 | B | C |
| ATOM | 3080 | CD | GLU | B | 97 | -1.835 | 16.944 | 56.427 | 1.00 | 40.61 | B | C |
| ATOM | 3081 | OE1 | GLU | B | 97 | -2.608 | 16.319 | 57.190 | 1.00 | 40.61 | B | O |
| ATOM | 3082 | OE2 | GLU | B | 97 | -1.847 | 16.813 | 55.181 | 1.00 | 40.61 | B | O |
| ATOM | 3083 | C | GLU | B | 97 | 0.027 | 20.351 | 58.638 | 1.00 | 38.77 | B | C |
| ATOM | 3084 | O | GLU | B | 97 | 0.901 | 20.651 | 57.831 | 1.00 | 38.77 | B | O |
| ATOM | 3085 | N | LEU | B | 98 | -0.910 | 21.209 | 59.030 | 1.00 | 32.75 | B | N |
| ATOM | 3086 | CA | LEU | B | 98 | -0.946 | 22.565 | 58.502 | 1.00 | 32.75 | B | C |
| ATOM | 3087 | CB | LEU | B | 98 | -2.189 | 23.299 | 59.020 | 1.00 | 30.03 | B | C |
| ATOM | 3088 | CG | LEU | B | 98 | -2.302 | 24.779 | 58.640 | 1.00 | 30.03 | B | C |
| ATOM | 3089 | CD1 | LEU | B | 98 | -2.025 | 24.945 | 57.152 | 1.00 | 30.03 | B | C |
| ATOM | 3090 | CD2 | LEU | B | 98 | -3.681 | 25.303 | 59.001 | 1.00 | 30.03 | B | C |
| ATOM | 3091 | C | LEU | B | 98 | 0.317 | 23.363 | 58.839 | 1.00 | 32.75 | B | C |
| ATOM | 3092 | O | LEU | B | 98 | 0.859 | 24.080 | 57.994 | 1.00 | 32.75 | B | O |
| ATOM | 3093 | N | GLN | B | 99 | 0.787 | 23.236 | 60.074 | 1.00 | 56.98 | B | N |
| ATOM | 3094 | CA | GLN | B | 99 | 1.978 | 23.954 | 60.495 | 1.00 | 56.98 | B | C |
| ATOM | 3095 | CB | GLN | B | 99 | 2.225 | 23.748 | 61.994 | 1.00 | 96.79 | B | C |
| ATOM | 3096 | CG | GLN | B | 99 | 1.362 | 24.621 | 62.902 | 1.00 | 96.79 | B | C |
| ATOM | 3097 | CD | GLN | B | 99 | 1.640 | 24.371 | 64.389 | 1.00 | 96.79 | B | C |
| ATOM | 3098 | OE1 | GLN | B | 99 | 1.056 | 25.034 | 65.266 | 1.00 | 96.79 | B | O |
| ATOM | 3099 | NE2 | GLN | B | 99 | 2.530 | 23.404 | 64.682 | 1.00 | 96.79 | B | N |
| ATOM | 3100 | C | GLN | B | 99 | 3.210 | 23.534 | 59.701 | 1.00 | 56.98 | B | C |
| ATOM | 3101 | O | GLN | B | 99 | 4.011 | 24.381 | 59.309 | 1.00 | 56.98 | B | O |
| ATOM | 3102 | N | ILE | B | 100 | 3.368 | 22.236 | 59.468 | 1.00 | 41.38 | B | N |
| ATOM | 3103 | CA | ILE | B | 100 | 4.509 | 21.749 | 58.702 | 1.00 | 41.38 | B | C |
| ATOM | 3104 | CB | ILE | B | 100 | 4.599 | 20.208 | 58.744 | 1.00 | 32.04 | B | C |
| ATOM | 3105 | CG2 | ILE | B | 100 | 5.651 | 19.718 | 57.767 | 1.00 | 32.04 | B | C |
| ATOM | 3106 | CG1 | ILE | B | 100 | 4.931 | 19.751 | 60.169 | 1.00 | 32.04 | B | C |
| ATOM | 3107 | CD1 | ILE | B | 100 | 4.935 | 18.261 | 60.362 | 1.00 | 32.04 | B | C |
| ATOM | 3108 | C | ILE | B | 100 | 4.360 | 22.190 | 57.257 | 1.00 | 41.38 | B | C |
| ATOM | 3109 | O | ILE | B | 100 | 5.219 | 22.871 | 56.704 | 1.00 | 41.38 | B | O |
| ATOM | 3110 | N | MET | B | 101 | 3.242 | 21.798 | 56.662 | 1.00 | 53.97 | B | N |
| ATOM | 3111 | CA | MET | B | 101 | 2.911 | 22.104 | 55.282 | 1.00 | 53.97 | B | C |
| ATOM | 3112 | CB | MET | B | 101 | 1.435 | 21.801 | 55.051 | 1.00 | 58.31 | B | C |
| ATOM | 3113 | CG | MET | B | 101 | 1.078 | 21.624 | 53.610 | 1.00 | 58.31 | B | C |
| ATOM | 3114 | SD | MET | B | 101 | 1.852 | 20.117 | 52.967 | 1.00 | 58.31 | B | S |
| ATOM | 3115 | CE | MET | B | 101 | 0.431 | 18.907 | 53.135 | 1.00 | 58.31 | B | C |
| ATOM | 3116 | C | MET | B | 101 | 3.178 | 23.564 | 54.948 | 1.00 | 53.97 | B | C |
| ATOM | 3117 | O | MET | B | 101 | 3.835 | 23.890 | 53.954 | 1.00 | 53.97 | B | O |
| ATOM | 3118 | N | ARG | B | 102 | 2.661 | 24.436 | 55.803 | 1.00 | 37.14 | B | N |
| ATOM | 3119 | CA | ARG | B | 102 | 2.762 | 25.883 | 55.642 | 1.00 | 37.14 | B | C |
| ATOM | 3120 | CB | ARG | B | 102 | 1.996 | 26.546 | 56.789 | 1.00 | 62.24 | B | C |
| ATOM | 3121 | CG | ARG | B | 102 | 1.642 | 28.006 | 56.591 | 1.00 | 62.24 | B | C |
| ATOM | 3122 | CD | ARG | B | 102 | 0.182 | 28.185 | 56.202 | 1.00 | 62.24 | B | C |
| ATOM | 3123 | NE | ARG | B | 102 | -0.228 | 29.585 | 56.315 | 1.00 | 62.24 | B | N |
| ATOM | 3124 | CZ | ARG | B | 102 | -0.504 | 30.188 | 57.466 | 1.00 | 62.24 | B | C |
| ATOM | 3125 | NH1 | ARG | B | 102 | -0.420 | 29.510 | 58.603 | 1.00 | 62.24 | B | N |
| ATOM | 3126 | NH2 | ARG | B | 102 | -0.857 | 31.467 | 57.479 | 1.00 | 62.24 | B | N |
| ATOM | 3127 | C | ARG | B | 102 | 4.169 | 26.487 | 55.547 | 1.00 | 37.14 | B | C |
| ATOM | 3128 | O | ARG | B | 102 | 4.321 | 27.605 | 55.067 | 1.00 | 37.14 | B | O |
| ATOM | 3129 | N | LYS | B | 103 | 5.194 | 25.767 | 55.997 | 1.00 | 42.28 | B | N |
| ATOM | 3130 | CA | LYS | B | 103 | 6.557 | 26.308 | 55.956 | 1.00 | 42.28 | B | C |
| ATOM | 3131 | CB | LYS | B | 103 | 7.205 | 26.256 | 57.350 | 1.00 | 60.27 | B | C |
| ATOM | 3132 | CG | LYS | B | 103 | 7.750 | 24.899 | 57.749 | 1.00 | 60.27 | B | C |
| ATOM | 3133 | CD | LYS | B | 103 | 8.501 | 24.930 | 59.086 | 1.00 | 60.27 | B | C |
| ATOM | 3134 | CE | LYS | B | 103 | 7.561 | 25.112 | 60.276 | 1.00 | 60.27 | B | C |
| ATOM | 3135 | NZ | LYS | B | 103 | 8.194 | 24.712 | 61.568 | 1.00 | 60.27 | B | N |
| ATOM | 3136 | C | LYS | B | 103 | 7.472 | 25.612 | 54.964 | 1.00 | 42.28 | B | C |
| ATOM | 3137 | O | LYS | B | 103 | 8.690 | 25.761 | 55.025 | 1.00 | 42.28 | B | O |
| ATOM | 3138 | N | LEU | B | 104 | 6.877 | 24.851 | 54.055 | 1.00 | 50.04 | B | N |
| ATOM | 3139 | CA | LEU | B | 104 | 7.630 | 24.127 | 53.041 | 1.00 | 50.04 | B | C |
| ATOM | 3140 | CB | LEU | B | 104 | 7.203 | 22.659 | 53.025 | 1.00 | 41.16 | B | C |
| ATOM | 3141 | CG | LEU | B | 104 | 7.991 | 21.643 | 53.855 | 1.00 | 41.16 | B | C |
| ATOM | 3142 | CD1 | LEU | B | 104 | 8.357 | 22.193 | 55.217 | 1.00 | 41.16 | B | C |

| | | | | | | | | | | | | |
|---|---|---|---|---|---|---|---|---|---|---|---|---|
| ATOM | 3143 | CD2 | LEU | B | 104 | 7.150 | 20.384 | 53.976 | 1.00 | 41.16 | B | C |
| ATOM | 3144 | C | LEU | B | 104 | 7.428 | 24.722 | 51.652 | 1.00 | 50.04 | B | C |
| ATOM | 3145 | O | LEU | B | 104 | 6.324 | 25.115 | 51.283 | 1.00 | 50.04 | B | O |
| ATOM | 3146 | N | ASP | B | 105 | 8.506 | 24.802 | 50.886 | 1.00 | 45.95 | B | N |
| ATOM | 3147 | CA | ASP | B | 105 | 8.429 | 25.308 | 49.522 | 1.00 | 45.95 | B | C |
| ATOM | 3148 | CB | ASP | B | 105 | 8.648 | 26.817 | 49.476 | 1.00 | 60.25 | B | C |
| ATOM | 3149 | CG | ASP | B | 105 | 8.411 | 27.401 | 48.070 | 1.00 | 60.25 | B | C |
| ATOM | 3150 | OD1 | ASP | B | 105 | 8.071 | 26.621 | 47.129 | 1.00 | 60.25 | B | O |
| ATOM | 3151 | OD2 | ASP | B | 105 | 8.568 | 28.642 | 47.932 | 1.00 | 60.25 | B | O |
| ATOM | 3152 | C | ASP | B | 105 | 9.473 | 24.607 | 48.670 | 1.00 | 45.95 | B | C |
| ATOM | 3153 | O | ASP | B | 105 | 10.592 | 25.094 | 48.505 | 1.00 | 45.95 | B | O |
| ATOM | 3154 | N | HIS | B | 106 | 9.092 | 23.457 | 48.129 | 1.00 | 50.78 | B | N |
| ATOM | 3155 | CA | HIS | B | 106 | 10.006 | 22.674 | 47.322 | 1.00 | 50.78 | B | C |
| ATOM | 3156 | CB | HIS | B | 106 | 10.624 | 21.542 | 48.156 | 1.00 | 50.33 | B | C |
| ATOM | 3157 | CG | HIS | B | 106 | 11.915 | 21.026 | 47.621 | 1.00 | 50.33 | B | C |
| ATOM | 3158 | CD2 | HIS | B | 106 | 13.186 | 21.172 | 48.058 | 1.00 | 50.33 | B | C |
| ATOM | 3159 | ND1 | HIS | B | 106 | 11.999 | 20.226 | 46.491 | 1.00 | 50.33 | B | N |
| ATOM | 3160 | CE1 | HIS | B | 106 | 13.259 | 19.909 | 46.275 | 1.00 | 50.33 | B | C |
| ATOM | 3161 | NE2 | HIS | B | 106 | 14.005 | 20.471 | 47.216 | 1.00 | 50.33 | B | N |
| ATOM | 3162 | C | HIS | B | 106 | 9.235 | 22.107 | 46.159 | 1.00 | 50.78 | B | C |
| ATOM | 3163 | O | HIS | B | 106 | 8.111 | 21.562 | 46.312 | 1.00 | 50.78 | B | O |
| ATOM | 3164 | N | CYS | B | 107 | 9.845 | 22.241 | 44.992 | 1.00 | 35.53 | B | N |
| ATOM | 3165 | CA | CYS | B | 107 | 9.175 | 21.757 | 43.846 | 1.00 | 35.53 | B | C |
| ATOM | 3166 | CB | CYS | B | 107 | 9.834 | 22.295 | 42.578 | 1.00 | 66.39 | B | C |
| ATOM | 3167 | SG | CYS | B | 107 | 9.222 | 24.019 | 42.193 | 1.00 | 66.39 | B | S |
| ATOM | 3168 | C | CYS | B | 107 | 9.032 | 20.256 | 43.855 | 1.00 | 35.53 | B | C |
| ATOM | 3169 | O | CYS | B | 107 | 8.482 | 19.681 | 42.916 | 1.00 | 35.53 | B | O |
| ATOM | 3170 | N | ASN | B | 108 | 9.462 | 19.600 | 44.927 | 1.00 | 40.59 | B | N |
| ATOM | 3171 | CA | ASN | B | 108 | 9.272 | 18.172 | 44.926 | 1.00 | 40.59 | B | C |
| ATOM | 3172 | CB | ASN | B | 108 | 10.595 | 17.440 | 45.084 | 1.00 | 31.95 | B | C |
| ATOM | 3173 | CG | ASN | B | 108 | 11.351 | 17.342 | 43.778 | 1.00 | 31.95 | B | C |
| ATOM | 3174 | OD1 | ASN | B | 108 | 12.493 | 17.765 | 43.677 | 1.00 | 31.95 | B | O |
| ATOM | 3175 | ND2 | ASN | B | 108 | 10.704 | 16.774 | 42.761 | 1.00 | 31.95 | B | N |
| ATOM | 3176 | C | ASN | B | 108 | 8.302 | 17.818 | 46.017 | 1.00 | 40.59 | B | C |
| ATOM | 3177 | O | ASN | B | 108 | 8.239 | 16.679 | 46.449 | 1.00 | 40.59 | B | O |
| ATOM | 3178 | N | ILE | B | 109 | 7.523 | 18.811 | 46.443 | 1.00 | 26.36 | B | N |
| ATOM | 3179 | CA | ILE | B | 109 | 6.515 | 18.622 | 47.477 | 1.00 | 26.36 | B | C |
| ATOM | 3180 | CB | ILE | B | 109 | 6.978 | 19.250 | 48.815 | 1.00 | 38.53 | B | C |
| ATOM | 3181 | CG2 | ILE | B | 109 | 5.880 | 19.186 | 49.853 | 1.00 | 38.53 | B | C |
| ATOM | 3182 | CG1 | ILE | B | 109 | 8.200 | 18.496 | 49.318 | 1.00 | 38.53 | B | C |
| ATOM | 3183 | CD1 | ILE | B | 109 | 8.712 | 18.985 | 50.633 | 1.00 | 38.53 | B | C |
| ATOM | 3184 | C | ILE | B | 109 | 5.223 | 19.280 | 47.018 | 1.00 | 26.36 | B | C |
| ATOM | 3185 | O | ILE | B | 109 | 5.273 | 20.368 | 46.456 | 1.00 | 26.36 | B | O |
| ATOM | 3186 | N | VAL | B | 110 | 4.077 | 18.632 | 47.230 | 1.00 | 35.57 | B | N |
| ATOM | 3187 | CA | VAL | B | 110 | 2.817 | 19.251 | 46.819 | 1.00 | 35.57 | B | C |
| ATOM | 3188 | CB | VAL | B | 110 | 1.561 | 18.489 | 47.269 | 1.00 | 46.26 | B | C |
| ATOM | 3189 | CG1 | VAL | B | 110 | 1.503 | 17.143 | 46.608 | 1.00 | 46.26 | B | C |
| ATOM | 3190 | CG2 | VAL | B | 110 | 1.544 | 18.367 | 48.778 | 1.00 | 46.26 | B | C |
| ATOM | 3191 | C | VAL | B | 110 | 2.753 | 20.596 | 47.493 | 1.00 | 35.57 | B | C |
| ATOM | 3192 | O | VAL | B | 110 | 3.096 | 20.726 | 48.661 | 1.00 | 35.57 | B | O |
| ATOM | 3193 | N | ARG | B | 111 | 2.319 | 21.602 | 46.751 | 1.00 | 48.70 | B | N |
| ATOM | 3194 | CA | ARG | B | 111 | 2.207 | 22.939 | 47.292 | 1.00 | 48.70 | B | C |
| ATOM | 3195 | CB | ARG | B | 111 | 2.378 | 23.980 | 46.172 | 1.00 | 76.02 | B | C |
| ATOM | 3196 | CG | ARG | B | 111 | 2.270 | 25.367 | 46.741 | 1.00 | 76.02 | B | C |
| ATOM | 3197 | CD | ARG | B | 111 | 2.605 | 26.467 | 45.925 | 1.00 | 76.02 | B | C |
| ATOM | 3198 | NE | ARG | B | 111 | 2.034 | 26.367 | 44.682 | 1.00 | 76.02 | B | N |
| ATOM | 3199 | CZ | ARG | B | 111 | 1.821 | 27.331 | 43.828 | 1.00 | 76.02 | B | C |
| ATOM | 3200 | NH1 | ARG | B | 111 | 2.079 | 28.641 | 44.060 | 1.00 | 76.02 | B | N |
| ATOM | 3201 | NH2 | ARG | B | 111 | 1.525 | 26.863 | 42.633 | 1.00 | 76.02 | B | N |
| ATOM | 3202 | C | ARG | B | 111 | 0.838 | 23.099 | 47.967 | 1.00 | 48.70 | B | C |
| ATOM | 3203 | O | ARG | B | 111 | -0.164 | 22.524 | 47.523 | 1.00 | 48.70 | B | O |
| ATOM | 3204 | N | LEU | B | 112 | 0.808 | 23.846 | 49.065 | 1.00 | 47.15 | B | N |
| ATOM | 3205 | CA | LEU | B | 112 | -0.436 | 24.109 | 49.762 | 1.00 | 47.15 | B | C |
| ATOM | 3206 | CB | LEU | B | 112 | -0.208 | 24.149 | 51.270 | 1.00 | 31.61 | B | C |
| ATOM | 3207 | CG | LEU | B | 112 | -1.380 | 24.619 | 52.134 | 1.00 | 31.61 | B | C |
| ATOM | 3208 | CD1 | LEU | B | 112 | -2.407 | 23.509 | 52.249 | 1.00 | 31.61 | B | C |
| ATOM | 3209 | CD2 | LEU | B | 112 | -0.885 | 25.002 | 53.509 | 1.00 | 31.61 | B | C |

| | | | | | | | | | | | | |
|---|---|---|---|---|---|---|---|---|---|---|---|---|
| ATOM | 3210 | C | LEU | B | 112 | -0.822 | 25.493 | 49.257 | 1.00 | 47.15 | B | C |
| ATOM | 3211 | O | LEU | B | 112 | -0.280 | 26.494 | 49.722 | 1.00 | 47.15 | B | O |
| ATOM | 3212 | N | ARG | B | 113 | -1.735 | 25.544 | 48.290 | 1.00 | 51.35 | B | N |
| ATOM | 3213 | CA | ARG | B | 113 | -2.185 | 26.808 | 47.712 | 1.00 | 51.35 | B | C |
| ATOM | 3214 | CB | ARG | B | 113 | -3.136 | 26.546 | 46.541 | 1.00 | 63.30 | B | C |
| ATOM | 3215 | CG | ARG | B | 113 | -2.575 | 25.614 | 45.474 | 1.00 | 63.30 | B | C |
| ATOM | 3216 | CD | ARG | B | 113 | -1.436 | 26.271 | 44.704 | 1.00 | 63.30 | B | C |
| ATOM | 3217 | NE | ARG | B | 113 | -1.908 | 27.068 | 43.571 | 1.00 | 63.30 | B | N |
| ATOM | 3218 | CZ | ARG | B | 113 | -2.465 | 26.553 | 42.474 | 1.00 | 63.30 | B | C |
| ATOM | 3219 | NH1 | ARG | B | 113 | -2.623 | 25.235 | 42.356 | 1.00 | 63.30 | B | N |
| ATOM | 3220 | NH2 | ARG | B | 113 | -2.861 | 27.355 | 41.493 | 1.00 | 63.30 | B | N |
| ATOM | 3221 | C | ARG | B | 113 | -2.892 | 27.679 | 48.745 | 1.00 | 51.35 | B | C |
| ATOM | 3222 | O | ARG | B | 113 | -2.595 | 28.869 | 48.869 | 1.00 | 51.35 | B | O |
| ATOM | 3223 | N | TYR | B | 114 | -3.829 | 27.083 | 49.476 | 1.00 | 39.49 | B | N |
| ATOM | 3224 | CA | TYR | B | 114 | -4.595 | 27.797 | 50.495 | 1.00 | 39.49 | B | C |
| ATOM | 3225 | CB | TYR | B | 114 | -5.871 | 28.430 | 49.910 | 1.00 | 48.51 | B | C |
| ATOM | 3226 | CG | TYR | B | 114 | -5.700 | 29.192 | 48.623 | 1.00 | 48.51 | B | C |
| ATOM | 3227 | CD1 | TYR | B | 114 | -5.087 | 30.443 | 48.603 | 1.00 | 48.51 | B | C |
| ATOM | 3228 | CE1 | TYR | B | 114 | -4.888 | 31.120 | 47.406 | 1.00 | 48.51 | B | C |
| ATOM | 3229 | CD2 | TYR | B | 114 | -6.112 | 28.640 | 47.410 | 1.00 | 48.51 | B | C |
| ATOM | 3230 | CE2 | TYR | B | 114 | -5.916 | 29.309 | 46.213 | 1.00 | 48.51 | B | C |
| ATOM | 3231 | CZ | TYR | B | 114 | -5.302 | 30.547 | 46.219 | 1.00 | 48.51 | B | C |
| ATOM | 3232 | OH | TYR | B | 114 | -5.084 | 31.211 | 45.037 | 1.00 | 48.51 | B | O |
| ATOM | 3233 | C | TYR | B | 114 | -5.065 | 26.789 | 51.519 | 1.00 | 39.49 | B | C |
| ATOM | 3234 | O | TYR | B | 114 | -4.844 | 25.586 | 51.386 | 1.00 | 39.49 | B | O |
| ATOM | 3235 | N | PHE | B | 115 | -5.730 | 27.296 | 52.543 | 1.00 | 41.15 | B | N |
| ATOM | 3236 | CA | PHE | B | 115 | -6.325 | 26.449 | 53.554 | 1.00 | 41.15 | B | C |
| ATOM | 3237 | CB | PHE | B | 115 | -5.305 | 26.040 | 54.632 | 1.00 | 43.07 | B | C |
| ATOM | 3238 | CG | PHE | B | 115 | -4.899 | 27.139 | 55.564 | 1.00 | 43.07 | B | C |
| ATOM | 3239 | CD1 | PHE | B | 115 | -5.600 | 27.361 | 56.740 | 1.00 | 43.07 | B | C |
| ATOM | 3240 | CD2 | PHE | B | 115 | -3.773 | 27.907 | 55.303 | 1.00 | 43.07 | B | C |
| ATOM | 3241 | CE1 | PHE | B | 115 | -5.180 | 28.327 | 57.645 | 1.00 | 43.07 | B | C |
| ATOM | 3242 | CE2 | PHE | B | 115 | -3.347 | 28.875 | 56.203 | 1.00 | 43.07 | B | C |
| ATOM | 3243 | CZ | PHE | B | 115 | -4.048 | 29.082 | 57.374 | 1.00 | 43.07 | B | C |
| ATOM | 3244 | C | PHE | B | 115 | -7.472 | 27.286 | 54.094 | 1.00 | 41.15 | B | C |
| ATOM | 3245 | O | PHE | B | 115 | -7.379 | 28.516 | 54.159 | 1.00 | 41.15 | B | O |
| ATOM | 3246 | N | PHE | B | 116 | -8.580 | 26.632 | 54.412 | 1.00 | 41.56 | B | N |
| ATOM | 3247 | CA | PHE | B | 116 | -9.725 | 27.352 | 54.923 | 1.00 | 41.56 | B | C |
| ATOM | 3248 | CB | PHE | B | 116 | -10.579 | 27.868 | 53.750 | 1.00 | 48.02 | B | C |
| ATOM | 3249 | CG | PHE | B | 116 | -11.284 | 26.789 | 52.962 | 1.00 | 48.02 | B | C |
| ATOM | 3250 | CD1 | PHE | B | 116 | -12.506 | 26.270 | 53.395 | 1.00 | 48.02 | B | C |
| ATOM | 3251 | CD2 | PHE | B | 116 | -10.755 | 26.324 | 51.760 | 1.00 | 48.02 | B | C |
| ATOM | 3252 | CE1 | PHE | B | 116 | -13.197 | 25.308 | 52.637 | 1.00 | 48.02 | B | C |
| ATOM | 3253 | CE2 | PHE | B | 116 | -11.442 | 25.358 | 50.994 | 1.00 | 48.02 | B | C |
| ATOM | 3254 | CZ | PHE | B | 116 | -12.664 | 24.855 | 51.437 | 1.00 | 48.02 | B | C |
| ATOM | 3255 | C | PHE | B | 116 | -10.528 | 26.460 | 55.850 | 1.00 | 41.56 | B | C |
| ATOM | 3256 | O | PHE | B | 116 | -10.311 | 25.252 | 55.891 | 1.00 | 41.56 | B | O |
| ATOM | 3257 | N | TYR | B | 117 | -11.440 | 27.063 | 56.605 | 1.00 | 53.88 | B | N |
| ATOM | 3258 | CA | TYR | B | 117 | -12.270 | 26.319 | 57.541 | 1.00 | 53.88 | B | C |
| ATOM | 3259 | CB | TYR | B | 117 | -12.194 | 26.973 | 58.914 | 1.00 | 45.96 | B | C |
| ATOM | 3260 | CG | TYR | B | 117 | -10.787 | 26.974 | 59.438 | 1.00 | 45.96 | B | C |
| ATOM | 3261 | CD1 | TYR | B | 117 | -10.275 | 25.870 | 60.116 | 1.00 | 45.96 | B | C |
| ATOM | 3262 | CE1 | TYR | B | 117 | -8.937 | 25.827 | 60.514 | 1.00 | 45.96 | B | C |
| ATOM | 3263 | CD2 | TYR | B | 117 | -9.931 | 28.041 | 59.173 | 1.00 | 45.96 | B | C |
| ATOM | 3264 | CE2 | TYR | B | 117 | -8.597 | 28.006 | 59.560 | 1.00 | 45.96 | B | C |
| ATOM | 3265 | CZ | TYR | B | 117 | -8.110 | 26.899 | 60.228 | 1.00 | 45.96 | B | C |
| ATOM | 3266 | OH | TYR | B | 117 | -6.794 | 26.866 | 60.602 | 1.00 | 45.96 | B | O |
| ATOM | 3267 | C | TYR | B | 117 | -13.712 | 26.232 | 57.057 | 1.00 | 53.88 | B | C |
| ATOM | 3268 | O | TYR | B | 117 | -14.110 | 26.944 | 56.129 | 1.00 | 53.88 | B | O |
| ATOM | 3269 | N | SER | B | 118 | -14.495 | 25.356 | 57.681 | 1.00 | 53.94 | B | N |
| ATOM | 3270 | CA | SER | B | 118 | -15.876 | 25.183 | 57.277 | 1.00 | 53.94 | B | C |
| ATOM | 3271 | CB | SER | B | 118 | -15.921 | 24.507 | 55.908 | 1.00 | 45.85 | B | C |
| ATOM | 3272 | OG | SER | B | 118 | -15.060 | 23.391 | 55.879 | 1.00 | 45.85 | B | O |
| ATOM | 3273 | C | SER | B | 118 | -16.711 | 24.387 | 58.265 | 1.00 | 53.94 | B | C |
| ATOM | 3274 | O | SER | B | 118 | -16.202 | 23.870 | 59.265 | 1.00 | 53.94 | B | O |
| ATOM | 3275 | N | SER | B | 119 | -18.005 | 24.289 | 57.973 | 1.00 | 57.31 | B | N |
| ATOM | 3276 | CA | SER | B | 119 | -18.932 | 23.554 | 58.831 | 1.00 | 57.31 | B | C |

| | | | | | | | | | | | | |
|---|---|---|---|---|---|---|---|---|---|---|---|---|
| ATOM | 3277 | CB | SER | B | 119 | -20.176 | 24.411 | 59.102 | 1.00 | 96.33 | B | C |
| ATOM | 3278 | OG | SER | B | 119 | -19.833 | 25.595 | 59.818 | 1.00 | 96.33 | B | O |
| ATOM | 3279 | C | SER | B | 119 | -19.340 | 22.223 | 58.200 | 1.00 | 57.31 | B | C |
| ATOM | 3280 | O | SER | B | 119 | -19.233 | 21.169 | 58.829 | 1.00 | 57.31 | B | O |
| ATOM | 3281 | N | ALA | B | 125 | -18.780 | 21.720 | 65.100 | 1.00 | 95.31 | B | N |
| ATOM | 3282 | CA | ALA | B | 125 | -17.407 | 21.298 | 64.818 | 1.00 | 95.31 | B | C |
| ATOM | 3283 | CB | ALA | B | 125 | -17.373 | 19.799 | 64.509 | 1.00 | 41.56 | B | C |
| ATOM | 3284 | C | ALA | B | 125 | -16.822 | 22.099 | 63.647 | 1.00 | 95.31 | B | C |
| ATOM | 3285 | O | ALA | B | 125 | -17.570 | 22.602 | 62.793 | 1.00 | 95.31 | B | O |
| ATOM | 3286 | N | ALA | B | 126 | -15.493 | 22.221 | 63.608 | 1.00 | 64.13 | B | N |
| ATOM | 3287 | CA | ALA | B | 126 | -14.828 | 22.969 | 62.543 | 1.00 | 64.13 | B | C |
| ATOM | 3288 | CB | ALA | B | 126 | -14.119 | 24.189 | 63.133 | 1.00 | 29.48 | B | C |
| ATOM | 3289 | C | ALA | B | 126 | -13.831 | 22.084 | 61.786 | 1.00 | 64.13 | B | C |
| ATOM | 3290 | O | ALA | B | 126 | -12.924 | 21.508 | 62.389 | 1.00 | 64.13 | B | O |
| ATOM | 3291 | N | TYR | B | 127 | -14.004 | 21.973 | 60.467 | 1.00 | 48.62 | B | N |
| ATOM | 3292 | CA | TYR | B | 127 | -13.117 | 21.156 | 59.634 | 1.00 | 48.62 | B | C |
| ATOM | 3293 | CB | TYR | B | 127 | -13.888 | 20.446 | 58.518 | 1.00 | 77.40 | B | C |
| ATOM | 3294 | CG | TYR | B | 127 | -15.042 | 19.578 | 58.960 | 1.00 | 77.40 | B | C |
| ATOM | 3295 | CD1 | TYR | B | 127 | -16.156 | 20.137 | 59.590 | 1.00 | 77.40 | B | C |
| ATOM | 3296 | CE1 | TYR | B | 127 | -17.250 | 19.353 | 59.958 | 1.00 | 77.40 | B | C |
| ATOM | 3297 | CD2 | TYR | B | 127 | -15.042 | 18.201 | 58.707 | 1.00 | 77.40 | B | C |
| ATOM | 3298 | CE2 | TYR | B | 127 | -16.130 | 17.397 | 59.067 | 1.00 | 77.40 | B | C |
| ATOM | 3299 | CZ | TYR | B | 127 | -17.236 | 17.980 | 59.692 | 1.00 | 77.40 | B | C |
| ATOM | 3300 | OH | TYR | B | 127 | -18.333 | 17.207 | 60.049 | 1.00 | 77.40 | B | O |
| ATOM | 3301 | C | TYR | B | 127 | -12.071 | 22.033 | 58.971 | 1.00 | 48.62 | B | C |
| ATOM | 3302 | O | TYR | B | 127 | -12.370 | 23.144 | 58.541 | 1.00 | 48.62 | B | O |
| ATOM | 3303 | N | LEU | B | 128 | -10.847 | 21.525 | 58.884 | 1.00 | 51.66 | B | N |
| ATOM | 3304 | CA | LEU | B | 128 | -9.754 | 22.253 | 58.246 | 1.00 | 51.66 | B | C |
| ATOM | 3305 | CB | LEU | B | 128 | -8.422 | 21.924 | 58.922 | 1.00 | 42.01 | B | C |
| ATOM | 3306 | CG | LEU | B | 128 | -7.170 | 22.468 | 58.238 | 1.00 | 42.01 | B | C |
| ATOM | 3307 | CD1 | LEU | B | 128 | -7.214 | 23.989 | 58.213 | 1.00 | 42.01 | B | C |
| ATOM | 3308 | CD2 | LEU | B | 128 | -5.941 | 21.977 | 58.976 | 1.00 | 42.01 | B | C |
| ATOM | 3309 | C | LEU | B | 128 | -9.720 | 21.792 | 56.799 | 1.00 | 51.66 | B | C |
| ATOM | 3310 | O | LEU | B | 128 | -9.969 | 20.619 | 56.517 | 1.00 | 51.66 | B | O |
| ATOM | 3311 | N | ASN | B | 129 | -9.414 | 22.701 | 55.879 | 1.00 | 46.18 | B | N |
| ATOM | 3312 | CA | ASN | B | 129 | -9.380 | 22.341 | 54.466 | 1.00 | 46.18 | B | C |
| ATOM | 3313 | CB | ASN | B | 129 | -10.525 | 23.024 | 53.722 | 1.00 | 41.67 | B | C |
| ATOM | 3314 | CG | ASN | B | 129 | -11.884 | 22.573 | 54.212 | 1.00 | 41.67 | B | C |
| ATOM | 3315 | OD1 | ASN | B | 129 | -12.487 | 21.660 | 53.649 | 1.00 | 41.67 | B | O |
| ATOM | 3316 | ND2 | ASN | B | 129 | -12.370 | 23.201 | 55.282 | 1.00 | 41.67 | B | N |
| ATOM | 3317 | C | ASN | B | 129 | -8.063 | 22.725 | 53.835 | 1.00 | 46.18 | B | C |
| ATOM | 3318 | O | ASN | B | 129 | -7.715 | 23.902 | 53.753 | 1.00 | 46.18 | B | O |
| ATOM | 3319 | N | LEU | B | 130 | -7.328 | 21.716 | 53.392 | 1.00 | 34.33 | B | N |
| ATOM | 3320 | CA | LEU | B | 130 | -6.046 | 21.936 | 52.753 | 1.00 | 34.33 | B | C |
| ATOM | 3321 | CB | LEU | B | 130 | -5.055 | 20.860 | 53.204 | 1.00 | 40.79 | B | C |
| ATOM | 3322 | CG | LEU | B | 130 | -4.780 | 20.852 | 54.711 | 1.00 | 40.79 | B | C |
| ATOM | 3323 | CD1 | LEU | B | 130 | -4.064 | 19.587 | 55.128 | 1.00 | 40.79 | B | C |
| ATOM | 3324 | CD2 | LEU | B | 130 | -3.954 | 22.061 | 55.060 | 1.00 | 40.79 | B | C |
| ATOM | 3325 | C | LEU | B | 130 | -6.257 | 21.864 | 51.258 | 1.00 | 34.33 | B | C |
| ATOM | 3326 | O | LEU | B | 130 | -6.691 | 20.839 | 50.749 | 1.00 | 34.33 | B | O |
| ATOM | 3327 | N | VAL | B | 131 | -5.976 | 22.963 | 50.566 | 1.00 | 36.97 | B | N |
| ATOM | 3328 | CA | VAL | B | 131 | -6.128 | 23.018 | 49.114 | 1.00 | 36.97 | B | C |
| ATOM | 3329 | CB | VAL | B | 131 | -6.624 | 24.382 | 48.640 | 1.00 | 41.64 | B | C |
| ATOM | 3330 | CG1 | VAL | B | 131 | -6.966 | 24.313 | 47.157 | 1.00 | 41.64 | B | C |
| ATOM | 3331 | CG2 | VAL | B | 131 | -7.812 | 24.831 | 49.479 | 1.00 | 41.64 | B | C |
| ATOM | 3332 | C | VAL | B | 131 | -4.755 | 22.804 | 48.524 | 1.00 | 36.97 | B | C |
| ATOM | 3333 | O | VAL | B | 131 | -3.908 | 23.689 | 48.599 | 1.00 | 36.97 | B | O |
| ATOM | 3334 | N | LEU | B | 132 | -4.548 | 21.640 | 47.920 | 1.00 | 44.19 | B | N |
| ATOM | 3335 | CA | LEU | B | 132 | -3.255 | 21.279 | 47.354 | 1.00 | 44.19 | B | C |
| ATOM | 3336 | CB | LEU | B | 132 | -2.805 | 19.968 | 47.971 | 1.00 | 34.00 | B | C |
| ATOM | 3337 | CG | LEU | B | 132 | -3.000 | 19.992 | 49.480 | 1.00 | 34.00 | B | C |
| ATOM | 3338 | CD1 | LEU | B | 132 | -3.293 | 18.606 | 49.998 | 1.00 | 34.00 | B | C |
| ATOM | 3339 | CD2 | LEU | B | 132 | -1.765 | 20.595 | 50.123 | 1.00 | 34.00 | B | C |
| ATOM | 3340 | C | LEU | B | 132 | -3.253 | 21.128 | 45.847 | 1.00 | 44.19 | B | C |
| ATOM | 3341 | O | LEU | B | 132 | -4.286 | 20.843 | 45.241 | 1.00 | 44.19 | B | O |
| ATOM | 3342 | N | ASP | B | 133 | -2.082 | 21.317 | 45.246 | 1.00 | 41.24 | B | N |
| ATOM | 3343 | CA | ASP | B | 133 | -1.923 | 21.164 | 43.810 | 1.00 | 41.24 | B | C |

| | | | | | | | | | | | | |
|---|---|---|---|---|---|---|---|---|---|---|---|---|
| ATOM | 3344 | CB | ASP | B | 133 | -0.457 | 21.232 | 43.428 | 1.00 | 73.21 | B | C |
| ATOM | 3345 | CG | ASP | B | 133 | 0.102 | 22.638 | 43.489 | 1.00 | 73.21 | B | C |
| ATOM | 3346 | OD1 | ASP | B | 133 | 1.351 | 22.773 | 43.503 | 1.00 | 73.21 | B | O |
| ATOM | 3347 | OD2 | ASP | B | 133 | -0.699 | 23.607 | 43.504 | 1.00 | 73.21 | B | O |
| ATOM | 3348 | C | ASP | B | 133 | -2.423 | 19.777 | 43.488 | 1.00 | 41.24 | B | C |
| ATOM | 3349 | O | ASP | B | 133 | -2.282 | 18.864 | 44.296 | 1.00 | 41.24 | B | O |
| ATOM | 3350 | N | TYR | B | 134 | -3.012 | 19.608 | 42.313 | 1.00 | 48.00 | B | N |
| ATOM | 3351 | CA | TYR | B | 134 | -3.500 | 18.299 | 41.929 | 1.00 | 48.00 | B | C |
| ATOM | 3352 | CB | TYR | B | 134 | -4.805 | 18.404 | 41.158 | 1.00 | 57.10 | B | C |
| ATOM | 3353 | CG | TYR | B | 134 | -5.382 | 17.051 | 40.852 | 1.00 | 57.10 | B | C |
| ATOM | 3354 | CD1 | TYR | B | 134 | -5.912 | 16.257 | 41.875 | 1.00 | 57.10 | B | C |
| ATOM | 3355 | CE1 | TYR | B | 134 | -6.408 | 14.989 | 41.617 | 1.00 | 57.10 | B | C |
| ATOM | 3356 | CD2 | TYR | B | 134 | -5.363 | 16.538 | 39.554 | 1.00 | 57.10 | B | C |
| ATOM | 3357 | CE2 | TYR | B | 134 | -5.857 | 15.261 | 39.282 | 1.00 | 57.10 | B | C |
| ATOM | 3358 | CZ | TYR | B | 134 | -6.377 | 14.495 | 40.324 | 1.00 | 57.10 | B | C |
| ATOM | 3359 | OH | TYR | B | 134 | -6.849 | 13.226 | 40.092 | 1.00 | 57.10 | B | O |
| ATOM | 3360 | C | TYR | B | 134 | -2.473 | 17.653 | 41.034 | 1.00 | 48.00 | B | C |
| ATOM | 3361 | O | TYR | B | 134 | -1.890 | 18.313 | 40.183 | 1.00 | 48.00 | B | O |
| ATOM | 3362 | N | VAL | B | 135 | -2.231 | 16.370 | 41.248 | 1.00 | 34.92 | B | N |
| ATOM | 3363 | CA | VAL | B | 135 | -1.301 | 15.628 | 40.417 | 1.00 | 34.92 | B | C |
| ATOM | 3364 | CB | VAL | B | 135 | 0.055 | 15.447 | 41.093 | 1.00 | 40.08 | B | C |
| ATOM | 3365 | CG1 | VAL | B | 135 | 1.069 | 14.994 | 40.075 | 1.00 | 40.08 | B | C |
| ATOM | 3366 | CG2 | VAL | B | 135 | 0.504 | 16.754 | 41.715 | 1.00 | 40.08 | B | C |
| ATOM | 3367 | C | VAL | B | 135 | -2.023 | 14.306 | 40.273 | 1.00 | 34.92 | B | C |
| ATOM | 3368 | O | VAL | B | 135 | -2.368 | 13.672 | 41.269 | 1.00 | 34.92 | B | O |
| ATOM | 3369 | N | PRO | B | 136 | -2.289 | 13.889 | 39.022 | 1.00 | 51.38 | B | N |
| ATOM | 3370 | CD | PRO | B | 136 | -1.933 | 14.643 | 37.804 | 1.00 | 40.54 | B | C |
| ATOM | 3371 | CA | PRO | B | 136 | -2.995 | 12.650 | 38.671 | 1.00 | 51.38 | B | C |
| ATOM | 3372 | CB | PRO | B | 136 | -3.364 | 12.886 | 37.212 | 1.00 | 40.54 | B | C |
| ATOM | 3373 | CG | PRO | B | 136 | -2.170 | 13.628 | 36.708 | 1.00 | 40.54 | B | C |
| ATOM | 3374 | C | PRO | B | 136 | -2.302 | 11.306 | 38.889 | 1.00 | 51.38 | B | C |
| ATOM | 3375 | O | PRO | B | 136 | -2.909 | 10.364 | 39.399 | 1.00 | 51.38 | B | O |
| ATOM | 3376 | N | GLU | B | 137 | -1.043 | 11.198 | 38.504 | 1.00 | 37.55 | B | N |
| ATOM | 3377 | CA | GLU | B | 137 | -0.363 | 9.932 | 38.678 | 1.00 | 37.55 | B | C |
| ATOM | 3378 | CB | GLU | B | 137 | 0.634 | 9.705 | 37.550 | 1.00 | 57.24 | B | C |
| ATOM | 3379 | CG | GLU | B | 137 | 0.045 | 9.081 | 36.321 | 1.00 | 57.24 | B | C |
| ATOM | 3380 | CD | GLU | B | 137 | -0.597 | 7.741 | 36.605 | 1.00 | 57.24 | B | C |
| ATOM | 3381 | OE1 | GLU | B | 137 | -1.728 | 7.724 | 37.149 | 1.00 | 57.24 | B | O |
| ATOM | 3382 | OE2 | GLU | B | 137 | 0.032 | 6.701 | 36.289 | 1.00 | 57.24 | B | O |
| ATOM | 3383 | C | GLU | B | 137 | 0.355 | 9.778 | 40.000 | 1.00 | 37.55 | B | C |
| ATOM | 3384 | O | GLU | B | 137 | 0.635 | 10.755 | 40.694 | 1.00 | 37.55 | B | O |
| ATOM | 3385 | N | THR | B | 138 | 0.636 | 8.529 | 40.346 | 1.00 | 39.79 | B | N |
| ATOM | 3386 | CA | THR | B | 138 | 1.372 | 8.220 | 41.555 | 1.00 | 39.79 | B | C |
| ATOM | 3387 | CB | THR | B | 138 | 0.469 | 7.752 | 42.700 | 1.00 | 43.81 | B | C |
| ATOM | 3388 | OG1 | THR | B | 138 | -0.125 | 6.496 | 42.357 | 1.00 | 43.81 | B | O |
| ATOM | 3389 | CG2 | THR | B | 138 | -0.610 | 8.784 | 42.977 | 1.00 | 43.81 | B | C |
| ATOM | 3390 | C | THR | B | 138 | 2.280 | 7.087 | 41.157 | 1.00 | 39.79 | B | C |
| ATOM | 3391 | O | THR | B | 138 | 1.966 | 6.324 | 40.240 | 1.00 | 39.79 | B | O |
| ATOM | 3392 | N | VAL | B | 139 | 3.418 | 6.997 | 41.824 | 1.00 | 35.42 | B | N |
| ATOM | 3393 | CA | VAL | B | 139 | 4.350 | 5.939 | 41.523 | 1.00 | 35.42 | B | C |
| ATOM | 3394 | CB | VAL | B | 139 | 5.582 | 5.999 | 42.455 | 1.00 | 27.88 | B | C |
| ATOM | 3395 | CG1 | VAL | B | 139 | 6.442 | 4.751 | 42.275 | 1.00 | 27.88 | B | C |
| ATOM | 3396 | CG2 | VAL | B | 139 | 6.402 | 7.244 | 42.154 | 1.00 | 27.88 | B | C |
| ATOM | 3397 | C | VAL | B | 139 | 3.625 | 4.610 | 41.701 | 1.00 | 35.42 | B | C |
| ATOM | 3398 | O | VAL | B | 139 | 3.881 | 3.659 | 40.970 | 1.00 | 35.42 | B | O |
| ATOM | 3399 | N | TYR | B | 140 | 2.700 | 4.553 | 42.654 | 1.00 | 25.47 | B | N |
| ATOM | 3400 | CA | TYR | B | 140 | 1.963 | 3.317 | 42.899 | 1.00 | 25.47 | B | C |
| ATOM | 3401 | CB | TYR | B | 140 | 0.924 | 3.485 | 44.009 | 1.00 | 38.11 | B | C |
| ATOM | 3402 | CG | TYR | B | 140 | 0.154 | 2.211 | 44.249 | 1.00 | 38.11 | B | C |
| ATOM | 3403 | CD1 | TYR | B | 140 | 0.791 | 1.095 | 44.779 | 1.00 | 38.11 | B | C |
| ATOM | 3404 | CE1 | TYR | B | 140 | 0.136 | -0.118 | 44.901 | 1.00 | 38.11 | B | C |
| ATOM | 3405 | CD2 | TYR | B | 140 | -1.176 | 2.085 | 43.853 | 1.00 | 38.11 | B | C |
| ATOM | 3406 | CE2 | TYR | B | 140 | -1.845 | 0.871 | 43.970 | 1.00 | 38.11 | B | C |
| ATOM | 3407 | CZ | TYR | B | 140 | -1.175 | -0.230 | 44.494 | 1.00 | 38.11 | B | C |
| ATOM | 3408 | OH | TYR | B | 140 | -1.784 | -1.457 | 44.594 | 1.00 | 38.11 | B | O |
| ATOM | 3409 | C | TYR | B | 140 | 1.252 | 2.868 | 41.651 | 1.00 | 25.47 | B | C |
| ATOM | 3410 | O | TYR | B | 140 | 1.380 | 1.730 | 41.225 | 1.00 | 25.47 | B | O |

| | | | | | | | | | | | | |
|---|---|---|---|---|---|---|---|---|---|---|---|---|
| ATOM | 3411 | N | ARG | B | 141 | 0.483 | 3.780 | 41.081 | 1.00 | 40.11 | B | N |
| ATOM | 3412 | CA | ARG | B | 141 | -0.259 | 3.504 | 39.868 | 1.00 | 40.11 | B | C |
| ATOM | 3413 | CB | ARG | B | 141 | -1.073 | 4.734 | 39.473 | 1.00 | 66.47 | B | C |
| ATOM | 3414 | CG | ARG | B | 141 | -2.390 | 4.850 | 40.222 | 1.00 | 66.47 | B | C |
| ATOM | 3415 | CD | ARG | B | 141 | -2.912 | 6.274 | 40.250 | 1.00 | 66.47 | B | C |
| ATOM | 3416 | NE | ARG | B | 141 | -4.353 | 6.244 | 40.428 | 1.00 | 66.47 | B | N |
| ATOM | 3417 | CZ | ARG | B | 141 | -5.209 | 6.152 | 39.421 | 1.00 | 66.47 | B | C |
| ATOM | 3418 | NH1 | ARG | B | 141 | -4.745 | 6.100 | 38.174 | 1.00 | 66.47 | B | N |
| ATOM | 3419 | NH2 | ARG | B | 141 | -6.516 | 6.076 | 39.662 | 1.00 | 66.47 | B | N |
| ATOM | 3420 | C | ARG | B | 141 | 0.671 | 3.105 | 38.736 | 1.00 | 40.11 | B | C |
| ATOM | 3421 | O | ARG | B | 141 | 0.451 | 2.099 | 38.071 | 1.00 | 40.11 | B | O |
| ATOM | 3422 | N | VAL | B | 142 | 1.710 | 3.897 | 38.516 | 1.00 | 52.29 | B | N |
| ATOM | 3423 | CA | VAL | B | 142 | 2.662 | 3.598 | 37.456 | 1.00 | 52.29 | B | C |
| ATOM | 3424 | CB | VAL | B | 142 | 3.744 | 4.698 | 37.338 | 1.00 | 40.91 | B | C |
| ATOM | 3425 | CG1 | VAL | B | 142 | 4.890 | 4.214 | 36.487 | 1.00 | 40.91 | B | C |
| ATOM | 3426 | CG2 | VAL | B | 142 | 3.148 | 5.948 | 36.728 | 1.00 | 40.91 | B | C |
| ATOM | 3427 | C | VAL | B | 142 | 3.340 | 2.256 | 37.718 | 1.00 | 52.29 | B | C |
| ATOM | 3428 | O | VAL | B | 142 | 3.470 | 1.437 | 36.816 | 1.00 | 52.29 | B | O |
| ATOM | 3429 | N | ALA | B | 143 | 3.766 | 2.027 | 38.954 | 1.00 | 41.14 | B | N |
| ATOM | 3430 | CA | ALA | B | 143 | 4.437 | 0.783 | 39.297 | 1.00 | 41.14 | B | C |
| ATOM | 3431 | CB | ALA | B | 143 | 4.839 | 0.794 | 40.758 | 1.00 | 37.11 | B | C |
| ATOM | 3432 | C | ALA | B | 143 | 3.527 | -0.398 | 39.025 | 1.00 | 41.14 | B | C |
| ATOM | 3433 | O | ALA | B | 143 | 3.947 | -1.396 | 38.444 | 1.00 | 41.14 | B | O |
| ATOM | 3434 | N | ARG | B | 144 | 2.276 | -0.268 | 39.455 | 1.00 | 54.22 | B | N |
| ATOM | 3435 | CA | ARG | B | 144 | 1.273 | -1.315 | 39.291 | 1.00 | 54.22 | B | C |
| ATOM | 3436 | CB | ARG | B | 144 | -0.058 | -0.843 | 39.895 | 1.00 | 63.44 | B | C |
| ATOM | 3437 | CG | ARG | B | 144 | -1.024 | -1.954 | 40.287 | 1.00 | 63.44 | B | C |
| ATOM | 3438 | CD | ARG | B | 144 | -2.383 | -1.808 | 39.601 | 1.00 | 63.44 | B | C |
| ATOM | 3439 | NE | ARG | B | 144 | -3.199 | -0.686 | 40.078 | 1.00 | 63.44 | B | N |
| ATOM | 3440 | CZ | ARG | B | 144 | -3.850 | -0.652 | 41.247 | 1.00 | 63.44 | B | C |
| ATOM | 3441 | NH1 | ARG | B | 144 | -3.791 | -1.684 | 42.090 | 1.00 | 63.44 | B | N |
| ATOM | 3442 | NH2 | ARG | B | 144 | -4.579 | 0.417 | 41.571 | 1.00 | 63.44 | B | N |
| ATOM | 3443 | C | ARG | B | 144 | 1.096 | -1.619 | 37.805 | 1.00 | 54.22 | B | C |
| ATOM | 3444 | O | ARG | B | 144 | 1.088 | -2.776 | 37.386 | 1.00 | 54.22 | B | O |
| ATOM | 3445 | N | HIS | B | 145 | 0.978 | -0.560 | 37.012 | 1.00 | 54.25 | B | N |
| ATOM | 3446 | CA | HIS | B | 145 | 0.774 | -0.675 | 35.574 | 1.00 | 54.25 | B | C |
| ATOM | 3447 | CB | HIS | B | 145 | 0.657 | 0.730 | 34.970 | 1.00 | 72.56 | B | C |
| ATOM | 3448 | CG | HIS | B | 145 | 0.136 | 0.753 | 33.565 | 1.00 | 72.56 | B | C |
| ATOM | 3449 | CD2 | HIS | B | 145 | -0.040 | -0.242 | 32.661 | 1.00 | 72.56 | B | C |
| ATOM | 3450 | ND1 | HIS | B | 145 | -0.183 | 1.927 | 32.911 | 1.00 | 72.56 | B | N |
| ATOM | 3451 | CE1 | HIS | B | 145 | -0.524 | 1.654 | 31.663 | 1.00 | 72.56 | B | C |
| ATOM | 3452 | NE2 | HIS | B | 145 | -0.443 | 0.345 | 31.485 | 1.00 | 72.56 | B | N |
| ATOM | 3453 | C | HIS | B | 145 | 1.843 | -1.513 | 34.856 | 1.00 | 54.25 | B | C |
| ATOM | 3454 | O | HIS | B | 145 | 1.499 | -2.351 | 34.018 | 1.00 | 54.25 | B | O |
| ATOM | 3455 | N | TYR | B | 146 | 3.125 | -1.313 | 35.164 | 1.00 | 42.49 | B | N |
| ATOM | 3456 | CA | TYR | B | 146 | 4.162 | -2.124 | 34.514 | 1.00 | 42.49 | B | C |
| ATOM | 3457 | CB | TYR | B | 146 | 5.573 | -1.560 | 34.728 | 1.00 | 35.95 | B | C |
| ATOM | 3458 | CG | TYR | B | 146 | 5.885 | -0.312 | 33.949 | 1.00 | 35.95 | B | C |
| ATOM | 3459 | CD1 | TYR | B | 146 | 5.325 | 0.904 | 34.309 | 1.00 | 35.95 | B | C |
| ATOM | 3460 | CE1 | TYR | B | 146 | 5.578 | 2.056 | 33.578 | 1.00 | 35.95 | B | C |
| ATOM | 3461 | CD2 | TYR | B | 146 | 6.720 | -0.348 | 32.832 | 1.00 | 35.95 | B | C |
| ATOM | 3462 | CE2 | TYR | B | 146 | 6.983 | 0.805 | 32.092 | 1.00 | 35.95 | B | C |
| ATOM | 3463 | CZ | TYR | B | 146 | 6.402 | 2.002 | 32.473 | 1.00 | 35.95 | B | C |
| ATOM | 3464 | OH | TYR | B | 146 | 6.598 | 3.146 | 31.738 | 1.00 | 35.95 | B | O |
| ATOM | 3465 | C | TYR | B | 146 | 4.114 | -3.507 | 35.122 | 1.00 | 42.49 | B | C |
| ATOM | 3466 | O | TYR | B | 146 | 4.336 | -4.504 | 34.449 | 1.00 | 42.49 | B | O |
| ATOM | 3467 | N | SER | B | 147 | 3.804 | -3.558 | 36.407 | 1.00 | 61.05 | B | N |
| ATOM | 3468 | CA | SER | B | 147 | 3.757 | -4.823 | 37.131 | 1.00 | 61.05 | B | C |
| ATOM | 3469 | CB | SER | B | 147 | 3.610 | -4.554 | 38.629 | 1.00 | 54.68 | B | C |
| ATOM | 3470 | OG | SER | B | 147 | 3.640 | -5.773 | 39.341 | 1.00 | 54.68 | B | O |
| ATOM | 3471 | C | SER | B | 147 | 2.664 | -5.797 | 36.661 | 1.00 | 61.05 | B | C |
| ATOM | 3472 | O | SER | B | 147 | 2.909 | -6.994 | 36.540 | 1.00 | 61.05 | B | O |
| ATOM | 3473 | N | ARG | B | 148 | 1.464 | -5.291 | 36.400 | 1.00 | 60.49 | B | N |
| ATOM | 3474 | CA | ARG | B | 148 | 0.382 | -6.151 | 35.942 | 1.00 | 60.49 | B | C |
| ATOM | 3475 | CB | ARG | B | 148 | -0.941 | -5.395 | 35.914 | 1.00 | 70.48 | B | C |
| ATOM | 3476 | CG | ARG | B | 148 | -1.691 | -5.365 | 37.219 | 1.00 | 70.48 | B | C |
| ATOM | 3477 | CD | ARG | B | 148 | -2.921 | -4.507 | 37.036 | 1.00 | 70.48 | B | C |

| | | | | | | | | | | | | |
|---|---|---|---|---|---|---|---|---|---|---|---|---|
| ATOM | 3478 | NE | ARG | B | 148 | -3.673 | -4.323 | 38.275 | 1.00 | 70.48 | B | N |
| ATOM | 3479 | CZ | ARG | B | 148 | -4.552 | -3.339 | 38.457 | 1.00 | 70.48 | B | C |
| ATOM | 3480 | NH1 | ARG | B | 148 | -4.774 | -2.461 | 37.476 | 1.00 | 70.48 | B | N |
| ATOM | 3481 | NH2 | ARG | B | 148 | -5.196 | -3.222 | 39.617 | 1.00 | 70.48 | B | N |
| ATOM | 3482 | C | ARG | B | 148 | 0.657 | -6.681 | 34.544 | 1.00 | 60.49 | B | C |
| ATOM | 3483 | O | ARG | B | 148 | 0.191 | -7.767 | 34.183 | 1.00 | 60.49 | B | O |
| ATOM | 3484 | N | ALA | B | 149 | 1.394 | -5.903 | 33.752 | 1.00 | 49.92 | B | N |
| ATOM | 3485 | CA | ALA | B | 149 | 1.721 | -6.305 | 32.385 | 1.00 | 49.92 | B | C |
| ATOM | 3486 | CB | ALA | B | 149 | 1.873 | -5.078 | 31.485 | 1.00 | 29.98 | B | C |
| ATOM | 3487 | C | ALA | B | 149 | 3.003 | -7.114 | 32.372 | 1.00 | 49.92 | B | C |
| ATOM | 3488 | O | ALA | B | 149 | 3.685 | -7.179 | 31.351 | 1.00 | 49.92 | B | O |
| ATOM | 3489 | N | LYS | B | 150 | 3.330 | -7.718 | 33.513 | 1.00 | 48.86 | B | N |
| ATOM | 3490 | CA | LYS | B | 150 | 4.532 | -8.530 | 33.626 | 1.00 | 48.86 | B | C |
| ATOM | 3491 | CB | LYS | B | 150 | 4.281 | -9.886 | 32.956 | 1.00 | 63.94 | B | C |
| ATOM | 3492 | CG | LYS | B | 150 | 4.984 | -11.069 | 33.616 | 1.00 | 63.94 | B | C |
| ATOM | 3493 | CD | LYS | B | 150 | 4.243 | -11.588 | 34.866 | 1.00 | 63.94 | B | C |
| ATOM | 3494 | CE | LYS | B | 150 | 2.955 | -12.342 | 34.494 | 1.00 | 63.94 | B | C |
| ATOM | 3495 | NZ | LYS | B | 150 | 2.464 | -13.237 | 35.593 | 1.00 | 63.94 | B | N |
| ATOM | 3496 | C | LYS | B | 150 | 5.694 | -7.800 | 32.934 | 1.00 | 48.86 | B | C |
| ATOM | 3497 | O | LYS | B | 150 | 6.387 | -8.376 | 32.110 | 1.00 | 48.86 | B | O |
| ATOM | 3498 | N | GLN | B | 151 | 5.894 | -6.530 | 33.283 | 1.00 | 61.48 | B | N |
| ATOM | 3499 | CA | GLN | B | 151 | 6.937 | -5.673 | 32.691 | 1.00 | 61.48 | B | C |
| ATOM | 3500 | CB | GLN | B | 151 | 6.259 | -4.702 | 31.718 | 1.00 | 56.71 | B | C |
| ATOM | 3501 | CG | GLN | B | 151 | 7.183 | -3.801 | 30.936 | 1.00 | 56.71 | B | C |
| ATOM | 3502 | CD | GLN | B | 151 | 7.385 | -4.290 | 29.520 | 1.00 | 56.71 | B | C |
| ATOM | 3503 | OE1 | GLN | B | 151 | 6.419 | -4.487 | 28.777 | 1.00 | 56.71 | B | O |
| ATOM | 3504 | NE2 | GLN | B | 151 | 8.643 | -4.487 | 29.132 | 1.00 | 56.71 | B | N |
| ATOM | 3505 | C | GLN | B | 151 | 7.719 | -4.877 | 33.769 | 1.00 | 61.48 | B | C |
| ATOM | 3506 | O | GLN | B | 151 | 7.175 | -4.543 | 34.834 | 1.00 | 61.48 | B | O |
| ATOM | 3507 | N | THR | B | 152 | 8.982 | -4.559 | 33.503 | 1.00 | 41.09 | B | N |
| ATOM | 3508 | CA | THR | B | 152 | 9.770 | -3.812 | 34.486 | 1.00 | 41.09 | B | C |
| ATOM | 3509 | CB | THR | B | 152 | 11.203 | -4.426 | 34.662 | 1.00 | 50.96 | B | C |
| ATOM | 3510 | OG1 | THR | B | 152 | 12.196 | -3.467 | 34.274 | 1.00 | 50.96 | B | O |
| ATOM | 3511 | CG2 | THR | B | 152 | 11.364 | -5.683 | 33.809 | 1.00 | 50.96 | B | C |
| ATOM | 3512 | C | THR | B | 152 | 9.899 | -2.333 | 34.127 | 1.00 | 41.09 | B | C |
| ATOM | 3513 | O | THR | B | 152 | 10.154 | -1.988 | 32.978 | 1.00 | 41.09 | B | O |
| ATOM | 3514 | N | LEU | B | 153 | 9.718 | -1.457 | 35.105 | 1.00 | 34.47 | B | N |
| ATOM | 3515 | CA | LEU | B | 153 | 9.836 | -0.021 | 34.864 | 1.00 | 34.47 | B | C |
| ATOM | 3516 | CB | LEU | B | 153 | 9.490 | 0.753 | 36.140 | 1.00 | 49.31 | B | C |
| ATOM | 3517 | CG | LEU | B | 153 | 9.632 | 2.278 | 36.102 | 1.00 | 49.31 | B | C |
| ATOM | 3518 | CD1 | LEU | B | 153 | 8.424 | 2.870 | 35.405 | 1.00 | 49.31 | B | C |
| ATOM | 3519 | CD2 | LEU | B | 153 | 9.753 | 2.837 | 37.516 | 1.00 | 49.31 | B | C |
| ATOM | 3520 | C | LEU | B | 153 | 11.258 | 0.341 | 34.430 | 1.00 | 34.47 | B | C |
| ATOM | 3521 | O | LEU | B | 153 | 12.225 | -0.043 | 35.072 | 1.00 | 34.47 | B | O |
| ATOM | 3522 | N | PRO | B | 154 | 11.407 | 1.078 | 33.325 | 1.00 | 41.89 | B | N |
| ATOM | 3523 | CD | PRO | B | 154 | 10.379 | 1.509 | 32.360 | 1.00 | 32.15 | B | C |
| ATOM | 3524 | CA | PRO | B | 154 | 12.753 | 1.459 | 32.870 | 1.00 | 41.89 | B | C |
| ATOM | 3525 | CB | PRO | B | 154 | 12.455 | 2.383 | 31.690 | 1.00 | 32.15 | B | C |
| ATOM | 3526 | CG | PRO | B | 154 | 11.198 | 1.797 | 31.131 | 1.00 | 32.15 | B | C |
| ATOM | 3527 | C | PRO | B | 154 | 13.583 | 2.156 | 33.980 | 1.00 | 41.89 | B | C |
| ATOM | 3528 | O | PRO | B | 154 | 13.086 | 3.062 | 34.657 | 1.00 | 41.89 | B | O |
| ATOM | 3529 | N | VAL | B | 155 | 14.841 | 1.747 | 34.155 | 1.00 | 47.18 | B | N |
| ATOM | 3530 | CA | VAL | B | 155 | 15.690 | 2.341 | 35.186 | 1.00 | 47.18 | B | C |
| ATOM | 3531 | CB | VAL | B | 155 | 17.062 | 1.620 | 35.321 | 1.00 | 27.43 | B | C |
| ATOM | 3532 | CG1 | VAL | B | 155 | 16.852 | 0.184 | 35.723 | 1.00 | 27.43 | B | C |
| ATOM | 3533 | CG2 | VAL | B | 155 | 17.838 | 1.706 | 34.031 | 1.00 | 27.43 | B | C |
| ATOM | 3534 | C | VAL | B | 155 | 15.955 | 3.833 | 35.021 | 1.00 | 47.18 | B | C |
| ATOM | 3535 | O | VAL | B | 155 | 16.602 | 4.437 | 35.875 | 1.00 | 47.18 | B | O |
| ATOM | 3536 | N | ILE | B | 156 | 15.483 | 4.446 | 33.940 | 1.00 | 38.15 | B | N |
| ATOM | 3537 | CA | ILE | B | 156 | 15.704 | 5.883 | 33.799 | 1.00 | 38.15 | B | C |
| ATOM | 3538 | CB | ILE | B | 156 | 15.614 | 6.381 | 32.316 | 1.00 | 36.35 | B | C |
| ATOM | 3539 | CG2 | ILE | B | 156 | 14.254 | 6.101 | 31.731 | 1.00 | 36.35 | B | C |
| ATOM | 3540 | CG1 | ILE | B | 156 | 15.876 | 7.889 | 32.255 | 1.00 | 36.35 | B | C |
| ATOM | 3541 | CD1 | ILE | B | 156 | 17.263 | 8.282 | 32.692 | 1.00 | 36.35 | B | C |
| ATOM | 3542 | C | ILE | B | 156 | 14.632 | 6.555 | 34.637 | 1.00 | 38.15 | B | C |
| ATOM | 3543 | O | ILE | B | 156 | 14.842 | 7.630 | 35.182 | 1.00 | 38.15 | B | O |
| ATOM | 3544 | N | TYR | B | 157 | 13.481 | 5.907 | 34.745 | 1.00 | 32.23 | B | N |

| | | | | | | | | | | | | |
|---|---|---|---|---|---|---|---|---|---|---|---|---|
| ATOM | 3545 | CA | TYR | B | 157 | 12.402 | 6.454 | 35.546 | 1.00 | 32.23 | B | C |
| ATOM | 3546 | CB | TYR | B | 157 | 11.072 | 5.818 | 35.144 | 1.00 | 48.60 | B | C |
| ATOM | 3547 | CG | TYR | B | 157 | 10.528 | 6.357 | 33.845 | 1.00 | 48.60 | B | C |
| ATOM | 3548 | CD1 | TYR | B | 157 | 10.099 | 5.495 | 32.833 | 1.00 | 48.60 | B | C |
| ATOM | 3549 | CE1 | TYR | B | 157 | 9.605 | 5.991 | 31.622 | 1.00 | 48.60 | B | C |
| ATOM | 3550 | CD2 | TYR | B | 157 | 10.451 | 7.730 | 33.622 | 1.00 | 48.60 | B | C |
| ATOM | 3551 | CE2 | TYR | B | 157 | 9.964 | 8.240 | 32.424 | 1.00 | 48.60 | B | C |
| ATOM | 3552 | CZ | TYR | B | 157 | 9.540 | 7.368 | 31.423 | 1.00 | 48.60 | B | C |
| ATOM | 3553 | OH | TYR | B | 157 | 9.053 | 7.876 | 30.237 | 1.00 | 48.60 | B | O |
| ATOM | 3554 | C | TYR | B | 157 | 12.701 | 6.181 | 37.009 | 1.00 | 32.23 | B | C |
| ATOM | 3555 | O | TYR | B | 157 | 12.361 | 6.971 | 37.886 | 1.00 | 32.23 | B | O |
| ATOM | 3556 | N | VAL | B | 158 | 13.355 | 5.053 | 37.253 | 1.00 | 36.65 | B | N |
| ATOM | 3557 | CA | VAL | B | 158 | 13.724 | 4.656 | 38.594 | 1.00 | 36.65 | B | C |
| ATOM | 3558 | CB | VAL | B | 158 | 14.334 | 3.237 | 38.620 | 1.00 | 33.40 | B | C |
| ATOM | 3559 | CG1 | VAL | B | 158 | 14.697 | 2.852 | 40.044 | 1.00 | 33.40 | B | C |
| ATOM | 3560 | CG2 | VAL | B | 158 | 13.340 | 2.237 | 38.065 | 1.00 | 33.40 | B | C |
| ATOM | 3561 | C | VAL | B | 158 | 14.734 | 5.650 | 39.128 | 1.00 | 36.65 | B | C |
| ATOM | 3562 | O | VAL | B | 158 | 14.684 | 6.019 | 40.302 | 1.00 | 36.65 | B | O |
| ATOM | 3563 | N | LYS | B | 159 | 15.643 | 6.096 | 38.274 | 1.00 | 26.24 | B | N |
| ATOM | 3564 | CA | LYS | B | 159 | 16.629 | 7.067 | 38.712 | 1.00 | 26.24 | B | C |
| ATOM | 3565 | CB | LYS | B | 159 | 17.707 | 7.235 | 37.656 | 1.00 | 35.29 | B | C |
| ATOM | 3566 | CG | LYS | B | 159 | 18.547 | 6.006 | 37.432 | 1.00 | 35.29 | B | C |
| ATOM | 3567 | CD | LYS | B | 159 | 19.477 | 6.219 | 36.262 | 1.00 | 35.29 | B | C |
| ATOM | 3568 | CE | LYS | B | 159 | 20.348 | 5.011 | 36.006 | 1.00 | 35.29 | B | C |
| ATOM | 3569 | NZ | LYS | B | 159 | 21.421 | 5.364 | 35.036 | 1.00 | 35.29 | B | N |
| ATOM | 3570 | C | LYS | B | 159 | 15.962 | 8.414 | 38.984 | 1.00 | 26.24 | B | C |
| ATOM | 3571 | O | LYS | B | 159 | 16.194 | 9.052 | 40.010 | 1.00 | 26.24 | B | O |
| ATOM | 3572 | N | LEU | B | 160 | 15.120 | 8.837 | 38.053 | 1.00 | 35.96 | B | N |
| ATOM | 3573 | CA | LEU | B | 160 | 14.411 | 10.099 | 38.168 | 1.00 | 35.96 | B | C |
| ATOM | 3574 | CB | LEU | B | 160 | 13.547 | 10.328 | 36.934 | 1.00 | 36.98 | B | C |
| ATOM | 3575 | CG | LEU | B | 160 | 14.327 | 10.878 | 35.755 | 1.00 | 36.98 | B | C |
| ATOM | 3576 | CD1 | LEU | B | 160 | 13.487 | 10.780 | 34.500 | 1.00 | 36.98 | B | C |
| ATOM | 3577 | CD2 | LEU | B | 160 | 14.740 | 12.317 | 36.062 | 1.00 | 36.98 | B | C |
| ATOM | 3578 | C | LEU | B | 160 | 13.538 | 10.217 | 39.396 | 1.00 | 35.96 | B | C |
| ATOM | 3579 | O | LEU | B | 160 | 13.583 | 11.221 | 40.090 | 1.00 | 35.96 | B | O |
| ATOM | 3580 | N | TYR | B | 161 | 12.730 | 9.202 | 39.659 | 1.00 | 33.69 | B | N |
| ATOM | 3581 | CA | TYR | B | 161 | 11.846 | 9.258 | 40.805 | 1.00 | 33.69 | B | C |
| ATOM | 3582 | CB | TYR | B | 161 | 10.839 | 8.116 | 40.756 | 1.00 | 54.12 | B | C |
| ATOM | 3583 | CG | TYR | B | 161 | 10.008 | 8.129 | 39.500 | 1.00 | 54.12 | B | C |
| ATOM | 3584 | CD1 | TYR | B | 161 | 9.920 | 9.279 | 38.719 | 1.00 | 54.12 | B | C |
| ATOM | 3585 | CE1 | TYR | B | 161 | 9.168 | 9.304 | 37.570 | 1.00 | 54.12 | B | C |
| ATOM | 3586 | CD2 | TYR | B | 161 | 9.312 | 6.998 | 39.090 | 1.00 | 54.12 | B | C |
| ATOM | 3587 | CE2 | TYR | B | 161 | 8.554 | 7.012 | 37.940 | 1.00 | 54.12 | B | C |
| ATOM | 3588 | CZ | TYR | B | 161 | 8.485 | 8.168 | 37.187 | 1.00 | 54.12 | B | C |
| ATOM | 3589 | OH | TYR | B | 161 | 7.703 | 8.206 | 36.057 | 1.00 | 54.12 | B | O |
| ATOM | 3590 | C | TYR | B | 161 | 12.584 | 9.230 | 42.119 | 1.00 | 33.69 | B | C |
| ATOM | 3591 | O | TYR | B | 161 | 12.407 | 10.119 | 42.957 | 1.00 | 33.69 | B | O |
| ATOM | 3592 | N | MET | B | 162 | 13.411 | 8.208 | 42.301 | 1.00 | 44.12 | B | N |
| ATOM | 3593 | CA | MET | B | 162 | 14.176 | 8.084 | 43.530 | 1.00 | 44.12 | B | C |
| ATOM | 3594 | CB | MET | B | 162 | 15.092 | 6.871 | 43.464 | 1.00 | 36.83 | B | C |
| ATOM | 3595 | CG | MET | B | 162 | 14.337 | 5.557 | 43.417 | 1.00 | 36.83 | B | C |
| ATOM | 3596 | SD | MET | B | 162 | 13.078 | 5.433 | 44.697 | 1.00 | 36.83 | B | S |
| ATOM | 3597 | CE | MET | B | 162 | 14.046 | 5.662 | 46.165 | 1.00 | 36.83 | B | C |
| ATOM | 3598 | C | MET | B | 162 | 14.984 | 9.344 | 43.795 | 1.00 | 44.12 | B | C |
| ATOM | 3599 | O | MET | B | 162 | 14.980 | 9.851 | 44.909 | 1.00 | 44.12 | B | O |
| ATOM | 3600 | N | TYR | B | 163 | 15.662 | 9.859 | 42.773 | 1.00 | 31.39 | B | N |
| ATOM | 3601 | CA | TYR | B | 163 | 16.444 | 11.070 | 42.941 | 1.00 | 31.39 | B | C |
| ATOM | 3602 | CB | TYR | B | 163 | 17.048 | 11.512 | 41.625 | 1.00 | 40.37 | B | C |
| ATOM | 3603 | CG | TYR | B | 163 | 17.880 | 12.764 | 41.776 | 1.00 | 40.37 | B | C |
| ATOM | 3604 | CD1 | TYR | B | 163 | 19.210 | 12.696 | 42.189 | 1.00 | 40.37 | B | C |
| ATOM | 3605 | CE1 | TYR | B | 163 | 19.978 | 13.839 | 42.325 | 1.00 | 40.37 | B | C |
| ATOM | 3606 | CD2 | TYR | B | 163 | 17.338 | 14.018 | 41.512 | 1.00 | 40.37 | B | C |
| ATOM | 3607 | CE2 | TYR | B | 163 | 18.101 | 15.175 | 41.648 | 1.00 | 40.37 | B | C |
| ATOM | 3608 | CZ | TYR | B | 163 | 19.417 | 15.075 | 42.049 | 1.00 | 40.37 | B | C |
| ATOM | 3609 | OH | TYR | B | 163 | 20.180 | 16.215 | 42.136 | 1.00 | 40.37 | B | O |
| ATOM | 3610 | C | TYR | B | 163 | 15.616 | 12.224 | 43.484 | 1.00 | 31.39 | B | C |
| ATOM | 3611 | O | TYR | B | 163 | 16.035 | 12.921 | 44.405 | 1.00 | 31.39 | B | O |

| | | | | | | | | | | | | |
|---|---|---|---|---|---|---|---|---|---|---|---|---|
| ATOM | 3612 | N | GLN | B | 164 | 14.446 | 12.442 | 42.896 | 1.00 | 38.31 | B | N |
| ATOM | 3613 | CA | GLN | B | 164 | 13.576 | 13.517 | 43.342 | 1.00 | 38.31 | B | C |
| ATOM | 3614 | CB | GLN | B | 164 | 12.444 | 13.721 | 42.343 | 1.00 | 42.66 | B | C |
| ATOM | 3615 | CG | GLN | B | 164 | 12.924 | 14.114 | 40.959 | 1.00 | 42.66 | B | C |
| ATOM | 3616 | CD | GLN | B | 164 | 11.773 | 14.395 | 40.007 | 1.00 | 42.66 | B | C |
| ATOM | 3617 | OE1 | GLN | B | 164 | 11.193 | 15.490 | 40.008 | 1.00 | 42.66 | B | O |
| ATOM | 3618 | NE2 | GLN | B | 164 | 11.423 | 13.397 | 39.198 | 1.00 | 42.66 | B | N |
| ATOM | 3619 | C | GLN | B | 164 | 13.022 | 13.228 | 44.740 | 1.00 | 38.31 | B | C |
| ATOM | 3620 | O | GLN | B | 164 | 12.877 | 14.136 | 45.554 | 1.00 | 38.31 | B | O |
| ATOM | 3621 | N | LEU | B | 165 | 12.713 | 11.967 | 45.025 | 1.00 | 36.88 | B | N |
| ATOM | 3622 | CA | LEU | B | 165 | 12.206 | 11.617 | 46.343 | 1.00 | 36.88 | B | C |
| ATOM | 3623 | CB | LEU | B | 165 | 11.825 | 10.137 | 46.414 | 1.00 | 30.94 | B | C |
| ATOM | 3624 | CG | LEU | B | 165 | 11.815 | 9.527 | 47.821 | 1.00 | 30.94 | B | C |
| ATOM | 3625 | CD1 | LEU | B | 165 | 10.897 | 10.305 | 48.731 | 1.00 | 30.94 | B | C |
| ATOM | 3626 | CD2 | LEU | B | 165 | 11.385 | 8.087 | 47.746 | 1.00 | 30.94 | B | C |
| ATOM | 3627 | C | LEU | B | 165 | 13.295 | 11.906 | 47.353 | 1.00 | 36.88 | B | C |
| ATOM | 3628 | O | LEU | B | 165 | 13.034 | 12.463 | 48.415 | 1.00 | 36.88 | B | O |
| ATOM | 3629 | N | PHE | B | 166 | 14.523 | 11.517 | 47.024 | 1.00 | 47.92 | B | N |
| ATOM | 3630 | CA | PHE | B | 166 | 15.641 | 11.750 | 47.924 | 1.00 | 47.92 | B | C |
| ATOM | 3631 | CB | PHE | B | 166 | 16.914 | 11.079 | 47.413 | 1.00 | 24.84 | B | C |
| ATOM | 3632 | CG | PHE | B | 166 | 17.056 | 9.654 | 47.863 | 1.00 | 24.84 | B | C |
| ATOM | 3633 | CD1 | PHE | B | 166 | 16.909 | 9.323 | 49.213 | 1.00 | 24.84 | B | C |
| ATOM | 3634 | CD2 | PHE | B | 166 | 17.309 | 8.641 | 46.951 | 1.00 | 24.84 | B | C |
| ATOM | 3635 | CE1 | PHE | B | 166 | 17.007 | 8.002 | 49.645 | 1.00 | 24.84 | B | C |
| ATOM | 3636 | CE2 | PHE | B | 166 | 17.409 | 7.325 | 47.373 | 1.00 | 24.84 | B | C |
| ATOM | 3637 | CZ | PHE | B | 166 | 17.255 | 7.005 | 48.727 | 1.00 | 24.84 | B | C |
| ATOM | 3638 | C | PHE | B | 166 | 15.880 | 13.228 | 48.108 | 1.00 | 47.92 | B | C |
| ATOM | 3639 | O | PHE | B | 166 | 16.198 | 13.681 | 49.209 | 1.00 | 47.92 | B | O |
| ATOM | 3640 | N | ARG | B | 167 | 15.710 | 13.995 | 47.040 | 1.00 | 39.10 | B | N |
| ATOM | 3641 | CA | ARG | B | 167 | 15.926 | 15.417 | 47.166 | 1.00 | 39.10 | B | C |
| ATOM | 3642 | CB | ARG | B | 167 | 15.878 | 16.097 | 45.794 | 1.00 | 31.17 | B | C |
| ATOM | 3643 | CG | ARG | B | 167 | 16.402 | 17.513 | 45.863 | 1.00 | 31.17 | B | C |
| ATOM | 3644 | CD | ARG | B | 167 | 16.415 | 18.220 | 44.554 | 1.00 | 31.17 | B | C |
| ATOM | 3645 | NE | ARG | B | 167 | 16.580 | 19.648 | 44.782 | 1.00 | 31.17 | B | N |
| ATOM | 3646 | CZ | ARG | B | 167 | 16.482 | 20.575 | 43.837 | 1.00 | 31.17 | B | C |
| ATOM | 3647 | NH1 | ARG | B | 167 | 16.219 | 20.232 | 42.582 | 1.00 | 31.17 | B | N |
| ATOM | 3648 | NH2 | ARG | B | 167 | 16.636 | 21.849 | 44.151 | 1.00 | 31.17 | B | N |
| ATOM | 3649 | C | ARG | B | 167 | 14.901 | 16.042 | 48.118 | 1.00 | 39.10 | B | C |
| ATOM | 3650 | O | ARG | B | 167 | 15.247 | 16.885 | 48.944 | 1.00 | 39.10 | B | O |
| ATOM | 3651 | N | SER | B | 168 | 13.649 | 15.609 | 48.019 | 1.00 | 33.04 | B | N |
| ATOM | 3652 | CA | SER | B | 168 | 12.589 | 16.146 | 48.864 | 1.00 | 33.04 | B | C |
| ATOM | 3653 | CB | SER | B | 168 | 11.226 | 15.606 | 48.421 | 1.00 | 36.87 | B | C |
| ATOM | 3654 | OG | SER | B | 168 | 11.014 | 14.289 | 48.895 | 1.00 | 36.87 | B | O |
| ATOM | 3655 | C | SER | B | 168 | 12.808 | 15.805 | 50.333 | 1.00 | 33.04 | B | C |
| ATOM | 3656 | O | SER | B | 168 | 12.531 | 16.612 | 51.221 | 1.00 | 33.04 | B | O |
| ATOM | 3657 | N | LEU | B | 169 | 13.287 | 14.594 | 50.584 | 1.00 | 44.38 | B | N |
| ATOM | 3658 | CA | LEU | B | 169 | 13.537 | 14.154 | 51.945 | 1.00 | 44.38 | B | C |
| ATOM | 3659 | CB | LEU | B | 169 | 13.934 | 12.683 | 51.950 | 1.00 | 29.63 | B | C |
| ATOM | 3660 | CG | LEU | B | 169 | 12.926 | 11.705 | 52.548 | 1.00 | 29.63 | B | C |
| ATOM | 3661 | CD1 | LEU | B | 169 | 11.517 | 12.107 | 52.204 | 1.00 | 29.63 | B | C |
| ATOM | 3662 | CD2 | LEU | B | 169 | 13.228 | 10.317 | 52.038 | 1.00 | 29.63 | B | C |
| ATOM | 3663 | C | LEU | B | 169 | 14.653 | 15.017 | 52.508 | 1.00 | 44.38 | B | C |
| ATOM | 3664 | O | LEU | B | 169 | 14.570 | 15.511 | 53.638 | 1.00 | 44.38 | B | O |
| ATOM | 3665 | N | ALA | B | 170 | 15.689 | 15.216 | 51.703 | 1.00 | 38.87 | B | N |
| ATOM | 3666 | CA | ALA | B | 170 | 16.814 | 16.031 | 52.114 | 1.00 | 38.87 | B | C |
| ATOM | 3667 | CB | ALA | B | 170 | 17.771 | 16.200 | 50.961 | 1.00 | 26.62 | B | C |
| ATOM | 3668 | C | ALA | B | 170 | 16.277 | 17.382 | 52.559 | 1.00 | 38.87 | B | C |
| ATOM | 3669 | O | ALA | B | 170 | 16.752 | 17.976 | 53.522 | 1.00 | 38.87 | B | O |
| ATOM | 3670 | N | TYR | B | 171 | 15.264 | 17.860 | 51.855 | 1.00 | 46.13 | B | N |
| ATOM | 3671 | CA | TYR | B | 171 | 14.665 | 19.143 | 52.173 | 1.00 | 46.13 | B | C |
| ATOM | 3672 | CB | TYR | B | 171 | 13.667 | 19.523 | 51.093 | 1.00 | 43.64 | B | C |
| ATOM | 3673 | CG | TYR | B | 171 | 12.989 | 20.827 | 51.378 | 1.00 | 43.64 | B | C |
| ATOM | 3674 | CD1 | TYR | B | 171 | 13.719 | 22.012 | 51.415 | 1.00 | 43.64 | B | C |
| ATOM | 3675 | CE1 | TYR | B | 171 | 13.106 | 23.219 | 51.691 | 1.00 | 43.64 | B | C |
| ATOM | 3676 | CD2 | TYR | B | 171 | 11.622 | 20.882 | 51.629 | 1.00 | 43.64 | B | C |
| ATOM | 3677 | CE2 | TYR | B | 171 | 10.998 | 22.090 | 51.910 | 1.00 | 43.64 | B | C |
| ATOM | 3678 | CZ | TYR | B | 171 | 11.749 | 23.253 | 51.937 | 1.00 | 43.64 | B | C |

| | | | | | | | | | | | | |
|---|---|---|---|---|---|---|---|---|---|---|---|---|
| ATOM | 3679 | OH | TYR | B | 171 | 11.151 | 24.455 | 52.207 | 1.00 | 43.64 | B | O |
| ATOM | 3680 | C | TYR | B | 171 | 13.966 | 19.181 | 53.529 | 1.00 | 46.13 | B | C |
| ATOM | 3681 | O | TYR | B | 171 | 14.407 | 19.874 | 54.445 | 1.00 | 46.13 | B | O |
| ATOM | 3682 | N | ILE | B | 172 | 12.867 | 18.445 | 53.654 | 1.00 | 45.00 | B | N |
| ATOM | 3683 | CA | ILE | B | 172 | 12.125 | 18.443 | 54.902 | 1.00 | 45.00 | B | C |
| ATOM | 3684 | CB | ILE | B | 172 | 10.881 | 17.533 | 54.835 | 1.00 | 31.79 | B | C |
| ATOM | 3685 | CG2 | ILE | B | 172 | 9.927 | 18.055 | 53.796 | 1.00 | 31.79 | B | C |
| ATOM | 3686 | CG1 | ILE | B | 172 | 11.295 | 16.092 | 54.545 | 1.00 | 31.79 | B | C |
| ATOM | 3687 | CD1 | ILE | B | 172 | 10.148 | 15.113 | 54.593 | 1.00 | 31.79 | B | C |
| ATOM | 3688 | C | ILE | B | 172 | 12.999 | 18.003 | 56.069 | 1.00 | 45.00 | B | C |
| ATOM | 3689 | O | ILE | B | 172 | 12.797 | 18.437 | 57.208 | 1.00 | 45.00 | B | O |
| ATOM | 3690 | N | HIS | B | 173 | 13.976 | 17.148 | 55.794 | 1.00 | 49.21 | B | N |
| ATOM | 3691 | CA | HIS | B | 173 | 14.837 | 16.694 | 56.868 | 1.00 | 49.21 | B | C |
| ATOM | 3692 | CB | HIS | B | 173 | 15.699 | 15.518 | 56.418 | 1.00 | 29.72 | B | C |
| ATOM | 3693 | CG | HIS | B | 173 | 14.954 | 14.222 | 56.384 | 1.00 | 29.72 | B | C |
| ATOM | 3694 | CD2 | HIS | B | 173 | 13.642 | 13.945 | 56.582 | 1.00 | 29.72 | B | C |
| ATOM | 3695 | ND1 | HIS | B | 173 | 15.568 | 13.014 | 56.145 | 1.00 | 29.72 | B | N |
| ATOM | 3696 | CE1 | HIS | B | 173 | 14.668 | 12.049 | 56.202 | 1.00 | 29.72 | B | C |
| ATOM | 3697 | NE2 | HIS | B | 173 | 13.492 | 12.587 | 56.466 | 1.00 | 29.72 | B | N |
| ATOM | 3698 | C | HIS | B | 173 | 15.705 | 17.794 | 57.445 | 1.00 | 49.21 | B | C |
| ATOM | 3699 | O | HIS | B | 173 | 16.105 | 17.707 | 58.606 | 1.00 | 49.21 | B | O |
| ATOM | 3700 | N | SER | B | 174 | 15.977 | 18.839 | 56.662 | 1.00 | 49.11 | B | N |
| ATOM | 3701 | CA | SER | B | 174 | 16.803 | 19.937 | 57.160 | 1.00 | 49.11 | B | C |
| ATOM | 3702 | CB | SER | B | 174 | 17.337 | 20.791 | 56.006 | 1.00 | 45.05 | B | C |
| ATOM | 3703 | OG | SER | B | 174 | 16.293 | 21.454 | 55.329 | 1.00 | 45.05 | B | O |
| ATOM | 3704 | C | SER | B | 174 | 16.081 | 20.828 | 58.176 | 1.00 | 49.11 | B | C |
| ATOM | 3705 | O | SER | B | 174 | 16.712 | 21.660 | 58.821 | 1.00 | 49.11 | B | O |
| ATOM | 3706 | N | PHE | B | 175 | 14.767 | 20.671 | 58.309 | 1.00 | 39.55 | B | N |
| ATOM | 3707 | CA | PHE | B | 175 | 14.016 | 21.446 | 59.289 | 1.00 | 39.55 | B | C |
| ATOM | 3708 | CB | PHE | B | 175 | 12.714 | 21.987 | 58.712 | 1.00 | 84.91 | B | C |
| ATOM | 3709 | CG | PHE | B | 175 | 12.897 | 22.928 | 57.566 | 1.00 | 84.91 | B | C |
| ATOM | 3710 | CD1 | PHE | B | 175 | 13.028 | 22.442 | 56.264 | 1.00 | 84.91 | B | C |
| ATOM | 3711 | CD2 | PHE | B | 175 | 12.938 | 24.305 | 57.783 | 1.00 | 84.91 | B | C |
| ATOM | 3712 | CE1 | PHE | B | 175 | 13.198 | 23.319 | 55.181 | 1.00 | 84.91 | B | C |
| ATOM | 3713 | CE2 | PHE | B | 175 | 13.107 | 25.194 | 56.713 | 1.00 | 84.91 | B | C |
| ATOM | 3714 | CZ | PHE | B | 175 | 13.237 | 24.700 | 55.404 | 1.00 | 84.91 | B | C |
| ATOM | 3715 | C | PHE | B | 175 | 13.664 | 20.500 | 60.419 | 1.00 | 39.55 | B | C |
| ATOM | 3716 | O | PHE | B | 175 | 12.823 | 20.815 | 61.271 | 1.00 | 39.55 | B | O |
| ATOM | 3717 | N | GLY | B | 176 | 14.298 | 19.328 | 60.409 | 1.00 | 51.81 | B | N |
| ATOM | 3718 | CA | GLY | B | 176 | 14.039 | 18.333 | 61.433 | 1.00 | 51.81 | B | C |
| ATOM | 3719 | C | GLY | B | 176 | 12.661 | 17.720 | 61.292 | 1.00 | 51.81 | B | C |
| ATOM | 3720 | O | GLY | B | 176 | 12.076 | 17.246 | 62.267 | 1.00 | 51.81 | B | O |
| ATOM | 3721 | N | ILE | B | 177 | 12.135 | 17.726 | 60.074 | 1.00 | 47.81 | B | N |
| ATOM | 3722 | CA | ILE | B | 177 | 10.824 | 17.156 | 59.813 | 1.00 | 47.81 | B | C |
| ATOM | 3723 | CB | ILE | B | 177 | 10.017 | 18.076 | 58.910 | 1.00 | 56.88 | B | C |
| ATOM | 3724 | CG2 | ILE | B | 177 | 8.729 | 17.386 | 58.490 | 1.00 | 56.88 | B | C |
| ATOM | 3725 | CG1 | ILE | B | 177 | 9.743 | 19.386 | 59.644 | 1.00 | 56.88 | B | C |
| ATOM | 3726 | CD1 | ILE | B | 177 | 9.290 | 20.516 | 58.727 | 1.00 | 56.88 | B | C |
| ATOM | 3727 | C | ILE | B | 177 | 10.959 | 15.791 | 59.146 | 1.00 | 47.81 | B | C |
| ATOM | 3728 | O | ILE | B | 177 | 11.678 | 15.645 | 58.156 | 1.00 | 47.81 | B | O |
| ATOM | 3729 | N | CYS | B | 178 | 10.259 | 14.802 | 59.695 | 1.00 | 29.70 | B | N |
| ATOM | 3730 | CA | CYS | B | 178 | 10.293 | 13.437 | 59.179 | 1.00 | 29.70 | B | C |
| ATOM | 3731 | CB | CYS | B | 178 | 10.711 | 12.483 | 60.298 | 1.00 | 46.01 | B | C |
| ATOM | 3732 | SG | CYS | B | 178 | 10.808 | 10.740 | 59.852 | 1.00 | 46.01 | B | S |
| ATOM | 3733 | C | CYS | B | 178 | 8.931 | 13.034 | 58.633 | 1.00 | 29.70 | B | C |
| ATOM | 3734 | O | CYS | B | 178 | 7.935 | 13.055 | 59.346 | 1.00 | 29.70 | B | O |
| ATOM | 3735 | N | HIS | B | 179 | 8.893 | 12.654 | 57.364 | 1.00 | 32.68 | B | N |
| ATOM | 3736 | CA | HIS | B | 179 | 7.647 | 12.265 | 56.731 | 1.00 | 32.68 | B | C |
| ATOM | 3737 | CB | HIS | B | 179 | 7.913 | 11.929 | 55.266 | 1.00 | 35.24 | B | C |
| ATOM | 3738 | CG | HIS | B | 179 | 6.668 | 11.781 | 54.455 | 1.00 | 35.24 | B | C |
| ATOM | 3739 | CD2 | HIS | B | 179 | 5.937 | 12.697 | 53.779 | 1.00 | 35.24 | B | C |
| ATOM | 3740 | ND1 | HIS | B | 179 | 5.970 | 10.597 | 54.378 | 1.00 | 35.24 | B | N |
| ATOM | 3741 | CE1 | HIS | B | 179 | 4.856 | 10.794 | 53.696 | 1.00 | 35.24 | B | C |
| ATOM | 3742 | NE2 | HIS | B | 179 | 4.813 | 12.059 | 53.324 | 1.00 | 35.24 | B | N |
| ATOM | 3743 | C | HIS | B | 179 | 6.936 | 11.101 | 57.436 | 1.00 | 32.68 | B | C |
| ATOM | 3744 | O | HIS | B | 179 | 5.723 | 11.131 | 57.622 | 1.00 | 32.68 | B | O |
| ATOM | 3745 | N | ARG | B | 180 | 7.697 | 10.080 | 57.817 | 1.00 | 29.37 | B | N |

| | | | | | | | | | | | | |
|---|---|---|---|---|---|---|---|---|---|---|---|---|
| ATOM | 3746 | CA | ARG | B | 180 | 7.165 | 8.917 | 58.523 | 1.00 | 29.37 | B | C |
| ATOM | 3747 | CB | ARG | B | 180 | 6.643 | 9.336 | 59.899 | 1.00 | 35.88 | B | C |
| ATOM | 3748 | CG | ARG | B | 180 | 7.675 | 9.967 | 60.792 | 1.00 | 35.88 | B | C |
| ATOM | 3749 | CD | ARG | B | 180 | 6.982 | 10.797 | 61.843 | 1.00 | 35.88 | B | C |
| ATOM | 3750 | NE | ARG | B | 180 | 6.321 | 9.990 | 62.861 | 1.00 | 35.88 | B | N |
| ATOM | 3751 | CZ | ARG | B | 180 | 5.417 | 10.461 | 63.715 | 1.00 | 35.88 | B | C |
| ATOM | 3752 | NH1 | ARG | B | 180 | 5.049 | 11.730 | 63.680 | 1.00 | 35.88 | B | N |
| ATOM | 3753 | NH2 | ARG | B | 180 | 4.896 | 9.661 | 64.626 | 1.00 | 35.88 | B | N |
| ATOM | 3754 | C | ARG | B | 180 | 6.077 | 8.133 | 57.794 | 1.00 | 29.37 | B | C |
| ATOM | 3755 | O | ARG | B | 180 | 5.392 | 7.310 | 58.400 | 1.00 | 29.37 | B | O |
| ATOM | 3756 | N | ASP | B | 181 | 5.904 | 8.394 | 56.505 | 1.00 | 35.63 | B | N |
| ATOM | 3757 | CA | ASP | B | 181 | 4.920 | 7.662 | 55.721 | 1.00 | 35.63 | B | C |
| ATOM | 3758 | CB | ASP | B | 181 | 3.535 | 8.265 | 55.898 | 1.00 | 42.67 | B | C |
| ATOM | 3759 | CG | ASP | B | 181 | 2.451 | 7.392 | 55.324 | 1.00 | 42.67 | B | C |
| ATOM | 3760 | OD1 | ASP | B | 181 | 2.717 | 6.191 | 55.116 | 1.00 | 42.67 | B | O |
| ATOM | 3761 | OD2 | ASP | B | 181 | 1.330 | 7.896 | 55.095 | 1.00 | 42.67 | B | O |
| ATOM | 3762 | C | ASP | B | 181 | 5.281 | 7.598 | 54.240 | 1.00 | 35.63 | B | C |
| ATOM | 3763 | O | ASP | B | 181 | 4.414 | 7.693 | 53.379 | 1.00 | 35.63 | B | O |
| ATOM | 3764 | N | ILE | B | 182 | 6.568 | 7.430 | 53.949 | 1.00 | 37.05 | B | N |
| ATOM | 3765 | CA | ILE | B | 182 | 7.032 | 7.331 | 52.573 | 1.00 | 37.05 | B | C |
| ATOM | 3766 | CB | ILE | B | 182 | 8.578 | 7.375 | 52.483 | 1.00 | 30.77 | B | C |
| ATOM | 3767 | CG2 | ILE | B | 182 | 9.027 | 7.275 | 51.035 | 1.00 | 30.77 | B | C |
| ATOM | 3768 | CG1 | ILE | B | 182 | 9.102 | 8.664 | 53.110 | 1.00 | 30.77 | B | C |
| ATOM | 3769 | CD1 | ILE | B | 182 | 8.641 | 9.929 | 52.418 | 1.00 | 30.77 | B | C |
| ATOM | 3770 | C | ILE | B | 182 | 6.566 | 6.012 | 51.978 | 1.00 | 37.05 | B | C |
| ATOM | 3771 | O | ILE | B | 182 | 6.848 | 4.936 | 52.516 | 1.00 | 37.05 | B | O |
| ATOM | 3772 | N | LYS | B | 183 | 5.833 | 6.109 | 50.873 | 1.00 | 31.82 | B | N |
| ATOM | 3773 | CA | LYS | B | 183 | 5.341 | 4.940 | 50.148 | 1.00 | 31.82 | B | C |
| ATOM | 3774 | CB | LYS | B | 183 | 4.137 | 4.317 | 50.859 | 1.00 | 40.48 | B | C |
| ATOM | 3775 | CG | LYS | B | 183 | 2.936 | 5.221 | 51.019 | 1.00 | 40.48 | B | C |
| ATOM | 3776 | CD | LYS | B | 183 | 1.824 | 4.485 | 51.736 | 1.00 | 40.48 | B | C |
| ATOM | 3777 | CE | LYS | B | 183 | 0.666 | 5.398 | 52.066 | 1.00 | 40.48 | B | C |
| ATOM | 3778 | NZ | LYS | B | 183 | -0.298 | 4.740 | 52.994 | 1.00 | 40.48 | B | N |
| ATOM | 3779 | C | LYS | B | 183 | 4.972 | 5.358 | 48.730 | 1.00 | 31.82 | B | C |
| ATOM | 3780 | O | LYS | B | 183 | 4.723 | 6.526 | 48.467 | 1.00 | 31.82 | B | O |
| ATOM | 3781 | N | PRO | B | 184 | 4.956 | 4.405 | 47.788 | 1.00 | 43.83 | B | N |
| ATOM | 3782 | CD | PRO | B | 184 | 5.294 | 2.978 | 47.938 | 1.00 | 42.42 | B | C |
| ATOM | 3783 | CA | PRO | B | 184 | 4.617 | 4.711 | 46.397 | 1.00 | 43.83 | B | C |
| ATOM | 3784 | CB | PRO | B | 184 | 4.530 | 3.329 | 45.760 | 1.00 | 42.42 | B | C |
| ATOM | 3785 | CG | PRO | B | 184 | 5.570 | 2.568 | 46.505 | 1.00 | 42.42 | B | C |
| ATOM | 3786 | C | PRO | B | 184 | 3.321 | 5.504 | 46.244 | 1.00 | 43.83 | B | C |
| ATOM | 3787 | O | PRO | B | 184 | 3.226 | 6.374 | 45.384 | 1.00 | 43.83 | B | O |
| ATOM | 3788 | N | GLN | B | 185 | 2.333 | 5.203 | 47.084 | 1.00 | 35.05 | B | N |
| ATOM | 3789 | CA | GLN | B | 185 | 1.037 | 5.865 | 47.038 | 1.00 | 35.05 | B | C |
| ATOM | 3790 | CB | GLN | B | 185 | 0.055 | 5.176 | 47.988 | 1.00 | 50.01 | B | C |
| ATOM | 3791 | CG | GLN | B | 185 | -0.285 | 3.736 | 47.617 | 1.00 | 50.01 | B | C |
| ATOM | 3792 | CD | GLN | B | 185 | 0.825 | 2.738 | 47.950 | 1.00 | 50.01 | B | C |
| ATOM | 3793 | OE1 | GLN | B | 185 | 1.966 | 3.113 | 48.240 | 1.00 | 50.01 | B | O |
| ATOM | 3794 | NE2 | GLN | B | 185 | 0.489 | 1.454 | 47.898 | 1.00 | 50.01 | B | N |
| ATOM | 3795 | C | GLN | B | 185 | 1.093 | 7.348 | 47.370 | 1.00 | 35.05 | B | C |
| ATOM | 3796 | O | GLN | B | 185 | 0.149 | 8.080 | 47.081 | 1.00 | 35.05 | B | O |
| ATOM | 3797 | N | ASN | B | 186 | 2.190 | 7.794 | 47.978 | 1.00 | 32.50 | B | N |
| ATOM | 3798 | CA | ASN | B | 186 | 2.335 | 9.200 | 48.349 | 1.00 | 32.50 | B | C |
| ATOM | 3799 | CB | ASN | B | 186 | 2.721 | 9.356 | 49.828 | 1.00 | 38.79 | B | C |
| ATOM | 3800 | CG | ASN | B | 186 | 1.567 | 9.089 | 50.772 | 1.00 | 38.79 | B | C |
| ATOM | 3801 | OD1 | ASN | B | 186 | 0.468 | 9.598 | 50.577 | 1.00 | 38.79 | B | O |
| ATOM | 3802 | ND2 | ASN | B | 186 | 1.816 | 8.301 | 51.810 | 1.00 | 38.79 | B | N |
| ATOM | 3803 | C | ASN | B | 186 | 3.360 | 9.929 | 47.503 | 1.00 | 32.50 | B | C |
| ATOM | 3804 | O | ASN | B | 186 | 3.784 | 11.023 | 47.846 | 1.00 | 32.50 | B | O |
| ATOM | 3805 | N | LEU | B | 187 | 3.770 | 9.327 | 46.401 | 1.00 | 25.65 | B | N |
| ATOM | 3806 | CA | LEU | B | 187 | 4.726 | 9.972 | 45.524 | 1.00 | 25.65 | B | C |
| ATOM | 3807 | CB | LEU | B | 187 | 5.890 | 9.031 | 45.210 | 1.00 | 40.50 | B | C |
| ATOM | 3808 | CG | LEU | B | 187 | 6.756 | 8.477 | 46.356 | 1.00 | 40.50 | B | C |
| ATOM | 3809 | CD1 | LEU | B | 187 | 7.730 | 7.458 | 45.793 | 1.00 | 40.50 | B | C |
| ATOM | 3810 | CD2 | LEU | B | 187 | 7.506 | 9.607 | 47.061 | 1.00 | 40.50 | B | C |
| ATOM | 3811 | C | LEU | B | 187 | 3.972 | 10.319 | 44.255 | 1.00 | 25.65 | B | C |
| ATOM | 3812 | O | LEU | B | 187 | 3.811 | 9.491 | 43.373 | 1.00 | 25.65 | B | O |

| | | | | | | | | | | | | |
|---|---|---|---|---|---|---|---|---|---|---|---|---|
| ATOM | 3813 | N | LEU | B | 188 | 3.477 | 11.544 | 44.178 | 1.00 | 41.74 | B | N |
| ATOM | 3814 | CA | LEU | B | 188 | 2.739 | 11.988 | 43.006 | 1.00 | 41.74 | B | C |
| ATOM | 3815 | CB | LEU | B | 188 | 1.935 | 13.239 | 43.326 | 1.00 | 40.28 | B | C |
| ATOM | 3816 | CG | LEU | B | 188 | 1.060 | 13.214 | 44.567 | 1.00 | 40.28 | B | C |
| ATOM | 3817 | CD1 | LEU | B | 188 | 0.305 | 14.521 | 44.598 | 1.00 | 40.28 | B | C |
| ATOM | 3818 | CD2 | LEU | B | 188 | 0.105 | 12.030 | 44.550 | 1.00 | 40.28 | B | C |
| ATOM | 3819 | C | LEU | B | 188 | 3.733 | 12.322 | 41.912 | 1.00 | 41.74 | B | C |
| ATOM | 3820 | O | LEU | B | 188 | 4.906 | 12.569 | 42.189 | 1.00 | 41.74 | B | O |
| ATOM | 3821 | N | LEU | B | 189 | 3.271 | 12.338 | 40.667 | 1.00 | 46.18 | B | N |
| ATOM | 3822 | CA | LEU | B | 189 | 4.160 | 12.661 | 39.560 | 1.00 | 46.18 | B | C |
| ATOM | 3823 | CB | LEU | B | 189 | 5.181 | 11.536 | 39.321 | 1.00 | 43.25 | B | C |
| ATOM | 3824 | CG | LEU | B | 189 | 4.653 | 10.194 | 38.811 | 1.00 | 43.25 | B | C |
| ATOM | 3825 | CD1 | LEU | B | 189 | 5.793 | 9.222 | 38.613 | 1.00 | 43.25 | B | C |
| ATOM | 3826 | CD2 | LEU | B | 189 | 3.679 | 9.632 | 39.803 | 1.00 | 43.25 | B | C |
| ATOM | 3827 | C | LEU | B | 189 | 3.420 | 12.947 | 38.272 | 1.00 | 46.18 | B | C |
| ATOM | 3828 | O | LEU | B | 189 | 2.474 | 12.260 | 37.921 | 1.00 | 46.18 | B | O |
| ATOM | 3829 | N | ASP | B | 190 | 3.882 | 13.982 | 37.583 | 1.00 | 47.63 | B | N |
| ATOM | 3830 | CA | ASP | B | 190 | 3.330 | 14.423 | 36.315 | 1.00 | 47.63 | B | C |
| ATOM | 3831 | CB | ASP | B | 190 | 3.602 | 15.922 | 36.151 | 1.00 | 60.16 | B | C |
| ATOM | 3832 | CG | ASP | B | 190 | 3.142 | 16.465 | 34.810 | 1.00 | 60.16 | B | C |
| ATOM | 3833 | OD1 | ASP | B | 190 | 3.705 | 16.076 | 33.763 | 1.00 | 60.16 | B | O |
| ATOM | 3834 | OD2 | ASP | B | 190 | 2.211 | 17.291 | 34.808 | 1.00 | 60.16 | B | O |
| ATOM | 3835 | C | ASP | B | 190 | 3.998 | 13.621 | 35.190 | 1.00 | 47.63 | B | C |
| ATOM | 3836 | O | ASP | B | 190 | 5.187 | 13.790 | 34.897 | 1.00 | 47.63 | B | O |
| ATOM | 3837 | N | PRO | B | 191 | 3.229 | 12.747 | 34.532 | 1.00 | 49.91 | B | N |
| ATOM | 3838 | CD | PRO | B | 191 | 1.757 | 12.661 | 34.582 | 1.00 | 70.68 | B | C |
| ATOM | 3839 | CA | PRO | B | 191 | 3.765 | 11.923 | 33.447 | 1.00 | 49.91 | B | C |
| ATOM | 3840 | CB | PRO | B | 191 | 2.532 | 11.165 | 32.952 | 1.00 | 70.68 | B | C |
| ATOM | 3841 | CG | PRO | B | 191 | 1.414 | 12.164 | 33.189 | 1.00 | 70.68 | B | C |
| ATOM | 3842 | C | PRO | B | 191 | 4.507 | 12.659 | 32.326 | 1.00 | 49.91 | B | C |
| ATOM | 3843 | O | PRO | B | 191 | 5.559 | 12.204 | 31.897 | 1.00 | 49.91 | B | O |
| ATOM | 3844 | N | ASP | B | 192 | 3.992 | 13.790 | 31.854 | 1.00 | 40.47 | B | N |
| ATOM | 3845 | CA | ASP | B | 192 | 4.671 | 14.511 | 30.764 | 1.00 | 40.47 | B | C |
| ATOM | 3846 | CB | ASP | B | 192 | 3.712 | 15.489 | 30.045 | 1.00 | 66.72 | B | C |
| ATOM | 3847 | CG | ASP | B | 192 | 2.408 | 14.833 | 29.590 | 1.00 | 66.72 | B | C |
| ATOM | 3848 | OD1 | ASP | B | 192 | 2.428 | 13.754 | 28.948 | 1.00 | 66.72 | B | O |
| ATOM | 3849 | OD2 | ASP | B | 192 | 1.348 | 15.430 | 29.874 | 1.00 | 66.72 | B | O |
| ATOM | 3850 | C | ASP | B | 192 | 5.923 | 15.299 | 31.174 | 1.00 | 40.47 | B | C |
| ATOM | 3851 | O | ASP | B | 192 | 6.729 | 15.676 | 30.321 | 1.00 | 40.47 | B | O |
| ATOM | 3852 | N | THR | B | 193 | 6.081 | 15.582 | 32.462 | 1.00 | 38.88 | B | N |
| ATOM | 3853 | CA | THR | B | 193 | 7.257 | 16.329 | 32.898 | 1.00 | 38.88 | B | C |
| ATOM | 3854 | CB | THR | B | 193 | 6.866 | 17.586 | 33.687 | 1.00 | 41.95 | B | C |
| ATOM | 3855 | OG1 | THR | B | 193 | 5.935 | 17.244 | 34.714 | 1.00 | 41.95 | B | O |
| ATOM | 3856 | CG2 | THR | B | 193 | 6.237 | 18.596 | 32.770 | 1.00 | 41.95 | B | C |
| ATOM | 3857 | C | THR | B | 193 | 8.184 | 15.469 | 33.735 | 1.00 | 38.88 | B | C |
| ATOM | 3858 | O | THR | B | 193 | 9.366 | 15.771 | 33.872 | 1.00 | 38.88 | B | O |
| ATOM | 3859 | N | ALA | B | 194 | 7.631 | 14.388 | 34.270 | 1.00 | 38.95 | B | N |
| ATOM | 3860 | CA | ALA | B | 194 | 8.375 | 13.443 | 35.088 | 1.00 | 38.95 | B | C |
| ATOM | 3861 | CB | ALA | B | 194 | 9.582 | 12.918 | 34.321 | 1.00 | 27.25 | B | C |
| ATOM | 3862 | C | ALA | B | 194 | 8.827 | 14.009 | 36.427 | 1.00 | 38.95 | B | C |
| ATOM | 3863 | O | ALA | B | 194 | 9.818 | 13.551 | 36.988 | 1.00 | 38.95 | B | O |
| ATOM | 3864 | N | VAL | B | 195 | 8.115 | 14.999 | 36.950 | 1.00 | 40.82 | B | N |
| ATOM | 3865 | CA | VAL | B | 195 | 8.508 | 15.550 | 38.228 | 1.00 | 40.82 | B | C |
| ATOM | 3866 | CB | VAL | B | 195 | 8.263 | 17.081 | 38.283 | 1.00 | 32.75 | B | C |
| ATOM | 3867 | CG1 | VAL | B | 195 | 8.522 | 17.688 | 36.915 | 1.00 | 32.75 | B | C |
| ATOM | 3868 | CG2 | VAL | B | 195 | 6.873 | 17.390 | 38.790 | 1.00 | 32.75 | B | C |
| ATOM | 3869 | C | VAL | B | 195 | 7.729 | 14.831 | 39.320 | 1.00 | 40.82 | B | C |
| ATOM | 3870 | O | VAL | B | 195 | 6.554 | 14.510 | 39.149 | 1.00 | 40.82 | B | O |
| ATOM | 3871 | N | LEU | B | 196 | 8.396 | 14.554 | 40.434 | 1.00 | 42.74 | B | N |
| ATOM | 3872 | CA | LEU | B | 196 | 7.766 | 13.859 | 41.546 | 1.00 | 42.74 | B | C |
| ATOM | 3873 | CB | LEU | B | 196 | 8.664 | 12.696 | 41.998 | 1.00 | 33.47 | B | C |
| ATOM | 3874 | CG | LEU | B | 196 | 8.221 | 11.800 | 43.159 | 1.00 | 33.47 | B | C |
| ATOM | 3875 | CD1 | LEU | B | 196 | 8.820 | 10.436 | 42.990 | 1.00 | 33.47 | B | C |
| ATOM | 3876 | CD2 | LEU | B | 196 | 8.641 | 12.402 | 44.473 | 1.00 | 33.47 | B | C |
| ATOM | 3877 | C | LEU | B | 196 | 7.500 | 14.820 | 42.699 | 1.00 | 42.74 | B | C |
| ATOM | 3878 | O | LEU | B | 196 | 8.224 | 15.793 | 42.888 | 1.00 | 42.74 | B | O |
| ATOM | 3879 | N | LYS | B | 197 | 6.462 | 14.542 | 43.474 | 1.00 | 35.44 | B | N |

| | | | | | | | | | | | | |
|---|---|---|---|---|---|---|---|---|---|---|---|---|
| ATOM | 3880 | CA | LYS | B | 197 | 6.108 | 15.400 | 44.589 | 1.00 | 35.44 | B | C |
| ATOM | 3881 | CB | LYS | B | 197 | 4.978 | 16.344 | 44.188 | 1.00 | 38.85 | B | C |
| ATOM | 3882 | CG | LYS | B | 197 | 5.424 | 17.393 | 43.231 | 1.00 | 38.85 | B | C |
| ATOM | 3883 | CD | LYS | B | 197 | 4.275 | 18.114 | 42.608 | 1.00 | 38.85 | B | C |
| ATOM | 3884 | CE | LYS | B | 197 | 4.822 | 19.176 | 41.682 | 1.00 | 38.85 | B | C |
| ATOM | 3885 | NZ | LYS | B | 197 | 5.886 | 19.948 | 42.398 | 1.00 | 38.85 | B | N |
| ATOM | 3886 | C | LYS | B | 197 | 5.672 | 14.625 | 45.805 | 1.00 | 35.44 | B | C |
| ATOM | 3887 | O | LYS | B | 197 | 4.738 | 13.828 | 45.742 | 1.00 | 35.44 | B | O |
| ATOM | 3888 | N | LEU | B | 198 | 6.348 | 14.859 | 46.922 | 1.00 | 38.91 | B | N |
| ATOM | 3889 | CA | LEU | B | 198 | 5.970 | 14.189 | 48.143 | 1.00 | 38.91 | B | C |
| ATOM | 3890 | CB | LEU | B | 198 | 7.025 | 14.417 | 49.222 | 1.00 | 41.34 | B | C |
| ATOM | 3891 | CG | LEU | B | 198 | 7.577 | 13.200 | 49.971 | 1.00 | 41.34 | B | C |
| ATOM | 3892 | CD1 | LEU | B | 198 | 8.440 | 13.680 | 51.124 | 1.00 | 41.34 | B | C |
| ATOM | 3893 | CD2 | LEU | B | 198 | 6.457 | 12.338 | 50.488 | 1.00 | 41.34 | B | C |
| ATOM | 3894 | C | LEU | B | 198 | 4.648 | 14.832 | 48.563 | 1.00 | 38.91 | B | C |
| ATOM | 3895 | O | LEU | B | 198 | 4.479 | 16.051 | 48.472 | 1.00 | 38.91 | B | O |
| ATOM | 3896 | N | CYS | B | 199 | 3.696 | 14.011 | 48.982 | 1.00 | 36.27 | B | N |
| ATOM | 3897 | CA | CYS | B | 199 | 2.422 | 14.529 | 49.448 | 1.00 | 36.27 | B | C |
| ATOM | 3898 | CB | CYS | B | 199 | 1.359 | 14.498 | 48.333 | 1.00 | 52.34 | B | C |
| ATOM | 3899 | SG | CYS | B | 199 | 0.591 | 12.885 | 48.004 | 1.00 | 52.34 | B | S |
| ATOM | 3900 | C | CYS | B | 199 | 1.996 | 13.678 | 50.641 | 1.00 | 36.27 | B | C |
| ATOM | 3901 | O | CYS | B | 199 | 2.587 | 12.646 | 50.907 | 1.00 | 36.27 | B | O |
| ATOM | 3902 | N | ASP | B | 200 | 0.970 | 14.131 | 51.348 | 1.00 | 53.72 | B | N |
| ATOM | 3903 | CA | ASP | B | 200 | 0.435 | 13.460 | 52.530 | 1.00 | 53.72 | B | C |
| ATOM | 3904 | CB | ASP | B | 200 | 0.157 | 11.974 | 52.272 | 1.00 | 59.46 | B | C |
| ATOM | 3905 | CG | ASP | B | 200 | -0.679 | 11.340 | 53.385 | 1.00 | 59.46 | B | C |
| ATOM | 3906 | OD1 | ASP | B | 200 | -0.872 | 11.998 | 54.436 | 1.00 | 59.46 | B | O |
| ATOM | 3907 | OD2 | ASP | B | 200 | -1.144 | 10.189 | 53.219 | 1.00 | 59.46 | B | O |
| ATOM | 3908 | C | ASP | B | 200 | 1.371 | 13.604 | 53.722 | 1.00 | 53.72 | B | C |
| ATOM | 3909 | O | ASP | B | 200 | 2.134 | 12.697 | 54.058 | 1.00 | 53.72 | B | O |
| ATOM | 3910 | N | PHE | B | 201 | 1.307 | 14.762 | 54.363 | 1.00 | 33.71 | B | N |
| ATOM | 3911 | CA | PHE | B | 201 | 2.144 | 15.023 | 55.517 | 1.00 | 33.71 | B | C |
| ATOM | 3912 | CB | PHE | B | 201 | 2.745 | 16.438 | 55.437 | 1.00 | 28.12 | B | C |
| ATOM | 3913 | CG | PHE | B | 201 | 3.886 | 16.573 | 54.450 | 1.00 | 28.12 | B | C |
| ATOM | 3914 | CD1 | PHE | B | 201 | 3.653 | 16.580 | 53.079 | 1.00 | 28.12 | B | C |
| ATOM | 3915 | CD2 | PHE | B | 201 | 5.195 | 16.714 | 54.903 | 1.00 | 28.12 | B | C |
| ATOM | 3916 | CE1 | PHE | B | 201 | 4.708 | 16.727 | 52.177 | 1.00 | 28.12 | B | C |
| ATOM | 3917 | CE2 | PHE | B | 201 | 6.259 | 16.864 | 54.013 | 1.00 | 28.12 | B | C |
| ATOM | 3918 | CZ | PHE | B | 201 | 6.015 | 16.870 | 52.651 | 1.00 | 28.12 | B | C |
| ATOM | 3919 | C | PHE | B | 201 | 1.308 | 14.864 | 56.781 | 1.00 | 33.71 | B | C |
| ATOM | 3920 | O | PHE | B | 201 | 1.585 | 15.481 | 57.800 | 1.00 | 33.71 | B | O |
| ATOM | 3921 | N | GLY | B | 202 | 0.292 | 14.013 | 56.707 | 1.00 | 48.40 | B | N |
| ATOM | 3922 | CA | GLY | B | 202 | -0.585 | 13.798 | 57.845 | 1.00 | 48.40 | B | C |
| ATOM | 3923 | C | GLY | B | 202 | 0.018 | 12.982 | 58.973 | 1.00 | 48.40 | B | C |
| ATOM | 3924 | O | GLY | B | 202 | -0.616 | 12.816 | 60.010 | 1.00 | 48.40 | B | O |
| ATOM | 3925 | N | SER | B | 203 | 1.235 | 12.474 | 58.767 | 1.00 | 44.27 | B | N |
| ATOM | 3926 | CA | SER | B | 203 | 1.957 | 11.671 | 59.755 | 1.00 | 44.27 | B | C |
| ATOM | 3927 | CB | SER | B | 203 | 2.155 | 10.239 | 59.252 | 1.00 | 34.43 | B | C |
| ATOM | 3928 | OG | SER | B | 203 | 0.934 | 9.649 | 58.868 | 1.00 | 34.43 | B | O |
| ATOM | 3929 | C | SER | B | 203 | 3.332 | 12.291 | 59.976 | 1.00 | 44.27 | B | C |
| ATOM | 3930 | O | SER | B | 203 | 4.128 | 11.808 | 60.792 | 1.00 | 44.27 | B | O |
| ATOM | 3931 | N | ALA | B | 204 | 3.614 | 13.349 | 59.219 | 1.00 | 41.64 | B | N |
| ATOM | 3932 | CA | ALA | B | 204 | 4.888 | 14.051 | 59.316 | 1.00 | 41.64 | B | C |
| ATOM | 3933 | CB | ALA | B | 204 | 4.997 | 15.111 | 58.208 | 1.00 | 48.14 | B | C |
| ATOM | 3934 | C | ALA | B | 204 | 4.997 | 14.703 | 60.685 | 1.00 | 41.64 | B | C |
| ATOM | 3935 | O | ALA | B | 204 | 3.998 | 15.125 | 61.259 | 1.00 | 41.64 | B | O |
| ATOM | 3936 | N | LYS | B | 205 | 6.215 | 14.789 | 61.204 | 1.00 | 42.49 | B | N |
| ATOM | 3937 | CA | LYS | B | 205 | 6.432 | 15.378 | 62.512 | 1.00 | 42.49 | B | C |
| ATOM | 3938 | CB | LYS | B | 205 | 6.070 | 14.373 | 63.598 | 1.00 | 32.64 | B | C |
| ATOM | 3939 | CG | LYS | B | 205 | 6.518 | 14.754 | 65.002 | 1.00 | 32.64 | B | C |
| ATOM | 3940 | CD | LYS | B | 205 | 5.997 | 13.744 | 66.002 | 1.00 | 32.64 | B | C |
| ATOM | 3941 | CE | LYS | B | 205 | 6.369 | 14.075 | 67.419 | 1.00 | 32.64 | B | C |
| ATOM | 3942 | NZ | LYS | B | 205 | 5.822 | 13.029 | 68.328 | 1.00 | 32.64 | B | N |
| ATOM | 3943 | C | LYS | B | 205 | 7.860 | 15.826 | 62.717 | 1.00 | 42.49 | B | C |
| ATOM | 3944 | O | LYS | B | 205 | 8.809 | 15.165 | 62.279 | 1.00 | 42.49 | B | O |
| ATOM | 3945 | N | GLN | B | 206 | 8.009 | 16.967 | 63.381 | 1.00 | 51.97 | B | N |
| ATOM | 3946 | CA | GLN | B | 206 | 9.335 | 17.482 | 63.683 | 1.00 | 51.97 | B | C |

| | | | | | | | | | | | | |
|---|---|---|---|---|---|---|---|---|---|---|---|---|
| ATOM | 3947 | CB | GLN | B | 206 | 9.256 | 18.957 | 64.055 | 1.00 | 73.48 | B | C |
| ATOM | 3948 | CG | GLN | B | 206 | 10.604 | 19.581 | 64.320 | 1.00 | 73.48 | B | C |
| ATOM | 3949 | CD | GLN | B | 206 | 10.648 | 21.049 | 63.922 | 1.00 | 73.48 | B | C |
| ATOM | 3950 | OE1 | GLN | B | 206 | 11.588 | 21.774 | 64.283 | 1.00 | 73.48 | B | O |
| ATOM | 3951 | NE2 | GLN | B | 206 | 9.632 | 21.499 | 63.162 | 1.00 | 73.48 | B | N |
| ATOM | 3952 | C | GLN | B | 206 | 9.825 | 16.648 | 64.860 | 1.00 | 51.97 | B | C |
| ATOM | 3953 | O | GLN | B | 206 | 9.181 | 16.591 | 65.898 | 1.00 | 51.97 | B | O |
| ATOM | 3954 | N | LEU | B | 207 | 10.946 | 15.968 | 64.683 | 1.00 | 41.20 | B | N |
| ATOM | 3955 | CA | LEU | B | 207 | 11.471 | 15.120 | 65.740 | 1.00 | 41.20 | B | C |
| ATOM | 3956 | CB | LEU | B | 207 | 12.116 | 13.862 | 65.153 | 1.00 | 41.59 | B | C |
| ATOM | 3957 | CG | LEU | B | 207 | 11.223 | 12.965 | 64.287 | 1.00 | 41.59 | B | C |
| ATOM | 3958 | CD1 | LEU | B | 207 | 12.077 | 11.902 | 63.626 | 1.00 | 41.59 | B | C |
| ATOM | 3959 | CD2 | LEU | B | 207 | 10.101 | 12.339 | 65.121 | 1.00 | 41.59 | B | C |
| ATOM | 3960 | C | LEU | B | 207 | 12.486 | 15.871 | 66.575 | 1.00 | 41.20 | B | C |
| ATOM | 3961 | O | LEU | B | 207 | 13.382 | 16.535 | 66.052 | 1.00 | 41.20 | B | O |
| ATOM | 3962 | N | VAL | B | 208 | 12.321 | 15.761 | 67.886 | 1.00 | 53.81 | B | N |
| ATOM | 3963 | CA | VAL | B | 208 | 13.198 | 16.404 | 68.847 | 1.00 | 53.81 | B | C |
| ATOM | 3964 | CB | VAL | B | 208 | 12.395 | 17.332 | 69.776 | 1.00 | 53.29 | B | C |
| ATOM | 3965 | CG1 | VAL | B | 208 | 13.093 | 17.466 | 71.120 | 1.00 | 53.29 | B | C |
| ATOM | 3966 | CG2 | VAL | B | 208 | 12.243 | 18.698 | 69.122 | 1.00 | 53.29 | B | C |
| ATOM | 3967 | C | VAL | B | 208 | 13.881 | 15.321 | 69.662 | 1.00 | 53.81 | B | C |
| ATOM | 3968 | O | VAL | B | 208 | 13.227 | 14.438 | 70.237 | 1.00 | 53.81 | B | O |
| ATOM | 3969 | N | ARG | B | 209 | 15.204 | 15.382 | 69.702 | 1.00 | 66.20 | B | N |
| ATOM | 3970 | CA | ARG | B | 209 | 15.965 | 14.399 | 70.451 | 1.00 | 66.20 | B | C |
| ATOM | 3971 | CB | ARG | B | 209 | 17.450 | 14.720 | 70.367 | 1.00100.00 | | B | C |
| ATOM | 3972 | CG | ARG | B | 209 | 18.362 | 13.615 | 70.919 | 1.00100.00 | | B | C |
| ATOM | 3973 | CD | ARG | B | 209 | 19.841 | 14.007 | 70.740 | 1.00100.00 | | B | C |
| ATOM | 3974 | NE | ARG | B | 209 | 20.048 | 14.636 | 69.430 | 1.00100.00 | | B | N |
| ATOM | 3975 | CZ | ARG | B | 209 | 21.183 | 15.209 | 69.034 | 1.00100.00 | | B | C |
| ATOM | 3976 | NH1 | ARG | B | 209 | 22.232 | 15.229 | 69.854 | 1.00100.00 | | B | N |
| ATOM | 3977 | NH2 | ARG | B | 209 | 21.259 | 15.781 | 67.825 | 1.00100.00 | | B | N |
| ATOM | 3978 | C | ARG | B | 209 | 15.523 | 14.437 | 71.906 | 1.00 | 66.20 | B | C |
| ATOM | 3979 | O | ARG | B | 209 | 15.667 | 15.467 | 72.572 | 1.00 | 66.20 | B | O |
| ATOM | 3980 | N | GLY | B | 210 | 14.977 | 13.333 | 72.405 | 1.00 | 62.30 | B | N |
| ATOM | 3981 | CA | GLY | B | 210 | 14.549 | 13.320 | 73.793 | 1.00 | 62.30 | B | C |
| ATOM | 3982 | C | GLY | B | 210 | 13.065 | 13.064 | 73.955 | 1.00 | 62.30 | B | C |
| ATOM | 3983 | O | GLY | B | 210 | 12.648 | 12.259 | 74.796 | 1.00 | 62.30 | B | O |
| ATOM | 3984 | N | GLU | B | 211 | 12.254 | 13.751 | 73.157 | 1.00 | 66.27 | B | N |
| ATOM | 3985 | CA | GLU | B | 211 | 10.811 | 13.552 | 73.222 | 1.00 | 66.27 | B | C |
| ATOM | 3986 | CB | GLU | B | 211 | 10.105 | 14.778 | 72.645 | 1.00 | 67.47 | B | C |
| ATOM | 3987 | CG | GLU | B | 211 | 10.582 | 16.088 | 73.268 | 1.00 | 67.47 | B | C |
| ATOM | 3988 | CD | GLU | B | 211 | 10.193 | 17.315 | 72.449 | 1.00 | 67.47 | B | C |
| ATOM | 3989 | OE1 | GLU | B | 211 | 10.521 | 18.447 | 72.877 | 1.00 | 67.47 | B | O |
| ATOM | 3990 | OE2 | GLU | B | 211 | 9.567 | 17.153 | 71.372 | 1.00 | 67.47 | B | O |
| ATOM | 3991 | C | GLU | B | 211 | 10.470 | 12.280 | 72.423 | 1.00 | 66.27 | B | C |
| ATOM | 3992 | O | GLU | B | 211 | 10.679 | 12.216 | 71.209 | 1.00 | 66.27 | B | O |
| ATOM | 3993 | N | PRO | B | 212 | 9.970 | 11.237 | 73.106 | 1.00 | 62.80 | B | N |
| ATOM | 3994 | CD | PRO | B | 212 | 9.630 | 11.161 | 74.535 | 1.00 | 63.66 | B | C |
| ATOM | 3995 | CA | PRO | B | 212 | 9.619 | 9.984 | 72.428 | 1.00 | 62.80 | B | C |
| ATOM | 3996 | CB | PRO | B | 212 | 9.123 | 9.094 | 73.569 | 1.00 | 63.66 | B | C |
| ATOM | 3997 | CG | PRO | B | 212 | 8.564 | 10.076 | 74.537 | 1.00 | 63.66 | B | C |
| ATOM | 3998 | C | PRO | B | 212 | 8.574 | 10.160 | 71.341 | 1.00 | 62.80 | B | C |
| ATOM | 3999 | O | PRO | B | 212 | 7.808 | 11.130 | 71.359 | 1.00 | 62.80 | B | O |
| ATOM | 4000 | N | ASN | B | 213 | 8.551 | 9.211 | 70.402 | 1.00 | 52.52 | B | N |
| ATOM | 4001 | CA | ASN | B | 213 | 7.611 | 9.222 | 69.281 | 1.00 | 52.52 | B | C |
| ATOM | 4002 | CB | ASN | B | 213 | 8.348 | 9.570 | 67.996 | 1.00 | 33.17 | B | C |
| ATOM | 4003 | CG | ASN | B | 213 | 9.161 | 10.832 | 68.122 | 1.00 | 33.17 | B | C |
| ATOM | 4004 | OD1 | ASN | B | 213 | 8.616 | 11.907 | 68.320 | 1.00 | 33.17 | B | O |
| ATOM | 4005 | ND2 | ASN | B | 213 | 10.475 | 10.705 | 68.018 | 1.00 | 33.17 | B | N |
| ATOM | 4006 | C | ASN | B | 213 | 6.983 | 7.846 | 69.147 | 1.00 | 52.52 | B | C |
| ATOM | 4007 | O | ASN | B | 213 | 7.568 | 6.856 | 69.583 | 1.00 | 52.52 | B | O |
| ATOM | 4008 | N | VAL | B | 214 | 5.788 | 7.786 | 68.560 | 1.00 | 35.95 | B | N |
| ATOM | 4009 | CA | VAL | B | 214 | 5.091 | 6.514 | 68.355 | 1.00 | 35.95 | B | C |
| ATOM | 4010 | CB | VAL | B | 214 | 3.688 | 6.743 | 67.740 | 1.00 | 42.51 | B | C |
| ATOM | 4011 | CG1 | VAL | B | 214 | 3.117 | 5.434 | 67.223 | 1.00 | 42.51 | B | C |
| ATOM | 4012 | CG2 | VAL | B | 214 | 2.758 | 7.346 | 68.784 | 1.00 | 42.51 | B | C |
| ATOM | 4013 | C | VAL | B | 214 | 5.913 | 5.640 | 67.405 | 1.00 | 35.95 | B | C |

| | | | | | | | | | | | | |
|---|---|---|---|---|---|---|---|---|---|---|---|---|
| ATOM | 4014 | O | VAL | B | 214 | 6.389 | 6.111 | 66.366 | 1.00 | 35.95 | B | O |
| ATOM | 4015 | N | SER | B | 215 | 6.089 | 4.370 | 67.753 | 1.00 | 46.54 | B | N |
| ATOM | 4016 | CA | SER | B | 215 | 6.879 | 3.495 | 66.893 | 1.00 | 46.54 | B | C |
| ATOM | 4017 | CB | SER | B | 215 | 7.292 | 2.221 | 67.652 | 1.00 | 32.83 | B | C |
| ATOM | 4018 | OG | SER | B | 215 | 6.178 | 1.548 | 68.194 | 1.00 | 32.83 | B | O |
| ATOM | 4019 | C | SER | B | 215 | 6.117 | 3.159 | 65.604 | 1.00 | 46.54 | B | C |
| ATOM | 4020 | O | SER | B | 215 | 6.193 | 2.051 | 65.064 | 1.00 | 46.54 | B | O |
| ATOM | 4021 | N | PTY | B | 216 | 4.116 | 2.221 | 66.523 | 1.00 | 45.96 | B | N |
| ATOM | 4022 | CA | PTY | B | 216 | 3.378 | 1.977 | 65.292 | 1.00 | 45.96 | B | C |
| ATOM | 4023 | C | PTY | B | 216 | 3.121 | 3.291 | 64.569 | 1.00 | 45.96 | B | C |
| ATOM | 4024 | O | PTY | B | 216 | 2.378 | 3.337 | 63.595 | 1.00 | 45.96 | B | O |
| ATOM | 4025 | CB | PTY | B | 216 | 2.053 | 1.311 | 65.627 | 1.00 | 45.96 | B | C |
| ATOM | 4026 | CG | PTY | B | 216 | 1.592 | 0.415 | 64.573 | 1.00 | 45.96 | B | C |
| ATOM | 4027 | CD1 | PTY | B | 216 | 2.197 | -0.906 | 64.410 | 1.00 | 45.96 | B | C |
| ATOM | 4028 | CD2 | PTY | B | 216 | 0.363 | 0.772 | 63.851 | 1.00 | 45.96 | B | C |
| ATOM | 4029 | CE1 | PTY | B | 216 | 1.551 | -1.879 | 63.519 | 1.00 | 45.96 | B | C |
| ATOM | 4030 | CE2 | PTY | B | 216 | -0.261 | -0.215 | 62.968 | 1.00 | 45.96 | B | C |
| ATOM | 4031 | CZ | PTY | B | 216 | 0.317 | -1.548 | 62.792 | 1.00 | 45.96 | B | C |
| ATOM | 4032 | OH | PTY | B | 216 | -0.323 | -2.503 | 62.045 | 1.00 | 45.96 | B | O |
| ATOM | 4033 | P | PTY | B | 216 | 0.024 | -2.514 | 60.579 | 1.00 | 45.96 | B | P |
| ATOM | 4034 | OP1 | PTY | B | 216 | 1.215 | -3.422 | 60.488 | 1.00 | 45.96 | B | O |
| ATOM | 4035 | OP2 | PTY | B | 216 | 0.267 | -1.014 | 60.088 | 1.00 | 45.96 | B | O |
| ATOM | 4036 | OP3 | PTY | B | 216 | -1.142 | -3.137 | 59.860 | 1.00 | 45.96 | B | O |
| ATOM | 4037 | N | ILE | B | 217 | 4.676 | 3.481 | 62.724 | 1.00 | 38.77 | B | N |
| ATOM | 4038 | CA | ILE | B | 217 | 4.065 | 4.272 | 61.674 | 1.00 | 38.77 | B | C |
| ATOM | 4039 | CB | ILE | B | 217 | 4.374 | 5.794 | 61.845 | 1.00 | 42.71 | B | C |
| ATOM | 4040 | CG2 | ILE | B | 217 | 3.479 | 6.407 | 62.910 | 1.00 | 42.71 | B | C |
| ATOM | 4041 | CG1 | ILE | B | 217 | 5.856 | 5.995 | 62.166 | 1.00 | 42.71 | B | C |
| ATOM | 4042 | CD1 | ILE | B | 217 | 6.278 | 5.436 | 63.488 | 1.00 | 42.71 | B | C |
| ATOM | 4043 | C | ILE | B | 217 | 4.595 | 3.788 | 60.334 | 1.00 | 38.77 | B | C |
| ATOM | 4044 | O | ILE | B | 217 | 5.426 | 2.887 | 60.289 | 1.00 | 38.77 | B | O |
| ATOM | 4045 | N | CYS | B | 218 | 4.120 | 4.403 | 59.258 | 1.00 | 38.72 | B | N |
| ATOM | 4046 | CA | CYS | B | 218 | 4.501 | 4.046 | 57.890 | 1.00 | 38.72 | B | C |
| ATOM | 4047 | CB | CYS | B | 218 | 5.994 | 3.692 | 57.750 | 1.00 | 47.58 | B | C |
| ATOM | 4048 | SG | CYS | B | 218 | 7.167 | 4.736 | 58.581 | 1.00 | 47.58 | B | S |
| ATOM | 4049 | C | CYS | B | 218 | 3.720 | 2.807 | 57.511 | 1.00 | 38.72 | B | C |
| ATOM | 4050 | O | CYS | B | 218 | 3.400 | 1.991 | 58.372 | 1.00 | 38.72 | B | O |
| ATOM | 4051 | N | SER | B | 219 | 3.412 | 2.657 | 56.229 | 1.00 | 36.32 | B | N |
| ATOM | 4052 | CA | SER | B | 219 | 2.715 | 1.461 | 55.809 | 1.00 | 36.32 | B | C |
| ATOM | 4053 | CB | SER | B | 219 | 2.459 | 1.473 | 54.306 | 1.00 | 59.07 | B | C |
| ATOM | 4054 | OG | SER | B | 219 | 1.606 | 2.546 | 53.955 | 1.00 | 59.07 | B | O |
| ATOM | 4055 | C | SER | B | 219 | 3.684 | 0.351 | 56.157 | 1.00 | 36.32 | B | C |
| ATOM | 4056 | O | SER | B | 219 | 4.884 | 0.492 | 55.962 | 1.00 | 36.32 | B | O |
| ATOM | 4057 | N | ARG | B | 220 | 3.157 | -0.741 | 56.684 | 1.00 | 36.73 | B | N |
| ATOM | 4058 | CA | ARG | B | 220 | 3.953 | -1.895 | 57.077 | 1.00 | 36.73 | B | C |
| ATOM | 4059 | CB | ARG | B | 220 | 3.040 | -3.101 | 57.213 | 1.00 | 45.16 | B | C |
| ATOM | 4060 | CG | ARG | B | 220 | 3.715 | -4.322 | 57.738 | 1.00 | 45.16 | B | C |
| ATOM | 4061 | CD | ARG | B | 220 | 2.686 | -5.199 | 58.386 | 1.00 | 45.16 | B | C |
| ATOM | 4062 | NE | ARG | B | 220 | 1.791 | -5.812 | 57.413 | 1.00 | 45.16 | B | N |
| ATOM | 4063 | CZ | ARG | B | 220 | 0.515 | -6.093 | 57.651 | 1.00 | 45.16 | B | C |
| ATOM | 4064 | NH1 | ARG | B | 220 | -0.024 | -5.808 | 58.826 | 1.00 | 45.16 | B | N |
| ATOM | 4065 | NH2 | ARG | B | 220 | -0.218 | -6.677 | 56.720 | 1.00 | 45.16 | B | N |
| ATOM | 4066 | C | ARG | B | 220 | 5.110 | -2.259 | 56.151 | 1.00 | 36.73 | B | C |
| ATOM | 4067 | O | ARG | B | 220 | 6.258 | -2.380 | 56.589 | 1.00 | 36.73 | B | O |
| ATOM | 4068 | N | TYR | B | 221 | 4.800 | -2.449 | 54.874 | 1.00 | 45.52 | B | N |
| ATOM | 4069 | CA | TYR | B | 221 | 5.804 | -2.827 | 53.890 | 1.00 | 45.52 | B | C |
| ATOM | 4070 | CB | TYR | B | 221 | 5.199 | -2.834 | 52.486 | 1.00 | 45.52 | B | C |
| ATOM | 4071 | CG | TYR | B | 221 | 4.196 | -3.930 | 52.236 | 1.00 | 45.52 | B | C |
| ATOM | 4072 | CD1 | TYR | B | 221 | 3.722 | -4.727 | 53.281 | 1.00 | 45.52 | B | C |
| ATOM | 4073 | CE1 | TYR | B | 221 | 2.809 | -5.737 | 53.049 | 1.00 | 45.52 | B | C |
| ATOM | 4074 | CD2 | TYR | B | 221 | 3.722 | -4.175 | 50.954 | 1.00 | 45.52 | B | C |
| ATOM | 4075 | CE2 | TYR | B | 221 | 2.806 | -5.182 | 50.712 | 1.00 | 45.52 | B | C |
| ATOM | 4076 | CZ | TYR | B | 221 | 2.354 | -5.959 | 51.757 | 1.00 | 45.52 | B | C |
| ATOM | 4077 | OH | TYR | B | 221 | 1.450 | -6.962 | 51.500 | 1.00 | 45.52 | B | O |
| ATOM | 4078 | C | TYR | B | 221 | 6.989 | -1.903 | 53.875 | 1.00 | 45.52 | B | C |
| ATOM | 4079 | O | TYR | B | 221 | 8.116 | -2.331 | 53.616 | 1.00 | 45.52 | B | O |
| ATOM | 4080 | N | TYR | B | 222 | 6.725 | -0.631 | 54.144 | 1.00 | 41.36 | B | N |

| | | | | | | | | | | | | |
|---|---|---|---|---|---|---|---|---|---|---|---|---|
| ATOM | 4081 | CA | TYR | B | 222 | 7.761 | 0.383 | 54.103 | 1.00 | 41.36 | B | C |
| ATOM | 4082 | CB | TYR | B | 222 | 7.249 | 1.569 | 53.294 | 1.00 | 50.21 | B | C |
| ATOM | 4083 | CG | TYR | B | 222 | 6.657 | 1.119 | 51.984 | 1.00 | 50.21 | B | C |
| ATOM | 4084 | CD1 | TYR | B | 222 | 5.322 | 0.728 | 51.898 | 1.00 | 50.21 | B | C |
| ATOM | 4085 | CE1 | TYR | B | 222 | 4.799 | 0.197 | 50.721 | 1.00 | 50.21 | B | C |
| ATOM | 4086 | CD2 | TYR | B | 222 | 7.456 | 0.975 | 50.854 | 1.00 | 50.21 | B | C |
| ATOM | 4087 | CE2 | TYR | B | 222 | 6.945 | 0.445 | 49.676 | 1.00 | 50.21 | B | C |
| ATOM | 4088 | CZ | TYR | B | 222 | 5.616 | 0.057 | 49.615 | 1.00 | 50.21 | B | C |
| ATOM | 4089 | OH | TYR | B | 222 | 5.118 | -0.472 | 48.444 | 1.00 | 50.21 | B | O |
| ATOM | 4090 | C | TYR | B | 222 | 8.245 | 0.840 | 55.452 | 1.00 | 41.36 | B | C |
| ATOM | 4091 | O | TYR | B | 222 | 8.982 | 1.813 | 55.533 | 1.00 | 41.36 | B | O |
| ATOM | 4092 | N | ARG | B | 223 | 7.849 | 0.123 | 56.501 | 1.00 | 43.99 | B | N |
| ATOM | 4093 | CA | ARG | B | 223 | 8.227 | 0.450 | 57.876 | 1.00 | 43.99 | B | C |
| ATOM | 4094 | CB | ARG | B | 223 | 7.213 | -0.174 | 58.841 | 1.00 | 40.59 | B | C |
| ATOM | 4095 | CG | ARG | B | 223 | 7.608 | -0.084 | 60.297 | 1.00 | 40.59 | B | C |
| ATOM | 4096 | CD | ARG | B | 223 | 6.407 | 0.092 | 61.195 | 1.00 | 40.59 | B | C |
| ATOM | 4097 | NE | ARG | B | 223 | 5.509 | -1.057 | 61.162 | 1.00 | 40.59 | B | N |
| ATOM | 4098 | CZ | ARG | B | 223 | 4.255 | -1.018 | 60.718 | 1.00 | 40.59 | B | C |
| ATOM | 4099 | NH1 | ARG | B | 223 | 3.734 | 0.118 | 60.260 | 1.00 | 40.59 | B | N |
| ATOM | 4100 | NH2 | ARG | B | 223 | 3.517 | -2.121 | 60.741 | 1.00 | 40.59 | B | N |
| ATOM | 4101 | C | ARG | B | 223 | 9.651 | 0.015 | 58.252 | 1.00 | 43.99 | B | C |
| ATOM | 4102 | O | ARG | B | 223 | 10.036 | -1.137 | 58.039 | 1.00 | 43.99 | B | O |
| ATOM | 4103 | N | ALA | B | 224 | 10.424 | 0.947 | 58.809 | 1.00 | 32.45 | B | N |
| ATOM | 4104 | CA | ALA | B | 224 | 11.799 | 0.679 | 59.231 | 1.00 | 32.45 | B | C |
| ATOM | 4105 | CB | ALA | B | 224 | 12.456 | 1.951 | 59.721 | 1.00 | 47.06 | B | C |
| ATOM | 4106 | C | ALA | B | 224 | 11.840 | -0.368 | 60.328 | 1.00 | 32.45 | B | C |
| ATOM | 4107 | O | ALA | B | 224 | 10.981 | -0.401 | 61.206 | 1.00 | 32.45 | B | O |
| ATOM | 4108 | N | PRO | B | 225 | 12.870 | -1.221 | 60.311 | 1.00 | 42.38 | B | N |
| ATOM | 4109 | CD | PRO | B | 225 | 14.095 | -1.123 | 59.498 | 1.00 | 28.16 | B | C |
| ATOM | 4110 | CA | PRO | B | 225 | 13.003 | -2.274 | 61.318 | 1.00 | 42.38 | B | C |
| ATOM | 4111 | CB | PRO | B | 225 | 14.292 | -2.980 | 60.899 | 1.00 | 28.16 | B | C |
| ATOM | 4112 | CG | PRO | B | 225 | 15.096 | -1.877 | 60.331 | 1.00 | 28.16 | B | C |
| ATOM | 4113 | C | PRO | B | 225 | 13.017 | -1.770 | 62.765 | 1.00 | 42.38 | B | C |
| ATOM | 4114 | O | PRO | B | 225 | 12.508 | -2.448 | 63.660 | 1.00 | 42.38 | B | O |
| ATOM | 4115 | N | GLU | B | 226 | 13.593 | -0.592 | 63.010 | 1.00 | 43.73 | B | N |
| ATOM | 4116 | CA | GLU | B | 226 | 13.611 | -0.067 | 64.372 | 1.00 | 43.73 | B | C |
| ATOM | 4117 | CB | GLU | B | 226 | 14.104 | 1.373 | 64.423 | 1.00 | 44.47 | B | C |
| ATOM | 4118 | CG | GLU | B | 226 | 15.430 | 1.592 | 63.783 | 1.00 | 44.47 | B | C |
| ATOM | 4119 | CD | GLU | B | 226 | 15.290 | 2.221 | 62.436 | 1.00 | 44.47 | B | C |
| ATOM | 4120 | OE1 | GLU | B | 226 | 15.122 | 3.453 | 62.362 | 1.00 | 44.47 | B | O |
| ATOM | 4121 | OE2 | GLU | B | 226 | 15.332 | 1.474 | 61.448 | 1.00 | 44.47 | B | O |
| ATOM | 4122 | C | GLU | B | 226 | 12.184 | -0.073 | 64.858 | 1.00 | 43.73 | B | C |
| ATOM | 4123 | O | GLU | B | 226 | 11.865 | -0.699 | 65.860 | 1.00 | 43.73 | B | O |
| ATOM | 4124 | N | LEU | B | 227 | 11.329 | 0.630 | 64.122 | 1.00 | 39.21 | B | N |
| ATOM | 4125 | CA | LEU | B | 227 | 9.919 | 0.749 | 64.455 | 1.00 | 39.21 | B | C |
| ATOM | 4126 | CB | LEU | B | 227 | 9.165 | 1.376 | 63.290 | 1.00 | 45.83 | B | C |
| ATOM | 4127 | CG | LEU | B | 227 | 9.664 | 2.730 | 62.805 | 1.00 | 45.83 | B | C |
| ATOM | 4128 | CD1 | LEU | B | 227 | 8.920 | 3.121 | 61.540 | 1.00 | 45.83 | B | C |
| ATOM | 4129 | CD2 | LEU | B | 227 | 9.472 | 3.757 | 63.898 | 1.00 | 45.83 | B | C |
| ATOM | 4130 | C | LEU | B | 227 | 9.248 | -0.566 | 64.825 | 1.00 | 39.21 | B | C |
| ATOM | 4131 | O | LEU | B | 227 | 8.453 | -0.613 | 65.749 | 1.00 | 39.21 | B | O |
| ATOM | 4132 | N | ILE | B | 228 | 9.546 | -1.635 | 64.102 | 1.00 | 34.46 | B | N |
| ATOM | 4133 | CA | ILE | B | 228 | 8.924 | -2.915 | 64.412 | 1.00 | 34.46 | B | C |
| ATOM | 4134 | CB | ILE | B | 228 | 9.321 | -4.010 | 63.399 | 1.00 | 42.47 | B | C |
| ATOM | 4135 | CG2 | ILE | B | 228 | 8.527 | -5.288 | 63.677 | 1.00 | 42.47 | B | C |
| ATOM | 4136 | CG1 | ILE | B | 228 | 9.054 | -3.524 | 61.973 | 1.00 | 42.47 | B | C |
| ATOM | 4137 | CD1 | ILE | B | 228 | 9.519 | -4.494 | 60.912 | 1.00 | 42.47 | B | C |
| ATOM | 4138 | C | ILE | B | 228 | 9.365 | -3.353 | 65.798 | 1.00 | 34.46 | B | C |
| ATOM | 4139 | O | ILE | B | 228 | 8.601 | -3.987 | 66.532 | 1.00 | 34.46 | B | O |
| ATOM | 4140 | N | PHE | B | 229 | 10.608 | -3.000 | 66.130 | 1.00 | 46.34 | B | N |
| ATOM | 4141 | CA | PHE | B | 229 | 11.230 | -3.310 | 67.420 | 1.00 | 46.34 | B | C |
| ATOM | 4142 | CB | PHE | B | 229 | 12.746 | -3.138 | 67.334 | 1.00 | 41.94 | B | C |
| ATOM | 4143 | CG | PHE | B | 229 | 13.488 | -4.392 | 66.976 | 1.00 | 41.94 | B | C |
| ATOM | 4144 | CD1 | PHE | B | 229 | 13.663 | -5.402 | 67.914 | 1.00 | 41.94 | B | C |
| ATOM | 4145 | CD2 | PHE | B | 229 | 14.037 | -4.550 | 65.708 | 1.00 | 41.94 | B | C |
| ATOM | 4146 | CE1 | PHE | B | 229 | 14.383 | -6.556 | 67.594 | 1.00 | 41.94 | B | C |
| ATOM | 4147 | CE2 | PHE | B | 229 | 14.758 | -5.692 | 65.373 | 1.00 | 41.94 | B | C |

| | | | | | | | | | | | | |
|---|---|---|---|---|---|---|---|---|---|---|---|---|
| ATOM | 4148 | CZ | PHE | B | 229 | 14.932 | -6.699 | 66.320 | 1.00 | 41.94 | B | C |
| ATOM | 4149 | C | PHE | B | 229 | 10.705 | -2.436 | 68.559 | 1.00 | 46.34 | B | C |
| ATOM | 4150 | O | PHE | B | 229 | 11.196 | -2.513 | 69.674 | 1.00 | 46.34 | B | O |
| ATOM | 4151 | N | GLY | B | 230 | 9.718 | -1.597 | 68.267 | 1.00 | 49.04 | B | N |
| ATOM | 4152 | CA | GLY | B | 230 | 9.133 | -0.748 | 69.286 | 1.00 | 49.04 | B | C |
| ATOM | 4153 | C | GLY | B | 230 | 9.902 | 0.518 | 69.598 | 1.00 | 49.04 | B | C |
| ATOM | 4154 | O | GLY | B | 230 | 9.517 | 1.277 | 70.501 | 1.00 | 49.04 | B | O |
| ATOM | 4155 | N | ALA | B | 231 | 10.980 | 0.758 | 68.857 | 1.00 | 62.29 | B | N |
| ATOM | 4156 | CA | ALA | B | 231 | 11.801 | 1.947 | 69.082 | 1.00 | 62.29 | B | C |
| ATOM | 4157 | CB | ALA | B | 231 | 12.874 | 2.042 | 68.006 | 1.00 | 46.15 | B | C |
| ATOM | 4158 | C | ALA | B | 231 | 10.947 | 3.219 | 69.093 | 1.00 | 62.29 | B | C |
| ATOM | 4159 | O | ALA | B | 231 | 10.116 | 3.421 | 68.196 | 1.00 | 62.29 | B | O |
| ATOM | 4160 | N | THR | B | 232 | 11.145 | 4.070 | 70.100 | 1.00 | 46.16 | B | N |
| ATOM | 4161 | CA | THR | B | 232 | 10.379 | 5.309 | 70.194 | 1.00 | 46.16 | B | C |
| ATOM | 4162 | CB | THR | B | 232 | 9.587 | 5.376 | 71.483 | 1.00 | 63.65 | B | C |
| ATOM | 4163 | OG1 | THR | B | 232 | 10.473 | 5.742 | 72.549 | 1.00 | 63.65 | B | O |
| ATOM | 4164 | CG2 | THR | B | 232 | 8.944 | 4.017 | 71.780 | 1.00 | 63.65 | B | C |
| ATOM | 4165 | C | THR | B | 232 | 11.285 | 6.532 | 70.136 | 1.00 | 46.16 | B | C |
| ATOM | 4166 | O | THR | B | 232 | 10.814 | 7.670 | 70.162 | 1.00 | 46.16 | B | O |
| ATOM | 4167 | N | ASP | B | 233 | 12.587 | 6.294 | 70.061 | 1.00 | 56.68 | B | N |
| ATOM | 4168 | CA | ASP | B | 233 | 13.548 | 7.383 | 69.967 | 1.00 | 56.68 | B | C |
| ATOM | 4169 | CB | ASP | B | 233 | 14.600 | 7.261 | 71.056 | 1.00 | 84.47 | B | C |
| ATOM | 4170 | CG | ASP | B | 233 | 15.601 | 6.160 | 70.757 | 1.00 | 84.47 | B | C |
| ATOM | 4171 | OD1 | ASP | B | 233 | 15.153 | 5.057 | 70.345 | 1.00 | 84.47 | B | O |
| ATOM | 4172 | OD2 | ASP | B | 233 | 16.825 | 6.401 | 70.930 | 1.00 | 84.47 | B | O |
| ATOM | 4173 | C | ASP | B | 233 | 14.239 | 7.267 | 68.616 | 1.00 | 56.68 | B | C |
| ATOM | 4174 | O | ASP | B | 233 | 15.465 | 7.425 | 68.517 | 1.00 | 56.68 | B | O |
| ATOM | 4175 | N | TYR | B | 234 | 13.456 | 6.982 | 67.576 | 1.00 | 49.14 | B | N |
| ATOM | 4176 | CA | TYR | B | 234 | 14.015 | 6.849 | 66.241 | 1.00 | 49.14 | B | C |
| ATOM | 4177 | CB | TYR | B | 234 | 13.059 | 6.053 | 65.352 | 1.00 | 40.85 | B | C |
| ATOM | 4178 | CG | TYR | B | 234 | 11.647 | 6.580 | 65.314 | 1.00 | 40.85 | B | C |
| ATOM | 4179 | CD1 | TYR | B | 234 | 11.282 | 7.597 | 64.431 | 1.00 | 40.85 | B | C |
| ATOM | 4180 | CE1 | TYR | B | 234 | 9.973 | 8.057 | 64.365 | 1.00 | 40.85 | B | C |
| ATOM | 4181 | CD2 | TYR | B | 234 | 10.659 | 6.040 | 66.141 | 1.00 | 40.85 | B | C |
| ATOM | 4182 | CE2 | TYR | B | 234 | 9.341 | 6.501 | 66.079 | 1.00 | 40.85 | B | C |
| ATOM | 4183 | CZ | TYR | B | 234 | 9.015 | 7.510 | 65.184 | 1.00 | 40.85 | B | C |
| ATOM | 4184 | OH | TYR | B | 234 | 7.731 | 7.978 | 65.101 | 1.00 | 40.85 | B | O |
| ATOM | 4185 | C | TYR | B | 234 | 14.319 | 8.212 | 65.643 | 1.00 | 49.14 | B | C |
| ATOM | 4186 | O | TYR | B | 234 | 13.928 | 9.240 | 66.186 | 1.00 | 49.14 | B | O |
| ATOM | 4187 | N | THR | B | 235 | 15.041 | 8.216 | 64.534 | 1.00 | 45.67 | B | N |
| ATOM | 4188 | CA | THR | B | 235 | 15.413 | 9.452 | 63.861 | 1.00 | 45.67 | B | C |
| ATOM | 4189 | CB | THR | B | 235 | 16.909 | 9.490 | 63.636 | 1.00 | 32.74 | B | C |
| ATOM | 4190 | OG1 | THR | B | 235 | 17.254 | 8.556 | 62.608 | 1.00 | 32.74 | B | O |
| ATOM | 4191 | CG2 | THR | B | 235 | 17.631 | 9.094 | 64.896 | 1.00 | 32.74 | B | C |
| ATOM | 4192 | C | THR | B | 235 | 14.717 | 9.506 | 62.505 | 1.00 | 45.67 | B | C |
| ATOM | 4193 | O | THR | B | 235 | 13.889 | 8.650 | 62.194 | 1.00 | 45.67 | B | O |
| ATOM | 4194 | N | SER | B | 236 | 15.054 | 10.497 | 61.689 | 1.00 | 37.65 | B | N |
| ATOM | 4195 | CA | SER | B | 236 | 14.430 | 10.605 | 60.378 | 1.00 | 37.65 | B | C |
| ATOM | 4196 | CB | SER | B | 236 | 14.556 | 12.038 | 59.842 | 1.00 | 35.25 | B | C |
| ATOM | 4197 | OG | SER | B | 236 | 15.905 | 12.373 | 59.579 | 1.00 | 35.25 | B | O |
| ATOM | 4198 | C | SER | B | 236 | 15.024 | 9.602 | 59.382 | 1.00 | 37.65 | B | C |
| ATOM | 4199 | O | SER | B | 236 | 14.610 | 9.529 | 58.227 | 1.00 | 37.65 | B | O |
| ATOM | 4200 | N | SER | B | 237 | 15.988 | 8.811 | 59.827 | 1.00 | 31.55 | B | N |
| ATOM | 4201 | CA | SER | B | 237 | 16.581 | 7.835 | 58.930 | 1.00 | 31.55 | B | C |
| ATOM | 4202 | CB | SER | B | 237 | 17.821 | 7.193 | 59.558 | 1.00 | 40.16 | B | C |
| ATOM | 4203 | OG | SER | B | 237 | 17.537 | 6.638 | 60.831 | 1.00 | 40.16 | B | O |
| ATOM | 4204 | C | SER | B | 237 | 15.540 | 6.778 | 58.602 | 1.00 | 31.55 | B | C |
| ATOM | 4205 | O | SER | B | 237 | 15.759 | 5.932 | 57.754 | 1.00 | 31.55 | B | O |
| ATOM | 4206 | N | ILE | B | 238 | 14.403 | 6.816 | 59.277 | 1.00 | 28.39 | B | N |
| ATOM | 4207 | CA | ILE | B | 238 | 13.361 | 5.850 | 58.979 | 1.00 | 28.39 | B | C |
| ATOM | 4208 | CB | ILE | B | 238 | 12.200 | 5.915 | 60.003 | 1.00 | 38.42 | B | C |
| ATOM | 4209 | CG2 | ILE | B | 238 | 12.677 | 5.444 | 61.358 | 1.00 | 38.42 | B | C |
| ATOM | 4210 | CG1 | ILE | B | 238 | 11.645 | 7.338 | 60.071 | 1.00 | 38.42 | B | C |
| ATOM | 4211 | CD1 | ILE | B | 238 | 10.352 | 7.447 | 60.818 | 1.00 | 38.42 | B | C |
| ATOM | 4212 | C | ILE | B | 238 | 12.806 | 6.133 | 57.580 | 1.00 | 28.39 | B | C |
| ATOM | 4213 | O | ILE | B | 238 | 12.379 | 5.222 | 56.878 | 1.00 | 28.39 | B | O |
| ATOM | 4214 | N | ASP | B | 239 | 12.807 | 7.398 | 57.177 | 1.00 | 32.83 | B | N |

| | | | | | | | | | | | | |
|---|---|---|---|---|---|---|---|---|---|---|---|---|
| ATOM | 4215 | CA | ASP | B | 239 | 12.316 | 7.731 | 55.850 | 1.00 | 32.83 | B | C |
| ATOM | 4216 | CB | ASP | B | 239 | 12.253 | 9.247 | 55.623 | 1.00 | 38.24 | B | C |
| ATOM | 4217 | CG | ASP | B | 239 | 11.101 | 9.911 | 56.346 | 1.00 | 38.24 | B | C |
| ATOM | 4218 | OD1 | ASP | B | 239 | 10.040 | 9.281 | 56.516 | 1.00 | 38.24 | B | O |
| ATOM | 4219 | OD2 | ASP | B | 239 | 11.246 | 11.087 | 56.727 | 1.00 | 38.24 | B | O |
| ATOM | 4220 | C | ASP | B | 239 | 13.243 | 7.122 | 54.807 | 1.00 | 32.83 | B | C |
| ATOM | 4221 | O | ASP | B | 239 | 12.792 | 6.651 | 53.767 | 1.00 | 32.83 | B | O |
| ATOM | 4222 | N | VAL | B | 240 | 14.541 | 7.143 | 55.085 | 1.00 | 32.85 | B | N |
| ATOM | 4223 | CA | VAL | B | 240 | 15.504 | 6.590 | 54.161 | 1.00 | 32.85 | B | C |
| ATOM | 4224 | CB | VAL | B | 240 | 16.922 | 6.930 | 54.584 | 1.00 | 25.84 | B | C |
| ATOM | 4225 | CG1 | VAL | B | 240 | 17.922 | 6.214 | 53.692 | 1.00 | 25.84 | B | C |
| ATOM | 4226 | CG2 | VAL | B | 240 | 17.120 | 8.430 | 54.497 | 1.00 | 25.84 | B | C |
| ATOM | 4227 | C | VAL | B | 240 | 15.334 | 5.084 | 54.031 | 1.00 | 32.85 | B | C |
| ATOM | 4228 | O | VAL | B | 240 | 15.577 | 4.526 | 52.960 | 1.00 | 32.85 | B | O |
| ATOM | 4229 | N | TRP | B | 241 | 14.915 | 4.417 | 55.105 | 1.00 | 37.39 | B | N |
| ATOM | 4230 | CA | TRP | B | 241 | 14.688 | 2.986 | 55.015 | 1.00 | 37.39 | B | C |
| ATOM | 4231 | CB | TRP | B | 241 | 14.377 | 2.378 | 56.379 | 1.00 | 28.29 | B | C |
| ATOM | 4232 | CG | TRP | B | 241 | 13.811 | 0.981 | 56.296 | 1.00 | 28.29 | B | C |
| ATOM | 4233 | CD2 | TRP | B | 241 | 14.529 | -0.255 | 56.419 | 1.00 | 28.29 | B | C |
| ATOM | 4234 | CE2 | TRP | B | 241 | 13.595 | -1.299 | 56.235 | 1.00 | 28.29 | B | C |
| ATOM | 4235 | CE3 | TRP | B | 241 | 15.870 | -0.584 | 56.632 | 1.00 | 28.29 | B | C |
| ATOM | 4236 | CD1 | TRP | B | 241 | 12.511 | 0.641 | 56.071 | 1.00 | 28.29 | B | C |
| ATOM | 4237 | NE1 | TRP | B | 241 | 12.372 | -0.723 | 56.038 | 1.00 | 28.29 | B | N |
| ATOM | 4238 | CZ2 | TRP | B | 241 | 13.956 | -2.645 | 56.300 | 1.00 | 28.29 | B | C |
| ATOM | 4239 | CZ3 | TRP | B | 241 | 16.225 | -1.927 | 56.695 | 1.00 | 28.29 | B | C |
| ATOM | 4240 | CH2 | TRP | B | 241 | 15.272 | -2.937 | 56.514 | 1.00 | 28.29 | B | C |
| ATOM | 4241 | C | TRP | B | 241 | 13.499 | 2.831 | 54.078 | 1.00 | 37.39 | B | C |
| ATOM | 4242 | O | TRP | B | 241 | 13.530 | 2.011 | 53.152 | 1.00 | 37.39 | B | O |
| ATOM | 4243 | N | SER | B | 242 | 12.466 | 3.645 | 54.308 | 1.00 | 30.46 | B | N |
| ATOM | 4244 | CA | SER | B | 242 | 11.252 | 3.630 | 53.484 | 1.00 | 30.46 | B | C |
| ATOM | 4245 | CB | SER | B | 242 | 10.253 | 4.670 | 53.971 | 1.00 | 30.80 | B | C |
| ATOM | 4246 | OG | SER | B | 242 | 9.811 | 4.377 | 55.268 | 1.00 | 30.80 | B | O |
| ATOM | 4247 | C | SER | B | 242 | 11.561 | 3.926 | 52.025 | 1.00 | 30.46 | B | C |
| ATOM | 4248 | O | SER | B | 242 | 10.947 | 3.357 | 51.137 | 1.00 | 30.46 | B | O |
| ATOM | 4249 | N | ALA | B | 243 | 12.502 | 4.830 | 51.786 | 1.00 | 27.92 | B | N |
| ATOM | 4250 | CA | ALA | B | 243 | 12.880 | 5.190 | 50.438 | 1.00 | 27.92 | B | C |
| ATOM | 4251 | CB | ALA | B | 243 | 13.828 | 6.369 | 50.472 | 1.00 | 22.13 | B | C |
| ATOM | 4252 | C | ALA | B | 243 | 13.542 | 4.001 | 49.761 | 1.00 | 27.92 | B | C |
| ATOM | 4253 | O | ALA | B | 243 | 13.323 | 3.734 | 48.583 | 1.00 | 27.92 | B | O |
| ATOM | 4254 | N | GLY | B | 244 | 14.360 | 3.284 | 50.521 | 1.00 | 39.89 | B | N |
| ATOM | 4255 | CA | GLY | B | 244 | 15.044 | 2.135 | 49.972 | 1.00 | 39.89 | B | C |
| ATOM | 4256 | C | GLY | B | 244 | 14.094 | 0.998 | 49.688 | 1.00 | 39.89 | B | C |
| ATOM | 4257 | O | GLY | B | 244 | 14.431 | 0.083 | 48.951 | 1.00 | 39.89 | B | O |
| ATOM | 4258 | N | CYS | B | 245 | 12.911 | 1.037 | 50.287 | 1.00 | 37.07 | B | N |
| ATOM | 4259 | CA | CYS | B | 245 | 11.915 | -0.008 | 50.060 | 1.00 | 37.07 | B | C |
| ATOM | 4260 | CB | CYS | B | 245 | 10.922 | -0.073 | 51.232 | 1.00 | 38.87 | B | C |
| ATOM | 4261 | SG | CYS | B | 245 | 11.510 | -0.970 | 52.704 | 1.00 | 38.87 | B | S |
| ATOM | 4262 | C | CYS | B | 245 | 11.174 | 0.257 | 48.748 | 1.00 | 37.07 | B | C |
| ATOM | 4263 | O | CYS | B | 245 | 10.693 | -0.663 | 48.098 | 1.00 | 37.07 | B | O |
| ATOM | 4264 | N | VAL | B | 246 | 11.088 | 1.527 | 48.368 | 1.00 | 41.56 | B | N |
| ATOM | 4265 | CA | VAL | B | 246 | 10.441 | 1.923 | 47.128 | 1.00 | 41.56 | B | C |
| ATOM | 4266 | CB | VAL | B | 246 | 10.171 | 3.433 | 47.090 | 1.00 | 35.23 | B | C |
| ATOM | 4267 | CG1 | VAL | B | 246 | 9.619 | 3.826 | 45.738 | 1.00 | 35.23 | B | C |
| ATOM | 4268 | CG2 | VAL | B | 246 | 9.212 | 3.819 | 48.188 | 1.00 | 35.23 | B | C |
| ATOM | 4269 | C | VAL | B | 246 | 11.381 | 1.582 | 45.982 | 1.00 | 41.56 | B | C |
| ATOM | 4270 | O | VAL | B | 246 | 10.964 | 1.051 | 44.959 | 1.00 | 41.56 | B | O |
| ATOM | 4271 | N | LEU | B | 247 | 12.657 | 1.896 | 46.152 | 1.00 | 32.86 | B | N |
| ATOM | 4272 | CA | LEU | B | 247 | 13.638 | 1.605 | 45.124 | 1.00 | 32.86 | B | C |
| ATOM | 4273 | CB | LEU | B | 247 | 15.011 | 2.115 | 45.545 | 1.00 | 33.11 | B | C |
| ATOM | 4274 | CG | LEU | B | 247 | 16.158 | 1.620 | 44.668 | 1.00 | 33.11 | B | C |
| ATOM | 4275 | CD1 | LEU | B | 247 | 15.910 | 2.026 | 43.238 | 1.00 | 33.11 | B | C |
| ATOM | 4276 | CD2 | LEU | B | 247 | 17.467 | 2.186 | 45.173 | 1.00 | 33.11 | B | C |
| ATOM | 4277 | C | LEU | B | 247 | 13.710 | 0.112 | 44.868 | 1.00 | 32.86 | B | C |
| ATOM | 4278 | O | LEU | B | 247 | 13.666 | -0.338 | 43.737 | 1.00 | 32.86 | B | O |
| ATOM | 4279 | N | ALA | B | 248 | 13.826 | -0.665 | 45.930 | 1.00 | 36.03 | B | N |
| ATOM | 4280 | CA | ALA | B | 248 | 13.906 | -2.104 | 45.767 | 1.00 | 36.03 | B | C |
| ATOM | 4281 | CB | ALA | B | 248 | 13.967 | -2.780 | 47.131 | 1.00 | 12.02 | B | C |

| | | | | | | | | | | | | |
|---|---|---|---|---|---|---|---|---|---|---|---|---|
| ATOM | 4282 | C | ALA | B | 248 | 12.699 | -2.606 | 44.977 | 1.00 | 36.03 | B | C |
| ATOM | 4283 | O | ALA | B | 248 | 12.835 | -3.436 | 44.086 | 1.00 | 36.03 | B | O |
| ATOM | 4284 | N | GLU | B | 249 | 11.525 | -2.076 | 45.306 | 1.00 | 35.82 | B | N |
| ATOM | 4285 | CA | GLU | B | 249 | 10.276 | -2.474 | 44.675 | 1.00 | 35.82 | B | C |
| ATOM | 4286 | CB | GLU | B | 249 | 9.102 | -1.800 | 45.368 | 1.00 | 36.69 | B | C |
| ATOM | 4287 | CG | GLU | B | 249 | 7.783 | -2.377 | 44.948 | 1.00 | 36.69 | B | C |
| ATOM | 4288 | CD | GLU | B | 249 | 6.607 | -1.837 | 45.732 | 1.00 | 36.69 | B | C |
| ATOM | 4289 | OE1 | GLU | B | 249 | 6.694 | -1.767 | 46.981 | 1.00 | 36.69 | B | O |
| ATOM | 4290 | OE2 | GLU | B | 249 | 5.587 | -1.500 | 45.092 | 1.00 | 36.69 | B | O |
| ATOM | 4291 | C | GLU | B | 249 | 10.223 | -2.151 | 43.203 | 1.00 | 35.82 | B | C |
| ATOM | 4292 | O | GLU | B | 249 | 9.639 | -2.888 | 42.418 | 1.00 | 35.82 | B | O |
| ATOM | 4293 | N | LEU | B | 250 | 10.838 | -1.042 | 42.828 | 1.00 | 38.98 | B | N |
| ATOM | 4294 | CA | LEU | B | 250 | 10.834 | -0.618 | 41.446 | 1.00 | 38.98 | B | C |
| ATOM | 4295 | CB | LEU | B | 250 | 11.215 | 0.853 | 41.373 | 1.00 | 29.76 | B | C |
| ATOM | 4296 | CG | LEU | B | 250 | 10.185 | 1.730 | 42.078 | 1.00 | 29.76 | B | C |
| ATOM | 4297 | CD1 | LEU | B | 250 | 10.688 | 3.149 | 42.122 | 1.00 | 29.76 | B | C |
| ATOM | 4298 | CD2 | LEU | B | 250 | 8.848 | 1.646 | 41.352 | 1.00 | 29.76 | B | C |
| ATOM | 4299 | C | LEU | B | 250 | 11.750 | -1.454 | 40.581 | 1.00 | 38.98 | B | C |
| ATOM | 4300 | O | LEU | B | 250 | 11.520 | -1.592 | 39.384 | 1.00 | 38.98 | B | O |
| ATOM | 4301 | N | LEU | B | 251 | 12.791 | -2.006 | 41.187 | 1.00 | 32.19 | B | N |
| ATOM | 4302 | CA | LEU | B | 251 | 13.734 | -2.847 | 40.471 | 1.00 | 32.19 | B | C |
| ATOM | 4303 | CB | LEU | B | 251 | 15.098 | -2.829 | 41.150 | 1.00 | 36.22 | B | C |
| ATOM | 4304 | CG | LEU | B | 251 | 15.829 | -1.528 | 41.463 | 1.00 | 36.22 | B | C |
| ATOM | 4305 | CD1 | LEU | B | 251 | 17.143 | -1.878 | 42.141 | 1.00 | 36.22 | B | C |
| ATOM | 4306 | CD2 | LEU | B | 251 | 16.081 | -0.728 | 40.210 | 1.00 | 36.22 | B | C |
| ATOM | 4307 | C | LEU | B | 251 | 13.209 | -4.268 | 40.515 | 1.00 | 32.19 | B | C |
| ATOM | 4308 | O | LEU | B | 251 | 13.536 | -5.093 | 39.674 | 1.00 | 32.19 | B | O |
| ATOM | 4309 | N | LEU | B | 252 | 12.385 | -4.535 | 41.516 | 1.00 | 41.40 | B | N |
| ATOM | 4310 | CA | LEU | B | 252 | 11.818 | -5.852 | 41.746 | 1.00 | 41.40 | B | C |
| ATOM | 4311 | CB | LEU | B | 252 | 11.691 | -6.094 | 43.245 | 1.00 | 45.11 | B | C |
| ATOM | 4312 | CG | LEU | B | 252 | 12.455 | -7.269 | 43.849 | 1.00 | 45.11 | B | C |
| ATOM | 4313 | CD1 | LEU | B | 252 | 13.946 | -7.179 | 43.519 | 1.00 | 45.11 | B | C |
| ATOM | 4314 | CD2 | LEU | B | 252 | 12.240 | -7.257 | 45.355 | 1.00 | 45.11 | B | C |
| ATOM | 4315 | C | LEU | B | 252 | 10.470 | -6.088 | 41.113 | 1.00 | 41.40 | B | C |
| ATOM | 4316 | O | LEU | B | 252 | 10.135 | -7.223 | 40.792 | 1.00 | 41.40 | B | O |
| ATOM | 4317 | N | GLY | B | 253 | 9.680 | -5.034 | 40.957 | 1.00 | 36.56 | B | N |
| ATOM | 4318 | CA | GLY | B | 253 | 8.367 | -5.195 | 40.365 | 1.00 | 36.56 | B | C |
| ATOM | 4319 | C | GLY | B | 253 | 7.325 | -5.597 | 41.390 | 1.00 | 36.56 | B | C |
| ATOM | 4320 | O | GLY | B | 253 | 6.169 | -5.816 | 41.051 | 1.00 | 36.56 | B | O |
| ATOM | 4321 | N | GLN | B | 254 | 7.736 | -5.701 | 42.647 | 1.00 | 39.83 | B | N |
| ATOM | 4322 | CA | GLN | B | 254 | 6.836 | -6.076 | 43.734 | 1.00 | 39.83 | B | C |
| ATOM | 4323 | CB | GLN | B | 254 | 6.594 | -7.589 | 43.718 | 1.00 | 43.57 | B | C |
| ATOM | 4324 | CG | GLN | B | 254 | 7.871 | -8.397 | 43.646 | 1.00 | 43.57 | B | C |
| ATOM | 4325 | CD | GLN | B | 254 | 7.682 | -9.836 | 44.054 | 1.00 | 43.57 | B | C |
| ATOM | 4326 | OE1 | GLN | B | 254 | 7.503 | -10.153 | 45.234 | 1.00 | 43.57 | B | O |
| ATOM | 4327 | NE2 | GLN | B | 254 | 7.720 | -10.722 | 43.077 | 1.00 | 43.57 | B | N |
| ATOM | 4328 | C | GLN | B | 254 | 7.475 | -5.668 | 45.063 | 1.00 | 39.83 | B | C |
| ATOM | 4329 | O | GLN | B | 254 | 8.698 | -5.560 | 45.161 | 1.00 | 39.83 | B | O |
| ATOM | 4330 | N | PRO | B | 255 | 6.660 | -5.430 | 46.102 | 1.00 | 42.09 | B | N |
| ATOM | 4331 | CD | PRO | B | 255 | 5.193 | -5.447 | 46.193 | 1.00 | 31.78 | B | C |
| ATOM | 4332 | CA | PRO | B | 255 | 7.245 | -5.039 | 47.385 | 1.00 | 42.09 | B | C |
| ATOM | 4333 | CB | PRO | B | 255 | 6.043 | -5.062 | 48.323 | 1.00 | 31.78 | B | C |
| ATOM | 4334 | CG | PRO | B | 255 | 4.942 | -4.623 | 47.436 | 1.00 | 31.78 | B | C |
| ATOM | 4335 | C | PRO | B | 255 | 8.352 | -6.004 | 47.808 | 1.00 | 42.09 | B | C |
| ATOM | 4336 | O | PRO | B | 255 | 8.208 | -7.216 | 47.676 | 1.00 | 42.09 | B | O |
| ATOM | 4337 | N | ILE | B | 256 | 9.458 | -5.454 | 48.301 | 1.00 | 40.78 | B | N |
| ATOM | 4338 | CA | ILE | B | 256 | 10.587 | -6.263 | 48.736 | 1.00 | 40.78 | B | C |
| ATOM | 4339 | CB | ILE | B | 256 | 11.854 | -5.380 | 48.925 | 1.00 | 32.43 | B | C |
| ATOM | 4340 | CG2 | ILE | B | 256 | 11.553 | -4.221 | 49.850 | 1.00 | 32.43 | B | C |
| ATOM | 4341 | CG1 | ILE | B | 256 | 13.013 | -6.213 | 49.460 | 1.00 | 32.43 | B | C |
| ATOM | 4342 | CD1 | ILE | B | 256 | 13.645 | -7.083 | 48.447 | 1.00 | 32.43 | B | C |
| ATOM | 4343 | C | ILE | B | 256 | 10.274 | -7.039 | 50.022 | 1.00 | 40.78 | B | C |
| ATOM | 4344 | O | ILE | B | 256 | 10.624 | -8.218 | 50.133 | 1.00 | 40.78 | B | O |
| ATOM | 4345 | N | PHE | B | 257 | 9.615 | -6.392 | 50.984 | 1.00 | 34.25 | B | N |
| ATOM | 4346 | CA | PHE | B | 257 | 9.263 | -7.049 | 52.250 | 1.00 | 34.25 | B | C |
| ATOM | 4347 | CB | PHE | B | 257 | 9.976 | -6.396 | 53.446 | 1.00 | 36.22 | B | C |
| ATOM | 4348 | CG | PHE | B | 257 | 11.453 | -6.200 | 53.264 | 1.00 | 36.22 | B | C |

| | | | | | | | | | | | | |
|---|---|---|---|---|---|---|---|---|---|---|---|---|
| ATOM | 4349 | CD1 | PHE | B | 257 | 12.282 | -7.267 | 52.981 | 1.00 | 36.22 | B | C |
| ATOM | 4350 | CD2 | PHE | B | 257 | 12.016 | -4.933 | 53.403 | 1.00 | 36.22 | B | C |
| ATOM | 4351 | CE1 | PHE | B | 257 | 13.653 | -7.075 | 52.837 | 1.00 | 36.22 | B | C |
| ATOM | 4352 | CE2 | PHE | B | 257 | 13.383 | -4.731 | 53.262 | 1.00 | 36.22 | B | C |
| ATOM | 4353 | CZ | PHE | B | 257 | 14.205 | -5.801 | 52.978 | 1.00 | 36.22 | B | C |
| ATOM | 4354 | C | PHE | B | 257 | 7.754 | -6.955 | 52.491 | 1.00 | 34.25 | B | C |
| ATOM | 4355 | O | PHE | B | 257 | 7.280 | -6.005 | 53.104 | 1.00 | 34.25 | B | O |
| ATOM | 4356 | N | PRO | B | 258 | 6.982 | -7.949 | 52.020 | 1.00 | 42.22 | B | N |
| ATOM | 4357 | CD | PRO | B | 258 | 7.426 | -8.975 | 51.057 | 1.00 | 32.14 | B | C |
| ATOM | 4358 | CA | PRO | B | 258 | 5.522 | -7.996 | 52.169 | 1.00 | 42.22 | B | C |
| ATOM | 4359 | CB | PRO | B | 258 | 5.090 | -8.705 | 50.897 | 1.00 | 32.14 | B | C |
| ATOM | 4360 | CG | PRO | B | 258 | 6.142 | -9.749 | 50.779 | 1.00 | 32.14 | B | C |
| ATOM | 4361 | C | PRO | B | 258 | 5.000 | -8.721 | 53.410 | 1.00 | 42.22 | B | C |
| ATOM | 4362 | O | PRO | B | 258 | 4.343 | -9.755 | 53.292 | 1.00 | 42.22 | B | O |
| ATOM | 4363 | N | GLY | B | 259 | 5.273 | -8.180 | 54.592 | 1.00 | 45.98 | B | N |
| ATOM | 4364 | CA | GLY | B | 259 | 4.818 | -8.822 | 55.811 | 1.00 | 45.98 | B | C |
| ATOM | 4365 | C | GLY | B | 259 | 3.308 | -8.929 | 55.896 | 1.00 | 45.98 | B | C |
| ATOM | 4366 | O | GLY | B | 259 | 2.603 | -8.003 | 55.487 | 1.00 | 45.98 | B | O |
| ATOM | 4367 | N | ASP | B | 260 | 2.791 | -10.044 | 56.416 | 1.00 | 57.20 | B | N |
| ATOM | 4368 | CA | ASP | B | 260 | 1.340 | -10.173 | 56.526 | 1.00 | 57.20 | B | C |
| ATOM | 4369 | CB | ASP | B | 260 | 0.889 | -11.647 | 56.486 | 1.00 | 85.73 | B | C |
| ATOM | 4370 | CG | ASP | B | 260 | 1.242 | -12.413 | 57.755 | 1.00 | 85.73 | B | C |
| ATOM | 4371 | OD1 | ASP | B | 260 | 1.264 | -11.785 | 58.842 | 1.00 | 85.73 | B | O |
| ATOM | 4372 | OD2 | ASP | B | 260 | 1.473 | -13.649 | 57.661 | 1.00 | 85.73 | B | O |
| ATOM | 4373 | C | ASP | B | 260 | 0.844 | -9.500 | 57.804 | 1.00 | 57.20 | B | C |
| ATOM | 4374 | O | ASP | B | 260 | -0.352 | -9.396 | 58.035 | 1.00 | 57.20 | B | O |
| ATOM | 4375 | N | SER | B | 261 | 1.770 | -9.047 | 58.638 | 1.00 | 49.50 | B | N |
| ATOM | 4376 | CA | SER | B | 261 | 1.403 | -8.362 | 59.872 | 1.00 | 49.50 | B | C |
| ATOM | 4377 | CB | SER | B | 261 | 0.969 | -9.363 | 60.938 | 1.00 | 56.87 | B | C |
| ATOM | 4378 | OG | SER | B | 261 | 2.090 | -10.062 | 61.451 | 1.00 | 56.87 | B | O |
| ATOM | 4379 | C | SER | B | 261 | 2.604 | -7.565 | 60.371 | 1.00 | 49.50 | B | C |
| ATOM | 4380 | O | SER | B | 261 | 3.728 | -7.775 | 59.914 | 1.00 | 49.50 | B | O |
| ATOM | 4381 | N | GLY | B | 262 | 2.356 | -6.647 | 61.302 | 1.00 | 42.57 | B | N |
| ATOM | 4382 | CA | GLY | B | 262 | 3.416 | -5.819 | 61.853 | 1.00 | 42.57 | B | C |
| ATOM | 4383 | C | GLY | B | 262 | 4.643 | -6.596 | 62.286 | 1.00 | 42.57 | B | C |
| ATOM | 4384 | O | GLY | B | 262 | 5.747 | -6.077 | 62.229 | 1.00 | 42.57 | B | O |
| ATOM | 4385 | N | VAL | B | 263 | 4.460 | -7.839 | 62.717 | 1.00 | 48.41 | B | N |
| ATOM | 4386 | CA | VAL | B | 263 | 5.580 | -8.668 | 63.163 | 1.00 | 48.41 | B | C |
| ATOM | 4387 | CB | VAL | B | 263 | 5.173 | -9.556 | 64.378 | 1.00 | 52.14 | B | C |
| ATOM | 4388 | CG1 | VAL | B | 263 | 6.263 | -10.575 | 64.681 | 1.00 | 52.14 | B | C |
| ATOM | 4389 | CG2 | VAL | B | 263 | 4.931 | -8.681 | 65.605 | 1.00 | 52.14 | B | C |
| ATOM | 4390 | C | VAL | B | 263 | 6.135 | -9.550 | 62.038 | 1.00 | 48.41 | B | C |
| ATOM | 4391 | O | VAL | B | 263 | 7.347 | -9.747 | 61.941 | 1.00 | 48.41 | B | O |
| ATOM | 4392 | N | ASP | B | 264 | 5.257 | -10.088 | 61.195 | 1.00 | 49.46 | B | N |
| ATOM | 4393 | CA | ASP | B | 264 | 5.698 | -10.916 | 60.065 | 1.00 | 49.46 | B | C |
| ATOM | 4394 | CB | ASP | B | 264 | 4.480 | -11.358 | 59.226 | 1.00 | 51.07 | B | C |
| ATOM | 4395 | CG | ASP | B | 264 | 4.856 | -12.273 | 58.046 | 1.00 | 51.07 | B | C |
| ATOM | 4396 | OD1 | ASP | B | 264 | 4.093 | -12.285 | 57.052 | 1.00 | 51.07 | B | O |
| ATOM | 4397 | OD2 | ASP | B | 264 | 5.894 | -12.982 | 58.107 | 1.00 | 51.07 | B | O |
| ATOM | 4398 | C | ASP | B | 264 | 6.631 | -10.035 | 59.216 | 1.00 | 49.46 | B | C |
| ATOM | 4399 | O | ASP | B | 264 | 7.560 | -10.525 | 58.572 | 1.00 | 49.46 | B | O |
| ATOM | 4400 | N | GLN | B | 265 | 6.367 | -8.728 | 59.236 | 1.00 | 32.91 | B | N |
| ATOM | 4401 | CA | GLN | B | 265 | 7.146 | -7.751 | 58.487 | 1.00 | 32.91 | B | C |
| ATOM | 4402 | CB | GLN | B | 265 | 6.688 | -6.332 | 58.850 | 1.00 | 37.01 | B | C |
| ATOM | 4403 | CG | GLN | B | 265 | 7.316 | -5.223 | 58.014 | 1.00 | 37.01 | B | C |
| ATOM | 4404 | CD | GLN | B | 265 | 7.175 | -5.485 | 56.534 | 1.00 | 37.01 | B | C |
| ATOM | 4405 | OE1 | GLN | B | 265 | 6.232 | -6.140 | 56.108 | 1.00 | 37.01 | B | O |
| ATOM | 4406 | NE2 | GLN | B | 265 | 8.104 | -4.968 | 55.742 | 1.00 | 37.01 | B | N |
| ATOM | 4407 | C | GLN | B | 265 | 8.630 | -7.903 | 58.784 | 1.00 | 32.91 | B | C |
| ATOM | 4408 | O | GLN | B | 265 | 9.470 | -7.887 | 57.881 | 1.00 | 32.91 | B | O |
| ATOM | 4409 | N | LEU | B | 266 | 8.944 | -8.037 | 60.063 | 1.00 | 47.01 | B | N |
| ATOM | 4410 | CA | LEU | B | 266 | 10.321 | -8.196 | 60.486 | 1.00 | 47.01 | B | C |
| ATOM | 4411 | CB | LEU | B | 266 | 10.385 | -8.221 | 62.012 | 1.00 | 38.05 | B | C |
| ATOM | 4412 | CG | LEU | B | 266 | 11.740 | -8.186 | 62.719 | 1.00 | 38.05 | B | C |
| ATOM | 4413 | CD1 | LEU | B | 266 | 12.628 | -7.087 | 62.129 | 1.00 | 38.05 | B | C |
| ATOM | 4414 | CD2 | LEU | B | 266 | 11.500 | -7.945 | 64.197 | 1.00 | 38.05 | B | C |
| ATOM | 4415 | C | LEU | B | 266 | 10.872 | -9.493 | 59.891 | 1.00 | 47.01 | B | C |

| | | | | | | | | | | | | |
|---|---|---|---|---|---|---|---|---|---|---|---|---|
| ATOM | 4416 | O | LEU | B | 266 | 11.987 | -9.520 | 59.350 | 1.00 | 47.01 | B | O |
| ATOM | 4417 | N | VAL | B | 267 | 10.084 | -10.562 | 59.972 | 1.00 | 61.36 | B | N |
| ATOM | 4418 | CA | VAL | B | 267 | 10.507 | -11.845 | 59.430 | 1.00 | 61.36 | B | C |
| ATOM | 4419 | CB | VAL | B | 267 | 9.389 | -12.909 | 59.538 | 1.00 | 35.86 | B | C |
| ATOM | 4420 | CG1 | VAL | B | 267 | 9.855 | -14.227 | 58.927 | 1.00 | 35.86 | B | C |
| ATOM | 4421 | CG2 | VAL | B | 267 | 9.001 | -13.104 | 60.988 | 1.00 | 35.86 | B | C |
| ATOM | 4422 | C | VAL | B | 267 | 10.910 | -11.688 | 57.963 | 1.00 | 61.36 | B | C |
| ATOM | 4423 | O | VAL | B | 267 | 12.032 | -12.033 | 57.590 | 1.00 | 61.36 | B | O |
| ATOM | 4424 | N | GLU | B | 268 | 10.008 | -11.162 | 57.137 | 1.00 | 35.56 | B | N |
| ATOM | 4425 | CA | GLU | B | 268 | 10.295 | -10.963 | 55.718 | 1.00 | 35.56 | B | C |
| ATOM | 4426 | CB | GLU | B | 268 | 9.151 | -10.186 | 55.054 | 1.00 | 52.64 | B | C |
| ATOM | 4427 | CG | GLU | B | 268 | 7.842 | -10.968 | 54.895 | 1.00 | 52.64 | B | C |
| ATOM | 4428 | CD | GLU | B | 268 | 7.987 | -12.190 | 53.983 | 1.00 | 52.64 | B | C |
| ATOM | 4429 | OE1 | GLU | B | 268 | 8.418 | -12.031 | 52.816 | 1.00 | 52.64 | B | O |
| ATOM | 4430 | OE2 | GLU | B | 268 | 7.663 | -13.314 | 54.434 | 1.00 | 52.64 | B | O |
| ATOM | 4431 | C | GLU | B | 268 | 11.612 | -10.216 | 55.518 | 1.00 | 35.56 | B | C |
| ATOM | 4432 | O | GLU | B | 268 | 12.412 | -10.581 | 54.660 | 1.00 | 35.56 | B | O |
| ATOM | 4433 | N | ILE | B | 269 | 11.821 | -9.165 | 56.315 | 1.00 | 47.73 | B | N |
| ATOM | 4434 | CA | ILE | B | 269 | 13.042 | -8.356 | 56.262 | 1.00 | 47.73 | B | C |
| ATOM | 4435 | CB | ILE | B | 269 | 13.025 | -7.226 | 57.324 | 1.00 | 32.01 | B | C |
| ATOM | 4436 | CG2 | ILE | B | 269 | 14.432 | -6.711 | 57.560 | 1.00 | 32.01 | B | C |
| ATOM | 4437 | CG1 | ILE | B | 269 | 12.113 | -6.090 | 56.869 | 1.00 | 32.01 | B | C |
| ATOM | 4438 | CD1 | ILE | B | 269 | 12.026 | -4.947 | 57.846 | 1.00 | 32.01 | B | C |
| ATOM | 4439 | C | ILE | B | 269 | 14.246 | -9.242 | 56.543 | 1.00 | 47.73 | B | C |
| ATOM | 4440 | O | ILE | B | 269 | 15.234 | -9.243 | 55.797 | 1.00 | 47.73 | B | O |
| ATOM | 4441 | N | ILE | B | 270 | 14.146 | -9.984 | 57.641 | 1.00 | 45.98 | B | N |
| ATOM | 4442 | CA | ILE | B | 270 | 15.200 | -10.894 | 58.063 | 1.00 | 45.98 | B | C |
| ATOM | 4443 | CB | ILE | B | 270 | 14.802 | -11.591 | 59.392 | 1.00 | 38.43 | B | C |
| ATOM | 4444 | CG2 | ILE | B | 270 | 15.673 | -12.817 | 59.642 | 1.00 | 38.43 | B | C |
| ATOM | 4445 | CG1 | ILE | B | 270 | 14.928 | -10.589 | 60.542 | 1.00 | 38.43 | B | C |
| ATOM | 4446 | CD1 | ILE | B | 270 | 14.376 | -11.081 | 61.857 | 1.00 | 38.43 | B | C |
| ATOM | 4447 | C | ILE | B | 270 | 15.516 | -11.941 | 56.992 | 1.00 | 45.98 | B | C |
| ATOM | 4448 | O | ILE | B | 270 | 16.642 | -12.429 | 56.897 | 1.00 | 45.98 | B | O |
| ATOM | 4449 | N | LYS | B | 271 | 14.526 | -12.268 | 56.170 | 1.00 | 51.03 | B | N |
| ATOM | 4450 | CA | LYS | B | 271 | 14.721 | -13.268 | 55.129 | 1.00 | 51.03 | B | C |
| ATOM | 4451 | CB | LYS | B | 271 | 13.385 | -13.630 | 54.499 | 1.00 | 48.51 | B | C |
| ATOM | 4452 | CG | LYS | B | 271 | 12.458 | -14.291 | 55.497 | 1.00 | 48.51 | B | C |
| ATOM | 4453 | CD | LYS | B | 271 | 11.033 | -14.442 | 54.972 | 1.00 | 48.51 | B | C |
| ATOM | 4454 | CE | LYS | B | 271 | 10.963 | -15.425 | 53.821 | 1.00 | 48.51 | B | C |
| ATOM | 4455 | NZ | LYS | B | 271 | 9.564 | -15.631 | 53.366 | 1.00 | 48.51 | B | N |
| ATOM | 4456 | C | LYS | B | 271 | 15.728 | -12.872 | 54.065 | 1.00 | 51.03 | B | C |
| ATOM | 4457 | O | LYS | B | 271 | 16.295 | -13.733 | 53.409 | 1.00 | 51.03 | B | O |
| ATOM | 4458 | N | VAL | B | 272 | 15.968 | -11.582 | 53.886 | 1.00 | 40.60 | B | N |
| ATOM | 4459 | CA | VAL | B | 272 | 16.956 | -11.176 | 52.897 | 1.00 | 40.60 | B | C |
| ATOM | 4460 | CB | VAL | B | 272 | 16.380 | -10.142 | 51.867 | 1.00 | 38.82 | B | C |
| ATOM | 4461 | CG1 | VAL | B | 272 | 14.970 | -10.513 | 51.484 | 1.00 | 38.82 | B | C |
| ATOM | 4462 | CG2 | VAL | B | 272 | 16.419 | -8.747 | 52.428 | 1.00 | 38.82 | B | C |
| ATOM | 4463 | C | VAL | B | 272 | 18.183 | -10.587 | 53.608 | 1.00 | 40.60 | B | C |
| ATOM | 4464 | O | VAL | B | 272 | 19.321 | -10.917 | 53.273 | 1.00 | 40.60 | B | O |
| ATOM | 4465 | N | LEU | B | 273 | 17.955 | -9.736 | 54.607 | 1.00 | 50.54 | B | N |
| ATOM | 4466 | CA | LEU | B | 273 | 19.066 | -9.127 | 55.326 | 1.00 | 50.54 | B | C |
| ATOM | 4467 | CB | LEU | B | 273 | 18.604 | -7.868 | 56.080 | 1.00 | 41.36 | B | C |
| ATOM | 4468 | CG | LEU | B | 273 | 17.903 | -6.701 | 55.364 | 1.00 | 41.36 | B | C |
| ATOM | 4469 | CD1 | LEU | B | 273 | 17.881 | -5.515 | 56.323 | 1.00 | 41.36 | B | C |
| ATOM | 4470 | CD2 | LEU | B | 273 | 18.617 | -6.308 | 54.086 | 1.00 | 41.36 | B | C |
| ATOM | 4471 | C | LEU | B | 273 | 19.708 | -10.103 | 56.313 | 1.00 | 50.54 | B | C |
| ATOM | 4472 | O | LEU | B | 273 | 20.863 | -9.926 | 56.718 | 1.00 | 50.54 | B | O |
| ATOM | 4473 | N | GLY | B | 274 | 18.962 | -11.135 | 56.695 | 1.00 | 58.41 | B | N |
| ATOM | 4474 | CA | GLY | B | 274 | 19.479 | -12.104 | 57.648 | 1.00 | 58.41 | B | C |
| ATOM | 4475 | C | GLY | B | 274 | 19.266 | -11.619 | 59.074 | 1.00 | 58.41 | B | C |
| ATOM | 4476 | O | GLY | B | 274 | 19.039 | -10.428 | 59.297 | 1.00 | 58.41 | B | O |
| ATOM | 4477 | N | THR | B | 275 | 19.331 | -12.528 | 60.045 | 1.00 | 52.95 | B | N |
| ATOM | 4478 | CA | THR | B | 275 | 19.141 | -12.153 | 61.445 | 1.00 | 52.95 | B | C |
| ATOM | 4479 | CB | THR | B | 275 | 19.414 | -13.344 | 62.365 | 1.00 | 46.17 | B | C |
| ATOM | 4480 | OG1 | THR | B | 275 | 18.563 | -14.439 | 61.992 | 1.00 | 46.17 | B | O |
| ATOM | 4481 | CG2 | THR | B | 275 | 19.139 | -12.966 | 63.807 | 1.00 | 46.17 | B | C |
| ATOM | 4482 | C | THR | B | 275 | 20.059 | -10.987 | 61.842 | 1.00 | 52.95 | B | C |

| | | | | | | | | | | | | |
|---|---|---|---|---|---|---|---|---|---|---|---|---|
| ATOM | 4483 | O | THR | B | 275 | 21.242 | -10.972 | 61.505 | 1.00 | 52.95 | B | O |
| ATOM | 4484 | N | PRO | B | 276 | 19.521 | -9.991 | 62.564 | 1.00 | 42.77 | B | N |
| ATOM | 4485 | CD | PRO | B | 276 | 18.168 | -9.888 | 63.126 | 1.00 | 27.79 | B | C |
| ATOM | 4486 | CA | PRO | B | 276 | 20.342 | -8.845 | 62.970 | 1.00 | 42.77 | B | C |
| ATOM | 4487 | CB | PRO | B | 276 | 19.312 | -7.825 | 63.455 | 1.00 | 27.79 | B | C |
| ATOM | 4488 | CG | PRO | B | 276 | 17.951 | -8.425 | 63.101 | 1.00 | 27.79 | B | C |
| ATOM | 4489 | C | PRO | B | 276 | 21.304 | -9.226 | 64.088 | 1.00 | 42.77 | B | C |
| ATOM | 4490 | O | PRO | B | 276 | 20.949 | -10.012 | 64.970 | 1.00 | 42.77 | B | O |
| ATOM | 4491 | N | THR | B | 277 | 22.511 | -8.667 | 64.066 | 1.00 | 47.00 | B | N |
| ATOM | 4492 | CA | THR | B | 277 | 23.490 | -8.962 | 65.111 | 1.00 | 47.00 | B | C |
| ATOM | 4493 | CB | THR | B | 277 | 24.873 | -8.402 | 64.768 | 1.00 | 47.52 | B | C |
| ATOM | 4494 | OG1 | THR | B | 277 | 24.901 | -6.998 | 65.044 | 1.00 | 47.52 | B | O |
| ATOM | 4495 | CG2 | THR | B | 277 | 25.182 | -8.604 | 63.313 | 1.00 | 47.52 | B | C |
| ATOM | 4496 | C | THR | B | 277 | 23.058 | -8.299 | 66.419 | 1.00 | 47.00 | B | C |
| ATOM | 4497 | O | THR | B | 277 | 22.225 | -7.388 | 66.419 | 1.00 | 47.00 | B | O |
| ATOM | 4498 | N | ALA | B | 278 | 23.651 | -8.733 | 67.530 | 1.00 | 46.03 | B | N |
| ATOM | 4499 | CA | ALA | B | 278 | 23.326 | -8.158 | 68.832 | 1.00 | 46.03 | B | C |
| ATOM | 4500 | CB | ALA | B | 278 | 24.235 | -8.766 | 69.912 | 1.00 | 48.64 | B | C |
| ATOM | 4501 | C | ALA | B | 278 | 23.518 | -6.638 | 68.765 | 1.00 | 46.03 | B | C |
| ATOM | 4502 | O | ALA | B | 278 | 22.646 | -5.856 | 69.173 | 1.00 | 46.03 | B | O |
| ATOM | 4503 | N | GLU | B | 279 | 24.672 | -6.241 | 68.237 | 1.00 | 56.62 | B | N |
| ATOM | 4504 | CA | GLU | B | 279 | 25.027 | -4.837 | 68.085 | 1.00 | 56.62 | B | C |
| ATOM | 4505 | CB | GLU | B | 279 | 26.322 | -4.708 | 67.285 | 1.00 | 92.84 | B | C |
| ATOM | 4506 | CG | GLU | B | 279 | 27.533 | -5.432 | 67.893 | 1.00 | 92.84 | B | C |
| ATOM | 4507 | CD | GLU | B | 279 | 27.252 | -6.898 | 68.286 | 1.00 | 92.84 | B | C |
| ATOM | 4508 | OE1 | GLU | B | 279 | 27.115 | -7.162 | 69.515 | 1.00 | 92.84 | B | O |
| ATOM | 4509 | OE2 | GLU | B | 279 | 27.165 | -7.771 | 67.378 | 1.00 | 92.84 | B | O |
| ATOM | 4510 | C | GLU | B | 279 | 23.896 | -4.157 | 67.331 | 1.00 | 56.62 | B | C |
| ATOM | 4511 | O | GLU | B | 279 | 23.199 | -3.299 | 67.886 | 1.00 | 56.62 | B | O |
| ATOM | 4512 | N | GLN | B | 280 | 23.727 | -4.552 | 66.066 | 1.00 | 49.32 | B | N |
| ATOM | 4513 | CA | GLN | B | 280 | 22.671 | -4.030 | 65.198 | 1.00 | 49.32 | B | C |
| ATOM | 4514 | CB | GLN | B | 280 | 22.436 | -4.992 | 64.028 | 1.00 | 42.19 | B | C |
| ATOM | 4515 | CG | GLN | B | 280 | 23.384 | -4.790 | 62.851 | 1.00 | 42.19 | B | C |
| ATOM | 4516 | CD | GLN | B | 280 | 23.237 | -5.871 | 61.799 | 1.00 | 42.19 | B | C |
| ATOM | 4517 | OE1 | GLN | B | 280 | 23.698 | -5.723 | 60.660 | 1.00 | 42.19 | B | O |
| ATOM | 4518 | NE2 | GLN | B | 280 | 22.600 | -6.980 | 62.180 | 1.00 | 42.19 | B | N |
| ATOM | 4519 | C | GLN | B | 280 | 21.366 | -3.826 | 65.961 | 1.00 | 49.32 | B | C |
| ATOM | 4520 | O | GLN | B | 280 | 20.835 | -2.717 | 66.020 | 1.00 | 49.32 | B | O |
| ATOM | 4521 | N | ILE | B | 281 | 20.850 | -4.900 | 66.541 | 1.00 | 49.78 | B | N |
| ATOM | 4522 | CA | ILE | B | 281 | 19.618 | -4.815 | 67.312 | 1.00 | 49.78 | B | C |
| ATOM | 4523 | CB | ILE | B | 281 | 19.206 | -6.203 | 67.882 | 1.00 | 45.29 | B | C |
| ATOM | 4524 | CG2 | ILE | B | 281 | 18.097 | -6.040 | 68.915 | 1.00 | 45.29 | B | C |
| ATOM | 4525 | CG1 | ILE | B | 281 | 18.742 | -7.117 | 66.741 | 1.00 | 45.29 | B | C |
| ATOM | 4526 | CD1 | ILE | B | 281 | 18.181 | -8.460 | 67.186 | 1.00 | 45.29 | B | C |
| ATOM | 4527 | C | ILE | B | 281 | 19.748 | -3.820 | 68.473 | 1.00 | 49.78 | B | C |
| ATOM | 4528 | O | ILE | B | 281 | 18.743 | -3.427 | 69.061 | 1.00 | 49.78 | B | O |
| ATOM | 4529 | N | ALA | B | 282 | 20.978 | -3.408 | 68.793 | 1.00 | 53.14 | B | N |
| ATOM | 4530 | CA | ALA | B | 282 | 21.214 | -2.461 | 69.891 | 1.00 | 53.14 | B | C |
| ATOM | 4531 | CB | ALA | B | 282 | 22.588 | -2.714 | 70.533 | 1.00 | 71.09 | B | C |
| ATOM | 4532 | C | ALA | B | 282 | 21.100 | -0.994 | 69.476 | 1.00 | 53.14 | B | C |
| ATOM | 4533 | O | ALA | B | 282 | 20.740 | -0.139 | 70.290 | 1.00 | 53.14 | B | O |
| ATOM | 4534 | N | GLU | B | 283 | 21.410 | -0.694 | 68.222 | 1.00 | 40.56 | B | N |
| ATOM | 4535 | CA | GLU | B | 283 | 21.322 | 0.683 | 67.755 | 1.00 | 40.56 | B | C |
| ATOM | 4536 | CB | GLU | B | 283 | 22.331 | 0.919 | 66.639 | 1.00 | 68.04 | B | C |
| ATOM | 4537 | CG | GLU | B | 283 | 23.723 | 0.421 | 66.982 | 1.00 | 68.04 | B | C |
| ATOM | 4538 | CD | GLU | B | 283 | 24.822 | 1.344 | 66.462 | 1.00 | 68.04 | B | C |
| ATOM | 4539 | OE1 | GLU | B | 283 | 25.715 | 0.863 | 65.708 | 1.00 | 68.04 | B | O |
| ATOM | 4540 | OE2 | GLU | B | 283 | 24.785 | 2.554 | 66.816 | 1.00 | 68.04 | B | O |
| ATOM | 4541 | C | GLU | B | 283 | 19.904 | 1.032 | 67.282 | 1.00 | 40.56 | B | C |
| ATOM | 4542 | O | GLU | B | 283 | 19.633 | 2.157 | 66.849 | 1.00 | 40.56 | B | O |
| ATOM | 4543 | N | MET | B | 284 | 19.007 | 0.055 | 67.384 | 1.00 | 49.75 | B | N |
| ATOM | 4544 | CA | MET | B | 284 | 17.617 | 0.230 | 67.004 | 1.00 | 49.75 | B | C |
| ATOM | 4545 | CB | MET | B | 284 | 17.107 | -1.018 | 66.257 | 1.00 | 52.86 | B | C |
| ATOM | 4546 | CG | MET | B | 284 | 17.624 | -1.183 | 64.807 | 1.00 | 52.86 | B | C |
| ATOM | 4547 | SD | MET | B | 284 | 17.371 | -2.860 | 64.070 | 1.00 | 52.86 | B | S |
| ATOM | 4548 | CE | MET | B | 284 | 15.705 | -2.803 | 63.774 | 1.00 | 52.86 | B | C |
| ATOM | 4549 | C | MET | B | 284 | 16.773 | 0.461 | 68.260 | 1.00 | 49.75 | B | C |

| | | | | | | | | | | | | |
|---|---|---|---|---|---|---|---|---|---|---|---|---|
| ATOM | 4550 | O | MET | B | 284 | 15.562 | 0.255 | 68.240 | 1.00 | 49.75 | B | O |
| ATOM | 4551 | N | GLY | B | 285 | 17.404 | 0.883 | 69.356 | 1.00 | 48.80 | B | N |
| ATOM | 4552 | CA | GLY | B | 285 | 16.661 | 1.111 | 70.590 | 1.00 | 48.80 | B | C |
| ATOM | 4553 | C | GLY | B | 285 | 15.872 | -0.116 | 71.028 | 1.00 | 48.80 | B | C |
| ATOM | 4554 | O | GLY | B | 285 | 16.293 | -1.260 | 70.812 | 1.00 | 48.80 | B | O |
| ATOM | 4555 | N | ALA | B | 296 | 17.466 | -16.972 | 58.731 | 1.00 | 53.57 | B | N |
| ATOM | 4556 | CA | ALA | B | 296 | 18.559 | -16.598 | 59.620 | 1.00 | 53.57 | B | C |
| ATOM | 4557 | CB | ALA | B | 296 | 18.891 | -17.773 | 60.570 | 1.00 | 21.86 | B | C |
| ATOM | 4558 | C | ALA | B | 296 | 19.817 | -16.147 | 58.855 | 1.00 | 53.57 | B | C |
| ATOM | 4559 | O | ALA | B | 296 | 20.500 | -15.215 | 59.284 | 1.00 | 53.57 | B | O |
| ATOM | 4560 | N | ALA | B | 297 | 20.125 | -16.784 | 57.725 | 1.00 | 66.06 | B | N |
| ATOM | 4561 | CA | ALA | B | 297 | 21.321 | -16.405 | 56.957 | 1.00 | 66.06 | B | C |
| ATOM | 4562 | CB | ALA | B | 297 | 21.935 | -17.643 | 56.293 | 1.00 | 40.80 | B | C |
| ATOM | 4563 | C | ALA | B | 297 | 21.045 | -15.314 | 55.907 | 1.00 | 66.06 | B | C |
| ATOM | 4564 | O | ALA | B | 297 | 20.015 | -15.340 | 55.223 | 1.00 | 66.06 | B | O |
| ATOM | 4565 | N | ALA | B | 298 | 21.975 | -14.368 | 55.777 | 1.00 | 36.23 | B | N |
| ATOM | 4566 | CA | ALA | B | 298 | 21.827 | -13.256 | 54.843 | 1.00 | 36.23 | B | C |
| ATOM | 4567 | CB | ALA | B | 298 | 22.861 | -12.190 | 55.144 | 1.00 | 60.82 | B | C |
| ATOM | 4568 | C | ALA | B | 298 | 21.911 | -13.652 | 53.375 | 1.00 | 36.23 | B | C |
| ATOM | 4569 | O | ALA | B | 298 | 22.878 | -14.274 | 52.931 | 1.00 | 36.23 | B | O |
| ATOM | 4570 | N | HIS | B | 299 | 20.889 | -13.261 | 52.624 | 1.00 | 46.16 | B | N |
| ATOM | 4571 | CA | HIS | B | 299 | 20.791 | -13.555 | 51.199 | 1.00 | 46.16 | B | C |
| ATOM | 4572 | CB | HIS | B | 299 | 19.316 | -13.570 | 50.799 | 1.00 | 56.19 | B | C |
| ATOM | 4573 | CG | HIS | B | 299 | 19.043 | -14.297 | 49.524 | 1.00 | 56.19 | B | C |
| ATOM | 4574 | CD2 | HIS | B | 299 | 18.844 | -15.613 | 49.275 | 1.00 | 56.19 | B | C |
| ATOM | 4575 | ND1 | HIS | B | 299 | 18.963 | -13.661 | 48.303 | 1.00 | 56.19 | B | N |
| ATOM | 4576 | CE1 | HIS | B | 299 | 18.726 | -14.552 | 47.357 | 1.00 | 56.19 | B | C |
| ATOM | 4577 | NE2 | HIS | B | 299 | 18.649 | -15.744 | 47.920 | 1.00 | 56.19 | B | N |
| ATOM | 4578 | C | HIS | B | 299 | 21.558 | -12.488 | 50.421 | 1.00 | 46.16 | B | C |
| ATOM | 4579 | O | HIS | B | 299 | 21.272 | -11.301 | 50.523 | 1.00 | 46.16 | B | O |
| ATOM | 4580 | N | PRO | B | 300 | 22.525 | -12.900 | 49.599 | 1.00 | 56.26 | B | N |
| ATOM | 4581 | CD | PRO | B | 300 | 22.777 | -14.265 | 49.090 | 1.00 | 46.90 | B | C |
| ATOM | 4582 | CA | PRO | B | 300 | 23.290 | -11.895 | 48.845 | 1.00 | 56.26 | B | C |
| ATOM | 4583 | CB | PRO | B | 300 | 24.251 | -12.749 | 48.012 | 1.00 | 46.90 | B | C |
| ATOM | 4584 | CG | PRO | B | 300 | 23.424 | -13.998 | 47.741 | 1.00 | 46.90 | B | C |
| ATOM | 4585 | C | PRO | B | 300 | 22.434 | -10.941 | 47.992 | 1.00 | 56.26 | B | C |
| ATOM | 4586 | O | PRO | B | 300 | 21.708 | -11.376 | 47.090 | 1.00 | 56.26 | B | O |
| ATOM | 4587 | N | TRP | B | 301 | 22.541 | -9.642 | 48.290 | 1.00 | 57.52 | B | N |
| ATOM | 4588 | CA | TRP | B | 301 | 21.795 | -8.601 | 47.582 | 1.00 | 57.52 | B | C |
| ATOM | 4589 | CB | TRP | B | 301 | 22.438 | -7.231 | 47.812 | 1.00 | 49.94 | B | C |
| ATOM | 4590 | CG | TRP | B | 301 | 21.896 | -6.515 | 49.012 | 1.00 | 49.94 | B | C |
| ATOM | 4591 | CD2 | TRP | B | 301 | 20.523 | -6.198 | 49.276 | 1.00 | 49.94 | B | C |
| ATOM | 4592 | CE2 | TRP | B | 301 | 20.468 | -5.609 | 50.562 | 1.00 | 49.94 | B | C |
| ATOM | 4593 | CE3 | TRP | B | 301 | 19.333 | -6.375 | 48.564 | 1.00 | 49.94 | B | C |
| ATOM | 4594 | CD1 | TRP | B | 301 | 22.602 | -6.092 | 50.107 | 1.00 | 49.94 | B | C |
| ATOM | 4595 | NE1 | TRP | B | 301 | 21.749 | -5.547 | 51.043 | 1.00 | 49.94 | B | N |
| ATOM | 4596 | CZ2 | TRP | B | 301 | 19.264 | -5.177 | 51.139 | 1.00 | 49.94 | B | C |
| ATOM | 4597 | CZ3 | TRP | B | 301 | 18.136 | -5.943 | 49.139 | 1.00 | 49.94 | B | C |
| ATOM | 4598 | CH2 | TRP | B | 301 | 18.112 | -5.362 | 50.416 | 1.00 | 49.94 | B | C |
| ATOM | 4599 | C | TRP | B | 301 | 21.648 | -8.834 | 46.083 | 1.00 | 57.52 | B | C |
| ATOM | 4600 | O | TRP | B | 301 | 20.562 | -8.645 | 45.517 | 1.00 | 57.52 | B | O |
| ATOM | 4601 | N | THR | B | 302 | 22.736 | -9.242 | 45.440 | 1.00 | 55.30 | B | N |
| ATOM | 4602 | CA | THR | B | 302 | 22.709 | -9.484 | 44.001 | 1.00 | 55.30 | B | C |
| ATOM | 4603 | CB | THR | B | 302 | 24.120 | -9.827 | 43.477 | 1.00 | 68.12 | B | C |
| ATOM | 4604 | OG1 | THR | B | 302 | 24.568 | -11.052 | 44.082 | 1.00 | 68.12 | B | O |
| ATOM | 4605 | CG2 | THR | B | 302 | 25.096 | -8.699 | 43.817 | 1.00 | 68.12 | B | C |
| ATOM | 4606 | C | THR | B | 302 | 21.737 | -10.599 | 43.587 | 1.00 | 55.30 | B | C |
| ATOM | 4607 | O | THR | B | 302 | 20.953 | -10.435 | 42.653 | 1.00 | 55.30 | B | O |
| ATOM | 4608 | N | ALA | B | 303 | 21.781 | -11.731 | 44.277 | 1.00 | 44.24 | B | N |
| ATOM | 4609 | CA | ALA | B | 303 | 20.887 | -12.833 | 43.935 | 1.00 | 44.24 | B | C |
| ATOM | 4610 | CB | ALA | B | 303 | 21.370 | -14.140 | 44.618 | 1.00 | 66.05 | B | C |
| ATOM | 4611 | C | ALA | B | 303 | 19.451 | -12.498 | 44.355 | 1.00 | 44.24 | B | C |
| ATOM | 4612 | O | ALA | B | 303 | 18.575 | -13.365 | 44.372 | 1.00 | 44.24 | B | O |
| ATOM | 4613 | N | VAL | B | 304 | 19.225 | -11.233 | 44.696 | 1.00 | 47.34 | B | N |
| ATOM | 4614 | CA | VAL | B | 304 | 17.906 | -10.767 | 45.120 | 1.00 | 47.34 | B | C |
| ATOM | 4615 | CB | VAL | B | 304 | 18.010 | -9.799 | 46.344 | 1.00 | 42.73 | B | C |
| ATOM | 4616 | CG1 | VAL | B | 304 | 16.626 | -9.278 | 46.737 | 1.00 | 42.73 | B | C |

| | | | | | | | | | | | | |
|---|---|---|---|---|---|---|---|---|---|---|---|---|
| ATOM | 4617 | CG2 | VAL | B | 304 | 18.664 | -10.514 | 47.508 | 1.00 | 42.73 | B | C |
| ATOM | 4618 | C | VAL | B | 304 | 17.196 | -10.043 | 43.979 | 1.00 | 47.34 | B | C |
| ATOM | 4619 | O | VAL | B | 304 | 15.977 | -10.105 | 43.866 | 1.00 | 47.34 | B | O |
| ATOM | 4620 | N | PHE | B | 305 | 17.965 | -9.363 | 43.136 | 1.00 | 35.28 | B | N |
| ATOM | 4621 | CA | PHE | B | 305 | 17.392 | -8.630 | 42.015 | 1.00 | 35.28 | B | C |
| ATOM | 4622 | CB | PHE | B | 305 | 18.068 | -7.263 | 41.905 | 1.00 | 44.78 | B | C |
| ATOM | 4623 | CG | PHE | B | 305 | 17.764 | -6.363 | 43.056 | 1.00 | 44.78 | B | C |
| ATOM | 4624 | CD1 | PHE | B | 305 | 16.614 | -5.580 | 43.057 | 1.00 | 44.78 | B | C |
| ATOM | 4625 | CD2 | PHE | B | 305 | 18.570 | -6.374 | 44.191 | 1.00 | 44.78 | B | C |
| ATOM | 4626 | CE1 | PHE | B | 305 | 16.266 | -4.826 | 44.178 | 1.00 | 44.78 | B | C |
| ATOM | 4627 | CE2 | PHE | B | 305 | 18.230 | -5.622 | 45.318 | 1.00 | 44.78 | B | C |
| ATOM | 4628 | CZ | PHE | B | 305 | 17.076 | -4.850 | 45.312 | 1.00 | 44.78 | B | C |
| ATOM | 4629 | C | PHE | B | 305 | 17.491 | -9.381 | 40.690 | 1.00 | 35.28 | B | C |
| ATOM | 4630 | O | PHE | B | 305 | 18.303 | -10.289 | 40.538 | 1.00 | 35.28 | B | O |
| ATOM | 4631 | N | ARG | B | 306 | 16.650 | -9.019 | 39.730 | 1.00 | 51.62 | B | N |
| ATOM | 4632 | CA | ARG | B | 306 | 16.724 | -9.698 | 38.448 | 1.00 | 51.62 | B | C |
| ATOM | 4633 | CB | ARG | B | 306 | 15.697 | -9.136 | 37.449 | 1.00 | 89.62 | B | C |
| ATOM | 4634 | CG | ARG | B | 306 | 15.625 | -7.604 | 37.346 | 1.00 | 89.62 | B | C |
| ATOM | 4635 | CD | ARG | B | 306 | 14.773 | -7.169 | 36.148 | 1.00 | 89.62 | B | C |
| ATOM | 4636 | NE | ARG | B | 306 | 15.604 | -6.948 | 34.965 | 1.00 | 89.62 | B | N |
| ATOM | 4637 | CZ | ARG | B | 306 | 16.308 | -5.837 | 34.739 | 1.00 | 89.62 | B | C |
| ATOM | 4638 | NH1 | ARG | B | 306 | 16.281 | -4.835 | 35.619 | 1.00 | 89.62 | B | N |
| ATOM | 4639 | NH2 | ARG | B | 306 | 17.034 | -5.721 | 33.630 | 1.00 | 89.62 | B | N |
| ATOM | 4640 | C | ARG | B | 306 | 18.141 | -9.551 | 37.894 | 1.00 | 51.62 | B | C |
| ATOM | 4641 | O | ARG | B | 306 | 18.860 | -8.602 | 38.222 | 1.00 | 51.62 | B | O |
| ATOM | 4642 | N | PRO | B | 307 | 18.563 | -10.507 | 37.057 | 1.00 | 57.13 | B | N |
| ATOM | 4643 | CD | PRO | B | 307 | 17.759 | -11.704 | 36.733 | 1.00 | 81.46 | B | C |
| ATOM | 4644 | CA | PRO | B | 307 | 19.876 | -10.578 | 36.409 | 1.00 | 57.13 | B | C |
| ATOM | 4645 | CB | PRO | B | 307 | 19.636 | -11.580 | 35.288 | 1.00 | 81.46 | B | C |
| ATOM | 4646 | CG | PRO | B | 307 | 18.752 | -12.586 | 35.972 | 1.00 | 81.46 | B | C |
| ATOM | 4647 | C | PRO | B | 307 | 20.499 | -9.281 | 35.906 | 1.00 | 57.13 | B | C |
| ATOM | 4648 | O | PRO | B | 307 | 21.584 | -8.901 | 36.343 | 1.00 | 57.13 | B | O |
| ATOM | 4649 | N | ALA | B | 308 | 19.826 | -8.602 | 34.984 | 1.00 | 66.58 | B | N |
| ATOM | 4650 | CA | ALA | B | 308 | 20.370 | -7.363 | 34.415 | 1.00 | 66.58 | B | C |
| ATOM | 4651 | CB | ALA | B | 308 | 19.839 | -7.176 | 32.984 | 1.00 | 70.42 | B | C |
| ATOM | 4652 | C | ALA | B | 308 | 20.127 | -6.090 | 35.239 | 1.00 | 66.58 | B | C |
| ATOM | 4653 | O | ALA | B | 308 | 20.043 | -4.992 | 34.687 | 1.00 | 66.58 | B | O |
| ATOM | 4654 | N | THR | B | 309 | 20.031 | -6.244 | 36.558 | 1.00 | 57.01 | B | N |
| ATOM | 4655 | CA | THR | B | 309 | 19.812 | -5.115 | 37.461 | 1.00 | 57.01 | B | C |
| ATOM | 4656 | CB | THR | B | 309 | 19.383 | -5.597 | 38.856 | 1.00 | 39.03 | B | C |
| ATOM | 4657 | OG1 | THR | B | 309 | 18.133 | -6.295 | 38.768 | 1.00 | 39.03 | B | O |
| ATOM | 4658 | CG2 | THR | B | 309 | 19.239 | -4.418 | 39.792 | 1.00 | 39.03 | B | C |
| ATOM | 4659 | C | THR | B | 309 | 21.096 | -4.299 | 37.606 | 1.00 | 57.01 | B | C |
| ATOM | 4660 | O | THR | B | 309 | 22.131 | -4.818 | 38.030 | 1.00 | 57.01 | B | O |
| ATOM | 4661 | N | PRO | B | 310 | 21.049 | -3.008 | 37.252 | 1.00 | 62.05 | B | N |
| ATOM | 4662 | CD | PRO | B | 310 | 19.865 | -2.228 | 36.852 | 1.00 | 35.50 | B | C |
| ATOM | 4663 | CA | PRO | B | 310 | 22.236 | -2.146 | 37.358 | 1.00 | 62.05 | B | C |
| ATOM | 4664 | CB | PRO | B | 310 | 21.658 | -0.745 | 37.180 | 1.00 | 35.50 | B | C |
| ATOM | 4665 | CG | PRO | B | 310 | 20.488 | -0.980 | 36.293 | 1.00 | 35.50 | B | C |
| ATOM | 4666 | C | PRO | B | 310 | 22.975 | -2.295 | 38.704 | 1.00 | 62.05 | B | C |
| ATOM | 4667 | O | PRO | B | 310 | 22.393 | -2.115 | 39.785 | 1.00 | 62.05 | B | O |
| ATOM | 4668 | N | PRO | B | 311 | 24.265 | -2.634 | 38.656 | 1.00 | 54.29 | B | N |
| ATOM | 4669 | CD | PRO | B | 311 | 25.011 | -3.040 | 37.456 | 1.00 | 41.27 | B | C |
| ATOM | 4670 | CA | PRO | B | 311 | 25.084 | -2.803 | 39.860 | 1.00 | 54.29 | B | C |
| ATOM | 4671 | CB | PRO | B | 311 | 26.485 | -2.913 | 39.291 | 1.00 | 41.27 | B | C |
| ATOM | 4672 | CG | PRO | B | 311 | 26.235 | -3.713 | 38.053 | 1.00 | 41.27 | B | C |
| ATOM | 4673 | C | PRO | B | 311 | 24.956 | -1.668 | 40.882 | 1.00 | 54.29 | B | C |
| ATOM | 4674 | O | PRO | B | 311 | 24.799 | -1.917 | 42.088 | 1.00 | 54.29 | B | O |
| ATOM | 4675 | N | GLU | B | 312 | 25.017 | -0.428 | 40.407 | 1.00 | 40.55 | B | N |
| ATOM | 4676 | CA | GLU | B | 312 | 24.907 | 0.715 | 41.298 | 1.00 | 40.55 | B | C |
| ATOM | 4677 | CB | GLU | B | 312 | 25.207 | 2.018 | 40.562 | 1.00 | 52.55 | B | C |
| ATOM | 4678 | CG | GLU | B | 312 | 26.558 | 2.044 | 39.904 | 1.00 | 52.55 | B | C |
| ATOM | 4679 | CD | GLU | B | 312 | 26.535 | 1.434 | 38.518 | 1.00 | 52.55 | B | C |
| ATOM | 4680 | OE1 | GLU | B | 312 | 25.914 | 0.361 | 38.328 | 1.00 | 52.55 | B | O |
| ATOM | 4681 | OE2 | GLU | B | 312 | 27.150 | 2.038 | 37.616 | 1.00 | 52.55 | B | O |
| ATOM | 4682 | C | GLU | B | 312 | 23.527 | 0.808 | 41.928 | 1.00 | 40.55 | B | C |
| ATOM | 4683 | O | GLU | B | 312 | 23.382 | 1.322 | 43.029 | 1.00 | 40.55 | B | O |

| | | | | | | | | | | | | |
|---|---|---|---|---|---|---|---|---|---|---|---|---|
| ATOM | 4684 | N | ALA | B | 313 | 22.506 | 0.333 | 41.232 | 1.00 | 24.24 | B | N |
| ATOM | 4685 | CA | ALA | B | 313 | 21.173 | 0.378 | 41.800 | 1.00 | 24.24 | B | C |
| ATOM | 4686 | CB | ALA | B | 313 | 20.162 | -0.206 | 40.815 | 1.00 | 42.45 | B | C |
| ATOM | 4687 | C | ALA | B | 313 | 21.249 | -0.473 | 43.063 | 1.00 | 24.24 | B | C |
| ATOM | 4688 | O | ALA | B | 313 | 20.747 | -0.106 | 44.119 | 1.00 | 24.24 | B | O |
| ATOM | 4689 | N | ILE | B | 314 | 21.915 | -1.613 | 42.937 | 1.00 | 39.01 | B | N |
| ATOM | 4690 | CA | ILE | B | 314 | 22.076 | -2.541 | 44.044 | 1.00 | 39.01 | B | C |
| ATOM | 4691 | CB | ILE | B | 314 | 22.703 | -3.858 | 43.555 | 1.00 | 34.60 | B | C |
| ATOM | 4692 | CG2 | ILE | B | 314 | 23.005 | -4.772 | 44.740 | 1.00 | 34.60 | B | C |
| ATOM | 4693 | CG1 | ILE | B | 314 | 21.745 | -4.542 | 42.570 | 1.00 | 34.60 | B | C |
| ATOM | 4694 | CD1 | ILE | B | 314 | 22.321 | -5.754 | 41.875 | 1.00 | 34.60 | B | C |
| ATOM | 4695 | C | ILE | B | 314 | 22.938 | -1.937 | 45.148 | 1.00 | 39.01 | B | C |
| ATOM | 4696 | O | ILE | B | 314 | 22.586 | -1.985 | 46.333 | 1.00 | 39.01 | B | O |
| ATOM | 4697 | N | ALA | B | 315 | 24.069 | -1.362 | 44.766 | 1.00 | 39.84 | B | N |
| ATOM | 4698 | CA | ALA | B | 315 | 24.948 | -0.744 | 45.752 | 1.00 | 39.84 | B | C |
| ATOM | 4699 | CB | ALA | B | 315 | 26.081 | -0.010 | 45.058 | 1.00 | 32.99 | B | C |
| ATOM | 4700 | C | ALA | B | 315 | 24.154 | 0.231 | 46.604 | 1.00 | 39.84 | B | C |
| ATOM | 4701 | O | ALA | B | 315 | 24.147 | 0.139 | 47.832 | 1.00 | 39.84 | B | O |
| ATOM | 4702 | N | LEU | B | 316 | 23.490 | 1.171 | 45.932 | 1.00 | 49.95 | B | N |
| ATOM | 4703 | CA | LEU | B | 316 | 22.672 | 2.192 | 46.582 | 1.00 | 49.95 | B | C |
| ATOM | 4704 | CB | LEU | B | 316 | 21.991 | 3.069 | 45.531 | 1.00 | 38.96 | B | C |
| ATOM | 4705 | CG | LEU | B | 316 | 20.815 | 3.910 | 46.024 | 1.00 | 38.96 | B | C |
| ATOM | 4706 | CD1 | LEU | B | 316 | 21.292 | 4.966 | 47.008 | 1.00 | 38.96 | B | C |
| ATOM | 4707 | CD2 | LEU | B | 316 | 20.135 | 4.547 | 44.843 | 1.00 | 38.96 | B | C |
| ATOM | 4708 | C | LEU | B | 316 | 21.610 | 1.577 | 47.468 | 1.00 | 49.95 | B | C |
| ATOM | 4709 | O | LEU | B | 316 | 21.379 | 2.025 | 48.585 | 1.00 | 49.95 | B | O |
| ATOM | 4710 | N | CYS | B | 317 | 20.960 | 0.542 | 46.965 | 1.00 | 32.79 | B | N |
| ATOM | 4711 | CA | CYS | B | 317 | 19.916 | -0.096 | 47.728 | 1.00 | 32.79 | B | C |
| ATOM | 4712 | CB | CYS | B | 317 | 19.287 | -1.208 | 46.899 | 1.00 | 47.83 | B | C |
| ATOM | 4713 | SG | CYS | B | 317 | 17.750 | -1.814 | 47.602 | 1.00 | 47.83 | B | S |
| ATOM | 4714 | C | CYS | B | 317 | 20.421 | -0.648 | 49.054 | 1.00 | 32.79 | B | C |
| ATOM | 4715 | O | CYS | B | 317 | 19.772 | -0.498 | 50.090 | 1.00 | 32.79 | B | O |
| ATOM | 4716 | N | SER | B | 318 | 21.587 | -1.278 | 49.027 | 1.00 | 38.18 | B | N |
| ATOM | 4717 | CA | SER | B | 318 | 22.158 | -1.867 | 50.236 | 1.00 | 38.18 | B | C |
| ATOM | 4718 | CB | SER | B | 318 | 23.337 | -2.756 | 49.863 | 1.00 | 51.43 | B | C |
| ATOM | 4719 | OG | SER | B | 318 | 24.295 | -1.998 | 49.151 | 1.00 | 51.43 | B | O |
| ATOM | 4720 | C | SER | B | 318 | 22.601 | -0.857 | 51.294 | 1.00 | 38.18 | B | C |
| ATOM | 4721 | O | SER | B | 318 | 22.694 | -1.194 | 52.472 | 1.00 | 38.18 | B | O |
| ATOM | 4722 | N | ARG | B | 319 | 22.882 | 0.373 | 50.882 | 1.00 | 38.46 | B | N |
| ATOM | 4723 | CA | ARG | B | 319 | 23.308 | 1.407 | 51.818 | 1.00 | 38.46 | B | C |
| ATOM | 4724 | CB | ARG | B | 319 | 24.301 | 2.340 | 51.141 | 1.00 | 51.85 | B | C |
| ATOM | 4725 | CG | ARG | B | 319 | 25.596 | 1.657 | 50.746 | 1.00 | 51.85 | B | C |
| ATOM | 4726 | CD | ARG | B | 319 | 26.496 | 1.387 | 51.945 | 1.00 | 51.85 | B | C |
| ATOM | 4727 | NE | ARG | B | 319 | 27.046 | 2.630 | 52.480 | 1.00 | 51.85 | B | N |
| ATOM | 4728 | CZ | ARG | B | 319 | 26.655 | 3.181 | 53.626 | 1.00 | 51.85 | B | C |
| ATOM | 4729 | NH1 | ARG | B | 319 | 25.713 | 2.588 | 54.354 | 1.00 | 51.85 | B | N |
| ATOM | 4730 | NH2 | ARG | B | 319 | 27.196 | 4.324 | 54.044 | 1.00 | 51.85 | B | N |
| ATOM | 4731 | C | ARG | B | 319 | 22.116 | 2.204 | 52.326 | 1.00 | 38.46 | B | C |
| ATOM | 4732 | O | ARG | B | 319 | 22.265 | 3.214 | 53.019 | 1.00 | 38.46 | B | O |
| ATOM | 4733 | N | LEU | B | 320 | 20.927 | 1.742 | 51.965 | 1.00 | 39.77 | B | N |
| ATOM | 4734 | CA | LEU | B | 320 | 19.696 | 2.383 | 52.381 | 1.00 | 39.77 | B | C |
| ATOM | 4735 | CB | LEU | B | 320 | 18.802 | 2.637 | 51.173 | 1.00 | 47.78 | B | C |
| ATOM | 4736 | CG | LEU | B | 320 | 19.300 | 3.645 | 50.137 | 1.00 | 47.78 | B | C |
| ATOM | 4737 | CD1 | LEU | B | 320 | 18.354 | 3.652 | 48.941 | 1.00 | 47.78 | B | C |
| ATOM | 4738 | CD2 | LEU | B | 320 | 19.376 | 5.030 | 50.761 | 1.00 | 47.78 | B | C |
| ATOM | 4739 | C | LEU | B | 320 | 19.015 | 1.426 | 53.325 | 1.00 | 39.77 | B | C |
| ATOM | 4740 | O | LEU | B | 320 | 18.551 | 1.812 | 54.398 | 1.00 | 39.77 | B | O |
| ATOM | 4741 | N | LEU | B | 321 | 18.984 | 0.160 | 52.920 | 1.00 | 43.04 | B | N |
| ATOM | 4742 | CA | LEU | B | 321 | 18.356 | -0.884 | 53.715 | 1.00 | 43.04 | B | C |
| ATOM | 4743 | CB | LEU | B | 321 | 17.724 | -1.925 | 52.788 | 1.00 | 40.51 | B | C |
| ATOM | 4744 | CG | LEU | B | 321 | 16.614 | -1.360 | 51.896 | 1.00 | 40.51 | B | C |
| ATOM | 4745 | CD1 | LEU | B | 321 | 16.155 | -2.400 | 50.891 | 1.00 | 40.51 | B | C |
| ATOM | 4746 | CD2 | LEU | B | 321 | 15.466 | -0.894 | 52.769 | 1.00 | 40.51 | B | C |
| ATOM | 4747 | C | LEU | B | 321 | 19.342 | -1.544 | 54.674 | 1.00 | 43.04 | B | C |
| ATOM | 4748 | O | LEU | B | 321 | 19.772 | -2.680 | 54.472 | 1.00 | 43.04 | B | O |
| ATOM | 4749 | N | GLU | B | 322 | 19.702 | -0.811 | 55.722 | 1.00 | 42.45 | B | N |
| ATOM | 4750 | CA | GLU | B | 322 | 20.625 | -1.311 | 56.733 | 1.00 | 42.45 | B | C |

| | | | | | | | | | | | | |
|---|---|---|---|---|---|---|---|---|---|---|---|---|
| ATOM | 4751 | CB | GLU | B | 322 | 21.842 | -0.378 | 56.855 | 1.00 | 52.98 | B | C |
| ATOM | 4752 | CG | GLU | B | 322 | 22.687 | -0.286 | 55.574 | 1.00 | 52.98 | B | C |
| ATOM | 4753 | CD | GLU | B | 322 | 24.095 | -0.888 | 55.713 | 1.00 | 52.98 | B | C |
| ATOM | 4754 | OE1 | GLU | B | 322 | 24.241 | -2.000 | 56.275 | 1.00 | 52.98 | B | O |
| ATOM | 4755 | OE2 | GLU | B | 322 | 25.060 | -0.248 | 55.237 | 1.00 | 52.98 | B | O |
| ATOM | 4756 | C | GLU | B | 322 | 19.872 | -1.367 | 58.053 | 1.00 | 42.45 | B | C |
| ATOM | 4757 | O | GLU | B | 322 | 19.012 | -0.530 | 58.300 | 1.00 | 42.45 | B | O |
| ATOM | 4758 | N | TYR | B | 323 | 20.169 | -2.362 | 58.888 | 1.00 | 38.29 | B | N |
| ATOM | 4759 | CA | TYR | B | 323 | 19.506 | -2.467 | 60.187 | 1.00 | 38.29 | B | C |
| ATOM | 4760 | CB | TYR | B | 323 | 19.950 | -3.734 | 60.920 | 1.00 | 50.18 | B | C |
| ATOM | 4761 | CG | TYR | B | 323 | 19.260 | -5.003 | 60.479 | 1.00 | 50.18 | B | C |
| ATOM | 4762 | CD1 | TYR | B | 323 | 17.908 | -5.220 | 60.745 | 1.00 | 50.18 | B | C |
| ATOM | 4763 | CE1 | TYR | B | 323 | 17.287 | -6.401 | 60.365 | 1.00 | 50.18 | B | C |
| ATOM | 4764 | CD2 | TYR | B | 323 | 19.968 | -6.002 | 59.816 | 1.00 | 50.18 | B | C |
| ATOM | 4765 | CE2 | TYR | B | 323 | 19.352 | -7.185 | 59.430 | 1.00 | 50.18 | B | C |
| ATOM | 4766 | CZ | TYR | B | 323 | 18.018 | -7.378 | 59.707 | 1.00 | 50.18 | B | C |
| ATOM | 4767 | OH | TYR | B | 323 | 17.422 | -8.556 | 59.329 | 1.00 | 50.18 | B | O |
| ATOM | 4768 | C | TYR | B | 323 | 19.857 | -1.249 | 61.041 | 1.00 | 38.29 | B | C |
| ATOM | 4769 | O | TYR | B | 323 | 18.991 | -0.572 | 61.574 | 1.00 | 38.29 | B | O |
| ATOM | 4770 | N | THR | B | 324 | 21.148 | -0.986 | 61.163 | 1.00 | 34.41 | B | N |
| ATOM | 4771 | CA | THR | B | 324 | 21.634 | 0.140 | 61.935 | 1.00 | 34.41 | B | C |
| ATOM | 4772 | CB | THR | B | 324 | 23.172 | 0.099 | 62.040 | 1.00 | 32.99 | B | C |
| ATOM | 4773 | OG1 | THR | B | 324 | 23.555 | -1.107 | 62.707 | 1.00 | 32.99 | B | O |
| ATOM | 4774 | CG2 | THR | B | 324 | 23.709 | 1.300 | 62.817 | 1.00 | 32.99 | B | C |
| ATOM | 4775 | C | THR | B | 324 | 21.198 | 1.443 | 61.287 | 1.00 | 34.41 | B | C |
| ATOM | 4776 | O | THR | B | 324 | 21.706 | 1.828 | 60.236 | 1.00 | 34.41 | B | O |
| ATOM | 4777 | N | PRO | B | 325 | 20.238 | 2.134 | 61.912 | 1.00 | 48.73 | B | N |
| ATOM | 4778 | CD | PRO | B | 325 | 19.590 | 1.757 | 63.181 | 1.00 | 36.88 | B | C |
| ATOM | 4779 | CA | PRO | B | 325 | 19.709 | 3.407 | 61.421 | 1.00 | 48.73 | B | C |
| ATOM | 4780 | CB | PRO | B | 325 | 18.906 | 3.921 | 62.615 | 1.00 | 36.88 | B | C |
| ATOM | 4781 | CG | PRO | B | 325 | 18.386 | 2.660 | 63.222 | 1.00 | 36.88 | B | C |
| ATOM | 4782 | C | PRO | B | 325 | 20.841 | 4.347 | 61.053 | 1.00 | 48.73 | B | C |
| ATOM | 4783 | O | PRO | B | 325 | 20.858 | 4.959 | 59.982 | 1.00 | 48.73 | B | O |
| ATOM | 4784 | N | THR | B | 326 | 21.794 | 4.435 | 61.966 | 1.00 | 45.81 | B | N |
| ATOM | 4785 | CA | THR | B | 326 | 22.952 | 5.291 | 61.822 | 1.00 | 45.81 | B | C |
| ATOM | 4786 | CB | THR | B | 326 | 23.799 | 5.206 | 63.126 | 1.00 | 47.93 | B | C |
| ATOM | 4787 | OG1 | THR | B | 326 | 23.984 | 6.526 | 63.652 | 1.00 | 47.93 | B | O |
| ATOM | 4788 | CG2 | THR | B | 326 | 25.174 | 4.522 | 62.874 | 1.00 | 47.93 | B | C |
| ATOM | 4789 | C | THR | B | 326 | 23.822 | 4.979 | 60.599 | 1.00 | 45.81 | B | C |
| ATOM | 4790 | O | THR | B | 326 | 24.606 | 5.813 | 60.158 | 1.00 | 45.81 | B | O |
| ATOM | 4791 | N | ALA | B | 327 | 23.664 | 3.785 | 60.047 | 1.00 | 42.43 | B | N |
| ATOM | 4792 | CA | ALA | B | 327 | 24.464 | 3.347 | 58.909 | 1.00 | 42.43 | B | C |
| ATOM | 4793 | CB | ALA | B | 327 | 24.632 | 1.833 | 58.974 | 1.00 | 43.15 | B | C |
| ATOM | 4794 | C | ALA | B | 327 | 23.944 | 3.728 | 57.530 | 1.00 | 42.43 | B | C |
| ATOM | 4795 | O | ALA | B | 327 | 24.696 | 3.703 | 56.551 | 1.00 | 42.43 | B | O |
| ATOM | 4796 | N | ARG | B | 328 | 22.659 | 4.062 | 57.452 | 1.00 | 46.97 | B | N |
| ATOM | 4797 | CA | ARG | B | 328 | 22.025 | 4.403 | 56.190 | 1.00 | 46.97 | B | C |
| ATOM | 4798 | CB | ARG | B | 328 | 20.519 | 4.480 | 56.385 | 1.00 | 34.46 | B | C |
| ATOM | 4799 | CG | ARG | B | 328 | 19.922 | 3.162 | 56.796 | 1.00 | 34.46 | B | C |
| ATOM | 4800 | CD | ARG | B | 328 | 18.537 | 3.313 | 57.364 | 1.00 | 34.46 | B | C |
| ATOM | 4801 | NE | ARG | B | 328 | 18.150 | 2.083 | 58.036 | 1.00 | 34.46 | B | N |
| ATOM | 4802 | CZ | ARG | B | 328 | 17.241 | 2.005 | 58.999 | 1.00 | 34.46 | B | C |
| ATOM | 4803 | NH1 | ARG | B | 328 | 16.608 | 3.094 | 59.416 | 1.00 | 34.46 | B | N |
| ATOM | 4804 | NH2 | ARG | B | 328 | 16.983 | 0.832 | 59.558 | 1.00 | 34.46 | B | N |
| ATOM | 4805 | C | ARG | B | 328 | 22.528 | 5.708 | 55.636 | 1.00 | 46.97 | B | C |
| ATOM | 4806 | O | ARG | B | 328 | 22.934 | 6.591 | 56.392 | 1.00 | 46.97 | B | O |
| ATOM | 4807 | N | LEU | B | 329 | 22.513 | 5.825 | 54.314 | 1.00 | 41.63 | B | N |
| ATOM | 4808 | CA | LEU | B | 329 | 22.942 | 7.053 | 53.663 | 1.00 | 41.63 | B | C |
| ATOM | 4809 | CB | LEU | B | 329 | 22.934 | 6.875 | 52.141 | 1.00 | 35.76 | B | C |
| ATOM | 4810 | CG | LEU | B | 329 | 24.158 | 6.359 | 51.385 | 1.00 | 35.76 | B | C |
| ATOM | 4811 | CD1 | LEU | B | 329 | 24.780 | 5.221 | 52.124 | 1.00 | 35.76 | B | C |
| ATOM | 4812 | CD2 | LEU | B | 329 | 23.750 | 5.937 | 49.999 | 1.00 | 35.76 | B | C |
| ATOM | 4813 | C | LEU | B | 329 | 21.969 | 8.176 | 54.037 | 1.00 | 41.63 | B | C |
| ATOM | 4814 | O | LEU | B | 329 | 20.843 | 7.919 | 54.463 | 1.00 | 41.63 | B | O |
| ATOM | 4815 | N | THR | B | 330 | 22.415 | 9.418 | 53.894 | 1.00 | 43.37 | B | N |
| ATOM | 4816 | CA | THR | B | 330 | 21.566 | 10.561 | 54.182 | 1.00 | 43.37 | B | C |
| ATOM | 4817 | CB | THR | B | 330 | 22.389 | 11.781 | 54.670 | 1.00 | 40.88 | B | C |

| | | | | | | | | | | | | |
|---|---|---|---|---|---|---|---|---|---|---|---|---|
| ATOM | 4818 | OG1 | THR | B | 330 | 23.339 | 12.161 | 53.666 | 1.00 | 40.88 | B | O |
| ATOM | 4819 | CG2 | THR | B | 330 | 23.117 | 11.448 | 55.936 | 1.00 | 40.88 | B | C |
| ATOM | 4820 | C | THR | B | 330 | 20.908 | 10.899 | 52.850 | 1.00 | 43.37 | B | C |
| ATOM | 4821 | O | THR | B | 330 | 21.437 | 10.568 | 51.793 | 1.00 | 43.37 | B | O |
| ATOM | 4822 | N | PRO | B | 331 | 19.742 | 11.552 | 52.881 | 1.00 | 42.73 | B | N |
| ATOM | 4823 | CD | PRO | B | 331 | 18.902 | 11.957 | 54.021 | 1.00 | 45.76 | B | C |
| ATOM | 4824 | CA | PRO | B | 331 | 19.098 | 11.884 | 51.610 | 1.00 | 42.73 | B | C |
| ATOM | 4825 | CB | PRO | B | 331 | 17.923 | 12.754 | 52.047 | 1.00 | 45.76 | B | C |
| ATOM | 4826 | CG | PRO | B | 331 | 17.550 | 12.149 | 53.371 | 1.00 | 45.76 | B | C |
| ATOM | 4827 | C | PRO | B | 331 | 20.035 | 12.586 | 50.620 | 1.00 | 42.73 | B | C |
| ATOM | 4828 | O | PRO | B | 331 | 20.103 | 12.207 | 49.457 | 1.00 | 42.73 | B | O |
| ATOM | 4829 | N | LEU | B | 332 | 20.764 | 13.597 | 51.074 | 1.00 | 34.87 | B | N |
| ATOM | 4830 | CA | LEU | B | 332 | 21.662 | 14.300 | 50.172 | 1.00 | 34.87 | B | C |
| ATOM | 4831 | CB | LEU | B | 332 | 22.347 | 15.466 | 50.874 | 1.00 | 39.71 | B | C |
| ATOM | 4832 | CG | LEU | B | 332 | 21.966 | 16.856 | 50.373 | 1.00 | 39.71 | B | C |
| ATOM | 4833 | CD1 | LEU | B | 332 | 22.994 | 17.864 | 50.859 | 1.00 | 39.71 | B | C |
| ATOM | 4834 | CD2 | LEU | B | 332 | 21.919 | 16.864 | 48.861 | 1.00 | 39.71 | B | C |
| ATOM | 4835 | C | LEU | B | 332 | 22.713 | 13.344 | 49.652 | 1.00 | 34.87 | B | C |
| ATOM | 4836 | O | LEU | B | 332 | 23.150 | 13.444 | 48.509 | 1.00 | 34.87 | B | O |
| ATOM | 4837 | N | GLU | B | 333 | 23.123 | 12.415 | 50.505 | 1.00 | 39.21 | B | N |
| ATOM | 4838 | CA | GLU | B | 333 | 24.127 | 11.422 | 50.141 | 1.00 | 39.21 | B | C |
| ATOM | 4839 | CB | GLU | B | 333 | 24.475 | 10.572 | 51.360 | 1.00 | 55.63 | B | C |
| ATOM | 4840 | CG | GLU | B | 333 | 25.845 | 10.827 | 51.915 | 1.00 | 55.63 | B | C |
| ATOM | 4841 | CD | GLU | B | 333 | 25.912 | 10.594 | 53.412 | 1.00 | 55.63 | B | C |
| ATOM | 4842 | OE1 | GLU | B | 333 | 25.559 | 9.478 | 53.862 | 1.00 | 55.63 | B | O |
| ATOM | 4843 | OE2 | GLU | B | 333 | 26.324 | 11.536 | 54.135 | 1.00 | 55.63 | B | O |
| ATOM | 4844 | C | GLU | B | 333 | 23.620 | 10.524 | 49.022 | 1.00 | 39.21 | B | C |
| ATOM | 4845 | O | GLU | B | 333 | 24.355 | 10.206 | 48.099 | 1.00 | 39.21 | B | O |
| ATOM | 4846 | N | ALA | B | 334 | 22.360 | 10.114 | 49.125 | 1.00 | 40.85 | B | N |
| ATOM | 4847 | CA | ALA | B | 334 | 21.734 | 9.262 | 48.126 | 1.00 | 40.85 | B | C |
| ATOM | 4848 | CB | ALA | B | 334 | 20.346 | 8.862 | 48.592 | 1.00 | 15.37 | B | C |
| ATOM | 4849 | C | ALA | B | 334 | 21.666 | 9.932 | 46.744 | 1.00 | 40.85 | B | C |
| ATOM | 4850 | O | ALA | B | 334 | 21.889 | 9.278 | 45.720 | 1.00 | 40.85 | B | O |
| ATOM | 4851 | N | CYS | B | 335 | 21.363 | 11.229 | 46.709 | 1.00 | 51.32 | B | N |
| ATOM | 4852 | CA | CYS | B | 335 | 21.299 | 11.958 | 45.435 | 1.00 | 51.32 | B | C |
| ATOM | 4853 | CB | CYS | B | 335 | 20.879 | 13.422 | 45.635 | 1.00 | 36.41 | B | C |
| ATOM | 4854 | SG | CYS | B | 335 | 19.226 | 13.696 | 46.298 | 1.00 | 36.41 | B | S |
| ATOM | 4855 | C | CYS | B | 335 | 22.684 | 11.942 | 44.824 | 1.00 | 51.32 | B | C |
| ATOM | 4856 | O | CYS | B | 335 | 22.845 | 11.855 | 43.607 | 1.00 | 51.32 | B | O |
| ATOM | 4857 | N | ALA | B | 336 | 23.685 | 12.032 | 45.688 | 1.00 | 43.29 | B | N |
| ATOM | 4858 | CA | ALA | B | 336 | 25.069 | 12.029 | 45.258 | 1.00 | 43.29 | B | C |
| ATOM | 4859 | CB | ALA | B | 336 | 25.959 | 12.450 | 46.412 | 1.00 | 61.77 | B | C |
| ATOM | 4860 | C | ALA | B | 336 | 25.504 | 10.667 | 44.734 | 1.00 | 43.29 | B | C |
| ATOM | 4861 | O | ALA | B | 336 | 26.491 | 10.576 | 44.011 | 1.00 | 43.29 | B | O |
| ATOM | 4862 | N | HIS | B | 337 | 24.773 | 9.616 | 45.089 | 1.00 | 28.49 | B | N |
| ATOM | 4863 | CA | HIS | B | 337 | 25.136 | 8.280 | 44.645 | 1.00 | 28.49 | B | C |
| ATOM | 4864 | CB | HIS | B | 337 | 24.081 | 7.270 | 45.069 | 1.00 | 34.57 | B | C |
| ATOM | 4865 | CG | HIS | B | 337 | 24.518 | 5.847 | 44.912 | 1.00 | 34.57 | B | C |
| ATOM | 4866 | CD2 | HIS | B | 337 | 24.581 | 5.050 | 43.820 | 1.00 | 34.57 | B | C |
| ATOM | 4867 | ND1 | HIS | B | 337 | 24.947 | 5.080 | 45.973 | 1.00 | 34.57 | B | N |
| ATOM | 4868 | CE1 | HIS | B | 337 | 25.249 | 3.868 | 45.542 | 1.00 | 34.57 | B | C |
| ATOM | 4869 | NE2 | HIS | B | 337 | 25.034 | 3.824 | 44.239 | 1.00 | 34.57 | B | N |
| ATOM | 4870 | C | HIS | B | 337 | 25.306 | 8.226 | 43.134 | 1.00 | 28.49 | B | C |
| ATOM | 4871 | O | HIS | B | 337 | 24.605 | 8.915 | 42.404 | 1.00 | 28.49 | B | O |
| ATOM | 4872 | N | SER | B | 338 | 26.229 | 7.390 | 42.668 | 1.00 | 49.69 | B | N |
| ATOM | 4873 | CA | SER | B | 338 | 26.506 | 7.269 | 41.238 | 1.00 | 49.69 | B | C |
| ATOM | 4874 | CB | SER | B | 338 | 27.840 | 6.538 | 41.013 | 1.00 | 46.42 | B | C |
| ATOM | 4875 | OG | SER | B | 338 | 27.908 | 5.335 | 41.750 | 1.00 | 46.42 | B | O |
| ATOM | 4876 | C | SER | B | 338 | 25.401 | 6.606 | 40.427 | 1.00 | 49.69 | B | C |
| ATOM | 4877 | O | SER | B | 338 | 25.416 | 6.669 | 39.202 | 1.00 | 49.69 | B | O |
| ATOM | 4878 | N | PHE | B | 339 | 24.455 | 5.964 | 41.105 | 1.00 | 42.45 | B | N |
| ATOM | 4879 | CA | PHE | B | 339 | 23.331 | 5.324 | 40.425 | 1.00 | 42.45 | B | C |
| ATOM | 4880 | CB | PHE | B | 339 | 22.370 | 4.744 | 41.464 | 1.00 | 45.70 | B | C |
| ATOM | 4881 | CG | PHE | B | 339 | 21.042 | 4.293 | 40.912 | 1.00 | 45.70 | B | C |
| ATOM | 4882 | CD1 | PHE | B | 339 | 20.967 | 3.316 | 39.924 | 1.00 | 45.70 | B | C |
| ATOM | 4883 | CD2 | PHE | B | 339 | 19.852 | 4.799 | 41.437 | 1.00 | 45.70 | B | C |
| ATOM | 4884 | CE1 | PHE | B | 339 | 19.721 | 2.844 | 39.472 | 1.00 | 45.70 | B | C |

| | | | | | | | | | | | | |
|---|---|---|---|---|---|---|---|---|---|---|---|---|
| ATOM | 4885 | CE2 | PHE | B | 339 | 18.611 | 4.338 | 40.995 | 1.00 | 45.70 | B | C |
| ATOM | 4886 | CZ | PHE | B | 339 | 18.545 | 3.358 | 40.012 | 1.00 | 45.70 | B | C |
| ATOM | 4887 | C | PHE | B | 339 | 22.641 | 6.423 | 39.623 | 1.00 | 42.45 | B | C |
| ATOM | 4888 | O | PHE | B | 339 | 22.111 | 6.193 | 38.536 | 1.00 | 42.45 | B | O |
| ATOM | 4889 | N | PHE | B | 340 | 22.694 | 7.633 | 40.161 | 1.00 | 48.61 | B | N |
| ATOM | 4890 | CA | PHE | B | 340 | 22.065 | 8.781 | 39.531 | 1.00 | 48.61 | B | C |
| ATOM | 4891 | CB | PHE | B | 340 | 21.514 | 9.717 | 40.608 | 1.00 | 36.00 | B | C |
| ATOM | 4892 | CG | PHE | B | 340 | 20.552 | 9.059 | 41.550 | 1.00 | 36.00 | B | C |
| ATOM | 4893 | CD1 | PHE | B | 340 | 19.404 | 8.441 | 41.073 | 1.00 | 36.00 | B | C |
| ATOM | 4894 | CD2 | PHE | B | 340 | 20.772 | 9.090 | 42.922 | 1.00 | 36.00 | B | C |
| ATOM | 4895 | CE1 | PHE | B | 340 | 18.486 | 7.870 | 41.944 | 1.00 | 36.00 | B | C |
| ATOM | 4896 | CE2 | PHE | B | 340 | 19.856 | 8.519 | 43.800 | 1.00 | 36.00 | B | C |
| ATOM | 4897 | CZ | PHE | B | 340 | 18.708 | 7.909 | 43.304 | 1.00 | 36.00 | B | C |
| ATOM | 4898 | C | PHE | B | 340 | 22.964 | 9.590 | 38.594 | 1.00 | 48.61 | B | C |
| ATOM | 4899 | O | PHE | B | 340 | 22.505 | 10.576 | 38.017 | 1.00 | 48.61 | B | O |
| ATOM | 4900 | N | ASP | B | 341 | 24.229 | 9.196 | 38.436 | 1.00 | 67.58 | B | N |
| ATOM | 4901 | CA | ASP | B | 341 | 25.145 | 9.945 | 37.560 | 1.00 | 67.58 | B | C |
| ATOM | 4902 | CB | ASP | B | 341 | 26.455 | 9.183 | 37.323 | 1.00 | 49.28 | B | C |
| ATOM | 4903 | CG | ASP | B | 341 | 27.282 | 9.034 | 38.586 | 1.00 | 49.28 | B | C |
| ATOM | 4904 | OD1 | ASP | B | 341 | 27.065 | 9.816 | 39.538 | 1.00 | 49.28 | B | O |
| ATOM | 4905 | OD2 | ASP | B | 341 | 28.158 | 8.144 | 38.623 | 1.00 | 49.28 | B | O |
| ATOM | 4906 | C | ASP | B | 341 | 24.527 | 10.290 | 36.210 | 1.00 | 67.58 | B | C |
| ATOM | 4907 | O | ASP | B | 341 | 24.651 | 11.419 | 35.735 | 1.00 | 67.58 | B | O |
| ATOM | 4908 | N | GLU | B | 342 | 23.855 | 9.327 | 35.593 | 1.00 | 47.77 | B | N |
| ATOM | 4909 | CA | GLU | B | 342 | 23.237 | 9.582 | 34.305 | 1.00 | 47.77 | B | C |
| ATOM | 4910 | CB | GLU | B | 342 | 22.356 | 8.397 | 33.902 | 1.00 | 61.12 | B | C |
| ATOM | 4911 | CG | GLU | B | 342 | 21.474 | 8.666 | 32.687 | 1.00 | 61.12 | B | C |
| ATOM | 4912 | CD | GLU | B | 342 | 20.818 | 7.409 | 32.140 | 1.00 | 61.12 | B | C |
| ATOM | 4913 | OE1 | GLU | B | 342 | 20.282 | 6.587 | 32.926 | 1.00 | 61.12 | B | O |
| ATOM | 4914 | OE2 | GLU | B | 342 | 20.832 | 7.255 | 30.905 | 1.00 | 61.12 | B | O |
| ATOM | 4915 | C | GLU | B | 342 | 22.416 | 10.875 | 34.301 | 1.00 | 47.77 | B | C |
| ATOM | 4916 | O | GLU | B | 342 | 22.329 | 11.556 | 33.282 | 1.00 | 47.77 | B | O |
| ATOM | 4917 | N | LEU | B | 343 | 21.821 | 11.220 | 35.439 | 1.00 | 47.54 | B | N |
| ATOM | 4918 | CA | LEU | B | 343 | 21.011 | 12.433 | 35.534 | 1.00 | 47.54 | B | C |
| ATOM | 4919 | CB | LEU | B | 343 | 20.207 | 12.426 | 36.835 | 1.00 | 37.91 | B | C |
| ATOM | 4920 | CG | LEU | B | 343 | 19.274 | 11.233 | 37.041 | 1.00 | 37.91 | B | C |
| ATOM | 4921 | CD1 | LEU | B | 343 | 18.581 | 11.366 | 38.378 | 1.00 | 37.91 | B | C |
| ATOM | 4922 | CD2 | LEU | B | 343 | 18.253 | 11.162 | 35.912 | 1.00 | 37.91 | B | C |
| ATOM | 4923 | C | LEU | B | 343 | 21.891 | 13.676 | 35.488 | 1.00 | 47.54 | B | C |
| ATOM | 4924 | O | LEU | B | 343 | 21.425 | 14.778 | 35.181 | 1.00 | 47.54 | B | O |
| ATOM | 4925 | N | ARG | B | 344 | 23.172 | 13.487 | 35.790 | 1.00 | 46.24 | B | N |
| ATOM | 4926 | CA | ARG | B | 344 | 24.123 | 14.586 | 35.809 | 1.00 | 46.24 | B | C |
| ATOM | 4927 | CB | ARG | B | 344 | 25.192 | 14.326 | 36.874 | 1.00 | 51.22 | B | C |
| ATOM | 4928 | CG | ARG | B | 344 | 24.795 | 14.819 | 38.275 | 1.00 | 51.22 | B | C |
| ATOM | 4929 | CD | ARG | B | 344 | 25.728 | 14.270 | 39.331 | 1.00 | 51.22 | B | C |
| ATOM | 4930 | NE | ARG | B | 344 | 25.497 | 12.845 | 39.535 | 1.00 | 51.22 | B | N |
| ATOM | 4931 | CZ | ARG | B | 344 | 24.762 | 12.337 | 40.523 | 1.00 | 51.22 | B | C |
| ATOM | 4932 | NH1 | ARG | B | 344 | 24.183 | 13.135 | 41.416 | 1.00 | 51.22 | B | N |
| ATOM | 4933 | NH2 | ARG | B | 344 | 24.594 | 11.025 | 40.612 | 1.00 | 51.22 | B | N |
| ATOM | 4934 | C | ARG | B | 344 | 24.741 | 14.818 | 34.444 | 1.00 | 46.24 | B | C |
| ATOM | 4935 | O | ARG | B | 344 | 25.411 | 15.826 | 34.213 | 1.00 | 46.24 | B | O |
| ATOM | 4936 | N | ASP | B | 345 | 24.498 | 13.875 | 33.541 | 1.00 | 49.45 | B | N |
| ATOM | 4937 | CA | ASP | B | 345 | 24.970 | 13.954 | 32.156 | 1.00 | 49.45 | B | C |
| ATOM | 4938 | CB | ASP | B | 345 | 24.508 | 12.688 | 31.425 | 1.00 | 50.74 | B | C |
| ATOM | 4939 | CG | ASP | B | 345 | 24.897 | 12.657 | 29.959 | 1.00 | 50.74 | B | C |
| ATOM | 4940 | OD1 | ASP | B | 345 | 24.925 | 13.723 | 29.304 | 1.00 | 50.74 | B | O |
| ATOM | 4941 | OD2 | ASP | B | 345 | 25.149 | 11.538 | 29.452 | 1.00 | 50.74 | B | O |
| ATOM | 4942 | C | ASP | B | 345 | 24.305 | 15.198 | 31.538 | 1.00 | 49.45 | B | C |
| ATOM | 4943 | O | ASP | B | 345 | 23.110 | 15.425 | 31.730 | 1.00 | 49.45 | B | O |
| ATOM | 4944 | N | PRO | B | 346 | 25.063 | 16.024 | 30.798 | 1.00 | 46.36 | B | N |
| ATOM | 4945 | CD | PRO | B | 346 | 26.477 | 15.920 | 30.407 | 1.00 | 26.69 | B | C |
| ATOM | 4946 | CA | PRO | B | 346 | 24.458 | 17.217 | 30.195 | 1.00 | 46.36 | B | C |
| ATOM | 4947 | CB | PRO | B | 346 | 25.671 | 17.981 | 29.690 | 1.00 | 26.69 | B | C |
| ATOM | 4948 | CG | PRO | B | 346 | 26.553 | 16.878 | 29.235 | 1.00 | 26.69 | B | C |
| ATOM | 4949 | C | PRO | B | 346 | 23.481 | 16.860 | 29.067 | 1.00 | 46.36 | B | C |
| ATOM | 4950 | O | PRO | B | 346 | 22.623 | 17.666 | 28.685 | 1.00 | 46.36 | B | O |
| ATOM | 4951 | N | ASN | B | 347 | 23.609 | 15.636 | 28.560 | 1.00 | 48.42 | B | N |

| | | | | | | | | | | | | |
|---|---|---|---|---|---|---|---|---|---|---|---|---|
| ATOM | 4952 | CA | ASN | B | 347 | 22.776 | 15.129 | 27.475 | 1.00 | 48.42 | B | C |
| ATOM | 4953 | CB | ASN | B | 347 | 23.595 | 14.169 | 26.628 | 1.00 | 80.67 | B | C |
| ATOM | 4954 | CG | ASN | B | 347 | 24.823 | 14.824 | 26.044 | 1.00 | 80.67 | B | C |
| ATOM | 4955 | OD1 | ASN | B | 347 | 25.849 | 14.162 | 25.824 | 1.00 | 80.67 | B | O |
| ATOM | 4956 | ND2 | ASN | B | 347 | 24.731 | 16.132 | 25.777 | 1.00 | 80.67 | B | N |
| ATOM | 4957 | C | ASN | B | 347 | 21.500 | 14.415 | 27.909 | 1.00 | 48.42 | B | C |
| ATOM | 4958 | O | ASN | B | 347 | 20.527 | 14.383 | 27.166 | 1.00 | 48.42 | B | O |
| ATOM | 4959 | N | VAL | B | 348 | 21.507 | 13.826 | 29.099 | 1.00 | 59.70 | B | N |
| ATOM | 4960 | CA | VAL | B | 348 | 20.339 | 13.093 | 29.572 | 1.00 | 59.70 | B | C |
| ATOM | 4961 | CB | VAL | B | 348 | 20.376 | 12.862 | 31.103 | 1.00 | 47.92 | B | C |
| ATOM | 4962 | CG1 | VAL | B | 348 | 20.423 | 14.188 | 31.844 | 1.00 | 47.92 | B | C |
| ATOM | 4963 | CG2 | VAL | B | 348 | 19.155 | 12.071 | 31.526 | 1.00 | 47.92 | B | C |
| ATOM | 4964 | C | VAL | B | 348 | 19.037 | 13.796 | 29.226 | 1.00 | 59.70 | B | C |
| ATOM | 4965 | O | VAL | B | 348 | 18.896 | 15.007 | 29.424 | 1.00 | 59.70 | B | O |
| ATOM | 4966 | N | LYS | B | 349 | 18.091 | 13.037 | 28.691 | 1.00 | 43.91 | B | N |
| ATOM | 4967 | CA | LYS | B | 349 | 16.795 | 13.596 | 28.346 | 1.00 | 43.91 | B | C |
| ATOM | 4968 | CB | LYS | B | 349 | 16.734 | 13.940 | 26.852 | 1.00 | 63.89 | B | C |
| ATOM | 4969 | CG | LYS | B | 349 | 17.968 | 14.686 | 26.357 | 1.00 | 63.89 | B | C |
| ATOM | 4970 | CD | LYS | B | 349 | 17.720 | 15.430 | 25.059 | 1.00 | 63.89 | B | C |
| ATOM | 4971 | CE | LYS | B | 349 | 16.909 | 16.706 | 25.301 | 1.00 | 63.89 | B | C |
| ATOM | 4972 | NZ | LYS | B | 349 | 17.651 | 17.701 | 26.144 | 1.00 | 63.89 | B | N |
| ATOM | 4973 | C | LYS | B | 349 | 15.764 | 12.546 | 28.689 | 1.00 | 43.91 | B | C |
| ATOM | 4974 | O | LYS | B | 349 | 16.111 | 11.382 | 28.879 | 1.00 | 43.91 | B | O |
| ATOM | 4975 | N | LEU | B | 350 | 14.503 | 12.940 | 28.795 | 1.00 | 44.58 | B | N |
| ATOM | 4976 | CA | LEU | B | 350 | 13.486 | 11.952 | 29.108 | 1.00 | 44.58 | B | C |
| ATOM | 4977 | CB | LEU | B | 350 | 12.196 | 12.634 | 29.554 | 1.00 | 52.90 | B | C |
| ATOM | 4978 | CG | LEU | B | 350 | 12.278 | 13.628 | 30.714 | 1.00 | 52.90 | B | C |
| ATOM | 4979 | CD1 | LEU | B | 350 | 10.914 | 14.260 | 30.933 | 1.00 | 52.90 | B | C |
| ATOM | 4980 | CD2 | LEU | B | 350 | 12.744 | 12.926 | 31.970 | 1.00 | 52.90 | B | C |
| ATOM | 4981 | C | LEU | B | 350 | 13.227 | 11.131 | 27.850 | 1.00 | 44.58 | B | C |
| ATOM | 4982 | O | LEU | B | 350 | 13.562 | 11.558 | 26.736 | 1.00 | 44.58 | B | O |
| ATOM | 4983 | N | PRO | B | 351 | 12.640 | 9.935 | 28.005 | 1.00 | 61.99 | B | N |
| ATOM | 4984 | CD | PRO | B | 351 | 12.159 | 9.272 | 29.228 | 1.00 | 53.32 | B | C |
| ATOM | 4985 | CA | PRO | B | 351 | 12.367 | 9.116 | 26.821 | 1.00 | 61.99 | B | C |
| ATOM | 4986 | CB | PRO | B | 351 | 11.933 | 7.778 | 27.421 | 1.00 | 53.32 | B | C |
| ATOM | 4987 | CG | PRO | B | 351 | 11.255 | 8.192 | 28.673 | 1.00 | 53.32 | B | C |
| ATOM | 4988 | C | PRO | B | 351 | 11.251 | 9.812 | 26.054 | 1.00 | 61.99 | B | C |
| ATOM | 4989 | O | PRO | B | 351 | 10.688 | 9.291 | 25.087 | 1.00 | 61.99 | B | O |
| ATOM | 4990 | N | ASN | B | 352 | 10.963 | 11.017 | 26.521 | 1.00 | 53.15 | B | N |
| ATOM | 4991 | CA | ASN | B | 352 | 9.930 | 11.881 | 25.985 | 1.00 | 53.15 | B | C |
| ATOM | 4992 | CB | ASN | B | 352 | 9.335 | 12.696 | 27.127 | 1.00 | 71.14 | B | C |
| ATOM | 4993 | CG | ASN | B | 352 | 7.891 | 12.992 | 26.913 | 1.00 | 71.14 | B | C |
| ATOM | 4994 | OD1 | ASN | B | 352 | 7.201 | 13.502 | 27.804 | 1.00 | 71.14 | B | O |
| ATOM | 4995 | ND2 | ASN | B | 352 | 7.404 | 12.667 | 25.715 | 1.00 | 71.14 | B | N |
| ATOM | 4996 | C | ASN | B | 352 | 10.499 | 12.840 | 24.966 | 1.00 | 53.15 | B | C |
| ATOM | 4997 | O | ASN | B | 352 | 9.749 | 13.531 | 24.299 | 1.00 | 53.15 | B | O |
| ATOM | 4998 | N | GLY | B | 353 | 11.826 | 12.888 | 24.871 | 1.00 | 42.84 | B | N |
| ATOM | 4999 | CA | GLY | B | 353 | 12.490 | 13.802 | 23.956 | 1.00 | 42.84 | B | C |
| ATOM | 5000 | C | GLY | B | 353 | 12.721 | 15.108 | 24.697 | 1.00 | 42.84 | B | C |
| ATOM | 5001 | O | GLY | B | 353 | 13.654 | 15.880 | 24.416 | 1.00 | 42.84 | B | O |
| ATOM | 5002 | N | ARG | B | 354 | 11.851 | 15.331 | 25.681 | 1.00 | 71.99 | B | N |
| ATOM | 5003 | CA | ARG | B | 354 | 11.879 | 16.519 | 26.527 | 1.00 | 71.99 | B | C |
| ATOM | 5004 | CB | ARG | B | 354 | 10.543 | 16.654 | 27.254 | 1.00 | 76.87 | B | C |
| ATOM | 5005 | CG | ARG | B | 354 | 9.351 | 16.550 | 26.334 | 1.00 | 76.87 | B | C |
| ATOM | 5006 | CD | ARG | B | 354 | 8.045 | 16.534 | 27.104 | 1.00 | 76.87 | B | C |
| ATOM | 5007 | NE | ARG | B | 354 | 7.952 | 17.668 | 28.015 | 1.00 | 76.87 | B | N |
| ATOM | 5008 | CZ | ARG | B | 354 | 6.805 | 18.166 | 28.477 | 1.00 | 76.87 | B | C |
| ATOM | 5009 | NH1 | ARG | B | 354 | 5.645 | 17.618 | 28.100 | 1.00 | 76.87 | B | N |
| ATOM | 5010 | NH2 | ARG | B | 354 | 6.819 | 19.216 | 29.305 | 1.00 | 76.87 | B | N |
| ATOM | 5011 | C | ARG | B | 354 | 13.001 | 16.428 | 27.554 | 1.00 | 71.99 | B | C |
| ATOM | 5012 | O | ARG | B | 354 | 13.456 | 15.329 | 27.910 | 1.00 | 71.99 | B | O |
| ATOM | 5013 | N | ASP | B | 355 | 13.443 | 17.581 | 28.040 | 1.00 | 47.06 | B | N |
| ATOM | 5014 | CA | ASP | B | 355 | 14.495 | 17.595 | 29.036 | 1.00 | 47.06 | B | C |
| ATOM | 5015 | CB | ASP | B | 355 | 15.095 | 18.987 | 29.156 | 1.00 | 86.90 | B | C |
| ATOM | 5016 | CG | ASP | B | 355 | 15.287 | 19.640 | 27.806 | 1.00 | 86.90 | B | C |
| ATOM | 5017 | OD1 | ASP | B | 355 | 14.247 | 20.001 | 27.190 | 1.00 | 86.90 | B | O |
| ATOM | 5018 | OD2 | ASP | B | 355 | 16.462 | 19.774 | 27.357 | 1.00 | 86.90 | B | O |

| | | | | | | | | | | | | |
|---|---|---|---|---|---|---|---|---|---|---|---|---|
| ATOM | 5019 | C | ASP | B | 355 | 13.941 | 17.170 | 30.382 | 1.00 | 47.06 | B | C |
| ATOM | 5020 | O | ASP | B | 355 | 12.730 | 17.151 | 30.608 | 1.00 | 47.06 | B | O |
| ATOM | 5021 | N | THR | B | 356 | 14.855 | 16.810 | 31.269 | 1.00 | 51.49 | B | N |
| ATOM | 5022 | CA | THR | B | 356 | 14.497 | 16.389 | 32.607 | 1.00 | 51.49 | B | C |
| ATOM | 5023 | CB | THR | B | 356 | 15.659 | 15.640 | 33.269 | 1.00 | 41.96 | B | C |
| ATOM | 5024 | OG1 | THR | B | 356 | 16.849 | 16.428 | 33.153 | 1.00 | 41.96 | B | O |
| ATOM | 5025 | CG2 | THR | B | 356 | 15.877 | 14.288 | 32.604 | 1.00 | 41.96 | B | C |
| ATOM | 5026 | C | THR | B | 356 | 14.214 | 17.634 | 33.424 | 1.00 | 51.49 | B | C |
| ATOM | 5027 | O | THR | B | 356 | 14.742 | 18.709 | 33.135 | 1.00 | 51.49 | B | O |
| ATOM | 5028 | N | PRO | B | 357 | 13.371 | 17.508 | 34.455 | 1.00 | 36.38 | B | N |
| ATOM | 5029 | CD | PRO | B | 357 | 12.692 | 16.305 | 34.968 | 1.00 | 39.95 | B | C |
| ATOM | 5030 | CA | PRO | B | 357 | 13.073 | 18.676 | 35.280 | 1.00 | 36.38 | B | C |
| ATOM | 5031 | CB | PRO | B | 357 | 12.118 | 18.115 | 36.341 | 1.00 | 39.95 | B | C |
| ATOM | 5032 | CG | PRO | B | 357 | 12.481 | 16.656 | 36.414 | 1.00 | 39.95 | B | C |
| ATOM | 5033 | C | PRO | B | 357 | 14.368 | 19.230 | 35.873 | 1.00 | 36.38 | B | C |
| ATOM | 5034 | O | PRO | B | 357 | 15.440 | 18.672 | 35.657 | 1.00 | 36.38 | B | O |
| ATOM | 5035 | N | ALA | B | 358 | 14.270 | 20.338 | 36.599 | 1.00 | 45.42 | B | N |
| ATOM | 5036 | CA | ALA | B | 358 | 15.444 | 20.942 | 37.219 | 1.00 | 45.42 | B | C |
| ATOM | 5037 | CB | ALA | B | 358 | 15.101 | 22.328 | 37.740 | 1.00 | 50.02 | B | C |
| ATOM | 5038 | C | ALA | B | 358 | 15.883 | 20.047 | 38.371 | 1.00 | 45.42 | B | C |
| ATOM | 5039 | O | ALA | B | 358 | 15.193 | 19.962 | 39.380 | 1.00 | 45.42 | B | O |
| ATOM | 5040 | N | LEU | B | 359 | 17.031 | 19.390 | 38.235 | 1.00 | 54.53 | B | N |
| ATOM | 5041 | CA | LEU | B | 359 | 17.493 | 18.491 | 39.289 | 1.00 | 54.53 | B | C |
| ATOM | 5042 | CB | LEU | B | 359 | 17.814 | 17.119 | 38.685 | 1.00 | 47.13 | B | C |
| ATOM | 5043 | CG | LEU | B | 359 | 16.666 | 16.603 | 37.817 | 1.00 | 47.13 | B | C |
| ATOM | 5044 | CD1 | LEU | B | 359 | 17.107 | 15.377 | 37.024 | 1.00 | 47.13 | B | C |
| ATOM | 5045 | CD2 | LEU | B | 359 | 15.461 | 16.315 | 38.708 | 1.00 | 47.13 | B | C |
| ATOM | 5046 | C | LEU | B | 359 | 18.705 | 19.004 | 40.059 | 1.00 | 54.53 | B | C |
| ATOM | 5047 | O | LEU | B | 359 | 19.245 | 18.293 | 40.916 | 1.00 | 54.53 | B | O |
| ATOM | 5048 | N | PHE | B | 360 | 19.111 | 20.242 | 39.786 | 1.00 | 58.89 | B | N |
| ATOM | 5049 | CA | PHE | B | 360 | 20.287 | 20.798 | 40.434 | 1.00 | 58.89 | B | C |
| ATOM | 5050 | CB | PHE | B | 360 | 21.410 | 20.912 | 39.415 | 1.00 | 49.50 | B | C |
| ATOM | 5051 | CG | PHE | B | 360 | 21.544 | 19.711 | 38.529 | 1.00 | 49.50 | B | C |
| ATOM | 5052 | CD1 | PHE | B | 360 | 21.860 | 18.469 | 39.065 | 1.00 | 49.50 | B | C |
| ATOM | 5053 | CD2 | PHE | B | 360 | 21.346 | 19.819 | 37.155 | 1.00 | 49.50 | B | C |
| ATOM | 5054 | CE1 | PHE | B | 360 | 21.978 | 17.341 | 38.247 | 1.00 | 49.50 | B | C |
| ATOM | 5055 | CE2 | PHE | B | 360 | 21.461 | 18.699 | 36.327 | 1.00 | 49.50 | B | C |
| ATOM | 5056 | CZ | PHE | B | 360 | 21.778 | 17.451 | 36.878 | 1.00 | 49.50 | B | C |
| ATOM | 5057 | C | PHE | B | 360 | 20.121 | 22.152 | 41.115 | 1.00 | 58.89 | B | C |
| ATOM | 5058 | O | PHE | B | 360 | 21.058 | 22.629 | 41.763 | 1.00 | 58.89 | B | O |
| ATOM | 5059 | N | ASN | B | 361 | 18.966 | 22.796 | 40.969 | 1.00 | 64.68 | B | N |
| ATOM | 5060 | CA | ASN | B | 361 | 18.785 | 24.096 | 41.617 | 1.00 | 64.68 | B | C |
| ATOM | 5061 | CB | ASN | B | 361 | 17.588 | 24.860 | 41.014 | 1.00 | 60.33 | B | C |
| ATOM | 5062 | CG | ASN | B | 361 | 16.299 | 24.053 | 41.009 | 1.00 | 60.33 | B | C |
| ATOM | 5063 | OD1 | ASN | B | 361 | 16.281 | 22.893 | 40.598 | 1.00 | 60.33 | B | O |
| ATOM | 5064 | ND2 | ASN | B | 361 | 15.208 | 24.674 | 41.446 | 1.00 | 60.33 | B | N |
| ATOM | 5065 | C | ASN | B | 361 | 18.624 | 23.897 | 43.120 | 1.00 | 64.68 | B | C |
| ATOM | 5066 | O | ASN | B | 361 | 17.543 | 24.086 | 43.681 | 1.00 | 64.68 | B | O |
| ATOM | 5067 | N | PHE | B | 362 | 19.725 | 23.499 | 43.753 | 1.00 | 52.01 | B | N |
| ATOM | 5068 | CA | PHE | B | 362 | 19.782 | 23.252 | 45.189 | 1.00 | 52.01 | B | C |
| ATOM | 5069 | CB | PHE | B | 362 | 20.986 | 22.377 | 45.524 | 1.00 | 42.20 | B | C |
| ATOM | 5070 | CG | PHE | B | 362 | 20.792 | 20.926 | 45.211 | 1.00 | 42.20 | B | C |
| ATOM | 5071 | CD1 | PHE | B | 362 | 20.268 | 20.061 | 46.172 | 1.00 | 42.20 | B | C |
| ATOM | 5072 | CD2 | PHE | B | 362 | 21.144 | 20.420 | 43.966 | 1.00 | 42.20 | B | C |
| ATOM | 5073 | CE1 | PHE | B | 362 | 20.103 | 18.709 | 45.894 | 1.00 | 42.20 | B | C |
| ATOM | 5074 | CE2 | PHE | B | 362 | 20.984 | 19.079 | 43.676 | 1.00 | 42.20 | B | C |
| ATOM | 5075 | CZ | PHE | B | 362 | 20.462 | 18.216 | 44.644 | 1.00 | 42.20 | B | C |
| ATOM | 5076 | C | PHE | B | 362 | 19.937 | 24.545 | 45.951 | 1.00 | 52.01 | B | C |
| ATOM | 5077 | O | PHE | B | 362 | 20.603 | 25.461 | 45.483 | 1.00 | 52.01 | B | O |
| ATOM | 5078 | N | THR | B | 363 | 19.336 | 24.616 | 47.131 | 1.00 | 48.45 | B | N |
| ATOM | 5079 | CA | THR | B | 363 | 19.458 | 25.802 | 47.967 | 1.00 | 48.45 | B | C |
| ATOM | 5080 | CB | THR | B | 363 | 18.093 | 26.278 | 48.468 | 1.00 | 49.95 | B | C |
| ATOM | 5081 | OG1 | THR | B | 363 | 17.549 | 25.305 | 49.373 | 1.00 | 49.95 | B | O |
| ATOM | 5082 | CG2 | THR | B | 363 | 17.149 | 26.498 | 47.283 | 1.00 | 49.95 | B | C |
| ATOM | 5083 | C | THR | B | 363 | 20.327 | 25.453 | 49.175 | 1.00 | 48.45 | B | C |
| ATOM | 5084 | O | THR | B | 363 | 20.348 | 24.296 | 49.619 | 1.00 | 48.45 | B | O |
| ATOM | 5085 | N | THR | B | 364 | 21.042 | 26.446 | 49.704 | 1.00 | 68.37 | B | N |

| | | | | | | | | | | | | |
|---|---|---|---|---|---|---|---|---|---|---|---|---|
| ATOM | 5086 | CA | THR | B | 364 | 21.916 | 26.225 | 50.864 | 1.00 | 68.37 | B | C |
| ATOM | 5087 | CB | THR | B | 364 | 22.425 | 27.541 | 51.477 | 1.00 | 46.75 | B | C |
| ATOM | 5088 | OG1 | THR | B | 364 | 21.414 | 28.076 | 52.341 | 1.00 | 46.75 | B | O |
| ATOM | 5089 | CG2 | THR | B | 364 | 22.771 | 28.539 | 50.384 | 1.00 | 46.75 | B | C |
| ATOM | 5090 | C | THR | B | 364 | 21.182 | 25.475 | 51.970 | 1.00 | 68.37 | B | C |
| ATOM | 5091 | O | THR | B | 364 | 21.811 | 24.803 | 52.794 | 1.00 | 68.37 | B | O |
| ATOM | 5092 | N | GLN | B | 365 | 19.856 | 25.610 | 51.993 | 1.00 | 42.99 | B | N |
| ATOM | 5093 | CA | GLN | B | 365 | 19.046 | 24.922 | 52.981 | 1.00 | 42.99 | B | C |
| ATOM | 5094 | CB | GLN | B | 365 | 17.612 | 25.438 | 52.946 | 1.00 | 52.17 | B | C |
| ATOM | 5095 | CG | GLN | B | 365 | 16.639 | 24.640 | 53.825 | 1.00 | 52.17 | B | C |
| ATOM | 5096 | CD | GLN | B | 365 | 16.827 | 24.891 | 55.311 | 1.00 | 52.17 | B | C |
| ATOM | 5097 | OE1 | GLN | B | 365 | 17.768 | 25.572 | 55.724 | 1.00 | 52.17 | B | O |
| ATOM | 5098 | NE2 | GLN | B | 365 | 15.932 | 24.340 | 56.123 | 1.00 | 52.17 | B | N |
| ATOM | 5099 | C | GLN | B | 365 | 19.064 | 23.428 | 52.668 | 1.00 | 42.99 | B | C |
| ATOM | 5100 | O | GLN | B | 365 | 19.195 | 22.605 | 53.566 | 1.00 | 42.99 | B | O |
| ATOM | 5101 | N | GLU | B | 366 | 18.935 | 23.082 | 51.391 | 1.00 | 45.28 | B | N |
| ATOM | 5102 | CA | GLU | B | 366 | 18.939 | 21.679 | 50.985 | 1.00 | 45.28 | B | C |
| ATOM | 5103 | CB | GLU | B | 366 | 18.597 | 21.524 | 49.495 | 1.00 | 51.55 | B | C |
| ATOM | 5104 | CG | GLU | B | 366 | 17.292 | 22.160 | 49.002 | 1.00 | 51.55 | B | C |
| ATOM | 5105 | CD | GLU | B | 366 | 17.040 | 21.860 | 47.520 | 1.00 | 51.55 | B | C |
| ATOM | 5106 | OE1 | GLU | B | 366 | 16.852 | 20.667 | 47.168 | 1.00 | 51.55 | B | O |
| ATOM | 5107 | OE2 | GLU | B | 366 | 17.038 | 22.813 | 46.710 | 1.00 | 51.55 | B | O |
| ATOM | 5108 | C | GLU | B | 366 | 20.327 | 21.093 | 51.217 | 1.00 | 45.28 | B | C |
| ATOM | 5109 | O | GLU | B | 366 | 20.472 | 19.998 | 51.777 | 1.00 | 45.28 | B | O |
| ATOM | 5110 | N | LEU | B | 367 | 21.342 | 21.834 | 50.778 | 1.00 | 56.74 | B | N |
| ATOM | 5111 | CA | LEU | B | 367 | 22.734 | 21.405 | 50.901 | 1.00 | 56.74 | B | C |
| ATOM | 5112 | CB | LEU | B | 367 | 23.595 | 22.201 | 49.922 | 1.00 | 60.11 | B | C |
| ATOM | 5113 | CG | LEU | B | 367 | 23.193 | 22.132 | 48.446 | 1.00 | 60.11 | B | C |
| ATOM | 5114 | CD1 | LEU | B | 367 | 23.625 | 23.407 | 47.727 | 1.00 | 60.11 | B | C |
| ATOM | 5115 | CD2 | LEU | B | 367 | 23.808 | 20.891 | 47.805 | 1.00 | 60.11 | B | C |
| ATOM | 5116 | C | LEU | B | 367 | 23.303 | 21.577 | 52.310 | 1.00 | 56.74 | B | C |
| ATOM | 5117 | O | LEU | B | 367 | 24.440 | 21.188 | 52.574 | 1.00 | 56.74 | B | O |
| ATOM | 5118 | N | SER | B | 368 | 22.514 | 22.138 | 53.219 | 1.00 | 55.54 | B | N |
| ATOM | 5119 | CA | SER | B | 368 | 23.005 | 22.387 | 54.566 | 1.00 | 55.54 | B | C |
| ATOM | 5120 | CB | SER | B | 368 | 21.879 | 22.943 | 55.446 | 1.00 | 46.32 | B | C |
| ATOM | 5121 | OG | SER | B | 368 | 20.856 | 21.989 | 55.633 | 1.00 | 46.32 | B | O |
| ATOM | 5122 | C | SER | B | 368 | 23.667 | 21.212 | 55.272 | 1.00 | 55.54 | B | C |
| ATOM | 5123 | O | SER | B | 368 | 24.812 | 21.353 | 55.751 | 1.00 | 55.54 | B | O |
| ATOM | 5124 | N | SER | B | 369 | 23.034 | 20.053 | 55.321 | 1.00 | 47.27 | B | N |
| ATOM | 5125 | CA | SER | B | 369 | 23.596 | 18.892 | 56.021 | 1.00 | 47.27 | B | C |
| ATOM | 5126 | CB | SER | B | 369 | 22.680 | 17.673 | 55.867 | 1.00 | 42.97 | B | C |
| ATOM | 5127 | OG | SER | B | 369 | 23.125 | 16.822 | 54.824 | 1.00 | 42.97 | B | O |
| ATOM | 5128 | C | SER | B | 369 | 25.013 | 18.491 | 55.610 | 1.00 | 47.27 | B | C |
| ATOM | 5129 | O | SER | B | 369 | 25.717 | 17.831 | 56.373 | 1.00 | 47.27 | B | O |
| ATOM | 5130 | N | ASN | B | 370 | 25.437 | 18.879 | 54.411 | 1.00 | 49.80 | B | N |
| ATOM | 5131 | CA | ASN | B | 370 | 26.771 | 18.520 | 53.944 | 1.00 | 49.80 | B | C |
| ATOM | 5132 | CB | ASN | B | 370 | 26.823 | 17.035 | 53.627 | 1.00 | 43.79 | B | C |
| ATOM | 5133 | CG | ASN | B | 370 | 28.221 | 16.563 | 53.338 | 1.00 | 43.79 | B | C |
| ATOM | 5134 | OD1 | ASN | B | 370 | 28.905 | 17.115 | 52.481 | 1.00 | 43.79 | B | O |
| ATOM | 5135 | ND2 | ASN | B | 370 | 28.660 | 15.534 | 54.053 | 1.00 | 43.79 | B | N |
| ATOM | 5136 | C | ASN | B | 370 | 27.178 | 19.313 | 52.705 | 1.00 | 49.80 | B | C |
| ATOM | 5137 | O | ASN | B | 370 | 27.340 | 18.754 | 51.612 | 1.00 | 49.80 | B | O |
| ATOM | 5138 | N | PRO | B | 371 | 27.369 | 20.630 | 52.868 | 1.00 | 42.27 | B | N |
| ATOM | 5139 | CD | PRO | B | 371 | 27.296 | 21.334 | 54.162 | 1.00 | 39.04 | B | C |
| ATOM | 5140 | CA | PRO | B | 371 | 27.752 | 21.551 | 51.800 | 1.00 | 42.27 | B | C |
| ATOM | 5141 | CB | PRO | B | 371 | 28.375 | 22.700 | 52.573 | 1.00 | 39.04 | B | C |
| ATOM | 5142 | CG | PRO | B | 371 | 27.456 | 22.782 | 53.757 | 1.00 | 39.04 | B | C |
| ATOM | 5143 | C | PRO | B | 371 | 28.646 | 21.035 | 50.664 | 1.00 | 42.27 | B | C |
| ATOM | 5144 | O | PRO | B | 371 | 28.243 | 21.028 | 49.502 | 1.00 | 42.27 | B | O |
| ATOM | 5145 | N | PRO | B | 372 | 29.862 | 20.576 | 50.977 | 1.00 | 51.61 | B | N |
| ATOM | 5146 | CD | PRO | B | 372 | 30.450 | 20.240 | 52.282 | 1.00 | 27.30 | B | C |
| ATOM | 5147 | CA | PRO | B | 372 | 30.718 | 20.100 | 49.888 | 1.00 | 51.61 | B | C |
| ATOM | 5148 | CB | PRO | B | 372 | 31.958 | 19.593 | 50.625 | 1.00 | 27.30 | B | C |
| ATOM | 5149 | CG | PRO | B | 372 | 31.402 | 19.131 | 51.908 | 1.00 | 27.30 | B | C |
| ATOM | 5150 | C | PRO | B | 372 | 30.137 | 19.070 | 48.903 | 1.00 | 51.61 | B | C |
| ATOM | 5151 | O | PRO | B | 372 | 30.739 | 18.819 | 47.850 | 1.00 | 51.61 | B | O |
| ATOM | 5152 | N | LEU | B | 373 | 28.989 | 18.473 | 49.218 | 1.00 | 40.62 | B | N |

| | | | | | | | | | | | | |
|---|---|---|---|---|---|---|---|---|---|---|---|---|
| ATOM | 5153 | CA | LEU | B | 373 | 28.408 | 17.506 | 48.293 | 1.00 | 40.62 | B | C |
| ATOM | 5154 | CB | LEU | B | 373 | 27.208 | 16.810 | 48.929 | 1.00 | 51.31 | B | C |
| ATOM | 5155 | CG | LEU | B | 373 | 27.582 | 15.557 | 49.741 | 1.00 | 51.31 | B | C |
| ATOM | 5156 | CD1 | LEU | B | 373 | 26.366 | 15.017 | 50.470 | 1.00 | 51.31 | B | C |
| ATOM | 5157 | CD2 | LEU | B | 373 | 28.150 | 14.493 | 48.808 | 1.00 | 51.31 | B | C |
| ATOM | 5158 | C | LEU | B | 373 | 27.993 | 18.200 | 46.995 | 1.00 | 40.62 | B | C |
| ATOM | 5159 | O | LEU | B | 373 | 27.749 | 17.559 | 45.963 | 1.00 | 40.62 | B | O |
| ATOM | 5160 | N | ALA | B | 374 | 27.950 | 19.525 | 47.061 | 1.00 | 46.33 | B | N |
| ATOM | 5161 | CA | ALA | B | 374 | 27.573 | 20.350 | 45.932 | 1.00 | 46.33 | B | C |
| ATOM | 5162 | CB | ALA | B | 374 | 27.552 | 21.807 | 46.357 | 1.00 | 26.93 | B | C |
| ATOM | 5163 | C | ALA | B | 374 | 28.498 | 20.166 | 44.733 | 1.00 | 46.33 | B | C |
| ATOM | 5164 | O | ALA | B | 374 | 28.106 | 20.434 | 43.592 | 1.00 | 46.33 | B | O |
| ATOM | 5165 | N | THR | B | 375 | 29.725 | 19.719 | 44.973 | 1.00 | 51.49 | B | N |
| ATOM | 5166 | CA | THR | B | 375 | 30.645 | 19.541 | 43.857 | 1.00 | 51.49 | B | C |
| ATOM | 5167 | CB | THR | B | 375 | 32.091 | 19.321 | 44.321 | 1.00 | 52.84 | B | C |
| ATOM | 5168 | OG1 | THR | B | 375 | 32.121 | 18.311 | 45.339 | 1.00 | 52.84 | B | O |
| ATOM | 5169 | CG2 | THR | B | 375 | 32.677 | 20.622 | 44.853 | 1.00 | 52.84 | B | C |
| ATOM | 5170 | C | THR | B | 375 | 30.224 | 18.359 | 43.018 | 1.00 | 51.49 | B | C |
| ATOM | 5171 | O | THR | B | 375 | 30.613 | 18.253 | 41.855 | 1.00 | 51.49 | B | O |
| ATOM | 5172 | N | ILE | B | 376 | 29.427 | 17.472 | 43.611 | 1.00 | 69.10 | B | N |
| ATOM | 5173 | CA | ILE | B | 376 | 28.948 | 16.298 | 42.891 | 1.00 | 69.10 | B | C |
| ATOM | 5174 | CB | ILE | B | 376 | 28.982 | 15.028 | 43.769 | 1.00 | 55.74 | B | C |
| ATOM | 5175 | CG2 | ILE | B | 376 | 28.553 | 13.817 | 42.944 | 1.00 | 55.74 | B | C |
| ATOM | 5176 | CG1 | ILE | B | 376 | 30.391 | 14.797 | 44.310 | 1.00 | 55.74 | B | C |
| ATOM | 5177 | CD1 | ILE | B | 376 | 30.526 | 13.519 | 45.140 | 1.00 | 55.74 | B | C |
| ATOM | 5178 | C | ILE | B | 376 | 27.510 | 16.519 | 42.424 | 1.00 | 69.10 | B | C |
| ATOM | 5179 | O | ILE | B | 376 | 27.174 | 16.256 | 41.265 | 1.00 | 69.10 | B | O |
| ATOM | 5180 | N | LEU | B | 377 | 26.669 | 17.014 | 43.328 | 1.00 | 53.55 | B | N |
| ATOM | 5181 | CA | LEU | B | 377 | 25.262 | 17.259 | 43.018 | 1.00 | 53.55 | B | C |
| ATOM | 5182 | CB | LEU | B | 377 | 24.524 | 17.693 | 44.281 | 1.00 | 53.61 | B | C |
| ATOM | 5183 | CG | LEU | B | 377 | 24.597 | 16.597 | 45.343 | 1.00 | 53.61 | B | C |
| ATOM | 5184 | CD1 | LEU | B | 377 | 24.124 | 17.131 | 46.679 | 1.00 | 53.61 | B | C |
| ATOM | 5185 | CD2 | LEU | B | 377 | 23.781 | 15.395 | 44.884 | 1.00 | 53.61 | B | C |
| ATOM | 5186 | C | LEU | B | 377 | 25.030 | 18.274 | 41.904 | 1.00 | 53.55 | B | C |
| ATOM | 5187 | O | LEU | B | 377 | 24.121 | 18.102 | 41.094 | 1.00 | 53.55 | B | O |
| ATOM | 5188 | N | ILE | B | 378 | 25.841 | 19.326 | 41.858 | 1.00 | 56.70 | B | N |
| ATOM | 5189 | CA | ILE | B | 378 | 25.697 | 20.349 | 40.823 | 1.00 | 56.70 | B | C |
| ATOM | 5190 | CB | ILE | B | 378 | 25.776 | 21.771 | 41.398 | 1.00 | 42.43 | B | C |
| ATOM | 5191 | CG2 | ILE | B | 378 | 25.682 | 22.772 | 40.270 | 1.00 | 42.43 | B | C |
| ATOM | 5192 | CG1 | ILE | B | 378 | 24.638 | 22.005 | 42.390 | 1.00 | 42.43 | B | C |
| ATOM | 5193 | CD1 | ILE | B | 378 | 24.727 | 23.313 | 43.136 | 1.00 | 42.43 | B | C |
| ATOM | 5194 | C | ILE | B | 378 | 26.806 | 20.189 | 39.796 | 1.00 | 56.70 | B | C |
| ATOM | 5195 | O | ILE | B | 378 | 27.880 | 20.783 | 39.924 | 1.00 | 56.70 | B | O |
| ATOM | 5196 | N | PRO | B | 379 | 26.558 | 19.378 | 38.756 | 1.00 | 60.86 | B | N |
| ATOM | 5197 | CD | PRO | B | 379 | 25.268 | 18.792 | 38.357 | 1.00 | 40.56 | B | C |
| ATOM | 5198 | CA | PRO | B | 379 | 27.566 | 19.160 | 37.723 | 1.00 | 60.86 | B | C |
| ATOM | 5199 | CB | PRO | B | 379 | 26.854 | 18.225 | 36.753 | 1.00 | 40.56 | B | C |
| ATOM | 5200 | CG | PRO | B | 379 | 25.430 | 18.660 | 36.871 | 1.00 | 40.56 | B | C |
| ATOM | 5201 | C | PRO | B | 379 | 27.970 | 20.486 | 37.092 | 1.00 | 60.86 | B | C |
| ATOM | 5202 | O | PRO | B | 379 | 27.224 | 21.470 | 37.156 | 1.00 | 60.86 | B | O |
| ATOM | 5203 | N | PRO | B | 380 | 29.162 | 20.531 | 36.479 | 1.00 | 78.88 | B | N |
| ATOM | 5204 | CD | PRO | B | 380 | 30.131 | 19.424 | 36.387 | 1.00 | 81.30 | B | C |
| ATOM | 5205 | CA | PRO | B | 380 | 29.690 | 21.736 | 35.831 | 1.00 | 78.88 | B | C |
| ATOM | 5206 | CB | PRO | B | 380 | 30.980 | 21.238 | 35.187 | 1.00 | 81.30 | B | C |
| ATOM | 5207 | CG | PRO | B | 380 | 31.426 | 20.161 | 36.153 | 1.00 | 81.30 | B | C |
| ATOM | 5208 | C | PRO | B | 380 | 28.743 | 22.393 | 34.821 | 1.00 | 78.88 | B | C |
| ATOM | 5209 | O | PRO | B | 380 | 28.437 | 23.586 | 34.944 | 1.00 | 78.88 | B | O |
| ATOM | 5210 | N | HIS | B | 381 | 28.277 | 21.629 | 33.830 | 1.00 | 61.90 | B | N |
| ATOM | 5211 | CA | HIS | B | 381 | 27.376 | 22.166 | 32.803 | 1.00 | 61.90 | B | C |
| ATOM | 5212 | CB | HIS | B | 381 | 26.906 | 21.038 | 31.879 | 1.00 | 63.66 | B | C |
| ATOM | 5213 | CG | HIS | B | 381 | 25.734 | 20.268 | 32.411 | 1.00 | 63.66 | B | C |
| ATOM | 5214 | CD2 | HIS | B | 381 | 25.666 | 19.076 | 33.052 | 1.00 | 63.66 | B | C |
| ATOM | 5215 | ND1 | HIS | B | 381 | 24.437 | 20.738 | 32.339 | 1.00 | 63.66 | B | N |
| ATOM | 5216 | CE1 | HIS | B | 381 | 23.622 | 19.869 | 32.912 | 1.00 | 63.66 | B | C |
| ATOM | 5217 | NE2 | HIS | B | 381 | 24.343 | 18.852 | 33.353 | 1.00 | 63.66 | B | N |
| ATOM | 5218 | C | HIS | B | 381 | 26.148 | 22.892 | 33.382 | 1.00 | 61.90 | B | C |
| ATOM | 5219 | O | HIS | B | 381 | 25.527 | 23.704 | 32.701 | 1.00 | 61.90 | B | O |

| | | | | | | | | | | | | |
|---|---|---|---|---|---|---|---|---|---|---|---|---|
| ATOM | 5220 | N | ALA | B | 382 | 25.783 | 22.601 | 34.627 | 1.00 | 75.54 | B | N |
| ATOM | 5221 | CA | ALA | B | 382 | 24.624 | 23.254 | 35.229 | 1.00 | 75.54 | B | C |
| ATOM | 5222 | CB | ALA | B | 382 | 23.912 | 22.291 | 36.163 | 1.00 | 65.68 | B | C |
| ATOM | 5223 | C | ALA | B | 382 | 25.023 | 24.530 | 35.984 | 1.00 | 75.54 | B | C |
| ATOM | 5224 | O | ALA | B | 382 | 24.157 | 25.305 | 36.439 | 1.00 | 75.54 | B | O |
| ATOM | 5225 | N | ARG | B | 383 | 26.335 | 24.742 | 36.123 | 1.00 | 79.78 | B | N |
| ATOM | 5226 | CA | ARG | B | 383 | 26.855 | 25.926 | 36.801 | 1.00 | 79.78 | B | C |
| ATOM | 5227 | CB | ARG | B | 383 | 28.351 | 25.748 | 37.102 | 1.00 | 75.02 | B | C |
| ATOM | 5228 | CG | ARG | B | 383 | 28.627 | 25.262 | 38.518 | 1.00 | 75.02 | B | C |
| ATOM | 5229 | CD | ARG | B | 383 | 28.969 | 23.785 | 38.604 | 1.00 | 75.02 | B | C |
| ATOM | 5230 | NE | ARG | B | 383 | 30.409 | 23.542 | 38.470 | 1.00 | 75.02 | B | N |
| ATOM | 5231 | CZ | ARG | B | 383 | 31.090 | 22.614 | 39.152 | 1.00 | 75.02 | B | C |
| ATOM | 5232 | NH1 | ARG | B | 383 | 30.476 | 21.823 | 40.036 | 1.00 | 75.02 | B | N |
| ATOM | 5233 | NH2 | ARG | B | 383 | 32.397 | 22.467 | 38.948 | 1.00 | 75.02 | B | N |
| ATOM | 5234 | C | ARG | B | 383 | 26.616 | 27.174 | 35.938 | 1.00 | 79.78 | B | C |
| ATOM | 5235 | O | ARG | B | 383 | 27.028 | 28.286 | 36.293 | 1.00 | 79.78 | B | O |
| ATOM | 5236 | N | ILE | B | 384 | 25.934 | 26.952 | 34.809 | 1.00100.00 | | B | N |
| ATOM | 5237 | CA | ILE | B | 384 | 25.561 | 27.977 | 33.816 | 1.00100.00 | | B | C |
| ATOM | 5238 | CB | ILE | B | 384 | 24.128 | 28.555 | 34.128 | 1.00 | 99.74 | B | C |
| ATOM | 5239 | CG2 | ILE | B | 384 | 23.581 | 29.336 | 32.905 | 1.00 | 99.74 | B | C |
| ATOM | 5240 | CG1 | ILE | B | 384 | 23.171 | 27.406 | 34.489 | 1.00 | 99.74 | B | C |
| ATOM | 5241 | CD1 | ILE | B | 384 | 21.698 | 27.831 | 34.666 | 1.00 | 99.74 | B | C |
| ATOM | 5242 | C | ILE | B | 384 | 26.555 | 29.152 | 33.649 | 1.00100.00 | | B | C |
| ATOM | 5243 | O | ILE | B | 384 | 27.205 | 29.217 | 32.566 | 1.00100.00 | | B | O |
| ATOM | 5244 | OXT | ILE | B | 384 | 26.671 | 29.989 | 34.591 | 1.00 | 99.74 | B | O |
| TER | 5245 | | ILE | B | 384 | | | | | | B | |
| ATOM | 5246 | C1 | 4A | I | 1 | 10.253 | 5.669 | 5.097 | 1.00 | 70.71 | I | C |
| ATOM | 5247 | C2 | 4A | I | 1 | 10.050 | 4.331 | 5.628 | 1.00 | 70.71 | I | C |
| ATOM | 5248 | C3 | 4A | I | 1 | 9.835 | 3.230 | 4.683 | 1.00 | 70.71 | I | C |
| ATOM | 5249 | C4 | 4A | I | 1 | 9.812 | 3.459 | 3.221 | 1.00 | 70.71 | I | C |
| ATOM | 5250 | C5 | 4A | I | 1 | 10.017 | 4.802 | 2.685 | 1.00 | 70.71 | I | C |
| ATOM | 5251 | C6 | 4A | I | 1 | 10.241 | 5.919 | 3.625 | 1.00 | 70.71 | I | C |
| ATOM | 5252 | C12 | 4A | I | 1 | 9.669 | 1.857 | 5.189 | 1.00 | 70.71 | I | C |
| ATOM | 5253 | N13 | 4A | I | 1 | 8.568 | 1.644 | 6.036 | 1.00 | 70.71 | I | N |
| ATOM | 5254 | N14 | 4A | I | 1 | 8.268 | 0.428 | 6.607 | 1.00 | 70.71 | I | N |
| ATOM | 5255 | C15 | 4A | I | 1 | 9.024 | -0.745 | 6.430 | 1.00 | 70.71 | I | C |
| ATOM | 5256 | C16 | 4A | I | 1 | 10.190 | -0.720 | 5.584 | 1.00 | 70.71 | I | C |
| ATOM | 5257 | C17 | 4A | I | 1 | 10.549 | 0.584 | 4.906 | 1.00 | 70.71 | I | C |
| ATOM | 5258 | C18 | 4A | I | 1 | 11.664 | 0.642 | 3.864 | 1.00 | 70.71 | I | C |
| ATOM | 5259 | C19 | 4A | I | 1 | 12.881 | 1.458 | 4.005 | 1.00 | 70.71 | I | C |
| ATOM | 5260 | C20 | 4A | I | 1 | 13.847 | 1.510 | 2.891 | 1.00 | 70.71 | I | C |
| ATOM | 5261 | C21 | 4A | I | 1 | 13.597 | 0.739 | 1.633 | 1.00 | 70.71 | I | C |
| ATOM | 5262 | C22 | 4A | I | 1 | 12.378 | -0.100 | 1.477 | 1.00 | 70.71 | I | C |
| ATOM | 5263 | C23 | 4A | I | 1 | 11.427 | -0.146 | 2.581 | 1.00 | 70.71 | I | C |
| ATOM | 5264 | N29 | 4A | I | 1 | 8.887 | -2.028 | 6.887 | 1.00 | 70.71 | I | N |
| ATOM | 5265 | N1 | 4A | I | 1 | 9.907 | -2.787 | 6.360 | 1.00 | 70.71 | I | N |
| ATOM | 5266 | C31 | 4A | I | 1 | 10.738 | -2.052 | 5.564 | 1.00 | 70.71 | I | C |
| ATOM | 5267 | N32 | 4A | I | 1 | 11.990 | -2.299 | 4.816 | 1.00 | 70.71 | I | N |
| TER | 5268 | | 4A | I | 1 | | | | | | I | |
| ATOM | 5269 | C1 | 4B | J | 1 | -4.156 | 13.281 | 52.166 | 1.00 | 63.32 | J | C |
| ATOM | 5270 | C2 | 4B | J | 1 | -3.989 | 13.950 | 50.879 | 1.00 | 63.32 | J | C |
| ATOM | 5271 | C3 | 4B | J | 1 | -3.476 | 13.224 | 49.702 | 1.00 | 63.32 | J | C |
| ATOM | 5272 | C4 | 4B | J | 1 | -3.138 | 11.794 | 49.844 | 1.00 | 63.32 | J | C |
| ATOM | 5273 | C5 | 4B | J | 1 | -3.314 | 11.124 | 51.155 | 1.00 | 63.32 | J | C |
| ATOM | 5274 | C6 | 4B | J | 1 | -3.821 | 11.866 | 52.320 | 1.00 | 63.32 | J | C |
| ATOM | 5275 | C12 | 4B | J | 1 | -3.263 | 13.932 | 48.405 | 1.00 | 63.32 | J | C |
| ATOM | 5276 | N13 | 4B | J | 1 | -2.461 | 15.111 | 48.508 | 1.00 | 63.32 | J | N |
| ATOM | 5277 | N14 | 4B | J | 1 | -2.104 | 15.935 | 47.447 | 1.00 | 63.32 | J | N |
| ATOM | 5278 | C15 | 4B | J | 1 | -2.495 | 15.715 | 46.132 | 1.00 | 63.32 | J | C |
| ATOM | 5279 | C16 | 4B | J | 1 | -3.308 | 14.574 | 45.818 | 1.00 | 63.32 | J | C |
| ATOM | 5280 | C17 | 4B | J | 1 | -3.735 | 13.610 | 46.936 | 1.00 | 63.32 | J | C |
| ATOM | 5281 | C18 | 4B | J | 1 | -4.555 | 12.369 | 46.537 | 1.00 | 63.32 | J | C |
| ATOM | 5282 | C19 | 4B | J | 1 | -5.940 | 12.170 | 46.996 | 1.00 | 63.32 | J | C |
| ATOM | 5283 | C20 | 4B | J | 1 | -6.688 | 10.974 | 46.565 | 1.00 | 63.32 | J | C |
| ATOM | 5284 | C21 | 4B | J | 1 | -6.066 | 9.967 | 45.672 | 1.00 | 63.32 | J | C |
| ATOM | 5285 | C22 | 4B | J | 1 | -4.680 | 10.139 | 45.198 | 1.00 | 63.32 | J | C |
| ATOM | 5286 | C23 | 4B | J | 1 | -3.939 | 11.317 | 45.626 | 1.00 | 63.32 | J | C |

| | | | | | | | | | | | | |
|---|---|---|---|---|---|---|---|---|---|---|---|---|
| ATOM | 5287 | N29 | 4B | J | 1 | -2.278 | 16.371 | 44.945 | 1.00 | 63.32 | J | N |
| ATOM | 5288 | N1 | 4B | J | 1 | -2.883 | 15.738 | 43.909 | 1.00 | 63.32 | J | N |
| ATOM | 5289 | C31 | 4B | J | 1 | -3.533 | 14.629 | 44.384 | 1.00 | 63.32 | J | C |
| ATOM | 5290 | N32 | 4B | J | 1 | -4.412 | 13.571 | 43.807 | 1.00 | 63.32 | J | N |
| TER | 5291 | | 4B | J | 1 | | | | | | J | |
| ATOM | 5292 | O | HOH | W | 1 | -1.499 | 8.933 | -13.094 | 1.00 | 26.17 | W | O |
| ATOM | 5293 | O | HOH | W | 2 | -3.474 | 10.448 | -6.384 | 1.00 | 20.73 | W | O |
| ATOM | 5294 | O | HOH | W | 3 | 10.121 | -2.709 | 55.746 | 1.00 | 23.52 | W | O |
| ATOM | 5295 | O | HOH | W | 4 | 9.265 | 10.406 | 29.771 | 1.00 | 32.87 | W | O |
| ATOM | 5296 | O | HOH | W | 5 | 2.433 | -1.556 | 53.562 | 1.00 | 25.79 | W | O |
| ATOM | 5297 | O | HOH | W | 6 | -10.271 | 12.840 | -5.136 | 1.00 | 28.43 | W | O |
| ATOM | 5298 | O | HOH | W | 7 | -9.544 | 22.066 | -6.134 | 1.00 | 51.67 | W | O |
| ATOM | 5299 | O | HOH | W | 8 | 3.275 | 10.739 | 67.679 | 1.00 | 37.05 | W | O |
| ATOM | 5300 | O | HOH | W | 9 | 6.641 | 8.203 | -10.828 | 1.00 | 34.94 | W | O |
| ATOM | 5301 | O | HOH | W | 10 | 1.509 | 17.175 | -11.339 | 1.00 | 31.08 | W | O |
| ATOM | 5302 | O | HOH | W | 11 | -9.030 | 31.029 | 55.887 | 1.00 | 48.32 | W | O |
| ATOM | 5303 | O | HOH | W | 12 | 5.198 | -3.326 | 62.884 | 1.00 | 39.14 | W | O |
| ATOM | 5304 | O | HOH | W | 13 | -3.865 | 13.932 | 9.234 | 1.00 | 40.44 | W | O |
| ATOM | 5305 | O | HOH | W | 15 | 2.173 | 7.983 | 2.977 | 1.00 | 30.25 | W | O |
| ATOM | 5306 | O | HOH | W | 16 | -0.614 | -9.702 | -15.250 | 1.00 | 33.85 | W | O |
| ATOM | 5307 | O | HOH | W | 17 | 22.550 | 16.289 | 41.249 | 1.00 | 45.82 | W | O |
| ATOM | 5308 | O | HOH | W | 18 | 14.655 | 17.087 | 41.536 | 1.00 | 43.86 | W | O |
| ATOM | 5309 | O | HOH | W | 19 | -11.850 | 29.586 | 56.221 | 1.00 | 37.36 | W | O |
| ATOM | 5310 | O | HOH | W | 20 | 16.052 | 2.949 | 31.753 | 1.00 | 26.10 | W | O |
| ATOM | 5311 | O | HOH | W | 21 | -8.034 | 18.041 | 4.405 | 1.00 | 43.98 | W | O |
| ATOM | 5312 | O | HOH | W | 22 | 1.718 | -0.681 | 48.664 | 1.00 | 36.25 | W | O |
| ATOM | 5313 | O | HOH | W | 23 | -2.152 | -16.363 | -12.425 | 1.00 | 35.35 | W | O |
| ATOM | 5314 | O | HOH | W | 24 | -15.048 | 7.668 | -5.828 | 1.00 | 36.07 | W | O |
| ATOM | 5315 | O | HOH | W | 25 | 3.655 | 11.966 | -1.762 | 1.00 | 42.93 | W | O |
| ATOM | 5316 | O | HOH | W | 26 | 8.730 | 6.832 | 55.789 | 1.00 | 42.73 | W | O |
| ATOM | 5317 | O | HOH | W | 27 | -6.767 | -9.603 | 3.297 | 1.00 | 62.80 | W | O |
| ATOM | 5318 | O | HOH | W | 28 | 1.470 | 5.443 | -14.392 | 1.00 | 37.15 | W | O |
| ATOM | 5319 | O | HOH | W | 29 | 20.700 | 14.552 | 53.440 | 1.00 | 41.48 | W | O |
| ATOM | 5320 | O | HOH | W | 30 | -14.476 | -3.140 | 13.989 | 1.00 | 42.42 | W | O |
| ATOM | 5321 | O | HOH | W | 31 | 18.723 | 28.062 | 51.847 | 1.00 | 40.69 | W | O |
| ATOM | 5322 | O | HOH | W | 32 | 10.155 | 3.921 | 58.239 | 1.00 | 32.93 | W | O |
| ATOM | 5323 | O | HOH | W | 33 | 9.437 | 7.018 | 23.941 | 1.00 | 46.93 | W | O |
| ATOM | 5324 | O | HOH | W | 34 | 5.230 | -3.459 | 20.049 | 1.00 | 39.47 | W | O |
| ATOM | 5325 | O | HOH | W | 37 | 11.281 | -1.146 | -3.827 | 1.00 | 58.71 | W | O |
| ATOM | 5326 | O | HOH | W | 38 | 6.505 | -14.066 | -6.485 | 1.00 | 50.26 | W | O |
| ATOM | 5327 | O | HOH | W | 39 | 8.895 | 4.730 | -1.163 | 1.00 | 71.70 | W | O |
| ATOM | 5328 | O | HOH | W | 40 | 7.784 | 12.669 | 8.842 | 1.00 | 52.29 | W | O |
| ATOM | 5329 | O | HOH | W | 41 | 8.145 | 8.969 | 3.989 | 1.00 | 36.14 | W | O |
| ATOM | 5330 | O | HOH | W | 42 | -1.337 | 19.127 | 3.100 | 1.00 | 30.02 | W | O |
| ATOM | 5331 | O | HOH | W | 43 | 6.002 | 14.573 | -4.459 | 1.00 | 49.52 | W | O |
| ATOM | 5332 | O | HOH | W | 44 | 1.275 | 2.485 | -13.625 | 1.00 | 39.24 | W | O |
| ATOM | 5333 | O | HOH | W | 45 | -2.940 | 21.659 | -16.749 | 1.00 | 30.01 | W | O |
| ATOM | 5334 | O | HOH | W | 46 | -14.450 | 18.693 | -6.321 | 1.00 | 42.63 | W | O |
| ATOM | 5335 | O | HOH | W | 47 | 7.691 | -2.124 | -14.096 | 1.00 | 51.24 | W | O |
| ATOM | 5336 | O | HOH | W | 48 | 2.797 | 3.725 | -22.272 | 1.00 | 54.13 | W | O |
| ATOM | 5337 | O | HOH | W | 49 | 12.806 | 18.864 | -20.430 | 1.00 | 47.31 | W | O |
| ATOM | 5338 | O | HOH | W | 50 | 7.761 | 17.426 | -13.700 | 1.00 | 47.08 | W | O |
| ATOM | 5339 | O | HOH | W | 51 | 4.041 | 17.228 | -23.197 | 1.00 | 39.31 | W | O |
| ATOM | 5340 | O | HOH | W | 52 | -10.596 | 4.345 | -22.103 | 1.00 | 44.02 | W | O |
| ATOM | 5341 | O | HOH | W | 53 | -12.482 | 8.523 | -20.065 | 1.00 | 40.66 | W | O |
| ATOM | 5342 | O | HOH | W | 54 | -12.024 | 19.964 | -23.235 | 1.00 | 54.21 | W | O |
| ATOM | 5343 | O | HOH | W | 55 | -19.664 | 13.333 | -15.537 | 1.00 | 22.84 | W | O |
| ATOM | 5344 | O | HOH | W | 56 | -18.788 | 8.054 | -5.562 | 1.00 | 42.10 | W | O |
| ATOM | 5345 | O | HOH | W | 57 | -19.406 | 4.325 | -6.364 | 1.00 | 29.43 | W | O |
| ATOM | 5346 | O | HOH | W | 58 | 3.342 | -18.639 | -4.174 | 1.00 | 40.50 | W | O |
| ATOM | 5347 | O | HOH | W | 59 | -1.816 | 30.431 | 46.686 | 1.00 | 38.80 | W | O |
| ATOM | 5348 | O | HOH | W | 60 | -15.890 | 21.282 | 50.428 | 1.00 | 39.30 | W | O |
| ATOM | 5349 | O | HOH | W | 61 | -17.132 | 24.941 | 51.488 | 1.00 | 51.28 | W | O |
| ATOM | 5350 | O | HOH | W | 62 | -15.135 | 15.048 | 36.943 | 1.00 | 46.52 | W | O |
| ATOM | 5351 | O | HOH | W | 63 | -4.489 | 22.460 | 36.793 | 1.00 | 51.69 | W | O |
| ATOM | 5352 | O | HOH | W | 65 | 0.311 | 13.432 | 37.969 | 1.00 | 64.84 | W | O |
| ATOM | 5353 | O | HOH | W | 66 | 19.128 | 13.434 | 56.847 | 1.00 | 42.91 | W | O |

| | | | | | | | | | | | | |
|---|---|---|---|---|---|---|---|---|---|---|---|---|
| ATOM | 5354 | O | HOH | W | 67 | -1.018 | 16.062 | 53.046 | 1.00 | 52.19 | W | O |
| ATOM | 5355 | O | HOH | W | 68 | 6.056 | 15.597 | 70.872 | 1.00 | 56.66 | W | O |
| ATOM | 5356 | O | HOH | W | 69 | 6.977 | 14.981 | 76.128 | 1.00 | 59.64 | W | O |
| ATOM | 5357 | O | HOH | W | 70 | 0.063 | 5.262 | 69.337 | 1.00 | 45.62 | W | O |
| ATOM | 5358 | O | HOH | W | 71 | 6.082 | 8.758 | 72.399 | 1.00 | 60.12 | W | O |
| ATOM | 5359 | O | HOH | W | 72 | -0.244 | 3.476 | 60.263 | 1.00 | 30.00 | W | O |
| ATOM | 5360 | O | HOH | W | 73 | 15.902 | 5.786 | 62.704 | 1.00 | 52.39 | W | O |
| ATOM | 5361 | O | HOH | W | 74 | 8.904 | -2.399 | 48.896 | 1.00 | 25.48 | W | O |
| ATOM | 5362 | O | HOH | W | 75 | 27.098 | -6.257 | 62.272 | 1.00 | 43.45 | W | O |
| ATOM | 5363 | O | HOH | W | 76 | 26.572 | 7.921 | 56.951 | 1.00 | 25.95 | W | O |
| ATOM | 5364 | O | HOH | W | 77 | 28.602 | 5.817 | 52.562 | 1.00 | 35.56 | W | O |
| ATOM | 5365 | O | HOH | W | 78 | 21.827 | 7.530 | 58.421 | 1.00 | 32.42 | W | O |
| ATOM | 5366 | O | HOH | W | 79 | 21.891 | 17.290 | 33.040 | 1.00 | 59.23 | W | O |
| ATOM | 5367 | O | HOH | W | 80 | -4.881 | 9.861 | 41.937 | 1.00 | 61.23 | W | O |
| ATOM | 5368 | O | HOH | W | 81 | 13.331 | -3.295 | 0.316 | 1.00 | 42.38 | W | O |
| ATOM | 5369 | O | HOH | W | 82 | -5.714 | -8.707 | 7.351 | 1.00 | 32.38 | W | O |
| ATOM | 5370 | O | HOH | W | 83 | 13.104 | 21.394 | 43.241 | 1.00 | 31.90 | W | O |
| TER | 5371 | | HOH | W | 83 | | | | | | W | |
| END | | | | | | | | | | | | |

# FIG. 4

| | Atom | Type | Resid | | # | X | Y | Z | OCC | B | | |
|---|---|---|---|---|---|---|---|---|---|---|---|---|
| ATOM | 1 | CB | SER | A | 25 | 49.677 | 12.283 | 58.759 | 1.00 | 77.22 | A | C |
| ATOM | 2 | OG | SER | A | 25 | 50.798 | 12.476 | 59.618 | 1.00 | 77.22 | A | O |
| ATOM | 3 | C | SER | A | 25 | 49.980 | 14.594 | 57.816 | 1.00 | 76.64 | A | C |
| ATOM | 4 | O | SER | A | 25 | 50.297 | 14.490 | 56.622 | 1.00 | 76.64 | A | O |
| ATOM | 5 | N | SER | A | 25 | 47.851 | 13.400 | 57.467 | 1.00 | 76.64 | A | N |
| ATOM | 6 | CA | SER | A | 25 | 48.974 | 13.616 | 58.437 | 1.00 | 76.64 | A | C |
| ATOM | 7 | N | MET | A | 26 | 50.486 | 15.530 | 58.625 | 1.00 | 62.89 | A | N |
| ATOM | 8 | CA | MET | A | 26 | 51.448 | 16.538 | 58.155 | 1.00 | 62.89 | A | C |
| ATOM | 9 | CB | MET | A | 26 | 51.284 | 17.826 | 58.955 | 1.00 | 69.43 | A | C |
| ATOM | 10 | CG | MET | A | 26 | 50.016 | 18.581 | 58.620 | 1.00 | 69.43 | A | C |
| ATOM | 11 | SD | MET | A | 26 | 49.425 | 19.512 | 60.053 | 1.00 | 69.43 | A | S |
| ATOM | 12 | CE | MET | A | 26 | 50.822 | 20.744 | 60.221 | 1.00 | 69.43 | A | C |
| ATOM | 13 | C | MET | A | 26 | 52.918 | 16.126 | 58.197 | 1.00 | 62.89 | A | C |
| ATOM | 14 | O | MET | A | 26 | 53.367 | 15.462 | 59.134 | 1.00 | 62.89 | A | O |
| ATOM | 15 | N | LYS | A | 27 | 53.667 | 16.545 | 57.182 | 1.00 | 63.46 | A | N |
| ATOM | 16 | CA | LYS | A | 27 | 55.088 | 16.236 | 57.085 | 1.00 | 63.46 | A | C |
| ATOM | 17 | CB | LYS | A | 27 | 55.333 | 15.288 | 55.904 | 1.00 | 57.46 | A | C |
| ATOM | 18 | CG | LYS | A | 27 | 56.509 | 14.335 | 56.083 | 1.00 | 57.46 | A | C |
| ATOM | 19 | CD | LYS | A | 27 | 56.059 | 12.990 | 56.666 | 1.00 | 57.46 | A | C |
| ATOM | 20 | CE | LYS | A | 27 | 55.268 | 12.133 | 55.643 | 1.00 | 57.46 | A | C |
| ATOM | 21 | NZ | LYS | A | 27 | 56.131 | 11.611 | 54.521 | 1.00 | 57.46 | A | N |
| ATOM | 22 | C | LYS | A | 27 | 55.809 | 17.571 | 56.855 | 1.00 | 63.46 | A | C |
| ATOM | 23 | O | LYS | A | 27 | 55.912 | 18.053 | 55.714 | 1.00 | 63.46 | A | O |
| ATOM | 24 | N | VAL | A | 28 | 56.309 | 18.167 | 57.934 | 1.00 | 59.71 | A | N |
| ATOM | 25 | CA | VAL | A | 28 | 56.988 | 19.471 | 57.844 | 1.00 | 59.71 | A | C |
| ATOM | 26 | CB | VAL | A | 28 | 56.688 | 20.321 | 59.104 | 1.00 | 58.68 | A | C |
| ATOM | 27 | CG1 | VAL | A | 28 | 56.827 | 19.449 | 60.346 | 1.00 | 58.68 | A | C |
| ATOM | 28 | CG2 | VAL | A | 28 | 57.659 | 21.515 | 59.189 | 1.00 | 58.68 | A | C |
| ATOM | 29 | C | VAL | A | 28 | 58.514 | 19.458 | 57.633 | 1.00 | 59.71 | A | C |
| ATOM | 30 | O | VAL | A | 28 | 59.269 | 19.085 | 58.536 | 1.00 | 59.71 | A | O |
| ATOM | 31 | N | GLY | A | 29 | 58.954 | 19.902 | 56.453 | 1.00 | 45.44 | A | N |
| ATOM | 32 | CA | GLY | A | 29 | 60.372 | 19.948 | 56.124 | 1.00 | 45.44 | A | C |
| ATOM | 33 | C | GLY | A | 29 | 60.706 | 21.326 | 55.590 | 1.00 | 45.44 | A | C |
| ATOM | 34 | O | GLY | A | 29 | 59.909 | 21.922 | 54.870 | 1.00 | 45.44 | A | O |
| ATOM | 35 | N | ARG | A | 30 | 61.879 | 21.844 | 55.939 | 1.00 | 87.33 | A | N |
| ATOM | 36 | CA | ARG | A | 30 | 62.293 | 23.179 | 55.490 | 1.00 | 87.33 | A | C |
| ATOM | 37 | CB | ARG | A | 30 | 63.008 | 23.915 | 56.637 | 1.00 | 70.93 | A | C |
| ATOM | 38 | CG | ARG | A | 30 | 63.531 | 25.306 | 56.281 | 1.00 | 70.93 | A | C |
| ATOM | 39 | CD | ARG | A | 30 | 64.167 | 25.982 | 57.480 | 1.00 | 70.93 | A | C |
| ATOM | 40 | NE | ARG | A | 30 | 63.179 | 26.355 | 58.504 | 1.00 | 70.93 | A | N |
| ATOM | 41 | CZ | ARG | A | 30 | 62.657 | 25.531 | 59.422 | 1.00 | 70.93 | A | C |
| ATOM | 42 | NH1 | ARG | A | 30 | 61.760 | 25.983 | 60.304 | 1.00 | 70.93 | A | N |
| ATOM | 43 | NH2 | ARG | A | 30 | 63.035 | 24.258 | 59.474 | 1.00 | 70.93 | A | N |
| ATOM | 44 | C | ARG | A | 30 | 63.209 | 23.143 | 54.250 | 1.00 | 87.33 | A | C |
| ATOM | 45 | O | ARG | A | 30 | 64.440 | 22.992 | 54.370 | 1.00 | 87.33 | A | O |
| ATOM | 46 | N | GLY | A | 31 | 62.621 | 23.301 | 53.064 | 1.00 | 66.72 | A | N |
| ATOM | 47 | CA | GLY | A | 31 | 63.428 | 23.265 | 51.853 | 1.00 | 66.72 | A | C |
| ATOM | 48 | C | GLY | A | 31 | 63.292 | 24.475 | 50.945 | 1.00 | 66.72 | A | C |
| ATOM | 49 | O | GLY | A | 31 | 62.246 | 25.145 | 50.941 | 1.00 | 66.72 | A | O |
| ATOM | 50 | N | GLY | A | 32 | 64.349 | 24.743 | 50.166 | 1.00100.00 | | A | N |
| ATOM | 51 | CA | GLY | A | 32 | 64.355 | 25.882 | 49.257 | 1.00100.00 | | A | C |
| ATOM | 52 | C | GLY | A | 32 | 64.616 | 27.197 | 49.995 | 1.00100.00 | | A | C |
| ATOM | 53 | O | GLY | A | 32 | 63.920 | 27.531 | 50.992 | 1.00100.00 | | A | O |
| ATOM | 54 | N | GLY | A | 33 | 65.621 | 27.940 | 49.510 | 1.00 | 74.90 | A | N |
| ATOM | 55 | CA | GLY | A | 33 | 66.002 | 29.208 | 50.123 | 1.00 | 74.90 | A | C |
| ATOM | 56 | C | GLY | A | 33 | 65.695 | 29.288 | 51.624 | 1.00 | 74.90 | A | C |
| ATOM | 57 | O | GLY | A | 33 | 65.168 | 30.296 | 52.116 | 1.00 | 74.90 | A | O |
| ATOM | 58 | N | GLY | A | 34 | 65.998 | 28.216 | 52.356 | 1.00 | 74.22 | A | N |
| ATOM | 59 | CA | GLY | A | 34 | 65.745 | 28.202 | 53.791 | 1.00 | 74.22 | A | C |
| ATOM | 60 | C | GLY | A | 34 | 64.337 | 28.566 | 54.271 | 1.00 | 74.22 | A | C |
| ATOM | 61 | O | GLY | A | 34 | 64.146 | 28.959 | 55.433 | 1.00 | 74.22 | A | O |

| | | | | | | | | | | | | |
|---|---|---|---|---|---|---|---|---|---|---|---|---|
| ATOM | 62 | N | GLY | A | 35 | 63.343 | 28.451 | 53.393 | 1.00 | 88.79 | A | N |
| ATOM | 63 | CA | GLY | A | 35 | 61.979 | 28.754 | 53.801 | 1.00 | 88.79 | A | C |
| ATOM | 64 | C | GLY | A | 35 | 61.375 | 27.487 | 54.392 | 1.00 | 88.79 | A | C |
| ATOM | 65 | O | GLY | A | 35 | 61.716 | 26.385 | 53.937 | 1.00 | 88.79 | A | O |
| ATOM | 66 | N | LYS | A | 36 | 60.515 | 27.607 | 55.405 | 1.00 | 59.36 | A | N |
| ATOM | 67 | CA | LYS | A | 36 | 59.888 | 26.415 | 55.987 | 1.00 | 59.36 | A | C |
| ATOM | 68 | CB | LYS | A | 36 | 59.296 | 26.711 | 57.359 | 1.00 | 44.45 | A | C |
| ATOM | 69 | CG | LYS | A | 36 | 58.399 | 25.587 | 57.832 | 1.00 | 44.45 | A | C |
| ATOM | 70 | CD | LYS | A | 36 | 57.847 | 25.826 | 59.217 | 1.00 | 44.45 | A | C |
| ATOM | 71 | CE | LYS | A | 36 | 56.700 | 24.854 | 59.475 | 1.00 | 44.45 | A | C |
| ATOM | 72 | NZ | LYS | A | 36 | 56.252 | 24.849 | 60.899 | 1.00 | 44.45 | A | N |
| ATOM | 73 | C | LYS | A | 36 | 58.772 | 25.864 | 55.087 | 1.00 | 59.36 | A | C |
| ATOM | 74 | O | LYS | A | 36 | 58.139 | 26.624 | 54.344 | 1.00 | 59.36 | A | O |
| ATOM | 75 | N | VAL | A | 37 | 58.523 | 24.554 | 55.168 | 1.00 | 48.63 | A | N |
| ATOM | 76 | CA | VAL | A | 37 | 57.492 | 23.917 | 54.341 | 1.00 | 48.63 | A | C |
| ATOM | 77 | CB | VAL | A | 37 | 58.109 | 23.287 | 53.069 | 1.00 | 26.72 | A | C |
| ATOM | 78 | CG1 | VAL | A | 37 | 57.012 | 22.685 | 52.200 | 1.00 | 26.72 | A | C |
| ATOM | 79 | CG2 | VAL | A | 37 | 58.896 | 24.334 | 52.296 | 1.00 | 26.72 | A | C |
| ATOM | 80 | C | VAL | A | 37 | 56.646 | 22.830 | 55.014 | 1.00 | 48.63 | A | C |
| ATOM | 81 | O | VAL | A | 37 | 57.166 | 21.953 | 55.715 | 1.00 | 48.63 | A | O |
| ATOM | 82 | N | THR | A | 38 | 55.333 | 22.898 | 54.792 | 1.00 | 43.29 | A | N |
| ATOM | 83 | CA | THR | A | 38 | 54.414 | 21.900 | 55.329 | 1.00 | 43.29 | A | C |
| ATOM | 84 | CB | THR | A | 38 | 53.210 | 22.535 | 56.069 | 1.00 | 22.74 | A | C |
| ATOM | 85 | OG1 | THR | A | 38 | 53.681 | 23.437 | 57.073 | 1.00 | 22.74 | A | O |
| ATOM | 86 | CG2 | THR | A | 38 | 52.376 | 21.459 | 56.755 | 1.00 | 22.74 | A | C |
| ATOM | 87 | C | THR | A | 38 | 53.885 | 21.091 | 54.145 | 1.00 | 43.29 | A | C |
| ATOM | 88 | O | THR | A | 38 | 53.501 | 21.646 | 53.108 | 1.00 | 43.29 | A | O |
| ATOM | 89 | N | THR | A | 39 | 53.876 | 19.775 | 54.291 | 1.00 | 27.07 | A | N |
| ATOM | 90 | CA | THR | A | 39 | 53.379 | 18.927 | 53.229 | 1.00 | 27.07 | A | C |
| ATOM | 91 | CB | THR | A | 39 | 54.531 | 18.146 | 52.549 | 1.00 | 40.75 | A | C |
| ATOM | 92 | OG1 | THR | A | 39 | 55.214 | 19.012 | 51.633 | 1.00 | 40.75 | A | O |
| ATOM | 93 | CG2 | THR | A | 39 | 54.000 | 16.944 | 51.784 | 1.00 | 40.75 | A | C |
| ATOM | 94 | C | THR | A | 39 | 52.368 | 17.966 | 53.804 | 1.00 | 27.07 | A | C |
| ATOM | 95 | O | THR | A | 39 | 52.627 | 17.301 | 54.802 | 1.00 | 27.07 | A | O |
| ATOM | 96 | N | VAL | A | 40 | 51.206 | 17.891 | 53.177 | 1.00 | 38.31 | A | N |
| ATOM | 97 | CA | VAL | A | 40 | 50.173 | 17.004 | 53.671 | 1.00 | 38.31 | A | C |
| ATOM | 98 | CB | VAL | A | 40 | 49.196 | 17.759 | 54.581 | 1.00 | 55.79 | A | C |
| ATOM | 99 | CG1 | VAL | A | 40 | 48.321 | 18.709 | 53.737 | 1.00 | 55.79 | A | C |
| ATOM | 100 | CG2 | VAL | A | 40 | 48.355 | 16.756 | 55.369 | 1.00 | 55.79 | A | C |
| ATOM | 101 | C | VAL | A | 40 | 49.392 | 16.416 | 52.503 | 1.00 | 38.31 | A | C |
| ATOM | 102 | O | VAL | A | 40 | 49.392 | 16.972 | 51.403 | 1.00 | 38.31 | A | O |
| ATOM | 103 | N | VAL | A | 41 | 48.737 | 15.283 | 52.738 | 1.00 | 39.91 | A | N |
| ATOM | 104 | CA | VAL | A | 41 | 47.918 | 14.681 | 51.699 | 1.00 | 39.91 | A | C |
| ATOM | 105 | CB | VAL | A | 41 | 48.000 | 13.135 | 51.703 | 1.00 | 39.28 | A | C |
| ATOM | 106 | CG1 | VAL | A | 41 | 47.414 | 12.584 | 50.400 | 1.00 | 39.28 | A | C |
| ATOM | 107 | CG2 | VAL | A | 41 | 49.450 | 12.688 | 51.838 | 1.00 | 39.28 | A | C |
| ATOM | 108 | C | VAL | A | 41 | 46.484 | 15.125 | 51.976 | 1.00 | 39.91 | A | C |
| ATOM | 109 | O | VAL | A | 41 | 45.936 | 14.848 | 53.044 | 1.00 | 39.91 | A | O |
| ATOM | 110 | N | ALA | A | 42 | 45.885 | 15.834 | 51.022 | 1.00 | 43.86 | A | N |
| ATOM | 111 | CA | ALA | A | 42 | 44.521 | 16.328 | 51.193 | 1.00 | 43.86 | A | C |
| ATOM | 112 | CB | ALA | A | 42 | 44.518 | 17.856 | 51.185 | 1.00 | 60.53 | A | C |
| ATOM | 113 | C | ALA | A | 42 | 43.526 | 15.800 | 50.156 | 1.00 | 43.86 | A | C |
| ATOM | 114 | O | ALA | A | 42 | 43.910 | 15.238 | 49.129 | 1.00 | 43.86 | A | O |
| ATOM | 115 | N | THR | A | 43 | 42.243 | 15.994 | 50.438 | 1.00 | 39.40 | A | N |
| ATOM | 116 | CA | THR | A | 43 | 41.173 | 15.559 | 49.547 | 1.00 | 39.40 | A | C |
| ATOM | 117 | CB | THR | A | 43 | 39.955 | 15.041 | 50.345 | 1.00 | 43.81 | A | C |
| ATOM | 118 | OG1 | THR | A | 43 | 40.408 | 14.168 | 51.381 | 1.00 | 43.81 | A | O |
| ATOM | 119 | CG2 | THR | A | 43 | 38.983 | 14.282 | 49.444 | 1.00 | 43.81 | A | C |
| ATOM | 120 | C | THR | A | 43 | 40.701 | 16.773 | 48.758 | 1.00 | 39.40 | A | C |
| ATOM | 121 | O | THR | A | 43 | 40.443 | 17.828 | 49.335 | 1.00 | 39.40 | A | O |
| ATOM | 122 | N | PRO | A | 44 | 40.551 | 16.630 | 47.435 | 1.00 | 42.91 | A | N |
| ATOM | 123 | CD | PRO | A | 44 | 40.761 | 15.420 | 46.632 | 1.00 | 32.42 | A | C |
| ATOM | 124 | CA | PRO | A | 44 | 40.101 | 17.746 | 46.588 | 1.00 | 42.91 | A | C |
| ATOM | 125 | CB | PRO | A | 44 | 40.184 | 17.172 | 45.167 | 1.00 | 32.42 | A | C |
| ATOM | 126 | CG | PRO | A | 44 | 41.107 | 16.012 | 45.288 | 1.00 | 32.42 | A | C |
| ATOM | 127 | C | PRO | A | 44 | 38.667 | 18.162 | 46.938 | 1.00 | 42.91 | A | C |
| ATOM | 128 | O | PRO | A | 44 | 37.771 | 17.317 | 47.000 | 1.00 | 42.91 | A | O |

| | | | | | | | | | | | | |
|---|---|---|---|---|---|---|---|---|---|---|---|---|
| ATOM | 129 | N | GLY | A | 45 | 38.455 | 19.458 | 47.142 | 1.00 | 34.24 | A | N |
| ATOM | 130 | CA | GLY | A | 45 | 37.133 | 19.961 | 47.495 | 1.00 | 34.24 | A | C |
| ATOM | 131 | C | GLY | A | 45 | 35.983 | 19.419 | 46.673 | 1.00 | 34.24 | A | C |
| ATOM | 132 | O | GLY | A | 45 | 34.961 | 19.002 | 47.222 | 1.00 | 34.24 | A | O |
| ATOM | 133 | N | ALA | A | 46 | 36.133 | 19.456 | 45.355 | 1.00 | 56.92 | A | N |
| ATOM | 134 | CA | ALA | A | 46 | 35.114 | 18.926 | 44.458 | 1.00 | 56.92 | A | C |
| ATOM | 135 | CB | ALA | A | 46 | 34.593 | 20.034 | 43.535 | 1.00 | 28.93 | A | C |
| ATOM | 136 | C | ALA | A | 46 | 35.801 | 17.802 | 43.660 | 1.00 | 56.92 | A | C |
| ATOM | 137 | O | ALA | A | 46 | 37.032 | 17.791 | 43.535 | 1.00 | 56.92 | A | O |
| ATOM | 138 | N | GLY | A | 47 | 35.026 | 16.858 | 43.131 | 1.00 | 57.70 | A | N |
| ATOM | 139 | CA | GLY | A | 47 | 35.635 | 15.768 | 42.389 | 1.00 | 57.70 | A | C |
| ATOM | 140 | C | GLY | A | 47 | 35.685 | 14.558 | 43.304 | 1.00 | 57.70 | A | C |
| ATOM | 141 | O | GLY | A | 47 | 35.382 | 14.677 | 44.504 | 1.00 | 57.70 | A | O |
| ATOM | 142 | N | PRO | A | 48 | 36.053 | 13.378 | 42.770 | 1.00 | 55.33 | A | N |
| ATOM | 143 | CD | PRO | A | 48 | 36.218 | 13.124 | 41.329 | 1.00 | 66.97 | A | C |
| ATOM | 144 | CA | PRO | A | 48 | 36.143 | 12.118 | 43.530 | 1.00 | 55.33 | A | C |
| ATOM | 145 | CB | PRO | A | 48 | 36.248 | 11.056 | 42.435 | 1.00 | 66.97 | A | C |
| ATOM | 146 | CG | PRO | A | 48 | 35.661 | 11.749 | 41.208 | 1.00 | 66.97 | A | C |
| ATOM | 147 | C | PRO | A | 48 | 37.334 | 12.066 | 44.493 | 1.00 | 55.33 | A | C |
| ATOM | 148 | O | PRO | A | 48 | 38.455 | 12.476 | 44.151 | 1.00 | 55.33 | A | O |
| ATOM | 149 | N | ASP | A | 49 | 37.072 | 11.545 | 45.691 | 1.00 | 63.53 | A | N |
| ATOM | 150 | CA | ASP | A | 49 | 38.082 | 11.450 | 46.743 | 1.00 | 63.53 | A | C |
| ATOM | 151 | CB | ASP | A | 49 | 37.561 | 10.652 | 47.948 | 1.00 | 58.40 | A | C |
| ATOM | 152 | CG | ASP | A | 49 | 38.534 | 10.687 | 49.153 | 1.00 | 58.40 | A | C |
| ATOM | 153 | OD1 | ASP | A | 49 | 39.761 | 10.932 | 48.950 | 1.00 | 58.40 | A | O |
| ATOM | 154 | OD2 | ASP | A | 49 | 38.054 | 10.457 | 50.298 | 1.00 | 58.40 | A | O |
| ATOM | 155 | C | ASP | A | 49 | 39.348 | 10.784 | 46.225 | 1.00 | 63.53 | A | C |
| ATOM | 156 | O | ASP | A | 49 | 39.462 | 9.555 | 46.232 | 1.00 | 63.53 | A | O |
| ATOM | 157 | N | ARG | A | 50 | 40.295 | 11.595 | 45.776 | 1.00 | 42.09 | A | N |
| ATOM | 158 | CA | ARG | A | 50 | 41.553 | 11.085 | 45.272 | 1.00 | 42.09 | A | C |
| ATOM | 159 | CB | ARG | A | 50 | 41.579 | 11.199 | 43.761 | 1.00 | 56.18 | A | C |
| ATOM | 160 | CG | ARG | A | 50 | 40.560 | 10.275 | 43.139 | 1.00 | 56.18 | A | C |
| ATOM | 161 | CD | ARG | A | 50 | 40.979 | 8.823 | 43.316 | 1.00 | 56.18 | A | C |
| ATOM | 162 | NE | ARG | A | 50 | 41.681 | 8.332 | 42.132 | 1.00 | 56.18 | A | N |
| ATOM | 163 | CZ | ARG | A | 50 | 42.345 | 7.182 | 42.061 | 1.00 | 56.18 | A | C |
| ATOM | 164 | NH1 | ARG | A | 50 | 42.418 | 6.374 | 43.124 | 1.00 | 56.18 | A | N |
| ATOM | 165 | NH2 | ARG | A | 50 | 42.922 | 6.838 | 40.911 | 1.00 | 56.18 | A | N |
| ATOM | 166 | C | ARG | A | 50 | 42.615 | 11.930 | 45.915 | 1.00 | 42.09 | A | C |
| ATOM | 167 | O | ARG | A | 50 | 43.021 | 12.955 | 45.372 | 1.00 | 42.09 | A | O |
| ATOM | 168 | N | PRO | A | 51 | 43.108 | 11.481 | 47.078 | 1.00 | 27.89 | A | N |
| ATOM | 169 | CD | PRO | A | 51 | 43.255 | 10.029 | 47.271 | 1.00 | 37.14 | A | C |
| ATOM | 170 | CA | PRO | A | 51 | 44.122 | 12.179 | 47.856 | 1.00 | 27.89 | A | C |
| ATOM | 171 | CB | PRO | A | 51 | 44.704 | 11.080 | 48.748 | 1.00 | 37.14 | A | C |
| ATOM | 172 | CG | PRO | A | 51 | 43.760 | 9.950 | 48.657 | 1.00 | 37.14 | A | C |
| ATOM | 173 | C | PRO | A | 51 | 45.190 | 12.700 | 46.920 | 1.00 | 27.89 | A | C |
| ATOM | 174 | O | PRO | A | 51 | 45.356 | 12.201 | 45.812 | 1.00 | 27.89 | A | O |
| ATOM | 175 | N | GLN | A | 52 | 45.922 | 13.700 | 47.379 | 1.00 | 53.50 | A | N |
| ATOM | 176 | CA | GLN | A | 52 | 47.000 | 14.260 | 46.595 | 1.00 | 53.50 | A | C |
| ATOM | 177 | CB | GLN | A | 52 | 46.437 | 15.106 | 45.456 | 1.00 | 61.95 | A | C |
| ATOM | 178 | CG | GLN | A | 52 | 45.042 | 15.643 | 45.712 | 1.00 | 61.95 | A | C |
| ATOM | 179 | CD | GLN | A | 52 | 44.230 | 15.796 | 44.413 | 1.00 | 61.95 | A | C |
| ATOM | 180 | OE1 | GLN | A | 52 | 44.094 | 14.835 | 43.633 | 1.00 | 61.95 | A | O |
| ATOM | 181 | NE2 | GLN | A | 52 | 43.681 | 17.003 | 44.180 | 1.00 | 61.95 | A | N |
| ATOM | 182 | C | GLN | A | 52 | 47.864 | 15.105 | 47.508 | 1.00 | 53.50 | A | C |
| ATOM | 183 | O | GLN | A | 52 | 47.363 | 15.766 | 48.426 | 1.00 | 53.50 | A | O |
| ATOM | 184 | N | GLU | A | 53 | 49.170 | 15.063 | 47.264 | 1.00 | 33.58 | A | N |
| ATOM | 185 | CA | GLU | A | 53 | 50.102 | 15.809 | 48.086 | 1.00 | 33.58 | A | C |
| ATOM | 186 | CB | GLU | A | 53 | 51.539 | 15.432 | 47.764 | 1.00 | 40.36 | A | C |
| ATOM | 187 | CG | GLU | A | 53 | 52.026 | 14.202 | 48.454 | 1.00 | 40.36 | A | C |
| ATOM | 188 | CD | GLU | A | 53 | 53.539 | 14.139 | 48.480 | 1.00 | 40.36 | A | C |
| ATOM | 189 | OE1 | GLU | A | 53 | 54.074 | 13.164 | 49.056 | 1.00 | 40.36 | A | O |
| ATOM | 190 | OE2 | GLU | A | 53 | 54.186 | 15.066 | 47.928 | 1.00 | 40.36 | A | O |
| ATOM | 191 | C | GLU | A | 53 | 49.955 | 17.297 | 47.848 | 1.00 | 33.58 | A | C |
| ATOM | 192 | O | GLU | A | 53 | 49.840 | 17.745 | 46.717 | 1.00 | 33.58 | A | O |
| ATOM | 193 | N | VAL | A | 54 | 49.951 | 18.058 | 48.933 | 1.00 | 44.16 | A | N |
| ATOM | 194 | CA | VAL | A | 54 | 49.872 | 19.505 | 48.858 | 1.00 | 44.16 | A | C |
| ATOM | 195 | CB | VAL | A | 54 | 48.501 | 20.070 | 49.304 | 1.00 | 23.21 | A | C |

| | | | | | | | | | | | | |
|---|---|---|---|---|---|---|---|---|---|---|---|---|
| ATOM | 196 | CG1 | VAL | A | 54 | 48.601 | 21.573 | 49.443 | 1.00 | 23.21 | A | C |
| ATOM | 197 | CG2 | VAL | A | 54 | 47.426 | 19.736 | 48.290 | 1.00 | 23.21 | A | C |
| ATOM | 198 | C | VAL | A | 54 | 50.919 | 20.041 | 49.818 | 1.00 | 44.16 | A | C |
| ATOM | 199 | O | VAL | A | 54 | 51.055 | 19.546 | 50.941 | 1.00 | 44.16 | A | O |
| ATOM | 200 | N | SER | A | 55 | 51.664 | 21.045 | 49.378 | 1.00 | 39.07 | A | N |
| ATOM | 201 | CA | SER | A | 55 | 52.692 | 21.628 | 50.229 | 1.00 | 39.07 | A | C |
| ATOM | 202 | CB | SER | A | 55 | 54.083 | 21.135 | 49.818 | 1.00 | 33.53 | A | C |
| ATOM | 203 | OG | SER | A | 55 | 54.432 | 21.631 | 48.537 | 1.00 | 33.53 | A | O |
| ATOM | 204 | C | SER | A | 55 | 52.655 | 23.152 | 50.155 | 1.00 | 39.07 | A | C |
| ATOM | 205 | O | SER | A | 55 | 52.713 | 23.746 | 49.074 | 1.00 | 39.07 | A | O |
| ATOM | 206 | N | TYR | A | 56 | 52.566 | 23.790 | 51.315 | 1.00 | 45.38 | A | N |
| ATOM | 207 | CA | TYR | A | 56 | 52.532 | 25.243 | 51.357 | 1.00 | 45.38 | A | C |
| ATOM | 208 | CB | TYR | A | 56 | 51.168 | 25.729 | 51.808 | 1.00 | 33.25 | A | C |
| ATOM | 209 | CG | TYR | A | 56 | 50.730 | 25.088 | 53.096 | 1.00 | 33.25 | A | C |
| ATOM | 210 | CD1 | TYR | A | 56 | 51.309 | 25.441 | 54.301 | 1.00 | 33.25 | A | C |
| ATOM | 211 | CE1 | TYR | A | 56 | 50.900 | 24.857 | 55.476 | 1.00 | 33.25 | A | C |
| ATOM | 212 | CD2 | TYR | A | 56 | 49.730 | 24.124 | 53.105 | 1.00 | 33.25 | A | C |
| ATOM | 213 | CE2 | TYR | A | 56 | 49.317 | 23.533 | 54.280 | 1.00 | 33.25 | A | C |
| ATOM | 214 | CZ | TYR | A | 56 | 49.903 | 23.905 | 55.462 | 1.00 | 33.25 | A | C |
| ATOM | 215 | OH | TYR | A | 56 | 49.476 | 23.332 | 56.636 | 1.00 | 33.25 | A | O |
| ATOM | 216 | C | TYR | A | 56 | 53.591 | 25.780 | 52.298 | 1.00 | 45.38 | A | C |
| ATOM | 217 | O | TYR | A | 56 | 54.306 | 25.013 | 52.948 | 1.00 | 45.38 | A | O |
| ATOM | 218 | N | THR | A | 57 | 53.690 | 27.102 | 52.368 | 1.00 | 37.57 | A | N |
| ATOM | 219 | CA | THR | A | 57 | 54.654 | 27.748 | 53.247 | 1.00 | 37.57 | A | C |
| ATOM | 220 | CB | THR | A | 57 | 56.006 | 28.053 | 52.562 | 1.00 | 51.42 | A | C |
| ATOM | 221 | OG1 | THR | A | 57 | 55.812 | 29.052 | 51.550 | 1.00 | 51.42 | A | O |
| ATOM | 222 | CG2 | THR | A | 57 | 56.597 | 26.806 | 51.938 | 1.00 | 51.42 | A | C |
| ATOM | 223 | C | THR | A | 57 | 54.123 | 29.099 | 53.656 | 1.00 | 37.57 | A | C |
| ATOM | 224 | O | THR | A | 57 | 53.010 | 29.478 | 53.299 | 1.00 | 37.57 | A | O |
| ATOM | 225 | N | ASP | A | 58 | 54.971 | 29.838 | 54.363 | 1.00 | 50.96 | A | N |
| ATOM | 226 | CA | ASP | A | 58 | 54.657 | 31.173 | 54.850 | 1.00 | 50.96 | A | C |
| ATOM | 227 | CB | ASP | A | 58 | 54.569 | 32.182 | 53.698 | 1.00 | 40.68 | A | C |
| ATOM | 228 | CG | ASP | A | 58 | 55.847 | 32.265 | 52.888 | 1.00 | 40.68 | A | C |
| ATOM | 229 | OD1 | ASP | A | 58 | 56.938 | 32.193 | 53.497 | 1.00 | 40.68 | A | O |
| ATOM | 230 | OD2 | ASP | A | 58 | 55.754 | 32.418 | 51.647 | 1.00 | 40.68 | A | O |
| ATOM | 231 | C | ASP | A | 58 | 53.354 | 31.220 | 55.632 | 1.00 | 50.96 | A | C |
| ATOM | 232 | O | ASP | A | 58 | 52.672 | 32.246 | 55.628 | 1.00 | 50.96 | A | O |
| ATOM | 233 | N | THR | A | 59 | 53.003 | 30.135 | 56.311 | 1.00 | 35.45 | A | N |
| ATOM | 234 | CA | THR | A | 59 | 51.750 | 30.145 | 57.050 | 1.00 | 35.45 | A | C |
| ATOM | 235 | CB | THR | A | 59 | 51.463 | 28.818 | 57.758 | 1.00 | 43.10 | A | C |
| ATOM | 236 | OG1 | THR | A | 59 | 52.375 | 28.654 | 58.847 | 1.00 | 43.10 | A | O |
| ATOM | 237 | CG2 | THR | A | 59 | 51.606 | 27.667 | 56.808 | 1.00 | 43.10 | A | C |
| ATOM | 238 | C | THR | A | 59 | 51.777 | 31.210 | 58.123 | 1.00 | 35.45 | A | C |
| ATOM | 239 | O | THR | A | 59 | 52.750 | 31.310 | 58.865 | 1.00 | 35.45 | A | O |
| ATOM | 240 | N | LYS | A | 60 | 50.704 | 32.000 | 58.199 | 1.00 | 53.69 | A | N |
| ATOM | 241 | CA | LYS | A | 60 | 50.592 | 33.063 | 59.191 | 1.00 | 53.69 | A | C |
| ATOM | 242 | CB | LYS | A | 60 | 51.114 | 34.386 | 58.612 | 1.00 | 44.14 | A | C |
| ATOM | 243 | CG | LYS | A | 60 | 50.133 | 35.127 | 57.732 | 1.00 | 44.14 | A | C |
| ATOM | 244 | CD | LYS | A | 60 | 50.672 | 36.487 | 57.248 | 1.00 | 44.14 | A | C |
| ATOM | 245 | CE | LYS | A | 60 | 51.625 | 36.336 | 56.042 | 1.00 | 44.14 | A | C |
| ATOM | 246 | NZ | LYS | A | 60 | 52.860 | 35.506 | 56.349 | 1.00 | 44.14 | A | N |
| ATOM | 247 | C | LYS | A | 60 | 49.134 | 33.212 | 59.622 | 1.00 | 53.69 | A | C |
| ATOM | 248 | O | LYS | A | 60 | 48.241 | 33.278 | 58.789 | 1.00 | 53.69 | A | O |
| ATOM | 249 | N | VAL | A | 61 | 48.902 | 33.254 | 60.929 | 1.00 | 56.03 | A | N |
| ATOM | 250 | CA | VAL | A | 61 | 47.556 | 33.381 | 61.486 | 1.00 | 56.03 | A | C |
| ATOM | 251 | CB | VAL | A | 61 | 47.612 | 33.266 | 63.022 | 1.00 | 44.24 | A | C |
| ATOM | 252 | CG1 | VAL | A | 61 | 46.216 | 33.021 | 63.578 | 1.00 | 44.24 | A | C |
| ATOM | 253 | CG2 | VAL | A | 61 | 48.571 | 32.145 | 63.432 | 1.00 | 44.24 | A | C |
| ATOM | 254 | C | VAL | A | 61 | 46.906 | 34.711 | 61.109 | 1.00 | 56.03 | A | C |
| ATOM | 255 | O | VAL | A | 61 | 47.431 | 35.768 | 61.458 | 1.00 | 56.03 | A | O |
| ATOM | 256 | N | ILE | A | 62 | 45.759 | 34.657 | 60.425 | 1.00 | 43.60 | A | N |
| ATOM | 257 | CA | ILE | A | 62 | 45.070 | 35.871 | 59.978 | 1.00 | 43.60 | A | C |
| ATOM | 258 | CB | ILE | A | 62 | 44.935 | 35.895 | 58.445 | 1.00 | 28.57 | A | C |
| ATOM | 259 | CG2 | ILE | A | 62 | 46.312 | 35.967 | 57.801 | 1.00 | 28.57 | A | C |
| ATOM | 260 | CG1 | ILE | A | 62 | 44.151 | 34.673 | 57.980 | 1.00 | 28.57 | A | C |
| ATOM | 261 | CD1 | ILE | A | 62 | 43.778 | 34.728 | 56.521 | 1.00 | 28.57 | A | C |
| ATOM | 262 | C | ILE | A | 62 | 43.683 | 36.034 | 60.608 | 1.00 | 43.60 | A | C |

| | | | | | | | | | | | | |
|---|---|---|---|---|---|---|---|---|---|---|---|---|
| ATOM | 263 | O | ILE | A | 62 | 42.934 | 36.958 | 60.291 | 1.00 | 43.60 | A | O |
| ATOM | 264 | N | GLY | A | 63 | 43.355 | 35.128 | 61.512 | 1.00 | 39.40 | A | N |
| ATOM | 265 | CA | GLY | A | 63 | 42.075 | 35.174 | 62.182 | 1.00 | 39.40 | A | C |
| ATOM | 266 | C | GLY | A | 63 | 42.101 | 34.047 | 63.204 | 1.00 | 39.40 | A | C |
| ATOM | 267 | O | GLY | A | 63 | 42.778 | 33.043 | 62.985 | 1.00 | 39.40 | A | O |
| ATOM | 268 | N | ASN | A | 64 | 41.380 | 34.214 | 64.311 | 1.00 | 51.69 | A | N |
| ATOM | 269 | CA | ASN | A | 64 | 41.316 | 33.231 | 65.407 | 1.00 | 51.69 | A | C |
| ATOM | 270 | CB | ASN | A | 64 | 41.884 | 33.765 | 66.712 | 1.00 | 55.00 | A | C |
| ATOM | 271 | CG | ASN | A | 64 | 43.295 | 34.128 | 66.628 | 1.00 | 55.00 | A | C |
| ATOM | 272 | OD1 | ASN | A | 64 | 44.160 | 33.352 | 67.039 | 1.00 | 55.00 | A | O |
| ATOM | 273 | ND2 | ASN | A | 64 | 43.573 | 35.323 | 66.094 | 1.00 | 55.00 | A | N |
| ATOM | 274 | C | ASN | A | 64 | 39.879 | 32.990 | 65.809 | 1.00 | 51.69 | A | C |
| ATOM | 275 | O | ASN | A | 64 | 39.615 | 32.437 | 66.886 | 1.00 | 51.69 | A | O |
| ATOM | 276 | N | GLY | A | 65 | 38.966 | 33.452 | 64.977 | 1.00 | 52.62 | A | N |
| ATOM | 277 | CA | GLY | A | 65 | 37.561 | 33.391 | 65.305 | 1.00 | 52.62 | A | C |
| ATOM | 278 | C | GLY | A | 65 | 36.764 | 32.200 | 65.800 | 1.00 | 52.62 | A | C |
| ATOM | 279 | O | GLY | A | 65 | 36.748 | 31.086 | 65.237 | 1.00 | 52.62 | A | O |
| ATOM | 280 | N | SER | A | 66 | 36.072 | 32.497 | 66.894 | 1.00 | 99.97 | A | N |
| ATOM | 281 | CA | SER | A | 66 | 35.159 | 31.581 | 67.540 | 1.00 | 99.97 | A | C |
| ATOM | 282 | CB | SER | A | 66 | 33.871 | 31.536 | 66.692 | 1.00 | 88.22 | A | C |
| ATOM | 283 | OG | SER | A | 66 | 33.386 | 32.855 | 66.430 | 1.00 | 88.22 | A | O |
| ATOM | 284 | C | SER | A | 66 | 35.782 | 30.201 | 67.736 | 1.00 | 99.97 | A | C |
| ATOM | 285 | O | SER | A | 66 | 36.614 | 29.969 | 68.634 | 1.00 | 99.97 | A | O |
| ATOM | 286 | N | PHE | A | 67 | 35.360 | 29.313 | 66.863 | 1.00 | 67.65 | A | N |
| ATOM | 287 | CA | PHE | A | 67 | 35.764 | 27.923 | 66.777 | 1.00 | 67.65 | A | C |
| ATOM | 288 | CB | PHE | A | 67 | 35.201 | 27.440 | 65.441 | 1.00100.00 | | A | C |
| ATOM | 289 | CG | PHE | A | 67 | 34.046 | 28.313 | 64.944 | 1.00100.00 | | A | C |
| ATOM | 290 | CD1 | PHE | A | 67 | 34.092 | 28.921 | 63.682 | 1.00100.00 | | A | C |
| ATOM | 291 | CD2 | PHE | A | 67 | 32.934 | 28.570 | 65.776 | 1.00100.00 | | A | C |
| ATOM | 292 | CE1 | PHE | A | 67 | 33.057 | 29.772 | 63.251 | 1.00100.00 | | A | C |
| ATOM | 293 | CE2 | PHE | A | 67 | 31.888 | 29.421 | 65.360 | 1.00100.00 | | A | C |
| ATOM | 294 | CZ | PHE | A | 67 | 31.953 | 30.024 | 64.092 | 1.00100.00 | | A | C |
| ATOM | 295 | C | PHE | A | 67 | 37.283 | 27.665 | 66.910 | 1.00 | 67.65 | A | C |
| ATOM | 296 | O | PHE | A | 67 | 37.748 | 27.165 | 67.943 | 1.00 | 67.65 | A | O |
| ATOM | 297 | N | GLY | A | 68 | 38.049 | 28.016 | 65.880 | 1.00 | 52.60 | A | N |
| ATOM | 298 | CA | GLY | A | 68 | 39.489 | 27.797 | 65.922 | 1.00 | 52.60 | A | C |
| ATOM | 299 | C | GLY | A | 68 | 40.305 | 28.838 | 65.170 | 1.00 | 52.60 | A | C |
| ATOM | 300 | O | GLY | A | 68 | 40.021 | 30.035 | 65.266 | 1.00 | 52.60 | A | O |
| ATOM | 301 | N | VAL | A | 69 | 41.295 | 28.392 | 64.397 | 1.00 | 37.21 | A | N |
| ATOM | 302 | CA | VAL | A | 69 | 42.164 | 29.319 | 63.667 | 1.00 | 37.21 | A | C |
| ATOM | 303 | CB | VAL | A | 69 | 43.657 | 29.018 | 63.928 | 1.00 | 46.84 | A | C |
| ATOM | 304 | CG1 | VAL | A | 69 | 44.514 | 30.207 | 63.473 | 1.00 | 46.84 | A | C |
| ATOM | 305 | CG2 | VAL | A | 69 | 43.885 | 28.707 | 65.405 | 1.00 | 46.84 | A | C |
| ATOM | 306 | C | VAL | A | 69 | 41.991 | 29.353 | 62.154 | 1.00 | 37.21 | A | C |
| ATOM | 307 | O | VAL | A | 69 | 41.537 | 28.377 | 61.543 | 1.00 | 37.21 | A | O |
| ATOM | 308 | N | VAL | A | 70 | 42.381 | 30.484 | 61.564 | 1.00 | 42.09 | A | N |
| ATOM | 309 | CA | VAL | A | 70 | 42.320 | 30.692 | 60.118 | 1.00 | 42.09 | A | C |
| ATOM | 310 | CB | VAL | A | 70 | 41.225 | 31.693 | 59.727 | 1.00 | 31.10 | A | C |
| ATOM | 311 | CG1 | VAL | A | 70 | 41.479 | 32.225 | 58.306 | 1.00 | 31.10 | A | C |
| ATOM | 312 | CG2 | VAL | A | 70 | 39.870 | 31.013 | 59.815 | 1.00 | 31.10 | A | C |
| ATOM | 313 | C | VAL | A | 70 | 43.658 | 31.233 | 59.649 | 1.00 | 42.09 | A | C |
| ATOM | 314 | O | VAL | A | 70 | 44.034 | 32.366 | 59.981 | 1.00 | 42.09 | A | O |
| ATOM | 315 | N | TYR | A | 71 | 44.374 | 30.427 | 58.870 | 1.00 | 40.02 | A | N |
| ATOM | 316 | CA | TYR | A | 71 | 45.688 | 30.836 | 58.400 | 1.00 | 40.02 | A | C |
| ATOM | 317 | CB | TYR | A | 71 | 46.730 | 29.732 | 58.632 | 1.00 | 52.79 | A | C |
| ATOM | 318 | CG | TYR | A | 71 | 46.656 | 29.032 | 59.967 | 1.00 | 52.79 | A | C |
| ATOM | 319 | CD1 | TYR | A | 71 | 45.708 | 28.039 | 60.211 | 1.00 | 52.79 | A | C |
| ATOM | 320 | CE1 | TYR | A | 71 | 45.656 | 27.380 | 61.444 | 1.00 | 52.79 | A | C |
| ATOM | 321 | CD2 | TYR | A | 71 | 47.546 | 29.351 | 60.986 | 1.00 | 52.79 | A | C |
| ATOM | 322 | CE2 | TYR | A | 71. | 47.502 | 28.698 | 62.223 | 1.00 | 52.79 | A | C |
| ATOM | 323 | CZ | TYR | A | 71 | 46.560 | 27.719 | 62.441 | 1.00 | 52.79 | A | C |
| ATOM | 324 | OH | TYR | A | 71 | 46.533 | 27.085 | 63.660 | 1.00 | 52.79 | A | O |
| ATOM | 325 | C | TYR | A | 71 | 45.715 | 31.195 | 56.927 | 1.00 | 40.02 | A | C |
| ATOM | 326 | O | TYR | A | 71 | 44.825 | 30.839 | 56.158 | 1.00 | 40.02 | A | O |
| ATOM | 327 | N | GLN | A | 72 | 46.767 | 31.900 | 56.549 | 1.00 | 43.31 | A | N |
| ATOM | 328 | CA | GLN | A | 72 | 46.969 | 32.301 | 55.176 | 1.00 | 43.31 | A | C |
| ATOM | 329 | CB | GLN | A | 72 | 47.156 | 33.810 | 55.093 | 1.00 | 52.63 | A | C |

| | | | | | | | | | | | | |
|---|---|---|---|---|---|---|---|---|---|---|---|---|
| ATOM | 330 | CG | GLN | A | 72 | 47.084 | 34.319 | 53.683 | 1.00 | 52.63 | A | C |
| ATOM | 331 | CD | GLN | A | 72 | 48.371 | 34.943 | 53.228 | 1.00 | 52.63 | A | C |
| ATOM | 332 | OE1 | GLN | A | 72 | 49.447 | 34.402 | 53.488 | 1.00 | 52.63 | A | O |
| ATOM | 333 | NE2 | GLN | A | 72 | 48.280 | 36.083 | 52.528 | 1.00 | 52.63 | A | N |
| ATOM | 334 | C | GLN | A | 72 | 48.252 | 31.584 | 54.788 | 1.00 | 43.31 | A | C |
| ATOM | 335 | O | GLN | A | 72 | 49.195 | 31.523 | 55.587 | 1.00 | 43.31 | A | O |
| ATOM | 336 | N | ALA | A | 73 | 48.291 | 31.032 | 53.580 | 1.00 | 43.37 | A | N |
| ATOM | 337 | CA | ALA | A | 73 | 49.471 | 30.303 | 53.136 | 1.00 | 43.37 | A | C |
| ATOM | 338 | CB | ALA | A | 73 | 49.310 | 28.827 | 53.462 | 1.00 | 18.00 | A | C |
| ATOM | 339 | C | ALA | A | 73 | 49.749 | 30.479 | 51.651 | 1.00 | 43.37 | A | C |
| ATOM | 340 | O | ALA | A | 73 | 48.914 | 30.993 | 50.898 | 1.00 | 43.37 | A | O |
| ATOM | 341 | N | LYS | A | 74 | 50.940 | 30.056 | 51.241 | 1.00 | 46.43 | A | N |
| ATOM | 342 | CA | LYS | A | 74 | 51.344 | 30.152 | 49.852 | 1.00 | 46.43 | A | C |
| ATOM | 343 | CB | LYS | A | 74 | 52.632 | 30.976 | 49.735 | 1.00 | 58.50 | A | C |
| ATOM | 344 | CG | LYS | A | 74 | 53.130 | 31.181 | 48.307 | 1.00 | 58.50 | A | C |
| ATOM | 345 | CD | LYS | A | 74 | 54.170 | 32.306 | 48.247 | 1.00 | 58.50 | A | C |
| ATOM | 346 | CE | LYS | A | 74 | 54.796 | 32.443 | 46.851 | 1.00 | 58.50 | A | C |
| ATOM | 347 | NZ | LYS | A | 74 | 55.734 | 31.281 | 46.489 | 1.00 | 58.50 | A | N |
| ATOM | 348 | C | LYS | A | 74 | 51.567 | 28.742 | 49.323 | 1.00 | 46.43 | A | C |
| ATOM | 349 | O | LYS | A | 74 | 52.326 | 27.964 | 49.909 | 1.00 | 46.43 | A | O |
| ATOM | 350 | N | LEU | A | 75 | 50.872 | 28.395 | 48.244 | 1.00 | 51.71 | A | N |
| ATOM | 351 | CA | LEU | A | 75 | 51.057 | 27.080 | 47.657 | 1.00 | 51.71 | A | C |
| ATOM | 352 | CB | LEU | A | 75 | 49.960 | 26.771 | 46.628 | 1.00 | 27.95 | A | C |
| ATOM | 353 | CG | LEU | A | 75 | 48.566 | 26.409 | 47.169 | 1.00 | 27.95 | A | C |
| ATOM | 354 | CD1 | LEU | A | 75 | 47.851 | 25.504 | 46.186 | 1.00 | 27.95 | A | C |
| ATOM | 355 | CD2 | LEU | A | 75 | 48.689 | 25.703 | 48.488 | 1.00 | 27.95 | A | C |
| ATOM | 356 | C | LEU | A | 75 | 52.448 | 27.066 | 47.011 | 1.00 | 51.71 | A | C |
| ATOM | 357 | O | LEU | A | 75 | 52.795 | 27.932 | 46.183 | 1.00 | 51.71 | A | O |
| ATOM | 358 | N | CYS | A | 76 | 53.239 | 26.070 | 47.410 | 1.00 | 65.32 | A | N |
| ATOM | 359 | CA | CYS | A | 76 | 54.613 | 25.917 | 46.939 | 1.00 | 65.32 | A | C |
| ATOM | 360 | CB | CYS | A | 76 | 55.212 | 24.617 | 47.465 | 1.00 | 35.49 | A | C |
| ATOM | 361 | SG | CYS | A | 76 | 55.669 | 24.745 | 49.166 | 1.00 | 35.49 | A | S |
| ATOM | 362 | C | CYS | A | 76 | 54.883 | 26.002 | 45.454 | 1.00 | 65.32 | A | C |
| ATOM | 363 | O | CYS | A | 76 | 55.771 | 26.747 | 45.027 | 1.00 | 65.32 | A | O |
| ATOM | 364 | N | ASP | A | 77 | 54.138 | 25.258 | 44.651 | 1.00 | 60.93 | A | N |
| ATOM | 365 | CA | ASP | A | 77 | 54.422 | 25.307 | 43.238 | 1.00 | 60.93 | A | C |
| ATOM | 366 | CB | ASP | A | 77 | 54.263 | 23.909 | 42.659 | 1.00 | 74.12 | A | C |
| ATOM | 367 | CG | ASP | A | 77 | 55.148 | 22.881 | 43.383 | 1.00 | 74.12 | A | C |
| ATOM | 368 | OD1 | ASP | A | 77 | 56.353 | 23.188 | 43.624 | 1.00 | 74.12 | A | O |
| ATOM | 369 | OD2 | ASP | A | 77 | 54.629 | 21.777 | 43.700 | 1.00 | 74.12 | A | O |
| ATOM | 370 | C | ASP | A | 77 | 53.639 | 26.343 | 42.452 | 1.00 | 60.93 | A | C |
| ATOM | 371 | O | ASP | A | 77 | 54.177 | 26.959 | 41.521 | 1.00 | 60.93 | A | O |
| ATOM | 372 | N | SER | A | 78 | 52.383 | 26.560 | 42.832 | 1.00 | 48.47 | A | N |
| ATOM | 373 | CA | SER | A | 78 | 51.559 | 27.547 | 42.138 | 1.00 | 48.47 | A | C |
| ATOM | 374 | CB | SER | A | 78 | 50.079 | 27.204 | 42.297 | 1.00 | 58.39 | A | C |
| ATOM | 375 | OG | SER | A | 78 | 49.722 | 27.176 | 43.669 | 1.00 | 58.39 | A | O |
| ATOM | 376 | C | SER | A | 78 | 51.805 | 28.977 | 42.635 | 1.00 | 48.47 | A | C |
| ATOM | 377 | O | SER | A | 78 | 51.609 | 29.936 | 41.887 | 1.00 | 48.47 | A | O |
| ATOM | 378 | N | GLY | A | 79 | 52.241 | 29.111 | 43.891 | 1.00 | 30.20 | A | N |
| ATOM | 379 | CA | GLY | A | 79 | 52.486 | 30.424 | 44.463 | 1.00 | 30.20 | A | C |
| ATOM | 380 | C | GLY | A | 79 | 51.181 | 31.017 | 44.968 | 1.00 | 30.20 | A | C |
| ATOM | 381 | O | GLY | A | 79 | 51.166 | 31.986 | 45.733 | 1.00 | 30.20 | A | O |
| ATOM | 382 | N | GLU | A | 80 | 50.076 | 30.425 | 44.530 | 1.00 | 45.07 | A | N |
| ATOM | 383 | CA | GLU | A | 80 | 48.746 | 30.867 | 44.926 | 1.00 | 45.07 | A | C |
| ATOM | 384 | CB | GLU | A | 80 | 47.708 | 29.912 | 44.340 | 1.00 | 56.84 | A | C |
| ATOM | 385 | CG | GLU | A | 80 | 47.675 | 29.942 | 42.820 | 1.00 | 56.84 | A | C |
| ATOM | 386 | CD | GLU | A | 80 | 46.967 | 28.739 | 42.223 | 1.00 | 56.84 | A | C |
| ATOM | 387 | OE1 | GLU | A | 80 | 45.824 | 28.432 | 42.656 | 1.00 | 56.84 | A | O |
| ATOM | 388 | OE2 | GLU | A | 80 | 47.566 | 28.109 | 41.317 | 1.00 | 56.84 | A | O |
| ATOM | 389 | C | GLU | A | 80 | 48.597 | 30.944 | 46.446 | 1.00 | 45.07 | A | C |
| ATOM | 390 | O | GLU | A | 80 | 49.114 | 30.101 | 47.176 | 1.00 | 45.07 | A | O |
| ATOM | 391 | N | LEU | A | 81 | 47.901 | 31.971 | 46.918 | 1.00 | 43.41 | A | N |
| ATOM | 392 | CA | LEU | A | 81 | 47.684 | 32.165 | 48.346 | 1.00 | 43.41 | A | C |
| ATOM | 393 | CB | LEU | A | 81 | 47.533 | 33.653 | 48.643 | 1.00 | 34.35 | A | C |
| ATOM | 394 | CG | LEU | A | 81 | 48.868 | 34.390 | 48.684 | 1.00 | 34.35 | A | C |
| ATOM | 395 | CD1 | LEU | A | 81 | 48.678 | 35.894 | 48.608 | 1.00 | 34.35 | A | C |
| ATOM | 396 | CD2 | LEU | A | 81 | 49.581 | 33.988 | 49.975 | 1.00 | 34.35 | A | C |

| | | | | | | | | | | | | |
|---|---|---|---|---|---|---|---|---|---|---|---|---|
| ATOM | 397 | C | LEU | A | 81 | 46.425 | 31.438 | 48.751 | 1.00 | 43.41 | A | C |
| ATOM | 398 | O | LEU | A | 81 | 45.490 | 31.361 | 47.966 | 1.00 | 43.41 | A | O |
| ATOM | 399 | N | VAL | A | 82 | 46.397 | 30.901 | 49.965 | 1.00 | 36.49 | A | N |
| ATOM | 400 | CA | VAL | A | 82 | 45.219 | 30.191 | 50.446 | 1.00 | 36.49 | A | C |
| ATOM | 401 | CB | VAL | A | 82 | 45.376 | 28.676 | 50.249 | 1.00 | 27.68 | A | C |
| ATOM | 402 | CG1 | VAL | A | 82 | 45.705 | 28.373 | 48.808 | 1.00 | 27.68 | A | C |
| ATOM | 403 | CG2 | VAL | A | 82 | 46.472 | 28.152 | 51.163 | 1.00 | 27.68 | A | C |
| ATOM | 404 | C | VAL | A | 82 | 44.896 | 30.448 | 51.924 | 1.00 | 36.49 | A | C |
| ATOM | 405 | O | VAL | A | 82 | 45.749 | 30.853 | 52.717 | 1.00 | 36.49 | A | O |
| ATOM | 406 | N | ALA | A | 83 | 43.650 | 30.200 | 52.293 | 1.00 | 32.59 | A | N |
| ATOM | 407 | CA | ALA | A | 83 | 43.222 | 30.380 | 53.667 | 1.00 | 32.59 | A | C |
| ATOM | 408 | CB | ALA | A | 83 | 42.005 | 31.270 | 53.712 | 1.00 | 35.22 | A | C |
| ATOM | 409 | C | ALA | A | 83 | 42.886 | 29.006 | 54.224 | 1.00 | 32.59 | A | C |
| ATOM | 410 | O | ALA | A | 83 | 42.195 | 28.217 | 53.578 | 1.00 | 32.59 | A | O |
| ATOM | 411 | N | ILE | A | 84 | 43.369 | 28.710 | 55.420 | 1.00 | 27.53 | A | N |
| ATOM | 412 | CA | ILE | A | 84 | 43.080 | 27.421 | 56.009 | 1.00 | 27.53 | A | C |
| ATOM | 413 | CB | ILE | A | 84 | 44.358 | 26.603 | 56.224 | 1.00 | 39.25 | A | C |
| ATOM | 414 | CG2 | ILE | A | 84 | 43.992 | 25.198 | 56.662 | 1.00 | 39.25 | A | C |
| ATOM | 415 | CG1 | ILE | A | 84 | 45.169 | 26.548 | 54.926 | 1.00 | 39.25 | A | C |
| ATOM | 416 | CD1 | ILE | A | 84 | 46.476 | 25.780 | 55.039 | 1.00 | 39.25 | A | C |
| ATOM | 417 | C | ILE | A | 84 | 42.350 | 27.545 | 57.335 | 1.00 | 27.53 | A | C |
| ATOM | 418 | O | ILE | A | 84 | 42.960 | 27.688 | 58.400 | 1.00 | 27.53 | A | O |
| ATOM | 419 | N | LYS | A | 85 | 41.029 | 27.505 | 57.267 | 1.00 | 40.37 | A | N |
| ATOM | 420 | CA | LYS | A | 85 | 40.244 | 27.580 | 58.477 | 1.00 | 40.37 | A | C |
| ATOM | 421 | CB | LYS | A | 85 | 38.773 | 27.866 | 58.145 | 1.00 | 37.30 | A | C |
| ATOM | 422 | CG | LYS | A | 85 | 37.794 | 27.528 | 59.270 | 1.00 | 37.30 | A | C |
| ATOM | 423 | CD | LYS | A | 85 | 36.453 | 28.215 | 59.094 | 1.00 | 37.30 | A | C |
| ATOM | 424 | CE | LYS | A | 85 | 35.546 | 27.907 | 60.287 | 1.00 | 37.30 | A | C |
| ATOM | 425 | NZ | LYS | A | 85 | 34.155 | 28.417 | 60.129 | 1.00 | 37.30 | A | N |
| ATOM | 426 | C | LYS | A | 85 | 40.384 | 26.214 | 59.132 | 1.00 | 40.37 | A | C |
| ATOM | 427 | O | LYS | A | 85 | 39.972 | 25.203 | 58.559 | 1.00 | 40.37 | A | O |
| ATOM | 428 | N | LYS | A | 86 | 41.002 | 26.182 | 60.309 | 1.00 | 50.17 | A | N |
| ATOM | 429 | CA | LYS | A | 86 | 41.157 | 24.934 | 61.046 | 1.00 | 50.17 | A | C |
| ATOM | 430 | CB | LYS | A | 86 | 42.583 | 24.790 | 61.612 | 1.00 | 53.30 | A | C |
| ATOM | 431 | CG | LYS | A | 86 | 42.688 | 23.767 | 62.752 | 1.00 | 53.30 | A | C |
| ATOM | 432 | CD | LYS | A | 86 | 43.979 | 22.901 | 62.724 | 1.00 | 53.30 | A | C |
| ATOM | 433 | CE | LYS | A | 86 | 45.276 | 23.721 | 62.826 | 1.00 | 53.30 | A | C |
| ATOM | 434 | NZ | LYS | A | 86 | 46.458 | 22.818 | 62.951 | 1.00 | 53.30 | A | N |
| ATOM | 435 | C | LYS | A | 86 | 40.133 | 24.946 | 62.178 | 1.00 | 50.17 | A | C |
| ATOM | 436 | O | LYS | A | 86 | 40.207 | 25.769 | 63.093 | 1.00 | 50.17 | A | O |
| ATOM | 437 | N | VAL | A | 87 | 39.152 | 24.057 | 62.094 | 1.00 | 62.26 | A | N |
| ATOM | 438 | CA | VAL | A | 87 | 38.144 | 23.986 | 63.136 | 1.00 | 62.26 | A | C |
| ATOM | 439 | CB | VAL | A | 87 | 36.726 | 24.057 | 62.551 | 1.00 | 40.17 | A | C |
| ATOM | 440 | CG1 | VAL | A | 87 | 35.903 | 22.833 | 62.974 | 1.00 | 40.17 | A | C |
| ATOM | 441 | CG2 | VAL | A | 87 | 36.068 | 25.339 | 63.026 | 1.00 | 40.17 | A | C |
| ATOM | 442 | C | VAL | A | 87 | 38.337 | 22.694 | 63.921 | 1.00 | 62.26 | A | C |
| ATOM | 443 | O | VAL | A | 87 | 38.841 | 21.704 | 63.380 | 1.00 | 62.26 | A | O |
| ATOM | 444 | N | LEU | A | 88 | 37.945 | 22.714 | 65.197 | 1.00 | 70.44 | A | N |
| ATOM | 445 | CA | LEU | A | 88 | 38.098 | 21.551 | 66.070 | 1.00 | 70.44 | A | C |
| ATOM | 446 | CB | LEU | A | 88 | 37.788 | 21.938 | 67.525 | 1.00 | 54.57 | A | C |
| ATOM | 447 | CG | LEU | A | 88 | 38.304 | 20.988 | 68.621 | 1.00 | 54.57 | A | C |
| ATOM | 448 | CD1 | LEU | A | 88 | 37.422 | 19.718 | 68.722 | 1.00 | 54.57 | A | C |
| ATOM | 449 | CD2 | LEU | A | 88 | 39.772 | 20.628 | 68.315 | 1.00 | 54.57 | A | C |
| ATOM | 450 | C | LEU | A | 88 | 37.191 | 20.412 | 65.616 | 1.00 | 70.44 | A | C |
| ATOM | 451 | O | LEU | A | 88 | 36.187 | 20.119 | 66.261 | 1.00 | 70.44 | A | O |
| ATOM | 452 | N | ALA | A | 89 | 37.561 | 19.777 | 64.505 | 1.00 | 68.97 | A | N |
| ATOM | 453 | CA | ALA | A | 89 | 36.783 | 18.682 | 63.927 | 1.00 | 68.97 | A | C |
| ATOM | 454 | CB | ALA | A | 89 | 37.603 | 17.951 | 62.868 | 1.00 | 71.54 | A | C |
| ATOM | 455 | C | ALA | A | 89 | 36.330 | 17.699 | 64.984 | 1.00 | 68.97 | A | C |
| ATOM | 456 | O | ALA | A | 89 | 37.095 | 16.819 | 65.375 | 1.00 | 68.97 | A | O |
| ATOM | 457 | N | ALA | A | 90 | 35.088 | 17.862 | 65.442 | 1.00 | 83.82 | A | N |
| ATOM | 458 | CA | ALA | A | 90 | 34.507 | 16.981 | 66.453 | 1.00 | 83.82 | A | C |
| ATOM | 459 | CB | ALA | A | 90 | 33.102 | 17.482 | 66.847 | 1.00 | 23.65 | A | C |
| ATOM | 460 | C | ALA | A | 90 | 34.419 | 15.548 | 65.901 | 1.00 | 83.82 | A | C |
| ATOM | 461 | O | ALA | A | 90 | 35.216 | 15.132 | 65.036 | 1.00 | 83.82 | A | O |
| ATOM | 462 | N | ALA | A | 91 | 33.432 | 14.802 | 66.392 | 1.00 | 62.13 | A | N |
| ATOM | 463 | CA | ALA | A | 91 | 33.233 | 13.426 | 65.957 | 1.00 | 62.13 | A | C |

| | | | | | | | | | | | | |
|---|---|---|---|---|---|---|---|---|---|---|---|---|
| ATOM | 464 | CB | ALA | A | 91 | 34.138 | 12.476 | 66.770 | 1.00 | 74.56 | A | C |
| ATOM | 465 | C | ALA | A | 91 | 31.766 | 13.079 | 66.160 | 1.00 | 62.13 | A | C |
| ATOM | 466 | O | ALA | A | 91 | 31.141 | 12.401 | 65.330 | 1.00 | 62.13 | A | O |
| ATOM | 467 | N | ALA | A | 92 | 31.216 | 13.562 | 67.268 | 1.00 | 55.16 | A | N |
| ATOM | 468 | CA | ALA | A | 92 | 29.815 | 13.316 | 67.561 | 1.00 | 55.16 | A | C |
| ATOM | 469 | CB | ALA | A | 92 | 29.322 | 14.279 | 68.657 | 1.00 | 43.81 | A | C |
| ATOM | 470 | C | ALA | A | 92 | 28.967 | 13.478 | 66.283 | 1.00 | 55.16 | A | C |
| ATOM | 471 | O | ALA | A | 92 | 28.010 | 12.737 | 66.084 | 1.00 | 55.16 | A | O |
| ATOM | 472 | N | ALA | A | 93 | 29.327 | 14.425 | 65.414 | 1.00 | 71.33 | A | N |
| ATOM | 473 | CA | ALA | A | 93 | 28.576 | 14.672 | 64.170 | 1.00 | 71.33 | A | C |
| ATOM | 474 | CB | ALA | A | 93 | 27.644 | 15.903 | 64.351 | 1.00 | 34.18 | A | C |
| ATOM | 475 | C | ALA | A | 93 | 29.453 | 14.864 | 62.913 | 1.00 | 71.33 | A | C |
| ATOM | 476 | O | ALA | A | 93 | 30.677 | 14.668 | 62.936 | 1.00 | 71.33 | A | O |
| ATOM | 477 | N | ALA | A | 94 | 28.804 | 15.243 | 61.815 | 1.00 | 60.71 | A | N |
| ATOM | 478 | CA | ALA | A | 94 | 29.487 | 15.473 | 60.540 | 1.00 | 60.71 | A | C |
| ATOM | 479 | CB | ALA | A | 94 | 28.632 | 14.929 | 59.386 | 1.00 | 38.92 | A | C |
| ATOM | 480 | C | ALA | A | 94 | 29.702 | 16.978 | 60.370 | 1.00 | 60.71 | A | C |
| ATOM | 481 | O | ALA | A | 94 | 28.896 | 17.773 | 60.857 | 1.00 | 60.71 | A | O |
| ATOM | 482 | N | ASN | A | 95 | 30.767 | 17.387 | 59.689 | 1.00 | 35.64 | A | N |
| ATOM | 483 | CA | ASN | A | 95 | 30.977 | 18.822 | 59.519 | 1.00 | 35.64 | A | C |
| ATOM | 484 | CB | ASN | A | 95 | 32.430 | 19.126 | 59.201 | 1.00 | 36.43 | A | C |
| ATOM | 485 | CG | ASN | A | 95 | 32.803 | 20.537 | 59.571 | 1.00 | 36.43 | A | C |
| ATOM | 486 | OD1 | ASN | A | 95 | 32.619 | 21.474 | 58.780 | 1.00 | 36.43 | A | O |
| ATOM | 487 | ND2 | ASN | A | 95 | 33.314 | 20.707 | 60.795 | 1.00 | 36.43 | A | N |
| ATOM | 488 | C | ASN | A | 95 | 30.054 | 19.381 | 58.437 | 1.00 | 35.64 | A | C |
| ATOM | 489 | O | ASN | A | 95 | 30.204 | 19.092 | 57.245 | 1.00 | 35.64 | A | O |
| ATOM | 490 | N | ARG | A | 96 | 29.093 | 20.186 | 58.879 | 1.00 | 32.87 | A | N |
| ATOM | 491 | CA | ARG | A | 96 | 28.086 | 20.779 | 58.012 | 1.00 | 32.87 | A | C |
| ATOM | 492 | CB | ARG | A | 96 | 27.027 | 21.454 | 58.886 | 1.00 | 48.40 | A | C |
| ATOM | 493 | CG | ARG | A | 96 | 25.963 | 22.225 | 58.135 | 1.00 | 48.40 | A | C |
| ATOM | 494 | CD | ARG | A | 96 | 24.939 | 22.841 | 59.101 | 1.00 | 48.40 | A | C |
| ATOM | 495 | NE | ARG | A | 96 | 24.286 | 21.811 | 59.911 | 1.00 | 48.40 | A | N |
| ATOM | 496 | CZ | ARG | A | 96 | 23.295 | 22.026 | 60.774 | 1.00 | 48.40 | A | C |
| ATOM | 497 | NH1 | ARG | A | 96 | 22.821 | 23.249 | 60.962 | 1.00 | 48.40 | A | N |
| ATOM | 498 | NH2 | ARG | A | 96 | 22.765 | 21.008 | 61.441 | 1.00 | 48.40 | A | N |
| ATOM | 499 | C | ARG | A | 96 | 28.650 | 21.767 | 56.999 | 1.00 | 32.87 | A | C |
| ATOM | 500 | O | ARG | A | 96 | 28.202 | 21.803 | 55.851 | 1.00 | 32.87 | A | O |
| ATOM | 501 | N | GLU | A | 97 | 29.616 | 22.576 | 57.420 | 1.00 | 29.99 | A | N |
| ATOM | 502 | CA | GLU | A | 97 | 30.228 | 23.531 | 56.510 | 1.00 | 29.99 | A | C |
| ATOM | 503 | CB | GLU | A | 97 | 31.232 | 24.426 | 57.245 | 1.00 | 25.35 | A | C |
| ATOM | 504 | CG | GLU | A | 97 | 32.161 | 25.173 | 56.318 | 1.00 | 25.35 | A | C |
| ATOM | 505 | CD | GLU | A | 97 | 32.845 | 26.357 | 56.976 | 1.00 | 25.35 | A | C |
| ATOM | 506 | OE1 | GLU | A | 97 | 33.221 | 26.242 | 58.158 | 1.00 | 25.35 | A | O |
| ATOM | 507 | OE2 | GLU | A | 97 | 33.025 | 27.400 | 56.304 | 1.00 | 25.35 | A | O |
| ATOM | 508 | C | GLU | A | 97 | 30.921 | 22.750 | 55.407 | 1.00 | 29.99 | A | C |
| ATOM | 509 | O | GLU | A | 97 | 30.669 | 22.979 | 54.230 | 1.00 | 29.99 | A | O |
| ATOM | 510 | N | LEU | A | 98 | 31.779 | 21.813 | 55.797 | 1.00 | 37.36 | A | N |
| ATOM | 511 | CA | LEU | A | 98 | 32.488 | 20.978 | 54.837 | 1.00 | 37.36 | A | C |
| ATOM | 512 | CB | LEU | A | 98 | 33.300 | 19.913 | 55.555 | 1.00 | 28.06 | A | C |
| ATOM | 513 | CG | LEU | A | 98 | 33.986 | 18.945 | 54.597 | 1.00 | 28.06 | A | C |
| ATOM | 514 | CD1 | LEU | A | 98 | 34.775 | 19.715 | 53.563 | 1.00 | 28.06 | A | C |
| ATOM | 515 | CD2 | LEU | A | 98 | 34.893 | 18.027 | 55.384 | 1.00 | 28.06 | A | C |
| ATOM | 516 | C | LEU | A | 98 | 31.526 | 20.288 | 53.889 | 1.00 | 37.36 | A | C |
| ATOM | 517 | O | LEU | A | 98 | 31.730 | 20.284 | 52.681 | 1.00 | 37.36 | A | O |
| ATOM | 518 | N | GLN | A | 99 | 30.478 | 19.694 | 54.439 | 1.00 | 38.99 | A | N |
| ATOM | 519 | CA | GLN | A | 99 | 29.515 | 19.006 | 53.604 | 1.00 | 38.99 | A | C |
| ATOM | 520 | CB | GLN | A | 99 | 28.376 | 18.431 | 54.455 | 1.00 | 92.00 | A | C |
| ATOM | 521 | CG | GLN | A | 99 | 28.832 | 17.229 | 55.304 | 1.00 | 92.00 | A | C |
| ATOM | 522 | CD | GLN | A | 99 | 29.937 | 16.386 | 54.593 | 1.00 | 92.00 | A | C |
| ATOM | 523 | OE1 | GLN | A | 99 | 29.756 | 15.911 | 53.449 | 1.00 | 92.00 | A | O |
| ATOM | 524 | NE2 | GLN | A | 99 | 31.087 | 16.216 | 55.271 | 1.00 | 92.00 | A | N |
| ATOM | 525 | C | GLN | A | 99 | 28.978 | 19.935 | 52.532 | 1.00 | 38.99 | A | C |
| ATOM | 526 | O | GLN | A | 99 | 28.976 | 19.586 | 51.352 | 1.00 | 38.99 | A | O |
| ATOM | 527 | N | ILE | A | 100 | 28.545 | 21.121 | 52.937 | 1.00 | 33.64 | A | N |
| ATOM | 528 | CA | ILE | A | 100 | 27.996 | 22.106 | 52.007 | 1.00 | 33.64 | A | C |
| ATOM | 529 | CB | ILE | A | 100 | 27.445 | 23.315 | 52.788 | 1.00 | 33.36 | A | C |
| ATOM | 530 | CG2 | ILE | A | 100 | 27.114 | 24.449 | 51.825 | 1.00 | 33.36 | A | C |

| | | | | | | | | | | | | |
|---|---|---|---|---|---|---|---|---|---|---|---|---|
| ATOM | 531 | CG1 | ILE | A | 100 | 26.235 | 22.883 | 53.621 | 1.00 | 33.36 | A | C |
| ATOM | 532 | CD1 | ILE | A | 100 | 25.552 | 24.023 | 54.339 | 1.00 | 33.36 | A | C |
| ATOM | 533 | C | ILE | A | 100 | 29.014 | 22.624 | 50.986 | 1.00 | 33.64 | A | C |
| ATOM | 534 | O | ILE | A | 100 | 28.720 | 22.720 | 49.795 | 1.00 | 33.64 | A | O |
| ATOM | 535 | N | MET | A | 101 | 30.205 | 22.966 | 51.463 | 1.00 | 29.35 | A | N |
| ATOM | 536 | CA | MET | A | 101 | 31.280 | 23.475 | 50.604 | 1.00 | 29.35 | A | C |
| ATOM | 537 | CB | MET | A | 101 | 32.547 | 23.711 | 51.440 | 1.00 | 46.68 | A | C |
| ATOM | 538 | CG | MET | A | 101 | 32.467 | 24.820 | 52.479 | 1.00 | 46.68 | A | C |
| ATOM | 539 | SD | MET | A | 101 | 32.274 | 26.402 | 51.682 | 1.00 | 46.68 | A | S |
| ATOM | 540 | CE | MET | A | 101 | 33.954 | 26.658 | 51.082 | 1.00 | 46.68 | A | C |
| ATOM | 541 | C | MET | A | 101 | 31.604 | 22.479 | 49.496 | 1.00 | 29.35 | A | C |
| ATOM | 542 | O | MET | A | 101 | 31.848 | 22.830 | 48.342 | 1.00 | 29.35 | A | O |
| ATOM | 543 | N | ARG | A | 102 | 31.622 | 21.224 | 49.903 | 1.00 | 32.04 | A | N |
| ATOM | 544 | CA | ARG | A | 102 | 31.907 | 20.087 | 49.060 | 1.00 | 32.04 | A | C |
| ATOM | 545 | CB | ARG | A | 102 | 31.741 | 18.857 | 49.949 | 1.00 | 56.25 | A | C |
| ATOM | 546 | CG | ARG | A | 102 | 32.589 | 17.691 | 49.607 | 1.00 | 56.25 | A | C |
| ATOM | 547 | CD | ARG | A | 102 | 33.722 | 17.550 | 50.577 | 1.00 | 56.25 | A | C |
| ATOM | 548 | NE | ARG | A | 102 | 34.347 | 16.254 | 50.367 | 1.00 | 56.25 | A | N |
| ATOM | 549 | CZ | ARG | A | 102 | 33.754 | 15.091 | 50.627 | 1.00 | 56.25 | A | C |
| ATOM | 550 | NH1 | ARG | A | 102 | 32.518 | 15.055 | 51.119 | 1.00 | 56.25 | A | N |
| ATOM | 551 | NH2 | ARG | A | 102 | 34.395 | 13.957 | 50.380 | 1.00 | 56.25 | A | N |
| ATOM | 552 | C | ARG | A | 102 | 30.975 | 19.986 | 47.838 | 1.00 | 32.04 | A | C |
| ATOM | 553 | O | ARG | A | 102 | 31.359 | 19.435 | 46.807 | 1.00 | 32.04 | A | O |
| ATOM | 554 | N | LYS | A | 103 | 29.756 | 20.505 | 47.960 | 1.00 | 25.30 | A | N |
| ATOM | 555 | CA | LYS | A | 103 | 28.798 | 20.427 | 46.866 | 1.00 | 25.30 | A | C |
| ATOM | 556 | CB | LYS | A | 103 | 27.498 | 19.745 | 47.339 | 1.00 | 44.25 | A | C |
| ATOM | 557 | CG | LYS | A | 103 | 26.735 | 20.405 | 48.500 | 1.00 | 44.25 | A | C |
| ATOM | 558 | CD | LYS | A | 103 | 25.356 | 20.971 | 48.071 | 1.00 | 44.25 | A | C |
| ATOM | 559 | CE | LYS | A | 103 | 24.353 | 19.913 | 47.553 | 1.00 | 44.25 | A | C |
| ATOM | 560 | NZ | LYS | A | 103 | 23.772 | 19.026 | 48.618 | 1.00 | 44.25 | A | N |
| ATOM | 561 | C | LYS | A | 103 | 28.467 | 21.757 | 46.227 | 1.00 | 25.30 | A | C |
| ATOM | 562 | O | LYS | A | 103 | 27.437 | 21.895 | 45.576 | 1.00 | 25.30 | A | O |
| ATOM | 563 | N | LEU | A | 104 | 29.342 | 22.735 | 46.408 | 1.00 | 28.40 | A | N |
| ATOM | 564 | CA | LEU | A | 104 | 29.139 | 24.049 | 45.822 | 1.00 | 28.40 | A | C |
| ATOM | 565 | CB | LEU | A | 104 | 29.066 | 25.105 | 46.922 | 1.00 | 25.15 | A | C |
| ATOM | 566 | CG | LEU | A | 104 | 27.689 | 25.624 | 47.366 | 1.00 | 25.15 | A | C |
| ATOM | 567 | CD1 | LEU | A | 104 | 26.688 | 24.493 | 47.484 | 1.00 | 25.15 | A | C |
| ATOM | 568 | CD2 | LEU | A | 104 | 27.842 | 26.349 | 48.707 | 1.00 | 25.15 | A | C |
| ATOM | 569 | C | LEU | A | 104 | 30.260 | 24.378 | 44.858 | 1.00 | 28.40 | A | C |
| ATOM | 570 | O | LEU | A | 104 | 31.423 | 24.118 | 45.135 | 1.00 | 28.40 | A | O |
| ATOM | 571 | N | ASP | A | 105 | 29.889 | 24.926 | 43.710 | 1.00 | 35.77 | A | N |
| ATOM | 572 | CA | ASP | A | 105 | 30.836 | 25.313 | 42.675 | 1.00 | 35.77 | A | C |
| ATOM | 573 | CB | ASP | A | 105 | 31.203 | 24.101 | 41.810 | 1.00 | 40.56 | A | C |
| ATOM | 574 | CG | ASP | A | 105 | 32.194 | 24.441 | 40.696 | 1.00 | 40.56 | A | C |
| ATOM | 575 | OD1 | ASP | A | 105 | 33.107 | 25.270 | 40.915 | 1.00 | 40.56 | A | O |
| ATOM | 576 | OD2 | ASP | A | 105 | 32.069 | 23.864 | 39.596 | 1.00 | 40.56 | A | O |
| ATOM | 577 | C | ASP | A | 105 | 30.142 | 26.384 | 41.857 | 1.00 | 35.77 | A | C |
| ATOM | 578 | O | ASP | A | 105 | 29.207 | 26.111 | 41.084 | 1.00 | 35.77 | A | O |
| ATOM | 579 | N | HIS | A | 106 | 30.589 | 27.617 | 42.067 | 1.00 | 22.21 | A | N |
| ATOM | 580 | CA | HIS | A | 106 | 30.023 | 28.771 | 41.387 | 1.00 | 22.21 | A | C |
| ATOM | 581 | CB | HIS | A | 106 | 28.769 | 29.228 | 42.131 | 1.00 | 31.23 | A | C |
| ATOM | 582 | CG | HIS | A | 106 | 28.044 | 30.343 | 41.456 | 1.00 | 31.23 | A | C |
| ATOM | 583 | CD2 | HIS | A | 106 | 26.909 | 30.353 | 40.718 | 1.00 | 31.23 | A | C |
| ATOM | 584 | ND1 | HIS | A | 106 | 28.501 | 31.643 | 41.478 | 1.00 | 31.23 | A | N |
| ATOM | 585 | CE1 | HIS | A | 106 | 27.678 | 32.407 | 40.780 | 1.00 | 31.23 | A | C |
| ATOM | 586 | NE2 | HIS | A | 106 | 26.703 | 31.649 | 40.308 | 1.00 | 31.23 | A | N |
| ATOM | 587 | C | HIS | A | 106 | 31.087 | 29.858 | 41.340 | 1.00 | 22.21 | A | C |
| ATOM | 588 | O | HIS | A | 106 | 31.873 | 29.994 | 42.265 | 1.00 | 22.21 | A | O |
| ATOM | 589 | N | CYS | A | 107 | 31.120 | 30.611 | 40.246 | 1.00 | 27.47 | A | N |
| ATOM | 590 | CA | CYS | A | 107 | 32.113 | 31.662 | 40.061 | 1.00 | 27.47 | A | C |
| ATOM | 591 | CB | CYS | A | 107 | 31.947 | 32.289 | 38.666 | 1.00 | 42.62 | A | C |
| ATOM | 592 | SG | CYS | A | 107 | 30.474 | 33.363 | 38.427 | 1.00 | 42.62 | A | S |
| ATOM | 593 | C | CYS | A | 107 | 32.057 | 32.743 | 41.133 | 1.00 | 27.47 | A | C |
| ATOM | 594 | O | CYS | A | 107 | 32.939 | 33.585 | 41.210 | 1.00 | 27.47 | A | O |
| ATOM | 595 | N | ASN | A | 108 | 31.022 | 32.719 | 41.962 | 1.00 | 29.12 | A | N |
| ATOM | 596 | CA | ASN | A | 108 | 30.878 | 33.722 | 43.011 | 1.00 | 29.12 | A | C |
| ATOM | 597 | CB | ASN | A | 108 | 29.609 | 34.541 | 42.766 | 1.00 | 32.48 | A | C |

| | | | | | | | | | | | | |
|---|---|---|---|---|---|---|---|---|---|---|---|---|
| ATOM | 598 | CG | ASN | A | 108 | 29.683 | 35.356 | 41.487 | 1.00 | 32.48 | A | C |
| ATOM | 599 | OD1 | ASN | A | 108 | 28.820 | 35.276 | 40.620 | 1.00 | 32.48 | A | O |
| ATOM | 600 | ND2 | ASN | A | 108 | 30.717 | 36.158 | 41.378 | 1.00 | 32.48 | A | N |
| ATOM | 601 | C | ASN | A | 108 | 30.871 | 33.130 | 44.429 | 1.00 | 29.12 | A | C |
| ATOM | 602 | O | ASN | A | 108 | 30.273 | 33.686 | 45.337 | 1.00 | 29.12 | A | O |
| ATOM | 603 | N | ILE | A | 109 | 31.539 | 32.004 | 44.619 | 1.00 | 22.15 | A | N |
| ATOM | 604 | CA | ILE | A | 109 | 31.623 | 31.371 | 45.933 | 1.00 | 22.15 | A | C |
| ATOM | 605 | CB | ILE | A | 109 | 30.820 | 30.054 | 45.985 | 1.00 | 16.82 | A | C |
| ATOM | 606 | CG2 | ILE | A | 109 | 30.812 | 29.508 | 47.391 | 1.00 | 16.82 | A | C |
| ATOM | 607 | CG1 | ILE | A | 109 | 29.401 | 30.257 | 45.468 | 1.00 | 16.82 | A | C |
| ATOM | 608 | CD1 | ILE | A | 109 | 28.407 | 30.682 | 46.503 | 1.00 | 16.82 | A | C |
| ATOM | 609 | C | ILE | A | 109 | 33.095 | 30.973 | 46.144 | 1.00 | 22.15 | A | C |
| ATOM | 610 | O | ILE | A | 109 | 33.705 | 30.364 | 45.263 | 1.00 | 22.15 | A | O |
| ATOM | 611 | N | VAL | A | 110 | 33.674 | 31.287 | 47.293 | 1.00 | 18.62 | A | N |
| ATOM | 612 | CA | VAL | A | 110 | 35.061 | 30.903 | 47.503 | 1.00 | 18.62 | A | C |
| ATOM | 613 | CB | VAL | A | 110 | 35.540 | 31.080 | 48.946 | 1.00 | 15.07 | A | C |
| ATOM | 614 | CG1 | VAL | A | 110 | 35.828 | 32.525 | 49.222 | 1.00 | 15.07 | A | C |
| ATOM | 615 | CG2 | VAL | A | 110 | 34.509 | 30.517 | 49.906 | 1.00 | 15.07 | A | C |
| ATOM | 616 | C | VAL | A | 110 | 35.153 | 29.427 | 47.237 | 1.00 | 18.62 | A | C |
| ATOM | 617 | O | VAL | A | 110 | 34.273 | 28.676 | 47.624 | 1.00 | 18.62 | A | O |
| ATOM | 618 | N | ARG | A | 111 | 36.235 | 29.014 | 46.600 | 1.00 | 33.04 | A | N |
| ATOM | 619 | CA | ARG | A | 111 | 36.443 | 27.618 | 46.282 | 1.00 | 33.04 | A | C |
| ATOM | 620 | CB | ARG | A | 111 | 37.272 | 27.525 | 44.999 | 1.00 | 59.15 | A | C |
| ATOM | 621 | CG | ARG | A | 111 | 37.405 | 26.131 | 44.394 | 1.00 | 59.15 | A | C |
| ATOM | 622 | CD | ARG | A | 111 | 38.213 | 26.193 | 43.093 | 1.00 | 59.15 | A | C |
| ATOM | 623 | NE | ARG | A | 111 | 39.581 | 26.687 | 43.317 | 1.00 | 59.15 | A | N |
| ATOM | 624 | CZ | ARG | A | 111 | 40.197 | 27.616 | 42.575 | 1.00 | 59.15 | A | C |
| ATOM | 625 | NH1 | ARG | A | 111 | 39.568 | 28.171 | 41.535 | 1.00 | 59.15 | A | N |
| ATOM | 626 | NH2 | ARG | A | 111 | 41.442 | 28.000 | 42.881 | 1.00 | 59.15 | A | N |
| ATOM | 627 | C | ARG | A | 111 | 37.146 | 26.866 | 47.411 | 1.00 | 33.04 | A | C |
| ATOM | 628 | O | ARG | A | 111 | 38.020 | 27.410 | 48.087 | 1.00 | 33.04 | A | O |
| ATOM | 629 | N | LEU | A | 112 | 36.751 | 25.614 | 47.616 | 1.00 | 37.72 | A | N |
| ATOM | 630 | CA | LEU | A | 112 | 37.360 | 24.777 | 48.640 | 1.00 | 37.72 | A | C |
| ATOM | 631 | CB | LEU | A | 112 | 36.302 | 23.897 | 49.330 | 1.00 | 17.99 | A | C |
| ATOM | 632 | CG | LEU | A | 112 | 36.917 | 22.962 | 50.379 | 1.00 | 17.99 | A | C |
| ATOM | 633 | CD1 | LEU | A | 112 | 37.560 | 23.800 | 51.476 | 1.00 | 17.99 | A | C |
| ATOM | 634 | CD2 | LEU | A | 112 | 35.878 | 22.010 | 50.943 | 1.00 | 17.99 | A | C |
| ATOM | 635 | C | LEU | A | 112 | 38.413 | 23.899 | 47.950 | 1.00 | 37.72 | A | C |
| ATOM | 636 | O | LEU | A | 112 | 38.165 | 22.740 | 47.655 | 1.00 | 37.72 | A | O |
| ATOM | 637 | N | ARG | A | 113 | 39.585 | 24.459 | 47.683 | 1.00 | 29.46 | A | N |
| ATOM | 638 | CA | ARG | A | 113 | 40.656 | 23.713 | 47.023 | 1.00 | 29.46 | A | C |
| ATOM | 639 | CB | ARG | A | 113 | 41.973 | 24.487 | 47.136 | 1.00 | 48.42 | A | C |
| ATOM | 640 | CG | ARG | A | 113 | 41.923 | 25.924 | 46.632 | 1.00 | 48.42 | A | C |
| ATOM | 641 | CD | ARG | A | 113 | 41.958 | 25.992 | 45.106 | 1.00 | 48.42 | A | C |
| ATOM | 642 | NE | ARG | A | 113 | 43.286 | 25.704 | 44.548 | 1.00 | 48.42 | A | N |
| ATOM | 643 | CZ | ARG | A | 113 | 44.312 | 26.556 | 44.551 | 1.00 | 48.42 | A | C |
| ATOM | 644 | NH1 | ARG | A | 113 | 44.167 | 27.763 | 45.089 | 1.00 | 48.42 | A | N |
| ATOM | 645 | NH2 | ARG | A | 113 | 45.479 | 26.204 | 44.006 | 1.00 | 48.42 | A | N |
| ATOM | 646 | C | ARG | A | 113 | 40.872 | 22.295 | 47.569 | 1.00 | 29.46 | A | C |
| ATOM | 647 | O | ARG | A | 113 | 40.866 | 21.327 | 46.822 | 1.00 | 29.46 | A | O |
| ATOM | 648 | N | TYR | A | 114 | 41.081 | 22.184 | 48.875 | 1.00 | 35.20 | A | N |
| ATOM | 649 | CA | TYR | A | 114 | 41.318 | 20.903 | 49.518 | 1.00 | 35.20 | A | C |
| ATOM | 650 | CB | TYR | A | 114 | 42.807 | 20.585 | 49.554 | 1.00 | 27.30 | A | C |
| ATOM | 651 | CG | TYR | A | 114 | 43.530 | 20.726 | 48.245 | 1.00 | 27.30 | A | C |
| ATOM | 652 | CD1 | TYR | A | 114 | 43.481 | 19.723 | 47.285 | 1.00 | 27.30 | A | C |
| ATOM | 653 | CE1 | TYR | A | 114 | 44.184 | 19.842 | 46.087 | 1.00 | 27.30 | A | C |
| ATOM | 654 | CD2 | TYR | A | 114 | 44.297 | 21.855 | 47.976 | 1.00 | 27.30 | A | C |
| ATOM | 655 | CE2 | TYR | A | 114 | 44.998 | 21.982 | 46.787 | 1.00 | 27.30 | A | C |
| ATOM | 656 | CZ | TYR | A | 114 | 44.936 | 20.974 | 45.850 | 1.00 | 27.30 | A | C |
| ATOM | 657 | OH | TYR | A | 114 | 45.615 | 21.106 | 44.666 | 1.00 | 27.30 | A | O |
| ATOM | 658 | C | TYR | A | 114 | 40.856 | 21.024 | 50.956 | 1.00 | 35.20 | A | C |
| ATOM | 659 | O | TYR | A | 114 | 40.539 | 22.112 | 51.438 | 1.00 | 35.20 | A | O |
| ATOM | 660 | N | PHE | A | 115 | 40.832 | 19.887 | 51.637 | 1.00 | 43.80 | A | N |
| ATOM | 661 | CA | PHE | A | 115 | 40.489 | 19.839 | 53.045 | 1.00 | 43.80 | A | C |
| ATOM | 662 | CB | PHE | A | 115 | 38.970 | 19.800 | 53.235 | 1.00 | 32.92 | A | C |
| ATOM | 663 | CG | PHE | A | 115 | 38.345 | 18.490 | 52.917 | 1.00 | 32.92 | A | C |
| ATOM | 664 | CD1 | PHE | A | 115 | 38.305 | 17.480 | 53.861 | 1.00 | 32.92 | A | C |

| | | | | | | | | | | | | |
|---|---|---|---|---|---|---|---|---|---|---|---|---|
| ATOM | 665 | CD2 | PHE | A | 115 | 37.759 | 18.269 | 51.678 | 1.00 | 32.92 | A | C |
| ATOM | 666 | CE1 | PHE | A | 115 | 37.679 | 16.264 | 53.572 | 1.00 | 32.92 | A | C |
| ATOM | 667 | CE2 | PHE | A | 115 | 37.127 | 17.047 | 51.377 | 1.00 | 32.92 | A | C |
| ATOM | 668 | CZ | PHE | A | 115 | 37.086 | 16.051 | 52.321 | 1.00 | 32.92 | A | C |
| ATOM | 669 | C | PHE | A | 115 | 41.184 | 18.606 | 53.633 | 1.00 | 43.80 | A | C |
| ATOM | 670 | O | PHE | A | 115 | 41.296 | 17.579 | 52.968 | 1.00 | 43.80 | A | O |
| ATOM | 671 | N | PHE | A | 116 | 41.691 | 18.725 | 54.855 | 1.00 | 37.21 | A | N |
| ATOM | 672 | CA | PHE | A | 116 | 42.378 | 17.611 | 55.486 | 1.00 | 37.21 | A | C |
| ATOM | 673 | CB | PHE | A | 116 | 43.821 | 17.564 | 55.012 | 1.00 | 34.78 | A | C |
| ATOM | 674 | CG | PHE | A | 116 | 44.658 | 18.724 | 55.488 | 1.00 | 34.78 | A | C |
| ATOM | 675 | CD1 | PHE | A | 116 | 45.273 | 18.688 | 56.727 | 1.00 | 34.78 | A | C |
| ATOM | 676 | CD2 | PHE | A | 116 | 44.827 | 19.857 | 54.695 | 1.00 | 34.78 | A | C |
| ATOM | 677 | CE1 | PHE | A | 116 | 46.041 | 19.753 | 57.158 | 1.00 | 34.78 | A | C |
| ATOM | 678 | CE2 | PHE | A | 116 | 45.601 | 20.931 | 55.129 | 1.00 | 34.78 | A | C |
| ATOM | 679 | CZ | PHE | A | 116 | 46.203 | 20.878 | 56.355 | 1.00 | 34.78 | A | C |
| ATOM | 680 | C | PHE | A | 116 | 42.346 | 17.662 | 57.006 | 1.00 | 37.21 | A | C |
| ATOM | 681 | O | PHE | A | 116 | 42.095 | 18.707 | 57.607 | 1.00 | 37.21 | A | O |
| ATOM | 682 | N | TYR | A | 117 | 42.604 | 16.516 | 57.622 | 1.00 | 53.98 | A | N |
| ATOM | 683 | CA | TYR | A | 117 | 42.620 | 16.413 | 59.076 | 1.00 | 53.98 | A | C |
| ATOM | 684 | CB | TYR | A | 117 | 41.848 | 15.173 | 59.499 | 1.00 | 34.79 | A | C |
| ATOM | 685 | CG | TYR | A | 117 | 40.396 | 15.194 | 59.079 | 1.00 | 34.79 | A | C |
| ATOM | 686 | CD1 | TYR | A | 117 | 39.445 | 15.899 | 59.830 | 1.00 | 34.79 | A | C |
| ATOM | 687 | CE1 | TYR | A | 117 | 38.111 | 15.954 | 59.432 | 1.00 | 34.79 | A | C |
| ATOM | 688 | CD2 | TYR | A | 117 | 39.973 | 14.536 | 57.912 | 1.00 | 34.79 | A | C |
| ATOM | 689 | CE2 | TYR | A | 117 | 38.649 | 14.584 | 57.502 | 1.00 | 34.79 | A | C |
| ATOM | 690 | CZ | TYR | A | 117 | 37.721 | 15.299 | 58.265 | 1.00 | 34.79 | A | C |
| ATOM | 691 | OH | TYR | A | 117 | 36.408 | 15.396 | 57.850 | 1.00 | 34.79 | A | O |
| ATOM | 692 | C | TYR | A | 117 | 44.071 | 16.319 | 59.561 | 1.00 | 53.98 | A | C |
| ATOM | 693 | O | TYR | A | 117 | 44.890 | 15.650 | 58.929 | 1.00 | 53.98 | A | O |
| ATOM | 694 | N | SER | A | 118 | 44.399 | 16.978 | 60.671 | 1.00 | 60.68 | A | N |
| ATOM | 695 | CA | SER | A | 118 | 45.781 | 16.961 | 61.170 | 1.00 | 60.68 | A | C |
| ATOM | 696 | CB | SER | A | 118 | 46.483 | 18.259 | 60.775 | 1.00 | 52.20 | A | C |
| ATOM | 697 | OG | SER | A | 118 | 45.914 | 19.350 | 61.484 | 1.00 | 52.20 | A | O |
| ATOM | 698 | C | SER | A | 118 | 45.853 | 16.799 | 62.686 | 1.00 | 60.68 | A | C |
| ATOM | 699 | O | SER | A | 118 | 46.895 | 16.472 | 63.280 | 1.00 | 60.68 | A | O |
| ATOM | 700 | N | SER | A | 119 | 44.707 | 17.055 | 63.285 | 1.00 | 98.84 | A | N |
| ATOM | 701 | CA | SER | A | 119 | 44.475 | 17.011 | 64.721 | 1.00 | 98.84 | A | C |
| ATOM | 702 | CB | SER | A | 119 | 45.428 | 17.985 | 65.476 | 1.00 | 52.54 | A | C |
| ATOM | 703 | OG | SER | A | 119 | 46.788 | 17.538 | 65.446 | 1.00 | 52.54 | A | O |
| ATOM | 704 | C | SER | A | 119 | 42.966 | 17.563 | 64.670 | 1.00 | 98.84 | A | C |
| ATOM | 705 | O | SER | A | 119 | 42.115 | 17.172 | 65.493 | 1.00 | 98.84 | A | O |
| ATOM | 706 | N | TYR | A | 127 | 42.659 | 18.424 | 63.664 | 1.00 | 82.31 | A | N |
| ATOM | 707 | CA | TYR | A | 127 | 41.310 | 19.055 | 63.415 | 1.00 | 82.31 | A | C |
| ATOM | 708 | CB | TYR | A | 127 | 41.247 | 20.529 | 63.867 | 1.00 | 68.57 | A | C |
| ATOM | 709 | CG | TYR | A | 127 | 41.791 | 20.942 | 65.213 | 1.00 | 68.57 | A | C |
| ATOM | 710 | CD1 | TYR | A | 127 | 41.395 | 22.171 | 65.780 | 1.00 | 68.57 | A | C |
| ATOM | 711 | CE1 | TYR | A | 127 | 41.927 | 22.614 | 67.001 | 1.00 | 68.57 | A | C |
| ATOM | 712 | CD2 | TYR | A | 127 | 42.728 | 20.162 | 65.896 | 1.00 | 68.57 | A | C |
| ATOM | 713 | CE2 | TYR | A | 127 | 43.271 | 20.578 | 67.107 | 1.00 | 68.57 | A | C |
| ATOM | 714 | CZ | TYR | A | 127 | 42.870 | 21.811 | 67.661 | 1.00 | 68.57 | A | C |
| ATOM | 715 | OH | TYR | A | 127 | 43.407 | 22.246 | 68.860 | 1.00 | 68.57 | A | O |
| ATOM | 716 | C | TYR | A | 127 | 40.970 | 19.124 | 61.898 | 1.00 | 82.31 | A | C |
| ATOM | 717 | O | TYR | A | 127 | 41.807 | 18.794 | 61.055 | 1.00 | 82.31 | A | O |
| ATOM | 718 | N | LEU | A | 128 | 39.764 | 19.610 | 61.557 | 1.00 | 41.85 | A | N |
| ATOM | 719 | CA | LEU | A | 128 | 39.353 | 19.772 | 60.142 | 1.00 | 41.85 | A | C |
| ATOM | 720 | CB | LEU | A | 128 | 37.829 | 19.954 | 59.992 | 1.00 | 37.86 | A | C |
| ATOM | 721 | CG | LEU | A | 128 | 37.370 | 20.254 | 58.550 | 1.00 | 37.86 | A | C |
| ATOM | 722 | CD1 | LEU | A | 128 | 37.836 | 19.132 | 57.644 | 1.00 | 37.86 | A | C |
| ATOM | 723 | CD2 | LEU | A | 128 | 35.866 | 20.404 | 58.458 | 1.00 | 37.86 | A | C |
| ATOM | 724 | C | LEU | A | 128 | 40.046 | 21.011 | 59.568 | 1.00 | 41.85 | A | C |
| ATOM | 725 | O | LEU | A | 128 | 39.937 | 22.113 | 60.129 | 1.00 | 41.85 | A | O |
| ATOM | 726 | N | ASN | A | 129 | 40.748 | 20.834 | 58.452 | 1.00 | 41.60 | A | N |
| ATOM | 727 | CA | ASN | A | 129 | 41.458 | 21.939 | 57.823 | 1.00 | 41.60 | A | C |
| ATOM | 728 | CB | ASN | A | 129 | 42.950 | 21.611 | 57.670 | 1.00 | 28.06 | A | C |
| ATOM | 729 | CG | ASN | A | 129 | 43.649 | 21.444 | 59.001 | 1.00 | 28.06 | A | C |
| ATOM | 730 | OD1 | ASN | A | 129 | 43.262 | 20.594 | 59.800 | 1.00 | 28.06 | A | O |
| ATOM | 731 | ND2 | ASN | A | 129 | 44.680 | 22.253 | 59.252 | 1.00 | 28.06 | A | N |

| | | | | | | | | | | | | |
|---|---|---|---|---|---|---|---|---|---|---|---|---|
| ATOM | 732 | C | ASN | A | 129 | 40.891 | 22.246 | 56.464 | 1.00 | 41.60 | A | C |
| ATOM | 733 | O | ASN | A | 129 | 41.142 | 21.504 | 55.523 | 1.00 | 41.60 | A | O |
| ATOM | 734 | N | LEU | A | 130 | 40.140 | 23.339 | 56.355 | 1.00 | 41.24 | A | N |
| ATOM | 735 | CA | LEU | A | 130 | 39.557 | 23.735 | 55.076 | 1.00 | 41.24 | A | C |
| ATOM | 736 | CB | LEU | A | 130 | 38.196 | 24.387 | 55.304 | 1.00 | 31.07 | A | C |
| ATOM | 737 | CG | LEU | A | 130 | 37.185 | 23.502 | 56.033 | 1.00 | 31.07 | A | C |
| ATOM | 738 | CD1 | LEU | A | 130 | 36.012 | 24.342 | 56.498 | 1.00 | 31.07 | A | C |
| ATOM | 739 | CD2 | LEU | A | 130 | 36.728 | 22.387 | 55.124 | 1.00 | 31.07 | A | C |
| ATOM | 740 | C | LEU | A | 130 | 40.487 | 24.701 | 54.360 | 1.00 | 41.24 | A | C |
| ATOM | 741 | O | LEU | A | 130 | 40.770 | 25.784 | 54.868 | 1.00 | 41.24 | A | O |
| ATOM | 742 | N | VAL | A | 131 | 40.977 | 24.293 | 53.190 | 1.00 | 35.40 | A | N |
| ATOM | 743 | CA | VAL | A | 131 | 41.876 | 25.127 | 52.396 | 1.00 | 35.40 | A | C |
| ATOM | 744 | CB | VAL | A | 131 | 42.980 | 24.297 | 51.712 | 1.00 | 29.92 | A | C |
| ATOM | 745 | CG1 | VAL | A | 131 | 44.036 | 25.225 | 51.138 | 1.00 | 29.92 | A | C |
| ATOM | 746 | CG2 | VAL | A | 131 | 43.607 | 23.352 | 52.715 | 1.00 | 29.92 | A | C |
| ATOM | 747 | C | VAL | A | 131 | 41.057 | 25.815 | 51.325 | 1.00 | 35.40 | A | C |
| ATOM | 748 | O | VAL | A | 131 | 40.723 | 25.198 | 50.312 | 1.00 | 35.40 | A | O |
| ATOM | 749 | N | LEU | A | 132 | 40.723 | 27.084 | 51.555 | 1.00 | 27.18 | A | N |
| ATOM | 750 | CA | LEU | A | 132 | 39.918 | 27.838 | 50.592 | 1.00 | 27.18 | A | C |
| ATOM | 751 | CB | LEU | A | 132 | 38.779 | 28.596 | 51.276 | 1.00 | 27.88 | A | C |
| ATOM | 752 | CG | LEU | A | 132 | 37.951 | 27.859 | 52.317 | 1.00 | 27.88 | A | C |
| ATOM | 753 | CD1 | LEU | A | 132 | 38.508 | 28.155 | 53.696 | 1.00 | 27.88 | A | C |
| ATOM | 754 | CD2 | LEU | A | 132 | 36.502 | 28.301 | 52.214 | 1.00 | 27.88 | A | C |
| ATOM | 755 | C | LEU | A | 132 | 40.728 | 28.844 | 49.817 | 1.00 | 27.18 | A | C |
| ATOM | 756 | O | LEU | A | 132 | 41.799 | 29.258 | 50.255 | 1.00 | 27.18 | A | O |
| ATOM | 757 | N | ASP | A | 133 | 40.218 | 29.244 | 48.660 | 1.00 | 36.71 | A | N |
| ATOM | 758 | CA | ASP | A | 133 | 40.925 | 30.230 | 47.866 | 1.00 | 36.71 | A | C |
| ATOM | 759 | CB | ASP | A | 133 | 40.101 | 30.667 | 46.660 | 1.00 | 44.92 | A | C |
| ATOM | 760 | CG | ASP | A | 133 | 40.275 | 29.759 | 45.466 | 1.00 | 44.92 | A | C |
| ATOM | 761 | OD1 | ASP | A | 133 | 41.352 | 29.114 | 45.379 | 1.00 | 44.92 | A | O |
| ATOM | 762 | OD2 | ASP | A | 133 | 39.343 | 29.723 | 44.617 | 1.00 | 44.92 | A | O |
| ATOM | 763 | C | ASP | A | 133 | 41.115 | 31.442 | 48.746 | 1.00 | 36.71 | A | C |
| ATOM | 764 | O | ASP | A | 133 | 40.288 | 31.700 | 49.610 | 1.00 | 36.71 | A | O |
| ATOM | 765 | N | TYR | A | 134 | 42.189 | 32.190 | 48.539 | 1.00 | 29.35 | A | N |
| ATOM | 766 | CA | TYR | A | 134 | 42.390 | 33.394 | 49.328 | 1.00 | 29.35 | A | C |
| ATOM | 767 | CB | TYR | A | 134 | 43.769 | 33.422 | 49.980 | 1.00 | 40.36 | A | C |
| ATOM | 768 | CG | TYR | A | 134 | 44.044 | 34.742 | 50.669 | 1.00 | 40.36 | A | C |
| ATOM | 769 | CD1 | TYR | A | 134 | 43.617 | 34.969 | 51.982 | 1.00 | 40.36 | A | C |
| ATOM | 770 | CE1 | TYR | A | 134 | 43.784 | 36.215 | 52.591 | 1.00 | 40.36 | A | C |
| ATOM | 771 | CD2 | TYR | A | 134 | 44.654 | 35.794 | 49.977 | 1.00 | 40.36 | A | C |
| ATOM | 772 | CE2 | TYR | A | 134 | 44.825 | 37.040 | 50.568 | 1.00 | 40.36 | A | C |
| ATOM | 773 | CZ | TYR | A | 134 | 44.386 | 37.248 | 51.876 | 1.00 | 40.36 | A | C |
| ATOM | 774 | OH | TYR | A | 134 | 44.531 | 38.497 | 52.446 | 1.00 | 40.36 | A | O |
| ATOM | 775 | C | TYR | A | 134 | 42.229 | 34.642 | 48.482 | 1.00 | 29.35 | A | C |
| ATOM | 776 | O | TYR | A | 134 | 42.987 | 34.856 | 47.538 | 1.00 | 29.35 | A | O |
| ATOM | 777 | N | VAL | A | 135 | 41.239 | 35.458 | 48.829 | 1.00 | 24.33 | A | N |
| ATOM | 778 | CA | VAL | A | 135 | 40.970 | 36.699 | 48.123 | 1.00 | 24.33 | A | C |
| ATOM | 779 | CB | VAL | A | 135 | 39.467 | 36.887 | 47.863 | 1.00 | 34.47 | A | C |
| ATOM | 780 | CG1 | VAL | A | 135 | 39.241 | 38.128 | 47.009 | 1.00 | 34.47 | A | C |
| ATOM | 781 | CG2 | VAL | A | 135 | 38.905 | 35.642 | 47.163 | 1.00 | 34.47 | A | C |
| ATOM | 782 | C | VAL | A | 135 | 41.503 | 37.809 | 49.007 | 1.00 | 24.33 | A | C |
| ATOM | 783 | O | VAL | A | 135 | 41.270 | 37.808 | 50.204 | 1.00 | 24.33 | A | O |
| ATOM | 784 | N | PRO | A | 136 | 42.236 | 38.768 | 48.426 | 1.00 | 35.10 | A | N |
| ATOM | 785 | CD | PRO | A | 136 | 42.697 | 38.770 | 47.029 | 1.00 | 21.20 | A | C |
| ATOM | 786 | CA | PRO | A | 136 | 42.822 | 39.892 | 49.158 | 1.00 | 35.10 | A | C |
| ATOM | 787 | CB | PRO | A | 136 | 43.968 | 40.301 | 48.257 | 1.00 | 21.20 | A | C |
| ATOM | 788 | CG | PRO | A | 136 | 43.368 | 40.116 | 46.919 | 1.00 | 21.20 | A | C |
| ATOM | 789 | C | PRO | A | 136 | 41.953 | 41.089 | 49.545 | 1.00 | 35.10 | A | C |
| ATOM | 790 | O | PRO | A | 136 | 42.422 | 41.982 | 50.235 | 1.00 | 35.10 | A | O |
| ATOM | 791 | N | GLU | A | 137 | 40.703 | 41.139 | 49.117 | 1.00 | 29.78 | A | N |
| ATOM | 792 | CA | GLU | A | 137 | 39.882 | 42.275 | 49.486 | 1.00 | 29.78 | A | C |
| ATOM | 793 | CB | GLU | A | 137 | 39.801 | 43.224 | 48.293 | 1.00 | 31.81 | A | C |
| ATOM | 794 | CG | GLU | A | 137 | 39.272 | 44.591 | 48.620 | 1.00 | 31.81 | A | C |
| ATOM | 795 | CD | GLU | A | 137 | 40.145 | 45.337 | 49.613 | 1.00 | 31.81 | A | C |
| ATOM | 796 | OE1 | GLU | A | 137 | 40.990 | 46.139 | 49.181 | 1.00 | 31.81 | A | O |
| ATOM | 797 | OE2 | GLU | A | 137 | 39.994 | 45.120 | 50.831 | 1.00 | 31.81 | A | O |
| ATOM | 798 | C | GLU | A | 137 | 38.492 | 41.818 | 49.900 | 1.00 | 29.78 | A | C |

| | | | | | | | | | | | | |
|---|---|---|---|---|---|---|---|---|---|---|---|---|
| ATOM | 799 | O | GLU | A | 137 | 38.088 | 40.676 | 49.648 | 1.00 | 29.78 | A | O |
| ATOM | 800 | N | THR | A | 138 | 37.761 | 42.710 | 50.553 | 1.00 | 23.95 | A | N |
| ATOM | 801 | CA | THR | A | 138 | 36.411 | 42.403 | 50.994 | 1.00 | 23.95 | A | C |
| ATOM | 802 | CB | THR | A | 138 | 36.400 | 42.044 | 52.464 | 1.00 | 23.03 | A | C |
| ATOM | 803 | OG1 | THR | A | 138 | 36.553 | 43.236 | 53.243 | 1.00 | 23.03 | A | O |
| ATOM | 804 | CG2 | THR | A | 138 | 37.537 | 41.056 | 52.759 | 1.00 | 23.03 | A | C |
| ATOM | 805 | C | THR | A | 138 | 35.575 | 43.649 | 50.742 | 1.00 | 23.95 | A | C |
| ATOM | 806 | O | THR | A | 138 | 36.072 | 44.759 | 50.861 | 1.00 | 23.95 | A | O |
| ATOM | 807 | N | VAL | A | 139 | 34.322 | 43.470 | 50.345 | 1.00 | 17.50 | A | N |
| ATOM | 808 | CA | VAL | A | 139 | 33.452 | 44.605 | 50.094 | 1.00 | 17.50 | A | C |
| ATOM | 809 | CB | VAL | A | 139 | 32.021 | 44.117 | 49.815 | 1.00 | 17.20 | A | C |
| ATOM | 810 | CG1 | VAL | A | 139 | 31.019 | 45.205 | 50.092 | 1.00 | 17.20 | A | C |
| ATOM | 811 | CG2 | VAL | A | 139 | 31.916 | 43.685 | 48.370 | 1.00 | 17.20 | A | C |
| ATOM | 812 | C | VAL | A | 139 | 33.493 | 45.505 | 51.318 | 1.00 | 17.50 | A | C |
| ATOM | 813 | O | VAL | A | 139 | 33.475 | 46.725 | 51.213 | 1.00 | 17.50 | A | O |
| ATOM | 814 | N | TYR | A | 140 | 33.585 | 44.883 | 52.485 | 1.00 | 33.12 | A | N |
| ATOM | 815 | CA | TYR | A | 140 | 33.639 | 45.615 | 53.737 | 1.00 | 33.12 | A | C |
| ATOM | 816 | CB | TYR | A | 140 | 33.755 | 44.644 | 54.902 | 1.00 | 29.86 | A | C |
| ATOM | 817 | CG | TYR | A | 140 | 33.944 | 45.348 | 56.214 | 1.00 | 29.86 | A | C |
| ATOM | 818 | CD1 | TYR | A | 140 | 32.848 | 45.807 | 56.947 | 1.00 | 29.86 | A | C |
| ATOM | 819 | CE1 | TYR | A | 140 | 33.026 | 46.507 | 58.129 | 1.00 | 29.86 | A | C |
| ATOM | 820 | CD2 | TYR | A | 140 | 35.224 | 45.607 | 56.698 | 1.00 | 29.86 | A | C |
| ATOM | 821 | CE2 | TYR | A | 140 | 35.413 | 46.303 | 57.868 | 1.00 | 29.86 | A | C |
| ATOM | 822 | CZ | TYR | A | 140 | 34.316 | 46.751 | 58.583 | 1.00 | 29.86 | A | C |
| ATOM | 823 | OH | TYR | A | 140 | 34.524 | 47.437 | 59.754 | 1.00 | 29.86 | A | O |
| ATOM | 824 | C | TYR | A | 140 | 34.789 | 46.619 | 53.788 | 1.00 | 33.12 | A | C |
| ATOM | 825 | O | TYR | A | 140 | 34.561 | 47.797 | 54.047 | 1.00 | 33.12 | A | O |
| ATOM | 826 | N | ARG | A | 141 | 36.018 | 46.160 | 53.552 | 1.00 | 29.44 | A | N |
| ATOM | 827 | CA | ARG | A | 141 | 37.175 | 47.056 | 53.579 | 1.00 | 29.44 | A | C |
| ATOM | 828 | CB | ARG | A | 141 | 38.468 | 46.281 | 53.335 | 1.00 | 45.77 | A | C |
| ATOM | 829 | CG | ARG | A | 141 | 38.668 | 45.100 | 54.259 | 1.00 | 45.77 | A | C |
| ATOM | 830 | CD | ARG | A | 141 | 40.149 | 44.831 | 54.466 | 1.00 | 45.77 | A | C |
| ATOM | 831 | NE | ARG | A | 141 | 40.942 | 45.326 | 53.342 | 1.00 | 45.77 | A | N |
| ATOM | 832 | CZ | ARG | A | 141 | 42.250 | 45.131 | 53.196 | 1.00 | 45.77 | A | C |
| ATOM | 833 | NH1 | ARG | A | 141 | 42.925 | 44.444 | 54.112 | 1.00 | 45.77 | A | N |
| ATOM | 834 | NH2 | ARG | A | 141 | 42.875 | 45.617 | 52.123 | 1.00 | 45.77 | A | N |
| ATOM | 835 | C | ARG | A | 141 | 37.044 | 48.155 | 52.532 | 1.00 | 29.44 | A | C |
| ATOM | 836 | O | ARG | A | 141 | 37.315 | 49.317 | 52.807 | 1.00 | 29.44 | A | O |
| ATOM | 837 | N | VAL | A | 142 | 36.623 | 47.790 | 51.330 | 1.00 | 26.03 | A | N |
| ATOM | 838 | CA | VAL | A | 142 | 36.467 | 48.768 | 50.271 | 1.00 | 26.03 | A | C |
| ATOM | 839 | CB | VAL | A | 142 | 36.127 | 48.084 | 48.934 | 1.00 | 26.17 | A | C |
| ATOM | 840 | CG1 | VAL | A | 142 | 35.595 | 49.101 | 47.934 | 1.00 | 26.17 | A | C |
| ATOM | 841 | CG2 | VAL | A | 142 | 37.387 | 47.422 | 48.375 | 1.00 | 26.17 | A | C |
| ATOM | 842 | C | VAL | A | 142 | 35.424 | 49.823 | 50.597 | 1.00 | 26.03 | A | C |
| ATOM | 843 | O | VAL | A | 142 | 35.627 | 50.988 | 50.289 | 1.00 | 26.03 | A | O |
| ATOM | 844 | N | ALA | A | 143 | 34.316 | 49.434 | 51.213 | 1.00 | 18.87 | A | N |
| ATOM | 845 | CA | ALA | A | 143 | 33.285 | 50.408 | 51.554 | 1.00 | 18.87 | A | C |
| ATOM | 846 | CB | ALA | A | 143 | 32.053 | 49.703 | 52.060 | 1.00 | 22.77 | A | C |
| ATOM | 847 | C | ALA | A | 143 | 33.798 | 51.373 | 52.608 | 1.00 | 18.87 | A | C |
| ATOM | 848 | O | ALA | A | 143 | 33.561 | 52.574 | 52.538 | 1.00 | 18.87 | A | O |
| ATOM | 849 | N | ARG | A | 144 | 34.501 | 50.826 | 53.586 | 1.00 | 28.56 | A | N |
| ATOM | 850 | CA | ARG | A | 144 | 35.061 | 51.599 | 54.671 | 1.00 | 28.56 | A | C |
| ATOM | 851 | CB | ARG | A | 144 | 35.756 | 50.648 | 55.618 | 1.00 | 76.36 | A | C |
| ATOM | 852 | CG | ARG | A | 144 | 36.730 | 51.255 | 56.567 | 1.00 | 76.36 | A | C |
| ATOM | 853 | CD | ARG | A | 144 | 37.143 | 50.195 | 57.579 | 1.00 | 76.36 | A | C |
| ATOM | 854 | NE | ARG | A | 144 | 38.233 | 50.643 | 58.450 | 1.00 | 76.36 | A | N |
| ATOM | 855 | CZ | ARG | A | 144 | 38.254 | 51.797 | 59.128 | 1.00 | 76.36 | A | C |
| ATOM | 856 | NH1 | ARG | A | 144 | 37.230 | 52.653 | 59.043 | 1.00 | 76.36 | A | N |
| ATOM | 857 | NH2 | ARG | A | 144 | 39.304 | 52.087 | 59.905 | 1.00 | 76.36 | A | N |
| ATOM | 858 | C | ARG | A | 144 | 36.012 | 52.658 | 54.159 | 1.00 | 28.56 | A | C |
| ATOM | 859 | O | ARG | A | 144 | 36.032 | 53.774 | 54.673 | 1.00 | 28.56 | A | O |
| ATOM | 860 | N | HIS | A | 145 | 36.801 | 52.302 | 53.149 | 1.00 | 33.41 | A | N |
| ATOM | 861 | CA | HIS | A | 145 | 37.758 | 53.222 | 52.538 | 1.00 | 33.41 | A | C |
| ATOM | 862 | CB | HIS | A | 145 | 38.504 | 52.475 | 51.425 | 1.00 | 53.58 | A | C |
| ATOM | 863 | CG | HIS | A | 145 | 39.633 | 53.237 | 50.806 | 1.00 | 53.58 | A | C |
| ATOM | 864 | CD2 | HIS | A | 145 | 40.970 | 53.015 | 50.820 | 1.00 | 53.58 | A | C |
| ATOM | 865 | ND1 | HIS | A | 145 | 39.435 | 54.305 | 49.955 | 1.00 | 53.58 | A | N |

| | | | | | | | | | | | | |
|---|---|---|---|---|---|---|---|---|---|---|---|---|
| ATOM | 866 | CE1 | HIS | A | 145 | 40.598 | 54.698 | 49.462 | 1.00 | 53.58 | A | C |
| ATOM | 867 | NE2 | HIS | A | 145 | 41.545 | 53.930 | 49.970 | 1.00 | 53.58 | A | N |
| ATOM | 868 | C | HIS | A | 145 | 37.030 | 54.456 | 51.985 | 1.00 | 33.41 | A | C |
| ATOM | 869 | O | HIS | A | 145 | 37.299 | 55.591 | 52.400 | 1.00 | 33.41 | A | O |
| ATOM | 870 | N | TYR | A | 146 | 36.094 | 54.220 | 51.067 | 1.00 | 38.87 | A | N |
| ATOM | 871 | CA | TYR | A | 146 | 35.320 | 55.301 | 50.459 | 1.00 | 38.87 | A | C |
| ATOM | 872 | CB | TYR | A | 146 | 34.327 | 54.749 | 49.434 | 1.00 | 24.39 | A | C |
| ATOM | 873 | CG | TYR | A | 146 | 35.001 | 54.373 | 48.156 | 1.00 | 24.39 | A | C |
| ATOM | 874 | CD1 | TYR | A | 146 | 35.957 | 53.379 | 48.134 | 1.00 | 24.39 | A | C |
| ATOM | 875 | CE1 | TYR | A | 146 | 36.679 | 53.108 | 46.989 | 1.00 | 24.39 | A | C |
| ATOM | 876 | CD2 | TYR | A | 146 | 34.768 | 55.083 | 46.992 | 1.00 | 24.39 | A | C |
| ATOM | 877 | CE2 | TYR | A | 146 | 35.483 | 54.823 | 45.836 | 1.00 | 24.39 | A | C |
| ATOM | 878 | CZ | TYR | A | 146 | 36.450 | 53.833 | 45.839 | 1.00 | 24.39 | A | C |
| ATOM | 879 | OH | TYR | A | 146 | 37.240 | 53.587 | 44.727 | 1.00 | 24.39 | A | O |
| ATOM | 880 | C | TYR | A | 146 | 34.570 | 56.085 | 51.521 | 1.00 | 38.87 | A | C |
| ATOM | 881 | O | TYR | A | 146 | 34.440 | 57.305 | 51.439 | 1.00 | 38.87 | A | O |
| ATOM | 882 | N | SER | A | 147 | 34.089 | 55.380 | 52.531 | 1.00 | 41.51 | A | N |
| ATOM | 883 | CA | SER | A | 147 | 33.341 | 56.030 | 53.589 | 1.00 | 41.51 | A | C |
| ATOM | 884 | CB | SER | A | 147 | 32.704 | 54.991 | 54.493 | 1.00 | 32.78 | A | C |
| ATOM | 885 | OG | SER | A | 147 | 32.061 | 55.635 | 55.569 | 1.00 | 32.78 | A | O |
| ATOM | 886 | C | SER | A | 147 | 34.230 | 56.934 | 54.417 | 1.00 | 41.51 | A | C |
| ATOM | 887 | O | SER | A | 147 | 33.836 | 58.046 | 54.771 | 1.00 | 41.51 | A | O |
| ATOM | 888 | N | ARG | A | 148 | 35.425 | 56.440 | 54.735 | 1.00 | 41.62 | A | N |
| ATOM | 889 | CA | ARG | A | 148 | 36.389 | 57.205 | 55.509 | 1.00 | 41.62 | A | C |
| ATOM | 890 | CB | ARG | A | 148 | 37.688 | 56.419 | 55.669 | 1.00 | 65.42 | A | C |
| ATOM | 891 | CG | ARG | A | 148 | 37.690 | 55.402 | 56.786 | 1.00 | 65.42 | A | C |
| ATOM | 892 | CD | ARG | A | 148 | 39.031 | 54.669 | 56.814 | 1.00 | 65.42 | A | C |
| ATOM | 893 | NE | ARG | A | 148 | 39.549 | 54.533 | 58.178 | 1.00 | 65.42 | A | N |
| ATOM | 894 | CZ | ARG | A | 148 | 40.061 | 55.531 | 58.908 | 1.00 | 65.42 | A | C |
| ATOM | 895 | NH1 | ARG | A | 148 | 40.135 | 56.767 | 58.412 | 1.00 | 65.42 | A | N |
| ATOM | 896 | NH2 | ARG | A | 148 | 40.507 | 55.298 | 60.143 | 1.00 | 65.42 | A | N |
| ATOM | 897 | C | ARG | A | 148 | 36.675 | 58.494 | 54.760 | 1.00 | 41.62 | A | C |
| ATOM | 898 | O | ARG | A | 148 | 36.526 | 59.582 | 55.302 | 1.00 | 41.62 | A | O |
| ATOM | 899 | N | ALA | A | 149 | 37.082 | 58.350 | 53.502 | 1.00 | 40.20 | A | N |
| ATOM | 900 | CA | ALA | A | 149 | 37.396 | 59.479 | 52.640 | 1.00 | 40.20 | A | C |
| ATOM | 901 | CB | ALA | A | 149 | 38.069 | 58.985 | 51.356 | 1.00 | 27.77 | A | C |
| ATOM | 902 | C | ALA | A | 149 | 36.122 | 60.254 | 52.285 | 1.00 | 40.20 | A | C |
| ATOM | 903 | O | ALA | A | 149 | 36.115 | 61.001 | 51.307 | 1.00 | 40.20 | A | O |
| ATOM | 904 | N | LYS | A | 150 | 35.060 | 60.081 | 53.075 | 1.00 | 41.83 | A | N |
| ATOM | 905 | CA | LYS | A | 150 | 33.774 | 60.749 | 52.842 | 1.00 | 41.83 | A | C |
| ATOM | 906 | CB | LYS | A | 150 | 33.775 | 62.180 | 53.406 | 1.00 | 53.62 | A | C |
| ATOM | 907 | CG | LYS | A | 150 | 33.635 | 62.272 | 54.923 | 1.00 | 53.62 | A | C |
| ATOM | 908 | CD | LYS | A | 150 | 32.651 | 63.387 | 55.364 | 1.00 | 53.62 | A | C |
| ATOM | 909 | CE | LYS | A | 150 | 33.146 | 64.812 | 55.025 | 1.00 | 53.62 | A | C |
| ATOM | 910 | NZ | LYS | A | 150 | 32.268 | 65.890 | 55.620 | 1.00 | 53.62 | A | N |
| ATOM | 911 | C | LYS | A | 150 | 33.392 | 60.795 | 51.365 | 1.00 | 41.83 | A | C |
| ATOM | 912 | O | LYS | A | 150 | 32.978 | 61.830 | 50.851 | 1.00 | 41.83 | A | O |
| ATOM | 913 | N | GLN | A | 151 | 33.551 | 59.671 | 50.680 | 1.00 | 43.44 | A | N |
| ATOM | 914 | CA | GLN | A | 151 | 33.193 | 59.582 | 49.274 | 1.00 | 43.44 | A | C |
| ATOM | 915 | CB | GLN | A | 151 | 34.415 | 59.233 | 48.451 | 1.00 | 48.03 | A | C |
| ATOM | 916 | CG | GLN | A | 151 | 35.655 | 59.971 | 48.837 | 1.00 | 48.03 | A | C |
| ATOM | 917 | CD | GLN | A | 151 | 36.788 | 59.703 | 47.854 | 1.00 | 48.03 | A | C |
| ATOM | 918 | OE1 | GLN | A | 151 | 37.974 | 59.751 | 48.223 | 1.00 | 48.03 | A | O |
| ATOM | 919 | NE2 | GLN | A | 151 | 36.430 | 59.422 | 46.582 | 1.00 | 48.03 | A | N |
| ATOM | 920 | C | GLN | A | 151 | 32.157 | 58.470 | 49.099 | 1.00 | 43.44 | A | C |
| ATOM | 921 | O | GLN | A | 151 | 31.711 | 57.850 | 50.068 | 1.00 | 43.44 | A | O |
| ATOM | 922 | N | THR | A | 152 | 31.769 | 58.218 | 47.858 | 1.00 | 43.50 | A | N |
| ATOM | 923 | CA | THR | A | 152 | 30.814 | 57.155 | 47.606 | 1.00 | 43.50 | A | C |
| ATOM | 924 | CB | THR | A | 152 | 29.463 | 57.699 | 47.083 | 1.00 | 66.50 | A | C |
| ATOM | 925 | OG1 | THR | A | 152 | 28.559 | 57.872 | 48.184 | 1.00 | 66.50 | A | O |
| ATOM | 926 | CG2 | THR | A | 152 | 28.848 | 56.741 | 46.058 | 1.00 | 66.50 | A | C |
| ATOM | 927 | C | THR | A | 152 | 31.404 | 56.184 | 46.600 | 1.00 | 43.50 | A | C |
| ATOM | 928 | O | THR | A | 152 | 32.116 | 56.574 | 45.671 | 1.00 | 43.50 | A | O |
| ATOM | 929 | N | LEU | A | 153 | 31.123 | 54.910 | 46.797 | 1.00 | 33.25 | A | N |
| ATOM | 930 | CA | LEU | A | 153 | 31.626 | 53.919 | 45.879 | 1.00 | 33.25 | A | C |
| ATOM | 931 | CB | LEU | A | 153 | 31.409 | 52.509 | 46.424 | 1.00 | 35.15 | A | C |
| ATOM | 932 | CG | LEU | A | 153 | 31.955 | 51.435 | 45.489 | 1.00 | 35.15 | A | C |

| | | | | | | | | | | | | |
|---|---|---|---|---|---|---|---|---|---|---|---|---|
| ATOM | 933 | CD1 | LEU | A | 153 | 33.476 | 51.558 | 45.445 | 1.00 | 35.15 | A | C |
| ATOM | 934 | CD2 | LEU | A | 153 | 31.520 | 50.063 | 45.967 | 1.00 | 35.15 | A | C |
| ATOM | 935 | C | LEU | A | 153 | 30.869 | 54.072 | 44.567 | 1.00 | 33.25 | A | C |
| ATOM | 936 | O | LEU | A | 153 | 29.634 | 54.079 | 44.547 | 1.00 | 33.25 | A | O |
| ATOM | 937 | N | PRO | A | 154 | 31.600 | 54.222 | 43.455 | 1.00 | 30.73 | A | N |
| ATOM | 938 | CD | PRO | A | 154 | 33.067 | 54.302 | 43.355 | 1.00 | 33.43 | A | C |
| ATOM | 939 | CA | PRO | A | 154 | 30.968 | 54.367 | 42.142 | 1.00 | 30.73 | A | C |
| ATOM | 940 | CB | PRO | A | 154 | 32.140 | 54.207 | 41.184 | 1.00 | 33.43 | A | C |
| ATOM | 941 | CG | PRO | A | 154 | 33.267 | 54.814 | 41.940 | 1.00 | 33.43 | A | C |
| ATOM | 942 | C | PRO | A | 154 | 29.926 | 53.265 | 41.936 | 1.00 | 30.73 | A | C |
| ATOM | 943 | O | PRO | A | 154 | 30.246 | 52.072 | 41.960 | 1.00 | 30.73 | A | O |
| ATOM | 944 | N | VAL | A | 155 | 28.681 | 53.675 | 41.724 | 1.00 | 36.26 | A | N |
| ATOM | 945 | CA | VAL | A | 155 | 27.573 | 52.752 | 41.521 | 1.00 | 36.26 | A | C |
| ATOM | 946 | CB | VAL | A | 155 | 26.346 | 53.504 | 40.993 | 1.00 | 39.09 | A | C |
| ATOM | 947 | CG1 | VAL | A | 155 | 26.050 | 54.709 | 41.893 | 1.00 | 39.09 | A | C |
| ATOM | 948 | CG2 | VAL | A | 155 | 26.590 | 53.932 | 39.568 | 1.00 | 39.09 | A | C |
| ATOM | 949 | C | VAL | A | 155 | 27.905 | 51.598 | 40.572 | 1.00 | 36.26 | A | C |
| ATOM | 950 | O | VAL | A | 155 | 27.410 | 50.489 | 40.739 | 1.00 | 36.26 | A | O |
| ATOM | 951 | N | ILE | A | 156 | 28.744 | 51.848 | 39.579 | 1.00 | 23.38 | A | N |
| ATOM | 952 | CA | ILE | A | 156 | 29.096 | 50.799 | 38.638 | 1.00 | 23.38 | A | C |
| ATOM | 953 | CB | ILE | A | 156 | 30.180 | 51.280 | 37.658 | 1.00 | 23.03 | A | C |
| ATOM | 954 | CG2 | ILE | A | 156 | 31.352 | 51.879 | 38.436 | 1.00 | 23.03 | A | C |
| ATOM | 955 | CG1 | ILE | A | 156 | 30.633 | 50.127 | 36.761 | 1.00 | 23.03 | A | C |
| ATOM | 956 | CD1 | ILE | A | 156 | 29.556 | 49.560 | 35.907 | 1.00 | 23.03 | A | C |
| ATOM | 957 | C | ILE | A | 156 | 29.603 | 49.597 | 39.411 | 1.00 | 23.38 | A | C |
| ATOM | 958 | O | ILE | A | 156 | 29.350 | 48.460 | 39.031 | 1.00 | 23.38 | A | O |
| ATOM | 959 | N | TYR | A | 157 | 30.326 | 49.860 | 40.496 | 1.00 | 31.92 | A | N |
| ATOM | 960 | CA | TYR | A | 157 | 30.853 | 48.799 | 41.347 | 1.00 | 31.92 | A | C |
| ATOM | 961 | CB | TYR | A | 157 | 31.939 | 49.339 | 42.273 | 1.00 | 37.39 | A | C |
| ATOM | 962 | CG | TYR | A | 157 | 33.249 | 49.528 | 41.560 | 1.00 | 37.39 | A | C |
| ATOM | 963 | CD1 | TYR | A | 157 | 33.854 | 50.784 | 41.472 | 1.00 | 37.39 | A | C |
| ATOM | 964 | CE1 | TYR | A | 157 | 35.043 | 50.953 | 40.769 | 1.00 | 37.39 | A | C |
| ATOM | 965 | CD2 | TYR | A | 157 | 33.869 | 48.448 | 40.931 | 1.00 | 37.39 | A | C |
| ATOM | 966 | CE2 | TYR | A | 157 | 35.046 | 48.603 | 40.229 | 1.00 | 37.39 | A | C |
| ATOM | 967 | CZ | TYR | A | 157 | 35.632 | 49.855 | 40.149 | 1.00 | 37.39 | A | C |
| ATOM | 968 | OH | TYR | A | 157 | 36.813 | 50.003 | 39.460 | 1.00 | 37.39 | A | O |
| ATOM | 969 | C | TYR | A | 157 | 29.728 | 48.224 | 42.180 | 1.00 | 31.92 | A | C |
| ATOM | 970 | O | TYR | A | 157 | 29.647 | 47.017 | 42.389 | 1.00 | 31.92 | A | O |
| ATOM | 971 | N | VAL | A | 158 | 28.867 | 49.107 | 42.670 | 1.00 | 29.52 | A | N |
| ATOM | 972 | CA | VAL | A | 158 | 27.729 | 48.684 | 43.463 | 1.00 | 29.52 | A | C |
| ATOM | 973 | CB | VAL | A | 158 | 26.807 | 49.886 | 43.799 | 1.00 | 20.87 | A | C |
| ATOM | 974 | CG1 | VAL | A | 158 | 25.562 | 49.410 | 44.540 | 1.00 | 20.87 | A | C |
| ATOM | 975 | CG2 | VAL | A | 158 | 27.564 | 50.903 | 44.635 | 1.00 | 20.87 | A | C |
| ATOM | 976 | C | VAL | A | 158 | 26.960 | 47.668 | 42.626 | 1.00 | 29.52 | A | C |
| ATOM | 977 | O | VAL | A | 158 | 26.588 | 46.598 | 43.111 | 1.00 | 29.52 | A | O |
| ATOM | 978 | N | LYS | A | 159 | 26.729 | 48.012 | 41.363 | 1.00 | 21.26 | A | N |
| ATOM | 979 | CA | LYS | A | 159 | 26.019 | 47.131 | 40.456 | 1.00 | 21.26 | A | C |
| ATOM | 980 | CB | LYS | A | 159 | 25.894 | 47.778 | 39.077 | 1.00 | 23.81 | A | C |
| ATOM | 981 | CG | LYS | A | 159 | 24.843 | 48.883 | 39.041 | 1.00 | 23.81 | A | C |
| ATOM | 982 | CD | LYS | A | 159 | 24.833 | 49.665 | 37.744 | 1.00 | 23.81 | A | C |
| ATOM | 983 | CE | LYS | A | 159 | 23.813 | 50.774 | 37.816 | 1.00 | 23.81 | A | C |
| ATOM | 984 | NZ | LYS | A | 159 | 24.120 | 51.889 | 36.896 | 1.00 | 23.81 | A | N |
| ATOM | 985 | C | LYS | A | 159 | 26.763 | 45.817 | 40.369 | 1.00 | 21.26 | A | C |
| ATOM | 986 | O | LYS | A | 159 | 26.236 | 44.783 | 40.771 | 1.00 | 21.26 | A | O |
| ATOM | 987 | N | LEU | A | 160 | 27.987 | 45.859 | 39.851 | 1.00 | 19.23 | A | N |
| ATOM | 988 | CA | LEU | A | 160 | 28.815 | 44.661 | 39.730 | 1.00 | 19.23 | A | C |
| ATOM | 989 | CB | LEU | A | 160 | 30.258 | 45.048 | 39.430 | 1.00 | 22.21 | A | C |
| ATOM | 990 | CG | LEU | A | 160 | 30.614 | 45.232 | 37.963 | 1.00 | 22.21 | A | C |
| ATOM | 991 | CD1 | LEU | A | 160 | 31.963 | 45.888 | 37.854 | 1.00 | 22.21 | A | C |
| ATOM | 992 | CD2 | LEU | A | 160 | 30.620 | 43.877 | 37.278 | 1.00 | 22.21 | A | C |
| ATOM | 993 | C | LEU | A | 160 | 28.797 | 43.774 | 40.964 | 1.00 | 19.23 | A | C |
| ATOM | 994 | O | LEU | A | 160 | 28.488 | 42.594 | 40.869 | 1.00 | 19.23 | A | O |
| ATOM | 995 | N | TYR | A | 161 | 29.133 | 44.340 | 42.120 | 1.00 | 36.30 | A | N |
| ATOM | 996 | CA | TYR | A | 161 | 29.167 | 43.567 | 43.355 | 1.00 | 36.30 | A | C |
| ATOM | 997 | CB | TYR | A | 161 | 29.760 | 44.416 | 44.495 | 1.00 | 27.20 | A | C |
| ATOM | 998 | CG | TYR | A | 161 | 31.175 | 44.957 | 44.253 | 1.00 | 27.20 | A | C |
| ATOM | 999 | CD1 | TYR | A | 161 | 32.106 | 44.258 | 43.485 | 1.00 | 27.20 | A | C |

| | | | | | | | | | | | | |
|---|---|---|---|---|---|---|---|---|---|---|---|---|
| ATOM | 1000 | CE1 | TYR | A | 161 | 33.395 | 44.757 | 43.277 | 1.00 | 27.20 | A | C |
| ATOM | 1001 | CD2 | TYR | A | 161 | 31.578 | 46.167 | 44.805 | 1.00 | 27.20 | A | C |
| ATOM | 1002 | CE2 | TYR | A | 161 | 32.861 | 46.666 | 44.602 | 1.00 | 27.20 | A | C |
| ATOM | 1003 | CZ | TYR | A | 161 | 33.763 | 45.958 | 43.840 | 1.00 | 27.20 | A | C |
| ATOM | 1004 | OH | TYR | A | 161 | 35.040 | 46.445 | 43.655 | 1.00 | 27.20 | A | O |
| ATOM | 1005 | C | TYR | A | 161 | 27.802 | 42.989 | 43.752 | 1.00 | 36.30 | A | C |
| ATOM | 1006 | O | TYR | A | 161 | 27.674 | 41.783 | 43.978 | 1.00 | 36.30 | A | O |
| ATOM | 1007 | N | MET | A | 162 | 26.776 | 43.829 | 43.816 | 1.00 | 25.99 | A | N |
| ATOM | 1008 | CA | MET | A | 162 | 25.465 | 43.333 | 44.194 | 1.00 | 25.99 | A | C |
| ATOM | 1009 | CB | MET | A | 162 | 24.468 | 44.485 | 44.293 | 1.00 | 24.75 | A | C |
| ATOM | 1010 | CG | MET | A | 162 | 24.722 | 45.419 | 45.482 | 1.00 | 24.75 | A | C |
| ATOM | 1011 | SD | MET | A | 162 | 24.940 | 44.534 | 47.033 | 1.00 | 24.75 | A | S |
| ATOM | 1012 | CE | MET | A | 162 | 23.360 | 43.816 | 47.220 | 1.00 | 24.75 | A | C |
| ATOM | 1013 | C | MET | A | 162 | 24.945 | 42.254 | 43.266 | 1.00 | 25.99 | A | C |
| ATOM | 1014 | O | MET | A | 162 | 24.413 | 41.251 | 43.729 | 1.00 | 25.99 | A | O |
| ATOM | 1015 | N | TYR | A | 163 | 25.100 | 42.457 | 41.959 | 1.00 | 19.18 | A | N |
| ATOM | 1016 | CA | TYR | A | 163 | 24.643 | 41.491 | 40.969 | 1.00 | 19.18 | A | C |
| ATOM | 1017 | CB | TYR | A | 163 | 25.077 | 41.942 | 39.581 | 1.00 | 22.75 | A | C |
| ATOM | 1018 | CG | TYR | A | 163 | 24.716 | 40.977 | 38.481 | 1.00 | 22.75 | A | C |
| ATOM | 1019 | CD1 | TYR | A | 163 | 23.412 | 40.868 | 38.027 | 1.00 | 22.75 | A | C |
| ATOM | 1020 | CE1 | TYR | A | 163 | 23.074 | 39.948 | 37.049 | 1.00 | 22.75 | A | C |
| ATOM | 1021 | CD2 | TYR | A | 163 | 25.679 | 40.140 | 37.925 | 1.00 | 22.75 | A | C |
| ATOM | 1022 | CE2 | TYR | A | 163 | 25.353 | 39.211 | 36.941 | 1.00 | 22.75 | A | C |
| ATOM | 1023 | CZ | TYR | A | 163 | 24.050 | 39.117 | 36.501 | 1.00 | 22.75 | A | C |
| ATOM | 1024 | OH | TYR | A | 163 | 23.730 | 38.220 | 35.495 | 1.00 | 22.75 | A | O |
| ATOM | 1025 | C | TYR | A | 163 | 25.263 | 40.146 | 41.277 | 1.00 | 19.18 | A | C |
| ATOM | 1026 | O | TYR | A | 163 | 24.567 | 39.150 | 41.426 | 1.00 | 19.18 | A | O |
| ATOM | 1027 | N | GLN | A | 164 | 26.584 | 40.126 | 41.378 | 1.00 | 27.19 | A | N |
| ATOM | 1028 | CA | GLN | A | 164 | 27.301 | 38.900 | 41.670 | 1.00 | 27.19 | A | C |
| ATOM | 1029 | CB | GLN | A | 164 | 28.799 | 39.178 | 41.727 | 1.00 | 12.65 | A | C |
| ATOM | 1030 | CG | GLN | A | 164 | 29.324 | 39.725 | 40.420 | 1.00 | 12.65 | A | C |
| ATOM | 1031 | CD | GLN | A | 164 | 30.818 | 39.712 | 40.336 | 1.00 | 12.65 | A | C |
| ATOM | 1032 | OE1 | GLN | A | 164 | 31.429 | 38.667 | 40.454 | 1.00 | 12.65 | A | O |
| ATOM | 1033 | NE2 | GLN | A | 164 | 31.419 | 40.873 | 40.120 | 1.00 | 12.65 | A | N |
| ATOM | 1034 | C | GLN | A | 164 | 26.808 | 38.287 | 42.968 | 1.00 | 27.19 | A | C |
| ATOM | 1035 | O | GLN | A | 164 | 26.597 | 37.078 | 43.036 | 1.00 | 27.19 | A | O |
| ATOM | 1036 | N | LEU | A | 165 | 26.608 | 39.106 | 43.995 | 1.00 | 21.52 | A | N |
| ATOM | 1037 | CA | LEU | A | 165 | 26.116 | 38.569 | 45.258 | 1.00 | 21.52 | A | C |
| ATOM | 1038 | CB | LEU | A | 165 | 25.953 | 39.681 | 46.296 | 1.00 | 18.83 | A | C |
| ATOM | 1039 | CG | LEU | A | 165 | 25.103 | 39.371 | 47.538 | 1.00 | 18.83 | A | C |
| ATOM | 1040 | CD1 | LEU | A | 165 | 25.688 | 38.215 | 48.321 | 1.00 | 18.83 | A | C |
| ATOM | 1041 | CD2 | LEU | A | 165 | 25.025 | 40.594 | 48.406 | 1.00 | 18.83 | A | C |
| ATOM | 1042 | C | LEU | A | 165 | 24.791 | 37.843 | 45.062 | 1.00 | 21.52 | A | C |
| ATOM | 1043 | O | LEU | A | 165 | 24.587 | 36.757 | 45.602 | 1.00 | 21.52 | A | O |
| ATOM | 1044 | N | PHE | A | 166 | 23.891 | 38.437 | 44.285 | 1.00 | 16.76 | A | N |
| ATOM | 1045 | CA | PHE | A | 166 | 22.599 | 37.823 | 44.047 | 1.00 | 16.76 | A | C |
| ATOM | 1046 | CB | PHE | A | 166 | 21.701 | 38.813 | 43.307 | 1.00 | 14.70 | A | C |
| ATOM | 1047 | CG | PHE | A | 166 | 21.039 | 39.820 | 44.219 | 1.00 | 14.70 | A | C |
| ATOM | 1048 | CD1 | PHE | A | 166 | 20.300 | 39.401 | 45.326 | 1.00 | 14.70 | A | C |
| ATOM | 1049 | CD2 | PHE | A | 166 | 21.155 | 41.180 | 43.985 | 1.00 | 14.70 | A | C |
| ATOM | 1050 | CE1 | PHE | A | 166 | 19.698 | 40.324 | 46.181 | 1.00 | 14.70 | A | C |
| ATOM | 1051 | CE2 | PHE | A | 166 | 20.553 | 42.100 | 44.839 | 1.00 | 14.70 | A | C |
| ATOM | 1052 | CZ | PHE | A | 166 | 19.827 | 41.668 | 45.933 | 1.00 | 14.70 | A | C |
| ATOM | 1053 | C | PHE | A | 166 | 22.740 | 36.506 | 43.296 | 1.00 | 16.76 | A | C |
| ATOM | 1054 | O | PHE | A | 166 | 22.045 | 35.528 | 43.589 | 1.00 | 16.76 | A | O |
| ATOM | 1055 | N | ARG | A | 167 | 23.671 | 36.470 | 42.347 | 1.00 | 29.87 | A | N |
| ATOM | 1056 | CA | ARG | A | 167 | 23.892 | 35.259 | 41.579 | 1.00 | 29.87 | A | C |
| ATOM | 1057 | CB | ARG | A | 167 | 24.850 | 35.533 | 40.419 | 1.00 | 30.66 | A | C |
| ATOM | 1058 | CG | ARG | A | 167 | 24.719 | 34.523 | 39.273 | 1.00 | 30.66 | A | C |
| ATOM | 1059 | CD | ARG | A | 167 | 25.749 | 34.763 | 38.194 | 1.00 | 30.66 | A | C |
| ATOM | 1060 | NE | ARG | A | 167 | 25.993 | 33.551 | 37.424 | 1.00 | 30.66 | A | N |
| ATOM | 1061 | CZ | ARG | A | 167 | 27.015 | 33.398 | 36.592 | 1.00 | 30.66 | A | C |
| ATOM | 1062 | NH1 | ARG | A | 167 | 27.887 | 34.386 | 36.430 | 1.00 | 30.66 | A | N |
| ATOM | 1063 | NH2 | ARG | A | 167 | 27.162 | 32.264 | 35.923 | 1.00 | 30.66 | A | N |
| ATOM | 1064 | C | ARG | A | 167 | 24.417 | 34.134 | 42.466 | 1.00 | 29.87 | A | C |
| ATOM | 1065 | O | ARG | A | 167 | 24.118 | 32.966 | 42.224 | 1.00 | 29.87 | A | O |
| ATOM | 1066 | N | SER | A | 168 | 25.180 | 34.476 | 43.500 | 1.00 | 22.74 | A | N |

| | | | | | | | | | | | | |
|---|---|---|---|---|---|---|---|---|---|---|---|---|
| ATOM | 1067 | CA | SER | A | 168 | 25.702 | 33.450 | 44.387 | 1.00 | 22.74 | A | C |
| ATOM | 1068 | CB | SER | A | 168 | 26.860 | 33.986 | 45.224 | 1.00 | 27.05 | A | C |
| ATOM | 1069 | OG | SER | A | 168 | 26.424 | 34.955 | 46.152 | 1.00 | 27.05 | A | O |
| ATOM | 1070 | C | SER | A | 168 | 24.570 | 32.994 | 45.279 | 1.00 | 22.74 | A | C |
| ATOM | 1071 | O | SER | A | 168 | 24.472 | 31.819 | 45.607 | 1.00 | 22.74 | A | O |
| ATOM | 1072 | N | LEU | A | 169 | 23.697 | 33.927 | 45.644 | 1.00 | 28.51 | A | N |
| ATOM | 1073 | CA | LEU | A | 169 | 22.563 | 33.612 | 46.495 | 1.00 | 28.51 | A | C |
| ATOM | 1074 | CB | LEU | A | 169 | 21.855 | 34.895 | 46.928 | 1.00 | 18.89 | A | C |
| ATOM | 1075 | CG | LEU | A | 169 | 21.823 | 35.221 | 48.424 | 1.00 | 18.89 | A | C |
| ATOM | 1076 | CD1 | LEU | A | 169 | 23.212 | 35.260 | 48.981 | 1.00 | 18.89 | A | C |
| ATOM | 1077 | CD2 | LEU | A | 169 | 21.129 | 36.547 | 48.636 | 1.00 | 18.89 | A | C |
| ATOM | 1078 | C | LEU | A | 169 | 21.602 | 32.712 | 45.745 | 1.00 | 28.51 | A | C |
| ATOM | 1079 | O | LEU | A | 169 | 21.136 | 31.714 | 46.285 | 1.00 | 28.51 | A | O |
| ATOM | 1080 | N | ALA | A | 170 | 21.320 | 33.060 | 44.491 | 1.00 | 23.92 | A | N |
| ATOM | 1081 | CA | ALA | A | 170 | 20.412 | 32.273 | 43.662 | 1.00 | 23.92 | A | C |
| ATOM | 1082 | CB | ALA | A | 170 | 20.293 | 32.899 | 42.278 | 1.00 | 18.89 | A | C |
| ATOM | 1083 | C | ALA | A | 170 | 20.934 | 30.844 | 43.550 | 1.00 | 23.92 | A | C |
| ATOM | 1084 | O | ALA | A | 170 | 20.167 | 29.879 | 43.498 | 1.00 | 23.92 | A | O |
| ATOM | 1085 | N | TYR | A | 171 | 22.251 | 30.713 | 43.536 | 1.00 | 26.18 | A | N |
| ATOM | 1086 | CA | TYR | A | 171 | 22.866 | 29.406 | 43.413 | 1.00 | 26.18 | A | C |
| ATOM | 1087 | CB | TYR | A | 171 | 24.347 | 29.560 | 43.077 | 1.00 | 38.36 | A | C |
| ATOM | 1088 | CG | TYR | A | 171 | 25.082 | 28.246 | 42.974 | 1.00 | 38.36 | A | C |
| ATOM | 1089 | CD1 | TYR | A | 171 | 24.788 | 27.343 | 41.962 | 1.00 | 38.36 | A | C |
| ATOM | 1090 | CE1 | TYR | A | 171 | 25.470 | 26.143 | 41.853 | 1.00 | 38.36 | A | C |
| ATOM | 1091 | CD2 | TYR | A | 171 | 26.080 | 27.912 | 43.883 | 1.00 | 38.36 | A | C |
| ATOM | 1092 | CE2 | TYR | A | 171 | 26.772 | 26.709 | 43.783 | 1.00 | 38.36 | A | C |
| ATOM | 1093 | CZ | TYR | A | 171 | 26.466 | 25.829 | 42.764 | 1.00 | 38.36 | A | C |
| ATOM | 1094 | OH | TYR | A | 171 | 27.181 | 24.655 | 42.647 | 1.00 | 38.36 | A | O |
| ATOM | 1095 | C | TYR | A | 171 | 22.723 | 28.522 | 44.635 | 1.00 | 26.18 | A | C |
| ATOM | 1096 | O | TYR | A | 171 | 22.280 | 27.380 | 44.533 | 1.00 | 26.18 | A | O |
| ATOM | 1097 | N | ILE | A | 172 | 23.103 | 29.034 | 45.795 | 1.00 | 14.66 | A | N |
| ATOM | 1098 | CA | ILE | A | 172 | 23.005 | 28.215 | 46.977 | 1.00 | 14.66 | A | C |
| ATOM | 1099 | CB | ILE | A | 172 | 23.785 | 28.830 | 48.163 | 1.00 | 21.63 | A | C |
| ATOM | 1100 | CG2 | ILE | A | 172 | 25.255 | 28.963 | 47.794 | 1.00 | 21.63 | A | C |
| ATOM | 1101 | CG1 | ILE | A | 172 | 23.199 | 30.186 | 48.544 | 1.00 | 21.63 | A | C |
| ATOM | 1102 | CD1 | ILE | A | 172 | 23.570 | 30.625 | 49.929 | 1.00 | 21.63 | A | C |
| ATOM | 1103 | C | ILE | A | 172 | 21.549 | 28.000 | 47.333 | 1.00 | 14.66 | A | C |
| ATOM | 1104 | O | ILE | A | 172 | 21.174 | 26.920 | 47.772 | 1.00 | 14.66 | A | O |
| ATOM | 1105 | N | HIS | A | 173 | 20.718 | 29.011 | 47.122 | 1.00 | 21.18 | A | N |
| ATOM | 1106 | CA | HIS | A | 173 | 19.306 | 28.870 | 47.447 | 1.00 | 21.18 | A | C |
| ATOM | 1107 | CB | HIS | A | 173 | 18.579 | 30.199 | 47.228 | 1.00 | 24.09 | A | C |
| ATOM | 1108 | CG | HIS | A | 173 | 18.817 | 31.199 | 48.318 | 1.00 | 24.09 | A | C |
| ATOM | 1109 | CD2 | HIS | A | 173 | 19.618 | 31.152 | 49.408 | 1.00 | 24.09 | A | C |
| ATOM | 1110 | ND1 | HIS | A | 173 | 18.197 | 32.428 | 48.355 | 1.00 | 24.09 | A | N |
| ATOM | 1111 | CE1 | HIS | A | 173 | 18.607 | 33.093 | 49.421 | 1.00 | 24.09 | A | C |
| ATOM | 1112 | NE2 | HIS | A | 173 | 19.470 | 32.341 | 50.076 | 1.00 | 24.09 | A | N |
| ATOM | 1113 | C | HIS | A | 173 | 18.691 | 27.764 | 46.600 | 1.00 | 21.18 | A | C |
| ATOM | 1114 | O | HIS | A | 173 | 17.722 | 27.115 | 46.988 | 1.00 | 21.18 | A | O |
| ATOM | 1115 | N | SER | A | 174 | 19.289 | 27.558 | 45.436 | 1.00 | 36.15 | A | N |
| ATOM | 1116 | CA | SER | A | 174 | 18.851 | 26.550 | 44.493 | 1.00 | 36.15 | A | C |
| ATOM | 1117 | CB | SER | A | 174 | 19.824 | 26.546 | 43.321 | 1.00 | 53.54 | A | C |
| ATOM | 1118 | OG | SER | A | 174 | 19.552 | 25.503 | 42.413 | 1.00 | 53.54 | A | O |
| ATOM | 1119 | C | SER | A | 174 | 18.854 | 25.198 | 45.184 | 1.00 | 36.15 | A | C |
| ATOM | 1120 | O | SER | A | 174 | 17.920 | 24.427 | 45.035 | 1.00 | 36.15 | A | O |
| ATOM | 1121 | N | PHE | A | 175 | 19.915 | 24.912 | 45.928 | 1.00 | 30.64 | A | N |
| ATOM | 1122 | CA | PHE | A | 175 | 20.029 | 23.653 | 46.657 | 1.00 | 30.64 | A | C |
| ATOM | 1123 | CB | PHE | A | 175 | 21.495 | 23.366 | 46.992 | 1.00 | 43.25 | A | C |
| ATOM | 1124 | CG | PHE | A | 175 | 22.379 | 23.086 | 45.805 | 1.00 | 43.25 | A | C |
| ATOM | 1125 | CD1 | PHE | A | 175 | 22.607 | 21.785 | 45.379 | 1.00 | 43.25 | A | C |
| ATOM | 1126 | CD2 | PHE | A | 175 | 23.076 | 24.114 | 45.182 | 1.00 | 43.25 | A | C |
| ATOM | 1127 | CE1 | PHE | A | 175 | 23.526 | 21.512 | 44.360 | 1.00 | 43.25 | A | C |
| ATOM | 1128 | CE2 | PHE | A | 175 | 24.001 | 23.846 | 44.155 | 1.00 | 43.25 | A | C |
| ATOM | 1129 | CZ | PHE | A | 175 | 24.225 | 22.547 | 43.750 | 1.00 | 43.25 | A | C |
| ATOM | 1130 | C | PHE | A | 175 | 19.267 | 23.730 | 47.985 | 1.00 | 30.64 | A | C |
| ATOM | 1131 | O | PHE | A | 175 | 19.416 | 22.856 | 48.841 | 1.00 | 30.64 | A | O |
| ATOM | 1132 | N | GLY | A | 176 | 18.480 | 24.789 | 48.166 | 1.00 | 38.74 | A | N |
| ATOM | 1133 | CA | GLY | A | 176 | 17.717 | 24.955 | 49.395 | 1.00 | 38.74 | A | C |

| | | | | | | | | | | | | |
|---|---|---|---|---|---|---|---|---|---|---|---|---|
| ATOM | 1134 | C | GLY | A | 176 | 18.543 | 25.412 | 50.580 | 1.00 | 38.74 | A | C |
| ATOM | 1135 | O | GLY | A | 176 | 18.042 | 25.479 | 51.702 | 1.00 | 38.74 | A | O |
| ATOM | 1136 | N | ILE | A | 177 | 19.809 | 25.731 | 50.321 | 1.00 | 32.10 | A | N |
| ATOM | 1137 | CA | ILE | A | 177 | 20.744 | 26.195 | 51.340 | 1.00 | 32.10 | A | C |
| ATOM | 1138 | CB | ILE | A | 177 | 22.180 | 25.839 | 50.921 | 1.00 | 40.60 | A | C |
| ATOM | 1139 | CG2 | ILE | A | 177 | 23.184 | 26.352 | 51.953 | 1.00 | 40.60 | A | C |
| ATOM | 1140 | CG1 | ILE | A | 177 | 22.292 | 24.325 | 50.743 | 1.00 | 40.60 | A | C |
| ATOM | 1141 | CD1 | ILE | A | 177 | 23.698 | 23.847 | 50.351 | 1.00 | 40.60 | A | C |
| ATOM | 1142 | C | ILE | A | 177 | 20.656 | 27.706 | 51.564 | 1.00 | 32.10 | A | C |
| ATOM | 1143 | O | ILE | A | 177 | 20.510 | 28.479 | 50.611 | 1.00 | 32.10 | A | O |
| ATOM | 1144 | N | CYS | A | 178 | 20.762 | 28.108 | 52.833 | 1.00 | 31.99 | A | N |
| ATOM | 1145 | CA | CYS | A | 178 | 20.688 | 29.511 | 53.243 | 1.00 | 31.99 | A | C |
| ATOM | 1146 | CB | CYS | A | 178 | 19.534 | 29.696 | 54.225 | 1.00 | 35.38 | A | C |
| ATOM | 1147 | SG | CYS | A | 178 | 19.131 | 31.389 | 54.607 | 1.00 | 35.38 | A | S |
| ATOM | 1148 | C | CYS | A | 178 | 21.997 | 29.877 | 53.909 | 1.00 | 31.99 | A | C |
| ATOM | 1149 | O | CYS | A | 178 | 22.462 | 29.163 | 54.788 | 1.00 | 31.99 | A | O |
| ATOM | 1150 | N | HIS | A | 179 | 22.606 | 30.975 | 53.484 | 1.00 | 29.87 | A | N |
| ATOM | 1151 | CA | HIS | A | 179 | 23.880 | 31.380 | 54.062 | 1.00 | 29.87 | A | C |
| ATOM | 1152 | CB | HIS | A | 179 | 24.518 | 32.488 | 53.215 | 1.00 | 20.76 | A | C |
| ATOM | 1153 | CG | HIS | A | 179 | 25.925 | 32.822 | 53.613 | 1.00 | 20.76 | A | C |
| ATOM | 1154 | CD2 | HIS | A | 179 | 27.110 | 32.525 | 53.030 | 1.00 | 20.76 | A | C |
| ATOM | 1155 | ND1 | HIS | A | 179 | 26.230 | 33.511 | 54.766 | 1.00 | 20.76 | A | N |
| ATOM | 1156 | CE1 | HIS | A | 179 | 27.541 | 33.622 | 54.878 | 1.00 | 20.76 | A | C |
| ATOM | 1157 | NE2 | HIS | A | 179 | 28.098 | 33.030 | 53.838 | 1.00 | 20.76 | A | N |
| ATOM | 1158 | C | HIS | A | 179 | 23.710 | 31.835 | 55.504 | 1.00 | 29.87 | A | C |
| ATOM | 1159 | O | HIS | A | 179 | 24.555 | 31.569 | 56.349 | 1.00 | 29.87 | A | O |
| ATOM | 1160 | N | ARG | A | 180 | 22.612 | 32.524 | 55.773 | 1.00 | 36.47 | A | N |
| ATOM | 1161 | CA | ARG | A | 180 | 22.313 | 33.005 | 57.110 | 1.00 | 36.47 | A | C |
| ATOM | 1162 | CB | ARG | A | 180 | 22.321 | 31.842 | 58.085 | 1.00 | 20.19 | A | C |
| ATOM | 1163 | CG | ARG | A | 180 | 21.094 | 30.980 | 58.119 | 1.00 | 20.19 | A | C |
| ATOM | 1164 | CD | ARG | A | 180 | 21.499 | 29.801 | 58.955 | 1.00 | 20.19 | A | C |
| ATOM | 1165 | NE | ARG | A | 180 | 20.436 | 29.175 | 59.718 | 1.00 | 20.19 | A | N |
| ATOM | 1166 | CZ | ARG | A | 180 | 20.683 | 28.273 | 60.654 | 1.00 | 20.19 | A | C |
| ATOM | 1167 | NH1 | ARG | A | 180 | 21.942 | 27.937 | 60.900 | 1.00 | 20.19 | A | N |
| ATOM | 1168 | NH2 | ARG | A | 180 | 19.695 | 27.715 | 61.342 | 1.00 | 20.19 | A | N |
| ATOM | 1169 | C | ARG | A | 180 | 23.203 | 34.109 | 57.665 | 1.00 | 36.47 | A | C |
| ATOM | 1170 | O | ARG | A | 180 | 22.906 | 34.638 | 58.739 | 1.00 | 36.47 | A | O |
| ATOM | 1171 | N | ASP | A | 181 | 24.281 | 34.465 | 56.973 | 1.00 | 25.40 | A | N |
| ATOM | 1172 | CA | ASP | A | 181 | 25.131 | 35.534 | 57.484 | 1.00 | 25.40 | A | C |
| ATOM | 1173 | CB | ASP | A | 181 | 26.238 | 34.961 | 58.354 | 1.00 | 35.53 | A | C |
| ATOM | 1174 | CG | ASP | A | 181 | 26.911 | 36.023 | 59.182 | 1.00 | 35.53 | A | C |
| ATOM | 1175 | OD1 | ASP | A | 181 | 26.267 | 37.073 | 59.394 | 1.00 | 35.53 | A | O |
| ATOM | 1176 | OD2 | ASP | A | 181 | 28.059 | 35.814 | 59.630 | 1.00 | 35.53 | A | O |
| ATOM | 1177 | C | ASP | A | 181 | 25.724 | 36.419 | 56.403 | 1.00 | 25.40 | A | C |
| ATOM | 1178 | O | ASP | A | 181 | 26.934 | 36.579 | 56.291 | 1.00 | 25.40 | A | O |
| ATOM | 1179 | N | ILE | A | 182 | 24.851 | 37.009 | 55.614 | 1.00 | 13.99 | A | N |
| ATOM | 1180 | CA | ILE | A | 182 | 25.291 | 37.872 | 54.549 | 1.00 | 13.99 | A | C |
| ATOM | 1181 | CB | ILE | A | 182 | 24.181 | 38.005 | 53.465 | 1.00 | 18.72 | A | C |
| ATOM | 1182 | CG2 | ILE | A | 182 | 24.629 | 38.890 | 52.328 | 1.00 | 18.72 | A | C |
| ATOM | 1183 | CG1 | ILE | A | 182 | 23.796 | 36.625 | 52.937 | 1.00 | 18.72 | A | C |
| ATOM | 1184 | CD1 | ILE | A | 182 | 24.882 | 35.915 | 52.248 | 1.00 | 18.72 | A | C |
| ATOM | 1185 | C | ILE | A | 182 | 25.612 | 39.240 | 55.127 | 1.00 | 13.99 | A | C |
| ATOM | 1186 | O | ILE | A | 182 | 24.750 | 39.926 | 55.662 | 1.00 | 13.99 | A | O |
| ATOM | 1187 | N | LYS | A | 183 | 26.872 | 39.619 | 55.027 | 1.00 | 17.89 | A | N |
| ATOM | 1188 | CA | LYS | A | 183 | 27.315 | 40.918 | 55.477 | 1.00 | 17.89 | A | C |
| ATOM | 1189 | CB | LYS | A | 183 | 27.608 | 40.915 | 56.973 | 1.00 | 21.96 | A | C |
| ATOM | 1190 | CG | LYS | A | 183 | 28.818 | 40.107 | 57.402 | 1.00 | 21.96 | A | C |
| ATOM | 1191 | CD | LYS | A | 183 | 29.061 | 40.316 | 58.882 | 1.00 | 21.96 | A | C |
| ATOM | 1192 | CE | LYS | A | 183 | 30.106 | 39.376 | 59.444 | 1.00 | 21.96 | A | C |
| ATOM | 1193 | NZ | LYS | A | 183 | 29.630 | 37.972 | 59.469 | 1.00 | 21.96 | A | N |
| ATOM | 1194 | C | LYS | A | 183 | 28.573 | 41.228 | 54.680 | 1.00 | 17.89 | A | C |
| ATOM | 1195 | O | LYS | A | 183 | 29.283 | 40.327 | 54.252 | 1.00 | 17.89 | A | O |
| ATOM | 1196 | N | PRO | A | 184 | 28.860 | 42.512 | 54.464 | 1.00 | 31.37 | A | N |
| ATOM | 1197 | CD | PRO | A | 184 | 28.091 | 43.655 | 54.981 | 1.00 | 32.42 | A | C |
| ATOM | 1198 | CA | PRO | A | 184 | 30.032 | 42.965 | 53.716 | 1.00 | 31.37 | A | C |
| ATOM | 1199 | CB | PRO | A | 184 | 30.135 | 44.421 | 54.118 | 1.00 | 32.42 | A | C |
| ATOM | 1200 | CG | PRO | A | 184 | 28.686 | 44.805 | 54.223 | 1.00 | 32.42 | A | C |

| | | | | | | | | | | | | |
|---|---|---|---|---|---|---|---|---|---|---|---|---|
| ATOM | 1201 | C | PRO | A | 184 | 31.316 | 42.181 | 53.989 | 1.00 | 31.37 | A | C |
| ATOM | 1202 | O | PRO | A | 184 | 32.044 | 41.841 | 53.057 | 1.00 | 31.37 | A | O |
| ATOM | 1203 | N | GLN | A | 185 | 31.592 | 41.889 | 55.256 | 1.00 | 19.40 | A | N |
| ATOM | 1204 | CA | GLN | A | 185 | 32.801 | 41.152 | 55.604 | 1.00 | 19.40 | A | C |
| ATOM | 1205 | CB | GLN | A | 185 | 32.901 | 40.971 | 57.127 | 1.00 | 41.36 | A | C |
| ATOM | 1206 | CG | GLN | A | 185 | 33.123 | 42.250 | 57.949 | 1.00 | 41.36 | A | C |
| ATOM | 1207 | CD | GLN | A | 185 | 31.918 | 42.592 | 58.859 | 1.00 | 41.36 | A | C |
| ATOM | 1208 | OE1 | GLN | A | 185 | 30.844 | 43.025 | 58.383 | 1.00 | 41.36 | A | O |
| ATOM | 1209 | NE2 | GLN | A | 185 | 32.094 | 42.382 | 60.174 | 1.00 | 41.36 | A | N |
| ATOM | 1210 | C | GLN | A | 185 | 32.899 | 39.782 | 54.932 | 1.00 | 19.40 | A | C |
| ATOM | 1211 | O | GLN | A | 185 | 33.984 | 39.228 | 54.819 | 1.00 | 19.40 | A | O |
| ATOM | 1212 | N | ASN | A | 186 | 31.770 | 39.244 | 54.487 | 1.00 | 20.13 | A | N |
| ATOM | 1213 | CA | ASN | A | 186 | 31.750 | 37.934 | 53.856 | 1.00 | 20.13 | A | C |
| ATOM | 1214 | CB | ASN | A | 186 | 30.545 | 37.118 | 54.323 | 1.00 | 23.76 | A | C |
| ATOM | 1215 | CG | ASN | A | 186 | 30.649 | 36.709 | 55.792 | 1.00 | 23.76 | A | C |
| ATOM | 1216 | OD1 | ASN | A | 186 | 31.687 | 36.208 | 56.227 | 1.00 | 23.76 | A | O |
| ATOM | 1217 | ND2 | ASN | A | 186 | 29.574 | 36.918 | 56.555 | 1.00 | 23.76 | A | N |
| ATOM | 1218 | C | ASN | A | 186 | 31.731 | 38.017 | 52.369 | 1.00 | 20.13 | A | C |
| ATOM | 1219 | O | ASN | A | 186 | 31.571 | 37.004 | 51.714 | 1.00 | 20.13 | A | O |
| ATOM | 1220 | N | LEU | A | 187 | 31.889 | 39.222 | 51.837 | 1.00 | 17.81 | A | N |
| ATOM | 1221 | CA | LEU | A | 187 | 31.898 | 39.431 | 50.399 | 1.00 | 17.81 | A | C |
| ATOM | 1222 | CB | LEU | A | 187 | 30.954 | 40.570 | 50.027 | 1.00 | 13.56 | A | C |
| ATOM | 1223 | CG | LEU | A | 187 | 29.513 | 40.311 | 50.442 | 1.00 | 13.56 | A | C |
| ATOM | 1224 | CD1 | LEU | A | 187 | 28.644 | 41.444 | 49.995 | 1.00 | 13.56 | A | C |
| ATOM | 1225 | CD2 | LEU | A | 187 | 29.055 | 39.002 | 49.864 | 1.00 | 13.56 | A | C |
| ATOM | 1226 | C | LEU | A | 187 | 33.313 | 39.747 | 49.947 | 1.00 | 17.81 | A | C |
| ATOM | 1227 | O | LEU | A | 187 | 33.734 | 40.900 | 49.945 | 1.00 | 17.81 | A | O |
| ATOM | 1228 | N | LEU | A | 188 | 34.053 | 38.706 | 49.588 | 1.00 | 27.82 | A | N |
| ATOM | 1229 | CA | LEU | A | 188 | 35.423 | 38.865 | 49.123 | 1.00 | 27.82 | A | C |
| ATOM | 1230 | CB | LEU | A | 188 | 36.181 | 37.548 | 49.202 | 1.00 | 26.49 | A | C |
| ATOM | 1231 | CG | LEU | A | 188 | 36.049 | 36.763 | 50.505 | 1.00 | 26.49 | A | C |
| ATOM | 1232 | CD1 | LEU | A | 188 | 36.878 | 35.511 | 50.366 | 1.00 | 26.49 | A | C |
| ATOM | 1233 | CD2 | LEU | A | 188 | 36.505 | 37.584 | 51.707 | 1.00 | 26.49 | A | C |
| ATOM | 1234 | C | LEU | A | 188 | 35.413 | 39.302 | 47.680 | 1.00 | 27.82 | A | C |
| ATOM | 1235 | O | LEU | A | 188 | 34.557 | 38.883 | 46.891 | 1.00 | 27.82 | A | O |
| ATOM | 1236 | N | LEU | A | 189 | 36.378 | 40.131 | 47.319 | 1.00 | 26.97 | A | N |
| ATOM | 1237 | CA | LEU | A | 189 | 36.438 | 40.571 | 45.948 | 1.00 | 26.97 | A | C |
| ATOM | 1238 | CB | LEU | A | 189 | 35.502 | 41.764 | 45.734 | 1.00 | 43.18 | A | C |
| ATOM | 1239 | CG | LEU | A | 189 | 35.867 | 43.147 | 46.266 | 1.00 | 43.18 | A | C |
| ATOM | 1240 | CD1 | LEU | A | 189 | 34.581 | 43.940 | 46.408 | 1.00 | 43.18 | A | C |
| ATOM | 1241 | CD2 | LEU | A | 189 | 36.575 | 43.052 | 47.607 | 1.00 | 43.18 | A | C |
| ATOM | 1242 | C | LEU | A | 189 | 37.840 | 40.892 | 45.498 | 1.00 | 26.97 | A | C |
| ATOM | 1243 | O | LEU | A | 189 | 38.639 | 41.454 | 46.235 | 1.00 | 26.97 | A | O |
| ATOM | 1244 | N | ASP | A | 190 | 38.133 | 40.470 | 44.280 | 1.00 | 33.93 | A | N |
| ATOM | 1245 | CA | ASP | A | 190 | 39.412 | 40.699 | 43.648 | 1.00 | 33.93 | A | C |
| ATOM | 1246 | CB | ASP | A | 190 | 39.597 | 39.647 | 42.557 | 1.00 | 47.01 | A | C |
| ATOM | 1247 | CG | ASP | A | 190 | 40.731 | 39.970 | 41.607 | 1.00 | 47.01 | A | C |
| ATOM | 1248 | OD1 | ASP | A | 190 | 41.170 | 41.152 | 41.577 | 1.00 | 47.01 | A | O |
| ATOM | 1249 | OD2 | ASP | A | 190 | 41.159 | 39.037 | 40.880 | 1.00 | 47.01 | A | O |
| ATOM | 1250 | C | ASP | A | 190 | 39.315 | 42.105 | 43.062 | 1.00 | 33.93 | A | C |
| ATOM | 1251 | O | ASP | A | 190 | 38.405 | 42.417 | 42.302 | 1.00 | 33.93 | A | O |
| ATOM | 1252 | N | PRO | A | 191 | 40.245 | 42.981 | 43.428 | 1.00 | 47.44 | A | N |
| ATOM | 1253 | CD | PRO | A | 191 | 41.327 | 42.710 | 44.391 | 1.00 | 42.50 | A | C |
| ATOM | 1254 | CA | PRO | A | 191 | 40.285 | 44.373 | 42.953 | 1.00 | 47.44 | A | C |
| ATOM | 1255 | CB | PRO | A | 191 | 41.392 | 44.988 | 43.806 | 1.00 | 42.50 | A | C |
| ATOM | 1256 | CG | PRO | A | 191 | 42.297 | 43.818 | 44.082 | 1.00 | 42.50 | A | C |
| ATOM | 1257 | C | PRO | A | 191 | 40.519 | 44.577 | 41.458 | 1.00 | 47.44 | A | C |
| ATOM | 1258 | O | PRO | A | 191 | 39.986 | 45.524 | 40.860 | 1.00 | 47.44 | A | O |
| ATOM | 1259 | N | ASP | A | 192 | 41.312 | 43.703 | 40.849 | 1.00 | 55.08 | A | N |
| ATOM | 1260 | CA | ASP | A | 192 | 41.564 | 43.845 | 39.429 | 1.00 | 55.08 | A | C |
| ATOM | 1261 | CB | ASP | A | 192 | 42.885 | 43.166 | 39.066 | 1.00 | 72.16 | A | C |
| ATOM | 1262 | CG | ASP | A | 192 | 44.079 | 43.839 | 39.742 | 1.00 | 72.16 | A | C |
| ATOM | 1263 | OD1 | ASP | A | 192 | 44.024 | 45.090 | 39.892 | 1.00 | 72.16 | A | O |
| ATOM | 1264 | OD2 | ASP | A | 192 | 45.055 | 43.127 | 40.108 | 1.00 | 72.16 | A | O |
| ATOM | 1265 | C | ASP | A | 192 | 40.413 | 43.336 | 38.564 | 1.00 | 55.08 | A | C |
| ATOM | 1266 | O | ASP | A | 192 | 39.873 | 44.084 | 37.748 | 1.00 | 55.08 | A | O |
| ATOM | 1267 | N | THR | A | 193 | 40.023 | 42.081 | 38.764 | 1.00 | 36.37 | A | N |

| | | | | | | | | | | | | |
|---|---|---|---|---|---|---|---|---|---|---|---|---|
| ATOM | 1268 | CA | THR | A | 193 | 38.958 | 41.474 | 37.983 | 1.00 | 36.37 | A | C |
| ATOM | 1269 | CB | THR | A | 193 | 38.959 | 39.953 | 38.165 | 1.00 | 33.81 | A | C |
| ATOM | 1270 | OG1 | THR | A | 193 | 38.771 | 39.647 | 39.549 | 1.00 | 33.81 | A | O |
| ATOM | 1271 | CG2 | THR | A | 193 | 40.281 | 39.368 | 37.724 | 1.00 | 33.81 | A | C |
| ATOM | 1272 | C | THR | A | 193 | 37.585 | 41.999 | 38.347 | 1.00 | 36.37 | A | C |
| ATOM | 1273 | O | THR | A | 193 | 36.695 | 42.001 | 37.509 | 1.00 | 36.37 | A | O |
| ATOM | 1274 | N | ALA | A | 194 | 37.413 | 42.437 | 39.592 | 1.00 | 29.10 | A | N |
| ATOM | 1275 | CA | ALA | A | 194 | 36.128 | 42.955 | 40.068 | 1.00 | 29.10 | A | C |
| ATOM | 1276 | CB | ALA | A | 194 | 35.525 | 43.917 | 39.052 | 1.00 | 21.98 | A | C |
| ATOM | 1277 | C | ALA | A | 194 | 35.133 | 41.837 | 40.364 | 1.00 | 29.10 | A | C |
| ATOM | 1278 | O | ALA | A | 194 | 33.940 | 42.095 | 40.538 | 1.00 | 29.10 | A | O |
| ATOM | 1279 | N | VAL | A | 195 | 35.604 | 40.594 | 40.423 | 1.00 | 43.02 | A | N |
| ATOM | 1280 | CA | VAL | A | 195 | 34.686 | 39.494 | 40.702 | 1.00 | 43.02 | A | C |
| ATOM | 1281 | CB | VAL | A | 195 | 35.109 | 38.136 | 39.996 | 1.00 | 25.11 | A | C |
| ATOM | 1282 | CG1 | VAL | A | 195 | 36.335 | 38.336 | 39.156 | 1.00 | 25.11 | A | C |
| ATOM | 1283 | CG2 | VAL | A | 195 | 35.300 | 37.013 | 41.019 | 1.00 | 25.11 | A | C |
| ATOM | 1284 | C | VAL | A | 195 | 34.498 | 39.284 | 42.194 | 1.00 | 43.02 | A | C |
| ATOM | 1285 | O | VAL | A | 195 | 35.461 | 39.185 | 42.941 | 1.00 | 43.02 | A | O |
| ATOM | 1286 | N | LEU | A | 196 | 33.241 | 39.224 | 42.618 | 1.00 | 30.70 | A | N |
| ATOM | 1287 | CA | LEU | A | 196 | 32.917 | 39.028 | 44.018 | 1.00 | 30.70 | A | C |
| ATOM | 1288 | CB | LEU | A | 196 | 31.612 | 39.753 | 44.358 | 1.00 | 13.71 | A | C |
| ATOM | 1289 | CG | LEU | A | 196 | 31.145 | 39.705 | 45.816 | 1.00 | 13.71 | A | C |
| ATOM | 1290 | CD1 | LEU | A | 196 | 30.257 | 40.875 | 46.110 | 1.00 | 13.71 | A | C |
| ATOM | 1291 | CD2 | LEU | A | 196 | 30.429 | 38.423 | 46.102 | 1.00 | 13.71 | A | C |
| ATOM | 1292 | C | LEU | A | 196 | 32.793 | 37.541 | 44.306 | 1.00 | 30.70 | A | C |
| ATOM | 1293 | O | LEU | A | 196 | 32.419 | 36.761 | 43.432 | 1.00 | 30.70 | A | O |
| ATOM | 1294 | N | LYS | A | 197 | 33.110 | 37.149 | 45.535 | 1.00 | 30.89 | A | N |
| ATOM | 1295 | CA | LYS | A | 197 | 33.024 | 35.751 | 45.920 | 1.00 | 30.89 | A | C |
| ATOM | 1296 | CB | LYS | A | 197 | 34.387 | 35.074 | 45.776 | 1.00 | 31.44 | A | C |
| ATOM | 1297 | CG | LYS | A | 197 | 34.771 | 34.688 | 44.363 | 1.00 | 31.44 | A | C |
| ATOM | 1298 | CD | LYS | A | 197 | 36.117 | 34.008 | 44.369 | 1.00 | 31.44 | A | C |
| ATOM | 1299 | CE | LYS | A | 197 | 36.499 | 33.537 | 42.978 | 1.00 | 31.44 | A | C |
| ATOM | 1300 | NZ | LYS | A | 197 | 35.583 | 32.473 | 42.464 | 1.00 | 31.44 | A | N |
| ATOM | 1301 | C | LYS | A | 197 | 32.544 | 35.587 | 47.348 | 1.00 | 30.89 | A | C |
| ATOM | 1302 | O | LYS | A | 197 | 33.234 | 35.978 | 48.273 | 1.00 | 30.89 | A | O |
| ATOM | 1303 | N | LEU | A | 198 | 31.380 | 34.979 | 47.525 | 1.00 | 23.21 | A | N |
| ATOM | 1304 | CA | LEU | A | 198 | 30.812 | 34.776 | 48.851 | 1.00 | 23.21 | A | C |
| ATOM | 1305 | CB | LEU | A | 198 | 29.400 | 34.217 | 48.736 | 1.00 | 24.78 | A | C |
| ATOM | 1306 | CG | LEU | A | 198 | 28.353 | 34.690 | 49.745 | 1.00 | 24.78 | A | C |
| ATOM | 1307 | CD1 | LEU | A | 198 | 27.231 | 33.663 | 49.819 | 1.00 | 24.78 | A | C |
| ATOM | 1308 | CD2 | LEU | A | 198 | 28.974 | 34.885 | 51.103 | 1.00 | 24.78 | A | C |
| ATOM | 1309 | C | LEU | A | 198 | 31.640 | 33.796 | 49.651 | 1.00 | 23.21 | A | C |
| ATOM | 1310 | O | LEU | A | 198 | 32.118 | 32.811 | 49.111 | 1.00 | 23.21 | A | O |
| ATOM | 1311 | N | CYS | A | 199 | 31.803 | 34.053 | 50.940 | 1.00 | 18.42 | A | N |
| ATOM | 1312 | CA | CYS | A | 199 | 32.569 | 33.140 | 51.776 | 1.00 | 18.42 | A | C |
| ATOM | 1313 | CB | CYS | A | 199 | 34.018 | 33.614 | 51.919 | 1.00 | 22.44 | A | C |
| ATOM | 1314 | SG | CYS | A | 199 | 34.285 | 35.202 | 52.689 | 1.00 | 22.44 | A | S |
| ATOM | 1315 | C | CYS | A | 199 | 31.944 | 33.018 | 53.148 | 1.00 | 18.42 | A | C |
| ATOM | 1316 | O | CYS | A | 199 | 30.954 | 33.676 | 53.433 | 1.00 | 18.42 | A | O |
| ATOM | 1317 | N | ASP | A | 200 | 32.519 | 32.152 | 53.980 | 1.00 | 34.83 | A | N |
| ATOM | 1318 | CA | ASP | A | 200 | 32.057 | 31.936 | 55.350 | 1.00 | 34.83 | A | C |
| ATOM | 1319 | CB | ASP | A | 200 | 31.980 | 33.266 | 56.082 | 1.00 | 37.26 | A | C |
| ATOM | 1320 | CG | ASP | A | 200 | 32.197 | 33.118 | 57.555 | 1.00 | 37.26 | A | C |
| ATOM | 1321 | OD1 | ASP | A | 200 | 31.851 | 32.027 | 58.069 | 1.00 | 37.26 | A | O |
| ATOM | 1322 | OD2 | ASP | A | 200 | 32.702 | 34.088 | 58.176 | 1.00 | 37.26 | A | O |
| ATOM | 1323 | C | ASP | A | 200 | 30.708 | 31.254 | 55.465 | 1.00 | 34.83 | A | C |
| ATOM | 1324 | O | ASP | A | 200 | 29.732 | 31.883 | 55.866 | 1.00 | 34.83 | A | O |
| ATOM | 1325 | N | PHE | A | 201 | 30.647 | 29.972 | 55.131 | 1.00 | 31.27 | A | N |
| ATOM | 1326 | CA | PHE | A | 201 | 29.386 | 29.251 | 55.214 | 1.00 | 31.27 | A | C |
| ATOM | 1327 | CB | PHE | A | 201 | 29.253 | 28.277 | 54.040 | 1.00 | 21.48 | A | C |
| ATOM | 1328 | CG | PHE | A | 201 | 29.143 | 28.960 | 52.708 | 1.00 | 21.48 | A | C |
| ATOM | 1329 | CD1 | PHE | A | 201 | 30.245 | 29.580 | 52.134 | 1.00 | 21.48 | A | C |
| ATOM | 1330 | CD2 | PHE | A | 201 | 27.932 | 29.011 | 52.037 | 1.00 | 21.48 | A | C |
| ATOM | 1331 | CE1 | PHE | A | 201 | 30.140 | 30.246 | 50.900 | 1.00 | 21.48 | A | C |
| ATOM | 1332 | CE2 | PHE | A | 201 | 27.819 | 29.674 | 50.806 | 1.00 | 21.48 | A | C |
| ATOM | 1333 | CZ | PHE | A | 201 | 28.926 | 30.291 | 50.240 | 1.00 | 21.48 | A | C |
| ATOM | 1334 | C | PHE | A | 201 | 29.292 | 28.518 | 56.542 | 1.00 | 31.27 | A | C |

| | | | | | | | | | | | | |
|---|---|---|---|---|---|---|---|---|---|---|---|---|
| ATOM | 1335 | O | PHE | A | 201 | 28.580 | 27.522 | 56.673 | 1.00 | 31.27 | A | O |
| ATOM | 1336 | N | GLY | A | 202 | 30.011 | 29.047 | 57.527 | 1.00 | 24.98 | A | N |
| ATOM | 1337 | CA | GLY | A | 202 | 30.021 | 28.470 | 58.855 | 1.00 | 24.98 | A | C |
| ATOM | 1338 | C | GLY | A | 202 | 28.655 | 28.488 | 59.502 | 1.00 | 24.98 | A | C |
| ATOM | 1339 | O | GLY | A | 202 | 28.422 | 27.773 | 60.471 | 1.00 | 24.98 | A | O |
| ATOM | 1340 | N | SER | A | 203 | 27.749 | 29.299 | 58.969 | 1.00 | 25.88 | A | N |
| ATOM | 1341 | CA | SER | A | 203 | 26.398 | 29.391 | 59.502 | 1.00 | 25.88 | A | C |
| ATOM | 1342 | CB | SER | A | 203 | 26.079 | 30.842 | 59.839 | 1.00 | 34.02 | A | C |
| ATOM | 1343 | OG | SER | A | 203 | 26.962 | 31.317 | 60.839 | 1.00 | 34.02 | A | O |
| ATOM | 1344 | C | SER | A | 203 | 25.375 | 28.842 | 58.515 | 1.00 | 25.88 | A | C |
| ATOM | 1345 | O | SER | A | 203 | 24.217 | 28.639 | 58.856 | 1.00 | 25.88 | A | O |
| ATOM | 1346 | N | ALA | A | 204 | 25.811 | 28.594 | 57.286 | 1.00 | 23.03 | A | N |
| ATOM | 1347 | CA | ALA | A | 204 | 24.922 | 28.077 | 56.263 | 1.00 | 23.03 | A | C |
| ATOM | 1348 | CB | ALA | A | 204 | 25.681 | 27.861 | 54.971 | 1.00 | 9.35 | A | C |
| ATOM | 1349 | C | ALA | A | 204 | 24.288 | 26.777 | 56.701 | 1.00 | 23.03 | A | C |
| ATOM | 1350 | O | ALA | A | 204 | 24.912 | 25.948 | 57.346 | 1.00 | 23.03 | A | O |
| ATOM | 1351 | N | LYS | A | 205 | 23.035 | 26.611 | 56.328 | 1.00 | 25.13 | A | N |
| ATOM | 1352 | CA | LYS | A | 205 | 22.295 | 25.421 | 56.651 | 1.00 | 25.13 | A | C |
| ATOM | 1353 | CB | LYS | A | 205 | 21.654 | 25.568 | 58.017 | 1.00 | 32.01 | A | C |
| ATOM | 1354 | CG | LYS | A | 205 | 20.949 | 24.311 | 58.461 | 1.00 | 32.01 | A | C |
| ATOM | 1355 | CD | LYS | A | 205 | 20.035 | 24.539 | 59.645 | 1.00 | 32.01 | A | C |
| ATOM | 1356 | CE | LYS | A | 205 | 19.320 | 23.258 | 60.014 | 1.00 | 32.01 | A | C |
| ATOM | 1357 | NZ | LYS | A | 205 | 18.427 | 23.425 | 61.188 | 1.00 | 32.01 | A | N |
| ATOM | 1358 | C | LYS | A | 205 | 21.208 | 25.235 | 55.604 | 1.00 | 25.13 | A | C |
| ATOM | 1359 | O | LYS | A | 205 | 20.733 | 26.185 | 54.989 | 1.00 | 25.13 | A | O |
| ATOM | 1360 | N | GLN | A | 206 | 20.823 | 23.986 | 55.417 | 1.00 | 35.29 | A | N |
| ATOM | 1361 | CA | GLN | A | 206 | 19.786 | 23.598 | 54.489 | 1.00 | 35.29 | A | C |
| ATOM | 1362 | CB | GLN | A | 206 | 20.024 | 22.129 | 54.149 | 1.00 | 47.39 | A | C |
| ATOM | 1363 | CG | GLN | A | 206 | 18.804 | 21.366 | 53.686 | 1.00 | 47.39 | A | C |
| ATOM | 1364 | CD | GLN | A | 206 | 18.940 | 20.851 | 52.269 | 1.00 | 47.39 | A | C |
| ATOM | 1365 | OE1 | GLN | A | 206 | 17.984 | 20.307 | 51.702 | 1.00 | 47.39 | A | O |
| ATOM | 1366 | NE2 | GLN | A | 206 | 20.133 | 21.014 | 51.684 | 1.00 | 47.39 | A | N |
| ATOM | 1367 | C | GLN | A | 206 | 18.449 | 23.791 | 55.209 | 1.00 | 35.29 | A | C |
| ATOM | 1368 | O | GLN | A | 206 | 18.184 | 23.121 | 56.203 | 1.00 | 35.29 | A | O |
| ATOM | 1369 | N | LEU | A | 207 | 17.619 | 24.714 | 54.737 | 1.00 | 24.64 | A | N |
| ATOM | 1370 | CA | LEU | A | 207 | 16.331 | 24.935 | 55.379 | 1.00 | 24.64 | A | C |
| ATOM | 1371 | CB | LEU | A | 207 | 15.896 | 26.383 | 55.223 | 1.00 | 18.39 | A | C |
| ATOM | 1372 | CG | LEU | A | 207 | 16.883 | 27.445 | 55.711 | 1.00 | 18.39 | A | C |
| ATOM | 1373 | CD1 | LEU | A | 207 | 16.117 | 28.749 | 55.914 | 1.00 | 18.39 | A | C |
| ATOM | 1374 | CD2 | LEU | A | 207 | 17.554 | 27.013 | 57.017 | 1.00 | 18.39 | A | C |
| ATOM | 1375 | C | LEU | A | 207 | 15.288 | 24.008 | 54.776 | 1.00 | 24.64 | A | C |
| ATOM | 1376 | O | LEU | A | 207 | 15.199 | 23.863 | 53.560 | 1.00 | 24.64 | A | O |
| ATOM | 1377 | N | VAL | A | 208 | 14.515 | 23.360 | 55.633 | 1.00 | 40.58 | A | N |
| ATOM | 1378 | CA | VAL | A | 208 | 13.471 | 22.444 | 55.186 | 1.00 | 40.58 | A | C |
| ATOM | 1379 | CB | VAL | A | 208 | 13.775 | 21.008 | 55.627 | 1.00 | 25.52 | A | C |
| ATOM | 1380 | CG1 | VAL | A | 208 | 12.647 | 20.088 | 55.211 | 1.00 | 25.52 | A | C |
| ATOM | 1381 | CG2 | VAL | A | 208 | 15.089 | 20.559 | 55.043 | 1.00 | 25.52 | A | C |
| ATOM | 1382 | C | VAL | A | 208 | 12.143 | 22.846 | 55.819 | 1.00 | 40.58 | A | C |
| ATOM | 1383 | O | VAL | A | 208 | 12.025 | 22.884 | 57.045 | 1.00 | 40.58 | A | O |
| ATOM | 1384 | N | ARG | A | 209 | 11.142 | 23.131 | 54.996 | 1.00 | 44.30 | A | N |
| ATOM | 1385 | CA | ARG | A | 209 | 9.852 | 23.544 | 55.525 | 1.00 | 44.30 | A | C |
| ATOM | 1386 | CB | ARG | A | 209 | 8.789 | 23.492 | 54.440 | 1.00 | 68.10 | A | C |
| ATOM | 1387 | CG | ARG | A | 209 | 7.703 | 24.557 | 54.561 | 1.00 | 68.10 | A | C |
| ATOM | 1388 | CD | ARG | A | 209 | 6.814 | 24.523 | 53.312 | 1.00 | 68.10 | A | C |
| ATOM | 1389 | NE | ARG | A | 209 | 7.591 | 24.132 | 52.124 | 1.00 | 68.10 | A | N |
| ATOM | 1390 | CZ | ARG | A | 209 | 7.088 | 23.897 | 50.907 | 1.00 | 68.10 | A | C |
| ATOM | 1391 | NH1 | ARG | A | 209 | 5.781 | 24.015 | 50.676 | 1.00 | 68.10 | A | N |
| ATOM | 1392 | NH2 | ARG | A | 209 | 7.898 | 23.507 | 49.919 | 1.00 | 68.10 | A | N |
| ATOM | 1393 | C | ARG | A | 209 | 9.487 | 22.598 | 56.644 | 1.00 | 44.30 | A | C |
| ATOM | 1394 | O | ARG | A | 209 | 9.804 | 21.406 | 56.581 | 1.00 | 44.30 | A | O |
| ATOM | 1395 | N | GLY | A | 210 | 8.848 | 23.140 | 57.677 | 1.00 | 51.16 | A | N |
| ATOM | 1396 | CA | GLY | A | 210 | 8.452 | 22.331 | 58.816 | 1.00 | 51.16 | A | C |
| ATOM | 1397 | C | GLY | A | 210 | 9.532 | 22.180 | 59.880 | 1.00 | 51.16 | A | C |
| ATOM | 1398 | O | GLY | A | 210 | 9.245 | 21.870 | 61.049 | 1.00 | 51.16 | A | O |
| ATOM | 1399 | N | GLU | A | 211 | 10.785 | 22.381 | 59.484 | 1.00 | 50.56 | A | N |
| ATOM | 1400 | CA | GLU | A | 211 | 11.889 | 22.279 | 60.438 | 1.00 | 50.56 | A | C |
| ATOM | 1401 | CB | GLU | A | 211 | 13.151 | 21.748 | 59.771 | 1.00 | 70.61 | A | C |

| | | | | | | | | | | | | |
|---|---|---|---|---|---|---|---|---|---|---|---|---|
| ATOM | 1402 | CG | GLU | A | 211 | 13.328 | 20.248 | 59.918 | 1.00 | 70.61 | A | C |
| ATOM | 1403 | CD | GLU | A | 211 | 14.531 | 19.738 | 59.130 | 1.00 | 70.61 | A | C |
| ATOM | 1404 | OE1 | GLU | A | 211 | 14.886 | 18.531 | 59.289 | 1.00 | 70.61 | A | O |
| ATOM | 1405 | OE2 | GLU | A | 211 | 15.097 | 20.563 | 58.355 | 1.00 | 70.61 | A | O |
| ATOM | 1406 | C | GLU | A | 211 | 12.165 | 23.644 | 61.023 | 1.00 | 50.56 | A | C |
| ATOM | 1407 | O | GLU | A | 211 | 12.222 | 24.625 | 60.315 | 1.00 | 50.56 | A | O |
| ATOM | 1408 | N | PRO | A | 212 | 12.328 | 23.722 | 62.335 | 1.00 | 43.08 | A | N |
| ATOM | 1409 | CD | PRO | A | 212 | 12.145 | 22.652 | 63.327 | 1.00 | 38.80 | A | C |
| ATOM | 1410 | CA | PRO | A | 212 | 12.591 | 24.998 | 62.990 | 1.00 | 43.08 | A | C |
| ATOM | 1411 | CB | PRO | A | 212 | 12.034 | 24.762 | 64.385 | 1.00 | 38.80 | A | C |
| ATOM | 1412 | CG | PRO | A | 212 | 12.456 | 23.364 | 64.638 | 1.00 | 38.80 | A | C |
| ATOM | 1413 | C | PRO | A | 212 | 14.090 | 25.243 | 62.979 | 1.00 | 43.08 | A | C |
| ATOM | 1414 | O | PRO | A | 212 | 14.870 | 24.285 | 62.940 | 1.00 | 43.08 | A | O |
| ATOM | 1415 | N | ASN | A | 213 | 14.490 | 26.512 | 62.993 | 1.00 | 40.58 | A | N |
| ATOM | 1416 | CA | ASN | A | 213 | 15.908 | 26.856 | 63.005 | 1.00 | 40.58 | A | C |
| ATOM | 1417 | CB | ASN | A | 213 | 16.359 | 27.262 | 61.616 | 1.00 | 25.80 | A | C |
| ATOM | 1418 | CG | ASN | A | 213 | 16.174 | 26.165 | 60.624 | 1.00 | 25.80 | A | C |
| ATOM | 1419 | OD1 | ASN | A | 213 | 16.940 | 25.205 | 60.597 | 1.00 | 25.80 | A | O |
| ATOM | 1420 | ND2 | ASN | A | 213 | 15.137 | 26.281 | 59.808 | 1.00 | 25.80 | A | N |
| ATOM | 1421 | C | ASN | A | 213 | 16.197 | 27.985 | 63.980 | 1.00 | 40.58 | A | C |
| ATOM | 1422 | O | ASN | A | 213 | 15.360 | 28.864 | 64.194 | 1.00 | 40.58 | A | O |
| ATOM | 1423 | N | VAL | A | 214 | 17.387 | 27.956 | 64.570 | 1.00 | 21.29 | A | N |
| ATOM | 1424 | CA | VAL | A | 214 | 17.781 | 28.972 | 65.531 | 1.00 | 21.29 | A | C |
| ATOM | 1425 | CB | VAL | A | 214 | 19.212 | 28.741 | 65.994 | 1.00 | 19.26 | A | C |
| ATOM | 1426 | CG1 | VAL | A | 214 | 19.324 | 27.364 | 66.600 | 1.00 | 19.26 | A | C |
| ATOM | 1427 | CG2 | VAL | A | 214 | 20.172 | 28.911 | 64.814 | 1.00 | 19.26 | A | C |
| ATOM | 1428 | C | VAL | A | 214 | 17.664 | 30.387 | 64.966 | 1.00 | 21.29 | A | C |
| ATOM | 1429 | O | VAL | A | 214 | 18.009 | 30.645 | 63.814 | 1.00 | 21.29 | A | O |
| ATOM | 1430 | N | SER | A | 215 | 17.195 | 31.314 | 65.788 | 1.00 | 31.77 | A | N |
| ATOM | 1431 | CA | SER | A | 215 | 17.023 | 32.677 | 65.324 | 1.00 | 31.77 | A | C |
| ATOM | 1432 | CB | SER | A | 215 | 15.637 | 33.211 | 65.737 | 1.00 | 43.62 | A | C |
| ATOM | 1433 | OG | SER | A | 215 | 15.265 | 32.797 | 67.041 | 1.00 | 43.62 | A | O |
| ATOM | 1434 | C | SER | A | 215 | 18.115 | 33.628 | 65.778 | 1.00 | 31.77 | A | C |
| ATOM | 1435 | O | SER | A | 215 | 17.931 | 34.839 | 65.733 | 1.00 | 31.77 | A | O |
| ATOM | 1436 | N | TYR | A | 216 | 19.242 | 33.094 | 66.231 | 1.00 | 28.27 | A | N |
| ATOM | 1437 | CA | TYR | A | 216 | 20.354 | 33.956 | 66.638 | 1.00 | 28.27 | A | C |
| ATOM | 1438 | CB | TYR | A | 216 | 20.781 | 33.656 | 68.073 | 1.00 | 26.32 | A | C |
| ATOM | 1439 | CG | TYR | A | 216 | 20.927 | 32.201 | 68.375 | 1.00 | 26.32 | A | C |
| ATOM | 1440 | CD1 | TYR | A | 216 | 21.967 | 31.460 | 67.832 | 1.00 | 26.32 | A | C |
| ATOM | 1441 | CE1 | TYR | A | 216 | 22.095 | 30.115 | 68.101 | 1.00 | 26.32 | A | C |
| ATOM | 1442 | CD2 | TYR | A | 216 | 20.013 | 31.556 | 69.200 | 1.00 | 26.32 | A | C |
| ATOM | 1443 | CE2 | TYR | A | 216 | 20.128 | 30.205 | 69.478 | 1.00 | 26.32 | A | C |
| ATOM | 1444 | CZ | TYR | A | 216 | 21.171 | 29.491 | 68.924 | 1.00 | 26.32 | A | C |
| ATOM | 1445 | OH | TYR | A | 216 | 21.277 | 28.148 | 69.186 | 1.00 | 26.32 | A | O |
| ATOM | 1446 | C | TYR | A | 216 | 21.549 | 33.802 | 65.682 | 1.00 | 28.27 | A | C |
| ATOM | 1447 | O | TYR | A | 216 | 22.714 | 33.869 | 66.090 | 1.00 | 28.27 | A | O |
| ATOM | 1448 | N | ILE | A | 217 | 21.246 | 33.652 | 64.391 | 1.00 | 38.24 | A | N |
| ATOM | 1449 | CA | ILE | A | 217 | 22.282 | 33.421 | 63.401 | 1.00 | 38.24 | A | C |
| ATOM | 1450 | CB | ILE | A | 217 | 21.890 | 32.306 | 62.401 | 1.00 | 46.69 | A | C |
| ATOM | 1451 | CG2 | ILE | A | 217 | 22.395 | 30.983 | 62.889 | 1.00 | 46.69 | A | C |
| ATOM | 1452 | CG1 | ILE | A | 217 | 20.388 | 32.344 | 62.136 | 1.00 | 46.69 | A | C |
| ATOM | 1453 | CD1 | ILE | A | 217 | 19.893 | 33.732 | 61.745 | 1.00 | 46.69 | A | C |
| ATOM | 1454 | C | ILE | A | 217 | 22.859 | 34.536 | 62.543 | 1.00 | 38.24 | A | C |
| ATOM | 1455 | O | ILE | A | 217 | 24.016 | 34.450 | 62.120 | 1.00 | 38.24 | A | O |
| ATOM | 1456 | N | CYS | A | 218 | 22.099 | 35.573 | 62.253 | 1.00 | 24.43 | A | N |
| ATOM | 1457 | CA | CYS | A | 218 | 22.663 | 36.597 | 61.383 | 1.00 | 24.43 | A | C |
| ATOM | 1458 | CB | CYS | A | 218 | 21.532 | 37.186 | 60.547 | 1.00 | 25.90 | A | C |
| ATOM | 1459 | SG | CYS | A | 218 | 22.011 | 38.102 | 59.142 | 1.00 | 25.90 | A | S |
| ATOM | 1460 | C | CYS | A | 218 | 23.346 | 37.690 | 62.197 | 1.00 | 24.43 | A | C |
| ATOM | 1461 | O | CYS | A | 218 | 23.021 | 37.883 | 63.362 | 1.00 | 24.43 | A | O |
| ATOM | 1462 | N | SER | A | 219 | 24.276 | 38.413 | 61.580 | 1.00 | 25.88 | A | N |
| ATOM | 1463 | CA | SER | A | 219 | 25.015 | 39.480 | 62.258 | 1.00 | 25.88 | A | C |
| ATOM | 1464 | CB | SER | A | 219 | 26.317 | 39.764 | 61.513 | 1.00 | 38.59 | A | C |
| ATOM | 1465 | OG | SER | A | 219 | 27.147 | 38.613 | 61.525 | 1.00 | 38.59 | A | O |
| ATOM | 1466 | C | SER | A | 219 | 24.227 | 40.775 | 62.424 | 1.00 | 25.88 | A | C |
| ATOM | 1467 | O | SER | A | 219 | 23.325 | 41.057 | 61.660 | 1.00 | 25.88 | A | O |
| ATOM | 1468 | N | ALA | A | 220 | 24.583 | 41.558 | 63.434 | 1.00 | 43.88 | A | N |

| | | | | | | | | | | | | |
|---|---|---|---|---|---|---|---|---|---|---|---|---|
| ATOM | 1469 | CA | ALA | A | 220 | 23.903 | 42.819 | 63.721 | 1.00 | 43.88 | A | C |
| ATOM | 1470 | CB | ALA | A | 220 | 24.509 | 43.469 | 64.973 | 1.00 | 46.88 | A | C |
| ATOM | 1471 | C | ALA | A | 220 | 23.987 | 43.777 | 62.542 | 1.00 | 43.88 | A | C |
| ATOM | 1472 | O | ALA | A | 220 | 25.028 | 43.888 | 61.885 | 1.00 | 43.88 | A | O |
| ATOM | 1473 | N | TYR | A | 221 | 22.887 | 44.480 | 62.295 | 1.00 | 28.11 | A | N |
| ATOM | 1474 | CA | TYR | A | 221 | 22.791 | 45.444 | 61.197 | 1.00 | 28.11 | A | C |
| ATOM | 1475 | CB | TYR | A | 221 | 24.151 | 46.093 | 60.874 | 1.00 | 42.62 | A | C |
| ATOM | 1476 | CG | TYR | A | 221 | 24.748 | 47.007 | 61.942 | 1.00 | 42.62 | A | C |
| ATOM | 1477 | CD1 | TYR | A | 221 | 24.168 | 47.134 | 63.209 | 1.00 | 42.62 | A | C |
| ATOM | 1478 | CE1 | TYR | A | 221 | 24.760 | 47.944 | 64.195 | 1.00 | 42.62 | A | C |
| ATOM | 1479 | CD2 | TYR | A | 221 | 25.932 | 47.717 | 61.689 | 1.00 | 42.62 | A | C |
| ATOM | 1480 | CE2 | TYR | A | 221 | 26.526 | 48.521 | 62.661 | 1.00 | 42.62 | A | C |
| ATOM | 1481 | CZ | TYR | A | 221 | 25.938 | 48.629 | 63.907 | 1.00 | 42.62 | A | C |
| ATOM | 1482 | OH | TYR | A | 221 | 26.532 | 49.413 | 64.868 | 1.00 | 42.62 | A | O |
| ATOM | 1483 | C | TYR | A | 221 | 22.234 | 44.833 | 59.913 | 1.00 | 28.11 | A | C |
| ATOM | 1484 | O | TYR | A | 221 | 21.744 | 45.556 | 59.048 | 1.00 | 28.11 | A | O |
| ATOM | 1485 | N | TYR | A | 222 | 22.290 | 43.511 | 59.797 | 1.00 | 26.75 | A | N |
| ATOM | 1486 | CA | TYR | A | 222 | 21.816 | 42.843 | 58.590 | 1.00 | 26.75 | A | C |
| ATOM | 1487 | CB | TYR | A | 222 | 23.004 | 42.233 | 57.864 | 1.00 | 17.88 | A | C |
| ATOM | 1488 | CG | TYR | A | 222 | 24.135 | 43.207 | 57.694 | 1.00 | 17.88 | A | C |
| ATOM | 1489 | CD1 | TYR | A | 222 | 24.167 | 44.085 | 56.620 | 1.00 | 17.88 | A | C |
| ATOM | 1490 | CE1 | TYR | A | 222 | 25.191 | 45.019 | 56.496 | 1.00 | 17.88 | A | C |
| ATOM | 1491 | CD2 | TYR | A | 222 | 25.152 | 43.286 | 58.640 | 1.00 | 17.88 | A | C |
| ATOM | 1492 | CE2 | TYR | A | 222 | 26.175 | 44.220 | 58.525 | 1.00 | 17.88 | A | C |
| ATOM | 1493 | CZ | TYR | A | 222 | 26.190 | 45.080 | 57.456 | 1.00 | 17.88 | A | C |
| ATOM | 1494 | OH | TYR | A | 222 | 27.198 | 46.005 | 57.368 | 1.00 | 17.88 | A | O |
| ATOM | 1495 | C | TYR | A | 222 | 20.786 | 41.755 | 58.825 | 1.00 | 26.75 | A | C |
| ATOM | 1496 | O | TYR | A | 222 | 20.453 | 41.016 | 57.901 | 1.00 | 26.75 | A | O |
| ATOM | 1497 | N | ARG | A | 223 | 20.279 | 41.660 | 60.049 | 1.00 | 16.93 | A | N |
| ATOM | 1498 | CA | ARG | A | 223 | 19.306 | 40.636 | 60.381 | 1.00 | 16.93 | A | C |
| ATOM | 1499 | CB | ARG | A | 223 | 19.281 | 40.410 | 61.887 | 1.00 | 37.89 | A | C |
| ATOM | 1500 | CG | ARG | A | 223 | 20.648 | 40.515 | 62.523 | 1.00 | 37.89 | A | C |
| ATOM | 1501 | CD | ARG | A | 223 | 20.613 | 40.249 | 64.030 | 1.00 | 37.89 | A | C |
| ATOM | 1502 | NE | ARG | A | 223 | 20.373 | 38.836 | 64.299 | 1.00 | 37.89 | A | N |
| ATOM | 1503 | CZ | ARG | A | 223 | 19.289 | 38.364 | 64.897 | 1.00 | 37.89 | A | C |
| ATOM | 1504 | NH1 | ARG | A | 223 | 18.339 | 39.196 | 65.300 | 1.00 | 37.89 | A | N |
| ATOM | 1505 | NH2 | ARG | A | 223 | 19.156 | 37.057 | 65.079 | 1.00 | 37.89 | A | N |
| ATOM | 1506 | C | ARG | A | 223 | 17.927 | 41.036 | 59.907 | 1.00 | 16.93 | A | C |
| ATOM | 1507 | O | ARG | A | 223 | 17.519 | 42.174 | 60.074 | 1.00 | 16.93 | A | O |
| ATOM | 1508 | N | ALA | A | 224 | 17.221 | 40.092 | 59.298 | 1.00 | 35.15 | A | N |
| ATOM | 1509 | CA | ALA | A | 224 | 15.867 | 40.329 | 58.809 | 1.00 | 35.15 | A | C |
| ATOM | 1510 | CB | ALA | A | 224 | 15.375 | 39.118 | 58.039 | 1.00 | 39.28 | A | C |
| ATOM | 1511 | C | ALA | A | 224 | 14.952 | 40.594 | 60.000 | 1.00 | 35.15 | A | C |
| ATOM | 1512 | O | ALA | A | 224 | 15.215 | 40.123 | 61.099 | 1.00 | 35.15 | A | O |
| ATOM | 1513 | N | PRO | A | 225 | 13.862 | 41.349 | 59.798 | 1.00 | 29.83 | A | N |
| ATOM | 1514 | CD | PRO | A | 225 | 13.372 | 41.930 | 58.540 | 1.00 | 20.02 | A | C |
| ATOM | 1515 | CA | PRO | A | 225 | 12.940 | 41.646 | 60.897 | 1.00 | 29.83 | A | C |
| ATOM | 1516 | CB | PRO | A | 225 | 11.792 | 42.367 | 60.203 | 1.00 | 20.02 | A | C |
| ATOM | 1517 | CG | PRO | A | 225 | 12.445 | 42.997 | 59.039 | 1.00 | 20.02 | A | C |
| ATOM | 1518 | C | PRO | A | 225 | 12.447 | 40.397 | 61.624 | 1.00 | 29.83 | A | C |
| ATOM | 1519 | O | PRO | A | 225 | 12.275 | 40.414 | 62.841 | 1.00 | 29.83 | A | O |
| ATOM | 1520 | N | GLU | A | 226 | 12.213 | 39.319 | 60.876 | 1.00 | 29.64 | A | N |
| ATOM | 1521 | CA | GLU | A | 226 | 11.718 | 38.073 | 61.464 | 1.00 | 29.64 | A | C |
| ATOM | 1522 | CB | GLU | A | 226 | 11.486 | 36.994 | 60.403 | 1.00 | 40.23 | A | C |
| ATOM | 1523 | CG | GLU | A | 226 | 10.982 | 37.480 | 59.077 | 1.00 | 40.23 | A | C |
| ATOM | 1524 | CD | GLU | A | 226 | 12.107 | 37.849 | 58.138 | 1.00 | 40.23 | A | C |
| ATOM | 1525 | OE1 | GLU | A | 226 | 12.788 | 36.930 | 57.617 | 1.00 | 40.23 | A | O |
| ATOM | 1526 | OE2 | GLU | A | 226 | 12.306 | 39.065 | 57.932 | 1.00 | 40.23 | A | O |
| ATOM | 1527 | C | GLU | A | 226 | 12.738 | 37.526 | 62.449 | 1.00 | 29.64 | A | C |
| ATOM | 1528 | O | GLU | A | 226 | 12.408 | 37.133 | 63.576 | 1.00 | 29.64 | A | O |
| ATOM | 1529 | N | LEU | A | 227 | 13.983 | 37.467 | 61.993 | 1.00 | 35.73 | A | N |
| ATOM | 1530 | CA | LEU | A | 227 | 15.055 | 36.985 | 62.838 | 1.00 | 35.73 | A | C |
| ATOM | 1531 | CB | LEU | A | 227 | 16.409 | 37.172 | 62.142 | 1.00 | 16.70 | A | C |
| ATOM | 1532 | CG | LEU | A | 227 | 16.693 | 36.265 | 60.942 | 1.00 | 16.70 | A | C |
| ATOM | 1533 | CD1 | LEU | A | 227 | 18.105 | 36.492 | 60.450 | 1.00 | 16.70 | A | C |
| ATOM | 1534 | CD2 | LEU | A | 227 | 16.508 | 34.807 | 61.345 | 1.00 | 16.70 | A | C |
| ATOM | 1535 | C | LEU | A | 227 | 15.048 | 37.713 | 64.181 | 1.00 | 35.73 | A | C |

| | | | | | | | | | | | | |
|---|---|---|---|---|---|---|---|---|---|---|---|---|
| ATOM | 1536 | O | LEU | A | 227 | 15.283 | 37.101 | 65.214 | 1.00 | 35.73 | A | O |
| ATOM | 1537 | N | ILE | A | 228 | 14.770 | 39.015 | 64.153 | 1.00 | 25.39 | A | N |
| ATOM | 1538 | CA | ILE | A | 228 | 14.738 | 39.854 | 65.344 | 1.00 | 25.39 | A | C |
| ATOM | 1539 | CB | ILE | A | 228 | 14.704 | 41.342 | 64.953 | 1.00 | 15.89 | A | C |
| ATOM | 1540 | CG2 | ILE | A | 228 | 14.482 | 42.199 | 66.165 | 1.00 | 15.89 | A | C |
| ATOM | 1541 | CG1 | ILE | A | 228 | 16.013 | 41.723 | 64.264 | 1.00 | 15.89 | A | C |
| ATOM | 1542 | CD1 | ILE | A | 228 | 16.019 | 43.124 | 63.683 | 1.00 | 15.89 | A | C |
| ATOM | 1543 | C | ILE | A | 228 | 13.521 | 39.536 | 66.184 | 1.00 | 25.39 | A | C |
| ATOM | 1544 | O | ILE | A | 228 | 13.539 | 39.718 | 67.397 | 1.00 | 25.39 | A | O |
| ATOM | 1545 | N | PHE | A | 229 | 12.461 | 39.063 | 65.531 | 1.00 | 29.84 | A | N |
| ATOM | 1546 | CA | PHE | A | 229 | 11.228 | 38.705 | 66.229 | 1.00 | 29.84 | A | C |
| ATOM | 1547 | CB | PHE | A | 229 | 10.029 | 38.842 | 65.297 | 1.00 | 31.67 | A | C |
| ATOM | 1548 | CG | PHE | A | 229 | 9.498 | 40.228 | 65.212 | 1.00 | 31.67 | A | C |
| ATOM | 1549 | CD1 | PHE | A | 229 | 8.940 | 40.837 | 66.324 | 1.00 | 31.67 | A | C |
| ATOM | 1550 | CD2 | PHE | A | 229 | 9.580 | 40.942 | 64.033 | 1.00 | 31.67 | A | C |
| ATOM | 1551 | CE1 | PHE | A | 229 | 8.480 | 42.133 | 66.265 | 1.00 | 31.67 | A | C |
| ATOM | 1552 | CE2 | PHE | A | 229 | 9.118 | 42.250 | 63.969 | 1.00 | 31.67 | A | C |
| ATOM | 1553 | CZ | PHE | A | 229 | 8.568 | 42.842 | 65.090 | 1.00 | 31.67 | A | C |
| ATOM | 1554 | C | PHE | A | 229 | 11.295 | 37.278 | 66.767 | 1.00 | 29.84 | A | C |
| ATOM | 1555 | O | PHE | A | 229 | 10.298 | 36.730 | 67.254 | 1.00 | 29.84 | A | O |
| ATOM | 1556 | N | GLY | A | 230 | 12.472 | 36.676 | 66.659 | 1.00 | 36.74 | A | N |
| ATOM | 1557 | CA | GLY | A | 230 | 12.656 | 35.335 | 67.174 | 1.00 | 36.74 | A | C |
| ATOM | 1558 | C | GLY | A | 230 | 12.022 | 34.228 | 66.357 | 1.00 | 36.74 | A | C |
| ATOM | 1559 | O | GLY | A | 230 | 11.994 | 33.081 | 66.799 | 1.00 | 36.74 | A | O |
| ATOM | 1560 | N | ALA | A | 231 | 11.516 | 34.551 | 65.169 | 1.00 | 22.97 | A | N |
| ATOM | 1561 | CA | ALA | A | 231 | 10.899 | 33.525 | 64.331 | 1.00 | 22.97 | A | C |
| ATOM | 1562 | CB | ALA | A | 231 | 10.503 | 34.108 | 62.985 | 1.00 | 11.00 | A | C |
| ATOM | 1563 | C | ALA | A | 231 | 11.878 | 32.377 | 64.133 | 1.00 | 22.97 | A | C |
| ATOM | 1564 | O | ALA | A | 231 | 13.093 | 32.578 | 64.129 | 1.00 | 22.97 | A | O |
| ATOM | 1565 | N | THR | A | 232 | 11.352 | 31.166 | 63.983 | 1.00 | 28.56 | A | N |
| ATOM | 1566 | CA | THR | A | 232 | 12.216 | 30.011 | 63.781 | 1.00 | 28.56 | A | C |
| ATOM | 1567 | CB | THR | A | 232 | 12.174 | 29.074 | 64.979 | 1.00 | 29.30 | A | C |
| ATOM | 1568 | OG1 | THR | A | 232 | 10.865 | 28.512 | 65.094 | 1.00 | 29.30 | A | O |
| ATOM | 1569 | CG2 | THR | A | 232 | 12.523 | 29.832 | 66.250 | 1.00 | 29.30 | A | C |
| ATOM | 1570 | C | THR | A | 232 | 11.831 | 29.233 | 62.532 | 1.00 | 28.56 | A | C |
| ATOM | 1571 | O | THR | A | 232 | 12.387 | 28.165 | 62.244 | 1.00 | 28.56 | A | O |
| ATOM | 1572 | N | ASP | A | 233 | 10.880 | 29.789 | 61.790 | 1.00 | 25.06 | A | N |
| ATOM | 1573 | CA | ASP | A | 233 | 10.403 | 29.185 | 60.552 | 1.00 | 25.06 | A | C |
| ATOM | 1574 | CB | ASP | A | 233 | 8.897 | 28.980 | 60.633 | 1.00 | 35.46 | A | C |
| ATOM | 1575 | CG | ASP | A | 233 | 8.148 | 30.290 | 60.782 | 1.00 | 35.46 | A | C |
| ATOM | 1576 | OD1 | ASP | A | 233 | 8.807 | 31.351 | 60.833 | 1.00 | 35.46 | A | O |
| ATOM | 1577 | OD2 | ASP | A | 233 | 6.902 | 30.259 | 60.854 | 1.00 | 35.46 | A | O |
| ATOM | 1578 | C | ASP | A | 233 | 10.721 | 30.106 | 59.372 | 1.00 | 25.06 | A | C |
| ATOM | 1579 | O | ASP | A | 233 | 9.965 | 30.177 | 58.397 | 1.00 | 25.06 | A | O |
| ATOM | 1580 | N | TYR | A | 234 | 11.837 | 30.817 | 59.473 | 1.00 | 23.49 | A | N |
| ATOM | 1581 | CA | TYR | A | 234 | 12.241 | 31.728 | 58.421 | 1.00 | 23.49 | A | C |
| ATOM | 1582 | CB | TYR | A | 234 | 13.345 | 32.661 | 58.933 | 1.00 | 19.44 | A | C |
| ATOM | 1583 | CG | TYR | A | 234 | 14.538 | 31.961 | 59.532 | 1.00 | 19.44 | A | C |
| ATOM | 1584 | CD1 | TYR | A | 234 | 15.641 | 31.634 | 58.751 | 1.00 | 19.44 | A | C |
| ATOM | 1585 | CE1 | TYR | A | 234 | 16.755 | 31.009 | 59.308 | 1.00 | 19.44 | A | C |
| ATOM | 1586 | CD2 | TYR | A | 234 | 14.574 | 31.639 | 60.887 | 1.00 | 19.44 | A | C |
| ATOM | 1587 | CE2 | TYR | A | 234 | 15.675 | 31.017 | 61.450 | 1.00 | 19.44 | A | C |
| ATOM | 1588 | CZ | TYR | A | 234 | 16.762 | 30.707 | 60.656 | 1.00 | 19.44 | A | C |
| ATOM | 1589 | OH | TYR | A | 234 | 17.866 | 30.106 | 61.202 | 1.00 | 19.44 | A | O |
| ATOM | 1590 | C | TYR | A | 234 | 12.693 | 30.958 | 57.184 | 1.00 | 23.49 | A | C |
| ATOM | 1591 | O | TYR | A | 234 | 13.073 | 29.791 | 57.263 | 1.00 | 23.49 | A | O |
| ATOM | 1592 | N | THR | A | 235 | 12.625 | 31.621 | 56.037 | 1.00 | 31.88 | A | N |
| ATOM | 1593 | CA | THR | A | 235 | 13.027 | 31.018 | 54.777 | 1.00 | 31.88 | A | C |
| ATOM | 1594 | CB | THR | A | 235 | 11.939 | 31.150 | 53.726 | 1.00 | 18.71 | A | C |
| ATOM | 1595 | OG1 | THR | A | 235 | 11.818 | 32.523 | 53.342 | 1.00 | 18.71 | A | O |
| ATOM | 1596 | CG2 | THR | A | 235 | 10.629 | 30.677 | 54.272 | 1.00 | 18.71 | A | C |
| ATOM | 1597 | C | THR | A | 235 | 14.265 | 31.697 | 54.223 | 1.00 | 31.88 | A | C |
| ATOM | 1598 | O | THR | A | 235 | 14.741 | 32.681 | 54.773 | 1.00 | 31.88 | A | O |
| ATOM | 1599 | N | SER | A | 236 | 14.788 | 31.181 | 53.121 | 1.00 | 30.52 | A | N |
| ATOM | 1600 | CA | SER | A | 236 | 15.979 | 31.780 | 52.529 | 1.00 | 30.52 | A | C |
| ATOM | 1601 | CB | SER | A | 236 | 16.323 | 31.083 | 51.220 | 1.00 | 26.32 | A | C |
| ATOM | 1602 | OG | SER | A | 236 | 16.803 | 29.781 | 51.472 | 1.00 | 26.32 | A | O |

| | | | | | | | | | | | | |
|---|---|---|---|---|---|---|---|---|---|---|---|---|
| ATOM | 1603 | C | SER | A | 236 | 15.845 | 33.277 | 52.276 | 1.00 | 30.52 | A | C |
| ATOM | 1604 | O | SER | A | 236 | 16.827 | 33.955 | 52.006 | 1.00 | 30.52 | A | O |
| ATOM | 1605 | N | SER | A | 237 | 14.630 | 33.796 | 52.358 | 1.00 | 26.49 | A | N |
| ATOM | 1606 | CA | SER | A | 237 | 14.422 | 35.206 | 52.118 | 1.00 | 26.49 | A | C |
| ATOM | 1607 | CB | SER | A | 237 | 12.940 | 35.541 | 52.173 | 1.00 | 46.05 | A | C |
| ATOM | 1608 | OG | SER | A | 237 | 12.346 | 35.074 | 53.370 | 1.00 | 46.05 | A | O |
| ATOM | 1609 | C | SER | A | 237 | 15.178 | 36.048 | 53.114 | 1.00 | 26.49 | A | C |
| ATOM | 1610 | O | SER | A | 237 | 15.258 | 37.259 | 52.944 | 1.00 | 26.49 | A | O |
| ATOM | 1611 | N | ILE | A | 238 | 15.734 | 35.421 | 54.150 | 1.00 | 26.16 | A | N |
| ATOM | 1612 | CA | ILE | A | 238 | 16.480 | 36.198 | 55.131 | 1.00 | 26.16 | A | C |
| ATOM | 1613 | CB | ILE | A | 238 | 16.822 | 35.419 | 56.418 | 1.00 | 27.50 | A | C |
| ATOM | 1614 | CG2 | ILE | A | 238 | 15.574 | 34.799 | 56.998 | 1.00 | 27.50 | A | C |
| ATOM | 1615 | CG1 | ILE | A | 238 | 17.894 | 34.377 | 56.138 | 1.00 | 27.50 | A | C |
| ATOM | 1616 | CD1 | ILE | A | 238 | 18.581 | 33.887 | 57.392 | 1.00 | 27.50 | A | C |
| ATOM | 1617 | C | ILE | A | 238 | 17.764 | 36.721 | 54.498 | 1.00 | 26.16 | A | C |
| ATOM | 1618 | O | ILE | A | 238 | 18.208 | 37.825 | 54.804 | 1.00 | 26.16 | A | O |
| ATOM | 1619 | N | ASP | A | 239 | 18.346 | 35.932 | 53.601 | 1.00 | 17.20 | A | N |
| ATOM | 1620 | CA | ASP | A | 239 | 19.557 | 36.334 | 52.905 | 1.00 | 17.20 | A | C |
| ATOM | 1621 | CB | ASP | A | 239 | 20.103 | 35.197 | 52.046 | 1.00 | 29.89 | A | C |
| ATOM | 1622 | CG | ASP | A | 239 | 20.864 | 34.170 | 52.846 | 1.00 | 29.89 | A | C |
| ATOM | 1623 | OD1 | ASP | A | 239 | 21.323 | 34.485 | 53.965 | 1.00 | 29.89 | A | O |
| ATOM | 1624 | OD2 | ASP | A | 239 | 21.021 | 33.044 | 52.340 | 1.00 | 29.89 | A | O |
| ATOM | 1625 | C | ASP | A | 239 | 19.254 | 37.507 | 52.003 | 1.00 | 17.20 | A | C |
| ATOM | 1626 | O | ASP | A | 239 | 20.038 | 38.436 | 51.924 | 1.00 | 17.20 | A | O |
| ATOM | 1627 | N | VAL | A | 240 | 18.118 | 37.466 | 51.319 | 1.00 | 21.44 | A | N |
| ATOM | 1628 | CA | VAL | A | 240 | 17.761 | 38.547 | 50.415 | 1.00 | 21.44 | A | C |
| ATOM | 1629 | CB | VAL | A | 240 | 16.436 | 38.253 | 49.693 | 1.00 | 11.49 | A | C |
| ATOM | 1630 | CG1 | VAL | A | 240 | 16.025 | 39.442 | 48.818 | 1.00 | 11.49 | A | C |
| ATOM | 1631 | CG2 | VAL | A | 240 | 16.606 | 37.007 | 48.846 | 1.00 | 11.49 | A | C |
| ATOM | 1632 | C | VAL | A | 240 | 17.666 | 39.862 | 51.164 | 1.00 | 21.44 | A | C |
| ATOM | 1633 | O | VAL | A | 240 | 18.063 | 40.907 | 50.649 | 1.00 | 21.44 | A | O |
| ATOM | 1634 | N | TRP | A | 241 | 17.163 | 39.793 | 52.394 | 1.00 | 26.09 | A | N |
| ATOM | 1635 | CA | TRP | A | 241 | 17.010 | 40.978 | 53.226 | 1.00 | 26.09 | A | C |
| ATOM | 1636 | CB | TRP | A | 241 | 16.330 | 40.624 | 54.553 | 1.00 | 20.95 | A | C |
| ATOM | 1637 | CG | TRP | A | 241 | 16.293 | 41.764 | 55.537 | 1.00 | 20.95 | A | C |
| ATOM | 1638 | CD2 | TRP | A | 241 | 15.248 | 42.732 | 55.700 | 1.00 | 20.95 | A | C |
| ATOM | 1639 | CE2 | TRP | A | 241 | 15.668 | 43.636 | 56.696 | 1.00 | 20.95 | A | C |
| ATOM | 1640 | CE3 | TRP | A | 241 | 13.992 | 42.919 | 55.104 | 1.00 | 20.95 | A | C |
| ATOM | 1641 | CD1 | TRP | A | 241 | 17.275 | 42.116 | 56.412 | 1.00 | 20.95 | A | C |
| ATOM | 1642 | NE1 | TRP | A | 241 | 16.911 | 43.238 | 57.110 | 1.00 | 20.95 | A | N |
| ATOM | 1643 | CZ2 | TRP | A | 241 | 14.890 | 44.719 | 57.103 | 1.00 | 20.95 | A | C |
| ATOM | 1644 | CZ3 | TRP | A | 241 | 13.213 | 44.000 | 55.511 | 1.00 | 20.95 | A | C |
| ATOM | 1645 | CH2 | TRP | A | 241 | 13.666 | 44.883 | 56.505 | 1.00 | 20.95 | A | C |
| ATOM | 1646 | C | TRP | A | 241 | 18.379 | 41.543 | 53.502 | 1.00 | 26.09 | A | C |
| ATOM | 1647 | O | TRP | A | 241 | 18.646 | 42.720 | 53.261 | 1.00 | 26.09 | A | O |
| ATOM | 1648 | N | SER | A | 242 | 19.240 | 40.680 | 54.014 | 1.00 | 22.40 | A | N |
| ATOM | 1649 | CA | SER | A | 242 | 20.595 | 41.060 | 54.337 | 1.00 | 22.40 | A | C |
| ATOM | 1650 | CB | SER | A | 242 | 21.386 | 39.831 | 54.781 | 1.00 | 36.07 | A | C |
| ATOM | 1651 | OG | SER | A | 242 | 20.709 | 39.139 | 55.817 | 1.00 | 36.07 | A | O |
| ATOM | 1652 | C | SER | A | 242 | 21.236 | 41.695 | 53.112 | 1.00 | 22.40 | A | C |
| ATOM | 1653 | O | SER | A | 242 | 21.926 | 42.712 | 53.213 | 1.00 | 22.40 | A | O |
| ATOM | 1654 | N | ALA | A | 243 | 20.990 | 41.082 | 51.957 | 1.00 | 30.21 | A | N |
| ATOM | 1655 | CA | ALA | A | 243 | 21.522 | 41.544 | 50.682 | 1.00 | 30.21 | A | C |
| ATOM | 1656 | CB | ALA | A | 243 | 21.005 | 40.662 | 49.576 | 1.00 | 31.59 | A | C |
| ATOM | 1657 | C | ALA | A | 243 | 21.101 | 42.995 | 50.461 | 1.00 | 30.21 | A | C |
| ATOM | 1658 | O | ALA | A | 243 | 21.931 | 43.857 | 50.144 | 1.00 | 30.21 | A | O |
| ATOM | 1659 | N | GLY | A | 244 | 19.804 | 43.247 | 50.629 | 1.00 | 29.66 | A | N |
| ATOM | 1660 | CA | GLY | A | 244 | 19.273 | 44.593 | 50.502 | 1.00 | 29.66 | A | C |
| ATOM | 1661 | C | GLY | A | 244 | 19.980 | 45.554 | 51.453 | 1.00 | 29.66 | A | C |
| ATOM | 1662 | O | GLY | A | 244 | 20.322 | 46.678 | 51.078 | 1.00 | 29.66 | A | O |
| ATOM | 1663 | N | CYS | A | 245 | 20.217 | 45.112 | 52.686 | 1.00 | 39.51 | A | N |
| ATOM | 1664 | CA | CYS | A | 245 | 20.893 | 45.946 | 53.674 | 1.00 | 39.51 | A | C |
| ATOM | 1665 | CB | CYS | A | 245 | 20.932 | 45.247 | 55.029 | 1.00 | 24.59 | A | C |
| ATOM | 1666 | SG | CYS | A | 245 | 19.331 | 44.946 | 55.720 | 1.00 | 24.59 | A | S |
| ATOM | 1667 | C | CYS | A | 245 | 22.313 | 46.312 | 53.247 | 1.00 | 39.51 | A | C |
| ATOM | 1668 | O | CYS | A | 245 | 22.842 | 47.355 | 53.658 | 1.00 | 39.51 | A | O |
| ATOM | 1669 | N | VAL | A | 246 | 22.929 | 45.455 | 52.435 | 1.00 | 16.32 | A | N |

| | | | | | | | | | | | | |
|---|---|---|---|---|---|---|---|---|---|---|---|---|
| ATOM | 1670 | CA | VAL | A | 246 | 24.279 | 45.712 | 51.936 | 1.00 | 16.32 | A | C |
| ATOM | 1671 | CB | VAL | A | 246 | 24.941 | 44.426 | 51.403 | 1.00 | 15.28 | A | C |
| ATOM | 1672 | CG1 | VAL | A | 246 | 26.151 | 44.772 | 50.543 | 1.00 | 15.28 | A | C |
| ATOM | 1673 | CG2 | VAL | A | 246 | 25.365 | 43.558 | 52.576 | 1.00 | 15.28 | A | C |
| ATOM | 1674 | C | VAL | A | 246 | 24.240 | 46.753 | 50.821 | 1.00 | 16.32 | A | C |
| ATOM | 1675 | O | VAL | A | 246 | 25.091 | 47.647 | 50.757 | 1.00 | 16.32 | A | O |
| ATOM | 1676 | N | LEU | A | 247 | 23.235 | 46.631 | 49.956 | 1.00 | 18.03 | A | N |
| ATOM | 1677 | CA | LEU | A | 247 | 23.049 | 47.548 | 48.844 | 1.00 | 18.03 | A | C |
| ATOM | 1678 | CB | LEU | A | 247 | 21.854 | 47.111 | 47.999 | 1.00 | 28.31 | A | C |
| ATOM | 1679 | CG | LEU | A | 247 | 21.376 | 48.066 | 46.898 | 1.00 | 28.31 | A | C |
| ATOM | 1680 | CD1 | LEU | A | 247 | 22.466 | 48.328 | 45.849 | 1.00 | 28.31 | A | C |
| ATOM | 1681 | CD2 | LEU | A | 247 | 20.147 | 47.466 | 46.264 | 1.00 | 28.31 | A | C |
| ATOM | 1682 | C | LEU | A | 247 | 22.801 | 48.937 | 49.399 | 1.00 | 18.03 | A | C |
| ATOM | 1683 | O | LEU | A | 247 | 23.455 | 49.902 | 49.019 | 1.00 | 18.03 | A | O |
| ATOM | 1684 | N | ALA | A | 248 | 21.838 | 49.035 | 50.301 | 1.00 | 21.61 | A | N |
| ATOM | 1685 | CA | ALA | A | 248 | 21.525 | 50.310 | 50.911 | 1.00 | 21.61 | A | C |
| ATOM | 1686 | CB | ALA | A | 248 | 20.347 | 50.158 | 51.855 | 1.00 | 36.42 | A | C |
| ATOM | 1687 | C | ALA | A | 248 | 22.737 | 50.853 | 51.662 | 1.00 | 21.61 | A | C |
| ATOM | 1688 | O | ALA | A | 248 | 22.917 | 52.060 | 51.782 | 1.00 | 21.61 | A | O |
| ATOM | 1689 | N | GLU | A | 249 | 23.573 | 49.961 | 52.174 | 1.00 | 14.50 | A | N |
| ATOM | 1690 | CA | GLU | A | 249 | 24.748 | 50.408 | 52.893 | 1.00 | 14.50 | A | C |
| ATOM | 1691 | CB | GLU | A | 249 | 25.335 | 49.252 | 53.700 | 1.00 | 22.79 | A | C |
| ATOM | 1692 | CG | GLU | A | 249 | 26.394 | 49.700 | 54.716 | 1.00 | 22.79 | A | C |
| ATOM | 1693 | CD | GLU | A | 249 | 26.916 | 48.574 | 55.551 | 1.00 | 22.79 | A | C |
| ATOM | 1694 | OE1 | GLU | A | 249 | 26.088 | 47.844 | 56.104 | 1.00 | 22.79 | A | O |
| ATOM | 1695 | OE2 | GLU | A | 249 | 28.140 | 48.417 | 55.676 | 1.00 | 22.79 | A | O |
| ATOM | 1696 | C | GLU | A | 249 | 25.799 | 50.980 | 51.943 | 1.00 | 14.50 | A | C |
| ATOM | 1697 | O | GLU | A | 249 | 26.478 | 51.950 | 52.274 | 1.00 | 14.50 | A | O |
| ATOM | 1698 | N | LEU | A | 250 | 25.951 | 50.374 | 50.770 | 1.00 | 32.33 | A | N |
| ATOM | 1699 | CA | LEU | A | 250 | 26.921 | 50.870 | 49.807 | 1.00 | 32.33 | A | C |
| ATOM | 1700 | CB | LEU | A | 250 | 27.186 | 49.830 | 48.729 | 1.00 | 22.86 | A | C |
| ATOM | 1701 | CG | LEU | A | 250 | 27.965 | 48.601 | 49.188 | 1.00 | 22.86 | A | C |
| ATOM | 1702 | CD1 | LEU | A | 250 | 28.239 | 47.715 | 47.984 | 1.00 | 22.86 | A | C |
| ATOM | 1703 | CD2 | LEU | A | 250 | 29.263 | 49.024 | 49.850 | 1.00 | 22.86 | A | C |
| ATOM | 1704 | C | LEU | A | 250 | 26.453 | 52.172 | 49.172 | 1.00 | 32.33 | A | C |
| ATOM | 1705 | O | LEU | A | 250 | 27.262 | 52.972 | 48.720 | 1.00 | 32.33 | A | O |
| ATOM | 1706 | N | LEU | A | 251 | 25.148 | 52.396 | 49.152 | 1.00 | 27.55 | A | N |
| ATOM | 1707 | CA | LEU | A | 251 | 24.616 | 53.618 | 48.578 | 1.00 | 27.55 | A | C |
| ATOM | 1708 | CB | LEU | A | 251 | 23.194 | 53.386 | 48.082 | 1.00 | 17.54 | A | C |
| ATOM | 1709 | CG | LEU | A | 251 | 23.072 | 52.482 | 46.858 | 1.00 | 17.54 | A | C |
| ATOM | 1710 | CD1 | LEU | A | 251 | 21.630 | 52.098 | 46.653 | 1.00 | 17.54 | A | C |
| ATOM | 1711 | CD2 | LEU | A | 251 | 23.619 | 53.177 | 45.652 | 1.00 | 17.54 | A | C |
| ATOM | 1712 | C | LEU | A | 251 | 24.628 | 54.760 | 49.585 | 1.00 | 27.55 | A | C |
| ATOM | 1713 | O | LEU | A | 251 | 24.774 | 55.915 | 49.205 | 1.00 | 27.55 | A | O |
| ATOM | 1714 | N | LEU | A | 252 | 24.469 | 54.444 | 50.864 | 1.00 | 22.97 | A | N |
| ATOM | 1715 | CA | LEU | A | 252 | 24.463 | 55.466 | 51.904 | 1.00 | 22.97 | A | C |
| ATOM | 1716 | CB | LEU | A | 252 | 23.631 | 55.018 | 53.090 | 1.00 | 46.75 | A | C |
| ATOM | 1717 | CG | LEU | A | 252 | 22.116 | 54.952 | 52.963 | 1.00 | 46.75 | A | C |
| ATOM | 1718 | CD1 | LEU | A | 252 | 21.518 | 54.664 | 54.363 | 1.00 | 46.75 | A | C |
| ATOM | 1719 | CD2 | LEU | A | 252 | 21.581 | 56.270 | 52.419 | 1.00 | 46.75 | A | C |
| ATOM | 1720 | C | LEU | A | 252 | 25.833 | 55.808 | 52.443 | 1.00 | 22.97 | A | C |
| ATOM | 1721 | O | LEU | A | 252 | 26.072 | 56.934 | 52.856 | 1.00 | 22.97 | A | O |
| ATOM | 1722 | N | GLY | A | 253 | 26.722 | 54.823 | 52.480 | 1.00 | 31.05 | A | N |
| ATOM | 1723 | CA | GLY | A | 253 | 28.050 | 55.065 | 53.010 | 1.00 | 31.05 | A | C |
| ATOM | 1724 | C | GLY | A | 253 | 28.061 | 54.769 | 54.502 | 1.00 | 31.05 | A | C |
| ATOM | 1725 | O | GLY | A | 253 | 28.958 | 55.177 | 55.240 | 1.00 | 31.05 | A | O |
| ATOM | 1726 | N | GLN | A | 254 | 27.036 | 54.054 | 54.948 | 1.00 | 35.51 | A | N |
| ATOM | 1727 | CA | GLN | A | 254 | 26.913 | 53.659 | 56.343 | 1.00 | 35.51 | A | C |
| ATOM | 1728 | CB | GLN | A | 254 | 26.691 | 54.884 | 57.222 | 1.00 | 42.91 | A | C |
| ATOM | 1729 | CG | GLN | A | 254 | 25.430 | 55.640 | 56.885 | 1.00 | 42.91 | A | C |
| ATOM | 1730 | CD | GLN | A | 254 | 25.292 | 56.945 | 57.658 | 1.00 | 42.91 | A | C |
| ATOM | 1731 | OE1 | GLN | A | 254 | 25.267 | 56.958 | 58.897 | 1.00 | 42.91 | A | O |
| ATOM | 1732 | NE2 | GLN | A | 254 | 25.202 | 58.058 | 56.921 | 1.00 | 42.91 | A | N |
| ATOM | 1733 | C | GLN | A | 254 | 25.717 | 52.724 | 56.439 | 1.00 | 35.51 | A | C |
| ATOM | 1734 | O | GLN | A | 254 | 24.848 | 52.740 | 55.574 | 1.00 | 35.51 | A | O |
| ATOM | 1735 | N | PRO | A | 255 | 25.655 | 51.893 | 57.488 | 1.00 | 21.73 | A | N |
| ATOM | 1736 | CD | PRO | A | 255 | 26.644 | 51.685 | 58.555 | 1.00 | 22.30 | A | C |

| | | | | | | | | | | | | |
|---|---|---|---|---|---|---|---|---|---|---|---|---|
| ATOM | 1737 | CA | PRO | A | 255 | 24.540 | 50.967 | 57.644 | 1.00 | 21.73 | A | C |
| ATOM | 1738 | CB | PRO | A | 255 | 24.845 | 50.292 | 58.976 | 1.00 | 22.30 | A | C |
| ATOM | 1739 | CG | PRO | A | 255 | 26.318 | 50.295 | 59.015 | 1.00 | 22.30 | A | C |
| ATOM | 1740 | C | PRO | A | 255 | 23.210 | 51.686 | 57.660 | 1.00 | 21.73 | A | C |
| ATOM | 1741 | O | PRO | A | 255 | 23.096 | 52.778 | 58.213 | 1.00 | 21.73 | A | O |
| ATOM | 1742 | N | ILE | A | 256 | 22.207 | 51.069 | 57.046 | 1.00 | 26.92 | A | N |
| ATOM | 1743 | CA | ILE | A | 256 | 20.874 | 51.646 | 57.005 | 1.00 | 26.92 | A | C |
| ATOM | 1744 | CB | ILE | A | 256 | 20.133 | 51.232 | 55.692 | 1.00 | 21.17 | A | C |
| ATOM | 1745 | CG2 | ILE | A | 256 | 19.915 | 49.719 | 55.649 | 1.00 | 21.17 | A | C |
| ATOM | 1746 | CG1 | ILE | A | 256 | 18.811 | 52.001 | 55.578 | 1.00 | 21.17 | A | C |
| ATOM | 1747 | CD1 | ILE | A | 256 | 18.171 | 51.956 | 54.216 | 1.00 | 21.17 | A | C |
| ATOM | 1748 | C | ILE | A | 256 | 20.022 | 51.308 | 58.243 | 1.00 | 26.92 | A | C |
| ATOM | 1749 | O | ILE | A | 256 | 19.204 | 52.121 | 58.660 | 1.00 | 26.92 | A | O |
| ATOM | 1750 | N | PHE | A | 257 | 20.218 | 50.135 | 58.846 | 1.00 | 26.77 | A | N |
| ATOM | 1751 | CA | PHE | A | 257 | 19.439 | 49.760 | 60.033 | 1.00 | 26.77 | A | C |
| ATOM | 1752 | CB | PHE | A | 257 | 18.498 | 48.605 | 59.695 | 1.00 | 27.68 | A | C |
| ATOM | 1753 | CG | PHE | A | 257 | 17.563 | 48.902 | 58.575 | 1.00 | 27.68 | A | C |
| ATOM | 1754 | CD1 | PHE | A | 257 | 16.850 | 50.097 | 58.541 | 1.00 | 27.68 | A | C |
| ATOM | 1755 | CD2 | PHE | A | 257 | 17.398 | 47.997 | 57.541 | 1.00 | 27.68 | A | C |
| ATOM | 1756 | CE1 | PHE | A | 257 | 15.985 | 50.390 | 57.482 | 1.00 | 27.68 | A | C |
| ATOM | 1757 | CE2 | PHE | A | 257 | 16.533 | 48.279 | 56.475 | 1.00 | 27.68 | A | C |
| ATOM | 1758 | CZ | PHE | A | 257 | 15.824 | 49.481 | 56.447 | 1.00 | 27.68 | A | C |
| ATOM | 1759 | C | PHE | A | 257 | 20.285 | 49.384 | 61.261 | 1.00 | 26.77 | A | C |
| ATOM | 1760 | O | PHE | A | 257 | 20.221 | 48.256 | 61.761 | 1.00 | 26.77 | A | O |
| ATOM | 1761 | N | PRO | A | 258 | 21.081 | 50.336 | 61.774 | 1.00 | 31.39 | A | N |
| ATOM | 1762 | CD | PRO | A | 258 | 21.240 | 51.711 | 61.268 | 1.00 | 27.63 | A | C |
| ATOM | 1763 | CA | PRO | A | 258 | 21.941 | 50.126 | 62.937 | 1.00 | 31.39 | A | C |
| ATOM | 1764 | CB | PRO | A | 258 | 22.845 | 51.350 | 62.901 | 1.00 | 27.63 | A | C |
| ATOM | 1765 | CG | PRO | A | 258 | 21.933 | 52.396 | 62.422 | 1.00 | 27.63 | A | C |
| ATOM | 1766 | C | PRO | A | 258 | 21.154 | 50.014 | 64.238 | 1.00 | 31.39 | A | C |
| ATOM | 1767 | O | PRO | A | 258 | 19.919 | 50.046 | 64.235 | 1.00 | 31.39 | A | O |
| ATOM | 1768 | N | GLY | A | 259 | 21.887 | 49.876 | 65.342 | 1.00 | 33.64 | A | N |
| ATOM | 1769 | CA | GLY | A | 259 | 21.282 | 49.762 | 66.655 | 1.00 | 33.64 | A | C |
| ATOM | 1770 | C | GLY | A | 259 | 22.026 | 48.716 | 67.443 | 1.00 | 33.64 | A | C |
| ATOM | 1771 | O | GLY | A | 259 | 22.488 | 47.729 | 66.872 | 1.00 | 33.64 | A | O |
| ATOM | 1772 | N | ASP | A | 260 | 22.163 | 48.933 | 68.745 | 1.00 | 27.12 | A | N |
| ATOM | 1773 | CA | ASP | A | 260 | 22.862 | 47.988 | 69.601 | 1.00 | 27.12 | A | C |
| ATOM | 1774 | CB | ASP | A | 260 | 23.556 | 48.721 | 70.753 | 1.00 | 43.49 | A | C |
| ATOM | 1775 | CG | ASP | A | 260 | 24.769 | 49.514 | 70.301 | 1.00 | 43.49 | A | C |
| ATOM | 1776 | OD1 | ASP | A | 260 | 25.100 | 49.452 | 69.097 | 1.00 | 43.49 | A | O |
| ATOM | 1777 | OD2 | ASP | A | 260 | 25.390 | 50.198 | 71.149 | 1.00 | 43.49 | A | O |
| ATOM | 1778 | C | ASP | A | 260 | 21.881 | 46.986 | 70.171 | 1.00 | 27.12 | A | C |
| ATOM | 1779 | O | ASP | A | 260 | 22.261 | 46.114 | 70.935 | 1.00 | 27.12 | A | O |
| ATOM | 1780 | N | SER | A | 261 | 20.615 | 47.109 | 69.800 | 1.00 | 19.99 | A | N |
| ATOM | 1781 | CA | SER | A | 261 | 19.600 | 46.201 | 70.315 | 1.00 | 19.99 | A | C |
| ATOM | 1782 | CB | SER | A | 261 | 18.931 | 46.794 | 71.548 | 1.00 | 13.89 | A | C |
| ATOM | 1783 | OG | SER | A | 261 | 18.027 | 47.820 | 71.174 | 1.00 | 13.89 | A | O |
| ATOM | 1784 | C | SER | A | 261 | 18.544 | 46.001 | 69.259 | 1.00 | 19.99 | A | C |
| ATOM | 1785 | O | SER | A | 261 | 18.383 | 46.837 | 68.378 | 1.00 | 19.99 | A | O |
| ATOM | 1786 | N | GLY | A | 262 | 17.818 | 44.895 | 69.367 | 1.00 | 32.23 | A | N |
| ATOM | 1787 | CA | GLY | A | 262 | 16.765 | 44.608 | 68.414 | 1.00 | 32.23 | A | C |
| ATOM | 1788 | C | GLY | A | 262 | 15.830 | 45.794 | 68.401 | 1.00 | 32.23 | A | C |
| ATOM | 1789 | O | GLY | A | 262 | 15.483 | 46.329 | 67.354 | 1.00 | 32.23 | A | O |
| ATOM | 1790 | N | VAL | A | 263 | 15.435 | 46.224 | 69.586 | 1.00 | 26.96 | A | N |
| ATOM | 1791 | CA | VAL | A | 263 | 14.545 | 47.361 | 69.716 | 1.00 | 26.96 | A | C |
| ATOM | 1792 | CB | VAL | A | 263 | 14.408 | 47.765 | 71.199 | 1.00 | 25.92 | A | C |
| ATOM | 1793 | CG1 | VAL | A | 263 | 13.398 | 48.885 | 71.363 | 1.00 | 25.92 | A | C |
| ATOM | 1794 | CG2 | VAL | A | 263 | 13.981 | 46.566 | 72.004 | 1.00 | 25.92 | A | C |
| ATOM | 1795 | C | VAL | A | 263 | 15.039 | 48.546 | 68.890 | 1.00 | 26.96 | A | C |
| ATOM | 1796 | O | VAL | A | 263 | 14.293 | 49.099 | 68.093 | 1.00 | 26.96 | A | O |
| ATOM | 1797 | N | ASP | A | 264 | 16.291 | 48.939 | 69.074 | 1.00 | 24.07 | A | N |
| ATOM | 1798 | CA | ASP | A | 264 | 16.811 | 50.060 | 68.308 | 1.00 | 24.07 | A | C |
| ATOM | 1799 | CB | ASP | A | 264 | 18.259 | 50.341 | 68.693 | 1.00 | 43.34 | A | C |
| ATOM | 1800 | CG | ASP | A | 264 | 18.376 | 50.971 | 70.056 | 1.00 | 43.34 | A | C |
| ATOM | 1801 | OD1 | ASP | A | 264 | 17.547 | 51.847 | 70.359 | 1.00 | 43.34 | A | O |
| ATOM | 1802 | OD2 | ASP | A | 264 | 19.293 | 50.614 | 70.820 | 1.00 | 43.34 | A | O |
| ATOM | 1803 | C | ASP | A | 264 | 16.711 | 49.795 | 66.817 | 1.00 | 24.07 | A | C |

| | | | | | | | | | | | | |
|---|---|---|---|---|---|---|---|---|---|---|---|---|
| ATOM | 1804 | O | ASP | A | 264 | 16.216 | 50.628 | 66.061 | 1.00 | 24.07 | A | O |
| ATOM | 1805 | N | GLN | A | 265 | 17.176 | 48.625 | 66.396 | 1.00 | 28.99 | A | N |
| ATOM | 1806 | CA | GLN | A | 265 | 17.133 | 48.254 | 64.992 | 1.00 | 28.99 | A | C |
| ATOM | 1807 | CB | GLN | A | 265 | 17.590 | 46.812 | 64.835 | 1.00 | 25.25 | A | C |
| ATOM | 1808 | CG | GLN | A | 265 | 19.036 | 46.622 | 65.224 | 1.00 | 25.25 | A | C |
| ATOM | 1809 | CD | GLN | A | 265 | 19.470 | 45.186 | 65.155 | 1.00 | 25.25 | A | C |
| ATOM | 1810 | OE1 | GLN | A | 265 | 19.273 | 44.531 | 64.139 | 1.00 | 25.25 | A | O |
| ATOM | 1811 | NE2 | GLN | A | 265 | 20.076 | 44.683 | 66.231 | 1.00 | 25.25 | A | N |
| ATOM | 1812 | C | GLN | A | 265 | 15.721 | 48.422 | 64.454 | 1.00 | 28.99 | A | C |
| ATOM | 1813 | O | GLN | A | 265 | 15.507 | 49.064 | 63.428 | 1.00 | 28.99 | A | O |
| ATOM | 1814 | N | LEU | A | 266 | 14.747 | 47.853 | 65.149 | 1.00 | 28.89 | A | N |
| ATOM | 1815 | CA | LEU | A | 266 | 13.371 | 47.965 | 64.694 | 1.00 | 28.89 | A | C |
| ATOM | 1816 | CB | LEU | A | 266 | 12.426 | 47.221 | 65.631 | 1.00 | 13.31 | A | C |
| ATOM | 1817 | CG | LEU | A | 266 | 12.087 | 45.808 | 65.177 | 1.00 | 13.31 | A | C |
| ATOM | 1818 | CD1 | LEU | A | 266 | 13.351 | 45.047 | 64.912 | 1.00 | 13.31 | A | C |
| ATOM | 1819 | CD2 | LEU | A | 266 | 11.255 | 45.128 | 66.232 | 1.00 | 13.31 | A | C |
| ATOM | 1820 | C | LEU | A | 266 | 12.932 | 49.411 | 64.551 | 1.00 | 28.89 | A | C |
| ATOM | 1821 | O | LEU | A | 266 | 12.168 | 49.745 | 63.649 | 1.00 | 28.89 | A | O |
| ATOM | 1822 | N | VAL | A | 267 | 13.419 | 50.271 | 65.440 | 1.00 | 31.51 | A | N |
| ATOM | 1823 | CA | VAL | A | 267 | 13.069 | 51.688 | 65.387 | 1.00 | 31.51 | A | C |
| ATOM | 1824 | CB | VAL | A | 267 | 13.753 | 52.489 | 66.526 | 1.00 | 22.59 | A | C |
| ATOM | 1825 | CG1 | VAL | A | 267 | 13.833 | 53.974 | 66.167 | 1.00 | 22.59 | A | C |
| ATOM | 1826 | CG2 | VAL | A | 267 | 13.011 | 52.293 | 67.815 | 1.00 | 22.59 | A | C |
| ATOM | 1827 | C | VAL | A | 267 | 13.599 | 52.197 | 64.059 | 1.00 | 31.51 | A | C |
| ATOM | 1828 | O | VAL | A | 267 | 12.892 | 52.858 | 63.301 | 1.00 | 31.51 | A | O |
| ATOM | 1829 | N | GLU | A | 268 | 14.858 | 51.887 | 63.781 | 1.00 | 36.26 | A | N |
| ATOM | 1830 | CA | GLU | A | 268 | 15.457 | 52.321 | 62.534 | 1.00 | 36.26 | A | C |
| ATOM | 1831 | CB | GLU | A | 268 | 16.943 | 51.973 | 62.501 | 1.00 | 38.51 | A | C |
| ATOM | 1832 | CG | GLU | A | 268 | 17.803 | 52.925 | 63.291 | 1.00 | 38.51 | A | C |
| ATOM | 1833 | CD | GLU | A | 268 | 17.452 | 54.382 | 63.005 | 1.00 | 38.51 | A | C |
| ATOM | 1834 | OE1 | GLU | A | 268 | 17.225 | 54.740 | 61.826 | 1.00 | 38.51 | A | O |
| ATOM | 1835 | OE2 | GLU | A | 268 | 17.405 | 55.180 | 63.960 | 1.00 | 38.51 | A | O |
| ATOM | 1836 | C | GLU | A | 268 | 14.767 | 51.759 | 61.291 | 1.00 | 36.26 | A | C |
| ATOM | 1837 | O | GLU | A | 268 | 14.734 | 52.419 | 60.260 | 1.00 | 36.26 | A | O |
| ATOM | 1838 | N | ILE | A | 269 | 14.225 | 50.545 | 61.377 | 1.00 | 28.41 | A | N |
| ATOM | 1839 | CA | ILE | A | 269 | 13.534 | 49.941 | 60.240 | 1.00 | 28.41 | A | C |
| ATOM | 1840 | CB | ILE | A | 269 | 13.335 | 48.435 | 60.450 | 1.00 | 16.63 | A | C |
| ATOM | 1841 | CG2 | ILE | A | 269 | 12.459 | 47.854 | 59.362 | 1.00 | 16.63 | A | C |
| ATOM | 1842 | CG1 | ILE | A | 269 | 14.680 | 47.729 | 60.418 | 1.00 | 16.63 | A | C |
| ATOM | 1843 | CD1 | ILE | A | 269 | 14.547 | 46.221 | 60.498 | 1.00 | 16.63 | A | C |
| ATOM | 1844 | C | ILE | A | 269 | 12.164 | 50.585 | 60.030 | 1.00 | 28.41 | A | C |
| ATOM | 1845 | O | ILE | A | 269 | 11.756 | 50.833 | 58.898 | 1.00 | 28.41 | A | O |
| ATOM | 1846 | N | ILE | A | 270 | 11.452 | 50.840 | 61.126 | 1.00 | 36.68 | A | N |
| ATOM | 1847 | CA | ILE | A | 270 | 10.138 | 51.468 | 61.054 | 1.00 | 36.68 | A | C |
| ATOM | 1848 | CB | ILE | A | 270 | 9.433 | 51.511 | 62.424 | 1.00 | 20.94 | A | C |
| ATOM | 1849 | CG2 | ILE | A | 270 | 8.140 | 52.278 | 62.309 | 1.00 | 20.94 | A | C |
| ATOM | 1850 | CG1 | ILE | A | 270 | 9.140 | 50.089 | 62.902 | 1.00 | 20.94 | A | C |
| ATOM | 1851 | CD1 | ILE | A | 270 | 8.451 | 50.012 | 64.218 | 1.00 | 20.94 | A | C |
| ATOM | 1852 | C | ILE | A | 270 | 10.297 | 52.899 | 60.552 | 1.00 | 36.68 | A | C |
| ATOM | 1853 | O | ILE | A | 270 | 9.492 | 53.374 | 59.749 | 1.00 | 36.68 | A | O |
| ATOM | 1854 | N | LYS | A | 271 | 11.339 | 53.582 | 61.020 | 1.00 | 33.68 | A | N |
| ATOM | 1855 | CA | LYS | A | 271 | 11.580 | 54.956 | 60.601 | 1.00 | 33.68 | A | C |
| ATOM | 1856 | CB | LYS | A | 271 | 12.961 | 55.459 | 61.063 | 1.00 | 45.14 | A | C |
| ATOM | 1857 | CG | LYS | A | 271 | 13.039 | 55.996 | 62.489 | 1.00 | 45.14 | A | C |
| ATOM | 1858 | CD | LYS | A | 271 | 14.379 | 56.743 | 62.738 | 1.00 | 45.14 | A | C |
| ATOM | 1859 | CE | LYS | A | 271 | 14.508 | 57.270 | 64.191 | 1.00 | 45.14 | A | C |
| ATOM | 1860 | NZ | LYS | A | 271 | 15.708 | 58.166 | 64.393 | 1.00 | 45.14 | A | N |
| ATOM | 1861 | C | LYS | A | 271 | 11.494 | 55.075 | 59.078 | 1.00 | 33.68 | A | C |
| ATOM | 1862 | O | LYS | A | 271 | 11.046 | 56.098 | 58.562 | 1.00 | 33.68 | A | O |
| ATOM | 1863 | N | VAL | A | 272 | 11.923 | 54.039 | 58.358 | 1.00 | 44.43 | A | N |
| ATOM | 1864 | CA | VAL | A | 272 | 11.882 | 54.065 | 56.892 | 1.00 | 44.43 | A | C |
| ATOM | 1865 | CB | VAL | A | 272 | 13.131 | 53.441 | 56.274 | 1.00 | 50.48 | A | C |
| ATOM | 1866 | CG1 | VAL | A | 272 | 14.290 | 54.402 | 56.360 | 1.00 | 50.48 | A | C |
| ATOM | 1867 | CG2 | VAL | A | 272 | 13.461 | 52.153 | 57.002 | 1.00 | 50.48 | A | C |
| ATOM | 1868 | C | VAL | A | 272 | 10.671 | 53.325 | 56.327 | 1.00 | 44.43 | A | C |
| ATOM | 1869 | O | VAL | A | 272 | 9.790 | 53.942 | 55.721 | 1.00 | 44.43 | A | O |
| ATOM | 1870 | N | LEU | A | 273 | 10.632 | 52.009 | 56.524 | 1.00 | 29.32 | A | N |

| | | | | | | | | | | | | |
|---|---|---|---|---|---|---|---|---|---|---|---|---|
| ATOM | 1871 | CA | LEU | A | 273 | 9.520 | 51.195 | 56.039 | 1.00 | 29.32 | A | C |
| ATOM | 1872 | CB | LEU | A | 273 | 9.745 | 49.715 | 56.360 | 1.00 | 22.66 | A | C |
| ATOM | 1873 | CG | LEU | A | 273 | 10.903 | 49.014 | 55.673 | 1.00 | 22.66 | A | C |
| ATOM | 1874 | CD1 | LEU | A | 273 | 10.648 | 47.523 | 55.789 | 1.00 | 22.66 | A | C |
| ATOM | 1875 | CD2 | LEU | A | 273 | 10.992 | 49.431 | 54.213 | 1.00 | 22.66 | A | C |
| ATOM | 1876 | C | LEU | A | 273 | 8.155 | 51.600 | 56.594 | 1.00 | 29.32 | A | C |
| ATOM | 1877 | O | LEU | A | 273 | 7.129 | 51.378 | 55.953 | 1.00 | 29.32 | A | O |
| ATOM | 1878 | N | GLY | A | 274 | 8.149 | 52.204 | 57.776 | 1.00 | 52.62 | A | N |
| ATOM | 1879 | CA | GLY | A | 274 | 6.898 | 52.579 | 58.401 | 1.00 | 52.62 | A | C |
| ATOM | 1880 | C | GLY | A | 274 | 6.430 | 51.450 | 59.309 | 1.00 | 52.62 | A | C |
| ATOM | 1881 | O | GLY | A | 274 | 6.860 | 50.294 | 59.194 | 1.00 | 52.62 | A | O |
| ATOM | 1882 | N | THR | A | 275 | 5.556 | 51.788 | 60.241 | 1.00 | 43.83 | A | N |
| ATOM | 1883 | CA | THR | A | 275 | 5.012 | 50.801 | 61.161 | 1.00 | 43.83 | A | C |
| ATOM | 1884 | CB | THR | A | 275 | 3.924 | 51.463 | 62.067 | 1.00 | 45.06 | A | C |
| ATOM | 1885 | OG1 | THR | A | 275 | 4.505 | 52.558 | 62.792 | 1.00 | 45.06 | A | O |
| ATOM | 1886 | CG2 | THR | A | 275 | 3.343 | 50.466 | 63.050 | 1.00 | 45.06 | A | C |
| ATOM | 1887 | C | THR | A | 275 | 4.397 | 49.605 | 60.407 | 1.00 | 43.83 | A | C |
| ATOM | 1888 | O | THR | A | 275 | 3.602 | 49.752 | 59.473 | 1.00 | 43.83 | A | O |
| ATOM | 1889 | N | PRO | A | 276 | 4.761 | 48.395 | 60.820 | 1.00 | 45.90 | A | N |
| ATOM | 1890 | CD | PRO | A | 276 | 5.700 | 48.063 | 61.902 | 1.00 | 40.12 | A | C |
| ATOM | 1891 | CA | PRO | A | 276 | 4.234 | 47.203 | 60.170 | 1.00 | 45.90 | A | C |
| ATOM | 1892 | CB | PRO | A | 276 | 5.203 | 46.109 | 60.599 | 1.00 | 40.12 | A | C |
| ATOM | 1893 | CG | PRO | A | 276 | 5.687 | 46.547 | 61.925 | 1.00 | 40.12 | A | C |
| ATOM | 1894 | C | PRO | A | 276 | 2.800 | 46.885 | 60.543 | 1.00 | 45.90 | A | C |
| ATOM | 1895 | O | PRO | A | 276 | 2.426 | 46.822 | 61.726 | 1.00 | 45.90 | A | O |
| ATOM | 1896 | N | THR | A | 277 | 2.004 | 46.676 | 59.502 | 1.00 | 49.26 | A | N |
| ATOM | 1897 | CA | THR | A | 277 | 0.611 | 46.343 | 59.673 | 1.00 | 49.26 | A | C |
| ATOM | 1898 | CB | THR | A | 277 | -0.103 | 46.229 | 58.321 | 1.00 | 42.51 | A | C |
| ATOM | 1899 | OG1 | THR | A | 277 | 0.278 | 45.003 | 57.678 | 1.00 | 42.51 | A | O |
| ATOM | 1900 | CG2 | THR | A | 277 | 0.282 | 47.422 | 57.430 | 1.00 | 42.51 | A | C |
| ATOM | 1901 | C | THR | A | 277 | 0.552 | 45.012 | 60.389 | 1.00 | 49.26 | A | C |
| ATOM | 1902 | O | THR | A | 277 | 1.421 | 44.161 | 60.209 | 1.00 | 49.26 | A | O |
| ATOM | 1903 | N | ALA | A | 278 | -0.461 | 44.845 | 61.224 | 1.00 | 47.32 | A | N |
| ATOM | 1904 | CA | ALA | A | 278 | -0.619 | 43.611 | 61.984 | 1.00 | 47.32 | A | C |
| ATOM | 1905 | CB | ALA | A | 278 | -1.938 | 43.632 | 62.740 | 0.00 | 34.21 | A | C |
| ATOM | 1906 | C | ALA | A | 278 | -0.548 | 42.388 | 61.071 | 1.00 | 47.32 | A | C |
| ATOM | 1907 | O | ALA | A | 278 | 0.058 | 41.358 | 61.416 | 1.00 | 47.32 | A | O |
| ATOM | 1908 | N | GLU | A | 279 | -1.163 | 42.501 | 59.896 | 1.00 | 66.97 | A | N |
| ATOM | 1909 | CA | GLU | A | 279 | -1.133 | 41.381 | 58.962 | 1.00 | 66.97 | A | C |
| ATOM | 1910 | CB | GLU | A | 279 | -1.981 | 41.682 | 57.708 | 1.00 | 80.79 | A | C |
| ATOM | 1911 | CG | GLU | A | 279 | -2.704 | 40.441 | 57.168 | 1.00 | 80.79 | A | C |
| ATOM | 1912 | CD | GLU | A | 279 | -1.851 | 39.169 | 57.321 | 1.00 | 80.79 | A | C |
| ATOM | 1913 | OE1 | GLU | A | 279 | -0.810 | 39.062 | 56.608 | 1.00 | 80.79 | A | O |
| ATOM | 1914 | OE2 | GLU | A | 279 | -2.227 | 38.295 | 58.157 | 1.00 | 80.79 | A | O |
| ATOM | 1915 | C | GLU | A | 279 | 0.345 | 41.130 | 58.581 | 1.00 | 66.97 | A | C |
| ATOM | 1916 | O | GLU | A | 279 | 0.826 | 39.982 | 58.536 | 1.00 | 66.97 | A | O |
| ATOM | 1917 | N | GLN | A | 280 | 1.067 | 42.214 | 58.305 | 1.00 | 52.80 | A | N |
| ATOM | 1918 | CA | GLN | A | 280 | 2.483 | 42.106 | 57.945 | 1.00 | 52.80 | A | C |
| ATOM | 1919 | CB | GLN | A | 280 | 3.084 | 43.505 | 57.724 | 1.00 | 41.24 | A | C |
| ATOM | 1920 | CG | GLN | A | 280 | 3.085 | 43.971 | 56.274 | 1.00 | 41.24 | A | C |
| ATOM | 1921 | CD | GLN | A | 280 | 3.436 | 45.440 | 56.141 | 1.00 | 41.24 | A | C |
| ATOM | 1922 | OE1 | GLN | A | 280 | 3.956 | 45.868 | 55.107 | 1.00 | 41.24 | A | O |
| ATOM | 1923 | NE2 | GLN | A | 280 | 3.149 | 46.228 | 57.187 | 1.00 | 41.24 | A | N |
| ATOM | 1924 | C | GLN | A | 280 | 3.269 | 41.361 | 59.031 | 1.00 | 52.80 | A | C |
| ATOM | 1925 | O | GLN | A | 280 | 4.145 | 40.520 | 58.742 | 1.00 | 52.80 | A | O |
| ATOM | 1926 | N | ILE | A | 281 | 2.938 | 41.652 | 60.286 | 1.00 | 55.19 | A | N |
| ATOM | 1927 | CA | ILE | A | 281 | 3.627 | 41.010 | 61.387 | 1.00 | 55.19 | A | C |
| ATOM | 1928 | CB | ILE | A | 281 | 3.108 | 41.511 | 62.729 | 1.00 | 62.37 | A | C |
| ATOM | 1929 | CG2 | ILE | A | 281 | 3.932 | 40.894 | 63.852 | 1.00 | 62.37 | A | C |
| ATOM | 1930 | CG1 | ILE | A | 281 | 3.166 | 43.042 | 62.752 | 1.00 | 62.37 | A | C |
| ATOM | 1931 | CD1 | ILE | A | 281 | 3.047 | 43.656 | 64.137 | 1.00 | 62.37 | A | C |
| ATOM | 1932 | C | ILE | A | 281 | 3.462 | 39.501 | 61.333 | 1.00 | 55.19 | A | C |
| ATOM | 1933 | O | ILE | A | 281 | 4.336 | 38.750 | 61.793 | 1.00 | 55.19 | A | O |
| ATOM | 1934 | N | ALA | A | 282 | 2.340 | 39.061 | 60.771 | 1.00 | 46.44 | A | N |
| ATOM | 1935 | CA | ALA | A | 282 | 2.066 | 37.637 | 60.669 | 1.00 | 46.44 | A | C |
| ATOM | 1936 | CB | ALA | A | 282 | 0.815 | 37.396 | 59.856 | 1.00 | 74.25 | A | C |
| ATOM | 1937 | C | ALA | A | 282 | 3.247 | 36.936 | 60.025 | 1.00 | 46.44 | A | C |

| | | | | | | | | | | | | |
|---|---|---|---|---|---|---|---|---|---|---|---|---|
| ATOM | 1938 | O | ALA | A | 282 | 3.868 | 36.064 | 60.647 | 1.00 | 46.44 | A | O |
| ATOM | 1939 | N | GLU | A | 283 | 3.565 | 37.322 | 58.785 | 1.00 | 68.04 | A | N |
| ATOM | 1940 | CA | GLU | A | 283 | 4.686 | 36.709 | 58.061 | 1.00 | 68.04 | A | C |
| ATOM | 1941 | CB | GLU | A | 283 | 4.932 | 37.416 | 56.726 | 0.00 | 68.87 | A | C |
| ATOM | 1942 | CG | GLU | A | 283 | 3.849 | 37.149 | 55.707 | 0.00 | 68.87 | A | C |
| ATOM | 1943 | CD | GLU | A | 283 | 2.694 | 38.121 | 55.826 | 1.00 | 68.87 | A | C |
| ATOM | 1944 | OE1 | GLU | A | 283 | 2.213 | 38.357 | 56.963 | 1.00 | 68.87 | A | O |
| ATOM | 1945 | OE2 | GLU | A | 283 | 2.254 | 38.636 | 54.772 | 1.00 | 68.87 | A | O |
| ATOM | 1946 | C | GLU | A | 283 | 5.970 | 36.689 | 58.898 | 1.00 | 68.04 | A | C |
| ATOM | 1947 | O | GLU | A | 283 | 6.839 | 35.816 | 58.715 | 1.00 | 68.04 | A | O |
| ATOM | 1948 | N | MET | A | 284 | 6.076 | 37.643 | 59.829 | 1.00 | 62.97 | A | N |
| ATOM | 1949 | CA | MET | A | 284 | 7.228 | 37.702 | 60.722 | 1.00 | 62.97 | A | C |
| ATOM | 1950 | CB | MET | A | 284 | 7.294 | 39.043 | 61.460 | 1.00 | 52.89 | A | C |
| ATOM | 1951 | CG | MET | A | 284 | 8.474 | 39.872 | 61.011 | 1.00 | 52.89 | A | C |
| ATOM | 1952 | SD | MET | A | 284 | 8.146 | 40.539 | 59.380 | 1.00 | 52.89 | A | S |
| ATOM | 1953 | CE | MET | A | 284 | 8.041 | 42.227 | 59.852 | 1.00 | 52.89 | A | C |
| ATOM | 1954 | C | MET | A | 284 | 7.182 | 36.560 | 61.739 | 1.00 | 62.97 | A | C |
| ATOM | 1955 | O | MET | A | 284 | 8.127 | 35.774 | 61.858 | 1.00 | 62.97 | A | O |
| ATOM | 1956 | N | ALA | A | 290 | 3.093 | 54.291 | 64.785 | 1.00 | 46.31 | A | N |
| ATOM | 1957 | CA | ALA | A | 290 | 2.842 | 55.103 | 63.603 | 1.00 | 46.31 | A | C |
| ATOM | 1958 | CB | ALA | A | 290 | 2.124 | 56.351 | 64.007 | 1.00 | 24.08 | A | C |
| ATOM | 1959 | C | ALA | A | 290 | 4.141 | 55.463 | 62.861 | 1.00 | 46.31 | A | C |
| ATOM | 1960 | O | ALA | A | 290 | 5.217 | 55.567 | 63.470 | 1.00 | 46.31 | A | O |
| ATOM | 1961 | N | ALA | A | 291 | 4.039 | 55.653 | 61.544 | 1.00 | 76.21 | A | N |
| ATOM | 1962 | CA | ALA | A | 291 | 5.204 | 56.008 | 60.726 | 1.00 | 76.21 | A | C |
| ATOM | 1963 | CB | ALA | A | 291 | 6.394 | 55.093 | 61.083 | 1.00 | 64.41 | A | C |
| ATOM | 1964 | C | ALA | A | 291 | 4.929 | 55.978 | 59.200 | 1.00 | 76.21 | A | C |
| ATOM | 1965 | O | ALA | A | 291 | 3.971 | 56.612 | 58.730 | 1.00 | 76.21 | A | O |
| ATOM | 1966 | N | ALA | A | 292 | 5.775 | 55.263 | 58.442 | 1.00 | 98.91 | A | N |
| ATOM | 1967 | CA | ALA | A | 292 | 5.661 | 55.142 | 56.969 | 1.00 | 98.91 | A | C |
| ATOM | 1968 | CB | ALA | A | 292 | 4.201 | 54.875 | 56.559 | 1.00 | 44.14 | A | C |
| ATOM | 1969 | C | ALA | A | 292 | 6.190 | 56.405 | 56.261 | 1.00 | 98.91 | A | C |
| ATOM | 1970 | O | ALA | A | 292 | 5.533 | 57.458 | 56.288 | 1.00 | 98.91 | A | O |
| ATOM | 1971 | N | ALA | A | 293 | 7.358 | 56.302 | 55.619 | 1.00 | 79.55 | A | N |
| ATOM | 1972 | CA | ALA | A | 293 | 7.956 | 57.468 | 54.949 | 1.00 | 79.55 | A | C |
| ATOM | 1973 | CB | ALA | A | 293 | 9.021 | 58.117 | 55.871 | 1.00 | 86.47 | A | C |
| ATOM | 1974 | C | ALA | A | 293 | 8.580 | 57.130 | 53.593 | 1.00 | 79.55 | A | C |
| ATOM | 1975 | O | ALA | A | 293 | 8.711 | 55.956 | 53.236 | 1.00 | 79.55 | A | O |
| ATOM | 1976 | N | PRO | A | 294 | 9.016 | 58.153 | 52.832 | 1.00 | 69.16 | A | N |
| ATOM | 1977 | CD | PRO | A | 294 | 9.199 | 59.593 | 53.104 | 1.00 | 76.85 | A | C |
| ATOM | 1978 | CA | PRO | A | 294 | 9.593 | 57.798 | 51.532 | 1.00 | 69.16 | A | C |
| ATOM | 1979 | CB | PRO | A | 294 | 9.707 | 59.139 | 50.824 | 1.00 | 76.85 | A | C |
| ATOM | 1980 | CG | PRO | A | 294 | 10.167 | 60.028 | 51.969 | 1.00 | 76.85 | A | C |
| ATOM | 1981 | C | PRO | A | 294 | 10.933 | 57.110 | 51.638 | 1.00 | 69.16 | A | C |
| ATOM | 1982 | O | PRO | A | 294 | 11.596 | 57.150 | 52.681 | 1.00 | 69.16 | A | O |
| ATOM | 1983 | N | TRP | A | 301 | 11.306 | 56.499 | 50.521 | 1.00 | 52.65 | A | N |
| ATOM | 1984 | CA | TRP | A | 301 | 12.541 | 55.757 | 50.373 | 1.00 | 52.65 | A | C |
| ATOM | 1985 | CB | TRP | A | 301 | 12.296 | 54.517 | 49.518 | 1.00 | 34.69 | A | C |
| ATOM | 1986 | CG | TRP | A | 301 | 12.371 | 53.228 | 50.258 | 1.00 | 34.69 | A | C |
| ATOM | 1987 | CD2 | TRP | A | 301 | 13.527 | 52.676 | 50.888 | 1.00 | 34.69 | A | C |
| ATOM | 1988 | CE2 | TRP | A | 301 | 13.169 | 51.393 | 51.366 | 1.00 | 34.69 | A | C |
| ATOM | 1989 | CE3 | TRP | A | 301 | 14.840 | 53.138 | 51.094 | 1.00 | 34.69 | A | C |
| ATOM | 1990 | CD1 | TRP | A | 301 | 11.379 | 52.294 | 50.383 | 1.00 | 34.69 | A | C |
| ATOM | 1991 | NE1 | TRP | A | 301 | 11.853 | 51.187 | 51.045 | 1.00 | 34.69 | A | N |
| ATOM | 1992 | CZ2 | TRP | A | 301 | 14.073 | 50.563 | 52.036 | 1.00 | 34.69 | A | C |
| ATOM | 1993 | CZ3 | TRP | A | 301 | 15.743 | 52.313 | 51.760 | 1.00 | 34.69 | A | C |
| ATOM | 1994 | CH2 | TRP | A | 301 | 15.352 | 51.038 | 52.223 | 1.00 | 34.69 | A | C |
| ATOM | 1995 | C | TRP | A | 301 | 13.502 | 56.662 | 49.645 | 1.00 | 52.65 | A | C |
| ATOM | 1996 | O | TRP | A | 301 | 14.719 | 56.477 | 49.703 | 1.00 | 52.65 | A | O |
| ATOM | 1997 | N | THR | A | 302 | 12.943 | 57.636 | 48.943 | 1.00 | 40.98 | A | N |
| ATOM | 1998 | CA | THR | A | 302 | 13.758 | 58.566 | 48.178 | 1.00 | 40.98 | A | C |
| ATOM | 1999 | CB | THR | A | 302 | 12.912 | 59.277 | 47.110 | 1.00 | 62.98 | A | C |
| ATOM | 2000 | OG1 | THR | A | 302 | 11.538 | 59.271 | 47.526 | 1.00 | 62.98 | A | O |
| ATOM | 2001 | CG2 | THR | A | 302 | 13.065 | 58.582 | 45.734 | 1.00 | 62.98 | A | C |
| ATOM | 2002 | C | THR | A | 302 | 14.408 | 59.578 | 49.112 | 1.00 | 40.98 | A | C |
| ATOM | 2003 | O | THR | A | 302 | 15.523 | 60.054 | 48.871 | 1.00 | 40.98 | A | O |
| ATOM | 2004 | N | ALA | A | 303 | 13.713 | 59.900 | 50.192 | 1.00 | 38.10 | A | N |

| | | | | | | | | | | | | |
|---|---|---|---|---|---|---|---|---|---|---|---|---|
| ATOM | 2005 | CA | ALA | A | 303 | 14.271 | 60.829 | 51.164 | 1.00 | 38.10 | A | C |
| ATOM | 2006 | CB | ALA | A | 303 | 13.212 | 61.173 | 52.219 | 1.00 | 49.99 | A | C |
| ATOM | 2007 | C | ALA | A | 303 | 15.486 | 60.166 | 51.831 | 1.00 | 38.10 | A | C |
| ATOM | 2008 | O | ALA | A | 303 | 16.351 | 60.825 | 52.420 | 1.00 | 38.10 | A | O |
| ATOM | 2009 | N | VAL | A | 304 | 15.532 | 58.847 | 51.718 | 1.00 | 36.96 | A | N |
| ATOM | 2010 | CA | VAL | A | 304 | 16.577 | 58.045 | 52.319 | 1.00 | 36.96 | A | C |
| ATOM | 2011 | CB | VAL | A | 304 | 16.205 | 56.562 | 52.283 | 1.00 | 42.39 | A | C |
| ATOM | 2012 | CG1 | VAL | A | 304 | 17.272 | 55.746 | 52.996 | 1.00 | 42.39 | A | C |
| ATOM | 2013 | CG2 | VAL | A | 304 | 14.837 | 56.358 | 52.910 | 1.00 | 42.39 | A | C |
| ATOM | 2014 | C | VAL | A | 304 | 17.981 | 58.166 | 51.751 | 1.00 | 36.96 | A | C |
| ATOM | 2015 | O | VAL | A | 304 | 18.943 | 58.158 | 52.511 | 1.00 | 36.96 | A | O |
| ATOM | 2016 | N | PHE | A | 305 | 18.117 | 58.263 | 50.432 | 1.00 | 28.00 | A | N |
| ATOM | 2017 | CA | PHE | A | 305 | 19.449 | 58.341 | 49.834 | 1.00 | 28.00 | A | C |
| ATOM | 2018 | CB | PHE | A | 305 | 19.537 | 57.383 | 48.662 | 1.00 | 31.80 | A | C |
| ATOM | 2019 | CG | PHE | A | 305 | 19.137 | 55.997 | 49.016 | 1.00 | 31.80 | A | C |
| ATOM | 2020 | CD1 | PHE | A | 305 | 19.986 | 55.193 | 49.775 | 1.00 | 31.80 | A | C |
| ATOM | 2021 | CD2 | PHE | A | 305 | 17.892 | 55.495 | 48.628 | 1.00 | 31.80 | A | C |
| ATOM | 2022 | CE1 | PHE | A | 305 | 19.605 | 53.889 | 50.153 | 1.00 | 31.80 | A | C |
| ATOM | 2023 | CE2 | PHE | A | 305 | 17.490 | 54.192 | 48.993 | 1.00 | 31.80 | A | C |
| ATOM | 2024 | CZ | PHE | A | 305 | 18.354 | 53.384 | 49.762 | 1.00 | 31.80 | A | C |
| ATOM | 2025 | C | PHE | A | 305 | 19.844 | 59.723 | 49.375 | 1.00 | 28.00 | A | C |
| ATOM | 2026 | O | PHE | A | 305 | 19.068 | 60.664 | 49.494 | 1.00 | 28.00 | A | O |
| ATOM | 2027 | N | ARG | A | 306 | 21.072 | 59.835 | 48.871 | 1.00 | 38.93 | A | N |
| ATOM | 2028 | CA | ARG | A | 306 | 21.584 | 61.101 | 48.367 | 1.00 | 38.93 | A | C |
| ATOM | 2029 | CB | ARG | A | 306 | 23.038 | 60.969 | 47.908 | 1.00 | 54.66 | A | C |
| ATOM | 2030 | CG | ARG | A | 306 | 23.948 | 60.159 | 48.801 | 1.00 | 54.66 | A | C |
| ATOM | 2031 | CD | ARG | A | 306 | 25.285 | 59.938 | 48.076 | 1.00 | 54.66 | A | C |
| ATOM | 2032 | NE | ARG | A | 306 | 25.123 | 59.895 | 46.616 | 1.00 | 54.66 | A | N |
| ATOM | 2033 | CZ | ARG | A | 306 | 26.105 | 59.673 | 45.738 | 1.00 | 54.66 | A | C |
| ATOM | 2034 | NH1 | ARG | A | 306 | 27.347 | 59.457 | 46.156 | 1.00 | 54.66 | A | N |
| ATOM | 2035 | NH2 | ARG | A | 306 | 25.849 | 59.699 | 44.433 | 1.00 | 54.66 | A | N |
| ATOM | 2036 | C | ARG | A | 306 | 20.739 | 61.513 | 47.163 | 1.00 | 38.93 | A | C |
| ATOM | 2037 | O | ARG | A | 306 | 20.261 | 60.680 | 46.389 | 1.00 | 38.93 | A | O |
| ATOM | 2038 | N | PRO | A | 307 | 20.558 | 62.817 | 46.982 | 1.00 | 61.10 | A | N |
| ATOM | 2039 | CD | PRO | A | 307 | 21.084 | 63.898 | 47.839 | 1.00 | 69.15 | A | C |
| ATOM | 2040 | CA | PRO | A | 307 | 19.762 | 63.346 | 45.872 | 1.00 | 61.10 | A | C |
| ATOM | 2041 | CB | PRO | A | 307 | 20.006 | 64.851 | 45.968 | 1.00 | 69.15 | A | C |
| ATOM | 2042 | CG | PRO | A | 307 | 20.179 | 65.063 | 47.477 | 1.00 | 69.15 | A | C |
| ATOM | 2043 | C | PRO | A | 307 | 20.080 | 62.784 | 44.479 | 1.00 | 61.10 | A | C |
| ATOM | 2044 | O | PRO | A | 307 | 19.162 | 62.522 | 43.696 | 1.00 | 61.10 | A | O |
| ATOM | 2045 | N | ALA | A | 308 | 21.359 | 62.597 | 44.159 | 1.00 | 31.52 | A | N |
| ATOM | 2046 | CA | ALA | A | 308 | 21.717 | 62.083 | 42.834 | 1.00 | 31.52 | A | C |
| ATOM | 2047 | CB | ALA | A | 308 | 23.144 | 62.515 | 42.480 | 1.00 | 53.50 | A | C |
| ATOM | 2048 | C | ALA | A | 308 | 21.584 | 60.556 | 42.675 | 1.00 | 31.52 | A | C |
| ATOM | 2049 | O | ALA | A | 308 | 21.883 | 60.008 | 41.617 | 1.00 | 31.52 | A | O |
| ATOM | 2050 | N | THR | A | 309 | 21.127 | 59.870 | 43.715 | 1.00 | 23.81 | A | N |
| ATOM | 2051 | CA | THR | A | 309 | 21.004 | 58.417 | 43.661 | 1.00 | 23.81 | A | C |
| ATOM | 2052 | CB | THR | A | 309 | 20.519 | 57.840 | 45.010 | 1.00 | 28.68 | A | C |
| ATOM | 2053 | OG1 | THR | A | 309 | 21.526 | 58.027 | 46.012 | 1.00 | 28.68 | A | O |
| ATOM | 2054 | CG2 | THR | A | 309 | 20.226 | 56.361 | 44.880 | 1.00 | 28.68 | A | C |
| ATOM | 2055 | C | THR | A | 309 | 20.039 | 57.957 | 42.586 | 1.00 | 23.81 | A | C |
| ATOM | 2056 | O | THR | A | 309 | 18.934 | 58.480 | 42.487 | 1.00 | 23.81 | A | O |
| ATOM | 2057 | N | PRO | A | 310 | 20.451 | 56.984 | 41.750 | 1.00 | 33.83 | A | N |
| ATOM | 2058 | CD | PRO | A | 310 | 21.820 | 56.497 | 41.543 | 1.00 | 16.70 | A | C |
| ATOM | 2059 | CA | PRO | A | 310 | 19.570 | 56.481 | 40.692 | 1.00 | 33.83 | A | C |
| ATOM | 2060 | CB | PRO | A | 310 | 20.459 | 55.500 | 39.919 | 1.00 | 16.70 | A | C |
| ATOM | 2061 | CG | PRO | A | 310 | 21.562 | 55.204 | 40.833 | 1.00 | 16.70 | A | C |
| ATOM | 2062 | C | PRO | A | 310 | 18.296 | 55.837 | 41.231 | 1.00 | 33.83 | A | C |
| ATOM | 2063 | O | PRO | A | 310 | 18.330 | 55.043 | 42.168 | 1.00 | 33.83 | A | O |
| ATOM | 2064 | N | PRO | A | 311 | 17.146 | 56.181 | 40.640 | 1.00 | 44.00 | A | N |
| ATOM | 2065 | CD | PRO | A | 311 | 16.978 | 57.145 | 39.540 | 1.00 | 35.22 | A | C |
| ATOM | 2066 | CA | PRO | A | 311 | 15.850 | 55.648 | 41.060 | 1.00 | 44.00 | A | C |
| ATOM | 2067 | CB | PRO | A | 311 | 14.870 | 56.335 | 40.110 | 1.00 | 35.22 | A | C |
| ATOM | 2068 | CG | PRO | A | 311 | 15.569 | 57.602 | 39.752 | 1.00 | 35.22 | A | C |
| ATOM | 2069 | C | PRO | A | 311 | 15.750 | 54.129 | 40.983 | 1.00 | 44.00 | A | C |
| ATOM | 2070 | O | PRO | A | 311 | 14.961 | 53.511 | 41.712 | 1.00 | 44.00 | A | O |
| ATOM | 2071 | N | GLU | A | 312 | 16.550 | 53.538 | 40.103 | 1.00 | 24.22 | A | N |

| | | | | | | | | | | | | |
|---|---|---|---|---|---|---|---|---|---|---|---|---|
| ATOM | 2072 | CA | GLU | A | 312 | 16.560 | 52.083 | 39.903 | 1.00 | 24.22 | A | C |
| ATOM | 2073 | CB | GLU | A | 312 | 17.367 | 51.729 | 38.644 | 1.00 | 62.63 | A | C |
| ATOM | 2074 | CG | GLU | A | 312 | 16.879 | 52.389 | 37.370 | 1.00 | 62.63 | A | C |
| ATOM | 2075 | CD | GLU | A | 312 | 16.758 | 53.921 | 37.485 | 1.00 | 62.63 | A | C |
| ATOM | 2076 | OE1 | GLU | A | 312 | 17.711 | 54.548 | 38.037 | 1.00 | 62.63 | A | O |
| ATOM | 2077 | OE2 | GLU | A | 312 | 15.715 | 54.475 | 37.014 | 1.00 | 62.63 | A | O |
| ATOM | 2078 | C | GLU | A | 312 | 17.174 | 51.340 | 41.093 | 1.00 | 24.22 | A | C |
| ATOM | 2079 | O | GLU | A | 312 | 16.722 | 50.251 | 41.465 | 1.00 | 24.22 | A | O |
| ATOM | 2080 | N | ALA | A | 313 | 18.222 | 51.928 | 41.663 | 1.00 | 27.51 | A | N |
| ATOM | 2081 | CA | ALA | A | 313 | 18.915 | 51.346 | 42.806 | 1.00 | 27.51 | A | C |
| ATOM | 2082 | CB | ALA | A | 313 | 20.254 | 52.055 | 43.034 | 1.00 | 32.80 | A | C |
| ATOM | 2083 | C | ALA | A | 313 | 18.046 | 51.469 | 44.040 | 1.00 | 27.51 | A | C |
| ATOM | 2084 | O | ALA | A | 313 | 18.024 | 50.582 | 44.899 | 1.00 | 27.51 | A | O |
| ATOM | 2085 | N | ILE | A | 314 | 17.314 | 52.570 | 44.109 | 1.00 | 37.36 | A | N |
| ATOM | 2086 | CA | ILE | A | 314 | 16.448 | 52.808 | 45.232 | 1.00 | 37.36 | A | C |
| ATOM | 2087 | CB | ILE | A | 314 | 16.261 | 54.327 | 45.426 | 1.00 | 55.52 | A | C |
| ATOM | 2088 | CG2 | ILE | A | 314 | 16.039 | 54.998 | 44.125 | 1.00 | 55.52 | A | C |
| ATOM | 2089 | CG1 | ILE | A | 314 | 15.151 | 54.604 | 46.416 | 1.00 | 55.52 | A | C |
| ATOM | 2090 | CD1 | ILE | A | 314 | 14.977 | 56.090 | 46.674 | 1.00 | 55.52 | A | C |
| ATOM | 2091 | C | ILE | A | 314 | 15.148 | 52.031 | 45.045 | 1.00 | 37.36 | A | C |
| ATOM | 2092 | O | ILE | A | 314 | 14.444 | 51.719 | 46.010 | 1.00 | 37.36 | A | O |
| ATOM | 2093 | N | ALA | A | 315 | 14.852 | 51.670 | 43.806 | 1.00 | 27.66 | A | N |
| ATOM | 2094 | CA | ALA | A | 315 | 13.658 | 50.881 | 43.532 | 1.00 | 27.66 | A | C |
| ATOM | 2095 | CB | ALA | A | 315 | 13.389 | 50.836 | 42.036 | 1.00 | 7.33 | A | C |
| ATOM | 2096 | C | ALA | A | 315 | 13.923 | 49.473 | 44.064 | 1.00 | 27.66 | A | C |
| ATOM | 2097 | O | ALA | A | 315 | 13.132 | 48.921 | 44.826 | 1.00 | 27.66 | A | O |
| ATOM | 2098 | N | LEU | A | 316 | 15.057 | 48.909 | 43.659 | 1.00 | 24.56 | A | N |
| ATOM | 2099 | CA | LEU | A | 316 | 15.469 | 47.574 | 44.068 | 1.00 | 24.56 | A | C |
| ATOM | 2100 | CB | LEU | A | 316 | 16.859 | 47.276 | 43.503 | 1.00 | 17.80 | A | C |
| ATOM | 2101 | CG | LEU | A | 316 | 17.502 | 45.933 | 43.835 | 1.00 | 17.80 | A | C |
| ATOM | 2102 | CD1 | LEU | A | 316 | 16.627 | 44.808 | 43.387 | 1.00 | 17.80 | A | C |
| ATOM | 2103 | CD2 | LEU | A | 316 | 18.842 | 45.845 | 43.170 | 1.00 | 17.80 | A | C |
| ATOM | 2104 | C | LEU | A | 316 | 15.472 | 47.408 | 45.589 | 1.00 | 24.56 | A | C |
| ATOM | 2105 | O | LEU | A | 316 | 15.040 | 46.377 | 46.108 | 1.00 | 24.56 | A | O |
| ATOM | 2106 | N | CYS | A | 317 | 15.948 | 48.420 | 46.307 | 1.00 | 25.76 | A | N |
| ATOM | 2107 | CA | CYS | A | 317 | 15.988 | 48.337 | 47.762 | 1.00 | 25.76 | A | C |
| ATOM | 2108 | CB | CYS | A | 317 | 16.507 | 49.628 | 48.377 | 1.00 | 30.60 | A | C |
| ATOM | 2109 | SG | CYS | A | 317 | 18.176 | 50.026 | 47.998 | 1.00 | 30.60 | A | S |
| ATOM | 2110 | C | CYS | A | 317 | 14.614 | 48.072 | 48.338 | 1.00 | 25.76 | A | C |
| ATOM | 2111 | O | CYS | A | 317 | 14.420 | 47.131 | 49.098 | 1.00 | 25.76 | A | O |
| ATOM | 2112 | N | SER | A | 318 | 13.661 | 48.920 | 47.976 | 1.00 | 30.58 | A | N |
| ATOM | 2113 | CA | SER | A | 318 | 12.304 | 48.796 | 48.475 | 1.00 | 30.58 | A | C |
| ATOM | 2114 | CB | SER | A | 318 | 11.411 | 49.842 | 47.820 | 1.00 | 41.98 | A | C |
| ATOM | 2115 | OG | SER | A | 318 | 11.197 | 49.512 | 46.461 | 1.00 | 41.98 | A | O |
| ATOM | 2116 | C | SER | A | 318 | 11.733 | 47.414 | 48.211 | 1.00 | 30.58 | A | C |
| ATOM | 2117 | O | SER | A | 318 | 10.846 | 46.956 | 48.932 | 1.00 | 30.58 | A | O |
| ATOM | 2118 | N | ARG | A | 319 | 12.252 | 46.753 | 47.180 | 1.00 | 29.39 | A | N |
| ATOM | 2119 | CA | ARG | A | 319 | 11.784 | 45.428 | 46.807 | 1.00 | 29.39 | A | C |
| ATOM | 2120 | CB | ARG | A | 319 | 11.844 | 45.277 | 45.289 | 1.00 | 37.05 | A | C |
| ATOM | 2121 | CG | ARG | A | 319 | 11.050 | 46.333 | 44.545 | 1.00 | 37.05 | A | C |
| ATOM | 2122 | CD | ARG | A | 319 | 9.536 | 46.223 | 44.775 | 1.00 | 37.05 | A | C |
| ATOM | 2123 | NE | ARG | A | 319 | 8.954 | 44.984 | 44.254 | 1.00 | 37.05 | A | N |
| ATOM | 2124 | CZ | ARG | A | 319 | 8.683 | 43.901 | 44.984 | 1.00 | 37.05 | A | C |
| ATOM | 2125 | NH1 | ARG | A | 319 | 8.927 | 43.882 | 46.294 | 1.00 | 37.05 | A | N |
| ATOM | 2126 | NH2 | ARG | A | 319 | 8.181 | 42.822 | 44.395 | 1.00 | 37.05 | A | N |
| ATOM | 2127 | C | ARG | A | 319 | 12.568 | 44.309 | 47.473 | 1.00 | 29.39 | A | C |
| ATOM | 2128 | O | ARG | A | 319 | 12.280 | 43.132 | 47.263 | 1.00 | 29.39 | A | O |
| ATOM | 2129 | N | LEU | A | 320 | 13.562 | 44.682 | 48.272 | 1.00 | 33.48 | A | N |
| ATOM | 2130 | CA | LEU | A | 320 | 14.383 | 43.715 | 48.982 | 1.00 | 33.48 | A | C |
| ATOM | 2131 | CB | LEU | A | 320 | 15.868 | 43.962 | 48.713 | 1.00 | 24.56 | A | C |
| ATOM | 2132 | CG | LEU | A | 320 | 16.332 | 43.750 | 47.271 | 1.00 | 24.56 | A | C |
| ATOM | 2133 | CD1 | LEU | A | 320 | 17.728 | 44.252 | 47.124 | 1.00 | 24.56 | A | C |
| ATOM | 2134 | CD2 | LEU | A | 320 | 16.260 | 42.280 | 46.900 | 1.00 | 24.56 | A | C |
| ATOM | 2135 | C | LEU | A | 320 | 14.101 | 43.835 | 50.464 | 1.00 | 33.48 | A | C |
| ATOM | 2136 | O | LEU | A | 320 | 14.081 | 42.834 | 51.175 | 1.00 | 33.48 | A | O |
| ATOM | 2137 | N | LEU | A | 321 | 13.867 | 45.062 | 50.922 | 1.00 | 20.95 | A | N |
| ATOM | 2138 | CA | LEU | A | 321 | 13.591 | 45.320 | 52.332 | 1.00 | 20.95 | A | C |

| | | | | | | | | | | | | |
|---|---|---|---|---|---|---|---|---|---|---|---|---|
| ATOM | 2139 | CB | LEU | A | 321 | 14.304 | 46.597 | 52.771 | 1.00 | 9.43 | A | C |
| ATOM | 2140 | CG | LEU | A | 321 | 15.808 | 46.577 | 52.505 | 1.00 | 9.43 | A | C |
| ATOM | 2141 | CD1 | LEU | A | 321 | 16.402 | 47.885 | 52.951 | 1.00 | 9.43 | A | C |
| ATOM | 2142 | CD2 | LEU | A | 321 | 16.452 | 45.400 | 53.208 | 1.00 | 9.43 | A | C |
| ATOM | 2143 | C | LEU | A | 321 | 12.086 | 45.416 | 52.596 | 1.00 | 20.95 | A | C |
| ATOM | 2144 | O | LEU | A | 321 | 11.548 | 46.492 | 52.839 | 1.00 | 20.95 | A | O |
| ATOM | 2145 | N | GLU | A | 322 | 11.420 | 44.268 | 52.537 | 1.00 | 25.12 | A | N |
| ATOM | 2146 | CA | GLU | A | 322 | 9.976 | 44.179 | 52.744 | 1.00 | 25.12 | A | C |
| ATOM | 2147 | CB | GLU | A | 322 | 9.302 | 43.453 | 51.563 | 1.00 | 35.51 | A | C |
| ATOM | 2148 | CG | GLU | A | 322 | 9.454 | 44.130 | 50.206 | 1.00 | 35.51 | A | C |
| ATOM | 2149 | CD | GLU | A | 322 | 8.220 | 44.898 | 49.778 | 1.00 | 35.51 | A | C |
| ATOM | 2150 | OE1 | GLU | A | 322 | 7.531 | 45.455 | 50.665 | 1.00 | 35.51 | A | O |
| ATOM | 2151 | OE2 | GLU | A | 322 | 7.961 | 44.949 | 48.550 | 1.00 | 35.51 | A | O |
| ATOM | 2152 | C | GLU | A | 322 | 9.679 | 43.395 | 54.010 | 1.00 | 25.12 | A | C |
| ATOM | 2153 | O | GLU | A | 322 | 10.239 | 42.327 | 54.199 | 1.00 | 25.12 | A | O |
| ATOM | 2154 | N | TYR | A | 323 | 8.796 | 43.898 | 54.871 | 1.00 | 18.04 | A | N |
| ATOM | 2155 | CA | TYR | A | 323 | 8.482 | 43.169 | 56.090 | 1.00 | 18.04 | A | C |
| ATOM | 2156 | CB | TYR | A | 323 | 7.303 | 43.792 | 56.845 | 1.00 | 29.33 | A | C |
| ATOM | 2157 | CG | TYR | A | 323 | 7.599 | 45.059 | 57.622 | 1.00 | 29.33 | A | C |
| ATOM | 2158 | CD1 | TYR | A | 323 | 8.669 | 45.124 | 58.530 | 1.00 | 29.33 | A | C |
| ATOM | 2159 | CE1 | TYR | A | 323 | 8.949 | 46.297 | 59.240 | 1.00 | 29.33 | A | C |
| ATOM | 2160 | CD2 | TYR | A | 323 | 6.807 | 46.197 | 57.444 | 1.00 | 29.33 | A | C |
| ATOM | 2161 | CE2 | TYR | A | 323 | 7.069 | 47.383 | 58.143 | 1.00 | 29.33 | A | C |
| ATOM | 2162 | CZ | TYR | A | 323 | 8.142 | 47.430 | 59.040 | 1.00 | 29.33 | A | C |
| ATOM | 2163 | OH | TYR | A | 323 | 8.397 | 48.606 | 59.724 | 1.00 | 29.33 | A | O |
| ATOM | 2164 | C | TYR | A | 323 | 8.105 | 41.756 | 55.697 | 1.00 | 18.04 | A | C |
| ATOM | 2165 | O | TYR | A | 323 | 8.743 | 40.798 | 56.095 | 1.00 | 18.04 | A | O |
| ATOM | 2166 | N | THR | A | 324 | 7.067 | 41.629 | 54.888 | 1.00 | 20.44 | A | N |
| ATOM | 2167 | CA | THR | A | 324 | 6.616 | 40.316 | 54.464 | 1.00 | 20.44 | A | C |
| ATOM | 2168 | CB | THR | A | 324 | 5.389 | 40.451 | 53.555 | 1.00 | 43.66 | A | C |
| ATOM | 2169 | OG1 | THR | A | 324 | 4.346 | 41.139 | 54.263 | 1.00 | 43.66 | A | O |
| ATOM | 2170 | CG2 | THR | A | 324 | 4.897 | 39.079 | 53.109 | 1.00 | 43.66 | A | C |
| ATOM | 2171 | C | THR | A | 324 | 7.737 | 39.613 | 53.709 | 1.00 | 20.44 | A | C |
| ATOM | 2172 | O | THR | A | 324 | 8.092 | 40.000 | 52.596 | 1.00 | 20.44 | A | O |
| ATOM | 2173 | N | PRO | A | 325 | 8.306 | 38.562 | 54.310 | 1.00 | 31.15 | A | N |
| ATOM | 2174 | CD | PRO | A | 325 | 7.983 | 38.061 | 55.652 | 1.00 | 28.89 | A | C |
| ATOM | 2175 | CA | PRO | A | 325 | 9.398 | 37.785 | 53.714 | 1.00 | 31.15 | A | C |
| ATOM | 2176 | CB | PRO | A | 325 | 9.628 | 36.676 | 54.743 | 1.00 | 28.89 | A | C |
| ATOM | 2177 | CG | PRO | A | 325 | 9.244 | 37.328 | 56.019 | 1.00 | 28.89 | A | C |
| ATOM | 2178 | C | PRO | A | 325 | 9.078 | 37.241 | 52.333 | 1.00 | 31.15 | A | C |
| ATOM | 2179 | O | PRO | A | 325 | 9.946 | 37.146 | 51.477 | 1.00 | 31.15 | A | O |
| ATOM | 2180 | N | THR | A | 326 | 7.813 | 36.911 | 52.129 | 1.00 | 23.05 | A | N |
| ATOM | 2181 | CA | THR | A | 326 | 7.334 | 36.344 | 50.886 | 1.00 | 23.05 | A | C |
| ATOM | 2182 | CB | THR | A | 326 | 5.992 | 35.642 | 51.185 | 1.00 | 26.65 | A | C |
| ATOM | 2183 | OG1 | THR | A | 326 | 5.785 | 34.605 | 50.237 | 1.00 | 26.65 | A | O |
| ATOM | 2184 | CG2 | THR | A | 326 | 4.831 | 36.612 | 51.129 | 1.00 | 26.65 | A | C |
| ATOM | 2185 | C | THR | A | 326 | 7.201 | 37.389 | 49.765 | 1.00 | 23.05 | A | C |
| ATOM | 2186 | O | THR | A | 326 | 7.131 | 37.040 | 48.591 | 1.00 | 23.05 | A | O |
| ATOM | 2187 | N | ALA | A | 327 | 7.193 | 38.670 | 50.130 | 1.00 | 29.64 | A | N |
| ATOM | 2188 | CA | ALA | A | 327 | 7.064 | 39.754 | 49.151 | 1.00 | 29.64 | A | C |
| ATOM | 2189 | CB | ALA | A | 327 | 6.246 | 40.891 | 49.743 | 1.00 | 1.79 | A | C |
| ATOM | 2190 | C | ALA | A | 327 | 8.397 | 40.302 | 48.635 | 1.00 | 29.64 | A | C |
| ATOM | 2191 | O | ALA | A | 327 | 8.430 | 41.171 | 47.763 | 1.00 | 29.64 | A | O |
| ATOM | 2192 | N | ARG | A | 328 | 9.500 | 39.807 | 49.181 | 1.00 | 36.12 | A | N |
| ATOM | 2193 | CA | ARG | A | 328 | 10.803 | 40.266 | 48.750 | 1.00 | 36.12 | A | C |
| ATOM | 2194 | CB | ARG | A | 328 | 11.860 | 39.855 | 49.766 | 1.00 | 20.53 | A | C |
| ATOM | 2195 | CG | ARG | A | 328 | 11.676 | 40.498 | 51.122 | 1.00 | 20.53 | A | C |
| ATOM | 2196 | CD | ARG | A | 328 | 12.482 | 39.792 | 52.183 | 1.00 | 20.53 | A | C |
| ATOM | 2197 | NE | ARG | A | 328 | 12.221 | 40.372 | 53.491 | 1.00 | 20.53 | A | N |
| ATOM | 2198 | CZ | ARG | A | 328 | 12.294 | 39.709 | 54.640 | 1.00 | 20.53 | A | C |
| ATOM | 2199 | NH1 | ARG | A | 328 | 12.615 | 38.427 | 54.665 | 1.00 | 20.53 | A | N |
| ATOM | 2200 | NH2 | ARG | A | 328 | 12.054 | 40.333 | 55.775 | 1.00 | 20.53 | A | N |
| ATOM | 2201 | C | ARG | A | 328 | 11.090 | 39.619 | 47.412 | 1.00 | 36.12 | A | C |
| ATOM | 2202 | O | ARG | A | 328 | 10.416 | 38.667 | 47.024 | 1.00 | 36.12 | A | O |
| ATOM | 2203 | N | LEU | A | 329 | 12.081 | 40.147 | 46.701 | 1.00 | 27.39 | A | N |
| ATOM | 2204 | CA | LEU | A | 329 | 12.460 | 39.601 | 45.411 | 1.00 | 27.39 | A | C |
| ATOM | 2205 | CB | LEU | A | 329 | 13.257 | 40.628 | 44.609 | 1.00 | 27.92 | A | C |

| | | | | | | | | | | | | |
|---|---|---|---|---|---|---|---|---|---|---|---|---|
| ATOM | 2206 | CG | LEU | A | 329 | 12.472 | 41.592 | 43.726 | 1.00 | 27.92 | A | C |
| ATOM | 2207 | CD1 | LEU | A | 329 | 11.304 | 42.173 | 44.494 | 1.00 | 27.92 | A | C |
| ATOM | 2208 | CD2 | LEU | A | 329 | 13.407 | 42.675 | 43.240 | 1.00 | 27.92 | A | C |
| ATOM | 2209 | C | LEU | A | 329 | 13.307 | 38.367 | 45.621 | 1.00 | 27.39 | A | C |
| ATOM | 2210 | O | LEU | A | 329 | 13.928 | 38.196 | 46.669 | 1.00 | 27.39 | A | O |
| ATOM | 2211 | N | THR | A | 330 | 13.318 | 37.487 | 44.634 | 1.00 | 28.11 | A | N |
| ATOM | 2212 | CA | THR | A | 330 | 14.136 | 36.297 | 44.752 | 1.00 | 28.11 | A | C |
| ATOM | 2213 | CB | THR | A | 330 | 13.544 | 35.111 | 43.991 | 1.00 | 20.87 | A | C |
| ATOM | 2214 | OG1 | THR | A | 330 | 13.693 | 35.322 | 42.584 | 1.00 | 20.87 | A | O |
| ATOM | 2215 | CG2 | THR | A | 330 | 12.078 | 34.961 | 44.318 | 1.00 | 20.87 | A | C |
| ATOM | 2216 | C | THR | A | 330 | 15.471 | 36.652 | 44.136 | 1.00 | 28.11 | A | C |
| ATOM | 2217 | O | THR | A | 330 | 15.531 | 37.434 | 43.189 | 1.00 | 28.11 | A | O |
| ATOM | 2218 | N | PRO | A | 331 | 16.560 | 36.091 | 44.676 | 1.00 | 28.11 | A | N |
| ATOM | 2219 | CD | PRO | A | 331 | 16.583 | 35.022 | 45.689 | 1.00 | 13.37 | A | C |
| ATOM | 2220 | CA | PRO | A | 331 | 17.902 | 36.354 | 44.167 | 1.00 | 28.11 | A | C |
| ATOM | 2221 | CB | PRO | A | 331 | 18.677 | 35.137 | 44.648 | 1.00 | 13.37 | A | C |
| ATOM | 2222 | CG | PRO | A | 331 | 18.051 | 34.881 | 45.992 | 1.00 | 13.37 | A | C |
| ATOM | 2223 | C | PRO | A | 331 | 17.910 | 36.505 | 42.648 | 1.00 | 28.11 | A | C |
| ATOM | 2224 | O | PRO | A | 331 | 18.459 | 37.480 | 42.125 | 1.00 | 28.11 | A | O |
| ATOM | 2225 | N | LEU | A | 332 | 17.286 | 35.557 | 41.939 | 1.00 | 27.74 | A | N |
| ATOM | 2226 | CA | LEU | A | 332 | 17.259 | 35.625 | 40.481 | 1.00 | 27.74 | A | C |
| ATOM | 2227 | CB | LEU | A | 332 | 16.712 | 34.346 | 39.856 | 1.00 | 27.63 | A | C |
| ATOM | 2228 | CG | LEU | A | 332 | 17.719 | 33.419 | 39.163 | 1.00 | 27.63 | A | C |
| ATOM | 2229 | CD1 | LEU | A | 332 | 16.958 | 32.375 | 38.387 | 1.00 | 27.63 | A | C |
| ATOM | 2230 | CD2 | LEU | A | 332 | 18.622 | 34.190 | 38.226 | 1.00 | 27.63 | A | C |
| ATOM | 2231 | C | LEU | A | 332 | 16.432 | 36.802 | 40.020 | 1.00 | 27.74 | A | C |
| ATOM | 2232 | O | LEU | A | 332 | 16.824 | 37.513 | 39.104 | 1.00 | 27.74 | A | O |
| ATOM | 2233 | N | GLU | A | 333 | 15.286 | 37.006 | 40.658 | 1.00 | 38.98 | A | N |
| ATOM | 2234 | CA | GLU | A | 333 | 14.427 | 38.140 | 40.323 | 1.00 | 38.98 | A | C |
| ATOM | 2235 | CB | GLU | A | 333 | 13.174 | 38.174 | 41.217 | 1.00 | 36.47 | A | C |
| ATOM | 2236 | CG | GLU | A | 333 | 12.123 | 37.131 | 40.865 | 1.00 | 36.47 | A | C |
| ATOM | 2237 | CD | GLU | A | 333 | 10.937 | 37.126 | 41.812 | 1.00 | 36.47 | A | C |
| ATOM | 2238 | OE1 | GLU | A | 333 | 11.095 | 37.565 | 42.979 | 1.00 | 36.47 | A | O |
| ATOM | 2239 | OE2 | GLU | A | 333 | 9.856 | 36.661 | 41.383 | 1.00 | 36.47 | A | O |
| ATOM | 2240 | C | GLU | A | 333 | 15.206 | 39.444 | 40.507 | 1.00 | 38.98 | A | C |
| ATOM | 2241 | O | GLU | A | 333 | 14.977 | 40.421 | 39.794 | 1.00 | 38.98 | A | O |
| ATOM | 2242 | N | ALA | A | 334 | 16.128 | 39.449 | 41.466 | 1.00 | 30.87 | A | N |
| ATOM | 2243 | CA | ALA | A | 334 | 16.936 | 40.621 | 41.743 | 1.00 | 30.87 | A | C |
| ATOM | 2244 | CB | ALA | A | 334 | 17.625 | 40.461 | 43.055 | 1.00 | 27.95 | A | C |
| ATOM | 2245 | C | ALA | A | 334 | 17.958 | 40.830 | 40.641 | 1.00 | 30.87 | A | C |
| ATOM | 2246 | O | ALA | A | 334 | 18.193 | 41.955 | 40.227 | 1.00 | 30.87 | A | O |
| ATOM | 2247 | N | CYS | A | 335 | 18.572 | 39.748 | 40.173 | 1.00 | 26.93 | A | N |
| ATOM | 2248 | CA | CYS | A | 335 | 19.550 | 39.849 | 39.095 | 1.00 | 26.93 | A | C |
| ATOM | 2249 | CB | CYS | A | 335 | 20.116 | 38.476 | 38.740 | 1.00 | 32.61 | A | C |
| ATOM | 2250 | SG | CYS | A | 335 | 21.388 | 37.885 | 39.834 | 1.00 | 32.61 | A | S |
| ATOM | 2251 | C | CYS | A | 335 | 18.941 | 40.456 | 37.836 | 1.00 | 26.93 | A | C |
| ATOM | 2252 | O | CYS | A | 335 | 19.618 | 41.143 | 37.086 | 1.00 | 26.93 | A | O |
| ATOM | 2253 | N | ALA | A | 336 | 17.656 | 40.200 | 37.625 | 1.00 | 28.02 | A | N |
| ATOM | 2254 | CA | ALA | A | 336 | 16.940 | 40.667 | 36.456 | 1.00 | 28.02 | A | C |
| ATOM | 2255 | CB | ALA | A | 336 | 15.818 | 39.716 | 36.160 | 1.00 | 18.31 | A | C |
| ATOM | 2256 | C | ALA | A | 336 | 16.390 | 42.061 | 36.638 | 1.00 | 28.02 | A | C |
| ATOM | 2257 | O | ALA | A | 336 | 15.688 | 42.559 | 35.763 | 1.00 | 28.02 | A | O |
| ATOM | 2258 | N | HIS | A | 337 | 16.680 | 42.685 | 37.778 | 1.00 | 29.34 | A | N |
| ATOM | 2259 | CA | HIS | A | 337 | 16.203 | 44.045 | 38.035 | 1.00 | 29.34 | A | C |
| ATOM | 2260 | CB | HIS | A | 337 | 16.419 | 44.436 | 39.500 | 1.00 | 26.90 | A | C |
| ATOM | 2261 | CG | HIS | A | 337 | 15.784 | 45.741 | 39.866 | 1.00 | 26.90 | A | C |
| ATOM | 2262 | CD2 | HIS | A | 337 | 16.212 | 47.016 | 39.718 | 1.00 | 26.90 | A | C |
| ATOM | 2263 | ND1 | HIS | A | 337 | 14.509 | 45.823 | 40.383 | 1.00 | 26.90 | A | N |
| ATOM | 2264 | CE1 | HIS | A | 337 | 14.179 | 47.093 | 40.536 | 1.00 | 26.90 | A | C |
| ATOM | 2265 | NE2 | HIS | A | 337 | 15.195 | 47.838 | 40.138 | 1.00 | 26.90 | A | N |
| ATOM | 2266 | C | HIS | A | 337 | 16.914 | 45.064 | 37.122 | 1.00 | 29.34 | A | C |
| ATOM | 2267 | O | HIS | A | 337 | 18.107 | 44.932 | 36.807 | 1.00 | 29.34 | A | O |
| ATOM | 2268 | N | SER | A | 338 | 16.173 | 46.088 | 36.709 | 1.00 | 30.66 | A | N |
| ATOM | 2269 | CA | SER | A | 338 | 16.701 | 47.106 | 35.813 | 1.00 | 30.66 | A | C |
| ATOM | 2270 | CB | SER | A | 338 | 15.581 | 48.047 | 35.396 | 1.00 | 37.71 | A | C |
| ATOM | 2271 | OG | SER | A | 338 | 14.747 | 48.335 | 36.498 | 1.00 | 37.71 | A | O |
| ATOM | 2272 | C | SER | A | 338 | 17.878 | 47.899 | 36.363 | 1.00 | 30.66 | A | C |

| | | | | | | | | | | | |
|---|---|---|---|---|---|---|---|---|---|---|---|
| ATOM | 2273 | O | SER | A | 338 | 18.509 | 48.661 | 35.638 | 1.00 | 30.66 | A | O |
| ATOM | 2274 | N | PHE | A | 339 | 18.178 | 47.722 | 37.641 | 1.00 | 28.31 | A | N |
| ATOM | 2275 | CA | PHE | A | 339 | 19.309 | 48.411 | 38.245 | 1.00 | 28.31 | A | C |
| ATOM | 2276 | CB | PHE | A | 339 | 19.295 | 48.236 | 39.769 | 1.00 | 25.07 | A | C |
| ATOM | 2277 | CG | PHE | A | 339 | 20.555 | 48.709 | 40.461 | 1.00 | 25.07 | A | C |
| ATOM | 2278 | CD1 | PHE | A | 339 | 21.047 | 49.995 | 40.259 | 1.00 | 25.07 | A | C |
| ATOM | 2279 | CD2 | PHE | A | 339 | 21.243 | 47.864 | 41.330 | 1.00 | 25.07 | A | C |
| ATOM | 2280 | CE1 | PHE | A | 339 | 22.206 | 50.426 | 40.916 | 1.00 | 25.07 | A | C |
| ATOM | 2281 | CE2 | PHE | A | 339 | 22.402 | 48.288 | 41.990 | 1.00 | 25.07 | A | C |
| ATOM | 2282 | CZ | PHE | A | 339 | 22.882 | 49.567 | 41.783 | 1.00 | 25.07 | A | C |
| ATOM | 2283 | C | PHE | A | 339 | 20.582 | 47.802 | 37.689 | 1.00 | 28.31 | A | C |
| ATOM | 2284 | O | PHE | A | 339 | 21.631 | 48.427 | 37.697 | 1.00 | 28.31 | A | O |
| ATOM | 2285 | N | PHE | A | 340 | 20.478 | 46.575 | 37.208 | 1.00 | 27.07 | A | N |
| ATOM | 2286 | CA | PHE | A | 340 | 21.624 | 45.877 | 36.663 | 1.00 | 27.07 | A | C |
| ATOM | 2287 | CB | PHE | A | 340 | 21.646 | 44.451 | 37.191 | 1.00 | 23.90 | A | C |
| ATOM | 2288 | CG | PHE | A | 340 | 21.651 | 44.358 | 38.683 | 1.00 | 23.90 | A | C |
| ATOM | 2289 | CD1 | PHE | A | 340 | 22.773 | 44.722 | 39.415 | 1.00 | 23.90 | A | C |
| ATOM | 2290 | CD2 | PHE | A | 340 | 20.533 | 43.887 | 39.359 | 1.00 | 23.90 | A | C |
| ATOM | 2291 | CE1 | PHE | A | 340 | 22.771 | 44.615 | 40.795 | 1.00 | 23.90 | A | C |
| ATOM | 2292 | CE2 | PHE | A | 340 | 20.528 | 43.779 | 40.729 | 1.00 | 23.90 | A | C |
| ATOM | 2293 | CZ | PHE | A | 340 | 21.644 | 44.140 | 41.449 | 1.00 | 23.90 | A | C |
| ATOM | 2294 | C | PHE | A | 340 | 21.613 | 45.845 | 35.145 | 1.00 | 27.07 | A | C |
| ATOM | 2295 | O | PHE | A | 340 | 22.329 | 45.042 | 34.531 | 1.00 | 27.07 | A | O |
| ATOM | 2296 | N | ASP | A | 341 | 20.801 | 46.696 | 34.526 | 1.00 | 33.66 | A | N |
| ATOM | 2297 | CA | ASP | A | 341 | 20.758 | 46.703 | 33.076 | 1.00 | 33.66 | A | C |
| ATOM | 2298 | CB | ASP | A | 341 | 19.709 | 47.708 | 32.579 | 1.00 | 40.09 | A | C |
| ATOM | 2299 | CG | ASP | A | 341 | 18.306 | 47.100 | 32.476 | 1.00 | 40.09 | A | C |
| ATOM | 2300 | OD1 | ASP | A | 341 | 18.187 | 45.885 | 32.190 | 1.00 | 40.09 | A | O |
| ATOM | 2301 | OD2 | ASP | A | 341 | 17.315 | 47.844 | 32.657 | 1.00 | 40.09 | A | O |
| ATOM | 2302 | C | ASP | A | 341 | 22.136 | 47.018 | 32.488 | 1.00 | 33.66 | A | C |
| ATOM | 2303 | O | ASP | A | 341 | 22.627 | 46.309 | 31.611 | 1.00 | 33.66 | A | O |
| ATOM | 2304 | N | GLU | A | 342 | 22.765 | 48.065 | 33.009 | 1.00 | 35.21 | A | N |
| ATOM | 2305 | CA | GLU | A | 342 | 24.069 | 48.508 | 32.543 | 1.00 | 35.21 | A | C |
| ATOM | 2306 | CB | GLU | A | 342 | 24.641 | 49.541 | 33.520 | 1.00 | 45.61 | A | C |
| ATOM | 2307 | CG | GLU | A | 342 | 25.943 | 50.172 | 33.056 | 1.00 | 45.61 | A | C |
| ATOM | 2308 | CD | GLU | A | 342 | 26.439 | 51.280 | 33.986 | 1.00 | 45.61 | A | C |
| ATOM | 2309 | OE1 | GLU | A | 342 | 25.568 | 51.980 | 34.564 | 1.00 | 45.61 | A | O |
| ATOM | 2310 | OE2 | GLU | A | 342 | 27.684 | 51.454 | 34.117 | 1.00 | 45.61 | A | O |
| ATOM | 2311 | C | GLU | A | 342 | 25.057 | 47.370 | 32.370 | 1.00 | 35.21 | A | C |
| ATOM | 2312 | O | GLU | A | 342 | 25.918 | 47.427 | 31.498 | 1.00 | 35.21 | A | O |
| ATOM | 2313 | N | LEU | A | 343 | 24.925 | 46.329 | 33.183 | 1.00 | 24.43 | A | N |
| ATOM | 2314 | CA | LEU | A | 343 | 25.848 | 45.213 | 33.109 | 1.00 | 24.43 | A | C |
| ATOM | 2315 | CB | LEU | A | 343 | 25.730 | 44.355 | 34.371 | 1.00 | 26.21 | A | C |
| ATOM | 2316 | CG | LEU | A | 343 | 25.870 | 45.122 | 35.694 | 1.00 | 26.21 | A | C |
| ATOM | 2317 | CD1 | LEU | A | 343 | 25.819 | 44.177 | 36.882 | 1.00 | 26.21 | A | C |
| ATOM | 2318 | CD2 | LEU | A | 343 | 27.194 | 45.885 | 35.692 | 1.00 | 26.21 | A | C |
| ATOM | 2319 | C | LEU | A | 343 | 25.539 | 44.389 | 31.877 | 1.00 | 24.43 | A | C |
| ATOM | 2320 | O | LEU | A | 343 | 26.402 | 43.703 | 31.337 | 1.00 | 24.43 | A | O |
| ATOM | 2321 | N | ARG | A | 344 | 24.294 | 44.467 | 31.433 | 1.00 | 22.77 | A | N |
| ATOM | 2322 | CA | ARG | A | 344 | 23.859 | 43.720 | 30.268 | 1.00 | 22.77 | A | C |
| ATOM | 2323 | CB | ARG | A | 344 | 22.361 | 43.437 | 30.354 | 1.00 | 27.26 | A | C |
| ATOM | 2324 | CG | ARG | A | 344 | 22.008 | 42.171 | 31.119 | 1.00 | 27.26 | A | C |
| ATOM | 2325 | CD | ARG | A | 344 | 20.495 | 41.962 | 31.183 | 1.00 | 27.26 | A | C |
| ATOM | 2326 | NE | ARG | A | 344 | 19.817 | 42.953 | 32.018 | 1.00 | 27.26 | A | N |
| ATOM | 2327 | CZ | ARG | A | 344 | 19.732 | 42.892 | 33.344 | 1.00 | 27.26 | A | C |
| ATOM | 2328 | NH1 | ARG | A | 344 | 20.275 | 41.886 | 34.015 | 1.00 | 27.26 | A | N |
| ATOM | 2329 | NH2 | ARG | A | 344 | 19.098 | 43.844 | 34.006 | 1.00 | 27.26 | A | N |
| ATOM | 2330 | C | ARG | A | 344 | 24.190 | 44.465 | 28.983 | 1.00 | 22.77 | A | C |
| ATOM | 2331 | O | ARG | A | 344 | 24.061 | 43.934 | 27.886 | 1.00 | 22.77 | A | O |
| ATOM | 2332 | N | ASP | A | 345 | 24.614 | 45.709 | 29.130 | 1.00 | 29.72 | A | N |
| ATOM | 2333 | CA | ASP | A | 345 | 25.010 | 46.525 | 27.992 | 1.00 | 29.72 | A | C |
| ATOM | 2334 | CB | ASP | A | 345 | 25.462 | 47.906 | 28.485 | 1.00 | 58.81 | A | C |
| ATOM | 2335 | CG | ASP | A | 345 | 25.788 | 48.875 | 27.342 | 1.00 | 58.81 | A | C |
| ATOM | 2336 | OD1 | ASP | A | 345 | 26.575 | 48.493 | 26.432 | 1.00 | 58.81 | A | O |
| ATOM | 2337 | OD2 | ASP | A | 345 | 25.256 | 50.019 | 27.367 | 1.00 | 58.81 | A | O |
| ATOM | 2338 | C | ASP | A | 345 | 26.195 | 45.801 | 27.347 | 1.00 | 29.72 | A | C |
| ATOM | 2339 | O | ASP | A | 345 | 27.093 | 45.310 | 28.031 | 1.00 | 29.72 | A | O |

| | | | | | | | | | | | | |
|---|---|---|---|---|---|---|---|---|---|---|---|---|
| ATOM | 2340 | N | PRO | A | 346 | 26.213 | 45.726 | 26.017 | 1.00 | 34.64 | A | N |
| ATOM | 2341 | CD | PRO | A | 346 | 25.235 | 46.245 | 25.051 | 1.00 | 28.88 | A | C |
| ATOM | 2342 | CA | PRO | A | 346 | 27.318 | 45.046 | 25.340 | 1.00 | 34.64 | A | C |
| ATOM | 2343 | CB | PRO | A | 346 | 26.826 | 44.972 | 23.906 | 1.00 | 28.88 | A | C |
| ATOM | 2344 | CG | PRO | A | 346 | 26.028 | 46.240 | 23.783 | 1.00 | 28.88 | A | C |
| ATOM | 2345 | C | PRO | A | 346 | 28.623 | 45.804 | 25.474 | 1.00 | 34.64 | A | C |
| ATOM | 2346 | O | PRO | A | 346 | 29.700 | 45.207 | 25.459 | 1.00 | 34.64 | A | O |
| ATOM | 2347 | N | ASN | A | 347 | 28.525 | 47.120 | 25.627 | 1.00 | 41.18 | A | N |
| ATOM | 2348 | CA | ASN | A | 347 | 29.729 | 47.931 | 25.757 | 1.00 | 41.18 | A | C |
| ATOM | 2349 | CB | ASN | A | 347 | 29.562 | 49.259 | 25.010 | 1.00 | 55.72 | A | C |
| ATOM | 2350 | CG | ASN | A | 347 | 29.919 | 49.138 | 23.532 | 1.00 | 55.72 | A | C |
| ATOM | 2351 | OD1 | ASN | A | 347 | 29.126 | 48.639 | 22.719 | 1.00 | 55.72 | A | O |
| ATOM | 2352 | ND2 | ASN | A | 347 | 31.137 | 49.571 | 23.183 | 1.00 | 55.72 | A | N |
| ATOM | 2353 | C | ASN | A | 347 | 30.263 | 48.184 | 27.163 | 1.00 | 41.18 | A | C |
| ATOM | 2354 | O | ASN | A | 347 | 31.285 | 48.859 | 27.326 | 1.00 | 41.18 | A | O |
| ATOM | 2355 | N | VAL | A | 348 | 29.594 | 47.627 | 28.172 | 1.00 | 40.49 | A | N |
| ATOM | 2356 | CA | VAL | A | 348 | 30.021 | 47.816 | 29.556 | 1.00 | 40.49 | A | C |
| ATOM | 2357 | CB | VAL | A | 348 | 29.137 | 47.011 | 30.542 | 1.00 | 35.15 | A | C |
| ATOM | 2358 | CG1 | VAL | A | 348 | 29.303 | 45.522 | 30.300 | 1.00 | 35.15 | A | C |
| ATOM | 2359 | CG2 | VAL | A | 348 | 29.497 | 47.369 | 31.976 | 1.00 | 35.15 | A | C |
| ATOM | 2360 | C | VAL | A | 348 | 31.478 | 47.395 | 29.754 | 1.00 | 40.49 | A | C |
| ATOM | 2361 | O | VAL | A | 348 | 31.912 | 46.362 | 29.240 | 1.00 | 40.49 | A | O |
| ATOM | 2362 | N | LYS | A | 349 | 32.229 | 48.216 | 30.483 | 1.00 | 29.53 | A | N |
| ATOM | 2363 | CA | LYS | A | 349 | 33.621 | 47.929 | 30.791 | 1.00 | 29.53 | A | C |
| ATOM | 2364 | CB | LYS | A | 349 | 34.562 | 48.508 | 29.716 | 1.00 | 50.78 | A | C |
| ATOM | 2365 | CG | LYS | A | 349 | 34.343 | 47.928 | 28.310 | 1.00 | 50.78 | A | C |
| ATOM | 2366 | CD | LYS | A | 349 | 35.654 | 47.672 | 27.587 | 1.00 | 50.78 | A | C |
| ATOM | 2367 | CE | LYS | A | 349 | 36.374 | 46.443 | 28.155 | 1.00 | 50.78 | A | C |
| ATOM | 2368 | NZ | LYS | A | 349 | 37.814 | 46.324 | 27.691 | 1.00 | 50.78 | A | N |
| ATOM | 2369 | C | LYS | A | 349 | 33.976 | 48.476 | 32.187 | 1.00 | 29.53 | A | C |
| ATOM | 2370 | O | LYS | A | 349 | 33.313 | 49.367 | 32.727 | 1.00 | 29.53 | A | O |
| ATOM | 2371 | N | LEU | A | 350 | 35.012 | 47.932 | 32.797 | 1.00 | 41.51 | A | N |
| ATOM | 2372 | CA | LEU | A | 350 | 35.377 | 48.423 | 34.111 | 1.00 | 41.51 | A | C |
| ATOM | 2373 | CB | LEU | A | 350 | 36.319 | 47.425 | 34.794 | 1.00 | 53.76 | A | C |
| ATOM | 2374 | CG | LEU | A | 350 | 35.550 | 46.166 | 35.131 | 1.00 | 53.76 | A | C |
| ATOM | 2375 | CD1 | LEU | A | 350 | 36.388 | 45.075 | 35.739 | 1.00 | 53.76 | A | C |
| ATOM | 2376 | CD2 | LEU | A | 350 | 34.365 | 46.519 | 35.996 | 1.00 | 53.76 | A | C |
| ATOM | 2377 | C | LEU | A | 350 | 36.020 | 49.796 | 34.006 | 1.00 | 41.51 | A | C |
| ATOM | 2378 | O | LEU | A | 350 | 36.639 | 50.108 | 32.982 | 1.00 | 41.51 | A | O |
| ATOM | 2379 | N | PRO | A | 351 | 35.891 | 50.634 | 35.057 | 1.00 | 34.17 | A | N |
| ATOM | 2380 | CD | PRO | A | 351 | 35.450 | 50.397 | 36.444 | 1.00 | 51.93 | A | C |
| ATOM | 2381 | CA | PRO | A | 351 | 36.527 | 51.952 | 34.927 | 1.00 | 34.17 | A | C |
| ATOM | 2382 | CB | PRO | A | 351 | 36.229 | 52.621 | 36.264 | 1.00 | 51.93 | A | C |
| ATOM | 2383 | CG | PRO | A | 351 | 36.216 | 51.474 | 37.216 | 1.00 | 51.93 | A | C |
| ATOM | 2384 | C | PRO | A | 351 | 38.009 | 51.671 | 34.737 | 1.00 | 34.17 | A | C |
| ATOM | 2385 | O | PRO | A | 351 | 38.711 | 52.349 | 33.987 | 1.00 | 34.17 | A | O |
| ATOM | 2386 | N | ASN | A | 352 | 38.422 | 50.619 | 35.437 | 1.00 | 48.43 | A | N |
| ATOM | 2387 | CA | ASN | A | 352 | 39.748 | 50.031 | 35.462 | 1.00 | 48.43 | A | C |
| ATOM | 2388 | CB | ASN | A | 352 | 39.570 | 48.635 | 36.069 | 1.00 | 77.92 | A | C |
| ATOM | 2389 | CG | ASN | A | 352 | 40.722 | 47.705 | 35.784 | 1.00 | 77.92 | A | C |
| ATOM | 2390 | OD1 | ASN | A | 352 | 41.283 | 47.708 | 34.680 | 1.00 | 77.92 | A | O |
| ATOM | 2391 | ND2 | ASN | A | 352 | 41.069 | 46.868 | 36.779 | 1.00 | 77.92 | A | N |
| ATOM | 2392 | C | ASN | A | 352 | 40.311 | 49.945 | 34.048 | 1.00 | 48.43 | A | C |
| ATOM | 2393 | O | ASN | A | 352 | 41.503 | 50.149 | 33.841 | 1.00 | 48.43 | A | O |
| ATOM | 2394 | N | GLY | A | 353 | 39.448 | 49.640 | 33.077 | 1.00 | 41.47 | A | N |
| ATOM | 2395 | CA | GLY | A | 353 | 39.882 | 49.523 | 31.690 | 1.00 | 41.47 | A | C |
| ATOM | 2396 | C | GLY | A | 353 | 39.598 | 48.140 | 31.103 | 1.00 | 41.47 | A | C |
| ATOM | 2397 | O | GLY | A | 353 | 39.253 | 47.992 | 29.921 | 1.00 | 41.47 | A | O |
| ATOM | 2398 | N | ARG | A | 354 | 39.746 | 47.115 | 31.946 | 1.00 | 42.07 | A | N |
| ATOM | 2399 | CA | ARG | A | 354 | 39.511 | 45.723 | 31.549 | 1.00 | 42.07 | A | C |
| ATOM | 2400 | CB | ARG | A | 354 | 39.952 | 44.767 | 32.672 | 1.00 | 78.17 | A | C |
| ATOM | 2401 | CG | ARG | A | 354 | 41.175 | 45.245 | 33.434 | 1.00 | 78.17 | A | C |
| ATOM | 2402 | CD | ARG | A | 354 | 41.569 | 44.345 | 34.605 | 1.00 | 78.17 | A | C |
| ATOM | 2403 | NE | ARG | A | 354 | 42.295 | 43.147 | 34.178 | 1.00 | 78.17 | A | N |
| ATOM | 2404 | CZ | ARG | A | 354 | 43.127 | 42.451 | 34.956 | 1.00 | 78.17 | A | C |
| ATOM | 2405 | NH1 | ARG | A | 354 | 43.340 | 42.842 | 36.213 | 1.00 | 78.17 | A | N |
| ATOM | 2406 | NH2 | ARG | A | 354 | 43.747 | 41.368 | 34.474 | 1.00 | 78.17 | A | N |

| | | | | | | | | | | | | |
|---|---|---|---|---|---|---|---|---|---|---|---|---|
| ATOM | 2407 | C | ARG | A | 354 | 38.039 | 45.430 | 31.248 | 1.00 | 42.07 | A | C |
| ATOM | 2408 | O | ARG | A | 354 | 37.184 | 46.321 | 31.238 | 1.00 | 42.07 | A | O |
| ATOM | 2409 | N | ASP | A | 355 | 37.759 | 44.154 | 31.029 | 1.00 | 34.98 | A | N |
| ATOM | 2410 | CA | ASP | A | 355 | 36.414 | 43.676 | 30.743 | 1.00 | 34.98 | A | C |
| ATOM | 2411 | CB | ASP | A | 355 | 36.495 | 42.437 | 29.861 | 1.00 | 79.47 | A | C |
| ATOM | 2412 | CG | ASP | A | 355 | 35.897 | 42.650 | 28.489 | 1.00 | 79.47 | A | C |
| ATOM | 2413 | OD1 | ASP | A | 355 | 34.745 | 43.167 | 28.418 | 1.00 | 79.47 | A | O |
| ATOM | 2414 | OD2 | ASP | A | 355 | 36.589 | 42.277 | 27.501 | 1.00 | 79.47 | A | O |
| ATOM | 2415 | C | ASP | A | 355 | 35.659 | 43.292 | 32.017 | 1.00 | 34.98 | A | C |
| ATOM | 2416 | O | ASP | A | 355 | 36.260 | 42.979 | 33.055 | 1.00 | 34.98 | A | O |
| ATOM | 2417 | N | THR | A | 356 | 34.335 | 43.282 | 31.930 | 1.00 | 37.81 | A | N |
| ATOM | 2418 | CA | THR | A | 356 | 33.558 | 42.913 | 33.099 | 1.00 | 37.81 | A | C |
| ATOM | 2419 | CB | THR | A | 356 | 32.061 | 43.251 | 32.949 | 1.00 | 31.34 | A | C |
| ATOM | 2420 | OG1 | THR | A | 356 | 31.344 | 42.080 | 32.553 | 1.00 | 31.34 | A | O |
| ATOM | 2421 | CG2 | THR | A | 356 | 31.861 | 44.322 | 31.918 | 1.00 | 31.34 | A | C |
| ATOM | 2422 | C | THR | A | 356 | 33.729 | 41.408 | 33.234 | 1.00 | 37.81 | A | C |
| ATOM | 2423 | O | THR | A | 356 | 33.905 | 40.703 | 32.234 | 1.00 | 37.81 | A | O |
| ATOM | 2424 | N | PRO | A | 357 | 33.693 | 40.888 | 34.469 | 1.00 | 29.15 | A | N |
| ATOM | 2425 | CD | PRO | A | 357 | 33.378 | 41.510 | 35.770 | 1.00 | 38.66 | A | C |
| ATOM | 2426 | CA | PRO | A | 357 | 33.861 | 39.440 | 34.605 | 1.00 | 29.15 | A | C |
| ATOM | 2427 | CB | PRO | A | 357 | 34.003 | 39.259 | 36.113 | 1.00 | 38.66 | A | C |
| ATOM | 2428 | CG | PRO | A | 357 | 33.049 | 40.305 | 36.645 | 1.00 | 38.66 | A | C |
| ATOM | 2429 | C | PRO | A | 357 | 32.644 | 38.714 | 34.045 | 1.00 | 29.15 | A | C |
| ATOM | 2430 | O | PRO | A | 357 | 31.714 | 39.335 | 33.518 | 1.00 | 29.15 | A | O |
| ATOM | 2431 | N | ALA | A | 358 | 32.661 | 37.393 | 34.153 | 1.00 | 39.07 | A | N |
| ATOM | 2432 | CA | ALA | A | 358 | 31.549 | 36.586 | 33.690 | 1.00 | 39.07 | A | C |
| ATOM | 2433 | CB | ALA | A | 358 | 31.825 | 35.138 | 33.958 | 1.00 | 33.19 | A | C |
| ATOM | 2434 | C | ALA | A | 358 | 30.318 | 37.015 | 34.462 | 1.00 | 39.07 | A | C |
| ATOM | 2435 | O | ALA | A | 358 | 30.358 | 37.100 | 35.686 | 1.00 | 39.07 | A | O |
| ATOM | 2436 | N | LEU | A | 359 | 29.222 | 37.293 | 33.772 | 1.00 | 28.37 | A | N |
| ATOM | 2437 | CA | LEU | A | 359 | 28.014 | 37.681 | 34.483 | 1.00 | 28.37 | A | C |
| ATOM | 2438 | CB | LEU | A | 359 | 27.738 | 39.178 | 34.345 | 1.00 | 25.93 | A | C |
| ATOM | 2439 | CG | LEU | A | 359 | 28.764 | 40.169 | 34.887 | 1.00 | 25.93 | A | C |
| ATOM | 2440 | CD1 | LEU | A | 359 | 28.127 | 41.551 | 34.902 | 1.00 | 25.93 | A | C |
| ATOM | 2441 | CD2 | LEU | A | 359 | 29.214 | 39.781 | 36.280 | 1.00 | 25.93 | A | C |
| ATOM | 2442 | C | LEU | A | 359 | 26.821 | 36.925 | 33.944 | 1.00 | 28.37 | A | C |
| ATOM | 2443 | O | LEU | A | 359 | 25.760 | 36.913 | 34.560 | 1.00 | 28.37 | A | O |
| ATOM | 2444 | N | PHE | A | 360 | 27.003 | 36.283 | 32.795 | 1.00 | 31.80 | A | N |
| ATOM | 2445 | CA | PHE | A | 360 | 25.912 | 35.560 | 32.153 | 1.00 | 31.80 | A | C |
| ATOM | 2446 | CB | PHE | A | 360 | 25.639 | 36.194 | 30.794 | 1.00 | 29.36 | A | C |
| ATOM | 2447 | CG | PHE | A | 360 | 25.803 | 37.681 | 30.782 | 1.00 | 29.36 | A | C |
| ATOM | 2448 | CD1 | PHE | A | 360 | 25.089 | 38.474 | 31.658 | 1.00 | 29.36 | A | C |
| ATOM | 2449 | CD2 | PHE | A | 360 | 26.670 | 38.293 | 29.890 | 1.00 | 29.36 | A | C |
| ATOM | 2450 | CE1 | PHE | A | 360 | 25.231 | 39.864 | 31.640 | 1.00 | 29.36 | A | C |
| ATOM | 2451 | CE2 | PHE | A | 360 | 26.816 | 39.682 | 29.868 | 1.00 | 29.36 | A | C |
| ATOM | 2452 | CZ | PHE | A | 360 | 26.095 | 40.466 | 30.745 | 1.00 | 29.36 | A | C |
| ATOM | 2453 | C | PHE | A | 360 | 26.044 | 34.042 | 31.979 | 1.00 | 31.80 | A | C |
| ATOM | 2454 | O | PHE | A | 360 | 25.123 | 33.405 | 31.502 | 1.00 | 31.80 | A | O |
| ATOM | 2455 | N | ASN | A | 361 | 27.165 | 33.450 | 32.361 | 1.00 | 34.36 | A | N |
| ATOM | 2456 | CA | ASN | A | 361 | 27.320 | 32.008 | 32.215 | 1.00 | 34.36 | A | C |
| ATOM | 2457 | CB | ASN | A | 361 | 28.810 | 31.641 | 32.317 | 1.00 | 41.86 | A | C |
| ATOM | 2458 | CG | ASN | A | 361 | 29.500 | 32.265 | 33.537 | 1.00 | 41.86 | A | C |
| ATOM | 2459 | OD1 | ASN | A | 361 | 29.067 | 33.309 | 34.043 | 1.00 | 41.86 | A | O |
| ATOM | 2460 | ND2 | ASN | A | 361 | 30.597 | 31.637 | 33.999 | 1.00 | 41.86 | A | N |
| ATOM | 2461 | C | ASN | A | 361 | 26.463 | 31.161 | 33.169 | 1.00 | 34.36 | A | C |
| ATOM | 2462 | O | ASN | A | 361 | 26.960 | 30.231 | 33.828 | 1.00 | 34.36 | A | O |
| ATOM | 2463 | N | PHE | A | 362 | 25.167 | 31.480 | 33.210 | 1.00 | 48.71 | A | N |
| ATOM | 2464 | CA | PHE | A | 362 | 24.210 | 30.785 | 34.066 | 1.00 | 48.71 | A | C |
| ATOM | 2465 | CB | PHE | A | 362 | 22.805 | 31.370 | 33.872 | 1.00 | 37.43 | A | C |
| ATOM | 2466 | CG | PHE | A | 362 | 22.604 | 32.706 | 34.543 | 1.00 | 37.43 | A | C |
| ATOM | 2467 | CD1 | PHE | A | 362 | 22.452 | 32.791 | 35.923 | 1.00 | 37.43 | A | C |
| ATOM | 2468 | CD2 | PHE | A | 362 | 22.580 | 33.883 | 33.796 | 1.00 | 37.43 | A | C |
| ATOM | 2469 | CE1 | PHE | A | 362 | 22.279 | 34.022 | 36.540 | 1.00 | 37.43 | A | C |
| ATOM | 2470 | CE2 | PHE | A | 362 | 22.408 | 35.115 | 34.414 | 1.00 | 37.43 | A | C |
| ATOM | 2471 | CZ | PHE | A | 362 | 22.258 | 35.182 | 35.782 | 1.00 | 37.43 | A | C |
| ATOM | 2472 | C | PHE | A | 362 | 24.177 | 29.300 | 33.776 | 1.00 | 48.71 | A | C |
| ATOM | 2473 | O | PHE | A | 362 | 24.822 | 28.823 | 32.847 | 1.00 | 48.71 | A | O |

| | | | | | | | | | | | | |
|---|---|---|---|---|---|---|---|---|---|---|---|---|
| ATOM | 2474 | N | THR | A | 363 | 23.430 | 28.570 | 34.590 | 1.00 | 41.24 | A | N |
| ATOM | 2475 | CA | THR | A | 363 | 23.272 | 27.136 | 34.411 | 1.00 | 41.24 | A | C |
| ATOM | 2476 | CB | THR | A | 363 | 24.049 | 26.365 | 35.474 | 1.00 | 27.92 | A | C |
| ATOM | 2477 | OG1 | THR | A | 363 | 23.456 | 26.588 | 36.760 | 1.00 | 27.92 | A | O |
| ATOM | 2478 | CG2 | THR | A | 363 | 25.494 | 26.832 | 35.494 | 1.00 | 27.92 | A | C |
| ATOM | 2479 | C | THR | A | 363 | 21.777 | 26.882 | 34.590 | 1.00 | 41.24 | A | C |
| ATOM | 2480 | O | THR | A | 363 | 21.014 | 27.802 | 34.902 | 1.00 | 41.24 | A | O |
| ATOM | 2481 | N | THR | A | 364 | 21.342 | 25.650 | 34.386 | 1.00 | 50.91 | A | N |
| ATOM | 2482 | CA | THR | A | 364 | 19.927 | 25.343 | 34.562 | 1.00 | 50.91 | A | C |
| ATOM | 2483 | CB | THR | A | 364 | 19.598 | 24.019 | 33.912 | 1.00 | 49.45 | A | C |
| ATOM | 2484 | OG1 | THR | A | 364 | 20.333 | 22.982 | 34.570 | 1.00 | 49.45 | A | O |
| ATOM | 2485 | CG2 | THR | A | 364 | 19.996 | 24.052 | 32.459 | 1.00 | 49.45 | A | C |
| ATOM | 2486 | C | THR | A | 364 | 19.663 | 25.257 | 36.067 | 1.00 | 50.91 | A | C |
| ATOM | 2487 | O | THR | A | 364 | 18.553 | 25.516 | 36.554 | 1.00 | 50.91 | A | O |
| ATOM | 2488 | N | GLN | A | 365 | 20.710 | 24.884 | 36.792 | 1.00 | 38.49 | A | N |
| ATOM | 2489 | CA | GLN | A | 365 | 20.657 | 24.789 | 38.242 | 1.00 | 38.49 | A | C |
| ATOM | 2490 | CB | GLN | A | 365 | 22.002 | 24.249 | 38.761 | 1.00 | 32.90 | A | C |
| ATOM | 2491 | CG | GLN | A | 365 | 22.134 | 24.200 | 40.266 | 1.00 | 32.90 | A | C |
| ATOM | 2492 | CD | GLN | A | 365 | 21.119 | 23.280 | 40.916 | 1.00 | 32.90 | A | C |
| ATOM | 2493 | OE1 | GLN | A | 365 | 20.077 | 22.974 | 40.329 | 1.00 | 32.90 | A | O |
| ATOM | 2494 | NE2 | GLN | A | 365 | 21.404 | 22.850 | 42.148 | 1.00 | 32.90 | A | N |
| ATOM | 2495 | C | GLN | A | 365 | 20.405 | 26.200 | 38.779 | 1.00 | 38.49 | A | C |
| ATOM | 2496 | O | GLN | A | 365 | 19.539 | 26.412 | 39.629 | 1.00 | 38.49 | A | O |
| ATOM | 2497 | N | GLU | A | 366 | 21.156 | 27.166 | 38.260 | 1.00 | 30.93 | A | N |
| ATOM | 2498 | CA | GLU | A | 366 | 21.017 | 28.542 | 38.695 | 1.00 | 30.93 | A | C |
| ATOM | 2499 | CB | GLU | A | 366 | 22.087 | 29.430 | 38.048 | 1.00 | 33.59 | A | C |
| ATOM | 2500 | CG | GLU | A | 366 | 23.543 | 29.140 | 38.425 | 1.00 | 33.59 | A | C |
| ATOM | 2501 | CD | GLU | A | 366 | 24.510 | 30.281 | 38.039 | 1.00 | 33.59 | A | C |
| ATOM | 2502 | OE1 | GLU | A | 366 | 24.445 | 31.382 | 38.654 | 1.00 | 33.59 | A | O |
| ATOM | 2503 | OE2 | GLU | A | 366 | 25.334 | 30.058 | 37.119 | 1.00 | 33.59 | A | O |
| ATOM | 2504 | C | GLU | A | 366 | 19.662 | 29.123 | 38.336 | 1.00 | 30.93 | A | C |
| ATOM | 2505 | O | GLU | A | 366 | 19.069 | 29.832 | 39.132 | 1.00 | 30.93 | A | O |
| ATOM | 2506 | N | LEU | A | 367 | 19.177 | 28.829 | 37.136 | 1.00 | 30.01 | A | N |
| ATOM | 2507 | CA | LEU | A | 367 | 17.906 | 29.381 | 36.669 | 1.00 | 30.01 | A | C |
| ATOM | 2508 | CB | LEU | A | 367 | 17.976 | 29.567 | 35.149 | 1.00 | 30.23 | A | C |
| ATOM | 2509 | CG | LEU | A | 367 | 19.210 | 30.344 | 34.667 | 1.00 | 30.23 | A | C |
| ATOM | 2510 | CD1 | LEU | A | 367 | 19.511 | 30.004 | 33.236 | 1.00 | 30.23 | A | C |
| ATOM | 2511 | CD2 | LEU | A | 367 | 19.002 | 31.841 | 34.852 | 1.00 | 30.23 | A | C |
| ATOM | 2512 | C | LEU | A | 367 | 16.640 | 28.598 | 37.012 | 1.00 | 30.01 | A | C |
| ATOM | 2513 | O | LEU | A | 367 | 15.527 | 29.051 | 36.709 | 1.00 | 30.01 | A | O |
| ATOM | 2514 | N | SER | A | 368 | 16.809 | 27.435 | 37.642 | 1.00 | 31.94 | A | N |
| ATOM | 2515 | CA | SER | A | 368 | 15.684 | 26.569 | 37.987 | 1.00 | 31.94 | A | C |
| ATOM | 2516 | CB | SER | A | 368 | 16.182 | 25.351 | 38.758 | 1.00 | 41.43 | A | C |
| ATOM | 2517 | OG | SER | A | 368 | 16.778 | 25.746 | 39.977 | 1.00 | 41.43 | A | O |
| ATOM | 2518 | C | SER | A | 368 | 14.526 | 27.208 | 38.755 | 1.00 | 31.94 | A | C |
| ATOM | 2519 | O | SER | A | 368 | 13.387 | 26.780 | 38.619 | 1.00 | 31.94 | A | O |
| ATOM | 2520 | N | SER | A | 369 | 14.807 | 28.225 | 39.558 | 1.00 | 33.00 | A | N |
| ATOM | 2521 | CA | SER | A | 369 | 13.762 | 28.887 | 40.330 | 1.00 | 33.00 | A | C |
| ATOM | 2522 | CB | SER | A | 369 | 14.387 | 29.916 | 41.271 | 1.00 | 38.11 | A | C |
| ATOM | 2523 | OG | SER | A | 369 | 15.415 | 30.661 | 40.631 | 1.00 | 38.11 | A | O |
| ATOM | 2524 | C | SER | A | 369 | 12.738 | 29.566 | 39.437 | 1.00 | 33.00 | A | C |
| ATOM | 2525 | O | SER | A | 369 | 11.603 | 29.778 | 39.840 | 1.00 | 33.00 | A | O |
| ATOM | 2526 | N | ASN | A | 370 | 13.147 | 29.905 | 38.221 | 1.00 | 49.14 | A | N |
| ATOM | 2527 | CA | ASN | A | 370 | 12.268 | 30.584 | 37.273 | 1.00 | 49.14 | A | C |
| ATOM | 2528 | CB | ASN | A | 370 | 11.925 | 31.978 | 37.810 | 1.00 | 35.52 | A | C |
| ATOM | 2529 | CG | ASN | A | 370 | 10.913 | 32.711 | 36.953 | 1.00 | 35.52 | A | C |
| ATOM | 2530 | OD1 | ASN | A | 370 | 10.817 | 32.487 | 35.749 | 1.00 | 35.52 | A | O |
| ATOM | 2531 | ND2 | ASN | A | 370 | 10.162 | 33.611 | 37.570 | 1.00 | 35.52 | A | N |
| ATOM | 2532 | C | ASN | A | 370 | 13.002 | 30.702 | 35.928 | 1.00 | 49.14 | A | C |
| ATOM | 2533 | O | ASN | A | 370 | 13.416 | 31.789 | 35.524 | 1.00 | 49.14 | A | O |
| ATOM | 2534 | N | PRO | A | 371 | 13.177 | 29.578 | 35.214 | 1.00 | 34.57 | A | N |
| ATOM | 2535 | CD | PRO | A | 371 | 12.817 | 28.200 | 35.566 | 1.00 | 22.28 | A | C |
| ATOM | 2536 | CA | PRO | A | 371 | 13.873 | 29.618 | 33.926 | 1.00 | 34.57 | A | C |
| ATOM | 2537 | CB | PRO | A | 371 | 13.607 | 28.231 | 33.337 | 1.00 | 22.28 | A | C |
| ATOM | 2538 | CG | PRO | A | 371 | 12.586 | 27.618 | 34.239 | 1.00 | 22.28 | A | C |
| ATOM | 2539 | C | PRO | A | 371 | 13.542 | 30.762 | 32.970 | 1.00 | 34.57 | A | C |
| ATOM | 2540 | O | PRO | A | 371 | 14.439 | 31.383 | 32.413 | 1.00 | 34.57 | A | O |

| | | | | | | | | | | | | |
|---|---|---|---|---|---|---|---|---|---|---|---|---|
| ATOM | 2541 | N | PRO | A | 372 | 12.260 | 31.063 | 32.768 | 1.00 | 31.58 | A | N |
| ATOM | 2542 | CD | PRO | A | 372 | 11.075 | 30.346 | 33.269 | 1.00 | 35.74 | A | C |
| ATOM | 2543 | CA | PRO | A | 372 | 11.864 | 32.149 | 31.867 | 1.00 | 31.58 | A | C |
| ATOM | 2544 | CB | PRO | A | 372 | 10.399 | 32.336 | 32.206 | 1.00 | 35.74 | A | C |
| ATOM | 2545 | CG | PRO | A | 372 | 9.964 | 30.910 | 32.399 | 1.00 | 35.74 | A | C |
| ATOM | 2546 | C | PRO | A | 372 | 12.661 | 33.442 | 32.010 | 1.00 | 31.58 | A | C |
| ATOM | 2547 | O | PRO | A | 372 | 12.675 | 34.266 | 31.092 | 1.00 | 31.58 | A | O |
| ATOM | 2548 | N | LEU | A | 373 | 13.326 | 33.623 | 33.150 | 1.00 | 41.80 | A | N |
| ATOM | 2549 | CA | LEU | A | 373 | 14.116 | 34.834 | 33.376 | 1.00 | 41.80 | A | C |
| ATOM | 2550 | CB | LEU | A | 373 | 14.411 | 35.020 | 34.868 | 1.00 | 21.32 | A | C |
| ATOM | 2551 | CG | LEU | A | 373 | 13.203 | 35.357 | 35.749 | 1.00 | 21.32 | A | C |
| ATOM | 2552 | CD1 | LEU | A | 373 | 13.623 | 35.433 | 37.217 | 1.00 | 21.32 | A | C |
| ATOM | 2553 | CD2 | LEU | A | 373 | 12.591 | 36.665 | 35.282 | 1.00 | 21.32 | A | C |
| ATOM | 2554 | C | LEU | A | 373 | 15.413 | 34.859 | 32.574 | 1.00 | 41.80 | A | C |
| ATOM | 2555 | O | LEU | A | 373 | 16.115 | 35.867 | 32.535 | 1.00 | 41.80 | A | O |
| ATOM | 2556 | N | ALA | A | 374 | 15.732 | 33.746 | 31.931 | 1.00 | 40.40 | A | N |
| ATOM | 2557 | CA | ALA | A | 374 | 16.934 | 33.680 | 31.105 | 1.00 | 40.40 | A | C |
| ATOM | 2558 | CB | ALA | A | 374 | 17.161 | 32.244 | 30.630 | 1.00 | 34.26 | A | C |
| ATOM | 2559 | C | ALA | A | 374 | 16.778 | 34.635 | 29.907 | 1.00 | 40.40 | A | C |
| ATOM | 2560 | O | ALA | A | 374 | 17.760 | 35.073 | 29.308 | 1.00 | 40.40 | A | O |
| ATOM | 2561 | N | THR | A | 375 | 15.528 | 34.959 | 29.581 | 1.00 | 30.56 | A | N |
| ATOM | 2562 | CA | THR | A | 375 | 15.217 | 35.841 | 28.467 | 1.00 | 30.56 | A | C |
| ATOM | 2563 | CB | THR | A | 375 | 13.684 | 35.968 | 28.256 | 1.00 | 30.03 | A | C |
| ATOM | 2564 | OG1 | THR | A | 375 | 13.087 | 36.565 | 29.415 | 1.00 | 30.03 | A | O |
| ATOM | 2565 | CG2 | THR | A | 375 | 13.058 | 34.601 | 28.030 | 1.00 | 30.03 | A | C |
| ATOM | 2566 | C | THR | A | 375 | 15.757 | 37.217 | 28.770 | 1.00 | 30.56 | A | C |
| ATOM | 2567 | O | THR | A | 375 | 15.964 | 38.010 | 27.861 | 1.00 | 30.56 | A | O |
| ATOM | 2568 | N | ILE | A | 376 | 15.953 | 37.507 | 30.051 | 1.00 | 27.03 | A | N |
| ATOM | 2569 | CA | ILE | A | 376 | 16.499 | 38.791 | 30.461 | 1.00 | 27.03 | A | C |
| ATOM | 2570 | CB | ILE | A | 376 | 15.644 | 39.420 | 31.582 | 1.00 | 27.95 | A | C |
| ATOM | 2571 | CG2 | ILE | A | 376 | 16.303 | 40.696 | 32.081 | 1.00 | 27.95 | A | C |
| ATOM | 2572 | CG1 | ILE | A | 376 | 14.241 | 39.737 | 31.059 | 1.00 | 27.95 | A | C |
| ATOM | 2573 | CD1 | ILE | A | 376 | 13.340 | 40.443 | 32.083 | 1.00 | 27.95 | A | C |
| ATOM | 2574 | C | ILE | A | 376 | 17.928 | 38.627 | 30.972 | 1.00 | 27.03 | A | C |
| ATOM | 2575 | O | ILE | A | 376 | 18.847 | 39.322 | 30.533 | 1.00 | 27.03 | A | O |
| ATOM | 2576 | N | LEU | A | 377 | 18.116 | 37.691 | 31.890 | 1.00 | 36.65 | A | N |
| ATOM | 2577 | CA | LEU | A | 377 | 19.438 | 37.456 | 32.455 | 1.00 | 36.65 | A | C |
| ATOM | 2578 | CB | LEU | A | 377 | 19.371 | 36.249 | 33.400 | 1.00 | 38.07 | A | C |
| ATOM | 2579 | CG | LEU | A | 377 | 18.414 | 36.578 | 34.557 | 1.00 | 38.07 | A | C |
| ATOM | 2580 | CD1 | LEU | A | 377 | 17.786 | 35.328 | 35.108 | 1.00 | 38.07 | A | C |
| ATOM | 2581 | CD2 | LEU | A | 377 | 19.156 | 37.337 | 35.631 | 1.00 | 38.07 | A | C |
| ATOM | 2582 | C | LEU | A | 377 | 20.554 | 37.314 | 31.419 | 1.00 | 36.65 | A | C |
| ATOM | 2583 | O | LEU | A | 377 | 21.637 | 37.875 | 31.618 | 1.00 | 36.65 | A | O |
| ATOM | 2584 | N | ILE | A | 378 | 20.294 | 36.601 | 30.319 | 1.00 | 36.19 | A | N |
| ATOM | 2585 | CA | ILE | A | 378 | 21.306 | 36.430 | 29.270 | 1.00 | 36.19 | A | C |
| ATOM | 2586 | CB | ILE | A | 378 | 21.323 | 35.006 | 28.683 | 1.00 | 22.57 | A | C |
| ATOM | 2587 | CG2 | ILE | A | 378 | 22.478 | 34.890 | 27.720 | 1.00 | 22.57 | A | C |
| ATOM | 2588 | CG1 | ILE | A | 378 | 21.456 | 33.957 | 29.788 | 1.00 | 22.57 | A | C |
| ATOM | 2589 | CD1 | ILE | A | 378 | 20.155 | 33.671 | 30.512 | 1.00 | 22.57 | A | C |
| ATOM | 2590 | C | ILE | A | 378 | 21.021 | 37.387 | 28.122 | 1.00 | 36.19 | A | C |
| ATOM | 2591 | O | ILE | A | 378 | 20.152 | 37.140 | 27.291 | 1.00 | 36.19 | A | O |
| ATOM | 2592 | N | PRO | A | 379 | 21.760 | 38.497 | 28.063 | 1.00 | 35.50 | A | N |
| ATOM | 2593 | CD | PRO | A | 379 | 22.926 | 38.769 | 28.920 | 1.00 | 35.03 | A | C |
| ATOM | 2594 | CA | PRO | A | 379 | 21.612 | 39.520 | 27.024 | 1.00 | 35.50 | A | C |
| ATOM | 2595 | CB | PRO | A | 379 | 22.650 | 40.560 | 27.438 | 1.00 | 35.03 | A | C |
| ATOM | 2596 | CG | PRO | A | 379 | 23.724 | 39.718 | 28.079 | 1.00 | 35.03 | A | C |
| ATOM | 2597 | C | PRO | A | 379 | 21.837 | 38.961 | 25.612 | 1.00 | 35.50 | A | C |
| ATOM | 2598 | O | PRO | A | 379 | 22.551 | 37.984 | 25.422 | 1.00 | 35.50 | A | O |
| ATOM | 2599 | N | PRO | A | 380 | 21.228 | 39.589 | 24.605 | 1.00 | 32.46 | A | N |
| ATOM | 2600 | CD | PRO | A | 380 | 20.441 | 40.824 | 24.738 | 1.00 | 17.91 | A | C |
| ATOM | 2601 | CA | PRO | A | 380 | 21.326 | 39.186 | 23.202 | 1.00 | 32.46 | A | C |
| ATOM | 2602 | CB | PRO | A | 380 | 20.688 | 40.365 | 22.476 | 1.00 | 17.91 | A | C |
| ATOM | 2603 | CG | PRO | A | 380 | 19.676 | 40.857 | 23.454 | 1.00 | 17.91 | A | C |
| ATOM | 2604 | C | PRO | A | 380 | 22.727 | 38.908 | 22.715 | 1.00 | 32.46 | A | C |
| ATOM | 2605 | O | PRO | A | 380 | 22.991 | 37.845 | 22.157 | 1.00 | 32.46 | A | O |
| ATOM | 2606 | N | HIS | A | 381 | 23.622 | 39.864 | 22.945 | 1.00 | 32.83 | A | N |
| ATOM | 2607 | CA | HIS | A | 381 | 25.000 | 39.761 | 22.488 | 1.00 | 32.83 | A | C |

| | | | | | | | | | | | | |
|---|---|---|---|---|---|---|---|---|---|---|---|---|
| ATOM | 2608 | CB | HIS | A | 381 | 25.714 | 41.100 | 22.728 | 1.00 | 42.18 | A | C |
| ATOM | 2609 | CG | HIS | A | 381 | 26.027 | 41.373 | 24.172 | 1.00 | 42.18 | A | C |
| ATOM | 2610 | CD2 | HIS | A | 381 | 25.412 | 42.187 | 25.073 | 1.00 | 42.18 | A | C |
| ATOM | 2611 | ND1 | HIS | A | 381 | 27.062 | 40.767 | 24.824 | 1.00 | 42.18 | A | N |
| ATOM | 2612 | CE1 | HIS | A | 381 | 27.092 | 41.193 | 26.093 | 1.00 | 42.18 | A | C |
| ATOM | 2613 | NE2 | HIS | A | 381 | 26.107 | 42.046 | 26.253 | 1.00 | 42.18 | A | N |
| ATOM | 2614 | C | HIS | A | 381 | 25.749 | 38.626 | 23.126 | 1.00 | 32.83 | A | C |
| ATOM | 2615 | O | HIS | A | 381 | 26.854 | 38.291 | 22.703 | 1.00 | 32.83 | A | O |
| ATOM | 2616 | N | ALA | A | 382 | 25.149 | 38.045 | 24.160 | 1.00 | 35.94 | A | N |
| ATOM | 2617 | CA | ALA | A | 382 | 25.750 | 36.913 | 24.851 | 1.00 | 35.94 | A | C |
| ATOM | 2618 | CB | ALA | A | 382 | 25.304 | 36.881 | 26.284 | 1.00 | 48.18 | A | C |
| ATOM | 2619 | C | ALA | A | 382 | 25.269 | 35.671 | 24.123 | 1.00 | 35.94 | A | C |
| ATOM | 2620 | O | ALA | A | 382 | 25.953 | 34.634 | 24.080 | 1.00 | 35.94 | A | O |
| ATOM | 2621 | N | ARG | A | 383 | 24.079 | 35.787 | 23.536 | 1.00 | 61.46 | A | N |
| ATOM | 2622 | CA | ARG | A | 383 | 23.539 | 34.673 | 22.783 | 1.00 | 61.46 | A | C |
| ATOM | 2623 | CB | ARG | A | 383 | 22.072 | 34.909 | 22.419 | 1.00 | 43.49 | A | C |
| ATOM | 2624 | CG | ARG | A | 383 | 21.123 | 34.447 | 23.550 | 1.00 | 43.49 | A | C |
| ATOM | 2625 | CD | ARG | A | 383 | 19.975 | 35.367 | 23.576 | 1.00 | 43.49 | A | C |
| ATOM | 2626 | NE | ARG | A | 383 | 19.167 | 35.440 | 24.782 | 1.00 | 43.49 | A | N |
| ATOM | 2627 | CZ | ARG | A | 383 | 18.108 | 36.242 | 24.808 | 1.00 | 43.49 | A | C |
| ATOM | 2628 | NH1 | ARG | A | 383 | 17.842 | 36.930 | 23.703 | 1.00 | 43.49 | A | N |
| ATOM | 2629 | NH2 | ARG | A | 383 | 17.339 | 36.393 | 25.880 | 1.00 | 43.49 | A | N |
| ATOM | 2630 | C | ARG | A | 383 | 24.449 | 34.489 | 21.584 | 1.00 | 61.46 | A | C |
| ATOM | 2631 | O | ARG | A | 383 | 24.693 | 33.338 | 21.196 | 1.00 | 61.46 | A | O |
| ATOM | 2632 | N | ILE | A | 384 | 24.959 | 35.628 | 21.064 | 1.00 | 68.42 | A | N |
| ATOM | 2633 | CA | ILE | A | 384 | 25.886 | 35.603 | 20.001 | 1.00 | 68.42 | A | C |
| ATOM | 2634 | CB | ILE | A | 384 | 26.367 | 36.951 | 19.427 | 1.00 | 49.18 | A | C |
| ATOM | 2635 | CG2 | ILE | A | 384 | 27.272 | 36.810 | 18.201 | 1.00 | 49.18 | A | C |
| ATOM | 2636 | CG1 | ILE | A | 384 | 25.121 | 37.519 | 18.765 | 1.00 | 49.18 | A | C |
| ATOM | 2637 | CD1 | ILE | A | 384 | 24.953 | 38.970 | 19.035 | 1.00 | 49.18 | A | C |
| ATOM | 2638 | C | ILE | A | 384 | 26.846 | 34.539 | 20.305 | 1.00 | 68.42 | A | C |
| ATOM | 2639 | O | ILE | A | 384 | 27.613 | 34.386 | 21.334 | 1.00 | 68.42 | A | O |
| ATOM | 2640 | N | ALA | A | 385 | 26.910 | 34.423 | 19.023 | 1.00 | 58.85 | A | N |
| ATOM | 2641 | CA | ALA | A | 385 | 27.193 | 33.362 | 18.203 | 1.00 | 58.85 | A | C |
| ATOM | 2642 | CB | ALA | A | 385 | 26.088 | 33.700 | 17.285 | 1.00 | 76.22 | A | C |
| ATOM | 2643 | C | ALA | A | 385 | 27.847 | 32.166 | 17.518 | 1.00 | 58.85 | A | C |
| ATOM | 2644 | O | ALA | A | 385 | 29.045 | 32.173 | 17.354 | 1.00 | 58.85 | A | O |
| ATOM | 2645 | OXT | ALA | A | 385 | 26.936 | 31.309 | 17.041 | 1.00 | 76.22 | A | O |
| TER | 2646 | | ALA | A | 385 | | | | | | A | |
| ATOM | 2647 | CB | VAL | B | 37 | 14.350 | 82.418 | 78.901 | 1.00 | 42.36 | B | C |
| ATOM | 2648 | CG1 | VAL | B | 37 | 13.496 | 82.004 | 80.100 | 1.00 | 42.36 | B | C |
| ATOM | 2649 | CG2 | VAL | B | 37 | 15.683 | 83.003 | 79.347 | 1.00 | 42.36 | B | C |
| ATOM | 2650 | C | VAL | B | 37 | 13.197 | 80.644 | 77.668 | 1.00 | 47.65 | B | C |
| ATOM | 2651 | O | VAL | B | 37 | 12.400 | 81.301 | 77.002 | 1.00 | 47.65 | B | O |
| ATOM | 2652 | N | VAL | B | 37 | 15.414 | 81.523 | 76.791 | 1.00 | 47.65 | B | N |
| ATOM | 2653 | CA | VAL | B | 37 | 14.582 | 81.195 | 78.003 | 1.00 | 47.65 | B | C |
| ATOM | 2654 | N | THR | B | 38 | 12.914 | 79.436 | 78.139 | 1.00 | 44.52 | B | N |
| ATOM | 2655 | CA | THR | B | 38 | 11.631 | 78.807 | 77.890 | 1.00 | 44.52 | B | C |
| ATOM | 2656 | CB | THR | B | 38 | 11.815 | 77.429 | 77.233 | 1.00 | 31.95 | B | C |
| ATOM | 2657 | OG1 | THR | B | 38 | 12.125 | 77.588 | 75.845 | 1.00 | 31.95 | B | O |
| ATOM | 2658 | CG2 | THR | B | 38 | 10.563 | 76.608 | 77.376 | 1.00 | 31.95 | B | C |
| ATOM | 2659 | C | THR | B | 38 | 10.894 | 78.621 | 79.209 | 1.00 | 44.52 | B | C |
| ATOM | 2660 | O | THR | B | 38 | 11.494 | 78.272 | 80.233 | 1.00 | 44.52 | B | O |
| ATOM | 2661 | N | THR | B | 39 | 9.592 | 78.873 | 79.185 | 1.00 | 27.14 | B | N |
| ATOM | 2662 | CA | THR | B | 39 | 8.777 | 78.721 | 80.377 | 1.00 | 27.14 | B | C |
| ATOM | 2663 | CB | THR | B | 39 | 8.166 | 80.054 | 80.834 | 1.00 | 35.52 | B | C |
| ATOM | 2664 | OG1 | THR | B | 39 | 9.202 | 81.031 | 80.966 | 1.00 | 35.52 | B | O |
| ATOM | 2665 | CG2 | THR | B | 39 | 7.490 | 79.894 | 82.187 | 1.00 | 35.52 | B | C |
| ATOM | 2666 | C | THR | B | 39 | 7.662 | 77.772 | 80.021 | 1.00 | 27.14 | B | C |
| ATOM | 2667 | O | THR | B | 39 | 7.048 | 77.901 | 78.976 | 1.00 | 27.14 | B | O |
| ATOM | 2668 | N | VAL | B | 40 | 7.403 | 76.807 | 80.884 | 1.00 | 26.48 | B | N |
| ATOM | 2669 | CA | VAL | B | 40 | 6.349 | 75.851 | 80.619 | 1.00 | 26.48 | B | C |
| ATOM | 2670 | CB | VAL | B | 40 | 6.932 | 74.606 | 79.887 | 1.00 | 32.96 | B | C |
| ATOM | 2671 | CG1 | VAL | B | 40 | 8.214 | 74.174 | 80.532 | 1.00 | 32.96 | B | C |
| ATOM | 2672 | CG2 | VAL | B | 40 | 5.960 | 73.461 | 79.933 | 1.00 | 32.96 | B | C |
| ATOM | 2673 | C | VAL | B | 40 | 5.721 | 75.461 | 81.945 | 1.00 | 26.48 | B | C |
| ATOM | 2674 | O | VAL | B | 40 | 6.323 | 75.679 | 82.997 | 1.00 | 26.48 | B | O |

| | | | | | | | | | | | |
|---|---|---|---|---|---|---|---|---|---|---|---|
| ATOM | 2675 | N | VAL | B | 41 | 4.501 | 74.930 | 81.901 | 1.00 | 23.20 | B | N |
| ATOM | 2676 | CA | VAL | B | 41 | 3.804 | 74.502 | 83.110 | 1.00 | 23.20 | B | C |
| ATOM | 2677 | CB | VAL | B | 41 | 2.313 | 74.848 | 83.034 | 1.00 | 31.21 | B | C |
| ATOM | 2678 | CG1 | VAL | B | 41 | 1.785 | 74.522 | 81.660 | 1.00 | 31.21 | B | C |
| ATOM | 2679 | CG2 | VAL | B | 41 | 1.548 | 74.074 | 84.074 | 1.00 | 31.21 | B | C |
| ATOM | 2680 | C | VAL | B | 41 | 3.980 | 72.996 | 83.101 | 1.00 | 23.20 | B | C |
| ATOM | 2681 | O | VAL | B | 41 | 3.522 | 72.327 | 82.178 | 1.00 | 23.20 | B | O |
| ATOM | 2682 | N | ALA | B | 42 | 4.677 | 72.473 | 84.110 | 1.00 | 31.57 | B | N |
| ATOM | 2683 | CA | ALA | B | 42 | 4.944 | 71.042 | 84.194 | 1.00 | 31.57 | B | C |
| ATOM | 2684 | CB | ALA | B | 42 | 6.430 | 70.793 | 84.064 | 1.00 | 8.97 | B | C |
| ATOM | 2685 | C | ALA | B | 42 | 4.431 | 70.372 | 85.454 | 1.00 | 31.57 | B | C |
| ATOM | 2686 | O | ALA | B | 42 | 4.234 | 70.995 | 86.493 | 1.00 | 31.57 | B | O |
| ATOM | 2687 | N | THR | B | 43 | 4.221 | 69.072 | 85.334 | 1.00 | 35.31 | B | N |
| ATOM | 2688 | CA | THR | B | 43 | 3.724 | 68.266 | 86.422 | 1.00 | 35.31 | B | C |
| ATOM | 2689 | CB | THR | B | 43 | 2.760 | 67.198 | 85.885 | 1.00 | 41.63 | B | C |
| ATOM | 2690 | OG1 | THR | B | 43 | 1.651 | 67.845 | 85.246 | 1.00 | 41.63 | B | O |
| ATOM | 2691 | CG2 | THR | B | 43 | 2.252 | 66.304 | 87.015 | 1.00 | 41.63 | B | C |
| ATOM | 2692 | C | THR | B | 43 | 4.897 | 67.596 | 87.107 | 1.00 | 35.31 | B | C |
| ATOM | 2693 | O | THR | B | 43 | 5.739 | 66.991 | 86.455 | 1.00 | 35.31 | B | O |
| ATOM | 2694 | N | PRO | B | 44 | 4.995 | 67.740 | 88.430 | 1.00 | 36.80 | B | N |
| ATOM | 2695 | CD | PRO | B | 44 | 4.348 | 68.814 | 89.208 | 1.00 | 31.41 | B | C |
| ATOM | 2696 | CA | PRO | B | 44 | 6.075 | 67.130 | 89.205 | 1.00 | 36.80 | B | C |
| ATOM | 2697 | CB | PRO | B | 44 | 5.763 | 67.587 | 90.619 | 1.00 | 31.41 | B | C |
| ATOM | 2698 | CG | PRO | B | 44 | 5.319 | 68.999 | 90.373 | 1.00 | 31.41 | B | C |
| ATOM | 2699 | C | PRO | B | 44 | 6.102 | 65.605 | 89.057 | 1.00 | 36.80 | B | C |
| ATOM | 2700 | O | PRO | B | 44 | 5.056 | 64.942 | 89.089 | 1.00 | 36.80 | B | O |
| ATOM | 2701 | N | GLY | B | 45 | 7.311 | 65.067 | 88.891 | 1.00 | 35.89 | B | N |
| ATOM | 2702 | CA | GLY | B | 45 | 7.494 | 63.637 | 88.725 | 1.00 | 35.89 | B | C |
| ATOM | 2703 | C | GLY | B | 45 | 6.731 | 62.881 | 89.791 | 1.00 | 35.89 | B | C |
| ATOM | 2704 | O | GLY | B | 45 | 5.745 | 62.169 | 89.522 | 1.00 | 35.89 | B | O |
| ATOM | 2705 | N | ALA | B | 46 | 7.182 | 63.041 | 91.024 | 1.00 | 49.05 | B | N |
| ATOM | 2706 | CA | ALA | B | 46 | 6.515 | 62.375 | 92.126 | 1.00 | 49.05 | B | C |
| ATOM | 2707 | CB | ALA | B | 46 | 7.491 | 62.175 | 93.290 | 1.00 | 41.31 | B | C |
| ATOM | 2708 | C | ALA | B | 46 | 5.323 | 63.231 | 92.569 | 1.00 | 49.05 | B | C |
| ATOM | 2709 | O | ALA | B | 46 | 4.769 | 64.012 | 91.783 | 1.00 | 49.05 | B | O |
| ATOM | 2710 | N | GLY | B | 47 | 4.919 | 63.064 | 93.822 | 1.00 | 43.71 | B | N |
| ATOM | 2711 | CA | GLY | B | 47 | 3.825 | 63.854 | 94.357 | 1.00 | 43.71 | B | C |
| ATOM | 2712 | C | GLY | B | 47 | 2.527 | 63.931 | 93.566 | 1.00 | 43.71 | B | C |
| ATOM | 2713 | O | GLY | B | 47 | 2.409 | 63.351 | 92.482 | 1.00 | 43.71 | B | O |
| ATOM | 2714 | N | PRO | B | 48 | 1.520 | 64.648 | 94.105 | 1.00 | 60.64 | B | N |
| ATOM | 2715 | CD | PRO | B | 48 | 1.592 | 65.364 | 95.394 | 1.00 | 66.43 | B | C |
| ATOM | 2716 | CA | PRO | B | 48 | 0.198 | 64.826 | 93.474 | 1.00 | 60.64 | B | C |
| ATOM | 2717 | CB | PRO | B | 48 | -0.664 | 65.337 | 94.623 | 1.00 | 66.43 | B | C |
| ATOM | 2718 | CG | PRO | B | 48 | 0.322 | 66.213 | 95.379 | 1.00 | 66.43 | B | C |
| ATOM | 2719 | C | PRO | B | 48 | 0.239 | 65.815 | 92.297 | 1.00 | 60.64 | B | C |
| ATOM | 2720 | O | PRO | B | 48 | 0.897 | 66.857 | 92.377 | 1.00 | 60.64 | B | O |
| ATOM | 2721 | N | ASP | B | 49 | -0.489 | 65.497 | 91.230 | 1.00 | 43.48 | B | N |
| ATOM | 2722 | CA | ASP | B | 49 | -0.510 | 66.325 | 90.029 | 1.00 | 43.48 | B | C |
| ATOM | 2723 | CB | ASP | B | 49 | -1.445 | 65.689 | 88.988 | 1.00 | 68.80 | B | C |
| ATOM | 2724 | CG | ASP | B | 49 | -1.056 | 64.238 | 88.660 | 1.00 | 68.80 | B | C |
| ATOM | 2725 | OD1 | ASP | B | 49 | -0.765 | 63.936 | 87.466 | 1.00 | 68.80 | B | O |
| ATOM | 2726 | OD2 | ASP | B | 49 | -1.040 | 63.410 | 89.612 | 1.00 | 68.80 | B | O |
| ATOM | 2727 | C | ASP | B | 49 | -0.853 | 67.795 | 90.205 | 1.00 | 43.48 | B | C |
| ATOM | 2728 | O | ASP | B | 49 | -1.944 | 68.226 | 89.844 | 1.00 | 43.48 | B | O |
| ATOM | 2729 | N | ARG | B | 50 | 0.106 | 68.550 | 90.741 | 1.00 | 46.20 | B | N |
| ATOM | 2730 | CA | ARG | B | 50 | 0.002 | 69.994 | 90.988 | 1.00 | 46.20 | B | C |
| ATOM | 2731 | CB | ARG | B | 50 | 0.477 | 70.308 | 92.404 | 1.00 | 57.67 | B | C |
| ATOM | 2732 | CG | ARG | B | 50 | -0.589 | 70.399 | 93.474 | 1.00 | 57.67 | B | C |
| ATOM | 2733 | CD | ARG | B | 50 | -1.591 | 69.268 | 93.392 | 1.00 | 57.67 | B | C |
| ATOM | 2734 | NE | ARG | B | 50 | -2.632 | 69.551 | 92.402 | 1.00 | 57.67 | B | N |
| ATOM | 2735 | CZ | ARG | B | 50 | -3.940 | 69.423 | 92.632 | 1.00 | 57.67 | B | C |
| ATOM | 2736 | NH1 | ARG | B | 50 | -4.351 | 69.020 | 93.835 | 1.00 | 57.67 | B | N |
| ATOM | 2737 | NH2 | ARG | B | 50 | -4.830 | 69.681 | 91.663 | 1.00 | 57.67 | B | N |
| ATOM | 2738 | C | ARG | B | 50 | 0.936 | 70.713 | 90.002 | 1.00 | 46.20 | B | C |
| ATOM | 2739 | O | ARG | B | 50 | 2.085 | 71.007 | 90.332 | 1.00 | 46.20 | B | O |
| ATOM | 2740 | N | PRO | B | 51 | 0.467 | 70.988 | 88.781 | 1.00 | 44.00 | B | N |
| ATOM | 2741 | CD | PRO | B | 51 | -0.637 | 70.290 | 88.098 | 1.00 | 31.45 | B | C |

| | | | | | | | | | | | | |
|---|---|---|---|---|---|---|---|---|---|---|---|---|
| ATOM | 2742 | CA | PRO | B | 51 | 1.321 | 71.674 | 87.804 | 1.00 | 44.00 | B | C |
| ATOM | 2743 | CB | PRO | B | 51 | 0.492 | 71.604 | 86.525 | 1.00 | 31.45 | B | C |
| ATOM | 2744 | CG | PRO | B | 51 | -0.160 | 70.269 | 86.661 | 1.00 | 31.45 | B | C |
| ATOM | 2745 | C | PRO | B | 51 | 1.805 | 73.073 | 88.105 | 1.00 | 44.00 | B | C |
| ATOM | 2746 | O | PRO | B | 51 | 1.000 | 73.972 | 88.290 | 1.00 | 44.00 | B | O |
| ATOM | 2747 | N | GLN | B | 52 | 3.123 | 73.255 | 88.141 | 1.00 | 27.69 | B | N |
| ATOM | 2748 | CA | GLN | B | 52 | 3.698 | 74.569 | 88.399 | 1.00 | 27.69 | B | C |
| ATOM | 2749 | CB | GLN | B | 52 | 4.576 | 74.542 | 89.649 | 1.00 | 42.73 | B | C |
| ATOM | 2750 | CG | GLN | B | 52 | 5.826 | 73.695 | 89.531 | 1.00 | 42.73 | B | C |
| ATOM | 2751 | CD | GLN | B | 52 | 6.203 | 73.035 | 90.859 | 1.00 | 42.73 | B | C |
| ATOM | 2752 | OE1 | GLN | B | 52 | 7.390 | 72.883 | 91.179 | 1.00 | 42.73 | B | O |
| ATOM | 2753 | NE2 | GLN | B | 52 | 5.185 | 72.626 | 91.634 | 1.00 | 42.73 | B | N |
| ATOM | 2754 | C | GLN | B | 52 | 4.484 | 75.084 | 87.199 | 1.00 | 27.69 | B | C |
| ATOM | 2755 | O | GLN | B | 52 | 4.738 | 74.354 | 86.238 | 1.00 | 27.69 | B | O |
| ATOM | 2756 | N | GLU | B | 53 | 4.842 | 76.360 | 87.243 | 1.00 | 38.58 | B | N |
| ATOM | 2757 | CA | GLU | B | 53 | 5.609 | 76.944 | 86.160 | 1.00 | 38.58 | B | C |
| ATOM | 2758 | CB | GLU | B | 53 | 5.288 | 78.446 | 86.055 | 1.00 | 75.47 | B | C |
| ATOM | 2759 | CG | GLU | B | 53 | 3.796 | 78.696 | 85.712 | 1.00 | 75.47 | B | C |
| ATOM | 2760 | CD | GLU | B | 53 | 3.288 | 80.124 | 86.028 | 1.00 | 75.47 | B | C |
| ATOM | 2761 | OE1 | GLU | B | 53 | 3.861 | 80.746 | 86.972 | 1.00 | 75.47 | B | O |
| ATOM | 2762 | OE2 | GLU | B | 53 | 2.311 | 80.588 | 85.347 | 1.00 | 75.47 | B | O |
| ATOM | 2763 | C | GLU | B | 53 | 7.085 | 76.661 | 86.357 | 1.00 | 38.58 | B | C |
| ATOM | 2764 | O | GLU | B | 53 | 7.597 | 76.723 | 87.474 | 1.00 | 38.58 | B | O |
| ATOM | 2765 | N | VAL | B | 54 | 7.741 | 76.296 | 85.260 | 1.00 | 42.21 | B | N |
| ATOM | 2766 | CA | VAL | B | 54 | 9.159 | 75.951 | 85.244 | 1.00 | 42.21 | B | C |
| ATOM | 2767 | CB | VAL | B | 54 | 9.335 | 74.425 | 85.101 | 1.00 | 31.58 | B | C |
| ATOM | 2768 | CG1 | VAL | B | 54 | 10.813 | 74.074 | 85.015 | 1.00 | 31.58 | B | C |
| ATOM | 2769 | CG2 | VAL | B | 54 | 8.671 | 73.716 | 86.286 | 1.00 | 31.58 | B | C |
| ATOM | 2770 | C | VAL | B | 54 | 9.796 | 76.655 | 84.055 | 1.00 | 42.21 | B | C |
| ATOM | 2771 | O | VAL | B | 54 | 9.248 | 76.640 | 82.950 | 1.00 | 42.21 | B | O |
| ATOM | 2772 | N | SER | B | 55 | 10.945 | 77.284 | 84.290 | 1.00 | 52.24 | B | N |
| ATOM | 2773 | CA | SER | B | 55 | 11.653 | 77.990 | 83.232 | 1.00 | 52.24 | B | C |
| ATOM | 2774 | CB | SER | B | 55 | 11.562 | 79.497 | 83.463 | 1.00 | 55.76 | B | C |
| ATOM | 2775 | OG | SER | B | 55 | 10.205 | 79.912 | 83.435 | 1.00 | 55.76 | B | O |
| ATOM | 2776 | C | SER | B | 55 | 13.102 | 77.537 | 83.158 | 1.00 | 52.24 | B | C |
| ATOM | 2777 | O | SER | B | 55 | 13.789 | 77.419 | 84.172 | 1.00 | 52.24 | B | O |
| ATOM | 2778 | N | TYR | B | 56 | 13.551 | 77.268 | 81.942 | 1.00 | 39.27 | B | N |
| ATOM | 2779 | CA | TYR | B | 56 | 14.904 | 76.808 | 81.713 | 1.00 | 39.27 | B | C |
| ATOM | 2780 | CB | TYR | B | 56 | 14.875 | 75.290 | 81.510 | 1.00 | 28.30 | B | C |
| ATOM | 2781 | CG | TYR | B | 56 | 14.041 | 74.820 | 80.318 | 1.00 | 28.30 | B | C |
| ATOM | 2782 | CD1 | TYR | B | 56 | 14.569 | 74.808 | 79.016 | 1.00 | 28.30 | B | C |
| ATOM | 2783 | CE1 | TYR | B | 56 | 13.817 | 74.339 | 77.926 | 1.00 | 28.30 | B | C |
| ATOM | 2784 | CD2 | TYR | B | 56 | 12.737 | 74.355 | 80.496 | 1.00 | 28.30 | B | C |
| ATOM | 2785 | CE2 | TYR | B | 56 | 11.980 | 73.884 | 79.414 | 1.00 | 28.30 | B | C |
| ATOM | 2786 | CZ | TYR | B | 56 | 12.527 | 73.880 | 78.137 | 1.00 | 28.30 | B | C |
| ATOM | 2787 | OH | TYR | B | 56 | 11.777 | 73.419 | 77.081 | 1.00 | 28.30 | B | O |
| ATOM | 2788 | C | TYR | B | 56 | 15.500 | 77.493 | 80.484 | 1.00 | 39.27 | B | C |
| ATOM | 2789 | O | TYR | B | 56 | 14.781 | 77.939 | 79.573 | 1.00 | 39.27 | B | O |
| ATOM | 2790 | N | THR | B | 57 | 16.827 | 77.549 | 80.455 | 1.00 | 57.44 | B | N |
| ATOM | 2791 | CA | THR | B | 57 | 17.522 | 78.187 | 79.353 | 1.00 | 57.44 | B | C |
| ATOM | 2792 | CB | THR | B | 57 | 17.796 | 79.655 | 79.720 | 1.00 | 40.39 | B | C |
| ATOM | 2793 | OG1 | THR | B | 57 | 18.044 | 80.416 | 78.528 | 1.00 | 40.39 | B | O |
| ATOM | 2794 | CG2 | THR | B | 57 | 18.978 | 79.742 | 80.696 | 1.00 | 40.39 | B | C |
| ATOM | 2795 | C | THR | B | 57 | 18.822 | 77.448 | 79.008 | 1.00 | 57.44 | B | C |
| ATOM | 2796 | O | THR | B | 57 | 19.197 | 76.480 | 79.683 | 1.00 | 57.44 | B | O |
| ATOM | 2797 | N | ASP | B | 58 | 19.498 | 77.905 | 77.955 | 1.00 | 37.63 | B | N |
| ATOM | 2798 | CA | ASP | B | 58 | 20.746 | 77.298 | 77.510 | 1.00 | 37.63 | B | C |
| ATOM | 2799 | CB | ASP | B | 58 | 21.804 | 77.253 | 78.626 | 1.00 | 50.66 | B | C |
| ATOM | 2800 | CG | ASP | B | 58 | 21.902 | 78.555 | 79.396 | 1.00 | 50.66 | B | C |
| ATOM | 2801 | OD1 | ASP | B | 58 | 21.812 | 79.627 | 78.757 | 1.00 | 50.66 | B | O |
| ATOM | 2802 | OD2 | ASP | B | 58 | 22.074 | 78.500 | 80.637 | 1.00 | 50.66 | B | O |
| ATOM | 2803 | C | ASP | B | 58 | 20.442 | 75.892 | 77.082 | 1.00 | 37.63 | B | C |
| ATOM | 2804 | O | ASP | B | 58 | 21.172 | 74.958 | 77.408 | 1.00 | 37.63 | B | O |
| ATOM | 2805 | N | THR | B | 59 | 19.336 | 75.739 | 76.372 | 1.00 | 49.68 | B | N |
| ATOM | 2806 | CA | THR | B | 59 | 18.987 | 74.429 | 75.869 | 1.00 | 49.68 | B | C |
| ATOM | 2807 | CB | THR | B | 59 | 17.670 | 74.489 | 75.081 | 1.00 | 44.03 | B | C |
| ATOM | 2808 | OG1 | THR | B | 59 | 16.586 | 74.620 | 76.007 | 1.00 | 44.03 | B | O |

| | | | | | | | | | | | | |
|---|---|---|---|---|---|---|---|---|---|---|---|---|
| ATOM | 2809 | CG2 | THR | B | 59 | 17.467 | 73.233 | 74.244 | 1.00 | 44.03 | B | C |
| ATOM | 2810 | C | THR | B | 59 | 20.154 | 74.078 | 74.948 | 1.00 | 49.68 | B | C |
| ATOM | 2811 | O | THR | B | 59 | 20.974 | 74.942 | 74.624 | 1.00 | 49.68 | B | O |
| ATOM | 2812 | N | LYS | B | 60 | 20.253 | 72.808 | 74.574 | 1.00 | 61.63 | B | N |
| ATOM | 2813 | CA | LYS | B | 60 | 21.292 | 72.334 | 73.669 | 1.00 | 61.63 | B | C |
| ATOM | 2814 | CB | LYS | B | 60 | 22.698 | 72.579 | 74.257 | 1.00 | 45.29 | B | C |
| ATOM | 2815 | CG | LYS | B | 60 | 23.269 | 71.437 | 75.104 | 1.00 | 45.29 | B | C |
| ATOM | 2816 | CD | LYS | B | 60 | 24.762 | 71.647 | 75.449 | 1.00 | 45.29 | B | C |
| ATOM | 2817 | CE | LYS | B | 60 | 24.989 | 72.588 | 76.656 | 1.00 | 45.29 | B | C |
| ATOM | 2818 | NZ | LYS | B | 60 | 24.639 | 74.042 | 76.429 | 1.00 | 45.29 | B | N |
| ATOM | 2819 | C | LYS | B | 60 | 21.023 | 70.839 | 73.480 | 1.00 | 61.63 | B | C |
| ATOM | 2820 | O | LYS | B | 60 | 20.705 | 70.128 | 74.449 | 1.00 | 61.63 | B | O |
| ATOM | 2821 | N | VAL | B | 61 | 21.100 | 70.373 | 72.234 | 1.00 | 46.94 | B | N |
| ATOM | 2822 | CA | VAL | B | 61 | 20.870 | 68.961 | 71.956 | 1.00 | 46.94 | B | C |
| ATOM | 2823 | CB | VAL | B | 61 | 20.832 | 68.680 | 70.457 | 1.00 | 31.47 | B | C |
| ATOM | 2824 | CG1 | VAL | B | 61 | 20.426 | 67.239 | 70.227 | 1.00 | 31.47 | B | C |
| ATOM | 2825 | CG2 | VAL | B | 61 | 19.857 | 69.633 | 69.778 | 1.00 | 31.47 | B | C |
| ATOM | 2826 | C | VAL | B | 61 | 22.050 | 68.200 | 72.565 | 1.00 | 46.94 | B | C |
| ATOM | 2827 | O | VAL | B | 61 | 23.163 | 68.725 | 72.624 | 1.00 | 46.94 | B | O |
| ATOM | 2828 | N | ILE | B | 62 | 21.816 | 66.983 | 73.048 | 1.00 | 42.00 | B | N |
| ATOM | 2829 | CA | ILE | B | 62 | 22.901 | 66.202 | 73.641 | 1.00 | 42.00 | B | C |
| ATOM | 2830 | CB | ILE | B | 62 | 22.853 | 66.204 | 75.179 | 1.00 | 32.41 | B | C |
| ATOM | 2831 | CG2 | ILE | B | 62 | 22.915 | 67.619 | 75.688 | 1.00 | 32.41 | B | C |
| ATOM | 2832 | CG1 | ILE | B | 62 | 21.585 | 65.503 | 75.673 | 1.00 | 32.41 | B | C |
| ATOM | 2833 | CD1 | ILE | B | 62 | 21.556 | 65.289 | 77.175 | 1.00 | 32.41 | B | C |
| ATOM | 2834 | C | ILE | B | 62 | 22.785 | 64.769 | 73.206 | 1.00 | 42.00 | B | C |
| ATOM | 2835 | O | ILE | B | 62 | 23.616 | 63.916 | 73.531 | 1.00 | 42.00 | B | O |
| ATOM | 2836 | N | GLY | B | 63 | 21.705 | 64.520 | 72.494 | 1.00 | 48.40 | B | N |
| ATOM | 2837 | CA | GLY | B | 63 | 21.442 | 63.211 | 71.967 | 1.00 | 48.40 | B | C |
| ATOM | 2838 | C | GLY | B | 63 | 20.320 | 63.443 | 71.002 | 1.00 | 48.40 | B | C |
| ATOM | 2839 | O | GLY | B | 63 | 19.672 | 64.501 | 71.006 | 1.00 | 48.40 | B | O |
| ATOM | 2840 | N | ASN | B | 64 | 20.140 | 62.478 | 70.124 | 1.00 | 77.09 | B | N |
| ATOM | 2841 | CA | ASN | B | 64 | 19.041 | 62.492 | 69.181 | 1.00 | 77.09 | B | C |
| ATOM | 2842 | CB | ASN | B | 64 | 19.605 | 62.422 | 67.771 | 1.00 | 79.31 | B | C |
| ATOM | 2843 | CG | ASN | B | 64 | 19.627 | 63.793 | 67.102 | 1.00 | 79.31 | B | C |
| ATOM | 2844 | OD1 | ASN | B | 64 | 18.564 | 64.360 | 66.772 | 1.00 | 79.31 | B | O |
| ATOM | 2845 | ND2 | ASN | B | 64 | 20.839 | 64.356 | 66.926 | 1.00 | 79.31 | B | N |
| ATOM | 2846 | C | ASN | B | 64 | 18.585 | 61.183 | 69.736 | 1.00 | 77.09 | B | C |
| ATOM | 2847 | O | ASN | B | 64 | 18.095 | 61.121 | 70.882 | 1.00 | 77.09 | B | O |
| ATOM | 2848 | N | GLY | B | 65 | 18.768 | 60.121 | 68.980 | 1.00 | 99.74 | B | N |
| ATOM | 2849 | CA | GLY | B | 65 | 18.415 | 58.861 | 69.572 | 1.00 | 99.74 | B | C |
| ATOM | 2850 | C | GLY | B | 65 | 17.031 | 58.335 | 69.343 | 1.00 | 99.74 | B | C |
| ATOM | 2851 | O | GLY | B | 65 | 15.997 | 58.893 | 69.773 | 1.00 | 99.74 | B | O |
| ATOM | 2852 | N | SER | B | 66 | 17.055 | 57.260 | 68.571 | 1.00 | 75.59 | B | N |
| ATOM | 2853 | CA | SER | B | 66 | 15.891 | 56.457 | 68.315 | 1.00 | 75.59 | B | C |
| ATOM | 2854 | CB | SER | B | 66 | 16.017 | 55.273 | 69.283 | 1.00 | 95.52 | B | C |
| ATOM | 2855 | OG | SER | B | 66 | 16.715 | 55.736 | 70.444 | 1.00 | 95.52 | B | O |
| ATOM | 2856 | C | SER | B | 66 | 14.546 | 57.171 | 68.546 | 1.00 | 75.59 | B | C |
| ATOM | 2857 | O | SER | B | 66 | 13.934 | 57.708 | 67.607 | 1.00 | 75.59 | B | O |
| ATOM | 2858 | N | ALA | B | 67 | 14.113 | 57.158 | 69.812 | 1.00 | 52.84 | B | N |
| ATOM | 2859 | CA | ALA | B | 67 | 12.836 | 57.740 | 70.225 | 1.00 | 52.84 | B | C |
| ATOM | 2860 | CB | ALA | B | 67 | 12.566 | 57.411 | 71.692 | 1.00 | 51.78 | B | C |
| ATOM | 2861 | C | ALA | B | 67 | 12.691 | 59.245 | 69.986 | 1.00 | 52.84 | B | C |
| ATOM | 2862 | O | ALA | B | 67 | 11.665 | 59.709 | 69.470 | 1.00 | 52.84 | B | O |
| ATOM | 2863 | N | GLY | B | 68 | 13.710 | 60.008 | 70.354 | 1.00 | 75.27 | B | N |
| ATOM | 2864 | CA | GLY | B | 68 | 13.632 | 61.441 | 70.144 | 1.00 | 75.27 | B | C |
| ATOM | 2865 | C | GLY | B | 68 | 14.794 | 62.213 | 70.741 | 1.00 | 75.27 | B | C |
| ATOM | 2866 | O | GLY | B | 68 | 15.546 | 61.713 | 71.603 | 1.00 | 75.27 | B | O |
| ATOM | 2867 | N | VAL | B | 69 | 14.938 | 63.452 | 70.285 | 1.00 | 49.39 | B | N |
| ATOM | 2868 | CA | VAL | B | 69 | 16.018 | 64.298 | 70.761 | 1.00 | 49.39 | B | C |
| ATOM | 2869 | CB | VAL | B | 69 | 15.952 | 65.715 | 70.124 | 1.00 | 53.98 | B | C |
| ATOM | 2870 | CG1 | VAL | B | 69 | 16.977 | 66.648 | 70.790 | 1.00 | 53.98 | B | C |
| ATOM | 2871 | CG2 | VAL | B | 69 | 16.235 | 65.617 | 68.631 | 1.00 | 53.98 | B | C |
| ATOM | 2872 | C | VAL | B | 69 | 16.017 | 64.440 | 72.282 | 1.00 | 49.39 | B | C |
| ATOM | 2873 | O | VAL | B | 69 | 14.963 | 64.570 | 72.914 | 1.00 | 49.39 | B | O |
| ATOM | 2874 | N | VAL | B | 70 | 17.210 | 64.386 | 72.863 | 1.00 | 40.04 | B | N |
| ATOM | 2875 | CA | VAL | B | 70 | 17.360 | 64.556 | 74.295 | 1.00 | 40.04 | B | C |

| | | | | | | | | | | | | |
|---|---|---|---|---|---|---|---|---|---|---|---|---|
| ATOM | 2876 | CB | VAL | B | 70 | 18.012 | 63.313 | 74.958 | 1.00 | 22.25 | B | C |
| ATOM | 2877 | CG1 | VAL | B | 70 | 18.282 | 63.579 | 76.417 | 1.00 | 22.25 | B | C |
| ATOM | 2878 | CG2 | VAL | B | 70 | 17.097 | 62.112 | 74.819 | 1.00 | 22.25 | B | C |
| ATOM | 2879 | C | VAL | B | 70 | 18.267 | 65.773 | 74.440 | 1.00 | 40.04 | B | C |
| ATOM | 2880 | O | VAL | B | 70 | 19.384 | 65.789 | 73.895 | 1.00 | 40.04 | B | O |
| ATOM | 2881 | N | TYR | B | 71 | 17.774 | 66.794 | 75.150 | 1.00 | 35.91 | B | N |
| ATOM | 2882 | CA | TYR | B | 71 | 18.541 | 68.021 | 75.353 | 1.00 | 35.91 | B | C |
| ATOM | 2883 | CB | TYR | B | 71 | 17.712 | 69.281 | 75.108 | 1.00 | 53.07 | B | C |
| ATOM | 2884 | CG | TYR | B | 71 | 16.812 | 69.294 | 73.903 | 1.00 | 53.07 | B | C |
| ATOM | 2885 | CD1 | TYR | B | 71 | 15.689 | 68.471 | 73.839 | 1.00 | 53.07 | B | C |
| ATOM | 2886 | CE1 | TYR | B | 71 | 14.796 | 68.560 | 72.776 | 1.00 | 53.07 | B | C |
| ATOM | 2887 | CD2 | TYR | B | 71 | 17.029 | 70.203 | 72.866 | 1.00 | 53.07 | B | C |
| ATOM | 2888 | CE2 | TYR | B | 71 | 16.146 | 70.304 | 71.794 | 1.00 | 53.07 | B | C |
| ATOM | 2889 | CZ | TYR | B | 71 | 15.028 | 69.481 | 71.753 | 1.00 | 53.07 | B | C |
| ATOM | 2890 | OH | TYR | B | 71 | 14.145 | 69.580 | 70.693 | 1.00 | 53.07 | B | O |
| ATOM | 2891 | C | TYR | B | 71 | 19.028 | 68.136 | 76.772 | 1.00 | 35.91 | B | C |
| ATOM | 2892 | O | TYR | B | 71 | 18.704 | 67.318 | 77.616 | 1.00 | 35.91 | B | O |
| ATOM | 2893 | N | GLN | B | 72 | 19.807 | 69.180 | 77.011 | 1.00 | 46.68 | B | N |
| ATOM | 2894 | CA | GLN | B | 72 | 20.334 | 69.508 | 78.325 | 1.00 | 46.68 | B | C |
| ATOM | 2895 | CB | GLN | B | 72 | 21.859 | 69.431 | 78.318 | 1.00 | 35.65 | B | C |
| ATOM | 2896 | CG | GLN | B | 72 | 22.550 | 70.623 | 78.964 | 1.00 | 35.65 | B | C |
| ATOM | 2897 | CD | GLN | B | 72 | 23.500 | 70.231 | 80.091 | 1.00 | 35.65 | B | C |
| ATOM | 2898 | OE1 | GLN | B | 72 | 24.283 | 71.051 | 80.554 | 1.00 | 35.65 | B | O |
| ATOM | 2899 | NE2 | GLN | B | 72 | 23.429 | 68.982 | 80.538 | 1.00 | 35.65 | B | N |
| ATOM | 2900 | C | GLN | B | 72 | 19.868 | 70.955 | 78.525 | 1.00 | 46.68 | B | C |
| ATOM | 2901 | O | GLN | B | 72 | 19.673 | 71.688 | 77.537 | 1.00 | 46.68 | B | O |
| ATOM | 2902 | N | ALA | B | 73 | 19.690 | 71.377 | 79.775 | 1.00 | 35.65 | B | N |
| ATOM | 2903 | CA | ALA | B | 73 | 19.232 | 72.738 | 80.012 | 1.00 | 35.65 | B | C |
| ATOM | 2904 | CB | ALA | B | 73 | 17.748 | 72.846 | 79.709 | 1.00 | 14.29 | B | C |
| ATOM | 2905 | C | ALA | B | 73 | 19.499 | 73.195 | 81.421 | 1.00 | 35.65 | B | C |
| ATOM | 2906 | O | ALA | B | 73 | 20.031 | 72.438 | 82.238 | 1.00 | 35.65 | B | O |
| ATOM | 2907 | N | LYS | B | 74 | 19.117 | 74.439 | 81.702 | 1.00 | 31.17 | B | N |
| ATOM | 2908 | CA | LYS | B | 74 | 19.321 | 75.015 | 83.022 | 1.00 | 31.17 | B | C |
| ATOM | 2909 | CB | LYS | B | 74 | 20.386 | 76.119 | 82.950 | 1.00 | 44.34 | B | C |
| ATOM | 2910 | CG | LYS | B | 74 | 20.924 | 76.609 | 84.298 | 1.00 | 44.34 | B | C |
| ATOM | 2911 | CD | LYS | B | 74 | 22.212 | 77.428 | 84.095 | 1.00 | 44.34 | B | C |
| ATOM | 2912 | CE | LYS | B | 74 | 22.886 | 77.816 | 85.413 | 1.00 | 44.34 | B | C |
| ATOM | 2913 | NZ | LYS | B | 74 | 22.134 | 78.892 | 86.140 | 1.00 | 44.34 | B | N |
| ATOM | 2914 | C | LYS | B | 74 | 18.028 | 75.577 | 83.599 | 1.00 | 31.17 | B | C |
| ATOM | 2915 | O | LYS | B | 74 | 17.384 | 76.427 | 82.988 | 1.00 | 31.17 | B | O |
| ATOM | 2916 | N | LEU | B | 75 | 17.634 | 75.071 | 84.764 | 1.00 | 35.87 | B | N |
| ATOM | 2917 | CA | LEU | B | 75 | 16.434 | 75.560 | 85.434 | 1.00 | 35.87 | B | C |
| ATOM | 2918 | CB | LEU | B | 75 | 16.103 | 74.685 | 86.651 | 1.00 | 13.58 | B | C |
| ATOM | 2919 | CG | LEU | B | 75 | 15.780 | 73.221 | 86.331 | 1.00 | 13.58 | B | C |
| ATOM | 2920 | CD1 | LEU | B | 75 | 15.367 | 72.495 | 87.585 | 1.00 | 13.58 | B | C |
| ATOM | 2921 | CD2 | LEU | B | 75 | 14.696 | 73.149 | 85.290 | 1.00 | 13.58 | B | C |
| ATOM | 2922 | C | LEU | B | 75 | 16.829 | 76.966 | 85.862 | 1.00 | 35.87 | B | C |
| ATOM | 2923 | O | LEU | B | 75 | 17.901 | 77.159 | 86.430 | 1.00 | 35.87 | B | O |
| ATOM | 2924 | N | CYS | B | 76 | 15.984 | 77.949 | 85.581 | 1.00 | 38.44 | B | N |
| ATOM | 2925 | CA | CYS | B | 76 | 16.313 | 79.326 | 85.909 | 1.00 | 38.44 | B | C |
| ATOM | 2926 | CB | CYS | B | 76 | 15.244 | 80.252 | 85.344 | 1.00 | 42.35 | B | C |
| ATOM | 2927 | SG | CYS | B | 76 | 15.114 | 80.062 | 83.552 | 1.00 | 42.35 | B | S |
| ATOM | 2928 | C | CYS | B | 76 | 16.520 | 79.591 | 87.388 | 1.00 | 38.44 | B | C |
| ATOM | 2929 | O | CYS | B | 76 | 17.660 | 79.649 | 87.855 | 1.00 | 38.44 | B | O |
| ATOM | 2930 | N | ASP | B | 77 | 15.426 | 79.757 | 88.128 | 1.00 | 69.63 | B | N |
| ATOM | 2931 | CA | ASP | B | 77 | 15.539 | 80.036 | 89.560 | 1.00 | 69.63 | B | C |
| ATOM | 2932 | CB | ASP | B | 77 | 14.172 | 79.896 | 90.263 | 1.00 | 73.68 | B | C |
| ATOM | 2933 | CG | ASP | B | 77 | 13.483 | 78.560 | 89.985 | 1.00 | 73.68 | B | C |
| ATOM | 2934 | OD1 | ASP | B | 77 | 13.510 | 78.090 | 88.820 | 1.00 | 73.68 | B | O |
| ATOM | 2935 | OD2 | ASP | B | 77 | 12.898 | 77.990 | 90.938 | 1.00 | 73.68 | B | O |
| ATOM | 2936 | C | ASP | B | 77 | 16.574 | 79.102 | 90.173 | 1.00 | 69.63 | B | C |
| ATOM | 2937 | O | ASP | B | 77 | 17.634 | 79.544 | 90.623 | 1.00 | 69.63 | B | O |
| ATOM | 2938 | N | SER | B | 78 | 16.288 | 77.809 | 90.131 | 1.00 | 44.16 | B | N |
| ATOM | 2939 | CA | SER | B | 78 | 17.181 | 76.786 | 90.672 | 1.00 | 44.16 | B | C |
| ATOM | 2940 | CB | SER | B | 78 | 16.629 | 75.389 | 90.336 | 1.00 | 61.16 | B | C |
| ATOM | 2941 | OG | SER | B | 78 | 17.465 | 74.358 | 90.840 | 1.00 | 61.16 | B | O |
| ATOM | 2942 | C | SER | B | 78 | 18.650 | 76.871 | 90.218 | 1.00 | 44.16 | B | C |

| | | | | | | | | | | | | |
|---|---|---|---|---|---|---|---|---|---|---|---|---|
| ATOM | 2943 | O | SER | B | 78 | 19.560 | 76.813 | 91.038 | 1.00 | 44.16 | B | O |
| ATOM | 2944 | N | GLY | B | 79 | 18.886 | 77.006 | 88.919 | 1.00 | 34.90 | B | N |
| ATOM | 2945 | CA | GLY | B | 79 | 20.254 | 77.044 | 88.429 | 1.00 | 34.90 | B | C |
| ATOM | 2946 | C | GLY | B | 79 | 20.741 | 75.644 | 88.053 | 1.00 | 34.90 | B | C |
| ATOM | 2947 | O | GLY | B | 79 | 21.646 | 75.482 | 87.225 | 1.00 | 34.90 | B | O |
| ATOM | 2948 | N | GLU | B | 80 | 20.126 | 74.634 | 88.669 | 1.00 | 40.40 | B | N |
| ATOM | 2949 | CA | GLU | B | 80 | 20.445 | 73.221 | 88.445 | 1.00 | 40.40 | B | C |
| ATOM | 2950 | CB | GLU | B | 80 | 19.532 | 72.338 | 89.293 | 1.00 | 51.75 | B | C |
| ATOM | 2951 | CG | GLU | B | 80 | 19.861 | 72.313 | 90.767 | 1.00 | 51.75 | B | C |
| ATOM | 2952 | CD | GLU | B | 80 | 18.792 | 71.596 | 91.580 | 1.00 | 51.75 | B | C |
| ATOM | 2953 | OE1 | GLU | B | 80 | 18.290 | 70.552 | 91.109 | 1.00 | 51.75 | B | O |
| ATOM | 2954 | OE2 | GLU | B | 80 | 18.459 | 72.067 | 92.691 | 1.00 | 51.75 | B | O |
| ATOM | 2955 | C | GLU | B | 80 | 20.305 | 72.769 | 86.999 | 1.00 | 40.40 | B | C |
| ATOM | 2956 | O | GLU | B | 80 | 19.486 | 73.300 | 86.248 | 1.00 | 40.40 | B | O |
| ATOM | 2957 | N | LEU | B | 81 | 21.095 | 71.766 | 86.623 | 1.00 | 34.93 | B | N |
| ATOM | 2958 | CA | LEU | B | 81 | 21.038 | 71.221 | 85.270 | 1.00 | 34.93 | B | C |
| ATOM | 2959 | CB | LEU | B | 81 | 22.421 | 70.749 | 84.810 | 1.00 | 47.60 | B | C |
| ATOM | 2960 | CG | LEU | B | 81 | 23.524 | 71.773 | 84.542 | 1.00 | 47.60 | B | C |
| ATOM | 2961 | CD1 | LEU | B | 81 | 24.686 | 71.067 | 83.842 | 1.00 | 47.60 | B | C |
| ATOM | 2962 | CD2 | LEU | B | 81 | 22.991 | 72.908 | 83.658 | 1.00 | 47.60 | B | C |
| ATOM | 2963 | C | LEU | B | 81 | 20.068 | 70.045 | 85.166 | 1.00 | 34.93 | B | C |
| ATOM | 2964 | O | LEU | B | 81 | 20.039 | 69.161 | 86.026 | 1.00 | 34.93 | B | O |
| ATOM | 2965 | N | VAL | B | 82 | 19.282 | 70.037 | 84.097 | 1.00 | 34.39 | B | N |
| ATOM | 2966 | CA | VAL | B | 82 | 18.333 | 68.962 | 83.864 | 1.00 | 34.39 | B | C |
| ATOM | 2967 | CB | VAL | B | 82 | 16.886 | 69.448 | 83.995 | 1.00 | 28.95 | B | C |
| ATOM | 2968 | CG1 | VAL | B | 82 | 16.629 | 69.934 | 85.410 | 1.00 | 28.95 | B | C |
| ATOM | 2969 | CG2 | VAL | B | 82 | 16.620 | 70.553 | 82.973 | 1.00 | 28.95 | B | C |
| ATOM | 2970 | C | VAL | B | 82 | 18.508 | 68.416 | 82.457 | 1.00 | 34.39 | B | C |
| ATOM | 2971 | O | VAL | B | 82 | 19.176 | 69.022 | 81.619 | 1.00 | 34.39 | B | O |
| ATOM | 2972 | N | ALA | B | 83 | 17.898 | 67.269 | 82.207 | 1.00 | 43.56 | B | N |
| ATOM | 2973 | CA | ALA | B | 83 | 17.952 | 66.633 | 80.903 | 1.00 | 43.56 | B | C |
| ATOM | 2974 | CB | ALA | B | 83 | 18.673 | 65.331 | 81.010 | 1.00 | 34.36 | B | C |
| ATOM | 2975 | C | ALA | B | 83 | 16.511 | 66.402 | 80.455 | 1.00 | 43.56 | B | C |
| ATOM | 2976 | O | ALA | B | 83 | 15.695 | 65.841 | 81.201 | 1.00 | 43.56 | B | O |
| ATOM | 2977 | N | ILE | B | 84 | 16.187 | 66.833 | 79.243 | 1.00 | 26.32 | B | N |
| ATOM | 2978 | CA | ILE | B | 84 | 14.834 | 66.659 | 78.757 | 1.00 | 26.32 | B | C |
| ATOM | 2979 | CB | ILE | B | 84 | 14.234 | 67.989 | 78.265 | 1.00 | 30.96 | B | C |
| ATOM | 2980 | CG2 | ILE | B | 84 | 12.720 | 67.830 | 78.087 | 1.00 | 30.96 | B | C |
| ATOM | 2981 | CG1 | ILE | B | 84 | 14.505 | 69.099 | 79.286 | 1.00 | 30.96 | B | C |
| ATOM | 2982 | CD1 | ILE | B | 84 | 14.048 | 70.465 | 78.839 | 1.00 | 30.96 | B | C |
| ATOM | 2983 | C | ILE | B | 84 | 14.779 | 65.657 | 77.620 | 1.00 | 26.32 | B | C |
| ATOM | 2984 | O | ILE | B | 84 | 15.302 | 65.915 | 76.537 | 1.00 | 26.32 | B | O |
| ATOM | 2985 | N | LYS | B | 85 | 14.156 | 64.510 | 77.879 | 1.00 | 32.13 | B | N |
| ATOM | 2986 | CA | LYS | B | 85 | 14.007 | 63.476 | 76.872 | 1.00 | 32.13 | B | C |
| ATOM | 2987 | CB | LYS | B | 85 | 13.956 | 62.098 | 77.524 | 1.00 | 42.19 | B | C |
| ATOM | 2988 | CG | LYS | B | 85 | 14.171 | 60.979 | 76.548 | 1.00 | 42.19 | B | C |
| ATOM | 2989 | CD | LYS | B | 85 | 13.456 | 59.700 | 76.941 | 1.00 | 42.19 | B | C |
| ATOM | 2990 | CE | LYS | B | 85 | 13.633 | 58.617 | 75.841 | 1.00 | 42.19 | B | C |
| ATOM | 2991 | NZ | LYS | B | 85 | 13.072 | 57.265 | 76.222 | 1.00 | 42.19 | B | N |
| ATOM | 2992 | C | LYS | B | 85 | 12.680 | 63.754 | 76.174 | 1.00 | 32.13 | B | C |
| ATOM | 2993 | O | LYS | B | 85 | 11.617 | 63.623 | 76.778 | 1.00 | 32.13 | B | O |
| ATOM | 2994 | N | LYS | B | 86 | 12.739 | 64.158 | 74.911 | 1.00 | 33.24 | B | N |
| ATOM | 2995 | CA | LYS | B | 86 | 11.533 | 64.454 | 74.145 | 1.00 | 33.24 | B | C |
| ATOM | 2996 | CB | LYS | B | 86 | 11.759 | 65.717 | 73.319 | 1.00 | 47.29 | B | C |
| ATOM | 2997 | CG | LYS | B | 86 | 10.523 | 66.274 | 72.658 | 1.00 | 47.29 | B | C |
| ATOM | 2998 | CD | LYS | B | 86 | 10.848 | 67.533 | 71.851 | 1.00 | 47.29 | B | C |
| ATOM | 2999 | CE | LYS | B | 86 | 9.566 | 68.290 | 71.476 | 1.00 | 47.29 | B | C |
| ATOM | 3000 | NZ | LYS | B | 86 | 9.840 | 69.426 | 70.542 | 1.00 | 47.29 | B | N |
| ATOM | 3001 | C | LYS | B | 86 | 11.206 | 63.281 | 73.222 | 1.00 | 33.24 | B | C |
| ATOM | 3002 | O | LYS | B | 86 | 11.945 | 63.007 | 72.274 | 1.00 | 33.24 | B | O |
| ATOM | 3003 | N | VAL | B | 87 | 10.106 | 62.588 | 73.503 | 1.00 | 32.07 | B | N |
| ATOM | 3004 | CA | VAL | B | 87 | 9.698 | 61.450 | 72.689 | 1.00 | 32.07 | B | C |
| ATOM | 3005 | CB | VAL | B | 87 | 9.626 | 60.147 | 73.508 | 1.00 | 23.22 | B | C |
| ATOM | 3006 | CG1 | VAL | B | 87 | 10.612 | 60.198 | 74.648 | 1.00 | 23.22 | B | C |
| ATOM | 3007 | CG2 | VAL | B | 87 | 8.207 | 59.902 | 73.987 | 1.00 | 23.22 | B | C |
| ATOM | 3008 | C | VAL | B | 87 | 8.322 | 61.680 | 72.092 | 1.00 | 32.07 | B | C |
| ATOM | 3009 | O | VAL | B | 87 | 7.496 | 62.390 | 72.668 | 1.00 | 32.07 | B | O |

| | | | | | | | | | | | | |
|---|---|---|---|---|---|---|---|---|---|---|---|---|
| ATOM | 3010 | N | LEU | B | 88 | 8.080 | 61.078 | 70.931 | 1.00 | 59.76 | B | N |
| ATOM | 3011 | CA | LEU | B | 88 | 6.782 | 61.201 | 70.281 | 1.00 | 59.76 | B | C |
| ATOM | 3012 | CB | LEU | B | 88 | 6.834 | 60.622 | 68.867 | 1.00 | 58.01 | B | C |
| ATOM | 3013 | CG | LEU | B | 88 | 5.523 | 60.627 | 68.078 | 1.00 | 58.01 | B | C |
| ATOM | 3014 | CD1 | LEU | B | 88 | 5.242 | 62.037 | 67.521 | 1.00 | 58.01 | B | C |
| ATOM | 3015 | CD2 | LEU | B | 88 | 5.638 | 59.612 | 66.950 | 1.00 | 58.01 | B | C |
| ATOM | 3016 | C | LEU | B | 88 | 5.804 | 60.387 | 71.127 | 1.00 | 59.76 | B | C |
| ATOM | 3017 | O | LEU | B | 88 | 6.057 | 59.211 | 71.424 | 1.00 | 59.76 | B | O |
| ATOM | 3018 | N | GLN | B | 89 | 4.686 | 60.993 | 71.514 | 1.00 | 46.82 | B | N |
| ATOM | 3019 | CA | GLN | B | 89 | 3.734 | 60.270 | 72.348 | 1.00 | 46.82 | B | C |
| ATOM | 3020 | CB | GLN | B | 89 | 3.766 | 60.833 | 73.771 | 1.00 | 72.47 | B | C |
| ATOM | 3021 | CG | GLN | B | 89 | 2.821 | 60.148 | 74.755 | 1.00 | 72.47 | B | C |
| ATOM | 3022 | CD | GLN | B | 89 | 2.874 | 58.630 | 74.646 | 1.00 | 72.47 | B | C |
| ATOM | 3023 | OE1 | GLN | B | 89 | 3.915 | 58.039 | 74.289 | 1.00 | 72.47 | B | O |
| ATOM | 3024 | NE2 | GLN | B | 89 | 1.749 | 57.986 | 74.959 | 1.00 | 72.47 | B | N |
| ATOM | 3025 | C | GLN | B | 89 | 2.312 | 60.292 | 71.829 | 1.00 | 46.82 | B | C |
| ATOM | 3026 | O | GLN | B | 89 | 1.649 | 61.331 | 71.879 | 1.00 | 46.82 | B | O |
| ATOM | 3027 | N | ASP | B | 90 | 1.845 | 59.140 | 71.346 | 1.00 | 49.25 | B | N |
| ATOM | 3028 | CA | ASP | B | 90 | 0.488 | 59.045 | 70.832 | 1.00 | 49.25 | B | C |
| ATOM | 3029 | CB | ASP | B | 90 | 0.237 | 57.705 | 70.139 | 1.00 | 60.57 | B | C |
| ATOM | 3030 | CG | ASP | B | 90 | -1.153 | 57.644 | 69.481 | 1.00 | 60.57 | B | C |
| ATOM | 3031 | OD1 | ASP | B | 90 | -1.696 | 56.526 | 69.321 | 1.00 | 60.57 | B | O |
| ATOM | 3032 | OD2 | ASP | B | 90 | -1.700 | 58.717 | 69.117 | 1.00 | 60.57 | B | O |
| ATOM | 3033 | C | ASP | B | 90 | -0.508 | 59.187 | 71.977 | 1.00 | 49.25 | B | C |
| ATOM | 3034 | O | ASP | B | 90 | -0.396 | 58.502 | 73.008 | 1.00 | 49.25 | B | O |
| ATOM | 3035 | N | ALA | B | 91 | -1.484 | 60.077 | 71.791 | 1.00 | 69.71 | B | N |
| ATOM | 3036 | CA | ALA | B | 91 | -2.513 | 60.314 | 72.804 | 1.00 | 69.71 | B | C |
| ATOM | 3037 | CB | ALA | B | 91 | -3.477 | 61.404 | 72.334 | 1.00 | 42.84 | B | C |
| ATOM | 3038 | C | ALA | B | 91 | -3.289 | 59.030 | 73.127 | 1.00 | 69.71 | B | C |
| ATOM | 3039 | O | ALA | B | 91 | -3.808 | 58.869 | 74.238 | 1.00 | 69.71 | B | O |
| ATOM | 3040 | N | ALA | B | 92 | -3.372 | 58.122 | 72.153 | 1.00 | 56.78 | B | N |
| ATOM | 3041 | CA | ALA | B | 92 | -4.069 | 56.854 | 72.353 | 1.00 | 56.78 | B | C |
| ATOM | 3042 | CB | ALA | B | 92 | -3.766 | 55.903 | 71.194 | 1.00 | 49.36 | B | C |
| ATOM | 3043 | C | ALA | B | 92 | -3.657 | 56.204 | 73.682 | 1.00 | 56.78 | B | C |
| ATOM | 3044 | O | ALA | B | 92 | -4.387 | 56.272 | 74.688 | 1.00 | 56.78 | B | O |
| ATOM | 3045 | N | ALA | B | 93 | -2.476 | 55.582 | 73.671 | 1.00 | 61.34 | B | N |
| ATOM | 3046 | CA | ALA | B | 93 | -1.933 | 54.899 | 74.841 | 1.00 | 61.34 | B | C |
| ATOM | 3047 | CB | ALA | B | 93 | -1.031 | 53.770 | 74.388 | 1.00 | 44.58 | B | C |
| ATOM | 3048 | C | ALA | B | 93 | -1.163 | 55.845 | 75.772 | 1.00 | 61.34 | B | C |
| ATOM | 3049 | O | ALA | B | 93 | -0.942 | 57.017 | 75.440 | 1.00 | 61.34 | B | O |
| ATOM | 3050 | N | LYS | B | 94 | -0.785 | 55.333 | 76.944 | 1.00 | 54.57 | B | N |
| ATOM | 3051 | CA | LYS | B | 94 | -0.016 | 56.100 | 77.932 | 1.00 | 54.57 | B | C |
| ATOM | 3052 | CB | LYS | B | 94 | -0.368 | 55.663 | 79.358 | 1.00 | 60.08 | B | C |
| ATOM | 3053 | CG | LYS | B | 94 | -1.841 | 55.784 | 79.699 | 1.00 | 60.08 | B | C |
| ATOM | 3054 | CD | LYS | B | 94 | -2.171 | 55.081 | 81.010 | 1.00 | 60.08 | B | C |
| ATOM | 3055 | CE | LYS | B | 94 | -3.646 | 55.289 | 81.389 | 1.00 | 60.08 | B | C |
| ATOM | 3056 | NZ | LYS | B | 94 | -4.569 | 54.953 | 80.238 | 1.00 | 60.08 | B | N |
| ATOM | 3057 | C | LYS | B | 94 | 1.442 | 55.773 | 77.649 | 1.00 | 54.57 | B | C |
| ATOM | 3058 | O | LYS | B | 94 | 1.731 | 54.865 | 76.869 | 1.00 | 54.57 | B | O |
| ATOM | 3059 | N | ASN | B | 95 | 2.361 | 56.495 | 78.274 | 1.00 | 27.23 | B | N |
| ATOM | 3060 | CA | ASN | B | 95 | 3.773 | 56.244 | 78.043 | 1.00 | 27.23 | B | C |
| ATOM | 3061 | CB | ASN | B | 95 | 4.523 | 57.571 | 77.989 | 1.00 | 26.90 | B | C |
| ATOM | 3062 | CG | ASN | B | 95 | 5.997 | 57.392 | 77.731 | 1.00 | 26.90 | B | C |
| ATOM | 3063 | OD1 | ASN | B | 95 | 6.739 | 56.906 | 78.585 | 1.00 | 26.90 | B | O |
| ATOM | 3064 | ND2 | ASN | B | 95 | 6.435 | 57.774 | 76.540 | 1.00 | 26.90 | B | N |
| ATOM | 3065 | C | ASN | B | 95 | 4.305 | 55.383 | 79.172 | 1.00 | 27.23 | B | C |
| ATOM | 3066 | O | ASN | B | 95 | 4.306 | 55.806 | 80.326 | 1.00 | 27.23 | B | O |
| ATOM | 3067 | N | ARG | B | 96 | 4.758 | 54.176 | 78.838 | 1.00 | 22.12 | B | N |
| ATOM | 3068 | CA | ARG | B | 96 | 5.266 | 53.251 | 79.849 | 1.00 | 22.12 | B | C |
| ATOM | 3069 | CB | ARG | B | 96 | 5.674 | 51.917 | 79.228 | 1.00 | 51.11 | B | C |
| ATOM | 3070 | CG | ARG | B | 96 | 5.018 | 50.714 | 79.892 | 1.00 | 51.11 | B | C |
| ATOM | 3071 | CD | ARG | B | 96 | 6.055 | 49.705 | 80.364 | 1.00 | 51.11 | B | C |
| ATOM | 3072 | NE | ARG | B | 96 | 6.982 | 49.331 | 79.291 | 1.00 | 51.11 | B | N |
| ATOM | 3073 | CZ | ARG | B | 96 | 6.701 | 48.509 | 78.275 | 1.00 | 51.11 | B | C |
| ATOM | 3074 | NH1 | ARG | B | 96 | 5.495 | 47.939 | 78.173 | 1.00 | 51.11 | B | N |
| ATOM | 3075 | NH2 | ARG | B | 96 | 7.640 | 48.265 | 77.352 | 1.00 | 51.11 | B | N |
| ATOM | 3076 | C | ARG | B | 96 | 6.425 | 53.809 | 80.649 | 1.00 | 22.12 | B | C |

| | | | | | | | | | | | | |
|---|---|---|---|---|---|---|---|---|---|---|---|---|
| ATOM | 3077 | O | ARG | B | 96 | 6.435 | 53.705 | 81.867 | 1.00 | 22.12 | B | O |
| ATOM | 3078 | N | GLU | B | 97 | 7.396 | 54.405 | 79.964 | 1.00 | 33.24 | B | N |
| ATOM | 3079 | CA | GLU | B | 97 | 8.563 | 54.974 | 80.632 | 1.00 | 33.24 | B | C |
| ATOM | 3080 | CB | GLU | B | 97 | 9.478 | 55.657 | 79.608 | 1.00 | 25.31 | B | C |
| ATOM | 3081 | CG | GLU | B | 97 | 10.862 | 56.037 | 80.121 | 1.00 | 25.31 | B | C |
| ATOM | 3082 | CD | GLU | B | 97 | 11.736 | 56.657 | 79.043 | 1.00 | 25.31 | B | C |
| ATOM | 3083 | OE1 | GLU | B | 97 | 12.944 | 56.851 | 79.286 | 1.00 | 25.31 | B | O |
| ATOM | 3084 | OE2 | GLU | B | 97 | 11.217 | 56.955 | 77.950 | 1.00 | 25.31 | B | O |
| ATOM | 3085 | C | GLU | B | 97 | 8.067 | 55.985 | 81.657 | 1.00 | 33.24 | B | C |
| ATOM | 3086 | O | GLU | B | 97 | 8.434 | 55.916 | 82.820 | 1.00 | 33.24 | B | O |
| ATOM | 3087 | N | LEU | B | 98 | 7.218 | 56.912 | 81.231 | 1.00 | 18.12 | B | N |
| ATOM | 3088 | CA | LEU | B | 98 | 6.692 | 57.912 | 82.147 | 1.00 | 18.12 | B | C |
| ATOM | 3089 | CB | LEU | B | 98 | 5.648 | 58.789 | 81.456 | 1.00 | 12.96 | B | C |
| ATOM | 3090 | CG | LEU | B | 98 | 4.906 | 59.779 | 82.373 | 1.00 | 12.96 | B | C |
| ATOM | 3091 | CD1 | LEU | B | 98 | 5.871 | 60.827 | 82.926 | 1.00 | 12.96 | B | C |
| ATOM | 3092 | CD2 | LEU | B | 98 | 3.802 | 60.467 | 81.606 | 1.00 | 12.96 | B | C |
| ATOM | 3093 | C | LEU | B | 98 | 6.034 | 57.249 | 83.333 | 1.00 | 18.12 | B | C |
| ATOM | 3094 | O | LEU | B | 98 | 6.332 | 57.556 | 84.473 | 1.00 | 18.12 | B | O |
| ATOM | 3095 | N | GLN | B | 99 | 5.121 | 56.340 | 83.041 | 1.00 | 36.34 | B | N |
| ATOM | 3096 | CA | GLN | B | 99 | 4.396 | 55.637 | 84.072 | 1.00 | 36.34 | B | C |
| ATOM | 3097 | CB | GLN | B | 99 | 3.519 | 54.551 | 83.455 | 1.00 | 99.29 | B | C |
| ATOM | 3098 | CG | GLN | B | 99 | 2.696 | 53.800 | 84.500 | 1.00 | 99.29 | B | C |
| ATOM | 3099 | CD | GLN | B | 99 | 2.055 | 52.504 | 83.979 | 1.00 | 99.29 | B | C |
| ATOM | 3100 | OE1 | GLN | B | 99 | 1.514 | 51.714 | 84.776 | 1.00 | 99.29 | B | O |
| ATOM | 3101 | NE2 | GLN | B | 99 | 2.115 | 52.276 | 82.644 | 1.00 | 99.29 | B | N |
| ATOM | 3102 | C | GLN | B | 99 | 5.279 | 55.012 | 85.132 | 1.00 | 36.34 | B | C |
| ATOM | 3103 | O | GLN | B | 99 | 4.975 | 55.133 | 86.314 | 1.00 | 36.34 | B | O |
| ATOM | 3104 | N | ILE | B | 100 | 6.358 | 54.338 | 84.727 | 1.00 | 34.19 | B | N |
| ATOM | 3105 | CA | ILE | B | 100 | 7.223 | 53.709 | 85.717 | 1.00 | 34.19 | B | C |
| ATOM | 3106 | CB | ILE | B | 100 | 8.060 | 52.519 | 85.138 | 1.00 | 25.81 | B | C |
| ATOM | 3107 | CG2 | ILE | B | 100 | 7.358 | 51.875 | 83.998 | 1.00 | 25.81 | B | C |
| ATOM | 3108 | CG1 | ILE | B | 100 | 9.437 | 52.987 | 84.722 | 1.00 | 25.81 | B | C |
| ATOM | 3109 | CD1 | ILE | B | 100 | 10.498 | 52.563 | 85.709 | 1.00 | 25.81 | B | C |
| ATOM | 3110 | C | ILE | B | 100 | 8.130 | 54.735 | 86.418 | 1.00 | 34.19 | B | C |
| ATOM | 3111 | O | ILE | B | 100 | 8.426 | 54.595 | 87.604 | 1.00 | 34.19 | B | O |
| ATOM | 3112 | N | MET | B | 101 | 8.540 | 55.777 | 85.699 | 1.00 | 23.14 | B | N |
| ATOM | 3113 | CA | MET | B | 101 | 9.381 | 56.826 | 86.273 | 1.00 | 23.14 | B | C |
| ATOM | 3114 | CB | MET | B | 101 | 9.686 | 57.897 | 85.226 | 1.00 | 41.31 | B | C |
| ATOM | 3115 | CG | MET | B | 101 | 10.809 | 57.546 | 84.279 | 1.00 | 41.31 | B | C |
| ATOM | 3116 | SD | MET | B | 101 | 12.380 | 57.518 | 85.109 | 1.00 | 41.31 | B | S |
| ATOM | 3117 | CE | MET | B | 101 | 12.395 | 55.853 | 85.726 | 1.00 | 41.31 | B | C |
| ATOM | 3118 | C | MET | B | 101 | 8.633 | 57.475 | 87.422 | 1.00 | 23.14 | B | C |
| ATOM | 3119 | O | MET | B | 101 | 9.155 | 57.653 | 88.508 | 1.00 | 23.14 | B | O |
| ATOM | 3120 | N | ARG | B | 102 | 7.389 | 57.823 | 87.164 | 1.00 | 28.05 | B | N |
| ATOM | 3121 | CA | ARG | B | 102 | 6.566 | 58.469 | 88.157 | 1.00 | 28.05 | B | C |
| ATOM | 3122 | CB | ARG | B | 102 | 5.178 | 58.712 | 87.589 | 1.00 | 37.12 | B | C |
| ATOM | 3123 | CG | ARG | B | 102 | 5.193 | 59.715 | 86.475 | 1.00 | 37.12 | B | C |
| ATOM | 3124 | CD | ARG | B | 102 | 4.727 | 61.033 | 86.980 | 1.00 | 37.12 | B | C |
| ATOM | 3125 | NE | ARG | B | 102 | 3.279 | 61.134 | 86.931 | 1.00 | 37.12 | B | N |
| ATOM | 3126 | CZ | ARG | B | 102 | 2.551 | 61.791 | 87.820 | 1.00 | 37.12 | B | C |
| ATOM | 3127 | NH1 | ARG | B | 102 | 3.136 | 62.411 | 88.850 | 1.00 | 37.12 | B | N |
| ATOM | 3128 | NH2 | ARG | B | 102 | 1.233 | 61.830 | 87.666 | 1.00 | 37.12 | B | N |
| ATOM | 3129 | C | ARG | B | 102 | 6.475 | 57.756 | 89.493 | 1.00 | 28.05 | B | C |
| ATOM | 3130 | O | ARG | B | 102 | 6.288 | 58.411 | 90.514 | 1.00 | 28.05 | B | O |
| ATOM | 3131 | N | LYS | B | 103 | 6.622 | 56.432 | 89.502 | 1.00 | 24.99 | B | N |
| ATOM | 3132 | CA | LYS | B | 103 | 6.526 | 55.684 | 90.757 | 1.00 | 24.99 | B | C |
| ATOM | 3133 | CB | LYS | B | 103 | 5.589 | 54.481 | 90.593 | 1.00 | 42.26 | B | C |
| ATOM | 3134 | CG | LYS | B | 103 | 6.257 | 53.201 | 90.133 | 1.00 | 42.26 | B | C |
| ATOM | 3135 | CD | LYS | B | 103 | 5.234 | 52.079 | 89.981 | 1.00 | 42.26 | B | C |
| ATOM | 3136 | CE | LYS | B | 103 | 4.320 | 52.311 | 88.763 | 1.00 | 42.26 | B | C |
| ATOM | 3137 | NZ | LYS | B | 103 | 3.169 | 51.357 | 88.679 | 1.00 | 42.26 | B | N |
| ATOM | 3138 | C | LYS | B | 103 | 7.864 | 55.226 | 91.339 | 1.00 | 24.99 | B | C |
| ATOM | 3139 | O | LYS | B | 103 | 7.906 | 54.423 | 92.267 | 1.00 | 24.99 | B | O |
| ATOM | 3140 | N | LEU | B | 104 | 8.958 | 55.756 | 90.809 | 1.00 | 25.17 | B | N |
| ATOM | 3141 | CA | LEU | B | 104 | 10.286 | 55.378 | 91.274 | 1.00 | 25.17 | B | C |
| ATOM | 3142 | CB | LEU | B | 104 | 11.166 | 54.990 | 90.093 | 1.00 | 22.33 | B | C |
| ATOM | 3143 | CG | LEU | B | 104 | 11.354 | 53.506 | 89.806 | 1.00 | 22.33 | B | C |

| | | | | | | | | | | | | |
|---|---|---|---|---|---|---|---|---|---|---|---|---|
| ATOM | 3144 | CD1 | LEU | B | 104 | 10.024 | 52.792 | 89.791 | 1.00 | 22.33 | B | C |
| ATOM | 3145 | CD2 | LEU | B | 104 | 12.061 | 53.363 | 88.472 | 1.00 | 22.33 | B | C |
| ATOM | 3146 | C | LEU | B | 104 | 10.958 | 56.497 | 92.033 | 1.00 | 25.17 | B | C |
| ATOM | 3147 | O | LEU | B | 104 | 10.962 | 57.641 | 91.589 | 1.00 | 25.17 | B | O |
| ATOM | 3148 | N | ASP | B | 105 | 11.528 | 56.161 | 93.181 | 1.00 | 26.27 | B | N |
| ATOM | 3149 | CA | ASP | B | 105 | 12.229 | 57.137 | 94.002 | 1.00 | 26.27 | B | C |
| ATOM | 3150 | CB | ASP | B | 105 | 11.273 | 57.865 | 94.949 | 1.00 | 30.41 | B | C |
| ATOM | 3151 | CG | ASP | B | 105 | 12.004 | 58.788 | 95.902 | 1.00 | 30.41 | B | C |
| ATOM | 3152 | OD1 | ASP | B | 105 | 12.916 | 59.520 | 95.432 | 1.00 | 30.41 | B | O |
| ATOM | 3153 | OD2 | ASP | B | 105 | 11.655 | 58.771 | 97.106 | 1.00 | 30.41 | B | O |
| ATOM | 3154 | C | ASP | B | 105 | 13.298 | 56.419 | 94.814 | 1.00 | 26.27 | B | C |
| ATOM | 3155 | O | ASP | B | 105 | 13.020 | 55.850 | 95.870 | 1.00 | 26.27 | B | O |
| ATOM | 3156 | N | HIS | B | 106 | 14.528 | 56.457 | 94.306 | 1.00 | 31.74 | B | N |
| ATOM | 3157 | CA | HIS | B | 106 | 15.659 | 55.804 | 94.950 | 1.00 | 31.74 | B | C |
| ATOM | 3158 | CB | HIS | B | 106 | 15.801 | 54.381 | 94.395 | 1.00 | 19.57 | B | C |
| ATOM | 3159 | CG | HIS | B | 106 | 16.793 | 53.530 | 95.130 | 1.00 | 19.57 | B | C |
| ATOM | 3160 | CD2 | HIS | B | 106 | 16.615 | 52.524 | 96.017 | 1.00 | 19.57 | B | C |
| ATOM | 3161 | ND1 | HIS | B | 106 | 18.154 | 53.667 | 94.976 | 1.00 | 19.57 | B | N |
| ATOM | 3162 | CE1 | HIS | B | 106 | 18.770 | 52.781 | 95.735 | 1.00 | 19.57 | B | C |
| ATOM | 3163 | NE2 | HIS | B | 106 | 17.860 | 52.076 | 96.376 | 1.00 | 19.57 | B | N |
| ATOM | 3164 | C | HIS | B | 106 | 16.927 | 56.602 | 94.685 | 1.00 | 31.74 | B | C |
| ATOM | 3165 | O | HIS | B | 106 | 17.151 | 57.040 | 93.572 | 1.00 | 31.74 | B | O |
| ATOM | 3166 | N | CYS | B | 107 | 17.747 | 56.791 | 95.708 | 1.00 | 18.06 | B | N |
| ATOM | 3167 | CA | CYS | B | 107 | 18.979 | 57.559 | 95.593 | 1.00 | 18.06 | B | C |
| ATOM | 3168 | CB | CYS | B | 107 | 19.737 | 57.515 | 96.919 | 1.00 | 25.39 | B | C |
| ATOM | 3169 | SG | CYS | B | 107 | 20.626 | 55.995 | 97.224 | 1.00 | 25.39 | B | S |
| ATOM | 3170 | C | CYS | B | 107 | 19.901 | 57.091 | 94.474 | 1.00 | 18.06 | B | C |
| ATOM | 3171 | O | CYS | B | 107 | 20.869 | 57.761 | 94.147 | 1.00 | 18.06 | B | O |
| ATOM | 3172 | N | ASN | B | 108 | 19.604 | 55.939 | 93.887 | 1.00 | 21.61 | B | N |
| ATOM | 3173 | CA | ASN | B | 108 | 20.433 | 55.413 | 92.820 | 1.00 | 21.61 | B | C |
| ATOM | 3174 | CB | ASN | B | 108 | 20.928 | 54.019 | 93.213 | 1.00 | 28.65 | B | C |
| ATOM | 3175 | CG | ASN | B | 108 | 22.054 | 54.066 | 94.241 | 1.00 | 28.65 | B | C |
| ATOM | 3176 | OD1 | ASN | B | 108 | 22.106 | 53.253 | 95.159 | 1.00 | 28.65 | B | O |
| ATOM | 3177 | ND2 | ASN | B | 108 | 22.967 | 55.008 | 94.074 | 1.00 | 28.65 | B | N |
| ATOM | 3178 | C | ASN | B | 108 | 19.764 | 55.374 | 91.458 | 1.00 | 21.61 | B | C |
| ATOM | 3179 | O | ASN | B | 108 | 20.224 | 54.681 | 90.561 | 1.00 | 21.61 | B | O |
| ATOM | 3180 | N | ILE | B | 109 | 18.671 | 56.107 | 91.301 | 1.00 | 15.37 | B | N |
| ATOM | 3181 | CA | ILE | B | 109 | 17.989 | 56.160 | 90.019 | 1.00 | 15.37 | B | C |
| ATOM | 3182 | CB | ILE | B | 109 | 16.642 | 55.410 | 90.054 | 1.00 | 23.79 | B | C |
| ATOM | 3183 | CG2 | ILE | B | 109 | 15.930 | 55.548 | 88.707 | 1.00 | 23.79 | B | C |
| ATOM | 3184 | CG1 | ILE | B | 109 | 16.897 | 53.925 | 90.327 | 1.00 | 23.79 | B | C |
| ATOM | 3185 | CD1 | ILE | B | 109 | 15.664 | 53.130 | 90.565 | 1.00 | 23.79 | B | C |
| ATOM | 3186 | C | ILE | B | 109 | 17.799 | 57.624 | 89.698 | 1.00 | 15.37 | B | C |
| ATOM | 3187 | O | ILE | B | 109 | 17.471 | 58.414 | 90.575 | 1.00 | 15.37 | B | O |
| ATOM | 3188 | N | VAL | B | 110 | 18.054 | 57.993 | 88.448 | 1.00 | 17.53 | B | N |
| ATOM | 3189 | CA | VAL | B | 110 | 17.903 | 59.375 | 88.025 | 1.00 | 17.53 | B | C |
| ATOM | 3190 | CB | VAL | B | 110 | 18.263 | 59.533 | 86.511 | 1.00 | 13.38 | B | C |
| ATOM | 3191 | CG1 | VAL | B | 110 | 17.155 | 59.003 | 85.625 | 1.00 | 13.38 | B | C |
| ATOM | 3192 | CG2 | VAL | B | 110 | 18.567 | 60.970 | 86.200 | 1.00 | 13.38 | B | C |
| ATOM | 3193 | C | VAL | B | 110 | 16.474 | 59.804 | 88.321 | 1.00 | 17.53 | B | C |
| ATOM | 3194 | O | VAL | B | 110 | 15.526 | 59.045 | 88.124 | 1.00 | 17.53 | B | O |
| ATOM | 3195 | N | ARG | B | 111 | 16.337 | 61.021 | 88.824 | 1.00 | 25.48 | B | N |
| ATOM | 3196 | CA | ARG | B | 111 | 15.035 | 61.568 | 89.168 | 1.00 | 25.48 | B | C |
| ATOM | 3197 | CB | ARG | B | 111 | 15.192 | 62.526 | 90.348 | 1.00 | 64.22 | B | C |
| ATOM | 3198 | CG | ARG | B | 111 | 13.948 | 63.320 | 90.673 | 1.00 | 64.22 | B | C |
| ATOM | 3199 | CD | ARG | B | 111 | 13.593 | 63.204 | 92.146 | 1.00 | 64.22 | B | C |
| ATOM | 3200 | NE | ARG | B | 111 | 13.800 | 64.459 | 92.873 | 1.00 | 64.22 | B | N |
| ATOM | 3201 | CZ | ARG | B | 111 | 14.964 | 65.109 | 92.969 | 1.00 | 64.22 | B | C |
| ATOM | 3202 | NH1 | ARG | B | 111 | 16.060 | 64.637 | 92.378 | 1.00 | 64.22 | B | N |
| ATOM | 3203 | NH2 | ARG | B | 111 | 15.036 | 66.233 | 93.678 | 1.00 | 64.22 | B | N |
| ATOM | 3204 | C | ARG | B | 111 | 14.298 | 62.280 | 88.029 | 1.00 | 25.48 | B | C |
| ATOM | 3205 | O | ARG | B | 111 | 14.895 | 62.999 | 87.225 | 1.00 | 25.48 | B | O |
| ATOM | 3206 | N | LEU | B | 112 | 12.989 | 62.061 | 87.972 | 1.00 | 20.16 | B | N |
| ATOM | 3207 | CA | LEU | B | 112 | 12.132 | 62.711 | 86.993 | 1.00 | 20.16 | B | C |
| ATOM | 3208 | CB | LEU | B | 112 | 10.974 | 61.789 | 86.604 | 1.00 | 19.51 | B | C |
| ATOM | 3209 | CG | LEU | B | 112 | 9.895 | 62.393 | 85.706 | 1.00 | 19.51 | B | C |
| ATOM | 3210 | CD1 | LEU | B | 112 | 10.462 | 62.661 | 84.326 | 1.00 | 19.51 | B | C |

| | | | | | | | | | | | | |
|---|---|---|---|---|---|---|---|---|---|---|---|---|
| ATOM | 3211 | CD2 | LEU | B | 112 | 8.705 | 61.449 | 85.636 | 1.00 | 19.51 | B | C |
| ATOM | 3212 | C | LEU | B | 112 | 11.614 | 63.955 | 87.718 | 1.00 | 20.16 | B | C |
| ATOM | 3213 | O | LEU | B | 112 | 10.667 | 63.890 | 88.489 | 1.00 | 20.16 | B | O |
| ATOM | 3214 | N | ARG | B | 113 | 12.263 | 65.084 | 87.485 | 1.00 | 27.13 | B | N |
| ATOM | 3215 | CA | ARG | B | 113 | 11.890 | 66.329 | 88.139 | 1.00 | 27.13 | B | C |
| ATOM | 3216 | CB | ARG | B | 113 | 12.906 | 67.410 | 87.775 | 1.00 | 44.98 | B | C |
| ATOM | 3217 | CG | ARG | B | 113 | 14.330 | 67.037 | 88.158 | 1.00 | 44.98 | B | C |
| ATOM | 3218 | CD | ARG | B | 113 | 14.478 | 66.967 | 89.681 | 1.00 | 44.98 | B | C |
| ATOM | 3219 | NE | ARG | B | 113 | 14.592 | 68.301 | 90.272 | 1.00 | 44.98 | B | N |
| ATOM | 3220 | CZ | ARG | B | 113 | 15.606 | 69.123 | 90.024 | 1.00 | 44.98 | B | C |
| ATOM | 3221 | NH1 | ARG | B | 113 | 16.573 | 68.718 | 89.207 | 1.00 | 44.98 | B | N |
| ATOM | 3222 | NH2 | ARG | B | 113 | 15.644 | 70.342 | 90.560 | 1.00 | 44.98 | B | N |
| ATOM | 3223 | C | ARG | B | 113 | 10.491 | 66.757 | 87.736 | 1.00 | 27.13 | B | C |
| ATOM | 3224 | O | ARG | B | 113 | 9.639 | 67.009 | 88.585 | 1.00 | 27.13 | B | O |
| ATOM | 3225 | N | TYR | B | 114 | 10.264 | 66.849 | 86.433 | 1.00 | 37.03 | B | N |
| ATOM | 3226 | CA | TYR | B | 114 | 8.969 | 67.242 | 85.908 | 1.00 | 37.03 | B | C |
| ATOM | 3227 | CB | TYR | B | 114 | 8.954 | 68.743 | 85.621 | 1.00 | 26.59 | B | C |
| ATOM | 3228 | CG | TYR | B | 114 | 9.244 | 69.593 | 86.836 | 1.00 | 26.59 | B | C |
| ATOM | 3229 | CD1 | TYR | B | 114 | 8.293 | 69.775 | 87.827 | 1.00 | 26.59 | B | C |
| ATOM | 3230 | CE1 | TYR | B | 114 | 8.578 | 70.507 | 88.965 | 1.00 | 26.59 | B | C |
| ATOM | 3231 | CD2 | TYR | B | 114 | 10.485 | 70.173 | 87.018 | 1.00 | 26.59 | B | C |
| ATOM | 3232 | CE2 | TYR | B | 114 | 10.772 | 70.902 | 88.155 | 1.00 | 26.59 | B | C |
| ATOM | 3233 | CZ | TYR | B | 114 | 9.817 | 71.059 | 89.124 | 1.00 | 26.59 | B | C |
| ATOM | 3234 | OH | TYR | B | 114 | 10.123 | 71.730 | 90.279 | 1.00 | 26.59 | B | O |
| ATOM | 3235 | C | TYR | B | 114 | 8.745 | 66.485 | 84.621 | 1.00 | 37.03 | B | C |
| ATOM | 3236 | O | TYR | B | 114 | 9.555 | 65.651 | 84.235 | 1.00 | 37.03 | B | O |
| ATOM | 3237 | N | PHE | B | 115 | 7.624 | 66.760 | 83.974 | 1.00 | 26.55 | B | N |
| ATOM | 3238 | CA | PHE | B | 115 | 7.315 | 66.144 | 82.700 | 1.00 | 26.55 | B | C |
| ATOM | 3239 | CB | PHE | B | 115 | 6.907 | 64.655 | 82.860 | 1.00 | 24.05 | B | C |
| ATOM | 3240 | CG | PHE | B | 115 | 5.510 | 64.415 | 83.383 | 1.00 | 24.05 | B | C |
| ATOM | 3241 | CD1 | PHE | B | 115 | 4.418 | 64.488 | 82.544 | 1.00 | 24.05 | B | C |
| ATOM | 3242 | CD2 | PHE | B | 115 | 5.301 | 64.043 | 84.710 | 1.00 | 24.05 | B | C |
| ATOM | 3243 | CE1 | PHE | B | 115 | 3.154 | 64.192 | 83.023 | 1.00 | 24.05 | B | C |
| ATOM | 3244 | CE2 | PHE | B | 115 | 4.032 | 63.749 | 85.186 | 1.00 | 24.05 | B | C |
| ATOM | 3245 | CZ | PHE | B | 115 | 2.968 | 63.821 | 84.348 | 1.00 | 24.05 | B | C |
| ATOM | 3246 | C | PHE | B | 115 | 6.220 | 67.012 | 82.147 | 1.00 | 26.55 | B | C |
| ATOM | 3247 | O | PHE | B | 115 | 5.408 | 67.531 | 82.912 | 1.00 | 26.55 | B | O |
| ATOM | 3248 | N | PHE | B | 116 | 6.230 | 67.213 | 80.833 | 1.00 | 32.58 | B | N |
| ATOM | 3249 | CA | PHE | B | 116 | 5.236 | 68.064 | 80.196 | 1.00 | 32.58 | B | C |
| ATOM | 3250 | CB | PHE | B | 116 | 5.601 | 69.536 | 80.432 | 1.00 | 30.53 | B | C |
| ATOM | 3251 | CG | PHE | B | 116 | 6.910 | 69.952 | 79.821 | 1.00 | 30.53 | B | C |
| ATOM | 3252 | CD1 | PHE | B | 116 | 6.981 | 70.351 | 78.489 | 1.00 | 30.53 | B | C |
| ATOM | 3253 | CD2 | PHE | B | 116 | 8.076 | 69.947 | 80.586 | 1.00 | 30.53 | B | C |
| ATOM | 3254 | CE1 | PHE | B | 116 | 8.204 | 70.747 | 77.927 | 1.00 | 30.53 | B | C |
| ATOM | 3255 | CE2 | PHE | B | 116 | 9.301 | 70.336 | 80.043 | 1.00 | 30.53 | B | C |
| ATOM | 3256 | CZ | PHE | B | 116 | 9.369 | 70.738 | 78.711 | 1.00 | 30.53 | B | C |
| ATOM | 3257 | C | PHE | B | 116 | 5.092 | 67.816 | 78.710 | 1.00 | 32.58 | B | C |
| ATOM | 3258 | O | PHE | B | 116 | 5.992 | 67.292 | 78.069 | 1.00 | 32.58 | B | O |
| ATOM | 3259 | N | TYR | B | 117 | 3.939 | 68.191 | 78.172 | 1.00 | 45.46 | B | N |
| ATOM | 3260 | CA | TYR | B | 117 | 3.687 | 68.037 | 76.757 | 1.00 | 45.46 | B | C |
| ATOM | 3261 | CB | TYR | B | 117 | 2.286 | 67.504 | 76.531 | 1.00 | 31.09 | B | C |
| ATOM | 3262 | CG | TYR | B | 117 | 2.031 | 66.260 | 77.316 | 1.00 | 31.09 | B | C |
| ATOM | 3263 | CD1 | TYR | B | 117 | 1.808 | 66.322 | 78.676 | 1.00 | 31.09 | B | C |
| ATOM | 3264 | CE1 | TYR | B | 117 | 1.585 | 65.173 | 79.411 | 1.00 | 31.09 | B | C |
| ATOM | 3265 | CD2 | TYR | B | 117 | 2.025 | 65.013 | 76.703 | 1.00 | 31.09 | B | C |
| ATOM | 3266 | CE2 | TYR | B | 117 | 1.803 | 63.852 | 77.435 | 1.00 | 31.09 | B | C |
| ATOM | 3267 | CZ | TYR | B | 117 | 1.587 | 63.943 | 78.786 | 1.00 | 31.09 | B | C |
| ATOM | 3268 | OH | TYR | B | 117 | 1.407 | 62.802 | 79.523 | 1.00 | 31.09 | B | O |
| ATOM | 3269 | C | TYR | B | 117 | 3.855 | 69.387 | 76.078 | 1.00 | 45.46 | B | C |
| ATOM | 3270 | O | TYR | B | 117 | 3.594 | 70.438 | 76.664 | 1.00 | 45.46 | B | O |
| ATOM | 3271 | N | SER | B | 118 | 4.332 | 69.336 | 74.847 | 1.00 | 60.64 | B | N |
| ATOM | 3272 | CA | SER | B | 118 | 4.542 | 70.515 | 74.013 | 1.00 | 60.64 | B | C |
| ATOM | 3273 | CB | SER | B | 118 | 5.874 | 71.186 | 74.336 | 1.00 | 49.95 | B | C |
| ATOM | 3274 | OG | SER | B | 118 | 6.954 | 70.339 | 73.983 | 1.00 | 49.95 | B | O |
| ATOM | 3275 | C | SER | B | 118 | 4.642 | 69.835 | 72.657 | 1.00 | 60.64 | B | C |
| ATOM | 3276 | O | SER | B | 118 | 5.628 | 69.140 | 72.359 | 1.00 | 60.64 | B | O |
| ATOM | 3277 | N | SER | B | 119 | 3.618 | 69.980 | 71.842 | 1.00 | 70.17 | B | N |

| | | | | | | | | | | | | |
|---|---|---|---|---|---|---|---|---|---|---|---|---|
| ATOM | 3278 | CA | SER | B | 119 | 3.673 | 69.338 | 70.537 | 1.00 | 70.17 | B | C |
| ATOM | 3279 | CB | SER | B | 119 | 2.334 | 69.560 | 69.791 | 1.00 | 81.49 | B | C |
| ATOM | 3280 | OG | SER | B | 119 | 2.076 | 70.953 | 69.621 | 1.00 | 81.49 | B | O |
| ATOM | 3281 | C | SER | B | 119 | 4.791 | 70.059 | 69.740 | 1.00 | 70.17 | B | C |
| ATOM | 3282 | O | SER | B | 119 | 5.119 | 71.240 | 69.994 | 1.00 | 70.17 | B | O |
| ATOM | 3283 | N | GLY | B | 120 | 5.345 | 69.349 | 68.760 | 1.00 | 99.28 | B | N |
| ATOM | 3284 | CA | GLY | B | 120 | 6.418 | 69.903 | 67.956 | 1.00 | 99.28 | B | C |
| ATOM | 3285 | C | GLY | B | 120 | 6.005 | 70.033 | 66.513 | 1.00 | 99.28 | B | C |
| ATOM | 3286 | O | GLY | B | 120 | 4.884 | 70.496 | 66.229 | 1.00 | 99.28 | B | O |
| ATOM | 3287 | N | ALA | B | 121 | 6.897 | 69.604 | 65.614 | 1.00 | 98.96 | B | N |
| ATOM | 3288 | CA | ALA | B | 121 | 6.673 | 69.685 | 64.169 | 1.00 | 98.96 | B | C |
| ATOM | 3289 | CB | ALA | B | 121 | 7.574 | 68.657 | 63.454 | 1.00 | 18.56 | B | C |
| ATOM | 3290 | C | ALA | B | 121 | 5.188 | 69.533 | 63.715 | 1.00 | 98.96 | B | C |
| ATOM | 3291 | O | ALA | B | 121 | 4.380 | 70.482 | 63.852 | 1.00 | 98.96 | B | O |
| ATOM | 3292 | N | ALA | B | 122 | 4.832 | 68.352 | 63.195 | 1.00100.00 | | B | N |
| ATOM | 3293 | CA | ALA | B | 122 | 3.469 | 68.094 | 62.709 | 1.00100.00 | | B | C |
| ATOM | 3294 | CB | ALA | B | 122 | 3.314 | 66.620 | 62.318 | 1.00 | 80.62 | B | C |
| ATOM | 3295 | C | ALA | B | 122 | 2.345 | 68.504 | 63.675 | 1.00100.00 | | B | C |
| ATOM | 3296 | O | ALA | B | 122 | 2.532 | 68.564 | 64.903 | 1.00100.00 | | B | O |
| ATOM | 3297 | N | ALA | B | 123 | 1.178 | 68.780 | 63.085 | 1.00 | 68.46 | B | N |
| ATOM | 3298 | CA | ALA | B | 123 | -0.030 | 69.223 | 63.801 | 1.00 | 68.46 | B | C |
| ATOM | 3299 | CB | ALA | B | 123 | -1.176 | 69.495 | 62.785 | 1.00 | 56.00 | B | C |
| ATOM | 3300 | C | ALA | B | 123 | -0.531 | 68.273 | 64.886 | 1.00 | 68.46 | B | C |
| ATOM | 3301 | O | ALA | B | 123 | -0.417 | 68.554 | 66.088 | 1.00 | 68.46 | B | O |
| ATOM | 3302 | N | ALA | B | 124 | -1.132 | 67.168 | 64.453 | 1.00100.00 | | B | N |
| ATOM | 3303 | CA | ALA | B | 124 | -1.650 | 66.169 | 65.389 | 1.00100.00 | | B | C |
| ATOM | 3304 | CB | ALA | B | 124 | -2.684 | 65.265 | 64.688 | 1.00 | 71.71 | B | C |
| ATOM | 3305 | C | ALA | B | 124 | -0.459 | 65.337 | 65.875 | 1.00100.00 | | B | C |
| ATOM | 3306 | O | ALA | B | 124 | -0.558 | 64.112 | 66.060 | 1.00100.00 | | B | O |
| ATOM | 3307 | N | GLU | B | 125 | 0.668 | 66.027 | 66.059 | 1.00 | 78.20 | B | N |
| ATOM | 3308 | CA | GLU | B | 125 | 1.922 | 65.422 | 66.492 | 1.00 | 78.20 | B | C |
| ATOM | 3309 | CB | GLU | B | 125 | 2.981 | 65.694 | 65.408 | 1.00 | 81.30 | B | C |
| ATOM | 3310 | CG | GLU | B | 125 | 4.273 | 64.892 | 65.512 | 1.00 | 81.30 | B | C |
| ATOM | 3311 | CD | GLU | B | 125 | 5.239 | 65.147 | 64.317 | 1.00 | 81.30 | B | C |
| ATOM | 3312 | OE1 | GLU | B | 125 | 5.209 | 64.380 | 63.302 | 1.00 | 81.30 | B | O |
| ATOM | 3313 | OE2 | GLU | B | 125 | 6.018 | 66.135 | 64.405 | 1.00 | 81.30 | B | O |
| ATOM | 3314 | C | GLU | B | 125 | 2.322 | 66.040 | 67.846 | 1.00 | 78.20 | B | C |
| ATOM | 3315 | O | GLU | B | 125 | 2.801 | 67.189 | 67.908 | 1.00 | 78.20 | B | O |
| ATOM | 3316 | N | VAL | B | 126 | 2.100 | 65.294 | 68.930 | 1.00 | 48.91 | B | N |
| ATOM | 3317 | CA | VAL | B | 126 | 2.438 | 65.811 | 70.259 | 1.00 | 48.91 | B | C |
| ATOM | 3318 | CB | VAL | B | 126 | 1.263 | 65.702 | 71.236 | 1.00 | 42.54 | B | C |
| ATOM | 3319 | CG1 | VAL | B | 126 | 0.038 | 66.299 | 70.611 | 1.00 | 42.54 | B | C |
| ATOM | 3320 | CG2 | VAL | B | 126 | 1.046 | 64.255 | 71.638 | 1.00 | 42.54 | B | C |
| ATOM | 3321 | C | VAL | B | 126 | 3.641 | 65.155 | 70.923 | 1.00 | 48.91 | B | C |
| ATOM | 3322 | O | VAL | B | 126 | 4.021 | 64.013 | 70.616 | 1.00 | 48.91 | B | O |
| ATOM | 3323 | N | TYR | B | 127 | 4.237 | 65.887 | 71.854 | 1.00 | 49.97 | B | N |
| ATOM | 3324 | CA | TYR | B | 127 | 5.392 | 65.358 | 72.540 | 1.00 | 49.97 | B | C |
| ATOM | 3325 | CB | TYR | B | 127 | 6.639 | 66.162 | 72.183 | 1.00 | 68.51 | B | C |
| ATOM | 3326 | CG | TYR | B | 127 | 7.024 | 66.011 | 70.744 | 1.00 | 68.51 | B | C |
| ATOM | 3327 | CD1 | TYR | B | 127 | 6.937 | 67.091 | 69.869 | 1.00 | 68.51 | B | C |
| ATOM | 3328 | CE1 | TYR | B | 127 | 7.237 | 66.947 | 68.515 | 1.00 | 68.51 | B | C |
| ATOM | 3329 | CD2 | TYR | B | 127 | 7.425 | 64.769 | 70.238 | 1.00 | 68.51 | B | C |
| ATOM | 3330 | CE2 | TYR | B | 127 | 7.725 | 64.608 | 68.890 | 1.00 | 68.51 | B | C |
| ATOM | 3331 | CZ | TYR | B | 127 | 7.630 | 65.708 | 68.029 | 1.00 | 68.51 | B | C |
| ATOM | 3332 | OH | TYR | B | 127 | 7.924 | 65.596 | 66.682 | 1.00 | 68.51 | B | O |
| ATOM | 3333 | C | TYR | B | 127 | 5.319 | 65.214 | 74.054 | 1.00 | 49.97 | B | C |
| ATOM | 3334 | O | TYR | B | 127 | 4.628 | 65.971 | 74.747 | 1.00 | 49.97 | B | O |
| ATOM | 3335 | N | LEU | B | 128 | 6.042 | 64.218 | 74.557 | 1.00 | 36.31 | B | N |
| ATOM | 3336 | CA | LEU | B | 128 | 6.128 | 63.984 | 75.984 | 1.00 | 36.31 | B | C |
| ATOM | 3337 | CB | LEU | B | 128 | 6.044 | 62.494 | 76.315 | 1.00 | 34.80 | B | C |
| ATOM | 3338 | CG | LEU | B | 128 | 6.162 | 62.161 | 77.804 | 1.00 | 34.80 | B | C |
| ATOM | 3339 | CD1 | LEU | B | 128 | 4.988 | 62.764 | 78.566 | 1.00 | 34.80 | B | C |
| ATOM | 3340 | CD2 | LEU | B | 128 | 6.192 | 60.657 | 77.982 | 1.00 | 34.80 | B | C |
| ATOM | 3341 | C | LEU | B | 128 | 7.513 | 64.484 | 76.305 | 1.00 | 36.31 | B | C |
| ATOM | 3342 | O | LEU | B | 128 | 8.471 | 64.113 | 75.640 | 1.00 | 36.31 | B | O |
| ATOM | 3343 | N | ASN | B | 129 | 7.619 | 65.362 | 77.288 | 1.00 | 27.12 | B | N |
| ATOM | 3344 | CA | ASN | B | 129 | 8.918 | 65.895 | 77.665 | 1.00 | 27.12 | B | C |

| | | | | | | | | | | | | |
|---|---|---|---|---|---|---|---|---|---|---|---|---|
| ATOM | 3345 | CB | ASN | B | 129 | 8.888 | 67.422 | 77.655 | 1.00 | 27.30 | B | C |
| ATOM | 3346 | CG | ASN | B | 129 | 8.807 | 67.992 | 76.258 | 1.00 | 27.30 | B | C |
| ATOM | 3347 | OD1 | ASN | B | 129 | 9.821 | 68.160 | 75.575 | 1.00 | 27.30 | B | O |
| ATOM | 3348 | ND2 | ASN | B | 129 | 7.592 | 68.282 | 75.816 | 1.00 | 27.30 | B | N |
| ATOM | 3349 | C | ASN | B | 129 | 9.240 | 65.379 | 79.061 | 1.00 | 27.12 | B | C |
| ATOM | 3350 | O | ASN | B | 129 | 8.470 | 65.583 | 79.998 | 1.00 | 27.12 | B | O |
| ATOM | 3351 | N | LEU | B | 130 | 10.366 | 64.686 | 79.197 | 1.00 | 36.25 | B | N |
| ATOM | 3352 | CA | LEU | B | 130 | 10.755 | 64.155 | 80.492 | 1.00 | 36.25 | B | C |
| ATOM | 3353 | CB | LEU | B | 130 | 11.065 | 62.652 | 80.402 | 1.00 | 25.20 | B | C |
| ATOM | 3354 | CG | LEU | B | 130 | 9.817 | 61.762 | 80.344 | 1.00 | 25.20 | B | C |
| ATOM | 3355 | CD1 | LEU | B | 130 | 9.018 | 62.111 | 79.126 | 1.00 | 25.20 | B | C |
| ATOM | 3356 | CD2 | LEU | B | 130 | 10.190 | 60.304 | 80.314 | 1.00 | 25.20 | B | C |
| ATOM | 3357 | C | LEU | B | 130 | 11.959 | 64.912 | 81.014 | 1.00 | 36.25 | B | C |
| ATOM | 3358 | O | LEU | B | 130 | 13.067 | 64.784 | 80.498 | 1.00 | 36.25 | B | O |
| ATOM | 3359 | N | VAL | B | 131 | 11.718 | 65.739 | 82.021 | 1.00 | 32.29 | B | N |
| ATOM | 3360 | CA | VAL | B | 131 | 12.781 | 66.512 | 82.630 | 1.00 | 32.29 | B | C |
| ATOM | 3361 | CB | VAL | B | 131 | 12.244 | 67.819 | 83.228 | 1.00 | 20.44 | B | C |
| ATOM | 3362 | CG1 | VAL | B | 131 | 13.356 | 68.542 | 83.978 | 1.00 | 20.44 | B | C |
| ATOM | 3363 | CG2 | VAL | B | 131 | 11.708 | 68.705 | 82.126 | 1.00 | 20.44 | B | C |
| ATOM | 3364 | C | VAL | B | 131 | 13.400 | 65.670 | 83.737 | 1.00 | 32.29 | B | C |
| ATOM | 3365 | O | VAL | B | 131 | 12.825 | 65.509 | 84.818 | 1.00 | 32.29 | B | O |
| ATOM | 3366 | N | LEU | B | 132 | 14.581 | 65.137 | 83.453 | 1.00 | 25.43 | B | N |
| ATOM | 3367 | CA | LEU | B | 132 | 15.293 | 64.294 | 84.395 | 1.00 | 25.43 | B | C |
| ATOM | 3368 | CB | LEU | B | 132 | 15.813 | 63.067 | 83.667 | 1.00 | 13.04 | B | C |
| ATOM | 3369 | CG | LEU | B | 132 | 14.693 | 62.276 | 83.012 | 1.00 | 13.04 | B | C |
| ATOM | 3370 | CD1 | LEU | B | 132 | 15.119 | 61.805 | 81.647 | 1.00 | 13.04 | B | C |
| ATOM | 3371 | CD2 | LEU | B | 132 | 14.316 | 61.128 | 83.916 | 1.00 | 13.04 | B | C |
| ATOM | 3372 | C | LEU | B | 132 | 16.451 | 65.059 | 84.968 | 1.00 | 25.43 | B | C |
| ATOM | 3373 | O | LEU | B | 132 | 16.802 | 66.115 | 84.463 | 1.00 | 25.43 | B | O |
| ATOM | 3374 | N | ASP | B | 133 | 17.030 | 64.541 | 86.040 | 1.00 | 23.27 | B | N |
| ATOM | 3375 | CA | ASP | B | 133 | 18.189 | 65.177 | 86.635 | 1.00 | 23.27 | B | C |
| ATOM | 3376 | CB | ASP | B | 133 | 18.625 | 64.414 | 87.872 | 1.00 | 34.83 | B | C |
| ATOM | 3377 | CG | ASP | B | 133 | 17.980 | 64.937 | 89.132 | 1.00 | 34.83 | B | C |
| ATOM | 3378 | OD1 | ASP | B | 133 | 17.884 | 64.138 | 90.098 | 1.00 | 34.83 | B | O |
| ATOM | 3379 | OD2 | ASP | B | 133 | 17.590 | 66.137 | 89.143 | 1.00 | 34.83 | B | O |
| ATOM | 3380 | C | ASP | B | 133 | 19.278 | 65.074 | 85.597 | 1.00 | 23.27 | B | C |
| ATOM | 3381 | O | ASP | B | 133 | 19.195 | 64.246 | 84.698 | 1.00 | 23.27 | B | O |
| ATOM | 3382 | N | TYR | B | 134 | 20.287 | 65.926 | 85.685 | 1.00 | 29.56 | B | N |
| ATOM | 3383 | CA | TYR | B | 134 | 21.386 | 65.811 | 84.743 | 1.00 | 29.56 | B | C |
| ATOM | 3384 | CB | TYR | B | 134 | 21.689 | 67.137 | 84.038 | 1.00 | 37.41 | B | C |
| ATOM | 3385 | CG | TYR | B | 134 | 22.869 | 66.994 | 83.101 | 1.00 | 37.41 | B | C |
| ATOM | 3386 | CD1 | TYR | B | 134 | 22.747 | 66.292 | 81.900 | 1.00 | 37.41 | B | C |
| ATOM | 3387 | CE1 | TYR | B | 134 | 23.856 | 65.982 | 81.133 | 1.00 | 37.41 | B | C |
| ATOM | 3388 | CD2 | TYR | B | 134 | 24.141 | 67.400 | 83.495 | 1.00 | 37.41 | B | C |
| ATOM | 3389 | CE2 | TYR | B | 134 | 25.253 | 67.092 | 82.737 | 1.00 | 37.41 | B | C |
| ATOM | 3390 | CZ | TYR | B | 134 | 25.106 | 66.377 | 81.563 | 1.00 | 37.41 | B | C |
| ATOM | 3391 | OH | TYR | B | 134 | 26.225 | 66.006 | 80.857 | 1.00 | 37.41 | B | O |
| ATOM | 3392 | C | TYR | B | 134 | 22.633 | 65.355 | 85.506 | 1.00 | 29.56 | B | C |
| ATOM | 3393 | O | TYR | B | 134 | 23.037 | 65.973 | 86.480 | 1.00 | 29.56 | B | O |
| ATOM | 3394 | N | VAL | B | 135 | 23.226 | 64.257 | 85.064 | 1.00 | 23.65 | B | N |
| ATOM | 3395 | CA | VAL | B | 135 | 24.434 | 63.732 | 85.675 | 1.00 | 23.65 | B | C |
| ATOM | 3396 | CB | VAL | B | 135 | 24.253 | 62.257 | 86.005 | 1.00 | 12.68 | B | C |
| ATOM | 3397 | CG1 | VAL | B | 135 | 25.449 | 61.747 | 86.776 | 1.00 | 12.68 | B | C |
| ATOM | 3398 | CG2 | VAL | B | 135 | 22.966 | 62.058 | 86.764 | 1.00 | 12.68 | B | C |
| ATOM | 3399 | C | VAL | B | 135 | 25.543 | 63.885 | 84.615 | 1.00 | 23.65 | B | C |
| ATOM | 3400 | O | VAL | B | 135 | 25.438 | 63.388 | 83.493 | 1.00 | 23.65 | B | O |
| ATOM | 3401 | N | PRO | B | 136 | 26.631 | 64.557 | 84.977 | 1.00 | 32.08 | B | N |
| ATOM | 3402 | CD | PRO | B | 136 | 26.897 | 64.888 | 86.387 | 1.00 | 35.00 | B | C |
| ATOM | 3403 | CA | PRO | B | 136 | 27.803 | 64.855 | 84.162 | 1.00 | 32.08 | B | C |
| ATOM | 3404 | CB | PRO | B | 136 | 28.592 | 65.769 | 85.069 | 1.00 | 35.00 | B | C |
| ATOM | 3405 | CG | PRO | B | 136 | 28.376 | 65.125 | 86.390 | 1.00 | 35.00 | B | C |
| ATOM | 3406 | C | PRO | B | 136 | 28.660 | 63.709 | 83.652 | 1.00 | 32.08 | B | C |
| ATOM | 3407 | O | PRO | B | 136 | 29.318 | 63.852 | 82.631 | 1.00 | 32.08 | B | O |
| ATOM | 3408 | N | GLU | B | 137 | 28.679 | 62.585 | 84.352 | 1.00 | 21.83 | B | N |
| ATOM | 3409 | CA | GLU | B | 137 | 29.514 | 61.482 | 83.899 | 1.00 | 21.83 | B | C |
| ATOM | 3410 | CB | GLU | B | 137 | 30.710 | 61.309 | 84.842 | 1.00 | 49.68 | B | C |
| ATOM | 3411 | CG | GLU | B | 137 | 31.888 | 60.579 | 84.210 | 1.00 | 49.68 | B | C |

| | | | | | | | | | | | | |
|---|---|---|---|---|---|---|---|---|---|---|---|---|
| ATOM | 3412 | CD | GLU | B | 137 | 32.338 | 61.247 | 82.921 | 1.00 | 49.68 | B | C |
| ATOM | 3413 | OE1 | GLU | B | 137 | 32.990 | 62.321 | 82.997 | 1.00 | 49.68 | B | O |
| ATOM | 3414 | OE2 | GLU | B | 137 | 32.020 | 60.702 | 81.838 | 1.00 | 49.68 | B | O |
| ATOM | 3415 | C | GLU | B | 137 | 28.751 | 60.162 | 83.760 | 1.00 | 21.83 | B | C |
| ATOM | 3416 | O | GLU | B | 137 | 27.601 | 60.050 | 84.166 | 1.00 | 21.83 | B | O |
| ATOM | 3417 | N | THR | B | 138 | 29.390 | 59.167 | 83.158 | 1.00 | 24.10 | B | N |
| ATOM | 3418 | CA | THR | B | 138 | 28.777 | 57.854 | 83.006 | 1.00 | 24.10 | B | C |
| ATOM | 3419 | CB | THR | B | 138 | 28.101 | 57.652 | 81.635 | 1.00 | 24.34 | B | C |
| ATOM | 3420 | OG1 | THR | B | 138 | 29.099 | 57.625 | 80.609 | 1.00 | 24.34 | B | O |
| ATOM | 3421 | CG2 | THR | B | 138 | 27.121 | 58.745 | 81.352 | 1.00 | 24.34 | B | C |
| ATOM | 3422 | C | THR | B | 138 | 29.819 | 56.759 | 83.090 | 1.00 | 24.10 | B | C |
| ATOM | 3423 | O | THR | B | 138 | 30.930 | 56.917 | 82.617 | 1.00 | 24.10 | B | O |
| ATOM | 3424 | N | VAL | B | 139 | 29.440 | 55.631 | 83.666 | 1.00 | 29.14 | B | N |
| ATOM | 3425 | CA | VAL | B | 139 | 30.351 | 54.510 | 83.780 | 1.00 | 29.14 | B | C |
| ATOM | 3426 | CB | VAL | B | 139 | 29.625 | 53.263 | 84.322 | 1.00 | 26.52 | B | C |
| ATOM | 3427 | CG1 | VAL | B | 139 | 30.490 | 52.023 | 84.144 | 1.00 | 26.52 | B | C |
| ATOM | 3428 | CG2 | VAL | B | 139 | 29.314 | 53.455 | 85.801 | 1.00 | 26.52 | B | C |
| ATOM | 3429 | C | VAL | B | 139 | 30.970 | 54.180 | 82.426 | 1.00 | 29.14 | B | C |
| ATOM | 3430 | O | VAL | B | 139 | 32.128 | 53.799 | 82.358 | 1.00 | 29.14 | B | O |
| ATOM | 3431 | N | TYR | B | 140 | 30.202 | 54.314 | 81.354 | 1.00 | 34.44 | B | N |
| ATOM | 3432 | CA | TYR | B | 140 | 30.724 | 54.026 | 80.021 | 1.00 | 34.44 | B | C |
| ATOM | 3433 | CB | TYR | B | 140 | 29.654 | 54.272 | 78.958 | 1.00 | 29.70 | B | C |
| ATOM | 3434 | CG | TYR | B | 140 | 30.192 | 54.178 | 77.551 | 1.00 | 29.70 | B | C |
| ATOM | 3435 | CD1 | TYR | B | 140 | 30.655 | 52.966 | 77.044 | 1.00 | 29.70 | B | C |
| ATOM | 3436 | CE1 | TYR | B | 140 | 31.194 | 52.872 | 75.763 | 1.00 | 29.70 | B | C |
| ATOM | 3437 | CD2 | TYR | B | 140 | 30.280 | 55.303 | 76.737 | 1.00 | 29.70 | B | C |
| ATOM | 3438 | CE2 | TYR | B | 140 | 30.818 | 55.219 | 75.457 | 1.00 | 29.70 | B | C |
| ATOM | 3439 | CZ | TYR | B | 140 | 31.274 | 53.995 | 74.974 | 1.00 | 29.70 | B | C |
| ATOM | 3440 | OH | TYR | B | 140 | 31.812 | 53.878 | 73.708 | 1.00 | 29.70 | B | O |
| ATOM | 3441 | C | TYR | B | 140 | 31.945 | 54.895 | 79.691 | 1.00 | 34.44 | B | C |
| ATOM | 3442 | O | TYR | B | 140 | 33.038 | 54.381 | 79.447 | 1.00 | 34.44 | B | O |
| ATOM | 3443 | N | ARG | B | 141 | 31.757 | 56.213 | 79.665 | 1.00 | 33.60 | B | N |
| ATOM | 3444 | CA | ARG | B | 141 | 32.856 | 57.124 | 79.360 | 1.00 | 33.60 | B | C |
| ATOM | 3445 | CB | ARG | B | 141 | 32.448 | 58.584 | 79.558 | 1.00 | 48.27 | B | C |
| ATOM | 3446 | CG | ARG | B | 141 | 31.232 | 59.046 | 78.805 | 1.00 | 48.27 | B | C |
| ATOM | 3447 | CD | ARG | B | 141 | 30.992 | 60.529 | 79.071 | 1.00 | 48.27 | B | C |
| ATOM | 3448 | NE | ARG | B | 141 | 31.972 | 61.383 | 78.398 | 1.00 | 48.27 | B | N |
| ATOM | 3449 | CZ | ARG | B | 141 | 32.585 | 62.428 | 78.956 | 1.00 | 48.27 | B | C |
| ATOM | 3450 | NH1 | ARG | B | 141 | 32.329 | 62.771 | 80.220 | 1.00 | 48.27 | B | N |
| ATOM | 3451 | NH2 | ARG | B | 141 | 33.470 | 63.129 | 78.247 | 1.00 | 48.27 | B | N |
| ATOM | 3452 | C | ARG | B | 141 | 34.065 | 56.866 | 80.251 | 1.00 | 33.60 | B | C |
| ATOM | 3453 | O | ARG | B | 141 | 35.192 | 56.805 | 79.773 | 1.00 | 33.60 | B | O |
| ATOM | 3454 | N | VAL | B | 142 | 33.823 | 56.737 | 81.550 | 1.00 | 26.50 | B | N |
| ATOM | 3455 | CA | VAL | B | 142 | 34.882 | 56.509 | 82.527 | 1.00 | 26.50 | B | C |
| ATOM | 3456 | CB | VAL | B | 142 | 34.312 | 56.423 | 83.960 | 1.00 | 20.22 | B | C |
| ATOM | 3457 | CG1 | VAL | B | 142 | 35.252 | 55.656 | 84.857 | 1.00 | 20.22 | B | C |
| ATOM | 3458 | CG2 | VAL | B | 142 | 34.089 | 57.817 | 84.509 | 1.00 | 20.22 | B | C |
| ATOM | 3459 | C | VAL | B | 142 | 35.677 | 55.253 | 82.256 | 1.00 | 26.50 | B | C |
| ATOM | 3460 | O | VAL | B | 142 | 36.890 | 55.256 | 82.395 | 1.00 | 26.50 | B | O |
| ATOM | 3461 | N | ALA | B | 143 | 34.997 | 54.178 | 81.890 | 1.00 | 28.17 | B | N |
| ATOM | 3462 | CA | ALA | B | 143 | 35.676 | 52.924 | 81.602 | 1.00 | 28.17 | B | C |
| ATOM | 3463 | CB | ALA | B | 143 | 34.655 | 51.826 | 81.355 | 1.00 | 20.98 | B | C |
| ATOM | 3464 | C | ALA | B | 143 | 36.538 | 53.120 | 80.364 | 1.00 | 28.17 | B | C |
| ATOM | 3465 | O | ALA | B | 143 | 37.686 | 52.679 | 80.310 | 1.00 | 28.17 | B | O |
| ATOM | 3466 | N | ARG | B | 144 | 35.952 | 53.783 | 79.371 | 1.00 | 29.64 | B | N |
| ATOM | 3467 | CA | ARG | B | 144 | 36.603 | 54.071 | 78.098 | 1.00 | 29.64 | B | C |
| ATOM | 3468 | CB | ARG | B | 144 | 35.749 | 55.069 | 77.311 | 1.00 | 66.90 | B | C |
| ATOM | 3469 | CG | ARG | B | 144 | 35.968 | 55.094 | 75.810 | 1.00 | 66.90 | B | C |
| ATOM | 3470 | CD | ARG | B | 144 | 35.105 | 54.035 | 75.155 | 1.00 | 66.90 | B | C |
| ATOM | 3471 | NE | ARG | B | 144 | 35.125 | 52.804 | 75.951 | 1.00 | 66.90 | B | N |
| ATOM | 3472 | CZ | ARG | B | 144 | 35.000 | 51.567 | 75.462 | 1.00 | 66.90 | B | C |
| ATOM | 3473 | NH1 | ARG | B | 144 | 34.842 | 51.380 | 74.149 | 1.00 | 66.90 | B | N |
| ATOM | 3474 | NH2 | ARG | B | 144 | 35.051 | 50.513 | 76.289 | 1.00 | 66.90 | B | N |
| ATOM | 3475 | C | ARG | B | 144 | 37.950 | 54.707 | 78.376 | 1.00 | 29.64 | B | C |
| ATOM | 3476 | O | ARG | B | 144 | 38.980 | 54.247 | 77.901 | 1.00 | 29.64 | B | O |
| ATOM | 3477 | N | HIS | B | 145 | 37.926 | 55.774 | 79.165 | 1.00 | 32.22 | B | N |
| ATOM | 3478 | CA | HIS | B | 145 | 39.135 | 56.499 | 79.502 | 1.00 | 32.22 | B | C |

| | | | | | | | | | | | | |
|---|---|---|---|---|---|---|---|---|---|---|---|---|
| ATOM | 3479 | CB | HIS | B | 145 | 38.793 | 57.710 | 80.381 | 1.00 | 100.00 | B | C |
| ATOM | 3480 | CG | HIS | B | 145 | 37.954 | 58.737 | 79.677 | 1.00 | 100.00 | B | C |
| ATOM | 3481 | CD2 | HIS | B | 145 | 37.505 | 58.795 | 78.395 | 1.00 | 100.00 | B | C |
| ATOM | 3482 | ND1 | HIS | B | 145 | 37.463 | 59.870 | 80.304 | 1.00 | 100.00 | B | N |
| ATOM | 3483 | CE1 | HIS | B | 145 | 36.747 | 60.576 | 79.440 | 1.00 | 100.00 | B | C |
| ATOM | 3484 | NE2 | HIS | B | 145 | 36.757 | 59.946 | 78.274 | 1.00 | 100.00 | B | N |
| ATOM | 3485 | C | HIS | B | 145 | 40.223 | 55.643 | 80.142 | 1.00 | 32.22 | B | C |
| ATOM | 3486 | O | HIS | B | 145 | 41.366 | 55.688 | 79.696 | 1.00 | 32.22 | B | O |
| ATOM | 3487 | N | TYR | B | 146 | 39.899 | 54.870 | 81.174 | 1.00 | 27.23 | B | N |
| ATOM | 3488 | CA | TYR | B | 146 | 40.928 | 54.031 | 81.774 | 1.00 | 27.23 | B | C |
| ATOM | 3489 | CB | TYR | B | 146 | 40.416 | 53.293 | 83.021 | 1.00 | 28.68 | B | C |
| ATOM | 3490 | CG | TYR | B | 146 | 40.320 | 54.143 | 84.270 | 1.00 | 28.68 | B | C |
| ATOM | 3491 | CD1 | TYR | B | 146 | 39.265 | 55.042 | 84.458 | 1.00 | 28.68 | B | C |
| ATOM | 3492 | CE1 | TYR | B | 146 | 39.207 | 55.867 | 85.587 | 1.00 | 28.68 | B | C |
| ATOM | 3493 | CD2 | TYR | B | 146 | 41.311 | 54.085 | 85.245 | 1.00 | 28.68 | B | C |
| ATOM | 3494 | CE2 | TYR | B | 146 | 41.262 | 54.906 | 86.376 | 1.00 | 28.68 | B | C |
| ATOM | 3495 | CZ | TYR | B | 146 | 40.211 | 55.793 | 86.535 | 1.00 | 28.68 | B | C |
| ATOM | 3496 | OH | TYR | B | 146 | 40.181 | 56.626 | 87.627 | 1.00 | 28.68 | B | O |
| ATOM | 3497 | C | TYR | B | 146 | 41.416 | 53.014 | 80.760 | 1.00 | 27.23 | B | C |
| ATOM | 3498 | O | TYR | B | 146 | 42.603 | 52.738 | 80.682 | 1.00 | 27.23 | B | O |
| ATOM | 3499 | N | SER | B | 147 | 40.497 | 52.466 | 79.974 | 1.00 | 36.56 | B | N |
| ATOM | 3500 | CA | SER | B | 147 | 40.846 | 51.474 | 78.965 | 1.00 | 36.56 | B | C |
| ATOM | 3501 | CB | SER | B | 147 | 39.587 | 50.910 | 78.325 | 1.00 | 48.82 | B | C |
| ATOM | 3502 | OG | SER | B | 147 | 39.914 | 49.888 | 77.402 | 1.00 | 48.82 | B | O |
| ATOM | 3503 | C | SER | B | 147 | 41.736 | 52.066 | 77.885 | 1.00 | 36.56 | B | C |
| ATOM | 3504 | O | SER | B | 147 | 42.738 | 51.468 | 77.497 | 1.00 | 36.56 | B | O |
| ATOM | 3505 | N | ARG | B | 148 | 41.368 | 53.244 | 77.399 | 1.00 | 49.04 | B | N |
| ATOM | 3506 | CA | ARG | B | 148 | 42.149 | 53.897 | 76.358 | 1.00 | 49.04 | B | C |
| ATOM | 3507 | CB | ARG | B | 148 | 41.529 | 55.250 | 75.998 | 1.00 | 51.06 | B | C |
| ATOM | 3508 | CG | ARG | B | 148 | 41.599 | 55.593 | 74.517 | 1.00 | 51.06 | B | C |
| ATOM | 3509 | CD | ARG | B | 148 | 40.238 | 56.087 | 73.980 | 1.00 | 51.06 | B | C |
| ATOM | 3510 | NE | ARG | B | 148 | 39.735 | 57.247 | 74.722 | 1.00 | 51.06 | B | N |
| ATOM | 3511 | CZ | ARG | B | 148 | 38.559 | 57.832 | 74.503 | 1.00 | 51.06 | B | C |
| ATOM | 3512 | NH1 | ARG | B | 148 | 37.752 | 57.366 | 73.552 | 1.00 | 51.06 | B | N |
| ATOM | 3513 | NH2 | ARG | B | 148 | 38.184 | 58.868 | 75.255 | 1.00 | 51.06 | B | N |
| ATOM | 3514 | C | ARG | B | 148 | 43.578 | 54.084 | 76.858 | 1.00 | 49.04 | B | C |
| ATOM | 3515 | O | ARG | B | 148 | 44.528 | 53.628 | 76.221 | 1.00 | 49.04 | B | O |
| ATOM | 3516 | N | ALA | B | 149 | 43.726 | 54.739 | 78.007 | 1.00 | 37.90 | B | N |
| ATOM | 3517 | CA | ALA | B | 149 | 45.044 | 54.979 | 78.592 | 1.00 | 37.90 | B | C |
| ATOM | 3518 | CB | ALA | B | 149 | 44.947 | 56.053 | 79.677 | 1.00 | 38.09 | B | C |
| ATOM | 3519 | C | ALA | B | 149 | 45.660 | 53.713 | 79.156 | 1.00 | 37.90 | B | C |
| ATOM | 3520 | O | ALA | B | 149 | 46.472 | 53.761 | 80.081 | 1.00 | 37.90 | B | O |
| ATOM | 3521 | N | ALA | B | 150 | 45.267 | 52.579 | 78.585 | 1.00 | 42.73 | B | N |
| ATOM | 3522 | CA | ALA | B | 150 | 45.779 | 51.283 | 79.006 | 1.00 | 42.73 | B | C |
| ATOM | 3523 | CB | ALA | B | 150 | 47.082 | 50.977 | 78.279 | 1.00 | 41.86 | B | C |
| ATOM | 3524 | C | ALA | B | 150 | 45.992 | 51.291 | 80.511 | 1.00 | 42.73 | B | C |
| ATOM | 3525 | O | ALA | B | 150 | 47.110 | 51.378 | 81.006 | 1.00 | 42.73 | B | O |
| ATOM | 3526 | N | GLN | B | 151 | 44.889 | 51.238 | 81.230 | 1.00 | 50.70 | B | N |
| ATOM | 3527 | CA | GLN | B | 151 | 44.914 | 51.231 | 82.676 | 1.00 | 50.70 | B | C |
| ATOM | 3528 | CB | GLN | B | 151 | 44.986 | 52.660 | 83.221 | 1.00 | 50.24 | B | C |
| ATOM | 3529 | CG | GLN | B | 151 | 46.383 | 53.236 | 83.282 | 1.00 | 50.24 | B | C |
| ATOM | 3530 | CD | GLN | B | 151 | 46.385 | 54.736 | 83.556 | 1.00 | 50.24 | B | C |
| ATOM | 3531 | OE1 | GLN | B | 151 | 45.717 | 55.215 | 84.476 | 1.00 | 50.24 | B | O |
| ATOM | 3532 | NE2 | GLN | B | 151 | 47.145 | 55.486 | 82.758 | 1.00 | 50.24 | B | N |
| ATOM | 3533 | C | GLN | B | 151 | 43.639 | 50.558 | 83.152 | 1.00 | 50.70 | B | C |
| ATOM | 3534 | O | GLN | B | 151 | 42.738 | 50.256 | 82.353 | 1.00 | 50.70 | B | O |
| ATOM | 3535 | N | THR | B | 152 | 43.565 | 50.325 | 84.454 | 1.00 | 57.34 | B | N |
| ATOM | 3536 | CA | THR | B | 152 | 42.397 | 49.703 | 85.045 | 1.00 | 57.34 | B | C |
| ATOM | 3537 | CB | THR | B | 152 | 42.776 | 48.476 | 85.841 | 1.00 | 72.48 | B | C |
| ATOM | 3538 | OG1 | THR | B | 152 | 41.689 | 48.152 | 86.722 | 1.00 | 72.48 | B | O |
| ATOM | 3539 | CG2 | THR | B | 152 | 44.063 | 48.753 | 86.665 | 1.00 | 72.48 | B | C |
| ATOM | 3540 | C | THR | B | 152 | 41.724 | 50.651 | 86.020 | 1.00 | 57.34 | B | C |
| ATOM | 3541 | O | THR | B | 152 | 42.394 | 51.427 | 86.713 | 1.00 | 57.34 | B | O |
| ATOM | 3542 | N | LEU | B | 153 | 40.402 | 50.573 | 86.083 | 1.00 | 40.88 | B | N |
| ATOM | 3543 | CA | LEU | B | 153 | 39.639 | 51.405 | 86.998 | 1.00 | 40.88 | B | C |
| ATOM | 3544 | CB | LEU | B | 153 | 38.161 | 51.338 | 86.632 | 1.00 | 34.78 | B | C |
| ATOM | 3545 | CG | LEU | B | 153 | 37.184 | 52.023 | 87.580 | 1.00 | 34.78 | B | C |

| | | | | | | | | | | | |
|---|---|---|---|---|---|---|---|---|---|---|---|
| ATOM | 3546 | CD1 | LEU | B | 153 | 37.290 | 53.531 | 87.436 | 1.00 | 34.78 | B | C |
| ATOM | 3547 | CD2 | LEU | B | 153 | 35.774 | 51.554 | 87.255 | 1.00 | 34.78 | B | C |
| ATOM | 3548 | C | LEU | B | 153 | 39.864 | 50.813 | 88.387 | 1.00 | 40.88 | B | C |
| ATOM | 3549 | O | LEU | B | 153 | 39.607 | 49.630 | 88.600 | 1.00 | 40.88 | B | O |
| ATOM | 3550 | N | PRO | B | 154 | 40.380 | 51.614 | 89.336 | 1.00 | 18.95 | B | N |
| ATOM | 3551 | CD | PRO | B | 154 | 40.933 | 52.966 | 89.165 | 1.00 | 22.48 | B | C |
| ATOM | 3552 | CA | PRO | B | 154 | 40.630 | 51.142 | 90.696 | 1.00 | 18.95 | B | C |
| ATOM | 3553 | CB | PRO | B | 154 | 40.963 | 52.425 | 91.438 | 1.00 | 22.48 | B | C |
| ATOM | 3554 | CG | PRO | B | 154 | 41.759 | 53.129 | 90.438 | 1.00 | 22.48 | B | C |
| ATOM | 3555 | C | PRO | B | 154 | 39.408 | 50.419 | 91.235 | 1.00 | 18.95 | B | C |
| ATOM | 3556 | O | PRO | B | 154 | 38.299 | 50.940 | 91.204 | 1.00 | 18.95 | B | O |
| ATOM | 3557 | N | VAL | B | 155 | 39.620 | 49.198 | 91.706 | 1.00 | 30.17 | B | N |
| ATOM | 3558 | CA | VAL | B | 155 | 38.534 | 48.368 | 92.217 | 1.00 | 30.17 | B | C |
| ATOM | 3559 | CB | VAL | B | 155 | 39.082 | 46.994 | 92.757 | 1.00 | 14.30 | B | C |
| ATOM | 3560 | CG1 | VAL | B | 155 | 40.517 | 46.796 | 92.307 | 1.00 | 14.30 | B | C |
| ATOM | 3561 | CG2 | VAL | B | 155 | 38.975 | 46.915 | 94.281 | 1.00 | 14.30 | B | C |
| ATOM | 3562 | C | VAL | B | 155 | 37.683 | 49.037 | 93.302 | 1.00 | 30.17 | B | C |
| ATOM | 3563 | O | VAL | B | 155 | 36.575 | 48.591 | 93.602 | 1.00 | 30.17 | B | O |
| ATOM | 3564 | N | ILE | B | 156 | 38.196 | 50.089 | 93.918 | 1.00 | 24.99 | B | N |
| ATOM | 3565 | CA | ILE | B | 156 | 37.391 | 50.743 | 94.929 | 1.00 | 24.99 | B | C |
| ATOM | 3566 | CB | ILE | B | 156 | 38.169 | 51.904 | 95.611 | 1.00 | 22.87 | B | C |
| ATOM | 3567 | CG2 | ILE | B | 156 | 38.744 | 52.845 | 94.578 | 1.00 | 22.87 | B | C |
| ATOM | 3568 | CG1 | ILE | B | 156 | 37.252 | 52.652 | 96.578 | 1.00 | 22.87 | B | C |
| ATOM | 3569 | CD1 | ILE | B | 156 | 36.659 | 51.771 | 97.664 | 1.00 | 22.87 | B | C |
| ATOM | 3570 | C | ILE | B | 156 | 36.155 | 51.251 | 94.194 | 1.00 | 24.99 | B | C |
| ATOM | 3571 | O | ILE | B | 156 | 35.031 | 50.990 | 94.605 | 1.00 | 24.99 | B | O |
| ATOM | 3572 | N | TYR | B | 157 | 36.380 | 51.950 | 93.087 | 1.00 | 28.38 | B | N |
| ATOM | 3573 | CA | TYR | B | 157 | 35.306 | 52.498 | 92.267 | 1.00 | 28.38 | B | C |
| ATOM | 3574 | CB | TYR | B | 157 | 35.885 | 53.305 | 91.104 | 1.00 | 35.68 | B | C |
| ATOM | 3575 | CG | TYR | B | 157 | 36.488 | 54.624 | 91.528 | 1.00 | 35.68 | B | C |
| ATOM | 3576 | CD1 | TYR | B | 157 | 35.796 | 55.474 | 92.381 | 1.00 | 35.68 | B | C |
| ATOM | 3577 | CE1 | TYR | B | 157 | 36.319 | 56.690 | 92.764 | 1.00 | 35.68 | B | C |
| ATOM | 3578 | CD2 | TYR | B | 157 | 37.735 | 55.034 | 91.062 | 1.00 | 35.68 | B | C |
| ATOM | 3579 | CE2 | TYR | B | 157 | 38.269 | 56.260 | 91.440 | 1.00 | 35.68 | B | C |
| ATOM | 3580 | CZ | TYR | B | 157 | 37.552 | 57.084 | 92.293 | 1.00 | 35.68 | B | C |
| ATOM | 3581 | OH | TYR | B | 157 | 38.060 | 58.308 | 92.675 | 1.00 | 35.68 | B | O |
| ATOM | 3582 | C | TYR | B | 157 | 34.410 | 51.405 | 91.727 | 1.00 | 28.38 | B | C |
| ATOM | 3583 | O | TYR | B | 157 | 33.193 | 51.553 | 91.714 | 1.00 | 28.38 | B | O |
| ATOM | 3584 | N | VAL | B | 158 | 35.020 | 50.322 | 91.257 | 1.00 | 26.28 | B | N |
| ATOM | 3585 | CA | VAL | B | 158 | 34.273 | 49.181 | 90.734 | 1.00 | 26.28 | B | C |
| ATOM | 3586 | CB | VAL | B | 158 | 35.225 | 48.071 | 90.263 | 1.00 | 12.86 | B | C |
| ATOM | 3587 | CG1 | VAL | B | 158 | 34.441 | 46.874 | 89.767 | 1.00 | 12.86 | B | C |
| ATOM | 3588 | CG2 | VAL | B | 158 | 36.115 | 48.620 | 89.173 | 1.00 | 12.86 | B | C |
| ATOM | 3589 | C | VAL | B | 158 | 33.350 | 48.642 | 91.819 | 1.00 | 26.28 | B | C |
| ATOM | 3590 | O | VAL | B | 158 | 32.275 | 48.131 | 91.537 | 1.00 | 26.28 | B | O |
| ATOM | 3591 | N | LYS | B | 159 | 33.781 | 48.770 | 93.063 | 1.00 | 26.23 | B | N |
| ATOM | 3592 | CA | LYS | B | 159 | 32.985 | 48.338 | 94.201 | 1.00 | 26.23 | B | C |
| ATOM | 3593 | CB | LYS | B | 159 | 33.847 | 48.340 | 95.474 | 1.00 | 32.64 | B | C |
| ATOM | 3594 | CG | LYS | B | 159 | 34.641 | 47.070 | 95.778 | 1.00 | 32.64 | B | C |
| ATOM | 3595 | CD | LYS | B | 159 | 35.284 | 47.208 | 97.142 | 1.00 | 32.64 | B | C |
| ATOM | 3596 | CE | LYS | B | 159 | 36.028 | 45.964 | 97.590 | 1.00 | 32.64 | B | C |
| ATOM | 3597 | NZ | LYS | B | 159 | 37.441 | 45.937 | 97.186 | 1.00 | 32.64 | B | N |
| ATOM | 3598 | C | LYS | B | 159 | 31.821 | 49.329 | 94.389 | 1.00 | 26.23 | B | C |
| ATOM | 3599 | O | LYS | B | 159 | 30.654 | 48.945 | 94.456 | 1.00 | 26.23 | B | O |
| ATOM | 3600 | N | LEU | B | 160 | 32.156 | 50.610 | 94.488 | 1.00 | 26.95 | B | N |
| ATOM | 3601 | CA | LEU | B | 160 | 31.153 | 51.641 | 94.692 | 1.00 | 26.95 | B | C |
| ATOM | 3602 | CB | LEU | B | 160 | 31.814 | 53.009 | 94.835 | 1.00 | 38.88 | B | C |
| ATOM | 3603 | CG | LEU | B | 160 | 32.424 | 53.341 | 96.197 | 1.00 | 38.88 | B | C |
| ATOM | 3604 | CD1 | LEU | B | 160 | 33.090 | 54.690 | 96.116 | 1.00 | 38.88 | B | C |
| ATOM | 3605 | CD2 | LEU | B | 160 | 31.356 | 53.345 | 97.273 | 1.00 | 38.88 | B | C |
| ATOM | 3606 | C | LEU | B | 160 | 30.110 | 51.711 | 93.592 | 1.00 | 26.95 | B | C |
| ATOM | 3607 | O | LEU | B | 160 | 28.933 | 51.948 | 93.850 | 1.00 | 26.95 | B | O |
| ATOM | 3608 | N | TYR | B | 161 | 30.545 | 51.523 | 92.357 | 1.00 | 22.18 | B | N |
| ATOM | 3609 | CA | TYR | B | 161 | 29.637 | 51.577 | 91.230 | 1.00 | 22.18 | B | C |
| ATOM | 3610 | CB | TYR | B | 161 | 30.451 | 51.717 | 89.948 | 1.00 | 37.20 | B | C |
| ATOM | 3611 | CG | TYR | B | 161 | 31.244 | 52.996 | 89.887 | 1.00 | 37.20 | B | C |
| ATOM | 3612 | CD1 | TYR | B | 161 | 30.942 | 54.053 | 90.739 | 1.00 | 37.20 | B | C |

| | | | | | | | | | | | | |
|---|---|---|---|---|---|---|---|---|---|---|---|---|
| ATOM | 3613 | CE1 | TYR | B | 161 | 31.640 | 55.249 | 90.676 | 1.00 | 37.20 | B | C |
| ATOM | 3614 | CD2 | TYR | B | 161 | 32.278 | 53.164 | 88.956 | 1.00 | 37.20 | B | C |
| ATOM | 3615 | CE2 | TYR | B | 161 | 32.992 | 54.363 | 88.876 | 1.00 | 37.20 | B | C |
| ATOM | 3616 | CZ | TYR | B | 161 | 32.664 | 55.405 | 89.743 | 1.00 | 37.20 | B | C |
| ATOM | 3617 | OH | TYR | B | 161 | 33.326 | 56.616 | 89.678 | 1.00 | 37.20 | B | O |
| ATOM | 3618 | C | TYR | B | 161 | 28.728 | 50.345 | 91.142 | 1.00 | 22.18 | B | C |
| ATOM | 3619 | O | TYR | B | 161 | 27.517 | 50.464 | 90.942 | 1.00 | 22.18 | B | O |
| ATOM | 3620 | N | MET | B | 162 | 29.322 | 49.162 | 91.290 | 1.00 | 22.94 | B | N |
| ATOM | 3621 | CA | MET | B | 162 | 28.573 | 47.909 | 91.210 | 1.00 | 22.94 | B | C |
| ATOM | 3622 | CB | MET | B | 162 | 29.517 | 46.706 | 91.224 | 1.00 | 19.71 | B | C |
| ATOM | 3623 | CG | MET | B | 162 | 30.210 | 46.445 | 89.914 | 1.00 | 19.71 | B | C |
| ATOM | 3624 | SD | MET | B | 162 | 29.114 | 46.564 | 88.510 | 1.00 | 19.71 | B | S |
| ATOM | 3625 | CE | MET | B | 162 | 28.023 | 45.193 | 88.756 | 1.00 | 19.71 | B | C |
| ATOM | 3626 | C | MET | B | 162 | 27.559 | 47.731 | 92.327 | 1.00 | 22.94 | B | C |
| ATOM | 3627 | O | MET | B | 162 | 26.451 | 47.255 | 92.095 | 1.00 | 22.94 | B | O |
| ATOM | 3628 | N | TYR | B | 163 | 27.942 | 48.104 | 93.541 | 1.00 | 27.38 | B | N |
| ATOM | 3629 | CA | TYR | B | 163 | 27.040 | 47.984 | 94.682 | 1.00 | 27.38 | B | C |
| ATOM | 3630 | CB | TYR | B | 163 | 27.756 | 48.383 | 95.985 | 1.00 | 18.22 | B | C |
| ATOM | 3631 | CG | TYR | B | 163 | 26.890 | 48.416 | 97.241 | 1.00 | 18.22 | B | C |
| ATOM | 3632 | CD1 | TYR | B | 163 | 26.697 | 47.286 | 98.011 | 1.00 | 18.22 | B | C |
| ATOM | 3633 | CE1 | TYR | B | 163 | 25.944 | 47.345 | 99.174 | 1.00 | 18.22 | B | C |
| ATOM | 3634 | CD2 | TYR | B | 163 | 26.296 | 49.606 | 97.669 | 1.00 | 18.22 | B | C |
| ATOM | 3635 | CE2 | TYR | B | 163 | 25.538 | 49.672 | 98.826 | 1.00 | 18.22 | B | C |
| ATOM | 3636 | CZ | TYR | B | 163 | 25.367 | 48.546 | 99.579 | 1.00 | 18.22 | B | C |
| ATOM | 3637 | OH | TYR | B | 163 | 24.646 | 48.633 | 100.752 | 1.00 | 18.22 | B | O |
| ATOM | 3638 | C | TYR | B | 163 | 25.818 | 48.872 | 94.458 | 1.00 | 27.38 | B | C |
| ATOM | 3639 | O | TYR | B | 163 | 24.684 | 48.437 | 94.617 | 1.00 | 27.38 | B | O |
| ATOM | 3640 | N | GLN | B | 164 | 26.056 | 50.123 | 94.083 | 1.00 | 33.04 | B | N |
| ATOM | 3641 | CA | GLN | B | 164 | 24.966 | 51.048 | 93.839 | 1.00 | 33.04 | B | C |
| ATOM | 3642 | CB | GLN | B | 164 | 25.524 | 52.435 | 93.535 | 1.00 | 22.81 | B | C |
| ATOM | 3643 | CG | GLN | B | 164 | 26.350 | 53.016 | 94.651 | 1.00 | 22.81 | B | C |
| ATOM | 3644 | CD | GLN | B | 164 | 26.713 | 54.468 | 94.411 | 1.00 | 22.81 | B | C |
| ATOM | 3645 | OE1 | GLN | B | 164 | 25.867 | 55.359 | 94.469 | 1.00 | 22.81 | B | O |
| ATOM | 3646 | NE2 | GLN | B | 164 | 27.979 | 54.709 | 94.136 | 1.00 | 22.81 | B | N |
| ATOM | 3647 | C | GLN | B | 164 | 24.082 | 50.568 | 92.681 | 1.00 | 33.04 | B | C |
| ATOM | 3648 | O | GLN | B | 164 | 22.879 | 50.846 | 92.646 | 1.00 | 33.04 | B | O |
| ATOM | 3649 | N | LEU | B | 165 | 24.670 | 49.848 | 91.731 | 1.00 | 33.23 | B | N |
| ATOM | 3650 | CA | LEU | B | 165 | 23.880 | 49.357 | 90.614 | 1.00 | 33.23 | B | C |
| ATOM | 3651 | CB | LEU | B | 165 | 24.750 | 48.651 | 89.570 | 1.00 | 11.12 | B | C |
| ATOM | 3652 | CG | LEU | B | 165 | 24.279 | 48.669 | 88.106 | 1.00 | 11.12 | B | C |
| ATOM | 3653 | CD1 | LEU | B | 165 | 24.998 | 47.571 | 87.364 | 1.00 | 11.12 | B | C |
| ATOM | 3654 | CD2 | LEU | B | 165 | 22.776 | 48.508 | 87.987 | 1.00 | 11.12 | B | C |
| ATOM | 3655 | C | LEU | B | 165 | 22.923 | 48.344 | 91.208 | 1.00 | 33.23 | B | C |
| ATOM | 3656 | O | LEU | B | 165 | 21.717 | 48.440 | 91.031 | 1.00 | 33.23 | B | O |
| ATOM | 3657 | N | PHE | B | 166 | 23.464 | 47.368 | 91.923 | 1.00 | 22.14 | B | N |
| ATOM | 3658 | CA | PHE | B | 166 | 22.628 | 46.346 | 92.532 | 1.00 | 22.14 | B | C |
| ATOM | 3659 | CB | PHE | B | 166 | 23.484 | 45.341 | 93.318 | 1.00 | 13.23 | B | C |
| ATOM | 3660 | CG | PHE | B | 166 | 24.256 | 44.392 | 92.450 | 1.00 | 13.23 | B | C |
| ATOM | 3661 | CD1 | PHE | B | 166 | 23.594 | 43.562 | 91.539 | 1.00 | 13.23 | B | C |
| ATOM | 3662 | CD2 | PHE | B | 166 | 25.645 | 44.360 | 92.502 | 1.00 | 13.23 | B | C |
| ATOM | 3663 | CE1 | PHE | B | 166 | 24.307 | 42.718 | 90.686 | 1.00 | 13.23 | B | C |
| ATOM | 3664 | CE2 | PHE | B | 166 | 26.366 | 43.529 | 91.664 | 1.00 | 13.23 | B | C |
| ATOM | 3665 | CZ | PHE | B | 166 | 25.695 | 42.703 | 90.748 | 1.00 | 13.23 | B | C |
| ATOM | 3666 | C | PHE | B | 166 | 21.536 | 46.924 | 93.446 | 1.00 | 22.14 | B | C |
| ATOM | 3667 | O | PHE | B | 166 | 20.453 | 46.367 | 93.541 | 1.00 | 22.14 | B | O |
| ATOM | 3668 | N | ARG | B | 167 | 21.815 | 48.027 | 94.126 | 1.00 | 23.66 | B | N |
| ATOM | 3669 | CA | ARG | B | 167 | 20.821 | 48.628 | 95.008 | 1.00 | 23.66 | B | C |
| ATOM | 3670 | CB | ARG | B | 167 | 21.433 | 49.757 | 95.836 | 1.00 | 22.23 | B | C |
| ATOM | 3671 | CG | ARG | B | 167 | 22.082 | 49.315 | 97.146 | 1.00 | 22.23 | B | C |
| ATOM | 3672 | CD | ARG | B | 167 | 21.367 | 49.935 | 98.317 | 1.00 | 22.23 | B | C |
| ATOM | 3673 | NE | ARG | B | 167 | 21.720 | 51.334 | 98.498 | 1.00 | 22.23 | B | N |
| ATOM | 3674 | CZ | ARG | B | 167 | 20.918 | 52.228 | 99.062 | 1.00 | 22.23 | B | C |
| ATOM | 3675 | NH1 | ARG | B | 167 | 19.721 | 51.871 | 99.493 | 1.00 | 22.23 | B | N |
| ATOM | 3676 | NH2 | ARG | B | 167 | 21.312 | 53.480 | 99.202 | 1.00 | 22.23 | B | N |
| ATOM | 3677 | C | ARG | B | 167 | 19.679 | 49.192 | 94.189 | 1.00 | 23.66 | B | C |
| ATOM | 3678 | O | ARG | B | 167 | 18.537 | 49.187 | 94.623 | 1.00 | 23.66 | B | O |
| ATOM | 3679 | N | SER | B | 168 | 19.994 | 49.699 | 93.006 | 1.00 | 28.83 | B | N |

| | | | | | | | | | | | | |
|---|---|---|---|---|---|---|---|---|---|---|---|---|
| ATOM | 3680 | CA | SER | B | 168 | 18.976 | 50.255 | 92.129 | 1.00 | 28.83 | B | C |
| ATOM | 3681 | CB | SER | B | 168 | 19.636 | 51.049 | 91.007 | 1.00 | 14.03 | B | C |
| ATOM | 3682 | OG | SER | B | 168 | 20.062 | 50.187 | 89.964 | 1.00 | 14.03 | B | O |
| ATOM | 3683 | C | SER | B | 168 | 18.174 | 49.107 | 91.514 | 1.00 | 28.83 | B | C |
| ATOM | 3684 | O | SER | B | 168 | 16.980 | 49.229 | 91.228 | 1.00 | 28.83 | B | O |
| ATOM | 3685 | N | LEU | B | 169 | 18.854 | 47.992 | 91.289 | 1.00 | 28.00 | B | N |
| ATOM | 3686 | CA | LEU | B | 169 | 18.219 | 46.821 | 90.706 | 1.00 | 28.00 | B | C |
| ATOM | 3687 | CB | LEU | B | 169 | 19.283 | 45.854 | 90.190 | 1.00 | 11.78 | B | C |
| ATOM | 3688 | CG | LEU | B | 169 | 19.467 | 45.762 | 88.675 | 1.00 | 11.78 | B | C |
| ATOM | 3689 | CD1 | LEU | B | 169 | 19.192 | 47.069 | 88.016 | 1.00 | 11.78 | B | C |
| ATOM | 3690 | CD2 | LEU | B | 169 | 20.855 | 45.300 | 88.384 | 1.00 | 11.78 | B | C |
| ATOM | 3691 | C | LEU | B | 169 | 17.342 | 46.134 | 91.740 | 1.00 | 28.00 | B | C |
| ATOM | 3692 | O | LEU | B | 169 | 16.272 | 45.638 | 91.422 | 1.00 | 28.00 | B | O |
| ATOM | 3693 | N | ALA | B | 170 | 17.797 | 46.106 | 92.986 | 1.00 | 23.73 | B | N |
| ATOM | 3694 | CA | ALA | B | 170 | 17.023 | 45.493 | 94.058 | 1.00 | 23.73 | B | C |
| ATOM | 3695 | CB | ALA | B | 170 | 17.851 | 45.471 | 95.335 | 1.00 | 14.07 | B | C |
| ATOM | 3696 | C | ALA | B | 170 | 15.737 | 46.292 | 94.275 | 1.00 | 23.73 | B | C |
| ATOM | 3697 | O | ALA | B | 170 | 14.701 | 45.750 | 94.669 | 1.00 | 23.73 | B | O |
| ATOM | 3698 | N | TYR | B | 171 | 15.824 | 47.591 | 94.010 | 1.00 | 28.49 | B | N |
| ATOM | 3699 | CA | TYR | B | 171 | 14.693 | 48.489 | 94.156 | 1.00 | 28.49 | B | C |
| ATOM | 3700 | CB | TYR | B | 171 | 15.153 | 49.943 | 94.102 | 1.00 | 15.64 | B | C |
| ATOM | 3701 | CG | TYR | B | 171 | 14.022 | 50.913 | 94.284 | 1.00 | 15.64 | B | C |
| ATOM | 3702 | CD1 | TYR | B | 171 | 13.410 | 51.061 | 95.515 | 1.00 | 15.64 | B | C |
| ATOM | 3703 | CE1 | TYR | B | 171 | 12.343 | 51.926 | 95.690 | 1.00 | 15.64 | B | C |
| ATOM | 3704 | CD2 | TYR | B | 171 | 13.537 | 51.659 | 93.222 | 1.00 | 15.64 | B | C |
| ATOM | 3705 | CE2 | TYR | B | 171 | 12.464 | 52.529 | 93.389 | 1.00 | 15.64 | B | C |
| ATOM | 3706 | CZ | TYR | B | 171 | 11.874 | 52.657 | 94.628 | 1.00 | 15.64 | B | C |
| ATOM | 3707 | OH | TYR | B | 171 | 10.823 | 53.520 | 94.808 | 1.00 | 15.64 | B | O |
| ATOM | 3708 | C | TYR | B | 171 | 13.646 | 48.258 | 93.071 | 1.00 | 28.49 | B | C |
| ATOM | 3709 | O | TYR | B | 171 | 12.535 | 47.853 | 93.363 | 1.00 | 28.49 | B | O |
| ATOM | 3710 | N | ILE | B | 172 | 13.991 | 48.506 | 91.816 | 1.00 | 24.30 | B | N |
| ATOM | 3711 | CA | ILE | B | 172 | 13.006 | 48.330 | 90.759 | 1.00 | 24.30 | B | C |
| ATOM | 3712 | CB | ILE | B | 172 | 13.552 | 48.716 | 89.356 | 1.00 | 18.18 | B | C |
| ATOM | 3713 | CG2 | ILE | B | 172 | 13.875 | 50.207 | 89.306 | 1.00 | 18.18 | B | C |
| ATOM | 3714 | CG1 | ILE | B | 172 | 14.763 | 47.856 | 89.001 | 1.00 | 18.18 | B | C |
| ATOM | 3715 | CD1 | ILE | B | 172 | 15.045 | 47.809 | 87.510 | 1.00 | 18.18 | B | C |
| ATOM | 3716 | C | ILE | B | 172 | 12.485 | 46.904 | 90.692 | 1.00 | 24.30 | B | C |
| ATOM | 3717 | O | ILE | B | 172 | 11.341 | 46.677 | 90.346 | 1.00 | 24.30 | B | O |
| ATOM | 3718 | N | HIS | B | 173 | 13.319 | 45.933 | 91.010 | 1.00 | 32.78 | B | N |
| ATOM | 3719 | CA | HIS | B | 173 | 12.856 | 44.562 | 90.977 | 1.00 | 32.78 | B | C |
| ATOM | 3720 | CB | HIS | B | 173 | 14.023 | 43.596 | 91.184 | 1.00 | 11.26 | B | C |
| ATOM | 3721 | CG | HIS | B | 173 | 14.889 | 43.425 | 89.977 | 1.00 | 11.26 | B | C |
| ATOM | 3722 | CD2 | HIS | B | 173 | 14.973 | 44.143 | 88.833 | 1.00 | 11.26 | B | C |
| ATOM | 3723 | ND1 | HIS | B | 173 | 15.848 | 42.439 | 89.881 | 1.00 | 11.26 | B | N |
| ATOM | 3724 | CE1 | HIS | B | 173 | 16.489 | 42.565 | 88.732 | 1.00 | 11.26 | B | C |
| ATOM | 3725 | NE2 | HIS | B | 173 | 15.977 | 43.590 | 88.078 | 1.00 | 11.26 | B | N |
| ATOM | 3726 | C | HIS | B | 173 | 11.807 | 44.313 | 92.055 | 1.00 | 32.78 | B | C |
| ATOM | 3727 | O | HIS | B | 173 | 10.956 | 43.441 | 91.900 | 1.00 | 32.78 | B | O |
| ATOM | 3728 | N | SER | B | 174 | 11.868 | 45.072 | 93.146 | 1.00 | 25.19 | B | N |
| ATOM | 3729 | CA | SER | B | 174 | 10.936 | 44.879 | 94.238 | 1.00 | 25.19 | B | C |
| ATOM | 3730 | CB | SER | B | 174 | 11.301 | 45.759 | 95.440 | 1.00 | 28.98 | B | C |
| ATOM | 3731 | OG | SER | B | 174 | 10.738 | 47.053 | 95.349 | 1.00 | 28.98 | B | O |
| ATOM | 3732 | C | SER | B | 174 | 9.515 | 45.141 | 93.798 | 1.00 | 25.19 | B | C |
| ATOM | 3733 | O | SER | B | 174 | 8.586 | 44.535 | 94.324 | 1.00 | 25.19 | B | O |
| ATOM | 3734 | N | PHE | B | 175 | 9.351 | 46.033 | 92.827 | 1.00 | 21.85 | B | N |
| ATOM | 3735 | CA | PHE | B | 175 | 8.035 | 46.364 | 92.313 | 1.00 | 21.85 | B | C |
| ATOM | 3736 | CB | PHE | B | 175 | 7.991 | 47.780 | 91.770 | 1.00 | 40.28 | B | C |
| ATOM | 3737 | CG | PHE | B | 175 | 8.221 | 48.820 | 92.799 | 1.00 | 40.28 | B | C |
| ATOM | 3738 | CD1 | PHE | B | 175 | 9.508 | 49.153 | 93.183 | 1.00 | 40.28 | B | C |
| ATOM | 3739 | CD2 | PHE | B | 175 | 7.143 | 49.481 | 93.383 | 1.00 | 40.28 | B | C |
| ATOM | 3740 | CE1 | PHE | B | 175 | 9.722 | 50.131 | 94.137 | 1.00 | 40.28 | B | C |
| ATOM | 3741 | CE2 | PHE | B | 175 | 7.337 | 50.469 | 94.349 | 1.00 | 40.28 | B | C |
| ATOM | 3742 | CZ | PHE | B | 175 | 8.628 | 50.800 | 94.727 | 1.00 | 40.28 | B | C |
| ATOM | 3743 | C | PHE | B | 175 | 7.735 | 45.412 | 91.183 | 1.00 | 21.85 | B | C |
| ATOM | 3744 | O | PHE | B | 175 | 6.683 | 45.504 | 90.555 | 1.00 | 21.85 | B | O |
| ATOM | 3745 | N | GLY | B | 176 | 8.677 | 44.514 | 90.910 | 1.00 | 25.12 | B | N |
| ATOM | 3746 | CA | GLY | B | 176 | 8.505 | 43.554 | 89.834 | 1.00 | 25.12 | B | C |

| | | | | | | | | | | | | |
|---|---|---|---|---|---|---|---|---|---|---|---|---|
| ATOM | 3747 | C | GLY | B | 176 | 8.855 | 44.093 | 88.453 | 1.00 | 25.12 | B | C |
| ATOM | 3748 | O | GLY | B | 176 | 8.510 | 43.491 | 87.431 | 1.00 | 25.12 | B | O |
| ATOM | 3749 | N | ILE | B | 177 | 9.544 | 45.231 | 88.421 | 1.00 | 25.99 | B | N |
| ATOM | 3750 | CA | ILE | B | 177 | 9.941 | 45.875 | 87.171 | 1.00 | 25.99 | B | C |
| ATOM | 3751 | CB | ILE | B | 177 | 9.895 | 47.401 | 87.281 | 1.00 | 13.91 | B | C |
| ATOM | 3752 | CG2 | ILE | B | 177 | 10.441 | 48.024 | 86.012 | 1.00 | 13.91 | B | C |
| ATOM | 3753 | CG1 | ILE | B | 177 | 8.464 | 47.850 | 87.552 | 1.00 | 13.91 | B | C |
| ATOM | 3754 | CD1 | ILE | B | 177 | 8.269 | 49.330 | 87.475 | 1.00 | 13.91 | B | C |
| ATOM | 3755 | C | ILE | B | 177 | 11.347 | 45.494 | 86.731 | 1.00 | 25.99 | B | C |
| ATOM | 3756 | O | ILE | B | 177 | 12.261 | 45.442 | 87.539 | 1.00 | 25.99 | B | O |
| ATOM | 3757 | N | CYS | B | 178 | 11.519 | 45.239 | 85.439 | 1.00 | 26.49 | B | N |
| ATOM | 3758 | CA | CYS | B | 178 | 12.827 | 44.877 | 84.913 | 1.00 | 26.49 | B | C |
| ATOM | 3759 | CB | CYS | B | 178 | 12.735 | 43.575 | 84.122 | 1.00 | 31.42 | B | C |
| ATOM | 3760 | SG | CYS | B | 178 | 14.325 | 42.818 | 83.657 | 1.00 | 31.42 | B | S |
| ATOM | 3761 | C | CYS | B | 178 | 13.305 | 45.995 | 84.008 | 1.00 | 26.49 | B | C |
| ATOM | 3762 | O | CYS | B | 178 | 12.540 | 46.516 | 83.205 | 1.00 | 26.49 | B | O |
| ATOM | 3763 | N | HIS | B | 179 | 14.572 | 46.367 | 84.133 | 1.00 | 31.89 | B | N |
| ATOM | 3764 | CA | HIS | B | 179 | 15.108 | 47.454 | 83.322 | 1.00 | 31.89 | B | C |
| ATOM | 3765 | CB | HIS | B | 179 | 16.458 | 47.919 | 83.871 | 1.00 | 20.47 | B | C |
| ATOM | 3766 | CG | HIS | B | 179 | 17.022 | 49.112 | 83.163 | 1.00 | 20.47 | B | C |
| ATOM | 3767 | CD2 | HIS | B | 179 | 17.100 | 50.412 | 83.537 | 1.00 | 20.47 | B | C |
| ATOM | 3768 | ND1 | HIS | B | 179 | 17.558 | 49.047 | 81.893 | 1.00 | 20.47 | B | N |
| ATOM | 3769 | CE1 | HIS | B | 179 | 17.939 | 50.255 | 81.517 | 1.00 | 20.47 | B | C |
| ATOM | 3770 | NE2 | HIS | B | 179 | 17.671 | 51.102 | 82.496 | 1.00 | 20.47 | B | N |
| ATOM | 3771 | C | HIS | B | 179 | 15.266 | 47.011 | 81.881 | 1.00 | 31.89 | B | C |
| ATOM | 3772 | O | HIS | B | 179 | 15.034 | 47.786 | 80.956 | 1.00 | 31.89 | B | O |
| ATOM | 3773 | N | ARG | B | 180 | 15.673 | 45.760 | 81.703 | 1.00 | 24.43 | B | N |
| ATOM | 3774 | CA | ARG | B | 180 | 15.861 | 45.172 | 80.377 | 1.00 | 24.43 | B | C |
| ATOM | 3775 | CB | ARG | B | 180 | 14.553 | 45.205 | 79.608 | 1.00 | 37.66 | B | C |
| ATOM | 3776 | CG | ARG | B | 180 | 13.520 | 44.225 | 80.065 | 1.00 | 37.66 | B | C |
| ATOM | 3777 | CD | ARG | B | 180 | 12.181 | 44.818 | 79.769 | 1.00 | 37.66 | B | C |
| ATOM | 3778 | NE | ARG | B | 180 | 11.291 | 43.892 | 79.092 | 1.00 | 37.66 | B | N |
| ATOM | 3779 | CZ | ARG | B | 180 | 10.172 | 44.275 | 78.500 | 1.00 | 37.66 | B | C |
| ATOM | 3780 | NH1 | ARG | B | 180 | 9.848 | 45.562 | 78.498 | 1.00 | 37.66 | B | N |
| ATOM | 3781 | NH2 | ARG | B | 180 | 9.346 | 43.374 | 77.978 | 1.00 | 37.66 | B | N |
| ATOM | 3782 | C | ARG | B | 180 | 16.962 | 45.775 | 79.481 | 1.00 | 24.43 | B | C |
| ATOM | 3783 | O | ARG | B | 180 | 17.109 | 45.390 | 78.316 | 1.00 | 24.43 | B | O |
| ATOM | 3784 | N | ASP | B | 181 | 17.728 | 46.721 | 80.008 | 1.00 | 27.62 | B | N |
| ATOM | 3785 | CA | ASP | B | 181 | 18.791 | 47.310 | 79.211 | 1.00 | 27.62 | B | C |
| ATOM | 3786 | CB | ASP | B | 181 | 18.199 | 48.356 | 78.261 | 1.00 | 24.31 | B | C |
| ATOM | 3787 | CG | ASP | B | 181 | 19.169 | 48.789 | 77.175 | 1.00 | 24.31 | B | C |
| ATOM | 3788 | OD1 | ASP | B | 181 | 19.871 | 47.919 | 76.615 | 1.00 | 24.31 | B | O |
| ATOM | 3789 | OD2 | ASP | B | 181 | 19.213 | 50.000 | 76.869 | 1.00 | 24.31 | B | O |
| ATOM | 3790 | C | ASP | B | 181 | 19.934 | 47.893 | 80.054 | 1.00 | 27.62 | B | C |
| ATOM | 3791 | O | ASP | B | 181 | 20.351 | 49.037 | 79.893 | 1.00 | 27.62 | B | O |
| ATOM | 3792 | N | ILE | B | 182 | 20.451 | 47.062 | 80.947 | 1.00 | 29.58 | B | N |
| ATOM | 3793 | CA | ILE | B | 182 | 21.541 | 47.459 | 81.812 | 1.00 | 29.58 | B | C |
| ATOM | 3794 | CB | ILE | B | 182 | 21.618 | 46.555 | 83.073 | 1.00 | 11.86 | B | C |
| ATOM | 3795 | CG2 | ILE | B | 182 | 22.680 | 47.073 | 84.010 | 1.00 | 11.86 | B | C |
| ATOM | 3796 | CG1 | ILE | B | 182 | 20.269 | 46.516 | 83.793 | 1.00 | 11.86 | B | C |
| ATOM | 3797 | CD1 | ILE | B | 182 | 19.870 | 47.818 | 84.419 | 1.00 | 11.86 | B | C |
| ATOM | 3798 | C | ILE | B | 182 | 22.876 | 47.374 | 81.085 | 1.00 | 29.58 | B | C |
| ATOM | 3799 | O | ILE | B | 182 | 23.388 | 46.287 | 80.842 | 1.00 | 29.58 | B | O |
| ATOM | 3800 | N | LYS | B | 183 | 23.430 | 48.527 | 80.738 | 1.00 | 21.55 | B | N |
| ATOM | 3801 | CA | LYS | B | 183 | 24.724 | 48.603 | 80.083 | 1.00 | 21.55 | B | C |
| ATOM | 3802 | CB | LYS | B | 183 | 24.543 | 48.691 | 78.566 | 1.00 | 14.71 | B | C |
| ATOM | 3803 | CG | LYS | B | 183 | 23.632 | 49.803 | 78.118 | 1.00 | 14.71 | B | C |
| ATOM | 3804 | CD | LYS | B | 183 | 23.434 | 49.788 | 76.623 | 1.00 | 14.71 | B | C |
| ATOM | 3805 | CE | LYS | B | 183 | 22.499 | 50.901 | 76.212 | 1.00 | 14.71 | B | C |
| ATOM | 3806 | NZ | LYS | B | 183 | 22.366 | 50.998 | 74.751 | 1.00 | 14.71 | B | N |
| ATOM | 3807 | C | LYS | B | 183 | 25.422 | 49.853 | 80.636 | 1.00 | 21.55 | B | C |
| ATOM | 3808 | O | LYS | B | 183 | 24.763 | 50.760 | 81.148 | 1.00 | 21.55 | B | O |
| ATOM | 3809 | N | PRO | B | 184 | 26.764 | 49.922 | 80.539 | 1.00 | 24.90 | B | N |
| ATOM | 3810 | CD | PRO | B | 184 | 27.668 | 48.940 | 79.917 | 1.00 | 18.30 | B | C |
| ATOM | 3811 | CA | PRO | B | 184 | 27.533 | 51.059 | 81.038 | 1.00 | 24.90 | B | C |
| ATOM | 3812 | CB | PRO | B | 184 | 28.921 | 50.779 | 80.502 | 1.00 | 18.30 | B | C |
| ATOM | 3813 | CG | PRO | B | 184 | 28.972 | 49.302 | 80.513 | 1.00 | 18.30 | B | C |

| | | | | | | | | | | | | |
|---|---|---|---|---|---|---|---|---|---|---|---|---|
| ATOM | 3814 | C | PRO | B | 184 | 27.037 | 52.433 | 80.633 | 1.00 | 24.90 | B | C |
| ATOM | 3815 | O | PRO | B | 184 | 27.242 | 53.401 | 81.365 | 1.00 | 24.90 | B | O |
| ATOM | 3816 | N | GLN | B | 185 | 26.397 | 52.523 | 79.471 | 1.00 | 22.32 | B | N |
| ATOM | 3817 | CA | GLN | B | 185 | 25.884 | 53.802 | 79.005 | 1.00 | 22.32 | B | C |
| ATOM | 3818 | CB | GLN | B | 185 | 25.484 | 53.725 | 77.529 | 1.00 | 52.56 | B | C |
| ATOM | 3819 | CG | GLN | B | 185 | 26.641 | 53.413 | 76.585 | 1.00 | 52.56 | B | C |
| ATOM | 3820 | CD | GLN | B | 185 | 26.663 | 51.955 | 76.174 | 1.00 | 52.56 | B | C |
| ATOM | 3821 | OE1 | GLN | B | 185 | 26.075 | 51.589 | 75.153 | 1.00 | 52.56 | B | O |
| ATOM | 3822 | NE2 | GLN | B | 185 | 27.317 | 51.106 | 76.976 | 1.00 | 52.56 | B | N |
| ATOM | 3823 | C | GLN | B | 185 | 24.698 | 54.271 | 79.846 | 1.00 | 22.32 | B | C |
| ATOM | 3824 | O | GLN | B | 185 | 24.411 | 55.463 | 79.901 | 1.00 | 22.32 | B | O |
| ATOM | 3825 | N | ASN | B | 186 | 24.017 | 53.340 | 80.509 | 1.00 | 26.83 | B | N |
| ATOM | 3826 | CA | ASN | B | 186 | 22.878 | 53.698 | 81.345 | 1.00 | 26.83 | B | C |
| ATOM | 3827 | CB | ASN | B | 186 | 21.757 | 52.676 | 81.221 | 1.00 | 25.78 | B | C |
| ATOM | 3828 | CG | ASN | B | 186 | 21.102 | 52.710 | 79.876 | 1.00 | 25.78 | B | C |
| ATOM | 3829 | OD1 | ASN | B | 186 | 21.007 | 53.763 | 79.259 | 1.00 | 25.78 | B | O |
| ATOM | 3830 | ND2 | ASN | B | 186 | 20.629 | 51.565 | 79.413 | 1.00 | 25.78 | B | N |
| ATOM | 3831 | C | ASN | B | 186 | 23.246 | 53.831 | 82.806 | 1.00 | 26.83 | B | C |
| ATOM | 3832 | O | ASN | B | 186 | 22.377 | 53.871 | 83.662 | 1.00 | 26.83 | B | O |
| ATOM | 3833 | N | LEU | B | 187 | 24.533 | 53.878 | 83.095 | 1.00 | 24.76 | B | N |
| ATOM | 3834 | CA | LEU | B | 187 | 24.991 | 54.056 | 84.463 | 1.00 | 24.76 | B | C |
| ATOM | 3835 | CB | LEU | B | 187 | 26.031 | 52.995 | 84.799 | 1.00 | 29.90 | B | C |
| ATOM | 3836 | CG | LEU | B | 187 | 25.613 | 51.678 | 85.451 | 1.00 | 29.90 | B | C |
| ATOM | 3837 | CD1 | LEU | B | 187 | 24.307 | 51.176 | 84.908 | 1.00 | 29.90 | B | C |
| ATOM | 3838 | CD2 | LEU | B | 187 | 26.707 | 50.680 | 85.221 | 1.00 | 29.90 | B | C |
| ATOM | 3839 | C | LEU | B | 187 | 25.606 | 55.456 | 84.615 | 1.00 | 24.76 | B | C |
| ATOM | 3840 | O | LEU | B | 187 | 26.808 | 55.655 | 84.406 | 1.00 | 24.76 | B | O |
| ATOM | 3841 | N | LEU | B | 188 | 24.772 | 56.431 | 84.962 | 1.00 | 20.74 | B | N |
| ATOM | 3842 | CA | LEU | B | 188 | 25.244 | 57.794 | 85.140 | 1.00 | 20.74 | B | C |
| ATOM | 3843 | CB | LEU | B | 188 | 24.073 | 58.767 | 85.157 | 1.00 | 14.15 | B | C |
| ATOM | 3844 | CG | LEU | B | 188 | 23.024 | 58.635 | 84.068 | 1.00 | 14.15 | B | C |
| ATOM | 3845 | CD1 | LEU | B | 188 | 22.085 | 59.808 | 84.173 | 1.00 | 14.15 | B | C |
| ATOM | 3846 | CD2 | LEU | B | 188 | 23.679 | 58.570 | 82.718 | 1.00 | 14.15 | B | C |
| ATOM | 3847 | C | LEU | B | 188 | 25.944 | 57.879 | 86.477 | 1.00 | 20.74 | B | C |
| ATOM | 3848 | O | LEU | B | 188 | 25.568 | 57.187 | 87.411 | 1.00 | 20.74 | B | O |
| ATOM | 3849 | N | LEU | B | 189 | 26.965 | 58.720 | 86.574 | 1.00 | 21.23 | B | N |
| ATOM | 3850 | CA | LEU | B | 189 | 27.668 | 58.885 | 87.839 | 1.00 | 21.23 | B | C |
| ATOM | 3851 | CB | LEU | B | 189 | 28.712 | 57.773 | 88.045 | 1.00 | 29.57 | B | C |
| ATOM | 3852 | CG | LEU | B | 189 | 29.736 | 57.413 | 86.971 | 1.00 | 29.57 | B | C |
| ATOM | 3853 | CD1 | LEU | B | 189 | 30.737 | 58.529 | 86.814 | 1.00 | 29.57 | B | C |
| ATOM | 3854 | CD2 | LEU | B | 189 | 30.430 | 56.130 | 87.359 | 1.00 | 29.57 | B | C |
| ATOM | 3855 | C | LEU | B | 189 | 28.304 | 60.258 | 87.990 | 1.00 | 21.23 | B | C |
| ATOM | 3856 | O | LEU | B | 189 | 28.824 | 60.823 | 87.038 | 1.00 | 21.23 | B | O |
| ATOM | 3857 | N | ASP | B | 190 | 28.226 | 60.792 | 89.204 | 1.00 | 32.78 | B | N |
| ATOM | 3858 | CA | ASP | B | 190 | 28.763 | 62.105 | 89.530 | 1.00 | 32.78 | B | C |
| ATOM | 3859 | CB | ASP | B | 190 | 27.921 | 62.757 | 90.621 | 1.00 | 40.83 | B | C |
| ATOM | 3860 | CG | ASP | B | 190 | 28.500 | 64.064 | 91.094 | 1.00 | 40.83 | B | C |
| ATOM | 3861 | OD1 | ASP | B | 190 | 29.674 | 64.076 | 91.532 | 1.00 | 40.83 | B | O |
| ATOM | 3862 | OD2 | ASP | B | 190 | 27.770 | 65.076 | 91.026 | 1.00 | 40.83 | B | O |
| ATOM | 3863 | C | ASP | B | 190 | 30.183 | 61.929 | 90.035 | 1.00 | 32.78 | B | C |
| ATOM | 3864 | O | ASP | B | 190 | 30.389 | 61.449 | 91.146 | 1.00 | 32.78 | B | O |
| ATOM | 3865 | N | PRO | B | 191 | 31.181 | 62.342 | 89.241 | 1.00 | 38.95 | B | N |
| ATOM | 3866 | CD | PRO | B | 191 | 31.074 | 63.316 | 88.144 | 1.00 | 45.26 | B | C |
| ATOM | 3867 | CA | PRO | B | 191 | 32.579 | 62.200 | 89.652 | 1.00 | 38.95 | B | C |
| ATOM | 3868 | CB | PRO | B | 191 | 33.328 | 63.107 | 88.679 | 1.00 | 45.26 | B | C |
| ATOM | 3869 | CG | PRO | B | 191 | 32.322 | 64.158 | 88.357 | 1.00 | 45.26 | B | C |
| ATOM | 3870 | C | PRO | B | 191 | 32.913 | 62.543 | 91.109 | 1.00 | 38.95 | B | C |
| ATOM | 3871 | O | PRO | B | 191 | 33.519 | 61.738 | 91.826 | 1.00 | 38.95 | B | O |
| ATOM | 3872 | N | ASP | B | 192 | 32.512 | 63.731 | 91.546 | 1.00 | 42.34 | B | N |
| ATOM | 3873 | CA | ASP | B | 192 | 32.830 | 64.194 | 92.897 | 1.00 | 42.34 | B | C |
| ATOM | 3874 | CB | ASP | B | 192 | 32.519 | 65.696 | 93.042 | 1.00 | 54.89 | B | C |
| ATOM | 3875 | CG | ASP | B | 192 | 33.153 | 66.549 | 91.931 | 1.00 | 54.89 | B | C |
| ATOM | 3876 | OD1 | ASP | B | 192 | 34.377 | 66.382 | 91.673 | 1.00 | 54.89 | B | O |
| ATOM | 3877 | OD2 | ASP | B | 192 | 32.418 | 67.384 | 91.328 | 1.00 | 54.89 | B | O |
| ATOM | 3878 | C | ASP | B | 192 | 32.210 | 63.479 | 94.089 | 1.00 | 42.34 | B | C |
| ATOM | 3879 | O | ASP | B | 192 | 32.827 | 63.399 | 95.152 | 1.00 | 42.34 | B | O |
| ATOM | 3880 | N | THR | B | 193 | 30.988 | 62.988 | 93.932 | 1.00 | 23.38 | B | N |

| | | | | | | | | | | | | |
|---|---|---|---|---|---|---|---|---|---|---|---|---|
| ATOM | 3881 | CA | THR | B | 193 | 30.311 | 62.312 | 95.025 | 1.00 | 23.38 | B | C |
| ATOM | 3882 | CB | THR | B | 193 | 28.808 | 62.659 | 95.042 | 1.00 | 22.93 | B | C |
| ATOM | 3883 | OG1 | THR | B | 193 | 28.127 | 61.945 | 94.001 | 1.00 | 22.93 | B | O |
| ATOM | 3884 | CG2 | THR | B | 193 | 28.620 | 64.142 | 94.809 | 1.00 | 22.93 | B | C |
| ATOM | 3885 | C | THR | B | 193 | 30.476 | 60.813 | 94.875 | 1.00 | 23.38 | B | C |
| ATOM | 3886 | O | THR | B | 193 | 30.385 | 60.075 | 95.842 | 1.00 | 23.38 | B | O |
| ATOM | 3887 | N | ALA | B | 194 | 30.728 | 60.373 | 93.648 | 1.00 | 26.23 | B | N |
| ATOM | 3888 | CA | ALA | B | 194 | 30.898 | 58.957 | 93.345 | 1.00 | 26.23 | B | C |
| ATOM | 3889 | CB | ALA | B | 194 | 31.947 | 58.338 | 94.273 | 1.00 | 19.42 | B | C |
| ATOM | 3890 | C | ALA | B | 194 | 29.574 | 58.204 | 93.461 | 1.00 | 26.23 | B | C |
| ATOM | 3891 | O | ALA | B | 194 | 29.541 | 57.018 | 93.747 | 1.00 | 26.23 | B | O |
| ATOM | 3892 | N | VAL | B | 195 | 28.481 | 58.909 | 93.230 | 1.00 | 32.50 | B | N |
| ATOM | 3893 | CA | VAL | B | 195 | 27.156 | 58.320 | 93.295 | 1.00 | 32.50 | B | C |
| ATOM | 3894 | CB | VAL | B | 195 | 26.117 | 59.388 | 93.689 | 1.00 | 22.98 | B | C |
| ATOM | 3895 | CG1 | VAL | B | 195 | 24.724 | 58.806 | 93.666 | 1.00 | 22.98 | B | C |
| ATOM | 3896 | CG2 | VAL | B | 195 | 26.443 | 59.945 | 95.051 | 1.00 | 22.98 | B | C |
| ATOM | 3897 | C | VAL | B | 195 | 26.819 | 57.824 | 91.902 | 1.00 | 32.50 | B | C |
| ATOM | 3898 | O | VAL | B | 195 | 27.130 | 58.484 | 90.906 | 1.00 | 32.50 | B | O |
| ATOM | 3899 | N | LEU | B | 196 | 26.194 | 56.658 | 91.833 | 1.00 | 27.60 | B | N |
| ATOM | 3900 | CA | LEU | B | 196 | 25.795 | 56.097 | 90.556 | 1.00 | 27.60 | B | C |
| ATOM | 3901 | CB | LEU | B | 196 | 26.174 | 54.616 | 90.480 | 1.00 | 26.68 | B | C |
| ATOM | 3902 | CG | LEU | B | 196 | 25.837 | 53.861 | 89.187 | 1.00 | 26.68 | B | C |
| ATOM | 3903 | CD1 | LEU | B | 196 | 26.826 | 52.736 | 89.000 | 1.00 | 26.68 | B | C |
| ATOM | 3904 | CD2 | LEU | B | 196 | 24.419 | 53.326 | 89.216 | 1.00 | 26.68 | B | C |
| ATOM | 3905 | C | LEU | B | 196 | 24.293 | 56.260 | 90.472 | 1.00 | 27.60 | B | C |
| ATOM | 3906 | O | LEU | B | 196 | 23.608 | 56.237 | 91.482 | 1.00 | 27.60 | B | O |
| ATOM | 3907 | N | LYS | B | 197 | 23.789 | 56.457 | 89.264 | 1.00 | 28.47 | B | N |
| ATOM | 3908 | CA | LYS | B | 197 | 22.361 | 56.627 | 89.045 | 1.00 | 28.47 | B | C |
| ATOM | 3909 | CB | LYS | B | 197 | 22.011 | 58.113 | 88.960 | 1.00 | 21.98 | B | C |
| ATOM | 3910 | CG | LYS | B | 197 | 21.970 | 58.752 | 90.310 | 1.00 | 21.98 | B | C |
| ATOM | 3911 | CD | LYS | B | 197 | 21.716 | 60.230 | 90.235 | 1.00 | 21.98 | B | C |
| ATOM | 3912 | CE | LYS | B | 197 | 20.947 | 60.688 | 91.463 | 1.00 | 21.98 | B | C |
| ATOM | 3913 | NZ | LYS | B | 197 | 21.329 | 59.903 | 92.657 | 1.00 | 21.98 | B | N |
| ATOM | 3914 | C | LYS | B | 197 | 21.954 | 55.918 | 87.767 | 1.00 | 28.47 | B | C |
| ATOM | 3915 | O | LYS | B | 197 | 22.519 | 56.169 | 86.705 | 1.00 | 28.47 | B | O |
| ATOM | 3916 | N | LEU | B | 198 | 20.994 | 55.009 | 87.881 | 1.00 | 23.96 | B | N |
| ATOM | 3917 | CA | LEU | B | 198 | 20.519 | 54.276 | 86.728 | 1.00 | 23.96 | B | C |
| ATOM | 3918 | CB | LEU | B | 198 | 19.796 | 53.010 | 87.171 | 1.00 | 19.80 | B | C |
| ATOM | 3919 | CG | LEU | B | 198 | 19.290 | 52.129 | 86.040 | 1.00 | 19.80 | B | C |
| ATOM | 3920 | CD1 | LEU | B | 198 | 20.454 | 51.550 | 85.279 | 1.00 | 19.80 | B | C |
| ATOM | 3921 | CD2 | LEU | B | 198 | 18.442 | 51.044 | 86.620 | 1.00 | 19.80 | B | C |
| ATOM | 3922 | C | LEU | B | 198 | 19.569 | 55.184 | 85.968 | 1.00 | 23.96 | B | C |
| ATOM | 3923 | O | LEU | B | 198 | 18.890 | 56.024 | 86.565 | 1.00 | 23.96 | B | O |
| ATOM | 3924 | N | CYS | B | 199 | 19.543 | 55.022 | 84.651 | 1.00 | 31.58 | B | N |
| ATOM | 3925 | CA | CYS | B | 199 | 18.677 | 55.819 | 83.799 | 1.00 | 31.58 | B | C |
| ATOM | 3926 | CB | CYS | B | 199 | 19.395 | 57.098 | 83.352 | 1.00 | 33.88 | B | C |
| ATOM | 3927 | SG | CYS | B | 199 | 20.792 | 56.837 | 82.269 | 1.00 | 33.88 | B | S |
| ATOM | 3928 | C | CYS | B | 199 | 18.213 | 55.009 | 82.586 | 1.00 | 31.58 | B | C |
| ATOM | 3929 | O | CYS | B | 199 | 18.522 | 53.828 | 82.465 | 1.00 | 31.58 | B | O |
| ATOM | 3930 | N | ASP | B | 200 | 17.459 | 55.656 | 81.708 | 1.00 | 29.06 | B | N |
| ATOM | 3931 | CA | ASP | B | 200 | 16.922 | 55.043 | 80.498 | 1.00 | 29.06 | B | C |
| ATOM | 3932 | CB | ASP | B | 200 | 18.057 | 54.653 | 79.558 | 1.00 | 26.76 | B | C |
| ATOM | 3933 | CG | ASP | B | 200 | 17.565 | 54.306 | 78.162 | 1.00 | 26.76 | B | C |
| ATOM | 3934 | OD1 | ASP | B | 200 | 16.445 | 54.734 | 77.804 | 1.00 | 26.76 | B | O |
| ATOM | 3935 | OD2 | ASP | B | 200 | 18.304 | 53.623 | 77.415 | 1.00 | 26.76 | B | O |
| ATOM | 3936 | C | ASP | B | 200 | 15.995 | 53.846 | 80.708 | 1.00 | 29.06 | B | C |
| ATOM | 3937 | O | ASP | B | 200 | 16.319 | 52.723 | 80.353 | 1.00 | 29.06 | B | O |
| ATOM | 3938 | N | PHE | B | 201 | 14.817 | 54.093 | 81.265 | 1.00 | 38.87 | B | N |
| ATOM | 3939 | CA | PHE | B | 201 | 13.873 | 53.014 | 81.490 | 1.00 | 38.87 | B | C |
| ATOM | 3940 | CB | PHE | B | 201 | 13.046 | 53.303 | 82.738 | 1.00 | 20.42 | B | C |
| ATOM | 3941 | CG | PHE | B | 201 | 13.850 | 53.279 | 83.995 | 1.00 | 20.42 | B | C |
| ATOM | 3942 | CD1 | PHE | B | 201 | 14.758 | 54.283 | 84.266 | 1.00 | 20.42 | B | C |
| ATOM | 3943 | CD2 | PHE | B | 201 | 13.760 | 52.214 | 84.874 | 1.00 | 20.42 | B | C |
| ATOM | 3944 | CE1 | PHE | B | 201 | 15.570 | 54.221 | 85.391 | 1.00 | 20.42 | B | C |
| ATOM | 3945 | CE2 | PHE | B | 201 | 14.575 | 52.147 | 86.005 | 1.00 | 20.42 | B | C |
| ATOM | 3946 | CZ | PHE | B | 201 | 15.478 | 53.151 | 86.260 | 1.00 | 20.42 | B | C |
| ATOM | 3947 | C | PHE | B | 201 | 12.970 | 52.834 | 80.287 | 1.00 | 38.87 | B | C |

| | | | | | | | | | | | | |
|---|---|---|---|---|---|---|---|---|---|---|---|---|
| ATOM | 3948 | O | PHE | B | 201 | 11.888 | 52.247 | 80.385 | 1.00 | 38.87 | B | O |
| ATOM | 3949 | N | GLY | B | 202 | 13.419 | 53.349 | 79.148 | 1.00 | 25.25 | B | N |
| ATOM | 3950 | CA | GLY | B | 202 | 12.633 | 53.247 | 77.937 | 1.00 | 25.25 | B | C |
| ATOM | 3951 | C | GLY | B | 202 | 12.262 | 51.812 | 77.614 | 1.00 | 25.25 | B | C |
| ATOM | 3952 | O | GLY | B | 202 | 11.267 | 51.571 | 76.934 | 1.00 | 25.25 | B | O |
| ATOM | 3953 | N | SER | B | 203 | 13.049 | 50.852 | 78.094 | 1.00 | 42.50 | B | N |
| ATOM | 3954 | CA | SER | B | 203 | 12.763 | 49.442 | 77.829 | 1.00 | 42.50 | B | C |
| ATOM | 3955 | CB | SER | B | 203 | 14.028 | 48.675 | 77.444 | 1.00 | 26.10 | B | C |
| ATOM | 3956 | OG | SER | B | 203 | 14.740 | 49.340 | 76.428 | 1.00 | 26.10 | B | O |
| ATOM | 3957 | C | SER | B | 203 | 12.166 | 48.745 | 79.029 | 1.00 | 42.50 | B | C |
| ATOM | 3958 | O | SER | B | 203 | 11.672 | 47.626 | 78.907 | 1.00 | 42.50 | B | O |
| ATOM | 3959 | N | ALA | B | 204 | 12.225 | 49.393 | 80.187 | 1.00 | 21.17 | B | N |
| ATOM | 3960 | CA | ALA | B | 204 | 11.691 | 48.798 | 81.396 | 1.00 | 21.17 | B | C |
| ATOM | 3961 | CB | ALA | B | 204 | 11.939 | 49.710 | 82.581 | 1.00 | 26.85 | B | C |
| ATOM | 3962 | C | ALA | B | 204 | 10.208 | 48.475 | 81.293 | 1.00 | 21.17 | B | C |
| ATOM | 3963 | O | ALA | B | 204 | 9.424 | 49.225 | 80.712 | 1.00 | 21.17 | B | O |
| ATOM | 3964 | N | LYS | B | 205 | 9.845 | 47.335 | 81.866 | 1.00 | 33.07 | B | N |
| ATOM | 3965 | CA | LYS | B | 205 | 8.471 | 46.860 | 81.902 | 1.00 | 33.07 | B | C |
| ATOM | 3966 | CB | LYS | B | 205 | 8.164 | 46.055 | 80.648 | 1.00 | 28.74 | B | C |
| ATOM | 3967 | CG | LYS | B | 205 | 6.795 | 45.437 | 80.634 | 1.00 | 28.74 | B | C |
| ATOM | 3968 | CD | LYS | B | 205 | 6.509 | 44.707 | 79.332 | 1.00 | 28.74 | B | C |
| ATOM | 3969 | CE | LYS | B | 205 | 5.077 | 44.204 | 79.332 | 1.00 | 28.74 | B | C |
| ATOM | 3970 | NZ | LYS | B | 205 | 4.825 | 43.218 | 78.243 | 1.00 | 28.74 | B | N |
| ATOM | 3971 | C | LYS | B | 205 | 8.244 | 45.972 | 83.121 | 1.00 | 33.07 | B | C |
| ATOM | 3972 | O | LYS | B | 205 | 9.180 | 45.358 | 83.636 | 1.00 | 33.07 | B | O |
| ATOM | 3973 | N | GLN | B | 206 | 7.006 | 45.910 | 83.593 | 1.00 | 33.01 | B | N |
| ATOM | 3974 | CA | GLN | B | 206 | 6.714 | 45.035 | 84.713 | 1.00 | 33.01 | B | C |
| ATOM | 3975 | CB | GLN | B | 206 | 5.382 | 45.378 | 85.360 | 1.00 | 59.99 | B | C |
| ATOM | 3976 | CG | GLN | B | 206 | 5.388 | 46.643 | 86.171 | 1.00 | 59.99 | B | C |
| ATOM | 3977 | CD | GLN | B | 206 | 4.321 | 46.610 | 87.270 | 1.00 | 59.99 | B | C |
| ATOM | 3978 | OE1 | GLN | B | 206 | 4.029 | 47.638 | 87.926 | 1.00 | 59.99 | B | O |
| ATOM | 3979 | NE2 | GLN | B | 206 | 3.735 | 45.417 | 87.483 | 1.00 | 59.99 | B | N |
| ATOM | 3980 | C | GLN | B | 206 | 6.632 | 43.621 | 84.155 | 1.00 | 33.01 | B | C |
| ATOM | 3981 | O | GLN | B | 206 | 5.941 | 43.385 | 83.159 | 1.00 | 33.01 | B | O |
| ATOM | 3982 | N | LEU | B | 207 | 7.340 | 42.682 | 84.772 | 1.00 | 27.17 | B | N |
| ATOM | 3983 | CA | LEU | B | 207 | 7.289 | 41.305 | 84.290 | 1.00 | 27.17 | B | C |
| ATOM | 3984 | CB | LEU | B | 207 | 8.676 | 40.672 | 84.262 | 1.00 | 29.69 | B | C |
| ATOM | 3985 | CG | LEU | B | 207 | 9.827 | 41.256 | 83.442 | 1.00 | 29.69 | B | C |
| ATOM | 3986 | CD1 | LEU | B | 207 | 11.043 | 40.305 | 83.591 | 1.00 | 29.69 | B | C |
| ATOM | 3987 | CD2 | LEU | B | 207 | 9.442 | 41.416 | 81.979 | 1.00 | 29.69 | B | C |
| ATOM | 3988 | C | LEU | B | 207 | 6.410 | 40.445 | 85.176 | 1.00 | 27.17 | B | C |
| ATOM | 3989 | O | LEU | B | 207 | 6.750 | 40.193 | 86.328 | 1.00 | 27.17 | B | O |
| ATOM | 3990 | N | VAL | B | 208 | 5.294 | 39.977 | 84.628 | 1.00 | 49.37 | B | N |
| ATOM | 3991 | CA | VAL | B | 208 | 4.365 | 39.150 | 85.382 | 1.00 | 49.37 | B | C |
| ATOM | 3992 | CB | VAL | B | 208 | 2.930 | 39.649 | 85.209 | 1.00 | 34.51 | B | C |
| ATOM | 3993 | CG1 | VAL | B | 208 | 2.048 | 39.011 | 86.262 | 1.00 | 34.51 | B | C |
| ATOM | 3994 | CG2 | VAL | B | 208 | 2.896 | 41.177 | 85.302 | 1.00 | 34.51 | B | C |
| ATOM | 3995 | C | VAL | B | 208 | 4.458 | 37.709 | 84.915 | 1.00 | 49.37 | B | C |
| ATOM | 3996 | O | VAL | B | 208 | 4.442 | 37.452 | 83.714 | 1.00 | 49.37 | B | O |
| ATOM | 3997 | N | ALA | B | 209 | 4.566 | 36.775 | 85.856 | 1.00 | 39.04 | B | N |
| ATOM | 3998 | CA | ALA | B | 209 | 4.671 | 35.359 | 85.509 | 1.00 | 39.04 | B | C |
| ATOM | 3999 | CB | ALA | B | 209 | 4.682 | 34.496 | 86.779 | 1.00 | 21.99 | B | C |
| ATOM | 4000 | C | ALA | B | 209 | 3.503 | 34.964 | 84.607 | 1.00 | 39.04 | B | C |
| ATOM | 4001 | O | ALA | B | 209 | 2.358 | 35.372 | 84.834 | 1.00 | 39.04 | B | O |
| ATOM | 4002 | N | GLY | B | 210 | 3.790 | 34.185 | 83.569 | 1.00 | 39.93 | B | N |
| ATOM | 4003 | CA | GLY | B | 210 | 2.725 | 33.769 | 82.678 | 1.00 | 39.93 | B | C |
| ATOM | 4004 | C | GLY | B | 210 | 2.635 | 34.651 | 81.450 | 1.00 | 39.93 | B | C |
| ATOM | 4005 | O | GLY | B | 210 | 2.223 | 34.190 | 80.376 | 1.00 | 39.93 | B | O |
| ATOM | 4006 | N | GLU | B | 211 | 3.021 | 35.916 | 81.599 | 1.00 | 44.65 | B | N |
| ATOM | 4007 | CA | GLU | B | 211 | 2.977 | 36.859 | 80.484 | 1.00 | 44.65 | B | C |
| ATOM | 4008 | CB | GLU | B | 211 | 2.809 | 38.289 | 80.992 | 1.00 | 51.93 | B | C |
| ATOM | 4009 | CG | GLU | B | 211 | 1.766 | 38.454 | 82.064 | 1.00 | 51.93 | B | C |
| ATOM | 4010 | CD | GLU | B | 211 | 1.451 | 39.924 | 82.326 | 1.00 | 51.93 | B | C |
| ATOM | 4011 | OE1 | GLU | B | 211 | 0.609 | 40.210 | 83.219 | 1.00 | 51.93 | B | O |
| ATOM | 4012 | OE2 | GLU | B | 211 | 2.051 | 40.784 | 81.625 | 1.00 | 51.93 | B | O |
| ATOM | 4013 | C | GLU | B | 211 | 4.246 | 36.784 | 79.638 | 1.00 | 44.65 | B | C |
| ATOM | 4014 | O | GLU | B | 211 | 5.357 | 36.850 | 80.157 | 1.00 | 44.65 | B | O |

| | | | | | | | | | | | | |
|---|---|---|---|---|---|---|---|---|---|---|---|---|
| ATOM | 4015 | N | PRO | B | 212 | 4.099 | 36.622 | 78.320 | 1.00 | 53.54 | B | N |
| ATOM | 4016 | CD | PRO | B | 212 | 2.921 | 36.173 | 77.555 | 1.00 | 30.17 | B | C |
| ATOM | 4017 | CA | PRO | B | 212 | 5.306 | 36.552 | 77.499 | 1.00 | 53.54 | B | C |
| ATOM | 4018 | CB | PRO | B | 212 | 4.830 | 35.792 | 76.260 | 1.00 | 30.17 | B | C |
| ATOM | 4019 | CG | PRO | B | 212 | 3.421 | 36.226 | 76.130 | 1.00 | 30.17 | B | C |
| ATOM | 4020 | C | PRO | B | 212 | 5.809 | 37.954 | 77.184 | 1.00 | 53.54 | B | C |
| ATOM | 4021 | O | PRO | B | 212 | 5.011 | 38.905 | 77.131 | 1.00 | 53.54 | B | O |
| ATOM | 4022 | N | ASN | B | 213 | 7.122 | 38.081 | 76.985 | 1.00 | 22.95 | B | N |
| ATOM | 4023 | CA | ASN | B | 213 | 7.704 | 39.374 | 76.673 | 1.00 | 22.95 | B | C |
| ATOM | 4024 | CB | ASN | B | 213 | 8.344 | 39.959 | 77.914 | 1.00 | 24.31 | B | C |
| ATOM | 4025 | CG | ASN | B | 213 | 7.335 | 40.255 | 78.992 | 1.00 | 24.31 | B | C |
| ATOM | 4026 | OD1 | ASN | B | 213 | 6.470 | 41.117 | 78.831 | 1.00 | 24.31 | B | O |
| ATOM | 4027 | ND2 | ASN | B | 213 | 7.440 | 39.543 | 80.105 | 1.00 | 24.31 | B | N |
| ATOM | 4028 | C | ASN | B | 213 | 8.710 | 39.259 | 75.554 | 1.00 | 22.95 | B | C |
| ATOM | 4029 | O | ASN | B | 213 | 9.355 | 38.225 | 75.404 | 1.00 | 22.95 | B | O |
| ATOM | 4030 | N | VAL | B | 214 | 8.825 | 40.324 | 74.762 | 1.00 | 31.28 | B | N |
| ATOM | 4031 | CA | VAL | B | 214 | 9.727 | 40.353 | 73.619 | 1.00 | 31.28 | B | C |
| ATOM | 4032 | CB | VAL | B | 214 | 9.650 | 41.698 | 72.909 | 1.00 | 17.02 | B | C |
| ATOM | 4033 | CG1 | VAL | B | 214 | 8.268 | 41.894 | 72.345 | 1.00 | 17.02 | B | C |
| ATOM | 4034 | CG2 | VAL | B | 214 | 9.992 | 42.803 | 73.879 | 1.00 | 17.02 | B | C |
| ATOM | 4035 | C | VAL | B | 214 | 11.162 | 40.082 | 74.033 | 1.00 | 31.28 | B | C |
| ATOM | 4036 | O | VAL | B | 214 | 11.629 | 40.576 | 75.057 | 1.00 | 31.28 | B | O |
| ATOM | 4037 | N | SER | B | 215 | 11.857 | 39.292 | 73.226 | 1.00 | 31.30 | B | N |
| ATOM | 4038 | CA | SER | B | 215 | 13.228 | 38.922 | 73.519 | 1.00 | 31.30 | B | C |
| ATOM | 4039 | CB | SER | B | 215 | 13.472 | 37.478 | 73.096 | 1.00 | 43.00 | B | C |
| ATOM | 4040 | OG | SER | B | 215 | 13.157 | 37.298 | 71.726 | 1.00 | 43.00 | B | O |
| ATOM | 4041 | C | SER | B | 215 | 14.261 | 39.798 | 72.854 | 1.00 | 31.30 | B | C |
| ATOM | 4042 | O | SER | B | 215 | 15.448 | 39.602 | 73.075 | 1.00 | 31.30 | B | O |
| ATOM | 4043 | N | TYR | B | 216 | 13.821 | 40.767 | 72.059 | 1.00 | 26.72 | B | N |
| ATOM | 4044 | CA | TYR | B | 216 | 14.750 | 41.637 | 71.344 | 1.00 | 26.72 | B | C |
| ATOM | 4045 | CB | TYR | B | 216 | 14.201 | 41.934 | 69.941 | 1.00 | 26.88 | B | C |
| ATOM | 4046 | CG | TYR | B | 216 | 12.805 | 42.490 | 69.923 | 1.00 | 26.88 | B | C |
| ATOM | 4047 | CD1 | TYR | B | 216 | 12.558 | 43.841 | 70.204 | 1.00 | 26.88 | B | C |
| ATOM | 4048 | CE1 | TYR | B | 216 | 11.261 | 44.363 | 70.185 | 1.00 | 26.88 | B | C |
| ATOM | 4049 | CD2 | TYR | B | 216 | 11.730 | 41.673 | 69.628 | 1.00 | 26.88 | B | C |
| ATOM | 4050 | CE2 | TYR | B | 216 | 10.435 | 42.175 | 69.606 | 1.00 | 26.88 | B | C |
| ATOM | 4051 | CZ | TYR | B | 216 | 10.205 | 43.521 | 69.883 | 1.00 | 26.88 | B | C |
| ATOM | 4052 | OH | TYR | B | 216 | 8.920 | 44.010 | 69.845 | 1.00 | 26.88 | B | O |
| ATOM | 4053 | C | TYR | B | 216 | 15.037 | 42.913 | 72.111 | 1.00 | 26.72 | B | C |
| ATOM | 4054 | O | TYR | B | 216 | 15.371 | 43.960 | 71.555 | 1.00 | 26.72 | B | O |
| ATOM | 4055 | N | ILE | B | 217 | 14.900 | 42.801 | 73.412 | 1.00 | 27.12 | B | N |
| ATOM | 4056 | CA | ILE | B | 217 | 15.173 | 43.893 | 74.316 | 1.00 | 27.12 | B | C |
| ATOM | 4057 | CB | ILE | B | 217 | 14.274 | 43.720 | 75.569 | 1.00 | 17.41 | B | C |
| ATOM | 4058 | CG2 | ILE | B | 217 | 14.902 | 42.762 | 76.549 | 1.00 | 17.41 | B | C |
| ATOM | 4059 | CG1 | ILE | B | 217 | 13.993 | 45.068 | 76.202 | 1.00 | 17.41 | B | C |
| ATOM | 4060 | CD1 | ILE | B | 217 | 13.006 | 45.879 | 75.396 | 1.00 | 17.41 | B | C |
| ATOM | 4061 | C | ILE | B | 217 | 16.651 | 43.628 | 74.650 | 1.00 | 27.12 | B | C |
| ATOM | 4062 | O | ILE | B | 217 | 17.220 | 42.619 | 74.230 | 1.00 | 27.12 | B | O |
| ATOM | 4063 | N | CYS | B | 218 | 17.264 | 44.530 | 75.395 | 1.00 | 26.58 | B | N |
| ATOM | 4064 | CA | CYS | B | 218 | 18.654 | 44.390 | 75.798 | 1.00 | 26.58 | B | C |
| ATOM | 4065 | CB | CYS | B | 218 | 18.862 | 43.082 | 76.537 | 1.00 | 26.71 | B | C |
| ATOM | 4066 | SG | CYS | B | 218 | 20.246 | 43.222 | 77.607 | 1.00 | 26.71 | B | S |
| ATOM | 4067 | C | CYS | B | 218 | 19.700 | 44.556 | 74.707 | 1.00 | 26.58 | B | C |
| ATOM | 4068 | O | CYS | B | 218 | 19.583 | 44.001 | 73.609 | 1.00 | 26.58 | B | O |
| ATOM | 4069 | N | SER | B | 219 | 20.716 | 45.355 | 75.036 | 1.00 | 24.61 | B | N |
| ATOM | 4070 | CA | SER | B | 219 | 21.835 | 45.653 | 74.143 | 1.00 | 24.61 | B | C |
| ATOM | 4071 | CB | SER | B | 219 | 22.611 | 46.873 | 74.648 | 1.00 | 25.72 | B | C |
| ATOM | 4072 | OG | SER | B | 219 | 21.820 | 48.042 | 74.571 | 1.00 | 25.72 | B | O |
| ATOM | 4073 | C | SER | B | 219 | 22.816 | 44.506 | 73.947 | 1.00 | 24.61 | B | C |
| ATOM | 4074 | O | SER | B | 219 | 22.986 | 43.663 | 74.812 | 1.00 | 24.61 | B | O |
| ATOM | 4075 | N | ARG | B | 220 | 23.487 | 44.536 | 72.800 | 1.00 | 49.64 | B | N |
| ATOM | 4076 | CA | ARG | B | 220 | 24.459 | 43.538 | 72.362 | 1.00 | 49.64 | B | C |
| ATOM | 4077 | CB | ARG | B | 220 | 25.450 | 44.205 | 71.384 | 1.00 | 60.86 | B | C |
| ATOM | 4078 | CG | ARG | B | 220 | 26.691 | 44.875 | 72.003 | 1.00 | 60.86 | B | C |
| ATOM | 4079 | CD | ARG | B | 220 | 26.511 | 46.301 | 72.518 | 1.00 | 60.86 | B | C |
| ATOM | 4080 | NE | ARG | B | 220 | 27.313 | 47.244 | 71.725 | 1.00 | 60.86 | B | N |
| ATOM | 4081 | CZ | ARG | B | 220 | 27.580 | 48.512 | 72.062 | 1.00 | 60.86 | B | C |

| | | | | | | | | | | | | |
|---|---|---|---|---|---|---|---|---|---|---|---|---|
| ATOM | 4082 | NH1 | ARG | B | 220 | 27.108 | 49.017 | 73.199 | 1.00 | 60.86 | B | N |
| ATOM | 4083 | NH2 | ARG | B | 220 | 28.322 | 49.282 | 71.257 | 1.00 | 60.86 | B | N |
| ATOM | 4084 | C | ARG | B | 220 | 25.210 | 42.720 | 73.426 | 1.00 | 49.64 | B | C |
| ATOM | 4085 | O | ARG | B | 220 | 24.704 | 41.699 | 73.895 | 1.00 | 49.64 | B | O |
| ATOM | 4086 | N | TYR | B | 221 | 26.401 | 43.175 | 73.798 | 1.00 | 22.39 | B | N |
| ATOM | 4087 | CA | TYR | B | 221 | 27.273 | 42.513 | 74.765 | 1.00 | 22.39 | B | C |
| ATOM | 4088 | CB | TYR | B | 221 | 28.546 | 43.349 | 74.956 | 1.00 | 40.76 | B | C |
| ATOM | 4089 | CG | TYR | B | 221 | 29.235 | 43.694 | 73.656 | 1.00 | 40.76 | B | C |
| ATOM | 4090 | CD1 | TYR | B | 221 | 29.217 | 42.799 | 72.584 | 1.00 | 40.76 | B | C |
| ATOM | 4091 | CE1 | TYR | B | 221 | 29.845 | 43.091 | 71.381 | 1.00 | 40.76 | B | C |
| ATOM | 4092 | CD2 | TYR | B | 221 | 29.911 | 44.903 | 73.489 | 1.00 | 40.76 | B | C |
| ATOM | 4093 | CE2 | TYR | B | 221 | 30.549 | 45.201 | 72.283 | 1.00 | 40.76 | B | C |
| ATOM | 4094 | CZ | TYR | B | 221 | 30.506 | 44.285 | 71.238 | 1.00 | 40.76 | B | C |
| ATOM | 4095 | OH | TYR | B | 221 | 31.115 | 44.549 | 70.040 | 1.00 | 40.76 | B | O |
| ATOM | 4096 | C | TYR | B | 221 | 26.732 | 42.167 | 76.144 | 1.00 | 22.39 | B | C |
| ATOM | 4097 | O | TYR | B | 221 | 27.382 | 41.419 | 76.866 | 1.00 | 22.39 | B | O |
| ATOM | 4098 | N | TYR | B | 222 | 25.563 | 42.684 | 76.518 | 1.00 | 20.48 | B | N |
| ATOM | 4099 | CA | TYR | B | 222 | 25.022 | 42.424 | 77.852 | 1.00 | 20.48 | B | C |
| ATOM | 4100 | CB | TYR | B | 222 | 24.795 | 43.753 | 78.576 | 1.00 | 26.43 | B | C |
| ATOM | 4101 | CG | TYR | B | 222 | 25.942 | 44.708 | 78.383 | 1.00 | 26.43 | B | C |
| ATOM | 4102 | CD1 | TYR | B | 222 | 27.044 | 44.684 | 79.216 | 1.00 | 26.43 | B | C |
| ATOM | 4103 | CE1 | TYR | B | 222 | 28.128 | 45.523 | 78.981 | 1.00 | 26.43 | B | C |
| ATOM | 4104 | CD2 | TYR | B | 222 | 25.953 | 45.595 | 77.314 | 1.00 | 26.43 | B | C |
| ATOM | 4105 | CE2 | TYR | B | 222 | 27.028 | 46.429 | 77.076 | 1.00 | 26.43 | B | C |
| ATOM | 4106 | CZ | TYR | B | 222 | 28.112 | 46.391 | 77.908 | 1.00 | 26.43 | B | C |
| ATOM | 4107 | OH | TYR | B | 222 | 29.179 | 47.224 | 77.667 | 1.00 | 26.43 | B | O |
| ATOM | 4108 | C | TYR | B | 222 | 23.759 | 41.592 | 77.952 | 1.00 | 20.48 | B | C |
| ATOM | 4109 | O | TYR | B | 222 | 23.195 | 41.476 | 79.034 | 1.00 | 20.48 | B | O |
| ATOM | 4110 | N | ARG | B | 223 | 23.329 | 41.000 | 76.847 | 1.00 | 16.85 | B | N |
| ATOM | 4111 | CA | ARG | B | 223 | 22.116 | 40.191 | 76.832 | 1.00 | 16.85 | B | C |
| ATOM | 4112 | CB | ARG | B | 223 | 21.637 | 40.030 | 75.398 | 1.00 | 21.43 | B | C |
| ATOM | 4113 | CG | ARG | B | 223 | 21.648 | 41.332 | 74.637 | 1.00 | 21.43 | B | C |
| ATOM | 4114 | CD | ARG | B | 223 | 21.234 | 41.169 | 73.195 | 1.00 | 21.43 | B | C |
| ATOM | 4115 | NE | ARG | B | 223 | 19.809 | 40.913 | 73.054 | 1.00 | 21.43 | B | N |
| ATOM | 4116 | CZ | ARG | B | 223 | 19.321 | 39.799 | 72.532 | 1.00 | 21.43 | B | C |
| ATOM | 4117 | NH1 | ARG | B | 223 | 20.153 | 38.859 | 72.117 | 1.00 | 21.43 | B | N |
| ATOM | 4118 | NH2 | ARG | B | 223 | 18.014 | 39.635 | 72.414 | 1.00 | 21.43 | B | N |
| ATOM | 4119 | C | ARG | B | 223 | 22.279 | 38.820 | 77.469 | 1.00 | 16.85 | B | C |
| ATOM | 4120 | O | ARG | B | 223 | 23.211 | 38.094 | 77.164 | 1.00 | 16.85 | B | O |
| ATOM | 4121 | N | ALA | B | 224 | 21.363 | 38.466 | 78.359 | 1.00 | 21.16 | B | N |
| ATOM | 4122 | CA | ALA | B | 224 | 21.429 | 37.170 | 79.025 | 1.00 | 21.16 | B | C |
| ATOM | 4123 | CB | ALA | B | 224 | 20.317 | 37.049 | 80.051 | 1.00 | 16.30 | B | C |
| ATOM | 4124 | C | ALA | B | 224 | 21.329 | 36.040 | 78.020 | 1.00 | 21.16 | B | C |
| ATOM | 4125 | O | ALA | B | 224 | 20.653 | 36.149 | 77.001 | 1.00 | 21.16 | B | O |
| ATOM | 4126 | N | PRO | B | 225 | 21.999 | 34.924 | 78.296 | 1.00 | 25.24 | B | N |
| ATOM | 4127 | CD | PRO | B | 225 | 22.876 | 34.615 | 79.430 | 1.00 | 32.24 | B | C |
| ATOM | 4128 | CA | PRO | B | 225 | 21.923 | 33.817 | 77.350 | 1.00 | 25.24 | B | C |
| ATOM | 4129 | CB | PRO | B | 225 | 22.695 | 32.713 | 78.054 | 1.00 | 32.24 | B | C |
| ATOM | 4130 | CG | PRO | B | 225 | 22.720 | 33.162 | 79.514 | 1.00 | 32.24 | B | C |
| ATOM | 4131 | C | PRO | B | 225 | 20.504 | 33.453 | 76.943 | 1.00 | 25.24 | B | C |
| ATOM | 4132 | O | PRO | B | 225 | 20.281 | 33.197 | 75.781 | 1.00 | 25.24 | B | O |
| ATOM | 4133 | N | GLU | B | 226 | 19.542 | 33.470 | 77.860 | 1.00 | 26.45 | B | N |
| ATOM | 4134 | CA | GLU | B | 226 | 18.160 | 33.145 | 77.494 | 1.00 | 26.45 | B | C |
| ATOM | 4135 | CB | GLU | B | 226 | 17.256 | 33.074 | 78.735 | 1.00 | 20.80 | B | C |
| ATOM | 4136 | CG | GLU | B | 226 | 17.195 | 34.344 | 79.569 | 1.00 | 20.80 | B | C |
| ATOM | 4137 | CD | GLU | B | 226 | 18.166 | 34.332 | 80.727 | 1.00 | 20.80 | B | C |
| ATOM | 4138 | OE1 | GLU | B | 226 | 19.322 | 33.945 | 80.513 | 1.00 | 20.80 | B | O |
| ATOM | 4139 | OE2 | GLU | B | 226 | 17.790 | 34.715 | 81.852 | 1.00 | 20.80 | B | O |
| ATOM | 4140 | C | GLU | B | 226 | 17.574 | 34.138 | 76.497 | 1.00 | 26.45 | B | C |
| ATOM | 4141 | O | GLU | B | 226 | 16.739 | 33.768 | 75.672 | 1.00 | 26.45 | B | O |
| ATOM | 4142 | N | LEU | B | 227 | 17.991 | 35.397 | 76.566 | 1.00 | 24.06 | B | N |
| ATOM | 4143 | CA | LEU | B | 227 | 17.458 | 36.374 | 75.626 | 1.00 | 24.06 | B | C |
| ATOM | 4144 | CB | LEU | B | 227 | 17.869 | 37.793 | 76.016 | 1.00 | 19.28 | B | C |
| ATOM | 4145 | CG | LEU | B | 227 | 17.267 | 38.307 | 77.318 | 1.00 | 19.28 | B | C |
| ATOM | 4146 | CD1 | LEU | B | 227 | 17.775 | 39.708 | 77.542 | 1.00 | 19.28 | B | C |
| ATOM | 4147 | CD2 | LEU | B | 227 | 15.758 | 38.277 | 77.263 | 1.00 | 19.28 | B | C |
| ATOM | 4148 | C | LEU | B | 227 | 17.976 | 36.040 | 74.230 | 1.00 | 24.06 | B | C |

| | | | | | | | | | | | | |
|---|---|---|---|---|---|---|---|---|---|---|---|---|
| ATOM | 4149 | O | LEU | B | 227 | 17.286 | 36.246 | 73.232 | 1.00 | 24.06 | B | O |
| ATOM | 4150 | N | ILE | B | 228 | 19.194 | 35.515 | 74.165 | 1.00 | 23.42 | B | N |
| ATOM | 4151 | CA | ILE | B | 228 | 19.769 | 35.152 | 72.887 | 1.00 | 23.42 | B | C |
| ATOM | 4152 | CB | ILE | B | 228 | 21.269 | 34.836 | 73.022 | 1.00 | 16.11 | B | C |
| ATOM | 4153 | CG2 | ILE | B | 228 | 21.817 | 34.339 | 71.694 | 1.00 | 16.11 | B | C |
| ATOM | 4154 | CG1 | ILE | B | 228 | 22.029 | 36.102 | 73.435 | 1.00 | 16.11 | B | C |
| ATOM | 4155 | CD1 | ILE | B | 228 | 23.483 | 35.860 | 73.803 | 1.00 | 16.11 | B | C |
| ATOM | 4156 | C | ILE | B | 228 | 19.031 | 33.944 | 72.316 | 1.00 | 23.42 | B | C |
| ATOM | 4157 | O | ILE | B | 228 | 18.849 | 33.838 | 71.104 | 1.00 | 23.42 | B | O |
| ATOM | 4158 | N | PHE | B | 229 | 18.590 | 33.042 | 73.187 | 1.00 | 34.60 | B | N |
| ATOM | 4159 | CA | PHE | B | 229 | 17.862 | 31.859 | 72.743 | 1.00 | 34.60 | B | C |
| ATOM | 4160 | CB | PHE | B | 229 | 17.938 | 30.746 | 73.789 | 1.00 | 19.68 | B | C |
| ATOM | 4161 | CG | PHE | B | 229 | 19.298 | 30.135 | 73.948 | 1.00 | 19.68 | B | C |
| ATOM | 4162 | CD1 | PHE | B | 229 | 19.920 | 29.506 | 72.879 | 1.00 | 19.68 | B | C |
| ATOM | 4163 | CD2 | PHE | B | 229 | 19.941 | 30.148 | 75.182 | 1.00 | 19.68 | B | C |
| ATOM | 4164 | CE1 | PHE | B | 229 | 21.158 | 28.893 | 73.028 | 1.00 | 19.68 | B | C |
| ATOM | 4165 | CE2 | PHE | B | 229 | 21.186 | 29.532 | 75.340 | 1.00 | 19.68 | B | C |
| ATOM | 4166 | CZ | PHE | B | 229 | 21.795 | 28.904 | 74.259 | 1.00 | 19.68 | B | C |
| ATOM | 4167 | C | PHE | B | 229 | 16.389 | 32.148 | 72.428 | 1.00 | 34.60 | B | C |
| ATOM | 4168 | O | PHE | B | 229 | 15.592 | 31.217 | 72.260 | 1.00 | 34.60 | B | O |
| ATOM | 4169 | N | GLY | B | 230 | 16.024 | 33.426 | 72.367 | 1.00 | 31.91 | B | N |
| ATOM | 4170 | CA | GLY | B | 230 | 14.653 | 33.798 | 72.044 | 1.00 | 31.91 | B | C |
| ATOM | 4171 | C | GLY | B | 230 | 13.571 | 33.530 | 73.079 | 1.00 | 31.91 | B | C |
| ATOM | 4172 | O | GLY | B | 230 | 12.380 | 33.657 | 72.784 | 1.00 | 31.91 | B | O |
| ATOM | 4173 | N | ALA | B | 231 | 13.974 | 33.148 | 74.287 | 1.00 | 24.97 | B | N |
| ATOM | 4174 | CA | ALA | B | 231 | 13.025 | 32.891 | 75.365 | 1.00 | 24.97 | B | C |
| ATOM | 4175 | CB | ALA | B | 231 | 13.766 | 32.594 | 76.645 | 1.00 | 15.84 | B | C |
| ATOM | 4176 | C | ALA | B | 231 | 12.139 | 34.110 | 75.561 | 1.00 | 24.97 | B | C |
| ATOM | 4177 | O | ALA | B | 231 | 12.584 | 35.237 | 75.386 | 1.00 | 24.97 | B | O |
| ATOM | 4178 | N | THR | B | 232 | 10.883 | 33.891 | 75.918 | 1.00 | 39.88 | B | N |
| ATOM | 4179 | CA | THR | B | 232 | 9.973 | 35.008 | 76.132 | 1.00 | 39.88 | B | C |
| ATOM | 4180 | CB | THR | B | 232 | 8.857 | 35.024 | 75.085 | 1.00 | 33.65 | B | C |
| ATOM | 4181 | OG1 | THR | B | 232 | 8.114 | 33.801 | 75.171 | 1.00 | 33.65 | B | O |
| ATOM | 4182 | CG2 | THR | B | 232 | 9.437 | 35.185 | 73.693 | 1.00 | 33.65 | B | C |
| ATOM | 4183 | C | THR | B | 232 | 9.328 | 34.959 | 77.512 | 1.00 | 39.88 | B | C |
| ATOM | 4184 | O | THR | B | 232 | 8.356 | 35.679 | 77.777 | 1.00 | 39.88 | B | O |
| ATOM | 4185 | N | ASP | B | 233 | 9.870 | 34.104 | 78.375 | 1.00 | 23.86 | B | N |
| ATOM | 4186 | CA | ASP | B | 233 | 9.379 | 33.954 | 79.736 | 1.00 | 23.86 | B | C |
| ATOM | 4187 | CB | ASP | B | 233 | 8.882 | 32.539 | 79.958 | 1.00 | 23.48 | B | C |
| ATOM | 4188 | CG | ASP | B | 233 | 9.952 | 31.513 | 79.708 | 1.00 | 23.48 | B | C |
| ATOM | 4189 | OD1 | ASP | B | 233 | 11.008 | 31.864 | 79.137 | 1.00 | 23.48 | B | O |
| ATOM | 4190 | OD2 | ASP | B | 233 | 9.733 | 30.347 | 80.078 | 1.00 | 23.48 | B | O |
| ATOM | 4191 | C | ASP | B | 233 | 10.516 | 34.253 | 80.700 | 1.00 | 23.86 | B | C |
| ATOM | 4192 | O | ASP | B | 233 | 10.584 | 33.705 | 81.802 | 1.00 | 23.86 | B | O |
| ATOM | 4193 | N | TYR | B | 234 | 11.412 | 35.137 | 80.275 | 1.00 | 32.46 | B | N |
| ATOM | 4194 | CA | TYR | B | 234 | 12.541 | 35.518 | 81.106 | 1.00 | 32.46 | B | C |
| ATOM | 4195 | CB | TYR | B | 234 | 13.530 | 36.354 | 80.290 | 1.00 | 23.59 | B | C |
| ATOM | 4196 | CG | TYR | B | 234 | 12.953 | 37.588 | 79.631 | 1.00 | 23.59 | B | C |
| ATOM | 4197 | CD1 | TYR | B | 234 | 12.158 | 37.495 | 78.495 | 1.00 | 23.59 | B | C |
| ATOM | 4198 | CE1 | TYR | B | 234 | 11.679 | 38.638 | 77.850 | 1.00 | 23.59 | B | C |
| ATOM | 4199 | CD2 | TYR | B | 234 | 13.248 | 38.855 | 80.112 | 1.00 | 23.59 | B | C |
| ATOM | 4200 | CE2 | TYR | B | 234 | 12.770 | 39.998 | 79.476 | 1.00 | 23.59 | B | C |
| ATOM | 4201 | CZ | TYR | B | 234 | 11.990 | 39.885 | 78.348 | 1.00 | 23.59 | B | C |
| ATOM | 4202 | OH | TYR | B | 234 | 11.537 | 41.034 | 77.737 | 1.00 | 23.59 | B | O |
| ATOM | 4203 | C | TYR | B | 234 | 12.096 | 36.286 | 82.351 | 1.00 | 32.46 | B | C |
| ATOM | 4204 | O | TYR | B | 234 | 11.012 | 36.871 | 82.382 | 1.00 | 32.46 | B | O |
| ATOM | 4205 | N | THR | B | 235 | 12.943 | 36.271 | 83.378 | 1.00 | 28.86 | B | N |
| ATOM | 4206 | CA | THR | B | 235 | 12.649 | 36.950 | 84.639 | 1.00 | 28.86 | B | C |
| ATOM | 4207 | CB | THR | B | 235 | 12.990 | 36.071 | 85.835 | 1.00 | 26.02 | B | C |
| ATOM | 4208 | OG1 | THR | B | 235 | 14.415 | 35.936 | 85.935 | 1.00 | 26.02 | B | O |
| ATOM | 4209 | CG2 | THR | B | 235 | 12.367 | 34.706 | 85.674 | 1.00 | 26.02 | B | C |
| ATOM | 4210 | C | THR | B | 235 | 13.499 | 38.194 | 84.760 | 1.00 | 28.86 | B | C |
| ATOM | 4211 | O | THR | B | 235 | 14.212 | 38.567 | 83.835 | 1.00 | 28.86 | B | O |
| ATOM | 4212 | N | SER | B | 236 | 13.420 | 38.838 | 85.913 | 1.00 | 29.36 | B | N |
| ATOM | 4213 | CA | SER | B | 236 | 14.213 | 40.025 | 86.148 | 1.00 | 29.36 | B | C |
| ATOM | 4214 | CB | SER | B | 236 | 13.747 | 40.719 | 87.420 | 1.00 | 65.06 | B | C |
| ATOM | 4215 | OG | SER | B | 236 | 12.472 | 41.299 | 87.192 | 1.00 | 65.06 | B | O |

| | | | | | | | | | | | | |
|---|---|---|---|---|---|---|---|---|---|---|---|---|
| ATOM | 4216 | C | SER | B | 236 | 15.673 | 39.639 | 86.245 | 1.00 | 29.36 | B | C |
| ATOM | 4217 | O | SER | B | 236 | 16.547 | 40.496 | 86.208 | 1.00 | 29.36 | B | O |
| ATOM | 4218 | N | SER | B | 237 | 15.925 | 38.338 | 86.340 | 1.00 | 26.30 | B | N |
| ATOM | 4219 | CA | SER | B | 237 | 17.277 | 37.810 | 86.429 | 1.00 | 26.30 | B | C |
| ATOM | 4220 | CB | SER | B | 237 | 17.220 | 36.288 | 86.387 | 1.00 | 45.78 | B | C |
| ATOM | 4221 | OG | SER | B | 237 | 18.520 | 35.744 | 86.450 | 1.00 | 45.78 | B | O |
| ATOM | 4222 | C | SER | B | 237 | 18.095 | 38.334 | 85.257 | 1.00 | 26.30 | B | C |
| ATOM | 4223 | O | SER | B | 237 | 19.322 | 38.407 | 85.304 | 1.00 | 26.30 | B | O |
| ATOM | 4224 | N | ILE | B | 238 | 17.381 | 38.691 | 84.201 | 1.00 | 25.85 | B | N |
| ATOM | 4225 | CA | ILE | B | 238 | 17.958 | 39.241 | 82.991 | 1.00 | 25.85 | B | C |
| ATOM | 4226 | CB | ILE | B | 238 | 16.794 | 39.748 | 82.087 | 1.00 | 28.25 | B | C |
| ATOM | 4227 | CG2 | ILE | B | 238 | 16.949 | 41.226 | 81.753 | 1.00 | 28.25 | B | C |
| ATOM | 4228 | CG1 | ILE | B | 238 | 16.743 | 38.944 | 80.797 | 1.00 | 28.25 | B | C |
| ATOM | 4229 | CD1 | ILE | B | 238 | 16.549 | 37.489 | 81.002 | 1.00 | 28.25 | B | C |
| ATOM | 4230 | C | ILE | B | 238 | 18.903 | 40.393 | 83.343 | 1.00 | 25.85 | B | C |
| ATOM | 4231 | O | ILE | B | 238 | 20.009 | 40.480 | 82.828 | 1.00 | 25.85 | B | O |
| ATOM | 4232 | N | ASP | B | 239 | 18.452 | 41.282 | 84.217 | 1.00 | 15.97 | B | N |
| ATOM | 4233 | CA | ASP | B | 239 | 19.260 | 42.410 | 84.632 | 1.00 | 15.97 | B | C |
| ATOM | 4234 | CB | ASP | B | 239 | 18.431 | 43.367 | 85.485 | 1.00 | 29.29 | B | C |
| ATOM | 4235 | CG | ASP | B | 239 | 17.408 | 44.144 | 84.662 | 1.00 | 29.29 | B | C |
| ATOM | 4236 | OD1 | ASP | B | 239 | 17.674 | 44.411 | 83.461 | 1.00 | 29.29 | B | O |
| ATOM | 4237 | OD2 | ASP | B | 239 | 16.346 | 44.501 | 85.225 | 1.00 | 29.29 | B | O |
| ATOM | 4238 | C | ASP | B | 239 | 20.490 | 41.959 | 85.402 | 1.00 | 15.97 | B | C |
| ATOM | 4239 | O | ASP | B | 239 | 21.586 | 42.439 | 85.162 | 1.00 | 15.97 | B | O |
| ATOM | 4240 | N | VAL | B | 240 | 20.314 | 41.023 | 86.321 | 1.00 | 14.56 | B | N |
| ATOM | 4241 | CA | VAL | B | 240 | 21.430 | 40.552 | 87.106 | 1.00 | 14.56 | B | C |
| ATOM | 4242 | CB | VAL | B | 240 | 20.998 | 39.391 | 88.021 | 1.00 | 17.75 | B | C |
| ATOM | 4243 | CG1 | VAL | B | 240 | 22.198 | 38.788 | 88.725 | 1.00 | 17.75 | B | C |
| ATOM | 4244 | CG2 | VAL | B | 240 | 20.015 | 39.897 | 89.062 | 1.00 | 17.75 | B | C |
| ATOM | 4245 | C | VAL | B | 240 | 22.558 | 40.120 | 86.196 | 1.00 | 14.56 | B | C |
| ATOM | 4246 | O | VAL | B | 240 | 23.702 | 40.467 | 86.443 | 1.00 | 14.56 | B | O |
| ATOM | 4247 | N | TRP | B | 241 | 22.241 | 39.370 | 85.142 | 1.00 | 17.93 | B | N |
| ATOM | 4248 | CA | TRP | B | 241 | 23.264 | 38.911 | 84.207 | 1.00 | 17.93 | B | C |
| ATOM | 4249 | CB | TRP | B | 241 | 22.659 | 38.019 | 83.121 | 1.00 | 27.72 | B | C |
| ATOM | 4250 | CG | TRP | B | 241 | 23.579 | 37.758 | 81.936 | 1.00 | 27.72 | B | C |
| ATOM | 4251 | CD2 | TRP | B | 241 | 24.390 | 36.597 | 81.695 | 1.00 | 27.72 | B | C |
| ATOM | 4252 | CE2 | TRP | B | 241 | 25.103 | 36.818 | 80.498 | 1.00 | 27.72 | B | C |
| ATOM | 4253 | CE3 | TRP | B | 241 | 24.575 | 35.375 | 82.373 | 1.00 | 27.72 | B | C |
| ATOM | 4254 | CD1 | TRP | B | 241 | 23.836 | 38.613 | 80.902 | 1.00 | 27.72 | B | C |
| ATOM | 4255 | NE1 | TRP | B | 241 | 24.749 | 38.062 | 80.039 | 1.00 | 27.72 | B | N |
| ATOM | 4256 | CZ2 | TRP | B | 241 | 26.002 | 35.890 | 79.965 | 1.00 | 27.72 | B | C |
| ATOM | 4257 | CZ3 | TRP | B | 241 | 25.471 | 34.443 | 81.840 | 1.00 | 27.72 | B | C |
| ATOM | 4258 | CH2 | TRP | B | 241 | 26.167 | 34.706 | 80.644 | 1.00 | 27.72 | B | C |
| ATOM | 4259 | C | TRP | B | 241 | 23.938 | 40.102 | 83.580 | 1.00 | 17.93 | B | C |
| ATOM | 4260 | O | TRP | B | 241 | 25.158 | 40.174 | 83.559 | 1.00 | 17.93 | B | O |
| ATOM | 4261 | N | SER | B | 242 | 23.142 | 41.039 | 83.077 | 1.00 | 11.89 | B | N |
| ATOM | 4262 | CA | SER | B | 242 | 23.682 | 42.245 | 82.457 | 1.00 | 11.89 | B | C |
| ATOM | 4263 | CB | SER | B | 242 | 22.551 | 43.208 | 82.078 | 1.00 | 32.88 | B | C |
| ATOM | 4264 | OG | SER | B | 242 | 21.591 | 42.599 | 81.233 | 1.00 | 32.88 | B | O |
| ATOM | 4265 | C | SER | B | 242 | 24.608 | 42.938 | 83.447 | 1.00 | 11.89 | B | C |
| ATOM | 4266 | O | SER | B | 242 | 25.646 | 43.475 | 83.082 | 1.00 | 11.89 | B | O |
| ATOM | 4267 | N | ALA | B | 243 | 24.207 | 42.925 | 84.712 | 1.00 | 22.79 | B | N |
| ATOM | 4268 | CA | ALA | B | 243 | 24.980 | 43.537 | 85.772 | 1.00 | 22.79 | B | C |
| ATOM | 4269 | CB | ALA | B | 243 | 24.235 | 43.409 | 87.055 | 1.00 | 14.79 | B | C |
| ATOM | 4270 | C | ALA | B | 243 | 26.322 | 42.828 | 85.861 | 1.00 | 22.79 | B | C |
| ATOM | 4271 | O | ALA | B | 243 | 27.364 | 43.467 | 85.941 | 1.00 | 22.79 | B | O |
| ATOM | 4272 | N | GLY | B | 244 | 26.296 | 41.502 | 85.825 | 1.00 | 30.07 | B | N |
| ATOM | 4273 | CA | GLY | B | 244 | 27.529 | 40.743 | 85.895 | 1.00 | 30.07 | B | C |
| ATOM | 4274 | C | GLY | B | 244 | 28.418 | 40.934 | 84.677 | 1.00 | 30.07 | B | C |
| ATOM | 4275 | O | GLY | B | 244 | 29.607 | 40.653 | 84.728 | 1.00 | 30.07 | B | O |
| ATOM | 4276 | N | CYS | B | 245 | 27.854 | 41.403 | 83.573 | 1.00 | 28.83 | B | N |
| ATOM | 4277 | CA | CYS | B | 245 | 28.655 | 41.637 | 82.379 | 1.00 | 28.83 | B | C |
| ATOM | 4278 | CB | CYS | B | 245 | 27.778 | 41.642 | 81.129 | 1.00 | 19.60 | B | C |
| ATOM | 4279 | SG | CYS | B | 245 | 27.203 | 40.034 | 80.584 | 1.00 | 19.60 | B | S |
| ATOM | 4280 | C | CYS | B | 245 | 29.354 | 42.987 | 82.517 | 1.00 | 28.83 | B | C |
| ATOM | 4281 | O | CYS | B | 245 | 30.401 | 43.216 | 81.917 | 1.00 | 28.83 | B | O |
| ATOM | 4282 | N | VAL | B | 246 | 28.766 | 43.892 | 83.298 | 1.00 | 29.43 | B | N |

| | | | | | | | | | | | | |
|---|---|---|---|---|---|---|---|---|---|---|---|---|
| ATOM | 4283 | CA | VAL | B | 246 | 29.362 | 45.206 | 83.506 | 1.00 | 29.43 | B | C |
| ATOM | 4284 | CB | VAL | B | 246 | 28.340 | 46.212 | 84.069 | 1.00 | 19.81 | B | C |
| ATOM | 4285 | CG1 | VAL | B | 246 | 29.016 | 47.526 | 84.399 | 1.00 | 19.81 | B | C |
| ATOM | 4286 | CG2 | VAL | B | 246 | 27.239 | 46.433 | 83.064 | 1.00 | 19.81 | B | C |
| ATOM | 4287 | C | VAL | B | 246 | 30.524 | 45.079 | 84.478 | 1.00 | 29.43 | B | C |
| ATOM | 4288 | O | VAL | B | 246 | 31.569 | 45.688 | 84.271 | 1.00 | 29.43 | B | O |
| ATOM | 4289 | N | LEU | B | 247 | 30.342 | 44.281 | 85.531 | 1.00 | 20.35 | B | N |
| ATOM | 4290 | CA | LEU | B | 247 | 31.386 | 44.073 | 86.528 | 1.00 | 20.35 | B | C |
| ATOM | 4291 | CB | LEU | B | 247 | 30.882 | 43.167 | 87.652 | 1.00 | 14.58 | B | C |
| ATOM | 4292 | CG | LEU | B | 247 | 31.931 | 42.538 | 88.572 | 1.00 | 14.58 | B | C |
| ATOM | 4293 | CD1 | LEU | B | 247 | 32.561 | 43.581 | 89.452 | 1.00 | 14.58 | B | C |
| ATOM | 4294 | CD2 | LEU | B | 247 | 31.286 | 41.470 | 89.394 | 1.00 | 14.58 | B | C |
| ATOM | 4295 | C | LEU | B | 247 | 32.576 | 43.424 | 85.866 | 1.00 | 20.35 | B | C |
| ATOM | 4296 | O | LEU | B | 247 | 33.708 | 43.833 | 86.076 | 1.00 | 20.35 | B | O |
| ATOM | 4297 | N | ALA | B | 248 | 32.315 | 42.399 | 85.068 | 1.00 | 26.30 | B | N |
| ATOM | 4298 | CA | ALA | B | 248 | 33.386 | 41.709 | 84.380 | 1.00 | 26.30 | B | C |
| ATOM | 4299 | CB | ALA | B | 248 | 32.826 | 40.552 | 83.596 | 1.00 | 26.17 | B | C |
| ATOM | 4300 | C | ALA | B | 248 | 34.096 | 42.680 | 83.450 | 1.00 | 26.30 | B | C |
| ATOM | 4301 | O | ALA | B | 248 | 35.323 | 42.686 | 83.363 | 1.00 | 26.30 | B | O |
| ATOM | 4302 | N | GLU | B | 249 | 33.326 | 43.510 | 82.759 | 1.00 | 21.36 | B | N |
| ATOM | 4303 | CA | GLU | B | 249 | 33.914 | 44.475 | 81.845 | 1.00 | 21.36 | B | C |
| ATOM | 4304 | CB | GLU | B | 249 | 32.815 | 45.221 | 81.080 | 1.00 | 22.74 | B | C |
| ATOM | 4305 | CG | GLU | B | 249 | 33.327 | 46.086 | 79.935 | 1.00 | 22.74 | B | C |
| ATOM | 4306 | CD | GLU | B | 249 | 32.226 | 46.545 | 78.999 | 1.00 | 22.74 | B | C |
| ATOM | 4307 | OE1 | GLU | B | 249 | 31.290 | 45.763 | 78.738 | 1.00 | 22.74 | B | O |
| ATOM | 4308 | OE2 | GLU | B | 249 | 32.307 | 47.683 | 78.503 | 1.00 | 22.74 | B | O |
| ATOM | 4309 | C | GLU | B | 249 | 34.821 | 45.476 | 82.561 | 1.00 | 21.36 | B | C |
| ATOM | 4310 | O | GLU | B | 249 | 35.886 | 45.813 | 82.061 | 1.00 | 21.36 | B | O |
| ATOM | 4311 | N | LEU | B | 250 | 34.403 | 45.943 | 83.735 | 1.00 | 33.51 | B | N |
| ATOM | 4312 | CA | LEU | B | 250 | 35.189 | 46.916 | 84.486 | 1.00 | 33.51 | B | C |
| ATOM | 4313 | CB | LEU | B | 250 | 34.363 | 47.498 | 85.631 | 1.00 | 17.10 | B | C |
| ATOM | 4314 | CG | LEU | B | 250 | 33.203 | 48.411 | 85.250 | 1.00 | 17.10 | B | C |
| ATOM | 4315 | CD1 | LEU | B | 250 | 32.393 | 48.759 | 86.486 | 1.00 | 17.10 | B | C |
| ATOM | 4316 | CD2 | LEU | B | 250 | 33.743 | 49.657 | 84.597 | 1.00 | 17.10 | B | C |
| ATOM | 4317 | C | LEU | B | 250 | 36.459 | 46.307 | 85.046 | 1.00 | 33.51 | B | C |
| ATOM | 4318 | O | LEU | B | 250 | 37.419 | 47.023 | 85.338 | 1.00 | 33.51 | B | O |
| ATOM | 4319 | N | LEU | B | 251 | 36.459 | 44.988 | 85.214 | 1.00 | 32.68 | B | N |
| ATOM | 4320 | CA | LEU | B | 251 | 37.638 | 44.311 | 85.725 | 1.00 | 32.68 | B | C |
| ATOM | 4321 | CB | LEU | B | 251 | 37.272 | 42.959 | 86.360 | 1.00 | 23.35 | B | C |
| ATOM | 4322 | CG | LEU | B | 251 | 36.310 | 42.957 | 87.564 | 1.00 | 23.35 | B | C |
| ATOM | 4323 | CD1 | LEU | B | 251 | 35.941 | 41.527 | 87.920 | 1.00 | 23.35 | B | C |
| ATOM | 4324 | CD2 | LEU | B | 251 | 36.931 | 43.658 | 88.767 | 1.00 | 23.35 | B | C |
| ATOM | 4325 | C | LEU | B | 251 | 38.609 | 44.098 | 84.574 | 1.00 | 32.68 | B | C |
| ATOM | 4326 | O | LEU | B | 251 | 39.784 | 44.416 | 84.690 | 1.00 | 32.68 | B | O |
| ATOM | 4327 | N | LEU | B | 252 | 38.110 | 43.595 | 83.452 | 1.00 | 23.85 | B | N |
| ATOM | 4328 | CA | LEU | B | 252 | 38.940 | 43.318 | 82.284 | 1.00 | 23.85 | B | C |
| ATOM | 4329 | CB | LEU | B | 252 | 38.227 | 42.313 | 81.382 | 1.00 | 36.48 | B | C |
| ATOM | 4330 | CG | LEU | B | 252 | 38.191 | 40.854 | 81.839 | 1.00 | 36.48 | B | C |
| ATOM | 4331 | CD1 | LEU | B | 252 | 37.074 | 40.111 | 81.115 | 1.00 | 36.48 | B | C |
| ATOM | 4332 | CD2 | LEU | B | 252 | 39.535 | 40.201 | 81.564 | 1.00 | 36.48 | B | C |
| ATOM | 4333 | C | LEU | B | 252 | 39.380 | 44.515 | 81.442 | 1.00 | 23.85 | B | C |
| ATOM | 4334 | O | LEU | B | 252 | 40.435 | 44.468 | 80.810 | 1.00 | 23.85 | B | O |
| ATOM | 4335 | N | GLY | B | 253 | 38.583 | 45.578 | 81.413 | 1.00 | 19.24 | B | N |
| ATOM | 4336 | CA | GLY | B | 253 | 38.939 | 46.733 | 80.603 | 1.00 | 19.24 | B | C |
| ATOM | 4337 | C | GLY | B | 253 | 38.394 | 46.622 | 79.189 | 1.00 | 19.24 | B | C |
| ATOM | 4338 | O | GLY | B | 253 | 38.808 | 47.346 | 78.284 | 1.00 | 19.24 | B | O |
| ATOM | 4339 | N | GLN | B | 254 | 37.465 | 45.690 | 79.006 | 1.00 | 36.67 | B | N |
| ATOM | 4340 | CA | GLN | B | 254 | 36.821 | 45.438 | 77.720 | 1.00 | 36.67 | B | C |
| ATOM | 4341 | CB | GLN | B | 254 | 37.826 | 44.867 | 76.724 | 1.00 | 49.98 | B | C |
| ATOM | 4342 | CG | GLN | B | 254 | 38.331 | 43.499 | 77.091 | 1.00 | 49.98 | B | C |
| ATOM | 4343 | CD | GLN | B | 254 | 39.117 | 42.869 | 75.967 | 1.00 | 49.98 | B | C |
| ATOM | 4344 | OE1 | GLN | B | 254 | 39.275 | 41.650 | 75.908 | 1.00 | 49.98 | B | O |
| ATOM | 4345 | NE2 | GLN | B | 254 | 39.622 | 43.699 | 75.067 | 1.00 | 49.98 | B | N |
| ATOM | 4346 | C | GLN | B | 254 | 35.676 | 44.448 | 77.952 | 1.00 | 36.67 | B | C |
| ATOM | 4347 | O | GLN | B | 254 | 35.648 | 43.741 | 78.956 | 1.00 | 36.67 | B | O |
| ATOM | 4348 | N | PRO | B | 255 | 34.720 | 44.378 | 77.019 | 1.00 | 27.71 | B | N |
| ATOM | 4349 | CD | PRO | B | 255 | 34.641 | 45.102 | 75.746 | 1.00 | 14.96 | B | C |

| | | | | | | | | | | | | |
|---|---|---|---|---|---|---|---|---|---|---|---|---|
| ATOM | 4350 | CA | PRO | B | 255 | 33.584 | 43.464 | 77.169 | 1.00 | 27.71 | B | C |
| ATOM | 4351 | CB | PRO | B | 255 | 32.782 | 43.714 | 75.892 | 1.00 | 14.96 | B | C |
| ATOM | 4352 | CG | PRO | B | 255 | 33.171 | 45.103 | 75.497 | 1.00 | 14.96 | B | C |
| ATOM | 4353 | C | PRO | B | 255 | 33.993 | 41.999 | 77.318 | 1.00 | 27.71 | B | C |
| ATOM | 4354 | O | PRO | B | 255 | 34.933 | 41.553 | 76.673 | 1.00 | 27.71 | B | O |
| ATOM | 4355 | N | ILE | B | 256 | 33.287 | 41.248 | 78.154 | 1.00 | 35.36 | B | N |
| ATOM | 4356 | CA | ILE | B | 256 | 33.615 | 39.838 | 78.345 | 1.00 | 35.36 | B | C |
| ATOM | 4357 | CB | ILE | B | 256 | 33.237 | 39.316 | 79.757 | 1.00 | 31.23 | B | C |
| ATOM | 4358 | CG2 | ILE | B | 256 | 31.719 | 39.403 | 79.984 | 1.00 | 31.23 | B | C |
| ATOM | 4359 | CG1 | ILE | B | 256 | 33.694 | 37.858 | 79.894 | 1.00 | 31.23 | B | C |
| ATOM | 4360 | CD1 | ILE | B | 256 | 33.231 | 37.154 | 81.175 | 1.00 | 31.23 | B | C |
| ATOM | 4361 | C | ILE | B | 256 | 32.947 | 38.908 | 77.339 | 1.00 | 35.36 | B | C |
| ATOM | 4362 | O | ILE | B | 256 | 33.496 | 37.866 | 77.030 | 1.00 | 35.36 | B | O |
| ATOM | 4363 | N | PHE | B | 257 | 31.757 | 39.252 | 76.855 | 1.00 | 30.96 | B | N |
| ATOM | 4364 | CA | PHE | B | 257 | 31.056 | 38.400 | 75.886 | 1.00 | 30.96 | B | C |
| ATOM | 4365 | CB | PHE | B | 257 | 29.784 | 37.824 | 76.503 | 1.00 | 18.54 | B | C |
| ATOM | 4366 | CG | PHE | B | 257 | 30.029 | 36.944 | 77.673 | 1.00 | 18.54 | B | C |
| ATOM | 4367 | CD1 | PHE | B | 257 | 31.022 | 35.972 | 77.636 | 1.00 | 18.54 | B | C |
| ATOM | 4368 | CD2 | PHE | B | 257 | 29.285 | 37.098 | 78.833 | 1.00 | 18.54 | B | C |
| ATOM | 4369 | CE1 | PHE | B | 257 | 31.272 | 35.161 | 78.752 | 1.00 | 18.54 | B | C |
| ATOM | 4370 | CE2 | PHE | B | 257 | 29.520 | 36.302 | 79.943 | 1.00 | 18.54 | B | C |
| ATOM | 4371 | CZ | PHE | B | 257 | 30.520 | 35.331 | 79.907 | 1.00 | 18.54 | B | C |
| ATOM | 4372 | C | PHE | B | 257 | 30.675 | 39.126 | 74.581 | 1.00 | 30.96 | B | C |
| ATOM | 4373 | O | PHE | B | 257 | 29.482 | 39.250 | 74.250 | 1.00 | 30.96 | B | O |
| ATOM | 4374 | N | PRO | B | 258 | 31.679 | 39.606 | 73.819 | 1.00 | 29.82 | B | N |
| ATOM | 4375 | CD | PRO | B | 258 | 33.133 | 39.501 | 74.045 | 1.00 | 20.52 | B | C |
| ATOM | 4376 | CA | PRO | B | 258 | 31.422 | 40.314 | 72.566 | 1.00 | 29.82 | B | C |
| ATOM | 4377 | CB | PRO | B | 258 | 32.745 | 41.001 | 72.290 | 1.00 | 20.52 | B | C |
| ATOM | 4378 | CG | PRO | B | 258 | 33.708 | 39.964 | 72.713 | 1.00 | 20.52 | B | C |
| ATOM | 4379 | C | PRO | B | 258 | 31.041 | 39.382 | 71.427 | 1.00 | 29.82 | B | C |
| ATOM | 4380 | O | PRO | B | 258 | 31.069 | 38.157 | 71.564 | 1.00 | 29.82 | B | O |
| ATOM | 4381 | N | GLY | B | 259 | 30.701 | 39.980 | 70.297 | 1.00 | 29.48 | B | N |
| ATOM | 4382 | CA | GLY | B | 259 | 30.314 | 39.206 | 69.138 | 1.00 | 29.48 | B | C |
| ATOM | 4383 | C | GLY | B | 259 | 29.259 | 39.941 | 68.336 | 1.00 | 29.48 | B | C |
| ATOM | 4384 | O | GLY | B | 259 | 28.322 | 40.500 | 68.896 | 1.00 | 29.48 | B | O |
| ATOM | 4385 | N | ASP | B | 260 | 29.395 | 39.956 | 67.022 | 1.00 | 28.45 | B | N |
| ATOM | 4386 | CA | ASP | B | 260 | 28.403 | 40.642 | 66.213 | 1.00 | 28.45 | B | C |
| ATOM | 4387 | CB | ASP | B | 260 | 28.960 | 40.947 | 64.817 | 1.00 | 53.75 | B | C |
| ATOM | 4388 | CG | ASP | B | 260 | 30.035 | 42.037 | 64.837 | 1.00 | 53.75 | B | C |
| ATOM | 4389 | OD1 | ASP | B | 260 | 29.983 | 42.893 | 65.756 | 1.00 | 53.75 | B | O |
| ATOM | 4390 | OD2 | ASP | B | 260 | 30.909 | 42.039 | 63.926 | 1.00 | 53.75 | B | O |
| ATOM | 4391 | C | ASP | B | 260 | 27.095 | 39.865 | 66.083 | 1.00 | 28.45 | B | C |
| ATOM | 4392 | O | ASP | B | 260 | 26.079 | 40.424 | 65.673 | 1.00 | 28.45 | B | O |
| ATOM | 4393 | N | SER | B | 261 | 27.117 | 38.576 | 66.414 | 1.00 | 25.64 | B | N |
| ATOM | 4394 | CA | SER | B | 261 | 25.907 | 37.767 | 66.322 | 1.00 | 25.64 | B | C |
| ATOM | 4395 | CB | SER | B | 261 | 25.971 | 36.814 | 65.128 | 1.00 | 18.81 | B | C |
| ATOM | 4396 | OG | SER | B | 261 | 26.696 | 35.640 | 65.440 | 1.00 | 18.81 | B | O |
| ATOM | 4397 | C | SER | B | 261 | 25.704 | 36.959 | 67.580 | 1.00 | 25.64 | B | C |
| ATOM | 4398 | O | SER | B | 261 | 26.633 | 36.779 | 68.360 | 1.00 | 25.64 | B | O |
| ATOM | 4399 | N | GLY | B | 262 | 24.478 | 36.477 | 67.773 | 1.00 | 27.61 | B | N |
| ATOM | 4400 | CA | GLY | B | 262 | 24.178 | 35.676 | 68.941 | 1.00 | 27.61 | B | C |
| ATOM | 4401 | C | GLY | B | 262 | 25.154 | 34.525 | 68.991 | 1.00 | 27.61 | B | C |
| ATOM | 4402 | O | GLY | B | 262 | 25.727 | 34.217 | 70.041 | 1.00 | 27.61 | B | O |
| ATOM | 4403 | N | VAL | B | 263 | 25.340 | 33.892 | 67.838 | 1.00 | 21.64 | B | N |
| ATOM | 4404 | CA | VAL | B | 263 | 26.257 | 32.772 | 67.706 | 1.00 | 21.64 | B | C |
| ATOM | 4405 | CB | VAL | B | 263 | 26.490 | 32.402 | 66.231 | 1.00 | 35.29 | B | C |
| ATOM | 4406 | CG1 | VAL | B | 263 | 27.649 | 31.418 | 66.118 | 1.00 | 35.29 | B | C |
| ATOM | 4407 | CG2 | VAL | B | 263 | 25.234 | 31.782 | 65.656 | 1.00 | 35.29 | B | C |
| ATOM | 4408 | C | VAL | B | 263 | 27.607 | 33.114 | 68.306 | 1.00 | 21.64 | B | C |
| ATOM | 4409 | O | VAL | B | 263 | 28.113 | 32.404 | 69.166 | 1.00 | 21.64 | B | O |
| ATOM | 4410 | N | ASP | B | 264 | 28.194 | 34.204 | 67.836 | 1.00 | 35.19 | B | N |
| ATOM | 4411 | CA | ASP | B | 264 | 29.497 | 34.627 | 68.333 | 1.00 | 35.19 | B | C |
| ATOM | 4412 | CB | ASP | B | 264 | 29.948 | 35.884 | 67.589 | 1.00 | 37.53 | B | C |
| ATOM | 4413 | CG | ASP | B | 264 | 30.257 | 35.612 | 66.144 | 1.00 | 37.53 | B | C |
| ATOM | 4414 | OD1 | ASP | B | 264 | 30.271 | 34.421 | 65.777 | 1.00 | 37.53 | B | O |
| ATOM | 4415 | OD2 | ASP | B | 264 | 30.489 | 36.578 | 65.385 | 1.00 | 37.53 | B | O |
| ATOM | 4416 | C | ASP | B | 264 | 29.502 | 34.876 | 69.846 | 1.00 | 35.19 | B | C |

| | | | | | | | | | | | | |
|---|---|---|---|---|---|---|---|---|---|---|---|---|
| ATOM | 4417 | O | ASP | B | 264 | 30.421 | 34.462 | 70.551 | 1.00 | 35.19 | B | O |
| ATOM | 4418 | N | GLN | B | 265 | 28.477 | 35.552 | 70.340 | 1.00 | 26.25 | B | N |
| ATOM | 4419 | CA | GLN | B | 265 | 28.384 | 35.831 | 71.757 | 1.00 | 26.25 | B | C |
| ATOM | 4420 | CB | GLN | B | 265 | 27.134 | 36.643 | 72.051 | 1.00 | 36.18 | B | C |
| ATOM | 4421 | CG | GLN | B | 265 | 26.959 | 37.862 | 71.176 | 1.00 | 36.18 | B | C |
| ATOM | 4422 | CD | GLN | B | 265 | 25.700 | 38.610 | 71.505 | 1.00 | 36.18 | B | C |
| ATOM | 4423 | OE1 | GLN | B | 265 | 25.007 | 39.085 | 70.613 | 1.00 | 36.18 | B | O |
| ATOM | 4424 | NE2 | GLN | B | 265 | 25.385 | 38.716 | 72.799 | 1.00 | 36.18 | B | N |
| ATOM | 4425 | C | GLN | B | 265 | 28.320 | 34.524 | 72.505 | 1.00 | 26.25 | B | C |
| ATOM | 4426 | O | GLN | B | 265 | 28.939 | 34.380 | 73.550 | 1.00 | 26.25 | B | O |
| ATOM | 4427 | N | LEU | B | 266 | 27.560 | 33.576 | 71.968 | 1.00 | 28.90 | B | N |
| ATOM | 4428 | CA | LEU | B | 266 | 27.430 | 32.265 | 72.582 | 1.00 | 28.90 | B | C |
| ATOM | 4429 | CB | LEU | B | 266 | 26.402 | 31.449 | 71.808 | 1.00 | 22.14 | B | C |
| ATOM | 4430 | CG | LEU | B | 266 | 24.981 | 31.434 | 72.380 | 1.00 | 22.14 | B | C |
| ATOM | 4431 | CD1 | LEU | B | 266 | 24.590 | 32.808 | 72.880 | 1.00 | 22.14 | B | C |
| ATOM | 4432 | CD2 | LEU | B | 266 | 24.003 | 30.947 | 71.318 | 1.00 | 22.14 | B | C |
| ATOM | 4433 | C | LEU | B | 266 | 28.770 | 31.528 | 72.617 | 1.00 | 28.90 | B | C |
| ATOM | 4434 | O | LEU | B | 266 | 29.080 | 30.839 | 73.590 | 1.00 | 28.90 | B | O |
| ATOM | 4435 | N | VAL | B | 267 | 29.578 | 31.664 | 71.571 | 1.00 | 36.65 | B | N |
| ATOM | 4436 | CA | VAL | B | 267 | 30.861 | 30.976 | 71.583 | 1.00 | 36.65 | B | C |
| ATOM | 4437 | CB | VAL | B | 267 | 31.597 | 30.985 | 70.178 | 1.00 | 33.45 | B | C |
| ATOM | 4438 | CG1 | VAL | B | 267 | 30.647 | 31.414 | 69.082 | 1.00 | 33.45 | B | C |
| ATOM | 4439 | CG2 | VAL | B | 267 | 32.845 | 31.871 | 70.203 | 1.00 | 33.45 | B | C |
| ATOM | 4440 | C | VAL | B | 267 | 31.760 | 31.607 | 72.627 | 1.00 | 36.65 | B | C |
| ATOM | 4441 | O | VAL | B | 267 | 32.619 | 30.939 | 73.190 | 1.00 | 36.65 | B | O |
| ATOM | 4442 | N | GLU | B | 268 | 31.555 | 32.893 | 72.884 | 1.00 | 30.24 | B | N |
| ATOM | 4443 | CA | GLU | B | 268 | 32.357 | 33.607 | 73.857 | 1.00 | 30.24 | B | C |
| ATOM | 4444 | CB | GLU | B | 268 | 32.252 | 35.106 | 73.601 | 1.00 | 43.19 | B | C |
| ATOM | 4445 | CG | GLU | B | 268 | 32.986 | 35.486 | 72.328 | 1.00 | 43.19 | B | C |
| ATOM | 4446 | CD | GLU | B | 268 | 34.418 | 34.998 | 72.371 | 1.00 | 43.19 | B | C |
| ATOM | 4447 | OE1 | GLU | B | 268 | 34.990 | 34.732 | 71.285 | 1.00 | 43.19 | B | O |
| ATOM | 4448 | OE2 | GLU | B | 268 | 34.954 | 34.885 | 73.504 | 1.00 | 43.19 | B | O |
| ATOM | 4449 | C | GLU | B | 268 | 31.941 | 33.257 | 75.265 | 1.00 | 30.24 | B | C |
| ATOM | 4450 | O | GLU | B | 268 | 32.783 | 33.187 | 76.156 | 1.00 | 30.24 | B | O |
| ATOM | 4451 | N | ILE | B | 269 | 30.647 | 33.021 | 75.457 | 1.00 | 43.79 | B | N |
| ATOM | 4452 | CA | ILE | B | 269 | 30.123 | 32.651 | 76.766 | 1.00 | 43.79 | B | C |
| ATOM | 4453 | CB | ILE | B | 269 | 28.582 | 32.701 | 76.794 | 1.00 | 24.38 | B | C |
| ATOM | 4454 | CG2 | ILE | B | 269 | 28.059 | 32.012 | 78.038 | 1.00 | 24.38 | B | C |
| ATOM | 4455 | CG1 | ILE | B | 269 | 28.114 | 34.154 | 76.725 | 1.00 | 24.38 | B | C |
| ATOM | 4456 | CD1 | ILE | B | 269 | 26.625 | 34.309 | 76.585 | 1.00 | 24.38 | B | C |
| ATOM | 4457 | C | ILE | B | 269 | 30.576 | 31.236 | 77.094 | 1.00 | 43.79 | B | C |
| ATOM | 4458 | O | ILE | B | 269 | 31.082 | 30.979 | 78.183 | 1.00 | 43.79 | B | O |
| ATOM | 4459 | N | ILE | B | 270 | 30.404 | 30.326 | 76.140 | 1.00 | 32.45 | B | N |
| ATOM | 4460 | CA | ILE | B | 270 | 30.792 | 28.936 | 76.323 | 1.00 | 32.45 | B | C |
| ATOM | 4461 | CB | ILE | B | 270 | 30.422 | 28.098 | 75.079 | 1.00 | 27.53 | B | C |
| ATOM | 4462 | CG2 | ILE | B | 270 | 30.912 | 26.665 | 75.243 | 1.00 | 27.53 | B | C |
| ATOM | 4463 | CG1 | ILE | B | 270 | 28.905 | 28.124 | 74.878 | 1.00 | 27.53 | B | C |
| ATOM | 4464 | CD1 | ILE | B | 270 | 28.418 | 27.343 | 73.693 | 1.00 | 27.53 | B | C |
| ATOM | 4465 | C | ILE | B | 270 | 32.282 | 28.833 | 76.575 | 1.00 | 32.45 | B | C |
| ATOM | 4466 | O | ILE | B | 270 | 32.726 | 27.971 | 77.331 | 1.00 | 32.45 | B | O |
| ATOM | 4467 | N | LYS | B | 271 | 33.058 | 29.711 | 75.948 | 1.00 | 35.52 | B | N |
| ATOM | 4468 | CA | LYS | B | 271 | 34.501 | 29.677 | 76.154 | 1.00 | 35.52 | B | C |
| ATOM | 4469 | CB | LYS | B | 271 | 35.207 | 30.772 | 75.348 | 1.00 | 46.41 | B | C |
| ATOM | 4470 | CG | LYS | B | 271 | 35.502 | 30.399 | 73.904 | 1.00 | 46.41 | B | C |
| ATOM | 4471 | CD | LYS | B | 271 | 36.294 | 31.492 | 73.182 | 1.00 | 46.41 | B | C |
| ATOM | 4472 | CE | LYS | B | 271 | 36.113 | 31.386 | 71.664 | 1.00 | 46.41 | B | C |
| ATOM | 4473 | NZ | LYS | B | 271 | 36.641 | 32.582 | 70.894 | 1.00 | 46.41 | B | N |
| ATOM | 4474 | C | LYS | B | 271 | 34.836 | 29.826 | 77.635 | 1.00 | 35.52 | B | C |
| ATOM | 4475 | O | LYS | B | 271 | 35.795 | 29.212 | 78.122 | 1.00 | 35.52 | B | O |
| ATOM | 4476 | N | VAL | B | 272 | 34.045 | 30.612 | 78.366 | 1.00 | 38.83 | B | N |
| ATOM | 4477 | CA | VAL | B | 272 | 34.333 | 30.788 | 79.783 | 1.00 | 38.83 | B | C |
| ATOM | 4478 | CB | VAL | B | 272 | 34.400 | 32.310 | 80.166 | 1.00 | 20.73 | B | C |
| ATOM | 4479 | CG1 | VAL | B | 272 | 34.199 | 33.170 | 78.938 | 1.00 | 20.73 | B | C |
| ATOM | 4480 | CG2 | VAL | B | 272 | 33.389 | 32.643 | 81.253 | 1.00 | 20.73 | B | C |
| ATOM | 4481 | C | VAL | B | 272 | 33.431 | 30.037 | 80.757 | 1.00 | 38.83 | B | C |
| ATOM | 4482 | O | VAL | B | 272 | 33.908 | 29.619 | 81.805 | 1.00 | 38.83 | B | O |
| ATOM | 4483 | N | LEU | B | 273 | 32.152 | 29.853 | 80.427 | 1.00 | 33.81 | B | N |

| | | | | | | | | | | | | |
|---|---|---|---|---|---|---|---|---|---|---|---|---|
| ATOM | 4484 | CA | LEU | B | 273 | 31.221 | 29.135 | 81.312 | 1.00 | 33.81 | B | C |
| ATOM | 4485 | CB | LEU | B | 273 | 29.821 | 29.739 | 81.259 | 1.00 | 23.99 | B | C |
| ATOM | 4486 | CG | LEU | B | 273 | 29.511 | 31.133 | 81.778 | 1.00 | 23.99 | B | C |
| ATOM | 4487 | CD1 | LEU | B | 273 | 28.015 | 31.315 | 81.666 | 1.00 | 23.99 | B | C |
| ATOM | 4488 | CD2 | LEU | B | 273 | 29.959 | 31.301 | 83.225 | 1.00 | 23.99 | B | C |
| ATOM | 4489 | C | LEU | B | 273 | 31.083 | 27.693 | 80.868 | 1.00 | 33.81 | B | C |
| ATOM | 4490 | O | LEU | B | 273 | 30.228 | 26.962 | 81.364 | 1.00 | 33.81 | B | O |
| ATOM | 4491 | N | GLY | B | 274 | 31.900 | 27.278 | 79.916 | 1.00 | 41.43 | B | N |
| ATOM | 4492 | CA | GLY | B | 274 | 31.780 | 25.914 | 79.448 | 1.00 | 41.43 | B | C |
| ATOM | 4493 | C | GLY | B | 274 | 30.527 | 25.713 | 78.611 | 1.00 | 41.43 | B | C |
| ATOM | 4494 | O | GLY | B | 274 | 29.763 | 26.646 | 78.379 | 1.00 | 41.43 | B | O |
| ATOM | 4495 | N | THR | B | 275 | 30.310 | 24.493 | 78.150 | 1.00 | 32.47 | B | N |
| ATOM | 4496 | CA | THR | B | 275 | 29.153 | 24.216 | 77.331 | 1.00 | 32.47 | B | C |
| ATOM | 4497 | CB | THR | B | 275 | 29.485 | 23.128 | 76.314 | 1.00 | 44.57 | B | C |
| ATOM | 4498 | OG1 | THR | B | 275 | 30.903 | 23.117 | 76.108 | 1.00 | 44.57 | B | O |
| ATOM | 4499 | CG2 | THR | B | 275 | 28.789 | 23.403 | 74.986 | 1.00 | 44.57 | B | C |
| ATOM | 4500 | C | THR | B | 275 | 27.959 | 23.826 | 78.203 | 1.00 | 32.47 | B | C |
| ATOM | 4501 | O | THR | B | 275 | 27.990 | 22.857 | 78.957 | 1.00 | 32.47 | B | O |
| ATOM | 4502 | N | PRO | B | 276 | 26.877 | 24.588 | 78.098 | 1.00 | 66.38 | B | N |
| ATOM | 4503 | CD | PRO | B | 276 | 26.539 | 25.537 | 77.022 | 1.00 | 42.61 | B | C |
| ATOM | 4504 | CA | PRO | B | 276 | 25.700 | 24.279 | 78.908 | 1.00 | 66.38 | B | C |
| ATOM | 4505 | CB | PRO | B | 276 | 24.622 | 25.209 | 78.345 | 1.00 | 42.61 | B | C |
| ATOM | 4506 | CG | PRO | B | 276 | 25.017 | 25.363 | 76.918 | 1.00 | 42.61 | B | C |
| ATOM | 4507 | C | PRO | B | 276 | 25.282 | 22.832 | 78.856 | 1.00 | 66.38 | B | C |
| ATOM | 4508 | O | PRO | B | 276 | 25.439 | 22.133 | 77.847 | 1.00 | 66.38 | B | O |
| ATOM | 4509 | N | THR | B | 277 | 24.722 | 22.409 | 79.969 | 1.00 | 48.21 | B | N |
| ATOM | 4510 | CA | THR | B | 277 | 24.238 | 21.070 | 80.113 | 1.00 | 48.21 | B | C |
| ATOM | 4511 | CB | THR | B | 277 | 24.134 | 20.733 | 81.563 | 1.00 | 43.89 | B | C |
| ATOM | 4512 | OG1 | THR | B | 277 | 22.881 | 21.204 | 82.063 | 1.00 | 43.89 | B | O |
| ATOM | 4513 | CG2 | THR | B | 277 | 25.262 | 21.442 | 82.323 | 0.00 | 43.89 | B | C |
| ATOM | 4514 | C | THR | B | 277 | 22.847 | 21.161 | 79.538 | 1.00 | 48.21 | B | C |
| ATOM | 4515 | O | THR | B | 277 | 22.262 | 22.253 | 79.474 | 1.00 | 48.21 | B | O |
| ATOM | 4516 | N | ALA | B | 278 | 22.312 | 20.014 | 79.150 | 1.00 | 55.97 | B | N |
| ATOM | 4517 | CA | ALA | B | 278 | 20.981 | 19.951 | 78.574 | 1.00 | 55.97 | B | C |
| ATOM | 4518 | CB | ALA | B | 278 | 20.574 | 18.504 | 78.384 | 1.00 | 43.89 | B | C |
| ATOM | 4519 | C | ALA | B | 278 | 19.995 | 20.654 | 79.481 | 1.00 | 55.97 | B | C |
| ATOM | 4520 | O | ALA | B | 278 | 19.233 | 21.533 | 79.043 | 1.00 | 55.97 | B | O |
| ATOM | 4521 | N | GLU | B | 279 | 20.026 | 20.293 | 80.753 | 0.00 | 61.38 | B | N |
| ATOM | 4522 | CA | GLU | B | 279 | 19.099 | 20.909 | 81.680 | 1.00 | 61.38 | B | C |
| ATOM | 4523 | CB | GLU | B | 279 | 19.356 | 20.419 | 83.109 | 1.00 | 77.90 | B | C |
| ATOM | 4524 | CG | GLU | B | 279 | 20.294 | 21.303 | 83.906 | 1.00 | 77.90 | B | C |
| ATOM | 4525 | CD | GLU | B | 279 | 21.306 | 20.494 | 84.717 | 1.00 | 77.90 | B | C |
| ATOM | 4526 | OE1 | GLU | B | 279 | 20.901 | 19.845 | 85.726 | 1.00 | 77.90 | B | O |
| ATOM | 4527 | OE2 | GLU | B | 279 | 22.508 | 20.510 | 84.330 | 1.00 | 77.90 | B | O |
| ATOM | 4528 | C | GLU | B | 279 | 19.181 | 22.445 | 81.623 | 1.00 | 61.38 | B | C |
| ATOM | 4529 | O | GLU | B | 279 | 18.151 | 23.133 | 81.487 | 1.00 | 61.38 | B | O |
| ATOM | 4530 | N | GLN | B | 280 | 20.403 | 22.972 | 81.713 | 1.00 | 47.11 | B | N |
| ATOM | 4531 | CA | GLN | B | 280 | 20.618 | 24.409 | 81.707 | 1.00 | 47.11 | B | C |
| ATOM | 4532 | CB | GLN | B | 280 | 22.109 | 24.697 | 81.650 | 1.00 | 48.45 | B | C |
| ATOM | 4533 | CG | GLN | B | 280 | 22.822 | 24.509 | 82.973 | 1.00 | 48.45 | B | C |
| ATOM | 4534 | CD | GLN | B | 280 | 24.331 | 24.554 | 82.796 | 1.00 | 48.45 | B | C |
| ATOM | 4535 | OE1 | GLN | B | 280 | 25.095 | 24.612 | 83.772 | 1.00 | 48.45 | B | O |
| ATOM | 4536 | NE2 | GLN | B | 280 | 24.775 | 24.523 | 81.532 | 1.00 | 48.45 | B | N |
| ATOM | 4537 | C | GLN | B | 280 | 19.905 | 25.094 | 80.558 | 1.00 | 47.11 | B | C |
| ATOM | 4538 | O | GLN | B | 280 | 18.986 | 25.886 | 80.766 | 0.00 | 47.11 | B | O |
| ATOM | 4539 | N | ILE | B | 281 | 20.313 | 24.760 | 79.341 | 1.00 | 54.00 | B | N |
| ATOM | 4540 | CA | ILE | B | 281 | 19.711 | 25.354 | 78.157 | 1.00 | 54.00 | B | C |
| ATOM | 4541 | CB | ILE | B | 281 | 20.303 | 24.747 | 76.871 | 0.00 | 31.68 | B | C |
| ATOM | 4542 | CG2 | ILE | B | 281 | 21.555 | 23.967 | 77.178 | 1.00 | 31.68 | B | C |
| ATOM | 4543 | CG1 | ILE | B | 281 | 19.305 | 23.789 | 76.239 | 1.00 | 31.68 | B | C |
| ATOM | 4544 | CD1 | ILE | B | 281 | 19.760 | 23.279 | 74.883 | 1.00 | 31.68 | B | C |
| ATOM | 4545 | C | ILE | B | 281 | 18.184 | 25.186 | 78.163 | 1.00 | 54.00 | B | C |
| ATOM | 4546 | O | ILE | B | 281 | 17.468 | 25.969 | 77.525 | 1.00 | 54.00 | B | O |
| ATOM | 4547 | N | ALA | B | 282 | 17.687 | 24.172 | 78.876 | 1.00 | 38.46 | B | N |
| ATOM | 4548 | CA | ALA | B | 282 | 16.242 | 23.956 | 78.963 | 1.00 | 38.46 | B | C |
| ATOM | 4549 | CB | ALA | B | 282 | 15.953 | 22.567 | 79.448 | 1.00 | 58.43 | B | C |
| ATOM | 4550 | C | ALA | B | 282 | 15.669 | 24.965 | 79.952 | 1.00 | 38.46 | B | C |

| | | | | | | | | | | | | |
|---|---|---|---|---|---|---|---|---|---|---|---|---|
| ATOM | 4551 | O | ALA | B | 282 | 14.463 | 25.041 | 80.177 | 1.00 | 38.46 | B | O |
| ATOM | 4552 | N | GLU | B | 283 | 16.560 | 25.740 | 80.549 | 1.00 | 60.70 | B | N |
| ATOM | 4553 | CA | GLU | B | 283 | 16.168 | 26.747 | 81.510 | 1.00 | 60.70 | B | C |
| ATOM | 4554 | CB | GLU | B | 283 | 17.058 | 26.699 | 82.708 | 1.00 | 75.14 | B | C |
| ATOM | 4555 | CG | GLU | B | 283 | 16.489 | 25.802 | 83.710 | 1.00 | 75.14 | B | C |
| ATOM | 4556 | CD | GLU | B | 283 | 17.512 | 24.820 | 84.253 | 1.00 | 75.14 | B | C |
| ATOM | 4557 | OE1 | GLU | B | 283 | 17.283 | 24.272 | 85.366 | 1.00 | 75.14 | B | O |
| ATOM | 4558 | OE2 | GLU | B | 283 | 18.546 | 24.586 | 83.572 | 1.00 | 75.14 | B | O |
| ATOM | 4559 | C | GLU | B | 283 | 16.246 | 28.122 | 80.953 | 1.00 | 60.70 | B | C |
| ATOM | 4560 | O | GLU | B | 283 | 15.736 | 29.061 | 81.559 | 1.00 | 60.70 | B | O |
| ATOM | 4561 | N | MET | B | 284 | 16.916 | 28.233 | 79.812 | 1.00 | 46.66 | B | N |
| ATOM | 4562 | CA | MET | B | 284 | 17.080 | 29.499 | 79.134 | 1.00 | 46.66 | B | C |
| ATOM | 4563 | CB | MET | B | 284 | 18.509 | 29.581 | 78.631 | 0.00 | 51.84 | B | C |
| ATOM | 4564 | CG | MET | B | 284 | 19.480 | 29.465 | 79.775 | 1.00 | 51.84 | B | C |
| ATOM | 4565 | SD | MET | B | 284 | 21.001 | 28.637 | 79.214 | 1.00 | 51.84 | B | S |
| ATOM | 4566 | CE | MET | B | 284 | 21.753 | 29.973 | 78.493 | 1.00 | 51.84 | B | C |
| ATOM | 4567 | C | MET | B | 284 | 16.076 | 29.705 | 77.993 | 1.00 | 46.66 | B | C |
| ATOM | 4568 | O | MET | B | 284 | 15.461 | 28.758 | 77.483 | 1.00 | 46.66 | B | O |
| ATOM | 4569 | N | ALA | B | 293 | 25.450 | 23.481 | 69.172 | 1.00 | 55.70 | B | N |
| ATOM | 4570 | CA | ALA | B | 293 | 26.651 | 22.854 | 68.621 | 1.00 | 55.70 | B | C |
| ATOM | 4571 | CB | ALA | B | 293 | 27.248 | 23.768 | 67.539 | 1.00 | 67.37 | B | C |
| ATOM | 4572 | C | ALA | B | 293 | 27.727 | 22.504 | 69.679 | 1.00 | 55.70 | B | C |
| ATOM | 4573 | O | ALA | B | 293 | 27.412 | 21.987 | 70.756 | 1.00 | 55.70 | B | O |
| ATOM | 4574 | N | ALA | B | 294 | 28.984 | 22.802 | 69.322 | 1.00 | 61.42 | B | N |
| ATOM | 4575 | CA | ALA | B | 294 | 30.218 | 22.603 | 70.109 | 1.00 | 61.42 | B | C |
| ATOM | 4576 | CB | ALA | B | 294 | 30.927 | 23.940 | 70.241 | 1.00 | 23.63 | B | C |
| ATOM | 4577 | C | ALA | B | 294 | 30.153 | 21.923 | 71.489 | 1.00 | 61.42 | B | C |
| ATOM | 4578 | O | ALA | B | 294 | 29.105 | 21.410 | 71.900 | 1.00 | 61.42 | B | O |
| ATOM | 4579 | N | ALA | B | 296 | 31.288 | 21.939 | 72.203 | 1.00 | 49.92 | B | N |
| ATOM | 4580 | CA | ALA | B | 296 | 31.384 | 21.309 | 73.533 | 1.00 | 49.92 | B | C |
| ATOM | 4581 | CB | ALA | B | 296 | 31.238 | 19.760 | 73.385 | 1.00 | 14.54 | B | C |
| ATOM | 4582 | C | ALA | B | 296 | 32.647 | 21.629 | 74.371 | 1.00 | 49.92 | B | C |
| ATOM | 4583 | O | ALA | B | 296 | 33.342 | 20.706 | 74.798 | 1.00 | 49.92 | B | O |
| ATOM | 4584 | N | ALA | B | 297 | 32.915 | 22.914 | 74.630 | 1.00 | 64.10 | B | N |
| ATOM | 4585 | CA | ALA | B | 297 | 34.096 | 23.362 | 75.407 | 1.00 | 64.10 | B | C |
| ATOM | 4586 | CB | ALA | B | 297 | 34.338 | 24.859 | 75.159 | 1.00 | 17.11 | B | C |
| ATOM | 4587 | C | ALA | B | 297 | 34.049 | 23.087 | 76.929 | 1.00 | 64.10 | B | C |
| ATOM | 4588 | O | ALA | B | 297 | 33.269 | 22.251 | 77.370 | 1.00 | 64.10 | B | O |
| ATOM | 4589 | N | ALA | B | 298 | 34.901 | 23.780 | 77.705 | 1.00 | 63.90 | B | N |
| ATOM | 4590 | CA | ALA | B | 298 | 34.997 | 23.661 | 79.182 | 1.00 | 63.90 | B | C |
| ATOM | 4591 | CB | ALA | B | 298 | 36.024 | 22.597 | 79.564 | 1.00 | 57.47 | B | C |
| ATOM | 4592 | C | ALA | B | 298 | 35.386 | 25.046 | 79.771 | 1.00 | 63.90 | B | C |
| ATOM | 4593 | O | ALA | B | 298 | 35.829 | 25.906 | 79.008 | 1.00 | 63.90 | B | O |
| ATOM | 4594 | N | ALA | B | 299 | 35.273 | 25.258 | 81.093 | 1.00 | 41.08 | B | N |
| ATOM | 4595 | CA | ALA | B | 299 | 35.492 | 26.594 | 81.726 | 1.00 | 41.08 | B | C |
| ATOM | 4596 | CB | ALA | B | 299 | 34.662 | 26.625 | 82.976 | 1.00 | 53.01 | B | C |
| ATOM | 4597 | C | ALA | B | 299 | 36.856 | 27.314 | 81.993 | 1.00 | 41.08 | B | C |
| ATOM | 4598 | O | ALA | B | 299 | 37.813 | 27.099 | 81.258 | 1.00 | 41.08 | B | O |
| ATOM | 4599 | N | ALA | B | 300 | 36.911 | 28.216 | 83.003 | 1.00 | 44.10 | B | N |
| ATOM | 4600 | CA | ALA | B | 300 | 38.147 | 28.958 | 83.333 | 1.00 | 44.10 | B | C |
| ATOM | 4601 | CB | ALA | B | 300 | 38.749 | 29.483 | 82.019 | 1.00 | 27.81 | B | C |
| ATOM | 4602 | C | ALA | B | 300 | 38.237 | 30.098 | 84.420 | 1.00 | 44.10 | B | C |
| ATOM | 4603 | O | ALA | B | 300 | 39.339 | 30.486 | 84.812 | 1.00 | 44.10 | B | O |
| ATOM | 4604 | N | TRP | B | 301 | 37.107 | 30.633 | 84.900 | 1.00 | 60.18 | B | N |
| ATOM | 4605 | CA | TRP | B | 301 | 37.072 | 31.808 | 85.838 | 1.00 | 60.18 | B | C |
| ATOM | 4606 | CB | TRP | B | 301 | 36.069 | 31.604 | 86.980 | 1.00 | 33.08 | B | C |
| ATOM | 4607 | CG | TRP | B | 301 | 34.627 | 31.926 | 86.573 | 1.00 | 33.08 | B | C |
| ATOM | 4608 | CD2 | TRP | B | 301 | 34.179 | 33.044 | 85.788 | 1.00 | 33.08 | B | C |
| ATOM | 4609 | CE2 | TRP | B | 301 | 32.804 | 32.846 | 85.515 | 1.00 | 33.08 | B | C |
| ATOM | 4610 | CE3 | TRP | B | 301 | 34.810 | 34.186 | 85.274 | 1.00 | 33.08 | B | C |
| ATOM | 4611 | CD1 | TRP | B | 301 | 33.520 | 31.131 | 86.763 | 1.00 | 33.08 | B | C |
| ATOM | 4612 | NE1 | TRP | B | 301 | 32.426 | 31.676 | 86.126 | 1.00 | 33.08 | B | N |
| ATOM | 4613 | CZ2 | TRP | B | 301 | 32.051 | 33.747 | 84.755 | 1.00 | 33.08 | B | C |
| ATOM | 4614 | CZ3 | TRP | B | 301 | 34.062 | 35.082 | 84.516 | 1.00 | 33.08 | B | C |
| ATOM | 4615 | CH2 | TRP | B | 301 | 32.697 | 34.853 | 84.261 | 1.00 | 33.08 | B | C |
| ATOM | 4616 | C | TRP | B | 301 | 38.353 | 32.460 | 86.415 | 1.00 | 60.18 | B | C |
| ATOM | 4617 | O | TRP | B | 301 | 38.796 | 33.503 | 85.914 | 1.00 | 60.18 | B | O |

| | | | | | | | | | | | | |
|---|---|---|---|---|---|---|---|---|---|---|---|---|
| ATOM | 4618 | N | THR | B | 302 | 38.928 | 31.906 | 87.473 | 1.00 | 44.13 | B | N |
| ATOM | 4619 | CA | THR | B | 302 | 40.134 | 32.508 | 88.068 | 1.00 | 44.13 | B | C |
| ATOM | 4620 | CB | THR | B | 302 | 40.924 | 31.497 | 88.900 | 1.00 | 55.62 | B | C |
| ATOM | 4621 | OG1 | THR | B | 302 | 40.028 | 30.696 | 89.679 | 1.00 | 55.62 | B | O |
| ATOM | 4622 | CG2 | THR | B | 302 | 41.892 | 32.233 | 89.823 | 1.00 | 55.62 | B | C |
| ATOM | 4623 | C | THR | B | 302 | 41.135 | 33.089 | 87.043 | 1.00 | 44.13 | B | C |
| ATOM | 4624 | O | THR | B | 302 | 41.738 | 34.163 | 87.255 | 1.00 | 44.13 | B | O |
| ATOM | 4625 | N | LYS | B | 303 | 41.324 | 32.359 | 85.943 | 1.00 | 57.98 | B | N |
| ATOM | 4626 | CA | LYS | B | 303 | 42.254 | 32.783 | 84.906 | 1.00 | 57.98 | B | C |
| ATOM | 4627 | CB | LYS | B | 303 | 43.093 | 31.587 | 84.422 | 1.00 | 68.26 | B | C |
| ATOM | 4628 | CG | LYS | B | 303 | 43.904 | 30.933 | 85.556 | 1.00 | 68.26 | B | C |
| ATOM | 4629 | CD | LYS | B | 303 | 45.020 | 30.044 | 85.017 | 1.00 | 68.26 | B | C |
| ATOM | 4630 | CE | LYS | B | 303 | 46.111 | 30.873 | 84.310 | 1.00 | 68.26 | B | C |
| ATOM | 4631 | NZ | LYS | B | 303 | 47.109 | 30.026 | 83.568 | 1.00 | 68.26 | B | N |
| ATOM | 4632 | C | LYS | B | 303 | 41.615 | 33.508 | 83.723 | 1.00 | 57.98 | B | C |
| ATOM | 4633 | O | LYS | B | 303 | 42.123 | 33.449 | 82.614 | 1.00 | 57.98 | B | O |
| ATOM | 4634 | N | VAL | B | 304 | 40.514 | 34.212 | 83.979 | 1.00 | 41.30 | B | N |
| ATOM | 4635 | CA | VAL | B | 304 | 39.813 | 34.994 | 82.962 | 1.00 | 41.30 | B | C |
| ATOM | 4636 | CB | VAL | B | 304 | 38.281 | 34.932 | 83.172 | 1.00 | 19.93 | B | C |
| ATOM | 4637 | CG1 | VAL | B | 304 | 37.596 | 35.955 | 82.313 | 1.00 | 19.93 | B | C |
| ATOM | 4638 | CG2 | VAL | B | 304 | 37.760 | 33.548 | 82.870 | 1.00 | 19.93 | B | C |
| ATOM | 4639 | C | VAL | B | 304 | 40.258 | 36.447 | 83.131 | 1.00 | 41.30 | B | C |
| ATOM | 4640 | O | VAL | B | 304 | 40.326 | 37.206 | 82.160 | 1.00 | 41.30 | B | O |
| ATOM | 4641 | N | PHE | B | 305 | 40.564 | 36.819 | 84.376 | 1.00 | 38.20 | B | N |
| ATOM | 4642 | CA | PHE | B | 305 | 40.988 | 38.176 | 84.709 | 1.00 | 38.20 | B | C |
| ATOM | 4643 | CB | PHE | B | 305 | 40.227 | 38.642 | 85.944 | 1.00 | 32.76 | B | C |
| ATOM | 4644 | CG | PHE | B | 305 | 38.738 | 38.464 | 85.828 | 1.00 | 32.76 | B | C |
| ATOM | 4645 | CD1 | PHE | B | 305 | 37.954 | 39.415 | 85.186 | 1.00 | 32.76 | B | C |
| ATOM | 4646 | CD2 | PHE | B | 305 | 38.120 | 37.320 | 86.321 | 1.00 | 32.76 | B | C |
| ATOM | 4647 | CE1 | PHE | B | 305 | 36.580 | 39.228 | 85.033 | 1.00 | 32.76 | B | C |
| ATOM | 4648 | CE2 | PHE | B | 305 | 36.741 | 37.131 | 86.170 | 1.00 | 32.76 | B | C |
| ATOM | 4649 | CZ | PHE | B | 305 | 35.977 | 38.090 | 85.524 | 1.00 | 32.76 | B | C |
| ATOM | 4650 | C | PHE | B | 305 | 42.483 | 38.302 | 84.941 | 1.00 | 38.20 | B | C |
| ATOM | 4651 | O | PHE | B | 305 | 43.209 | 37.309 | 84.885 | 1.00 | 38.20 | B | O |
| ATOM | 4652 | N | ARG | B | 306 | 42.942 | 39.531 | 85.178 | 1.00 | 34.34 | B | N |
| ATOM | 4653 | CA | ARG | B | 306 | 44.359 | 39.783 | 85.430 | 1.00 | 34.34 | B | C |
| ATOM | 4654 | CB | ARG | B | 306 | 44.606 | 41.269 | 85.676 | 1.00 | 79.12 | B | C |
| ATOM | 4655 | CG | ARG | B | 306 | 44.206 | 42.139 | 84.510 | 1.00 | 79.12 | B | C |
| ATOM | 4656 | CD | ARG | B | 306 | 43.024 | 43.086 | 84.843 | 1.00 | 79.12 | B | C |
| ATOM | 4657 | NE | ARG | B | 306 | 43.391 | 44.497 | 84.654 | 1.00 | 79.12 | B | N |
| ATOM | 4658 | CZ | ARG | B | 306 | 44.393 | 45.103 | 85.300 | 1.00 | 79.12 | B | C |
| ATOM | 4659 | NH1 | ARG | B | 306 | 45.120 | 44.421 | 86.176 | 1.00 | 79.12 | B | N |
| ATOM | 4660 | NH2 | ARG | B | 306 | 44.682 | 46.387 | 85.070 | 1.00 | 79.12 | B | N |
| ATOM | 4661 | C | ARG | B | 306 | 44.799 | 38.978 | 86.648 | 1.00 | 34.34 | B | C |
| ATOM | 4662 | O | ARG | B | 306 | 43.968 | 38.516 | 87.437 | 1.00 | 34.34 | B | O |
| ATOM | 4663 | N | PRO | B | 307 | 46.114 | 38.793 | 86.820 | 1.00 | 55.56 | B | N |
| ATOM | 4664 | CD | PRO | B | 307 | 47.231 | 39.109 | 85.907 | 1.00 | 80.55 | B | C |
| ATOM | 4665 | CA | PRO | B | 307 | 46.584 | 38.020 | 87.974 | 1.00 | 55.56 | B | C |
| ATOM | 4666 | CB | PRO | B | 307 | 48.094 | 37.947 | 87.758 | 1.00 | 80.55 | B | C |
| ATOM | 4667 | CG | PRO | B | 307 | 48.229 | 38.011 | 86.239 | 1.00 | 80.55 | B | C |
| ATOM | 4668 | C | PRO | B | 307 | 46.229 | 38.661 | 89.310 | 1.00 | 55.56 | B | C |
| ATOM | 4669 | O | PRO | B | 307 | 45.662 | 38.020 | 90.192 | 1.00 | 55.56 | B | O |
| ATOM | 4670 | N | ALA | B | 308 | 46.565 | 39.932 | 89.461 | 1.00 | 43.60 | B | N |
| ATOM | 4671 | CA | ALA | B | 308 | 46.278 | 40.628 | 90.710 | 1.00 | 43.60 | B | C |
| ATOM | 4672 | CB | ALA | B | 308 | 46.705 | 42.093 | 90.587 | 1.00 | 69.61 | B | C |
| ATOM | 4673 | C | ALA | B | 308 | 44.810 | 40.544 | 91.174 | 1.00 | 43.60 | B | C |
| ATOM | 4674 | O | ALA | B | 308 | 44.536 | 40.431 | 92.369 | 1.00 | 43.60 | B | O |
| ATOM | 4675 | N | THR | B | 309 | 43.883 | 40.610 | 90.218 | 1.00 | 38.20 | B | N |
| ATOM | 4676 | CA | THR | B | 309 | 42.439 | 40.583 | 90.472 | 1.00 | 38.20 | B | C |
| ATOM | 4677 | CB | THR | B | 309 | 41.675 | 39.937 | 89.296 | 1.00 | 29.06 | B | C |
| ATOM | 4678 | OG1 | THR | B | 309 | 42.058 | 40.567 | 88.067 | 1.00 | 29.06 | B | O |
| ATOM | 4679 | CG2 | THR | B | 309 | 40.176 | 40.109 | 89.495 | 1.00 | 29.06 | B | C |
| ATOM | 4680 | C | THR | B | 309 | 41.996 | 39.880 | 91.741 | 1.00 | 38.20 | B | C |
| ATOM | 4681 | O | THR | B | 309 | 42.288 | 38.713 | 91.939 | 1.00 | 38.20 | B | O |
| ATOM | 4682 | N | PRO | B | 310 | 41.265 | 40.591 | 92.612 | 1.00 | 45.68 | B | N |
| ATOM | 4683 | CD | PRO | B | 310 | 40.902 | 42.011 | 92.472 | 1.00 | 19.85 | B | C |
| ATOM | 4684 | CA | PRO | B | 310 | 40.760 | 40.058 | 93.880 | 1.00 | 45.68 | B | C |

| | | | | | | | | | | | | |
|---|---|---|---|---|---|---|---|---|---|---|---|---|
| ATOM | 4685 | CB | PRO | B | 310 | 39.951 | 41.219 | 94.449 | 1.00 | 19.85 | B | C |
| ATOM | 4686 | CG | PRO | B | 310 | 40.630 | 42.408 | 93.906 | 1.00 | 19.85 | B | C |
| ATOM | 4687 | C | PRO | B | 310 | 39.885 | 38.843 | 93.634 | 1.00 | 45.68 | B | C |
| ATOM | 4688 | O | PRO | B | 310 | 39.087 | 38.816 | 92.693 | 1.00 | 45.68 | B | O |
| ATOM | 4689 | N | PRO | B | 311 | 40.022 | 37.815 | 94.480 | 1.00 | 35.96 | B | N |
| ATOM | 4690 | CD | PRO | B | 311 | 40.887 | 37.672 | 95.661 | 1.00 | 34.46 | B | C |
| ATOM | 4691 | CA | PRO | B | 311 | 39.200 | 36.621 | 94.292 | 1.00 | 35.96 | B | C |
| ATOM | 4692 | CB | PRO | B | 311 | 39.761 | 35.656 | 95.332 | 1.00 | 34.46 | B | C |
| ATOM | 4693 | CG | PRO | B | 311 | 40.196 | 36.568 | 96.420 | 1.00 | 34.46 | B | C |
| ATOM | 4694 | C | PRO | B | 311 | 37.716 | 36.933 | 94.485 | 1.00 | 35.96 | B | C |
| ATOM | 4695 | O | PRO | B | 311 | 36.884 | 36.490 | 93.694 | 1.00 | 35.96 | B | O |
| ATOM | 4696 | N | GLU | B | 312 | 37.379 | 37.711 | 95.511 | 1.00 | 40.71 | B | N |
| ATOM | 4697 | CA | GLU | B | 312 | 35.973 | 38.031 | 95.737 | 1.00 | 40.71 | B | C |
| ATOM | 4698 | CB | GLU | B | 312 | 35.766 | 38.797 | 97.059 | 1.00 | 71.15 | B | C |
| ATOM | 4699 | CG | GLU | B | 312 | 36.748 | 39.934 | 97.313 | 1.00 | 71.15 | B | C |
| ATOM | 4700 | CD | GLU | B | 312 | 38.112 | 39.427 | 97.779 | 1.00 | 71.15 | B | C |
| ATOM | 4701 | OE1 | GLU | B | 312 | 39.118 | 40.164 | 97.603 | 1.00 | 71.15 | B | O |
| ATOM | 4702 | OE2 | GLU | B | 312 | 38.163 | 38.293 | 98.325 | 1.00 | 71.15 | B | O |
| ATOM | 4703 | C | GLU | B | 312 | 35.276 | 38.752 | 94.561 | 1.00 | 40.71 | B | C |
| ATOM | 4704 | O | GLU | B | 312 | 34.060 | 38.625 | 94.383 | 1.00 | 40.71 | B | O |
| ATOM | 4705 | N | ALA | B | 313 | 36.020 | 39.497 | 93.750 | 1.00 | 32.75 | B | N |
| ATOM | 4706 | CA | ALA | B | 313 | 35.402 | 40.158 | 92.601 | 1.00 | 32.75 | B | C |
| ATOM | 4707 | CB | ALA | B | 313 | 36.348 | 41.162 | 92.017 | 1.00 | 34.59 | B | C |
| ATOM | 4708 | C | ALA | B | 313 | 35.114 | 39.059 | 91.578 | 1.00 | 32.75 | B | C |
| ATOM | 4709 | O | ALA | B | 313 | 34.068 | 39.034 | 90.917 | 1.00 | 32.75 | B | O |
| ATOM | 4710 | N | ILE | B | 314 | 36.068 | 38.148 | 91.448 | 1.00 | 29.23 | B | N |
| ATOM | 4711 | CA | ILE | B | 314 | 35.920 | 37.028 | 90.529 | 1.00 | 29.23 | B | C |
| ATOM | 4712 | CB | ILE | B | 314 | 37.208 | 36.166 | 90.479 | 1.00 | 29.78 | B | C |
| ATOM | 4713 | CG2 | ILE | B | 314 | 36.928 | 34.830 | 89.836 | 1.00 | 29.78 | B | C |
| ATOM | 4714 | CG1 | ILE | B | 314 | 38.310 | 36.908 | 89.727 | 1.00 | 29.78 | B | C |
| ATOM | 4715 | CD1 | ILE | B | 314 | 39.666 | 36.224 | 89.801 | 1.00 | 29.78 | B | C |
| ATOM | 4716 | C | ILE | B | 314 | 34.783 | 36.151 | 91.036 | 1.00 | 29.23 | B | C |
| ATOM | 4717 | O | ILE | B | 314 | 33.917 | 35.726 | 90.270 | 1.00 | 29.23 | B | O |
| ATOM | 4718 | N | ALA | B | 315 | 34.806 | 35.869 | 92.335 | 1.00 | 31.73 | B | N |
| ATOM | 4719 | CA | ALA | B | 315 | 33.783 | 35.044 | 92.963 | 1.00 | 31.73 | B | C |
| ATOM | 4720 | CB | ALA | B | 315 | 34.001 | 35.021 | 94.464 | 1.00 | 18.45 | B | C |
| ATOM | 4721 | C | ALA | B | 315 | 32.374 | 35.556 | 92.639 | 1.00 | 31.73 | B | C |
| ATOM | 4722 | O | ALA | B | 315 | 31.501 | 34.796 | 92.210 | 1.00 | 31.73 | B | O |
| ATOM | 4723 | N | LEU | B | 316 | 32.156 | 36.851 | 92.854 | 1.00 | 32.87 | B | N |
| ATOM | 4724 | CA | LEU | B | 316 | 30.867 | 37.465 | 92.567 | 1.00 | 32.87 | B | C |
| ATOM | 4725 | CB | LEU | B | 316 | 30.891 | 38.927 | 92.983 | 1.00 | 24.09 | B | C |
| ATOM | 4726 | CG | LEU | B | 316 | 29.672 | 39.721 | 92.550 | 1.00 | 24.09 | B | C |
| ATOM | 4727 | CD1 | LEU | B | 316 | 28.427 | 39.072 | 93.111 | 1.00 | 24.09 | B | C |
| ATOM | 4728 | CD2 | LEU | B | 316 | 29.825 | 41.151 | 93.016 | 1.00 | 24.09 | B | C |
| ATOM | 4729 | C | LEU | B | 316 | 30.471 | 37.345 | 91.093 | 1.00 | 32.87 | B | C |
| ATOM | 4730 | O | LEU | B | 316 | 29.298 | 37.209 | 90.786 | 1.00 | 32.87 | B | O |
| ATOM | 4731 | N | CYS | B | 317 | 31.432 | 37.407 | 90.178 | 1.00 | 45.55 | B | N |
| ATOM | 4732 | CA | CYS | B | 317 | 31.105 | 37.252 | 88.766 | 1.00 | 45.55 | B | C |
| ATOM | 4733 | CB | CYS | B | 317 | 32.353 | 37.334 | 87.897 | 1.00 | 56.00 | B | C |
| ATOM | 4734 | SG | CYS | B | 317 | 32.713 | 38.983 | 87.292 | 1.00 | 56.00 | B | S |
| ATOM | 4735 | C | CYS | B | 317 | 30.461 | 35.908 | 88.522 | 1.00 | 45.55 | B | C |
| ATOM | 4736 | O | CYS | B | 317 | 29.343 | 35.832 | 88.012 | 1.00 | 45.55 | B | O |
| ATOM | 4737 | N | SER | B | 318 | 31.178 | 34.850 | 88.889 | 1.00 | 30.83 | B | N |
| ATOM | 4738 | CA | SER | B | 318 | 30.694 | 33.495 | 88.689 | 1.00 | 30.83 | B | C |
| ATOM | 4739 | CB | SER | B | 318 | 31.700 | 32.504 | 89.262 | 1.00 | 42.43 | B | C |
| ATOM | 4740 | OG | SER | B | 318 | 32.078 | 32.899 | 90.565 | 1.00 | 42.43 | B | O |
| ATOM | 4741 | C | SER | B | 318 | 29.322 | 33.250 | 89.306 | 1.00 | 30.83 | B | C |
| ATOM | 4742 | O | SER | B | 318 | 28.679 | 32.246 | 89.008 | 1.00 | 30.83 | B | O |
| ATOM | 4743 | N | ARG | B | 319 | 28.869 | 34.152 | 90.169 | 1.00 | 34.23 | B | N |
| ATOM | 4744 | CA | ARG | B | 319 | 27.566 | 33.968 | 90.787 | 1.00 | 34.23 | B | C |
| ATOM | 4745 | CB | ARG | B | 319 | 27.616 | 34.304 | 92.272 | 1.00 | 34.07 | B | C |
| ATOM | 4746 | CG | ARG | B | 319 | 28.417 | 33.313 | 93.107 | 1.00 | 34.07 | B | C |
| ATOM | 4747 | CD | ARG | B | 319 | 28.096 | 31.871 | 92.760 | 1.00 | 34.07 | B | C |
| ATOM | 4748 | NE | ARG | B | 319 | 26.667 | 31.660 | 92.536 | 1.00 | 34.07 | B | N |
| ATOM | 4749 | CZ | ARG | B | 319 | 25.752 | 31.567 | 93.496 | 1.00 | 34.07 | B | C |
| ATOM | 4750 | NH1 | ARG | B | 319 | 26.107 | 31.666 | 94.776 | 1.00 | 34.07 | B | N |
| ATOM | 4751 | NH2 | ARG | B | 319 | 24.475 | 31.367 | 93.174 | 1.00 | 34.07 | B | N |

| | | | | | | | | | | | | |
|---|---|---|---|---|---|---|---|---|---|---|---|---|
| ATOM | 4752 | C | ARG | B | 319 | 26.527 | 34.821 | 90.095 | 1.00 | 34.23 | B | C |
| ATOM | 4753 | O | ARG | B | 319 | 25.328 | 34.742 | 90.389 | 1.00 | 34.23 | B | O |
| ATOM | 4754 | N | LEU | B | 320 | 27.006 | 35.639 | 89.166 | 1.00 | 42.79 | B | N |
| ATOM | 4755 | CA | LEU | B | 320 | 26.136 | 36.504 | 88.377 | 1.00 | 42.79 | B | C |
| ATOM | 4756 | CB | LEU | B | 320 | 26.745 | 37.908 | 88.270 | 1.00 | 19.31 | B | C |
| ATOM | 4757 | CG | LEU | B | 320 | 26.864 | 38.624 | 89.617 | 1.00 | 19.31 | B | C |
| ATOM | 4758 | CD1 | LEU | B | 320 | 27.617 | 39.926 | 89.487 | 1.00 | 19.31 | B | C |
| ATOM | 4759 | CD2 | LEU | B | 320 | 25.465 | 38.856 | 90.153 | 1.00 | 19.31 | B | C |
| ATOM | 4760 | C | LEU | B | 320 | 25.957 | 35.892 | 86.984 | 1.00 | 42.79 | B | C |
| ATOM | 4761 | O | LEU | B | 320 | 24.843 | 35.760 | 86.479 | 1.00 | 42.79 | B | O |
| ATOM | 4762 | N | LEU | B | 321 | 27.064 | 35.493 | 86.375 | 1.00 | 26.99 | B | N |
| ATOM | 4763 | CA | LEU | B | 321 | 27.011 | 34.894 | 85.055 | 1.00 | 26.99 | B | C |
| ATOM | 4764 | CB | LEU | B | 321 | 28.305 | 35.230 | 84.301 | 1.00 | 26.31 | B | C |
| ATOM | 4765 | CG | LEU | B | 321 | 28.601 | 36.737 | 84.204 | 1.00 | 26.31 | B | C |
| ATOM | 4766 | CD1 | LEU | B | 321 | 29.801 | 36.992 | 83.321 | 1.00 | 26.31 | B | C |
| ATOM | 4767 | CD2 | LEU | B | 321 | 27.401 | 37.463 | 83.663 | 1.00 | 26.31 | B | C |
| ATOM | 4768 | C | LEU | B | 321 | 26.769 | 33.378 | 85.096 | 1.00 | 26.99 | B | C |
| ATOM | 4769 | O | LEU | B | 321 | 27.667 | 32.581 | 84.835 | 1.00 | 26.99 | B | O |
| ATOM | 4770 | N | GLU | B | 322 | 25.532 | 33.004 | 85.411 | 1.00 | 38.35 | B | N |
| ATOM | 4771 | CA | GLU | B | 322 | 25.116 | 31.610 | 85.500 | 1.00 | 38.35 | B | C |
| ATOM | 4772 | CB | GLU | B | 322 | 24.459 | 31.363 | 86.854 | 1.00 | 46.96 | B | C |
| ATOM | 4773 | CG | GLU | B | 322 | 24.840 | 30.050 | 87.492 | 1.00 | 46.96 | B | C |
| ATOM | 4774 | CD | GLU | B | 322 | 26.074 | 30.167 | 88.355 | 1.00 | 46.96 | B | C |
| ATOM | 4775 | OE1 | GLU | B | 322 | 25.968 | 30.832 | 89.407 | 1.00 | 46.96 | B | O |
| ATOM | 4776 | OE2 | GLU | B | 322 | 27.134 | 29.609 | 87.989 | 1.00 | 46.96 | B | O |
| ATOM | 4777 | C | GLU | B | 322 | 24.101 | 31.318 | 84.403 | 1.00 | 38.35 | B | C |
| ATOM | 4778 | O | GLU | B | 322 | 23.195 | 32.113 | 84.175 | 1.00 | 38.35 | B | O |
| ATOM | 4779 | N | TYR | B | 323 | 24.252 | 30.188 | 83.719 | 1.00 | 46.83 | B | N |
| ATOM | 4780 | CA | TYR | B | 323 | 23.301 | 29.803 | 82.670 | 1.00 | 46.83 | B | C |
| ATOM | 4781 | CB | TYR | B | 323 | 23.624 | 28.403 | 82.141 | 1.00 | 30.11 | B | C |
| ATOM | 4782 | CG | TYR | B | 323 | 24.710 | 28.379 | 81.098 | 1.00 | 30.11 | B | C |
| ATOM | 4783 | CD1 | TYR | B | 323 | 24.591 | 29.123 | 79.930 | 1.00 | 30.11 | B | C |
| ATOM | 4784 | CE1 | TYR | B | 323 | 25.590 | 29.102 | 78.961 | 1.00 | 30.11 | B | C |
| ATOM | 4785 | CD2 | TYR | B | 323 | 25.860 | 27.610 | 81.269 | 1.00 | 30.11 | B | C |
| ATOM | 4786 | CE2 | TYR | B | 323 | 26.866 | 27.584 | 80.300 | 1.00 | 30.11 | B | C |
| ATOM | 4787 | CZ | TYR | B | 323 | 26.716 | 28.333 | 79.155 | 1.00 | 30.11 | B | C |
| ATOM | 4788 | OH | TYR | B | 323 | 27.690 | 28.329 | 78.198 | 1.00 | 30.11 | B | O |
| ATOM | 4789 | C | TYR | B | 323 | 21.875 | 29.800 | 83.240 | 1.00 | 46.83 | B | C |
| ATOM | 4790 | O | TYR | B | 323 | 20.985 | 30.511 | 82.762 | 1.00 | 46.83 | B | O |
| ATOM | 4791 | N | THR | B | 324 | 21.670 | 28.990 | 84.270 | 1.00 | 35.80 | B | N |
| ATOM | 4792 | CA | THR | B | 324 | 20.368 | 28.892 | 84.899 | 1.00 | 35.80 | B | C |
| ATOM | 4793 | CB | THR | B | 324 | 20.361 | 27.769 | 85.964 | 1.00 | 31.94 | B | C |
| ATOM | 4794 | OG1 | THR | B | 324 | 20.220 | 26.496 | 85.316 | 1.00 | 31.94 | B | O |
| ATOM | 4795 | CG2 | THR | B | 324 | 19.224 | 27.964 | 86.948 | 1.00 | 31.94 | B | C |
| ATOM | 4796 | C | THR | B | 324 | 19.989 | 30.233 | 85.530 | 1.00 | 35.80 | B | C |
| ATOM | 4797 | O | THR | B | 324 | 20.622 | 30.681 | 86.491 | 1.00 | 35.80 | B | O |
| ATOM | 4798 | N | PRO | B | 325 | 18.956 | 30.902 | 84.986 | 1.00 | 38.24 | B | N |
| ATOM | 4799 | CD | PRO | B | 325 | 18.128 | 30.560 | 83.815 | 1.00 | 29.83 | B | C |
| ATOM | 4800 | CA | PRO | B | 325 | 18.555 | 32.194 | 85.554 | 1.00 | 38.24 | B | C |
| ATOM | 4801 | CB | PRO | B | 325 | 17.260 | 32.517 | 84.826 | 1.00 | 29.83 | B | C |
| ATOM | 4802 | CG | PRO | B | 325 | 17.480 | 31.891 | 83.479 | 1.00 | 29.83 | B | C |
| ATOM | 4803 | C | PRO | B | 325 | 18.334 | 32.115 | 87.052 | 1.00 | 38.24 | B | C |
| ATOM | 4804 | O | PRO | B | 325 | 18.868 | 32.925 | 87.815 | 1.00 | 38.24 | B | O |
| ATOM | 4805 | N | THR | B | 326 | 17.559 | 31.126 | 87.480 | 1.00 | 17.96 | B | N |
| ATOM | 4806 | CA | THR | B | 326 | 17.271 | 30.990 | 88.897 | 1.00 | 17.96 | B | C |
| ATOM | 4807 | CB | THR | B | 326 | 16.238 | 29.876 | 89.157 | 1.00 | 26.51 | B | C |
| ATOM | 4808 | OG1 | THR | B | 326 | 16.822 | 28.597 | 88.895 | 1.00 | 26.51 | B | O |
| ATOM | 4809 | CG2 | THR | B | 326 | 15.039 | 30.059 | 88.260 | 1.00 | 26.51 | B | C |
| ATOM | 4810 | C | THR | B | 326 | 18.486 | 30.770 | 89.815 | 1.00 | 17.96 | B | C |
| ATOM | 4811 | O | THR | B | 326 | 18.381 | 30.911 | 91.023 | 1.00 | 17.96 | B | O |
| ATOM | 4812 | N | ALA | B | 327 | 19.643 | 30.446 | 89.258 | 1.00 | 25.28 | B | N |
| ATOM | 4813 | CA | ALA | B | 327 | 20.826 | 30.217 | 90.080 | 1.00 | 25.28 | B | C |
| ATOM | 4814 | CB | ALA | B | 327 | 21.666 | 29.148 | 89.452 | 1.00 | 15.72 | B | C |
| ATOM | 4815 | C | ALA | B | 327 | 21.670 | 31.465 | 90.290 | 1.00 | 25.28 | B | C |
| ATOM | 4816 | O | ALA | B | 327 | 22.604 | 31.456 | 91.083 | 1.00 | 25.28 | B | O |
| ATOM | 4817 | N | ARG | B | 328 | 21.349 | 32.539 | 89.576 | 1.00 | 39.75 | B | N |
| ATOM | 4818 | CA | ARG | B | 328 | 22.114 | 33.777 | 89.691 | 1.00 | 39.75 | B | C |

| | | | | | | | | | | | | |
|---|---|---|---|---|---|---|---|---|---|---|---|---|
| ATOM | 4819 | CB | ARG | B | 328 | 21.787 | 34.702 | 88.524 | 1.00 | 21.28 | B | C |
| ATOM | 4820 | CG | ARG | B | 328 | 21.926 | 34.025 | 87.167 | 1.00 | 21.28 | B | C |
| ATOM | 4821 | CD | ARG | B | 328 | 21.342 | 34.863 | 86.041 | 1.00 | 21.28 | B | C |
| ATOM | 4822 | NE | ARG | B | 328 | 21.229 | 34.068 | 84.833 | 1.00 | 21.28 | B | N |
| ATOM | 4823 | CZ | ARG | B | 328 | 20.626 | 34.473 | 83.735 | 1.00 | 21.28 | B | C |
| ATOM | 4824 | NH1 | ARG | B | 328 | 20.077 | 35.680 | 83.683 | 1.00 | 21.28 | B | N |
| ATOM | 4825 | NH2 | ARG | B | 328 | 20.563 | 33.656 | 82.702 | 1.00 | 21.28 | B | N |
| ATOM | 4826 | C | ARG | B | 328 | 21.743 | 34.457 | 90.976 | 1.00 | 39.75 | B | C |
| ATOM | 4827 | O | ARG | B | 328 | 20.676 | 34.209 | 91.497 | 1.00 | 39.75 | B | O |
| ATOM | 4828 | N | LEU | B | 329 | 22.617 | 35.313 | 91.488 | 1.00 | 35.15 | B | N |
| ATOM | 4829 | CA | LEU | B | 329 | 22.329 | 36.035 | 92.719 | 1.00 | 35.15 | B | C |
| ATOM | 4830 | CB | LEU | B | 329 | 23.570 | 36.799 | 93.190 | 1.00 | 20.61 | B | C |
| ATOM | 4831 | CG | LEU | B | 329 | 24.546 | 36.101 | 94.135 | 1.00 | 20.61 | B | C |
| ATOM | 4832 | CD1 | LEU | B | 329 | 24.587 | 34.628 | 93.849 | 1.00 | 20.61 | B | C |
| ATOM | 4833 | CD2 | LEU | B | 329 | 25.913 | 36.708 | 93.971 | 1.00 | 20.61 | B | C |
| ATOM | 4834 | C | LEU | B | 329 | 21.195 | 37.022 | 92.497 | 1.00 | 35.15 | B | C |
| ATOM | 4835 | O | LEU | B | 329 | 20.833 | 37.342 | 91.366 | 1.00 | 35.15 | B | O |
| ATOM | 4836 | N | THR | B | 330 | 20.630 | 37.479 | 93.603 | 1.00 | 32.26 | B | N |
| ATOM | 4837 | CA | THR | B | 330 | 19.567 | 38.468 | 93.606 | 1.00 | 32.26 | B | C |
| ATOM | 4838 | CB | THR | B | 330 | 18.620 | 38.213 | 94.766 | 1.00 | 34.27 | B | C |
| ATOM | 4839 | OG1 | THR | B | 330 | 17.655 | 37.243 | 94.368 | 1.00 | 34.27 | B | O |
| ATOM | 4840 | CG2 | THR | B | 330 | 17.923 | 39.487 | 95.207 | 1.00 | 34.27 | B | C |
| ATOM | 4841 | C | THR | B | 330 | 20.260 | 39.804 | 93.833 | 1.00 | 32.26 | B | C |
| ATOM | 4842 | O | THR | B | 330 | 21.237 | 39.883 | 94.569 | 1.00 | 32.26 | B | O |
| ATOM | 4843 | N | PRO | B | 331 | 19.765 | 40.873 | 93.211 | 1.00 | 26.20 | B | N |
| ATOM | 4844 | CD | PRO | B | 331 | 18.581 | 40.983 | 92.348 | 1.00 | 31.93 | B | C |
| ATOM | 4845 | CA | PRO | B | 331 | 20.394 | 42.183 | 93.398 | 1.00 | 26.20 | B | C |
| ATOM | 4846 | CB | PRO | B | 331 | 19.336 | 43.140 | 92.855 | 1.00 | 31.93 | B | C |
| ATOM | 4847 | CG | PRO | B | 331 | 18.753 | 42.370 | 91.749 | 1.00 | 31.93 | B | C |
| ATOM | 4848 | C | PRO | B | 331 | 20.763 | 42.465 | 94.860 | 1.00 | 26.20 | B | C |
| ATOM | 4849 | O | PRO | B | 331 | 21.815 | 43.036 | 95.157 | 1.00 | 26.20 | B | O |
| ATOM | 4850 | N | LEU | B | 332 | 19.898 | 42.037 | 95.770 | 1.00 | 22.38 | B | N |
| ATOM | 4851 | CA | LEU | B | 332 | 20.116 | 42.248 | 97.189 | 1.00 | 22.38 | B | C |
| ATOM | 4852 | CB | LEU | B | 332 | 18.789 | 42.085 | 97.913 | 1.00 | 12.07 | B | C |
| ATOM | 4853 | CG | LEU | B | 332 | 18.716 | 42.551 | 99.356 | 1.00 | 12.07 | B | C |
| ATOM | 4854 | CD1 | LEU | B | 332 | 18.920 | 44.030 | 99.448 | 1.00 | 12.07 | B | C |
| ATOM | 4855 | CD2 | LEU | B | 332 | 17.371 | 42.158 | 99.902 | 1.00 | 12.07 | B | C |
| ATOM | 4856 | C | LEU | B | 332 | 21.153 | 41.264 | 97.718 | 1.00 | 22.38 | B | C |
| ATOM | 4857 | O | LEU | B | 332 | 21.947 | 41.599 | 98.596 | 1.00 | 22.38 | B | O |
| ATOM | 4858 | N | GLU | B | 333 | 21.144 | 40.052 | 97.173 | 1.00 | 30.57 | B | N |
| ATOM | 4859 | CA | GLU | B | 333 | 22.098 | 39.032 | 97.585 | 1.00 | 30.57 | B | C |
| ATOM | 4860 | CB | GLU | B | 333 | 21.769 | 37.678 | 96.958 | 1.00 | 38.47 | B | C |
| ATOM | 4861 | CG | GLU | B | 333 | 20.461 | 37.054 | 97.387 | 1.00 | 38.47 | B | C |
| ATOM | 4862 | CD | GLU | B | 333 | 20.234 | 35.707 | 96.714 | 1.00 | 38.47 | B | C |
| ATOM | 4863 | OE1 | GLU | B | 333 | 20.480 | 35.597 | 95.482 | 1.00 | 38.47 | B | O |
| ATOM | 4864 | OE2 | GLU | B | 333 | 19.806 | 34.768 | 97.428 | 1.00 | 38.47 | B | O |
| ATOM | 4865 | C | GLU | B | 333 | 23.461 | 39.461 | 97.106 | 1.00 | 30.57 | B | C |
| ATOM | 4866 | O | GLU | B | 333 | 24.469 | 39.137 | 97.716 | 1.00 | 30.57 | B | O |
| ATOM | 4867 | N | ALA | B | 334 | 23.479 | 40.202 | 96.006 | 1.00 | 27.39 | B | N |
| ATOM | 4868 | CA | ALA | B | 334 | 24.723 | 40.661 | 95.431 | 1.00 | 27.39 | B | C |
| ATOM | 4869 | CB | ALA | B | 334 | 24.486 | 41.146 | 94.007 | 1.00 | 31.78 | B | C |
| ATOM | 4870 | C | ALA | B | 334 | 25.278 | 41.769 | 96.299 | 1.00 | 27.39 | B | C |
| ATOM | 4871 | O | ALA | B | 334 | 26.483 | 41.832 | 96.532 | 1.00 | 27.39 | B | O |
| ATOM | 4872 | N | CYS | B | 335 | 24.395 | 42.625 | 96.793 | 1.00 | 22.12 | B | N |
| ATOM | 4873 | CA | CYS | B | 335 | 24.813 | 43.733 | 97.637 | 1.00 | 22.12 | B | C |
| ATOM | 4874 | CB | CYS | B | 335 | 23.604 | 44.573 | 98.037 | 1.00 | 29.50 | B | C |
| ATOM | 4875 | SG | CYS | B | 335 | 23.071 | 45.758 | 96.793 | 1.00 | 29.50 | B | S |
| ATOM | 4876 | C | CYS | B | 335 | 25.511 | 43.258 | 98.892 | 1.00 | 22.12 | B | C |
| ATOM | 4877 | O | CYS | B | 335 | 26.407 | 43.928 | 99.402 | 1.00 | 22.12 | B | O |
| ATOM | 4878 | N | ALA | B | 336 | 25.100 | 42.095 | 99.384 | 1.00 | 19.83 | B | N |
| ATOM | 4879 | CA | ALA | B | 336 | 25.669 | 41.540 | 100.604 | 1.00 | 19.83 | B | C |
| ATOM | 4880 | CB | ALA | B | 336 | 24.625 | 40.724 | 101.351 | 1.00 | 10.05 | B | C |
| ATOM | 4881 | C | ALA | B | 336 | 26.906 | 40.705 | 100.380 | 1.00 | 19.83 | B | C |
| ATOM | 4882 | O | ALA | B | 336 | 27.506 | 40.230 | 101.346 | 1.00 | 19.83 | B | O |
| ATOM | 4883 | N | HIS | B | 337 | 27.299 | 40.550 | 99.118 | 1.00 | 20.93 | B | N |
| ATOM | 4884 | CA | HIS | B | 337 | 28.478 | 39.771 | 98.781 | 1.00 | 20.93 | B | C |
| ATOM | 4885 | CB | HIS | B | 337 | 28.627 | 39.676 | 97.264 | 1.00 | 31.62 | B | C |

| | | | | | | | | | | | | |
|---|---|---|---|---|---|---|---|---|---|---|---|---|
| ATOM | 4886 | CG | HIS | B | 337 | 29.469 | 38.523 | 96.815 | 1.00 | 31.62 | B | C |
| ATOM | 4887 | CD2 | HIS | B | 337 | 29.129 | 37.262 | 96.455 | 1.00 | 31.62 | B | C |
| ATOM | 4888 | ND1 | HIS | B | 337 | 30.840 | 38.597 | 96.707 | 1.00 | 31.62 | B | N |
| ATOM | 4889 | CE1 | HIS | B | 337 | 31.309 | 37.432 | 96.297 | 1.00 | 31.62 | B | C |
| ATOM | 4890 | NE2 | HIS | B | 337 | 30.292 | 36.606 | 96.135 | 1.00 | 31.62 | B | N |
| ATOM | 4891 | C | HIS | B | 337 | 29.740 | 40.359 | 99.423 | 1.00 | 20.93 | B | C |
| ATOM | 4892 | O | HIS | B | 337 | 29.847 | 41.563 | 99.646 | 1.00 | 20.93 | B | O |
| ATOM | 4893 | N | SER | B | 338 | 30.692 | 39.493 | 99.735 | 1.00 | 35.62 | B | N |
| ATOM | 4894 | CA | SER | B | 338 | 31.927 | 39.923 | 100.382 | 1.00 | 35.62 | B | C |
| ATOM | 4895 | CB | SER | B | 338 | 32.800 | 38.703 | 100.643 | 1.00 | 34.29 | B | C |
| ATOM | 4896 | OG | SER | B | 338 | 32.005 | 37.635 | 101.127 | 1.00 | 34.29 | B | O |
| ATOM | 4897 | C | SER | B | 338 | 32.710 | 40.960 | 99.583 | 1.00 | 35.62 | B | C |
| ATOM | 4898 | O | SER | B | 338 | 33.414 | 41.808 | 100.140 | 1.00 | 35.62 | B | O |
| ATOM | 4899 | N | PHE | B | 339 | 32.605 | 40.883 | 98.265 | 1.00 | 42.35 | B | N |
| ATOM | 4900 | CA | PHE | B | 339 | 33.313 | 41.830 | 97.426 | 1.00 | 42.35 | B | C |
| ATOM | 4901 | CB | PHE | B | 339 | 33.009 | 41.548 | 95.955 | 1.00 | 27.58 | B | C |
| ATOM | 4902 | CG | PHE | B | 339 | 33.615 | 42.541 | 95.033 | 1.00 | 27.58 | B | C |
| ATOM | 4903 | CD1 | PHE | B | 339 | 34.979 | 42.786 | 95.068 | 1.00 | 27.58 | B | C |
| ATOM | 4904 | CD2 | PHE | B | 339 | 32.827 | 43.283 | 94.172 | 1.00 | 27.58 | B | C |
| ATOM | 4905 | CE1 | PHE | B | 339 | 35.550 | 43.763 | 94.261 | 1.00 | 27.58 | B | C |
| ATOM | 4906 | CE2 | PHE | B | 339 | 33.396 | 44.265 | 93.358 | 1.00 | 27.58 | B | C |
| ATOM | 4907 | CZ | PHE | B | 339 | 34.759 | 44.502 | 93.407 | 1.00 | 27.58 | B | C |
| ATOM | 4908 | C | PHE | B | 339 | 32.972 | 43.292 | 97.780 | 1.00 | 42.35 | B | C |
| ATOM | 4909 | O | PHE | B | 339 | 33.696 | 44.217 | 97.414 | 1.00 | 42.35 | B | O |
| ATOM | 4910 | N | PHE | B | 340 | 31.885 | 43.501 | 98.513 | 1.00 | 31.40 | B | N |
| ATOM | 4911 | CA | PHE | B | 340 | 31.475 | 44.851 | 98.879 | 1.00 | 31.40 | B | C |
| ATOM | 4912 | CB | PHE | B | 340 | 29.994 | 45.036 | 98.548 | 1.00 | 24.44 | B | C |
| ATOM | 4913 | CG | PHE | B | 340 | 29.682 | 44.905 | 97.086 | 1.00 | 24.44 | B | C |
| ATOM | 4914 | CD1 | PHE | B | 340 | 30.332 | 45.706 | 96.151 | 1.00 | 24.44 | B | C |
| ATOM | 4915 | CD2 | PHE | B | 340 | 28.722 | 44.005 | 96.643 | 1.00 | 24.44 | B | C |
| ATOM | 4916 | CE1 | PHE | B | 340 | 30.036 | 45.617 | 94.813 | 1.00 | 24.44 | B | C |
| ATOM | 4917 | CE2 | PHE | B | 340 | 28.419 | 43.912 | 95.299 | 1.00 | 24.44 | B | C |
| ATOM | 4918 | CZ | PHE | B | 340 | 29.079 | 44.722 | 94.384 | 1.00 | 24.44 | B | C |
| ATOM | 4919 | C | PHE | B | 340 | 31.718 | 45.160 | 100.354 | 1.00 | 31.40 | B | C |
| ATOM | 4920 | O | PHE | B | 340 | 31.229 | 46.155 | 100.876 | 1.00 | 31.40 | B | O |
| ATOM | 4921 | N | ASP | B | 341 | 32.478 | 44.298 | 101.018 | 1.00 | 23.54 | B | N |
| ATOM | 4922 | CA | ASP | B | 341 | 32.776 | 44.468 | 102.428 | 1.00 | 23.54 | B | C |
| ATOM | 4923 | CB | ASP | B | 341 | 33.531 | 43.258 | 102.945 | 1.00 | 44.69 | B | C |
| ATOM | 4924 | CG | ASP | B | 341 | 32.606 | 42.140 | 103.346 | 1.00 | 44.69 | B | C |
| ATOM | 4925 | OD1 | ASP | B | 341 | 31.700 | 41.820 | 102.552 | 1.00 | 44.69 | B | O |
| ATOM | 4926 | OD2 | ASP | B | 341 | 32.777 | 41.584 | 104.451 | 1.00 | 44.69 | B | O |
| ATOM | 4927 | C | ASP | B | 341 | 33.572 | 45.719 | 102.729 | 1.00 | 23.54 | B | C |
| ATOM | 4928 | O | ASP | B | 341 | 33.398 | 46.331 | 103.771 | 1.00 | 23.54 | B | O |
| ATOM | 4929 | N | GLU | B | 342 | 34.457 | 46.108 | 101.831 | 1.00 | 29.22 | B | N |
| ATOM | 4930 | CA | GLU | B | 342 | 35.231 | 47.299 | 102.092 | 1.00 | 29.22 | B | C |
| ATOM | 4931 | CB | GLU | B | 342 | 36.255 | 47.538 | 100.994 | 1.00 | 33.81 | B | C |
| ATOM | 4932 | CG | GLU | B | 342 | 37.004 | 48.831 | 101.186 | 1.00 | 33.81 | B | C |
| ATOM | 4933 | CD | GLU | B | 342 | 38.266 | 48.911 | 100.368 | 1.00 | 33.81 | B | C |
| ATOM | 4934 | OE1 | GLU | B | 342 | 38.209 | 48.647 | 99.143 | 1.00 | 33.81 | B | O |
| ATOM | 4935 | OE2 | GLU | B | 342 | 39.314 | 49.249 | 100.963 | 1.00 | 33.81 | B | O |
| ATOM | 4936 | C | GLU | B | 342 | 34.333 | 48.513 | 102.215 | 1.00 | 29.22 | B | C |
| ATOM | 4937 | O | GLU | B | 342 | 34.667 | 49.462 | 102.908 | 1.00 | 29.22 | B | O |
| ATOM | 4938 | N | LEU | B | 343 | 33.193 | 48.497 | 101.542 | 1.00 | 26.55 | B | N |
| ATOM | 4939 | CA | LEU | B | 343 | 32.295 | 49.635 | 101.619 | 1.00 | 26.55 | B | C |
| ATOM | 4940 | CB | LEU | B | 343 | 31.220 | 49.541 | 100.540 | 1.00 | 10.33 | B | C |
| ATOM | 4941 | CG | LEU | B | 343 | 31.707 | 49.406 | 99.101 | 1.00 | 10.33 | B | C |
| ATOM | 4942 | CD1 | LEU | B | 343 | 30.538 | 49.491 | 98.163 | 1.00 | 10.33 | B | C |
| ATOM | 4943 | CD2 | LEU | B | 343 | 32.709 | 50.484 | 98.808 | 1.00 | 10.33 | B | C |
| ATOM | 4944 | C | LEU | B | 343 | 31.658 | 49.651 | 102.995 | 1.00 | 26.55 | B | C |
| ATOM | 4945 | O | LEU | B | 343 | 31.305 | 50.706 | 103.514 | 1.00 | 26.55 | B | O |
| ATOM | 4946 | N | ARG | B | 344 | 31.522 | 48.480 | 103.600 | 1.00 | 38.61 | B | N |
| ATOM | 4947 | CA | ARG | B | 344 | 30.921 | 48.412 | 104.924 | 1.00 | 38.61 | B | C |
| ATOM | 4948 | CB | ARG | B | 344 | 30.298 | 47.035 | 105.152 | 1.00 | 22.01 | B | C |
| ATOM | 4949 | CG | ARG | B | 344 | 29.006 | 46.864 | 104.401 | 1.00 | 22.01 | B | C |
| ATOM | 4950 | CD | ARG | B | 344 | 28.327 | 45.582 | 104.741 | 1.00 | 22.01 | B | C |
| ATOM | 4951 | NE | ARG | B | 344 | 28.812 | 44.476 | 103.929 | 1.00 | 22.01 | B | N |
| ATOM | 4952 | CZ | ARG | B | 344 | 28.584 | 44.357 | 102.629 | 1.00 | 22.01 | B | C |

| | | | | | | | | | | | | |
|---|---|---|---|---|---|---|---|---|---|---|---|---|
| ATOM | 4953 | NH1 | ARG | B | 344 | 27.883 | 45.286 | 101.995 | 1.00 | 22.01 | B | N |
| ATOM | 4954 | NH2 | ARG | B | 344 | 29.038 | 43.299 | 101.971 | 1.00 | 22.01 | B | N |
| ATOM | 4955 | C | ARG | B | 344 | 31.903 | 48.755 | 106.046 | 1.00 | 38.61 | B | C |
| ATOM | 4956 | O | ARG | B | 344 | 31.522 | 48.869 | 107.214 | 1.00 | 38.61 | B | O |
| ATOM | 4957 | N | ASP | B | 345 | 33.173 | 48.917 | 105.691 | 1.00 | 39.92 | B | N |
| ATOM | 4958 | CA | ASP | B | 345 | 34.178 | 49.280 | 106.675 | 1.00 | 39.92 | B | C |
| ATOM | 4959 | CB | ASP | B | 345 | 35.574 | 49.188 | 106.060 | 1.00 | 56.74 | B | C |
| ATOM | 4960 | CG | ASP | B | 345 | 36.676 | 49.571 | 107.037 | 1.00 | 56.74 | B | C |
| ATOM | 4961 | OD1 | ASP | B | 345 | 37.737 | 48.892 | 107.020 | 1.00 | 56.74 | B | O |
| ATOM | 4962 | OD2 | ASP | B | 345 | 36.483 | 50.552 | 107.798 | 1.00 | 56.74 | B | O |
| ATOM | 4963 | C | ASP | B | 345 | 33.879 | 50.712 | 107.099 | 1.00 | 39.92 | B | C |
| ATOM | 4964 | O | ASP | B | 345 | 33.586 | 51.561 | 106.259 | 1.00 | 39.92 | B | O |
| ATOM | 4965 | N | PRO | B | 346 | 33.925 | 50.996 | 108.410 | 1.00 | 38.14 | B | N |
| ATOM | 4966 | CD | PRO | B | 346 | 34.190 | 50.072 | 109.525 | 1.00 | 23.04 | B | C |
| ATOM | 4967 | CA | PRO | B | 346 | 33.650 | 52.346 | 108.916 | 1.00 | 38.14 | B | C |
| ATOM | 4968 | CB | PRO | B | 346 | 33.595 | 52.139 | 110.427 | 1.00 | 23.04 | B | C |
| ATOM | 4969 | CG | PRO | B | 346 | 34.553 | 51.026 | 110.647 | 1.00 | 23.04 | B | C |
| ATOM | 4970 | C | PRO | B | 346 | 34.695 | 53.400 | 108.503 | 1.00 | 38.14 | B | C |
| ATOM | 4971 | O | PRO | B | 346 | 34.445 | 54.612 | 108.555 | 1.00 | 38.14 | B | O |
| ATOM | 4972 | N | ASN | B | 347 | 35.864 | 52.929 | 108.085 | 1.00 | 41.84 | B | N |
| ATOM | 4973 | CA | ASN | B | 347 | 36.945 | 53.816 | 107.669 | 1.00 | 41.84 | B | C |
| ATOM | 4974 | CB | ASN | B | 347 | 38.290 | 53.242 | 108.107 | 1.00 | 43.09 | B | C |
| ATOM | 4975 | CG | ASN | B | 347 | 38.393 | 53.057 | 109.599 | 1.00 | 43.09 | B | C |
| ATOM | 4976 | OD1 | ASN | B | 347 | 39.398 | 52.540 | 110.095 | 1.00 | 43.09 | B | O |
| ATOM | 4977 | ND2 | ASN | B | 347 | 37.360 | 53.481 | 110.335 | 1.00 | 43.09 | B | N |
| ATOM | 4978 | C | ASN | B | 347 | 37.021 | 54.005 | 106.157 | 1.00 | 41.84 | B | C |
| ATOM | 4979 | O | ASN | B | 347 | 37.968 | 54.602 | 105.651 | 1.00 | 41.84 | B | O |
| ATOM | 4980 | N | VAL | B | 348 | 36.048 | 53.488 | 105.425 | 1.00 | 37.40 | B | N |
| ATOM | 4981 | CA | VAL | B | 348 | 36.118 | 53.619 | 103.991 | 1.00 | 37.40 | B | C |
| ATOM | 4982 | CB | VAL | B | 348 | 35.037 | 52.759 | 103.293 | 1.00 | 45.60 | B | C |
| ATOM | 4983 | CG1 | VAL | B | 348 | 33.656 | 53.418 | 103.387 | 1.00 | 45.60 | B | C |
| ATOM | 4984 | CG2 | VAL | B | 348 | 35.438 | 52.519 | 101.868 | 1.00 | 45.60 | B | C |
| ATOM | 4985 | C | VAL | B | 348 | 35.983 | 55.075 | 103.625 | 1.00 | 37.40 | B | C |
| ATOM | 4986 | O | VAL | B | 348 | 35.179 | 55.806 | 104.210 | 1.00 | 37.40 | B | O |
| ATOM | 4987 | N | LYS | B | 349 | 36.795 | 55.487 | 102.660 | 1.00 | 35.45 | B | N |
| ATOM | 4988 | CA | LYS | B | 349 | 36.822 | 56.863 | 102.196 | 1.00 | 35.45 | B | C |
| ATOM | 4989 | CB | LYS | B | 349 | 37.954 | 57.582 | 102.919 | 1.00 | 31.92 | B | C |
| ATOM | 4990 | CG | LYS | B | 349 | 37.606 | 58.901 | 103.563 | 1.00 | 31.92 | B | C |
| ATOM | 4991 | CD | LYS | B | 349 | 36.696 | 58.744 | 104.770 | 1.00 | 31.92 | B | C |
| ATOM | 4992 | CE | LYS | B | 349 | 36.695 | 60.011 | 105.627 | 1.00 | 31.92 | B | C |
| ATOM | 4993 | NZ | LYS | B | 349 | 35.784 | 59.892 | 106.810 | 1.00 | 31.92 | B | N |
| ATOM | 4994 | C | LYS | B | 349 | 37.101 | 56.853 | 100.697 | 1.00 | 35.45 | B | C |
| ATOM | 4995 | O | LYS | B | 349 | 37.668 | 55.886 | 100.165 | 1.00 | 35.45 | B | O |
| ATOM | 4996 | N | LEU | B | 350 | 36.699 | 57.924 | 100.016 | 1.00 | 29.15 | B | N |
| ATOM | 4997 | CA | LEU | B | 350 | 36.935 | 58.040 | 98.580 | 1.00 | 29.15 | B | C |
| ATOM | 4998 | CB | LEU | B | 350 | 36.004 | 59.078 | 97.962 | 1.00 | 18.06 | B | C |
| ATOM | 4999 | CG | LEU | B | 350 | 34.544 | 58.640 | 97.906 | 1.00 | 18.06 | B | C |
| ATOM | 5000 | CD1 | LEU | B | 350 | 33.728 | 59.624 | 97.079 | 1.00 | 18.06 | B | C |
| ATOM | 5001 | CD2 | LEU | B | 350 | 34.456 | 57.267 | 97.292 | 1.00 | 18.06 | B | C |
| ATOM | 5002 | C | LEU | B | 350 | 38.364 | 58.459 | 98.329 | 1.00 | 29.15 | B | C |
| ATOM | 5003 | O | LEU | B | 350 | 38.913 | 59.269 | 99.069 | 1.00 | 29.15 | B | O |
| ATOM | 5004 | N | PRO | B | 351 | 38.991 | 57.907 | 97.284 | 1.00 | 37.43 | B | N |
| ATOM | 5005 | CD | PRO | B | 351 | 38.428 | 57.099 | 96.191 | 1.00 | 26.12 | B | C |
| ATOM | 5006 | CA | PRO | B | 351 | 40.376 | 58.283 | 96.993 | 1.00 | 37.43 | B | C |
| ATOM | 5007 | CB | PRO | B | 351 | 40.666 | 57.516 | 95.704 | 1.00 | 26.12 | B | C |
| ATOM | 5008 | CG | PRO | B | 351 | 39.338 | 57.444 | 95.053 | 1.00 | 26.12 | B | C |
| ATOM | 5009 | C | PRO | B | 351 | 40.402 | 59.800 | 96.811 | 1.00 | 37.43 | B | C |
| ATOM | 5010 | O | PRO | B | 351 | 41.381 | 60.472 | 97.146 | 1.00 | 37.43 | B | O |
| ATOM | 5011 | N | ASN | B | 352 | 39.291 | 60.336 | 96.318 | 1.00 | 37.92 | B | N |
| ATOM | 5012 | CA | ASN | B | 352 | 39.187 | 61.771 | 96.104 | 1.00 | 37.92 | B | C |
| ATOM | 5013 | CB | ASN | B | 352 | 37.900 | 62.107 | 95.337 | 1.00 | 46.28 | B | C |
| ATOM | 5014 | CG | ASN | B | 352 | 36.801 | 62.640 | 96.236 | 1.00 | 46.28 | B | C |
| ATOM | 5015 | OD1 | ASN | B | 352 | 36.457 | 62.024 | 97.249 | 1.00 | 46.28 | B | O |
| ATOM | 5016 | ND2 | ASN | B | 352 | 36.233 | 63.790 | 95.864 | 1.00 | 46.28 | B | N |
| ATOM | 5017 | C | ASN | B | 352 | 39.268 | 62.567 | 97.413 | 1.00 | 37.92 | B | C |
| ATOM | 5018 | O | ASN | B | 352 | 39.404 | 63.783 | 97.403 | 1.00 | 37.92 | B | O |
| ATOM | 5019 | N | GLY | B | 353 | 39.181 | 61.878 | 98.541 | 1.00 | 27.22 | B | N |

| | | | | | | | | | | | | |
|---|---|---|---|---|---|---|---|---|---|---|---|---|
| ATOM | 5020 | CA | GLY | B | 353 | 39.281 | 62.556 | 99.818 | 1.00 | 27.22 | B | C |
| ATOM | 5021 | C | GLY | B | 353 | 38.005 | 62.643 | 100.618 | 1.00 | 27.22 | B | C |
| ATOM | 5022 | O | GLY | B | 353 | 38.039 | 62.497 | 101.844 | 1.00 | 27.22 | B | O |
| ATOM | 5023 | N | ARG | B | 354 | 36.876 | 62.884 | 99.957 | 1.00 | 28.21 | B | N |
| ATOM | 5024 | CA | ARG | B | 354 | 35.637 | 62.986 | 100.703 | 1.00 | 28.21 | B | C |
| ATOM | 5025 | CB | ARG | B | 354 | 34.632 | 63.866 | 99.958 | 1.00 | 48.05 | B | C |
| ATOM | 5026 | CG | ARG | B | 354 | 34.137 | 63.366 | 98.645 | 1.00 | 48.05 | B | C |
| ATOM | 5027 | CD | ARG | B | 354 | 33.250 | 64.441 | 98.002 | 1.00 | 48.05 | B | C |
| ATOM | 5028 | NE | ARG | B | 354 | 34.030 | 65.601 | 97.554 | 1.00 | 48.05 | B | N |
| ATOM | 5029 | CZ | ARG | B | 354 | 33.544 | 66.608 | 96.823 | 1.00 | 48.05 | B | C |
| ATOM | 5030 | NH1 | ARG | B | 354 | 32.266 | 66.594 | 96.455 | 1.00 | 48.05 | B | N |
| ATOM | 5031 | NH2 | ARG | B | 354 | 34.339 | 67.620 | 96.459 | 1.00 | 48.05 | B | N |
| ATOM | 5032 | C | ARG | B | 354 | 35.033 | 61.641 | 101.101 | 1.00 | 28.21 | B | C |
| ATOM | 5033 | O | ARG | B | 354 | 35.621 | 60.594 | 100.846 | 1.00 | 28.21 | B | O |
| ATOM | 5034 | N | ASP | B | 355 | 33.886 | 61.680 | 101.771 | 1.00 | 53.18 | B | N |
| ATOM | 5035 | CA | ASP | B | 355 | 33.198 | 60.474 | 102.220 | 1.00 | 53.18 | B | C |
| ATOM | 5036 | CB | ASP | B | 355 | 32.170 | 60.827 | 103.286 | 1.00 | 54.41 | B | C |
| ATOM | 5037 | CG | ASP | B | 355 | 32.778 | 60.931 | 104.649 | 1.00 | 54.41 | B | C |
| ATOM | 5038 | OD1 | ASP | B | 355 | 32.000 | 61.075 | 105.622 | 1.00 | 54.41 | B | O |
| ATOM | 5039 | OD2 | ASP | B | 355 | 34.030 | 60.861 | 104.728 | 1.00 | 54.41 | B | O |
| ATOM | 5040 | C | ASP | B | 355 | 32.507 | 59.664 | 101.137 | 1.00 | 53.18 | B | C |
| ATOM | 5041 | O | ASP | B | 355 | 32.314 | 60.131 | 100.005 | 1.00 | 53.18 | B | O |
| ATOM | 5042 | N | THR | B | 356 | 32.154 | 58.437 | 101.496 | 1.00 | 33.81 | B | N |
| ATOM | 5043 | CA | THR | B | 356 | 31.441 | 57.568 | 100.581 | 1.00 | 33.81 | B | C |
| ATOM | 5044 | CB | THR | B | 356 | 31.395 | 56.107 | 101.093 | 1.00 | 44.24 | B | C |
| ATOM | 5045 | OG1 | THR | B | 356 | 30.871 | 56.078 | 102.427 | 1.00 | 44.24 | B | O |
| ATOM | 5046 | CG2 | THR | B | 356 | 32.767 | 55.493 | 101.095 | 1.00 | 44.24 | B | C |
| ATOM | 5047 | C | THR | B | 356 | 30.010 | 58.109 | 100.539 | 1.00 | 33.81 | B | C |
| ATOM | 5048 | O | THR | B | 356 | 29.555 | 58.770 | 101.479 | 1.00 | 33.81 | B | O |
| ATOM | 5049 | N | PRO | B | 357 | 29.286 | 57.872 | 99.439 | 1.00 | 27.32 | B | N |
| ATOM | 5050 | CD | PRO | B | 357 | 29.655 | 57.397 | 98.094 | 1.00 | 23.87 | B | C |
| ATOM | 5051 | CA | PRO | B | 357 | 27.926 | 58.396 | 99.450 | 1.00 | 27.32 | B | C |
| ATOM | 5052 | CB | PRO | B | 357 | 27.563 | 58.431 | 97.971 | 1.00 | 23.87 | B | C |
| ATOM | 5053 | CG | PRO | B | 357 | 28.314 | 57.276 | 97.419 | 1.00 | 23.87 | B | C |
| ATOM | 5054 | C | PRO | B | 357 | 27.054 | 57.443 | 100.248 | 1.00 | 27.32 | B | C |
| ATOM | 5055 | O | PRO | B | 357 | 27.545 | 56.444 | 100.779 | 1.00 | 27.32 | B | O |
| ATOM | 5056 | N | ALA | B | 358 | 25.766 | 57.752 | 100.337 | 1.00 | 29.72 | B | N |
| ATOM | 5057 | CA | ALA | B | 358 | 24.825 | 56.906 | 101.067 | 1.00 | 29.72 | B | C |
| ATOM | 5058 | CB | ALA | B | 358 | 23.414 | 57.492 | 100.988 | 1.00 | 40.70 | B | C |
| ATOM | 5059 | C | ALA | B | 358 | 24.829 | 55.511 | 100.467 | 1.00 | 29.72 | B | C |
| ATOM | 5060 | O | ALA | B | 358 | 24.567 | 55.339 | 99.277 | 1.00 | 29.72 | B | O |
| ATOM | 5061 | N | LEU | B | 359 | 25.104 | 54.521 | 101.301 | 1.00 | 24.10 | B | N |
| ATOM | 5062 | CA | LEU | B | 359 | 25.156 | 53.147 | 100.856 | 1.00 | 24.10 | B | C |
| ATOM | 5063 | CB | LEU | B | 359 | 26.600 | 52.649 | 100.977 | 1.00 | 21.04 | B | C |
| ATOM | 5064 | CG | LEU | B | 359 | 27.603 | 52.739 | 99.814 | 1.00 | 21.04 | B | C |
| ATOM | 5065 | CD1 | LEU | B | 359 | 27.244 | 53.817 | 98.824 | 1.00 | 21.04 | B | C |
| ATOM | 5066 | CD2 | LEU | B | 359 | 28.989 | 52.968 | 100.397 | 1.00 | 21.04 | B | C |
| ATOM | 5067 | C | LEU | B | 359 | 24.232 | 52.264 | 101.670 | 1.00 | 24.10 | B | C |
| ATOM | 5068 | O | LEU | B | 359 | 23.811 | 51.199 | 101.212 | 1.00 | 24.10 | B | O |
| ATOM | 5069 | N | PHE | B | 360 | 23.883 | 52.730 | 102.865 | 1.00 | 33.64 | B | N |
| ATOM | 5070 | CA | PHE | B | 360 | 23.063 | 51.930 | 103.768 | 1.00 | 33.64 | B | C |
| ATOM | 5071 | CB | PHE | B | 360 | 23.863 | 51.731 | 105.050 | 1.00 | 22.54 | B | C |
| ATOM | 5072 | CG | PHE | B | 360 | 25.326 | 51.577 | 104.810 | 1.00 | 22.54 | B | C |
| ATOM | 5073 | CD1 | PHE | B | 360 | 25.820 | 50.456 | 104.158 | 1.00 | 22.54 | B | C |
| ATOM | 5074 | CD2 | PHE | B | 360 | 26.212 | 52.579 | 105.198 | 1.00 | 22.54 | B | C |
| ATOM | 5075 | CE1 | PHE | B | 360 | 27.184 | 50.335 | 103.890 | 1.00 | 22.54 | B | C |
| ATOM | 5076 | CE2 | PHE | B | 360 | 27.575 | 52.479 | 104.941 | 1.00 | 22.54 | B | C |
| ATOM | 5077 | CZ | PHE | B | 360 | 28.068 | 51.355 | 104.285 | 1.00 | 22.54 | B | C |
| ATOM | 5078 | C | PHE | B | 360 | 21.644 | 52.395 | 104.107 | 1.00 | 33.64 | B | C |
| ATOM | 5079 | O | PHE | B | 360 | 20.973 | 51.755 | 104.921 | 1.00 | 33.64 | B | O |
| ATOM | 5080 | N | ASN | B | 361 | 21.175 | 53.479 | 103.493 | 1.00 | 25.01 | B | N |
| ATOM | 5081 | CA | ASN | B | 361 | 19.833 | 53.979 | 103.793 | 1.00 | 25.01 | B | C |
| ATOM | 5082 | CB | ASN | B | 361 | 19.686 | 55.455 | 103.362 | 1.00 | 20.81 | B | C |
| ATOM | 5083 | CG | ASN | B | 361 | 20.113 | 55.705 | 101.929 | 1.00 | 20.81 | B | C |
| ATOM | 5084 | OD1 | ASN | B | 361 | 20.985 | 55.029 | 101.400 | 1.00 | 20.81 | B | O |
| ATOM | 5085 | ND2 | ASN | B | 361 | 19.511 | 56.698 | 101.305 | 1.00 | 20.81 | B | N |
| ATOM | 5086 | C | ASN | B | 361 | 18.771 | 53.115 | 103.119 | 1.00 | 25.01 | B | C |

| | | | | | | | | | | | | |
|---|---|---|---|---|---|---|---|---|---|---|---|---|
| ATOM | 5087 | O | ASN | B | 361 | 17.941 | 53.604 | 102.370 | 1.00 | 25.01 | B | O |
| ATOM | 5088 | N | PHE | B | 362 | 18.816 | 51.817 | 103.386 | 1.00 | 18.99 | B | N |
| ATOM | 5089 | CA | PHE | B | 362 | 17.853 | 50.877 | 102.835 | 1.00 | 18.99 | B | C |
| ATOM | 5090 | CB | PHE | B | 362 | 18.232 | 49.460 | 103.223 | 1.00 | 24.18 | B | C |
| ATOM | 5091 | CG | PHE | B | 362 | 19.279 | 48.854 | 102.365 | 1.00 | 24.18 | B | C |
| ATOM | 5092 | CD1 | PHE | B | 362 | 18.930 | 48.143 | 101.226 | 1.00 | 24.18 | B | C |
| ATOM | 5093 | CD2 | PHE | B | 362 | 20.620 | 48.947 | 102.713 | 1.00 | 24.18 | B | C |
| ATOM | 5094 | CE1 | PHE | B | 362 | 19.902 | 47.517 | 100.446 | 1.00 | 24.18 | B | C |
| ATOM | 5095 | CE2 | PHE | B | 362 | 21.610 | 48.326 | 101.940 | 1.00 | 24.18 | B | C |
| ATOM | 5096 | CZ | PHE | B | 362 | 21.249 | 47.607 | 100.804 | 1.00 | 24.18 | B | C |
| ATOM | 5097 | C | PHE | B | 362 | 16.479 | 51.144 | 103.409 | 1.00 | 18.99 | B | C |
| ATOM | 5098 | O | PHE | B | 362 | 16.362 | 51.782 | 104.430 | 1.00 | 18.99 | B | O |
| ATOM | 5099 | N | THR | B | 363 | 15.448 | 50.639 | 102.745 | 1.00 | 32.21 | B | N |
| ATOM | 5100 | CA | THR | B | 363 | 14.077 | 50.772 | 103.220 | 1.00 | 32.21 | B | C |
| ATOM | 5101 | CB | THR | B | 363 | 13.210 | 51.730 | 102.358 | 1.00 | 12.54 | B | C |
| ATOM | 5102 | OG1 | THR | B | 363 | 13.240 | 51.312 | 100.991 | 1.00 | 12.54 | B | O |
| ATOM | 5103 | CG2 | THR | B | 363 | 13.699 | 53.146 | 102.467 | 1.00 | 12.54 | B | C |
| ATOM | 5104 | C | THR | B | 363 | 13.471 | 49.382 | 103.145 | 1.00 | 32.21 | B | C |
| ATOM | 5105 | O | THR | B | 363 | 14.001 | 48.505 | 102.457 | 1.00 | 32.21 | B | O |
| ATOM | 5106 | N | THR | B | 364 | 12.378 | 49.169 | 103.869 | 1.00 | 39.95 | B | N |
| ATOM | 5107 | CA | THR | B | 364 | 11.736 | 47.866 | 103.869 | 1.00 | 39.95 | B | C |
| ATOM | 5108 | CB | THR | B | 364 | 10.474 | 47.855 | 104.755 | 1.00 | 36.28 | B | C |
| ATOM | 5109 | OG1 | THR | B | 364 | 9.994 | 49.193 | 104.918 | 1.00 | 36.28 | B | O |
| ATOM | 5110 | CG2 | THR | B | 364 | 10.784 | 47.281 | 106.115 | 1.00 | 36.28 | B | C |
| ATOM | 5111 | C | THR | B | 364 | 11.392 | 47.431 | 102.448 | 1.00 | 39.95 | B | C |
| ATOM | 5112 | O | THR | B | 364 | 11.537 | 46.254 | 102.112 | 1.00 | 39.95 | B | O |
| ATOM | 5113 | N | GLN | B | 365 | 10.965 | 48.374 | 101.609 | 1.00 | 20.67 | B | N |
| ATOM | 5114 | CA | GLN | B | 365 | 10.623 | 48.050 | 100.228 | 1.00 | 20.67 | B | C |
| ATOM | 5115 | CB | GLN | B | 365 | 10.228 | 49.308 | 99.445 | 1.00 | 32.56 | B | C |
| ATOM | 5116 | CG | GLN | B | 365 | 8.875 | 49.228 | 98.735 | 1.00 | 32.56 | B | C |
| ATOM | 5117 | CD | GLN | B | 365 | 8.821 | 48.177 | 97.652 | 1.00 | 32.56 | B | C |
| ATOM | 5118 | OE1 | GLN | B | 365 | 9.042 | 46.986 | 97.896 | 1.00 | 32.56 | B | O |
| ATOM | 5119 | NE2 | GLN | B | 365 | 8.524 | 48.611 | 96.443 | 1.00 | 32.56 | B | N |
| ATOM | 5120 | C | GLN | B | 365 | 11.821 | 47.405 | 99.551 | 1.00 | 20.67 | B | C |
| ATOM | 5121 | O | GLN | B | 365 | 11.704 | 46.382 | 98.885 | 1.00 | 20.67 | B | O |
| ATOM | 5122 | N | GLU | B | 366 | 12.981 | 48.014 | 99.759 | 1.00 | 42.00 | B | N |
| ATOM | 5123 | CA | GLU | B | 366 | 14.237 | 47.586 | 99.167 | 1.00 | 42.00 | B | C |
| ATOM | 5124 | CB | GLU | B | 366 | 15.258 | 48.705 | 99.353 | 1.00 | 23.99 | B | C |
| ATOM | 5125 | CG | GLU | B | 366 | 16.543 | 48.522 | 98.588 | 1.00 | 23.99 | B | C |
| ATOM | 5126 | CD | GLU | B | 366 | 17.356 | 49.794 | 98.538 | 1.00 | 23.99 | B | C |
| ATOM | 5127 | OE1 | GLU | B | 366 | 17.114 | 50.671 | 99.396 | 1.00 | 23.99 | B | O |
| ATOM | 5128 | OE2 | GLU | B | 366 | 18.231 | 49.913 | 97.654 | 1.00 | 23.99 | B | O |
| ATOM | 5129 | C | GLU | B | 366 | 14.774 | 46.268 | 99.710 | 1.00 | 42.00 | B | C |
| ATOM | 5130 | O | GLU | B | 366 | 15.489 | 45.539 | 99.004 | 1.00 | 42.00 | B | O |
| ATOM | 5131 | N | LEU | B | 367 | 14.416 | 45.954 | 100.954 | 1.00 | 28.86 | B | N |
| ATOM | 5132 | CA | LEU | B | 367 | 14.883 | 44.724 | 101.587 | 1.00 | 28.86 | B | C |
| ATOM | 5133 | CB | LEU | B | 367 | 15.217 | 44.975 | 103.057 | 1.00 | 33.61 | B | C |
| ATOM | 5134 | CG | LEU | B | 367 | 16.281 | 46.007 | 103.398 | 1.00 | 33.61 | B | C |
| ATOM | 5135 | CD1 | LEU | B | 367 | 16.092 | 46.427 | 104.839 | 1.00 | 33.61 | B | C |
| ATOM | 5136 | CD2 | LEU | B | 367 | 17.667 | 45.422 | 103.148 | 1.00 | 33.61 | B | C |
| ATOM | 5137 | C | LEU | B | 367 | 13.870 | 43.590 | 101.509 | 1.00 | 28.86 | B | C |
| ATOM | 5138 | O | LEU | B | 367 | 14.207 | 42.432 | 101.747 | 1.00 | 28.86 | B | O |
| ATOM | 5139 | N | SER | B | 368 | 12.637 | 43.937 | 101.166 | 1.00 | 26.89 | B | N |
| ATOM | 5140 | CA | SER | B | 368 | 11.534 | 42.993 | 101.088 | 1.00 | 26.89 | B | C |
| ATOM | 5141 | CB | SER | B | 368 | 10.387 | 43.603 | 100.301 | 1.00 | 47.59 | B | C |
| ATOM | 5142 | OG | SER | B | 368 | 10.649 | 43.538 | 98.911 | 1.00 | 47.59 | B | O |
| ATOM | 5143 | C | SER | B | 368 | 11.846 | 41.639 | 100.490 | 1.00 | 26.89 | B | C |
| ATOM | 5144 | O | SER | B | 368 | 11.273 | 40.646 | 100.905 | 1.00 | 26.89 | B | O |
| ATOM | 5145 | N | SER | B | 369 | 12.729 | 41.580 | 99.505 | 1.00 | 43.58 | B | N |
| ATOM | 5146 | CA | SER | B | 369 | 13.045 | 40.292 | 98.894 | 1.00 | 43.58 | B | C |
| ATOM | 5147 | CB | SER | B | 369 | 13.963 | 40.475 | 97.690 | 1.00 | 19.53 | B | C |
| ATOM | 5148 | OG | SER | B | 369 | 15.266 | 40.838 | 98.094 | 1.00 | 19.53 | B | O |
| ATOM | 5149 | C | SER | B | 369 | 13.699 | 39.306 | 99.858 | 1.00 | 43.58 | B | C |
| ATOM | 5150 | O | SER | B | 369 | 13.681 | 38.102 | 99.622 | 1.00 | 43.58 | B | O |
| ATOM | 5151 | N | ASN | B | 370 | 14.287 | 39.817 | 100.933 | 1.00 | 32.25 | B | N |
| ATOM | 5152 | CA | ASN | B | 370 | 14.943 | 38.977 | 101.927 | 1.00 | 32.25 | B | C |
| ATOM | 5153 | CB | ASN | B | 370 | 16.120 | 38.242 | 101.285 | 1.00 | 29.13 | B | C |

| | | | | | | | | | | | | |
|---|---|---|---|---|---|---|---|---|---|---|---|---|
| ATOM | 5154 | CG | ASN | B | 370 | 16.924 | 37.439 | 102.274 | 1.00 | 29.13 | B | C |
| ATOM | 5155 | OD1 | ASN | B | 370 | 17.462 | 36.398 | 101.936 | 1.00 | 29.13 | B | O |
| ATOM | 5156 | ND2 | ASN | B | 370 | 17.027 | 37.927 | 103.495 | 1.00 | 29.13 | B | N |
| ATOM | 5157 | C | ASN | B | 370 | 15.409 | 39.898 | 103.053 | 1.00 | 32.25 | B | C |
| ATOM | 5158 | O | ASN | B | 370 | 16.583 | 40.282 | 103.129 | 1.00 | 32.25 | B | O |
| ATOM | 5159 | N | PRO | B | 371 | 14.475 | 40.262 | 103.949 | 1.00 | 38.02 | B | N |
| ATOM | 5160 | CD | PRO | B | 371 | 13.060 | 39.841 | 103.948 | 1.00 | 14.24 | B | C |
| ATOM | 5161 | CA | PRO | B | 371 | 14.749 | 41.148 | 105.081 | 1.00 | 38.02 | B | C |
| ATOM | 5162 | CB | PRO | B | 371 | 13.438 | 41.121 | 105.865 | 1.00 | 14.24 | B | C |
| ATOM | 5163 | CG | PRO | B | 371 | 12.409 | 40.901 | 104.785 | 1.00 | 14.24 | B | C |
| ATOM | 5164 | C | PRO | B | 371 | 15.947 | 40.761 | 105.931 | 1.00 | 38.02 | B | C |
| ATOM | 5165 | O | PRO | B | 371 | 16.752 | 41.616 | 106.284 | 1.00 | 38.02 | B | O |
| ATOM | 5166 | N | PRO | B | 372 | 16.093 | 39.471 | 106.264 | 1.00 | 23.96 | B | N |
| ATOM | 5167 | CD | PRO | B | 372 | 15.271 | 38.300 | 105.914 | 1.00 | 12.61 | B | C |
| ATOM | 5168 | CA | PRO | B | 372 | 17.235 | 39.072 | 107.087 | 1.00 | 23.96 | B | C |
| ATOM | 5169 | CB | PRO | B | 372 | 17.268 | 37.562 | 106.917 | 1.00 | 12.61 | B | C |
| ATOM | 5170 | CG | PRO | B | 372 | 15.829 | 37.229 | 106.830 | 1.00 | 12.61 | B | C |
| ATOM | 5171 | C | PRO | B | 372 | 18.543 | 39.733 | 106.685 | 1.00 | 23.96 | B | C |
| ATOM | 5172 | O | PRO | B | 372 | 19.339 | 40.099 | 107.537 | 1.00 | 23.96 | B | O |
| ATOM | 5173 | N | LEU | B | 373 | 18.744 | 39.901 | 105.382 | 1.00 | 20.62 | B | N |
| ATOM | 5174 | CA | LEU | B | 373 | 19.953 | 40.508 | 104.837 | 1.00 | 20.62 | B | C |
| ATOM | 5175 | CB | LEU | B | 373 | 19.738 | 40.763 | 103.344 | 1.00 | 27.09 | B | C |
| ATOM | 5176 | CG | LEU | B | 373 | 20.377 | 39.777 | 102.366 | 1.00 | 27.09 | B | C |
| ATOM | 5177 | CD1 | LEU | B | 373 | 20.648 | 38.458 | 103.076 | 1.00 | 27.09 | B | C |
| ATOM | 5178 | CD2 | LEU | B | 373 | 19.472 | 39.603 | 101.151 | 1.00 | 27.09 | B | C |
| ATOM | 5179 | C | LEU | B | 373 | 20.370 | 41.810 | 105.528 | 1.00 | 20.62 | B | C |
| ATOM | 5180 | O | LEU | B | 373 | 21.545 | 42.181 | 105.542 | 1.00 | 20.62 | B | O |
| ATOM | 5181 | N | ALA | B | 374 | 19.403 | 42.502 | 106.106 | 1.00 | 32.24 | B | N |
| ATOM | 5182 | CA | ALA | B | 374 | 19.686 | 43.762 | 106.767 | 1.00 | 32.24 | B | C |
| ATOM | 5183 | CB | ALA | B | 374 | 18.434 | 44.289 | 107.444 | 1.00 | 11.12 | B | C |
| ATOM | 5184 | C | ALA | B | 374 | 20.799 | 43.624 | 107.784 | 1.00 | 32.24 | B | C |
| ATOM | 5185 | O | ALA | B | 374 | 21.491 | 44.593 | 108.078 | 1.00 | 32.24 | B | O |
| ATOM | 5186 | N | THR | B | 375 | 20.985 | 42.425 | 108.320 | 1.00 | 23.81 | B | N |
| ATOM | 5187 | CA | THR | B | 375 | 22.031 | 42.231 | 109.309 | 1.00 | 23.81 | B | C |
| ATOM | 5188 | CB | THR | B | 375 | 22.074 | 40.813 | 109.840 | 1.00 | 40.95 | B | C |
| ATOM | 5189 | OG1 | THR | B | 375 | 20.991 | 40.629 | 110.757 | 1.00 | 40.95 | B | O |
| ATOM | 5190 | CG2 | THR | B | 375 | 23.398 | 40.550 | 110.550 | 1.00 | 40.95 | B | C |
| ATOM | 5191 | C | THR | B | 375 | 23.370 | 42.556 | 108.709 | 1.00 | 23.81 | B | C |
| ATOM | 5192 | O | THR | B | 375 | 24.238 | 43.106 | 109.376 | 1.00 | 23.81 | B | O |
| ATOM | 5193 | N | ILE | B | 376 | 23.535 | 42.211 | 107.436 | 1.00 | 35.60 | B | N |
| ATOM | 5194 | CA | ILE | B | 376 | 24.786 | 42.482 | 106.746 | 1.00 | 35.60 | B | C |
| ATOM | 5195 | CB | ILE | B | 376 | 25.087 | 41.443 | 105.665 | 1.00 | 27.48 | B | C |
| ATOM | 5196 | CG2 | ILE | B | 376 | 26.252 | 41.899 | 104.839 | 1.00 | 27.48 | B | C |
| ATOM | 5197 | CG1 | ILE | B | 376 | 25.404 | 40.097 | 106.301 | 1.00 | 27.48 | B | C |
| ATOM | 5198 | CD1 | ILE | B | 376 | 25.493 | 38.969 | 105.308 | 1.00 | 27.48 | B | C |
| ATOM | 5199 | C | ILE | B | 376 | 24.753 | 43.850 | 106.065 | 1.00 | 35.60 | B | C |
| ATOM | 5200 | O | ILE | B | 376 | 25.602 | 44.702 | 106.310 | 1.00 | 35.60 | B | O |
| ATOM | 5201 | N | LEU | B | 377 | 23.757 | 44.047 | 105.211 | 1.00 | 28.84 | B | N |
| ATOM | 5202 | CA | LEU | B | 377 | 23.628 | 45.282 | 104.453 | 1.00 | 28.84 | B | C |
| ATOM | 5203 | CB | LEU | B | 377 | 22.255 | 45.343 | 103.775 | 1.00 | 29.20 | B | C |
| ATOM | 5204 | CG | LEU | B | 377 | 22.116 | 44.627 | 102.426 | 1.00 | 29.20 | B | C |
| ATOM | 5205 | CD1 | LEU | B | 377 | 23.316 | 43.724 | 102.169 | 1.00 | 29.20 | B | C |
| ATOM | 5206 | CD2 | LEU | B | 377 | 20.816 | 43.851 | 102.401 | 1.00 | 29.20 | B | C |
| ATOM | 5207 | C | LEU | B | 377 | 23.876 | 46.566 | 105.233 | 1.00 | 28.84 | B | C |
| ATOM | 5208 | O | LEU | B | 377 | 24.468 | 47.504 | 104.700 | 1.00 | 28.84 | B | O |
| ATOM | 5209 | N | ILE | B | 378 | 23.420 | 46.608 | 106.489 | 1.00 | 35.49 | B | N |
| ATOM | 5210 | CA | ILE | B | 378 | 23.595 | 47.785 | 107.348 | 1.00 | 35.49 | B | C |
| ATOM | 5211 | CB | ILE | B | 378 | 22.301 | 48.166 | 108.084 | 1.00 | 25.32 | B | C |
| ATOM | 5212 | CG2 | ILE | B | 378 | 22.554 | 49.375 | 108.968 | 1.00 | 25.32 | B | C |
| ATOM | 5213 | CG1 | ILE | B | 378 | 21.196 | 48.483 | 107.080 | 1.00 | 25.32 | B | C |
| ATOM | 5214 | CD1 | ILE | B | 378 | 19.822 | 48.668 | 107.725 | 1.00 | 25.32 | B | C |
| ATOM | 5215 | C | ILE | B | 378 | 24.663 | 47.574 | 108.419 | 1.00 | 35.49 | B | C |
| ATOM | 5216 | O | ILE | B | 378 | 24.357 | 47.175 | 109.531 | 1.00 | 35.49 | B | O |
| ATOM | 5217 | N | PRO | B | 379 | 25.928 | 47.879 | 108.113 | 1.00 | 33.91 | B | N |
| ATOM | 5218 | CD | PRO | B | 379 | 26.425 | 48.704 | 107.004 | 1.00 | 37.92 | B | C |
| ATOM | 5219 | CA | PRO | B | 379 | 26.976 | 47.679 | 109.112 | 1.00 | 33.91 | B | C |
| ATOM | 5220 | CB | PRO | B | 379 | 28.203 | 48.294 | 108.449 | 1.00 | 37.92 | B | C |

| | | | | | | | | | | | | |
|---|---|---|---|---|---|---|---|---|---|---|---|---|
| ATOM | 5221 | CG | PRO | B | 379 | 27.619 | 49.380 | 107.631 | 1.00 | 37.92 | B | C |
| ATOM | 5222 | C | PRO | B | 379 | 26.672 | 48.290 | 110.473 | 1.00 | 33.91 | B | C |
| ATOM | 5223 | O | PRO | B | 379 | 25.893 | 49.245 | 110.582 | 1.00 | 33.91 | B | O |
| ATOM | 5224 | N | PRO | B | 380 | 27.294 | 47.738 | 111.535 | 1.00 | 40.78 | B | N |
| ATOM | 5225 | CD | PRO | B | 380 | 28.319 | 46.680 | 111.472 | 1.00 | 32.38 | B | C |
| ATOM | 5226 | CA | PRO | B | 380 | 27.125 | 48.187 | 112.918 | 1.00 | 40.78 | B | C |
| ATOM | 5227 | CB | PRO | B | 380 | 28.292 | 47.524 | 113.639 | 1.00 | 32.38 | B | C |
| ATOM | 5228 | CG | PRO | B | 380 | 28.436 | 46.265 | 112.926 | 1.00 | 32.38 | B | C |
| ATOM | 5229 | C | PRO | B | 380 | 27.168 | 49.715 | 113.057 | 1.00 | 40.78 | B | C |
| ATOM | 5230 | O | PRO | B | 380 | 26.228 | 50.345 | 113.549 | 1.00 | 40.78 | B | O |
| ATOM | 5231 | N | HIS | B | 381 | 28.263 | 50.306 | 112.597 | 1.00 | 34.22 | B | N |
| ATOM | 5232 | CA | HIS | B | 381 | 28.455 | 51.747 | 112.698 | 1.00 | 34.22 | B | C |
| ATOM | 5233 | CB | HIS | B | 381 | 29.843 | 52.108 | 112.214 | 1.00 | 33.19 | B | C |
| ATOM | 5234 | CG | HIS | B | 381 | 29.983 | 52.023 | 110.728 | 1.00 | 33.19 | B | C |
| ATOM | 5235 | CD2 | HIS | B | 381 | 30.511 | 51.061 | 109.936 | 1.00 | 33.19 | B | C |
| ATOM | 5236 | ND1 | HIS | B | 381 | 29.509 | 53.000 | 109.881 | 1.00 | 33.19 | B | N |
| ATOM | 5237 | CE1 | HIS | B | 381 | 29.744 | 52.647 | 108.631 | 1.00 | 33.19 | B | C |
| ATOM | 5238 | NE2 | HIS | B | 381 | 30.351 | 51.474 | 108.637 | 1.00 | 33.19 | B | N |
| ATOM | 5239 | C | HIS | B | 381 | 27.452 | 52.637 | 111.912 | 1.00 | 34.22 | B | C |
| ATOM | 5240 | O | HIS | B | 381 | 27.422 | 53.851 | 112.106 | 1.00 | 34.22 | B | O |
| ATOM | 5241 | N | ALA | B | 382 | 26.651 | 52.068 | 111.016 | 1.00 | 58.90 | B | N |
| ATOM | 5242 | CA | ALA | B | 382 | 25.717 | 52.897 | 110.239 | 1.00 | 58.90 | B | C |
| ATOM | 5243 | CB | ALA | B | 382 | 25.610 | 52.352 | 108.810 | 1.00 | 24.34 | B | C |
| ATOM | 5244 | C | ALA | B | 382 | 24.316 | 53.028 | 110.858 | 1.00 | 58.90 | B | C |
| ATOM | 5245 | O | ALA | B | 382 | 23.856 | 52.055 | 111.506 | 1.00 | 58.90 | B | O |
| ATOM | 5246 | OXT | ALA | B | 382 | 23.675 | 54.089 | 110.657 | 1.00 | 24.34 | B | O |
| TER | 5247 | | ALA | B | 382 | | | | | | B | |
| ATOM | 5248 | O | HOH | W | 3 | 15.375 | 58.039 | 78.308 | 1.00 | 28.96 | W | O |
| ATOM | 5249 | O | HOH | W | 2 | 36.184 | 58.995 | 88.945 | 1.00 | 28.59 | W | O |
| ATOM | 5250 | O | HOH | W | 4 | 16.766 | 58.125 | 81.755 | 1.00 | 22.88 | W | O |
| ATOM | 5251 | O | HOH | W | 5 | 26.239 | 46.976 | 102.908 | 1.00 | 41.98 | W | O |
| ATOM | 5252 | O | HOH | W | 6 | 22.272 | 38.560 | 70.890 | 1.00 | 25.85 | W | O |
| ATOM | 5253 | O | HOH | W | 7 | 19.070 | 41.701 | 66.616 | 1.00 | 21.64 | W | O |
| TER | 5254 | | HOH | W | 7 | | | | | | W | |
| ATOM | 5255 | C1 | INH | I | 1 | 40.782 | 39.301 | 55.233 | 1.00 | 45.17 | I | C |
| ATOM | 5256 | N2 | INH | I | 1 | 40.469 | 38.584 | 56.389 | 1.00 | 45.17 | I | N |
| ATOM | 5257 | C3 | INH | I | 1 | 40.087 | 37.234 | 56.333 | 1.00 | 45.17 | I | C |
| ATOM | 5258 | N4 | INH | I | 1 | 40.040 | 36.640 | 55.063 | 1.00 | 45.17 | I | N |
| ATOM | 5259 | C5 | INH | I | 1 | 40.325 | 37.279 | 53.869 | 1.00 | 45.17 | I | C |
| ATOM | 5260 | C6 | INH | I | 1 | 40.713 | 38.659 | 53.868 | 1.00 | 45.17 | I | C |
| ATOM | 5261 | C7 | INH | I | 1 | 41.036 | 39.402 | 52.660 | 1.00 | 45.17 | I | C |
| ATOM | 5262 | C8 | INH | I | 1 | 41.455 | 40.820 | 52.793 | 1.00 | 45.17 | I | C |
| ATOM | 5263 | C9 | INH | I | 1 | 41.544 | 41.472 | 54.114 | 1.00 | 45.17 | I | C |
| ATOM | 5264 | C10 | INH | I | 1 | 41.203 | 40.697 | 55.322 | 1.00 | 45.17 | I | C |
| ATOM | 5265 | C15 | INH | I | 1 | 39.725 | 36.472 | 57.531 | 1.00 | 45.17 | I | C |
| ATOM | 5266 | C16 | INH | I | 1 | 40.267 | 36.940 | 58.795 | 1.00 | 45.17 | I | C |
| ATOM | 5267 | C17 | INH | I | 1 | 39.896 | 36.180 | 59.952 | 1.00 | 45.17 | I | C |
| ATOM | 5268 | N18 | INH | I | 1 | 39.044 | 35.040 | 59.824 | 1.00 | 45.17 | I | N |
| ATOM | 5269 | C19 | INH | I | 1 | 38.512 | 34.573 | 58.603 | 1.00 | 45.17 | I | C |
| ATOM | 5270 | C20 | INH | I | 1 | 38.846 | 35.296 | 57.390 | 1.00 | 45.17 | I | C |
| ATOM | 5271 | N25 | INH | I | 1 | 40.263 | 36.633 | 52.626 | 1.00 | 45.17 | I | N |
| ATOM | 5272 | C26 | INH | I | 1 | 39.762 | 35.254 | 52.406 | 1.00 | 45.17 | I | C |
| ATOM | 5273 | N27 | INH | I | 1 | 40.098 | 34.408 | 51.423 | 1.00 | 45.17 | I | N |
| ATOM | 5274 | N1 | INH | I | 1 | 39.465 | 33.241 | 51.604 | 1.00 | 45.17 | I | N |
| ATOM | 5275 | C29 | INH | I | 1 | 38.695 | 33.262 | 52.689 | 1.00 | 45.17 | I | C |
| ATOM | 5276 | C30 | INH | I | 1 | 38.848 | 34.534 | 53.242 | 1.00 | 45.17 | I | C |
| ATOM | 5277 | C33 | INH | I | 1 | 37.818 | 32.194 | 53.256 | 1.00 | 45.17 | I | C |
| TER | 5278 | | INH | I | 1 | | | | | | I | |
| END | | | | | | | | | | | | |

# FIG. 5

| | | Atom Type | Resid | | # | X | Y | Z | Occ | B | | |
|---|---|---|---|---|---|---|---|---|---|---|---|---|
| ATOM | 1 | CB | SER | A | 25 | 50.525 | 13.786 | 60.068 | 1.00 | 58.97 | A | C |
| ATOM | 2 | OG | SER | A | 25 | 51.460 | 14.567 | 60.805 | 1.00 | 59.45 | A | O |
| ATOM | 3 | C | SER | A | 25 | 50.204 | 15.506 | 58.258 | 1.00 | 58.77 | A | C |
| ATOM | 4 | O | SER | A | 25 | 50.006 | 15.267 | 57.052 | 1.00 | 58.51 | A | O |
| ATOM | 5 | N | SER | A | 25 | 48.413 | 13.839 | 58.781 | 1.00 | 58.43 | A | N |
| ATOM | 6 | CA | SER | A | 25 | 49.505 | 14.680 | 59.350 | 1.00 | 58.91 | A | C |
| ATOM | 7 | N | MET | A | 26 | 51.021 | 16.472 | 58.679 | 1.00 | 58.75 | A | N |
| ATOM | 8 | CA | MET | A | 26 | 51.731 | 17.328 | 57.737 | 1.00 | 59.40 | A | C |
| ATOM | 9 | CB | MET | A | 26 | 51.255 | 18.777 | 57.876 | 1.00 | 60.00 | A | C |
| ATOM | 10 | CG | MET | A | 26 | 51.399 | 19.370 | 59.273 | 1.00 | 60.28 | A | C |
| ATOM | 11 | SD | MET | A | 26 | 50.974 | 21.150 | 59.298 | 1.00 | 62.28 | A | S |
| ATOM | 12 | CE | MET | A | 26 | 49.132 | 21.083 | 59.155 | 1.00 | 59.79 | A | C |
| ATOM | 13 | C | MET | A | 26 | 53.259 | 17.292 | 57.868 | 1.00 | 59.88 | A | C |
| ATOM | 14 | O | MET | A | 26 | 53.820 | 17.193 | 58.982 | 1.00 | 60.14 | A | O |
| ATOM | 15 | N | LYS | A | 27 | 53.920 | 17.376 | 56.716 | 1.00 | 60.01 | A | N |
| ATOM | 16 | CA | LYS | A | 27 | 55.378 | 17.377 | 56.622 | 1.00 | 59.77 | A | C |
| ATOM | 17 | CB | LYS | A | 27 | 55.794 | 17.002 | 55.194 | 1.00 | 60.08 | A | C |
| ATOM | 18 | CG | LYS | A | 27 | 54.712 | 16.211 | 54.420 | 1.00 | 60.25 | A | C |
| ATOM | 19 | CD | LYS | A | 27 | 55.021 | 14.718 | 54.320 | 1.00 | 60.62 | A | C |
| ATOM | 20 | CE | LYS | A | 27 | 55.273 | 14.086 | 55.695 | 1.00 | 60.17 | A | C |
| ATOM | 21 | NZ | LYS | A | 27 | 55.809 | 12.691 | 55.554 | 1.00 | 59.43 | A | N |
| ATOM | 22 | C | LYS | A | 27 | 55.756 | 18.826 | 56.923 | 1.00 | 59.87 | A | C |
| ATOM | 23 | O | LYS | A | 27 | 54.878 | 19.651 | 57.085 | 1.00 | 60.55 | A | O |
| ATOM | 24 | N | VAL | A | 28 | 57.045 | 19.145 | 56.985 | 1.00 | 59.86 | A | N |
| ATOM | 25 | CA | VAL | A | 28 | 57.454 | 20.517 | 57.291 | 1.00 | 59.22 | A | C |
| ATOM | 26 | CB | VAL | A | 28 | 56.981 | 20.916 | 58.714 | 1.00 | 59.14 | A | C |
| ATOM | 27 | CG1 | VAL | A | 28 | 57.268 | 19.766 | 59.689 | 1.00 | 59.26 | A | C |
| ATOM | 28 | CG2 | VAL | A | 28 | 57.686 | 22.193 | 59.178 | 1.00 | 58.44 | A | C |
| ATOM | 29 | C | VAL | A | 28 | 58.974 | 20.651 | 57.205 | 1.00 | 59.24 | A | C |
| ATOM | 30 | O | VAL | A | 28 | 59.698 | 20.003 | 57.954 | 1.00 | 58.99 | A | O |
| ATOM | 31 | N | GLY | A | 29 | 59.442 | 21.500 | 56.290 | 1.00 | 59.43 | A | N |
| ATOM | 32 | CA | GLY | A | 29 | 60.869 | 21.698 | 56.105 | 1.00 | 60.14 | A | C |
| ATOM | 33 | C | GLY | A | 29 | 61.172 | 23.136 | 55.725 | 1.00 | 61.64 | A | C |
| ATOM | 34 | O | GLY | A | 29 | 60.397 | 24.042 | 56.054 | 1.00 | 61.50 | A | O |
| ATOM | 35 | N | ARG | A | 30 | 62.289 | 23.360 | 55.030 | 1.00 | 62.90 | A | N |
| ATOM | 36 | CA | ARG | A | 30 | 62.672 | 24.714 | 54.625 | 1.00 | 64.10 | A | C |
| ATOM | 37 | CB | ARG | A | 30 | 63.600 | 25.345 | 55.682 | 1.00 | 63.51 | A | C |
| ATOM | 38 | CG | ARG | A | 30 | 62.947 | 25.534 | 57.051 | 1.00 | 64.09 | A | C |
| ATOM | 39 | CD | ARG | A | 30 | 63.822 | 26.343 | 58.021 | 1.00 | 64.87 | A | C |
| ATOM | 40 | NE | ARG | A | 30 | 63.126 | 26.652 | 59.277 | 1.00 | 64.27 | A | N |
| ATOM | 41 | CZ | ARG | A | 30 | 62.622 | 25.739 | 60.110 | 1.00 | 64.27 | A | C |
| ATOM | 42 | NH1 | ARG | A | 30 | 62.008 | 26.122 | 61.228 | 1.00 | 63.19 | A | N |
| ATOM | 43 | NH2 | ARG | A | 30 | 62.732 | 24.441 | 59.827 | 1.00 | 63.59 | A | N |
| ATOM | 44 | C | ARG | A | 30 | 63.361 | 24.726 | 53.258 | 1.00 | 65.03 | A | C |
| ATOM | 45 | O | ARG | A | 30 | 62.693 | 24.682 | 52.194 | 1.00 | 65.27 | A | O |
| ATOM | 46 | N | GLY | A | 31 | 64.692 | 24.785 | 53.277 | 1.00 | 66.07 | A | N |
| ATOM | 47 | CA | GLY | A | 31 | 65.444 | 24.803 | 52.032 | 1.00 | 66.84 | A | C |
| ATOM | 48 | C | GLY | A | 31 | 64.930 | 25.911 | 51.131 | 1.00 | 67.49 | A | C |
| ATOM | 49 | O | GLY | A | 31 | 64.182 | 26.803 | 51.591 | 1.00 | 67.60 | A | O |
| ATOM | 50 | N | GLY | A | 32 | 65.314 | 25.872 | 49.856 | 1.00 | 67.78 | A | N |
| ATOM | 51 | CA | GLY | A | 32 | 64.856 | 26.895 | 48.929 | 1.00 | 67.77 | A | C |
| ATOM | 52 | C | GLY | A | 32 | 65.294 | 28.302 | 49.318 | 1.00 | 67.71 | A | C |
| ATOM | 53 | O | GLY | A | 32 | 64.955 | 29.288 | 48.626 | 1.00 | 67.42 | A | O |
| ATOM | 54 | N | GLY | A | 33 | 66.037 | 28.413 | 50.419 | 1.00 | 67.26 | A | N |
| ATOM | 55 | CA | GLY | A | 33 | 66.515 | 29.718 | 50.845 | 1.00 | 66.49 | A | C |
| ATOM | 56 | C | GLY | A | 33 | 66.116 | 30.193 | 52.233 | 1.00 | 65.96 | A | C |
| ATOM | 57 | O | GLY | A | 33 | 65.822 | 31.381 | 52.413 | 1.00 | 66.43 | A | O |

| | | | | | | | | | | | | |
|---|---|---|---|---|---|---|---|---|---|---|---|---|
| ATOM | 58 | N | GLY | A | 34 | 66.090 | 29.287 | 53.209 | 1.00 | 64.96 | A | N |
| ATOM | 59 | CA | GLY | A | 34 | 65.747 | 29.679 | 54.570 | 1.00 | 63.30 | A | C |
| ATOM | 60 | C | GLY | A | 34 | 64.274 | 29.681 | 54.965 | 1.00 | 62.62 | A | C |
| ATOM | 61 | O | GLY | A | 34 | 63.935 | 29.321 | 56.113 | 1.00 | 62.57 | A | O |
| ATOM | 62 | N | GLY | A | 35 | 63.399 | 30.092 | 54.044 | 1.00 | 61.44 | A | N |
| ATOM | 63 | CA | GLY | A | 35 | 61.968 | 30.140 | 54.329 | 1.00 | 59.61 | A | C |
| ATOM | 64 | C | GLY | A | 35 | 61.351 | 28.775 | 54.616 | 1.00 | 58.45 | A | C |
| ATOM | 65 | O | GLY | A | 35 | 61.651 | 27.764 | 53.907 | 1.00 | 58.70 | A | O |
| ATOM | 66 | N | LYS | A | 36 | 60.485 | 28.740 | 55.634 | 1.00 | 56.00 | A | N |
| ATOM | 67 | CA | LYS | A | 36 | 59.804 | 27.523 | 56.097 | 1.00 | 53.37 | A | C |
| ATOM | 68 | CB | LYS | A | 36 | 59.270 | 27.736 | 57.513 | 1.00 | 52.99 | A | C |
| ATOM | 69 | CG | LYS | A | 36 | 58.499 | 26.548 | 58.055 | 1.00 | 53.34 | A | C |
| ATOM | 70 | CD | LYS | A | 36 | 57.511 | 26.978 | 59.141 | 1.00 | 53.43 | A | C |
| ATOM | 71 | CE | LYS | A | 36 | 56.682 | 25.789 | 59.617 | 1.00 | 53.63 | A | C |
| ATOM | 72 | NZ | LYS | A | 36 | 55.630 | 26.174 | 60.608 | 1.00 | 53.54 | A | N |
| ATOM | 73 | C | LYS | A | 36 | 58.648 | 27.050 | 55.217 | 1.00 | 52.22 | A | C |
| ATOM | 74 | O | LYS | A | 36 | 57.699 | 27.798 | 54.956 | 1.00 | 52.26 | A | O |
| ATOM | 75 | N | VAL | A | 37 | 58.709 | 25.785 | 54.809 | 1.00 | 49.85 | A | N |
| ATOM | 76 | CA | VAL | A | 37 | 57.687 | 25.207 | 53.944 | 1.00 | 47.09 | A | C |
| ATOM | 77 | CB | VAL | A | 37 | 58.334 | 24.565 | 52.709 | 1.00 | 47.71 | A | C |
| ATOM | 78 | CG1 | VAL | A | 37 | 57.271 | 23.916 | 51.830 | 1.00 | 47.93 | A | C |
| ATOM | 79 | CG2 | VAL | A | 37 | 59.124 | 25.607 | 51.947 | 1.00 | 47.47 | A | C |
| ATOM | 80 | C | VAL | A | 37 | 56.821 | 24.144 | 54.610 | 1.00 | 45.60 | A | C |
| ATOM | 81 | O | VAL | A | 37 | 57.328 | 23.255 | 55.305 | 1.00 | 44.56 | A | O |
| ATOM | 82 | N | THR | A | 38 | 55.512 | 24.234 | 54.386 | 1.00 | 43.86 | A | N |
| ATOM | 83 | CA | THR | A | 38 | 54.588 | 23.255 | 54.941 | 1.00 | 42.42 | A | C |
| ATOM | 84 | CB | THR | A | 38 | 53.471 | 23.922 | 55.760 | 1.00 | 42.44 | A | C |
| ATOM | 85 | OG1 | THR | A | 38 | 54.057 | 24.774 | 56.756 | 1.00 | 43.50 | A | O |
| ATOM | 86 | CG2 | THR | A | 38 | 52.620 | 22.861 | 56.449 | 1.00 | 39.73 | A | C |
| ATOM | 87 | C | THR | A | 38 | 53.971 | 22.429 | 53.816 | 1.00 | 41.94 | A | C |
| ATOM | 88 | O | THR | A | 38 | 53.277 | 22.954 | 52.933 | 1.00 | 41.54 | A | O |
| ATOM | 89 | N | THR | A | 39 | 54.248 | 21.132 | 53.849 | 1.00 | 41.21 | A | N |
| ATOM | 90 | CA | THR | A | 39 | 53.733 | 20.199 | 52.863 | 1.00 | 39.88 | A | C |
| ATOM | 91 | CB | THR | A | 39 | 54.879 | 19.348 | 52.228 | 1.00 | 40.37 | A | C |
| ATOM | 92 | OG1 | THR | A | 39 | 55.648 | 20.172 | 51.343 | 1.00 | 41.50 | A | O |
| ATOM | 93 | CG2 | THR | A | 39 | 54.315 | 18.177 | 51.435 | 1.00 | 40.37 | A | C |
| ATOM | 94 | C | THR | A | 39 | 52.718 | 19.288 | 53.533 | 1.00 | 38.84 | A | C |
| ATOM | 95 | O | THR | A | 39 | 53.009 | 18.666 | 54.556 | 1.00 | 38.74 | A | O |
| ATOM | 96 | N | VAL | A | 40 | 51.524 | 19.223 | 52.951 | 1.00 | 37.06 | A | N |
| ATOM | 97 | CA | VAL | A | 40 | 50.445 | 18.410 | 53.497 | 1.00 | 36.31 | A | C |
| ATOM | 98 | CB | VAL | A | 40 | 49.414 | 19.312 | 54.232 | 1.00 | 37.19 | A | C |
| ATOM | 99 | CG1 | VAL | A | 40 | 48.700 | 20.213 | 53.230 | 1.00 | 35.51 | A | C |
| ATOM | 100 | CG2 | VAL | A | 40 | 48.413 | 18.458 | 54.993 | 1.00 | 38.77 | A | C |
| ATOM | 101 | C | VAL | A | 40 | 49.729 | 17.635 | 52.378 | 1.00 | 35.66 | A | C |
| ATOM | 102 | O | VAL | A | 40 | 49.787 | 18.021 | 51.221 | 1.00 | 36.32 | A | O |
| ATOM | 103 | N | VAL | A | 41 | 49.080 | 16.527 | 52.718 | 1.00 | 34.41 | A | N |
| ATOM | 104 | CA | VAL | A | 41 | 48.343 | 15.772 | 51.712 | 1.00 | 32.76 | A | C |
| ATOM | 105 | CB | VAL | A | 41 | 48.433 | 14.243 | 51.921 | 1.00 | 31.73 | A | C |
| ATOM | 106 | CG1 | VAL | A | 41 | 47.594 | 13.541 | 50.865 | 1.00 | 31.12 | A | C |
| ATOM | 107 | CG2 | VAL | A | 41 | 49.881 | 13.772 | 51.836 | 1.00 | 30.03 | A | C |
| ATOM | 108 | C | VAL | A | 41 | 46.881 | 16.185 | 51.856 | 1.00 | 33.34 | A | C |
| ATOM | 109 | O | VAL | A | 41 | 46.244 | 15.907 | 52.881 | 1.00 | 31.86 | A | O |
| ATOM | 110 | N | ALA | A | 42 | 46.345 | 16.848 | 50.835 | 1.00 | 32.88 | A | N |
| ATOM | 111 | CA | ALA | A | 42 | 44.966 | 17.308 | 50.901 | 1.00 | 33.72 | A | C |
| ATOM | 112 | CB | ALA | A | 42 | 44.929 | 18.823 | 50.793 | 1.00 | 34.11 | A | C |
| ATOM | 113 | C | ALA | A | 42 | 44.009 | 16.694 | 49.875 | 1.00 | 33.79 | A | C |
| ATOM | 114 | O | ALA | A | 42 | 44.422 | 16.141 | 48.871 | 1.00 | 34.09 | A | O |
| ATOM | 115 | N | THR | A | 43 | 42.715 | 16.824 | 50.155 | 1.00 | 33.74 | A | N |
| ATOM | 116 | CA | THR | A | 43 | 41.657 | 16.326 | 49.285 | 1.00 | 33.43 | A | C |
| ATOM | 117 | CB | THR | A | 43 | 40.462 | 15.796 | 50.112 | 1.00 | 33.68 | A | C |
| ATOM | 118 | OG1 | THR | A | 43 | 40.915 | 14.820 | 51.060 | 1.00 | 32.70 | A | O |
| ATOM | 119 | CG2 | THR | A | 43 | 39.409 | 15.175 | 49.203 | 1.00 | 32.72 | A | C |
| ATOM | 120 | C | THR | A | 43 | 41.140 | 17.490 | 48.445 | 1.00 | 34.82 | A | C |

| | | | | | | | | | | | | |
|---|---|---|---|---|---|---|---|---|---|---|---|---|
| ATOM | 121 | O | THR | A | 43 | 40.944 | 18.574 | 48.958 | 1.00 | 35.11 | A | O |
| ATOM | 122 | N | PRO | A | 44 | 40.908 | 17.267 | 47.140 | 1.00 | 36.18 | A | N |
| ATOM | 123 | CD | PRO | A | 44 | 41.172 | 16.020 | 46.398 | 1.00 | 35.95 | A | C |
| ATOM | 124 | CA | PRO | A | 44 | 40.408 | 18.316 | 46.240 | 1.00 | 36.40 | A | C |
| ATOM | 125 | CB | PRO | A | 44 | 40.342 | 17.606 | 44.887 | 1.00 | 35.65 | A | C |
| ATOM | 126 | CG | PRO | A | 44 | 41.390 | 16.530 | 45.001 | 1.00 | 36.92 | A | C |
| ATOM | 127 | C | PRO | A | 44 | 39.019 | 18.779 | 46.689 | 1.00 | 37.09 | A | C |
| ATOM | 128 | O | PRO | A | 44 | 38.226 | 17.977 | 47.151 | 1.00 | 35.98 | A | O |
| ATOM | 129 | N | GLY | A | 45 | 38.732 | 20.071 | 46.547 | 1.00 | 38.88 | A | N |
| ATOM | 130 | CA | GLY | A | 45 | 37.425 | 20.575 | 46.944 | 1.00 | 41.67 | A | C |
| ATOM | 131 | C | GLY | A | 45 | 36.305 | 19.915 | 46.158 | 1.00 | 43.54 | A | C |
| ATOM | 132 | O | GLY | A | 45 | 35.312 | 19.504 | 46.723 | 1.00 | 44.32 | A | O |
| ATOM | 133 | N | ALA | A | 46 | 36.478 | 19.817 | 44.843 | 1.00 | 45.58 | A | N |
| ATOM | 134 | CA | ALA | A | 46 | 35.489 | 19.187 | 43.970 | 1.00 | 48.05 | A | C |
| ATOM | 135 | CB | ALA | A | 46 | 35.068 | 20.166 | 42.873 | 1.00 | 47.77 | A | C |
| ATOM | 136 | C | ALA | A | 46 | 36.103 | 17.924 | 43.343 | 1.00 | 49.63 | A | C |
| ATOM | 137 | O | ALA | A | 46 | 37.323 | 17.736 | 43.358 | 1.00 | 49.44 | A | O |
| ATOM | 138 | N | GLY | A | 47 | 35.262 | 17.067 | 42.778 | 1.00 | 51.39 | A | N |
| ATOM | 139 | CA | GLY | A | 47 | 35.774 | 15.849 | 42.173 | 1.00 | 53.09 | A | C |
| ATOM | 140 | C | GLY | A | 47 | 35.907 | 14.789 | 43.247 | 1.00 | 54.67 | A | C |
| ATOM | 141 | O | GLY | A | 47 | 35.760 | 15.100 | 44.428 | 1.00 | 55.27 | A | O |
| ATOM | 142 | N | PRO | A | 48 | 36.186 | 13.528 | 42.882 | 1.00 | 55.40 | A | N |
| ATOM | 143 | CD | PRO | A | 48 | 36.121 | 12.991 | 41.518 | 1.00 | 55.61 | A | C |
| ATOM | 144 | CA | PRO | A | 48 | 36.324 | 12.438 | 43.862 | 1.00 | 55.23 | A | C |
| ATOM | 145 | CB | PRO | A | 48 | 36.210 | 11.176 | 43.003 | 1.00 | 55.88 | A | C |
| ATOM | 146 | CG | PRO | A | 48 | 35.446 | 11.665 | 41.771 | 1.00 | 56.89 | A | C |
| ATOM | 147 | C | PRO | A | 48 | 37.639 | 12.474 | 44.648 | 1.00 | 54.49 | A | C |
| ATOM | 148 | O | PRO | A | 48 | 38.691 | 12.927 | 44.140 | 1.00 | 53.39 | A | O |
| ATOM | 149 | N | ASP | A | 49 | 37.571 | 11.980 | 45.884 | 1.00 | 53.85 | A | N |
| ATOM | 150 | CA | ASP | A | 49 | 38.724 | 11.956 | 46.766 | 1.00 | 52.11 | A | C |
| ATOM | 151 | CB | ASP | A | 49 | 38.436 | 11.145 | 48.027 | 1.00 | 53.04 | A | C |
| ATOM | 152 | CG | ASP | A | 49 | 39.656 | 11.024 | 48.922 | 1.00 | 54.33 | A | C |
| ATOM | 153 | OD1 | ASP | A | 49 | 40.331 | 12.059 | 49.148 | 1.00 | 53.63 | A | O |
| ATOM | 154 | OD2 | ASP | A | 49 | 39.935 | 9.900 | 49.402 | 1.00 | 55.67 | A | O |
| ATOM | 155 | C | ASP | A | 49 | 39.951 | 11.369 | 46.077 | 1.00 | 50.64 | A | C |
| ATOM | 156 | O | ASP | A | 49 | 40.047 | 10.154 | 45.854 | 1.00 | 51.29 | A | O |
| ATOM | 157 | N | ARG | A | 50 | 40.878 | 12.255 | 45.732 | 1.00 | 48.25 | A | N |
| ATOM | 158 | CA | ARG | A | 50 | 42.145 | 11.899 | 45.105 | 1.00 | 45.92 | A | C |
| ATOM | 159 | CB | ARG | A | 50 | 42.100 | 12.149 | 43.600 | 1.00 | 46.66 | A | C |
| ATOM | 160 | CG | ARG | A | 50 | 43.426 | 11.900 | 42.925 | 1.00 | 47.80 | A | C |
| ATOM | 161 | CD | ARG | A | 50 | 43.235 | 11.618 | 41.444 | 1.00 | 50.23 | A | C |
| ATOM | 162 | NE | ARG | A | 50 | 43.166 | 12.822 | 40.624 | 1.00 | 50.76 | A | N |
| ATOM | 163 | CZ | ARG | A | 50 | 42.453 | 12.908 | 39.502 | 1.00 | 51.67 | A | C |
| ATOM | 164 | NH1 | ARG | A | 50 | 41.749 | 11.854 | 39.087 | 1.00 | 50.57 | A | N |
| ATOM | 165 | NH2 | ARG | A | 50 | 42.456 | 14.032 | 38.793 | 1.00 | 51.40 | A | N |
| ATOM | 166 | C | ARG | A | 50 | 43.201 | 12.807 | 45.743 | 1.00 | 43.59 | A | C |
| ATOM | 167 | O | ARG | A | 50 | 43.532 | 13.867 | 45.214 | 1.00 | 42.53 | A | O |
| ATOM | 168 | N | PRO | A | 51 | 43.772 | 12.371 | 46.872 | 1.00 | 42.25 | A | N |
| ATOM | 169 | CD | PRO | A | 51 | 43.870 | 10.949 | 47.240 | 1.00 | 41.38 | A | C |
| ATOM | 170 | CA | PRO | A | 51 | 44.776 | 13.147 | 47.604 | 1.00 | 40.83 | A | C |
| ATOM | 171 | CB | PRO | A | 51 | 45.353 | 12.136 | 48.595 | 1.00 | 40.67 | A | C |
| ATOM | 172 | CG | PRO | A | 51 | 44.339 | 11.039 | 48.659 | 1.00 | 41.56 | A | C |
| ATOM | 173 | C | PRO | A | 51 | 45.879 | 13.690 | 46.721 | 1.00 | 40.24 | A | C |
| ATOM | 174 | O | PRO | A | 51 | 46.152 | 13.169 | 45.655 | 1.00 | 40.61 | A | O |
| ATOM | 175 | N | GLN | A | 52 | 46.538 | 14.732 | 47.199 | 1.00 | 40.74 | A | N |
| ATOM | 176 | CA | GLN | A | 52 | 47.642 | 15.320 | 46.464 | 1.00 | 41.06 | A | C |
| ATOM | 177 | CB | GLN | A | 52 | 47.116 | 16.117 | 45.277 | 1.00 | 43.82 | A | C |
| ATOM | 178 | CG | GLN | A | 52 | 45.734 | 16.675 | 45.493 | 1.00 | 46.93 | A | C |
| ATOM | 179 | CD | GLN | A | 52 | 44.914 | 16.654 | 44.212 | 1.00 | 49.85 | A | C |
| ATOM | 180 | OE1 | GLN | A | 52 | 44.915 | 15.648 | 43.472 | 1.00 | 49.51 | A | O |
| ATOM | 181 | NE2 | GLN | A | 52 | 44.196 | 17.752 | 43.941 | 1.00 | 51.56 | A | N |
| ATOM | 182 | C | GLN | A | 52 | 48.441 | 16.227 | 47.366 | 1.00 | 39.71 | A | C |
| ATOM | 183 | O | GLN | A | 52 | 47.884 | 16.988 | 48.129 | 1.00 | 40.61 | A | O |

| | | | | | | | | | | | | |
|---|---|---|---|---|---|---|---|---|---|---|---|---|
| ATOM | 184 | N | GLU | A | 53 | 49.759 | 16.127 | 47.267 | 1.00 | 38.79 | A | N |
| ATOM | 185 | CA | GLU | A | 53 | 50.632 | 16.933 | 48.096 | 1.00 | 38.52 | A | C |
| ATOM | 186 | CB | GLU | A | 53 | 52.083 | 16.518 | 47.928 | 1.00 | 39.77 | A | C |
| ATOM | 187 | CG | GLU | A | 53 | 52.396 | 15.193 | 48.540 | 1.00 | 41.60 | A | C |
| ATOM | 188 | CD | GLU | A | 53 | 53.876 | 14.942 | 48.579 | 1.00 | 41.96 | A | C |
| ATOM | 189 | OE1 | GLU | A | 53 | 54.272 | 13.864 | 49.064 | 1.00 | 43.69 | A | O |
| ATOM | 190 | OE2 | GLU | A | 53 | 54.637 | 15.828 | 48.129 | 1.00 | 41.87 | A | O |
| ATOM | 191 | C | GLU | A | 53 | 50.528 | 18.401 | 47.757 | 1.00 | 37.62 | A | C |
| ATOM | 192 | O | GLU | A | 53 | 50.575 | 18.784 | 46.602 | 1.00 | 37.31 | A | O |
| ATOM | 193 | N | VAL | A | 54 | 50.393 | 19.221 | 48.787 | 1.00 | 36.29 | A | N |
| ATOM | 194 | CA | VAL | A | 54 | 50.314 | 20.651 | 48.589 | 1.00 | 35.98 | A | C |
| ATOM | 195 | CB | VAL | A | 54 | 48.902 | 21.201 | 48.952 | 1.00 | 36.75 | A | C |
| ATOM | 196 | CG1 | VAL | A | 54 | 48.832 | 22.694 | 48.672 | 1.00 | 35.95 | A | C |
| ATOM | 197 | CG2 | VAL | A | 54 | 47.836 | 20.478 | 48.149 | 1.00 | 36.23 | A | C |
| ATOM | 198 | C | VAL | A | 54 | 51.355 | 21.285 | 49.503 | 1.00 | 34.98 | A | C |
| ATOM | 199 | O | VAL | A | 54 | 51.435 | 20.950 | 50.680 | 1.00 | 34.94 | A | O |
| ATOM | 200 | N | SER | A | 55 | 52.158 | 22.181 | 48.941 | 1.00 | 33.94 | A | N |
| ATOM | 201 | CA | SER | A | 55 | 53.212 | 22.863 | 49.686 | 1.00 | 34.42 | A | C |
| ATOM | 202 | CB | SER | A | 55 | 54.588 | 22.564 | 49.075 | 1.00 | 35.24 | A | C |
| ATOM | 203 | OG | SER | A | 55 | 54.867 | 21.175 | 49.087 | 1.00 | 37.72 | A | O |
| ATOM | 204 | C | SER | A | 55 | 52.984 | 24.365 | 49.642 | 1.00 | 33.26 | A | C |
| ATOM | 205 | O | SER | A | 55 | 52.781 | 24.937 | 48.575 | 1.00 | 31.61 | A | O |
| ATOM | 206 | N | TYR | A | 56 | 53.023 | 25.000 | 50.806 | 1.00 | 34.02 | A | N |
| ATOM | 207 | CA | TYR | A | 56 | 52.826 | 26.441 | 50.877 | 1.00 | 35.32 | A | C |
| ATOM | 208 | CB | TYR | A | 56 | 51.377 | 26.765 | 51.236 | 1.00 | 35.51 | A | C |
| ATOM | 209 | CG | TYR | A | 56 | 50.881 | 26.140 | 52.525 | 1.00 | 35.43 | A | C |
| ATOM | 210 | CD1 | TYR | A | 56 | 51.184 | 26.699 | 53.762 | 1.00 | 35.08 | A | C |
| ATOM | 211 | CE1 | TYR | A | 56 | 50.687 | 26.144 | 54.946 | 1.00 | 35.64 | A | C |
| ATOM | 212 | CD2 | TYR | A | 56 | 50.075 | 25.002 | 52.495 | 1.00 | 35.75 | A | C |
| ATOM | 213 | CE2 | TYR | A | 56 | 49.575 | 24.438 | 53.665 | 1.00 | 35.84 | A | C |
| ATOM | 214 | CZ | TYR | A | 56 | 49.881 | 25.013 | 54.885 | 1.00 | 36.18 | A | C |
| ATOM | 215 | OH | TYR | A | 56 | 49.366 | 24.459 | 56.031 | 1.00 | 37.16 | A | O |
| ATOM | 216 | C | TYR | A | 56 | 53.770 | 27.081 | 51.885 | 1.00 | 35.63 | A | C |
| ATOM | 217 | O | TYR | A | 56 | 54.270 | 26.422 | 52.773 | 1.00 | 35.68 | A | O |
| ATOM | 218 | N | THR | A | 57 | 54.008 | 28.377 | 51.718 | 1.00 | 37.70 | A | N |
| ATOM | 219 | CA | THR | A | 57 | 54.878 | 29.124 | 52.614 | 1.00 | 40.01 | A | C |
| ATOM | 220 | CB | THR | A | 57 | 56.275 | 29.367 | 52.002 | 1.00 | 41.03 | A | C |
| ATOM | 221 | OG1 | THR | A | 57 | 56.736 | 28.180 | 51.348 | 1.00 | 45.34 | A | O |
| ATOM | 222 | CG2 | THR | A | 57 | 57.267 | 29.737 | 53.093 | 1.00 | 42.02 | A | C |
| ATOM | 223 | C | THR | A | 57 | 54.283 | 30.499 | 52.885 | 1.00 | 40.37 | A | C |
| ATOM | 224 | O | THR | A | 57 | 53.144 | 30.771 | 52.533 | 1.00 | 40.19 | A | O |
| ATOM | 225 | N | ASP | A | 58 | 55.092 | 31.360 | 53.498 | 1.00 | 41.66 | A | N |
| ATOM | 226 | CA | ASP | A | 58 | 54.709 | 32.726 | 53.825 | 1.00 | 42.65 | A | C |
| ATOM | 227 | CB | ASP | A | 58 | 54.832 | 33.619 | 52.588 | 1.00 | 44.47 | A | C |
| ATOM | 228 | CG | ASP | A | 58 | 56.247 | 33.667 | 52.049 | 1.00 | 47.19 | A | C |
| ATOM | 229 | OD1 | ASP | A | 58 | 57.162 | 34.057 | 52.822 | 1.00 | 48.17 | A | O |
| ATOM | 230 | OD2 | ASP | A | 58 | 56.437 | 33.317 | 50.855 | 1.00 | 46.47 | A | O |
| ATOM | 231 | C | ASP | A | 58 | 53.306 | 32.854 | 54.394 | 1.00 | 42.35 | A | C |
| ATOM | 232 | O | ASP | A | 58 | 52.510 | 33.639 | 53.895 | 1.00 | 42.18 | A | O |
| ATOM | 233 | N | THR | A | 59 | 52.998 | 32.093 | 55.437 | 1.00 | 41.67 | A | N |
| ATOM | 234 | CA | THR | A | 59 | 51.669 | 32.195 | 56.010 | 1.00 | 41.28 | A | C |
| ATOM | 235 | CB | THR | A | 59 | 51.234 | 30.901 | 56.732 | 1.00 | 41.78 | A | C |
| ATOM | 236 | OG1 | THR | A | 59 | 52.104 | 30.652 | 57.841 | 1.00 | 43.07 | A | O |
| ATOM | 237 | CG2 | THR | A | 59 | 51.265 | 29.719 | 55.772 | 1.00 | 39.88 | A | C |
| ATOM | 238 | C | THR | A | 59 | 51.580 | 33.348 | 56.992 | 1.00 | 41.08 | A | C |
| ATOM | 239 | O | THR | A | 59 | 52.515 | 33.627 | 57.726 | 1.00 | 41.18 | A | O |
| ATOM | 240 | N | LYS | A | 60 | 50.446 | 34.030 | 56.979 | 1.00 | 40.59 | A | N |
| ATOM | 241 | CA | LYS | A | 60 | 50.220 | 35.146 | 57.886 | 1.00 | 40.60 | A | C |
| ATOM | 242 | CB | LYS | A | 60 | 50.673 | 36.464 | 57.255 | 1.00 | 40.46 | A | C |
| ATOM | 243 | CG | LYS | A | 60 | 50.004 | 36.790 | 55.937 | 1.00 | 42.32 | A | C |
| ATOM | 244 | CD | LYS | A | 60 | 50.484 | 38.126 | 55.387 | 1.00 | 44.33 | A | C |
| ATOM | 245 | CE | LYS | A | 60 | 52.016 | 38.167 | 55.222 | 1.00 | 46.73 | A | C |
| ATOM | 246 | NZ | LYS | A | 60 | 52.555 | 37.173 | 54.225 | 1.00 | 47.60 | A | N |

| | | | | | | | | | | | | |
|---|---|---|---|---|---|---|---|---|---|---|---|---|
| ATOM | 247 | C | LYS | A | 60 | 48.728 | 35.201 | 58.192 | 1.00 | 39.63 | A | C |
| ATOM | 248 | O | LYS | A | 60 | 47.905 | 34.743 | 57.390 | 1.00 | 39.27 | A | O |
| ATOM | 249 | N | VAL | A | 61 | 48.379 | 35.749 | 59.350 | 1.00 | 38.44 | A | N |
| ATOM | 250 | CA | VAL | A | 61 | 46.979 | 35.852 | 59.722 | 1.00 | 37.54 | A | C |
| ATOM | 251 | CB | VAL | A | 61 | 46.806 | 36.101 | 61.233 | 1.00 | 36.66 | A | C |
| ATOM | 252 | CG1 | VAL | A | 61 | 45.331 | 36.201 | 61.573 | 1.00 | 33.77 | A | C |
| ATOM | 253 | CG2 | VAL | A | 61 | 47.463 | 34.970 | 62.025 | 1.00 | 36.27 | A | C |
| ATOM | 254 | C | VAL | A | 61 | 46.339 | 37.011 | 58.968 | 1.00 | 37.39 | A | C |
| ATOM | 255 | O | VAL | A | 61 | 46.963 | 38.050 | 58.756 | 1.00 | 36.45 | A | O |
| ATOM | 256 | N | ILE | A | 62 | 45.091 | 36.823 | 58.555 | 1.00 | 37.26 | A | N |
| ATOM | 257 | CA | ILE | A | 62 | 44.374 | 37.864 | 57.834 | 1.00 | 36.99 | A | C |
| ATOM | 258 | CB | ILE | A | 62 | 44.300 | 37.561 | 56.306 | 1.00 | 35.84 | A | C |
| ATOM | 259 | CG2 | ILE | A | 62 | 45.698 | 37.444 | 55.736 | 1.00 | 35.15 | A | C |
| ATOM | 260 | CG1 | ILE | A | 62 | 43.528 | 36.265 | 56.043 | 1.00 | 35.77 | A | C |
| ATOM | 261 | CD1 | ILE | A | 62 | 43.376 | 35.927 | 54.553 | 1.00 | 32.53 | A | C |
| ATOM | 262 | C | ILE | A | 62 | 42.959 | 38.014 | 58.378 | 1.00 | 37.86 | A | C |
| ATOM | 263 | O | ILE | A | 62 | 42.255 | 38.989 | 58.064 | 1.00 | 38.57 | A | O |
| ATOM | 264 | N | GLY | A | 63 | 42.538 | 37.050 | 59.191 | 1.00 | 37.18 | A | N |
| ATOM | 265 | CA | GLY | A | 63 | 41.205 | 37.119 | 59.753 | 1.00 | 37.58 | A | C |
| ATOM | 266 | C | GLY | A | 63 | 40.997 | 36.212 | 60.949 | 1.00 | 37.75 | A | C |
| ATOM | 267 | O | GLY | A | 63 | 41.750 | 35.281 | 61.171 | 1.00 | 38.45 | A | O |
| ATOM | 268 | N | ASN | A | 64 | 39.950 | 36.496 | 61.711 | 1.00 | 37.92 | A | N |
| ATOM | 269 | CA | ASN | A | 64 | 39.622 | 35.715 | 62.892 | 1.00 | 37.46 | A | C |
| ATOM | 270 | CB | ASN | A | 64 | 39.903 | 36.538 | 64.146 | 1.00 | 41.19 | A | C |
| ATOM | 271 | CG | ASN | A | 64 | 40.295 | 35.681 | 65.327 | 1.00 | 44.57 | A | C |
| ATOM | 272 | OD1 | ASN | A | 64 | 41.502 | 35.329 | 65.502 | 1.00 | 47.77 | A | O |
| ATOM | 273 | ND2 | ASN | A | 64 | 39.304 | 35.312 | 66.148 | 1.00 | 45.47 | A | N |
| ATOM | 274 | C | ASN | A | 64 | 38.131 | 35.407 | 62.833 | 1.00 | 35.65 | A | C |
| ATOM | 275 | O | ASN | A | 64 | 37.350 | 36.157 | 62.218 | 1.00 | 34.96 | A | O |
| ATOM | 276 | N | GLY | A | 65 | 37.733 | 34.322 | 63.486 | 1.00 | 33.01 | A | N |
| ATOM | 277 | CA | GLY | A | 65 | 36.334 | 33.940 | 63.503 | 1.00 | 31.40 | A | C |
| ATOM | 278 | C | GLY | A | 65 | 36.053 | 32.978 | 64.636 | 1.00 | 30.43 | A | C |
| ATOM | 279 | O | GLY | A | 65 | 36.962 | 32.608 | 65.356 | 1.00 | 29.27 | A | O |
| ATOM | 280 | N | SER | A | 66 | 34.799 | 32.566 | 64.785 | 1.00 | 29.07 | A | N |
| ATOM | 281 | CA | SER | A | 66 | 34.435 | 31.660 | 65.860 | 1.00 | 30.76 | A | C |
| ATOM | 282 | CB | SER | A | 66 | 32.911 | 31.576 | 65.994 | 1.00 | 30.92 | A | C |
| ATOM | 283 | OG | SER | A | 66 | 32.380 | 32.801 | 66.480 | 1.00 | 32.86 | A | O |
| ATOM | 284 | C | SER | A | 66 | 35.032 | 30.266 | 65.741 | 1.00 | 30.82 | A | C |
| ATOM | 285 | O | SER | A | 66 | 34.772 | 29.527 | 64.775 | 1.00 | 29.48 | A | O |
| ATOM | 286 | N | PHE | A | 67 | 35.843 | 29.923 | 66.741 | 1.00 | 30.86 | A | N |
| ATOM | 287 | CA | PHE | A | 67 | 36.495 | 28.623 | 66.813 | 1.00 | 31.30 | A | C |
| ATOM | 288 | CB | PHE | A | 67 | 35.453 | 27.509 | 66.688 | 1.00 | 30.03 | A | C |
| ATOM | 289 | CG | PHE | A | 67 | 34.498 | 27.424 | 67.856 | 1.00 | 29.46 | A | C |
| ATOM | 290 | CD1 | PHE | A | 67 | 33.122 | 27.370 | 67.641 | 1.00 | 28.80 | A | C |
| ATOM | 291 | CD2 | PHE | A | 67 | 34.976 | 27.347 | 69.159 | 1.00 | 28.83 | A | C |
| ATOM | 292 | CE1 | PHE | A | 67 | 32.239 | 27.234 | 68.706 | 1.00 | 28.73 | A | C |
| ATOM | 293 | CE2 | PHE | A | 67 | 34.105 | 27.211 | 70.230 | 1.00 | 29.22 | A | C |
| ATOM | 294 | CZ | PHE | A | 67 | 32.734 | 27.154 | 70.006 | 1.00 | 29.15 | A | C |
| ATOM | 295 | C | PHE | A | 67 | 37.572 | 28.425 | 65.746 | 1.00 | 32.43 | A | C |
| ATOM | 296 | O | PHE | A | 67 | 37.941 | 27.278 | 65.447 | 1.00 | 32.77 | A | O |
| ATOM | 297 | N | GLY | A | 68 | 38.081 | 29.519 | 65.174 | 1.00 | 30.86 | A | N |
| ATOM | 298 | CA | GLY | A | 68 | 39.105 | 29.383 | 64.147 | 1.00 | 30.15 | A | C |
| ATOM | 299 | C | GLY | A | 68 | 39.805 | 30.644 | 63.666 | 1.00 | 29.81 | A | C |
| ATOM | 300 | O | GLY | A | 68 | 39.566 | 31.716 | 64.175 | 1.00 | 28.81 | A | O |
| ATOM | 301 | N | VAL | A | 69 | 40.672 | 30.487 | 62.663 | 1.00 | 29.23 | A | N |
| ATOM | 302 | CA | VAL | A | 69 | 41.436 | 31.596 | 62.089 | 1.00 | 28.94 | A | C |
| ATOM | 303 | CB | VAL | A | 69 | 42.844 | 31.699 | 62.722 | 1.00 | 29.44 | A | C |
| ATOM | 304 | CG1 | VAL | A | 69 | 43.446 | 33.059 | 62.411 | 1.00 | 28.13 | A | C |
| ATOM | 305 | CG2 | VAL | A | 69 | 42.775 | 31.458 | 64.222 | 1.00 | 29.37 | A | C |
| ATOM | 306 | C | VAL | A | 69 | 41.633 | 31.442 | 60.571 | 1.00 | 29.53 | A | C |
| ATOM | 307 | O | VAL | A | 69 | 41.593 | 30.346 | 60.037 | 1.00 | 28.84 | A | O |
| ATOM | 308 | N | VAL | A | 70 | 41.868 | 32.560 | 59.893 | 1.00 | 29.93 | A | N |
| ATOM | 309 | CA | VAL | A | 70 | 42.072 | 32.555 | 58.452 | 1.00 | 30.32 | A | C |

| | | | | | | | | | | | | |
|---|---|---|---|---|---|---|---|---|---|---|---|---|
| ATOM | 310 | CB | VAL | A | 70 | 41.072 | 33.483 | 57.743 | 1.00 | 30.50 | A | C |
| ATOM | 311 | CG1 | VAL | A | 70 | 41.188 | 33.317 | 56.238 | 1.00 | 28.93 | A | C |
| ATOM | 312 | CG2 | VAL | A | 70 | 39.667 | 33.163 | 58.202 | 1.00 | 31.53 | A | C |
| ATOM | 313 | C | VAL | A | 70 | 43.481 | 33.010 | 58.099 | 1.00 | 30.93 | A | C |
| ATOM | 314 | O | VAL | A | 70 | 43.916 | 34.105 | 58.470 | 1.00 | 30.84 | A | O |
| ATOM | 315 | N | TYR | A | 71 | 44.196 | 32.162 | 57.376 | 1.00 | 30.68 | A | N |
| ATOM | 316 | CA | TYR | A | 71 | 45.550 | 32.488 | 56.984 | 1.00 | 30.62 | A | C |
| ATOM | 317 | CB | TYR | A | 71 | 46.506 | 31.338 | 57.299 | 1.00 | 30.44 | A | C |
| ATOM | 318 | CG | TYR | A | 71 | 46.585 | 30.966 | 58.756 | 1.00 | 32.09 | A | C |
| ATOM | 319 | CD1 | TYR | A | 71 | 45.610 | 30.171 | 59.344 | 1.00 | 31.19 | A | C |
| ATOM | 320 | CE1 | TYR | A | 71 | 45.690 | 29.818 | 60.685 | 1.00 | 34.60 | A | C |
| ATOM | 321 | CD2 | TYR | A | 71 | 47.645 | 31.406 | 59.546 | 1.00 | 32.90 | A | C |
| ATOM | 322 | CE2 | TYR | A | 71 | 47.737 | 31.061 | 60.885 | 1.00 | 33.65 | A | C |
| ATOM | 323 | CZ | TYR | A | 71 | 46.759 | 30.266 | 61.452 | 1.00 | 35.33 | A | C |
| ATOM | 324 | OH | TYR | A | 71 | 46.854 | 29.903 | 62.779 | 1.00 | 37.58 | A | O |
| ATOM | 325 | C | TYR | A | 71 | 45.663 | 32.805 | 55.513 | 1.00 | 31.25 | A | C |
| ATOM | 326 | O | TYR | A | 71 | 44.785 | 32.501 | 54.714 | 1.00 | 30.16 | A | O |
| ATOM | 327 | N | GLN | A | 72 | 46.776 | 33.439 | 55.185 | 1.00 | 32.97 | A | N |
| ATOM | 328 | CA | GLN | A | 72 | 47.110 | 33.803 | 53.828 | 1.00 | 34.46 | A | C |
| ATOM | 329 | CB | GLN | A | 72 | 47.472 | 35.284 | 53.763 | 1.00 | 36.27 | A | C |
| ATOM | 330 | CG | GLN | A | 72 | 47.314 | 35.911 | 52.389 | 1.00 | 40.42 | A | C |
| ATOM | 331 | CD | GLN | A | 72 | 48.639 | 36.172 | 51.692 | 1.00 | 41.41 | A | C |
| ATOM | 332 | OE1 | GLN | A | 72 | 49.461 | 36.988 | 52.143 | 1.00 | 39.69 | A | O |
| ATOM | 333 | NE2 | GLN | A | 72 | 48.855 | 35.479 | 50.584 | 1.00 | 44.38 | A | N |
| ATOM | 334 | C | GLN | A | 72 | 48.348 | 32.946 | 53.614 | 1.00 | 34.55 | A | C |
| ATOM | 335 | O | GLN | A | 72 | 49.186 | 32.867 | 54.492 | 1.00 | 34.46 | A | O |
| ATOM | 336 | N | ALA | A | 73 | 48.440 | 32.272 | 52.477 | 1.00 | 34.63 | A | N |
| ATOM | 337 | CA | ALA | A | 73 | 49.600 | 31.431 | 52.200 | 1.00 | 34.72 | A | C |
| ATOM | 338 | CB | ALA | A | 73 | 49.318 | 29.990 | 52.593 | 1.00 | 33.70 | A | C |
| ATOM | 339 | C | ALA | A | 73 | 49.935 | 31.512 | 50.721 | 1.00 | 35.24 | A | C |
| ATOM | 340 | O | ALA | A | 73 | 49.104 | 31.916 | 49.910 | 1.00 | 35.30 | A | O |
| ATOM | 341 | N | LYS | A | 74 | 51.162 | 31.134 | 50.384 | 1.00 | 35.39 | A | N |
| ATOM | 342 | CA | LYS | A | 74 | 51.626 | 31.158 | 49.004 | 1.00 | 36.39 | A | C |
| ATOM | 343 | CB | LYS | A | 74 | 52.808 | 32.129 | 48.881 | 1.00 | 36.75 | A | C |
| ATOM | 344 | CG | LYS | A | 74 | 53.562 | 32.103 | 47.548 | 1.00 | 39.09 | A | C |
| ATOM | 345 | CD | LYS | A | 74 | 54.634 | 33.195 | 47.538 | 1.00 | 40.79 | A | C |
| ATOM | 346 | CE | LYS | A | 74 | 55.639 | 33.035 | 46.399 | 1.00 | 44.47 | A | C |
| ATOM | 347 | NZ | LYS | A | 74 | 56.606 | 31.896 | 46.584 | 1.00 | 46.12 | A | N |
| ATOM | 348 | C | LYS | A | 74 | 52.040 | 29.746 | 48.601 | 1.00 | 36.44 | A | C |
| ATOM | 349 | O | LYS | A | 74 | 52.890 | 29.130 | 49.251 | 1.00 | 36.48 | A | O |
| ATOM | 350 | N | LEU | A | 75 | 51.422 | 29.228 | 47.543 | 1.00 | 36.69 | A | N |
| ATOM | 351 | CA | LEU | A | 75 | 51.751 | 27.892 | 47.066 | 1.00 | 36.99 | A | C |
| ATOM | 352 | CB | LEU | A | 75 | 50.795 | 27.466 | 45.943 | 1.00 | 35.64 | A | C |
| ATOM | 353 | CG | LEU | A | 75 | 49.304 | 27.417 | 46.298 | 1.00 | 36.12 | A | C |
| ATOM | 354 | CD1 | LEU | A | 75 | 48.477 | 27.005 | 45.082 | 1.00 | 35.46 | A | C |
| ATOM | 355 | CD2 | LEU | A | 75 | 49.091 | 26.443 | 47.445 | 1.00 | 35.86 | A | C |
| ATOM | 356 | C | LEU | A | 75 | 53.186 | 27.948 | 46.553 | 1.00 | 37.23 | A | C |
| ATOM | 357 | O | LEU | A | 75 | 53.530 | 28.796 | 45.738 | 1.00 | 36.86 | A | O |
| ATOM | 358 | N | CYS | A | 76 | 54.014 | 27.038 | 47.051 | 1.00 | 38.34 | A | N |
| ATOM | 359 | CA | CYS | A | 76 | 55.416 | 26.975 | 46.684 | 1.00 | 40.14 | A | C |
| ATOM | 360 | CB | CYS | A | 76 | 56.035 | 25.714 | 47.259 | 1.00 | 39.64 | A | C |
| ATOM | 361 | SG | CYS | A | 76 | 56.171 | 25.851 | 49.024 | 1.00 | 42.10 | A | S |
| ATOM | 362 | C | CYS | A | 76 | 55.762 | 27.063 | 45.216 | 1.00 | 42.19 | A | C |
| ATOM | 363 | O | CYS | A | 76 | 56.546 | 27.916 | 44.819 | 1.00 | 43.39 | A | O |
| ATOM | 364 | N | ASP | A | 77 | 55.178 | 26.190 | 44.407 | 1.00 | 43.44 | A | N |
| ATOM | 365 | CA | ASP | A | 77 | 55.504 | 26.180 | 42.992 | 1.00 | 45.48 | A | C |
| ATOM | 366 | CB | ASP | A | 77 | 55.147 | 24.814 | 42.408 | 1.00 | 46.72 | A | C |
| ATOM | 367 | CG | ASP | A | 77 | 55.702 | 23.670 | 43.246 | 1.00 | 48.58 | A | C |
| ATOM | 368 | OD1 | ASP | A | 77 | 56.919 | 23.703 | 43.569 | 1.00 | 48.52 | A | O |
| ATOM | 369 | OD2 | ASP | A | 77 | 54.924 | 22.741 | 43.580 | 1.00 | 49.89 | A | O |
| ATOM | 370 | C | ASP | A | 77 | 54.901 | 27.305 | 42.150 | 1.00 | 46.46 | A | C |
| ATOM | 371 | O | ASP | A | 77 | 55.639 | 28.062 | 41.536 | 1.00 | 47.55 | A | O |
| ATOM | 372 | N | SER | A | 78 | 53.575 | 27.424 | 42.114 | 1.00 | 46.40 | A | N |

| | | | | | | | | | | | | |
|---|---|---|---|---|---|---|---|---|---|---|---|---|
| ATOM | 373 | CA | SER | A | 78 | 52.955 | 28.475 | 41.309 | 1.00 | 46.21 | A | C |
| ATOM | 374 | CB | SER | A | 78 | 51.474 | 28.168 | 41.061 | 1.00 | 47.11 | A | C |
| ATOM | 375 | OG | SER | A | 78 | 50.746 | 28.119 | 42.287 | 1.00 | 48.83 | A | O |
| ATOM | 376 | C | SER | A | 78 | 53.075 | 29.841 | 41.966 | 1.00 | 45.81 | A | C |
| ATOM | 377 | O | SER | A | 78 | 52.998 | 30.869 | 41.296 | 1.00 | 45.84 | A | O |
| ATOM | 378 | N | GLY | A | 79 | 53.267 | 29.852 | 43.280 | 1.00 | 45.35 | A | N |
| ATOM | 379 | CA | GLY | A | 79 | 53.376 | 31.116 | 43.982 | 1.00 | 44.40 | A | C |
| ATOM | 380 | C | GLY | A | 79 | 51.998 | 31.731 | 44.145 | 1.00 | 43.72 | A | C |
| ATOM | 381 | O | GLY | A | 79 | 51.862 | 32.854 | 44.621 | 1.00 | 44.83 | A | O |
| ATOM | 382 | N | GLU | A | 80 | 50.969 | 30.998 | 43.735 | 1.00 | 42.03 | A | N |
| ATOM | 383 | CA | GLU | A | 80 | 49.608 | 31.495 | 43.861 | 1.00 | 40.41 | A | C |
| ATOM | 384 | CB | GLU | A | 80 | 48.611 | 30.475 | 43.324 | 1.00 | 41.47 | A | C |
| ATOM | 385 | CG | GLU | A | 80 | 47.726 | 31.019 | 42.238 | 1.00 | 43.88 | A | C |
| ATOM | 386 | CD | GLU | A | 80 | 46.649 | 30.039 | 41.822 | 1.00 | 46.40 | A | C |
| ATOM | 387 | OE1 | GLU | A | 80 | 47.001 | 28.941 | 41.322 | 1.00 | 48.05 | A | O |
| ATOM | 388 | OE2 | GLU | A | 80 | 45.451 | 30.372 | 41.995 | 1.00 | 46.84 | A | O |
| ATOM | 389 | C | GLU | A | 80 | 49.311 | 31.746 | 45.333 | 1.00 | 38.67 | A | C |
| ATOM | 390 | O | GLU | A | 80 | 49.733 | 30.992 | 46.191 | 1.00 | 38.27 | A | O |
| ATOM | 391 | N | LEU | A | 81 | 48.587 | 32.818 | 45.613 | 1.00 | 36.24 | A | N |
| ATOM | 392 | CA | LEU | A | 81 | 48.231 | 33.133 | 46.980 | 1.00 | 34.61 | A | C |
| ATOM | 393 | CB | LEU | A | 81 | 48.089 | 34.646 | 47.147 | 1.00 | 34.92 | A | C |
| ATOM | 394 | CG | LEU | A | 81 | 49.382 | 35.434 | 46.925 | 1.00 | 34.71 | A | C |
| ATOM | 395 | CD1 | LEU | A | 81 | 49.062 | 36.888 | 46.638 | 1.00 | 35.66 | A | C |
| ATOM | 396 | CD2 | LEU | A | 81 | 50.279 | 35.291 | 48.145 | 1.00 | 33.74 | A | C |
| ATOM | 397 | C | LEU | A | 81 | 46.914 | 32.444 | 47.299 | 1.00 | 32.70 | A | C |
| ATOM | 398 | O | LEU | A | 81 | 45.976 | 32.494 | 46.514 | 1.00 | 33.64 | A | O |
| ATOM | 399 | N | VAL | A | 82 | 46.853 | 31.783 | 48.443 | 1.00 | 29.90 | A | N |
| ATOM | 400 | CA | VAL | A | 82 | 45.633 | 31.110 | 48.837 | 1.00 | 29.56 | A | C |
| ATOM | 401 | CB | VAL | A | 82 | 45.769 | 29.576 | 48.799 | 1.00 | 29.32 | A | C |
| ATOM | 402 | CG1 | VAL | A | 82 | 45.990 | 29.104 | 47.376 | 1.00 | 28.13 | A | C |
| ATOM | 403 | CG2 | VAL | A | 82 | 46.891 | 29.135 | 49.722 | 1.00 | 27.67 | A | C |
| ATOM | 404 | C | VAL | A | 82 | 45.269 | 31.497 | 50.252 | 1.00 | 29.12 | A | C |
| ATOM | 405 | O | VAL | A | 82 | 46.074 | 32.054 | 50.983 | 1.00 | 28.81 | A | O |
| ATOM | 406 | N | ALA | A | 83 | 44.034 | 31.191 | 50.622 | 1.00 | 29.18 | A | N |
| ATOM | 407 | CA | ALA | A | 83 | 43.548 | 31.474 | 51.957 | 1.00 | 29.07 | A | C |
| ATOM | 408 | CB | ALA | A | 83 | 42.254 | 32.274 | 51.888 | 1.00 | 28.84 | A | C |
| ATOM | 409 | C | ALA | A | 83 | 43.298 | 30.126 | 52.612 | 1.00 | 28.44 | A | C |
| ATOM | 410 | O | ALA | A | 83 | 42.836 | 29.195 | 51.959 | 1.00 | 28.54 | A | O |
| ATOM | 411 | N | ILE | A | 84 | 43.628 | 30.019 | 53.893 | 1.00 | 26.72 | A | N |
| ATOM | 412 | CA | ILE | A | 84 | 43.404 | 28.782 | 54.615 | 1.00 | 26.01 | A | C |
| ATOM | 413 | CB | ILE | A | 84 | 44.721 | 28.119 | 55.050 | 1.00 | 26.24 | A | C |
| ATOM | 414 | CG2 | ILE | A | 84 | 44.417 | 26.852 | 55.834 | 1.00 | 25.63 | A | C |
| ATOM | 415 | CG1 | ILE | A | 84 | 45.577 | 27.786 | 53.824 | 1.00 | 27.98 | A | C |
| ATOM | 416 | CD1 | ILE | A | 84 | 46.959 | 27.236 | 54.168 | 1.00 | 27.69 | A | C |
| ATOM | 417 | C | ILE | A | 84 | 42.586 | 29.078 | 55.860 | 1.00 | 27.11 | A | C |
| ATOM | 418 | O | ILE | A | 84 | 43.044 | 29.775 | 56.767 | 1.00 | 26.79 | A | O |
| ATOM | 419 | N | LYS | A | 85 | 41.366 | 28.552 | 55.891 | 1.00 | 26.91 | A | N |
| ATOM | 420 | CA | LYS | A | 85 | 40.481 | 28.741 | 57.027 | 1.00 | 25.96 | A | C |
| ATOM | 421 | CB | LYS | A | 85 | 39.032 | 28.911 | 56.560 | 1.00 | 24.28 | A | C |
| ATOM | 422 | CG | LYS | A | 85 | 38.041 | 29.138 | 57.691 | 1.00 | 22.07 | A | C |
| ATOM | 423 | CD | LYS | A | 85 | 36.641 | 29.434 | 57.166 | 1.00 | 22.14 | A | C |
| ATOM | 424 | CE | LYS | A | 85 | 35.680 | 29.713 | 58.303 | 1.00 | 19.26 | A | C |
| ATOM | 425 | NZ | LYS | A | 85 | 34.395 | 30.290 | 57.848 | 1.00 | 19.29 | A | N |
| ATOM | 426 | C | LYS | A | 85 | 40.613 | 27.509 | 57.911 | 1.00 | 27.57 | A | C |
| ATOM | 427 | O | LYS | A | 85 | 40.297 | 26.398 | 57.495 | 1.00 | 28.04 | A | O |
| ATOM | 428 | N | LYS | A | 86 | 41.094 | 27.721 | 59.129 | 1.00 | 27.79 | A | N |
| ATOM | 429 | CA | LYS | A | 86 | 41.296 | 26.631 | 60.072 | 1.00 | 28.55 | A | C |
| ATOM | 430 | CB | LYS | A | 86 | 42.763 | 26.599 | 60.507 | 1.00 | 28.39 | A | C |
| ATOM | 431 | CG | LYS | A | 86 | 43.109 | 25.484 | 61.455 | 1.00 | 28.76 | A | C |
| ATOM | 432 | CD | LYS | A | 86 | 44.599 | 25.452 | 61.717 | 1.00 | 30.21 | A | C |
| ATOM | 433 | CE | LYS | A | 86 | 44.982 | 24.208 | 62.490 | 1.00 | 29.24 | A | C |
| ATOM | 434 | NZ | LYS | A | 86 | 46.414 | 24.259 | 62.842 | 1.00 | 31.95 | A | N |
| ATOM | 435 | C | LYS | A | 86 | 40.397 | 26.808 | 61.282 | 1.00 | 28.53 | A | C |

| | | | | | | | | | | | | |
|---|---|---|---|---|---|---|---|---|---|---|---|---|
| ATOM | 436 | O | LYS | A | 86 | 40.567 | 27.739 | 62.060 | 1.00 | 29.97 | A | O |
| ATOM | 437 | N | VAL | A | 87 | 39.433 | 25.912 | 61.431 | 1.00 | 28.76 | A | N |
| ATOM | 438 | CA | VAL | A | 87 | 38.509 | 25.987 | 62.551 | 1.00 | 31.05 | A | C |
| ATOM | 439 | CB | VAL | A | 87 | 37.088 | 26.346 | 62.068 | 1.00 | 31.01 | A | C |
| ATOM | 440 | CG1 | VAL | A | 87 | 36.537 | 25.220 | 61.196 | 1.00 | 31.31 | A | C |
| ATOM | 441 | CG2 | VAL | A | 87 | 36.178 | 26.608 | 63.269 | 1.00 | 33.83 | A | C |
| ATOM | 442 | C | VAL | A | 87 | 38.483 | 24.651 | 63.286 | 1.00 | 32.18 | A | C |
| ATOM | 443 | O | VAL | A | 87 | 38.869 | 23.623 | 62.736 | 1.00 | 32.86 | A | O |
| ATOM | 444 | N | LEU | A | 88 | 38.021 | 24.681 | 64.530 | 1.00 | 33.24 | A | N |
| ATOM | 445 | CA | LEU | A | 88 | 37.954 | 23.494 | 65.362 | 1.00 | 33.98 | A | C |
| ATOM | 446 | CB | LEU | A | 88 | 37.599 | 23.910 | 66.793 | 1.00 | 35.07 | A | C |
| ATOM | 447 | CG | LEU | A | 88 | 37.942 | 22.986 | 67.966 | 1.00 | 35.34 | A | C |
| ATOM | 448 | CD1 | LEU | A | 88 | 37.202 | 21.684 | 67.812 | 1.00 | 35.31 | A | C |
| ATOM | 449 | CD2 | LEU | A | 88 | 39.444 | 22.749 | 68.018 | 1.00 | 35.33 | A | C |
| ATOM | 450 | C | LEU | A | 88 | 36.941 | 22.484 | 64.816 | 1.00 | 35.06 | A | C |
| ATOM | 451 | O | LEU | A | 88 | 35.806 | 22.830 | 64.483 | 1.00 | 35.11 | A | O |
| ATOM | 452 | N | GLN | A | 89 | 37.366 | 21.227 | 64.736 | 1.00 | 35.30 | A | N |
| ATOM | 453 | CA | GLN | A | 89 | 36.533 | 20.147 | 64.219 | 1.00 | 36.13 | A | C |
| ATOM | 454 | CB | GLN | A | 89 | 37.385 | 19.240 | 63.321 | 1.00 | 37.17 | A | C |
| ATOM | 455 | CG | GLN | A | 89 | 36.713 | 17.965 | 62.810 | 1.00 | 38.28 | A | C |
| ATOM | 456 | CD | GLN | A | 89 | 35.380 | 18.205 | 62.098 | 1.00 | 37.76 | A | C |
| ATOM | 457 | OE1 | GLN | A | 89 | 35.283 | 18.989 | 61.188 | 1.00 | 38.70 | A | O |
| ATOM | 458 | NE2 | GLN | A | 89 | 34.352 | 17.489 | 62.531 | 1.00 | 38.85 | A | N |
| ATOM | 459 | C | GLN | A | 89 | 35.905 | 19.328 | 65.334 | 1.00 | 36.18 | A | C |
| ATOM | 460 | O | GLN | A | 89 | 36.606 | 18.841 | 66.229 | 1.00 | 36.57 | A | O |
| ATOM | 461 | N | ALA | A | 90 | 34.582 | 19.185 | 65.291 | 1.00 | 36.14 | A | N |
| ATOM | 462 | CA | ALA | A | 90 | 33.886 | 18.396 | 66.301 | 1.00 | 37.75 | A | C |
| ATOM | 463 | CB | ALA | A | 90 | 32.410 | 18.737 | 66.333 | 1.00 | 36.48 | A | C |
| ATOM | 464 | C | ALA | A | 90 | 34.090 | 16.936 | 65.910 | 1.00 | 39.57 | A | C |
| ATOM | 465 | O | ALA | A | 90 | 33.777 | 16.521 | 64.788 | 1.00 | 39.68 | A | O |
| ATOM | 466 | N | ALA | A | 91 | 34.629 | 16.156 | 66.836 | 1.00 | 41.02 | A | N |
| ATOM | 467 | CA | ALA | A | 91 | 34.899 | 14.753 | 66.559 | 1.00 | 41.90 | A | C |
| ATOM | 468 | CB | ALA | A | 91 | 35.768 | 14.162 | 67.683 | 1.00 | 40.64 | A | C |
| ATOM | 469 | C | ALA | A | 91 | 33.606 | 13.941 | 66.381 | 1.00 | 42.48 | A | C |
| ATOM | 470 | O | ALA | A | 91 | 33.638 | 12.810 | 65.894 | 1.00 | 43.38 | A | O |
| ATOM | 471 | N | ALA | A | 92 | 32.462 | 14.538 | 66.710 | 1.00 | 42.34 | A | N |
| ATOM | 472 | CA | ALA | A | 92 | 31.171 | 13.834 | 66.621 | 1.00 | 42.47 | A | C |
| ATOM | 473 | CB | ALA | A | 92 | 30.246 | 14.343 | 67.726 | 1.00 | 41.77 | A | C |
| ATOM | 474 | C | ALA | A | 92 | 30.404 | 13.852 | 65.285 | 1.00 | 42.56 | A | C |
| ATOM | 475 | O | ALA | A | 92 | 29.665 | 12.914 | 64.989 | 1.00 | 42.97 | A | O |
| ATOM | 476 | N | ALA | A | 93 | 30.558 | 14.902 | 64.483 | 1.00 | 42.60 | A | N |
| ATOM | 477 | CA | ALA | A | 93 | 29.820 | 14.974 | 63.220 | 1.00 | 42.01 | A | C |
| ATOM | 478 | CB | ALA | A | 93 | 28.563 | 15.840 | 63.398 | 1.00 | 42.15 | A | C |
| ATOM | 479 | C | ALA | A | 93 | 30.670 | 15.538 | 62.099 | 1.00 | 41.23 | A | C |
| ATOM | 480 | O | ALA | A | 93 | 31.761 | 16.068 | 62.332 | 1.00 | 42.07 | A | O |
| ATOM | 481 | N | ALA | A | 94 | 30.178 | 15.421 | 60.872 | 1.00 | 39.86 | A | N |
| ATOM | 482 | CA | ALA | A | 94 | 30.918 | 15.963 | 59.743 | 1.00 | 36.91 | A | C |
| ATOM | 483 | CB | ALA | A | 94 | 30.402 | 15.365 | 58.432 | 1.00 | 38.31 | A | C |
| ATOM | 484 | C | ALA | A | 94 | 30.701 | 17.476 | 59.778 | 1.00 | 34.55 | A | C |
| ATOM | 485 | O | ALA | A | 94 | 29.674 | 17.946 | 60.241 | 1.00 | 32.26 | A | O |
| ATOM | 486 | N | ASN | A | 95 | 31.679 | 18.233 | 59.306 | 1.00 | 32.53 | A | N |
| ATOM | 487 | CA | ASN | A | 95 | 31.559 | 19.685 | 59.324 | 1.00 | 31.56 | A | C |
| ATOM | 488 | CB | ASN | A | 95 | 32.958 | 20.312 | 59.204 | 1.00 | 32.90 | A | C |
| ATOM | 489 | CG | ASN | A | 95 | 32.930 | 21.834 | 59.174 | 1.00 | 33.64 | A | C |
| ATOM | 490 | OD1 | ASN | A | 95 | 32.433 | 22.424 | 58.240 | 1.00 | 34.01 | A | O |
| ATOM | 491 | ND2 | ASN | A | 95 | 33.485 | 22.464 | 60.207 | 1.00 | 33.58 | A | N |
| ATOM | 492 | C | ASN | A | 95 | 30.621 | 20.215 | 58.233 | 1.00 | 30.12 | A | C |
| ATOM | 493 | O | ASN | A | 95 | 30.877 | 20.067 | 57.051 | 1.00 | 28.59 | A | O |
| ATOM | 494 | N | ARG | A | 96 | 29.528 | 20.836 | 58.662 | 1.00 | 27.88 | A | N |
| ATOM | 495 | CA | ARG | A | 96 | 28.525 | 21.367 | 57.745 | 1.00 | 26.92 | A | C |
| ATOM | 496 | CB | ARG | A | 96 | 27.372 | 21.984 | 58.547 | 1.00 | 26.31 | A | C |
| ATOM | 497 | CG | ARG | A | 96 | 26.193 | 22.442 | 57.700 | 1.00 | 27.72 | A | C |
| ATOM | 498 | CD | ARG | A | 96 | 25.224 | 23.275 | 58.522 | 1.00 | 28.87 | A | C |

| | | | | | | | | | | | | |
|---|---|---|---|---|---|---|---|---|---|---|---|---|
| ATOM | 499 | NE | ARG | A | 96 | 24.429 | 22.461 | 59.434 | 1.00 | 33.63 | A | N |
| ATOM | 500 | CZ | ARG | A | 96 | 23.686 | 22.951 | 60.427 | 1.00 | 33.88 | A | C |
| ATOM | 501 | NH1 | ARG | A | 96 | 23.639 | 24.265 | 60.645 | 1.00 | 31.87 | A | N |
| ATOM | 502 | NH2 | ARG | A | 96 | 22.982 | 22.124 | 61.193 | 1.00 | 32.49 | A | N |
| ATOM | 503 | C | ARG | A | 96 | 29.054 | 22.393 | 56.737 | 1.00 | 25.73 | A | C |
| ATOM | 504 | O | ARG | A | 96 | 28.611 | 22.424 | 55.592 | 1.00 | 24.03 | A | O |
| ATOM | 505 | N | GLU | A | 97 | 29.990 | 23.236 | 57.159 | 1.00 | 24.16 | A | N |
| ATOM | 506 | CA | GLU | A | 97 | 30.540 | 24.231 | 56.247 | 1.00 | 24.06 | A | C |
| ATOM | 507 | CB | GLU | A | 97 | 31.478 | 25.182 | 56.999 | 1.00 | 24.42 | A | C |
| ATOM | 508 | CG | GLU | A | 97 | 32.031 | 26.334 | 56.165 | 1.00 | 24.60 | A | C |
| ATOM | 509 | CD | GLU | A | 97 | 32.820 | 27.323 | 57.009 | 1.00 | 26.85 | A | C |
| ATOM | 510 | OE1 | GLU | A | 97 | 33.150 | 26.992 | 58.168 | 1.00 | 27.55 | A | O |
| ATOM | 511 | OE2 | GLU | A | 97 | 33.115 | 28.428 | 56.515 | 1.00 | 26.78 | A | O |
| ATOM | 512 | C | GLU | A | 97 | 31.279 | 23.533 | 55.110 | 1.00 | 24.85 | A | C |
| ATOM | 513 | O | GLU | A | 97 | 31.148 | 23.912 | 53.950 | 1.00 | 24.11 | A | O |
| ATOM | 514 | N | LEU | A | 98 | 32.047 | 22.503 | 55.459 | 1.00 | 24.42 | A | N |
| ATOM | 515 | CA | LEU | A | 98 | 32.797 | 21.737 | 54.475 | 1.00 | 26.18 | A | C |
| ATOM | 516 | CB | LEU | A | 98 | 33.611 | 20.648 | 55.167 | 1.00 | 27.48 | A | C |
| ATOM | 517 | CG | LEU | A | 98 | 34.271 | 19.632 | 54.234 | 1.00 | 25.78 | A | C |
| ATOM | 518 | CD1 | LEU | A | 98 | 35.211 | 20.329 | 53.258 | 1.00 | 21.87 | A | C |
| ATOM | 519 | CD2 | LEU | A | 98 | 35.022 | 18.624 | 55.076 | 1.00 | 25.82 | A | C |
| ATOM | 520 | C | LEU | A | 98 | 31.841 | 21.092 | 53.480 | 1.00 | 27.37 | A | C |
| ATOM | 521 | O | LEU | A | 98 | 31.966 | 21.278 | 52.285 | 1.00 | 26.79 | A | O |
| ATOM | 522 | N | GLN | A | 99 | 30.878 | 20.340 | 54.001 | 1.00 | 28.29 | A | N |
| ATOM | 523 | CA | GLN | A | 99 | 29.913 | 19.643 | 53.162 | 1.00 | 29.91 | A | C |
| ATOM | 524 | CB | GLN | A | 99 | 28.855 | 18.963 | 54.035 | 1.00 | 32.74 | A | C |
| ATOM | 525 | CG | GLN | A | 99 | 29.393 | 17.813 | 54.885 | 1.00 | 36.96 | A | C |
| ATOM | 526 | CD | GLN | A | 99 | 28.313 | 17.195 | 55.760 | 1.00 | 40.53 | A | C |
| ATOM | 527 | OE1 | GLN | A | 99 | 27.932 | 17.758 | 56.810 | 1.00 | 42.46 | A | O |
| ATOM | 528 | NE2 | GLN | A | 99 | 27.797 | 16.037 | 55.335 | 1.00 | 41.04 | A | N |
| ATOM | 529 | C | GLN | A | 99 | 29.244 | 20.566 | 52.151 | 1.00 | 30.10 | A | C |
| ATOM | 530 | O | GLN | A | 99 | 28.978 | 20.174 | 51.028 | 1.00 | 29.94 | A | O |
| ATOM | 531 | N | ILE | A | 100 | 28.982 | 21.803 | 52.549 | 1.00 | 29.63 | A | N |
| ATOM | 532 | CA | ILE | A | 100 | 28.352 | 22.745 | 51.638 | 1.00 | 28.47 | A | C |
| ATOM | 533 | CB | ILE | A | 100 | 27.732 | 23.914 | 52.429 | 1.00 | 28.03 | A | C |
| ATOM | 534 | CG2 | ILE | A | 100 | 27.334 | 25.041 | 51.496 | 1.00 | 28.21 | A | C |
| ATOM | 535 | CG1 | ILE | A | 100 | 26.517 | 23.403 | 53.216 | 1.00 | 28.42 | A | C |
| ATOM | 536 | CD1 | ILE | A | 100 | 25.863 | 24.440 | 54.122 | 1.00 | 27.04 | A | C |
| ATOM | 537 | C | ILE | A | 100 | 29.332 | 23.280 | 50.596 | 1.00 | 28.22 | A | C |
| ATOM | 538 | O | ILE | A | 100 | 29.006 | 23.344 | 49.438 | 1.00 | 29.68 | A | O |
| ATOM | 539 | N | MET | A | 101 | 30.531 | 23.654 | 51.027 | 1.00 | 28.62 | A | N |
| ATOM | 540 | CA | MET | A | 101 | 31.555 | 24.175 | 50.122 | 1.00 | 29.44 | A | C |
| ATOM | 541 | CB | MET | A | 101 | 32.839 | 24.473 | 50.899 | 1.00 | 31.00 | A | C |
| ATOM | 542 | CG | MET | A | 101 | 32.719 | 25.492 | 52.020 | 1.00 | 33.30 | A | C |
| ATOM | 543 | SD | MET | A | 101 | 32.696 | 27.165 | 51.414 | 1.00 | 35.35 | A | S |
| ATOM | 544 | CE | MET | A | 101 | 34.314 | 27.301 | 50.773 | 1.00 | 35.15 | A | C |
| ATOM | 545 | C | MET | A | 101 | 31.888 | 23.159 | 49.033 | 1.00 | 30.68 | A | C |
| ATOM | 546 | O | MET | A | 101 | 32.171 | 23.526 | 47.912 | 1.00 | 31.84 | A | O |
| ATOM | 547 | N | ARG | A | 102 | 31.863 | 21.878 | 49.388 | 1.00 | 31.84 | A | N |
| ATOM | 548 | CA | ARG | A | 102 | 32.195 | 20.826 | 48.441 | 1.00 | 32.66 | A | C |
| ATOM | 549 | CB | ARG | A | 102 | 32.285 | 19.477 | 49.149 | 1.00 | 32.24 | A | C |
| ATOM | 550 | CG | ARG | A | 102 | 33.431 | 19.404 | 50.126 | 1.00 | 33.70 | A | C |
| ATOM | 551 | CD | ARG | A | 102 | 33.641 | 17.985 | 50.604 | 1.00 | 35.68 | A | C |
| ATOM | 552 | NE | ARG | A | 102 | 34.271 | 17.149 | 49.586 | 1.00 | 36.80 | A | N |
| ATOM | 553 | CZ | ARG | A | 102 | 34.438 | 15.837 | 49.709 | 1.00 | 37.78 | A | C |
| ATOM | 554 | NH1 | ARG | A | 102 | 34.016 | 15.216 | 50.806 | 1.00 | 36.61 | A | N |
| ATOM | 555 | NH2 | ARG | A | 102 | 35.035 | 15.149 | 48.741 | 1.00 | 37.58 | A | N |
| ATOM | 556 | C | ARG | A | 102 | 31.232 | 20.722 | 47.280 | 1.00 | 32.61 | A | C |
| ATOM | 557 | O | ARG | A | 102 | 31.615 | 20.293 | 46.206 | 1.00 | 32.16 | A | O |
| ATOM | 558 | N | LYS | A | 103 | 29.985 | 21.125 | 47.479 | 1.00 | 31.91 | A | N |
| ATOM | 559 | CA | LYS | A | 103 | 29.050 | 21.023 | 46.377 | 1.00 | 32.56 | A | C |
| ATOM | 560 | CB | LYS | A | 103 | 27.745 | 20.372 | 46.864 | 1.00 | 33.92 | A | C |
| ATOM | 561 | CG | LYS | A | 103 | 26.925 | 21.183 | 47.827 | 1.00 | 35.89 | A | C |

| | | | | | | | | | | | | |
|---|---|---|---|---|---|---|---|---|---|---|---|---|
| ATOM | 562 | CD | LYS | A | 103 | 25.693 | 20.403 | 48.303 | 1.00 | 37.48 | A | C |
| ATOM | 563 | CE | LYS | A | 103 | 26.084 | 19.200 | 49.167 | 1.00 | 39.44 | A | C |
| ATOM | 564 | NZ | LYS | A | 103 | 24.919 | 18.460 | 49.742 | 1.00 | 37.19 | A | N |
| ATOM | 565 | C | LYS | A | 103 | 28.779 | 22.347 | 45.674 | 1.00 | 31.37 | A | C |
| ATOM | 566 | O | LYS | A | 103 | 27.853 | 22.442 | 44.889 | 1.00 | 31.67 | A | O |
| ATOM | 567 | N | LEU | A | 104 | 29.615 | 23.352 | 45.933 | 1.00 | 30.60 | A | N |
| ATOM | 568 | CA | LEU | A | 104 | 29.451 | 24.668 | 45.308 | 1.00 | 30.00 | A | C |
| ATOM | 569 | CB | LEU | A | 104 | 29.440 | 25.763 | 46.383 | 1.00 | 30.57 | A | C |
| ATOM | 570 | CG | LEU | A | 104 | 28.092 | 26.090 | 47.051 | 1.00 | 31.61 | A | C |
| ATOM | 571 | CD1 | LEU | A | 104 | 27.359 | 24.824 | 47.406 | 1.00 | 31.55 | A | C |
| ATOM | 572 | CD2 | LEU | A | 104 | 28.321 | 26.947 | 48.293 | 1.00 | 30.88 | A | C |
| ATOM | 573 | C | LEU | A | 104 | 30.511 | 24.985 | 44.252 | 1.00 | 29.44 | A | C |
| ATOM | 574 | O | LEU | A | 104 | 31.692 | 24.761 | 44.454 | 1.00 | 28.95 | A | O |
| ATOM | 575 | N | ASP | A | 105 | 30.058 | 25.505 | 43.116 | 1.00 | 29.83 | A | N |
| ATOM | 576 | CA | ASP | A | 105 | 30.948 | 25.875 | 42.023 | 1.00 | 30.50 | A | C |
| ATOM | 577 | CB | ASP | A | 105 | 31.166 | 24.685 | 41.084 | 1.00 | 32.96 | A | C |
| ATOM | 578 | CG | ASP | A | 105 | 32.224 | 24.959 | 40.026 | 1.00 | 35.00 | A | C |
| ATOM | 579 | OD1 | ASP | A | 105 | 33.320 | 25.438 | 40.389 | 1.00 | 36.67 | A | O |
| ATOM | 580 | OD2 | ASP | A | 105 | 31.963 | 24.682 | 38.837 | 1.00 | 35.50 | A | O |
| ATOM | 581 | C | ASP | A | 105 | 30.317 | 27.044 | 41.273 | 1.00 | 29.90 | A | C |
| ATOM | 582 | O | ASP | A | 105 | 29.458 | 26.859 | 40.406 | 1.00 | 30.14 | A | O |
| ATOM | 583 | N | HIS | A | 106 | 30.754 | 28.247 | 41.625 | 1.00 | 27.86 | A | N |
| ATOM | 584 | CA | HIS | A | 106 | 30.225 | 29.464 | 41.033 | 1.00 | 25.21 | A | C |
| ATOM | 585 | CB | HIS | A | 106 | 28.992 | 29.918 | 41.813 | 1.00 | 23.32 | A | C |
| ATOM | 586 | CG | HIS | A | 106 | 28.200 | 30.985 | 41.123 | 1.00 | 24.32 | A | C |
| ATOM | 587 | CD2 | HIS | A | 106 | 27.027 | 30.924 | 40.449 | 1.00 | 22.72 | A | C |
| ATOM | 588 | ND1 | HIS | A | 106 | 28.593 | 32.303 | 41.094 | 1.00 | 24.26 | A | N |
| ATOM | 589 | CE1 | HIS | A | 106 | 27.691 | 33.013 | 40.434 | 1.00 | 24.81 | A | C |
| ATOM | 590 | NE2 | HIS | A | 106 | 26.734 | 32.197 | 40.035 | 1.00 | 23.05 | A | N |
| ATOM | 591 | C | HIS | A | 106 | 31.287 | 30.548 | 41.056 | 1.00 | 24.83 | A | C |
| ATOM | 592 | O | HIS | A | 106 | 32.050 | 30.659 | 42.007 | 1.00 | 24.42 | A | O |
| ATOM | 593 | N | CYS | A | 107 | 31.313 | 31.346 | 39.996 | 1.00 | 26.11 | A | N |
| ATOM | 594 | CA | CYS | A | 107 | 32.276 | 32.427 | 39.843 | 1.00 | 26.34 | A | C |
| ATOM | 595 | CB | CYS | A | 107 | 32.111 | 33.083 | 38.461 | 1.00 | 26.91 | A | C |
| ATOM | 596 | SG | CYS | A | 107 | 30.484 | 33.874 | 38.165 | 1.00 | 33.29 | A | S |
| ATOM | 597 | C | CYS | A | 107 | 32.177 | 33.487 | 40.936 | 1.00 | 26.23 | A | C |
| ATOM | 598 | O | CYS | A | 107 | 33.121 | 34.243 | 41.146 | 1.00 | 25.83 | A | O |
| ATOM | 599 | N | ASN | A | 108 | 31.039 | 33.546 | 41.629 | 1.00 | 25.73 | A | N |
| ATOM | 600 | CA | ASN | A | 108 | 30.861 | 34.533 | 42.698 | 1.00 | 24.30 | A | C |
| ATOM | 601 | CB | ASN | A | 108 | 29.560 | 35.325 | 42.490 | 1.00 | 24.94 | A | C |
| ATOM | 602 | CG | ASN | A | 108 | 29.589 | 36.209 | 41.248 | 1.00 | 23.76 | A | C |
| ATOM | 603 | OD1 | ASN | A | 108 | 28.644 | 36.256 | 40.519 | 1.00 | 25.46 | A | O |
| ATOM | 604 | ND2 | ASN | A | 108 | 30.682 | 36.916 | 41.036 | 1.00 | 23.07 | A | N |
| ATOM | 605 | C | ASN | A | 108 | 30.887 | 33.931 | 44.114 | 1.00 | 25.00 | A | C |
| ATOM | 606 | O | ASN | A | 108 | 30.325 | 34.500 | 45.048 | 1.00 | 24.27 | A | O |
| ATOM | 607 | N | ILE | A | 109 | 31.544 | 32.782 | 44.264 | 1.00 | 24.92 | A | N |
| ATOM | 608 | CA | ILE | A | 109 | 31.679 | 32.119 | 45.563 | 1.00 | 24.42 | A | C |
| ATOM | 609 | CB | ILE | A | 109 | 30.825 | 30.816 | 45.650 | 1.00 | 26.44 | A | C |
| ATOM | 610 | CG2 | ILE | A | 109 | 30.955 | 30.190 | 47.036 | 1.00 | 23.13 | A | C |
| ATOM | 611 | CG1 | ILE | A | 109 | 29.352 | 31.113 | 45.348 | 1.00 | 26.69 | A | C |
| ATOM | 612 | CD1 | ILE | A | 109 | 28.689 | 32.016 | 46.352 | 1.00 | 32.06 | A | C |
| ATOM | 613 | C | ILE | A | 109 | 33.149 | 31.724 | 45.712 | 1.00 | 25.75 | A | C |
| ATOM | 614 | O | ILE | A | 109 | 33.737 | 31.205 | 44.775 | 1.00 | 26.60 | A | O |
| ATOM | 615 | N | VAL | A | 110 | 33.756 | 31.972 | 46.869 | 1.00 | 24.89 | A | N |
| ATOM | 616 | CA | VAL | A | 110 | 35.154 | 31.584 | 47.027 | 1.00 | 25.43 | A | C |
| ATOM | 617 | CB | VAL | A | 110 | 35.706 | 31.847 | 48.442 | 1.00 | 25.74 | A | C |
| ATOM | 618 | CG1 | VAL | A | 110 | 36.265 | 33.239 | 48.511 | 1.00 | 27.82 | A | C |
| ATOM | 619 | CG2 | VAL | A | 110 | 34.623 | 31.626 | 49.495 | 1.00 | 24.55 | A | C |
| ATOM | 620 | C | VAL | A | 110 | 35.262 | 30.095 | 46.755 | 1.00 | 26.31 | A | C |
| ATOM | 621 | O | VAL | A | 110 | 34.450 | 29.312 | 47.226 | 1.00 | 25.50 | A | O |
| ATOM | 622 | N | ARG | A | 111 | 36.278 | 29.711 | 45.998 | 1.00 | 27.27 | A | N |
| ATOM | 623 | CA | ARG | A | 111 | 36.459 | 28.313 | 45.645 | 1.00 | 28.36 | A | C |
| ATOM | 624 | CB | ARG | A | 111 | 37.207 | 28.219 | 44.310 | 1.00 | 31.91 | A | C |

| | | | | | | | | | | | | |
|---|---|---|---|---|---|---|---|---|---|---|---|---|
| ATOM | 625 | CG | ARG | A | 111 | 37.234 | 26.833 | 43.642 | 1.00 | 36.37 | A | C |
| ATOM | 626 | CD | ARG | A | 111 | 38.125 | 26.856 | 42.378 | 1.00 | 38.84 | A | C |
| ATOM | 627 | NE | ARG | A | 111 | 39.296 | 25.989 | 42.537 | 1.00 | 44.01 | A | N |
| ATOM | 628 | CZ | ARG | A | 111 | 40.298 | 25.885 | 41.661 | 1.00 | 45.92 | A | C |
| ATOM | 629 | NH1 | ARG | A | 111 | 40.291 | 26.591 | 40.533 | 1.00 | 46.76 | A | N |
| ATOM | 630 | NH2 | ARG | A | 111 | 41.331 | 25.091 | 41.929 | 1.00 | 47.57 | A | N |
| ATOM | 631 | C | ARG | A | 111 | 37.220 | 27.523 | 46.699 | 1.00 | 27.30 | A | C |
| ATOM | 632 | O | ARG | A | 111 | 38.222 | 27.978 | 47.232 | 1.00 | 26.21 | A | O |
| ATOM | 633 | N | LEU | A | 112 | 36.721 | 26.334 | 47.005 | 1.00 | 27.85 | A | N |
| ATOM | 634 | CA | LEU | A | 112 | 37.404 | 25.458 | 47.948 | 1.00 | 28.30 | A | C |
| ATOM | 635 | CB | LEU | A | 112 | 36.408 | 24.538 | 48.652 | 1.00 | 27.34 | A | C |
| ATOM | 636 | CG | LEU | A | 112 | 37.040 | 23.528 | 49.614 | 1.00 | 28.58 | A | C |
| ATOM | 637 | CD1 | LEU | A | 112 | 37.530 | 24.240 | 50.872 | 1.00 | 27.84 | A | C |
| ATOM | 638 | CD2 | LEU | A | 112 | 36.024 | 22.458 | 49.971 | 1.00 | 26.40 | A | C |
| ATOM | 639 | C | LEU | A | 112 | 38.357 | 24.623 | 47.085 | 1.00 | 28.38 | A | C |
| ATOM | 640 | O | LEU | A | 112 | 37.925 | 23.727 | 46.389 | 1.00 | 27.42 | A | O |
| ATOM | 641 | N | ARG | A | 113 | 39.647 | 24.936 | 47.118 | 1.00 | 29.93 | A | N |
| ATOM | 642 | CA | ARG | A | 113 | 40.609 | 24.192 | 46.311 | 1.00 | 32.07 | A | C |
| ATOM | 643 | CB | ARG | A | 113 | 41.898 | 24.999 | 46.122 | 1.00 | 33.62 | A | C |
| ATOM | 644 | CG | ARG | A | 113 | 41.734 | 26.475 | 46.434 | 1.00 | 38.94 | A | C |
| ATOM | 645 | CD | ARG | A | 113 | 41.973 | 27.377 | 45.233 | 1.00 | 41.20 | A | C |
| ATOM | 646 | NE | ARG | A | 113 | 43.379 | 27.380 | 44.841 | 1.00 | 44.83 | A | N |
| ATOM | 647 | CZ | ARG | A | 113 | 43.921 | 28.270 | 44.013 | 1.00 | 44.75 | A | C |
| ATOM | 648 | NH1 | ARG | A | 113 | 43.169 | 29.240 | 43.489 | 1.00 | 42.53 | A | N |
| ATOM | 649 | NH2 | ARG | A | 113 | 45.212 | 28.179 | 43.709 | 1.00 | 43.78 | A | N |
| ATOM | 650 | C | ARG | A | 113 | 40.923 | 22.857 | 46.979 | 1.00 | 31.18 | A | C |
| ATOM | 651 | O | ARG | A | 113 | 40.882 | 21.822 | 46.355 | 1.00 | 32.30 | A | O |
| ATOM | 652 | N | TYR | A | 114 | 41.221 | 22.903 | 48.266 | 1.00 | 31.26 | A | N |
| ATOM | 653 | CA | TYR | A | 114 | 41.542 | 21.702 | 49.023 | 1.00 | 30.92 | A | C |
| ATOM | 654 | CB | TYR | A | 114 | 43.059 | 21.517 | 49.111 | 1.00 | 31.74 | A | C |
| ATOM | 655 | CG | TYR | A | 114 | 43.781 | 21.408 | 47.790 | 1.00 | 34.41 | A | C |
| ATOM | 656 | CD1 | TYR | A | 114 | 43.638 | 20.276 | 46.980 | 1.00 | 37.17 | A | C |
| ATOM | 657 | CE1 | TYR | A | 114 | 44.320 | 20.170 | 45.758 | 1.00 | 38.28 | A | C |
| ATOM | 658 | CD2 | TYR | A | 114 | 44.623 | 22.433 | 47.354 | 1.00 | 35.77 | A | C |
| ATOM | 659 | CE2 | TYR | A | 114 | 45.305 | 22.341 | 46.139 | 1.00 | 37.03 | A | C |
| ATOM | 660 | CZ | TYR | A | 114 | 45.152 | 21.211 | 45.350 | 1.00 | 38.59 | A | C |
| ATOM | 661 | OH | TYR | A | 114 | 45.813 | 21.125 | 44.149 | 1.00 | 41.48 | A | O |
| ATOM | 662 | C | TYR | A | 114 | 41.009 | 21.807 | 50.446 | 1.00 | 30.08 | A | C |
| ATOM | 663 | O | TYR | A | 114 | 40.527 | 22.856 | 50.874 | 1.00 | 29.73 | A | O |
| ATOM | 664 | N | PHE | A | 115 | 41.113 | 20.698 | 51.168 | 1.00 | 29.55 | A | N |
| ATOM | 665 | CA | PHE | A | 115 | 40.726 | 20.629 | 52.563 | 1.00 | 29.94 | A | C |
| ATOM | 666 | CB | PHE | A | 115 | 39.194 | 20.494 | 52.714 | 1.00 | 28.30 | A | C |
| ATOM | 667 | CG | PHE | A | 115 | 38.666 | 19.090 | 52.629 | 1.00 | 28.34 | A | C |
| ATOM | 668 | CD1 | PHE | A | 115 | 38.677 | 18.257 | 53.745 | 1.00 | 28.64 | A | C |
| ATOM | 669 | CD2 | PHE | A | 115 | 38.113 | 18.611 | 51.445 | 1.00 | 28.79 | A | C |
| ATOM | 670 | CE1 | PHE | A | 115 | 38.143 | 16.968 | 53.686 | 1.00 | 28.04 | A | C |
| ATOM | 671 | CE2 | PHE | A | 115 | 37.577 | 17.324 | 51.376 | 1.00 | 28.27 | A | C |
| ATOM | 672 | CZ | PHE | A | 115 | 37.593 | 16.502 | 52.500 | 1.00 | 28.65 | A | C |
| ATOM | 673 | C | PHE | A | 115 | 41.488 | 19.456 | 53.177 | 1.00 | 31.17 | A | C |
| ATOM | 674 | O | PHE | A | 115 | 41.646 | 18.406 | 52.561 | 1.00 | 31.90 | A | O |
| ATOM | 675 | N | PHE | A | 116 | 42.012 | 19.668 | 54.378 | 1.00 | 32.24 | A | N |
| ATOM | 676 | CA | PHE | A | 116 | 42.772 | 18.639 | 55.062 | 1.00 | 32.08 | A | C |
| ATOM | 677 | CB | PHE | A | 116 | 44.234 | 18.681 | 54.630 | 1.00 | 32.39 | A | C |
| ATOM | 678 | CG | PHE | A | 116 | 44.939 | 19.957 | 54.977 | 1.00 | 31.34 | A | C |
| ATOM | 679 | CD1 | PHE | A | 116 | 45.609 | 20.092 | 56.190 | 1.00 | 30.55 | A | C |
| ATOM | 680 | CD2 | PHE | A | 116 | 44.961 | 21.017 | 54.075 | 1.00 | 30.13 | A | C |
| ATOM | 681 | CE1 | PHE | A | 116 | 46.298 | 21.265 | 56.496 | 1.00 | 29.65 | A | C |
| ATOM | 682 | CE2 | PHE | A | 116 | 45.642 | 22.188 | 54.369 | 1.00 | 30.18 | A | C |
| ATOM | 683 | CZ | PHE | A | 116 | 46.315 | 22.314 | 55.583 | 1.00 | 29.75 | A | C |
| ATOM | 684 | C | PHE | A | 116 | 42.656 | 18.842 | 56.547 | 1.00 | 33.30 | A | C |
| ATOM | 685 | O | PHE | A | 116 | 42.134 | 19.844 | 56.990 | 1.00 | 33.47 | A | O |
| ATOM | 686 | N | TYR | A | 117 | 43.159 | 17.880 | 57.308 | 1.00 | 34.48 | A | N |
| ATOM | 687 | CA | TYR | A | 117 | 43.093 | 17.932 | 58.759 | 1.00 | 35.57 | A | C |

| | | | | | | | | | | | | |
|---|---|---|---|---|---|---|---|---|---|---|---|---|
| ATOM | 688 | CB | TYR | A | 117 | 42.394 | 16.672 | 59.255 | 1.00 | 35.84 | A | C |
| ATOM | 689 | CG | TYR | A | 117 | 40.951 | 16.593 | 58.825 | 1.00 | 37.03 | A | C |
| ATOM | 690 | CD1 | TYR | A | 117 | 39.961 | 17.270 | 59.535 | 1.00 | 37.57 | A | C |
| ATOM | 691 | CE1 | TYR | A | 117 | 38.626 | 17.225 | 59.135 | 1.00 | 37.56 | A | C |
| ATOM | 692 | CD2 | TYR | A | 117 | 40.573 | 15.864 | 57.695 | 1.00 | 37.79 | A | C |
| ATOM | 693 | CE2 | TYR | A | 117 | 39.234 | 15.810 | 57.282 | 1.00 | 36.71 | A | C |
| ATOM | 694 | CZ | TYR | A | 117 | 38.270 | 16.496 | 58.010 | 1.00 | 38.06 | A | C |
| ATOM | 695 | OH | TYR | A | 117 | 36.948 | 16.466 | 57.624 | 1.00 | 38.45 | A | O |
| ATOM | 696 | C | TYR | A | 117 | 44.431 | 18.085 | 59.450 | 1.00 | 36.75 | A | C |
| ATOM | 697 | O | TYR | A | 117 | 45.432 | 17.535 | 59.001 | 1.00 | 38.74 | A | O |
| ATOM | 698 | N | SER | A | 118 | 44.429 | 18.831 | 60.550 | 1.00 | 36.72 | A | N |
| ATOM | 699 | CA | SER | A | 118 | 45.635 | 19.071 | 61.334 | 1.00 | 37.40 | A | C |
| ATOM | 700 | CB | SER | A | 118 | 45.992 | 20.558 | 61.332 | 1.00 | 37.93 | A | C |
| ATOM | 701 | OG | SER | A | 118 | 46.248 | 21.021 | 60.022 | 1.00 | 40.53 | A | O |
| ATOM | 702 | C | SER | A | 118 | 45.419 | 18.625 | 62.774 | 1.00 | 37.39 | A | C |
| ATOM | 703 | O | SER | A | 118 | 46.218 | 18.949 | 63.644 | 1.00 | 38.48 | A | O |
| ATOM | 704 | N | TYR | A | 127 | 41.390 | 19.745 | 63.848 | 1.00 | 37.57 | A | N |
| ATOM | 705 | CA | TYR | A | 127 | 41.199 | 20.948 | 63.030 | 1.00 | 37.96 | A | C |
| ATOM | 706 | CB | TYR | A | 127 | 42.422 | 21.860 | 63.146 | 1.00 | 39.52 | A | C |
| ATOM | 707 | CG | TYR | A | 127 | 42.579 | 22.517 | 64.493 | 1.00 | 42.15 | A | C |
| ATOM | 708 | CD1 | TYR | A | 127 | 41.862 | 23.666 | 64.815 | 1.00 | 42.97 | A | C |
| ATOM | 709 | CE1 | TYR | A | 127 | 41.979 | 24.263 | 66.069 | 1.00 | 44.69 | A | C |
| ATOM | 710 | CD2 | TYR | A | 127 | 43.423 | 21.971 | 65.460 | 1.00 | 43.68 | A | C |
| ATOM | 711 | CE2 | TYR | A | 127 | 43.550 | 22.557 | 66.726 | 1.00 | 45.11 | A | C |
| ATOM | 712 | CZ | TYR | A | 127 | 42.819 | 23.705 | 67.024 | 1.00 | 45.43 | A | C |
| ATOM | 713 | OH | TYR | A | 127 | 42.916 | 24.293 | 68.271 | 1.00 | 44.98 | A | O |
| ATOM | 714 | C | TYR | A | 127 | 40.935 | 20.695 | 61.542 | 1.00 | 36.97 | A | C |
| ATOM | 715 | O | TYR | A | 127 | 41.614 | 19.887 | 60.902 | 1.00 | 37.79 | A | O |
| ATOM | 716 | N | LEU | A | 128 | 39.943 | 21.396 | 60.999 | 1.00 | 34.67 | A | N |
| ATOM | 717 | CA | LEU | A | 128 | 39.625 | 21.295 | 59.579 | 1.00 | 32.45 | A | C |
| ATOM | 718 | CB | LEU | A | 128 | 38.111 | 21.298 | 59.344 | 1.00 | 32.18 | A | C |
| ATOM | 719 | CG | LEU | A | 128 | 37.660 | 21.626 | 57.915 | 1.00 | 30.60 | A | C |
| ATOM | 720 | CD1 | LEU | A | 128 | 38.258 | 20.639 | 56.933 | 1.00 | 30.01 | A | C |
| ATOM | 721 | CD2 | LEU | A | 128 | 36.150 | 21.601 | 57.849 | 1.00 | 31.54 | A | C |
| ATOM | 722 | C | LEU | A | 128 | 40.242 | 22.515 | 58.909 | 1.00 | 31.35 | A | C |
| ATOM | 723 | O | LEU | A | 128 | 40.040 | 23.625 | 59.352 | 1.00 | 30.98 | A | O |
| ATOM | 724 | N | ASN | A | 129 | 41.010 | 22.287 | 57.849 | 1.00 | 30.09 | A | N |
| ATOM | 725 | CA | ASN | A | 129 | 41.662 | 23.364 | 57.120 | 1.00 | 29.08 | A | C |
| ATOM | 726 | CB | ASN | A | 129 | 43.162 | 23.080 | 56.980 | 1.00 | 28.44 | A | C |
| ATOM | 727 | CG | ASN | A | 129 | 43.877 | 23.004 | 58.323 | 1.00 | 28.92 | A | C |
| ATOM | 728 | OD1 | ASN | A | 129 | 43.803 | 22.002 | 59.017 | 1.00 | 30.94 | A | O |
| ATOM | 729 | ND2 | ASN | A | 129 | 44.567 | 24.075 | 58.689 | 1.00 | 28.68 | A | N |
| ATOM | 730 | C | ASN | A | 129 | 41.030 | 23.497 | 55.738 | 1.00 | 29.96 | A | C |
| ATOM | 731 | O | ASN | A | 129 | 41.002 | 22.526 | 54.949 | 1.00 | 29.81 | A | O |
| ATOM | 732 | N | LEU | A | 130 | 40.511 | 24.683 | 55.434 | 1.00 | 28.53 | A | N |
| ATOM | 733 | CA | LEU | A | 130 | 39.885 | 24.895 | 54.133 | 1.00 | 27.02 | A | C |
| ATOM | 734 | CB | LEU | A | 130 | 38.513 | 25.564 | 54.301 | 1.00 | 26.92 | A | C |
| ATOM | 735 | CG | LEU | A | 130 | 37.440 | 24.828 | 55.113 | 1.00 | 25.96 | A | C |
| ATOM | 736 | CD1 | LEU | A | 130 | 36.347 | 25.817 | 55.477 | 1.00 | 25.82 | A | C |
| ATOM | 737 | CD2 | LEU | A | 130 | 36.872 | 23.653 | 54.338 | 1.00 | 24.23 | A | C |
| ATOM | 738 | C | LEU | A | 130 | 40.784 | 25.756 | 53.259 | 1.00 | 26.37 | A | C |
| ATOM | 739 | O | LEU | A | 130 | 40.991 | 26.916 | 53.539 | 1.00 | 25.84 | A | O |
| ATOM | 740 | N | VAL | A | 131 | 41.327 | 25.157 | 52.203 | 1.00 | 26.54 | A | N |
| ATOM | 741 | CA | VAL | A | 131 | 42.199 | 25.882 | 51.285 | 1.00 | 26.12 | A | C |
| ATOM | 742 | CB | VAL | A | 131 | 43.176 | 24.926 | 50.545 | 1.00 | 26.38 | A | C |
| ATOM | 743 | CG1 | VAL | A | 131 | 44.251 | 25.734 | 49.839 | 1.00 | 25.73 | A | C |
| ATOM | 744 | CG2 | VAL | A | 131 | 43.809 | 23.953 | 51.529 | 1.00 | 25.29 | A | C |
| ATOM | 745 | C | VAL | A | 131 | 41.291 | 26.581 | 50.276 | 1.00 | 26.60 | A | C |
| ATOM | 746 | O | VAL | A | 131 | 40.776 | 25.963 | 49.355 | 1.00 | 27.40 | A | O |
| ATOM | 747 | N | LEU | A | 132 | 41.092 | 27.878 | 50.478 | 1.00 | 25.68 | A | N |
| ATOM | 748 | CA | LEU | A | 132 | 40.223 | 28.666 | 49.619 | 1.00 | 24.63 | A | C |
| ATOM | 749 | CB | LEU | A | 132 | 39.235 | 29.457 | 50.473 | 1.00 | 23.45 | A | C |
| ATOM | 750 | CG | LEU | A | 132 | 38.509 | 28.714 | 51.588 | 1.00 | 23.88 | A | C |

| | | | | | | | | | | | | |
|---|---|---|---|---|---|---|---|---|---|---|---|---|
| ATOM | 751 | CD1 | LEU | A | 132 | 37.869 | 29.709 | 52.526 | 1.00 | 23.36 | A | C |
| ATOM | 752 | CD2 | LEU | A | 132 | 37.475 | 27.789 | 51.003 | 1.00 | 22.29 | A | C |
| ATOM | 753 | C | LEU | A | 132 | 41.015 | 29.651 | 48.781 | 1.00 | 25.28 | A | C |
| ATOM | 754 | O | LEU | A | 132 | 42.193 | 29.904 | 49.046 | 1.00 | 24.89 | A | O |
| ATOM | 755 | N | ASP | A | 133 | 40.356 | 30.200 | 47.764 | 1.00 | 25.85 | A | N |
| ATOM | 756 | CA | ASP | A | 133 | 40.982 | 31.204 | 46.924 | 1.00 | 27.52 | A | C |
| ATOM | 757 | CB | ASP | A | 133 | 40.052 | 31.690 | 45.816 | 1.00 | 30.24 | A | C |
| ATOM | 758 | CG | ASP | A | 133 | 39.870 | 30.688 | 44.714 | 1.00 | 32.98 | A | C |
| ATOM | 759 | OD1 | ASP | A | 133 | 40.842 | 29.966 | 44.389 | 1.00 | 34.80 | A | O |
| ATOM | 760 | OD2 | ASP | A | 133 | 38.750 | 30.648 | 44.155 | 1.00 | 36.35 | A | O |
| ATOM | 761 | C | ASP | A | 133 | 41.226 | 32.391 | 47.826 | 1.00 | 27.54 | A | C |
| ATOM | 762 | O | ASP | A | 133 | 40.477 | 32.629 | 48.757 | 1.00 | 28.23 | A | O |
| ATOM | 763 | N | TYR | A | 134 | 42.276 | 33.139 | 47.548 | 1.00 | 28.07 | A | N |
| ATOM | 764 | CA | TYR | A | 134 | 42.534 | 34.320 | 48.336 | 1.00 | 28.58 | A | C |
| ATOM | 765 | CB | TYR | A | 134 | 44.007 | 34.435 | 48.690 | 1.00 | 29.78 | A | C |
| ATOM | 766 | CG | TYR | A | 134 | 44.360 | 35.824 | 49.144 | 1.00 | 30.31 | A | C |
| ATOM | 767 | CD1 | TYR | A | 134 | 44.011 | 36.275 | 50.417 | 1.00 | 29.57 | A | C |
| ATOM | 768 | CE1 | TYR | A | 134 | 44.268 | 37.581 | 50.805 | 1.00 | 31.23 | A | C |
| ATOM | 769 | CD2 | TYR | A | 134 | 44.982 | 36.715 | 48.272 | 1.00 | 30.73 | A | C |
| ATOM | 770 | CE2 | TYR | A | 134 | 45.240 | 38.019 | 48.646 | 1.00 | 31.63 | A | C |
| ATOM | 771 | CZ | TYR | A | 134 | 44.885 | 38.448 | 49.910 | 1.00 | 32.23 | A | C |
| ATOM | 772 | OH | TYR | A | 134 | 45.151 | 39.748 | 50.272 | 1.00 | 33.70 | A | O |
| ATOM | 773 | C | TYR | A | 134 | 42.119 | 35.515 | 47.491 | 1.00 | 28.83 | A | C |
| ATOM | 774 | O | TYR | A | 134 | 42.466 | 35.602 | 46.307 | 1.00 | 29.47 | A | O |
| ATOM | 775 | N | VAL | A | 135 | 41.362 | 36.422 | 48.091 | 1.00 | 27.56 | A | N |
| ATOM | 776 | CA | VAL | A | 135 | 40.915 | 37.621 | 47.399 | 1.00 | 26.92 | A | C |
| ATOM | 777 | CB | VAL | A | 135 | 39.374 | 37.697 | 47.399 | 1.00 | 27.43 | A | C |
| ATOM | 778 | CG1 | VAL | A | 135 | 38.889 | 38.851 | 46.548 | 1.00 | 24.42 | A | C |
| ATOM | 779 | CG2 | VAL | A | 135 | 38.820 | 36.391 | 46.860 | 1.00 | 25.36 | A | C |
| ATOM | 780 | C | VAL | A | 135 | 41.573 | 38.756 | 48.184 | 1.00 | 27.34 | A | C |
| ATOM | 781 | O | VAL | A | 135 | 41.580 | 38.750 | 49.389 | 1.00 | 26.12 | A | O |
| ATOM | 782 | N | PRO | A | 136 | 42.156 | 39.733 | 47.479 | 1.00 | 28.37 | A | N |
| ATOM | 783 | CD | PRO | A | 136 | 42.039 | 39.879 | 46.015 | 1.00 | 27.81 | A | C |
| ATOM | 784 | CA | PRO | A | 136 | 42.854 | 40.885 | 48.057 | 1.00 | 28.39 | A | C |
| ATOM | 785 | CB | PRO | A | 136 | 43.475 | 41.544 | 46.832 | 1.00 | 27.05 | A | C |
| ATOM | 786 | CG | PRO | A | 136 | 42.425 | 41.323 | 45.797 | 1.00 | 29.44 | A | C |
| ATOM | 787 | C | PRO | A | 136 | 42.075 | 41.887 | 48.896 | 1.00 | 28.18 | A | C |
| ATOM | 788 | O | PRO | A | 136 | 42.636 | 42.522 | 49.790 | 1.00 | 29.18 | A | O |
| ATOM | 789 | N | GLU | A | 137 | 40.788 | 42.033 | 48.626 | 1.00 | 27.38 | A | N |
| ATOM | 790 | CA | GLU | A | 137 | 40.013 | 43.022 | 49.349 | 1.00 | 26.22 | A | C |
| ATOM | 791 | CB | GLU | A | 137 | 39.940 | 44.286 | 48.482 | 1.00 | 27.87 | A | C |
| ATOM | 792 | CG | GLU | A | 137 | 39.646 | 45.554 | 49.239 | 1.00 | 30.15 | A | C |
| ATOM | 793 | CD | GLU | A | 137 | 40.706 | 45.876 | 50.255 | 1.00 | 29.54 | A | C |
| ATOM | 794 | OE1 | GLU | A | 137 | 41.845 | 46.200 | 49.859 | 1.00 | 30.88 | A | O |
| ATOM | 795 | OE2 | GLU | A | 137 | 40.397 | 45.799 | 51.457 | 1.00 | 33.09 | A | O |
| ATOM | 796 | C | GLU | A | 137 | 38.610 | 42.551 | 49.720 | 1.00 | 25.32 | A | C |
| ATOM | 797 | O | GLU | A | 137 | 38.173 | 41.507 | 49.294 | 1.00 | 24.97 | A | O |
| ATOM | 798 | N | THR | A | 138 | 37.921 | 43.349 | 50.531 | 1.00 | 25.37 | A | N |
| ATOM | 799 | CA | THR | A | 138 | 36.554 | 43.053 | 50.963 | 1.00 | 23.31 | A | C |
| ATOM | 800 | CB | THR | A | 138 | 36.476 | 42.694 | 52.467 | 1.00 | 22.37 | A | C |
| ATOM | 801 | OG1 | THR | A | 138 | 36.848 | 43.836 | 53.250 | 1.00 | 21.65 | A | O |
| ATOM | 802 | CG2 | THR | A | 138 | 37.383 | 41.521 | 52.790 | 1.00 | 17.71 | A | C |
| ATOM | 803 | C | THR | A | 138 | 35.680 | 44.283 | 50.766 | 1.00 | 24.19 | A | C |
| ATOM | 804 | O | THR | A | 138 | 36.177 | 45.380 | 50.599 | 1.00 | 25.72 | A | O |
| ATOM | 805 | N | VAL | A | 139 | 34.368 | 44.089 | 50.788 | 1.00 | 24.02 | A | N |
| ATOM | 806 | CA | VAL | A | 139 | 33.460 | 45.212 | 50.646 | 1.00 | 22.25 | A | C |
| ATOM | 807 | CB | VAL | A | 139 | 32.016 | 44.741 | 50.425 | 1.00 | 21.33 | A | C |
| ATOM | 808 | CG1 | VAL | A | 139 | 31.064 | 45.925 | 50.509 | 1.00 | 20.32 | A | C |
| ATOM | 809 | CG2 | VAL | A | 139 | 31.895 | 44.077 | 49.062 | 1.00 | 22.48 | A | C |
| ATOM | 810 | C | VAL | A | 139 | 33.538 | 46.043 | 51.927 | 1.00 | 23.06 | A | C |
| ATOM | 811 | O | VAL | A | 139 | 33.522 | 47.263 | 51.885 | 1.00 | 23.39 | A | O |
| ATOM | 812 | N | TYR | A | 140 | 33.637 | 45.373 | 53.067 | 1.00 | 22.36 | A | N |
| ATOM | 813 | CA | TYR | A | 140 | 33.727 | 46.089 | 54.331 | 1.00 | 22.67 | A | C |

| | | | | | | | | | | | | |
|---|---|---|---|---|---|---|---|---|---|---|---|---|
| ATOM | 814 | CB | TYR | A | 140 | 33.961 | 45.118 | 55.484 | 1.00 | 20.99 | A | C |
| ATOM | 815 | CG | TYR | A | 140 | 34.085 | 45.788 | 56.838 | 1.00 | 21.76 | A | C |
| ATOM | 816 | CD1 | TYR | A | 140 | 32.976 | 46.367 | 57.465 | 1.00 | 20.71 | A | C |
| ATOM | 817 | CE1 | TYR | A | 140 | 33.094 | 46.992 | 58.711 | 1.00 | 22.90 | A | C |
| ATOM | 818 | CD2 | TYR | A | 140 | 35.314 | 45.852 | 57.485 | 1.00 | 21.20 | A | C |
| ATOM | 819 | CE2 | TYR | A | 140 | 35.445 | 46.472 | 58.721 | 1.00 | 23.96 | A | C |
| ATOM | 820 | CZ | TYR | A | 140 | 34.337 | 47.038 | 59.329 | 1.00 | 24.06 | A | C |
| ATOM | 821 | OH | TYR | A | 140 | 34.491 | 47.635 | 60.552 | 1.00 | 25.70 | A | O |
| ATOM | 822 | C | TYR | A | 140 | 34.862 | 47.116 | 54.291 | 1.00 | 24.19 | A | C |
| ATOM | 823 | O | TYR | A | 140 | 34.632 | 48.285 | 54.541 | 1.00 | 24.36 | A | O |
| ATOM | 824 | N | ARG | A | 141 | 36.076 | 46.669 | 53.963 | 1.00 | 24.94 | A | N |
| ATOM | 825 | CA | ARG | A | 141 | 37.235 | 47.567 | 53.907 | 1.00 | 25.11 | A | C |
| ATOM | 826 | CB | ARG | A | 141 | 38.512 | 46.776 | 53.613 | 1.00 | 25.94 | A | C |
| ATOM | 827 | CG | ARG | A | 141 | 38.915 | 45.795 | 54.712 | 1.00 | 29.78 | A | C |
| ATOM | 828 | CD | ARG | A | 141 | 40.116 | 44.954 | 54.288 | 1.00 | 33.41 | A | C |
| ATOM | 829 | NE | ARG | A | 141 | 41.349 | 45.739 | 54.224 | 1.00 | 37.25 | A | N |
| ATOM | 830 | CZ | ARG | A | 141 | 42.337 | 45.519 | 53.364 | 1.00 | 39.19 | A | C |
| ATOM | 831 | NH1 | ARG | A | 141 | 42.245 | 44.537 | 52.481 | 1.00 | 41.44 | A | N |
| ATOM | 832 | NH2 | ARG | A | 141 | 43.430 | 46.274 | 53.387 | 1.00 | 42.54 | A | N |
| ATOM | 833 | C | ARG | A | 141 | 37.081 | 48.680 | 52.874 | 1.00 | 25.17 | A | C |
| ATOM | 834 | O | ARG | A | 141 | 37.326 | 49.845 | 53.165 | 1.00 | 24.16 | A | O |
| ATOM | 835 | N | VAL | A | 142 | 36.671 | 48.310 | 51.666 | 1.00 | 24.93 | A | N |
| ATOM | 836 | CA | VAL | A | 142 | 36.486 | 49.278 | 50.602 | 1.00 | 24.76 | A | C |
| ATOM | 837 | CB | VAL | A | 142 | 36.053 | 48.585 | 49.297 | 1.00 | 24.91 | A | C |
| ATOM | 838 | CG1 | VAL | A | 142 | 35.784 | 49.618 | 48.220 | 1.00 | 23.97 | A | C |
| ATOM | 839 | CG2 | VAL | A | 142 | 37.140 | 47.636 | 48.845 | 1.00 | 25.06 | A | C |
| ATOM | 840 | C | VAL | A | 142 | 35.455 | 50.338 | 50.976 | 1.00 | 25.32 | A | C |
| ATOM | 841 | O | VAL | A | 142 | 35.702 | 51.521 | 50.819 | 1.00 | 24.96 | A | O |
| ATOM | 842 | N | ALA | A | 143 | 34.305 | 49.907 | 51.474 | 1.00 | 24.11 | A | N |
| ATOM | 843 | CA | ALA | A | 143 | 33.263 | 50.849 | 51.855 | 1.00 | 26.44 | A | C |
| ATOM | 844 | CB | ALA | A | 143 | 32.032 | 50.102 | 52.360 | 1.00 | 24.93 | A | C |
| ATOM | 845 | C | ALA | A | 143 | 33.788 | 51.783 | 52.935 | 1.00 | 27.62 | A | C |
| ATOM | 846 | O | ALA | A | 143 | 33.561 | 52.975 | 52.891 | 1.00 | 28.58 | A | O |
| ATOM | 847 | N | ARG | A | 144 | 34.498 | 51.217 | 53.903 | 1.00 | 29.19 | A | N |
| ATOM | 848 | CA | ARG | A | 144 | 35.048 | 51.995 | 54.998 | 1.00 | 32.22 | A | C |
| ATOM | 849 | CB | ARG | A | 144 | 35.762 | 51.072 | 55.981 | 1.00 | 35.02 | A | C |
| ATOM | 850 | CG | ARG | A | 144 | 36.132 | 51.737 | 57.280 | 1.00 | 38.81 | A | C |
| ATOM | 851 | CD | ARG | A | 144 | 37.215 | 50.973 | 58.000 | 1.00 | 42.99 | A | C |
| ATOM | 852 | NE | ARG | A | 144 | 37.733 | 51.743 | 59.125 | 1.00 | 46.72 | A | N |
| ATOM | 853 | CZ | ARG | A | 144 | 37.070 | 51.956 | 60.258 | 1.00 | 48.00 | A | C |
| ATOM | 854 | NH1 | ARG | A | 144 | 35.851 | 51.445 | 60.426 | 1.00 | 47.43 | A | N |
| ATOM | 855 | NH2 | ARG | A | 144 | 37.627 | 52.694 | 61.217 | 1.00 | 48.61 | A | N |
| ATOM | 856 | C | ARG | A | 144 | 36.018 | 53.057 | 54.484 | 1.00 | 33.14 | A | C |
| ATOM | 857 | O | ARG | A | 144 | 36.038 | 54.169 | 54.992 | 1.00 | 32.69 | A | O |
| ATOM | 858 | N | HIS | A | 145 | 36.824 | 52.699 | 53.481 | 1.00 | 34.01 | A | N |
| ATOM | 859 | CA | HIS | A | 145 | 37.776 | 53.638 | 52.896 | 1.00 | 35.09 | A | C |
| ATOM | 860 | CB | HIS | A | 145 | 38.562 | 52.989 | 51.746 | 1.00 | 37.71 | A | C |
| ATOM | 861 | CG | HIS | A | 145 | 39.741 | 52.179 | 52.194 | 1.00 | 41.33 | A | C |
| ATOM | 862 | CD2 | HIS | A | 145 | 40.073 | 50.887 | 51.968 | 1.00 | 42.76 | A | C |
| ATOM | 863 | ND1 | HIS | A | 145 | 40.750 | 52.705 | 52.978 | 1.00 | 42.84 | A | N |
| ATOM | 864 | CE1 | HIS | A | 145 | 41.655 | 51.758 | 53.216 | 1.00 | 43.34 | A | C |
| ATOM | 865 | NE2 | HIS | A | 145 | 41.261 | 50.650 | 52.612 | 1.00 | 43.14 | A | N |
| ATOM | 866 | C | HIS | A | 145 | 37.032 | 54.857 | 52.380 | 1.00 | 35.20 | A | C |
| ATOM | 867 | O | HIS | A | 145 | 37.360 | 55.969 | 52.718 | 1.00 | 35.56 | A | O |
| ATOM | 868 | N | TYR | A | 146 | 36.035 | 54.623 | 51.532 | 1.00 | 34.54 | A | N |
| ATOM | 869 | CA | TYR | A | 146 | 35.250 | 55.712 | 50.963 | 1.00 | 33.75 | A | C |
| ATOM | 870 | CB | TYR | A | 146 | 34.256 | 55.185 | 49.931 | 1.00 | 30.94 | A | C |
| ATOM | 871 | CG | TYR | A | 146 | 34.855 | 54.880 | 48.581 | 1.00 | 27.60 | A | C |
| ATOM | 872 | CD1 | TYR | A | 146 | 35.541 | 53.692 | 48.349 | 1.00 | 27.50 | A | C |
| ATOM | 873 | CE1 | TYR | A | 146 | 36.080 | 53.405 | 47.095 | 1.00 | 24.98 | A | C |
| ATOM | 874 | CD2 | TYR | A | 146 | 34.728 | 55.780 | 47.529 | 1.00 | 25.97 | A | C |
| ATOM | 875 | CE2 | TYR | A | 146 | 35.262 | 55.507 | 46.282 | 1.00 | 25.54 | A | C |
| ATOM | 876 | CZ | TYR | A | 146 | 35.937 | 54.321 | 46.071 | 1.00 | 24.36 | A | C |

| | | | | | | | | | | | | |
|---|---|---|---|---|---|---|---|---|---|---|---|---|
| ATOM | 877 | OH | TYR | A | 146 | 36.472 | 54.067 | 44.837 | 1.00 | 23.71 | A | O |
| ATOM | 878 | C | TYR | A | 146 | 34.487 | 56.485 | 52.027 | 1.00 | 34.86 | A | C |
| ATOM | 879 | O | TYR | A | 146 | 34.317 | 57.688 | 51.929 | 1.00 | 35.20 | A | O |
| ATOM | 880 | N | SER | A | 147 | 34.035 | 55.773 | 53.047 | 1.00 | 35.60 | A | N |
| ATOM | 881 | CA | SER | A | 147 | 33.279 | 56.386 | 54.130 | 1.00 | 37.66 | A | C |
| ATOM | 882 | CB | SER | A | 147 | 32.607 | 55.301 | 54.970 | 1.00 | 37.50 | A | C |
| ATOM | 883 | OG | SER | A | 147 | 31.762 | 55.871 | 55.949 | 1.00 | 39.07 | A | O |
| ATOM | 884 | C | SER | A | 147 | 34.189 | 57.237 | 55.008 | 1.00 | 39.51 | A | C |
| ATOM | 885 | O | SER | A | 147 | 33.781 | 58.281 | 55.525 | 1.00 | 38.73 | A | O |
| ATOM | 886 | N | ARG | A | 148 | 35.427 | 56.783 | 55.170 | 1.00 | 41.25 | A | N |
| ATOM | 887 | CA | ARG | A | 148 | 36.405 | 57.501 | 55.978 | 1.00 | 43.49 | A | C |
| ATOM | 888 | CB | ARG | A | 148 | 37.698 | 56.690 | 56.108 | 1.00 | 45.44 | A | C |
| ATOM | 889 | CG | ARG | A | 148 | 37.780 | 55.843 | 57.371 | 1.00 | 49.61 | A | C |
| ATOM | 890 | CD | ARG | A | 148 | 37.615 | 56.727 | 58.607 | 1.00 | 53.54 | A | C |
| ATOM | 891 | NE | ARG | A | 148 | 37.996 | 56.056 | 59.848 | 1.00 | 56.00 | A | N |
| ATOM | 892 | CZ | ARG | A | 148 | 39.253 | 55.800 | 60.205 | 1.00 | 57.33 | A | C |
| ATOM | 893 | NH1 | ARG | A | 148 | 40.258 | 56.161 | 59.410 | 1.00 | 57.00 | A | N |
| ATOM | 894 | NH2 | ARG | A | 148 | 39.503 | 55.194 | 61.365 | 1.00 | 58.07 | A | N |
| ATOM | 895 | C | ARG | A | 148 | 36.726 | 58.857 | 55.359 | 1.00 | 43.77 | A | C |
| ATOM | 896 | O | ARG | A | 148 | 36.967 | 59.852 | 56.080 | 1.00 | 43.40 | A | O |
| ATOM | 897 | N | ALA | A | 149 | 36.733 | 58.902 | 54.030 | 1.00 | 43.17 | A | N |
| ATOM | 898 | CA | ALA | A | 149 | 37.031 | 60.137 | 53.319 | 1.00 | 43.72 | A | C |
| ATOM | 899 | CB | ALA | A | 149 | 37.890 | 59.834 | 52.093 | 1.00 | 43.41 | A | C |
| ATOM | 900 | C | ALA | A | 149 | 35.765 | 60.890 | 52.906 | 1.00 | 44.08 | A | C |
| ATOM | 901 | O | ALA | A | 149 | 35.792 | 61.718 | 51.990 | 1.00 | 44.85 | A | O |
| ATOM | 902 | N | LYS | A | 150 | 34.658 | 60.599 | 53.578 | 1.00 | 44.43 | A | N |
| ATOM | 903 | CA | LYS | A | 150 | 33.390 | 61.259 | 53.291 | 1.00 | 45.35 | A | C |
| ATOM | 904 | CB | LYS | A | 150 | 33.451 | 62.706 | 53.801 | 1.00 | 46.87 | A | C |
| ATOM | 905 | CG | LYS | A | 150 | 33.765 | 62.778 | 55.299 | 1.00 | 48.25 | A | C |
| ATOM | 906 | CD | LYS | A | 150 | 34.014 | 64.195 | 55.803 | 1.00 | 50.09 | A | C |
| ATOM | 907 | CE | LYS | A | 150 | 32.721 | 64.998 | 55.916 | 1.00 | 51.38 | A | C |
| ATOM | 908 | NZ | LYS | A | 150 | 32.929 | 66.276 | 56.688 | 1.00 | 51.86 | A | N |
| ATOM | 909 | C | LYS | A | 150 | 32.982 | 61.222 | 51.812 | 1.00 | 45.10 | A | C |
| ATOM | 910 | O | LYS | A | 150 | 32.211 | 62.057 | 51.349 | 1.00 | 44.58 | A | O |
| ATOM | 911 | N | GLN | A | 151 | 33.503 | 60.242 | 51.081 | 1.00 | 44.60 | A | N |
| ATOM | 912 | CA | GLN | A | 151 | 33.190 | 60.082 | 49.664 | 1.00 | 44.04 | A | C |
| ATOM | 913 | CB | GLN | A | 151 | 34.451 | 59.727 | 48.879 | 1.00 | 45.13 | A | C |
| ATOM | 914 | CG | GLN | A | 151 | 35.722 | 60.357 | 49.428 | 1.00 | 46.36 | A | C |
| ATOM | 915 | CD | GLN | A | 151 | 36.944 | 59.982 | 48.614 | 1.00 | 47.77 | A | C |
| ATOM | 916 | OE1 | GLN | A | 151 | 37.165 | 60.513 | 47.522 | 1.00 | 48.90 | A | O |
| ATOM | 917 | NE2 | GLN | A | 151 | 37.740 | 59.047 | 49.136 | 1.00 | 48.82 | A | N |
| ATOM | 918 | C | GLN | A | 151 | 32.211 | 58.928 | 49.550 | 1.00 | 43.06 | A | C |
| ATOM | 919 | O | GLN | A | 151 | 31.859 | 58.300 | 50.540 | 1.00 | 43.20 | A | O |
| ATOM | 920 | N | THR | A | 152 | 31.777 | 58.645 | 48.330 | 1.00 | 41.46 | A | N |
| ATOM | 921 | CA | THR | A | 152 | 30.861 | 57.540 | 48.115 | 1.00 | 39.10 | A | C |
| ATOM | 922 | CB | THR | A | 152 | 29.423 | 58.034 | 47.877 | 1.00 | 40.15 | A | C |
| ATOM | 923 | OG1 | THR | A | 152 | 28.547 | 56.903 | 47.778 | 1.00 | 43.16 | A | O |
| ATOM | 924 | CG2 | THR | A | 152 | 29.339 | 58.852 | 46.604 | 1.00 | 41.20 | A | C |
| ATOM | 925 | C | THR | A | 152 | 31.348 | 56.706 | 46.940 | 1.00 | 35.67 | A | C |
| ATOM | 926 | O | THR | A | 152 | 31.980 | 57.223 | 46.023 | 1.00 | 34.37 | A | O |
| ATOM | 927 | N | LEU | A | 153 | 31.070 | 55.410 | 46.984 | 1.00 | 32.31 | A | N |
| ATOM | 928 | CA | LEU | A | 153 | 31.494 | 54.509 | 45.919 | 1.00 | 30.47 | A | C |
| ATOM | 929 | CB | LEU | A | 153 | 31.265 | 53.058 | 46.356 | 1.00 | 30.09 | A | C |
| ATOM | 930 | CG | LEU | A | 153 | 31.768 | 51.892 | 45.497 | 1.00 | 31.25 | A | C |
| ATOM | 931 | CD1 | LEU | A | 153 | 33.296 | 51.893 | 45.421 | 1.00 | 29.06 | A | C |
| ATOM | 932 | CD2 | LEU | A | 153 | 31.281 | 50.585 | 46.122 | 1.00 | 30.39 | A | C |
| ATOM | 933 | C | LEU | A | 153 | 30.714 | 54.804 | 44.634 | 1.00 | 28.28 | A | C |
| ATOM | 934 | O | LEU | A | 153 | 29.502 | 54.987 | 44.668 | 1.00 | 27.63 | A | O |
| ATOM | 935 | N | PRO | A | 154 | 31.412 | 54.884 | 43.490 | 1.00 | 26.06 | A | N |
| ATOM | 936 | CD | PRO | A | 154 | 32.870 | 54.805 | 43.307 | 1.00 | 25.35 | A | C |
| ATOM | 937 | CA | PRO | A | 154 | 30.732 | 55.155 | 42.220 | 1.00 | 25.69 | A | C |
| ATOM | 938 | CB | PRO | A | 154 | 31.862 | 55.073 | 41.195 | 1.00 | 25.62 | A | C |
| ATOM | 939 | CG | PRO | A | 154 | 33.066 | 55.504 | 41.985 | 1.00 | 25.69 | A | C |

| | | | | | | | | | | | |
|---|---|---|---|---|---|---|---|---|---|---|---|
| ATOM | 940 | C | PRO | A | 154 | 29.675 | 54.078 | 42.004 | 1.00 | 25.53 | A | C |
| ATOM | 941 | O | PRO | A | 154 | 29.946 | 52.916 | 42.165 | 1.00 | 24.95 | A | O |
| ATOM | 942 | N | VAL | A | 155 | 28.470 | 54.496 | 41.636 | 1.00 | 26.12 | A | N |
| ATOM | 943 | CA | VAL | A | 155 | 27.369 | 53.570 | 41.427 | 1.00 | 26.91 | A | C |
| ATOM | 944 | CB | VAL | A | 155 | 26.115 | 54.331 | 40.945 | 1.00 | 27.47 | A | C |
| ATOM | 945 | CG1 | VAL | A | 155 | 25.776 | 55.424 | 41.955 | 1.00 | 28.32 | A | C |
| ATOM | 946 | CG2 | VAL | A | 155 | 26.354 | 54.939 | 39.585 | 1.00 | 29.58 | A | C |
| ATOM | 947 | C | VAL | A | 155 | 27.692 | 52.403 | 40.493 | 1.00 | 26.27 | A | C |
| ATOM | 948 | O | VAL | A | 155 | 27.206 | 51.294 | 40.703 | 1.00 | 24.85 | A | O |
| ATOM | 949 | N | ILE | A | 156 | 28.525 | 52.640 | 39.482 | 1.00 | 25.24 | A | N |
| ATOM | 950 | CA | ILE | A | 156 | 28.878 | 51.577 | 38.553 | 1.00 | 24.58 | A | C |
| ATOM | 951 | CB | ILE | A | 156 | 29.901 | 52.063 | 37.492 | 1.00 | 24.91 | A | C |
| ATOM | 952 | CG2 | ILE | A | 156 | 31.203 | 52.451 | 38.157 | 1.00 | 27.44 | A | C |
| ATOM | 953 | CG1 | ILE | A | 156 | 30.143 | 50.965 | 36.456 | 1.00 | 24.90 | A | C |
| ATOM | 954 | CD1 | ILE | A | 156 | 28.883 | 50.504 | 35.742 | 1.00 | 23.78 | A | C |
| ATOM | 955 | C | ILE | A | 156 | 29.436 | 50.383 | 39.335 | 1.00 | 23.61 | A | C |
| ATOM | 956 | O | ILE | A | 156 | 29.171 | 49.228 | 38.996 | 1.00 | 22.96 | A | O |
| ATOM | 957 | N | TYR | A | 157 | 30.196 | 50.663 | 40.390 | 1.00 | 23.42 | A | N |
| ATOM | 958 | CA | TYR | A | 157 | 30.743 | 49.593 | 41.225 | 1.00 | 23.31 | A | C |
| ATOM | 959 | CB | TYR | A | 157 | 31.901 | 50.105 | 42.086 | 1.00 | 22.84 | A | C |
| ATOM | 960 | CG | TYR | A | 157 | 33.176 | 50.278 | 41.312 | 1.00 | 24.56 | A | C |
| ATOM | 961 | CD1 | TYR | A | 157 | 33.700 | 51.544 | 41.061 | 1.00 | 25.30 | A | C |
| ATOM | 962 | CE1 | TYR | A | 157 | 34.859 | 51.700 | 40.314 | 1.00 | 26.14 | A | C |
| ATOM | 963 | CD2 | TYR | A | 157 | 33.849 | 49.169 | 40.792 | 1.00 | 24.58 | A | C |
| ATOM | 964 | CE2 | TYR | A | 157 | 35.007 | 49.317 | 40.046 | 1.00 | 25.15 | A | C |
| ATOM | 965 | CZ | TYR | A | 157 | 35.502 | 50.586 | 39.812 | 1.00 | 26.28 | A | C |
| ATOM | 966 | OH | TYR | A | 157 | 36.647 | 50.744 | 39.081 | 1.00 | 30.67 | A | O |
| ATOM | 967 | C | TYR | A | 157 | 29.659 | 49.022 | 42.126 | 1.00 | 22.86 | A | C |
| ATOM | 968 | O | TYR | A | 157 | 29.646 | 47.842 | 42.408 | 1.00 | 23.77 | A | O |
| ATOM | 969 | N | VAL | A | 158 | 28.756 | 49.881 | 42.579 | 1.00 | 21.87 | A | N |
| ATOM | 970 | CA | VAL | A | 158 | 27.659 | 49.433 | 43.417 | 1.00 | 22.49 | A | C |
| ATOM | 971 | CB | VAL | A | 158 | 26.766 | 50.619 | 43.832 | 1.00 | 23.37 | A | C |
| ATOM | 972 | CG1 | VAL | A | 158 | 25.600 | 50.127 | 44.678 | 1.00 | 22.21 | A | C |
| ATOM | 973 | CG2 | VAL | A | 158 | 27.594 | 51.650 | 44.594 | 1.00 | 22.17 | A | C |
| ATOM | 974 | C | VAL | A | 158 | 26.840 | 48.431 | 42.598 | 1.00 | 23.15 | A | C |
| ATOM | 975 | O | VAL | A | 158 | 26.441 | 47.394 | 43.100 | 1.00 | 24.25 | A | O |
| ATOM | 976 | N | LYS | A | 159 | 26.617 | 48.761 | 41.327 | 1.00 | 21.71 | A | N |
| ATOM | 977 | CA | LYS | A | 159 | 25.860 | 47.910 | 40.416 | 1.00 | 21.45 | A | C |
| ATOM | 978 | CB | LYS | A | 159 | 25.703 | 48.593 | 39.055 | 1.00 | 21.82 | A | C |
| ATOM | 979 | CG | LYS | A | 159 | 24.717 | 49.740 | 39.040 | 1.00 | 21.87 | A | C |
| ATOM | 980 | CD | LYS | A | 159 | 24.615 | 50.388 | 37.671 | 1.00 | 21.60 | A | C |
| ATOM | 981 | CE | LYS | A | 159 | 23.659 | 51.573 | 37.702 | 1.00 | 21.37 | A | C |
| ATOM | 982 | NZ | LYS | A | 159 | 23.579 | 52.247 | 36.370 | 1.00 | 22.49 | A | N |
| ATOM | 983 | C | LYS | A | 159 | 26.537 | 46.563 | 40.229 | 1.00 | 21.63 | A | C |
| ATOM | 984 | O | LYS | A | 159 | 25.916 | 45.536 | 40.396 | 1.00 | 20.39 | A | O |
| ATOM | 985 | N | LEU | A | 160 | 27.820 | 46.587 | 39.882 | 1.00 | 21.81 | A | N |
| ATOM | 986 | CA | LEU | A | 160 | 28.580 | 45.359 | 39.671 | 1.00 | 22.68 | A | C |
| ATOM | 987 | CB | LEU | A | 160 | 30.007 | 45.683 | 39.239 | 1.00 | 20.62 | A | C |
| ATOM | 988 | CG | LEU | A | 160 | 30.235 | 46.141 | 37.806 | 1.00 | 21.92 | A | C |
| ATOM | 989 | CD1 | LEU | A | 160 | 31.664 | 46.644 | 37.660 | 1.00 | 20.65 | A | C |
| ATOM | 990 | CD2 | LEU | A | 160 | 29.967 | 44.987 | 36.852 | 1.00 | 21.55 | A | C |
| ATOM | 991 | C | LEU | A | 160 | 28.636 | 44.474 | 40.912 | 1.00 | 23.35 | A | C |
| ATOM | 992 | O | LEU | A | 160 | 28.423 | 43.273 | 40.829 | 1.00 | 24.77 | A | O |
| ATOM | 993 | N | TYR | A | 161 | 28.933 | 45.078 | 42.058 | 1.00 | 23.09 | A | N |
| ATOM | 994 | CA | TYR | A | 161 | 29.034 | 44.325 | 43.301 | 1.00 | 22.97 | A | C |
| ATOM | 995 | CB | TYR | A | 161 | 29.575 | 45.211 | 44.441 | 1.00 | 23.29 | A | C |
| ATOM | 996 | CG | TYR | A | 161 | 30.975 | 45.768 | 44.225 | 1.00 | 25.50 | A | C |
| ATOM | 997 | CD1 | TYR | A | 161 | 31.814 | 45.270 | 43.221 | 1.00 | 25.11 | A | C |
| ATOM | 998 | CE1 | TYR | A | 161 | 33.097 | 45.794 | 43.031 | 1.00 | 27.40 | A | C |
| ATOM | 999 | CD2 | TYR | A | 161 | 31.462 | 46.806 | 45.032 | 1.00 | 26.64 | A | C |
| ATOM | 1000 | CE2 | TYR | A | 161 | 32.738 | 47.331 | 44.852 | 1.00 | 24.97 | A | C |
| ATOM | 1001 | CZ | TYR | A | 161 | 33.550 | 46.827 | 43.854 | 1.00 | 27.66 | A | C |
| ATOM | 1002 | OH | TYR | A | 161 | 34.816 | 47.351 | 43.681 | 1.00 | 30.52 | A | O |

| | | | | | | | | | | | | |
|---|---|---|---|---|---|---|---|---|---|---|---|---|
| ATOM | 1003 | C | TYR | A | 161 | 27.717 | 43.699 | 43.721 | 1.00 | 21.64 | A | C |
| ATOM | 1004 | O | TYR | A | 161 | 27.676 | 42.532 | 44.051 | 1.00 | 21.38 | A | O |
| ATOM | 1005 | N | MET | A | 162 | 26.644 | 44.486 | 43.703 | 1.00 | 22.15 | A | N |
| ATOM | 1006 | CA | MET | A | 162 | 25.339 | 43.976 | 44.100 | 1.00 | 21.26 | A | C |
| ATOM | 1007 | CB | MET | A | 162 | 24.319 | 45.111 | 44.180 | 1.00 | 21.81 | A | C |
| ATOM | 1008 | CG | MET | A | 162 | 24.531 | 46.043 | 45.373 | 1.00 | 22.37 | A | C |
| ATOM | 1009 | SD | MET | A | 162 | 24.870 | 45.141 | 46.921 | 1.00 | 21.68 | A | S |
| ATOM | 1010 | CE | MET | A | 162 | 23.291 | 44.377 | 47.212 | 1.00 | 21.67 | A | C |
| ATOM | 1011 | C | MET | A | 162 | 24.850 | 42.904 | 43.145 | 1.00 | 21.23 | A | C |
| ATOM | 1012 | O | MET | A | 162 | 24.328 | 41.899 | 43.570 | 1.00 | 22.61 | A | O |
| ATOM | 1013 | N | TYR | A | 163 | 25.032 | 43.130 | 41.850 | 1.00 | 20.57 | A | N |
| ATOM | 1014 | CA | TYR | A | 163 | 24.602 | 42.167 | 40.848 | 1.00 | 20.38 | A | C |
| ATOM | 1015 | CB | TYR | A | 163 | 25.025 | 42.636 | 39.458 | 1.00 | 21.22 | A | C |
| ATOM | 1016 | CG | TYR | A | 163 | 24.622 | 41.690 | 38.350 | 1.00 | 22.32 | A | C |
| ATOM | 1017 | CD1 | TYR | A | 163 | 23.352 | 41.753 | 37.780 | 1.00 | 22.40 | A | C |
| ATOM | 1018 | CE1 | TYR | A | 163 | 22.980 | 40.877 | 36.758 | 1.00 | 25.27 | A | C |
| ATOM | 1019 | CD2 | TYR | A | 163 | 25.509 | 40.727 | 37.878 | 1.00 | 20.69 | A | C |
| ATOM | 1020 | CE2 | TYR | A | 163 | 25.149 | 39.850 | 36.863 | 1.00 | 21.54 | A | C |
| ATOM | 1021 | CZ | TYR | A | 163 | 23.883 | 39.933 | 36.306 | 1.00 | 23.36 | A | C |
| ATOM | 1022 | OH | TYR | A | 163 | 23.531 | 39.090 | 35.278 | 1.00 | 26.02 | A | O |
| ATOM | 1023 | C | TYR | A | 163 | 25.227 | 40.798 | 41.132 | 1.00 | 20.74 | A | C |
| ATOM | 1024 | O | TYR | A | 163 | 24.530 | 39.777 | 41.198 | 1.00 | 18.53 | A | O |
| ATOM | 1025 | N | GLN | A | 164 | 26.547 | 40.786 | 41.289 | 1.00 | 19.75 | A | N |
| ATOM | 1026 | CA | GLN | A | 164 | 27.260 | 39.544 | 41.568 | 1.00 | 20.34 | A | C |
| ATOM | 1027 | CB | GLN | A | 164 | 28.775 | 39.805 | 41.579 | 1.00 | 20.02 | A | C |
| ATOM | 1028 | CG | GLN | A | 164 | 29.320 | 40.266 | 40.214 | 1.00 | 20.98 | A | C |
| ATOM | 1029 | CD | GLN | A | 164 | 30.809 | 40.601 | 40.225 | 1.00 | 21.58 | A | C |
| ATOM | 1030 | OE1 | GLN | A | 164 | 31.637 | 39.726 | 40.243 | 1.00 | 21.16 | A | O |
| ATOM | 1031 | NE2 | GLN | A | 164 | 31.131 | 41.887 | 40.219 | 1.00 | 19.60 | A | N |
| ATOM | 1032 | C | GLN | A | 164 | 26.796 | 38.919 | 42.893 | 1.00 | 20.15 | A | C |
| ATOM | 1033 | O | GLN | A | 164 | 26.704 | 37.706 | 43.008 | 1.00 | 19.97 | A | O |
| ATOM | 1034 | N | LEU | A | 165 | 26.491 | 39.749 | 43.885 | 1.00 | 19.17 | A | N |
| ATOM | 1035 | CA | LEU | A | 165 | 26.009 | 39.225 | 45.158 | 1.00 | 19.84 | A | C |
| ATOM | 1036 | CB | LEU | A | 165 | 25.810 | 40.365 | 46.162 | 1.00 | 18.00 | A | C |
| ATOM | 1037 | CG | LEU | A | 165 | 25.025 | 40.047 | 47.444 | 1.00 | 21.59 | A | C |
| ATOM | 1038 | CD1 | LEU | A | 165 | 25.689 | 38.910 | 48.215 | 1.00 | 19.52 | A | C |
| ATOM | 1039 | CD2 | LEU | A | 165 | 24.931 | 41.317 | 48.304 | 1.00 | 21.48 | A | C |
| ATOM | 1040 | C | LEU | A | 165 | 24.692 | 38.467 | 44.937 | 1.00 | 19.03 | A | C |
| ATOM | 1041 | O | LEU | A | 165 | 24.524 | 37.373 | 45.415 | 1.00 | 17.83 | A | O |
| ATOM | 1042 | N | PHE | A | 166 | 23.770 | 39.066 | 44.193 | 1.00 | 18.52 | A | N |
| ATOM | 1043 | CA | PHE | A | 166 | 22.497 | 38.418 | 43.926 | 1.00 | 19.39 | A | C |
| ATOM | 1044 | CB | PHE | A | 166 | 21.546 | 39.396 | 43.242 | 1.00 | 17.62 | A | C |
| ATOM | 1045 | CG | PHE | A | 166 | 20.977 | 40.417 | 44.185 | 1.00 | 19.57 | A | C |
| ATOM | 1046 | CD1 | PHE | A | 166 | 20.256 | 40.008 | 45.303 | 1.00 | 17.71 | A | C |
| ATOM | 1047 | CD2 | PHE | A | 166 | 21.183 | 41.779 | 43.983 | 1.00 | 18.94 | A | C |
| ATOM | 1048 | CE1 | PHE | A | 166 | 19.753 | 40.931 | 46.199 | 1.00 | 17.52 | A | C |
| ATOM | 1049 | CE2 | PHE | A | 166 | 20.684 | 42.709 | 44.876 | 1.00 | 17.07 | A | C |
| ATOM | 1050 | CZ | PHE | A | 166 | 19.965 | 42.283 | 45.989 | 1.00 | 19.21 | A | C |
| ATOM | 1051 | C | PHE | A | 166 | 22.671 | 37.137 | 43.119 | 1.00 | 20.05 | A | C |
| ATOM | 1052 | O | PHE | A | 166 | 21.955 | 36.182 | 43.341 | 1.00 | 19.94 | A | O |
| ATOM | 1053 | N | ARG | A | 167 | 23.636 | 37.107 | 42.201 | 1.00 | 20.69 | A | N |
| ATOM | 1054 | CA | ARG | A | 167 | 23.865 | 35.883 | 41.437 | 1.00 | 21.34 | A | C |
| ATOM | 1055 | CB | ARG | A | 167 | 24.940 | 36.067 | 40.371 | 1.00 | 22.24 | A | C |
| ATOM | 1056 | CG | ARG | A | 167 | 24.388 | 36.204 | 38.965 | 1.00 | 24.84 | A | C |
| ATOM | 1057 | CD | ARG | A | 167 | 25.492 | 36.099 | 37.919 | 1.00 | 24.84 | A | C |
| ATOM | 1058 | NE | ARG | A | 167 | 25.798 | 34.717 | 37.564 | 1.00 | 24.78 | A | N |
| ATOM | 1059 | CZ | ARG | A | 167 | 26.712 | 34.368 | 36.666 | 1.00 | 27.41 | A | C |
| ATOM | 1060 | NH1 | ARG | A | 167 | 27.416 | 35.296 | 36.032 | 1.00 | 25.13 | A | N |
| ATOM | 1061 | NH2 | ARG | A | 167 | 26.911 | 33.090 | 36.376 | 1.00 | 29.55 | A | N |
| ATOM | 1062 | C | ARG | A | 167 | 24.284 | 34.732 | 42.351 | 1.00 | 20.49 | A | C |
| ATOM | 1063 | O | ARG | A | 167 | 23.804 | 33.610 | 42.200 | 1.00 | 20.99 | A | O |
| ATOM | 1064 | N | SER | A | 168 | 25.187 | 35.016 | 43.289 | 1.00 | 19.40 | A | N |
| ATOM | 1065 | CA | SER | A | 168 | 25.656 | 33.995 | 44.214 | 1.00 | 20.18 | A | C |

| | | | | | | | | | | | | |
|---|---|---|---|---|---|---|---|---|---|---|---|---|
| ATOM | 1066 | CB | SER | A | 168 | 26.764 | 34.544 | 45.117 | 1.00 | 19.91 | A | C |
| ATOM | 1067 | OG | SER | A | 168 | 26.290 | 35.574 | 45.956 | 1.00 | 19.31 | A | O |
| ATOM | 1068 | C | SER | A | 168 | 24.502 | 33.483 | 45.059 | 1.00 | 20.36 | A | C |
| ATOM | 1069 | O | SER | A | 168 | 24.402 | 32.296 | 45.316 | 1.00 | 21.19 | A | O |
| ATOM | 1070 | N | LEU | A | 169 | 23.631 | 34.394 | 45.477 | 1.00 | 20.43 | A | N |
| ATOM | 1071 | CA | LEU | A | 169 | 22.474 | 34.026 | 46.285 | 1.00 | 20.80 | A | C |
| ATOM | 1072 | CB | LEU | A | 169 | 21.767 | 35.283 | 46.783 | 1.00 | 21.14 | A | C |
| ATOM | 1073 | CG | LEU | A | 169 | 22.111 | 35.787 | 48.191 | 1.00 | 22.65 | A | C |
| ATOM | 1074 | CD1 | LEU | A | 169 | 23.570 | 35.553 | 48.531 | 1.00 | 21.25 | A | C |
| ATOM | 1075 | CD2 | LEU | A | 169 | 21.755 | 37.256 | 48.273 | 1.00 | 19.59 | A | C |
| ATOM | 1076 | C | LEU | A | 169 | 21.512 | 33.153 | 45.481 | 1.00 | 20.88 | A | C |
| ATOM | 1077 | O | LEU | A | 169 | 21.018 | 32.136 | 45.985 | 1.00 | 20.39 | A | O |
| ATOM | 1078 | N | ALA | A | 170 | 21.252 | 33.550 | 44.236 | 1.00 | 20.69 | A | N |
| ATOM | 1079 | CA | ALA | A | 170 | 20.364 | 32.788 | 43.368 | 1.00 | 20.55 | A | C |
| ATOM | 1080 | CB | ALA | A | 170 | 20.237 | 33.469 | 42.010 | 1.00 | 19.38 | A | C |
| ATOM | 1081 | C | ALA | A | 170 | 20.937 | 31.385 | 43.207 | 1.00 | 20.45 | A | C |
| ATOM | 1082 | O | ALA | A | 170 | 20.208 | 30.418 | 43.165 | 1.00 | 21.67 | A | O |
| ATOM | 1083 | N | TYR | A | 171 | 22.259 | 31.287 | 43.143 | 1.00 | 20.49 | A | N |
| ATOM | 1084 | CA | TYR | A | 171 | 22.896 | 29.991 | 42.995 | 1.00 | 22.64 | A | C |
| ATOM | 1085 | CB | TYR | A | 171 | 24.386 | 30.154 | 42.679 | 1.00 | 22.36 | A | C |
| ATOM | 1086 | CG | TYR | A | 171 | 25.127 | 28.838 | 42.574 | 1.00 | 22.82 | A | C |
| ATOM | 1087 | CD1 | TYR | A | 171 | 24.903 | 27.963 | 41.507 | 1.00 | 22.58 | A | C |
| ATOM | 1088 | CE1 | TYR | A | 171 | 25.581 | 26.740 | 41.420 | 1.00 | 22.06 | A | C |
| ATOM | 1089 | CD2 | TYR | A | 171 | 26.045 | 28.458 | 43.552 | 1.00 | 24.15 | A | C |
| ATOM | 1090 | CE2 | TYR | A | 171 | 26.728 | 27.242 | 43.475 | 1.00 | 24.83 | A | C |
| ATOM | 1091 | CZ | TYR | A | 171 | 26.492 | 26.390 | 42.408 | 1.00 | 24.45 | A | C |
| ATOM | 1092 | OH | TYR | A | 171 | 27.183 | 25.202 | 42.336 | 1.00 | 26.64 | A | O |
| ATOM | 1093 | C | TYR | A | 171 | 22.738 | 29.107 | 44.231 | 1.00 | 22.57 | A | C |
| ATOM | 1094 | O | TYR | A | 171 | 22.331 | 27.975 | 44.111 | 1.00 | 23.58 | A | O |
| ATOM | 1095 | N | ILE | A | 172 | 23.063 | 29.629 | 45.412 | 1.00 | 22.43 | A | N |
| ATOM | 1096 | CA | ILE | A | 172 | 22.944 | 28.825 | 46.627 | 1.00 | 22.47 | A | C |
| ATOM | 1097 | CB | ILE | A | 172 | 23.684 | 29.473 | 47.842 | 1.00 | 21.02 | A | C |
| ATOM | 1098 | CG2 | ILE | A | 172 | 25.162 | 29.626 | 47.525 | 1.00 | 20.11 | A | C |
| ATOM | 1099 | CG1 | ILE | A | 172 | 23.079 | 30.832 | 48.193 | 1.00 | 21.53 | A | C |
| ATOM | 1100 | CD1 | ILE | A | 172 | 23.607 | 31.399 | 49.509 | 1.00 | 19.74 | A | C |
| ATOM | 1101 | C | ILE | A | 172 | 21.493 | 28.554 | 46.996 | 1.00 | 22.06 | A | C |
| ATOM | 1102 | O | ILE | A | 172 | 21.186 | 27.506 | 47.491 | 1.00 | 24.17 | A | O |
| ATOM | 1103 | N | HIS | A | 173 | 20.612 | 29.514 | 46.736 | 1.00 | 23.39 | A | N |
| ATOM | 1104 | CA | HIS | A | 173 | 19.192 | 29.353 | 47.029 | 1.00 | 23.80 | A | C |
| ATOM | 1105 | CB | HIS | A | 173 | 18.447 | 30.680 | 46.817 | 1.00 | 24.00 | A | C |
| ATOM | 1106 | CG | HIS | A | 173 | 18.693 | 31.704 | 47.889 | 1.00 | 25.06 | A | C |
| ATOM | 1107 | CD2 | HIS | A | 173 | 19.483 | 31.678 | 48.987 | 1.00 | 23.88 | A | C |
| ATOM | 1108 | ND1 | HIS | A | 173 | 18.086 | 32.944 | 47.881 | 1.00 | 24.03 | A | N |
| ATOM | 1109 | CE1 | HIS | A | 173 | 18.496 | 33.635 | 48.930 | 1.00 | 23.46 | A | C |
| ATOM | 1110 | NE2 | HIS | A | 173 | 19.343 | 32.892 | 49.616 | 1.00 | 20.63 | A | N |
| ATOM | 1111 | C | HIS | A | 173 | 18.567 | 28.269 | 46.143 | 1.00 | 24.69 | A | C |
| ATOM | 1112 | O | HIS | A | 173 | 17.658 | 27.588 | 46.563 | 1.00 | 24.19 | A | O |
| ATOM | 1113 | N | SER | A | 174 | 19.059 | 28.121 | 44.915 | 1.00 | 25.26 | A | N |
| ATOM | 1114 | CA | SER | A | 174 | 18.510 | 27.113 | 44.005 | 1.00 | 26.41 | A | C |
| ATOM | 1115 | CB | SER | A | 174 | 19.149 | 27.215 | 42.616 | 1.00 | 25.59 | A | C |
| ATOM | 1116 | OG | SER | A | 174 | 20.384 | 26.523 | 42.562 | 1.00 | 26.10 | A | O |
| ATOM | 1117 | C | SER | A | 174 | 18.759 | 25.719 | 44.579 | 1.00 | 27.90 | A | C |
| ATOM | 1118 | O | SER | A | 174 | 18.145 | 24.750 | 44.149 | 1.00 | 28.29 | A | O |
| ATOM | 1119 | N | PHE | A | 175 | 19.675 | 25.633 | 45.544 | 1.00 | 27.17 | A | N |
| ATOM | 1120 | CA | PHE | A | 175 | 19.987 | 24.367 | 46.211 | 1.00 | 27.47 | A | C |
| ATOM | 1121 | CB | PHE | A | 175 | 21.489 | 24.262 | 46.491 | 1.00 | 28.29 | A | C |
| ATOM | 1122 | CG | PHE | A | 175 | 22.322 | 23.882 | 45.299 | 1.00 | 31.85 | A | C |
| ATOM | 1123 | CD1 | PHE | A | 175 | 22.165 | 22.642 | 44.690 | 1.00 | 32.08 | A | C |
| ATOM | 1124 | CD2 | PHE | A | 175 | 23.309 | 24.744 | 44.821 | 1.00 | 32.21 | A | C |
| ATOM | 1125 | CE1 | PHE | A | 175 | 22.980 | 22.257 | 43.624 | 1.00 | 32.88 | A | C |
| ATOM | 1126 | CE2 | PHE | A | 175 | 24.132 | 24.369 | 43.753 | 1.00 | 34.33 | A | C |
| ATOM | 1127 | CZ | PHE | A | 175 | 23.967 | 23.121 | 43.154 | 1.00 | 33.80 | A | C |
| ATOM | 1128 | C | PHE | A | 175 | 19.248 | 24.321 | 47.553 | 1.00 | 26.78 | A | C |

| | | | | | | | | | | | | |
|---|---|---|---|---|---|---|---|---|---|---|---|---|
| ATOM | 1129 | O | PHE | A | 175 | 19.392 | 23.359 | 48.329 | 1.00 | 25.52 | A | O |
| ATOM | 1130 | N | GLY | A | 176 | 18.478 | 25.370 | 47.828 | 1.00 | 25.08 | A | N |
| ATOM | 1131 | CA | GLY | A | 176 | 17.744 | 25.463 | 49.079 | 1.00 | 25.69 | A | C |
| ATOM | 1132 | C | GLY | A | 176 | 18.611 | 26.006 | 50.208 | 1.00 | 25.83 | A | C |
| ATOM | 1133 | O | GLY | A | 176 | 18.138 | 26.199 | 51.322 | 1.00 | 27.19 | A | O |
| ATOM | 1134 | N | ILE | A | 177 | 19.882 | 26.266 | 49.913 | 1.00 | 23.32 | A | N |
| ATOM | 1135 | CA | ILE | A | 177 | 20.814 | 26.770 | 50.916 | 1.00 | 23.33 | A | C |
| ATOM | 1136 | CB | ILE | A | 177 | 22.273 | 26.450 | 50.537 | 1.00 | 23.29 | A | C |
| ATOM | 1137 | CG2 | ILE | A | 177 | 23.210 | 26.946 | 51.637 | 1.00 | 25.07 | A | C |
| ATOM | 1138 | CG1 | ILE | A | 177 | 22.447 | 24.943 | 50.338 | 1.00 | 22.67 | A | C |
| ATOM | 1139 | CD1 | ILE | A | 177 | 23.726 | 24.570 | 49.619 | 1.00 | 21.61 | A | C |
| ATOM | 1140 | C | ILE | A | 177 | 20.731 | 28.281 | 51.170 | 1.00 | 22.53 | A | C |
| ATOM | 1141 | O | ILE | A | 177 | 20.777 | 29.080 | 50.248 | 1.00 | 21.69 | A | O |
| ATOM | 1142 | N | CYS | A | 178 | 20.615 | 28.650 | 52.442 | 1.00 | 20.86 | A | N |
| ATOM | 1143 | CA | CYS | A | 178 | 20.554 | 30.050 | 52.837 | 1.00 | 18.66 | A | C |
| ATOM | 1144 | CB | CYS | A | 178 | 19.393 | 30.279 | 53.809 | 1.00 | 17.32 | A | C |
| ATOM | 1145 | SG | CYS | A | 178 | 19.100 | 32.001 | 54.290 | 1.00 | 19.61 | A | S |
| ATOM | 1146 | C | CYS | A | 178 | 21.877 | 30.365 | 53.519 | 1.00 | 18.25 | A | C |
| ATOM | 1147 | O | CYS | A | 178 | 22.362 | 29.579 | 54.315 | 1.00 | 19.71 | A | O |
| ATOM | 1148 | N | HIS | A | 179 | 22.463 | 31.510 | 53.189 | 1.00 | 18.16 | A | N |
| ATOM | 1149 | CA | HIS | A | 179 | 23.739 | 31.913 | 53.778 | 1.00 | 17.78 | A | C |
| ATOM | 1150 | CB | HIS | A | 179 | 24.333 | 33.076 | 52.973 | 1.00 | 16.49 | A | C |
| ATOM | 1151 | CG | HIS | A | 179 | 25.766 | 33.370 | 53.294 | 1.00 | 14.10 | A | C |
| ATOM | 1152 | CD2 | HIS | A | 179 | 26.910 | 32.982 | 52.681 | 1.00 | 13.02 | A | C |
| ATOM | 1153 | ND1 | HIS | A | 179 | 26.147 | 34.138 | 54.369 | 1.00 | 13.56 | A | N |
| ATOM | 1154 | CE1 | HIS | A | 179 | 27.464 | 34.215 | 54.408 | 1.00 | 13.07 | A | C |
| ATOM | 1155 | NE2 | HIS | A | 179 | 27.950 | 33.520 | 53.393 | 1.00 | 13.96 | A | N |
| ATOM | 1156 | C | HIS | A | 179 | 23.539 | 32.308 | 55.251 | 1.00 | 19.49 | A | C |
| ATOM | 1157 | O | HIS | A | 179 | 24.254 | 31.834 | 56.132 | 1.00 | 19.19 | A | O |
| ATOM | 1158 | N | ARG | A | 180 | 22.557 | 33.171 | 55.495 | 1.00 | 18.62 | A | N |
| ATOM | 1159 | CA | ARG | A | 180 | 22.208 | 33.634 | 56.837 | 1.00 | 21.68 | A | C |
| ATOM | 1160 | CB | ARG | A | 180 | 22.085 | 32.468 | 57.807 | 1.00 | 21.18 | A | C |
| ATOM | 1161 | CG | ARG | A | 180 | 21.112 | 31.410 | 57.442 | 1.00 | 21.12 | A | C |
| ATOM | 1162 | CD | ARG | A | 180 | 21.516 | 30.195 | 58.228 | 1.00 | 22.76 | A | C |
| ATOM | 1163 | NE | ARG | A | 180 | 20.494 | 29.733 | 59.144 | 1.00 | 21.72 | A | N |
| ATOM | 1164 | CZ | ARG | A | 180 | 20.715 | 28.843 | 60.103 | 1.00 | 19.88 | A | C |
| ATOM | 1165 | NH1 | ARG | A | 180 | 21.929 | 28.330 | 60.272 | 1.00 | 15.18 | A | N |
| ATOM | 1166 | NH2 | ARG | A | 180 | 19.713 | 28.453 | 60.874 | 1.00 | 19.13 | A | N |
| ATOM | 1167 | C | ARG | A | 180 | 23.133 | 34.651 | 57.484 | 1.00 | 20.91 | A | C |
| ATOM | 1168 | O | ARG | A | 180 | 22.864 | 35.087 | 58.575 | 1.00 | 24.57 | A | O |
| ATOM | 1169 | N | ASP | A | 181 | 24.229 | 35.010 | 56.834 | 1.00 | 20.35 | A | N |
| ATOM | 1170 | CA | ASP | A | 181 | 25.125 | 35.982 | 57.438 | 1.00 | 19.99 | A | C |
| ATOM | 1171 | CB | ASP | A | 181 | 26.216 | 35.273 | 58.252 | 1.00 | 19.51 | A | C |
| ATOM | 1172 | CG | ASP | A | 181 | 26.906 | 36.204 | 59.240 | 1.00 | 20.71 | A | C |
| ATOM | 1173 | OD1 | ASP | A | 181 | 26.374 | 37.295 | 59.521 | 1.00 | 18.47 | A | O |
| ATOM | 1174 | OD2 | ASP | A | 181 | 27.978 | 35.836 | 59.750 | 1.00 | 21.37 | A | O |
| ATOM | 1175 | C | ASP | A | 181 | 25.733 | 36.905 | 56.398 | 1.00 | 18.59 | A | C |
| ATOM | 1176 | O | ASP | A | 181 | 26.909 | 37.167 | 56.407 | 1.00 | 19.22 | A | O |
| ATOM | 1177 | N | ILE | A | 182 | 24.884 | 37.392 | 55.502 | 1.00 | 17.64 | A | N |
| ATOM | 1178 | CA | ILE | A | 182 | 25.301 | 38.304 | 54.454 | 1.00 | 16.25 | A | C |
| ATOM | 1179 | CB | ILE | A | 182 | 24.186 | 38.484 | 53.397 | 1.00 | 15.61 | A | C |
| ATOM | 1180 | CG2 | ILE | A | 182 | 24.597 | 39.534 | 52.369 | 1.00 | 14.67 | A | C |
| ATOM | 1181 | CG1 | ILE | A | 182 | 23.888 | 37.139 | 52.725 | 1.00 | 14.46 | A | C |
| ATOM | 1182 | CD1 | ILE | A | 182 | 25.063 | 36.534 | 51.993 | 1.00 | 13.03 | A | C |
| ATOM | 1183 | C | ILE | A | 182 | 25.611 | 39.652 | 55.096 | 1.00 | 17.44 | A | C |
| ATOM | 1184 | O | ILE | A | 182 | 24.785 | 40.230 | 55.779 | 1.00 | 17.43 | A | O |
| ATOM | 1185 | N | LYS | A | 183 | 26.829 | 40.123 | 54.873 | 1.00 | 17.35 | A | N |
| ATOM | 1186 | CA | LYS | A | 183 | 27.284 | 41.392 | 55.399 | 1.00 | 18.18 | A | C |
| ATOM | 1187 | CB | LYS | A | 183 | 27.552 | 41.282 | 56.903 | 1.00 | 18.75 | A | C |
| ATOM | 1188 | CG | LYS | A | 183 | 28.676 | 40.331 | 57.270 | 1.00 | 17.72 | A | C |
| ATOM | 1189 | CD | LYS | A | 183 | 28.802 | 40.217 | 58.776 | 1.00 | 18.58 | A | C |
| ATOM | 1190 | CE | LYS | A | 183 | 29.845 | 39.206 | 59.165 | 1.00 | 19.80 | A | C |
| ATOM | 1191 | NZ | LYS | A | 183 | 30.013 | 39.171 | 60.631 | 1.00 | 22.26 | A | N |

| | | | | | | | | | | | | |
|---|---|---|---|---|---|---|---|---|---|---|---|---|
| ATOM | 1192 | C | LYS | A | 183 | 28.573 | 41.758 | 54.670 | 1.00 | 18.52 | A | C |
| ATOM | 1193 | O | LYS | A | 183 | 29.251 | 40.902 | 54.132 | 1.00 | 19.57 | A | O |
| ATOM | 1194 | N | PRO | A | 184 | 28.920 | 43.050 | 54.658 | 1.00 | 18.88 | A | N |
| ATOM | 1195 | CD | PRO | A | 184 | 28.172 | 44.143 | 55.308 | 1.00 | 16.59 | A | C |
| ATOM | 1196 | CA | PRO | A | 184 | 30.126 | 43.561 | 53.998 | 1.00 | 19.64 | A | C |
| ATOM | 1197 | CB | PRO | A | 184 | 30.231 | 44.983 | 54.537 | 1.00 | 19.10 | A | C |
| ATOM | 1198 | CG | PRO | A | 184 | 28.783 | 45.374 | 54.677 | 1.00 | 19.96 | A | C |
| ATOM | 1199 | C | PRO | A | 184 | 31.397 | 42.738 | 54.254 | 1.00 | 18.96 | A | C |
| ATOM | 1200 | O | PRO | A | 184 | 32.149 | 42.478 | 53.339 | 1.00 | 18.11 | A | O |
| ATOM | 1201 | N | GLN | A | 185 | 31.628 | 42.323 | 55.494 | 1.00 | 20.04 | A | N |
| ATOM | 1202 | CA | GLN | A | 185 | 32.827 | 41.540 | 55.790 | 1.00 | 22.09 | A | C |
| ATOM | 1203 | CB | GLN | A | 185 | 33.005 | 41.329 | 57.294 | 1.00 | 23.09 | A | C |
| ATOM | 1204 | CG | GLN | A | 185 | 33.208 | 42.586 | 58.086 | 1.00 | 27.04 | A | C |
| ATOM | 1205 | CD | GLN | A | 185 | 32.116 | 42.757 | 59.114 | 1.00 | 32.28 | A | C |
| ATOM | 1206 | OE1 | GLN | A | 185 | 30.953 | 43.150 | 58.785 | 1.00 | 32.89 | A | O |
| ATOM | 1207 | NE2 | GLN | A | 185 | 32.443 | 42.440 | 60.363 | 1.00 | 31.85 | A | N |
| ATOM | 1208 | C | GLN | A | 185 | 32.864 | 40.178 | 55.123 | 1.00 | 19.62 | A | C |
| ATOM | 1209 | O | GLN | A | 185 | 33.899 | 39.602 | 55.021 | 1.00 | 19.09 | A | O |
| ATOM | 1210 | N | ASN | A | 186 | 31.718 | 39.666 | 54.690 | 1.00 | 18.14 | A | N |
| ATOM | 1211 | CA | ASN | A | 186 | 31.713 | 38.365 | 54.041 | 1.00 | 19.36 | A | C |
| ATOM | 1212 | CB | ASN | A | 186 | 30.567 | 37.500 | 54.575 | 1.00 | 19.07 | A | C |
| ATOM | 1213 | CG | ASN | A | 186 | 30.793 | 37.060 | 56.011 | 1.00 | 21.43 | A | C |
| ATOM | 1214 | OD1 | ASN | A | 186 | 31.923 | 36.925 | 56.446 | 1.00 | 19.90 | A | O |
| ATOM | 1215 | ND2 | ASN | A | 186 | 29.708 | 36.828 | 56.745 | 1.00 | 18.80 | A | N |
| ATOM | 1216 | C | ASN | A | 186 | 31.629 | 38.468 | 52.529 | 1.00 | 18.17 | A | C |
| ATOM | 1217 | O | ASN | A | 186 | 31.339 | 37.497 | 51.870 | 1.00 | 18.03 | A | O |
| ATOM | 1218 | N | LEU | A | 187 | 31.892 | 39.662 | 52.006 | 1.00 | 18.75 | A | N |
| ATOM | 1219 | CA | LEU | A | 187 | 31.851 | 39.916 | 50.569 | 1.00 | 19.90 | A | C |
| ATOM | 1220 | CB | LEU | A | 187 | 30.846 | 41.021 | 50.258 | 1.00 | 18.13 | A | C |
| ATOM | 1221 | CG | LEU | A | 187 | 29.421 | 40.794 | 50.753 | 1.00 | 18.67 | A | C |
| ATOM | 1222 | CD1 | LEU | A | 187 | 28.574 | 42.018 | 50.420 | 1.00 | 16.49 | A | C |
| ATOM | 1223 | CD2 | LEU | A | 187 | 28.839 | 39.532 | 50.114 | 1.00 | 19.21 | A | C |
| ATOM | 1224 | C | LEU | A | 187 | 33.235 | 40.325 | 50.077 | 1.00 | 22.55 | A | C |
| ATOM | 1225 | O | LEU | A | 187 | 33.655 | 41.474 | 50.247 | 1.00 | 22.40 | A | O |
| ATOM | 1226 | N | LEU | A | 188 | 33.939 | 39.374 | 49.469 | 1.00 | 23.91 | A | N |
| ATOM | 1227 | CA | LEU | A | 188 | 35.288 | 39.611 | 48.958 | 1.00 | 24.48 | A | C |
| ATOM | 1228 | CB | LEU | A | 188 | 36.074 | 38.295 | 48.934 | 1.00 | 24.77 | A | C |
| ATOM | 1229 | CG | LEU | A | 188 | 36.140 | 37.455 | 50.217 | 1.00 | 23.60 | A | C |
| ATOM | 1230 | CD1 | LEU | A | 188 | 36.712 | 36.091 | 49.892 | 1.00 | 23.69 | A | C |
| ATOM | 1231 | CD2 | LEU | A | 188 | 36.996 | 38.156 | 51.267 | 1.00 | 25.69 | A | C |
| ATOM | 1232 | C | LEU | A | 188 | 35.244 | 40.202 | 47.554 | 1.00 | 26.02 | A | C |
| ATOM | 1233 | O | LEU | A | 188 | 34.322 | 39.934 | 46.797 | 1.00 | 25.57 | A | O |
| ATOM | 1234 | N | LEU | A | 189 | 36.242 | 41.009 | 47.208 | 1.00 | 27.43 | A | N |
| ATOM | 1235 | CA | LEU | A | 189 | 36.264 | 41.602 | 45.880 | 1.00 | 30.16 | A | C |
| ATOM | 1236 | CB | LEU | A | 189 | 35.383 | 42.862 | 45.847 | 1.00 | 32.37 | A | C |
| ATOM | 1237 | CG | LEU | A | 189 | 35.883 | 44.205 | 46.392 | 1.00 | 33.47 | A | C |
| ATOM | 1238 | CD1 | LEU | A | 189 | 34.688 | 45.104 | 46.711 | 1.00 | 33.49 | A | C |
| ATOM | 1239 | CD2 | LEU | A | 189 | 36.694 | 43.996 | 47.644 | 1.00 | 35.85 | A | C |
| ATOM | 1240 | C | LEU | A | 189 | 37.660 | 41.924 | 45.370 | 1.00 | 31.24 | A | C |
| ATOM | 1241 | O | LEU | A | 189 | 38.540 | 42.352 | 46.118 | 1.00 | 29.45 | A | O |
| ATOM | 1242 | N | ASP | A | 190 | 37.856 | 41.687 | 44.078 | 1.00 | 33.90 | A | N |
| ATOM | 1243 | CA | ASP | A | 190 | 39.129 | 41.969 | 43.438 | 1.00 | 36.18 | A | C |
| ATOM | 1244 | CB | ASP | A | 190 | 39.398 | 40.965 | 42.319 | 1.00 | 37.22 | A | C |
| ATOM | 1245 | CG | ASP | A | 190 | 40.747 | 41.180 | 41.655 | 1.00 | 38.85 | A | C |
| ATOM | 1246 | OD1 | ASP | A | 190 | 40.926 | 42.217 | 40.974 | 1.00 | 39.12 | A | O |
| ATOM | 1247 | OD2 | ASP | A | 190 | 41.633 | 40.314 | 41.827 | 1.00 | 38.62 | A | O |
| ATOM | 1248 | C | ASP | A | 190 | 39.000 | 43.382 | 42.878 | 1.00 | 37.61 | A | C |
| ATOM | 1249 | O | ASP | A | 190 | 38.185 | 43.641 | 42.023 | 1.00 | 37.68 | A | O |
| ATOM | 1250 | N | PRO | A | 191 | 39.808 | 44.315 | 43.382 | 1.00 | 39.88 | A | N |
| ATOM | 1251 | CD | PRO | A | 191 | 40.912 | 44.094 | 44.333 | 1.00 | 40.38 | A | C |
| ATOM | 1252 | CA | PRO | A | 191 | 39.771 | 45.711 | 42.925 | 1.00 | 40.90 | A | C |
| ATOM | 1253 | CB | PRO | A | 191 | 40.803 | 46.391 | 43.825 | 1.00 | 42.25 | A | C |
| ATOM | 1254 | CG | PRO | A | 191 | 41.820 | 45.284 | 44.045 | 1.00 | 42.29 | A | C |

| | | | | | | | | | | | |
|---|---|---|---|---|---|---|---|---|---|---|---|
| ATOM | 1255 | C | PRO | A | 191 | 40.068 | 45.932 | 41.444 | 1.00 | 41.61 | A | C |
| ATOM | 1256 | O | PRO | A | 191 | 39.553 | 46.883 | 40.837 | 1.00 | 41.28 | A | O |
| ATOM | 1257 | N | ASP | A | 192 | 40.882 | 45.059 | 40.858 | 1.00 | 41.57 | A | N |
| ATOM | 1258 | CA | ASP | A | 192 | 41.232 | 45.202 | 39.452 | 1.00 | 41.17 | A | C |
| ATOM | 1259 | CB | ASP | A | 192 | 42.606 | 44.587 | 39.186 | 1.00 | 43.68 | A | C |
| ATOM | 1260 | CG | ASP | A | 192 | 43.722 | 45.340 | 39.895 | 1.00 | 46.09 | A | C |
| ATOM | 1261 | OD1 | ASP | A | 192 | 43.684 | 46.597 | 39.895 | 1.00 | 46.26 | A | O |
| ATOM | 1262 | OD2 | ASP | A | 192 | 44.639 | 44.680 | 40.443 | 1.00 | 47.63 | A | O |
| ATOM | 1263 | C | ASP | A | 192 | 40.213 | 44.638 | 38.471 | 1.00 | 40.76 | A | C |
| ATOM | 1264 | O | ASP | A | 192 | 39.939 | 45.263 | 37.464 | 1.00 | 42.95 | A | O |
| ATOM | 1265 | N | THR | A | 193 | 39.654 | 43.465 | 38.762 | 1.00 | 37.99 | A | N |
| ATOM | 1266 | CA | THR | A | 193 | 38.682 | 42.847 | 37.869 | 1.00 | 34.58 | A | C |
| ATOM | 1267 | CB | THR | A | 193 | 38.890 | 41.320 | 37.812 | 1.00 | 35.35 | A | C |
| ATOM | 1268 | OG1 | THR | A | 193 | 38.874 | 40.774 | 39.138 | 1.00 | 36.63 | A | O |
| ATOM | 1269 | CG2 | THR | A | 193 | 40.219 | 40.999 | 37.163 | 1.00 | 34.98 | A | C |
| ATOM | 1270 | C | THR | A | 193 | 37.232 | 43.152 | 38.267 | 1.00 | 32.98 | A | C |
| ATOM | 1271 | O | THR | A | 193 | 36.303 | 42.863 | 37.533 | 1.00 | 32.69 | A | O |
| ATOM | 1272 | N | ALA | A | 194 | 37.053 | 43.742 | 39.440 | 1.00 | 31.61 | A | N |
| ATOM | 1273 | CA | ALA | A | 194 | 35.724 | 44.080 | 39.922 | 1.00 | 29.36 | A | C |
| ATOM | 1274 | CB | ALA | A | 194 | 35.035 | 45.028 | 38.933 | 1.00 | 29.52 | A | C |
| ATOM | 1275 | C | ALA | A | 194 | 34.857 | 42.850 | 40.181 | 1.00 | 28.98 | A | C |
| ATOM | 1276 | O | ALA | A | 194 | 33.640 | 42.949 | 40.199 | 1.00 | 29.30 | A | O |
| ATOM | 1277 | N | VAL | A | 195 | 35.483 | 41.689 | 40.381 | 1.00 | 28.53 | A | N |
| ATOM | 1278 | CA | VAL | A | 195 | 34.716 | 40.478 | 40.657 | 1.00 | 28.65 | A | C |
| ATOM | 1279 | CB | VAL | A | 195 | 35.410 | 39.187 | 40.115 | 1.00 | 29.89 | A | C |
| ATOM | 1280 | CG1 | VAL | A | 195 | 35.888 | 39.413 | 38.685 | 1.00 | 30.51 | A | C |
| ATOM | 1281 | CG2 | VAL | A | 195 | 36.561 | 38.769 | 41.011 | 1.00 | 32.97 | A | C |
| ATOM | 1282 | C | VAL | A | 195 | 34.477 | 40.343 | 42.162 | 1.00 | 28.37 | A | C |
| ATOM | 1283 | O | VAL | A | 195 | 35.361 | 40.646 | 42.983 | 1.00 | 29.07 | A | O |
| ATOM | 1284 | N | LEU | A | 196 | 33.274 | 39.912 | 42.522 | 1.00 | 25.60 | A | N |
| ATOM | 1285 | CA | LEU | A | 196 | 32.915 | 39.744 | 43.917 | 1.00 | 23.53 | A | C |
| ATOM | 1286 | CB | LEU | A | 196 | 31.665 | 40.570 | 44.238 | 1.00 | 22.71 | A | C |
| ATOM | 1287 | CG | LEU | A | 196 | 31.114 | 40.550 | 45.670 | 1.00 | 21.35 | A | C |
| ATOM | 1288 | CD1 | LEU | A | 196 | 30.375 | 41.846 | 45.944 | 1.00 | 22.02 | A | C |
| ATOM | 1289 | CD2 | LEU | A | 196 | 30.201 | 39.355 | 45.867 | 1.00 | 19.59 | A | C |
| ATOM | 1290 | C | LEU | A | 196 | 32.686 | 38.279 | 44.254 | 1.00 | 23.99 | A | C |
| ATOM | 1291 | O | LEU | A | 196 | 32.112 | 37.553 | 43.484 | 1.00 | 25.08 | A | O |
| ATOM | 1292 | N | LYS | A | 197 | 33.165 | 37.855 | 45.416 | 1.00 | 22.64 | A | N |
| ATOM | 1293 | CA | LYS | A | 197 | 32.984 | 36.475 | 45.838 | 1.00 | 23.00 | A | C |
| ATOM | 1294 | CB | LYS | A | 197 | 34.302 | 35.699 | 45.746 | 1.00 | 23.64 | A | C |
| ATOM | 1295 | CG | LYS | A | 197 | 34.755 | 35.466 | 44.313 | 1.00 | 26.17 | A | C |
| ATOM | 1296 | CD | LYS | A | 197 | 36.154 | 34.887 | 44.242 | 1.00 | 28.36 | A | C |
| ATOM | 1297 | CE | LYS | A | 197 | 36.573 | 34.645 | 42.802 | 1.00 | 28.98 | A | C |
| ATOM | 1298 | NZ | LYS | A | 197 | 37.889 | 33.947 | 42.751 | 1.00 | 31.14 | A | N |
| ATOM | 1299 | C | LYS | A | 197 | 32.443 | 36.412 | 47.253 | 1.00 | 22.28 | A | C |
| ATOM | 1300 | O | LYS | A | 197 | 32.912 | 37.108 | 48.146 | 1.00 | 20.40 | A | O |
| ATOM | 1301 | N | LEU | A | 198 | 31.432 | 35.570 | 47.426 | 1.00 | 21.91 | A | N |
| ATOM | 1302 | CA | LEU | A | 198 | 30.783 | 35.365 | 48.710 | 1.00 | 22.46 | A | C |
| ATOM | 1303 | CB | LEU | A | 198 | 29.413 | 34.724 | 48.495 | 1.00 | 23.95 | A | C |
| ATOM | 1304 | CG | LEU | A | 198 | 28.198 | 35.273 | 49.237 | 1.00 | 25.55 | A | C |
| ATOM | 1305 | CD1 | LEU | A | 198 | 27.048 | 34.289 | 49.065 | 1.00 | 23.19 | A | C |
| ATOM | 1306 | CD2 | LEU | A | 198 | 28.515 | 35.472 | 50.716 | 1.00 | 25.66 | A | C |
| ATOM | 1307 | C | LEU | A | 198 | 31.664 | 34.406 | 49.503 | 1.00 | 22.38 | A | C |
| ATOM | 1308 | O | LEU | A | 198 | 32.150 | 33.447 | 48.954 | 1.00 | 23.29 | A | O |
| ATOM | 1309 | N | CYS | A | 199 | 31.881 | 34.684 | 50.785 | 1.00 | 21.55 | A | N |
| ATOM | 1310 | CA | CYS | A | 199 | 32.690 | 33.799 | 51.617 | 1.00 | 20.14 | A | C |
| ATOM | 1311 | CB | CYS | A | 199 | 34.099 | 34.368 | 51.843 | 1.00 | 19.78 | A | C |
| ATOM | 1312 | SG | CYS | A | 199 | 34.226 | 35.759 | 53.015 | 1.00 | 19.52 | A | S |
| ATOM | 1313 | C | CYS | A | 199 | 32.003 | 33.607 | 52.962 | 1.00 | 21.14 | A | C |
| ATOM | 1314 | O | CYS | A | 199 | 30.941 | 34.164 | 53.204 | 1.00 | 20.27 | A | O |
| ATOM | 1315 | N | ASP | A | 200 | 32.639 | 32.815 | 53.821 | 1.00 | 20.99 | A | N |
| ATOM | 1316 | CA | ASP | A | 200 | 32.152 | 32.485 | 55.161 | 1.00 | 21.09 | A | C |
| ATOM | 1317 | CB | ASP | A | 200 | 32.046 | 33.736 | 56.050 | 1.00 | 21.97 | A | C |

| | | | | | | | | | | | | |
|---|---|---|---|---|---|---|---|---|---|---|---|---|
| ATOM | 1318 | CG | ASP | A | 200 | 31.694 | 33.387 | 57.497 | 1.00 | 24.11 | A | C |
| ATOM | 1319 | OD1 | ASP | A | 200 | 31.638 | 32.181 | 57.824 | 1.00 | 25.48 | A | O |
| ATOM | 1320 | OD2 | ASP | A | 200 | 31.474 | 34.308 | 58.309 | 1.00 | 23.83 | A | O |
| ATOM | 1321 | C | ASP | A | 200 | 30.810 | 31.759 | 55.175 | 1.00 | 21.48 | A | C |
| ATOM | 1322 | O | ASP | A | 200 | 29.764 | 32.370 | 55.381 | 1.00 | 19.01 | A | O |
| ATOM | 1323 | N | PHE | A | 201 | 30.849 | 30.445 | 54.979 | 1.00 | 21.24 | A | N |
| ATOM | 1324 | CA | PHE | A | 201 | 29.628 | 29.659 | 54.984 | 1.00 | 20.62 | A | C |
| ATOM | 1325 | CB | PHE | A | 201 | 29.624 | 28.679 | 53.812 | 1.00 | 18.79 | A | C |
| ATOM | 1326 | CG | PHE | A | 201 | 29.365 | 29.333 | 52.493 | 1.00 | 17.54 | A | C |
| ATOM | 1327 | CD1 | PHE | A | 201 | 30.361 | 30.055 | 51.855 | 1.00 | 17.19 | A | C |
| ATOM | 1328 | CD2 | PHE | A | 201 | 28.107 | 29.270 | 51.911 | 1.00 | 15.42 | A | C |
| ATOM | 1329 | CE1 | PHE | A | 201 | 30.107 | 30.701 | 50.650 | 1.00 | 17.06 | A | C |
| ATOM | 1330 | CE2 | PHE | A | 201 | 27.842 | 29.912 | 50.709 | 1.00 | 14.34 | A | C |
| ATOM | 1331 | CZ | PHE | A | 201 | 28.838 | 30.632 | 50.080 | 1.00 | 16.14 | A | C |
| ATOM | 1332 | C | PHE | A | 201 | 29.412 | 28.924 | 56.300 | 1.00 | 20.18 | A | C |
| ATOM | 1333 | O | PHE | A | 201 | 28.715 | 27.936 | 56.345 | 1.00 | 21.05 | A | O |
| ATOM | 1334 | N | GLY | A | 202 | 30.009 | 29.445 | 57.368 | 1.00 | 18.60 | A | N |
| ATOM | 1335 | CA | GLY | A | 202 | 29.867 | 28.842 | 58.680 | 1.00 | 18.49 | A | C |
| ATOM | 1336 | C | GLY | A | 202 | 28.456 | 28.903 | 59.241 | 1.00 | 20.11 | A | C |
| ATOM | 1337 | O | GLY | A | 202 | 28.122 | 28.189 | 60.171 | 1.00 | 19.79 | A | O |
| ATOM | 1338 | N | SER | A | 203 | 27.621 | 29.762 | 58.674 | 1.00 | 20.69 | A | N |
| ATOM | 1339 | CA | SER | A | 203 | 26.245 | 29.876 | 59.137 | 1.00 | 21.14 | A | C |
| ATOM | 1340 | CB | SER | A | 203 | 25.901 | 31.344 | 59.419 | 1.00 | 20.93 | A | C |
| ATOM | 1341 | OG | SER | A | 203 | 26.703 | 31.858 | 60.468 | 1.00 | 23.49 | A | O |
| ATOM | 1342 | C | SER | A | 203 | 25.287 | 29.298 | 58.109 | 1.00 | 20.46 | A | C |
| ATOM | 1343 | O | SER | A | 203 | 24.126 | 29.102 | 58.394 | 1.00 | 20.04 | A | O |
| ATOM | 1344 | N | ALA | A | 204 | 25.802 | 29.018 | 56.913 | 1.00 | 20.25 | A | N |
| ATOM | 1345 | CA | ALA | A | 204 | 24.998 | 28.471 | 55.824 | 1.00 | 20.24 | A | C |
| ATOM | 1346 | CB | ALA | A | 204 | 25.879 | 28.210 | 54.622 | 1.00 | 20.89 | A | C |
| ATOM | 1347 | C | ALA | A | 204 | 24.274 | 27.193 | 56.217 | 1.00 | 21.13 | A | C |
| ATOM | 1348 | O | ALA | A | 204 | 24.821 | 26.356 | 56.901 | 1.00 | 20.29 | A | O |
| ATOM | 1349 | N | LYS | A | 205 | 23.038 | 27.066 | 55.751 | 1.00 | 22.01 | A | N |
| ATOM | 1350 | CA | LYS | A | 205 | 22.215 | 25.902 | 56.045 | 1.00 | 24.22 | A | C |
| ATOM | 1351 | CB | LYS | A | 205 | 21.651 | 26.023 | 57.465 | 1.00 | 23.86 | A | C |
| ATOM | 1352 | CG | LYS | A | 205 | 20.914 | 24.790 | 57.948 | 1.00 | 26.26 | A | C |
| ATOM | 1353 | CD | LYS | A | 205 | 20.464 | 24.931 | 59.390 | 1.00 | 27.03 | A | C |
| ATOM | 1354 | CE | LYS | A | 205 | 19.882 | 23.621 | 59.898 | 1.00 | 25.43 | A | C |
| ATOM | 1355 | NZ | LYS | A | 205 | 18.754 | 23.186 | 59.041 | 1.00 | 28.24 | A | N |
| ATOM | 1356 | C | LYS | A | 205 | 21.065 | 25.730 | 55.052 | 1.00 | 24.71 | A | C |
| ATOM | 1357 | O | LYS | A | 205 | 20.491 | 26.705 | 54.554 | 1.00 | 22.45 | A | O |
| ATOM | 1358 | N | GLN | A | 206 | 20.731 | 24.479 | 54.759 | 1.00 | 26.86 | A | N |
| ATOM | 1359 | CA | GLN | A | 206 | 19.623 | 24.217 | 53.860 | 1.00 | 28.77 | A | C |
| ATOM | 1360 | CB | GLN | A | 206 | 19.641 | 22.774 | 53.366 | 1.00 | 31.21 | A | C |
| ATOM | 1361 | CG | GLN | A | 206 | 18.552 | 22.508 | 52.336 | 1.00 | 33.91 | A | C |
| ATOM | 1362 | CD | GLN | A | 206 | 18.680 | 21.152 | 51.683 | 1.00 | 36.62 | A | C |
| ATOM | 1363 | OE1 | GLN | A | 206 | 17.925 | 20.820 | 50.782 | 1.00 | 39.64 | A | O |
| ATOM | 1364 | NE2 | GLN | A | 206 | 19.644 | 20.362 | 52.136 | 1.00 | 35.67 | A | N |
| ATOM | 1365 | C | GLN | A | 206 | 18.344 | 24.478 | 54.653 | 1.00 | 28.44 | A | C |
| ATOM | 1366 | O | GLN | A | 206 | 18.196 | 23.999 | 55.757 | 1.00 | 29.95 | A | O |
| ATOM | 1367 | N | LEU | A | 207 | 17.436 | 25.260 | 54.088 | 1.00 | 28.95 | A | N |
| ATOM | 1368 | CA | LEU | A | 207 | 16.183 | 25.562 | 54.766 | 1.00 | 30.71 | A | C |
| ATOM | 1369 | CB | LEU | A | 207 | 15.844 | 27.049 | 54.620 | 1.00 | 29.63 | A | C |
| ATOM | 1370 | CG | LEU | A | 207 | 16.796 | 28.039 | 55.304 | 1.00 | 29.84 | A | C |
| ATOM | 1371 | CD1 | LEU | A | 207 | 16.263 | 29.465 | 55.162 | 1.00 | 29.52 | A | C |
| ATOM | 1372 | CD2 | LEU | A | 207 | 16.941 | 27.673 | 56.767 | 1.00 | 29.53 | A | C |
| ATOM | 1373 | C | LEU | A | 207 | 15.040 | 24.732 | 54.204 | 1.00 | 32.15 | A | C |
| ATOM | 1374 | O | LEU | A | 207 | 14.568 | 24.983 | 53.100 | 1.00 | 33.45 | A | O |
| ATOM | 1375 | N | VAL | A | 208 | 14.597 | 23.743 | 54.971 | 1.00 | 34.40 | A | N |
| ATOM | 1376 | CA | VAL | A | 208 | 13.502 | 22.881 | 54.537 | 1.00 | 35.77 | A | C |
| ATOM | 1377 | CB | VAL | A | 208 | 13.779 | 21.419 | 54.926 | 1.00 | 36.08 | A | C |
| ATOM | 1378 | CG1 | VAL | A | 208 | 12.613 | 20.518 | 54.494 | 1.00 | 36.89 | A | C |
| ATOM | 1379 | CG2 | VAL | A | 208 | 15.064 | 20.964 | 54.266 | 1.00 | 34.48 | A | C |
| ATOM | 1380 | C | VAL | A | 208 | 12.193 | 23.347 | 55.166 | 1.00 | 37.50 | A | C |

| | | | | | | | | | | | | |
|---|---|---|---|---|---|---|---|---|---|---|---|---|
| ATOM | 1381 | O | VAL | A | 208 | 12.136 | 23.602 | 56.348 | 1.00 | 38.00 | A | O |
| ATOM | 1382 | N | ARG | A | 209 | 11.147 | 23.467 | 54.353 | 1.00 | 39.83 | A | N |
| ATOM | 1383 | CA | ARG | A | 209 | 9.851 | 23.920 | 54.846 | 1.00 | 41.02 | A | C |
| ATOM | 1384 | CB | ARG | A | 209 | 8.824 | 23.958 | 53.710 | 1.00 | 43.38 | A | C |
| ATOM | 1385 | CG | ARG | A | 209 | 9.176 | 24.916 | 52.563 | 1.00 | 49.07 | A | C |
| ATOM | 1386 | CD | ARG | A | 209 | 8.025 | 24.990 | 51.536 | 1.00 | 52.84 | A | C |
| ATOM | 1387 | NE | ARG | A | 209 | 7.418 | 23.670 | 51.343 | 1.00 | 55.76 | A | N |
| ATOM | 1388 | CZ | ARG | A | 209 | 6.268 | 23.445 | 50.709 | 1.00 | 57.50 | A | C |
| ATOM | 1389 | NH1 | ARG | A | 209 | 5.574 | 24.457 | 50.184 | 1.00 | 57.88 | A | N |
| ATOM | 1390 | NH2 | ARG | A | 209 | 5.802 | 22.200 | 50.621 | 1.00 | 58.01 | A | N |
| ATOM | 1391 | C | ARG | A | 209 | 9.347 | 23.012 | 55.960 | 1.00 | 40.20 | A | C |
| ATOM | 1392 | O | ARG | A | 209 | 9.457 | 21.778 | 55.875 | 1.00 | 40.11 | A | O |
| ATOM | 1393 | N | GLY | A | 210 | 8.807 | 23.631 | 57.007 | 1.00 | 38.40 | A | N |
| ATOM | 1394 | CA | GLY | A | 210 | 8.289 | 22.871 | 58.129 | 1.00 | 36.84 | A | C |
| ATOM | 1395 | C | GLY | A | 210 | 9.333 | 22.613 | 59.190 | 1.00 | 36.09 | A | C |
| ATOM | 1396 | O | GLY | A | 210 | 8.985 | 22.284 | 60.328 | 1.00 | 36.77 | A | O |
| ATOM | 1397 | N | GLU | A | 211 | 10.609 | 22.741 | 58.834 | 1.00 | 33.96 | A | N |
| ATOM | 1398 | CA | GLU | A | 211 | 11.666 | 22.528 | 59.821 | 1.00 | 34.17 | A | C |
| ATOM | 1399 | CB | GLU | A | 211 | 12.895 | 21.883 | 59.173 | 1.00 | 35.98 | A | C |
| ATOM | 1400 | CG | GLU | A | 211 | 12.623 | 20.531 | 58.526 | 1.00 | 38.93 | A | C |
| ATOM | 1401 | CD | GLU | A | 211 | 13.890 | 19.869 | 58.012 | 1.00 | 41.19 | A | C |
| ATOM | 1402 | OE1 | GLU | A | 211 | 13.816 | 18.708 | 57.546 | 1.00 | 41.62 | A | O |
| ATOM | 1403 | OE2 | GLU | A | 211 | 14.965 | 20.512 | 58.078 | 1.00 | 42.78 | A | O |
| ATOM | 1404 | C | GLU | A | 211 | 12.051 | 23.864 | 60.441 | 1.00 | 32.36 | A | C |
| ATOM | 1405 | O | GLU | A | 211 | 12.182 | 24.875 | 59.741 | 1.00 | 31.51 | A | O |
| ATOM | 1406 | N | PRO | A | 212 | 12.206 | 23.891 | 61.771 | 1.00 | 31.10 | A | N |
| ATOM | 1407 | CD | PRO | A | 212 | 11.838 | 22.812 | 62.709 | 1.00 | 31.66 | A | C |
| ATOM | 1408 | CA | PRO | A | 212 | 12.578 | 25.113 | 62.491 | 1.00 | 28.94 | A | C |
| ATOM | 1409 | CB | PRO | A | 212 | 12.081 | 24.835 | 63.906 | 1.00 | 30.70 | A | C |
| ATOM | 1410 | CG | PRO | A | 212 | 12.309 | 23.353 | 64.043 | 1.00 | 31.31 | A | C |
| ATOM | 1411 | C | PRO | A | 212 | 14.084 | 25.358 | 62.420 | 1.00 | 27.46 | A | C |
| ATOM | 1412 | O | PRO | A | 212 | 14.867 | 24.428 | 62.240 | 1.00 | 26.20 | A | O |
| ATOM | 1413 | N | ASN | A | 213 | 14.473 | 26.622 | 62.542 | 1.00 | 26.02 | A | N |
| ATOM | 1414 | CA | ASN | A | 213 | 15.877 | 27.019 | 62.511 | 1.00 | 24.26 | A | C |
| ATOM | 1415 | CB | ASN | A | 213 | 16.258 | 27.494 | 61.108 | 1.00 | 23.22 | A | C |
| ATOM | 1416 | CG | ASN | A | 213 | 16.329 | 26.350 | 60.110 | 1.00 | 22.84 | A | C |
| ATOM | 1417 | OD1 | ASN | A | 213 | 17.253 | 25.553 | 60.135 | 1.00 | 23.26 | A | O |
| ATOM | 1418 | ND2 | ASN | A | 213 | 15.338 | 26.264 | 59.239 | 1.00 | 22.17 | A | N |
| ATOM | 1419 | C | ASN | A | 213 | 16.127 | 28.114 | 63.538 | 1.00 | 23.35 | A | C |
| ATOM | 1420 | O | ASN | A | 213 | 15.240 | 28.875 | 63.845 | 1.00 | 22.28 | A | O |
| ATOM | 1421 | N | VAL | A | 214 | 17.345 | 28.173 | 64.067 | 1.00 | 22.95 | A | N |
| ATOM | 1422 | CA | VAL | A | 214 | 17.698 | 29.164 | 65.081 | 1.00 | 22.87 | A | C |
| ATOM | 1423 | CB | VAL | A | 214 | 19.135 | 28.925 | 65.608 | 1.00 | 22.74 | A | C |
| ATOM | 1424 | CG1 | VAL | A | 214 | 19.263 | 27.491 | 66.120 | 1.00 | 19.57 | A | C |
| ATOM | 1425 | CG2 | VAL | A | 214 | 20.160 | 29.204 | 64.513 | 1.00 | 21.74 | A | C |
| ATOM | 1426 | C | VAL | A | 214 | 17.575 | 30.587 | 64.544 | 1.00 | 23.51 | A | C |
| ATOM | 1427 | O | VAL | A | 214 | 17.961 | 30.879 | 63.424 | 1.00 | 22.27 | A | O |
| ATOM | 1428 | N | SER | A | 215 | 17.023 | 31.468 | 65.366 | 1.00 | 24.22 | A | N |
| ATOM | 1429 | CA | SER | A | 215 | 16.829 | 32.844 | 64.957 | 1.00 | 25.98 | A | C |
| ATOM | 1430 | CB | SER | A | 215 | 15.447 | 33.318 | 65.414 | 1.00 | 26.10 | A | C |
| ATOM | 1431 | OG | SER | A | 215 | 15.242 | 33.018 | 66.783 | 1.00 | 28.75 | A | O |
| ATOM | 1432 | C | SER | A | 215 | 17.903 | 33.814 | 65.437 | 1.00 | 25.74 | A | C |
| ATOM | 1433 | O | SER | A | 215 | 17.702 | 34.999 | 65.384 | 1.00 | 28.23 | A | O |
| ATOM | 1434 | N | TYR | A | 216 | 19.042 | 33.308 | 65.895 | 1.00 | 24.31 | A | N |
| ATOM | 1435 | CA | TYR | A | 216 | 20.113 | 34.193 | 66.351 | 1.00 | 25.04 | A | C |
| ATOM | 1436 | CB | TYR | A | 216 | 20.513 | 33.847 | 67.790 | 1.00 | 23.93 | A | C |
| ATOM | 1437 | CG | TYR | A | 216 | 20.756 | 32.381 | 68.026 | 1.00 | 24.84 | A | C |
| ATOM | 1438 | CD1 | TYR | A | 216 | 21.882 | 31.743 | 67.502 | 1.00 | 25.13 | A | C |
| ATOM | 1439 | CE1 | TYR | A | 216 | 22.089 | 30.381 | 67.686 | 1.00 | 24.27 | A | C |
| ATOM | 1440 | CD2 | TYR | A | 216 | 19.841 | 31.614 | 68.744 | 1.00 | 24.66 | A | C |
| ATOM | 1441 | CE2 | TYR | A | 216 | 20.042 | 30.255 | 68.932 | 1.00 | 23.08 | A | C |
| ATOM | 1442 | CZ | TYR | A | 216 | 21.163 | 29.647 | 68.400 | 1.00 | 23.40 | A | C |
| ATOM | 1443 | OH | TYR | A | 216 | 21.352 | 28.302 | 68.584 | 1.00 | 24.02 | A | O |

| | | | | | | | | | | | | |
|---|---|---|---|---|---|---|---|---|---|---|---|---|
| ATOM | 1444 | C | TYR | A | 216 | 21.334 | 34.112 | 65.435 | 1.00 | 25.24 | A | C |
| ATOM | 1445 | O | TYR | A | 216 | 22.449 | 34.329 | 65.880 | 1.00 | 24.80 | A | O |
| ATOM | 1446 | N | ILE | A | 217 | 21.108 | 33.831 | 64.150 | 1.00 | 25.83 | A | N |
| ATOM | 1447 | CA | ILE | A | 217 | 22.216 | 33.700 | 63.212 | 1.00 | 26.17 | A | C |
| ATOM | 1448 | CB | ILE | A | 217 | 21.989 | 32.546 | 62.202 | 1.00 | 25.14 | A | C |
| ATOM | 1449 | CG2 | ILE | A | 217 | 22.581 | 31.262 | 62.762 | 1.00 | 26.35 | A | C |
| ATOM | 1450 | CG1 | ILE | A | 217 | 20.505 | 32.418 | 61.847 | 1.00 | 23.39 | A | C |
| ATOM | 1451 | CD1 | ILE | A | 217 | 19.995 | 33.470 | 60.893 | 1.00 | 18.62 | A | C |
| ATOM | 1452 | C | ILE | A | 217 | 22.755 | 34.872 | 62.389 | 1.00 | 27.83 | A | C |
| ATOM | 1453 | O | ILE | A | 217 | 23.927 | 34.825 | 61.958 | 1.00 | 32.85 | A | O |
| ATOM | 1454 | N | CYS | A | 218 | 21.979 | 35.918 | 62.154 | 1.00 | 24.22 | A | N |
| ATOM | 1455 | CA | CYS | A | 218 | 22.502 | 36.990 | 61.298 | 1.00 | 23.07 | A | C |
| ATOM | 1456 | CB | CYS | A | 218 | 21.340 | 37.602 | 60.507 | 1.00 | 23.52 | A | C |
| ATOM | 1457 | SG | CYS | A | 218 | 21.743 | 38.309 | 58.907 | 1.00 | 22.42 | A | S |
| ATOM | 1458 | C | CYS | A | 218 | 23.276 | 38.073 | 62.068 | 1.00 | 22.56 | A | C |
| ATOM | 1459 | O | CYS | A | 218 | 23.199 | 38.133 | 63.262 | 1.00 | 22.72 | A | O |
| ATOM | 1460 | N | SER | A | 219 | 24.025 | 38.921 | 61.371 | 1.00 | 21.48 | A | N |
| ATOM | 1461 | CA | SER | A | 219 | 24.795 | 39.961 | 62.058 | 1.00 | 21.35 | A | C |
| ATOM | 1462 | CB | SER | A | 219 | 26.165 | 40.116 | 61.407 | 1.00 | 21.34 | A | C |
| ATOM | 1463 | OG | SER | A | 219 | 26.992 | 39.012 | 61.728 | 1.00 | 20.80 | A | O |
| ATOM | 1464 | C | SER | A | 219 | 24.138 | 41.335 | 62.182 | 1.00 | 21.75 | A | C |
| ATOM | 1465 | O | SER | A | 219 | 23.396 | 41.772 | 61.309 | 1.00 | 21.07 | A | O |
| ATOM | 1466 | N | ARG | A | 220 | 24.442 | 42.007 | 63.289 | 1.00 | 22.38 | A | N |
| ATOM | 1467 | CA | ARG | A | 220 | 23.911 | 43.335 | 63.598 | 1.00 | 23.74 | A | C |
| ATOM | 1468 | CB | ARG | A | 220 | 24.730 | 43.949 | 64.734 | 1.00 | 26.59 | A | C |
| ATOM | 1469 | CG | ARG | A | 220 | 24.174 | 45.242 | 65.290 | 1.00 | 32.85 | A | C |
| ATOM | 1470 | CD | ARG | A | 220 | 24.613 | 45.477 | 66.739 | 1.00 | 37.44 | A | C |
| ATOM | 1471 | NE | ARG | A | 220 | 25.371 | 46.715 | 66.904 | 1.00 | 40.19 | A | N |
| ATOM | 1472 | CZ | ARG | A | 220 | 26.507 | 46.978 | 66.266 | 1.00 | 41.61 | A | C |
| ATOM | 1473 | NH1 | ARG | A | 220 | 27.017 | 46.092 | 65.416 | 1.00 | 42.25 | A | N |
| ATOM | 1474 | NH2 | ARG | A | 220 | 27.144 | 48.120 | 66.486 | 1.00 | 41.57 | A | N |
| ATOM | 1475 | C | ARG | A | 220 | 23.915 | 44.264 | 62.385 | 1.00 | 22.42 | A | C |
| ATOM | 1476 | O | ARG | A | 220 | 24.913 | 44.379 | 61.709 | 1.00 | 24.35 | A | O |
| ATOM | 1477 | N | TYR | A | 221 | 22.778 | 44.909 | 62.135 | 1.00 | 21.09 | A | N |
| ATOM | 1478 | CA | TYR | A | 221 | 22.581 | 45.838 | 61.016 | 1.00 | 21.32 | A | C |
| ATOM | 1479 | CB | TYR | A | 221 | 23.894 | 46.497 | 60.531 | 1.00 | 20.88 | A | C |
| ATOM | 1480 | CG | TYR | A | 221 | 24.609 | 47.397 | 61.511 | 1.00 | 23.30 | A | C |
| ATOM | 1481 | CD1 | TYR | A | 221 | 23.982 | 47.846 | 62.674 | 1.00 | 23.59 | A | C |
| ATOM | 1482 | CE1 | TYR | A | 221 | 24.654 | 48.663 | 63.579 | 1.00 | 26.91 | A | C |
| ATOM | 1483 | CD2 | TYR | A | 221 | 25.933 | 47.796 | 61.271 | 1.00 | 23.68 | A | C |
| ATOM | 1484 | CE2 | TYR | A | 221 | 26.617 | 48.615 | 62.161 | 1.00 | 25.70 | A | C |
| ATOM | 1485 | CZ | TYR | A | 221 | 25.971 | 49.046 | 63.322 | 1.00 | 29.19 | A | C |
| ATOM | 1486 | OH | TYR | A | 221 | 26.642 | 49.839 | 64.233 | 1.00 | 31.39 | A | O |
| ATOM | 1487 | C | TYR | A | 221 | 21.964 | 45.166 | 59.794 | 1.00 | 20.64 | A | C |
| ATOM | 1488 | O | TYR | A | 221 | 21.220 | 45.790 | 59.044 | 1.00 | 20.14 | A | O |
| ATOM | 1489 | N | TYR | A | 222 | 22.275 | 43.892 | 59.593 | 1.00 | 21.11 | A | N |
| ATOM | 1490 | CA | TYR | A | 222 | 21.787 | 43.181 | 58.416 | 1.00 | 21.00 | A | C |
| ATOM | 1491 | CB | TYR | A | 222 | 22.981 | 42.515 | 57.721 | 1.00 | 18.45 | A | C |
| ATOM | 1492 | CG | TYR | A | 222 | 24.127 | 43.481 | 57.566 | 1.00 | 18.72 | A | C |
| ATOM | 1493 | CD1 | TYR | A | 222 | 24.101 | 44.460 | 56.570 | 1.00 | 18.10 | A | C |
| ATOM | 1494 | CE1 | TYR | A | 222 | 25.086 | 45.433 | 56.490 | 1.00 | 18.10 | A | C |
| ATOM | 1495 | CD2 | TYR | A | 222 | 25.179 | 43.499 | 58.478 | 1.00 | 18.77 | A | C |
| ATOM | 1496 | CE2 | TYR | A | 222 | 26.177 | 44.474 | 58.409 | 1.00 | 18.84 | A | C |
| ATOM | 1497 | CZ | TYR | A | 222 | 26.119 | 45.440 | 57.414 | 1.00 | 19.57 | A | C |
| ATOM | 1498 | OH | TYR | A | 222 | 27.078 | 46.421 | 57.345 | 1.00 | 19.23 | A | O |
| ATOM | 1499 | C | TYR | A | 222 | 20.699 | 42.148 | 58.689 | 1.00 | 20.92 | A | C |
| ATOM | 1500 | O | TYR | A | 222 | 20.270 | 41.465 | 57.792 | 1.00 | 20.65 | A | O |
| ATOM | 1501 | N | ARG | A | 223 | 20.239 | 42.071 | 59.929 | 1.00 | 20.60 | A | N |
| ATOM | 1502 | CA | ARG | A | 223 | 19.232 | 41.084 | 60.287 | 1.00 | 20.00 | A | C |
| ATOM | 1503 | CB | ARG | A | 223 | 19.207 | 40.864 | 61.805 | 1.00 | 19.80 | A | C |
| ATOM | 1504 | CG | ARG | A | 223 | 20.555 | 40.729 | 62.509 | 1.00 | 21.30 | A | C |
| ATOM | 1505 | CD | ARG | A | 223 | 20.343 | 40.361 | 63.994 | 1.00 | 21.90 | A | C |
| ATOM | 1506 | NE | ARG | A | 223 | 20.033 | 38.939 | 64.132 | 1.00 | 23.51 | A | N |

| | | | | | | | | | | | | |
|---|---|---|---|---|---|---|---|---|---|---|---|---|
| ATOM | 1507 | CZ | ARG | A | 223 | 19.231 | 38.411 | 65.052 | 1.00 | 23.80 | A | C |
| ATOM | 1508 | NH1 | ARG | A | 223 | 18.622 | 39.174 | 65.952 | 1.00 | 24.92 | A | N |
| ATOM | 1509 | NH2 | ARG | A | 223 | 19.040 | 37.101 | 65.069 | 1.00 | 22.28 | A | N |
| ATOM | 1510 | C | ARG | A | 223 | 17.820 | 41.438 | 59.837 | 1.00 | 19.85 | A | C |
| ATOM | 1511 | O | ARG | A | 223 | 17.312 | 42.527 | 60.120 | 1.00 | 21.01 | A | O |
| ATOM | 1512 | N | ALA | A | 224 | 17.186 | 40.499 | 59.147 | 1.00 | 20.02 | A | N |
| ATOM | 1513 | CA | ALA | A | 224 | 15.822 | 40.680 | 58.681 | 1.00 | 19.70 | A | C |
| ATOM | 1514 | CB | ALA | A | 224 | 15.398 | 39.481 | 57.830 | 1.00 | 18.82 | A | C |
| ATOM | 1515 | C | ALA | A | 224 | 14.916 | 40.804 | 59.910 | 1.00 | 19.36 | A | C |
| ATOM | 1516 | O | ALA | A | 224 | 15.175 | 40.204 | 60.935 | 1.00 | 18.68 | A | O |
| ATOM | 1517 | N | PRO | A | 225 | 13.840 | 41.598 | 59.801 | 1.00 | 20.51 | A | N |
| ATOM | 1518 | CD | PRO | A | 225 | 13.438 | 42.251 | 58.541 | 1.00 | 20.02 | A | C |
| ATOM | 1519 | CA | PRO | A | 225 | 12.853 | 41.857 | 60.856 | 1.00 | 22.48 | A | C |
| ATOM | 1520 | CB | PRO | A | 225 | 11.730 | 42.573 | 60.104 | 1.00 | 24.04 | A | C |
| ATOM | 1521 | CG | PRO | A | 225 | 12.444 | 43.259 | 59.000 | 1.00 | 20.84 | A | C |
| ATOM | 1522 | C | PRO | A | 225 | 12.348 | 40.588 | 61.553 | 1.00 | 24.51 | A | C |
| ATOM | 1523 | O | PRO | A | 225 | 12.253 | 40.545 | 62.790 | 1.00 | 25.75 | A | O |
| ATOM | 1524 | N | GLU | A | 226 | 12.020 | 39.568 | 60.761 | 1.00 | 24.27 | A | N |
| ATOM | 1525 | CA | GLU | A | 226 | 11.531 | 38.304 | 61.312 | 1.00 | 24.50 | A | C |
| ATOM | 1526 | CB | GLU | A | 226 | 11.199 | 37.276 | 60.217 | 1.00 | 26.25 | A | C |
| ATOM | 1527 | CG | GLU | A | 226 | 10.769 | 37.847 | 58.885 | 1.00 | 30.48 | A | C |
| ATOM | 1528 | CD | GLU | A | 226 | 11.941 | 38.296 | 58.036 | 1.00 | 28.74 | A | C |
| ATOM | 1529 | OE1 | GLU | A | 226 | 12.403 | 37.519 | 57.169 | 1.00 | 27.92 | A | O |
| ATOM | 1530 | OE2 | GLU | A | 226 | 12.398 | 39.432 | 58.248 | 1.00 | 30.29 | A | O |
| ATOM | 1531 | C | GLU | A | 226 | 12.573 | 37.680 | 62.239 | 1.00 | 23.33 | A | C |
| ATOM | 1532 | O | GLU | A | 226 | 12.220 | 37.050 | 63.228 | 1.00 | 24.73 | A | O |
| ATOM | 1533 | N | LEU | A | 227 | 13.853 | 37.835 | 61.915 | 1.00 | 21.87 | A | N |
| ATOM | 1534 | CA | LEU | A | 227 | 14.887 | 37.264 | 62.770 | 1.00 | 21.72 | A | C |
| ATOM | 1535 | CB | LEU | A | 227 | 16.267 | 37.321 | 62.097 | 1.00 | 20.27 | A | C |
| ATOM | 1536 | CG | LEU | A | 227 | 16.529 | 36.432 | 60.871 | 1.00 | 18.67 | A | C |
| ATOM | 1537 | CD1 | LEU | A | 227 | 17.976 | 36.609 | 60.414 | 1.00 | 19.13 | A | C |
| ATOM | 1538 | CD2 | LEU | A | 227 | 16.265 | 34.969 | 61.217 | 1.00 | 16.28 | A | C |
| ATOM | 1539 | C | LEU | A | 227 | 14.912 | 38.018 | 64.093 | 1.00 | 21.64 | A | C |
| ATOM | 1540 | O | LEU | A | 227 | 14.965 | 37.424 | 65.126 | 1.00 | 22.23 | A | O |
| ATOM | 1541 | N | ILE | A | 228 | 14.856 | 39.342 | 64.028 | 1.00 | 21.73 | A | N |
| ATOM | 1542 | CA | ILE | A | 228 | 14.863 | 40.156 | 65.234 | 1.00 | 24.05 | A | C |
| ATOM | 1543 | CB | ILE | A | 228 | 14.803 | 41.664 | 64.880 | 1.00 | 23.39 | A | C |
| ATOM | 1544 | CG2 | ILE | A | 228 | 14.806 | 42.505 | 66.148 | 1.00 | 23.59 | A | C |
| ATOM | 1545 | CG1 | ILE | A | 228 | 16.011 | 42.043 | 64.016 | 1.00 | 23.22 | A | C |
| ATOM | 1546 | CD1 | ILE | A | 228 | 16.087 | 43.512 | 63.668 | 1.00 | 22.01 | A | C |
| ATOM | 1547 | C | ILE | A | 228 | 13.657 | 39.769 | 66.097 | 1.00 | 24.63 | A | C |
| ATOM | 1548 | O | ILE | A | 228 | 13.763 | 39.649 | 67.300 | 1.00 | 25.29 | A | O |
| ATOM | 1549 | N | PHE | A | 229 | 12.518 | 39.559 | 65.446 | 1.00 | 24.98 | A | N |
| ATOM | 1550 | CA | PHE | A | 229 | 11.292 | 39.168 | 66.130 | 1.00 | 24.17 | A | C |
| ATOM | 1551 | CB | PHE | A | 229 | 10.103 | 39.321 | 65.181 | 1.00 | 23.57 | A | C |
| ATOM | 1552 | CG | PHE | A | 229 | 9.551 | 40.712 | 65.126 | 1.00 | 23.62 | A | C |
| ATOM | 1553 | CD1 | PHE | A | 229 | 9.024 | 41.305 | 66.272 | 1.00 | 24.77 | A | C |
| ATOM | 1554 | CD2 | PHE | A | 229 | 9.552 | 41.431 | 63.938 | 1.00 | 22.88 | A | C |
| ATOM | 1555 | CE1 | PHE | A | 229 | 8.511 | 42.592 | 66.235 | 1.00 | 24.08 | A | C |
| ATOM | 1556 | CE2 | PHE | A | 229 | 9.040 | 42.722 | 63.894 | 1.00 | 22.38 | A | C |
| ATOM | 1557 | CZ | PHE | A | 229 | 8.517 | 43.301 | 65.042 | 1.00 | 21.87 | A | C |
| ATOM | 1558 | C | PHE | A | 229 | 11.339 | 37.735 | 66.658 | 1.00 | 23.31 | A | C |
| ATOM | 1559 | O | PHE | A | 229 | 10.394 | 37.273 | 67.261 | 1.00 | 24.41 | A | O |
| ATOM | 1560 | N | GLY | A | 230 | 12.444 | 37.039 | 66.413 | 1.00 | 23.43 | A | N |
| ATOM | 1561 | CA | GLY | A | 230 | 12.588 | 35.678 | 66.898 | 1.00 | 23.01 | A | C |
| ATOM | 1562 | C | GLY | A | 230 | 11.828 | 34.577 | 66.179 | 1.00 | 25.12 | A | C |
| ATOM | 1563 | O | GLY | A | 230 | 11.425 | 33.592 | 66.809 | 1.00 | 26.12 | A | O |
| ATOM | 1564 | N | ALA | A | 231 | 11.630 | 34.724 | 64.872 | 1.00 | 24.41 | A | N |
| ATOM | 1565 | CA | ALA | A | 231 | 10.919 | 33.711 | 64.096 | 1.00 | 24.32 | A | C |
| ATOM | 1566 | CB | ALA | A | 231 | 10.418 | 34.301 | 62.787 | 1.00 | 24.71 | A | C |
| ATOM | 1567 | C | ALA | A | 231 | 11.870 | 32.560 | 63.814 | 1.00 | 25.29 | A | C |
| ATOM | 1568 | O | ALA | A | 231 | 13.074 | 32.759 | 63.670 | 1.00 | 25.15 | A | O |
| ATOM | 1569 | N | THR | A | 232 | 11.320 | 31.354 | 63.748 | 1.00 | 25.46 | A | N |

| | | | | | | | | | | | | |
|---|---|---|---|---|---|---|---|---|---|---|---|---|
| ATOM | 1570 | CA | THR | A | 232 | 12.121 | 30.169 | 63.477 | 1.00 | 25.39 | A | C |
| ATOM | 1571 | CB | THR | A | 232 | 12.047 | 29.182 | 64.655 | 1.00 | 26.47 | A | C |
| ATOM | 1572 | OG1 | THR | A | 232 | 10.704 | 28.708 | 64.797 | 1.00 | 26.48 | A | O |
| ATOM | 1573 | CG2 | THR | A | 232 | 12.467 | 29.874 | 65.941 | 1.00 | 26.00 | A | C |
| ATOM | 1574 | C | THR | A | 232 | 11.626 | 29.477 | 62.205 | 1.00 | 24.62 | A | C |
| ATOM | 1575 | O | THR | A | 232 | 12.090 | 28.398 | 61.857 | 1.00 | 23.39 | A | O |
| ATOM | 1576 | N | ASP | A | 233 | 10.685 | 30.119 | 61.517 | 1.00 | 24.23 | A | N |
| ATOM | 1577 | CA | ASP | A | 233 | 10.127 | 29.571 | 60.282 | 1.00 | 25.86 | A | C |
| ATOM | 1578 | CB | ASP | A | 233 | 8.597 | 29.491 | 60.381 | 1.00 | 26.67 | A | C |
| ATOM | 1579 | CG | ASP | A | 233 | 7.944 | 30.861 | 60.412 | 1.00 | 29.74 | A | C |
| ATOM | 1580 | OD1 | ASP | A | 233 | 8.600 | 31.826 | 60.856 | 1.00 | 29.55 | A | O |
| ATOM | 1581 | OD2 | ASP | A | 233 | 6.768 | 30.977 | 60.007 | 1.00 | 33.46 | A | O |
| ATOM | 1582 | C | ASP | A | 233 | 10.505 | 30.457 | 59.096 | 1.00 | 24.85 | A | C |
| ATOM | 1583 | O | ASP | A | 233 | 9.806 | 30.484 | 58.087 | 1.00 | 23.56 | A | O |
| ATOM | 1584 | N | TYR | A | 234 | 11.614 | 31.179 | 59.224 | 1.00 | 23.87 | A | N |
| ATOM | 1585 | CA | TYR | A | 234 | 12.050 | 32.070 | 58.154 | 1.00 | 23.85 | A | C |
| ATOM | 1586 | CB | TYR | A | 234 | 13.116 | 33.040 | 58.674 | 1.00 | 22.65 | A | C |
| ATOM | 1587 | CG | TYR | A | 234 | 14.353 | 32.369 | 59.223 | 1.00 | 21.36 | A | C |
| ATOM | 1588 | CD1 | TYR | A | 234 | 15.379 | 31.960 | 58.379 | 1.00 | 19.80 | A | C |
| ATOM | 1589 | CE1 | TYR | A | 234 | 16.518 | 31.339 | 58.888 | 1.00 | 20.68 | A | C |
| ATOM | 1590 | CD2 | TYR | A | 234 | 14.490 | 32.134 | 60.596 | 1.00 | 21.47 | A | C |
| ATOM | 1591 | CE2 | TYR | A | 234 | 15.620 | 31.509 | 61.115 | 1.00 | 20.06 | A | C |
| ATOM | 1592 | CZ | TYR | A | 234 | 16.629 | 31.115 | 60.256 | 1.00 | 20.28 | A | C |
| ATOM | 1593 | OH | TYR | A | 234 | 17.744 | 30.485 | 60.757 | 1.00 | 21.37 | A | O |
| ATOM | 1594 | C | TYR | A | 234 | 12.569 | 31.322 | 56.926 | 1.00 | 23.90 | A | C |
| ATOM | 1595 | O | TYR | A | 234 | 12.939 | 30.151 | 57.000 | 1.00 | 23.42 | A | O |
| ATOM | 1596 | N | THR | A | 235 | 12.576 | 32.013 | 55.794 | 1.00 | 24.75 | A | N |
| ATOM | 1597 | CA | THR | A | 235 | 13.041 | 31.431 | 54.541 | 1.00 | 26.17 | A | C |
| ATOM | 1598 | CB | THR | A | 235 | 12.013 | 31.641 | 53.424 | 1.00 | 27.71 | A | C |
| ATOM | 1599 | OG1 | THR | A | 235 | 11.956 | 33.034 | 53.092 | 1.00 | 30.51 | A | O |
| ATOM | 1600 | CG2 | THR | A | 235 | 10.623 | 31.176 | 53.879 | 1.00 | 23.34 | A | C |
| ATOM | 1601 | C | THR | A | 235 | 14.352 | 32.070 | 54.108 | 1.00 | 25.82 | A | C |
| ATOM | 1602 | O | THR | A | 235 | 14.886 | 32.950 | 54.792 | 1.00 | 24.42 | A | O |
| ATOM | 1603 | N | SER | A | 236 | 14.866 | 31.626 | 52.967 | 1.00 | 24.34 | A | N |
| ATOM | 1604 | CA | SER | A | 236 | 16.118 | 32.154 | 52.457 | 1.00 | 24.64 | A | C |
| ATOM | 1605 | CB | SER | A | 236 | 16.602 | 31.333 | 51.263 | 1.00 | 26.12 | A | C |
| ATOM | 1606 | OG | SER | A | 236 | 15.882 | 31.704 | 50.098 | 1.00 | 31.25 | A | O |
| ATOM | 1607 | C | SER | A | 236 | 15.965 | 33.619 | 52.058 | 1.00 | 22.56 | A | C |
| ATOM | 1608 | O | SER | A | 236 | 16.925 | 34.259 | 51.684 | 1.00 | 19.92 | A | O |
| ATOM | 1609 | N | SER | A | 237 | 14.748 | 34.147 | 52.150 | 1.00 | 21.54 | A | N |
| ATOM | 1610 | CA | SER | A | 237 | 14.548 | 35.541 | 51.808 | 1.00 | 20.50 | A | C |
| ATOM | 1611 | CB | SER | A | 237 | 13.061 | 35.893 | 51.716 | 1.00 | 19.95 | A | C |
| ATOM | 1612 | OG | SER | A | 237 | 12.436 | 35.906 | 52.981 | 1.00 | 23.05 | A | O |
| ATOM | 1613 | C | SER | A | 237 | 15.258 | 36.449 | 52.814 | 1.00 | 21.13 | A | C |
| ATOM | 1614 | O | SER | A | 237 | 15.360 | 37.661 | 52.583 | 1.00 | 21.45 | A | O |
| ATOM | 1615 | N | ILE | A | 238 | 15.756 | 35.879 | 53.918 | 1.00 | 17.74 | A | N |
| ATOM | 1616 | CA | ILE | A | 238 | 16.479 | 36.701 | 54.888 | 1.00 | 17.12 | A | C |
| ATOM | 1617 | CB | ILE | A | 238 | 16.835 | 35.968 | 56.200 | 1.00 | 15.45 | A | C |
| ATOM | 1618 | CG2 | ILE | A | 238 | 15.574 | 35.607 | 56.957 | 1.00 | 12.81 | A | C |
| ATOM | 1619 | CG1 | ILE | A | 238 | 17.723 | 34.770 | 55.907 | 1.00 | 15.25 | A | C |
| ATOM | 1620 | CD1 | ILE | A | 238 | 18.328 | 34.167 | 57.157 | 1.00 | 16.44 | A | C |
| ATOM | 1621 | C | ILE | A | 238 | 17.770 | 37.219 | 54.240 | 1.00 | 18.31 | A | C |
| ATOM | 1622 | O | ILE | A | 238 | 18.236 | 38.304 | 54.559 | 1.00 | 17.56 | A | O |
| ATOM | 1623 | N | ASP | A | 239 | 18.325 | 36.435 | 53.315 | 1.00 | 17.56 | A | N |
| ATOM | 1624 | CA | ASP | A | 239 | 19.530 | 36.832 | 52.603 | 1.00 | 18.92 | A | C |
| ATOM | 1625 | CB | ASP | A | 239 | 20.022 | 35.700 | 51.701 | 1.00 | 19.79 | A | C |
| ATOM | 1626 | CG | ASP | A | 239 | 20.623 | 34.546 | 52.482 | 1.00 | 21.07 | A | C |
| ATOM | 1627 | OD1 | ASP | A | 239 | 21.118 | 34.768 | 53.606 | 1.00 | 21.63 | A | O |
| ATOM | 1628 | OD2 | ASP | A | 239 | 20.618 | 33.413 | 51.955 | 1.00 | 24.63 | A | O |
| ATOM | 1629 | C | ASP | A | 239 | 19.251 | 38.066 | 51.745 | 1.00 | 19.73 | A | C |
| ATOM | 1630 | O | ASP | A | 239 | 20.083 | 38.946 | 51.631 | 1.00 | 17.98 | A | O |
| ATOM | 1631 | N | VAL | A | 240 | 18.068 | 38.109 | 51.142 | 1.00 | 18.89 | A | N |
| ATOM | 1632 | CA | VAL | A | 240 | 17.689 | 39.239 | 50.309 | 1.00 | 19.59 | A | C |

| | | | | | | | | | | | | |
|---|---|---|---|---|---|---|---|---|---|---|---|---|
| ATOM | 1633 | CB | VAL | A | 240 | 16.351 | 38.986 | 49.577 | 1.00 | 19.71 | A | C |
| ATOM | 1634 | CG1 | VAL | A | 240 | 15.913 | 40.240 | 48.861 | 1.00 | 19.47 | A | C |
| ATOM | 1635 | CG2 | VAL | A | 240 | 16.521 | 37.859 | 48.567 | 1.00 | 20.01 | A | C |
| ATOM | 1636 | C | VAL | A | 240 | 17.598 | 40.511 | 51.144 | 1.00 | 19.83 | A | C |
| ATOM | 1637 | O | VAL | A | 240 | 18.071 | 41.560 | 50.722 | 1.00 | 19.90 | A | O |
| ATOM | 1638 | N | TRP | A | 241 | 16.997 | 40.413 | 52.329 | 1.00 | 19.02 | A | N |
| ATOM | 1639 | CA | TRP | A | 241 | 16.901 | 41.576 | 53.209 | 1.00 | 18.15 | A | C |
| ATOM | 1640 | CB | TRP | A | 241 | 16.165 | 41.222 | 54.505 | 1.00 | 16.19 | A | C |
| ATOM | 1641 | CG | TRP | A | 241 | 16.218 | 42.319 | 55.528 | 1.00 | 16.64 | A | C |
| ATOM | 1642 | CD2 | TRP | A | 241 | 15.225 | 43.325 | 55.759 | 1.00 | 16.90 | A | C |
| ATOM | 1643 | CE2 | TRP | A | 241 | 15.732 | 44.191 | 56.755 | 1.00 | 16.17 | A | C |
| ATOM | 1644 | CE3 | TRP | A | 241 | 13.961 | 43.591 | 55.209 | 1.00 | 15.56 | A | C |
| ATOM | 1645 | CD1 | TRP | A | 241 | 17.251 | 42.600 | 56.379 | 1.00 | 15.77 | A | C |
| ATOM | 1646 | NE1 | TRP | A | 241 | 16.967 | 43.719 | 57.117 | 1.00 | 15.00 | A | N |
| ATOM | 1647 | CZ2 | TRP | A | 241 | 15.011 | 45.297 | 57.228 | 1.00 | 16.60 | A | C |
| ATOM | 1648 | CZ3 | TRP | A | 241 | 13.245 | 44.692 | 55.678 | 1.00 | 16.99 | A | C |
| ATOM | 1649 | CH2 | TRP | A | 241 | 13.777 | 45.534 | 56.674 | 1.00 | 17.83 | A | C |
| ATOM | 1650 | C | TRP | A | 241 | 18.322 | 42.056 | 53.516 | 1.00 | 16.99 | A | C |
| ATOM | 1651 | O | TRP | A | 241 | 18.614 | 43.240 | 53.431 | 1.00 | 16.62 | A | O |
| ATOM | 1652 | N | SER | A | 242 | 19.201 | 41.120 | 53.857 | 1.00 | 16.68 | A | N |
| ATOM | 1653 | CA | SER | A | 242 | 20.588 | 41.461 | 54.155 | 1.00 | 17.39 | A | C |
| ATOM | 1654 | CB | SER | A | 242 | 21.378 | 40.207 | 54.524 | 1.00 | 16.23 | A | C |
| ATOM | 1655 | OG | SER | A | 242 | 21.008 | 39.737 | 55.799 | 1.00 | 16.51 | A | O |
| ATOM | 1656 | C | SER | A | 242 | 21.251 | 42.146 | 52.964 | 1.00 | 17.70 | A | C |
| ATOM | 1657 | O | SER | A | 242 | 21.971 | 43.117 | 53.119 | 1.00 | 18.92 | A | O |
| ATOM | 1658 | N | ALA | A | 243 | 21.001 | 41.617 | 51.773 | 1.00 | 17.09 | A | N |
| ATOM | 1659 | CA | ALA | A | 243 | 21.570 | 42.188 | 50.567 | 1.00 | 17.85 | A | C |
| ATOM | 1660 | CB | ALA | A | 243 | 21.192 | 41.342 | 49.355 | 1.00 | 18.13 | A | C |
| ATOM | 1661 | C | ALA | A | 243 | 21.054 | 43.613 | 50.421 | 1.00 | 18.27 | A | C |
| ATOM | 1662 | O | ALA | A | 243 | 21.805 | 44.522 | 50.077 | 1.00 | 18.76 | A | O |
| ATOM | 1663 | N | GLY | A | 244 | 19.768 | 43.794 | 50.697 | 1.00 | 18.87 | A | N |
| ATOM | 1664 | CA | GLY | A | 244 | 19.170 | 45.115 | 50.626 | 1.00 | 18.26 | A | C |
| ATOM | 1665 | C | GLY | A | 244 | 19.853 | 46.080 | 51.582 | 1.00 | 19.54 | A | C |
| ATOM | 1666 | O | GLY | A | 244 | 20.009 | 47.246 | 51.264 | 1.00 | 20.43 | A | O |
| ATOM | 1667 | N | CYS | A | 245 | 20.261 | 45.595 | 52.754 | 1.00 | 17.58 | A | N |
| ATOM | 1668 | CA | CYS | A | 245 | 20.927 | 46.459 | 53.722 | 1.00 | 17.48 | A | C |
| ATOM | 1669 | CB | CYS | A | 245 | 21.036 | 45.758 | 55.082 | 1.00 | 15.82 | A | C |
| ATOM | 1670 | SG | CYS | A | 245 | 19.448 | 45.528 | 55.931 | 1.00 | 16.96 | A | S |
| ATOM | 1671 | C | CYS | A | 245 | 22.313 | 46.849 | 53.202 | 1.00 | 17.67 | A | C |
| ATOM | 1672 | O | CYS | A | 245 | 22.741 | 47.965 | 53.383 | 1.00 | 15.55 | A | O |
| ATOM | 1673 | N | VAL | A | 246 | 22.992 | 45.907 | 52.553 | 1.00 | 15.07 | A | N |
| ATOM | 1674 | CA | VAL | A | 246 | 24.313 | 46.166 | 51.986 | 1.00 | 17.46 | A | C |
| ATOM | 1675 | CB | VAL | A | 246 | 24.917 | 44.874 | 51.372 | 1.00 | 16.46 | A | C |
| ATOM | 1676 | CG1 | VAL | A | 246 | 26.199 | 45.182 | 50.628 | 1.00 | 14.47 | A | C |
| ATOM | 1677 | CG2 | VAL | A | 246 | 25.172 | 43.853 | 52.472 | 1.00 | 16.49 | A | C |
| ATOM | 1678 | C | VAL | A | 246 | 24.229 | 47.243 | 50.901 | 1.00 | 18.71 | A | C |
| ATOM | 1679 | O | VAL | A | 246 | 25.061 | 48.157 | 50.864 | 1.00 | 18.67 | A | O |
| ATOM | 1680 | N | LEU | A | 247 | 23.225 | 47.118 | 50.030 | 1.00 | 18.44 | A | N |
| ATOM | 1681 | CA | LEU | A | 247 | 23.000 | 48.078 | 48.952 | 1.00 | 19.22 | A | C |
| ATOM | 1682 | CB | LEU | A | 247 | 21.798 | 47.654 | 48.096 | 1.00 | 20.02 | A | C |
| ATOM | 1683 | CG | LEU | A | 247 | 21.271 | 48.706 | 47.108 | 1.00 | 21.95 | A | C |
| ATOM | 1684 | CD1 | LEU | A | 247 | 22.363 | 49.074 | 46.110 | 1.00 | 18.27 | A | C |
| ATOM | 1685 | CD2 | LEU | A | 247 | 20.025 | 48.171 | 46.378 | 1.00 | 24.09 | A | C |
| ATOM | 1686 | C | LEU | A | 247 | 22.754 | 49.461 | 49.545 | 1.00 | 19.30 | A | C |
| ATOM | 1687 | O | LEU | A | 247 | 23.410 | 50.416 | 49.189 | 1.00 | 20.80 | A | O |
| ATOM | 1688 | N | ALA | A | 248 | 21.798 | 49.550 | 50.458 | 1.00 | 18.29 | A | N |
| ATOM | 1689 | CA | ALA | A | 248 | 21.476 | 50.815 | 51.093 | 1.00 | 18.77 | A | C |
| ATOM | 1690 | CB | ALA | A | 248 | 20.385 | 50.606 | 52.132 | 1.00 | 17.56 | A | C |
| ATOM | 1691 | C | ALA | A | 248 | 22.719 | 51.439 | 51.740 | 1.00 | 20.52 | A | C |
| ATOM | 1692 | O | ALA | A | 248 | 22.924 | 52.640 | 51.641 | 1.00 | 20.16 | A | O |
| ATOM | 1693 | N | GLU | A | 249 | 23.545 | 50.612 | 52.385 | 1.00 | 20.37 | A | N |
| ATOM | 1694 | CA | GLU | A | 249 | 24.756 | 51.097 | 53.047 | 1.00 | 20.88 | A | C |
| ATOM | 1695 | CB | GLU | A | 249 | 25.392 | 49.983 | 53.894 | 1.00 | 21.08 | A | C |

| | | | | | | | | | | | | |
|---|---|---|---|---|---|---|---|---|---|---|---|---|
| ATOM | 1696 | CG | GLU | A | 249 | 26.499 | 50.452 | 54.847 | 1.00 | 18.92 | A | C |
| ATOM | 1697 | CD | GLU | A | 249 | 26.915 | 49.377 | 55.840 | 1.00 | 21.50 | A | C |
| ATOM | 1698 | OE1 | GLU | A | 249 | 26.079 | 48.518 | 56.163 | 1.00 | 22.72 | A | O |
| ATOM | 1699 | OE2 | GLU | A | 249 | 28.065 | 49.391 | 56.315 | 1.00 | 22.94 | A | O |
| ATOM | 1700 | C | GLU | A | 249 | 25.780 | 51.636 | 52.060 | 1.00 | 20.58 | A | C |
| ATOM | 1701 | O | GLU | A | 249 | 26.415 | 52.646 | 52.322 | 1.00 | 21.29 | A | O |
| ATOM | 1702 | N | LEU | A | 250 | 25.946 | 50.953 | 50.929 | 1.00 | 21.81 | A | N |
| ATOM | 1703 | CA | LEU | A | 250 | 26.886 | 51.396 | 49.908 | 1.00 | 21.37 | A | C |
| ATOM | 1704 | CB | LEU | A | 250 | 27.023 | 50.329 | 48.821 | 1.00 | 20.07 | A | C |
| ATOM | 1705 | CG | LEU | A | 250 | 27.759 | 49.032 | 49.182 | 1.00 | 20.08 | A | C |
| ATOM | 1706 | CD1 | LEU | A | 250 | 27.800 | 48.102 | 47.974 | 1.00 | 18.14 | A | C |
| ATOM | 1707 | CD2 | LEU | A | 250 | 29.162 | 49.352 | 49.648 | 1.00 | 19.26 | A | C |
| ATOM | 1708 | C | LEU | A | 250 | 26.420 | 52.720 | 49.298 | 1.00 | 23.66 | A | C |
| ATOM | 1709 | O | LEU | A | 250 | 27.218 | 53.539 | 48.916 | 1.00 | 24.74 | A | O |
| ATOM | 1710 | N | LEU | A | 251 | 25.110 | 52.919 | 49.228 | 1.00 | 24.13 | A | N |
| ATOM | 1711 | CA | LEU | A | 251 | 24.565 | 54.146 | 48.666 | 1.00 | 24.98 | A | C |
| ATOM | 1712 | CB | LEU | A | 251 | 23.114 | 53.918 | 48.226 | 1.00 | 24.19 | A | C |
| ATOM | 1713 | CG | LEU | A | 251 | 22.905 | 52.964 | 47.044 | 1.00 | 25.74 | A | C |
| ATOM | 1714 | CD1 | LEU | A | 251 | 21.428 | 52.660 | 46.868 | 1.00 | 25.68 | A | C |
| ATOM | 1715 | CD2 | LEU | A | 251 | 23.481 | 53.583 | 45.774 | 1.00 | 25.45 | A | C |
| ATOM | 1716 | C | LEU | A | 251 | 24.622 | 55.277 | 49.681 | 1.00 | 25.55 | A | C |
| ATOM | 1717 | O | LEU | A | 251 | 25.010 | 56.379 | 49.360 | 1.00 | 27.04 | A | O |
| ATOM | 1718 | N | LEU | A | 252 | 24.256 | 54.973 | 50.916 | 1.00 | 26.13 | A | N |
| ATOM | 1719 | CA | LEU | A | 252 | 24.204 | 55.960 | 51.989 | 1.00 | 26.94 | A | C |
| ATOM | 1720 | CB | LEU | A | 252 | 23.243 | 55.437 | 53.058 | 1.00 | 28.67 | A | C |
| ATOM | 1721 | CG | LEU | A | 252 | 22.404 | 56.433 | 53.854 | 1.00 | 32.52 | A | C |
| ATOM | 1722 | CD1 | LEU | A | 252 | 21.210 | 56.911 | 53.028 | 1.00 | 31.94 | A | C |
| ATOM | 1723 | CD2 | LEU | A | 252 | 21.916 | 55.748 | 55.121 | 1.00 | 34.91 | A | C |
| ATOM | 1724 | C | LEU | A | 252 | 25.539 | 56.345 | 52.645 | 1.00 | 26.15 | A | C |
| ATOM | 1725 | O | LEU | A | 252 | 25.660 | 57.422 | 53.170 | 1.00 | 28.07 | A | O |
| ATOM | 1726 | N | GLY | A | 253 | 26.523 | 55.451 | 52.618 | 1.00 | 25.41 | A | N |
| ATOM | 1727 | CA | GLY | A | 253 | 27.807 | 55.737 | 53.239 | 1.00 | 22.23 | A | C |
| ATOM | 1728 | C | GLY | A | 253 | 27.850 | 55.297 | 54.692 | 1.00 | 22.93 | A | C |
| ATOM | 1729 | O | GLY | A | 253 | 28.843 | 55.506 | 55.371 | 1.00 | 21.70 | A | O |
| ATOM | 1730 | N | GLN | A | 254 | 26.760 | 54.686 | 55.160 | 1.00 | 21.55 | A | N |
| ATOM | 1731 | CA | GLN | A | 254 | 26.652 | 54.206 | 56.538 | 1.00 | 21.86 | A | C |
| ATOM | 1732 | CB | GLN | A | 254 | 26.348 | 55.374 | 57.486 | 1.00 | 23.52 | A | C |
| ATOM | 1733 | CG | GLN | A | 254 | 24.982 | 56.037 | 57.248 | 1.00 | 28.30 | A | C |
| ATOM | 1734 | CD | GLN | A | 254 | 24.698 | 57.219 | 58.181 | 1.00 | 32.70 | A | C |
| ATOM | 1735 | OE1 | GLN | A | 254 | 23.558 | 57.441 | 58.587 | 1.00 | 33.89 | A | O |
| ATOM | 1736 | NE2 | GLN | A | 254 | 25.734 | 57.989 | 58.505 | 1.00 | 35.14 | A | N |
| ATOM | 1737 | C | GLN | A | 254 | 25.517 | 53.183 | 56.609 | 1.00 | 20.25 | A | C |
| ATOM | 1738 | O | GLN | A | 254 | 24.703 | 53.101 | 55.714 | 1.00 | 20.50 | A | O |
| ATOM | 1739 | N | PRO | A | 255 | 25.468 | 52.381 | 57.681 | 1.00 | 20.14 | A | N |
| ATOM | 1740 | CD | PRO | A | 255 | 26.483 | 52.240 | 58.741 | 1.00 | 20.65 | A | C |
| ATOM | 1741 | CA | PRO | A | 255 | 24.410 | 51.374 | 57.834 | 1.00 | 19.64 | A | C |
| ATOM | 1742 | CB | PRO | A | 255 | 24.758 | 50.707 | 59.162 | 1.00 | 19.15 | A | C |
| ATOM | 1743 | CG | PRO | A | 255 | 26.244 | 50.825 | 59.230 | 1.00 | 20.07 | A | C |
| ATOM | 1744 | C | PRO | A | 255 | 23.027 | 52.020 | 57.872 | 1.00 | 20.02 | A | C |
| ATOM | 1745 | O | PRO | A | 255 | 22.817 | 52.995 | 58.575 | 1.00 | 21.62 | A | O |
| ATOM | 1746 | N | ILE | A | 256 | 22.083 | 51.459 | 57.126 | 1.00 | 21.00 | A | N |
| ATOM | 1747 | CA | ILE | A | 256 | 20.735 | 52.005 | 57.080 | 1.00 | 20.37 | A | C |
| ATOM | 1748 | CB | ILE | A | 256 | 19.967 | 51.468 | 55.827 | 1.00 | 20.91 | A | C |
| ATOM | 1749 | CG2 | ILE | A | 256 | 19.785 | 49.947 | 55.909 | 1.00 | 18.52 | A | C |
| ATOM | 1750 | CG1 | ILE | A | 256 | 18.620 | 52.181 | 55.704 | 1.00 | 21.76 | A | C |
| ATOM | 1751 | CD1 | ILE | A | 256 | 17.883 | 51.908 | 54.407 | 1.00 | 24.16 | A | C |
| ATOM | 1752 | C | ILE | A | 256 | 19.926 | 51.745 | 58.353 | 1.00 | 20.18 | A | C |
| ATOM | 1753 | O | ILE | A | 256 | 19.146 | 52.598 | 58.768 | 1.00 | 19.86 | A | O |
| ATOM | 1754 | N | PHE | A | 257 | 20.120 | 50.583 | 58.977 | 1.00 | 20.20 | A | N |
| ATOM | 1755 | CA | PHE | A | 257 | 19.378 | 50.253 | 60.201 | 1.00 | 20.68 | A | C |
| ATOM | 1756 | CB | PHE | A | 257 | 18.462 | 49.052 | 59.948 | 1.00 | 21.02 | A | C |
| ATOM | 1757 | CG | PHE | A | 257 | 17.507 | 49.240 | 58.801 | 1.00 | 21.13 | A | C |
| ATOM | 1758 | CD1 | PHE | A | 257 | 16.690 | 50.367 | 58.732 | 1.00 | 22.09 | A | C |

| | | | | | | | | | | | | |
|---|---|---|---|---|---|---|---|---|---|---|---|---|
| ATOM | 1759 | CD2 | PHE | A | 257 | 17.397 | 48.275 | 57.806 | 1.00 | 20.75 | A | C |
| ATOM | 1760 | CE1 | PHE | A | 257 | 15.774 | 50.524 | 57.682 | 1.00 | 21.86 | A | C |
| ATOM | 1761 | CE2 | PHE | A | 257 | 16.485 | 48.424 | 56.756 | 1.00 | 20.38 | A | C |
| ATOM | 1762 | CZ | PHE | A | 257 | 15.672 | 49.549 | 56.694 | 1.00 | 18.09 | A | C |
| ATOM | 1763 | C | PHE | A | 257 | 20.305 | 49.937 | 61.376 | 1.00 | 20.08 | A | C |
| ATOM | 1764 | O | PHE | A | 257 | 20.440 | 48.794 | 61.770 | 1.00 | 19.49 | A | O |
| ATOM | 1765 | N | PRO | A | 258 | 20.945 | 50.961 | 61.957 | 1.00 | 20.17 | A | N |
| ATOM | 1766 | CD | PRO | A | 258 | 20.972 | 52.377 | 61.553 | 1.00 | 21.81 | A | C |
| ATOM | 1767 | CA | PRO | A | 258 | 21.853 | 50.724 | 63.083 | 1.00 | 20.77 | A | C |
| ATOM | 1768 | CB | PRO | A | 258 | 22.751 | 51.952 | 63.054 | 1.00 | 20.65 | A | C |
| ATOM | 1769 | CG | PRO | A | 258 | 21.789 | 53.031 | 62.665 | 1.00 | 20.13 | A | C |
| ATOM | 1770 | C | PRO | A | 258 | 21.137 | 50.575 | 64.412 | 1.00 | 21.80 | A | C |
| ATOM | 1771 | O | PRO | A | 258 | 19.948 | 50.853 | 64.526 | 1.00 | 21.28 | A | O |
| ATOM | 1772 | N | GLY | A | 259 | 21.884 | 50.137 | 65.418 | 1.00 | 21.76 | A | N |
| ATOM | 1773 | CA | GLY | A | 259 | 21.317 | 49.961 | 66.737 | 1.00 | 20.69 | A | C |
| ATOM | 1774 | C | GLY | A | 259 | 22.021 | 48.835 | 67.454 | 1.00 | 21.92 | A | C |
| ATOM | 1775 | O | GLY | A | 259 | 22.371 | 47.838 | 66.847 | 1.00 | 22.99 | A | O |
| ATOM | 1776 | N | ASP | A | 260 | 22.233 | 49.008 | 68.752 | 1.00 | 21.55 | A | N |
| ATOM | 1777 | CA | ASP | A | 260 | 22.897 | 48.008 | 69.564 | 1.00 | 21.79 | A | C |
| ATOM | 1778 | CB | ASP | A | 260 | 23.748 | 48.669 | 70.641 | 1.00 | 25.26 | A | C |
| ATOM | 1779 | CG | ASP | A | 260 | 24.752 | 49.638 | 70.069 | 1.00 | 27.14 | A | C |
| ATOM | 1780 | OD1 | ASP | A | 260 | 25.076 | 49.509 | 68.865 | 1.00 | 26.38 | A | O |
| ATOM | 1781 | OD2 | ASP | A | 260 | 25.214 | 50.515 | 70.833 | 1.00 | 27.67 | A | O |
| ATOM | 1782 | C | ASP | A | 260 | 21.910 | 47.075 | 70.238 | 1.00 | 21.06 | A | C |
| ATOM | 1783 | O | ASP | A | 260 | 22.273 | 46.345 | 71.128 | 1.00 | 21.95 | A | O |
| ATOM | 1784 | N | SER | A | 261 | 20.652 | 47.129 | 69.828 | 1.00 | 19.62 | A | N |
| ATOM | 1785 | CA | SER | A | 261 | 19.658 | 46.240 | 70.402 | 1.00 | 19.11 | A | C |
| ATOM | 1786 | CB | SER | A | 261 | 18.977 | 46.883 | 71.616 | 1.00 | 18.53 | A | C |
| ATOM | 1787 | OG | SER | A | 261 | 17.987 | 47.819 | 71.234 | 1.00 | 18.77 | A | O |
| ATOM | 1788 | C | SER | A | 261 | 18.620 | 45.952 | 69.331 | 1.00 | 19.87 | A | C |
| ATOM | 1789 | O | SER | A | 261 | 18.413 | 46.755 | 68.428 | 1.00 | 20.16 | A | O |
| ATOM | 1790 | N | GLY | A | 262 | 17.976 | 44.797 | 69.436 | 1.00 | 20.74 | A | N |
| ATOM | 1791 | CA | GLY | A | 262 | 16.953 | 44.447 | 68.477 | 1.00 | 20.55 | A | C |
| ATOM | 1792 | C | GLY | A | 262 | 15.890 | 45.529 | 68.428 | 1.00 | 22.06 | A | C |
| ATOM | 1793 | O | GLY | A | 262 | 15.479 | 45.946 | 67.358 | 1.00 | 22.17 | A | O |
| ATOM | 1794 | N | VAL | A | 263 | 15.461 | 45.989 | 69.599 | 1.00 | 22.00 | A | N |
| ATOM | 1795 | CA | VAL | A | 263 | 14.447 | 47.031 | 69.695 | 1.00 | 23.54 | A | C |
| ATOM | 1796 | CB | VAL | A | 263 | 14.188 | 47.436 | 71.173 | 1.00 | 24.78 | A | C |
| ATOM | 1797 | CG1 | VAL | A | 263 | 12.903 | 48.228 | 71.272 | 1.00 | 26.49 | A | C |
| ATOM | 1798 | CG2 | VAL | A | 263 | 14.122 | 46.211 | 72.056 | 1.00 | 26.55 | A | C |
| ATOM | 1799 | C | VAL | A | 263 | 14.868 | 48.282 | 68.927 | 1.00 | 23.14 | A | C |
| ATOM | 1800 | O | VAL | A | 263 | 14.091 | 48.827 | 68.169 | 1.00 | 22.95 | A | O |
| ATOM | 1801 | N | ASP | A | 264 | 16.107 | 48.726 | 69.144 | 1.00 | 22.67 | A | N |
| ATOM | 1802 | CA | ASP | A | 264 | 16.630 | 49.912 | 68.473 | 1.00 | 22.53 | A | C |
| ATOM | 1803 | CB | ASP | A | 264 | 18.013 | 50.271 | 69.032 | 1.00 | 21.68 | A | C |
| ATOM | 1804 | CG | ASP | A | 264 | 17.928 | 50.935 | 70.403 | 1.00 | 22.73 | A | C |
| ATOM | 1805 | OD1 | ASP | A | 264 | 16.811 | 51.347 | 70.779 | 1.00 | 23.53 | A | O |
| ATOM | 1806 | OD2 | ASP | A | 264 | 18.958 | 51.062 | 71.097 | 1.00 | 23.65 | A | O |
| ATOM | 1807 | C | ASP | A | 264 | 16.679 | 49.717 | 66.954 | 1.00 | 22.23 | A | C |
| ATOM | 1808 | O | ASP | A | 264 | 16.373 | 50.617 | 66.203 | 1.00 | 22.69 | A | O |
| ATOM | 1809 | N | GLN | A | 265 | 17.055 | 48.521 | 66.520 | 1.00 | 20.63 | A | N |
| ATOM | 1810 | CA | GLN | A | 265 | 17.111 | 48.219 | 65.106 | 1.00 | 20.98 | A | C |
| ATOM | 1811 | CB | GLN | A | 265 | 17.674 | 46.820 | 64.891 | 1.00 | 18.91 | A | C |
| ATOM | 1812 | CG | GLN | A | 265 | 19.168 | 46.749 | 65.093 | 1.00 | 20.51 | A | C |
| ATOM | 1813 | CD | GLN | A | 265 | 19.629 | 45.381 | 65.542 | 1.00 | 23.62 | A | C |
| ATOM | 1814 | OE1 | GLN | A | 265 | 19.075 | 44.370 | 65.143 | 1.00 | 20.92 | A | O |
| ATOM | 1815 | NE2 | GLN | A | 265 | 20.666 | 45.352 | 66.379 | 1.00 | 24.53 | A | N |
| ATOM | 1816 | C | GLN | A | 265 | 15.698 | 48.311 | 64.544 | 1.00 | 20.93 | A | C |
| ATOM | 1817 | O | GLN | A | 265 | 15.477 | 48.896 | 63.491 | 1.00 | 18.95 | A | O |
| ATOM | 1818 | N | LEU | A | 266 | 14.744 | 47.737 | 65.266 | 1.00 | 21.03 | A | N |
| ATOM | 1819 | CA | LEU | A | 266 | 13.355 | 47.770 | 64.833 | 1.00 | 22.88 | A | C |
| ATOM | 1820 | CB | LEU | A | 266 | 12.469 | 47.007 | 65.817 | 1.00 | 24.42 | A | C |
| ATOM | 1821 | CG | LEU | A | 266 | 11.875 | 45.725 | 65.233 | 1.00 | 24.76 | A | C |

| | | | | | | | | | | | | |
|---|---|---|---|---|---|---|---|---|---|---|---|---|
| ATOM | 1822 | CD1 | LEU | A | 266 | 12.934 | 44.960 | 64.473 | 1.00 | 26.33 | A | C |
| ATOM | 1823 | CD2 | LEU | A | 266 | 11.300 | 44.877 | 66.350 | 1.00 | 26.09 | A | C |
| ATOM | 1824 | C | LEU | A | 266 | 12.839 | 49.192 | 64.664 | 1.00 | 22.84 | A | C |
| ATOM | 1825 | O | LEU | A | 266 | 12.156 | 49.491 | 63.692 | 1.00 | 22.54 | A | O |
| ATOM | 1826 | N | VAL | A | 267 | 13.182 | 50.062 | 65.609 | 1.00 | 21.29 | A | N |
| ATOM | 1827 | CA | VAL | A | 267 | 12.761 | 51.456 | 65.541 | 1.00 | 22.26 | A | C |
| ATOM | 1828 | CB | VAL | A | 267 | 13.260 | 52.251 | 66.758 | 1.00 | 21.58 | A | C |
| ATOM | 1829 | CG1 | VAL | A | 267 | 13.056 | 53.732 | 66.522 | 1.00 | 18.41 | A | C |
| ATOM | 1830 | CG2 | VAL | A | 267 | 12.534 | 51.795 | 68.008 | 1.00 | 19.55 | A | C |
| ATOM | 1831 | C | VAL | A | 267 | 13.339 | 52.111 | 64.290 | 1.00 | 22.84 | A | C |
| ATOM | 1832 | O | VAL | A | 267 | 12.661 | 52.877 | 63.604 | 1.00 | 23.39 | A | O |
| ATOM | 1833 | N | GLU | A | 268 | 14.603 | 51.816 | 64.013 | 1.00 | 22.26 | A | N |
| ATOM | 1834 | CA | GLU | A | 268 | 15.258 | 52.379 | 62.849 | 1.00 | 22.21 | A | C |
| ATOM | 1835 | CB | GLU | A | 268 | 16.730 | 51.965 | 62.794 | 1.00 | 23.64 | A | C |
| ATOM | 1836 | CG | GLU | A | 268 | 17.614 | 52.644 | 63.827 | 1.00 | 27.30 | A | C |
| ATOM | 1837 | CD | GLU | A | 268 | 17.555 | 54.162 | 63.746 | 1.00 | 30.66 | A | C |
| ATOM | 1838 | OE1 | GLU | A | 268 | 17.654 | 54.705 | 62.621 | 1.00 | 31.13 | A | O |
| ATOM | 1839 | OE2 | GLU | A | 268 | 17.417 | 54.807 | 64.813 | 1.00 | 30.86 | A | O |
| ATOM | 1840 | C | GLU | A | 268 | 14.546 | 51.907 | 61.586 | 1.00 | 22.05 | A | C |
| ATOM | 1841 | O | GLU | A | 268 | 14.365 | 52.670 | 60.661 | 1.00 | 20.47 | A | O |
| ATOM | 1842 | N | ILE | A | 269 | 14.146 | 50.640 | 61.554 | 1.00 | 21.96 | A | N |
| ATOM | 1843 | CA | ILE | A | 269 | 13.455 | 50.118 | 60.383 | 1.00 | 24.17 | A | C |
| ATOM | 1844 | CB | ILE | A | 269 | 13.296 | 48.585 | 60.429 | 1.00 | 22.70 | A | C |
| ATOM | 1845 | CG2 | ILE | A | 269 | 12.398 | 48.121 | 59.286 | 1.00 | 21.84 | A | C |
| ATOM | 1846 | CG1 | ILE | A | 269 | 14.666 | 47.919 | 60.323 | 1.00 | 21.67 | A | C |
| ATOM | 1847 | CD1 | ILE | A | 269 | 14.635 | 46.438 | 60.551 | 1.00 | 19.34 | A | C |
| ATOM | 1848 | C | ILE | A | 269 | 12.073 | 50.743 | 60.272 | 1.00 | 26.16 | A | C |
| ATOM | 1849 | O | ILE | A | 269 | 11.599 | 51.031 | 59.173 | 1.00 | 26.24 | A | O |
| ATOM | 1850 | N | ILE | A | 270 | 11.437 | 50.951 | 61.422 | 1.00 | 26.94 | A | N |
| ATOM | 1851 | CA | ILE | A | 270 | 10.118 | 51.553 | 61.465 | 1.00 | 27.39 | A | C |
| ATOM | 1852 | CB | ILE | A | 270 | 9.516 | 51.485 | 62.896 | 1.00 | 26.42 | A | C |
| ATOM | 1853 | CG2 | ILE | A | 270 | 8.259 | 52.340 | 62.982 | 1.00 | 27.68 | A | C |
| ATOM | 1854 | CG1 | ILE | A | 270 | 9.187 | 50.031 | 63.243 | 1.00 | 24.85 | A | C |
| ATOM | 1855 | CD1 | ILE | A | 270 | 8.777 | 49.795 | 64.675 | 1.00 | 23.03 | A | C |
| ATOM | 1856 | C | ILE | A | 270 | 10.182 | 53.005 | 60.980 | 1.00 | 27.78 | A | C |
| ATOM | 1857 | O | ILE | A | 270 | 9.306 | 53.446 | 60.274 | 1.00 | 28.70 | A | O |
| ATOM | 1858 | N | LYS | A | 271 | 11.242 | 53.727 | 61.341 | 1.00 | 28.60 | A | N |
| ATOM | 1859 | CA | LYS | A | 271 | 11.380 | 55.117 | 60.916 | 1.00 | 29.22 | A | C |
| ATOM | 1860 | CB | LYS | A | 271 | 12.647 | 55.755 | 61.498 | 1.00 | 29.65 | A | C |
| ATOM | 1861 | CG | LYS | A | 271 | 12.633 | 55.992 | 62.995 | 1.00 | 33.54 | A | C |
| ATOM | 1862 | CD | LYS | A | 271 | 13.797 | 56.896 | 63.399 | 1.00 | 36.47 | A | C |
| ATOM | 1863 | CE | LYS | A | 271 | 14.407 | 56.453 | 64.713 | 1.00 | 39.12 | A | C |
| ATOM | 1864 | NZ | LYS | A | 271 | 14.677 | 57.616 | 65.627 | 1.00 | 43.57 | A | N |
| ATOM | 1865 | C | LYS | A | 271 | 11.415 | 55.283 | 59.393 | 1.00 | 30.54 | A | C |
| ATOM | 1866 | O | LYS | A | 271 | 11.246 | 56.384 | 58.895 | 1.00 | 30.70 | A | O |
| ATOM | 1867 | N | VAL | A | 272 | 11.648 | 54.202 | 58.652 | 1.00 | 30.52 | A | N |
| ATOM | 1868 | CA | VAL | A | 272 | 11.672 | 54.322 | 57.201 | 1.00 | 30.17 | A | C |
| ATOM | 1869 | CB | VAL | A | 272 | 13.035 | 53.881 | 56.580 | 1.00 | 30.73 | A | C |
| ATOM | 1870 | CG1 | VAL | A | 272 | 14.197 | 54.546 | 57.311 | 1.00 | 28.40 | A | C |
| ATOM | 1871 | CG2 | VAL | A | 272 | 13.158 | 52.386 | 56.603 | 1.00 | 33.61 | A | C |
| ATOM | 1872 | C | VAL | A | 272 | 10.550 | 53.560 | 56.514 | 1.00 | 29.95 | A | C |
| ATOM | 1873 | O | VAL | A | 272 | 9.941 | 54.081 | 55.592 | 1.00 | 31.86 | A | O |
| ATOM | 1874 | N | LEU | A | 273 | 10.261 | 52.343 | 56.964 | 1.00 | 29.24 | A | N |
| ATOM | 1875 | CA | LEU | A | 273 | 9.196 | 51.548 | 56.349 | 1.00 | 28.82 | A | C |
| ATOM | 1876 | CB | LEU | A | 273 | 9.465 | 50.049 | 56.535 | 1.00 | 27.62 | A | C |
| ATOM | 1877 | CG | LEU | A | 273 | 10.633 | 49.392 | 55.792 | 1.00 | 26.98 | A | C |
| ATOM | 1878 | CD1 | LEU | A | 273 | 10.587 | 47.893 | 56.003 | 1.00 | 23.92 | A | C |
| ATOM | 1879 | CD2 | LEU | A | 273 | 10.538 | 49.711 | 54.308 | 1.00 | 28.04 | A | C |
| ATOM | 1880 | C | LEU | A | 273 | 7.825 | 51.863 | 56.916 | 1.00 | 29.85 | A | C |
| ATOM | 1881 | O | LEU | A | 273 | 6.817 | 51.597 | 56.294 | 1.00 | 30.72 | A | O |
| ATOM | 1882 | N | GLY | A | 274 | 7.806 | 52.432 | 58.112 | 1.00 | 29.58 | A | N |
| ATOM | 1883 | CA | GLY | A | 274 | 6.549 | 52.730 | 58.759 | 1.00 | 30.38 | A | C |
| ATOM | 1884 | C | GLY | A | 274 | 6.286 | 51.543 | 59.656 | 1.00 | 31.41 | A | C |

| | | | | | | | | | | | | |
|---|---|---|---|---|---|---|---|---|---|---|---|---|
| ATOM | 1885 | O | GLY | A | 274 | 6.904 | 50.478 | 59.494 | 1.00 | 29.55 | A | O |
| ATOM | 1886 | N | THR | A | 275 | 5.379 | 51.729 | 60.604 | 1.00 | 32.10 | A | N |
| ATOM | 1887 | CA | THR | A | 275 | 5.025 | 50.688 | 61.549 | 1.00 | 33.11 | A | C |
| ATOM | 1888 | CB | THR | A | 275 | 4.063 | 51.247 | 62.606 | 1.00 | 32.43 | A | C |
| ATOM | 1889 | OG1 | THR | A | 275 | 4.620 | 52.452 | 63.146 | 1.00 | 30.38 | A | O |
| ATOM | 1890 | CG2 | THR | A | 275 | 3.872 | 50.251 | 63.734 | 1.00 | 34.23 | A | C |
| ATOM | 1891 | C | THR | A | 275 | 4.388 | 49.525 | 60.794 | 1.00 | 33.87 | A | C |
| ATOM | 1892 | O | THR | A | 275 | 3.584 | 49.727 | 59.885 | 1.00 | 34.46 | A | O |
| ATOM | 1893 | N | PRO | A | 276 | 4.776 | 48.288 | 61.132 | 1.00 | 33.03 | A | N |
| ATOM | 1894 | CD | PRO | A | 276 | 5.884 | 47.864 | 62.006 | 1.00 | 33.31 | A | C |
| ATOM | 1895 | CA | PRO | A | 276 | 4.186 | 47.144 | 60.434 | 1.00 | 34.01 | A | C |
| ATOM | 1896 | CB | PRO | A | 276 | 5.123 | 45.993 | 60.802 | 1.00 | 33.61 | A | C |
| ATOM | 1897 | CG | PRO | A | 276 | 5.618 | 46.379 | 62.156 | 1.00 | 33.03 | A | C |
| ATOM | 1898 | C | PRO | A | 276 | 2.734 | 46.867 | 60.829 | 1.00 | 34.65 | A | C |
| ATOM | 1899 | O | PRO | A | 276 | 2.351 | 46.971 | 61.993 | 1.00 | 35.03 | A | O |
| ATOM | 1900 | N | THR | A | 277 | 1.934 | 46.507 | 59.836 | 1.00 | 35.12 | A | N |
| ATOM | 1901 | CA | THR | A | 277 | 0.526 | 46.204 | 60.044 | 1.00 | 34.44 | A | C |
| ATOM | 1902 | CB | THR | A | 277 | -0.210 | 46.156 | 58.692 | 1.00 | 33.20 | A | C |
| ATOM | 1903 | OG1 | THR | A | 277 | 0.263 | 45.039 | 57.927 | 1.00 | 32.89 | A | O |
| ATOM | 1904 | CG2 | THR | A | 277 | 0.052 | 47.434 | 57.904 | 1.00 | 31.06 | A | C |
| ATOM | 1905 | C | THR | A | 277 | 0.411 | 44.849 | 60.740 | 1.00 | 35.39 | A | C |
| ATOM | 1906 | O | THR | A | 277 | 1.392 | 44.106 | 60.833 | 1.00 | 35.16 | A | O |
| ATOM | 1907 | N | ALA | A | 278 | -0.782 | 44.533 | 61.231 | 1.00 | 35.51 | A | N |
| ATOM | 1908 | CA | ALA | A | 278 | -1.001 | 43.268 | 61.919 | 1.00 | 35.92 | A | C |
| ATOM | 1909 | CB | ALA | A | 278 | -2.450 | 43.166 | 62.391 | 1.00 | 37.57 | A | C |
| ATOM | 1910 | C | ALA | A | 278 | -0.679 | 42.109 | 60.995 | 1.00 | 35.25 | A | C |
| ATOM | 1911 | O | ALA | A | 278 | -0.017 | 41.149 | 61.392 | 1.00 | 35.23 | A | O |
| ATOM | 1912 | N | ALA | A | 279 | -1.152 | 42.212 | 59.756 | 1.00 | 35.14 | A | N |
| ATOM | 1913 | CA | ALA | A | 279 | -0.937 | 41.169 | 58.759 | 1.00 | 35.77 | A | C |
| ATOM | 1914 | CB | ALA | A | 279 | -1.675 | 41.524 | 57.456 | 1.00 | 33.47 | A | C |
| ATOM | 1915 | C | ALA | A | 279 | 0.556 | 40.967 | 58.493 | 1.00 | 35.63 | A | C |
| ATOM | 1916 | O | ALA | A | 279 | 1.025 | 39.829 | 58.326 | 1.00 | 36.30 | A | O |
| ATOM | 1917 | N | GLN | A | 280 | 1.299 | 42.067 | 58.461 | 1.00 | 35.02 | A | N |
| ATOM | 1918 | CA | GLN | A | 280 | 2.734 | 41.992 | 58.223 | 1.00 | 34.97 | A | C |
| ATOM | 1919 | CB | GLN | A | 280 | 3.295 | 43.396 | 58.000 | 1.00 | 33.88 | A | C |
| ATOM | 1920 | CG | GLN | A | 280 | 2.932 | 43.969 | 56.641 | 1.00 | 32.79 | A | C |
| ATOM | 1921 | CD | GLN | A | 280 | 3.331 | 45.420 | 56.477 | 1.00 | 33.33 | A | C |
| ATOM | 1922 | OE1 | GLN | A | 280 | 3.716 | 45.853 | 55.381 | 1.00 | 32.27 | A | O |
| ATOM | 1923 | NE2 | GLN | A | 280 | 3.228 | 46.191 | 57.560 | 1.00 | 31.22 | A | N |
| ATOM | 1924 | C | GLN | A | 280 | 3.444 | 41.289 | 59.386 | 1.00 | 36.10 | A | C |
| ATOM | 1925 | O | GLN | A | 280 | 4.411 | 40.523 | 59.182 | 1.00 | 35.36 | A | O |
| ATOM | 1926 | N | ALA | A | 281 | 2.968 | 41.536 | 60.602 | 1.00 | 36.11 | A | N |
| ATOM | 1927 | CA | ALA | A | 281 | 3.553 | 40.897 | 61.769 | 1.00 | 36.54 | A | C |
| ATOM | 1928 | CB | ALA | A | 281 | 2.892 | 41.406 | 63.044 | 1.00 | 37.37 | A | C |
| ATOM | 1929 | C | ALA | A | 281 | 3.334 | 39.398 | 61.630 | 1.00 | 37.38 | A | C |
| ATOM | 1930 | O | ALA | A | 281 | 4.220 | 38.595 | 61.936 | 1.00 | 36.62 | A | O |
| ATOM | 1931 | N | ALA | A | 282 | 2.150 | 39.024 | 61.148 | 1.00 | 38.09 | A | N |
| ATOM | 1932 | CA | ALA | A | 282 | 1.808 | 37.615 | 60.977 | 1.00 | 38.76 | A | C |
| ATOM | 1933 | CB | ALA | A | 282 | 0.383 | 37.485 | 60.446 | 1.00 | 39.32 | A | C |
| ATOM | 1934 | C | ALA | A | 282 | 2.774 | 36.891 | 60.041 | 1.00 | 39.44 | A | C |
| ATOM | 1935 | O | ALA | A | 282 | 3.257 | 35.801 | 60.360 | 1.00 | 39.40 | A | O |
| ATOM | 1936 | N | ALA | A | 283 | 3.045 | 37.502 | 58.889 | 1.00 | 39.72 | A | N |
| ATOM | 1937 | CA | ALA | A | 283 | 3.937 | 36.913 | 57.897 | 1.00 | 39.85 | A | C |
| ATOM | 1938 | CB | ALA | A | 283 | 3.950 | 37.763 | 56.633 | 1.00 | 39.77 | A | C |
| ATOM | 1939 | C | ALA | A | 283 | 5.353 | 36.740 | 58.433 | 1.00 | 39.40 | A | C |
| ATOM | 1940 | O | ALA | A | 283 | 5.998 | 35.711 | 58.179 | 1.00 | 39.41 | A | O |
| ATOM | 1941 | N | MET | A | 284 | 5.840 | 37.748 | 59.161 | 1.00 | 39.37 | A | N |
| ATOM | 1942 | CA | MET | A | 284 | 7.179 | 37.678 | 59.753 | 1.00 | 39.28 | A | C |
| ATOM | 1943 | CB | MET | A | 284 | 7.616 | 39.053 | 60.269 | 1.00 | 37.37 | A | C |
| ATOM | 1944 | CG | MET | A | 284 | 7.738 | 40.109 | 59.179 | 1.00 | 36.82 | A | C |
| ATOM | 1945 | SD | MET | A | 284 | 8.496 | 41.668 | 59.697 | 1.00 | 31.23 | A | S |
| ATOM | 1946 | CE | MET | A | 284 | 7.143 | 42.434 | 60.635 | 1.00 | 32.69 | A | C |
| ATOM | 1947 | C | MET | A | 284 | 7.155 | 36.639 | 60.879 | 1.00 | 39.40 | A | C |

| | | | | | | | | | | | | |
|---|---|---|---|---|---|---|---|---|---|---|---|---|
| ATOM | 1948 | O | MET | A | 284 | 8.181 | 36.045 | 61.222 | 1.00 | 38.91 | A | O |
| ATOM | 1949 | N | ASN | A | 285 | 5.969 | 36.442 | 61.449 | 1.00 | 40.86 | A | N |
| ATOM | 1950 | CA | ASN | A | 285 | 5.743 | 35.429 | 62.479 | 1.00 | 41.64 | A | C |
| ATOM | 1951 | CB | ASN | A | 285 | 5.780 | 34.058 | 61.813 | 1.00 | 42.06 | A | C |
| ATOM | 1952 | CG | ASN | A | 285 | 4.924 | 33.036 | 62.528 | 1.00 | 42.83 | A | C |
| ATOM | 1953 | OD1 | ASN | A | 285 | 4.831 | 33.023 | 63.767 | 1.00 | 42.65 | A | O |
| ATOM | 1954 | ND2 | ASN | A | 285 | 4.296 | 32.160 | 61.754 | 1.00 | 42.68 | A | N |
| ATOM | 1955 | C | ASN | A | 285 | 6.727 | 35.428 | 63.643 | 1.00 | 41.88 | A | C |
| ATOM | 1956 | O | ASN | A | 285 | 7.531 | 34.508 | 63.780 | 1.00 | 41.13 | A | O |
| ATOM | 1957 | N | PRO | A | 286 | 6.664 | 36.439 | 64.512 | 1.00 | 43.03 | A | N |
| ATOM | 1958 | CD | PRO | A | 286 | 5.742 | 37.587 | 64.499 | 1.00 | 43.64 | A | C |
| ATOM | 1959 | CA | PRO | A | 286 | 7.584 | 36.496 | 65.658 | 1.00 | 43.94 | A | C |
| ATOM | 1960 | CB | PRO | A | 286 | 7.435 | 37.935 | 66.142 | 1.00 | 45.24 | A | C |
| ATOM | 1961 | CG | PRO | A | 286 | 5.991 | 38.229 | 65.862 | 1.00 | 45.21 | A | C |
| ATOM | 1962 | C | PRO | A | 286 | 7.255 | 35.479 | 66.753 | 1.00 | 43.53 | A | C |
| ATOM | 1963 | O | PRO | A | 286 | 6.109 | 35.028 | 66.852 | 1.00 | 43.19 | A | O |
| ATOM | 1964 | N | ASN | A | 287 | 8.248 | 35.112 | 67.568 | 1.00 | 43.55 | A | N |
| ATOM | 1965 | CA | ASN | A | 287 | 7.987 | 34.173 | 68.655 | 1.00 | 43.99 | A | C |
| ATOM | 1966 | CB | ASN | A | 287 | 9.289 | 33.767 | 69.392 | 1.00 | 42.48 | A | C |
| ATOM | 1967 | CG | ASN | A | 287 | 10.069 | 34.953 | 69.978 | 1.00 | 42.15 | A | C |
| ATOM | 1968 | OD1 | ASN | A | 287 | 9.662 | 36.119 | 69.866 | 1.00 | 39.50 | A | O |
| ATOM | 1969 | ND2 | ASN | A | 287 | 11.212 | 34.646 | 70.616 | 1.00 | 39.81 | A | N |
| ATOM | 1970 | C | ASN | A | 287 | 6.973 | 34.827 | 69.598 | 1.00 | 44.16 | A | C |
| ATOM | 1971 | O | ASN | A | 287 | 6.074 | 34.173 | 70.103 | 1.00 | 44.13 | A | O |
| ATOM | 1972 | N | TYR | A | 288 | 7.124 | 36.135 | 69.803 | 1.00 | 45.02 | A | N |
| ATOM | 1973 | CA | TYR | A | 288 | 6.206 | 36.915 | 70.631 | 1.00 | 45.95 | A | C |
| ATOM | 1974 | CB | TYR | A | 288 | 6.414 | 36.682 | 72.131 | 1.00 | 46.67 | A | C |
| ATOM | 1975 | CG | TYR | A | 288 | 5.538 | 37.600 | 72.955 | 1.00 | 47.83 | A | C |
| ATOM | 1976 | CD1 | TYR | A | 288 | 4.144 | 37.490 | 72.911 | 1.00 | 49.04 | A | C |
| ATOM | 1977 | CE1 | TYR | A | 288 | 3.326 | 38.370 | 73.617 | 1.00 | 50.03 | A | C |
| ATOM | 1978 | CD2 | TYR | A | 288 | 6.094 | 38.613 | 73.736 | 1.00 | 48.07 | A | C |
| ATOM | 1979 | CE2 | TYR | A | 288 | 5.288 | 39.500 | 74.448 | 1.00 | 49.43 | A | C |
| ATOM | 1980 | CZ | TYR | A | 288 | 3.902 | 39.370 | 74.387 | 1.00 | 50.66 | A | C |
| ATOM | 1981 | OH | TYR | A | 288 | 3.090 | 40.233 | 75.101 | 1.00 | 51.30 | A | O |
| ATOM | 1982 | C | TYR | A | 288 | 6.367 | 38.404 | 70.366 | 1.00 | 46.28 | A | C |
| ATOM | 1983 | O | TYR | A | 288 | 7.491 | 38.921 | 70.248 | 1.00 | 46.95 | A | O |
| ATOM | 1984 | N | GLY | A | 289 | 5.240 | 39.098 | 70.286 | 1.00 | 45.93 | A | N |
| ATOM | 1985 | CA | GLY | A | 289 | 5.276 | 40.525 | 70.050 | 1.00 | 45.71 | A | C |
| ATOM | 1986 | C | GLY | A | 289 | 4.190 | 41.178 | 70.883 | 1.00 | 46.08 | A | C |
| ATOM | 1987 | O | GLY | A | 289 | 3.161 | 40.553 | 71.174 | 1.00 | 45.17 | A | O |
| ATOM | 1988 | N | ALA | A | 290 | 4.417 | 42.425 | 71.278 | 1.00 | 45.39 | A | N |
| ATOM | 1989 | CA | ALA | A | 290 | 3.440 | 43.153 | 72.063 | 1.00 | 45.68 | A | C |
| ATOM | 1990 | CB | ALA | A | 290 | 4.067 | 43.638 | 73.372 | 1.00 | 45.69 | A | C |
| ATOM | 1991 | C | ALA | A | 290 | 2.953 | 44.336 | 71.237 | 1.00 | 45.63 | A | C |
| ATOM | 1992 | O | ALA | A | 290 | 1.795 | 44.705 | 71.307 | 1.00 | 45.67 | A | O |
| ATOM | 2004 | N | TRP | A | 301 | 11.214 | 56.597 | 50.481 | 1.00 | 45.06 | A | N |
| ATOM | 2005 | CA | TRP | A | 301 | 12.541 | 56.076 | 50.210 | 1.00 | 43.81 | A | C |
| ATOM | 2006 | CB | TRP | A | 301 | 12.441 | 54.838 | 49.325 | 1.00 | 42.40 | A | C |
| ATOM | 2007 | CG | TRP | A | 301 | 12.170 | 53.594 | 50.089 | 1.00 | 41.47 | A | C |
| ATOM | 2008 | CD2 | TRP | A | 301 | 13.127 | 52.838 | 50.832 | 1.00 | 40.02 | A | C |
| ATOM | 2009 | CE2 | TRP | A | 301 | 12.453 | 51.724 | 51.384 | 1.00 | 39.64 | A | C |
| ATOM | 2010 | CE3 | TRP | A | 301 | 14.498 | 52.990 | 51.090 | 1.00 | 39.55 | A | C |
| ATOM | 2011 | CD1 | TRP | A | 301 | 10.977 | 52.940 | 50.212 | 1.00 | 41.82 | A | C |
| ATOM | 2012 | NE1 | TRP | A | 301 | 11.136 | 51.808 | 50.990 | 1.00 | 41.59 | A | N |
| ATOM | 2013 | CZ2 | TRP | A | 301 | 13.094 | 50.768 | 52.170 | 1.00 | 39.13 | A | C |
| ATOM | 2014 | CZ3 | TRP | A | 301 | 15.141 | 52.042 | 51.874 | 1.00 | 39.37 | A | C |
| ATOM | 2015 | CH2 | TRP | A | 301 | 14.437 | 50.943 | 52.405 | 1.00 | 38.91 | A | C |
| ATOM | 2016 | C | TRP | A | 301 | 13.403 | 57.118 | 49.524 | 1.00 | 43.97 | A | C |
| ATOM | 2017 | O | TRP | A | 301 | 14.612 | 57.133 | 49.673 | 1.00 | 44.02 | A | O |
| ATOM | 2018 | N | THR | A | 302 | 12.763 | 57.995 | 48.767 | 1.00 | 44.65 | A | N |
| ATOM | 2019 | CA | THR | A | 302 | 13.483 | 59.027 | 48.046 | 1.00 | 44.70 | A | C |
| ATOM | 2020 | CB | THR | A | 302 | 12.544 | 59.760 | 47.079 | 1.00 | 45.51 | A | C |
| ATOM | 2021 | OG1 | THR | A | 302 | 11.221 | 59.212 | 47.199 | 1.00 | 47.43 | A | O |

| | | | | | | | | | | | | |
|---|---|---|---|---|---|---|---|---|---|---|---|---|
| ATOM | 2022 | CG2 | THR | A | 302 | 13.025 | 59.584 | 45.643 | 1.00 | 44.92 | A | C |
| ATOM | 2023 | C | THR | A | 302 | 14.120 | 60.011 | 49.014 | 1.00 | 43.43 | A | C |
| ATOM | 2024 | O | THR | A | 302 | 15.179 | 60.550 | 48.742 | 1.00 | 43.88 | A | O |
| ATOM | 2025 | N | ALA | A | 303 | 13.477 | 60.217 | 50.157 | 1.00 | 42.24 | A | N |
| ATOM | 2026 | CA | ALA | A | 303 | 13.998 | 61.127 | 51.169 | 1.00 | 40.88 | A | C |
| ATOM | 2027 | CB | ALA | A | 303 | 12.909 | 61.412 | 52.209 | 1.00 | 40.11 | A | C |
| ATOM | 2028 | C | ALA | A | 303 | 15.221 | 60.500 | 51.847 | 1.00 | 39.65 | A | C |
| ATOM | 2029 | O | ALA | A | 303 | 16.103 | 61.194 | 52.350 | 1.00 | 39.99 | A | O |
| ATOM | 2030 | N | VAL | A | 304 | 15.257 | 59.173 | 51.847 | 1.00 | 37.86 | A | N |
| ATOM | 2031 | CA | VAL | A | 304 | 16.333 | 58.429 | 52.487 | 1.00 | 35.43 | A | C |
| ATOM | 2032 | CB | VAL | A | 304 | 16.058 | 56.903 | 52.425 | 1.00 | 34.87 | A | C |
| ATOM | 2033 | CG1 | VAL | A | 304 | 17.274 | 56.120 | 52.908 | 1.00 | 32.37 | A | C |
| ATOM | 2034 | CG2 | VAL | A | 304 | 14.844 | 56.566 | 53.276 | 1.00 | 32.48 | A | C |
| ATOM | 2035 | C | VAL | A | 304 | 17.731 | 58.692 | 51.926 | 1.00 | 34.73 | A | C |
| ATOM | 2036 | O | VAL | A | 304 | 18.676 | 58.874 | 52.682 | 1.00 | 33.78 | A | O |
| ATOM | 2037 | N | PHE | A | 305 | 17.863 | 58.718 | 50.603 | 1.00 | 33.59 | A | N |
| ATOM | 2038 | CA | PHE | A | 305 | 19.169 | 58.902 | 49.994 | 1.00 | 32.69 | A | C |
| ATOM | 2039 | CB | PHE | A | 305 | 19.330 | 57.914 | 48.847 | 1.00 | 30.63 | A | C |
| ATOM | 2040 | CG | PHE | A | 305 | 19.138 | 56.490 | 49.258 | 1.00 | 28.96 | A | C |
| ATOM | 2041 | CD1 | PHE | A | 305 | 20.135 | 55.812 | 49.950 | 1.00 | 25.66 | A | C |
| ATOM | 2042 | CD2 | PHE | A | 305 | 17.942 | 55.831 | 48.980 | 1.00 | 26.44 | A | C |
| ATOM | 2043 | CE1 | PHE | A | 305 | 19.942 | 54.496 | 50.361 | 1.00 | 25.79 | A | C |
| ATOM | 2044 | CE2 | PHE | A | 305 | 17.743 | 54.520 | 49.387 | 1.00 | 24.79 | A | C |
| ATOM | 2045 | CZ | PHE | A | 305 | 18.742 | 53.849 | 50.076 | 1.00 | 23.69 | A | C |
| ATOM | 2046 | C | PHE | A | 305 | 19.497 | 60.297 | 49.480 | 1.00 | 34.95 | A | C |
| ATOM | 2047 | O | PHE | A | 305 | 18.623 | 61.146 | 49.314 | 1.00 | 34.78 | A | O |
| ATOM | 2048 | N | ARG | A | 306 | 20.777 | 60.513 | 49.200 | 1.00 | 37.40 | A | N |
| ATOM | 2049 | CA | ARG | A | 306 | 21.229 | 61.798 | 48.700 | 1.00 | 39.51 | A | C |
| ATOM | 2050 | CB | ARG | A | 306 | 22.759 | 61.797 | 48.525 | 1.00 | 40.32 | A | C |
| ATOM | 2051 | CG | ARG | A | 306 | 23.343 | 60.778 | 47.533 | 1.00 | 43.39 | A | C |
| ATOM | 2052 | CD | ARG | A | 306 | 24.881 | 60.812 | 47.560 | 1.00 | 46.22 | A | C |
| ATOM | 2053 | NE | ARG | A | 306 | 25.501 | 60.466 | 46.275 | 1.00 | 49.82 | A | N |
| ATOM | 2054 | CZ | ARG | A | 306 | 25.957 | 59.258 | 45.935 | 1.00 | 52.51 | A | C |
| ATOM | 2055 | NH1 | ARG | A | 306 | 25.881 | 58.232 | 46.785 | 1.00 | 52.45 | A | N |
| ATOM | 2056 | NH2 | ARG | A | 306 | 26.497 | 59.075 | 44.730 | 1.00 | 52.94 | A | N |
| ATOM | 2057 | C | ARG | A | 306 | 20.524 | 62.125 | 47.395 | 1.00 | 41.07 | A | C |
| ATOM | 2058 | O | ARG | A | 306 | 20.258 | 61.248 | 46.588 | 1.00 | 41.37 | A | O |
| ATOM | 2059 | N | PRO | A | 307 | 20.197 | 63.407 | 47.188 | 1.00 | 41.64 | A | N |
| ATOM | 2060 | CD | PRO | A | 307 | 20.623 | 64.504 | 48.078 | 1.00 | 42.48 | A | C |
| ATOM | 2061 | CA | PRO | A | 307 | 19.510 | 63.942 | 46.009 | 1.00 | 43.02 | A | C |
| ATOM | 2062 | CB | PRO | A | 307 | 19.957 | 65.401 | 46.004 | 1.00 | 42.91 | A | C |
| ATOM | 2063 | CG | PRO | A | 307 | 19.933 | 65.723 | 47.463 | 1.00 | 43.48 | A | C |
| ATOM | 2064 | C | PRO | A | 307 | 19.758 | 63.265 | 44.657 | 1.00 | 43.10 | A | C |
| ATOM | 2065 | O | PRO | A | 307 | 18.804 | 62.788 | 44.016 | 1.00 | 43.71 | A | O |
| ATOM | 2066 | N | ALA | A | 308 | 21.017 | 63.231 | 44.221 | 1.00 | 41.64 | A | N |
| ATOM | 2067 | CA | ALA | A | 308 | 21.357 | 62.652 | 42.919 | 1.00 | 40.52 | A | C |
| ATOM | 2068 | CB | ALA | A | 308 | 22.764 | 63.096 | 42.504 | 1.00 | 41.00 | A | C |
| ATOM | 2069 | C | ALA | A | 308 | 21.239 | 61.130 | 42.791 | 1.00 | 39.22 | A | C |
| ATOM | 2070 | O | ALA | A | 308 | 21.394 | 60.583 | 41.711 | 1.00 | 38.32 | A | O |
| ATOM | 2071 | N | THR | A | 309 | 20.951 | 60.450 | 43.893 | 1.00 | 37.40 | A | N |
| ATOM | 2072 | CA | THR | A | 309 | 20.850 | 58.999 | 43.859 | 1.00 | 36.03 | A | C |
| ATOM | 2073 | CB | THR | A | 309 | 20.291 | 58.457 | 45.171 | 1.00 | 35.60 | A | C |
| ATOM | 2074 | OG1 | THR | A | 309 | 21.128 | 58.887 | 46.250 | 1.00 | 34.01 | A | O |
| ATOM | 2075 | CG2 | THR | A | 309 | 20.257 | 56.936 | 45.138 | 1.00 | 34.95 | A | C |
| ATOM | 2076 | C | THR | A | 309 | 19.959 | 58.525 | 42.722 | 1.00 | 35.20 | A | C |
| ATOM | 2077 | O | THR | A | 309 | 18.897 | 59.051 | 42.503 | 1.00 | 34.87 | A | O |
| ATOM | 2078 | N | PRO | A | 310 | 20.420 | 57.526 | 41.963 | 1.00 | 34.41 | A | N |
| ATOM | 2079 | CD | PRO | A | 310 | 21.783 | 56.980 | 41.926 | 1.00 | 33.93 | A | C |
| ATOM | 2080 | CA | PRO | A | 310 | 19.621 | 57.006 | 40.853 | 1.00 | 34.35 | A | C |
| ATOM | 2081 | CB | PRO | A | 310 | 20.554 | 55.984 | 40.193 | 1.00 | 33.67 | A | C |
| ATOM | 2082 | CG | PRO | A | 310 | 21.551 | 55.661 | 41.266 | 1.00 | 35.69 | A | C |
| ATOM | 2083 | C | PRO | A | 310 | 18.309 | 56.390 | 41.330 | 1.00 | 33.76 | A | C |
| ATOM | 2084 | O | PRO | A | 310 | 18.292 | 55.573 | 42.250 | 1.00 | 34.57 | A | O |

| | | | | | | | | | | | | |
|---|---|---|---|---|---|---|---|---|---|---|---|---|
| ATOM | 2085 | N | PRO | A | 311 | 17.191 | 56.794 | 40.710 | 1.00 | 33.11 | A | N |
| ATOM | 2086 | CD | PRO | A | 311 | 17.146 | 57.764 | 39.602 | 1.00 | 31.68 | A | C |
| ATOM | 2087 | CA | PRO | A | 311 | 15.845 | 56.309 | 41.037 | 1.00 | 31.82 | A | C |
| ATOM | 2088 | CB | PRO | A | 311 | 14.983 | 56.906 | 39.928 | 1.00 | 32.46 | A | C |
| ATOM | 2089 | CG | PRO | A | 311 | 15.696 | 58.170 | 39.588 | 1.00 | 32.92 | A | C |
| ATOM | 2090 | C | PRO | A | 311 | 15.750 | 54.787 | 41.050 | 1.00 | 31.44 | A | C |
| ATOM | 2091 | O | PRO | A | 311 | 15.095 | 54.202 | 41.923 | 1.00 | 31.89 | A | O |
| ATOM | 2092 | N | GLU | A | 312 | 16.393 | 54.144 | 40.075 | 1.00 | 30.47 | A | N |
| ATOM | 2093 | CA | GLU | A | 312 | 16.356 | 52.686 | 39.984 | 1.00 | 30.21 | A | C |
| ATOM | 2094 | CB | GLU | A | 312 | 17.121 | 52.185 | 38.751 | 1.00 | 31.71 | A | C |
| ATOM | 2095 | CG | GLU | A | 312 | 16.671 | 52.726 | 37.402 | 1.00 | 35.62 | A | C |
| ATOM | 2096 | CD | GLU | A | 312 | 17.147 | 54.155 | 37.128 | 1.00 | 39.45 | A | C |
| ATOM | 2097 | OE1 | GLU | A | 312 | 18.120 | 54.624 | 37.771 | 1.00 | 39.22 | A | O |
| ATOM | 2098 | OE2 | GLU | A | 312 | 16.548 | 54.805 | 36.244 | 1.00 | 41.86 | A | O |
| ATOM | 2099 | C | GLU | A | 312 | 16.965 | 52.020 | 41.221 | 1.00 | 29.24 | A | C |
| ATOM | 2100 | O | GLU | A | 312 | 16.528 | 50.952 | 41.635 | 1.00 | 28.83 | A | O |
| ATOM | 2101 | N | ALA | A | 313 | 17.992 | 52.647 | 41.787 | 1.00 | 27.49 | A | N |
| ATOM | 2102 | CA | ALA | A | 313 | 18.649 | 52.110 | 42.973 | 1.00 | 26.21 | A | C |
| ATOM | 2103 | CB | ALA | A | 313 | 19.860 | 52.961 | 43.336 | 1.00 | 24.09 | A | C |
| ATOM | 2104 | C | ALA | A | 313 | 17.657 | 52.115 | 44.121 | 1.00 | 25.84 | A | C |
| ATOM | 2105 | O | ALA | A | 313 | 17.529 | 51.145 | 44.860 | 1.00 | 22.94 | A | O |
| ATOM | 2106 | N | ILE | A | 314 | 16.952 | 53.233 | 44.254 | 1.00 | 27.14 | A | N |
| ATOM | 2107 | CA | ILE | A | 314 | 15.971 | 53.391 | 45.315 | 1.00 | 28.41 | A | C |
| ATOM | 2108 | CB | ILE | A | 314 | 15.453 | 54.841 | 45.353 | 1.00 | 29.47 | A | C |
| ATOM | 2109 | CG2 | ILE | A | 314 | 14.383 | 54.995 | 46.430 | 1.00 | 31.27 | A | C |
| ATOM | 2110 | CG1 | ILE | A | 314 | 16.626 | 55.781 | 45.651 | 1.00 | 30.70 | A | C |
| ATOM | 2111 | CD1 | ILE | A | 314 | 16.291 | 57.244 | 45.534 | 1.00 | 29.93 | A | C |
| ATOM | 2112 | C | ILE | A | 314 | 14.811 | 52.417 | 45.158 | 1.00 | 27.99 | A | C |
| ATOM | 2113 | O | ILE | A | 314 | 14.355 | 51.830 | 46.140 | 1.00 | 27.91 | A | O |
| ATOM | 2114 | N | ALA | A | 315 | 14.357 | 52.242 | 43.921 | 1.00 | 26.99 | A | N |
| ATOM | 2115 | CA | ALA | A | 315 | 13.255 | 51.338 | 43.623 | 1.00 | 27.18 | A | C |
| ATOM | 2116 | CB | ALA | A | 315 | 12.936 | 51.366 | 42.124 | 1.00 | 25.67 | A | C |
| ATOM | 2117 | C | ALA | A | 315 | 13.638 | 49.934 | 44.055 | 1.00 | 26.46 | A | C |
| ATOM | 2118 | O | ALA | A | 315 | 12.860 | 49.243 | 44.695 | 1.00 | 26.99 | A | O |
| ATOM | 2119 | N | LEU | A | 316 | 14.850 | 49.526 | 43.696 | 1.00 | 25.48 | A | N |
| ATOM | 2120 | CA | LEU | A | 316 | 15.347 | 48.203 | 44.056 | 1.00 | 25.14 | A | C |
| ATOM | 2121 | CB | LEU | A | 316 | 16.763 | 48.005 | 43.497 | 1.00 | 22.99 | A | C |
| ATOM | 2122 | CG | LEU | A | 316 | 17.519 | 46.751 | 43.954 | 1.00 | 24.83 | A | C |
| ATOM | 2123 | CD1 | LEU | A | 316 | 16.679 | 45.511 | 43.679 | 1.00 | 22.07 | A | C |
| ATOM | 2124 | CD2 | LEU | A | 316 | 18.861 | 46.664 | 43.235 | 1.00 | 23.72 | A | C |
| ATOM | 2125 | C | LEU | A | 316 | 15.344 | 48.033 | 45.579 | 1.00 | 24.81 | A | C |
| ATOM | 2126 | O | LEU | A | 316 | 14.879 | 47.021 | 46.082 | 1.00 | 25.21 | A | O |
| ATOM | 2127 | N | CYS | A | 317 | 15.851 | 49.030 | 46.303 | 1.00 | 24.47 | A | N |
| ATOM | 2128 | CA | CYS | A | 317 | 15.882 | 48.960 | 47.763 | 1.00 | 26.27 | A | C |
| ATOM | 2129 | CB | CYS | A | 317 | 16.445 | 50.239 | 48.389 | 1.00 | 26.91 | A | C |
| ATOM | 2130 | SG | CYS | A | 317 | 18.232 | 50.414 | 48.359 | 1.00 | 31.13 | A | S |
| ATOM | 2131 | C | CYS | A | 317 | 14.499 | 48.727 | 48.347 | 1.00 | 26.12 | A | C |
| ATOM | 2132 | O | CYS | A | 317 | 14.330 | 47.920 | 49.251 | 1.00 | 26.44 | A | O |
| ATOM | 2133 | N | SER | A | 318 | 13.511 | 49.447 | 47.834 | 1.00 | 25.86 | A | N |
| ATOM | 2134 | CA | SER | A | 318 | 12.159 | 49.293 | 48.349 | 1.00 | 26.58 | A | C |
| ATOM | 2135 | CB | SER | A | 318 | 11.219 | 50.323 | 47.727 | 1.00 | 27.14 | A | C |
| ATOM | 2136 | OG | SER | A | 318 | 10.875 | 49.959 | 46.407 | 1.00 | 30.27 | A | O |
| ATOM | 2137 | C | SER | A | 318 | 11.616 | 47.884 | 48.101 | 1.00 | 26.40 | A | C |
| ATOM | 2138 | O | SER | A | 318 | 10.777 | 47.405 | 48.854 | 1.00 | 26.62 | A | O |
| ATOM | 2139 | N | ARG | A | 319 | 12.106 | 47.217 | 47.057 | 1.00 | 25.73 | A | N |
| ATOM | 2140 | CA | ARG | A | 319 | 11.625 | 45.876 | 46.740 | 1.00 | 25.90 | A | C |
| ATOM | 2141 | CB | ARG | A | 319 | 11.659 | 45.652 | 45.230 | 1.00 | 26.84 | A | C |
| ATOM | 2142 | CG | ARG | A | 319 | 10.806 | 46.647 | 44.479 | 1.00 | 29.32 | A | C |
| ATOM | 2143 | CD | ARG | A | 319 | 9.318 | 46.437 | 44.755 | 1.00 | 31.31 | A | C |
| ATOM | 2144 | NE | ARG | A | 319 | 8.778 | 45.391 | 43.895 | 1.00 | 33.81 | A | N |
| ATOM | 2145 | CZ | ARG | A | 319 | 8.721 | 44.099 | 44.208 | 1.00 | 35.41 | A | C |
| ATOM | 2146 | NH1 | ARG | A | 319 | 9.162 | 43.665 | 45.385 | 1.00 | 36.68 | A | N |
| ATOM | 2147 | NH2 | ARG | A | 319 | 8.249 | 43.233 | 43.324 | 1.00 | 33.23 | A | N |

| | | | | | | | | | | | | |
|---|---|---|---|---|---|---|---|---|---|---|---|---|
| ATOM | 2148 | C | ARG | A | 319 | 12.408 | 44.774 | 47.440 | 1.00 | 26.29 | A | C |
| ATOM | 2149 | O | ARG | A | 319 | 12.092 | 43.581 | 47.309 | 1.00 | 25.97 | A | O |
| ATOM | 2150 | N | LEU | A | 320 | 13.430 | 45.178 | 48.183 | 1.00 | 25.71 | A | N |
| ATOM | 2151 | CA | LEU | A | 320 | 14.261 | 44.240 | 48.920 | 1.00 | 24.46 | A | C |
| ATOM | 2152 | CB | LEU | A | 320 | 15.747 | 44.546 | 48.698 | 1.00 | 23.01 | A | C |
| ATOM | 2153 | CG | LEU | A | 320 | 16.315 | 44.482 | 47.276 | 1.00 | 21.96 | A | C |
| ATOM | 2154 | CD1 | LEU | A | 320 | 17.795 | 44.819 | 47.313 | 1.00 | 20.47 | A | C |
| ATOM | 2155 | CD2 | LEU | A | 320 | 16.112 | 43.091 | 46.686 | 1.00 | 21.59 | A | C |
| ATOM | 2156 | C | LEU | A | 320 | 13.935 | 44.342 | 50.401 | 1.00 | 24.05 | A | C |
| ATOM | 2157 | O | LEU | A | 320 | 13.678 | 43.352 | 51.057 | 1.00 | 24.07 | A | O |
| ATOM | 2158 | N | LEU | A | 321 | 13.949 | 45.567 | 50.909 | 1.00 | 24.89 | A | N |
| ATOM | 2159 | CA | LEU | A | 321 | 13.671 | 45.832 | 52.312 | 1.00 | 24.70 | A | C |
| ATOM | 2160 | CB | LEU | A | 321 | 14.386 | 47.125 | 52.717 | 1.00 | 23.75 | A | C |
| ATOM | 2161 | CG | LEU | A | 321 | 15.895 | 47.053 | 52.425 | 1.00 | 22.70 | A | C |
| ATOM | 2162 | CD1 | LEU | A | 321 | 16.557 | 48.400 | 52.672 | 1.00 | 22.21 | A | C |
| ATOM | 2163 | CD2 | LEU | A | 321 | 16.535 | 45.970 | 53.295 | 1.00 | 20.58 | A | C |
| ATOM | 2164 | C | LEU | A | 321 | 12.158 | 45.917 | 52.548 | 1.00 | 25.11 | A | C |
| ATOM | 2165 | O | LEU | A | 321 | 11.589 | 46.989 | 52.643 | 1.00 | 26.40 | A | O |
| ATOM | 2166 | N | GLU | A | 322 | 11.518 | 44.758 | 52.626 | 1.00 | 24.72 | A | N |
| ATOM | 2167 | CA | GLU | A | 322 | 10.080 | 44.699 | 52.842 | 1.00 | 26.51 | A | C |
| ATOM | 2168 | CB | GLU | A | 322 | 9.372 | 44.148 | 51.603 | 1.00 | 26.96 | A | C |
| ATOM | 2169 | CG | GLU | A | 322 | 9.688 | 44.889 | 50.325 | 1.00 | 31.52 | A | C |
| ATOM | 2170 | CD | GLU | A | 322 | 8.440 | 45.341 | 49.597 | 1.00 | 33.86 | A | C |
| ATOM | 2171 | OE1 | GLU | A | 322 | 7.669 | 46.128 | 50.191 | 1.00 | 36.19 | A | O |
| ATOM | 2172 | OE2 | GLU | A | 322 | 8.231 | 44.912 | 48.438 | 1.00 | 33.19 | A | O |
| ATOM | 2173 | C | GLU | A | 322 | 9.752 | 43.801 | 54.031 | 1.00 | 26.06 | A | C |
| ATOM | 2174 | O | GLU | A | 322 | 10.362 | 42.762 | 54.209 | 1.00 | 26.23 | A | O |
| ATOM | 2175 | N | TYR | A | 323 | 8.777 | 44.215 | 54.833 | 1.00 | 26.28 | A | N |
| ATOM | 2176 | CA | TYR | A | 323 | 8.376 | 43.433 | 55.997 | 1.00 | 25.93 | A | C |
| ATOM | 2177 | CB | TYR | A | 323 | 7.226 | 44.122 | 56.738 | 1.00 | 25.73 | A | C |
| ATOM | 2178 | CG | TYR | A | 323 | 7.656 | 45.267 | 57.620 | 1.00 | 24.57 | A | C |
| ATOM | 2179 | CD1 | TYR | A | 323 | 8.569 | 45.071 | 58.658 | 1.00 | 23.90 | A | C |
| ATOM | 2180 | CE1 | TYR | A | 323 | 8.944 | 46.118 | 59.492 | 1.00 | 24.02 | A | C |
| ATOM | 2181 | CD2 | TYR | A | 323 | 7.133 | 46.543 | 57.433 | 1.00 | 23.87 | A | C |
| ATOM | 2182 | CE2 | TYR | A | 323 | 7.500 | 47.594 | 58.256 | 1.00 | 24.56 | A | C |
| ATOM | 2183 | CZ | TYR | A | 323 | 8.405 | 47.379 | 59.284 | 1.00 | 25.67 | A | C |
| ATOM | 2184 | OH | TYR | A | 323 | 8.754 | 48.433 | 60.096 | 1.00 | 25.93 | A | O |
| ATOM | 2185 | C | TYR | A | 323 | 7.948 | 42.023 | 55.589 | 1.00 | 25.13 | A | C |
| ATOM | 2186 | O | TYR | A | 323 | 8.484 | 41.021 | 56.087 | 1.00 | 24.40 | A | O |
| ATOM | 2187 | N | THR | A | 324 | 6.984 | 41.947 | 54.684 | 1.00 | 24.41 | A | N |
| ATOM | 2188 | CA | THR | A | 324 | 6.504 | 40.657 | 54.218 | 1.00 | 24.91 | A | C |
| ATOM | 2189 | CB | THR | A | 324 | 5.310 | 40.828 | 53.251 | 1.00 | 25.79 | A | C |
| ATOM | 2190 | OG1 | THR | A | 324 | 4.277 | 41.583 | 53.899 | 1.00 | 25.90 | A | O |
| ATOM | 2191 | CG2 | THR | A | 324 | 4.750 | 39.473 | 52.849 | 1.00 | 25.36 | A | C |
| ATOM | 2192 | C | THR | A | 324 | 7.663 | 39.973 | 53.507 | 1.00 | 23.59 | A | C |
| ATOM | 2193 | O | THR | A | 324 | 8.095 | 40.412 | 52.474 | 1.00 | 24.84 | A | O |
| ATOM | 2194 | N | PRO | A | 325 | 8.183 | 38.886 | 54.088 | 1.00 | 24.10 | A | N |
| ATOM | 2195 | CD | PRO | A | 325 | 7.739 | 38.253 | 55.344 | 1.00 | 23.78 | A | C |
| ATOM | 2196 | CA | PRO | A | 325 | 9.305 | 38.147 | 53.509 | 1.00 | 24.48 | A | C |
| ATOM | 2197 | CB | PRO | A | 325 | 9.487 | 36.982 | 54.479 | 1.00 | 24.85 | A | C |
| ATOM | 2198 | CG | PRO | A | 325 | 8.970 | 37.532 | 55.782 | 1.00 | 23.36 | A | C |
| ATOM | 2199 | C | PRO | A | 325 | 9.053 | 37.675 | 52.088 | 1.00 | 25.66 | A | C |
| ATOM | 2200 | O | PRO | A | 325 | 9.934 | 37.735 | 51.247 | 1.00 | 24.22 | A | O |
| ATOM | 2201 | N | THR | A | 326 | 7.830 | 37.214 | 51.844 | 1.00 | 27.02 | A | N |
| ATOM | 2202 | CA | THR | A | 326 | 7.419 | 36.689 | 50.550 | 1.00 | 26.74 | A | C |
| ATOM | 2203 | CB | THR | A | 326 | 6.085 | 35.927 | 50.695 | 1.00 | 29.59 | A | C |
| ATOM | 2204 | OG1 | THR | A | 326 | 5.840 | 35.157 | 49.515 | 1.00 | 33.02 | A | O |
| ATOM | 2205 | CG2 | THR | A | 326 | 4.935 | 36.902 | 50.904 | 1.00 | 27.84 | A | C |
| ATOM | 2206 | C | THR | A | 326 | 7.271 | 37.776 | 49.481 | 1.00 | 26.97 | A | C |
| ATOM | 2207 | O | THR | A | 326 | 7.229 | 37.483 | 48.284 | 1.00 | 25.81 | A | O |
| ATOM | 2208 | N | ALA | A | 327 | 7.189 | 39.028 | 49.911 | 1.00 | 25.61 | A | N |
| ATOM | 2209 | CA | ALA | A | 327 | 7.038 | 40.133 | 48.968 | 1.00 | 25.41 | A | C |
| ATOM | 2210 | CB | ALA | A | 327 | 6.340 | 41.304 | 49.635 | 1.00 | 24.03 | A | C |

| | | | | | | | | | | | | |
|---|---|---|---|---|---|---|---|---|---|---|---|---|
| ATOM | 2211 | C | ALA | A | 327 | 8.391 | 40.580 | 48.410 | 1.00 | 25.75 | A | C |
| ATOM | 2212 | O | ALA | A | 327 | 8.469 | 41.178 | 47.340 | 1.00 | 25.62 | A | O |
| ATOM | 2213 | N | ARG | A | 328 | 9.452 | 40.279 | 49.143 | 1.00 | 24.18 | A | N |
| ATOM | 2214 | CA | ARG | A | 328 | 10.785 | 40.655 | 48.718 | 1.00 | 24.33 | A | C |
| ATOM | 2215 | CB | ARG | A | 328 | 11.796 | 40.237 | 49.781 | 1.00 | 21.44 | A | C |
| ATOM | 2216 | CG | ARG | A | 328 | 11.505 | 40.847 | 51.136 | 1.00 | 22.88 | A | C |
| ATOM | 2217 | CD | ARG | A | 328 | 12.340 | 40.219 | 52.224 | 1.00 | 20.95 | A | C |
| ATOM | 2218 | NE | ARG | A | 328 | 11.873 | 40.630 | 53.545 | 1.00 | 22.65 | A | N |
| ATOM | 2219 | CZ | ARG | A | 328 | 12.177 | 40.002 | 54.674 | 1.00 | 20.63 | A | C |
| ATOM | 2220 | NH1 | ARG | A | 328 | 12.951 | 38.925 | 54.654 | 1.00 | 18.03 | A | N |
| ATOM | 2221 | NH2 | ARG | A | 328 | 11.706 | 40.456 | 55.823 | 1.00 | 20.79 | A | N |
| ATOM | 2222 | C | ARG | A | 328 | 11.138 | 40.009 | 47.381 | 1.00 | 24.96 | A | C |
| ATOM | 2223 | O | ARG | A | 328 | 10.677 | 38.933 | 47.061 | 1.00 | 26.89 | A | O |
| ATOM | 2224 | N | LEU | A | 329 | 11.957 | 40.696 | 46.604 | 1.00 | 23.72 | A | N |
| ATOM | 2225 | CA | LEU | A | 329 | 12.387 | 40.181 | 45.319 | 1.00 | 22.62 | A | C |
| ATOM | 2226 | CB | LEU | A | 329 | 13.236 | 41.224 | 44.607 | 1.00 | 22.13 | A | C |
| ATOM | 2227 | CG | LEU | A | 329 | 12.734 | 41.968 | 43.378 | 1.00 | 23.10 | A | C |
| ATOM | 2228 | CD1 | LEU | A | 329 | 11.279 | 42.346 | 43.497 | 1.00 | 24.14 | A | C |
| ATOM | 2229 | CD2 | LEU | A | 329 | 13.615 | 43.197 | 43.213 | 1.00 | 23.03 | A | C |
| ATOM | 2230 | C | LEU | A | 329 | 13.240 | 38.953 | 45.553 | 1.00 | 22.27 | A | C |
| ATOM | 2231 | O | LEU | A | 329 | 13.847 | 38.805 | 46.616 | 1.00 | 19.94 | A | O |
| ATOM | 2232 | N | THR | A | 330 | 13.282 | 38.072 | 44.561 | 1.00 | 21.28 | A | N |
| ATOM | 2233 | CA | THR | A | 330 | 14.124 | 36.897 | 44.667 | 1.00 | 22.23 | A | C |
| ATOM | 2234 | CB | THR | A | 330 | 13.608 | 35.714 | 43.838 | 1.00 | 23.51 | A | C |
| ATOM | 2235 | OG1 | THR | A | 330 | 13.637 | 36.051 | 42.448 | 1.00 | 24.62 | A | O |
| ATOM | 2236 | CG2 | THR | A | 330 | 12.181 | 35.351 | 44.252 | 1.00 | 24.27 | A | C |
| ATOM | 2237 | C | THR | A | 330 | 15.474 | 37.336 | 44.114 | 1.00 | 22.58 | A | C |
| ATOM | 2238 | O | THR | A | 330 | 15.557 | 38.316 | 43.393 | 1.00 | 22.85 | A | O |
| ATOM | 2239 | N | PRO | A | 331 | 16.553 | 36.633 | 44.476 | 1.00 | 23.47 | A | N |
| ATOM | 2240 | CD | PRO | A | 331 | 16.679 | 35.568 | 45.484 | 1.00 | 21.96 | A | C |
| ATOM | 2241 | CA | PRO | A | 331 | 17.864 | 37.020 | 43.959 | 1.00 | 23.24 | A | C |
| ATOM | 2242 | CB | PRO | A | 331 | 18.765 | 35.897 | 44.454 | 1.00 | 23.79 | A | C |
| ATOM | 2243 | CG | PRO | A | 331 | 18.156 | 35.579 | 45.783 | 1.00 | 22.46 | A | C |
| ATOM | 2244 | C | PRO | A | 331 | 17.852 | 37.135 | 42.428 | 1.00 | 23.13 | A | C |
| ATOM | 2245 | O | PRO | A | 331 | 18.316 | 38.123 | 41.885 | 1.00 | 22.39 | A | O |
| ATOM | 2246 | N | LEU | A | 332 | 17.308 | 36.131 | 41.738 | 1.00 | 24.17 | A | N |
| ATOM | 2247 | CA | LEU | A | 332 | 17.260 | 36.185 | 40.275 | 1.00 | 23.38 | A | C |
| ATOM | 2248 | CB | LEU | A | 332 | 16.604 | 34.945 | 39.677 | 1.00 | 24.20 | A | C |
| ATOM | 2249 | CG | LEU | A | 332 | 17.487 | 33.868 | 39.040 | 1.00 | 24.72 | A | C |
| ATOM | 2250 | CD1 | LEU | A | 332 | 16.587 | 32.990 | 38.178 | 1.00 | 24.97 | A | C |
| ATOM | 2251 | CD2 | LEU | A | 332 | 18.590 | 34.472 | 38.184 | 1.00 | 23.04 | A | C |
| ATOM | 2252 | C | LEU | A | 332 | 16.486 | 37.401 | 39.800 | 1.00 | 24.53 | A | C |
| ATOM | 2253 | O | LEU | A | 332 | 16.924 | 38.107 | 38.898 | 1.00 | 24.59 | A | O |
| ATOM | 2254 | N | GLU | A | 333 | 15.320 | 37.630 | 40.399 | 1.00 | 25.02 | A | N |
| ATOM | 2255 | CA | GLU | A | 333 | 14.499 | 38.782 | 40.035 | 1.00 | 24.40 | A | C |
| ATOM | 2256 | CB | GLU | A | 333 | 13.221 | 38.823 | 40.880 | 1.00 | 24.55 | A | C |
| ATOM | 2257 | CG | GLU | A | 333 | 12.193 | 37.765 | 40.491 | 1.00 | 24.74 | A | C |
| ATOM | 2258 | CD | GLU | A | 333 | 11.093 | 37.596 | 41.530 | 1.00 | 27.07 | A | C |
| ATOM | 2259 | OE1 | GLU | A | 333 | 11.114 | 38.318 | 42.547 | 1.00 | 25.88 | A | O |
| ATOM | 2260 | OE2 | GLU | A | 333 | 10.208 | 36.733 | 41.335 | 1.00 | 29.21 | A | O |
| ATOM | 2261 | C | GLU | A | 333 | 15.304 | 40.068 | 40.223 | 1.00 | 23.78 | A | C |
| ATOM | 2262 | O | GLU | A | 333 | 15.290 | 40.944 | 39.360 | 1.00 | 22.76 | A | O |
| ATOM | 2263 | N | ALA | A | 334 | 16.009 | 40.161 | 41.348 | 1.00 | 22.05 | A | N |
| ATOM | 2264 | CA | ALA | A | 334 | 16.836 | 41.329 | 41.636 | 1.00 | 21.29 | A | C |
| ATOM | 2265 | CB | ALA | A | 334 | 17.581 | 41.151 | 42.970 | 1.00 | 18.46 | A | C |
| ATOM | 2266 | C | ALA | A | 334 | 17.831 | 41.512 | 40.492 | 1.00 | 20.08 | A | C |
| ATOM | 2267 | O | ALA | A | 334 | 18.002 | 42.600 | 40.001 | 1.00 | 21.09 | A | O |
| ATOM | 2268 | N | CYS | A | 335 | 18.474 | 40.423 | 40.082 | 1.00 | 19.66 | A | N |
| ATOM | 2269 | CA | CYS | A | 335 | 19.433 | 40.465 | 38.992 | 1.00 | 21.00 | A | C |
| ATOM | 2270 | CB | CYS | A | 335 | 19.952 | 39.063 | 38.677 | 1.00 | 21.69 | A | C |
| ATOM | 2271 | SG | CYS | A | 335 | 21.350 | 38.518 | 39.685 | 1.00 | 21.88 | A | S |
| ATOM | 2272 | C | CYS | A | 335 | 18.828 | 41.063 | 37.726 | 1.00 | 22.43 | A | C |
| ATOM | 2273 | O | CYS | A | 335 | 19.531 | 41.686 | 36.952 | 1.00 | 22.19 | A | O |

| | | | | | | | | | | | | |
|---|---|---|---|---|---|---|---|---|---|---|---|---|
| ATOM | 2274 | N | ALA | A | 336 | 17.524 | 40.865 | 37.540 | 1.00 | 23.65 | A | N |
| ATOM | 2275 | CA | ALA | A | 336 | 16.815 | 41.361 | 36.366 | 1.00 | 24.08 | A | C |
| ATOM | 2276 | CB | ALA | A | 336 | 15.651 | 40.441 | 36.050 | 1.00 | 22.39 | A | C |
| ATOM | 2277 | C | ALA | A | 336 | 16.304 | 42.790 | 36.526 | 1.00 | 24.92 | A | C |
| ATOM | 2278 | O | ALA | A | 336 | 15.799 | 43.355 | 35.591 | 1.00 | 24.50 | A | O |
| ATOM | 2279 | N | HIS | A | 337 | 16.443 | 43.363 | 37.719 | 1.00 | 25.99 | A | N |
| ATOM | 2280 | CA | HIS | A | 337 | 15.967 | 44.723 | 37.972 | 1.00 | 25.61 | A | C |
| ATOM | 2281 | CB | HIS | A | 337 | 16.251 | 45.132 | 39.424 | 1.00 | 25.29 | A | C |
| ATOM | 2282 | CG | HIS | A | 337 | 15.482 | 46.336 | 39.875 | 1.00 | 25.53 | A | C |
| ATOM | 2283 | CD2 | HIS | A | 337 | 15.797 | 47.653 | 39.866 | 1.00 | 24.56 | A | C |
| ATOM | 2284 | ND1 | HIS | A | 337 | 14.210 | 46.254 | 40.401 | 1.00 | 26.12 | A | N |
| ATOM | 2285 | CE1 | HIS | A | 337 | 13.777 | 47.468 | 40.698 | 1.00 | 23.20 | A | C |
| ATOM | 2286 | NE2 | HIS | A | 337 | 14.721 | 48.334 | 40.383 | 1.00 | 23.05 | A | N |
| ATOM | 2287 | C | HIS | A | 337 | 16.604 | 45.739 | 37.030 | 1.00 | 26.29 | A | C |
| ATOM | 2288 | O | HIS | A | 337 | 17.750 | 45.596 | 36.644 | 1.00 | 26.04 | A | O |
| ATOM | 2289 | N | SER | A | 338 | 15.849 | 46.775 | 36.680 | 1.00 | 27.22 | A | N |
| ATOM | 2290 | CA | SER | A | 338 | 16.345 | 47.794 | 35.762 | 1.00 | 28.25 | A | C |
| ATOM | 2291 | CB | SER | A | 338 | 15.250 | 48.832 | 35.477 | 1.00 | 29.56 | A | C |
| ATOM | 2292 | OG | SER | A | 338 | 15.004 | 49.634 | 36.618 | 1.00 | 34.72 | A | O |
| ATOM | 2293 | C | SER | A | 338 | 17.604 | 48.491 | 36.272 | 1.00 | 27.07 | A | C |
| ATOM | 2294 | O | SER | A | 338 | 18.355 | 49.042 | 35.484 | 1.00 | 27.12 | A | O |
| ATOM | 2295 | N | PHE | A | 339 | 17.834 | 48.454 | 37.584 | 1.00 | 25.78 | A | N |
| ATOM | 2296 | CA | PHE | A | 339 | 19.020 | 49.083 | 38.166 | 1.00 | 24.26 | A | C |
| ATOM | 2297 | CB | PHE | A | 339 | 19.034 | 48.894 | 39.691 | 1.00 | 25.56 | A | C |
| ATOM | 2298 | CG | PHE | A | 339 | 20.268 | 49.449 | 40.368 | 1.00 | 24.73 | A | C |
| ATOM | 2299 | CD1 | PHE | A | 339 | 20.611 | 50.790 | 40.229 | 1.00 | 24.19 | A | C |
| ATOM | 2300 | CD2 | PHE | A | 339 | 21.087 | 48.628 | 41.140 | 1.00 | 25.29 | A | C |
| ATOM | 2301 | CE1 | PHE | A | 339 | 21.751 | 51.306 | 40.847 | 1.00 | 23.85 | A | C |
| ATOM | 2302 | CE2 | PHE | A | 339 | 22.236 | 49.140 | 41.764 | 1.00 | 22.59 | A | C |
| ATOM | 2303 | CZ | PHE | A | 339 | 22.563 | 50.477 | 41.615 | 1.00 | 22.17 | A | C |
| ATOM | 2304 | C | PHE | A | 339 | 20.297 | 48.493 | 37.577 | 1.00 | 23.16 | A | C |
| ATOM | 2305 | O | PHE | A | 339 | 21.330 | 49.144 | 37.554 | 1.00 | 22.05 | A | O |
| ATOM | 2306 | N | PHE | A | 340 | 20.212 | 47.256 | 37.098 | 1.00 | 23.05 | A | N |
| ATOM | 2307 | CA | PHE | A | 340 | 21.369 | 46.584 | 36.516 | 1.00 | 24.33 | A | C |
| ATOM | 2308 | CB | PHE | A | 340 | 21.423 | 45.124 | 36.984 | 1.00 | 21.25 | A | C |
| ATOM | 2309 | CG | PHE | A | 340 | 21.501 | 44.964 | 38.476 | 1.00 | 20.77 | A | C |
| ATOM | 2310 | CD1 | PHE | A | 340 | 22.608 | 45.428 | 39.185 | 1.00 | 20.07 | A | C |
| ATOM | 2311 | CD2 | PHE | A | 340 | 20.472 | 44.347 | 39.175 | 1.00 | 18.91 | A | C |
| ATOM | 2312 | CE1 | PHE | A | 340 | 22.688 | 45.278 | 40.567 | 1.00 | 19.80 | A | C |
| ATOM | 2313 | CE2 | PHE | A | 340 | 20.541 | 44.191 | 40.553 | 1.00 | 21.51 | A | C |
| ATOM | 2314 | CZ | PHE | A | 340 | 21.659 | 44.662 | 41.253 | 1.00 | 21.21 | A | C |
| ATOM | 2315 | C | PHE | A | 340 | 21.380 | 46.617 | 34.989 | 1.00 | 24.66 | A | C |
| ATOM | 2316 | O | PHE | A | 340 | 22.153 | 45.894 | 34.361 | 1.00 | 26.20 | A | O |
| ATOM | 2317 | N | ASP | A | 341 | 20.535 | 47.441 | 34.379 | 1.00 | 24.72 | A | N |
| ATOM | 2318 | CA | ASP | A | 341 | 20.512 | 47.499 | 32.912 | 1.00 | 26.03 | A | C |
| ATOM | 2319 | CB | ASP | A | 341 | 19.410 | 48.438 | 32.415 | 1.00 | 25.59 | A | C |
| ATOM | 2320 | CG | ASP | A | 341 | 18.022 | 47.882 | 32.655 | 1.00 | 27.02 | A | C |
| ATOM | 2321 | OD1 | ASP | A | 341 | 17.887 | 46.664 | 32.901 | 1.00 | 27.88 | A | O |
| ATOM | 2322 | OD2 | ASP | A | 341 | 17.057 | 48.660 | 32.589 | 1.00 | 29.89 | A | O |
| ATOM | 2323 | C | ASP | A | 341 | 21.854 | 47.916 | 32.312 | 1.00 | 25.52 | A | C |
| ATOM | 2324 | O | ASP | A | 341 | 22.270 | 47.381 | 31.285 | 1.00 | 25.27 | A | O |
| ATOM | 2325 | N | GLU | A | 342 | 22.531 | 48.856 | 32.966 | 1.00 | 24.57 | A | N |
| ATOM | 2326 | CA | GLU | A | 342 | 23.820 | 49.332 | 32.487 | 1.00 | 26.48 | A | C |
| ATOM | 2327 | CB | GLU | A | 342 | 24.394 | 50.358 | 33.456 | 1.00 | 27.29 | A | C |
| ATOM | 2328 | CG | GLU | A | 342 | 25.388 | 51.307 | 32.811 | 1.00 | 31.64 | A | C |
| ATOM | 2329 | CD | GLU | A | 342 | 26.154 | 52.139 | 33.824 | 1.00 | 32.83 | A | C |
| ATOM | 2330 | OE1 | GLU | A | 342 | 25.526 | 52.696 | 34.752 | 1.00 | 34.69 | A | O |
| ATOM | 2331 | OE2 | GLU | A | 342 | 27.390 | 52.241 | 33.682 | 1.00 | 34.17 | A | O |
| ATOM | 2332 | C | GLU | A | 342 | 24.822 | 48.183 | 32.319 | 1.00 | 26.14 | A | C |
| ATOM | 2333 | O | GLU | A | 342 | 25.624 | 48.182 | 31.397 | 1.00 | 25.00 | A | O |
| ATOM | 2334 | N | LEU | A | 343 | 24.763 | 47.207 | 33.219 | 1.00 | 25.71 | A | N |
| ATOM | 2335 | CA | LEU | A | 343 | 25.673 | 46.077 | 33.157 | 1.00 | 25.67 | A | C |
| ATOM | 2336 | CB | LEU | A | 343 | 25.533 | 45.205 | 34.411 | 1.00 | 24.21 | A | C |

| | | | | | | | | | | | | |
|---|---|---|---|---|---|---|---|---|---|---|---|---|
| ATOM | 2337 | CG | LEU | A | 343 | 25.755 | 45.878 | 35.765 | 1.00 | 25.39 | A | C |
| ATOM | 2338 | CD1 | LEU | A | 343 | 25.643 | 44.840 | 36.873 | 1.00 | 21.34 | A | C |
| ATOM | 2339 | CD2 | LEU | A | 343 | 27.130 | 46.554 | 35.786 | 1.00 | 23.56 | A | C |
| ATOM | 2340 | C | LEU | A | 343 | 25.389 | 45.229 | 31.931 | 1.00 | 24.73 | A | C |
| ATOM | 2341 | O | LEU | A | 343 | 26.234 | 44.511 | 31.489 | 1.00 | 25.16 | A | O |
| ATOM | 2342 | N | ARG | A | 344 | 24.176 | 45.320 | 31.399 | 1.00 | 26.15 | A | N |
| ATOM | 2343 | CA | ARG | A | 344 | 23.806 | 44.538 | 30.223 | 1.00 | 28.53 | A | C |
| ATOM | 2344 | CB | ARG | A | 344 | 22.325 | 44.149 | 30.297 | 1.00 | 27.72 | A | C |
| ATOM | 2345 | CG | ARG | A | 344 | 22.046 | 43.012 | 31.281 | 1.00 | 26.01 | A | C |
| ATOM | 2346 | CD | ARG | A | 344 | 20.563 | 42.668 | 31.344 | 1.00 | 25.58 | A | C |
| ATOM | 2347 | NE | ARG | A | 344 | 19.802 | 43.619 | 32.149 | 1.00 | 26.26 | A | N |
| ATOM | 2348 | CZ | ARG | A | 344 | 19.619 | 43.524 | 33.463 | 1.00 | 26.67 | A | C |
| ATOM | 2349 | NH1 | ARG | A | 344 | 20.143 | 42.509 | 34.138 | 1.00 | 24.77 | A | N |
| ATOM | 2350 | NH2 | ARG | A | 344 | 18.911 | 44.451 | 34.104 | 1.00 | 26.42 | A | N |
| ATOM | 2351 | C | ARG | A | 344 | 24.104 | 45.260 | 28.912 | 1.00 | 29.77 | A | C |
| ATOM | 2352 | O | ARG | A | 344 | 24.013 | 44.682 | 27.848 | 1.00 | 29.60 | A | O |
| ATOM | 2353 | N | ASP | A | 345 | 24.460 | 46.535 | 29.012 | 1.00 | 31.85 | A | N |
| ATOM | 2354 | CA | ASP | A | 345 | 24.803 | 47.333 | 27.846 | 1.00 | 32.99 | A | C |
| ATOM | 2355 | CB | ASP | A | 345 | 25.095 | 48.772 | 28.271 | 1.00 | 35.59 | A | C |
| ATOM | 2356 | CG | ASP | A | 345 | 25.313 | 49.698 | 27.093 | 1.00 | 37.69 | A | C |
| ATOM | 2357 | OD1 | ASP | A | 345 | 26.188 | 49.397 | 26.245 | 1.00 | 37.99 | A | O |
| ATOM | 2358 | OD2 | ASP | A | 345 | 24.610 | 50.730 | 27.022 | 1.00 | 37.09 | A | O |
| ATOM | 2359 | C | ASP | A | 345 | 26.058 | 46.700 | 27.256 | 1.00 | 33.00 | A | C |
| ATOM | 2360 | O | ASP | A | 345 | 26.990 | 46.403 | 27.974 | 1.00 | 32.36 | A | O |
| ATOM | 2361 | N | PRO | A | 346 | 26.082 | 46.479 | 25.933 | 1.00 | 33.50 | A | N |
| ATOM | 2362 | CD | PRO | A | 346 | 25.039 | 46.802 | 24.945 | 1.00 | 33.43 | A | C |
| ATOM | 2363 | CA | PRO | A | 346 | 27.249 | 45.874 | 25.283 | 1.00 | 33.90 | A | C |
| ATOM | 2364 | CB | PRO | A | 346 | 26.762 | 45.653 | 23.854 | 1.00 | 34.78 | A | C |
| ATOM | 2365 | CG | PRO | A | 346 | 25.825 | 46.812 | 23.649 | 1.00 | 34.54 | A | C |
| ATOM | 2366 | C | PRO | A | 346 | 28.514 | 46.730 | 25.346 | 1.00 | 34.67 | A | C |
| ATOM | 2367 | O | PRO | A | 346 | 29.612 | 46.201 | 25.305 | 1.00 | 34.73 | A | O |
| ATOM | 2368 | N | ASN | A | 347 | 28.353 | 48.044 | 25.465 | 1.00 | 34.66 | A | N |
| ATOM | 2369 | CA | ASN | A | 347 | 29.508 | 48.938 | 25.520 | 1.00 | 37.48 | A | C |
| ATOM | 2370 | CB | ASN | A | 347 | 29.134 | 50.276 | 24.875 | 1.00 | 38.16 | A | C |
| ATOM | 2371 | CG | ASN | A | 347 | 28.557 | 50.094 | 23.485 | 1.00 | 40.41 | A | C |
| ATOM | 2372 | OD1 | ASN | A | 347 | 27.454 | 50.604 | 23.170 | 1.00 | 39.94 | A | O |
| ATOM | 2373 | ND2 | ASN | A | 347 | 29.278 | 49.348 | 22.642 | 1.00 | 39.73 | A | N |
| ATOM | 2374 | C | ASN | A | 347 | 30.089 | 49.173 | 26.911 | 1.00 | 37.25 | A | C |
| ATOM | 2375 | O | ASN | A | 347 | 31.199 | 49.695 | 27.043 | 1.00 | 37.97 | A | O |
| ATOM | 2376 | N | VAL | A | 348 | 29.357 | 48.768 | 27.944 | 1.00 | 37.04 | A | N |
| ATOM | 2377 | CA | VAL | A | 348 | 29.818 | 48.981 | 29.309 | 1.00 | 36.37 | A | C |
| ATOM | 2378 | CB | VAL | A | 348 | 28.824 | 48.397 | 30.344 | 1.00 | 34.79 | A | C |
| ATOM | 2379 | CG1 | VAL | A | 348 | 28.889 | 46.869 | 30.357 | 1.00 | 31.47 | A | C |
| ATOM | 2380 | CG2 | VAL | A | 348 | 29.126 | 48.974 | 31.721 | 1.00 | 33.15 | A | C |
| ATOM | 2381 | C | VAL | A | 348 | 31.206 | 48.427 | 29.599 | 1.00 | 37.06 | A | C |
| ATOM | 2382 | O | VAL | A | 348 | 31.543 | 47.316 | 29.225 | 1.00 | 37.25 | A | O |
| ATOM | 2383 | N | LYS | A | 349 | 32.005 | 49.248 | 30.270 | 1.00 | 39.24 | A | N |
| ATOM | 2384 | CA | LYS | A | 349 | 33.353 | 48.886 | 30.663 | 1.00 | 40.12 | A | C |
| ATOM | 2385 | CB | LYS | A | 349 | 34.366 | 49.411 | 29.642 | 1.00 | 43.03 | A | C |
| ATOM | 2386 | CG | LYS | A | 349 | 34.313 | 48.660 | 28.302 | 1.00 | 45.52 | A | C |
| ATOM | 2387 | CD | LYS | A | 349 | 35.354 | 49.179 | 27.310 | 1.00 | 47.88 | A | C |
| ATOM | 2388 | CE | LYS | A | 349 | 36.781 | 48.819 | 27.739 | 1.00 | 47.89 | A | C |
| ATOM | 2389 | NZ | LYS | A | 349 | 37.807 | 49.359 | 26.774 | 1.00 | 47.26 | A | N |
| ATOM | 2390 | C | LYS | A | 349 | 33.610 | 49.450 | 32.049 | 1.00 | 40.40 | A | C |
| ATOM | 2391 | O | LYS | A | 349 | 32.809 | 50.206 | 32.560 | 1.00 | 39.36 | A | O |
| ATOM | 2392 | N | LEU | A | 350 | 34.717 | 49.048 | 32.665 | 1.00 | 41.08 | A | N |
| ATOM | 2393 | CA | LEU | A | 350 | 35.062 | 49.548 | 33.985 | 1.00 | 41.94 | A | C |
| ATOM | 2394 | CB | LEU | A | 350 | 35.971 | 48.559 | 34.714 | 1.00 | 41.32 | A | C |
| ATOM | 2395 | CG | LEU | A | 350 | 35.421 | 47.143 | 34.889 | 1.00 | 40.27 | A | C |
| ATOM | 2396 | CD1 | LEU | A | 350 | 36.355 | 46.348 | 35.757 | 1.00 | 40.05 | A | C |
| ATOM | 2397 | CD2 | LEU | A | 350 | 34.050 | 47.198 | 35.512 | 1.00 | 39.52 | A | C |
| ATOM | 2398 | C | LEU | A | 350 | 35.790 | 50.871 | 33.802 | 1.00 | 43.68 | A | C |
| ATOM | 2399 | O | LEU | A | 350 | 36.382 | 51.112 | 32.758 | 1.00 | 43.58 | A | O |

| | | | | | | | | | | | | |
|---|---|---|---|---|---|---|---|---|---|---|---|---|
| ATOM | 2400 | N | PRO | A | 351 | 35.719 | 51.763 | 34.804 | 1.00 | 45.55 | A | N |
| ATOM | 2401 | CD | PRO | A | 351 | 35.186 | 51.594 | 36.165 | 1.00 | 45.78 | A | C |
| ATOM | 2402 | CA | PRO | A | 351 | 36.423 | 53.043 | 34.664 | 1.00 | 46.61 | A | C |
| ATOM | 2403 | CB | PRO | A | 351 | 36.233 | 53.697 | 36.035 | 1.00 | 46.23 | A | C |
| ATOM | 2404 | CG | PRO | A | 351 | 36.082 | 52.516 | 36.960 | 1.00 | 46.86 | A | C |
| ATOM | 2405 | C | PRO | A | 351 | 37.861 | 52.624 | 34.379 | 1.00 | 47.59 | A | C |
| ATOM | 2406 | O | PRO | A | 351 | 38.558 | 53.212 | 33.576 | 1.00 | 47.54 | A | O |
| ATOM | 2407 | N | ASN | A | 352 | 38.234 | 51.534 | 35.042 | 1.00 | 49.21 | A | N |
| ATOM | 2408 | CA | ASN | A | 352 | 39.534 | 50.881 | 34.952 | 1.00 | 49.72 | A | C |
| ATOM | 2409 | CB | ASN | A | 352 | 39.385 | 49.481 | 35.533 | 1.00 | 51.32 | A | C |
| ATOM | 2410 | CG | ASN | A | 352 | 40.692 | 48.769 | 35.675 | 1.00 | 53.22 | A | C |
| ATOM | 2411 | OD1 | ASN | A | 352 | 41.591 | 48.906 | 34.824 | 1.00 | 54.71 | A | O |
| ATOM | 2412 | ND2 | ASN | A | 352 | 40.819 | 47.979 | 36.743 | 1.00 | 53.01 | A | N |
| ATOM | 2413 | C | ASN | A | 352 | 40.000 | 50.779 | 33.498 | 1.00 | 50.01 | A | C |
| ATOM | 2414 | O | ASN | A | 352 | 41.204 | 50.717 | 33.217 | 1.00 | 50.18 | A | O |
| ATOM | 2415 | N | GLY | A | 353 | 39.037 | 50.757 | 32.579 | 1.00 | 49.52 | A | N |
| ATOM | 2416 | CA | GLY | A | 353 | 39.352 | 50.634 | 31.170 | 1.00 | 47.60 | A | C |
| ATOM | 2417 | C | GLY | A | 353 | 39.147 | 49.194 | 30.712 | 1.00 | 47.40 | A | C |
| ATOM | 2418 | O | GLY | A | 353 | 38.792 | 48.927 | 29.541 | 1.00 | 46.87 | A | O |
| ATOM | 2419 | N | ARG | A | 354 | 39.362 | 48.260 | 31.635 | 1.00 | 45.39 | A | N |
| ATOM | 2420 | CA | ARG | A | 354 | 39.229 | 46.834 | 31.350 | 1.00 | 44.99 | A | C |
| ATOM | 2421 | CB | ARG | A | 354 | 39.793 | 46.004 | 32.527 | 1.00 | 46.73 | A | C |
| ATOM | 2422 | CG | ARG | A | 354 | 41.145 | 46.495 | 33.084 | 1.00 | 48.43 | A | C |
| ATOM | 2423 | CD | ARG | A | 354 | 41.635 | 45.679 | 34.302 | 1.00 | 50.79 | A | C |
| ATOM | 2424 | NE | ARG | A | 354 | 41.984 | 44.295 | 33.960 | 1.00 | 53.35 | A | N |
| ATOM | 2425 | CZ | ARG | A | 354 | 42.438 | 43.387 | 34.830 | 1.00 | 54.46 | A | C |
| ATOM | 2426 | NH1 | ARG | A | 354 | 42.605 | 43.708 | 36.109 | 1.00 | 54.97 | A | N |
| ATOM | 2427 | NH2 | ARG | A | 354 | 42.718 | 42.148 | 34.423 | 1.00 | 53.27 | A | N |
| ATOM | 2428 | C | ARG | A | 354 | 37.760 | 46.458 | 31.140 | 1.00 | 43.54 | A | C |
| ATOM | 2429 | O | ARG | A | 354 | 36.842 | 47.265 | 31.394 | 1.00 | 42.89 | A | O |
| ATOM | 2430 | N | ASP | A | 355 | 37.537 | 45.232 | 30.679 | 1.00 | 42.30 | A | N |
| ATOM | 2431 | CA | ASP | A | 355 | 36.187 | 44.733 | 30.457 | 1.00 | 41.67 | A | C |
| ATOM | 2432 | CB | ASP | A | 355 | 36.202 | 43.540 | 29.491 | 1.00 | 42.68 | A | C |
| ATOM | 2433 | CG | ASP | A | 355 | 35.802 | 43.931 | 28.072 | 1.00 | 44.56 | A | C |
| ATOM | 2434 | OD1 | ASP | A | 355 | 34.811 | 43.365 | 27.555 | 1.00 | 44.63 | A | O |
| ATOM | 2435 | OD2 | ASP | A | 355 | 36.470 | 44.806 | 27.472 | 1.00 | 45.58 | A | O |
| ATOM | 2436 | C | ASP | A | 355 | 35.557 | 44.309 | 31.784 | 1.00 | 40.59 | A | C |
| ATOM | 2437 | O | ASP | A | 355 | 36.250 | 44.087 | 32.762 | 1.00 | 40.84 | A | O |
| ATOM | 2438 | N | THR | A | 356 | 34.232 | 44.224 | 31.815 | 1.00 | 38.63 | A | N |
| ATOM | 2439 | CA | THR | A | 356 | 33.545 | 43.796 | 33.021 | 1.00 | 36.65 | A | C |
| ATOM | 2440 | CB | THR | A | 356 | 32.033 | 44.093 | 32.982 | 1.00 | 35.85 | A | C |
| ATOM | 2441 | OG1 | THR | A | 356 | 31.502 | 43.689 | 31.717 | 1.00 | 36.83 | A | O |
| ATOM | 2442 | CG2 | THR | A | 356 | 31.769 | 45.556 | 33.213 | 1.00 | 35.16 | A | C |
| ATOM | 2443 | C | THR | A | 356 | 33.705 | 42.290 | 33.107 | 1.00 | 35.54 | A | C |
| ATOM | 2444 | O | THR | A | 356 | 33.920 | 41.631 | 32.100 | 1.00 | 35.71 | A | O |
| ATOM | 2445 | N | PRO | A | 357 | 33.612 | 41.729 | 34.319 | 1.00 | 34.54 | A | N |
| ATOM | 2446 | CD | PRO | A | 357 | 33.466 | 42.346 | 35.649 | 1.00 | 32.89 | A | C |
| ATOM | 2447 | CA | PRO | A | 357 | 33.755 | 40.279 | 34.427 | 1.00 | 33.29 | A | C |
| ATOM | 2448 | CB | PRO | A | 357 | 33.832 | 40.048 | 35.931 | 1.00 | 33.35 | A | C |
| ATOM | 2449 | CG | PRO | A | 357 | 33.015 | 41.173 | 36.486 | 1.00 | 32.84 | A | C |
| ATOM | 2450 | C | PRO | A | 357 | 32.545 | 39.585 | 33.812 | 1.00 | 33.03 | A | C |
| ATOM | 2451 | O | PRO | A | 357 | 31.642 | 40.231 | 33.278 | 1.00 | 31.81 | A | O |
| ATOM | 2452 | N | ALA | A | 358 | 32.538 | 38.262 | 33.902 | 1.00 | 33.91 | A | N |
| ATOM | 2453 | CA | ALA | A | 358 | 31.447 | 37.469 | 33.365 | 1.00 | 34.14 | A | C |
| ATOM | 2454 | CB | ALA | A | 358 | 31.798 | 35.982 | 33.439 | 1.00 | 36.10 | A | C |
| ATOM | 2455 | C | ALA | A | 358 | 30.195 | 37.769 | 34.190 | 1.00 | 32.98 | A | C |
| ATOM | 2456 | O | ALA | A | 358 | 30.218 | 37.685 | 35.415 | 1.00 | 35.01 | A | O |
| ATOM | 2457 | N | LEU | A | 359 | 29.107 | 38.120 | 33.517 | 1.00 | 31.67 | A | N |
| ATOM | 2458 | CA | LEU | A | 359 | 27.874 | 38.458 | 34.209 | 1.00 | 29.19 | A | C |
| ATOM | 2459 | CB | LEU | A | 359 | 27.596 | 39.958 | 34.069 | 1.00 | 28.75 | A | C |
| ATOM | 2460 | CG | LEU | A | 359 | 28.626 | 40.946 | 34.616 | 1.00 | 29.69 | A | C |
| ATOM | 2461 | CD1 | LEU | A | 359 | 28.122 | 42.368 | 34.391 | 1.00 | 29.71 | A | C |
| ATOM | 2462 | CD2 | LEU | A | 359 | 28.855 | 40.697 | 36.104 | 1.00 | 27.24 | A | C |

| | | | | | | | | | | | | |
|---|---|---|---|---|---|---|---|---|---|---|---|---|
| ATOM | 2463 | C | LEU | A | 359 | 26.667 | 37.698 | 33.674 | 1.00 | 28.40 | A | C |
| ATOM | 2464 | O | LEU | A | 359 | 25.607 | 37.662 | 34.312 | 1.00 | 25.60 | A | O |
| ATOM | 2465 | N | PHE | A | 360 | 26.839 | 37.084 | 32.507 | 1.00 | 28.29 | A | N |
| ATOM | 2466 | CA | PHE | A | 360 | 25.748 | 36.379 | 31.854 | 1.00 | 28.55 | A | C |
| ATOM | 2467 | CB | PHE | A | 360 | 25.464 | 37.072 | 30.520 | 1.00 | 29.41 | A | C |
| ATOM | 2468 | CG | PHE | A | 360 | 25.595 | 38.576 | 30.581 | 1.00 | 28.68 | A | C |
| ATOM | 2469 | CD1 | PHE | A | 360 | 24.714 | 39.334 | 31.342 | 1.00 | 26.61 | A | C |
| ATOM | 2470 | CD2 | PHE | A | 360 | 26.624 | 39.229 | 29.906 | 1.00 | 28.94 | A | C |
| ATOM | 2471 | CE1 | PHE | A | 360 | 24.855 | 40.718 | 31.428 | 1.00 | 26.66 | A | C |
| ATOM | 2472 | CE2 | PHE | A | 360 | 26.770 | 40.616 | 29.989 | 1.00 | 26.78 | A | C |
| ATOM | 2473 | CZ | PHE | A | 360 | 25.885 | 41.357 | 30.753 | 1.00 | 25.41 | A | C |
| ATOM | 2474 | C | PHE | A | 360 | 25.896 | 34.873 | 31.631 | 1.00 | 28.98 | A | C |
| ATOM | 2475 | O | PHE | A | 360 | 25.055 | 34.275 | 31.008 | 1.00 | 29.89 | A | O |
| ATOM | 2476 | N | ASN | A | 361 | 26.955 | 34.269 | 32.156 | 1.00 | 29.63 | A | N |
| ATOM | 2477 | CA | ASN | A | 361 | 27.177 | 32.833 | 31.987 | 1.00 | 30.30 | A | C |
| ATOM | 2478 | CB | ASN | A | 361 | 28.656 | 32.510 | 32.227 | 1.00 | 31.70 | A | C |
| ATOM | 2479 | CG | ASN | A | 361 | 29.218 | 33.235 | 33.437 | 1.00 | 35.98 | A | C |
| ATOM | 2480 | OD1 | ASN | A | 361 | 28.937 | 34.437 | 33.649 | 1.00 | 38.15 | A | O |
| ATOM | 2481 | ND2 | ASN | A | 361 | 30.023 | 32.528 | 34.240 | 1.00 | 37.88 | A | N |
| ATOM | 2482 | C | ASN | A | 361 | 26.278 | 31.990 | 32.892 | 1.00 | 30.10 | A | C |
| ATOM | 2483 | O | ASN | A | 361 | 26.738 | 31.078 | 33.567 | 1.00 | 29.39 | A | O |
| ATOM | 2484 | N | PHE | A | 362 | 24.987 | 32.307 | 32.876 | 1.00 | 28.93 | A | N |
| ATOM | 2485 | CA | PHE | A | 362 | 23.987 | 31.616 | 33.683 | 1.00 | 29.14 | A | C |
| ATOM | 2486 | CB | PHE | A | 362 | 22.638 | 32.337 | 33.555 | 1.00 | 29.19 | A | C |
| ATOM | 2487 | CG | PHE | A | 362 | 22.545 | 33.615 | 34.342 | 1.00 | 27.33 | A | C |
| ATOM | 2488 | CD1 | PHE | A | 362 | 22.416 | 33.584 | 35.726 | 1.00 | 27.59 | A | C |
| ATOM | 2489 | CD2 | PHE | A | 362 | 22.578 | 34.850 | 33.699 | 1.00 | 27.96 | A | C |
| ATOM | 2490 | CE1 | PHE | A | 362 | 22.321 | 34.765 | 36.459 | 1.00 | 26.49 | A | C |
| ATOM | 2491 | CE2 | PHE | A | 362 | 22.484 | 36.038 | 34.422 | 1.00 | 27.95 | A | C |
| ATOM | 2492 | CZ | PHE | A | 362 | 22.354 | 35.996 | 35.806 | 1.00 | 26.91 | A | C |
| ATOM | 2493 | C | PHE | A | 362 | 23.785 | 30.145 | 33.301 | 1.00 | 30.86 | A | C |
| ATOM | 2494 | O | PHE | A | 362 | 23.663 | 29.810 | 32.118 | 1.00 | 31.63 | A | O |
| ATOM | 2495 | N | THR | A | 363 | 23.746 | 29.274 | 34.303 | 1.00 | 30.86 | A | N |
| ATOM | 2496 | CA | THR | A | 363 | 23.512 | 27.848 | 34.079 | 1.00 | 31.77 | A | C |
| ATOM | 2497 | CB | THR | A | 363 | 24.246 | 26.989 | 35.106 | 1.00 | 30.63 | A | C |
| ATOM | 2498 | OG1 | THR | A | 363 | 23.716 | 27.262 | 36.408 | 1.00 | 31.03 | A | O |
| ATOM | 2499 | CG2 | THR | A | 363 | 25.737 | 27.283 | 35.084 | 1.00 | 27.78 | A | C |
| ATOM | 2500 | C | THR | A | 363 | 22.015 | 27.634 | 34.286 | 1.00 | 32.75 | A | C |
| ATOM | 2501 | O | THR | A | 363 | 21.330 | 28.518 | 34.807 | 1.00 | 34.68 | A | O |
| ATOM | 2502 | N | THR | A | 364 | 21.510 | 26.466 | 33.896 | 1.00 | 33.39 | A | N |
| ATOM | 2503 | CA | THR | A | 364 | 20.083 | 26.173 | 34.038 | 1.00 | 33.20 | A | C |
| ATOM | 2504 | CB | THR | A | 364 | 19.691 | 24.892 | 33.217 | 1.00 | 34.61 | A | C |
| ATOM | 2505 | OG1 | THR | A | 364 | 19.450 | 23.788 | 34.096 | 1.00 | 36.53 | A | O |
| ATOM | 2506 | CG2 | THR | A | 364 | 20.810 | 24.518 | 32.264 | 1.00 | 32.07 | A | C |
| ATOM | 2507 | C | THR | A | 364 | 19.776 | 26.010 | 35.530 | 1.00 | 33.05 | A | C |
| ATOM | 2508 | O | THR | A | 364 | 18.692 | 26.330 | 35.990 | 1.00 | 33.48 | A | O |
| ATOM | 2509 | N | GLN | A | 365 | 20.769 | 25.533 | 36.272 | 1.00 | 32.28 | A | N |
| ATOM | 2510 | CA | GLN | A | 365 | 20.655 | 25.361 | 37.718 | 1.00 | 32.81 | A | C |
| ATOM | 2511 | CB | GLN | A | 365 | 21.958 | 24.776 | 38.270 | 1.00 | 35.24 | A | C |
| ATOM | 2512 | CG | GLN | A | 365 | 22.082 | 24.818 | 39.788 | 1.00 | 37.35 | A | C |
| ATOM | 2513 | CD | GLN | A | 365 | 21.272 | 23.730 | 40.470 | 1.00 | 38.62 | A | C |
| ATOM | 2514 | OE1 | GLN | A | 365 | 20.876 | 23.874 | 41.607 | 1.00 | 38.48 | A | O |
| ATOM | 2515 | NE2 | GLN | A | 365 | 21.040 | 22.627 | 39.764 | 1.00 | 38.95 | A | N |
| ATOM | 2516 | C | GLN | A | 365 | 20.420 | 26.740 | 38.342 | 1.00 | 32.91 | A | C |
| ATOM | 2517 | O | GLN | A | 365 | 19.573 | 26.899 | 39.224 | 1.00 | 32.67 | A | O |
| ATOM | 2518 | N | GLU | A | 366 | 21.179 | 27.730 | 37.869 | 1.00 | 31.69 | A | N |
| ATOM | 2519 | CA | GLU | A | 366 | 21.071 | 29.105 | 38.358 | 1.00 | 30.32 | A | C |
| ATOM | 2520 | CB | GLU | A | 366 | 22.167 | 29.990 | 37.750 | 1.00 | 30.88 | A | C |
| ATOM | 2521 | CG | GLU | A | 366 | 23.515 | 29.889 | 38.417 | 1.00 | 32.22 | A | C |
| ATOM | 2522 | CD | GLU | A | 366 | 24.489 | 30.955 | 37.931 | 1.00 | 32.66 | A | C |
| ATOM | 2523 | OE1 | GLU | A | 366 | 24.253 | 32.152 | 38.202 | 1.00 | 30.99 | A | O |
| ATOM | 2524 | OE2 | GLU | A | 366 | 25.489 | 30.588 | 37.277 | 1.00 | 34.81 | A | O |
| ATOM | 2525 | C | GLU | A | 366 | 19.724 | 29.764 | 38.059 | 1.00 | 29.25 | A | C |

| | | | | | | | | | | | |
|---|---|---|---|---|---|---|---|---|---|---|---|
| ATOM | 2526 | O | GLU | A | 366 | 19.225 | 30.558 | 38.853 | 1.00 | 27.47 | A | O |
| ATOM | 2527 | N | LEU | A | 367 | 19.152 | 29.435 | 36.903 | 1.00 | 29.63 | A | N |
| ATOM | 2528 | CA | LEU | A | 367 | 17.877 | 30.007 | 36.481 | 1.00 | 28.50 | A | C |
| ATOM | 2529 | CB | LEU | A | 367 | 17.878 | 30.196 | 34.962 | 1.00 | 28.97 | A | C |
| ATOM | 2530 | CG | LEU | A | 367 | 18.997 | 31.076 | 34.404 | 1.00 | 29.75 | A | C |
| ATOM | 2531 | CD1 | LEU | A | 367 | 19.244 | 30.745 | 32.938 | 1.00 | 28.31 | A | C |
| ATOM | 2532 | CD2 | LEU | A | 367 | 18.625 | 32.540 | 34.599 | 1.00 | 25.35 | A | C |
| ATOM | 2533 | C | LEU | A | 367 | 16.661 | 29.162 | 36.861 | 1.00 | 28.17 | A | C |
| ATOM | 2534 | O | LEU | A | 367 | 15.536 | 29.584 | 36.660 | 1.00 | 28.25 | A | O |
| ATOM | 2535 | N | SER | A | 368 | 16.891 | 27.979 | 37.421 | 1.00 | 28.97 | A | N |
| ATOM | 2536 | CA | SER | A | 368 | 15.784 | 27.095 | 37.763 | 1.00 | 29.89 | A | C |
| ATOM | 2537 | CB | SER | A | 368 | 16.297 | 25.866 | 38.528 | 1.00 | 30.01 | A | C |
| ATOM | 2538 | OG | SER | A | 368 | 16.938 | 26.205 | 39.743 | 1.00 | 32.56 | A | O |
| ATOM | 2539 | C | SER | A | 368 | 14.602 | 27.728 | 38.501 | 1.00 | 30.57 | A | C |
| ATOM | 2540 | O | SER | A | 368 | 13.482 | 27.306 | 38.317 | 1.00 | 31.09 | A | O |
| ATOM | 2541 | N | SER | A | 369 | 14.845 | 28.758 | 39.303 | 1.00 | 30.70 | A | N |
| ATOM | 2542 | CA | SER | A | 369 | 13.756 | 29.398 | 40.046 | 1.00 | 31.32 | A | C |
| ATOM | 2543 | CB | SER | A | 369 | 14.324 | 30.382 | 41.082 | 1.00 | 29.95 | A | C |
| ATOM | 2544 | OG | SER | A | 369 | 14.841 | 31.558 | 40.469 | 1.00 | 30.40 | A | O |
| ATOM | 2545 | C | SER | A | 369 | 12.775 | 30.135 | 39.144 | 1.00 | 31.09 | A | C |
| ATOM | 2546 | O | SER | A | 369 | 11.638 | 30.353 | 39.525 | 1.00 | 29.92 | A | O |
| ATOM | 2547 | N | ASN | A | 370 | 13.225 | 30.512 | 37.950 | 1.00 | 31.56 | A | N |
| ATOM | 2548 | CA | ASN | A | 370 | 12.374 | 31.248 | 37.013 | 1.00 | 31.80 | A | C |
| ATOM | 2549 | CB | ASN | A | 370 | 12.136 | 32.653 | 37.563 | 1.00 | 31.57 | A | C |
| ATOM | 2550 | CG | ASN | A | 370 | 10.889 | 33.296 | 37.009 | 1.00 | 32.92 | A | C |
| ATOM | 2551 | OD1 | ASN | A | 370 | 10.449 | 32.977 | 35.900 | 1.00 | 35.29 | A | O |
| ATOM | 2552 | ND2 | ASN | A | 370 | 10.313 | 34.221 | 37.774 | 1.00 | 32.07 | A | N |
| ATOM | 2553 | C | ASN | A | 370 | 13.072 | 31.324 | 35.642 | 1.00 | 33.49 | A | C |
| ATOM | 2554 | O | ASN | A | 370 | 13.471 | 32.391 | 35.207 | 1.00 | 34.24 | A | O |
| ATOM | 2555 | N | PRO | A | 371 | 13.237 | 30.173 | 34.955 | 1.00 | 34.76 | A | N |
| ATOM | 2556 | CD | PRO | A | 371 | 12.907 | 28.806 | 35.389 | 1.00 | 34.81 | A | C |
| ATOM | 2557 | CA | PRO | A | 371 | 13.896 | 30.151 | 33.643 | 1.00 | 34.26 | A | C |
| ATOM | 2558 | CB | PRO | A | 371 | 13.538 | 28.765 | 33.071 | 1.00 | 34.24 | A | C |
| ATOM | 2559 | CG | PRO | A | 371 | 12.597 | 28.141 | 34.083 | 1.00 | 35.26 | A | C |
| ATOM | 2560 | C | PRO | A | 371 | 13.598 | 31.301 | 32.682 | 1.00 | 34.41 | A | C |
| ATOM | 2561 | O | PRO | A | 371 | 14.505 | 31.900 | 32.155 | 1.00 | 36.21 | A | O |
| ATOM | 2562 | N | PRO | A | 372 | 12.316 | 31.623 | 32.459 | 1.00 | 33.69 | A | N |
| ATOM | 2563 | CD | PRO | A | 372 | 11.135 | 30.956 | 33.033 | 1.00 | 33.28 | A | C |
| ATOM | 2564 | CA | PRO | A | 372 | 11.918 | 32.711 | 31.554 | 1.00 | 32.81 | A | C |
| ATOM | 2565 | CB | PRO | A | 372 | 10.420 | 32.827 | 31.809 | 1.00 | 33.43 | A | C |
| ATOM | 2566 | CG | PRO | A | 372 | 10.032 | 31.404 | 32.111 | 1.00 | 33.66 | A | C |
| ATOM | 2567 | C | PRO | A | 372 | 12.653 | 34.035 | 31.801 | 1.00 | 32.53 | A | C |
| ATOM | 2568 | O | PRO | A | 372 | 12.723 | 34.876 | 30.912 | 1.00 | 32.11 | A | O |
| ATOM | 2569 | N | LEU | A | 373 | 13.193 | 34.224 | 33.004 | 1.00 | 31.52 | A | N |
| ATOM | 2570 | CA | LEU | A | 373 | 13.915 | 35.459 | 33.300 | 1.00 | 31.96 | A | C |
| ATOM | 2571 | CB | LEU | A | 373 | 14.300 | 35.532 | 34.784 | 1.00 | 31.89 | A | C |
| ATOM | 2572 | CG | LEU | A | 373 | 13.206 | 35.955 | 35.770 | 1.00 | 32.00 | A | C |
| ATOM | 2573 | CD1 | LEU | A | 373 | 13.776 | 35.943 | 37.190 | 1.00 | 31.19 | A | C |
| ATOM | 2574 | CD2 | LEU | A | 373 | 12.688 | 37.350 | 35.407 | 1.00 | 28.40 | A | C |
| ATOM | 2575 | C | LEU | A | 373 | 15.155 | 35.591 | 32.433 | 1.00 | 32.08 | A | C |
| ATOM | 2576 | O | LEU | A | 373 | 15.651 | 36.694 | 32.223 | 1.00 | 32.06 | A | O |
| ATOM | 2577 | N | ALA | A | 374 | 15.639 | 34.460 | 31.924 | 1.00 | 33.21 | A | N |
| ATOM | 2578 | CA | ALA | A | 374 | 16.816 | 34.434 | 31.056 | 1.00 | 34.19 | A | C |
| ATOM | 2579 | CB | ALA | A | 374 | 17.031 | 33.032 | 30.504 | 1.00 | 33.39 | A | C |
| ATOM | 2580 | C | ALA | A | 374 | 16.676 | 35.422 | 29.907 | 1.00 | 34.29 | A | C |
| ATOM | 2581 | O | ALA | A | 374 | 17.668 | 35.891 | 29.373 | 1.00 | 34.36 | A | O |
| ATOM | 2582 | N | THR | A | 375 | 15.435 | 35.739 | 29.543 | 1.00 | 35.43 | A | N |
| ATOM | 2583 | CA | THR | A | 375 | 15.172 | 36.656 | 28.432 | 1.00 | 36.83 | A | C |
| ATOM | 2584 | CB | THR | A | 375 | 13.683 | 36.587 | 27.986 | 1.00 | 38.00 | A | C |
| ATOM | 2585 | OG1 | THR | A | 375 | 12.848 | 37.204 | 28.980 | 1.00 | 38.00 | A | O |
| ATOM | 2586 | CG2 | THR | A | 375 | 13.247 | 35.122 | 27.798 | 1.00 | 37.91 | A | C |
| ATOM | 2587 | C | THR | A | 375 | 15.491 | 38.090 | 28.814 | 1.00 | 36.46 | A | C |
| ATOM | 2588 | O | THR | A | 375 | 15.233 | 38.998 | 28.061 | 1.00 | 36.82 | A | O |

| | | | | | | | | | | | | |
|---|---|---|---|---|---|---|---|---|---|---|---|---|
| ATOM | 2589 | N | ILE | A | 376 | 16.039 | 38.284 | 30.006 | 1.00 | 35.99 | A | N |
| ATOM | 2590 | CA | ILE | A | 376 | 16.406 | 39.621 | 30.457 | 1.00 | 34.98 | A | C |
| ATOM | 2591 | CB | ILE | A | 376 | 15.506 | 40.089 | 31.622 | 1.00 | 35.89 | A | C |
| ATOM | 2592 | CG2 | ILE | A | 376 | 16.077 | 41.344 | 32.263 | 1.00 | 35.50 | A | C |
| ATOM | 2593 | CG1 | ILE | A | 376 | 14.089 | 40.352 | 31.118 | 1.00 | 36.27 | A | C |
| ATOM | 2594 | CD1 | ILE | A | 376 | 13.100 | 40.612 | 32.230 | 1.00 | 35.44 | A | C |
| ATOM | 2595 | C | ILE | A | 376 | 17.837 | 39.560 | 30.944 | 1.00 | 34.64 | A | C |
| ATOM | 2596 | O | ILE | A | 376 | 18.618 | 40.460 | 30.693 | 1.00 | 34.50 | A | O |
| ATOM | 2597 | N | LEU | A | 377 | 18.154 | 38.467 | 31.633 | 1.00 | 33.68 | A | N |
| ATOM | 2598 | CA | LEU | A | 377 | 19.475 | 38.228 | 32.187 | 1.00 | 32.69 | A | C |
| ATOM | 2599 | CB | LEU | A | 377 | 19.425 | 36.990 | 33.074 | 1.00 | 32.34 | A | C |
| ATOM | 2600 | CG | LEU | A | 377 | 18.455 | 37.147 | 34.250 | 1.00 | 32.26 | A | C |
| ATOM | 2601 | CD1 | LEU | A | 377 | 18.375 | 35.835 | 34.997 | 1.00 | 32.36 | A | C |
| ATOM | 2602 | CD2 | LEU | A | 377 | 18.922 | 38.275 | 35.181 | 1.00 | 31.05 | A | C |
| ATOM | 2603 | C | LEU | A | 377 | 20.537 | 38.070 | 31.113 | 1.00 | 33.40 | A | C |
| ATOM | 2604 | O | LEU | A | 377 | 21.646 | 38.558 | 31.264 | 1.00 | 34.81 | A | O |
| ATOM | 2605 | N | ILE | A | 378 | 20.207 | 37.367 | 30.034 | 1.00 | 32.83 | A | N |
| ATOM | 2606 | CA | ILE | A | 378 | 21.174 | 37.186 | 28.958 | 1.00 | 32.78 | A | C |
| ATOM | 2607 | CB | ILE | A | 378 | 21.139 | 35.757 | 28.385 | 1.00 | 31.82 | A | C |
| ATOM | 2608 | CG2 | ILE | A | 378 | 22.228 | 35.591 | 27.364 | 1.00 | 32.60 | A | C |
| ATOM | 2609 | CG1 | ILE | A | 378 | 21.379 | 34.735 | 29.490 | 1.00 | 33.59 | A | C |
| ATOM | 2610 | CD1 | ILE | A | 378 | 20.187 | 34.489 | 30.363 | 1.00 | 36.15 | A | C |
| ATOM | 2611 | C | ILE | A | 378 | 20.814 | 38.182 | 27.851 | 1.00 | 32.56 | A | C |
| ATOM | 2612 | O | ILE | A | 378 | 19.839 | 38.011 | 27.146 | 1.00 | 32.66 | A | O |
| ATOM | 2613 | N | PRO | A | 379 | 21.593 | 39.260 | 27.718 | 1.00 | 31.91 | A | N |
| ATOM | 2614 | CD | PRO | A | 379 | 22.712 | 39.727 | 28.560 | 1.00 | 31.90 | A | C |
| ATOM | 2615 | CA | PRO | A | 379 | 21.280 | 40.232 | 26.671 | 1.00 | 32.11 | A | C |
| ATOM | 2616 | CB | PRO | A | 379 | 22.090 | 41.459 | 27.082 | 1.00 | 30.20 | A | C |
| ATOM | 2617 | CG | PRO | A | 379 | 23.306 | 40.847 | 27.722 | 1.00 | 30.36 | A | C |
| ATOM | 2618 | C | PRO | A | 379 | 21.620 | 39.730 | 25.267 | 1.00 | 32.65 | A | C |
| ATOM | 2619 | O | PRO | A | 379 | 22.444 | 38.824 | 25.096 | 1.00 | 33.21 | A | O |
| ATOM | 2620 | N | PRO | A | 380 | 20.976 | 40.308 | 24.244 | 1.00 | 32.59 | A | N |
| ATOM | 2621 | CD | PRO | A | 380 | 19.941 | 41.357 | 24.311 | 1.00 | 32.34 | A | C |
| ATOM | 2622 | CA | PRO | A | 380 | 21.220 | 39.908 | 22.858 | 1.00 | 32.94 | A | C |
| ATOM | 2623 | CB | PRO | A | 380 | 20.637 | 41.074 | 22.069 | 1.00 | 32.83 | A | C |
| ATOM | 2624 | CG | PRO | A | 380 | 19.414 | 41.400 | 22.866 | 1.00 | 34.46 | A | C |
| ATOM | 2625 | C | PRO | A | 380 | 22.668 | 39.626 | 22.508 | 1.00 | 33.50 | A | C |
| ATOM | 2626 | O | PRO | A | 380 | 22.966 | 38.553 | 22.006 | 1.00 | 34.93 | A | O |
| ATOM | 2627 | N | HIS | A | 381 | 23.568 | 40.570 | 22.781 | 1.00 | 33.97 | A | N |
| ATOM | 2628 | CA | HIS | A | 381 | 24.978 | 40.369 | 22.442 | 1.00 | 35.02 | A | C |
| ATOM | 2629 | CB | HIS | A | 381 | 25.774 | 41.659 | 22.698 | 1.00 | 34.66 | A | C |
| ATOM | 2630 | CG | HIS | A | 381 | 26.073 | 41.923 | 24.144 | 1.00 | 35.87 | A | C |
| ATOM | 2631 | CD2 | HIS | A | 381 | 25.494 | 42.756 | 25.039 | 1.00 | 35.39 | A | C |
| ATOM | 2632 | ND1 | HIS | A | 381 | 27.083 | 41.272 | 24.821 | 1.00 | 35.59 | A | N |
| ATOM | 2633 | CE1 | HIS | A | 381 | 27.114 | 41.697 | 26.073 | 1.00 | 35.58 | A | C |
| ATOM | 2634 | NE2 | HIS | A | 381 | 26.160 | 42.597 | 26.233 | 1.00 | 35.01 | A | N |
| ATOM | 2635 | C | HIS | A | 381 | 25.589 | 39.191 | 23.188 | 1.00 | 36.21 | A | C |
| ATOM | 2636 | O | HIS | A | 381 | 25.056 | 38.824 | 24.262 | 1.00 | 37.56 | A | O |
| ATOM | 2637 | OXT | HIS | A | 381 | 26.598 | 38.652 | 22.683 | 1.00 | 37.26 | A | O |
| TER | 2638 | | HIS | A | 381 | | | | | | A | |
| ATOM | 2639 | CB | VAL | B | 37 | 14.181 | 81.964 | 79.298 | 1.00 | 45.87 | B | C |
| ATOM | 2640 | CG1 | VAL | B | 37 | 13.322 | 81.636 | 80.518 | 1.00 | 46.03 | B | C |
| ATOM | 2641 | CG2 | VAL | B | 37 | 15.571 | 82.399 | 79.726 | 1.00 | 47.05 | B | C |
| ATOM | 2642 | C | VAL | B | 37 | 12.914 | 80.144 | 78.170 | 1.00 | 43.89 | B | C |
| ATOM | 2643 | O | VAL | B | 37 | 12.062 | 80.753 | 77.527 | 1.00 | 43.66 | B | O |
| ATOM | 2644 | N | VAL | B | 37 | 14.982 | 81.089 | 77.109 | 1.00 | 45.22 | B | N |
| ATOM | 2645 | CA | VAL | B | 37 | 14.303 | 80.737 | 78.393 | 1.00 | 45.09 | B | C |
| ATOM | 2646 | N | THR | B | 38 | 12.693 | 78.955 | 78.725 | 1.00 | 42.23 | B | N |
| ATOM | 2647 | CA | THR | B | 38 | 11.421 | 78.264 | 78.553 | 1.00 | 40.10 | B | C |
| ATOM | 2648 | CB | THR | B | 38 | 11.644 | 76.858 | 77.958 | 1.00 | 40.16 | B | C |
| ATOM | 2649 | OG1 | THR | B | 38 | 12.425 | 76.963 | 76.762 | 1.00 | 39.95 | B | O |
| ATOM | 2650 | CG2 | THR | B | 38 | 10.312 | 76.194 | 77.629 | 1.00 | 39.73 | B | C |
| ATOM | 2651 | C | THR | B | 38 | 10.653 | 78.102 | 79.855 | 1.00 | 38.49 | B | C |

| | | | | | | | | | | | |
|---|---|---|---|---|---|---|---|---|---|---|---|
| ATOM | 2652 | O | THR | B | 38 | 11.202 | 77.670 | 80.866 | 1.00 | 38.24 | B | O |
| ATOM | 2653 | N | THR | B | 39 | 9.373 | 78.449 | 79.814 | 1.00 | 36.91 | B | N |
| ATOM | 2654 | CA | THR | B | 39 | 8.520 | 78.324 | 80.985 | 1.00 | 36.56 | B | C |
| ATOM | 2655 | CB | THR | B | 39 | 7.912 | 79.682 | 81.388 | 1.00 | 37.22 | B | C |
| ATOM | 2656 | OG1 | THR | B | 39 | 8.969 | 80.595 | 81.718 | 1.00 | 37.58 | B | O |
| ATOM | 2657 | CG2 | THR | B | 39 | 7.011 | 79.521 | 82.599 | 1.00 | 36.13 | B | C |
| ATOM | 2658 | C | THR | B | 39 | 7.419 | 77.345 | 80.607 | 1.00 | 35.14 | B | C |
| ATOM | 2659 | O | THR | B | 39 | 6.752 | 77.499 | 79.587 | 1.00 | 34.79 | B | O |
| ATOM | 2660 | N | VAL | B | 40 | 7.252 | 76.323 | 81.432 | 1.00 | 33.44 | B | N |
| ATOM | 2661 | CA | VAL | B | 40 | 6.253 | 75.306 | 81.176 | 1.00 | 32.53 | B | C |
| ATOM | 2662 | CB | VAL | B | 40 | 6.939 | 74.009 | 80.642 | 1.00 | 32.52 | B | C |
| ATOM | 2663 | CG1 | VAL | B | 40 | 7.724 | 73.320 | 81.758 | 1.00 | 29.12 | B | C |
| ATOM | 2664 | CG2 | VAL | B | 40 | 5.910 | 73.078 | 80.050 | 1.00 | 33.55 | B | C |
| ATOM | 2665 | C | VAL | B | 40 | 5.533 | 75.000 | 82.479 | 1.00 | 32.46 | B | C |
| ATOM | 2666 | O | VAL | B | 40 | 6.010 | 75.362 | 83.537 | 1.00 | 32.96 | B | O |
| ATOM | 2667 | N | VAL | B | 41 | 4.367 | 74.368 | 82.395 | 1.00 | 31.78 | B | N |
| ATOM | 2668 | CA | VAL | B | 41 | 3.648 | 73.983 | 83.603 | 1.00 | 32.95 | B | C |
| ATOM | 2669 | CB | VAL | B | 41 | 2.165 | 74.467 | 83.582 | 1.00 | 32.38 | B | C |
| ATOM | 2670 | CG1 | VAL | B | 41 | 1.586 | 74.333 | 82.196 | 1.00 | 35.48 | B | C |
| ATOM | 2671 | CG2 | VAL | B | 41 | 1.349 | 73.692 | 84.591 | 1.00 | 30.37 | B | C |
| ATOM | 2672 | C | VAL | B | 41 | 3.771 | 72.464 | 83.656 | 1.00 | 33.05 | B | C |
| ATOM | 2673 | O | VAL | B | 41 | 3.191 | 71.763 | 82.859 | 1.00 | 33.78 | B | O |
| ATOM | 2674 | N | ALA | B | 42 | 4.567 | 71.981 | 84.604 | 1.00 | 33.16 | B | N |
| ATOM | 2675 | CA | ALA | B | 42 | 4.841 | 70.554 | 84.739 | 1.00 | 33.30 | B | C |
| ATOM | 2676 | CB | ALA | B | 42 | 6.352 | 70.336 | 84.688 | 1.00 | 31.60 | B | C |
| ATOM | 2677 | C | ALA | B | 42 | 4.273 | 69.882 | 85.981 | 1.00 | 33.33 | B | C |
| ATOM | 2678 | O | ALA | B | 42 | 3.987 | 70.528 | 86.979 | 1.00 | 33.60 | B | O |
| ATOM | 2679 | N | THR | B | 43 | 4.145 | 68.560 | 85.898 | 1.00 | 33.17 | B | N |
| ATOM | 2680 | CA | THR | B | 43 | 3.604 | 67.756 | 86.983 | 1.00 | 33.71 | B | C |
| ATOM | 2681 | CB | THR | B | 43 | 2.650 | 66.680 | 86.442 | 1.00 | 32.89 | B | C |
| ATOM | 2682 | OG1 | THR | B | 43 | 1.761 | 67.273 | 85.489 | 1.00 | 33.03 | B | O |
| ATOM | 2683 | CG2 | THR | B | 43 | 1.842 | 66.076 | 87.570 | 1.00 | 32.03 | B | C |
| ATOM | 2684 | C | THR | B | 43 | 4.745 | 67.071 | 87.727 | 1.00 | 34.19 | B | C |
| ATOM | 2685 | O | THR | B | 43 | 5.598 | 66.428 | 87.115 | 1.00 | 33.92 | B | O |
| ATOM | 2686 | N | PRO | B | 44 | 4.777 | 67.206 | 89.064 | 1.00 | 35.11 | B | N |
| ATOM | 2687 | CD | PRO | B | 44 | 3.908 | 68.054 | 89.901 | 1.00 | 34.33 | B | C |
| ATOM | 2688 | CA | PRO | B | 44 | 5.830 | 66.582 | 89.869 | 1.00 | 35.02 | B | C |
| ATOM | 2689 | CB | PRO | B | 44 | 5.440 | 66.951 | 91.299 | 1.00 | 34.20 | B | C |
| ATOM | 2690 | CG | PRO | B | 44 | 4.773 | 68.290 | 91.114 | 1.00 | 34.80 | B | C |
| ATOM | 2691 | C | PRO | B | 44 | 5.904 | 65.076 | 89.652 | 1.00 | 35.32 | B | C |
| ATOM | 2692 | O | PRO | B | 44 | 4.880 | 64.411 | 89.532 | 1.00 | 34.69 | B | O |
| ATOM | 2693 | N | GLY | B | 45 | 7.131 | 64.559 | 89.598 | 1.00 | 37.74 | B | N |
| ATOM | 2694 | CA | GLY | B | 45 | 7.356 | 63.138 | 89.397 | 1.00 | 40.39 | B | C |
| ATOM | 2695 | C | GLY | B | 45 | 6.619 | 62.286 | 90.417 | 1.00 | 42.87 | B | C |
| ATOM | 2696 | O | GLY | B | 45 | 5.884 | 61.358 | 90.051 | 1.00 | 42.92 | B | O |
| ATOM | 2697 | N | ALA | B | 46 | 6.815 | 62.597 | 91.698 | 1.00 | 45.11 | B | N |
| ATOM | 2698 | CA | ALA | B | 46 | 6.153 | 61.869 | 92.778 | 1.00 | 47.84 | B | C |
| ATOM | 2699 | CB | ALA | B | 46 | 7.107 | 61.685 | 93.957 | 1.00 | 47.70 | B | C |
| ATOM | 2700 | C | ALA | B | 46 | 4.908 | 62.636 | 93.226 | 1.00 | 49.45 | B | C |
| ATOM | 2701 | O | ALA | B | 46 | 4.438 | 63.538 | 92.518 | 1.00 | 49.67 | B | O |
| ATOM | 2702 | N | GLY | B | 47 | 4.377 | 62.270 | 94.394 | 1.00 | 50.91 | B | N |
| ATOM | 2703 | CA | GLY | B | 47 | 3.193 | 62.937 | 94.926 | 1.00 | 51.56 | B | C |
| ATOM | 2704 | C | GLY | B | 47 | 1.997 | 62.954 | 93.983 | 1.00 | 51.63 | B | C |
| ATOM | 2705 | O | GLY | B | 47 | 2.040 | 62.347 | 92.899 | 1.00 | 51.16 | B | O |
| ATOM | 2706 | N | PRO | B | 48 | 0.911 | 63.651 | 94.366 | 1.00 | 52.03 | B | N |
| ATOM | 2707 | CD | PRO | B | 48 | 0.780 | 64.440 | 95.609 | 1.00 | 52.32 | B | C |
| ATOM | 2708 | CA | PRO | B | 48 | -0.308 | 63.756 | 93.557 | 1.00 | 51.83 | B | C |
| ATOM | 2709 | CB | PRO | B | 48 | -1.361 | 64.109 | 94.593 | 1.00 | 52.98 | B | C |
| ATOM | 2710 | CG | PRO | B | 48 | -0.600 | 65.098 | 95.455 | 1.00 | 52.84 | B | C |
| ATOM | 2711 | C | PRO | B | 48 | -0.135 | 64.860 | 92.514 | 1.00 | 50.88 | B | C |
| ATOM | 2712 | O | PRO | B | 48 | 0.640 | 65.809 | 92.735 | 1.00 | 51.04 | B | O |
| ATOM | 2713 | N | ASP | B | 49 | -0.861 | 64.757 | 91.401 | 1.00 | 49.65 | B | N |
| ATOM | 2714 | CA | ASP | B | 49 | -0.765 | 65.744 | 90.323 | 1.00 | 49.14 | B | C |

| | | | | | | | | | | | | |
|---|---|---|---|---|---|---|---|---|---|---|---|---|
| ATOM | 2715 | CB | ASP | B | 49 | -1.626 | 65.300 | 89.124 | 1.00 | 50.19 | B | C |
| ATOM | 2716 | CG | ASP | B | 49 | -1.165 | 63.958 | 88.518 | 1.00 | 52.24 | B | C |
| ATOM | 2717 | OD1 | ASP | B | 49 | -1.738 | 63.537 | 87.480 | 1.00 | 52.15 | B | O |
| ATOM | 2718 | OD2 | ASP | B | 49 | -0.234 | 63.320 | 89.069 | 1.00 | 52.77 | B | O |
| ATOM | 2719 | C | ASP | B | 49 | -1.098 | 67.183 | 90.706 | 1.00 | 47.67 | B | C |
| ATOM | 2720 | O | ASP | B | 49 | -2.241 | 67.605 | 90.633 | 1.00 | 47.17 | B | O |
| ATOM | 2721 | N | ARG | B | 50 | -0.058 | 67.922 | 91.084 | 1.00 | 46.45 | B | N |
| ATOM | 2722 | CA | ARG | B | 50 | -0.155 | 69.327 | 91.493 | 1.00 | 45.36 | B | C |
| ATOM | 2723 | CB | ARG | B | 50 | 0.271 | 69.435 | 92.961 | 1.00 | 47.64 | B | C |
| ATOM | 2724 | CG | ARG | B | 50 | -0.550 | 70.387 | 93.819 | 1.00 | 52.01 | B | C |
| ATOM | 2725 | CD | ARG | B | 50 | -1.006 | 69.689 | 95.106 | 1.00 | 56.22 | B | C |
| ATOM | 2726 | NE | ARG | B | 50 | -1.931 | 68.591 | 94.808 | 1.00 | 58.58 | B | N |
| ATOM | 2727 | CZ | ARG | B | 50 | -3.220 | 68.756 | 94.500 | 1.00 | 60.24 | B | C |
| ATOM | 2728 | NH1 | ARG | B | 50 | -3.743 | 69.982 | 94.459 | 1.00 | 59.12 | B | N |
| ATOM | 2729 | NH2 | ARG | B | 50 | -3.979 | 67.695 | 94.208 | 1.00 | 60.37 | B | N |
| ATOM | 2730 | C | ARG | B | 50 | 0.802 | 70.130 | 90.598 | 1.00 | 42.46 | B | C |
| ATOM | 2731 | O | ARG | B | 50 | 1.892 | 70.478 | 91.011 | 1.00 | 41.22 | B | O |
| ATOM | 2732 | N | PRO | B | 51 | 0.398 | 70.418 | 89.352 | 1.00 | 40.85 | B | N |
| ATOM | 2733 | CD | PRO | B | 51 | -0.714 | 69.803 | 88.604 | 1.00 | 40.77 | B | C |
| ATOM | 2734 | CA | PRO | B | 51 | 1.263 | 71.180 | 88.446 | 1.00 | 40.21 | B | C |
| ATOM | 2735 | CB | PRO | B | 51 | 0.447 | 71.237 | 87.156 | 1.00 | 40.07 | B | C |
| ATOM | 2736 | CG | PRO | B | 51 | -0.225 | 69.902 | 87.148 | 1.00 | 40.01 | B | C |
| ATOM | 2737 | C | PRO | B | 51 | 1.752 | 72.538 | 88.896 | 1.00 | 39.51 | B | C |
| ATOM | 2738 | O | PRO | B | 51 | 1.049 | 73.261 | 89.568 | 1.00 | 39.62 | B | O |
| ATOM | 2739 | N | GLN | B | 52 | 2.989 | 72.851 | 88.523 | 1.00 | 38.55 | B | N |
| ATOM | 2740 | CA | GLN | B | 52 | 3.594 | 74.134 | 88.844 | 1.00 | 39.09 | B | C |
| ATOM | 2741 | CB | GLN | B | 52 | 4.478 | 74.050 | 90.090 | 1.00 | 38.77 | B | C |
| ATOM | 2742 | CG | GLN | B | 52 | 5.359 | 72.826 | 90.164 | 1.00 | 40.53 | B | C |
| ATOM | 2743 | CD | GLN | B | 52 | 6.040 | 72.697 | 91.514 | 1.00 | 40.09 | B | C |
| ATOM | 2744 | OE1 | GLN | B | 52 | 7.125 | 73.232 | 91.730 | 1.00 | 38.90 | B | O |
| ATOM | 2745 | NE2 | GLN | B | 52 | 5.388 | 71.994 | 92.438 | 1.00 | 39.28 | B | N |
| ATOM | 2746 | C | GLN | B | 52 | 4.387 | 74.631 | 87.665 | 1.00 | 39.06 | B | C |
| ATOM | 2747 | O | GLN | B | 52 | 4.726 | 73.874 | 86.754 | 1.00 | 40.34 | B | O |
| ATOM | 2748 | N | GLU | B | 53 | 4.657 | 75.924 | 87.674 | 1.00 | 38.55 | B | N |
| ATOM | 2749 | CA | GLU | B | 53 | 5.430 | 76.543 | 86.615 | 1.00 | 38.19 | B | C |
| ATOM | 2750 | CB | GLU | B | 53 | 5.204 | 78.049 | 86.658 | 1.00 | 40.23 | B | C |
| ATOM | 2751 | CG | GLU | B | 53 | 5.458 | 78.791 | 85.378 | 1.00 | 44.18 | B | C |
| ATOM | 2752 | CD | GLU | B | 53 | 5.342 | 80.294 | 85.553 | 1.00 | 46.76 | B | C |
| ATOM | 2753 | OE1 | GLU | B | 53 | 6.137 | 80.842 | 86.348 | 1.00 | 46.88 | B | O |
| ATOM | 2754 | OE2 | GLU | B | 53 | 4.471 | 80.924 | 84.893 | 1.00 | 48.45 | B | O |
| ATOM | 2755 | C | GLU | B | 53 | 6.892 | 76.226 | 86.847 | 1.00 | 37.85 | B | C |
| ATOM | 2756 | O | GLU | B | 53 | 7.363 | 76.197 | 88.001 | 1.00 | 37.70 | B | O |
| ATOM | 2757 | N | VAL | B | 54 | 7.604 | 75.957 | 85.757 | 1.00 | 36.95 | B | N |
| ATOM | 2758 | CA | VAL | B | 54 | 9.022 | 75.641 | 85.831 | 1.00 | 35.93 | B | C |
| ATOM | 2759 | CB | VAL | B | 54 | 9.266 | 74.125 | 85.793 | 1.00 | 36.09 | B | C |
| ATOM | 2760 | CG1 | VAL | B | 54 | 10.762 | 73.837 | 85.830 | 1.00 | 35.17 | B | C |
| ATOM | 2761 | CG2 | VAL | B | 54 | 8.575 | 73.473 | 86.988 | 1.00 | 33.64 | B | C |
| ATOM | 2762 | C | VAL | B | 54 | 9.699 | 76.318 | 84.662 | 1.00 | 35.70 | B | C |
| ATOM | 2763 | O | VAL | B | 54 | 9.257 | 76.190 | 83.525 | 1.00 | 35.35 | B | O |
| ATOM | 2764 | N | SER | B | 55 | 10.748 | 77.079 | 84.964 | 1.00 | 35.94 | B | N |
| ATOM | 2765 | CA | SER | B | 55 | 11.512 | 77.797 | 83.953 | 1.00 | 36.22 | B | C |
| ATOM | 2766 | CB | SER | B | 55 | 11.528 | 79.295 | 84.263 | 1.00 | 37.61 | B | C |
| ATOM | 2767 | OG | SER | B | 55 | 10.227 | 79.854 | 84.138 | 1.00 | 41.50 | B | O |
| ATOM | 2768 | C | SER | B | 55 | 12.942 | 77.283 | 83.897 | 1.00 | 35.47 | B | C |
| ATOM | 2769 | O | SER | B | 55 | 13.593 | 77.113 | 84.931 | 1.00 | 34.02 | B | O |
| ATOM | 2770 | N | TYR | B | 56 | 13.423 | 77.049 | 82.680 | 1.00 | 34.65 | B | N |
| ATOM | 2771 | CA | TYR | B | 56 | 14.774 | 76.558 | 82.459 | 1.00 | 34.64 | B | C |
| ATOM | 2772 | CB | TYR | B | 56 | 14.752 | 75.032 | 82.315 | 1.00 | 31.97 | B | C |
| ATOM | 2773 | CG | TYR | B | 56 | 13.931 | 74.503 | 81.150 | 1.00 | 30.99 | B | C |
| ATOM | 2774 | CD1 | TYR | B | 56 | 14.435 | 74.516 | 79.850 | 1.00 | 30.26 | B | C |
| ATOM | 2775 | CE1 | TYR | B | 56 | 13.695 | 73.995 | 78.781 | 1.00 | 29.41 | B | C |
| ATOM | 2776 | CD2 | TYR | B | 56 | 12.659 | 73.962 | 81.353 | 1.00 | 29.02 | B | C |
| ATOM | 2777 | CE2 | TYR | B | 56 | 11.918 | 73.442 | 80.293 | 1.00 | 28.75 | B | C |

| | | | | | | | | | | | | |
|---|---|---|---|---|---|---|---|---|---|---|---|---|
| ATOM | 2778 | CZ | TYR | B | 56 | 12.442 | 73.463 | 79.016 | 1.00 | 28.31 | B | C |
| ATOM | 2779 | OH | TYR | B | 56 | 11.715 | 72.944 | 77.977 | 1.00 | 28.95 | B | O |
| ATOM | 2780 | C | TYR | B | 56 | 15.394 | 77.210 | 81.233 | 1.00 | 36.33 | B | C |
| ATOM | 2781 | O | TYR | B | 56 | 14.689 | 77.652 | 80.329 | 1.00 | 36.12 | B | O |
| ATOM | 2782 | N | THR | B | 57 | 16.722 | 77.265 | 81.212 | 1.00 | 38.39 | B | N |
| ATOM | 2783 | CA | THR | B | 57 | 17.442 | 77.898 | 80.115 | 1.00 | 40.35 | B | C |
| ATOM | 2784 | CB | THR | B | 57 | 17.645 | 79.387 | 80.455 | 1.00 | 40.96 | B | C |
| ATOM | 2785 | OG1 | THR | B | 57 | 18.300 | 80.057 | 79.371 | 1.00 | 42.01 | B | O |
| ATOM | 2786 | CG2 | THR | B | 57 | 18.449 | 79.522 | 81.740 | 1.00 | 39.97 | B | C |
| ATOM | 2787 | C | THR | B | 57 | 18.785 | 77.191 | 79.891 | 1.00 | 40.83 | B | C |
| ATOM | 2788 | O | THR | B | 57 | 19.117 | 76.248 | 80.606 | 1.00 | 42.14 | B | O |
| ATOM | 2789 | N | ASP | B | 58 | 19.546 | 77.644 | 78.897 | 1.00 | 41.24 | B | N |
| ATOM | 2790 | CA | ASP | B | 58 | 20.846 | 77.047 | 78.573 | 1.00 | 41.80 | B | C |
| ATOM | 2791 | CB | ASP | B | 58 | 21.832 | 77.160 | 79.750 | 1.00 | 43.00 | B | C |
| ATOM | 2792 | CG | ASP | B | 58 | 21.991 | 78.579 | 80.262 | 1.00 | 45.46 | B | C |
| ATOM | 2793 | OD1 | ASP | B | 58 | 21.918 | 79.534 | 79.450 | 1.00 | 46.44 | B | O |
| ATOM | 2794 | OD2 | ASP | B | 58 | 22.215 | 78.730 | 81.489 | 1.00 | 46.33 | B | O |
| ATOM | 2795 | C | ASP | B | 58 | 20.657 | 75.572 | 78.253 | 1.00 | 41.14 | B | C |
| ATOM | 2796 | O | ASP | B | 58 | 21.258 | 74.708 | 78.887 | 1.00 | 40.66 | B | O |
| ATOM | 2797 | N | THR | B | 59 | 19.819 | 75.292 | 77.266 | 1.00 | 40.96 | B | N |
| ATOM | 2798 | CA | THR | B | 59 | 19.539 | 73.921 | 76.875 | 1.00 | 41.42 | B | C |
| ATOM | 2799 | CB | THR | B | 59 | 18.086 | 73.805 | 76.387 | 1.00 | 41.68 | B | C |
| ATOM | 2800 | OG1 | THR | B | 59 | 17.933 | 74.533 | 75.162 | 1.00 | 42.59 | B | O |
| ATOM | 2801 | CG2 | THR | B | 59 | 17.135 | 74.403 | 77.421 | 1.00 | 41.42 | B | C |
| ATOM | 2802 | C | THR | B | 59 | 20.483 | 73.457 | 75.772 | 1.00 | 41.72 | B | C |
| ATOM | 2803 | O | THR | B | 59 | 20.711 | 74.162 | 74.798 | 1.00 | 42.32 | B | O |
| ATOM | 2804 | N | LYS | B | 60 | 21.040 | 72.266 | 75.939 | 1.00 | 41.97 | B | N |
| ATOM | 2805 | CA | LYS | B | 60 | 21.945 | 71.713 | 74.942 | 1.00 | 42.41 | B | C |
| ATOM | 2806 | CB | LYS | B | 60 | 23.399 | 72.022 | 75.319 | 1.00 | 43.13 | B | C |
| ATOM | 2807 | CG | LYS | B | 60 | 23.771 | 71.600 | 76.735 | 1.00 | 44.49 | B | C |
| ATOM | 2808 | CD | LYS | B | 60 | 25.227 | 71.927 | 77.058 | 1.00 | 45.77 | B | C |
| ATOM | 2809 | CE | LYS | B | 60 | 25.538 | 73.416 | 76.849 | 1.00 | 46.79 | B | C |
| ATOM | 2810 | NZ | LYS | B | 60 | 24.883 | 74.323 | 77.843 | 1.00 | 47.25 | B | N |
| ATOM | 2811 | C | LYS | B | 60 | 21.730 | 70.211 | 74.881 | 1.00 | 41.48 | B | C |
| ATOM | 2812 | O | LYS | B | 60 | 21.292 | 69.604 | 75.852 | 1.00 | 41.14 | B | O |
| ATOM | 2813 | N | VAL | B | 61 | 22.020 | 69.622 | 73.729 | 1.00 | 40.90 | B | N |
| ATOM | 2814 | CA | VAL | B | 61 | 21.866 | 68.183 | 73.557 | 1.00 | 40.39 | B | C |
| ATOM | 2815 | CB | VAL | B | 61 | 21.902 | 67.789 | 72.078 | 1.00 | 39.66 | B | C |
| ATOM | 2816 | CG1 | VAL | B | 61 | 21.748 | 66.283 | 71.946 | 1.00 | 40.11 | B | C |
| ATOM | 2817 | CG2 | VAL | B | 61 | 20.804 | 68.514 | 71.326 | 1.00 | 39.16 | B | C |
| ATOM | 2818 | C | VAL | B | 61 | 23.012 | 67.469 | 74.247 | 1.00 | 40.21 | B | C |
| ATOM | 2819 | O | VAL | B | 61 | 24.129 | 67.948 | 74.222 | 1.00 | 39.99 | B | O |
| ATOM | 2820 | N | ILE | B | 62 | 22.724 | 66.329 | 74.872 | 1.00 | 40.28 | B | N |
| ATOM | 2821 | CA | ILE | B | 62 | 23.773 | 65.559 | 75.530 | 1.00 | 39.95 | B | C |
| ATOM | 2822 | CB | ILE | B | 62 | 23.764 | 65.741 | 77.072 | 1.00 | 39.71 | B | C |
| ATOM | 2823 | CG2 | ILE | B | 62 | 24.058 | 67.196 | 77.418 | 1.00 | 39.47 | B | C |
| ATOM | 2824 | CG1 | ILE | B | 62 | 22.417 | 65.342 | 77.666 | 1.00 | 39.02 | B | C |
| ATOM | 2825 | CD1 | ILE | B | 62 | 22.411 | 65.421 | 79.181 | 1.00 | 37.18 | B | C |
| ATOM | 2826 | C | ILE | B | 62 | 23.661 | 64.083 | 75.196 | 1.00 | 40.63 | B | C |
| ATOM | 2827 | O | ILE | B | 62 | 24.600 | 63.295 | 75.448 | 1.00 | 41.36 | B | O |
| ATOM | 2828 | N | GLY | B | 63 | 22.525 | 63.709 | 74.611 | 1.00 | 40.07 | B | N |
| ATOM | 2829 | CA | GLY | B | 63 | 22.306 | 62.323 | 74.254 | 1.00 | 38.83 | B | C |
| ATOM | 2830 | C | GLY | B | 63 | 21.210 | 62.119 | 73.225 | 1.00 | 39.22 | B | C |
| ATOM | 2831 | O | GLY | B | 63 | 20.388 | 62.995 | 72.981 | 1.00 | 38.22 | B | O |
| ATOM | 2832 | N | ASN | B | 64 | 21.207 | 60.935 | 72.625 | 1.00 | 40.15 | B | N |
| ATOM | 2833 | CA | ASN | B | 64 | 20.215 | 60.585 | 71.622 | 1.00 | 40.36 | B | C |
| ATOM | 2834 | CB | ASN | B | 64 | 20.879 | 60.498 | 70.249 | 1.00 | 42.53 | B | C |
| ATOM | 2835 | CG | ASN | B | 64 | 19.954 | 60.933 | 69.150 | 1.00 | 46.72 | B | C |
| ATOM | 2836 | OD1 | ASN | B | 64 | 18.820 | 60.456 | 69.063 | 1.00 | 50.94 | B | O |
| ATOM | 2837 | ND2 | ASN | B | 64 | 20.417 | 61.851 | 68.303 | 1.00 | 48.26 | B | N |
| ATOM | 2838 | C | ASN | B | 64 | 19.606 | 59.237 | 72.011 | 1.00 | 38.17 | B | C |
| ATOM | 2839 | O | ASN | B | 64 | 20.119 | 58.574 | 72.875 | 1.00 | 37.75 | B | O |
| ATOM | 2840 | N | GLY | B | 65 | 18.509 | 58.848 | 71.369 | 1.00 | 36.95 | B | N |

| | | | | | | | | | | | | |
|---|---|---|---|---|---|---|---|---|---|---|---|---|
| ATOM | 2841 | CA | GLY | B | 65 | 17.885 | 57.579 | 71.696 | 1.00 | 35.86 | B | C |
| ATOM | 2842 | C | GLY | B | 65 | 16.640 | 57.348 | 70.858 | 1.00 | 36.18 | B | C |
| ATOM | 2843 | O | GLY | B | 65 | 16.101 | 58.268 | 70.247 | 1.00 | 37.93 | B | O |
| ATOM | 2844 | N | SER | B | 66 | 16.178 | 56.108 | 70.829 | 1.00 | 35.10 | B | N |
| ATOM | 2845 | CA | SER | B | 66 | 14.988 | 55.749 | 70.069 | 1.00 | 34.94 | B | C |
| ATOM | 2846 | CB | SER | B | 66 | 14.499 | 54.353 | 70.484 | 1.00 | 35.95 | B | C |
| ATOM | 2847 | OG | SER | B | 66 | 15.055 | 53.333 | 69.679 | 1.00 | 35.94 | B | O |
| ATOM | 2848 | C | SER | B | 66 | 13.820 | 56.721 | 70.263 | 1.00 | 34.71 | B | C |
| ATOM | 2849 | O | SER | B | 66 | 13.272 | 56.839 | 71.368 | 1.00 | 34.39 | B | O |
| ATOM | 2850 | N | PHE | B | 67 | 13.436 | 57.401 | 69.190 | 1.00 | 33.84 | B | N |
| ATOM | 2851 | CA | PHE | B | 67 | 12.308 | 58.320 | 69.231 | 1.00 | 32.96 | B | C |
| ATOM | 2852 | CB | PHE | B | 67 | 11.048 | 57.553 | 69.629 | 1.00 | 33.92 | B | C |
| ATOM | 2853 | CG | PHE | B | 67 | 10.594 | 56.574 | 68.594 | 1.00 | 34.01 | B | C |
| ATOM | 2854 | CD1 | PHE | B | 67 | 10.151 | 55.306 | 68.957 | 1.00 | 33.42 | B | C |
| ATOM | 2855 | CD2 | PHE | B | 67 | 10.590 | 56.935 | 67.248 | 1.00 | 34.03 | B | C |
| ATOM | 2856 | CE1 | PHE | B | 67 | 9.708 | 54.409 | 67.989 | 1.00 | 34.42 | B | C |
| ATOM | 2857 | CE2 | PHE | B | 67 | 10.154 | 56.057 | 66.273 | 1.00 | 32.30 | B | C |
| ATOM | 2858 | CZ | PHE | B | 67 | 9.709 | 54.790 | 66.639 | 1.00 | 33.72 | B | C |
| ATOM | 2859 | C | PHE | B | 67 | 12.462 | 59.513 | 70.149 | 1.00 | 32.00 | B | C |
| ATOM | 2860 | O | PHE | B | 67 | 11.473 | 60.083 | 70.572 | 1.00 | 31.80 | B | O |
| ATOM | 2861 | N | GLY | B | 68 | 13.690 | 59.894 | 70.462 | 1.00 | 30.68 | B | N |
| ATOM | 2862 | CA | GLY | B | 68 | 13.842 | 61.037 | 71.339 | 1.00 | 31.23 | B | C |
| ATOM | 2863 | C | GLY | B | 68 | 15.241 | 61.572 | 71.516 | 1.00 | 30.46 | B | C |
| ATOM | 2864 | O | GLY | B | 68 | 16.189 | 61.050 | 70.978 | 1.00 | 31.01 | B | O |
| ATOM | 2865 | N | VAL | B | 69 | 15.341 | 62.629 | 72.312 | 1.00 | 29.66 | B | N |
| ATOM | 2866 | CA | VAL | B | 69 | 16.606 | 63.277 | 72.579 | 1.00 | 27.83 | B | C |
| ATOM | 2867 | CB | VAL | B | 69 | 16.748 | 64.554 | 71.717 | 1.00 | 28.08 | B | C |
| ATOM | 2868 | CG1 | VAL | B | 69 | 18.135 | 65.154 | 71.887 | 1.00 | 25.91 | B | C |
| ATOM | 2869 | CG2 | VAL | B | 69 | 16.467 | 64.228 | 70.259 | 1.00 | 26.53 | B | C |
| ATOM | 2870 | C | VAL | B | 69 | 16.663 | 63.661 | 74.054 | 1.00 | 28.85 | B | C |
| ATOM | 2871 | O | VAL | B | 69 | 15.633 | 63.803 | 74.716 | 1.00 | 26.81 | B | O |
| ATOM | 2872 | N | VAL | B | 70 | 17.878 | 63.829 | 74.560 | 1.00 | 28.60 | B | N |
| ATOM | 2873 | CA | VAL | B | 70 | 18.073 | 64.210 | 75.946 | 1.00 | 28.61 | B | C |
| ATOM | 2874 | CB | VAL | B | 70 | 18.832 | 63.129 | 76.724 | 1.00 | 28.25 | B | C |
| ATOM | 2875 | CG1 | VAL | B | 70 | 18.542 | 63.262 | 78.205 | 1.00 | 27.74 | B | C |
| ATOM | 2876 | CG2 | VAL | B | 70 | 18.445 | 61.760 | 76.222 | 1.00 | 29.37 | B | C |
| ATOM | 2877 | C | VAL | B | 70 | 18.893 | 65.490 | 75.963 | 1.00 | 29.77 | B | C |
| ATOM | 2878 | O | VAL | B | 70 | 20.013 | 65.534 | 75.431 | 1.00 | 28.78 | B | O |
| ATOM | 2879 | N | TYR | B | 71 | 18.326 | 66.535 | 76.558 | 1.00 | 30.50 | B | N |
| ATOM | 2880 | CA | TYR | B | 71 | 19.006 | 67.819 | 76.648 | 1.00 | 31.25 | B | C |
| ATOM | 2881 | CB | TYR | B | 71 | 18.087 | 68.968 | 76.220 | 1.00 | 31.70 | B | C |
| ATOM | 2882 | CG | TYR | B | 71 | 17.411 | 68.801 | 74.879 | 1.00 | 33.21 | B | C |
| ATOM | 2883 | CD1 | TYR | B | 71 | 16.358 | 67.905 | 74.715 | 1.00 | 32.74 | B | C |
| ATOM | 2884 | CE1 | TYR | B | 71 | 15.722 | 67.761 | 73.491 | 1.00 | 33.81 | B | C |
| ATOM | 2885 | CD2 | TYR | B | 71 | 17.811 | 69.554 | 73.780 | 1.00 | 33.50 | B | C |
| ATOM | 2886 | CE2 | TYR | B | 71 | 17.182 | 69.418 | 72.549 | 1.00 | 33.71 | B | C |
| ATOM | 2887 | CZ | TYR | B | 71 | 16.134 | 68.520 | 72.412 | 1.00 | 34.48 | B | C |
| ATOM | 2888 | OH | TYR | B | 71 | 15.493 | 68.370 | 71.201 | 1.00 | 36.82 | B | O |
| ATOM | 2889 | C | TYR | B | 71 | 19.400 | 68.060 | 78.089 | 1.00 | 31.39 | B | C |
| ATOM | 2890 | O | TYR | B | 71 | 18.910 | 67.395 | 78.992 | 1.00 | 32.09 | B | O |
| ATOM | 2891 | N | GLN | B | 72 | 20.309 | 69.003 | 78.297 | 1.00 | 30.90 | B | N |
| ATOM | 2892 | CA | GLN | B | 72 | 20.699 | 69.371 | 79.647 | 1.00 | 30.66 | B | C |
| ATOM | 2893 | CB | GLN | B | 72 | 22.209 | 69.461 | 79.800 | 1.00 | 30.09 | B | C |
| ATOM | 2894 | CG | GLN | B | 72 | 22.616 | 69.902 | 81.191 | 1.00 | 30.34 | B | C |
| ATOM | 2895 | CD | GLN | B | 72 | 24.071 | 70.306 | 81.278 | 1.00 | 31.51 | B | C |
| ATOM | 2896 | OE1 | GLN | B | 72 | 24.491 | 71.254 | 80.643 | 1.00 | 31.18 | B | O |
| ATOM | 2897 | NE2 | GLN | B | 72 | 24.843 | 69.579 | 82.077 | 1.00 | 32.22 | B | N |
| ATOM | 2898 | C | GLN | B | 72 | 20.117 | 70.763 | 79.773 | 1.00 | 31.30 | B | C |
| ATOM | 2899 | O | GLN | B | 72 | 20.010 | 71.468 | 78.773 | 1.00 | 30.92 | B | O |
| ATOM | 2900 | N | ALA | B | 73 | 19.741 | 71.161 | 80.981 | 1.00 | 31.44 | B | N |
| ATOM | 2901 | CA | ALA | B | 73 | 19.171 | 72.489 | 81.177 | 1.00 | 32.11 | B | C |
| ATOM | 2902 | CB | ALA | B | 73 | 17.676 | 72.474 | 80.852 | 1.00 | 31.04 | B | C |
| ATOM | 2903 | C | ALA | B | 73 | 19.391 | 72.952 | 82.605 | 1.00 | 33.46 | B | C |

| | | | | | | | | | | | | |
|---|---|---|---|---|---|---|---|---|---|---|---|---|
| ATOM | 2904 | O | ALA | B | 73 | 19.699 | 72.150 | 83.478 | 1.00 | 32.81 | B | O |
| ATOM | 2905 | N | LYS | B | 74 | 19.228 | 74.256 | 82.828 | 1.00 | 35.22 | B | N |
| ATOM | 2906 | CA | LYS | B | 74 | 19.407 | 74.847 | 84.150 | 1.00 | 35.87 | B | C |
| ATOM | 2907 | CB | LYS | B | 74 | 20.454 | 75.976 | 84.098 | 1.00 | 37.42 | B | C |
| ATOM | 2908 | CG | LYS | B | 74 | 21.090 | 76.331 | 85.455 | 1.00 | 38.63 | B | C |
| ATOM | 2909 | CD | LYS | B | 74 | 21.909 | 77.628 | 85.403 | 1.00 | 40.43 | B | C |
| ATOM | 2910 | CE | LYS | B | 74 | 20.992 | 78.874 | 85.369 | 1.00 | 43.61 | B | C |
| ATOM | 2911 | NZ | LYS | B | 74 | 21.709 | 80.194 | 85.264 | 1.00 | 43.36 | B | N |
| ATOM | 2912 | C | LYS | B | 74 | 18.070 | 75.416 | 84.623 | 1.00 | 36.31 | B | C |
| ATOM | 2913 | O | LYS | B | 74 | 17.430 | 76.210 | 83.916 | 1.00 | 36.29 | B | O |
| ATOM | 2914 | N | LEU | B | 75 | 17.638 | 74.993 | 85.806 | 1.00 | 36.45 | B | N |
| ATOM | 2915 | CA | LEU | B | 75 | 16.392 | 75.485 | 86.370 | 1.00 | 37.19 | B | C |
| ATOM | 2916 | CB | LEU | B | 75 | 16.022 | 74.665 | 87.604 | 1.00 | 35.56 | B | C |
| ATOM | 2917 | CG | LEU | B | 75 | 15.801 | 73.173 | 87.337 | 1.00 | 34.79 | B | C |
| ATOM | 2918 | CD1 | LEU | B | 75 | 15.468 | 72.455 | 88.639 | 1.00 | 33.11 | B | C |
| ATOM | 2919 | CD2 | LEU | B | 75 | 14.679 | 72.997 | 86.316 | 1.00 | 32.99 | B | C |
| ATOM | 2920 | C | LEU | B | 75 | 16.646 | 76.941 | 86.752 | 1.00 | 38.58 | B | C |
| ATOM | 2921 | O | LEU | B | 75 | 17.580 | 77.236 | 87.505 | 1.00 | 39.12 | B | O |
| ATOM | 2922 | N | CYS | B | 76 | 15.823 | 77.846 | 86.233 | 1.00 | 39.46 | B | N |
| ATOM | 2923 | CA | CYS | B | 76 | 15.985 | 79.275 | 86.500 | 1.00 | 40.69 | B | C |
| ATOM | 2924 | CB | CYS | B | 76 | 14.875 | 80.060 | 85.808 | 1.00 | 38.41 | B | C |
| ATOM | 2925 | SG | CYS | B | 76 | 15.046 | 80.024 | 84.032 | 1.00 | 39.56 | B | S |
| ATOM | 2926 | C | CYS | B | 76 | 16.071 | 79.710 | 87.962 | 1.00 | 42.06 | B | C |
| ATOM | 2927 | O | CYS | B | 76 | 16.948 | 80.505 | 88.323 | 1.00 | 42.20 | B | O |
| ATOM | 2928 | N | ASP | B | 77 | 15.183 | 79.202 | 88.808 | 1.00 | 43.56 | B | N |
| ATOM | 2929 | CA | ASP | B | 77 | 15.207 | 79.607 | 90.209 | 1.00 | 45.52 | B | C |
| ATOM | 2930 | CB | ASP | B | 77 | 13.868 | 79.273 | 90.897 | 1.00 | 47.95 | B | C |
| ATOM | 2931 | CG | ASP | B | 77 | 13.495 | 77.797 | 90.794 | 1.00 | 49.88 | B | C |
| ATOM | 2932 | OD1 | ASP | B | 77 | 13.813 | 77.177 | 89.751 | 1.00 | 51.56 | B | O |
| ATOM | 2933 | OD2 | ASP | B | 77 | 12.866 | 77.260 | 91.744 | 1.00 | 50.61 | B | O |
| ATOM | 2934 | C | ASP | B | 77 | 16.366 | 79.035 | 91.011 | 1.00 | 45.85 | B | C |
| ATOM | 2935 | O | ASP | B | 77 | 17.146 | 79.793 | 91.598 | 1.00 | 47.71 | B | O |
| ATOM | 2936 | N | SER | B | 78 | 16.492 | 77.710 | 91.040 | 1.00 | 44.95 | B | N |
| ATOM | 2937 | CA | SER | B | 78 | 17.561 | 77.069 | 91.808 | 1.00 | 43.46 | B | C |
| ATOM | 2938 | CB | SER | B | 78 | 17.155 | 75.644 | 92.192 | 1.00 | 43.80 | B | C |
| ATOM | 2939 | OG | SER | B | 78 | 17.043 | 74.813 | 91.046 | 1.00 | 42.95 | B | O |
| ATOM | 2940 | C | SER | B | 78 | 18.903 | 77.020 | 91.078 | 1.00 | 43.16 | B | C |
| ATOM | 2941 | O | SER | B | 78 | 19.928 | 76.723 | 91.684 | 1.00 | 44.41 | B | O |
| ATOM | 2942 | N | GLY | B | 79 | 18.900 | 77.308 | 89.782 | 1.00 | 41.71 | B | N |
| ATOM | 2943 | CA | GLY | B | 79 | 20.142 | 77.260 | 89.032 | 1.00 | 41.11 | B | C |
| ATOM | 2944 | C | GLY | B | 79 | 20.673 | 75.840 | 88.883 | 1.00 | 40.59 | B | C |
| ATOM | 2945 | O | GLY | B | 79 | 21.739 | 75.631 | 88.335 | 1.00 | 40.52 | B | O |
| ATOM | 2946 | N | GLU | B | 80 | 19.920 | 74.865 | 89.379 | 1.00 | 39.43 | B | N |
| ATOM | 2947 | CA | GLU | B | 80 | 20.313 | 73.462 | 89.289 | 1.00 | 39.79 | B | C |
| ATOM | 2948 | CB | GLU | B | 80 | 19.420 | 72.605 | 90.181 | 1.00 | 40.68 | B | C |
| ATOM | 2949 | CG | GLU | B | 80 | 19.741 | 72.683 | 91.650 | 1.00 | 44.91 | B | C |
| ATOM | 2950 | CD | GLU | B | 80 | 18.612 | 72.142 | 92.503 | 1.00 | 46.94 | B | C |
| ATOM | 2951 | OE1 | GLU | B | 80 | 18.094 | 71.045 | 92.171 | 1.00 | 46.01 | B | O |
| ATOM | 2952 | OE2 | GLU | B | 80 | 18.255 | 72.816 | 93.505 | 1.00 | 48.33 | B | O |
| ATOM | 2953 | C | GLU | B | 80 | 20.234 | 72.906 | 87.872 | 1.00 | 37.73 | B | C |
| ATOM | 2954 | O | GLU | B | 80 | 19.337 | 73.270 | 87.093 | 1.00 | 37.55 | B | O |
| ATOM | 2955 | N | LEU | B | 81 | 21.165 | 72.010 | 87.552 | 1.00 | 34.45 | B | N |
| ATOM | 2956 | CA | LEU | B | 81 | 21.195 | 71.375 | 86.243 | 1.00 | 32.16 | B | C |
| ATOM | 2957 | CB | LEU | B | 81 | 22.629 | 70.994 | 85.872 | 1.00 | 32.40 | B | C |
| ATOM | 2958 | CG | LEU | B | 81 | 23.622 | 72.154 | 85.748 | 1.00 | 33.28 | B | C |
| ATOM | 2959 | CD1 | LEU | B | 81 | 25.048 | 71.618 | 85.757 | 1.00 | 32.03 | B | C |
| ATOM | 2960 | CD2 | LEU | B | 81 | 23.342 | 72.937 | 84.471 | 1.00 | 32.23 | B | C |
| ATOM | 2961 | C | LEU | B | 81 | 20.314 | 70.123 | 86.249 | 1.00 | 30.49 | B | C |
| ATOM | 2962 | O | LEU | B | 81 | 20.349 | 69.331 | 87.182 | 1.00 | 29.49 | B | O |
| ATOM | 2963 | N | VAL | B | 82 | 19.519 | 69.970 | 85.194 | 1.00 | 28.84 | B | N |
| ATOM | 2964 | CA | VAL | B | 82 | 18.624 | 68.833 | 85.038 | 1.00 | 26.86 | B | C |
| ATOM | 2965 | CB | VAL | B | 82 | 17.144 | 69.219 | 85.249 | 1.00 | 27.13 | B | C |
| ATOM | 2966 | CG1 | VAL | B | 82 | 16.892 | 69.637 | 86.692 | 1.00 | 27.54 | B | C |

| | | | | | | | | | | | | |
|---|---|---|---|---|---|---|---|---|---|---|---|---|
| ATOM | 2967 | CG2 | VAL | B | 82 | 16.773 | 70.338 | 84.287 | 1.00 | 27.24 | B | C |
| ATOM | 2968 | C | VAL | B | 82 | 18.734 | 68.328 | 83.609 | 1.00 | 26.54 | B | C |
| ATOM | 2969 | O | VAL | B | 82 | 19.213 | 69.030 | 82.724 | 1.00 | 26.19 | B | O |
| ATOM | 2970 | N | ALA | B | 83 | 18.274 | 67.103 | 83.396 | 1.00 | 24.80 | B | N |
| ATOM | 2971 | CA | ALA | B | 83 | 18.283 | 66.499 | 82.079 | 1.00 | 24.83 | B | C |
| ATOM | 2972 | CB | ALA | B | 83 | 18.895 | 65.108 | 82.144 | 1.00 | 22.79 | B | C |
| ATOM | 2973 | C | ALA | B | 83 | 16.829 | 66.410 | 81.650 | 1.00 | 25.03 | B | C |
| ATOM | 2974 | O | ALA | B | 83 | 15.958 | 66.160 | 82.470 | 1.00 | 26.82 | B | O |
| ATOM | 2975 | N | ILE | B | 84 | 16.569 | 66.633 | 80.369 | 1.00 | 24.30 | B | N |
| ATOM | 2976 | CA | ILE | B | 84 | 15.207 | 66.551 | 79.866 | 1.00 | 24.79 | B | C |
| ATOM | 2977 | CB | ILE | B | 84 | 14.705 | 67.921 | 79.332 | 1.00 | 24.79 | B | C |
| ATOM | 2978 | CG2 | ILE | B | 84 | 13.295 | 67.782 | 78.766 | 1.00 | 23.59 | B | C |
| ATOM | 2979 | CG1 | ILE | B | 84 | 14.724 | 68.954 | 80.460 | 1.00 | 23.42 | B | C |
| ATOM | 2980 | CD1 | ILE | B | 84 | 14.215 | 70.312 | 80.053 | 1.00 | 24.56 | B | C |
| ATOM | 2981 | C | ILE | B | 84 | 15.151 | 65.528 | 78.746 | 1.00 | 24.67 | B | C |
| ATOM | 2982 | O | ILE | B | 84 | 15.795 | 65.685 | 77.729 | 1.00 | 25.63 | B | O |
| ATOM | 2983 | N | LYS | B | 85 | 14.377 | 64.471 | 78.959 | 1.00 | 24.66 | B | N |
| ATOM | 2984 | CA | LYS | B | 85 | 14.237 | 63.423 | 77.962 | 1.00 | 24.96 | B | C |
| ATOM | 2985 | CB | LYS | B | 85 | 14.244 | 62.053 | 78.638 | 1.00 | 23.28 | B | C |
| ATOM | 2986 | CG | LYS | B | 85 | 14.175 | 60.884 | 77.675 | 1.00 | 21.99 | B | C |
| ATOM | 2987 | CD | LYS | B | 85 | 14.436 | 59.565 | 78.391 | 1.00 | 20.16 | B | C |
| ATOM | 2988 | CE | LYS | B | 85 | 14.304 | 58.402 | 77.447 | 1.00 | 19.31 | B | C |
| ATOM | 2989 | NZ | LYS | B | 85 | 14.751 | 57.127 | 78.039 | 1.00 | 20.35 | B | N |
| ATOM | 2990 | C | LYS | B | 85 | 12.933 | 63.654 | 77.209 | 1.00 | 26.55 | B | C |
| ATOM | 2991 | O | LYS | B | 85 | 11.837 | 63.563 | 77.775 | 1.00 | 25.23 | B | O |
| ATOM | 2992 | N | LYS | B | 86 | 13.075 | 63.955 | 75.923 | 1.00 | 27.62 | B | N |
| ATOM | 2993 | CA | LYS | B | 86 | 11.948 | 64.244 | 75.051 | 1.00 | 28.89 | B | C |
| ATOM | 2994 | CB | LYS | B | 86 | 12.239 | 65.534 | 74.284 | 1.00 | 28.86 | B | C |
| ATOM | 2995 | CG | LYS | B | 86 | 11.039 | 66.192 | 73.631 | 1.00 | 31.54 | B | C |
| ATOM | 2996 | CD | LYS | B | 86 | 11.464 | 67.509 | 73.009 | 1.00 | 31.29 | B | C |
| ATOM | 2997 | CE | LYS | B | 86 | 10.275 | 68.319 | 72.534 | 1.00 | 33.43 | B | C |
| ATOM | 2998 | NZ | LYS | B | 86 | 10.699 | 69.670 | 72.045 | 1.00 | 35.19 | B | N |
| ATOM | 2999 | C | LYS | B | 86 | 11.695 | 63.108 | 74.066 | 1.00 | 29.90 | B | C |
| ATOM | 3000 | O | LYS | B | 86 | 12.466 | 62.911 | 73.140 | 1.00 | 31.52 | B | O |
| ATOM | 3001 | N | VAL | B | 87 | 10.619 | 62.355 | 74.281 | 1.00 | 30.70 | B | N |
| ATOM | 3002 | CA | VAL | B | 87 | 10.275 | 61.266 | 73.380 | 1.00 | 31.49 | B | C |
| ATOM | 3003 | CB | VAL | B | 87 | 10.234 | 59.887 | 74.094 | 1.00 | 31.38 | B | C |
| ATOM | 3004 | CG1 | VAL | B | 87 | 11.566 | 59.609 | 74.772 | 1.00 | 31.79 | B | C |
| ATOM | 3005 | CG2 | VAL | B | 87 | 9.091 | 59.839 | 75.095 | 1.00 | 30.40 | B | C |
| ATOM | 3006 | C | VAL | B | 87 | 8.912 | 61.504 | 72.746 | 1.00 | 33.21 | B | C |
| ATOM | 3007 | O | VAL | B | 87 | 8.030 | 62.136 | 73.325 | 1.00 | 32.82 | B | O |
| ATOM | 3008 | N | LEU | B | 88 | 8.762 | 60.998 | 71.532 | 1.00 | 34.72 | B | N |
| ATOM | 3009 | CA | LEU | B | 88 | 7.516 | 61.106 | 70.804 | 1.00 | 37.39 | B | C |
| ATOM | 3010 | CB | LEU | B | 88 | 7.678 | 60.463 | 69.434 | 1.00 | 37.72 | B | C |
| ATOM | 3011 | CG | LEU | B | 88 | 6.399 | 60.346 | 68.617 | 1.00 | 37.64 | B | C |
| ATOM | 3012 | CD1 | LEU | B | 88 | 6.031 | 61.723 | 68.064 | 1.00 | 37.34 | B | C |
| ATOM | 3013 | CD2 | LEU | B | 88 | 6.618 | 59.331 | 67.495 | 1.00 | 39.26 | B | C |
| ATOM | 3014 | C | LEU | B | 88 | 6.458 | 60.344 | 71.600 | 1.00 | 38.79 | B | C |
| ATOM | 3015 | O | LEU | B | 88 | 6.735 | 59.261 | 72.117 | 1.00 | 38.51 | B | O |
| ATOM | 3016 | N | GLN | B | 89 | 5.250 | 60.898 | 71.691 | 1.00 | 40.50 | B | N |
| ATOM | 3017 | CA | GLN | B | 89 | 4.177 | 60.243 | 72.438 | 1.00 | 44.17 | B | C |
| ATOM | 3018 | CB | GLN | B | 89 | 3.959 | 60.963 | 73.771 | 1.00 | 44.86 | B | C |
| ATOM | 3019 | CG | GLN | B | 89 | 2.828 | 60.402 | 74.633 | 1.00 | 46.81 | B | C |
| ATOM | 3020 | CD | GLN | B | 89 | 3.037 | 58.938 | 75.001 | 1.00 | 48.36 | B | C |
| ATOM | 3021 | OE1 | GLN | B | 89 | 4.168 | 58.423 | 74.964 | 1.00 | 48.42 | B | O |
| ATOM | 3022 | NE2 | GLN | B | 89 | 1.950 | 58.260 | 75.377 | 1.00 | 49.10 | B | N |
| ATOM | 3023 | C | GLN | B | 89 | 2.861 | 60.227 | 71.672 | 1.00 | 46.16 | B | C |
| ATOM | 3024 | O | GLN | B | 89 | 2.511 | 61.201 | 71.008 | 1.00 | 47.77 | B | O |
| ATOM | 3025 | N | ASP | B | 90 | 2.133 | 59.121 | 71.758 | 1.00 | 47.82 | B | N |
| ATOM | 3026 | CA | ASP | B | 90 | 0.836 | 59.048 | 71.096 | 1.00 | 50.10 | B | C |
| ATOM | 3027 | CB | ASP | B | 90 | 0.833 | 57.991 | 69.991 | 1.00 | 52.08 | B | C |
| ATOM | 3028 | CG | ASP | B | 90 | -0.449 | 58.018 | 69.170 | 1.00 | 54.43 | B | C |
| ATOM | 3029 | OD1 | ASP | B | 90 | -1.529 | 57.763 | 69.762 | 1.00 | 54.41 | B | O |

| | | | | | | | | | | | | |
|---|---|---|---|---|---|---|---|---|---|---|---|---|
| ATOM | 3030 | OD2 | ASP | B | 90 | -0.374 | 58.303 | 67.943 | 1.00 | 54.89 | B | O |
| ATOM | 3031 | C | ASP | B | 90 | -0.229 | 58.719 | 72.147 | 1.00 | 50.67 | B | C |
| ATOM | 3032 | O | ASP | B | 90 | -0.012 | 57.854 | 73.033 | 1.00 | 48.83 | B | O |
| ATOM | 3033 | N | ALA | B | 91 | -1.371 | 59.410 | 72.045 | 1.00 | 51.54 | B | N |
| ATOM | 3034 | CA | ALA | B | 91 | -2.492 | 59.245 | 72.979 | 1.00 | 51.47 | B | C |
| ATOM | 3035 | CB | ALA | B | 91 | -3.516 | 60.360 | 72.759 | 1.00 | 51.46 | B | C |
| ATOM | 3036 | C | ALA | B | 91 | -3.191 | 57.881 | 72.929 | 1.00 | 51.09 | B | C |
| ATOM | 3037 | O | ALA | B | 91 | -4.097 | 57.610 | 73.729 | 1.00 | 50.84 | B | O |
| ATOM | 3038 | N | ALA | B | 92 | -2.772 | 57.023 | 72.003 | 1.00 | 50.96 | B | N |
| ATOM | 3039 | CA | ALA | B | 92 | -3.366 | 55.695 | 71.887 | 1.00 | 50.64 | B | C |
| ATOM | 3040 | CB | ALA | B | 92 | -2.821 | 54.967 | 70.661 | 1.00 | 50.30 | B | C |
| ATOM | 3041 | C | ALA | B | 92 | -3.106 | 54.871 | 73.148 | 1.00 | 50.22 | B | C |
| ATOM | 3042 | O | ALA | B | 92 | -3.922 | 54.054 | 73.532 | 1.00 | 50.82 | B | O |
| ATOM | 3043 | N | ALA | B | 93 | -1.969 | 55.095 | 73.799 | 1.00 | 49.50 | B | N |
| ATOM | 3044 | CA | ALA | B | 93 | -1.665 | 54.340 | 75.010 | 1.00 | 48.41 | B | C |
| ATOM | 3045 | CB | ALA | B | 93 | -0.810 | 53.110 | 74.664 | 1.00 | 49.00 | B | C |
| ATOM | 3046 | C | ALA | B | 93 | -0.969 | 55.191 | 76.072 | 1.00 | 47.28 | B | C |
| ATOM | 3047 | O | ALA | B | 93 | -0.657 | 56.364 | 75.849 | 1.00 | 46.84 | B | O |
| ATOM | 3048 | N | LYS | B | 94 | -0.733 | 54.586 | 77.231 | 1.00 | 45.33 | B | N |
| ATOM | 3049 | CA | LYS | B | 94 | -0.084 | 55.265 | 78.349 | 1.00 | 43.20 | B | C |
| ATOM | 3050 | CB | LYS | B | 94 | -0.685 | 54.752 | 79.659 | 1.00 | 45.03 | B | C |
| ATOM | 3051 | CG | LYS | B | 94 | -0.206 | 55.481 | 80.896 | 1.00 | 48.73 | B | C |
| ATOM | 3052 | CD | LYS | B | 94 | -0.813 | 54.888 | 82.171 | 1.00 | 50.90 | B | C |
| ATOM | 3053 | CE | LYS | B | 94 | -2.270 | 55.344 | 82.373 | 1.00 | 52.71 | B | C |
| ATOM | 3054 | NZ | LYS | B | 94 | -2.781 | 54.980 | 83.737 | 1.00 | 53.37 | B | N |
| ATOM | 3055 | C | LYS | B | 94 | 1.415 | 54.970 | 78.289 | 1.00 | 40.73 | B | C |
| ATOM | 3056 | O | LYS | B | 94 | 1.804 | 53.832 | 78.099 | 1.00 | 40.73 | B | O |
| ATOM | 3057 | N | ASN | B | 95 | 2.251 | 55.998 | 78.440 | 1.00 | 37.76 | B | N |
| ATOM | 3058 | CA | ASN | B | 95 | 3.705 | 55.814 | 78.373 | 1.00 | 34.01 | B | C |
| ATOM | 3059 | CB | ASN | B | 95 | 4.419 | 57.157 | 78.361 | 1.00 | 31.80 | B | C |
| ATOM | 3060 | CG | ASN | B | 95 | 5.904 | 57.002 | 78.168 | 1.00 | 31.14 | B | C |
| ATOM | 3061 | OD1 | ASN | B | 95 | 6.606 | 56.530 | 79.060 | 1.00 | 29.75 | B | O |
| ATOM | 3062 | ND2 | ASN | B | 95 | 6.391 | 57.373 | 76.986 | 1.00 | 27.24 | B | N |
| ATOM | 3063 | C | ASN | B | 95 | 4.214 | 54.973 | 79.530 | 1.00 | 31.59 | B | C |
| ATOM | 3064 | O | ASN | B | 95 | 4.159 | 55.384 | 80.689 | 1.00 | 32.00 | B | O |
| ATOM | 3065 | N | ARG | B | 96 | 4.721 | 53.793 | 79.204 | 1.00 | 29.01 | B | N |
| ATOM | 3066 | CA | ARG | B | 96 | 5.221 | 52.885 | 80.219 | 1.00 | 27.71 | B | C |
| ATOM | 3067 | CB | ARG | B | 96 | 5.584 | 51.540 | 79.602 | 1.00 | 28.48 | B | C |
| ATOM | 3068 | CG | ARG | B | 96 | 5.576 | 50.417 | 80.619 | 1.00 | 32.19 | B | C |
| ATOM | 3069 | CD | ARG | B | 96 | 6.533 | 49.309 | 80.248 | 1.00 | 34.58 | B | C |
| ATOM | 3070 | NE | ARG | B | 96 | 6.256 | 48.687 | 78.957 | 1.00 | 39.97 | B | N |
| ATOM | 3071 | CZ | ARG | B | 96 | 5.214 | 47.895 | 78.697 | 1.00 | 42.25 | B | C |
| ATOM | 3072 | NH1 | ARG | B | 96 | 4.319 | 47.615 | 79.642 | 1.00 | 45.03 | B | N |
| ATOM | 3073 | NH2 | ARG | B | 96 | 5.079 | 47.355 | 77.493 | 1.00 | 41.46 | B | N |
| ATOM | 3074 | C | ARG | B | 96 | 6.417 | 53.443 | 80.998 | 1.00 | 27.16 | B | C |
| ATOM | 3075 | O | ARG | B | 96 | 6.463 | 53.341 | 82.217 | 1.00 | 26.17 | B | O |
| ATOM | 3076 | N | GLU | B | 97 | 7.375 | 54.041 | 80.295 | 1.00 | 24.68 | B | N |
| ATOM | 3077 | CA | GLU | B | 97 | 8.550 | 54.598 | 80.953 | 1.00 | 23.54 | B | C |
| ATOM | 3078 | CB | GLU | B | 97 | 9.456 | 55.287 | 79.927 | 1.00 | 21.38 | B | C |
| ATOM | 3079 | CG | GLU | B | 97 | 10.776 | 55.778 | 80.509 | 1.00 | 20.14 | B | C |
| ATOM | 3080 | CD | GLU | B | 97 | 11.763 | 56.260 | 79.454 | 1.00 | 17.80 | B | C |
| ATOM | 3081 | OE1 | GLU | B | 97 | 12.932 | 56.502 | 79.811 | 1.00 | 18.16 | B | O |
| ATOM | 3082 | OE2 | GLU | B | 97 | 11.379 | 56.402 | 78.277 | 1.00 | 19.14 | B | O |
| ATOM | 3083 | C | GLU | B | 97 | 8.125 | 55.599 | 82.031 | 1.00 | 22.96 | B | C |
| ATOM | 3084 | O | GLU | B | 97 | 8.625 | 55.568 | 83.140 | 1.00 | 22.16 | B | O |
| ATOM | 3085 | N | LEU | B | 98 | 7.198 | 56.487 | 81.690 | 1.00 | 23.57 | B | N |
| ATOM | 3086 | CA | LEU | B | 98 | 6.724 | 57.483 | 82.639 | 1.00 | 23.39 | B | C |
| ATOM | 3087 | CB | LEU | B | 98 | 5.718 | 58.431 | 81.973 | 1.00 | 24.66 | B | C |
| ATOM | 3088 | CG | LEU | B | 98 | 4.917 | 59.349 | 82.913 | 1.00 | 23.96 | B | C |
| ATOM | 3089 | CD1 | LEU | B | 98 | 5.857 | 60.302 | 83.634 | 1.00 | 23.87 | B | C |
| ATOM | 3090 | CD2 | LEU | B | 98 | 3.889 | 60.140 | 82.119 | 1.00 | 25.69 | B | C |
| ATOM | 3091 | C | LEU | B | 98 | 6.064 | 56.827 | 83.842 | 1.00 | 24.79 | B | C |
| ATOM | 3092 | O | LEU | B | 98 | 6.366 | 57.160 | 84.986 | 1.00 | 22.59 | B | O |

| | | | | | | | | | | | | |
|---|---|---|---|---|---|---|---|---|---|---|---|---|
| ATOM | 3093 | N | GLN | B | 99 | 5.161 | 55.891 | 83.561 | 1.00 | 26.62 | B | N |
| ATOM | 3094 | CA | GLN | B | 99 | 4.443 | 55.177 | 84.604 | 1.00 | 28.24 | B | C |
| ATOM | 3095 | CB | GLN | B | 99 | 3.548 | 54.088 | 84.001 | 1.00 | 31.93 | B | C |
| ATOM | 3096 | CG | GLN | B | 99 | 2.838 | 53.246 | 85.050 | 1.00 | 38.20 | B | C |
| ATOM | 3097 | CD | GLN | B | 99 | 1.990 | 52.113 | 84.464 | 1.00 | 43.07 | B | C |
| ATOM | 3098 | OE1 | GLN | B | 99 | 1.349 | 51.333 | 85.220 | 1.00 | 43.76 | B | O |
| ATOM | 3099 | NE2 | GLN | B | 99 | 1.975 | 52.002 | 83.125 | 1.00 | 44.63 | B | N |
| ATOM | 3100 | C | GLN | B | 99 | 5.380 | 54.557 | 85.624 | 1.00 | 28.23 | B | C |
| ATOM | 3101 | O | GLN | B | 99 | 5.124 | 54.651 | 86.820 | 1.00 | 26.96 | B | O |
| ATOM | 3102 | N | ILE | B | 100 | 6.471 | 53.940 | 85.166 | 1.00 | 26.64 | B | N |
| ATOM | 3103 | CA | ILE | B | 100 | 7.375 | 53.313 | 86.117 | 1.00 | 26.42 | B | C |
| ATOM | 3104 | CB | ILE | B | 100 | 8.202 | 52.151 | 85.480 | 1.00 | 26.54 | B | C |
| ATOM | 3105 | CG2 | ILE | B | 100 | 7.632 | 51.759 | 84.141 | 1.00 | 26.76 | B | C |
| ATOM | 3106 | CG1 | ILE | B | 100 | 9.674 | 52.515 | 85.412 | 1.00 | 25.09 | B | C |
| ATOM | 3107 | CD1 | ILE | B | 100 | 10.502 | 51.618 | 86.280 | 1.00 | 26.99 | B | C |
| ATOM | 3108 | C | ILE | B | 100 | 8.281 | 54.299 | 86.859 | 1.00 | 25.59 | B | C |
| ATOM | 3109 | O | ILE | B | 100 | 8.558 | 54.100 | 88.017 | 1.00 | 25.70 | B | O |
| ATOM | 3110 | N | MET | B | 101 | 8.716 | 55.364 | 86.190 | 1.00 | 25.68 | B | N |
| ATOM | 3111 | CA | MET | B | 101 | 9.570 | 56.363 | 86.828 | 1.00 | 27.09 | B | C |
| ATOM | 3112 | CB | MET | B | 101 | 9.999 | 57.429 | 85.818 | 1.00 | 27.05 | B | C |
| ATOM | 3113 | CG | MET | B | 101 | 10.909 | 56.916 | 84.709 | 1.00 | 28.17 | B | C |
| ATOM | 3114 | SD | MET | B | 101 | 12.574 | 57.556 | 84.861 | 1.00 | 29.73 | B | S |
| ATOM | 3115 | CE | MET | B | 101 | 13.220 | 56.459 | 86.109 | 1.00 | 30.12 | B | C |
| ATOM | 3116 | C | MET | B | 101 | 8.802 | 57.031 | 87.971 | 1.00 | 27.58 | B | C |
| ATOM | 3117 | O | MET | B | 101 | 9.368 | 57.392 | 88.970 | 1.00 | 27.76 | B | O |
| ATOM | 3118 | N | ARG | B | 102 | 7.496 | 57.174 | 87.788 | 1.00 | 28.46 | B | N |
| ATOM | 3119 | CA | ARG | B | 102 | 6.632 | 57.793 | 88.779 | 1.00 | 30.62 | B | C |
| ATOM | 3120 | CB | ARG | B | 102 | 5.204 | 57.873 | 88.245 | 1.00 | 32.28 | B | C |
| ATOM | 3121 | CG | ARG | B | 102 | 5.026 | 58.886 | 87.125 | 1.00 | 35.24 | B | C |
| ATOM | 3122 | CD | ARG | B | 102 | 4.457 | 60.175 | 87.670 | 1.00 | 37.73 | B | C |
| ATOM | 3123 | NE | ARG | B | 102 | 3.054 | 60.331 | 87.311 | 1.00 | 39.04 | B | N |
| ATOM | 3124 | CZ | ARG | B | 102 | 2.212 | 61.155 | 87.924 | 1.00 | 39.47 | B | C |
| ATOM | 3125 | NH1 | ARG | B | 102 | 2.623 | 61.899 | 88.947 | 1.00 | 39.46 | B | N |
| ATOM | 3126 | NH2 | ARG | B | 102 | 0.962 | 61.253 | 87.493 | 1.00 | 40.13 | B | N |
| ATOM | 3127 | C | ARG | B | 102 | 6.635 | 57.059 | 90.113 | 1.00 | 30.20 | B | C |
| ATOM | 3128 | O | ARG | B | 102 | 6.551 | 57.686 | 91.144 | 1.00 | 29.54 | B | O |
| ATOM | 3129 | N | LYS | B | 103 | 6.734 | 55.735 | 90.095 | 1.00 | 29.63 | B | N |
| ATOM | 3130 | CA | LYS | B | 103 | 6.730 | 55.004 | 91.353 | 1.00 | 32.48 | B | C |
| ATOM | 3131 | CB | LYS | B | 103 | 5.818 | 53.774 | 91.271 | 1.00 | 34.00 | B | C |
| ATOM | 3132 | CG | LYS | B | 103 | 6.233 | 52.732 | 90.272 | 1.00 | 38.09 | B | C |
| ATOM | 3133 | CD | LYS | B | 103 | 5.146 | 51.667 | 90.099 | 1.00 | 40.56 | B | C |
| ATOM | 3134 | CE | LYS | B | 103 | 3.822 | 52.272 | 89.621 | 1.00 | 42.60 | B | C |
| ATOM | 3135 | NZ | LYS | B | 103 | 2.843 | 51.198 | 89.276 | 1.00 | 43.62 | B | N |
| ATOM | 3136 | C | LYS | B | 103 | 8.115 | 54.601 | 91.859 | 1.00 | 31.82 | B | C |
| ATOM | 3137 | O | LYS | B | 103 | 8.240 | 53.767 | 92.742 | 1.00 | 33.01 | B | O |
| ATOM | 3138 | N | LEU | B | 104 | 9.156 | 55.214 | 91.308 | 1.00 | 30.75 | B | N |
| ATOM | 3139 | CA | LEU | B | 104 | 10.514 | 54.898 | 91.734 | 1.00 | 28.25 | B | C |
| ATOM | 3140 | CB | LEU | B | 104 | 11.412 | 54.665 | 90.511 | 1.00 | 27.60 | B | C |
| ATOM | 3141 | CG | LEU | B | 104 | 11.609 | 53.237 | 89.993 | 1.00 | 30.03 | B | C |
| ATOM | 3142 | CD1 | LEU | B | 104 | 10.285 | 52.504 | 89.910 | 1.00 | 27.85 | B | C |
| ATOM | 3143 | CD2 | LEU | B | 104 | 12.292 | 53.293 | 88.620 | 1.00 | 30.19 | B | C |
| ATOM | 3144 | C | LEU | B | 104 | 11.116 | 55.997 | 92.598 | 1.00 | 26.48 | B | C |
| ATOM | 3145 | O | LEU | B | 104 | 11.058 | 57.142 | 92.259 | 1.00 | 24.83 | B | O |
| ATOM | 3146 | N | ASP | B | 105 | 11.688 | 55.608 | 93.731 | 1.00 | 26.35 | B | N |
| ATOM | 3147 | CA | ASP | B | 105 | 12.319 | 56.553 | 94.637 | 1.00 | 26.73 | B | C |
| ATOM | 3148 | CB | ASP | B | 105 | 11.321 | 57.051 | 95.679 | 1.00 | 29.61 | B | C |
| ATOM | 3149 | CG | ASP | B | 105 | 11.933 | 58.061 | 96.635 | 1.00 | 31.21 | B | C |
| ATOM | 3150 | OD1 | ASP | B | 105 | 12.654 | 58.962 | 96.160 | 1.00 | 33.72 | B | O |
| ATOM | 3151 | OD2 | ASP | B | 105 | 11.686 | 57.965 | 97.856 | 1.00 | 34.06 | B | O |
| ATOM | 3152 | C | ASP | B | 105 | 13.474 | 55.844 | 95.327 | 1.00 | 25.93 | B | C |
| ATOM | 3153 | O | ASP | B | 105 | 13.289 | 55.158 | 96.351 | 1.00 | 24.30 | B | O |
| ATOM | 3154 | N | HIS | B | 106 | 14.664 | 56.008 | 94.753 | 1.00 | 25.02 | B | N |
| ATOM | 3155 | CA | HIS | B | 106 | 15.871 | 55.382 | 95.277 | 1.00 | 23.21 | B | C |

| | | | | | | | | | | | | |
|---|---|---|---|---|---|---|---|---|---|---|---|---|
| ATOM | 3156 | CB | HIS | B | 106 | 16.022 | 53.977 | 94.678 | 1.00 | 21.26 | B | C |
| ATOM | 3157 | CG | HIS | B | 106 | 17.083 | 53.150 | 95.328 | 1.00 | 21.90 | B | C |
| ATOM | 3158 | CD2 | HIS | B | 106 | 17.005 | 52.198 | 96.291 | 1.00 | 21.79 | B | C |
| ATOM | 3159 | ND1 | HIS | B | 106 | 18.418 | 53.263 | 95.012 | 1.00 | 21.85 | B | N |
| ATOM | 3160 | CE1 | HIS | B | 106 | 19.118 | 52.419 | 95.752 | 1.00 | 22.43 | B | C |
| ATOM | 3161 | NE2 | HIS | B | 106 | 18.281 | 51.764 | 96.535 | 1.00 | 23.54 | B | N |
| ATOM | 3162 | C | HIS | B | 106 | 17.099 | 56.226 | 94.961 | 1.00 | 21.84 | B | C |
| ATOM | 3163 | O | HIS | B | 106 | 17.198 | 56.788 | 93.903 | 1.00 | 19.76 | B | O |
| ATOM | 3164 | N | CYS | B | 107 | 18.027 | 56.290 | 95.912 | 1.00 | 22.14 | B | N |
| ATOM | 3165 | CA | CYS | B | 107 | 19.250 | 57.072 | 95.773 | 1.00 | 22.96 | B | C |
| ATOM | 3166 | CB | CYS | B | 107 | 20.068 | 56.988 | 97.065 | 1.00 | 22.25 | B | C |
| ATOM | 3167 | SG | CYS | B | 107 | 20.662 | 55.327 | 97.485 | 1.00 | 26.72 | B | S |
| ATOM | 3168 | C | CYS | B | 107 | 20.126 | 56.646 | 94.596 | 1.00 | 22.77 | B | C |
| ATOM | 3169 | O | CYS | B | 107 | 20.988 | 57.394 | 94.173 | 1.00 | 22.42 | B | O |
| ATOM | 3170 | N | ASN | B | 108 | 19.888 | 55.447 | 94.072 | 1.00 | 21.75 | B | N |
| ATOM | 3171 | CA | ASN | B | 108 | 20.669 | 54.935 | 92.957 | 1.00 | 20.80 | B | C |
| ATOM | 3172 | CB | ASN | B | 108 | 21.220 | 53.546 | 93.317 | 1.00 | 20.59 | B | C |
| ATOM | 3173 | CG | ASN | B | 108 | 22.385 | 53.613 | 94.304 | 1.00 | 21.06 | B | C |
| ATOM | 3174 | OD1 | ASN | B | 108 | 22.389 | 52.943 | 95.314 | 1.00 | 21.00 | B | O |
| ATOM | 3175 | ND2 | ASN | B | 108 | 23.380 | 54.427 | 93.988 | 1.00 | 20.95 | B | N |
| ATOM | 3176 | C | ASN | B | 108 | 19.893 | 54.884 | 91.631 | 1.00 | 20.93 | B | C |
| ATOM | 3177 | O | ASN | B | 108 | 20.231 | 54.120 | 90.732 | 1.00 | 20.68 | B | O |
| ATOM | 3178 | N | ILE | B | 109 | 18.857 | 55.710 | 91.522 | 1.00 | 19.80 | B | N |
| ATOM | 3179 | CA | ILE | B | 109 | 18.044 | 55.793 | 90.314 | 1.00 | 19.37 | B | C |
| ATOM | 3180 | CB | ILE | B | 109 | 16.696 | 55.048 | 90.487 | 1.00 | 19.98 | B | C |
| ATOM | 3181 | CG2 | ILE | B | 109 | 15.735 | 55.417 | 89.355 | 1.00 | 19.11 | B | C |
| ATOM | 3182 | CG1 | ILE | B | 109 | 16.945 | 53.538 | 90.507 | 1.00 | 20.54 | B | C |
| ATOM | 3183 | CD1 | ILE | B | 109 | 15.730 | 52.701 | 90.855 | 1.00 | 20.51 | B | C |
| ATOM | 3184 | C | ILE | B | 109 | 17.781 | 57.266 | 89.997 | 1.00 | 20.47 | B | C |
| ATOM | 3185 | O | ILE | B | 109 | 17.333 | 58.032 | 90.857 | 1.00 | 19.75 | B | O |
| ATOM | 3186 | N | VAL | B | 110 | 18.076 | 57.668 | 88.767 | 1.00 | 19.92 | B | N |
| ATOM | 3187 | CA | VAL | B | 110 | 17.866 | 59.050 | 88.377 | 1.00 | 21.91 | B | C |
| ATOM | 3188 | CB | VAL | B | 110 | 18.193 | 59.264 | 86.874 | 1.00 | 21.51 | B | C |
| ATOM | 3189 | CG1 | VAL | B | 110 | 17.296 | 58.415 | 86.004 | 1.00 | 19.89 | B | C |
| ATOM | 3190 | CG2 | VAL | B | 110 | 18.051 | 60.729 | 86.527 | 1.00 | 25.44 | B | C |
| ATOM | 3191 | C | VAL | B | 110 | 16.431 | 59.470 | 88.701 | 1.00 | 21.92 | B | C |
| ATOM | 3192 | O | VAL | B | 110 | 15.481 | 58.840 | 88.278 | 1.00 | 19.95 | B | O |
| ATOM | 3193 | N | ARG | B | 111 | 16.307 | 60.541 | 89.481 | 1.00 | 23.46 | B | N |
| ATOM | 3194 | CA | ARG | B | 111 | 15.006 | 61.042 | 89.913 | 1.00 | 25.61 | B | C |
| ATOM | 3195 | CB | ARG | B | 111 | 15.159 | 61.924 | 91.164 | 1.00 | 26.91 | B | C |
| ATOM | 3196 | CG | ARG | B | 111 | 13.854 | 62.627 | 91.579 | 1.00 | 33.08 | B | C |
| ATOM | 3197 | CD | ARG | B | 111 | 13.879 | 63.229 | 93.002 | 1.00 | 36.93 | B | C |
| ATOM | 3198 | NE | ARG | B | 111 | 14.380 | 64.606 | 93.052 | 1.00 | 42.45 | B | N |
| ATOM | 3199 | CZ | ARG | B | 111 | 15.657 | 64.949 | 93.248 | 1.00 | 45.10 | B | C |
| ATOM | 3200 | NH1 | ARG | B | 111 | 16.589 | 64.011 | 93.413 | 1.00 | 44.65 | B | N |
| ATOM | 3201 | NH2 | ARG | B | 111 | 16.004 | 66.234 | 93.290 | 1.00 | 44.22 | B | N |
| ATOM | 3202 | C | ARG | B | 111 | 14.196 | 61.803 | 88.875 | 1.00 | 25.18 | B | C |
| ATOM | 3203 | O | ARG | B | 111 | 14.705 | 62.655 | 88.180 | 1.00 | 23.87 | B | O |
| ATOM | 3204 | N | LEU | B | 112 | 12.915 | 61.466 | 88.790 | 1.00 | 25.93 | B | N |
| ATOM | 3205 | CA | LEU | B | 112 | 12.005 | 62.144 | 87.879 | 1.00 | 26.23 | B | C |
| ATOM | 3206 | CB | LEU | B | 112 | 10.857 | 61.212 | 87.473 | 1.00 | 24.02 | B | C |
| ATOM | 3207 | CG | LEU | B | 112 | 9.840 | 61.848 | 86.512 | 1.00 | 23.47 | B | C |
| ATOM | 3208 | CD1 | LEU | B | 112 | 10.529 | 62.173 | 85.195 | 1.00 | 21.60 | B | C |
| ATOM | 3209 | CD2 | LEU | B | 112 | 8.655 | 60.924 | 86.293 | 1.00 | 20.41 | B | C |
| ATOM | 3210 | C | LEU | B | 112 | 11.449 | 63.356 | 88.637 | 1.00 | 27.06 | B | C |
| ATOM | 3211 | O | LEU | B | 112 | 10.547 | 63.222 | 89.440 | 1.00 | 27.48 | B | O |
| ATOM | 3212 | N | ARG | B | 113 | 12.013 | 64.530 | 88.385 | 1.00 | 28.15 | B | N |
| ATOM | 3213 | CA | ARG | B | 113 | 11.563 | 65.754 | 89.048 | 1.00 | 31.19 | B | C |
| ATOM | 3214 | CB | ARG | B | 113 | 12.549 | 66.899 | 88.773 | 1.00 | 32.97 | B | C |
| ATOM | 3215 | CG | ARG | B | 113 | 14.024 | 66.652 | 89.130 | 1.00 | 36.86 | B | C |
| ATOM | 3216 | CD | ARG | B | 113 | 14.333 | 66.856 | 90.617 | 1.00 | 42.50 | B | C |
| ATOM | 3217 | NE | ARG | B | 113 | 15.664 | 67.450 | 90.814 | 1.00 | 47.31 | B | N |
| ATOM | 3218 | CZ | ARG | B | 113 | 15.970 | 68.713 | 90.507 | 1.00 | 48.14 | B | C |

| | | | | | | | | | | | | |
|---|---|---|---|---|---|---|---|---|---|---|---|---|
| ATOM | 3219 | NH1 | ARG | B | 113 | 15.039 | 69.515 | 90.005 | 1.00 | 48.91 | B | N |
| ATOM | 3220 | NH2 | ARG | B | 113 | 17.208 | 69.171 | 90.668 | 1.00 | 48.41 | B | N |
| ATOM | 3221 | C | ARG | B | 113 | 10.184 | 66.162 | 88.522 | 1.00 | 30.94 | B | C |
| ATOM | 3222 | O | ARG | B | 113 | 9.255 | 66.355 | 89.277 | 1.00 | 30.37 | B | O |
| ATOM | 3223 | N | TYR | B | 114 | 10.078 | 66.298 | 87.207 | 1.00 | 31.12 | B | N |
| ATOM | 3224 | CA | TYR | B | 114 | 8.827 | 66.691 | 86.583 | 1.00 | 32.52 | B | C |
| ATOM | 3225 | CB | TYR | B | 114 | 8.806 | 68.204 | 86.332 | 1.00 | 33.71 | B | C |
| ATOM | 3226 | CG | TYR | B | 114 | 9.053 | 69.069 | 87.544 | 1.00 | 35.20 | B | C |
| ATOM | 3227 | CD1 | TYR | B | 114 | 8.098 | 69.188 | 88.553 | 1.00 | 35.80 | B | C |
| ATOM | 3228 | CE1 | TYR | B | 114 | 8.337 | 69.990 | 89.680 | 1.00 | 37.72 | B | C |
| ATOM | 3229 | CD2 | TYR | B | 114 | 10.251 | 69.766 | 87.683 | 1.00 | 35.01 | B | C |
| ATOM | 3230 | CE2 | TYR | B | 114 | 10.497 | 70.562 | 88.795 | 1.00 | 37.04 | B | C |
| ATOM | 3231 | CZ | TYR | B | 114 | 9.543 | 70.670 | 89.789 | 1.00 | 36.93 | B | C |
| ATOM | 3232 | OH | TYR | B | 114 | 9.813 | 71.457 | 90.886 | 1.00 | 39.07 | B | O |
| ATOM | 3233 | C | TYR | B | 114 | 8.679 | 66.008 | 85.233 | 1.00 | 32.54 | B | C |
| ATOM | 3234 | O | TYR | B | 114 | 9.616 | 65.390 | 84.715 | 1.00 | 33.29 | B | O |
| ATOM | 3235 | N | PHE | B | 115 | 7.480 | 66.135 | 84.676 | 1.00 | 31.18 | B | N |
| ATOM | 3236 | CA | PHE | B | 115 | 7.171 | 65.629 | 83.352 | 1.00 | 30.16 | B | C |
| ATOM | 3237 | CB | PHE | B | 115 | 6.712 | 64.159 | 83.394 | 1.00 | 28.72 | B | C |
| ATOM | 3238 | CG | PHE | B | 115 | 5.325 | 63.951 | 83.922 | 1.00 | 26.93 | B | C |
| ATOM | 3239 | CD1 | PHE | B | 115 | 4.224 | 64.013 | 83.073 | 1.00 | 27.95 | B | C |
| ATOM | 3240 | CD2 | PHE | B | 115 | 5.119 | 63.660 | 85.267 | 1.00 | 27.34 | B | C |
| ATOM | 3241 | CE1 | PHE | B | 115 | 2.938 | 63.787 | 83.555 | 1.00 | 26.30 | B | C |
| ATOM | 3242 | CE2 | PHE | B | 115 | 3.841 | 63.432 | 85.762 | 1.00 | 26.35 | B | C |
| ATOM | 3243 | CZ | PHE | B | 115 | 2.747 | 63.493 | 84.905 | 1.00 | 26.95 | B | C |
| ATOM | 3244 | C | PHE | B | 115 | 6.102 | 66.572 | 82.794 | 1.00 | 30.54 | B | C |
| ATOM | 3245 | O | PHE | B | 115 | 5.292 | 67.133 | 83.551 | 1.00 | 30.66 | B | O |
| ATOM | 3246 | N | PHE | B | 116 | 6.132 | 66.783 | 81.483 | 1.00 | 29.85 | B | N |
| ATOM | 3247 | CA | PHE | B | 116 | 5.180 | 67.663 | 80.823 | 1.00 | 29.99 | B | C |
| ATOM | 3248 | CB | PHE | B | 116 | 5.548 | 69.131 | 81.093 | 1.00 | 28.10 | B | C |
| ATOM | 3249 | CG | PHE | B | 116 | 6.870 | 69.570 | 80.485 | 1.00 | 27.56 | B | C |
| ATOM | 3250 | CD1 | PHE | B | 116 | 6.948 | 69.976 | 79.150 | 1.00 | 27.34 | B | C |
| ATOM | 3251 | CD2 | PHE | B | 116 | 8.035 | 69.584 | 81.254 | 1.00 | 26.63 | B | C |
| ATOM | 3252 | CE1 | PHE | B | 116 | 8.168 | 70.391 | 78.593 | 1.00 | 27.15 | B | C |
| ATOM | 3253 | CE2 | PHE | B | 116 | 9.258 | 69.995 | 80.709 | 1.00 | 25.61 | B | C |
| ATOM | 3254 | CZ | PHE | B | 116 | 9.326 | 70.399 | 79.379 | 1.00 | 25.95 | B | C |
| ATOM | 3255 | C | PHE | B | 116 | 5.172 | 67.395 | 79.321 | 1.00 | 31.02 | B | C |
| ATOM | 3256 | O | PHE | B | 116 | 6.101 | 66.814 | 78.791 | 1.00 | 29.79 | B | O |
| ATOM | 3257 | N | TYR | B | 117 | 4.115 | 67.833 | 78.647 | 1.00 | 31.65 | B | N |
| ATOM | 3258 | CA | TYR | B | 117 | 3.997 | 67.629 | 77.213 | 1.00 | 32.90 | B | C |
| ATOM | 3259 | CB | TYR | B | 117 | 2.601 | 67.121 | 76.881 | 1.00 | 32.86 | B | C |
| ATOM | 3260 | CG | TYR | B | 117 | 2.269 | 65.859 | 77.638 | 1.00 | 33.06 | B | C |
| ATOM | 3261 | CD1 | TYR | B | 117 | 1.663 | 65.911 | 78.895 | 1.00 | 32.13 | B | C |
| ATOM | 3262 | CE1 | TYR | B | 117 | 1.408 | 64.742 | 79.621 | 1.00 | 33.48 | B | C |
| ATOM | 3263 | CD2 | TYR | B | 117 | 2.611 | 64.616 | 77.123 | 1.00 | 32.71 | B | C |
| ATOM | 3264 | CE2 | TYR | B | 117 | 2.361 | 63.448 | 77.833 | 1.00 | 33.12 | B | C |
| ATOM | 3265 | CZ | TYR | B | 117 | 1.762 | 63.515 | 79.076 | 1.00 | 33.64 | B | C |
| ATOM | 3266 | OH | TYR | B | 117 | 1.521 | 62.344 | 79.754 | 1.00 | 35.67 | B | O |
| ATOM | 3267 | C | TYR | B | 117 | 4.287 | 68.915 | 76.446 | 1.00 | 33.98 | B | C |
| ATOM | 3268 | O | TYR | B | 117 | 4.112 | 70.006 | 76.973 | 1.00 | 35.53 | B | O |
| ATOM | 3269 | N | SER | B | 118 | 4.734 | 68.776 | 75.200 | 1.00 | 34.62 | B | N |
| ATOM | 3270 | CA | SER | B | 118 | 5.047 | 69.927 | 74.355 | 1.00 | 35.91 | B | C |
| ATOM | 3271 | CB | SER | B | 118 | 6.382 | 70.541 | 74.763 | 1.00 | 36.30 | B | C |
| ATOM | 3272 | OG | SER | B | 118 | 7.445 | 69.644 | 74.482 | 1.00 | 36.08 | B | O |
| ATOM | 3273 | C | SER | B | 118 | 5.146 | 69.529 | 72.891 | 1.00 | 37.26 | B | C |
| ATOM | 3274 | O | SER | B | 118 | 5.026 | 68.360 | 72.545 | 1.00 | 36.84 | B | O |
| ATOM | 3275 | N | SER | B | 119 | 5.375 | 70.517 | 72.034 | 1.00 | 39.40 | B | N |
| ATOM | 3276 | CA | SER | B | 119 | 5.516 | 70.258 | 70.610 | 1.00 | 41.76 | B | C |
| ATOM | 3277 | CB | SER | B | 119 | 5.294 | 71.539 | 69.808 | 1.00 | 43.05 | B | C |
| ATOM | 3278 | OG | SER | B | 119 | 3.911 | 71.797 | 69.646 | 1.00 | 46.48 | B | O |
| ATOM | 3279 | C | SER | B | 119 | 6.909 | 69.705 | 70.314 | 1.00 | 42.34 | B | C |
| ATOM | 3280 | O | SER | B | 119 | 7.806 | 69.777 | 71.155 | 1.00 | 42.61 | B | O |
| ATOM | 3281 | N | VAL | B | 126 | 2.685 | 65.135 | 69.551 | 1.00 | 35.13 | B | N |

| | | | | | | | | | | | | |
|---|---|---|---|---|---|---|---|---|---|---|---|---|
| ATOM | 3282 | CA | VAL | B | 126 | 2.963 | 65.550 | 70.922 | 1.00 | 35.05 | B | C |
| ATOM | 3283 | CB | VAL | B | 126 | 1.722 | 65.367 | 71.846 | 1.00 | 35.66 | B | C |
| ATOM | 3284 | CG1 | VAL | B | 126 | 0.512 | 66.066 | 71.241 | 1.00 | 36.14 | B | C |
| ATOM | 3285 | CG2 | VAL | B | 126 | 1.441 | 63.891 | 72.071 | 1.00 | 36.17 | B | C |
| ATOM | 3286 | C | VAL | B | 126 | 4.137 | 64.781 | 71.527 | 1.00 | 34.04 | B | C |
| ATOM | 3287 | O | VAL | B | 126 | 4.367 | 63.600 | 71.214 | 1.00 | 34.58 | B | O |
| ATOM | 3288 | N | TYR | B | 127 | 4.874 | 65.458 | 72.399 | 1.00 | 32.21 | B | N |
| ATOM | 3289 | CA | TYR | B | 127 | 6.023 | 64.857 | 73.051 | 1.00 | 30.32 | B | C |
| ATOM | 3290 | CB | TYR | B | 127 | 7.304 | 65.606 | 72.688 | 1.00 | 32.58 | B | C |
| ATOM | 3291 | CG | TYR | B | 127 | 7.670 | 65.563 | 71.229 | 1.00 | 35.04 | B | C |
| ATOM | 3292 | CD1 | TYR | B | 127 | 7.235 | 66.554 | 70.351 | 1.00 | 36.90 | B | C |
| ATOM | 3293 | CE1 | TYR | B | 127 | 7.572 | 66.517 | 68.997 | 1.00 | 39.03 | B | C |
| ATOM | 3294 | CD2 | TYR | B | 127 | 8.450 | 64.528 | 70.723 | 1.00 | 36.75 | B | C |
| ATOM | 3295 | CE2 | TYR | B | 127 | 8.794 | 64.479 | 69.370 | 1.00 | 39.00 | B | C |
| ATOM | 3296 | CZ | TYR | B | 127 | 8.355 | 65.477 | 68.517 | 1.00 | 39.19 | B | C |
| ATOM | 3297 | OH | TYR | B | 127 | 8.719 | 65.443 | 67.192 | 1.00 | 38.94 | B | O |
| ATOM | 3298 | C | TYR | B | 127 | 5.910 | 64.837 | 74.563 | 1.00 | 28.75 | B | C |
| ATOM | 3299 | O | TYR | B | 127 | 5.327 | 65.723 | 75.172 | 1.00 | 27.27 | B | O |
| ATOM | 3300 | N | LEU | B | 128 | 6.492 | 63.802 | 75.150 | 1.00 | 26.92 | B | N |
| ATOM | 3301 | CA | LEU | B | 128 | 6.529 | 63.639 | 76.584 | 1.00 | 25.28 | B | C |
| ATOM | 3302 | CB | LEU | B | 128 | 6.360 | 62.170 | 76.950 | 1.00 | 25.56 | B | C |
| ATOM | 3303 | CG | LEU | B | 128 | 6.621 | 61.824 | 78.414 | 1.00 | 24.73 | B | C |
| ATOM | 3304 | CD1 | LEU | B | 128 | 5.696 | 62.630 | 79.319 | 1.00 | 22.95 | B | C |
| ATOM | 3305 | CD2 | LEU | B | 128 | 6.433 | 60.328 | 78.611 | 1.00 | 23.83 | B | C |
| ATOM | 3306 | C | LEU | B | 128 | 7.910 | 64.120 | 76.998 | 1.00 | 25.48 | B | C |
| ATOM | 3307 | O | LEU | B | 128 | 8.909 | 63.716 | 76.416 | 1.00 | 24.97 | B | O |
| ATOM | 3308 | N | ASN | B | 129 | 7.949 | 64.996 | 77.992 | 1.00 | 24.91 | B | N |
| ATOM | 3309 | CA | ASN | B | 129 | 9.193 | 65.557 | 78.495 | 1.00 | 24.80 | B | C |
| ATOM | 3310 | CB | ASN | B | 129 | 9.138 | 67.087 | 78.458 | 1.00 | 25.13 | B | C |
| ATOM | 3311 | CG | ASN | B | 129 | 9.192 | 67.641 | 77.053 | 1.00 | 25.63 | B | C |
| ATOM | 3312 | OD1 | ASN | B | 129 | 10.237 | 68.081 | 76.596 | 1.00 | 27.02 | B | O |
| ATOM | 3313 | ND2 | ASN | B | 129 | 8.060 | 67.614 | 76.357 | 1.00 | 25.06 | B | N |
| ATOM | 3314 | C | ASN | B | 129 | 9.460 | 65.119 | 79.926 | 1.00 | 24.76 | B | C |
| ATOM | 3315 | O | ASN | B | 129 | 8.735 | 65.478 | 80.821 | 1.00 | 25.89 | B | O |
| ATOM | 3316 | N | LEU | B | 130 | 10.517 | 64.341 | 80.127 | 1.00 | 24.71 | B | N |
| ATOM | 3317 | CA | LEU | B | 130 | 10.866 | 63.895 | 81.467 | 1.00 | 24.32 | B | C |
| ATOM | 3318 | CB | LEU | B | 130 | 11.267 | 62.411 | 81.453 | 1.00 | 25.00 | B | C |
| ATOM | 3319 | CG | LEU | B | 130 | 10.165 | 61.372 | 81.209 | 1.00 | 25.81 | B | C |
| ATOM | 3320 | CD1 | LEU | B | 130 | 9.715 | 61.428 | 79.777 | 1.00 | 29.93 | B | C |
| ATOM | 3321 | CD2 | LEU | B | 130 | 10.681 | 59.981 | 81.512 | 1.00 | 27.10 | B | C |
| ATOM | 3322 | C | LEU | B | 130 | 12.014 | 64.750 | 82.011 | 1.00 | 23.56 | B | C |
| ATOM | 3323 | O | LEU | B | 130 | 13.090 | 64.793 | 81.419 | 1.00 | 23.85 | B | O |
| ATOM | 3324 | N | VAL | B | 131 | 11.771 | 65.452 | 83.116 | 1.00 | 23.09 | B | N |
| ATOM | 3325 | CA | VAL | B | 131 | 12.816 | 66.279 | 83.722 | 1.00 | 23.23 | B | C |
| ATOM | 3326 | CB | VAL | B | 131 | 12.268 | 67.580 | 84.345 | 1.00 | 23.20 | B | C |
| ATOM | 3327 | CG1 | VAL | B | 131 | 13.420 | 68.397 | 84.909 | 1.00 | 21.29 | B | C |
| ATOM | 3328 | CG2 | VAL | B | 131 | 11.528 | 68.394 | 83.295 | 1.00 | 21.92 | B | C |
| ATOM | 3329 | C | VAL | B | 131 | 13.470 | 65.453 | 84.813 | 1.00 | 23.26 | B | C |
| ATOM | 3330 | O | VAL | B | 131 | 12.906 | 65.239 | 85.880 | 1.00 | 24.05 | B | O |
| ATOM | 3331 | N | LEU | B | 132 | 14.675 | 64.993 | 84.526 | 1.00 | 23.07 | B | N |
| ATOM | 3332 | CA | LEU | B | 132 | 15.410 | 64.146 | 85.444 | 1.00 | 21.73 | B | C |
| ATOM | 3333 | CB | LEU | B | 132 | 15.844 | 62.889 | 84.697 | 1.00 | 21.89 | B | C |
| ATOM | 3334 | CG | LEU | B | 132 | 14.723 | 62.227 | 83.888 | 1.00 | 20.35 | B | C |
| ATOM | 3335 | CD1 | LEU | B | 132 | 15.295 | 61.473 | 82.704 | 1.00 | 21.52 | B | C |
| ATOM | 3336 | CD2 | LEU | B | 132 | 13.924 | 61.314 | 84.801 | 1.00 | 19.23 | B | C |
| ATOM | 3337 | C | LEU | B | 132 | 16.635 | 64.840 | 85.996 | 1.00 | 22.45 | B | C |
| ATOM | 3338 | O | LEU | B | 132 | 17.071 | 65.868 | 85.483 | 1.00 | 21.95 | B | O |
| ATOM | 3339 | N | ASP | B | 133 | 17.190 | 64.260 | 87.051 | 1.00 | 22.31 | B | N |
| ATOM | 3340 | CA | ASP | B | 133 | 18.400 | 64.801 | 87.636 | 1.00 | 24.14 | B | C |
| ATOM | 3341 | CB | ASP | B | 133 | 18.805 | 64.000 | 88.871 | 1.00 | 25.74 | B | C |
| ATOM | 3342 | CG | ASP | B | 133 | 18.061 | 64.426 | 90.120 | 1.00 | 28.64 | B | C |
| ATOM | 3343 | OD1 | ASP | B | 133 | 18.139 | 63.674 | 91.119 | 1.00 | 30.83 | B | O |
| ATOM | 3344 | OD2 | ASP | B | 133 | 17.416 | 65.507 | 90.115 | 1.00 | 28.19 | B | O |

| | | | | | | | | | | | | |
|---|---|---|---|---|---|---|---|---|---|---|---|---|
| ATOM | 3345 | C | ASP | B | 133 | 19.486 | 64.663 | 86.578 | 1.00 | 24.57 | B | C |
| ATOM | 3346 | O | ASP | B | 133 | 19.467 | 63.724 | 85.781 | 1.00 | 24.25 | B | O |
| ATOM | 3347 | N | TYR | B | 134 | 20.414 | 65.611 | 86.558 | 1.00 | 25.18 | B | N |
| ATOM | 3348 | CA | TYR | B | 134 | 21.516 | 65.558 | 85.617 | 1.00 | 25.17 | B | C |
| ATOM | 3349 | CB | TYR | B | 134 | 21.910 | 66.954 | 85.133 | 1.00 | 25.49 | B | C |
| ATOM | 3350 | CG | TYR | B | 134 | 23.196 | 66.940 | 84.329 | 1.00 | 26.49 | B | C |
| ATOM | 3351 | CD1 | TYR | B | 134 | 23.212 | 66.453 | 83.023 | 1.00 | 27.70 | B | C |
| ATOM | 3352 | CE1 | TYR | B | 134 | 24.395 | 66.358 | 82.297 | 1.00 | 27.11 | B | C |
| ATOM | 3353 | CD2 | TYR | B | 134 | 24.407 | 67.337 | 84.898 | 1.00 | 25.93 | B | C |
| ATOM | 3354 | CE2 | TYR | B | 134 | 25.601 | 67.245 | 84.178 | 1.00 | 27.67 | B | C |
| ATOM | 3355 | CZ | TYR | B | 134 | 25.583 | 66.752 | 82.881 | 1.00 | 27.14 | B | C |
| ATOM | 3356 | OH | TYR | B | 134 | 26.745 | 66.646 | 82.161 | 1.00 | 28.84 | B | O |
| ATOM | 3357 | C | TYR | B | 134 | 22.703 | 64.940 | 86.337 | 1.00 | 25.04 | B | C |
| ATOM | 3358 | O | TYR | B | 134 | 22.980 | 65.270 | 87.479 | 1.00 | 24.34 | B | O |
| ATOM | 3359 | N | VAL | B | 135 | 23.387 | 64.029 | 85.663 | 1.00 | 24.40 | B | N |
| ATOM | 3360 | CA | VAL | B | 135 | 24.559 | 63.390 | 86.234 | 1.00 | 24.60 | B | C |
| ATOM | 3361 | CB | VAL | B | 135 | 24.339 | 61.871 | 86.375 | 1.00 | 24.14 | B | C |
| ATOM | 3362 | CG1 | VAL | B | 135 | 25.470 | 61.241 | 87.175 | 1.00 | 22.36 | B | C |
| ATOM | 3363 | CG2 | VAL | B | 135 | 23.011 | 61.623 | 87.069 | 1.00 | 22.74 | B | C |
| ATOM | 3364 | C | VAL | B | 135 | 25.689 | 63.719 | 85.254 | 1.00 | 25.16 | B | C |
| ATOM | 3365 | O | VAL | B | 135 | 25.536 | 63.573 | 84.048 | 1.00 | 25.65 | B | O |
| ATOM | 3366 | N | PRO | B | 136 | 26.831 | 64.188 | 85.769 | 1.00 | 25.69 | B | N |
| ATOM | 3367 | CD | PRO | B | 136 | 27.146 | 64.266 | 87.208 | 1.00 | 26.83 | B | C |
| ATOM | 3368 | CA | PRO | B | 136 | 27.990 | 64.563 | 84.952 | 1.00 | 26.29 | B | C |
| ATOM | 3369 | CB | PRO | B | 136 | 28.961 | 65.136 | 85.980 | 1.00 | 26.41 | B | C |
| ATOM | 3370 | CG | PRO | B | 136 | 28.660 | 64.319 | 87.209 | 1.00 | 28.38 | B | C |
| ATOM | 3371 | C | PRO | B | 136 | 28.650 | 63.515 | 84.058 | 1.00 | 27.47 | B | C |
| ATOM | 3372 | O | PRO | B | 136 | 29.102 | 63.833 | 82.973 | 1.00 | 27.21 | B | O |
| ATOM | 3373 | N | GLU | B | 137 | 28.710 | 62.264 | 84.502 | 1.00 | 28.50 | B | N |
| ATOM | 3374 | CA | GLU | B | 137 | 29.365 | 61.255 | 83.691 | 1.00 | 28.59 | B | C |
| ATOM | 3375 | CB | GLU | B | 137 | 30.797 | 61.048 | 84.200 | 1.00 | 30.35 | B | C |
| ATOM | 3376 | CG | GLU | B | 137 | 31.829 | 60.724 | 83.118 | 1.00 | 34.51 | B | C |
| ATOM | 3377 | CD | GLU | B | 137 | 32.493 | 61.955 | 82.525 | 1.00 | 36.01 | B | C |
| ATOM | 3378 | OE1 | GLU | B | 137 | 32.559 | 63.005 | 83.202 | 1.00 | 38.26 | B | O |
| ATOM | 3379 | OE2 | GLU | B | 137 | 32.978 | 61.865 | 81.383 | 1.00 | 39.63 | B | O |
| ATOM | 3380 | C | GLU | B | 137 | 28.613 | 59.927 | 83.684 | 1.00 | 28.74 | B | C |
| ATOM | 3381 | O | GLU | B | 137 | 27.544 | 59.804 | 84.263 | 1.00 | 29.50 | B | O |
| ATOM | 3382 | N | THR | B | 138 | 29.184 | 58.940 | 83.004 | 1.00 | 26.67 | B | N |
| ATOM | 3383 | CA | THR | B | 138 | 28.589 | 57.615 | 82.911 | 1.00 | 26.13 | B | C |
| ATOM | 3384 | CB | THR | B | 138 | 27.842 | 57.426 | 81.580 | 1.00 | 25.95 | B | C |
| ATOM | 3385 | OG1 | THR | B | 138 | 28.781 | 57.434 | 80.501 | 1.00 | 27.78 | B | O |
| ATOM | 3386 | CG2 | THR | B | 138 | 26.838 | 58.538 | 81.362 | 1.00 | 26.46 | B | C |
| ATOM | 3387 | C | THR | B | 138 | 29.697 | 56.570 | 82.977 | 1.00 | 25.87 | B | C |
| ATOM | 3388 | O | THR | B | 138 | 30.855 | 56.884 | 82.749 | 1.00 | 25.23 | B | O |
| ATOM | 3389 | N | VAL | B | 139 | 29.343 | 55.326 | 83.275 | 1.00 | 25.65 | B | N |
| ATOM | 3390 | CA | VAL | B | 139 | 30.350 | 54.274 | 83.350 | 1.00 | 25.96 | B | C |
| ATOM | 3391 | CB | VAL | B | 139 | 29.756 | 52.958 | 83.911 | 1.00 | 27.02 | B | C |
| ATOM | 3392 | CG1 | VAL | B | 139 | 30.753 | 51.819 | 83.759 | 1.00 | 22.51 | B | C |
| ATOM | 3393 | CG2 | VAL | B | 139 | 29.414 | 53.136 | 85.379 | 1.00 | 24.86 | B | C |
| ATOM | 3394 | C | VAL | B | 139 | 30.954 | 54.030 | 81.971 | 1.00 | 26.04 | B | C |
| ATOM | 3395 | O | VAL | B | 139 | 32.128 | 53.740 | 81.854 | 1.00 | 24.86 | B | O |
| ATOM | 3396 | N | TYR | B | 140 | 30.138 | 54.159 | 80.930 | 1.00 | 26.51 | B | N |
| ATOM | 3397 | CA | TYR | B | 140 | 30.633 | 53.981 | 79.572 | 1.00 | 27.78 | B | C |
| ATOM | 3398 | CB | TYR | B | 140 | 29.547 | 54.290 | 78.543 | 1.00 | 27.01 | B | C |
| ATOM | 3399 | CG | TYR | B | 140 | 30.040 | 54.125 | 77.125 | 1.00 | 26.24 | B | C |
| ATOM | 3400 | CD1 | TYR | B | 140 | 30.312 | 52.862 | 76.611 | 1.00 | 26.21 | B | C |
| ATOM | 3401 | CE1 | TYR | B | 140 | 30.816 | 52.701 | 75.329 | 1.00 | 26.92 | B | C |
| ATOM | 3402 | CD2 | TYR | B | 140 | 30.282 | 55.237 | 76.311 | 1.00 | 26.89 | B | C |
| ATOM | 3403 | CE2 | TYR | B | 140 | 30.787 | 55.091 | 75.022 | 1.00 | 26.37 | B | C |
| ATOM | 3404 | CZ | TYR | B | 140 | 31.052 | 53.817 | 74.538 | 1.00 | 28.74 | B | C |
| ATOM | 3405 | OH | TYR | B | 140 | 31.564 | 53.649 | 73.271 | 1.00 | 30.22 | B | O |
| ATOM | 3406 | C | TYR | B | 140 | 31.838 | 54.894 | 79.291 | 1.00 | 28.60 | B | C |
| ATOM | 3407 | O | TYR | B | 140 | 32.877 | 54.422 | 78.847 | 1.00 | 28.78 | B | O |

| | | | | | | | | | | | | |
|---|---|---|---|---|---|---|---|---|---|---|---|---|
| ATOM | 3408 | N | ARG | B | 141 | 31.688 | 56.194 | 79.545 | 1.00 | 27.50 | B | N |
| ATOM | 3409 | CA | ARG | B | 141 | 32.776 | 57.147 | 79.299 | 1.00 | 27.92 | B | C |
| ATOM | 3410 | CB | ARG | B | 141 | 32.294 | 58.584 | 79.472 | 1.00 | 27.57 | B | C |
| ATOM | 3411 | CG | ARG | B | 141 | 31.100 | 58.954 | 78.638 | 1.00 | 30.10 | B | C |
| ATOM | 3412 | CD | ARG | B | 141 | 30.641 | 60.338 | 79.022 | 1.00 | 32.27 | B | C |
| ATOM | 3413 | NE | ARG | B | 141 | 31.049 | 61.316 | 78.025 | 1.00 | 37.27 | B | N |
| ATOM | 3414 | CZ | ARG | B | 141 | 31.449 | 62.548 | 78.306 | 1.00 | 38.16 | B | C |
| ATOM | 3415 | NH1 | ARG | B | 141 | 31.509 | 62.959 | 79.567 | 1.00 | 38.91 | B | N |
| ATOM | 3416 | NH2 | ARG | B | 141 | 31.767 | 63.373 | 77.318 | 1.00 | 41.92 | B | N |
| ATOM | 3417 | C | ARG | B | 141 | 33.962 | 56.928 | 80.221 | 1.00 | 27.02 | B | C |
| ATOM | 3418 | O | ARG | B | 141 | 35.094 | 56.961 | 79.788 | 1.00 | 28.42 | B | O |
| ATOM | 3419 | N | VAL | B | 142 | 33.690 | 56.735 | 81.502 | 1.00 | 27.37 | B | N |
| ATOM | 3420 | CA | VAL | B | 142 | 34.743 | 56.501 | 82.475 | 1.00 | 28.11 | B | C |
| ATOM | 3421 | CB | VAL | B | 142 | 34.149 | 56.278 | 83.864 | 1.00 | 28.21 | B | C |
| ATOM | 3422 | CG1 | VAL | B | 142 | 35.212 | 55.760 | 84.815 | 1.00 | 26.55 | B | C |
| ATOM | 3423 | CG2 | VAL | B | 142 | 33.561 | 57.595 | 84.377 | 1.00 | 28.68 | B | C |
| ATOM | 3424 | C | VAL | B | 142 | 35.574 | 55.285 | 82.074 | 1.00 | 30.53 | B | C |
| ATOM | 3425 | O | VAL | B | 142 | 36.806 | 55.358 | 82.017 | 1.00 | 29.04 | B | O |
| ATOM | 3426 | N | ALA | B | 143 | 34.895 | 54.176 | 81.785 | 1.00 | 30.77 | B | N |
| ATOM | 3427 | CA | ALA | B | 143 | 35.577 | 52.957 | 81.375 | 1.00 | 31.81 | B | C |
| ATOM | 3428 | CB | ALA | B | 143 | 34.562 | 51.861 | 81.046 | 1.00 | 31.46 | B | C |
| ATOM | 3429 | C | ALA | B | 143 | 36.448 | 53.238 | 80.155 | 1.00 | 33.02 | B | C |
| ATOM | 3430 | O | ALA | B | 143 | 37.569 | 52.763 | 80.076 | 1.00 | 33.27 | B | O |
| ATOM | 3431 | N | ARG | B | 144 | 35.922 | 54.019 | 79.214 | 1.00 | 33.55 | B | N |
| ATOM | 3432 | CA | ARG | B | 144 | 36.654 | 54.346 | 77.993 | 1.00 | 35.56 | B | C |
| ATOM | 3433 | CB | ARG | B | 144 | 35.789 | 55.197 | 77.063 | 1.00 | 37.03 | B | C |
| ATOM | 3434 | CG | ARG | B | 144 | 35.795 | 54.724 | 75.620 | 1.00 | 40.50 | B | C |
| ATOM | 3435 | CD | ARG | B | 144 | 35.120 | 55.723 | 74.672 | 1.00 | 44.64 | B | C |
| ATOM | 3436 | NE | ARG | B | 144 | 34.691 | 55.054 | 73.447 | 1.00 | 49.27 | B | N |
| ATOM | 3437 | CZ | ARG | B | 144 | 34.302 | 55.671 | 72.334 | 1.00 | 51.57 | B | C |
| ATOM | 3438 | NH1 | ARG | B | 144 | 34.285 | 57.002 | 72.268 | 1.00 | 52.08 | B | N |
| ATOM | 3439 | NH2 | ARG | B | 144 | 33.924 | 54.949 | 71.282 | 1.00 | 52.28 | B | N |
| ATOM | 3440 | C | ARG | B | 144 | 37.953 | 55.084 | 78.290 | 1.00 | 35.40 | B | C |
| ATOM | 3441 | O | ARG | B | 144 | 39.000 | 54.685 | 77.844 | 1.00 | 35.35 | B | O |
| ATOM | 3442 | N | HIS | B | 145 | 37.854 | 56.174 | 79.042 | 1.00 | 36.15 | B | N |
| ATOM | 3443 | CA | HIS | B | 145 | 39.015 | 56.974 | 79.426 | 1.00 | 36.67 | B | C |
| ATOM | 3444 | CB | HIS | B | 145 | 38.600 | 58.017 | 80.469 | 1.00 | 39.26 | B | C |
| ATOM | 3445 | CG | HIS | B | 145 | 37.609 | 59.023 | 79.965 | 1.00 | 44.12 | B | C |
| ATOM | 3446 | CD2 | HIS | B | 145 | 37.316 | 59.436 | 78.704 | 1.00 | 45.34 | B | C |
| ATOM | 3447 | ND1 | HIS | B | 145 | 36.804 | 59.765 | 80.807 | 1.00 | 46.26 | B | N |
| ATOM | 3448 | CE1 | HIS | B | 145 | 36.058 | 60.589 | 80.089 | 1.00 | 47.02 | B | C |
| ATOM | 3449 | NE2 | HIS | B | 145 | 36.349 | 60.412 | 78.813 | 1.00 | 46.44 | B | N |
| ATOM | 3450 | C | HIS | B | 145 | 40.127 | 56.091 | 79.999 | 1.00 | 35.59 | B | C |
| ATOM | 3451 | O | HIS | B | 145 | 41.298 | 56.300 | 79.715 | 1.00 | 35.93 | B | O |
| ATOM | 3452 | N | TYR | B | 146 | 39.760 | 55.107 | 80.812 | 1.00 | 34.78 | B | N |
| ATOM | 3453 | CA | TYR | B | 146 | 40.762 | 54.229 | 81.395 | 1.00 | 34.96 | B | C |
| ATOM | 3454 | CB | TYR | B | 146 | 40.177 | 53.423 | 82.554 | 1.00 | 33.21 | B | C |
| ATOM | 3455 | CG | TYR | B | 146 | 40.140 | 54.168 | 83.865 | 1.00 | 32.58 | B | C |
| ATOM | 3456 | CD1 | TYR | B | 146 | 39.123 | 55.086 | 84.146 | 1.00 | 32.15 | B | C |
| ATOM | 3457 | CE1 | TYR | B | 146 | 39.100 | 55.794 | 85.351 | 1.00 | 31.29 | B | C |
| ATOM | 3458 | CD2 | TYR | B | 146 | 41.139 | 53.969 | 84.825 | 1.00 | 32.81 | B | C |
| ATOM | 3459 | CE2 | TYR | B | 146 | 41.128 | 54.670 | 86.033 | 1.00 | 32.39 | B | C |
| ATOM | 3460 | CZ | TYR | B | 146 | 40.105 | 55.578 | 86.291 | 1.00 | 32.02 | B | C |
| ATOM | 3461 | OH | TYR | B | 146 | 40.091 | 56.258 | 87.491 | 1.00 | 32.34 | B | O |
| ATOM | 3462 | C | TYR | B | 146 | 41.313 | 53.280 | 80.343 | 1.00 | 36.61 | B | C |
| ATOM | 3463 | O | TYR | B | 146 | 42.516 | 53.056 | 80.268 | 1.00 | 36.46 | B | O |
| ATOM | 3464 | N | SER | B | 147 | 40.419 | 52.732 | 79.528 | 1.00 | 37.20 | B | N |
| ATOM | 3465 | CA | SER | B | 147 | 40.808 | 51.803 | 78.481 | 1.00 | 38.55 | B | C |
| ATOM | 3466 | CB | SER | B | 147 | 39.566 | 51.332 | 77.729 | 1.00 | 37.57 | B | C |
| ATOM | 3467 | OG | SER | B | 147 | 39.830 | 50.129 | 77.038 | 1.00 | 40.67 | B | O |
| ATOM | 3468 | C | SER | B | 147 | 41.781 | 52.494 | 77.529 | 1.00 | 40.32 | B | C |
| ATOM | 3469 | O | SER | B | 147 | 42.874 | 52.000 | 77.292 | 1.00 | 39.67 | B | O |
| ATOM | 3470 | N | ARG | B | 148 | 41.373 | 53.643 | 76.992 | 1.00 | 42.12 | B | N |

| | | | | | | | | | | | | |
|---|---|---|---|---|---|---|---|---|---|---|---|---|
| ATOM | 3471 | CA | ARG | B | 148 | 42.218 | 54.426 | 76.085 | 1.00 | 44.20 | B | C |
| ATOM | 3472 | CB | ARG | B | 148 | 41.579 | 55.800 | 75.801 | 1.00 | 46.25 | B | C |
| ATOM | 3473 | CG | ARG | B | 148 | 40.730 | 55.905 | 74.530 | 1.00 | 49.56 | B | C |
| ATOM | 3474 | CD | ARG | B | 148 | 41.568 | 55.598 | 73.294 | 1.00 | 53.58 | B | C |
| ATOM | 3475 | NE | ARG | B | 148 | 40.930 | 55.970 | 72.027 | 1.00 | 56.02 | B | N |
| ATOM | 3476 | CZ | ARG | B | 148 | 40.941 | 57.197 | 71.504 | 1.00 | 57.54 | B | C |
| ATOM | 3477 | NH1 | ARG | B | 148 | 41.556 | 58.196 | 72.137 | 1.00 | 57.74 | B | N |
| ATOM | 3478 | NH2 | ARG | B | 148 | 40.363 | 57.422 | 70.328 | 1.00 | 58.16 | B | N |
| ATOM | 3479 | C | ARG | B | 148 | 43.601 | 54.651 | 76.711 | 1.00 | 44.29 | B | C |
| ATOM | 3480 | O | ARG | B | 148 | 44.626 | 54.427 | 76.072 | 1.00 | 44.48 | B | O |
| ATOM | 3481 | N | ALA | B | 149 | 43.611 | 55.095 | 77.966 | 1.00 | 44.16 | B | N |
| ATOM | 3482 | CA | ALA | B | 149 | 44.850 | 55.375 | 78.689 | 1.00 | 44.28 | B | C |
| ATOM | 3483 | CB | ALA | B | 149 | 44.551 | 56.186 | 79.943 | 1.00 | 43.04 | B | C |
| ATOM | 3484 | C | ALA | B | 149 | 45.610 | 54.109 | 79.067 | 1.00 | 45.22 | B | C |
| ATOM | 3485 | O | ALA | B | 149 | 46.476 | 54.125 | 79.967 | 1.00 | 45.19 | B | O |
| ATOM | 3486 | N | ALA | B | 150 | 45.290 | 53.008 | 78.397 | 1.00 | 45.12 | B | N |
| ATOM | 3487 | CA | ALA | B | 150 | 45.966 | 51.750 | 78.670 | 1.00 | 45.16 | B | C |
| ATOM | 3488 | CB | ALA | B | 150 | 47.389 | 51.799 | 78.120 | 1.00 | 44.88 | B | C |
| ATOM | 3489 | C | ALA | B | 150 | 45.990 | 51.461 | 80.165 | 1.00 | 45.56 | B | C |
| ATOM | 3490 | O | ALA | B | 150 | 46.973 | 50.931 | 80.692 | 1.00 | 46.93 | B | O |
| ATOM | 3491 | N | GLN | B | 151 | 44.908 | 51.810 | 80.853 | 1.00 | 45.39 | B | N |
| ATOM | 3492 | CA | GLN | B | 151 | 44.827 | 51.560 | 82.283 | 1.00 | 44.36 | B | C |
| ATOM | 3493 | CB | GLN | B | 151 | 45.089 | 52.837 | 83.062 | 1.00 | 46.16 | B | C |
| ATOM | 3494 | CG | GLN | B | 151 | 46.468 | 53.388 | 82.864 | 1.00 | 48.61 | B | C |
| ATOM | 3495 | CD | GLN | B | 151 | 46.894 | 54.163 | 84.065 | 1.00 | 49.02 | B | C |
| ATOM | 3496 | OE1 | GLN | B | 151 | 46.108 | 54.884 | 84.637 | 1.00 | 50.40 | B | O |
| ATOM | 3497 | NE2 | GLN | B | 151 | 48.149 | 54.011 | 84.458 | 1.00 | 50.14 | B | N |
| ATOM | 3498 | C | GLN | B | 151 | 43.504 | 50.976 | 82.762 | 1.00 | 43.12 | B | C |
| ATOM | 3499 | O | GLN | B | 151 | 42.510 | 50.949 | 82.034 | 1.00 | 42.20 | B | O |
| ATOM | 3500 | N | THR | B | 152 | 43.500 | 50.539 | 84.016 | 1.00 | 40.80 | B | N |
| ATOM | 3501 | CA | THR | B | 152 | 42.314 | 49.942 | 84.610 | 1.00 | 40.17 | B | C |
| ATOM | 3502 | CB | THR | B | 152 | 42.629 | 48.540 | 85.168 | 1.00 | 40.98 | B | C |
| ATOM | 3503 | OG1 | THR | B | 152 | 41.732 | 48.252 | 86.251 | 1.00 | 43.52 | B | O |
| ATOM | 3504 | CG2 | THR | B | 152 | 44.064 | 48.477 | 85.676 | 1.00 | 41.18 | B | C |
| ATOM | 3505 | C | THR | B | 152 | 41.736 | 50.769 | 85.759 | 1.00 | 37.44 | B | C |
| ATOM | 3506 | O | THR | B | 152 | 42.471 | 51.417 | 86.524 | 1.00 | 36.99 | B | O |
| ATOM | 3507 | N | LEU | B | 153 | 40.414 | 50.725 | 85.880 | 1.00 | 33.99 | B | N |
| ATOM | 3508 | CA | LEU | B | 153 | 39.716 | 51.457 | 86.919 | 1.00 | 31.50 | B | C |
| ATOM | 3509 | CB | LEU | B | 153 | 38.208 | 51.406 | 86.665 | 1.00 | 30.57 | B | C |
| ATOM | 3510 | CG | LEU | B | 153 | 37.289 | 52.046 | 87.705 | 1.00 | 31.09 | B | C |
| ATOM | 3511 | CD1 | LEU | B | 153 | 37.528 | 53.557 | 87.759 | 1.00 | 29.89 | B | C |
| ATOM | 3512 | CD2 | LEU | B | 153 | 35.839 | 51.753 | 87.333 | 1.00 | 31.46 | B | C |
| ATOM | 3513 | C | LEU | B | 153 | 40.036 | 50.843 | 88.284 | 1.00 | 30.81 | B | C |
| ATOM | 3514 | O | LEU | B | 153 | 39.875 | 49.644 | 88.481 | 1.00 | 30.56 | B | O |
| ATOM | 3515 | N | PRO | B | 154 | 40.521 | 51.658 | 89.236 | 1.00 | 28.71 | B | N |
| ATOM | 3516 | CD | PRO | B | 154 | 40.896 | 53.079 | 89.145 | 1.00 | 28.24 | B | C |
| ATOM | 3517 | CA | PRO | B | 154 | 40.834 | 51.111 | 90.564 | 1.00 | 28.81 | B | C |
| ATOM | 3518 | CB | PRO | B | 154 | 41.116 | 52.366 | 91.390 | 1.00 | 28.88 | B | C |
| ATOM | 3519 | CG | PRO | B | 154 | 41.777 | 53.267 | 90.367 | 1.00 | 29.18 | B | C |
| ATOM | 3520 | C | PRO | B | 154 | 39.655 | 50.286 | 91.101 | 1.00 | 28.18 | B | C |
| ATOM | 3521 | O | PRO | B | 154 | 38.518 | 50.689 | 90.999 | 1.00 | 27.62 | B | O |
| ATOM | 3522 | N | VAL | B | 155 | 39.950 | 49.120 | 91.658 | 1.00 | 28.64 | B | N |
| ATOM | 3523 | CA | VAL | B | 155 | 38.908 | 48.236 | 92.166 | 1.00 | 29.32 | B | C |
| ATOM | 3524 | CB | VAL | B | 155 | 39.519 | 46.941 | 92.753 | 1.00 | 30.82 | B | C |
| ATOM | 3525 | CG1 | VAL | B | 155 | 40.441 | 46.292 | 91.727 | 1.00 | 33.38 | B | C |
| ATOM | 3526 | CG2 | VAL | B | 155 | 40.273 | 47.246 | 94.029 | 1.00 | 30.45 | B | C |
| ATOM | 3527 | C | VAL | B | 155 | 37.993 | 48.857 | 93.223 | 1.00 | 28.81 | B | C |
| ATOM | 3528 | O | VAL | B | 155 | 36.892 | 48.395 | 93.423 | 1.00 | 28.57 | B | O |
| ATOM | 3529 | N | ILE | B | 156 | 38.457 | 49.896 | 93.909 | 1.00 | 28.44 | B | N |
| ATOM | 3530 | CA | ILE | B | 156 | 37.617 | 50.504 | 94.921 | 1.00 | 26.70 | B | C |
| ATOM | 3531 | CB | ILE | B | 156 | 38.391 | 51.582 | 95.729 | 1.00 | 27.48 | B | C |
| ATOM | 3532 | CG2 | ILE | B | 156 | 39.022 | 52.599 | 94.794 | 1.00 | 27.95 | B | C |
| ATOM | 3533 | CG1 | ILE | B | 156 | 37.447 | 52.272 | 96.718 | 1.00 | 27.56 | B | C |

| | | | | | | | | | | | | |
|---|---|---|---|---|---|---|---|---|---|---|---|---|
| ATOM | 3534 | CD1 | ILE | B | 156 | 36.828 | 51.324 | 97.719 | 1.00 | 27.27 | B | C |
| ATOM | 3535 | C | ILE | B | 156 | 36.390 | 51.096 | 94.235 | 1.00 | 25.15 | B | C |
| ATOM | 3536 | O | ILE | B | 156 | 35.297 | 51.000 | 94.738 | 1.00 | 23.86 | B | O |
| ATOM | 3537 | N | TYR | B | 157 | 36.596 | 51.683 | 93.060 | 1.00 | 25.03 | B | N |
| ATOM | 3538 | CA | TYR | B | 157 | 35.501 | 52.273 | 92.294 | 1.00 | 24.90 | B | C |
| ATOM | 3539 | CB | TYR | B | 157 | 36.043 | 53.176 | 91.183 | 1.00 | 27.49 | B | C |
| ATOM | 3540 | CG | TYR | B | 157 | 36.581 | 54.491 | 91.692 | 1.00 | 30.64 | B | C |
| ATOM | 3541 | CD1 | TYR | B | 157 | 35.742 | 55.409 | 92.327 | 1.00 | 31.25 | B | C |
| ATOM | 3542 | CE1 | TYR | B | 157 | 36.228 | 56.635 | 92.772 | 1.00 | 33.95 | B | C |
| ATOM | 3543 | CD2 | TYR | B | 157 | 37.922 | 54.830 | 91.517 | 1.00 | 32.08 | B | C |
| ATOM | 3544 | CE2 | TYR | B | 157 | 38.420 | 56.052 | 91.958 | 1.00 | 33.66 | B | C |
| ATOM | 3545 | CZ | TYR | B | 157 | 37.572 | 56.950 | 92.575 | 1.00 | 35.06 | B | C |
| ATOM | 3546 | OH | TYR | B | 157 | 38.064 | 58.172 | 92.967 | 1.00 | 37.01 | B | O |
| ATOM | 3547 | C | TYR | B | 157 | 34.635 | 51.179 | 91.693 | 1.00 | 24.46 | B | C |
| ATOM | 3548 | O | TYR | B | 157 | 33.415 | 51.305 | 91.632 | 1.00 | 23.17 | B | O |
| ATOM | 3549 | N | VAL | B | 158 | 35.277 | 50.106 | 91.245 | 1.00 | 22.43 | B | N |
| ATOM | 3550 | CA | VAL | B | 158 | 34.545 | 48.990 | 90.671 | 1.00 | 21.94 | B | C |
| ATOM | 3551 | CB | VAL | B | 158 | 35.492 | 47.869 | 90.189 | 1.00 | 22.46 | B | C |
| ATOM | 3552 | CG1 | VAL | B | 158 | 34.678 | 46.681 | 89.702 | 1.00 | 20.20 | B | C |
| ATOM | 3553 | CG2 | VAL | B | 158 | 36.386 | 48.391 | 89.061 | 1.00 | 20.75 | B | C |
| ATOM | 3554 | C | VAL | B | 158 | 33.608 | 48.433 | 91.742 | 1.00 | 21.97 | B | C |
| ATOM | 3555 | O | VAL | B | 158 | 32.504 | 47.992 | 91.438 | 1.00 | 21.12 | B | O |
| ATOM | 3556 | N | LYS | B | 159 | 34.062 | 48.463 | 92.995 | 1.00 | 21.21 | B | N |
| ATOM | 3557 | CA | LYS | B | 159 | 33.254 | 47.983 | 94.107 | 1.00 | 21.53 | B | C |
| ATOM | 3558 | CB | LYS | B | 159 | 34.091 | 47.880 | 95.385 | 1.00 | 22.50 | B | C |
| ATOM | 3559 | CG | LYS | B | 159 | 34.997 | 46.666 | 95.458 | 1.00 | 21.22 | B | C |
| ATOM | 3560 | CD | LYS | B | 159 | 35.815 | 46.694 | 96.722 | 1.00 | 19.70 | B | C |
| ATOM | 3561 | CE | LYS | B | 159 | 36.753 | 45.499 | 96.805 | 1.00 | 20.38 | B | C |
| ATOM | 3562 | NZ | LYS | B | 159 | 37.641 | 45.611 | 97.999 | 1.00 | 22.34 | B | N |
| ATOM | 3563 | C | LYS | B | 159 | 32.080 | 48.931 | 94.350 | 1.00 | 21.81 | B | C |
| ATOM | 3564 | O | LYS | B | 159 | 30.929 | 48.506 | 94.434 | 1.00 | 20.81 | B | O |
| ATOM | 3565 | N | LEU | B | 160 | 32.384 | 50.219 | 94.460 | 1.00 | 21.78 | B | N |
| ATOM | 3566 | CA | LEU | B | 160 | 31.357 | 51.223 | 94.690 | 1.00 | 22.22 | B | C |
| ATOM | 3567 | CB | LEU | B | 160 | 31.999 | 52.610 | 94.797 | 1.00 | 22.88 | B | C |
| ATOM | 3568 | CG | LEU | B | 160 | 32.756 | 52.951 | 96.089 | 1.00 | 24.90 | B | C |
| ATOM | 3569 | CD1 | LEU | B | 160 | 33.691 | 54.120 | 95.831 | 1.00 | 21.47 | B | C |
| ATOM | 3570 | CD2 | LEU | B | 160 | 31.764 | 53.272 | 97.216 | 1.00 | 23.45 | B | C |
| ATOM | 3571 | C | LEU | B | 160 | 30.293 | 51.240 | 93.596 | 1.00 | 22.54 | B | C |
| ATOM | 3572 | O | LEU | B | 160 | 29.118 | 51.250 | 93.881 | 1.00 | 21.35 | B | O |
| ATOM | 3573 | N | TYR | B | 161 | 30.726 | 51.239 | 92.339 | 1.00 | 22.98 | B | N |
| ATOM | 3574 | CA | TYR | B | 161 | 29.794 | 51.287 | 91.220 | 1.00 | 23.30 | B | C |
| ATOM | 3575 | CB | TYR | B | 161 | 30.559 | 51.511 | 89.914 | 1.00 | 24.73 | B | C |
| ATOM | 3576 | CG | TYR | B | 161 | 31.315 | 52.827 | 89.869 | 1.00 | 26.16 | B | C |
| ATOM | 3577 | CD1 | TYR | B | 161 | 30.983 | 53.875 | 90.728 | 1.00 | 25.50 | B | C |
| ATOM | 3578 | CE1 | TYR | B | 161 | 31.638 | 55.108 | 90.649 | 1.00 | 28.02 | B | C |
| ATOM | 3579 | CD2 | TYR | B | 161 | 32.329 | 53.040 | 88.929 | 1.00 | 26.06 | B | C |
| ATOM | 3580 | CE2 | TYR | B | 161 | 32.991 | 54.268 | 88.838 | 1.00 | 27.43 | B | C |
| ATOM | 3581 | CZ | TYR | B | 161 | 32.638 | 55.299 | 89.702 | 1.00 | 28.35 | B | C |
| ATOM | 3582 | OH | TYR | B | 161 | 33.259 | 56.525 | 89.606 | 1.00 | 30.25 | B | O |
| ATOM | 3583 | C | TYR | B | 161 | 28.898 | 50.051 | 91.102 | 1.00 | 21.99 | B | C |
| ATOM | 3584 | O | TYR | B | 161 | 27.695 | 50.176 | 90.944 | 1.00 | 19.35 | B | O |
| ATOM | 3585 | N | MET | B | 162 | 29.497 | 48.867 | 91.190 | 1.00 | 20.57 | B | N |
| ATOM | 3586 | CA | MET | B | 162 | 28.742 | 47.627 | 91.086 | 1.00 | 19.93 | B | C |
| ATOM | 3587 | CB | MET | B | 162 | 29.686 | 46.422 | 91.013 | 1.00 | 20.07 | B | C |
| ATOM | 3588 | CG | MET | B | 162 | 30.505 | 46.315 | 89.725 | 1.00 | 22.07 | B | C |
| ATOM | 3589 | SD | MET | B | 162 | 29.528 | 46.389 | 88.196 | 1.00 | 23.25 | B | S |
| ATOM | 3590 | CE | MET | B | 162 | 28.440 | 44.964 | 88.404 | 1.00 | 19.06 | B | C |
| ATOM | 3591 | C | MET | B | 162 | 27.780 | 47.444 | 92.252 | 1.00 | 19.82 | B | C |
| ATOM | 3592 | O | MET | B | 162 | 26.672 | 46.971 | 92.074 | 1.00 | 17.95 | B | O |
| ATOM | 3593 | N | TYR | B | 163 | 28.221 | 47.814 | 93.449 | 1.00 | 19.40 | B | N |
| ATOM | 3594 | CA | TYR | B | 163 | 27.377 | 47.692 | 94.628 | 1.00 | 19.22 | B | C |
| ATOM | 3595 | CB | TYR | B | 163 | 28.117 | 48.154 | 95.881 | 1.00 | 19.98 | B | C |
| ATOM | 3596 | CG | TYR | B | 163 | 27.269 | 48.138 | 97.138 | 1.00 | 20.59 | B | C |

| | | | | | | | | | | | | |
|---|---|---|---|---|---|---|---|---|---|---|---|---|
| ATOM | 3597 | CD1 | TYR | B | 163 | 27.129 | 46.978 | 97.898 | 1.00 | 19.76 | B | C |
| ATOM | 3598 | CE1 | TYR | B | 163 | 26.361 | 46.966 | 99.066 | 1.00 | 19.05 | B | C |
| ATOM | 3599 | CD2 | TYR | B | 163 | 26.616 | 49.291 | 97.574 | 1.00 | 21.01 | B | C |
| ATOM | 3600 | CE2 | TYR | B | 163 | 25.848 | 49.292 | 98.735 | 1.00 | 19.15 | B | C |
| ATOM | 3601 | CZ | TYR | B | 163 | 25.727 | 48.130 | 99.476 | 1.00 | 19.28 | B | C |
| ATOM | 3602 | OH | TYR | B | 163 | 24.976 | 48.136 | 100.624 | 1.00 | 18.32 | B | O |
| ATOM | 3603 | C | TYR | B | 163 | 26.112 | 48.521 | 94.471 | 1.00 | 18.27 | B | C |
| ATOM | 3604 | O | TYR | B | 163 | 25.030 | 48.044 | 94.750 | 1.00 | 19.93 | B | O |
| ATOM | 3605 | N | GLN | B | 164 | 26.267 | 49.763 | 94.023 | 1.00 | 16.69 | B | N |
| ATOM | 3606 | CA | GLN | B | 164 | 25.129 | 50.648 | 93.845 | 1.00 | 16.70 | B | C |
| ATOM | 3607 | CB | GLN | B | 164 | 25.615 | 52.077 | 93.593 | 1.00 | 17.20 | B | C |
| ATOM | 3608 | CG | GLN | B | 164 | 26.448 | 52.638 | 94.749 | 1.00 | 19.19 | B | C |
| ATOM | 3609 | CD | GLN | B | 164 | 27.077 | 53.983 | 94.433 | 1.00 | 21.90 | B | C |
| ATOM | 3610 | OE1 | GLN | B | 164 | 26.422 | 55.014 | 94.495 | 1.00 | 20.75 | B | O |
| ATOM | 3611 | NE2 | GLN | B | 164 | 28.359 | 53.967 | 94.074 | 1.00 | 21.44 | B | N |
| ATOM | 3612 | C | GLN | B | 164 | 24.223 | 50.161 | 92.721 | 1.00 | 17.39 | B | C |
| ATOM | 3613 | O | GLN | B | 164 | 23.036 | 50.350 | 92.774 | 1.00 | 16.87 | B | O |
| ATOM | 3614 | N | LEU | B | 165 | 24.809 | 49.517 | 91.715 | 1.00 | 17.33 | B | N |
| ATOM | 3615 | CA | LEU | B | 165 | 24.050 | 48.992 | 90.586 | 1.00 | 17.65 | B | C |
| ATOM | 3616 | CB | LEU | B | 165 | 24.989 | 48.365 | 89.547 | 1.00 | 16.79 | B | C |
| ATOM | 3617 | CG | LEU | B | 165 | 24.553 | 48.286 | 88.073 | 1.00 | 19.32 | B | C |
| ATOM | 3618 | CD1 | LEU | B | 165 | 25.367 | 47.205 | 87.376 | 1.00 | 14.83 | B | C |
| ATOM | 3619 | CD2 | LEU | B | 165 | 23.063 | 48.012 | 87.942 | 1.00 | 15.93 | B | C |
| ATOM | 3620 | C | LEU | B | 165 | 23.125 | 47.899 | 91.123 | 1.00 | 17.98 | B | C |
| ATOM | 3621 | O | LEU | B | 165 | 21.957 | 47.877 | 90.835 | 1.00 | 17.86 | B | O |
| ATOM | 3622 | N | PHE | B | 166 | 23.692 | 46.986 | 91.905 | 1.00 | 18.65 | B | N |
| ATOM | 3623 | CA | PHE | B | 166 | 22.913 | 45.896 | 92.467 | 1.00 | 18.86 | B | C |
| ATOM | 3624 | CB | PHE | B | 166 | 23.832 | 44.893 | 93.176 | 1.00 | 17.46 | B | C |
| ATOM | 3625 | CG | PHE | B | 166 | 24.585 | 43.983 | 92.231 | 1.00 | 17.81 | B | C |
| ATOM | 3626 | CD1 | PHE | B | 166 | 23.897 | 43.170 | 91.329 | 1.00 | 18.30 | B | C |
| ATOM | 3627 | CD2 | PHE | B | 166 | 25.978 | 43.933 | 92.243 | 1.00 | 17.89 | B | C |
| ATOM | 3628 | CE1 | PHE | B | 166 | 24.583 | 42.324 | 90.455 | 1.00 | 15.97 | B | C |
| ATOM | 3629 | CE2 | PHE | B | 166 | 26.672 | 43.089 | 91.371 | 1.00 | 17.33 | B | C |
| ATOM | 3630 | CZ | PHE | B | 166 | 25.971 | 42.284 | 90.476 | 1.00 | 16.86 | B | C |
| ATOM | 3631 | C | PHE | B | 166 | 21.824 | 46.423 | 93.401 | 1.00 | 20.31 | B | C |
| ATOM | 3632 | O | PHE | B | 166 | 20.751 | 45.863 | 93.468 | 1.00 | 20.76 | B | O |
| ATOM | 3633 | N | ARG | B | 167 | 22.113 | 47.513 | 94.106 | 1.00 | 20.34 | B | N |
| ATOM | 3634 | CA | ARG | B | 167 | 21.128 | 48.127 | 94.989 | 1.00 | 19.69 | B | C |
| ATOM | 3635 | CB | ARG | B | 167 | 21.743 | 49.331 | 95.705 | 1.00 | 20.99 | B | C |
| ATOM | 3636 | CG | ARG | B | 167 | 22.235 | 49.036 | 97.107 | 1.00 | 23.78 | B | C |
| ATOM | 3637 | CD | ARG | B | 167 | 21.324 | 49.690 | 98.130 | 1.00 | 25.21 | B | C |
| ATOM | 3638 | NE | ARG | B | 167 | 21.905 | 50.918 | 98.646 | 1.00 | 26.75 | B | N |
| ATOM | 3639 | CZ | ARG | B | 167 | 21.279 | 51.782 | 99.436 | 1.00 | 27.08 | B | C |
| ATOM | 3640 | NH1 | ARG | B | 167 | 20.024 | 51.564 | 99.808 | 1.00 | 25.34 | B | N |
| ATOM | 3641 | NH2 | ARG | B | 167 | 21.925 | 52.857 | 99.871 | 1.00 | 27.28 | B | N |
| ATOM | 3642 | C | ARG | B | 167 | 19.920 | 48.588 | 94.172 | 1.00 | 19.18 | B | C |
| ATOM | 3643 | O | ARG | B | 167 | 18.792 | 48.312 | 94.538 | 1.00 | 18.10 | B | O |
| ATOM | 3644 | N | SER | B | 168 | 20.167 | 49.290 | 93.066 | 1.00 | 18.41 | B | N |
| ATOM | 3645 | CA | SER | B | 168 | 19.066 | 49.771 | 92.235 | 1.00 | 19.60 | B | C |
| ATOM | 3646 | CB | SER | B | 168 | 19.583 | 50.657 | 91.087 | 1.00 | 19.13 | B | C |
| ATOM | 3647 | OG | SER | B | 168 | 20.296 | 49.921 | 90.112 | 1.00 | 15.20 | B | O |
| ATOM | 3648 | C | SER | B | 168 | 18.264 | 48.586 | 91.680 | 1.00 | 20.66 | B | C |
| ATOM | 3649 | O | SER | B | 168 | 17.055 | 48.624 | 91.649 | 1.00 | 22.59 | B | O |
| ATOM | 3650 | N | LEU | B | 169 | 18.956 | 47.534 | 91.256 | 1.00 | 19.58 | B | N |
| ATOM | 3651 | CA | LEU | B | 169 | 18.287 | 46.355 | 90.732 | 1.00 | 19.52 | B | C |
| ATOM | 3652 | CB | LEU | B | 169 | 19.321 | 45.360 | 90.206 | 1.00 | 19.00 | B | C |
| ATOM | 3653 | CG | LEU | B | 169 | 19.616 | 45.276 | 88.699 | 1.00 | 20.42 | B | C |
| ATOM | 3654 | CD1 | LEU | B | 169 | 19.438 | 46.611 | 88.000 | 1.00 | 19.90 | B | C |
| ATOM | 3655 | CD2 | LEU | B | 169 | 21.023 | 44.753 | 88.517 | 1.00 | 18.86 | B | C |
| ATOM | 3656 | C | LEU | B | 169 | 17.412 | 45.697 | 91.803 | 1.00 | 20.38 | B | C |
| ATOM | 3657 | O | LEU | B | 169 | 16.293 | 45.304 | 91.526 | 1.00 | 19.37 | B | O |
| ATOM | 3658 | N | ALA | B | 170 | 17.932 | 45.576 | 93.022 | 1.00 | 20.42 | B | N |
| ATOM | 3659 | CA | ALA | B | 170 | 17.162 | 44.989 | 94.117 | 1.00 | 21.59 | B | C |

| | | | | | | | | | | | | |
|---|---|---|---|---|---|---|---|---|---|---|---|---|
| ATOM | 3660 | CB | ALA | B | 170 | 17.997 | 44.950 | 95.404 | 1.00 | 21.41 | B | C |
| ATOM | 3661 | C | ALA | B | 170 | 15.897 | 45.819 | 94.332 | 1.00 | 20.96 | B | C |
| ATOM | 3662 | O | ALA | B | 170 | 14.830 | 45.278 | 94.549 | 1.00 | 21.26 | B | O |
| ATOM | 3663 | N | TYR | B | 171 | 16.029 | 47.140 | 94.256 | 1.00 | 21.12 | B | N |
| ATOM | 3664 | CA | TYR | B | 171 | 14.882 | 48.020 | 94.433 | 1.00 | 21.70 | B | C |
| ATOM | 3665 | CB | TYR | B | 171 | 15.313 | 49.490 | 94.384 | 1.00 | 23.35 | B | C |
| ATOM | 3666 | CG | TYR | B | 171 | 14.166 | 50.475 | 94.521 | 1.00 | 23.33 | B | C |
| ATOM | 3667 | CD1 | TYR | B | 171 | 13.476 | 50.613 | 95.725 | 1.00 | 24.00 | B | C |
| ATOM | 3668 | CE1 | TYR | B | 171 | 12.399 | 51.498 | 95.846 | 1.00 | 23.82 | B | C |
| ATOM | 3669 | CD2 | TYR | B | 171 | 13.748 | 51.245 | 93.434 | 1.00 | 25.41 | B | C |
| ATOM | 3670 | CE2 | TYR | B | 171 | 12.669 | 52.131 | 93.542 | 1.00 | 24.08 | B | C |
| ATOM | 3671 | CZ | TYR | B | 171 | 12.000 | 52.247 | 94.751 | 1.00 | 23.87 | B | C |
| ATOM | 3672 | OH | TYR | B | 171 | 10.926 | 53.095 | 94.863 | 1.00 | 25.05 | B | O |
| ATOM | 3673 | C | TYR | B | 171 | 13.818 | 47.756 | 93.360 | 1.00 | 22.68 | B | C |
| ATOM | 3674 | O | TYR | B | 171 | 12.710 | 47.357 | 93.680 | 1.00 | 22.57 | B | O |
| ATOM | 3675 | N | ILE | B | 172 | 14.155 | 47.973 | 92.089 | 1.00 | 20.43 | B | N |
| ATOM | 3676 | CA | ILE | B | 172 | 13.173 | 47.751 | 91.032 | 1.00 | 20.83 | B | C |
| ATOM | 3677 | CB | ILE | B | 172 | 13.720 | 48.115 | 89.618 | 1.00 | 21.45 | B | C |
| ATOM | 3678 | CG2 | ILE | B | 172 | 13.962 | 49.622 | 89.531 | 1.00 | 18.27 | B | C |
| ATOM | 3679 | CG1 | ILE | B | 172 | 14.988 | 47.321 | 89.294 | 1.00 | 20.89 | B | C |
| ATOM | 3680 | CD1 | ILE | B | 172 | 15.377 | 47.391 | 87.819 | 1.00 | 20.73 | B | C |
| ATOM | 3681 | C | ILE | B | 172 | 12.620 | 46.323 | 91.012 | 1.00 | 20.40 | B | C |
| ATOM | 3682 | O | ILE | B | 172 | 11.427 | 46.137 | 90.849 | 1.00 | 20.85 | B | O |
| ATOM | 3683 | N | HIS | B | 173 | 13.482 | 45.324 | 91.204 | 1.00 | 20.17 | B | N |
| ATOM | 3684 | CA | HIS | B | 173 | 13.039 | 43.929 | 91.208 | 1.00 | 21.35 | B | C |
| ATOM | 3685 | CB | HIS | B | 173 | 14.235 | 42.961 | 91.349 | 1.00 | 21.95 | B | C |
| ATOM | 3686 | CG | HIS | B | 173 | 15.100 | 42.874 | 90.124 | 1.00 | 24.88 | B | C |
| ATOM | 3687 | CD2 | HIS | B | 173 | 15.101 | 43.600 | 88.978 | 1.00 | 23.62 | B | C |
| ATOM | 3688 | ND1 | HIS | B | 173 | 16.128 | 41.964 | 89.999 | 1.00 | 25.44 | B | N |
| ATOM | 3689 | CE1 | HIS | B | 173 | 16.726 | 42.132 | 88.830 | 1.00 | 24.51 | B | C |
| ATOM | 3690 | NE2 | HIS | B | 173 | 16.119 | 43.120 | 88.194 | 1.00 | 23.62 | B | N |
| ATOM | 3691 | C | HIS | B | 173 | 12.017 | 43.647 | 92.317 | 1.00 | 21.56 | B | C |
| ATOM | 3692 | O | HIS | B | 173 | 11.117 | 42.851 | 92.129 | 1.00 | 20.59 | B | O |
| ATOM | 3693 | N | SER | B | 174 | 12.157 | 44.307 | 93.463 | 1.00 | 20.64 | B | N |
| ATOM | 3694 | CA | SER | B | 174 | 11.218 | 44.100 | 94.557 | 1.00 | 22.46 | B | C |
| ATOM | 3695 | CB | SER | B | 174 | 11.594 | 44.938 | 95.780 | 1.00 | 20.63 | B | C |
| ATOM | 3696 | OG | SER | B | 174 | 11.159 | 46.273 | 95.625 | 1.00 | 20.84 | B | O |
| ATOM | 3697 | C | SER | B | 174 | 9.809 | 44.475 | 94.092 | 1.00 | 24.09 | B | C |
| ATOM | 3698 | O | SER | B | 174 | 8.842 | 44.021 | 94.651 | 1.00 | 25.84 | B | O |
| ATOM | 3699 | N | PHE | B | 175 | 9.714 | 45.313 | 93.064 | 1.00 | 24.80 | B | N |
| ATOM | 3700 | CA | PHE | B | 175 | 8.418 | 45.709 | 92.527 | 1.00 | 25.57 | B | C |
| ATOM | 3701 | CB | PHE | B | 175 | 8.419 | 47.172 | 92.087 | 1.00 | 26.92 | B | C |
| ATOM | 3702 | CG | PHE | B | 175 | 8.446 | 48.143 | 93.222 | 1.00 | 30.46 | B | C |
| ATOM | 3703 | CD1 | PHE | B | 175 | 9.625 | 48.786 | 93.574 | 1.00 | 30.26 | B | C |
| ATOM | 3704 | CD2 | PHE | B | 175 | 7.285 | 48.412 | 93.945 | 1.00 | 32.51 | B | C |
| ATOM | 3705 | CE1 | PHE | B | 175 | 9.656 | 49.689 | 94.630 | 1.00 | 32.46 | B | C |
| ATOM | 3706 | CE2 | PHE | B | 175 | 7.301 | 49.316 | 95.009 | 1.00 | 36.01 | B | C |
| ATOM | 3707 | CZ | PHE | B | 175 | 8.495 | 49.958 | 95.352 | 1.00 | 34.69 | B | C |
| ATOM | 3708 | C | PHE | B | 175 | 8.080 | 44.844 | 91.323 | 1.00 | 25.57 | B | C |
| ATOM | 3709 | O | PHE | B | 175 | 7.104 | 45.090 | 90.644 | 1.00 | 25.44 | B | O |
| ATOM | 3710 | N | GLY | B | 176 | 8.909 | 43.835 | 91.073 | 1.00 | 24.32 | B | N |
| ATOM | 3711 | CA | GLY | B | 176 | 8.696 | 42.958 | 89.935 | 1.00 | 24.39 | B | C |
| ATOM | 3712 | C | GLY | B | 176 | 9.144 | 43.561 | 88.609 | 1.00 | 24.36 | B | C |
| ATOM | 3713 | O | GLY | B | 176 | 9.004 | 42.939 | 87.552 | 1.00 | 24.07 | B | O |
| ATOM | 3714 | N | ILE | B | 177 | 9.698 | 44.770 | 88.669 | 1.00 | 23.66 | B | N |
| ATOM | 3715 | CA | ILE | B | 177 | 10.151 | 45.478 | 87.476 | 1.00 | 22.96 | B | C |
| ATOM | 3716 | CB | ILE | B | 177 | 10.048 | 47.010 | 87.698 | 1.00 | 23.38 | B | C |
| ATOM | 3717 | CG2 | ILE | B | 177 | 10.585 | 47.766 | 86.487 | 1.00 | 23.52 | B | C |
| ATOM | 3718 | CG1 | ILE | B | 177 | 8.584 | 47.380 | 87.972 | 1.00 | 22.36 | B | C |
| ATOM | 3719 | CD1 | ILE | B | 177 | 8.333 | 48.855 | 88.182 | 1.00 | 20.71 | B | C |
| ATOM | 3720 | C | ILE | B | 177 | 11.566 | 45.106 | 87.040 | 1.00 | 22.24 | B | C |
| ATOM | 3721 | O | ILE | B | 177 | 12.491 | 45.130 | 87.830 | 1.00 | 20.00 | B | O |
| ATOM | 3722 | N | CYS | B | 178 | 11.703 | 44.739 | 85.770 | 1.00 | 21.94 | B | N |

| | | | | | | | | | | | | |
|---|---|---|---|---|---|---|---|---|---|---|---|---|
| ATOM | 3723 | CA | CYS | B | 178 | 12.997 | 44.371 | 85.197 | 1.00 | 20.74 | B | C |
| ATOM | 3724 | CB | CYS | B | 178 | 12.902 | 43.044 | 84.445 | 1.00 | 22.76 | B | C |
| ATOM | 3725 | SG | CYS | B | 178 | 14.485 | 42.451 | 83.803 | 1.00 | 23.40 | B | S |
| ATOM | 3726 | C | CYS | B | 178 | 13.407 | 45.469 | 84.228 | 1.00 | 20.07 | B | C |
| ATOM | 3727 | O | CYS | B | 178 | 12.616 | 45.896 | 83.374 | 1.00 | 18.12 | B | O |
| ATOM | 3728 | N | HIS | B | 179 | 14.648 | 45.919 | 84.367 | 1.00 | 19.45 | B | N |
| ATOM | 3729 | CA | HIS | B | 179 | 15.179 | 46.981 | 83.528 | 1.00 | 18.29 | B | C |
| ATOM | 3730 | CB | HIS | B | 179 | 16.532 | 47.424 | 84.075 | 1.00 | 17.40 | B | C |
| ATOM | 3731 | CG | HIS | B | 179 | 17.073 | 48.648 | 83.411 | 1.00 | 14.71 | B | C |
| ATOM | 3732 | CD2 | HIS | B | 179 | 17.115 | 49.935 | 83.822 | 1.00 | 14.81 | B | C |
| ATOM | 3733 | ND1 | HIS | B | 179 | 17.622 | 48.624 | 82.149 | 1.00 | 13.45 | B | N |
| ATOM | 3734 | CE1 | HIS | B | 179 | 17.981 | 49.849 | 81.808 | 1.00 | 15.68 | B | C |
| ATOM | 3735 | NE2 | HIS | B | 179 | 17.683 | 50.663 | 82.806 | 1.00 | 15.19 | B | N |
| ATOM | 3736 | C | HIS | B | 179 | 15.285 | 46.537 | 82.071 | 1.00 | 19.97 | B | C |
| ATOM | 3737 | O | HIS | B | 179 | 14.804 | 47.224 | 81.180 | 1.00 | 19.85 | B | O |
| ATOM | 3738 | N | ARG | B | 180 | 15.928 | 45.389 | 81.858 | 1.00 | 19.86 | B | N |
| ATOM | 3739 | CA | ARG | B | 180 | 16.093 | 44.781 | 80.537 | 1.00 | 21.26 | B | C |
| ATOM | 3740 | CB | ARG | B | 180 | 14.742 | 44.707 | 79.826 | 1.00 | 20.78 | B | C |
| ATOM | 3741 | CG | ARG | B | 180 | 13.683 | 43.959 | 80.595 | 1.00 | 21.99 | B | C |
| ATOM | 3742 | CD | ARG | B | 180 | 12.329 | 44.378 | 80.080 | 1.00 | 23.94 | B | C |
| ATOM | 3743 | NE | ARG | B | 180 | 11.764 | 43.429 | 79.135 | 1.00 | 24.23 | B | N |
| ATOM | 3744 | CZ | ARG | B | 180 | 10.877 | 43.746 | 78.199 | 1.00 | 23.78 | B | C |
| ATOM | 3745 | NH1 | ARG | B | 180 | 10.457 | 45.001 | 78.066 | 1.00 | 22.69 | B | N |
| ATOM | 3746 | NH2 | ARG | B | 180 | 10.383 | 42.797 | 77.421 | 1.00 | 21.12 | B | N |
| ATOM | 3747 | C | ARG | B | 180 | 17.111 | 45.405 | 79.594 | 1.00 | 20.45 | B | C |
| ATOM | 3748 | O | ARG | B | 180 | 17.281 | 44.923 | 78.488 | 1.00 | 22.35 | B | O |
| ATOM | 3749 | N | ASP | B | 181 | 17.772 | 46.477 | 80.017 | 1.00 | 20.41 | B | N |
| ATOM | 3750 | CA | ASP | B | 181 | 18.792 | 47.099 | 79.174 | 1.00 | 20.56 | B | C |
| ATOM | 3751 | CB | ASP | B | 181 | 18.173 | 48.204 | 78.307 | 1.00 | 19.42 | B | C |
| ATOM | 3752 | CG | ASP | B | 181 | 19.020 | 48.526 | 77.083 | 1.00 | 20.25 | B | C |
| ATOM | 3753 | OD1 | ASP | B | 181 | 19.867 | 47.691 | 76.701 | 1.00 | 19.97 | B | O |
| ATOM | 3754 | OD2 | ASP | B | 181 | 18.834 | 49.607 | 76.496 | 1.00 | 21.94 | B | O |
| ATOM | 3755 | C | ASP | B | 181 | 19.957 | 47.656 | 80.003 | 1.00 | 19.76 | B | C |
| ATOM | 3756 | O | ASP | B | 181 | 20.375 | 48.782 | 79.823 | 1.00 | 19.12 | B | O |
| ATOM | 3757 | N | ILE | B | 182 | 20.459 | 46.836 | 80.925 | 1.00 | 19.93 | B | N |
| ATOM | 3758 | CA | ILE | B | 182 | 21.582 | 47.211 | 81.770 | 1.00 | 18.29 | B | C |
| ATOM | 3759 | CB | ILE | B | 182 | 21.753 | 46.239 | 82.968 | 1.00 | 18.18 | B | C |
| ATOM | 3760 | CG2 | ILE | B | 182 | 23.058 | 46.531 | 83.692 | 1.00 | 18.34 | B | C |
| ATOM | 3761 | CG1 | ILE | B | 182 | 20.548 | 46.332 | 83.913 | 1.00 | 19.15 | B | C |
| ATOM | 3762 | CD1 | ILE | B | 182 | 20.366 | 47.670 | 84.600 | 1.00 | 19.51 | B | C |
| ATOM | 3763 | C | ILE | B | 182 | 22.843 | 47.147 | 80.915 | 1.00 | 18.91 | B | C |
| ATOM | 3764 | O | ILE | B | 182 | 23.162 | 46.099 | 80.335 | 1.00 | 18.27 | B | O |
| ATOM | 3765 | N | LYS | B | 183 | 23.536 | 48.278 | 80.841 | 1.00 | 18.82 | B | N |
| ATOM | 3766 | CA | LYS | B | 183 | 24.770 | 48.422 | 80.078 | 1.00 | 19.33 | B | C |
| ATOM | 3767 | CB | LYS | B | 183 | 24.470 | 48.489 | 78.575 | 1.00 | 18.66 | B | C |
| ATOM | 3768 | CG | LYS | B | 183 | 23.419 | 49.509 | 78.171 | 1.00 | 19.97 | B | C |
| ATOM | 3769 | CD | LYS | B | 183 | 23.393 | 49.694 | 76.660 | 1.00 | 19.81 | B | C |
| ATOM | 3770 | CE | LYS | B | 183 | 22.228 | 50.562 | 76.210 | 1.00 | 21.53 | B | C |
| ATOM | 3771 | NZ | LYS | B | 183 | 22.305 | 50.883 | 74.755 | 1.00 | 21.65 | B | N |
| ATOM | 3772 | C | LYS | B | 183 | 25.483 | 49.701 | 80.533 | 1.00 | 20.15 | B | C |
| ATOM | 3773 | O | LYS | B | 183 | 24.866 | 50.588 | 81.072 | 1.00 | 19.09 | B | O |
| ATOM | 3774 | N | PRO | B | 184 | 26.807 | 49.790 | 80.317 | 1.00 | 21.23 | B | N |
| ATOM | 3775 | CD | PRO | B | 184 | 27.663 | 48.797 | 79.641 | 1.00 | 18.82 | B | C |
| ATOM | 3776 | CA | PRO | B | 184 | 27.586 | 50.971 | 80.718 | 1.00 | 21.66 | B | C |
| ATOM | 3777 | CB | PRO | B | 184 | 28.915 | 50.769 | 79.990 | 1.00 | 20.95 | B | C |
| ATOM | 3778 | CG | PRO | B | 184 | 29.058 | 49.267 | 79.986 | 1.00 | 20.35 | B | C |
| ATOM | 3779 | C | PRO | B | 184 | 26.942 | 52.327 | 80.414 | 1.00 | 21.24 | B | C |
| ATOM | 3780 | O | PRO | B | 184 | 26.950 | 53.207 | 81.260 | 1.00 | 20.40 | B | O |
| ATOM | 3781 | N | GLN | B | 185 | 26.385 | 52.475 | 79.209 | 1.00 | 22.24 | B | N |
| ATOM | 3782 | CA | GLN | B | 185 | 25.729 | 53.716 | 78.777 | 1.00 | 22.91 | B | C |
| ATOM | 3783 | CB | GLN | B | 185 | 25.269 | 53.608 | 77.327 | 1.00 | 24.70 | B | C |
| ATOM | 3784 | CG | GLN | B | 185 | 26.389 | 53.429 | 76.325 | 1.00 | 31.01 | B | C |
| ATOM | 3785 | CD | GLN | B | 185 | 25.982 | 53.839 | 74.927 | 1.00 | 35.53 | B | C |

| | | | | | | | | | | | | |
|---|---|---|---|---|---|---|---|---|---|---|---|---|
| ATOM | 3786 | OE1 | GLN | B | 185 | 26.778 | 53.759 | 73.996 | 1.00 | 37.89 | B | O |
| ATOM | 3787 | NE2 | GLN | B | 185 | 24.724 | 54.273 | 74.767 | 1.00 | 37.49 | B | N |
| ATOM | 3788 | C | GLN | B | 185 | 24.525 | 54.136 | 79.615 | 1.00 | 22.24 | B | C |
| ATOM | 3789 | O | GLN | B | 185 | 24.163 | 55.298 | 79.634 | 1.00 | 21.83 | B | O |
| ATOM | 3790 | N | ASN | B | 186 | 23.902 | 53.185 | 80.296 | 1.00 | 21.04 | B | N |
| ATOM | 3791 | CA | ASN | B | 186 | 22.739 | 53.502 | 81.109 | 1.00 | 20.91 | B | C |
| ATOM | 3792 | CB | ASN | B | 186 | 21.643 | 52.464 | 80.872 | 1.00 | 21.11 | B | C |
| ATOM | 3793 | CG | ASN | B | 186 | 21.041 | 52.574 | 79.487 | 1.00 | 22.98 | B | C |
| ATOM | 3794 | OD1 | ASN | B | 186 | 21.047 | 53.641 | 78.893 | 1.00 | 21.56 | B | O |
| ATOM | 3795 | ND2 | ASN | B | 186 | 20.501 | 51.470 | 78.977 | 1.00 | 24.20 | B | N |
| ATOM | 3796 | C | ASN | B | 186 | 23.078 | 53.586 | 82.590 | 1.00 | 20.70 | B | C |
| ATOM | 3797 | O | ASN | B | 186 | 22.204 | 53.526 | 83.444 | 1.00 | 20.56 | B | O |
| ATOM | 3798 | N | LEU | B | 187 | 24.367 | 53.718 | 82.874 | 1.00 | 20.37 | B | N |
| ATOM | 3799 | CA | LEU | B | 187 | 24.862 | 53.822 | 84.235 | 1.00 | 21.68 | B | C |
| ATOM | 3800 | CB | LEU | B | 187 | 25.857 | 52.686 | 84.507 | 1.00 | 21.08 | B | C |
| ATOM | 3801 | CG | LEU | B | 187 | 25.345 | 51.384 | 85.147 | 1.00 | 23.12 | B | C |
| ATOM | 3802 | CD1 | LEU | B | 187 | 23.953 | 51.053 | 84.676 | 1.00 | 23.01 | B | C |
| ATOM | 3803 | CD2 | LEU | B | 187 | 26.311 | 50.254 | 84.837 | 1.00 | 21.69 | B | C |
| ATOM | 3804 | C | LEU | B | 187 | 25.531 | 55.184 | 84.428 | 1.00 | 22.94 | B | C |
| ATOM | 3805 | O | LEU | B | 187 | 26.654 | 55.400 | 83.998 | 1.00 | 23.78 | B | O |
| ATOM | 3806 | N | LEU | B | 188 | 24.815 | 56.100 | 85.070 | 1.00 | 22.73 | B | N |
| ATOM | 3807 | CA | LEU | B | 188 | 25.317 | 57.451 | 85.313 | 1.00 | 21.92 | B | C |
| ATOM | 3808 | CB | LEU | B | 188 | 24.144 | 58.422 | 85.478 | 1.00 | 21.07 | B | C |
| ATOM | 3809 | CG | LEU | B | 188 | 23.019 | 58.386 | 84.436 | 1.00 | 22.25 | B | C |
| ATOM | 3810 | CD1 | LEU | B | 188 | 21.832 | 59.189 | 84.940 | 1.00 | 22.66 | B | C |
| ATOM | 3811 | CD2 | LEU | B | 188 | 23.499 | 58.921 | 83.113 | 1.00 | 23.75 | B | C |
| ATOM | 3812 | C | LEU | B | 188 | 26.157 | 57.470 | 86.587 | 1.00 | 22.02 | B | C |
| ATOM | 3813 | O | LEU | B | 188 | 25.943 | 56.667 | 87.498 | 1.00 | 17.40 | B | O |
| ATOM | 3814 | N | LEU | B | 189 | 27.119 | 58.384 | 86.650 | 1.00 | 23.47 | B | N |
| ATOM | 3815 | CA | LEU | B | 189 | 27.944 | 58.486 | 87.841 | 1.00 | 28.29 | B | C |
| ATOM | 3816 | CB | LEU | B | 189 | 29.070 | 57.441 | 87.822 | 1.00 | 30.86 | B | C |
| ATOM | 3817 | CG | LEU | B | 189 | 30.085 | 57.420 | 86.680 | 1.00 | 34.10 | B | C |
| ATOM | 3818 | CD1 | LEU | B | 189 | 31.060 | 58.594 | 86.841 | 1.00 | 35.95 | B | C |
| ATOM | 3819 | CD2 | LEU | B | 189 | 30.845 | 56.096 | 86.690 | 1.00 | 34.00 | B | C |
| ATOM | 3820 | C | LEU | B | 189 | 28.518 | 59.864 | 88.061 | 1.00 | 29.27 | B | C |
| ATOM | 3821 | O | LEU | B | 189 | 28.860 | 60.566 | 87.128 | 1.00 | 30.36 | B | O |
| ATOM | 3822 | N | ASP | B | 190 | 28.583 | 60.252 | 89.324 | 1.00 | 31.26 | B | N |
| ATOM | 3823 | CA | ASP | B | 190 | 29.127 | 61.543 | 89.691 | 1.00 | 33.94 | B | C |
| ATOM | 3824 | CB | ASP | B | 190 | 28.314 | 62.152 | 90.828 | 1.00 | 35.10 | B | C |
| ATOM | 3825 | CG | ASP | B | 190 | 28.759 | 63.552 | 91.160 | 1.00 | 36.45 | B | C |
| ATOM | 3826 | OD1 | ASP | B | 190 | 29.942 | 63.723 | 91.529 | 1.00 | 38.19 | B | O |
| ATOM | 3827 | OD2 | ASP | B | 190 | 27.933 | 64.483 | 91.043 | 1.00 | 38.48 | B | O |
| ATOM | 3828 | C | ASP | B | 190 | 30.560 | 61.279 | 90.146 | 1.00 | 34.46 | B | C |
| ATOM | 3829 | O | ASP | B | 190 | 30.780 | 60.700 | 91.183 | 1.00 | 34.62 | B | O |
| ATOM | 3830 | N | PRO | B | 191 | 31.546 | 61.711 | 89.353 | 1.00 | 36.30 | B | N |
| ATOM | 3831 | CD | PRO | B | 191 | 31.395 | 62.662 | 88.239 | 1.00 | 36.97 | B | C |
| ATOM | 3832 | CA | PRO | B | 191 | 32.963 | 61.511 | 89.671 | 1.00 | 37.27 | B | C |
| ATOM | 3833 | CB | PRO | B | 191 | 33.678 | 62.316 | 88.585 | 1.00 | 37.64 | B | C |
| ATOM | 3834 | CG | PRO | B | 191 | 32.714 | 63.424 | 88.295 | 1.00 | 37.84 | B | C |
| ATOM | 3835 | C | PRO | B | 191 | 33.371 | 61.938 | 91.080 | 1.00 | 37.42 | B | C |
| ATOM | 3836 | O | PRO | B | 191 | 34.105 | 61.228 | 91.768 | 1.00 | 38.47 | B | O |
| ATOM | 3837 | N | ASP | B | 192 | 32.874 | 63.085 | 91.516 | 1.00 | 36.60 | B | N |
| ATOM | 3838 | CA | ASP | B | 192 | 33.235 | 63.602 | 92.824 | 1.00 | 37.14 | B | C |
| ATOM | 3839 | CB | ASP | B | 192 | 32.936 | 65.102 | 92.885 | 1.00 | 38.13 | B | C |
| ATOM | 3840 | CG | ASP | B | 192 | 33.708 | 65.881 | 91.837 | 1.00 | 40.13 | B | C |
| ATOM | 3841 | OD1 | ASP | B | 192 | 34.910 | 65.581 | 91.657 | 1.00 | 41.22 | B | O |
| ATOM | 3842 | OD2 | ASP | B | 192 | 33.129 | 66.790 | 91.199 | 1.00 | 41.40 | B | O |
| ATOM | 3843 | C | ASP | B | 192 | 32.632 | 62.906 | 94.039 | 1.00 | 36.28 | B | C |
| ATOM | 3844 | O | ASP | B | 192 | 33.296 | 62.791 | 95.063 | 1.00 | 37.01 | B | O |
| ATOM | 3845 | N | THR | B | 193 | 31.389 | 62.447 | 93.937 | 1.00 | 33.67 | B | N |
| ATOM | 3846 | CA | THR | B | 193 | 30.744 | 61.774 | 95.067 | 1.00 | 30.70 | B | C |
| ATOM | 3847 | CB | THR | B | 193 | 29.284 | 62.232 | 95.223 | 1.00 | 31.07 | B | C |
| ATOM | 3848 | OG1 | THR | B | 193 | 28.571 | 61.964 | 94.010 | 1.00 | 30.40 | B | O |

| | | | | | | | | | | | | |
|---|---|---|---|---|---|---|---|---|---|---|---|---|
| ATOM | 3849 | CG2 | THR | B | 193 | 29.218 | 63.730 | 95.529 | 1.00 | 28.84 | B | C |
| ATOM | 3850 | C | THR | B | 193 | 30.744 | 60.256 | 94.946 | 1.00 | 28.29 | B | C |
| ATOM | 3851 | O | THR | B | 193 | 30.404 | 59.570 | 95.885 | 1.00 | 27.69 | B | O |
| ATOM | 3852 | N | ALA | B | 194 | 31.124 | 59.753 | 93.774 | 1.00 | 26.20 | B | N |
| ATOM | 3853 | CA | ALA | B | 194 | 31.167 | 58.315 | 93.512 | 1.00 | 25.82 | B | C |
| ATOM | 3854 | CB | ALA | B | 194 | 32.104 | 57.620 | 94.519 | 1.00 | 23.20 | B | C |
| ATOM | 3855 | C | ALA | B | 194 | 29.771 | 57.678 | 93.559 | 1.00 | 24.35 | B | C |
| ATOM | 3856 | O | ALA | B | 194 | 29.630 | 56.501 | 93.793 | 1.00 | 22.79 | B | O |
| ATOM | 3857 | N | VAL | B | 195 | 28.748 | 58.487 | 93.317 | 1.00 | 24.20 | B | N |
| ATOM | 3858 | CA | VAL | B | 195 | 27.373 | 58.008 | 93.316 | 1.00 | 24.68 | B | C |
| ATOM | 3859 | CB | VAL | B | 195 | 26.401 | 59.135 | 93.752 | 1.00 | 26.38 | B | C |
| ATOM | 3860 | CG1 | VAL | B | 195 | 24.946 | 58.711 | 93.501 | 1.00 | 26.09 | B | C |
| ATOM | 3861 | CG2 | VAL | B | 195 | 26.609 | 59.456 | 95.240 | 1.00 | 23.95 | B | C |
| ATOM | 3862 | C | VAL | B | 195 | 26.977 | 57.522 | 91.918 | 1.00 | 24.97 | B | C |
| ATOM | 3863 | O | VAL | B | 195 | 27.272 | 58.170 | 90.919 | 1.00 | 24.97 | B | O |
| ATOM | 3864 | N | LEU | B | 196 | 26.312 | 56.372 | 91.860 | 1.00 | 23.36 | B | N |
| ATOM | 3865 | CA | LEU | B | 196 | 25.880 | 55.814 | 90.588 | 1.00 | 22.63 | B | C |
| ATOM | 3866 | CB | LEU | B | 196 | 26.439 | 54.389 | 90.413 | 1.00 | 23.07 | B | C |
| ATOM | 3867 | CG | LEU | B | 196 | 25.995 | 53.568 | 89.188 | 1.00 | 22.32 | B | C |
| ATOM | 3868 | CD1 | LEU | B | 196 | 27.031 | 52.513 | 88.835 | 1.00 | 20.89 | B | C |
| ATOM | 3869 | CD2 | LEU | B | 196 | 24.658 | 52.922 | 89.471 | 1.00 | 20.34 | B | C |
| ATOM | 3870 | C | LEU | B | 196 | 24.366 | 55.799 | 90.506 | 1.00 | 22.12 | B | C |
| ATOM | 3871 | O | LEU | B | 196 | 23.692 | 55.444 | 91.455 | 1.00 | 22.46 | B | O |
| ATOM | 3872 | N | LYS | B | 197 | 23.840 | 56.205 | 89.359 | 1.00 | 21.40 | B | N |
| ATOM | 3873 | CA | LYS | B | 197 | 22.401 | 56.230 | 89.155 | 1.00 | 20.52 | B | C |
| ATOM | 3874 | CB | LYS | B | 197 | 21.884 | 57.672 | 89.111 | 1.00 | 21.80 | B | C |
| ATOM | 3875 | CG | LYS | B | 197 | 22.102 | 58.463 | 90.397 | 1.00 | 23.52 | B | C |
| ATOM | 3876 | CD | LYS | B | 197 | 21.410 | 59.820 | 90.329 | 1.00 | 27.44 | B | C |
| ATOM | 3877 | CE | LYS | B | 197 | 21.757 | 60.694 | 91.530 | 1.00 | 26.35 | B | C |
| ATOM | 3878 | NZ | LYS | B | 197 | 21.603 | 59.944 | 92.793 | 1.00 | 29.52 | B | N |
| ATOM | 3879 | C | LYS | B | 197 | 22.008 | 55.517 | 87.873 | 1.00 | 20.15 | B | C |
| ATOM | 3880 | O | LYS | B | 197 | 22.571 | 55.761 | 86.821 | 1.00 | 16.06 | B | O |
| ATOM | 3881 | N | LEU | B | 198 | 21.035 | 54.622 | 87.990 | 1.00 | 18.98 | B | N |
| ATOM | 3882 | CA | LEU | B | 198 | 20.527 | 53.881 | 86.852 | 1.00 | 18.22 | B | C |
| ATOM | 3883 | CB | LEU | B | 198 | 19.777 | 52.640 | 87.349 | 1.00 | 17.57 | B | C |
| ATOM | 3884 | CG | LEU | B | 198 | 19.200 | 51.671 | 86.318 | 1.00 | 16.99 | B | C |
| ATOM | 3885 | CD1 | LEU | B | 198 | 20.318 | 51.095 | 85.468 | 1.00 | 17.46 | B | C |
| ATOM | 3886 | CD2 | LEU | B | 198 | 18.451 | 50.565 | 87.035 | 1.00 | 15.22 | B | C |
| ATOM | 3887 | C | LEU | B | 198 | 19.581 | 54.815 | 86.097 | 1.00 | 18.35 | B | C |
| ATOM | 3888 | O | LEU | B | 198 | 18.847 | 55.573 | 86.716 | 1.00 | 18.41 | B | O |
| ATOM | 3889 | N | CYS | B | 199 | 19.624 | 54.765 | 84.766 | 1.00 | 18.99 | B | N |
| ATOM | 3890 | CA | CYS | B | 199 | 18.776 | 55.606 | 83.914 | 1.00 | 18.78 | B | C |
| ATOM | 3891 | CB | CYS | B | 199 | 19.554 | 56.832 | 83.414 | 1.00 | 18.96 | B | C |
| ATOM | 3892 | SG | CYS | B | 199 | 20.731 | 56.503 | 82.075 | 1.00 | 19.73 | B | S |
| ATOM | 3893 | C | CYS | B | 199 | 18.257 | 54.815 | 82.714 | 1.00 | 19.20 | B | C |
| ATOM | 3894 | O | CYS | B | 199 | 18.576 | 53.648 | 82.552 | 1.00 | 18.40 | B | O |
| ATOM | 3895 | N | ASP | B | 200 | 17.469 | 55.480 | 81.875 | 1.00 | 19.26 | B | N |
| ATOM | 3896 | CA | ASP | B | 200 | 16.887 | 54.882 | 80.675 | 1.00 | 21.20 | B | C |
| ATOM | 3897 | CB | ASP | B | 200 | 17.986 | 54.494 | 79.679 | 1.00 | 21.39 | B | C |
| ATOM | 3898 | CG | ASP | B | 200 | 17.426 | 53.973 | 78.368 | 1.00 | 22.10 | B | C |
| ATOM | 3899 | OD1 | ASP | B | 200 | 16.210 | 54.111 | 78.135 | 1.00 | 23.97 | B | O |
| ATOM | 3900 | OD2 | ASP | B | 200 | 18.198 | 53.427 | 77.564 | 1.00 | 24.08 | B | O |
| ATOM | 3901 | C | ASP | B | 200 | 15.982 | 53.679 | 80.956 | 1.00 | 21.59 | B | C |
| ATOM | 3902 | O | ASP | B | 200 | 16.381 | 52.513 | 80.808 | 1.00 | 22.15 | B | O |
| ATOM | 3903 | N | PHE | B | 201 | 14.752 | 53.976 | 81.351 | 1.00 | 20.94 | B | N |
| ATOM | 3904 | CA | PHE | B | 201 | 13.782 | 52.940 | 81.647 | 1.00 | 20.87 | B | C |
| ATOM | 3905 | CB | PHE | B | 201 | 13.009 | 53.305 | 82.917 | 1.00 | 20.01 | B | C |
| ATOM | 3906 | CG | PHE | B | 201 | 13.818 | 53.152 | 84.164 | 1.00 | 20.27 | B | C |
| ATOM | 3907 | CD1 | PHE | B | 201 | 14.845 | 54.047 | 84.460 | 1.00 | 19.83 | B | C |
| ATOM | 3908 | CD2 | PHE | B | 201 | 13.613 | 52.065 | 85.005 | 1.00 | 18.14 | B | C |
| ATOM | 3909 | CE1 | PHE | B | 201 | 15.659 | 53.858 | 85.573 | 1.00 | 16.64 | B | C |
| ATOM | 3910 | CE2 | PHE | B | 201 | 14.419 | 51.864 | 86.117 | 1.00 | 20.03 | B | C |
| ATOM | 3911 | CZ | PHE | B | 201 | 15.448 | 52.763 | 86.403 | 1.00 | 17.76 | B | C |

| | | | | | | | | | | | | |
|---|---|---|---|---|---|---|---|---|---|---|---|---|
| ATOM | 3912 | C | PHE | B | 201 | 12.833 | 52.676 | 80.488 | 1.00 | 19.77 | B | C |
| ATOM | 3913 | O | PHE | B | 201 | 11.745 | 52.186 | 80.685 | 1.00 | 21.01 | B | O |
| ATOM | 3914 | N | GLY | B | 202 | 13.283 | 52.995 | 79.276 | 1.00 | 20.60 | B | N |
| ATOM | 3915 | CA | GLY | B | 202 | 12.471 | 52.780 | 78.089 | 1.00 | 19.16 | B | C |
| ATOM | 3916 | C | GLY | B | 202 | 12.211 | 51.315 | 77.768 | 1.00 | 19.09 | B | C |
| ATOM | 3917 | O | GLY | B | 202 | 11.342 | 51.011 | 76.981 | 1.00 | 19.34 | B | O |
| ATOM | 3918 | N | SER | B | 203 | 12.969 | 50.410 | 78.374 | 1.00 | 18.23 | B | N |
| ATOM | 3919 | CA | SER | B | 203 | 12.772 | 48.981 | 78.143 | 1.00 | 21.08 | B | C |
| ATOM | 3920 | CB | SER | B | 203 | 14.097 | 48.303 | 77.769 | 1.00 | 20.35 | B | C |
| ATOM | 3921 | OG | SER | B | 203 | 14.625 | 48.819 | 76.565 | 1.00 | 22.39 | B | O |
| ATOM | 3922 | C | SER | B | 203 | 12.218 | 48.303 | 79.402 | 1.00 | 21.25 | B | C |
| ATOM | 3923 | O | SER | B | 203 | 11.814 | 47.151 | 79.366 | 1.00 | 21.87 | B | O |
| ATOM | 3924 | N | ALA | B | 204 | 12.210 | 49.029 | 80.514 | 1.00 | 21.58 | B | N |
| ATOM | 3925 | CA | ALA | B | 204 | 11.737 | 48.466 | 81.770 | 1.00 | 21.26 | B | C |
| ATOM | 3926 | CB | ALA | B | 204 | 11.852 | 49.485 | 82.879 | 1.00 | 21.06 | B | C |
| ATOM | 3927 | C | ALA | B | 204 | 10.310 | 47.945 | 81.694 | 1.00 | 22.75 | B | C |
| ATOM | 3928 | O | ALA | B | 204 | 9.465 | 48.533 | 81.055 | 1.00 | 19.66 | B | O |
| ATOM | 3929 | N | LYS | B | 205 | 10.064 | 46.820 | 82.357 | 1.00 | 24.34 | B | N |
| ATOM | 3930 | CA | LYS | B | 205 | 8.733 | 46.232 | 82.369 | 1.00 | 25.50 | B | C |
| ATOM | 3931 | CB | LYS | B | 205 | 8.456 | 45.506 | 81.049 | 1.00 | 26.87 | B | C |
| ATOM | 3932 | CG | LYS | B | 205 | 7.063 | 44.887 | 80.975 | 1.00 | 29.53 | B | C |
| ATOM | 3933 | CD | LYS | B | 205 | 6.785 | 44.232 | 79.628 | 1.00 | 32.14 | B | C |
| ATOM | 3934 | CE | LYS | B | 205 | 5.423 | 43.533 | 79.624 | 1.00 | 32.08 | B | C |
| ATOM | 3935 | NZ | LYS | B | 205 | 5.199 | 42.772 | 78.353 | 1.00 | 38.10 | B | N |
| ATOM | 3936 | C | LYS | B | 205 | 8.507 | 45.262 | 83.529 | 1.00 | 25.37 | B | C |
| ATOM | 3937 | O | LYS | B | 205 | 9.393 | 44.501 | 83.914 | 1.00 | 24.30 | B | O |
| ATOM | 3938 | N | GLN | B | 206 | 7.307 | 45.295 | 84.090 | 1.00 | 26.61 | B | N |
| ATOM | 3939 | CA | GLN | B | 206 | 6.995 | 44.385 | 85.176 | 1.00 | 28.25 | B | C |
| ATOM | 3940 | CB | GLN | B | 206 | 5.665 | 44.762 | 85.839 | 1.00 | 31.79 | B | C |
| ATOM | 3941 | CG | GLN | B | 206 | 5.370 | 43.975 | 87.119 | 1.00 | 36.57 | B | C |
| ATOM | 3942 | CD | GLN | B | 206 | 4.183 | 44.532 | 87.912 | 1.00 | 40.82 | B | C |
| ATOM | 3943 | OE1 | GLN | B | 206 | 3.916 | 44.098 | 89.062 | 1.00 | 42.49 | B | O |
| ATOM | 3944 | NE2 | GLN | B | 206 | 3.461 | 45.488 | 87.316 | 1.00 | 41.19 | B | N |
| ATOM | 3945 | C | GLN | B | 206 | 6.894 | 43.008 | 84.545 | 1.00 | 28.60 | B | C |
| ATOM | 3946 | O | GLN | B | 206 | 6.188 | 42.828 | 83.548 | 1.00 | 28.36 | B | O |
| ATOM | 3947 | N | LEU | B | 207 | 7.626 | 42.048 | 85.099 | 1.00 | 28.92 | B | N |
| ATOM | 3948 | CA | LEU | B | 207 | 7.606 | 40.689 | 84.579 | 1.00 | 29.53 | B | C |
| ATOM | 3949 | CB | LEU | B | 207 | 9.025 | 40.118 | 84.498 | 1.00 | 28.73 | B | C |
| ATOM | 3950 | CG | LEU | B | 207 | 10.064 | 40.834 | 83.620 | 1.00 | 28.73 | B | C |
| ATOM | 3951 | CD1 | LEU | B | 207 | 11.356 | 40.027 | 83.602 | 1.00 | 27.04 | B | C |
| ATOM | 3952 | CD2 | LEU | B | 207 | 9.535 | 41.001 | 82.214 | 1.00 | 26.77 | B | C |
| ATOM | 3953 | C | LEU | B | 207 | 6.733 | 39.805 | 85.465 | 1.00 | 31.83 | B | C |
| ATOM | 3954 | O | LEU | B | 207 | 7.081 | 39.525 | 86.605 | 1.00 | 32.51 | B | O |
| ATOM | 3955 | N | VAL | B | 208 | 5.593 | 39.382 | 84.925 | 1.00 | 32.85 | B | N |
| ATOM | 3956 | CA | VAL | B | 208 | 4.651 | 38.526 | 85.639 | 1.00 | 32.67 | B | C |
| ATOM | 3957 | CB | VAL | B | 208 | 3.190 | 38.969 | 85.372 | 1.00 | 33.21 | B | C |
| ATOM | 3958 | CG1 | VAL | B | 208 | 2.219 | 38.066 | 86.137 | 1.00 | 33.10 | B | C |
| ATOM | 3959 | CG2 | VAL | B | 208 | 3.005 | 40.430 | 85.788 | 1.00 | 30.55 | B | C |
| ATOM | 3960 | C | VAL | B | 208 | 4.828 | 37.081 | 85.166 | 1.00 | 33.20 | B | C |
| ATOM | 3961 | O | VAL | B | 208 | 4.955 | 36.827 | 83.959 | 1.00 | 32.70 | B | O |
| ATOM | 3962 | N | ALA | B | 209 | 4.837 | 36.139 | 86.109 | 1.00 | 33.05 | B | N |
| ATOM | 3963 | CA | ALA | B | 209 | 5.007 | 34.724 | 85.775 | 1.00 | 33.77 | B | C |
| ATOM | 3964 | CB | ALA | B | 209 | 5.096 | 33.889 | 87.047 | 1.00 | 33.63 | B | C |
| ATOM | 3965 | C | ALA | B | 209 | 3.873 | 34.215 | 84.890 | 1.00 | 33.72 | B | C |
| ATOM | 3966 | O | ALA | B | 209 | 2.703 | 34.491 | 85.139 | 1.00 | 34.36 | B | O |
| ATOM | 3967 | N | GLY | B | 210 | 4.232 | 33.477 | 83.846 | 1.00 | 34.07 | B | N |
| ATOM | 3968 | CA | GLY | B | 210 | 3.223 | 32.964 | 82.940 | 1.00 | 33.96 | B | C |
| ATOM | 3969 | C | GLY | B | 210 | 2.947 | 33.903 | 81.772 | 1.00 | 34.33 | B | C |
| ATOM | 3970 | O | GLY | B | 210 | 2.317 | 33.517 | 80.797 | 1.00 | 34.80 | B | O |
| ATOM | 3971 | N | GLU | B | 211 | 3.395 | 35.149 | 81.869 | 1.00 | 33.95 | B | N |
| ATOM | 3972 | CA | GLU | B | 211 | 3.181 | 36.093 | 80.773 | 1.00 | 34.51 | B | C |
| ATOM | 3973 | CB | GLU | B | 211 | 2.901 | 37.506 | 81.302 | 1.00 | 35.74 | B | C |
| ATOM | 3974 | CG | GLU | B | 211 | 1.576 | 37.660 | 82.039 | 1.00 | 37.35 | B | C |

| | | | | | | | | | | | | |
|---|---|---|---|---|---|---|---|---|---|---|---|---|
| ATOM | 3975 | CD | GLU | B | 211 | 1.319 | 39.090 | 82.488 | 1.00 | 39.05 | B | C |
| ATOM | 3976 | OE1 | GLU | B | 211 | 0.232 | 39.350 | 83.051 | 1.00 | 41.80 | B | O |
| ATOM | 3977 | OE2 | GLU | B | 211 | 2.199 | 39.959 | 82.281 | 1.00 | 38.46 | B | O |
| ATOM | 3978 | C | GLU | B | 211 | 4.425 | 36.127 | 79.876 | 1.00 | 33.84 | B | C |
| ATOM | 3979 | O | GLU | B | 211 | 5.547 | 36.158 | 80.361 | 1.00 | 33.91 | B | O |
| ATOM | 3980 | N | PRO | B | 212 | 4.236 | 36.099 | 78.551 | 1.00 | 32.61 | B | N |
| ATOM | 3981 | CD | PRO | B | 212 | 3.029 | 35.721 | 77.793 | 1.00 | 32.06 | B | C |
| ATOM | 3982 | CA | PRO | B | 212 | 5.418 | 36.130 | 77.686 | 1.00 | 31.05 | B | C |
| ATOM | 3983 | CB | PRO | B | 212 | 4.944 | 35.406 | 76.432 | 1.00 | 31.34 | B | C |
| ATOM | 3984 | CG | PRO | B | 212 | 3.500 | 35.819 | 76.349 | 1.00 | 33.13 | B | C |
| ATOM | 3985 | C | PRO | B | 212 | 5.919 | 37.544 | 77.392 | 1.00 | 29.70 | B | C |
| ATOM | 3986 | O | PRO | B | 212 | 5.146 | 38.499 | 77.355 | 1.00 | 28.93 | B | O |
| ATOM | 3987 | N | ASN | B | 213 | 7.224 | 37.660 | 77.176 | 1.00 | 26.44 | B | N |
| ATOM | 3988 | CA | ASN | B | 213 | 7.829 | 38.946 | 76.893 | 1.00 | 24.71 | B | C |
| ATOM | 3989 | CB | ASN | B | 213 | 8.512 | 39.465 | 78.154 | 1.00 | 24.12 | B | C |
| ATOM | 3990 | CG | ASN | B | 213 | 7.508 | 39.813 | 79.238 | 1.00 | 24.81 | B | C |
| ATOM | 3991 | OD1 | ASN | B | 213 | 6.789 | 40.807 | 79.130 | 1.00 | 25.19 | B | O |
| ATOM | 3992 | ND2 | ASN | B | 213 | 7.431 | 38.979 | 80.273 | 1.00 | 20.74 | B | N |
| ATOM | 3993 | C | ASN | B | 213 | 8.810 | 38.842 | 75.732 | 1.00 | 24.37 | B | C |
| ATOM | 3994 | O | ASN | B | 213 | 9.464 | 37.813 | 75.551 | 1.00 | 23.57 | B | O |
| ATOM | 3995 | N | VAL | B | 214 | 8.891 | 39.915 | 74.945 | 1.00 | 23.72 | B | N |
| ATOM | 3996 | CA | VAL | B | 214 | 9.771 | 39.953 | 73.784 | 1.00 | 22.40 | B | C |
| ATOM | 3997 | CB | VAL | B | 214 | 9.721 | 41.335 | 73.089 | 1.00 | 23.09 | B | C |
| ATOM | 3998 | CG1 | VAL | B | 214 | 8.279 | 41.659 | 72.711 | 1.00 | 23.23 | B | C |
| ATOM | 3999 | CG2 | VAL | B | 214 | 10.295 | 42.423 | 74.002 | 1.00 | 23.43 | B | C |
| ATOM | 4000 | C | VAL | B | 214 | 11.204 | 39.616 | 74.158 | 1.00 | 22.65 | B | C |
| ATOM | 4001 | O | VAL | B | 214 | 11.686 | 39.996 | 75.222 | 1.00 | 20.95 | B | O |
| ATOM | 4002 | N | SER | B | 215 | 11.891 | 38.894 | 73.283 | 1.00 | 23.20 | B | N |
| ATOM | 4003 | CA | SER | B | 215 | 13.260 | 38.515 | 73.594 | 1.00 | 24.77 | B | C |
| ATOM | 4004 | CB | SER | B | 215 | 13.478 | 37.018 | 73.312 | 1.00 | 26.83 | B | C |
| ATOM | 4005 | OG | SER | B | 215 | 13.280 | 36.689 | 71.945 | 1.00 | 26.38 | B | O |
| ATOM | 4006 | C | SER | B | 215 | 14.321 | 39.343 | 72.871 | 1.00 | 24.55 | B | C |
| ATOM | 4007 | O | SER | B | 215 | 15.485 | 39.021 | 72.933 | 1.00 | 26.32 | B | O |
| ATOM | 4008 | N | TYR | B | 216 | 13.916 | 40.420 | 72.210 | 1.00 | 22.14 | B | N |
| ATOM | 4009 | CA | TYR | B | 216 | 14.870 | 41.255 | 71.477 | 1.00 | 23.30 | B | C |
| ATOM | 4010 | CB | TYR | B | 216 | 14.315 | 41.550 | 70.080 | 1.00 | 22.47 | B | C |
| ATOM | 4011 | CG | TYR | B | 216 | 12.900 | 42.096 | 70.084 | 1.00 | 24.45 | B | C |
| ATOM | 4012 | CD1 | TYR | B | 216 | 12.637 | 43.429 | 70.412 | 1.00 | 26.49 | B | C |
| ATOM | 4013 | CE1 | TYR | B | 216 | 11.324 | 43.924 | 70.417 | 1.00 | 26.61 | B | C |
| ATOM | 4014 | CD2 | TYR | B | 216 | 11.820 | 41.273 | 69.773 | 1.00 | 23.82 | B | C |
| ATOM | 4015 | CE2 | TYR | B | 216 | 10.515 | 41.750 | 69.779 | 1.00 | 22.15 | B | C |
| ATOM | 4016 | CZ | TYR | B | 216 | 10.269 | 43.069 | 70.099 | 1.00 | 26.83 | B | C |
| ATOM | 4017 | OH | TYR | B | 216 | 8.969 | 43.531 | 70.090 | 1.00 | 27.14 | B | O |
| ATOM | 4018 | C | TYR | B | 216 | 15.160 | 42.569 | 72.192 | 1.00 | 22.00 | B | C |
| ATOM | 4019 | O | TYR | B | 216 | 15.546 | 43.547 | 71.572 | 1.00 | 20.70 | B | O |
| ATOM | 4020 | N | ILE | B | 217 | 15.020 | 42.541 | 73.513 | 1.00 | 23.21 | B | N |
| ATOM | 4021 | CA | ILE | B | 217 | 15.154 | 43.711 | 74.365 | 1.00 | 24.46 | B | C |
| ATOM | 4022 | CB | ILE | B | 217 | 14.186 | 43.536 | 75.572 | 1.00 | 24.58 | B | C |
| ATOM | 4023 | CG2 | ILE | B | 217 | 14.795 | 42.621 | 76.650 | 1.00 | 21.47 | B | C |
| ATOM | 4024 | CG1 | ILE | B | 217 | 13.826 | 44.897 | 76.137 | 1.00 | 26.80 | B | C |
| ATOM | 4025 | CD1 | ILE | B | 217 | 12.891 | 45.650 | 75.237 | 1.00 | 27.53 | B | C |
| ATOM | 4026 | C | ILE | B | 217 | 16.498 | 44.204 | 74.918 | 1.00 | 25.63 | B | C |
| ATOM | 4027 | O | ILE | B | 217 | 16.637 | 45.412 | 75.225 | 1.00 | 31.55 | B | O |
| ATOM | 4028 | N | CYS | B | 218 | 17.487 | 43.333 | 75.059 | 1.00 | 23.77 | B | N |
| ATOM | 4029 | CA | CYS | B | 218 | 18.752 | 43.778 | 75.659 | 1.00 | 21.88 | B | C |
| ATOM | 4030 | CB | CYS | B | 218 | 19.252 | 42.671 | 76.597 | 1.00 | 20.93 | B | C |
| ATOM | 4031 | SG | CYS | B | 218 | 20.451 | 43.132 | 77.853 | 1.00 | 18.00 | B | S |
| ATOM | 4032 | C | CYS | B | 218 | 19.820 | 44.171 | 74.619 | 1.00 | 20.36 | B | C |
| ATOM | 4033 | O | CYS | B | 218 | 19.697 | 43.855 | 73.454 | 1.00 | 19.25 | B | O |
| ATOM | 4034 | N | SER | B | 219 | 20.861 | 44.872 | 75.054 | 1.00 | 20.20 | B | N |
| ATOM | 4035 | CA | SER | B | 219 | 21.910 | 45.324 | 74.142 | 1.00 | 20.24 | B | C |
| ATOM | 4036 | CB | SER | B | 219 | 22.427 | 46.692 | 74.593 | 1.00 | 21.62 | B | C |
| ATOM | 4037 | OG | SER | B | 219 | 21.467 | 47.703 | 74.344 | 1.00 | 22.61 | B | O |

| | | | | | | | | | | | | |
|---|---|---|---|---|---|---|---|---|---|---|---|---|
| ATOM | 4038 | C | SER | B | 219 | 23.090 | 44.380 | 73.927 | 1.00 | 20.73 | B | C |
| ATOM | 4039 | O | SER | B | 219 | 23.507 | 43.699 | 74.837 | 1.00 | 20.26 | B | O |
| ATOM | 4040 | N | ALA | B | 220 | 23.614 | 44.365 | 72.701 | 1.00 | 21.57 | B | N |
| ATOM | 4041 | CA | ALA | B | 220 | 24.755 | 43.524 | 72.320 | 1.00 | 22.45 | B | C |
| ATOM | 4042 | CB | ALA | B | 220 | 25.296 | 43.971 | 70.962 | 1.00 | 23.18 | B | C |
| ATOM | 4043 | C | ALA | B | 220 | 25.873 | 43.577 | 73.346 | 1.00 | 20.42 | B | C |
| ATOM | 4044 | O | ALA | B | 220 | 26.277 | 44.630 | 73.724 | 1.00 | 19.81 | B | O |
| ATOM | 4045 | N | TYR | B | 221 | 26.365 | 42.399 | 73.728 | 1.00 | 20.58 | B | N |
| ATOM | 4046 | CA | TYR | B | 221 | 27.435 | 42.216 | 74.717 | 1.00 | 20.23 | B | C |
| ATOM | 4047 | CB | TYR | B | 221 | 28.346 | 43.450 | 74.867 | 1.00 | 21.68 | B | C |
| ATOM | 4048 | CG | TYR | B | 221 | 29.188 | 43.883 | 73.675 | 1.00 | 24.04 | B | C |
| ATOM | 4049 | CD1 | TYR | B | 221 | 29.327 | 43.080 | 72.536 | 1.00 | 25.90 | B | C |
| ATOM | 4050 | CE1 | TYR | B | 221 | 30.098 | 43.502 | 71.445 | 1.00 | 26.31 | B | C |
| ATOM | 4051 | CD2 | TYR | B | 221 | 29.843 | 45.115 | 73.693 | 1.00 | 23.88 | B | C |
| ATOM | 4052 | CE2 | TYR | B | 221 | 30.612 | 45.540 | 72.621 | 1.00 | 27.25 | B | C |
| ATOM | 4053 | CZ | TYR | B | 221 | 30.738 | 44.734 | 71.500 | 1.00 | 26.91 | B | C |
| ATOM | 4054 | OH | TYR | B | 221 | 31.512 | 45.165 | 70.446 | 1.00 | 28.71 | B | O |
| ATOM | 4055 | C | TYR | B | 221 | 26.881 | 41.914 | 76.102 | 1.00 | 19.77 | B | C |
| ATOM | 4056 | O | TYR | B | 221 | 27.462 | 41.140 | 76.822 | 1.00 | 20.31 | B | O |
| ATOM | 4057 | N | TYR | B | 222 | 25.759 | 42.533 | 76.465 | 1.00 | 19.15 | B | N |
| ATOM | 4058 | CA | TYR | B | 222 | 25.178 | 42.360 | 77.802 | 1.00 | 20.26 | B | C |
| ATOM | 4059 | CB | TYR | B | 222 | 24.870 | 43.745 | 78.383 | 1.00 | 18.46 | B | C |
| ATOM | 4060 | CG | TYR | B | 222 | 26.021 | 44.706 | 78.194 | 1.00 | 19.13 | B | C |
| ATOM | 4061 | CD1 | TYR | B | 222 | 27.136 | 44.669 | 79.039 | 1.00 | 19.51 | B | C |
| ATOM | 4062 | CE1 | TYR | B | 222 | 28.256 | 45.467 | 78.791 | 1.00 | 19.49 | B | C |
| ATOM | 4063 | CD2 | TYR | B | 222 | 26.055 | 45.566 | 77.099 | 1.00 | 18.02 | B | C |
| ATOM | 4064 | CE2 | TYR | B | 222 | 27.167 | 46.366 | 76.843 | 1.00 | 18.28 | B | C |
| ATOM | 4065 | CZ | TYR | B | 222 | 28.263 | 46.307 | 77.684 | 1.00 | 19.86 | B | C |
| ATOM | 4066 | OH | TYR | B | 222 | 29.362 | 47.083 | 77.411 | 1.00 | 19.68 | B | O |
| ATOM | 4067 | C | TYR | B | 222 | 23.938 | 41.480 | 77.899 | 1.00 | 19.48 | B | C |
| ATOM | 4068 | O | TYR | B | 222 | 23.352 | 41.376 | 78.949 | 1.00 | 19.34 | B | O |
| ATOM | 4069 | N | ARG | B | 223 | 23.569 | 40.837 | 76.798 | 1.00 | 18.11 | B | N |
| ATOM | 4070 | CA | ARG | B | 223 | 22.381 | 39.994 | 76.761 | 1.00 | 18.48 | B | C |
| ATOM | 4071 | CB | ARG | B | 223 | 21.912 | 39.843 | 75.321 | 1.00 | 17.53 | B | C |
| ATOM | 4072 | CG | ARG | B | 223 | 21.889 | 41.142 | 74.556 | 1.00 | 19.07 | B | C |
| ATOM | 4073 | CD | ARG | B | 223 | 21.320 | 40.928 | 73.177 | 1.00 | 19.87 | B | C |
| ATOM | 4074 | NE | ARG | B | 223 | 19.865 | 40.942 | 73.200 | 1.00 | 20.11 | B | N |
| ATOM | 4075 | CZ | ARG | B | 223 | 19.097 | 40.053 | 72.584 | 1.00 | 20.06 | B | C |
| ATOM | 4076 | NH1 | ARG | B | 223 | 19.638 | 39.057 | 71.895 | 1.00 | 19.59 | B | N |
| ATOM | 4077 | NH2 | ARG | B | 223 | 17.781 | 40.180 | 72.638 | 1.00 | 20.18 | B | N |
| ATOM | 4078 | C | ARG | B | 223 | 22.580 | 38.612 | 77.379 | 1.00 | 19.44 | B | C |
| ATOM | 4079 | O | ARG | B | 223 | 23.547 | 37.914 | 77.082 | 1.00 | 19.76 | B | O |
| ATOM | 4080 | N | ALA | B | 224 | 21.653 | 38.223 | 78.243 | 1.00 | 19.05 | B | N |
| ATOM | 4081 | CA | ALA | B | 224 | 21.722 | 36.920 | 78.880 | 1.00 | 19.06 | B | C |
| ATOM | 4082 | CB | ALA | B | 224 | 20.647 | 36.792 | 79.951 | 1.00 | 16.11 | B | C |
| ATOM | 4083 | C | ALA | B | 224 | 21.521 | 35.858 | 77.802 | 1.00 | 20.32 | B | C |
| ATOM | 4084 | O | ALA | B | 224 | 20.811 | 36.082 | 76.821 | 1.00 | 20.41 | B | O |
| ATOM | 4085 | N | PRO | B | 225 | 22.165 | 34.694 | 77.969 | 1.00 | 20.46 | B | N |
| ATOM | 4086 | CD | PRO | B | 225 | 23.060 | 34.345 | 79.084 | 1.00 | 20.79 | B | C |
| ATOM | 4087 | CA | PRO | B | 225 | 22.057 | 33.589 | 77.017 | 1.00 | 18.96 | B | C |
| ATOM | 4088 | CB | PRO | B | 225 | 22.851 | 32.466 | 77.688 | 1.00 | 19.69 | B | C |
| ATOM | 4089 | CG | PRO | B | 225 | 22.873 | 32.867 | 79.159 | 1.00 | 20.59 | B | C |
| ATOM | 4090 | C | PRO | B | 225 | 20.615 | 33.207 | 76.669 | 1.00 | 18.78 | B | C |
| ATOM | 4091 | O | PRO | B | 225 | 20.327 | 32.945 | 75.530 | 1.00 | 18.47 | B | O |
| ATOM | 4092 | N | GLU | B | 226 | 19.712 | 33.205 | 77.646 | 1.00 | 19.13 | B | N |
| ATOM | 4093 | CA | GLU | B | 226 | 18.323 | 32.855 | 77.355 | 1.00 | 20.23 | B | C |
| ATOM | 4094 | CB | GLU | B | 226 | 17.484 | 32.816 | 78.638 | 1.00 | 21.15 | B | C |
| ATOM | 4095 | CG | GLU | B | 226 | 17.497 | 34.097 | 79.450 | 1.00 | 20.87 | B | C |
| ATOM | 4096 | CD | GLU | B | 226 | 18.509 | 34.053 | 80.581 | 1.00 | 21.43 | B | C |
| ATOM | 4097 | OE1 | GLU | B | 226 | 19.614 | 33.511 | 80.377 | 1.00 | 21.23 | B | O |
| ATOM | 4098 | OE2 | GLU | B | 226 | 18.199 | 34.569 | 81.672 | 1.00 | 20.39 | B | O |
| ATOM | 4099 | C | GLU | B | 226 | 17.695 | 33.824 | 76.354 | 1.00 | 20.03 | B | C |
| ATOM | 4100 | O | GLU | B | 226 | 16.861 | 33.433 | 75.565 | 1.00 | 19.93 | B | O |

| | | | | | | | | | | | | |
|---|---|---|---|---|---|---|---|---|---|---|---|---|
| ATOM | 4101 | N | LEU | B | 227 | 18.103 | 35.090 | 76.404 | 1.00 | 20.03 | B | N |
| ATOM | 4102 | CA | LEU | B | 227 | 17.580 | 36.093 | 75.479 | 1.00 | 19.66 | B | C |
| ATOM | 4103 | CB | LEU | B | 227 | 18.003 | 37.503 | 75.900 | 1.00 | 18.23 | B | C |
| ATOM | 4104 | CG | LEU | B | 227 | 17.467 | 38.115 | 77.197 | 1.00 | 18.85 | B | C |
| ATOM | 4105 | CD1 | LEU | B | 227 | 18.034 | 39.510 | 77.335 | 1.00 | 16.46 | B | C |
| ATOM | 4106 | CD2 | LEU | B | 227 | 15.942 | 38.160 | 77.195 | 1.00 | 14.82 | B | C |
| ATOM | 4107 | C | LEU | B | 227 | 18.103 | 35.822 | 74.069 | 1.00 | 20.56 | B | C |
| ATOM | 4108 | O | LEU | B | 227 | 17.376 | 35.926 | 73.102 | 1.00 | 20.42 | B | O |
| ATOM | 4109 | N | ILE | B | 228 | 19.382 | 35.482 | 73.966 | 1.00 | 20.20 | B | N |
| ATOM | 4110 | CA | ILE | B | 228 | 19.967 | 35.193 | 72.669 | 1.00 | 21.28 | B | C |
| ATOM | 4111 | CB | ILE | B | 228 | 21.477 | 34.917 | 72.782 | 1.00 | 22.09 | B | C |
| ATOM | 4112 | CG2 | ILE | B | 228 | 22.047 | 34.650 | 71.395 | 1.00 | 21.31 | B | C |
| ATOM | 4113 | CG1 | ILE | B | 228 | 22.179 | 36.111 | 73.449 | 1.00 | 22.87 | B | C |
| ATOM | 4114 | CD1 | ILE | B | 228 | 23.675 | 35.945 | 73.620 | 1.00 | 19.17 | B | C |
| ATOM | 4115 | C | ILE | B | 228 | 19.264 | 33.967 | 72.073 | 1.00 | 21.15 | B | C |
| ATOM | 4116 | O | ILE | B | 228 | 18.960 | 33.930 | 70.889 | 1.00 | 19.46 | B | O |
| ATOM | 4117 | N | PHE | B | 229 | 19.007 | 32.978 | 72.924 | 1.00 | 21.12 | B | N |
| ATOM | 4118 | CA | PHE | B | 229 | 18.333 | 31.761 | 72.507 | 1.00 | 22.54 | B | C |
| ATOM | 4119 | CB | PHE | B | 229 | 18.441 | 30.691 | 73.597 | 1.00 | 21.61 | B | C |
| ATOM | 4120 | CG | PHE | B | 229 | 19.747 | 29.935 | 73.595 | 1.00 | 22.04 | B | C |
| ATOM | 4121 | CD1 | PHE | B | 229 | 20.147 | 29.205 | 72.479 | 1.00 | 22.75 | B | C |
| ATOM | 4122 | CD2 | PHE | B | 229 | 20.554 | 29.913 | 74.729 | 1.00 | 22.62 | B | C |
| ATOM | 4123 | CE1 | PHE | B | 229 | 21.334 | 28.458 | 72.499 | 1.00 | 24.68 | B | C |
| ATOM | 4124 | CE2 | PHE | B | 229 | 21.740 | 29.171 | 74.757 | 1.00 | 21.88 | B | C |
| ATOM | 4125 | CZ | PHE | B | 229 | 22.129 | 28.441 | 73.641 | 1.00 | 21.95 | B | C |
| ATOM | 4126 | C | PHE | B | 229 | 16.867 | 32.011 | 72.149 | 1.00 | 23.69 | B | C |
| ATOM | 4127 | O | PHE | B | 229 | 16.210 | 31.136 | 71.636 | 1.00 | 25.36 | B | O |
| ATOM | 4128 | N | GLY | B | 230 | 16.365 | 33.212 | 72.437 | 1.00 | 23.28 | B | N |
| ATOM | 4129 | CA | GLY | B | 230 | 14.991 | 33.546 | 72.092 | 1.00 | 22.60 | B | C |
| ATOM | 4130 | C | GLY | B | 230 | 13.886 | 33.186 | 73.073 | 1.00 | 22.98 | B | C |
| ATOM | 4131 | O | GLY | B | 230 | 12.729 | 33.081 | 72.687 | 1.00 | 23.31 | B | O |
| ATOM | 4132 | N | ALA | B | 231 | 14.233 | 32.993 | 74.341 | 1.00 | 22.37 | B | N |
| ATOM | 4133 | CA | ALA | B | 231 | 13.231 | 32.658 | 75.344 | 1.00 | 21.04 | B | C |
| ATOM | 4134 | CB | ALA | B | 231 | 13.906 | 32.227 | 76.644 | 1.00 | 21.67 | B | C |
| ATOM | 4135 | C | ALA | B | 231 | 12.345 | 33.864 | 75.594 | 1.00 | 20.81 | B | C |
| ATOM | 4136 | O | ALA | B | 231 | 12.793 | 34.987 | 75.497 | 1.00 | 20.98 | B | O |
| ATOM | 4137 | N | THR | B | 232 | 11.079 | 33.608 | 75.901 | 1.00 | 21.63 | B | N |
| ATOM | 4138 | CA | THR | B | 232 | 10.122 | 34.667 | 76.185 | 1.00 | 21.91 | B | C |
| ATOM | 4139 | CB | THR | B | 232 | 8.954 | 34.659 | 75.181 | 1.00 | 22.66 | B | C |
| ATOM | 4140 | OG1 | THR | B | 232 | 8.249 | 33.418 | 75.284 | 1.00 | 22.84 | B | O |
| ATOM | 4141 | CG2 | THR | B | 232 | 9.472 | 34.839 | 73.751 | 1.00 | 22.60 | B | C |
| ATOM | 4142 | C | THR | B | 232 | 9.559 | 34.500 | 77.592 | 1.00 | 22.71 | B | C |
| ATOM | 4143 | O | THR | B | 232 | 8.645 | 35.212 | 77.990 | 1.00 | 22.59 | B | O |
| ATOM | 4144 | N | ASP | B | 233 | 10.119 | 33.550 | 78.338 | 1.00 | 22.46 | B | N |
| ATOM | 4145 | CA | ASP | B | 233 | 9.682 | 33.280 | 79.706 | 1.00 | 24.98 | B | C |
| ATOM | 4146 | CB | ASP | B | 233 | 9.273 | 31.810 | 79.846 | 1.00 | 25.12 | B | C |
| ATOM | 4147 | CG | ASP | B | 233 | 10.450 | 30.869 | 79.711 | 1.00 | 28.36 | B | C |
| ATOM | 4148 | OD1 | ASP | B | 233 | 11.380 | 31.181 | 78.932 | 1.00 | 29.73 | B | O |
| ATOM | 4149 | OD2 | ASP | B | 233 | 10.444 | 29.806 | 80.367 | 1.00 | 30.28 | B | O |
| ATOM | 4150 | C | ASP | B | 233 | 10.822 | 33.593 | 80.671 | 1.00 | 24.58 | B | C |
| ATOM | 4151 | O | ASP | B | 233 | 10.952 | 32.967 | 81.723 | 1.00 | 23.76 | B | O |
| ATOM | 4152 | N | TYR | B | 234 | 11.651 | 34.567 | 80.301 | 1.00 | 23.58 | B | N |
| ATOM | 4153 | CA | TYR | B | 234 | 12.786 | 34.943 | 81.137 | 1.00 | 21.60 | B | C |
| ATOM | 4154 | CB | TYR | B | 234 | 13.809 | 35.730 | 80.325 | 1.00 | 19.91 | B | C |
| ATOM | 4155 | CG | TYR | B | 234 | 13.254 | 36.975 | 79.659 | 1.00 | 19.23 | B | C |
| ATOM | 4156 | CD1 | TYR | B | 234 | 12.743 | 36.927 | 78.361 | 1.00 | 18.95 | B | C |
| ATOM | 4157 | CE1 | TYR | B | 234 | 12.253 | 38.074 | 77.734 | 1.00 | 19.39 | B | C |
| ATOM | 4158 | CD2 | TYR | B | 234 | 13.253 | 38.202 | 80.319 | 1.00 | 17.93 | B | C |
| ATOM | 4159 | CE2 | TYR | B | 234 | 12.766 | 39.356 | 79.700 | 1.00 | 17.98 | B | C |
| ATOM | 4160 | CZ | TYR | B | 234 | 12.269 | 39.284 | 78.413 | 1.00 | 18.72 | B | C |
| ATOM | 4161 | OH | TYR | B | 234 | 11.785 | 40.409 | 77.796 | 1.00 | 17.19 | B | O |
| ATOM | 4162 | C | TYR | B | 234 | 12.354 | 35.763 | 82.343 | 1.00 | 21.61 | B | C |
| ATOM | 4163 | O | TYR | B | 234 | 11.302 | 36.350 | 82.339 | 1.00 | 23.80 | B | O |

| | | | | | | | | | | | | |
|---|---|---|---|---|---|---|---|---|---|---|---|---|
| ATOM | 4164 | N | THR | B | 235 | 13.194 | 35.789 | 83.374 | 1.00 | 22.93 | B | N |
| ATOM | 4165 | CA | THR | B | 235 | 12.906 | 36.544 | 84.591 | 1.00 | 23.36 | B | C |
| ATOM | 4166 | CB | THR | B | 235 | 13.169 | 35.686 | 85.833 | 1.00 | 25.09 | B | C |
| ATOM | 4167 | OG1 | THR | B | 235 | 14.540 | 35.272 | 85.836 | 1.00 | 23.05 | B | O |
| ATOM | 4168 | CG2 | THR | B | 235 | 12.267 | 34.454 | 85.834 | 1.00 | 25.19 | B | C |
| ATOM | 4169 | C | THR | B | 235 | 13.768 | 37.801 | 84.694 | 1.00 | 23.20 | B | C |
| ATOM | 4170 | O | THR | B | 235 | 14.510 | 38.132 | 83.778 | 1.00 | 22.48 | B | O |
| ATOM | 4171 | N | SER | B | 236 | 13.663 | 38.484 | 85.829 | 1.00 | 21.85 | B | N |
| ATOM | 4172 | CA | SER | B | 236 | 14.429 | 39.696 | 86.095 | 1.00 | 22.91 | B | C |
| ATOM | 4173 | CB | SER | B | 236 | 13.972 | 40.328 | 87.416 | 1.00 | 23.54 | B | C |
| ATOM | 4174 | OG | SER | B | 236 | 12.651 | 40.841 | 87.310 | 1.00 | 29.80 | B | O |
| ATOM | 4175 | C | SER | B | 236 | 15.919 | 39.364 | 86.184 | 1.00 | 21.39 | B | C |
| ATOM | 4176 | O | SER | B | 236 | 16.776 | 40.242 | 86.136 | 1.00 | 18.93 | B | O |
| ATOM | 4177 | N | SER | B | 237 | 16.197 | 38.076 | 86.318 | 1.00 | 19.44 | B | N |
| ATOM | 4178 | CA | SER | B | 237 | 17.547 | 37.544 | 86.411 | 1.00 | 20.67 | B | C |
| ATOM | 4179 | CB | SER | B | 237 | 17.442 | 36.021 | 86.426 | 1.00 | 21.10 | B | C |
| ATOM | 4180 | OG | SER | B | 237 | 18.612 | 35.427 | 86.912 | 1.00 | 29.43 | B | O |
| ATOM | 4181 | C | SER | B | 237 | 18.398 | 38.008 | 85.211 | 1.00 | 20.00 | B | C |
| ATOM | 4182 | O | SER | B | 237 | 19.619 | 37.935 | 85.223 | 1.00 | 18.04 | B | O |
| ATOM | 4183 | N | ILE | B | 238 | 17.709 | 38.452 | 84.170 | 1.00 | 18.38 | B | N |
| ATOM | 4184 | CA | ILE | B | 238 | 18.332 | 38.964 | 82.958 | 1.00 | 20.29 | B | C |
| ATOM | 4185 | CB | ILE | B | 238 | 17.194 | 39.436 | 81.983 | 1.00 | 20.99 | B | C |
| ATOM | 4186 | CG2 | ILE | B | 238 | 17.359 | 40.878 | 81.577 | 1.00 | 24.39 | B | C |
| ATOM | 4187 | CG1 | ILE | B | 238 | 17.117 | 38.502 | 80.796 | 1.00 | 21.26 | B | C |
| ATOM | 4188 | CD1 | ILE | B | 238 | 16.662 | 37.140 | 81.174 | 1.00 | 25.68 | B | C |
| ATOM | 4189 | C | ILE | B | 238 | 19.254 | 40.133 | 83.324 | 1.00 | 18.33 | B | C |
| ATOM | 4190 | O | ILE | B | 238 | 20.346 | 40.247 | 82.799 | 1.00 | 18.89 | B | O |
| ATOM | 4191 | N | ASP | B | 239 | 18.791 | 40.988 | 84.236 | 1.00 | 17.71 | B | N |
| ATOM | 4192 | CA | ASP | B | 239 | 19.570 | 42.142 | 84.679 | 1.00 | 16.54 | B | C |
| ATOM | 4193 | CB | ASP | B | 239 | 18.745 | 43.039 | 85.611 | 1.00 | 16.46 | B | C |
| ATOM | 4194 | CG | ASP | B | 239 | 17.584 | 43.722 | 84.903 | 1.00 | 17.67 | B | C |
| ATOM | 4195 | OD1 | ASP | B | 239 | 17.640 | 43.885 | 83.668 | 1.00 | 19.90 | B | O |
| ATOM | 4196 | OD2 | ASP | B | 239 | 16.617 | 44.111 | 85.590 | 1.00 | 17.84 | B | O |
| ATOM | 4197 | C | ASP | B | 239 | 20.851 | 41.721 | 85.403 | 1.00 | 16.47 | B | C |
| ATOM | 4198 | O | ASP | B | 239 | 21.892 | 42.354 | 85.264 | 1.00 | 14.36 | B | O |
| ATOM | 4199 | N | VAL | B | 240 | 20.762 | 40.650 | 86.179 | 1.00 | 15.84 | B | N |
| ATOM | 4200 | CA | VAL | B | 240 | 21.916 | 40.164 | 86.914 | 1.00 | 16.97 | B | C |
| ATOM | 4201 | CB | VAL | B | 240 | 21.533 | 39.000 | 87.844 | 1.00 | 15.91 | B | C |
| ATOM | 4202 | CG1 | VAL | B | 240 | 22.777 | 38.392 | 88.463 | 1.00 | 18.68 | B | C |
| ATOM | 4203 | CG2 | VAL | B | 240 | 20.607 | 39.507 | 88.937 | 1.00 | 18.95 | B | C |
| ATOM | 4204 | C | VAL | B | 240 | 23.014 | 39.715 | 85.950 | 1.00 | 17.33 | B | C |
| ATOM | 4205 | O | VAL | B | 240 | 24.199 | 39.968 | 86.193 | 1.00 | 17.59 | B | O |
| ATOM | 4206 | N | TRP | B | 241 | 22.630 | 39.047 | 84.865 | 1.00 | 16.11 | B | N |
| ATOM | 4207 | CA | TRP | B | 241 | 23.623 | 38.609 | 83.893 | 1.00 | 16.45 | B | C |
| ATOM | 4208 | CB | TRP | B | 241 | 22.988 | 37.716 | 82.813 | 1.00 | 15.82 | B | C |
| ATOM | 4209 | CG | TRP | B | 241 | 23.918 | 37.419 | 81.659 | 1.00 | 16.49 | B | C |
| ATOM | 4210 | CD2 | TRP | B | 241 | 24.718 | 36.244 | 81.466 | 1.00 | 17.69 | B | C |
| ATOM | 4211 | CE2 | TRP | B | 241 | 25.469 | 36.433 | 80.288 | 1.00 | 17.18 | B | C |
| ATOM | 4212 | CE3 | TRP | B | 241 | 24.876 | 35.041 | 82.177 | 1.00 | 16.22 | B | C |
| ATOM | 4213 | CD1 | TRP | B | 241 | 24.209 | 38.248 | 80.611 | 1.00 | 14.97 | B | C |
| ATOM | 4214 | NE1 | TRP | B | 241 | 25.134 | 37.669 | 79.788 | 1.00 | 16.23 | B | N |
| ATOM | 4215 | CZ2 | TRP | B | 241 | 26.363 | 35.480 | 79.800 | 1.00 | 17.92 | B | C |
| ATOM | 4216 | CZ3 | TRP | B | 241 | 25.765 | 34.091 | 81.693 | 1.00 | 16.87 | B | C |
| ATOM | 4217 | CH2 | TRP | B | 241 | 26.497 | 34.315 | 80.516 | 1.00 | 17.97 | B | C |
| ATOM | 4218 | C | TRP | B | 241 | 24.261 | 39.853 | 83.267 | 1.00 | 16.07 | B | C |
| ATOM | 4219 | O | TRP | B | 241 | 25.470 | 39.910 | 83.093 | 1.00 | 14.40 | B | O |
| ATOM | 4220 | N | SER | B | 242 | 23.434 | 40.846 | 82.941 | 1.00 | 15.43 | B | N |
| ATOM | 4221 | CA | SER | B | 242 | 23.940 | 42.088 | 82.366 | 1.00 | 15.51 | B | C |
| ATOM | 4222 | CB | SER | B | 242 | 22.799 | 43.060 | 82.059 | 1.00 | 15.11 | B | C |
| ATOM | 4223 | OG | SER | B | 242 | 22.076 | 42.659 | 80.914 | 1.00 | 14.78 | B | O |
| ATOM | 4224 | C | SER | B | 242 | 24.913 | 42.741 | 83.339 | 1.00 | 17.40 | B | C |
| ATOM | 4225 | O | SER | B | 242 | 25.980 | 43.188 | 82.939 | 1.00 | 18.71 | B | O |
| ATOM | 4226 | N | ALA | B | 243 | 24.539 | 42.787 | 84.616 | 1.00 | 16.36 | B | N |

| | | | | | | | | | | | | |
|---|---|---|---|---|---|---|---|---|---|---|---|---|
| ATOM | 4227 | CA | ALA | B | 243 | 25.404 | 43.381 | 85.628 | 1.00 | 17.00 | B | C |
| ATOM | 4228 | CB | ALA | B | 243 | 24.740 | 43.319 | 86.993 | 1.00 | 15.86 | B | C |
| ATOM | 4229 | C | ALA | B | 243 | 26.740 | 42.636 | 85.655 | 1.00 | 17.11 | B | C |
| ATOM | 4230 | O | ALA | B | 243 | 27.799 | 43.245 | 85.740 | 1.00 | 17.20 | B | O |
| ATOM | 4231 | N | GLY | B | 244 | 26.673 | 41.315 | 85.578 | 1.00 | 17.37 | B | N |
| ATOM | 4232 | CA | GLY | B | 244 | 27.882 | 40.514 | 85.586 | 1.00 | 15.78 | B | C |
| ATOM | 4233 | C | GLY | B | 244 | 28.792 | 40.865 | 84.424 | 1.00 | 18.09 | B | C |
| ATOM | 4234 | O | GLY | B | 244 | 30.007 | 40.862 | 84.570 | 1.00 | 17.96 | B | O |
| ATOM | 4235 | N | CYS | B | 245 | 28.199 | 41.164 | 83.265 | 1.00 | 16.86 | B | N |
| ATOM | 4236 | CA | CYS | B | 245 | 28.978 | 41.534 | 82.091 | 1.00 | 17.34 | B | C |
| ATOM | 4237 | CB | CYS | B | 245 | 28.099 | 41.569 | 80.835 | 1.00 | 16.67 | B | C |
| ATOM | 4238 | SG | CYS | B | 245 | 27.486 | 39.962 | 80.295 | 1.00 | 19.19 | B | S |
| ATOM | 4239 | C | CYS | B | 245 | 29.649 | 42.894 | 82.289 | 1.00 | 15.76 | B | C |
| ATOM | 4240 | O | CYS | B | 245 | 30.722 | 43.128 | 81.779 | 1.00 | 14.91 | B | O |
| ATOM | 4241 | N | VAL | B | 246 | 28.999 | 43.784 | 83.032 | 1.00 | 14.70 | B | N |
| ATOM | 4242 | CA | VAL | B | 246 | 29.565 | 45.099 | 83.297 | 1.00 | 15.45 | B | C |
| ATOM | 4243 | CB | VAL | B | 246 | 28.539 | 46.037 | 83.957 | 1.00 | 15.75 | B | C |
| ATOM | 4244 | CG1 | VAL | B | 246 | 29.224 | 47.314 | 84.439 | 1.00 | 13.36 | B | C |
| ATOM | 4245 | CG2 | VAL | B | 246 | 27.437 | 46.364 | 82.960 | 1.00 | 15.46 | B | C |
| ATOM | 4246 | C | VAL | B | 246 | 30.758 | 44.919 | 84.235 | 1.00 | 17.58 | B | C |
| ATOM | 4247 | O | VAL | B | 246 | 31.832 | 45.479 | 84.001 | 1.00 | 18.48 | B | O |
| ATOM | 4248 | N | LEU | B | 247 | 30.562 | 44.128 | 85.290 | 1.00 | 17.17 | B | N |
| ATOM | 4249 | CA | LEU | B | 247 | 31.625 | 43.863 | 86.256 | 1.00 | 19.91 | B | C |
| ATOM | 4250 | CB | LEU | B | 247 | 31.167 | 42.839 | 87.314 | 1.00 | 19.07 | B | C |
| ATOM | 4251 | CG | LEU | B | 247 | 32.246 | 42.212 | 88.222 | 1.00 | 21.55 | B | C |
| ATOM | 4252 | CD1 | LEU | B | 247 | 32.969 | 43.307 | 89.000 | 1.00 | 19.84 | B | C |
| ATOM | 4253 | CD2 | LEU | B | 247 | 31.613 | 41.199 | 89.194 | 1.00 | 19.90 | B | C |
| ATOM | 4254 | C | LEU | B | 247 | 32.853 | 43.314 | 85.547 | 1.00 | 20.90 | B | C |
| ATOM | 4255 | O | LEU | B | 247 | 33.949 | 43.812 | 85.720 | 1.00 | 20.69 | B | O |
| ATOM | 4256 | N | ALA | B | 248 | 32.641 | 42.272 | 84.753 | 1.00 | 20.76 | B | N |
| ATOM | 4257 | CA | ALA | B | 248 | 33.726 | 41.647 | 84.018 | 1.00 | 21.55 | B | C |
| ATOM | 4258 | CB | ALA | B | 248 | 33.189 | 40.502 | 83.157 | 1.00 | 19.96 | B | C |
| ATOM | 4259 | C | ALA | B | 248 | 34.447 | 42.666 | 83.144 | 1.00 | 21.66 | B | C |
| ATOM | 4260 | O | ALA | B | 248 | 35.671 | 42.704 | 83.122 | 1.00 | 22.29 | B | O |
| ATOM | 4261 | N | GLU | B | 249 | 33.678 | 43.493 | 82.437 | 1.00 | 21.26 | B | N |
| ATOM | 4262 | CA | GLU | B | 249 | 34.245 | 44.503 | 81.545 | 1.00 | 20.43 | B | C |
| ATOM | 4263 | CB | GLU | B | 249 | 33.135 | 45.249 | 80.796 | 1.00 | 20.55 | B | C |
| ATOM | 4264 | CG | GLU | B | 249 | 33.630 | 45.995 | 79.564 | 1.00 | 19.07 | B | C |
| ATOM | 4265 | CD | GLU | B | 249 | 32.510 | 46.508 | 78.670 | 1.00 | 19.83 | B | C |
| ATOM | 4266 | OE1 | GLU | B | 249 | 31.433 | 45.880 | 78.629 | 1.00 | 18.24 | B | O |
| ATOM | 4267 | OE2 | GLU | B | 249 | 32.720 | 47.530 | 77.987 | 1.00 | 20.19 | B | O |
| ATOM | 4268 | C | GLU | B | 249 | 35.120 | 45.497 | 82.312 | 1.00 | 20.65 | B | C |
| ATOM | 4269 | O | GLU | B | 249 | 36.236 | 45.787 | 81.908 | 1.00 | 21.15 | B | O |
| ATOM | 4270 | N | LEU | B | 250 | 34.607 | 46.009 | 83.423 | 1.00 | 20.69 | B | N |
| ATOM | 4271 | CA | LEU | B | 250 | 35.358 | 46.964 | 84.226 | 1.00 | 22.44 | B | C |
| ATOM | 4272 | CB | LEU | B | 250 | 34.510 | 47.464 | 85.400 | 1.00 | 22.33 | B | C |
| ATOM | 4273 | CG | LEU | B | 250 | 33.242 | 48.268 | 85.087 | 1.00 | 22.02 | B | C |
| ATOM | 4274 | CD1 | LEU | B | 250 | 32.577 | 48.684 | 86.392 | 1.00 | 20.39 | B | C |
| ATOM | 4275 | CD2 | LEU | B | 250 | 33.587 | 49.490 | 84.258 | 1.00 | 20.81 | B | C |
| ATOM | 4276 | C | LEU | B | 250 | 36.663 | 46.347 | 84.753 | 1.00 | 23.96 | B | C |
| ATOM | 4277 | O | LEU | B | 250 | 37.628 | 47.052 | 84.976 | 1.00 | 24.30 | B | O |
| ATOM | 4278 | N | LEU | B | 251 | 36.680 | 45.030 | 84.945 | 1.00 | 23.91 | B | N |
| ATOM | 4279 | CA | LEU | B | 251 | 37.878 | 44.356 | 85.430 | 1.00 | 25.82 | B | C |
| ATOM | 4280 | CB | LEU | B | 251 | 37.528 | 43.032 | 86.116 | 1.00 | 25.27 | B | C |
| ATOM | 4281 | CG | LEU | B | 251 | 36.711 | 43.098 | 87.402 | 1.00 | 24.96 | B | C |
| ATOM | 4282 | CD1 | LEU | B | 251 | 36.294 | 41.699 | 87.815 | 1.00 | 24.06 | B | C |
| ATOM | 4283 | CD2 | LEU | B | 251 | 37.531 | 43.769 | 88.491 | 1.00 | 23.69 | B | C |
| ATOM | 4284 | C | LEU | B | 251 | 38.838 | 44.064 | 84.281 | 1.00 | 27.08 | B | C |
| ATOM | 4285 | O | LEU | B | 251 | 40.021 | 44.251 | 84.415 | 1.00 | 28.30 | B | O |
| ATOM | 4286 | N | LEU | B | 252 | 38.299 | 43.611 | 83.151 | 1.00 | 27.15 | B | N |
| ATOM | 4287 | CA | LEU | B | 252 | 39.112 | 43.274 | 81.988 | 1.00 | 26.81 | B | C |
| ATOM | 4288 | CB | LEU | B | 252 | 38.382 | 42.245 | 81.116 | 1.00 | 27.29 | B | C |
| ATOM | 4289 | CG | LEU | B | 252 | 38.452 | 40.770 | 81.543 | 1.00 | 28.26 | B | C |

| | | | | | | | | | | | | |
|---|---|---|---|---|---|---|---|---|---|---|---|---|
| ATOM | 4290 | CD1 | LEU | B | 252 | 37.345 | 39.981 | 80.862 | 1.00 | 28.49 | B | C |
| ATOM | 4291 | CD2 | LEU | B | 252 | 39.812 | 40.191 | 81.202 | 1.00 | 26.61 | B | C |
| ATOM | 4292 | C | LEU | B | 252 | 39.570 | 44.441 | 81.106 | 1.00 | 26.86 | B | C |
| ATOM | 4293 | O | LEU | B | 252 | 40.627 | 44.376 | 80.510 | 1.00 | 25.89 | B | O |
| ATOM | 4294 | N | GLY | B | 253 | 38.774 | 45.505 | 81.024 | 1.00 | 25.72 | B | N |
| ATOM | 4295 | CA | GLY | B | 253 | 39.148 | 46.626 | 80.180 | 1.00 | 23.26 | B | C |
| ATOM | 4296 | C | GLY | B | 253 | 38.495 | 46.539 | 78.809 | 1.00 | 23.33 | B | C |
| ATOM | 4297 | O | GLY | B | 253 | 38.740 | 47.373 | 77.943 | 1.00 | 23.03 | B | O |
| ATOM | 4298 | N | GLN | B | 254 | 37.671 | 45.513 | 78.614 | 1.00 | 22.16 | B | N |
| ATOM | 4299 | CA | GLN | B | 254 | 36.949 | 45.305 | 77.355 | 1.00 | 23.80 | B | C |
| ATOM | 4300 | CB | GLN | B | 254 | 37.870 | 44.698 | 76.289 | 1.00 | 25.24 | B | C |
| ATOM | 4301 | CG | GLN | B | 254 | 38.667 | 43.489 | 76.744 | 1.00 | 29.25 | B | C |
| ATOM | 4302 | CD | GLN | B | 254 | 39.550 | 42.924 | 75.641 | 1.00 | 32.94 | B | C |
| ATOM | 4303 | OE1 | GLN | B | 254 | 39.073 | 42.224 | 74.740 | 1.00 | 34.35 | B | O |
| ATOM | 4304 | NE2 | GLN | B | 254 | 40.845 | 43.230 | 75.703 | 1.00 | 33.94 | B | N |
| ATOM | 4305 | C | GLN | B | 254 | 35.766 | 44.376 | 77.606 | 1.00 | 22.34 | B | C |
| ATOM | 4306 | O | GLN | B | 254 | 35.707 | 43.727 | 78.623 | 1.00 | 23.11 | B | O |
| ATOM | 4307 | N | PRO | B | 255 | 34.807 | 44.312 | 76.673 | 1.00 | 20.37 | B | N |
| ATOM | 4308 | CD | PRO | B | 255 | 34.689 | 45.040 | 75.402 | 1.00 | 19.12 | B | C |
| ATOM | 4309 | CA | PRO | B | 255 | 33.653 | 43.427 | 76.882 | 1.00 | 19.33 | B | C |
| ATOM | 4310 | CB | PRO | B | 255 | 32.814 | 43.649 | 75.623 | 1.00 | 19.07 | B | C |
| ATOM | 4311 | CG | PRO | B | 255 | 33.201 | 45.029 | 75.185 | 1.00 | 20.39 | B | C |
| ATOM | 4312 | C | PRO | B | 255 | 34.116 | 41.970 | 77.020 | 1.00 | 19.64 | B | C |
| ATOM | 4313 | O | PRO | B | 255 | 35.065 | 41.553 | 76.362 | 1.00 | 18.39 | B | O |
| ATOM | 4314 | N | ILE | B | 256 | 33.444 | 41.204 | 77.876 | 1.00 | 19.48 | B | N |
| ATOM | 4315 | CA | ILE | B | 256 | 33.804 | 39.814 | 78.087 | 1.00 | 19.22 | B | C |
| ATOM | 4316 | CB | ILE | B | 256 | 33.383 | 39.317 | 79.491 | 1.00 | 21.34 | B | C |
| ATOM | 4317 | CG2 | ILE | B | 256 | 31.858 | 39.376 | 79.639 | 1.00 | 19.53 | B | C |
| ATOM | 4318 | CG1 | ILE | B | 256 | 33.911 | 37.890 | 79.710 | 1.00 | 20.79 | B | C |
| ATOM | 4319 | CD1 | ILE | B | 256 | 33.831 | 37.401 | 81.148 | 1.00 | 18.89 | B | C |
| ATOM | 4320 | C | ILE | B | 256 | 33.195 | 38.888 | 77.033 | 1.00 | 20.64 | B | C |
| ATOM | 4321 | O | ILE | B | 256 | 33.785 | 37.885 | 76.680 | 1.00 | 19.92 | B | O |
| ATOM | 4322 | N | PHE | B | 257 | 32.016 | 39.238 | 76.528 | 1.00 | 20.72 | B | N |
| ATOM | 4323 | CA | PHE | B | 257 | 31.355 | 38.418 | 75.515 | 1.00 | 19.92 | B | C |
| ATOM | 4324 | CB | PHE | B | 257 | 30.100 | 37.756 | 76.096 | 1.00 | 18.00 | B | C |
| ATOM | 4325 | CG | PHE | B | 257 | 30.368 | 36.885 | 77.291 | 1.00 | 19.45 | B | C |
| ATOM | 4326 | CD1 | PHE | B | 257 | 31.380 | 35.926 | 77.262 | 1.00 | 20.42 | B | C |
| ATOM | 4327 | CD2 | PHE | B | 257 | 29.602 | 37.012 | 78.446 | 1.00 | 19.96 | B | C |
| ATOM | 4328 | CE1 | PHE | B | 257 | 31.625 | 35.111 | 78.365 | 1.00 | 20.59 | B | C |
| ATOM | 4329 | CE2 | PHE | B | 257 | 29.839 | 36.201 | 79.551 | 1.00 | 20.07 | B | C |
| ATOM | 4330 | CZ | PHE | B | 257 | 30.855 | 35.247 | 79.513 | 1.00 | 19.52 | B | C |
| ATOM | 4331 | C | PHE | B | 257 | 30.977 | 39.228 | 74.276 | 1.00 | 20.28 | B | C |
| ATOM | 4332 | O | PHE | B | 257 | 29.822 | 39.516 | 74.053 | 1.00 | 20.93 | B | O |
| ATOM | 4333 | N | PRO | B | 258 | 31.970 | 39.610 | 73.462 | 1.00 | 20.75 | B | N |
| ATOM | 4334 | CD | PRO | B | 258 | 33.417 | 39.468 | 73.710 | 1.00 | 19.68 | B | C |
| ATOM | 4335 | CA | PRO | B | 258 | 31.728 | 40.388 | 72.244 | 1.00 | 19.66 | B | C |
| ATOM | 4336 | CB | PRO | B | 258 | 33.101 | 40.978 | 71.936 | 1.00 | 19.46 | B | C |
| ATOM | 4337 | CG | PRO | B | 258 | 34.023 | 39.888 | 72.376 | 1.00 | 18.19 | B | C |
| ATOM | 4338 | C | PRO | B | 258 | 31.215 | 39.519 | 71.112 | 1.00 | 20.92 | B | C |
| ATOM | 4339 | O | PRO | B | 258 | 31.140 | 38.316 | 71.245 | 1.00 | 23.05 | B | O |
| ATOM | 4340 | N | GLY | B | 259 | 30.876 | 40.149 | 69.994 | 1.00 | 21.99 | B | N |
| ATOM | 4341 | CA | GLY | B | 259 | 30.372 | 39.415 | 68.851 | 1.00 | 22.30 | B | C |
| ATOM | 4342 | C | GLY | B | 259 | 29.268 | 40.201 | 68.180 | 1.00 | 24.26 | B | C |
| ATOM | 4343 | O | GLY | B | 259 | 28.428 | 40.783 | 68.850 | 1.00 | 24.30 | B | O |
| ATOM | 4344 | N | ASP | B | 260 | 29.267 | 40.213 | 66.854 | 1.00 | 23.77 | B | N |
| ATOM | 4345 | CA | ASP | B | 260 | 28.261 | 40.957 | 66.114 | 1.00 | 26.22 | B | C |
| ATOM | 4346 | CB | ASP | B | 260 | 28.875 | 41.513 | 64.829 | 1.00 | 30.10 | B | C |
| ATOM | 4347 | CG | ASP | B | 260 | 30.100 | 42.380 | 65.100 | 1.00 | 33.70 | B | C |
| ATOM | 4348 | OD1 | ASP | B | 260 | 30.284 | 42.811 | 66.267 | 1.00 | 33.87 | B | O |
| ATOM | 4349 | OD2 | ASP | B | 260 | 30.869 | 42.637 | 64.146 | 1.00 | 36.21 | B | O |
| ATOM | 4350 | C | ASP | B | 260 | 27.008 | 40.149 | 65.796 | 1.00 | 24.83 | B | C |
| ATOM | 4351 | O | ASP | B | 260 | 26.092 | 40.645 | 65.162 | 1.00 | 26.33 | B | O |
| ATOM | 4352 | N | SER | B | 261 | 26.980 | 38.904 | 66.250 | 1.00 | 24.08 | B | N |

| | | | | | | | | | | | | |
|---|---|---|---|---|---|---|---|---|---|---|---|---|
| ATOM | 4353 | CA | SER | B | 261 | 25.834 | 38.038 | 66.027 | 1.00 | 21.60 | B | C |
| ATOM | 4354 | CB | SER | B | 261 | 26.116 | 37.050 | 64.894 | 1.00 | 21.44 | B | C |
| ATOM | 4355 | OG | SER | B | 261 | 27.163 | 36.165 | 65.241 | 1.00 | 22.83 | B | O |
| ATOM | 4356 | C | SER | B | 261 | 25.566 | 37.274 | 67.312 | 1.00 | 21.91 | B | C |
| ATOM | 4357 | O | SER | B | 261 | 26.447 | 37.174 | 68.184 | 1.00 | 19.20 | B | O |
| ATOM | 4358 | N | GLY | B | 262 | 24.351 | 36.746 | 67.425 | 1.00 | 20.02 | B | N |
| ATOM | 4359 | CA | GLY | B | 262 | 23.991 | 35.970 | 68.593 | 1.00 | 21.70 | B | C |
| ATOM | 4360 | C | GLY | B | 262 | 24.868 | 34.734 | 68.685 | 1.00 | 21.26 | B | C |
| ATOM | 4361 | O | GLY | B | 262 | 25.333 | 34.382 | 69.754 | 1.00 | 20.17 | B | O |
| ATOM | 4362 | N | VAL | B | 263 | 25.096 | 34.083 | 67.548 | 1.00 | 21.98 | B | N |
| ATOM | 4363 | CA | VAL | B | 263 | 25.938 | 32.890 | 67.523 | 1.00 | 23.15 | B | C |
| ATOM | 4364 | CB | VAL | B | 263 | 26.001 | 32.246 | 66.101 | 1.00 | 22.75 | B | C |
| ATOM | 4365 | CG1 | VAL | B | 263 | 24.630 | 31.795 | 65.686 | 1.00 | 24.44 | B | C |
| ATOM | 4366 | CG2 | VAL | B | 263 | 26.553 | 33.234 | 65.090 | 1.00 | 22.70 | B | C |
| ATOM | 4367 | C | VAL | B | 263 | 27.358 | 33.215 | 67.988 | 1.00 | 22.06 | B | C |
| ATOM | 4368 | O | VAL | B | 263 | 27.918 | 32.491 | 68.771 | 1.00 | 21.55 | B | O |
| ATOM | 4369 | N | ASP | B | 264 | 27.920 | 34.317 | 67.502 | 1.00 | 22.01 | B | N |
| ATOM | 4370 | CA | ASP | B | 264 | 29.272 | 34.705 | 67.893 | 1.00 | 23.09 | B | C |
| ATOM | 4371 | CB | ASP | B | 264 | 29.745 | 35.914 | 67.058 | 1.00 | 22.78 | B | C |
| ATOM | 4372 | CG | ASP | B | 264 | 30.148 | 35.534 | 65.630 | 1.00 | 24.32 | B | C |
| ATOM. | 4373 | OD1 | ASP | B | 264 | 30.265 | 34.327 | 65.334 | 1.00 | 25.53 | B | O |
| ATOM | 4374 | OD2 | ASP | B | 264 | 30.370 | 36.444 | 64.803 | 1.00 | 24.79 | B | O |
| ATOM | 4375 | C | ASP | B | 264 | 29.347 | 35.030 | 69.386 | 1.00 | 22.89 | B | C |
| ATOM | 4376 | O | ASP | B | 264 | 30.338 | 34.756 | 70.032 | 1.00 | 24.33 | B | O |
| ATOM | 4377 | N | GLN | B | 265 | 28.283 | 35.619 | 69.921 | 1.00 | 23.27 | B | N |
| ATOM | 4378 | CA | GLN | B | 265 | 28.219 | 35.954 | 71.338 | 1.00 | 23.07 | B | C |
| ATOM | 4379 | CB | GLN | B | 265 | 26.981 | 36.814 | 71.609 | 1.00 | 22.60 | B | C |
| ATOM | 4380 | CG | GLN | B | 265 | 27.035 | 38.159 | 70.916 | 1.00 | 24.03 | B | C |
| ATOM | 4381 | CD | GLN | B | 265 | 25.687 | 38.855 | 70.841 | 1.00 | 25.26 | B | C |
| ATOM | 4382 | OE1 | GLN | B | 265 | 25.521 | 39.792 | 70.090 | 1.00 | 26.91 | B | O |
| ATOM | 4383 | NE2 | GLN | B | 265 | 24.726 | 38.392 | 71.626 | 1.00 | 25.36 | B | N |
| ATOM | 4384 | C | GLN | B | 265 | 28.161 | 34.665 | 72.152 | 1.00 | 21.83 | B | C |
| ATOM | 4385 | O | GLN | B | 265 | 28.792 | 34.555 | 73.180 | 1.00 | 20.92 | B | O |
| ATOM | 4386 | N | LEU | B | 266 | 27.393 | 33.699 | 71.656 | 1.00 | 21.65 | B | N |
| ATOM | 4387 | CA | LEU | B | 266 | 27.252 | 32.408 | 72.311 | 1.00 | 23.72 | B | C |
| ATOM | 4388 | CB | LEU | B | 266 | 26.204 | 31.561 | 71.589 | 1.00 | 24.37 | B | C |
| ATOM | 4389 | CG | LEU | B | 266 | 24.827 | 31.443 | 72.251 | 1.00 | 28.13 | B | C |
| ATOM | 4390 | CD1 | LEU | B | 266 | 24.346 | 32.801 | 72.661 | 1.00 | 29.34 | B | C |
| ATOM | 4391 | CD2 | LEU | B | 266 | 23.832 | 30.780 | 71.294 | 1.00 | 26.64 | B | C |
| ATOM | 4392 | C | LEU | B | 266 | 28.577 | 31.658 | 72.357 | 1.00 | 24.07 | B | C |
| ATOM | 4393 | O | LEU | B | 266 | 28.915 | 31.086 | 73.371 | 1.00 | 24.14 | B | O |
| ATOM | 4394 | N | VAL | B | 267 | 29.331 | 31.658 | 71.262 | 1.00 | 24.46 | B | N |
| ATOM | 4395 | CA | VAL | B | 267 | 30.596 | 30.943 | 71.298 | 1.00 | 25.76 | B | C |
| ATOM | 4396 | CB | VAL | B | 267 | 31.272 | 30.855 | 69.898 | 1.00 | 25.92 | B | C |
| ATOM | 4397 | CG1 | VAL | B | 267 | 30.346 | 30.138 | 68.923 | 1.00 | 23.57 | B | C |
| ATOM | 4398 | CG2 | VAL | B | 267 | 31.633 | 32.232 | 69.391 | 1.00 | 29.47 | B | C |
| ATOM | 4399 | C | VAL | B | 267 | 31.526 | 31.610 | 72.300 | 1.00 | 26.25 | B | C |
| ATOM | 4400 | O | VAL | B | 267 | 32.235 | 30.942 | 73.041 | 1.00 | 26.95 | B | O |
| ATOM | 4401 | N | GLU | B | 268 | 31.496 | 32.935 | 72.336 | 1.00 | 26.39 | B | N |
| ATOM | 4402 | CA | GLU | B | 268 | 32.335 | 33.678 | 73.262 | 1.00 | 26.36 | B | C |
| ATOM | 4403 | CB | GLU | B | 268 | 32.116 | 35.176 | 73.036 | 1.00 | 28.65 | B | C |
| ATOM | 4404 | CG | GLU | B | 268 | 33.324 | 36.024 | 73.339 | 1.00 | 32.25 | B | C |
| ATOM | 4405 | CD | GLU | B | 268 | 34.589 | 35.505 | 72.666 | 1.00 | 32.96 | B | C |
| ATOM | 4406 | OE1 | GLU | B | 268 | 34.691 | 35.570 | 71.422 | 1.00 | 31.50 | B | O |
| ATOM | 4407 | OE2 | GLU | B | 268 | 35.478 | 35.024 | 73.397 | 1.00 | 34.30 | B | O |
| ATOM | 4408 | C | GLU | B | 268 | 31.997 | 33.274 | 74.699 | 1.00 | 25.39 | B | C |
| ATOM | 4409 | O | GLU | B | 268 | 32.885 | 33.097 | 75.520 | 1.00 | 24.97 | B | O |
| ATOM | 4410 | N | ILE | B | 269 | 30.705 | 33.115 | 74.977 | 1.00 | 24.06 | B | N |
| ATOM | 4411 | CA | ILE | B | 269 | 30.227 | 32.715 | 76.296 | 1.00 | 24.37 | B | C |
| ATOM | 4412 | CB | ILE | B | 269 | 28.688 | 32.838 | 76.377 | 1.00 | 22.90 | B | C |
| ATOM | 4413 | CG2 | ILE | B | 269 | 28.168 | 32.176 | 77.637 | 1.00 | 21.09 | B | C |
| ATOM | 4414 | CG1 | ILE | B | 269 | 28.283 | 34.313 | 76.320 | 1.00 | 24.81 | B | C |
| ATOM | 4415 | CD1 | ILE | B | 269 | 26.802 | 34.535 | 76.088 | 1.00 | 22.49 | B | C |

| | | | | | | | | | | | |
|---|---|---|---|---|---|---|---|---|---|---|---|
| ATOM | 4416 | C | ILE | B | 269 | 30.632 | 31.267 | 76.551 | 1.00 | 25.86 | B | C |
| ATOM | 4417 | O | ILE | B | 269 | 31.028 | 30.920 | 77.648 | 1.00 | 27.37 | B | O |
| ATOM | 4418 | N | ILE | B | 270 | 30.528 | 30.432 | 75.520 | 1.00 | 25.52 | B | N |
| ATOM | 4419 | CA | ILE | B | 270 | 30.894 | 29.029 | 75.639 | 1.00 | 25.98 | B | C |
| ATOM | 4420 | CB | ILE | B | 270 | 30.465 | 28.245 | 74.368 | 1.00 | 25.08 | B | C |
| ATOM | 4421 | CG2 | ILE | B | 270 | 31.104 | 26.873 | 74.344 | 1.00 | 25.73 | B | C |
| ATOM | 4422 | CG1 | ILE | B | 270 | 28.943 | 28.110 | 74.340 | 1.00 | 25.31 | B | C |
| ATOM | 4423 | CD1 | ILE | B | 270 | 28.395 | 27.549 | 73.043 | 1.00 | 26.10 | B | C |
| ATOM | 4424 | C | ILE | B | 270 | 32.395 | 28.857 | 75.891 | 1.00 | 26.52 | B | C |
| ATOM | 4425 | O | ILE | B | 270 | 32.790 | 27.954 | 76.610 | 1.00 | 26.79 | B | O |
| ATOM | 4426 | N | LYS | B | 271 | 33.222 | 29.726 | 75.310 | 1.00 | 26.31 | B | N |
| ATOM | 4427 | CA | LYS | B | 271 | 34.665 | 29.621 | 75.518 | 1.00 | 28.20 | B | C |
| ATOM | 4428 | CB | LYS | B | 271 | 35.436 | 30.544 | 74.566 | 1.00 | 27.64 | B | C |
| ATOM | 4429 | CG | LYS | B | 271 | 35.196 | 30.233 | 73.090 | 1.00 | 30.55 | B | C |
| ATOM | 4430 | CD | LYS | B | 271 | 36.406 | 30.548 | 72.218 | 1.00 | 31.64 | B | C |
| ATOM | 4431 | CE | LYS | B | 271 | 36.830 | 31.987 | 72.313 | 1.00 | 34.01 | B | C |
| ATOM | 4432 | NZ | LYS | B | 271 | 37.948 | 32.242 | 71.381 | 1.00 | 36.53 | B | N |
| ATOM | 4433 | C | LYS | B | 271 | 35.071 | 29.898 | 76.973 | 1.00 | 30.02 | B | C |
| ATOM | 4434 | O | LYS | B | 271 | 36.229 | 29.680 | 77.354 | 1.00 | 30.55 | B | O |
| ATOM | 4435 | N | VAL | B | 272 | 34.142 | 30.375 | 77.797 | 1.00 | 29.13 | B | N |
| ATOM | 4436 | CA | VAL | B | 272 | 34.502 | 30.598 | 79.191 | 1.00 | 29.42 | B | C |
| ATOM | 4437 | CB | VAL | B | 272 | 34.355 | 32.083 | 79.614 | 1.00 | 29.11 | B | C |
| ATOM | 4438 | CG1 | VAL | B | 272 | 35.152 | 32.975 | 78.674 | 1.00 | 26.93 | B | C |
| ATOM | 4439 | CG2 | VAL | B | 272 | 32.905 | 32.476 | 79.645 | 1.00 | 32.30 | B | C |
| ATOM | 4440 | C | VAL | B | 272 | 33.697 | 29.712 | 80.142 | 1.00 | 28.77 | B | C |
| ATOM | 4441 | O | VAL | B | 272 | 34.260 | 29.139 | 81.053 | 1.00 | 28.18 | B | O |
| ATOM | 4442 | N | LEU | B | 273 | 32.390 | 29.582 | 79.913 | 1.00 | 28.62 | B | N |
| ATOM | 4443 | CA | LEU | B | 273 | 31.546 | 28.763 | 80.789 | 1.00 | 28.69 | B | C |
| ATOM | 4444 | CB | LEU | B | 273 | 30.111 | 29.280 | 80.805 | 1.00 | 28.90 | B | C |
| ATOM | 4445 | CG | LEU | B | 273 | 29.833 | 30.615 | 81.488 | 1.00 | 30.45 | B | C |
| ATOM | 4446 | CD1 | LEU | B | 273 | 28.330 | 30.886 | 81.471 | 1.00 | 28.67 | B | C |
| ATOM | 4447 | CD2 | LEU | B | 273 | 30.350 | 30.569 | 82.913 | 1.00 | 30.96 | B | C |
| ATOM | 4448 | C | LEU | B | 273 | 31.492 | 27.305 | 80.374 | 1.00 | 29.35 | B | C |
| ATOM | 4449 | O | LEU | B | 273 | 31.008 | 26.454 | 81.119 | 1.00 | 29.33 | B | O |
| ATOM | 4450 | N | GLY | B | 274 | 31.984 | 27.019 | 79.177 | 1.00 | 29.45 | B | N |
| ATOM | 4451 | CA | GLY | B | 274 | 31.936 | 25.659 | 78.689 | 1.00 | 29.13 | B | C |
| ATOM | 4452 | C | GLY | B | 274 | 30.610 | 25.476 | 77.978 | 1.00 | 30.27 | B | C |
| ATOM | 4453 | O | GLY | B | 274 | 29.718 | 26.307 | 78.121 | 1.00 | 29.19 | B | O |
| ATOM | 4454 | N | THR | B | 275 | 30.477 | 24.404 | 77.203 | 1.00 | 30.51 | B | N |
| ATOM | 4455 | CA | THR | B | 275 | 29.232 | 24.147 | 76.490 | 1.00 | 30.90 | B | C |
| ATOM | 4456 | CB | THR | B | 275 | 29.342 | 22.890 | 75.556 | 1.00 | 31.79 | B | C |
| ATOM | 4457 | OG1 | THR | B | 275 | 30.536 | 22.964 | 74.765 | 1.00 | 31.66 | B | O |
| ATOM | 4458 | CG2 | THR | B | 275 | 28.141 | 22.808 | 74.617 | 1.00 | 30.52 | B | C |
| ATOM | 4459 | C | THR | B | 275 | 28.142 | 23.872 | 77.523 | 1.00 | 32.08 | B | C |
| ATOM | 4460 | O | THR | B | 275 | 28.368 | 23.151 | 78.493 | 1.00 | 32.20 | B | O |
| ATOM | 4461 | N | PRO | B | 276 | 26.943 | 24.438 | 77.327 | 1.00 | 32.69 | B | N |
| ATOM | 4462 | CD | PRO | B | 276 | 26.514 | 25.351 | 76.252 | 1.00 | 32.13 | B | C |
| ATOM | 4463 | CA | PRO | B | 276 | 25.855 | 24.215 | 78.279 | 1.00 | 33.62 | B | C |
| ATOM | 4464 | CB | PRO | B | 276 | 24.860 | 25.306 | 77.908 | 1.00 | 32.88 | B | C |
| ATOM | 4465 | CG | PRO | B | 276 | 25.007 | 25.369 | 76.429 | 1.00 | 32.61 | B | C |
| ATOM | 4466 | C | PRO | B | 276 | 25.248 | 22.816 | 78.163 | 1.00 | 34.94 | B | C |
| ATOM | 4467 | O | PRO | B | 276 | 25.032 | 22.325 | 77.068 | 1.00 | 36.62 | B | O |
| ATOM | 4468 | N | THR | B | 277 | 24.967 | 22.192 | 79.304 | 1.00 | 35.21 | B | N |
| ATOM | 4469 | CA | THR | B | 277 | 24.381 | 20.857 | 79.329 | 1.00 | 35.04 | B | C |
| ATOM | 4470 | CB | THR | B | 277 | 24.405 | 20.261 | 80.741 | 1.00 | 33.91 | B | C |
| ATOM | 4471 | OG1 | THR | B | 277 | 23.457 | 20.948 | 81.568 | 1.00 | 33.49 | B | O |
| ATOM | 4472 | CG2 | THR | B | 277 | 25.789 | 20.401 | 81.346 | 1.00 | 33.85 | B | C |
| ATOM | 4473 | C | THR | B | 277 | 22.928 | 20.920 | 78.866 | 1.00 | 36.68 | B | C |
| ATOM | 4474 | O | THR | B | 277 | 22.288 | 21.972 | 78.929 | 1.00 | 36.55 | B | O |
| ATOM | 4475 | N | ALA | B | 278 | 22.399 | 19.789 | 78.409 | 1.00 | 37.71 | B | N |
| ATOM | 4476 | CA | ALA | B | 278 | 21.020 | 19.762 | 77.941 | 1.00 | 38.44 | B | C |
| ATOM | 4477 | CB | ALA | B | 278 | 20.625 | 18.341 | 77.529 | 1.00 | 38.44 | B | C |
| ATOM | 4478 | C | ALA | B | 278 | 20.062 | 20.301 | 79.005 | 1.00 | 38.62 | B | C |

| | | | | | | | | | | | | |
|---|---|---|---|---|---|---|---|---|---|---|---|---|
| ATOM | 4479 | O | ALA | B | 278 | 19.017 | 20.871 | 78.678 | 1.00 | 39.10 | B | O |
| ATOM | 4480 | N | ALA | B | 279 | 20.410 | 20.132 | 80.278 | 1.00 | 39.05 | B | N |
| ATOM | 4481 | CA | ALA | B | 279 | 19.551 | 20.632 | 81.352 | 1.00 | 39.31 | B | C |
| ATOM | 4482 | CB | ALA | B | 279 | 20.028 | 20.103 | 82.719 | 1.00 | 38.66 | B | C |
| ATOM | 4483 | C | ALA | B | 279 | 19.565 | 22.164 | 81.345 | 1.00 | 38.98 | B | C |
| ATOM | 4484 | O | ALA | B | 279 | 18.535 | 22.803 | 81.468 | 1.00 | 39.28 | B | O |
| ATOM | 4485 | N | GLN | B | 280 | 20.756 | 22.733 | 81.192 | 1.00 | 38.64 | B | N |
| ATOM | 4486 | CA | GLN | B | 280 | 20.915 | 24.177 | 81.175 | 1.00 | 37.09 | B | C |
| ATOM | 4487 | CB | GLN | B | 280 | 22.397 | 24.513 | 81.171 | 1.00 | 35.48 | B | C |
| ATOM | 4488 | CG | GLN | B | 280 | 23.036 | 24.280 | 82.526 | 1.00 | 33.98 | B | C |
| ATOM | 4489 | CD | GLN | B | 280 | 24.546 | 24.303 | 82.473 | 1.00 | 32.84 | B | C |
| ATOM | 4490 | OE1 | GLN | B | 280 | 25.200 | 24.617 | 83.462 | 1.00 | 33.03 | B | O |
| ATOM | 4491 | NE2 | GLN | B | 280 | 25.110 | 23.952 | 81.319 | 1.00 | 28.32 | B | N |
| ATOM | 4492 | C | GLN | B | 280 | 20.202 | 24.863 | 80.016 | 1.00 | 37.75 | B | C |
| ATOM | 4493 | O | GLN | B | 280 | 19.532 | 25.866 | 80.213 | 1.00 | 37.93 | B | O |
| ATOM | 4494 | N | ILE | B | 281 | 20.344 | 24.329 | 78.808 | 1.00 | 38.44 | B | N |
| ATOM | 4495 | CA | ILE | B | 281 | 19.668 | 24.926 | 77.661 | 1.00 | 39.91 | B | C |
| ATOM | 4496 | CB | ILE | B | 281 | 19.962 | 24.133 | 76.383 | 1.00 | 41.83 | B | C |
| ATOM | 4497 | CG2 | ILE | B | 281 | 19.641 | 22.682 | 76.616 | 1.00 | 43.81 | B | C |
| ATOM | 4498 | CG1 | ILE | B | 281 | 19.129 | 24.659 | 75.207 | 1.00 | 44.43 | B | C |
| ATOM | 4499 | CD1 | ILE | B | 281 | 19.639 | 25.976 | 74.594 | 1.00 | 46.50 | B | C |
| ATOM | 4500 | C | ILE | B | 281 | 18.172 | 24.867 | 77.961 | 1.00 | 39.77 | B | C |
| ATOM | 4501 | O | ILE | B | 281 | 17.397 | 25.741 | 77.555 | 1.00 | 39.64 | B | O |
| ATOM | 4502 | N | ALA | B | 282 | 17.782 | 23.835 | 78.703 | 1.00 | 39.75 | B | N |
| ATOM | 4503 | CA | ALA | B | 282 | 16.389 | 23.625 | 79.073 | 1.00 | 39.37 | B | C |
| ATOM | 4504 | CB | ALA | B | 282 | 16.211 | 22.215 | 79.653 | 1.00 | 40.34 | B | C |
| ATOM | 4505 | C | ALA | B | 282 | 15.892 | 24.670 | 80.074 | 1.00 | 39.67 | B | C |
| ATOM | 4506 | O | ALA | B | 282 | 14.799 | 25.219 | 79.922 | 1.00 | 37.97 | B | O |
| ATOM | 4507 | N | GLU | B | 283 | 16.683 | 24.931 | 81.107 | 1.00 | 39.94 | B | N |
| ATOM | 4508 | CA | GLU | B | 283 | 16.287 | 25.924 | 82.099 | 1.00 | 40.48 | B | C |
| ATOM | 4509 | CB | GLU | B | 283 | 17.316 | 25.968 | 83.236 | 1.00 | 42.08 | B | C |
| ATOM | 4510 | CG | GLU | B | 283 | 17.727 | 24.565 | 83.724 | 1.00 | 45.84 | B | C |
| ATOM | 4511 | CD | GLU | B | 283 | 18.029 | 24.505 | 85.208 | 1.00 | 48.33 | B | C |
| ATOM | 4512 | OE1 | GLU | B | 283 | 17.108 | 24.816 | 86.008 | 1.00 | 49.27 | B | O |
| ATOM | 4513 | OE2 | GLU | B | 283 | 19.180 | 24.141 | 85.575 | 1.00 | 49.66 | B | O |
| ATOM | 4514 | C | GLU | B | 283 | 16.160 | 27.285 | 81.399 | 1.00 | 39.29 | B | C |
| ATOM | 4515 | O | GLU | B | 283 | 15.337 | 28.117 | 81.780 | 1.00 | 39.33 | B | O |
| ATOM | 4516 | N | MET | B | 284 | 16.961 | 27.485 | 80.355 | 1.00 | 37.42 | B | N |
| ATOM | 4517 | CA | MET | B | 284 | 16.932 | 28.724 | 79.586 | 1.00 | 37.52 | B | C |
| ATOM | 4518 | CB | MET | B | 284 | 18.265 | 28.953 | 78.866 | 1.00 | 36.91 | B | C |
| ATOM | 4519 | CG | MET | B | 284 | 19.546 | 28.822 | 79.673 | 1.00 | 37.86 | B | C |
| ATOM | 4520 | SD | MET | B | 284 | 21.027 | 29.115 | 78.617 | 1.00 | 37.58 | B | S |
| ATOM | 4521 | CE | MET | B | 284 | 22.023 | 29.988 | 79.695 | 1.00 | 40.12 | B | C |
| ATOM | 4522 | C | MET | B | 284 | 15.866 | 28.643 | 78.497 | 1.00 | 37.61 | B | C |
| ATOM | 4523 | O | MET | B | 284 | 15.405 | 29.680 | 77.978 | 1.00 | 36.17 | B | O |
| ATOM | 4524 | N | ASN | B | 285 | 15.484 | 27.408 | 78.171 | 1.00 | 38.35 | B | N |
| ATOM | 4525 | CA | ASN | B | 285 | 14.560 | 27.080 | 77.078 | 1.00 | 40.83 | B | C |
| ATOM | 4526 | CB | ASN | B | 285 | 13.185 | 26.586 | 77.586 | 1.00 | 42.17 | B | C |
| ATOM | 4527 | CG | ASN | B | 285 | 12.283 | 27.694 | 78.012 | 1.00 | 43.04 | B | C |
| ATOM | 4528 | OD1 | ASN | B | 285 | 12.727 | 28.657 | 78.632 | 1.00 | 48.16 | B | O |
| ATOM | 4529 | ND2 | ASN | B | 285 | 10.994 | 27.569 | 77.695 | 1.00 | 40.21 | B | N |
| ATOM | 4530 | C | ASN | B | 285 | 14.381 | 28.131 | 75.988 | 1.00 | 40.48 | B | C |
| ATOM | 4531 | O | ASN | B | 285 | 13.656 | 29.093 | 76.146 | 1.00 | 40.15 | B | O |
| ATOM | 4532 | N | PRO | B | 286 | 15.069 | 27.939 | 74.850 | 1.00 | 41.48 | B | N |
| ATOM | 4533 | CD | PRO | B | 286 | 16.051 | 26.862 | 74.605 | 1.00 | 42.10 | B | C |
| ATOM | 4534 | CA | PRO | B | 286 | 15.007 | 28.849 | 73.702 | 1.00 | 42.55 | B | C |
| ATOM | 4535 | CB | PRO | B | 286 | 16.277 | 28.507 | 72.929 | 1.00 | 42.83 | B | C |
| ATOM | 4536 | CG | PRO | B | 286 | 16.372 | 27.033 | 73.117 | 1.00 | 42.44 | B | C |
| ATOM | 4537 | C | PRO | B | 286 | 13.775 | 28.571 | 72.862 | 1.00 | 42.88 | B | C |
| ATOM | 4538 | O | PRO | B | 286 | 13.035 | 27.635 | 73.136 | 1.00 | 43.24 | B | O |
| ATOM | 4539 | N | ASN | B | 287 | 13.566 | 29.384 | 71.827 | 1.00 | 43.95 | B | N |
| ATOM | 4540 | CA | ASN | B | 287 | 12.443 | 29.124 | 70.940 | 1.00 | 44.04 | B | C |
| ATOM | 4541 | CB | ASN | B | 287 | 12.256 | 30.236 | 69.900 | 1.00 | 43.89 | B | C |

| | | | | | | | | | | | | |
|---|---|---|---|---|---|---|---|---|---|---|---|---|
| ATOM | 4542 | CG | ASN | B | 287 | 13.570 | 30.837 | 69.418 | 1.00 | 44.76 | B | C |
| ATOM | 4543 | OD1 | ASN | B | 287 | 14.619 | 30.170 | 69.382 | 1.00 | 44.79 | B | O |
| ATOM | 4544 | ND2 | ASN | B | 287 | 13.511 | 32.105 | 69.004 | 1.00 | 43.67 | B | N |
| ATOM | 4545 | C | ASN | B | 287 | 12.872 | 27.832 | 70.259 | 1.00 | 44.63 | B | C |
| ATOM | 4546 | O | ASN | B | 287 | 12.085 | 26.891 | 70.117 | 1.00 | 45.43 | B | O |
| ATOM | 4547 | N | TYR | B | 288 | 14.152 | 27.774 | 69.897 | 1.00 | 44.76 | B | N |
| ATOM | 4548 | CA | TYR | B | 288 | 14.710 | 26.604 | 69.241 | 1.00 | 45.03 | B | C |
| ATOM | 4549 | CB | TYR | B | 288 | 14.115 | 26.463 | 67.841 | 1.00 | 44.88 | B | C |
| ATOM | 4550 | CG | TYR | B | 288 | 14.765 | 25.383 | 67.004 | 1.00 | 46.28 | B | C |
| ATOM | 4551 | CD1 | TYR | B | 288 | 15.492 | 25.706 | 65.860 | 1.00 | 45.84 | B | C |
| ATOM | 4552 | CE1 | TYR | B | 288 | 16.109 | 24.720 | 65.103 | 1.00 | 48.08 | B | C |
| ATOM | 4553 | CD2 | TYR | B | 288 | 14.668 | 24.038 | 67.374 | 1.00 | 46.98 | B | C |
| ATOM | 4554 | CE2 | TYR | B | 288 | 15.279 | 23.035 | 66.628 | 1.00 | 47.94 | B | C |
| ATOM | 4555 | CZ | TYR | B | 288 | 15.998 | 23.383 | 65.491 | 1.00 | 49.21 | B | C |
| ATOM | 4556 | OH | TYR | B | 288 | 16.606 | 22.402 | 64.741 | 1.00 | 49.87 | B | O |
| ATOM | 4557 | C | TYR | B | 288 | 16.221 | 26.705 | 69.135 | 1.00 | 45.52 | B | C |
| ATOM | 4558 | O | TYR | B | 288 | 16.785 | 27.793 | 69.013 | 1.00 | 46.00 | B | O |
| ATOM | 4559 | N | ALA | B | 289 | 16.876 | 25.557 | 69.190 | 1.00 | 46.29 | B | N |
| ATOM | 4560 | CA | ALA | B | 289 | 18.326 | 25.508 | 69.069 | 1.00 | 47.83 | B | C |
| ATOM | 4561 | CB | ALA | B | 289 | 19.000 | 25.805 | 70.413 | 1.00 | 47.95 | B | C |
| ATOM | 4562 | C | ALA | B | 289 | 18.717 | 24.121 | 68.605 | 1.00 | 47.44 | B | C |
| ATOM | 4563 | O | ALA | B | 289 | 18.054 | 23.147 | 68.928 | 1.00 | 47.57 | B | O |
| ATOM | 4564 | N | GLU | B | 290 | 19.774 | 24.045 | 67.809 | 1.00 | 47.36 | B | N |
| ATOM | 4565 | CA | GLU | B | 290 | 20.271 | 22.752 | 67.368 | 1.00 | 48.25 | B | C |
| ATOM | 4566 | CB | GLU | B | 290 | 20.885 | 22.839 | 65.974 | 1.00 | 47.83 | B | C |
| ATOM | 4567 | CG | GLU | B | 290 | 20.018 | 23.485 | 64.927 | 1.00 | 46.60 | B | C |
| ATOM | 4568 | CD | GLU | B | 290 | 20.775 | 23.663 | 63.619 | 1.00 | 46.76 | B | C |
| ATOM | 4569 | OE1 | GLU | B | 290 | 21.161 | 24.817 | 63.297 | 1.00 | 46.43 | B | O |
| ATOM | 4570 | OE2 | GLU | B | 290 | 21.003 | 22.642 | 62.924 | 1.00 | 45.01 | B | O |
| ATOM | 4571 | C | GLU | B | 290 | 21.388 | 22.514 | 68.379 | 1.00 | 48.76 | B | C |
| ATOM | 4572 | O | GLU | B | 290 | 22.108 | 23.453 | 68.754 | 1.00 | 50.08 | B | O |
| ATOM | 4573 | N | PHE | B | 291 | 21.554 | 21.280 | 68.819 | 1.00 | 48.54 | B | N |
| ATOM | 4574 | CA | PHE | B | 291 | 22.593 | 20.994 | 69.790 | 1.00 | 48.27 | B | C |
| ATOM | 4575 | CB | PHE | B | 291 | 22.150 | 19.805 | 70.647 | 1.00 | 46.73 | B | C |
| ATOM | 4576 | CG | PHE | B | 291 | 20.920 | 20.104 | 71.451 | 1.00 | 45.58 | B | C |
| ATOM | 4577 | CD1 | PHE | B | 291 | 19.668 | 20.101 | 70.835 | 1.00 | 44.62 | B | C |
| ATOM | 4578 | CD2 | PHE | B | 291 | 21.016 | 20.513 | 72.778 | 1.00 | 44.68 | B | C |
| ATOM | 4579 | CE1 | PHE | B | 291 | 18.530 | 20.509 | 71.520 | 1.00 | 44.48 | B | C |
| ATOM | 4580 | CE2 | PHE | B | 291 | 19.881 | 20.923 | 73.478 | 1.00 | 44.64 | B | C |
| ATOM | 4581 | CZ | PHE | B | 291 | 18.633 | 20.924 | 72.846 | 1.00 | 44.65 | B | C |
| ATOM | 4582 | C | PHE | B | 291 | 23.929 | 20.764 | 69.087 | 1.00 | 48.41 | B | C |
| ATOM | 4583 | O | PHE | B | 291 | 24.512 | 19.686 | 69.150 | 1.00 | 48.19 | B | O |
| ATOM | 4584 | N | LYS | B | 292 | 24.393 | 21.827 | 68.429 | 1.00 | 48.84 | B | N |
| ATOM | 4585 | CA | LYS | B | 292 | 25.645 | 21.844 | 67.676 | 1.00 | 49.03 | B | C |
| ATOM | 4586 | CB | LYS | B | 292 | 25.357 | 22.087 | 66.189 | 1.00 | 49.54 | B | C |
| ATOM | 4587 | CG | LYS | B | 292 | 24.377 | 21.100 | 65.550 | 1.00 | 50.85 | B | C |
| ATOM | 4588 | CD | LYS | B | 292 | 25.089 | 19.853 | 65.054 | 1.00 | 52.56 | B | C |
| ATOM | 4589 | CE | LYS | B | 292 | 24.223 | 19.065 | 64.082 | 1.00 | 54.48 | B | C |
| ATOM | 4590 | NZ | LYS | B | 292 | 22.934 | 18.661 | 64.725 | 1.00 | 54.20 | B | N |
| ATOM | 4591 | C | LYS | B | 292 | 26.504 | 23.000 | 68.185 | 1.00 | 48.82 | B | C |
| ATOM | 4592 | O | LYS | B | 292 | 26.169 | 24.155 | 67.955 | 1.00 | 50.24 | B | O |
| ATOM | 4593 | N | PHE | B | 293 | 27.597 | 22.704 | 68.881 | 1.00 | 47.42 | B | N |
| ATOM | 4594 | CA | PHE | B | 293 | 28.466 | 23.773 | 69.357 | 1.00 | 46.47 | B | C |
| ATOM | 4595 | CB | PHE | B | 293 | 27.830 | 24.477 | 70.564 | 1.00 | 44.76 | B | C |
| ATOM | 4596 | CG | PHE | B | 293 | 27.144 | 25.775 | 70.222 | 1.00 | 42.93 | B | C |
| ATOM | 4597 | CD1 | PHE | B | 293 | 25.793 | 25.967 | 70.518 | 1.00 | 42.19 | B | C |
| ATOM | 4598 | CD2 | PHE | B | 293 | 27.854 | 26.812 | 69.615 | 1.00 | 41.65 | B | C |
| ATOM | 4599 | CE1 | PHE | B | 293 | 25.156 | 27.179 | 70.214 | 1.00 | 41.40 | B | C |
| ATOM | 4600 | CE2 | PHE | B | 293 | 27.235 | 28.024 | 69.307 | 1.00 | 41.12 | B | C |
| ATOM | 4601 | CZ | PHE | B | 293 | 25.882 | 28.213 | 69.606 | 1.00 | 41.96 | B | C |
| ATOM | 4602 | C | PHE | B | 293 | 29.861 | 23.263 | 69.714 | 1.00 | 46.24 | B | C |
| ATOM | 4603 | O | PHE | B | 293 | 30.098 | 22.057 | 69.740 | 1.00 | 45.25 | B | O |
| ATOM | 4608 | N | TRP | B | 301 | 37.768 | 30.531 | 85.621 | 1.00 | 33.70 | B | N |

| | | | | | | | | | | | | |
|---|---|---|---|---|---|---|---|---|---|---|---|---|
| ATOM | 4609 | CA | TRP | B | 301 | 37.545 | 31.952 | 85.847 | 1.00 | 33.06 | B | C |
| ATOM | 4610 | CB | TRP | B | 301 | 36.453 | 32.158 | 86.894 | 1.00 | 31.08 | B | C |
| ATOM | 4611 | CG | TRP | B | 301 | 35.096 | 32.090 | 86.297 | 1.00 | 29.17 | B | C |
| ATOM | 4612 | CD2 | TRP | B | 301 | 34.485 | 33.074 | 85.456 | 1.00 | 27.03 | B | C |
| ATOM | 4613 | CE2 | TRP | B | 301 | 33.212 | 32.588 | 85.098 | 1.00 | 27.60 | B | C |
| ATOM | 4614 | CE3 | TRP | B | 301 | 34.889 | 34.327 | 84.970 | 1.00 | 26.66 | B | C |
| ATOM | 4615 | CD1 | TRP | B | 301 | 34.198 | 31.070 | 86.411 | 1.00 | 28.84 | B | C |
| ATOM | 4616 | NE1 | TRP | B | 301 | 33.062 | 31.359 | 85.695 | 1.00 | 28.04 | B | N |
| ATOM | 4617 | CZ2 | TRP | B | 301 | 32.336 | 33.304 | 84.273 | 1.00 | 28.28 | B | C |
| ATOM | 4618 | CZ3 | TRP | B | 301 | 34.022 | 35.041 | 84.151 | 1.00 | 25.91 | B | C |
| ATOM | 4619 | CH2 | TRP | B | 301 | 32.759 | 34.530 | 83.813 | 1.00 | 26.22 | B | C |
| ATOM | 4620 | C | TRP | B | 301 | 38.778 | 32.760 | 86.222 | 1.00 | 33.58 | B | C |
| ATOM | 4621 | O | TRP | B | 301 | 38.967 | 33.877 | 85.725 | 1.00 | 33.77 | B | O |
| ATOM | 4622 | N | THR | B | 302 | 39.622 | 32.201 | 87.084 | 1.00 | 34.88 | B | N |
| ATOM | 4623 | CA | THR | B | 302 | 40.825 | 32.903 | 87.515 | 1.00 | 36.12 | B | C |
| ATOM | 4624 | CB | THR | B | 302 | 41.577 | 32.110 | 88.584 | 1.00 | 36.50 | B | C |
| ATOM | 4625 | OG1 | THR | B | 302 | 40.649 | 31.654 | 89.574 | 1.00 | 35.65 | B | O |
| ATOM | 4626 | CG2 | THR | B | 302 | 42.634 | 32.998 | 89.257 | 1.00 | 36.54 | B | C |
| ATOM | 4627 | C | THR | B | 302 | 41.794 | 33.211 | 86.370 | 1.00 | 36.96 | B | C |
| ATOM | 4628 | O | THR | B | 302 | 42.619 | 34.124 | 86.477 | 1.00 | 37.46 | B | O |
| ATOM | 4629 | N | LYS | B | 303 | 41.703 | 32.460 | 85.278 | 1.00 | 36.83 | B | N |
| ATOM | 4630 | CA | LYS | B | 303 | 42.589 | 32.717 | 84.151 | 1.00 | 37.34 | B | C |
| ATOM | 4631 | CB | LYS | B | 303 | 43.017 | 31.393 | 83.516 | 1.00 | 40.09 | B | C |
| ATOM | 4632 | CG | LYS | B | 303 | 43.868 | 30.544 | 84.475 | 1.00 | 42.92 | B | C |
| ATOM | 4633 | CD | LYS | B | 303 | 44.770 | 29.590 | 83.720 | 1.00 | 45.66 | B | C |
| ATOM | 4634 | CE | LYS | B | 303 | 45.746 | 28.864 | 84.644 | 1.00 | 47.53 | B | C |
| ATOM | 4635 | NZ | LYS | B | 303 | 46.734 | 28.062 | 83.844 | 1.00 | 46.97 | B | N |
| ATOM | 4636 | C | LYS | B | 303 | 41.970 | 33.666 | 83.119 | 1.00 | 36.24 | B | C |
| ATOM | 4637 | O | LYS | B | 303 | 42.608 | 34.042 | 82.147 | 1.00 | 35.66 | B | O |
| ATOM | 4638 | N | VAL | B | 304 | 40.728 | 34.061 | 83.364 | 1.00 | 34.79 | B | N |
| ATOM | 4639 | CA | VAL | B | 304 | 40.007 | 34.990 | 82.498 | 1.00 | 34.93 | B | C |
| ATOM | 4640 | CB | VAL | B | 304 | 38.484 | 34.972 | 82.819 | 1.00 | 34.91 | B | C |
| ATOM | 4641 | CG1 | VAL | B | 304 | 37.769 | 36.111 | 82.087 | 1.00 | 32.44 | B | C |
| ATOM | 4642 | CG2 | VAL | B | 304 | 37.886 | 33.619 | 82.446 | 1.00 | 33.32 | B | C |
| ATOM | 4643 | C | VAL | B | 304 | 40.510 | 36.414 | 82.709 | 1.00 | 34.84 | B | C |
| ATOM | 4644 | O | VAL | B | 304 | 40.617 | 37.183 | 81.766 | 1.00 | 34.93 | B | O |
| ATOM | 4645 | N | PHE | B | 305 | 40.819 | 36.740 | 83.962 | 1.00 | 33.78 | B | N |
| ATOM | 4646 | CA | PHE | B | 305 | 41.271 | 38.076 | 84.344 | 1.00 | 34.67 | B | C |
| ATOM | 4647 | CB | PHE | B | 305 | 40.516 | 38.505 | 85.611 | 1.00 | 31.76 | B | C |
| ATOM | 4648 | CG | PHE | B | 305 | 39.020 | 38.368 | 85.501 | 1.00 | 28.26 | B | C |
| ATOM | 4649 | CD1 | PHE | B | 305 | 38.248 | 39.395 | 84.968 | 1.00 | 27.03 | B | C |
| ATOM | 4650 | CD2 | PHE | B | 305 | 38.384 | 37.196 | 85.901 | 1.00 | 27.85 | B | C |
| ATOM | 4651 | CE1 | PHE | B | 305 | 36.864 | 39.264 | 84.838 | 1.00 | 24.27 | B | C |
| ATOM | 4652 | CE2 | PHE | B | 305 | 36.997 | 37.051 | 85.776 | 1.00 | 27.29 | B | C |
| ATOM | 4653 | CZ | PHE | B | 305 | 36.237 | 38.091 | 85.239 | 1.00 | 25.87 | B | C |
| ATOM | 4654 | C | PHE | B | 305 | 42.779 | 38.172 | 84.571 | 1.00 | 36.62 | B | C |
| ATOM | 4655 | O | PHE | B | 305 | 43.481 | 37.160 | 84.564 | 1.00 | 37.06 | B | O |
| ATOM | 4656 | N | ARG | B | 306 | 43.266 | 39.399 | 84.764 | 1.00 | 38.68 | B | N |
| ATOM | 4657 | CA | ARG | B | 306 | 44.691 | 39.639 | 85.001 | 1.00 | 41.34 | B | C |
| ATOM | 4658 | CB | ARG | B | 306 | 44.954 | 41.124 | 85.208 | 1.00 | 43.31 | B | C |
| ATOM | 4659 | CG | ARG | B | 306 | 44.554 | 41.993 | 84.035 | 1.00 | 48.75 | B | C |
| ATOM | 4660 | CD | ARG | B | 306 | 44.365 | 43.427 | 84.505 | 1.00 | 51.25 | B | C |
| ATOM | 4661 | NE | ARG | B | 306 | 43.776 | 43.433 | 85.841 | 1.00 | 53.87 | B | N |
| ATOM | 4662 | CZ | ARG | B | 306 | 43.216 | 44.491 | 86.415 | 1.00 | 55.76 | B | C |
| ATOM | 4663 | NH1 | ARG | B | 306 | 43.163 | 45.648 | 85.762 | 1.00 | 56.29 | B | N |
| ATOM | 4664 | NH2 | ARG | B | 306 | 42.717 | 44.387 | 87.648 | 1.00 | 56.11 | B | N |
| ATOM | 4665 | C | ARG | B | 306 | 45.088 | 38.875 | 86.264 | 1.00 | 42.00 | B | C |
| ATOM | 4666 | O | ARG | B | 306 | 44.235 | 38.479 | 87.052 | 1.00 | 41.36 | B | O |
| ATOM | 4667 | N | PRO | B | 307 | 46.394 | 38.652 | 86.459 | 1.00 | 42.77 | B | N |
| ATOM | 4668 | CD | PRO | B | 307 | 47.450 | 38.784 | 85.439 | 1.00 | 43.61 | B | C |
| ATOM | 4669 | CA | PRO | B | 307 | 46.895 | 37.931 | 87.631 | 1.00 | 43.22 | B | C |
| ATOM | 4670 | CB | PRO | B | 307 | 48.393 | 37.818 | 87.353 | 1.00 | 43.40 | B | C |
| ATOM | 4671 | CG | PRO | B | 307 | 48.427 | 37.694 | 85.846 | 1.00 | 43.30 | B | C |

| | | | | | | | | | | | | |
|---|---|---|---|---|---|---|---|---|---|---|---|---|
| ATOM | 4672 | C | PRO | B | 307 | 46.591 | 38.566 | 88.989 | 1.00 | 43.52 | B | C |
| ATOM | 4673 | O | PRO | B | 307 | 46.231 | 37.868 | 89.936 | 1.00 | 44.25 | B | O |
| ATOM | 4674 | N | ALA | B | 308 | 46.725 | 39.883 | 89.082 | 1.00 | 43.22 | B | N |
| ATOM | 4675 | CA | ALA | B | 308 | 46.480 | 40.591 | 90.342 | 1.00 | 43.23 | B | C |
| ATOM | 4676 | CB | ALA | B | 308 | 46.952 | 42.047 | 90.205 | 1.00 | 44.62 | B | C |
| ATOM | 4677 | C | ALA | B | 308 | 45.009 | 40.559 | 90.782 | 1.00 | 42.25 | B | C |
| ATOM | 4678 | O | ALA | B | 308 | 44.720 | 40.485 | 91.965 | 1.00 | 43.89 | B | O |
| ATOM | 4679 | N | THR | B | 309 | 44.097 | 40.617 | 89.817 | 1.00 | 39.85 | B | N |
| ATOM | 4680 | CA | THR | B | 309 | 42.656 | 40.612 | 90.074 | 1.00 | 36.44 | B | C |
| ATOM | 4681 | CB | THR | B | 309 | 41.877 | 39.942 | 88.904 | 1.00 | 36.02 | B | C |
| ATOM | 4682 | OG1 | THR | B | 309 | 42.229 | 40.570 | 87.664 | 1.00 | 34.20 | B | O |
| ATOM | 4683 | CG2 | THR | B | 309 | 40.375 | 40.079 | 89.116 | 1.00 | 33.39 | B | C |
| ATOM | 4684 | C | THR | B | 309 | 42.225 | 39.937 | 91.375 | 1.00 | 34.50 | B | C |
| ATOM | 4685 | O | THR | B | 309 | 42.495 | 38.774 | 91.596 | 1.00 | 33.92 | B | O |
| ATOM | 4686 | N | PRO | B | 310 | 41.524 | 40.681 | 92.242 | 1.00 | 34.02 | B | N |
| ATOM | 4687 | CD | PRO | B | 310 | 41.097 | 42.075 | 92.033 | 1.00 | 33.66 | B | C |
| ATOM | 4688 | CA | PRO | B | 310 | 41.037 | 40.181 | 93.531 | 1.00 | 33.91 | B | C |
| ATOM | 4689 | CB | PRO | B | 310 | 40.236 | 41.359 | 94.080 | 1.00 | 33.33 | B | C |
| ATOM | 4690 | CG | PRO | B | 310 | 40.880 | 42.547 | 93.439 | 1.00 | 34.03 | B | C |
| ATOM | 4691 | C | PRO | B | 310 | 40.174 | 38.929 | 93.375 | 1.00 | 33.50 | B | C |
| ATOM | 4692 | O | PRO | B | 310 | 39.200 | 38.925 | 92.631 | 1.00 | 33.08 | B | O |
| ATOM | 4693 | N | PRO | B | 311 | 40.520 | 37.856 | 94.098 | 1.00 | 33.46 | B | N |
| ATOM | 4694 | CD | PRO | B | 311 | 41.602 | 37.750 | 95.094 | 1.00 | 33.78 | B | C |
| ATOM | 4695 | CA | PRO | B | 311 | 39.758 | 36.609 | 94.015 | 1.00 | 32.88 | B | C |
| ATOM | 4696 | CB | PRO | B | 311 | 40.479 | 35.686 | 95.002 | 1.00 | 32.47 | B | C |
| ATOM | 4697 | CG | PRO | B | 311 | 41.104 | 36.630 | 95.979 | 1.00 | 34.03 | B | C |
| ATOM | 4698 | C | PRO | B | 311 | 38.263 | 36.747 | 94.288 | 1.00 | 32.52 | B | C |
| ATOM | 4699 | O | PRO | B | 311 | 37.467 | 36.026 | 93.692 | 1.00 | 32.74 | B | O |
| ATOM | 4700 | N | GLU | B | 312 | 37.870 | 37.665 | 95.169 | 1.00 | 32.49 | B | N |
| ATOM | 4701 | CA | GLU | B | 312 | 36.439 | 37.826 | 95.431 | 1.00 | 32.71 | B | C |
| ATOM | 4702 | CB | GLU | B | 312 | 36.175 | 38.669 | 96.687 | 1.00 | 35.32 | B | C |
| ATOM | 4703 | CG | GLU | B | 312 | 37.250 | 39.668 | 97.039 | 1.00 | 41.49 | B | C |
| ATOM | 4704 | CD | GLU | B | 312 | 38.433 | 39.015 | 97.737 | 1.00 | 43.16 | B | C |
| ATOM | 4705 | OE1 | GLU | B | 312 | 39.538 | 39.004 | 97.150 | 1.00 | 42.47 | B | O |
| ATOM | 4706 | OE2 | GLU | B | 312 | 38.249 | 38.518 | 98.877 | 1.00 | 44.10 | B | O |
| ATOM | 4707 | C | GLU | B | 312 | 35.692 | 38.410 | 94.224 | 1.00 | 30.99 | B | C |
| ATOM | 4708 | O | GLU | B | 312 | 34.534 | 38.086 | 94.002 | 1.00 | 29.65 | B | O |
| ATOM | 4709 | N | ALA | B | 313 | 36.364 | 39.250 | 93.439 | 1.00 | 28.61 | B | N |
| ATOM | 4710 | CA | ALA | B | 313 | 35.736 | 39.832 | 92.257 | 1.00 | 28.28 | B | C |
| ATOM | 4711 | CB | ALA | B | 313 | 36.680 | 40.847 | 91.587 | 1.00 | 27.99 | B | C |
| ATOM | 4712 | C | ALA | B | 313 | 35.408 | 38.685 | 91.294 | 1.00 | 27.74 | B | C |
| ATOM | 4713 | O | ALA | B | 313 | 34.305 | 38.600 | 90.755 | 1.00 | 25.60 | B | O |
| ATOM | 4714 | N | ILE | B | 314 | 36.386 | 37.805 | 91.101 | 1.00 | 26.81 | B | N |
| ATOM | 4715 | CA | ILE | B | 314 | 36.234 | 36.650 | 90.229 | 1.00 | 27.52 | B | C |
| ATOM | 4716 | CB | ILE | B | 314 | 37.553 | 35.839 | 90.161 | 1.00 | 27.83 | B | C |
| ATOM | 4717 | CG2 | ILE | B | 314 | 37.328 | 34.510 | 89.448 | 1.00 | 27.37 | B | C |
| ATOM | 4718 | CG1 | ILE | B | 314 | 38.614 | 36.658 | 89.425 | 1.00 | 26.77 | B | C |
| ATOM | 4719 | CD1 | ILE | B | 314 | 39.988 | 36.037 | 89.426 | 1.00 | 26.43 | B | C |
| ATOM | 4720 | C | ILE | B | 314 | 35.106 | 35.750 | 90.729 | 1.00 | 28.18 | B | C |
| ATOM | 4721 | O | ILE | B | 314 | 34.268 | 35.309 | 89.948 | 1.00 | 28.99 | B | O |
| ATOM | 4722 | N | ALA | B | 315 | 35.089 | 35.484 | 92.034 | 1.00 | 27.61 | B | N |
| ATOM | 4723 | CA | ALA | B | 315 | 34.051 | 34.644 | 92.624 | 1.00 | 26.83 | B | C |
| ATOM | 4724 | CB | ALA | B | 315 | 34.229 | 34.579 | 94.136 | 1.00 | 27.84 | B | C |
| ATOM | 4725 | C | ALA | B | 315 | 32.674 | 35.212 | 92.278 | 1.00 | 26.06 | B | C |
| ATOM | 4726 | O | ALA | B | 315 | 31.811 | 34.500 | 91.766 | 1.00 | 26.97 | B | O |
| ATOM | 4727 | N | LEU | B | 316 | 32.486 | 36.501 | 92.555 | 1.00 | 24.90 | B | N |
| ATOM | 4728 | CA | LEU | B | 316 | 31.222 | 37.174 | 92.269 | 1.00 | 23.50 | B | C |
| ATOM | 4729 | CB | LEU | B | 316 | 31.310 | 38.670 | 92.613 | 1.00 | 20.43 | B | C |
| ATOM | 4730 | CG | LEU | B | 316 | 30.056 | 39.531 | 92.383 | 1.00 | 18.30 | B | C |
| ATOM | 4731 | CD1 | LEU | B | 316 | 28.856 | 38.863 | 93.025 | 1.00 | 17.27 | B | C |
| ATOM | 4732 | CD2 | LEU | B | 316 | 30.262 | 40.932 | 92.956 | 1.00 | 14.27 | B | C |
| ATOM | 4733 | C | LEU | B | 316 | 30.832 | 36.992 | 90.802 | 1.00 | 23.09 | B | C |
| ATOM | 4734 | O | LEU | B | 316 | 29.697 | 36.667 | 90.500 | 1.00 | 21.07 | B | O |

| | | | | | | | | | | | | |
|---|---|---|---|---|---|---|---|---|---|---|---|---|
| ATOM | 4735 | N | CYS | B | 317 | 31.788 | 37.203 | 89.900 | 1.00 | 23.42 | B | N |
| ATOM | 4736 | CA | CYS | B | 317 | 31.527 | 37.032 | 88.478 | 1.00 | 25.74 | B | C |
| ATOM | 4737 | CB | CYS | B | 317 | 32.804 | 37.231 | 87.664 | 1.00 | 27.81 | B | C |
| ATOM | 4738 | SG | CYS | B | 317 | 33.142 | 38.925 | 87.210 | 1.00 | 34.98 | B | S |
| ATOM | 4739 | C | CYS | B | 317 | 30.994 | 35.643 | 88.184 | 1.00 | 26.22 | B | C |
| ATOM | 4740 | O | CYS | B | 317 | 30.033 | 35.488 | 87.438 | 1.00 | 28.08 | B | O |
| ATOM | 4741 | N | SER | B | 318 | 31.625 | 34.630 | 88.769 | 1.00 | 25.35 | B | N |
| ATOM | 4742 | CA | SER | B | 318 | 31.217 | 33.258 | 88.523 | 1.00 | 25.44 | B | C |
| ATOM | 4743 | CB | SER | B | 318 | 32.234 | 32.276 | 89.127 | 1.00 | 27.76 | B | C |
| ATOM | 4744 | OG | SER | B | 318 | 32.200 | 32.278 | 90.551 | 1.00 | 31.85 | B | O |
| ATOM | 4745 | C | SER | B | 318 | 29.835 | 32.963 | 89.059 | 1.00 | 24.60 | B | C |
| ATOM | 4746 | O | SER | B | 318 | 29.226 | 32.011 | 88.643 | 1.00 | 24.43 | B | O |
| ATOM | 4747 | N | ARG | B | 319 | 29.353 | 33.788 | 89.986 | 1.00 | 24.03 | B | N |
| ATOM | 4748 | CA | ARG | B | 319 | 28.023 | 33.583 | 90.548 | 1.00 | 24.21 | B | C |
| ATOM | 4749 | CB | ARG | B | 319 | 28.023 | 33.838 | 92.062 | 1.00 | 25.91 | B | C |
| ATOM | 4750 | CG | ARG | B | 319 | 28.788 | 32.787 | 92.886 | 1.00 | 29.97 | B | C |
| ATOM | 4751 | CD | ARG | B | 319 | 28.390 | 31.347 | 92.535 | 1.00 | 34.05 | B | C |
| ATOM | 4752 | NE | ARG | B | 319 | 26.938 | 31.190 | 92.399 | 1.00 | 39.33 | B | N |
| ATOM | 4753 | CZ | ARG | B | 319 | 26.064 | 31.365 | 93.387 | 1.00 | 39.40 | B | C |
| ATOM | 4754 | NH1 | ARG | B | 319 | 26.487 | 31.698 | 94.601 | 1.00 | 39.76 | B | N |
| ATOM | 4755 | NH2 | ARG | B | 319 | 24.760 | 31.235 | 93.155 | 1.00 | 38.52 | B | N |
| ATOM | 4756 | C | ARG | B | 319 | 26.988 | 34.463 | 89.853 | 1.00 | 23.04 | B | C |
| ATOM | 4757 | O | ARG | B | 319 | 25.815 | 34.390 | 90.148 | 1.00 | 22.96 | B | O |
| ATOM | 4758 | N | LEU | B | 320 | 27.440 | 35.287 | 88.918 | 1.00 | 21.83 | B | N |
| ATOM | 4759 | CA | LEU | B | 320 | 26.533 | 36.154 | 88.172 | 1.00 | 22.05 | B | C |
| ATOM | 4760 | CB | LEU | B | 320 | 27.128 | 37.563 | 88.055 | 1.00 | 19.96 | B | C |
| ATOM | 4761 | CG | LEU | B | 320 | 27.288 | 38.273 | 89.401 | 1.00 | 19.20 | B | C |
| ATOM | 4762 | CD1 | LEU | B | 320 | 28.111 | 39.533 | 89.267 | 1.00 | 19.11 | B | C |
| ATOM | 4763 | CD2 | LEU | B | 320 | 25.914 | 38.575 | 89.942 | 1.00 | 17.65 | B | C |
| ATOM | 4764 | C | LEU | B | 320 | 26.326 | 35.551 | 86.784 | 1.00 | 22.71 | B | C |
| ATOM | 4765 | O | LEU | B | 320 | 25.210 | 35.217 | 86.397 | 1.00 | 22.55 | B | O |
| ATOM | 4766 | N | LEU | B | 321 | 27.432 | 35.396 | 86.063 | 1.00 | 22.24 | B | N |
| ATOM | 4767 | CA | LEU | B | 321 | 27.434 | 34.851 | 84.711 | 1.00 | 22.52 | B | C |
| ATOM | 4768 | CB | LEU | B | 321 | 28.742 | 35.244 | 84.027 | 1.00 | 20.62 | B | C |
| ATOM | 4769 | CG | LEU | B | 321 | 28.944 | 36.755 | 84.143 | 1.00 | 19.98 | B | C |
| ATOM | 4770 | CD1 | LEU | B | 321 | 30.353 | 37.140 | 83.776 | 1.00 | 18.38 | B | C |
| ATOM | 4771 | CD2 | LEU | B | 321 | 27.917 | 37.452 | 83.252 | 1.00 | 19.80 | B | C |
| ATOM | 4772 | C | LEU | B | 321 | 27.267 | 33.338 | 84.734 | 1.00 | 21.42 | B | C |
| ATOM | 4773 | O | LEU | B | 321 | 28.208 | 32.595 | 84.576 | 1.00 | 19.75 | B | O |
| ATOM | 4774 | N | GLU | B | 322 | 26.024 | 32.920 | 84.910 | 1.00 | 22.64 | B | N |
| ATOM | 4775 | CA | GLU | B | 322 | 25.666 | 31.517 | 85.019 | 1.00 | 24.14 | B | C |
| ATOM | 4776 | CB | GLU | B | 322 | 25.175 | 31.284 | 86.446 | 1.00 | 26.90 | B | C |
| ATOM | 4777 | CG | GLU | B | 322 | 25.420 | 29.916 | 87.001 | 1.00 | 34.64 | B | C |
| ATOM | 4778 | CD | GLU | B | 322 | 26.104 | 29.958 | 88.357 | 1.00 | 37.65 | B | C |
| ATOM | 4779 | OE1 | GLU | B | 322 | 25.728 | 30.803 | 89.194 | 1.00 | 39.58 | B | O |
| ATOM | 4780 | OE2 | GLU | B | 322 | 27.013 | 29.138 | 88.594 | 1.00 | 40.88 | B | O |
| ATOM | 4781 | C | GLU | B | 322 | 24.564 | 31.187 | 84.010 | 1.00 | 23.52 | B | C |
| ATOM | 4782 | O | GLU | B | 322 | 23.633 | 31.970 | 83.823 | 1.00 | 23.28 | B | O |
| ATOM | 4783 | N | TYR | B | 323 | 24.681 | 30.032 | 83.362 | 1.00 | 22.96 | B | N |
| ATOM | 4784 | CA | TYR | B | 323 | 23.682 | 29.602 | 82.386 | 1.00 | 22.52 | B | C |
| ATOM | 4785 | CB | TYR | B | 323 | 24.063 | 28.243 | 81.800 | 1.00 | 21.41 | B | C |
| ATOM | 4786 | CG | TYR | B | 323 | 25.135 | 28.293 | 80.731 | 1.00 | 20.64 | B | C |
| ATOM | 4787 | CD1 | TYR | B | 323 | 24.927 | 28.982 | 79.542 | 1.00 | 19.55 | B | C |
| ATOM | 4788 | CE1 | TYR | B | 323 | 25.871 | 28.983 | 78.530 | 1.00 | 20.78 | B | C |
| ATOM | 4789 | CD2 | TYR | B | 323 | 26.335 | 27.598 | 80.884 | 1.00 | 21.41 | B | C |
| ATOM | 4790 | CE2 | TYR | B | 323 | 27.293 | 27.589 | 79.873 | 1.00 | 22.23 | B | C |
| ATOM | 4791 | CZ | TYR | B | 323 | 27.050 | 28.283 | 78.696 | 1.00 | 22.70 | B | C |
| ATOM | 4792 | OH | TYR | B | 323 | 27.979 | 28.271 | 77.683 | 1.00 | 23.44 | B | O |
| ATOM | 4793 | C | TYR | B | 323 | 22.291 | 29.506 | 83.006 | 1.00 | 22.56 | B | C |
| ATOM | 4794 | O | TYR | B | 323 | 21.343 | 30.100 | 82.510 | 1.00 | 21.17 | B | O |
| ATOM | 4795 | N | THR | B | 324 | 22.178 | 28.742 | 84.088 | 1.00 | 21.98 | B | N |
| ATOM | 4796 | CA | THR | B | 324 | 20.893 | 28.580 | 84.747 | 1.00 | 23.78 | B | C |
| ATOM | 4797 | CB | THR | B | 324 | 20.949 | 27.483 | 85.827 | 1.00 | 24.69 | B | C |

| | | | | | | | | | | | | |
|---|---|---|---|---|---|---|---|---|---|---|---|---|
| ATOM | 4798 | OG1 | THR | B | 324 | 21.456 | 26.275 | 85.247 | 1.00 | 23.78 | B | O |
| ATOM | 4799 | CG2 | THR | B | 324 | 19.554 | 27.215 | 86.386 | 1.00 | 24.73 | B | C |
| ATOM | 4800 | C | THR | B | 324 | 20.504 | 29.915 | 85.366 | 1.00 | 23.12 | B | C |
| ATOM | 4801 | O | THR | B | 324 | 21.143 | 30.395 | 86.279 | 1.00 | 25.20 | B | O |
| ATOM | 4802 | N | PRO | B | 325 | 19.449 | 30.539 | 84.840 | 1.00 | 23.45 | B | N |
| ATOM | 4803 | CD | PRO | B | 325 | 18.630 | 30.094 | 83.697 | 1.00 | 23.72 | B | C |
| ATOM | 4804 | CA | PRO | B | 325 | 18.988 | 31.832 | 85.355 | 1.00 | 22.55 | B | C |
| ATOM | 4805 | CB | PRO | B | 325 | 17.719 | 32.103 | 84.544 | 1.00 | 22.16 | B | C |
| ATOM | 4806 | CG | PRO | B | 325 | 17.981 | 31.396 | 83.241 | 1.00 | 24.49 | B | C |
| ATOM | 4807 | C | PRO | B | 325 | 18.719 | 31.816 | 86.857 | 1.00 | 23.11 | B | C |
| ATOM | 4808 | O | PRO | B | 325 | 19.126 | 32.725 | 87.564 | 1.00 | 22.38 | B | O |
| ATOM | 4809 | N | THR | B | 326 | 18.035 | 30.776 | 87.334 | 1.00 | 23.85 | B | N |
| ATOM | 4810 | CA | THR | B | 326 | 17.704 | 30.657 | 88.758 | 1.00 | 23.82 | B | C |
| ATOM | 4811 | CB | THR | B | 326 | 16.707 | 29.495 | 89.022 | 1.00 | 24.98 | B | C |
| ATOM | 4812 | OG1 | THR | B | 326 | 17.267 | 28.262 | 88.553 | 1.00 | 26.02 | B | O |
| ATOM | 4813 | CG2 | THR | B | 326 | 15.384 | 29.748 | 88.321 | 1.00 | 25.23 | B | C |
| ATOM | 4814 | C | THR | B | 326 | 18.911 | 30.448 | 89.664 | 1.00 | 23.37 | B | C |
| ATOM | 4815 | O | THR | B | 326 | 18.808 | 30.601 | 90.875 | 1.00 | 23.10 | B | O |
| ATOM | 4816 | N | ALA | B | 327 | 20.050 | 30.098 | 89.077 | 1.00 | 22.41 | B | N |
| ATOM | 4817 | CA | ALA | B | 327 | 21.270 | 29.871 | 89.855 | 1.00 | 23.11 | B | C |
| ATOM | 4818 | CB | ALA | B | 327 | 22.178 | 28.916 | 89.118 | 1.00 | 22.34 | B | C |
| ATOM | 4819 | C | ALA | B | 327 | 22.019 | 31.170 | 90.136 | 1.00 | 23.55 | B | C |
| ATOM | 4820 | O | ALA | B | 327 | 22.772 | 31.258 | 91.086 | 1.00 | 23.42 | B | O |
| ATOM | 4821 | N | ARG | B | 328 | 21.802 | 32.172 | 89.294 | 1.00 | 23.04 | B | N |
| ATOM | 4822 | CA | ARG | B | 328 | 22.479 | 33.452 | 89.456 | 1.00 | 22.84 | B | C |
| ATOM | 4823 | CB | ARG | B | 328 | 22.128 | 34.396 | 88.302 | 1.00 | 20.05 | B | C |
| ATOM | 4824 | CG | ARG | B | 328 | 22.458 | 33.845 | 86.918 | 1.00 | 20.00 | B | C |
| ATOM | 4825 | CD | ARG | B | 328 | 21.780 | 34.648 | 85.819 | 1.00 | 18.05 | B | C |
| ATOM | 4826 | NE | ARG | B | 328 | 21.954 | 34.021 | 84.516 | 1.00 | 18.51 | B | N |
| ATOM | 4827 | CZ | ARG | B | 328 | 21.201 | 34.272 | 83.453 | 1.00 | 17.85 | B | C |
| ATOM | 4828 | NH1 | ARG | B | 328 | 20.204 | 35.145 | 83.523 | 1.00 | 17.01 | B | N |
| ATOM | 4829 | NH2 | ARG | B | 328 | 21.447 | 33.640 | 82.317 | 1.00 | 19.64 | B | N |
| ATOM | 4830 | C | ARG | B | 328 | 22.102 | 34.111 | 90.770 | 1.00 | 23.25 | B | C |
| ATOM | 4831 | O | ARG | B | 328 | 21.027 | 33.894 | 91.289 | 1.00 | 22.12 | B | O |
| ATOM | 4832 | N | LEU | B | 329 | 23.019 | 34.907 | 91.300 | 1.00 | 23.71 | B | N |
| ATOM | 4833 | CA | LEU | B | 329 | 22.763 | 35.625 | 92.535 | 1.00 | 23.63 | B | C |
| ATOM | 4834 | CB | LEU | B | 329 | 24.003 | 36.391 | 92.979 | 1.00 | 22.01 | B | C |
| ATOM | 4835 | CG | LEU | B | 329 | 24.911 | 35.722 | 94.002 | 1.00 | 24.74 | B | C |
| ATOM | 4836 | CD1 | LEU | B | 329 | 25.052 | 34.252 | 93.689 | 1.00 | 25.55 | B | C |
| ATOM | 4837 | CD2 | LEU | B | 329 | 26.262 | 36.423 | 94.001 | 1.00 | 21.98 | B | C |
| ATOM | 4838 | C | LEU | B | 329 | 21.641 | 36.624 | 92.329 | 1.00 | 23.40 | B | C |
| ATOM | 4839 | O | LEU | B | 329 | 21.347 | 37.049 | 91.205 | 1.00 | 22.56 | B | O |
| ATOM | 4840 | N | THR | B | 330 | 21.024 | 36.981 | 93.443 | 1.00 | 23.07 | B | N |
| ATOM | 4841 | CA | THR | B | 330 | 19.957 | 37.967 | 93.496 | 1.00 | 22.79 | B | C |
| ATOM | 4842 | CB | THR | B | 330 | 19.136 | 37.812 | 94.808 | 1.00 | 23.51 | B | C |
| ATOM | 4843 | OG1 | THR | B | 330 | 18.344 | 36.620 | 94.758 | 1.00 | 29.16 | B | O |
| ATOM | 4844 | CG2 | THR | B | 330 | 18.244 | 39.013 | 95.033 | 1.00 | 26.07 | B | C |
| ATOM | 4845 | C | THR | B | 330 | 20.711 | 39.276 | 93.636 | 1.00 | 20.93 | B | C |
| ATOM | 4846 | O | THR | B | 330 | 21.829 | 39.284 | 94.119 | 1.00 | 19.09 | B | O |
| ATOM | 4847 | N | PRO | B | 331 | 20.115 | 40.394 | 93.207 | 1.00 | 19.73 | B | N |
| ATOM | 4848 | CD | PRO | B | 331 | 18.955 | 40.550 | 92.313 | 1.00 | 20.77 | B | C |
| ATOM | 4849 | CA | PRO | B | 331 | 20.831 | 41.657 | 93.359 | 1.00 | 20.17 | B | C |
| ATOM | 4850 | CB | PRO | B | 331 | 19.841 | 42.671 | 92.806 | 1.00 | 20.27 | B | C |
| ATOM | 4851 | CG | PRO | B | 331 | 19.203 | 41.907 | 91.688 | 1.00 | 18.56 | B | C |
| ATOM | 4852 | C | PRO | B | 331 | 21.165 | 41.884 | 94.839 | 1.00 | 21.15 | B | C |
| ATOM | 4853 | O | PRO | B | 331 | 22.273 | 42.275 | 95.170 | 1.00 | 22.52 | B | O |
| ATOM | 4854 | N | LEU | B | 332 | 20.211 | 41.599 | 95.725 | 1.00 | 20.27 | B | N |
| ATOM | 4855 | CA | LEU | B | 332 | 20.440 | 41.798 | 97.153 | 1.00 | 22.53 | B | C |
| ATOM | 4856 | CB | LEU | B | 332 | 19.149 | 41.555 | 97.946 | 1.00 | 22.63 | B | C |
| ATOM | 4857 | CG | LEU | B | 332 | 19.165 | 42.101 | 99.380 | 1.00 | 24.61 | B | C |
| ATOM | 4858 | CD1 | LEU | B | 332 | 19.293 | 43.630 | 99.360 | 1.00 | 24.26 | B | C |
| ATOM | 4859 | CD2 | LEU | B | 332 | 17.890 | 41.683 | 100.095 | 1.00 | 24.48 | B | C |
| ATOM | 4860 | C | LEU | B | 332 | 21.560 | 40.881 | 97.641 | 1.00 | 22.91 | B | C |

| | | | | | | | | | | | | |
|---|---|---|---|---|---|---|---|---|---|---|---|---|
| ATOM | 4861 | O | LEU | B | 332 | 22.420 | 41.307 | 98.406 | 1.00 | 21.91 | B | O |
| ATOM | 4862 | N | GLU | B | 333 | 21.549 | 39.629 | 97.185 | 1.00 | 22.31 | B | N |
| ATOM | 4863 | CA | GLU | B | 333 | 22.589 | 38.676 | 97.553 | 1.00 | 23.59 | B | C |
| ATOM | 4864 | CB | GLU | B | 333 | 22.326 | 37.297 | 96.925 | 1.00 | 24.78 | B | C |
| ATOM | 4865 | CG | GLU | B | 333 | 21.099 | 36.564 | 97.488 | 1.00 | 29.10 | B | C |
| ATOM | 4866 | CD | GLU | B | 333 | 20.734 | 35.298 | 96.712 | 1.00 | 29.76 | B | C |
| ATOM | 4867 | OE1 | GLU | B | 333 | 21.366 | 35.020 | 95.672 | 1.00 | 31.60 | B | O |
| ATOM | 4868 | OE2 | GLU | B | 333 | 19.802 | 34.585 | 97.142 | 1.00 | 31.48 | B | O |
| ATOM | 4869 | C | GLU | B | 333 | 23.922 | 39.216 | 97.056 | 1.00 | 23.81 | B | C |
| ATOM | 4870 | O | GLU | B | 333 | 24.897 | 39.183 | 97.766 | 1.00 | 24.22 | B | O |
| ATOM | 4871 | N | ALA | B | 334 | 23.949 | 39.720 | 95.822 | 1.00 | 23.95 | B | N |
| ATOM | 4872 | CA | ALA | B | 334 | 25.187 | 40.256 | 95.267 | 1.00 | 23.82 | B | C |
| ATOM | 4873 | CB | ALA | B | 334 | 24.947 | 40.784 | 93.850 | 1.00 | 22.52 | B | C |
| ATOM | 4874 | C | ALA | B | 334 | 25.708 | 41.369 | 96.185 | 1.00 | 23.52 | B | C |
| ATOM | 4875 | O | ALA | B | 334 | 26.880 | 41.430 | 96.468 | 1.00 | 22.39 | B | O |
| ATOM | 4876 | N | CYS | B | 335 | 24.805 | 42.230 | 96.645 | 1.00 | 23.22 | B | N |
| ATOM | 4877 | CA | CYS | B | 335 | 25.174 | 43.317 | 97.543 | 1.00 | 22.90 | B | C |
| ATOM | 4878 | CB | CYS | B | 335 | 23.925 | 44.108 | 97.960 | 1.00 | 23.13 | B | C |
| ATOM | 4879 | SG | CYS | B | 335 | 23.216 | 45.222 | 96.696 | 1.00 | 22.65 | B | S |
| ATOM | 4880 | C | CYS | B | 335 | 25.883 | 42.781 | 98.793 | 1.00 | 24.18 | B | C |
| ATOM | 4881 | O | CYS | B | 335 | 26.797 | 43.407 | 99.304 | 1.00 | 23.61 | B | O |
| ATOM | 4882 | N | ALA | B | 336 | 25.461 | 41.611 | 99.265 | 1.00 | 23.53 | B | N |
| ATOM | 4883 | CA | ALA | B | 336 | 26.041 | 41.007 | 100.460 | 1.00 | 24.28 | B | C |
| ATOM | 4884 | CB | ALA | B | 336 | 25.031 | 40.065 | 101.105 | 1.00 | 22.83 | B | C |
| ATOM | 4885 | C | ALA | B | 336 | 27.351 | 40.264 | 100.230 | 1.00 | 24.78 | B | C |
| ATOM | 4886 | O | ALA | B | 336 | 28.030 | 39.878 | 101.194 | 1.00 | 24.50 | B | O |
| ATOM | 4887 | N | HIS | B | 337 | 27.709 | 40.071 | 98.967 | 1.00 | 23.22 | B | N |
| ATOM | 4888 | CA | HIS | B | 337 | 28.930 | 39.347 | 98.624 | 1.00 | 24.07 | B | C |
| ATOM | 4889 | CB | HIS | B | 337 | 29.100 | 39.338 | 97.107 | 1.00 | 24.21 | B | C |
| ATOM | 4890 | CG | HIS | B | 337 | 30.001 | 38.255 | 96.611 | 1.00 | 23.83 | B | C |
| ATOM | 4891 | CD2 | HIS | B | 337 | 29.736 | 36.987 | 96.209 | 1.00 | 21.55 | B | C |
| ATOM | 4892 | ND1 | HIS | B | 337 | 31.366 | 38.406 | 96.519 | 1.00 | 24.34 | B | N |
| ATOM | 4893 | CE1 | HIS | B | 337 | 31.904 | 37.282 | 96.082 | 1.00 | 23.68 | B | C |
| ATOM | 4894 | NE2 | HIS | B | 337 | 30.932 | 36.404 | 95.888 | 1.00 | 22.14 | B | N |
| ATOM | 4895 | C | HIS | B | 337 | 30.183 | 39.925 | 99.304 | 1.00 | 23.67 | B | C |
| ATOM | 4896 | O | HIS | B | 337 | 30.281 | 41.120 | 99.539 | 1.00 | 21.21 | B | O |
| ATOM | 4897 | N | SER | B | 338 | 31.135 | 39.050 | 99.611 | 1.00 | 24.00 | B | N |
| ATOM | 4898 | CA | SER | B | 338 | 32.373 | 39.459 | 100.263 | 1.00 | 26.48 | B | C |
| ATOM | 4899 | CB | SER | B | 338 | 33.272 | 38.248 | 100.518 | 1.00 | 27.52 | B | C |
| ATOM | 4900 | OG | SER | B | 338 | 32.655 | 37.372 | 101.441 | 1.00 | 31.32 | B | O |
| ATOM | 4901 | C | SER | B | 338 | 33.145 | 40.504 | 99.474 | 1.00 | 25.88 | B | C |
| ATOM | 4902 | O | SER | B | 338 | 33.866 | 41.315 | 100.055 | 1.00 | 26.28 | B | O |
| ATOM | 4903 | N | PHE | B | 339 | 32.993 | 40.487 | 98.153 | 1.00 | 24.47 | B | N |
| ATOM | 4904 | CA | PHE | B | 339 | 33.689 | 41.450 | 97.309 | 1.00 | 23.54 | B | C |
| ATOM | 4905 | CB | PHE | B | 339 | 33.307 | 41.264 | 95.840 | 1.00 | 21.99 | B | C |
| ATOM | 4906 | CG | PHE | B | 339 | 33.969 | 42.247 | 94.919 | 1.00 | 20.69 | B | C |
| ATOM | 4907 | CD1 | PHE | B | 339 | 35.354 | 42.298 | 94.817 | 1.00 | 21.07 | B | C |
| ATOM | 4908 | CD2 | PHE | B | 339 | 33.211 | 43.125 | 94.158 | 1.00 | 21.99 | B | C |
| ATOM | 4909 | CE1 | PHE | B | 339 | 35.977 | 43.214 | 93.966 | 1.00 | 22.10 | B | C |
| ATOM | 4910 | CE2 | PHE | B | 339 | 33.825 | 44.045 | 93.303 | 1.00 | 24.62 | B | C |
| ATOM | 4911 | CZ | PHE | B | 339 | 35.211 | 44.091 | 93.206 | 1.00 | 22.56 | B | C |
| ATOM | 4912 | C | PHE | B | 339 | 33.390 | 42.883 | 97.728 | 1.00 | 23.82 | B | C |
| ATOM | 4913 | O | PHE | B | 339 | 34.167 | 43.775 | 97.471 | 1.00 | 25.31 | B | O |
| ATOM | 4914 | N | PHE | B | 340 | 32.257 | 43.096 | 98.383 | 1.00 | 23.73 | B | N |
| ATOM | 4915 | CA | PHE | B | 340 | 31.886 | 44.440 | 98.809 | 1.00 | 23.92 | B | C |
| ATOM | 4916 | CB | PHE | B | 340 | 30.401 | 44.679 | 98.515 | 1.00 | 22.64 | B | C |
| ATOM | 4917 | CG | PHE | B | 340 | 30.053 | 44.594 | 97.041 | 1.00 | 23.75 | B | C |
| ATOM | 4918 | CD1 | PHE | B | 340 | 30.632 | 45.473 | 96.122 | 1.00 | 21.37 | B | C |
| ATOM | 4919 | CD2 | PHE | B | 340 | 29.155 | 43.631 | 96.573 | 1.00 | 20.44 | B | C |
| ATOM | 4920 | CE1 | PHE | B | 340 | 30.325 | 45.397 | 94.767 | 1.00 | 18.92 | B | C |
| ATOM | 4921 | CE2 | PHE | B | 340 | 28.844 | 43.551 | 95.219 | 1.00 | 21.42 | B | C |
| ATOM | 4922 | CZ | PHE | B | 340 | 29.432 | 44.438 | 94.313 | 1.00 | 20.94 | B | C |
| ATOM | 4923 | C | PHE | B | 340 | 32.189 | 44.706 | 100.286 | 1.00 | 24.19 | B | C |

| | | | | | | | | | | | | |
|---|---|---|---|---|---|---|---|---|---|---|---|---|
| ATOM | 4924 | O | PHE | B | 340 | 31.794 | 45.732 | 100.827 | 1.00 | 24.46 | B | O |
| ATOM | 4925 | N | ASP | B | 341 | 32.903 | 43.787 | 100.928 | 1.00 | 23.88 | B | N |
| ATOM | 4926 | CA | ASP | B | 341 | 33.236 | 43.939 | 102.344 | 1.00 | 25.42 | B | C |
| ATOM | 4927 | CB | ASP | B | 341 | 34.087 | 42.767 | 102.830 | 1.00 | 23.55 | B | C |
| ATOM | 4928 | CG | ASP | B | 341 | 33.289 | 41.503 | 102.983 | 1.00 | 25.11 | B | C |
| ATOM | 4929 | OD1 | ASP | B | 341 | 32.044 | 41.583 | 102.942 | 1.00 | 23.29 | B | O |
| ATOM | 4930 | OD2 | ASP | B | 341 | 33.903 | 40.430 | 103.154 | 1.00 | 27.05 | B | O |
| ATOM | 4931 | C | ASP | B | 341 | 33.970 | 45.234 | 102.659 | 1.00 | 25.43 | B | C |
| ATOM | 4932 | O | ASP | B | 341 | 33.739 | 45.823 | 103.683 | 1.00 | 27.00 | B | O |
| ATOM | 4933 | N | GLU | B | 342 | 34.860 | 45.660 | 101.770 | 1.00 | 25.32 | B | N |
| ATOM | 4934 | CA | GLU | B | 342 | 35.604 | 46.883 | 102.005 | 1.00 | 27.03 | B | C |
| ATOM | 4935 | CB | GLU | B | 342 | 36.598 | 47.141 | 100.861 | 1.00 | 28.22 | B | C |
| ATOM | 4936 | CG | GLU | B | 342 | 37.309 | 48.502 | 100.934 | 1.00 | 31.21 | B | C |
| ATOM | 4937 | CD | GLU | B | 342 | 38.533 | 48.616 | 100.013 | 1.00 | 32.07 | B | C |
| ATOM | 4938 | OE1 | GLU | B | 342 | 38.489 | 48.117 | 98.863 | 1.00 | 29.84 | B | O |
| ATOM | 4939 | OE2 | GLU | B | 342 | 39.538 | 49.227 | 100.445 | 1.00 | 35.15 | B | O |
| ATOM | 4940 | C | GLU | B | 342 | 34.661 | 48.066 | 102.183 | 1.00 | 27.02 | B | C |
| ATOM | 4941 | O | GLU | B | 342 | 34.876 | 48.894 | 103.047 | 1.00 | 25.83 | B | O |
| ATOM | 4942 | N | LEU | B | 343 | 33.605 | 48.130 | 101.374 | 1.00 | 26.44 | B | N |
| ATOM | 4943 | CA | LEU | B | 343 | 32.661 | 49.237 | 101.474 | 1.00 | 26.04 | B | C |
| ATOM | 4944 | CB | LEU | B | 343 | 31.581 | 49.144 | 100.389 | 1.00 | 24.65 | B | C |
| ATOM | 4945 | CG | LEU | B | 343 | 32.059 | 49.135 | 98.929 | 1.00 | 25.00 | B | C |
| ATOM | 4946 | CD1 | LEU | B | 343 | 30.855 | 49.095 | 97.983 | 1.00 | 21.25 | B | C |
| ATOM | 4947 | CD2 | LEU | B | 343 | 32.914 | 50.356 | 98.657 | 1.00 | 23.12 | B | C |
| ATOM | 4948 | C | LEU | B | 343 | 32.009 | 49.284 | 102.852 | 1.00 | 26.52 | B | C |
| ATOM | 4949 | O | LEU | B | 343 | 31.597 | 50.333 | 103.303 | 1.00 | 27.13 | B | O |
| ATOM | 4950 | N | ARG | B | 344 | 31.946 | 48.139 | 103.524 | 1.00 | 26.89 | B | N |
| ATOM | 4951 | CA | ARG | B | 344 | 31.328 | 48.074 | 104.841 | 1.00 | 27.93 | B | C |
| ATOM | 4952 | CB | ARG | B | 344 | 30.664 | 46.711 | 105.036 | 1.00 | 26.31 | B | C |
| ATOM | 4953 | CG | ARG | B | 344 | 29.413 | 46.527 | 104.186 | 1.00 | 23.72 | B | C |
| ATOM | 4954 | CD | ARG | B | 344 | 28.770 | 45.199 | 104.470 | 1.00 | 23.25 | B | C |
| ATOM | 4955 | NE | ARG | B | 344 | 29.419 | 44.113 | 103.745 | 1.00 | 22.03 | B | N |
| ATOM | 4956 | CZ | ARG | B | 344 | 29.141 | 43.787 | 102.488 | 1.00 | 21.32 | B | C |
| ATOM | 4957 | NH1 | ARG | B | 344 | 28.221 | 44.465 | 101.816 | 1.00 | 19.06 | B | N |
| ATOM | 4958 | NH2 | ARG | B | 344 | 29.780 | 42.780 | 101.905 | 1.00 | 19.94 | B | N |
| ATOM | 4959 | C | ARG | B | 344 | 32.308 | 48.359 | 105.977 | 1.00 | 29.92 | B | C |
| ATOM | 4960 | O | ARG | B | 344 | 31.938 | 48.359 | 107.145 | 1.00 | 31.20 | B | O |
| ATOM | 4961 | N | ASP | B | 345 | 33.561 | 48.608 | 105.621 | 1.00 | 32.06 | B | N |
| ATOM | 4962 | CA | ASP | B | 345 | 34.590 | 48.913 | 106.602 | 1.00 | 33.37 | B | C |
| ATOM | 4963 | CB | ASP | B | 345 | 35.970 | 48.721 | 105.967 | 1.00 | 34.50 | B | C |
| ATOM | 4964 | CG | ASP | B | 345 | 37.110 | 48.948 | 106.945 | 1.00 | 37.00 | B | C |
| ATOM | 4965 | OD1 | ASP | B | 345 | 37.911 | 48.011 | 107.147 | 1.00 | 39.20 | B | O |
| ATOM | 4966 | OD2 | ASP | B | 345 | 37.213 | 50.060 | 107.506 | 1.00 | 37.35 | B | O |
| ATOM | 4967 | C | ASP | B | 345 | 34.401 | 50.376 | 107.030 | 1.00 | 34.23 | B | C |
| ATOM | 4968 | O | ASP | B | 345 | 34.492 | 51.283 | 106.211 | 1.00 | 35.01 | B | O |
| ATOM | 4969 | N | PRO | B | 346 | 34.135 | 50.616 | 108.328 | 1.00 | 35.02 | B | N |
| ATOM | 4970 | CD | PRO | B | 346 | 34.152 | 49.641 | 109.431 | 1.00 | 34.65 | B | C |
| ATOM | 4971 | CA | PRO | B | 346 | 33.934 | 51.973 | 108.845 | 1.00 | 35.03 | B | C |
| ATOM | 4972 | CB | PRO | B | 346 | 33.859 | 51.767 | 110.357 | 1.00 | 34.47 | B | C |
| ATOM | 4973 | CG | PRO | B | 346 | 34.633 | 50.502 | 110.579 | 1.00 | 36.17 | B | C |
| ATOM | 4974 | C | PRO | B | 346 | 34.990 | 53.016 | 108.460 | 1.00 | 36.38 | B | C |
| ATOM | 4975 | O | PRO | B | 346 | 34.700 | 54.207 | 108.428 | 1.00 | 37.71 | B | O |
| ATOM | 4976 | N | ASN | B | 347 | 36.206 | 52.574 | 108.164 | 1.00 | 37.49 | B | N |
| ATOM | 4977 | CA | ASN | B | 347 | 37.289 | 53.491 | 107.810 | 1.00 | 37.99 | B | C |
| ATOM | 4978 | CB | ASN | B | 347 | 38.628 | 52.950 | 108.292 | 1.00 | 40.84 | B | C |
| ATOM | 4979 | CG | ASN | B | 347 | 38.721 | 52.868 | 109.786 | 1.00 | 42.67 | B | C |
| ATOM | 4980 | OD1 | ASN | B | 347 | 39.550 | 52.130 | 110.312 | 1.00 | 45.31 | B | O |
| ATOM | 4981 | ND2 | ASN | B | 347 | 37.881 | 53.633 | 110.489 | 1.00 | 43.33 | B | N |
| ATOM | 4982 | C | ASN | B | 347 | 37.438 | 53.731 | 106.317 | 1.00 | 38.00 | B | C |
| ATOM | 4983 | O | ASN | B | 347 | 38.305 | 54.489 | 105.898 | 1.00 | 38.30 | B | O |
| ATOM | 4984 | N | VAL | B | 348 | 36.613 | 53.076 | 105.511 | 1.00 | 36.49 | B | N |
| ATOM | 4985 | CA | VAL | B | 348 | 36.729 | 53.247 | 104.077 | 1.00 | 35.65 | B | C |
| ATOM | 4986 | CB | VAL | B | 348 | 35.689 | 52.400 | 103.312 | 1.00 | 35.36 | B | C |

| | | | | | | | | | | | | |
|---|---|---|---|---|---|---|---|---|---|---|---|---|
| ATOM | 4987 | CG1 | VAL | B | 348 | 34.289 | 52.982 | 103.483 | 1.00 | 35.20 | B | C |
| ATOM | 4988 | CG2 | VAL | B | 348 | 36.074 | 52.331 | 101.853 | 1.00 | 34.49 | B | C |
| ATOM | 4989 | C | VAL | B | 348 | 36.559 | 54.714 | 103.718 | 1.00 | 35.01 | B | C |
| ATOM | 4990 | O | VAL | B | 348 | 35.840 | 55.429 | 104.371 | 1.00 | 34.42 | B | O |
| ATOM | 4991 | N | LYS | B | 349 | 37.230 | 55.138 | 102.653 | 1.00 | 35.22 | B | N |
| ATOM | 4992 | CA | LYS | B | 349 | 37.184 | 56.522 | 102.213 | 1.00 | 35.87 | B | C |
| ATOM | 4993 | CB | LYS | B | 349 | 38.271 | 57.321 | 102.958 | 1.00 | 36.43 | B | C |
| ATOM | 4994 | CG | LYS | B | 349 | 37.848 | 58.637 | 103.631 | 1.00 | 38.79 | B | C |
| ATOM | 4995 | CD | LYS | B | 349 | 37.141 | 58.421 | 104.972 | 1.00 | 37.91 | B | C |
| ATOM | 4996 | CE | LYS | B | 349 | 36.972 | 59.741 | 105.735 | 1.00 | 39.57 | B | C |
| ATOM | 4997 | NZ | LYS | B | 349 | 36.133 | 59.596 | 106.970 | 1.00 | 38.40 | B | N |
| ATOM | 4998 | C | LYS | B | 349 | 37.512 | 56.523 | 100.714 | 1.00 | 36.55 | B | C |
| ATOM | 4999 | O | LYS | B | 349 | 38.168 | 55.617 | 100.213 | 1.00 | 36.18 | B | O |
| ATOM | 5000 | N | LEU | B | 350 | 37.063 | 57.547 | 100.002 | 1.00 | 37.03 | B | N |
| ATOM | 5001 | CA | LEU | B | 350 | 37.335 | 57.642 | 98.575 | 1.00 | 37.44 | B | C |
| ATOM | 5002 | CB | LEU | B | 350 | 36.346 | 58.614 | 97.929 | 1.00 | 34.68 | B | C |
| ATOM | 5003 | CG | LEU | B | 350 | 34.869 | 58.357 | 98.236 | 1.00 | 34.46 | B | C |
| ATOM | 5004 | CD1 | LEU | B | 350 | 34.047 | 59.542 | 97.794 | 1.00 | 33.31 | B | C |
| ATOM | 5005 | CD2 | LEU | B | 350 | 34.394 | 57.084 | 97.532 | 1.00 | 33.14 | B | C |
| ATOM | 5006 | C | LEU | B | 350 | 38.764 | 58.163 | 98.388 | 1.00 | 38.26 | B | C |
| ATOM | 5007 | O | LEU | B | 350 | 39.238 | 58.934 | 99.183 | 1.00 | 37.25 | B | O |
| ATOM | 5008 | N | PRO | B | 351 | 39.460 | 57.733 | 97.322 | 1.00 | 40.73 | B | N |
| ATOM | 5009 | CD | PRO | B | 351 | 38.953 | 57.021 | 96.136 | 1.00 | 41.70 | B | C |
| ATOM | 5010 | CA | PRO | B | 351 | 40.839 | 58.207 | 97.099 | 1.00 | 41.65 | B | C |
| ATOM | 5011 | CB | PRO | B | 351 | 41.206 | 57.590 | 95.751 | 1.00 | 42.14 | B | C |
| ATOM | 5012 | CG | PRO | B | 351 | 39.883 | 57.539 | 95.034 | 1.00 | 42.52 | B | C |
| ATOM | 5013 | C | PRO | B | 351 | 40.764 | 59.720 | 97.008 | 1.00 | 41.47 | B | C |
| ATOM | 5014 | O | PRO | B | 351 | 41.752 | 60.434 | 97.091 | 1.00 | 41.77 | B | O |
| ATOM | 5015 | N | ASN | B | 352 | 39.531 | 60.160 | 96.827 | 1.00 | 41.76 | B | N |
| ATOM | 5016 | CA | ASN | B | 352 | 39.124 | 61.548 | 96.722 | 1.00 | 42.17 | B | C |
| ATOM | 5017 | CB | ASN | B | 352 | 37.621 | 61.552 | 96.450 | 1.00 | 43.59 | B | C |
| ATOM | 5018 | CG | ASN | B | 352 | 37.140 | 62.825 | 95.814 | 1.00 | 43.75 | B | C |
| ATOM | 5019 | OD1 | ASN | B | 352 | 35.949 | 62.959 | 95.492 | 1.00 | 42.35 | B | O |
| ATOM | 5020 | ND2 | ASN | B | 352 | 38.055 | 63.771 | 95.612 | 1.00 | 44.16 | B | N |
| ATOM | 5021 | C | ASN | B | 352 | 39.376 | 62.245 | 98.052 | 1.00 | 41.52 | B | C |
| ATOM | 5022 | O | ASN | B | 352 | 39.534 | 63.459 | 98.117 | 1.00 | 40.01 | B | O |
| ATOM | 5023 | N | GLY | B | 353 | 39.366 | 61.446 | 99.114 | 1.00 | 41.86 | B | N |
| ATOM | 5024 | CA | GLY | B | 353 | 39.559 | 61.969 | 100.449 | 1.00 | 42.93 | B | C |
| ATOM | 5025 | C | GLY | B | 353 | 38.215 | 62.110 | 101.137 | 1.00 | 42.86 | B | C |
| ATOM | 5026 | O | GLY | B | 353 | 38.120 | 61.935 | 102.334 | 1.00 | 42.99 | B | O |
| ATOM | 5027 | N | ARG | B | 354 | 37.168 | 62.415 | 100.376 | 1.00 | 42.97 | B | N |
| ATOM | 5028 | CA | ARG | B | 354 | 35.856 | 62.565 | 100.984 | 1.00 | 43.17 | B | C |
| ATOM | 5029 | CB | ARG | B | 354 | 34.953 | 63.449 | 100.114 | 1.00 | 44.06 | B | C |
| ATOM | 5030 | CG | ARG | B | 354 | 34.672 | 62.961 | 98.717 | 1.00 | 45.60 | B | C |
| ATOM | 5031 | CD | ARG | B | 354 | 33.619 | 63.866 | 98.058 | 1.00 | 46.42 | B | C |
| ATOM | 5032 | NE | ARG | B | 354 | 34.141 | 65.173 | 97.653 | 1.00 | 47.22 | B | N |
| ATOM | 5033 | CZ | ARG | B | 354 | 33.388 | 66.143 | 97.136 | 1.00 | 48.28 | B | C |
| ATOM | 5034 | NH1 | ARG | B | 354 | 32.081 | 65.951 | 96.975 | 1.00 | 47.90 | B | N |
| ATOM | 5035 | NH2 | ARG | B | 354 | 33.937 | 67.293 | 96.753 | 1.00 | 47.14 | B | N |
| ATOM | 5036 | C | ARG | B | 354 | 35.190 | 61.230 | 101.325 | 1.00 | 42.28 | B | C |
| ATOM | 5037 | O | ARG | B | 354 | 35.658 | 60.174 | 100.916 | 1.00 | 43.09 | B | O |
| ATOM | 5038 | N | ASP | B | 355 | 34.118 | 61.283 | 102.108 | 1.00 | 41.26 | B | N |
| ATOM | 5039 | CA | ASP | B | 355 | 33.404 | 60.075 | 102.528 | 1.00 | 41.03 | B | C |
| ATOM | 5040 | CB | ASP | B | 355 | 32.408 | 60.410 | 103.640 | 1.00 | 43.37 | B | C |
| ATOM | 5041 | CG | ASP | B | 355 | 33.065 | 60.517 | 104.991 | 1.00 | 46.36 | B | C |
| ATOM | 5042 | OD1 | ASP | B | 355 | 32.442 | 61.101 | 105.914 | 1.00 | 48.01 | B | O |
| ATOM | 5043 | OD2 | ASP | B | 355 | 34.202 | 60.005 | 105.130 | 1.00 | 47.07 | B | O |
| ATOM | 5044 | C | ASP | B | 355 | 32.644 | 59.367 | 101.420 | 1.00 | 38.45 | B | C |
| ATOM | 5045 | O | ASP | B | 355 | 32.237 | 59.979 | 100.439 | 1.00 | 37.11 | B | O |
| ATOM | 5046 | N | THR | B | 356 | 32.446 | 58.066 | 101.605 | 1.00 | 35.96 | B | N |
| ATOM | 5047 | CA | THR | B | 356 | 31.696 | 57.280 | 100.639 | 1.00 | 34.21 | B | C |
| ATOM | 5048 | CB | THR | B | 356 | 31.758 | 55.766 | 100.979 | 1.00 | 34.60 | B | C |
| ATOM | 5049 | OG1 | THR | B | 356 | 31.431 | 55.565 | 102.362 | 1.00 | 35.17 | B | O |

| | | | | | | | | | | | | |
|---|---|---|---|---|---|---|---|---|---|---|---|---|
| ATOM | 5050 | CG2 | THR | B | 356 | 33.147 | 55.217 | 100.706 | 1.00 | 34.18 | B | C |
| ATOM | 5051 | C | THR | B | 356 | 30.248 | 57.754 | 100.713 | 1.00 | 32.15 | B | C |
| ATOM | 5052 | O | THR | B | 356 | 29.806 | 58.293 | 101.734 | 1.00 | 29.87 | B | O |
| ATOM | 5053 | N | PRO | B | 357 | 29.496 | 57.590 | 99.616 | 1.00 | 31.03 | B | N |
| ATOM | 5054 | CD | PRO | B | 357 | 29.884 | 57.011 | 98.316 | 1.00 | 30.30 | B | C |
| ATOM | 5055 | CA | PRO | B | 357 | 28.094 | 58.010 | 99.610 | 1.00 | 29.67 | B | C |
| ATOM | 5056 | CB | PRO | B | 357 | 27.729 | 57.945 | 98.134 | 1.00 | 29.34 | B | C |
| ATOM | 5057 | CG | PRO | B | 357 | 28.541 | 56.776 | 97.655 | 1.00 | 30.39 | B | C |
| ATOM | 5058 | C | PRO | B | 357 | 27.293 | 57.040 | 100.458 | 1.00 | 29.08 | B | C |
| ATOM | 5059 | O | PRO | B | 357 | 27.848 | 56.079 | 100.991 | 1.00 | 27.86 | B | O |
| ATOM | 5060 | N | ALA | B | 358 | 25.996 | 57.296 | 100.584 | 1.00 | 30.37 | B | N |
| ATOM | 5061 | CA | ALA | B | 358 | 25.110 | 56.429 | 101.362 | 1.00 | 31.52 | B | C |
| ATOM | 5062 | CB | ALA | B | 358 | 23.707 | 57.034 | 101.403 | 1.00 | 32.97 | B | C |
| ATOM | 5063 | C | ALA | B | 358 | 25.076 | 55.049 | 100.705 | 1.00 | 29.74 | B | C |
| ATOM | 5064 | O | ALA | B | 358 | 24.822 | 54.928 | 99.511 | 1.00 | 31.37 | B | O |
| ATOM | 5065 | N | LEU | B | 359 | 25.337 | 54.021 | 101.503 | 1.00 | 26.93 | B | N |
| ATOM | 5066 | CA | LEU | B | 359 | 25.370 | 52.655 | 101.023 | 1.00 | 25.83 | B | C |
| ATOM | 5067 | CB | LEU | B | 359 | 26.815 | 52.147 | 101.066 | 1.00 | 24.76 | B | C |
| ATOM | 5068 | CG | LEU | B | 359 | 27.762 | 52.315 | 99.868 | 1.00 | 26.91 | B | C |
| ATOM | 5069 | CD1 | LEU | B | 359 | 27.324 | 53.444 | 98.954 | 1.00 | 24.36 | B | C |
| ATOM | 5070 | CD2 | LEU | B | 359 | 29.178 | 52.537 | 100.400 | 1.00 | 23.98 | B | C |
| ATOM | 5071 | C | LEU | B | 359 | 24.484 | 51.732 | 101.845 | 1.00 | 24.60 | B | C |
| ATOM | 5072 | O | LEU | B | 359 | 24.112 | 50.693 | 101.385 | 1.00 | 23.07 | B | O |
| ATOM | 5073 | N | PHE | B | 360 | 24.134 | 52.149 | 103.058 | 1.00 | 24.60 | B | N |
| ATOM | 5074 | CA | PHE | B | 360 | 23.349 | 51.291 | 103.945 | 1.00 | 25.30 | B | C |
| ATOM | 5075 | CB | PHE | B | 360 | 24.167 | 51.046 | 105.204 | 1.00 | 25.56 | B | C |
| ATOM | 5076 | CG | PHE | B | 360 | 25.644 | 51.017 | 104.946 | 1.00 | 26.58 | B | C |
| ATOM | 5077 | CD1 | PHE | B | 360 | 26.204 | 50.028 | 104.143 | 1.00 | 27.01 | B | C |
| ATOM | 5078 | CD2 | PHE | B | 360 | 26.467 | 52.013 | 105.456 | 1.00 | 26.20 | B | C |
| ATOM | 5079 | CE1 | PHE | B | 360 | 27.562 | 50.036 | 103.848 | 1.00 | 27.42 | B | C |
| ATOM | 5080 | CE2 | PHE | B | 360 | 27.824 | 52.032 | 105.168 | 1.00 | 26.38 | B | C |
| ATOM | 5081 | CZ | PHE | B | 360 | 28.372 | 51.042 | 104.363 | 1.00 | 28.10 | B | C |
| ATOM | 5082 | C | PHE | B | 360 | 21.943 | 51.750 | 104.318 | 1.00 | 24.82 | B | C |
| ATOM | 5083 | O | PHE | B | 360 | 21.321 | 51.149 | 105.181 | 1.00 | 26.12 | B | O |
| ATOM | 5084 | N | ASN | B | 361 | 21.435 | 52.794 | 103.670 | 1.00 | 24.33 | B | N |
| ATOM | 5085 | CA | ASN | B | 361 | 20.089 | 53.273 | 103.978 | 1.00 | 24.98 | B | C |
| ATOM | 5086 | CB | ASN | B | 361 | 19.893 | 54.716 | 103.474 | 1.00 | 26.58 | B | C |
| ATOM | 5087 | CG | ASN | B | 361 | 20.321 | 54.901 | 102.024 | 1.00 | 29.42 | B | C |
| ATOM | 5088 | OD1 | ASN | B | 361 | 20.132 | 54.039 | 101.196 | 1.00 | 32.21 | B | O |
| ATOM | 5089 | ND2 | ASN | B | 361 | 20.889 | 56.055 | 101.724 | 1.00 | 33.29 | B | N |
| ATOM | 5090 | C | ASN | B | 361 | 19.029 | 52.364 | 103.344 | 1.00 | 24.38 | B | C |
| ATOM | 5091 | O | ASN | B | 361 | 18.175 | 52.813 | 102.599 | 1.00 | 23.67 | B | O |
| ATOM | 5092 | N | PHE | B | 362 | 19.098 | 51.077 | 103.645 | 1.00 | 24.57 | B | N |
| ATOM | 5093 | CA | PHE | B | 362 | 18.140 | 50.129 | 103.093 | 1.00 | 24.89 | B | C |
| ATOM | 5094 | CB | PHE | B | 362 | 18.609 | 48.700 | 103.375 | 1.00 | 23.91 | B | C |
| ATOM | 5095 | CG | PHE | B | 362 | 19.772 | 48.253 | 102.528 | 1.00 | 25.46 | B | C |
| ATOM | 5096 | CD1 | PHE | B | 362 | 19.565 | 47.762 | 101.236 | 1.00 | 26.59 | B | C |
| ATOM | 5097 | CD2 | PHE | B | 362 | 21.072 | 48.295 | 103.025 | 1.00 | 24.87 | B | C |
| ATOM | 5098 | CE1 | PHE | B | 362 | 20.635 | 47.314 | 100.451 | 1.00 | 23.17 | B | C |
| ATOM | 5099 | CE2 | PHE | B | 362 | 22.150 | 47.852 | 102.249 | 1.00 | 25.96 | B | C |
| ATOM | 5100 | CZ | PHE | B | 362 | 21.927 | 47.357 | 100.957 | 1.00 | 23.58 | B | C |
| ATOM | 5101 | C | PHE | B | 362 | 16.740 | 50.325 | 103.689 | 1.00 | 24.67 | B | C |
| ATOM | 5102 | O | PHE | B | 362 | 16.610 | 50.616 | 104.871 | 1.00 | 22.10 | B | O |
| ATOM | 5103 | N | THR | B | 363 | 15.704 | 50.190 | 102.860 | 1.00 | 24.90 | B | N |
| ATOM | 5104 | CA | THR | B | 363 | 14.323 | 50.283 | 103.352 | 1.00 | 26.09 | B | C |
| ATOM | 5105 | CB | THR | B | 363 | 13.389 | 51.066 | 102.402 | 1.00 | 24.99 | B | C |
| ATOM | 5106 | OG1 | THR | B | 363 | 13.351 | 50.416 | 101.128 | 1.00 | 25.92 | B | O |
| ATOM | 5107 | CG2 | THR | B | 363 | 13.856 | 52.499 | 102.241 | 1.00 | 22.62 | B | C |
| ATOM | 5108 | C | THR | B | 363 | 13.823 | 48.839 | 103.413 | 1.00 | 27.30 | B | C |
| ATOM | 5109 | O | THR | B | 363 | 14.477 | 47.933 | 102.875 | 1.00 | 25.90 | B | O |
| ATOM | 5110 | N | THR | B | 364 | 12.678 | 48.608 | 104.052 | 1.00 | 28.13 | B | N |
| ATOM | 5111 | CA | THR | B | 364 | 12.165 | 47.244 | 104.138 | 1.00 | 29.66 | B | C |
| ATOM | 5112 | CB | THR | B | 364 | 11.015 | 47.095 | 105.181 | 1.00 | 30.27 | B | C |

| | | | | | | | | | | | | |
|---|---|---|---|---|---|---|---|---|---|---|---|---|
| ATOM | 5113 | OG1 | THR | B | 364 | 9.826 | 47.707 | 104.679 | 1.00 | 33.06 | B | O |
| ATOM | 5114 | CG2 | THR | B | 364 | 11.392 | 47.751 | 106.512 | 1.00 | 31.83 | B | C |
| ATOM | 5115 | C | THR | B | 364 | 11.657 | 46.779 | 102.769 | 1.00 | 29.64 | B | C |
| ATOM | 5116 | O | THR | B | 364 | 11.570 | 45.588 | 102.499 | 1.00 | 31.36 | B | O |
| ATOM | 5117 | N | GLN | B | 365 | 11.332 | 47.725 | 101.901 | 1.00 | 28.47 | B | N |
| ATOM | 5118 | CA | GLN | B | 365 | 10.855 | 47.369 | 100.580 | 1.00 | 27.36 | B | C |
| ATOM | 5119 | CB | GLN | B | 365 | 10.264 | 48.606 | 99.902 | 1.00 | 27.42 | B | C |
| ATOM | 5120 | CG | GLN | B | 365 | 8.982 | 48.316 | 99.143 | 1.00 | 32.91 | B | C |
| ATOM | 5121 | CD | GLN | B | 365 | 9.268 | 47.680 | 97.819 | 1.00 | 31.95 | B | C |
| ATOM | 5122 | OE1 | GLN | B | 365 | 8.544 | 46.825 | 97.353 | 1.00 | 30.31 | B | O |
| ATOM | 5123 | NE2 | GLN | B | 365 | 10.348 | 48.121 | 97.197 | 1.00 | 35.72 | B | N |
| ATOM | 5124 | C | GLN | B | 365 | 12.049 | 46.802 | 99.805 | 1.00 | 26.95 | B | C |
| ATOM | 5125 | O | GLN | B | 365 | 11.947 | 45.781 | 99.112 | 1.00 | 23.62 | B | O |
| ATOM | 5126 | N | GLU | B | 366 | 13.193 | 47.458 | 99.987 | 1.00 | 26.52 | B | N |
| ATOM | 5127 | CA | GLU | B | 366 | 14.450 | 47.098 | 99.348 | 1.00 | 25.70 | B | C |
| ATOM | 5128 | CB | GLU | B | 366 | 15.466 | 48.223 | 99.586 | 1.00 | 27.11 | B | C |
| ATOM | 5129 | CG | GLU | B | 366 | 16.788 | 48.058 | 98.872 | 1.00 | 28.21 | B | C |
| ATOM | 5130 | CD | GLU | B | 366 | 17.615 | 49.340 | 98.857 | 1.00 | 29.14 | B | C |
| ATOM | 5131 | OE1 | GLU | B | 366 | 17.233 | 50.317 | 99.535 | 1.00 | 27.79 | B | O |
| ATOM | 5132 | OE2 | GLU | B | 366 | 18.653 | 49.367 | 98.162 | 1.00 | 29.80 | B | O |
| ATOM | 5133 | C | GLU | B | 366 | 15.009 | 45.768 | 99.836 | 1.00 | 24.99 | B | C |
| ATOM | 5134 | O | GLU | B | 366 | 15.732 | 45.112 | 99.122 | 1.00 | 24.61 | B | O |
| ATOM | 5135 | N | LEU | B | 367 | 14.649 | 45.364 | 101.050 | 1.00 | 25.72 | B | N |
| ATOM | 5136 | CA | LEU | B | 367 | 15.152 | 44.111 | 101.606 | 1.00 | 25.96 | B | C |
| ATOM | 5137 | CB | LEU | B | 367 | 15.595 | 44.340 | 103.058 | 1.00 | 26.67 | B | C |
| ATOM | 5138 | CG | LEU | B | 367 | 16.763 | 45.311 | 103.276 | 1.00 | 26.61 | B | C |
| ATOM | 5139 | CD1 | LEU | B | 367 | 16.788 | 45.775 | 104.726 | 1.00 | 26.07 | B | C |
| ATOM | 5140 | CD2 | LEU | B | 367 | 18.070 | 44.630 | 102.903 | 1.00 | 26.33 | B | C |
| ATOM | 5141 | C | LEU | B | 367 | 14.120 | 42.995 | 101.553 | 1.00 | 26.19 | B | C |
| ATOM | 5142 | O | LEU | B | 367 | 14.427 | 41.856 | 101.882 | 1.00 | 27.97 | B | O |
| ATOM | 5143 | N | SER | B | 368 | 12.912 | 43.328 | 101.110 | 1.00 | 25.96 | B | N |
| ATOM | 5144 | CA | SER | B | 368 | 11.806 | 42.371 | 101.055 | 1.00 | 27.28 | B | C |
| ATOM | 5145 | CB | SER | B | 368 | 10.609 | 42.982 | 100.300 | 1.00 | 28.01 | B | C |
| ATOM | 5146 | OG | SER | B | 368 | 10.872 | 43.170 | 98.914 | 1.00 | 28.25 | B | O |
| ATOM | 5147 | C | SER | B | 368 | 12.098 | 40.979 | 100.489 | 1.00 | 27.62 | B | C |
| ATOM | 5148 | O | SER | B | 368 | 11.494 | 39.996 | 100.920 | 1.00 | 27.98 | B | O |
| ATOM | 5149 | N | SER | B | 369 | 13.020 | 40.874 | 99.542 | 1.00 | 27.00 | B | N |
| ATOM | 5150 | CA | SER | B | 369 | 13.302 | 39.568 | 98.966 | 1.00 | 27.25 | B | C |
| ATOM | 5151 | CB | SER | B | 369 | 14.050 | 39.694 | 97.643 | 1.00 | 25.41 | B | C |
| ATOM | 5152 | OG | SER | B | 369 | 15.442 | 39.664 | 97.860 | 1.00 | 25.22 | B | O |
| ATOM | 5153 | C | SER | B | 369 | 14.099 | 38.647 | 99.893 | 1.00 | 28.69 | B | C |
| ATOM | 5154 | O | SER | B | 369 | 14.191 | 37.442 | 99.643 | 1.00 | 29.05 | B | O |
| ATOM | 5155 | N | ASN | B | 370 | 14.680 | 39.202 | 100.955 | 1.00 | 28.49 | B | N |
| ATOM | 5156 | CA | ASN | B | 370 | 15.463 | 38.391 | 101.890 | 1.00 | 27.75 | B | C |
| ATOM | 5157 | CB | ASN | B | 370 | 16.702 | 37.837 | 101.174 | 1.00 | 27.25 | B | C |
| ATOM | 5158 | CG | ASN | B | 370 | 17.373 | 36.695 | 101.936 | 1.00 | 27.50 | B | C |
| ATOM | 5159 | OD1 | ASN | B | 370 | 18.102 | 35.887 | 101.348 | 1.00 | 24.58 | B | O |
| ATOM | 5160 | ND2 | ASN | B | 370 | 17.139 | 36.630 | 103.240 | 1.00 | 27.81 | B | N |
| ATOM | 5161 | C | ASN | B | 370 | 15.876 | 39.250 | 103.091 | 1.00 | 28.17 | B | C |
| ATOM | 5162 | O | ASN | B | 370 | 17.046 | 39.565 | 103.268 | 1.00 | 27.11 | B | O |
| ATOM | 5163 | N | PRO | B | 371 | 14.898 | 39.622 | 103.936 | 1.00 | 28.17 | B | N |
| ATOM | 5164 | CD | PRO | B | 371 | 13.508 | 39.133 | 103.858 | 1.00 | 27.14 | B | C |
| ATOM | 5165 | CA | PRO | B | 371 | 15.088 | 40.449 | 105.135 | 1.00 | 28.11 | B | C |
| ATOM | 5166 | CB | PRO | B | 371 | 13.794 | 40.231 | 105.914 | 1.00 | 27.45 | B | C |
| ATOM | 5167 | CG | PRO | B | 371 | 12.785 | 40.048 | 104.819 | 1.00 | 28.42 | B | C |
| ATOM | 5168 | C | PRO | B | 371 | 16.320 | 40.125 | 105.980 | 1.00 | 27.42 | B | C |
| ATOM | 5169 | O | PRO | B | 371 | 17.088 | 41.018 | 106.313 | 1.00 | 26.80 | B | O |
| ATOM | 5170 | N | PRO | B | 372 | 16.519 | 38.844 | 106.345 | 1.00 | 27.69 | B | N |
| ATOM | 5171 | CD | PRO | B | 372 | 15.664 | 37.656 | 106.189 | 1.00 | 28.66 | B | C |
| ATOM | 5172 | CA | PRO | B | 372 | 17.696 | 38.530 | 107.156 | 1.00 | 28.34 | B | C |
| ATOM | 5173 | CB | PRO | B | 372 | 17.653 | 37.000 | 107.284 | 1.00 | 28.21 | B | C |
| ATOM | 5174 | CG | PRO | B | 372 | 16.666 | 36.555 | 106.233 | 1.00 | 29.29 | B | C |
| ATOM | 5175 | C | PRO | B | 372 | 19.033 | 39.050 | 106.625 | 1.00 | 28.31 | B | C |

| | | | | | | | | | | | | |
|---|---|---|---|---|---|---|---|---|---|---|---|---|
| ATOM | 5176 | O | PRO | B | 372 | 19.964 | 39.261 | 107.389 | 1.00 | 28.76 | B | O |
| ATOM | 5177 | N | LEU | B | 373 | 19.117 | 39.270 | 105.316 | 1.00 | 27.97 | B | N |
| ATOM | 5178 | CA | LEU | B | 373 | 20.347 | 39.768 | 104.710 | 1.00 | 27.53 | B | C |
| ATOM | 5179 | CB | LEU | B | 373 | 20.142 | 39.967 | 103.202 | 1.00 | 26.84 | B | C |
| ATOM | 5180 | CG | LEU | B | 373 | 21.005 | 39.192 | 102.188 | 1.00 | 28.15 | B | C |
| ATOM | 5181 | CD1 | LEU | B | 373 | 21.285 | 37.768 | 102.665 | 1.00 | 27.32 | B | C |
| ATOM | 5182 | CD2 | LEU | B | 373 | 20.286 | 39.180 | 100.841 | 1.00 | 26.41 | B | C |
| ATOM | 5183 | C | LEU | B | 373 | 20.759 | 41.097 | 105.372 | 1.00 | 27.64 | B | C |
| ATOM | 5184 | O | LEU | B | 373 | 21.931 | 41.484 | 105.351 | 1.00 | 26.89 | B | O |
| ATOM | 5185 | N | ALA | B | 374 | 19.798 | 41.798 | 105.964 | 1.00 | 26.31 | B | N |
| ATOM | 5186 | CA | ALA | B | 374 | 20.121 | 43.056 | 106.619 | 1.00 | 28.47 | B | C |
| ATOM | 5187 | CB | ALA | B | 374 | 18.905 | 43.588 | 107.365 | 1.00 | 26.79 | B | C |
| ATOM | 5188 | C | ALA | B | 374 | 21.300 | 42.911 | 107.592 | 1.00 | 28.95 | B | C |
| ATOM | 5189 | O | ALA | B | 374 | 22.063 | 43.845 | 107.786 | 1.00 | 27.39 | B | O |
| ATOM | 5190 | N | THR | B | 375 | 21.441 | 41.730 | 108.192 | 1.00 | 29.88 | B | N |
| ATOM | 5191 | CA | THR | B | 375 | 22.528 | 41.479 | 109.132 | 1.00 | 32.45 | B | C |
| ATOM | 5192 | CB | THR | B | 375 | 22.586 | 40.014 | 109.590 | 1.00 | 33.38 | B | C |
| ATOM | 5193 | OG1 | THR | B | 375 | 21.345 | 39.652 | 110.200 | 1.00 | 37.85 | B | O |
| ATOM | 5194 | CG2 | THR | B | 375 | 23.698 | 39.830 | 110.603 | 1.00 | 36.44 | B | C |
| ATOM | 5195 | C | THR | B | 375 | 23.889 | 41.814 | 108.533 | 1.00 | 32.33 | B | C |
| ATOM | 5196 | O | THR | B | 375 | 24.785 | 42.239 | 109.234 | 1.00 | 32.47 | B | O |
| ATOM | 5197 | N | ILE | B | 376 | 24.033 | 41.598 | 107.231 | 1.00 | 33.08 | B | N |
| ATOM | 5198 | CA | ILE | B | 376 | 25.286 | 41.880 | 106.548 | 1.00 | 32.97 | B | C |
| ATOM | 5199 | CB | ILE | B | 376 | 25.568 | 40.859 | 105.446 | 1.00 | 33.68 | B | C |
| ATOM | 5200 | CG2 | ILE | B | 376 | 26.826 | 41.270 | 104.674 | 1.00 | 33.49 | B | C |
| ATOM | 5201 | CG1 | ILE | B | 376 | 25.716 | 39.468 | 106.064 | 1.00 | 34.23 | B | C |
| ATOM | 5202 | CD1 | ILE | B | 376 | 25.820 | 38.355 | 105.040 | 1.00 | 34.11 | B | C |
| ATOM | 5203 | C | ILE | B | 376 | 25.260 | 43.266 | 105.913 | 1.00 | 32.85 | B | C |
| ATOM | 5204 | O | ILE | B | 376 | 26.180 | 44.074 | 106.102 | 1.00 | 34.10 | B | O |
| ATOM | 5205 | N | LEU | B | 377 | 24.201 | 43.535 | 105.161 | 1.00 | 31.94 | B | N |
| ATOM | 5206 | CA | LEU | B | 377 | 24.046 | 44.809 | 104.470 | 1.00 | 30.79 | B | C |
| ATOM | 5207 | CB | LEU | B | 377 | 22.672 | 44.891 | 103.795 | 1.00 | 28.09 | B | C |
| ATOM | 5208 | CG | LEU | B | 377 | 22.523 | 44.098 | 102.494 | 1.00 | 27.11 | B | C |
| ATOM | 5209 | CD1 | LEU | B | 377 | 23.825 | 44.177 | 101.695 | 1.00 | 24.92 | B | C |
| ATOM | 5210 | CD2 | LEU | B | 377 | 22.207 | 42.677 | 102.796 | 1.00 | 27.01 | B | C |
| ATOM | 5211 | C | LEU | B | 377 | 24.235 | 46.043 | 105.333 | 1.00 | 30.37 | B | C |
| ATOM | 5212 | O | LEU | B | 377 | 24.834 | 47.019 | 104.900 | 1.00 | 28.54 | B | O |
| ATOM | 5213 | N | ILE | B | 378 | 23.691 | 46.005 | 106.545 | 1.00 | 31.01 | B | N |
| ATOM | 5214 | CA | ILE | B | 378 | 23.820 | 47.129 | 107.458 | 1.00 | 30.89 | B | C |
| ATOM | 5215 | CB | ILE | B | 378 | 22.479 | 47.463 | 108.123 | 1.00 | 29.77 | B | C |
| ATOM | 5216 | CG2 | ILE | B | 378 | 22.636 | 48.671 | 109.028 | 1.00 | 30.86 | B | C |
| ATOM | 5217 | CG1 | ILE | B | 378 | 21.444 | 47.795 | 107.048 | 1.00 | 30.55 | B | C |
| ATOM | 5218 | CD1 | ILE | B | 378 | 20.063 | 48.086 | 107.589 | 1.00 | 30.68 | B | C |
| ATOM | 5219 | C | ILE | B | 378 | 24.851 | 46.779 | 108.512 | 1.00 | 31.39 | B | C |
| ATOM | 5220 | O | ILE | B | 378 | 24.567 | 46.078 | 109.450 | 1.00 | 31.67 | B | O |
| ATOM | 5221 | N | PRO | B | 379 | 26.082 | 47.274 | 108.341 | 1.00 | 32.55 | B | N |
| ATOM | 5222 | CD | PRO | B | 379 | 26.499 | 48.217 | 107.288 | 1.00 | 32.85 | B | C |
| ATOM | 5223 | CA | PRO | B | 379 | 27.182 | 47.007 | 109.275 | 1.00 | 33.33 | B | C |
| ATOM | 5224 | CB | PRO | B | 379 | 28.396 | 47.530 | 108.521 | 1.00 | 32.72 | B | C |
| ATOM | 5225 | CG | PRO | B | 379 | 27.836 | 48.719 | 107.808 | 1.00 | 33.74 | B | C |
| ATOM | 5226 | C | PRO | B | 379 | 26.994 | 47.673 | 110.637 | 1.00 | 34.29 | B | C |
| ATOM | 5227 | O | PRO | B | 379 | 26.373 | 48.741 | 110.751 | 1.00 | 33.62 | B | O |
| ATOM | 5228 | N | PRO | B | 380 | 27.552 | 47.055 | 111.689 | 1.00 | 35.13 | B | N |
| ATOM | 5229 | CD | PRO | B | 380 | 28.513 | 45.938 | 111.615 | 1.00 | 35.49 | B | C |
| ATOM | 5230 | CA | PRO | B | 380 | 27.459 | 47.558 | 113.058 | 1.00 | 35.71 | B | C |
| ATOM | 5231 | CB | PRO | B | 380 | 28.649 | 46.897 | 113.741 | 1.00 | 36.44 | B | C |
| ATOM | 5232 | CG | PRO | B | 380 | 28.686 | 45.556 | 113.072 | 1.00 | 35.68 | B | C |
| ATOM | 5233 | C | PRO | B | 380 | 27.478 | 49.089 | 113.194 | 1.00 | 36.41 | B | C |
| ATOM | 5234 | O | PRO | B | 380 | 26.595 | 49.674 | 113.831 | 1.00 | 35.02 | B | O |
| ATOM | 5235 | N | HIS | B | 381 | 28.458 | 49.732 | 112.565 | 1.00 | 36.09 | B | N |
| ATOM | 5236 | CA | HIS | B | 381 | 28.629 | 51.183 | 112.681 | 1.00 | 37.29 | B | C |
| ATOM | 5237 | CB | HIS | B | 381 | 30.038 | 51.556 | 112.213 | 1.00 | 37.38 | B | C |
| ATOM | 5238 | CG | HIS | B | 381 | 30.283 | 51.284 | 110.759 | 1.00 | 38.62 | B | C |

| | | | | | | | | | | | | |
|---|---|---|---|---|---|---|---|---|---|---|---|---|
| ATOM | 5239 | CD2 | HIS | B | 381 | 30.608 | 50.140 | 110.109 | 1.00 | 38.36 | B | C |
| ATOM | 5240 | ND1 | HIS | B | 381 | 30.183 | 52.260 | 109.790 | 1.00 | 37.99 | B | N |
| ATOM | 5241 | CE1 | HIS | B | 381 | 30.438 | 51.731 | 108.605 | 1.00 | 36.87 | B | C |
| ATOM | 5242 | NE2 | HIS | B | 381 | 30.699 | 50.447 | 108.772 | 1.00 | 37.78 | B | N |
| ATOM | 5243 | C | HIS | B | 381 | 27.640 | 52.190 | 112.062 | 1.00 | 38.44 | B | C |
| ATOM | 5244 | O | HIS | B | 381 | 27.429 | 53.256 | 112.627 | 1.00 | 38.56 | B | O |
| ATOM | 5245 | N | ALA | B | 382 | 27.025 | 51.881 | 110.926 | 1.00 | 40.21 | B | N |
| ATOM | 5246 | CA | ALA | B | 382 | 26.119 | 52.857 | 110.309 | 1.00 | 42.27 | B | C |
| ATOM | 5247 | CB | ALA | B | 382 | 25.947 | 52.537 | 108.818 | 1.00 | 43.33 | B | C |
| ATOM | 5248 | C | ALA | B | 382 | 24.748 | 52.998 | 110.972 | 1.00 | 42.94 | B | C |
| ATOM | 5249 | O | ALA | B | 382 | 23.915 | 53.783 | 110.455 | 1.00 | 43.42 | B | O |
| ATOM | 5250 | OXT | ALA | B | 382 | 24.526 | 52.334 | 112.008 | 1.00 | 45.84 | B | O |
| TER | 5251 | | ALA | B | 382 | | | | | | B | |
| ATOM | 5252 | O | HOH | W | 1 | 20.170 | 40.353 | 79.716 | 1.00 | 16.22 | W | O |
| ATOM | 5253 | O | HOH | W | 2 | 21.694 | 40.432 | 33.508 | 1.00 | 18.07 | W | O |
| ATOM | 5254 | O | HOH | W | 3 | 18.991 | 26.492 | 62.341 | 1.00 | 16.94 | W | O |
| ATOM | 5255 | O | HOH | W | 4 | 16.128 | 39.802 | 91.548 | 1.00 | 23.87 | W | O |
| ATOM | 5256 | O | HOH | W | 5 | 19.619 | 43.910 | 81.460 | 1.00 | 18.68 | W | O |
| ATOM | 5257 | O | HOH | W | 6 | 26.256 | 38.744 | 77.201 | 1.00 | 19.01 | W | O |
| ATOM | 5258 | O | HOH | W | 7 | 22.022 | 36.866 | 55.577 | 1.00 | 17.22 | W | O |
| ATOM | 5259 | O | HOH | W | 8 | 20.330 | 43.777 | 62.453 | 1.00 | 19.57 | W | O |
| ATOM | 5260 | O | HOH | W | 9 | 7.721 | 42.017 | 76.700 | 1.00 | 21.49 | W | O |
| ATOM | 5261 | O | HOH | W | 10 | 22.350 | 37.509 | 65.625 | 1.00 | 15.73 | W | O |
| ATOM | 5262 | O | HOH | W | 11 | 5.141 | 47.547 | 83.429 | 1.00 | 22.45 | W | O |
| ATOM | 5263 | O | HOH | W | 12 | 23.883 | 52.889 | 97.574 | 1.00 | 18.56 | W | O |
| ATOM | 5264 | O | HOH | W | 13 | 38.167 | 50.269 | 81.471 | 1.00 | 20.23 | W | O |
| ATOM | 5265 | O | HOH | W | 14 | 18.785 | 38.566 | 57.421 | 1.00 | 18.92 | W | O |
| ATOM | 5266 | O | HOH | W | 15 | 18.628 | 34.067 | 89.684 | 1.00 | 18.94 | W | O |
| ATOM | 5267 | O | HOH | W | 16 | 27.656 | 35.217 | 62.487 | 1.00 | 20.76 | W | O |
| ATOM | 5268 | O | HOH | W | 17 | 14.756 | 50.177 | 80.590 | 1.00 | 16.02 | W | O |
| ATOM | 5269 | O | HOH | W | 18 | 14.319 | 56.748 | 82.076 | 1.00 | 18.50 | W | O |
| ATOM | 5270 | O | HOH | W | 19 | 31.278 | 38.735 | 65.562 | 1.00 | 23.82 | W | O |
| ATOM | 5271 | O | HOH | W | 20 | 30.303 | 41.113 | 77.352 | 1.00 | 20.28 | W | O |
| ATOM | 5272 | O | HOH | W | 21 | 37.674 | 36.510 | 59.142 | 1.00 | 15.70 | W | O |
| ATOM | 5273 | O | HOH | W | 22 | 36.720 | 42.940 | 55.960 | 1.00 | 22.03 | W | O |
| ATOM | 5274 | O | HOH | W | 23 | 19.248 | 42.101 | 66.364 | 1.00 | 23.60 | W | O |
| ATOM | 5275 | O | HOH | W | 24 | 25.184 | 57.421 | 78.018 | 1.00 | 20.72 | W | O |
| ATOM | 5276 | O | HOH | W | 25 | 26.186 | 46.519 | 102.789 | 1.00 | 19.56 | W | O |
| ATOM | 5277 | O | HOH | W | 26 | 13.488 | 57.294 | 90.029 | 1.00 | 22.16 | W | O |
| ATOM | 5278 | O | HOH | W | 27 | 23.467 | 20.728 | 51.526 | 1.00 | 29.77 | W | O |
| ATOM | 5279 | O | HOH | W | 28 | 12.329 | 34.775 | 55.691 | 1.00 | 23.91 | W | O |
| ATOM | 5280 | O | HOH | W | 29 | 34.706 | 37.587 | 57.062 | 1.00 | 14.54 | W | O |
| ATOM | 5281 | O | HOH | W | 30 | 48.261 | 26.008 | 58.716 | 1.00 | 34.91 | W | O |
| ATOM | 5282 | O | HOH | W | 32 | 21.261 | 51.525 | 69.728 | 1.00 | 24.43 | W | O |
| ATOM | 5283 | O | HOH | W | 33 | 22.268 | 38.919 | 70.367 | 1.00 | 26.10 | W | O |
| ATOM | 5284 | O | HOH | W | 34 | 32.508 | 25.192 | 60.102 | 1.00 | 26.27 | W | O |
| ATOM | 5285 | O | HOH | W | 35 | 35.879 | 44.067 | 99.705 | 1.00 | 25.28 | W | O |
| ATOM | 5286 | O | HOH | W | 36 | 17.788 | 41.357 | 68.938 | 1.00 | 17.26 | W | O |
| ATOM | 5287 | O | HOH | W | 37 | 12.366 | 70.858 | 75.814 | 1.00 | 42.21 | W | O |
| ATOM | 5288 | O | HOH | W | 38 | 12.992 | 55.584 | 76.464 | 1.00 | 27.43 | W | O |
| ATOM | 5289 | O | HOH | W | 39 | 28.573 | 37.205 | 38.001 | 1.00 | 19.37 | W | O |
| ATOM | 5290 | O | HOH | W | 40 | 15.855 | 34.842 | 82.986 | 1.00 | 22.08 | W | O |
| ATOM | 5291 | O | HOH | W | 41 | 3.098 | 77.686 | 89.384 | 1.00 | 34.74 | W | O |
| ATOM | 5292 | O | HOH | W | 42 | 9.951 | 48.891 | 50.913 | 1.00 | 28.22 | W | O |
| ATOM | 5293 | O | HOH | W | 43 | 3.758 | 49.864 | 86.297 | 1.00 | 48.04 | W | O |
| ATOM | 5294 | O | HOH | W | 44 | 24.704 | 25.652 | 87.767 | 1.00 | 39.40 | W | O |
| ATOM | 5295 | O | HOH | W | 45 | 18.394 | 45.159 | 59.331 | 1.00 | 23.94 | W | O |
| ATOM | 5296 | O | HOH | W | 46 | 12.921 | 42.159 | 38.628 | 1.00 | 23.08 | W | O |
| ATOM | 5297 | O | HOH | W | 47 | 22.591 | 22.024 | 55.775 | 1.00 | 21.24 | W | O |
| ATOM | 5298 | O | HOH | W | 48 | 36.386 | 40.253 | 56.603 | 1.00 | 23.35 | W | O |
| ATOM | 5299 | O | HOH | W | 49 | 24.318 | 54.904 | 104.654 | 1.00 | 22.79 | W | O |
| ATOM | 5300 | O | HOH | W | 50 | 27.230 | 38.495 | 74.285 | 1.00 | 23.18 | W | O |
| ATOM | 5301 | O | HOH | W | 51 | 30.150 | 47.609 | 56.541 | 1.00 | 33.23 | W | O |

| | | | | | | | | | | | | |
|---|---|---|---|---|---|---|---|---|---|---|---|---|
| ATOM | 5302 | O | HOH | W | 52 | 31.107 | 43.072 | 79.230 | 1.00 | 20.70 | W | O |
| ATOM | 5303 | O | HOH | W | 53 | 18.270 | 59.794 | 92.761 | 1.00 | 25.35 | W | O |
| ATOM | 5304 | O | HOH | W | 54 | 17.789 | 48.989 | 73.761 | 1.00 | 17.16 | W | O |
| ATOM | 5305 | O | HOH | W | 55 | 22.809 | 42.920 | 23.972 | 1.00 | 38.67 | W | O |
| ATOM | 5306 | O | HOH | W | 56 | 28.490 | 33.149 | 59.393 | 1.00 | 23.17 | W | O |
| ATOM | 5307 | O | HOH | W | 57 | 29.607 | 51.883 | 55.707 | 1.00 | 31.48 | W | O |
| ATOM | 5308 | O | HOH | W | 58 | 33.481 | 52.076 | 77.290 | 1.00 | 32.53 | W | O |
| ATOM | 5309 | O | HOH | W | 59 | 13.117 | 40.892 | 94.180 | 1.00 | 23.53 | W | O |
| ATOM | 5310 | O | HOH | W | 60 | 33.783 | 56.942 | 104.073 | 1.00 | 33.16 | W | O |
| ATOM | 5311 | O | HOH | W | 61 | 14.023 | 29.012 | 51.865 | 1.00 | 25.40 | W | O |
| ATOM | 5312 | O | HOH | W | 62 | 8.827 | 36.806 | 80.408 | 1.00 | 23.35 | W | O |
| ATOM | 5313 | O | HOH | W | 63 | 33.570 | 20.752 | 62.952 | 1.00 | 33.83 | W | O |
| ATOM | 5314 | O | HOH | W | 64 | 11.221 | 37.791 | 87.180 | 1.00 | 23.44 | W | O |
| ATOM | 5315 | O | HOH | W | 65 | 13.376 | 37.065 | 48.517 | 1.00 | 20.68 | W | O |
| ATOM | 5316 | O | HOH | W | 66 | 33.551 | 29.277 | 54.124 | 1.00 | 23.71 | W | O |
| ATOM | 5317 | O | HOH | W | 67 | 27.813 | 57.206 | 77.637 | 1.00 | 21.24 | W | O |
| ATOM | 5318 | O | HOH | W | 68 | 16.797 | 58.134 | 81.617 | 1.00 | 23.90 | W | O |
| ATOM | 5319 | O | HOH | W | 69 | 27.438 | 31.317 | 56.455 | 1.00 | 22.34 | W | O |
| ATOM | 5320 | O | HOH | W | 70 | 7.638 | 36.875 | 83.067 | 1.00 | 28.72 | W | O |
| ATOM | 5321 | O | HOH | W | 71 | 10.825 | 36.839 | 48.773 | 1.00 | 20.05 | W | O |
| ATOM | 5322 | O | HOH | W | 72 | 15.124 | 42.298 | 98.046 | 1.00 | 27.21 | W | O |
| ATOM | 5323 | O | HOH | W | 73 | 22.074 | 46.280 | 77.847 | 1.00 | 24.95 | W | O |
| ATOM | 5324 | O | HOH | W | 74 | 12.802 | 28.789 | 81.633 | 1.00 | 30.80 | W | O |
| ATOM | 5325 | O | HOH | W | 75 | 11.457 | 27.773 | 55.431 | 1.00 | 30.03 | W | O |
| ATOM | 5326 | O | HOH | W | 76 | 16.195 | 33.533 | 42.818 | 1.00 | 33.19 | W | O |
| ATOM | 5327 | O | HOH | W | 77 | 7.093 | 31.328 | 56.591 | 1.00 | 35.40 | W | O |
| ATOM | 5328 | O | HOH | W | 78 | 16.886 | 37.254 | 66.838 | 1.00 | 34.93 | W | O |
| ATOM | 5329 | O | HOH | W | 79 | 2.322 | 39.540 | 55.049 | 1.00 | 33.60 | W | O |
| ATOM | 5330 | O | HOH | W | 80 | 10.595 | 41.337 | 97.163 | 1.00 | 37.30 | W | O |
| ATOM | 5331 | O | HOH | W | 81 | 30.347 | 60.873 | 98.638 | 1.00 | 25.44 | W | O |
| ATOM | 5332 | O | HOH | W | 82 | 24.621 | 50.354 | 73.304 | 1.00 | 35.73 | W | O |
| ATOM | 5333 | O | HOH | W | 83 | 53.422 | 19.857 | 46.543 | 1.00 | 34.90 | W | O |
| ATOM | 5334 | O | HOH | W | 84 | 30.161 | 36.044 | 99.404 | 1.00 | 32.32 | W | O |
| ATOM | 5335 | O | HOH | W | 85 | 23.154 | 41.314 | 66.764 | 1.00 | 42.51 | W | O |
| ATOM | 5336 | O | HOH | W | 86 | 2.720 | 55.131 | 87.673 | 1.00 | 30.01 | W | O |
| ATOM | 5337 | O | HOH | W | 87 | 16.181 | 28.960 | 49.005 | 1.00 | 25.06 | W | O |
| ATOM | 5338 | O | HOH | W | 88 | 29.221 | 33.001 | 62.883 | 1.00 | 31.57 | W | O |
| ATOM | 5339 | O | HOH | W | 89 | 32.302 | 16.244 | 52.693 | 1.00 | 44.89 | W | O |
| ATOM | 5340 | O | HOH | W | 90 | 7.361 | 46.542 | 54.030 | 1.00 | 32.56 | W | O |
| ATOM | 5341 | O | HOH | W | 91 | 23.464 | 48.554 | 55.840 | 1.00 | 22.07 | W | O |
| ATOM | 5342 | O | HOH | W | 92 | 33.062 | 28.605 | 44.044 | 1.00 | 30.51 | W | O |
| ATOM | 5343 | O | HOH | W | 93 | 16.534 | 50.785 | 77.003 | 1.00 | 20.12 | W | O |
| ATOM | 5344 | O | HOH | W | 94 | 9.702 | 33.771 | 56.558 | 1.00 | 30.62 | W | O |
| ATOM | 5345 | O | HOH | W | 95 | 18.790 | 46.428 | 61.982 | 1.00 | 31.65 | W | O |
| ATOM | 5346 | O | HOH | W | 96 | 36.726 | 59.543 | 90.341 | 1.00 | 40.54 | W | O |
| ATOM | 5347 | O | HOH | W | 97 | 26.064 | 36.652 | 98.818 | 1.00 | 26.06 | W | O |
| ATOM | 5348 | O | HOH | W | 98 | 25.101 | 39.398 | 58.631 | 1.00 | 23.14 | W | O |
| ATOM | 5349 | O | HOH | W | 99 | 11.532 | 40.940 | 89.802 | 1.00 | 29.66 | W | O |
| ATOM | 5350 | O | HOH | W | 100 | 14.955 | 57.618 | 92.205 | 1.00 | 17.12 | W | O |
| ATOM | 5351 | O | HOH | W | 101 | 18.430 | 42.132 | 28.131 | 1.00 | 42.95 | W | O |
| ATOM | 5352 | O | HOH | W | 102 | 21.607 | 58.760 | 75.590 | 1.00 | 21.99 | W | O |
| ATOM | 5353 | O | HOH | W | 103 | 22.044 | 56.244 | 77.461 | 1.00 | 28.13 | W | O |
| ATOM | 5354 | O | HOH | W | 104 | 23.638 | 56.292 | 75.230 | 1.00 | 32.64 | W | O |
| ATOM | 5355 | O | HOH | W | 105 | 23.772 | 59.281 | 76.631 | 1.00 | 28.52 | W | O |
| ATOM | 5356 | O | HOH | W | 106 | 27.895 | 56.780 | 72.597 | 1.00 | 36.26 | W | O |
| ATOM | 5357 | O | HOH | W | 107 | 26.759 | 50.633 | 76.922 | 1.00 | 24.71 | W | O |
| ATOM | 5358 | O | HOH | W | 108 | 15.784 | 23.473 | 57.448 | 1.00 | 25.74 | W | O |
| ATOM | 5359 | O | HOH | W | 109 | 33.054 | 38.529 | 60.395 | 1.00 | 35.76 | W | O |
| ATOM | 5360 | O | HOH | W | 110 | 35.210 | 31.735 | 53.876 | 1.00 | 21.45 | W | O |
| TER | 5361 | | HOH | W | 110 | | | | | | W | |
| ATOM | 5362 | C1 | 448 | I | 1 | 41.090 | 41.549 | 53.570 | 1.00 | 28.57 | I | C |
| ATOM | 5363 | C2 | 448 | I | 1 | 41.082 | 40.476 | 52.485 | 1.00 | 29.88 | I | C |
| ATOM | 5364 | C3 | 448 | I | 1 | 40.874 | 39.064 | 52.906 | 1.00 | 28.88 | I | C |

| | | | | | | | | | | | | |
|---|---|---|---|---|---|---|---|---|---|---|---|---|
| ATOM | 5365 | C4 | 448 | I | 1 | 40.739 | 38.760 | 54.371 | 1.00 | 30.14 | I | C |
| ATOM | 5366 | C5 | 448 | I | 1 | 40.583 | 39.736 | 55.423 | 1.00 | 30.77 | I | C |
| ATOM | 5367 | C6 | 448 | I | 1 | 40.818 | 41.121 | 55.007 | 1.00 | 29.89 | I | C |
| ATOM | 5368 | N12 | 448 | I | 1 | 40.827 | 38.109 | 51.899 | 1.00 | 29.03 | I | N |
| ATOM | 5369 | C13 | 448 | I | 1 | 40.184 | 36.879 | 51.933 | 1.00 | 28.36 | I | C |
| ATOM | 5370 | N14 | 448 | I | 1 | 40.417 | 35.960 | 50.947 | 1.00 | 28.06 | I | N |
| ATOM | 5371 | C15 | 448 | I | 1 | 39.747 | 34.717 | 51.134 | 1.00 | 27.41 | I | C |
| ATOM | 5372 | C16 | 448 | I | 1 | 38.871 | 34.333 | 52.234 | 1.00 | 27.22 | I | C |
| ATOM | 5373 | C17 | 448 | I | 1 | 38.646 | 35.329 | 53.272 | 1.00 | 26.03 | I | C |
| ATOM | 5374 | N18 | 448 | I | 1 | 39.340 | 36.526 | 53.024 | 1.00 | 28.32 | I | N |
| ATOM | 5375 | C20 | 448 | I | 1 | 37.738 | 35.331 | 54.509 | 1.00 | 25.51 | I | C |
| ATOM | 5376 | C21 | 448 | I | 1 | 37.559 | 34.125 | 55.010 | 1.00 | 25.54 | I | C |
| ATOM | 5377 | C22 | 448 | I | 1 | 36.602 | 34.628 | 56.043 | 1.00 | 25.44 | I | C |
| ATOM | 5378 | N23 | 448 | I | 1 | 36.475 | 36.147 | 56.002 | 1.00 | 24.44 | I | N |
| ATOM | 5379 | C24 | 448 | I | 1 | 37.314 | 36.564 | 54.816 | 1.00 | 26.32 | I | C |
| ATOM | 5380 | C25 | 448 | I | 1 | 38.258 | 33.003 | 52.196 | 1.00 | 27.01 | I | C |
| ATOM | 5381 | C30 | 448 | I | 1 | 35.845 | 33.802 | 57.034 | 1.00 | 24.78 | I | C |
| ATOM | 5382 | O31 | 448 | I | 1 | 35.608 | 32.678 | 56.669 | 1.00 | 23.74 | I | O |
| ATOM | 5383 | N32 | 448 | I | 1 | 35.490 | 34.356 | 58.181 | 1.00 | 23.50 | I | N |
| ATOM | 5384 | C33 | 448 | I | 1 | 34.606 | 33.769 | 59.087 | 1.00 | 22.50 | I | C |
| ATOM | 5385 | C34 | 448 | I | 1 | 33.941 | 34.868 | 59.938 | 1.00 | 21.89 | I | C |
| ATOM | 5386 | O35 | 448 | I | 1 | 33.229 | 35.720 | 59.134 | 1.00 | 21.64 | I | O |
| ATOM | 5387 | C38 | 448 | I | 1 | 35.329 | 32.832 | 60.026 | 1.00 | 21.55 | I | C |
| ATOM | 5388 | C40 | 448 | I | 1 | 36.827 | 32.800 | 60.086 | 1.00 | 19.97 | I | C |
| ATOM | 5389 | C41 | 448 | I | 1 | 37.498 | 31.807 | 61.016 | 1.00 | 21.08 | I | C |
| ATOM | 5390 | C42 | 448 | I | 1 | 36.660 | 30.923 | 61.911 | 1.00 | 21.00 | I | C |
| ATOM | 5391 | C43 | 448 | I | 1 | 35.157 | 30.985 | 61.840 | 1.00 | 19.39 | I | C |
| ATOM | 5392 | C44 | 448 | I | 1 | 34.502 | 31.903 | 60.877 | 1.00 | 19.71 | I | C |
| TER | 5393 | | 448 | I | 1 | | | | | | I | |
| ATOM | 5394 | C1 | 449 | J | 1 | 27.316 | 62.175 | 79.708 | 1.00 | 30.96 | J | C |
| ATOM | 5395 | C2 | 449 | J | 1 | 26.414 | 62.601 | 80.882 | 1.00 | 30.39 | J | C |
| ATOM | 5396 | C3 | 449 | J | 1 | 24.947 | 62.479 | 80.667 | 1.00 | 28.87 | J | C |
| ATOM | 5397 | C4 | 449 | J | 1 | 24.455 | 61.952 | 79.333 | 1.00 | 30.33 | J | C |
| ATOM | 5398 | C5 | 449 | J | 1 | 25.286 | 61.413 | 78.285 | 1.00 | 31.43 | J | C |
| ATOM | 5399 | C6 | 449 | J | 1 | 26.707 | 61.599 | 78.440 | 1.00 | 29.55 | J | C |
| ATOM | 5400 | N12 | 449 | J | 1 | 24.126 | 62.897 | 81.728 | 1.00 | 28.10 | J | N |
| ATOM | 5401 | C13 | 449 | J | 1 | 22.861 | 62.380 | 82.015 | 1.00 | 25.83 | J | C |
| ATOM | 5402 | N14 | 449 | J | 1 | 22.168 | 62.861 | 83.108 | 1.00 | 25.15 | J | N |
| ATOM | 5403 | C15 | 449 | J | 1 | 20.869 | 62.338 | 83.264 | 1.00 | 24.03 | J | C |
| ATOM | 5404 | C16 | 449 | J | 1 | 20.203 | 61.368 | 82.387 | 1.00 | 24.98 | J | C |
| ATOM | 5405 | C17 | 449 | J | 1 | 20.959 | 60.880 | 81.234 | 1.00 | 25.32 | J | C |
| ATOM | 5406 | N18 | 449 | J | 1 | 22.246 | 61.430 | 81.143 | 1.00 | 23.60 | J | N |
| ATOM | 5407 | C20 | 449 | J | 1 | 20.621 | 59.824 | 80.143 | 1.00 | 25.68 | J | C |
| ATOM | 5408 | C21 | 449 | J | 1 | 19.325 | 59.771 | 79.864 | 1.00 | 25.60 | J | C |
| ATOM | 5409 | C22 | 449 | J | 1 | 19.572 | 58.681 | 78.850 | 1.00 | 25.43 | J | C |
| ATOM | 5410 | N23 | 449 | J | 1 | 21.062 | 58.372 | 78.684 | 1.00 | 24.96 | J | N |
| ATOM | 5411 | C24 | 449 | J | 1 | 21.728 | 59.195 | 79.718 | 1.00 | 26.88 | J | C |
| ATOM | 5412 | C25 | 449 | J | 1 | 18.884 | 60.854 | 82.765 | 1.00 | 26.81 | J | C |
| ATOM | 5413 | C30 | 449 | J | 1 | 18.531 | 57.921 | 78.082 | 1.00 | 24.42 | J | C |
| ATOM | 5414 | O31 | 449 | J | 1 | 17.447 | 57.814 | 78.618 | 1.00 | 22.75 | J | O |
| ATOM | 5415 | N32 | 449 | J | 1 | 18.897 | 57.417 | 76.910 | 1.00 | 23.50 | J | N |
| ATOM | 5416 | C33 | 449 | J | 1 | 18.031 | 56.509 | 76.218 | 1.00 | 21.55 | J | C |
| ATOM | 5417 | C34 | 449 | J | 1 | 18.862 | 55.602 | 75.337 | 1.00 | 20.27 | J | C |
| ATOM | 5418 | O35 | 449 | J | 1 | 19.574 | 54.753 | 76.137 | 1.00 | 21.10 | J | O |
| ATOM | 5419 | C38 | 449 | J | 1 | 17.033 | 57.274 | 75.362 | 1.00 | 21.26 | J | C |
| ATOM | 5420 | C40 | 449 | J | 1 | 17.208 | 58.747 | 75.084 | 1.00 | 21.26 | J | C |
| ATOM | 5421 | C41 | 449 | J | 1 | 16.185 | 59.467 | 74.225 | 1.00 | 22.35 | J | C |
| ATOM | 5422 | C42 | 449 | J | 1 | 15.020 | 58.689 | 73.651 | 1.00 | 21.25 | J | C |
| ATOM | 5423 | C43 | 449 | J | 1 | 14.867 | 57.229 | 73.931 | 1.00 | 21.27 | J | C |
| ATOM | 5424 | C44 | 449 | J | 1 | 15.853 | 56.541 | 74.796 | 1.00 | 21.84 | J | C |
| TER | 5425 | | 449 | J | 1 | | | | | | J | |
| END | | | | | | | | | | | | |

FIG. 6

| | | Atom Type | Resid | | # | X | Y | Z | Occ | B | | |
|---|---|---|---|---|---|---|---|---|---|---|---|---|
| ATOM | 1 | CB | LYS | A | 36 | 59.641 | 27.464 | 57.131 | 1.00 | 51.95 | A | C |
| ATOM | 2 | CG | LYS | A | 36 | 58.660 | 26.332 | 57.498 | 1.00 | 51.95 | A | C |
| ATOM | 3 | CD | LYS | A | 36 | 57.961 | 26.551 | 58.834 | 1.00 | 51.95 | A | C |
| ATOM | 4 | CE | LYS | A | 36 | 56.777 | 25.596 | 58.967 | 1.00 | 51.95 | A | C |
| ATOM | 5 | NZ | LYS | A | 36 | 55.963 | 25.820 | 60.203 | 1.00 | 51.95 | A | N |
| ATOM | 6 | C | LYS | A | 36 | 59.062 | 26.875 | 54.830 | 1.00 | 56.75 | A | C |
| ATOM | 7 | O | LYS | A | 36 | 58.240 | 27.702 | 54.419 | 1.00 | 56.75 | A | O |
| ATOM | 8 | N | LYS | A | 36 | 60.903 | 28.540 | 55.247 | 1.00 | 56.75 | A | N |
| ATOM | 9 | CA | LYS | A | 36 | 60.212 | 27.296 | 55.721 | 1.00 | 56.75 | A | C |
| ATOM | 10 | N | VAL | A | 37 | 58.976 | 25.574 | 54.577 | 1.00 | 43.54 | A | N |
| ATOM | 11 | CA | VAL | A | 37 | 57.917 | 25.054 | 53.734 | 1.00 | 43.54 | A | C |
| ATOM | 12 | CB | VAL | A | 37 | 58.495 | 24.409 | 52.496 | 1.00 | 35.30 | A | C |
| ATOM | 13 | CG1 | VAL | A | 37 | 57.375 | 24.070 | 51.526 | 1.00 | 35.30 | A | C |
| ATOM | 14 | CG2 | VAL | A | 37 | 59.517 | 25.339 | 51.875 | 1.00 | 35.30 | A | C |
| ATOM | 15 | C | VAL | A | 37 | 57.048 | 24.029 | 54.442 | 1.00 | 43.54 | A | C |
| ATOM | 16 | O | VAL | A | 37 | 57.513 | 23.309 | 55.332 | 1.00 | 43.54 | A | O |
| ATOM | 17 | N | THR | A | 38 | 55.780 | 23.973 | 54.047 | 1.00 | 38.06 | A | N |
| ATOM | 18 | CA | THR | A | 38 | 54.856 | 23.021 | 54.635 | 1.00 | 38.06 | A | C |
| ATOM | 19 | CB | THR | A | 38 | 53.695 | 23.705 | 55.372 | 1.00 | 30.23 | A | C |
| ATOM | 20 | OG1 | THR | A | 38 | 54.215 | 24.625 | 56.333 | 1.00 | 30.23 | A | O |
| ATOM | 21 | CG2 | THR | A | 38 | 52.848 | 22.676 | 56.089 | 1.00 | 30.23 | A | C |
| ATOM | 22 | C | THR | A | 38 | 54.259 | 22.178 | 53.522 | 1.00 | 38.06 | A | C |
| ATOM | 23 | O | THR | A | 38 | 53.657 | 22.712 | 52.584 | 1.00 | 38.06 | A | O |
| ATOM | 24 | N | THR | A | 39 | 54.438 | 20.862 | 53.624 | 1.00 | 34.77 | A | N |
| ATOM | 25 | CA | THR | A | 39 | 53.901 | 19.948 | 52.630 | 1.00 | 34.77 | A | C |
| ATOM | 26 | CB | THR | A | 39 | 55.015 | 19.178 | 51.897 | 1.00 | 28.29 | A | C |
| ATOM | 27 | OG1 | THR | A | 39 | 55.692 | 20.069 | 51.009 | 1.00 | 28.29 | A | O |
| ATOM | 28 | CG2 | THR | A | 39 | 54.429 | 18.046 | 51.067 | 1.00 | 28.29 | A | C |
| ATOM | 29 | C | THR | A | 39 | 52.962 | 18.970 | 53.299 | 1.00 | 34.77 | A | C |
| ATOM | 30 | O | THR | A | 39 | 53.329 | 18.325 | 54.272 | 1.00 | 34.77 | A | O |
| ATOM | 31 | N | VAL | A | 40 | 51.746 | 18.872 | 52.773 | 1.00 | 45.36 | A | N |
| ATOM | 32 | CA | VAL | A | 40 | 50.741 | 17.984 | 53.341 | 1.00 | 45.36 | A | C |
| ATOM | 33 | CB | VAL | A | 40 | 49.682 | 18.801 | 54.156 | 1.00 | 77.60 | A | C |
| ATOM | 34 | CG1 | VAL | A | 40 | 49.062 | 19.894 | 53.285 | 1.00 | 77.60 | A | C |
| ATOM | 35 | CG2 | VAL | A | 40 | 48.589 | 17.859 | 54.691 | 1.00 | 77.60 | A | C |
| ATOM | 36 | C | VAL | A | 40 | 50.042 | 17.162 | 52.260 | 1.00 | 45.36 | A | C |
| ATOM | 37 | O | VAL | A | 40 | 50.332 | 17.318 | 51.078 | 1.00 | 45.36 | A | O |
| ATOM | 38 | N | VAL | A | 41 | 49.153 | 16.260 | 52.652 | 1.00 | 38.14 | A | N |
| ATOM | 39 | CA | VAL | A | 41 | 48.421 | 15.493 | 51.652 | 1.00 | 38.14 | A | C |
| ATOM | 40 | CB | VAL | A | 41 | 48.532 | 13.962 | 51.873 | 1.00 | 21.48 | A | C |
| ATOM | 41 | CG1 | VAL | A | 41 | 47.745 | 13.225 | 50.793 | 1.00 | 21.48 | A | C |
| ATOM | 42 | CG2 | VAL | A | 41 | 49.994 | 13.537 | 51.833 | 1.00 | 21.48 | A | C |
| ATOM | 43 | C | VAL | A | 41 | 46.961 | 15.917 | 51.768 | 1.00 | 38.14 | A | C |
| ATOM | 44 | O | VAL | A | 41 | 46.319 | 15.669 | 52.791 | 1.00 | 38.14 | A | O |
| ATOM | 45 | N | ALA | A | 42 | 46.438 | 16.557 | 50.724 | 1.00 | 42.43 | A | N |
| ATOM | 46 | CA | ALA | A | 42 | 45.061 | 17.036 | 50.765 | 1.00 | 42.43 | A | C |
| ATOM | 47 | CB | ALA | A | 42 | 45.039 | 18.546 | 50.581 | 1.00 | 38.94 | A | C |
| ATOM | 48 | C | ALA | A | 42 | 44.088 | 16.392 | 49.781 | 1.00 | 42.43 | A | C |
| ATOM | 49 | O | ALA | A | 42 | 44.484 | 15.745 | 48.805 | 1.00 | 42.43 | A | O |
| ATOM | 50 | N | THR | A | 43 | 42.804 | 16.602 | 50.056 | 1.00 | 46.78 | A | N |
| ATOM | 51 | CA | THR | A | 43 | 41.720 | 16.088 | 49.235 | 1.00 | 46.78 | A | C |
| ATOM | 52 | CB | THR | A | 43 | 40.578 | 15.571 | 50.117 | 1.00 | 35.10 | A | C |
| ATOM | 53 | OG1 | THR | A | 43 | 41.104 | 14.668 | 51.095 | 1.00 | 35.10 | A | O |
| ATOM | 54 | CG2 | THR | A | 43 | 39.526 | 14.863 | 49.284 | 1.00 | 35.10 | A | C |
| ATOM | 55 | C | THR | A | 43 | 41.160 | 17.231 | 48.394 | 1.00 | 46.78 | A | C |
| ATOM | 56 | O | THR | A | 43 | 40.965 | 18.340 | 48.895 | 1.00 | 46.78 | A | O |
| ATOM | 57 | N | PRO | A | 44 | 40.895 | 16.979 | 47.106 | 1.00 | 38.20 | A | N |

| | | | | | | | | | | | | |
|---|---|---|---|---|---|---|---|---|---|---|---|---|
| ATOM | 58 | CD | PRO | A | 44 | 41.291 | 15.786 | 46.345 | 1.00 | 43.73 | A | C |
| ATOM | 59 | CA | PRO | A | 44 | 40.350 | 18.000 | 46.209 | 1.00 | 38.20 | A | C |
| ATOM | 60 | CB | PRO | A | 44 | 40.281 | 17.271 | 44.871 | 1.00 | 43.73 | A | C |
| ATOM | 61 | CG | PRO | A | 44 | 41.430 | 16.346 | 44.946 | 1.00 | 43.73 | A | C |
| ATOM | 62 | C | PRO | A | 44 | 38.959 | 18.439 | 46.686 | 1.00 | 38.20 | A | C |
| ATOM | 63 | O | PRO | A | 44 | 38.205 | 17.629 | 47.238 | 1.00 | 38.20 | A | O |
| ATOM | 64 | N | GLY | A | 45 | 38.628 | 19.714 | 46.476 | 1.00 | 41.76 | A | N |
| ATOM | 65 | CA | GLY | A | 45 | 37.327 | 20.216 | 46.878 | 1.00 | 41.76 | A | C |
| ATOM | 66 | C | GLY | A | 45 | 36.231 | 19.497 | 46.118 | 1.00 | 41.76 | A | C |
| ATOM | 67 | O | GLY | A | 45 | 35.233 | 19.061 | 46.705 | 1.00 | 41.76 | A | O |
| ATOM | 68 | N | ALA | A | 46 | 36.431 | 19.366 | 44.803 | 1.00 | 75.71 | A | N |
| ATOM | 69 | CA | ALA | A | 46 | 35.487 | 18.684 | 43.918 | 1.00 | 75.71 | A | C |
| ATOM | 70 | CB | ALA | A | 46 | 34.960 | 19.667 | 42.876 | 1.00 | 42.88 | A | C |
| ATOM | 71 | C | ALA | A | 46 | 36.170 | 17.479 | 43.239 | 1.00 | 75.71 | A | C |
| ATOM | 72 | O | ALA | A | 46 | 37.402 | 17.441 | 43.100 | 1.00 | 75.71 | A | O |
| ATOM | 73 | N | GLY | A | 47 | 35.366 | 16.504 | 42.812 | 1.00 | 65.07 | A | N |
| ATOM | 74 | CA | GLY | A | 47 | 35.911 | 15.307 | 42.190 | 1.00 | 65.07 | A | C |
| ATOM | 75 | C | GLY | A | 47 | 36.134 | 14.269 | 43.283 | 1.00 | 65.07 | A | C |
| ATOM | 76 | O | GLY | A | 47 | 36.183 | 14.637 | 44.466 | 1.00 | 65.07 | A | O |
| ATOM | 77 | N | PRO | A | 48 | 36.256 | 12.968 | 42.941 | 1.00 | 71.08 | A | N |
| ATOM | 78 | CD | PRO | A | 48 | 35.816 | 12.375 | 41.666 | 1.00 | 86.91 | A | C |
| ATOM | 79 | CA | PRO | A | 48 | 36.469 | 11.912 | 43.944 | 1.00 | 71.08 | A | C |
| ATOM | 80 | CB | PRO | A | 48 | 36.357 | 10.630 | 43.120 | 1.00 | 86.91 | A | C |
| ATOM | 81 | CG | PRO | A | 48 | 35.314 | 11.000 | 42.117 | 1.00 | 86.91 | A | C |
| ATOM | 82 | C | PRO | A | 48 | 37.788 | 11.992 | 44.721 | 1.00 | 71.08 | A | C |
| ATOM | 83 | O | PRO | A | 48 | 38.839 | 12.350 | 44.165 | 1.00 | 71.08 | A | O |
| ATOM | 84 | N | ASP | A | 49 | 37.727 | 11.637 | 46.006 | 1.00 | 50.89 | A | N |
| ATOM | 85 | CA | ASP | A | 49 | 38.910 | 11.689 | 46.849 | 1.00 | 50.89 | A | C |
| ATOM | 86 | CB | ASP | A | 49 | 38.683 | 11.060 | 48.234 | 1.00 | 46.24 | A | C |
| ATOM | 87 | CG | ASP | A | 49 | 39.978 | 11.005 | 49.065 | 1.00 | 46.24 | A | C |
| ATOM | 88 | OD1 | ASP | A | 49 | 40.831 | 11.905 | 48.888 | 1.00 | 46.24 | A | O |
| ATOM | 89 | OD2 | ASP | A | 49 | 40.154 | 10.082 | 49.900 | 1.00 | 46.24 | A | O |
| ATOM | 90 | C | ASP | A | 49 | 40.103 | 11.016 | 46.197 | 1.00 | 50.89 | A | C |
| ATOM | 91 | O | ASP | A | 49 | 40.167 | 9.796 | 46.103 | 1.00 | 50.89 | A | O |
| ATOM | 92 | N | ARG | A | 50 | 41.032 | 11.838 | 45.731 | 1.00 | 42.12 | A | N |
| ATOM | 93 | CA | ARG | A | 50 | 42.270 | 11.368 | 45.128 | 1.00 | 42.12 | A | C |
| ATOM | 94 | CB | ARG | A | 50 | 42.189 | 11.435 | 43.601 | 1.00 | 90.07 | A | C |
| ATOM | 95 | CG | ARG | A | 50 | 42.446 | 10.085 | 42.972 | 1.00 | 90.07 | A | C |
| ATOM | 96 | CD | ARG | A | 50 | 42.105 | 9.997 | 41.480 | 1.00 | 90.07 | A | C |
| ATOM | 97 | NE | ARG | A | 50 | 42.309 | 8.617 | 41.054 | 1.00 | 90.07 | A | N |
| ATOM | 98 | CZ | ARG | A | 50 | 41.618 | 7.595 | 41.560 | 1.00 | 90.07 | A | C |
| ATOM | 99 | NH1 | ARG | A | 50 | 40.674 | 7.826 | 42.483 | 1.00 | 90.07 | A | N |
| ATOM | 100 | NH2 | ARG | A | 50 | 41.911 | 6.341 | 41.204 | 1.00 | 90.07 | A | N |
| ATOM | 101 | C | ARG | A | 50 | 43.333 | 12.328 | 45.650 | 1.00 | 42.12 | A | C |
| ATOM | 102 | O | ARG | A | 50 | 43.835 | 13.179 | 44.926 | 1.00 | 42.12 | A | O |
| ATOM | 103 | N | PRO | A | 51 | 43.718 | 12.154 | 46.917 | 1.00 | 24.73 | A | N |
| ATOM | 104 | CD | PRO | A | 51 | 43.793 | 10.758 | 47.391 | 1.00 | 35.32 | A | C |
| ATOM | 105 | CA | PRO | A | 51 | 44.697 | 12.968 | 47.637 | 1.00 | 24.73 | A | C |
| ATOM | 106 | CB | PRO | A | 51 | 45.123 | 12.054 | 48.785 | 1.00 | 35.32 | A | C |
| ATOM | 107 | CG | PRO | A | 51 | 45.103 | 10.737 | 48.149 | 1.00 | 35.32 | A | C |
| ATOM | 108 | C | PRO | A | 51 | 45.852 | 13.345 | 46.772 | 1.00 | 24.73 | A | C |
| ATOM | 109 | O | PRO | A | 51 | 46.109 | 12.713 | 45.768 | 1.00 | 24.73 | A | O |
| ATOM | 110 | N | GLN | A | 52 | 46.552 | 14.389 | 47.178 | 1.00 | 40.86 | A | N |
| ATOM | 111 | CA | GLN | A | 52 | 47.716 | 14.853 | 46.449 | 1.00 | 40.86 | A | C |
| ATOM | 112 | CB | GLN | A | 52 | 47.275 | 15.608 | 45.206 | 1.00 | 68.82 | A | C |
| ATOM | 113 | CG | GLN | A | 52 | 45.882 | 16.189 | 45.328 | 1.00 | 68.82 | A | C |
| ATOM | 114 | CD | GLN | A | 52 | 45.117 | 16.119 | 44.013 | 1.00 | 68.82 | A | C |
| ATOM | 115 | OE1 | GLN | A | 52 | 44.710 | 17.148 | 43.462 | 1.00 | 68.82 | A | O |
| ATOM | 116 | NE2 | GLN | A | 52 | 44.932 | 14.906 | 43.493 | 1.00 | 68.82 | A | N |
| ATOM | 117 | C | GLN | A | 52 | 48.533 | 15.769 | 47.353 | 1.00 | 40.86 | A | C |
| ATOM | 118 | O | GLN | A | 52 | 48.006 | 16.450 | 48.240 | 1.00 | 40.86 | A | O |
| ATOM | 119 | N | GLU | A | 53 | 49.839 | 15.739 | 47.127 | 1.00 | 43.20 | A | N |
| ATOM | 120 | CA | GLU | A | 53 | 50.780 | 16.530 | 47.888 | 1.00 | 43.20 | A | C |

| | | | | | | | | | | | | |
|---|---|---|---|---|---|---|---|---|---|---|---|---|
| ATOM | 121 | CB | GLU | A | 53 | 52.202 | 16.154 | 47.521 | 1.00 | 43.19 | A | C |
| ATOM | 122 | CG | GLU | A | 53 | 52.736 | 14.966 | 48.248 | 1.00 | 43.19 | A | C |
| ATOM | 123 | CD | GLU | A | 53 | 54.232 | 14.999 | 48.278 | 1.00 | 43.19 | A | C |
| ATOM | 124 | OE1 | GLU | A | 53 | 54.828 | 14.143 | 48.956 | 1.00 | 43.19 | A | O |
| ATOM | 125 | OE2 | GLU | A | 53 | 54.809 | 15.895 | 47.625 | 1.00 | 43.19 | A | O |
| ATOM | 126 | C | GLU | A | 53 | 50.591 | 17.992 | 47.533 | 1.00 | 43.20 | A | C |
| ATOM | 127 | O | GLU | A | 53 | 50.448 | 18.359 | 46.366 | 1.00 | 43.20 | A | O |
| ATOM | 128 | N | VAL | A | 54 | 50.599 | 18.829 | 48.552 | 1.00 | 28.32 | A | N |
| ATOM | 129 | CA | VAL | A | 54 | 50.458 | 20.248 | 48.337 | 1.00 | 28.32 | A | C |
| ATOM | 130 | CB | VAL | A | 54 | 49.082 | 20.792 | 48.793 | 1.00 | 38.47 | A | C |
| ATOM | 131 | CG1 | VAL | A | 54 | 49.046 | 22.298 | 48.581 | 1.00 | 38.47 | A | C |
| ATOM | 132 | CG2 | VAL | A | 54 | 47.953 | 20.121 | 48.023 | 1.00 | 38.47 | A | C |
| ATOM | 133 | C | VAL | A | 54 | 51.516 | 20.908 | 49.197 | 1.00 | 28.32 | A | C |
| ATOM | 134 | O | VAL | A | 54 | 51.676 | 20.575 | 50.381 | 1.00 | 28.32 | A | O |
| ATOM | 135 | N | SER | A | 55 | 52.237 | 21.841 | 48.593 | 1.00 | 49.20 | A | N |
| ATOM | 136 | CA | SER | A | 55 | 53.292 | 22.562 | 49.292 | 1.00 | 49.20 | A | C |
| ATOM | 137 | CB | SER | A | 55 | 54.644 | 22.318 | 48.616 | 1.00 | 39.01 | A | C |
| ATOM | 138 | OG | SER | A | 55 | 54.994 | 20.952 | 48.700 | 1.00 | 39.01 | A | O |
| ATOM | 139 | C | SER | A | 55 | 53.003 | 24.055 | 49.285 | 1.00 | 49.20 | A | C |
| ATOM | 140 | O | SER | A | 55 | 52.630 | 24.631 | 48.258 | 1.00 | 49.20 | A | O |
| ATOM | 141 | N | TYR | A | 56 | 53.192 | 24.691 | 50.427 | 1.00 | 44.73 | A | N |
| ATOM | 142 | CA | TYR | A | 56 | 52.924 | 26.109 | 50.500 | 1.00 | 44.73 | A | C |
| ATOM | 143 | CB | TYR | A | 56 | 51.451 | 26.339 | 50.781 | 1.00 | 34.73 | A | C |
| ATOM | 144 | CG | TYR | A | 56 | 51.009 | 25.826 | 52.116 | 1.00 | 34.73 | A | C |
| ATOM | 145 | CD1 | TYR | A | 56 | 51.235 | 26.566 | 53.265 | 1.00 | 34.73 | A | C |
| ATOM | 146 | CE1 | TYR | A | 56 | 50.803 | 26.120 | 54.505 | 1.00 | 34.73 | A | C |
| ATOM | 147 | CD2 | TYR | A | 56 | 50.343 | 24.608 | 52.228 | 1.00 | 34.73 | A | C |
| ATOM | 148 | CE2 | TYR | A | 56 | 49.899 | 24.145 | 53.467 | 1.00 | 34.73 | A | C |
| ATOM | 149 | CZ | TYR | A | 56 | 50.136 | 24.910 | 54.601 | 1.00 | 34.73 | A | C |
| ATOM | 150 | OH | TYR | A | 56 | 49.705 | 24.477 | 55.828 | 1.00 | 34.73 | A | O |
| ATOM | 151 | C | TYR | A | 56 | 53.769 | 26.758 | 51.569 | 1.00 | 44.73 | A | C |
| ATOM | 152 | O | TYR | A | 56 | 54.033 | 26.169 | 52.615 | 1.00 | 44.73 | A | O |
| ATOM | 153 | N | THR | A | 57 | 54.169 | 27.989 | 51.300 | 1.00 | 51.41 | A | N |
| ATOM | 154 | CA | THR | A | 57 | 55.024 | 28.731 | 52.203 | 1.00 | 51.41 | A | C |
| ATOM | 155 | CB | THR | A | 57 | 56.432 | 28.862 | 51.593 | 1.00 | 58.53 | A | C |
| ATOM | 156 | OG1 | THR | A | 57 | 57.249 | 29.690 | 52.431 | 1.00 | 58.53 | A | O |
| ATOM | 157 | CG2 | THR | A | 57 | 56.344 | 29.485 | 50.189 | 1.00 | 58.53 | A | C |
| ATOM | 158 | C | THR | A | 57 | 54.476 | 30.128 | 52.470 | 1.00 | 51.41 | A | C |
| ATOM | 159 | O | THR | A | 57 | 53.324 | 30.415 | 52.163 | 1.00 | 51.41 | A | O |
| ATOM | 160 | N | ASP | A | 58 | 55.323 | 30.994 | 53.024 | 1.00 | 45.63 | A | N |
| ATOM | 161 | CA | ASP | A | 58 | 54.956 | 32.369 | 53.341 | 1.00 | 45.63 | A | C |
| ATOM | 162 | CB | ASP | A | 58 | 54.889 | 33.229 | 52.071 | 1.00 | 63.56 | A | C |
| ATOM | 163 | CG | ASP | A | 58 | 56.253 | 33.457 | 51.440 | 1.00 | 63.56 | A | C |
| ATOM | 164 | OD1 | ASP | A | 58 | 57.226 | 33.707 | 52.190 | 1.00 | 63.56 | A | O |
| ATOM | 165 | OD2 | ASP | A | 58 | 56.349 | 33.402 | 50.188 | 1.00 | 63.56 | A | O |
| ATOM | 166 | C | ASP | A | 58 | 53.634 | 32.477 | 54.085 | 1.00 | 45.63 | A | C |
| ATOM | 167 | O | ASP | A | 58 | 52.881 | 33.431 | 53.885 | 1.00 | 45.63 | A | O |
| ATOM | 168 | N | THR | A | 59 | 53.348 | 31.504 | 54.940 | 1.00 | 31.47 | A | N |
| ATOM | 169 | CA | THR | A | 59 | 52.105 | 31.527 | 55.695 | 1.00 | 31.47 | A | C |
| ATOM | 170 | CB | THR | A | 59 | 51.941 | 30.230 | 56.544 | 1.00 | 38.11 | A | C |
| ATOM | 171 | OG1 | THR | A | 59 | 50.708 | 30.279 | 57.273 | 1.00 | 38.11 | A | O |
| ATOM | 172 | CG2 | THR | A | 59 | 53.100 | 30.078 | 57.531 | 1.00 | 38.11 | A | C |
| ATOM | 173 | C | THR | A | 59 | 52.063 | 32.739 | 56.619 | 1.00 | 31.47 | A | C |
| ATOM | 174 | O | THR | A | 59 | 53.097 | 33.196 | 57.085 | 1.00 | 31.47 | A | O |
| ATOM | 175 | N | LYS | A | 60 | 50.862 | 33.260 | 56.857 | 1.00 | 56.55 | A | N |
| ATOM | 176 | CA | LYS | A | 60 | 50.651 | 34.404 | 57.747 | 1.00 | 56.55 | A | C |
| ATOM | 177 | CB | LYS | A | 60 | 51.121 | 35.712 | 57.094 | 1.00 | 30.87 | A | C |
| ATOM | 178 | CG | LYS | A | 60 | 50.182 | 36.278 | 56.050 | 1.00 | 30.87 | A | C |
| ATOM | 179 | CD | LYS | A | 60 | 50.717 | 37.564 | 55.444 | 1.00 | 30.87 | A | C |
| ATOM | 180 | CE | LYS | A | 60 | 51.802 | 37.309 | 54.384 | 1.00 | 30.87 | A | C |
| ATOM | 181 | NZ | LYS | A | 60 | 53.075 | 36.786 | 54.941 | 1.00 | 30.87 | A | N |
| ATOM | 182 | C | LYS | A | 60 | 49.163 | 34.500 | 58.100 | 1.00 | 56.55 | A | C |
| ATOM | 183 | O | LYS | A | 60 | 48.298 | 34.048 | 57.335 | 1.00 | 56.55 | A | O |

| | | | | | | | | | | | | |
|---|---|---|---|---|---|---|---|---|---|---|---|---|
| ATOM | 184 | N | VAL | A | 61 | 48.855 | 35.083 | 59.252 | 1.00 | 43.34 | A | N |
| ATOM | 185 | CA | VAL | A | 61 | 47.465 | 35.206 | 59.651 | 1.00 | 43.34 | A | C |
| ATOM | 186 | CB | VAL | A | 61 | 47.302 | 35.189 | 61.154 | 1.00 | 31.64 | A | C |
| ATOM | 187 | CG1 | VAL | A | 61 | 45.815 | 35.114 | 61.491 | 1.00 | 31.64 | A | C |
| ATOM | 188 | CG2 | VAL | A | 61 | 48.071 | 34.026 | 61.745 | 1.00 | 31.64 | A | C |
| ATOM | 189 | C | VAL | A | 61 | 46.846 | 36.500 | 59.164 | 1.00 | 43.34 | A | C |
| ATOM | 190 | O | VAL | A | 61 | 47.433 | 37.564 | 59.330 | 1.00 | 43.34 | A | O |
| ATOM | 191 | N | ILE | A | 62 | 45.660 | 36.418 | 58.566 | 1.00 | 47.96 | A | N |
| ATOM | 192 | CA | ILE | A | 62 | 44.991 | 37.627 | 58.096 | 1.00 | 47.96 | A | C |
| ATOM | 193 | CB | ILE | A | 62 | 44.593 | 37.559 | 56.587 | 1.00 | 53.40 | A | C |
| ATOM | 194 | CG2 | ILE | A | 62 | 45.839 | 37.583 | 55.706 | 1.00 | 53.40 | A | C |
| ATOM | 195 | CG1 | ILE | A | 62 | 43.774 | 36.304 | 56.303 | 1.00 | 53.40 | A | C |
| ATOM | 196 | CD1 | ILE | A | 62 | 43.540 | 36.053 | 54.812 | 1.00 | 53.40 | A | C |
| ATOM | 197 | C | ILE | A | 62 | 43.738 | 37.899 | 58.902 | 1.00 | 47.96 | A | C |
| ATOM | 198 | O | ILE | A | 62 | 43.253 | 39.028 | 58.933 | 1.00 | 47.96 | A | O |
| ATOM | 199 | N | GLY | A | 63 | 43.218 | 36.865 | 59.558 | 1.00 | 36.83 | A | N |
| ATOM | 200 | CA | GLY | A | 63 | 42.019 | 37.042 | 60.352 | 1.00 | 36.83 | A | C |
| ATOM | 201 | C | GLY | A | 63 | 41.806 | 35.945 | 61.366 | 1.00 | 36.83 | A | C |
| ATOM | 202 | O | GLY | A | 63 | 41.755 | 34.762 | 61.011 | 1.00 | 36.83 | A | O |
| ATOM | 203 | N | ASN | A | 64 | 41.678 | 36.330 | 62.635 | 1.00 | 87.22 | A | N |
| ATOM | 204 | CA | ASN | A | 64 | 41.460 | 35.359 | 63.716 | 1.00 | 87.22 | A | C |
| ATOM | 205 | CB | ASN | A | 64 | 42.582 | 35.463 | 64.762 | 1.00 | 69.96 | A | C |
| ATOM | 206 | CG | ASN | A | 64 | 42.319 | 34.604 | 65.980 | 1.00 | 69.96 | A | C |
| ATOM | 207 | OD1 | ASN | A | 64 | 41.679 | 35.043 | 66.940 | 1.00 | 69.96 | A | O |
| ATOM | 208 | ND2 | ASN | A | 64 | 42.800 | 33.360 | 65.944 | 1.00 | 69.96 | A | N |
| ATOM | 209 | C | ASN | A | 64 | 40.101 | 35.559 | 64.383 | 1.00 | 87.22 | A | C |
| ATOM | 210 | O | ASN | A | 64 | 39.992 | 36.205 | 65.420 | 1.00 | 87.22 | A | O |
| ATOM | 211 | N | GLY | A | 65 | 39.059 | 35.008 | 63.776 | 1.00 | 61.88 | A | N |
| ATOM | 212 | CA | GLY | A | 65 | 37.741 | 35.174 | 64.356 | 1.00 | 61.88 | A | C |
| ATOM | 213 | C | GLY | A | 65 | 37.099 | 33.915 | 64.933 | 1.00 | 61.88 | A | C |
| ATOM | 214 | O | GLY | A | 65 | 37.481 | 32.768 | 64.636 | 1.00 | 61.88 | A | O |
| ATOM | 215 | N | SER | A | 66 | 36.078 | 34.167 | 65.749 | 1.00 | 90.58 | A | N |
| ATOM | 216 | CA | SER | A | 66 | 35.299 | 33.133 | 66.430 | 1.00 | 90.58 | A | C |
| ATOM | 217 | CB | SER | A | 66 | 33.828 | 33.595 | 66.552 | 1.00 | 43.35 | A | C |
| ATOM | 218 | OG | SER | A | 66 | 33.241 | 33.838 | 65.280 | 1.00 | 43.35 | A | O |
| ATOM | 219 | C | SER | A | 66 | 35.351 | 31.689 | 65.871 | 1.00 | 90.58 | A | C |
| ATOM | 220 | O | SER | A | 66 | 35.642 | 30.736 | 66.627 | 1.00 | 90.58 | A | O |
| ATOM | 221 | N | PHE | A | 67 | 35.061 | 31.523 | 64.576 | 1.00 | 88.57 | A | N |
| ATOM | 222 | CA | PHE | A | 67 | 35.082 | 30.188 | 63.957 | 1.00 | 88.57 | A | C |
| ATOM | 223 | CB | PHE | A | 67 | 34.557 | 30.266 | 62.526 | 1.00 | 73.56 | A | C |
| ATOM | 224 | CG | PHE | A | 67 | 34.967 | 31.522 | 61.807 | 1.00 | 73.56 | A | C |
| ATOM | 225 | CD1 | PHE | A | 67 | 36.290 | 31.974 | 61.850 | 1.00 | 73.56 | A | C |
| ATOM | 226 | CD2 | PHE | A | 67 | 34.034 | 32.259 | 61.085 | 1.00 | 73.56 | A | C |
| ATOM | 227 | CE1 | PHE | A | 67 | 36.687 | 33.139 | 61.182 | 1.00 | 73.56 | A | C |
| ATOM | 228 | CE2 | PHE | A | 67 | 34.420 | 33.430 | 60.408 | 1.00 | 73.56 | A | C |
| ATOM | 229 | CZ | PHE | A | 67 | 35.754 | 33.867 | 60.462 | 1.00 | 73.56 | A | C |
| ATOM | 230 | C | PHE | A | 67 | 36.493 | 29.574 | 63.950 | 1.00 | 88.57 | A | C |
| ATOM | 231 | O | PHE | A | 67 | 36.686 | 28.435 | 64.394 | 1.00 | 88.57 | A | O |
| ATOM | 232 | N | GLY | A | 68 | 37.464 | 30.333 | 63.444 | 1.00 | 53.41 | A | N |
| ATOM | 233 | CA | GLY | A | 68 | 38.827 | 29.854 | 63.392 | 1.00 | 53.41 | A | C |
| ATOM | 234 | C | GLY | A | 68 | 39.815 | 30.869 | 62.846 | 1.00 | 53.41 | A | C |
| ATOM | 235 | O | GLY | A | 68 | 39.470 | 32.027 | 62.554 | 1.00 | 53.41 | A | O |
| ATOM | 236 | N | VAL | A | 69 | 41.063 | 30.436 | 62.715 | 1.00 | 53.52 | A | N |
| ATOM | 237 | CA | VAL | A | 69 | 42.080 | 31.320 | 62.191 | 1.00 | 53.52 | A | C |
| ATOM | 238 | CB | VAL | A | 69 | 43.487 | 30.957 | 62.705 | 1.00 | 61.53 | A | C |
| ATOM | 239 | CG1 | VAL | A | 69 | 44.410 | 32.151 | 62.494 | 1.00 | 61.53 | A | C |
| ATOM | 240 | CG2 | VAL | A | 69 | 43.435 | 30.539 | 64.180 | 1.00 | 61.53 | A | C |
| ATOM | 241 | C | VAL | A | 69 | 42.084 | 31.174 | 60.683 | 1.00 | 53.52 | A | C |
| ATOM | 242 | O | VAL | A | 69 | 42.137 | 30.063 | 60.164 | 1.00 | 53.52 | A | O |
| ATOM | 243 | N | VAL | A | 70 | 42.007 | 32.296 | 59.983 | 1.00 | 35.19 | A | N |
| ATOM | 244 | CA | VAL | A | 70 | 42.045 | 32.285 | 58.530 | 1.00 | 35.19 | A | C |
| ATOM | 245 | CB | VAL | A | 70 | 40.999 | 33.209 | 57.947 | 1.00 | 35.93 | A | C |
| ATOM | 246 | CG1 | VAL | A | 70 | 41.201 | 33.323 | 56.445 | 1.00 | 35.93 | A | C |

| | | | | | | | | | | | | |
|---|---|---|---|---|---|---|---|---|---|---|---|---|
| ATOM | 247 | CG2 | VAL | A | 70 | 39.604 | 32.699 | 58.292 | 1.00 | 35.93 | A | C |
| ATOM | 248 | C | VAL | A | 70 | 43.409 | 32.782 | 58.069 | 1.00 | 35.19 | A | C |
| ATOM | 249 | O | VAL | A | 70 | 43.689 | 33.990 | 58.102 | 1.00 | 35.19 | A | O |
| ATOM | 250 | N | TYR | A | 71 | 44.267 | 31.860 | 57.651 | 1.00 | 36.17 | A | N |
| ATOM | 251 | CA | TYR | A | 71 | 45.592 | 32.257 | 57.197 | 1.00 | 36.17 | A | C |
| ATOM | 252 | CB | TYR | A | 71 | 46.628 | 31.164 | 57.449 | 1.00 | 40.57 | A | C |
| ATOM | 253 | CG | TYR | A | 71 | 46.667 | 30.589 | 58.838 | 1.00 | 40.57 | A | C |
| ATOM | 254 | CD1 | TYR | A | 71 | 45.791 | 29.576 | 59.212 | 1.00 | 40.57 | A | C |
| ATOM | 255 | CE1 | TYR | A | 71 | 45.879 | 28.964 | 60.474 | 1.00 | 40.57 | A | C |
| ATOM | 256 | CD2 | TYR | A | 71 | 47.633 | 31.002 | 59.759 | 1.00 | 40.57 | A | C |
| ATOM | 257 | CE2 | TYR | A | 71 | 47.734 | 30.405 | 61.025 | 1.00 | 40.57 | A | C |
| ATOM | 258 | CZ | TYR | A | 71 | 46.851 | 29.380 | 61.380 | 1.00 | 40.57 | A | C |
| ATOM | 259 | OH | TYR | A | 71 | 46.932 | 28.757 | 62.620 | 1.00 | 40.57 | A | O |
| ATOM | 260 | C | TYR | A | 71 | 45.632 | 32.560 | 55.711 | 1.00 | 36.17 | A | C |
| ATOM | 261 | O | TYR | A | 71 | 44.642 | 32.442 | 54.988 | 1.00 | 36.17 | A | O |
| ATOM | 262 | N | GLN | A | 72 | 46.810 | 32.968 | 55.276 | 1.00 | 38.22 | A | N |
| ATOM | 263 | CA | GLN | A | 72 | 47.067 | 33.231 | 53.880 | 1.00 | 38.22 | A | C |
| ATOM | 264 | CB | GLN | A | 72 | 47.265 | 34.717 | 53.600 | 1.00 | 45.37 | A | C |
| ATOM | 265 | CG | GLN | A | 72 | 47.407 | 34.988 | 52.116 | 1.00 | 45.37 | A | C |
| ATOM | 266 | CD | GLN | A | 72 | 48.446 | 36.039 | 51.806 | 1.00 | 45.37 | A | C |
| ATOM | 267 | OE1 | GLN | A | 72 | 49.588 | 35.946 | 52.260 | 1.00 | 45.37 | A | O |
| ATOM | 268 | NE2 | GLN | A | 72 | 48.063 | 37.043 | 51.015 | 1.00 | 45.37 | A | N |
| ATOM | 269 | C | GLN | A | 72 | 48.382 | 32.499 | 53.701 | 1.00 | 38.22 | A | C |
| ATOM | 270 | O | GLN | A | 72 | 49.159 | 32.381 | 54.658 | 1.00 | 38.22 | A | O |
| ATOM | 271 | N | ALA | A | 73 | 48.636 | 32.010 | 52.495 | 1.00 | 32.49 | A | N |
| ATOM | 272 | CA | ALA | A | 73 | 49.855 | 31.269 | 52.231 | 1.00 | 32.49 | A | C |
| ATOM | 273 | CB | ALA | A | 73 | 49.705 | 29.834 | 52.715 | 1.00 | 13.62 | A | C |
| ATOM | 274 | C | ALA | A | 73 | 50.137 | 31.292 | 50.743 | 1.00 | 32.49 | A | C |
| ATOM | 275 | O | ALA | A | 73 | 49.280 | 31.691 | 49.940 | 1.00 | 32.49 | A | O |
| ATOM | 276 | N | LYS | A | 74 | 51.341 | 30.859 | 50.380 | 1.00 | 50.07 | A | N |
| ATOM | 277 | CA | LYS | A | 74 | 51.745 | 30.842 | 48.987 | 1.00 | 50.07 | A | C |
| ATOM | 278 | CB | LYS | A | 74 | 52.955 | 31.764 | 48.770 | 1.00 | 50.67 | A | C |
| ATOM | 279 | CG | LYS | A | 74 | 53.221 | 32.110 | 47.298 | 1.00 | 50.67 | A | C |
| ATOM | 280 | CD | LYS | A | 74 | 54.715 | 32.335 | 47.011 | 1.00 | 50.67 | A | C |
| ATOM | 281 | CE | LYS | A | 74 | 55.482 | 30.996 | 46.923 | 1.00 | 50.67 | A | C |
| ATOM | 282 | NZ | LYS | A | 74 | 56.934 | 31.099 | 46.527 | 1.00 | 50.67 | A | N |
| ATOM | 283 | C | LYS | A | 74 | 52.100 | 29.432 | 48.523 | 1.00 | 50.07 | A | C |
| ATOM | 284 | O | LYS | A | 74 | 52.969 | 28.766 | 49.101 | 1.00 | 50.07 | A | O |
| ATOM | 285 | N | LEU | A | 75 | 51.423 | 28.980 | 47.474 | 1.00 | 36.01 | A | N |
| ATOM | 286 | CA | LEU | A | 75 | 51.707 | 27.670 | 46.931 | 1.00 | 36.01 | A | C |
| ATOM | 287 | CB | LEU | A | 75 | 50.638 | 27.271 | 45.913 | 1.00 | 30.23 | A | C |
| ATOM | 288 | CG | LEU | A | 75 | 49.300 | 26.971 | 46.600 | 1.00 | 30.23 | A | C |
| ATOM | 289 | CD1 | LEU | A | 75 | 48.312 | 26.437 | 45.586 | 1.00 | 30.23 | A | C |
| ATOM | 290 | CD2 | LEU | A | 75 | 49.500 | 25.953 | 47.713 | 1.00 | 30.23 | A | C |
| ATOM | 291 | C | LEU | A | 75 | 53.087 | 27.736 | 46.295 | 1.00 | 36.01 | A | C |
| ATOM | 292 | O | LEU | A | 75 | 53.361 | 28.597 | 45.461 | 1.00 | 36.01 | A | O |
| ATOM | 293 | N | CYS | A | 76 | 53.954 | 26.826 | 46.724 | 1.00 | 56.58 | A | N |
| ATOM | 294 | CA | CYS | A | 76 | 55.327 | 26.748 | 46.246 | 1.00 | 56.58 | A | C |
| ATOM | 295 | CB | CYS | A | 76 | 55.975 | 25.488 | 46.791 | 1.00 | 46.89 | A | C |
| ATOM | 296 | SG | CYS | A | 76 | 56.289 | 25.583 | 48.533 | 1.00 | 46.89 | A | S |
| ATOM | 297 | C | CYS | A | 76 | 55.558 | 26.789 | 44.735 | 1.00 | 56.58 | A | C |
| ATOM | 298 | O | CYS | A | 76 | 56.180 | 27.725 | 44.205 | 1.00 | 56.58 | A | O |
| ATOM | 299 | N | ASP | A | 77 | 55.064 | 25.774 | 44.040 | 1.00 | 58.16 | A | N |
| ATOM | 300 | CA | ASP | A | 77 | 55.283 | 25.691 | 42.610 | 1.00 | 58.16 | A | C |
| ATOM | 301 | CB | ASP | A | 77 | 54.991 | 24.261 | 42.142 | 1.00 | 63.21 | A | C |
| ATOM | 302 | CG | ASP | A | 77 | 55.718 | 23.205 | 43.001 | 1.00 | 63.21 | A | C |
| ATOM | 303 | OD1 | ASP | A | 77 | 56.968 | 23.318 | 43.164 | 1.00 | 63.21 | A | O |
| ATOM | 304 | OD2 | ASP | A | 77 | 55.032 | 22.273 | 43.508 | 1.00 | 63.21 | A | O |
| ATOM | 305 | C | ASP | A | 77 | 54.522 | 26.719 | 41.774 | 1.00 | 58.16 | A | C |
| ATOM | 306 | O | ASP | A | 77 | 55.149 | 27.519 | 41.066 | 1.00 | 58.16 | A | O |
| ATOM | 307 | N | SER | A | 78 | 53.190 | 26.719 | 41.851 | 1.00 | 49.21 | A | N |
| ATOM | 308 | CA | SER | A | 78 | 52.396 | 27.666 | 41.065 | 1.00 | 49.21 | A | C |
| ATOM | 309 | CB | SER | A | 78 | 50.902 | 27.375 | 41.219 | 1.00 | 73.03 | A | C |

| | | | | | | | | | | | | |
|---|---|---|---|---|---|---|---|---|---|---|---|---|
| ATOM | 310 | OG | SER | A | 78 | 50.484 | 27.594 | 42.553 | 1.00 | 73.03 | A | O |
| ATOM | 311 | C | SER | A | 78 | 52.671 | 29.108 | 41.465 | 1.00 | 49.21 | A | C |
| ATOM | 312 | O | SER | A | 78 | 52.558 | 30.019 | 40.645 | 1.00 | 49.21 | A | O |
| ATOM | 313 | N | GLY | A | 79 | 53.048 | 29.309 | 42.723 | 1.00 | 51.16 | A | N |
| ATOM | 314 | CA | GLY | A | 79 | 53.316 | 30.655 | 43.196 | 1.00 | 51.16 | A | C |
| ATOM | 315 | C | GLY | A | 79 | 52.031 | 31.349 | 43.611 | 1.00 | 51.16 | A | C |
| ATOM | 316 | O | GLY | A | 79 | 52.060 | 32.429 | 44.201 | 1.00 | 51.16 | A | O |
| ATOM | 317 | N | GLU | A | 80 | 50.902 | 30.718 | 43.314 | 1.00 | 34.82 | A | N |
| ATOM | 318 | CA | GLU | A | 80 | 49.606 | 31.276 | 43.659 | 1.00 | 34.82 | A | C |
| ATOM | 319 | CB | GLU | A | 80 | 48.495 | 30.330 | 43.237 | 1.00 | 49.18 | A | C |
| ATOM | 320 | CG | GLU | A | 80 | 48.469 | 30.041 | 41.770 | 1.00 | 49.18 | A | C |
| ATOM | 321 | CD | GLU | A | 80 | 47.217 | 29.317 | 41.378 | 1.00 | 49.18 | A | C |
| ATOM | 322 | OE1 | GLU | A | 80 | 47.062 | 29.020 | 40.178 | 1.00 | 49.18 | A | O |
| ATOM | 323 | OE2 | GLU | A | 80 | 46.391 | 29.050 | 42.278 | 1.00 | 49.18 | A | O |
| ATOM | 324 | C | GLU | A | 80 | 49.442 | 31.561 | 45.146 | 1.00 | 34.82 | A | C |
| ATOM | 325 | O | GLU | A | 80 | 50.253 | 31.148 | 45.975 | 1.00 | 34.82 | A | O |
| ATOM | 326 | N | LEU | A | 81 | 48.365 | 32.264 | 45.473 | 1.00 | 33.23 | A | N |
| ATOM | 327 | CA | LEU | A | 81 | 48.065 | 32.617 | 46.854 | 1.00 | 33.23 | A | C |
| ATOM | 328 | CB | LEU | A | 81 | 47.820 | 34.123 | 46.949 | 1.00 | 39.17 | A | C |
| ATOM | 329 | CG | LEU | A | 81 | 49.104 | 34.925 | 46.718 | 1.00 | 39.17 | A | C |
| ATOM | 330 | CD1 | LEU | A | 81 | 48.769 | 36.365 | 46.396 | 1.00 | 39.17 | A | C |
| ATOM | 331 | CD2 | LEU | A | 81 | 49.986 | 34.832 | 47.957 | 1.00 | 39.17 | A | C |
| ATOM | 332 | C | LEU | A | 81 | 46.845 | 31.841 | 47.323 | 1.00 | 33.23 | A | C |
| ATOM | 333 | O | LEU | A | 81 | 45.968 | 31.528 | 46.528 | 1.00 | 33.23 | A | O |
| ATOM | 334 | N | VAL | A | 82 | 46.786 | 31.520 | 48.607 | 1.00 | 25.10 | A | N |
| ATOM | 335 | CA | VAL | A | 82 | 45.649 | 30.767 | 49.108 | 1.00 | 25.10 | A | C |
| ATOM | 336 | CB | VAL | A | 82 | 45.935 | 29.241 | 49.129 | 1.00 | 28.41 | A | C |
| ATOM | 337 | CG1 | VAL | A | 82 | 46.039 | 28.708 | 47.708 | 1.00 | 28.41 | A | C |
| ATOM | 338 | CG2 | VAL | A | 82 | 47.219 | 28.955 | 49.898 | 1.00 | 28.41 | A | C |
| ATOM | 339 | C | VAL | A | 82 | 45.218 | 31.186 | 50.497 | 1.00 | 25.10 | A | C |
| ATOM | 340 | O | VAL | A | 82 | 45.964 | 31.831 | 51.229 | 1.00 | 25.10 | A | O |
| ATOM | 341 | N | ALA | A | 83 | 43.998 | 30.811 | 50.849 | 1.00 | 24.18 | A | N |
| ATOM | 342 | CA | ALA | A | 83 | 43.453 | 31.122 | 52.153 | 1.00 | 24.18 | A | C |
| ATOM | 343 | CB | ALA | A | 83 | 42.165 | 31.897 | 51.999 | 1.00 | 9.22 | A | C |
| ATOM | 344 | C | ALA | A | 83 | 43.205 | 29.806 | 52.883 | 1.00 | 24.18 | A | C |
| ATOM | 345 | O | ALA | A | 83 | 42.644 | 28.860 | 52.319 | 1.00 | 24.18 | A | O |
| ATOM | 346 | N | ILE | A | 84 | 43.636 | 29.737 | 54.135 | 1.00 | 25.59 | A | N |
| ATOM | 347 | CA | ILE | A | 84 | 43.444 | 28.521 | 54.891 | 1.00 | 25.59 | A | C |
| ATOM | 348 | CB | ILE | A | 84 | 44.797 | 27.880 | 55.298 | 1.00 | 38.74 | A | C |
| ATOM | 349 | CG2 | ILE | A | 84 | 44.543 | 26.545 | 56.002 | 1.00 | 38.74 | A | C |
| ATOM | 350 | CG1 | ILE | A | 84 | 45.663 | 27.629 | 54.057 | 1.00 | 38.74 | A | C |
| ATOM | 351 | CD1 | ILE | A | 84 | 47.060 | 27.081 | 54.367 | 1.00 | 38.74 | A | C |
| ATOM | 352 | C | ILE | A | 84 | 42.612 | 28.741 | 56.137 | 1.00 | 25.59 | A | C |
| ATOM | 353 | O | ILE | A | 84 | 43.087 | 29.300 | 57.130 | 1.00 | 25.59 | A | O |
| ATOM | 354 | N | LYS | A | 85 | 41.360 | 28.300 | 56.078 | 1.00 | 43.26 | A | N |
| ATOM | 355 | CA | LYS | A | 85 | 40.464 | 28.420 | 57.220 | 1.00 | 43.26 | A | C |
| ATOM | 356 | CB | LYS | A | 85 | 39.004 | 28.389 | 56.762 | 1.00 | 41.66 | A | C |
| ATOM | 357 | CG | LYS | A | 85 | 38.024 | 28.917 | 57.801 | 1.00 | 41.66 | A | C |
| ATOM | 358 | CD | LYS | A | 85 | 36.711 | 29.375 | 57.167 | 1.00 | 41.66 | A | C |
| ATOM | 359 | CE | LYS | A | 85 | 35.686 | 29.736 | 58.239 | 1.00 | 41.66 | A | C |
| ATOM | 360 | NZ | LYS | A | 85 | 34.414 | 30.256 | 57.658 | 1.00 | 41.66 | A | N |
| ATOM | 361 | C | LYS | A | 85 | 40.802 | 27.215 | 58.085 | 1.00 | 43.26 | A | C |
| ATOM | 362 | O | LYS | A | 85 | 41.007 | 26.115 | 57.571 | 1.00 | 43.26 | A | O |
| ATOM | 363 | N | LYS | A | 86 | 40.879 | 27.413 | 59.392 | 1.00 | 38.38 | A | N |
| ATOM | 364 | CA | LYS | A | 86 | 41.252 | 26.319 | 60.270 | 1.00 | 38.38 | A | C |
| ATOM | 365 | CB | LYS | A | 86 | 42.726 | 26.477 | 60.644 | 1.00 | 38.54 | A | C |
| ATOM | 366 | CG | LYS | A | 86 | 43.332 | 25.333 | 61.395 | 1.00 | 38.54 | A | C |
| ATOM | 367 | CD | LYS | A | 86 | 44.805 | 25.600 | 61.632 | 1.00 | 38.54 | A | C |
| ATOM | 368 | CE | LYS | A | 86 | 45.406 | 24.523 | 62.519 | 1.00 | 38.54 | A | C |
| ATOM | 369 | NZ | LYS | A | 86 | 46.854 | 24.746 | 62.778 | 1.00 | 38.54 | A | N |
| ATOM | 370 | C | LYS | A | 86 | 40.380 | 26.332 | 61.503 | 1.00 | 38.38 | A | C |
| ATOM | 371 | O | LYS | A | 86 | 40.452 | 27.263 | 62.298 | 1.00 | 38.38 | A | O |
| ATOM | 372 | N | VAL | A | 87 | 39.556 | 25.301 | 61.660 | 1.00 | 39.10 | A | N |

| | | | | | | | | | | | | |
|---|---|---|---|---|---|---|---|---|---|---|---|---|
| ATOM | 373 | CA | VAL | A | 87 | 38.656 | 25.215 | 62.802 | 1.00 | 39.10 | A | C |
| ATOM | 374 | CB | VAL | A | 87 | 37.219 | 25.578 | 62.386 | 1.00 | 48.18 | A | C |
| ATOM | 375 | CG1 | VAL | A | 87 | 36.748 | 24.613 | 61.291 | 1.00 | 48.18 | A | C |
| ATOM | 376 | CG2 | VAL | A | 87 | 36.281 | 25.537 | 63.617 | 1.00 | 48.18 | A | C |
| ATOM | 377 | C | VAL | A | 87 | 38.646 | 23.820 | 63.421 | 1.00 | 39.10 | A | C |
| ATOM | 378 | O | VAL | A | 87 | 38.805 | 22.820 | 62.715 | 1.00 | 39.10 | A | O |
| ATOM | 379 | N | LEU | A | 88 | 38.447 | 23.774 | 64.740 | 1.00 | 45.79 | A | N |
| ATOM | 380 | CA | LEU | A | 88 | 38.409 | 22.520 | 65.489 | 1.00 | 45.79 | A | C |
| ATOM | 381 | CB | LEU | A | 88 | 38.125 | 22.778 | 66.968 | 1.00 | 36.83 | A | C |
| ATOM | 382 | CG | LEU | A | 88 | 39.212 | 22.350 | 67.951 | 1.00 | 36.83 | A | C |
| ATOM | 383 | CD1 | LEU | A | 88 | 40.143 | 23.522 | 68.191 | 1.00 | 36.83 | A | C |
| ATOM | 384 | CD2 | LEU | A | 88 | 38.587 | 21.905 | 69.265 | 1.00 | 36.83 | A | C |
| ATOM | 385 | C | LEU | A | 88 | 37.313 | 21.625 | 64.951 | 1.00 | 45.79 | A | C |
| ATOM | 386 | O | LEU | A | 88 | 36.195 | 22.090 | 64.708 | 1.00 | 45.79 | A | O |
| ATOM | 387 | N | GLN | A | 89 | 37.633 | 20.343 | 64.762 | 1.00 | 80.36 | A | N |
| ATOM | 388 | CA | GLN | A | 89 | 36.647 | 19.391 | 64.258 | 1.00 | 80.36 | A | C |
| ATOM | 389 | CB | GLN | A | 89 | 37.075 | 18.811 | 62.903 | 1.00 | 60.59 | A | C |
| ATOM | 390 | CG | GLN | A | 89 | 35.880 | 18.374 | 62.051 | 1.00 | 60.59 | A | C |
| ATOM | 391 | CD | GLN | A | 89 | 34.667 | 19.295 | 62.243 | 1.00 | 60.59 | A | C |
| ATOM | 392 | OE1 | GLN | A | 89 | 34.803 | 20.530 | 62.313 | 1.00 | 60.59 | A | O |
| ATOM | 393 | NE2 | GLN | A | 89 | 33.476 | 18.697 | 62.332 | 1.00 | 60.59 | A | N |
| ATOM | 394 | C | GLN | A | 89 | 36.421 | 18.272 | 65.269 | 1.00 | 80.36 | A | C |
| ATOM | 395 | O | GLN | A | 89 | 37.356 | 17.534 | 65.636 | 1.00 | 80.36 | A | O |
| ATOM | 396 | N | ALA | A | 90 | 35.171 | 18.164 | 65.719 | 1.00100.00 | | A | N |
| ATOM | 397 | CA | ALA | A | 90 | 34.792 | 17.161 | 66.713 | 1.00100.00 | | A | C |
| ATOM | 398 | CB | ALA | A | 90 | 33.628 | 17.688 | 67.589 | 1.00 | 70.85 | A | C |
| ATOM | 399 | C | ALA | A | 90 | 34.413 | 15.816 | 66.103 | 1.00100.00 | | A | C |
| ATOM | 400 | O | ALA | A | 90 | 33.277 | 15.640 | 65.637 | 1.00100.00 | | A | O |
| ATOM | 401 | N | ALA | A | 91 | 35.372 | 14.884 | 66.133 | 1.00 | 72.86 | A | N |
| ATOM | 402 | CA | ALA | A | 91 | 35.225 | 13.520 | 65.612 | 1.00 | 72.86 | A | C |
| ATOM | 403 | CB | ALA | A | 91 | 36.088 | 12.569 | 66.447 | 1.00 | 55.19 | A | C |
| ATOM | 404 | C | ALA | A | 91 | 33.788 | 12.966 | 65.500 | 1.00 | 72.86 | A | C |
| ATOM | 405 | O | ALA | A | 91 | 33.540 | 12.075 | 64.678 | 1.00 | 72.86 | A | O |
| ATOM | 406 | N | ALA | A | 92 | 32.854 | 13.478 | 66.313 | 1.00100.00 | | A | N |
| ATOM | 407 | CA | ALA | A | 92 | 31.444 | 13.036 | 66.275 | 1.00100.00 | | A | C |
| ATOM | 408 | CB | ALA | A | 92 | 30.581 | 13.900 | 67.224 | 1.00 | 35.15 | A | C |
| ATOM | 409 | C | ALA | A | 92 | 30.853 | 13.069 | 64.855 | 1.00100.00 | | A | C |
| ATOM | 410 | O | ALA | A | 92 | 30.889 | 12.064 | 64.131 | 1.00100.00 | | A | O |
| ATOM | 411 | N | ALA | A | 93 | 30.311 | 14.217 | 64.449 | 1.00 | 98.66 | A | N |
| ATOM | 412 | CA | ALA | A | 93 | 29.712 | 14.331 | 63.105 | 1.00 | 98.66 | A | C |
| ATOM | 413 | CB | ALA | A | 93 | 28.389 | 15.125 | 63.172 | 1.00 | 47.13 | A | C |
| ATOM | 414 | C | ALA | A | 93 | 30.632 | 14.968 | 62.061 | 1.00 | 98.66 | A | C |
| ATOM | 415 | O | ALA | A | 93 | 31.743 | 15.431 | 62.369 | 1.00 | 98.66 | A | O |
| ATOM | 416 | N | ALA | A | 94 | 30.150 | 14.990 | 60.821 | 1.00 | 55.10 | A | N |
| ATOM | 417 | CA | ALA | A | 94 | 30.902 | 15.587 | 59.723 | 1.00 | 55.10 | A | C |
| ATOM | 418 | CB | ALA | A | 94 | 30.293 | 15.149 | 58.375 | 1.00 | 28.93 | A | C |
| ATOM | 419 | C | ALA | A | 94 | 30.843 | 17.116 | 59.879 | 1.00 | 55.10 | A | C |
| ATOM | 420 | O | ALA | A | 94 | 29.994 | 17.644 | 60.605 | 1.00 | 55.10 | A | O |
| ATOM | 421 | N | ASN | A | 95 | 31.756 | 17.821 | 59.215 | 1.00 | 42.24 | A | N |
| ATOM | 422 | CA | ASN | A | 95 | 31.772 | 19.278 | 59.287 | 1.00 | 42.24 | A | C |
| ATOM | 423 | CB | ASN | A | 95 | 33.180 | 19.803 | 59.020 | 1.00 | 43.95 | A | C |
| ATOM | 424 | CG | ASN | A | 95 | 33.269 | 21.310 | 59.144 | 1.00 | 43.95 | A | C |
| ATOM | 425 | OD1 | ASN | A | 95 | 33.161 | 22.041 | 58.152 | 1.00 | 43.95 | A | O |
| ATOM | 426 | ND2 | ASN | A | 95 | 33.456 | 21.786 | 60.368 | 1.00 | 43.95 | A | N |
| ATOM | 427 | C | ASN | A | 95 | 30.779 | 19.823 | 58.257 | 1.00 | 42.24 | A | C |
| ATOM | 428 | O | ASN | A | 95 | 30.989 | 19.730 | 57.040 | 1.00 | 42.24 | A | O |
| ATOM | 429 | N | ARG | A | 96 | 29.685 | 20.385 | 58.757 | 1.00 | 40.20 | A | N |
| ATOM | 430 | CA | ARG | A | 96 | 28.633 | 20.903 | 57.893 | 1.00 | 40.20 | A | C |
| ATOM | 431 | CB | ARG | A | 96 | 27.528 | 21.533 | 58.744 | 1.00 | 34.03 | A | C |
| ATOM | 432 | CG | ARG | A | 96 | 26.338 | 21.994 | 57.934 | 1.00 | 34.03 | A | C |
| ATOM | 433 | CD | ARG | A | 96 | 25.357 | 22.797 | 58.777 | 1.00 | 34.03 | A | C |
| ATOM | 434 | NE | ARG | A | 96 | 24.495 | 21.979 | 59.628 | 1.00 | 34.03 | A | N |
| ATOM | 435 | CZ | ARG | A | 96 | 23.716 | 22.472 | 60.589 | 1.00 | 34.03 | A | C |

| | | | | | | | | | | | | |
|---|---|---|---|---|---|---|---|---|---|---|---|---|
| ATOM | 436 | NH1 | ARG | A | 96 | 23.702 | 23.782 | 60.817 | 1.00 | 34.03 | A | N |
| ATOM | 437 | NH2 | ARG | A | 96 | 22.943 | 21.663 | 61.309 | 1.00 | 34.03 | A | N |
| ATOM | 438 | C | ARG | A | 96 | 29.123 | 21.916 | 56.845 | 1.00 | 40.20 | A | C |
| ATOM | 439 | O | ARG | A | 96 | 28.633 | 21.939 | 55.711 | 1.00 | 40.20 | A | O |
| ATOM | 440 | N | GLU | A | 97 | 30.083 | 22.759 | 57.204 | 1.00 | 32.44 | A | N |
| ATOM | 441 | CA | GLU | A | 97 | 30.559 | 23.725 | 56.231 | 1.00 | 32.44 | A | C |
| ATOM | 442 | CB | GLU | A | 97 | 31.494 | 24.746 | 56.894 | 1.00 | 25.68 | A | C |
| ATOM | 443 | CG | GLU | A | 97 | 32.125 | 25.729 | 55.912 | 1.00 | 25.68 | A | C |
| ATOM | 444 | CD | GLU | A | 97 | 32.970 | 26.808 | 56.583 | 1.00 | 25.68 | A | C |
| ATOM | 445 | OE1 | GLU | A | 97 | 33.521 | 26.545 | 57.670 | 1.00 | 25.68 | A | O |
| ATOM | 446 | OE2 | GLU | A | 97 | 33.104 | 27.915 | 56.012 | 1.00 | 25.68 | A | O |
| ATOM | 447 | C | GLU | A | 97 | 31.274 | 23.027 | 55.085 | 1.00 | 32.44 | A | C |
| ATOM | 448 | O | GLU | A | 97 | 31.055 | 23.334 | 53.920 | 1.00 | 32.44 | A | O |
| ATOM | 449 | N | LEU | A | 98 | 32.132 | 22.080 | 55.432 | 1.00 | 33.81 | A | N |
| ATOM | 450 | CA | LEU | A | 98 | 32.893 | 21.325 | 54.449 | 1.00 | 33.81 | A | C |
| ATOM | 451 | CB | LEU | A | 98 | 33.734 | 20.257 | 55.153 | 1.00 | 21.52 | A | C |
| ATOM | 452 | CG | LEU | A | 98 | 34.579 | 19.367 | 54.246 | 1.00 | 21.52 | A | C |
| ATOM | 453 | CD1 | LEU | A | 98 | 35.460 | 20.225 | 53.359 | 1.00 | 21.52 | A | C |
| ATOM | 454 | CD2 | LEU | A | 98 | 35.414 | 18.444 | 55.103 | 1.00 | 21.52 | A | C |
| ATOM | 455 | C | LEU | A | 98 | 31.961 | 20.654 | 53.463 | 1.00 | 33.81 | A | C |
| ATOM | 456 | O | LEU | A | 98 | 32.127 | 20.781 | 52.250 | 1.00 | 33.81 | A | O |
| ATOM | 457 | N | GLN | A | 99 | 30.975 | 19.939 | 53.995 | 1.00 | 43.72 | A | N |
| ATOM | 458 | CA | GLN | A | 99 | 30.032 | 19.229 | 53.149 | 1.00 | 43.72 | A | C |
| ATOM | 459 | CB | GLN | A | 99 | 29.110 | 18.347 | 54.000 | 1.00 | 63.84 | A | C |
| ATOM | 460 | CG | GLN | A | 99 | 29.860 | 17.148 | 54.642 | 1.00 | 63.84 | A | C |
| ATOM | 461 | CD | GLN | A | 99 | 30.944 | 16.519 | 53.712 | 1.00 | 63.84 | A | C |
| ATOM | 462 | OE1 | GLN | A | 99 | 30.659 | 16.109 | 52.574 | 1.00 | 63.84 | A | O |
| ATOM | 463 | NE2 | GLN | A | 99 | 32.186 | 16.447 | 54.211 | 1.00 | 63.84 | A | N |
| ATOM | 464 | C | GLN | A | 99 | 29.248 | 20.127 | 52.197 | 1.00 | 43.72 | A | C |
| ATOM | 465 | O | GLN | A | 99 | 28.800 | 19.665 | 51.150 | 1.00 | 43.72 | A | O |
| ATOM | 466 | N | ILE | A | 100 | 29.094 | 21.407 | 52.537 | 1.00 | 26.77 | A | N |
| ATOM | 467 | CA | ILE | A | 100 | 28.402 | 22.335 | 51.647 | 1.00 | 26.77 | A | C |
| ATOM | 468 | CB | ILE | A | 100 | 27.806 | 23.528 | 52.415 | 1.00 | 17.29 | A | C |
| ATOM | 469 | CG2 | ILE | A | 100 | 27.498 | 24.666 | 51.454 | 1.00 | 17.29 | A | C |
| ATOM | 470 | CG1 | ILE | A | 100 | 26.541 | 23.073 | 53.152 | 1.00 | 17.29 | A | C |
| ATOM | 471 | CD1 | ILE | A | 100 | 25.813 | 24.154 | 53.909 | 1.00 | 17.29 | A | C |
| ATOM | 472 | C | ILE | A | 100 | 29.392 | 22.834 | 50.597 | 1.00 | 26.77 | A | C |
| ATOM | 473 | O | ILE | A | 100 | 29.118 | 22.755 | 49.396 | 1.00 | 26.77 | A | O |
| ATOM | 474 | N | MET | A | 101 | 30.544 | 23.329 | 51.052 | 1.00 | 25.73 | A | N |
| ATOM | 475 | CA | MET | A | 101 | 31.596 | 23.804 | 50.151 | 1.00 | 25.73 | A | C |
| ATOM | 476 | CB | MET | A | 101 | 32.885 | 24.062 | 50.941 | 1.00 | 42.87 | A | C |
| ATOM | 477 | CG | MET | A | 101 | 32.812 | 25.234 | 51.924 | 1.00 | 42.87 | A | C |
| ATOM | 478 | SD | MET | A | 101 | 32.199 | 26.731 | 51.113 | 1.00 | 42.87 | A | S |
| ATOM | 479 | CE | MET | A | 101 | 33.605 | 27.161 | 50.113 | 1.00 | 42.87 | A | C |
| ATOM | 480 | C | MET | A | 101 | 31.858 | 22.762 | 49.051 | 1.00 | 25.73 | A | C |
| ATOM | 481 | O | MET | A | 101 | 31.852 | 23.063 | 47.854 | 1.00 | 25.73 | A | O |
| ATOM | 482 | N | ARG | A | 102 | 32.079 | 21.526 | 49.488 | 1.00 | 39.47 | A | N |
| ATOM | 483 | CA | ARG | A | 102 | 32.334 | 20.386 | 48.613 | 1.00 | 39.47 | A | C |
| ATOM | 484 | CB | ARG | A | 102 | 32.312 | 19.107 | 49.465 | 1.00 | 46.50 | A | C |
| ATOM | 485 | CG | ARG | A | 102 | 33.668 | 18.567 | 49.903 | 1.00 | 46.50 | A | C |
| ATOM | 486 | CD | ARG | A | 102 | 34.159 | 17.565 | 48.859 | 1.00 | 46.50 | A | C |
| ATOM | 487 | NE | ARG | A | 102 | 34.683 | 16.322 | 49.426 | 1.00 | 46.50 | A | N |
| ATOM | 488 | CZ | ARG | A | 102 | 34.103 | 15.632 | 50.408 | 1.00 | 46.50 | A | C |
| ATOM | 489 | NH1 | ARG | A | 102 | 32.969 | 16.060 | 50.955 | 1.00 | 46.50 | A | N |
| ATOM | 490 | NH2 | ARG | A | 102 | 34.652 | 14.500 | 50.840 | 1.00 | 46.50 | A | N |
| ATOM | 491 | C | ARG | A | 102 | 31.338 | 20.254 | 47.451 | 1.00 | 39.47 | A | C |
| ATOM | 492 | O | ARG | A | 102 | 31.656 | 19.646 | 46.435 | 1.00 | 39.47 | A | O |
| ATOM | 493 | N | LYS | A | 103 | 30.143 | 20.813 | 47.576 | 1.00 | 23.86 | A | N |
| ATOM | 494 | CA | LYS | A | 103 | 29.200 | 20.676 | 46.481 | 1.00 | 23.86 | A | C |
| ATOM | 495 | CB | LYS | A | 103 | 27.917 | 19.997 | 46.965 | 1.00 | 68.26 | A | C |
| ATOM | 496 | CG | LYS | A | 103 | 27.098 | 20.791 | 47.962 | 1.00 | 68.26 | A | C |
| ATOM | 497 | CD | LYS | A | 103 | 25.982 | 19.924 | 48.567 | 1.00 | 68.26 | A | C |
| ATOM | 498 | CE | LYS | A | 103 | 25.188 | 20.694 | 49.632 | 1.00 | 68.26 | A | C |

| | | | | | | | | | | | | |
|---|---|---|---|---|---|---|---|---|---|---|---|---|
| ATOM | 499 | NZ | LYS | A | 103 | 24.281 | 19.856 | 50.495 | 1.00 | 68.26 | A | N |
| ATOM | 500 | C | LYS | A | 103 | 28.853 | 21.953 | 45.750 | 1.00 | 23.86 | A | C |
| ATOM | 501 | O | LYS | A | 103 | 27.944 | 21.957 | 44.933 | 1.00 | 23.86 | A | O |
| ATOM | 502 | N | LEU | A | 104 | 29.580 | 23.030 | 46.027 | 1.00 | 25.37 | A | N |
| ATOM | 503 | CA | LEU | A | 104 | 29.317 | 24.312 | 45.378 | 1.00 | 25.37 | A | C |
| ATOM | 504 | CB | LEU | A | 104 | 29.259 | 25.433 | 46.419 | 1.00 | 33.38 | A | C |
| ATOM | 505 | CG | LEU | A | 104 | 27.917 | 25.775 | 47.082 | 1.00 | 33.38 | A | C |
| ATOM | 506 | CD1 | LEU | A | 104 | 27.308 | 24.557 | 47.747 | 1.00 | 33.38 | A | C |
| ATOM | 507 | CD2 | LEU | A | 104 | 28.133 | 26.871 | 48.093 | 1.00 | 33.38 | A | C |
| ATOM | 508 | C | LEU | A | 104 | 30.367 | 24.650 | 44.343 | 1.00 | 25.37 | A | C |
| ATOM | 509 | O | LEU | A | 104 | 31.553 | 24.437 | 44.569 | 1.00 | 25.37 | A | O |
| ATOM | 510 | N | ASP | A | 105 | 29.921 | 25.167 | 43.204 | 1.00 | 39.46 | A | N |
| ATOM | 511 | CA | ASP | A | 105 | 30.826 | 25.574 | 42.139 | 1.00 | 39.46 | A | C |
| ATOM | 512 | CB | ASP | A | 105 | 31.168 | 24.383 | 41.242 | 1.00 | 40.28 | A | C |
| ATOM | 513 | CG | ASP | A | 105 | 32.215 | 24.725 | 40.193 | 1.00 | 40.28 | A | C |
| ATOM | 514 | OD1 | ASP | A | 105 | 33.221 | 25.378 | 40.549 | 1.00 | 40.28 | A | O |
| ATOM | 515 | OD2 | ASP | A | 105 | 32.035 | 24.329 | 39.020 | 1.00 | 40.28 | A | O |
| ATOM | 516 | C | ASP | A | 105 | 30.154 | 26.676 | 41.324 | 1.00 | 39.46 | A | C |
| ATOM | 517 | O | ASP | A | 105 | 29.374 | 26.400 | 40.404 | 1.00 | 39.46 | A | O |
| ATOM | 518 | N | HIS | A | 106 | 30.455 | 27.924 | 41.687 | 1.00 | 26.69 | A | N |
| ATOM | 519 | CA | HIS | A | 106 | 29.908 | 29.100 | 41.018 | 1.00 | 26.69 | A | C |
| ATOM | 520 | CB | HIS | A | 106 | 28.713 | 29.624 | 41.809 | 1.00 | 20.40 | A | C |
| ATOM | 521 | CG | HIS | A | 106 | 27.887 | 30.632 | 41.078 | 1.00 | 20.40 | A | C |
| ATOM | 522 | CD2 | HIS | A | 106 | 26.642 | 30.548 | 40.549 | 1.00 | 20.40 | A | C |
| ATOM | 523 | ND1 | HIS | A | 106 | 28.308 | 31.924 | 40.860 | 1.00 | 20.40 | A | N |
| ATOM | 524 | CE1 | HIS | A | 106 | 27.357 | 32.593 | 40.234 | 1.00 | 20.40 | A | C |
| ATOM | 525 | NE2 | HIS | A | 106 | 26.333 | 31.781 | 40.035 | 1.00 | 20.40 | A | N |
| ATOM | 526 | C | HIS | A | 106 | 31.020 | 30.150 | 40.944 | 1.00 | 26.69 | A | C |
| ATOM | 527 | O | HIS | A | 106 | 31.847 | 30.258 | 41.846 | 1.00 | 26.69 | A | O |
| ATOM | 528 | N | CYS | A | 107 | 31.048 | 30.913 | 39.860 | 1.00 | 30.52 | A | N |
| ATOM | 529 | CA | CYS | A | 107 | 32.075 | 31.922 | 39.678 | 1.00 | 30.52 | A | C |
| ATOM | 530 | CB | CYS | A | 107 | 31.957 | 32.542 | 38.287 | 1.00 | 46.42 | A | C |
| ATOM | 531 | SG | CYS | A | 107 | 30.360 | 33.343 | 37.933 | 1.00 | 46.42 | A | S |
| ATOM | 532 | C | CYS | A | 107 | 32.001 | 33.001 | 40.736 | 1.00 | 30.52 | A | C |
| ATOM | 533 | O | CYS | A | 107 | 32.953 | 33.762 | 40.917 | 1.00 | 30.52 | A | O |
| ATOM | 534 | N | ASN | A | 108 | 30.876 | 33.052 | 41.446 | 1.00 | 31.80 | A | N |
| ATOM | 535 | CA | ASN | A | 108 | 30.663 | 34.055 | 42.483 | 1.00 | 31.80 | A | C |
| ATOM | 536 | CB | ASN | A | 108 | 29.335 | 34.771 | 42.226 | 1.00 | 33.24 | A | C |
| ATOM | 537 | CG | ASN | A | 108 | 29.405 | 35.715 | 41.041 | 1.00 | 33.24 | A | C |
| ATOM | 538 | OD1 | ASN | A | 108 | 28.483 | 35.778 | 40.229 | 1.00 | 33.24 | A | O |
| ATOM | 539 | ND2 | ASN | A | 108 | 30.499 | 36.464 | 40.943 | 1.00 | 33.24 | A | N |
| ATOM | 540 | C | ASN | A | 108 | 30.721 | 33.525 | 43.914 | 1.00 | 31.80 | A | C |
| ATOM | 541 | O | ASN | A | 108 | 30.172 | 34.126 | 44.828 | 1.00 | 31.80 | A | O |
| ATOM | 542 | N | ILE | A | 109 | 31.388 | 32.399 | 44.103 | 1.00 | 16.97 | A | N |
| ATOM | 543 | CA | ILE | A | 109 | 31.545 | 31.805 | 45.422 | 1.00 | 16.97 | A | C |
| ATOM | 544 | CB | ILE | A | 109 | 30.613 | 30.570 | 45.587 | 1.00 | 38.56 | A | C |
| ATOM | 545 | CG2 | ILE | A | 109 | 30.801 | 29.928 | 46.963 | 1.00 | 38.56 | A | C |
| ATOM | 546 | CG1 | ILE | A | 109 | 29.155 | 31.006 | 45.405 | 1.00 | 38.56 | A | C |
| ATOM | 547 | CD1 | ILE | A | 109 | 28.117 | 29.936 | 45.719 | 1.00 | 38.56 | A | C |
| ATOM | 548 | C | ILE | A | 109 | 33.018 | 31.387 | 45.431 | 1.00 | 16.97 | A | C |
| ATOM | 549 | O | ILE | A | 109 | 33.549 | 30.961 | 44.395 | 1.00 | 16.97 | A | O |
| ATOM | 550 | N | VAL | A | 110 | 33.691 | 31.527 | 46.572 | 1.00 | 21.69 | A | N |
| ATOM | 551 | CA | VAL | A | 110 | 35.102 | 31.160 | 46.633 | 1.00 | 21.69 | A | C |
| ATOM | 552 | CB | VAL | A | 110 | 35.777 | 31.522 | 47.982 | 1.00 | 27.20 | A | C |
| ATOM | 553 | CG1 | VAL | A | 110 | 37.199 | 32.013 | 47.727 | 1.00 | 27.20 | A | C |
| ATOM | 554 | CG2 | VAL | A | 110 | 34.969 | 32.534 | 48.725 | 1.00 | 27.20 | A | C |
| ATOM | 555 | C | VAL | A | 110 | 35.242 | 29.661 | 46.455 | 1.00 | 21.69 | A | C |
| ATOM | 556 | O | VAL | A | 110 | 34.499 | 28.889 | 47.054 | 1.00 | 21.69 | A | O |
| ATOM | 557 | N | ARG | A | 111 | 36.206 | 29.252 | 45.644 | 1.00 | 21.09 | A | N |
| ATOM | 558 | CA | ARG | A | 111 | 36.412 | 27.839 | 45.407 | 1.00 | 21.09 | A | C |
| ATOM | 559 | CB | ARG | A | 111 | 37.060 | 27.592 | 44.039 | 1.00 | 52.23 | A | C |
| ATOM | 560 | CG | ARG | A | 111 | 37.444 | 26.125 | 43.817 | 1.00 | 52.23 | A | C |
| ATOM | 561 | CD | ARG | A | 111 | 37.957 | 25.834 | 42.403 | 1.00 | 52.23 | A | C |

| | | | | | | | | | | | | |
|---|---|---|---|---|---|---|---|---|---|---|---|---|
| ATOM | 562 | NE | ARG | A | 111 | 39.020 | 26.756 | 41.989 | 1.00 | 52.23 | A | N |
| ATOM | 563 | CZ | ARG | A | 111 | 40.227 | 26.383 | 41.555 | 1.00 | 52.23 | A | C |
| ATOM | 564 | NH1 | ARG | A | 111 | 40.544 | 25.088 | 41.473 | 1.00 | 52.23 | A | N |
| ATOM | 565 | NH2 | ARG | A | 111 | 41.119 | 27.311 | 41.202 | 1.00 | 52.23 | A | N |
| ATOM | 566 | C | ARG | A | 111 | 37.269 | 27.191 | 46.467 | 1.00 | 21.09 | A | C |
| ATOM | 567 | O | ARG | A | 111 | 38.293 | 27.728 | 46.892 | 1.00 | 21.09 | A | O |
| ATOM | 568 | N | LEU | A | 112 | 36.831 | 26.014 | 46.880 | 1.00 | 29.79 | A | N |
| ATOM | 569 | CA | LEU | A | 112 | 37.547 | 25.223 | 47.864 | 1.00 | 29.79 | A | C |
| ATOM | 570 | CB | LEU | A | 112 | 36.555 | 24.348 | 48.641 | 1.00 | 21.10 | A | C |
| ATOM | 571 | CG | LEU | A | 112 | 37.162 | 23.388 | 49.656 | 1.00 | 21.10 | A | C |
| ATOM | 572 | CD1 | LEU | A | 112 | 37.859 | 24.193 | 50.747 | 1.00 | 21.10 | A | C |
| ATOM | 573 | CD2 | LEU | A | 112 | 36.079 | 22.494 | 50.233 | 1.00 | 21.10 | A | C |
| ATOM | 574 | C | LEU | A | 112 | 38.520 | 24.359 | 47.059 | 1.00 | 29.79 | A | C |
| ATOM | 575 | O | LEU | A | 112 | 38.096 | 23.475 | 46.336 | 1.00 | 29.79 | A | O |
| ATOM | 576 | N | ARG | A | 113 | 39.816 | 24.612 | 47.159 | 1.00 | 21.31 | A | N |
| ATOM | 577 | CA | ARG | A | 113 | 40.747 | 23.816 | 46.380 | 1.00 | 21.31 | A | C |
| ATOM | 578 | CB | ARG | A | 113 | 42.104 | 24.509 | 46.261 | 1.00 | 42.03 | A | C |
| ATOM | 579 | CG | ARG | A | 113 | 42.106 | 26.005 | 46.555 | 1.00 | 42.03 | A | C |
| ATOM | 580 | CD | ARG | A | 113 | 42.669 | 26.861 | 45.411 | 1.00 | 42.03 | A | C |
| ATOM | 581 | NE | ARG | A | 113 | 43.873 | 26.293 | 44.811 | 1.00 | 42.03 | A | N |
| ATOM | 582 | CZ | ARG | A | 113 | 44.660 | 26.940 | 43.958 | 1.00 | 42.03 | A | C |
| ATOM | 583 | NH1 | ARG | A | 113 | 44.368 | 28.192 | 43.619 | 1.00 | 42.03 | A | N |
| ATOM | 584 | NH2 | ARG | A | 113 | 45.709 | 26.319 | 43.419 | 1.00 | 42.03 | A | N |
| ATOM | 585 | C | ARG | A | 113 | 40.936 | 22.454 | 47.032 | 1.00 | 21.31 | A | C |
| ATOM | 586 | O | ARG | A | 113 | 40.527 | 21.423 | 46.508 | 1.00 | 21.31 | A | O |
| ATOM | 587 | N | TYR | A | 114 | 41.564 | 22.468 | 48.197 | 1.00 | 36.43 | A | N |
| ATOM | 588 | CA | TYR | A | 114 | 41.824 | 21.258 | 48.942 | 1.00 | 36.43 | A | C |
| ATOM | 589 | CB | TYR | A | 114 | 43.329 | 20.974 | 48.972 | 1.00 | 43.26 | A | C |
| ATOM | 590 | CG | TYR | A | 114 | 44.118 | 21.443 | 47.757 | 1.00 | 43.26 | A | C |
| ATOM | 591 | CD1 | TYR | A | 114 | 44.086 | 20.743 | 46.544 | 1.00 | 43.26 | A | C |
| ATOM | 592 | CE1 | TYR | A | 114 | 44.856 | 21.157 | 45.442 | 1.00 | 43.26 | A | C |
| ATOM | 593 | CD2 | TYR | A | 114 | 44.933 | 22.571 | 47.836 | 1.00 | 43.26 | A | C |
| ATOM | 594 | CE2 | TYR | A | 114 | 45.704 | 22.997 | 46.745 | 1.00 | 43.26 | A | C |
| ATOM | 595 | CZ | TYR | A | 114 | 45.662 | 22.289 | 45.553 | 1.00 | 43.26 | A | C |
| ATOM | 596 | OH | TYR | A | 114 | 46.409 | 22.740 | 44.479 | 1.00 | 43.26 | A | O |
| ATOM | 597 | C | TYR | A | 114 | 41.345 | 21.424 | 50.370 | 1.00 | 36.43 | A | C |
| ATOM | 598 | O | TYR | A | 114 | 40.906 | 22.494 | 50.766 | 1.00 | 36.43 | A | O |
| ATOM | 599 | N | PHE | A | 115 | 41.436 | 20.346 | 51.135 | 1.00 | 37.36 | A | N |
| ATOM | 600 | CA | PHE | A | 115 | 41.071 | 20.360 | 52.546 | 1.00 | 37.36 | A | C |
| ATOM | 601 | CB | PHE | A | 115 | 39.547 | 20.330 | 52.727 | 1.00 | 31.41 | A | C |
| ATOM | 602 | CG | PHE | A | 115 | 38.928 | 18.984 | 52.537 | 1.00 | 31.41 | A | C |
| ATOM | 603 | CD1 | PHE | A | 115 | 38.855 | 18.086 | 53.589 | 1.00 | 31.41 | A | C |
| ATOM | 604 | CD2 | PHE | A | 115 | 38.420 | 18.614 | 51.304 | 1.00 | 31.41 | A | C |
| ATOM | 605 | CE1 | PHE | A | 115 | 38.272 | 16.838 | 53.418 | 1.00 | 31.41 | A | C |
| ATOM | 606 | CE2 | PHE | A | 115 | 37.834 | 17.370 | 51.117 | 1.00 | 31.41 | A | C |
| ATOM | 607 | CZ | PHE | A | 115 | 37.763 | 16.479 | 52.175 | 1.00 | 31.41 | A | C |
| ATOM | 608 | C | PHE | A | 115 | 41.731 | 19.130 | 53.161 | 1.00 | 37.36 | A | C |
| ATOM | 609 | O | PHE | A | 115 | 41.610 | 18.021 | 52.640 | 1.00 | 37.36 | A | O |
| ATOM | 610 | N | PHE | A | 116 | 42.458 | 19.345 | 54.252 | 1.00 | 34.91 | A | N |
| ATOM | 611 | CA | PHE | A | 116 | 43.171 | 18.272 | 54.925 | 1.00 | 34.91 | A | C |
| ATOM | 612 | CB | PHE | A | 116 | 44.667 | 18.386 | 54.615 | 1.00 | 35.65 | A | C |
| ATOM | 613 | CG | PHE | A | 116 | 45.257 | 19.708 | 54.983 | 1.00 | 35.65 | A | C |
| ATOM | 614 | CD1 | PHE | A | 116 | 45.927 | 19.871 | 56.189 | 1.00 | 35.65 | A | C |
| ATOM | 615 | CD2 | PHE | A | 116 | 45.115 | 20.802 | 54.143 | 1.00 | 35.65 | A | C |
| ATOM | 616 | CE1 | PHE | A | 116 | 46.445 | 21.108 | 56.559 | 1.00 | 35.65 | A | C |
| ATOM | 617 | CE2 | PHE | A | 116 | 45.630 | 22.052 | 54.504 | 1.00 | 35.65 | A | C |
| ATOM | 618 | CZ | PHE | A | 116 | 46.298 | 22.203 | 55.715 | 1.00 | 35.65 | A | C |
| ATOM | 619 | C | PHE | A | 116 | 42.912 | 18.295 | 56.428 | 1.00 | 34.91 | A | C |
| ATOM | 620 | O | PHE | A | 116 | 42.072 | 19.059 | 56.901 | 1.00 | 34.91 | A | O |
| ATOM | 621 | N | TYR | A | 117 | 43.644 | 17.484 | 57.186 | 1.00 | 53.15 | A | N |
| ATOM | 622 | CA | TYR | A | 117 | 43.399 | 17.431 | 58.624 | 1.00 | 53.15 | A | C |
| ATOM | 623 | CB | TYR | A | 117 | 42.578 | 16.176 | 58.938 | 1.00 | 28.74 | A | C |
| ATOM | 624 | CG | TYR | A | 117 | 41.117 | 16.232 | 58.572 | 1.00 | 28.74 | A | C |

| | | | | | | | | | | | | |
|---|---|---|---|---|---|---|---|---|---|---|---|---|
| ATOM | 625 | CD1 | TYR | A | 117 | 40.200 | 16.914 | 59.378 | 1.00 | 28.74 | A | C |
| ATOM | 626 | CE1 | TYR | A | 117 | 38.818 | 16.894 | 59.096 | 1.00 | 28.74 | A | C |
| ATOM | 627 | CD2 | TYR | A | 117 | 40.631 | 15.536 | 57.462 | 1.00 | 28.74 | A | C |
| ATOM | 628 | CE2 | TYR | A | 117 | 39.252 | 15.507 | 57.165 | 1.00 | 28.74 | A | C |
| ATOM | 629 | CZ | TYR | A | 117 | 38.351 | 16.184 | 57.991 | 1.00 | 28.74 | A | C |
| ATOM | 630 | OH | TYR | A | 117 | 36.993 | 16.112 | 57.735 | 1.00 | 28.74 | A | O |
| ATOM | 631 | C | TYR | A | 117 | 44.506 | 17.521 | 59.688 | 1.00 | 53.15 | A | C |
| ATOM | 632 | O | TYR | A | 117 | 44.991 | 16.494 | 60.165 | 1.00 | 53.15 | A | O |
| ATOM | 633 | N | SER | A | 118 | 44.911 | 18.734 | 60.057 | 1.00 | 41.96 | A | N |
| ATOM | 634 | CA | SER | A | 118 | 45.858 | 18.902 | 61.164 | 1.00 | 41.96 | A | C |
| ATOM | 635 | CB | SER | A | 118 | 46.606 | 20.245 | 61.054 | 1.00 | 60.34 | A | C |
| ATOM | 636 | OG | SER | A | 118 | 46.934 | 20.788 | 62.339 | 1.00 | 60.34 | A | O |
| ATOM | 637 | C | SER | A | 118 | 44.736 | 19.007 | 62.243 | 1.00 | 41.96 | A | C |
| ATOM | 638 | O | SER | A | 118 | 44.798 | 18.354 | 63.295 | 1.00 | 41.96 | A | O |
| ATOM | 639 | N | TYR | A | 127 | 43.730 | 19.853 | 61.897 | 1.00 | 90.04 | A | N |
| ATOM | 640 | CA | TYR | A | 127 | 42.417 | 20.205 | 62.567 | 1.00 | 90.04 | A | C |
| ATOM | 641 | CB | TYR | A | 127 | 42.329 | 21.690 | 62.942 | 1.00 | 72.26 | A | C |
| ATOM | 642 | CG | TYR | A | 127 | 42.884 | 22.103 | 64.288 | 1.00 | 72.26 | A | C |
| ATOM | 643 | CD1 | TYR | A | 127 | 42.025 | 22.436 | 65.337 | 1.00 | 72.26 | A | C |
| ATOM | 644 | CE1 | TYR | A | 127 | 42.521 | 22.889 | 66.558 | 1.00 | 72.26 | A | C |
| ATOM | 645 | CD2 | TYR | A | 127 | 44.266 | 22.228 | 64.499 | 1.00 | 72.26 | A | C |
| ATOM | 646 | CE2 | TYR | A | 127 | 44.774 | 22.684 | 65.729 | 1.00 | 72.26 | A | C |
| ATOM | 647 | CZ | TYR | A | 127 | 43.887 | 23.011 | 66.750 | 1.00 | 72.26 | A | C |
| ATOM | 648 | OH | TYR | A | 127 | 44.348 | 23.467 | 67.969 | 1.00 | 72.26 | A | O |
| ATOM | 649 | C | TYR | A | 127 | 41.712 | 20.069 | 61.211 | 1.00 | 90.04 | A | C |
| ATOM | 650 | O | TYR | A | 127 | 42.157 | 19.274 | 60.381 | 1.00 | 90.04 | A | O |
| ATOM | 651 | N | LEU | A | 128 | 40.660 | 20.835 | 60.941 | 1.00 | 31.65 | A | N |
| ATOM | 652 | CA | LEU | A | 128 | 40.057 | 20.769 | 59.604 | 1.00 | 31.65 | A | C |
| ATOM | 653 | CB | LEU | A | 128 | 38.530 | 20.804 | 59.660 | 1.00 | 33.85 | A | C |
| ATOM | 654 | CG | LEU | A | 128 | 37.915 | 21.066 | 58.272 | 1.00 | 33.85 | A | C |
| ATOM | 655 | CD1 | LEU | A | 128 | 38.331 | 19.977 | 57.291 | 1.00 | 33.85 | A | C |
| ATOM | 656 | CD2 | LEU | A | 128 | 36.418 | 21.122 | 58.371 | 1.00 | 33.85 | A | C |
| ATOM | 657 | C | LEU | A | 128 | 40.542 | 22.011 | 58.863 | 1.00 | 31.65 | A | C |
| ATOM | 658 | O | LEU | A | 128 | 40.159 | 23.123 | 59.202 | 1.00 | 31.65 | A | O |
| ATOM | 659 | N | ASN | A | 129 | 41.380 | 21.841 | 57.850 | 1.00 | 44.05 | A | N |
| ATOM | 660 | CA | ASN | A | 129 | 41.901 | 23.004 | 57.124 | 1.00 | 44.05 | A | C |
| ATOM | 661 | CB | ASN | A | 129 | 43.416 | 22.882 | 56.989 | 1.00 | 34.27 | A | C |
| ATOM | 662 | CG | ASN | A | 129 | 44.098 | 22.637 | 58.329 | 1.00 | 34.27 | A | C |
| ATOM | 663 | OD1 | ASN | A | 129 | 43.718 | 21.735 | 59.080 | 1.00 | 34.27 | A | O |
| ATOM | 664 | ND2 | ASN | A | 129 | 45.112 | 23.435 | 58.631 | 1.00 | 34.27 | A | N |
| ATOM | 665 | C | ASN | A | 129 | 41.268 | 23.164 | 55.749 | 1.00 | 44.05 | A | C |
| ATOM | 666 | O | ASN | A | 129 | 41.319 | 22.252 | 54.931 | 1.00 | 44.05 | A | O |
| ATOM | 667 | N | LEU | A | 130 | 40.664 | 24.317 | 55.490 | 1.00 | 31.79 | A | N |
| ATOM | 668 | CA | LEU | A | 130 | 40.034 | 24.551 | 54.198 | 1.00 | 31.79 | A | C |
| ATOM | 669 | CB | LEU | A | 130 | 38.636 | 25.152 | 54.376 | 1.00 | 30.77 | A | C |
| ATOM | 670 | CG | LEU | A | 130 | 37.513 | 24.314 | 55.000 | 1.00 | 30.77 | A | C |
| ATOM | 671 | CD1 | LEU | A | 130 | 36.439 | 24.075 | 53.966 | 1.00 | 30.77 | A | C |
| ATOM | 672 | CD2 | LEU | A | 130 | 38.042 | 22.986 | 55.512 | 1.00 | 30.77 | A | C |
| ATOM | 673 | C | LEU | A | 130 | 40.873 | 25.485 | 53.339 | 1.00 | 31.79 | A | C |
| ATOM | 674 | O | LEU | A | 130 | 40.950 | 26.681 | 53.609 | 1.00 | 31.79 | A | O |
| ATOM | 675 | N | VAL | A | 131 | 41.500 | 24.937 | 52.302 | 1.00 | 34.74 | A | N |
| ATOM | 676 | CA | VAL | A | 131 | 42.322 | 25.732 | 51.392 | 1.00 | 34.74 | A | C |
| ATOM | 677 | CB | VAL | A | 131 | 43.483 | 24.894 | 50.776 | 1.00 | 25.96 | A | C |
| ATOM | 678 | CG1 | VAL | A | 131 | 44.545 | 25.816 | 50.192 | 1.00 | 25.96 | A | C |
| ATOM | 679 | CG2 | VAL | A | 131 | 44.097 | 23.984 | 51.827 | 1.00 | 25.96 | A | C |
| ATOM | 680 | C | VAL | A | 131 | 41.424 | 26.226 | 50.258 | 1.00 | 34.74 | A | C |
| ATOM | 681 | O | VAL | A | 131 | 40.990 | 25.434 | 49.421 | 1.00 | 34.74 | A | O |
| ATOM | 682 | N | LEU | A | 132 | 41.134 | 27.529 | 50.246 | 1.00 | 30.04 | A | N |
| ATOM | 683 | CA | LEU | A | 132 | 40.282 | 28.123 | 49.212 | 1.00 | 30.04 | A | C |
| ATOM | 684 | CB | LEU | A | 132 | 39.040 | 28.764 | 49.838 | 1.00 | 13.89 | A | C |
| ATOM | 685 | CG | LEU | A | 132 | 38.323 | 28.015 | 50.967 | 1.00 | 13.89 | A | C |
| ATOM | 686 | CD1 | LEU | A | 132 | 38.956 | 28.385 | 52.306 | 1.00 | 13.89 | A | C |
| ATOM | 687 | CD2 | LEU | A | 132 | 36.847 | 28.373 | 50.980 | 1.00 | 13.89 | A | C |

| | | | | | | | | | | | | |
|---|---|---|---|---|---|---|---|---|---|---|---|---|
| ATOM | 688 | C | LEU | A | 132 | 41.070 | 29.194 | 48.476 | 1.00 | 30.04 | A | C |
| ATOM | 689 | O | LEU | A | 132 | 42.142 | 29.595 | 48.932 | 1.00 | 30.04 | A | O |
| ATOM | 690 | N | ASP | A | 133 | 40.556 | 29.654 | 47.338 | 1.00 | 29.79 | A | N |
| ATOM | 691 | CA | ASP | A | 133 | 41.253 | 30.704 | 46.602 | 1.00 | 29.79 | A | C |
| ATOM | 692 | CB | ASP | A | 133 | 40.480 | 31.144 | 45.362 | 1.00 | 40.28 | A | C |
| ATOM | 693 | CG | ASP | A | 133 | 40.365 | 30.059 | 44.325 | 1.00 | 40.28 | A | C |
| ATOM | 694 | OD1 | ASP | A | 133 | 41.320 | 29.258 | 44.180 | 1.00 | 40.28 | A | O |
| ATOM | 695 | OD2 | ASP | A | 133 | 39.320 | 30.026 | 43.640 | 1.00 | 40.28 | A | O |
| ATOM | 696 | C | ASP | A | 133 | 41.383 | 31.912 | 47.508 | 1.00 | 29.79 | A | C |
| ATOM | 697 | O | ASP | A | 133 | 40.493 | 32.191 | 48.304 | 1.00 | 29.79 | A | O |
| ATOM | 698 | N | TYR | A | 134 | 42.489 | 32.630 | 47.397 | 1.00 | 31.83 | A | N |
| ATOM | 699 | CA | TYR | A | 134 | 42.655 | 33.816 | 48.214 | 1.00 | 31.83 | A | C |
| ATOM | 700 | CB | TYR | A | 134 | 44.103 | 33.964 | 48.670 | 1.00 | 33.17 | A | C |
| ATOM | 701 | CG | TYR | A | 134 | 44.403 | 35.350 | 49.185 | 1.00 | 33.17 | A | C |
| ATOM | 702 | CD1 | TYR | A | 134 | 43.999 | 35.750 | 50.460 | 1.00 | 33.17 | A | C |
| ATOM | 703 | CE1 | TYR | A | 134 | 44.175 | 37.066 | 50.888 | 1.00 | 33.17 | A | C |
| ATOM | 704 | CD2 | TYR | A | 134 | 45.001 | 36.299 | 48.356 | 1.00 | 33.17 | A | C |
| ATOM | 705 | CE2 | TYR | A | 134 | 45.178 | 37.613 | 48.773 | 1.00 | 33.17 | A | C |
| ATOM | 706 | CZ | TYR | A | 134 | 44.759 | 37.989 | 50.034 | 1.00 | 33.17 | A | C |
| ATOM | 707 | OH | TYR | A | 134 | 44.881 | 39.294 | 50.426 | 1.00 | 33.17 | A | O |
| ATOM | 708 | C | TYR | A | 134 | 42.237 | 35.068 | 47.431 | 1.00 | 31.83 | A | C |
| ATOM | 709 | O | TYR | A | 134 | 42.701 | 35.310 | 46.317 | 1.00 | 31.83 | A | O |
| ATOM | 710 | N | VAL | A | 135 | 41.337 | 35.848 | 48.013 | 1.00 | 29.74 | A | N |
| ATOM | 711 | CA | VAL | A | 135 | 40.890 | 37.076 | 47.390 | 1.00 | 29.74 | A | C |
| ATOM | 712 | CB | VAL | A | 135 | 39.369 | 37.127 | 47.259 | 1.00 | 19.46 | A | C |
| ATOM | 713 | CG1 | VAL | A | 135 | 38.963 | 38.374 | 46.500 | 1.00 | 19.46 | A | C |
| ATOM | 714 | CG2 | VAL | A | 135 | 38.875 | 35.889 | 46.539 | 1.00 | 19.46 | A | C |
| ATOM | 715 | C | VAL | A | 135 | 41.395 | 38.163 | 48.331 | 1.00 | 29.74 | A | C |
| ATOM | 716 | O | VAL | A | 135 | 41.230 | 38.075 | 49.545 | 1.00 | 29.74 | A | O |
| ATOM | 717 | N | PRO | A | 136 | 42.021 | 39.204 | 47.769 | 1.00 | 32.72 | A | N |
| ATOM | 718 | CD | PRO | A | 136 | 42.131 | 39.376 | 46.306 | 1.00 | 13.44 | A | C |
| ATOM | 719 | CA | PRO | A | 136 | 42.609 | 40.353 | 48.454 | 1.00 | 32.72 | A | C |
| ATOM | 720 | CB | PRO | A | 136 | 43.503 | 40.935 | 47.372 | 1.00 | 13.44 | A | C |
| ATOM | 721 | CG | PRO | A | 136 | 42.625 | 40.790 | 46.171 | 1.00 | 13.44 | A | C |
| ATOM | 722 | C | PRO | A | 136 | 41.726 | 41.434 | 49.079 | 1.00 | 32.72 | A | C |
| ATOM | 723 | O | PRO | A | 136 | 42.236 | 42.294 | 49.791 | 1.00 | 32.72 | A | O |
| ATOM | 724 | N | GLU | A | 137 | 40.426 | 41.424 | 48.844 | 1.00 | 18.95 | A | N |
| ATOM | 725 | CA | GLU | A | 137 | 39.648 | 42.507 | 49.404 | 1.00 | 18.95 | A | C |
| ATOM | 726 | CB | GLU | A | 137 | 39.672 | 43.671 | 48.406 | 1.00 | 34.97 | A | C |
| ATOM | 727 | CG | GLU | A | 137 | 39.314 | 45.020 | 48.986 | 1.00 | 34.97 | A | C |
| ATOM | 728 | CD | GLU | A | 137 | 40.360 | 45.528 | 49.949 | 1.00 | 34.97 | A | C |
| ATOM | 729 | OE1 | GLU | A | 137 | 41.460 | 45.916 | 49.504 | 1.00 | 34.97 | A | O |
| ATOM | 730 | OE2 | GLU | A | 137 | 40.077 | 45.527 | 51.163 | 1.00 | 34.97 | A | O |
| ATOM | 731 | C | GLU | A | 137 | 38.221 | 42.136 | 49.765 | 1.00 | 18.95 | A | C |
| ATOM | 732 | O | GLU | A | 137 | 37.668 | 41.187 | 49.225 | 1.00 | 18.95 | A | O |
| ATOM | 733 | N | THR | A | 138 | 37.626 | 42.896 | 50.680 | 1.00 | 16.14 | A | N |
| ATOM | 734 | CA | THR | A | 138 | 36.257 | 42.644 | 51.119 | 1.00 | 16.14 | A | C |
| ATOM | 735 | CB | THR | A | 138 | 36.224 | 42.199 | 52.586 | 1.00 | 2.67 | A | C |
| ATOM | 736 | OG1 | THR | A | 138 | 36.412 | 43.340 | 53.423 | 1.00 | 2.67 | A | O |
| ATOM | 737 | CG2 | THR | A | 138 | 37.309 | 41.176 | 52.866 | 1.00 | 2.67 | A | C |
| ATOM | 738 | C | THR | A | 138 | 35.377 | 43.891 | 50.988 | 1.00 | 16.14 | A | C |
| ATOM | 739 | O | THR | A | 138 | 35.837 | 45.003 | 51.199 | 1.00 | 16.14 | A | O |
| ATOM | 740 | N | VAL | A | 139 | 34.107 | 43.710 | 50.650 | 1.00 | 16.21 | A | N |
| ATOM | 741 | CA | VAL | A | 139 | 33.220 | 44.854 | 50.523 | 1.00 | 16.21 | A | C |
| ATOM | 742 | CB | VAL | A | 139 | 31.747 | 44.419 | 50.316 | 1.00 | 12.77 | A | C |
| ATOM | 743 | CG1 | VAL | A | 139 | 30.812 | 45.598 | 50.555 | 1.00 | 12.77 | A | C |
| ATOM | 744 | CG2 | VAL | A | 139 | 31.552 | 43.905 | 48.897 | 1.00 | 12.77 | A | C |
| ATOM | 745 | C | VAL | A | 139 | 33.312 | 45.744 | 51.757 | 1.00 | 16.21 | A | C |
| ATOM | 746 | O | VAL | A | 139 | 33.259 | 46.962 | 51.647 | 1.00 | 16.21 | A | O |
| ATOM | 747 | N | TYR | A | 140 | 33.444 | 45.130 | 52.928 | 1.00 | 25.49 | A | N |
| ATOM | 748 | CA | TYR | A | 140 | 33.549 | 45.865 | 54.179 | 1.00 | 25.49 | A | C |
| ATOM | 749 | CB | TYR | A | 140 | 33.780 | 44.891 | 55.330 | 1.00 | 32.02 | A | C |
| ATOM | 750 | CG | TYR | A | 140 | 33.996 | 45.568 | 56.663 | 1.00 | 32.02 | A | C |

| | | | | | | | | | | | | |
|---|---|---|---|---|---|---|---|---|---|---|---|---|
| ATOM | 751 | CD1 | TYR | A | 140 | 32.935 | 45.800 | 57.534 | 1.00 | 32.02 | A | C |
| ATOM | 752 | CE1 | TYR | A | 140 | 33.125 | 46.488 | 58.737 | 1.00 | 32.02 | A | C |
| ATOM | 753 | CD2 | TYR | A | 140 | 35.250 | 46.032 | 57.025 | 1.00 | 32.02 | A | C |
| ATOM | 754 | CE2 | TYR | A | 140 | 35.451 | 46.715 | 58.212 | 1.00 | 32.02 | A | C |
| ATOM | 755 | CZ | TYR | A | 140 | 34.390 | 46.944 | 59.060 | 1.00 | 32.02 | A | C |
| ATOM | 756 | OH | TYR | A | 140 | 34.612 | 47.658 | 60.211 | 1.00 | 32.02 | A | O |
| ATOM | 757 | C | TYR | A | 140 | 34.713 | 46.857 | 54.122 | 1.00 | 25.49 | A | C |
| ATOM | 758 | O | TYR | A | 140 | 34.533 | 48.070 | 54.336 | 1.00 | 25.49 | A | O |
| ATOM | 759 | N | ARG | A | 141 | 35.908 | 46.326 | 53.849 | 1.00 | 29.45 | A | N |
| ATOM | 760 | CA | ARG | A | 141 | 37.126 | 47.127 | 53.754 | 1.00 | 29.45 | A | C |
| ATOM | 761 | CB | ARG | A | 141 | 38.307 | 46.265 | 53.337 | 1.00 | 38.14 | A | C |
| ATOM | 762 | CG | ARG | A | 141 | 38.820 | 45.305 | 54.362 | 1.00 | 38.14 | A | C |
| ATOM | 763 | CD | ARG | A | 141 | 40.033 | 44.616 | 53.779 | 1.00 | 38.14 | A | C |
| ATOM | 764 | NE | ARG | A | 141 | 41.291 | 45.142 | 54.304 | 1.00 | 38.14 | A | N |
| ATOM | 765 | CZ | ARG | A | 141 | 42.396 | 45.326 | 53.580 | 1.00 | 38.14 | A | C |
| ATOM | 766 | NH1 | ARG | A | 141 | 42.415 | 45.042 | 52.276 | 1.00 | 38.14 | A | N |
| ATOM | 767 | NH2 | ARG | A | 141 | 43.502 | 45.772 | 54.169 | 1.00 | 38.14 | A | N |
| ATOM | 768 | C | ARG | A | 141 | 36.994 | 48.244 | 52.729 | 1.00 | 29.45 | A | C |
| ATOM | 769 | O | ARG | A | 141 | 37.533 | 49.331 | 52.903 | 1.00 | 29.45 | A | O |
| ATOM | 770 | N | VAL | A | 142 | 36.289 | 47.954 | 51.647 | 1.00 | 23.63 | A | N |
| ATOM | 771 | CA | VAL | A | 142 | 36.106 | 48.922 | 50.586 | 1.00 | 23.63 | A | C |
| ATOM | 772 | CB | VAL | A | 142 | 35.598 | 48.234 | 49.305 | 1.00 | 19.03 | A | C |
| ATOM | 773 | CG1 | VAL | A | 142 | 35.551 | 49.223 | 48.168 | 1.00 | 19.03 | A | C |
| ATOM | 774 | CG2 | VAL | A | 142 | 36.496 | 47.063 | 48.959 | 1.00 | 19.03 | A | C |
| ATOM | 775 | C | VAL | A | 142 | 35.137 | 50.006 | 51.006 | 1.00 | 23.63 | A | C |
| ATOM | 776 | O | VAL | A | 142 | 35.475 | 51.183 | 50.999 | 1.00 | 23.63 | A | O |
| ATOM | 777 | N | ALA | A | 143 | 33.931 | 49.605 | 51.377 | 1.00 | 30.71 | A | N |
| ATOM | 778 | CA | ALA | A | 143 | 32.912 | 50.550 | 51.796 | 1.00 | 30.71 | A | C |
| ATOM | 779 | CB | ALA | A | 143 | 31.719 | 49.803 | 52.355 | 1.00 | 6.50 | A | C |
| ATOM | 780 | C | ALA | A | 143 | 33.462 | 51.503 | 52.851 | 1.00 | 30.71 | A | C |
| ATOM | 781 | O | ALA | A | 143 | 33.176 | 52.701 | 52.843 | 1.00 | 30.71 | A | O |
| ATOM | 782 | N | ARG | A | 144 | 34.267 | 50.964 | 53.753 | 1.00 | 33.48 | A | N |
| ATOM | 783 | CA | ARG | A | 144 | 34.824 | 51.753 | 54.840 | 1.00 | 33.48 | A | C |
| ATOM | 784 | CB | ARG | A | 144 | 35.444 | 50.801 | 55.862 | 1.00 | 65.81 | A | C |
| ATOM | 785 | CG | ARG | A | 144 | 35.918 | 51.452 | 57.130 | 1.00 | 65.81 | A | C |
| ATOM | 786 | CD | ARG | A | 144 | 36.438 | 50.383 | 58.072 | 1.00 | 65.81 | A | C |
| ATOM | 787 | NE | ARG | A | 144 | 37.265 | 50.920 | 59.155 | 1.00 | 65.81 | A | N |
| ATOM | 788 | CZ | ARG | A | 144 | 36.793 | 51.607 | 60.193 | 1.00 | 65.81 | A | C |
| ATOM | 789 | NH1 | ARG | A | 144 | 35.487 | 51.845 | 60.291 | 1.00 | 65.81 | A | N |
| ATOM | 790 | NH2 | ARG | A | 144 | 37.624 | 52.054 | 61.135 | 1.00 | 65.81 | A | N |
| ATOM | 791 | C | ARG | A | 144 | 35.840 | 52.810 | 54.402 | 1.00 | 33.48 | A | C |
| ATOM | 792 | O | ARG | A | 144 | 35.918 | 53.891 | 54.990 | 1.00 | 33.48 | A | O |
| ATOM | 793 | N | HIS | A | 145 | 36.620 | 52.490 | 53.376 | 1.00 | 40.62 | A | N |
| ATOM | 794 | CA | HIS | A | 145 | 37.630 | 53.402 | 52.856 | 1.00 | 40.62 | A | C |
| ATOM | 795 | CB | HIS | A | 145 | 38.503 | 52.675 | 51.825 | 1.00 | 68.45 | A | C |
| ATOM | 796 | CG | HIS | A | 145 | 39.277 | 53.590 | 50.918 | 1.00 | 68.45 | A | C |
| ATOM | 797 | CD2 | HIS | A | 145 | 38.881 | 54.346 | 49.862 | 1.00 | 68.45 | A | C |
| ATOM | 798 | ND1 | HIS | A | 145 | 40.638 | 53.805 | 51.045 | 1.00 | 68.45 | A | N |
| ATOM | 799 | CE1 | HIS | A | 145 | 41.042 | 54.648 | 50.112 | 1.00 | 68.45 | A | C |
| ATOM | 800 | NE2 | HIS | A | 145 | 39.994 | 54.993 | 49.379 | 1.00 | 68.45 | A | N |
| ATOM | 801 | C | HIS | A | 145 | 36.950 | 54.606 | 52.220 | 1.00 | 40.62 | A | C |
| ATOM | 802 | O | HIS | A | 145 | 37.448 | 55.731 | 52.306 | 1.00 | 40.62 | A | O |
| ATOM | 803 | N | TYR | A | 146 | 35.814 | 54.357 | 51.572 | 1.00 | 34.93 | A | N |
| ATOM | 804 | CA | TYR | A | 146 | 35.056 | 55.415 | 50.923 | 1.00 | 34.93 | A | C |
| ATOM | 805 | CB | TYR | A | 146 | 34.095 | 54.835 | 49.889 | 1.00 | 27.21 | A | C |
| ATOM | 806 | CG | TYR | A | 146 | 34.703 | 54.588 | 48.526 | 1.00 | 27.21 | A | C |
| ATOM | 807 | CD1 | TYR | A | 146 | 35.127 | 53.317 | 48.147 | 1.00 | 27.21 | A | C |
| ATOM | 808 | CE1 | TYR | A | 146 | 35.657 | 53.080 | 46.893 | 1.00 | 27.21 | A | C |
| ATOM | 809 | CD2 | TYR | A | 146 | 34.835 | 55.622 | 47.606 | 1.00 | 27.21 | A | C |
| ATOM | 810 | CE2 | TYR | A | 146 | 35.371 | 55.389 | 46.339 | 1.00 | 27.21 | A | C |
| ATOM | 811 | CZ | TYR | A | 146 | 35.780 | 54.110 | 46.001 | 1.00 | 27.21 | A | C |
| ATOM | 812 | OH | TYR | A | 146 | 36.353 | 53.845 | 44.788 | 1.00 | 27.21 | A | O |
| ATOM | 813 | C | TYR | A | 146 | 34.256 | 56.203 | 51.944 | 1.00 | 34.93 | A | C |

| | | | | | | | | | | | | |
|---|---|---|---|---|---|---|---|---|---|---|---|---|
| ATOM | 814 | O | TYR | A | 146 | 33.966 | 57.380 | 51.748 | 1.00 | 34.93 | A | O |
| ATOM | 815 | N | SER | A | 147 | 33.903 | 55.555 | 53.045 | 1.00 | 36.80 | A | N |
| ATOM | 816 | CA | SER | A | 147 | 33.106 | 56.201 | 54.073 | 1.00 | 36.80 | A | C |
| ATOM | 817 | CB | SER | A | 147 | 32.450 | 55.145 | 54.955 | 1.00 | 36.25 | A | C |
| ATOM | 818 | OG | SER | A | 147 | 31.575 | 55.743 | 55.895 | 1.00 | 36.25 | A | O |
| ATOM | 819 | C | SER | A | 147 | 33.899 | 57.164 | 54.939 | 1.00 | 36.80 | A | C |
| ATOM | 820 | O | SER | A | 147 | 33.361 | 58.163 | 55.423 | 1.00 | 36.80 | A | O |
| ATOM | 821 | N | ARG | A | 148 | 35.175 | 56.862 | 55.149 | 1.00 | 47.53 | A | N |
| ATOM | 822 | CA | ARG | A | 148 | 36.006 | 57.724 | 55.969 | 1.00 | 47.53 | A | C |
| ATOM | 823 | CB | ARG | A | 148 | 37.235 | 56.967 | 56.490 | 1.00 | 71.97 | A | C |
| ATOM | 824 | CG | ARG | A | 148 | 36.913 | 55.768 | 57.363 | 1.00 | 71.97 | A | C |
| ATOM | 825 | CD | ARG | A | 148 | 36.722 | 56.126 | 58.835 | 1.00 | 71.97 | A | C |
| ATOM | 826 | NE | ARG | A | 148 | 37.523 | 55.225 | 59.677 | 1.00 | 71.97 | A | N |
| ATOM | 827 | CZ | ARG | A | 148 | 38.807 | 55.428 | 59.999 | 1.00 | 71.97 | A | C |
| ATOM | 828 | NH1 | ARG | A | 148 | 39.458 | 56.518 | 59.568 | 1.00 | 71.97 | A | N |
| ATOM | 829 | NH2 | ARG | A | 148 | 39.455 | 54.522 | 60.730 | 1.00 | 71.97 | A | N |
| ATOM | 830 | C | ARG | A | 148 | 36.443 | 58.904 | 55.125 | 1.00 | 47.53 | A | C |
| ATOM | 831 | O | ARG | A | 148 | 36.850 | 59.929 | 55.664 | 1.00 | 47.53 | A | O |
| ATOM | 832 | N | ALA | A | 149 | 36.361 | 58.762 | 53.807 | 1.00 | 45.63 | A | N |
| ATOM | 833 | CA | ALA | A | 149 | 36.756 | 59.851 | 52.920 | 1.00 | 45.63 | A | C |
| ATOM | 834 | CB | ALA | A | 149 | 37.397 | 59.302 | 51.652 | 1.00 | 43.12 | A | C |
| ATOM | 835 | C | ALA | A | 149 | 35.545 | 60.701 | 52.561 | 1.00 | 45.63 | A | C |
| ATOM | 836 | O | ALA | A | 149 | 35.607 | 61.539 | 51.652 | 1.00 | 45.63 | A | O |
| ATOM | 837 | N | LYS | A | 150 | 34.446 | 60.482 | 53.277 | 1.00 | 57.23 | A | N |
| ATOM | 838 | CA | LYS | A | 150 | 33.218 | 61.230 | 53.040 | 1.00 | 57.23 | A | C |
| ATOM | 839 | CB | LYS | A | 150 | 33.427 | 62.725 | 53.350 | 1.00 | 79.42 | A | C |
| ATOM | 840 | CG | LYS | A | 150 | 33.362 | 63.076 | 54.846 | 1.00 | 79.42 | A | C |
| ATOM | 841 | CD | LYS | A | 150 | 33.392 | 64.594 | 55.114 | 1.00 | 79.42 | A | C |
| ATOM | 842 | CE | LYS | A | 150 | 32.160 | 65.347 | 54.556 | 1.00 | 79.42 | A | C |
| ATOM | 843 | NZ | LYS | A | 150 | 32.105 | 65.438 | 53.056 | 1.00 | 79.42 | A | N |
| ATOM | 844 | C | LYS | A | 150 | 32.676 | 61.066 | 51.620 | 1.00 | 57.23 | A | C |
| ATOM | 845 | O | LYS | A | 150 | 31.889 | 61.901 | 51.150 | 1.00 | 57.23 | A | O |
| ATOM | 846 | N | GLN | A | 151 | 33.098 | 60.004 | 50.933 | 1.00 | 44.00 | A | N |
| ATOM | 847 | CA | GLN | A | 151 | 32.610 | 59.747 | 49.577 | 1.00 | 44.00 | A | C |
| ATOM | 848 | CB | GLN | A | 151 | 33.754 | 59.508 | 48.604 | 1.00 | 59.06 | A | C |
| ATOM | 849 | CG | GLN | A | 151 | 35.106 | 60.059 | 48.983 | 1.00 | 59.06 | A | C |
| ATOM | 850 | CD | GLN | A | 151 | 36.136 | 59.694 | 47.909 | 1.00 | 59.06 | A | C |
| ATOM | 851 | OE1 | GLN | A | 151 | 35.828 | 59.741 | 46.704 | 1.00 | 59.06 | A | O |
| ATOM | 852 | NE2 | GLN | A | 151 | 37.349 | 59.328 | 48.330 | 1.00 | 59.06 | A | N |
| ATOM | 853 | C | GLN | A | 151 | 31.746 | 58.490 | 49.598 | 1.00 | 44.00 | A | C |
| ATOM | 854 | O | GLN | A | 151 | 31.551 | 57.871 | 50.649 | 1.00 | 44.00 | A | O |
| ATOM | 855 | N | THR | A | 152 | 31.234 | 58.127 | 48.425 | 1.00 | 35.42 | A | N |
| ATOM | 856 | CA | THR | A | 152 | 30.413 | 56.938 | 48.267 | 1.00 | 35.42 | A | C |
| ATOM | 857 | CB | THR | A | 152 | 28.915 | 57.322 | 48.067 | 1.00 | 45.83 | A | C |
| ATOM | 858 | OG1 | THR | A | 152 | 28.158 | 56.860 | 49.198 | 1.00 | 45.83 | A | O |
| ATOM | 859 | CG2 | THR | A | 152 | 28.338 | 56.718 | 46.775 | 1.00 | 45.83 | A | C |
| ATOM | 860 | C | THR | A | 152 | 30.957 | 56.175 | 47.065 | 1.00 | 35.42 | A | C |
| ATOM | 861 | O | THR | A | 152 | 31.432 | 56.770 | 46.099 | 1.00 | 35.42 | A | O |
| ATOM | 862 | N | LEU | A | 153 | 30.919 | 54.856 | 47.149 | 1.00 | 23.86 | A | N |
| ATOM | 863 | CA | LEU | A | 153 | 31.380 | 54.003 | 46.067 | 1.00 | 23.86 | A | C |
| ATOM | 864 | CB | LEU | A | 153 | 31.215 | 52.545 | 46.498 | 1.00 | 41.54 | A | C |
| ATOM | 865 | CG | LEU | A | 153 | 31.488 | 51.457 | 45.460 | 1.00 | 41.54 | A | C |
| ATOM | 866 | CD1 | LEU | A | 153 | 32.988 | 51.344 | 45.214 | 1.00 | 41.54 | A | C |
| ATOM | 867 | CD2 | LEU | A | 153 | 30.925 | 50.139 | 45.962 | 1.00 | 41.54 | A | C |
| ATOM | 868 | C | LEU | A | 153 | 30.559 | 54.277 | 44.793 | 1.00 | 23.86 | A | C |
| ATOM | 869 | O | LEU | A | 153 | 29.333 | 54.356 | 44.853 | 1.00 | 23.86 | A | O |
| ATOM | 870 | N | PRO | A | 154 | 31.223 | 54.448 | 43.633 | 1.00 | 23.34 | A | N |
| ATOM | 871 | CD | PRO | A | 154 | 32.682 | 54.476 | 43.446 | 1.00 | 17.97 | A | C |
| ATOM | 872 | CA | PRO | A | 154 | 30.522 | 54.705 | 42.366 | 1.00 | 23.34 | A | C |
| ATOM | 873 | CB | PRO | A | 154 | 31.629 | 54.600 | 41.328 | 1.00 | 17.97 | A | C |
| ATOM | 874 | CG | PRO | A | 154 | 32.822 | 55.039 | 42.056 | 1.00 | 17.97 | A | C |
| ATOM | 875 | C | PRO | A | 154 | 29.461 | 53.631 | 42.137 | 1.00 | 23.34 | A | C |
| ATOM | 876 | O | PRO | A | 154 | 29.712 | 52.445 | 42.361 | 1.00 | 23.34 | A | O |

| | | | | | | | | | | | | |
|---|---|---|---|---|---|---|---|---|---|---|---|---|
| ATOM | 877 | N | VAL | A | 155 | 28.287 | 54.032 | 41.664 | 1.00 | 33.96 | A | N |
| ATOM | 878 | CA | VAL | A | 155 | 27.212 | 53.072 | 41.458 | 1.00 | 33.96 | A | C |
| ATOM | 879 | CB | VAL | A | 155 | 25.917 | 53.791 | 41.063 | 1.00 | 27.12 | A | C |
| ATOM | 880 | CG1 | VAL | A | 155 | 25.611 | 54.854 | 42.097 | 1.00 | 27.12 | A | C |
| ATOM | 881 | CG2 | VAL | A | 155 | 26.041 | 54.395 | 39.675 | 1.00 | 27.12 | A | C |
| ATOM | 882 | C | VAL | A | 155 | 27.525 | 51.947 | 40.467 | 1.00 | 33.96 | A | C |
| ATOM | 883 | O | VAL | A | 155 | 26.992 | 50.841 | 40.596 | 1.00 | 33.96 | A | O |
| ATOM | 884 | N | ILE | A | 156 | 28.380 | 52.209 | 39.486 | 1.00 | 23.34 | A | N |
| ATOM | 885 | CA | ILE | A | 156 | 28.701 | 51.165 | 38.535 | 1.00 | 23.34 | A | C |
| ATOM | 886 | CB | ILE | A | 156 | 29.771 | 51.634 | 37.527 | 1.00 | 16.35 | A | C |
| ATOM | 887 | CG2 | ILE | A | 156 | 30.983 | 52.163 | 38.266 | 1.00 | 16.35 | A | C |
| ATOM | 888 | CG1 | ILE | A | 156 | 30.166 | 50.479 | 36.603 | 1.00 | 16.35 | A | C |
| ATOM | 889 | CD1 | ILE | A | 156 | 29.030 | 49.947 | 35.760 | 1.00 | 16.35 | A | C |
| ATOM | 890 | C | ILE | A | 156 | 29.206 | 49.963 | 39.328 | 1.00 | 23.34 | A | C |
| ATOM | 891 | O | ILE | A | 156 | 28.882 | 48.820 | 39.010 | 1.00 | 23.34 | A | O |
| ATOM | 892 | N | TYR | A | 157 | 29.983 | 50.236 | 40.373 | 1.00 | 21.81 | A | N |
| ATOM | 893 | CA | TYR | A | 157 | 30.528 | 49.192 | 41.246 | 1.00 | 21.81 | A | C |
| ATOM | 894 | CB | TYR | A | 157 | 31.638 | 49.766 | 42.140 | 1.00 | 43.54 | A | C |
| ATOM | 895 | CG | TYR | A | 157 | 32.934 | 49.949 | 41.406 | 1.00 | 43.54 | A | C |
| ATOM | 896 | CD1 | TYR | A | 157 | 33.640 | 51.148 | 41.471 | 1.00 | 43.54 | A | C |
| ATOM | 897 | CE1 | TYR | A | 157 | 34.805 | 51.328 | 40.732 | 1.00 | 43.54 | A | C |
| ATOM | 898 | CD2 | TYR | A | 157 | 33.427 | 48.931 | 40.591 | 1.00 | 43.54 | A | C |
| ATOM | 899 | CE2 | TYR | A | 157 | 34.585 | 49.098 | 39.852 | 1.00 | 43.54 | A | C |
| ATOM | 900 | CZ | TYR | A | 157 | 35.265 | 50.297 | 39.923 | 1.00 | 43.54 | A | C |
| ATOM | 901 | OH | TYR | A | 157 | 36.393 | 50.467 | 39.163 | 1.00 | 43.54 | A | O |
| ATOM | 902 | C | TYR | A | 157 | 29.449 | 48.585 | 42.131 | 1.00 | 21.81 | A | C |
| ATOM | 903 | O | TYR | A | 157 | 29.436 | 47.384 | 42.374 | 1.00 | 21.81 | A | O |
| ATOM | 904 | N | VAL | A | 158 | 28.555 | 49.434 | 42.626 | 1.00 | 24.10 | A | N |
| ATOM | 905 | CA | VAL | A | 158 | 27.464 | 48.989 | 43.479 | 1.00 | 24.10 | A | C |
| ATOM | 906 | CB | VAL | A | 158 | 26.672 | 50.209 | 44.034 | 1.00 | 17.15 | A | C |
| ATOM | 907 | CG1 | VAL | A | 158 | 25.524 | 49.742 | 44.902 | 1.00 | 17.15 | A | C |
| ATOM | 908 | CG2 | VAL | A | 158 | 27.593 | 51.100 | 44.839 | 1.00 | 17.15 | A | C |
| ATOM | 909 | C | VAL | A | 158 | 26.543 | 48.067 | 42.678 | 1.00 | 24.10 | A | C |
| ATOM | 910 | O | VAL | A | 158 | 25.893 | 47.185 | 43.236 | 1.00 | 24.10 | A | O |
| ATOM | 911 | N | LYS | A | 159 | 26.500 | 48.279 | 41.368 | 1.00 | 20.02 | A | N |
| ATOM | 912 | CA | LYS | A | 159 | 25.692 | 47.464 | 40.485 | 1.00 | 20.02 | A | C |
| ATOM | 913 | CB | LYS | A | 159 | 25.603 | 48.128 | 39.111 | 1.00 | 28.50 | A | C |
| ATOM | 914 | CG | LYS | A | 159 | 24.290 | 48.852 | 38.871 | 1.00 | 28.50 | A | C |
| ATOM | 915 | CD | LYS | A | 159 | 24.323 | 49.734 | 37.630 | 1.00 | 28.50 | A | C |
| ATOM | 916 | CE | LYS | A | 159 | 23.817 | 51.133 | 37.965 | 1.00 | 28.50 | A | C |
| ATOM | 917 | NZ | LYS | A | 159 | 23.130 | 51.843 | 36.836 | 1.00 | 28.50 | A | N |
| ATOM | 918 | C | LYS | A | 159 | 26.362 | 46.106 | 40.364 | 1.00 | 20.02 | A | C |
| ATOM | 919 | O | LYS | A | 159 | 25.771 | 45.076 | 40.694 | 1.00 | 20.02 | A | O |
| ATOM | 920 | N | LEU | A | 160 | 27.608 | 46.126 | 39.897 | 1.00 | 17.34 | A | N |
| ATOM | 921 | CA | LEU | A | 160 | 28.405 | 44.924 | 39.705 | 1.00 | 17.34 | A | C |
| ATOM | 922 | CB | LEU | A | 160 | 29.820 | 45.305 | 39.298 | 1.00 | 29.38 | A | C |
| ATOM | 923 | CG | LEU | A | 160 | 29.995 | 45.598 | 37.811 | 1.00 | 29.38 | A | C |
| ATOM | 924 | CD1 | LEU | A | 160 | 31.334 | 46.265 | 37.570 | 1.00 | 29.38 | A | C |
| ATOM | 925 | CD2 | LEU | A | 160 | 29.879 | 44.292 | 37.032 | 1.00 | 29.38 | A | C |
| ATOM | 926 | C | LEU | A | 160 | 28.469 | 43.998 | 40.901 | 1.00 | 17.34 | A | C |
| ATOM | 927 | O | LEU | A | 160 | 28.170 | 42.814 | 40.793 | 1.00 | 17.34 | A | O |
| ATOM | 928 | N | TYR | A | 161 | 28.875 | 44.542 | 42.041 | 1.00 | 23.02 | A | N |
| ATOM | 929 | CA | TYR | A | 161 | 28.985 | 43.755 | 43.251 | 1.00 | 23.02 | A | C |
| ATOM | 930 | CB | TYR | A | 161 | 29.591 | 44.595 | 44.387 | 1.00 | 20.95 | A | C |
| ATOM | 931 | CG | TYR | A | 161 | 30.978 | 45.164 | 44.108 | 1.00 | 20.95 | A | C |
| ATOM | 932 | CD1 | TYR | A | 161 | 31.877 | 44.500 | 43.268 | 1.00 | 20.95 | A | C |
| ATOM | 933 | CE1 | TYR | A | 161 | 33.158 | 45.004 | 43.047 | 1.00 | 20.95 | A | C |
| ATOM | 934 | CD2 | TYR | A | 161 | 31.404 | 46.352 | 44.719 | 1.00 | 20.95 | A | C |
| ATOM | 935 | CE2 | TYR | A | 161 | 32.678 | 46.860 | 44.500 | 1.00 | 20.95 | A | C |
| ATOM | 936 | CZ | TYR | A | 161 | 33.551 | 46.186 | 43.669 | 1.00 | 20.95 | A | C |
| ATOM | 937 | OH | TYR | A | 161 | 34.813 | 46.704 | 43.467 | 1.00 | 20.95 | A | O |
| ATOM | 938 | C | TYR | A | 161 | 27.630 | 43.195 | 43.672 | 1.00 | 23.02 | A | C |
| ATOM | 939 | O | TYR | A | 161 | 27.478 | 41.984 | 43.855 | 1.00 | 23.02 | A | O |

| | | | | | | | | | | | | |
|---|---|---|---|---|---|---|---|---|---|---|---|---|
| ATOM | 940 | N | MET | A | 162 | 26.639 | 44.069 | 43.800 | 1.00 | 28.41 | A | N |
| ATOM | 941 | CA | MET | A | 162 | 25.324 | 43.628 | 44.220 | 1.00 | 28.41 | A | C |
| ATOM | 942 | CB | MET | A | 162 | 24.419 | 44.839 | 44.479 | 1.00 | 26.10 | A | C |
| ATOM | 943 | CG | MET | A | 162 | 24.757 | 45.581 | 45.782 | 1.00 | 26.10 | A | C |
| ATOM | 944 | SD | MET | A | 162 | 25.020 | 44.426 | 47.159 | 1.00 | 26.10 | A | S |
| ATOM | 945 | CE | MET | A | 162 | 23.384 | 43.953 | 47.447 | 1.00 | 26.10 | A | C |
| ATOM | 946 | C | MET | A | 162 | 24.657 | 42.641 | 43.286 | 1.00 | 28.41 | A | C |
| ATOM | 947 | O | MET | A | 162 | 23.814 | 41.861 | 43.720 | 1.00 | 28.41 | A | O |
| ATOM | 948 | N | TYR | A | 163 | 25.034 | 42.662 | 42.011 | 1.00 | 21.23 | A | N |
| ATOM | 949 | CA | TYR | A | 163 | 24.460 | 41.748 | 41.027 | 1.00 | 21.23 | A | C |
| ATOM | 950 | CB | TYR | A | 163 | 24.752 | 42.260 | 39.619 | 1.00 | 27.81 | A | C |
| ATOM | 951 | CG | TYR | A | 163 | 24.279 | 41.346 | 38.505 | 1.00 | 27.81 | A | C |
| ATOM | 952 | CD1 | TYR | A | 163 | 22.979 | 41.429 | 38.010 | 1.00 | 27.81 | A | C |
| ATOM | 953 | CE1 | TYR | A | 163 | 22.545 | 40.591 | 36.997 | 1.00 | 27.81 | A | C |
| ATOM | 954 | CD2 | TYR | A | 163 | 25.136 | 40.395 | 37.955 | 1.00 | 27.81 | A | C |
| ATOM | 955 | CE2 | TYR | A | 163 | 24.714 | 39.551 | 36.946 | 1.00 | 27.81 | A | C |
| ATOM | 956 | CZ | TYR | A | 163 | 23.417 | 39.651 | 36.460 | 1.00 | 27.81 | A | C |
| ATOM | 957 | OH | TYR | A | 163 | 23.004 | 38.837 | 35.411 | 1.00 | 27.81 | A | O |
| ATOM | 958 | C | TYR | A | 163 | 25.104 | 40.393 | 41.231 | 1.00 | 21.23 | A | C |
| ATOM | 959 | O | TYR | A | 163 | 24.426 | 39.379 | 41.329 | 1.00 | 21.23 | A | O |
| ATOM | 960 | N | GLN | A | 164 | 26.428 | 40.385 | 41.299 | 1.00 | 20.80 | A | N |
| ATOM | 961 | CA | GLN | A | 164 | 27.169 | 39.155 | 41.510 | 1.00 | 20.80 | A | C |
| ATOM | 962 | CB | GLN | A | 164 | 28.665 | 39.441 | 41.482 | 1.00 | 16.33 | A | C |
| ATOM | 963 | CG | GLN | A | 164 | 29.158 | 40.082 | 40.210 | 1.00 | 16.33 | A | C |
| ATOM | 964 | CD | GLN | A | 164 | 30.667 | 40.176 | 40.175 | 1.00 | 16.33 | A | C |
| ATOM | 965 | OE1 | GLN | A | 164 | 31.353 | 39.163 | 40.123 | 1.00 | 16.33 | A | O |
| ATOM | 966 | NE2 | GLN | A | 164 | 31.193 | 41.395 | 40.217 | 1.00 | 16.33 | A | N |
| ATOM | 967 | C | GLN | A | 164 | 26.807 | 38.487 | 42.830 | 1.00 | 20.80 | A | C |
| ATOM | 968 | O | GLN | A | 164 | 26.989 | 37.287 | 42.981 | 1.00 | 20.80 | A | O |
| ATOM | 969 | N | LEU | A | 165 | 26.313 | 39.260 | 43.791 | 1.00 | 22.27 | A | N |
| ATOM | 970 | CA | LEU | A | 165 | 25.929 | 38.695 | 45.082 | 1.00 | 22.27 | A | C |
| ATOM | 971 | CB | LEU | A | 165 | 25.776 | 39.796 | 46.137 | 1.00 | 17.10 | A | C |
| ATOM | 972 | CG | LEU | A | 165 | 25.078 | 39.400 | 47.442 | 1.00 | 17.10 | A | C |
| ATOM | 973 | CD1 | LEU | A | 165 | 25.988 | 38.593 | 48.334 | 1.00 | 17.10 | A | C |
| ATOM | 974 | CD2 | LEU | A | 165 | 24.654 | 40.643 | 48.158 | 1.00 | 17.10 | A | C |
| ATOM | 975 | C | LEU | A | 165 | 24.615 | 37.951 | 44.940 | 1.00 | 22.27 | A | C |
| ATOM | 976 | O | LEU | A | 165 | 24.399 | 36.923 | 45.585 | 1.00 | 22.27 | A | O |
| ATOM | 977 | N | PHE | A | 166 | 23.733 | 38.472 | 44.095 | 1.00 | 19.30 | A | N |
| ATOM | 978 | CA | PHE | A | 166 | 22.461 | 37.817 | 43.909 | 1.00 | 19.30 | A | C |
| ATOM | 979 | CB | PHE | A | 166 | 21.448 | 38.787 | 43.298 | 1.00 | 15.89 | A | C |
| ATOM | 980 | CG | PHE | A | 166 | 20.812 | 39.694 | 44.319 | 1.00 | 15.89 | A | C |
| ATOM | 981 | CD1 | PHE | A | 166 | 20.210 | 39.159 | 45.457 | 1.00 | 15.89 | A | C |
| ATOM | 982 | CD2 | PHE | A | 166 | 20.815 | 41.071 | 44.159 | 1.00 | 15.89 | A | C |
| ATOM | 983 | CE1 | PHE | A | 166 | 19.626 | 39.985 | 46.421 | 1.00 | 15.89 | A | C |
| ATOM | 984 | CE2 | PHE | A | 166 | 20.235 | 41.903 | 45.119 | 1.00 | 15,89 | A | C |
| ATOM | 985 | CZ | PHE | A | 166 | 19.637 | 41.357 | 46.248 | 1.00 | 15.89 | A | C |
| ATOM | 986 | C | PHE | A | 166 | 22.623 | 36.538 | 43.105 | 1.00 | 19.30 | A | C |
| ATOM | 987 | O | PHE | A | 166 | 21.931 | 35.559 | 43.356 | 1.00 | 19.30 | A | O |
| ATOM | 988 | N | ARG | A | 167 | 23.564 | 36.522 | 42.172 | 1.00 | 23.59 | A | N |
| ATOM | 989 | CA | ARG | A | 167 | 23.796 | 35.320 | 41.393 | 1.00 | 23.59 | A | C |
| ATOM | 990 | CB | ARG | A | 167 | 24.835 | 35.558 | 40.308 | 1.00 | 25.39 | A | C |
| ATOM | 991 | CG | ARG | A | 167 | 24.246 | 35.956 | 38.969 | 1.00 | 25.39 | A | C |
| ATOM | 992 | CD | ARG | A | 167 | 25.275 | 35.828 | 37.849 | 1.00 | 25.39 | A | C |
| ATOM | 993 | NE | ARG | A | 167 | 25.537 | 34.438 | 37.475 | 1.00 | 25.39 | A | N |
| ATOM | 994 | CZ | ARG | A | 167 | 26.552 | 34.053 | 36.712 | 1.00 | 25.39 | A | C |
| ATOM | 995 | NH1 | ARG | A | 167 | 27.411 | 34.933 | 36.246 | 1.00 | 25.39 | A | N |
| ATOM | 996 | NH2 | ARG | A | 167 | 26.691 | 32.789 | 36.385 | 1.00 | 25.39 | A | N |
| ATOM | 997 | C | ARG | A | 167 | 24.272 | 34.186 | 42.279 | 1.00 | 23.59 | A | C |
| ATOM | 998 | O | ARG | A | 167 | 23.942 | 33.027 | 42.031 | 1.00 | 23.59 | A | O |
| ATOM | 999 | N | SER | A | 168 | 25.058 | 34.515 | 43.303 | 1.00 | 20.74 | A | N |
| ATOM | 1000 | CA | SER | A | 168 | 25.558 | 33.496 | 44.220 | 1.00 | 20.74 | A | C |
| ATOM | 1001 | CB | SER | A | 168 | 26.718 | 34.038 | 45.069 | 1.00 | 19.61 | A | C |
| ATOM | 1002 | OG | SER | A | 168 | 26.284 | 35.023 | 45.985 | 1.00 | 19.61 | A | O |

| | | | | | | | | | | | | |
|---|---|---|---|---|---|---|---|---|---|---|---|---|
| ATOM | 1003 | C | SER | A | 168 | 24.415 | 33.038 | 45.110 | 1.00 | 20.74 | A | C |
| ATOM | 1004 | O | SER | A | 168 | 24.326 | 31.874 | 45.470 | 1.00 | 20.74 | A | O |
| ATOM | 1005 | N | LEU | A | 169 | 23.532 | 33.967 | 45.436 | 1.00 | 18.88 | A | N |
| ATOM | 1006 | CA | LEU | A | 169 | 22.381 | 33.680 | 46.266 | 1.00 | 18.88 | A | C |
| ATOM | 1007 | CB | LEU | A | 169 | 21.723 | 34.992 | 46.677 | 1.00 | 22.01 | A | C |
| ATOM | 1008 | CG | LEU | A | 169 | 21.769 | 35.369 | 48.155 | 1.00 | 22.01 | A | C |
| ATOM | 1009 | CD1 | LEU | A | 169 | 23.097 | 35.013 | 48.780 | 1.00 | 22.01 | A | C |
| ATOM | 1010 | CD2 | LEU | A | 169 | 21.501 | 36.840 | 48.273 | 1.00 | 22.01 | A | C |
| ATOM | 1011 | C | LEU | A | 169 | 21.388 | 32.813 | 45.502 | 1.00 | 18.88 | A | C |
| ATOM | 1012 | O | LEU | A | 169 | 20.853 | 31.846 | 46.042 | 1.00 | 18.88 | A | O |
| ATOM | 1013 | N | ALA | A | 170 | 21.150 | 33.156 | 44.242 | 1.00 | 16.34 | A | N |
| ATOM | 1014 | CA | ALA | A | 170 | 20.218 | 32.394 | 43.428 | 1.00 | 16.34 | A | C |
| ATOM | 1015 | CB | ALA | A | 170 | 20.000 | 33.077 | 42.092 | 1.00 | 10.39 | A | C |
| ATOM | 1016 | C | ALA | A | 170 | 20.770 | 31.001 | 43.217 | 1.00 | 16.34 | A | C |
| ATOM | 1017 | O | ALA | A | 170 | 20.022 | 30.045 | 43.020 | 1.00 | 16.34 | A | O |
| ATOM | 1018 | N | TYR | A | 171 | 22.092 | 30.888 | 43.271 | 1.00 | 32.57 | A | N |
| ATOM | 1019 | CA | TYR | A | 171 | 22.745 | 29.602 | 43.074 | 1.00 | 32.57 | A | C |
| ATOM | 1020 | CB | TYR | A | 171 | 24.242 | 29.777 | 42.829 | 1.00 | 25.76 | A | C |
| ATOM | 1021 | CG | TYR | A | 171 | 24.996 | 28.462 | 42.692 | 1.00 | 25.76 | A | C |
| ATOM | 1022 | CD1 | TYR | A | 171 | 24.742 | 27.592 | 41.631 | 1.00 | 25.76 | A | C |
| ATOM | 1023 | CE1 | TYR | A | 171 | 25.446 | 26.380 | 41.498 | 1.00 | 25.76 | A | C |
| ATOM | 1024 | CD2 | TYR | A | 171 | 25.973 | 28.092 | 43.619 | 1.00 | 25.76 | A | C |
| ATOM | 1025 | CE2 | TYR | A | 171 | 26.677 | 26.891 | 43.493 | 1.00 | 25.76 | A | C |
| ATOM | 1026 | CZ | TYR | A | 171 | 26.413 | 26.039 | 42.431 | 1.00 | 25.76 | A | C |
| ATOM | 1027 | OH | TYR | A | 171 | 27.130 | 24.864 | 42.307 | 1.00 | 25.76 | A | O |
| ATOM | 1028 | C | TYR | A | 171 | 22.554 | 28.688 | 44.259 | 1.00 | 32.57 | A | C |
| ATOM | 1029 | O | TYR | A | 171 | 21.932 | 27.637 | 44.138 | 1.00 | 32.57 | A | O |
| ATOM | 1030 | N | ILE | A | 172 | 23.093 | 29.078 | 45.407 | 1.00 | 26.47 | A | N |
| ATOM | 1031 | CA | ILE | A | 172 | 22.970 | 28.243 | 46.588 | 1.00 | 26.47 | A | C |
| ATOM | 1032 | CB | ILE | A | 172 | 23.764 | 28.819 | 47.775 | 1.00 | 20.39 | A | C |
| ATOM | 1033 | CG2 | ILE | A | 172 | 25.241 | 28.816 | 47.450 | 1.00 | 20.39 | A | C |
| ATOM | 1034 | CG1 | ILE | A | 172 | 23.270 | 30.225 | 48.110 | 1.00 | 20.39 | A | C |
| ATOM | 1035 | CD1 | ILE | A | 172 | 23.849 | 30.784 | 49.393 | 1.00 | 20.39 | A | C |
| ATOM | 1036 | C | ILE | A | 172 | 21.521 | 28.010 | 47.010 | 1.00 | 26.47 | A | C |
| ATOM | 1037 | O | ILE | A | 172 | 21.194 | 26.965 | 47.556 | 1.00 | 26.47 | A | O |
| ATOM | 1038 | N | HIS | A | 173 | 20.645 | 28.972 | 46.757 | 1.00 | 20.06 | A | N |
| ATOM | 1039 | CA | HIS | A | 173 | 19.261 | 28.773 | 47.137 | 1.00 | 20.06 | A | C |
| ATOM | 1040 | CB | HIS | A | 173 | 18.459 | 30.074 | 47.005 | 1.00 | 19.53 | A | C |
| ATOM | 1041 | CG | HIS | A | 173 | 18.766 | 31.081 | 48.075 | 1.00 | 19.53 | A | C |
| ATOM | 1042 | CD2 | HIS | A | 173 | 19.557 | 31.003 | 49.174 | 1.00 | 19.53 | A | C |
| ATOM | 1043 | ND1 | HIS | A | 173 | 18.254 | 32.361 | 48.066 | 1.00 | 19.53 | A | N |
| ATOM | 1044 | CE1 | HIS | A | 173 | 18.720 | 33.026 | 49.107 | 1.00 | 19.53 | A | C |
| ATOM | 1045 | NE2 | HIS | A | 173 | 19.513 | 32.226 | 49.794 | 1.00 | 19.53 | A | N |
| ATOM | 1046 | C | HIS | A | 173 | 18.656 | 27.666 | 46.294 | 1.00 | 20.06 | A | C |
| ATOM | 1047 | O | HIS | A | 173 | 17.895 | 26.862 | 46.808 | 1.00 | 20.06 | A | O |
| ATOM | 1048 | N | SER | A | 174 | 19.004 | 27.596 | 45.012 | 1.00 | 29.63 | A | N |
| ATOM | 1049 | CA | SER | A | 174 | 18.450 | 26.548 | 44.151 | 1.00 | 29.63 | A | C |
| ATOM | 1050 | CB | SER | A | 174 | 19.023 | 26.639 | 42.727 | 1.00 | 28.82 | A | C |
| ATOM | 1051 | OG | SER | A | 174 | 20.343 | 26.131 | 42.654 | 1.00 | 28.82 | A | O |
| ATOM | 1052 | C | SER | A | 174 | 18.743 | 25.164 | 44.741 | 1.00 | 29.63 | A | C |
| ATOM | 1053 | O | SER | A | 174 | 18.114 | 24.178 | 44.380 | 1.00 | 29.63 | A | O |
| ATOM | 1054 | N | PHE | A | 175 | 19.700 | 25.101 | 45.652 | 1.00 | 23.50 | A | N |
| ATOM | 1055 | CA | PHE | A | 175 | 20.042 | 23.850 | 46.302 | 1.00 | 23.50 | A | C |
| ATOM | 1056 | CB | PHE | A | 175 | 21.554 | 23.781 | 46.534 | 1.00 | 45.44 | A | C |
| ATOM | 1057 | CG | PHE | A | 175 | 22.338 | 23.338 | 45.338 | 1.00 | 45.44 | A | C |
| ATOM | 1058 | CD1 | PHE | A | 175 | 22.577 | 21.986 | 45.111 | 1.00 | 45.44 | A | C |
| ATOM | 1059 | CD2 | PHE | A | 175 | 22.843 | 24.269 | 44.441 | 1.00 | 45.44 | A | C |
| ATOM | 1060 | CE1 | PHE | A | 175 | 23.307 | 21.566 | 44.009 | 1.00 | 45.44 | A | C |
| ATOM | 1061 | CE2 | PHE | A | 175 | 23.580 | 23.863 | 43.326 | 1.00 | 45.44 | A | C |
| ATOM | 1062 | CZ | PHE | A | 175 | 23.813 | 22.508 | 43.111 | 1.00 | 45.44 | A | C |
| ATOM | 1063 | C | PHE | A | 175 | 19.348 | 23.780 | 47.653 | 1.00 | 23.50 | A | C |
| ATOM | 1064 | O | PHE | A | 175 | 19.482 | 22.790 | 48.363 | 1.00 | 23.50 | A | O |
| ATOM | 1065 | N | GLY | A | 176 | 18.624 | 24.836 | 48.011 | 1.00 | 24.00 | A | N |

| | | | | | | | | | | | | |
|---|---|---|---|---|---|---|---|---|---|---|---|---|
| ATOM | 1066 | CA | GLY | A | 176 | 17.949 | 24.874 | 49.297 | 1.00 | 24.00 | A | C |
| ATOM | 1067 | C | GLY | A | 176 | 18.932 | 25.133 | 50.424 | 1.00 | 24.00 | A | C |
| ATOM | 1068 | O | GLY | A | 176 | 18.737 | 24.665 | 51.538 | 1.00 | 24.00 | A | O |
| ATOM | 1069 | N | ILE | A | 177 | 19.996 | 25.870 | 50.125 | 1.00 | 27.54 | A | N |
| ATOM | 1070 | CA | ILE | A | 177 | 21.023 | 26.209 | 51.102 | 1.00 | 27.54 | A | C |
| ATOM | 1071 | CB | ILE | A | 177 | 22.427 | 25.917 | 50.561 | 1.00 | 38.36 | A | C |
| ATOM | 1072 | CG2 | ILE | A | 177 | 23.464 | 26.225 | 51.620 | 1.00 | 38.36 | A | C |
| ATOM | 1073 | CG1 | ILE | A | 177 | 22.524 | 24.461 | 50.117 | 1.00 | 38.36 | A | C |
| ATOM | 1074 | CD1 | ILE | A | 177 | 23.858 | 24.118 | 49.444 | 1.00 | 38.36 | A | C |
| ATOM | 1075 | C | ILE | A | 177 | 20.942 | 27.701 | 51.391 | 1.00 | 27.54 | A | C |
| ATOM | 1076 | O | ILE | A | 177 | 20.911 | 28.518 | 50.471 | 1.00 | 27.54 | A | O |
| ATOM | 1077 | N | CYS | A | 178 | 20.919 | 28.041 | 52.674 | 1.00 | 24.00 | A | N |
| ATOM | 1078 | CA | CYS | A | 178 | 20.819 | 29.419 | 53.114 | 1.00 | 24.00 | A | C |
| ATOM | 1079 | CB | CYS | A | 178 | 19.703 | 29.536 | 54.140 | 1.00 | 21.11 | A | C |
| ATOM | 1080 | SG | CYS | A | 178 | 19.050 | 31.174 | 54.358 | 1.00 | 21.11 | A | S |
| ATOM | 1081 | C | CYS | A | 178 | 22.137 | 29.798 | 53.747 | 1.00 | 24.00 | A | C |
| ATOM | 1082 | O | CYS | A | 178 | 22.710 | 29.011 | 54.492 | 1.00 | 24.00 | A | O |
| ATOM | 1083 | N | HIS | A | 179 | 22.624 | 30.999 | 53.450 | 1.00 | 20.94 | A | N |
| ATOM | 1084 | CA | HIS | A | 179 | 23.885 | 31.462 | 54.014 | 1.00 | 20.94 | A | C |
| ATOM | 1085 | CB | HIS | A | 179 | 24.395 | 32.667 | 53.235 | 1.00 | 15.09 | A | C |
| ATOM | 1086 | CG | HIS | A | 179 | 25.828 | 32.989 | 53.505 | 1.00 | 15.09 | A | C |
| ATOM | 1087 | CD2 | HIS | A | 179 | 26.948 | 32.701 | 52.805 | 1.00 | 15.09 | A | C |
| ATOM | 1088 | ND1 | HIS | A | 179 | 26.244 | 33.660 | 54.636 | 1.00 | 15.09 | A | N |
| ATOM | 1089 | CE1 | HIS | A | 179 | 27.558 | 33.770 | 54.621 | 1.00 | 15.09 | A | C |
| ATOM | 1090 | NE2 | HIS | A | 179 | 28.011 | 33.195 | 53.522 | 1.00 | 15.09 | A | N |
| ATOM | 1091 | C | HIS | A | 179 | 23.701 | 31.824 | 55.487 | 1.00 | 20.94 | A | C |
| ATOM | 1092 | O | HIS | A | 179 | 24.487 | 31.408 | 56.334 | 1.00 | 20.94 | A | O |
| ATOM | 1093 | N | ARG | A | 180 | 22.653 | 32.596 | 55.771 | 1.00 | 32.96 | A | N |
| ATOM | 1094 | CA | ARG | A | 180 | 22.314 | 33.019 | 57.126 | 1.00 | 32.96 | A | C |
| ATOM | 1095 | CB | ARG | A | 180 | 22.311 | 31.822 | 58.058 | 1.00 | 22.71 | A | C |
| ATOM | 1096 | CG | ARG | A | 180 | 21.227 | 30.816 | 57.779 | 1.00 | 22.71 | A | C |
| ATOM | 1097 | CD | ARG | A | 180 | 21.630 | 29.546 | 58.434 | 1.00 | 22.71 | A | C |
| ATOM | 1098 | NE | ARG | A | 180 | 20.586 | 28.978 | 59.253 | 1.00 | 22.71 | A | N |
| ATOM | 1099 | CZ | ARG | A | 180 | 20.816 | 28.059 | 60.176 | 1.00 | 22.71 | A | C |
| ATOM | 1100 | NH1 | ARG | A | 180 | 22.053 | 27.628 | 60.383 | 1.00 | 22.71 | A | N |
| ATOM | 1101 | NH2 | ARG | A | 180 | 19.813 | 27.565 | 60.884 | 1.00 | 22.71 | A | N |
| ATOM | 1102 | C | ARG | A | 180 | 23.222 | 34.095 | 57.722 | 1.00 | 32.96 | A | C |
| ATOM | 1103 | O | ARG | A | 180 | 22.979 | 34.585 | 58.830 | 1.00 | 32.96 | A | O |
| ATOM | 1104 | N | ASP | A | 181 | 24.266 | 34.471 | 56.992 | 1.00 | 20.89 | A | N |
| ATOM | 1105 | CA | ASP | A | 181 | 25.176 | 35.476 | 57.502 | 1.00 | 20.89 | A | C |
| ATOM | 1106 | CB | ASP | A | 181 | 26.249 | 34.796 | 58.358 | 1.00 | 33.02 | A | C |
| ATOM | 1107 | CG | ASP | A | 181 | 27.059 | 35.780 | 59.181 | 1.00 | 33.02 | A | C |
| ATOM | 1108 | OD1 | ASP | A | 181 | 26.455 | 36.709 | 59.768 | 1.00 | 33.02 | A | O |
| ATOM | 1109 | OD2 | ASP | A | 181 | 28.299 | 35.615 | 59.250 | 1.00 | 33.02 | A | O |
| ATOM | 1110 | C | ASP | A | 181 | 25.805 | 36.344 | 56.416 | 1.00 | 20.89 | A | C |
| ATOM | 1111 | O | ASP | A | 181 | 27.025 | 36.537 | 56.362 | 1.00 | 20.89 | A | O |
| ATOM | 1112 | N | ILE | A | 182 | 24.951 | 36.866 | 55.549 | 1.00 | 13.90 | A | N |
| ATOM | 1113 | CA | ILE | A | 182 | 25.400 | 37.744 | 54.494 | 1.00 | 13.90 | A | C |
| ATOM | 1114 | CB | ILE | A | 182 | 24.316 | 37.916 | 53.408 | 1.00 | 7.58 | A | C |
| ATOM | 1115 | CG2 | ILE | A | 182 | 24.764 | 38.929 | 52.380 | 1.00 | 7.58 | A | C |
| ATOM | 1116 | CG1 | ILE | A | 182 | 23.976 | 36.563 | 52.777 | 1.00 | 7.58 | A | C |
| ATOM | 1117 | CD1 | ILE | A | 182 | 25.068 | 35.971 | 51.953 | 1.00 | 7.58 | A | C |
| ATOM | 1118 | C | ILE | A | 182 | 25.676 | 39.098 | 55.148 | 1.00 | 13.90 | A | C |
| ATOM | 1119 | O | ILE | A | 182 | 24.805 | 39.702 | 55.777 | 1.00 | 13.90 | A | O |
| ATOM | 1120 | N | LYS | A | 183 | 26.914 | 39.550 | 55.022 | 1.00 | 21.14 | A | N |
| ATOM | 1121 | CA | LYS | A | 183 | 27.339 | 40.840 | 55.559 | 1.00 | 21.14 | A | C |
| ATOM | 1122 | CB | LYS | A | 183 | 27.748 | 40.744 | 57.019 | 1.00 | 14.96 | A | C |
| ATOM | 1123 | CG | LYS | A | 183 | 28.857 | 39.744 | 57.323 | 1.00 | 14.96 | A | C |
| ATOM | 1124 | CD | LYS | A | 183 | 28.950 | 39.510 | 58.818 | 1.00 | 14.96 | A | C |
| ATOM | 1125 | CE | LYS | A | 183 | 29.991 | 38.469 | 59.154 | 1.00 | 14.96 | A | C |
| ATOM | 1126 | NZ | LYS | A | 183 | 30.032 | 38.184 | 60.605 | 1.00 | 14.96 | A | N |
| ATOM | 1127 | C | LYS | A | 183 | 28.523 | 41.253 | 54.711 | 1.00 | 21.14 | A | C |
| ATOM | 1128 | O | LYS | A | 183 | 29.214 | 40.402 | 54.153 | 1.00 | 21.14 | A | O |

| | | | | | | | | | | | | |
|---|---|---|---|---|---|---|---|---|---|---|---|---|
| ATOM | 1129 | N | PRO | A | 184 | 28.758 | 42.569 | 54.565 | 1.00 | 23.23 | A | N |
| ATOM | 1130 | CD | PRO | A | 184 | 27.869 | 43.679 | 54.932 | 1.00 | 14.33 | A | C |
| ATOM | 1131 | CA | PRO | A | 184 | 29.891 | 43.059 | 53.780 | 1.00 | 23.23 | A | C |
| ATOM | 1132 | CB | PRO | A | 184 | 29.954 | 44.505 | 54.191 | 1.00 | 14.33 | A | C |
| ATOM | 1133 | CG | PRO | A | 184 | 28.522 | 44.841 | 54.267 | 1.00 | 14.33 | A | C |
| ATOM | 1134 | C | PRO | A | 184 | 31.227 | 42.349 | 54.020 | 1.00 | 23.23 | A | C |
| ATOM | 1135 | O | PRO | A | 184 | 32.044 | 42.204 | 53.109 | 1.00 | 23.23 | A | O |
| ATOM | 1136 | N | GLN | A | 185 | 31.475 | 41.922 | 55.245 | 1.00 | 23.24 | A | N |
| ATOM | 1137 | CA | GLN | A | 185 | 32.728 | 41.250 | 55.570 | 1.00 | 23.24 | A | C |
| ATOM | 1138 | CB | GLN | A | 185 | 32.844 | 41.133 | 57.080 | 1.00 | 56.98 | A | C |
| ATOM | 1139 | CG | GLN | A | 185 | 34.263 | 41.061 | 57.554 | 1.00 | 56.98 | A | C |
| ATOM | 1140 | CD | GLN | A | 185 | 34.430 | 40.091 | 58.713 | 1.00 | 56.98 | A | C |
| ATOM | 1141 | OE1 | GLN | A | 185 | 35.486 | 39.472 | 58.862 | 1.00 | 56.98 | A | O |
| ATOM | 1142 | NE2 | GLN | A | 185 | 33.392 | 39.972 | 59.563 | 1.00 | 56.98 | A | N |
| ATOM | 1143 | C | GLN | A | 185 | 32.829 | 39.867 | 54.927 | 1.00 | 23.24 | A | C |
| ATOM | 1144 | O | GLN | A | 185 | 33.923 | 39.340 | 54.764 | 1.00 | 23.24 | A | O |
| ATOM | 1145 | N | ASN | A | 186 | 31.690 | 39.290 | 54.570 | 1.00 | 23.76 | A | N |
| ATOM | 1146 | CA | ASN | A | 186 | 31.648 | 37.976 | 53.939 | 1.00 | 23.76 | A | C |
| ATOM | 1147 | CB | ASN | A | 186 | 30.401 | 37.215 | 54.354 | 1.00 | 27.48 | A | C |
| ATOM | 1148 | CG | ASN | A | 186 | 30.589 | 36.452 | 55.625 | 1.00 | 27.48 | A | C |
| ATOM | 1149 | OD1 | ASN | A | 186 | 31.710 | 36.348 | 56.130 | 1.00 | 27.48 | A | O |
| ATOM | 1150 | ND2 | ASN | A | 186 | 29.508 | 35.915 | 56.166 | 1.00 | 27.48 | A | N |
| ATOM | 1151 | C | ASN | A | 186 | 31.674 | 38.089 | 52.441 | 1.00 | 23.76 | A | C |
| ATOM | 1152 | O | ASN | A | 186 | 31.599 | 37.080 | 51.755 | 1.00 | 23.76 | A | O |
| ATOM | 1153 | N | LEU | A | 187 | 31.755 | 39.317 | 51.938 | 1.00 | 17.74 | A | N |
| ATOM | 1154 | CA | LEU | A | 187 | 31.781 | 39.557 | 50.503 | 1.00 | 17.74 | A | C |
| ATOM | 1155 | CB | LEU | A | 187 | 30.792 | 40.652 | 50.144 | 1.00 | 18.83 | A | C |
| ATOM | 1156 | CG | LEU | A | 187 | 29.359 | 40.389 | 50.586 | 1.00 | 18.83 | A | C |
| ATOM | 1157 | CD1 | LEU | A | 187 | 28.471 | 41.526 | 50.111 | 1.00 | 18.83 | A | C |
| ATOM | 1158 | CD2 | LEU | A | 187 | 28.869 | 39.070 | 50.023 | 1.00 | 18.83 | A | C |
| ATOM | 1159 | C | LEU | A | 187 | 33.180 | 39.942 | 50.028 | 1.00 | 17.74 | A | C |
| ATOM | 1160 | O | LEU | A | 187 | 33.594 | 41.088 | 50.145 | 1.00 | 17.74 | A | O |
| ATOM | 1161 | N | LEU | A | 188 | 33.900 | 38.959 | 49.496 | 1.00 | 27.59 | A | N |
| ATOM | 1162 | CA | LEU | A | 188 | 35.253 | 39.150 | 48.973 | 1.00 | 27.59 | A | C |
| ATOM | 1163 | CB | LEU | A | 188 | 35.998 | 37.813 | 48.906 | 1.00 | 36.69 | A | C |
| ATOM | 1164 | CG | LEU | A | 188 | 36.072 | 36.885 | 50.115 | 1.00 | 36.69 | A | C |
| ATOM | 1165 | CD1 | LEU | A | 188 | 36.622 | 35.549 | 49.681 | 1.00 | 36.69 | A | C |
| ATOM | 1166 | CD2 | LEU | A | 188 | 36.943 | 37.502 | 51.189 | 1.00 | 36.69 | A | C |
| ATOM | 1167 | C | LEU | A | 188 | 35.159 | 39.697 | 47.557 | 1.00 | 27.59 | A | C |
| ATOM | 1168 | O | LEU | A | 188 | 34.180 | 39.442 | 46.852 | 1.00 | 27.59 | A | O |
| ATOM | 1169 | N | LEU | A | 189 | 36.169 | 40.444 | 47.131 | 1.00 | 24.81 | A | N |
| ATOM | 1170 | CA | LEU | A | 189 | 36.161 | 40.972 | 45.776 | 1.00 | 24.81 | A | C |
| ATOM | 1171 | CB | LEU | A | 189 | 35.237 | 42.204 | 45.673 | 1.00 | 41.46 | A | C |
| ATOM | 1172 | CG | LEU | A | 189 | 35.596 | 43.572 | 46.264 | 1.00 | 41.46 | A | C |
| ATOM | 1173 | CD1 | LEU | A | 189 | 34.317 | 44.330 | 46.594 | 1.00 | 41.46 | A | C |
| ATOM | 1174 | CD2 | LEU | A | 189 | 36.401 | 43.406 | 47.516 | 1.00 | 41.46 | A | C |
| ATOM | 1175 | C | LEU | A | 189 | 37.566 | 41.291 | 45.294 | 1.00 | 24.81 | A | C |
| ATOM | 1176 | O | LEU | A | 189 | 38.493 | 41.437 | 46.089 | 1.00 | 24.81 | A | O |
| ATOM | 1177 | N | ASP | A | 190 | 37.724 | 41.339 | 43.977 | 1.00 | 36.67 | A | N |
| ATOM | 1178 | CA | ASP | A | 190 | 39.004 | 41.658 | 43.370 | 1.00 | 36.67 | A | C |
| ATOM | 1179 | CB | ASP | A | 190 | 39.333 | 40.670 | 42.252 | 1.00 | 40.59 | A | C |
| ATOM | 1180 | CG | ASP | A | 190 | 40.750 | 40.831 | 41.737 | 1.00 | 40.59 | A | C |
| ATOM | 1181 | OD1 | ASP | A | 190 | 41.678 | 40.682 | 42.553 | 1.00 | 40.59 | A | O |
| ATOM | 1182 | OD2 | ASP | A | 190 | 40.944 | 41.107 | 40.530 | 1.00 | 40.59 | A | O |
| ATOM | 1183 | C | ASP | A | 190 | 38.814 | 43.060 | 42.806 | 1.00 | 36.67 | A | C |
| ATOM | 1184 | O | ASP | A | 190 | 37.932 | 43.293 | 41.975 | 1.00 | 36.67 | A | O |
| ATOM | 1185 | N | PRO | A | 191 | 39.635 | 44.015 | 43.253 | 1.00 | 42.36 | A | N |
| ATOM | 1186 | CD | PRO | A | 191 | 40.759 | 43.837 | 44.182 | 1.00 | 47.79 | A | C |
| ATOM | 1187 | CA | PRO | A | 191 | 39.551 | 45.409 | 42.797 | 1.00 | 42.36 | A | C |
| ATOM | 1188 | CB | PRO | A | 191 | 40.668 | 46.098 | 43.574 | 1.00 | 47.79 | A | C |
| ATOM | 1189 | CG | PRO | A | 191 | 40.858 | 45.204 | 44.785 | 1.00 | 47.79 | A | C |
| ATOM | 1190 | C | PRO | A | 191 | 39.732 | 45.588 | 41.291 | 1.00 | 42.36 | A | C |
| ATOM | 1191 | O | PRO | A | 191 | 39.049 | 46.396 | 40.659 | 1.00 | 42.36 | A | O |

| | | | | | | | | | | | | |
|---|---|---|---|---|---|---|---|---|---|---|---|---|
| ATOM | 1192 | N | ASP | A | 192 | 40.653 | 44.835 | 40.711 | 1.00 | 49.12 | A | N |
| ATOM | 1193 | CA | ASP | A | 192 | 40.881 | 44.984 | 39.293 | 1.00 | 49.12 | A | C |
| ATOM | 1194 | CB | ASP | A | 192 | 42.246 | 44.425 | 38.916 | 1.00 | 49.22 | A | C |
| ATOM | 1195 | CG | ASP | A | 192 | 43.359 | 45.087 | 39.693 | 1.00 | 49.22 | A | C |
| ATOM | 1196 | OD1 | ASP | A | 192 | 43.228 | 46.307 | 39.986 | 1.00 | 49.22 | A | O |
| ATOM | 1197 | OD2 | ASP | A | 192 | 44.352 | 44.389 | 40.006 | 1.00 | 49.22 | A | O |
| ATOM | 1198 | C | ASP | A | 192 | 39.788 | 44.360 | 38.435 | 1.00 | 49.12 | A | C |
| ATOM | 1199 | O | ASP | A | 192 | 39.170 | 45.051 | 37.610 | 1.00 | 49.12 | A | O |
| ATOM | 1200 | N | THR | A | 193 | 39.530 | 43.068 | 38.619 | 1.00 | 29.20 | A | N |
| ATOM | 1201 | CA | THR | A | 193 | 38.503 | 42.430 | 37.814 | 1.00 | 29.20 | A | C |
| ATOM | 1202 | CB | THR | A | 193 | 38.691 | 40.895 | 37.752 | 1.00 | 26.36 | A | C |
| ATOM | 1203 | OG1 | THR | A | 193 | 38.782 | 40.354 | 39.071 | 1.00 | 26.36 | A | O |
| ATOM | 1204 | CG2 | THR | A | 193 | 39.950 | 40.557 | 36.996 | 1.00 | 26.36 | A | C |
| ATOM | 1205 | C | THR | A | 193 | 37.074 | 42.751 | 38.249 | 1.00 | 29.20 | A | C |
| ATOM | 1206 | O | THR | A | 193 | 36.133 | 42.476 | 37.513 | 1.00 | 29.20 | A | O |
| ATOM | 1207 | N | ALA | A | 194 | 36.918 | 43.347 | 39.430 | 1.00 | 22.39 | A | N |
| ATOM | 1208 | CA | ALA | A | 194 | 35.605 | 43.705 | 39.965 | 1.00 | 22.39 | A | C |
| ATOM | 1209 | CB | ALA | A | 194 | 34.881 | 44.641 | 39.010 | 1.00 | 8.33 | A | C |
| ATOM | 1210 | C | ALA | A | 194 | 34.741 | 42.487 | 40.249 | 1.00 | 22.39 | A | C |
| ATOM | 1211 | O | ALA | A | 194 | 33.524 | 42.601 | 40.382 | 1.00 | 22.39 | A | O |
| ATOM | 1212 | N | VAL | A | 195 | 35.370 | 41.318 | 40.341 | 1.00 | 30.34 | A | N |
| ATOM | 1213 | CA | VAL | A | 195 | 34.638 | 40.085 | 40.613 | 1.00 | 30.34 | A | C |
| ATOM | 1214 | CB | VAL | A | 195 | 35.361 | 38.864 | 39.995 | 1.00 | 40.89 | A | C |
| ATOM | 1215 | CG1 | VAL | A | 195 | 36.832 | 38.976 | 40.229 | 1.00 | 40.89 | A | C |
| ATOM | 1216 | CG2 | VAL | A | 195 | 34.829 | 37.569 | 40.602 | 1.00 | 40.89 | A | C |
| ATOM | 1217 | C | VAL | A | 195 | 34.397 | 39.859 | 42.111 | 1.00 | 30.34 | A | C |
| ATOM | 1218 | O | VAL | A | 195 | 35.323 | 39.910 | 42.930 | 1.00 | 30.34 | A | O |
| ATOM | 1219 | N | LEU | A | 196 | 33.133 | 39.633 | 42.458 | 1.00 | 34.55 | A | N |
| ATOM | 1220 | CA | LEU | A | 196 | 32.742 | 39.406 | 43.838 | 1.00 | 34.55 | A | C |
| ATOM | 1221 | CB | LEU | A | 196 | 31.475 | 40.200 | 44.162 | 1.00 | 15.74 | A | C |
| ATOM | 1222 | CG | LEU | A | 196 | 31.062 | 40.239 | 45.635 | 1.00 | 15.74 | A | C |
| ATOM | 1223 | CD1 | LEU | A | 196 | 30.369 | 41.552 | 45.917 | 1.00 | 15.74 | A | C |
| ATOM | 1224 | CD2 | LEU | A | 196 | 30.166 | 39.080 | 45.971 | 1.00 | 15.74 | A | C |
| ATOM | 1225 | C | LEU | A | 196 | 32.526 | 37.924 | 44.107 | 1.00 | 34.55 | A | C |
| ATOM | 1226 | O | LEU | A | 196 | 31.972 | 37.203 | 43.280 | 1.00 | 34.55 | A | O |
| ATOM | 1227 | N | LYS | A | 197 | 32.979 | 37.474 | 45.271 | 1.00 | 22.73 | A | N |
| ATOM | 1228 | CA | LYS | A | 197 | 32.852 | 36.079 | 45.652 | 1.00 | 22.73 | A | C |
| ATOM | 1229 | CB | LYS | A | 197 | 34.203 | 35.377 | 45.506 | 1.00 | 26.65 | A | C |
| ATOM | 1230 | CG | LYS | A | 197 | 34.627 | 35.271 | 44.054 | 1.00 | 26.65 | A | C |
| ATOM | 1231 | CD | LYS | A | 197 | 35.867 | 34.442 | 43.823 | 1.00 | 26.65 | A | C |
| ATOM | 1232 | CE | LYS | A | 197 | 36.092 | 34.336 | 42.322 | 1.00 | 26.65 | A | C |
| ATOM | 1233 | NZ | LYS | A | 197 | 37.036 | 33.258 | 41.912 | 1.00 | 26.65 | A | N |
| ATOM | 1234 | C | LYS | A | 197 | 32.354 | 35.992 | 47.078 | 1.00 | 22.73 | A | C |
| ATOM | 1235 | O | LYS | A | 197 | 32.863 | 36.674 | 47.962 | 1.00 | 22.73 | A | O |
| ATOM | 1236 | N | LEU | A | 198 | 31.342 | 35.161 | 47.290 | 1.00 | 27.40 | A | N |
| ATOM | 1237 | CA | LEU | A | 198 | 30.756 | 34.967 | 48.611 | 1.00 | 27.40 | A | C |
| ATOM | 1238 | CB | LEU | A | 198 | 29.355 | 34.375 | 48.481 | 1.00 | 24.98 | A | C |
| ATOM | 1239 | CG | LEU | A | 198 | 28.303 | 34.786 | 49.503 | 1.00 | 24.98 | A | C |
| ATOM | 1240 | CD1 | LEU | A | 198 | 27.194 | 33.749 | 49.531 | 1.00 | 24.98 | A | C |
| ATOM | 1241 | CD2 | LEU | A | 198 | 28.931 | 34.908 | 50.855 | 1.00 | 24.98 | A | C |
| ATOM | 1242 | C | LEU | A | 198 | 31.639 | 33.969 | 49.353 | 1.00 | 27.40 | A | C |
| ATOM | 1243 | O | LEU | A | 198 | 32.227 | 33.092 | 48.731 | 1.00 | 27.40 | A | O |
| ATOM | 1244 | N | CYS | A | 199 | 31.740 | 34.095 | 50.673 | 1.00 | 15.48 | A | N |
| ATOM | 1245 | CA | CYS | A | 199 | 32.548 | 33.164 | 51.435 | 1.00 | 15.48 | A | C |
| ATOM | 1246 | CB | CYS | A | 199 | 34.001 | 33.648 | 51.526 | 1.00 | 17.26 | A | C |
| ATOM | 1247 | SG | CYS | A | 199 | 34.334 | 35.049 | 52.579 | 1.00 | 17.26 | A | S |
| ATOM | 1248 | C | CYS | A | 199 | 31.978 | 32.965 | 52.821 | 1.00 | 15.48 | A | C |
| ATOM | 1249 | O | CYS | A | 199 | 30.895 | 33.456 | 53.123 | 1.00 | 15.48 | A | O |
| ATOM | 1250 | N | ASP | A | 200 | 32.704 | 32.211 | 53.646 | 1.00 | 24.37 | A | N |
| ATOM | 1251 | CA | ASP | A | 200 | 32.325 | 31.936 | 55.032 | 1.00 | 24.37 | A | C |
| ATOM | 1252 | CB | ASP | A | 200 | 32.358 | 33.244 | 55.829 | 1.00 | 47.06 | A | C |
| ATOM | 1253 | CG | ASP | A | 200 | 32.402 | 33.030 | 57.342 | 1.00 | 47.06 | A | C |
| ATOM | 1254 | OD1 | ASP | A | 200 | 32.343 | 31.857 | 57.808 | 1.00 | 47.06 | A | O |

| | | | | | | | | | | | | |
|---|---|---|---|---|---|---|---|---|---|---|---|---|
| ATOM | 1255 | OD2 | ASP | A | 200 | 32.499 | 34.061 | 58.064 | 1.00 | 47.06 | A | O |
| ATOM | 1256 | C | ASP | A | 200 | 30.953 | 31.283 | 55.168 | 1.00 | 24.37 | A | C |
| ATOM | 1257 | O | ASP | A | 200 | 29.989 | 31.927 | 55.567 | 1.00 | 24.37 | A | O |
| ATOM | 1258 | N | PHE | A | 201 | 30.871 | 29.999 | 54.843 | 1.00 | 30.20 | A | N |
| ATOM | 1259 | CA | PHE | A | 201 | 29.610 | 29.278 | 54.947 | 1.00 | 30.20 | A | C |
| ATOM | 1260 | CB | PHE | A | 201 | 29.460 | 28.296 | 53.788 | 1.00 | 25.93 | A | C |
| ATOM | 1261 | CG | PHE | A | 201 | 29.331 | 28.961 | 52.458 | 1.00 | 25.93 | A | C |
| ATOM | 1262 | CD1 | PHE | A | 201 | 30.401 | 29.659 | 51.915 | 1.00 | 25.93 | A | C |
| ATOM | 1263 | CD2 | PHE | A | 201 | 28.127 | 28.933 | 51.764 | 1.00 | 25.93 | A | C |
| ATOM | 1264 | CE1 | PHE | A | 201 | 30.279 | 30.322 | 50.697 | 1.00 | 25.93 | A | C |
| ATOM | 1265 | CE2 | PHE | A | 201 | 27.991 | 29.591 | 50.550 | 1.00 | 25.93 | A | C |
| ATOM | 1266 | CZ | PHE | A | 201 | 29.073 | 30.291 | 50.017 | 1.00 | 25.93 | A | C |
| ATOM | 1267 | C | PHE | A | 201 | 29.533 | 28.541 | 56.272 | 1.00 | 30.20 | A | C |
| ATOM | 1268 | O | PHE | A | 201 | 28.889 | 27.496 | 56.383 | 1.00 | 30.20 | A | O |
| ATOM | 1269 | N | GLY | A | 202 | 30.200 | 29.100 | 57.274 | 1.00 | 23.48 | A | N |
| ATOM | 1270 | CA | GLY | A | 202 | 30.191 | 28.501 | 58.593 | 1.00 | 23.48 | A | C |
| ATOM | 1271 | C | GLY | A | 202 | 28.851 | 28.628 | 59.295 | 1.00 | 23.48 | A | C |
| ATOM | 1272 | O | GLY | A | 202 | 28.700 | 28.158 | 60.405 | 1.00 | 23.48 | A | O |
| ATOM | 1273 | N | SER | A | 203 | 27.875 | 29.264 | 58.666 | 1.00 | 24.05 | A | N |
| ATOM | 1274 | CA | SER | A | 203 | 26.564 | 29.400 | 59.283 | 1.00 | 24.05 | A | C |
| ATOM | 1275 | CB | SER | A | 203 | 26.270 | 30.860 | 59.634 | 1.00 | 26.29 | A | C |
| ATOM | 1276 | OG | SER | A | 203 | 27.124 | 31.317 | 60.662 | 1.00 | 26.29 | A | O |
| ATOM | 1277 | C | SER | A | 203 | 25.495 | 28.884 | 58.336 | 1.00 | 24.05 | A | C |
| ATOM | 1278 | O | SER | A | 203 | 24.313 | 28.867 | 58.666 | 1.00 | 24.05 | A | O |
| ATOM | 1279 | N | ALA | A | 204 | 25.916 | 28.453 | 57.156 | 1.00 | 25.04 | A | N |
| ATOM | 1280 | CA | ALA | A | 204 | 24.982 | 27.948 | 56.172 | 1.00 | 25.04 | A | C |
| ATOM | 1281 | CB | ALA | A | 204 | 25.671 | 27.819 | 54.831 | 1.00 | 29.87 | A | C |
| ATOM | 1282 | C | ALA | A | 204 | 24.378 | 26.614 | 56.563 | 1.00 | 25.04 | A | C |
| ATOM | 1283 | O | ALA | A | 204 | 25.001 | 25.799 | 57.236 | 1.00 | 25.04 | A | O |
| ATOM | 1284 | N | LYS | A | 205 | 23.151 | 26.400 | 56.114 | 1.00 | 29.49 | A | N |
| ATOM | 1285 | CA | LYS | A | 205 | 22.431 | 25.168 | 56.376 | 1.00 | 29.49 | A | C |
| ATOM | 1286 | CB | LYS | A | 205 | 21.632 | 25.272 | 57.674 | 1.00 | 46.29 | A | C |
| ATOM | 1287 | CG | LYS | A | 205 | 20.968 | 23.954 | 58.047 | 1.00 | 46.29 | A | C |
| ATOM | 1288 | CD | LYS | A | 205 | 19.819 | 24.087 | 59.031 | 1.00 | 46.29 | A | C |
| ATOM | 1289 | CE | LYS | A | 205 | 19.373 | 22.698 | 59.463 | 1.00 | 46.29 | A | C |
| ATOM | 1290 | NZ | LYS | A | 205 | 18.148 | 22.694 | 60.317 | 1.00 | 46.29 | A | N |
| ATOM | 1291 | C | LYS | A | 205 | 21.457 | 24.886 | 55.224 | 1.00 | 29.49 | A | C |
| ATOM | 1292 | O | LYS | A | 205 | 20.948 | 25.808 | 54.575 | 1.00 | 29.49 | A | O |
| ATOM | 1293 | N | GLN | A | 206 | 21.199 | 23.604 | 54.989 | 1.00 | 41.39 | A | N |
| ATOM | 1294 | CA | GLN | A | 206 | 20.278 | 23.162 | 53.952 | 1.00 | 41.39 | A | C |
| ATOM | 1295 | CB | GLN | A | 206 | 20.605 | 21.708 | 53.602 | 1.00 | 38.22 | A | C |
| ATOM | 1296 | CG | GLN | A | 206 | 20.617 | 21.356 | 52.112 | 1.00 | 38.22 | A | C |
| ATOM | 1297 | CD | GLN | A | 206 | 19.249 | 20.939 | 51.608 | 1.00 | 38.22 | A | C |
| ATOM | 1298 | OE1 | GLN | A | 206 | 18.524 | 20.212 | 52.285 | 1.00 | 38.22 | A | O |
| ATOM | 1299 | NE2 | GLN | A | 206 | 18.893 | 21.385 | 50.414 | 1.00 | 38.22 | A | N |
| ATOM | 1300 | C | GLN | A | 206 | 18.874 | 23.273 | 54.548 | 1.00 | 41.39 | A | C |
| ATOM | 1301 | O | GLN | A | 206 | 18.418 | 22.351 | 55.201 | 1.00 | 41.39 | A | O |
| ATOM | 1302 | N | LEU | A | 207 | 18.187 | 24.389 | 54.332 | 1.00 | 33.88 | A | N |
| ATOM | 1303 | CA | LEU | A | 207 | 16.857 | 24.585 | 54.907 | 1.00 | 33.88 | A | C |
| ATOM | 1304 | CB | LEU | A | 207 | 16.427 | 26.042 | 54.772 | 1.00 | 20.86 | A | C |
| ATOM | 1305 | CG | LEU | A | 207 | 17.289 | 27.097 | 55.467 | 1.00 | 20.86 | A | C |
| ATOM | 1306 | CD1 | LEU | A | 207 | 16.509 | 28.407 | 55.451 | 1.00 | 20.86 | A | C |
| ATOM | 1307 | CD2 | LEU | A | 207 | 17.637 | 26.692 | 56.907 | 1.00 | 20.86 | A | C |
| ATOM | 1308 | C | LEU | A | 207 | 15.721 | 23.711 | 54.396 | 1.00 | 33.88 | A | C |
| ATOM | 1309 | O | LEU | A | 207 | 15.133 | 23.982 | 53.357 | 1.00 | 33.88 | A | O |
| ATOM | 1310 | N | VAL | A | 208 | 15.389 | 22.676 | 55.150 | 1.00 | 36.07 | A | N |
| ATOM | 1311 | CA | VAL | A | 208 | 14.303 | 21.788 | 54.780 | 1.00 | 36.07 | A | C |
| ATOM | 1312 | CB | VAL | A | 208 | 14.454 | 20.412 | 55.429 | 1.00 | 30.05 | A | C |
| ATOM | 1313 | CG1 | VAL | A | 208 | 13.247 | 19.563 | 55.102 | 1.00 | 30.05 | A | C |
| ATOM | 1314 | CG2 | VAL | A | 208 | 15.750 | 19.755 | 54.969 | 1.00 | 30.05 | A | C |
| ATOM | 1315 | C | VAL | A | 208 | 13.004 | 22.382 | 55.288 | 1.00 | 36.07 | A | C |
| ATOM | 1316 | O | VAL | A | 208 | 12.967 | 22.955 | 56.371 | 1.00 | 36.07 | A | O |
| ATOM | 1317 | N | ARG | A | 209 | 11.937 | 22.233 | 54.514 | 1.00 | 48.38 | A | N |

| | | | | | | | | | | | | |
|---|---|---|---|---|---|---|---|---|---|---|---|---|
| ATOM | 1318 | CA | ARG | A | 209 | 10.627 | 22.751 | 54.900 | 1.00 | 48.38 | A | C |
| ATOM | 1319 | CB | ARG | A | 209 | 9.655 | 22.709 | 53.717 | 1.00 | 87.17 | A | C |
| ATOM | 1320 | CG | ARG | A | 209 | 8.221 | 23.002 | 54.102 | 1.00 | 87.17 | A | C |
| ATOM | 1321 | CD | ARG | A | 209 | 7.720 | 24.281 | 53.449 | 1.00 | 87.17 | A | C |
| ATOM | 1322 | NE | ARG | A | 209 | 6.878 | 24.020 | 52.276 | 1.00 | 87.17 | A | N |
| ATOM | 1323 | CZ | ARG | A | 209 | 7.288 | 23.426 | 51.151 | 1.00 | 87.17 | A | C |
| ATOM | 1324 | NH1 | ARG | A | 209 | 8.552 | 23.013 | 51.021 | 1.00 | 87.17 | A | N |
| ATOM | 1325 | NH2 | ARG | A | 209 | 6.428 | 23.251 | 50.147 | 1.00 | 87.17 | A | N |
| ATOM | 1326 | C | ARG | A | 209 | 10.056 | 21.920 | 56.033 | 1.00 | 48.38 | A | C |
| ATOM | 1327 | O | ARG | A | 209 | 10.146 | 20.687 | 56.020 | 1.00 | 48.38 | A | O |
| ATOM | 1328 | N | GLY | A | 210 | 9.460 | 22.608 | 57.004 | 1.00 | 42.37 | A | N |
| ATOM | 1329 | CA | GLY | A | 210 | 8.869 | 21.937 | 58.146 | 1.00 | 42.37 | A | C |
| ATOM | 1330 | C | GLY | A | 210 | 9.875 | 21.787 | 59.260 | 1.00 | 42.37 | A | C |
| ATOM | 1331 | O | GLY | A | 210 | 9.513 | 21.738 | 60.431 | 1.00 | 42.37 | A | O |
| ATOM | 1332 | N | GLU | A | 211 | 11.149 | 21.716 | 58.902 | 1.00 | 51.86 | A | N |
| ATOM | 1333 | CA | GLU | A | 211 | 12.191 | 21.575 | 59.912 | 1.00 | 51.86 | A | C |
| ATOM | 1334 | CB | GLU | A | 211 | 13.426 | 20.930 | 59.295 | 1.00 | 63.27 | A | C |
| ATOM | 1335 | CG | GLU | A | 211 | 13.274 | 19.428 | 59.160 | 1.00 | 63.27 | A | C |
| ATOM | 1336 | CD | GLU | A | 211 | 14.371 | 18.799 | 58.338 | 1.00 | 63.27 | A | C |
| ATOM | 1337 | OE1 | GLU | A | 211 | 15.557 | 19.142 | 58.551 | 1.00 | 63.27 | A | O |
| ATOM | 1338 | OE2 | GLU | A | 211 | 14.037 | 17.953 | 57.482 | 1.00 | 63.27 | A | O |
| ATOM | 1339 | C | GLU | A | 211 | 12.543 | 22.925 | 60.524 | 1.00 | 51.86 | A | C |
| ATOM | 1340 | O | GLU | A | 211 | 12.675 | 23.925 | 59.812 | 1.00 | 51.86 | A | O |
| ATOM | 1341 | N | PRO | A | 212 | 12.675 | 22.977 | 61.860 | 1.00 | 41.65 | A | N |
| ATOM | 1342 | CD | PRO | A | 212 | 12.238 | 21.968 | 62.842 | 1.00 | 22.84 | A | C |
| ATOM | 1343 | CA | PRO | A | 212 | 13.012 | 24.236 | 62.528 | 1.00 | 41.65 | A | C |
| ATOM | 1344 | CB | PRO | A | 212 | 12.444 | 24.036 | 63.931 | 1.00 | 22.84 | A | C |
| ATOM | 1345 | CG | PRO | A | 212 | 12.660 | 22.585 | 64.160 | 1.00 | 22.84 | A | C |
| ATOM | 1346 | C | PRO | A | 212 | 14.496 | 24.543 | 62.523 | 1.00 | 41.65 | A | C |
| ATOM | 1347 | O | PRO | A | 212 | 15.326 | 23.641 | 62.565 | 1.00 | 41.65 | A | O |
| ATOM | 1348 | N | ASN | A | 213 | 14.818 | 25.830 | 62.464 | 1.00 | 28.49 | A | N |
| ATOM | 1349 | CA | ASN | A | 213 | 16.204 | 26.285 | 62.452 | 1.00 | 28.49 | A | C |
| ATOM | 1350 | CB | ASN | A | 213 | 16.550 | 26.787 | 61.068 | 1.00 | 27.65 | A | C |
| ATOM | 1351 | CG | ASN | A | 213 | 16.254 | 25.766 | 60.030 | 1.00 | 27.65 | A | C |
| ATOM | 1352 | OD1 | ASN | A | 213 | 16.914 | 24.737 | 59.964 | 1.00 | 27.65 | A | O |
| ATOM | 1353 | ND2 | ASN | A | 213 | 15.236 | 26.020 | 59.225 | 1.00 | 27.65 | A | N |
| ATOM | 1354 | C | ASN | A | 213 | 16.378 | 27.379 | 63.490 | 1.00 | 28.49 | A | C |
| ATOM | 1355 | O | ASN | A | 213 | 15.443 | 28.119 | 63.790 | 1.00 | 28.49 | A | O |
| ATOM | 1356 | N | VAL | A | 214 | 17.581 | 27.471 | 64.044 | 1.00 | 24.30 | A | N |
| ATOM | 1357 | CA | VAL | A | 214 | 17.862 | 28.460 | 65.073 | 1.00 | 24.30 | A | C |
| ATOM | 1358 | CB | VAL | A | 214 | 19.288 | 28.268 | 65.629 | 1.00 | 19.14 | A | C |
| ATOM | 1359 | CG1 | VAL | A | 214 | 19.382 | 26.930 | 66.325 | 1.00 | 19.14 | A | C |
| ATOM | 1360 | CG2 | VAL | A | 214 | 20.307 | 28.339 | 64.501 | 1.00 | 19.14 | A | C |
| ATOM | 1361 | C | VAL | A | 214 | 17.682 | 29.886 | 64.564 | 1.00 | 24.30 | A | C |
| ATOM | 1362 | O | VAL | A | 214 | 17.854 | 30.155 | 63.385 | 1.00 | 24.30 | A | O |
| ATOM | 1363 | N | SER | A | 215 | 17.337 | 30.802 | 65.460 | 1.00 | 19.39 | A | N |
| ATOM | 1364 | CA | SER | A | 215 | 17.121 | 32.185 | 65.057 | 1.00 | 19.39 | A | C |
| ATOM | 1365 | CB | SER | A | 215 | 15.758 | 32.677 | 65.557 | 1.00 | 32.97 | A | C |
| ATOM | 1366 | OG | SER | A | 215 | 15.610 | 32.445 | 66.944 | 1.00 | 32.97 | A | O |
| ATOM | 1367 | C | SER | A | 215 | 18.204 | 33.143 | 65.511 | 1.00 | 19.39 | A | C |
| ATOM | 1368 | O | SER | A | 215 | 18.102 | 34.334 | 65.298 | 1.00 | 19.39 | A | O |
| ATOM | 1369 | N | TYR | A | 216 | 19.245 | 32.627 | 66.136 | 1.00 | 30.90 | A | N |
| ATOM | 1370 | CA | TYR | A | 216 | 20.333 | 33.486 | 66.584 | 1.00 | 30.90 | A | C |
| ATOM | 1371 | CB | TYR | A | 216 | 20.761 | 33.077 | 67.995 | 1.00 | 36.20 | A | C |
| ATOM | 1372 | CG | TYR | A | 216 | 21.058 | 31.607 | 68.130 | 1.00 | 36.20 | A | C |
| ATOM | 1373 | CD1 | TYR | A | 216 | 22.220 | 31.056 | 67.594 | 1.00 | 36.20 | A | C |
| ATOM | 1374 | CE1 | TYR | A | 216 | 22.510 | 29.712 | 67.742 | 1.00 | 36.20 | A | C |
| ATOM | 1375 | CD2 | TYR | A | 216 | 20.191 | 30.769 | 68.812 | 1.00 | 36.20 | A | C |
| ATOM | 1376 | CE2 | TYR | A | 216 | 20.471 | 29.423 | 68.967 | 1.00 | 36.20 | A | C |
| ATOM | 1377 | CZ | TYR | A | 216 | 21.632 | 28.901 | 68.435 | 1.00 | 36.20 | A | C |
| ATOM | 1378 | OH | TYR | A | 216 | 21.930 | 27.571 | 68.628 | 1.00 | 36.20 | A | O |
| ATOM | 1379 | C | TYR | A | 216 | 21.510 | 33.387 | 65.610 | 1.00 | 30.90 | A | C |
| ATOM | 1380 | O | TYR | A | 216 | 22.675 | 33.480 | 66.007 | 1.00 | 30.90 | A | O |

| | | | | | | | | | | | | |
|---|---|---|---|---|---|---|---|---|---|---|---|---|
| ATOM | 1381 | N | ILE | A | 217 | 21.184 | 33.242 | 64.328 | 1.00 | 33.95 | A | N |
| ATOM | 1382 | CA | ILE | A | 217 | 22.180 | 33.074 | 63.286 | 1.00 | 33.95 | A | C |
| ATOM | 1383 | CB | ILE | A | 217 | 21.665 | 32.147 | 62.172 | 1.00 | 23.35 | A | C |
| ATOM | 1384 | CG2 | ILE | A | 217 | 22.060 | 30.711 | 62.471 | 1.00 | 23.35 | A | C |
| ATOM | 1385 | CG1 | ILE | A | 217 | 20.171 | 32.388 | 61.965 | 1.00 | 23.35 | A | C |
| ATOM | 1386 | CD1 | ILE | A | 217 | 19.745 | 32.263 | 60.548 | 1.00 | 23.35 | A | C |
| ATOM | 1387 | C | ILE | A | 217 | 22.779 | 34.271 | 62.564 | 1.00 | 33.95 | A | C |
| ATOM | 1388 | O | ILE | A | 217 | 23.972 | 34.250 | 62.222 | 1.00 | 33.95 | A | O |
| ATOM | 1389 | N | CYS | A | 218 | 21.986 | 35.301 | 62.307 | 1.00 | 27.47 | A | N |
| ATOM | 1390 | CA | CYS | A | 218 | 22.515 | 36.417 | 61.537 | 1.00 | 27.47 | A | C |
| ATOM | 1391 | CB | CYS | A | 218 | 21.365 | 37.062 | 60.766 | 1.00 | 21.91 | A | C |
| ATOM | 1392 | SG | CYS | A | 218 | 21.878 | 38.063 | 59.388 | 1.00 | 21.91 | A | S |
| ATOM | 1393 | C | CYS | A | 218 | 23.279 | 37.463 | 62.334 | 1.00 | 27.47 | A | C |
| ATOM | 1394 | O | CYS | A | 218 | 23.194 | 37.497 | 63.552 | 1.00 | 27.47 | A | O |
| ATOM | 1395 | N | SER | A | 219 | 24.029 | 38.311 | 61.643 | 1.00 | 17.71 | A | N |
| ATOM | 1396 | CA | SER | A | 219 | 24.804 | 39.358 | 62.296 | 1.00 | 17.71 | A | C |
| ATOM | 1397 | CB | SER | A | 219 | 26.111 | 39.599 | 61.537 | 1.00 | 37.76 | A | C |
| ATOM | 1398 | OG | SER | A | 219 | 26.946 | 38.451 | 61.592 | 1.00 | 37.76 | A | O |
| ATOM | 1399 | C | SER | A | 219 | 24.031 | 40.664 | 62.422 | 1.00 | 17.71 | A | C |
| ATOM | 1400 | O | SER | A | 219 | 23.157 | 40.962 | 61.619 | 1.00 | 17.71 | A | O |
| ATOM | 1401 | N | ARG | A | 220 | 24.367 | 41.436 | 63.446 | 1.00 | 26.36 | A | N |
| ATOM | 1402 | CA | ARG | A | 220 | 23.735 | 42.724 | 63.721 | 1.00 | 26.36 | A | C |
| ATOM | 1403 | CB | ARG | A | 220 | 24.453 | 43.358 | 64.918 | 1.00 | 47.85 | A | C |
| ATOM | 1404 | CG | ARG | A | 220 | 24.362 | 44.868 | 65.075 | 1.00 | 47.85 | A | C |
| ATOM | 1405 | CD | ARG | A | 220 | 24.662 | 45.241 | 66.532 | 1.00 | 47.85 | A | C |
| ATOM | 1406 | NE | ARG | A | 220 | 25.169 | 46.600 | 66.722 | 1.00 | 47.85 | A | N |
| ATOM | 1407 | CZ | ARG | A | 220 | 26.401 | 47.002 | 66.408 | 1.00 | 47.85 | A | C |
| ATOM | 1408 | NH1 | ARG | A | 220 | 27.284 | 46.157 | 65.869 | 1.00 | 47.85 | A | N |
| ATOM | 1409 | NH2 | ARG | A | 220 | 26.764 | 48.253 | 66.664 | 1.00 | 47.85 | A | N |
| ATOM | 1410 | C | ARG | A | 220 | 23.756 | 43.644 | 62.502 | 1.00 | 26.36 | A | C |
| ATOM | 1411 | O | ARG | A | 220 | 24.728 | 43.672 | 61.763 | 1.00 | 26.36 | A | O |
| ATOM | 1412 | N | TYR | A | 221 | 22.677 | 44.393 | 62.303 | 1.00 | 19.12 | A | N |
| ATOM | 1413 | CA | TYR | A | 221 | 22.532 | 45.310 | 61.172 | 1.00 | 19.12 | A | C |
| ATOM | 1414 | CB | TYR | A | 221 | 23.859 | 45.936 | 60.746 | 1.00 | 43.64 | A | C |
| ATOM | 1415 | CG | TYR | A | 221 | 24.521 | 46.861 | 61.737 | 1.00 | 43.64 | A | C |
| ATOM | 1416 | CD1 | TYR | A | 221 | 23.907 | 47.208 | 62.943 | 1.00 | 43.64 | A | C |
| ATOM | 1417 | CE1 | TYR | A | 221 | 24.560 | 48.038 | 63.867 | 1.00 | 43.64 | A | C |
| ATOM | 1418 | CD2 | TYR | A | 221 | 25.796 | 47.372 | 61.471 | 1.00 | 43.64 | A | C |
| ATOM | 1419 | CE2 | TYR | A | 221 | 26.453 | 48.197 | 62.371 | 1.00 | 43.64 | A | C |
| ATOM | 1420 | CZ | TYR | A | 221 | 25.838 | 48.529 | 63.567 | 1.00 | 43.64 | A | C |
| ATOM | 1421 | OH | TYR | A | 221 | 26.515 | 49.357 | 64.444 | 1.00 | 43.64 | A | O |
| ATOM | 1422 | C | TYR | A | 221 | 21.947 | 44.633 | 59.938 | 1.00 | 19.12 | A | C |
| ATOM | 1423 | O | TYR | A | 221 | 21.317 | 45.286 | 59.111 | 1.00 | 19.12 | A | O |
| ATOM | 1424 | N | TYR | A | 222 | 22.137 | 43.325 | 59.814 | 1.00 | 21.81 | A | N |
| ATOM | 1425 | CA | TYR | A | 222 | 21.668 | 42.622 | 58.624 | 1.00 | 21.81 | A | C |
| ATOM | 1426 | CB | TYR | A | 222 | 22.873 | 41.976 | 57.945 | 1.00 | 22.08 | A | C |
| ATOM | 1427 | CG | TYR | A | 222 | 24.000 | 42.953 | 57.800 | 1.00 | 22.08 | A | C |
| ATOM | 1428 | CD1 | TYR | A | 222 | 23.941 | 43.973 | 56.852 | 1.00 | 22.08 | A | C |
| ATOM | 1429 | CE1 | TYR | A | 222 | 24.938 | 44.930 | 56.763 | 1.00 | 22.08 | A | C |
| ATOM | 1430 | CD2 | TYR | A | 222 | 25.090 | 42.911 | 58.658 | 1.00 | 22.08 | A | C |
| ATOM | 1431 | CE2 | TYR | A | 222 | 26.101 | 43.870 | 58.577 | 1.00 | 22.08 | A | C |
| ATOM | 1432 | CZ | TYR | A | 222 | 26.017 | 44.878 | 57.627 | 1.00 | 22.08 | A | C |
| ATOM | 1433 | OH | TYR | A | 222 | 27.003 | 45.832 | 57.559 | 1.00 | 22.08 | A | O |
| ATOM | 1434 | C | TYR | A | 222 | 20.573 | 41.591 | 58.808 | 1.00 | 21.81 | A | C |
| ATOM | 1435 | O | TYR | A | 222 | 20.057 | 41.048 | 57.832 | 1.00 | 21.81 | A | O |
| ATOM | 1436 | N | ARG | A | 223 | 20.215 | 41.336 | 60.056 | 1.00 | 20.07 | A | N |
| ATOM | 1437 | CA | ARG | A | 223 | 19.198 | 40.353 | 60.379 | 1.00 | 20.07 | A | C |
| ATOM | 1438 | CB | ARG | A | 223 | 19.194 | 40.081 | 61.875 | 1.00 | 32.25 | A | C |
| ATOM | 1439 | CG | ARG | A | 223 | 20.575 | 39.876 | 62.450 | 1.00 | 32.25 | A | C |
| ATOM | 1440 | CD | ARG | A | 223 | 20.486 | 39.687 | 63.927 | 1.00 | 32.25 | A | C |
| ATOM | 1441 | NE | ARG | A | 223 | 19.861 | 38.412 | 64.231 | 1.00 | 32.25 | A | N |
| ATOM | 1442 | CZ | ARG | A | 223 | 19.151 | 38.187 | 65.323 | 1.00 | 32.25 | A | C |
| ATOM | 1443 | NH1 | ARG | A | 223 | 18.982 | 39.163 | 66.208 | 1.00 | 32.25 | A | N |

| | | | | | | | | | | | | |
|---|---|---|---|---|---|---|---|---|---|---|---|---|
| ATOM | 1444 | NH2 | ARG | A | 223 | 18.613 | 36.998 | 65.524 | 1.00 | 32.25 | A | N |
| ATOM | 1445 | C | ARG | A | 223 | 17.815 | 40.774 | 59.935 | 1.00 | 20.07 | A | C |
| ATOM | 1446 | O | ARG | A | 223 | 17.406 | 41.922 | 60.116 | 1.00 | 20.07 | A | O |
| ATOM | 1447 | N | ALA | A | 224 | 17.099 | 39.822 | 59.349 | 1.00 | 39.74 | A | N |
| ATOM | 1448 | CA | ALA | A | 224 | 15.748 | 40.055 | 58.859 | 1.00 | 39.74 | A | C |
| ATOM | 1449 | CB | ALA | A | 224 | 15.332 | 38.935 | 57.930 | 1.00 | 38.16 | A | C |
| ATOM | 1450 | C | ALA | A | 224 | 14.789 | 40.158 | 60.030 | 1.00 | 39.74 | A | C |
| ATOM | 1451 | O | ALA | A | 224 | 14.984 | 39.519 | 61.054 | 1.00 | 39.74 | A | O |
| ATOM | 1452 | N | PRO | A | 225 | 13.727 | 40.953 | 59.882 | 1.00 | 29.06 | A | N |
| ATOM | 1453 | CD | PRO | A | 225 | 13.321 | 41.572 | 58.610 | 1.00 | 31.89 | A | C |
| ATOM | 1454 | CA | PRO | A | 225 | 12.709 | 41.174 | 60.918 | 1.00 | 29.06 | A | C |
| ATOM | 1455 | CB | PRO | A | 225 | 11.588 | 41.872 | 60.152 | 1.00 | 31.89 | A | C |
| ATOM | 1456 | CG | PRO | A | 225 | 12.321 | 42.588 | 59.061 | 1.00 | 31.89 | A | C |
| ATOM | 1457 | C | PRO | A | 225 | 12.216 | 39.919 | 61.626 | 1.00 | 29.06 | A | C |
| ATOM | 1458 | O | PRO | A | 225 | 11.963 | 39.937 | 62.826 | 1.00 | 29.06 | A | O |
| ATOM | 1459 | N | GLU | A | 226 | 12.074 | 38.834 | 60.875 | 1.00 | 29.04 | A | N |
| ATOM | 1460 | CA | GLU | A | 226 | 11.601 | 37.581 | 61.436 | 1.00 | 29.04 | A | C |
| ATOM | 1461 | CB | GLU | A | 226 | 11.171 | 36.643 | 60.313 | 1.00 | 30.42 | A | C |
| ATOM | 1462 | CG | GLU | A | 226 | 10.811 | 37.354 | 59.003 | 1.00 | 30.42 | A | C |
| ATOM | 1463 | CD | GLU | A | 226 | 11.991 | 37.475 | 58.042 | 1.00 | 30.42 | A | C |
| ATOM | 1464 | OE1 | GLU | A | 226 | 12.562 | 36.435 | 57.649 | 1.00 | 30.42 | A | O |
| ATOM | 1465 | OE2 | GLU | A | 226 | 12.344 | 38.609 | 57.674 | 1.00 | 30.42 | A | O |
| ATOM | 1466 | C | GLU | A | 226 | 12.675 | 36.911 | 62.287 | 1.00 | 29.04 | A | C |
| ATOM | 1467 | O | GLU | A | 226 | 12.380 | 36.061 | 63.123 | 1.00 | 29.04 | A | O |
| ATOM | 1468 | N | LEU | A | 227 | 13.929 | 37.270 | 62.071 | 1.00 | 30.09 | A | N |
| ATOM | 1469 | CA | LEU | A | 227 | 14.984 | 36.676 | 62.873 | 1.00 | 30.09 | A | C |
| ATOM | 1470 | CB | LEU | A | 227 | 16.335 | 36.770 | 62.152 | 1.00 | 14.48 | A | C |
| ATOM | 1471 | CG | LEU | A | 227 | 16.511 | 35.843 | 60.939 | 1.00 | 14.48 | A | C |
| ATOM | 1472 | CD1 | LEU | A | 227 | 17.902 | 36.054 | 60.366 | 1.00 | 14.48 | A | C |
| ATOM | 1473 | CD2 | LEU | A | 227 | 16.317 | 34.379 | 61.320 | 1.00 | 14.48 | A | C |
| ATOM | 1474 | C | LEU | A | 227 | 15.028 | 37.414 | 64.202 | 1.00 | 30.09 | A | C |
| ATOM | 1475 | O | LEU | A | 227 | 15.310 | 36.829 | 65.244 | 1.00 | 30.09 | A | O |
| ATOM | 1476 | N | ILE | A | 228 | 14.722 | 38.702 | 64.158 | 1.00 | 22.58 | A | N |
| ATOM | 1477 | CA | ILE | A | 228 | 14.727 | 39.523 | 65.352 | 1.00 | 22.58 | A | C |
| ATOM | 1478 | CB | ILE | A | 228 | 14.562 | 41.019 | 64.990 | 1.00 | 17.70 | A | C |
| ATOM | 1479 | CG2 | ILE | A | 228 | 14.296 | 41.837 | 66.238 | 1.00 | 17.70 | A | C |
| ATOM | 1480 | CG1 | ILE | A | 228 | 15.811 | 41.519 | 64.255 | 1.00 | 17.70 | A | C |
| ATOM | 1481 | CD1 | ILE | A | 228 | 15.633 | 42.891 | 63.602 | 1.00 | 17.70 | A | C |
| ATOM | 1482 | C | ILE | A | 228 | 13.569 | 39.073 | 66.220 | 1.00 | 22.58 | A | C |
| ATOM | 1483 | O | ILE | A | 228 | 13.646 | 39.102 | 67.442 | 1.00 | 22.58 | A | O |
| ATOM | 1484 | N | PHE | A | 229 | 12.497 | 38.641 | 65.571 | 1.00 | 34.78 | A | N |
| ATOM | 1485 | CA | PHE | A | 229 | 11.308 | 38.179 | 66.271 | 1.00 | 34.78 | A | C |
| ATOM | 1486 | CB | PHE | A | 229 | 10.072 | 38.324 | 65.382 | 1.00 | 23.42 | A | C |
| ATOM | 1487 | CG | PHE | A | 229 | 9.541 | 39.723 | 65.298 | 1.00 | 23.42 | A | C |
| ATOM | 1488 | CD1 | PHE | A | 229 | 9.099 | 40.385 | 66.441 | 1.00 | 23.42 | A | C |
| ATOM | 1489 | CD2 | PHE | A | 229 | 9.464 | 40.376 | 64.075 | 1.00 | 23.42 | A | C |
| ATOM | 1490 | CE1 | PHE | A | 229 | 8.589 | 41.680 | 66.365 | 1.00 | 23.42 | A | C |
| ATOM | 1491 | CE2 | PHE | A | 229 | 8.959 | 41.670 | 63.989 | 1.00 | 23.42 | A | C |
| ATOM | 1492 | CZ | PHE | A | 229 | 8.519 | 42.323 | 65.133 | 1.00 | 23.42 | A | C |
| ATOM | 1493 | C | PHE | A | 229 | 11.425 | 36.729 | 66.706 | 1.00 | 34.78 | A | C |
| ATOM | 1494 | O | PHE | A | 229 | 10.438 | 36.138 | 67.113 | 1.00 | 34.78 | A | O |
| ATOM | 1495 | N | GLY | A | 230 | 12.620 | 36.157 | 66.596 | 1.00 | 32.23 | A | N |
| ATOM | 1496 | CA | GLY | A | 230 | 12.843 | 34.783 | 67.016 | 1.00 | 32.23 | A | C |
| ATOM | 1497 | C | GLY | A | 230 | 12.188 | 33.680 | 66.205 | 1.00 | 32.23 | A | C |
| ATOM | 1498 | O | GLY | A | 230 | 12.112 | 32.540 | 66.660 | 1.00 | 32.23 | A | O |
| ATOM | 1499 | N | ALA | A | 231 | 11.714 | 34.004 | 65.006 | 1.00 | 28.55 | A | N |
| ATOM | 1500 | CA | ALA | A | 231 | 11.083 | 32.997 | 64.154 | 1.00 | 28.55 | A | C |
| ATOM | 1501 | CB | ALA | A | 231 | 10.648 | 33.619 | 62.834 | 1.00 | 8.54 | A | C |
| ATOM | 1502 | C | ALA | A | 231 | 12.071 | 31.870 | 63.892 | 1.00 | 28.55 | A | C |
| ATOM | 1503 | O | ALA | A | 231 | 13.265 | 32.108 | 63.750 | 1.00 | 28.55 | A | O |
| ATOM | 1504 | N | THR | A | 232 | 11.584 | 30.637 | 63.842 | 1.00 | 39.05 | A | N |
| ATOM | 1505 | CA | THR | A | 232 | 12.469 | 29.509 | 63.565 | 1.00 | 39.05 | A | C |
| ATOM | 1506 | CB | THR | A | 232 | 12.511 | 28.526 | 64.733 | 1.00 | 32.41 | A | C |

| | | | | | | | | | | | | |
|---|---|---|---|---|---|---|---|---|---|---|---|---|
| ATOM | 1507 | OG1 | THR | A | 232 | 11.176 | 28.198 | 65.112 | 1.00 | 32.41 | A | O |
| ATOM | 1508 | CG2 | THR | A | 232 | 13.240 | 29.127 | 65.914 | 1.00 | 32.41 | A | C |
| ATOM | 1509 | C | THR | A | 232 | 12.012 | 28.761 | 62.309 | 1.00 | 39.05 | A | C |
| ATOM | 1510 | O | THR | A | 232 | 12.594 | 27.735 | 61.926 | 1.00 | 39.05 | A | O |
| ATOM | 1511 | N | ASP | A | 233 | 10.974 | 29.291 | 61.667 | 1.00 | 29.68 | A | N |
| ATOM | 1512 | CA | ASP | A | 233 | 10.446 | 28.699 | 60.444 | 1.00 | 29.68 | A | C |
| ATOM | 1513 | CB | ASP | A | 233 | 8.940 | 28.463 | 60.587 | 1.00 | 30.27 | A | C |
| ATOM | 1514 | CG | ASP | A | 233 | 8.145 | 29.752 | 60.554 | 1.00 | 30.27 | A | C |
| ATOM | 1515 | OD1 | ASP | A | 233 | 8.537 | 30.711 | 61.247 | 1.00 | 30.27 | A | O |
| ATOM | 1516 | OD2 | ASP | A | 233 | 7.126 | 29.812 | 59.839 | 1.00 | 30.27 | A | O |
| ATOM | 1517 | C | ASP | A | 233 | 10.733 | 29.624 | 59.256 | 1.00 | 29.68 | A | C |
| ATOM | 1518 | O | ASP | A | 233 | 9.914 | 29.778 | 58.344 | 1.00 | 29.68 | A | O |
| ATOM | 1519 | N | TYR | A | 234 | 11.910 | 30.237 | 59.276 | 1.00 | 31.59 | A | N |
| ATOM | 1520 | CA | TYR | A | 234 | 12.310 | 31.155 | 58.216 | 1.00 | 31.59 | A | C |
| ATOM | 1521 | CB | TYR | A | 234 | 13.355 | 32.153 | 58.740 | 1.00 | 11.36 | A | C |
| ATOM | 1522 | CG | TYR | A | 234 | 14.615 | 31.511 | 59.295 | 1.00 | 11.36 | A | C |
| ATOM | 1523 | CD1 | TYR | A | 234 | 15.626 | 31.059 | 58.449 | 1.00 | 11.36 | A | C |
| ATOM | 1524 | CE1 | TYR | A | 234 | 16.785 | 30.477 | 58.963 | 1.00 | 11.36 | A | C |
| ATOM | 1525 | CD2 | TYR | A | 234 | 14.796 | 31.360 | 60.673 | 1.00 | 11.36 | A | C |
| ATOM | 1526 | CE2 | TYR | A | 234 | 15.950 | 30.777 | 61.190 | 1.00 | 11.36 | A | C |
| ATOM | 1527 | CZ | TYR | A | 234 | 16.937 | 30.339 | 60.329 | 1.00 | 11.36 | A | C |
| ATOM | 1528 | OH | TYR | A | 234 | 18.076 | 29.757 | 60.817 | 1.00 | 11.36 | A | O |
| ATOM | 1529 | C | TYR | A | 234 | 12.849 | 30.438 | 56.986 | 1.00 | 31.59 | A | C |
| ATOM | 1530 | O | TYR | A | 234 | 13.284 | 29.290 | 57.048 | 1.00 | 31.59 | A | O |
| ATOM | 1531 | N | THR | A | 235 | 12.821 | 31.145 | 55.866 | 1.00 | 32.65 | A | N |
| ATOM | 1532 | CA | THR | A | 235 | 13.295 | 30.609 | 54.605 | 1.00 | 32.65 | A | C |
| ATOM | 1533 | CB | THR | A | 235 | 12.183 | 30.633 | 53.565 | 1.00 | 33.38 | A | C |
| ATOM | 1534 | OG1 | THR | A | 235 | 11.831 | 31.990 | 53.287 | 1.00 | 33.38 | A | O |
| ATOM | 1535 | CG2 | THR | A | 235 | 10.960 | 29.912 | 54.089 | 1.00 | 33.38 | A | C |
| ATOM | 1536 | C | THR | A | 235 | 14.466 | 31.444 | 54.101 | 1.00 | 32.65 | A | C |
| ATOM | 1537 | O | THR | A | 235 | 14.894 | 32.385 | 54.757 | 1.00 | 32.65 | A | O |
| ATOM | 1538 | N | SER | A | 236 | 14.971 | 31.095 | 52.928 | 1.00 | 23.70 | A | N |
| ATOM | 1539 | CA | SER | A | 236 | 16.098 | 31.788 | 52.333 | 1.00 | 23.70 | A | C |
| ATOM | 1540 | CB | SER | A | 236 | 16.423 | 31.149 | 50.987 | 1.00 | 20.73 | A | C |
| ATOM | 1541 | OG | SER | A | 236 | 16.789 | 29.793 | 51.138 | 1.00 | 20.73 | A | O |
| ATOM | 1542 | C | SER | A | 236 | 15.880 | 33.283 | 52.148 | 1.00 | 23.70 | A | C |
| ATOM | 1543 | O | SER | A | 236 | 16.815 | 34.019 | 51.834 | 1.00 | 23.70 | A | O |
| ATOM | 1544 | N | SER | A | 237 | 14.647 | 33.734 | 52.331 | 1.00 | 21.91 | A | N |
| ATOM | 1545 | CA | SER | A | 237 | 14.347 | 35.141 | 52.169 | 1.00 | 21.91 | A | C |
| ATOM | 1546 | CB | SER | A | 237 | 12.890 | 35.416 | 52.500 | 1.00 | 44.41 | A | C |
| ATOM | 1547 | OG | SER | A | 237 | 12.144 | 34.212 | 52.532 | 1.00 | 44.41 | A | O |
| ATOM | 1548 | C | SER | A | 237 | 15.232 | 35.973 | 53.073 | 1.00 | 21.91 | A | C |
| ATOM | 1549 | O | SER | A | 237 | 15.502 | 37.132 | 52.761 | 1.00 | 21.91 | A | O |
| ATOM | 1550 | N | ILE | A | 238 | 15.688 | 35.392 | 54.186 | 1.00 | 15.47 | A | N |
| ATOM | 1551 | CA | ILE | A | 238 | 16.536 | 36.129 | 55.115 | 1.00 | 15.47 | A | C |
| ATOM | 1552 | CB | ILE | A | 238 | 16.938 | 35.289 | 56.353 | 1.00 | 19.37 | A | C |
| ATOM | 1553 | CG2 | ILE | A | 238 | 15.702 | 34.868 | 57.119 | 1.00 | 19.37 | A | C |
| ATOM | 1554 | CG1 | ILE | A | 238 | 17.783 | 34.091 | 55.936 | 1.00 | 19.37 | A | C |
| ATOM | 1555 | CD1 | ILE | A | 238 | 18.383 | 33.352 | 57.111 | 1.00 | 19.37 | A | C |
| ATOM | 1556 | C | ILE | A | 238 | 17.792 | 36.636 | 54.434 | 1.00 | 15.47 | A | C |
| ATOM | 1557 | O | ILE | A | 238 | 18.266 | 37.726 | 54.732 | 1.00 | 15.47 | A | O |
| ATOM | 1558 | N | ASP | A | 239 | 18.313 | 35.842 | 53.507 | 1.00 | 27.65 | A | N |
| ATOM | 1559 | CA | ASP | A | 239 | 19.503 | 36.208 | 52.756 | 1.00 | 27.65 | A | C |
| ATOM | 1560 | CB | ASP | A | 239 | 19.974 | 35.035 | 51.902 | 1.00 | 25.40 | A | C |
| ATOM | 1561 | CG | ASP | A | 239 | 20.830 | 34.060 | 52.669 | 1.00 | 25.40 | A | C |
| ATOM | 1562 | OD1 | ASP | A | 239 | 21.003 | 34.235 | 53.889 | 1.00 | 25.40 | A | O |
| ATOM | 1563 | OD2 | ASP | A | 239 | 21.339 | 33.110 | 52.052 | 1.00 | 25.40 | A | O |
| ATOM | 1564 | C | ASP | A | 239 | 19.185 | 37.382 | 51.854 | 1.00 | 27.65 | A | C |
| ATOM | 1565 | O | ASP | A | 239 | 20.022 | 38.253 | 51.655 | 1.00 | 27.65 | A | O |
| ATOM | 1566 | N | VAL | A | 240 | 17.975 | 37.405 | 51.306 | 1.00 | 27.60 | A | N |
| ATOM | 1567 | CA | VAL | A | 240 | 17.582 | 38.486 | 50.413 | 1.00 | 27.60 | A | C |
| ATOM | 1568 | CB | VAL | A | 240 | 16.252 | 38.162 | 49.675 | 1.00 | 24.32 | A | C |
| ATOM | 1569 | CG1 | VAL | A | 240 | 15.867 | 39.315 | 48.736 | 1.00 | 24.32 | A | C |

| | | | | | | | | | | | | |
|---|---|---|---|---|---|---|---|---|---|---|---|---|
| ATOM | 1570 | CG2 | VAL | A | 240 | 16.413 | 36.860 | 48.876 | 1.00 | 24.32 | A | C |
| ATOM | 1571 | C | VAL | A | 240 | 17.445 | 39.803 | 51.149 | 1.00 | 27.60 | A | C |
| ATOM | 1572 | O | VAL | A | 240 | 17.668 | 40.867 | 50.572 | 1.00 | 27.60 | A | O |
| ATOM | 1573 | N | TRP | A | 241 | 17.083 | 39.722 | 52.426 | 1.00 | 20.61 | A | N |
| ATOM | 1574 | CA | TRP | A | 241 | 16.924 | 40.903 | 53.262 | 1.00 | 20.61 | A | C |
| ATOM | 1575 | CB | TRP | A | 241 | 16.156 | 40.540 | 54.533 | 1.00 | 22.37 | A | C |
| ATOM | 1576 | CG | TRP | A | 241 | 16.088 | 41.646 | 55.553 | 1.00 | 22.37 | A | C |
| ATOM | 1577 | CD2 | TRP | A | 241 | 15.029 | 42.599 | 55.702 | 1.00 | 22.37 | A | C |
| ATOM | 1578 | CE2 | TRP | A | 241 | 15.419 | 43.463 | 56.757 | 1.00 | 22.37 | A | C |
| ATOM | 1579 | CE3 | TRP | A | 241 | 13.773 | 42.740 | 55.123 | 1.00 | 22.37 | A | C |
| ATOM | 1580 | CD1 | TRP | A | 241 | 17.054 | 42.000 | 56.445 | 1.00 | 22.37 | A | C |
| ATOM | 1581 | NE1 | TRP | A | 241 | 16.659 | 43.101 | 57.158 | 1.00 | 22.37 | A | N |
| ATOM | 1582 | CZ2 | TRP | A | 241 | 14.612 | 44.545 | 57.149 | 1.00 | 22.37 | A | C |
| ATOM | 1583 | CZ3 | TRP | A | 241 | 12.978 | 43.808 | 55.522 | 1.00 | 22.37 | A | C |
| ATOM | 1584 | CH2 | TRP | A | 241 | 13.387 | 44.671 | 56.560 | 1.00 | 22.37 | A | C |
| ATOM | 1585 | C | TRP | A | 241 | 18.297 | 41.447 | 53.606 | 1.00 | 20.61 | A | C |
| ATOM | 1586 | O | TRP | A | 241 | 18.552 | 42.641 | 53.486 | 1.00 | 20.61 | A | O |
| ATOM | 1587 | N | SER | A | 242 | 19.181 | 40.555 | 54.024 | 1.00 | 24.28 | A | N |
| ATOM | 1588 | CA | SER | A | 242 | 20.528 | 40.942 | 54.385 | 1.00 | 24.28 | A | C |
| ATOM | 1589 | CB | SER | A | 242 | 21.281 | 39.750 | 54.968 | 1.00 | 51.96 | A | C |
| ATOM | 1590 | OG | SER | A | 242 | 21.367 | 38.719 | 54.010 | 1.00 | 51.96 | A | O |
| ATOM | 1591 | C | SER | A | 242 | 21.258 | 41.500 | 53.177 | 1.00 | 24.28 | A | C |
| ATOM | 1592 | O | SER | A | 242 | 22.085 | 42.395 | 53.307 | 1.00 | 24.28 | A | O |
| ATOM | 1593 | N | ALA | A | 243 | 20.935 | 40.979 | 52.000 | 1.00 | 27.27 | A | N |
| ATOM | 1594 | CA | ALA | A | 243 | 21.569 | 41.436 | 50.773 | 1.00 | 27.27 | A | C |
| ATOM | 1595 | CB | ALA | A | 243 | 21.195 | 40.537 | 49.615 | 1.00 | 19.62 | A | C |
| ATOM | 1596 | C | ALA | A | 243 | 21.098 | 42.853 | 50.528 | 1.00 | 27.27 | A | C |
| ATOM | 1597 | O | ALA | A | 243 | 21.883 | 43.725 | 50.144 | 1.00 | 27.27 | A | O |
| ATOM | 1598 | N | GLY | A | 244 | 19.813 | 43.080 | 50.779 | 1.00 | 23.34 | A | N |
| ATOM | 1599 | CA | GLY | A | 244 | 19.237 | 44.397 | 50.589 | 1.00 | 23.34 | A | C |
| ATOM | 1600 | C | GLY | A | 244 | 19.853 | 45.415 | 51.523 | 1.00 | 23.34 | A | C |
| ATOM | 1601 | O | GLY | A | 244 | 19.968 | 46.587 | 51.182 | 1.00 | 23.34 | A | O |
| ATOM | 1602 | N | CYS | A | 245 | 20.237 | 44.970 | 52.713 | 1.00 | 28.06 | A | N |
| ATOM | 1603 | CA | CYS | A | 245 | 20.859 | 45.851 | 53.692 | 1.00 | 28.06 | A | C |
| ATOM | 1604 | CB | CYS | A | 245 | 20.998 | 45.139 | 55.030 | 1.00 | 18.34 | A | C |
| ATOM | 1605 | SG | CYS | A | 245 | 19.452 | 44.946 | 55.870 | 1.00 | 18.34 | A | S |
| ATOM | 1606 | C | CYS | A | 245 | 22.230 | 46.276 | 53.188 | 1.00 | 28.06 | A | C |
| ATOM | 1607 | O | CYS | A | 245 | 22.715 | 47.354 | 53.511 | 1.00 | 28.06 | A | O |
| ATOM | 1608 | N | VAL | A | 246 | 22.860 | 45.420 | 52.399 | 1.00 | 18.53 | A | N |
| ATOM | 1609 | CA | VAL | A | 246 | 24.156 | 45.756 | 51.844 | 1.00 | 18.53 | A | C |
| ATOM | 1610 | CB | VAL | A | 246 | 24.866 | 44.517 | 51.315 | 1.00 | 28.80 | A | C |
| ATOM | 1611 | CG1 | VAL | A | 246 | 26.152 | 44.929 | 50.565 | 1.00 | 28.80 | A | C |
| ATOM | 1612 | CG2 | VAL | A | 246 | 25.178 | 43.583 | 52.489 | 1.00 | 28.80 | A | C |
| ATOM | 1613 | C | VAL | A | 246 | 23.993 | 46.766 | 50.709 | 1.00 | 18.53 | A | C |
| ATOM | 1614 | O | VAL | A | 246 | 24.771 | 47.707 | 50.586 | 1.00 | 18.53 | A | O |
| ATOM | 1615 | N | LEU | A | 247 | 22.980 | 46.574 | 49.883 | 1.00 | 15.05 | A | N |
| ATOM | 1616 | CA | LEU | A | 247 | 22.738 | 47.501 | 48.799 | 1.00 | 15.05 | A | C |
| ATOM | 1617 | CB | LEU | A | 247 | 21.523 | 47.059 | 47.991 | 1.00 | 23.78 | A | C |
| ATOM | 1618 | CG | LEU | A | 247 | 21.196 | 47.935 | 46.787 | 1.00 | 23.78 | A | C |
| ATOM | 1619 | CD1 | LEU | A | 247 | 22.345 | 47.897 | 45.797 | 1.00 | 23.78 | A | C |
| ATOM | 1620 | CD2 | LEU | A | 247 | 19.907 | 47.457 | 46.148 | 1.00 | 23.78 | A | C |
| ATOM | 1621 | C | LEU | A | 247 | 22.484 | 48.883 | 49.389 | 1.00 | 15.05 | A | C |
| ATOM | 1622 | O | LEU | A | 247 | 23.106 | 49.876 | 49.005 | 1.00 | 15.05 | A | O |
| ATOM | 1623 | N | ALA | A | 248 | 21.560 | 48.938 | 50.338 | 1.00 | 29.33 | A | N |
| ATOM | 1624 | CA | ALA | A | 248 | 21.208 | 50.189 | 50.976 | 1.00 | 29.33 | A | C |
| ATOM | 1625 | CB | ALA | A | 248 | 20.056 | 49.977 | 51.934 | 1.00 | 29.28 | A | C |
| ATOM | 1626 | C | ALA | A | 248 | 22.394 | 50.798 | 51.701 | 1.00 | 29.33 | A | C |
| ATOM | 1627 | O | ALA | A | 248 | 22.507 | 52.018 | 51.763 | 1.00 | 29.33 | A | O |
| ATOM | 1628 | N | GLU | A | 249 | 23.280 | 49.966 | 52.243 | 1.00 | 23.27 | A | N |
| ATOM | 1629 | CA | GLU | A | 249 | 24.454 | 50.477 | 52.949 | 1.00 | 23.27 | A | C |
| ATOM | 1630 | CB | GLU | A | 249 | 25.086 | 49.392 | 53.836 | 1.00 | 21.28 | A | C |
| ATOM | 1631 | CG | GLU | A | 249 | 26.201 | 49.917 | 54.763 | 1.00 | 21.28 | A | C |
| ATOM | 1632 | CD | GLU | A | 249 | 26.807 | 48.848 | 55.661 | 1.00 | 21.28 | A | C |

| | | | | | | | | | | | | |
|---|---|---|---|---|---|---|---|---|---|---|---|---|
| ATOM | 1633 | OE1 | GLU | A | 249 | 26.043 | 48.082 | 56.274 | 1.00 | 21.28 | A | O |
| ATOM | 1634 | OE2 | GLU | A | 249 | 28.046 | 48.783 | 55.770 | 1.00 | 21.28 | A | O |
| ATOM | 1635 | C | GLU | A | 249 | 25.515 | 51.011 | 52.009 | 1.00 | 23.27 | A | C |
| ATOM | 1636 | O | GLU | A | 249 | 26.317 | 51.855 | 52.395 | 1.00 | 23.27 | A | O |
| ATOM | 1637 | N | LEU | A | 250 | 25.545 | 50.518 | 50.779 | 1.00 | 29.28 | A | N |
| ATOM | 1638 | CA | LEU | A | 250 | 26.543 | 51.005 | 49.838 | 1.00 | 29.28 | A | C |
| ATOM | 1639 | CB | LEU | A | 250 | 26.853 | 49.941 | 48.786 | 1.00 | 17.65 | A | C |
| ATOM | 1640 | CG | LEU | A | 250 | 27.688 | 48.781 | 49.346 | 1.00 | 17.65 | A | C |
| ATOM | 1641 | CD1 | LEU | A | 250 | 27.984 | 47.744 | 48.268 | 1.00 | 17.65 | A | C |
| ATOM | 1642 | CD2 | LEU | A | 250 | 28.976 | 49.325 | 49.916 | 1.00 | 17.65 | A | C |
| ATOM | 1643 | C | LEU | A | 250 | 26.084 | 52.308 | 49.208 | 1.00 | 29.28 | A | C |
| ATOM | 1644 | O | LEU | A | 250 | 26.899 | 53.161 | 48.878 | 1.00 | 29.28 | A | O |
| ATOM | 1645 | N | LEU | A | 251 | 24.777 | 52.475 | 49.064 | 1.00 | 33.91 | A | N |
| ATOM | 1646 | CA | LEU | A | 251 | 24.250 | 53.706 | 48.503 | 1.00 | 33.91 | A | C |
| ATOM | 1647 | CB | LEU | A | 251 | 22.810 | 53.518 | 48.040 | 1.00 | 17.87 | A | C |
| ATOM | 1648 | CG | LEU | A | 251 | 22.586 | 52.544 | 46.893 | 1.00 | 17.87 | A | C |
| ATOM | 1649 | CD1 | LEU | A | 251 | 21.106 | 52.441 | 46.626 | 1.00 | 17.87 | A | C |
| ATOM | 1650 | CD2 | LEU | A | 251 | 23.334 | 53.003 | 45.649 | 1.00 | 17.87 | A | C |
| ATOM | 1651 | C | LEU | A | 251 | 24.291 | 54.805 | 49.556 | 1.00 | 33.91 | A | C |
| ATOM | 1652 | O | LEU | A | 251 | 24.632 | 55.948 | 49.259 | 1.00 | 33.91 | A | O |
| ATOM | 1653 | N | LEU | A | 252 | 23.960 | 54.454 | 50.791 | 1.00 | 24.63 | A | N |
| ATOM | 1654 | CA | LEU | A | 252 | 23.918 | 55.420 | 51.874 | 1.00 | 24.63 | A | C |
| ATOM | 1655 | CB | LEU | A | 252 | 23.040 | 54.877 | 53.006 | 1.00 | 51.78 | A | C |
| ATOM | 1656 | CG | LEU | A | 252 | 22.366 | 55.940 | 53.898 | 1.00 | 51.78 | A | C |
| ATOM | 1657 | CD1 | LEU | A | 252 | 21.095 | 56.435 | 53.197 | 1.00 | 51.78 | A | C |
| ATOM | 1658 | CD2 | LEU | A | 252 | 22.010 | 55.368 | 55.281 | 1.00 | 51.78 | A | C |
| ATOM | 1659 | C | LEU | A | 252 | 25.268 | 55.822 | 52.448 | 1.00 | 24.63 | A | C |
| ATOM | 1660 | O | LEU | A | 252 | 25.445 | 56.966 | 52.845 | 1.00 | 24.63 | A | O |
| ATOM | 1661 | N | GLY | A | 253 | 26.211 | 54.889 | 52.509 | 1.00 | 22.47 | A | N |
| ATOM | 1662 | CA | GLY | A | 253 | 27.516 | 55.196 | 53.065 | 1.00 | 22.47 | A | C |
| ATOM | 1663 | C | GLY | A | 253 | 27.625 | 54.797 | 54.525 | 1.00 | 22.47 | A | C |
| ATOM | 1664 | O | GLY | A | 253 | 28.633 | 55.040 | 55.187 | 1.00 | 22.47 | A | O |
| ATOM | 1665 | N | GLN | A | 254 | 26.572 | 54.173 | 55.031 | 1.00 | 20.71 | A | N |
| ATOM | 1666 | CA | GLN | A | 254 | 26.532 | 53.711 | 56.407 | 1.00 | 20.71 | A | C |
| ATOM | 1667 | CB | GLN | A | 254 | 26.332 | 54.891 | 57.350 | 1.00 | 46.21 | A | C |
| ATOM | 1668 | CG | GLN | A | 254 | 25.211 | 55.825 | 56.932 | 1.00 | 46.21 | A | C |
| ATOM | 1669 | CD | GLN | A | 254 | 24.534 | 56.458 | 58.130 | 1.00 | 46.21 | A | C |
| ATOM | 1670 | OE1 | GLN | A | 254 | 24.059 | 55.749 | 59.022 | 1.00 | 46.21 | A | O |
| ATOM | 1671 | NE2 | GLN | A | 254 | 24.480 | 57.789 | 58.163 | 1.00 | 46.21 | A | N |
| ATOM | 1672 | C | GLN | A | 254 | 25.366 | 52.738 | 56.525 | 1.00 | 20.71 | A | C |
| ATOM | 1673 | O | GLN | A | 254 | 24.488 | 52.717 | 55.676 | 1.00 | 20.71 | A | O |
| ATOM | 1674 | N | PRO | A | 255 | 25.348 | 51.915 | 57.580 | 1.00 | 15.57 | A | N |
| ATOM | 1675 | CD | PRO | A | 255 | 26.420 | 51.769 | 58.573 | 1.00 | 27.15 | A | C |
| ATOM | 1676 | CA | PRO | A | 255 | 24.295 | 50.928 | 57.828 | 1.00 | 15.57 | A | C |
| ATOM | 1677 | CB | PRO | A | 255 | 24.743 | 50.279 | 59.129 | 1.00 | 27.15 | A | C |
| ATOM | 1678 | CG | PRO | A | 255 | 26.214 | 50.358 | 59.034 | 1.00 | 27.15 | A | C |
| ATOM | 1679 | C | PRO | A | 255 | 22.876 | 51.499 | 57.926 | 1.00 | 15.57 | A | C |
| ATOM | 1680 | O | PRO | A | 255 | 22.600 | 52.391 | 58.721 | 1.00 | 15.57 | A | O |
| ATOM | 1681 | N | ILE | A | 256 | 21.971 | 50.945 | 57.130 | 1.00 | 22.32 | A | N |
| ATOM | 1682 | CA | ILE | A | 256 | 20.598 | 51.417 | 57.109 | 1.00 | 22.32 | A | C |
| ATOM | 1683 | CB | ILE | A | 256 | 19.851 | 50.947 | 55.813 | 1.00 | 25.12 | A | C |
| ATOM | 1684 | CG2 | ILE | A | 256 | 19.696 | 49.436 | 55.777 | 1.00 | 25.12 | A | C |
| ATOM | 1685 | CG1 | ILE | A | 256 | 18.493 | 51.639 | 55.731 | 1.00 | 25.12 | A | C |
| ATOM | 1686 | CD1 | ILE | A | 256 | 17.842 | 51.523 | 54.382 | 1.00 | 25.12 | A | C |
| ATOM | 1687 | C | ILE | A | 256 | 19.773 | 51.092 | 58.352 | 1.00 | 22.32 | A | C |
| ATOM | 1688 | O | ILE | A | 256 | 19.025 | 51.949 | 58.815 | 1.00 | 22.32 | A | O |
| ATOM | 1689 | N | PHE | A | 257 | 19.898 | 49.884 | 58.902 | 1.00 | 24.26 | A | N |
| ATOM | 1690 | CA | PHE | A | 257 | 19.135 | 49.534 | 60.116 | 1.00 | 24.26 | A | C |
| ATOM | 1691 | CB | PHE | A | 257 | 18.205 | 48.344 | 59.851 | 1.00 | 15.35 | A | C |
| ATOM | 1692 | CG | PHE | A | 257 | 17.269 | 48.543 | 58.704 | 1.00 | 15.35 | A | C |
| ATOM | 1693 | CD1 | PHE | A | 257 | 16.415 | 49.635 | 58.665 | 1.00 | 15.35 | A | C |
| ATOM | 1694 | CD2 | PHE | A | 257 | 17.217 | 47.617 | 57.672 | 1.00 | 15.35 | A | C |
| ATOM | 1695 | CE1 | PHE | A | 257 | 15.522 | 49.807 | 57.616 | 1.00 | 15.35 | A | C |

| | | | | | | | | | | | | |
|---|---|---|---|---|---|---|---|---|---|---|---|---|
| ATOM | 1696 | CE2 | PHE | A | 257 | 16.328 | 47.780 | 56.620 | 1.00 | 15.35 | A | C |
| ATOM | 1697 | CZ | PHE | A | 257 | 15.477 | 48.878 | 56.595 | 1.00 | 15.35 | A | C |
| ATOM | 1698 | C | PHE | A | 257 | 20.047 | 49.186 | 61.306 | 1.00 | 24.26 | A | C |
| ATOM | 1699 | O | PHE | A | 257 | 20.075 | 48.052 | 61.774 | 1.00 | 24.26 | A | O |
| ATOM | 1700 | N | PRO | A | 258 | 20.789 | 50.170 | 61.822 | 1.00 | 21.64 | A | N |
| ATOM | 1701 | CD | PRO | A | 258 | 20.787 | 51.588 | 61.414 | 1.00 | 26.29 | A | C |
| ATOM | 1702 | CA | PRO | A | 258 | 21.701 | 49.947 | 62.948 | 1.00 | 21.64 | A | C |
| ATOM | 1703 | CB | PRO | A | 258 | 22.595 | 51.174 | 62.897 | 1.00 | 26.29 | A | C |
| ATOM | 1704 | CG | PRO | A | 258 | 21.622 | 52.250 | 62.500 | 1.00 | 26.29 | A | C |
| ATOM | 1705 | C | PRO | A | 258 | 21.015 | 49.766 | 64.302 | 1.00 | 21.64 | A | C |
| ATOM | 1706 | O | PRO | A | 258 | 19.801 | 49.888 | 64.417 | 1.00 | 21.64 | A | O |
| ATOM | 1707 | N | GLY | A | 259 | 21.807 | 49.474 | 65.322 | 1.00 | 17.78 | A | N |
| ATOM | 1708 | CA | GLY | A | 259 | 21.254 | 49.273 | 66.648 | 1.00 | 17.78 | A | C |
| ATOM | 1709 | C | GLY | A | 259 | 21.923 | 48.118 | 67.366 | 1.00 | 17.78 | A | C |
| ATOM | 1710 | O | GLY | A | 259 | 22.204 | 47.080 | 66.759 | 1.00 | 17.78 | A | O |
| ATOM | 1711 | N | ASP | A | 260 | 22.197 | 48.282 | 68.655 | 1.00 | 29.38 | A | N |
| ATOM | 1712 | CA | ASP | A | 260 | 22.835 | 47.201 | 69.392 | 1.00 | 29.38 | A | C |
| ATOM | 1713 | CB | ASP | A | 260 | 23.787 | 47.759 | 70.460 | 1.00 | 60.06 | A | C |
| ATOM | 1714 | CG | ASP | A | 260 | 25.049 | 48.368 | 69.851 | 1.00 | 60.06 | A | C |
| ATOM | 1715 | OD1 | ASP | A | 260 | 25.214 | 48.271 | 68.613 | 1.00 | 60.06 | A | O |
| ATOM | 1716 | OD2 | ASP | A | 260 | 25.879 | 48.939 | 70.604 | 1.00 | 60.06 | A | O |
| ATOM | 1717 | C | ASP | A | 260 | 21.818 | 46.268 | 70.026 | 1.00 | 29.38 | A | C |
| ATOM | 1718 | O | ASP | A | 260 | 22.173 | 45.316 | 70.707 | 1.00 | 29.38 | A | O |
| ATOM | 1719 | N | SER | A | 261 | 20.545 | 46.538 | 69.792 | 1.00 | 25.76 | A | N |
| ATOM | 1720 | CA | SER | A | 261 | 19.504 | 45.696 | 70.342 | 1.00 | 25.76 | A | C |
| ATOM | 1721 | CB | SER | A | 261 | 18.829 | 46.375 | 71.533 | 1.00 | 22.94 | A | C |
| ATOM | 1722 | OG | SER | A | 261 | 17.897 | 47.344 | 71.092 | 1.00 | 22.94 | A | O |
| ATOM | 1723 | C | SER | A | 261 | 18.477 | 45.462 | 69.252 | 1.00 | 25.76 | A | C |
| ATOM | 1724 | O | SER | A | 261 | 18.369 | 46.244 | 68.315 | 1.00 | 25.76 | A | O |
| ATOM | 1725 | N | GLY | A | 262 | 17.731 | 44.374 | 69.378 | 1.00 | 27.23 | A | N |
| ATOM | 1726 | CA | GLY | A | 262 | 16.702 | 44.086 | 68.405 | 1.00 | 27.23 | A | C |
| ATOM | 1727 | C | GLY | A | 262 | 15.694 | 45.210 | 68.464 | 1.00 | 27.23 | A | C |
| ATOM | 1728 | O | GLY | A | 262 | 15.196 | 45.664 | 67.447 | 1.00 | 27.23 | A | O |
| ATOM | 1729 | N | VAL | A | 263 | 15.390 | 45.670 | 69.665 | 1.00 | 26.78 | A | N |
| ATOM | 1730 | CA | VAL | A | 263 | 14.452 | 46.759 | 69.799 | 1.00 | 26.78 | A | C |
| ATOM | 1731 | CB | VAL | A | 263 | 14.223 | 47.119 | 71.290 | 1.00 | 33.29 | A | C |
| ATOM | 1732 | CG1 | VAL | A | 263 | 13.568 | 48.480 | 71.416 | 1.00 | 33.29 | A | C |
| ATOM | 1733 | CG2 | VAL | A | 263 | 13.330 | 46.082 | 71.925 | 1.00 | 33.29 | A | C |
| ATOM | 1734 | C | VAL | A | 263 | 14.956 | 47.982 | 69.040 | 1.00 | 26.78 | A | C |
| ATOM | 1735 | O | VAL | A | 263 | 14.209 | 48.620 | 68.304 | 1.00 | 26.78 | A | O |
| ATOM | 1736 | N | ASP | A | 264 | 16.225 | 48.320 | 69.209 | 1.00 | 27.87 | A | N |
| ATOM | 1737 | CA | ASP | A | 264 | 16.726 | 49.474 | 68.496 | 1.00 | 27.87 | A | C |
| ATOM | 1738 | CB | ASP | A | 264 | 18.124 | 49.850 | 68.999 | 1.00 | 26.70 | A | C |
| ATOM | 1739 | CG | ASP | A | 264 | 18.066 | 50.551 | 70.347 | 1.00 | 26.70 | A | C |
| ATOM | 1740 | OD1 | ASP | A | 264 | 19.041 | 51.227 | 70.751 | 1.00 | 26.70 | A | O |
| ATOM | 1741 | OD2 | ASP | A | 264 | 17.012 | 50.410 | 71.002 | 1.00 | 26.70 | A | O |
| ATOM | 1742 | C | ASP | A | 264 | 16.688 | 49.245 | 66.988 | 1.00 | 27.87 | A | C |
| ATOM | 1743 | O | ASP | A | 264 | 16.309 | 50.141 | 66.231 | 1.00 | 27.87 | A | O |
| ATOM | 1744 | N | GLN | A | 265 | 17.045 | 48.037 | 66.564 | 1.00 | 27.08 | A | N |
| ATOM | 1745 | CA | GLN | A | 265 | 17.046 | 47.692 | 65.145 | 1.00 | 27.08 | A | C |
| ATOM | 1746 | CB | GLN | A | 265 | 17.551 | 46.262 | 64.946 | 1.00 | 30.70 | A | C |
| ATOM | 1747 | CG | GLN | A | 265 | 19.038 | 46.132 | 64.815 | 1.00 | 30.70 | A | C |
| ATOM | 1748 | CD | GLN | A | 265 | 19.547 | 44.806 | 65.349 | 1.00 | 30.70 | A | C |
| ATOM | 1749 | OE1 | GLN | A | 265 | 18.908 | 43.764 | 65.182 | 1.00 | 30.70 | A | O |
| ATOM | 1750 | NE2 | GLN | A | 265 | 20.710 | 44.836 | 65.987 | 1.00 | 30.70 | A | N |
| ATOM | 1751 | C | GLN | A | 265 | 15.632 | 47.812 | 64.588 | 1.00 | 27.08 | A | C |
| ATOM | 1752 | O | GLN | A | 265 | 15.414 | 48.373 | 63.517 | 1.00 | 27.08 | A | O |
| ATOM | 1753 | N | LEU | A | 266 | 14.671 | 47.279 | 65.325 | 1.00 | 20.92 | A | N |
| ATOM | 1754 | CA | LEU | A | 266 | 13.285 | 47.337 | 64.906 | 1.00 | 20.92 | A | C |
| ATOM | 1755 | CB | LEU | A | 266 | 12.412 | 46.584 | 65.898 | 1.00 | 21.87 | A | C |
| ATOM | 1756 | CG | LEU | A | 266 | 11.795 | 45.304 | 65.368 | 1.00 | 21.87 | A | C |
| ATOM | 1757 | CD1 | LEU | A | 266 | 12.796 | 44.547 | 64.541 | 1.00 | 21.87 | A | C |
| ATOM | 1758 | CD2 | LEU | A | 266 | 11.329 | 44.486 | 66.537 | 1.00 | 21.87 | A | C |

| | | | | | | | | | | | | |
|---|---|---|---|---|---|---|---|---|---|---|---|---|
| ATOM | 1759 | C | LEU | A | 266 | 12.745 | 48.762 | 64.739 | 1.00 | 20.92 | A | C |
| ATOM | 1760 | O | LEU | A | 266 | 11.898 | 49.010 | 63.893 | 1.00 | 20.92 | A | O |
| ATOM | 1761 | N | VAL | A | 267 | 13.216 | 49.696 | 65.554 | 1.00 | 23.75 | A | N |
| ATOM | 1762 | CA | VAL | A | 267 | 12.757 | 51.072 | 65.439 | 1.00 | 23.75 | A | C |
| ATOM | 1763 | CB | VAL | A | 267 | 13.313 | 51.950 | 66.575 | 1.00 | 33.02 | A | C |
| ATOM | 1764 | CG1 | VAL | A | 267 | 13.234 | 53.417 | 66.184 | 1.00 | 33.02 | A | C |
| ATOM | 1765 | CG2 | VAL | A | 267 | 12.534 | 51.703 | 67.855 | 1.00 | 33.02 | A | C |
| ATOM | 1766 | C | VAL | A | 267 | 13.257 | 51.642 | 64.116 | 1.00 | 23.75 | A | C |
| ATOM | 1767 | O | VAL | A | 267 | 12.508 | 52.258 | 63.360 | 1.00 | 23.75 | A | O |
| ATOM | 1768 | N | GLU | A | 268 | 14.543 | 51.436 | 63.860 | 1.00 | 27.05 | A | N |
| ATOM | 1769 | CA | GLU | A | 268 | 15.182 | 51.899 | 62.645 | 1.00 | 27.05 | A | C |
| ATOM | 1770 | CB | GLU | A | 268 | 16.640 | 51.447 | 62.639 | 1.00 | 50.90 | A | C |
| ATOM | 1771 | CG | GLU | A | 268 | 17.680 | 52.550 | 62.811 | 1.00 | 50.90 | A | C |
| ATOM | 1772 | CD | GLU | A | 268 | 17.217 | 53.675 | 63.725 | 1.00 | 50.90 | A | C |
| ATOM | 1773 | OE1 | GLU | A | 268 | 16.391 | 54.494 | 63.256 | 1.00 | 50.90 | A | O |
| ATOM | 1774 | OE2 | GLU | A | 268 | 17.672 | 53.739 | 64.901 | 1.00 | 50.90 | A | O |
| ATOM | 1775 | C | GLU | A | 268 | 14.468 | 51.352 | 61.405 | 1.00 | 27.05 | A | C |
| ATOM | 1776 | O | GLU | A | 268 | 14.274 | 52.072 | 60.427 | 1.00 | 27.05 | A | O |
| ATOM | 1777 | N | ILE | A | 269 | 14.077 | 50.078 | 61.441 | 1.00 | 28.63 | A | N |
| ATOM | 1778 | CA | ILE | A | 269 | 13.393 | 49.466 | 60.303 | 1.00 | 28.63 | A | C |
| ATOM | 1779 | CB | ILE | A | 269 | 13.173 | 47.939 | 60.491 | 1.00 | 19.00 | A | C |
| ATOM | 1780 | CG2 | ILE | A | 269 | 12.276 | 47.395 | 59.375 | 1.00 | 19.00 | A | C |
| ATOM | 1781 | CG1 | ILE | A | 269 | 14.513 | 47.208 | 60.507 | 1.00 | 19.00 | A | C |
| ATOM | 1782 | CD1 | ILE | A | 269 | 14.386 | 45.763 | 60.852 | 1.00 | 19.00 | A | C |
| ATOM | 1783 | C | ILE | A | 269 | 12.033 | 50.096 | 60.104 | 1.00 | 28.63 | A | C |
| ATOM | 1784 | O | ILE | A | 269 | 11.671 | 50.445 | 58.987 | 1.00 | 28.63 | A | O |
| ATOM | 1785 | N | ILE | A | 270 | 11.277 | 50.219 | 61.195 | 1.00 | 40.36 | A | N |
| ATOM | 1786 | CA | ILE | A | 270 | 9.945 | 50.816 | 61.154 | 1.00 | 40.36 | A | C |
| ATOM | 1787 | CB | ILE | A | 270 | 9.277 | 50.791 | 62.527 | 1.00 | 25.65 | A | C |
| ATOM | 1788 | CG2 | ILE | A | 270 | 8.029 | 51.638 | 62.507 | 1.00 | 25.65 | A | C |
| ATOM | 1789 | CG1 | ILE | A | 270 | 8.917 | 49.356 | 62.898 | 1.00 | 25.65 | A | C |
| ATOM | 1790 | CD1 | ILE | A | 270 | 8.307 | 49.222 | 64.280 | 1.00 | 25.65 | A | C |
| ATOM | 1791 | C | ILE | A | 270 | 10.009 | 52.261 | 60.660 | 1.00 | 40.36 | A | C |
| ATOM | 1792 | O | ILE | A | 270 | 9.115 | 52.716 | 59.940 | 1.00 | 40.36 | A | O |
| ATOM | 1793 | N | LYS | A | 271 | 11.066 | 52.977 | 61.050 | 1.00 | 33.44 | A | N |
| ATOM | 1794 | CA | LYS | A | 271 | 11.250 | 54.355 | 60.618 | 1.00 | 33.44 | A | C |
| ATOM | 1795 | CB | LYS | A | 271 | 12.659 | 54.869 | 60.951 | 1.00 | 43.40 | A | C |
| ATOM | 1796 | CG | LYS | A | 271 | 12.817 | 55.531 | 62.297 | 1.00 | 43.40 | A | C |
| ATOM | 1797 | CD | LYS | A | 271 | 14.157 | 56.269 | 62.394 | 1.00 | 43.40 | A | C |
| ATOM | 1798 | CE | LYS | A | 271 | 14.348 | 56.917 | 63.771 | 1.00 | 43.40 | A | C |
| ATOM | 1799 | NZ | LYS | A | 271 | 15.555 | 57.810 | 63.828 | 1.00 | 43.40 | A | N |
| ATOM | 1800 | C | LYS | A | 271 | 11.086 | 54.432 | 59.106 | 1.00 | 33.44 | A | C |
| ATOM | 1801 | O | LYS | A | 271 | 10.432 | 55.329 | 58.582 | 1.00 | 33.44 | A | O |
| ATOM | 1802 | N | VAL | A | 272 | 11.675 | 53.479 | 58.400 | 1.00 | 28.34 | A | N |
| ATOM | 1803 | CA | VAL | A | 272 | 11.607 | 53.533 | 56.958 | 1.00 | 28.34 | A | C |
| ATOM | 1804 | CB | VAL | A | 272 | 13.018 | 53.392 | 56.335 | 1.00 | 33.65 | A | C |
| ATOM | 1805 | CG1 | VAL | A | 272 | 14.074 | 53.908 | 57.299 | 1.00 | 33.65 | A | C |
| ATOM | 1806 | CG2 | VAL | A | 272 | 13.276 | 51.957 | 55.958 | 1.00 | 33.65 | A | C |
| ATOM | 1807 | C | VAL | A | 272 | 10.678 | 52.574 | 56.230 | 1.00 | 28.34 | A | C |
| ATOM | 1808 | O | VAL | A | 272 | 10.624 | 52.616 | 55.016 | 1.00 | 28.34 | A | O |
| ATOM | 1809 | N | LEU | A | 273 | 9.969 | 51.702 | 56.932 | 1.00 | 29.75 | A | N |
| ATOM | 1810 | CA | LEU | A | 273 | 9.053 | 50.783 | 56.255 | 1.00 | 29.75 | A | C |
| ATOM | 1811 | CB | LEU | A | 273 | 9.384 | 49.307 | 56.525 | 1.00 | 12.18 | A | C |
| ATOM | 1812 | CG | LEU | A | 273 | 10.705 | 48.622 | 56.196 | 1.00 | 12.18 | A | C |
| ATOM | 1813 | CD1 | LEU | A | 273 | 10.419 | 47.149 | 55.973 | 1.00 | 12.18 | A | C |
| ATOM | 1814 | CD2 | LEU | A | 273 | 11.342 | 49.219 | 54.975 | 1.00 | 12.18 | A | C |
| ATOM | 1815 | C | LEU | A | 273 | 7.656 | 51.024 | 56.770 | 1.00 | 29.75 | A | C |
| ATOM | 1816 | O | LEU | A | 273 | 6.708 | 50.383 | 56.330 | 1.00 | 29.75 | A | O |
| ATOM | 1817 | N | GLY | A | 274 | 7.532 | 51.937 | 57.725 | 1.00 | 42.91 | A | N |
| ATOM | 1818 | CA | GLY | A | 274 | 6.235 | 52.207 | 58.311 | 1.00 | 42.91 | A | C |
| ATOM | 1819 | C | GLY | A | 274 | 5.897 | 51.109 | 59.307 | 1.00 | 42.91 | A | C |
| ATOM | 1820 | O | GLY | A | 274 | 6.561 | 50.066 | 59.361 | 1.00 | 42.91 | A | O |
| ATOM | 1821 | N | THR | A | 275 | 4.870 | 51.349 | 60.111 | 1.00 | 42.54 | A | N |

| | | | | | | | | | | | | |
|---|---|---|---|---|---|---|---|---|---|---|---|---|
| ATOM | 1822 | CA | THR | A | 275 | 4.429 | 50.376 | 61.102 | 1.00 | 42.54 | A | C |
| ATOM | 1823 | CB | THR | A | 275 | 3.333 | 50.980 | 62.030 | 1.00 | 43.47 | A | C |
| ATOM | 1824 | OG1 | THR | A | 275 | 3.913 | 51.981 | 62.878 | 1.00 | 43.47 | A | O |
| ATOM | 1825 | CG2 | THR | A | 275 | 2.684 | 49.896 | 62.882 | 1.00 | 43.47 | A | C |
| ATOM | 1826 | C | THR | A | 275 | 3.835 | 49.168 | 60.370 | 1.00 | 42.54 | A | C |
| ATOM | 1827 | O | THR | A | 275 | 2.917 | 49.314 | 59.551 | 1.00 | 42.54 | A | O |
| ATOM | 1828 | N | PRO | A | 276 | 4.356 | 47.959 | 60.652 | 1.00 | 47.64 | A | N |
| ATOM | 1829 | CD | PRO | A | 276 | 5.425 | 47.590 | 61.595 | 1.00 | 34.03 | A | C |
| ATOM | 1830 | CA | PRO | A | 276 | 3.830 | 46.764 | 59.992 | 1.00 | 47.64 | A | C |
| ATOM | 1831 | CB | PRO | A | 276 | 4.701 | 45.639 | 60.548 | 1.00 | 34.03 | A | C |
| ATOM | 1832 | CG | PRO | A | 276 | 5.091 | 46.140 | 61.888 | 1.00 | 34.03 | A | C |
| ATOM | 1833 | C | PRO | A | 276 | 2.369 | 46.544 | 60.287 | 1.00 | 47.64 | A | C |
| ATOM | 1834 | O | PRO | A | 276 | 1.923 | 46.724 | 61.422 | 1.00 | 47.64 | A | O |
| ATOM | 1835 | N | THR | A | 277 | 1.629 | 46.157 | 59.254 | 1.00 | 64.64 | A | N |
| ATOM | 1836 | CA | THR | A | 277 | 0.210 | 45.889 | 59.392 | 1.00 | 64.64 | A | C |
| ATOM | 1837 | CB | THR | A | 277 | -0.426 | 45.645 | 58.027 | 1.00 | 35.35 | A | C |
| ATOM | 1838 | OG1 | THR | A | 277 | 0.065 | 44.410 | 57.490 | 1.00 | 35.35 | A | O |
| ATOM | 1839 | CG2 | THR | A | 277 | -0.076 | 46.781 | 57.081 | 1.00 | 35.35 | A | C |
| ATOM | 1840 | C | THR | A | 277 | 0.115 | 44.628 | 60.236 | 1.00 | 64.64 | A | C |
| ATOM | 1841 | O | THR | A | 277 | 1.125 | 44.178 | 60.815 | 1.00 | 64.64 | A | O |
| ATOM | 1842 | N | ARG | A | 278 | -1.084 | 44.055 | 60.329 | 1.00 | 63.77 | A | N |
| ATOM | 1843 | CA | ARG | A | 278 | -1.233 | 42.829 | 61.101 | 1.00 | 63.77 | A | C |
| ATOM | 1844 | CB | ARG | A | 278 | -2.649 | 42.297 | 60.968 | 1.00 | 95.57 | A | C |
| ATOM | 1845 | CG | ARG | A | 278 | -2.943 | 41.189 | 61.944 | 1.00 | 95.57 | A | C |
| ATOM | 1846 | CD | ARG | A | 278 | -2.724 | 41.636 | 63.386 | 1.00 | 95.57 | A | C |
| ATOM | 1847 | NE | ARG | A | 278 | -3.959 | 41.491 | 64.156 | 1.00 | 95.57 | A | N |
| ATOM | 1848 | CZ | ARG | A | 278 | -5.069 | 42.203 | 63.936 | 1.00 | 95.57 | A | C |
| ATOM | 1849 | NH1 | ARG | A | 278 | -5.097 | 43.125 | 62.968 | 1.00 | 95.57 | A | N |
| ATOM | 1850 | NH2 | ARG | A | 278 | -6.161 | 41.981 | 64.671 | 1.00 | 95.57 | A | N |
| ATOM | 1851 | C | ARG | A | 278 | -0.222 | 41.811 | 60.546 | 1.00 | 63.77 | A | C |
| ATOM | 1852 | O | ARG | A | 278 | 0.081 | 40.802 | 61.203 | 1.00 | 63.77 | A | O |
| ATOM | 1853 | N | GLU | A | 279 | 0.287 | 42.122 | 59.340 | 1.00 | 54.18 | A | N |
| ATOM | 1854 | CA | GLU | A | 279 | 1.292 | 41.346 | 58.601 | 1.00 | 54.18 | A | C |
| ATOM | 1855 | CB | GLU | A | 279 | 1.870 | 42.202 | 57.464 | 1.00 | 37.58 | A | C |
| ATOM | 1856 | CG | GLU | A | 279 | 2.654 | 43.466 | 57.903 | 1.00 | 37.58 | A | C |
| ATOM | 1857 | CD | GLU | A | 279 | 3.002 | 44.404 | 56.720 | 1.00 | 37.58 | A | C |
| ATOM | 1858 | OE1 | GLU | A | 279 | 3.446 | 43.910 | 55.656 | 1.00 | 37.58 | A | O |
| ATOM | 1859 | OE2 | GLU | A | 279 | 2.844 | 45.638 | 56.855 | 1.00 | 37.58 | A | O |
| ATOM | 1860 | C | GLU | A | 279 | 2.441 | 40.822 | 59.478 | 1.00 | 54.18 | A | C |
| ATOM | 1861 | O | GLU | A | 279 | 3.256 | 40.005 | 59.034 | 1.00 | 54.18 | A | O |
| ATOM | 1862 | N | ALA | A | 280 | 2.518 | 41.311 | 60.713 | 1.00 | 49.67 | A | N |
| ATOM | 1863 | CA | ALA | A | 280 | 3.532 | 40.840 | 61.640 | 1.00 | 49.67 | A | C |
| ATOM | 1864 | CB | ALA | A | 280 | 3.312 | 41.470 | 63.022 | 1.00 | 25.58 | A | C |
| ATOM | 1865 | C | ALA | A | 280 | 3.364 | 39.315 | 61.719 | 1.00 | 49.67 | A | C |
| ATOM | 1866 | O | ALA | A | 280 | 4.326 | 38.571 | 61.937 | 1.00 | 49.67 | A | O |
| ATOM | 1867 | N | ALA | A | 281 | 2.129 | 38.856 | 61.528 | 1.00 | 45.53 | A | N |
| ATOM | 1868 | CA | ALA | A | 281 | 1.816 | 37.430 | 61.582 | 1.00 | 45.53 | A | C |
| ATOM | 1869 | CB | ALA | A | 281 | 0.350 | 37.206 | 61.223 | 1.00 | 26.83 | A | C |
| ATOM | 1870 | C | ALA | A | 281 | 2.716 | 36.578 | 60.679 | 1.00 | 45.53 | A | C |
| ATOM | 1871 | O | ALA | A | 281 | 3.119 | 35.470 | 61.048 | 1.00 | 45.53 | A | O |
| ATOM | 1872 | N | ARG | A | 282 | 3.034 | 37.088 | 59.496 | 1.00 | 64.41 | A | N |
| ATOM | 1873 | CA | ARG | A | 282 | 3.884 | 36.334 | 58.584 | 1.00 | 64.41 | A | C |
| ATOM | 1874 | CB | ARG | A | 282 | 3.721 | 36.851 | 57.151 | 1.00 | 67.90 | A | C |
| ATOM | 1875 | CG | ARG | A | 282 | 2.366 | 36.476 | 56.555 | 1.00 | 67.90 | A | C |
| ATOM | 1876 | CD | ARG | A | 282 | 2.089 | 37.151 | 55.224 | 1.00 | 67.90 | A | C |
| ATOM | 1877 | NE | ARG | A | 282 | 2.112 | 38.620 | 55.294 | 1.00 | 67.90 | A | N |
| ATOM | 1878 | CZ | ARG | A | 282 | 1.264 | 39.418 | 54.631 | 1.00 | 67.90 | A | C |
| ATOM | 1879 | NH1 | ARG | A | 282 | 0.312 | 38.882 | 53.860 | 1.00 | 67.90 | A | N |
| ATOM | 1880 | NH2 | ARG | A | 282 | 1.389 | 40.748 | 54.698 | 1.00 | 67.90 | A | N |
| ATOM | 1881 | C | ARG | A | 282 | 5.355 | 36.339 | 58.985 | 1.00 | 64.41 | A | C |
| ATOM | 1882 | O | ARG | A | 282 | 6.207 | 35.919 | 58.204 | 1.00 | 64.41 | A | O |
| ATOM | 1883 | N | GLU | A | 283 | 5.652 | 36.792 | 60.206 | 1.00 | 66.41 | A | N |
| ATOM | 1884 | CA | GLU | A | 283 | 7.031 | 36.821 | 60.685 | 1.00 | 66.41 | A | C |

| | | | | | | | | | | | | |
|---|---|---|---|---|---|---|---|---|---|---|---|---|
| ATOM | 1885 | CB | GLU | A | 283 | 7.559 | 38.259 | 60.784 | 1.00 | 37.54 | A | C |
| ATOM | 1886 | CG | GLU | A | 283 | 7.072 | 39.256 | 59.741 | 1.00 | 37.54 | A | C |
| ATOM | 1887 | CD | GLU | A | 283 | 7.776 | 40.612 | 59.882 | 1.00 | 37.54 | A | C |
| ATOM | 1888 | OE1 | GLU | A | 283 | 8.936 | 40.710 | 59.450 | 1.00 | 37.54 | A | O |
| ATOM | 1889 | OE2 | GLU | A | 283 | 7.189 | 41.572 | 60.436 | 1.00 | 37.54 | A | O |
| ATOM | 1890 | C | GLU | A | 283 | 7.163 | 36.193 | 62.074 | 1.00 | 66.41 | A | C |
| ATOM | 1891 | O | GLU | A | 283 | 7.676 | 35.074 | 62.231 | 1.00 | 66.41 | A | O |
| ATOM | 1892 | N | ALA | A | 284 | 6.678 | 36.938 | 63.069 | 1.00 | 56.51 | A | N |
| ATOM | 1893 | CA | ALA | A | 284 | 6.740 | 36.601 | 64.503 | 1.00 | 56.51 | A | C |
| ATOM | 1894 | CB | ALA | A | 284 | 5.764 | 37.507 | 65.270 | 1.00 | 25.98 | A | C |
| ATOM | 1895 | C | ALA | A | 284 | 6.596 | 35.173 | 65.037 | 1.00 | 56.51 | A | C |
| ATOM | 1896 | O | ALA | A | 284 | 7.582 | 34.517 | 65.398 | 1.00 | 56.51 | A | O |
| ATOM | 1897 | N | GLY | A | 285 | 5.355 | 34.719 | 65.122 | 1.00 | 66.12 | A | N |
| ATOM | 1898 | CA | GLY | A | 285 | 5.076 | 33.410 | 65.687 | 1.00 | 66.12 | A | C |
| ATOM | 1899 | C | GLY | A | 285 | 4.065 | 33.722 | 66.780 | 1.00 | 66.12 | A | C |
| ATOM | 1900 | O | GLY | A | 285 | 3.053 | 33.041 | 66.929 | 1.00 | 66.12 | A | O |
| ATOM | 1901 | N | GLY | A | 286 | 4.348 | 34.782 | 67.533 | 1.00 | 48.45 | A | N |
| ATOM | 1902 | CA | GLY | A | 286 | 3.459 | 35.246 | 68.583 | 1.00 | 48.45 | A | C |
| ATOM | 1903 | C | GLY | A | 286 | 3.235 | 36.719 | 68.261 | 1.00 | 48.45 | A | C |
| ATOM | 1904 | O | GLY | A | 286 | 4.211 | 37.412 | 67.958 | 1.00 | 48.45 | A | O |
| ATOM | 1905 | N | GLY | A | 287 | 1.985 | 37.199 | 68.307 | 1.00 | 84.78 | A | N |
| ATOM | 1906 | CA | GLY | A | 287 | 1.684 | 38.593 | 67.981 | 1.00 | 84.78 | A | C |
| ATOM | 1907 | C | GLY | A | 287 | 1.533 | 39.533 | 69.173 | 1.00 | 84.78 | A | C |
| ATOM | 1908 | O | GLY | A | 287 | 0.485 | 39.590 | 69.837 | 1.00 | 84.78 | A | O |
| ATOM | 1909 | N | TRP | A | 301 | 11.237 | 56.187 | 52.176 | 1.00 | 41.92 | A | N |
| ATOM | 1910 | CA | TRP | A | 301 | 12.210 | 55.612 | 51.243 | 1.00 | 41.92 | A | C |
| ATOM | 1911 | CB | TRP | A | 301 | 11.539 | 54.561 | 50.337 | 1.00 | 39.24 | A | C |
| ATOM | 1912 | CG | TRP | A | 301 | 11.679 | 53.121 | 50.831 | 1.00 | 39.24 | A | C |
| ATOM | 1913 | CD2 | TRP | A | 301 | 12.903 | 52.443 | 51.200 | 1.00 | 39.24 | A | C |
| ATOM | 1914 | CE2 | TRP | A | 301 | 12.543 | 51.130 | 51.607 | 1.00 | 39.24 | A | C |
| ATOM | 1915 | CE3 | TRP | A | 301 | 14.244 | 52.840 | 51.284 | 1.00 | 39.24 | A | C |
| ATOM | 1916 | CD1 | TRP | A | 301 | 10.673 | 52.198 | 50.985 | 1.00 | 39.24 | A | C |
| ATOM | 1917 | NE1 | TRP | A | 301 | 11.197 | 50.996 | 51.444 | 1.00 | 39.24 | A | N |
| ATOM | 1918 | CZ2 | TRP | A | 301 | 13.505 | 50.193 | 52.017 | 1.00 | 39.24 | A | C |
| ATOM | 1919 | CZ3 | TRP | A | 301 | 15.194 | 51.899 | 51.702 | 1.00 | 39.24 | A | C |
| ATOM | 1920 | CH2 | TRP | A | 301 | 14.813 | 50.605 | 52.089 | 1.00 | 39.24 | A | C |
| ATOM | 1921 | C | TRP | A | 301 | 12.869 | 56.684 | 50.384 | 1.00 | 41.92 | A | C |
| ATOM | 1922 | O | TRP | A | 301 | 14.058 | 56.620 | 50.079 | 1.00 | 41.92 | A | O |
| ATOM | 1923 | N | THR | A | 302 | 12.092 | 57.683 | 50.004 | 1.00 | 59.13 | A | N |
| ATOM | 1924 | CA | THR | A | 302 | 12.613 | 58.755 | 49.174 | 1.00 | 59.13 | A | C |
| ATOM | 1925 | CB | THR | A | 302 | 11.487 | 59.407 | 48.416 | 1.00 | 65.93 | A | C |
| ATOM | 1926 | OG1 | THR | A | 302 | 10.559 | 59.936 | 49.367 | 1.00 | 65.93 | A | O |
| ATOM | 1927 | CG2 | THR | A | 302 | 10.758 | 58.374 | 47.536 | 1.00 | 65.93 | A | C |
| ATOM | 1928 | C | THR | A | 302 | 13.332 | 59.828 | 49.984 | 1.00 | 59.13 | A | C |
| ATOM | 1929 | O | THR | A | 302 | 13.959 | 60.715 | 49.418 | 1.00 | 59.13 | A | O |
| ATOM | 1930 | N | ALA | A | 303 | 13.223 | 59.768 | 51.304 | 1.00 | 43.27 | A | N |
| ATOM | 1931 | CA | ALA | A | 303 | 13.909 | 60.742 | 52.149 | 1.00 | 43.27 | A | C |
| ATOM | 1932 | CB | ALA | A | 303 | 13.030 | 61.114 | 53.353 | 1.00 | 30.80 | A | C |
| ATOM | 1933 | C | ALA | A | 303 | 15.212 | 60.094 | 52.625 | 1.00 | 43.27 | A | C |
| ATOM | 1934 | O | ALA | A | 303 | 16.125 | 60.772 | 53.112 | 1.00 | 43.27 | A | O |
| ATOM | 1935 | N | VAL | A | 304 | 15.293 | 58.775 | 52.455 | 1.00 | 23.52 | A | N |
| ATOM | 1936 | CA | VAL | A | 304 | 16.448 | 58.013 | 52.900 | 1.00 | 23.52 | A | C |
| ATOM | 1937 | CB | VAL | A | 304 | 16.178 | 56.487 | 52.826 | 1.00 | 33.44 | A | C |
| ATOM | 1938 | CG1 | VAL | A | 304 | 17.446 | 55.709 | 53.194 | 1.00 | 33.44 | A | C |
| ATOM | 1939 | CG2 | VAL | A | 304 | 15.036 | 56.115 | 53.763 | 1.00 | 33.44 | A | C |
| ATOM | 1940 | C | VAL | A | 304 | 17.768 | 58.274 | 52.186 | 1.00 | 23.52 | A | C |
| ATOM | 1941 | O | VAL | A | 304 | 18.774 | 58.571 | 52.825 | 1.00 | 23.52 | A | O |
| ATOM | 1942 | N | PHE | A | 305 | 17.768 | 58.166 | 50.861 | 1.00 | 28.00 | A | N |
| ATOM | 1943 | CA | PHE | A | 305 | 18.997 | 58.335 | 50.093 | 1.00 | 28.00 | A | C |
| ATOM | 1944 | CB | PHE | A | 305 | 18.967 | 57.393 | 48.900 | 1.00 | 29.64 | A | C |
| ATOM | 1945 | CG | PHE | A | 305 | 18.858 | 55.952 | 49.287 | 1.00 | 29.64 | A | C |
| ATOM | 1946 | CD1 | PHE | A | 305 | 19.938 | 55.295 | 49.877 | 1.00 | 29.64 | A | C |
| ATOM | 1947 | CD2 | PHE | A | 305 | 17.664 | 55.255 | 49.101 | 1.00 | 29.64 | A | C |

| | | | | | | | | | | | | |
|---|---|---|---|---|---|---|---|---|---|---|---|---|
| ATOM | 1948 | CE1 | PHE | A | 305 | 19.832 | 53.961 | 50.267 | 1.00 | 29.64 | A | C |
| ATOM | 1949 | CE2 | PHE | A | 305 | 17.549 | 53.925 | 49.486 | 1.00 | 29.64 | A | C |
| ATOM | 1950 | CZ | PHE | A | 305 | 18.632 | 53.274 | 50.075 | 1.00 | 29.64 | A | C |
| ATOM | 1951 | C | PHE | A | 305 | 19.376 | 59.719 | 49.625 | 1.00 | 28.00 | A | C |
| ATOM | 1952 | O | PHE | A | 305 | 18.518 | 60.559 | 49.372 | 1.00 | 28.00 | A | O |
| ATOM | 1953 | N | ARG | A | 306 | 20.687 | 59.929 | 49.496 | 1.00 | 52.15 | A | N |
| ATOM | 1954 | CA | ARG | A | 306 | 21.255 | 61.209 | 49.070 | 1.00 | 52.15 | A | C |
| ATOM | 1955 | CB | ARG | A | 306 | 22.792 | 61.129 | 49.094 | 1.00 | 70.11 | A | C |
| ATOM | 1956 | CG | ARG | A | 306 | 23.453 | 60.474 | 47.873 | 1.00 | 70.11 | A | C |
| ATOM | 1957 | CD | ARG | A | 306 | 24.971 | 60.320 | 48.086 | 1.00 | 70.11 | A | C |
| ATOM | 1958 | NE | ARG | A | 306 | 25.726 | 60.114 | 46.845 | 1.00 | 70.11 | A | N |
| ATOM | 1959 | CZ | ARG | A | 306 | 25.646 | 59.033 | 46.064 | 1.00 | 70.11 | A | C |
| ATOM | 1960 | NH1 | ARG | A | 306 | 24.829 | 58.024 | 46.386 | 1.00 | 70.11 | A | N |
| ATOM | 1961 | NH2 | ARG | A | 306 | 26.389 | 58.957 | 44.958 | 1.00 | 70.11 | A | N |
| ATOM | 1962 | C | ARG | A | 306 | 20.756 | 61.628 | 47.687 | 1.00 | 52.15 | A | C |
| ATOM | 1963 | O | ARG | A | 306 | 20.417 | 60.787 | 46.855 | 1.00 | 52.15 | A | O |
| ATOM | 1964 | N | PRO | A | 307 | 20.719 | 62.944 | 47.423 | 1.00 | 67.61 | A | N |
| ATOM | 1965 | CD | PRO | A | 307 | 21.489 | 63.941 | 48.200 | 1.00 | 39.85 | A | C |
| ATOM | 1966 | CA | PRO | A | 307 | 20.262 | 63.528 | 46.157 | 1.00 | 67.61 | A | C |
| ATOM | 1967 | CB | PRO | A | 307 | 21.209 | 64.709 | 45.983 | 1.00 | 39.85 | A | C |
| ATOM | 1968 | CG | PRO | A | 307 | 21.325 | 65.215 | 47.389 | 1.00 | 39.85 | A | C |
| ATOM | 1969 | C | PRO | A | 307 | 20.204 | 62.653 | 44.898 | 1.00 | 67.61 | A | C |
| ATOM | 1970 | O | PRO | A | 307 | 19.227 | 61.925 | 44.684 | 1.00 | 67.61 | A | O |
| ATOM | 1971 | N | ALA | A | 308 | 21.239 | 62.730 | 44.065 | 1.00 | 35.09 | A | N |
| ATOM | 1972 | CA | ALA | A | 308 | 21.246 | 61.988 | 42.804 | 1.00 | 35.09 | A | C |
| ATOM | 1973 | CB | ALA | A | 308 | 22.414 | 62.481 | 41.934 | 1.00 | 58.83 | A | C |
| ATOM | 1974 | C | ALA | A | 308 | 21.236 | 60.449 | 42.865 | 1.00 | 35.09 | A | C |
| ATOM | 1975 | O | ALA | A | 308 | 21.704 | 59.783 | 41.932 | 1.00 | 35.09 | A | O |
| ATOM | 1976 | N | THR | A | 309 | 20.699 | 59.885 | 43.945 | 1.00 | 29.58 | A | N |
| ATOM | 1977 | CA | THR | A | 309 | 20.631 | 58.435 | 44.075 | 1.00 | 29.58 | A | C |
| ATOM | 1978 | CB | THR | A | 309 | 20.065 | 57.997 | 45.438 | 1.00 | 34.47 | A | C |
| ATOM | 1979 | OG1 | THR | A | 309 | 20.915 | 58.476 | 46.485 | 1.00 | 34.47 | A | O |
| ATOM | 1980 | CG2 | THR | A | 309 | 19.994 | 56.467 | 45.517 | 1.00 | 34.47 | A | C |
| ATOM | 1981 | C | THR | A | 309 | 19.702 | 57.928 | 42.990 | 1.00 | 29.58 | A | C |
| ATOM | 1982 | O | THR | A | 309 | 18.563 | 58.371 | 42.904 | 1.00 | 29.58 | A | O |
| ATOM | 1983 | N | PRO | A | 310 | 20.177 | 57.011 | 42.129 | 1.00 | 40.87 | A | N |
| ATOM | 1984 | CD | PRO | A | 310 | 21.566 | 56.637 | 41.825 | 1.00 | 21.05 | A | C |
| ATOM | 1985 | CA | PRO | A | 310 | 19.270 | 56.525 | 41.081 | 1.00 | 40.87 | A | C |
| ATOM | 1986 | CB | PRO | A | 310 | 20.147 | 55.577 | 40.238 | 1.00 | 21.05 | A | C |
| ATOM | 1987 | CG | PRO | A | 310 | 21.377 | 55.349 | 41.057 | 1.00 | 21.05 | A | C |
| ATOM | 1988 | C | PRO | A | 310 | 18.003 | 55.859 | 41.579 | 1.00 | 40.87 | A | C |
| ATOM | 1989 | O | PRO | A | 310 | 18.045 | 54.999 | 42.450 | 1.00 | 40.87 | A | O |
| ATOM | 1990 | N | PRO | A | 311 | 16.853 | 56.272 | 41.035 | 1.00 | 30.99 | A | N |
| ATOM | 1991 | CD | PRO | A | 311 | 16.730 | 57.382 | 40.075 | 1.00 | 28.33 | A | C |
| ATOM | 1992 | CA | PRO | A | 311 | 15.533 | 55.744 | 41.388 | 1.00 | 30.99 | A | C |
| ATOM | 1993 | CB | PRO | A | 311 | 14.613 | 56.432 | 40.388 | 1.00 | 28.33 | A | C |
| ATOM | 1994 | CG | PRO | A | 311 | 15.268 | 57.761 | 40.219 | 1.00 | 28.33 | A | C |
| ATOM | 1995 | C | PRO | A | 311 | 15.443 | 54.221 | 41.289 | 1.00 | 30.99 | A | C |
| ATOM | 1996 | O | PRO | A | 311 | 14.808 | 53.588 | 42.123 | 1.00 | 30.99 | A | O |
| ATOM | 1997 | N | GLU | A | 312 | 16.059 | 53.627 | 40.272 | 1.00 | 27.46 | A | N |
| ATOM | 1998 | CA | GLU | A | 312 | 16.009 | 52.173 | 40.133 | 1.00 | 27.46 | A | C |
| ATOM | 1999 | CB | GLU | A | 312 | 16.861 | 51.687 | 38.951 | 1.00 | 55.32 | A | C |
| ATOM | 2000 | CG | GLU | A | 312 | 16.373 | 52.102 | 37.587 | 1.00 | 55.32 | A | C |
| ATOM | 2001 | CD | GLU | A | 312 | 16.159 | 53.596 | 37.496 | 1.00 | 55.32 | A | C |
| ATOM | 2002 | OE1 | GLU | A | 312 | 17.036 | 54.359 | 37.982 | 1.00 | 55.32 | A | O |
| ATOM | 2003 | OE2 | GLU | A | 312 | 15.114 | 54.005 | 36.936 | 1.00 | 55.32 | A | O |
| ATOM | 2004 | C | GLU | A | 312 | 16.551 | 51.526 | 41.403 | 1.00 | 27.46 | A | C |
| ATOM | 2005 | O | GLU | A | 312 | 15.955 | 50.591 | 41.948 | 1.00 | 27.46 | A | O |
| ATOM | 2006 | N | ALA | A | 313 | 17.700 | 52.028 | 41.852 | 1.00 | 23.66 | A | N |
| ATOM | 2007 | CA | ALA | A | 313 | 18.371 | 51.534 | 43.048 | 1.00 | 23.66 | A | C |
| ATOM | 2008 | CB | ALA | A | 313 | 19.632 | 52.340 | 43.304 | 1.00 | 27.46 | A | C |
| ATOM | 2009 | C | ALA | A | 313 | 17.473 | 51.590 | 44.270 | 1.00 | 23.66 | A | C |
| ATOM | 2010 | O | ALA | A | 313 | 17.398 | 50.638 | 45.049 | 1.00 | 23.66 | A | O |

| | | | | | | | | | | | | |
|---|---|---|---|---|---|---|---|---|---|---|---|---|
| ATOM | 2011 | N | ILE | A | 314 | 16.791 | 52.713 | 44.437 | 1.00 | 27.49 | A | N |
| ATOM | 2012 | CA | ILE | A | 314 | 15.912 | 52.878 | 45.574 | 1.00 | 27.49 | A | C |
| ATOM | 2013 | CB | ILE | A | 314 | 15.557 | 54.379 | 45.755 | 1.00 | 65.11 | A | C |
| ATOM | 2014 | CG2 | ILE | A | 314 | 15.163 | 54.981 | 44.442 | 1.00 | 65.11 | A | C |
| ATOM | 2015 | CG1 | ILE | A | 314 | 14.456 | 54.549 | 46.801 | 1.00 | 65.11 | A | C |
| ATOM | 2016 | CD1 | ILE | A | 314 | 14.128 | 55.996 | 47.079 | 1.00 | 65.11 | A | C |
| ATOM | 2017 | C | ILE | A | 314 | 14.676 | 51.996 | 45.468 | 1.00 | 27.49 | A | C |
| ATOM | 2018 | O | ILE | A | 314 | 14.094 | 51.609 | 46.478 | 1.00 | 27.49 | A | O |
| ATOM | 2019 | N | ALA | A | 315 | 14.307 | 51.652 | 44.241 | 1.00 | 25.32 | A | N |
| ATOM | 2020 | CA | ALA | A | 315 | 13.153 | 50.792 | 43.990 | 1.00 | 25.32 | A | C |
| ATOM | 2021 | CB | ALA | A | 315 | 12.776 | 50.837 | 42.499 | 1.00 | 20.65 | A | C |
| ATOM | 2022 | C | ALA | A | 315 | 13.490 | 49.356 | 44.413 | 1.00 | 25.32 | A | C |
| ATOM | 2023 | O | ALA | A | 315 | 12.695 | 48.687 | 45.076 | 1.00 | 25.32 | A | O |
| ATOM | 2024 | N | LEU | A | 316 | 14.680 | 48.896 | 44.033 | 1.00 | 29.86 | A | N |
| ATOM | 2025 | CA | LEU | A | 316 | 15.121 | 47.542 | 44.353 | 1.00 | 29.86 | A | C |
| ATOM | 2026 | CB | LEU | A | 316 | 16.434 | 47.231 | 43.625 | 1.00 | 16.30 | A | C |
| ATOM | 2027 | CG | LEU | A | 316 | 17.021 | 45.844 | 43.892 | 1.00 | 16.30 | A | C |
| ATOM | 2028 | CD1 | LEU | A | 316 | 16.059 | 44.780 | 43.396 | 1.00 | 16.30 | A | C |
| ATOM | 2029 | CD2 | LEU | A | 316 | 18.372 | 45.707 | 43.213 | 1.00 | 16.30 | A | C |
| ATOM | 2030 | C | LEU | A | 316 | 15.281 | 47.333 | 45.864 | 1.00 | 29.86 | A | C |
| ATOM | 2031 | O | LEU | A | 316 | 15.140 | 46.225 | 46.364 | 1.00 | 29.86 | A | O |
| ATOM | 2032 | N | CYS | A | 317 | 15.573 | 48.402 | 46.591 | 1.00 | 32.62 | A | N |
| ATOM | 2033 | CA | CYS | A | 317 | 15.723 | 48.303 | 48.035 | 1.00 | 32.62 | A | C |
| ATOM | 2034 | CB | CYS | A | 317 | 16.294 | 49.593 | 48.636 | 1.00 | 28.06 | A | C |
| ATOM | 2035 | SG | CYS | A | 317 | 18.048 | 49.858 | 48.418 | 1.00 | 28.06 | A | S |
| ATOM | 2036 | C | CYS | A | 317 | 14.399 | 48.045 | 48.709 | 1.00 | 32.62 | A | C |
| ATOM | 2037 | O | CYS | A | 317 | 14.336 | 47.317 | 49.698 | 1.00 | 32.62 | A | O |
| ATOM | 2038 | N | SER | A | 318 | 13.348 | 48.668 | 48.181 | 1.00 | 25.43 | A | N |
| ATOM | 2039 | CA | SER | A | 318 | 11.999 | 48.539 | 48.738 | 1.00 | 25.43 | A | C |
| ATOM | 2040 | CB | SER | A | 318 | 11.091 | 49.617 | 48.159 | 1.00 | 32.84 | A | C |
| ATOM | 2041 | OG | SER | A | 318 | 11.035 | 49.480 | 46.750 | 1.00 | 32.84 | A | O |
| ATOM | 2042 | C | SER | A | 318 | 11.384 | 47.170 | 48.468 | 1.00 | 25.43 | A | C |
| ATOM | 2043 | O | SER | A | 318 | 10.539 | 46.690 | 49.230 | 1.00 | 25.43 | A | O |
| ATOM | 2044 | N | ARG | A | 319 | 11.808 | 46.558 | 47.369 | 1.00 | 29.94 | A | N |
| ATOM | 2045 | CA | ARG | A | 319 | 11.317 | 45.250 | 46.980 | 1.00 | 29.94 | A | C |
| ATOM | 2046 | CB | ARG | A | 319 | 11.343 | 45.111 | 45.465 | 1.00 | 43.35 | A | C |
| ATOM | 2047 | CG | ARG | A | 319 | 10.577 | 46.203 | 44.747 | 1.00 | 43.35 | A | C |
| ATOM | 2048 | CD | ARG | A | 319 | 9.076 | 46.107 | 44.951 | 1.00 | 43.35 | A | C |
| ATOM | 2049 | NE | ARG | A | 319 | 8.507 | 44.916 | 44.325 | 1.00 | 43.35 | A | N |
| ATOM | 2050 | CZ | ARG | A | 319 | 8.392 | 43.734 | 44.931 | 1.00 | 43.35 | A | C |
| ATOM | 2051 | NH1 | ARG | A | 319 | 8.808 | 43.586 | 46.185 | 1.00 | 43.35 | A | N |
| ATOM | 2052 | NH2 | ARG | A | 319 | 7.853 | 42.695 | 44.294 | 1.00 | 43.35 | A | N |
| ATOM | 2053 | C | ARG | A | 319 | 12.162 | 44.160 | 47.619 | 1.00 | 29.94 | A | C |
| ATOM | 2054 | O | ARG | A | 319 | 11.827 | 42.977 | 47.543 | 1.00 | 29.94 | A | O |
| ATOM | 2055 | N | LEU | A | 320 | 13.258 | 44.558 | 48.255 | 1.00 | 15.48 | A | N |
| ATOM | 2056 | CA | LEU | A | 320 | 14.130 | 43.600 | 48.905 | 1.00 | 15.48 | A | C |
| ATOM | 2057 | CB | LEU | A | 320 | 15.595 | 43.969 | 48.651 | 1.00 | 12.94 | A | C |
| ATOM | 2058 | CG | LEU | A | 320 | 16.104 | 43.671 | 47.232 | 1.00 | 12.94 | A | C |
| ATOM | 2059 | CD1 | LEU | A | 320 | 17.545 | 44.111 | 47.074 | 1.00 | 12.94 | A | C |
| ATOM | 2060 | CD2 | LEU | A | 320 | 15.977 | 42.182 | 46.957 | 1.00 | 12.94 | A | C |
| ATOM | 2061 | C | LEU | A | 320 | 13.829 | 43.586 | 50.385 | 1.00 | 15.48 | A | C |
| ATOM | 2062 | O | LEU | A | 320 | 13.659 | 42.533 | 50.990 | 1.00 | 15.48 | A | O |
| ATOM | 2063 | N | LEU | A | 321 | 13.739 | 44.780 | 50.950 | 1.00 | 22.14 | A | N |
| ATOM | 2064 | CA | LEU | A | 321 | 13.470 | 44.956 | 52.363 | 1.00 | 22.14 | A | C |
| ATOM | 2065 | CB | LEU | A | 321 | 14.210 | 46.196 | 52.860 | 1.00 | 13.09 | A | C |
| ATOM | 2066 | CG | LEU | A | 321 | 15.722 | 46.126 | 52.633 | 1.00 | 13.09 | A | C |
| ATOM | 2067 | CD1 | LEU | A | 321 | 16.354 | 47.470 | 52.936 | 1.00 | 13.09 | A | C |
| ATOM | 2068 | CD2 | LEU | A | 321 | 16.323 | 45.029 | 53.494 | 1.00 | 13.09 | A | C |
| ATOM | 2069 | C | LEU | A | 321 | 11.972 | 45.085 | 52.600 | 1.00 | 22.14 | A | C |
| ATOM | 2070 | O | LEU | A | 321 | 11.455 | 46.179 | 52.794 | 1.00 | 22.14 | A | O |
| ATOM | 2071 | N | GLU | A | 322 | 11.283 | 43.950 | 52.576 | 1.00 | 39.55 | A | N |
| ATOM | 2072 | CA | GLU | A | 322 | 9.837 | 43.906 | 52.778 | 1.00 | 39.55 | A | C |
| ATOM | 2073 | CB | GLU | A | 322 | 9.154 | 43.265 | 51.563 | 1.00 | 39.14 | A | C |

| | | | | | | | | | | | | |
|---|---|---|---|---|---|---|---|---|---|---|---|---|
| ATOM | 2074 | CG | GLU | A | 322 | 9.408 | 44.001 | 50.266 | 1.00 | 39.14 | A | C |
| ATOM | 2075 | CD | GLU | A | 322 | 8.129 | 44.516 | 49.616 | 1.00 | 39.14 | A | C |
| ATOM | 2076 | OE1 | GLU | A | 322 | 7.164 | 44.838 | 50.350 | 1.00 | 39.14 | A | O |
| ATOM | 2077 | OE2 | GLU | A | 322 | 8.098 | 44.614 | 48.365 | 1.00 | 39.14 | A | O |
| ATOM | 2078 | C | GLU | A | 322 | 9.514 | 43.083 | 54.022 | 1.00 | 39.55 | A | C |
| ATOM | 2079 | O | GLU | A | 322 | 10.126 | 42.039 | 54.241 | 1.00 | 39.55 | A | O |
| ATOM | 2080 | N | TYR | A | 323 | 8.556 | 43.534 | 54.831 | 1.00 | 18.00 | A | N |
| ATOM | 2081 | CA | TYR | A | 323 | 8.202 | 42.797 | 56.038 | 1.00 | 18.00 | A | C |
| ATOM | 2082 | CB | TYR | A | 323 | 7.027 | 43.460 | 56.757 | 1.00 | 27.51 | A | C |
| ATOM | 2083 | CG | TYR | A | 323 | 7.414 | 44.653 | 57.598 | 1.00 | 27.51 | A | C |
| ATOM | 2084 | CD1 | TYR | A | 323 | 8.369 | 44.538 | 58.614 | 1.00 | 27.51 | A | C |
| ATOM | 2085 | CE1 | TYR | A | 323 | 8.731 | 45.633 | 59.396 | 1.00 | 27.51 | A | C |
| ATOM | 2086 | CD2 | TYR | A | 323 | 6.825 | 45.894 | 57.386 | 1.00 | 27.51 | A | C |
| ATOM | 2087 | CE2 | TYR | A | 323 | 7.174 | 47.001 | 58.165 | 1.00 | 27.51 | A | C |
| ATOM | 2088 | CZ | TYR | A | 323 | 8.130 | 46.867 | 59.168 | 1.00 | 27.51 | A | C |
| ATOM | 2089 | OH | TYR | A | 323 | 8.480 | 47.972 | 59.919 | 1.00 | 27.51 | A | O |
| ATOM | 2090 | C | TYR | A | 323 | 7.864 | 41.344 | 55.759 | 1.00 | 18.00 | A | C |
| ATOM | 2091 | O | TYR | A | 323 | 8.456 | 40.441 | 56.338 | 1.00 | 18.00 | A | O |
| ATOM | 2092 | N | THR | A | 324 | 6.906 | 41.114 | 54.874 | 1.00 | 31.56 | A | N |
| ATOM | 2093 | CA | THR | A | 324 | 6.525 | 39.750 | 54.538 | 1.00 | 31.56 | A | C |
| ATOM | 2094 | CB | THR | A | 324 | 5.279 | 39.710 | 53.650 | 1.00 | 37.28 | A | C |
| ATOM | 2095 | OG1 | THR | A | 324 | 4.172 | 40.305 | 54.344 | 1.00 | 37.28 | A | O |
| ATOM | 2096 | CG2 | THR | A | 324 | 4.950 | 38.268 | 53.288 | 1.00 | 37.28 | A | C |
| ATOM | 2097 | C | THR | A | 324 | 7.661 | 39.078 | 53.782 | 1.00 | 31.56 | A | C |
| ATOM | 2098 | O | THR | A | 324 | 7.983 | 39.441 | 52.649 | 1.00 | 31.56 | A | O |
| ATOM | 2099 | N | PRO | A | 325 | 8.280 | 38.073 | 54.400 | 1.00 | 22.39 | A | N |
| ATOM | 2100 | CD | PRO | A | 325 | 8.071 | 37.549 | 55.754 | 1.00 | 18.66 | A | C |
| ATOM | 2101 | CA | PRO | A | 325 | 9.380 | 37.396 | 53.721 | 1.00 | 22.39 | A | C |
| ATOM | 2102 | CB | PRO | A | 325 | 9.783 | 36.300 | 54.713 | 1.00 | 18.66 | A | C |
| ATOM | 2103 | CG | PRO | A | 325 | 8.614 | 36.179 | 55.632 | 1.00 | 18.66 | A | C |
| ATOM | 2104 | C | PRO | A | 325 | 9.013 | 36.874 | 52.349 | 1.00 | 22.39 | A | C |
| ATOM | 2105 | O | PRO | A | 325 | 9.818 | 36.885 | 51.436 | 1.00 | 22.39 | A | O |
| ATOM | 2106 | N | THR | A | 326 | 7.770 | 36.463 | 52.201 | 1.00 | 23.30 | A | N |
| ATOM | 2107 | CA | THR | A | 326 | 7.279 | 35.892 | 50.955 | 1.00 | 23.30 | A | C |
| ATOM | 2108 | CB | THR | A | 326 | 5.948 | 35.174 | 51.240 | 1.00 | 25.06 | A | C |
| ATOM | 2109 | OG1 | THR | A | 326 | 5.851 | 34.007 | 50.425 | 1.00 | 25.06 | A | O |
| ATOM | 2110 | CG2 | THR | A | 326 | 4.772 | 36.099 | 50.979 | 1.00 | 25.06 | A | C |
| ATOM | 2111 | C | THR | A | 326 | 7.104 | 36.926 | 49.845 | 1.00 | 23.30 | A | C |
| ATOM | 2112 | O | THR | A | 326 | 7.032 | 36.578 | 48.666 | 1.00 | 23.30 | A | O |
| ATOM | 2113 | N | ALA | A | 327 | 7.050 | 38.196 | 50.239 | 1.00 | 44.50 | A | N |
| ATOM | 2114 | CA | ALA | A | 327 | 6.872 | 39.304 | 49.299 | 1.00 | 44.50 | A | C |
| ATOM | 2115 | CB | ALA | A | 327 | 6.063 | 40.414 | 49.961 | 1.00 | 16.21 | A | C |
| ATOM | 2116 | C | ALA | A | 327 | 8.185 | 39.872 | 48.748 | 1.00 | 44.50 | A | C |
| ATOM | 2117 | O | ALA | A | 327 | 8.182 | 40.640 | 47.783 | 1.00 | 44.50 | A | O |
| ATOM | 2118 | N | ARG | A | 328 | 9.312 | 39.508 | 49.348 | 1.00 | 30.85 | A | N |
| ATOM | 2119 | CA | ARG | A | 328 | 10.583 | 40.018 | 48.860 | 1.00 | 30.85 | A | C |
| ATOM | 2120 | CB | ARG | A | 328 | 11.700 | 39.682 | 49.841 | 1.00 | 16.09 | A | C |
| ATOM | 2121 | CG | ARG | A | 328 | 11.524 | 40.354 | 51.170 | 1.00 | 16.09 | A | C |
| ATOM | 2122 | CD | ARG | A | 328 | 12.386 | 39.735 | 52.242 | 1.00 | 16.09 | A | C |
| ATOM | 2123 | NE | ARG | A | 328 | 11.793 | 39.984 | 53.546 | 1.00 | 16.09 | A | N |
| ATOM | 2124 | CZ | ARG | A | 328 | 12.142 | 39.366 | 54.662 | 1.00 | 16.09 | A | C |
| ATOM | 2125 | NH1 | ARG | A | 328 | 13.098 | 38.451 | 54.658 | 1.00 | 16.09 | A | N |
| ATOM | 2126 | NH2 | ARG | A | 328 | 11.508 | 39.652 | 55.780 | 1.00 | 16.09 | A | N |
| ATOM | 2127 | C | ARG | A | 328 | 10.873 | 39.413 | 47.504 | 1.00 | 30.85 | A | C |
| ATOM | 2128 | O | ARG | A | 328 | 10.252 | 38.433 | 47.126 | 1.00 | 30.85 | A | O |
| ATOM | 2129 | N | LEU | A | 329 | 11.804 | 40.006 | 46.766 | 1.00 | 25.19 | A | N |
| ATOM | 2130 | CA | LEU | A | 329 | 12.169 | 39.483 | 45.455 | 1.00 | 25.19 | A | C |
| ATOM | 2131 | CB | LEU | A | 329 | 12.904 | 40.543 | 44.637 | 1.00 | 20.15 | A | C |
| ATOM | 2132 | CG | LEU | A | 329 | 12.113 | 41.532 | 43.791 | 1.00 | 20.15 | A | C |
| ATOM | 2133 | CD1 | LEU | A | 329 | 10.782 | 41.843 | 44.417 | 1.00 | 20.15 | A | C |
| ATOM | 2134 | CD2 | LEU | A | 329 | 12.941 | 42.787 | 43.640 | 1.00 | 20.15 | A | C |
| ATOM | 2135 | C | LEU | A | 329 | 13.079 | 38.285 | 45.627 | 1.00 | 25.19 | A | C |
| ATOM | 2136 | O | LEU | A | 329 | 13.736 | 38.137 | 46.656 | 1.00 | 25.19 | A | O |

| | | | | | | | | | | | | |
|---|---|---|---|---|---|---|---|---|---|---|---|---|
| ATOM | 2137 | N | THR | A | 330 | 13.114 | 37.421 | 44.628 | 1.00 | 24.41 | A | N |
| ATOM | 2138 | CA | THR | A | 330 | 13.989 | 36.273 | 44.703 | 1.00 | 24.41 | A | C |
| ATOM | 2139 | CB | THR | A | 330 | 13.474 | 35.104 | 43.856 | 1.00 | 21.52 | A | C |
| ATOM | 2140 | OG1 | THR | A | 330 | 13.442 | 35.492 | 42.479 | 1.00 | 21.52 | A | O |
| ATOM | 2141 | CG2 | THR | A | 330 | 12.086 | 34.693 | 44.296 | 1.00 | 21.52 | A | C |
| ATOM | 2142 | C | THR | A | 330 | 15.323 | 36.722 | 44.149 | 1.00 | 24.41 | A | C |
| ATOM | 2143 | O | THR | A | 330 | 15.385 | 37.691 | 43.400 | 1.00 | 24.41 | A | O |
| ATOM | 2144 | N | PRO | A | 331 | 16.414 | 36.037 | 44.521 | 1.00 | 29.54 | A | N |
| ATOM | 2145 | CD | PRO | A | 331 | 16.502 | 34.899 | 45.454 | 1.00 | 13.64 | A | C |
| ATOM | 2146 | CA | PRO | A | 331 | 17.741 | 36.408 | 44.023 | 1.00 | 29.54 | A | C |
| ATOM | 2147 | CB | PRO | A | 331 | 18.599 | 35.217 | 44.430 | 1.00 | 13.64 | A | C |
| ATOM | 2148 | CG | PRO | A | 331 | 17.986 | 34.802 | 45.721 | 1.00 | 13.64 | A | C |
| ATOM | 2149 | C | PRO | A | 331 | 17.740 | 36.628 | 42.506 | 1.00 | 29.54 | A | C |
| ATOM | 2150 | O | PRO | A | 331 | 18.295 | 37.608 | 42.019 | 1.00 | 29.54 | A | O |
| ATOM | 2151 | N | LEU | A | 332 | 17.106 | 35.724 | 41.762 | 1.00 | 31.33 | A | N |
| ATOM | 2152 | CA | LEU | A | 332 | 17.066 | 35.853 | 40.313 | 1.00 | 31.33 | A | C |
| ATOM | 2153 | CB | LEU | A | 332 | 16.515 | 34.596 | 39.651 | 1.00 | 33.42 | A | C |
| ATOM | 2154 | CG | LEU | A | 332 | 17.535 | 33.667 | 38.999 | 1.00 | 33.42 | A | C |
| ATOM | 2155 | CD1 | LEU | A | 332 | 16.797 | 32.577 | 38.239 | 1.00 | 33.42 | A | C |
| ATOM | 2156 | CD2 | LEU | A | 332 | 18.423 | 34.452 | 38.067 | 1.00 | 33.42 | A | C |
| ATOM | 2157 | C | LEU | A | 332 | 16.241 | 37.030 | 39.854 | 1.00 | 31.33 | A | C |
| ATOM | 2158 | O | LEU | A | 332 | 16.604 | 37.702 | 38.892 | 1.00 | 31.33 | A | O |
| ATOM | 2159 | N | GLU | A | 333 | 15.120 | 37.278 | 40.523 | 1.00 | 27.63 | A | N |
| ATOM | 2160 | CA | GLU | A | 333 | 14.264 | 38.400 | 40.143 | 1.00 | 27.63 | A | C |
| ATOM | 2161 | CB | GLU | A | 333 | 12.952 | 38.383 | 40.923 | 1.00 | 30.53 | A | C |
| ATOM | 2162 | CG | GLU | A | 333 | 12.091 | 37.177 | 40.668 | 1.00 | 30.53 | A | C |
| ATOM | 2163 | CD | GLU | A | 333 | 10.945 | 37.100 | 41.639 | 1.00 | 30.53 | A | C |
| ATOM | 2164 | OE1 | GLU | A | 333 | 11.113 | 37.557 | 42.792 | 1.00 | 30.53 | A | O |
| ATOM | 2165 | OE2 | GLU | A | 333 | 9.878 | 36.575 | 41.272 | 1.00 | 30.53 | A | O |
| ATOM | 2166 | C | GLU | A | 333 | 14.992 | 39.699 | 40.430 | 1.00 | 27.63 | A | C |
| ATOM | 2167 | O | GLU | A | 333 | 14.690 | 40.741 | 39.846 | 1.00 | 27.63 | A | O |
| ATOM | 2168 | N | ALA | A | 334 | 15.947 | 39.628 | 41.348 | 1.00 | 21.92 | A | N |
| ATOM | 2169 | CA | ALA | A | 334 | 16.727 | 40.797 | 41.701 | 1.00 | 21.92 | A | C |
| ATOM | 2170 | CB | ALA | A | 334 | 17.431 | 40.586 | 43.039 | 1.00 | 38.95 | A | C |
| ATOM | 2171 | C | ALA | A | 334 | 17.737 | 41.008 | 40.587 | 1.00 | 21.92 | A | C |
| ATOM | 2172 | O | ALA | A | 334 | 17.878 | 42.109 | 40.068 | 1.00 | 21.92 | A | O |
| ATOM | 2173 | N | CYS | A | 335 | 18.431 | 39.944 | 40.211 | 1.00 | 28.10 | A | N |
| ATOM | 2174 | CA | CYS | A | 335 | 19.415 | 40.039 | 39.139 | 1.00 | 28.10 | A | C |
| ATOM | 2175 | CB | CYS | A | 335 | 19.932 | 38.655 | 38.747 | 1.00 | 26.43 | A | C |
| ATOM | 2176 | SG | CYS | A | 335 | 21.134 | 37.920 | 39.855 | 1.00 | 26.43 | A | S |
| ATOM | 2177 | C | CYS | A | 335 | 18.816 | 40.683 | 37.901 | 1.00 | 28.10 | A | C |
| ATOM | 2178 | O | CYS | A | 335 | 19.481 | 41.445 | 37.198 | 1.00 | 28.10 | A | O |
| ATOM | 2179 | N | ALA | A | 336 | 17.550 | 40.361 | 37.648 | 1.00 | 19.08 | A | N |
| ATOM | 2180 | CA | ALA | A | 336 | 16.819 | 40.851 | 36.490 | 1.00 | 19.08 | A | C |
| ATOM | 2181 | CB | ALA | A | 336 | 15.774 | 39.835 | 36.101 | 1.00 | 20.87 | A | C |
| ATOM | 2182 | C | ALA | A | 336 | 16.170 | 42.204 | 36.696 | 1.00 | 19.08 | A | C |
| ATOM | 2183 | O | ALA | A | 336 | 15.474 | 42.688 | 35.816 | 1.00 | 19.08 | A | O |
| ATOM | 2184 | N | HIS | A | 337 | 16.399 | 42.815 | 37.854 | 1.00 | 31.75 | A | N |
| ATOM | 2185 | CA | HIS | A | 337 | 15.806 | 44.116 | 38.158 | 1.00 | 31.75 | A | C |
| ATOM | 2186 | CB | HIS | A | 337 | 16.097 | 44.541 | 39.593 | 1.00 | 27.83 | A | C |
| ATOM | 2187 | CG | HIS | A | 337 | 15.377 | 45.786 | 40.003 | 1.00 | 27.83 | A | C |
| ATOM | 2188 | CD2 | HIS | A | 337 | 15.786 | 47.073 | 40.070 | 1.00 | 27.83 | A | C |
| ATOM | 2189 | ND1 | HIS | A | 337 | 14.057 | 45.786 | 40.396 | 1.00 | 27.83 | A | N |
| ATOM | 2190 | CE1 | HIS | A | 337 | 13.685 | 47.018 | 40.691 | 1.00 | 27.83 | A | C |
| ATOM | 2191 | NE2 | HIS | A | 337 | 14.718 | 47.820 | 40.502 | 1.00 | 27.83 | A | N |
| ATOM | 2192 | C | HIS | A | 337 | 16.338 | 45.176 | 37.222 | 1.00 | 31.75 | A | C |
| ATOM | 2193 | O | HIS | A | 337 | 17.455 | 45.063 | 36.720 | 1.00 | 31.75 | A | O |
| ATOM | 2194 | N | SER | A | 338 | 15.539 | 46.216 | 37.005 | 1.00 | 34.32 | A | N |
| ATOM | 2195 | CA | SER | A | 338 | 15.922 | 47.279 | 36.095 | 1.00 | 34.32 | A | C |
| ATOM | 2196 | CB | SER | A | 338 | 14.782 | 48.290 | 35.957 | 1.00 | 33.71 | A | C |
| ATOM | 2197 | OG | SER | A | 338 | 14.909 | 49.341 | 36.892 | 1.00 | 33.71 | A | O |
| ATOM | 2198 | C | SER | A | 338 | 17.208 | 47.977 | 36.521 | 1.00 | 34.32 | A | C |
| ATOM | 2199 | O | SER | A | 338 | 17.978 | 48.424 | 35.670 | 1.00 | 34.32 | A | O |

| | | | | | | | | | | | | |
|---|---|---|---|---|---|---|---|---|---|---|---|---|
| ATOM | 2200 | N | PHE | A | 339 | 17.446 | 48.052 | 37.829 | 1.00 | 19.65 | A | N |
| ATOM | 2201 | CA | PHE | A | 339 | 18.642 | 48.702 | 38.367 | 1.00 | 19.65 | A | C |
| ATOM | 2202 | CB | PHE | A | 339 | 18.693 | 48.515 | 39.891 | 1.00 | 24.09 | A | C |
| ATOM | 2203 | CG | PHE | A | 339 | 19.953 | 49.033 | 40.538 | 1.00 | 24.09 | A | C |
| ATOM | 2204 | CD1 | PHE | A | 339 | 20.366 | 50.350 | 40.352 | 1.00 | 24.09 | A | C |
| ATOM | 2205 | CD2 | PHE | A | 339 | 20.720 | 48.205 | 41.345 | 1.00 | 24.09 | A | C |
| ATOM | 2206 | CE1 | PHE | A | 339 | 21.527 | 50.829 | 40.962 | 1.00 | 24.09 | A | C |
| ATOM | 2207 | CE2 | PHE | A | 339 | 21.881 | 48.674 | 41.956 | 1.00 | 24.09 | A | C |
| ATOM | 2208 | CZ | PHE | A | 339 | 22.284 | 49.987 | 41.766 | 1.00 | 24.09 | A | C |
| ATOM | 2209 | C | PHE | A | 339 | 19.926 | 48.182 | 37.738 | 1.00 | 19.65 | A | C |
| ATOM | 2210 | O | PHE | A | 339 | 20.904 | 48.915 | 37.631 | 1.00 | 19.65 | A | O |
| ATOM | 2211 | N | PHE | A | 340 | 19.901 | 46.922 | 37.306 | 1.00 | 17.89 | A | N |
| ATOM | 2212 | CA | PHE | A | 340 | 21.055 | 46.255 | 36.695 | 1.00 | 17.89 | A | C |
| ATOM | 2213 | CB | PHE | A | 340 | 21.087 | 44.800 | 37.142 | 1.00 | 22.64 | A | C |
| ATOM | 2214 | CG | PHE | A | 340 | 21.231 | 44.641 | 38.614 | 1.00 | 22.64 | A | C |
| ATOM | 2215 | CD1 | PHE | A | 340 | 22.400 | 45.021 | 39.249 | 1.00 | 22.64 | A | C |
| ATOM | 2216 | CD2 | PHE | A | 340 | 20.197 | 44.118 | 39.374 | 1.00 | 22.64 | A | C |
| ATOM | 2217 | CE1 | PHE | A | 340 | 22.527 | 44.910 | 40.624 | 1.00 | 22.64 | A | C |
| ATOM | 2218 | CE2 | PHE | A | 340 | 20.315 | 44.005 | 40.748 | 1.00 | 22.64 | A | C |
| ATOM | 2219 | CZ | PHE | A | 340 | 21.483 | 44.389 | 41.373 | 1.00 | 22.64 | A | C |
| ATOM | 2220 | C | PHE | A | 340 | 21.142 | 46.307 | 35.168 | 1.00 | 17.89 | A | C |
| ATOM | 2221 | O | PHE | A | 340 | 22.050 | 45.738 | 34.573 | 1.00 | 17.89 | A | O |
| ATOM | 2222 | N | ASP | A | 341 | 20.208 | 46.989 | 34.530 | 1.00 | 36.31 | A | N |
| ATOM | 2223 | CA | ASP | A | 341 | 20.238 | 47.092 | 33.082 | 1.00 | 36.31 | A | C |
| ATOM | 2224 | CB | ASP | A | 341 | 19.159 | 48.072 | 32.604 | 1.00 | 45.23 | A | C |
| ATOM | 2225 | CG | ASP | A | 341 | 17.752 | 47.452 | 32.602 | 1.00 | 45.23 | A | C |
| ATOM | 2226 | OD1 | ASP | A | 341 | 17.626 | 46.234 | 32.897 | 1.00 | 45.23 | A | O |
| ATOM | 2227 | OD2 | ASP | A | 341 | 16.767 | 48.175 | 32.297 | 1.00 | 45.23 | A | O |
| ATOM | 2228 | C | ASP | A | 341 | 21.602 | 47.496 | 32.510 | 1.00 | 36.31 | A | C |
| ATOM | 2229 | O | ASP | A | 341 | 22.053 | 46.900 | 31.533 | 1.00 | 36.31 | A | O |
| ATOM | 2230 | N | GLU | A | 342 | 22.261 | 48.487 | 33.114 | 1.00 | 23.08 | A | N |
| ATOM | 2231 | CA | GLU | A | 342 | 23.562 | 48.949 | 32.626 | 1.00 | 23.08 | A | C |
| ATOM | 2232 | CB | GLU | A | 342 | 24.176 | 49.956 | 33.591 | 1.00 | 35.33 | A | C |
| ATOM | 2233 | CG | GLU | A | 342 | 25.407 | 50.676 | 33.033 | 1.00 | 35.33 | A | C |
| ATOM | 2234 | CD | GLU | A | 342 | 25.912 | 51.798 | 33.952 | 1.00 | 35.33 | A | C |
| ATOM | 2235 | OE1 | GLU | A | 342 | 25.068 | 52.552 | 34.490 | 1.00 | 35.33 | A | O |
| ATOM | 2236 | OE2 | GLU | A | 342 | 27.144 | 51.941 | 34.131 | 1.00 | 35.33 | A | O |
| ATOM | 2237 | C | GLU | A | 342 | 24.563 | 47.818 | 32.410 | 1.00 | 23.08 | A | C |
| ATOM | 2238 | O | GLU | A | 342 | 25.389 | 47.875 | 31.501 | 1.00 | 23.08 | A | O |
| ATOM | 2239 | N | LEU | A | 343 | 24.490 | 46.790 | 33.247 | 1.00 | 15.62 | A | N |
| ATOM | 2240 | CA | LEU | A | 343 | 25.405 | 45.663 | 33.140 | 1.00 | 15.62 | A | C |
| ATOM | 2241 | CB | LEU | A | 343 | 25.303 | 44.790 | 34.389 | 1.00 | 34.06 | A | C |
| ATOM | 2242 | CG | LEU | A | 343 | 25.480 | 45.518 | 35.726 | 1.00 | 34.06 | A | C |
| ATOM | 2243 | CD1 | LEU | A | 343 | 25.331 | 44.530 | 36.896 | 1.00 | 34.06 | A | C |
| ATOM | 2244 | CD2 | LEU | A | 343 | 26.855 | 46.208 | 35.742 | 1.00 | 34.06 | A | C |
| ATOM | 2245 | C | LEU | A | 343 | 25.087 | 44.833 | 31.920 | 1.00 | 15.62 | A | C |
| ATOM | 2246 | O | LEU | A | 343 | 25.935 | 44.127 | 31.395 | 1.00 | 15.62 | A | O |
| ATOM | 2247 | N | ARG | A | 344 | 23.842 | 44.915 | 31.477 | 1.00 | 39.47 | A | N |
| ATOM | 2248 | CA | ARG | A | 344 | 23.402 | 44.167 | 30.310 | 1.00 | 39.47 | A | C |
| ATOM | 2249 | CB | ARG | A | 344 | 21.918 | 43.832 | 30.434 | 1.00 | 24.87 | A | C |
| ATOM | 2250 | CG | ARG | A | 344 | 21.666 | 42.611 | 31.298 | 1.00 | 24.87 | A | C |
| ATOM | 2251 | CD | ARG | A | 344 | 20.194 | 42.309 | 31.412 | 1.00 | 24.87 | A | C |
| ATOM | 2252 | NE | ARG | A | 344 | 19.499 | 43.210 | 32.331 | 1.00 | 24.87 | A | N |
| ATOM | 2253 | CZ | ARG | A | 344 | 19.408 | 43.029 | 33.647 | 1.00 | 24.87 | A | C |
| ATOM | 2254 | NH1 | ARG | A | 344 | 19.970 | 41.972 | 34.226 | 1.00 | 24.87 | A | N |
| ATOM | 2255 | NH2 | ARG | A | 344 | 18.741 | 43.907 | 34.385 | 1.00 | 24.87 | A | N |
| ATOM | 2256 | C | ARG | A | 344 | 23.687 | 44.913 | 29.010 | 1.00 | 39.47 | A | C |
| ATOM | 2257 | O | ARG | A | 344 | 23.548 | 44.355 | 27.927 | 1.00 | 39.47 | A | O |
| ATOM | 2258 | N | ASP | A | 345 | 24.087 | 46.176 | 29.121 | 1.00 | 39.02 | A | N |
| ATOM | 2259 | CA | ASP | A | 345 | 24.441 | 46.960 | 27.948 | 1.00 | 39.02 | A | C |
| ATOM | 2260 | CB | ASP | A | 345 | 24.695 | 48.418 | 28.357 | 1.00 | 33.23 | A | C |
| ATOM | 2261 | CG | ASP | A | 345 | 25.125 | 49.306 | 27.189 | 1.00 | 33.23 | A | C |
| ATOM | 2262 | OD1 | ASP | A | 345 | 25.985 | 48.893 | 26.384 | 1.00 | 33.23 | A | O |

| | | | | | | | | | | | | |
|---|---|---|---|---|---|---|---|---|---|---|---|---|
| ATOM | 2263 | OD2 | ASP | A | 345 | 24.615 | 50.437 | 27.099 | 1.00 | 33.23 | A | O |
| ATOM | 2264 | C | ASP | A | 345 | 25.721 | 46.320 | 27.395 | 1.00 | 39.02 | A | C |
| ATOM | 2265 | O | ASP | A | 345 | 26.615 | 45.950 | 28.160 | 1.00 | 39.02 | A | O |
| ATOM | 2266 | N | PRO | A | 346 | 25.811 | 46.154 | 26.065 | 1.00 | 29.11 | A | N |
| ATOM | 2267 | CD | PRO | A | 346 | 24.746 | 46.385 | 25.074 | 1.00 | 15.50 | A | C |
| ATOM | 2268 | CA | PRO | A | 346 | 26.993 | 45.554 | 25.431 | 1.00 | 29.11 | A | C |
| ATOM | 2269 | CB | PRO | A | 346 | 26.522 | 45.302 | 24.003 | 1.00 | 15.50 | A | C |
| ATOM | 2270 | CG | PRO | A | 346 | 25.515 | 46.395 | 23.788 | 1.00 | 15.50 | A | C |
| ATOM | 2271 | C | PRO | A | 346 | 28.248 | 46.431 | 25.488 | 1.00 | 29.11 | A | C |
| ATOM | 2272 | O | PRO | A | 346 | 29.370 | 45.931 | 25.431 | 1.00 | 29.11 | A | O |
| ATOM | 2273 | N | ASN | A | 347 | 28.051 | 47.734 | 25.628 | 1.00 | 43.93 | A | N |
| ATOM | 2274 | CA | ASN | A | 347 | 29.163 | 48.676 | 25.675 | 1.00 | 43.93 | A | C |
| ATOM | 2275 | CB | ASN | A | 347 | 28.730 | 49.987 | 25.038 | 1.00 | 45.58 | A | C |
| ATOM | 2276 | CG | ASN | A | 347 | 28.522 | 49.850 | 23.553 | 1.00 | 45.58 | A | C |
| ATOM | 2277 | OD1 | ASN | A | 347 | 27.705 | 50.566 | 22.962 | 1.00 | 45.58 | A | O |
| ATOM | 2278 | ND2 | ASN | A | 347 | 29.265 | 48.924 | 22.931 | 1.00 | 45.58 | A | N |
| ATOM | 2279 | C | ASN | A | 347 | 29.744 | 48.949 | 27.058 | 1.00 | 43.93 | A | C |
| ATOM | 2280 | O | ASN | A | 347 | 30.772 | 49.624 | 27.194 | 1.00 | 43.93 | A | O |
| ATOM | 2281 | N | VAL | A | 348 | 29.095 | 48.415 | 28.082 | 1.00 | 34.26 | A | N |
| ATOM | 2282 | CA | VAL | A | 348 | 29.556 | 48.627 | 29.432 | 1.00 | 34.26 | A | C |
| ATOM | 2283 | CB | VAL | A | 348 | 28.593 | 47.988 | 30.446 | 1.00 | 26.97 | A | C |
| ATOM | 2284 | CG1 | VAL | A | 348 | 28.742 | 46.482 | 30.445 | 1.00 | 26.97 | A | C |
| ATOM | 2285 | CG2 | VAL | A | 348 | 28.842 | 48.577 | 31.820 | 1.00 | 26.97 | A | C |
| ATOM | 2286 | C | VAL | A | 348 | 30.962 | 48.099 | 29.653 | 1.00 | 34.26 | A | C |
| ATOM | 2287 | O | VAL | A | 348 | 31.288 | 46.963 | 29.298 | 1.00 | 34.26 | A | O |
| ATOM | 2288 | N | LYS | A | 349 | 31.790 | 48.967 | 30.225 | 1.00 | 38.62 | A | N |
| ATOM | 2289 | CA | LYS | A | 349 | 33.179 | 48.671 | 30.558 | 1.00 | 38.62 | A | C |
| ATOM | 2290 | CB | LYS | A | 349 | 34.134 | 49.366 | 29.574 | 1.00 | 44.36 | A | C |
| ATOM | 2291 | CG | LYS | A | 349 | 34.190 | 48.725 | 28.183 | 1.00 | 44.36 | A | C |
| ATOM | 2292 | CD | LYS | A | 349 | 34.312 | 47.188 | 28.282 | 1.00 | 44.36 | A | C |
| ATOM | 2293 | CE | LYS | A | 349 | 35.064 | 46.547 | 27.106 | 1.00 | 44.36 | A | C |
| ATOM | 2294 | NZ | LYS | A | 349 | 36.549 | 46.520 | 27.324 | 1.00 | 44.36 | A | N |
| ATOM | 2295 | C | LYS | A | 349 | 33.420 | 49.171 | 31.989 | 1.00 | 38.62 | A | C |
| ATOM | 2296 | O | LYS | A | 349 | 32.594 | 49.909 | 32.540 | 1.00 | 38.62 | A | O |
| ATOM | 2297 | N | LEU | A | 350 | 34.532 | 48.763 | 32.594 | 1.00 | 42.94 | A | N |
| ATOM | 2298 | CA | LEU | A | 350 | 34.845 | 49.197 | 33.949 | 1.00 | 42.94 | A | C |
| ATOM | 2299 | CB | LEU | A | 350 | 35.752 | 48.183 | 34.639 | 1.00 | 53.75 | A | C |
| ATOM | 2300 | CG | LEU | A | 350 | 35.084 | 46.845 | 34.957 | 1.00 | 53.75 | A | C |
| ATOM | 2301 | CD1 | LEU | A | 350 | 36.142 | 45.837 | 35.380 | 1.00 | 53.75 | A | C |
| ATOM | 2302 | CD2 | LEU | A | 350 | 34.055 | 47.028 | 36.043 | 1.00 | 53.75 | A | C |
| ATOM | 2303 | C | LEU | A | 350 | 35.534 | 50.551 | 33.919 | 1.00 | 42.94 | A | C |
| ATOM | 2304 | O | LEU | A | 350 | 36.042 | 50.978 | 32.878 | 1.00 | 42.94 | A | O |
| ATOM | 2305 | N | PRO | A | 351 | 35.562 | 51.250 | 35.063 | 1.00 | 41.49 | A | N |
| ATOM | 2306 | CD | PRO | A | 351 | 35.067 | 50.922 | 36.407 | 1.00 | 51.76 | A | C |
| ATOM | 2307 | CA | PRO | A | 351 | 36.222 | 52.553 | 35.063 | 1.00 | 41.49 | A | C |
| ATOM | 2308 | CB | PRO | A | 351 | 36.046 | 53.034 | 36.503 | 1.00 | 51.76 | A | C |
| ATOM | 2309 | CG | PRO | A | 351 | 34.854 | 52.284 | 36.974 | 1.00 | 51.76 | A | C |
| ATOM | 2310 | C | PRO | A | 351 | 37.690 | 52.357 | 34.693 | 1.00 | 41.49 | A | C |
| ATOM | 2311 | O | PRO | A | 351 | 38.307 | 53.187 | 34.025 | 1.00 | 41.49 | A | O |
| ATOM | 2312 | N | ASN | A | 352 | 38.226 | 51.220 | 35.114 | 1.00 | 49.39 | A | N |
| ATOM | 2313 | CA | ASN | A | 352 | 39.623 | 50.901 | 34.880 | 1.00 | 49.39 | A | C |
| ATOM | 2314 | CB | ASN | A | 352 | 40.030 | 49.751 | 35.821 | 1.00 | 64.51 | A | C |
| ATOM | 2315 | CG | ASN | A | 352 | 39.875 | 48.373 | 35.187 | 1.00 | 64.51 | A | C |
| ATOM | 2316 | OD1 | ASN | A | 352 | 39.148 | 48.192 | 34.196 | 1.00 | 64.51 | A | O |
| ATOM | 2317 | ND2 | ASN | A | 352 | 40.556 | 47.380 | 35.773 | 1.00 | 64.51 | A | N |
| ATOM | 2318 | C | ASN | A | 352 | 39.933 | 50.571 | 33.427 | 1.00 | 49.39 | A | C |
| ATOM | 2319 | O | ASN | A | 352 | 41.078 | 50.291 | 33.087 | 1.00 | 49.39 | A | O |
| ATOM | 2320 | N | GLY | A | 353 | 38.916 | 50.614 | 32.572 | 1.00 | 56.36 | A | N |
| ATOM | 2321 | CA | GLY | A | 353 | 39.130 | 50.307 | 31.170 | 1.00 | 56.36 | A | C |
| ATOM | 2322 | C | GLY | A | 353 | 38.632 | 48.924 | 30.761 | 1.00 | 56.36 | A | C |
| ATOM | 2323 | O | GLY | A | 353 | 37.757 | 48.812 | 29.890 | 1.00 | 56.36 | A | O |
| ATOM | 2324 | N | ARG | A | 354 | 39.201 | 47.880 | 31.371 | 1.00 | 54.38 | A | N |
| ATOM | 2325 | CA | ARG | A | 354 | 38.849 | 46.479 | 31.102 | 1.00 | 54.38 | A | C |

| | | | | | | | | | | | | |
|---|---|---|---|---|---|---|---|---|---|---|---|---|
| ATOM | 2326 | CB | ARG | A | 354 | 39.243 | 45.600 | 32.277 | 1.00 | 64.32 | A | C |
| ATOM | 2327 | CG | ARG | A | 354 | 40.705 | 45.465 | 32.560 | 1.00 | 64.32 | A | C |
| ATOM | 2328 | CD | ARG | A | 354 | 40.858 | 45.002 | 33.989 | 1.00 | 64.32 | A | C |
| ATOM | 2329 | NE | ARG | A | 354 | 41.811 | 43.907 | 34.149 | 1.00 | 64.32 | A | N |
| ATOM | 2330 | CZ | ARG | A | 354 | 42.752 | 43.880 | 35.095 | 1.00 | 64.32 | A | C |
| ATOM | 2331 | NH1 | ARG | A | 354 | 42.862 | 44.894 | 35.954 | 1.00 | 64.32 | A | N |
| ATOM | 2332 | NH2 | ARG | A | 354 | 43.575 | 42.842 | 35.193 | 1.00 | 64.32 | A | N |
| ATOM | 2333 | C | ARG | A | 354 | 37.377 | 46.163 | 30.854 | 1.00 | 54.38 | A | C |
| ATOM | 2334 | O | ARG | A | 354 | 36.497 | 47.027 | 30.897 | 1.00 | 54.38 | A | O |
| ATOM | 2335 | N | ASP | A | 355 | 37.118 | 44.875 | 30.664 | 1.00 | 54.92 | A | N |
| ATOM | 2336 | CA | ASP | A | 355 | 35.769 | 44.401 | 30.431 | 1.00 | 54.92 | A | C |
| ATOM | 2337 | CB | ASP | A | 355 | 35.790 | 43.168 | 29.547 | 1.00 | 61.21 | A | C |
| ATOM | 2338 | CG | ASP | A | 355 | 34.822 | 43.274 | 28.401 | 1.00 | 61.21 | A | C |
| ATOM | 2339 | OD1 | ASP | A | 355 | 33.617 | 43.557 | 28.629 | 1.00 | 61.21 | A | O |
| ATOM | 2340 | OD2 | ASP | A | 355 | 35.279 | 43.078 | 27.262 | 1.00 | 61.21 | A | O |
| ATOM | 2341 | C | ASP | A | 355 | 35.182 | 44.027 | 31.769 | 1.00 | 54.92 | A | C |
| ATOM | 2342 | O | ASP | A | 355 | 35.917 | 43.926 | 32.753 | 1.00 | 54.92 | A | O |
| ATOM | 2343 | N | THR | A | 356 | 33.868 | 43.821 | 31.826 | 1.00 | 33.41 | A | N |
| ATOM | 2344 | CA | THR | A | 356 | 33.280 | 43.424 | 33.095 | 1.00 | 33.41 | A | C |
| ATOM | 2345 | CB | THR | A | 356 | 31.766 | 43.744 | 33.214 | 1.00 | 32.80 | A | C |
| ATOM | 2346 | OG1 | THR | A | 356 | 31.027 | 42.914 | 32.319 | 1.00 | 32.80 | A | O |
| ATOM | 2347 | CG2 | THR | A | 356 | 31.492 | 45.208 | 32.908 | 1.00 | 32.80 | A | C |
| ATOM | 2348 | C | THR | A | 356 | 33.457 | 41.918 | 33.211 | 1.00 | 33.41 | A | C |
| ATOM | 2349 | O | THR | A | 356 | 33.768 | 41.240 | 32.240 | 1.00 | 33.41 | A | O |
| ATOM | 2350 | N | PRO | A | 357 | 33.278 | 41.375 | 34.413 | 1.00 | 22.19 | A | N |
| ATOM | 2351 | CD | PRO | A | 357 | 32.895 | 42.021 | 35.682 | 1.00 | 42.65 | A | C |
| ATOM | 2352 | CA | PRO | A | 357 | 33.437 | 39.926 | 34.569 | 1.00 | 22.19 | A | C |
| ATOM | 2353 | CB | PRO | A | 357 | 33.323 | 39.711 | 36.081 | 1.00 | 42.65 | A | C |
| ATOM | 2354 | CG | PRO | A | 357 | 33.508 | 41.111 | 36.682 | 1.00 | 42.65 | A | C |
| ATOM | 2355 | C | PRO | A | 357 | 32.297 | 39.224 | 33.848 | 1.00 | 22.19 | A | C |
| ATOM | 2356 | O | PRO | A | 357 | 31.454 | 39.864 | 33.222 | 1.00 | 22.19 | A | O |
| ATOM | 2357 | N | ALA | A | 358 | 32.275 | 37.901 | 33.974 | 1.00 | 48.19 | A | N |
| ATOM | 2358 | CA | ALA | A | 358 | 31.256 | 37.061 | 33.358 | 1.00 | 48.19 | A | C |
| ATOM | 2359 | CB | ALA | A | 358 | 31.718 | 35.625 | 33.359 | 1.00 | 55.58 | A | C |
| ATOM | 2360 | C | ALA | A | 358 | 29.942 | 37.193 | 34.122 | 1.00 | 48.19 | A | C |
| ATOM | 2361 | O | ALA | A | 358 | 29.816 | 36.654 | 35.217 | 1.00 | 48.19 | A | O |
| ATOM | 2362 | N | LEU | A | 359 | 28.965 | 37.891 | 33.545 | 1.00 | 28.69 | A | N |
| ATOM | 2363 | CA | LEU | A | 359 | 27.691 | 38.097 | 34.224 | 1.00 | 28.69 | A | C |
| ATOM | 2364 | CB | LEU | A | 359 | 27.344 | 39.581 | 34.227 | 1.00 | 19.29 | A | C |
| ATOM | 2365 | CG | LEU | A | 359 | 28.413 | 40.546 | 34.715 | 1.00 | 19.29 | A | C |
| ATOM | 2366 | CD1 | LEU | A | 359 | 28.027 | 41.942 | 34.279 | 1.00 | 19.29 | A | C |
| ATOM | 2367 | CD2 | LEU | A | 359 | 28.557 | 40.459 | 36.225 | 1.00 | 19.29 | A | C |
| ATOM | 2368 | C | LEU | A | 359 | 26.496 | 37.344 | 33.655 | 1.00 | 28.69 | A | C |
| ATOM | 2369 | O | LEU | A | 359 | 25.415 | 37.361 | 34.250 | 1.00 | 28.69 | A | O |
| ATOM | 2370 | N | PHE | A | 360 | 26.667 | 36.686 | 32.513 | 1.00 | 16.37 | A | N |
| ATOM | 2371 | CA | PHE | A | 360 | 25.538 | 35.988 | 31.912 | 1.00 | 16.37 | A | C |
| ATOM | 2372 | CB | PHE | A | 360 | 25.179 | 36.690 | 30.610 | 1.00 | 21.72 | A | C |
| ATOM | 2373 | CG | PHE | A | 360 | 25.151 | 38.182 | 30.737 | 1.00 | 21.72 | A | C |
| ATOM | 2374 | CD1 | PHE | A | 360 | 24.217 | 38.801 | 31.554 | 1.00 | 21.72 | A | C |
| ATOM | 2375 | CD2 | PHE | A | 360 | 26.090 | 38.973 | 30.078 | 1.00 | 21.72 | A | C |
| ATOM | 2376 | CE1 | PHE | A | 360 | 24.220 | 40.193 | 31.715 | 1.00 | 21.72 | A | C |
| ATOM | 2377 | CE2 | PHE | A | 360 | 26.100 | 40.353 | 30.235 | 1.00 | 21.72 | A | C |
| ATOM | 2378 | CZ | PHE | A | 360 | 25.166 | 40.965 | 31.053 | 1.00 | 21.72 | A | C |
| ATOM | 2379 | C | PHE | A | 360 | 25.669 | 34.478 | 31.694 | 1.00 | 16.37 | A | C |
| ATOM | 2380 | O | PHE | A | 360 | 24.750 | 33.839 | 31.197 | 1.00 | 16.37 | A | O |
| ATOM | 2381 | N | ASN | A | 361 | 26.798 | 33.908 | 32.090 | 1.00 | 19.29 | A | N |
| ATOM | 2382 | CA | ASN | A | 361 | 27.026 | 32.481 | 31.932 | 1.00 | 19.29 | A | C |
| ATOM | 2383 | CB | ASN | A | 361 | 28.519 | 32.185 | 32.128 | 1.00 | 26.28 | A | C |
| ATOM | 2384 | CG | ASN | A | 361 | 29.066 | 32.741 | 33.436 | 1.00 | 26.28 | A | C |
| ATOM | 2385 | OD1 | ASN | A | 361 | 28.379 | 33.480 | 34.155 | 1.00 | 26.28 | A | O |
| ATOM | 2386 | ND2 | ASN | A | 361 | 30.315 | 32.393 | 33.750 | 1.00 | 26.28 | A | N |
| ATOM | 2387 | C | ASN | A | 361 | 26.150 | 31.603 | 32.851 | 1.00 | 19.29 | A | C |
| ATOM | 2388 | O | ASN | A | 361 | 26.614 | 30.619 | 33.441 | 1.00 | 19.29 | A | O |

| | | | | | | | | | | | | |
|---|---|---|---|---|---|---|---|---|---|---|---|---|
| ATOM | 2389 | N | PHE | A | 362 | 24.870 | 31.958 | 32.946 | 1.00 | 33.58 | A | N |
| ATOM | 2390 | CA | PHE | A | 362 | 23.910 | 31.240 | 33.787 | 1.00 | 33.58 | A | C |
| ATOM | 2391 | CB | PHE | A | 362 | 22.523 | 31.886 | 33.677 | 1.00 | 29.79 | A | C |
| ATOM | 2392 | CG | PHE | A | 362 | 22.409 | 33.227 | 34.349 | 1.00 | 29.79 | A | C |
| ATOM | 2393 | CD1 | PHE | A | 362 | 22.484 | 33.338 | 35.728 | 1.00 | 29.79 | A | C |
| ATOM | 2394 | CD2 | PHE | A | 362 | 22.166 | 34.375 | 33.599 | 1.00 | 29.79 | A | C |
| ATOM | 2395 | CE1 | PHE | A | 362 | 22.323 | 34.573 | 36.347 | 1.00 | 29.79 | A | C |
| ATOM | 2396 | CE2 | PHE | A | 362 | 22.005 | 35.609 | 34.206 | 1.00 | 29.79 | A | C |
| ATOM | 2397 | CZ | PHE | A | 362 | 22.077 | 35.710 | 35.582 | 1.00 | 29.79 | A | C |
| ATOM | 2398 | C | PHE | A | 362 | 23.773 | 29.768 | 33.421 | 1.00 | 33.58 | A | C |
| ATOM | 2399 | O | PHE | A | 362 | 23.881 | 29.408 | 32.253 | 1.00 | 33.58 | A | O |
| ATOM | 2400 | N | THR | A | 363 | 23.530 | 28.921 | 34.416 | 1.00 | 30.86 | A | N |
| ATOM | 2401 | CA | THR | A | 363 | 23.329 | 27.491 | 34.173 | 1.00 | 30.86 | A | C |
| ATOM | 2402 | CB | THR | A | 363 | 24.059 | 26.606 | 35.204 | 1.00 | 16.07 | A | C |
| ATOM | 2403 | OG1 | THR | A | 363 | 23.635 | 26.968 | 36.525 | 1.00 | 16.07 | A | O |
| ATOM | 2404 | CG2 | THR | A | 363 | 25.578 | 26.742 | 35.066 | 1.00 | 16.07 | A | C |
| ATOM | 2405 | C | THR | A | 363 | 21.839 | 27.225 | 34.337 | 1.00 | 30.86 | A | C |
| ATOM | 2406 | O | THR | A | 363 | 21.091 | 28.116 | 34.768 | 1.00 | 30.86 | A | O |
| ATOM | 2407 | N | THR | A | 364 | 21.398 | 26.015 | 33.999 | 1.00 | 39.51 | A | N |
| ATOM | 2408 | CA | THR | A | 364 | 19.979 | 25.695 | 34.138 | 1.00 | 39.51 | A | C |
| ATOM | 2409 | CB | THR | A | 364 | 19.614 | 24.406 | 33.382 | 1.00 | 39.12 | A | C |
| ATOM | 2410 | OG1 | THR | A | 364 | 20.506 | 23.360 | 33.777 | 1.00 | 39.12 | A | O |
| ATOM | 2411 | CG2 | THR | A | 364 | 19.715 | 24.629 | 31.864 | 1.00 | 39.12 | A | C |
| ATOM | 2412 | C | THR | A | 364 | 19.659 | 25.551 | 35.613 | 1.00 | 39.51 | A | C |
| ATOM | 2413 | O | THR | A | 364 | 18.537 | 25.821 | 36.039 | 1.00 | 39.51 | A | O |
| ATOM | 2414 | N | GLN | A | 365 | 20.666 | 25.132 | 36.379 | 1.00 | 36.75 | A | N |
| ATOM | 2415 | CA | GLN | A | 365 | 20.573 | 24.962 | 37.833 | 1.00 | 36.75 | A | C |
| ATOM | 2416 | CB | GLN | A | 365 | 21.894 | 24.367 | 38.346 | 1.00 | 47.16 | A | C |
| ATOM | 2417 | CG | GLN | A | 365 | 22.070 | 24.335 | 39.862 | 1.00 | 47.16 | A | C |
| ATOM | 2418 | CD | GLN | A | 365 | 21.009 | 23.497 | 40.549 | 1.00 | 47.16 | A | C |
| ATOM | 2419 | OE1 | GLN | A | 365 | 20.071 | 23.019 | 39.897 | 1.00 | 47.16 | A | O |
| ATOM | 2420 | NE2 | GLN | A | 365 | 21.138 | 23.319 | 41.872 | 1.00 | 47.16 | A | N |
| ATOM | 2421 | C | GLN | A | 365 | 20.365 | 26.339 | 38.450 | 1.00 | 36.75 | A | C |
| ATOM | 2422 | O | GLN | A | 365 | 19.545 | 26.530 | 39.339 | 1.00 | 36.75 | A | O |
| ATOM | 2423 | N | GLU | A | 366 | 21.133 | 27.292 | 37.946 | 1.00 | 37.67 | A | N |
| ATOM | 2424 | CA | GLU | A | 366 | 21.102 | 28.665 | 38.403 | 1.00 | 37.67 | A | C |
| ATOM | 2425 | CB | GLU | A | 366 | 22.240 | 29.422 | 37.694 | 1.00 | 37.36 | A | C |
| ATOM | 2426 | CG | GLU | A | 366 | 22.602 | 30.784 | 38.261 | 1.00 | 37.36 | A | C |
| ATOM | 2427 | CD | GLU | A | 366 | 24.055 | 31.181 | 37.966 | 1.00 | 37.36 | A | C |
| ATOM | 2428 | OE1 | GLU | A | 366 | 24.463 | 32.297 | 38.366 | 1.00 | 37.36 | A | O |
| ATOM | 2429 | OE2 | GLU | A | 366 | 24.791 | 30.374 | 37.346 | 1.00 | 37.36 | A | O |
| ATOM | 2430 | C | GLU | A | 366 | 19.752 | 29.304 | 38.108 | 1.00 | 37.67 | A | C |
| ATOM | 2431 | O | GLU | A | 366 | 19.254 | 30.105 | 38.904 | 1.00 | 37.67 | A | O |
| ATOM | 2432 | N | LEU | A | 367 | 19.152 | 28.927 | 36.981 | 1.00 | 25.31 | A | N |
| ATOM | 2433 | CA | LEU | A | 367 | 17.872 | 29.496 | 36.550 | 1.00 | 25.31 | A | C |
| ATOM | 2434 | CB | LEU | A | 367 | 17.909 | 29.693 | 35.029 | 1.00 | 31.66 | A | C |
| ATOM | 2435 | CG | LEU | A | 367 | 18.921 | 30.735 | 34.509 | 1.00 | 31.66 | A | C |
| ATOM | 2436 | CD1 | LEU | A | 367 | 19.415 | 30.364 | 33.113 | 1.00 | 31.66 | A | C |
| ATOM | 2437 | CD2 | LEU | A | 367 | 18.272 | 32.117 | 34.512 | 1.00 | 31.66 | A | C |
| ATOM | 2438 | C | LEU | A | 367 | 16.617 | 28.704 | 36.947 | 1.00 | 25.31 | A | C |
| ATOM | 2439 | O | LEU | A | 367 | 15.485 | 29.176 | 36.778 | 1.00 | 25.31 | A | O |
| ATOM | 2440 | N | SER | A | 368 | 16.825 | 27.513 | 37.496 | 1.00 | 20.65 | A | N |
| ATOM | 2441 | CA | SER | A | 368 | 15.730 | 26.652 | 37.890 | 1.00 | 20.65 | A | C |
| ATOM | 2442 | CB | SER | A | 368 | 16.280 | 25.457 | 38.653 | 1.00 | 28.35 | A | C |
| ATOM | 2443 | OG | SER | A | 368 | 17.176 | 25.873 | 39.652 | 1.00 | 28.35 | A | O |
| ATOM | 2444 | C | SER | A | 368 | 14.605 | 27.318 | 38.669 | 1.00 | 20.65 | A | C |
| ATOM | 2445 | O | SER | A | 368 | 13.446 | 26.962 | 38.501 | 1.00 | 20.65 | A | O |
| ATOM | 2446 | N | SER | A | 369 | 14.931 | 28.284 | 39.516 | 1.00 | 34.66 | A | N |
| ATOM | 2447 | CA | SER | A | 369 | 13.905 | 28.979 | 40.295 | 1.00 | 34.66 | A | C |
| ATOM | 2448 | CB | SER | A | 369 | 14.541 | 30.112 | 41.106 | 1.00 | 33.79 | A | C |
| ATOM | 2449 | OG | SER | A | 369 | 15.471 | 30.836 | 40.320 | 1.00 | 33.79 | A | O |
| ATOM | 2450 | C | SER | A | 369 | 12.808 | 29.548 | 39.400 | 1.00 | 34.66 | A | C |
| ATOM | 2451 | O | SER | A | 369 | 11.645 | 29.612 | 39.789 | 1.00 | 34.66 | A | O |

| | | | | | | | | | | | | |
|---|---|---|---|---|---|---|---|---|---|---|---|---|
| ATOM | 2452 | N | ASN | A | 370 | 13.191 | 29.951 | 38.194 | 1.00 | 35.26 | A | N |
| ATOM | 2453 | CA | ASN | A | 370 | 12.256 | 30.550 | 37.247 | 1.00 | 35.26 | A | C |
| ATOM | 2454 | CB | ASN | A | 370 | 11.783 | 31.898 | 37.776 | 1.00 | 37.19 | A | C |
| ATOM | 2455 | CG | ASN | A | 370 | 10.816 | 32.572 | 36.848 | 1.00 | 37.19 | A | C |
| ATOM | 2456 | OD1 | ASN | A | 370 | 10.690 | 33.790 | 36.866 | 1.00 | 37.19 | A | O |
| ATOM | 2457 | ND2 | ASN | A | 370 | 10.111 | 31.787 | 36.033 | 1.00 | 37.19 | A | N |
| ATOM | 2458 | C | ASN | A | 370 | 12.961 | 30.739 | 35.900 | 1.00 | 35.26 | A | C |
| ATOM | 2459 | O | ASN | A | 370 | 13.481 | 31.814 | 35.599 | 1.00 | 35.26 | A | O |
| ATOM | 2460 | N | PRO | A | 371 | 12.970 | 29.686 | 35.066 | 1.00 | 34.97 | A | N |
| ATOM | 2461 | CD | PRO | A | 371 | 12.208 | 28.449 | 35.327 | 1.00 | 15.23 | A | C |
| ATOM | 2462 | CA | PRO | A | 371 | 13.594 | 29.639 | 33.736 | 1.00 | 34.97 | A | C |
| ATOM | 2463 | CB | PRO | A | 371 | 13.082 | 28.315 | 33.169 | 1.00 | 15.23 | A | C |
| ATOM | 2464 | CG | PRO | A | 371 | 12.865 | 27.482 | 34.402 | 1.00 | 15.23 | A | C |
| ATOM | 2465 | C | PRO | A | 371 | 13.303 | 30.814 | 32.799 | 1.00 | 34.97 | A | C |
| ATOM | 2466 | O | PRO | A | 371 | 14.218 | 31.420 | 32.255 | 1.00 | 34.97 | A | O |
| ATOM | 2467 | N | PRO | A | 372 | 12.021 | 31.149 | 32.593 | 1.00 | 27.53 | A | N |
| ATOM | 2468 | CD | PRO | A | 372 | 10.793 | 30.557 | 33.143 | 1.00 | 21.88 | A | C |
| ATOM | 2469 | CA | PRO | A | 372 | 11.686 | 32.259 | 31.702 | 1.00 | 27.53 | A | C |
| ATOM | 2470 | CB | PRO | A | 372 | 10.156 | 32.294 | 31.749 | 1.00 | 21.88 | A | C |
| ATOM | 2471 | CG | PRO | A | 372 | 9.834 | 31.717 | 33.051 | 1.00 | 21.88 | A | C |
| ATOM | 2472 | C | PRO | A | 372 | 12.321 | 33.612 | 31.991 | 1.00 | 27.53 | A | C |
| ATOM | 2473 | O | PRO | A | 372 | 11.998 | 34.598 | 31.336 | 1.00 | 27.53 | A | O |
| ATOM | 2474 | N | LEU | A | 373 | 13.216 | 33.673 | 32.968 | 1.00 | 34.78 | A | N |
| ATOM | 2475 | CA | LEU | A | 373 | 13.877 | 34.938 | 33.266 | 1.00 | 34.78 | A | C |
| ATOM | 2476 | CB | LEU | A | 373 | 14.281 | 35.036 | 34.738 | 1.00 | 28.61 | A | C |
| ATOM | 2477 | CG | LEU | A | 373 | 13.158 | 35.385 | 35.718 | 1.00 | 28.61 | A | C |
| ATOM | 2478 | CD1 | LEU | A | 373 | 13.753 | 35.690 | 37.099 | 1.00 | 28.61 | A | C |
| ATOM | 2479 | CD2 | LEU | A | 373 | 12.377 | 36.588 | 35.190 | 1.00 | 28.61 | A | C |
| ATOM | 2480 | C | LEU | A | 373 | 15.104 | 35.062 | 32.391 | 1.00 | 34.78 | A | C |
| ATOM | 2481 | O | LEU | A | 373 | 15.615 | 36.159 | 32.178 | 1.00 | 34.78 | A | O |
| ATOM | 2482 | N | ALA | A | 374 | 15.573 | 33.923 | 31.890 | 1.00 | 50.14 | A | N |
| ATOM | 2483 | CA | ALA | A | 374 | 16.729 | 33.901 | 31.005 | 1.00 | 50.14 | A | C |
| ATOM | 2484 | CB | ALA | A | 374 | 16.844 | 32.545 | 30.330 | 1.00 | 36.86 | A | C |
| ATOM | 2485 | C | ALA | A | 374 | 16.555 | 34.994 | 29.956 | 1.00 | 50.14 | A | C |
| ATOM | 2486 | O | ALA | A | 374 | 17.509 | 35.695 | 29.605 | 1.00 | 50.14 | A | O |
| ATOM | 2487 | N | THR | A | 375 | 15.317 | 35.142 | 29.486 | 1.00 | 34.91 | A | N |
| ATOM | 2488 | CA | THR | A | 375 | 14.955 | 36.130 | 28.466 | 1.00 | 34.91 | A | C |
| ATOM | 2489 | CB | THR | A | 375 | 13.418 | 36.058 | 28.161 | 1.00 | 33.40 | A | C |
| ATOM | 2490 | OG1 | THR | A | 375 | 12.669 | 36.663 | 29.229 | 1.00 | 33.40 | A | O |
| ATOM | 2491 | CG2 | THR | A | 375 | 12.976 | 34.605 | 28.018 | 1.00 | 33.40 | A | C |
| ATOM | 2492 | C | THR | A | 375 | 15.314 | 37.561 | 28.864 | 1.00 | 34.91 | A | C |
| ATOM | 2493 | O | THR | A | 375 | 15.206 | 38.485 | 28.067 | 1.00 | 34.91 | A | O |
| ATOM | 2494 | N | ILE | A | 376 | 15.705 | 37.756 | 30.112 | 1.00 | 31.61 | A | N |
| ATOM | 2495 | CA | ILE | A | 376 | 16.104 | 39.079 | 30.548 | 1.00 | 31.61 | A | C |
| ATOM | 2496 | CB | ILE | A | 376 | 15.224 | 39.602 | 31.676 | 1.00 | 22.89 | A | C |
| ATOM | 2497 | CG2 | ILE | A | 376 | 15.783 | 40.916 | 32.179 | 1.00 | 22.89 | A | C |
| ATOM | 2498 | CG1 | ILE | A | 376 | 13.790 | 39.783 | 31.186 | 1.00 | 22.89 | A | C |
| ATOM | 2499 | CD1 | ILE | A | 376 | 12.895 | 40.491 | 32.183 | 1.00 | 22.89 | A | C |
| ATOM | 2500 | C | ILE | A | 376 | 17.521 | 38.954 | 31.061 | 1.00 | 31.61 | A | C |
| ATOM | 2501 | O | ILE | A | 376 | 18.372 | 39.787 | 30.776 | 1.00 | 31.61 | A | O |
| ATOM | 2502 | N | LEU | A | 377 | 17.772 | 37.885 | 31.799 | 1.00 | 36.14 | A | N |
| ATOM | 2503 | CA | LEU | A | 377 | 19.085 | 37.654 | 32.357 | 1.00 | 36.14 | A | C |
| ATOM | 2504 | CB | LEU | A | 377 | 19.031 | 36.417 | 33.255 | 1.00 | 28.44 | A | C |
| ATOM | 2505 | CG | LEU | A | 377 | 18.157 | 36.724 | 34.477 | 1.00 | 28.44 | A | C |
| ATOM | 2506 | CD1 | LEU | A | 377 | 17.746 | 35.452 | 35.170 | 1.00 | 28.44 | A | C |
| ATOM | 2507 | CD2 | LEU | A | 377 | 18.917 | 37.656 | 35.419 | 1.00 | 28.44 | A | C |
| ATOM | 2508 | C | LEU | A | 377 | 20.185 | 37.557 | 31.306 | 1.00 | 36.14 | A | C |
| ATOM | 2509 | O | LEU | A | 377 | 21.228 | 38.177 | 31.467 | 1.00 | 36.14 | A | O |
| ATOM | 2510 | N | ILE | A | 378 | 19.963 | 36.803 | 30.231 | 1.00 | 32.93 | A | N |
| ATOM | 2511 | CA | ILE | A | 378 | 20.973 | 36.685 | 29.166 | 1.00 | 32.93 | A | C |
| ATOM | 2512 | CB | ILE | A | 378 | 20.952 | 35.299 | 28.491 | 1.00 | 11.15 | A | C |
| ATOM | 2513 | CG2 | ILE | A | 378 | 22.005 | 35.254 | 27.423 | 1.00 | 11.15 | A | C |
| ATOM | 2514 | CG1 | ILE | A | 378 | 21.196 | 34.188 | 29.504 | 1.00 | 11.15 | A | C |

| | | | | | | | | | | | | |
|---|---|---|---|---|---|---|---|---|---|---|---|---|
| ATOM | 2515 | CD1 | ILE | A | 378 | 20.004 | 33.880 | 30.348 | 1.00 | 11.15 | A | C |
| ATOM | 2516 | C | ILE | A | 378 | 20.674 | 37.707 | 28.063 | 1.00 | 32.93 | A | C |
| ATOM | 2517 | O | ILE | A | 378 | 19.805 | 37.473 | 27.228 | 1.00 | 32.93 | A | O |
| ATOM | 2518 | N | PRO | A | 379 | 21.385 | 38.853 | 28.043 | 1.00 | 50.49 | A | N |
| ATOM | 2519 | CD | PRO | A | 379 | 22.459 | 39.315 | 28.942 | 1.00 | 26.95 | A | C |
| ATOM | 2520 | CA | PRO | A | 379 | 21.113 | 39.846 | 26.995 | 1.00 | 50.49 | A | C |
| ATOM | 2521 | CB | PRO | A | 379 | 21.962 | 41.043 | 27.422 | 1.00 | 26.95 | A | C |
| ATOM | 2522 | CG | PRO | A | 379 | 23.112 | 40.418 | 28.135 | 1.00 | 26.95 | A | C |
| ATOM | 2523 | C | PRO | A | 379 | 21.408 | 39.339 | 25.576 | 1.00 | 50.49 | A | C |
| ATOM | 2524 | O | PRO | A | 379 | 22.232 | 38.434 | 25.379 | 1.00 | 50.49 | A | O |
| ATOM | 2525 | N | PRO | A | 380 | 20.722 | 39.909 | 24.573 | 1.00 | 30.62 | A | N |
| ATOM | 2526 | CD | PRO | A | 380 | 19.705 | 40.970 | 24.684 | 1.00 | 29.17 | A | C |
| ATOM | 2527 | CA | PRO | A | 380 | 20.906 | 39.509 | 23.179 | 1.00 | 30.62 | A | C |
| ATOM | 2528 | CB | PRO | A | 380 | 20.255 | 40.649 | 22.418 | 1.00 | 29.17 | A | C |
| ATOM | 2529 | CG | PRO | A | 380 | 19.075 | 40.966 | 23.304 | 1.00 | 29.17 | A | C |
| ATOM | 2530 | C | PRO | A | 380 | 22.344 | 39.248 | 22.780 | 1.00 | 30.62 | A | C |
| ATOM | 2531 | O | PRO | A | 380 | 22.654 | 38.170 | 22.276 | 1.00 | 30.62 | A | O |
| ATOM | 2532 | N | HIS | A | 381 | 23.234 | 40.203 | 23.023 | 1.00 | 21.55 | A | N |
| ATOM | 2533 | CA | HIS | A | 381 | 24.620 | 39.995 | 22.646 | 1.00 | 21.55 | A | C |
| ATOM | 2534 | CB | HIS | A | 381 | 25.397 | 41.309 | 22.790 | 1.00 | 30.89 | A | C |
| ATOM | 2535 | CG | HIS | A | 381 | 25.501 | 41.809 | 24.197 | 1.00 | 30.89 | A | C |
| ATOM | 2536 | CD2 | HIS | A | 381 | 24.703 | 42.640 | 24.910 | 1.00 | 30.89 | A | C |
| ATOM | 2537 | ND1 | HIS | A | 381 | 26.507 | 41.416 | 25.054 | 1.00 | 30.89 | A | N |
| ATOM | 2538 | CE1 | HIS | A | 381 | 26.324 | 41.982 | 26.234 | 1.00 | 30.89 | A | C |
| ATOM | 2539 | NE2 | HIS | A | 381 | 25.237 | 42.728 | 26.174 | 1.00 | 30.89 | A | N |
| ATOM | 2540 | C | HIS | A | 381 | 25.284 | 38.855 | 23.428 | 1.00 | 21.55 | A | C |
| ATOM | 2541 | O | HIS | A | 381 | 24.680 | 38.332 | 24.406 | 1.00 | 21.55 | A | O |
| ATOM | 2542 | OXT | HIS | A | 381 | 26.416 | 38.490 | 23.029 | 1.00 | 30.89 | A | O |
| TER | 2543 | | HIS | A | 381 | | | | | | A | |
| ATOM | 2544 | CB | VAL | B | 37 | 15.131 | 80.790 | 77.616 | 1.00 | 47.59 | B | C |
| ATOM | 2545 | CG1 | VAL | B | 37 | 14.611 | 81.253 | 76.246 | 1.00 | 47.59 | B | C |
| ATOM | 2546 | CG2 | VAL | B | 37 | 16.523 | 81.372 | 77.882 | 1.00 | 47.59 | B | C |
| ATOM | 2547 | C | VAL | B | 37 | 12.855 | 80.373 | 78.592 | 1.00 | 57.05 | B | C |
| ATOM | 2548 | O | VAL | B | 37 | 11.750 | 80.925 | 78.640 | 1.00 | 57.05 | B | O |
| ATOM | 2549 | N | VAL | B | 37 | 14.721 | 81.136 | 80.095 | 1.00 | 57.05 | B | N |
| ATOM | 2550 | CA | VAL | B | 37 | 14.127 | 81.225 | 78.722 | 1.00 | 57.05 | B | C |
| ATOM | 2551 | N | THR | B | 38 | 12.982 | 79.048 | 78.440 | 1.00 | 36.93 | B | N |
| ATOM | 2552 | CA | THR | B | 38 | 11.781 | 78.207 | 78.316 | 1.00 | 36.93 | B | C |
| ATOM | 2553 | CB | THR | B | 38 | 12.105 | 76.829 | 77.671 | 1.00 | 52.44 | B | C |
| ATOM | 2554 | OG1 | THR | B | 38 | 12.653 | 77.028 | 76.359 | 1.00 | 52.44 | B | O |
| ATOM | 2555 | CG2 | THR | B | 38 | 10.833 | 75.989 | 77.534 | 1.00 | 52.44 | B | C |
| ATOM | 2556 | C | THR | B | 38 | 11.032 | 77.986 | 79.637 | 1.00 | 36.93 | B | C |
| ATOM | 2557 | O | THR | B | 38 | 11.623 | 77.626 | 80.659 | 1.00 | 36.93 | B | O |
| ATOM | 2558 | N | THR | B | 39 | 9.723 | 78.235 | 79.600 | 1.00 | 28.99 | B | N |
| ATOM | 2559 | CA | THR | B | 39 | 8.850 | 78.072 | 80.765 | 1.00 | 28.99 | B | C |
| ATOM | 2560 | CB | THR | B | 39 | 8.189 | 79.390 | 81.191 | 1.00 | 45.61 | B | C |
| ATOM | 2561 | OG1 | THR | B | 39 | 9.196 | 80.374 | 81.464 | 1.00 | 45.61 | B | O |
| ATOM | 2562 | CG2 | THR | B | 39 | 7.352 | 79.165 | 82.434 | 1.00 | 45.61 | B | C |
| ATOM | 2563 | C | THR | B | 39 | 7.739 | 77.121 | 80.370 | 1.00 | 28.99 | B | C |
| ATOM | 2564 | O | THR | B | 39 | 7.103 | 77.288 | 79.327 | 1.00 | 28.99 | B | O |
| ATOM | 2565 | N | VAL | B | 40 | 7.482 | 76.140 | 81.223 | 1.00 | 28.06 | B | N |
| ATOM | 2566 | CA | VAL | B | 40 | 6.473 | 75.142 | 80.917 | 1.00 | 28.06 | B | C |
| ATOM | 2567 | CB | VAL | B | 40 | 7.170 | 73.902 | 80.269 | 1.00 | 22.26 | B | C |
| ATOM | 2568 | CG1 | VAL | B | 40 | 7.757 | 72.993 | 81.341 | 1.00 | 22.26 | B | C |
| ATOM | 2569 | CG2 | VAL | B | 40 | 6.203 | 73.165 | 79.373 | 1.00 | 22.26 | B | C |
| ATOM | 2570 | C | VAL | B | 40 | 5.747 | 74.755 | 82.200 | 1.00 | 28.06 | B | C |
| ATOM | 2571 | O | VAL | B | 40 | 6.197 | 75.090 | 83.293 | 1.00 | 28.06 | B | O |
| ATOM | 2572 | N | VAL | B | 41 | 4.617 | 74.069 | 82.062 | 1.00 | 15.23 | B | N |
| ATOM | 2573 | CA | VAL | B | 41 | 3.838 | 73.643 | 83.214 | 1.00 | 15.23 | B | C |
| ATOM | 2574 | CB | VAL | B | 41 | 2.356 | 74.057 | 83.055 | 1.00 | 16.51 | B | C |
| ATOM | 2575 | CG1 | VAL | B | 41 | 1.541 | 73.576 | 84.245 | 1.00 | 16.51 | B | C |
| ATOM | 2576 | CG2 | VAL | B | 41 | 2.256 | 75.580 | 82.938 | 1.00 | 16.51 | B | C |
| ATOM | 2577 | C | VAL | B | 41 | 3.981 | 72.134 | 83.305 | 1.00 | 15.23 | B | C |

| | | | | | | | | | | | | |
|---|---|---|---|---|---|---|---|---|---|---|---|---|
| ATOM | 2578 | O | VAL | B | 41 | 3.457 | 71.408 | 82.481 | 1.00 | 15.23 | B | O |
| ATOM | 2579 | N | ALA | B | 42 | 4.702 | 71.669 | 84.318 | 1.00 | 29.57 | B | N |
| ATOM | 2580 | CA | ALA | B | 42 | 4.972 | 70.248 | 84.480 | 1.00 | 29.57 | B | C |
| ATOM | 2581 | CB | ALA | B | 42 | 6.484 | 70.009 | 84.456 | 1.00 | 17.99 | B | C |
| ATOM | 2582 | C | ALA | B | 42 | 4.384 | 69.597 | 85.709 | 1.00 | 29.57 | B | C |
| ATOM | 2583 | O | ALA | B | 42 | 4.047 | 70.263 | 86.687 | 1.00 | 29.57 | B | O |
| ATOM | 2584 | N | THR | B | 43 | 4.303 | 68.270 | 85.641 | 1.00 | 30.78 | B | N |
| ATOM | 2585 | CA | THR | B | 43 | 3.759 | 67.442 | 86.702 | 1.00 | 30.78 | B | C |
| ATOM | 2586 | CB | THR | B | 43 | 2.829 | 66.375 | 86.118 | 1.00 | 32.42 | B | C |
| ATOM | 2587 | OG1 | THR | B | 43 | 1.763 | 67.019 | 85.416 | 1.00 | 32.42 | B | O |
| ATOM | 2588 | CG2 | THR | B | 43 | 2.265 | 65.476 | 87.214 | 1.00 | 32.42 | B | C |
| ATOM | 2589 | C | THR | B | 43 | 4.884 | 66.742 | 87.454 | 1.00 | 30.78 | B | C |
| ATOM | 2590 | O | THR | B | 43 | 5.773 | 66.130 | 86.844 | 1.00 | 30.78 | B | O |
| ATOM | 2591 | N | PRO | B | 44 | 4.880 | 66.846 | 88.789 | 1.00 | 26.31 | B | N |
| ATOM | 2592 | CD | PRO | B | 44 | 4.140 | 67.872 | 89.543 | 1.00 | 30.53 | B | C |
| ATOM | 2593 | CA | PRO | B | 44 | 5.889 | 66.218 | 89.644 | 1.00 | 26.31 | B | C |
| ATOM | 2594 | CB | PRO | B | 44 | 5.461 | 66.650 | 91.037 | 1.00 | 30.53 | B | C |
| ATOM | 2595 | CG | PRO | B | 44 | 5.000 | 68.040 | 90.786 | 1.00 | 30.53 | B | C |
| ATOM | 2596 | C | PRO | B | 44 | 5.948 | 64.704 | 89.496 | 1.00 | 26.31 | B | C |
| ATOM | 2597 | O | PRO | B | 44 | 4.915 | 64.033 | 89.432 | 1.00 | 26.31 | B | O |
| ATOM | 2598 | N | GLY | B | 45 | 7.174 | 64.184 | 89.443 | 1.00 | 30.08 | B | N |
| ATOM | 2599 | CA | GLY | B | 45 | 7.392 | 62.757 | 89.287 | 1.00 | 30.08 | B | C |
| ATOM | 2600 | C | GLY | B | 45 | 6.617 | 61.959 | 90.306 | 1.00 | 30.08 | B | C |
| ATOM | 2601 | O | GLY | B | 45 | 5.934 | 60.988 | 89.971 | 1.00 | 30.08 | B | O |
| ATOM | 2602 | N | ALA | B | 46 | 6.717 | 62.377 | 91.560 | 1.00 | 42.85 | B | N |
| ATOM | 2603 | CA | ALA | B | 46 | 6.022 | 61.686 | 92.631 | 1.00 | 42.85 | B | C |
| ATOM | 2604 | CB | ALA | B | 46 | 6.904 | 61.634 | 93.877 | 1.00 | 55.45 | B | C |
| ATOM | 2605 | C | ALA | B | 46 | 4.703 | 62.387 | 92.947 | 1.00 | 42.85 | B | C |
| ATOM | 2606 | O | ALA | B | 46 | 4.094 | 63.028 | 92.083 | 1.00 | 42.85 | B | O |
| ATOM | 2607 | N | GLY | B | 47 | 4.269 | 62.243 | 94.194 | 1.00 | 62.50 | B | N |
| ATOM | 2608 | CA | GLY | B | 47 | 3.036 | 62.858 | 94.655 | 1.00 | 62.50 | B | C |
| ATOM | 2609 | C | GLY | B | 47 | 1.830 | 62.804 | 93.730 | 1.00 | 62.50 | B | C |
| ATOM | 2610 | O | GLY | B | 47 | 1.783 | 62.030 | 92.772 | 1.00 | 62.50 | B | O |
| ATOM | 2611 | N | PRO | B | 48 | 0.814 | 63.631 | 94.013 | 1.00 | 87.34 | B | N |
| ATOM | 2612 | CD | PRO | B | 48 | 0.832 | 64.632 | 95.104 | 1.00 | 67.66 | B | C |
| ATOM | 2613 | CA | PRO | B | 48 | -0.423 | 63.715 | 93.218 | 1.00 | 87.34 | B | C |
| ATOM | 2614 | CB | PRO | B | 48 | -1.364 | 64.446 | 94.158 | 1.00 | 67.66 | B | C |
| ATOM | 2615 | CG | PRO | B | 48 | -0.397 | 65.461 | 94.819 | 1.00 | 67.66 | B | C |
| ATOM | 2616 | C | PRO | B | 48 | -0.083 | 64.559 | 91.987 | 1.00 | 87.34 | B | C |
| ATOM | 2617 | O | PRO | B | 48 | 1.054 | 65.009 | 91.861 | 1.00 | 87.34 | B | O |
| ATOM | 2618 | N | ASP | B | 49 | -1.033 | 64.803 | 91.092 | 1.00 | 46.29 | B | N |
| ATOM | 2619 | CA | ASP | B | 49 | -0.702 | 65.619 | 89.922 | 1.00 | 46.29 | B | C |
| ATOM | 2620 | CB | ASP | B | 49 | -1.410 | 65.089 | 88.669 | 1.00 | 50.78 | B | C |
| ATOM | 2621 | CG | ASP | B | 49 | -1.230 | 63.583 | 88.493 | 1.00 | 50.78 | B | C |
| ATOM | 2622 | OD1 | ASP | B | 49 | -1.193 | 63.105 | 87.327 | 1.00 | 50.78 | B | O |
| ATOM | 2623 | OD2 | ASP | B | 49 | -1.142 | 62.874 | 89.530 | 1.00 | 50.78 | B | O |
| ATOM | 2624 | C | ASP | B | 49 | -1.048 | 67.076 | 90.137 | 1.00 | 46.29 | B | C |
| ATOM | 2625 | O | ASP | B | 49 | -2.139 | 67.520 | 89.786 | 1.00 | 46.29 | B | O |
| ATOM | 2626 | N | ARG | B | 50 | -0.105 | 67.810 | 90.717 | 1.00 | 37.61 | B | N |
| ATOM | 2627 | CA | ARG | B | 50 | -0.282 | 69.230 | 90.984 | 1.00 | 37.61 | B | C |
| ATOM | 2628 | CB | ARG | B | 50 | -0.245 | 69.444 | 92.491 | 1.00 | 67.01 | B | C |
| ATOM | 2629 | CG | ARG | B | 50 | -1.177 | 68.404 | 93.041 | 1.00 | 67.01 | B | C |
| ATOM | 2630 | CD | ARG | B | 50 | -2.322 | 68.790 | 93.978 | 1.00 | 67.01 | B | C |
| ATOM | 2631 | NE | ARG | B | 50 | -1.789 | 68.453 | 95.324 | 1.00 | 67.01 | B | N |
| ATOM | 2632 | CZ | ARG | B | 50 | -2.140 | 67.402 | 96.184 | 1.00 | 67.01 | B | C |
| ATOM | 2633 | NH1 | ARG | B | 50 | -3.049 | 66.496 | 95.937 | 1.00 | 67.01 | B | N |
| ATOM | 2634 | NH2 | ARG | B | 50 | -1.535 | 67.272 | 97.349 | 1.00 | 67.01 | B | N |
| ATOM | 2635 | C | ARG | B | 50 | 0.792 | 70.001 | 90.236 | 1.00 | 37.61 | B | C |
| ATOM | 2636 | O | ARG | B | 50 | 1.805 | 70.417 | 90.808 | 1.00 | 37.61 | B | O |
| ATOM | 2637 | N | PRO | B | 51 | 0.590 | 70.172 | 88.916 | 1.00 | 33.49 | B | N |
| ATOM | 2638 | CD | PRO | B | 51 | -0.438 | 69.496 | 88.113 | 1.00 | 30.36 | B | C |
| ATOM | 2639 | CA | PRO | B | 51 | 1.519 | 70.888 | 88.041 | 1.00 | 33.49 | B | C |
| ATOM | 2640 | CB | PRO | B | 51 | 0.909 | 70.715 | 86.643 | 1.00 | 30.36 | B | C |

| | | | | | | | | | | | | |
|---|---|---|---|---|---|---|---|---|---|---|---|---|
| ATOM | 2641 | CG | PRO | B | 51 | -0.493 | 70.368 | 86.894 | 1.00 | 30.36 | B | C |
| ATOM | 2642 | C | PRO | B | 51 | 1.851 | 72.325 | 88.366 | 1.00 | 33.49 | B | C |
| ATOM | 2643 | O | PRO | B | 51 | 0.983 | 73.120 | 88.708 | 1.00 | 33.49 | B | O |
| ATOM | 2644 | N | GLN | B | 52 | 3.133 | 72.648 | 88.260 | 1.00 | 31.33 | B | N |
| ATOM | 2645 | CA | GLN | B | 52 | 3.578 | 73.993 | 88.526 | 1.00 | 31.33 | B | C |
| ATOM | 2646 | CB | GLN | B | 52 | 4.326 | 74.062 | 89.858 | 1.00 | 58.78 | B | C |
| ATOM | 2647 | CG | GLN | B | 52 | 4.630 | 75.501 | 90.351 | 1.00 | 58.78 | B | C |
| ATOM | 2648 | CD | GLN | B | 52 | 3.527 | 76.529 | 90.026 | 1.00 | 58.78 | B | C |
| ATOM | 2649 | OE1 | GLN | B | 52 | 2.326 | 76.214 | 90.087 | 1.00 | 58.78 | B | O |
| ATOM | 2650 | NE2 | GLN | B | 52 | 3.936 | 77.768 | 89.693 | 1.00 | 58.78 | B | N |
| ATOM | 2651 | C | GLN | B | 52 | 4.438 | 74.471 | 87.386 | 1.00 | 31.33 | B | C |
| ATOM | 2652 | O | GLN | B | 52 | 4.856 | 73.666 | 86.568 | 1.00 | 31.33 | B | O |
| ATOM | 2653 | N | GLU | B | 53 | 4.670 | 75.781 | 87.304 | 1.00 | 40.33 | B | N |
| ATOM | 2654 | CA | GLU | B | 53 | 5.511 | 76.324 | 86.249 | 1.00 | 40.33 | B | C |
| ATOM | 2655 | CB | GLU | B | 53 | 5.248 | 77.836 | 86.073 | 1.00 | 63.35 | B | C |
| ATOM | 2656 | CG | GLU | B | 53 | 3.899 | 78.137 | 85.354 | 1.00 | 63.35 | B | C |
| ATOM | 2657 | CD | GLU | B | 53 | 3.180 | 79.441 | 85.806 | 1.00 | 63.35 | B | C |
| ATOM | 2658 | OE1 | GLU | B | 53 | 3.442 | 79.916 | 86.937 | 1.00 | 63.35 | B | O |
| ATOM | 2659 | OE2 | GLU | B | 53 | 2.329 | 79.974 | 85.036 | 1.00 | 63.35 | B | O |
| ATOM | 2660 | C | GLU | B | 53 | 6.960 | 76.019 | 86.586 | 1.00 | 40.33 | B | C |
| ATOM | 2661 | O | GLU | B | 53 | 7.353 | 76.033 | 87.755 | 1.00 | 40.33 | B | O |
| ATOM | 2662 | N | VAL | B | 54 | 7.722 | 75.661 | 85.559 | 1.00 | 27.34 | B | N |
| ATOM | 2663 | CA | VAL | B | 54 | 9.132 | 75.357 | 85.699 | 1.00 | 27.34 | B | C |
| ATOM | 2664 | CB | VAL | B | 54 | 9.388 | 73.854 | 85.595 | 1.00 | 15.49 | B | C |
| ATOM | 2665 | CG1 | VAL | B | 54 | 10.873 | 73.565 | 85.722 | 1.00 | 15.49 | B | C |
| ATOM | 2666 | CG2 | VAL | B | 54 | 8.619 | 73.141 | 86.685 | 1.00 | 15.49 | B | C |
| ATOM | 2667 | C | VAL | B | 54 | 9.801 | 76.087 | 84.565 | 1.00 | 27.34 | B | C |
| ATOM | 2668 | O | VAL | B | 54 | 9.280 | 76.097 | 83.442 | 1.00 | 27.34 | B | O |
| ATOM | 2669 | N | SER | B | 55 | 10.925 | 76.735 | 84.866 | 1.00 | 51.28 | B | N |
| ATOM | 2670 | CA | SER | B | 55 | 11.678 | 77.468 | 83.847 | 1.00 | 51.28 | B | C |
| ATOM | 2671 | CB | SER | B | 55 | 11.601 | 78.976 | 84.108 | 1.00 | 57.71 | B | C |
| ATOM | 2672 | OG | SER | B | 55 | 10.302 | 79.477 | 83.805 | 1.00 | 57.71 | B | O |
| ATOM | 2673 | C | SER | B | 55 | 13.122 | 77.013 | 83.778 | 1.00 | 51.28 | B | C |
| ATOM | 2674 | O | SER | B | 55 | 13.793 | 76.876 | 84.802 | 1.00 | 51.28 | B | O |
| ATOM | 2675 | N | TYR | B | 56 | 13.592 | 76.759 | 82.565 | 1.00 | 40.91 | B | N |
| ATOM | 2676 | CA | TYR | B | 56 | 14.968 | 76.321 | 82.369 | 1.00 | 40.91 | B | C |
| ATOM | 2677 | CB | TYR | B | 56 | 15.025 | 74.801 | 82.227 | 1.00 | 18.59 | B | C |
| ATOM | 2678 | CG | TYR | B | 56 | 14.267 | 74.252 | 81.043 | 1.00 | 18.59 | B | C |
| ATOM | 2679 | CD1 | TYR | B | 56 | 14.812 | 74.289 | 79.761 | 1.00 | 18.59 | B | C |
| ATOM | 2680 | CE1 | TYR | B | 56 | 14.127 | 73.764 | 78.682 | 1.00 | 18.59 | B | C |
| ATOM | 2681 | CD2 | TYR | B | 56 | 13.005 | 73.674 | 81.207 | 1.00 | 18.59 | B | C |
| ATOM | 2682 | CE2 | TYR | B | 56 | 12.312 | 73.143 | 80.126 | 1.00 | 18.59 | B | C |
| ATOM | 2683 | CZ | TYR | B | 56 | 12.882 | 73.190 | 78.873 | 1.00 | 18.59 | B | C |
| ATOM | 2684 | OH | TYR | B | 56 | 12.217 | 72.631 | 77.815 | 1.00 | 18.59 | B | O |
| ATOM | 2685 | C | TYR | B | 56 | 15.584 | 76.985 | 81.154 | 1.00 | 40.91 | B | C |
| ATOM | 2686 | O | TYR | B | 56 | 14.890 | 77.363 | 80.193 | 1.00 | 40.91 | B | O |
| ATOM | 2687 | N | THR | B | 57 | 16.903 | 77.107 | 81.203 | 1.00 | 45.57 | B | N |
| ATOM | 2688 | CA | THR | B | 57 | 17.654 | 77.753 | 80.143 | 1.00 | 45.57 | B | C |
| ATOM | 2689 | CB | THR | B | 57 | 18.056 | 79.151 | 80.624 | 1.00 | 48.29 | B | C |
| ATOM | 2690 | OG1 | THR | B | 57 | 18.588 | 79.907 | 79.534 | 1.00 | 48.29 | B | O |
| ATOM | 2691 | CG2 | THR | B | 57 | 19.096 | 79.036 | 81.727 | 1.00 | 48.29 | B | C |
| ATOM | 2692 | C | THR | B | 57 | 18.905 | 76.925 | 79.845 | 1.00 | 45.57 | B | C |
| ATOM | 2693 | O | THR | B | 57 | 19.072 | 75.838 | 80.396 | 1.00 | 45.57 | B | O |
| ATOM | 2694 | N | ASP | B | 58 | 19.773 | 77.452 | 78.982 | 1.00 | 34.14 | B | N |
| ATOM | 2695 | CA | ASP | B | 58 | 21.022 | 76.791 | 78.607 | 1.00 | 34.14 | B | C |
| ATOM | 2696 | CB | ASP | B | 58 | 22.002 | 76.775 | 79.785 | 1.00 | 54.91 | B | C |
| ATOM | 2697 | CG | ASP | B | 58 | 22.321 | 78.162 | 80.302 | 1.00 | 54.91 | B | C |
| ATOM | 2698 | OD1 | ASP | B | 58 | 22.388 | 79.118 | 79.485 | 1.00 | 54.91 | B | O |
| ATOM | 2699 | OD2 | ASP | B | 58 | 22.523 | 78.287 | 81.530 | 1.00 | 54.91 | B | O |
| ATOM | 2700 | C | ASP | B | 58 | 20.778 | 75.356 | 78.156 | 1.00 | 34.14 | B | C |
| ATOM | 2701 | O | ASP | B | 58 | 21.453 | 74.411 | 78.590 | 1.00 | 34.14 | B | O |
| ATOM | 2702 | N | THR | B | 59 | 19.809 | 75.189 | 77.280 | 1.00 | 40.00 | B | N |
| ATOM | 2703 | CA | THR | B | 59 | 19.512 | 73.864 | 76.807 | 1.00 | 40.00 | B | C |

| | | | | | | | | | | | | |
|---|---|---|---|---|---|---|---|---|---|---|---|---|
| ATOM | 2704 | CB | THR | B | 59 | 18.140 | 73.828 | 76.170 | 1.00 | 40.48 | B | C |
| ATOM | 2705 | OG1 | THR | B | 59 | 17.204 | 74.458 | 77.055 | 1.00 | 40.48 | B | O |
| ATOM | 2706 | CG2 | THR | B | 59 | 17.718 | 72.377 | 75.912 | 1.00 | 40.48 | B | C |
| ATOM | 2707 | C | THR | B | 59 | 20.550 | 73.434 | 75.783 | 1.00 | 40.00 | B | C |
| ATOM | 2708 | O | THR | B | 59 | 21.054 | 74.251 | 75.016 | 1.00 | 40.00 | B | O |
| ATOM | 2709 | N | LYS | B | 60 | 20.880 | 72.150 | 75.781 | 1.00 | 52.57 | B | N |
| ATOM | 2710 | CA | LYS | B | 60 | 21.843 | 71.630 | 74.824 | 1.00 | 52.57 | B | C |
| ATOM | 2711 | CB | LYS | B | 60 | 23.269 | 72.002 | 75.238 | 1.00 | 75.41 | B | C |
| ATOM | 2712 | CG | LYS | B | 60 | 23.659 | 71.539 | 76.626 | 1.00 | 75.41 | B | C |
| ATOM | 2713 | CD | LYS | B | 60 | 25.128 | 71.849 | 76.916 | 1.00 | 75.41 | B | C |
| ATOM | 2714 | CE | LYS | B | 60 | 25.433 | 73.360 | 76.862 | 1.00 | 75.41 | B | C |
| ATOM | 2715 | NZ | LYS | B | 60 | 25.021 | 74.125 | 78.094 | 1.00 | 75.41 | B | N |
| ATOM | 2716 | C | LYS | B | 60 | 21.705 | 70.118 | 74.738 | 1.00 | 52.57 | B | C |
| ATOM | 2717 | O | LYS | B | 60 | 21.409 | 69.445 | 75.726 | 1.00 | 52.57 | B | O |
| ATOM | 2718 | N | VAL | B | 61 | 21.913 | 69.597 | 73.540 | 1.00 | 37.90 | B | N |
| ATOM | 2719 | CA | VAL | B | 61 | 21.811 | 68.173 | 73.293 | 1.00 | 37.90 | B | C |
| ATOM | 2720 | CB | VAL | B | 61 | 21.588 | 67.919 | 71.811 | 1.00 | 36.26 | B | C |
| ATOM | 2721 | CG1 | VAL | B | 61 | 21.392 | 66.436 | 71.563 | 1.00 | 36.26 | B | C |
| ATOM | 2722 | CG2 | VAL | B | 61 | 20.390 | 68.738 | 71.339 | 1.00 | 36.26 | B | C |
| ATOM | 2723 | C | VAL | B | 61 | 23.062 | 67.435 | 73.728 | 1.00 | 37.90 | B | C |
| ATOM | 2724 | O | VAL | B | 61 | 24.170 | 67.842 | 73.397 | 1.00 | 37.90 | B | O |
| ATOM | 2725 | N | ILE | B | 62 | 22.882 | 66.340 | 74.461 | 1.00 | 40.49 | B | N |
| ATOM | 2726 | CA | ILE | B | 62 | 24.023 | 65.564 | 74.929 | 1.00 | 40.49 | B | C |
| ATOM | 2727 | CB | ILE | B | 62 | 24.086 | 65.536 | 76.474 | 1.00 | 52.01 | B | C |
| ATOM | 2728 | CG2 | ILE | B | 62 | 23.919 | 66.952 | 77.025 | 1.00 | 52.01 | B | C |
| ATOM | 2729 | CG1 | ILE | B | 62 | 22.966 | 64.689 | 77.056 | 1.00 | 52.01 | B | C |
| ATOM | 2730 | CD1 | ILE | B | 62 | 22.882 | 64.840 | 78.567 | 1.00 | 52.01 | B | C |
| ATOM | 2731 | C | ILE | B | 62 | 23.977 | 64.145 | 74.402 | 1.00 | 40.49 | B | C |
| ATOM | 2732 | O | ILE | B | 62 | 24.964 | 63.418 | 74.453 | 1.00 | 40.49 | B | O |
| ATOM | 2733 | N | GLY | B | 63 | 22.824 | 63.754 | 73.886 | 1.00 | 23.11 | B | N |
| ATOM | 2734 | CA | GLY | B | 63 | 22.674 | 62.412 | 73.363 | 1.00 | 23.11 | B | C |
| ATOM | 2735 | C | GLY | B | 63 | 21.504 | 62.414 | 72.416 | 1.00 | 23.11 | B | C |
| ATOM | 2736 | O | GLY | B | 63 | 20.506 | 63.081 | 72.657 | 1.00 | 23.11 | B | O |
| ATOM | 2737 | N | ASN | B | 64 | 21.614 | 61.663 | 71.333 | 1.00 | 52.26 | B | N |
| ATOM | 2738 | CA | ASN | B | 64 | 20.549 | 61.635 | 70.342 | 1.00 | 52.26 | B | C |
| ATOM | 2739 | CB | ASN | B | 64 | 20.910 | 62.566 | 69.182 | 1.00 | 99.59 | B | C |
| ATOM | 2740 | CG | ASN | B | 64 | 19.730 | 62.842 | 68.264 | 1.00 | 99.59 | B | C |
| ATOM | 2741 | OD1 | ASN | B | 64 | 19.314 | 61.986 | 67.451 | 1.00 | 99.59 | B | O |
| ATOM | 2742 | ND2 | ASN | B | 64 | 19.172 | 64.053 | 68.389 | 1.00 | 99.59 | B | N |
| ATOM | 2743 | C | ASN | B | 64 | 20.441 | 60.221 | 69.850 | 1.00 | 52.26 | B | C |
| ATOM | 2744 | O | ASN | B | 64 | 20.800 | 59.949 | 68.704 | 1.00 | 52.26 | B | O |
| ATOM | 2745 | N | GLY | B | 65 | 19.992 | 59.329 | 70.734 | 1.00 | 68.33 | B | N |
| ATOM | 2746 | CA | GLY | B | 65 | 19.840 | 57.931 | 70.390 | 1.00 | 68.33 | B | C |
| ATOM | 2747 | C | GLY | B | 65 | 18.635 | 57.558 | 69.534 | 1.00 | 68.33 | B | C |
| ATOM | 2748 | O | GLY | B | 65 | 18.123 | 58.349 | 68.713 | 1.00 | 68.33 | B | O |
| ATOM | 2749 | N | SER | B | 66 | 18.225 | 56.312 | 69.714 | 1.00 | 79.80 | B | N |
| ATOM | 2750 | CA | SER | B | 66 | 17.093 | 55.721 | 69.021 | 1.00 | 79.80 | B | C |
| ATOM | 2751 | CB | SER | B | 66 | 17.309 | 54.201 | 68.946 | 1.00 | 52.53 | B | C |
| ATOM | 2752 | OG | SER | B | 66 | 16.238 | 53.467 | 69.525 | 1.00 | 52.53 | B | O |
| ATOM | 2753 | C | SER | B | 66 | 15.817 | 56.084 | 69.811 | 1.00 | 79.80 | B | C |
| ATOM | 2754 | O | SER | B | 66 | 14.800 | 56.492 | 69.208 | 1.00 | 79.80 | B | O |
| ATOM | 2755 | N | PHE | B | 67 | 15.885 | 55.938 | 71.137 | 1.00 | 62.02 | B | N |
| ATOM | 2756 | CA | PHE | B | 67 | 14.768 | 56.237 | 72.043 | 1.00 | 62.02 | B | C |
| ATOM | 2757 | CB | PHE | B | 67 | 15.234 | 56.042 | 73.491 | 1.00 | 79.95 | B | C |
| ATOM | 2758 | CG | PHE | B | 67 | 16.474 | 56.832 | 73.853 | 1.00 | 79.95 | B | C |
| ATOM | 2759 | CD1 | PHE | B | 67 | 16.371 | 58.091 | 74.458 | 1.00 | 79.95 | B | C |
| ATOM | 2760 | CD2 | PHE | B | 67 | 17.744 | 56.336 | 73.538 | 1.00 | 79.95 | B | C |
| ATOM | 2761 | CE1 | PHE | B | 67 | 17.525 | 58.840 | 74.758 | 1.00 | 79.95 | B | C |
| ATOM | 2762 | CE2 | PHE | B | 67 | 18.910 | 57.068 | 73.827 | 1.00 | 79.95 | B | C |
| ATOM | 2763 | CZ | PHE | B | 67 | 18.805 | 58.324 | 74.434 | 1.00 | 79.95 | B | C |
| ATOM | 2764 | C | PHE | B | 67 | 14.223 | 57.670 | 71.858 | 1.00 | 62.02 | B | C |
| ATOM | 2765 | O | PHE | B | 67 | 13.015 | 57.864 | 71.686 | 1.00 | 62.02 | B | O |
| ATOM | 2766 | N | GLY | B | 68 | 15.138 | 58.637 | 71.845 | 1.00 | 61.32 | B | N |

| | | | | | | | | | | | | |
|---|---|---|---|---|---|---|---|---|---|---|---|---|
| ATOM | 2767 | CA | GLY | B | 68 | 14.786 | 60.045 | 71.727 | 1.00 | 61.32 | B | C |
| ATOM | 2768 | C | GLY | B | 68 | 16.027 | 60.922 | 71.870 | 1.00 | 61.32 | B | C |
| ATOM | 2769 | O | GLY | B | 68 | 17.132 | 60.470 | 71.542 | 1.00 | 61.32 | B | O |
| ATOM | 2770 | N | VAL | B | 69 | 15.855 | 62.148 | 72.375 | 1.00 | 50.35 | B | N |
| ATOM | 2771 | CA | VAL | B | 69 | 16.968 | 63.097 | 72.547 | 1.00 | 50.35 | B | C |
| ATOM | 2772 | CB | VAL | B | 69 | 16.823 | 64.410 | 71.766 | 1.00 | 31.36 | B | C |
| ATOM | 2773 | CG1 | VAL | B | 69 | 18.092 | 65.238 | 72.062 | 1.00 | 31.36 | B | C |
| ATOM | 2774 | CG2 | VAL | B | 69 | 16.651 | 64.167 | 70.295 | 1.00 | 31.36 | B | C |
| ATOM | 2775 | C | VAL | B | 69 | 17.054 | 63.490 | 73.990 | 1.00 | 50.35 | B | C |
| ATOM | 2776 | O | VAL | B | 69 | 16.030 | 63.635 | 74.647 | 1.00 | 50.35 | B | O |
| ATOM | 2777 | N | VAL | B | 70 | 18.286 | 63.601 | 74.468 | 1.00 | 40.09 | B | N |
| ATOM | 2778 | CA | VAL | B | 70 | 18.519 | 63.980 | 75.848 | 1.00 | 40.09 | B | C |
| ATOM | 2779 | CB | VAL | B | 70 | 19.277 | 62.888 | 76.591 | 1.00 | 21.98 | B | C |
| ATOM | 2780 | CG1 | VAL | B | 70 | 19.480 | 63.289 | 78.035 | 1.00 | 21.98 | B | C |
| ATOM | 2781 | CG2 | VAL | B | 70 | 18.509 | 61.589 | 76.488 | 1.00 | 21.98 | B | C |
| ATOM | 2782 | C | VAL | B | 70 | 19.289 | 65.289 | 75.927 | 1.00 | 40.09 | B | C |
| ATOM | 2783 | O | VAL | B | 70 | 20.460 | 65.369 | 75.543 | 1.00 | 40.09 | B | O |
| ATOM | 2784 | N | TYR | B | 71 | 18.610 | 66.317 | 76.419 | 1.00 | 30.48 | B | N |
| ATOM | 2785 | CA | TYR | B | 71 | 19.215 | 67.625 | 76.554 | 1.00 | 30.48 | B | C |
| ATOM | 2786 | CB | TYR | B | 71 | 18.210 | 68.737 | 76.239 | 1.00 | 38.18 | B | C |
| ATOM | 2787 | CG | TYR | B | 71 | 17.473 | 68.539 | 74.940 | 1.00 | 38.18 | B | C |
| ATOM | 2788 | CD1 | TYR | B | 71 | 16.534 | 67.526 | 74.810 | 1.00 | 38.18 | B | C |
| ATOM | 2789 | CE1 | TYR | B | 71 | 15.884 | 67.301 | 73.608 | 1.00 | 38.18 | B | C |
| ATOM | 2790 | CD2 | TYR | B | 71 | 17.745 | 69.338 | 73.828 | 1.00 | 38.18 | B | C |
| ATOM | 2791 | CE2 | TYR | B | 71 | 17.103 | 69.122 | 72.619 | 1.00 | 38.18 | B | C |
| ATOM | 2792 | CZ | TYR | B | 71 | 16.171 | 68.097 | 72.513 | 1.00 | 38.18 | B | C |
| ATOM | 2793 | OH | TYR | B | 71 | 15.536 | 67.839 | 71.319 | 1.00 | 38.18 | B | O |
| ATOM | 2794 | C | TYR | B | 71 | 19.644 | 67.764 | 77.983 | 1.00 | 30.48 | B | C |
| ATOM | 2795 | O | TYR | B | 71 | 19.272 | 66.955 | 78.831 | 1.00 | 30.48 | B | O |
| ATOM | 2796 | N | GLN | B | 72 | 20.452 | 68.788 | 78.232 | 1.00 | 39.61 | B | N |
| ATOM | 2797 | CA | GLN | B | 72 | 20.907 | 69.109 | 79.569 | 1.00 | 39.61 | B | C |
| ATOM | 2798 | CB | GLN | B | 72 | 22.422 | 69.123 | 79.665 | 1.00 | 38.68 | B | C |
| ATOM | 2799 | CG | GLN | B | 72 | 22.894 | 69.567 | 81.035 | 1.00 | 38.68 | B | C |
| ATOM | 2800 | CD | GLN | B | 72 | 24.395 | 69.671 | 81.130 | 1.00 | 38.68 | B | C |
| ATOM | 2801 | OE1 | GLN | B | 72 | 24.955 | 69.755 | 82.230 | 1.00 | 38.68 | B | O |
| ATOM | 2802 | NE2 | GLN | B | 72 | 25.065 | 69.669 | 79.979 | 1.00 | 38.68 | B | N |
| ATOM | 2803 | C | GLN | B | 72 | 20.397 | 70.515 | 79.697 | 1.00 | 39.61 | B | C |
| ATOM | 2804 | O | GLN | B | 72 | 20.300 | 71.216 | 78.692 | 1.00 | 39.61 | B | O |
| ATOM | 2805 | N | ALA | B | 73 | 20.065 | 70.935 | 80.906 | 1.00 | 27.59 | B | N |
| ATOM | 2806 | CA | ALA | B | 73 | 19.552 | 72.281 | 81.089 | 1.00 | 27.59 | B | C |
| ATOM | 2807 | CB | ALA | B | 73 | 18.097 | 72.361 | 80.627 | 1.00 | 20.91 | B | C |
| ATOM | 2808 | C | ALA | B | 73 | 19.658 | 72.698 | 82.538 | 1.00 | 27.59 | B | C |
| ATOM | 2809 | O | ALA | B | 73 | 19.923 | 71.872 | 83.423 | 1.00 | 27.59 | B | O |
| ATOM | 2810 | N | LYS | B | 74 | 19.434 | 73.987 | 82.772 | 1.00 | 31.83 | B | N |
| ATOM | 2811 | CA | LYS | B | 74 | 19.518 | 74.540 | 84.112 | 1.00 | 31.83 | B | C |
| ATOM | 2812 | CB | LYS | B | 74 | 20.559 | 75.658 | 84.136 | 1.00 | 49.94 | B | C |
| ATOM | 2813 | CG | LYS | B | 74 | 21.087 | 75.996 | 85.515 | 1.00 | 49.94 | B | C |
| ATOM | 2814 | CD | LYS | B | 74 | 21.617 | 77.433 | 85.552 | 1.00 | 49.94 | B | C |
| ATOM | 2815 | CE | LYS | B | 74 | 22.637 | 77.622 | 86.658 | 1.00 | 49.94 | B | C |
| ATOM | 2816 | NZ | LYS | B | 74 | 23.852 | 76.796 | 86.397 | 1.00 | 49.94 | B | N |
| ATOM | 2817 | C | LYS | B | 74 | 18.165 | 75.072 | 84.579 | 1.00 | 31.83 | B | C |
| ATOM | 2818 | O | LYS | B | 74 | 17.529 | 75.901 | 83.918 | 1.00 | 31.83 | B | O |
| ATOM | 2819 | N | LEU | B | 75 | 17.716 | 74.569 | 85.717 | 1.00 | 35.44 | B | N |
| ATOM | 2820 | CA | LEU | B | 75 | 16.463 | 75.021 | 86.269 | 1.00 | 35.44 | B | C |
| ATOM | 2821 | CB | LEU | B | 75 | 16.075 | 74.151 | 87.465 | 1.00 | 25.11 | B | C |
| ATOM | 2822 | CG | LEU | B | 75 | 15.820 | 72.674 | 87.157 | 1.00 | 25.11 | B | C |
| ATOM | 2823 | CD1 | LEU | B | 75 | 15.409 | 71.948 | 88.421 | 1.00 | 25.11 | B | C |
| ATOM | 2824 | CD2 | LEU | B | 75 | 14.724 | 72.554 | 86.111 | 1.00 | 25.11 | B | C |
| ATOM | 2825 | C | LEU | B | 75 | 16.754 | 76.439 | 86.720 | 1.00 | 35.44 | B | C |
| ATOM | 2826 | O | LEU | B | 75 | 17.640 | 76.652 | 87.546 | 1.00 | 35.44 | B | O |
| ATOM | 2827 | N | CYS | B | 76 | 16.026 | 77.410 | 86.175 | 1.00 | 39.86 | B | N |
| ATOM | 2828 | CA | CYS | B | 76 | 16.242 | 78.807 | 86.535 | 1.00 | 39.86 | B | C |
| ATOM | 2829 | CB | CYS | B | 76 | 15.208 | 79.700 | 85.860 | 1.00 | 48.80 | B | C |

| | | | | | | | | | | | | |
|---|---|---|---|---|---|---|---|---|---|---|---|---|
| ATOM | 2830 | SG | CYS | B | 76 | 15.353 | 79.704 | 84.061 | 1.00 | 48.80 | B | S |
| ATOM | 2831 | C | CYS | B | 76 | 16.219 | 79.056 | 88.031 | 1.00 | 39.86 | B | C |
| ATOM | 2832 | O | CYS | B | 76 | 17.191 | 79.566 | 88.587 | 1.00 | 39.86 | B | O |
| ATOM | 2833 | N | ASP | B | 77 | 15.129 | 78.687 | 88.696 | 1.00 | 49.76 | B | N |
| ATOM | 2834 | CA | ASP | B | 77 | 15.033 | 78.934 | 90.134 | 1.00 | 49.76 | B | C |
| ATOM | 2835 | CB | ASP | B | 77 | 13.661 | 78.472 | 90.671 | 1.00 | 80.19 | B | C |
| ATOM | 2836 | CG | ASP | B | 77 | 13.436 | 76.954 | 90.539 | 1.00 | 80.19 | B | C |
| ATOM | 2837 | OD1 | ASP | B | 77 | 13.650 | 76.381 | 89.429 | 1.00 | 80.19 | B | O |
| ATOM | 2838 | OD2 | ASP | B | 77 | 13.027 | 76.336 | 91.559 | 1.00 | 80.19 | B | O |
| ATOM | 2839 | C | ASP | B | 77 | 16.190 | 78.320 | 90.931 | 1.00 | 49.76 | B | C |
| ATOM | 2840 | O | ASP | B | 77 | 17.083 | 79.043 | 91.375 | 1.00 | 49.76 | B | O |
| ATOM | 2841 | N | SER | B | 78 | 16.202 | 77.001 | 91.092 | 1.00 | 46.34 | B | N |
| ATOM | 2842 | CA | SER | B | 78 | 17.267 | 76.325 | 91.845 | 1.00 | 46.34 | B | C |
| ATOM | 2843 | CB | SER | B | 78 | 16.919 | 74.846 | 92.046 | 1.00 | 34.05 | B | C |
| ATOM | 2844 | OG | SER | B | 78 | 16.676 | 74.210 | 90.798 | 1.00 | 34.05 | B | O |
| ATOM | 2845 | C | SER | B | 78 | 18.642 | 76.411 | 91.191 | 1.00 | 46.34 | B | C |
| ATOM | 2846 | O | SER | B | 78 | 19.653 | 76.276 | 91.864 | 1.00 | 46.34 | B | O |
| ATOM | 2847 | N | GLY | B | 79 | 18.677 | 76.614 | 89.882 | 1.00 | 31.69 | B | N |
| ATOM | 2848 | CA | GLY | B | 79 | 19.952 | 76.687 | 89.191 | 1.00 | 31.69 | B | C |
| ATOM | 2849 | C | GLY | B | 79 | 20.607 | 75.323 | 89.020 | 1.00 | 31.69 | B | C |
| ATOM | 2850 | O | GLY | B | 79 | 21.757 | 75.234 | 88.595 | 1.00 | 31.69 | B | O |
| ATOM | 2851 | N | GLU | B | 80 | 19.872 | 74.261 | 89.338 | 1.00 | 39.92 | B | N |
| ATOM | 2852 | CA | GLU | B | 80 | 20.386 | 72.903 | 89.229 | 1.00 | 39.92 | B | C |
| ATOM | 2853 | CB | GLU | B | 80 | 19.605 | 71.991 | 90.157 | 1.00 | 51.32 | B | C |
| ATOM | 2854 | CG | GLU | B | 80 | 19.441 | 72.543 | 91.561 | 1.00 | 51.32 | B | C |
| ATOM | 2855 | CD | GLU | B | 80 | 18.611 | 71.620 | 92.435 | 1.00 | 51.32 | B | C |
| ATOM | 2856 | OE1 | GLU | B | 80 | 19.114 | 70.526 | 92.791 | 1.00 | 51.32 | B | O |
| ATOM | 2857 | OE2 | GLU | B | 80 | 17.450 | 71.981 | 92.750 | 1.00 | 51.32 | B | O |
| ATOM | 2858 | C | GLU | B | 80 | 20.285 | 72.369 | 87.803 | 1.00 | 39.92 | B | C |
| ATOM | 2859 | O | GLU | B | 80 | 19.388 | 72.759 | 87.046 | 1.00 | 39.92 | B | O |
| ATOM | 2860 | N | LEU | B | 81 | 21.207 | 71.473 | 87.449 | 1.00 | 22.46 | B | N |
| ATOM | 2861 | CA | LEU | B | 81 | 21.233 | 70.871 | 86.123 | 1.00 | 22.46 | B | C |
| ATOM | 2862 | CB | LEU | B | 81 | 22.652 | 70.413 | 85.779 | 1.00 | 20.85 | B | C |
| ATOM | 2863 | CG | LEU | B | 81 | 23.772 | 71.455 | 85.642 | 1.00 | 20.85 | B | C |
| ATOM | 2864 | CD1 | LEU | B | 81 | 25.102 | 70.737 | 85.513 | 1.00 | 20.85 | B | C |
| ATOM | 2865 | CD2 | LEU | B | 81 | 23.539 | 72.352 | 84.428 | 1.00 | 20.85 | B | C |
| ATOM | 2866 | C | LEU | B | 81 | 20.277 | 69.681 | 86.057 | 1.00 | 22.46 | B | C |
| ATOM | 2867 | O | LEU | B | 81 | 20.123 | 68.937 | 87.024 | 1.00 | 22.46 | B | O |
| ATOM | 2868 | N | VAL | B | 82 | 19.642 | 69.511 | 84.902 | 1.00 | 30.78 | B | N |
| ATOM | 2869 | CA | VAL | B | 82 | 18.694 | 68.428 | 84.696 | 1.00 | 30.78 | B | C |
| ATOM | 2870 | CB | VAL | B | 82 | 17.242 | 68.898 | 84.884 | 1.00 | 31.08 | B | C |
| ATOM | 2871 | CG1 | VAL | B | 82 | 16.991 | 69.253 | 86.329 | 1.00 | 31.08 | B | C |
| ATOM | 2872 | CG2 | VAL | B | 82 | 16.965 | 70.084 | 83.970 | 1.00 | 31.08 | B | C |
| ATOM | 2873 | C | VAL | B | 82 | 18.779 | 67.839 | 83.306 | 1.00 | 30.78 | B | C |
| ATOM | 2874 | O | VAL | B | 82 | 19.204 | 68.495 | 82.358 | 1.00 | 30.78 | B | O |
| ATOM | 2875 | N | ALA | B | 83 | 18.342 | 66.592 | 83.199 | 1.00 | 24.30 | B | N |
| ATOM | 2876 | CA | ALA | B | 83 | 18.332 | 65.881 | 81.938 | 1.00 | 24.30 | B | C |
| ATOM | 2877 | CB | ALA | B | 83 | 18.868 | 64.464 | 82.141 | 1.00 | 22.71 | B | C |
| ATOM | 2878 | C | ALA | B | 83 | 16.883 | 65.841 | 81.464 | 1.00 | 24.30 | B | C |
| ATOM | 2879 | O | ALA | B | 83 | 15.964 | 65.725 | 82.273 | 1.00 | 24.30 | B | O |
| ATOM | 2880 | N | ILE | B | 84 | 16.674 | 65.954 | 80.157 | 1.00 | 18.14 | B | N |
| ATOM | 2881 | CA | ILE | B | 84 | 15.323 | 65.918 | 79.629 | 1.00 | 18.14 | B | C |
| ATOM | 2882 | CB | ILE | B | 84 | 14.831 | 67.312 | 79.133 | 1.00 | 21.12 | B | C |
| ATOM | 2883 | CG2 | ILE | B | 84 | 13.371 | 67.216 | 78.684 | 1.00 | 21.12 | B | C |
| ATOM | 2884 | CG1 | ILE | B | 84 | 14.931 | 68.351 | 80.255 | 1.00 | 21.12 | B | C |
| ATOM | 2885 | CD1 | ILE | B | 84 | 14.388 | 69.721 | 79.882 | 1.00 | 21.12 | B | C |
| ATOM | 2886 | C | ILE | B | 84 | 15.232 | 64.951 | 78.475 | 1.00 | 18.14 | B | C |
| ATOM | 2887 | O | ILE | B | 84 | 15.663 | 65.255 | 77.368 | 1.00 | 18.14 | B | O |
| ATOM | 2888 | N | LYS | B | 85 | 14.674 | 63.773 | 78.737 | 1.00 | 28.86 | B | N |
| ATOM | 2889 | CA | LYS | B | 85 | 14.526 | 62.787 | 77.686 | 1.00 | 28.86 | B | C |
| ATOM | 2890 | CB | LYS | B | 85 | 14.564 | 61.369 | 78.244 | 1.00 | 34.67 | B | C |
| ATOM | 2891 | CG | LYS | B | 85 | 14.557 | 60.336 | 77.135 | 1.00 | 34.67 | B | C |
| ATOM | 2892 | CD | LYS | B | 85 | 13.899 | 59.027 | 77.549 | 1.00 | 34.67 | B | C |

| | | | | | | | | | | | | |
|---|---|---|---|---|---|---|---|---|---|---|---|---|
| ATOM | 2893 | CE | LYS | B | 85 | 14.853 | 58.118 | 78.298 | 1.00 | 34.67 | B | C |
| ATOM | 2894 | NZ | LYS | B | 85 | 14.233 | 56.781 | 78.443 | 1.00 | 34.67 | B | N |
| ATOM | 2895 | C | LYS | B | 85 | 13.189 | 63.045 | 77.016 | 1.00 | 28.86 | B | C |
| ATOM | 2896 | O | LYS | B | 85 | 12.131 | 62.776 | 77.583 | 1.00 | 28.86 | B | O |
| ATOM | 2897 | N | LYS | B | 86 | 13.254 | 63.594 | 75.809 | 1.00 | 38.23 | B | N |
| ATOM | 2898 | CA | LYS | B | 86 | 12.068 | 63.912 | 75.026 | 1.00 | 38.23 | B | C |
| ATOM | 2899 | CB | LYS | B | 86 | 12.292 | 65.217 | 74.261 | 1.00 | 36.16 | B | C |
| ATOM | 2900 | CG | LYS | B | 86 | 11.086 | 65.659 | 73.467 | 1.00 | 36.16 | B | C |
| ATOM | 2901 | CD | LYS | B | 86 | 11.475 | 66.550 | 72.296 | 1.00 | 36.16 | B | C |
| ATOM | 2902 | CE | LYS | B | 86 | 11.420 | 68.028 | 72.623 | 1.00 | 36.16 | B | C |
| ATOM | 2903 | NZ | LYS | B | 86 | 11.754 | 68.811 | 71.391 | 1.00 | 36.16 | B | N |
| ATOM | 2904 | C | LYS | B | 86 | 11.807 | 62.784 | 74.036 | 1.00 | 38.23 | B | C |
| ATOM | 2905 | O | LYS | B | 86 | 12.725 | 62.376 | 73.325 | 1.00 | 38.23 | B | O |
| ATOM | 2906 | N | VAL | B | 87 | 10.577 | 62.271 | 74.004 | 1.00 | 33.31 | B | N |
| ATOM | 2907 | CA | VAL | B | 87 | 10.222 | 61.195 | 73.075 | 1.00 | 33.31 | B | C |
| ATOM | 2908 | CB | VAL | B | 87 | 10.315 | 59.802 | 73.724 | 1.00 | 19.45 | B | C |
| ATOM | 2909 | CG1 | VAL | B | 87 | 11.573 | 59.696 | 74.551 | 1.00 | 19.45 | B | C |
| ATOM | 2910 | CG2 | VAL | B | 87 | 9.083 | 59.526 | 74.551 | 1.00 | 19.45 | B | C |
| ATOM | 2911 | C | VAL | B | 87 | 8.804 | 61.356 | 72.535 | 1.00 | 33.31 | B | C |
| ATOM | 2912 | O | VAL | B | 87 | 7.900 | 61.818 | 73.233 | 1.00 | 33.31 | B | O |
| ATOM | 2913 | N | LEU | B | 88 | 8.609 | 60.970 | 71.285 | 1.00 | 45.95 | B | N |
| ATOM | 2914 | CA | LEU | B | 88 | 7.299 | 61.076 | 70.677 | 1.00 | 45.95 | B | C |
| ATOM | 2915 | CB | LEU | B | 88 | 7.390 | 60.644 | 69.219 | 1.00 | 48.13 | B | C |
| ATOM | 2916 | CG | LEU | B | 88 | 6.190 | 60.972 | 68.344 | 1.00 | 48.13 | B | C |
| ATOM | 2917 | CD1 | LEU | B | 88 | 6.151 | 62.473 | 68.080 | 1.00 | 48.13 | B | C |
| ATOM | 2918 | CD2 | LEU | B | 88 | 6.300 | 60.190 | 67.051 | 1.00 | 48.13 | B | C |
| ATOM | 2919 | C | LEU | B | 88 | 6.326 | 60.166 | 71.439 | 1.00 | 45.95 | B | C |
| ATOM | 2920 | O | LEU | B | 88 | 6.661 | 59.029 | 71.757 | 1.00 | 45.95 | B | O |
| ATOM | 2921 | N | GLN | B | 89 | 5.135 | 60.666 | 71.750 | 1.00 | 43.61 | B | N |
| ATOM | 2922 | CA | GLN | B | 89 | 4.151 | 59.865 | 72.468 | 1.00 | 43.61 | B | C |
| ATOM | 2923 | CB | GLN | B | 89 | 3.633 | 60.612 | 73.710 | 1.00 | 50.98 | B | C |
| ATOM | 2924 | CG | GLN | B | 89 | 3.560 | 59.816 | 75.042 | 1.00 | 50.98 | B | C |
| ATOM | 2925 | CD | GLN | B | 89 | 3.732 | 58.309 | 74.884 | 1.00 | 50.98 | B | C |
| ATOM | 2926 | OE1 | GLN | B | 89 | 4.763 | 57.839 | 74.391 | 1.00 | 50.98 | B | O |
| ATOM | 2927 | NE2 | GLN | B | 89 | 2.727 | 57.544 | 75.310 | 1.00 | 50.98 | B | N |
| ATOM | 2928 | C | GLN | B | 89 | 3.014 | 59.677 | 71.487 | 1.00 | 43.61 | B | C |
| ATOM | 2929 | O | GLN | B | 89 | 2.940 | 60.387 | 70.483 | 1.00 | 43.61 | B | O |
| ATOM | 2930 | N | ASP | B | 90 | 2.132 | 58.728 | 71.766 | 1.00 | 70.28 | B | N |
| ATOM | 2931 | CA | ASP | B | 90 | 1.000 | 58.476 | 70.888 | 1.00 | 70.28 | B | C |
| ATOM | 2932 | CB | ASP | B | 90 | 1.017 | 57.033 | 70.389 | 1.00 | 81.91 | B | C |
| ATOM | 2933 | CG | ASP | B | 90 | -0.365 | 56.566 | 69.916 | 1.00 | 81.91 | B | C |
| ATOM | 2934 | OD1 | ASP | B | 90 | -0.771 | 55.424 | 70.275 | 1.00 | 81.91 | B | O |
| ATOM | 2935 | OD2 | ASP | B | 90 | -1.048 | 57.342 | 69.198 | 1.00 | 81.91 | B | O |
| ATOM | 2936 | C | ASP | B | 90 | -0.284 | 58.721 | 71.674 | 1.00 | 70.28 | B | C |
| ATOM | 2937 | O | ASP | B | 90 | -0.820 | 57.794 | 72.304 | 1.00 | 70.28 | B | O |
| ATOM | 2938 | N | ALA | B | 91 | -0.754 | 59.973 | 71.640 | 1.00 | 87.37 | B | N |
| ATOM | 2939 | CA | ALA | B | 91 | -1.974 | 60.417 | 72.345 | 1.00 | 87.37 | B | C |
| ATOM | 2940 | CB | ALA | B | 91 | -2.872 | 61.221 | 71.383 | 1.00 | 51.16 | B | C |
| ATOM | 2941 | C | ALA | B | 91 | -2.784 | 59.295 | 73.002 | 1.00 | 87.37 | B | C |
| ATOM | 2942 | O | ALA | B | 91 | -3.047 | 59.335 | 74.204 | 1.00 | 87.37 | B | O |
| ATOM | 2943 | N | ALA | B | 92 | -3.174 | 58.300 | 72.207 | 1.00 | 59.11 | B | N |
| ATOM | 2944 | CA | ALA | B | 92 | -3.949 | 57.163 | 72.711 | 1.00 | 59.11 | B | C |
| ATOM | 2945 | CB | ALA | B | 92 | -4.128 | 56.123 | 71.599 | 1.00 | 59.63 | B | C |
| ATOM | 2946 | C | ALA | B | 92 | -3.305 | 56.499 | 73.929 | 1.00 | 59.11 | B | C |
| ATOM | 2947 | O | ALA | B | 92 | -3.694 | 56.761 | 75.075 | 1.00 | 59.11 | B | O |
| ATOM | 2948 | N | ALA | B | 93 | -2.318 | 55.642 | 73.653 | 1.00 | 88.18 | B | N |
| ATOM | 2949 | CA | ALA | B | 93 | -1.582 | 54.871 | 74.668 | 1.00 | 88.18 | B | C |
| ATOM | 2950 | CB | ALA | B | 93 | -0.562 | 53.930 | 73.963 | 1.00 | 62.13 | B | C |
| ATOM | 2951 | C | ALA | B | 93 | -0.877 | 55.675 | 75.781 | 1.00 | 88.18 | B | C |
| ATOM | 2952 | O | ALA | B | 93 | -0.690 | 56.894 | 75.673 | 1.00 | 88.18 | B | O |
| ATOM | 2953 | N | LYS | B | 94 | -0.512 | 54.963 | 76.850 | 1.00 | 49.48 | B | N |
| ATOM | 2954 | CA | LYS | B | 94 | 0.164 | 55.533 | 78.013 | 1.00 | 49.48 | B | C |
| ATOM | 2955 | CB | LYS | B | 94 | -0.497 | 55.055 | 79.307 | 1.00 | 78.96 | B | C |

| | | | | | | | | | | | | |
|---|---|---|---|---|---|---|---|---|---|---|---|---|
| ATOM | 2956 | CG | LYS | B | 94 | -1.983 | 55.417 | 79.413 | 1.00 | 78.96 | B | C |
| ATOM | 2957 | CD | LYS | B | 94 | -2.568 | 55.121 | 80.810 | 1.00 | 78.96 | B | C |
| ATOM | 2958 | CE | LYS | B | 94 | -2.915 | 53.630 | 81.019 | 1.00 | 78.96 | B | C |
| ATOM | 2959 | NZ | LYS | B | 94 | -1.749 | 52.696 | 80.875 | 1.00 | 78.96 | B | N |
| ATOM | 2960 | C | LYS | B | 94 | 1.597 | 55.052 | 77.994 | 1.00 | 49.48 | B | C |
| ATOM | 2961 | O | LYS | B | 94 | 1.852 | 53.850 | 77.866 | 1.00 | 49.48 | B | O |
| ATOM | 2962 | N | ASN | B | 95 | 2.532 | 55.984 | 78.137 | 1.00 | 20.37 | B | N |
| ATOM | 2963 | CA | ASN | B | 95 | 3.950 | 55.652 | 78.115 | 1.00 | 20.37 | B | C |
| ATOM | 2964 | CB | ASN | B | 95 | 4.779 | 56.927 | 78.163 | 1.00 | 24.54 | B | C |
| ATOM | 2965 | CG | ASN | B | 95 | 6.228 | 56.667 | 77.895 | 1.00 | 24.54 | B | C |
| ATOM | 2966 | OD1 | ASN | B | 95 | 6.890 | 55.952 | 78.651 | 1.00 | 24.54 | B | O |
| ATOM | 2967 | ND2 | ASN | B | 95 | 6.737 | 57.232 | 76.803 | 1.00 | 24.54 | B | N |
| ATOM | 2968 | C | ASN | B | 95 | 4.364 | 54.734 | 79.248 | 1.00 | 20.37 | B | C |
| ATOM | 2969 | O | ASN | B | 95 | 4.338 | 55.128 | 80.410 | 1.00 | 20.37 | B | O |
| ATOM | 2970 | N | ARG | B | 96 | 4.764 | 53.512 | 78.904 | 1.00 | 33.02 | B | N |
| ATOM | 2971 | CA | ARG | B | 96 | 5.168 | 52.534 | 79.915 | 1.00 | 33.02 | B | C |
| ATOM | 2972 | CB | ARG | B | 96 | 5.504 | 51.194 | 79.259 | 1.00 | 40.46 | B | C |
| ATOM | 2973 | CG | ARG | B | 96 | 5.720 | 50.087 | 80.264 | 1.00 | 40.46 | B | C |
| ATOM | 2974 | CD | ARG | B | 96 | 6.343 | 48.868 | 79.627 | 1.00 | 40.46 | B | C |
| ATOM | 2975 | NE | ARG | B | 96 | 5.396 | 48.052 | 78.876 | 1.00 | 40.46 | B | N |
| ATOM | 2976 | CZ | ARG | B | 96 | 5.767 | 47.121 | 78.001 | 1.00 | 40.46 | B | C |
| ATOM | 2977 | NH1 | ARG | B | 96 | 7.067 | 46.913 | 77.777 | 1.00 | 40.46 | B | N |
| ATOM | 2978 | NH2 | ARG | B | 96 | 4.857 | 46.386 | 77.365 | 1.00 | 40.46 | B | N |
| ATOM | 2979 | C | ARG | B | 96 | 6.346 | 52.998 | 80.782 | 1.00 | 33.02 | B | C |
| ATOM | 2980 | O | ARG | B | 96 | 6.391 | 52.753 | 81.995 | 1.00 | 33.02 | B | O |
| ATOM | 2981 | N | GLU | B | 97 | 7.291 | 53.686 | 80.158 | 1.00 | 31.93 | B | N |
| ATOM | 2982 | CA | GLU | B | 97 | 8.463 | 54.155 | 80.868 | 1.00 | 31.93 | B | C |
| ATOM | 2983 | CB | GLU | B | 97 | 9.470 | 54.734 | 79.892 | 1.00 | 29.96 | B | C |
| ATOM | 2984 | CG | GLU | B | 97 | 10.807 | 55.027 | 80.523 | 1.00 | 29.96 | B | C |
| ATOM | 2985 | CD | GLU | B | 97 | 11.705 | 55.840 | 79.618 | 1.00 | 29.96 | B | C |
| ATOM | 2986 | OE1 | GLU | B | 97 | 11.442 | 55.878 | 78.400 | 1.00 | 29.96 | B | O |
| ATOM | 2987 | OE2 | GLU | B | 97 | 12.681 | 56.429 | 80.125 | 1.00 | 29.96 | B | O |
| ATOM | 2988 | C | GLU | B | 97 | 8.095 | 55.203 | 81.900 | 1.00 | 31.93 | B | C |
| ATOM | 2989 | O | GLU | B | 97 | 8.613 | 55.178 | 83.005 | 1.00 | 31.93 | B | O |
| ATOM | 2990 | N | LEU | B | 98 | 7.210 | 56.129 | 81.549 | 1.00 | 13.88 | B | N |
| ATOM | 2991 | CA | LEU | B | 98 | 6.820 | 57.158 | 82.498 | 1.00 | 13.88 | B | C |
| ATOM | 2992 | CB | LEU | B | 98 | 5.868 | 58.166 | 81.855 | 1.00 | 21.78 | B | C |
| ATOM | 2993 | CG | LEU | B | 98 | 5.112 | 59.138 | 82.789 | 1.00 | 21.78 | B | C |
| ATOM | 2994 | CD1 | LEU | B | 98 | 6.012 | 60.247 | 83.332 | 1.00 | 21.78 | B | C |
| ATOM | 2995 | CD2 | LEU | B | 98 | 3.976 | 59.758 | 82.009 | 1.00 | 21.78 | B | C |
| ATOM | 2996 | C | LEU | B | 98 | 6.133 | 56.532 | 83.692 | 1.00 | 13.88 | B | C |
| ATOM | 2997 | O | LEU | B | 98 | 6.336 | 56.941 | 84.829 | 1.00 | 13.88 | B | O |
| ATOM | 2998 | N | GLN | B | 99 | 5.304 | 55.538 | 83.397 | 1.00 | 35.56 | B | N |
| ATOM | 2999 | CA | GLN | B | 99 | 4.522 | 54.799 | 84.385 | 1.00 | 35.56 | B | C |
| ATOM | 3000 | CB | GLN | B | 99 | 3.747 | 53.672 | 83.680 | 1.00 | 75.29 | B | C |
| ATOM | 3001 | CG | GLN | B | 99 | 2.942 | 52.763 | 84.617 | 1.00 | 75.29 | B | C |
| ATOM | 3002 | CD | GLN | B | 99 | 3.023 | 51.262 | 84.254 | 1.00 | 75.29 | B | C |
| ATOM | 3003 | OE1 | GLN | B | 99 | 2.634 | 50.839 | 83.143 | 1.00 | 75.29 | B | O |
| ATOM | 3004 | NE2 | GLN | B | 99 | 3.524 | 50.448 | 85.203 | 1.00 | 75.29 | B | N |
| ATOM | 3005 | C | GLN | B | 99 | 5.422 | 54.198 | 85.446 | 1.00 | 35.56 | B | C |
| ATOM | 3006 | O | GLN | B | 99 | 5.101 | 54.213 | 86.633 | 1.00 | 35.56 | B | O |
| ATOM | 3007 | N | ILE | B | 100 | 6.546 | 53.657 | 84.991 | 1.00 | 25.44 | B | N |
| ATOM | 3008 | CA | ILE | B | 100 | 7.511 | 53.019 | 85.862 | 1.00 | 25.44 | B | C |
| ATOM | 3009 | CB | ILE | B | 100 | 8.390 | 52.062 | 85.031 | 1.00 | 24.37 | B | C |
| ATOM | 3010 | CG2 | ILE | B | 100 | 9.561 | 51.536 | 85.846 | 1.00 | 24.37 | B | C |
| ATOM | 3011 | CG1 | ILE | B | 100 | 7.501 | 50.928 | 84.517 | 1.00 | 24.37 | B | C |
| ATOM | 3012 | CD1 | ILE | B | 100 | 8.245 | 49.745 | 83.962 | 1.00 | 24.37 | B | C |
| ATOM | 3013 | C | ILE | B | 100 | 8.335 | 54.065 | 86.618 | 1.00 | 25.44 | B | C |
| ATOM | 3014 | O | ILE | B | 100 | 8.480 | 53.969 | 87.834 | 1.00 | 25.44 | B | O |
| ATOM | 3015 | N | MET | B | 101 | 8.851 | 55.073 | 85.920 | 1.00 | 17.69 | B | N |
| ATOM | 3016 | CA | MET | B | 101 | 9.624 | 56.129 | 86.579 | 1.00 | 17.69 | B | C |
| ATOM | 3017 | CB | MET | B | 101 | 9.932 | 57.257 | 85.597 | 1.00 | 43.54 | B | C |
| ATOM | 3018 | CG | MET | B | 101 | 10.836 | 56.883 | 84.445 | 1.00 | 43.54 | B | C |

| | | | | | | | | | | | | |
|---|---|---|---|---|---|---|---|---|---|---|---|---|
| ATOM | 3019 | SD | MET | B | 101 | 12.554 | 56.904 | 84.902 | 1.00 | 43.54 | B | S |
| ATOM | 3020 | CE | MET | B | 101 | 12.660 | 55.381 | 85.849 | 1.00 | 43.54 | B | C |
| ATOM | 3021 | C | MET | B | 101 | 8.854 | 56.743 | 87.751 | 1.00 | 17.69 | B | C |
| ATOM | 3022 | O | MET | B | 101 | 9.411 | 56.971 | 88.819 | 1.00 | 17.69 | B | O |
| ATOM | 3023 | N | ARG | B | 102 | 7.576 | 57.027 | 87.508 | 1.00 | 22.85 | B | N |
| ATOM | 3024 | CA | ARG | B | 102 | 6.664 | 57.624 | 88.475 | 1.00 | 22.85 | B | C |
| ATOM | 3025 | CB | ARG | B | 102 | 5.232 | 57.654 | 87.917 | 1.00 | 40.41 | B | C |
| ATOM | 3026 | CG | ARG | B | 102 | 4.893 | 58.798 | 86.959 | 1.00 | 40.41 | B | C |
| ATOM | 3027 | CD | ARG | B | 102 | 4.491 | 60.053 | 87.709 | 1.00 | 40.41 | B | C |
| ATOM | 3028 | NE | ARG | B | 102 | 3.102 | 60.442 | 87.464 | 1.00 | 40.41 | B | N |
| ATOM | 3029 | CZ | ARG | B | 102 | 2.422 | 61.301 | 88.227 | 1.00 | 40.41 | B | C |
| ATOM | 3030 | NH1 | ARG | B | 102 | 3.000 | 61.863 | 89.290 | 1.00 | 40.41 | B | N |
| ATOM | 3031 | NH2 | ARG | B | 102 | 1.161 | 61.601 | 87.933 | 1.00 | 40.41 | B | N |
| ATOM | 3032 | C | ARG | B | 102 | 6.639 | 56.884 | 89.798 | 1.00 | 22.85 | B | C |
| ATOM | 3033 | O | ARG | B | 102 | 6.440 | 57.491 | 90.846 | 1.00 | 22.85 | B | O |
| ATOM | 3034 | N | LYS | B | 103 | 6.840 | 55.574 | 89.752 | 1.00 | 16.67 | B | N |
| ATOM | 3035 | CA | LYS | B | 103 | 6.785 | 54.763 | 90.960 | 1.00 | 16.67 | B | C |
| ATOM | 3036 | CB | LYS | B | 103 | 6.128 | 53.425 | 90.632 | 1.00 | 44.32 | B | C |
| ATOM | 3037 | CG | LYS | B | 103 | 5.713 | 52.644 | 91.853 | 1.00 | 44.32 | B | C |
| ATOM | 3038 | CD | LYS | B | 103 | 5.973 | 51.152 | 91.691 | 1.00 | 44.32 | B | C |
| ATOM | 3039 | CE | LYS | B | 103 | 7.475 | 50.839 | 91.613 | 1.00 | 44.32 | B | C |
| ATOM | 3040 | NZ | LYS | B | 103 | 8.253 | 51.249 | 92.833 | 1.00 | 44.32 | B | N |
| ATOM | 3041 | C | LYS | B | 103 | 8.126 | 54.496 | 91.670 | 1.00 | 16.67 | B | C |
| ATOM | 3042 | O | LYS | B | 103 | 8.158 | 54.066 | 92.817 | 1.00 | 16.67 | B | O |
| ATOM | 3043 | N | LEU | B | 104 | 9.236 | 54.755 | 91.000 | 1.00 | 28.51 | B | N |
| ATOM | 3044 | CA | LEU | B | 104 | 10.536 | 54.488 | 91.595 | 1.00 | 28.51 | B | C |
| ATOM | 3045 | CB | LEU | B | 104 | 11.524 | 54.073 | 90.495 | 1.00 | 26.45 | B | C |
| ATOM | 3046 | CG | LEU | B | 104 | 11.519 | 52.628 | 89.982 | 1.00 | 26.45 | B | C |
| ATOM | 3047 | CD1 | LEU | B | 104 | 10.107 | 52.103 | 89.875 | 1.00 | 26.45 | B | C |
| ATOM | 3048 | CD2 | LEU | B | 104 | 12.208 | 52.571 | 88.630 | 1.00 | 26.45 | B | C |
| ATOM | 3049 | C | LEU | B | 104 | 11.136 | 55.635 | 92.410 | 1.00 | 28.51 | B | C |
| ATOM | 3050 | O | LEU | B | 104 | 11.040 | 56.799 | 92.037 | 1.00 | 28.51 | B | O |
| ATOM | 3051 | N | ASP | B | 105 | 11.749 | 55.285 | 93.535 | 1.00 | 26.35 | B | N |
| ATOM | 3052 | CA | ASP | B | 105 | 12.403 | 56.251 | 94.409 | 1.00 | 26.35 | B | C |
| ATOM | 3053 | CB | ASP | B | 105 | 11.419 | 56.867 | 95.410 | 1.00 | 33.95 | B | C |
| ATOM | 3054 | CG | ASP | B | 105 | 12.118 | 57.754 | 96.460 | 1.00 | 33.95 | B | C |
| ATOM | 3055 | OD1 | ASP | B | 105 | 12.903 | 58.653 | 96.076 | 1.00 | 33.95 | B | O |
| ATOM | 3056 | OD2 | ASP | B | 105 | 11.881 | 57.556 | 97.670 | 1.00 | 33.95 | B | O |
| ATOM | 3057 | C | ASP | B | 105 | 13.514 | 55.532 | 95.169 | 1.00 | 26.35 | B | C |
| ATOM | 3058 | O | ASP | B | 105 | 13.279 | 54.957 | 96.228 | 1.00 | 26.35 | B | O |
| ATOM | 3059 | N | HIS | B | 106 | 14.724 | 55.564 | 94.624 | 1.00 | 16.48 | B | N |
| ATOM | 3060 | CA | HIS | B | 106 | 15.852 | 54.892 | 95.248 | 1.00 | 16.48 | B | C |
| ATOM | 3061 | CB | HIS | B | 106 | 15.956 | 53.466 | 94.705 | 1.00 | 19.33 | B | C |
| ATOM | 3062 | CG | HIS | B | 106 | 17.070 | 52.670 | 95.299 | 1.00 | 19.33 | B | C |
| ATOM | 3063 | CD2 | HIS | B | 106 | 17.057 | 51.627 | 96.160 | 1.00 | 19.33 | B | C |
| ATOM | 3064 | ND1 | HIS | B | 106 | 18.393 | 52.905 | 95.002 | 1.00 | 19.33 | B | N |
| ATOM | 3065 | CE1 | HIS | B | 106 | 19.149 | 52.039 | 95.653 | 1.00 | 19.33 | B | C |
| ATOM | 3066 | NE2 | HIS | B | 106 | 18.363 | 51.252 | 96.363 | 1.00 | 19.33 | B | N |
| ATOM | 3067 | C | HIS | B | 106 | 17.119 | 55.685 | 94.967 | 1.00 | 16.48 | B | C |
| ATOM | 3068 | O | HIS | B | 106 | 17.346 | 56.120 | 93.852 | 1.00 | 16.48 | B | O |
| ATOM | 3069 | N | CYS | B | 107 | 17.936 | 55.883 | 95.991 | 1.00 | 15.60 | B | N |
| ATOM | 3070 | CA | CYS | B | 107 | 19.158 | 56.658 | 95.846 | 1.00 | 15.60 | B | C |
| ATOM | 3071 | CB | CYS | B | 107 | 19.976 | 56.605 | 97.140 | 1.00 | 20.85 | B | C |
| ATOM | 3072 | SG | CYS | B | 107 | 20.729 | 54.999 | 97.506 | 1.00 | 20.85 | B | S |
| ATOM | 3073 | C | CYS | B | 107 | 20.038 | 56.202 | 94.679 | 1.00 | 15.60 | B | C |
| ATOM | 3074 | O | CYS | B | 107 | 20.937 | 56.928 | 94.254 | 1.00 | 15.60 | B | O |
| ATOM | 3075 | N | ASN | B | 108 | 19.791 | 55.004 | 94.159 | 1.00 | 16.03 | B | N |
| ATOM | 3076 | CA | ASN | B | 108 | 20.595 | 54.509 | 93.052 | 1.00 | 16.03 | B | C |
| ATOM | 3077 | CB | ASN | B | 108 | 21.253 | 53.187 | 93.431 | 1.00 | 25.05 | B | C |
| ATOM | 3078 | CG | ASN | B | 108 | 22.450 | 53.381 | 94.322 | 1.00 | 25.05 | B | C |
| ATOM | 3079 | OD1 | ASN | B | 108 | 23.352 | 54.128 | 93.983 | 1.00 | 25.05 | B | O |
| ATOM | 3080 | ND2 | ASN | B | 108 | 22.471 | 52.710 | 95.461 | 1.00 | 25.05 | B | N |
| ATOM | 3081 | C | ASN | B | 108 | 19.860 | 54.359 | 91.717 | 1.00 | 16.03 | B | C |

| | | | | | | | | | | | | |
|---|---|---|---|---|---|---|---|---|---|---|---|---|
| ATOM | 3082 | O | ASN | B | 108 | 20.140 | 53.460 | 90.933 | 1.00 | 16.03 | B | O |
| ATOM | 3083 | N | ILE | B | 109 | 18.917 | 55.260 | 91.479 | 1.00 | 14.89 | B | N |
| ATOM | 3084 | CA | ILE | B | 109 | 18.140 | 55.304 | 90.254 | 1.00 | 14.89 | B | C |
| ATOM | 3085 | CB | ILE | B | 109 | 16.823 | 54.526 | 90.400 | 1.00 | 12.94 | B | C |
| ATOM | 3086 | CG2 | ILE | B | 109 | 15.971 | 54.688 | 89.151 | 1.00 | 12.94 | B | C |
| ATOM | 3087 | CG1 | ILE | B | 109 | 17.128 | 53.051 | 90.641 | 1.00 | 12.94 | B | C |
| ATOM | 3088 | CD1 | ILE | B | 109 | 15.906 | 52.167 | 90.712 | 1.00 | 12.94 | B | C |
| ATOM | 3089 | C | ILE | B | 109 | 17.869 | 56.784 | 90.043 | 1.00 | 14.89 | B | C |
| ATOM | 3090 | O | ILE | B | 109 | 17.498 | 57.473 | 90.985 | 1.00 | 14.89 | B | O |
| ATOM | 3091 | N | VAL | B | 110 | 18.073 | 57.296 | 88.834 | 1.00 | 9.20 | B | N |
| ATOM | 3092 | CA | VAL | B | 110 | 17.843 | 58.722 | 88.624 | 1.00 | 9.20 | B | C |
| ATOM | 3093 | CB | VAL | B | 110 | 18.210 | 59.198 | 87.193 | 1.00 | 13.61 | B | C |
| ATOM | 3094 | CG1 | VAL | B | 110 | 19.689 | 59.085 | 86.989 | 1.00 | 13.61 | B | C |
| ATOM | 3095 | CG2 | VAL | B | 110 | 17.459 | 58.407 | 86.146 | 1.00 | 13.61 | B | C |
| ATOM | 3096 | C | VAL | B | 110 | 16.413 | 59.110 | 88.911 | 1.00 | 9.20 | B | C |
| ATOM | 3097 | O | VAL | B | 110 | 15.481 | 58.435 | 88.497 | 1.00 | 9.20 | B | O |
| ATOM | 3098 | N | ARG | B | 111 | 16.255 | 60.217 | 89.619 | 1.00 | 27.63 | B | N |
| ATOM | 3099 | CA | ARG | B | 111 | 14.947 | 60.719 | 89.994 | 1.00 | 27.63 | B | C |
| ATOM | 3100 | CB | ARG | B | 111 | 15.085 | 61.601 | 91.241 | 1.00 | 47.05 | B | C |
| ATOM | 3101 | CG | ARG | B | 111 | 13.787 | 62.116 | 91.831 | 1.00 | 47.05 | B | C |
| ATOM | 3102 | CD | ARG | B | 111 | 14.088 | 63.222 | 92.817 | 1.00 | 47.05 | B | C |
| ATOM | 3103 | NE | ARG | B | 111 | 14.855 | 64.288 | 92.169 | 1.00 | 47.05 | B | N |
| ATOM | 3104 | CZ | ARG | B | 111 | 15.511 | 65.253 | 92.815 | 1.00 | 47.05 | B | C |
| ATOM | 3105 | NH1 | ARG | B | 111 | 15.491 | 65.290 | 94.144 | 1.00 | 47.05 | B | N |
| ATOM | 3106 | NH2 | ARG | B | 111 | 16.197 | 66.170 | 92.132 | 1.00 | 47.05 | B | N |
| ATOM | 3107 | C | ARG | B | 111 | 14.272 | 61.495 | 88.874 | 1.00 | 27.63 | B | C |
| ATOM | 3108 | O | ARG | B | 111 | 14.902 | 62.291 | 88.186 | 1.00 | 27.63 | B | O |
| ATOM | 3109 | N | LEU | B | 112 | 12.986 | 61.227 | 88.676 | 1.00 | 22.82 | B | N |
| ATOM | 3110 | CA | LEU | B | 112 | 12.201 | 61.929 | 87.674 | 1.00 | 22.82 | B | C |
| ATOM | 3111 | CB | LEU | B | 112 | 11.062 | 61.037 | 87.163 | 1.00 | 21.64 | B | C |
| ATOM | 3112 | CG | LEU | B | 112 | 10.025 | 61.737 | 86.278 | 1.00 | 21.64 | B | C |
| ATOM | 3113 | CD1 | LEU | B | 112 | 10.652 | 62.115 | 84.946 | 1.00 | 21.64 | B | C |
| ATOM | 3114 | CD2 | LEU | B | 112 | 8.823 | 60.829 | 86.087 | 1.00 | 21.64 | B | C |
| ATOM | 3115 | C | LEU | B | 112 | 11.634 | 63.130 | 88.431 | 1.00 | 22.82 | B | C |
| ATOM | 3116 | O | LEU | B | 112 | 10.841 | 62.974 | 89.353 | 1.00 | 22.82 | B | O |
| ATOM | 3117 | N | ARG | B | 113 | 12.055 | 64.330 | 88.056 | 1.00 | 27.81 | B | N |
| ATOM | 3118 | CA | ARG | B | 113 | 11.596 | 65.529 | 88.743 | 1.00 | 27.81 | B | C |
| ATOM | 3119 | CB | ARG | B | 113 | 12.650 | 66.619 | 88.612 | 1.00 | 43.06 | B | C |
| ATOM | 3120 | CG | ARG | B | 113 | 13.927 | 66.392 | 89.397 | 1.00 | 43.06 | B | C |
| ATOM | 3121 | CD | ARG | B | 113 | 14.995 | 67.304 | 88.830 | 1.00 | 43.06 | B | C |
| ATOM | 3122 | NE | ARG | B | 113 | 15.917 | 67.832 | 89.830 | 1.00 | 43.06 | B | N |
| ATOM | 3123 | CZ | ARG | B | 113 | 15.584 | 68.726 | 90.758 | 1.00 | 43.06 | B | C |
| ATOM | 3124 | NH1 | ARG | B | 113 | 14.337 | 69.196 | 90.822 | 1.00 | 43.06 | B | N |
| ATOM | 3125 | NH2 | ARG | B | 113 | 16.504 | 69.166 | 91.611 | 1.00 | 43.06 | B | N |
| ATOM | 3126 | C | ARG | B | 113 | 10.266 | 66.044 | 88.229 | 1.00 | 27.81 | B | C |
| ATOM | 3127 | O | ARG | B | 113 | 9.384 | 66.415 | 88.998 | 1.00 | 27.81 | B | O |
| ATOM | 3128 | N | TYR | B | 114 | 10.133 | 66.070 | 86.915 | 1.00 | 25.55 | B | N |
| ATOM | 3129 | CA | TYR | B | 114 | 8.922 | 66.531 | 86.279 | 1.00 | 25.55 | B | C |
| ATOM | 3130 | CB | TYR | B | 114 | 9.042 | 68.006 | 85.947 | 1.00 | 36.59 | B | C |
| ATOM | 3131 | CG | TYR | B | 114 | 9.207 | 68.885 | 87.152 | 1.00 | 36.59 | B | C |
| ATOM | 3132 | CD1 | TYR | B | 114 | 8.181 | 69.010 | 88.082 | 1.00 | 36.59 | B | C |
| ATOM | 3133 | CE1 | TYR | B | 114 | 8.320 | 69.818 | 89.193 | 1.00 | 36.59 | B | C |
| ATOM | 3134 | CD2 | TYR | B | 114 | 10.389 | 69.595 | 87.367 | 1.00 | 36.59 | B | C |
| ATOM | 3135 | CE2 | TYR | B | 114 | 10.541 | 70.405 | 88.479 | 1.00 | 36.59 | B | C |
| ATOM | 3136 | CZ | TYR | B | 114 | 9.499 | 70.515 | 89.393 | 1.00 | 36.59 | B | C |
| ATOM | 3137 | OH | TYR | B | 114 | 9.620 | 71.310 | 90.517 | 1.00 | 36.59 | B | O |
| ATOM | 3138 | C | TYR | B | 114 | 8.779 | 65.763 | 84.981 | 1.00 | 25.55 | B | C |
| ATOM | 3139 | O | TYR | B | 114 | 9.599 | 64.904 | 84.635 | 1.00 | 25.55 | B | O |
| ATOM | 3140 | N | PHE | B | 115 | 7.703 | 66.084 | 84.281 | 1.00 | 29.31 | B | N |
| ATOM | 3141 | CA | PHE | B | 115 | 7.410 | 65.539 | 82.979 | 1.00 | 29.31 | B | C |
| ATOM | 3142 | CB | PHE | B | 115 | 6.990 | 64.059 | 83.041 | 1.00 | 22.81 | B | C |
| ATOM | 3143 | CG | PHE | B | 115 | 5.648 | 63.804 | 83.666 | 1.00 | 22.81 | B | C |
| ATOM | 3144 | CD1 | PHE | B | 115 | 4.516 | 63.631 | 82.873 | 1.00 | 22.81 | B | C |

| | | | | | | | | | | | | |
|---|---|---|---|---|---|---|---|---|---|---|---|---|
| ATOM | 3145 | CD2 | PHE | B | 115 | 5.526 | 63.655 | 85.041 | 1.00 | 22.81 | B | C |
| ATOM | 3146 | CE1 | PHE | B | 115 | 3.284 | 63.312 | 83.440 | 1.00 | 22.81 | B | C |
| ATOM | 3147 | CE2 | PHE | B | 115 | 4.287 | 63.333 | 85.620 | 1.00 | 22.81 | B | C |
| ATOM | 3148 | CZ | PHE | B | 115 | 3.169 | 63.159 | 84.813 | 1.00 | 22.81 | B | C |
| ATOM | 3149 | C | PHE | B | 115 | 6.319 | 66.460 | 82.482 | 1.00 | 29.31 | B | C |
| ATOM | 3150 | O | PHE | B | 115 | 5.529 | 66.974 | 83.273 | 1.00 | 29.31 | B | O |
| ATOM | 3151 | N | PHE | B | 116 | 6.325 | 66.719 | 81.182 | 1.00 | 28.12 | B | N |
| ATOM | 3152 | CA | PHE | B | 116 | 5.353 | 67.601 | 80.566 | 1.00 | 28.12 | B | C |
| ATOM | 3153 | CB | PHE | B | 116 | 5.793 | 69.060 | 80.765 | 1.00 | 25.10 | B | C |
| ATOM | 3154 | CG | PHE | B | 116 | 7.147 | 69.392 | 80.170 | 1.00 | 25.10 | B | C |
| ATOM | 3155 | CD1 | PHE | B | 116 | 7.268 | 69.803 | 78.844 | 1.00 | 25.10 | B | C |
| ATOM | 3156 | CD2 | PHE | B | 116 | 8.299 | 69.318 | 80.942 | 1.00 | 25.10 | B | C |
| ATOM | 3157 | CE1 | PHE | B | 116 | 8.522 | 70.144 | 78.305 | 1.00 | 25.10 | B | C |
| ATOM | 3158 | CE2 | PHE | B | 116 | 9.550 | 69.654 | 80.410 | 1.00 | 25.10 | B | C |
| ATOM | 3159 | CZ | PHE | B | 116 | 9.660 | 70.066 | 79.092 | 1.00 | 25.10 | B | C |
| ATOM | 3160 | C | PHE | B | 116 | 5.262 | 67.272 | 79.084 | 1.00 | 28.12 | B | C |
| ATOM | 3161 | O | PHE | B | 116 | 6.153 | 66.630 | 78.534 | 1.00 | 28.12 | B | O |
| ATOM | 3162 | N | TYR | B | 117 | 4.188 | 67.703 | 78.433 | 1.00 | 23.48 | B | N |
| ATOM | 3163 | CA | TYR | B | 117 | 4.052 | 67.426 | 77.019 | 1.00 | 23.48 | B | C |
| ATOM | 3164 | CB | TYR | B | 117 | 2.682 | 66.862 | 76.717 | 1.00 | 19.00 | B | C |
| ATOM | 3165 | CG | TYR | B | 117 | 2.443 | 65.586 | 77.448 | 1.00 | 19.00 | B | C |
| ATOM | 3166 | CD1 | TYR | B | 117 | 1.994 | 65.597 | 78.767 | 1.00 | 19.00 | B | C |
| ATOM | 3167 | CE1 | TYR | B | 117 | 1.790 | 64.415 | 79.462 | 1.00 | 19.00 | B | C |
| ATOM | 3168 | CD2 | TYR | B | 117 | 2.690 | 64.360 | 76.842 | 1.00 | 19.00 | B | C |
| ATOM | 3169 | CE2 | TYR | B | 117 | 2.489 | 63.171 | 77.535 | 1.00 | 19.00 | B | C |
| ATOM | 3170 | CZ | TYR | B | 117 | 2.035 | 63.214 | 78.841 | 1.00 | 19.00 | B | C |
| ATOM | 3171 | OH | TYR | B | 117 | 1.795 | 62.055 | 79.523 | 1.00 | 19.00 | B | O |
| ATOM | 3172 | C | TYR | B | 117 | 4.309 | 68.638 | 76.153 | 1.00 | 23.48 | B | C |
| ATOM | 3173 | O | TYR | B | 117 | 3.933 | 69.754 | 76.502 | 1.00 | 23.48 | B | O |
| ATOM | 3174 | N | SER | B | 118 | 4.988 | 68.395 | 75.032 | 1.00 | 36.96 | B | N |
| ATOM | 3175 | CA | SER | B | 118 | 5.306 | 69.421 | 74.056 | 1.00 | 36.96 | B | C |
| ATOM | 3176 | CB | SER | B | 118 | 6.768 | 69.877 | 74.154 | 1.00 | 30.83 | B | C |
| ATOM | 3177 | OG | SER | B | 118 | 7.674 | 68.814 | 73.940 | 1.00 | 30.83 | B | O |
| ATOM | 3178 | C | SER | B | 118 | 5.008 | 68.890 | 72.681 | 1.00 | 36.96 | B | C |
| ATOM | 3179 | O | SER | B | 118 | 5.008 | 67.685 | 72.463 | 1.00 | 36.96 | B | O |
| ATOM | 3180 | N | SER | B | 119 | 4.708 | 69.779 | 71.751 | 1.00 | 77.94 | B | N |
| ATOM | 3181 | CA | SER | B | 119 | 4.423 | 69.320 | 70.410 | 1.00 | 77.94 | B | C |
| ATOM | 3182 | CB | SER | B | 119 | 3.365 | 70.200 | 69.759 | 1.00 | 91.50 | B | C |
| ATOM | 3183 | OG | SER | B | 119 | 3.921 | 71.480 | 69.509 | 1.00 | 91.50 | B | O |
| ATOM | 3184 | C | SER | B | 119 | 5.721 | 69.405 | 69.605 | 1.00 | 77.94 | B | C |
| ATOM | 3185 | O | SER | B | 119 | 6.828 | 69.497 | 70.179 | 1.00 | 77.94 | B | O |
| ATOM | 3186 | N | GLY | B | 120 | 5.572 | 69.379 | 68.281 | 1.00100.00 | | B | N |
| ATOM | 3187 | CA | GLY | B | 120 | 6.718 | 69.460 | 67.400 | 1.00100.00 | | B | C |
| ATOM | 3188 | C | GLY | B | 120 | 6.239 | 69.569 | 65.967 | 1.00100.00 | | B | C |
| ATOM | 3189 | O | GLY | B | 120 | 5.079 | 69.931 | 65.723 | 1.00100.00 | | B | O |
| ATOM | 3190 | N | ALA | B | 121 | 7.138 | 69.251 | 65.034 | 1.00100.00 | | B | N |
| ATOM | 3191 | CA | ALA | B | 121 | 6.876 | 69.279 | 63.594 | 1.00100.00 | | B | C |
| ATOM | 3192 | CB | ALA | B | 121 | 7.581 | 68.085 | 62.930 | 1.00 | 45.94 | B | C |
| ATOM | 3193 | C | ALA | B | 121 | 5.383 | 69.284 | 63.213 | 1.00100.00 | | B | C |
| ATOM | 3194 | O | ALA | B | 121 | 4.753 | 70.355 | 63.094 | 1.00100.00 | | B | O |
| ATOM | 3195 | N | ALA | B | 122 | 4.827 | 68.085 | 63.029 | 1.00100.00 | | B | N |
| ATOM | 3196 | CA | ALA | B | 122 | 3.422 | 67.932 | 62.647 | 1.00100.00 | | B | C |
| ATOM | 3197 | CB | ALA | B | 122 | 3.099 | 66.444 | 62.447 | 1.00 | 67.88 | B | C |
| ATOM | 3198 | C | ALA | B | 122 | 2.440 | 68.538 | 63.659 | 1.00100.00 | | B | C |
| ATOM | 3199 | O | ALA | B | 122 | 2.838 | 69.101 | 64.693 | 1.00100.00 | | B | O |
| ATOM | 3200 | N | ALA | B | 123 | 1.150 | 68.417 | 63.352 | 1.00100.00 | | B | N |
| ATOM | 3201 | CA | ALA | B | 123 | 0.109 | 68.928 | 64.240 | 1.00100.00 | | B | C |
| ATOM | 3202 | CB | ALA | B | 123 | -1.236 | 69.028 | 63.487 | 1.00 | 77.16 | B | C |
| ATOM | 3203 | C | ALA | B | 123 | -0.007 | 67.959 | 65.420 | 1.00100.00 | | B | C |
| ATOM | 3204 | O | ALA | B | 123 | 0.183 | 68.358 | 66.583 | 1.00100.00 | | B | O |
| ATOM | 3205 | N | ALA | B | 124 | -0.295 | 66.688 | 65.108 | 1.00 | 89.24 | B | N |
| ATOM | 3206 | CA | ALA | B | 124 | -0.438 | 65.637 | 66.125 | 1.00 | 89.24 | B | C |
| ATOM | 3207 | CB | ALA | B | 124 | -0.941 | 64.344 | 65.471 | 1.00 | 57.79 | B | C |

| | | | | | | | | | | | | |
|---|---|---|---|---|---|---|---|---|---|---|---|---|
| ATOM | 3208 | C | ALA | B | 124 | 0.894 | 65.372 | 66.845 | 1.00 | 89.24 | B | C |
| ATOM | 3209 | O | ALA | B | 124 | 0.922 | 64.768 | 67.925 | 1.00 | 89.24 | B | O |
| ATOM | 3210 | N | ALA | B | 125 | 1.988 | 65.831 | 66.230 | 1.00 | 67.84 | B | N |
| ATOM | 3211 | CA | ALA | B | 125 | 3.346 | 65.657 | 66.764 | 1.00 | 67.84 | B | C |
| ATOM | 3212 | CB | ALA | B | 125 | 4.353 | 66.481 | 65.913 | 1.00 | 67.70 | B | C |
| ATOM | 3213 | C | ALA | B | 125 | 3.491 | 66.031 | 68.244 | 1.00 | 67.84 | B | C |
| ATOM | 3214 | O | ALA | B | 125 | 4.064 | 67.079 | 68.562 | 1.00 | 67.84 | B | O |
| ATOM | 3215 | N | VAL | B | 126 | 2.994 | 65.173 | 69.141 | 1.00 | 47.71 | B | N |
| ATOM | 3216 | CA | VAL | B | 126 | 3.089 | 65.451 | 70.579 | 1.00 | 47.71 | B | C |
| ATOM | 3217 | CB | VAL | B | 126 | 1.764 | 65.122 | 71.324 | 1.00 | 45.00 | B | C |
| ATOM | 3218 | CG1 | VAL | B | 126 | 0.629 | 65.950 | 70.729 | 1.00 | 45.00 | B | C |
| ATOM | 3219 | CG2 | VAL | B | 126 | 1.458 | 63.627 | 71.241 | 1.00 | 45.00 | B | C |
| ATOM | 3220 | C | VAL | B | 126 | 4.251 | 64.737 | 71.288 | 1.00 | 47.71 | B | C |
| ATOM | 3221 | O | VAL | B | 126 | 4.495 | 63.527 | 71.105 | 1.00 | 47.71 | B | O |
| ATOM | 3222 | N | TYR | B | 127 | 4.944 | 65.511 | 72.122 | 1.00 | 44.73 | B | N |
| ATOM | 3223 | CA | TYR | B | 127 | 6.103 | 65.034 | 72.855 | 1.00 | 44.73 | B | C |
| ATOM | 3224 | CB | TYR | B | 127 | 7.328 | 65.842 | 72.434 | 1.00 | 53.40 | B | C |
| ATOM | 3225 | CG | TYR | B | 127 | 7.758 | 65.570 | 71.023 | 1.00 | 53.40 | B | C |
| ATOM | 3226 | CD1 | TYR | B | 127 | 8.239 | 64.314 | 70.652 | 1.00 | 53.40 | B | C |
| ATOM | 3227 | CE1 | TYR | B | 127 | 8.623 | 64.044 | 69.334 | 1.00 | 53.40 | B | C |
| ATOM | 3228 | CD2 | TYR | B | 127 | 7.667 | 66.554 | 70.050 | 1.00 | 53.40 | B | C |
| ATOM | 3229 | CE2 | TYR | B | 127 | 8.047 | 66.299 | 68.724 | 1.00 | 53.40 | B | C |
| ATOM | 3230 | CZ | TYR | B | 127 | 8.522 | 65.043 | 68.377 | 1.00 | 53.40 | B | C |
| ATOM | 3231 | OH | TYR | B | 127 | 8.877 | 64.789 | 67.073 | 1.00 | 53.40 | B | O |
| ATOM | 3232 | C | TYR | B | 127 | 6.090 | 64.925 | 74.368 | 1.00 | 44.73 | B | C |
| ATOM | 3233 | O | TYR | B | 127 | 5.765 | 65.881 | 75.070 | 1.00 | 44.73 | B | O |
| ATOM | 3234 | N | LEU | B | 128 | 6.465 | 63.741 | 74.855 | 1.00 | 40.10 | B | N |
| ATOM | 3235 | CA | LEU | B | 128 | 6.568 | 63.475 | 76.287 | 1.00 | 40.10 | B | C |
| ATOM | 3236 | CB | LEU | B | 128 | 6.346 | 62.002 | 76.594 | 1.00 | 21.48 | B | C |
| ATOM | 3237 | CG | LEU | B | 128 | 6.848 | 61.582 | 77.974 | 1.00 | 21.48 | B | C |
| ATOM | 3238 | CD1 | LEU | B | 128 | 6.107 | 62.352 | 79.045 | 1.00 | 21.48 | B | C |
| ATOM | 3239 | CD2 | LEU | B | 128 | 6.664 | 60.085 | 78.153 | 1.00 | 21.48 | B | C |
| ATOM | 3240 | C | LEU | B | 128 | 7.978 | 63.848 | 76.698 | 1.00 | 40.10 | B | C |
| ATOM | 3241 | O | LEU | B | 128 | 8.941 | 63.320 | 76.146 | 1.00 | 40.10 | B | O |
| ATOM | 3242 | N | ASN | B | 129 | 8.100 | 64.763 | 77.656 | 1.00 | 29.10 | B | N |
| ATOM | 3243 | CA | ASN | B | 129 | 9.401 | 65.213 | 78.140 | 1.00 | 29.10 | B | C |
| ATOM | 3244 | CB | ASN | B | 129 | 9.465 | 66.736 | 78.125 | 1.00 | 25.06 | B | C |
| ATOM | 3245 | CG | ASN | B | 129 | 9.279 | 67.309 | 76.742 | 1.00 | 25.06 | B | C |
| ATOM | 3246 | OD1 | ASN | B | 129 | 10.244 | 67.565 | 76.033 | 1.00 | 25.06 | B | O |
| ATOM | 3247 | ND2 | ASN | B | 129 | 8.031 | 67.505 | 76.343 | 1.00 | 25.06 | B | N |
| ATOM | 3248 | C | ASN | B | 129 | 9.624 | 64.724 | 79.562 | 1.00 | 29.10 | B | C |
| ATOM | 3249 | O | ASN | B | 129 | 8.817 | 64.993 | 80.444 | 1.00 | 29.10 | B | O |
| ATOM | 3250 | N | LEU | B | 130 | 10.721 | 64.003 | 79.778 | 1.00 | 30.84 | B | N |
| ATOM | 3251 | CA | LEU | B | 130 | 11.065 | 63.485 | 81.098 | 1.00 | 30.84 | B | C |
| ATOM | 3252 | CB | LEU | B | 130 | 11.469 | 62.012 | 80.993 | 1.00 | 16.04 | B | C |
| ATOM | 3253 | CG | LEU | B | 130 | 10.420 | 60.903 | 80.959 | 1.00 | 16.04 | B | C |
| ATOM | 3254 | CD1 | LEU | B | 130 | 9.127 | 61.396 | 80.363 | 1.00 | 16.04 | B | C |
| ATOM | 3255 | CD2 | LEU | B | 130 | 10.974 | 59.746 | 80.157 | 1.00 | 16.04 | B | C |
| ATOM | 3256 | C | LEU | B | 130 | 12.211 | 64.286 | 81.703 | 1.00 | 30.84 | B | C |
| ATOM | 3257 | O | LEU | B | 130 | 13.365 | 64.133 | 81.311 | 1.00 | 30.84 | B | O |
| ATOM | 3258 | N | VAL | B | 131 | 11.889 | 65.139 | 82.666 | 1.00 | 20.22 | B | N |
| ATOM | 3259 | CA | VAL | B | 131 | 12.892 | 65.960 | 83.320 | 1.00 | 20.22 | B | C |
| ATOM | 3260 | CB | VAL | B | 131 | 12.295 | 67.309 | 83.747 | 1.00 | 18.78 | B | C |
| ATOM | 3261 | CG1 | VAL | B | 131 | 13.370 | 68.189 | 84.365 | 1.00 | 18.78 | B | C |
| ATOM | 3262 | CG2 | VAL | B | 131 | 11.677 | 67.991 | 82.537 | 1.00 | 18.78 | B | C |
| ATOM | 3263 | C | VAL | B | 131 | 13.477 | 65.232 | 84.525 | 1.00 | 20.22 | B | C |
| ATOM | 3264 | O | VAL | B | 131 | 12.930 | 65.249 | 85.626 | 1.00 | 20.22 | B | O |
| ATOM | 3265 | N | LEU | B | 132 | 14.614 | 64.590 | 84.285 | 1.00 | 28.82 | B | N |
| ATOM | 3266 | CA | LEU | B | 132 | 15.315 | 63.820 | 85.305 | 1.00 | 28.82 | B | C |
| ATOM | 3267 | CB | LEU | B | 132 | 15.754 | 62.489 | 84.703 | 1.00 | 8.95 | B | C |
| ATOM | 3268 | CG | LEU | B | 132 | 14.658 | 61.829 | 83.873 | 1.00 | 8.95 | B | C |
| ATOM | 3269 | CD1 | LEU | B | 132 | 14.971 | 61.954 | 82.415 | 1.00 | 8.95 | B | C |
| ATOM | 3270 | CD2 | LEU | B | 132 | 14.546 | 60.390 | 84.266 | 1.00 | 8.95 | B | C |

| | | | | | | | | | | | | |
|---|---|---|---|---|---|---|---|---|---|---|---|---|
| ATOM | 3271 | C | LEU | B | 132 | 16.532 | 64.571 | 85.815 | 1.00 | 28.82 | B | C |
| ATOM | 3272 | O | LEU | B | 132 | 16.911 | 65.591 | 85.252 | 1.00 | 28.82 | B | O |
| ATOM | 3273 | N | ASP | B | 133 | 17.132 | 64.064 | 86.886 | 1.00 | 33.82 | B | N |
| ATOM | 3274 | CA | ASP | B | 133 | 18.337 | 64.662 | 87.467 | 1.00 | 33.82 | B | C |
| ATOM | 3275 | CB | ASP | B | 133 | 18.765 | 63.885 | 88.705 | 1.00 | 30.44 | B | C |
| ATOM | 3276 | CG | ASP | B | 133 | 18.147 | 64.419 | 89.962 | 1.00 | 30.44 | B | C |
| ATOM | 3277 | OD1 | ASP | B | 133 | 18.012 | 63.630 | 90.925 | 1.00 | 30.44 | B | O |
| ATOM | 3278 | OD2 | ASP | B | 133 | 17.814 | 65.629 | 89.990 | 1.00 | 30.44 | B | O |
| ATOM | 3279 | C | ASP | B | 133 | 19.461 | 64.594 | 86.452 | 1.00 | 33.82 | B | C |
| ATOM | 3280 | O | ASP | B | 133 | 19.354 | 63.897 | 85.459 | 1.00 | 33.82 | B | O |
| ATOM | 3281 | N | TYR | B | 134 | 20.544 | 65.311 | 86.688 | 1.00 | 20.94 | B | N |
| ATOM | 3282 | CA | TYR | B | 134 | 21.658 | 65.244 | 85.754 | 1.00 | 20.94 | B | C |
| ATOM | 3283 | CB | TYR | B | 134 | 22.052 | 66.639 | 85.256 | 1.00 | 28.15 | B | C |
| ATOM | 3284 | CG | TYR | B | 134 | 23.213 | 66.602 | 84.290 | 1.00 | 28.15 | B | C |
| ATOM | 3285 | CD1 | TYR | B | 134 | 23.013 | 66.291 | 82.944 | 1.00 | 28.15 | B | C |
| ATOM | 3286 | CE1 | TYR | B | 134 | 24.077 | 66.146 | 82.080 | 1.00 | 28.15 | B | C |
| ATOM | 3287 | CD2 | TYR | B | 134 | 24.521 | 66.777 | 84.739 | 1.00 | 28.15 | B | C |
| ATOM | 3288 | CE2 | TYR | B | 134 | 25.594 | 66.633 | 83.879 | 1.00 | 28.15 | B | C |
| ATOM | 3289 | CZ | TYR | B | 134 | 25.364 | 66.312 | 82.555 | 1.00 | 28.15 | B | C |
| ATOM | 3290 | OH | TYR | B | 134 | 26.427 | 66.116 | 81.714 | 1.00 | 28.15 | B | O |
| ATOM | 3291 | C | TYR | B | 134 | 22.864 | 64.589 | 86.416 | 1.00 | 20.94 | B | C |
| ATOM | 3292 | O | TYR | B | 134 | 23.187 | 64.901 | 87.554 | 1.00 | 20.94 | B | O |
| ATOM | 3293 | N | VAL | B | 135 | 23.499 | 63.660 | 85.711 | 1.00 | 32.22 | B | N |
| ATOM | 3294 | CA | VAL | B | 135 | 24.694 | 62.998 | 86.222 | 1.00 | 32.22 | B | C |
| ATOM | 3295 | CB | VAL | B | 135 | 24.467 | 61.486 | 86.466 | 1.00 | 23.09 | B | C |
| ATOM | 3296 | CG1 | VAL | B | 135 | 25.578 | 60.929 | 87.359 | 1.00 | 23.09 | B | C |
| ATOM | 3297 | CG2 | VAL | B | 135 | 23.115 | 61.265 | 87.105 | 1.00 | 23.09 | B | C |
| ATOM | 3298 | C | VAL | B | 135 | 25.761 | 63.214 | 85.136 | 1.00 | 32.22 | B | C |
| ATOM | 3299 | O | VAL | B | 135 | 25.524 | 62.920 | 83.963 | 1.00 | 32.22 | B | O |
| ATOM | 3300 | N | PRO | B | 136 | 26.944 | 63.734 | 85.523 | 1.00 | 21.91 | B | N |
| ATOM | 3301 | CD | PRO | B | 136 | 27.321 | 63.835 | 86.941 | 1.00 | 27.54 | B | C |
| ATOM | 3302 | CA | PRO | B | 136 | 28.099 | 64.043 | 84.669 | 1.00 | 21.91 | B | C |
| ATOM | 3303 | CB | PRO | B | 136 | 29.124 | 64.619 | 85.644 | 1.00 | 27.54 | B | C |
| ATOM | 3304 | CG | PRO | B | 136 | 28.380 | 64.868 | 86.898 | 1.00 | 27.54 | B | C |
| ATOM | 3305 | C | PRO | B | 136 | 28.740 | 62.934 | 83.842 | 1.00 | 21.91 | B | C |
| ATOM | 3306 | O | PRO | B | 136 | 29.275 | 63.204 | 82.780 | 1.00 | 21.91 | B | O |
| ATOM | 3307 | N | GLU | B | 137 | 28.733 | 61.698 | 84.321 | 1.00 | 23.98 | B | N |
| ATOM | 3308 | CA | GLU | B | 137 | 29.379 | 60.650 | 83.547 | 1.00 | 23.98 | B | C |
| ATOM | 3309 | CB | GLU | B | 137 | 30.815 | 60.442 | 84.058 | 1.00 | 38.31 | B | C |
| ATOM | 3310 | CG | GLU | B | 137 | 31.842 | 60.146 | 82.962 | 1.00 | 38.31 | B | C |
| ATOM | 3311 | CD | GLU | B | 137 | 32.361 | 61.399 | 82.297 | 1.00 | 38.31 | B | C |
| ATOM | 3312 | OE1 | GLU | B | 137 | 32.824 | 61.308 | 81.147 | 1.00 | 38.31 | B | O |
| ATOM | 3313 | OE2 | GLU | B | 137 | 32.323 | 62.476 | 82.930 | 1.00 | 38.31 | B | O |
| ATOM | 3314 | C | GLU | B | 137 | 28.633 | 59.321 | 83.540 | 1.00 | 23.98 | B | C |
| ATOM | 3315 | O | GLU | B | 137 | 27.537 | 59.196 | 84.090 | 1.00 | 23.98 | B | O |
| ATOM | 3316 | N | THR | B | 138 | 29.239 | 58.329 | 82.902 | 1.00 | 24.20 | B | N |
| ATOM | 3317 | CA | THR | B | 138 | 28.640 | 57.009 | 82.806 | 1.00 | 24.20 | B | C |
| ATOM | 3318 | CB | THR | B | 138 | 27.845 | 56.855 | 81.509 | 1.00 | 29.04 | B | C |
| ATOM | 3319 | OG1 | THR | B | 138 | 28.753 | 56.753 | 80.409 | 1.00 | 29.04 | B | O |
| ATOM | 3320 | CG2 | THR | B | 138 | 26.940 | 58.054 | 81.296 | 1.00 | 29.04 | B | C |
| ATOM | 3321 | C | THR | B | 138 | 29.721 | 55.950 | 82.809 | 1.00 | 24.20 | B | C |
| ATOM | 3322 | O | THR | B | 138 | 30.820 | 56.190 | 82.346 | 1.00 | 24.20 | B | O |
| ATOM | 3323 | N | VAL | B | 139 | 29.398 | 54.770 | 83.322 | 1.00 | 29.48 | B | N |
| ATOM | 3324 | CA | VAL | B | 139 | 30.357 | 53.677 | 83.364 | 1.00 | 29.48 | B | C |
| ATOM | 3325 | CB | VAL | B | 139 | 29.674 | 52.377 | 83.859 | 1.00 | 23.63 | B | C |
| ATOM | 3326 | CG1 | VAL | B | 139 | 30.486 | 51.151 | 83.443 | 1.00 | 23.63 | B | C |
| ATOM | 3327 | CG2 | VAL | B | 139 | 29.544 | 52.416 | 85.383 | 1.00 | 23.63 | B | C |
| ATOM | 3328 | C | VAL | B | 139 | 31.010 | 53.439 | 81.999 | 1.00 | 29.48 | B | C |
| ATOM | 3329 | O | VAL | B | 139 | 32.197 | 53.161 | 81.912 | 1.00 | 29.48 | B | O |
| ATOM | 3330 | N | TYR | B | 140 | 30.231 | 53.553 | 80.935 | 1.00 | 30.63 | B | N |
| ATOM | 3331 | CA | TYR | B | 140 | 30.739 | 53.351 | 79.584 | 1.00 | 30.63 | B | C |
| ATOM | 3332 | CB | TYR | B | 140 | 29.648 | 53.685 | 78.560 | 1.00 | 34.08 | B | C |
| ATOM | 3333 | CG | TYR | B | 140 | 30.130 | 53.605 | 77.138 | 1.00 | 34.08 | B | C |

| | | | | | | | | | | | | |
|---|---|---|---|---|---|---|---|---|---|---|---|---|
| ATOM | 3334 | CD1 | TYR | B | 140 | 30.244 | 52.383 | 76.482 | 1.00 | 34.08 | B | C |
| ATOM | 3335 | CE1 | TYR | B | 140 | 30.785 | 52.306 | 75.188 | 1.00 | 34.08 | B | C |
| ATOM | 3336 | CD2 | TYR | B | 140 | 30.557 | 54.756 | 76.470 | 1.00 | 34.08 | B | C |
| ATOM | 3337 | CE2 | TYR | B | 140 | 31.093 | 54.698 | 75.188 | 1.00 | 34.08 | B | C |
| ATOM | 3338 | CZ | TYR | B | 140 | 31.213 | 53.474 | 74.548 | 1.00 | 34.08 | B | C |
| ATOM | 3339 | OH | TYR | B | 140 | 31.795 | 53.434 | 73.291 | 1.00 | 34.08 | B | O |
| ATOM | 3340 | C | TYR | B | 140 | 31.976 | 54.224 | 79.340 | 1.00 | 30.63 | B | C |
| ATOM | 3341 | O | TYR | B | 140 | 33.049 | 53.717 | 78.999 | 1.00 | 30.63 | B | O |
| ATOM | 3342 | N | ARG | B | 141 | 31.813 | 55.536 | 79.517 | 1.00 | 24.15 | B | N |
| ATOM | 3343 | CA | ARG | B | 141 | 32.897 | 56.500 | 79.332 | 1.00 | 24.15 | B | C |
| ATOM | 3344 | CB | ARG | B | 141 | 32.367 | 57.928 | 79.501 | 1.00 | 27.54 | B | C |
| ATOM | 3345 | CG | ARG | B | 141 | 31.493 | 58.421 | 78.370 | 1.00 | 27.54 | B | C |
| ATOM | 3346 | CD | ARG | B | 141 | 30.514 | 59.450 | 78.896 | 1.00 | 27.54 | B | C |
| ATOM | 3347 | NE | ARG | B | 141 | 31.167 | 60.669 | 79.362 | 1.00 | 27.54 | B | N |
| ATOM | 3348 | CZ | ARG | B | 141 | 31.475 | 61.701 | 78.576 | 1.00 | 27.54 | B | C |
| ATOM | 3349 | NH1 | ARG | B | 141 | 31.185 | 61.668 | 77.282 | 1.00 | 27.54 | B | N |
| ATOM | 3350 | NH2 | ARG | B | 141 | 32.077 | 62.768 | 79.086 | 1.00 | 27.54 | B | N |
| ATOM | 3351 | C | ARG | B | 141 | 34.065 | 56.281 | 80.287 | 1.00 | 24.15 | B | C |
| ATOM | 3352 | O | ARG | B | 141 | 35.207 | 56.247 | 79.869 | 1.00 | 24.15 | B | O |
| ATOM | 3353 | N | VAL | B | 142 | 33.783 | 56.131 | 81.579 | 1.00 | 27.57 | B | N |
| ATOM | 3354 | CA | VAL | B | 142 | 34.830 | 55.939 | 82.588 | 1.00 | 27.57 | B | C |
| ATOM | 3355 | CB | VAL | B | 142 | 34.216 | 55.826 | 84.005 | 1.00 | 9.19 | B | C |
| ATOM | 3356 | CG1 | VAL | B | 142 | 35.271 | 55.447 | 85.013 | 1.00 | 9.19 | B | C |
| ATOM | 3357 | CG2 | VAL | B | 142 | 33.582 | 57.138 | 84.401 | 1.00 | 9.19 | B | C |
| ATOM | 3358 | C | VAL | B | 142 | 35.717 | 54.720 | 82.353 | 1.00 | 27.57 | B | C |
| ATOM | 3359 | O | VAL | B | 142 | 36.851 | 54.694 | 82.814 | 1.00 | 27.57 | B | O |
| ATOM | 3360 | N | ALA | B | 143 | 35.203 | 53.724 | 81.651 | 1.00 | 43.49 | B | N |
| ATOM | 3361 | CA | ALA | B | 143 | 35.939 | 52.487 | 81.396 | 1.00 | 43.49 | B | C |
| ATOM | 3362 | CB | ALA | B | 143 | 35.005 | 51.318 | 81.572 | 1.00 | 2.21 | B | C |
| ATOM | 3363 | C | ALA | B | 143 | 36.600 | 52.427 | 80.012 | 1.00 | 43.49 | B | C |
| ATOM | 3364 | O | ALA | B | 143 | 37.169 | 51.384 | 79.632 | 1.00 | 43.49 | B | O |
| ATOM | 3365 | N | ARG | B | 144 | 36.519 | 53.528 | 79.274 | 1.00 | 30.01 | B | N |
| ATOM | 3366 | CA | ARG | B | 144 | 37.098 | 53.639 | 77.941 | 1.00 | 30.01 | B | C |
| ATOM | 3367 | CB | ARG | B | 144 | 36.089 | 54.267 | 76.948 | 1.00 | 99.82 | B | C |
| ATOM | 3368 | CG | ARG | B | 144 | 35.421 | 55.584 | 77.375 | 1.00 | 99.82 | B | C |
| ATOM | 3369 | CD | ARG | B | 144 | 36.373 | 56.741 | 77.125 | 1.00 | 99.82 | B | C |
| ATOM | 3370 | NE | ARG | B | 144 | 35.873 | 58.054 | 77.540 | 1.00 | 99.82 | B | N |
| ATOM | 3371 | CZ | ARG | B | 144 | 36.583 | 59.166 | 77.376 | 1.00 | 99.82 | B | C |
| ATOM | 3372 | NH1 | ARG | B | 144 | 36.124 | 60.371 | 77.757 | 1.00 | 99.82 | B | N |
| ATOM | 3373 | NH2 | ARG | B | 144 | 37.801 | 59.067 | 76.812 | 1.00 | 99.82 | B | N |
| ATOM | 3374 | C | ARG | B | 144 | 38.281 | 54.527 | 78.178 | 1.00 | 30.01 | B | C |
| ATOM | 3375 | O | ARG | B | 144 | 39.344 | 54.341 | 77.594 | 1.00 | 30.01 | B | O |
| ATOM | 3376 | N | HIS | B | 145 | 38.098 | 55.447 | 79.099 | 1.00 | 38.20 | B | N |
| ATOM | 3377 | CA | HIS | B | 145 | 39.182 | 56.313 | 79.433 | 1.00 | 38.20 | B | C |
| ATOM | 3378 | CB | HIS | B | 145 | 38.895 | 57.142 | 80.574 | 1.00 | 85.90 | B | C |
| ATOM | 3379 | CG | HIS | B | 145 | 40.106 | 57.832 | 81.051 | 1.00 | 85.90 | B | C |
| ATOM | 3380 | CD2 | HIS | B | 145 | 40.504 | 59.097 | 80.795 | 1.00 | 85.90 | B | C |
| ATOM | 3381 | ND1 | HIS | B | 145 | 40.945 | 57.278 | 81.951 | 1.00 | 85.90 | B | N |
| ATOM | 3382 | CE1 | HIS | B | 145 | 41.821 | 58.239 | 82.325 | 1.00 | 85.90 | B | C |
| ATOM | 3383 | NE2 | HIS | B | 145 | 41.551 | 59.332 | 81.651 | 1.00 | 85.90 | B | N |
| ATOM | 3384 | C | HIS | B | 145 | 40.261 | 55.432 | 79.976 | 1.00 | 38.20 | B | C |
| ATOM | 3385 | O | HIS | B | 145 | 41.450 | 55.693 | 79.762 | 1.00 | 38.20 | B | O |
| ATOM | 3386 | N | TYR | B | 146 | 39.806 | 54.490 | 80.795 | 1.00 | 39.49 | B | N |
| ATOM | 3387 | CA | TYR | B | 146 | 40.629 | 53.496 | 81.403 | 1.00 | 39.49 | B | C |
| ATOM | 3388 | CB | TYR | B | 146 | 40.003 | 52.864 | 82.674 | 1.00 | 59.95 | B | C |
| ATOM | 3389 | CG | TYR | B | 146 | 40.530 | 53.483 | 83.907 | 1.00 | 59.95 | B | C |
| ATOM | 3390 | CD1 | TYR | B | 146 | 39.725 | 54.322 | 84.660 | 1.00 | 59.95 | B | C |
| ATOM | 3391 | CE1 | TYR | B | 146 | 40.208 | 55.005 | 85.730 | 1.00 | 59.95 | B | C |
| ATOM | 3392 | CD2 | TYR | B | 146 | 41.864 | 53.316 | 84.276 | 1.00 | 59.95 | B | C |
| ATOM | 3393 | CE2 | TYR | B | 146 | 42.373 | 53.995 | 85.346 | 1.00 | 59.95 | B | C |
| ATOM | 3394 | CZ | TYR | B | 146 | 41.536 | 54.849 | 86.068 | 1.00 | 59.95 | B | C |
| ATOM | 3395 | OH | TYR | B | 146 | 42.064 | 55.636 | 87.055 | 1.00 | 59.95 | B | O |
| ATOM | 3396 | C | TYR | B | 146 | 40.924 | 52.307 | 80.558 | 1.00 | 39.49 | B | C |

| | | | | | | | | | | | | |
|---|---|---|---|---|---|---|---|---|---|---|---|---|
| ATOM | 3397 | O | TYR | B | 146 | 41.779 | 51.492 | 80.928 | 1.00 | 39.49 | B | O |
| ATOM | 3398 | N | SER | B | 147 | 40.427 | 52.382 | 79.249 | 1.00 | 39.48 | B | N |
| ATOM | 3399 | CA | SER | B | 147 | 40.808 | 51.272 | 78.568 | 1.00 | 39.48 | B | C |
| ATOM | 3400 | CB | SER | B | 147 | 39.628 | 50.518 | 77.909 | 1.00 | 47.43 | B | C |
| ATOM | 3401 | OG | SER | B | 147 | 40.221 | 49.329 | 77.325 | 1.00 | 47.43 | B | O |
| ATOM | 3402 | C | SER | B | 147 | 41.825 | 51.809 | 77.692 | 1.00 | 39.48 | B | C |
| ATOM | 3403 | O | SER | B | 147 | 42.937 | 51.250 | 77.607 | 1.00 | 39.48 | B | O |
| ATOM | 3404 | N | ARG | B | 148 | 41.467 | 52.944 | 77.124 | 1.00 | 46.72 | B | N |
| ATOM | 3405 | CA | ARG | B | 148 | 42.278 | 53.645 | 76.189 | 1.00 | 46.72 | B | C |
| ATOM | 3406 | CB | ARG | B | 148 | 41.457 | 54.819 | 75.661 | 1.00 | 74.20 | B | C |
| ATOM | 3407 | CG | ARG | B | 148 | 41.155 | 54.719 | 74.166 | 1.00 | 74.20 | B | C |
| ATOM | 3408 | CD | ARG | B | 148 | 39.750 | 55.195 | 73.805 | 1.00 | 74.20 | B | C |
| ATOM | 3409 | NE | ARG | B | 148 | 39.434 | 56.464 | 74.451 | 1.00 | 74.20 | B | N |
| ATOM | 3410 | CZ | ARG | B | 148 | 38.491 | 57.301 | 74.021 | 1.00 | 74.20 | B | C |
| ATOM | 3411 | NH1 | ARG | B | 148 | 37.773 | 56.999 | 72.937 | 1.00 | 74.20 | B | N |
| ATOM | 3412 | NH2 | ARG | B | 148 | 38.260 | 58.439 | 74.669 | 1.00 | 74.20 | B | N |
| ATOM | 3413 | C | ARG | B | 148 | 43.617 | 54.115 | 76.742 | 1.00 | 46.72 | B | C |
| ATOM | 3414 | O | ARG | B | 148 | 44.469 | 54.546 | 75.978 | 1.00 | 46.72 | B | O |
| ATOM | 3415 | N | ALA | B | 149 | 43.811 | 54.052 | 78.053 | 1.00 | 48.34 | B | N |
| ATOM | 3416 | CA | ALA | B | 149 | 45.081 | 54.482 | 78.636 | 1.00 | 48.34 | B | C |
| ATOM | 3417 | CB | ALA | B | 149 | 44.824 | 55.460 | 79.772 | 1.00 | 23.93 | B | C |
| ATOM | 3418 | C | ALA | B | 149 | 45.865 | 53.261 | 79.143 | 1.00 | 48.34 | B | C |
| ATOM | 3419 | O | ALA | B | 149 | 46.673 | 53.356 | 80.077 | 1.00 | 48.34 | B | O |
| ATOM | 3420 | N | ALA | B | 150 | 45.603 | 52.112 | 78.528 | 1.00 | 44.48 | B | N |
| ATOM | 3421 | CA | ALA | B | 150 | 46.268 | 50.872 | 78.893 | 1.00 | 44.48 | B | C |
| ATOM | 3422 | CB | ALA | B | 150 | 47.758 | 51.012 | 78.690 | 1.00 | 58.73 | B | C |
| ATOM | 3423 | C | ALA | B | 150 | 45.989 | 50.397 | 80.319 | 1.00 | 44.48 | B | C |
| ATOM | 3424 | O | ALA | B | 150 | 46.576 | 49.414 | 80.764 | 1.00 | 44.48 | B | O |
| ATOM | 3425 | N | GLN | B | 151 | 45.098 | 51.057 | 81.047 | 1.00 | 50.44 | B | N |
| ATOM | 3426 | CA | GLN | B | 151 | 44.848 | 50.588 | 82.406 | 1.00 | 50.44 | B | C |
| ATOM | 3427 | CB | GLN | B | 151 | 45.099 | 51.687 | 83.418 | 1.00 | 55.44 | B | C |
| ATOM | 3428 | CG | GLN | B | 151 | 45.882 | 52.833 | 82.882 | 1.00 | 55.44 | B | C |
| ATOM | 3429 | CD | GLN | B | 151 | 46.335 | 53.722 | 83.992 | 1.00 | 55.44 | B | C |
| ATOM | 3430 | OE1 | GLN | B | 151 | 47.060 | 53.279 | 84.896 | 1.00 | 55.44 | B | O |
| ATOM | 3431 | NE2 | GLN | B | 151 | 45.906 | 54.982 | 83.955 | 1.00 | 55.44 | B | N |
| ATOM | 3432 | C | GLN | B | 151 | 43.468 | 50.043 | 82.694 | 1.00 | 50.44 | B | C |
| ATOM | 3433 | O | GLN | B | 151 | 42.620 | 49.978 | 81.832 | 1.00 | 50.44 | B | O |
| ATOM | 3434 | N | THR | B | 152 | 43.260 | 49.618 | 83.931 | 1.00 | 41.74 | B | N |
| ATOM | 3435 | CA | THR | B | 152 | 41.961 | 49.107 | 84.350 | 1.00 | 41.74 | B | C |
| ATOM | 3436 | CB | THR | B | 152 | 42.047 | 47.634 | 84.832 | 1.00 | 41.58 | B | C |
| ATOM | 3437 | OG1 | THR | B | 152 | 42.931 | 47.539 | 85.957 | 1.00 | 41.58 | B | O |
| ATOM | 3438 | CG2 | THR | B | 152 | 42.559 | 46.738 | 83.705 | 1.00 | 41.58 | B | C |
| ATOM | 3439 | C | THR | B | 152 | 41.477 | 49.977 | 85.503 | 1.00 | 41.74 | B | C |
| ATOM | 3440 | O | THR | B | 152 | 42.288 | 50.556 | 86.228 | 1.00 | 41.74 | B | O |
| ATOM | 3441 | N | LEU | B | 153 | 40.163 | 50.095 | 85.657 | 1.00 | 25.89 | B | N |
| ATOM | 3442 | CA | LEU | B | 153 | 39.603 | 50.892 | 86.740 | 1.00 | 25.89 | B | C |
| ATOM | 3443 | CB | LEU | B | 153 | 38.081 | 50.952 | 86.594 | 1.00 | 30.62 | B | C |
| ATOM | 3444 | CG | LEU | B | 153 | 37.250 | 51.421 | 87.798 | 1.00 | 30.62 | B | C |
| ATOM | 3445 | CD1 | LEU | B | 153 | 37.463 | 52.910 | 88.068 | 1.00 | 30.62 | B | C |
| ATOM | 3446 | CD2 | LEU | B | 153 | 35.774 | 51.139 | 87.515 | 1.00 | 30.62 | B | C |
| ATOM | 3447 | C | LEU | B | 153 | 39.970 | 50.221 | 88.072 | 1.00 | 25.89 | B | C |
| ATOM | 3448 | O | LEU | B | 153 | 39.873 | 49.000 | 88.202 | 1.00 | 25.89 | B | O |
| ATOM | 3449 | N | PRO | B | 154 | 40.431 | 51.004 | 89.065 | 1.00 | 23.15 | B | N |
| ATOM | 3450 | CD | PRO | B | 154 | 40.746 | 52.440 | 89.009 | 1.00 | 20.18 | B | C |
| ATOM | 3451 | CA | PRO | B | 154 | 40.795 | 50.442 | 90.372 | 1.00 | 23.15 | B | C |
| ATOM | 3452 | CB | PRO | B | 154 | 41.007 | 51.680 | 91.229 | 1.00 | 20.18 | B | C |
| ATOM | 3453 | CG | PRO | B | 154 | 41.571 | 52.634 | 90.265 | 1.00 | 20.18 | B | C |
| ATOM | 3454 | C | PRO | B | 154 | 39.645 | 49.570 | 90.891 | 1.00 | 23.15 | B | C |
| ATOM | 3455 | O | PRO | B | 154 | 38.487 | 49.854 | 90.619 | 1.00 | 23.15 | B | O |
| ATOM | 3456 | N | VAL | B | 155 | 39.941 | 48.513 | 91.636 | 1.00 | 28.64 | B | N |
| ATOM | 3457 | CA | VAL | B | 155 | 38.849 | 47.670 | 92.127 | 1.00 | 28.64 | B | C |
| ATOM | 3458 | CB | VAL | B | 155 | 39.345 | 46.320 | 92.713 | 1.00 | 29.01 | B | C |
| ATOM | 3459 | CG1 | VAL | B | 155 | 40.078 | 45.529 | 91.668 | 1.00 | 29.01 | B | C |

| | | | | | | | | | | | | |
|---|---|---|---|---|---|---|---|---|---|---|---|---|
| ATOM | 3460 | CG2 | VAL | B | 155 | 40.219 | 46.569 | 93.916 | 1.00 | 29.01 | B | C |
| ATOM | 3461 | C | VAL | B | 155 | 37.977 | 48.296 | 93.204 | 1.00 | 28.64 | B | C |
| ATOM | 3462 | O | VAL | B | 155 | 36.905 | 47.786 | 93.494 | 1.00 | 28.64 | B | O |
| ATOM | 3463 | N | ILE | B | 156 | 38.435 | 49.375 | 93.824 | 1.00 | 27.92 | B | N |
| ATOM | 3464 | CA | ILE | B | 156 | 37.632 | 49.986 | 94.861 | 1.00 | 27.92 | B | C |
| ATOM | 3465 | CB | ILE | B | 156 | 38.421 | 51.082 | 95.646 | 1.00 | 16.09 | B | C |
| ATOM | 3466 | CG2 | ILE | B | 156 | 38.942 | 52.160 | 94.718 | 1.00 | 16.09 | B | C |
| ATOM | 3467 | CG1 | ILE | B | 156 | 37.524 | 51.701 | 96.714 | 1.00 | 16.09 | B | C |
| ATOM | 3468 | CD1 | ILE | B | 156 | 37.046 | 50.725 | 97.764 | 1.00 | 16.09 | B | C |
| ATOM | 3469 | C | ILE | B | 156 | 36.420 | 50.555 | 94.142 | 1.00 | 27.92 | B | C |
| ATOM | 3470 | O | ILE | B | 156 | 35.290 | 50.386 | 94.588 | 1.00 | 27.92 | B | O |
| ATOM | 3471 | N | TYR | B | 157 | 36.659 | 51.193 | 93.002 | 1.00 | 22.61 | B | N |
| ATOM | 3472 | CA | TYR | B | 157 | 35.582 | 51.775 | 92.207 | 1.00 | 22.61 | B | C |
| ATOM | 3473 | CB | TYR | B | 157 | 36.153 | 52.656 | 91.086 | 1.00 | 38.18 | B | C |
| ATOM | 3474 | CG | TYR | B | 157 | 36.678 | 53.996 | 91.555 | 1.00 | 38.18 | B | C |
| ATOM | 3475 | CD1 | TYR | B | 157 | 35.815 | 54.950 | 92.094 | 1.00 | 38.18 | B | C |
| ATOM | 3476 | CE1 | TYR | B | 157 | 36.287 | 56.191 | 92.517 | 1.00 | 38.18 | B | C |
| ATOM | 3477 | CD2 | TYR | B | 157 | 38.038 | 54.314 | 91.449 | 1.00 | 38.18 | B | C |
| ATOM | 3478 | CE2 | TYR | B | 157 | 38.528 | 55.560 | 91.868 | 1.00 | 38.18 | B | C |
| ATOM | 3479 | CZ | TYR | B | 157 | 37.648 | 56.492 | 92.399 | 1.00 | 38.18 | B | C |
| ATOM | 3480 | OH | TYR | B | 157 | 38.120 | 57.723 | 92.798 | 1.00 | 38.18 | B | O |
| ATOM | 3481 | C | TYR | B | 157 | 34.681 | 50.699 | 91.608 | 1.00 | 22.61 | B | C |
| ATOM | 3482 | O | TYR | B | 157 | 33.480 | 50.885 | 91.516 | 1.00 | 22.61 | B | O |
| ATOM | 3483 | N | VAL | B | 158 | 35.270 | 49.579 | 91.197 | 1.00 | 27.37 | B | N |
| ATOM | 3484 | CA | VAL | B | 158 | 34.513 | 48.462 | 90.637 | 1.00 | 27.37 | B | C |
| ATOM | 3485 | CB | VAL | B | 158 | 35.450 | 47.319 | 90.172 | 1.00 | 24.60 | B | C |
| ATOM | 3486 | CG1 | VAL | B | 158 | 34.650 | 46.040 | 89.930 | 1.00 | 24.60 | B | C |
| ATOM | 3487 | CG2 | VAL | B | 158 | 36.164 | 47.737 | 88.896 | 1.00 | 24.60 | B | C |
| ATOM | 3488 | C | VAL | B | 158 | 33.560 | 47.930 | 91.706 | 1.00 | 27.37 | B | C |
| ATOM | 3489 | O | VAL | B | 158 | 32.432 | 47.562 | 91.414 | 1.00 | 27.37 | B | O |
| ATOM | 3490 | N | LYS | B | 159 | 34.028 | 47.896 | 92.944 | 1.00 | 12.85 | B | N |
| ATOM | 3491 | CA | LYS | B | 159 | 33.225 | 47.439 | 94.065 | 1.00 | 12.85 | B | C |
| ATOM | 3492 | CB | LYS | B | 159 | 34.086 | 47.331 | 95.340 | 1.00 | 23.85 | B | C |
| ATOM | 3493 | CG | LYS | B | 159 | 34.988 | 46.116 | 95.386 | 1.00 | 23.85 | B | C |
| ATOM | 3494 | CD | LYS | B | 159 | 35.804 | 46.037 | 96.660 | 1.00 | 23.85 | B | C |
| ATOM | 3495 | CE | LYS | B | 159 | 36.770 | 44.862 | 96.579 | 1.00 | 23.85 | B | C |
| ATOM | 3496 | NZ | LYS | B | 159 | 37.625 | 44.703 | 97.775 | 1.00 | 23.85 | B | N |
| ATOM | 3497 | C | LYS | B | 159 | 32.063 | 48.396 | 94.324 | 1.00 | 12.85 | B | C |
| ATOM | 3498 | O | LYS | B | 159 | 30.921 | 47.966 | 94.463 | 1.00 | 12.85 | B | O |
| ATOM | 3499 | N | LEU | B | 160 | 32.378 | 49.691 | 94.394 | 1.00 | 18.84 | B | N |
| ATOM | 3500 | CA | LEU | B | 160 | 31.398 | 50.745 | 94.636 | 1.00 | 18.84 | B | C |
| ATOM | 3501 | CB | LEU | B | 160 | 32.092 | 52.107 | 94.708 | 1.00 | 23.07 | B | C |
| ATOM | 3502 | CG | LEU | B | 160 | 32.874 | 52.445 | 95.970 | 1.00 | 23.07 | B | C |
| ATOM | 3503 | CD1 | LEU | B | 160 | 33.617 | 53.720 | 95.731 | 1.00 | 23.07 | B | C |
| ATOM | 3504 | CD2 | LEU | B | 160 | 31.945 | 52.576 | 97.172 | 1.00 | 23.07 | B | C |
| ATOM | 3505 | C | LEU | B | 160 | 30.306 | 50.830 | 93.587 | 1.00 | 18.84 | B | C |
| ATOM | 3506 | O | LEU | B | 160 | 29.124 | 50.945 | 93.921 | 1.00 | 18.84 | B | O |
| ATOM | 3507 | N | TYR | B | 161 | 30.705 | 50.798 | 92.318 | 1.00 | 12.79 | B | N |
| ATOM | 3508 | CA | TYR | B | 161 | 29.751 | 50.874 | 91.231 | 1.00 | 12.79 | B | C |
| ATOM | 3509 | CB | TYR | B | 161 | 30.469 | 51.097 | 89.904 | 1.00 | 29.69 | B | C |
| ATOM | 3510 | CG | TYR | B | 161 | 31.226 | 52.404 | 89.831 | 1.00 | 29.69 | B | C |
| ATOM | 3511 | CD1 | TYR | B | 161 | 30.986 | 53.421 | 90.757 | 1.00 | 29.69 | B | C |
| ATOM | 3512 | CE1 | TYR | B | 161 | 31.717 | 54.602 | 90.733 | 1.00 | 29.69 | B | C |
| ATOM | 3513 | CD2 | TYR | B | 161 | 32.217 | 52.611 | 88.859 | 1.00 | 29.69 | B | C |
| ATOM | 3514 | CE2 | TYR | B | 161 | 32.955 | 53.796 | 88.823 | 1.00 | 29.69 | B | C |
| ATOM | 3515 | CZ | TYR | B | 161 | 32.702 | 54.786 | 89.767 | 1.00 | 29.69 | B | C |
| ATOM | 3516 | OH | TYR | B | 161 | 33.440 | 55.952 | 89.762 | 1.00 | 29.69 | B | O |
| ATOM | 3517 | C | TYR | B | 161 | 28.918 | 49.608 | 91.155 | 1.00 | 12.79 | B | C |
| ATOM | 3518 | O | TYR | B | 161 | 27.699 | 49.676 | 91.114 | 1.00 | 12.79 | B | O |
| ATOM | 3519 | N | MET | B | 162 | 29.571 | 48.454 | 91.142 | 1.00 | 15.76 | B | N |
| ATOM | 3520 | CA | MET | B | 162 | 28.856 | 47.183 | 91.064 | 1.00 | 15.76 | B | C |
| ATOM | 3521 | CB | MET | B | 162 | 29.836 | 46.014 | 91.086 | 1.00 | 27.25 | B | C |
| ATOM | 3522 | CG | MET | B | 162 | 30.608 | 45.840 | 89.808 | 1.00 | 27.25 | B | C |

| | | | | | | | | | | | | |
|---|---|---|---|---|---|---|---|---|---|---|---|---|
| ATOM | 3523 | SD | MET | B | 162 | 29.544 | 45.941 | 88.342 | 1.00 | 27.25 | B | S |
| ATOM | 3524 | CE | MET | B | 162 | 28.555 | 44.453 | 88.512 | 1.00 | 27.25 | B | C |
| ATOM | 3525 | C | MET | B | 162 | 27.839 | 46.981 | 92.186 | 1.00 | 15.76 | B | C |
| ATOM | 3526 | O | MET | B | 162 | 26.740 | 46.479 | 91.965 | 1.00 | 15.76 | B | O |
| ATOM | 3527 | N | TYR | B | 163 | 28.223 | 47.357 | 93.399 | 1.00 | 30.15 | B | N |
| ATOM | 3528 | CA | TYR | B | 163 | 27.347 | 47.231 | 94.556 | 1.00 | 30.15 | B | C |
| ATOM | 3529 | CB | TYR | B | 163 | 28.100 | 47.621 | 95.833 | 1.00 | 21.46 | B | C |
| ATOM | 3530 | CG | TYR | B | 163 | 27.283 | 47.587 | 97.110 | 1.00 | 21.46 | B | C |
| ATOM | 3531 | CD1 | TYR | B | 163 | 27.000 | 46.387 | 97.744 | 1.00 | 21.46 | B | C |
| ATOM | 3532 | CE1 | TYR | B | 163 | 26.263 | 46.358 | 98.917 | 1.00 | 21.46 | B | C |
| ATOM | 3533 | CD2 | TYR | B | 163 | 26.801 | 48.768 | 97.686 | 1.00 | 21.46 | B | C |
| ATOM | 3534 | CE2 | TYR | B | 163 | 26.061 | 48.750 | 98.854 | 1.00 | 21.46 | B | C |
| ATOM | 3535 | CZ | TYR | B | 163 | 25.794 | 47.542 | 99.468 | 1.00 | 21.46 | B | C |
| ATOM | 3536 | OH | TYR | B | 163 | 25.066 | 47.517 | 100.638 | 1.00 | 21.46 | B | O |
| ATOM | 3537 | C | TYR | B | 163 | 26.103 | 48.110 | 94.411 | 1.00 | 30.15 | B | C |
| ATOM | 3538 | O | TYR | B | 163 | 24.987 | 47.666 | 94.655 | 1.00 | 30.15 | B | O |
| ATOM | 3539 | N | GLN | B | 164 | 26.302 | 49.363 | 94.021 | 1.00 | 18.17 | B | N |
| ATOM | 3540 | CA | GLN | B | 164 | 25.192 | 50.277 | 93.848 | 1.00 | 18.17 | B | C |
| ATOM | 3541 | CB | GLN | B | 164 | 25.713 | 51.684 | 93.577 | 1.00 | 15.62 | B | C |
| ATOM | 3542 | CG | GLN | B | 164 | 26.524 | 52.219 | 94.722 | 1.00 | 15.62 | B | C |
| ATOM | 3543 | CD | GLN | B | 164 | 27.064 | 53.603 | 94.471 | 1.00 | 15.62 | B | C |
| ATOM | 3544 | OE1 | GLN | B | 164 | 26.311 | 54.568 | 94.354 | 1.00 | 15.62 | B | O |
| ATOM | 3545 | NE2 | GLN | B | 164 | 28.387 | 53.710 | 94.381 | 1.00 | 15.62 | B | N |
| ATOM | 3546 | C | GLN | B | 164 | 24.263 | 49.815 | 92.723 | 1.00 | 18.17 | B | C |
| ATOM | 3547 | O | GLN | B | 164 | 23.068 | 50.105 | 92.732 | 1.00 | 18.17 | B | O |
| ATOM | 3548 | N | LEU | B | 165 | 24.803 | 49.085 | 91.759 | 1.00 | 17.96 | B | N |
| ATOM | 3549 | CA | LEU | B | 165 | 23.982 | 48.604 | 90.663 | 1.00 | 17.96 | B | C |
| ATOM | 3550 | CB | LEU | B | 165 | 24.844 | 47.991 | 89.560 | 1.00 | 12.94 | B | C |
| ATOM | 3551 | CG | LEU | B | 165 | 24.338 | 47.962 | 88.114 | 1.00 | 12.94 | B | C |
| ATOM | 3552 | CD1 | LEU | B | 165 | 25.057 | 46.843 | 87.413 | 1.00 | 12.94 | B | C |
| ATOM | 3553 | CD2 | LEU | B | 165 | 22.837 | 47.763 | 88.025 | 1.00 | 12.94 | B | C |
| ATOM | 3554 | C | LEU | B | 165 | 23.057 | 47.521 | 91.198 | 1.00 | 17.96 | B | C |
| ATOM | 3555 | O | LEU | B | 165 | 21.877 | 47.455 | 90.856 | 1.00 | 17.96 | B | O |
| ATOM | 3556 | N | PHE | B | 166 | 23.608 | 46.653 | 92.035 | 1.00 | 28.01 | B | N |
| ATOM | 3557 | CA | PHE | B | 166 | 22.823 | 45.572 | 92.574 | 1.00 | 28.01 | B | C |
| ATOM | 3558 | CB | PHE | B | 166 | 23.719 | 44.580 | 93.316 | 1.00 | 16.06 | B | C |
| ATOM | 3559 | CG | PHE | B | 166 | 24.535 | 43.705 | 92.404 | 1.00 | 16.06 | B | C |
| ATOM | 3560 | CD1 | PHE | B | 166 | 23.912 | 42.885 | 91.463 | 1.00 | 16.06 | B | C |
| ATOM | 3561 | CD2 | PHE | B | 166 | 25.925 | 43.715 | 92.462 | 1.00 | 16.06 | B | C |
| ATOM | 3562 | CE1 | PHE | B | 166 | 24.666 | 42.086 | 90.593 | 1.00 | 16.06 | B | C |
| ATOM | 3563 | CE2 | PHE | B | 166 | 26.683 | 42.921 | 91.599 | 1.00 | 16.06 | B | C |
| ATOM | 3564 | CZ | PHE | B | 166 | 26.049 | 42.110 | 90.662 | 1.00 | 16.06 | B | C |
| ATOM | 3565 | C | PHE | B | 166 | 21.722 | 46.098 | 93.466 | 1.00 | 28.01 | B | C |
| ATOM | 3566 | O | PHE | B | 166 | 20.619 | 45.569 | 93.457 | 1.00 | 28.01 | B | O |
| ATOM | 3567 | N | ARG | B | 167 | 21.997 | 47.147 | 94.226 | 1.00 | 31.87 | B | N |
| ATOM | 3568 | CA | ARG | B | 167 | 20.964 | 47.689 | 95.079 | 1.00 | 31.87 | B | C |
| ATOM | 3569 | CB | ARG | B | 167 | 21.485 | 48.848 | 95.921 | 1.00 | 16.29 | B | C |
| ATOM | 3570 | CG | ARG | B | 167 | 22.302 | 48.435 | 97.121 | 1.00 | 16.29 | B | C |
| ATOM | 3571 | CD | ARG | B | 167 | 22.464 | 49.601 | 98.055 | 1.00 | 16.29 | B | C |
| ATOM | 3572 | NE | ARG | B | 167 | 21.210 | 49.956 | 98.706 | 1.00 | 16.29 | B | N |
| ATOM | 3573 | CZ | ARG | B | 167 | 21.011 | 51.085 | 99.375 | 1.00 | 16.29 | B | C |
| ATOM | 3574 | NH1 | ARG | B | 167 | 21.982 | 51.983 | 99.478 | 1.00 | 16.29 | B | N |
| ATOM | 3575 | NH2 | ARG | B | 167 | 19.847 | 51.307 | 99.958 | 1.00 | 16.29 | B | N |
| ATOM | 3576 | C | ARG | B | 167 | 19.801 | 48.165 | 94.229 | 1.00 | 31.87 | B | C |
| ATOM | 3577 | O | ARG | B | 167 | 18.645 | 47.934 | 94.568 | 1.00 | 31.87 | B | O |
| ATOM | 3578 | N | SER | B | 168 | 20.098 | 48.816 | 93.114 | 1.00 | 18.22 | B | N |
| ATOM | 3579 | CA | SER | B | 168 | 19.030 | 49.329 | 92.265 | 1.00 | 18.22 | B | C |
| ATOM | 3580 | CB | SER | B | 168 | 19.589 | 50.285 | 91.200 | 1.00 | 12.02 | B | C |
| ATOM | 3581 | OG | SER | B | 168 | 20.308 | 49.601 | 90.201 | 1.00 | 12.02 | B | O |
| ATOM | 3582 | C | SER | B | 168 | 18.258 | 48.190 | 91.616 | 1.00 | 18.22 | B | C |
| ATOM | 3583 | O | SER | B | 168 | 17.059 | 48.301 | 91.352 | 1.00 | 18.22 | B | O |
| ATOM | 3584 | N | LEU | B | 169 | 18.954 | 47.087 | 91.373 | 1.00 | 18.80 | B | N |
| ATOM | 3585 | CA | LEU | B | 169 | 18.338 | 45.916 | 90.770 | 1.00 | 18.80 | B | C |

| | | | | | | | | | | | | |
|---|---|---|---|---|---|---|---|---|---|---|---|---|
| ATOM | 3586 | CB | LEU | B | 169 | 19.427 | 44.982 | 90.234 | 1.00 | 10.10 | B | C |
| ATOM | 3587 | CG | LEU | B | 169 | 19.668 | 44.920 | 88.720 | 1.00 | 10.10 | B | C |
| ATOM | 3588 | CD1 | LEU | B | 169 | 19.329 | 46.222 | 88.037 | 1.00 | 10.10 | B | C |
| ATOM | 3589 | CD2 | LEU | B | 169 | 21.108 | 44.541 | 88.479 | 1.00 | 10.10 | B | C |
| ATOM | 3590 | C | LEU | B | 169 | 17.470 | 45.215 | 91.819 | 1.00 | 18.80 | B | C |
| ATOM | 3591 | O | LEU | B | 169 | 16.352 | 44.792 | 91.535 | 1.00 | 18.80 | B | O |
| ATOM | 3592 | N | ALA | B | 170 | 17.994 | 45.114 | 93.039 | 1.00 | 25.18 | B | N |
| ATOM | 3593 | CA | ALA | B | 170 | 17.272 | 44.504 | 94.155 | 1.00 | 25.18 | B | C |
| ATOM | 3594 | CB | ALA | B | 170 | 18.128 | 44.531 | 95.414 | 1.00 | 6.97 | B | C |
| ATOM | 3595 | C | ALA | B | 170 | 15.991 | 45.294 | 94.391 | 1.00 | 25.18 | B | C |
| ATOM | 3596 | O | ALA | B | 170 | 14.939 | 44.727 | 94.682 | 1.00 | 25.18 | B | O |
| ATOM | 3597 | N | TYR | B | 171 | 16.096 | 46.611 | 94.263 | 1.00 | 23.91 | B | N |
| ATOM | 3598 | CA | TYR | B | 171 | 14.955 | 47.482 | 94.442 | 1.00 | 23.91 | B | C |
| ATOM | 3599 | CB | TYR | B | 171 | 15.372 | 48.945 | 94.328 | 1.00 | 18.06 | B | C |
| ATOM | 3600 | CG | TYR | B | 171 | 14.227 | 49.930 | 94.506 | 1.00 | 18.06 | B | C |
| ATOM | 3601 | CD1 | TYR | B | 171 | 13.676 | 50.180 | 95.763 | 1.00 | 18.06 | B | C |
| ATOM | 3602 | CE1 | TYR | B | 171 | 12.644 | 51.112 | 95.932 | 1.00 | 18.06 | B | C |
| ATOM | 3603 | CD2 | TYR | B | 171 | 13.714 | 50.632 | 93.420 | 1.00 | 18.06 | B | C |
| ATOM | 3604 | CE2 | TYR | B | 171 | 12.689 | 51.561 | 93.576 | 1.00 | 18.06 | B | C |
| ATOM | 3605 | CZ | TYR | B | 171 | 12.157 | 51.798 | 94.833 | 1.00 | 18.06 | B | C |
| ATOM | 3606 | OH | TYR | B | 171 | 11.136 | 52.711 | 94.989 | 1.00 | 18.06 | B | O |
| ATOM | 3607 | C | TYR | B | 171 | 13.892 | 47.173 | 93.399 | 1.00 | 23.91 | B | C |
| ATOM | 3608 | O | TYR | B | 171 | 12.902 | 46.513 | 93.693 | 1.00 | 23.91 | B | O |
| ATOM | 3609 | N | ILE | B | 172 | 14.105 | 47.637 | 92.173 | 1.00 | 17.03 | B | N |
| ATOM | 3610 | CA | ILE | B | 172 | 13.133 | 47.425 | 91.109 | 1.00 | 17.03 | B | C |
| ATOM | 3611 | CB | ILE | B | 172 | 13.704 | 47.823 | 89.731 | 1.00 | 21.31 | B | C |
| ATOM | 3612 | CG2 | ILE | B | 172 | 14.249 | 49.247 | 89.795 | 1.00 | 21.31 | B | C |
| ATOM | 3613 | CG1 | ILE | B | 172 | 14.773 | 46.819 | 89.295 | 1.00 | 21.31 | B | C |
| ATOM | 3614 | CD1 | ILE | B | 172 | 15.140 | 46.906 | 87.830 | 1.00 | 21.31 | B | C |
| ATOM | 3615 | C | ILE | B | 172 | 12.606 | 45.992 | 91.024 | 1.00 | 17.03 | B | C |
| ATOM | 3616 | O | ILE | B | 172 | 11.427 | 45.770 | 90.770 | 1.00 | 17.03 | B | O |
| ATOM | 3617 | N | HIS | B | 173 | 13.464 | 45.009 | 91.240 | 1.00 | 24.56 | B | N |
| ATOM | 3618 | CA | HIS | B | 173 | 12.996 | 43.637 | 91.155 | 1.00 | 24.56 | B | C |
| ATOM | 3619 | CB | HIS | B | 173 | 14.174 | 42.662 | 91.221 | 1.00 | 17.06 | B | C |
| ATOM | 3620 | CG | HIS | B | 173 | 15.029 | 42.674 | 89.989 | 1.00 | 17.06 | B | C |
| ATOM | 3621 | CD2 | HIS | B | 173 | 14.911 | 43.364 | 88.829 | 1.00 | 17.06 | B | C |
| ATOM | 3622 | ND1 | HIS | B | 173 | 16.177 | 41.919 | 89.864 | 1.00 | 17.06 | B | N |
| ATOM | 3623 | CE1 | HIS | B | 173 | 16.728 | 42.146 | 88.684 | 1.00 | 17.06 | B | C |
| ATOM | 3624 | NE2 | HIS | B | 173 | 15.979 | 43.019 | 88.038 | 1.00 | 17.06 | B | N |
| ATOM | 3625 | C | HIS | B | 173 | 11.958 | 43.323 | 92.225 | 1.00 | 24.56 | B | C |
| ATOM | 3626 | O | HIS | B | 173 | 11.023 | 42.560 | 91.978 | 1.00 | 24.56 | B | O |
| ATOM | 3627 | N | SER | B | 174 | 12.101 | 43.925 | 93.402 | 1.00 | 27.79 | B | N |
| ATOM | 3628 | CA | SER | B | 174 | 11.146 | 43.691 | 94.478 | 1.00 | 27.79 | B | C |
| ATOM | 3629 | CB | SER | B | 174 | 11.487 | 44.525 | 95.713 | 1.00 | 25.93 | B | C |
| ATOM | 3630 | OG | SER | B | 174 | 11.286 | 45.904 | 95.474 | 1.00 | 25.93 | B | O |
| ATOM | 3631 | C | SER | B | 174 | 9.738 | 44.029 | 94.009 | 1.00 | 27.79 | B | C |
| ATOM | 3632 | O | SER | B | 174 | 8.769 | 43.462 | 94.512 | 1.00 | 27.79 | B | O |
| ATOM | 3633 | N | PHE | B | 175 | 9.634 | 44.953 | 93.050 | 1.00 | 17.77 | B | N |
| ATOM | 3634 | CA | PHE | B | 175 | 8.348 | 45.365 | 92.501 | 1.00 | 17.77 | B | C |
| ATOM | 3635 | CB | PHE | B | 175 | 8.355 | 46.826 | 92.065 | 1.00 | 36.06 | B | C |
| ATOM | 3636 | CG | PHE | B | 175 | 8.514 | 47.792 | 93.185 | 1.00 | 36.06 | B | C |
| ATOM | 3637 | CD1 | PHE | B | 175 | 9.778 | 48.215 | 93.579 | 1.00 | 36.06 | B | C |
| ATOM | 3638 | CD2 | PHE | B | 175 | 7.395 | 48.285 | 93.855 | 1.00 | 36.06 | B | C |
| ATOM | 3639 | CE1 | PHE | B | 175 | 9.929 | 49.115 | 94.624 | 1.00 | 36.06 | B | C |
| ATOM | 3640 | CE2 | PHE | B | 175 | 7.537 | 49.190 | 94.911 | 1.00 | 36.06 | B | C |
| ATOM | 3641 | CZ | PHE | B | 175 | 8.809 | 49.606 | 95.295 | 1.00 | 36.06 | B | C |
| ATOM | 3642 | C | PHE | B | 175 | 7.987 | 44.532 | 91.286 | 1.00 | 17.77 | B | C |
| ATOM | 3643 | O | PHE | B | 175 | 6.938 | 44.747 | 90.677 | 1.00 | 17.77 | B | O |
| ATOM | 3644 | N | GLY | B | 176 | 8.861 | 43.595 | 90.924 | 1.00 | 26.80 | B | N |
| ATOM | 3645 | CA | GLY | B | 176 | 8.611 | 42.757 | 89.762 | 1.00 | 26.80 | B | C |
| ATOM | 3646 | C | GLY | B | 176 | 8.851 | 43.476 | 88.442 | 1.00 | 26.80 | B | C |
| ATOM | 3647 | O | GLY | B | 176 | 8.218 | 43.191 | 87.428 | 1.00 | 26.80 | B | O |
| ATOM | 3648 | N | ILE | B | 177 | 9.774 | 44.425 | 88.464 | 1.00 | 23.69 | B | N |

| | | | | | | | | | | | | |
|---|---|---|---|---|---|---|---|---|---|---|---|---|
| ATOM | 3649 | CA | ILE | B | 177 | 10.113 | 45.199 | 87.283 | 1.00 | 23.69 | B | C |
| ATOM | 3650 | CB | ILE | B | 177 | 10.126 | 46.716 | 87.602 | 1.00 | 18.65 | B | C |
| ATOM | 3651 | CG2 | ILE | B | 177 | 10.671 | 47.508 | 86.426 | 1.00 | 18.65 | B | C |
| ATOM | 3652 | CG1 | ILE | B | 177 | 8.710 | 47.167 | 87.962 | 1.00 | 18.65 | B | C |
| ATOM | 3653 | CD1 | ILE | B | 177 | 8.597 | 48.630 | 88.309 | 1.00 | 18.65 | B | C |
| ATOM | 3654 | C | ILE | B | 177 | 11.488 | 44.768 | 86.799 | 1.00 | 23.69 | B | C |
| ATOM | 3655 | O | ILE | B | 177 | 12.412 | 44.629 | 87.592 | 1.00 | 23.69 | B | O |
| ATOM | 3656 | N | CYS | B | 178 | 11.613 | 44.533 | 85.498 | 1.00 | 20.94 | B | N |
| ATOM | 3657 | CA | CYS | B | 178 | 12.889 | 44.138 | 84.925 | 1.00 | 20.94 | B | C |
| ATOM | 3658 | CB | CYS | B | 178 | 12.740 | 42.870 | 84.088 | 1.00 | 36.73 | B | C |
| ATOM | 3659 | SG | CYS | B | 178 | 14.291 | 42.010 | 83.753 | 1.00 | 36.73 | B | S |
| ATOM | 3660 | C | CYS | B | 178 | 13.350 | 45.274 | 84.039 | 1.00 | 20.94 | B | C |
| ATOM | 3661 | O | CYS | B | 178 | 12.545 | 45.875 | 83.325 | 1.00 | 20.94 | B | O |
| ATOM | 3662 | N | HIS | B | 179 | 14.643 | 45.571 | 84.098 | 1.00 | 12.33 | B | N |
| ATOM | 3663 | CA | HIS | B | 179 | 15.214 | 46.640 | 83.301 | 1.00 | 12.33 | B | C |
| ATOM | 3664 | CB | HIS | B | 179 | 16.579 | 47.031 | 83.865 | 1.00 | 14.66 | B | C |
| ATOM | 3665 | CG | HIS | B | 179 | 17.133 | 48.282 | 83.273 | 1.00 | 14.66 | B | C |
| ATOM | 3666 | CD2 | HIS | B | 179 | 17.130 | 49.556 | 83.727 | 1.00 | 14.66 | B | C |
| ATOM | 3667 | ND1 | HIS | B | 179 | 17.720 | 48.320 | 82.025 | 1.00 | 14.66 | B | N |
| ATOM | 3668 | CE1 | HIS | B | 179 | 18.050 | 49.564 | 81.738 | 1.00 | 14.66 | B | C |
| ATOM | 3669 | NE2 | HIS | B | 179 | 17.702 | 50.335 | 82.753 | 1.00 | 14.66 | B | N |
| ATOM | 3670 | C | HIS | B | 179 | 15.338 | 46.193 | 81.851 | 1.00 | 12.33 | B | C |
| ATOM | 3671 | O | HIS | B | 179 | 15.035 | 46.954 | 80.935 | 1.00 | 12.33 | B | O |
| ATOM | 3672 | N | ARG | B | 180 | 15.774 | 44.949 | 81.663 | 1.00 | 24.62 | B | N |
| ATOM | 3673 | CA | ARG | B | 180 | 15.951 | 44.342 | 80.343 | 1.00 | 24.62 | B | C |
| ATOM | 3674 | CB | ARG | B | 180 | 14.602 | 44.280 | 79.623 | 1.00 | 20.65 | B | C |
| ATOM | 3675 | CG | ARG | B | 180 | 13.566 | 43.407 | 80.299 | 1.00 | 20.65 | B | C |
| ATOM | 3676 | CD | ARG | B | 180 | 12.200 | 43.989 | 80.011 | 1.00 | 20.65 | B | C |
| ATOM | 3677 | NE | ARG | B | 180 | 11.507 | 43.338 | 78.909 | 1.00 | 20.65 | B | N |
| ATOM | 3678 | CZ | ARG | B | 180 | 10.694 | 43.964 | 78.066 | 1.00 | 20.65 | B | C |
| ATOM | 3679 | NH1 | ARG | B | 180 | 10.471 | 45.269 | 78.172 | 1.00 | 20.65 | B | N |
| ATOM | 3680 | NH2 | ARG | B | 180 | 10.071 | 43.268 | 77.133 | 1.00 | 20.65 | B | N |
| ATOM | 3681 | C | ARG | B | 180 | 17.002 | 45.001 | 79.430 | 1.00 | 24.62 | B | C |
| ATOM | 3682 | O | ARG | B | 180 | 17.163 | 44.602 | 78.272 | 1.00 | 24.62 | B | O |
| ATOM | 3683 | N | ASP | B | 181 | 17.715 | 46.003 | 79.944 | 1.00 | 28.53 | B | N |
| ATOM | 3684 | CA | ASP | B | 181 | 18.746 | 46.667 | 79.154 | 1.00 | 28.53 | B | C |
| ATOM | 3685 | CB | ASP | B | 181 | 18.140 | 47.782 | 78.308 | 1.00 | 34.26 | B | C |
| ATOM | 3686 | CG | ASP | B | 181 | 19.115 | 48.308 | 77.272 | 1.00 | 34.26 | B | C |
| ATOM | 3687 | OD1 | ASP | B | 181 | 19.750 | 47.465 | 76.600 | 1.00 | 34.26 | B | O |
| ATOM | 3688 | OD2 | ASP | B | 181 | 19.240 | 49.550 | 77.127 | 1.00 | 34.26 | B | O |
| ATOM | 3689 | C | ASP | B | 181 | 19.915 | 47.234 | 79.965 | 1.00 | 28.53 | B | C |
| ATOM | 3690 | O | ASP | B | 181 | 20.376 | 48.355 | 79.720 | 1.00 | 28.53 | B | O |
| ATOM | 3691 | N | ILE | B | 182 | 20.402 | 46.449 | 80.919 | 1.00 | 18.84 | B | N |
| ATOM | 3692 | CA | ILE | B | 182 | 21.524 | 46.877 | 81.728 | 1.00 | 18.84 | B | C |
| ATOM | 3693 | CB | ILE | B | 182 | 21.731 | 45.974 | 82.962 | 1.00 | 17.27 | B | C |
| ATOM | 3694 | CG2 | ILE | B | 182 | 22.978 | 46.403 | 83.723 | 1.00 | 17.27 | B | C |
| ATOM | 3695 | CG1 | ILE | B | 182 | 20.517 | 46.048 | 83.881 | 1.00 | 17.27 | B | C |
| ATOM | 3696 | CD1 | ILE | B | 182 | 20.401 | 47.326 | 84.611 | 1.00 | 17.27 | B | C |
| ATOM | 3697 | C | ILE | B | 182 | 22.771 | 46.794 | 80.877 | 1.00 | 18.84 | B | C |
| ATOM | 3698 | O | ILE | B | 182 | 23.123 | 45.726 | 80.380 | 1.00 | 18.84 | B | O |
| ATOM | 3699 | N | LYS | B | 183 | 23.415 | 47.940 | 80.694 | 1.00 | 16.86 | B | N |
| ATOM | 3700 | CA | LYS | B | 183 | 24.654 | 48.041 | 79.944 | 1.00 | 16.86 | B | C |
| ATOM | 3701 | CB | LYS | B | 183 | 24.379 | 48.179 | 78.450 | 1.00 | 19.22 | B | C |
| ATOM | 3702 | CG | LYS | B | 183 | 23.410 | 49.283 | 78.039 | 1.00 | 19.22 | B | C |
| ATOM | 3703 | CD | LYS | B | 183 | 23.061 | 49.138 | 76.568 | 1.00 | 19.22 | B | C |
| ATOM | 3704 | CE | LYS | B | 183 | 22.250 | 50.302 | 76.058 | 1.00 | 19.22 | B | C |
| ATOM | 3705 | NZ | LYS | B | 183 | 21.948 | 50.146 | 74.616 | 1.00 | 19.22 | B | N |
| ATOM | 3706 | C | LYS | B | 183 | 25.375 | 49.266 | 80.488 | 1.00 | 16.86 | B | C |
| ATOM | 3707 | O | LYS | B | 183 | 24.740 | 50.187 | 81.004 | 1.00 | 16.86 | B | O |
| ATOM | 3708 | N | PRO | B | 184 | 26.714 | 49.288 | 80.387 | 1.00 | 19.99 | B | N |
| ATOM | 3709 | CD | PRO | B | 184 | 27.543 | 48.288 | 79.692 | 1.00 | 9.83 | B | C |
| ATOM | 3710 | CA | PRO | B | 184 | 27.548 | 50.387 | 80.870 | 1.00 | 19.99 | B | C |
| ATOM | 3711 | CB | PRO | B | 184 | 28.911 | 50.068 | 80.265 | 1.00 | 9.83 | B | C |

| | | | | | | | | | | | | |
|---|---|---|---|---|---|---|---|---|---|---|---|---|
| ATOM | 3712 | CG | PRO | B | 184 | 28.913 | 48.595 | 80.221 | 1.00 | 9.83 | B | C |
| ATOM | 3713 | C | PRO | B | 184 | 27.071 | 51.784 | 80.505 | 1.00 | 19.99 | B | C |
| ATOM | 3714 | O | PRO | B | 184 | 27.314 | 52.726 | 81.252 | 1.00 | 19.99 | B | O |
| ATOM | 3715 | N | GLN | B | 185 | 26.406 | 51.920 | 79.363 | 1.00 | 17.94 | B | N |
| ATOM | 3716 | CA | GLN | B | 185 | 25.922 | 53.225 | 78.930 | 1.00 | 17.94 | B | C |
| ATOM | 3717 | CB | GLN | B | 185 | 25.508 | 53.204 | 77.460 | 1.00 | 42.77 | B | C |
| ATOM | 3718 | CG | GLN | B | 185 | 25.088 | 54.590 | 76.993 | 1.00 | 42.77 | B | C |
| ATOM | 3719 | CD | GLN | B | 185 | 24.522 | 54.647 | 75.580 | 1.00 | 42.77 | B | C |
| ATOM | 3720 | OE1 | GLN | B | 185 | 24.092 | 55.713 | 75.132 | 1.00 | 42.77 | B | O |
| ATOM | 3721 | NE2 | GLN | B | 185 | 24.518 | 53.514 | 74.874 | 1.00 | 42.77 | B | N |
| ATOM | 3722 | C | GLN | B | 185 | 24.749 | 53.726 | 79.766 | 1.00 | 17.94 | B | C |
| ATOM | 3723 | O | GLN | B | 185 | 24.543 | 54.935 | 79.897 | 1.00 | 17.94 | B | O |
| ATOM | 3724 | N | ASN | B | 186 | 23.994 | 52.778 | 80.324 | 1.00 | 23.18 | B | N |
| ATOM | 3725 | CA | ASN | B | 186 | 22.827 | 53.068 | 81.151 | 1.00 | 23.18 | B | C |
| ATOM | 3726 | CB | ASN | B | 186 | 21.759 | 51.993 | 80.988 | 1.00 | 29.63 | B | C |
| ATOM | 3727 | CG | ASN | B | 186 | 20.994 | 52.136 | 79.703 | 1.00 | 29.63 | B | C |
| ATOM | 3728 | OD1 | ASN | B | 186 | 20.861 | 53.242 | 79.170 | 1.00 | 29.63 | B | O |
| ATOM | 3729 | ND2 | ASN | B | 186 | 20.466 | 51.022 | 79.198 | 1.00 | 29.63 | B | N |
| ATOM | 3730 | C | ASN | B | 186 | 23.173 | 53.197 | 82.618 | 1.00 | 23.18 | B | C |
| ATOM | 3731 | O | ASN | B | 186 | 22.295 | 53.300 | 83.461 | 1.00 | 23.18 | B | O |
| ATOM | 3732 | N | LEU | B | 187 | 24.461 | 53.178 | 82.920 | 1.00 | 21.52 | B | N |
| ATOM | 3733 | CA | LEU | B | 187 | 24.930 | 53.320 | 84.289 | 1.00 | 21.52 | B | C |
| ATOM | 3734 | CB | LEU | B | 187 | 25.945 | 52.227 | 84.610 | 1.00 | 19.24 | B | C |
| ATOM | 3735 | CG | LEU | B | 187 | 25.484 | 50.897 | 85.200 | 1.00 | 19.24 | B | C |
| ATOM | 3736 | CD1 | LEU | B | 187 | 24.157 | 50.468 | 84.645 | 1.00 | 19.24 | B | C |
| ATOM | 3737 | CD2 | LEU | B | 187 | 26.554 | 49.862 | 84.921 | 1.00 | 19.24 | B | C |
| ATOM | 3738 | C | LEU | B | 187 | 25.574 | 54.687 | 84.483 | 1.00 | 21.52 | B | C |
| ATOM | 3739 | O | LEU | B | 187 | 26.745 | 54.876 | 84.170 | 1.00 | 21.52 | B | O |
| ATOM | 3740 | N | LEU | B | 188 | 24.804 | 55.640 | 84.991 | 1.00 | 21.48 | B | N |
| ATOM | 3741 | CA | LEU | B | 188 | 25.309 | 56.993 | 85.225 | 1.00 | 21.48 | B | C |
| ATOM | 3742 | CB | LEU | B | 188 | 24.140 | 57.980 | 85.330 | 1.00 | 20.19 | B | C |
| ATOM | 3743 | CG | LEU | B | 188 | 23.072 | 57.975 | 84.233 | 1.00 | 20.19 | B | C |
| ATOM | 3744 | CD1 | LEU | B | 188 | 21.926 | 58.840 | 84.683 | 1.00 | 20.19 | B | C |
| ATOM | 3745 | CD2 | LEU | B | 188 | 23.639 | 58.478 | 82.916 | 1.00 | 20.19 | B | C |
| ATOM | 3746 | C | LEU | B | 188 | 26.101 | 57.033 | 86.533 | 1.00 | 21.48 | B | C |
| ATOM | 3747 | O | LEU | B | 188 | 25.846 | 56.254 | 87.440 | 1.00 | 21.48 | B | O |
| ATOM | 3748 | N | LEU | B | 189 | 27.070 | 57.932 | 86.633 | 1.00 | 27.21 | B | N |
| ATOM | 3749 | CA | LEU | B | 189 | 27.833 | 58.045 | 87.871 | 1.00 | 27.21 | B | C |
| ATOM | 3750 | CB | LEU | B | 189 | 28.882 | 56.936 | 87.954 | 1.00 | 36.98 | B | C |
| ATOM | 3751 | CG | LEU | B | 189 | 29.939 | 56.891 | 86.854 | 1.00 | 36.98 | B | C |
| ATOM | 3752 | CD1 | LEU | B | 189 | 30.944 | 58.018 | 87.056 | 1.00 | 36.98 | B | C |
| ATOM | 3753 | CD2 | LEU | B | 189 | 30.641 | 55.546 | 86.893 | 1.00 | 36.98 | B | C |
| ATOM | 3754 | C | LEU | B | 189 | 28.503 | 59.399 | 88.051 | 1.00 | 27.21 | B | C |
| ATOM | 3755 | O | LEU | B | 189 | 28.881 | 60.053 | 87.082 | 1.00 | 27.21 | B | O |
| ATOM | 3756 | N | ASP | B | 190 | 28.633 | 59.809 | 89.308 | 1.00 | 29.66 | B | N |
| ATOM | 3757 | CA | ASP | B | 190 | 29.272 | 61.071 | 89.651 | 1.00 | 29.66 | B | C |
| ATOM | 3758 | CB | ASP | B | 190 | 28.535 | 61.736 | 90.810 | 1.00 | 32.26 | B | C |
| ATOM | 3759 | CG | ASP | B | 190 | 28.901 | 63.197 | 90.972 | 1.00 | 32.26 | B | C |
| ATOM | 3760 | OD1 | ASP | B | 190 | 30.045 | 63.492 | 91.381 | 1.00 | 32.26 | B | O |
| ATOM | 3761 | OD2 | ASP | B | 190 | 28.046 | 64.056 | 90.683 | 1.00 | 32.26 | B | O |
| ATOM | 3762 | C | ASP | B | 190 | 30.699 | 60.731 | 90.062 | 1.00 | 29.66 | B | C |
| ATOM | 3763 | O | ASP | B | 190 | 30.917 | 60.042 | 91.051 | 1.00 | 29.66 | B | O |
| ATOM | 3764 | N | PRO | B | 191 | 31.690 | 61.214 | 89.307 | 1.00 | 44.08 | B | N |
| ATOM | 3765 | CD | PRO | B | 191 | 31.572 | 62.198 | 88.218 | 1.00 | 90.75 | B | C |
| ATOM | 3766 | CA | PRO | B | 191 | 33.098 | 60.935 | 89.607 | 1.00 | 44.08 | B | C |
| ATOM | 3767 | CB | PRO | B | 191 | 33.853 | 61.774 | 88.572 | 1.00 | 90.75 | B | C |
| ATOM | 3768 | CG | PRO | B | 191 | 32.854 | 61.970 | 87.459 | 1.00 | 90.75 | B | C |
| ATOM | 3769 | C | PRO | B | 191 | 33.505 | 61.295 | 91.031 | 1.00 | 44.08 | B | C |
| ATOM | 3770 | O | PRO | B | 191 | 34.142 | 60.505 | 91.739 | 1.00 | 44.08 | B | O |
| ATOM | 3771 | N | ASP | B | 192 | 33.116 | 62.487 | 91.455 | 1.00 | 33.29 | B | N |
| ATOM | 3772 | CA | ASP | B | 192 | 33.483 | 62.948 | 92.777 | 1.00 | 33.29 | B | C |
| ATOM | 3773 | CB | ASP | B | 192 | 33.296 | 64.468 | 92.846 | 1.00 | 43.52 | B | C |
| ATOM | 3774 | CG | ASP | B | 192 | 34.058 | 65.205 | 91.723 | 1.00 | 43.52 | B | C |

| | | | | | | | | | | | | |
|---|---|---|---|---|---|---|---|---|---|---|---|---|
| ATOM | 3775 | OD1 | ASP | B | 192 | 34.935 | 64.583 | 91.066 | 1.00 | 43.52 | B | O |
| ATOM | 3776 | OD2 | ASP | B | 192 | 33.790 | 66.409 | 91.500 | 1.00 | 43.52 | B | O |
| ATOM | 3777 | C | ASP | B | 192 | 32.808 | 62.248 | 93.950 | 1.00 | 33.29 | B | C |
| ATOM | 3778 | O | ASP | B | 192 | 33.475 | 61.946 | 94.927 | 1.00 | 33.29 | B | O |
| ATOM | 3779 | N | THR | B | 193 | 31.508 | 61.971 | 93.868 | 1.00 | 19.77 | B | N |
| ATOM | 3780 | CA | THR | B | 193 | 30.834 | 61.298 | 94.973 | 1.00 | 19.77 | B | C |
| ATOM | 3781 | CB | THR | B | 193 | 29.395 | 61.747 | 95.097 | 1.00 | 25.39 | B | C |
| ATOM | 3782 | OG1 | THR | B | 193 | 28.690 | 61.442 | 93.895 | 1.00 | 25.39 | B | O |
| ATOM | 3783 | CG2 | THR | B | 193 | 29.344 | 63.223 | 95.331 | 1.00 | 25.39 | B | C |
| ATOM | 3784 | C | THR | B | 193 | 30.853 | 59.794 | 94.828 | 1.00 | 19.77 | B | C |
| ATOM | 3785 | O | THR | B | 193 | 30.587 | 59.080 | 95.785 | 1.00 | 19.77 | B | O |
| ATOM | 3786 | N | ALA | B | 194 | 31.177 | 59.316 | 93.631 | 1.00 | 26.84 | B | N |
| ATOM | 3787 | CA | ALA | B | 194 | 31.245 | 57.878 | 93.345 | 1.00 | 26.84 | B | C |
| ATOM | 3788 | CB | ALA | B | 194 | 32.244 | 57.200 | 94.304 | 1.00 | 7.07 | B | C |
| ATOM | 3789 | C | ALA | B | 194 | 29.854 | 57.227 | 93.451 | 1.00 | 26.84 | B | C |
| ATOM | 3790 | O | ALA | B | 194 | 29.701 | 56.038 | 93.722 | 1.00 | 26.84 | B | O |
| ATOM | 3791 | N | VAL | B | 195 | 28.837 | 58.035 | 93.226 | 1.00 | 31.47 | B | N |
| ATOM | 3792 | CA | VAL | B | 195 | 27.476 | 57.567 | 93.279 | 1.00 | 31.47 | B | C |
| ATOM | 3793 | CB | VAL | B | 195 | 26.548 | 58.728 | 93.659 | 1.00 | 19.14 | B | C |
| ATOM | 3794 | CG1 | VAL | B | 195 | 25.088 | 58.316 | 93.502 | 1.00 | 19.14 | B | C |
| ATOM | 3795 | CG2 | VAL | B | 195 | 26.852 | 59.170 | 95.083 | 1.00 | 19.14 | B | C |
| ATOM | 3796 | C | VAL | B | 195 | 27.096 | 57.033 | 91.900 | 1.00 | 31.47 | B | C |
| ATOM | 3797 | O | VAL | B | 195 | 27.450 | 57.630 | 90.881 | 1.00 | 31.47 | B | O |
| ATOM | 3798 | N | LEU | B | 196 | 26.393 | 55.903 | 91.869 | 1.00 | 24.14 | B | N |
| ATOM | 3799 | CA | LEU | B | 196 | 25.961 | 55.301 | 90.612 | 1.00 | 24.14 | B | C |
| ATOM | 3800 | CB | LEU | B | 196 | 26.452 | 53.851 | 90.506 | 1.00 | 15.62 | B | C |
| ATOM | 3801 | CG | LEU | B | 196 | 26.087 | 53.044 | 89.251 | 1.00 | 15.62 | B | C |
| ATOM | 3802 | CD1 | LEU | B | 196 | 26.986 | 51.838 | 89.161 | 1.00 | 15.62 | B | C |
| ATOM | 3803 | CD2 | LEU | B | 196 | 24.636 | 52.606 | 89.285 | 1.00 | 15.62 | B | C |
| ATOM | 3804 | C | LEU | B | 196 | 24.447 | 55.317 | 90.576 | 1.00 | 24.14 | B | C |
| ATOM | 3805 | O | LEU | B | 196 | 23.794 | 54.945 | 91.542 | 1.00 | 24.14 | B | O |
| ATOM | 3806 | N | LYS | B | 197 | 23.889 | 55.732 | 89.453 | 1.00 | 20.25 | B | N |
| ATOM | 3807 | CA | LYS | B | 197 | 22.448 | 55.791 | 89.293 | 1.00 | 20.25 | B | C |
| ATOM | 3808 | CB | LYS | B | 197 | 21.981 | 57.245 | 89.331 | 1.00 | 18.19 | B | C |
| ATOM | 3809 | CG | LYS | B | 197 | 22.201 | 57.942 | 90.646 | 1.00 | 18.19 | B | C |
| ATOM | 3810 | CD | LYS | B | 197 | 21.754 | 59.386 | 90.552 | 1.00 | 18.19 | B | C |
| ATOM | 3811 | CE | LYS | B | 197 | 21.899 | 60.097 | 91.880 | 1.00 | 18.19 | B | C |
| ATOM | 3812 | NZ | LYS | B | 197 | 21.040 | 59.490 | 92.929 | 1.00 | 18.19 | B | N |
| ATOM | 3813 | C | LYS | B | 197 | 21.991 | 55.163 | 87.984 | 1.00 | 20.25 | B | C |
| ATOM | 3814 | O | LYS | B | 197 | 22.381 | 55.621 | 86.911 | 1.00 | 20.25 | B | O |
| ATOM | 3815 | N | LEU | B | 198 | 21.163 | 54.125 | 88.072 | 1.00 | 13.46 | B | N |
| ATOM | 3816 | CA | LEU | B | 198 | 20.641 | 53.460 | 86.880 | 1.00 | 13.46 | B | C |
| ATOM | 3817 | CB | LEU | B | 198 | 19.903 | 52.177 | 87.267 | 1.00 | 13.67 | B | C |
| ATOM | 3818 | CG | LEU | B | 198 | 19.224 | 51.359 | 86.171 | 1.00 | 13.67 | B | C |
| ATOM | 3819 | CD1 | LEU | B | 198 | 20.234 | 50.645 | 85.315 | 1.00 | 13.67 | B | C |
| ATOM | 3820 | CD2 | LEU | B | 198 | 18.321 | 50.350 | 86.831 | 1.00 | 13.67 | B | C |
| ATOM | 3821 | C | LEU | B | 198 | 19.684 | 54.409 | 86.174 | 1.00 | 13.46 | B | C |
| ATOM | 3822 | O | LEU | B | 198 | 19.079 | 55.263 | 86.819 | 1.00 | 13.46 | B | O |
| ATOM | 3823 | N | CYS | B | 199 | 19.546 | 54.252 | 84.859 | 1.00 | 13.25 | B | N |
| ATOM | 3824 | CA | CYS | B | 199 | 18.671 | 55.106 | 84.054 | 1.00 | 13.25 | B | C |
| ATOM | 3825 | CB | CYS | B | 199 | 19.383 | 56.413 | 83.702 | 1.00 | 25.34 | B | C |
| ATOM | 3826 | SG | CYS | B | 199 | 20.574 | 56.287 | 82.349 | 1.00 | 25.34 | B | S |
| ATOM | 3827 | C | CYS | B | 199 | 18.217 | 54.433 | 82.763 | 1.00 | 13.25 | B | C |
| ATOM | 3828 | O | CYS | B | 199 | 18.645 | 53.330 | 82.440 | 1.00 | 13.25 | B | O |
| ATOM | 3829 | N | ASP | B | 200 | 17.355 | 55.122 | 82.022 | 1.00 | 33.37 | B | N |
| ATOM | 3830 | CA | ASP | B | 200 | 16.811 | 54.614 | 80.759 | 1.00 | 33.37 | B | C |
| ATOM | 3831 | CB | ASP | B | 200 | 17.934 | 54.286 | 79.773 | 1.00 | 47.67 | B | C |
| ATOM | 3832 | CG | ASP | B | 200 | 17.426 | 54.077 | 78.349 | 1.00 | 47.67 | B | C |
| ATOM | 3833 | OD1 | ASP | B | 200 | 16.224 | 53.748 | 78.172 | 1.00 | 47.67 | B | O |
| ATOM | 3834 | OD2 | ASP | B | 200 | 18.239 | 54.231 | 77.403 | 1.00 | 47.67 | B | O |
| ATOM | 3835 | C | ASP | B | 200 | 15.938 | 53.368 | 80.974 | 1.00 | 33.37 | B | C |
| ATOM | 3836 | O | ASP | B | 200 | 16.360 | 52.231 | 80.728 | 1.00 | 33.37 | B | O |
| ATOM | 3837 | N | PHE | B | 201 | 14.714 | 53.579 | 81.438 | 1.00 | 27.42 | B | N |

| | | | | | | | | | | | | |
|---|---|---|---|---|---|---|---|---|---|---|---|---|
| ATOM | 3838 | CA | PHE | B | 201 | 13.826 | 52.452 | 81.655 | 1.00 | 27.42 | B | C |
| ATOM | 3839 | CB | PHE | B | 201 | 13.029 | 52.643 | 82.942 | 1.00 | 22.69 | B | C |
| ATOM | 3840 | CG | PHE | B | 201 | 13.831 | 52.392 | 84.187 | 1.00 | 22.69 | B | C |
| ATOM | 3841 | CD1 | PHE | B | 201 | 14.849 | 53.261 | 84.560 | 1.00 | 22.69 | B | C |
| ATOM | 3842 | CD2 | PHE | B | 201 | 13.581 | 51.278 | 84.980 | 1.00 | 22.69 | B | C |
| ATOM | 3843 | CE1 | PHE | B | 201 | 15.609 | 53.026 | 85.710 | 1.00 | 22.69 | B | C |
| ATOM | 3844 | CE2 | PHE | B | 201 | 14.334 | 51.035 | 86.130 | 1.00 | 22.69 | B | C |
| ATOM | 3845 | CZ | PHE | B | 201 | 15.348 | 51.910 | 86.494 | 1.00 | 22.69 | B | C |
| ATOM | 3846 | C | PHE | B | 201 | 12.895 | 52.266 | 80.468 | 1.00 | 27.42 | B | C |
| ATOM | 3847 | O | PHE | B | 201 | 11.802 | 51.716 | 80.596 | 1.00 | 27.42 | B | O |
| ATOM | 3848 | N | GLY | B | 202 | 13.356 | 52.724 | 79.308 | 1.00 | 20.61 | B | N |
| ATOM | 3849 | CA | GLY | B | 202 | 12.575 | 52.614 | 78.093 | 1.00 | 20.61 | B | C |
| ATOM | 3850 | C | GLY | B | 202 | 12.374 | 51.188 | 77.612 | 1.00 | 20.61 | B | C |
| ATOM | 3851 | O | GLY | B | 202 | 11.692 | 50.959 | 76.625 | 1.00 | 20.61 | B | O |
| ATOM | 3852 | N | SER | B | 203 | 12.968 | 50.224 | 78.300 | 1.00 | 30.31 | B | N |
| ATOM | 3853 | CA | SER | B | 203 | 12.819 | 48.827 | 77.914 | 1.00 | 30.31 | B | C |
| ATOM | 3854 | CB | SER | B | 203 | 14.159 | 48.224 | 77.477 | 1.00 | 35.22 | B | C |
| ATOM | 3855 | OG | SER | B | 203 | 14.666 | 48.841 | 76.307 | 1.00 | 35.22 | B | O |
| ATOM | 3856 | C | SER | B | 203 | 12.295 | 48.015 | 79.076 | 1.00 | 30.31 | B | C |
| ATOM | 3857 | O | SER | B | 203 | 12.082 | 46.814 | 78.941 | 1.00 | 30.31 | B | O |
| ATOM | 3858 | N | ALA | B | 204 | 12.096 | 48.671 | 80.218 | 1.00 | 29.27 | B | N |
| ATOM | 3859 | CA | ALA | B | 204 | 11.617 | 47.998 | 81.414 | 1.00 | 29.27 | B | C |
| ATOM | 3860 | CB | ALA | B | 204 | 11.801 | 48.887 | 82.626 | 1.00 | 17.60 | B | C |
| ATOM | 3861 | C | ALA | B | 204 | 10.167 | 47.603 | 81.286 | 1.00 | 29.27 | B | C |
| ATOM | 3862 | O | ALA | B | 204 | 9.405 | 48.220 | 80.545 | 1.00 | 29.27 | B | O |
| ATOM | 3863 | N | LYS | B | 205 | 9.798 | 46.570 | 82.034 | 1.00 | 27.63 | B | N |
| ATOM | 3864 | CA | LYS | B | 205 | 8.441 | 46.038 | 82.053 | 1.00 | 27.63 | B | C |
| ATOM | 3865 | CB | LYS | B | 205 | 8.241 | 45.099 | 80.856 | 1.00 | 29.45 | B | C |
| ATOM | 3866 | CG | LYS | B | 205 | 6.899 | 44.397 | 80.784 | 1.00 | 29.45 | B | C |
| ATOM | 3867 | CD | LYS | B | 205 | 6.807 | 43.549 | 79.518 | 1.00 | 29.45 | B | C |
| ATOM | 3868 | CE | LYS | B | 205 | 5.459 | 42.841 | 79.364 | 1.00 | 29.45 | B | C |
| ATOM | 3869 | NZ | LYS | B | 205 | 5.366 | 42.159 | 78.031 | 1.00 | 29.45 | B | N |
| ATOM | 3870 | C | LYS | B | 205 | 8.181 | 45.274 | 83.356 | 1.00 | 27.63 | B | C |
| ATOM | 3871 | O | LYS | B | 205 | 9.075 | 44.628 | 83.921 | 1.00 | 27.63 | B | O |
| ATOM | 3872 | N | GLN | B | 206 | 6.950 | 45.373 | 83.839 | 1.00 | 28.11 | B | N |
| ATOM | 3873 | CA | GLN | B | 206 | 6.535 | 44.660 | 85.041 | 1.00 | 28.11 | B | C |
| ATOM | 3874 | CB | GLN | B | 206 | 5.174 | 45.188 | 85.467 | 1.00 | 46.32 | B | C |
| ATOM | 3875 | CG | GLN | B | 206 | 5.189 | 46.150 | 86.624 | 1.00 | 46.32 | B | C |
| ATOM | 3876 | CD | GLN | B | 206 | 4.891 | 45.444 | 87.929 | 1.00 | 46.32 | B | C |
| ATOM | 3877 | OE1 | GLN | B | 206 | 4.926 | 46.058 | 89.003 | 1.00 | 46.32 | B | O |
| ATOM | 3878 | NE2 | GLN | B | 206 | 4.595 | 44.138 | 87.846 | 1.00 | 46.32 | B | N |
| ATOM | 3879 | C | GLN | B | 206 | 6.409 | 43.188 | 84.628 | 1.00 | 28.11 | B | C |
| ATOM | 3880 | O | GLN | B | 206 | 5.451 | 42.831 | 83.962 | 1.00 | 28.11 | B | O |
| ATOM | 3881 | N | LEU | B | 207 | 7.369 | 42.341 | 84.989 | 1.00 | 34.86 | B | N |
| ATOM | 3882 | CA | LEU | B | 207 | 7.287 | 40.931 | 84.596 | 1.00 | 34.86 | B | C |
| ATOM | 3883 | CB | LEU | B | 207 | 8.646 | 40.226 | 84.677 | 1.00 | 17.27 | B | C |
| ATOM | 3884 | CG | LEU | B | 207 | 9.785 | 40.598 | 83.725 | 1.00 | 17.27 | B | C |
| ATOM | 3885 | CD1 | LEU | B | 207 | 10.791 | 39.462 | 83.689 | 1.00 | 17.27 | B | C |
| ATOM | 3886 | CD2 | LEU | B | 207 | 9.250 | 40.861 | 82.329 | 1.00 | 17.27 | B | C |
| ATOM | 3887 | C | LEU | B | 207 | 6.294 | 40.147 | 85.439 | 1.00 | 34.86 | B | C |
| ATOM | 3888 | O | LEU | B | 207 | 6.524 | 39.885 | 86.623 | 1.00 | 34.86 | B | O |
| ATOM | 3889 | N | VAL | B | 208 | 5.194 | 39.756 | 84.807 | 1.00 | 40.24 | B | N |
| ATOM | 3890 | CA | VAL | B | 208 | 4.140 | 39.009 | 85.466 | 1.00 | 40.24 | B | C |
| ATOM | 3891 | CB | VAL | B | 208 | 2.768 | 39.586 | 85.072 | 1.00 | 23.70 | B | C |
| ATOM | 3892 | CG1 | VAL | B | 208 | 1.663 | 38.864 | 85.795 | 1.00 | 23.70 | B | C |
| ATOM | 3893 | CG2 | VAL | B | 208 | 2.731 | 41.061 | 85.388 | 1.00 | 23.70 | B | C |
| ATOM | 3894 | C | VAL | B | 208 | 4.221 | 37.537 | 85.062 | 1.00 | 40.24 | B | C |
| ATOM | 3895 | O | VAL | B | 208 | 4.140 | 37.210 | 83.873 | 1.00 | 40.24 | B | O |
| ATOM | 3896 | N | ALA | B | 209 | 4.385 | 36.665 | 86.061 | 1.00 | 38.06 | B | N |
| ATOM | 3897 | CA | ALA | B | 209 | 4.481 | 35.217 | 85.864 | 1.00 | 38.06 | B | C |
| ATOM | 3898 | CB | ALA | B | 209 | 4.315 | 34.490 | 87.200 | 1.00 | 20.16 | B | C |
| ATOM | 3899 | C | ALA | B | 209 | 3.439 | 34.713 | 84.872 | 1.00 | 38.06 | B | C |
| ATOM | 3900 | O | ALA | B | 209 | 2.307 | 35.220 | 84.815 | 1.00 | 38.06 | B | O |

| | | | | | | | | | | | | |
|---|---|---|---|---|---|---|---|---|---|---|---|---|
| ATOM | 3901 | N | GLY | B | 210 | 3.832 | 33.715 | 84.086 | 1.00 | 32.96 | B | N |
| ATOM | 3902 | CA | GLY | B | 210 | 2.925 | 33.159 | 83.102 | 1.00 | 32.96 | B | C |
| ATOM | 3903 | C | GLY | B | 210 | 2.738 | 34.028 | 81.875 | 1.00 | 32.96 | B | C |
| ATOM | 3904 | O | GLY | B | 210 | 2.419 | 33.521 | 80.805 | 1.00 | 32.96 | B | O |
| ATOM | 3905 | N | GLU | B | 211 | 2.910 | 35.338 | 82.025 | 1.00 | 45.91 | B | N |
| ATOM | 3906 | CA | GLU | B | 211 | 2.756 | 36.249 | 80.895 | 1.00 | 45.91 | B | C |
| ATOM | 3907 | CB | GLU | B | 211 | 2.490 | 37.678 | 81.378 | 1.00 | 56.63 | B | C |
| ATOM | 3908 | CG | GLU | B | 211 | 1.156 | 37.875 | 82.088 | 1.00 | 56.63 | B | C |
| ATOM | 3909 | CD | GLU | B | 211 | 0.802 | 39.355 | 82.320 | 1.00 | 56.63 | B | C |
| ATOM | 3910 | OE1 | GLU | B | 211 | -0.317 | 39.620 | 82.810 | 1.00 | 56.63 | B | O |
| ATOM | 3911 | OE2 | GLU | B | 211 | 1.629 | 40.254 | 82.019 | 1.00 | 56.63 | B | O |
| ATOM | 3912 | C | GLU | B | 211 | 4.024 | 36.218 | 80.043 | 1.00 | 45.91 | B | C |
| ATOM | 3913 | O | GLU | B | 211 | 5.141 | 36.160 | 80.563 | 1.00 | 45.91 | B | O |
| ATOM | 3914 | N | PRO | B | 212 | 3.872 | 36.243 | 78.717 | 1.00 | 48.50 | B | N |
| ATOM | 3915 | CD | PRO | B | 212 | 2.626 | 36.080 | 77.956 | 1.00 | 35.05 | B | C |
| ATOM | 3916 | CA | PRO | B | 212 | 5.037 | 36.212 | 77.822 | 1.00 | 48.50 | B | C |
| ATOM | 3917 | CB | PRO | B | 212 | 4.437 | 35.782 | 76.480 | 1.00 | 35.05 | B | C |
| ATOM | 3918 | CG | PRO | B | 212 | 3.085 | 35.191 | 76.848 | 1.00 | 35.05 | B | C |
| ATOM | 3919 | C | PRO | B | 212 | 5.677 | 37.593 | 77.710 | 1.00 | 48.50 | B | C |
| ATOM | 3920 | O | PRO | B | 212 | 5.024 | 38.611 | 77.952 | 1.00 | 48.50 | B | O |
| ATOM | 3921 | N | ASN | B | 213 | 6.952 | 37.626 | 77.346 | 1.00 | 22.62 | B | N |
| ATOM | 3922 | CA | ASN | B | 213 | 7.641 | 38.891 | 77.162 | 1.00 | 22.62 | B | C |
| ATOM | 3923 | CB | ASN | B | 213 | 8.515 | 39.208 | 78.364 | 1.00 | 26.17 | B | C |
| ATOM | 3924 | CG | ASN | B | 213 | 7.747 | 39.185 | 79.667 | 1.00 | 26.17 | B | C |
| ATOM | 3925 | OD1 | ASN | B | 213 | 6.856 | 40.013 | 79.901 | 1.00 | 26.17 | B | O |
| ATOM | 3926 | ND2 | ASN | B | 213 | 8.088 | 38.229 | 80.528 | 1.00 | 26.17 | B | N |
| ATOM | 3927 | C | ASN | B | 213 | 8.508 | 38.697 | 75.945 | 1.00 | 22.62 | B | C |
| ATOM | 3928 | O | ASN | B | 213 | 8.979 | 37.583 | 75.701 | 1.00 | 22.62 | B | O |
| ATOM | 3929 | N | VAL | B | 214 | 8.710 | 39.767 | 75.176 | 1.00 | 27.02 | B | N |
| ATOM | 3930 | CA | VAL | B | 214 | 9.525 | 39.677 | 73.966 | 1.00 | 27.02 | B | C |
| ATOM | 3931 | CB | VAL | B | 214 | 9.416 | 40.961 | 73.126 | 1.00 | 27.52 | B | C |
| ATOM | 3932 | CG1 | VAL | B | 214 | 7.977 | 41.137 | 72.658 | 1.00 | 27.52 | B | C |
| ATOM | 3933 | CG2 | VAL | B | 214 | 9.884 | 42.162 | 73.927 | 1.00 | 27.52 | B | C |
| ATOM | 3934 | C | VAL | B | 214 | 10.987 | 39.385 | 74.274 | 1.00 | 27.02 | B | C |
| ATOM | 3935 | O | VAL | B | 214 | 11.517 | 39.822 | 75.296 | 1.00 | 27.02 | B | O |
| ATOM | 3936 | N | SER | B | 215 | 11.637 | 38.640 | 73.391 | 1.00 | 26.29 | B | N |
| ATOM | 3937 | CA | SER | B | 215 | 13.028 | 38.275 | 73.603 | 1.00 | 26.29 | B | C |
| ATOM | 3938 | CB | SER | B | 215 | 13.218 | 36.785 | 73.301 | 1.00 | 37.52 | B | C |
| ATOM | 3939 | OG | SER | B | 215 | 12.713 | 36.440 | 72.018 | 1.00 | 37.52 | B | O |
| ATOM | 3940 | C | SER | B | 215 | 14.067 | 39.095 | 72.842 | 1.00 | 26.29 | B | C |
| ATOM | 3941 | O | SER | B | 215 | 15.252 | 38.800 | 72.912 | 1.00 | 26.29 | B | O |
| ATOM | 3942 | N | TYR | B | 216 | 13.638 | 40.120 | 72.121 | 1.00 | 34.00 | B | N |
| ATOM | 3943 | CA | TYR | B | 216 | 14.584 | 40.951 | 71.382 | 1.00 | 34.00 | B | C |
| ATOM | 3944 | CB | TYR | B | 216 | 14.002 | 41.308 | 70.008 | 1.00 | 25.15 | B | C |
| ATOM | 3945 | CG | TYR | B | 216 | 12.631 | 41.953 | 70.040 | 1.00 | 25.15 | B | C |
| ATOM | 3946 | CD1 | TYR | B | 216 | 12.475 | 43.319 | 70.291 | 1.00 | 25.15 | B | C |
| ATOM | 3947 | CE1 | TYR | B | 216 | 11.210 | 43.915 | 70.295 | 1.00 | 25.15 | B | C |
| ATOM | 3948 | CD2 | TYR | B | 216 | 11.488 | 41.199 | 69.800 | 1.00 | 25.15 | B | C |
| ATOM | 3949 | CE2 | TYR | B | 216 | 10.219 | 41.783 | 69.801 | 1.00 | 25.15 | B | C |
| ATOM | 3950 | CZ | TYR | B | 216 | 10.088 | 43.137 | 70.044 | 1.00 | 25.15 | B | C |
| ATOM | 3951 | OH | TYR | B | 216 | 8.839 | 43.709 | 69.999 | 1.00 | 25.15 | B | O |
| ATOM | 3952 | C | TYR | B | 216 | 14.859 | 42.223 | 72.184 | 1.00 | 34.00 | B | C |
| ATOM | 3953 | O | TYR | B | 216 | 15.147 | 43.285 | 71.623 | 1.00 | 34.00 | B | O |
| ATOM | 3954 | N | ILE | B | 217 | 14.827 | 42.088 | 73.502 | 1.00 | 30.37 | B | N |
| ATOM | 3955 | CA | ILE | B | 217 | 14.968 | 43.225 | 74.386 | 1.00 | 30.37 | B | C |
| ATOM | 3956 | CB | ILE | B | 217 | 14.033 | 42.987 | 75.611 | 1.00 | 14.70 | B | C |
| ATOM | 3957 | CG2 | ILE | B | 217 | 14.725 | 42.154 | 76.681 | 1.00 | 14.70 | B | C |
| ATOM | 3958 | CG1 | ILE | B | 217 | 13.569 | 44.324 | 76.147 | 1.00 | 14.70 | B | C |
| ATOM | 3959 | CD1 | ILE | B | 217 | 12.900 | 45.173 | 75.071 | 1.00 | 14.70 | B | C |
| ATOM | 3960 | C | ILE | B | 217 | 16.334 | 43.738 | 74.884 | 1.00 | 30.37 | B | C |
| ATOM | 3961 | O | ILE | B | 217 | 16.483 | 44.932 | 75.179 | 1.00 | 30.37 | B | O |
| ATOM | 3962 | N | CYS | B | 218 | 17.334 | 42.877 | 74.968 | 1.00 | 25.01 | B | N |
| ATOM | 3963 | CA | CYS | B | 218 | 18.616 | 43.328 | 75.499 | 1.00 | 25.01 | B | C |

| | | | | | | | | | | | | |
|---|---|---|---|---|---|---|---|---|---|---|---|---|
| ATOM | 3964 | CB | CYS | B | 218 | 19.151 | 42.244 | 76.433 | 1.00 | 26.63 | B | C |
| ATOM | 3965 | SG | CYS | B | 218 | 20.338 | 42.777 | 77.643 | 1.00 | 26.63 | B | S |
| ATOM | 3966 | C | CYS | B | 218 | 19.677 | 43.728 | 74.450 | 1.00 | 25.01 | B | C |
| ATOM | 3967 | O | CYS | B | 218 | 19.548 | 43.415 | 73.259 | 1.00 | 25.01 | B | O |
| ATOM | 3968 | N | SER | B | 219 | 20.718 | 44.428 | 74.899 | 1.00 | 22.97 | B | N |
| ATOM | 3969 | CA | SER | B | 219 | 21.798 | 44.869 | 74.016 | 1.00 | 22.97 | B | C |
| ATOM | 3970 | CB | SER | B | 219 | 22.398 | 46.184 | 74.510 | 1.00 | 35.90 | B | C |
| ATOM | 3971 | OG | SER | B | 219 | 21.494 | 47.258 | 74.359 | 1.00 | 35.90 | B | O |
| ATOM | 3972 | C | SER | B | 219 | 22.936 | 43.869 | 73.860 | 1.00 | 22.97 | B | C |
| ATOM | 3973 | O | SER | B | 219 | 23.244 | 43.112 | 74.770 | 1.00 | 22.97 | B | O |
| ATOM | 3974 | N | ARG | B | 220 | 23.568 | 43.932 | 72.692 | 1.00 | 41.43 | B | N |
| ATOM | 3975 | CA | ARG | B | 220 | 24.689 | 43.096 | 72.237 | 1.00 | 41.43 | B | C |
| ATOM | 3976 | CB | ARG | B | 220 | 25.661 | 43.983 | 71.457 | 1.00 | 71.33 | B | C |
| ATOM | 3977 | CG | ARG | B | 220 | 26.316 | 43.328 | 70.229 | 1.00 | 71.33 | B | C |
| ATOM | 3978 | CD | ARG | B | 220 | 27.156 | 44.361 | 69.465 | 1.00 | 71.33 | B | C |
| ATOM | 3979 | NE | ARG | B | 220 | 27.374 | 45.558 | 70.284 | 1.00 | 71.33 | B | N |
| ATOM | 3980 | CZ | ARG | B | 220 | 28.277 | 46.506 | 70.034 | 1.00 | 71.33 | B | C |
| ATOM | 3981 | NH1 | ARG | B | 220 | 29.079 | 46.413 | 68.965 | 1.00 | 71.33 | B | N |
| ATOM | 3982 | NH2 | ARG | B | 220 | 28.374 | 47.552 | 70.862 | 1.00 | 71.33 | B | N |
| ATOM | 3983 | C | ARG | B | 220 | 25.498 | 42.180 | 73.172 | 1.00 | 41.43 | B | C |
| ATOM | 3984 | O | ARG | B | 220 | 25.189 | 40.985 | 73.285 | 1.00 | 41.43 | B | O |
| ATOM | 3985 | N | TYR | B | 221 | 26.544 | 42.698 | 73.812 | 1.00 | 20.42 | B | N |
| ATOM | 3986 | CA | TYR | B | 221 | 27.371 | 41.850 | 74.683 | 1.00 | 20.42 | B | C |
| ATOM | 3987 | CB | TYR | B | 221 | 28.684 | 42.552 | 74.996 | 1.00 | 38.33 | B | C |
| ATOM | 3988 | CG | TYR | B | 221 | 29.359 | 43.176 | 73.809 | 1.00 | 38.33 | B | C |
| ATOM | 3989 | CD1 | TYR | B | 221 | 29.551 | 42.454 | 72.631 | 1.00 | 38.33 | B | C |
| ATOM | 3990 | CE1 | TYR | B | 221 | 30.218 | 43.003 | 71.552 | 1.00 | 38.33 | B | C |
| ATOM | 3991 | CD2 | TYR | B | 221 | 29.848 | 44.478 | 73.872 | 1.00 | 38.33 | B | C |
| ATOM | 3992 | CE2 | TYR | B | 221 | 30.520 | 45.040 | 72.792 | 1.00 | 38.33 | B | C |
| ATOM | 3993 | CZ | TYR | B | 221 | 30.699 | 44.292 | 71.638 | 1.00 | 38.33 | B | C |
| ATOM | 3994 | OH | TYR | B | 221 | 31.378 | 44.827 | 70.568 | 1.00 | 38.33 | B | O |
| ATOM | 3995 | C | TYR | B | 221 | 26.767 | 41.444 | 76.026 | 1.00 | 20.42 | B | C |
| ATOM | 3996 | O | TYR | B | 221 | 27.333 | 40.605 | 76.720 | 1.00 | 20.42 | B | O |
| ATOM | 3997 | N | TYR | B | 222 | 25.618 | 42.018 | 76.376 | 1.00 | 31.39 | B | N |
| ATOM | 3998 | CA | TYR | B | 222 | 25.005 | 41.766 | 77.672 | 1.00 | 31.39 | B | C |
| ATOM | 3999 | CB | TYR | B | 222 | 24.718 | 43.120 | 78.316 | 1.00 | 27.59 | B | C |
| ATOM | 4000 | CG | TYR | B | 222 | 25.860 | 44.084 | 78.101 | 1.00 | 27.59 | B | C |
| ATOM | 4001 | CD1 | TYR | B | 222 | 26.993 | 44.056 | 78.920 | 1.00 | 27.59 | B | C |
| ATOM | 4002 | CE1 | TYR | B | 222 | 28.078 | 44.896 | 78.677 | 1.00 | 27.59 | B | C |
| ATOM | 4003 | CD2 | TYR | B | 222 | 25.840 | 44.975 | 77.038 | 1.00 | 27.59 | B | C |
| ATOM | 4004 | CE2 | TYR | B | 222 | 26.911 | 45.814 | 76.788 | 1.00 | 27.59 | B | C |
| ATOM | 4005 | CZ | TYR | B | 222 | 28.033 | 45.778 | 77.607 | 1.00 | 27.59 | B | C |
| ATOM | 4006 | OH | TYR | B | 222 | 29.089 | 46.634 | 77.356 | 1.00 | 27.59 | B | O |
| ATOM | 4007 | C | TYR | B | 222 | 23.770 | 40.892 | 77.774 | 1.00 | 31.39 | B | C |
| ATOM | 4008 | O | TYR | B | 222 | 23.235 | 40.720 | 78.868 | 1.00 | 31.39 | B | O |
| ATOM | 4009 | N | ARG | B | 223 | 23.323 | 40.329 | 76.659 | 1.00 | 24.07 | B | N |
| ATOM | 4010 | CA | ARG | B | 223 | 22.123 | 39.497 | 76.655 | 1.00 | 24.07 | B | C |
| ATOM | 4011 | CB | ARG | B | 223 | 21.664 | 39.239 | 75.226 | 1.00 | 22.52 | B | C |
| ATOM | 4012 | CG | ARG | B | 223 | 21.995 | 40.349 | 74.263 | 1.00 | 22.52 | B | C |
| ATOM | 4013 | CD | ARG | B | 223 | 21.227 | 40.170 | 72.992 | 1.00 | 22.52 | B | C |
| ATOM | 4014 | NE | ARG | B | 223 | 19.807 | 40.372 | 73.240 | 1.00 | 22.52 | B | N |
| ATOM | 4015 | CZ | ARG | B | 223 | 18.845 | 39.565 | 72.813 | 1.00 | 22.52 | B | C |
| ATOM | 4016 | NH1 | ARG | B | 223 | 19.137 | 38.484 | 72.105 | 1.00 | 22.52 | B | N |
| ATOM | 4017 | NH2 | ARG | B | 223 | 17.589 | 39.839 | 73.115 | 1.00 | 22.52 | B | N |
| ATOM | 4018 | C | ARG | B | 223 | 22.356 | 38.168 | 77.336 | 1.00 | 24.07 | B | C |
| ATOM | 4019 | O | ARG | B | 223 | 23.406 | 37.555 | 77.167 | 1.00 | 24.07 | B | O |
| ATOM | 4020 | N | ALA | B | 224 | 21.372 | 37.721 | 78.108 | 1.00 | 23.11 | B | N |
| ATOM | 4021 | CA | ALA | B | 224 | 21.479 | 36.452 | 78.803 | 1.00 | 23.11 | B | C |
| ATOM | 4022 | CB | ALA | B | 224 | 20.420 | 36.361 | 79.876 | 1.00 | 39.52 | B | C |
| ATOM | 4023 | C | ALA | B | 224 | 21.282 | 35.349 | 77.777 | 1.00 | 23.11 | B | C |
| ATOM | 4024 | O | ALA | B | 224 | 20.626 | 35.555 | 76.751 | 1.00 | 23.11 | B | O |
| ATOM | 4025 | N | PRO | B | 225 | 21.848 | 34.157 | 78.044 | 1.00 | 25.35 | B | N |
| ATOM | 4026 | CD | PRO | B | 225 | 22.498 | 33.815 | 79.322 | 1.00 | 29.95 | B | C |

| | | | | | | | | | | | | |
|---|---|---|---|---|---|---|---|---|---|---|---|---|
| ATOM | 4027 | CA | PRO | B | 225 | 21.763 | 32.977 | 77.170 | 1.00 | 25.35 | B | C |
| ATOM | 4028 | CB | PRO | B | 225 | 22.314 | 31.856 | 78.039 | 1.00 | 29.95 | B | C |
| ATOM | 4029 | CG | PRO | B | 225 | 23.236 | 32.552 | 78.966 | 1.00 | 29.95 | B | C |
| ATOM | 4030 | C | PRO | B | 225 | 20.319 | 32.705 | 76.748 | 1.00 | 25.35 | B | C |
| ATOM | 4031 | O | PRO | B | 225 | 20.038 | 32.510 | 75.575 | 1.00 | 25.35 | B | O |
| ATOM | 4032 | N | GLU | B | 226 | 19.401 | 32.698 | 77.707 | 1.00 | 21.36 | B | N |
| ATOM | 4033 | CA | GLU | B | 226 | 18.005 | 32.453 | 77.386 | 1.00 | 21.36 | B | C |
| ATOM | 4034 | CB | GLU | B | 226 | 17.133 | 32.477 | 78.648 | 1.00 | 25.12 | B | C |
| ATOM | 4035 | CG | GLU | B | 226 | 17.170 | 33.785 | 79.432 | 1.00 | 25.12 | B | C |
| ATOM | 4036 | CD | GLU | B | 226 | 18.056 | 33.711 | 80.661 | 1.00 | 25.12 | B | C |
| ATOM | 4037 | OE1 | GLU | B | 226 | 19.202 | 33.247 | 80.534 | 1.00 | 25.12 | B | O |
| ATOM | 4038 | OE2 | GLU | B | 226 | 17.606 | 34.119 | 81.751 | 1.00 | 25.12 | B | O |
| ATOM | 4039 | C | GLU | B | 226 | 17.453 | 33.460 | 76.383 | 1.00 | 21.36 | B | C |
| ATOM | 4040 | O | GLU | B | 226 | 16.589 | 33.123 | 75.581 | 1.00 | 21.36 | B | O |
| ATOM | 4041 | N | LEU | B | 227 | 17.937 | 34.697 | 76.432 | 1.00 | 27.19 | B | N |
| ATOM | 4042 | CA | LEU | B | 227 | 17.462 | 35.715 | 75.503 | 1.00 | 27.19 | B | C |
| ATOM | 4043 | CB | LEU | B | 227 | 17.924 | 37.109 | 75.933 | 1.00 | 19.10 | B | C |
| ATOM | 4044 | CG | LEU | B | 227 | 17.323 | 37.750 | 77.186 | 1.00 | 19.10 | B | C |
| ATOM | 4045 | CD1 | LEU | B | 227 | 17.618 | 39.235 | 77.125 | 1.00 | 19.10 | B | C |
| ATOM | 4046 | CD2 | LEU | B | 227 | 15.822 | 37.532 | 77.253 | 1.00 | 19.10 | B | C |
| ATOM | 4047 | C | LEU | B | 227 | 17.962 | 35.421 | 74.091 | 1.00 | 27.19 | B | C |
| ATOM | 4048 | O | LEU | B | 227 | 17.252 | 35.634 | 73.112 | 1.00 | 27.19 | B | O |
| ATOM | 4049 | N | ILE | B | 228 | 19.195 | 34.935 | 73.997 | 1.00 | 22.44 | B | N |
| ATOM | 4050 | CA | ILE | B | 228 | 19.783 | 34.589 | 72.722 | 1.00 | 22.44 | B | C |
| ATOM | 4051 | CB | ILE | B | 228 | 21.255 | 34.196 | 72.908 | 1.00 | 11.12 | B | C |
| ATOM | 4052 | CG2 | ILE | B | 228 | 21.851 | 33.741 | 71.602 | 1.00 | 11.12 | B | C |
| ATOM | 4053 | CG1 | ILE | B | 228 | 22.037 | 35.396 | 73.440 | 1.00 | 11.12 | B | C |
| ATOM | 4054 | CD1 | ILE | B | 228 | 23.515 | 35.121 | 73.695 | 1.00 | 11.12 | B | C |
| ATOM | 4055 | C | ILE | B | 228 | 18.999 | 33.421 | 72.125 | 1.00 | 22.44 | B | C |
| ATOM | 4056 | O | ILE | B | 228 | 18.772 | 33.349 | 70.911 | 1.00 | 22.44 | B | O |
| ATOM | 4057 | N | PHE | B | 229 | 18.588 | 32.499 | 72.988 | 1.00 | 26.42 | B | N |
| ATOM | 4058 | CA | PHE | B | 229 | 17.816 | 31.340 | 72.549 | 1.00 | 26.42 | B | C |
| ATOM | 4059 | CB | PHE | B | 229 | 17.855 | 30.213 | 73.588 | 1.00 | 16.73 | B | C |
| ATOM | 4060 | CG | PHE | B | 229 | 19.179 | 29.519 | 73.693 | 1.00 | 16.73 | B | C |
| ATOM | 4061 | CD1 | PHE | B | 229 | 19.804 | 29.009 | 72.574 | 1.00 | 16.73 | B | C |
| ATOM | 4062 | CD2 | PHE | B | 229 | 19.786 | 29.344 | 74.927 | 1.00 | 16.73 | B | C |
| ATOM | 4063 | CE1 | PHE | B | 229 | 21.015 | 28.329 | 72.681 | 1.00 | 16.73 | B | C |
| ATOM | 4064 | CE2 | PHE | B | 229 | 20.996 | 28.664 | 75.037 | 1.00 | 16.73 | B | C |
| ATOM | 4065 | CZ | PHE | B | 229 | 21.606 | 28.158 | 73.907 | 1.00 | 16.73 | B | C |
| ATOM | 4066 | C | PHE | B | 229 | 16.359 | 31.686 | 72.246 | 1.00 | 26.42 | B | C |
| ATOM | 4067 | O | PHE | B | 229 | 15.592 | 30.810 | 71.861 | 1.00 | 26.42 | B | O |
| ATOM | 4068 | N | GLY | B | 230 | 15.979 | 32.949 | 72.429 | 1.00 | 22.42 | B | N |
| ATOM | 4069 | CA | GLY | B | 230 | 14.616 | 33.365 | 72.138 | 1.00 | 22.42 | B | C |
| ATOM | 4070 | C | GLY | B | 230 | 13.569 | 33.072 | 73.200 | 1.00 | 22.42 | B | C |
| ATOM | 4071 | O | GLY | B | 230 | 12.379 | 33.234 | 72.955 | 1.00 | 22.42 | B | O |
| ATOM | 4072 | N | ALA | B | 231 | 13.992 | 32.639 | 74.378 | 1.00 | 17.62 | B | N |
| ATOM | 4073 | CA | ALA | B | 231 | 13.041 | 32.358 | 75.434 | 1.00 | 17.62 | B | C |
| ATOM | 4074 | CB | ALA | B | 231 | 13.770 | 32.005 | 76.705 | 1.00 | 7.73 | B | C |
| ATOM | 4075 | C | ALA | B | 231 | 12.186 | 33.588 | 75.650 | 1.00 | 17.62 | B | C |
| ATOM | 4076 | O | ALA | B | 231 | 12.667 | 34.704 | 75.501 | 1.00 | 17.62 | B | O |
| ATOM | 4077 | N | THR | B | 232 | 10.916 | 33.390 | 75.990 | 1.00 | 34.74 | B | N |
| ATOM | 4078 | CA | THR | B | 232 | 10.019 | 34.513 | 76.234 | 1.00 | 34.74 | B | C |
| ATOM | 4079 | CB | THR | B | 232 | 8.834 | 34.495 | 75.276 | 1.00 | 53.64 | B | C |
| ATOM | 4080 | OG1 | THR | B | 232 | 8.118 | 33.263 | 75.448 | 1.00 | 53.64 | B | O |
| ATOM | 4081 | CG2 | THR | B | 232 | 9.313 | 34.649 | 73.825 | 1.00 | 53.64 | B | C |
| ATOM | 4082 | C | THR | B | 232 | 9.478 | 34.448 | 77.649 | 1.00 | 34.74 | B | C |
| ATOM | 4083 | O | THR | B | 232 | 8.756 | 35.342 | 78.101 | 1.00 | 34.74 | B | O |
| ATOM | 4084 | N | ASP | B | 233 | 9.840 | 33.378 | 78.340 | 1.00 | 23.31 | B | N |
| ATOM | 4085 | CA | ASP | B | 233 | 9.403 | 33.133 | 79.708 | 1.00 | 23.31 | B | C |
| ATOM | 4086 | CB | ASP | B | 233 | 8.988 | 31.679 | 79.830 | 1.00 | 35.56 | B | C |
| ATOM | 4087 | CG | ASP | B | 233 | 10.123 | 30.752 | 79.497 | 1.00 | 35.56 | B | C |
| ATOM | 4088 | OD1 | ASP | B | 233 | 11.016 | 31.187 | 78.733 | 1.00 | 35.56 | B | O |
| ATOM | 4089 | OD2 | ASP | B | 233 | 10.136 | 29.600 | 79.980 | 1.00 | 35.56 | B | O |

| | | | | | | | | | | | | |
|---|---|---|---|---|---|---|---|---|---|---|---|---|
| ATOM | 4090 | C | ASP | B | 233 | 10.562 | 33.413 | 80.663 | 1.00 | 23.31 | B | C |
| ATOM | 4091 | O | ASP | B | 233 | 10.790 | 32.665 | 81.613 | 1.00 | 23.31 | B | O |
| ATOM | 4092 | N | TYR | B | 234 | 11.290 | 34.495 | 80.409 | 1.00 | 32.53 | B | N |
| ATOM | 4093 | CA | TYR | B | 234 | 12.423 | 34.859 | 81.254 | 1.00 | 32.53 | B | C |
| ATOM | 4094 | CB | TYR | B | 234 | 13.470 | 35.653 | 80.452 | 1.00 | 18.24 | B | C |
| ATOM | 4095 | CG | TYR | B | 234 | 12.966 | 36.903 | 79.746 | 1.00 | 18.24 | B | C |
| ATOM | 4096 | CD1 | TYR | B | 234 | 12.317 | 36.822 | 78.520 | 1.00 | 18.24 | B | C |
| ATOM | 4097 | CE1 | TYR | B | 234 | 11.878 | 37.973 | 77.852 | 1.00 | 18.24 | B | C |
| ATOM | 4098 | CD2 | TYR | B | 234 | 13.166 | 38.172 | 80.295 | 1.00 | 18.24 | B | C |
| ATOM | 4099 | CE2 | TYR | B | 234 | 12.727 | 39.330 | 79.635 | 1.00 | 18.24 | B | C |
| ATOM | 4100 | CZ | TYR | B | 234 | 12.080 | 39.219 | 78.414 | 1.00 | 18.24 | B | C |
| ATOM | 4101 | OH | TYR | B | 234 | 11.599 | 40.339 | 77.772 | 1.00 | 18.24 | B | O |
| ATOM | 4102 | C | TYR | B | 234 | 12.020 | 35.671 | 82.474 | 1.00 | 32.53 | B | C |
| ATOM | 4103 | O | TYR | B | 234 | 10.966 | 36.304 | 82.488 | 1.00 | 32.53 | B | O |
| ATOM | 4104 | N | THR | B | 235 | 12.866 | 35.647 | 83.496 | 1.00 | 20.87 | B | N |
| ATOM | 4105 | CA | THR | B | 235 | 12.617 | 36.417 | 84.707 | 1.00 | 20.87 | B | C |
| ATOM | 4106 | CB | THR | B | 235 | 12.776 | 35.572 | 85.983 | 1.00 | 27.25 | B | C |
| ATOM | 4107 | OG1 | THR | B | 235 | 13.736 | 34.537 | 85.756 | 1.00 | 27.25 | B | O |
| ATOM | 4108 | CG2 | THR | B | 235 | 11.444 | 34.972 | 86.396 | 1.00 | 27.25 | B | C |
| ATOM | 4109 | C | THR | B | 235 | 13.568 | 37.590 | 84.815 | 1.00 | 20.87 | B | C |
| ATOM | 4110 | O | THR | B | 235 | 14.148 | 38.034 | 83.831 | 1.00 | 20.87 | B | O |
| ATOM | 4111 | N | SER | B | 236 | 13.719 | 38.101 | 86.025 | 1.00 | 27.16 | B | N |
| ATOM | 4112 | CA | SER | B | 236 | 14.614 | 39.221 | 86.249 | 1.00 | 27.16 | B | C |
| ATOM | 4113 | CB | SER | B | 236 | 14.307 | 39.880 | 87.581 | 1.00 | 24.74 | B | C |
| ATOM | 4114 | OG | SER | B | 236 | 12.990 | 40.358 | 87.560 | 1.00 | 24.74 | B | O |
| ATOM | 4115 | C | SER | B | 236 | 16.056 | 38.749 | 86.257 | 1.00 | 27.16 | B | C |
| ATOM | 4116 | O | SER | B | 236 | 16.970 | 39.561 | 86.330 | 1.00 | 27.16 | B | O |
| ATOM | 4117 | N | SER | B | 237 | 16.259 | 37.438 | 86.195 | 1.00 | 25.87 | B | N |
| ATOM | 4118 | CA | SER | B | 237 | 17.613 | 36.911 | 86.215 | 1.00 | 25.87 | B | C |
| ATOM | 4119 | CB | SER | B | 237 | 17.636 | 35.378 | 86.076 | 1.00 | 31.90 | B | C |
| ATOM | 4120 | OG | SER | B | 237 | 16.361 | 34.791 | 86.244 | 1.00 | 31.90 | B | O |
| ATOM | 4121 | C | SER | B | 237 | 18.418 | 37.506 | 85.068 | 1.00 | 25.87 | B | C |
| ATOM | 4122 | O | SER | B | 237 | 19.652 | 37.557 | 85.126 | 1.00 | 25.87 | B | O |
| ATOM | 4123 | N | ILE | B | 238 | 17.718 | 37.964 | 84.033 | 1.00 | 17.87 | B | N |
| ATOM | 4124 | CA | ILE | B | 238 | 18.388 | 38.530 | 82.877 | 1.00 | 17.87 | B | C |
| ATOM | 4125 | CB | ILE | B | 238 | 17.394 | 38.833 | 81.729 | 1.00 | 19.74 | B | C |
| ATOM | 4126 | CG2 | ILE | B | 238 | 16.351 | 37.757 | 81.661 | 1.00 | 19.74 | B | C |
| ATOM | 4127 | CG1 | ILE | B | 238 | 16.688 | 40.160 | 81.944 | 1.00 | 19.74 | B | C |
| ATOM | 4128 | CD1 | ILE | B | 238 | 15.960 | 40.607 | 80.717 | 1.00 | 19.74 | B | C |
| ATOM | 4129 | C | ILE | B | 238 | 19.172 | 39.793 | 83.222 | 1.00 | 17.87 | B | C |
| ATOM | 4130 | O | ILE | B | 238 | 20.202 | 40.080 | 82.602 | 1.00 | 17.87 | B | O |
| ATOM | 4131 | N | ASP | B | 239 | 18.688 | 40.545 | 84.206 | 1.00 | 17.36 | B | N |
| ATOM | 4132 | CA | ASP | B | 239 | 19.382 | 41.754 | 84.628 | 1.00 | 17.36 | B | C |
| ATOM | 4133 | CB | ASP | B | 239 | 18.484 | 42.654 | 85.479 | 1.00 | 17.79 | B | C |
| ATOM | 4134 | CG | ASP | B | 239 | 17.501 | 43.456 | 84.644 | 1.00 | 17.79 | B | C |
| ATOM | 4135 | OD1 | ASP | B | 239 | 17.770 | 43.686 | 83.446 | 1.00 | 17.79 | B | O |
| ATOM | 4136 | OD2 | ASP | B | 239 | 16.465 | 43.873 | 85.197 | 1.00 | 17.79 | B | O |
| ATOM | 4137 | C | ASP | B | 239 | 20.621 | 41.386 | 85.418 | 1.00 | 17.36 | B | C |
| ATOM | 4138 | O | ASP | B | 239 | 21.615 | 42.097 | 85.380 | 1.00 | 17.36 | B | O |
| ATOM | 4139 | N | VAL | B | 240 | 20.553 | 40.263 | 86.121 | 1.00 | 23.77 | B | N |
| ATOM | 4140 | CA | VAL | B | 240 | 21.667 | 39.772 | 86.922 | 1.00 | 23.77 | B | C |
| ATOM | 4141 | CB | VAL | B | 240 | 21.225 | 38.584 | 87.803 | 1.00 | 20.93 | B | C |
| ATOM | 4142 | CG1 | VAL | B | 240 | 22.434 | 37.909 | 88.424 | 1.00 | 20.93 | B | C |
| ATOM | 4143 | CG2 | VAL | B | 240 | 20.286 | 39.078 | 88.896 | 1.00 | 20.93 | B | C |
| ATOM | 4144 | C | VAL | B | 240 | 22.813 | 39.329 | 86.012 | 1.00 | 23.77 | B | C |
| ATOM | 4145 | O | VAL | B | 240 | 23.985 | 39.523 | 86.331 | 1.00 | 23.77 | B | O |
| ATOM | 4146 | N | TRP | B | 241 | 22.465 | 38.722 | 84.882 | 1.00 | 18.13 | B | N |
| ATOM | 4147 | CA | TRP | B | 241 | 23.463 | 38.268 | 83.929 | 1.00 | 18.13 | B | C |
| ATOM | 4148 | CB | TRP | B | 241 | 22.810 | 37.384 | 82.846 | 1.00 | 23.70 | B | C |
| ATOM | 4149 | CG | TRP | B | 241 | 23.736 | 37.018 | 81.672 | 1.00 | 23.70 | B | C |
| ATOM | 4150 | CD2 | TRP | B | 241 | 24.463 | 35.790 | 81.499 | 1.00 | 23.70 | B | C |
| ATOM | 4151 | CE2 | TRP | B | 241 | 25.223 | 35.920 | 80.311 | 1.00 | 23.70 | B | C |
| ATOM | 4152 | CE3 | TRP | B | 241 | 24.555 | 34.601 | 82.236 | 1.00 | 23.70 | B | C |

| | | | | | | | | | | | | |
|---|---|---|---|---|---|---|---|---|---|---|---|---|
| ATOM | 4153 | CD1 | TRP | B | 241 | 24.073 | 37.808 | 80.601 | 1.00 | 23.70 | B | C |
| ATOM | 4154 | NE1 | TRP | B | 241 | 24.962 | 37.154 | 79.787 | 1.00 | 23.70 | B | N |
| ATOM | 4155 | CZ2 | TRP | B | 241 | 26.053 | 34.897 | 79.842 | 1.00 | 23.70 | B | C |
| ATOM | 4156 | CZ3 | TRP | B | 241 | 25.382 | 33.587 | 81.766 | 1.00 | 23.70 | B | C |
| ATOM | 4157 | CH2 | TRP | B | 241 | 26.124 | 33.745 | 80.582 | 1.00 | 23.70 | B | C |
| ATOM | 4158 | C | TRP | B | 241 | 24.104 | 39.513 | 83.312 | 1.00 | 18.13 | B | C |
| ATOM | 4159 | O | TRP | B | 241 | 25.328 | 39.625 | 83.224 | 1.00 | 18.13 | B | O |
| ATOM | 4160 | N | SER | B | 242 | 23.267 | 40.454 | 82.894 | 1.00 | 14.88 | B | N |
| ATOM | 4161 | CA | SER | B | 242 | 23.767 | 41.687 | 82.299 | 1.00 | 14.88 | B | C |
| ATOM | 4162 | CB | SER | B | 242 | 22.609 | 42.630 | 81.952 | 1.00 | 25.53 | B | C |
| ATOM | 4163 | OG | SER | B | 242 | 21.744 | 42.045 | 80.996 | 1.00 | 25.53 | B | O |
| ATOM | 4164 | C | SER | B | 242 | 24.681 | 42.366 | 83.303 | 1.00 | 14.88 | B | C |
| ATOM | 4165 | O | SER | B | 242 | 25.759 | 42.836 | 82.966 | 1.00 | 14.88 | B | O |
| ATOM | 4166 | N | ALA | B | 243 | 24.235 | 42.419 | 84.548 | 1.00 | 26.41 | B | N |
| ATOM | 4167 | CA | ALA | B | 243 | 25.040 | 43.015 | 85.601 | 1.00 | 26.41 | B | C |
| ATOM | 4168 | CB | ALA | B | 243 | 24.352 | 42.837 | 86.932 | 1.00 | 7.85 | B | C |
| ATOM | 4169 | C | ALA | B | 243 | 26.407 | 42.337 | 85.628 | 1.00 | 26.41 | B | C |
| ATOM | 4170 | O | ALA | B | 243 | 27.442 | 43.002 | 85.598 | 1.00 | 26.41 | B | O |
| ATOM | 4171 | N | GLY | B | 244 | 26.399 | 41.010 | 85.676 | 1.00 | 21.74 | B | N |
| ATOM | 4172 | CA | GLY | B | 244 | 27.633 | 40.251 | 85.703 | 1.00 | 21.74 | B | C |
| ATOM | 4173 | C | GLY | B | 244 | 28.551 | 40.524 | 84.531 | 1.00 | 21.74 | B | C |
| ATOM | 4174 | O | GLY | B | 244 | 29.771 | 40.489 | 84.691 | 1.00 | 21.74 | B | O |
| ATOM | 4175 | N | CYS | B | 245 | 27.988 | 40.779 | 83.351 | 1.00 | 29.73 | B | N |
| ATOM | 4176 | CA | CYS | B | 245 | 28.822 | 41.077 | 82.191 | 1.00 | 29.73 | B | C |
| ATOM | 4177 | CB | CYS | B | 245 | 28.006 | 41.096 | 80.893 | 1.00 | 22.96 | B | C |
| ATOM | 4178 | SG | CYS | B | 245 | 27.324 | 39.543 | 80.330 | 1.00 | 22.96 | B | S |
| ATOM | 4179 | C | CYS | B | 245 | 29.495 | 42.443 | 82.356 | 1.00 | 29.73 | B | C |
| ATOM | 4180 | O | CYS | B | 245 | 30.484 | 42.740 | 81.690 | 1.00 | 29.73 | B | O |
| ATOM | 4181 | N | VAL | B | 246 | 28.946 | 43.289 | 83.218 | 1.00 | 24.92 | B | N |
| ATOM | 4182 | CA | VAL | B | 246 | 29.547 | 44.591 | 83.441 | 1.00 | 24.92 | B | C |
| ATOM | 4183 | CB | VAL | B | 246 | 28.527 | 45.585 | 84.025 | 1.00 | 14.74 | B | C |
| ATOM | 4184 | CG1 | VAL | B | 246 | 29.191 | 46.932 | 84.299 | 1.00 | 14.74 | B | C |
| ATOM | 4185 | CG2 | VAL | B | 246 | 27.384 | 45.761 | 83.042 | 1.00 | 14.74 | B | C |
| ATOM | 4186 | C | VAL | B | 246 | 30.698 | 44.373 | 84.410 | 1.00 | 24.92 | B | C |
| ATOM | 4187 | O | VAL | B | 246 | 31.794 | 44.872 | 84.203 | 1.00 | 24.92 | B | O |
| ATOM | 4188 | N | LEU | B | 247 | 30.448 | 43.598 | 85.457 | 1.00 | 22.14 | B | N |
| ATOM | 4189 | CA | LEU | B | 247 | 31.473 | 43.313 | 86.454 | 1.00 | 22.14 | B | C |
| ATOM | 4190 | CB | LEU | B | 247 | 30.940 | 42.308 | 87.493 | 1.00 | 15.79 | B | C |
| ATOM | 4191 | CG | LEU | B | 247 | 31.610 | 42.098 | 88.857 | 1.00 | 15.79 | B | C |
| ATOM | 4192 | CD1 | LEU | B | 247 | 31.498 | 40.633 | 89.208 | 1.00 | 15.79 | B | C |
| ATOM | 4193 | CD2 | LEU | B | 247 | 33.064 | 42.497 | 88.841 | 1.00 | 15.79 | B | C |
| ATOM | 4194 | C | LEU | B | 247 | 32.686 | 42.722 | 85.748 | 1.00 | 22.14 | B | C |
| ATOM | 4195 | O | LEU | B | 247 | 33.806 | 43.184 | 85.923 | 1.00 | 22.14 | B | O |
| ATOM | 4196 | N | ALA | B | 248 | 32.451 | 41.693 | 84.944 | 1.00 | 32.65 | B | N |
| ATOM | 4197 | CA | ALA | B | 248 | 33.531 | 41.047 | 84.223 | 1.00 | 32.65 | B | C |
| ATOM | 4198 | CB | ALA | B | 248 | 32.992 | 39.905 | 83.378 | 1.00 | 7.67 | B | C |
| ATOM | 4199 | C | ALA | B | 248 | 34.265 | 42.044 | 83.347 | 1.00 | 32.65 | B | C |
| ATOM | 4200 | O | ALA | B | 248 | 35.492 | 42.075 | 83.340 | 1.00 | 32.65 | B | O |
| ATOM | 4201 | N | GLU | B | 249 | 33.508 | 42.865 | 82.620 | 1.00 | 20.30 | B | N |
| ATOM | 4202 | CA | GLU | B | 249 | 34.078 | 43.863 | 81.717 | 1.00 | 20.30 | B | C |
| ATOM | 4203 | CB | GLU | B | 249 | 32.966 | 44.612 | 81.001 | 1.00 | 23.58 | B | C |
| ATOM | 4204 | CG | GLU | B | 249 | 33.426 | 45.429 | 79.815 | 1.00 | 23.58 | B | C |
| ATOM | 4205 | CD | GLU | B | 249 | 32.276 | 45.756 | 78.894 | 1.00 | 23.58 | B | C |
| ATOM | 4206 | OE1 | GLU | B | 249 | 31.467 | 44.843 | 78.638 | 1.00 | 23.58 | B | O |
| ATOM | 4207 | OE2 | GLU | B | 249 | 32.180 | 46.905 | 78.420 | 1.00 | 23.58 | B | O |
| ATOM | 4208 | C | GLU | B | 249 | 34.984 | 44.870 | 82.415 | 1.00 | 20.30 | B | C |
| ATOM | 4209 | O | GLU | B | 249 | 36.016 | 45.251 | 81.890 | 1.00 | 20.30 | B | O |
| ATOM | 4210 | N | LEU | B | 250 | 34.590 | 45.305 | 83.600 | 1.00 | 27.64 | B | N |
| ATOM | 4211 | CA | LEU | B | 250 | 35.375 | 46.263 | 84.347 | 1.00 | 27.64 | B | C |
| ATOM | 4212 | CB | LEU | B | 250 | 34.519 | 46.870 | 85.448 | 1.00 | 24.89 | B | C |
| ATOM | 4213 | CG | LEU | B | 250 | 33.524 | 47.884 | 84.899 | 1.00 | 24.89 | B | C |
| ATOM | 4214 | CD1 | LEU | B | 250 | 32.614 | 48.350 | 86.008 | 1.00 | 24.89 | B | C |
| ATOM | 4215 | CD2 | LEU | B | 250 | 34.280 | 49.058 | 84.294 | 1.00 | 24.89 | B | C |

| | | | | | | | | | | | | |
|---|---|---|---|---|---|---|---|---|---|---|---|---|
| ATOM | 4216 | C | LEU | B | 250 | 36.648 | 45.667 | 84.943 | 1.00 | 27.64 | B | C |
| ATOM | 4217 | O | LEU | B | 250 | 37.592 | 46.394 | 85.248 | 1.00 | 27.64 | B | O |
| ATOM | 4218 | N | LEU | B | 251 | 36.671 | 44.349 | 85.123 | 1.00 | 21.76 | B | N |
| ATOM | 4219 | CA | LEU | B | 251 | 37.845 | 43.683 | 85.658 | 1.00 | 21.76 | B | C |
| ATOM | 4220 | CB | LEU | B | 251 | 37.460 | 42.380 | 86.344 | 1.00 | 14.59 | B | C |
| ATOM | 4221 | CG | LEU | B | 251 | 36.527 | 42.478 | 87.541 | 1.00 | 14.59 | B | C |
| ATOM | 4222 | CD1 | LEU | B | 251 | 36.110 | 41.087 | 87.954 | 1.00 | 14.59 | B | C |
| ATOM | 4223 | CD2 | LEU | B | 251 | 37.214 | 43.203 | 88.670 | 1.00 | 14.59 | B | C |
| ATOM | 4224 | C | LEU | B | 251 | 38.780 | 43.374 | 84.501 | 1.00 | 21.76 | B | C |
| ATOM | 4225 | O | LEU | B | 251 | 39.995 | 43.532 | 84.615 | 1.00 | 21.76 | B | O |
| ATOM | 4226 | N | LEU | B | 252 | 38.209 | 42.939 | 83.381 | 1.00 | 22.32 | B | N |
| ATOM | 4227 | CA | LEU | B | 252 | 39.001 | 42.607 | 82.205 | 1.00 | 22.32 | B | C |
| ATOM | 4228 | CB | LEU | B | 252 | 38.225 | 41.674 | 81.283 | 1.00 | 27.03 | B | C |
| ATOM | 4229 | CG | LEU | B | 252 | 38.371 | 40.212 | 81.710 | 1.00 | 27.03 | B | C |
| ATOM | 4230 | CD1 | LEU | B | 252 | 37.295 | 39.380 | 81.023 | 1.00 | 27.03 | B | C |
| ATOM | 4231 | CD2 | LEU | B | 252 | 39.782 | 39.704 | 81.377 | 1.00 | 27.03 | B | C |
| ATOM | 4232 | C | LEU | B | 252 | 39.504 | 43.815 | 81.431 | 1.00 | 22.32 | B | C |
| ATOM | 4233 | O | LEU | B | 252 | 40.687 | 43.883 | 81.096 | 1.00 | 22.32 | B | O |
| ATOM | 4234 | N | GLY | B | 253 | 38.619 | 44.769 | 81.160 | 1.00 | 20.41 | B | N |
| ATOM | 4235 | CA | GLY | B | 253 | 39.017 | 45.960 | 80.428 | 1.00 | 20.41 | B | C |
| ATOM | 4236 | C | GLY | B | 253 | 38.460 | 45.938 | 79.028 | 1.00 | 20.41 | B | C |
| ATOM | 4237 | O | GLY | B | 253 | 38.801 | 46.775 | 78.196 | 1.00 | 20.41 | B | O |
| ATOM | 4238 | N | GLN | B | 254 | 37.608 | 44.949 | 78.776 | 1.00 | 45.04 | B | N |
| ATOM | 4239 | CA | GLN | B | 254 | 36.952 | 44.767 | 77.485 | 1.00 | 45.04 | B | C |
| ATOM | 4240 | CB | GLN | B | 254 | 37.926 | 44.165 | 76.462 | 1.00 | 38.17 | B | C |
| ATOM | 4241 | CG | GLN | B | 254 | 38.728 | 42.997 | 76.971 | 1.00 | 38.17 | B | C |
| ATOM | 4242 | CD | GLN | B | 254 | 39.516 | 42.321 | 75.864 | 1.00 | 38.17 | B | C |
| ATOM | 4243 | OE1 | GLN | B | 254 | 40.482 | 42.872 | 75.346 | 1.00 | 38.17 | B | O |
| ATOM | 4244 | NE2 | GLN | B | 254 | 39.097 | 41.120 | 75.491 | 1.00 | 38.17 | B | N |
| ATOM | 4245 | C | GLN | B | 254 | 35.755 | 43.849 | 77.683 | 1.00 | 45.04 | B | C |
| ATOM | 4246 | O | GLN | B | 254 | 35.643 | 43.193 | 78.708 | 1.00 | 45.04 | B | O |
| ATOM | 4247 | N | PRO | B | 255 | 34.841 | 43.790 | 76.709 | 1.00 | 27.88 | B | N. |
| ATOM | 4248 | CD | PRO | B | 255 | 34.728 | 44.590 | 75.479 | 1.00 | 17.14 | B | C |
| ATOM | 4249 | CA | PRO | B | 255 | 33.674 | 42.916 | 76.877 | 1.00 | 27.88 | B | C |
| ATOM | 4250 | CB | PRO | B | 255 | 32.891 | 43.151 | 75.584 | 1.00 | 17.14 | B | C |
| ATOM | 4251 | CG | PRO | B | 255 | 33.234 | 44.586 | 75.243 | 1.00 | 17.14 | B | C |
| ATOM | 4252 | C | PRO | B | 255 | 34.057 | 41.450 | 77.076 | 1.00 | 27.88 | B | C |
| ATOM | 4253 | O | PRO | B | 255 | 34.969 | 40.960 | 76.432 | 1.00 | 27.88 | B | O |
| ATOM | 4254 | N | ILE | B | 256 | 33.352 | 40.755 | 77.962 | 1.00 | 19.18 | B | N |
| ATOM | 4255 | CA | ILE | B | 256 | 33.630 | 39.351 | 78.231 | 1.00 | 19.18 | B | C |
| ATOM | 4256 | CB | ILE | B | 256 | 33.141 | 38.954 | 79.644 | 1.00 | 28.68 | B | C |
| ATOM | 4257 | CG2 | ILE | B | 256 | 31.705 | 39.361 | 79.832 | 1.00 | 28.68 | B | C |
| ATOM | 4258 | CG1 | ILE | B | 256 | 33.282 | 37.448 | 79.855 | 1.00 | 28.68 | B | C |
| ATOM | 4259 | CD1 | ILE | B | 256 | 34.689 | 36.971 | 79.856 | 1.00 | 28.68 | B | C |
| ATOM | 4260 | C | ILE | B | 256 | 33.033 | 38.394 | 77.208 | 1.00 | 19.18 | B | C |
| ATOM | 4261 | O | ILE | B | 256 | 33.678 | 37.411 | 76.848 | 1.00 | 19.18 | B | O |
| ATOM | 4262 | N | PHE | B | 257 | 31.812 | 38.672 | 76.746 | 1.00 | 23.68 | B | N |
| ATOM | 4263 | CA | PHE | B | 257 | 31.137 | 37.821 | 75.753 | 1.00 | 23.68 | B | C |
| ATOM | 4264 | CB | PHE | B | 257 | 29.858 | 37.233 | 76.344 | 1.00 | 33.17 | B | C |
| ATOM | 4265 | CG | PHE | B | 257 | 30.073 | 36.445 | 77.596 | 1.00 | 33.17 | B | C |
| ATOM | 4266 | CD1 | PHE | B | 257 | 31.064 | 35.468 | 77.661 | 1.00 | 33.17 | B | C |
| ATOM | 4267 | CD2 | PHE | B | 257 | 29.279 | 36.669 | 78.709 | 1.00 | 33.17 | B | C |
| ATOM | 4268 | CE1 | PHE | B | 257 | 31.255 | 34.722 | 78.818 | 1.00 | 33.17 | B | C |
| ATOM | 4269 | CE2 | PHE | B | 257 | 29.456 | 35.935 | 79.867 | 1.00 | 33.17 | B | C |
| ATOM | 4270 | CZ | PHE | B | 257 | 30.450 | 34.957 | 79.924 | 1.00 | 33.17 | B | C |
| ATOM | 4271 | C | PHE | B | 257 | 30.778 | 38.584 | 74.470 | 1.00 | 23.68 | B | C |
| ATOM | 4272 | O | PHE | B | 257 | 29.601 | 38.737 | 74.145 | 1.00 | 23.68 | B | O |
| ATOM | 4273 | N | PRO | B | 258 | 31.785 | 39.040 | 73.706 | 1.00 | 33.74 | B | N |
| ATOM | 4274 | CD | PRO | B | 258 | 33.241 | 38.858 | 73.876 | 1.00 | 18.69 | B | C |
| ATOM | 4275 | CA | PRO | B | 258 | 31.500 | 39.784 | 72.473 | 1.00 | 33.74 | B | C |
| ATOM | 4276 | CB | PRO | B | 258 | 32.831 | 40.455 | 72.176 | 1.00 | 18.69 | B | C |
| ATOM | 4277 | CG | PRO | B | 258 | 33.798 | 39.366 | 72.545 | 1.00 | 18.69 | B | C |
| ATOM | 4278 | C | PRO | B | 258 | 31.038 | 38.919 | 71.314 | 1.00 | 33.74 | B | C |

| | | | | | | | | | | | | |
|---|---|---|---|---|---|---|---|---|---|---|---|---|
| ATOM | 4279 | O | PRO | B | 258 | 31.107 | 37.684 | 71.375 | 1.00 | 33.74 | B | O |
| ATOM | 4280 | N | GLY | B | 259 | 30.588 | 39.587 | 70.255 | 1.00 | 29.83 | B | N |
| ATOM | 4281 | CA | GLY | B | 259 | 30.108 | 38.893 | 69.068 | 1.00 | 29.83 | B | C |
| ATOM | 4282 | C | GLY | B | 259 | 28.936 | 39.617 | 68.438 | 1.00 | 29.83 | B | C |
| ATOM | 4283 | O | GLY | B | 259 | 28.042 | 40.081 | 69.147 | 1.00 | 29.83 | B | O |
| ATOM | 4284 | N | ASP | B | 260 | 28.934 | 39.721 | 67.113 | 1.00 | 21.48 | B | N |
| ATOM | 4285 | CA | ASP | B | 260 | 27.854 | 40.410 | 66.418 | 1.00 | 21.48 | B | C |
| ATOM | 4286 | CB | ASP | B | 260 | 28.354 | 41.000 | 65.103 | 1.00 | 53.56 | B | C |
| ATOM | 4287 | CG | ASP | B | 260 | 29.434 | 42.035 | 65.316 | 1.00 | 53.56 | B | C |
| ATOM | 4288 | OD1 | ASP | B | 260 | 29.199 | 42.972 | 66.121 | 1.00 | 53.56 | B | O |
| ATOM | 4289 | OD2 | ASP | B | 260 | 30.519 | 41.914 | 64.683 | 1.00 | 53.56 | B | O |
| ATOM | 4290 | C | ASP | B | 260 | 26.670 | 39.506 | 66.153 | 1.00 | 21.48 | B | C |
| ATOM | 4291 | O | ASP | B | 260 | 25.625 | 39.955 | 65.701 | 1.00 | 21.48 | B | O |
| ATOM | 4292 | N | SER | B | 261 | 26.831 | 38.224 | 66.427 | 1.00 | 22.48 | B | N |
| ATOM | 4293 | CA | SER | B | 261 | 25.742 | 37.304 | 66.206 | 1.00 | 22.48 | B | C |
| ATOM | 4294 | CB | SER | B | 261 | 26.055 | 36.372 | 65.047 | 1.00 | 16.57 | B | C |
| ATOM | 4295 | OG | SER | B | 261 | 26.806 | 35.269 | 65.499 | 1.00 | 16.57 | B | O |
| ATOM | 4296 | C | SER | B | 261 | 25.545 | 36.494 | 67.469 | 1.00 | 22.48 | B | C |
| ATOM | 4297 | O | SER | B | 261 | 26.478 | 36.325 | 68.248 | 1.00 | 22.48 | B | O |
| ATOM | 4298 | N | GLY | B | 262 | 24.323 | 36.008 | 67.669 | 1.00 | 32.89 | B | N |
| ATOM | 4299 | CA | GLY | B | 262 | 24.035 | 35.205 | 68.840 | 1.00 | 32.89 | B | C |
| ATOM | 4300 | C | GLY | B | 262 | 24.973 | 34.023 | 68.852 | 1.00 | 32.89 | B | C |
| ATOM | 4301 | O | GLY | B | 262 | 25.506 | 33.642 | 69.898 | 1.00 | 32.89 | B | O |
| ATOM | 4302 | N | VAL | B | 263 | 25.169 | 33.439 | 67.677 | 1.00 | 30.51 | B | N |
| ATOM | 4303 | CA | VAL | B | 263 | 26.064 | 32.310 | 67.548 | 1.00 | 30.51 | B | C |
| ATOM | 4304 | CB | VAL | B | 263 | 26.204 | 31.867 | 66.076 | 1.00 | 37.28 | B | C |
| ATOM | 4305 | CG1 | VAL | B | 263 | 27.000 | 30.583 | 65.993 | 1.00 | 37.28 | B | C |
| ATOM | 4306 | CG2 | VAL | B | 263 | 24.840 | 31.666 | 65.471 | 1.00 | 37.28 | B | C |
| ATOM | 4307 | C | VAL | B | 263 | 27.426 | 32.738 | 68.095 | 1.00 | 30.51 | B | C |
| ATOM | 4308 | O | VAL | B | 263 | 27.922 | 32.144 | 69.042 | 1.00 | 30.51 | B | O |
| ATOM | 4309 | N | ASP | B | 264 | 28.027 | 33.774 | 67.519 | 1.00 | 28.22 | B | N |
| ATOM | 4310 | CA | ASP | B | 264 | 29.328 | 34.228 | 68.006 | 1.00 | 28.22 | B | C |
| ATOM | 4311 | CB | ASP | B | 264 | 29.744 | 35.537 | 67.327 | 1.00 | 35.54 | B | C |
| ATOM | 4312 | CG | ASP | B | 264 | 30.209 | 35.342 | 65.901 | 1.00 | 35.54 | B | C |
| ATOM | 4313 | OD1 | ASP | B | 264 | 30.443 | 34.183 | 65.513 | 1.00 | 35.54 | B | O |
| ATOM | 4314 | OD2 | ASP | B | 264 | 30.353 | 36.349 | 65.177 | 1.00 | 35.54 | B | O |
| ATOM | 4315 | C | ASP | B | 264 | 29.334 | 34.444 | 69.520 | 1.00 | 28.22 | B | C |
| ATOM | 4316 | O | ASP | B | 264 | 30.258 | 34.025 | 70.208 | 1.00 | 28.22 | B | O |
| ATOM | 4317 | N | GLN | B | 265 | 28.305 | 35.116 | 70.025 | 1.00 | 25.46 | B | N |
| ATOM | 4318 | CA | GLN | B | 265 | 28.176 | 35.393 | 71.452 | 1.00 | 25.46 | B | C |
| ATOM | 4319 | CB | GLN | B | 265 | 26.900 | 36.197 | 71.732 | 1.00 | 20.33 | B | C |
| ATOM | 4320 | CG | GLN | B | 265 | 26.926 | 37.607 | 71.184 | 1.00 | 20.33 | B | C |
| ATOM | 4321 | CD | GLN | B | 265 | 25.556 | 38.233 | 71.141 | 1.00 | 20.33 | B | C |
| ATOM | 4322 | OE1 | GLN | B | 265 | 25.389 | 39.335 | 70.625 | 1.00 | 20.33 | B | O |
| ATOM | 4323 | NE2 | GLN | B | 265 | 24.562 | 37.535 | 71.678 | 1.00 | 20.33 | B | N |
| ATOM | 4324 | C | GLN | B | 265 | 28.128 | 34.108 | 72.236 | 1.00 | 25.46 | B | C |
| ATOM | 4325 | O | GLN | B | 265 | 28.774 | 33.978 | 73.276 | 1.00 | 25.46 | B | O |
| ATOM | 4326 | N | LEU | B | 266 | 27.348 | 33.164 | 71.725 | 1.00 | 19.47 | B | N |
| ATOM | 4327 | CA | LEU | B | 266 | 27.183 | 31.877 | 72.364 | 1.00 | 19.47 | B | C |
| ATOM | 4328 | CB | LEU | B | 266 | 26.117 | 31.080 | 71.622 | 1.00 | 18.40 | B | C |
| ATOM | 4329 | CG | LEU | B | 266 | 24.928 | 30.552 | 72.421 | 1.00 | 18.40 | B | C |
| ATOM | 4330 | CD1 | LEU | B | 266 | 24.223 | 31.678 | 73.124 | 1.00 | 18.40 | B | C |
| ATOM | 4331 | CD2 | LEU | B | 266 | 23.965 | 29.854 | 71.484 | 1.00 | 18.40 | B | C |
| ATOM | 4332 | C | LEU | B | 266 | 28.486 | 31.101 | 72.440 | 1.00 | 19.47 | B | C |
| ATOM | 4333 | O | LEU | B | 266 | 28.720 | 30.408 | 73.414 | 1.00 | 19.47 | B | O |
| ATOM | 4334 | N | VAL | B | 267 | 29.351 | 31.224 | 71.439 | 1.00 | 20.40 | B | N |
| ATOM | 4335 | CA | VAL | B | 267 | 30.617 | 30.486 | 71.478 | 1.00 | 20.40 | B | C |
| ATOM | 4336 | CB | VAL | B | 267 | 31.295 | 30.383 | 70.043 | 1.00 | 35.08 | B | C |
| ATOM | 4337 | CG1 | VAL | B | 267 | 30.465 | 31.122 | 69.008 | 1.00 | 35.08 | B | C |
| ATOM | 4338 | CG2 | VAL | B | 267 | 32.745 | 30.909 | 70.072 | 1.00 | 35.08 | B | C |
| ATOM | 4339 | C | VAL | B | 267 | 31.576 | 31.078 | 72.511 | 1.00 | 20.40 | B | C |
| ATOM | 4340 | O | VAL | B | 267 | 32.318 | 30.355 | 73.176 | 1.00 | 20.40 | B | O |
| ATOM | 4341 | N | GLU | B | 268 | 31.535 | 32.396 | 72.655 | 1.00 | 30.36 | B | N |

| | | | | | | | | | | | | |
|---|---|---|---|---|---|---|---|---|---|---|---|---|
| ATOM | 4342 | CA | GLU | B | 268 | 32.377 | 33.084 | 73.616 | 1.00 | 30.36 | B | C |
| ATOM | 4343 | CB | GLU | B | 268 | 32.236 | 34.585 | 73.408 | 1.00 | 47.21 | B | C |
| ATOM | 4344 | CG | GLU | B | 268 | 33.559 | 35.324 | 73.456 | 1.00 | 47.21 | B | C |
| ATOM | 4345 | CD | GLU | B | 268 | 34.483 | 34.965 | 72.311 | 1.00 | 47.21 | B | C |
| ATOM | 4346 | OE1 | GLU | B | 268 | 34.094 | 35.173 | 71.142 | 1.00 | 47.21 | B | O |
| ATOM | 4347 | OE2 | GLU | B | 268 | 35.601 | 34.481 | 72.578 | 1.00 | 47.21 | B | O |
| ATOM | 4348 | C | GLU | B | 268 | 31.957 | 32.680 | 75.036 | 1.00 | 30.36 | B | C |
| ATOM | 4349 | O | GLU | B | 268 | 32.802 | 32.449 | 75.907 | 1.00 | 30.36 | B | O |
| ATOM | 4350 | N | ILE | B | 269 | 30.645 | 32.583 | 75.252 | 1.00 | 33.57 | B | N |
| ATOM | 4351 | CA | ILE | B | 269 | 30.102 | 32.191 | 76.548 | 1.00 | 33.57 | B | C |
| ATOM | 4352 | CB | ILE | B | 269 | 28.562 | 32.271 | 76.559 | 1.00 | 22.05 | B | C |
| ATOM | 4353 | CG2 | ILE | B | 269 | 27.996 | 31.555 | 77.780 | 1.00 | 22.05 | B | C |
| ATOM | 4354 | CG1 | ILE | B | 269 | 28.117 | 33.723 | 76.565 | 1.00 | 22.05 | B | C |
| ATOM | 4355 | CD1 | ILE | B | 269 | 26.626 | 33.864 | 76.359 | 1.00 | 22.05 | B | C |
| ATOM | 4356 | C | ILE | B | 269 | 30.513 | 30.758 | 76.864 | 1.00 | 33.57 | B | C |
| ATOM | 4357 | O | ILE | B | 269 | 30.995 | 30.465 | 77.955 | 1.00 | 33.57 | B | O |
| ATOM | 4358 | N | ILE | B | 270 | 30.313 | 29.863 | 75.907 | 1.00 | 35.14 | B | N |
| ATOM | 4359 | CA | ILE | B | 270 | 30.667 | 28.467 | 76.108 | 1.00 | 35.14 | B | C |
| ATOM | 4360 | CB | ILE | B | 270 | 30.283 | 27.635 | 74.869 | 1.00 | 22.39 | B | C |
| ATOM | 4361 | CG2 | ILE | B | 270 | 30.874 | 26.240 | 74.962 | 1.00 | 22.39 | B | C |
| ATOM | 4362 | CG1 | ILE | B | 270 | 28.756 | 27.563 | 74.772 | 1.00 | 22.39 | B | C |
| ATOM | 4363 | CD1 | ILE | B | 270 | 28.241 | 27.009 | 73.479 | 1.00 | 22.39 | B | C |
| ATOM | 4364 | C | ILE | B | 270 | 32.154 | 28.333 | 76.414 | 1.00 | 35.14 | B | C |
| ATOM | 4365 | O | ILE | B | 270 | 32.548 | 27.545 | 77.274 | 1.00 | 35.14 | B | O |
| ATOM | 4366 | N | LYS | B | 271 | 32.974 | 29.117 | 75.724 | 1.00 | 33.16 | B | N |
| ATOM | 4367 | CA | LYS | B | 271 | 34.409 | 29.092 | 75.961 | 1.00 | 33.16 | B | C |
| ATOM | 4368 | CB | LYS | B | 271 | 35.115 | 30.224 | 75.207 | 1.00 | 54.07 | B | C |
| ATOM | 4369 | CG | LYS | B | 271 | 35.361 | 29.985 | 73.730 | 1.00 | 54.07 | B | C |
| ATOM | 4370 | CD | LYS | B | 271 | 36.140 | 31.155 | 73.131 | 1.00 | 54.07 | B | C |
| ATOM | 4371 | CE | LYS | B | 271 | 36.232 | 31.054 | 71.607 | 1.00 | 54.07 | B | C |
| ATOM | 4372 | NZ | LYS | B | 271 | 37.012 | 32.181 | 70.988 | 1.00 | 54.07 | B | N |
| ATOM | 4373 | C | LYS | B | 271 | 34.739 | 29.231 | 77.439 | 1.00 | 33.16 | B | C |
| ATOM | 4374 | O | LYS | B | 271 | 35.710 | 28.638 | 77.905 | 1.00 | 33.16 | B | O |
| ATOM | 4375 | N | VAL | B | 272 | 33.947 | 30.005 | 78.181 | 1.00 | 32.89 | B | N |
| ATOM | 4376 | CA | VAL | B | 272 | 34.255 | 30.204 | 79.590 | 1.00 | 32.89 | B | C |
| ATOM | 4377 | CB | VAL | B | 272 | 34.339 | 31.720 | 79.955 | 1.00 | 34.59 | B | C |
| ATOM | 4378 | CG1 | VAL | B | 272 | 34.648 | 32.541 | 78.719 | 1.00 | 34.59 | B | C |
| ATOM | 4379 | CG2 | VAL | B | 272 | 33.061 | 32.183 | 80.624 | 1.00 | 34.59 | B | C |
| ATOM | 4380 | C | VAL | B | 272 | 33.342 | 29.518 | 80.590 | 1.00 | 32.89 | B | C |
| ATOM | 4381 | O | VAL | B | 272 | 33.689 | 29.427 | 81.758 | 1.00 | 32.89 | B | O |
| ATOM | 4382 | N | LEU | B | 273 | 32.176 | 29.046 | 80.169 | 1.00 | 43.03 | B | N |
| ATOM | 4383 | CA | LEU | B | 273 | 31.286 | 28.365 | 81.115 | 1.00 | 43.03 | B | C |
| ATOM | 4384 | CB | LEU | B | 273 | 29.848 | 28.893 | 81.010 | 1.00 | 41.26 | B | C |
| ATOM | 4385 | CG | LEU | B | 273 | 29.487 | 30.334 | 81.379 | 1.00 | 41.26 | B | C |
| ATOM | 4386 | CD1 | LEU | B | 273 | 27.967 | 30.488 | 81.336 | 1.00 | 41.26 | B | C |
| ATOM | 4387 | CD2 | LEU | B | 273 | 30.001 | 30.675 | 82.761 | 1.00 | 41.26 | B | C |
| ATOM | 4388 | C | LEU | B | 273 | 31.269 | 26.865 | 80.835 | 1.00 | 43.03 | B | C |
| ATOM | 4389 | O | LEU | B | 273 | 30.674 | 26.082 | 81.572 | 1.00 | 43.03 | B | O |
| ATOM | 4390 | N | GLY | B | 274 | 31.929 | 26.463 | 79.759 | 1.00 | 45.55 | B | N |
| ATOM | 4391 | CA | GLY | B | 274 | 31.925 | 25.061 | 79.402 | 1.00 | 45.55 | B | C |
| ATOM | 4392 | C | GLY | B | 274 | 30.690 | 24.810 | 78.559 | 1.00 | 45.55 | B | C |
| ATOM | 4393 | O | GLY | B | 274 | 29.863 | 25.705 | 78.360 | 1.00 | 45.55 | B | O |
| ATOM | 4394 | N | THR | B | 275 | 30.560 | 23.598 | 78.039 | 1.00 | 53.69 | B | N |
| ATOM | 4395 | CA | THR | B | 275 | 29.394 | 23.273 | 77.231 | 1.00 | 53.69 | B | C |
| ATOM | 4396 | CB | THR | B | 275 | 29.691 | 22.059 | 76.291 | 1.00 | 69.03 | B | C |
| ATOM | 4397 | OG1 | THR | B | 275 | 30.631 | 22.461 | 75.278 | 1.00 | 69.03 | B | O |
| ATOM | 4398 | CG2 | THR | B | 275 | 28.392 | 21.529 | 75.631 | 1.00 | 69.03 | B | C |
| ATOM | 4399 | C | THR | B | 275 | 28.234 | 22.972 | 78.180 | 1.00 | 53.69 | B | C |
| ATOM | 4400 | O | THR | B | 275 | 28.357 | 22.152 | 79.082 | 1.00 | 53.69 | B | O |
| ATOM | 4401 | N | PRO | B | 276 | 27.093 | 23.639 | 77.987 | 1.00 | 48.85 | B | N |
| ATOM | 4402 | CD | PRO | B | 276 | 26.727 | 24.420 | 76.797 | 1.00 | 25.57 | B | C |
| ATOM | 4403 | CA | PRO | B | 276 | 25.928 | 23.419 | 78.854 | 1.00 | 48.85 | B | C |
| ATOM | 4404 | CB | PRO | B | 276 | 24.809 | 24.203 | 78.158 | 1.00 | 25.57 | B | C |

| | | | | | | | | | | | | |
|---|---|---|---|---|---|---|---|---|---|---|---|---|
| ATOM | 4405 | CG | PRO | B | 276 | 25.528 | 25.179 | 77.280 | 1.00 | 25.57 | B | C |
| ATOM | 4406 | C | PRO | B | 276 | 25.559 | 21.937 | 78.956 | 1.00 | 48.85 | B | C |
| ATOM | 4407 | O | PRO | B | 276 | 25.763 | 21.173 | 78.008 | 1.00 | 48.85 | B | O |
| ATOM | 4408 | N | THR | B | 277 | 25.020 | 21.538 | 80.108 | 1.00 | 39.27 | B | N |
| ATOM | 4409 | CA | THR | B | 277 | 24.572 | 20.167 | 80.315 | 1.00 | 39.27 | B | C |
| ATOM | 4410 | CB | THR | B | 277 | 24.227 | 19.908 | 81.775 | 1.00 | 37.39 | B | C |
| ATOM | 4411 | OG1 | THR | B | 277 | 22.994 | 20.574 | 82.090 | 1.00 | 37.39 | B | O |
| ATOM | 4412 | CG2 | THR | B | 277 | 25.343 | 20.424 | 82.682 | 1.00 | 37.39 | B | C |
| ATOM | 4413 | C | THR | B | 277 | 23.264 | 20.150 | 79.544 | 1.00 | 39.27 | B | C |
| ATOM | 4414 | O | THR | B | 277 | 23.153 | 20.808 | 78.522 | 1.00 | 39.27 | B | O |
| ATOM | 4415 | N | ALA | B | 278 | 22.265 | 19.422 | 80.034 | 1.00 | 60.15 | B | N |
| ATOM | 4416 | CA | ALA | B | 278 | 20.965 | 19.409 | 79.354 | 1.00 | 60.15 | B | C |
| ATOM | 4417 | CB | ALA | B | 278 | 20.088 | 18.276 | 79.891 | 1.00 | 54.16 | B | C |
| ATOM | 4418 | C | ALA | B | 278 | 20.311 | 20.772 | 79.632 | 1.00 | 60.15 | B | C |
| ATOM | 4419 | O | ALA | B | 278 | 19.197 | 21.048 | 79.163 | 1.00 | 60.15 | B | O |
| ATOM | 4420 | N | GLU | B | 279 | 21.037 | 21.595 | 80.410 | 1.00 | 72.61 | B | N |
| ATOM | 4421 | CA | GLU | B | 279 | 20.648 | 22.959 | 80.810 | 1.00 | 72.61 | B | C |
| ATOM | 4422 | CB | GLU | B | 279 | 21.893 | 23.785 | 81.181 | 1.00 | 28.73 | B | C |
| ATOM | 4423 | CG | GLU | B | 279 | 22.461 | 23.548 | 82.586 | 1.00 | 28.73 | B | C |
| ATOM | 4424 | CD | GLU | B | 279 | 23.966 | 23.844 | 82.686 | 1.00 | 28.73 | B | C |
| ATOM | 4425 | OE1 | GLU | B | 279 | 24.489 | 23.961 | 83.820 | 1.00 | 28.73 | B | O |
| ATOM | 4426 | OE2 | GLU | B | 279 | 24.634 | 23.944 | 81.628 | 1.00 | 28.73 | B | O |
| ATOM | 4427 | C | GLU | B | 279 | 19.884 | 23.692 | 79.710 | 1.00 | 72.61 | B | C |
| ATOM | 4428 | O | GLU | B | 279 | 19.042 | 24.538 | 80.002 | 1.00 | 72.61 | B | O |
| ATOM | 4429 | N | GLN | B | 280 | 20.195 | 23.393 | 78.451 | 1.00 | 48.17 | B | N |
| ATOM | 4430 | CA | GLN | B | 280 | 19.479 | 24.013 | 77.344 | 1.00 | 48.17 | B | C |
| ATOM | 4431 | CB | GLN | B | 280 | 19.603 | 23.149 | 76.084 | 1.00 | 75.64 | B | C |
| ATOM | 4432 | CG | GLN | B | 280 | 20.910 | 23.340 | 75.332 | 1.00 | 75.64 | B | C |
| ATOM | 4433 | CD | GLN | B | 280 | 22.109 | 23.381 | 76.258 | 1.00 | 75.64 | B | C |
| ATOM | 4434 | OE1 | GLN | B | 280 | 22.536 | 22.352 | 76.790 | 1.00 | 75.64 | B | O |
| ATOM | 4435 | NE2 | GLN | B | 280 | 22.654 | 24.586 | 76.472 | 1.00 | 75.64 | B | N |
| ATOM | 4436 | C | GLN | B | 280 | 18.023 | 24.076 | 77.776 | 1.00 | 48.17 | B | C |
| ATOM | 4437 | O | GLN | B | 280 | 17.331 | 25.074 | 77.575 | 1.00 | 48.17 | B | O |
| ATOM | 4438 | N | ALA | B | 281 | 17.576 | 22.987 | 78.388 | 1.00 | 43.37 | B | N |
| ATOM | 4439 | CA | ALA | B | 281 | 16.219 | 22.886 | 78.883 | 1.00 | 43.37 | B | C |
| ATOM | 4440 | CB | ALA | B | 281 | 16.084 | 21.633 | 79.783 | 1.00 | 69.66 | B | C |
| ATOM | 4441 | C | ALA | B | 281 | 15.914 | 24.152 | 79.676 | 1.00 | 43.37 | B | C |
| ATOM | 4442 | O | ALA | B | 281 | 15.025 | 24.931 | 79.317 | 1.00 | 43.37 | B | O |
| ATOM | 4443 | N | ALA | B | 282 | 16.677 | 24.343 | 80.750 | 1.00 | 69.80 | B | N |
| ATOM | 4444 | CA | ALA | B | 282 | 16.536 | 25.500 | 81.634 | 1.00 | 69.80 | B | C |
| ATOM | 4445 | CB | ALA | B | 282 | 17.637 | 25.466 | 82.711 | 1.00 | 54.43 | B | C |
| ATOM | 4446 | C | ALA | B | 282 | 16.580 | 26.833 | 80.864 | 1.00 | 69.80 | B | C |
| ATOM | 4447 | O | ALA | B | 282 | 15.812 | 27.758 | 81.173 | 1.00 | 69.80 | B | O |
| ATOM | 4448 | N | GLU | B | 283 | 17.470 | 26.924 | 79.870 | 1.00 | 48.81 | B | N |
| ATOM | 4449 | CA | GLU | B | 283 | 17.605 | 28.133 | 79.049 | 1.00 | 48.81 | B | C |
| ATOM | 4450 | CB | GLU | B | 283 | 19.018 | 28.242 | 78.475 | 1.00 | 39.92 | B | C |
| ATOM | 4451 | CG | GLU | B | 283 | 20.105 | 28.298 | 79.528 | 1.00 | 39.92 | B | C |
| ATOM | 4452 | CD | GLU | B | 283 | 21.486 | 28.297 | 78.921 | 1.00 | 39.92 | B | C |
| ATOM | 4453 | OE1 | GLU | B | 283 | 21.854 | 27.294 | 78.268 | 1.00 | 39.92 | B | O |
| ATOM | 4454 | OE2 | GLU | B | 283 | 22.203 | 29.304 | 79.090 | 1.00 | 39.92 | B | O |
| ATOM | 4455 | C | GLU | B | 283 | 16.604 | 28.106 | 77.899 | 1.00 | 48.81 | B | C |
| ATOM | 4456 | O | GLU | B | 283 | 15.683 | 28.922 | 77.851 | 1.00 | 48.81 | B | O |
| ATOM | 4457 | N | TRP | B | 301 | 36.661 | 29.269 | 84.514 | 1.00 | 62.04 | B | N |
| ATOM | 4458 | CA | TRP | B | 301 | 37.086 | 30.614 | 84.905 | 1.00 | 62.04 | B | C |
| ATOM | 4459 | CB | TRP | B | 301 | 36.259 | 31.102 | 86.099 | 1.00 | 30.73 | B | C |
| ATOM | 4460 | CG | TRP | B | 301 | 34.827 | 31.431 | 85.753 | 1.00 | 30.73 | B | C |
| ATOM | 4461 | CD2 | TRP | B | 301 | 34.360 | 32.655 | 85.183 | 1.00 | 30.73 | B | C |
| ATOM | 4462 | CE2 | TRP | B | 301 | 32.967 | 32.523 | 84.990 | 1.00 | 30.73 | B | C |
| ATOM | 4463 | CE3 | TRP | B | 301 | 34.988 | 33.849 | 84.809 | 1.00 | 30.73 | B | C |
| ATOM | 4464 | CD1 | TRP | B | 301 | 33.727 | 30.623 | 85.887 | 1.00 | 30.73 | B | C |
| ATOM | 4465 | NE1 | TRP | B | 301 | 32.606 | 31.277 | 85.430 | 1.00 | 30.73 | B | N |
| ATOM | 4466 | CZ2 | TRP | B | 301 | 32.189 | 33.549 | 84.440 | 1.00 | 30.73 | B | C |
| ATOM | 4467 | CZ3 | TRP | B | 301 | 34.214 | 34.869 | 84.261 | 1.00 | 30.73 | B | C |

| | | | | | | | | | | | | |
|---|---|---|---|---|---|---|---|---|---|---|---|---|
| ATOM | 4468 | CH2 | TRP | B | 301 | 32.830 | 34.710 | 84.080 | 1.00 | 30.73 | B | C |
| ATOM | 4469 | C | TRP | B | 301 | 38.580 | 30.673 | 85.245 | 1.00 | 62.04 | B | C |
| ATOM | 4470 | O | TRP | B | 301 | 39.397 | 30.033 | 84.565 | 1.00 | 62.04 | B | O |
| ATOM | 4471 | N | THR | B | 302 | 38.919 | 31.452 | 86.279 | 1.00 | 60.34 | B | N |
| ATOM | 4472 | CA | THR | B | 302 | 40.294 | 31.645 | 86.783 | 1.00 | 60.34 | B | C |
| ATOM | 4473 | CB | THR | B | 302 | 40.616 | 30.681 | 87.972 | 1.00 | 58.11 | B | C |
| ATOM | 4474 | OG1 | THR | B | 302 | 39.410 | 30.087 | 88.462 | 1.00 | 58.11 | B | O |
| ATOM | 4475 | CG2 | THR | B | 302 | 41.257 | 31.455 | 89.125 | 1.00 | 58.11 | B | C |
| ATOM | 4476 | C | THR | B | 302 | 41.450 | 31.538 | 85.777 | 1.00 | 60.34 | B | C |
| ATOM | 4477 | O | THR | B | 302 | 42.592 | 31.255 | 86.151 | 1.00 | 60.34 | B | O |
| ATOM | 4478 | N | LYS | B | 303 | 41.160 | 31.767 | 84.504 | 1.00 | 56.89 | B | N |
| ATOM | 4479 | CA | LYS | B | 303 | 42.186 | 31.724 | 83.466 | 1.00 | 56.89 | B | C |
| ATOM | 4480 | CB | LYS | B | 303 | 42.223 | 30.346 | 82.781 | 1.00 | 71.54 | B | C |
| ATOM | 4481 | CG | LYS | B | 303 | 43.188 | 29.315 | 83.413 | 1.00 | 71.54 | B | C |
| ATOM | 4482 | CD | LYS | B | 303 | 44.396 | 29.014 | 82.488 | 1.00 | 71.54 | B | C |
| ATOM | 4483 | CE | LYS | B | 303 | 45.526 | 30.030 | 82.644 | 1.00 | 71.54 | B | C |
| ATOM | 4484 | NZ | LYS | B | 303 | 46.118 | 29.939 | 84.019 | 1.00 | 71.54 | B | N |
| ATOM | 4485 | C | LYS | B | 303 | 41.738 | 32.796 | 82.489 | 1.00 | 56.89 | B | C |
| ATOM | 4486 | O | LYS | B | 303 | 42.308 | 32.977 | 81.411 | 1.00 | 56.89 | B | O |
| ATOM | 4487 | N | VAL | B | 304 | 40.704 | 33.512 | 82.917 | 1.00 | 42.84 | B | N |
| ATOM | 4488 | CA | VAL | B | 304 | 40.085 | 34.581 | 82.157 | 1.00 | 42.84 | B | C |
| ATOM | 4489 | CB | VAL | B | 304 | 38.568 | 34.549 | 82.393 | 1.00 | 37.64 | B | C |
| ATOM | 4490 | CG1 | VAL | B | 304 | 37.909 | 35.731 | 81.717 | 1.00 | 37.64 | B | C |
| ATOM | 4491 | CG2 | VAL | B | 304 | 37.999 | 33.227 | 81.907 | 1.00 | 37.64 | B | C |
| ATOM | 4492 | C | VAL | B | 304 | 40.591 | 35.963 | 82.530 | 1.00 | 42.84 | B | C |
| ATOM | 4493 | O | VAL | B | 304 | 40.737 | 36.820 | 81.657 | 1.00 | 42.84 | B | O |
| ATOM | 4494 | N | PHE | B | 305 | 40.854 | 36.175 | 83.821 | 1.00 | 41.53 | B | N |
| ATOM | 4495 | CA | PHE | B | 305 | 41.294 | 37.486 | 84.317 | 1.00 | 41.53 | B | C |
| ATOM | 4496 | CB | PHE | B | 305 | 40.599 | 37.763 | 85.655 | 1.00 | 28.42 | B | C |
| ATOM | 4497 | CG | PHE | B | 305 | 39.109 | 37.862 | 85.529 | 1.00 | 28.42 | B | C |
| ATOM | 4498 | CD1 | PHE | B | 305 | 38.511 | 39.056 | 85.116 | 1.00 | 28.42 | B | C |
| ATOM | 4499 | CD2 | PHE | B | 305 | 38.305 | 36.742 | 85.732 | 1.00 | 28.42 | B | C |
| ATOM | 4500 | CE1 | PHE | B | 305 | 37.139 | 39.132 | 84.910 | 1.00 | 28.42 | B | C |
| ATOM | 4501 | CE2 | PHE | B | 305 | 36.928 | 36.808 | 85.526 | 1.00 | 28.42 | B | C |
| ATOM | 4502 | CZ | PHE | B | 305 | 36.346 | 38.002 | 85.112 | 1.00 | 28.42 | B | C |
| ATOM | 4503 | C | PHE | B | 305 | 42.807 | 37.705 | 84.427 | 1.00 | 41.53 | B | C |
| ATOM | 4504 | O | PHE | B | 305 | 43.590 | 36.751 | 84.399 | 1.00 | 41.53 | B | O |
| ATOM | 4505 | N | ARG | B | 306 | 43.208 | 38.972 | 84.534 | 1.00 | 34.43 | B | N |
| ATOM | 4506 | CA | ARG | B | 306 | 44.624 | 39.306 | 84.648 | 1.00 | 34.43 | B | C |
| ATOM | 4507 | CB | ARG | B | 306 | 44.831 | 40.823 | 84.701 | 1.00 | 92.00 | B | C |
| ATOM | 4508 | CG | ARG | B | 306 | 44.210 | 41.493 | 85.912 | 1.00 | 92.00 | B | C |
| ATOM | 4509 | CD | ARG | B | 306 | 45.278 | 42.174 | 86.767 | 1.00 | 92.00 | B | C |
| ATOM | 4510 | NE | ARG | B | 306 | 45.756 | 43.425 | 86.168 | 1.00 | 92.00 | B | N |
| ATOM | 4511 | CZ | ARG | B | 306 | 45.055 | 44.561 | 86.136 | 1.00 | 92.00 | B | C |
| ATOM | 4512 | NH1 | ARG | B | 306 | 43.836 | 44.611 | 86.678 | 1.00 | 92.00 | B | N |
| ATOM | 4513 | NH2 | ARG | B | 306 | 45.564 | 45.647 | 85.550 | 1.00 | 92.00 | B | N |
| ATOM | 4514 | C | ARG | B | 306 | 45.164 | 38.654 | 85.921 | 1.00 | 34.43 | B | C |
| ATOM | 4515 | O | ARG | B | 306 | 44.430 | 37.985 | 86.653 | 1.00 | 34.43 | B | O |
| ATOM | 4516 | N | PRO | B | 307 | 46.461 | 38.845 | 86:207 | 1.00 | 53.97 | B | N |
| ATOM | 4517 | CD | PRO | B | 307 | 47.513 | 39.427 | 85.348 | 1.00 | 90.25 | B | C |
| ATOM | 4518 | CA | PRO | B | 307 | 47.057 | 38.245 | 87.406 | 1.00 | 53.97 | B | C |
| ATOM | 4519 | CB | PRO | B | 307 | 48.528 | 38.655 | 87.301 | 1.00 | 90.25 | B | C |
| ATOM | 4520 | CG | PRO | B | 307 | 48.757 | 38.682 | 85.813 | 1.00 | 90.25 | B | C |
| ATOM | 4521 | C | PRO | B | 307 | 46.449 | 38.582 | 88.777 | 1.00 | 53.97 | B | C |
| ATOM | 4522 | O | PRO | B | 307 | 45.706 | 37.785 | 89.368 | 1.00 | 53.97 | B | O |
| ATOM | 4523 | N | ALA | B | 308 | 46.781 | 39.762 | 89.285 | 1.00 | 59.73 | B | N |
| ATOM | 4524 | CA | ALA | B | 308 | 46.308 | 40.194 | 90.598 | 1.00 | 59.73 | B | C |
| ATOM | 4525 | CB | ALA | B | 308 | 46.748 | 41.633 | 90.852 | 1.00 | 67.44 | B | C |
| ATOM | 4526 | C | ALA | B | 308 | 44.800 | 40.074 | 90.831 | 1.00 | 59.73 | B | C |
| ATOM | 4527 | O | ALA | B | 308 | 44.362 | 40.005 | 91.982 | 1.00 | 59.73 | B | O |
| ATOM | 4528 | N | THR | B | 309 | 44.011 | 40.066 | 89.757 | 1.00 | 42.81 | B | N |
| ATOM | 4529 | CA | THR | B | 309 | 42.556 | 39.965 | 89.879 | 1.00 | 42.81 | B | C |
| ATOM | 4530 | CB | THR | B | 309 | 41.939 | 39.197 | 88.687 | 1.00 | 51.20 | B | C |

| | | | | | | | | | | | | |
|---|---|---|---|---|---|---|---|---|---|---|---|---|
| ATOM | 4531 | OG1 | THR | B | 309 | 41.981 | 40.011 | 87.507 | 1.00 | 51.20 | B | O |
| ATOM | 4532 | CG2 | THR | B | 309 | 40.484 | 38.833 | 88.992 | 1.00 | 51.20 | B | C |
| ATOM | 4533 | C | THR | B | 309 | 42.087 | 39.274 | 91.155 | 1.00 | 42.81 | B | C |
| ATOM | 4534 | O | THR | B | 309 | 42.283 | 38.073 | 91.328 | 1.00 | 42.81 | B | O |
| ATOM | 4535 | N | PRO | B | 310 | 41.441 | 40.026 | 92.058 | 1.00 | 50.22 | B | N |
| ATOM | 4536 | CD | PRO | B | 310 | 41.084 | 41.451 | 91.952 | 1.00 | 21.85 | B | C |
| ATOM | 4537 | CA | PRO | B | 310 | 40.951 | 39.460 | 93.321 | 1.00 | 50.22 | B | C |
| ATOM | 4538 | CB | PRO | B | 310 | 40.113 | 40.592 | 93.912 | 1.00 | 21.85 | B | C |
| ATOM | 4539 | CG | PRO | B | 310 | 40.794 | 41.803 | 93.403 | 1.00 | 21.85 | B | C |
| ATOM | 4540 | C | PRO | B | 310 | 40.117 | 38.199 | 93.106 | 1.00 | 50.22 | B | C |
| ATOM | 4541 | O | PRO | B | 310 | 39.261 | 38.149 | 92.219 | 1.00 | 50.22 | B | O |
| ATOM | 4542 | N | PRO | B | 311 | 40.362 | 37.158 | 93.914 | 1.00 | 39.44 | B | N |
| ATOM | 4543 | CD | PRO | B | 311 | 41.310 | 37.040 | 95.034 | 1.00 | 29.28 | B | C |
| ATOM | 4544 | CA | PRO | B | 311 | 39.591 | 35.930 | 93.761 | 1.00 | 39.44 | B | C |
| ATOM | 4545 | CB | PRO | B | 311 | 40.234 | 34.990 | 94.779 | 1.00 | 29.28 | B | C |
| ATOM | 4546 | CG | PRO | B | 311 | 40.695 | 35.921 | 95.847 | 1.00 | 29.28 | B | C |
| ATOM | 4547 | C | PRO | B | 311 | 38.118 | 36.180 | 94.033 | 1.00 | 39.44 | B | C |
| ATOM | 4548 | O | PRO | B | 311 | 37.271 | 35.718 | 93.284 | 1.00 | 39.44 | B | O |
| ATOM | 4549 | N | GLU | B | 312 | 37.815 | 36.917 | 95.098 | 1.00 | 30.58 | B | N |
| ATOM | 4550 | CA | GLU | B | 312 | 36.426 | 37.220 | 95.447 | 1.00 | 30.58 | B | C |
| ATOM | 4551 | CB | GLU | B | 312 | 36.365 | 38.189 | 96.639 | 1.00 | 67.01 | B | C |
| ATOM | 4552 | CG | GLU | B | 312 | 37.636 | 38.994 | 96.851 | 1.00 | 67.01 | B | C |
| ATOM | 4553 | CD | GLU | B | 312 | 38.731 | 38.170 | 97.518 | 1.00 | 67.01 | B | C |
| ATOM | 4554 | OE1 | GLU | B | 312 | 39.938 | 38.506 | 97.350 | 1.00 | 67.01 | B | O |
| ATOM | 4555 | OE2 | GLU | B | 312 | 38.372 | 37.194 | 98.223 | 1.00 | 67.01 | B | O |
| ATOM | 4556 | C | GLU | B | 312 | 35.659 | 37.793 | 94.252 | 1.00 | 30.58 | B | C |
| ATOM | 4557 | O | GLU | B | 312 | 34.485 | 37.464 | 94.042 | 1.00 | 30.58 | B | O |
| ATOM | 4558 | N | ALA | B | 313 | 36.326 | 38.635 | 93.467 | 1.00 | 24.22 | B | N |
| ATOM | 4559 | CA | ALA | B | 313 | 35.698 | 39.232 | 92.300 | 1.00 | 24.22 | B | C |
| ATOM | 4560 | CB | ALA | B | 313 | 36.638 | 40.251 | 91.679 | 1.00 | 19.24 | B | C |
| ATOM | 4561 | C | ALA | B | 313 | 35.345 | 38.133 | 91.289 | 1.00 | 24.22 | B | C |
| ATOM | 4562 | O | ALA | B | 313 | 34.225 | 38.090 | 90.761 | 1.00 | 24.22 | B | O |
| ATOM | 4563 | N | ILE | B | 314 | 36.303 | 37.246 | 91.036 | 1.00 | 18.97 | B | N |
| ATOM | 4564 | CA | ILE | B | 314 | 36.115 | 36.139 | 90.105 | 1.00 | 18.97 | B | C |
| ATOM | 4565 | CB | ILE | B | 314 | 37.393 | 35.272 | 90.003 | 1.00 | 19.96 | B | C |
| ATOM | 4566 | CG2 | ILE | B | 314 | 37.164 | 34.096 | 89.080 | 1.00 | 19.96 | B | C |
| ATOM | 4567 | CG1 | ILE | B | 314 | 38.547 | 36.110 | 89.468 | 1.00 | 19.96 | B | C |
| ATOM | 4568 | CD1 | ILE | B | 314 | 39.811 | 35.334 | 89.299 | 1.00 | 19.96 | B | C |
| ATOM | 4569 | C | ILE | B | 314 | 34.964 | 35.256 | 90.600 | 1.00 | 18.97 | B | C |
| ATOM | 4570 | O | ILE | B | 314 | 34.082 | 34.850 | 89.842 | 1.00 | 18.97 | B | O |
| ATOM | 4571 | N | ALA | B | 315 | 34.983 | 34.965 | 91.890 | 1.00 | 40.39 | B | N |
| ATOM | 4572 | CA | ALA | B | 315 | 33.948 | 34.146 | 92.498 | 1.00 | 40.39 | B | C |
| ATOM | 4573 | CB | ALA | B | 315 | 34.157 | 34.113 | 93.993 | 1.00 | 27.17 | B | C |
| ATOM | 4574 | C | ALA | B | 315 | 32.540 | 34.668 | 92.184 | 1.00 | 40.39 | B | C |
| ATOM | 4575 | O | ALA | B | 315 | 31.658 | 33.909 | 91.764 | 1.00 | 40.39 | B | O |
| ATOM | 4576 | N | LEU | B | 316 | 32.344 | 35.970 | 92.404 | 1.00 | 34.20 | B | N |
| ATOM | 4577 | CA | LEU | B | 316 | 31.073 | 36.635 | 92.152 | 1.00 | 34.20 | B | C |
| ATOM | 4578 | CB | LEU | B | 316 | 31.130 | 38.080 | 92.652 | 1.00 | 21.83 | B | C |
| ATOM | 4579 | CG | LEU | B | 316 | 29.907 | 38.952 | 92.372 | 1.00 | 21.83 | B | C |
| ATOM | 4580 | CD1 | LEU | B | 316 | 28.678 | 38.333 | 92.984 | 1.00 | 21.83 | B | C |
| ATOM | 4581 | CD2 | LEU | B | 316 | 30.128 | 40.335 | 92.920 | 1.00 | 21.83 | B | C |
| ATOM | 4582 | C | LEU | B | 316 | 30.692 | 36.607 | 90.679 | 1.00 | 34.20 | B | C |
| ATOM | 4583 | O | LEU | B | 316 | 29.510 | 36.657 | 90.351 | 1.00 | 34.20 | B | O |
| ATOM | 4584 | N | CYS | B | 317 | 31.663 | 36.532 | 89.782 | 1.00 | 23.37 | B | N |
| ATOM | 4585 | CA | CYS | B | 317 | 31.285 | 36.479 | 88.376 | 1.00 | 23.37 | B | C |
| ATOM | 4586 | CB | CYS | B | 317 | 32.500 | 36.604 | 87.465 | 1.00 | 39.29 | B | C |
| ATOM | 4587 | SG | CYS | B | 317 | 32.958 | 38.291 | 87.128 | 1.00 | 39.29 | B | S |
| ATOM | 4588 | C | CYS | B | 317 | 30.608 | 35.147 | 88.104 | 1.00 | 23.37 | B | C |
| ATOM | 4589 | O | CYS | B | 317 | 29.492 | 35.078 | 87.578 | 1.00 | 23.37 | B | O |
| ATOM | 4590 | N | SER | B | 318 | 31.309 | 34.086 | 88.469 | 1.00 | 30.53 | B | N |
| ATOM | 4591 | CA | SER | B | 318 | 30.832 | 32.733 | 88.270 | 1.00 | 30.53 | B | C |
| ATOM | 4592 | CB | SER | B | 318 | 31.791 | 31.772 | 88.947 | 1.00 | 64.27 | B | C |
| ATOM | 4593 | OG | SER | B | 318 | 32.324 | 32.371 | 90.120 | 1.00 | 64.27 | B | O |

| | | | | | | | | | | | | |
|---|---|---|---|---|---|---|---|---|---|---|---|---|
| ATOM | 4594 | C | SER | B | 318 | 29.438 | 32.514 | 88.808 | 1.00 | 30.53 | B | C |
| ATOM | 4595 | O | SER | B | 318 | 28.650 | 31.780 | 88.207 | 1.00 | 30.53 | B | O |
| ATOM | 4596 | N | ARG | B | 319 | 29.137 | 33.144 | 89.942 | 1.00 | 30.82 | B | N |
| ATOM | 4597 | CA | ARG | B | 319 | 27.826 | 33.008 | 90.572 | 1.00 | 30.82 | B | C |
| ATOM | 4598 | CB | ARG | B | 319 | 27.928 | 33.291 | 92.080 | 1.00 | 34.18 | B | C |
| ATOM | 4599 | CG | ARG | B | 319 | 28.700 | 32.253 | 92.882 | 1.00 | 34.18 | B | C |
| ATOM | 4600 | CD | ARG | B | 319 | 28.306 | 30.834 | 92.492 | 1.00 | 34.18 | B | C |
| ATOM | 4601 | NE | ARG | B | 319 | 26.868 | 30.687 | 92.272 | 1.00 | 34.18 | B | N |
| ATOM | 4602 | CZ | ARG | B | 319 | 25.952 | 30.653 | 93.237 | 1.00 | 34.18 | B | C |
| ATOM | 4603 | NH1 | ARG | B | 319 | 26.314 | 30.754 | 94.511 | 1.00 | 34.18 | B | N |
| ATOM | 4604 | NH2 | ARG | B | 319 | 24.665 | 30.517 | 92.926 | 1.00 | 34.18 | B | N |
| ATOM | 4605 | C | ARG | B | 319 | 26.779 | 33.929 | 89.952 | 1.00 | 30.82 | B | C |
| ATOM | 4606 | O | ARG | B | 319 | 25.600 | 33.877 | 90.317 | 1.00 | 30.82 | B | O |
| ATOM | 4607 | N | LEU | B | 320 | 27.217 | 34.780 | 89.030 | 1.00 | 26.79 | B | N |
| ATOM | 4608 | CA | LEU | B | 320 | 26.314 | 35.697 | 88.346 | 1.00 | 26.79 | B | C |
| ATOM | 4609 | CB | LEU | B | 320 | 26.924 | 37.099 | 88.258 | 1.00 | 10.35 | B | C |
| ATOM | 4610 | CG | LEU | B | 320 | 26.935 | 37.904 | 89.549 | 1.00 | 10.35 | B | C |
| ATOM | 4611 | CD1 | LEU | B | 320 | 27.723 | 39.181 | 89.386 | 1.00 | 10.35 | B | C |
| ATOM | 4612 | CD2 | LEU | B | 320 | 25.503 | 38.192 | 89.939 | 1.00 | 10.35 | B | C |
| ATOM | 4613 | C | LEU | B | 320 | 26.053 | 35.172 | 86.944 | 1.00 | 26.79 | B | C |
| ATOM | 4614 | O | LEU | B | 320 | 24.908 | 35.075 | 86.503 | 1.00 | 26.79 | B | O |
| ATOM | 4615 | N | LEU | B | 321 | 27.130 | 34.818 | 86.252 | 1.00 | 21.49 | B | N |
| ATOM | 4616 | CA | LEU | B | 321 | 27.021 | 34.320 | 84.897 | 1.00 | 21.49 | B | C |
| ATOM | 4617 | CB | LEU | B | 321 | 28.253 | 34.745 | 84.104 | 1.00 | 15.20 | B | C |
| ATOM | 4618 | CG | LEU | B | 321 | 28.459 | 36.260 | 84.125 | 1.00 | 15.20 | B | C |
| ATOM | 4619 | CD1 | LEU | B | 321 | 29.767 | 36.615 | 83.472 | 1.00 | 15.20 | B | C |
| ATOM | 4620 | CD2 | LEU | B | 321 | 27.299 | 36.938 | 83.437 | 1.00 | 15.20 | B | C |
| ATOM | 4621 | C | LEU | B | 321 | 26.854 | 32.818 | 84.852 | 1.00 | 21.49 | B | C |
| ATOM | 4622 | O | LEU | B | 321 | 27.758 | 32.105 | 84.437 | 1.00 | 21.49 | B | O |
| ATOM | 4623 | N | GLU | B | 322 | 25.680 | 32.354 | 85.277 | 1.00 | 35.41 | B | N |
| ATOM | 4624 | CA | GLU | B | 322 | 25.341 | 30.925 | 85.309 | 1.00 | 35.41 | B | C |
| ATOM | 4625 | CB | GLU | B | 322 | 24.807 | 30.544 | 86.701 | 1.00 | 55.23 | B | C |
| ATOM | 4626 | CG | GLU | B | 322 | 25.412 | 29.258 | 87.294 | 1.00 | 55.23 | B | C |
| ATOM | 4627 | CD | GLU | B | 322 | 26.294 | 29.513 | 88.531 | 1.00 | 55.23 | B | C |
| ATOM | 4628 | OE1 | GLU | B | 322 | 25.809 | 30.132 | 89.517 | 1.00 | 55.23 | B | O |
| ATOM | 4629 | OE2 | GLU | B | 322 | 27.473 | 29.083 | 88.526 | 1.00 | 55.23 | B | O |
| ATOM | 4630 | C | GLU | B | 322 | 24.282 | 30.622 | 84.255 | 1.00 | 35.41 | B | C |
| ATOM | 4631 | O | GLU | B | 322 | 23.384 | 31.432 | 84.043 | 1.00 | 35.41 | B | O |
| ATOM | 4632 | N | TYR | B | 323 | 24.390 | 29.464 | 83.597 | 1.00 | 25.83 | B | N |
| ATOM | 4633 | CA | TYR | B | 323 | 23.424 | 29.064 | 82.565 | 1.00 | 25.83 | B | C |
| ATOM | 4634 | CB | TYR | B | 323 | 23.804 | 27.702 | 81.973 | 1.00 | 22.98 | B | C |
| ATOM | 4635 | CG | TYR | B | 323 | 24.925 | 27.730 | 80.953 | 1.00 | 22.98 | B | C |
| ATOM | 4636 | CD1 | TYR | B | 323 | 24.768 | 28.368 | 79.722 | 1.00 | 22.98 | B | C |
| ATOM | 4637 | CE1 | TYR | B | 323 | 25.774 | 28.341 | 78.761 | 1.00 | 22.98 | B | C |
| ATOM | 4638 | CD2 | TYR | B | 323 | 26.129 | 27.071 | 81.198 | 1.00 | 22.98 | B | C |
| ATOM | 4639 | CE2 | TYR | B | 323 | 27.146 | 27.041 | 80.239 | 1.00 | 22.98 | B | C |
| ATOM | 4640 | CZ | TYR | B | 323 | 26.958 | 27.673 | 79.027 | 1.00 | 22.98 | B | C |
| ATOM | 4641 | OH | TYR | B | 323 | 27.946 | 27.608 | 78.078 | 1.00 | 22.98 | B | O |
| ATOM | 4642 | C | TYR | B | 323 | 21.986 | 29.012 | 83.106 | 1.00 | 25.83 | B | C |
| ATOM | 4643 | O | TYR | B | 323 | 21.095 | 29.676 | 82.583 | 1.00 | 25.83 | B | O |
| ATOM | 4644 | N | THR | B | 324 | 21.745 | 28.227 | 84.147 | 1.00 | 29.80 | B | N |
| ATOM | 4645 | CA | THR | B | 324 | 20.399 | 28.174 | 84.692 | 1.00 | 29.80 | B | C |
| ATOM | 4646 | CB | THR | B | 324 | 20.240 | 27.084 | 85.757 | 1.00 | 44.00 | B | C |
| ATOM | 4647 | OG1 | THR | B | 324 | 20.390 | 25.796 | 85.150 | 1.00 | 44.00 | B | O |
| ATOM | 4648 | CG2 | THR | B | 324 | 18.861 | 27.183 | 86.409 | 1.00 | 44.00 | B | C |
| ATOM | 4649 | C | THR | B | 324 | 20.057 | 29.512 | 85.331 | 1.00 | 29.80 | B | C |
| ATOM | 4650 | O | THR | B | 324 | 20.684 | 29.936 | 86.300 | 1.00 | 29.80 | B | O |
| ATOM | 4651 | N | PRO | B | 325 | 19.043 | 30.195 | 84.795 | 1.00 | 40.32 | B | N |
| ATOM | 4652 | CD | PRO | B | 325 | 18.201 | 29.794 | 83.653 | 1.00 | 43.14 | B | C |
| ATOM | 4653 | CA | PRO | B | 325 | 18.635 | 31.493 | 85.329 | 1.00 | 40.32 | B | C |
| ATOM | 4654 | CB | PRO | B | 325 | 17.387 | 31.827 | 84.512 | 1.00 | 43.14 | B | C |
| ATOM | 4655 | CG | PRO | B | 325 | 17.615 | 31.122 | 83.210 | 1.00 | 43.14 | B | C |
| ATOM | 4656 | C | PRO | B | 325 | 18.338 | 31.423 | 86.819 | 1.00 | 40.32 | B | C |

| | | | | | | | | | | | | |
|---|---|---|---|---|---|---|---|---|---|---|---|---|
| ATOM | 4657 | O | PRO | B | 325 | 18.670 | 32.344 | 87.567 | 1.00 | 40.32 | B | O |
| ATOM | 4658 | N | THR | B | 326 | 17.727 | 30.327 | 87.262 | 1.00 | 30.35 | B | N |
| ATOM | 4659 | CA | THR | B | 326 | 17.391 | 30.191 | 88.678 | 1.00 | 30.35 | B | C |
| ATOM | 4660 | CB | THR | B | 326 | 16.453 | 29.010 | 88.959 | 1.00 | 21.05 | B | C |
| ATOM | 4661 | OG1 | THR | B | 326 | 17.209 | 27.790 | 88.973 | 1.00 | 21.05 | B | O |
| ATOM | 4662 | CG2 | THR | B | 326 | 15.378 | 28.918 | 87.906 | 1.00 | 21.05 | B | C |
| ATOM | 4663 | C | THR | B | 326 | 18.591 | 29.971 | 89.582 | 1.00 | 30.35 | B | C |
| ATOM | 4664 | O | THR | B | 326 | 18.465 | 30.047 | 90.797 | 1.00 | 30.35 | B | O |
| ATOM | 4665 | N | ALA | B | 327 | 19.751 | 29.684 | 89.010 | 1.00 | 18.44 | B | N |
| ATOM | 4666 | CA | ALA | B | 327 | 20.924 | 29.416 | 89.824 | 1.00 | 18.44 | B | C |
| ATOM | 4667 | CB | ALA | B | 327 | 21.729 | 28.306 | 89.212 | 1.00 | 15.66 | B | C |
| ATOM | 4668 | C | ALA | B | 327 | 21.799 | 30.627 | 90.004 | 1.00 | 18.44 | B | C |
| ATOM | 4669 | O | ALA | B | 327 | 22.827 | 30.559 | 90.672 | 1.00 | 18.44 | B | O |
| ATOM | 4670 | N | ARG | B | 328 | 21.397 | 31.737 | 89.405 | 1.00 | 27.31 | B | N |
| ATOM | 4671 | CA | ARG | B | 328 | 22.186 | 32.957 | 89.505 | 1.00 | 27.31 | B | C |
| ATOM | 4672 | CB | ARG | B | 328 | 21.853 | 33.907 | 88.353 | 1.00 | 11.21 | B | C |
| ATOM | 4673 | CG | ARG | B | 328 | 22.018 | 33.282 | 86.991 | 1.00 | 11.21 | B | C |
| ATOM | 4674 | CD | ARG | B | 328 | 21.396 | 34.102 | 85.886 | 1.00 | 11.21 | B | C |
| ATOM | 4675 | NE | ARG | B | 328 | 21.451 | 33.344 | 84.646 | 1.00 | 11.21 | B | N |
| ATOM | 4676 | CZ | ARG | B | 328 | 20.734 | 33.604 | 83.565 | 1.00 | 11.21 | B | C |
| ATOM | 4677 | NH1 | ARG | B | 328 | 19.890 | 34.620 | 83.543 | 1.00 | 11.21 | B | N |
| ATOM | 4678 | NH2 | ARG | B | 328 | 20.849 | 32.821 | 82.513 | 1.00 | 11.21 | B | N |
| ATOM | 4679 | C | ARG | B | 328 | 21.854 | 33.641 | 90.806 | 1.00 | 27.31 | B | C |
| ATOM | 4680 | O | ARG | B | 328 | 20.741 | 33.514 | 91.310 | 1.00 | 27.31 | B | O |
| ATOM | 4681 | N | LEU | B | 329 | 22.821 | 34.363 | 91.350 | 1.00 | 21.75 | B | N |
| ATOM | 4682 | CA | LEU | B | 329 | 22.599 | 35.088 | 92.585 | 1.00 | 21.75 | B | C |
| ATOM | 4683 | CB | LEU | B | 329 | 23.847 | 35.866 | 92.966 | 1.00 | 25.64 | B | C |
| ATOM | 4684 | CG | LEU | B | 329 | 24.865 | 35.236 | 93.913 | 1.00 | 25.64 | B | C |
| ATOM | 4685 | CD1 | LEU | B | 329 | 24.984 | 33.743 | 93.692 | 1.00 | 25.64 | B | C |
| ATOM | 4686 | CD2 | LEU | B | 329 | 26.197 | 35.941 | 93.686 | 1.00 | 25.64 | B | C |
| ATOM | 4687 | C | LEU | B | 329 | 21.460 | 36.064 | 92.384 | 1.00 | 21.75 | B | C |
| ATOM | 4688 | O | LEU | B | 329 | 21.024 | 36.322 | 91.259 | 1.00 | 21.75 | B | O |
| ATOM | 4689 | N | THR | B | 330 | 20.983 | 36.596 | 93.496 | 1.00 | 25.41 | B | N |
| ATOM | 4690 | CA | THR | B | 330 | 19.904 | 37.570 | 93.511 | 1.00 | 25.41 | B | C |
| ATOM | 4691 | CB | THR | B | 330 | 19.010 | 37.358 | 94.751 | 1.00 | 50.79 | B | C |
| ATOM | 4692 | OG1 | THR | B | 330 | 18.289 | 36.130 | 94.604 | 1.00 | 50.79 | B | O |
| ATOM | 4693 | CG2 | THR | B | 330 | 18.031 | 38.521 | 94.946 | 1.00 | 50.79 | B | C |
| ATOM | 4694 | C | THR | B | 330 | 20.636 | 38.881 | 93.678 | 1.00 | 25.41 | B | C |
| ATOM | 4695 | O | THR | B | 330 | 21.762 | 38.897 | 94.175 | 1.00 | 25.41 | B | O |
| ATOM | 4696 | N | PRO | B | 331 | 20.035 | 39.992 | 93.244 | 1.00 | 20.00 | B | N |
| ATOM | 4697 | CD | PRO | B | 331 | 18.905 | 40.127 | 92.314 | 1.00 | 30.41 | B | C |
| ATOM | 4698 | CA | PRO | B | 331 | 20.723 | 41.272 | 93.412 | 1.00 | 20.00 | B | C |
| ATOM | 4699 | CB | PRO | B | 331 | 19.695 | 42.268 | 92.917 | 1.00 | 30.41 | B | C |
| ATOM | 4700 | CG | PRO | B | 331 | 19.106 | 41.524 | 91.767 | 1.00 | 30.41 | B | C |
| ATOM | 4701 | C | PRO | B | 331 | 21.120 | 41.508 | 94.871 | 1.00 | 20.00 | B | C |
| ATOM | 4702 | O | PRO | B | 331 | 22.179 | 42.055 | 95.169 | 1.00 | 20.00 | B | O |
| ATOM | 4703 | N | LEU | B | 332 | 20.269 | 41.071 | 95.784 | 1.00 | 28.39 | B | N |
| ATOM | 4704 | CA | LEU | B | 332 | 20.550 | 41.249 | 97.197 | 1.00 | 28.39 | B | C |
| ATOM | 4705 | CB | LEU | B | 332 | 19.273 | 40.980 | 97.990 | 1.00 | 22.19 | B | C |
| ATOM | 4706 | CG | LEU | B | 332 | 19.186 | 41.701 | 99.326 | 1.00 | 22.19 | B | C |
| ATOM | 4707 | CD1 | LEU | B | 332 | 19.571 | 43.164 | 99.168 | 1.00 | 22.19 | B | C |
| ATOM | 4708 | CD2 | LEU | B | 332 | 17.777 | 41.566 | 99.850 | 1.00 | 22.19 | B | C |
| ATOM | 4709 | C | LEU | B | 332 | 21.689 | 40.336 | 97.643 | 1.00 | 28.39 | B | C |
| ATOM | 4710 | O | LEU | B | 332 | 22.614 | 40.783 | 98.324 | 1.00 | 28.39 | B | O |
| ATOM | 4711 | N | GLU | B | 333 | 21.631 | 39.064 | 97.243 | 1.00 | 28.81 | B | N |
| ATOM | 4712 | CA | GLU | B | 333 | 22.674 | 38.112 | 97.620 | 1.00 | 28.81 | B | C |
| ATOM | 4713 | CB | GLU | B | 333 | 22.339 | 36.703 | 97.147 | 1.00 | 27.28 | B | C |
| ATOM | 4714 | CG | GLU | B | 333 | 21.037 | 36.176 | 97.688 | 1.00 | 27.28 | B | C |
| ATOM | 4715 | CD | GLU | B | 333 | 20.663 | 34.829 | 97.096 | 1.00 | 27.28 | B | C |
| ATOM | 4716 | OE1 | GLU | B | 333 | 20.991 | 34.596 | 95.913 | 1.00 | 27.28 | B | O |
| ATOM | 4717 | OE2 | GLU | B | 333 | 20.028 | 34.022 | 97.806 | 1.00 | 27.28 | B | O |
| ATOM | 4718 | C | GLU | B | 333 | 23.990 | 38.538 | 97.026 | 1.00 | 28.81 | B | C |
| ATOM | 4719 | O | GLU | B | 333 | 25.043 | 38.177 | 97.538 | 1.00 | 28.81 | B | O |

| | | | | | | | | | | | | |
|---|---|---|---|---|---|---|---|---|---|---|---|---|
| ATOM | 4720 | N | ALA | B | 334 | 23.922 | 39.302 | 95.939 | 1.00 | 31.79 | B | N |
| ATOM | 4721 | CA | ALA | B | 334 | 25.123 | 39.795 | 95.279 | 1.00 | 31.79 | B | C |
| ATOM | 4722 | CB | ALA | B | 334 | 24.797 | 40.267 | 93.850 | 1.00 | 37.89 | B | C |
| ATOM | 4723 | C | ALA | B | 334 | 25.699 | 40.936 | 96.108 | 1.00 | 31.79 | B | C |
| ATOM | 4724 | O | ALA | B | 334 | 26.912 | 41.032 | 96.275 | 1.00 | 31.79 | B | O |
| ATOM | 4725 | N | CYS | B | 335 | 24.817 | 41.783 | 96.635 | 1.00 | 24.00 | B | N |
| ATOM | 4726 | CA | CYS | B | 335 | 25.233 | 42.907 | 97.468 | 1.00 | 24.00 | B | C |
| ATOM | 4727 | CB | CYS | B | 335 | 24.019 | 43.689 | 97.966 | 1.00 | 23.27 | B | C |
| ATOM | 4728 | SG | CYS | B | 335 | 23.279 | 44.785 | 96.758 | 1.00 | 23.27 | B | S |
| ATOM | 4729 | C | CYS | B | 335 | 25.981 | 42.377 | 98.675 | 1.00 | 24.00 | B | C |
| ATOM | 4730 | O | CYS | B | 335 | 26.978 | 42.950 | 99.111 | 1.00 | 24.00 | B | O |
| ATOM | 4731 | N | ALA | B | 336 | 25.482 | 41.265 | 99.202 | 1.00 | 29.00 | B | N |
| ATOM | 4732 | CA | ALA | B | 336 | 26.050 | 40.641 | 100.384 | 1.00 | 29.00 | B | C |
| ATOM | 4733 | CB | ALA | B | 336 | 24.976 | 39.857 | 101.111 | 1.00 | 15.99 | B | C |
| ATOM | 4734 | C | ALA | B | 336 | 27.228 | 39.739 | 100.109 | 1.00 | 29.00 | B | C |
| ATOM | 4735 | O | ALA | B | 336 | 27.724 | 39.098 | 101.019 | 1.00 | 29.00 | B | O |
| ATOM | 4736 | N | HIS | B | 337 | 27.682 | 39.687 | 98.864 | 1.00 | 19.93 | B | N |
| ATOM | 4737 | CA | HIS | B | 337 | 28.807 | 38.837 | 98.512 | 1.00 | 19.93 | B | C |
| ATOM | 4738 | CB | HIS | B | 337 | 28.972 | 38.795 | 96.989 | 1.00 | 32.75 | B | C |
| ATOM | 4739 | CG | HIS | B | 337 | 29.886 | 37.708 | 96.511 | 1.00 | 32.75 | B | C |
| ATOM | 4740 | CD2 | HIS | B | 337 | 29.623 | 36.452 | 96.077 | 1.00 | 32.75 | B | C |
| ATOM | 4741 | ND1 | HIS | B | 337 | 31.262 | 37.829 | 96.522 | 1.00 | 32.75 | B | N |
| ATOM | 4742 | CE1 | HIS | B | 337 | 31.803 | 36.694 | 96.121 | 1.00 | 32.75 | B | C |
| ATOM | 4743 | NE2 | HIS | B | 337 | 30.830 | 35.842 | 95.846 | 1.00 | 32.75 | B | N |
| ATOM | 4744 | C | HIS | B | 337 | 30.094 | 39.310 | 99.178 | 1.00 | 19.93 | B | C |
| ATOM | 4745 | O | HIS | B | 337 | 30.265 | 40.486 | 99.475 | 1.00 | 19.93 | B | O |
| ATOM | 4746 | N | SER | B | 338 | 31.005 | 38.379 | 99.411 | 1.00 | 28.34 | B | N |
| ATOM | 4747 | CA | SER | B | 338 | 32.271 | 38.697 | 100.052 | 1.00 | 28.34 | B | C |
| ATOM | 4748 | CB | SER | B | 338 | 33.108 | 37.424 | 100.178 | 1.00 | 51.45 | B | C |
| ATOM | 4749 | OG | SER | B | 338 | 34.043 | 37.539 | 101.236 | 1.00 | 51.45 | B | O |
| ATOM | 4750 | C | SER | B | 338 | 33.069 | 39.781 | 99.306 | 1.00 | 28.34 | B | C |
| ATOM | 4751 | O | SER | B | 338 | 33.743 | 40.614 | 99.918 | 1.00 | 28.34 | B | O |
| ATOM | 4752 | N | PHE | B | 339 | 32.996 | 39.764 | 97.981 | 1.00 | 30.23 | B | N |
| ATOM | 4753 | CA | PHE | B | 339 | 33.697 | 40.752 | 97.173 | 1.00 | 30.23 | B | C |
| ATOM | 4754 | CB | PHE | B | 339 | 33.278 | 40.604 | 95.712 | 1.00 | 19.76 | B | C |
| ATOM | 4755 | CG | PHE | B | 339 | 33.845 | 41.653 | 94.814 | 1.00 | 19.76 | B | C |
| ATOM | 4756 | CD1 | PHE | B | 339 | 35.209 | 41.725 | 94.590 | 1.00 | 19.76 | B | C |
| ATOM | 4757 | CD2 | PHE | B | 339 | 33.017 | 42.587 | 94.214 | 1.00 | 19.76 | B | C |
| ATOM | 4758 | CE1 | PHE | B | 339 | 35.749 | 42.719 | 93.783 | 1.00 | 19.76 | B | C |
| ATOM | 4759 | CE2 | PHE | B | 339 | 33.540 | 43.585 | 93.407 | 1.00 | 19.76 | B | C |
| ATOM | 4760 | CZ | PHE | B | 339 | 34.914 | 43.653 | 93.187 | 1.00 | 19.76 | B | C |
| ATOM | 4761 | C | PHE | B | 339 | 33.444 | 42.200 | 97.631 | 1.00 | 30.23 | B | C |
| ATOM | 4762 | O | PHE | B | 339 | 34.286 | 43.067 | 97.425 | 1.00 | 30.23 | B | O |
| ATOM | 4763 | N | PHE | B | 340 | 32.300 | 42.462 | 98.260 | 1.00 | 23.95 | B | N |
| ATOM | 4764 | CA | PHE | B | 340 | 31.976 | 43.823 | 98.692 | 1.00 | 23.95 | B | C |
| ATOM | 4765 | CB | PHE | B | 340 | 30.503 | 44.127 | 98.396 | 1.00 | 41.57 | B | C |
| ATOM | 4766 | CG | PHE | B | 340 | 30.168 | 44.117 | 96.928 | 1.00 | 41.57 | B | C |
| ATOM | 4767 | CD1 | PHE | B | 340 | 30.671 | 45.102 | 96.078 | 1.00 | 41.57 | B | C |
| ATOM | 4768 | CD2 | PHE | B | 340 | 29.368 | 43.114 | 96.386 | 1.00 | 41.57 | B | C |
| ATOM | 4769 | CE1 | PHE | B | 340 | 30.373 | 45.084 | 94.717 | 1.00 | 41.57 | B | C |
| ATOM | 4770 | CE2 | PHE | B | 340 | 29.070 | 43.094 | 95.029 | 1.00 | 41.57 | B | C |
| ATOM | 4771 | CZ | PHE | B | 340 | 29.571 | 44.076 | 94.196 | 1.00 | 41.57 | B | C |
| ATOM | 4772 | C | PHE | B | 340 | 32.271 | 44.168 | 100.147 | 1.00 | 23.95 | B | C |
| ATOM | 4773 | O | PHE | B | 340 | 31.939 | 45.259 | 100.617 | 1.00 | 23.95 | B | O |
| ATOM | 4774 | N | ASP | B | 341 | 32.900 | 43.242 | 100.855 | 1.00 | 19.60 | B | N |
| ATOM | 4775 | CA | ASP | B | 341 | 33.227 | 43.460 | 102.247 | 1.00 | 19.60 | B | C |
| ATOM | 4776 | CB | ASP | B | 341 | 34.096 | 42.326 | 102.749 | 1.00 | 28.00 | B | C |
| ATOM | 4777 | CG | ASP | B | 341 | 33.363 | 41.026 | 102.787 | 1.00 | 28.00 | B | C |
| ATOM | 4778 | OD1 | ASP | B | 341 | 32.118 | 41.063 | 102.824 | 1.00 | 28.00 | B | O |
| ATOM | 4779 | OD2 | ASP | B | 341 | 34.029 | 39.973 | 102.794 | 1.00 | 28.00 | B | O |
| ATOM | 4780 | C | ASP | B | 341 | 33.930 | 44.785 | 102.505 | 1.00 | 19.60 | B | C |
| ATOM | 4781 | O | ASP | B | 341 | 33.575 | 45.517 | 103.416 | 1.00 | 19.60 | B | O |
| ATOM | 4782 | N | GLU | B | 342 | 34.940 | 45.080 | 101.706 | 1.00 | 21.23 | B | N |

| | | | | | | | | | | | | |
|---|---|---|---|---|---|---|---|---|---|---|---|---|
| ATOM | 4783 | CA | GLU | B | 342 | 35.700 | 46.309 | 101.858 | 1.00 | 21.23 | B | C |
| ATOM | 4784 | CB | GLU | B | 342 | 36.689 | 46.444 | 100.696 | 1.00 | 38.47 | B | C |
| ATOM | 4785 | CG | GLU | B | 342 | 37.574 | 47.678 | 100.757 | 1.00 | 38.47 | B | C |
| ATOM | 4786 | CD | GLU | B | 342 | 38.522 | 47.807 | 99.559 | 1.00 | 38.47 | B | C |
| ATOM | 4787 | OE1 | GLU | B | 342 | 39.238 | 48.826 | 99.504 | 1.00 | 38.47 | B | O |
| ATOM | 4788 | OE2 | GLU | B | 342 | 38.561 | 46.908 | 98.676 | 1.00 | 38.47 | B | O |
| ATOM | 4789 | C | GLU | B | 342 | 34.836 | 47.563 | 101.945 | 1.00 | 21.23 | B | C |
| ATOM | 4790 | O | GLU | B | 342 | 35.297 | 48.602 | 102.412 | 1.00 | 21.23 | B | O |
| ATOM | 4791 | N | LEU | B | 343 | 33.592 | 47.476 | 101.488 | 1.00 | 16.96 | B | N |
| ATOM | 4792 | CA | LEU | B | 343 | 32.701 | 48.631 | 101.520 | 1.00 | 16.96 | B | C |
| ATOM | 4793 | CB | LEU | B | 343 | 31.649 | 48.522 | 100.415 | 1.00 | 16.76 | B | C |
| ATOM | 4794 | CG | LEU | B | 343 | 32.164 | 48.517 | 98.977 | 1.00 | 16.76 | B | C |
| ATOM | 4795 | CD1 | LEU | B | 343 | 31.001 | 48.457 | 98.019 | 1.00 | 16.76 | B | C |
| ATOM | 4796 | CD2 | LEU | B | 343 | 32.987 | 49.760 | 98.725 | 1.00 | 16.76 | B | C |
| ATOM | 4797 | C | LEU | B | 343 | 32.030 | 48.692 | 102.882 | 1.00 | 16.96 | B | C |
| ATOM | 4798 | O | LEU | B | 343 | 31.615 | 49.753 | 103.352 | 1.00 | 16.96 | B | O |
| ATOM | 4799 | N | ARG | B | 344 | 31.935 | 47.535 | 103.517 | 1.00 | 37.49 | B | N |
| ATOM | 4800 | CA | ARG | B | 344 | 31.324 | 47.446 | 104.823 | 1.00 | 37.49 | B | C |
| ATOM | 4801 | CB | ARG | B | 344 | 30.694 | 46.076 | 105.024 | 1.00 | 29.30 | B | C |
| ATOM | 4802 | CG | ARG | B | 344 | 29.335 | 45.942 | 104.383 | 1.00 | 29.30 | B | C |
| ATOM | 4803 | CD | ARG | B | 344 | 28.867 | 44.516 | 104.479 | 1.00 | 29.30 | B | C |
| ATOM | 4804 | NE | ARG | B | 344 | 29.666 | 43.647 | 103.625 | 1.00 | 29.30 | B | N |
| ATOM | 4805 | CZ | ARG | B | 344 | 29.360 | 43.361 | 102.367 | 1.00 | 29.30 | B | C |
| ATOM | 4806 | NH1 | ARG | B | 344 | 28.269 | 43.874 | 101.811 | 1.00 | 29.30 | B | N |
| ATOM | 4807 | NH2 | ARG | B | 344 | 30.143 | 42.553 | 101.668 | 1.00 | 29.30 | B | N |
| ATOM | 4808 | C | ARG | B | 344 | 32.339 | 47.716 | 105.916 | 1.00 | 37.49 | B | C |
| ATOM | 4809 | O | ARG | B | 344 | 32.019 | 47.642 | 107.108 | 1.00 | 37.49 | B | O |
| ATOM | 4810 | N | ASP | B | 345 | 33.569 | 48.019 | 105.517 | 1.00 | 32.41 | B | N |
| ATOM | 4811 | CA | ASP | B | 345 | 34.595 | 48.321 | 106.493 | 1.00 | 32.41 | B | C |
| ATOM | 4812 | CB | ASP | B | 345 | 35.982 | 48.167 | 105.889 | 1.00 | 43.73 | B | C |
| ATOM | 4813 | CG | ASP | B | 345 | 37.079 | 48.197 | 106.944 | 1.00 | 43.73 | B | C |
| ATOM | 4814 | OD1 | ASP | B | 345 | 37.609 | 47.100 | 107.248 | 1.00 | 43.73 | B | O |
| ATOM | 4815 | OD2 | ASP | B | 345 | 37.399 | 49.296 | 107.476 | 1.00 | 43.73 | B | O |
| ATOM | 4816 | C | ASP | B | 345 | 34.394 | 49.777 | 106.903 | 1.00 | 32.41 | B | C |
| ATOM | 4817 | O | ASP | B | 345 | 34.366 | 50.675 | 106.055 | 1.00 | 32.41 | B | O |
| ATOM | 4818 | N | PRO | B | 346 | 34.261 | 50.031 | 108.211 | 1.00 | 32.35 | B | N |
| ATOM | 4819 | CD | PRO | B | 346 | 34.511 | 49.069 | 109.296 | 1.00 | 35.21 | B | C |
| ATOM | 4820 | CA | PRO | B | 346 | 34.062 | 51.374 | 108.761 | 1.00 | 32.35 | B | C |
| ATOM | 4821 | CB | PRO | B | 346 | 34.112 | 51.133 | 110.264 | 1.00 | 35.21 | B | C |
| ATOM | 4822 | CG | PRO | B | 346 | 35.045 | 49.971 | 110.377 | 1.00 | 35.21 | B | C |
| ATOM | 4823 | C | PRO | B | 346 | 35.094 | 52.407 | 108.333 | 1.00 | 32.35 | B | C |
| ATOM | 4824 | O | PRO | B | 346 | 34.783 | 53.587 | 108.212 | 1.00 | 32.35 | B | O |
| ATOM | 4825 | N | ASN | B | 347 | 36.327 | 51.965 | 108.112 | 1.00 | 36.58 | B | N |
| ATOM | 4826 | CA | ASN | B | 347 | 37.407 | 52.872 | 107.730 | 1.00 | 36.58 | B | C |
| ATOM | 4827 | CB | ASN | B | 347 | 38.734 | 52.352 | 108.284 | 1.00 | 55.21 | B | C |
| ATOM | 4828 | CG | ASN | B | 347 | 38.849 | 52.526 | 109.791 | 1.00 | 55.21 | B | C |
| ATOM | 4829 | OD1 | ASN | B | 347 | 39.798 | 52.030 | 110.417 | 1.00 | 55.21 | B | O |
| ATOM | 4830 | ND2 | ASN | B | 347 | 37.886 | 53.240 | 110.385 | 1.00 | 55.21 | B | N |
| ATOM | 4831 | C | ASN | B | 347 | 37.565 | 53.125 | 106.240 | 1.00 | 36.58 | B | C |
| ATOM | 4832 | O | ASN | B | 347 | 38.482 | 53.834 | 105.832 | 1.00 | 36.58 | B | O |
| ATOM | 4833 | N | VAL | B | 348 | 36.684 | 52.559 | 105.421 | 1.00 | 39.90 | B | N |
| ATOM | 4834 | CA | VAL | B | 348 | 36.812 | 52.752 | 103.984 | 1.00 | 39.90 | B | C |
| ATOM | 4835 | CB | VAL | B | 348 | 35.848 | 51.851 | 103.170 | 1.00 | 31.32 | B | C |
| ATOM | 4836 | CG1 | VAL | B | 348 | 34.420 | 52.351 | 103.272 | 1.00 | 31.32 | B | C |
| ATOM | 4837 | CG2 | VAL | B | 348 | 36.300 | 51.823 | 101.725 | 1.00 | 31.32 | B | C |
| ATOM | 4838 | C | VAL | B | 348 | 36.578 | 54.202 | 103.590 | 1.00 | 39.90 | B | C |
| ATOM | 4839 | O | VAL | B | 348 | 35.653 | 54.864 | 104.079 | 1.00 | 39.90 | B | O |
| ATOM | 4840 | N | LYS | B | 349 | 37.437 | 54.692 | 102.707 | 1.00 | 25.40 | B | N |
| ATOM | 4841 | CA | LYS | B | 349 | 37.349 | 56.059 | 102.220 | 1.00 | 25.40 | B | C |
| ATOM | 4842 | CB | LYS | B | 349 | 38.466 | 56.904 | 102.836 | 1.00 | 35.84 | B | C |
| ATOM | 4843 | CG | LYS | B | 349 | 38.026 | 58.264 | 103.361 | 1.00 | 35.84 | B | C |
| ATOM | 4844 | CD | LYS | B | 349 | 37.371 | 58.166 | 104.741 | 1.00 | 35.84 | B | C |
| ATOM | 4845 | CE | LYS | B | 349 | 36.968 | 59.552 | 105.258 | 1.00 | 35.84 | B | C |

| | | | | | | | | | | | | |
|---|---|---|---|---|---|---|---|---|---|---|---|---|
| ATOM | 4846 | NZ | LYS | B | 349 | 36.585 | 59.524 | 106.699 | 1.00 | 35.84 | B | N |
| ATOM | 4847 | C | LYS | B | 349 | 37.530 | 55.981 | 100.709 | 1.00 | 25.40 | B | C |
| ATOM | 4848 | O | LYS | B | 349 | 37.854 | 54.924 | 100.174 | 1.00 | 25.40 | B | O |
| ATOM | 4849 | N | LEU | B | 350 | 37.322 | 57.088 | 100.012 | 1.00 | 37.15 | B | N |
| ATOM | 4850 | CA | LEU | B | 350 | 37.478 | 57.078 | 98.563 | 1.00 | 37.15 | B | C |
| ATOM | 4851 | CB | LEU | B | 350 | 36.508 | 58.062 | 97.914 | 1.00 | 13.22 | B | C |
| ATOM | 4852 | CG | LEU | B | 350 | 35.022 | 57.804 | 98.154 | 1.00 | 13.22 | B | C |
| ATOM | 4853 | CD1 | LEU | B | 350 | 34.234 | 58.923 | 97.521 | 1.00 | 13.22 | B | C |
| ATOM | 4854 | CD2 | LEU | B | 350 | 34.605 | 56.456 | 97.586 | 1.00 | 13.22 | B | C |
| ATOM | 4855 | C | LEU | B | 350 | 38.905 | 57.460 | 98.215 | 1.00 | 37.15 | B | C |
| ATOM | 4856 | O | LEU | B | 350 | 39.649 | 57.946 | 99.060 | 1.00 | 37.15 | B | O |
| ATOM | 4857 | N | PRO | B | 351 | 39.311 | 57.254 | 96.958 | 1.00 | 39.18 | B | N |
| ATOM | 4858 | CD | PRO | B | 351 | 38.598 | 56.761 | 95.772 | 1.00 | 36.83 | B | C |
| ATOM | 4859 | CA | PRO | B | 351 | 40.687 | 57.619 | 96.622 | 1.00 | 39.18 | B | C |
| ATOM | 4860 | CB | PRO | B | 351 | 40.864 | 57.048 | 95.212 | 1.00 | 36.83 | B | C |
| ATOM | 4861 | CG | PRO | B | 351 | 39.700 | 56.094 | 95.035 | 1.00 | 36.83 | B | C |
| ATOM | 4862 | C | PRO | B | 351 | 40.837 | 59.138 | 96.626 | 1.00 | 39.18 | B | C |
| ATOM | 4863 | O | PRO | B | 351 | 41.946 | 59.660 | 96.629 | 1.00 | 39.18 | B | O |
| ATOM | 4864 | N | ASN | B | 352 | 39.718 | 59.852 | 96.616 | 1.00 | 25.97 | B | N |
| ATOM | 4865 | CA | ASN | B | 352 | 39.780 | 61.306 | 96.615 | 1.00 | 25.97 | B | C |
| ATOM | 4866 | CB | ASN | B | 352 | 38.695 | 61.884 | 95.700 | 1.00 | 37.02 | B | C |
| ATOM | 4867 | CG | ASN | B | 352 | 37.337 | 62.005 | 96.376 | 1.00 | 37.02 | B | C |
| ATOM | 4868 | OD1 | ASN | B | 352 | 36.352 | 62.359 | 95.733 | 1.00 | 37.02 | B | O |
| ATOM | 4869 | ND2 | ASN | B | 352 | 37.281 | 61.728 | 97.671 | 1.00 | 37.02 | B | N |
| ATOM | 4870 | C | ASN | B | 352 | 39.672 | 61.893 | 98.023 | 1.00 | 25.97 | B | C |
| ATOM | 4871 | O | ASN | B | 352 | 39.508 | 63.095 | 98.206 | 1.00 | 25.97 | B | O |
| ATOM | 4872 | N | GLY | B | 353 | 39.725 | 61.021 | 99.018 | 1.00 | 35.80 | B | N |
| ATOM | 4873 | CA | GLY | B | 353 | 39.675 | 61.474 | 100.387 | 1.00 | 35.80 | B | C |
| ATOM | 4874 | C | GLY | B | 353 | 38.327 | 61.569 | 101.067 | 1.00 | 35.80 | B | C |
| ATOM | 4875 | O | GLY | B | 353 | 38.269 | 61.454 | 102.295 | 1.00 | 35.80 | B | O |
| ATOM | 4876 | N | ARG | B | 354 | 37.246 | 61.789 | 100.324 | 1.00 | 36.30 | B | N |
| ATOM | 4877 | CA | ARG | B | 354 | 35.947 | 61.893 | 100.982 | 1.00 | 36.30 | B | C |
| ATOM | 4878 | CB | ARG | B | 354 | 34.974 | 62.725 | 100.143 | 1.00 | 45.23 | B | C |
| ATOM | 4879 | CG | ARG | B | 354 | 34.924 | 62.417 | 98.674 | 1.00 | 45.23 | B | C |
| ATOM | 4880 | CD | ARG | B | 354 | 34.017 | 63.418 | 97.940 | 1.00 | 45.23 | B | C |
| ATOM | 4881 | NE | ARG | B | 354 | 34.722 | 64.622 | 97.483 | 1.00 | 45.23 | B | N |
| ATOM | 4882 | CZ | ARG | B | 354 | 34.125 | 65.661 | 96.890 | 1.00 | 45.23 | B | C |
| ATOM | 4883 | NH1 | ARG | B | 354 | 32.807 | 65.649 | 96.686 | 1.00 | 45.23 | B | N |
| ATOM | 4884 | NH2 | ARG | B | 354 | 34.840 | 66.706 | 96.482 | 1.00 | 45.23 | B | N |
| ATOM | 4885 | C | ARG | B | 354 | 35.335 | 60.549 | 101.363 | 1.00 | 36.30 | B | C |
| ATOM | 4886 | O | ARG | B | 354 | 35.903 | 59.498 | 101.073 | 1.00 | 36.30 | B | O |
| ATOM | 4887 | N | ASP | B | 355 | 34.198 | 60.581 | 102.051 | 1.00 | 38.82 | B | N |
| ATOM | 4888 | CA | ASP | B | 355 | 33.547 | 59.346 | 102.474 | 1.00 | 38.82 | B | C |
| ATOM | 4889 | CB | ASP | B | 355 | 32.612 | 59.598 | 103.653 | 1.00 | 60.40 | B | C |
| ATOM | 4890 | CG | ASP | B | 355 | 33.334 | 60.188 | 104.834 | 1.00 | 60.40 | B | C |
| ATOM | 4891 | OD1 | ASP | B | 355 | 33.771 | 61.363 | 104.724 | 1.00 | 60.40 | B | O |
| ATOM | 4892 | OD2 | ASP | B | 355 | 33.484 | 59.480 | 105.863 | 1.00 | 60.40 | B | O |
| ATOM | 4893 | C | ASP | B | 355 | 32.769 | 58.717 | 101.348 | 1.00 | 38.82 | B | C |
| ATOM | 4894 | O | ASP | B | 355 | 32.411 | 59.377 | 100.373 | 1.00 | 38.82 | B | O |
| ATOM | 4895 | N | THR | B | 356 | 32.518 | 57.427 | 101.489 | 1.00 | 34.74 | B | N |
| ATOM | 4896 | CA | THR | B | 356 | 31.769 | 56.698 | 100.491 | 1.00 | 34.74 | B | C |
| ATOM | 4897 | CB | THR | B | 356 | 31.916 | 55.186 | 100.734 | 1.00 | 34.59 | B | C |
| ATOM | 4898 | OG1 | THR | B | 356 | 31.670 | 54.899 | 102.116 | 1.00 | 34.59 | B | O |
| ATOM | 4899 | CG2 | THR | B | 356 | 33.321 | 54.735 | 100.395 | 1.00 | 34.59 | B | C |
| ATOM | 4900 | C | THR | B | 356 | 30.304 | 57.124 | 100.594 | 1.00 | 34.74 | B | C |
| ATOM | 4901 | O | THR | B | 356 | 29.847 | 57.570 | 101.653 | 1.00 | 34.74 | B | O |
| ATOM | 4902 | N | PRO | B | 357 | 29.556 | 57.021 | 99.486 | 1.00 | 16.76 | B | N |
| ATOM | 4903 | CD | PRO | B | 357 | 29.983 | 56.591 | 98.145 | 1.00 | 21.95 | B | C |
| ATOM | 4904 | CA | PRO | B | 357 | 28.146 | 57.400 | 99.486 | 1.00 | 16.76 | B | C |
| ATOM | 4905 | CB | PRO | B | 357 | 27.794 | 57.392 | 98.006 | 1.00 | 21.95 | B | C |
| ATOM | 4906 | CG | PRO | B | 357 | 28.670 | 56.336 | 97.468 | 1.00 | 21.95 | B | C |
| ATOM | 4907 | C | PRO | B | 357 | 27.307 | 56.442 | 100.313 | 1.00 | 16.76 | B | C |
| ATOM | 4908 | O | PRO | B | 357 | 27.813 | 55.445 | 100.808 | 1.00 | 16.76 | B | O |

| | | | | | | | | | | | | |
|---|---|---|---|---|---|---|---|---|---|---|---|---|
| ATOM | 4909 | N | ALA | B | 358 | 26.033 | 56.766 | 100.484 | 1.00 | 26.37 | B | N |
| ATOM | 4910 | CA | ALA | B | 358 | 25.109 | 55.926 | 101.247 | 1.00 | 26.37 | B | C |
| ATOM | 4911 | CB | ALA | B | 358 | 23.696 | 56.501 | 101.146 | 1.00 | 23.45 | B | C |
| ATOM | 4912 | C | ALA | B | 358 | 25.132 | 54.520 | 100.666 | 1.00 | 26.37 | B | C |
| ATOM | 4913 | O | ALA | B | 358 | 24.879 | 54.337 | 99.475 | 1.00 | 26.37 | B | O |
| ATOM | 4914 | N | LEU | B | 359 | 25.414 | 53.527 | 101.499 | 1.00 | 23.95 | B | N |
| ATOM | 4915 | CA | LEU | B | 359 | 25.492 | 52.151 | 101.023 | 1.00 | 23.95 | B | C |
| ATOM | 4916 | CB | LEU | B | 359 | 26.948 | 51.690 | 101.096 | 1.00 | 21.92 | B | C |
| ATOM | 4917 | CG | LEU | B | 359 | 27.868 | 51.807 | 99.872 | 1.00 | 21.92 | B | C |
| ATOM | 4918 | CD1 | LEU | B | 359 | 27.549 | 53.015 | 99.032 | 1.00 | 21.92 | B | C |
| ATOM | 4919 | CD2 | LEU | B | 359 | 29.302 | 51.866 | 100.352 | 1.00 | 21.92 | B | C |
| ATOM | 4920 | C | LEU | B | 359 | 24.617 | 51.193 | 101.810 | 1.00 | 23.95 | B | C |
| ATOM | 4921 | O | LEU | B | 359 | 24.251 | 50.122 | 101.320 | 1.00 | 23.95 | B | O |
| ATOM | 4922 | N | PHE | B | 360 | 24.260 | 51.609 | 103.021 | 1.00 | 29.67 | B | N |
| ATOM | 4923 | CA | PHE | B | 360 | 23.491 | 50.774 | 103.927 | 1.00 | 29.67 | B | C |
| ATOM | 4924 | CB | PHE | B | 360 | 24.319 | 50.573 | 105.187 | 1.00 | 23.59 | B | C |
| ATOM | 4925 | CG | PHE | B | 360 | 25.777 | 50.487 | 104.916 | 1.00 | 23.59 | B | C |
| ATOM | 4926 | CD1 | PHE | B | 360 | 26.298 | 49.419 | 104.196 | 1.00 | 23.59 | B | C |
| ATOM | 4927 | CD2 | PHE | B | 360 | 26.626 | 51.492 | 105.329 | 1.00 | 23.59 | B | C |
| ATOM | 4928 | CE1 | PHE | B | 360 | 27.649 | 49.363 | 103.881 | 1.00 | 23.59 | B | C |
| ATOM | 4929 | CE2 | PHE | B | 360 | 27.972 | 51.451 | 105.023 | 1.00 | 23.59 | B | C |
| ATOM | 4930 | CZ | PHE | B | 360 | 28.489 | 50.383 | 104.298 | 1.00 | 23.59 | B | C |
| ATOM | 4931 | C | PHE | B | 360 | 22.109 | 51.264 | 104.313 | 1.00 | 29.67 | B | C |
| ATOM | 4932 | O | PHE | B | 360 | 21.492 | 50.692 | 105.203 | 1.00 | 29.67 | B | O |
| ATOM | 4933 | N | ASN | B | 361 | 21.619 | 52.313 | 103.665 | 1.00 | 27.09 | B | N |
| ATOM | 4934 | CA | ASN | B | 361 | 20.298 | 52.841 | 103.992 | 1.00 | 27.09 | B | C |
| ATOM | 4935 | CB | ASN | B | 361 | 20.185 | 54.287 | 103.512 | 1.00 | 19.24 | B | C |
| ATOM | 4936 | CG | ASN | B | 361 | 20.409 | 54.426 | 102.021 | 1.00 | 19.24 | B | C |
| ATOM | 4937 | OD1 | ASN | B | 361 | 21.218 | 53.711 | 101.431 | 1.00 | 19.24 | B | O |
| ATOM | 4938 | ND2 | ASN | B | 361 | 19.706 | 55.361 | 101.406 | 1.00 | 19.24 | B | N |
| ATOM | 4939 | C | ASN | B | 361 | 19.203 | 51.993 | 103.353 | 1.00 | 27.09 | B | C |
| ATOM | 4940 | O | ASN | B | 361 | 18.332 | 52.507 | 102.663 | 1.00 | 27.09 | B | O |
| ATOM | 4941 | N | PHE | B | 362 | 19.261 | 50.689 | 103.583 | 1.00 | 14.79 | B | N |
| ATOM | 4942 | CA | PHE | B | 362 | 18.274 | 49.767 | 103.046 | 1.00 | 14.79 | B | C |
| ATOM | 4943 | CB | PHE | B | 362 | 18.673 | 48.332 | 103.379 | 1.00 | 29.26 | B | C |
| ATOM | 4944 | CG | PHE | B | 362 | 19.821 | 47.821 | 102.576 | 1.00 | 29.26 | B | C |
| ATOM | 4945 | CD1 | PHE | B | 362 | 19.638 | 47.429 | 101.254 | 1.00 | 29.26 | B | C |
| ATOM | 4946 | CD2 | PHE | B | 362 | 21.093 | 47.746 | 103.126 | 1.00 | 29.26 | B | C |
| ATOM | 4947 | CE1 | PHE | B | 362 | 20.707 | 46.971 | 100.494 | 1.00 | 29.26 | B | C |
| ATOM | 4948 | CE2 | PHE | B | 362 | 22.163 | 47.289 | 102.368 | 1.00 | 29.26 | B | C |
| ATOM | 4949 | CZ | PHE | B | 362 | 21.967 | 46.902 | 101.053 | 1.00 | 29.26 | B | C |
| ATOM | 4950 | C | PHE | B | 362 | 16.903 | 50.030 | 103.649 | 1.00 | 14.79 | B | C |
| ATOM | 4951 | O | PHE | B | 362 | 16.794 | 50.551 | 104.756 | 1.00 | 14.79 | B | O |
| ATOM | 4952 | N | THR | B | 363 | 15.861 | 49.665 | 102.905 | 1.00 | 23.27 | B | N |
| ATOM | 4953 | CA | THR | B | 363 | 14.484 | 49.788 | 103.368 | 1.00 | 23.27 | B | C |
| ATOM | 4954 | CB | THR | B | 363 | 13.630 | 50.640 | 102.446 | 1.00 | 14.99 | B | C |
| ATOM | 4955 | OG1 | THR | B | 363 | 13.622 | 50.056 | 101.140 | 1.00 | 14.99 | B | O |
| ATOM | 4956 | CG2 | THR | B | 363 | 14.156 | 52.041 | 102.393 | 1.00 | 14.99 | B | C |
| ATOM | 4957 | C | THR | B | 363 | 13.903 | 48.377 | 103.379 | 1.00 | 23.27 | B | C |
| ATOM | 4958 | O | THR | B | 363 | 14.467 | 47.470 | 102.766 | 1.00 | 23.27 | B | O |
| ATOM | 4959 | N | THR | B | 364 | 12.780 | 48.187 | 104.067 | 1.00 | 29.59 | B | N |
| ATOM | 4960 | CA | THR | B | 364 | 12.159 | 46.866 | 104.114 | 1.00 | 29.59 | B | C |
| ATOM | 4961 | CB | THR | B | 364 | 10.932 | 46.835 | 105.068 | 1.00 | 29.47 | B | C |
| ATOM | 4962 | OG1 | THR | B | 364 | 9.964 | 47.794 | 104.643 | 1.00 | 29.47 | B | O |
| ATOM | 4963 | CG2 | THR | B | 364 | 11.348 | 47.178 | 106.495 | 1.00 | 29.47 | B | C |
| ATOM | 4964 | C | THR | B | 364 | 11.740 | 46.387 | 102.714 | 1.00 | 29.59 | B | C |
| ATOM | 4965 | O | THR | B | 364 | 11.779 | 45.197 | 102.438 | 1.00 | 29.59 | B | O |
| ATOM | 4966 | N | GLN | B | 365 | 11.349 | 47.305 | 101.830 | 1.00 | 30.05 | B | N |
| ATOM | 4967 | CA | GLN | B | 365 | 10.965 | 46.922 | 100.473 | 1.00 | 30.05 | B | C |
| ATOM | 4968 | CB | GLN | B | 365 | 10.452 | 48.119 | 99.668 | 1.00 | 27.65 | B | C |
| ATOM | 4969 | CG | GLN | B | 365 | 8.988 | 48.028 | 99.226 | 1.00 | 27.65 | B | C |
| ATOM | 4970 | CD | GLN | B | 365 | 8.716 | 46.925 | 98.217 | 1.00 | 27.65 | B | C |
| ATOM | 4971 | OE1 | GLN | B | 365 | 9.164 | 46.990 | 97.082 | 1.00 | 27.65 | B | O |

| | | | | | | | | | | | | |
|---|---|---|---|---|---|---|---|---|---|---|---|---|
| ATOM | 4972 | NE2 | GLN | B | 365 | 7.975 | 45.907 | 98.634 | 1.00 | 27.65 | B | N |
| ATOM | 4973 | C | GLN | B | 365 | 12.191 | 46.374 | 99.762 | 1.00 | 30.05 | B | C |
| ATOM | 4974 | O | GLN | B | 365 | 12.140 | 45.360 | 99.062 | 1.00 | 30.05 | B | O |
| ATOM | 4975 | N | GLU | B | 366 | 13.303 | 47.072 | 99.954 | 1.00 | 38.74 | B | N |
| ATOM | 4976 | CA | GLU | B | 366 | 14.556 | 46.718 | 99.322 | 1.00 | 38.74 | B | C |
| ATOM | 4977 | CB | GLU | B | 366 | 15.535 | 47.888 | 99.490 | 1.00 | 27.83 | B | C |
| ATOM | 4978 | CG | GLU | B | 366 | 16.957 | 47.601 | 99.061 | 1.00 | 27.83 | B | C |
| ATOM | 4979 | CD | GLU | B | 366 | 17.761 | 48.858 | 98.828 | 1.00 | 27.83 | B | C |
| ATOM | 4980 | OE1 | GLU | B | 366 | 17.549 | 49.854 | 99.543 | 1.00 | 27.83 | B | O |
| ATOM | 4981 | OE2 | GLU | B | 366 | 18.617 | 48.846 | 97.931 | 1.00 | 27.83 | B | O |
| ATOM | 4982 | C | GLU | B | 366 | 15.123 | 45.403 | 99.862 | 1.00 | 38.74 | B | C |
| ATOM | 4983 | O | GLU | B | 366 | 15.932 | 44.745 | 99.202 | 1.00 | 38.74 | B | O |
| ATOM | 4984 | N | LEU | B | 367 | 14.683 | 45.003 | 101.049 | 1.00 | 32.77 | B | N |
| ATOM | 4985 | CA | LEU | B | 367 | 15.174 | 43.758 | 101.637 | 1.00 | 32.77 | B | C |
| ATOM | 4986 | CB | LEU | B | 367 | 15.571 | 43.964 | 103.101 | 1.00 | 26.49 | B | C |
| ATOM | 4987 | CG | LEU | B | 367 | 16.766 | 44.883 | 103.388 | 1.00 | 26.49 | B | C |
| ATOM | 4988 | CD1 | LEU | B | 367 | 16.802 | 45.194 | 104.865 | 1.00 | 26.49 | B | C |
| ATOM | 4989 | CD2 | LEU | B | 367 | 18.069 | 44.241 | 102.932 | 1.00 | 26.49 | B | C |
| ATOM | 4990 | C | LEU | B | 367 | 14.141 | 42.647 | 101.558 | 1.00 | 32.77 | B | C |
| ATOM | 4991 | O | LEU | B | 367 | 14.427 | 41.506 | 101.913 | 1.00 | 32.77 | B | O |
| ATOM | 4992 | N | SER | B | 368 | 12.949 | 42.984 | 101.079 | 1.00 | 17.32 | B | N |
| ATOM | 4993 | CA | SER | B | 368 | 11.860 | 42.025 | 100.968 | 1.00 | 17.32 | B | C |
| ATOM | 4994 | CB | SER | B | 368 | 10.768 | 42.585 | 100.059 | 1.00 | 28.23 | B | C |
| ATOM | 4995 | OG | SER | B | 368 | 11.104 | 42.412 | 98.694 | 1.00 | 28.23 | B | O |
| ATOM | 4996 | C | SER | B | 368 | 12.297 | 40.658 | 100.424 | 1.00 | 17.32 | B | C |
| ATOM | 4997 | O | SER | B | 368 | 11.965 | 39.613 | 100.982 | 1.00 | 17.32 | B | O |
| ATOM | 4998 | N | SER | B | 369 | 13.041 | 40.664 | 99.332 | 1.00 | 37.96 | B | N |
| ATOM | 4999 | CA | SER | B | 369 | 13.479 | 39.417 | 98.723 | 1.00 | 37.96 | B | C |
| ATOM | 5000 | CB | SER | B | 369 | 14.503 | 39.702 | 97.627 | 1.00 | 39.95 | B | C |
| ATOM | 5001 | OG | SER | B | 369 | 15.730 | 39.048 | 97.914 | 1.00 | 39.95 | B | O |
| ATOM | 5002 | C | SER | B | 369 | 14.085 | 38.425 | 99.716 | 1.00 | 37.96 | B | C |
| ATOM | 5003 | O | SER | B | 369 | 14.061 | 37.212 | 99.492 | 1.00 | 37.96 | B | O |
| ATOM | 5004 | N | ASN | B | 370 | 14.629 | 38.933 | 100.814 | 1.00 | 24.96 | B | N |
| ATOM | 5005 | CA | ASN | B | 370 | 15.255 | 38.062 | 101.797 | 1.00 | 24.96 | B | C |
| ATOM | 5006 | CB | ASN | B | 370 | 16.397 | 37.300 | 101.117 | 1.00 | 22.74 | B | C |
| ATOM | 5007 | CG | ASN | B | 370 | 17.270 | 36.529 | 102.090 | 1.00 | 22.74 | B | C |
| ATOM | 5008 | OD1 | ASN | B | 370 | 18.201 | 35.840 | 101.675 | 1.00 | 22.74 | B | O |
| ATOM | 5009 | ND2 | ASN | B | 370 | 16.983 | 36.638 | 103.380 | 1.00 | 22.74 | B | N |
| ATOM | 5010 | C | ASN | B | 370 | 15.781 | 38.881 | 102.977 | 1.00 | 24.96 | B | C |
| ATOM | 5011 | O | ASN | B | 370 | 16.978 | 39.141 | 103.083 | 1.00 | 24.96 | B | O |
| ATOM | 5012 | N | PRO | B | 371 | 14.875 | 39.287 | 103.885 | 1.00 | 21.40 | B | N |
| ATOM | 5013 | CD | PRO | B | 371 | 13.428 | 39.025 | 103.749 | 1.00 | 15.76 | B | C |
| ATOM | 5014 | CA | PRO | B | 371 | 15.139 | 40.082 | 105.093 | 1.00 | 21.40 | B | C |
| ATOM | 5015 | CB | PRO | B | 371 | 13.834 | 39.964 | 105.868 | 1.00 | 15.76 | B | C |
| ATOM | 5016 | CG | PRO | B | 371 | 12.825 | 39.982 | 104.768 | 1.00 | 15.76 | B | C |
| ATOM | 5017 | C | PRO | B | 371 | 16.350 | 39.695 | 105.935 | 1.00 | 21.40 | B | C |
| ATOM | 5018 | O | PRO | B | 371 | 17.136 | 40.545 | 106.327 | 1.00 | 21.40 | B | O |
| ATOM | 5019 | N | PRO | B | 372 | 16.511 | 38.407 | 106.233 | 1.00 | 29.97 | B | N |
| ATOM | 5020 | CD | PRO | B | 372 | 15.629 | 37.282 | 105.877 | 1.00 | 15.38 | B | C |
| ATOM | 5021 | CA | PRO | B | 372 | 17.641 | 37.941 | 107.036 | 1.00 | 29.97 | B | C |
| ATOM | 5022 | CB | PRO | B | 372 | 17.576 | 36.435 | 106.861 | 1.00 | 15.38 | B | C |
| ATOM | 5023 | CG | PRO | B | 372 | 16.117 | 36.187 | 106.788 | 1.00 | 15.38 | B | C |
| ATOM | 5024 | C | PRO | B | 372 | 18.989 | 38.493 | 106.607 | 1.00 | 29.97 | B | C |
| ATOM | 5025 | O | PRO | B | 372 | 19.922 | 38.557 | 107.403 | 1.00 | 29.97 | B | O |
| ATOM | 5026 | N | LEU | B | 373 | 19.101 | 38.878 | 105.344 | 1.00 | 34.38 | B | N |
| ATOM | 5027 | CA | LEU | B | 373 | 20.357 | 39.405 | 104.838 | 1.00 | 34.38 | B | C |
| ATOM | 5028 | CB | LEU | B | 373 | 20.293 | 39.522 | 103.315 | 1.00 | 52.52 | B | C |
| ATOM | 5029 | CG | LEU | B | 373 | 21.137 | 38.498 | 102.542 | 1.00 | 52.52 | B | C |
| ATOM | 5030 | CD1 | LEU | B | 373 | 20.564 | 37.111 | 102.717 | 1.00 | 52.52 | B | C |
| ATOM | 5031 | CD2 | LEU | B | 373 | 21.162 | 38.877 | 101.072 | 1.00 | 52.52 | B | C |
| ATOM | 5032 | C | LEU | B | 373 | 20.758 | 40.748 | 105.455 | 1.00 | 34.38 | B | C |
| ATOM | 5033 | O | LEU | B | 373 | 21.912 | 41.171 | 105.340 | 1.00 | 34.38 | B | O |
| ATOM | 5034 | N | ALA | B | 374 | 19.821 | 41.419 | 106.114 | 1.00 | 27.92 | B | N |

| | | | | | | | | | | | | |
|---|---|---|---|---|---|---|---|---|---|---|---|---|
| ATOM | 5035 | CA | ALA | B | 374 | 20.144 | 42.703 | 106.726 | 1.00 | 27.92 | B | C |
| ATOM | 5036 | CB | ALA | B | 374 | 18.923 | 43.266 | 107.443 | 1.00 | 14.14 | B | C |
| ATOM | 5037 | C | ALA | B | 374 | 21.300 | 42.564 | 107.716 | 1.00 | 27.92 | B | C |
| ATOM | 5038 | O | ALA | B | 374 | 22.007 | 43.530 | 107.995 | 1.00 | 27.92 | B | O |
| ATOM | 5039 | N | THR | B | 375 | 21.492 | 41.357 | 108.242 | 1.00 | 30.95 | B | N |
| ATOM | 5040 | CA | THR | B | 375 | 22.555 | 41.136 | 109.214 | 1.00 | 30.95 | B | C |
| ATOM | 5041 | CB | THR | B | 375 | 22.539 | 39.719 | 109.794 | 1.00 | 35.18 | B | C |
| ATOM | 5042 | OG1 | THR | B | 375 | 21.202 | 39.374 | 110.154 | 1.00 | 35.18 | B | O |
| ATOM | 5043 | CG2 | THR | B | 375 | 23.388 | 39.654 | 111.057 | 1.00 | 35.18 | B | C |
| ATOM | 5044 | C | THR | B | 375 | 23.901 | 41.396 | 108.564 | 1.00 | 30.95 | B | C |
| ATOM | 5045 | O | THR | B | 375 | 24.826 | 41.894 | 109.207 | 1.00 | 30.95 | B | O |
| ATOM | 5046 | N | ILE | B | 376 | 24.005 | 41.062 | 107.283 | 1.00 | 37.43 | B | N |
| ATOM | 5047 | CA | ILE | B | 376 | 25.237 | 41.269 | 106.540 | 1.00 | 37.43 | B | C |
| ATOM | 5048 | CB | ILE | B | 376 | 25.453 | 40.209 | 105.481 | 1.00 | 29.24 | B | C |
| ATOM | 5049 | CG2 | ILE | B | 376 | 26.764 | 40.473 | 104.774 | 1.00 | 29.24 | B | C |
| ATOM | 5050 | CG1 | ILE | B | 376 | 25.446 | 38.823 | 106.114 | 1.00 | 29.24 | B | C |
| ATOM | 5051 | CD1 | ILE | B | 376 | 25.556 | 37.706 | 105.095 | 1.00 | 29.24 | B | C |
| ATOM | 5052 | C | ILE | B | 376 | 25.221 | 42.608 | 105.819 | 1.00 | 37.43 | B | C |
| ATOM | 5053 | O | ILE | B | 376 | 26.196 | 43.356 | 105.867 | 1.00 | 37.43 | B | O |
| ATOM | 5054 | N | LEU | B | 377 | 24.111 | 42.914 | 105.153 | 1.00 | 17.91 | B | N |
| ATOM | 5055 | CA | LEU | B | 377 | 24.013 | 44.162 | 104.419 | 1.00 | 17.91 | B | C |
| ATOM | 5056 | CB | LEU | B | 377 | 22.671 | 44.244 | 103.684 | 1.00 | 18.81 | B | C |
| ATOM | 5057 | CG | LEU | B | 377 | 22.554 | 43.487 | 102.350 | 1.00 | 18.81 | B | C |
| ATOM | 5058 | CD1 | LEU | B | 377 | 23.899 | 43.475 | 101.634 | 1.00 | 18.81 | B | C |
| ATOM | 5059 | CD2 | LEU | B | 377 | 22.097 | 42.079 | 102.593 | 1.00 | 18.81 | B | C |
| ATOM | 5060 | C | LEU | B | 377 | 24.253 | 45.440 | 105.233 | 1.00 | 17.91 | B | C |
| ATOM | 5061 | O | LEU | B | 377 | 24.903 | 46.371 | 104.755 | 1.00 | 17.91 | B | O |
| ATOM | 5062 | N | ILE | B | 378 | 23.732 | 45.496 | 106.457 | 1.00 | 26.15 | B | N |
| ATOM | 5063 | CA | ILE | B | 378 | 23.919 | 46.679 | 107.302 | 1.00 | 26.15 | B | C |
| ATOM | 5064 | CB | ILE | B | 378 | 22.626 | 47.112 | 108.016 | 1.00 | 17.20 | B | C |
| ATOM | 5065 | CG2 | ILE | B | 378 | 22.877 | 48.400 | 108.792 | 1.00 | 17.20 | B | C |
| ATOM | 5066 | CG1 | ILE | B | 378 | 21.522 | 47.386 | 107.002 | 1.00 | 17.20 | B | C |
| ATOM | 5067 | CD1 | ILE | B | 378 | 20.200 | 47.649 | 107.636 | 1.00 | 17.20 | B | C |
| ATOM | 5068 | C | ILE | B | 378 | 24.978 | 46.389 | 108.348 | 1.00 | 26.15 | B | C |
| ATOM | 5069 | O | ILE | B | 378 | 24.701 | 45.763 | 109.364 | 1.00 | 26.15 | B | O |
| ATOM | 5070 | N | PRO | B | 379 | 26.215 | 46.857 | 108.111 | 1.00 | 33.94 | B | N |
| ATOM | 5071 | CD | PRO | B | 379 | 26.638 | 47.720 | 106.997 | 1.00 | 27.24 | B | C |
| ATOM | 5072 | CA | PRO | B | 379 | 27.337 | 46.643 | 109.023 | 1.00 | 33.94 | B | C |
| ATOM | 5073 | CB | PRO | B | 379 | 28.503 | 47.287 | 108.276 | 1.00 | 27.24 | B | C |
| ATOM | 5074 | CG | PRO | B | 379 | 27.853 | 48.403 | 107.575 | 1.00 | 27.24 | B | C |
| ATOM | 5075 | C | PRO | B | 379 | 27.142 | 47.228 | 110.419 | 1.00 | 33.94 | B | C |
| ATOM | 5076 | O | PRO | B | 379 | 26.520 | 48.275 | 110.594 | 1.00 | 33.94 | B | O |
| ATOM | 5077 | N | PRO | B | 380 | 27.709 | 46.560 | 111.430 | 1.00 | 33.97 | B | N |
| ATOM | 5078 | CD | PRO | B | 380 | 28.618 | 45.414 | 111.243 | 1.00 | 32.45 | B | C |
| ATOM | 5079 | CA | PRO | B | 380 | 27.641 | 46.935 | 112.841 | 1.00 | 33.97 | B | C |
| ATOM | 5080 | CB | PRO | B | 380 | 28.766 | 46.120 | 113.471 | 1.00 | 32.45 | B | C |
| ATOM | 5081 | CG | PRO | B | 380 | 28.781 | 44.888 | 112.657 | 1.00 | 32.45 | B | C |
| ATOM | 5082 | C | PRO | B | 380 | 27.796 | 48.415 | 113.152 | 1.00 | 33.97 | B | C |
| ATOM | 5083 | O | PRO | B | 380 | 27.334 | 48.876 | 114.189 | 1.00 | 33.97 | B | O |
| ATOM | 5084 | N | HIS | B | 381 | 28.444 | 49.157 | 112.267 | 1.00 | 33.16 | B | N |
| ATOM | 5085 | CA | HIS | B | 381 | 28.676 | 50.571 | 112.519 | 1.00 | 33.16 | B | C |
| ATOM | 5086 | CB | HIS | B | 381 | 30.126 | 50.917 | 112.172 | 1.00 | 25.36 | B | C |
| ATOM | 5087 | CG | HIS | B | 381 | 30.453 | 50.774 | 110.718 | 1.00 | 25.36 | B | C |
| ATOM | 5088 | CD2 | HIS | B | 381 | 30.827 | 49.697 | 109.990 | 1.00 | 25.36 | B | C |
| ATOM | 5089 | ND1 | HIS | B | 381 | 30.376 | 51.826 | 109.830 | 1.00 | 25.36 | B | N |
| ATOM | 5090 | CE1 | HIS | B | 381 | 30.689 | 51.404 | 108.620 | 1.00 | 25.36 | B | C |
| ATOM | 5091 | NE2 | HIS | B | 381 | 30.967 | 50.114 | 108.689 | 1.00 | 25.36 | B | N |
| ATOM | 5092 | C | HIS | B | 381 | 27.751 | 51.543 | 111.793 | 1.00 | 33.16 | B | C |
| ATOM | 5093 | O | HIS | B | 381 | 27.703 | 52.716 | 112.138 | 1.00 | 33.16 | B | O |
| ATOM | 5094 | N | ALA | B | 382 | 27.002 | 51.077 | 110.804 | 1.00 | 34.55 | B | N |
| ATOM | 5095 | CA | ALA | B | 382 | 26.137 | 51.982 | 110.047 | 1.00 | 34.55 | B | C |
| ATOM | 5096 | CB | ALA | B | 382 | 25.826 | 51.367 | 108.699 | 1.00 | 51.30 | B | C |
| ATOM | 5097 | C | ALA | B | 382 | 24.837 | 52.404 | 110.742 | 1.00 | 34.55 | B | C |

| | | | | | | | | | | | | |
|---|---|---|---|---|---|---|---|---|---|---|---|---|
| ATOM | 5098 | O | ALA | B | 382 | 24.687 | 53.616 | 110.995 | 1.00 | 34.55 | B | O |
| ATOM | 5099 | OXT | ALA | B | 382 | 23.975 | 51.543 | 111.022 | 1.00 | 51.30 | B | O |
| TER | 5100 | | ALA | B | 382 | | | | | | B | |
| ATOM | 5101 | O | HOH | W | 1 | 6.884 | 43.261 | 76.228 | 1.00 | 4.71 | W | O |
| ATOM | 5102 | O | HOH | W | 2 | 13.671 | 71.230 | 75.371 | 1.00 | 26.58 | W | O |
| ATOM | 5103 | O | HOH | W | 3 | 24.031 | 52.355 | 97.568 | 1.00 | 18.47 | W | O |
| ATOM | 5104 | O | HOH | W | 4 | 21.920 | 45.607 | 77.747 | 1.00 | 21.56 | W | O |
| ATOM | 5105 | O | HOH | W | 5 | 14.844 | 49.877 | 80.739 | 1.00 | 27.44 | W | O |
| ATOM | 5106 | O | HOH | W | 6 | 12.108 | 34.233 | 55.498 | 1.00 | 10.13 | W | O |
| ATOM | 5107 | O | HOH | W | 7 | 30.003 | 40.262 | 77.662 | 1.00 | 35.85 | W | O |
| ATOM | 5108 | O | HOH | W | 8 | 19.970 | 39.689 | 79.413 | 1.00 | 17.86 | W | O |
| ATOM | 5109 | O | HOH | W | 9 | 18.645 | 38.017 | 57.432 | 1.00 | 20.91 | W | O |
| ATOM | 5110 | O | HOH | W | 10 | 9.591 | 53.585 | 97.286 | 1.00 | 6.30 | W | O |
| ATOM | 5111 | O | HOH | W | 11 | 13.130 | 31.272 | 80.326 | 1.00 | 25.36 | W | O |
| ATOM | 5112 | O | HOH | W | 12 | 26.690 | 45.855 | 102.976 | 1.00 | 30.90 | W | O |
| ATOM | 5113 | O | HOH | W | 13 | 4.791 | 33.157 | 61.302 | 1.00 | 40.39 | W | O |
| ATOM | 5114 | O | HOH | W | 14 | 39.534 | 46.733 | 87.188 | 1.00 | 25.98 | W | O |
| ATOM | 5115 | O | HOH | W | 15 | 14.615 | 57.255 | 92.346 | 1.00 | 22.22 | W | O |
| ATOM | 5116 | O | HOH | W | 16 | 31.695 | 51.737 | 105.400 | 1.00 | 25.68 | W | O |
| ATOM | 5117 | O | HOH | W | 17 | 26.193 | 37.668 | 77.252 | 1.00 | 18.51 | W | O |
| ATOM | 5118 | O | HOH | W | 18 | 20.267 | 43.312 | 62.793 | 1.00 | 33.57 | W | O |
| ATOM | 5119 | O | HOH | W | 19 | 18.709 | 60.142 | 91.276 | 1.00 | 28.86 | W | O |
| ATOM | 5120 | O | HOH | W | 20 | 25.039 | 55.741 | 96.972 | 1.00 | 19.09 | W | O |
| ATOM | 5121 | O | HOH | W | 21 | 12.205 | 58.941 | 89.719 | 1.00 | 20.06 | W | O |
| ATOM | 5122 | O | HOH | W | 22 | 19.044 | 43.587 | 81.053 | 1.00 | 30.63 | W | O |
| ATOM | 5123 | O | HOH | W | 23 | 23.292 | 26.709 | 85.852 | 1.00 | 37.91 | W | O |
| ATOM | 5124 | O | HOH | W | 24 | 24.830 | 49.824 | 73.410 | 1.00 | 35.22 | W | O |
| ATOM | 5125 | O | HOH | W | 25 | 22.858 | 22.020 | 56.688 | 1.00 | 23.79 | W | O |
| ATOM | 5126 | O | HOH | W | 26 | 9.077 | 54.896 | 76.657 | 1.00 | 23.67 | W | O |
| ATOM | 5127 | O | HOH | W | 27 | 2.986 | 60.035 | 77.857 | 1.00 | 36.42 | W | O |
| ATOM | 5128 | O | HOH | W | 28 | 10.147 | 36.275 | 69.986 | 1.00 | 31.72 | W | O |
| ATOM | 5129 | O | HOH | W | 29 | 3.329 | 71.512 | 92.548 | 1.00 | 22.56 | W | O |
| ATOM | 5130 | O | HOH | W | 30 | 22.205 | 36.195 | 55.588 | 1.00 | 29.04 | W | O |
| ATOM | 5131 | O | HOH | W | 31 | 36.733 | 59.106 | 90.404 | 1.00 | 29.08 | W | O |
| ATOM | 5132 | O | HOH | W | 32 | 30.254 | 65.353 | 78.598 | 1.00 | 32.14 | W | O |
| ATOM | 5133 | O | HOH | W | 33 | 10.344 | 49.830 | 102.685 | 1.00 | 24.89 | W | O |
| ATOM | 5134 | O | HOH | W | 34 | 30.323 | 22.013 | 38.917 | 1.00 | 38.50 | W | O |
| ATOM | 5135 | O | HOH | W | 35 | 55.593 | 18.422 | 47.168 | 1.00 | 22.50 | W | O |
| ATOM | 5136 | O | HOH | W | 36 | 16.081 | 33.453 | 43.021 | 1.00 | 21.23 | W | O |
| ATOM | 5137 | O | HOH | W | 37 | 39.507 | 29.603 | 40.903 | 1.00 | 26.28 | W | O |
| ATOM | 5138 | O | HOH | W | 38 | 27.976 | 43.384 | 108.440 | 1.00 | 26.60 | W | O |
| ATOM | 5139 | O | HOH | W | 39 | 15.808 | 27.157 | 34.475 | 1.00 | 43.88 | W | O |
| ATOM | 5140 | O | HOH | W | 40 | 12.584 | 42.992 | 104.279 | 1.00 | 46.10 | W | O |
| ATOM | 5141 | O | HOH | W | 41 | 34.804 | 47.633 | 78.254 | 1.00 | 32.79 | W | O |
| ATOM | 5142 | O | HOH | W | 42 | 27.642 | 34.834 | 62.584 | 1.00 | 24.77 | W | O |
| ATOM | 5143 | O | HOH | W | 43 | 36.584 | 62.345 | 45.774 | 1.00 | 24.67 | W | O |
| ATOM | 5144 | O | HOH | W | 44 | 41.906 | 7.567 | 49.764 | 1.00 | 40.25 | W | O |
| ATOM | 5145 | O | HOH | W | 45 | 26.998 | 50.322 | 77.314 | 1.00 | 38.44 | W | O |
| ATOM | 5146 | O | HOH | W | 46 | 26.508 | 66.828 | 79.027 | 1.00 | 24.04 | W | O |
| ATOM | 5147 | O | HOH | W | 47 | 27.119 | 22.719 | 81.301 | 1.00 | 43.04 | W | O |
| ATOM | 5148 | O | HOH | W | 48 | 18.611 | 44.709 | 59.301 | 1.00 | 24.30 | W | O |
| ATOM | 5149 | O | HOH | W | 49 | 36.817 | 31.604 | 44.199 | 1.00 | 45.47 | W | O |
| ATOM | 5150 | O | HOH | W | 50 | 42.993 | 58.011 | 98.756 | 1.00 | 39.71 | W | O |
| ATOM | 5151 | O | HOH | W | 51 | 23.099 | 40.340 | 70.072 | 1.00 | 32.55 | W | O |
| ATOM | 5152 | O | HOH | W | 52 | 28.783 | 51.727 | 53.480 | 1.00 | 23.57 | W | O |
| ATOM | 5153 | O | HOH | W | 53 | 36.426 | 48.130 | 81.024 | 1.00 | 27.94 | W | O |
| ATOM | 5154 | O | HOH | W | 54 | 27.319 | 23.187 | 40.149 | 1.00 | 46.42 | W | O |
| ATOM | 5155 | O | HOH | W | 55 | 16.054 | 39.188 | 91.189 | 1.00 | 27.75 | W | O |
| ATOM | 5156 | O | HOH | W | 56 | 16.909 | 41.384 | 68.972 | 1.00 | 37.02 | W | O |
| ATOM | 5157 | O | HOH | W | 57 | 27.355 | 43.934 | 62.307 | 1.00 | 30.09 | W | O |
| ATOM | 5158 | O | HOH | W | 58 | 21.903 | 53.438 | 73.576 | 1.00 | 46.74 | W | O |
| ATOM | 5159 | O | HOH | W | 59 | 26.606 | 51.934 | 75.017 | 1.00 | 32.05 | W | O |
| ATOM | 5160 | O | HOH | W | 60 | 27.600 | 31.159 | 56.303 | 1.00 | 34.50 | W | O |

| | | | | | | | | | | | | |
|---|---|---|---|---|---|---|---|---|---|---|---|---|
| ATOM | 5161 | O | HOH | W | 61 | 23.136 | 48.022 | 56.078 | 1.00 | 18.85 | W | O |
| ATOM | 5162 | O | HOH | W | 62 | 29.544 | 45.688 | 57.642 | 1.00 | 44.18 | W | O |
| ATOM | 5163 | O | HOH | W | 63 | 29.332 | 61.464 | 75.261 | 1.00 | 37.80 | W | O |
| ATOM | 5164 | O | HOH | W | 64 | 30.900 | 49.454 | 77.309 | 1.00 | 57.04 | W | O |
| ATOM | 5165 | O | HOH | W | 65 | 6.078 | 33.390 | 80.922 | 1.00 | 38.03 | W | O |
| ATOM | 5166 | O | HOH | W | 66 | 40.212 | 20.720 | 43.696 | 1.00 | 27.79 | W | O |
| ATOM | 5167 | O | HOH | W | 67 | 21.894 | 51.741 | 69.426 | 1.00 | 32.99 | W | O |
| ATOM | 5168 | O | HOH | W | 68 | 21.749 | 42.402 | 69.793 | 1.00 | 17.20 | W | O |
| ATOM | 5169 | O | HOH | W | 69 | 6.889 | 34.009 | 54.593 | 1.00 | 26.05 | W | O |
| ATOM | 5170 | O | HOH | W | 70 | 20.193 | 47.911 | 29.110 | 1.00 | 18.73 | W | O |
| ATOM | 5171 | O | HOH | W | 71 | 9.706 | 30.727 | 41.672 | 1.00 | 38.56 | W | O |
| ATOM | 5172 | O | HOH | W | 72 | 35.643 | 43.555 | 99.767 | 1.00 | 56.19 | W | O |
| ATOM | 5173 | O | HOH | W | 73 | 11.363 | 59.321 | 69.910 | 1.00 | 23.40 | W | O |
| ATOM | 5174 | O | HOH | W | 74 | 36.659 | 36.969 | 76.675 | 1.00 | 37.35 | W | O |
| ATOM | 5175 | O | HOH | W | 75 | 11.880 | 43.841 | 38.931 | 1.00 | 24.39 | W | O |
| ATOM | 5176 | O | HOH | W | 76 | 42.132 | 52.296 | 112.553 | 1.00 | 36.58 | W | O |
| ATOM | 5177 | O | HOH | W | 77 | 28.105 | 67.017 | 88.993 | 1.00 | 66.30 | W | O |
| ATOM | 5178 | O | HOH | W | 78 | 8.451 | 37.166 | 44.627 | 1.00 | 47.12 | W | O |
| ATOM | 5179 | O | HOH | W | 79 | 5.468 | 42.975 | 53.416 | 1.00 | 29.78 | W | O |
| ATOM | 5180 | O | HOH | W | 80 | 16.235 | 28.112 | 48.850 | 1.00 | 27.07 | W | O |
| ATOM | 5181 | O | HOH | W | 81 | 10.940 | 34.131 | 40.697 | 1.00 | 29.39 | W | O |
| ATOM | 5182 | O | HOH | W | 82 | 28.730 | 48.582 | 73.292 | 1.00 | 49.76 | W | O |
| ATOM | 5183 | O | HOH | W | 83 | 16.724 | 50.571 | 77.535 | 1.00 | 24.75 | W | O |
| ATOM | 5184 | O | HOH | W | 84 | 32.742 | 37.557 | 68.570 | 1.00 | 29.09 | W | O |
| ATOM | 5185 | O | HOH | W | 85 | 17.738 | 63.594 | 93.861 | 1.00 | 36.19 | W | O |
| ATOM | 5186 | O | HOH | W | 86 | 28.035 | 37.039 | 37.905 | 1.00 | 36.51 | W | O |
| ATOM | 5187 | O | HOH | W | 87 | 26.123 | 57.217 | 77.087 | 1.00 | 47.19 | W | O |
| ATOM | 5188 | O | HOH | W | 88 | 44.377 | 59.550 | 94.892 | 1.00 | 27.17 | W | O |
| ATOM | 5189 | O | HOH | W | 89 | 19.999 | 53.082 | 75.659 | 1.00 | 61.91 | W | O |
| ATOM | 5190 | O | HOH | W | 90 | 49.638 | 29.061 | 39.391 | 1.00 | 32.08 | W | O |
| ATOM | 5191 | O | HOH | W | 91 | 5.802 | 44.337 | 97.832 | 1.00 | 46.90 | W | O |
| ATOM | 5192 | O | HOH | W | 92 | 6.229 | 48.186 | 98.929 | 1.00 | 35.44 | W | O |
| ATOM | 5193 | O | HOH | W | 93 | 25.587 | 59.175 | 99.589 | 1.00 | 34.16 | W | O |
| ATOM | 5194 | O | HOH | W | 94 | 41.158 | 56.390 | 71.372 | 1.00 | 29.53 | W | O |
| ATOM | 5195 | O | HOH | W | 95 | 21.618 | 38.349 | 70.855 | 1.00 | 39.71 | W | O |
| ATOM | 5196 | O | HOH | W | 96 | 2.765 | 40.979 | 79.475 | 1.00 | 21.32 | W | O |
| ATOM | 5197 | O | HOH | W | 97 | 12.805 | 57.569 | 66.310 | 1.00 | 37.38 | W | O |
| ATOM | 5198 | O | HOH | W | 98 | 17.056 | 57.174 | 66.071 | 1.00 | 42.50 | W | O |
| ATOM | 5199 | O | HOH | W | 99 | 36.884 | 43.052 | 55.832 | 1.00 | 16.23 | W | O |
| TER | 5200 | | HOH | W | 99 | | | | | | W | |
| ATOM | 5201 | C1 | 12A | I | 1 | 41.595 | 41.642 | 53.375 | 1.00 | 38.54 | I | C |
| ATOM | 5202 | C2 | 12A | I | 1 | 40.567 | 41.087 | 54.312 | 1.00 | 38.54 | I | C |
| ATOM | 5203 | C3 | 12A | I | 1 | 40.191 | 39.658 | 54.166 | 1.00 | 38.54 | I | C |
| ATOM | 5204 | C4 | 12A | I | 1 | 40.858 | 38.816 | 53.052 | 1.00 | 38.54 | I | C |
| ATOM | 5205 | C5 | 12A | I | 1 | 41.871 | 39.344 | 52.194 | 1.00 | 38.54 | I | C |
| ATOM | 5206 | C6 | 12A | I | 1 | 42.246 | 40.759 | 52.344 | 1.00 | 38.54 | I | C |
| ATOM | 5207 | N11 | 12A | I | 1 | 39.180 | 39.128 | 55.015 | 1.00 | 38.54 | I | N |
| ATOM | 5208 | C12 | 12A | I | 1 | 38.740 | 37.849 | 54.862 | 1.00 | 38.54 | I | C |
| ATOM | 5209 | N13 | 12A | I | 1 | 39.484 | 37.165 | 53.967 | 1.00 | 38.54 | I | N |
| ATOM | 5210 | C14 | 12A | I | 1 | 40.403 | 37.531 | 52.980 | 1.00 | 38.54 | I | C |
| ATOM | 5211 | N15 | 12A | I | 1 | 40.873 | 36.636 | 52.051 | 1.00 | 38.54 | I | N |
| ATOM | 5212 | C16 | 12A | I | 1 | 40.172 | 35.463 | 51.474 | 1.00 | 38.54 | I | C |
| ATOM | 5213 | C17 | 12A | I | 1 | 39.277 | 34.574 | 52.246 | 1.00 | 38.54 | I | C |
| ATOM | 5214 | C18 | 12A | I | 1 | 38.794 | 33.517 | 51.324 | 1.00 | 38.54 | I | C |
| ATOM | 5215 | N19 | 12A | I | 1 | 39.448 | 33.831 | 50.182 | 1.00 | 38.54 | I | N |
| ATOM | 5216 | N20 | 12A | I | 1 | 40.229 | 34.904 | 50.284 | 1.00 | 38.54 | I | N |
| ATOM | 5217 | C21 | 12A | I | 1 | 38.831 | 34.481 | 53.660 | 1.00 | 38.54 | I | C |
| ATOM | 5218 | C22 | 12A | I | 1 | 37.788 | 33.621 | 54.155 | 1.00 | 38.54 | I | C |
| ATOM | 5219 | C23 | 12A | I | 1 | 37.324 | 32.564 | 53.200 | 1.00 | 38.54 | I | C |
| ATOM | 5220 | C24 | 12A | I | 1 | 37.839 | 32.487 | 51.796 | 1.00 | 38.54 | I | C |
| ATOM | 5221 | C29 | 12A | I | 1 | 37.817 | 37.229 | 55.744 | 1.00 | 38.54 | I | C |
| ATOM | 5222 | C30 | 12A | I | 1 | 38.134 | 36.851 | 57.114 | 1.00 | 38.54 | I | C |
| ATOM | 5223 | C31 | 12A | I | 1 | 37.009 | 36.379 | 57.992 | 1.00 | 38.54 | I | C |

| | | | | | | | | | | | | |
|---|---|---|---|---|---|---|---|---|---|---|---|---|
| ATOM | 5224 | C32 | 12A | I | 1 | 35.643 | 36.244 | 57.450 | 1.00 | 38.54 | I | C |
| ATOM | 5225 | C33 | 12A | I | 1 | 35.372 | 36.591 | 56.012 | 1.00 | 38.54 | I | C |
| ATOM | 5226 | C34 | 12A | I | 1 | 36.501 | 37.087 | 55.181 | 1.00 | 38.54 | I | C |
| ATOM | 5227 | C39 | 12A | I | 1 | 39.518 | 36.900 | 57.738 | 1.00 | 38.54 | I | C |
| ATOM | 5228 | F40 | 12A | I | 1 | 39.832 | 35.670 | 58.027 | 1.00 | 38.54 | I | F |
| ATOM | 5229 | F41 | 12A | I | 1 | 39.470 | 37.659 | 58.853 | 1.00 | 38.54 | I | F |
| ATOM | 5230 | F42 | 12A | I | 1 | 40.508 | 37.437 | 56.949 | 1.00 | 38.54 | I | F |
| TER | 5231 | | 12A | I | 1 | | | | | | I | |
| ATOM | 5232 | C1 | 12B | J | 1 | 28.164 | 61.927 | 79.557 | 1.00 | 38.48 | J | C |
| ATOM | 5233 | C2 | 12B | J | 1 | 27.275 | 60.912 | 78.898 | 1.00 | 38.48 | J | C |
| ATOM | 5234 | C3 | 12B | J | 1 | 25.827 | 60.876 | 79.289 | 1.00 | 38.48 | J | C |
| ATOM | 5235 | C4 | 12B | J | 1 | 25.300 | 61.898 | 80.352 | 1.00 | 38.48 | J | C |
| ATOM | 5236 | C5 | 12B | J | 1 | 26.153 | 62.897 | 80.970 | 1.00 | 38.48 | J | C |
| ATOM | 5237 | C6 | 12B | J | 1 | 27.583 | 62.920 | 80.543 | 1.00 | 38.48 | J | C |
| ATOM | 5238 | N11 | 12B | J | 1 | 24.996 | 59.871 | 78.752 | 1.00 | 38.48 | J | N |
| ATOM | 5239 | C12 | 12B | J | 1 | 23.721 | 59.743 | 79.164 | 1.00 | 38.48 | J | C |
| ATOM | 5240 | N13 | 12B | J | 1 | 23.313 | 60.777 | 79.957 | 1.00 | 38.48 | J | N |
| ATOM | 5241 | C14 | 12B | J | 1 | 23.972 | 61.744 | 80.661 | 1.00 | 38.48 | J | C |
| ATOM | 5242 | N15 | 12B | J | 1 | 23.333 | 62.535 | 81.572 | 1.00 | 38.48 | J | N |
| ATOM | 5243 | C16 | 12B | J | 1 | 22.227 | 62.185 | 82.473 | 1.00 | 38.48 | J | C |
| ATOM | 5244 | C17 | 12B | J | 1 | 21.052 | 61.421 | 82.024 | 1.00 | 38.48 | J | C |
| ATOM | 5245 | C18 | 12B | J | 1 | 20.124 | 61.295 | 83.172 | 1.00 | 38.48 | J | C |
| ATOM | 5246 | N19 | 12B | J | 1 | 20.804 | 61.972 | 84.134 | 1.00 | 38.48 | J | N |
| ATOM | 5247 | N20 | 12B | J | 1 | 21.979 | 62.488 | 83.726 | 1.00 | 38.48 | J | N |
| ATOM | 5248 | C21 | 12B | J | 1 | 20.592 | 60.821 | 80.721 | 1.00 | 38.48 | J | C |
| ATOM | 5249 | C22 | 12B | J | 1 | 19.403 | 60.017 | 80.537 | 1.00 | 38.48 | J | C |
| ATOM | 5250 | C23 | 12B | J | 1 | 18.503 | 59.923 | 81.721 | 1.00 | 38.48 | J | C |
| ATOM | 5251 | C24 | 12B | J | 1 | 18.840 | 60.558 | 83.033 | 1.00 | 38.48 | J | C |
| ATOM | 5252 | C29 | 12B | J | 1 | 22.808 | 58.858 | 78.496 | 1.00 | 38.48 | J | C |
| ATOM | 5253 | C30 | 12B | J | 1 | 22.261 | 59.070 | 77.113 | 1.00 | 38.48 | J | C |
| ATOM | 5254 | C31 | 12B | J | 1 | 21.446 | 57.955 | 76.507 | 1.00 | 38.48 | J | C |
| ATOM | 5255 | C32 | 12B | J | 1 | 21.117 | 56.757 | 77.302 | 1.00 | 38.48 | J | C |
| ATOM | 5256 | C33 | 12B | J | 1 | 21.638 | 56.637 | 78.706 | 1.00 | 38.48 | J | C |
| ATOM | 5257 | C34 | 12B | J | 1 | 22.496 | 57.711 | 79.287 | 1.00 | 38.48 | J | C |
| ATOM | 5258 | C39 | 12B | J | 1 | 22.493 | 60.360 | 76.292 | 1.00 | 38.48 | J | C |
| ATOM | 5259 | F40 | 12B | J | 1 | 23.050 | 61.351 | 76.922 | 1.00 | 38.48 | J | F |
| ATOM | 5260 | F41 | 12B | J | 1 | 21.334 | 60.883 | 75.995 | 1.00 | 38.48 | J | F |
| ATOM | 5261 | F42 | 12B | J | 1 | 23.275 | 60.056 | 75.224 | 1.00 | 38.48 | J | F |
| TER | 5262 | | 12B | J | 1 | | | | | | J | |
| END | | | | | | | | | | | | |

# FIG. 7

| | Atom Type | Resid | | # | X | Y | Z | OCC | B | |
|---|---|---|---|---|---|---|---|---|---|---|
| ATOM | 1 | CB | VAL A | 37 | 1.918 | 33.912 | 12.559 | 1.00 | 42.12 | C |
| ATOM | 2 | CG1 | VAL A | 37 | 2.177 | 34.532 | 13.927 | 1.00 | 42.12 | C |
| ATOM | 3 | CG2 | VAL A | 37 | 2.837 | 34.529 | 11.500 | 1.00 | 42.12 | C |
| ATOM | 4 | C | VAL A | 37 | 1.443 | 31.855 | 13.860 | 1.00 | 53.45 | C |
| ATOM | 5 | O | VAL A | 37 | 0.232 | 31.634 | 13.845 | 1.00 | 53.45 | O |
| ATOM | 6 | N | VAL A | 37 | 1.733 | 31.713 | 11.355 | 1.00 | 53.45 | N |
| ATOM | 7 | CA | VAL A | 37 | 2.155 | 32.392 | 12.623 | 1.00 | 53.45 | C |
| ATOM | 8 | N | THR A | 38 | 2.202 | 31.664 | 14.936 | 1.00 | 52.22 | N |
| ATOM | 9 | CA | THR A | 38 | 1.640 | 31.130 | 16.171 | 1.00 | 52.22 | C |
| ATOM | 10 | CB | THR A | 38 | 2.243 | 29.757 | 16.518 | 1.00 | 59.61 | C |
| ATOM | 11 | OG1 | THR A | 38 | 2.046 | 28.857 | 15.422 | 1.00 | 59.61 | O |
| ATOM | 12 | CG2 | THR A | 38 | 1.573 | 29.175 | 17.756 | 1.00 | 59.61 | C |
| ATOM | 13 | C | THR A | 38 | 1.858 | 32.047 | 17.352 | 1.00 | 52.22 | C |
| ATOM | 14 | O | THR A | 38 | 2.901 | 32.693 | 17.464 | 1.00 | 52.22 | O |
| ATOM | 15 | N | THR A | 39 | 0.862 | 32.090 | 18.233 | 1.00 | 39.26 | N |
| ATOM | 16 | CA | THR A | 39 | 0.929 | 32.929 | 19.427 | 1.00 | 39.26 | C |
| ATOM | 17 | CB | THR A | 39 | 0.003 | 34.152 | 19.303 | 1.00 | 43.32 | C |
| ATOM | 18 | OG1 | THR A | 39 | 0.351 | 34.890 | 18.127 | 1.00 | 43.32 | O |
| ATOM | 19 | CG2 | THR A | 39 | 0.153 | 35.062 | 20.510 | 1.00 | 43.32 | C |
| ATOM | 20 | C | THR A | 39 | 0.550 | 32.143 | 20.678 | 1.00 | 39.26 | C |
| ATOM | 21 | O | THR A | 39 | -0.407 | 31.381 | 20.671 | 1.00 | 39.26 | O |
| ATOM | 22 | N | VAL A | 40 | 1.318 | 32.323 | 21.747 | 1.00 | 35.33 | N |
| ATOM | 23 | CA | VAL A | 40 | 1.054 | 31.623 | 22.994 | 1.00 | 35.33 | C |
| ATOM | 24 | CB | VAL A | 40 | 1.896 | 30.317 | 23.113 | 1.00 | 33.55 | C |
| ATOM | 25 | CG1 | VAL A | 40 | 1.580 | 29.381 | 21.969 | 1.00 | 33.55 | C |
| ATOM | 26 | CG2 | VAL A | 40 | 3.376 | 30.647 | 23.126 | 1.00 | 33.55 | C |
| ATOM | 27 | C | VAL A | 40 | 1.377 | 32.500 | 24.193 | 1.00 | 35.33 | C |
| ATOM | 28 | O | VAL A | 40 | 1.861 | 33.624 | 24.057 | 1.00 | 35.33 | O |
| ATOM | 29 | N | VAL A | 41 | 1.084 | 31.980 | 25.373 | 1.00 | 39.77 | N |
| ATOM | 30 | CA | VAL A | 41 | 1.381 | 32.697 | 26.593 | 1.00 | 39.77 | C |
| ATOM | 31 | CB | VAL A | 41 | 0.147 | 32.807 | 27.510 | 1.00 | 31.47 | C |
| ATOM | 32 | CG1 | VAL A | 41 | 0.518 | 33.580 | 28.763 | 1.00 | 31.47 | C |
| ATOM | 33 | CG2 | VAL A | 41 | -1.010 | 33.453 | 26.756 | 1.00 | 31.47 | C |
| ATOM | 34 | C | VAL A | 41 | 2.435 | 31.827 | 27.252 | 1.00 | 39.77 | C |
| ATOM | 35 | O | VAL A | 41 | 2.147 | 30.718 | 27.723 | 1.00 | 39.77 | O |
| ATOM | 36 | N | ALA A | 42 | 3.664 | 32.324 | 27.260 | 1.00 | 37.34 | N |
| ATOM | 37 | CA | ALA A | 42 | 4.767 | 31.568 | 27.830 | 1.00 | 37.34 | C |
| ATOM | 38 | CB | ALA A | 42 | 5.815 | 31.290 | 26.734 | 1.00 | 21.39 | C |
| ATOM | 39 | C | ALA A | 42 | 5.392 | 32.308 | 29.008 | 1.00 | 37.34 | C |
| ATOM | 40 | O | ALA A | 42 | 5.167 | 33.503 | 29.185 | 1.00 | 37.34 | O |
| ATOM | 41 | N | THR A | 43 | 6.172 | 31.589 | 29.807 | 1.00 | 44.44 | N |
| ATOM | 42 | CA | THR A | 43 | 6.839 | 32.160 | 30.973 | 1.00 | 44.44 | C |
| ATOM | 43 | CB | THR A | 43 | 6.501 | 31.338 | 32.227 | 1.00 | 33.32 | C |
| ATOM | 44 | OG1 | THR A | 43 | 5.088 | 31.369 | 32.455 | 1.00 | 33.32 | O |
| ATOM | 45 | CG2 | THR A | 43 | 7.238 | 31.871 | 33.434 | 1.00 | 33.32 | C |
| ATOM | 46 | C | THR A | 43 | 8.354 | 32.117 | 30.756 | 1.00 | 44.44 | C |
| ATOM | 47 | O | THR A | 43 | 8.890 | 31.111 | 30.328 | 1.00 | 44.44 | O |
| ATOM | 48 | N | PRO A | 44 | 9.078 | 33.201 | 31.062 | 1.00 | 38.15 | N |
| ATOM | 49 | CD | PRO A | 44 | 8.753 | 34.418 | 31.812 | 1.00 | 42.08 | C |
| ATOM | 50 | CA | PRO A | 44 | 10.523 | 33.074 | 30.829 | 1.00 | 38.15 | C |
| ATOM | 51 | CB | PRO A | 44 | 11.067 | 34.480 | 31.048 | 1.00 | 42.08 | C |
| ATOM | 52 | CG | PRO A | 44 | 9.896 | 35.289 | 31.518 | 1.00 | 42.08 | C |
| ATOM | 53 | C | PRO A | 44 | 11.156 | 32.091 | 31.795 | 1.00 | 38.15 | C |
| ATOM | 54 | O | PRO A | 44 | 10.600 | 31.813 | 32.853 | 1.00 | 38.15 | O |
| ATOM | 55 | N | GLY A | 45 | 12.309 | 31.551 | 31.418 | 1.00 | 36.57 | N |
| ATOM | 56 | CA | GLY A | 45 | 12.982 | 30.573 | 32.249 | 1.00 | 36.57 | C |
| ATOM | 57 | C | GLY A | 45 | 13.647 | 31.080 | 33.508 | 1.00 | 36.57 | C |
| ATOM | 58 | O | GLY A | 45 | 13.574 | 30.406 | 34.542 | 1.00 | 36.57 | O |

| | | | | | | | | | | | |
|---|---|---|---|---|---|---|---|---|---|---|---|
| ATOM | 59 | N | ALA | A | 46 | 14.280 | 32.251 | 33.436 | 1.00 | 62.30 | N |
| ATOM | 60 | CA | ALA | A | 46 | 15.035 | 32.785 | 34.568 | 1.00 | 62.30 | C |
| ATOM | 61 | CB | ALA | A | 46 | 16.488 | 32.991 | 34.145 | 1.00 | 63.64 | C |
| ATOM | 62 | C | ALA | A | 46 | 14.492 | 34.062 | 35.201 | 1.00 | 62.30 | C |
| ATOM | 63 | O | ALA | A | 46 | 14.530 | 34.197 | 36.428 | 1.00 | 62.30 | O |
| ATOM | 64 | N | GLY | A | 47 | 13.974 | 34.984 | 34.388 | 1.00 | 90.60 | N |
| ATOM | 65 | CA | GLY | A | 47 | 13.463 | 36.240 | 34.918 | 1.00 | 90.60 | C |
| ATOM | 66 | C | GLY | A | 47 | 12.381 | 35.986 | 35.938 | 1.00 | 90.60 | C |
| ATOM | 67 | O | GLY | A | 47 | 12.415 | 34.998 | 36.661 | 1.00 | 90.60 | O |
| ATOM | 68 | N | PRO | A | 48 | 11.379 | 36.851 | 36.015 | 1.00 | 87.43 | N |
| ATOM | 69 | CD | PRO | A | 48 | 11.181 | 38.126 | 35.308 | 1.00 | 75.51 | C |
| ATOM | 70 | CA | PRO | A | 48 | 10.329 | 36.607 | 37.007 | 1.00 | 87.43 | C |
| ATOM | 71 | CB | PRO | A | 48 | 9.708 | 37.971 | 37.167 | 1.00 | 75.51 | C |
| ATOM | 72 | CG | PRO | A | 48 | 9.822 | 38.503 | 35.746 | 1.00 | 75.51 | C |
| ATOM | 73 | C | PRO | A | 48 | 9.325 | 35.595 | 36.525 | 1.00 | 87.43 | C |
| ATOM | 74 | O | PRO | A | 48 | 9.304 | 35.259 | 35.350 | 1.00 | 87.43 | O |
| ATOM | 75 | N | ASP | A | 49 | 8.506 | 35.067 | 37.417 | 1.00 | 61.52 | N |
| ATOM | 76 | CA | ASP | A | 49 | 7.541 | 34.116 | 36.913 | 1.00 | 61.52 | C |
| ATOM | 77 | CB | ASP | A | 49 | 7.181 | 33.047 | 37.953 | 1.00 | 58.30 | C |
| ATOM | 78 | CG | ASP | A | 49 | 6.498 | 31.838 | 37.320 | 1.00 | 58.30 | C |
| ATOM | 79 | OD1 | ASP | A | 49 | 6.743 | 30.691 | 37.782 | 1.00 | 58.30 | O |
| ATOM | 80 | OD2 | ASP | A | 49 | 5.718 | 32.069 | 36.358 | 1.00 | 58.30 | O |
| ATOM | 81 | C | ASP | A | 49 | 6.360 | 34.966 | 36.550 | 1.00 | 61.52 | C |
| ATOM | 82 | O | ASP | A | 49 | 5.492 | 35.196 | 37.383 | 1.00 | 61.52 | O |
| ATOM | 83 | N | ARG | A | 50 | 6.389 | 35.500 | 35.328 | 1.00 | 65.48 | N |
| ATOM | 84 | CA | ARG | A | 50 | 5.316 | 36.356 | 34.833 | 1.00 | 65.48 | C |
| ATOM | 85 | CB | ARG | A | 50 | 5.703 | 37.824 | 34.944 | 1.00 | 92.34 | C |
| ATOM | 86 | CG | ARG | A | 50 | 4.572 | 38.688 | 35.482 | 1.00 | 92.34 | C |
| ATOM | 87 | CD | ARG | A | 50 | 4.777 | 40.130 | 35.077 | 1.00 | 92.34 | C |
| ATOM | 88 | NE | ARG | A | 50 | 4.258 | 41.091 | 36.055 | 1.00 | 92.34 | N |
| ATOM | 89 | CZ | ARG | A | 50 | 3.053 | 41.677 | 36.021 | 1.00 | 92.34 | C |
| ATOM | 90 | NH1 | ARG | A | 50 | 2.168 | 41.427 | 35.044 | 1.00 | 92.34 | N |
| ATOM | 91 | NH2 | ARG | A | 50 | 2.726 | 42.542 | 36.978 | 1.00 | 92.34 | N |
| ATOM | 92 | C | ARG | A | 50 | 5.024 | 36.027 | 33.374 | 1.00 | 65.48 | C |
| ATOM | 93 | O | ARG | A | 50 | 5.807 | 36.337 | 32.487 | 1.00 | 65.48 | O |
| ATOM | 94 | N | PRO | A | 51 | 3.871 | 35.413 | 33.100 | 1.00 | 33.34 | N |
| ATOM | 95 | CD | PRO | A | 51 | 2.758 | 35.214 | 34.034 | 1.00 | 36.17 | C |
| ATOM | 96 | CA | PRO | A | 51 | 3.485 | 35.040 | 31.732 | 1.00 | 33.34 | C |
| ATOM | 97 | CB | PRO | A | 51 | 2.078 | 34.479 | 31.894 | 1.00 | 36.17 | C |
| ATOM | 98 | CG | PRO | A | 51 | 1.958 | 34.200 | 33.326 | 1.00 | 36.17 | C |
| ATOM | 99 | C | PRO | A | 51 | 3.487 | 36.206 | 30.755 | 1.00 | 33.34 | C |
| ATOM | 100 | O | PRO | A | 51 | 3.142 | 37.341 | 31.102 | 1.00 | 33.34 | O |
| ATOM | 101 | N | GLN | A | 52 | 3.876 | 35.932 | 29.523 | 1.00 | 55.88 | N |
| ATOM | 102 | CA | GLN | A | 52 | 3.889 | 36.974 | 28.507 | 1.00 | 55.88 | C |
| ATOM | 103 | CB | GLN | A | 52 | 5.256 | 37.630 | 28.385 | 1.00 | 32.84 | C |
| ATOM | 104 | CG | GLN | A | 52 | 6.321 | 36.806 | 27.693 | 1.00 | 32.84 | C |
| ATOM | 105 | CD | GLN | A | 52 | 7.660 | 37.534 | 27.685 | 1.00 | 32.84 | C |
| ATOM | 106 | OE1 | GLN | A | 52 | 8.092 | 38.059 | 26.653 | 1.00 | 32.84 | O |
| ATOM | 107 | NE2 | GLN | A | 52 | 8.326 | 37.566 | 28.838 | 1.00 | 32.84 | N |
| ATOM | 108 | C | GLN | A | 52 | 3.573 | 36.280 | 27.221 | 1.00 | 55.88 | C |
| ATOM | 109 | O | GLN | A | 52 | 3.757 | 35.058 | 27.101 | 1.00 | 55.88 | O |
| ATOM | 110 | N | GLU | A | 53 | 3.048 | 37.030 | 26.264 | 1.00 | 57.27 | N |
| ATOM | 111 | CA | GLU | A | 53 | 2.767 | 36.379 | 25.002 | 1.00 | 57.27 | C |
| ATOM | 112 | CB | GLU | A | 53 | 1.492 | 36.914 | 24.357 | 1.00 | 68.20 | C |
| ATOM | 113 | CG | GLU | A | 53 | 1.706 | 37.689 | 23.118 | 1.00 | 68.20 | C |
| ATOM | 114 | CD | GLU | A | 53 | 0.448 | 38.317 | 22.668 | 1.00 | 68.20 | C |
| ATOM | 115 | OE1 | GLU | A | 53 | -0.525 | 38.355 | 23.471 | 1.00 | 68.20 | O |
| ATOM | 116 | OE2 | GLU | A | 53 | 0.466 | 38.767 | 21.512 | 1.00 | 68.20 | O |
| ATOM | 117 | C | GLU | A | 53 | 3.964 | 36.469 | 24.057 | 1.00 | 57.27 | C |
| ATOM | 118 | O | GLU | A | 53 | 4.618 | 37.509 | 23.890 | 1.00 | 57.27 | O |
| ATOM | 119 | N | VAL | A | 54 | 4.247 | 35.325 | 23.460 | 1.00 | 47.62 | N |
| ATOM | 120 | CA | VAL | A | 54 | 5.350 | 35.169 | 22.542 | 1.00 | 47.62 | C |
| ATOM | 121 | CB | VAL | A | 54 | 6.369 | 34.171 | 23.126 | 1.00 | 67.98 | C |

| | | | | | | | | | | | |
|---|---|---|---|---|---|---|---|---|---|---|---|
| ATOM | 122 | CG1 | VAL | A | 54 | 7.589 | 34.078 | 22.247 | 1.00 | 67.98 | C |
| ATOM | 123 | CG2 | VAL | A | 54 | 6.740 | 34.589 | 24.537 | 1.00 | 67.98 | C |
| ATOM | 124 | C | VAL | A | 54 | 4.805 | 34.621 | 21.223 | 1.00 | 47.62 | C |
| ATOM | 125 | O | VAL | A | 54 | 4.025 | 33.661 | 21.204 | 1.00 | 47.62 | O |
| ATOM | 126 | N | SER | A | 55 | 5.197 | 35.242 | 20.118 | 1.00 | 38.73 | N |
| ATOM | 127 | CA | SER | A | 55 | 4.747 | 34.788 | 18.813 | 1.00 | 38.73 | C |
| ATOM | 128 | CB | SER | A | 55 | 3.940 | 35.870 | 18.119 | 1.00 | 74.91 | C |
| ATOM | 129 | OG | SER | A | 55 | 2.651 | 35.957 | 18.688 | 1.00 | 74.91 | O |
| ATOM | 130 | C | SER | A | 55 | 5.918 | 34.410 | 17.942 | 1.00 | 38.73 | C |
| ATOM | 131 | O | SER | A | 55 | 6.924 | 35.121 | 17.879 | 1.00 | 38.73 | O |
| ATOM | 132 | N | TYR | A | 56 | 5.784 | 33.289 | 17.258 | 1.00 | 48.72 | N |
| ATOM | 133 | CA | TYR | A | 56 | 6.856 | 32.837 | 16.407 | 1.00 | 48.72 | C |
| ATOM | 134 | CB | TYR | A | 56 | 7.643 | 31.725 | 17.094 | 1.00 | 43.32 | C |
| ATOM | 135 | CG | TYR | A | 56 | 6.822 | 30.515 | 17.438 | 1.00 | 43.32 | C |
| ATOM | 136 | CD1 | TYR | A | 56 | 6.523 | 29.570 | 16.465 | 1.00 | 43.32 | C |
| ATOM | 137 | CE1 | TYR | A | 56 | 5.780 | 28.450 | 16.763 | 1.00 | 43.32 | C |
| ATOM | 138 | CD2 | TYR | A | 56 | 6.344 | 30.311 | 18.736 | 1.00 | 43.32 | C |
| ATOM | 139 | CE2 | TYR | A | 56 | 5.594 | 29.190 | 19.051 | 1.00 | 43.32 | C |
| ATOM | 140 | CZ | TYR | A | 56 | 5.317 | 28.258 | 18.057 | 1.00 | 43.32 | C |
| ATOM | 141 | OH | TYR | A | 56 | 4.601 | 27.117 | 18.355 | 1.00 | 43.32 | O |
| ATOM | 142 | C | TYR | A | 56 | 6.252 | 32.349 | 15.121 | 1.00 | 48.72 | C |
| ATOM | 143 | O | TYR | A | 56 | 5.048 | 32.462 | 14.913 | 1.00 | 48.72 | O |
| ATOM | 144 | N | THR | A | 57 | 7.088 | 31.790 | 14.260 | 1.00 | 96.81 | N |
| ATOM | 145 | CA | THR | A | 57 | 6.608 | 31.330 | 12.977 | 1.00 | 96.81 | C |
| ATOM | 146 | CB | THR | A | 57 | 6.274 | 32.555 | 12.116 | 1.00 | 76.27 | C |
| ATOM | 147 | OG1 | THR | A | 57 | 5.608 | 32.141 | 10.919 | 1.00 | 76.27 | O |
| ATOM | 148 | CG2 | THR | A | 57 | 7.552 | 33.327 | 11.790 | 1.00 | 76.27 | C |
| ATOM | 149 | C | THR | A | 57 | 7.669 | 30.469 | 12.294 | 1.00 | 96.81 | C |
| ATOM | 150 | O | THR | A | 57 | 8.776 | 30.312 | 12.817 | 1.00 | 96.81 | O |
| ATOM | 151 | N | ASP | A | 58 | 7.332 | 29.913 | 11.132 | 1.00 | 56.40 | N |
| ATOM | 152 | CA | ASP | A | 58 | 8.269 | 29.066 | 10.401 | 1.00 | 56.40 | C |
| ATOM | 153 | CB | ASP | A | 58 | 9.508 | 29.865 | 9.998 | 1.00 | 93.62 | C |
| ATOM | 154 | CG | ASP | A | 58 | 9.244 | 30.808 | 8.846 | 1.00 | 93.62 | C |
| ATOM | 155 | OD1 | ASP | A | 58 | 8.976 | 30.320 | 7.727 | 1.00 | 93.62 | O |
| ATOM | 156 | OD2 | ASP | A | 58 | 9.300 | 32.039 | 9.055 | 1.00 | 93.62 | O |
| ATOM | 157 | C | ASP | A | 58 | 8.692 | 27.880 | 11.262 | 1.00 | 56.40 | C |
| ATOM | 158 | O | ASP | A | 58 | 9.885 | 27.604 | 11.409 | 1.00 | 56.40 | O |
| ATOM | 159 | N | THR | A | 59 | 7.709 | 27.188 | 11.832 | 1.00 | 71.62 | N |
| ATOM | 160 | CA | THR | A | 59 | 7.972 | 26.028 | 12.681 | 1.00 | 71.62 | C |
| ATOM | 161 | CB | THR | A | 59 | 6.778 | 25.758 | 13.614 | 1.00 | 73.90 | C |
| ATOM | 162 | OG1 | THR | A | 59 | 6.460 | 26.962 | 14.324 | 1.00 | 73.90 | O |
| ATOM | 163 | CG2 | THR | A | 59 | 7.118 | 24.671 | 14.619 | 1.00 | 73.90 | C |
| ATOM | 164 | C | THR | A | 59 | 8.236 | 24.799 | 11.813 | 1.00 | 71.62 | C |
| ATOM | 165 | O | THR | A | 59 | 7.418 | 24.446 | 10.970 | 1.00 | 71.62 | O |
| ATOM | 166 | N | LYS | A | 60 | 9.370 | 24.142 | 12.028 | 1.00 | 49.31 | N |
| ATOM | 167 | CA | LYS | A | 60 | 9.738 | 22.982 | 11.221 | 1.00 | 49.31 | C |
| ATOM | 168 | CB | LYS | A | 60 | 10.773 | 23.389 | 10.175 | 1.00 | 73.59 | C |
| ATOM | 169 | CG | LYS | A | 60 | 10.884 | 24.903 | 9.980 | 1.00 | 73.59 | C |
| ATOM | 170 | CD | LYS | A | 60 | 12.291 | 25.346 | 9.624 | 1.00 | 73.59 | C |
| ATOM | 171 | CE | LYS | A | 60 | 12.678 | 24.889 | 8.244 | 1.00 | 73.59 | C |
| ATOM | 172 | NZ | LYS | A | 60 | 13.424 | 25.970 | 7.537 | 1.00 | 73.59 | N |
| ATOM | 173 | C | LYS | A | 60 | 10.348 | 21.949 | 12.139 | 1.00 | 49.31 | C |
| ATOM | 174 | O | LYS | A | 60 | 11.047 | 22.309 | 13.076 | 1.00 | 49.31 | O |
| ATOM | 175 | N | VAL | A | 61 | 10.114 | 20.672 | 11.884 | 1.00 | 43.56 | N |
| ATOM | 176 | CA | VAL | A | 61 | 10.674 | 19.672 | 12.782 | 1.00 | 43.56 | C |
| ATOM | 177 | CB | VAL | A | 61 | 9.949 | 18.325 | 12.659 | 1.00 | 33.04 | C |
| ATOM | 178 | CG1 | VAL | A | 61 | 10.198 | 17.504 | 13.917 | 1.00 | 33.04 | C |
| ATOM | 179 | CG2 | VAL | A | 61 | 8.450 | 18.560 | 12.420 | 1.00 | 33.04 | C |
| ATOM | 180 | C | VAL | A | 61 | 12.157 | 19.461 | 12.522 | 1.00 | 43.56 | C |
| ATOM | 181 | O | VAL | A | 61 | 12.604 | 19.533 | 11.376 | 1.00 | 43.56 | O |
| ATOM | 182 | N | ILE | A | 62 | 12.914 | 19.222 | 13.590 | 1.00 | 45.16 | N |
| ATOM | 183 | CA | ILE | A | 62 | 14.336 | 18.992 | 13.489 | 1.00 | 45.16 | C |
| ATOM | 184 | CB | ILE | A | 62 | 15.167 | 20.315 | 13.777 | 1.00 | 36.34 | C |

| | | | | | | | | | | | |
|---|---|---|---|---|---|---|---|---|---|---|---|
| ATOM | 185 | CG2 | ILE | A | 62 | 14.891 | 21.367 | 12.719 | 1.00 | 36.34 | C |
| ATOM | 186 | CG1 | ILE | A | 62 | 14.768 | 20.920 | 15.110 | 1.00 | 36.34 | C |
| ATOM | 187 | CD1 | ILE | A | 62 | 15.423 | 22.307 | 15.347 | 1.00 | 36.34 | C |
| ATOM | 188 | C | ILE | A | 62 | 14.826 | 17.833 | 14.379 | 1.00 | 45.16 | C |
| ATOM | 189 | O | ILE | A | 62 | 15.547 | 17.018 | 13.866 | 1.00 | 45.16 | O |
| ATOM | 190 | N | GLY | A | 63 | 14.416 | 17.678 | 15.638 | 1.00 | 39.89 | N |
| ATOM | 191 | CA | GLY | A | 63 | 15.002 | 16.592 | 16.417 | 1.00 | 39.89 | C |
| ATOM | 192 | C | GLY | A | 63 | 14.038 | 15.510 | 16.891 | 1.00 | 39.89 | C |
| ATOM | 193 | O | GLY | A | 63 | 12.895 | 15.775 | 17.217 | 1.00 | 39.89 | O |
| ATOM | 194 | N | ASN | A | 64 | 14.603 | 14.321 | 17.140 | 1.00 | 43.43 | N |
| ATOM | 195 | CA | ASN | A | 64 | 13.774 | 13.276 | 17.691 | 1.00 | 43.43 | C |
| ATOM | 196 | CB | ASN | A | 64 | 13.378 | 12.229 | 16.672 | 1.00 | 74.61 | C |
| ATOM | 197 | CG | ASN | A | 64 | 12.242 | 12.746 | 15.736 | 1.00 | 74.61 | C |
| ATOM | 198 | OD1 | ASN | A | 64 | 11.397 | 11.970 | 15.281 | 1.00 | 74.61 | O |
| ATOM | 199 | ND2 | ASN | A | 64 | 12.256 | 14.059 | 15.424 | 1.00 | 74.61 | N |
| ATOM | 200 | C | ASN | A | 64 | 14.154 | 12.638 | 18.983 | 1.00 | 43.43 | C |
| ATOM | 201 | O | ASN | A | 64 | 13.548 | 12.987 | 19.961 | 1.00 | 43.43 | O |
| ATOM | 202 | N | GLY | A | 65 | 15.022 | 11.642 | 18.992 | 1.00 | 30.93 | N |
| ATOM | 203 | CA | GLY | A | 65 | 15.356 | 11.005 | 20.244 | 1.00 | 30.93 | C |
| ATOM | 204 | C | GLY | A | 65 | 14.172 | 10.448 | 21.023 | 1.00 | 30.93 | C |
| ATOM | 205 | O | GLY | A | 65 | 13.021 | 10.568 | 20.610 | 1.00 | 30.93 | O |
| ATOM | 206 | N | SER | A | 66 | 14.495 | 9.771 | 22.122 | 1.00 | 80.13 | N |
| ATOM | 207 | CA | SER | A | 66 | 13.514 | 9.137 | 22.994 | 1.00 | 80.13 | C |
| ATOM | 208 | CB | SER | A | 66 | 14.069 | 8.969 | 24.400 | 1.00 | 68.64 | C |
| ATOM | 209 | OG | SER | A | 66 | 15.063 | 7.973 | 24.408 | 1.00 | 68.64 | O |
| ATOM | 210 | C | SER | A | 66 | 12.156 | 9.819 | 23.127 | 1.00 | 80.13 | C |
| ATOM | 211 | O | SER | A | 66 | 11.242 | 9.537 | 22.350 | 1.00 | 80.13 | O |
| ATOM | 212 | N | ALA | A | 67 | 12.001 | 10.661 | 24.152 | 1.00 | 76.33 | N |
| ATOM | 213 | CA | ALA | A | 67 | 10.719 | 11.299 | 24.418 | 1.00 | 76.33 | C |
| ATOM | 214 | CB | ALA | A | 67 | 10.342 | 11.064 | 25.863 | 1.00 | 58.91 | C |
| ATOM | 215 | C | ALA | A | 67 | 10.763 | 12.780 | 24.122 | 1.00 | 76.33 | C |
| ATOM | 216 | O | ALA | A | 67 | 11.407 | 13.545 | 24.833 | 1.00 | 76.33 | O |
| ATOM | 217 | N | GLY | A | 68 | 10.087 | 13.180 | 23.058 | 1.00 | 66.99 | N |
| ATOM | 218 | CA | GLY | A | 68 | 10.051 | 14.585 | 22.728 | 1.00 | 66.99 | C |
| ATOM | 219 | C | GLY | A | 68 | 10.647 | 15.038 | 21.418 | 1.00 | 66.99 | C |
| ATOM | 220 | O | GLY | A | 68 | 11.859 | 14.933 | 21.159 | 1.00 | 66.99 | O |
| ATOM | 221 | N | VAL | A | 69 | 9.799 | 15.586 | 20.569 | 1.00 | 47.39 | N |
| ATOM | 222 | CA | VAL | A | 69 | 10.331 | 16.072 | 19.315 | 1.00 | 47.39 | C |
| ATOM | 223 | CB | VAL | A | 69 | 9.363 | 15.755 | 18.129 | 1.00 | 51.90 | C |
| ATOM | 224 | CG1 | VAL | A | 69 | 9.372 | 16.869 | 17.066 | 1.00 | 51.90 | C |
| ATOM | 225 | CG2 | VAL | A | 69 | 9.821 | 14.444 | 17.428 | 1.00 | 51.90 | C |
| ATOM | 226 | C | VAL | A | 69 | 10.624 | 17.555 | 19.444 | 1.00 | 47.39 | C |
| ATOM | 227 | O | VAL | A | 69 | 9.935 | 18.274 | 20.161 | 1.00 | 47.39 | O |
| ATOM | 228 | N | VAL | A | 70 | 11.640 | 17.991 | 18.714 | 1.00 | 39.11 | N |
| ATOM | 229 | CA | VAL | A | 70 | 12.103 | 19.364 | 18.762 | 1.00 | 39.11 | C |
| ATOM | 230 | CB | VAL | A | 70 | 13.628 | 19.390 | 19.068 | 1.00 | 46.74 | C |
| ATOM | 231 | CG1 | VAL | A | 70 | 14.167 | 20.798 | 19.020 | 1.00 | 46.74 | C |
| ATOM | 232 | CG2 | VAL | A | 70 | 13.890 | 18.763 | 20.431 | 1.00 | 46.74 | C |
| ATOM | 233 | C | VAL | A | 70 | 11.832 | 20.025 | 17.419 | 1.00 | 39.11 | C |
| ATOM | 234 | O | VAL | A | 70 | 12.084 | 19.446 | 16.354 | 1.00 | 39.11 | O |
| ATOM | 235 | N | TYR | A | 71 | 11.294 | 21.237 | 17.485 | 1.00 | 46.83 | N |
| ATOM | 236 | CA | TYR | A | 71 | 10.977 | 22.007 | 16.299 | 1.00 | 46.83 | C |
| ATOM | 237 | CB | TYR | A | 71 | 9.528 | 22.501 | 16.359 | 1.00 | 53.95 | C |
| ATOM | 238 | CG | TYR | A | 71 | 8.458 | 21.427 | 16.400 | 1.00 | 53.95 | C |
| ATOM | 239 | CD1 | TYR | A | 71 | 8.419 | 20.479 | 17.425 | 1.00 | 53.95 | C |
| ATOM | 240 | CE1 | TYR | A | 71 | 7.399 | 19.520 | 17.482 | 1.00 | 53.95 | C |
| ATOM | 241 | CD2 | TYR | A | 71 | 7.450 | 21.392 | 15.430 | 1.00 | 53.95 | C |
| ATOM | 242 | CE2 | TYR | A | 71 | 6.426 | 20.447 | 15.478 | 1.00 | 53.95 | C |
| ATOM | 243 | CZ | TYR | A | 71 | 6.405 | 19.513 | 16.505 | 1.00 | 53.95 | C |
| ATOM | 244 | OH | TYR | A | 71 | 5.387 | 18.583 | 16.548 | 1.00 | 53.95 | O |
| ATOM | 245 | C | TYR | A | 71 | 11.907 | 23.211 | 16.259 | 1.00 | 46.83 | C |
| ATOM | 246 | O | TYR | A | 71 | 12.634 | 23.479 | 17.210 | 1.00 | 46.83 | O |
| ATOM | 247 | N | GLN | A | 72 | 11.861 | 23.941 | 15.156 | 1.00 | 51.45 | N |

| | | | | | | | | | | | |
|---|---|---|---|---|---|---|---|---|---|---|---|
| ATOM | 248 | CA | GLN | A | 72 | 12.676 | 25.136 | 14.971 | 1.00 | 51.45 | C |
| ATOM | 249 | CB | GLN | A | 72 | 13.730 | 24.884 | 13.892 | 1.00 | 78.04 | C |
| ATOM | 250 | CG | GLN | A | 72 | 14.722 | 26.021 | 13.671 | 1.00 | 78.04 | C |
| ATOM | 251 | CD | GLN | A | 72 | 14.392 | 26.865 | 12.458 | 1.00 | 78.04 | C |
| ATOM | 252 | OE1 | GLN | A | 72 | 13.460 | 27.669 | 12.478 | 1.00 | 78.04 | O |
| ATOM | 253 | NE2 | GLN | A | 72 | 15.155 | 26.679 | 11.385 | 1.00 | 78.04 | N |
| ATOM | 254 | C | GLN | A | 72 | 11.717 | 26.239 | 14.534 | 1.00 | 51.45 | C |
| ATOM | 255 | O | GLN | A | 72 | 10.863 | 26.013 | 13.678 | 1.00 | 51.45 | O |
| ATOM | 256 | N | ALA | A | 73 | 11.847 | 27.426 | 15.121 | 1.00 | 39.89 | N |
| ATOM | 257 | CA | ALA | A | 73 | 10.953 | 28.531 | 14.791 | 1.00 | 39.89 | C |
| ATOM | 258 | CB | ALA | A | 73 | 9.811 | 28.588 | 15.788 | 1.00 | 60.92 | C |
| ATOM | 259 | C | ALA | A | 73 | 11.659 | 29.867 | 14.760 | 1.00 | 39.89 | C |
| ATOM | 260 | O | ALA | A | 73 | 12.873 | 29.945 | 14.949 | 1.00 | 39.89 | O |
| ATOM | 261 | N | LYS | A | 74 | 10.883 | 30.923 | 14.533 | 1.00 | 46.58 | N |
| ATOM | 262 | CA | LYS | A | 74 | 11.435 | 32.266 | 14.469 | 1.00 | 46.58 | C |
| ATOM | 263 | CB | LYS | A | 74 | 11.625 | 32.675 | 13.006 | 1.00 | 90.82 | C |
| ATOM | 264 | CG | LYS | A | 74 | 12.349 | 34.004 | 12.807 | 1.00 | 90.82 | C |
| ATOM | 265 | CD | LYS | A | 74 | 11.381 | 35.145 | 12.546 | 1.00 | 90.82 | C |
| ATOM | 266 | CE | LYS | A | 74 | 11.662 | 35.819 | 11.213 | 1.00 | 90.82 | C |
| ATOM | 267 | NZ | LYS | A | 74 | 11.393 | 34.932 | 10.047 | 1.00 | 90.82 | N |
| ATOM | 268 | C | LYS | A | 74 | 10.577 | 33.303 | 15.189 | 1.00 | 46.58 | C |
| ATOM | 269 | O | LYS | A | 74 | 9.490 | 33.667 | 14.729 | 1.00 | 46.58 | O |
| ATOM | 270 | N | LEU | A | 75 | 11.070 | 33.770 | 16.329 | 1.00 | 70.67 | N |
| ATOM | 271 | CA | LEU | A | 75 | 10.367 | 34.781 | 17.101 | 1.00 | 70.67 | C |
| ATOM | 272 | CB | LEU | A | 75 | 11.245 | 35.258 | 18.259 | 1.00 | 47.72 | C |
| ATOM | 273 | CG | LEU | A | 75 | 11.844 | 34.145 | 19.114 | 1.00 | 47.72 | C |
| ATOM | 274 | CD1 | LEU | A | 75 | 12.721 | 34.743 | 20.205 | 1.00 | 47.72 | C |
| ATOM | 275 | CD2 | LEU | A | 75 | 10.724 | 33.312 | 19.704 | 1.00 | 47.72 | C |
| ATOM | 276 | C | LEU | A | 75 | 10.109 | 35.931 | 16.143 | 1.00 | 70.67 | C |
| ATOM | 277 | O | LEU | A | 75 | 10.929 | 36.205 | 15.268 | 1.00 | 70.67 | O |
| ATOM | 278 | N | CYS | A | 76 | 8.979 | 36.608 | 16.311 | 1.00 | 63.01 | N |
| ATOM | 279 | CA | CYS | A | 76 | 8.628 | 37.715 | 15.433 | 1.00 | 63.01 | C |
| ATOM | 280 | CB | CYS | A | 76 | 7.115 | 37.822 | 15.341 | 1.00 | 56.47 | C |
| ATOM | 281 | SG | CYS | A | 76 | 6.376 | 36.251 | 14.858 | 1.00 | 56.47 | S |
| ATOM | 282 | C | CYS | A | 76 | 9.227 | 39.042 | 15.868 | 1.00 | 63.01 | C |
| ATOM | 283 | O | CYS | A | 76 | 9.762 | 39.778 | 15.048 | 1.00 | 63.01 | O |
| ATOM | 284 | N | ASP | A | 77 | 9.159 | 39.331 | 17.161 | 1.00 | 82.56 | N |
| ATOM | 285 | CA | ASP | A | 77 | 9.684 | 40.577 | 17.717 | 1.00 | 82.56 | C |
| ATOM | 286 | CB | ASP | A | 77 | 9.403 | 40.619 | 19.218 | 1.00 | 98.35 | C |
| ATOM | 287 | CG | ASP | A | 77 | 10.123 | 39.519 | 19.965 | 1.00 | 98.35 | C |
| ATOM | 288 | OD1 | ASP | A | 77 | 10.839 | 38.730 | 19.315 | 1.00 | 98.35 | O |
| ATOM | 289 | OD2 | ASP | A | 77 | 9.978 | 39.435 | 21.201 | 1.00 | 98.35 | O |
| ATOM | 290 | C | ASP | A | 77 | 11.186 | 40.826 | 17.494 | 1.00 | 82.56 | C |
| ATOM | 291 | O | ASP | A | 77 | 11.669 | 41.942 | 17.705 | 1.00 | 82.56 | O |
| ATOM | 292 | N | SER | A | 78 | 11.928 | 39.802 | 17.081 | 1.00 | 56.03 | N |
| ATOM | 293 | CA | SER | A | 78 | 13.366 | 39.963 | 16.870 | 1.00 | 56.03 | C |
| ATOM | 294 | CB | SER | A | 78 | 14.131 | 39.469 | 18.099 | 1.00 | 73.70 | C |
| ATOM | 295 | OG | SER | A | 78 | 13.805 | 38.119 | 18.377 | 1.00 | 73.70 | O |
| ATOM | 296 | C | SER | A | 78 | 13.854 | 39.218 | 15.639 | 1.00 | 56.03 | C |
| ATOM | 297 | O | SER | A | 78 | 14.887 | 39.553 | 15.066 | 1.00 | 56.03 | O |
| ATOM | 298 | N | GLY | A | 79 | 13.101 | 38.207 | 15.235 | 1.00 | 44.63 | N |
| ATOM | 299 | CA | GLY | A | 79 | 13.488 | 37.440 | 14.074 | 1.00 | 44.63 | C |
| ATOM | 300 | C | GLY | A | 79 | 14.506 | 36.393 | 14.463 | 1.00 | 44.63 | C |
| ATOM | 301 | O | GLY | A | 79 | 15.034 | 35.704 | 13.598 | 1.00 | 44.63 | O |
| ATOM | 302 | N | GLU | A | 80 | 14.775 | 36.266 | 15.762 | 1.00 | 40.60 | N |
| ATOM | 303 | CA | GLU | A | 80 | 15.742 | 35.292 | 16.253 | 1.00 | 40.60 | C |
| ATOM | 304 | CB | GLU | A | 80 | 16.004 | 35.484 | 17.751 | 1.00 | 68.27 | C |
| ATOM | 305 | CG | GLU | A | 80 | 16.276 | 36.909 | 18.174 | 1.00 | 68.27 | C |
| ATOM | 306 | CD | GLU | A | 80 | 16.439 | 37.033 | 19.672 | 1.00 | 68.27 | C |
| ATOM | 307 | OE1 | GLU | A | 80 | 15.914 | 38.012 | 20.252 | 1.00 | 68.27 | O |
| ATOM | 308 | OE2 | GLU | A | 80 | 17.099 | 36.152 | 20.266 | 1.00 | 68.27 | O |
| ATOM | 309 | C | GLU | A | 80 | 15.258 | 33.865 | 16.020 | 1.00 | 40.60 | C |
| ATOM | 310 | O | GLU | A | 80 | 14.056 | 33.579 | 16.072 | 1.00 | 40.60 | O |

| | | | | | | | | | | | |
|---|---|---|---|---|---|---|---|---|---|---|---|
| ATOM | 311 | N | LEU | A | 81 | 16.212 | 32.979 | 15.749 | 1.00 | 62.69 | N |
| ATOM | 312 | CA | LEU | A | 81 | 15.932 | 31.569 | 15.532 | 1.00 | 62.69 | C |
| ATOM | 313 | CB | LEU | A | 81 | 17.016 | 30.937 | 14.665 | 1.00 | 53.78 | C |
| ATOM | 314 | CG | LEU | A | 81 | 16.710 | 30.915 | 13.174 | 1.00 | 53.78 | C |
| ATOM | 315 | CD1 | LEU | A | 81 | 17.932 | 30.456 | 12.423 | 1.00 | 53.78 | C |
| ATOM | 316 | CD2 | LEU | A | 81 | 15.537 | 29.987 | 12.911 | 1.00 | 53.78 | C |
| ATOM | 317 | C | LEU | A | 81 | 15.915 | 30.893 | 16.889 | 1.00 | 62.69 | C |
| ATOM | 318 | O | LEU | A | 81 | 16.831 | 31.071 | 17.699 | 1.00 | 62.69 | O |
| ATOM | 319 | N | VAL | A | 82 | 14.867 | 30.127 | 17.150 | 1.00 | 33.64 | N |
| ATOM | 320 | CA | VAL | A | 82 | 14.777 | 29.440 | 18.420 | 1.00 | 33.64 | C |
| ATOM | 321 | CB | VAL | A | 82 | 13.688 | 30.061 | 19.340 | 1.00 | 30.34 | C |
| ATOM | 322 | CG1 | VAL | A | 82 | 14.026 | 31.499 | 19.635 | 1.00 | 30.34 | C |
| ATOM | 323 | CG2 | VAL | A | 82 | 12.321 | 29.981 | 18.684 | 1.00 | 30.34 | C |
| ATOM | 324 | C | VAL | A | 82 | 14.433 | 28.005 | 18.158 | 1.00 | 33.64 | C |
| ATOM | 325 | O | VAL | A | 82 | 14.114 | 27.626 | 17.035 | 1.00 | 33.64 | O |
| ATOM | 326 | N | ALA | A | 83 | 14.529 | 27.207 | 19.207 | 1.00 | 40.13 | N |
| ATOM | 327 | CA | ALA | A | 83 | 14.181 | 25.800 | 19.149 | 1.00 | 40.13 | C |
| ATOM | 328 | CB | ALA | A | 83 | 15.361 | 24.944 | 19.575 | 1.00 | 12.91 | C |
| ATOM | 329 | C | ALA | A | 83 | 13.010 | 25.615 | 20.117 | 1.00 | 40.13 | C |
| ATOM | 330 | O | ALA | A | 83 | 12.853 | 26.368 | 21.087 | 1.00 | 40.13 | O |
| ATOM | 331 | N | ILE | A | 84 | 12.171 | 24.627 | 19.846 | 1.00 | 35.00 | N |
| ATOM | 332 | CA | ILE | A | 84 | 11.035 | 24.379 | 20.715 | 1.00 | 35.00 | C |
| ATOM | 333 | CB | ILE | A | 84 | 9.711 | 24.907 | 20.104 | 1.00 | 35.93 | C |
| ATOM | 334 | CG2 | ILE | A | 84 | 8.569 | 24.675 | 21.075 | 1.00 | 35.93 | C |
| ATOM | 335 | CG1 | ILE | A | 84 | 9.813 | 26.407 | 19.818 | 1.00 | 35.93 | C |
| ATOM | 336 | CD1 | ILE | A | 84 | 8.526 | 26.997 | 19.267 | 1.00 | 35.93 | C |
| ATOM | 337 | C | ILE | A | 84 | 10.905 | 22.895 | 20.962 | 1.00 | 35.00 | C |
| ATOM | 338 | O | ILE | A | 84 | 10.612 | 22.120 | 20.054 | 1.00 | 35.00 | O |
| ATOM | 339 | N | LYS | A | 85 | 11.128 | 22.497 | 22.199 | 1.00 | 26.59 | N |
| ATOM | 340 | CA | LYS | A | 85 | 11.030 | 21.091 | 22.520 | 1.00 | 26.59 | C |
| ATOM | 341 | CB | LYS | A | 85 | 12.114 | 20.695 | 23.518 | 1.00 | 20.88 | C |
| ATOM | 342 | CG | LYS | A | 85 | 12.160 | 19.201 | 23.798 | 1.00 | 20.88 | C |
| ATOM | 343 | CD | LYS | A | 85 | 13.235 | 18.902 | 24.826 | 1.00 | 20.88 | C |
| ATOM | 344 | CE | LYS | A | 85 | 13.357 | 17.426 | 25.086 | 1.00 | 20.88 | C |
| ATOM | 345 | NZ | LYS | A | 85 | 14.423 | 17.170 | 26.087 | 1.00 | 20.88 | N |
| ATOM | 346 | C | LYS | A | 85 | 9.656 | 20.774 | 23.097 | 1.00 | 26.59 | C |
| ATOM | 347 | O | LYS | A | 85 | 9.323 | 21.207 | 24.211 | 1.00 | 26.59 | O |
| ATOM | 348 | N | LYS | A | 86 | 8.861 | 20.024 | 22.334 | 1.00 | 42.98 | N |
| ATOM | 349 | CA | LYS | A | 86 | 7.523 | 19.635 | 22.776 | 1.00 | 42.98 | C |
| ATOM | 350 | CB | LYS | A | 86 | 6.537 | 19.649 | 21.609 | 1.00 | 66.38 | C |
| ATOM | 351 | CG | LYS | A | 86 | 5.141 | 20.127 | 21.989 | 1.00 | 66.38 | C |
| ATOM | 352 | CD | LYS | A | 86 | 4.314 | 20.434 | 20.762 | 1.00 | 66.38 | C |
| ATOM | 353 | CE | LYS | A | 86 | 3.798 | 19.172 | 20.112 | 1.00 | 66.38 | C |
| ATOM | 354 | NZ | LYS | A | 86 | 3.465 | 19.426 | 18.680 | 1.00 | 66.38 | N |
| ATOM | 355 | C | LYS | A | 86 | 7.655 | 18.234 | 23.338 | 1.00 | 42.98 | C |
| ATOM | 356 | O | LYS | A | 86 | 8.040 | 17.311 | 22.618 | 1.00 | 42.98 | O |
| ATOM | 357 | N | VAL | A | 87 | 7.356 | 18.087 | 24.628 | 1.00 | 50.70 | N |
| ATOM | 358 | CA | VAL | A | 87 | 7.473 | 16.799 | 25.312 | 1.00 | 50.70 | C |
| ATOM | 359 | CB | VAL | A | 87 | 8.657 | 16.787 | 26.285 | 1.00 | 48.68 | C |
| ATOM | 360 | CG1 | VAL | A | 87 | 8.736 | 15.441 | 26.979 | 1.00 | 48.68 | C |
| ATOM | 361 | CG2 | VAL | A | 87 | 9.943 | 17.083 | 25.537 | 1.00 | 48.68 | C |
| ATOM | 362 | C | VAL | A | 87 | 6.243 | 16.424 | 26.112 | 1.00 | 50.70 | C |
| ATOM | 363 | O | VAL | A | 87 | 5.759 | 17.207 | 26.925 | 1.00 | 50.70 | O |
| ATOM | 364 | N | LEU | A | 88 | 5.758 | 15.208 | 25.899 | 1.00 | 41.76 | N |
| ATOM | 365 | CA | LEU | A | 88 | 4.579 | 14.741 | 26.598 | 1.00 | 41.76 | C |
| ATOM | 366 | CB | LEU | A | 88 | 4.033 | 13.478 | 25.934 | 1.00 | 44.55 | C |
| ATOM | 367 | CG | LEU | A | 88 | 2.595 | 13.116 | 26.320 | 1.00 | 44.55 | C |
| ATOM | 368 | CD1 | LEU | A | 88 | 1.865 | 12.644 | 25.093 | 1.00 | 44.55 | C |
| ATOM | 369 | CD2 | LEU | A | 88 | 2.577 | 12.045 | 27.397 | 1.00 | 44.55 | C |
| ATOM | 370 | C | LEU | A | 88 | 4.848 | 14.465 | 28.065 | 1.00 | 41.76 | C |
| ATOM | 371 | O | LEU | A | 88 | 5.869 | 13.872 | 28.422 | 1.00 | 41.76 | O |
| ATOM | 372 | N | GLN | A | 89 | 3.903 | 14.897 | 28.897 | 1.00 | 54.87 | N |
| ATOM | 373 | CA | GLN | A | 89 | 3.959 | 14.744 | 30.348 | 1.00 | 54.87 | C |

| | | | | | | | | | | | |
|---|---|---|---|---|---|---|---|---|---|---|---|
| ATOM | 374 | CB | GLN | A | 89 | 3.275 | 15.930 | 31.017 | 1.00 | 42.26 | C |
| ATOM | 375 | CG | GLN | A | 89 | 3.965 | 17.272 | 30.765 | 1.00 | 23.68 | C |
| ATOM | 376 | CD | GLN | A | 89 | 5.423 | 17.274 | 31.261 | 1.00 | 23.68 | C |
| ATOM | 377 | OE1 | GLN | A | 89 | 5.696 | 16.929 | 32.433 | 1.00 | 23.68 | O |
| ATOM | 378 | NE2 | GLN | A | 89 | 6.364 | 17.659 | 30.380 | 1.00 | 23.68 | N |
| ATOM | 379 | C | GLN | A | 89 | 3.216 | 13.505 | 30.679 | 1.00 | 54.87 | C |
| ATOM | 380 | O | GLN | A | 89 | 2.051 | 13.374 | 30.300 | 1.00 | 54.87 | O |
| ATOM | 381 | N | ASP | A | 90 | 3.830 | 12.588 | 31.395 | 1.00 | 57.98 | N |
| ATOM | 382 | CA | ASP | A | 90 | 3.078 | 11.391 | 31.633 | 1.00 | 57.98 | C |
| ATOM | 383 | CB | ASP | A | 90 | 3.319 | 10.425 | 30.457 | 1.00 | 78.54 | C |
| ATOM | 384 | CG | ASP | A | 90 | 4.029 | 9.167 | 30.837 | 1.00 | 78.54 | C |
| ATOM | 385 | OD1 | ASP | A | 90 | 3.447 | 8.324 | 31.565 | 1.00 | 78.54 | O |
| ATOM | 386 | OD2 | ASP | A | 90 | 5.157 | 9.017 | 30.332 | 1.00 | 78.54 | O |
| ATOM | 387 | C | ASP | A | 90 | 3.433 | 10.863 | 33.036 | 1.00 | 57.98 | C |
| ATOM | 388 | O | ASP | A | 90 | 4.608 | 10.795 | 33.449 | 1.00 | 57.98 | O |
| ATOM | 389 | N | ALA | A | 91 | 2.391 | 10.612 | 33.823 | 1.00 | 56.75 | N |
| ATOM | 390 | CA | ALA | A | 91 | 2.563 | 10.151 | 35.201 | 1.00 | 56.75 | C |
| ATOM | 391 | CB | ALA | A | 91 | 1.266 | 10.270 | 35.945 | 1.00 | 36.46 | C |
| ATOM | 392 | C | ALA | A | 91 | 3.161 | 8.759 | 35.451 | 1.00 | 56.75 | C |
| ATOM | 393 | O | ALA | A | 91 | 3.261 | 8.364 | 36.616 | 1.00 | 56.75 | O |
| ATOM | 394 | N | GLY | A | 92 | 3.585 | 8.024 | 34.421 | 1.00 | 76.24 | N |
| ATOM | 395 | CA | GLY | A | 92 | 4.179 | 6.724 | 34.687 | 1.00 | 76.24 | C |
| ATOM | 396 | C | GLY | A | 92 | 5.458 | 7.007 | 35.436 | 1.00 | 76.24 | C |
| ATOM | 397 | O | GLY | A | 92 | 5.996 | 6.121 | 36.091 | 1.00 | 76.24 | O |
| ATOM | 398 | N | PHE | A | 93 | 5.922 | 8.259 | 35.381 | 1.00 | 84.86 | N |
| ATOM | 399 | CA | PHE | A | 93 | 7.197 | 8.635 | 36.022 | 1.00 | 84.86 | C |
| ATOM | 400 | CB | PHE | A | 93 | 8.417 | 8.260 | 35.144 | 1.00 | 23.68 | C |
| ATOM | 401 | CG | PHE | A | 93 | 8.133 | 7.161 | 34.141 | 1.00 | 23.68 | C |
| ATOM | 402 | CD1 | PHE | A | 93 | 7.255 | 7.436 | 33.072 | 1.00 | 23.68 | C |
| ATOM | 403 | CD2 | PHE | A | 93 | 8.576 | 5.819 | 34.350 | 1.00 | 23.68 | C |
| ATOM | 404 | CE1 | PHE | A | 93 | 6.785 | 6.423 | 32.230 | 1.00 | 23.68 | C |
| ATOM | 405 | CE2 | PHE | A | 93 | 8.111 | 4.759 | 33.508 | 1.00 | 23.68 | C |
| ATOM | 406 | CZ | PHE | A | 93 | 7.203 | 5.072 | 32.441 | 1.00 | 23.68 | C |
| ATOM | 407 | C | PHE | A | 93 | 7.336 | 10.100 | 36.349 | 1.00 | 84.86 | C |
| ATOM | 408 | O | PHE | A | 93 | 6.511 | 10.935 | 35.978 | 1.00 | 84.86 | O |
| ATOM | 409 | N | LYS | A | 94 | 8.454 | 10.403 | 36.989 | 1.00 | 48.04 | N |
| ATOM | 410 | CA | LYS | A | 94 | 8.742 | 11.748 | 37.427 | 1.00 | 48.04 | C |
| ATOM | 411 | CB | LYS | A | 94 | 9.648 | 11.686 | 38.657 | 1.00 | 56.53 | C |
| ATOM | 412 | CG | LYS | A | 94 | 9.547 | 10.377 | 39.466 | 1.00 | 56.53 | C |
| ATOM | 413 | CD | LYS | A | 94 | 9.029 | 10.598 | 40.883 | 1.00 | 56.53 | C |
| ATOM | 414 | CE | LYS | A | 94 | 9.667 | 9.593 | 41.831 | 1.00 | 56.53 | C |
| ATOM | 415 | NZ | LYS | A | 94 | 8.683 | 8.993 | 42.773 | 1.00 | 56.53 | N |
| ATOM | 416 | C | LYS | A | 94 | 9.359 | 12.634 | 36.347 | 1.00 | 48.04 | C |
| ATOM | 417 | O | LYS | A | 94 | 10.184 | 12.185 | 35.538 | 1.00 | 48.04 | O |
| ATOM | 418 | N | ASN | A | 95 | 8.956 | 13.903 | 36.362 | 1.00 | 67.64 | N |
| ATOM | 419 | CA | ASN | A | 95 | 9.402 | 14.903 | 35.395 | 1.00 | 67.64 | C |
| ATOM | 420 | CB | ASN | A | 95 | 8.176 | 15.478 | 34.679 | 1.00 | 93.97 | C |
| ATOM | 421 | CG | ASN | A | 95 | 7.158 | 14.393 | 34.294 | 1.00 | 93.97 | C |
| ATOM | 422 | OD1 | ASN | A | 95 | 6.551 | 13.762 | 35.164 | 1.00 | 93.97 | O |
| ATOM | 423 | ND2 | ASN | A | 95 | 6.968 | 14.180 | 32.993 | 1.00 | 93.97 | N |
| ATOM | 424 | C | ASN | A | 95 | 10.158 | 16.014 | 36.092 | 1.00 | 67.64 | C |
| ATOM | 425 | O | ASN | A | 95 | 9.581 | 16.724 | 36.907 | 1.00 | 67.64 | O |
| ATOM | 426 | N | ARG | A | 96 | 11.436 | 16.181 | 35.784 | 1.00 | 37.22 | N |
| ATOM | 427 | CA | ARG | A | 96 | 12.198 | 17.242 | 36.442 | 1.00 | 37.22 | C |
| ATOM | 428 | CB | ARG | A | 96 | 13.188 | 16.626 | 37.382 | 1.00 | 37.63 | C |
| ATOM | 429 | CG | ARG | A | 96 | 12.671 | 15.325 | 37.929 | 1.00 | 37.63 | C |
| ATOM | 430 | CD | ARG | A | 96 | 13.631 | 14.744 | 38.910 | 1.00 | 37.63 | C |
| ATOM | 431 | NE | ARG | A | 96 | 13.040 | 13.622 | 39.635 | 1.00 | 37.63 | N |
| ATOM | 432 | CZ | ARG | A | 96 | 13.128 | 12.330 | 39.296 | 1.00 | 37.63 | C |
| ATOM | 433 | NH1 | ARG | A | 96 | 13.801 | 11.922 | 38.211 | 1.00 | 37.63 | N |
| ATOM | 434 | NH2 | ARG | A | 96 | 12.528 | 11.424 | 40.064 | 1.00 | 37.63 | N |
| ATOM | 435 | C | ARG | A | 96 | 12.941 | 18.130 | 35.452 | 1.00 | 37.22 | C |
| ATOM | 436 | O | ARG | A | 96 | 13.621 | 19.089 | 35.828 | 1.00 | 37.22 | O |

| | | | | | | | | | | | |
|---|---|---|---|---|---|---|---|---|---|---|---|
| ATOM | 437 | N | GLU | A | 97 | 12.762 | 17.820 | 34.177 | 1.00 | 42.30 | N |
| ATOM | 438 | CA | GLU | A | 97 | 13.417 | 18.553 | 33.133 | 1.00 | 42.30 | C |
| ATOM | 439 | CB | GLU | A | 97 | 12.981 | 18.005 | 31.774 | 1.00 | 47.72 | C |
| ATOM | 440 | CG | GLU | A | 97 | 12.913 | 19.020 | 30.681 | 1.00 | 47.72 | C |
| ATOM | 441 | CD | GLU | A | 97 | 13.215 | 18.422 | 29.319 | 1.00 | 47.72 | C |
| ATOM | 442 | OE1 | GLU | A | 97 | 12.349 | 17.699 | 28.774 | 1.00 | 47.72 | O |
| ATOM | 443 | OE2 | GLU | A | 97 | 14.334 | 18.672 | 28.815 | 1.00 | 47.72 | O |
| ATOM | 444 | C | GLU | A | 97 | 13.111 | 20.024 | 33.311 | 1.00 | 42.30 | C |
| ATOM | 445 | O | GLU | A | 97 | 14.035 | 20.813 | 33.434 | 1.00 | 42.30 | O |
| ATOM | 446 | N | LEU | A | 98 | 11.833 | 20.401 | 33.359 | 1.00 | 30.62 | N |
| ATOM | 447 | CA | LEU | A | 98 | 11.506 | 21.821 | 33.571 | 1.00 | 30.62 | C |
| ATOM | 448 | CB | LEU | A | 98 | 9.986 | 22.085 | 33.691 | 1.00 | 15.17 | C |
| ATOM | 449 | CG | LEU | A | 98 | 9.594 | 23.589 | 33.876 | 1.00 | 15.17 | C |
| ATOM | 450 | CD1 | LEU | A | 98 | 10.041 | 24.474 | 32.695 | 1.00 | 15.17 | C |
| ATOM | 451 | CD2 | LEU | A | 98 | 8.067 | 23.693 | 34.020 | 1.00 | 15.17 | C |
| ATOM | 452 | C | LEU | A | 98 | 12.195 | 22.375 | 34.825 | 1.00 | 30.62 | C |
| ATOM | 453 | O | LEU | A | 98 | 12.848 | 23.406 | 34.742 | 1.00 | 30.62 | O |
| ATOM | 454 | N | GLN | A | 99 | 12.043 | 21.698 | 35.968 | 1.00 | 41.17 | N |
| ATOM | 455 | CA | GLN | A | 99 | 12.648 | 22.108 | 37.248 | 1.00 | 41.17 | C |
| ATOM | 456 | CB | GLN | A | 99 | 12.549 | 20.981 | 38.276 | 1.00 | 61.47 | C |
| ATOM | 457 | CG | GLN | A | 99 | 11.157 | 20.495 | 38.602 | 1.00 | 61.47 | C |
| ATOM | 458 | CD | GLN | A | 99 | 11.182 | 19.155 | 39.331 | 1.00 | 61.47 | C |
| ATOM | 459 | OE1 | GLN | A | 99 | 10.332 | 18.298 | 39.089 | 1.00 | 61.47 | O |
| ATOM | 460 | NE2 | GLN | A | 99 | 12.154 | 18.972 | 40.227 | 1.00 | 61.47 | N |
| ATOM | 461 | C | GLN | A | 99 | 14.121 | 22.465 | 37.128 | 1.00 | 41.17 | C |
| ATOM | 462 | O | GLN | A | 99 | 14.514 | 23.605 | 37.375 | 1.00 | 41.17 | O |
| ATOM | 463 | N | ILE | A | 100 | 14.934 | 21.465 | 36.787 | 1.00 | 32.75 | N |
| ATOM | 464 | CA | ILE | A | 100 | 16.371 | 21.655 | 36.649 | 1.00 | 32.75 | C |
| ATOM | 465 | CB | ILE | A | 100 | 17.059 | 20.375 | 36.119 | 1.00 | 26.40 | C |
| ATOM | 466 | CG2 | ILE | A | 100 | 18.432 | 20.699 | 35.546 | 1.00 | 26.40 | C |
| ATOM | 467 | CG1 | ILE | A | 100 | 17.219 | 19.367 | 37.257 | 1.00 | 26.40 | C |
| ATOM | 468 | CD1 | ILE | A | 100 | 16.038 | 18.466 | 37.451 | 1.00 | 26.40 | C |
| ATOM | 469 | C | ILE | A | 100 | 16.634 | 22.789 | 35.688 | 1.00 | 32.75 | C |
| ATOM | 470 | O | ILE | A | 100 | 17.222 | 23.803 | 36.049 | 1.00 | 32.75 | O |
| ATOM | 471 | N | MET | A | 101 | 16.186 | 22.584 | 34.459 | 1.00 | 33.05 | N |
| ATOM | 472 | CA | MET | A | 101 | 16.320 | 23.539 | 33.371 | 1.00 | 33.05 | C |
| ATOM | 473 | CB | MET | A | 101 | 15.258 | 23.218 | 32.318 | 1.00 | 49.78 | C |
| ATOM | 474 | CG | MET | A | 101 | 15.417 | 23.952 | 31.019 | 1.00 | 49.78 | C |
| ATOM | 475 | SD | MET | A | 101 | 16.628 | 23.137 | 29.988 | 1.00 | 49.78 | S |
| ATOM | 476 | CE | MET | A | 101 | 15.678 | 21.737 | 29.462 | 1.00 | 49.78 | C |
| ATOM | 477 | C | MET | A | 101 | 16.155 | 24.990 | 33.846 | 1.00 | 33.05 | C |
| ATOM | 478 | O | MET | A | 101 | 17.034 | 25.836 | 33.652 | 1.00 | 33.05 | O |
| ATOM | 479 | N | ARG | A | 102 | 15.024 | 25.270 | 34.478 | 1.00 | 42.97 | N |
| ATOM | 480 | CA | ARG | A | 102 | 14.728 | 26.611 | 34.961 | 1.00 | 42.97 | C |
| ATOM | 481 | CB | ARG | A | 102 | 13.380 | 26.616 | 35.658 | 1.00 | 35.14 | C |
| ATOM | 482 | CG | ARG | A | 102 | 12.268 | 26.172 | 34.777 | 1.00 | 35.14 | C |
| ATOM | 483 | CD | ARG | A | 102 | 10.987 | 26.416 | 35.473 | 1.00 | 35.14 | C |
| ATOM | 484 | NE | ARG | A | 102 | 10.913 | 27.816 | 35.866 | 1.00 | 35.14 | N |
| ATOM | 485 | CZ | ARG | A | 102 | 9.893 | 28.361 | 36.526 | 1.00 | 35.14 | C |
| ATOM | 486 | NH1 | ARG | A | 102 | 8.841 | 27.623 | 36.881 | 1.00 | 35.14 | N |
| ATOM | 487 | NH2 | ARG | A | 102 | 9.922 | 29.656 | 36.821 | 1.00 | 35.14 | N |
| ATOM | 488 | C | ARG | A | 102 | 15.757 | 27.264 | 35.882 | 1.00 | 42.97 | C |
| ATOM | 489 | O | ARG | A | 102 | 15.767 | 28.483 | 36.025 | 1.00 | 42.97 | O |
| ATOM | 490 | N | LYS | A | 103 | 16.624 | 26.480 | 36.508 | 1.00 | 42.60 | N |
| ATOM | 491 | CA | LYS | A | 103 | 17.605 | 27.066 | 37.405 | 1.00 | 42.60 | C |
| ATOM | 492 | CB | LYS | A | 103 | 17.454 | 26.465 | 38.800 | 1.00 | 41.71 | C |
| ATOM | 493 | CG | LYS | A | 103 | 17.505 | 24.957 | 38.839 | 1.00 | 41.71 | C |
| ATOM | 494 | CD | LYS | A | 103 | 17.237 | 24.459 | 40.260 | 1.00 | 41.71 | C |
| ATOM | 495 | CE | LYS | A | 103 | 16.878 | 22.970 | 40.294 | 1.00 | 41.71 | C |
| ATOM | 496 | NZ | LYS | A | 103 | 16.500 | 22.530 | 41.657 | 1.00 | 41.71 | N |
| ATOM | 497 | C | LYS | A | 103 | 19.046 | 26.922 | 36.937 | 1.00 | 42.60 | C |
| ATOM | 498 | O | LYS | A | 103 | 19.975 | 27.044 | 37.733 | 1.00 | 42.60 | O |
| ATOM | 499 | N | LEU | A | 104 | 19.239 | 26.664 | 35.650 | 1.00 | 32.41 | N |

| | | | | | | | | | | | |
|---|---|---|---|---|---|---|---|---|---|---|---|
| ATOM | 500 | CA | LEU | A | 104 | 20.587 | 26.516 | 35.112 | 1.00 | 32.41 | C |
| ATOM | 501 | CB | LEU | A | 104 | 20.657 | 25.293 | 34.190 | 1.00 | 33.79 | C |
| ATOM | 502 | CG | LEU | A | 104 | 20.922 | 23.927 | 34.832 | 1.00 | 33.79 | C |
| ATOM | 503 | CD1 | LEU | A | 104 | 20.276 | 23.834 | 36.192 | 1.00 | 33.79 | C |
| ATOM | 504 | CD2 | LEU | A | 104 | 20.397 | 22.849 | 33.928 | 1.00 | 33.79 | C |
| ATOM | 505 | C | LEU | A | 104 | 20.981 | 27.763 | 34.341 | 1.00 | 32.41 | C |
| ATOM | 506 | O | LEU | A | 104 | 20.145 | 28.398 | 33.710 | 1.00 | 32.41 | O |
| ATOM | 507 | N | ASP | A | 105 | 22.255 | 28.123 | 34.403 | 1.00 | 36.32 | N |
| ATOM | 508 | CA | ASP | A | 105 | 22.731 | 29.299 | 33.691 | 1.00 | 36.32 | C |
| ATOM | 509 | CB | ASP | A | 105 | 22.407 | 30.586 | 34.448 | 1.00 | 33.72 | C |
| ATOM | 510 | CG | ASP | A | 105 | 22.860 | 31.824 | 33.698 | 1.00 | 33.72 | C |
| ATOM | 511 | OD1 | ASP | A | 105 | 22.981 | 32.886 | 34.334 | 1.00 | 33.72 | O |
| ATOM | 512 | OD2 | ASP | A | 105 | 23.077 | 31.730 | 32.466 | 1.00 | 33.72 | O |
| ATOM | 513 | C | ASP | A | 105 | 24.223 | 29.198 | 33.526 | 1.00 | 36.32 | C |
| ATOM | 514 | O | ASP | A | 105 | 24.994 | 29.720 | 34.346 | 1.00 | 36.32 | O |
| ATOM | 515 | N | HIS | A | 106 | 24.622 | 28.514 | 32.463 | 1.00 | 64.52 | N |
| ATOM | 516 | CA | HIS | A | 106 | 26.027 | 28.303 | 32.160 | 1.00 | 64.52 | C |
| ATOM | 517 | CB | HIS | A | 106 | 26.426 | 26.881 | 32.575 | 1.00 | 45.87 | C |
| ATOM | 518 | CG | HIS | A | 106 | 27.899 | 26.622 | 32.538 | 1.00 | 45.87 | C |
| ATOM | 519 | CD2 | HIS | A | 106 | 28.885 | 26.957 | 33.391 | 1.00 | 45.87 | C |
| ATOM | 520 | ND1 | HIS | A | 106 | 28.498 | 25.915 | 31.515 | 1.00 | 45.87 | N |
| ATOM | 521 | CE1 | HIS | A | 106 | 29.795 | 25.825 | 31.749 | 1.00 | 45.87 | C |
| ATOM | 522 | NE2 | HIS | A | 106 | 30.060 | 26.449 | 32.876 | 1.00 | 45.87 | N |
| ATOM | 523 | C | HIS | A | 106 | 26.258 | 28.519 | 30.663 | 1.00 | 64.52 | C |
| ATOM | 524 | O | HIS | A | 106 | 25.331 | 28.369 | 29.857 | 1.00 | 64.52 | O |
| ATOM | 525 | N | CYS | A | 107 | 27.495 | 28.861 | 30.305 | 1.00 | 38.72 | N |
| ATOM | 526 | CA | CYS | A | 107 | 27.901 | 29.124 | 28.920 | 1.00 | 38.72 | C |
| ATOM | 527 | CB | CYS | A | 107 | 29.303 | 29.716 | 28.890 | 1.00 | 49.67 | C |
| ATOM | 528 | SG | CYS | A | 107 | 30.473 | 28.762 | 29.771 | 1.00 | 49.67 | S |
| ATOM | 529 | C | CYS | A | 107 | 27.883 | 27.912 | 28.018 | 1.00 | 38.72 | C |
| ATOM | 530 | O | CYS | A | 107 | 27.878 | 28.040 | 26.797 | 1.00 | 38.72 | O |
| ATOM | 531 | N | ASN | A | 108 | 27.874 | 26.734 | 28.615 | 1.00 | 26.21 | N |
| ATOM | 532 | CA | ASN | A | 108 | 27.881 | 25.513 | 27.833 | 1.00 | 26.21 | C |
| ATOM | 533 | CB | ASN | A | 108 | 29.206 | 24.810 | 28.043 | 1.00 | 39.72 | C |
| ATOM | 534 | CG | ASN | A | 108 | 30.338 | 25.528 | 27.357 | 1.00 | 39.72 | C |
| ATOM | 535 | OD1 | ASN | A | 108 | 31.384 | 25.743 | 27.943 | 1.00 | 39.72 | O |
| ATOM | 536 | ND2 | ASN | A | 108 | 30.127 | 25.913 | 26.101 | 1.00 | 39.72 | N |
| ATOM | 537 | C | ASN | A | 108 | 26.691 | 24.594 | 28.125 | 1.00 | 26.21 | C |
| ATOM | 538 | O | ASN | A | 108 | 26.769 | 23.361 | 28.098 | 1.00 | 26.21 | O |
| ATOM | 539 | N | ILE | A | 109 | 25.577 | 25.255 | 28.386 | 1.00 | 27.27 | N |
| ATOM | 540 | CA | ILE | A | 109 | 24.305 | 24.624 | 28.625 | 1.00 | 27.27 | C |
| ATOM | 541 | CB | ILE | A | 109 | 23.998 | 24.596 | 30.101 | 1.00 | 16.97 | C |
| ATOM | 542 | CG2 | ILE | A | 109 | 22.574 | 24.094 | 30.329 | 1.00 | 16.97 | C |
| ATOM | 543 | CG1 | ILE | A | 109 | 25.035 | 23.702 | 30.796 | 1.00 | 16.97 | C |
| ATOM | 544 | CD1 | ILE | A | 109 | 24.817 | 23.540 | 32.313 | 1.00 | 16.97 | C |
| ATOM | 545 | C | ILE | A | 109 | 23.311 | 25.492 | 27.863 | 1.00 | 27.27 | C |
| ATOM | 546 | O | ILE | A | 109 | 23.413 | 26.721 | 27.880 | 1.00 | 27.27 | O |
| ATOM | 547 | N | VAL | A | 110 | 22.380 | 24.850 | 27.166 | 1.00 | 26.69 | N |
| ATOM | 548 | CA | VAL | A | 110 | 21.401 | 25.584 | 26.390 | 1.00 | 26.69 | C |
| ATOM | 549 | CB | VAL | A | 110 | 20.447 | 24.680 | 25.655 | 1.00 | 17.52 | C |
| ATOM | 550 | CG1 | VAL | A | 110 | 19.954 | 25.387 | 24.402 | 1.00 | 17.52 | C |
| ATOM | 551 | CG2 | VAL | A | 110 | 21.117 | 23.380 | 25.339 | 1.00 | 17.52 | C |
| ATOM | 552 | C | VAL | A | 110 | 20.558 | 26.453 | 27.278 | 1.00 | 26.69 | C |
| ATOM | 553 | O | VAL | A | 110 | 20.073 | 26.018 | 28.324 | 1.00 | 26.69 | O |
| ATOM | 554 | N | ARG | A | 111 | 20.373 | 27.689 | 26.850 | 1.00 | 23.32 | N |
| ATOM | 555 | CA | ARG | A | 111 | 19.570 | 28.614 | 27.609 | 1.00 | 23.32 | C |
| ATOM | 556 | CB | ARG | A | 111 | 19.996 | 30.029 | 27.254 | 1.00 | 47.27 | C |
| ATOM | 557 | CG | ARG | A | 111 | 18.939 | 31.101 | 27.396 | 1.00 | 47.27 | C |
| ATOM | 558 | CD | ARG | A | 111 | 19.656 | 32.430 | 27.444 | 1.00 | 47.27 | C |
| ATOM | 559 | NE | ARG | A | 111 | 18.834 | 33.595 | 27.134 | 1.00 | 47.27 | N |
| ATOM | 560 | CZ | ARG | A | 111 | 18.918 | 34.292 | 26.003 | 1.00 | 47.27 | C |
| ATOM | 561 | NH1 | ARG | A | 111 | 19.783 | 33.940 | 25.058 | 1.00 | 47.27 | N |
| ATOM | 562 | NH2 | ARG | A | 111 | 18.159 | 35.366 | 25.835 | 1.00 | 47.27 | N |

| | | | | | | | | | | | |
|---|---|---|---|---|---|---|---|---|---|---|---|
| ATOM | 563 | C | ARG | A | 111 | 18.078 | 28.417 | 27.358 | 1.00 | 23.32 | C |
| ATOM | 564 | O | ARG | A | 111 | 17.626 | 28.420 | 26.208 | 1.00 | 23.32 | O |
| ATOM | 565 | N | LEU | A | 112 | 17.325 | 28.209 | 28.437 | 1.00 | 27.65 | N |
| ATOM | 566 | CA | LEU | A | 112 | 15.873 | 28.056 | 28.344 | 1.00 | 27.65 | C |
| ATOM | 567 | CB | LEU | A | 112 | 15.318 | 27.284 | 29.543 | 1.00 | 33.64 | C |
| ATOM | 568 | CG | LEU | A | 112 | 13.790 | 27.280 | 29.620 | 1.00 | 33.64 | C |
| ATOM | 569 | CD1 | LEU | A | 112 | 13.194 | 26.362 | 28.540 | 1.00 | 33.64 | C |
| ATOM | 570 | CD2 | LEU | A | 112 | 13.365 | 26.828 | 31.013 | 1.00 | 33.64 | C |
| ATOM | 571 | C | LEU | A | 112 | 15.303 | 29.473 | 28.337 | 1.00 | 27.65 | C |
| ATOM | 572 | O | LEU | A | 112 | 15.488 | 30.236 | 29.278 | 1.00 | 27.65 | O |
| ATOM | 573 | N | ARG | A | 113 | 14.608 | 29.822 | 27.268 | 1.00 | 32.67 | N |
| ATOM | 574 | CA | ARG | A | 113 | 14.060 | 31.162 | 27.140 | 1.00 | 32.67 | C |
| ATOM | 575 | CB | ARG | A | 113 | 13.993 | 31.538 | 25.661 | 1.00 | 52.67 | C |
| ATOM | 576 | CG | ARG | A | 113 | 15.343 | 31.495 | 24.963 | 1.00 | 52.67 | C |
| ATOM | 577 | CD | ARG | A | 113 | 15.729 | 32.863 | 24.477 | 1.00 | 52.67 | C |
| ATOM | 578 | NE | ARG | A | 113 | 16.140 | 32.835 | 23.078 | 1.00 | 52.67 | N |
| ATOM | 579 | CZ | ARG | A | 113 | 16.036 | 33.875 | 22.255 | 1.00 | 52.67 | C |
| ATOM | 580 | NH1 | ARG | A | 113 | 15.531 | 35.030 | 22.690 | 1.00 | 52.67 | N |
| ATOM | 581 | NH2 | ARG | A | 113 | 16.431 | 33.763 | 20.995 | 1.00 | 52.67 | N |
| ATOM | 582 | C | ARG | A | 113 | 12.696 | 31.307 | 27.771 | 1.00 | 32.67 | C |
| ATOM | 583 | O | ARG | A | 113 | 12.530 | 32.015 | 28.762 | 1.00 | 32.67 | O |
| ATOM | 584 | N | TYR | A | 114 | 11.723 | 30.637 | 27.168 | 1.00 | 39.41 | N |
| ATOM | 585 | CA | TYR | A | 114 | 10.354 | 30.664 | 27.644 | 1.00 | 39.41 | C |
| ATOM | 586 | CB | TYR | A | 114 | 9.440 | 31.357 | 26.631 | 1.00 | 38.00 | C |
| ATOM | 587 | CG | TYR | A | 114 | 9.888 | 32.739 | 26.225 | 1.00 | 38.00 | C |
| ATOM | 588 | CD1 | TYR | A | 114 | 9.689 | 33.832 | 27.071 | 1.00 | 38.00 | C |
| ATOM | 589 | CE1 | TYR | A | 114 | 10.130 | 35.097 | 26.719 | 1.00 | 38.00 | C |
| ATOM | 590 | CD2 | TYR | A | 114 | 10.541 | 32.952 | 25.008 | 1.00 | 38.00 | C |
| ATOM | 591 | CE2 | TYR | A | 114 | 10.993 | 34.216 | 24.646 | 1.00 | 38.00 | C |
| ATOM | 592 | CZ | TYR | A | 114 | 10.784 | 35.287 | 25.508 | 1.00 | 38.00 | C |
| ATOM | 593 | OH | TYR | A | 114 | 11.250 | 36.545 | 25.175 | 1.00 | 38.00 | O |
| ATOM | 594 | C | TYR | A | 114 | 9.940 | 29.220 | 27.731 | 1.00 | 39.41 | C |
| ATOM | 595 | O | TYR | A | 114 | 10.720 | 28.317 | 27.431 | 1.00 | 39.41 | O |
| ATOM | 596 | N | PHE | A | 115 | 8.702 | 29.010 | 28.144 | 1.00 | 42.29 | N |
| ATOM | 597 | CA | PHE | A | 115 | 8.138 | 27.676 | 28.224 | 1.00 | 42.29 | C |
| ATOM | 598 | CB | PHE | A | 115 | 8.713 | 26.868 | 29.395 | 1.00 | 29.66 | C |
| ATOM | 599 | CG | PHE | A | 115 | 8.308 | 27.376 | 30.743 | 1.00 | 29.66 | C |
| ATOM | 600 | CD1 | PHE | A | 115 | 7.195 | 26.850 | 31.400 | 1.00 | 29.66 | C |
| ATOM | 601 | CD2 | PHE | A | 115 | 9.046 | 28.382 | 31.365 | 1.00 | 29.66 | C |
| ATOM | 602 | CE1 | PHE | A | 115 | 6.832 | 27.308 | 32.642 | 1.00 | 29.66 | C |
| ATOM | 603 | CE2 | PHE | A | 115 | 8.695 | 28.850 | 32.608 | 1.00 | 29.66 | C |
| ATOM | 604 | CZ | PHE | A | 115 | 7.584 | 28.314 | 33.256 | 1.00 | 29.66 | C |
| ATOM | 605 | C | PHE | A | 115 | 6.649 | 27.891 | 28.378 | 1.00 | 42.29 | C |
| ATOM | 606 | O | PHE | A | 115 | 6.186 | 28.977 | 28.753 | 1.00 | 42.29 | O |
| ATOM | 607 | N | PHE | A | 116 | 5.902 | 26.853 | 28.046 | 1.00 | 38.49 | N |
| ATOM | 608 | CA | PHE | A | 116 | 4.465 | 26.913 | 28.126 | 1.00 | 38.49 | C |
| ATOM | 609 | CB | PHE | A | 116 | 3.941 | 27.879 | 27.062 | 1.00 | 47.12 | C |
| ATOM | 610 | CG | PHE | A | 116 | 4.256 | 27.466 | 25.653 | 1.00 | 47.12 | C |
| ATOM | 611 | CD1 | PHE | A | 116 | 3.363 | 26.677 | 24.938 | 1.00 | 47.12 | C |
| ATOM | 612 | CD2 | PHE | A | 116 | 5.426 | 27.886 | 25.026 | 1.00 | 47.12 | C |
| ATOM | 613 | CE1 | PHE | A | 116 | 3.619 | 26.313 | 23.616 | 1.00 | 47.12 | C |
| ATOM | 614 | CE2 | PHE | A | 116 | 5.693 | 27.523 | 23.691 | 1.00 | 47.12 | C |
| ATOM | 615 | CZ | PHE | A | 116 | 4.785 | 26.738 | 22.990 | 1.00 | 47.12 | C |
| ATOM | 616 | C | PHE | A | 116 | 3.916 | 25.520 | 27.917 | 1.00 | 38.49 | C |
| ATOM | 617 | O | PHE | A | 116 | 4.579 | 24.658 | 27.332 | 1.00 | 38.49 | O |
| ATOM | 618 | N | TYR | A | 117 | 2.713 | 25.295 | 28.426 | 1.00 | 36.04 | N |
| ATOM | 619 | CA | TYR | A | 117 | 2.065 | 23.995 | 28.296 | 1.00 | 36.04 | C |
| ATOM | 620 | CB | TYR | A | 117 | 1.393 | 23.604 | 29.609 | 1.00 | 41.26 | C |
| ATOM | 621 | CG | TYR | A | 117 | 2.374 | 23.114 | 30.634 | 1.00 | 41.26 | C |
| ATOM | 622 | CD1 | TYR | A | 117 | 2.787 | 21.794 | 30.636 | 1.00 | 41.26 | C |
| ATOM | 623 | CE1 | TYR | A | 117 | 3.727 | 21.340 | 31.542 | 1.00 | 41.26 | C |
| ATOM | 624 | CD2 | TYR | A | 117 | 2.923 | 23.980 | 31.570 | 1.00 | 41.26 | C |
| ATOM | 625 | CE2 | TYR | A | 117 | 3.867 | 23.543 | 32.484 | 1.00 | 41.26 | C |

| | | | | | | | | | | | |
|---|---|---|---|---|---|---|---|---|---|---|---|
| ATOM | 626 | CZ | TYR | A | 117 | 4.269 | 22.217 | 32.469 | 1.00 | 41.26 | C |
| ATOM | 627 | OH | TYR | A | 117 | 5.209 | 21.758 | 33.379 | 1.00 | 41.26 | O |
| ATOM | 628 | C | TYR | A | 117 | 1.048 | 24.056 | 27.179 | 1.00 | 36.04 | C |
| ATOM | 629 | O | TYR | A | 117 | 0.569 | 25.138 | 26.837 | 1.00 | 36.04 | O |
| ATOM | 630 | N | SER | A | 118 | 0.724 | 22.893 | 26.616 | 1.00 | 28.41 | N |
| ATOM | 631 | CA | SER | A | 118 | -0.216 | 22.817 | 25.500 | 1.00 | 28.41 | C |
| ATOM | 632 | CB | SER | A | 118 | 0.520 | 23.061 | 24.181 | 1.00 | 53.28 | C |
| ATOM | 633 | OG | SER | A | 118 | 0.413 | 21.936 | 23.323 | 1.00 | 53.28 | O |
| ATOM | 634 | C | SER | A | 118 | -0.897 | 21.475 | 25.438 | 1.00 | 28.41 | C |
| ATOM | 635 | O | SER | A | 118 | -0.470 | 20.514 | 26.099 | 1.00 | 28.41 | O |
| ATOM | 636 | N | SER | A | 119 | -1.948 | 21.380 | 24.637 | 1.00 | 67.47 | N |
| ATOM | 637 | CA | SER | A | 119 | -2.622 | 20.096 | 24.548 | 1.00 | 67.47 | C |
| ATOM | 638 | CB | SER | A | 119 | -4.115 | 20.205 | 24.863 | 1.00 | 58.15 | C |
| ATOM | 639 | OG | SER | A | 119 | -4.565 | 21.552 | 25.033 | 1.00 | 58.15 | O |
| ATOM | 640 | C | SER | A | 119 | -2.424 | 19.474 | 23.168 | 1.00 | 67.47 | C |
| ATOM | 641 | O | SER | A | 119 | -2.324 | 20.124 | 22.143 | 1.00 | 67.47 | O |
| ATOM | 642 | N | GLY | A | 120 | -2.341 | 18.163 | 23.180 | 1.00 | 79.78 | N |
| ATOM | 643 | CA | GLY | A | 120 | -2.139 | 17.413 | 21.958 | 1.00 | 79.78 | C |
| ATOM | 644 | C | GLY | A | 120 | -3.323 | 16.570 | 21.472 | 1.00 | 79.78 | C |
| ATOM | 645 | O | GLY | A | 120 | -3.155 | 15.430 | 20.977 | 1.00 | 79.78 | O |
| ATOM | 646 | N | ALA | A | 121 | -4.534 | 17.093 | 21.661 | 1.00103.54 | | N |
| ATOM | 647 | CA | ALA | A | 121 | -5.739 | 16.425 | 21.195 | 1.00103.54 | | C |
| ATOM | 648 | CB | ALA | A | 121 | -5.803 | 16.537 | 19.683 | 1.00 | 48.24 | C |
| ATOM | 649 | C | ALA | A | 121 | -5.826 | 14.970 | 21.621 | 1.00103.54 | | C |
| ATOM | 650 | O | ALA | A | 121 | -5.925 | 14.088 | 20.780 | 1.00103.54 | | O |
| ATOM | 651 | N | GLY | A | 122 | -5.799 | 14.725 | 22.926 | 1.00 | 72.86 | N |
| ATOM | 652 | CA | GLY | A | 122 | -5.888 | 13.363 | 23.414 | 1.00 | 72.86 | C |
| ATOM | 653 | C | GLY | A | 122 | -6.871 | 13.218 | 24.554 | 1.00 | 72.86 | C |
| ATOM | 654 | O | GLY | A | 122 | -7.764 | 14.052 | 24.728 | 1.00 | 72.86 | O |
| ATOM | 655 | N | GLY | A | 123 | -6.708 | 12.156 | 25.334 | 1.00 | 80.74 | N |
| ATOM | 656 | CA | GLY | A | 123 | -7.599 | 11.925 | 26.460 | 1.00 | 80.74 | C |
| ATOM | 657 | C | GLY | A | 123 | -6.945 | 12.401 | 27.732 | 1.00 | 80.74 | C |
| ATOM | 658 | O | GLY | A | 123 | -6.390 | 11.612 | 28.502 | 1.00 | 80.74 | O |
| ATOM | 659 | N | ALA | A | 124 | -7.005 | 13.706 | 27.944 | 1.00 | 74.32 | N |
| ATOM | 660 | CA | ALA | A | 124 | -6.387 | 14.309 | 29.110 | 1.00 | 74.32 | C |
| ATOM | 661 | CB | ALA | A | 124 | -6.918 | 13.675 | 30.437 | 1.00 | 89.05 | C |
| ATOM | 662 | C | ALA | A | 124 | -4.898 | 14.152 | 28.974 | 1.00 | 74.32 | C |
| ATOM | 663 | O | ALA | A | 124 | -4.296 | 13.357 | 29.682 | 1.00 | 74.32 | O |
| ATOM | 664 | N | GLU | A | 125 | -4.319 | 14.891 | 28.030 | 1.00 | 72.44 | N |
| ATOM | 665 | CA | GLU | A | 125 | -2.883 | 14.826 | 27.807 | 1.00 | 72.44 | C |
| ATOM | 666 | CB | GLU | A | 125 | -2.572 | 13.809 | 26.699 | 1.00 | 85.94 | C |
| ATOM | 667 | CG | GLU | A | 125 | -2.424 | 14.342 | 25.276 | 1.00 | 85.94 | C |
| ATOM | 668 | CD | GLU | A | 125 | -2.266 | 13.192 | 24.281 | 1.00 | 85.94 | C |
| ATOM | 669 | OE1 | GLU | A | 125 | -2.385 | 13.430 | 23.058 | 1.00 | 85.94 | O |
| ATOM | 670 | OE2 | GLU | A | 125 | -2.025 | 12.047 | 24.740 | 1.00 | 85.94 | O |
| ATOM | 671 | C | GLU | A | 125 | -2.252 | 16.205 | 27.534 | 1.00 | 72.44 | C |
| ATOM | 672 | O | GLU | A | 125 | -2.574 | 16.878 | 26.574 | 1.00 | 72.44 | O |
| ATOM | 673 | N | VAL | A | 126 | -1.342 | 16.585 | 28.422 | 1.00 | 44.28 | N |
| ATOM | 674 | CA | VAL | A | 126 | -0.614 | 17.858 | 28.482 | 1.00 | 44.28 | C |
| ATOM | 675 | CB | VAL | A | 126 | -0.552 | 18.180 | 29.961 | 1.00 | 65.54 | C |
| ATOM | 676 | CG1 | VAL | A | 126 | 0.171 | 19.442 | 30.244 | 1.00 | 65.54 | C |
| ATOM | 677 | CG2 | VAL | A | 126 | -2.001 | 18.182 | 30.510 | 1.00 | 65.54 | C |
| ATOM | 678 | C | VAL | A | 126 | 0.833 | 17.743 | 27.869 | 1.00 | 44.28 | C |
| ATOM | 679 | O | VAL | A | 126 | 1.531 | 16.721 | 27.975 | 1.00 | 44.28 | O |
| ATOM | 680 | N | TYR | A | 127 | 1.270 | 18.778 | 27.162 | 1.00 | 41.43 | N |
| ATOM | 681 | CA | TYR | A | 127 | 2.659 | 18.774 | 26.629 | 1.00 | 41.43 | C |
| ATOM | 682 | CB | TYR | A | 127 | 2.723 | 18.869 | 25.084 | 1.00 | 75.34 | C |
| ATOM | 683 | CG | TYR | A | 127 | 2.353 | 17.591 | 24.347 | 1.00 | 75.34 | C |
| ATOM | 684 | CD1 | TYR | A | 127 | 1.031 | 17.336 | 24.015 | 1.00 | 75.34 | C |
| ATOM | 685 | CE1 | TYR | A | 127 | 0.656 | 16.161 | 23.401 | 1.00 | 75.34 | C |
| ATOM | 686 | CD2 | TYR | A | 127 | 3.312 | 16.618 | 24.024 | 1.00 | 75.34 | C |
| ATOM | 687 | CE2 | TYR | A | 127 | 2.942 | 15.430 | 23.402 | 1.00 | 75.34 | C |
| ATOM | 688 | CZ | TYR | A | 127 | 1.612 | 15.210 | 23.096 | 1.00 | 75.34 | C |

| | | | | | | | | | | | |
|---|---|---|---|---|---|---|---|---|---|---|---|
| ATOM | 689 | OH | TYR | A | 127 | 1.234 | 14.017 | 22.520 | 1.00 | 75.34 | O |
| ATOM | 690 | C | TYR | A | 127 | 3.458 | 19.945 | 27.194 | 1.00 | 41.43 | C |
| ATOM | 691 | O | TYR | A | 127 | 2.903 | 21.002 | 27.495 | 1.00 | 41.43 | O |
| ATOM | 692 | N | LEU | A | 128 | 4.757 | 19.755 | 27.385 | 1.00 | 34.78 | N |
| ATOM | 693 | CA | LEU | A | 128 | 5.567 | 20.876 | 27.848 | 1.00 | 34.78 | C |
| ATOM | 694 | CB | LEU | A | 128 | 6.526 | 20.493 | 28.990 | 1.00 | 51.00 | C |
| ATOM | 695 | CG | LEU | A | 128 | 7.729 | 21.455 | 28.976 | 1.00 | 51.00 | C |
| ATOM | 696 | CD1 | LEU | A | 128 | 7.313 | 22.821 | 29.502 | 1.00 | 51.00 | C |
| ATOM | 697 | CD2 | LEU | A | 128 | 8.870 | 20.886 | 29.776 | 1.00 | 51.00 | C |
| ATOM | 698 | C | LEU | A | 128 | 6.394 | 21.428 | 26.689 | 1.00 | 34.78 | C |
| ATOM | 699 | O | LEU | A | 128 | 7.169 | 20.711 | 26.076 | 1.00 | 34.78 | O |
| ATOM | 700 | N | ASN | A | 129 | 6.259 | 22.710 | 26.401 | 1.00 | 36.64 | N |
| ATOM | 701 | CA | ASN | A | 129 | 7.042 | 23.289 | 25.315 | 1.00 | 36.64 | C |
| ATOM | 702 | CB | ASN | A | 129 | 6.145 | 24.104 | 24.390 | 1.00 | 48.50 | C |
| ATOM | 703 | CG | ASN | A | 129 | 4.900 | 23.351 | 23.985 | 1.00 | 48.50 | C |
| ATOM | 704 | OD1 | ASN | A | 129 | 4.708 | 23.037 | 22.815 | 1.00 | 48.50 | O |
| ATOM | 705 | ND2 | ASN | A | 129 | 4.043 | 23.052 | 24.959 | 1.00 | 48.50 | N |
| ATOM | 706 | C | ASN | A | 129 | 8.162 | 24.170 | 25.860 | 1.00 | 36.64 | C |
| ATOM | 707 | O | ASN | A | 129 | 7.922 | 25.139 | 26.573 | 1.00 | 36.64 | O |
| ATOM | 708 | N | LEU | A | 130 | 9.392 | 23.823 | 25.513 | 1.00 | 39.80 | N |
| ATOM | 709 | CA | LEU | A | 130 | 10.542 | 24.571 | 25.971 | 1.00 | 39.80 | C |
| ATOM | 710 | CB | LEU | A | 130 | 11.577 | 23.604 | 26.536 | 1.00 | 23.13 | C |
| ATOM | 711 | CG | LEU | A | 130 | 11.104 | 22.763 | 27.721 | 1.00 | 23.13 | C |
| ATOM | 712 | CD1 | LEU | A | 130 | 12.071 | 21.621 | 27.975 | 1.00 | 23.13 | C |
| ATOM | 713 | CD2 | LEU | A | 130 | 10.988 | 23.652 | 28.937 | 1.00 | 23.13 | C |
| ATOM | 714 | C | LEU | A | 130 | 11.152 | 25.352 | 24.825 | 1.00 | 39.80 | C |
| ATOM | 715 | O | LEU | A | 130 | 11.647 | 24.770 | 23.873 | 1.00 | 39.80 | O |
| ATOM | 716 | N | VAL | A | 131 | 11.103 | 26.672 | 24.907 | 1.00 | 29.49 | N |
| ATOM | 717 | CA | VAL | A | 131 | 11.690 | 27.506 | 23.872 | 1.00 | 29.49 | C |
| ATOM | 718 | CB | VAL | A | 131 | 10.959 | 28.864 | 23.756 | 1.00 | 35.65 | C |
| ATOM | 719 | CG1 | VAL | A | 131 | 11.474 | 29.634 | 22.528 | 1.00 | 35.65 | C |
| ATOM | 720 | CG2 | VAL | A | 131 | 9.453 | 28.633 | 23.672 | 1.00 | 35.65 | C |
| ATOM | 721 | C | VAL | A | 131 | 13.140 | 27.738 | 24.298 | 1.00 | 29.49 | C |
| ATOM | 722 | O | VAL | A | 131 | 13.413 | 28.353 | 25.337 | 1.00 | 29.49 | O |
| ATOM | 723 | N | LEU | A | 132 | 14.068 | 27.240 | 23.494 | 1.00 | 34.39 | N |
| ATOM | 724 | CA | LEU | A | 132 | 15.480 | 27.360 | 23.811 | 1.00 | 34.39 | C |
| ATOM | 725 | CB | LEU | A | 132 | 16.075 | 25.956 | 23.921 | 1.00 | 23.32 | C |
| ATOM | 726 | CG | LEU | A | 132 | 15.185 | 24.921 | 24.636 | 1.00 | 23.32 | C |
| ATOM | 727 | CD1 | LEU | A | 132 | 15.772 | 23.544 | 24.465 | 1.00 | 23.32 | C |
| ATOM | 728 | CD2 | LEU | A | 132 | 15.044 | 25.261 | 26.122 | 1.00 | 23.32 | C |
| ATOM | 729 | C | LEU | A | 132 | 16.213 | 28.148 | 22.736 | 1.00 | 34.39 | C |
| ATOM | 730 | O | LEU | A | 132 | 15.598 | 28.631 | 21.784 | 1.00 | 34.39 | O |
| ATOM | 731 | N | ASP | A | 133 | 17.522 | 28.315 | 22.909 | 1.00 | 31.22 | N |
| ATOM | 732 | CA | ASP | A | 133 | 18.315 | 28.992 | 21.898 | 1.00 | 31.22 | C |
| ATOM | 733 | CB | ASP | A | 133 | 19.705 | 29.326 | 22.418 | 1.00 | 32.83 | C |
| ATOM | 734 | CG | ASP | A | 133 | 19.734 | 30.593 | 23.205 | 1.00 | 32.83 | C |
| ATOM | 735 | OD1 | ASP | A | 133 | 20.748 | 30.845 | 23.882 | 1.00 | 32.83 | O |
| ATOM | 736 | OD2 | ASP | A | 133 | 18.745 | 31.347 | 23.145 | 1.00 | 32.83 | O |
| ATOM | 737 | C | ASP | A | 133 | 18.456 | 27.954 | 20.804 | 1.00 | 31.22 | C |
| ATOM | 738 | O | ASP | A | 133 | 18.453 | 26.749 | 21.085 | 1.00 | 31.22 | O |
| ATOM | 739 | N | TYR | A | 134 | 18.573 | 28.390 | 19.561 | 1.00 | 25.59 | N |
| ATOM | 740 | CA | TYR | A | 134 | 18.736 | 27.419 | 18.494 | 1.00 | 25.59 | C |
| ATOM | 741 | CB | TYR | A | 134 | 18.066 | 27.906 | 17.210 | 1.00 | 55.10 | C |
| ATOM | 742 | CG | TYR | A | 134 | 18.352 | 26.993 | 16.051 | 1.00 | 55.10 | C |
| ATOM | 743 | CD1 | TYR | A | 134 | 17.736 | 25.746 | 15.951 | 1.00 | 55.10 | C |
| ATOM | 744 | CE1 | TYR | A | 134 | 18.069 | 24.862 | 14.932 | 1.00 | 55.10 | C |
| ATOM | 745 | CD2 | TYR | A | 134 | 19.304 | 27.334 | 15.097 | 1.00 | 55.10 | C |
| ATOM | 746 | CE2 | TYR | A | 134 | 19.642 | 26.457 | 14.081 | 1.00 | 55.10 | C |
| ATOM | 747 | CZ | TYR | A | 134 | 19.025 | 25.226 | 14.003 | 1.00 | 55.10 | C |
| ATOM | 748 | OH | TYR | A | 134 | 19.368 | 24.357 | 12.992 | 1.00 | 55.10 | O |
| ATOM | 749 | C | TYR | A | 134 | 20.228 | 27.151 | 18.226 | 1.00 | 25.59 | C |
| ATOM | 750 | O | TYR | A | 134 | 20.997 | 28.087 | 17.999 | 1.00 | 25.59 | O |
| ATOM | 751 | N | VAL | A | 135 | 20.632 | 25.880 | 18.270 | 1.00 | 32.70 | N |

| | | | | | | | | | | | |
|---|---|---|---|---|---|---|---|---|---|---|---|
| ATOM | 752 | CA | VAL | A | 135 | 22.020 | 25.495 | 18.005 | 1.00 | 32.70 | C |
| ATOM | 753 | CB | VAL | A | 135 | 22.619 | 24.710 | 19.182 | 1.00 | 41.13 | C |
| ATOM | 754 | CG1 | VAL | A | 135 | 24.117 | 24.526 | 18.984 | 1.00 | 41.13 | C |
| ATOM | 755 | CG2 | VAL | A | 135 | 22.353 | 25.457 | 20.461 | 1.00 | 41.13 | C |
| ATOM | 756 | C | VAL | A | 135 | 22.003 | 24.636 | 16.738 | 1.00 | 32.70 | C |
| ATOM | 757 | O | VAL | A | 135 | 21.388 | 23.564 | 16.713 | 1.00 | 32.70 | O |
| ATOM | 758 | N | PRO | A | 136 | 22.709 | 25.088 | 15.679 | 1.00 | 28.31 | N |
| ATOM | 759 | CD | PRO | A | 136 | 23.751 | 26.123 | 15.783 | 1.00 | 33.29 | C |
| ATOM | 760 | CA | PRO | A | 136 | 22.800 | 24.431 | 14.371 | 1.00 | 28.31 | C |
| ATOM | 761 | CB | PRO | A | 136 | 23.847 | 25.269 | 13.632 | 1.00 | 33.29 | C |
| ATOM | 762 | CG | PRO | A | 136 | 23.866 | 26.564 | 14.364 | 1.00 | 33.29 | C |
| ATOM | 763 | C | PRO | A | 136 | 23.131 | 22.940 | 14.289 | 1.00 | 28.31 | C |
| ATOM | 764 | O | PRO | A | 136 | 22.544 | 22.222 | 13.479 | 1.00 | 28.31 | O |
| ATOM | 765 | N | GLU | A | 137 | 24.067 | 22.470 | 15.106 | 1.00 | 23.09 | N |
| ATOM | 766 | CA | GLU | A | 137 | 24.449 | 21.074 | 15.009 | 1.00 | 23.09 | C |
| ATOM | 767 | CB | GLU | A | 137 | 25.725 | 20.972 | 14.148 | 1.00 | 74.63 | C |
| ATOM | 768 | CG | GLU | A | 137 | 25.668 | 19.979 | 12.975 | 1.00 | 74.63 | C |
| ATOM | 769 | CD | GLU | A | 137 | 25.599 | 20.661 | 11.618 | 1.00 | 74.63 | C |
| ATOM | 770 | OE1 | GLU | A | 137 | 25.635 | 19.957 | 10.583 | 1.00 | 74.63 | O |
| ATOM | 771 | OE2 | GLU | A | 137 | 25.509 | 21.905 | 11.590 | 1.00 | 74.63 | O |
| ATOM | 772 | C | GLU | A | 137 | 24.666 | 20.375 | 16.358 | 1.00 | 23.09 | C |
| ATOM | 773 | O | GLU | A | 137 | 24.410 | 20.952 | 17.417 | 1.00 | 23.09 | O |
| ATOM | 774 | N | THR | A | 138 | 25.108 | 19.117 | 16.300 | 1.00 | 18.36 | N |
| ATOM | 775 | CA | THR | A | 138 | 25.413 | 18.336 | 17.501 | 1.00 | 18.36 | C |
| ATOM | 776 | CB | THR | A | 138 | 24.401 | 17.229 | 17.793 | 1.00 | 23.68 | C |
| ATOM | 777 | OG1 | THR | A | 138 | 24.423 | 16.285 | 16.719 | 1.00 | 23.68 | O |
| ATOM | 778 | CG2 | THR | A | 138 | 23.020 | 17.794 | 17.981 | 1.00 | 23.68 | C |
| ATOM | 779 | C | THR | A | 138 | 26.744 | 17.617 | 17.326 | 1.00 | 18.36 | C |
| ATOM | 780 | O | THR | A | 138 | 27.265 | 17.492 | 16.215 | 1.00 | 18.36 | O |
| ATOM | 781 | N | VAL | A | 139 | 27.276 | 17.113 | 18.429 | 1.00 | 37.43 | N |
| ATOM | 782 | CA | VAL | A | 139 | 28.541 | 16.415 | 18.374 | 1.00 | 37.43 | C |
| ATOM | 783 | CB | VAL | A | 139 | 29.048 | 16.070 | 19.782 | 1.00 | 27.08 | C |
| ATOM | 784 | CG1 | VAL | A | 139 | 30.329 | 15.262 | 19.689 | 1.00 | 27.08 | C |
| ATOM | 785 | CG2 | VAL | A | 139 | 29.308 | 17.345 | 20.557 | 1.00 | 27.08 | C |
| ATOM | 786 | C | VAL | A | 139 | 28.426 | 15.143 | 17.545 | 1.00 | 37.43 | C |
| ATOM | 787 | O | VAL | A | 139 | 29.390 | 14.734 | 16.902 | 1.00 | 37.43 | O |
| ATOM | 788 | N | TYR | A | 140 | 27.247 | 14.528 | 17.542 | 1.00 | 37.38 | N |
| ATOM | 789 | CA | TYR | A | 140 | 27.036 | 13.303 | 16.777 | 1.00 | 37.38 | C |
| ATOM | 790 | CB | TYR | A | 140 | 25.664 | 12.700 | 17.082 | 1.00 | 39.64 | C |
| ATOM | 791 | CG | TYR | A | 140 | 25.457 | 11.349 | 16.446 | 1.00 | 39.64 | C |
| ATOM | 792 | CD1 | TYR | A | 140 | 26.203 | 10.246 | 16.862 | 1.00 | 39.64 | C |
| ATOM | 793 | CE1 | TYR | A | 140 | 26.016 | 8.986 | 16.291 | 1.00 | 39.64 | C |
| ATOM | 794 | CD2 | TYR | A | 140 | 24.521 | 11.167 | 15.434 | 1.00 | 39.64 | C |
| ATOM | 795 | CE2 | TYR | A | 140 | 24.328 | 9.912 | 14.852 | 1.00 | 39.64 | C |
| ATOM | 796 | CZ | TYR | A | 140 | 25.079 | 8.823 | 15.292 | 1.00 | 39.64 | C |
| ATOM | 797 | OH | TYR | A | 140 | 24.880 | 7.566 | 14.767 | 1.00 | 39.64 | O |
| ATOM | 798 | C | TYR | A | 140 | 27.106 | 13.596 | 15.290 | 1.00 | 37.38 | C |
| ATOM | 799 | O | TYR | A | 140 | 27.935 | 13.031 | 14.567 | 1.00 | 37.38 | O |
| ATOM | 800 | N | ARG | A | 141 | 26.212 | 14.479 | 14.848 | 1.00 | 30.09 | N |
| ATOM | 801 | CA | ARG | A | 141 | 26.130 | 14.871 | 13.453 | 1.00 | 30.09 | C |
| ATOM | 802 | CB | ARG | A | 141 | 25.273 | 16.134 | 13.321 | 1.00 | 74.54 | C |
| ATOM | 803 | CG | ARG | A | 141 | 24.297 | 16.078 | 12.168 | 1.00 | 74.54 | C |
| ATOM | 804 | CD | ARG | A | 141 | 24.343 | 17.335 | 11.309 | 1.00 | 74.54 | C |
| ATOM | 805 | NE | ARG | A | 141 | 23.206 | 18.226 | 11.535 | 1.00 | 74.54 | N |
| ATOM | 806 | CZ | ARG | A | 141 | 22.573 | 18.869 | 10.556 | 1.00 | 74.54 | C |
| ATOM | 807 | NH1 | ARG | A | 141 | 22.962 | 18.713 | 9.292 | 1.00 | 74.54 | N |
| ATOM | 808 | NH2 | ARG | A | 141 | 21.561 | 19.678 | 10.834 | 1.00 | 74.54 | N |
| ATOM | 809 | C | ARG | A | 141 | 27.540 | 15.117 | 12.898 | 1.00 | 30.09 | C |
| ATOM | 810 | O | ARG | A | 141 | 27.893 | 14.641 | 11.822 | 1.00 | 30.09 | O |
| ATOM | 811 | N | VAL | A | 142 | 28.348 | 15.850 | 13.654 | 1.00 | 34.17 | N |
| ATOM | 812 | CA | VAL | A | 142 | 29.712 | 16.153 | 13.245 | 1.00 | 34.17 | C |
| ATOM | 813 | CB | VAL | A | 142 | 30.348 | 17.200 | 14.198 | 1.00 | 20.19 | C |
| ATOM | 814 | CG1 | VAL | A | 142 | 31.843 | 17.351 | 13.917 | 1.00 | 20.19 | C |

| | | | | | | | | | | | |
|---|---|---|---|---|---|---|---|---|---|---|---|
| ATOM | 815 | CG2 | VAL | A | 142 | 29.641 | 18.542 | 14.019 | 1.00 | 20.19 | C |
| ATOM | 816 | C | VAL | A | 142 | 30.570 | 14.891 | 13.222 | 1.00 | 34.17 | C |
| ATOM | 817 | O | VAL | A | 142 | 31.281 | 14.621 | 12.253 | 1.00 | 34.17 | O |
| ATOM | 818 | N | ALA | A | 143 | 30.513 | 14.121 | 14.301 | 1.00 | 35.19 | N |
| ATOM | 819 | CA | ALA | A | 143 | 31.287 | 12.882 | 14.393 | 1.00 | 35.19 | C |
| ATOM | 820 | CB | ALA | A | 143 | 30.922 | 12.117 | 15.650 | 1.00 | 7.68 | C |
| ATOM | 821 | C | ALA | A | 143 | 30.965 | 12.039 | 13.193 | 1.00 | 35.19 | C |
| ATOM | 822 | O | ALA | A | 143 | 31.858 | 11.574 | 12.494 | 1.00 | 35.19 | O |
| ATOM | 823 | N | ARG | A | 144 | 29.666 | 11.845 | 12.991 | 1.00 | 36.20 | N |
| ATOM | 824 | CA | ARG | A | 144 | 29.115 | 11.074 | 11.887 | 1.00 | 36.20 | C |
| ATOM | 825 | CB | ARG | A | 144 | 27.586 | 11.279 | 11.872 | 1.00 | 51.75 | C |
| ATOM | 826 | CG | ARG | A | 144 | 26.744 | 10.180 | 11.209 | 1.00 | 51.75 | C |
| ATOM | 827 | CD | ARG | A | 144 | 26.563 | 8.919 | 12.072 | 1.00 | 51.75 | C |
| ATOM | 828 | NE | ARG | A | 144 | 27.708 | 8.006 | 12.016 | 1.00 | 51.75 | N |
| ATOM | 829 | CZ | ARG | A | 144 | 28.066 | 7.301 | 10.942 | 1.00 | 51.75 | C |
| ATOM | 830 | NH1 | ARG | A | 144 | 27.373 | 7.389 | 9.814 | 1.00 | 51.75 | N |
| ATOM | 831 | NH2 | ARG | A | 144 | 29.131 | 6.510 | 10.987 | 1.00 | 51.75 | N |
| ATOM | 832 | C | ARG | A | 144 | 29.767 | 11.551 | 10.562 | 1.00 | 36.20 | C |
| ATOM | 833 | O | ARG | A | 144 | 30.278 | 10.746 | 9.776 | 1.00 | 36.20 | O |
| ATOM | 834 | N | HIS | A | 145 | 29.783 | 12.866 | 10.348 | 1.00 | 41.63 | N |
| ATOM | 835 | CA | HIS | A | 145 | 30.353 | 13.482 | 9.142 | 1.00 | 41.63 | C |
| ATOM | 836 | CB | HIS | A | 145 | 30.287 | 15.006 | 9.275 | 1.00 | 48.91 | C |
| ATOM | 837 | CG | HIS | A | 145 | 30.570 | 15.749 | 8.004 | 1.00 | 48.91 | C |
| ATOM | 838 | CD2 | HIS | A | 145 | 29.735 | 16.334 | 7.112 | 1.00 | 48.91 | C |
| ATOM | 839 | ND1 | HIS | A | 145 | 31.848 | 15.985 | 7.545 | 1.00 | 48.91 | N |
| ATOM | 840 | CE1 | HIS | A | 145 | 31.788 | 16.687 | 6.428 | 1.00 | 48.91 | C |
| ATOM | 841 | NE2 | HIS | A | 145 | 30.519 | 16.912 | 6.143 | 1.00 | 48.91 | N |
| ATOM | 842 | C | HIS | A | 145 | 31.791 | 13.059 | 8.837 | 1.00 | 41.63 | C |
| ATOM | 843 | O | HIS | A | 145 | 32.171 | 12.898 | 7.675 | 1.00 | 41.63 | O |
| ATOM | 844 | N | TYR | A | 146 | 32.594 | 12.899 | 9.879 | 1.00 | 37.91 | N |
| ATOM | 845 | CA | TYR | A | 146 | 33.969 | 12.493 | 9.683 | 1.00 | 37.91 | C |
| ATOM | 846 | CB | TYR | A | 146 | 34.813 | 12.774 | 10.922 | 1.00 | 36.82 | C |
| ATOM | 847 | CG | TYR | A | 146 | 35.365 | 14.169 | 10.913 | 1.00 | 36.82 | C |
| ATOM | 848 | CD1 | TYR | A | 146 | 34.541 | 15.278 | 11.156 | 1.00 | 36.82 | C |
| ATOM | 849 | CE1 | TYR | A | 146 | 35.028 | 16.578 | 11.017 | 1.00 | 36.82 | C |
| ATOM | 850 | CD2 | TYR | A | 146 | 36.681 | 14.398 | 10.543 | 1.00 | 36.82 | C |
| ATOM | 851 | CE2 | TYR | A | 146 | 37.166 | 15.676 | 10.404 | 1.00 | 36.82 | C |
| ATOM | 852 | CZ | TYR | A | 146 | 36.342 | 16.759 | 10.637 | 1.00 | 36.82 | C |
| ATOM | 853 | OH | TYR | A | 146 | 36.842 | 18.022 | 10.466 | 1.00 | 36.82 | O |
| ATOM | 854 | C | TYR | A | 146 | 33.989 | 11.031 | 9.375 | 1.00 | 37.91 | C |
| ATOM | 855 | O | TYR | A | 146 | 34.442 | 10.640 | 8.317 | 1.00 | 37.91 | O |
| ATOM | 856 | N | SER | A | 147 | 33.511 | 10.223 | 10.312 | 1.00 | 36.88 | N |
| ATOM | 857 | CA | SER | A | 147 | 33.439 | 8.785 | 10.125 | 1.00 | 36.88 | C |
| ATOM | 858 | CB | SER | A | 147 | 32.520 | 8.192 | 11.199 | 1.00 | 63.45 | C |
| ATOM | 859 | OG | SER | A | 147 | 31.923 | 6.983 | 10.765 | 1.00 | 63.45 | O |
| ATOM | 860 | C | SER | A | 147 | 32.900 | 8.465 | 8.718 | 1.00 | 36.88 | C |
| ATOM | 861 | O | SER | A | 147 | 33.445 | 7.625 | 8.003 | 1.00 | 36.88 | O |
| ATOM | 862 | N | ARG | A | 148 | 31.839 | 9.157 | 8.317 | 1.00 | 47.80 | N |
| ATOM | 863 | CA | ARG | A | 148 | 31.235 | 8.929 | 7.012 | 1.00 | 47.80 | C |
| ATOM | 864 | CB | ARG | A | 148 | 29.949 | 9.740 | 6.890 | 1.00 | 48.33 | C |
| ATOM | 865 | CG | ARG | A | 148 | 28.990 | 9.196 | 5.867 | 1.00 | 48.33 | C |
| ATOM | 866 | CD | ARG | A | 148 | 27.853 | 10.153 | 5.561 | 1.00 | 48.33 | C |
| ATOM | 867 | NE | ARG | A | 148 | 26.884 | 10.280 | 6.646 | 1.00 | 48.33 | N |
| ATOM | 868 | CZ | ARG | A | 148 | 26.960 | 11.177 | 7.624 | 1.00 | 48.33 | C |
| ATOM | 869 | NH1 | ARG | A | 148 | 27.970 | 12.036 | 7.661 | 1.00 | 48.33 | N |
| ATOM | 870 | NH2 | ARG | A | 148 | 26.011 | 11.232 | 8.552 | 1.00 | 48.33 | N |
| ATOM | 871 | C | ARG | A | 148 | 32.192 | 9.290 | 5.874 | 1.00 | 47.80 | C |
| ATOM | 872 | O | ARG | A | 148 | 32.072 | 8.776 | 4.761 | 1.00 | 47.80 | O |
| ATOM | 873 | N | ALA | A | 149 | 33.137 | 10.181 | 6.149 | 1.00 | 37.14 | N |
| ATOM | 874 | CA | ALA | A | 149 | 34.118 | 10.595 | 5.150 | 1.00 | 37.14 | C |
| ATOM | 875 | CB | ALA | A | 149 | 34.372 | 12.098 | 5.228 | 1.00 | 33.18 | C |
| ATOM | 876 | C | ALA | A | 149 | 35.405 | 9.846 | 5.426 | 1.00 | 37.14 | C |
| ATOM | 877 | O | ALA | A | 149 | 36.483 | 10.248 | 4.984 | 1.00 | 37.14 | O |

| | | | | | | | | | | | |
|---|---|---|---|---|---|---|---|---|---|---|---|
| ATOM | 878 | N | LYS | A | 150 | 35.288 | 8.762 | 6.182 | 1.00 | 46.06 | N |
| ATOM | 879 | CA | LYS | A | 150 | 36.440 | 7.936 | 6.528 | 1.00 | 46.06 | C |
| ATOM | 880 | CB | LYS | A | 150 | 36.909 | 7.136 | 5.303 | 1.00 | 73.01 | C |
| ATOM | 881 | CG | LYS | A | 150 | 35.907 | 6.101 | 4.739 | 1.00 | 73.01 | C |
| ATOM | 882 | CD | LYS | A | 150 | 34.832 | 6.715 | 3.831 | 1.00 | 73.01 | C |
| ATOM | 883 | CE | LYS | A | 150 | 34.239 | 5.680 | 2.865 | 1.00 | 73.01 | C |
| ATOM | 884 | NZ | LYS | A | 150 | 33.843 | 4.407 | 3.525 | 1.00 | 73.01 | N |
| ATOM | 885 | C | LYS | A | 150 | 37.618 | 8.728 | 7.113 | 1.00 | 46.06 | C |
| ATOM | 886 | O | LYS | A | 150 | 38.755 | 8.270 | 7.077 | 1.00 | 46.06 | O |
| ATOM | 887 | N | GLN | A | 151 | 37.349 | 9.916 | 7.646 | 1.00 | 62.86 | N |
| ATOM | 888 | CA | GLN | A | 151 | 38.397 | 10.728 | 8.262 | 1.00 | 62.86 | C |
| ATOM | 889 | CB | GLN | A | 151 | 38.332 | 12.184 | 7.812 | 1.00 | 54.44 | C |
| ATOM | 890 | CG | GLN | A | 151 | 38.408 | 12.416 | 6.336 | 1.00 | 54.44 | C |
| ATOM | 891 | CD | GLN | A | 151 | 38.744 | 13.859 | 6.012 | 1.00 | 54.44 | C |
| ATOM | 892 | OE1 | GLN | A | 151 | 38.200 | 14.794 | 6.611 | 1.00 | 54.44 | O |
| ATOM | 893 | NE2 | GLN | A | 151 | 39.647 | 14.049 | 5.056 | 1.00 | 54.44 | N |
| ATOM | 894 | C | GLN | A | 151 | 38.187 | 10.700 | 9.766 | 1.00 | 62.86 | C |
| ATOM | 895 | O | GLN | A | 151 | 37.162 | 10.214 | 10.254 | 1.00 | 62.86 | O |
| ATOM | 896 | N | THR | A | 152 | 39.149 | 11.246 | 10.499 | 1.00 | 62.58 | N |
| ATOM | 897 | CA | THR | A | 152 | 39.061 | 11.286 | 11.952 | 1.00 | 62.58 | C |
| ATOM | 898 | CB | THR | A | 152 | 40.220 | 10.477 | 12.590 | 1.00 | 43.99 | C |
| ATOM | 899 | OG1 | THR | A | 152 | 40.086 | 10.480 | 14.018 | 1.00 | 43.99 | O |
| ATOM | 900 | CG2 | THR | A | 152 | 41.567 | 11.064 | 12.181 | 1.00 | 43.99 | C |
| ATOM | 901 | C | THR | A | 152 | 39.088 | 12.731 | 12.454 | 1.00 | 62.58 | C |
| ATOM | 902 | O | THR | A | 152 | 39.941 | 13.525 | 12.051 | 1.00 | 62.58 | O |
| ATOM | 903 | N | LEU | A | 153 | 38.137 | 13.054 | 13.328 | 1.00 | 31.93 | N |
| ATOM | 904 | CA | LEU | A | 153 | 37.990 | 14.389 | 13.917 | 1.00 | 31.93 | C |
| ATOM | 905 | CB | LEU | A | 153 | 36.906 | 14.367 | 14.995 | 1.00 | 42.36 | C |
| ATOM | 906 | CG | LEU | A | 153 | 36.645 | 15.697 | 15.695 | 1.00 | 42.36 | C |
| ATOM | 907 | CD1 | LEU | A | 153 | 35.838 | 16.584 | 14.774 | 1.00 | 42.36 | C |
| ATOM | 908 | CD2 | LEU | A | 153 | 35.908 | 15.463 | 17.000 | 1.00 | 42.36 | C |
| ATOM | 909 | C | LEU | A | 153 | 39.282 | 14.917 | 14.530 | 1.00 | 31.93 | C |
| ATOM | 910 | O | LEU | A | 153 | 39.908 | 14.254 | 15.360 | 1.00 | 31.93 | O |
| ATOM | 911 | N | PRO | A | 154 | 39.689 | 16.131 | 14.132 | 1.00 | 56.82 | N |
| ATOM | 912 | CD | PRO | A | 154 | 38.986 | 16.929 | 13.112 | 1.00 | 35.32 | C |
| ATOM | 913 | CA | PRO | A | 154 | 40.901 | 16.825 | 14.593 | 1.00 | 56.82 | C |
| ATOM | 914 | CB | PRO | A | 154 | 40.836 | 18.155 | 13.847 | 1.00 | 35.32 | C |
| ATOM | 915 | CG | PRO | A | 154 | 40.077 | 17.815 | 12.596 | 1.00 | 35.32 | C |
| ATOM | 916 | C | PRO | A | 154 | 40.957 | 17.033 | 16.111 | 1.00 | 56.82 | C |
| ATOM | 917 | O | PRO | A | 154 | 40.098 | 17.711 | 16.671 | 1.00 | 56.82 | O |
| ATOM | 918 | N | VAL | A | 155 | 41.977 | 16.469 | 16.760 | 1.00 | 40.65 | N |
| ATOM | 919 | CA | VAL | A | 155 | 42.149 | 16.575 | 18.212 | 1.00 | 40.65 | C |
| ATOM | 920 | CB | VAL | A | 155 | 43.603 | 16.268 | 18.638 | 1.00 | 36.21 | C |
| ATOM | 921 | CG1 | VAL | A | 155 | 43.912 | 14.806 | 18.394 | 1.00 | 36.21 | C |
| ATOM | 922 | CG2 | VAL | A | 155 | 44.575 | 17.160 | 17.868 | 1.00 | 36.21 | C |
| ATOM | 923 | C | VAL | A | 155 | 41.765 | 17.928 | 18.803 | 1.00 | 40.65 | C |
| ATOM | 924 | O | VAL | A | 155 | 41.214 | 17.996 | 19.899 | 1.00 | 40.65 | O |
| ATOM | 925 | N | ILE | A | 156 | 42.069 | 19.006 | 18.092 | 1.00 | 36.26 | N |
| ATOM | 926 | CA | ILE | A | 156 | 41.701 | 20.322 | 18.582 | 1.00 | 36.26 | C |
| ATOM | 927 | CB | ILE | A | 156 | 41.969 | 21.406 | 17.522 | 1.00 | 41.68 | C |
| ATOM | 928 | CG2 | ILE | A | 156 | 41.117 | 21.159 | 16.276 | 1.00 | 41.68 | C |
| ATOM | 929 | CG1 | ILE | A | 156 | 41.633 | 22.781 | 18.099 | 1.00 | 41.68 | C |
| ATOM | 930 | CD1 | ILE | A | 156 | 42.420 | 23.146 | 19.344 | 1.00 | 41.68 | C |
| ATOM | 931 | C | ILE | A | 156 | 40.209 | 20.336 | 18.958 | 1.00 | 36.26 | C |
| ATOM | 932 | O | ILE | A | 156 | 39.828 | 20.864 | 19.997 | 1.00 | 36.26 | O |
| ATOM | 933 | N | TYR | A | 157 | 39.379 | 19.739 | 18.111 | 1.00 | 21.98 | N |
| ATOM | 934 | CA | TYR | A | 157 | 37.944 | 19.666 | 18.341 | 1.00 | 21.98 | C |
| ATOM | 935 | CB | TYR | A | 157 | 37.236 | 19.249 | 17.053 | 1.00 | 31.03 | C |
| ATOM | 936 | CG | TYR | A | 157 | 37.359 | 20.300 | 15.979 | 1.00 | 31.03 | C |
| ATOM | 937 | CD1 | TYR | A | 157 | 37.931 | 20.003 | 14.740 | 1.00 | 31.03 | C |
| ATOM | 938 | CE1 | TYR | A | 157 | 38.108 | 20.997 | 13.773 | 1.00 | 31.03 | C |
| ATOM | 939 | CD2 | TYR | A | 157 | 36.957 | 21.618 | 16.226 | 1.00 | 31.03 | C |
| ATOM | 940 | CE2 | TYR | A | 157 | 37.125 | 22.615 | 15.274 | 1.00 | 31.03 | C |

| | | | | | | | | | | | |
|---|---|---|---|---|---|---|---|---|---|---|---|
| ATOM | 941 | CZ | TYR | A | 157 | 37.701 | 22.307 | 14.048 | 1.00 | 31.03 | C |
| ATOM | 942 | OH | TYR | A | 157 | 37.863 | 23.315 | 13.106 | 1.00 | 31.03 | O |
| ATOM | 943 | C | TYR | A | 157 | 37.610 | 18.695 | 19.459 | 1.00 | 21.98 | C |
| ATOM | 944 | O | TYR | A | 157 | 36.703 | 18.943 | 20.259 | 1.00 | 21.98 | O |
| ATOM | 945 | N | VAL | A | 158 | 38.329 | 17.580 | 19.512 | 1.00 | 23.88 | N |
| ATOM | 946 | CA | VAL | A | 158 | 38.103 | 16.611 | 20.568 | 1.00 | 23.88 | C |
| ATOM | 947 | CB | VAL | A | 158 | 39.054 | 15.417 | 20.431 | 1.00 | 29.93 | C |
| ATOM | 948 | CG1 | VAL | A | 158 | 38.782 | 14.392 | 21.527 | 1.00 | 29.93 | C |
| ATOM | 949 | CG2 | VAL | A | 158 | 38.871 | 14.791 | 19.053 | 1.00 | 29.93 | C |
| ATOM | 950 | C | VAL | A | 158 | 38.362 | 17.347 | 21.876 | 1.00 | 23.88 | C |
| ATOM | 951 | O | VAL | A | 158 | 37.589 | 17.229 | 22.832 | 1.00 | 23.88 | O |
| ATOM | 952 | N | LYS | A | 159 | 39.435 | 18.137 | 21.900 | 1.00 | 43.02 | N |
| ATOM | 953 | CA | LYS | A | 159 | 39.795 | 18.912 | 23.088 | 1.00 | 43.02 | C |
| ATOM | 954 | CB | LYS | A | 159 | 41.134 | 19.633 | 22.889 | 1.00 | 28.29 | C |
| ATOM | 955 | CG | LYS | A | 159 | 42.346 | 18.705 | 22.801 | 1.00 | 28.29 | C |
| ATOM | 956 | CD | LYS | A | 159 | 43.646 | 19.465 | 22.457 | 1.00 | 28.29 | C |
| ATOM | 957 | CE | LYS | A | 159 | 44.846 | 18.519 | 22.292 | 1.00 | 28.29 | C |
| ATOM | 958 | NZ | LYS | A | 159 | 46.098 | 19.248 | 21.968 | 1.00 | 28.29 | N |
| ATOM | 959 | C | LYS | A | 159 | 38.715 | 19.939 | 23.388 | 1.00 | 43.02 | C |
| ATOM | 960 | O | LYS | A | 159 | 38.151 | 19.954 | 24.485 | 1.00 | 43.02 | O |
| ATOM | 961 | N | LEU | A | 160 | 38.429 | 20.787 | 22.402 | 1.00 | 31.72 | N |
| ATOM | 962 | CA | LEU | A | 160 | 37.419 | 21.831 | 22.549 | 1.00 | 31.72 | C |
| ATOM | 963 | CB | LEU | A | 160 | 37.255 | 22.616 | 21.252 | 1.00 | 16.45 | C |
| ATOM | 964 | CG | LEU | A | 160 | 38.320 | 23.672 | 20.934 | 1.00 | 16.45 | C |
| ATOM | 965 | CD1 | LEU | A | 160 | 38.127 | 24.214 | 19.522 | 1.00 | 16.45 | C |
| ATOM | 966 | CD2 | LEU | A | 160 | 38.212 | 24.807 | 21.947 | 1.00 | 16.45 | C |
| ATOM | 967 | C | LEU | A | 160 | 36.059 | 21.313 | 22.984 | 1.00 | 31.72 | C |
| ATOM | 968 | O | LEU | A | 160 | 35.418 | 21.921 | 23.840 | 1.00 | 31.72 | O |
| ATOM | 969 | N | TYR | A | 161 | 35.595 | 20.206 | 22.410 | 1.00 | 35.85 | N |
| ATOM | 970 | CA | TYR | A | 161 | 34.293 | 19.689 | 22.814 | 1.00 | 35.85 | C |
| ATOM | 971 | CB | TYR | A | 161 | 33.749 | 18.706 | 21.775 | 1.00 | 32.78 | C |
| ATOM | 972 | CG | TYR | A | 161 | 33.616 | 19.298 | 20.388 | 1.00 | 32.78 | C |
| ATOM | 973 | CD1 | TYR | A | 161 | 33.349 | 20.659 | 20.218 | 1.00 | 32.78 | C |
| ATOM | 974 | CE1 | TYR | A | 161 | 33.275 | 21.227 | 18.942 | 1.00 | 32.78 | C |
| ATOM | 975 | CD2 | TYR | A | 161 | 33.798 | 18.505 | 19.240 | 1.00 | 32.78 | C |
| ATOM | 976 | CE2 | TYR | A | 161 | 33.724 | 19.059 | 17.955 | 1.00 | 32.78 | C |
| ATOM | 977 | CZ | TYR | A | 161 | 33.470 | 20.418 | 17.811 | 1.00 | 32.78 | C |
| ATOM | 978 | OH | TYR | A | 161 | 33.455 | 20.960 | 16.540 | 1.00 | 32.78 | O |
| ATOM | 979 | C | TYR | A | 161 | 34.328 | 19.026 | 24.189 | 1.00 | 35.85 | C |
| ATOM | 980 | O | TYR | A | 161 | 33.496 | 19.316 | 25.045 | 1.00 | 35.85 | O |
| ATOM | 981 | N | MET | A | 162 | 35.299 | 18.153 | 24.418 | 1.00 | 38.07 | N |
| ATOM | 982 | CA | MET | A | 162 | 35.371 | 17.464 | 25.696 | 1.00 | 38.07 | C |
| ATOM | 983 | CB | MET | A | 162 | 36.516 | 16.458 | 25.676 | 1.00 | 28.87 | C |
| ATOM | 984 | CG | MET | A | 162 | 36.236 | 15.309 | 24.736 | 1.00 | 28.87 | C |
| ATOM | 985 | SD | MET | A | 162 | 34.586 | 14.591 | 25.047 | 1.00 | 28.87 | S |
| ATOM | 986 | CE | MET | A | 162 | 34.879 | 13.932 | 26.695 | 1.00 | 28.87 | C |
| ATOM | 987 | C | MET | A | 162 | 35.521 | 18.438 | 26.848 | 1.00 | 38.07 | C |
| ATOM | 988 | O | MET | A | 162 | 34.883 | 18.307 | 27.901 | 1.00 | 38.07 | O |
| ATOM | 989 | N | TYR | A | 163 | 36.355 | 19.441 | 26.638 | 1.00 | 37.79 | N |
| ATOM | 990 | CA | TYR | A | 163 | 36.569 | 20.425 | 27.671 | 1.00 | 37.79 | C |
| ATOM | 991 | CB | TYR | A | 163 | 37.580 | 21.458 | 27.203 | 1.00 | 36.19 | C |
| ATOM | 992 | CG | TYR | A | 163 | 37.820 | 22.549 | 28.211 | 1.00 | 36.19 | C |
| ATOM | 993 | CD1 | TYR | A | 163 | 37.055 | 23.729 | 28.206 | 1.00 | 36.19 | C |
| ATOM | 994 | CE1 | TYR | A | 163 | 37.266 | 24.718 | 29.155 | 1.00 | 36.19 | C |
| ATOM | 995 | CD2 | TYR | A | 163 | 38.792 | 22.393 | 29.191 | 1.00 | 36.19 | C |
| ATOM | 996 | CE2 | TYR | A | 163 | 39.005 | 23.364 | 30.131 | 1.00 | 36.19 | C |
| ATOM | 997 | CZ | TYR | A | 163 | 38.244 | 24.516 | 30.108 | 1.00 | 36.19 | C |
| ATOM | 998 | OH | TYR | A | 163 | 38.484 | 25.477 | 31.038 | 1.00 | 36.19 | O |
| ATOM | 999 | C | TYR | A | 163 | 35.275 | 21.120 | 28.084 | 1.00 | 37.79 | C |
| ATOM | 1000 | O | TYR | A | 163 | 35.008 | 21.266 | 29.274 | 1.00 | 37.79 | O |
| ATOM | 1001 | N | GLN | A | 164 | 34.485 | 21.558 | 27.104 | 1.00 | 26.79 | N |
| ATOM | 1002 | CA | GLN | A | 164 | 33.237 | 22.256 | 27.397 | 1.00 | 26.79 | C |
| ATOM | 1003 | CB | GLN | A | 164 | 32.677 | 22.919 | 26.130 | 1.00 | 29.86 | C |

| | | | | | | | | | | | |
|---|---|---|---|---|---|---|---|---|---|---|---|
| ATOM | 1004 | CG | GLN | A | 164 | 33.611 | 23.990 | 25.561 | 1.00 | 29.86 | C |
| ATOM | 1005 | CD | GLN | A | 164 | 33.099 | 24.601 | 24.270 | 1.00 | 29.86 | C |
| ATOM | 1006 | OE1 | GLN | A | 164 | 32.298 | 25.529 | 24.285 | 1.00 | 29.86 | O |
| ATOM | 1007 | NE2 | GLN | A | 164 | 33.556 | 24.072 | 23.142 | 1.00 | 29.86 | N |
| ATOM | 1008 | C | GLN | A | 164 | 32.239 | 21.287 | 28.015 | 1.00 | 26.79 | C |
| ATOM | 1009 | O | GLN | A | 164 | 31.435 | 21.675 | 28.867 | 1.00 | 26.79 | O |
| ATOM | 1010 | N | LEU | A | 165 | 32.320 | 20.020 | 27.607 | 1.00 | 24.79 | N |
| ATOM | 1011 | CA | LEU | A | 165 | 31.446 | 18.982 | 28.147 | 1.00 | 24.79 | C |
| ATOM | 1012 | CB | LEU | A | 165 | 31.806 | 17.617 | 27.563 | 1.00 | 34.42 | C |
| ATOM | 1013 | CG | LEU | A | 165 | 30.708 | 16.548 | 27.528 | 1.00 | 34.42 | C |
| ATOM | 1014 | CD1 | LEU | A | 165 | 31.378 | 15.182 | 27.372 | 1.00 | 34.42 | C |
| ATOM | 1015 | CD2 | LEU | A | 165 | 29.836 | 16.604 | 28.783 | 1.00 | 34.42 | C |
| ATOM | 1016 | C | LEU | A | 165 | 31.676 | 18.932 | 29.654 | 1.00 | 24.79 | C |
| ATOM | 1017 | O | LEU | A | 165 | 30.745 | 19.072 | 30.450 | 1.00 | 24.79 | O |
| ATOM | 1018 | N | PHE | A | 166 | 32.935 | 18.733 | 30.033 | 1.00 | 24.41 | N |
| ATOM | 1019 | CA | PHE | A | 166 | 33.312 | 18.653 | 31.436 | 1.00 | 24.41 | C |
| ATOM | 1020 | CB | PHE | A | 166 | 34.806 | 18.358 | 31.563 | 1.00 | 31.21 | C |
| ATOM | 1021 | CG | PHE | A | 166 | 35.184 | 16.971 | 31.131 | 1.00 | 31.21 | C |
| ATOM | 1022 | CD1 | PHE | A | 166 | 34.536 | 15.853 | 31.678 | 1.00 | 31.21 | C |
| ATOM | 1023 | CD2 | PHE | A | 166 | 36.163 | 16.773 | 30.156 | 1.00 | 31.21 | C |
| ATOM | 1024 | CE1 | PHE | A | 166 | 34.852 | 14.559 | 31.260 | 1.00 | 31.21 | C |
| ATOM | 1025 | CE2 | PHE | A | 166 | 36.487 | 15.482 | 29.731 | 1.00 | 31.21 | C |
| ATOM | 1026 | CZ | PHE | A | 166 | 35.824 | 14.375 | 30.289 | 1.00 | 31.21 | C |
| ATOM | 1027 | C | PHE | A | 166 | 32.963 | 19.879 | 32.264 | 1.00 | 24.41 | C |
| ATOM | 1028 | O | PHE | A | 166 | 32.680 | 19.761 | 33.458 | 1.00 | 24.41 | O |
| ATOM | 1029 | N | ARG | A | 167 | 32.982 | 21.056 | 31.649 | 1.00 | 33.03 | N |
| ATOM | 1030 | CA | ARG | A | 167 | 32.649 | 22.279 | 32.382 | 1.00 | 33.03 | C |
| ATOM | 1031 | CB | ARG | A | 167 | 32.938 | 23.531 | 31.558 | 1.00 | 30.50 | C |
| ATOM | 1032 | CG | ARG | A | 167 | 34.335 | 24.072 | 31.720 | 1.00 | 30.50 | C |
| ATOM | 1033 | CD | ARG | A | 167 | 34.417 | 25.477 | 31.183 | 1.00 | 30.50 | C |
| ATOM | 1034 | NE | ARG | A | 167 | 33.792 | 26.463 | 32.066 | 1.00 | 30.50 | N |
| ATOM | 1035 | CZ | ARG | A | 167 | 33.663 | 27.754 | 31.759 | 1.00 | 30.50 | C |
| ATOM | 1036 | NH1 | ARG | A | 167 | 34.108 | 28.214 | 30.596 | 1.00 | 30.50 | N |
| ATOM | 1037 | NH2 | ARG | A | 167 | 33.098 | 28.591 | 32.616 | 1.00 | 30.50 | N |
| ATOM | 1038 | C | ARG | A | 167 | 31.189 | 22.287 | 32.766 | 1.00 | 33.03 | C |
| ATOM | 1039 | O | ARG | A | 167 | 30.841 | 22.600 | 33.902 | 1.00 | 33.03 | O |
| ATOM | 1040 | N | SER | A | 168 | 30.337 | 21.959 | 31.804 | 1.00 | 25.48 | N |
| ATOM | 1041 | CA | SER | A | 168 | 28.898 | 21.899 | 32.044 | 1.00 | 25.48 | C |
| ATOM | 1042 | CB | SER | A | 168 | 28.150 | 21.555 | 30.739 | 1.00 | 19.51 | C |
| ATOM | 1043 | OG | SER | A | 168 | 28.451 | 20.248 | 30.263 | 1.00 | 19.51 | O |
| ATOM | 1044 | C | SER | A | 168 | 28.612 | 20.829 | 33.110 | 1.00 | 25.48 | C |
| ATOM | 1045 | O | SER | A | 168 | 27.726 | 20.989 | 33.952 | 1.00 | 25.48 | O |
| ATOM | 1046 | N | LEU | A | 169 | 29.374 | 19.738 | 33.065 | 1.00 | 16.31 | N |
| ATOM | 1047 | CA | LEU | A | 169 | 29.195 | 18.651 | 34.021 | 1.00 | 16.31 | C |
| ATOM | 1048 | CB | LEU | A | 169 | 29.981 | 17.411 | 33.584 | 1.00 | 30.58 | C |
| ATOM | 1049 | CG | LEU | A | 169 | 29.442 | 16.753 | 32.309 | 1.00 | 30.58 | C |
| ATOM | 1050 | CD1 | LEU | A | 169 | 30.323 | 15.592 | 31.892 | 1.00 | 30.58 | C |
| ATOM | 1051 | CD2 | LEU | A | 169 | 28.013 | 16.277 | 32.559 | 1.00 | 30.58 | C |
| ATOM | 1052 | C | LEU | A | 169 | 29.629 | 19.090 | 35.402 | 1.00 | 16.31 | C |
| ATOM | 1053 | O | LEU | A | 169 | 29.017 | 18.721 | 36.392 | 1.00 | 16.31 | O |
| ATOM | 1054 | N | ALA | A | 170 | 30.682 | 19.899 | 35.473 | 1.00 | 22.58 | N |
| ATOM | 1055 | CA | ALA | A | 170 | 31.153 | 20.379 | 36.771 | 1.00 | 22.58 | C |
| ATOM | 1056 | CB | ALA | A | 170 | 32.494 | 21.095 | 36.625 | 1.00 | 27.67 | C |
| ATOM | 1057 | C | ALA | A | 170 | 30.126 | 21.315 | 37.378 | 1.00 | 22.58 | C |
| ATOM | 1058 | O | ALA | A | 170 | 29.887 | 21.292 | 38.581 | 1.00 | 22.58 | O |
| ATOM | 1059 | N | TYR | A | 171 | 29.520 | 22.131 | 36.525 | 1.00 | 22.18 | N |
| ATOM | 1060 | CA | TYR | A | 171 | 28.501 | 23.083 | 36.951 | 1.00 | 22.18 | C |
| ATOM | 1061 | CB | TYR | A | 171 | 27.981 | 23.894 | 35.759 | 1.00 | 28.29 | C |
| ATOM | 1062 | CG | TYR | A | 171 | 26.894 | 24.892 | 36.110 | 1.00 | 28.29 | C |
| ATOM | 1063 | CD1 | TYR | A | 171 | 27.203 | 26.112 | 36.711 | 1.00 | 28.29 | C |
| ATOM | 1064 | CE1 | TYR | A | 171 | 26.205 | 27.057 | 36.992 | 1.00 | 28.29 | C |
| ATOM | 1065 | CD2 | TYR | A | 171 | 25.557 | 24.630 | 35.807 | 1.00 | 28.29 | C |
| ATOM | 1066 | CE2 | TYR | A | 171 | 24.547 | 25.565 | 36.082 | 1.00 | 28.29 | C |

| | | | | | | | | | | | |
|---|---|---|---|---|---|---|---|---|---|---|---|
| ATOM | 1067 | CZ | TYR | A | 171 | 24.874 | 26.775 | 36.673 | 1.00 | 28.29 | C |
| ATOM | 1068 | OH | TYR | A | 171 | 23.863 | 27.682 | 36.935 | 1.00 | 28.29 | O |
| ATOM | 1069 | C | TYR | A | 171 | 27.329 | 22.363 | 37.603 | 1.00 | 22.18 | C |
| ATOM | 1070 | O | TYR | A | 171 | 27.127 | 22.441 | 38.813 | 1.00 | 22.18 | O |
| ATOM | 1071 | N | ILE | A | 172 | 26.563 | 21.646 | 36.796 | 1.00 | 24.16 | N |
| ATOM | 1072 | CA | ILE | A | 172 | 25.407 | 20.959 | 37.317 | 1.00 | 24.16 | C |
| ATOM | 1073 | CB | ILE | A | 172 | 24.723 | 20.108 | 36.241 | 1.00 | 20.28 | C |
| ATOM | 1074 | CG2 | ILE | A | 172 | 24.261 | 20.985 | 35.104 | 1.00 | 20.28 | C |
| ATOM | 1075 | CG1 | ILE | A | 172 | 25.689 | 19.035 | 35.741 | 1.00 | 20.28 | C |
| ATOM | 1076 | CD1 | ILE | A | 172 | 25.053 | 17.994 | 34.801 | 1.00 | 20.28 | C |
| ATOM | 1077 | C | ILE | A | 172 | 25.751 | 20.067 | 38.504 | 1.00 | 24.16 | C |
| ATOM | 1078 | O | ILE | A | 172 | 24.966 | 19.965 | 39.449 | 1.00 | 24.16 | O |
| ATOM | 1079 | N | HIS | A | 173 | 26.916 | 19.425 | 38.476 | 1.00 | 26.38 | N |
| ATOM | 1080 | CA | HIS | A | 173 | 27.291 | 18.536 | 39.576 | 1.00 | 26.38 | C |
| ATOM | 1081 | CB | HIS | A | 173 | 28.595 | 17.797 | 39.266 | 1.00 | 19.20 | C |
| ATOM | 1082 | CG | HIS | A | 173 | 28.428 | 16.625 | 38.342 | 1.00 | 19.20 | C |
| ATOM | 1083 | CD2 | HIS | A | 173 | 27.363 | 16.204 | 37.613 | 1.00 | 19.20 | C |
| ATOM | 1084 | ND1 | HIS | A | 173 | 29.462 | 15.761 | 38.040 | 1.00 | 19.20 | N |
| ATOM | 1085 | CE1 | HIS | A | 173 | 29.044 | 14.866 | 37.164 | 1.00 | 19.20 | C |
| ATOM | 1086 | NE2 | HIS | A | 173 | 27.776 | 15.113 | 36.887 | 1.00 | 19.20 | N |
| ATOM | 1087 | C | HIS | A | 173 | 27.428 | 19.267 | 40.904 | 1.00 | 26.38 | C |
| ATOM | 1088 | O | HIS | A | 173 | 27.203 | 18.691 | 41.975 | 1.00 | 26.38 | O |
| ATOM | 1089 | N | SER | A | 174 | 27.787 | 20.541 | 40.833 | 1.00 | 24.68 | N |
| ATOM | 1090 | CA | SER | A | 174 | 27.962 | 21.345 | 42.033 | 1.00 | 24.68 | C |
| ATOM | 1091 | CB | SER | A | 174 | 28.691 | 22.650 | 41.700 | 1.00 | 23.96 | C |
| ATOM | 1092 | OG | SER | A | 174 | 27.892 | 23.471 | 40.878 | 1.00 | 23.96 | O |
| ATOM | 1093 | C | SER | A | 174 | 26.616 | 21.655 | 42.663 | 1.00 | 24.68 | C |
| ATOM | 1094 | O | SER | A | 174 | 26.546 | 22.216 | 43.755 | 1.00 | 24.68 | O |
| ATOM | 1095 | N | PHE | A | 175 | 25.552 | 21.302 | 41.951 | 1.00 | 20.70 | N |
| ATOM | 1096 | CA | PHE | A | 175 | 24.189 | 21.499 | 42.425 | 1.00 | 20.70 | C |
| ATOM | 1097 | CB | PHE | A | 175 | 23.342 | 22.209 | 41.372 | 1.00 | 23.38 | C |
| ATOM | 1098 | CG | PHE | A | 175 | 23.661 | 23.662 | 41.217 | 1.00 | 23.38 | C |
| ATOM | 1099 | CD1 | PHE | A | 175 | 24.101 | 24.173 | 39.996 | 1.00 | 23.38 | C |
| ATOM | 1100 | CD2 | PHE | A | 175 | 23.526 | 24.527 | 42.298 | 1.00 | 23.38 | C |
| ATOM | 1101 | CE1 | PHE | A | 175 | 24.400 | 25.528 | 39.865 | 1.00 | 23.38 | C |
| ATOM | 1102 | CE2 | PHE | A | 175 | 23.822 | 25.879 | 42.187 | 1.00 | 23.38 | C |
| ATOM | 1103 | CZ | PHE | A | 175 | 24.259 | 26.385 | 40.972 | 1.00 | 23.38 | C |
| ATOM | 1104 | C | PHE | A | 175 | 23.582 | 20.134 | 42.700 | 1.00 | 20.70 | C |
| ATOM | 1105 | O | PHE | A | 175 | 22.384 | 20.019 | 42.942 | 1.00 | 20.70 | O |
| ATOM | 1106 | N | GLY | A | 176 | 24.415 | 19.099 | 42.635 | 1.00 | 34.72 | N |
| ATOM | 1107 | CA | GLY | A | 176 | 23.942 | 17.752 | 42.877 | 1.00 | 34.72 | C |
| ATOM | 1108 | C | GLY | A | 176 | 23.068 | 17.198 | 41.763 | 1.00 | 34.72 | C |
| ATOM | 1109 | O | GLY | A | 176 | 22.448 | 16.134 | 41.922 | 1.00 | 34.72 | O |
| ATOM | 1110 | N | ILE | A | 177 | 23.009 | 17.915 | 40.640 | 1.00 | 28.18 | N |
| ATOM | 1111 | CA | ILE | A | 177 | 22.211 | 17.472 | 39.497 | 1.00 | 28.18 | C |
| ATOM | 1112 | CB | ILE | A | 177 | 21.720 | 18.643 | 38.637 | 1.00 | 32.28 | C |
| ATOM | 1113 | CG2 | ILE | A | 177 | 20.910 | 18.105 | 37.471 | 1.00 | 32.28 | C |
| ATOM | 1114 | CG1 | ILE | A | 177 | 20.863 | 19.591 | 39.475 | 1.00 | 32.28 | C |
| ATOM | 1115 | CD1 | ILE | A | 177 | 20.427 | 20.857 | 38.702 | 1.00 | 32.28 | C |
| ATOM | 1116 | C | ILE | A | 177 | 23.025 | 16.542 | 38.596 | 1.00 | 28.18 | C |
| ATOM | 1117 | O | ILE | A | 177 | 24.140 | 16.862 | 38.192 | 1.00 | 28.18 | O |
| ATOM | 1118 | N | CYS | A | 178 | 22.457 | 15.383 | 38.293 | 1.00 | 25.93 | N |
| ATOM | 1119 | CA | CYS | A | 178 | 23.113 | 14.398 | 37.444 | 1.00 | 25.93 | C |
| ATOM | 1120 | CB | CYS | A | 178 | 23.148 | 13.026 | 38.135 | 1.00 | 27.96 | C |
| ATOM | 1121 | SG | CYS | A | 178 | 23.916 | 11.754 | 37.085 | 1.00 | 27.96 | S |
| ATOM | 1122 | C | CYS | A | 178 | 22.339 | 14.319 | 36.143 | 1.00 | 25.93 | C |
| ATOM | 1123 | O | CYS | A | 178 | 21.134 | 14.107 | 36.144 | 1.00 | 25.93 | O |
| ATOM | 1124 | N | HIS | A | 179 | 23.045 | 14.494 | 35.035 | 1.00 | 39.21 | N |
| ATOM | 1125 | CA | HIS | A | 179 | 22.416 | 14.492 | 33.724 | 1.00 | 39.21 | C |
| ATOM | 1126 | CB | HIS | A | 179 | 23.439 | 14.832 | 32.638 | 1.00 | 32.96 | C |
| ATOM | 1127 | CG | HIS | A | 179 | 22.822 | 15.179 | 31.317 | 1.00 | 32.96 | C |
| ATOM | 1128 | CD2 | HIS | A | 179 | 22.462 | 16.364 | 30.785 | 1.00 | 32.96 | C |
| ATOM | 1129 | ND1 | HIS | A | 179 | 22.449 | 14.215 | 30.399 | 1.00 | 32.96 | N |

| | | | | | | | | | | | |
|---|---|---|---|---|---|---|---|---|---|---|---|
| ATOM | 1130 | CE1 | HIS | A | 179 | 21.885 | 14.804 | 29.360 | 1.00 | 32.96 | C |
| ATOM | 1131 | NE2 | HIS | A | 179 | 21.875 | 16.102 | 29.564 | 1.00 | 32.96 | N |
| ATOM | 1132 | C | HIS | A | 179 | 21.780 | 13.157 | 33.448 | 1.00 | 39.21 | C |
| ATOM | 1133 | O | HIS | A | 179 | 20.638 | 13.091 | 33.009 | 1.00 | 39.21 | O |
| ATOM | 1134 | N | ARG | A | 180 | 22.530 | 12.098 | 33.719 | 1.00 | 34.87 | N |
| ATOM | 1135 | CA | ARG | A | 180 | 22.051 | 10.736 | 33.517 | 1.00 | 34.87 | C |
| ATOM | 1136 | CB | ARG | A | 180 | 20.721 | 10.567 | 34.247 | 1.00 | 36.57 | C |
| ATOM | 1137 | CG | ARG | A | 180 | 20.918 | 10.107 | 35.665 | 1.00 | 36.57 | C |
| ATOM | 1138 | CD | ARG | A | 180 | 19.651 | 10.251 | 36.490 | 1.00 | 36.57 | C |
| ATOM | 1139 | NE | ARG | A | 180 | 19.354 | 8.975 | 37.129 | 1.00 | 36.57 | N |
| ATOM | 1140 | CZ | ARG | A | 180 | 18.134 | 8.470 | 37.249 | 1.00 | 36.57 | C |
| ATOM | 1141 | NH1 | ARG | A | 180 | 17.106 | 9.160 | 36.766 | 1.00 | 36.57 | N |
| ATOM | 1142 | NH2 | ARG | A | 180 | 17.939 | 7.288 | 37.845 | 1.00 | 36.57 | N |
| ATOM | 1143 | C | ARG | A | 180 | 21.895 | 10.237 | 32.062 | 1.00 | 34.87 | C |
| ATOM | 1144 | O | ARG | A | 180 | 21.421 | 9.112 | 31.842 | 1.00 | 34.87 | O |
| ATOM | 1145 | N | ASP | A | 181 | 22.286 | 11.044 | 31.076 | 1.00 | 28.81 | N |
| ATOM | 1146 | CA | ASP | A | 181 | 22.164 | 10.648 | 29.685 | 1.00 | 28.81 | C |
| ATOM | 1147 | CB | ASP | A | 181 | 20.741 | 10.889 | 29.177 | 1.00 | 34.86 | C |
| ATOM | 1148 | CG | ASP | A | 181 | 20.464 | 10.144 | 27.892 | 1.00 | 34.86 | C |
| ATOM | 1149 | OD1 | ASP | A | 181 | 21.216 | 9.164 | 27.631 | 1.00 | 34.86 | O |
| ATOM | 1150 | OD2 | ASP | A | 181 | 19.499 | 10.501 | 27.178 | 1.00 | 34.86 | O |
| ATOM | 1151 | C | ASP | A | 181 | 23.148 | 11.394 | 28.803 | 1.00 | 28.81 | C |
| ATOM | 1152 | O | ASP | A | 181 | 22.775 | 11.931 | 27.757 | 1.00 | 28.81 | O |
| ATOM | 1153 | N | ILE | A | 182 | 24.408 | 11.412 | 29.212 | 1.00 | 29.00 | N |
| ATOM | 1154 | CA | ILE | A | 182 | 25.429 | 12.095 | 28.438 | 1.00 | 29.00 | C |
| ATOM | 1155 | CB | ILE | A | 182 | 26.663 | 12.431 | 29.309 | 1.00 | 12.99 | C |
| ATOM | 1156 | CG2 | ILE | A | 182 | 27.734 | 13.107 | 28.466 | 1.00 | 12.99 | C |
| ATOM | 1157 | CG1 | ILE | A | 182 | 26.256 | 13.394 | 30.436 | 1.00 | 12.99 | C |
| ATOM | 1158 | CD1 | ILE | A | 182 | 25.823 | 14.755 | 29.960 | 1.00 | 12.99 | C |
| ATOM | 1159 | C | ILE | A | 182 | 25.846 | 11.242 | 27.254 | 1.00 | 29.00 | C |
| ATOM | 1160 | O | ILE | A | 182 | 26.456 | 10.196 | 27.426 | 1.00 | 29.00 | O |
| ATOM | 1161 | N | LYS | A | 183 | 25.481 | 11.691 | 26.058 | 1.00 | 28.26 | N |
| ATOM | 1162 | CA | LYS | A | 183 | 25.800 | 11.003 | 24.820 | 1.00 | 28.26 | C |
| ATOM | 1163 | CB | LYS | A | 183 | 24.670 | 10.048 | 24.439 | 1.00 | 27.22 | C |
| ATOM | 1164 | CG | LYS | A | 183 | 23.353 | 10.745 | 24.225 | 1.00 | 27.22 | C |
| ATOM | 1165 | CD | LYS | A | 183 | 22.271 | 9.769 | 23.845 | 1.00 | 27.22 | C |
| ATOM | 1166 | CE | LYS | A | 183 | 20.892 | 10.437 | 23.847 | 1.00 | 27.22 | C |
| ATOM | 1167 | NZ | LYS | A | 183 | 19.753 | 9.504 | 23.533 | 1.00 | 27.22 | N |
| ATOM | 1168 | C | LYS | A | 183 | 25.930 | 12.098 | 23.759 | 1.00 | 28.26 | C |
| ATOM | 1169 | O | LYS | A | 183 | 25.362 | 13.181 | 23.910 | 1.00 | 28.26 | O |
| ATOM | 1170 | N | PRO | A | 184 | 26.657 | 11.819 | 22.660 | 1.00 | 38.15 | N |
| ATOM | 1171 | CD | PRO | A | 184 | 27.084 | 10.454 | 22.313 | 1.00 | 14.24 | C |
| ATOM | 1172 | CA | PRO | A | 184 | 26.911 | 12.723 | 21.528 | 1.00 | 38.15 | C |
| ATOM | 1173 | CB | PRO | A | 184 | 27.347 | 11.764 | 20.434 | 1.00 | 14.24 | C |
| ATOM | 1174 | CG | PRO | A | 184 | 28.031 | 10.702 | 21.183 | 1.00 | 14.24 | C |
| ATOM | 1175 | C | PRO | A | 184 | 25.706 | 13.548 | 21.074 | 1.00 | 38.15 | C |
| ATOM | 1176 | O | PRO | A | 184 | 25.816 | 14.743 | 20.782 | 1.00 | 38.15 | O |
| ATOM | 1177 | N | GLN | A | 185 | 24.566 | 12.865 | 21.000 | 1.00 | 32.77 | N |
| ATOM | 1178 | CA | GLN | A | 185 | 23.291 | 13.424 | 20.563 | 1.00 | 32.77 | C |
| ATOM | 1179 | CB | GLN | A | 185 | 22.241 | 12.310 | 20.478 | 1.00 | 41.74 | C |
| ATOM | 1180 | CG | GLN | A | 185 | 22.586 | 11.195 | 19.499 | 1.00 | 41.74 | C |
| ATOM | 1181 | CD | GLN | A | 185 | 23.507 | 10.142 | 20.081 | 1.00 | 41.74 | C |
| ATOM | 1182 | OE1 | GLN | A | 185 | 24.278 | 10.406 | 21.010 | 1.00 | 41.74 | O |
| ATOM | 1183 | NE2 | GLN | A | 185 | 23.444 | 8.938 | 19.525 | 1.00 | 41.74 | N |
| ATOM | 1184 | C | GLN | A | 185 | 22.732 | 14.573 | 21.399 | 1.00 | 32.77 | C |
| ATOM | 1185 | O | GLN | A | 185 | 21.997 | 15.406 | 20.867 | 1.00 | 32.77 | O |
| ATOM | 1186 | N | ASN | A | 186 | 23.061 | 14.613 | 22.695 | 1.00 | 29.39 | N |
| ATOM | 1187 | CA | ASN | A | 186 | 22.575 | 15.678 | 23.590 | 1.00 | 29.39 | C |
| ATOM | 1188 | CB | ASN | A | 186 | 22.210 | 15.132 | 24.983 | 1.00 | 32.60 | C |
| ATOM | 1189 | CG | ASN | A | 186 | 21.181 | 14.047 | 24.934 | 1.00 | 32.60 | C |
| ATOM | 1190 | OD1 | ASN | A | 186 | 20.300 | 14.081 | 24.104 | 1.00 | 32.60 | O |
| ATOM | 1191 | ND2 | ASN | A | 186 | 21.278 | 13.080 | 25.839 | 1.00 | 32.60 | N |
| ATOM | 1192 | C | ASN | A | 186 | 23.605 | 16.794 | 23.784 | 1.00 | 29.39 | C |

| | | | | | | | | | | | |
|---|---|---|---|---|---|---|---|---|---|---|---|
| ATOM | 1193 | O | ASN | A | 186 | 23.511 | 17.585 | 24.732 | 1.00 | 29.39 | O |
| ATOM | 1194 | N | LEU | A | 187 | 24.594 | 16.842 | 22.896 | 1.00 | 36.77 | N |
| ATOM | 1195 | CA | LEU | A | 187 | 25.634 | 17.859 | 22.956 | 1.00 | 36.77 | C |
| ATOM | 1196 | CB | LEU | A | 187 | 27.010 | 17.201 | 23.044 | 1.00 | 19.99 | C |
| ATOM | 1197 | CG | LEU | A | 187 | 27.275 | 16.151 | 24.137 | 1.00 | 19.99 | C |
| ATOM | 1198 | CD1 | LEU | A | 187 | 28.680 | 15.581 | 23.931 | 1.00 | 19.99 | C |
| ATOM | 1199 | CD2 | LEU | A | 187 | 27.129 | 16.753 | 25.545 | 1.00 | 19.99 | C |
| ATOM | 1200 | C | LEU | A | 187 | 25.560 | 18.717 | 21.696 | 1.00 | 36.77 | C |
| ATOM | 1201 | O | LEU | A | 187 | 26.114 | 18.347 | 20.665 | 1.00 | 36.77 | O |
| ATOM | 1202 | N | LEU | A | 188 | 24.866 | 19.853 | 21.788 | 1.00 | 29.86 | N |
| ATOM | 1203 | CA | LEU | A | 188 | 24.702 | 20.785 | 20.665 | 1.00 | 29.86 | C |
| ATOM | 1204 | CB | LEU | A | 188 | 23.544 | 21.754 | 20.926 | 1.00 | 16.82 | C |
| ATOM | 1205 | CG | LEU | A | 188 | 22.291 | 21.128 | 21.558 | 1.00 | 16.82 | C |
| ATOM | 1206 | CD1 | LEU | A | 188 | 21.252 | 22.199 | 21.758 | 1.00 | 16.82 | C |
| ATOM | 1207 | CD2 | LEU | A | 188 | 21.743 | 19.974 | 20.682 | 1.00 | 16.82 | C |
| ATOM | 1208 | C | LEU | A | 188 | 25.951 | 21.613 | 20.475 | 1.00 | 29.86 | C |
| ATOM | 1209 | O | LEU | A | 188 | 26.729 | 21.806 | 21.408 | 1.00 | 29.86 | O |
| ATOM | 1210 | N | LEU | A | 189 | 26.154 | 22.115 | 19.271 | 1.00 | 26.56 | N |
| ATOM | 1211 | CA | LEU | A | 189 | 27.322 | 22.941 | 19.061 | 1.00 | 26.56 | C |
| ATOM | 1212 | CB | LEU | A | 189 | 28.592 | 22.084 | 19.137 | 1.00 | 41.86 | C |
| ATOM | 1213 | CG | LEU | A | 189 | 28.842 | 21.051 | 18.046 | 1.00 | 41.86 | C |
| ATOM | 1214 | CD1 | LEU | A | 189 | 29.301 | 21.762 | 16.791 | 1.00 | 41.86 | C |
| ATOM | 1215 | CD2 | LEU | A | 189 | 29.916 | 20.071 | 18.485 | 1.00 | 41.86 | C |
| ATOM | 1216 | C | LEU | A | 189 | 27.292 | 23.749 | 17.769 | 1.00 | 26.56 | C |
| ATOM | 1217 | O | LEU | A | 189 | 26.711 | 23.324 | 16.763 | 1.00 | 26.56 | O |
| ATOM | 1218 | N | ASP | A | 190 | 27.896 | 24.936 | 17.827 | 1.00 | 40.08 | N |
| ATOM | 1219 | CA | ASP | A | 190 | 27.996 | 25.814 | 16.672 | 1.00 | 40.08 | C |
| ATOM | 1220 | CB | ASP | A | 190 | 27.942 | 27.278 | 17.094 | 1.00 | 55.08 | C |
| ATOM | 1221 | CG | ASP | A | 190 | 27.546 | 28.190 | 15.954 | 1.00 | 55.08 | C |
| ATOM | 1222 | OD1 | ASP | A | 190 | 28.416 | 28.500 | 15.101 | 1.00 | 55.08 | O |
| ATOM | 1223 | OD2 | ASP | A | 190 | 26.352 | 28.576 | 15.908 | 1.00 | 55.08 | O |
| ATOM | 1224 | C | ASP | A | 190 | 29.356 | 25.484 | 16.083 | 1.00 | 40.08 | C |
| ATOM | 1225 | O | ASP | A | 190 | 30.395 | 25.821 | 16.661 | 1.00 | 40.08 | O |
| ATOM | 1226 | N | PRO | A | 191 | 29.367 | 24.823 | 14.915 | 1.00 | 44.37 | N |
| ATOM | 1227 | CD | PRO | A | 191 | 28.226 | 24.766 | 13.988 | 1.00 | 60.41 | C |
| ATOM | 1228 | CA | PRO | A | 191 | 30.613 | 24.429 | 14.247 | 1.00 | 44.37 | C |
| ATOM | 1229 | CB | PRO | A | 191 | 30.142 | 23.601 | 13.041 | 1.00 | 60.41 | C |
| ATOM | 1230 | CG | PRO | A | 191 | 28.630 | 23.683 | 13.033 | 1.00 | 60.41 | C |
| ATOM | 1231 | C | PRO | A | 191 | 31.532 | 25.568 | 13.832 | 1.00 | 44.37 | C |
| ATOM | 1232 | O | PRO | A | 191 | 32.698 | 25.337 | 13.529 | 1.00 | 44.37 | O |
| ATOM | 1233 | N | ASP | A | 192 | 31.014 | 26.791 | 13.810 | 1.00 | 43.59 | N |
| ATOM | 1234 | CA | ASP | A | 192 | 31.839 | 27.930 | 13.434 | 1.00 | 43.59 | C |
| ATOM | 1235 | CB | ASP | A | 192 | 31.005 | 29.010 | 12.758 | 1.00 | 71.88 | C |
| ATOM | 1236 | CG | ASP | A | 192 | 30.633 | 28.641 | 11.343 | 1.00 | 71.88 | C |
| ATOM | 1237 | OD1 | ASP | A | 192 | 31.474 | 28.037 | 10.644 | 1.00 | 71.88 | O |
| ATOM | 1238 | OD2 | ASP | A | 192 | 29.506 | 28.964 | 10.923 | 1.00 | 71.88 | O |
| ATOM | 1239 | C | ASP | A | 192 | 32.555 | 28.513 | 14.631 | 1.00 | 43.59 | C |
| ATOM | 1240 | O | ASP | A | 192 | 33.748 | 28.800 | 14.573 | 1.00 | 43.59 | O |
| ATOM | 1241 | N | THR | A | 193 | 31.824 | 28.685 | 15.722 | 1.00 | 30.17 | N |
| ATOM | 1242 | CA | THR | A | 193 | 32.417 | 29.227 | 16.937 | 1.00 | 30.17 | C |
| ATOM | 1243 | CB | THR | A | 193 | 31.385 | 30.007 | 17.739 | 1.00 | 29.26 | C |
| ATOM | 1244 | OG1 | THR | A | 193 | 30.313 | 29.128 | 18.090 | 1.00 | 29.26 | O |
| ATOM | 1245 | CG2 | THR | A | 193 | 30.844 | 31.166 | 16.910 | 1.00 | 29.26 | C |
| ATOM | 1246 | C | THR | A | 193 | 32.977 | 28.100 | 17.804 | 1.00 | 30.17 | C |
| ATOM | 1247 | O | THR | A | 193 | 33.744 | 28.343 | 18.743 | 1.00 | 30.17 | O |
| ATOM | 1248 | N | ALA | A | 194 | 32.573 | 26.872 | 17.485 | 1.00 | 26.36 | N |
| ATOM | 1249 | CA | ALA | A | 194 | 33.031 | 25.684 | 18.199 | 1.00 | 26.36 | C |
| ATOM | 1250 | CB | ALA | A | 194 | 34.555 | 25.670 | 18.234 | 1.00 | 11.10 | C |
| ATOM | 1251 | C | ALA | A | 194 | 32.488 | 25.552 | 19.624 | 1.00 | 26.36 | C |
| ATOM | 1252 | O | ALA | A | 194 | 33.017 | 24.762 | 20.420 | 1.00 | 26.36 | O |
| ATOM | 1253 | N | VAL | A | 195 | 31.437 | 26.307 | 19.940 | 1.00 | 23.80 | N |
| ATOM | 1254 | CA | VAL | A | 195 | 30.871 | 26.279 | 21.282 | 1.00 | 23.80 | C |
| ATOM | 1255 | CB | VAL | A | 195 | 30.160 | 27.632 | 21.628 | 1.00 | 37.97 | C |

| | | | | | | | | | | | |
|---|---|---|---|---|---|---|---|---|---|---|---|
| ATOM | 1256 | CG1 | VAL | A | 195 | 30.990 | 28.806 | 21.094 | 1.00 | 37.97 | C |
| ATOM | 1257 | CG2 | VAL | A | 195 | 28.754 | 27.667 | 21.064 | 1.00 | 37.97 | C |
| ATOM | 1258 | C | VAL | A | 195 | 29.919 | 25.103 | 21.483 | 1.00 | 23.80 | C |
| ATOM | 1259 | O | VAL | A | 195 | 29.095 | 24.781 | 20.626 | 1.00 | 23.80 | O |
| ATOM | 1260 | N | LEU | A | 196 | 30.048 | 24.462 | 22.636 | 1.00 | 35.42 | N |
| ATOM | 1261 | CA | LEU | A | 196 | 29.245 | 23.301 | 22.953 | 1.00 | 35.42 | C |
| ATOM | 1262 | CB | LEU | A | 196 | 30.179 | 22.182 | 23.392 | 1.00 | 24.79 | C |
| ATOM | 1263 | CG | LEU | A | 196 | 29.538 | 20.848 | 23.743 | 1.00 | 24.79 | C |
| ATOM | 1264 | CD1 | LEU | A | 196 | 30.491 | 19.729 | 23.367 | 1.00 | 24.79 | C |
| ATOM | 1265 | CD2 | LEU | A | 196 | 29.180 | 20.810 | 25.228 | 1.00 | 24.79 | C |
| ATOM | 1266 | C | LEU | A | 196 | 28.205 | 23.590 | 24.030 | 1.00 | 35.42 | C |
| ATOM | 1267 | O | LEU | A | 196 | 28.505 | 24.228 | 25.030 | 1.00 | 35.42 | O |
| ATOM | 1268 | N | LYS | A | 197 | 26.982 | 23.108 | 23.819 | 1.00 | 34.18 | N |
| ATOM | 1269 | CA | LYS | A | 197 | 25.887 | 23.319 | 24.759 | 1.00 | 34.18 | C |
| ATOM | 1270 | CB | LYS | A | 197 | 24.839 | 24.235 | 24.130 | 1.00 | 24.21 | C |
| ATOM | 1271 | CG | LYS | A | 197 | 25.363 | 25.549 | 23.594 | 1.00 | 24.21 | C |
| ATOM | 1272 | CD | LYS | A | 197 | 25.357 | 26.662 | 24.641 | 1.00 | 24.21 | C |
| ATOM | 1273 | CE | LYS | A | 197 | 25.711 | 28.022 | 24.011 | 1.00 | 24.21 | C |
| ATOM | 1274 | NZ | LYS | A | 197 | 25.568 | 29.177 | 24.948 | 1.00 | 24.21 | N |
| ATOM | 1275 | C | LYS | A | 197 | 25.197 | 22.012 | 25.161 | 1.00 | 34.18 | C |
| ATOM | 1276 | O | LYS | A | 197 | 24.617 | 21.323 | 24.315 | 1.00 | 34.18 | O |
| ATOM | 1277 | N | LEU | A | 198 | 25.244 | 21.690 | 26.453 | 1.00 | 23.66 | N |
| ATOM | 1278 | CA | LEU | A | 198 | 24.604 | 20.495 | 26.990 | 1.00 | 23.66 | C |
| ATOM | 1279 | CB | LEU | A | 198 | 25.010 | 20.308 | 28.442 | 1.00 | 19.87 | C |
| ATOM | 1280 | CG | LEU | A | 198 | 24.470 | 19.067 | 29.139 | 1.00 | 19.87 | C |
| ATOM | 1281 | CD1 | LEU | A | 198 | 25.010 | 17.822 | 28.463 | 1.00 | 19.87 | C |
| ATOM | 1282 | CD2 | LEU | A | 198 | 24.871 | 19.120 | 30.606 | 1.00 | 19.87 | C |
| ATOM | 1283 | C | LEU | A | 198 | 23.101 | 20.709 | 26.932 | 1.00 | 23.66 | C |
| ATOM | 1284 | O | LEU | A | 198 | 22.626 | 21.754 | 27.367 | 1.00 | 23.66 | O |
| ATOM | 1285 | N | CYS | A | 199 | 22.354 | 19.739 | 26.400 | 1.00 | 20.98 | N |
| ATOM | 1286 | CA | CYS | A | 199 | 20.891 | 19.864 | 26.315 | 1.00 | 20.98 | C |
| ATOM | 1287 | CB | CYS | A | 199 | 20.469 | 20.154 | 24.880 | 1.00 | 29.42 | C |
| ATOM | 1288 | SG | CYS | A | 199 | 20.630 | 18.692 | 23.817 | 1.00 | 29.42 | S |
| ATOM | 1289 | C | CYS | A | 199 | 20.169 | 18.596 | 26.781 | 1.00 | 20.98 | C |
| ATOM | 1290 | O | CYS | A | 199 | 20.797 | 17.595 | 27.106 | 1.00 | 20.98 | O |
| ATOM | 1291 | N | ASP | A | 200 | 18.842 | 18.657 | 26.812 | 1.00 | 34.06 | N |
| ATOM | 1292 | CA | ASP | A | 200 | 18.026 | 17.513 | 27.200 | 1.00 | 34.06 | C |
| ATOM | 1293 | CB | ASP | A | 200 | 18.319 | 16.345 | 26.250 | 1.00 | 48.97 | C |
| ATOM | 1294 | CG | ASP | A | 200 | 17.372 | 15.174 | 26.431 | 1.00 | 48.97 | C |
| ATOM | 1295 | OD1 | ASP | A | 200 | 16.369 | 15.307 | 27.156 | 1.00 | 48.97 | O |
| ATOM | 1296 | OD2 | ASP | A | 200 | 17.625 | 14.113 | 25.827 | 1.00 | 48.97 | O |
| ATOM | 1297 | C | ASP | A | 200 | 18.213 | 17.052 | 28.645 | 1.00 | 34.06 | C |
| ATOM | 1298 | O | ASP | A | 200 | 19.108 | 16.260 | 28.940 | 1.00 | 34.06 | O |
| ATOM | 1299 | N | PHE | A | 201 | 17.363 | 17.527 | 29.545 | 1.00 | 45.49 | N |
| ATOM | 1300 | CA | PHE | A | 201 | 17.458 | 17.102 | 30.936 | 1.00 | 45.49 | C |
| ATOM | 1301 | CB | PHE | A | 201 | 17.649 | 18.307 | 31.861 | 1.00 | 30.56 | C |
| ATOM | 1302 | CG | PHE | A | 201 | 18.934 | 19.039 | 31.632 | 1.00 | 30.56 | C |
| ATOM | 1303 | CD1 | PHE | A | 201 | 19.007 | 20.064 | 30.682 | 1.00 | 30.56 | C |
| ATOM | 1304 | CD2 | PHE | A | 201 | 20.091 | 18.666 | 32.328 | 1.00 | 30.56 | C |
| ATOM | 1305 | CE1 | PHE | A | 201 | 20.219 | 20.710 | 30.422 | 1.00 | 30.56 | C |
| ATOM | 1306 | CE2 | PHE | A | 201 | 21.309 | 19.298 | 32.083 | 1.00 | 30.56 | C |
| ATOM | 1307 | CZ | PHE | A | 201 | 21.377 | 20.327 | 31.122 | 1.00 | 30.56 | C |
| ATOM | 1308 | C | PHE | A | 201 | 16.225 | 16.306 | 31.357 | 1.00 | 45.49 | C |
| ATOM | 1309 | O | PHE | A | 201 | 15.696 | 16.485 | 32.461 | 1.00 | 45.49 | O |
| ATOM | 1310 | N | GLY | A | 202 | 15.781 | 15.423 | 30.467 | 1.00 | 44.79 | N |
| ATOM | 1311 | CA | GLY | A | 202 | 14.618 | 14.600 | 30.738 | 1.00 | 44.79 | C |
| ATOM | 1312 | C | GLY | A | 202 | 15.006 | 13.524 | 31.721 | 1.00 | 44.79 | C |
| ATOM | 1313 | O | GLY | A | 202 | 14.253 | 13.206 | 32.637 | 1.00 | 44.79 | O |
| ATOM | 1314 | N | SER | A | 203 | 16.200 | 12.972 | 31.543 | 1.00 | 24.03 | N |
| ATOM | 1315 | CA | SER | A | 203 | 16.697 | 11.924 | 32.438 | 1.00 | 24.03 | C |
| ATOM | 1316 | CB | SER | A | 203 | 17.698 | 11.041 | 31.699 | 1.00 | 34.17 | C |
| ATOM | 1317 | OG | SER | A | 203 | 17.126 | 10.584 | 30.495 | 1.00 | 34.17 | O |
| ATOM | 1318 | C | SER | A | 203 | 17.367 | 12.475 | 33.705 | 1.00 | 24.03 | C |

| | | | | | | | | | | | |
|---|---|---|---|---|---|---|---|---|---|---|---|
| ATOM | 1319 | O | SER | A | 203 | 17.644 | 11.711 | 34.643 | 1.00 | 24.03 | O |
| ATOM | 1320 | N | ALA | A | 204 | 17.630 | 13.788 | 33.717 | 1.00 | 31.63 | N |
| ATOM | 1321 | CA | ALA | A | 204 | 18.289 | 14.468 | 34.844 | 1.00 | 31.63 | C |
| ATOM | 1322 | CB | ALA | A | 204 | 18.519 | 15.990 | 34.541 | 1.00 | 8.87 | C |
| ATOM | 1323 | C | ALA | A | 204 | 17.533 | 14.326 | 36.146 | 1.00 | 31.63 | C |
| ATOM | 1324 | O | ALA | A | 204 | 16.318 | 14.101 | 36.176 | 1.00 | 31.63 | O |
| ATOM | 1325 | N | LYS | A | 205 | 18.270 | 14.509 | 37.232 | 1.00 | 47.66 | N |
| ATOM | 1326 | CA | LYS | A | 205 | 17.716 | 14.379 | 38.570 | 1.00 | 47.66 | C |
| ATOM | 1327 | CB | LYS | A | 205 | 17.251 | 12.939 | 38.780 | 1.00 | 37.58 | C |
| ATOM | 1328 | CG | LYS | A | 205 | 16.704 | 12.640 | 40.161 | 1.00 | 37.58 | C |
| ATOM | 1329 | CD | LYS | A | 205 | 16.547 | 11.140 | 40.353 | 1.00 | 37.58 | C |
| ATOM | 1330 | CE | LYS | A | 205 | 15.866 | 10.802 | 41.661 | 1.00 | 37.58 | C |
| ATOM | 1331 | NZ | LYS | A | 205 | 15.729 | 9.338 | 41.873 | 1.00 | 37.58 | N |
| ATOM | 1332 | C | LYS | A | 205 | 18.797 | 14.713 | 39.589 | 1.00 | 47.66 | C |
| ATOM | 1333 | O | LYS | A | 205 | 19.987 | 14.487 | 39.344 | 1.00 | 47.66 | O |
| ATOM | 1334 | N | GLN | A | 206 | 18.392 | 15.255 | 40.732 | 1.00 | 42.57 | N |
| ATOM | 1335 | CA | GLN | A | 206 | 19.368 | 15.580 | 41.762 | 1.00 | 42.57 | C |
| ATOM | 1336 | CB | GLN | A | 206 | 18.963 | 16.826 | 42.534 | 1.00 | 45.57 | C |
| ATOM | 1337 | CG | GLN | A | 206 | 17.829 | 17.575 | 41.909 | 1.00 | 45.57 | C |
| ATOM | 1338 | CD | GLN | A | 206 | 17.781 | 19.000 | 42.372 | 1.00 | 45.57 | C |
| ATOM | 1339 | OE1 | GLN | A | 206 | 16.793 | 19.696 | 42.144 | 1.00 | 45.57 | O |
| ATOM | 1340 | NE2 | GLN | A | 206 | 18.853 | 19.456 | 43.020 | 1.00 | 45.57 | N |
| ATOM | 1341 | C | GLN | A | 206 | 19.410 | 14.377 | 42.674 | 1.00 | 42.57 | C |
| ATOM | 1342 | O | GLN | A | 206 | 18.449 | 14.077 | 43.384 | 1.00 | 42.57 | O |
| ATOM | 1343 | N | LEU | A | 207 | 20.528 | 13.675 | 42.631 | 1.00 | 34.88 | N |
| ATOM | 1344 | CA | LEU | A | 207 | 20.690 | 12.482 | 43.418 | 1.00 | 34.88 | C |
| ATOM | 1345 | CB | LEU | A | 207 | 21.732 | 11.582 | 42.763 | 1.00 | 34.24 | C |
| ATOM | 1346 | CG | LEU | A | 207 | 21.746 | 11.423 | 41.245 | 1.00 | 34.24 | C |
| ATOM | 1347 | CD1 | LEU | A | 207 | 22.939 | 10.549 | 40.898 | 1.00 | 34.24 | C |
| ATOM | 1348 | CD2 | LEU | A | 207 | 20.433 | 10.813 | 40.718 | 1.00 | 34.24 | C |
| ATOM | 1349 | C | LEU | A | 207 | 21.094 | 12.743 | 44.864 | 1.00 | 34.88 | C |
| ATOM | 1350 | O | LEU | A | 207 | 22.103 | 13.410 | 45.149 | 1.00 | 34.88 | O |
| ATOM | 1351 | N | VAL | A | 208 | 20.296 | 12.194 | 45.773 | 1.00 | 39.95 | N |
| ATOM | 1352 | CA | VAL | A | 208 | 20.553 | 12.298 | 47.200 | 1.00 | 39.95 | C |
| ATOM | 1353 | CB | VAL | A | 208 | 19.404 | 12.981 | 47.930 | 1.00 | 54.85 | C |
| ATOM | 1354 | CG1 | VAL | A | 208 | 19.293 | 14.383 | 47.460 | 1.00 | 54.85 | C |
| ATOM | 1355 | CG2 | VAL | A | 208 | 18.107 | 12.250 | 47.672 | 1.00 | 54.85 | C |
| ATOM | 1356 | C | VAL | A | 208 | 20.670 | 10.870 | 47.702 | 1.00 | 39.95 | C |
| ATOM | 1357 | O | VAL | A | 208 | 19.805 | 10.033 | 47.416 | 1.00 | 39.95 | O |
| ATOM | 1358 | N | ARG | A | 209 | 21.743 | 10.585 | 48.431 | 1.00 | 35.64 | N |
| ATOM | 1359 | CA | ARG | A | 209 | 21.962 | 9.240 | 48.947 | 1.00 | 35.64 | C |
| ATOM | 1360 | CB | ARG | A | 209 | 23.307 | 9.176 | 49.663 | 1.00 | 34.73 | C |
| ATOM | 1361 | CG | ARG | A | 209 | 24.224 | 8.122 | 49.120 | 1.00 | 34.73 | C |
| ATOM | 1362 | CD | ARG | A | 209 | 23.912 | 6.828 | 49.780 | 1.00 | 34.73 | C |
| ATOM | 1363 | NE | ARG | A | 209 | 24.418 | 6.807 | 51.152 | 1.00 | 34.73 | N |
| ATOM | 1364 | CZ | ARG | A | 209 | 25.546 | 6.209 | 51.548 | 1.00 | 34.73 | C |
| ATOM | 1365 | NH1 | ARG | A | 209 | 26.330 | 5.560 | 50.677 | 1.00 | 34.73 | N |
| ATOM | 1366 | NH2 | ARG | A | 209 | 25.881 | 6.226 | 52.837 | 1.00 | 34.73 | N |
| ATOM | 1367 | C | ARG | A | 209 | 20.832 | 8.803 | 49.881 | 1.00 | 35.64 | C |
| ATOM | 1368 | O | ARG | A | 209 | 20.302 | 9.596 | 50.670 | 1.00 | 35.64 | O |
| ATOM | 1369 | N | GLY | A | 210 | 20.462 | 7.531 | 49.775 | 1.00 | 44.07 | N |
| ATOM | 1370 | CA | GLY | A | 210 | 19.396 | 6.998 | 50.600 | 1.00 | 44.07 | C |
| ATOM | 1371 | C | GLY | A | 210 | 18.103 | 6.940 | 49.813 | 1.00 | 44.07 | C |
| ATOM | 1372 | O | GLY | A | 210 | 17.270 | 6.045 | 49.991 | 1.00 | 44.07 | O |
| ATOM | 1373 | N | GLU | A | 211 | 17.933 | 7.914 | 48.929 | 1.00 | 45.77 | N |
| ATOM | 1374 | CA | GLU | A | 211 | 16.744 | 7.983 | 48.082 | 1.00 | 45.77 | C |
| ATOM | 1375 | CB | GLU | A | 211 | 16.358 | 9.438 | 47.834 | 1.00 | 67.80 | C |
| ATOM | 1376 | CG | GLU | A | 211 | 14.873 | 9.639 | 47.681 | 1.00 | 67.80 | C |
| ATOM | 1377 | CD | GLU | A | 211 | 14.149 | 9.565 | 49.008 | 1.00 | 67.80 | C |
| ATOM | 1378 | OE1 | GLU | A | 211 | 14.351 | 10.468 | 49.848 | 1.00 | 67.80 | O |
| ATOM | 1379 | OE2 | GLU | A | 211 | 13.382 | 8.604 | 49.215 | 1.00 | 67.80 | O |
| ATOM | 1380 | C | GLU | A | 211 | 17.092 | 7.304 | 46.754 | 1.00 | 45.77 | C |
| ATOM | 1381 | O | GLU | A | 211 | 17.648 | 7.931 | 45.855 | 1.00 | 45.77 | O |

| | | | | | | | | | | | |
|---|---|---|---|---|---|---|---|---|---|---|---|
| ATOM | 1382 | N | PRO | A | 212 | 16.726 | 6.019 | 46.610 | 1.00 | 40.11 | N |
| ATOM | 1383 | CD | PRO | A | 212 | 15.668 | 5.419 | 47.440 | 1.00 | 14.05 | C |
| ATOM | 1384 | CA | PRO | A | 212 | 16.969 | 5.172 | 45.443 | 1.00 | 40.11 | C |
| ATOM | 1385 | CB | PRO | A | 212 | 16.186 | 3.913 | 45.758 | 1.00 | 14.05 | C |
| ATOM | 1386 | CG | PRO | A | 212 | 15.021 | 4.448 | 46.466 | 1.00 | 14.05 | C |
| ATOM | 1387 | C | PRO | A | 212 | 16.622 | 5.728 | 44.088 | 1.00 | 40.11 | C |
| ATOM | 1388 | O | PRO | A | 212 | 15.564 | 6.309 | 43.855 | 1.00 | 40.11 | O |
| ATOM | 1389 | N | ASN | A | 213 | 17.550 | 5.486 | 43.183 | 1.00 | 39.80 | N |
| ATOM | 1390 | CA | ASN | A | 213 | 17.444 | 5.961 | 41.826 | 1.00 | 39.80 | C |
| ATOM | 1391 | CB | ASN | A | 213 | 18.635 | 6.846 | 41.534 | 1.00 | 39.29 | C |
| ATOM | 1392 | CG | ASN | A | 213 | 18.900 | 7.831 | 42.646 | 1.00 | 39.29 | C |
| ATOM | 1393 | OD1 | ASN | A | 213 | 18.261 | 8.884 | 42.719 | 1.00 | 39.29 | O |
| ATOM | 1394 | ND2 | ASN | A | 213 | 19.834 | 7.485 | 43.542 | 1.00 | 39.29 | N |
| ATOM | 1395 | C | ASN | A | 213 | 17.433 | 4.752 | 40.947 | 1.00 | 39.80 | C |
| ATOM | 1396 | O | ASN | A | 213 | 18.113 | 3.777 | 41.264 | 1.00 | 39.80 | O |
| ATOM | 1397 | N | VAL | A | 214 | 16.678 | 4.821 | 39.853 | 1.00 | 34.97 | N |
| ATOM | 1398 | CA | VAL | A | 214 | 16.512 | 3.686 | 38.950 | 1.00 | 34.97 | C |
| ATOM | 1399 | CB | VAL | A | 214 | 15.534 | 4.016 | 37.812 | 1.00 | 30.29 | C |
| ATOM | 1400 | CG1 | VAL | A | 214 | 16.153 | 4.989 | 36.836 | 1.00 | 30.29 | C |
| ATOM | 1401 | CG2 | VAL | A | 214 | 15.139 | 2.732 | 37.112 | 1.00 | 30.29 | C |
| ATOM | 1402 | C | VAL | A | 214 | 17.810 | 3.159 | 38.338 | 1.00 | 34.97 | C |
| ATOM | 1403 | O | VAL | A | 214 | 18.659 | 3.929 | 37.941 | 1.00 | 34.97 | O |
| ATOM | 1404 | N | SER | A | 215 | 17.990 | 1.854 | 38.205 | 1.00 | 58.90 | N |
| ATOM | 1405 | CA | SER | A | 215 | 19.253 | 1.398 | 37.612 | 1.00 | 58.90 | C |
| ATOM | 1406 | CB | SER | A | 215 | 19.560 | -0.076 | 37.920 | 1.00 | 57.81 | C |
| ATOM | 1407 | OG | SER | A | 215 | 18.639 | -0.723 | 38.816 | 1.00 | 57.81 | O |
| ATOM | 1408 | C | SER | A | 215 | 18.696 | 1.483 | 36.213 | 1.00 | 58.90 | C |
| ATOM | 1409 | O | SER | A | 215 | 17.720 | 2.161 | 36.040 | 1.00 | 58.90 | O |
| ATOM | 1410 | N | PTY | A | 216 | 19.225 | 0.724 | 35.245 | 1.00 | 36.49 | N |
| ATOM | 1411 | CA | PTY | A | 216 | 18.739 | 0.735 | 33.841 | 1.00 | 36.49 | C |
| ATOM | 1412 | C | PTY | A | 216 | 18.523 | 2.186 | 33.405 | 1.00 | 36.49 | C |
| ATOM | 1413 | O | PTY | A | 216 | 17.390 | 2.725 | 33.625 | 1.00 | 36.49 | O |
| ATOM | 1414 | CB | PTY | A | 216 | 17.451 | -0.144 | 33.686 | 1.00 | 80.44 | C |
| ATOM | 1415 | CG | PTY | A | 216 | 17.498 | -0.412 | 32.177 | 1.00 | 80.44 | C |
| ATOM | 1416 | CD1 | PTY | A | 216 | 16.360 | 0.085 | 31.436 | 1.00 | 80.44 | C |
| ATOM | 1417 | CD2 | PTY | A | 216 | 18.565 | -1.108 | 31.406 | 1.00 | 80.44 | C |
| ATOM | 1418 | CE1 | PTY | A | 216 | 16.237 | -0.086 | 29.981 | 1.00 | 80.44 | C |
| ATOM | 1419 | CE2 | PTY | A | 216 | 18.454 | -1.290 | 29.947 | 1.00 | 80.44 | C |
| ATOM | 1420 | CZ | PTY | A | 216 | 17.286 | -0.785 | 29.194 | 1.00 | 80.44 | C |
| ATOM | 1421 | OH | PTY | A | 216 | 17.134 | -0.909 | 27.784 | 1.00 | 80.44 | O |
| ATOM | 1422 | P | PTY | A | 216 | 18.246 | -0.970 | 26.713 | 1.00 | 80.44 | P |
| ATOM | 1423 | OP1 | PTY | A | 216 | 18.909 | 0.292 | 26.591 | 1.00 | 80.44 | O |
| ATOM | 1424 | OP2 | PTY | A | 216 | 17.419 | -1.327 | 25.461 | 1.00 | 80.44 | O |
| ATOM | 1425 | OP3 | PTY | A | 216 | 19.179 | -2.008 | 27.015 | 1.00 | 80.44 | O |
| ATOM | 1426 | N | ILE | A | 217 | 19.571 | 2.846 | 32.911 | 1.00 | 42.50 | N |
| ATOM | 1427 | CA | ILE | A | 217 | 19.383 | 4.228 | 32.533 | 1.00 | 42.50 | C |
| ATOM | 1428 | CB | ILE | A | 217 | 19.183 | 5.109 | 33.740 | 1.00 | 28.87 | C |
| ATOM | 1429 | CG2 | ILE | A | 217 | 20.527 | 5.592 | 34.308 | 1.00 | 28.87 | C |
| ATOM | 1430 | CG1 | ILE | A | 217 | 18.287 | 6.225 | 33.299 | 1.00 | 28.87 | C |
| ATOM | 1431 | CD1 | ILE | A | 217 | 18.178 | 7.266 | 34.252 | 1.00 | 28.87 | C |
| ATOM | 1432 | C | ILE | A | 217 | 20.594 | 4.731 | 31.803 | 1.00 | 42.50 | C |
| ATOM | 1433 | O | ILE | A | 217 | 21.724 | 4.279 | 32.058 | 1.00 | 42.50 | O |
| ATOM | 1434 | N | CYS | A | 218 | 20.355 | 5.660 | 30.887 | 1.00 | 45.77 | N |
| ATOM | 1435 | CA | CYS | A | 218 | 21.416 | 6.230 | 30.093 | 1.00 | 45.77 | C |
| ATOM | 1436 | CB | CYS | A | 218 | 22.678 | 6.400 | 30.949 | 1.00 | 59.57 | C |
| ATOM | 1437 | SG | CYS | A | 218 | 24.130 | 7.076 | 30.122 | 1.00 | 59.57 | S |
| ATOM | 1438 | C | CYS | A | 218 | 21.704 | 5.368 | 28.875 | 1.00 | 45.77 | C |
| ATOM | 1439 | O | CYS | A | 218 | 21.898 | 4.151 | 28.986 | 1.00 | 45.77 | O |
| ATOM | 1440 | N | SER | A | 219 | 21.726 | 6.007 | 27.711 | 1.00 | 47.72 | N |
| ATOM | 1441 | CA | SER | A | 219 | 22.019 | 5.304 | 26.466 | 1.00 | 47.72 | C |
| ATOM | 1442 | CB | SER | A | 219 | 22.644 | 6.276 | 25.451 | 1.00 | 44.18 | C |
| ATOM | 1443 | OG | SER | A | 219 | 21.694 | 6.604 | 24.448 | 1.00 | 44.18 | O |
| ATOM | 1444 | C | SER | A | 219 | 22.938 | 4.083 | 26.683 | 1.00 | 47.72 | C |

| | | | | | | | | | | | |
|---|---|---|---|---|---|---|---|---|---|---|---|
| ATOM | 1445 | O | SER | A | 219 | 24.066 | 4.236 | 27.167 | 1.00 | 47.72 | O |
| ATOM | 1446 | N | ARG | A | 220 | 22.446 | 2.884 | 26.349 | 1.00 | 26.24 | N |
| ATOM | 1447 | CA | ARG | A | 220 | 23.206 | 1.649 | 26.528 | 1.00 | 26.24 | C |
| ATOM | 1448 | CB | ARG | A | 220 | 22.721 | 0.547 | 25.581 | 1.00 | 68.23 | C |
| ATOM | 1449 | CG | ARG | A | 220 | 23.815 | -0.580 | 25.407 | 1.00 | 68.23 | C |
| ATOM | 1450 | CD | ARG | A | 220 | 23.266 | -1.951 | 25.057 | 1.00 | 68.23 | C |
| ATOM | 1451 | NE | ARG | A | 220 | 22.090 | -1.703 | 24.253 | 1.00 | 68.23 | N |
| ATOM | 1452 | CZ | ARG | A | 220 | 20.971 | -1.240 | 24.791 | 1.00 | 68.23 | C |
| ATOM | 1453 | NH1 | ARG | A | 220 | 20.953 | -1.018 | 26.111 | 1.00 | 68.23 | N |
| ATOM | 1454 | NH2 | ARG | A | 220 | 19.913 | -0.972 | 24.028 | 1.00 | 68.23 | N |
| ATOM | 1455 | C | ARG | A | 220 | 24.704 | 1.774 | 26.304 | 1.00 | 26.24 | C |
| ATOM | 1456 | O | ARG | A | 220 | 25.500 | 1.226 | 27.062 | 1.00 | 26.24 | O |
| ATOM | 1457 | N | TYR | A | 221 | 25.047 | 2.506 | 25.246 | 1.00 | 41.63 | N |
| ATOM | 1458 | CA | TYR | A | 221 | 26.418 | 2.666 | 24.810 | 1.00 | 41.63 | C |
| ATOM | 1459 | CB | TYR | A | 221 | 26.437 | 3.301 | 23.426 | 1.00 | 68.07 | C |
| ATOM | 1460 | CG | TYR | A | 221 | 26.013 | 2.409 | 22.305 | 1.00 | 68.07 | C |
| ATOM | 1461 | CD1 | TYR | A | 221 | 25.327 | 1.201 | 22.510 | 1.00 | 68.07 | C |
| ATOM | 1462 | CE1 | TYR | A | 221 | 24.973 | 0.398 | 21.417 | 1.00 | 68.07 | C |
| ATOM | 1463 | CD2 | TYR | A | 221 | 26.322 | 2.778 | 21.013 | 1.00 | 68.07 | C |
| ATOM | 1464 | CE2 | TYR | A | 221 | 25.987 | 1.984 | 19.923 | 1.00 | 68.07 | C |
| ATOM | 1465 | CZ | TYR | A | 221 | 25.309 | 0.796 | 20.126 | 1.00 | 68.07 | C |
| ATOM | 1466 | OH | TYR | A | 221 | 24.959 | 0.011 | 19.047 | 1.00 | 68.07 | O |
| ATOM | 1467 | C | TYR | A | 221 | 27.289 | 3.465 | 25.730 | 1.00 | 41.63 | C |
| ATOM | 1468 | O | TYR | A | 221 | 28.496 | 3.223 | 25.825 | 1.00 | 41.63 | O |
| ATOM | 1469 | N | TYR | A | 222 | 26.649 | 4.417 | 26.397 | 1.00 | 37.92 | N |
| ATOM | 1470 | CA | TYR | A | 222 | 27.310 | 5.338 | 27.305 | 1.00 | 37.92 | C |
| ATOM | 1471 | CB | TYR | A | 222 | 26.861 | 6.743 | 26.942 | 1.00 | 39.51 | C |
| ATOM | 1472 | CG | TYR | A | 222 | 27.229 | 7.091 | 25.529 | 1.00 | 39.51 | C |
| ATOM | 1473 | CD1 | TYR | A | 222 | 26.341 | 6.881 | 24.456 | 1.00 | 39.51 | C |
| ATOM | 1474 | CE1 | TYR | A | 222 | 26.737 | 7.188 | 23.148 | 1.00 | 39.51 | C |
| ATOM | 1475 | CD2 | TYR | A | 222 | 28.500 | 7.602 | 25.257 | 1.00 | 39.51 | C |
| ATOM | 1476 | CE2 | TYR | A | 222 | 28.890 | 7.901 | 23.982 | 1.00 | 39.51 | C |
| ATOM | 1477 | CZ | TYR | A | 222 | 28.011 | 7.694 | 22.936 | 1.00 | 39.51 | C |
| ATOM | 1478 | OH | TYR | A | 222 | 28.380 | 8.005 | 21.662 | 1.00 | 39.51 | O |
| ATOM | 1479 | C | TYR | A | 222 | 27.033 | 5.066 | 28.773 | 1.00 | 37.92 | C |
| ATOM | 1480 | O | TYR | A | 222 | 27.455 | 5.815 | 29.656 | 1.00 | 37.92 | O |
| ATOM | 1481 | N | ARG | A | 223 | 26.334 | 3.976 | 29.026 | 1.00 | 42.25 | N |
| ATOM | 1482 | CA | ARG | A | 223 | 25.976 | 3.616 | 30.366 | 1.00 | 42.25 | C |
| ATOM | 1483 | CB | ARG | A | 223 | 24.838 | 2.629 | 30.299 | 1.00 | 51.01 | C |
| ATOM | 1484 | CG | ARG | A | 223 | 23.906 | 2.793 | 31.419 | 1.00 | 51.01 | C |
| ATOM | 1485 | CD | ARG | A | 223 | 22.530 | 2.353 | 31.008 | 1.00 | 51.01 | C |
| ATOM | 1486 | NE | ARG | A | 223 | 22.412 | 0.944 | 30.704 | 1.00 | 51.01 | N |
| ATOM | 1487 | CZ | ARG | A | 223 | 21.585 | 0.476 | 29.773 | 1.00 | 51.01 | C |
| ATOM | 1488 | NH1 | ARG | A | 223 | 20.807 | 1.311 | 29.058 | 1.00 | 51.01 | N |
| ATOM | 1489 | NH2 | ARG | A | 223 | 21.543 | -0.830 | 29.540 | 1.00 | 51.01 | N |
| ATOM | 1490 | C | ARG | A | 223 | 27.154 | 3.069 | 31.179 | 1.00 | 42.25 | C |
| ATOM | 1491 | O | ARG | A | 223 | 27.888 | 2.168 | 30.755 | 1.00 | 42.25 | O |
| ATOM | 1492 | N | ALA | A | 224 | 27.342 | 3.677 | 32.342 | 1.00 | 38.57 | N |
| ATOM | 1493 | CA | ALA | A | 224 | 28.397 | 3.310 | 33.295 | 1.00 | 38.57 | C |
| ATOM | 1494 | CB | ALA | A | 224 | 28.377 | 4.251 | 34.470 | 1.00 | 62.40 | C |
| ATOM | 1495 | C | ALA | A | 224 | 28.207 | 1.868 | 33.777 | 1.00 | 38.57 | C |
| ATOM | 1496 | O | ALA | A | 224 | 27.100 | 1.446 | 34.039 | 1.00 | 38.57 | O |
| ATOM | 1497 | N | PRO | A | 225 | 29.297 | 1.148 | 34.023 | 1.00 | 42.00 | N |
| ATOM | 1498 | CD | PRO | A | 225 | 30.639 | 1.696 | 34.266 | 1.00 | 47.49 | C |
| ATOM | 1499 | CA | PRO | A | 225 | 29.201 | -0.259 | 34.483 | 1.00 | 42.00 | C |
| ATOM | 1500 | CB | PRO | A | 225 | 30.669 | -0.597 | 34.796 | 1.00 | 47.49 | C |
| ATOM | 1501 | CG | PRO | A | 225 | 31.197 | 0.703 | 35.242 | 1.00 | 47.49 | C |
| ATOM | 1502 | C | PRO | A | 225 | 28.214 | -0.646 | 35.621 | 1.00 | 42.00 | C |
| ATOM | 1503 | O | PRO | A | 225 | 27.394 | -1.568 | 35.477 | 1.00 | 42.00 | O |
| ATOM | 1504 | N | GLU | A | 226 | 28.285 | 0.082 | 36.736 | 1.00 | 51.36 | N |
| ATOM | 1505 | CA | GLU | A | 226 | 27.485 | -0.162 | 37.935 | 1.00 | 51.36 | C |
| ATOM | 1506 | CB | GLU | A | 226 | 27.946 | 0.814 | 39.010 | 1.00 | 45.00 | C |
| ATOM | 1507 | CG | GLU | A | 226 | 29.229 | 1.592 | 38.594 | 1.00 | 45.00 | C |

| | | | | | | | | | | | |
|---|---|---|---|---|---|---|---|---|---|---|---|
| ATOM | 1508 | CD | GLU | A | 226 | 28.952 | 3.030 | 38.154 | 1.00 | 45.00 | C |
| ATOM | 1509 | OE1 | GLU | A | 226 | 28.318 | 3.775 | 38.923 | 1.00 | 45.00 | O |
| ATOM | 1510 | OE2 | GLU | A | 226 | 29.374 | 3.421 | 37.055 | 1.00 | 45.00 | O |
| ATOM | 1511 | C | GLU | A | 226 | 25.995 | -0.036 | 37.687 | 1.00 | 51.36 | C |
| ATOM | 1512 | O | GLU | A | 226 | 25.201 | -0.529 | 38.470 | 1.00 | 51.36 | O |
| ATOM | 1513 | N | LEU | A | 227 | 25.615 | 0.611 | 36.592 | 1.00 | 44.44 | N |
| ATOM | 1514 | CA | LEU | A | 227 | 24.204 | 0.753 | 36.271 | 1.00 | 44.44 | C |
| ATOM | 1515 | CB | LEU | A | 227 | 24.004 | 1.893 | 35.282 | 1.00 | 59.63 | C |
| ATOM | 1516 | CG | LEU | A | 227 | 23.493 | 3.128 | 36.011 | 1.00 | 59.63 | C |
| ATOM | 1517 | CD1 | LEU | A | 227 | 23.997 | 4.382 | 35.341 | 1.00 | 59.63 | C |
| ATOM | 1518 | CD2 | LEU | A | 227 | 21.980 | 3.072 | 36.043 | 1.00 | 59.63 | C |
| ATOM | 1519 | C | LEU | A | 227 | 23.684 | -0.552 | 35.697 | 1.00 | 44.44 | C |
| ATOM | 1520 | O | LEU | A | 227 | 22.522 | -0.910 | 35.903 | 1.00 | 44.44 | O |
| ATOM | 1521 | N | ALA | A | 228 | 24.553 | -1.263 | 34.980 | 1.00 | 63.12 | N |
| ATOM | 1522 | CA | ALA | A | 228 | 24.188 | -2.549 | 34.393 | 1.00 | 63.12 | C |
| ATOM | 1523 | CB | ALA | A | 228 | 25.182 | -2.941 | 33.287 | 1.00 | 32.33 | C |
| ATOM | 1524 | C | ALA | A | 228 | 24.227 | -3.568 | 35.526 | 1.00 | 63.12 | C |
| ATOM | 1525 | O | ALA | A | 228 | 23.649 | -4.653 | 35.438 | 1.00 | 63.12 | O |
| ATOM | 1526 | N | PHE | A | 229 | 24.922 | -3.197 | 36.595 | 1.00 | 41.64 | N |
| ATOM | 1527 | CA | PHE | A | 229 | 25.044 | -4.046 | 37.765 | 1.00 | 41.64 | C |
| ATOM | 1528 | CB | PHE | A | 229 | 26.388 | -3.816 | 38.451 | 1.00 | 53.25 | C |
| ATOM | 1529 | CG | PHE | A | 229 | 27.513 | -4.629 | 37.874 | 1.00 | 53.25 | C |
| ATOM | 1530 | CD1 | PHE | A | 229 | 27.396 | -6.006 | 37.747 | 1.00 | 53.25 | C |
| ATOM | 1531 | CD2 | PHE | A | 229 | 28.706 | -4.025 | 37.494 | 1.00 | 53.25 | C |
| ATOM | 1532 | CE1 | PHE | A | 229 | 28.450 | -6.767 | 37.260 | 1.00 | 53.25 | C |
| ATOM | 1533 | CE2 | PHE | A | 229 | 29.765 | -4.783 | 37.007 | 1.00 | 53.25 | C |
| ATOM | 1534 | CZ | PHE | A | 229 | 29.634 | -6.156 | 36.890 | 1.00 | 53.25 | C |
| ATOM | 1535 | C | PHE | A | 229 | 23.913 | -3.781 | 38.745 | 1.00 | 41.64 | C |
| ATOM | 1536 | O | PHE | A | 229 | 23.998 | -4.159 | 39.909 | 1.00 | 41.64 | O |
| ATOM | 1537 | N | GLY | A | 230 | 22.870 | -3.108 | 38.265 | 1.00 | 50.34 | N |
| ATOM | 1538 | CA | GLY | A | 230 | 21.701 | -2.824 | 39.081 | 1.00 | 50.34 | C |
| ATOM | 1539 | C | GLY | A | 230 | 21.842 | -1.924 | 40.294 | 1.00 | 50.34 | C |
| ATOM | 1540 | O | GLY | A | 230 | 20.929 | -1.884 | 41.116 | 1.00 | 50.34 | O |
| ATOM | 1541 | N | ALA | A | 231 | 22.965 | -1.218 | 40.425 | 1.00 | 34.19 | N |
| ATOM | 1542 | CA | ALA | A | 231 | 23.157 | -0.306 | 41.559 | 1.00 | 34.19 | C |
| ATOM | 1543 | CB | ALA | A | 231 | 24.559 | 0.310 | 41.527 | 1.00 | 22.48 | C |
| ATOM | 1544 | C | ALA | A | 231 | 22.102 | 0.803 | 41.501 | 1.00 | 34.19 | C |
| ATOM | 1545 | O | ALA | A | 231 | 21.911 | 1.431 | 40.459 | 1.00 | 34.19 | O |
| ATOM | 1546 | N | THR | A | 232 | 21.412 | 1.037 | 42.614 | 1.00 | 44.97 | N |
| ATOM | 1547 | CA | THR | A | 232 | 20.388 | 2.078 | 42.646 | 1.00 | 44.97 | C |
| ATOM | 1548 | CB | THR | A | 232 | 19.042 | 1.545 | 43.163 | 1.00 | 52.29 | C |
| ATOM | 1549 | OG1 | THR | A | 232 | 19.224 | 0.972 | 44.465 | 1.00 | 52.29 | O |
| ATOM | 1550 | CG2 | THR | A | 232 | 18.479 | 0.510 | 42.199 | 1.00 | 52.29 | C |
| ATOM | 1551 | C | THR | A | 232 | 20.828 | 3.230 | 43.533 | 1.00 | 44.97 | C |
| ATOM | 1552 | O | THR | A | 232 | 20.124 | 4.229 | 43.673 | 1.00 | 44.97 | O |
| ATOM | 1553 | N | ASP | A | 233 | 22.010 | 3.085 | 44.117 | 1.00 | 35.44 | N |
| ATOM | 1554 | CA | ASP | A | 233 | 22.548 | 4.109 | 44.986 | 1.00 | 35.44 | C |
| ATOM | 1555 | CB | ASP | A | 233 | 22.815 | 3.524 | 46.371 | 1.00 | 47.23 | C |
| ATOM | 1556 | CG | ASP | A | 233 | 22.460 | 4.479 | 47.488 | 1.00 | 47.23 | C |
| ATOM | 1557 | OD1 | ASP | A | 233 | 22.777 | 4.153 | 48.654 | 1.00 | 47.23 | O |
| ATOM | 1558 | OD2 | ASP | A | 233 | 21.857 | 5.540 | 47.206 | 1.00 | 47.23 | O |
| ATOM | 1559 | C | ASP | A | 233 | 23.846 | 4.628 | 44.390 | 1.00 | 35.44 | C |
| ATOM | 1560 | O | ASP | A | 233 | 24.865 | 4.683 | 45.084 | 1.00 | 35.44 | O |
| ATOM | 1561 | N | TYR | A | 234 | 23.811 | 5.002 | 43.109 | 1.00 | 33.77 | N |
| ATOM | 1562 | CA | TYR | A | 234 | 24.999 | 5.525 | 42.417 | 1.00 | 33.77 | C |
| ATOM | 1563 | CB | TYR | A | 234 | 24.968 | 5.122 | 40.939 | 1.00 | 21.49 | C |
| ATOM | 1564 | CG | TYR | A | 234 | 23.659 | 5.426 | 40.240 | 1.00 | 21.49 | C |
| ATOM | 1565 | CD1 | TYR | A | 234 | 23.412 | 6.680 | 39.680 | 1.00 | 21.49 | C |
| ATOM | 1566 | CE1 | TYR | A | 234 | 22.172 | 6.965 | 39.071 | 1.00 | 21.49 | C |
| ATOM | 1567 | CD2 | TYR | A | 234 | 22.645 | 4.469 | 40.176 | 1.00 | 21.49 | C |
| ATOM | 1568 | CE2 | TYR | A | 234 | 21.417 | 4.748 | 39.572 | 1.00 | 21.49 | C |
| ATOM | 1569 | CZ | TYR | A | 234 | 21.194 | 5.982 | 39.028 | 1.00 | 21.49 | C |
| ATOM | 1570 | OH | TYR | A | 234 | 20.005 | 6.209 | 38.418 | 1.00 | 21.49 | O |

| | | | | | | | | | | | |
|---|---|---|---|---|---|---|---|---|---|---|---|
| ATOM | 1571 | C | TYR | A | 234 | 25.137 | 7.045 | 42.540 | 1.00 | 33.77 | C |
| ATOM | 1572 | O | TYR | A | 234 | 24.223 | 7.723 | 42.990 | 1.00 | 33.77 | O |
| ATOM | 1573 | N | THR | A | 235 | 26.286 | 7.573 | 42.136 | 1.00 | 31.78 | N |
| ATOM | 1574 | CA | THR | A | 235 | 26.552 | 9.008 | 42.218 | 1.00 | 31.78 | C |
| ATOM | 1575 | CB | THR | A | 235 | 27.905 | 9.279 | 42.876 | 1.00 | 44.33 | C |
| ATOM | 1576 | OG1 | THR | A | 235 | 28.953 | 8.893 | 41.981 | 1.00 | 44.33 | O |
| ATOM | 1577 | CG2 | THR | A | 235 | 28.042 | 8.479 | 44.156 | 1.00 | 44.33 | C |
| ATOM | 1578 | C | THR | A | 235 | 26.569 | 9.662 | 40.844 | 1.00 | 31.78 | C |
| ATOM | 1579 | O | THR | A | 235 | 26.435 | 8.989 | 39.819 | 1.00 | 31.78 | O |
| ATOM | 1580 | N | SER | A | 236 | 26.741 | 10.977 | 40.820 | 1.00 | 38.26 | N |
| ATOM | 1581 | CA | SER | A | 236 | 26.759 | 11.700 | 39.552 | 1.00 | 38.26 | C |
| ATOM | 1582 | CB | SER | A | 236 | 26.764 | 13.205 | 39.794 | 1.00 | 41.33 | C |
| ATOM | 1583 | OG | SER | A | 236 | 27.940 | 13.576 | 40.475 | 1.00 | 41.33 | O |
| ATOM | 1584 | C | SER | A | 236 | 27.976 | 11.320 | 38.718 | 1.00 | 38.26 | C |
| ATOM | 1585 | O | SER | A | 236 | 28.081 | 11.682 | 37.537 | 1.00 | 38.26 | O |
| ATOM | 1586 | N | SER | A | 237 | 28.896 | 10.587 | 39.336 | 1.00 | 49.94 | N |
| ATOM | 1587 | CA | SER | A | 237 | 30.108 | 10.157 | 38.653 | 1.00 | 49.94 | C |
| ATOM | 1588 | CB | SER | A | 237 | 31.006 | 9.351 | 39.606 | 1.00 | 47.98 | C |
| ATOM | 1589 | OG | SER | A | 237 | 30.382 | 8.159 | 40.056 | 1.00 | 47.98 | O |
| ATOM | 1590 | C | SER | A | 237 | 29.780 | 9.325 | 37.416 | 1.00 | 49.94 | C |
| ATOM | 1591 | O | SER | A | 237 | 30.622 | 9.148 | 36.536 | 1.00 | 49.94 | O |
| ATOM | 1592 | N | ILE | A | 238 | 28.553 | 8.825 | 37.338 | 1.00 | 34.32 | N |
| ATOM | 1593 | CA | ILE | A | 238 | 28.161 | 8.015 | 36.195 | 1.00 | 34.32 | C |
| ATOM | 1594 | CB | ILE | A | 238 | 26.755 | 7.386 | 36.391 | 1.00 | 19.16 | C |
| ATOM | 1595 | CG2 | ILE | A | 238 | 26.717 | 6.605 | 37.702 | 1.00 | 19.16 | C |
| ATOM | 1596 | CG1 | ILE | A | 238 | 25.679 | 8.470 | 36.373 | 1.00 | 19.16 | C |
| ATOM | 1597 | CD1 | ILE | A | 238 | 24.269 | 7.916 | 36.462 | 1.00 | 19.16 | C |
| ATOM | 1598 | C | ILE | A | 238 | 28.192 | 8.829 | 34.905 | 1.00 | 34.32 | C |
| ATOM | 1599 | O | ILE | A | 238 | 28.286 | 8.268 | 33.813 | 1.00 | 34.32 | O |
| ATOM | 1600 | N | ASP | A | 239 | 28.105 | 10.151 | 35.035 | 1.00 | 26.01 | N |
| ATOM | 1601 | CA | ASP | A | 239 | 28.158 | 11.046 | 33.879 | 1.00 | 26.01 | C |
| ATOM | 1602 | CB | ASP | A | 239 | 27.679 | 12.465 | 34.274 | 1.00 | 32.48 | C |
| ATOM | 1603 | CG | ASP | A | 239 | 26.166 | 12.549 | 34.483 | 1.00 | 32.48 | C |
| ATOM | 1604 | OD1 | ASP | A | 239 | 25.456 | 11.613 | 34.076 | 1.00 | 32.48 | O |
| ATOM | 1605 | OD2 | ASP | A | 239 | 25.674 | 13.552 | 35.036 | 1.00 | 32.48 | O |
| ATOM | 1606 | C | ASP | A | 239 | 29.603 | 11.111 | 33.333 | 1.00 | 26.01 | C |
| ATOM | 1607 | O | ASP | A | 239 | 29.826 | 11.327 | 32.138 | 1.00 | 26.01 | O |
| ATOM | 1608 | N | VAL | A | 240 | 30.570 | 10.903 | 34.224 | 1.00 | 20.88 | N |
| ATOM | 1609 | CA | VAL | A | 240 | 31.973 | 10.960 | 33.865 | 1.00 | 20.88 | C |
| ATOM | 1610 | CB | VAL | A | 240 | 32.881 | 10.895 | 35.098 | 1.00 | 18.97 | C |
| ATOM | 1611 | CG1 | VAL | A | 240 | 34.332 | 11.204 | 34.699 | 1.00 | 18.97 | C |
| ATOM | 1612 | CG2 | VAL | A | 240 | 32.392 | 11.880 | 36.143 | 1.00 | 18.97 | C |
| ATOM | 1613 | C | VAL | A | 240 | 32.321 | 9.810 | 32.963 | 1.00 | 20.88 | C |
| ATOM | 1614 | O | VAL | A | 240 | 33.127 | 9.966 | 32.034 | 1.00 | 20.88 | O |
| ATOM | 1615 | N | TRP | A | 241 | 31.715 | 8.655 | 33.239 | 1.00 | 32.86 | N |
| ATOM | 1616 | CA | TRP | A | 241 | 31.940 | 7.453 | 32.438 | 1.00 | 32.86 | C |
| ATOM | 1617 | CB | TRP | A | 241 | 31.270 | 6.239 | 33.095 | 1.00 | 34.44 | C |
| ATOM | 1618 | CG | TRP | A | 241 | 31.285 | 4.985 | 32.248 | 1.00 | 34.44 | C |
| ATOM | 1619 | CD2 | TRP | A | 241 | 32.271 | 3.938 | 32.279 | 1.00 | 34.44 | C |
| ATOM | 1620 | CE2 | TRP | A | 241 | 31.880 | 2.973 | 31.308 | 1.00 | 34.44 | C |
| ATOM | 1621 | CE3 | TRP | A | 241 | 33.425 | 3.709 | 33.046 | 1.00 | 34.44 | C |
| ATOM | 1622 | CD1 | TRP | A | 241 | 30.382 | 4.633 | 31.277 | 1.00 | 34.44 | C |
| ATOM | 1623 | NE1 | TRP | A | 241 | 30.737 | 3.425 | 30.711 | 1.00 | 34.44 | N |
| ATOM | 1624 | CZ2 | TRP | A | 241 | 32.633 | 1.813 | 31.068 | 1.00 | 34.44 | C |
| ATOM | 1625 | CZ3 | TRP | A | 241 | 34.166 | 2.557 | 32.805 | 1.00 | 34.44 | C |
| ATOM | 1626 | CH2 | TRP | A | 241 | 33.757 | 1.615 | 31.831 | 1.00 | 34.44 | C |
| ATOM | 1627 | C | TRP | A | 241 | 31.397 | 7.649 | 31.022 | 1.00 | 32.86 | C |
| ATOM | 1628 | O | TRP | A | 241 | 32.036 | 7.278 | 30.032 | 1.00 | 32.86 | O |
| ATOM | 1629 | N | SER | A | 242 | 30.214 | 8.239 | 30.926 | 1.00 | 40.81 | N |
| ATOM | 1630 | CA | SER | A | 242 | 29.618 | 8.489 | 29.626 | 1.00 | 40.81 | C |
| ATOM | 1631 | CB | SER | A | 242 | 28.212 | 9.055 | 29.793 | 1.00 | 35.24 | C |
| ATOM | 1632 | OG | SER | A | 242 | 27.443 | 8.218 | 30.629 | 1.00 | 35.24 | O |
| ATOM | 1633 | C | SER | A | 242 | 30.486 | 9.490 | 28.876 | 1.00 | 40.81 | C |

| | | | | | | | | | | | |
|---|---|---|---|---|---|---|---|---|---|---|---|
| ATOM | 1634 | O | SER | A | 242 | 30.619 | 9.409 | 27.649 | 1.00 | 40.81 | O |
| ATOM | 1635 | N | ALA | A | 243 | 31.063 | 10.438 | 29.620 | 1.00 | 25.31 | N |
| ATOM | 1636 | CA | ALA | A | 243 | 31.927 | 11.455 | 29.034 | 1.00 | 25.31 | C |
| ATOM | 1637 | CB | ALA | A | 243 | 32.391 | 12.432 | 30.107 | 1.00 | 49.37 | C |
| ATOM | 1638 | C | ALA | A | 243 | 33.112 | 10.717 | 28.436 | 1.00 | 25.31 | C |
| ATOM | 1639 | O | ALA | A | 243 | 33.537 | 10.991 | 27.312 | 1.00 | 25.31 | O |
| ATOM | 1640 | N | GLY | A | 244 | 33.620 | 9.753 | 29.197 | 1.00 | 28.18 | N |
| ATOM | 1641 | CA | GLY | A | 244 | 34.750 | 8.963 | 28.748 | 1.00 | 28.18 | C |
| ATOM | 1642 | C | GLY | A | 244 | 34.395 | 8.194 | 27.500 | 1.00 | 28.18 | C |
| ATOM | 1643 | O | GLY | A | 244 | 35.214 | 8.033 | 26.582 | 1.00 | 28.18 | O |
| ATOM | 1644 | N | CYS | A | 245 | 33.157 | 7.720 | 27.465 | 1.00 | 38.39 | N |
| ATOM | 1645 | CA | CYS | A | 245 | 32.680 | 6.967 | 26.321 | 1.00 | 38.39 | C |
| ATOM | 1646 | CB | CYS | A | 245 | 31.280 | 6.427 | 26.573 | 1.00 | 29.10 | C |
| ATOM | 1647 | SG | CYS | A | 245 | 31.200 | 5.180 | 27.838 | 1.00 | 29.10 | S |
| ATOM | 1648 | C | CYS | A | 245 | 32.658 | 7.863 | 25.108 | 1.00 | 38.39 | C |
| ATOM | 1649 | O | CYS | A | 245 | 32.999 | 7.427 | 24.020 | 1.00 | 38.39 | O |
| ATOM | 1650 | N | VAL | A | 246 | 32.254 | 9.115 | 25.297 | 1.00 | 42.15 | N |
| ATOM | 1651 | CA | VAL | A | 246 | 32.197 | 10.064 | 24.195 | 1.00 | 42.15 | C |
| ATOM | 1652 | CB | VAL | A | 246 | 31.480 | 11.384 | 24.620 | 1.00 | 22.39 | C |
| ATOM | 1653 | CG1 | VAL | A | 246 | 31.423 | 12.360 | 23.441 | 1.00 | 22.39 | C |
| ATOM | 1654 | CG2 | VAL | A | 246 | 30.063 | 11.072 | 25.115 | 1.00 | 22.39 | C |
| ATOM | 1655 | C | VAL | A | 246 | 33.613 | 10.371 | 23.707 | 1.00 | 42.15 | C |
| ATOM | 1656 | O | VAL | A | 246 | 33.883 | 10.332 | 22.504 | 1.00 | 42.15 | O |
| ATOM | 1657 | N | LEU | A | 247 | 34.516 | 10.670 | 24.640 | 1.00 | 38.53 | N |
| ATOM | 1658 | CA | LEU | A | 247 | 35.898 | 10.970 | 24.276 | 1.00 | 38.53 | C |
| ATOM | 1659 | CB | LEU | A | 247 | 36.771 | 11.180 | 25.516 | 1.00 | 30.05 | C |
| ATOM | 1660 | CG | LEU | A | 247 | 38.271 | 11.205 | 25.181 | 1.00 | 30.05 | C |
| ATOM | 1661 | CD1 | LEU | A | 247 | 38.582 | 12.449 | 24.346 | 1.00 | 30.05 | C |
| ATOM | 1662 | CD2 | LEU | A | 247 | 39.096 | 11.187 | 26.444 | 1.00 | 30.05 | C |
| ATOM | 1663 | C | LEU | A | 247 | 36.467 | 9.806 | 23.485 | 1.00 | 38.53 | C |
| ATOM | 1664 | O | LEU | A | 247 | 37.105 | 9.993 | 22.446 | 1.00 | 38.53 | O |
| ATOM | 1665 | N | ALA | A | 248 | 36.250 | 8.598 | 23.999 | 1.00 | 32.57 | N |
| ATOM | 1666 | CA | ALA | A | 248 | 36.738 | 7.403 | 23.330 | 1.00 | 32.57 | C |
| ATOM | 1667 | CB | ALA | A | 248 | 36.273 | 6.173 | 24.081 | 1.00 | 27.10 | C |
| ATOM | 1668 | C | ALA | A | 248 | 36.202 | 7.380 | 21.900 | 1.00 | 32.57 | C |
| ATOM | 1669 | O | ALA | A | 248 | 36.956 | 7.261 | 20.931 | 1.00 | 32.57 | O |
| ATOM | 1670 | N | GLU | A | 249 | 34.886 | 7.509 | 21.793 | 1.00 | 45.84 | N |
| ATOM | 1671 | CA | GLU | A | 249 | 34.168 | 7.513 | 20.526 | 1.00 | 45.84 | C |
| ATOM | 1672 | CB | GLU | A | 249 | 32.676 | 7.687 | 20.813 | 1.00 | 35.09 | C |
| ATOM | 1673 | CG | GLU | A | 249 | 31.804 | 7.735 | 19.592 | 1.00 | 35.09 | C |
| ATOM | 1674 | CD | GLU | A | 249 | 30.376 | 7.417 | 19.919 | 1.00 | 35.09 | C |
| ATOM | 1675 | OE1 | GLU | A | 249 | 30.155 | 6.787 | 20.988 | 1.00 | 35.09 | O |
| ATOM | 1676 | OE2 | GLU | A | 249 | 29.494 | 7.778 | 19.099 | 1.00 | 35.09 | O |
| ATOM | 1677 | C | GLU | A | 249 | 34.647 | 8.588 | 19.543 | 1.00 | 45.84 | C |
| ATOM | 1678 | O | GLU | A | 249 | 34.514 | 8.430 | 18.331 | 1.00 | 45.84 | O |
| ATOM | 1679 | N | LEU | A | 250 | 35.186 | 9.690 | 20.055 | 1.00 | 34.77 | N |
| ATOM | 1680 | CA | LEU | A | 250 | 35.683 | 10.743 | 19.180 | 1.00 | 34.77 | C |
| ATOM | 1681 | CB | LEU | A | 250 | 35.823 | 12.050 | 19.943 | 1.00 | 36.13 | C |
| ATOM | 1682 | CG | LEU | A | 250 | 34.502 | 12.772 | 20.158 | 1.00 | 36.13 | C |
| ATOM | 1683 | CD1 | LEU | A | 250 | 34.725 | 13.951 | 21.087 | 1.00 | 36.13 | C |
| ATOM | 1684 | CD2 | LEU | A | 250 | 33.949 | 13.219 | 18.817 | 1.00 | 36.13 | C |
| ATOM | 1685 | C | LEU | A | 250 | 37.032 | 10.347 | 18.619 | 1.00 | 34.77 | C |
| ATOM | 1686 | O | LEU | A | 250 | 37.362 | 10.654 | 17.472 | 1.00 | 34.77 | O |
| ATOM | 1687 | N | LEU | A | 251 | 37.814 | 9.668 | 19.446 | 1.00 | 47.64 | N |
| ATOM | 1688 | CA | LEU | A | 251 | 39.133 | 9.209 | 19.052 | 1.00 | 47.64 | C |
| ATOM | 1689 | CB | LEU | A | 251 | 39.948 | 8.857 | 20.296 | 1.00 | 23.98 | C |
| ATOM | 1690 | CG | LEU | A | 251 | 40.337 | 10.062 | 21.135 | 1.00 | 23.98 | C |
| ATOM | 1691 | CD1 | LEU | A | 251 | 41.142 | 9.605 | 22.347 | 1.00 | 23.98 | C |
| ATOM | 1692 | CD2 | LEU | A | 251 | 41.134 | 11.026 | 20.255 | 1.00 | 23.98 | C |
| ATOM | 1693 | C | LEU | A | 251 | 39.027 | 7.985 | 18.155 | 1.00 | 47.64 | C |
| ATOM | 1694 | O | LEU | A | 251 | 39.900 | 7.739 | 17.314 | 1.00 | 47.64 | O |
| ATOM | 1695 | N | LEU | A | 252 | 37.948 | 7.230 | 18.338 | 1.00 | 46.58 | N |
| ATOM | 1696 | CA | LEU | A | 252 | 37.740 | 6.007 | 17.583 | 1.00 | 46.58 | C |

| | | | | | | | | | | | |
|---|---|---|---|---|---|---|---|---|---|---|---|
| ATOM | 1697 | CB | LEU | A | 252 | 37.070 | 4.942 | 18.452 | 1.00 | 46.31 | C |
| ATOM | 1698 | CG | LEU | A | 252 | 37.820 | 3.648 | 18.707 | 1.00 | 46.31 | C |
| ATOM | 1699 | CD1 | LEU | A | 252 | 38.946 | 3.923 | 19.622 | 1.00 | 46.31 | C |
| ATOM | 1700 | CD2 | LEU | A | 252 | 36.893 | 2.640 | 19.336 | 1.00 | 46.31 | C |
| ATOM | 1701 | C | LEU | A | 252 | 36.911 | 6.191 | 16.340 | 1.00 | 46.58 | C |
| ATOM | 1702 | O | LEU | A | 252 | 36.934 | 5.338 | 15.468 | 1.00 | 46.58 | O |
| ATOM | 1703 | N | GLY | A | 253 | 36.186 | 7.297 | 16.241 | 1.00 | 28.57 | N |
| ATOM | 1704 | CA | GLY | A | 253 | 35.361 | 7.499 | 15.065 | 1.00 | 28.57 | C |
| ATOM | 1705 | C | GLY | A | 253 | 34.173 | 6.562 | 15.122 | 1.00 | 28.57 | C |
| ATOM | 1706 | O | GLY | A | 253 | 33.582 | 6.232 | 14.098 | 1.00 | 28.57 | O |
| ATOM | 1707 | N | GLN | A | 254 | 33.838 | 6.125 | 16.335 | 1.00 | 27.85 | N |
| ATOM | 1708 | CA | GLN | A | 254 | 32.704 | 5.238 | 16.579 | 1.00 | 27.85 | C |
| ATOM | 1709 | CB | GLN | A | 254 | 32.716 | 4.087 | 15.596 | 1.00 | 49.54 | C |
| ATOM | 1710 | CG | GLN | A | 254 | 33.990 | 3.316 | 15.651 | 1.00 | 49.54 | C |
| ATOM | 1711 | CD | GLN | A | 254 | 33.788 | 1.888 | 15.237 | 1.00 | 49.54 | C |
| ATOM | 1712 | OE1 | GLN | A | 254 | 33.707 | 0.988 | 16.080 | 1.00 | 49.54 | O |
| ATOM | 1713 | NE2 | GLN | A | 254 | 33.684 | 1.664 | 13.929 | 1.00 | 49.54 | N |
| ATOM | 1714 | C | GLN | A | 254 | 32.683 | 4.674 | 18.005 | 1.00 | 27.85 | C |
| ATOM | 1715 | O | GLN | A | 254 | 33.724 | 4.504 | 18.649 | 1.00 | 27.85 | O |
| ATOM | 1716 | N | PRO | A | 255 | 31.481 | 4.362 | 18.503 | 1.00 | 29.97 | N |
| ATOM | 1717 | CD | PRO | A | 255 | 30.246 | 4.399 | 17.700 | 1.00 | 18.84 | C |
| ATOM | 1718 | CA | PRO | A | 255 | 31.224 | 3.812 | 19.839 | 1.00 | 29.97 | C |
| ATOM | 1719 | CB | PRO | A | 255 | 29.819 | 3.245 | 19.707 | 1.00 | 18.84 | C |
| ATOM | 1720 | CG | PRO | A | 255 | 29.176 | 4.193 | 18.732 | 1.00 | 18.84 | C |
| ATOM | 1721 | C | PRO | A | 255 | 32.222 | 2.739 | 20.242 | 1.00 | 29.97 | C |
| ATOM | 1722 | O | PRO | A | 255 | 32.461 | 1.798 | 19.490 | 1.00 | 29.97 | O |
| ATOM | 1723 | N | ILE | A | 256 | 32.782 | 2.877 | 21.440 | 1.00 | 42.67 | N |
| ATOM | 1724 | CA | ILE | A | 256 | 33.759 | 1.925 | 21.971 | 1.00 | 42.67 | C |
| ATOM | 1725 | CB | ILE | A | 256 | 34.675 | 2.622 | 22.983 | 1.00 | 27.42 | C |
| ATOM | 1726 | CG2 | ILE | A | 256 | 33.850 | 3.159 | 24.121 | 1.00 | 27.42 | C |
| ATOM | 1727 | CG1 | ILE | A | 256 | 35.713 | 1.652 | 23.522 | 1.00 | 27.42 | C |
| ATOM | 1728 | CD1 | ILE | A | 256 | 36.742 | 2.318 | 24.398 | 1.00 | 27.42 | C |
| ATOM | 1729 | C | ILE | A | 256 | 33.101 | 0.721 | 22.649 | 1.00 | 42.67 | C |
| ATOM | 1730 | O | ILE | A | 256 | 33.695 | -0.348 | 22.741 | 1.00 | 42.67 | O |
| ATOM | 1731 | N | PHE | A | 257 | 31.879 | 0.905 | 23.134 | 1.00 | 49.60 | N |
| ATOM | 1732 | CA | PHE | A | 257 | 31.145 | -0.171 | 23.792 | 1.00 | 49.60 | C |
| ATOM | 1733 | CB | PHE | A | 257 | 30.934 | 0.136 | 25.281 | 1.00 | 31.85 | C |
| ATOM | 1734 | CG | PHE | A | 257 | 32.221 | 0.304 | 26.062 | 1.00 | 31.85 | C |
| ATOM | 1735 | CD1 | PHE | A | 257 | 33.280 | -0.592 | 25.895 | 1.00 | 31.85 | C |
| ATOM | 1736 | CD2 | PHE | A | 257 | 32.376 | 1.364 | 26.961 | 1.00 | 31.85 | C |
| ATOM | 1737 | CE1 | PHE | A | 257 | 34.473 | -0.435 | 26.605 | 1.00 | 31.85 | C |
| ATOM | 1738 | CE2 | PHE | A | 257 | 33.565 | 1.526 | 27.676 | 1.00 | 31.85 | C |
| ATOM | 1739 | CZ | PHE | A | 257 | 34.618 | 0.621 | 27.494 | 1.00 | 31.85 | C |
| ATOM | 1740 | C | PHE | A | 257 | 29.807 | -0.295 | 23.090 | 1.00 | 49.60 | C |
| ATOM | 1741 | O | PHE | A | 257 | 28.760 | 0.047 | 23.639 | 1.00 | 49.60 | O |
| ATOM | 1742 | N | PRO | A | 258 | 29.833 | -0.785 | 21.850 | 1.00 | 74.41 | N |
| ATOM | 1743 | CD | PRO | A | 258 | 31.056 | -1.288 | 21.203 | 1.00 | 59.92 | C |
| ATOM | 1744 | CA | PRO | A | 258 | 28.664 | -0.984 | 20.993 | 1.00 | 74.41 | C |
| ATOM | 1745 | CB | PRO | A | 258 | 29.287 | -1.196 | 19.626 | 1.00 | 59.92 | C |
| ATOM | 1746 | CG | PRO | A | 258 | 30.517 | -1.961 | 19.963 | 1.00 | 59.92 | C |
| ATOM | 1747 | C | PRO | A | 258 | 27.841 | -2.174 | 21.460 | 1.00 | 74.41 | C |
| ATOM | 1748 | O | PRO | A | 258 | 27.191 | -2.854 | 20.659 | 1.00 | 74.41 | O |
| ATOM | 1749 | N | GLY | A | 259 | 27.884 | -2.403 | 22.773 | 1.00 | 41.04 | N |
| ATOM | 1750 | CA | GLY | A | 259 | 27.152 | -3.496 | 23.388 | 1.00 | 41.04 | C |
| ATOM | 1751 | C | GLY | A | 259 | 25.715 | -3.584 | 22.913 | 1.00 | 41.04 | C |
| ATOM | 1752 | O | GLY | A | 259 | 25.065 | -2.559 | 22.699 | 1.00 | 41.04 | O |
| ATOM | 1753 | N | ASP | A | 260 | 25.213 | -4.810 | 22.765 | 1.00 | 78.48 | N |
| ATOM | 1754 | CA | ASP | A | 260 | 23.853 | -5.011 | 22.283 | 1.00 | 78.48 | C |
| ATOM | 1755 | CB | ASP | A | 260 | 23.795 | -6.235 | 21.338 | 1.00 | 81.48 | C |
| ATOM | 1756 | CG | ASP | A | 260 | 23.597 | -7.569 | 22.068 | 1.00 | 81.48 | C |
| ATOM | 1757 | OD1 | ASP | A | 260 | 23.030 | -8.497 | 21.449 | 1.00 | 81.48 | O |
| ATOM | 1758 | OD2 | ASP | A | 260 | 24.008 | -7.711 | 23.239 | 1.00 | 81.48 | O |
| ATOM | 1759 | C | ASP | A | 260 | 22.734 | -5.101 | 23.328 | 1.00 | 78.48 | C |

| | | | | | | | | | | | |
|---|---|---|---|---|---|---|---|---|---|---|---|
| ATOM | 1760 | O | ASP | A | 260 | 21.597 | -4.709 | 23.046 | 1.00 | 78.48 | O |
| ATOM | 1761 | N | SER | A | 261 | 23.048 | -5.588 | 24.527 | 1.00 | 63.04 | N |
| ATOM | 1762 | CA | SER | A | 261 | 22.040 | -5.732 | 25.572 | 1.00 | 63.04 | C |
| ATOM | 1763 | CB | SER | A | 261 | 21.181 | -6.971 | 25.285 | 1.00 | 76.61 | C |
| ATOM | 1764 | OG | SER | A | 261 | 20.145 | -7.131 | 26.237 | 1.00 | 76.61 | O |
| ATOM | 1765 | C | SER | A | 261 | 22.777 | -5.902 | 26.881 | 1.00 | 63.04 | C |
| ATOM | 1766 | O | SER | A | 261 | 23.735 | -6.664 | 26.936 | 1.00 | 63.04 | O |
| ATOM | 1767 | N | GLY | A | 262 | 22.337 | -5.188 | 27.917 | 1.00 | 55.63 | N |
| ATOM | 1768 | CA | GLY | A | 262 | 22.973 | -5.274 | 29.225 | 1.00 | 55.63 | C |
| ATOM | 1769 | C | GLY | A | 262 | 23.988 | -6.394 | 29.366 | 1.00 | 55.63 | C |
| ATOM | 1770 | O | GLY | A | 262 | 25.101 | -6.171 | 29.860 | 1.00 | 55.63 | O |
| ATOM | 1771 | N | GLY | A | 263 | 23.593 | -7.599 | 28.946 | 1.00 | 46.75 | N |
| ATOM | 1772 | CA | GLY | A | 263 | 24.486 | -8.742 | 29.001 | 1.00 | 46.75 | C |
| ATOM | 1773 | C | GLY | A | 263 | 25.741 | -8.500 | 28.178 | 1.00 | 46.75 | C |
| ATOM | 1774 | O | GLY | A | 263 | 26.845 | -8.737 | 28.653 | 1.00 | 46.75 | O |
| ATOM | 1775 | N | ASP | A | 264 | 25.597 | -8.011 | 26.951 | 1.00 | 72.15 | N |
| ATOM | 1776 | CA | ASP | A | 264 | 26.776 | -7.779 | 26.131 | 1.00 | 72.15 | C |
| ATOM | 1777 | CB | ASP | A | 264 | 26.423 | -7.812 | 24.638 | 1.00 | 79.37 | C |
| ATOM | 1778 | CG | ASP | A | 264 | 27.617 | -8.174 | 23.766 | 1.00 | 79.37 | C |
| ATOM | 1779 | OD1 | ASP | A | 264 | 27.640 | -7.776 | 22.583 | 1.00 | 79.37 | O |
| ATOM | 1780 | OD2 | ASP | A | 264 | 28.531 | -8.865 | 24.263 | 1.00 | 79.37 | O |
| ATOM | 1781 | C | ASP | A | 264 | 27.462 | -6.459 | 26.464 | 1.00 | 72.15 | C |
| ATOM | 1782 | O | ASP | A | 264 | 28.677 | -6.328 | 26.280 | 1.00 | 72.15 | O |
| ATOM | 1783 | N | GLN | A | 265 | 26.702 | -5.481 | 26.952 | 1.00 | 44.20 | N |
| ATOM | 1784 | CA | GLN | A | 265 | 27.304 | -4.199 | 27.283 | 1.00 | 44.20 | C |
| ATOM | 1785 | CB | GLN | A | 265 | 26.255 | -3.253 | 27.884 | 1.00 | 38.28 | C |
| ATOM | 1786 | CG | GLN | A | 265 | 26.694 | -1.779 | 28.080 | 1.00 | 38.28 | C |
| ATOM | 1787 | CD | GLN | A | 265 | 27.405 | -1.182 | 26.871 | 1.00 | 38.28 | C |
| ATOM | 1788 | OE1 | GLN | A | 265 | 26.959 | -1.330 | 25.733 | 1.00 | 38.28 | O |
| ATOM | 1789 | NE2 | GLN | A | 265 | 28.510 | -0.491 | 27.121 | 1.00 | 38.28 | N |
| ATOM | 1790 | C | GLN | A | 265 | 28.476 | -4.419 | 28.246 | 1.00 | 44.20 | C |
| ATOM | 1791 | O | GLN | A | 265 | 29.580 | -3.912 | 28.014 | 1.00 | 44.20 | O |
| ATOM | 1792 | N | LEU | A | 266 | 28.260 | -5.196 | 29.306 | 1.00 | 39.57 | N |
| ATOM | 1793 | CA | LEU | A | 266 | 29.342 | -5.450 | 30.255 | 1.00 | 39.57 | C |
| ATOM | 1794 | CB | LEU | A | 266 | 28.827 | -6.131 | 31.528 | 1.00 | 46.71 | C |
| ATOM | 1795 | CG | LEU | A | 266 | 29.945 | -6.504 | 32.519 | 1.00 | 46.71 | C |
| ATOM | 1796 | CD1 | LEU | A | 266 | 30.777 | -5.269 | 32.872 | 1.00 | 46.71 | C |
| ATOM | 1797 | CD2 | LEU | A | 266 | 29.340 | -7.106 | 33.769 | 1.00 | 46.71 | C |
| ATOM | 1798 | C | LEU | A | 266 | 30.477 | -6.286 | 29.666 | 1.00 | 39.57 | C |
| ATOM | 1799 | O | LEU | A | 266 | 31.613 | -6.224 | 30.146 | 1.00 | 39.57 | O |
| ATOM | 1800 | N | VAL | A | 267 | 30.188 | -7.087 | 28.649 | 1.00 | 46.20 | N |
| ATOM | 1801 | CA | VAL | A | 267 | 31.257 | -7.872 | 28.054 | 1.00 | 46.20 | C |
| ATOM | 1802 | CB | VAL | A | 267 | 30.710 | -8.857 | 27.013 | 1.00 | 60.14 | C |
| ATOM | 1803 | CG1 | VAL | A | 267 | 31.579 | -8.859 | 25.770 | 1.00 | 60.14 | C |
| ATOM | 1804 | CG2 | VAL | A | 267 | 30.687 | -10.245 | 27.611 | 1.00 | 60.14 | C |
| ATOM | 1805 | C | VAL | A | 267 | 32.242 | -6.910 | 27.409 | 1.00 | 46.20 | C |
| ATOM | 1806 | O | VAL | A | 267 | 33.432 | -6.871 | 27.756 | 1.00 | 46.20 | O |
| ATOM | 1807 | N | GLU | A | 268 | 31.714 | -6.115 | 26.486 | 1.00 | 41.45 | N |
| ATOM | 1808 | CA | GLU | A | 268 | 32.493 | -5.111 | 25.787 | 1.00 | 41.45 | C |
| ATOM | 1809 | CB | GLU | A | 268 | 31.547 | -4.099 | 25.132 | 1.00 | 51.39 | C |
| ATOM | 1810 | CG | GLU | A | 268 | 31.051 | -4.526 | 23.770 | 1.00 | 51.39 | C |
| ATOM | 1811 | CD | GLU | A | 268 | 32.135 | -4.413 | 22.732 | 1.00 | 51.39 | C |
| ATOM | 1812 | OE1 | GLU | A | 268 | 33.292 | -4.133 | 23.105 | 1.00 | 51.39 | O |
| ATOM | 1813 | OE2 | GLU | A | 268 | 31.836 | -4.604 | 21.542 | 1.00 | 51.39 | O |
| ATOM | 1814 | C | GLU | A | 268 | 33.444 | -4.389 | 26.737 | 1.00 | 41.45 | C |
| ATOM | 1815 | O | GLU | A | 268 | 34.624 | -4.220 | 26.444 | 1.00 | 41.45 | O |
| ATOM | 1816 | N | ILE | A | 269 | 32.927 | -3.985 | 27.887 | 1.00 | 40.82 | N |
| ATOM | 1817 | CA | ILE | A | 269 | 33.727 | -3.261 | 28.858 | 1.00 | 40.82 | C |
| ATOM | 1818 | CB | ILE | A | 269 | 32.820 | -2.724 | 29.979 | 1.00 | 42.65 | C |
| ATOM | 1819 | CG2 | ILE | A | 269 | 33.537 | -1.631 | 30.783 | 1.00 | 42.65 | C |
| ATOM | 1820 | CG1 | ILE | A | 269 | 31.544 | -2.175 | 29.333 | 1.00 | 42.65 | C |
| ATOM | 1821 | CD1 | ILE | A | 269 | 30.835 | -1.137 | 30.126 | 1.00 | 42.65 | C |
| ATOM | 1822 | C | ILE | A | 269 | 34.843 | -4.126 | 29.429 | 1.00 | 40.82 | C |

| | | | | | | | | | | | |
|---|---|---|---|---|---|---|---|---|---|---|---|
| ATOM | 1823 | O | ILE | A | 269 | 36.006 | -3.690 | 29.544 | 1.00 | 40.82 | O |
| ATOM | 1824 | N | ILE | A | 270 | 34.496 | -5.363 | 29.766 | 1.00 | 57.32 | N |
| ATOM | 1825 | CA | ILE | A | 270 | 35.473 | -6.279 | 30.321 | 1.00 | 57.32 | C |
| ATOM | 1826 | CB | ILE | A | 270 | 34.760 | -7.489 | 30.997 | 1.00 | 69.21 | C |
| ATOM | 1827 | CG2 | ILE | A | 270 | 35.767 | -8.565 | 31.346 | 1.00 | 69.21 | C |
| ATOM | 1828 | CG1 | ILE | A | 270 | 34.041 | -7.017 | 32.272 | 1.00 | 69.21 | C |
| ATOM | 1829 | CD1 | ILE | A | 270 | 33.293 | -8.078 | 33.001 | 1.00 | 69.21 | C |
| ATOM | 1830 | C | ILE | A | 270 | 36.439 | -6.738 | 29.225 | 1.00 | 57.32 | C |
| ATOM | 1831 | O | ILE | A | 270 | 37.522 | -7.237 | 29.518 | 1.00 | 57.32 | O |
| ATOM | 1832 | N | LYS | A | 271 | 36.064 | -6.521 | 27.968 | 1.00 | 59.60 | N |
| ATOM | 1833 | CA | LYS | A | 271 | 36.884 | -6.935 | 26.834 | 1.00 | 59.60 | C |
| ATOM | 1834 | CB | LYS | A | 271 | 36.007 | -7.041 | 25.578 | 1.00 | 76.71 | C |
| ATOM | 1835 | CG | LYS | A | 271 | 36.417 | -8.148 | 24.616 | 1.00 | 76.71 | C |
| ATOM | 1836 | CD | LYS | A | 271 | 35.718 | -9.449 | 24.966 | 1.00 | 76.71 | C |
| ATOM | 1837 | CE | LYS | A | 271 | 34.528 | -9.693 | 24.058 | 1.00 | 76.71 | C |
| ATOM | 1838 | NZ | LYS | A | 271 | 33.685 | -10.823 | 24.536 | 1.00 | 76.71 | N |
| ATOM | 1839 | C | LYS | A | 271 | 38.011 | -5.943 | 26.574 | 1.00 | 59.60 | C |
| ATOM | 1840 | O | LYS | A | 271 | 38.890 | -6.183 | 25.742 | 1.00 | 59.60 | O |
| ATOM | 1841 | N | VAL | A | 272 | 37.997 | -4.851 | 27.321 | 1.00 | 51.34 | N |
| ATOM | 1842 | CA | VAL | A | 272 | 38.953 | -3.772 | 27.135 | 1.00 | 51.34 | C |
| ATOM | 1843 | CB | VAL | A | 272 | 38.211 | -2.561 | 26.563 | 1.00 | 63.55 | C |
| ATOM | 1844 | CG1 | VAL | A | 272 | 36.795 | -2.562 | 27.102 | 1.00 | 63.55 | C |
| ATOM | 1845 | CG2 | VAL | A | 272 | 38.898 | -1.264 | 26.961 | 1.00 | 63.55 | C |
| ATOM | 1846 | C | VAL | A | 272 | 39.571 | -3.383 | 28.450 | 1.00 | 51.34 | C |
| ATOM | 1847 | O | VAL | A | 272 | 40.739 | -2.997 | 28.514 | 1.00 | 51.34 | O |
| ATOM | 1848 | N | LEU | A | 273 | 38.762 | -3.479 | 29.495 | 1.00 | 49.43 | N |
| ATOM | 1849 | CA | LEU | A | 273 | 39.191 | -3.121 | 30.833 | 1.00 | 49.43 | C |
| ATOM | 1850 | CB | LEU | A | 273 | 38.077 | -2.325 | 31.507 | 1.00 | 45.16 | C |
| ATOM | 1851 | CG | LEU | A | 273 | 38.078 | -0.877 | 31.107 | 1.00 | 45.16 | C |
| ATOM | 1852 | CD1 | LEU | A | 273 | 37.008 | -0.097 | 31.838 | 1.00 | 45.16 | C |
| ATOM | 1853 | CD2 | LEU | A | 273 | 39.440 | -0.399 | 31.472 | 1.00 | 45.16 | C |
| ATOM | 1854 | C | LEU | A | 273 | 39.616 | -4.287 | 31.726 | 1.00 | 49.43 | C |
| ATOM | 1855 | O | LEU | A | 273 | 40.184 | -4.083 | 32.803 | 1.00 | 49.43 | O |
| ATOM | 1856 | N | GLY | A | 274 | 39.375 | -5.506 | 31.265 | 1.00 | 48.33 | N |
| ATOM | 1857 | CA | GLY | A | 274 | 39.736 | -6.659 | 32.058 | 1.00 | 48.33 | C |
| ATOM | 1858 | C | GLY | A | 274 | 38.612 | -6.963 | 33.013 | 1.00 | 48.33 | C |
| ATOM | 1859 | O | GLY | A | 274 | 37.648 | -6.200 | 33.130 | 1.00 | 48.33 | O |
| ATOM | 1860 | N | THR | A | 275 | 38.725 | -8.096 | 33.686 | 1.00 | 53.25 | N |
| ATOM | 1861 | CA | THR | A | 275 | 37.708 | -8.482 | 34.630 | 1.00 | 53.25 | C |
| ATOM | 1862 | CB | THR | A | 275 | 37.869 | -9.970 | 35.006 | 1.00 | 42.47 | C |
| ATOM | 1863 | OG1 | THR | A | 275 | 37.149 | -10.763 | 34.060 | 1.00 | 42.47 | O |
| ATOM | 1864 | CG2 | THR | A | 275 | 37.343 | -10.260 | 36.392 | 1.00 | 42.47 | C |
| ATOM | 1865 | C | THR | A | 275 | 37.806 | -7.585 | 35.854 | 1.00 | 53.25 | C |
| ATOM | 1866 | O | THR | A | 275 | 38.905 | -7.173 | 36.255 | 1.00 | 53.25 | O |
| ATOM | 1867 | N | PRO | A | 276 | 36.648 | -7.223 | 36.427 | 1.00 | 51.75 | N |
| ATOM | 1868 | CD | PRO | A | 276 | 35.297 | -7.572 | 35.951 | 1.00 | 37.19 | C |
| ATOM | 1869 | CA | PRO | A | 276 | 36.617 | -6.370 | 37.615 | 1.00 | 51.75 | C |
| ATOM | 1870 | CB | PRO | A | 276 | 35.267 | -5.671 | 37.514 | 1.00 | 37.19 | C |
| ATOM | 1871 | CG | PRO | A | 276 | 34.395 | -6.669 | 36.783 | 1.00 | 37.19 | C |
| ATOM | 1872 | C | PRO | A | 276 | 36.758 | -7.208 | 38.878 | 1.00 | 51.75 | C |
| ATOM | 1873 | O | PRO | A | 276 | 36.031 | -8.189 | 39.060 | 1.00 | 51.75 | O |
| ATOM | 1874 | N | THR | A | 277 | 37.676 | -6.792 | 39.750 | 1.00 | 57.70 | N |
| ATOM | 1875 | CA | THR | A | 277 | 37.973 | -7.473 | 41.013 | 1.00 | 57.70 | C |
| ATOM | 1876 | CB | THR | A | 277 | 39.083 | -6.725 | 41.783 | 1.00 | 52.56 | C |
| ATOM | 1877 | OG1 | THR | A | 277 | 38.582 | -5.462 | 42.260 | 1.00 | 52.56 | O |
| ATOM | 1878 | CG2 | THR | A | 277 | 40.284 | -6.477 | 40.861 | 1.00 | 52.56 | C |
| ATOM | 1879 | C | THR | A | 277 | 36.741 | -7.587 | 41.915 | 1.00 | 57.70 | C |
| ATOM | 1880 | O | THR | A | 277 | 35.818 | -6.782 | 41.807 | 1.00 | 57.70 | O |
| ATOM | 1881 | N | ARG | A | 278 | 36.722 | -8.582 | 42.800 | 1.00 | 67.76 | N |
| ATOM | 1882 | CA | ARG | A | 278 | 35.577 | -8.756 | 43.687 | 1.00 | 67.76 | C |
| ATOM | 1883 | CB | ARG | A | 278 | 35.801 | -9.926 | 44.658 | 1.00 | 82.36 | C |
| ATOM | 1884 | CG | ARG | A | 278 | 35.966 | -11.298 | 43.986 | 1.00 | 82.36 | C |
| ATOM | 1885 | CD | ARG | A | 278 | 35.526 | -12.440 | 44.911 | 1.00 | 82.36 | C |

| | | | | | | | | | | | |
|---|---|---|---|---|---|---|---|---|---|---|---|
| ATOM | 1886 | NE | ARG | A | 278 | 35.855 | -12.174 | 46.312 | 1.00 | 82.36 | N |
| ATOM | 1887 | CZ | ARG | A | 278 | 35.606 | -13.006 | 47.322 | 1.00 | 82.36 | C |
| ATOM | 1888 | NH1 | ARG | A | 278 | 35.020 | -14.181 | 47.105 | 1.00 | 82.36 | N |
| ATOM | 1889 | NH2 | ARG | A | 278 | 35.936 | -12.653 | 48.558 | 1.00 | 82.36 | N |
| ATOM | 1890 | C | ARG | A | 278 | 35.312 | -7.465 | 44.456 | 1.00 | 67.76 | C |
| ATOM | 1891 | O | ARG | A | 278 | 34.171 | -7.146 | 44.773 | 1.00 | 67.76 | O |
| ATOM | 1892 | N | GLU | A | 279 | 36.368 | -6.713 | 44.744 | 1.00 | 52.27 | N |
| ATOM | 1893 | CA | GLU | A | 279 | 36.210 | -5.454 | 45.453 | 1.00 | 52.27 | C |
| ATOM | 1894 | CB | GLU | A | 279 | 37.574 | -4.914 | 45.884 | 1.00 | 103.68 | C |
| ATOM | 1895 | CG | GLU | A | 279 | 38.020 | -5.406 | 47.257 | 1.00 | 103.68 | C |
| ATOM | 1896 | CD | GLU | A | 279 | 38.191 | -6.913 | 47.331 | 1.00 | 103.68 | C |
| ATOM | 1897 | OE1 | GLU | A | 279 | 38.209 | -7.441 | 48.464 | 1.00 | 103.68 | O |
| ATOM | 1898 | OE2 | GLU | A | 279 | 38.318 | -7.566 | 46.268 | 1.00 | 103.68 | O |
| ATOM | 1899 | C | GLU | A | 279 | 35.488 | -4.447 | 44.559 | 1.00 | 52.27 | C |
| ATOM | 1900 | O | GLU | A | 279 | 34.472 | -3.873 | 44.970 | 1.00 | 52.27 | O |
| ATOM | 1901 | N | GLN | A | 280 | 36.004 | -4.249 | 43.340 | 1.00 | 47.01 | N |
| ATOM | 1902 | CA | GLN | A | 280 | 35.395 | -3.329 | 42.369 | 1.00 | 47.01 | C |
| ATOM | 1903 | CB | GLN | A | 280 | 36.078 | -3.430 | 41.001 | 1.00 | 53.57 | C |
| ATOM | 1904 | CG | GLN | A | 280 | 37.430 | -2.728 | 40.934 | 1.00 | 53.57 | C |
| ATOM | 1905 | CD | GLN | A | 280 | 38.229 | -3.082 | 39.687 | 1.00 | 53.57 | C |
| ATOM | 1906 | OE1 | GLN | A | 280 | 38.046 | -4.145 | 39.098 | 1.00 | 53.57 | O |
| ATOM | 1907 | NE2 | GLN | A | 280 | 39.136 | -2.200 | 39.296 | 1.00 | 53.57 | N |
| ATOM | 1908 | C | GLN | A | 280 | 33.923 | -3.642 | 42.208 | 1.00 | 47.01 | C |
| ATOM | 1909 | O | GLN | A | 280 | 33.134 | -2.785 | 41.843 | 1.00 | 47.01 | O |
| ATOM | 1910 | N | ILE | A | 281 | 33.551 | -4.878 | 42.481 | 1.00 | 36.21 | N |
| ATOM | 1911 | CA | ILE | A | 281 | 32.159 | -5.259 | 42.372 | 1.00 | 36.21 | C |
| ATOM | 1912 | CB | ILE | A | 281 | 31.996 | -6.785 | 42.514 | 1.00 | 53.52 | C |
| ATOM | 1913 | CG2 | ILE | A | 281 | 30.588 | -7.189 | 42.105 | 1.00 | 53.52 | C |
| ATOM | 1914 | CG1 | ILE | A | 281 | 33.028 | -7.515 | 41.645 | 1.00 | 53.52 | C |
| ATOM | 1915 | CD1 | ILE | A | 281 | 32.831 | -7.358 | 40.163 | 1.00 | 53.52 | C |
| ATOM | 1916 | C | ILE | A | 281 | 31.360 | -4.552 | 43.481 | 1.00 | 36.21 | C |
| ATOM | 1917 | O | ILE | A | 281 | 30.324 | -3.926 | 43.218 | 1.00 | 36.21 | O |
| ATOM | 1918 | N | ALA | A | 282 | 31.849 | -4.655 | 44.716 | 1.00 | 67.82 | N |
| ATOM | 1919 | CA | ALA | A | 282 | 31.194 | -4.021 | 45.855 | 1.00 | 67.82 | C |
| ATOM | 1920 | CB | ALA | A | 282 | 31.993 | -4.280 | 47.138 | 1.00 | 36.59 | C |
| ATOM | 1921 | C | ALA | A | 282 | 31.121 | -2.526 | 45.580 | 1.00 | 67.82 | C |
| ATOM | 1922 | O | ALA | A | 282 | 30.048 | -1.921 | 45.633 | 1.00 | 67.82 | O |
| ATOM | 1923 | N | GLU | A | 283 | 32.279 | -1.945 | 45.281 | 1.00 | 65.09 | N |
| ATOM | 1924 | CA | GLU | A | 283 | 32.383 | -0.527 | 44.989 | 1.00 | 65.09 | C |
| ATOM | 1925 | CB | GLU | A | 283 | 33.790 | -0.231 | 44.457 | 1.00 | 47.29 | C |
| ATOM | 1926 | CG | GLU | A | 283 | 34.894 | -0.583 | 45.484 | 1.00 | 47.29 | C |
| ATOM | 1927 | CD | GLU | A | 283 | 36.344 | -0.525 | 44.944 | 1.00 | 47.29 | C |
| ATOM | 1928 | OE1 | GLU | A | 283 | 36.736 | -1.424 | 44.159 | 1.00 | 47.29 | O |
| ATOM | 1929 | OE2 | GLU | A | 283 | 37.097 | 0.412 | 45.320 | 1.00 | 47.29 | O |
| ATOM | 1930 | C | GLU | A | 283 | 31.303 | -0.080 | 43.997 | 1.00 | 65.09 | C |
| ATOM | 1931 | O | GLU | A | 283 | 30.701 | 0.980 | 44.160 | 1.00 | 65.09 | O |
| ATOM | 1932 | N | MET | A | 284 | 31.036 | -0.895 | 42.984 | 1.00 | 40.50 | N |
| ATOM | 1933 | CA | MET | A | 284 | 30.019 | -0.536 | 42.001 | 1.00 | 40.50 | C |
| ATOM | 1934 | CB | MET | A | 284 | 30.162 | -1.394 | 40.743 | 1.00 | 61.67 | C |
| ATOM | 1935 | CG | MET | A | 284 | 31.518 | -1.262 | 40.059 | 1.00 | 61.67 | C |
| ATOM | 1936 | SD | MET | A | 284 | 31.628 | -2.303 | 38.597 | 1.00 | 61.67 | S |
| ATOM | 1937 | CE | MET | A | 284 | 30.620 | -1.428 | 37.655 | 1.00 | 61.67 | C |
| ATOM | 1938 | C | MET | A | 284 | 28.625 | -0.699 | 42.589 | 1.00 | 40.50 | C |
| ATOM | 1939 | O | MET | A | 284 | 27.847 | 0.255 | 42.640 | 1.00 | 40.50 | O |
| ATOM | 1940 | N | ASN | A | 285 | 28.308 | -1.916 | 43.021 | 1.00 | 45.74 | N |
| ATOM | 1941 | CA | ASN | A | 285 | 27.016 | -2.216 | 43.632 | 1.00 | 45.74 | C |
| ATOM | 1942 | CB | ASN | A | 285 | 25.963 | -2.611 | 42.594 | 1.00 | 33.96 | C |
| ATOM | 1943 | CG | ASN | A | 285 | 24.713 | -3.208 | 43.238 | 1.00 | 33.96 | C |
| ATOM | 1944 | OD1 | ASN | A | 285 | 24.555 | -3.155 | 44.455 | 1.00 | 33.96 | O |
| ATOM | 1945 | ND2 | ASN | A | 285 | 23.821 | -3.769 | 42.426 | 1.00 | 33.96 | N |
| ATOM | 1946 | C | ASN | A | 285 | 27.205 | -3.374 | 44.573 | 1.00 | 45.74 | C |
| ATOM | 1947 | O | ASN | A | 285 | 27.436 | -4.490 | 44.142 | 1.00 | 45.74 | O |
| ATOM | 1948 | N | PRO | A | 286 | 27.119 | -3.128 | 45.878 | 1.00 | 72.20 | N |

| | | | | | | | | | | | |
|---|---|---|---|---|---|---|---|---|---|---|---|
| ATOM | 1949 | CD | PRO | A | 286 | 27.071 | -1.854 | 46.607 | 1.00 | 29.05 | C |
| ATOM | 1950 | CA | PRO | A | 286 | 27.300 | -4.254 | 46.792 | 1.00 | 72.20 | C |
| ATOM | 1951 | CB | PRO | A | 286 | 27.330 | -3.583 | 48.169 | 1.00 | 29.05 | C |
| ATOM | 1952 | CG | PRO | A | 286 | 26.582 | -2.295 | 47.951 | 1.00 | 29.05 | C |
| ATOM | 1953 | C | PRO | A | 286 | 26.232 | -5.350 | 46.654 | 1.00 | 72.20 | C |
| ATOM | 1954 | O | PRO | A | 286 | 26.554 | -6.533 | 46.727 | 1.00 | 72.20 | O |
| ATOM | 1955 | N | ASN | A | 287 | 24.978 | -4.968 | 46.424 | 1.00 | 55.18 | N |
| ATOM | 1956 | CA | ASN | A | 287 | 23.904 | -5.953 | 46.277 | 1.00 | 55.18 | C |
| ATOM | 1957 | CB | ASN | A | 287 | 22.548 | -5.257 | 46.141 | 1.00 | 76.93 | C |
| ATOM | 1958 | CG | ASN | A | 287 | 22.288 | -4.261 | 47.256 | 1.00 | 76.93 | C |
| ATOM | 1959 | OD1 | ASN | A | 287 | 21.140 | -4.049 | 47.660 | 1.00 | 76.93 | O |
| ATOM | 1960 | ND2 | ASN | A | 287 | 23.351 | -3.633 | 47.751 | 1.00 | 76.93 | N |
| ATOM | 1961 | C | ASN | A | 287 | 24.126 | -6.861 | 45.068 | 1.00 | 55.18 | C |
| ATOM | 1962 | O | ASN | A | 287 | 23.477 | -7.902 | 44.929 | 1.00 | 55.18 | O |
| ATOM | 1963 | N | ALA | A | 294 | 35.182 | -14.914 | 34.965 | 1.00 | 46.50 | N |
| ATOM | 1964 | CA | ALA | A | 294 | 36.394 | -15.530 | 34.431 | 1.00 | 46.50 | C |
| ATOM | 1965 | CB | ALA | A | 294 | 36.106 | -16.163 | 33.081 | 1.00 | 34.59 | C |
| ATOM | 1966 | C | ALA | A | 294 | 37.493 | -14.486 | 34.290 | 1.00 | 46.50 | C |
| ATOM | 1967 | O | ALA | A | 294 | 37.532 | -13.755 | 33.300 | 1.00 | 46.50 | O |
| ATOM | 1968 | N | ALA | A | 295 | 38.383 | -14.414 | 35.274 | 1.00 | 65.10 | N |
| ATOM | 1969 | CA | ALA | A | 295 | 39.468 | -13.436 | 35.245 | 1.00 | 65.10 | C |
| ATOM | 1970 | CB | ALA | A | 295 | 40.586 | -13.868 | 36.177 | 1.00 | 28.74 | C |
| ATOM | 1971 | C | ALA | A | 295 | 40.037 | -13.247 | 33.844 | 1.00 | 65.10 | C |
| ATOM | 1972 | O | ALA | A | 295 | 40.432 | -14.214 | 33.209 | 1.00 | 65.10 | O |
| ATOM | 1973 | N | ALA | A | 296 | 40.086 | -12.013 | 33.354 | 1.00 | 55.28 | N |
| ATOM | 1974 | CA | ALA | A | 296 | 40.647 | -11.755 | 32.031 | 1.00 | 55.28 | C |
| ATOM | 1975 | CB | ALA | A | 296 | 39.512 | -11.617 | 30.954 | 1.00 | 27.51 | C |
| ATOM | 1976 | C | ALA | A | 296 | 41.480 | -10.486 | 32.115 | 1.00 | 55.28 | C |
| ATOM | 1977 | O | ALA | A | 296 | 41.059 | -9.488 | 32.732 | 1.00 | 55.28 | O |
| ATOM | 1978 | N | ALA | A | 297 | 42.674 | -10.551 | 31.529 | 1.00 | 78.25 | N |
| ATOM | 1979 | CA | ALA | A | 297 | 43.594 | -9.419 | 31.487 | 1.00 | 78.25 | C |
| ATOM | 1980 | CB | ALA | A | 297 | 44.828 | -9.793 | 30.698 | 1.00 | 53.02 | C |
| ATOM | 1981 | C | ALA | A | 297 | 42.883 | -8.250 | 30.813 | 1.00 | 78.25 | C |
| ATOM | 1982 | O | ALA | A | 297 | 41.667 | -8.297 | 30.572 | 1.00 | 78.25 | O |
| ATOM | 1983 | N | ALA | A | 298 | 43.603 | -7.191 | 30.496 | 1.00 | 80.48 | N |
| ATOM | 1984 | CA | ALA | A | 298 | 42.912 | -6.107 | 29.842 | 1.00 | 80.48 | C |
| ATOM | 1985 | CB | ALA | A | 298 | 42.554 | -5.005 | 30.829 | 1.00 | 61.69 | C |
| ATOM | 1986 | C | ALA | A | 298 | 43.797 | -5.589 | 28.774 | 1.00 | 80.48 | C |
| ATOM | 1987 | O | ALA | A | 298 | 45.009 | -5.459 | 28.956 | 1.00 | 80.48 | O |
| ATOM | 1988 | N | HIS | A | 299 | 43.176 | -5.291 | 27.650 | 1.00 | 76.13 | N |
| ATOM | 1989 | CA | HIS | A | 299 | 43.933 | -4.801 | 26.551 | 1.00 | 76.13 | C |
| ATOM | 1990 | CB | HIS | A | 299 | 42.995 | -4.512 | 25.365 | 1.00 | 32.75 | C |
| ATOM | 1991 | CG | HIS | A | 299 | 43.645 | -4.684 | 24.032 | 1.00 | 23.68 | C |
| ATOM | 1992 | CD2 | HIS | A | 299 | 43.900 | -3.800 | 23.042 | 1.00 | 23.68 | C |
| ATOM | 1993 | ND1 | HIS | A | 299 | 44.108 | -5.916 | 23.591 | 1.00 | 23.68 | N |
| ATOM | 1994 | CE1 | HIS | A | 299 | 44.623 | -5.770 | 22.376 | 1.00 | 23.68 | C |
| ATOM | 1995 | NE2 | HIS | A | 299 | 44.515 | -4.502 | 22.021 | 1.00 | 23.68 | N |
| ATOM | 1996 | C | HIS | A | 299 | 44.475 | -3.508 | 27.086 | 1.00 | 76.13 | C |
| ATOM | 1997 | O | HIS | A | 299 | 43.942 | -2.976 | 27.995 | 1.00 | 76.13 | O |
| ATOM | 1998 | N | PRO | A | 300 | 45.585 | -3.021 | 26.563 | 1.00 | 51.41 | N |
| ATOM | 1999 | CD | PRO | A | 300 | 46.601 | -3.841 | 25.915 | 1.00 | 68.30 | C |
| ATOM | 2000 | CA | PRO | A | 300 | 46.157 | -1.746 | 27.025 | 1.00 | 51.41 | C |
| ATOM | 2001 | CB | PRO | A | 300 | 47.398 | -1.595 | 26.171 | 1.00 | 68.30 | C |
| ATOM | 2002 | CG | PRO | A | 300 | 47.788 | -2.953 | 25.952 | 1.00 | 68.30 | C |
| ATOM | 2003 | C | PRO | A | 300 | 45.142 | -0.744 | 26.584 | 1.00 | 51.41 | C |
| ATOM | 2004 | O | PRO | A | 300 | 44.110 | -1.136 | 26.049 | 1.00 | 51.41 | O |
| ATOM | 2005 | N | TRP | A | 301 | 45.439 | 0.531 | 26.785 | 1.00 | 53.94 | N |
| ATOM | 2006 | CA | TRP | A | 301 | 44.519 | 1.577 | 26.396 | 1.00 | 53.94 | C |
| ATOM | 2007 | CB | TRP | A | 301 | 44.606 | 2.735 | 27.374 | 1.00 | 51.46 | C |
| ATOM | 2008 | CG | TRP | A | 301 | 43.759 | 2.431 | 28.485 | 1.00 | 51.46 | C |
| ATOM | 2009 | CD2 | TRP | A | 301 | 42.336 | 2.388 | 28.462 | 1.00 | 51.46 | C |
| ATOM | 2010 | CE2 | TRP | A | 301 | 41.945 | 1.923 | 29.737 | 1.00 | 51.46 | C |
| ATOM | 2011 | CE3 | TRP | A | 301 | 41.375 | 2.682 | 27.491 | 1.00 | 51.46 | C |

| | | | | | | | | | | | |
|---|---|---|---|---|---|---|---|---|---|---|---|
| ATOM | 2012 | CD1 | TRP | A | 301 | 44.145 | 2.010 | 29.714 | 1.00 | 51.46 | C |
| ATOM | 2013 | NE1 | TRP | A | 301 | 43.049 | 1.696 | 30.468 | 1.00 | 51.46 | N |
| ATOM | 2014 | CZ2 | TRP | A | 301 | 40.594 | 1.769 | 30.051 | 1.00 | 51.46 | C |
| ATOM | 2015 | CZ3 | TRP | A | 301 | 40.053 | 2.512 | 27.811 | 1.00 | 51.46 | C |
| ATOM | 2016 | CH2 | TRP | A | 301 | 39.668 | 2.062 | 29.081 | 1.00 | 51.46 | C |
| ATOM | 2017 | C | TRP | A | 301 | 44.933 | 1.991 | 25.024 | 1.00 | 53.94 | C |
| ATOM | 2018 | O | TRP | A | 301 | 44.121 | 2.299 | 24.144 | 1.00 | 53.94 | O |
| ATOM | 2019 | N | THR | A | 302 | 46.240 | 1.897 | 24.864 | 1.00 | 51.94 | N |
| ATOM | 2020 | CA | THR | A | 302 | 46.975 | 2.237 | 23.666 | 1.00 | 51.94 | C |
| ATOM | 2021 | CB | THR | A | 302 | 48.443 | 2.356 | 24.035 | 1.00 | 54.81 | C |
| ATOM | 2022 | OG1 | THR | A | 302 | 49.161 | 2.871 | 22.918 | 1.00 | 54.81 | O |
| ATOM | 2023 | CG2 | THR | A | 302 | 48.989 | 1.009 | 24.488 | 1.00 | 54.81 | C |
| ATOM | 2024 | C | THR | A | 302 | 46.804 | 1.246 | 22.508 | 1.00 | 51.94 | C |
| ATOM | 2025 | O | THR | A | 302 | 47.024 | 1.594 | 21.338 | 1.00 | 51.94 | O |
| ATOM | 2026 | N | ALA | A | 303 | 46.393 | 0.030 | 22.844 | 1.00 | 55.43 | N |
| ATOM | 2027 | CA | ALA | A | 303 | 46.211 | -1.003 | 21.861 | 1.00 | 55.43 | C |
| ATOM | 2028 | CB | ALA | A | 303 | 46.418 | -2.368 | 22.517 | 1.00 | 65.28 | C |
| ATOM | 2029 | C | ALA | A | 303 | 44.827 | -0.849 | 21.331 | 1.00 | 55.43 | C |
| ATOM | 2030 | O | ALA | A | 303 | 44.378 | -1.641 | 20.518 | 1.00 | 55.43 | O |
| ATOM | 2031 | N | VAL | A | 304 | 44.188 | 0.219 | 21.784 | 1.00 | 60.12 | N |
| ATOM | 2032 | CA | VAL | A | 304 | 42.815 | 0.529 | 21.428 | 1.00 | 60.12 | C |
| ATOM | 2033 | CB | VAL | A | 304 | 42.050 | 0.732 | 22.733 | 1.00 | 65.14 | C |
| ATOM | 2034 | CG1 | VAL | A | 304 | 40.613 | 1.144 | 22.502 | 1.00 | 65.14 | C |
| ATOM | 2035 | CG2 | VAL | A | 304 | 42.137 | -0.561 | 23.531 | 1.00 | 65.14 | C |
| ATOM | 2036 | C | VAL | A | 304 | 42.564 | 1.706 | 20.479 | 1.00 | 60.12 | C |
| ATOM | 2037 | O | VAL | A | 304 | 41.623 | 1.694 | 19.679 | 1.00 | 60.12 | O |
| ATOM | 2038 | N | PHE | A | 305 | 43.407 | 2.721 | 20.535 | 1.00 | 63.30 | N |
| ATOM | 2039 | CA | PHE | A | 305 | 43.178 | 3.864 | 19.686 | 1.00 | 63.30 | C |
| ATOM | 2040 | CB | PHE | A | 305 | 43.303 | 5.145 | 20.506 | 1.00 | 50.37 | C |
| ATOM | 2041 | CG | PHE | A | 305 | 42.202 | 5.309 | 21.516 | 1.00 | 50.37 | C |
| ATOM | 2042 | CD1 | PHE | A | 305 | 40.904 | 5.638 | 21.118 | 1.00 | 50.37 | C |
| ATOM | 2043 | CD2 | PHE | A | 305 | 42.433 | 5.007 | 22.844 | 1.00 | 50.37 | C |
| ATOM | 2044 | CE1 | PHE | A | 305 | 39.858 | 5.650 | 22.039 | 1.00 | 50.37 | C |
| ATOM | 2045 | CE2 | PHE | A | 305 | 41.410 | 5.016 | 23.756 | 1.00 | 50.37 | C |
| ATOM | 2046 | CZ | PHE | A | 305 | 40.115 | 5.332 | 23.362 | 1.00 | 50.37 | C |
| ATOM | 2047 | C | PHE | A | 305 | 44.218 | 3.731 | 18.631 | 1.00 | 63.30 | C |
| ATOM | 2048 | O | PHE | A | 305 | 45.084 | 2.855 | 18.713 | 1.00 | 63.30 | O |
| ATOM | 2049 | N | ARG | A | 306 | 44.116 | 4.567 | 17.619 | 1.00 | 78.76 | N |
| ATOM | 2050 | CA | ARG | A | 306 | 45.044 | 4.492 | 16.528 | 1.00 | 78.76 | C |
| ATOM | 2051 | CB | ARG | A | 306 | 44.789 | 5.628 | 15.538 | 1.00 | 103.68 | C |
| ATOM | 2052 | CG | ARG | A | 306 | 43.547 | 5.394 | 14.716 | 1.00 | 103.68 | C |
| ATOM | 2053 | CD | ARG | A | 306 | 42.306 | 5.411 | 15.606 | 1.00 | 103.68 | C |
| ATOM | 2054 | NE | ARG | A | 306 | 41.152 | 4.710 | 15.034 | 1.00 | 103.68 | N |
| ATOM | 2055 | CZ | ARG | A | 306 | 40.951 | 3.387 | 15.084 | 1.00 | 103.68 | C |
| ATOM | 2056 | NH1 | ARG | A | 306 | 41.823 | 2.571 | 15.690 | 1.00 | 103.68 | N |
| ATOM | 2057 | NH2 | ARG | A | 306 | 39.859 | 2.866 | 14.529 | 1.00 | 103.68 | N |
| ATOM | 2058 | C | ARG | A | 306 | 46.467 | 4.574 | 16.984 | 1.00 | 78.76 | C |
| ATOM | 2059 | O | ARG | A | 306 | 46.767 | 4.995 | 18.104 | 1.00 | 78.76 | O |
| ATOM | 2060 | N | PRO | A | 307 | 47.382 | 4.131 | 16.130 | 1.00 | 60.85 | N |
| ATOM | 2061 | CD | PRO | A | 307 | 47.239 | 3.396 | 14.864 | 1.00 | 52.78 | C |
| ATOM | 2062 | CA | PRO | A | 307 | 48.779 | 4.224 | 16.547 | 1.00 | 60.85 | C |
| ATOM | 2063 | CB | PRO | A | 307 | 49.527 | 3.818 | 15.295 | 1.00 | 52.78 | C |
| ATOM | 2064 | CG | PRO | A | 307 | 48.603 | 2.831 | 14.678 | 1.00 | 52.78 | C |
| ATOM | 2065 | C | PRO | A | 307 | 49.012 | 5.694 | 16.874 | 1.00 | 60.85 | C |
| ATOM | 2066 | O | PRO | A | 307 | 49.388 | 6.052 | 17.990 | 1.00 | 60.85 | O |
| ATOM | 2067 | N | ALA | A | 308 | 48.705 | 6.535 | 15.892 | 1.00 | 59.09 | N |
| ATOM | 2068 | CA | ALA | A | 308 | 48.847 | 7.994 | 16.002 | 1.00 | 59.09 | C |
| ATOM | 2069 | CB | ALA | A | 308 | 48.416 | 8.641 | 14.698 | 1.00 | 33.38 | C |
| ATOM | 2070 | C | ALA | A | 308 | 48.181 | 8.765 | 17.170 | 1.00 | 59.09 | C |
| ATOM | 2071 | O | ALA | A | 308 | 48.650 | 9.854 | 17.513 | 1.00 | 59.09 | O |
| ATOM | 2072 | N | THR | A | 309 | 47.110 | 8.240 | 17.765 | 1.00 | 53.54 | N |
| ATOM | 2073 | CA | THR | A | 309 | 46.429 | 8.916 | 18.873 | 1.00 | 53.54 | C |
| ATOM | 2074 | CB | THR | A | 309 | 45.460 | 7.948 | 19.564 | 1.00 | 60.52 | C |

| | | | | | | | | | | | |
|---|---|---|---|---|---|---|---|---|---|---|---|
| ATOM | 2075 | OG1 | THR | A | 309 | 44.826 | 7.127 | 18.575 | 1.00 | 60.52 | O |
| ATOM | 2076 | CG2 | THR | A | 309 | 44.393 | 8.716 | 20.309 | 1.00 | 60.52 | C |
| ATOM | 2077 | C | THR | A | 309 | 47.406 | 9.490 | 19.916 | 1.00 | 53.54 | C |
| ATOM | 2078 | O | THR | A | 309 | 48.381 | 8.847 | 20.306 | 1.00 | 53.54 | O |
| ATOM | 2079 | N | PRO | A | 310 | 47.148 | 10.724 | 20.375 | 1.00 | 46.26 | N |
| ATOM | 2080 | CD | PRO | A | 310 | 46.108 | 11.633 | 19.870 | 1.00 | 37.32 | C |
| ATOM | 2081 | CA | PRO | A | 310 | 47.977 | 11.411 | 21.365 | 1.00 | 46.26 | C |
| ATOM | 2082 | CB | PRO | A | 310 | 47.329 | 12.782 | 21.467 | 1.00 | 37.32 | C |
| ATOM | 2083 | CG | PRO | A | 310 | 46.731 | 12.969 | 20.132 | 1.00 | 37.32 | C |
| ATOM | 2084 | C | PRO | A | 310 | 47.979 | 10.694 | 22.704 | 1.00 | 46.26 | C |
| ATOM | 2085 | O | PRO | A | 310 | 46.931 | 10.356 | 23.255 | 1.00 | 46.26 | O |
| ATOM | 2086 | N | PRO | A | 311 | 49.167 | 10.481 | 23.260 | 1.00 | 42.77 | N |
| ATOM | 2087 | CD | PRO | A | 311 | 50.461 | 10.996 | 22.787 | 1.00 | 28.90 | C |
| ATOM | 2088 | CA | PRO | A | 311 | 49.308 | 9.797 | 24.545 | 1.00 | 42.77 | C |
| ATOM | 2089 | CB | PRO | A | 311 | 50.793 | 9.970 | 24.864 | 1.00 | 28.90 | C |
| ATOM | 2090 | CG | PRO | A | 311 | 51.429 | 10.116 | 23.527 | 1.00 | 28.90 | C |
| ATOM | 2091 | C | PRO | A | 311 | 48.419 | 10.392 | 25.634 | 1.00 | 42.77 | C |
| ATOM | 2092 | O | PRO | A | 311 | 47.808 | 9.669 | 26.431 | 1.00 | 42.77 | O |
| ATOM | 2093 | N | GLU | A | 312 | 48.349 | 11.718 | 25.652 | 1.00 | 54.66 | N |
| ATOM | 2094 | CA | GLU | A | 312 | 47.572 | 12.421 | 26.658 | 1.00 | 54.66 | C |
| ATOM | 2095 | CB | GLU | A | 312 | 47.926 | 13.903 | 26.636 | 1.00 | 69.68 | C |
| ATOM | 2096 | CG | GLU | A | 312 | 47.966 | 14.501 | 28.020 | 1.00 | 69.68 | C |
| ATOM | 2097 | CD | GLU | A | 312 | 48.933 | 13.770 | 28.938 | 1.00 | 69.68 | C |
| ATOM | 2098 | OE1 | GLU | A | 312 | 50.159 | 13.964 | 28.791 | 1.00 | 69.68 | O |
| ATOM | 2099 | OE2 | GLU | A | 312 | 48.468 | 12.991 | 29.800 | 1.00 | 69.68 | O |
| ATOM | 2100 | C | GLU | A | 312 | 46.068 | 12.238 | 26.515 | 1.00 | 54.66 | C |
| ATOM | 2101 | O | GLU | A | 312 | 45.318 | 12.467 | 27.460 | 1.00 | 54.66 | O |
| ATOM | 2102 | N | ALA | A | 313 | 45.625 | 11.826 | 25.335 | 1.00 | 40.49 | N |
| ATOM | 2103 | CA | ALA | A | 313 | 44.207 | 11.603 | 25.120 | 1.00 | 40.49 | C |
| ATOM | 2104 | CB | ALA | A | 313 | 43.886 | 11.640 | 23.629 | 1.00 | 29.07 | C |
| ATOM | 2105 | C | ALA | A | 313 | 43.881 | 10.236 | 25.702 | 1.00 | 40.49 | C |
| ATOM | 2106 | O | ALA | A | 313 | 42.816 | 10.028 | 26.291 | 1.00 | 40.49 | O |
| ATOM | 2107 | N | ILE | A | 314 | 44.820 | 9.310 | 25.538 | 1.00 | 46.94 | N |
| ATOM | 2108 | CA | ILE | A | 314 | 44.649 | 7.958 | 26.041 | 1.00 | 46.94 | C |
| ATOM | 2109 | CB | ILE | A | 314 | 45.668 | 6.995 | 25.404 | 1.00 | 40.68 | C |
| ATOM | 2110 | CG2 | ILE | A | 314 | 45.335 | 5.549 | 25.795 | 1.00 | 40.68 | C |
| ATOM | 2111 | CG1 | ILE | A | 314 | 45.638 | 7.167 | 23.879 | 1.00 | 40.68 | C |
| ATOM | 2112 | CD1 | ILE | A | 314 | 46.664 | 6.366 | 23.123 | 1.00 | 40.68 | C |
| ATOM | 2113 | C | ILE | A | 314 | 44.813 | 7.953 | 27.553 | 1.00 | 46.94 | C |
| ATOM | 2114 | O | ILE | A | 314 | 44.257 | 7.099 | 28.236 | 1.00 | 46.94 | O |
| ATOM | 2115 | N | ALA | A | 315 | 45.570 | 8.911 | 28.076 | 1.00 | 48.22 | N |
| ATOM | 2116 | CA | ALA | A | 315 | 45.771 | 9.006 | 29.519 | 1.00 | 48.22 | C |
| ATOM | 2117 | CB | ALA | A | 315 | 46.874 | 10.012 | 29.821 | 1.00 | 30.87 | C |
| ATOM | 2118 | C | ALA | A | 315 | 44.451 | 9.448 | 30.166 | 1.00 | 48.22 | C |
| ATOM | 2119 | O | ALA | A | 315 | 43.917 | 8.781 | 31.057 | 1.00 | 48.22 | O |
| ATOM | 2120 | N | LEU | A | 316 | 43.931 | 10.579 | 29.697 | 1.00 | 48.77 | N |
| ATOM | 2121 | CA | LEU | A | 316 | 42.677 | 11.131 | 30.197 | 1.00 | 48.77 | C |
| ATOM | 2122 | CB | LEU | A | 316 | 42.261 | 12.328 | 29.342 | 1.00 | 55.08 | C |
| ATOM | 2123 | CG | LEU | A | 316 | 40.914 | 12.985 | 29.643 | 1.00 | 55.08 | C |
| ATOM | 2124 | CD1 | LEU | A | 316 | 40.956 | 13.640 | 31.009 | 1.00 | 55.08 | C |
| ATOM | 2125 | CD2 | LEU | A | 316 | 40.609 | 14.015 | 28.576 | 1.00 | 55.08 | C |
| ATOM | 2126 | C | LEU | A | 316 | 41.579 | 10.083 | 30.141 | 1.00 | 48.77 | C |
| ATOM | 2127 | O | LEU | A | 316 | 40.779 | 9.952 | 31.059 | 1.00 | 48.77 | O |
| ATOM | 2128 | N | CYS | A | 317 | 41.556 | 9.335 | 29.048 | 1.00 | 47.14 | N |
| ATOM | 2129 | CA | CYS | A | 317 | 40.548 | 8.315 | 28.844 | 1.00 | 47.14 | C |
| ATOM | 2130 | CB | CYS | A | 317 | 40.643 | 7.778 | 27.403 | 1.00 | 38.56 | C |
| ATOM | 2131 | SG | CYS | A | 317 | 39.252 | 6.729 | 26.866 | 1.00 | 38.56 | S |
| ATOM | 2132 | C | CYS | A | 317 | 40.687 | 7.186 | 29.859 | 1.00 | 47.14 | C |
| ATOM | 2133 | O | CYS | A | 317 | 39.694 | 6.597 | 30.279 | 1.00 | 47.14 | O |
| ATOM | 2134 | N | SER | A | 318 | 41.914 | 6.891 | 30.269 | 1.00 | 43.48 | N |
| ATOM | 2135 | CA | SER | A | 318 | 42.123 | 5.813 | 31.230 | 1.00 | 43.48 | C |
| ATOM | 2136 | CB | SER | A | 318 | 43.591 | 5.380 | 31.252 | 1.00 | 48.56 | C |
| ATOM | 2137 | OG | SER | A | 318 | 44.435 | 6.438 | 31.667 | 1.00 | 48.56 | O |

| | | | | | | | | | | | |
|---|---|---|---|---|---|---|---|---|---|---|---|
| ATOM | 2138 | C | SER | A | 318 | 41.684 | 6.200 | 32.639 | 1.00 | 43.48 | C |
| ATOM | 2139 | O | SER | A | 318 | 41.477 | 5.326 | 33.480 | 1.00 | 43.48 | O |
| ATOM | 2140 | N | ARG | A | 319 | 41.545 | 7.505 | 32.891 | 1.00 | 45.47 | N |
| ATOM | 2141 | CA | ARG | A | 319 | 41.115 | 8.005 | 34.199 | 1.00 | 45.47 | C |
| ATOM | 2142 | CB | ARG | A | 319 | 41.907 | 9.259 | 34.583 | 1.00 | 82.71 | C |
| ATOM | 2143 | CG | ARG | A | 319 | 43.344 | 8.982 | 35.002 | 1.00 | 82.71 | C |
| ATOM | 2144 | CD | ARG | A | 319 | 43.424 | 8.130 | 36.276 | 1.00 | 82.71 | C |
| ATOM | 2145 | NE | ARG | A | 319 | 42.892 | 8.814 | 37.458 | 1.00 | 82.71 | N |
| ATOM | 2146 | CZ | ARG | A | 319 | 42.881 | 8.294 | 38.686 | 1.00 | 82.71 | C |
| ATOM | 2147 | NH1 | ARG | A | 319 | 43.372 | 7.077 | 38.906 | 1.00 | 82.71 | N |
| ATOM | 2148 | NH2 | ARG | A | 319 | 42.384 | 8.990 | 39.701 | 1.00 | 82.71 | N |
| ATOM | 2149 | C | ARG | A | 319 | 39.616 | 8.311 | 34.231 | 1.00 | 45.47 | C |
| ATOM | 2150 | O | ARG | A | 319 | 39.093 | 8.800 | 35.235 | 1.00 | 45.47 | O |
| ATOM | 2151 | N | LEU | A | 320 | 38.935 | 8.019 | 33.126 | 1.00 | 36.24 | N |
| ATOM | 2152 | CA | LEU | A | 320 | 37.499 | 8.244 | 33.021 | 1.00 | 36.24 | C |
| ATOM | 2153 | CB | LEU | A | 320 | 37.171 | 9.047 | 31.760 | 1.00 | 26.54 | C |
| ATOM | 2154 | CG | LEU | A | 320 | 37.836 | 10.416 | 31.558 | 1.00 | 26.54 | C |
| ATOM | 2155 | CD1 | LEU | A | 320 | 37.400 | 10.983 | 30.217 | 1.00 | 26.54 | C |
| ATOM | 2156 | CD2 | LEU | A | 320 | 37.470 | 11.370 | 32.680 | 1.00 | 26.54 | C |
| ATOM | 2157 | C | LEU | A | 320 | 36.737 | 6.917 | 32.968 | 1.00 | 36.24 | C |
| ATOM | 2158 | O | LEU | A | 320 | 35.667 | 6.776 | 33.570 | 1.00 | 36.24 | O |
| ATOM | 2159 | N | LEU | A | 321 | 37.290 | 5.946 | 32.243 | 1.00 | 46.23 | N |
| ATOM | 2160 | CA | LEU | A | 321 | 36.647 | 4.648 | 32.099 | 1.00 | 46.23 | C |
| ATOM | 2161 | CB | LEU | A | 321 | 36.805 | 4.164 | 30.658 | 1.00 | 37.73 | C |
| ATOM | 2162 | CG | LEU | A | 321 | 36.182 | 5.127 | 29.634 | 1.00 | 37.73 | C |
| ATOM | 2163 | CD1 | LEU | A | 321 | 36.420 | 4.619 | 28.216 | 1.00 | 37.73 | C |
| ATOM | 2164 | CD2 | LEU | A | 321 | 34.689 | 5.269 | 29.905 | 1.00 | 37.73 | C |
| ATOM | 2165 | C | LEU | A | 321 | 37.171 | 3.604 | 33.080 | 1.00 | 46.23 | C |
| ATOM | 2166 | O | LEU | A | 321 | 37.873 | 2.664 | 32.699 | 1.00 | 46.23 | O |
| ATOM | 2167 | N | GLU | A | 322 | 36.799 | 3.776 | 34.347 | 1.00 | 49.21 | N |
| ATOM | 2168 | CA | GLU | A | 322 | 37.215 | 2.876 | 35.421 | 1.00 | 49.21 | C |
| ATOM | 2169 | CB | GLU | A | 322 | 38.115 | 3.620 | 36.401 | 1.00 | 58.28 | C |
| ATOM | 2170 | CG | GLU | A | 322 | 39.120 | 4.523 | 35.739 | 1.00 | 58.28 | C |
| ATOM | 2171 | CD | GLU | A | 322 | 40.370 | 4.682 | 36.574 | 1.00 | 58.28 | C |
| ATOM | 2172 | OE1 | GLU | A | 322 | 40.991 | 3.654 | 36.919 | 1.00 | 58.28 | O |
| ATOM | 2173 | OE2 | GLU | A | 322 | 40.746 | 5.828 | 36.887 | 1.00 | 58.28 | O |
| ATOM | 2174 | C | GLU | A | 322 | 36.018 | 2.314 | 36.185 | 1.00 | 49.21 | C |
| ATOM | 2175 | O | GLU | A | 322 | 35.027 | 3.010 | 36.392 | 1.00 | 49.21 | O |
| ATOM | 2176 | N | TYR | A | 323 | 36.127 | 1.061 | 36.619 | 1.00 | 58.48 | N |
| ATOM | 2177 | CA | TYR | A | 323 | 35.050 | 0.413 | 37.357 | 1.00 | 58.48 | C |
| ATOM | 2178 | CB | TYR | A | 323 | 35.417 | -1.039 | 37.680 | 1.00 | 58.49 | C |
| ATOM | 2179 | CG | TYR | A | 323 | 35.323 | -1.969 | 36.495 | 1.00 | 58.49 | C |
| ATOM | 2180 | CD1 | TYR | A | 323 | 34.110 | -2.161 | 35.831 | 1.00 | 58.49 | C |
| ATOM | 2181 | CE1 | TYR | A | 323 | 34.026 | -2.983 | 34.709 | 1.00 | 58.49 | C |
| ATOM | 2182 | CD2 | TYR | A | 323 | 36.450 | -2.630 | 36.012 | 1.00 | 58.49 | C |
| ATOM | 2183 | CE2 | TYR | A | 323 | 36.373 | -3.452 | 34.894 | 1.00 | 58.49 | C |
| ATOM | 2184 | CZ | TYR | A | 323 | 35.164 | -3.620 | 34.247 | 1.00 | 58.49 | C |
| ATOM | 2185 | OH | TYR | A | 323 | 35.111 | -4.405 | 33.124 | 1.00 | 58.49 | O |
| ATOM | 2186 | C | TYR | A | 323 | 34.688 | 1.140 | 38.649 | 1.00 | 58.48 | C |
| ATOM | 2187 | O | TYR | A | 323 | 33.507 | 1.395 | 38.908 | 1.00 | 58.48 | O |
| ATOM | 2188 | N | THR | A | 324 | 35.695 | 1.458 | 39.463 | 1.00 | 39.53 | N |
| ATOM | 2189 | CA | THR | A | 324 | 35.458 | 2.151 | 40.725 | 1.00 | 39.53 | C |
| ATOM | 2190 | CB | THR | A | 324 | 36.758 | 2.271 | 41.533 | 1.00 | 64.34 | C |
| ATOM | 2191 | OG1 | THR | A | 324 | 37.193 | 0.968 | 41.929 | 1.00 | 64.34 | O |
| ATOM | 2192 | CG2 | THR | A | 324 | 36.542 | 3.112 | 42.763 | 1.00 | 64.34 | C |
| ATOM | 2193 | C | THR | A | 324 | 34.903 | 3.546 | 40.429 | 1.00 | 39.53 | C |
| ATOM | 2194 | O | THR | A | 324 | 35.638 | 4.456 | 40.044 | 1.00 | 39.53 | O |
| ATOM | 2195 | N | PRO | A | 325 | 33.591 | 3.732 | 40.615 | 1.00 | 50.25 | N |
| ATOM | 2196 | CD | PRO | A | 325 | 32.652 | 2.776 | 41.220 | 1.00 | 40.06 | C |
| ATOM | 2197 | CA | PRO | A | 325 | 32.937 | 5.022 | 40.357 | 1.00 | 50.25 | C |
| ATOM | 2198 | CB | PRO | A | 325 | 31.520 | 4.805 | 40.883 | 1.00 | 40.06 | C |
| ATOM | 2199 | CG | PRO | A | 325 | 31.329 | 3.323 | 40.772 | 1.00 | 40.06 | C |
| ATOM | 2200 | C | PRO | A | 325 | 33.603 | 6.216 | 41.036 | 1.00 | 50.25 | C |

| | | | | | | | | | | | |
|---|---|---|---|---|---|---|---|---|---|---|---|
| ATOM | 2201 | O | PRO | A | 325 | 33.439 | 7.363 | 40.616 | 1.00 | 50.25 | O |
| ATOM | 2202 | N | THR | A | 326 | 34.361 | 5.941 | 42.085 | 1.00 | 35.83 | N |
| ATOM | 2203 | CA | THR | A | 326 | 35.010 | 6.992 | 42.844 | 1.00 | 35.83 | C |
| ATOM | 2204 | CB | THR | A | 326 | 34.942 | 6.670 | 44.316 | 1.00 | 32.41 | C |
| ATOM | 2205 | OG1 | THR | A | 326 | 35.662 | 5.452 | 44.555 | 1.00 | 32.41 | O |
| ATOM | 2206 | CG2 | THR | A | 326 | 33.484 | 6.482 | 44.741 | 1.00 | 32.41 | C |
| ATOM | 2207 | C | THR | A | 326 | 36.454 | 7.157 | 42.444 | 1.00 | 35.83 | C |
| ATOM | 2208 | O | THR | A | 326 | 37.144 | 8.044 | 42.939 | 1.00 | 35.83 | O |
| ATOM | 2209 | N | ALA | A | 327 | 36.910 | 6.288 | 41.551 | 1.00 | 33.10 | N |
| ATOM | 2210 | CA | ALA | A | 327 | 38.283 | 6.336 | 41.053 | 1.00 | 33.10 | C |
| ATOM | 2211 | CB | ALA | A | 327 | 38.727 | 4.939 | 40.621 | 1.00 | 24.97 | C |
| ATOM | 2212 | C | ALA | A | 327 | 38.370 | 7.306 | 39.869 | 1.00 | 33.10 | C |
| ATOM | 2213 | O | ALA | A | 327 | 39.437 | 7.863 | 39.573 | 1.00 | 33.10 | O |
| ATOM | 2214 | N | ARG | A | 328 | 37.228 | 7.497 | 39.208 | 1.00 | 33.80 | N |
| ATOM | 2215 | CA | ARG | A | 328 | 37.111 | 8.371 | 38.054 | 1.00 | 33.80 | C |
| ATOM | 2216 | CB | ARG | A | 328 | 35.703 | 8.299 | 37.494 | 1.00 | 38.44 | C |
| ATOM | 2217 | CG | ARG | A | 328 | 35.360 | 6.939 | 37.012 | 1.00 | 38.44 | C |
| ATOM | 2218 | CD | ARG | A | 328 | 33.890 | 6.800 | 36.792 | 1.00 | 38.44 | C |
| ATOM | 2219 | NE | ARG | A | 328 | 33.550 | 5.389 | 36.741 | 1.00 | 38.44 | N |
| ATOM | 2220 | CZ | ARG | A | 328 | 32.311 | 4.923 | 36.756 | 1.00 | 38.44 | C |
| ATOM | 2221 | NH1 | ARG | A | 328 | 31.287 | 5.770 | 36.812 | 1.00 | 38.44 | N |
| ATOM | 2222 | NH2 | ARG | A | 328 | 32.101 | 3.610 | 36.754 | 1.00 | 38.44 | N |
| ATOM | 2223 | C | ARG | A | 328 | 37.421 | 9.808 | 38.381 | 1.00 | 33.80 | C |
| ATOM | 2224 | O | ARG | A | 328 | 37.336 | 10.233 | 39.528 | 1.00 | 33.80 | O |
| ATOM | 2225 | N | LEU | A | 329 | 37.788 | 10.556 | 37.353 | 1.00 | 32.96 | N |
| ATOM | 2226 | CA | LEU | A | 329 | 38.075 | 11.961 | 37.523 | 1.00 | 32.96 | C |
| ATOM | 2227 | CB | LEU | A | 329 | 38.799 | 12.504 | 36.296 | 1.00 | 42.22 | C |
| ATOM | 2228 | CG | LEU | A | 329 | 40.316 | 12.656 | 36.400 | 1.00 | 42.22 | C |
| ATOM | 2229 | CD1 | LEU | A | 329 | 40.929 | 11.372 | 36.939 | 1.00 | 42.22 | C |
| ATOM | 2230 | CD2 | LEU | A | 329 | 40.884 | 13.011 | 35.034 | 1.00 | 42.22 | C |
| ATOM | 2231 | C | LEU | A | 329 | 36.759 | 12.701 | 37.715 | 1.00 | 32.96 | C |
| ATOM | 2232 | O | LEU | A | 329 | 35.681 | 12.187 | 37.416 | 1.00 | 32.96 | O |
| ATOM | 2233 | N | THR | A | 330 | 36.860 | 13.913 | 38.225 | 1.00 | 30.95 | N |
| ATOM | 2234 | CA | THR | A | 330 | 35.696 | 14.731 | 38.453 | 1.00 | 30.95 | C |
| ATOM | 2235 | CB | THR | A | 330 | 35.808 | 15.465 | 39.782 | 1.00 | 17.99 | C |
| ATOM | 2236 | OG1 | THR | A | 330 | 36.861 | 16.431 | 39.693 | 1.00 | 17.99 | O |
| ATOM | 2237 | CG2 | THR | A | 330 | 36.140 | 14.506 | 40.900 | 1.00 | 17.99 | C |
| ATOM | 2238 | C | THR | A | 330 | 35.745 | 15.757 | 37.346 | 1.00 | 30.95 | C |
| ATOM | 2239 | O | THR | A | 330 | 36.830 | 16.116 | 36.879 | 1.00 | 30.95 | O |
| ATOM | 2240 | N | PRO | A | 331 | 34.579 | 16.232 | 36.890 | 1.00 | 23.95 | N |
| ATOM | 2241 | CD | PRO | A | 331 | 33.221 | 15.818 | 37.290 | 1.00 | 22.40 | C |
| ATOM | 2242 | CA | PRO | A | 331 | 34.527 | 17.238 | 35.822 | 1.00 | 23.95 | C |
| ATOM | 2243 | CB | PRO | A | 331 | 33.145 | 17.843 | 36.014 | 1.00 | 22.40 | C |
| ATOM | 2244 | CG | PRO | A | 331 | 32.334 | 16.610 | 36.322 | 1.00 | 22.40 | C |
| ATOM | 2245 | C | PRO | A | 331 | 35.654 | 18.278 | 35.901 | 1.00 | 23.95 | C |
| ATOM | 2246 | O | PRO | A | 331 | 36.348 | 18.515 | 34.910 | 1.00 | 23.95 | O |
| ATOM | 2247 | N | LEU | A | 332 | 35.851 | 18.883 | 37.075 | 1.00 | 25.00 | N |
| ATOM | 2248 | CA | LEU | A | 332 | 36.914 | 19.886 | 37.224 | 1.00 | 25.00 | C |
| ATOM | 2249 | CB | LEU | A | 332 | 36.912 | 20.500 | 38.625 | 1.00 | 26.96 | C |
| ATOM | 2250 | CG | LEU | A | 332 | 36.204 | 21.840 | 38.801 | 1.00 | 26.96 | C |
| ATOM | 2251 | CD1 | LEU | A | 332 | 36.522 | 22.383 | 40.179 | 1.00 | 26.96 | C |
| ATOM | 2252 | CD2 | LEU | A | 332 | 36.659 | 22.817 | 37.740 | 1.00 | 26.96 | C |
| ATOM | 2253 | C | LEU | A | 332 | 38.306 | 19.323 | 36.949 | 1.00 | 25.00 | C |
| ATOM | 2254 | O | LEU | A | 332 | 39.108 | 19.902 | 36.206 | 1.00 | 25.00 | O |
| ATOM | 2255 | N | GLU | A | 333 | 38.602 | 18.192 | 37.569 | 1.00 | 39.30 | N |
| ATOM | 2256 | CA | GLU | A | 333 | 39.896 | 17.578 | 37.370 | 1.00 | 39.30 | C |
| ATOM | 2257 | CB | GLU | A | 333 | 39.975 | 16.265 | 38.151 | 1.00 | 35.74 | C |
| ATOM | 2258 | CG | GLU | A | 333 | 39.834 | 16.496 | 39.647 | 1.00 | 35.74 | C |
| ATOM | 2259 | CD | GLU | A | 333 | 39.749 | 15.223 | 40.448 | 1.00 | 35.74 | C |
| ATOM | 2260 | OE1 | GLU | A | 333 | 39.193 | 14.245 | 39.916 | 1.00 | 35.74 | O |
| ATOM | 2261 | OE2 | GLU | A | 333 | 40.213 | 15.203 | 41.616 | 1.00 | 35.74 | O |
| ATOM | 2262 | C | GLU | A | 333 | 40.098 | 17.358 | 35.882 | 1.00 | 39.30 | C |
| ATOM | 2263 | O | GLU | A | 333 | 41.071 | 17.844 | 35.305 | 1.00 | 39.30 | O |

| | | | | | | | | | | | |
|---|---|---|---|---|---|---|---|---|---|---|---|
| ATOM | 2264 | N | ALA | A | 334 | 39.159 | 16.662 | 35.253 | 1.00 | 33.96 | N |
| ATOM | 2265 | CA | ALA | A | 334 | 39.270 | 16.399 | 33.832 | 1.00 | 33.96 | C |
| ATOM | 2266 | CB | ALA | A | 334 | 37.994 | 15.766 | 33.318 | 1.00 | 13.33 | C |
| ATOM | 2267 | C | ALA | A | 334 | 39.591 | 17.666 | 33.033 | 1.00 | 33.96 | C |
| ATOM | 2268 | O | ALA | A | 334 | 40.367 | 17.608 | 32.085 | 1.00 | 33.96 | O |
| ATOM | 2269 | N | CYS | A | 335 | 39.007 | 18.805 | 33.407 | 1.00 | 23.90 | N |
| ATOM | 2270 | CA | CYS | A | 335 | 39.261 | 20.062 | 32.680 | 1.00 | 23.90 | C |
| ATOM | 2271 | CB | CYS | A | 335 | 38.398 | 21.207 | 33.211 | 1.00 | 32.10 | C |
| ATOM | 2272 | SG | CYS | A | 335 | 36.677 | 21.134 | 32.784 | 1.00 | 32.10 | S |
| ATOM | 2273 | C | CYS | A | 335 | 40.703 | 20.492 | 32.806 | 1.00 | 23.90 | C |
| ATOM | 2274 | O | CYS | A | 335 | 41.279 | 21.054 | 31.872 | 1.00 | 23.90 | O |
| ATOM | 2275 | N | ALA | A | 336 | 41.268 | 20.231 | 33.981 | 1.00 | 33.55 | N |
| ATOM | 2276 | CA | ALA | A | 336 | 42.643 | 20.602 | 34.285 | 1.00 | 33.55 | C |
| ATOM | 2277 | CB | ALA | A | 336 | 42.834 | 20.714 | 35.824 | 1.00 | 9.24 | C |
| ATOM | 2278 | C | ALA | A | 336 | 43.657 | 19.633 | 33.695 | 1.00 | 33.55 | C |
| ATOM | 2279 | O | ALA | A | 336 | 44.851 | 19.912 | 33.701 | 1.00 | 33.55 | O |
| ATOM | 2280 | N | HIS | A | 337 | 43.181 | 18.500 | 33.185 | 1.00 | 26.51 | N |
| ATOM | 2281 | CA | HIS | A | 337 | 44.061 | 17.493 | 32.593 | 1.00 | 26.51 | C |
| ATOM | 2282 | CB | HIS | A | 337 | 43.239 | 16.336 | 32.038 | 1.00 | 39.46 | C |
| ATOM | 2283 | CG | HIS | A | 337 | 44.044 | 15.108 | 31.760 | 1.00 | 39.46 | C |
| ATOM | 2284 | CD2 | HIS | A | 337 | 44.621 | 14.657 | 30.621 | 1.00 | 39.46 | C |
| ATOM | 2285 | ND1 | HIS | A | 337 | 44.336 | 14.179 | 32.733 | 1.00 | 39.46 | N |
| ATOM | 2286 | CE1 | HIS | A | 337 | 45.054 | 13.205 | 32.205 | 1.00 | 39.46 | C |
| ATOM | 2287 | NE2 | HIS | A | 337 | 45.241 | 13.471 | 30.924 | 1.00 | 39.46 | N |
| ATOM | 2288 | C | HIS | A | 337 | 44.967 | 18.064 | 31.482 | 1.00 | 26.51 | C |
| ATOM | 2289 | O | HIS | A | 337 | 44.624 | 19.045 | 30.801 | 1.00 | 26.51 | O |
| ATOM | 2290 | N | SER | A | 338 | 46.126 | 17.433 | 31.309 | 1.00 | 42.41 | N |
| ATOM | 2291 | CA | SER | A | 338 | 47.110 | 17.854 | 30.321 | 1.00 | 42.41 | C |
| ATOM | 2292 | CB | SER | A | 338 | 48.344 | 16.966 | 30.415 | 1.00 | 57.76 | C |
| ATOM | 2293 | OG | SER | A | 338 | 48.958 | 17.075 | 31.684 | 1.00 | 57.76 | O |
| ATOM | 2294 | C | SER | A | 338 | 46.584 | 17.816 | 28.895 | 1.00 | 42.41 | C |
| ATOM | 2295 | O | SER | A | 338 | 47.007 | 18.595 | 28.043 | 1.00 | 42.41 | O |
| ATOM | 2296 | N | PHE | A | 339 | 45.677 | 16.890 | 28.624 | 1.00 | 23.73 | N |
| ATOM | 2297 | CA | PHE | A | 339 | 45.112 | 16.771 | 27.288 | 1.00 | 23.73 | C |
| ATOM | 2298 | CB | PHE | A | 339 | 44.037 | 15.686 | 27.265 | 1.00 | 38.37 | C |
| ATOM | 2299 | CG | PHE | A | 339 | 43.402 | 15.500 | 25.927 | 1.00 | 38.37 | C |
| ATOM | 2300 | CD1 | PHE | A | 339 | 44.154 | 15.077 | 24.837 | 1.00 | 38.37 | C |
| ATOM | 2301 | CD2 | PHE | A | 339 | 42.056 | 15.775 | 25.742 | 1.00 | 38.37 | C |
| ATOM | 2302 | CE1 | PHE | A | 339 | 43.573 | 14.933 | 23.575 | 1.00 | 38.37 | C |
| ATOM | 2303 | CE2 | PHE | A | 339 | 41.465 | 15.634 | 24.483 | 1.00 | 38.37 | C |
| ATOM | 2304 | CZ | PHE | A | 339 | 42.228 | 15.212 | 23.398 | 1.00 | 38.37 | C |
| ATOM | 2305 | C | PHE | A | 339 | 44.526 | 18.093 | 26.802 | 1.00 | 23.73 | C |
| ATOM | 2306 | O | PHE | A | 339 | 44.490 | 18.370 | 25.603 | 1.00 | 23.73 | O |
| ATOM | 2307 | N | PHE | A | 340 | 44.078 | 18.920 | 27.734 | 1.00 | 31.39 | N |
| ATOM | 2308 | CA | PHE | A | 340 | 43.497 | 20.202 | 27.361 | 1.00 | 31.39 | C |
| ATOM | 2309 | CB | PHE | A | 340 | 42.298 | 20.504 | 28.246 | 1.00 | 38.70 | C |
| ATOM | 2310 | CG | PHE | A | 340 | 41.253 | 19.462 | 28.187 | 1.00 | 38.70 | C |
| ATOM | 2311 | CD1 | PHE | A | 340 | 40.501 | 19.296 | 27.038 | 1.00 | 38.70 | C |
| ATOM | 2312 | CD2 | PHE | A | 340 | 41.041 | 18.613 | 29.267 | 1.00 | 38.70 | C |
| ATOM | 2313 | CE1 | PHE | A | 340 | 39.547 | 18.297 | 26.963 | 1.00 | 38.70 | C |
| ATOM | 2314 | CE2 | PHE | A | 340 | 40.087 | 17.606 | 29.205 | 1.00 | 38.70 | C |
| ATOM | 2315 | CZ | PHE | A | 340 | 39.338 | 17.444 | 28.056 | 1.00 | 38.70 | C |
| ATOM | 2316 | C | PHE | A | 340 | 44.481 | 21.347 | 27.456 | 1.00 | 31.39 | C |
| ATOM | 2317 | O | PHE | A | 340 | 44.091 | 22.513 | 27.342 | 1.00 | 31.39 | O |
| ATOM | 2318 | N | ASP | A | 341 | 45.755 | 21.022 | 27.655 | 1.00 | 34.82 | N |
| ATOM | 2319 | CA | ASP | A | 341 | 46.772 | 22.059 | 27.773 | 1.00 | 34.82 | C |
| ATOM | 2320 | CB | ASP | A | 341 | 48.153 | 21.452 | 28.040 | 1.00 | 48.46 | C |
| ATOM | 2321 | CG | ASP | A | 341 | 48.291 | 20.927 | 29.459 | 1.00 | 48.46 | C |
| ATOM | 2322 | OD1 | ASP | A | 341 | 47.364 | 21.160 | 30.272 | 1.00 | 48.46 | O |
| ATOM | 2323 | OD2 | ASP | A | 341 | 49.326 | 20.289 | 29.762 | 1.00 | 48.46 | O |
| ATOM | 2324 | C | ASP | A | 341 | 46.829 | 22.976 | 26.560 | 1.00 | 34.82 | C |
| ATOM | 2325 | O | ASP | A | 341 | 47.020 | 24.189 | 26.712 | 1.00 | 34.82 | O |
| ATOM | 2326 | N | GLU | A | 342 | 46.643 | 22.419 | 25.363 | 1.00 | 31.21 | N |

| | | | | | | | | | | | |
|---|---|---|---|---|---|---|---|---|---|---|---|
| ATOM | 2327 | CA | GLU | A | 342 | 46.692 | 23.257 | 24.174 | 1.00 | 31.21 | C |
| ATOM | 2328 | CB | GLU | A | 342 | 46.430 | 22.458 | 22.896 | 1.00 | 31.06 | C |
| ATOM | 2329 | CG | GLU | A | 342 | 46.732 | 23.280 | 21.643 | 1.00 | 31.06 | C |
| ATOM | 2330 | CD | GLU | A | 342 | 46.383 | 22.578 | 20.345 | 1.00 | 31.06 | C |
| ATOM | 2331 | OE1 | GLU | A | 342 | 46.188 | 21.344 | 20.340 | 1.00 | 31.06 | O |
| ATOM | 2332 | OE2 | GLU | A | 342 | 46.317 | 23.268 | 19.313 | 1.00 | 31.06 | O |
| ATOM | 2333 | C | GLU | A | 342 | 45.674 | 24.374 | 24.280 | 1.00 | 31.21 | C |
| ATOM | 2334 | O | GLU | A | 342 | 45.945 | 25.508 | 23.875 | 1.00 | 31.21 | O |
| ATOM | 2335 | N | LEU | A | 343 | 44.507 | 24.057 | 24.835 | 1.00 | 29.10 | N |
| ATOM | 2336 | CA | LEU | A | 343 | 43.441 | 25.043 | 24.975 | 1.00 | 29.10 | C |
| ATOM | 2337 | CB | LEU | A | 343 | 42.182 | 24.395 | 25.553 | 1.00 | 26.20 | C |
| ATOM | 2338 | CG | LEU | A | 343 | 41.504 | 23.359 | 24.658 | 1.00 | 26.20 | C |
| ATOM | 2339 | CD1 | LEU | A | 343 | 40.258 | 22.861 | 25.323 | 1.00 | 26.20 | C |
| ATOM | 2340 | CD2 | LEU | A | 343 | 41.167 | 23.980 | 23.322 | 1.00 | 26.20 | C |
| ATOM | 2341 | C | LEU | A | 343 | 43.839 | 26.204 | 25.859 | 1.00 | 29.10 | C |
| ATOM | 2342 | O | LEU | A | 343 | 43.379 | 27.332 | 25.660 | 1.00 | 29.10 | O |
| ATOM | 2343 | N | ARG | A | 344 | 44.684 | 25.915 | 26.844 | 1.00 | 30.56 | N |
| ATOM | 2344 | CA | ARG | A | 344 | 45.154 | 26.927 | 27.784 | 1.00 | 30.56 | C |
| ATOM | 2345 | CB | ARG | A | 344 | 45.715 | 26.247 | 29.030 | 1.00 | 34.12 | C |
| ATOM | 2346 | CG | ARG | A | 344 | 44.712 | 26.160 | 30.171 | 1.00 | 34.12 | C |
| ATOM | 2347 | CD | ARG | A | 344 | 45.278 | 25.366 | 31.330 | 1.00 | 34.12 | C |
| ATOM | 2348 | NE | ARG | A | 344 | 45.164 | 23.931 | 31.106 | 1.00 | 34.12 | N |
| ATOM | 2349 | CZ | ARG | A | 344 | 44.029 | 23.253 | 31.210 | 1.00 | 34.12 | C |
| ATOM | 2350 | NH1 | ARG | A | 344 | 42.893 | 23.874 | 31.533 | 1.00 | 34.12 | N |
| ATOM | 2351 | NH2 | ARG | A | 344 | 44.040 | 21.944 | 31.019 | 1.00 | 34.12 | N |
| ATOM | 2352 | C | ARG | A | 344 | 46.203 | 27.861 | 27.190 | 1.00 | 30.56 | C |
| ATOM | 2353 | O | ARG | A | 344 | 46.569 | 28.879 | 27.793 | 1.00 | 30.56 | O |
| ATOM | 2354 | N | ASP | A | 345 | 46.672 | 27.501 | 26.000 | 1.00 | 29.41 | N |
| ATOM | 2355 | CA | ASP | A | 345 | 47.675 | 28.271 | 25.294 | 1.00 | 29.41 | C |
| ATOM | 2356 | CB | ASP | A | 345 | 48.088 | 27.532 | 24.031 | 1.00 | 42.08 | C |
| ATOM | 2357 | CG | ASP | A | 345 | 49.229 | 28.212 | 23.304 | 1.00 | 42.08 | C |
| ATOM | 2358 | OD1 | ASP | A | 345 | 50.268 | 27.547 | 23.118 | 1.00 | 42.08 | O |
| ATOM | 2359 | OD2 | ASP | A | 345 | 49.096 | 29.398 | 22.917 | 1.00 | 42.08 | O |
| ATOM | 2360 | C | ASP | A | 345 | 47.088 | 29.623 | 24.932 | 1.00 | 29.41 | C |
| ATOM | 2361 | O | ASP | A | 345 | 45.916 | 29.722 | 24.564 | 1.00 | 29.41 | O |
| ATOM | 2362 | N | PRO | A | 346 | 47.893 | 30.687 | 25.035 | 1.00 | 50.05 | N |
| ATOM | 2363 | CD | PRO | A | 346 | 49.242 | 30.727 | 25.630 | 1.00 | 36.94 | C |
| ATOM | 2364 | CA | PRO | A | 346 | 47.417 | 32.033 | 24.710 | 1.00 | 50.05 | C |
| ATOM | 2365 | CB | PRO | A | 346 | 48.453 | 32.926 | 25.379 | 1.00 | 36.94 | C |
| ATOM | 2366 | CG | PRO | A | 346 | 49.714 | 32.107 | 25.266 | 1.00 | 36.94 | C |
| ATOM | 2367 | C | PRO | A | 346 | 47.256 | 32.335 | 23.212 | 1.00 | 50.05 | C |
| ATOM | 2368 | O | PRO | A | 346 | 46.734 | 33.384 | 22.855 | 1.00 | 50.05 | O |
| ATOM | 2369 | N | ASN | A | 347 | 47.687 | 31.426 | 22.339 | 1.00 | 44.11 | N |
| ATOM | 2370 | CA | ASN | A | 347 | 47.576 | 31.651 | 20.894 | 1.00 | 44.11 | C |
| ATOM | 2371 | CB | ASN | A | 347 | 48.950 | 31.582 | 20.251 | 1.00 | 39.11 | C |
| ATOM | 2372 | CG | ASN | A | 347 | 49.889 | 32.624 | 20.792 | 1.00 | 39.11 | C |
| ATOM | 2373 | OD1 | ASN | A | 347 | 49.626 | 33.827 | 20.705 | 1.00 | 39.11 | O |
| ATOM | 2374 | ND2 | ASN | A | 347 | 50.998 | 32.174 | 21.358 | 1.00 | 39.11 | N |
| ATOM | 2375 | C | ASN | A | 347 | 46.646 | 30.694 | 20.148 | 1.00 | 44.11 | C |
| ATOM | 2376 | O | ASN | A | 347 | 46.223 | 30.974 | 19.026 | 1.00 | 44.11 | O |
| ATOM | 2377 | N | VAL | A | 348 | 46.351 | 29.558 | 20.764 | 1.00 | 49.39 | N |
| ATOM | 2378 | CA | VAL | A | 348 | 45.470 | 28.582 | 20.157 | 1.00 | 49.39 | C |
| ATOM | 2379 | CB | VAL | A | 348 | 44.987 | 27.552 | 21.188 | 1.00 | 33.81 | C |
| ATOM | 2380 | CG1 | VAL | A | 348 | 44.343 | 28.266 | 22.372 | 1.00 | 33.81 | C |
| ATOM | 2381 | CG2 | VAL | A | 348 | 43.999 | 26.595 | 20.534 | 1.00 | 33.81 | C |
| ATOM | 2382 | C | VAL | A | 348 | 44.246 | 29.249 | 19.548 | 1.00 | 49.39 | C |
| ATOM | 2383 | O | VAL | A | 348 | 43.542 | 30.022 | 20.194 | 1.00 | 49.39 | O |
| ATOM | 2384 | N | LYS | A | 349 | 44.000 | 28.943 | 18.286 | 1.00 | 35.34 | N |
| ATOM | 2385 | CA | LYS | A | 349 | 42.852 | 29.490 | 17.594 | 1.00 | 35.34 | C |
| ATOM | 2386 | CB | LYS | A | 349 | 43.280 | 30.640 | 16.684 | 1.00 | 73.38 | C |
| ATOM | 2387 | CG | LYS | A | 349 | 43.704 | 31.905 | 17.410 | 1.00 | 73.38 | C |
| ATOM | 2388 | CD | LYS | A | 349 | 44.378 | 32.880 | 16.450 | 1.00 | 73.38 | C |
| ATOM | 2389 | CE | LYS | A | 349 | 45.645 | 32.265 | 15.856 | 1.00 | 73.38 | C |

| | | | | | | | | | | | |
|---|---|---|---|---|---|---|---|---|---|---|---|
| ATOM | 2390 | NZ | LYS | A | 349 | 46.361 | 33.176 | 14.924 | 1.00 | 73.38 | N |
| ATOM | 2391 | C | LYS | A | 349 | 42.278 | 28.355 | 16.763 | 1.00 | 35.34 | C |
| ATOM | 2392 | O | LYS | A | 349 | 42.966 | 27.364 | 16.519 | 1.00 | 35.34 | O |
| ATOM | 2393 | N | LEU | A | 350 | 41.024 | 28.506 | 16.336 | 1.00 | 40.56 | N |
| ATOM | 2394 | CA | LEU | A | 350 | 40.339 | 27.508 | 15.528 | 1.00 | 40.56 | C |
| ATOM | 2395 | CB | LEU | A | 350 | 38.868 | 27.917 | 15.349 | 1.00 | 42.75 | C |
| ATOM | 2396 | CG | LEU | A | 350 | 38.000 | 28.154 | 16.589 | 1.00 | 42.75 | C |
| ATOM | 2397 | CD1 | LEU | A | 350 | 36.673 | 28.767 | 16.157 | 1.00 | 42.75 | C |
| ATOM | 2398 | CD2 | LEU | A | 350 | 37.766 | 26.847 | 17.306 | 1.00 | 42.75 | C |
| ATOM | 2399 | C | LEU | A | 350 | 40.997 | 27.425 | 14.150 | 1.00 | 40.56 | C |
| ATOM | 2400 | O | LEU | A | 350 | 41.420 | 28.433 | 13.593 | 1.00 | 40.56 | O |
| ATOM | 2401 | N | PRO | A | 351 | 41.077 | 26.223 | 13.571 | 1.00 | 40.85 | N |
| ATOM | 2402 | CD | PRO | A | 351 | 40.421 | 24.965 | 13.921 | 1.00 | 56.73 | C |
| ATOM | 2403 | CA | PRO | A | 351 | 41.709 | 26.162 | 12.256 | 1.00 | 40.85 | C |
| ATOM | 2404 | CB | PRO | A | 351 | 41.816 | 24.671 | 11.967 | 1.00 | 56.73 | C |
| ATOM | 2405 | CG | PRO | A | 351 | 41.070 | 24.000 | 13.040 | 1.00 | 56.73 | C |
| ATOM | 2406 | C | PRO | A | 351 | 40.830 | 26.862 | 11.280 | 1.00 | 40.85 | C |
| ATOM | 2407 | O | PRO | A | 351 | 41.162 | 26.975 | 10.113 | 1.00 | 40.85 | O |
| ATOM | 2408 | N | ASN | A | 352 | 39.722 | 27.366 | 11.790 | 1.00 | 42.11 | N |
| ATOM | 2409 | CA | ASN | A | 352 | 38.772 | 28.085 | 10.973 | 1.00 | 42.11 | C |
| ATOM | 2410 | CB | ASN | A | 352 | 37.360 | 27.880 | 11.524 | 1.00 | 45.39 | C |
| ATOM | 2411 | CG | ASN | A | 352 | 36.630 | 29.179 | 11.756 | 1.00 | 45.39 | C |
| ATOM | 2412 | OD1 | ASN | A | 352 | 37.039 | 29.983 | 12.588 | 1.00 | 45.39 | O |
| ATOM | 2413 | ND2 | ASN | A | 352 | 35.547 | 29.399 | 11.017 | 1.00 | 45.39 | N |
| ATOM | 2414 | C | ASN | A | 352 | 39.114 | 29.569 | 10.910 | 1.00 | 42.11 | C |
| ATOM | 2415 | O | ASN | A | 352 | 38.403 | 30.361 | 10.283 | 1.00 | 42.11 | O |
| ATOM | 2416 | N | GLY | A | 353 | 40.212 | 29.943 | 11.549 | 1.00 | 41.59 | N |
| ATOM | 2417 | CA | GLY | A | 353 | 40.602 | 31.336 | 11.552 | 1.00 | 41.59 | C |
| ATOM | 2418 | C | GLY | A | 353 | 40.403 | 32.020 | 12.900 | 1.00 | 41.59 | C |
| ATOM | 2419 | O | GLY | A | 353 | 41.377 | 32.381 | 13.554 | 1.00 | 41.59 | O |
| ATOM | 2420 | N | ARG | A | 354 | 39.152 | 32.204 | 13.319 | 1.00 | 43.09 | N |
| ATOM | 2421 | CA | ARG | A | 354 | 38.848 | 32.893 | 14.579 | 1.00 | 43.09 | C |
| ATOM | 2422 | CB | ARG | A | 354 | 37.334 | 33.097 | 14.712 | 1.00 | 55.05 | C |
| ATOM | 2423 | CG | ARG | A | 354 | 36.531 | 31.826 | 14.785 | 1.00 | 55.05 | C |
| ATOM | 2424 | CD | ARG | A | 354 | 35.056 | 32.129 | 14.970 | 1.00 | 55.05 | C |
| ATOM | 2425 | NE | ARG | A | 354 | 34.507 | 32.872 | 13.840 | 1.00 | 55.05 | N |
| ATOM | 2426 | CZ | ARG | A | 354 | 33.235 | 33.252 | 13.742 | 1.00 | 55.05 | C |
| ATOM | 2427 | NH1 | ARG | A | 354 | 32.371 | 32.955 | 14.706 | 1.00 | 55.05 | N |
| ATOM | 2428 | NH2 | ARG | A | 354 | 32.824 | 33.946 | 12.690 | 1.00 | 55.05 | N |
| ATOM | 2429 | C | ARG | A | 354 | 39.385 | 32.269 | 15.872 | 1.00 | 43.09 | C |
| ATOM | 2430 | O | ARG | A | 354 | 39.877 | 31.134 | 15.895 | 1.00 | 43.09 | O |
| ATOM | 2431 | N | ASP | A | 355 | 39.299 | 33.043 | 16.951 | 1.00 | 68.70 | N |
| ATOM | 2432 | CA | ASP | A | 355 | 39.752 | 32.575 | 18.248 | 1.00 | 68.70 | C |
| ATOM | 2433 | CB | ASP | A | 355 | 40.025 | 33.749 | 19.218 | 1.00 | 86.20 | C |
| ATOM | 2434 | CG | ASP | A | 355 | 39.164 | 34.984 | 18.943 | 1.00 | 86.20 | C |
| ATOM | 2435 | OD1 | ASP | A | 355 | 38.085 | 34.870 | 18.330 | 1.00 | 86.20 | O |
| ATOM | 2436 | OD2 | ASP | A | 355 | 39.570 | 36.088 | 19.368 | 1.00 | 86.20 | O |
| ATOM | 2437 | C | ASP | A | 355 | 38.732 | 31.618 | 18.853 | 1.00 | 68.70 | C |
| ATOM | 2438 | O | ASP | A | 355 | 37.584 | 31.545 | 18.405 | 1.00 | 68.70 | O |
| ATOM | 2439 | N | THR | A | 356 | 39.165 | 30.869 | 19.861 | 1.00 | 39.43 | N |
| ATOM | 2440 | CA | THR | A | 356 | 38.293 | 29.911 | 20.529 | 1.00 | 39.43 | C |
| ATOM | 2441 | CB | THR | A | 356 | 39.087 | 28.946 | 21.425 | 1.00 | 61.06 | C |
| ATOM | 2442 | OG1 | THR | A | 356 | 39.497 | 29.629 | 22.618 | 1.00 | 61.06 | O |
| ATOM | 2443 | CG2 | THR | A | 356 | 40.308 | 28.428 | 20.694 | 1.00 | 61.06 | C |
| ATOM | 2444 | C | THR | A | 356 | 37.286 | 30.638 | 21.420 | 1.00 | 39.43 | C |
| ATOM | 2445 | O | THR | A | 356 | 37.413 | 31.837 | 21.684 | 1.00 | 39.43 | O |
| ATOM | 2446 | N | PRO | A | 357 | 36.259 | 29.915 | 21.885 | 1.00 | 44.47 | N |
| ATOM | 2447 | CD | PRO | A | 357 | 35.902 | 28.546 | 21.457 | 1.00 | 30.18 | C |
| ATOM | 2448 | CA | PRO | A | 357 | 35.225 | 30.490 | 22.753 | 1.00 | 44.47 | C |
| ATOM | 2449 | CB | PRO | A | 357 | 34.094 | 29.474 | 22.659 | 1.00 | 30.18 | C |
| ATOM | 2450 | CG | PRO | A | 357 | 34.827 | 28.161 | 22.447 | 1.00 | 30.18 | C |
| ATOM | 2451 | C | PRO | A | 357 | 35.704 | 30.682 | 24.185 | 1.00 | 44.47 | C |
| ATOM | 2452 | O | PRO | A | 357 | 36.864 | 30.401 | 24.504 | 1.00 | 44.47 | O |

| | | | | | | | | | | | |
|---|---|---|---|---|---|---|---|---|---|---|---|
| ATOM | 2453 | N | ALA | A | 358 | 34.808 | 31.160 | 25.044 | 1.00 | 46.92 | N |
| ATOM | 2454 | CA | ALA | A | 358 | 35.145 | 31.382 | 26.447 | 1.00 | 46.92 | C |
| ATOM | 2455 | CB | ALA | A | 358 | 34.014 | 32.094 | 27.150 | 1.00 | 46.66 | C |
| ATOM | 2456 | C | ALA | A | 358 | 35.415 | 30.050 | 27.122 | 1.00 | 46.92 | C |
| ATOM | 2457 | O | ALA | A | 358 | 34.570 | 29.159 | 27.097 | 1.00 | 46.92 | O |
| ATOM | 2458 | N | LEU | A | 359 | 36.586 | 29.911 | 27.736 | 1.00 | 25.84 | N |
| ATOM | 2459 | CA | LEU | A | 359 | 36.929 | 28.654 | 28.384 | 1.00 | 25.84 | C |
| ATOM | 2460 | CB | LEU | A | 359 | 37.962 | 27.911 | 27.535 | 1.00 | 16.03 | C |
| ATOM | 2461 | CG | LEU | A | 359 | 37.651 | 27.849 | 26.036 | 1.00 | 16.03 | C |
| ATOM | 2462 | CD1 | LEU | A | 359 | 38.846 | 27.325 | 25.291 | 1.00 | 16.03 | C |
| ATOM | 2463 | CD2 | LEU | A | 359 | 36.431 | 26.997 | 25.796 | 1.00 | 16.03 | C |
| ATOM | 2464 | C | LEU | A | 359 | 37.476 | 28.821 | 29.796 | 1.00 | 25.84 | C |
| ATOM | 2465 | O | LEU | A | 359 | 37.634 | 27.837 | 30.528 | 1.00 | 25.84 | O |
| ATOM | 2466 | N | PHE | A | 360 | 37.743 | 30.062 | 30.195 | 1.00 | 37.29 | N |
| ATOM | 2467 | CA | PHE | A | 360 | 38.325 | 30.297 | 31.514 | 1.00 | 37.29 | C |
| ATOM | 2468 | CB | PHE | A | 360 | 39.739 | 30.852 | 31.344 | 1.00 | 32.66 | C |
| ATOM | 2469 | CG | PHE | A | 360 | 40.553 | 30.089 | 30.366 | 1.00 | 32.66 | C |
| ATOM | 2470 | CD1 | PHE | A | 360 | 40.880 | 28.759 | 30.616 | 1.00 | 32.66 | C |
| ATOM | 2471 | CD2 | PHE | A | 360 | 40.930 | 30.670 | 29.160 | 1.00 | 32.66 | C |
| ATOM | 2472 | CE1 | PHE | A | 360 | 41.571 | 28.011 | 29.669 | 1.00 | 32.66 | C |
| ATOM | 2473 | CE2 | PHE | A | 360 | 41.616 | 29.941 | 28.207 | 1.00 | 32.66 | C |
| ATOM | 2474 | CZ | PHE | A | 360 | 41.941 | 28.605 | 28.454 | 1.00 | 32.66 | C |
| ATOM | 2475 | C | PHE | A | 360 | 37.551 | 31.197 | 32.462 | 1.00 | 37.29 | C |
| ATOM | 2476 | O | PHE | A | 360 | 38.084 | 31.619 | 33.494 | 1.00 | 37.29 | O |
| ATOM | 2477 | N | ASN | A | 361 | 36.305 | 31.495 | 32.117 | 1.00 | 34.80 | N |
| ATOM | 2478 | CA | ASN | A | 361 | 35.480 | 32.339 | 32.967 | 1.00 | 34.80 | C |
| ATOM | 2479 | CB | ASN | A | 361 | 34.514 | 33.165 | 32.107 | 1.00 | 39.17 | C |
| ATOM | 2480 | CG | ASN | A | 361 | 33.619 | 32.308 | 31.228 | 1.00 | 39.17 | C |
| ATOM | 2481 | OD1 | ASN | A | 361 | 33.991 | 31.208 | 30.822 | 1.00 | 39.17 | O |
| ATOM | 2482 | ND2 | ASN | A | 361 | 32.437 | 32.823 | 30.912 | 1.00 | 39.17 | N |
| ATOM | 2483 | C | ASN | A | 361 | 34.726 | 31.499 | 34.006 | 1.00 | 34.80 | C |
| ATOM | 2484 | O | ASN | A | 361 | 33.499 | 31.539 | 34.107 | 1.00 | 34.80 | O |
| ATOM | 2485 | N | PHE | A | 362 | 35.491 | 30.737 | 34.778 | 1.00 | 33.97 | N |
| ATOM | 2486 | CA | PHE | A | 362 | 34.958 | 29.879 | 35.823 | 1.00 | 33.97 | C |
| ATOM | 2487 | CB | PHE | A | 362 | 36.045 | 28.959 | 36.357 | 1.00 | 34.37 | C |
| ATOM | 2488 | CG | PHE | A | 362 | 36.377 | 27.837 | 35.463 | 1.00 | 34.37 | C |
| ATOM | 2489 | CD1 | PHE | A | 362 | 35.676 | 26.642 | 35.554 | 1.00 | 34.37 | C |
| ATOM | 2490 | CD2 | PHE | A | 362 | 37.393 | 27.967 | 34.525 | 1.00 | 34.37 | C |
| ATOM | 2491 | CE1 | PHE | A | 362 | 35.980 | 25.579 | 34.721 | 1.00 | 34.37 | C |
| ATOM | 2492 | CE2 | PHE | A | 362 | 37.710 | 26.924 | 33.688 | 1.00 | 34.37 | C |
| ATOM | 2493 | CZ | PHE | A | 362 | 36.999 | 25.715 | 33.785 | 1.00 | 34.37 | C |
| ATOM | 2494 | C | PHE | A | 362 | 34.476 | 30.705 | 36.988 | 1.00 | 33.97 | C |
| ATOM | 2495 | O | PHE | A | 362 | 35.220 | 31.532 | 37.500 | 1.00 | 33.97 | O |
| ATOM | 2496 | N | THR | A | 363 | 33.250 | 30.458 | 37.425 | 1.00 | 32.52 | N |
| ATOM | 2497 | CA | THR | A | 363 | 32.694 | 31.176 | 38.553 | 1.00 | 32.52 | C |
| ATOM | 2498 | CB | THR | A | 363 | 31.176 | 31.276 | 38.471 | 1.00 | 21.33 | C |
| ATOM | 2499 | OG1 | THR | A | 363 | 30.598 | 29.962 | 38.485 | 1.00 | 21.33 | O |
| ATOM | 2500 | CG2 | THR | A | 363 | 30.781 | 32.042 | 37.218 | 1.00 | 21.33 | C |
| ATOM | 2501 | C | THR | A | 363 | 33.048 | 30.440 | 39.821 | 1.00 | 32.52 | C |
| ATOM | 2502 | O | THR | A | 363 | 33.293 | 29.237 | 39.798 | 1.00 | 32.52 | O |
| ATOM | 2503 | N | THR | A | 364 | 33.067 | 31.163 | 40.933 | 1.00 | 30.27 | N |
| ATOM | 2504 | CA | THR | A | 364 | 33.400 | 30.566 | 42.217 | 1.00 | 30.27 | C |
| ATOM | 2505 | CB | THR | A | 364 | 33.125 | 31.621 | 43.328 | 1.00 | 27.41 | C |
| ATOM | 2506 | OG1 | THR | A | 364 | 33.012 | 30.985 | 44.604 | 1.00 | 27.41 | O |
| ATOM | 2507 | CG2 | THR | A | 364 | 31.872 | 32.417 | 42.995 | 1.00 | 27.41 | C |
| ATOM | 2508 | C | THR | A | 364 | 32.656 | 29.211 | 42.440 | 1.00 | 30.27 | C |
| ATOM | 2509 | O | THR | A | 364 | 33.262 | 28.194 | 42.825 | 1.00 | 30.27 | O |
| ATOM | 2510 | N | GLN | A | 365 | 31.356 | 29.196 | 42.154 | 1.00 | 29.93 | N |
| ATOM | 2511 | CA | GLN | A | 365 | 30.514 | 27.996 | 42.288 | 1.00 | 29.93 | C |
| ATOM | 2512 | CB | GLN | A | 365 | 29.099 | 28.332 | 41.817 | 1.00 | 35.17 | C |
| ATOM | 2513 | CG | GLN | A | 365 | 28.207 | 27.145 | 41.516 | 1.00 | 35.17 | C |
| ATOM | 2514 | CD | GLN | A | 365 | 27.319 | 26.783 | 42.687 | 1.00 | 35.17 | C |
| ATOM | 2515 | OE1 | GLN | A | 365 | 26.772 | 27.667 | 43.350 | 1.00 | 35.17 | O |

| | | | | | | | | | | | |
|---|---|---|---|---|---|---|---|---|---|---|---|
| ATOM | 2516 | NE2 | GLN | A | 365 | 27.153 | 25.483 | 42.940 | 1.00 | 35.17 | N |
| ATOM | 2517 | C | GLN | A | 365 | 31.049 | 26.832 | 41.454 | 1.00 | 29.93 | C |
| ATOM | 2518 | O | GLN | A | 365 | 31.197 | 25.705 | 41.924 | 1.00 | 29.93 | O |
| ATOM | 2519 | N | GLU | A | 366 | 31.313 | 27.140 | 40.195 | 1.00 | 29.68 | N |
| ATOM | 2520 | CA | GLU | A | 366 | 31.826 | 26.189 | 39.222 | 1.00 | 29.68 | C |
| ATOM | 2521 | CB | GLU | A | 366 | 32.073 | 26.938 | 37.908 | 1.00 | 41.27 | C |
| ATOM | 2522 | CG | GLU | A | 366 | 31.795 | 26.150 | 36.657 | 1.00 | 41.27 | C |
| ATOM | 2523 | CD | GLU | A | 366 | 32.017 | 26.973 | 35.418 | 1.00 | 41.27 | C |
| ATOM | 2524 | OE1 | GLU | A | 366 | 32.391 | 26.375 | 34.389 | 1.00 | 41.27 | O |
| ATOM | 2525 | OE2 | GLU | A | 366 | 31.810 | 28.211 | 35.468 | 1.00 | 41.27 | O |
| ATOM | 2526 | C | GLU | A | 366 | 33.127 | 25.520 | 39.698 | 1.00 | 29.68 | C |
| ATOM | 2527 | O | GLU | A | 366 | 33.487 | 24.420 | 39.260 | 1.00 | 29.68 | O |
| ATOM | 2528 | N | LEU | A | 367 | 33.834 | 26.203 | 40.588 | 1.00 | 30.00 | N |
| ATOM | 2529 | CA | LEU | A | 367 | 35.085 | 25.690 | 41.112 | 1.00 | 30.00 | C |
| ATOM | 2530 | CB | LEU | A | 367 | 36.096 | 26.834 | 41.232 | 1.00 | 31.99 | C |
| ATOM | 2531 | CG | LEU | A | 367 | 36.624 | 27.216 | 39.849 | 1.00 | 31.99 | C |
| ATOM | 2532 | CD1 | LEU | A | 367 | 37.348 | 28.533 | 39.879 | 1.00 | 31.99 | C |
| ATOM | 2533 | CD2 | LEU | A | 367 | 37.546 | 26.111 | 39.372 | 1.00 | 31.99 | C |
| ATOM | 2534 | C | LEU | A | 367 | 34.873 | 25.011 | 42.455 | 1.00 | 30.00 | C |
| ATOM | 2535 | O | LEU | A | 367 | 35.670 | 24.171 | 42.865 | 1.00 | 30.00 | O |
| ATOM | 2536 | N | SER | A | 368 | 33.779 | 25.373 | 43.117 | 1.00 | 29.55 | N |
| ATOM | 2537 | CA | SER | A | 368 | 33.414 | 24.832 | 44.423 | 1.00 | 29.55 | C |
| ATOM | 2538 | CB | SER | A | 368 | 31.889 | 24.788 | 44.546 | 1.00 | 33.45 | C |
| ATOM | 2539 | OG | SER | A | 368 | 31.356 | 23.791 | 43.696 | 1.00 | 33.45 | O |
| ATOM | 2540 | C | SER | A | 368 | 33.991 | 23.460 | 44.858 | 1.00 | 29.55 | C |
| ATOM | 2541 | O | SER | A | 368 | 34.470 | 23.337 | 45.988 | 1.00 | 29.55 | O |
| ATOM | 2542 | N | SER | A | 369 | 33.953 | 22.438 | 43.997 | 1.00 | 29.73 | N |
| ATOM | 2543 | CA | SER | A | 369 | 34.449 | 21.104 | 44.384 | 1.00 | 29.73 | C |
| ATOM | 2544 | CB | SER | A | 369 | 34.083 | 20.052 | 43.332 | 1.00 | 41.05 | C |
| ATOM | 2545 | OG | SER | A | 369 | 34.814 | 20.233 | 42.131 | 1.00 | 41.05 | O |
| ATOM | 2546 | C | SER | A | 369 | 35.949 | 21.016 | 44.654 | 1.00 | 29.73 | C |
| ATOM | 2547 | O | SER | A | 369 | 36.415 | 20.084 | 45.306 | 1.00 | 29.73 | O |
| ATOM | 2548 | N | ASN | A | 370 | 36.711 | 21.974 | 44.147 | 1.00 | 28.55 | N |
| ATOM | 2549 | CA | ASN | A | 370 | 38.143 | 21.976 | 44.359 | 1.00 | 28.55 | C |
| ATOM | 2550 | CB | ASN | A | 370 | 38.768 | 20.814 | 43.592 | 1.00 | 33.26 | C |
| ATOM | 2551 | CG | ASN | A | 370 | 40.166 | 20.494 | 44.065 | 1.00 | 33.26 | C |
| ATOM | 2552 | OD1 | ASN | A | 370 | 40.999 | 21.383 | 44.206 | 1.00 | 33.26 | O |
| ATOM | 2553 | ND2 | ASN | A | 370 | 40.433 | 19.218 | 44.306 | 1.00 | 33.26 | N |
| ATOM | 2554 | C | ASN | A | 370 | 38.689 | 23.321 | 43.865 | 1.00 | 28.55 | C |
| ATOM | 2555 | O | ASN | A | 370 | 39.221 | 23.432 | 42.762 | 1.00 | 28.55 | O |
| ATOM | 2556 | N | PRO | A | 371 | 38.535 | 24.374 | 44.686 | 1.00 | 42.14 | N |
| ATOM | 2557 | CD | PRO | A | 371 | 37.771 | 24.358 | 45.945 | 1.00 | 29.58 | C |
| ATOM | 2558 | CA | PRO | A | 371 | 38.984 | 25.734 | 44.388 | 1.00 | 42.14 | C |
| ATOM | 2559 | CB | PRO | A | 371 | 38.679 | 26.476 | 45.673 | 1.00 | 29.58 | C |
| ATOM | 2560 | CG | PRO | A | 371 | 37.424 | 25.816 | 46.111 | 1.00 | 29.58 | C |
| ATOM | 2561 | C | PRO | A | 371 | 40.433 | 25.877 | 43.976 | 1.00 | 42.14 | C |
| ATOM | 2562 | O | PRO | A | 371 | 40.737 | 26.670 | 43.099 | 1.00 | 42.14 | O |
| ATOM | 2563 | N | PRO | A | 372 | 41.350 | 25.118 | 44.601 | 1.00 | 45.38 | N |
| ATOM | 2564 | CD | PRO | A | 372 | 41.194 | 24.202 | 45.744 | 1.00 | 44.11 | C |
| ATOM | 2565 | CA | PRO | A | 372 | 42.761 | 25.226 | 44.233 | 1.00 | 45.38 | C |
| ATOM | 2566 | CB | PRO | A | 372 | 43.466 | 24.489 | 45.370 | 1.00 | 44.11 | C |
| ATOM | 2567 | CG | PRO | A | 372 | 42.508 | 23.449 | 45.732 | 1.00 | 44.11 | C |
| ATOM | 2568 | C | PRO | A | 372 | 43.142 | 24.693 | 42.846 | 1.00 | 45.38 | C |
| ATOM | 2569 | O | PRO | A | 372 | 44.305 | 24.785 | 42.449 | 1.00 | 45.38 | O |
| ATOM | 2570 | N | LEU | A | 373 | 42.184 | 24.129 | 42.114 | 1.00 | 30.67 | N |
| ATOM | 2571 | CA | LEU | A | 373 | 42.469 | 23.645 | 40.761 | 1.00 | 30.67 | C |
| ATOM | 2572 | CB | LEU | A | 373 | 41.425 | 22.639 | 40.291 | 1.00 | 32.38 | C |
| ATOM | 2573 | CG | LEU | A | 373 | 41.415 | 21.257 | 40.928 | 1.00 | 32.38 | C |
| ATOM | 2574 | CD1 | LEU | A | 373 | 40.256 | 20.468 | 40.373 | 1.00 | 32.38 | C |
| ATOM | 2575 | CD2 | LEU | A | 373 | 42.708 | 20.539 | 40.635 | 1.00 | 32.38 | C |
| ATOM | 2576 | C | LEU | A | 373 | 42.440 | 24.822 | 39.800 | 1.00 | 30.67 | C |
| ATOM | 2577 | O | LEU | A | 373 | 42.810 | 24.680 | 38.638 | 1.00 | 30.67 | O |
| ATOM | 2578 | N | ALA | A | 374 | 41.988 | 25.975 | 40.292 | 1.00 | 40.84 | N |

| | | | | | | | | | | | |
|---|---|---|---|---|---|---|---|---|---|---|---|
| ATOM | 2579 | CA | ALA | A | 374 | 41.878 | 27.188 | 39.483 | 1.00 | 40.84 | C |
| ATOM | 2580 | CB | ALA | A | 374 | 41.044 | 28.251 | 40.223 | 1.00 | 19.66 | C |
| ATOM | 2581 | C | ALA | A | 374 | 43.226 | 27.769 | 39.087 | 1.00 | 40.84 | C |
| ATOM | 2582 | O | ALA | A | 374 | 43.328 | 28.489 | 38.090 | 1.00 | 40.84 | O |
| ATOM | 2583 | N | THR | A | 375 | 44.263 | 27.464 | 39.858 | 1.00 | 43.92 | N |
| ATOM | 2584 | CA | THR | A | 375 | 45.590 | 27.977 | 39.535 | 1.00 | 43.92 | C |
| ATOM | 2585 | CB | THR | A | 375 | 46.575 | 27.779 | 40.724 | 1.00 | 44.87 | C |
| ATOM | 2586 | OG1 | THR | A | 375 | 46.694 | 26.390 | 41.037 | 1.00 | 44.87 | O |
| ATOM | 2587 | CG2 | THR | A | 375 | 46.049 | 28.473 | 41.960 | 1.00 | 44.87 | C |
| ATOM | 2588 | C | THR | A | 375 | 46.105 | 27.280 | 38.274 | 1.00 | 43.92 | C |
| ATOM | 2589 | O | THR | A | 375 | 47.161 | 27.631 | 37.756 | 1.00 | 43.92 | O |
| ATOM | 2590 | N | ILE | A | 376 | 45.340 | 26.296 | 37.791 | 1.00 | 50.95 | N |
| ATOM | 2591 | CA | ILE | A | 376 | 45.665 | 25.542 | 36.572 | 1.00 | 50.95 | C |
| ATOM | 2592 | CB | ILE | A | 376 | 45.656 | 24.009 | 36.798 | 1.00 | 15.54 | C |
| ATOM | 2593 | CG2 | ILE | A | 376 | 45.845 | 23.274 | 35.462 | 1.00 | 15.54 | C |
| ATOM | 2594 | CG1 | ILE | A | 376 | 46.756 | 23.609 | 37.769 | 1.00 | 15.54 | C |
| ATOM | 2595 | CD1 | ILE | A | 376 | 46.914 | 22.096 | 37.873 | 1.00 | 15.54 | C |
| ATOM | 2596 | C | ILE | A | 376 | 44.634 | 25.820 | 35.480 | 1.00 | 50.95 | C |
| ATOM | 2597 | O | ILE | A | 376 | 44.975 | 25.998 | 34.310 | 1.00 | 50.95 | O |
| ATOM | 2598 | N | LEU | A | 377 | 43.368 | 25.826 | 35.873 | 1.00 | 41.31 | N |
| ATOM | 2599 | CA | LEU | A | 377 | 42.274 | 26.069 | 34.950 | 1.00 | 41.31 | C |
| ATOM | 2600 | CB | LEU | A | 377 | 40.948 | 25.810 | 35.661 | 1.00 | 21.36 | C |
| ATOM | 2601 | CG | LEU | A | 377 | 40.707 | 24.344 | 36.042 | 1.00 | 21.36 | C |
| ATOM | 2602 | CD1 | LEU | A | 377 | 39.605 | 24.236 | 37.070 | 1.00 | 21.36 | C |
| ATOM | 2603 | CD2 | LEU | A | 377 | 40.357 | 23.553 | 34.800 | 1.00 | 21.36 | C |
| ATOM | 2604 | C | LEU | A | 377 | 42.304 | 27.488 | 34.390 | 1.00 | 41.31 | C |
| ATOM | 2605 | O | LEU | A | 377 | 41.889 | 27.719 | 33.251 | 1.00 | 41.31 | O |
| ATOM | 2606 | N | ILE | A | 378 | 42.785 | 28.438 | 35.187 | 1.00 | 47.30 | N |
| ATOM | 2607 | CA | ILE | A | 378 | 42.860 | 29.818 | 34.728 | 1.00 | 47.30 | C |
| ATOM | 2608 | CB | ILE | A | 378 | 42.256 | 30.796 | 35.761 | 1.00 | 45.25 | C |
| ATOM | 2609 | CG2 | ILE | A | 378 | 42.033 | 32.163 | 35.123 | 1.00 | 45.25 | C |
| ATOM | 2610 | CG1 | ILE | A | 378 | 40.911 | 30.260 | 36.243 | 1.00 | 45.25 | C |
| ATOM | 2611 | CD1 | ILE | A | 378 | 40.244 | 31.127 | 37.283 | 1.00 | 45.25 | C |
| ATOM | 2612 | C | ILE | A | 378 | 44.308 | 30.215 | 34.459 | 1.00 | 47.30 | C |
| ATOM | 2613 | O | ILE | A | 378 | 45.052 | 30.549 | 35.380 | 1.00 | 47.30 | O |
| ATOM | 2614 | N | PRO | A | 379 | 44.727 | 30.174 | 33.185 | 1.00 | 29.86 | N |
| ATOM | 2615 | CD | PRO | A | 379 | 43.930 | 29.853 | 31.990 | 1.00 | 35.20 | C |
| ATOM | 2616 | CA | PRO | A | 379 | 46.094 | 30.535 | 32.809 | 1.00 | 29.86 | C |
| ATOM | 2617 | CB | PRO | A | 379 | 46.143 | 30.174 | 31.328 | 1.00 | 35.20 | C |
| ATOM | 2618 | CG | PRO | A | 379 | 44.758 | 30.467 | 30.880 | 1.00 | 35.20 | C |
| ATOM | 2619 | C | PRO | A | 379 | 46.361 | 32.021 | 33.069 | 1.00 | 29.86 | C |
| ATOM | 2620 | O | PRO | A | 379 | 45.446 | 32.841 | 33.056 | 1.00 | 29.86 | O |
| ATOM | 2621 | N | PRO | A | 380 | 47.630 | 32.382 | 33.294 | 1.00 | 45.90 | N |
| ATOM | 2622 | CD | PRO | A | 380 | 48.823 | 31.548 | 33.047 | 1.00 | 35.29 | C |
| ATOM | 2623 | CA | PRO | A | 380 | 48.011 | 33.772 | 33.564 | 1.00 | 45.90 | C |
| ATOM | 2624 | CB | PRO | A | 380 | 49.497 | 33.786 | 33.225 | 1.00 | 35.29 | C |
| ATOM | 2625 | CG | PRO | A | 380 | 49.933 | 32.395 | 33.626 | 1.00 | 35.29 | C |
| ATOM | 2626 | C | PRO | A | 380 | 47.223 | 34.805 | 32.763 | 1.00 | 45.90 | C |
| ATOM | 2627 | O | PRO | A | 380 | 46.529 | 35.650 | 33.335 | 1.00 | 45.90 | O |
| ATOM | 2628 | N | HIS | A | 381 | 47.333 | 34.732 | 31.440 | 1.00 | 38.40 | N |
| ATOM | 2629 | CA | HIS | A | 381 | 46.641 | 35.673 | 30.561 | 1.00 | 38.40 | C |
| ATOM | 2630 | CB | HIS | A | 381 | 46.903 | 35.309 | 29.087 | 1.00 | 61.92 | C |
| ATOM | 2631 | CG | HIS | A | 381 | 46.246 | 34.037 | 28.640 | 1.00 | 61.92 | C |
| ATOM | 2632 | CD2 | HIS | A | 381 | 46.654 | 32.748 | 28.714 | 1.00 | 61.92 | C |
| ATOM | 2633 | ND1 | HIS | A | 381 | 45.001 | 34.011 | 28.046 | 1.00 | 61.92 | N |
| ATOM | 2634 | CE1 | HIS | A | 381 | 44.670 | 32.761 | 27.776 | 1.00 | 61.92 | C |
| ATOM | 2635 | NE2 | HIS | A | 381 | 45.655 | 31.974 | 28.171 | 1.00 | 61.92 | N |
| ATOM | 2636 | C | HIS | A | 381 | 45.139 | 35.733 | 30.844 | 1.00 | 38.40 | C |
| ATOM | 2637 | O | HIS | A | 381 | 44.540 | 36.804 | 30.793 | 1.00 | 38.40 | O |
| ATOM | 2638 | N | ALA | A | 382 | 44.538 | 34.588 | 31.156 | 1.00 | 35.85 | N |
| ATOM | 2639 | CA | ALA | A | 382 | 43.102 | 34.529 | 31.445 | 1.00 | 35.85 | C |
| ATOM | 2640 | CB | ALA | A | 382 | 42.675 | 33.071 | 31.740 | 1.00 | 32.13 | C |
| ATOM | 2641 | C | ALA | A | 382 | 42.711 | 35.449 | 32.614 | 1.00 | 35.85 | C |

| | | | | | | | | | | | |
|---|---|---|---|---|---|---|---|---|---|---|---|
| ATOM | 2642 | O | ALA | A | 382 | 41.657 | 36.085 | 32.592 | 1.00 | 35.85 | O |
| ATOM | 2643 | N | ARG | A | 383 | 43.562 | 35.511 | 33.632 | 1.00 | 55.72 | N |
| ATOM | 2644 | CA | ARG | A | 383 | 43.307 | 36.362 | 34.783 | 1.00 | 55.72 | C |
| ATOM | 2645 | CB | ARG | A | 383 | 44.431 | 36.187 | 35.818 | 1.00 | 55.55 | C |
| ATOM | 2646 | CG | ARG | A | 383 | 44.879 | 34.726 | 36.035 | 1.00 | 55.55 | C |
| ATOM | 2647 | CD | ARG | A | 383 | 45.854 | 34.587 | 37.213 | 1.00 | 55.55 | C |
| ATOM | 2648 | NE | ARG | A | 383 | 45.203 | 34.108 | 38.433 | 1.00 | 55.55 | N |
| ATOM | 2649 | CZ | ARG | A | 383 | 45.128 | 32.827 | 38.800 | 1.00 | 55.55 | C |
| ATOM | 2650 | NH1 | ARG | A | 383 | 45.673 | 31.873 | 38.053 | 1.00 | 55.55 | N |
| ATOM | 2651 | NH2 | ARG | A | 383 | 44.488 | 32.486 | 39.911 | 1.00 | 55.55 | N |
| ATOM | 2652 | C | ARG | A | 383 | 43.233 | 37.822 | 34.286 | 1.00 | 55.72 | C |
| ATOM | 2653 | O | ARG | A | 383 | 44.286 | 38.493 | 34.244 | 1.00 | 55.72 | O |
| ATOM | 2654 | OXT | ARG | A | 383 | 42.127 | 38.281 | 33.901 | 1.00 | 55.55 | O |
| TER | 2655 | | ARG | A | 383 | | | | | | |
| ATOM | 2656 | CB | VAL | B | 37 | 21.755 | 33.564 | 97.403 | 1.00 | 27.95 | C |
| ATOM | 2657 | CG1 | VAL | B | 37 | 22.602 | 34.021 | 96.220 | 1.00 | 27.95 | C |
| ATOM | 2658 | CG2 | VAL | B | 37 | 20.383 | 34.212 | 97.411 | 1.00 | 27.95 | C |
| ATOM | 2659 | C | VAL | B | 37 | 22.981 | 31.442 | 97.304 | 1.00 | 45.39 | C |
| ATOM | 2660 | O | VAL | B | 37 | 23.858 | 31.784 | 98.086 | 1.00 | 45.39 | O |
| ATOM | 2661 | N | VAL | B | 37 | 20.805 | 31.581 | 98.557 | 1.00 | 45.39 | N |
| ATOM | 2662 | CA | VAL | B | 37 | 21.591 | 32.056 | 97.377 | 1.00 | 45.39 | C |
| ATOM | 2663 | N | THR | B | 38 | 23.181 | 30.552 | 96.340 | 1.00 | 46.04 | N |
| ATOM | 2664 | CA | THR | B | 38 | 24.454 | 29.867 | 96.159 | 1.00 | 46.04 | C |
| ATOM | 2665 | CB | THR | B | 38 | 24.231 | 28.512 | 95.452 | 1.00 | 37.12 | C |
| ATOM | 2666 | OG1 | THR | B | 38 | 23.308 | 27.718 | 96.212 | 1.00 | 37.12 | O |
| ATOM | 2667 | CG2 | THR | B | 38 | 25.547 | 27.766 | 95.289 | 1.00 | 37.12 | C |
| ATOM | 2668 | C | THR | B | 38 | 25.428 | 30.685 | 95.320 | 1.00 | 46.04 | C |
| ATOM | 2669 | O | THR | B | 38 | 25.056 | 31.213 | 94.271 | 1.00 | 46.04 | O |
| ATOM | 2670 | N | THR | B | 39 | 26.671 | 30.801 | 95.777 | 1.00 | 42.59 | N |
| ATOM | 2671 | CA | THR | B | 39 | 27.659 | 31.530 | 94.989 | 1.00 | 42.59 | C |
| ATOM | 2672 | CB | THR | B | 39 | 27.994 | 32.902 | 95.596 | 1.00 | 52.78 | C |
| ATOM | 2673 | OG1 | THR | B | 39 | 26.800 | 33.692 | 95.691 | 1.00 | 52.78 | O |
| ATOM | 2674 | CG2 | THR | B | 39 | 28.984 | 33.632 | 94.703 | 1.00 | 52.78 | C |
| ATOM | 2675 | C | THR | B | 39 | 28.957 | 30.746 | 94.767 | 1.00 | 42.59 | C |
| ATOM | 2676 | O | THR | B | 39 | 29.570 | 30.223 | 95.712 | 1.00 | 42.59 | O |
| ATOM | 2677 | N | VAL | B | 40 | 29.354 | 30.678 | 93.495 | 1.00 | 30.11 | N |
| ATOM | 2678 | CA | VAL | B | 40 | 30.557 | 29.968 | 93.073 | 1.00 | 30.11 | C |
| ATOM | 2679 | CB | VAL | B | 40 | 30.206 | 28.609 | 92.421 | 1.00 | 27.23 | C |
| ATOM | 2680 | CG1 | VAL | B | 40 | 29.379 | 27.773 | 93.378 | 1.00 | 27.23 | C |
| ATOM | 2681 | CG2 | VAL | B | 40 | 29.443 | 28.831 | 91.116 | 1.00 | 27.23 | C |
| ATOM | 2682 | C | VAL | B | 40 | 31.354 | 30.754 | 92.046 | 1.00 | 30.11 | C |
| ATOM | 2683 | O | VAL | B | 40 | 30.924 | 31.799 | 91.547 | 1.00 | 30.11 | O |
| ATOM | 2684 | N | VAL | B | 41 | 32.529 | 30.232 | 91.733 | 1.00 | 30.49 | N |
| ATOM | 2685 | CA | VAL | B | 41 | 33.385 | 30.833 | 90.724 | 1.00 | 30.49 | C |
| ATOM | 2686 | CB | VAL | B | 41 | 34.837 | 31.008 | 91.231 | 1.00 | 13.85 | C |
| ATOM | 2687 | CG1 | VAL | B | 41 | 35.675 | 31.759 | 90.184 | 1.00 | 13.85 | C |
| ATOM | 2688 | CG2 | VAL | B | 41 | 34.828 | 31.743 | 92.565 | 1.00 | 13.85 | C |
| ATOM | 2689 | C | VAL | B | 41 | 33.359 | 29.814 | 89.594 | 1.00 | 30.49 | C |
| ATOM | 2690 | O | VAL | B | 41 | 33.866 | 28.709 | 89.747 | 1.00 | 30.49 | O |
| ATOM | 2691 | N | ALA | B | 42 | 32.735 | 30.168 | 88.478 | 1.00 | 41.92 | N |
| ATOM | 2692 | CA | ALA | B | 42 | 32.656 | 29.251 | 87.346 | 1.00 | 41.92 | C |
| ATOM | 2693 | CB | ALA | B | 42 | 31.188 | 28.954 | 87.004 | 1.00 | 23.84 | C |
| ATOM | 2694 | C | ALA | B | 42 | 33.375 | 29.849 | 86.142 | 1.00 | 41.92 | C |
| ATOM | 2695 | O | ALA | B | 42 | 33.742 | 31.021 | 86.152 | 1.00 | 41.92 | O |
| ATOM | 2696 | N | THR | B | 43 | 33.591 | 29.041 | 85.112 | 1.00 | 34.45 | N |
| ATOM | 2697 | CA | THR | B | 43 | 34.261 | 29.527 | 83.919 | 1.00 | 34.45 | C |
| ATOM | 2698 | CB | THR | B | 43 | 35.514 | 28.693 | 83.585 | 1.00 | 32.66 | C |
| ATOM | 2699 | OG1 | THR | B | 43 | 36.333 | 28.539 | 84.747 | 1.00 | 32.66 | O |
| ATOM | 2700 | CG2 | THR | B | 43 | 36.312 | 29.395 | 82.513 | 1.00 | 32.66 | C |
| ATOM | 2701 | C | THR | B | 43 | 33.293 | 29.408 | 82.747 | 1.00 | 34.45 | C |
| ATOM | 2702 | O | THR | B | 43 | 32.580 | 28.417 | 82.623 | 1.00 | 34.45 | O |
| ATOM | 2703 | N | PRO | B | 44 | 33.225 | 30.429 | 81.881 | 1.00 | 54.52 | N |
| ATOM | 2704 | CD | PRO | B | 44 | 33.607 | 31.835 | 82.033 | 1.00 | 52.03 | C |

| | | | | | | | | | | | |
|---|---|---|---|---|---|---|---|---|---|---|---|
| ATOM | 2705 | CA | PRO | B | 44 | 32.279 | 30.252 | 80.773 | 1.00 | 54.52 | C |
| ATOM | 2706 | CB | PRO | B | 44 | 32.291 | 31.600 | 80.064 | 1.00 | 52.03 | C |
| ATOM | 2707 | CG | PRO | B | 44 | 33.414 | 32.336 | 80.685 | 1.00 | 52.03 | C |
| ATOM | 2708 | C | PRO | B | 44 | 32.693 | 29.122 | 79.867 | 1.00 | 54.52 | C |
| ATOM | 2709 | O | PRO | B | 44 | 33.869 | 28.780 | 79.814 | 1.00 | 54.52 | O |
| ATOM | 2710 | N | GLY | B | 45 | 31.729 | 28.527 | 79.174 | 1.00 | 41.04 | N |
| ATOM | 2711 | CA | GLY | B | 45 | 32.040 | 27.402 | 78.314 | 1.00 | 41.04 | C |
| ATOM | 2712 | C | GLY | B | 45 | 32.842 | 27.880 | 77.143 | 1.00 | 41.04 | C |
| ATOM | 2713 | O | GLY | B | 45 | 33.883 | 27.307 | 76.804 | 1.00 | 41.04 | O |
| ATOM | 2714 | N | GLN | B | 46 | 32.367 | 28.959 | 76.539 | 1.00 | 74.52 | N |
| ATOM | 2715 | CA | GLN | B | 46 | 33.037 | 29.506 | 75.376 | 1.00 | 74.52 | C |
| ATOM | 2716 | CB | GLN | B | 46 | 32.018 | 29.917 | 74.335 | 1.00 | 62.23 | C |
| ATOM | 2717 | CG | GLN | B | 46 | 30.887 | 28.918 | 74.256 | 1.00 | 23.68 | C |
| ATOM | 2718 | CD | GLN | B | 46 | 30.824 | 28.228 | 72.887 | 1.00 | 23.68 | C |
| ATOM | 2719 | OE1 | GLN | B | 46 | 31.816 | 27.592 | 72.440 | 1.00 | 23.68 | O |
| ATOM | 2720 | NE2 | GLN | B | 46 | 29.654 | 28.325 | 72.218 | 1.00 | 23.68 | N |
| ATOM | 2721 | C | GLN | B | 46 | 33.932 | 30.677 | 75.671 | 1.00 | 74.52 | C |
| ATOM | 2722 | O | GLN | B | 46 | 35.117 | 30.594 | 75.375 | 1.00 | 74.52 | O |
| ATOM | 2723 | N | GLY | B | 47 | 33.366 | 31.757 | 76.218 | 1.00 | 83.59 | N |
| ATOM | 2724 | CA | GLY | B | 47 | 34.126 | 32.953 | 76.546 | 1.00 | 83.59 | C |
| ATOM | 2725 | C | GLY | B | 47 | 35.603 | 32.726 | 76.818 | 1.00 | 83.59 | C |
| ATOM | 2726 | O | GLY | B | 47 | 36.080 | 31.605 | 76.856 | 1.00 | 83.59 | O |
| ATOM | 2727 | N | PRO | B | 48 | 36.377 | 33.783 | 77.029 | 1.00 | 60.48 | N |
| ATOM | 2728 | CD | PRO | B | 48 | 36.140 | 35.231 | 76.957 | 1.00 | 65.93 | C |
| ATOM | 2729 | CA | PRO | B | 48 | 37.794 | 33.481 | 77.283 | 1.00 | 60.48 | C |
| ATOM | 2730 | CB | PRO | B | 48 | 38.414 | 34.847 | 77.417 | 1.00 | 65.93 | C |
| ATOM | 2731 | CG | PRO | B | 48 | 37.470 | 35.721 | 76.608 | 1.00 | 65.93 | C |
| ATOM | 2732 | C | PRO | B | 48 | 37.900 | 32.727 | 78.594 | 1.00 | 60.48 | C |
| ATOM | 2733 | O | PRO | B | 48 | 37.142 | 33.007 | 79.505 | 1.00 | 60.48 | O |
| ATOM | 2734 | N | ASP | B | 49 | 38.818 | 31.768 | 78.681 | 1.00 | 52.11 | N |
| ATOM | 2735 | CA | ASP | B | 49 | 38.981 | 30.956 | 79.886 | 1.00 | 52.11 | C |
| ATOM | 2736 | CB | ASP | B | 49 | 40.070 | 29.895 | 79.666 | 1.00 | 46.62 | C |
| ATOM | 2737 | CG | ASP | B | 49 | 40.092 | 28.848 | 80.765 | 1.00 | 46.62 | C |
| ATOM | 2738 | OD1 | ASP | B | 49 | 40.166 | 29.240 | 81.947 | 1.00 | 46.62 | O |
| ATOM | 2739 | OD2 | ASP | B | 49 | 40.040 | 27.635 | 80.462 | 1.00 | 46.62 | O |
| ATOM | 2740 | C | ASP | B | 49 | 39.344 | 31.887 | 81.035 | 1.00 | 52.11 | C |
| ATOM | 2741 | O | ASP | B | 49 | 40.519 | 32.105 | 81.322 | 1.00 | 52.11 | O |
| ATOM | 2742 | N | ARG | B | 50 | 38.320 | 32.447 | 81.675 | 1.00 | 42.35 | N |
| ATOM | 2743 | CA | ARG | B | 50 | 38.510 | 33.385 | 82.771 | 1.00 | 42.35 | C |
| ATOM | 2744 | CB | ARG | B | 50 | 38.454 | 34.830 | 82.255 | 1.00 | 96.20 | C |
| ATOM | 2745 | CG | ARG | B | 50 | 39.371 | 35.088 | 81.065 | 1.00 | 96.20 | C |
| ATOM | 2746 | CD | ARG | B | 50 | 40.264 | 36.306 | 81.265 | 1.00 | 96.20 | C |
| ATOM | 2747 | NE | ARG | B | 50 | 39.750 | 37.504 | 80.605 | 1.00 | 96.20 | N |
| ATOM | 2748 | CZ | ARG | B | 50 | 40.478 | 38.598 | 80.399 | 1.00 | 96.20 | C |
| ATOM | 2749 | NH1 | ARG | B | 50 | 41.745 | 38.637 | 80.803 | 1.00 | 96.20 | N |
| ATOM | 2750 | NH2 | ARG | B | 50 | 39.947 | 39.651 | 79.790 | 1.00 | 96.20 | N |
| ATOM | 2751 | C | ARG | B | 50 | 37.425 | 33.178 | 83.807 | 1.00 | 42.35 | C |
| ATOM | 2752 | O | ARG | B | 50 | 36.268 | 33.538 | 83.593 | 1.00 | 42.35 | O |
| ATOM | 2753 | N | PRO | B | 51 | 37.785 | 32.604 | 84.956 | 1.00 | 37.14 | N |
| ATOM | 2754 | CD | PRO | B | 51 | 39.141 | 32.314 | 85.447 | 1.00 | 29.69 | C |
| ATOM | 2755 | CA | PRO | B | 51 | 36.790 | 32.368 | 86.003 | 1.00 | 37.14 | C |
| ATOM | 2756 | CB | PRO | B | 51 | 37.619 | 31.766 | 87.135 | 1.00 | 29.69 | C |
| ATOM | 2757 | CG | PRO | B | 51 | 38.963 | 32.409 | 86.948 | 1.00 | 29.69 | C |
| ATOM | 2758 | C | PRO | B | 51 | 36.060 | 33.629 | 86.431 | 1.00 | 37.14 | C |
| ATOM | 2759 | O | PRO | B | 51 | 36.568 | 34.733 | 86.272 | 1.00 | 37.14 | O |
| ATOM | 2760 | N | GLN | B | 52 | 34.860 | 33.447 | 86.966 | 1.00 | 39.00 | N |
| ATOM | 2761 | CA | GLN | B | 52 | 34.044 | 34.557 | 87.438 | 1.00 | 39.00 | C |
| ATOM | 2762 | CB | GLN | B | 52 | 33.367 | 35.276 | 86.264 | 1.00 | 31.91 | C |
| ATOM | 2763 | CG | GLN | B | 52 | 32.525 | 34.400 | 85.347 | 1.00 | 31.91 | C |
| ATOM | 2764 | CD | GLN | B | 52 | 31.620 | 35.233 | 84.448 | 1.00 | 31.91 | C |
| ATOM | 2765 | OE1 | GLN | B | 52 | 30.713 | 35.907 | 84.928 | 1.00 | 31.91 | O |
| ATOM | 2766 | NE2 | GLN | B | 52 | 31.872 | 35.197 | 83.140 | 1.00 | 31.91 | N |
| ATOM | 2767 | C | GLN | B | 52 | 32.988 | 34.097 | 88.436 | 1.00 | 39.00 | C |

| | | | | | | | | | | | |
|---|---|---|---|---|---|---|---|---|---|---|---|
| ATOM | 2768 | O | GLN | B | 52 | 32.675 | 32.910 | 88.532 | 1.00 | 39.00 | O |
| ATOM | 2769 | N | GLU | B | 53 | 32.443 | 35.048 | 89.181 | 1.00 | 51.90 | N |
| ATOM | 2770 | CA | GLU | B | 53 | 31.431 | 34.729 | 90.173 | 1.00 | 51.90 | C |
| ATOM | 2771 | CB | GLU | B | 53 | 31.240 | 35.905 | 91.132 | 1.00 | 68.62 | C |
| ATOM | 2772 | CG | GLU | B | 53 | 30.450 | 35.533 | 92.385 | 1.00 | 68.62 | C |
| ATOM | 2773 | CD | GLU | B | 53 | 30.255 | 36.716 | 93.295 | 1.00 | 68.62 | C |
| ATOM | 2774 | OE1 | GLU | B | 53 | 29.727 | 36.599 | 94.413 | 1.00 | 68.62 | O |
| ATOM | 2775 | OE2 | GLU | B | 53 | 30.633 | 37.795 | 92.880 | 1.00 | 68.62 | O |
| ATOM | 2776 | C | GLU | B | 53 | 30.109 | 34.397 | 89.496 | 1.00 | 51.90 | C |
| ATOM | 2777 | O | GLU | B | 53 | 29.715 | 35.047 | 88.530 | 1.00 | 51.90 | O |
| ATOM | 2778 | N | VAL | B | 54 | 29.425 | 33.380 | 89.997 | 1.00 | 38.89 | N |
| ATOM | 2779 | CA | VAL | B | 54 | 28.153 | 32.999 | 89.415 | 1.00 | 38.89 | C |
| ATOM | 2780 | CB | VAL | B | 54 | 28.312 | 31.754 | 88.482 | 1.00 | 16.21 | C |
| ATOM | 2781 | CG1 | VAL | B | 54 | 26.945 | 31.355 | 87.924 | 1.00 | 16.21 | C |
| ATOM | 2782 | CG2 | VAL | B | 54 | 29.264 | 32.073 | 87.309 | 1.00 | 16.21 | C |
| ATOM | 2783 | C | VAL | B | 54 | 27.144 | 32.697 | 90.523 | 1.00 | 38.89 | C |
| ATOM | 2784 | O | VAL | B | 54 | 27.266 | 31.697 | 91.234 | 1.00 | 38.89 | O |
| ATOM | 2785 | N | SER | B | 55 | 26.149 | 33.566 | 90.681 | 1.00 | 46.06 | N |
| ATOM | 2786 | CA | SER | B | 55 | 25.151 | 33.354 | 91.724 | 1.00 | 46.06 | C |
| ATOM | 2787 | CB | SER | B | 55 | 24.930 | 34.635 | 92.526 | 1.00 | 61.81 | C |
| ATOM | 2788 | OG | SER | B | 55 | 26.001 | 34.846 | 93.431 | 1.00 | 61.81 | O |
| ATOM | 2789 | C | SER | B | 55 | 23.826 | 32.849 | 91.193 | 1.00 | 46.06 | C |
| ATOM | 2790 | O | SER | B | 55 | 23.357 | 33.272 | 90.137 | 1.00 | 46.06 | O |
| ATOM | 2791 | N | TYR | B | 56 | 23.225 | 31.934 | 91.940 | 1.00 | 36.50 | N |
| ATOM | 2792 | CA | TYR | B | 56 | 21.957 | 31.357 | 91.546 | 1.00 | 36.50 | C |
| ATOM | 2793 | CB | TYR | B | 56 | 22.182 | 30.181 | 90.583 | 1.00 | 26.32 | C |
| ATOM | 2794 | CG | TYR | B | 56 | 23.110 | 29.098 | 91.091 | 1.00 | 26.32 | C |
| ATOM | 2795 | CD1 | TYR | B | 56 | 22.635 | 28.057 | 91.885 | 1.00 | 26.32 | C |
| ATOM | 2796 | CE1 | TYR | B | 56 | 23.491 | 27.037 | 92.323 | 1.00 | 26.32 | C |
| ATOM | 2797 | CD2 | TYR | B | 56 | 24.470 | 29.102 | 90.745 | 1.00 | 26.32 | C |
| ATOM | 2798 | CE2 | TYR | B | 56 | 25.336 | 28.095 | 91.172 | 1.00 | 26.32 | C |
| ATOM | 2799 | CZ | TYR | B | 56 | 24.841 | 27.064 | 91.958 | 1.00 | 26.32 | C |
| ATOM | 2800 | OH | TYR | B | 56 | 25.681 | 26.056 | 92.362 | 1.00 | 26.32 | O |
| ATOM | 2801 | C | TYR | B | 56 | 21.163 | 30.902 | 92.753 | 1.00 | 36.50 | C |
| ATOM | 2802 | O | TYR | B | 56 | 21.721 | 30.644 | 93.822 | 1.00 | 36.50 | O |
| ATOM | 2803 | N | THR | B | 57 | 19.856 | 30.787 | 92.563 | 1.00 | 51.95 | N |
| ATOM | 2804 | CA | THR | B | 57 | 18.966 | 30.380 | 93.635 | 1.00 | 51.95 | C |
| ATOM | 2805 | CB | THR | B | 57 | 18.242 | 31.592 | 94.212 | 1.00 | 37.29 | C |
| ATOM | 2806 | OG1 | THR | B | 57 | 17.880 | 32.478 | 93.142 | 1.00 | 37.29 | O |
| ATOM | 2807 | CG2 | THR | B | 57 | 19.122 | 32.300 | 95.215 | 1.00 | 37.29 | C |
| ATOM | 2808 | C | THR | B | 57 | 17.906 | 29.402 | 93.169 | 1.00 | 51.95 | C |
| ATOM | 2809 | O | THR | B | 57 | 17.864 | 29.026 | 91.996 | 1.00 | 51.95 | O |
| ATOM | 2810 | N | ASP | B | 58 | 17.058 | 28.996 | 94.111 | 1.00 | 65.44 | N |
| ATOM | 2811 | CA | ASP | B | 58 | 15.953 | 28.100 | 93.824 | 1.00 | 65.44 | C |
| ATOM | 2812 | CB | ASP | B | 58 | 14.920 | 28.845 | 92.964 | 1.00 | 51.07 | C |
| ATOM | 2813 | CG | ASP | B | 58 | 14.763 | 30.329 | 93.361 | 1.00 | 51.07 | C |
| ATOM | 2814 | OD1 | ASP | B | 58 | 14.055 | 30.622 | 94.341 | 1.00 | 51.07 | O |
| ATOM | 2815 | OD2 | ASP | B | 58 | 15.350 | 31.212 | 92.696 | 1.00 | 51.07 | O |
| ATOM | 2816 | C | ASP | B | 58 | 16.468 | 26.863 | 93.091 | 1.00 | 65.44 | C |
| ATOM | 2817 | O | ASP | B | 58 | 16.049 | 26.561 | 91.973 | 1.00 | 65.44 | O |
| ATOM | 2818 | N | THR | B | 59 | 17.389 | 26.159 | 93.732 | 1.00 | 33.46 | N |
| ATOM | 2819 | CA | THR | B | 59 | 17.984 | 24.961 | 93.165 | 1.00 | 33.46 | C |
| ATOM | 2820 | CB | THR | B | 59 | 19.433 | 24.831 | 93.643 | 1.00 | 29.94 | C |
| ATOM | 2821 | OG1 | THR | B | 59 | 19.471 | 24.689 | 95.068 | 1.00 | 29.94 | O |
| ATOM | 2822 | CG2 | THR | B | 59 | 20.190 | 26.077 | 93.289 | 1.00 | 29.94 | C |
| ATOM | 2823 | C | THR | B | 59 | 17.186 | 23.711 | 93.540 | 1.00 | 33.46 | C |
| ATOM | 2824 | O | THR | B | 59 | 17.123 | 23.335 | 94.708 | 1.00 | 33.46 | O |
| ATOM | 2825 | N | LYS | B | 60 | 16.592 | 23.066 | 92.542 | 1.00 | 45.53 | N |
| ATOM | 2826 | CA | LYS | B | 60 | 15.768 | 21.888 | 92.771 | 1.00 | 45.53 | C |
| ATOM | 2827 | CB | LYS | B | 60 | 14.309 | 22.256 | 92.517 | 1.00 | 34.55 | C |
| ATOM | 2828 | CG | LYS | B | 60 | 14.134 | 23.286 | 91.390 | 1.00 | 34.55 | C |
| ATOM | 2829 | CD | LYS | B | 60 | 12.757 | 23.947 | 91.399 | 1.00 | 34.55 | C |
| ATOM | 2830 | CE | LYS | B | 60 | 12.570 | 24.783 | 92.644 | 1.00 | 34.55 | C |

| | | | | | | | | | | | |
|---|---|---|---|---|---|---|---|---|---|---|---|
| ATOM | 2831 | NZ | LYS | B | 60 | 11.340 | 25.606 | 92.589 | 1.00 | 34.55 | N |
| ATOM | 2832 | C | LYS | B | 60 | 16.194 | 20.723 | 91.896 | 1.00 | 45.53 | C |
| ATOM | 2833 | O | LYS | B | 60 | 16.719 | 20.924 | 90.798 | 1.00 | 45.53 | O |
| ATOM | 2834 | N | VAL | B | 61 | 15.972 | 19.509 | 92.387 | 1.00 | 46.69 | N |
| ATOM | 2835 | CA | VAL | B | 61 | 16.362 | 18.314 | 91.663 | 1.00 | 46.69 | C |
| ATOM | 2836 | CB | VAL | B | 61 | 16.448 | 17.101 | 92.611 | 1.00 | 37.01 | C |
| ATOM | 2837 | CG1 | VAL | B | 61 | 17.234 | 15.987 | 91.961 | 1.00 | 37.01 | C |
| ATOM | 2838 | CG2 | VAL | B | 61 | 17.102 | 17.508 | 93.931 | 1.00 | 37.01 | C |
| ATOM | 2839 | C | VAL | B | 61 | 15.373 | 18.036 | 90.553 | 1.00 | 46.69 | C |
| ATOM | 2840 | O | VAL | B | 61 | 14.168 | 18.277 | 90.705 | 1.00 | 46.69 | O |
| ATOM | 2841 | N | ILE | B | 62 | 15.893 | 17.552 | 89.432 | 1.00 | 39.48 | N |
| ATOM | 2842 | CA | ILE | B | 62 | 15.067 | 17.269 | 88.274 | 1.00 | 39.48 | C |
| ATOM | 2843 | CB | ILE | B | 62 | 15.648 | 17.941 | 87.023 | 1.00 | 28.45 | C |
| ATOM | 2844 | CG2 | ILE | B | 62 | 14.757 | 17.688 | 85.795 | 1.00 | 28.45 | C |
| ATOM | 2845 | CG1 | ILE | B | 62 | 15.789 | 19.430 | 87.284 | 1.00 | 28.45 | C |
| ATOM | 2846 | CD1 | ILE | B | 62 | 15.807 | 20.264 | 86.044 | 1.00 | 28.45 | C |
| ATOM | 2847 | C | ILE | B | 62 | 15.036 | 15.789 | 88.020 | 1.00 | 39.48 | C |
| ATOM | 2848 | O | ILE | B | 62 | 14.016 | 15.116 | 88.177 | 1.00 | 39.48 | O |
| ATOM | 2849 | N | GLY | B | 63 | 16.189 | 15.296 | 87.610 | 1.00 | 70.45 | N |
| ATOM | 2850 | CA | GLY | B | 63 | 16.325 | 13.893 | 87.328 | 1.00 | 70.45 | C |
| ATOM | 2851 | C | GLY | B | 63 | 17.771 | 13.511 | 87.442 | 1.00 | 70.45 | C |
| ATOM | 2852 | O | GLY | B | 63 | 18.565 | 14.191 | 88.106 | 1.00 | 70.45 | O |
| ATOM | 2853 | N | ASN | B | 64 | 18.118 | 12.439 | 86.745 | 1.00 | 65.27 | N |
| ATOM | 2854 | CA | ASN | B | 64 | 19.469 | 11.890 | 86.747 | 1.00 | 65.27 | C |
| ATOM | 2855 | CB | ASN | B | 64 | 19.895 | 11.452 | 88.166 | 1.00 | 49.33 | C |
| ATOM | 2856 | CG | ASN | B | 64 | 18.769 | 10.806 | 88.950 | 1.00 | 49.33 | C |
| ATOM | 2857 | OD1 | ASN | B | 64 | 17.998 | 10.008 | 88.416 | 1.00 | 49.33 | O |
| ATOM | 2858 | ND2 | ASN | B | 64 | 18.685 | 11.132 | 90.230 | 1.00 | 49.33 | N |
| ATOM | 2859 | C | ASN | B | 64 | 19.418 | 10.677 | 85.832 | 1.00 | 65.27 | C |
| ATOM | 2860 | O | ASN | B | 64 | 18.379 | 10.016 | 85.731 | 1.00 | 65.27 | O |
| ATOM | 2861 | N | GLY | B | 65 | 20.545 | 10.400 | 85.181 | 1.00 | 54.13 | N |
| ATOM | 2862 | CA | GLY | B | 65 | 20.651 | 9.280 | 84.275 | 1.00 | 54.13 | C |
| ATOM | 2863 | C | GLY | B | 65 | 21.523 | 8.237 | 84.912 | 1.00 | 54.13 | C |
| ATOM | 2864 | O | GLY | B | 65 | 21.487 | 8.060 | 86.131 | 1.00 | 54.13 | O |
| ATOM | 2865 | N | SER | B | 66 | 22.295 | 7.548 | 84.086 | 1.00 | 99.97 | N |
| ATOM | 2866 | CA | SER | B | 66 | 23.194 | 6.524 | 84.573 | 1.00 | 99.97 | C |
| ATOM | 2867 | CB | SER | B | 66 | 23.988 | 5.934 | 83.403 | 1.00 | 23.68 | C |
| ATOM | 2868 | OG | SER | B | 66 | 23.295 | 4.838 | 82.795 | 1.00 | 23.68 | O |
| ATOM | 2869 | C | SER | B | 66 | 24.146 | 7.158 | 85.587 | 1.00 | 99.97 | C |
| ATOM | 2870 | O | SER | B | 66 | 24.265 | 6.692 | 86.720 | 1.00 | 99.97 | O |
| ATOM | 2871 | N | PHE | B | 67 | 24.796 | 8.243 | 85.183 | 1.00 | 93.06 | N |
| ATOM | 2872 | CA | PHE | B | 67 | 25.766 | 8.931 | 86.037 | 1.00 | 93.06 | C |
| ATOM | 2873 | CB | PHE | B | 67 | 27.214 | 8.845 | 85.475 | 1.00 | 59.78 | C |
| ATOM | 2874 | CG | PHE | B | 67 | 27.557 | 7.516 | 84.880 | 1.00 | 59.78 | C |
| ATOM | 2875 | CD1 | PHE | B | 67 | 27.498 | 7.325 | 83.495 | 1.00 | 59.78 | C |
| ATOM | 2876 | CD2 | PHE | B | 67 | 27.832 | 6.427 | 85.711 | 1.00 | 59.78 | C |
| ATOM | 2877 | CE1 | PHE | B | 67 | 27.698 | 6.079 | 82.946 | 1.00 | 59.78 | C |
| ATOM | 2878 | CE2 | PHE | B | 67 | 28.031 | 5.182 | 85.185 | 1.00 | 59.78 | C |
| ATOM | 2879 | CZ | PHE | B | 67 | 27.963 | 4.997 | 83.793 | 1.00 | 59.78 | C |
| ATOM | 2880 | C | PHE | B | 67 | 25.341 | 10.352 | 85.970 | 1.00 | 93.06 | C |
| ATOM | 2881 | O | PHE | B | 67 | 24.910 | 10.792 | 84.918 | 1.00 | 93.06 | O |
| ATOM | 2882 | N | GLY | B | 68 | 25.525 | 11.089 | 87.056 | 1.00 | 49.67 | N |
| ATOM | 2883 | CA | GLY | B | 68 | 25.133 | 12.489 | 87.061 | 1.00 | 49.67 | C |
| ATOM | 2884 | C | GLY | B | 68 | 23.678 | 12.850 | 87.395 | 1.00 | 49.67 | C |
| ATOM | 2885 | O | GLY | B | 68 | 22.711 | 12.391 | 86.760 | 1.00 | 49.67 | O |
| ATOM | 2886 | N | VAL | B | 69 | 23.547 | 13.743 | 88.373 | 1.00 | 42.78 | N |
| ATOM | 2887 | CA | VAL | B | 69 | 22.279 | 14.232 | 88.858 | 1.00 | 42.78 | C |
| ATOM | 2888 | CB | VAL | B | 69 | 22.240 | 14.123 | 90.376 | 1.00 | 42.42 | C |
| ATOM | 2889 | CG1 | VAL | B | 69 | 23.635 | 14.300 | 90.928 | 1.00 | 42.42 | C |
| ATOM | 2890 | CG2 | VAL | B | 69 | 21.279 | 15.145 | 90.955 | 1.00 | 42.42 | C |
| ATOM | 2891 | C | VAL | B | 69 | 22.092 | 15.679 | 88.423 | 1.00 | 42.78 | C |
| ATOM | 2892 | O | VAL | B | 69 | 22.923 | 16.547 | 88.692 | 1.00 | 42.78 | O |
| ATOM | 2893 | N | VAL | B | 70 | 20.978 | 15.920 | 87.745 | 1.00 | 47.47 | N |

| | | | | | | | | | | | |
|---|---|---|---|---|---|---|---|---|---|---|---|
| ATOM | 2894 | CA | VAL | B | 70 | 20.659 | 17.252 | 87.255 | 1.00 | 47.47 | C |
| ATOM | 2895 | CB | VAL | B | 70 | 20.104 | 17.169 | 85.834 | 1.00 | 27.15 | C |
| ATOM | 2896 | CG1 | VAL | B | 70 | 20.086 | 18.578 | 85.207 | 1.00 | 27.15 | C |
| ATOM | 2897 | CG2 | VAL | B | 70 | 20.988 | 16.228 | 85.023 | 1.00 | 27.15 | C |
| ATOM | 2898 | C | VAL | B | 70 | 19.731 | 18.131 | 88.117 | 1.00 | 47.47 | C |
| ATOM | 2899 | O | VAL | B | 70 | 18.672 | 17.706 | 88.582 | 1.00 | 47.47 | O |
| ATOM | 2900 | N | TYR | B | 71 | 20.152 | 19.380 | 88.317 | 1.00 | 32.02 | N |
| ATOM | 2901 | CA | TYR | B | 71 | 19.402 | 20.363 | 89.106 | 1.00 | 32.02 | C |
| ATOM | 2902 | CB | TYR | B | 71 | 20.244 | 20.888 | 90.266 | 1.00 | 46.76 | C |
| ATOM | 2903 | CG | TYR | B | 71 | 20.637 | 19.863 | 91.299 | 1.00 | 46.76 | C |
| ATOM | 2904 | CD1 | TYR | B | 71 | 21.762 | 19.060 | 91.127 | 1.00 | 46.76 | C |
| ATOM | 2905 | CE1 | TYR | B | 71 | 22.137 | 18.132 | 92.098 | 1.00 | 46.76 | C |
| ATOM | 2906 | CD2 | TYR | B | 71 | 19.896 | 19.711 | 92.468 | 1.00 | 46.76 | C |
| ATOM | 2907 | CE2 | TYR | B | 71 | 20.263 | 18.789 | 93.435 | 1.00 | 46.76 | C |
| ATOM | 2908 | CZ | TYR | B | 71 | 21.383 | 18.004 | 93.241 | 1.00 | 46.76 | C |
| ATOM | 2909 | OH | TYR | B | 71 | 21.737 | 17.071 | 94.181 | 1.00 | 46.76 | O |
| ATOM | 2910 | C | TYR | B | 71 | 18.973 | 21.578 | 88.282 | 1.00 | 32.02 | C |
| ATOM | 2911 | O | TYR | B | 71 | 19.579 | 21.899 | 87.256 | 1.00 | 32.02 | O |
| ATOM | 2912 | N | GLN | B | 72 | 17.921 | 22.251 | 88.737 | 1.00 | 36.33 | N |
| ATOM | 2913 | CA | GLN | B | 72 | 17.466 | 23.453 | 88.060 | 1.00 | 36.33 | C |
| ATOM | 2914 | CB | GLN | B | 72 | 15.948 | 23.476 | 87.847 | 1.00 | 32.86 | C |
| ATOM | 2915 | CG | GLN | B | 72 | 15.505 | 24.775 | 87.157 | 1.00 | 32.86 | C |
| ATOM | 2916 | CD | GLN | B | 72 | 14.020 | 25.020 | 87.225 | 1.00 | 32.86 | C |
| ATOM | 2917 | OE1 | GLN | B | 72 | 13.359 | 25.164 | 86.201 | 1.00 | 32.86 | O |
| ATOM | 2918 | NE2 | GLN | B | 72 | 13.484 | 25.078 | 88.434 | 1.00 | 32.86 | N |
| ATOM | 2919 | C | GLN | B | 72 | 17.828 | 24.586 | 88.992 | 1.00 | 36.33 | C |
| ATOM | 2920 | O | GLN | B | 72 | 17.905 | 24.392 | 90.198 | 1.00 | 36.33 | O |
| ATOM | 2921 | N | ALA | B | 73 | 18.063 | 25.768 | 88.445 | 1.00 | 31.94 | N |
| ATOM | 2922 | CA | ALA | B | 73 | 18.391 | 26.900 | 89.289 | 1.00 | 31.94 | C |
| ATOM | 2923 | CB | ALA | B | 73 | 19.825 | 26.824 | 89.718 | 1.00 | 7.09 | C |
| ATOM | 2924 | C | ALA | B | 73 | 18.136 | 28.184 | 88.537 | 1.00 | 31.94 | C |
| ATOM | 2925 | O | ALA | B | 73 | 17.946 | 28.165 | 87.317 | 1.00 | 31.94 | O |
| ATOM | 2926 | N | LYS | B | 74 | 18.131 | 29.298 | 89.265 | 1.00 | 37.21 | N |
| ATOM | 2927 | CA | LYS | B | 74 | 17.884 | 30.594 | 88.652 | 1.00 | 37.21 | C |
| ATOM | 2928 | CB | LYS | B | 74 | 16.598 | 31.206 | 89.224 | 1.00 | 39.75 | C |
| ATOM | 2929 | CG | LYS | B | 74 | 16.014 | 32.389 | 88.448 | 1.00 | 39.75 | C |
| ATOM | 2930 | CD | LYS | B | 74 | 14.780 | 32.917 | 89.177 | 1.00 | 39.75 | C |
| ATOM | 2931 | CE | LYS | B | 74 | 14.094 | 34.023 | 88.411 | 1.00 | 39.75 | C |
| ATOM | 2932 | NZ | LYS | B | 74 | 12.978 | 34.613 | 89.195 | 1.00 | 39.75 | N |
| ATOM | 2933 | C | LYS | B | 74 | 19.062 | 31.521 | 88.893 | 1.00 | 37.21 | C |
| ATOM | 2934 | O | LYS | B | 74 | 19.469 | 31.744 | 90.033 | 1.00 | 37.21 | O |
| ATOM | 2935 | N | LEU | B | 75 | 19.613 | 32.041 | 87.801 | 1.00 | 31.31 | N |
| ATOM | 2936 | CA | LEU | B | 75 | 20.739 | 32.963 | 87.859 | 1.00 | 31.31 | C |
| ATOM | 2937 | CB | LEU | B | 75 | 21.384 | 33.113 | 86.476 | 1.00 | 25.85 | C |
| ATOM | 2938 | CG | LEU | B | 75 | 21.781 | 31.758 | 85.878 | 1.00 | 25.85 | C |
| ATOM | 2939 | CD1 | LEU | B | 75 | 22.488 | 31.953 | 84.553 | 1.00 | 25.85 | C |
| ATOM | 2940 | CD2 | LEU | B | 75 | 22.677 | 30.983 | 86.875 | 1.00 | 25.85 | C |
| ATOM | 2941 | C | LEU | B | 75 | 20.199 | 34.296 | 88.322 | 1.00 | 31.31 | C |
| ATOM | 2942 | O | LEU | B | 75 | 19.233 | 34.801 | 87.756 | 1.00 | 31.31 | O |
| ATOM | 2943 | N | CYS | B | 76 | 20.823 | 34.855 | 89.357 | 1.00 | 40.78 | N |
| ATOM | 2944 | CA | CYS | B | 76 | 20.409 | 36.135 | 89.931 | 1.00 | 40.78 | C |
| ATOM | 2945 | CB | CYS | B | 76 | 21.187 | 36.402 | 91.219 | 1.00 | 35.55 | C |
| ATOM | 2946 | SG | CYS | B | 76 | 21.279 | 35.009 | 92.333 | 1.00 | 35.55 | S |
| ATOM | 2947 | C | CYS | B | 76 | 20.705 | 37.245 | 88.942 | 1.00 | 40.78 | C |
| ATOM | 2948 | O | CYS | B | 76 | 20.009 | 38.263 | 88.873 | 1.00 | 40.78 | O |
| ATOM | 2949 | N | ASP | B | 77 | 21.761 | 37.015 | 88.176 | 1.00 | 60.55 | N |
| ATOM | 2950 | CA | ASP | B | 77 | 22.246 | 37.955 | 87.188 | 1.00 | 60.55 | C |
| ATOM | 2951 | CB | ASP | B | 77 | 23.452 | 37.338 | 86.460 | 1.00 | 84.67 | C |
| ATOM | 2952 | CG | ASP | B | 77 | 24.505 | 36.778 | 87.428 | 1.00 | 84.67 | C |
| ATOM | 2953 | OD1 | ASP | B | 77 | 24.439 | 35.574 | 87.765 | 1.00 | 84.67 | O |
| ATOM | 2954 | OD2 | ASP | B | 77 | 25.393 | 37.546 | 87.867 | 1.00 | 84.67 | O |
| ATOM | 2955 | C | ASP | B | 77 | 21.173 | 38.376 | 86.191 | 1.00 | 60.55 | C |
| ATOM | 2956 | O | ASP | B | 77 | 21.184 | 39.502 | 85.697 | 1.00 | 60.55 | O |

| | | | | | | | | | | | |
|---|---|---|---|---|---|---|---|---|---|---|---|
| ATOM | 2957 | N | SER | B | 78 | 20.228 | 37.486 | 85.912 | 1.00 | 67.21 | N |
| ATOM | 2958 | CA | SER | B | 78 | 19.186 | 37.798 | 84.942 | 1.00 | 67.21 | C |
| ATOM | 2959 | CB | SER | B | 78 | 19.666 | 37.418 | 83.550 | 1.00 | 51.67 | C |
| ATOM | 2960 | OG | SER | B | 78 | 19.956 | 36.030 | 83.504 | 1.00 | 51.67 | O |
| ATOM | 2961 | C | SER | B | 78 | 17.871 | 37.082 | 85.207 | 1.00 | 67.21 | C |
| ATOM | 2962 | O | SER | B | 78 | 16.928 | 37.208 | 84.424 | 1.00 | 67.21 | O |
| ATOM | 2963 | N | GLY | B | 79 | 17.812 | 36.321 | 86.295 | 1.00 | 48.03 | N |
| ATOM | 2964 | CA | GLY | B | 79 | 16.594 | 35.608 | 86.624 | 1.00 | 48.03 | C |
| ATOM | 2965 | C | GLY | B | 79 | 16.286 | 34.517 | 85.619 | 1.00 | 48.03 | C |
| ATOM | 2966 | O | GLY | B | 79 | 15.161 | 34.012 | 85.559 | 1.00 | 48.03 | O |
| ATOM | 2967 | N | GLU | B | 80 | 17.285 | 34.153 | 84.820 | 1.00 | 42.19 | N |
| ATOM | 2968 | CA | GLU | B | 80 | 17.107 | 33.103 | 83.825 | 1.00 | 42.19 | C |
| ATOM | 2969 | CB | GLU | B | 80 | 18.226 | 33.137 | 82.795 | 1.00 | 63.41 | C |
| ATOM | 2970 | CG | GLU | B | 80 | 18.342 | 34.413 | 82.014 | 1.00 | 63.41 | C |
| ATOM | 2971 | CD | GLU | B | 80 | 18.926 | 34.170 | 80.645 | 1.00 | 63.41 | C |
| ATOM | 2972 | OE1 | GLU | B | 80 | 18.189 | 33.640 | 79.794 | 1.00 | 63.41 | O |
| ATOM | 2973 | OE2 | GLU | B | 80 | 20.113 | 34.489 | 80.420 | 1.00 | 63.41 | O |
| ATOM | 2974 | C | GLU | B | 80 | 17.124 | 31.738 | 84.493 | 1.00 | 42.19 | C |
| ATOM | 2975 | O | GLU | B | 80 | 17.700 | 31.571 | 85.574 | 1.00 | 42.19 | O |
| ATOM | 2976 | N | LEU | B | 81 | 16.499 | 30.761 | 83.841 | 1.00 | 33.86 | N |
| ATOM | 2977 | CA | LEU | B | 81 | 16.465 | 29.391 | 84.361 | 1.00 | 33.86 | C |
| ATOM | 2978 | CB | LEU | B | 81 | 15.123 | 28.722 | 84.059 | 1.00 | 30.28 | C |
| ATOM | 2979 | CG | LEU | B | 81 | 14.138 | 28.724 | 85.233 | 1.00 | 30.28 | C |
| ATOM | 2980 | CD1 | LEU | B | 81 | 13.854 | 30.153 | 85.673 | 1.00 | 30.28 | C |
| ATOM | 2981 | CD2 | LEU | B | 81 | 12.844 | 28.029 | 84.816 | 1.00 | 30.28 | C |
| ATOM | 2982 | C | LEU | B | 81 | 17.585 | 28.567 | 83.754 | 1.00 | 33.86 | C |
| ATOM | 2983 | O | LEU | B | 81 | 17.845 | 28.656 | 82.556 | 1.00 | 33.86 | O |
| ATOM | 2984 | N | VAL | B | 82 | 18.260 | 27.773 | 84.574 | 1.00 | 40.41 | N |
| ATOM | 2985 | CA | VAL | B | 82 | 19.353 | 26.954 | 84.065 | 1.00 | 40.41 | C |
| ATOM | 2986 | CB | VAL | B | 82 | 20.750 | 27.617 | 84.269 | 1.00 | 25.85 | C |
| ATOM | 2987 | CG1 | VAL | B | 82 | 20.863 | 28.893 | 83.428 | 1.00 | 25.85 | C |
| ATOM | 2988 | CG2 | VAL | B | 82 | 20.975 | 27.918 | 85.750 | 1.00 | 25.85 | C |
| ATOM | 2989 | C | VAL | B | 82 | 19.394 | 25.601 | 84.724 | 1.00 | 40.41 | C |
| ATOM | 2990 | O | VAL | B | 82 | 18.787 | 25.370 | 85.774 | 1.00 | 40.41 | O |
| ATOM | 2991 | N | ALA | B | 83 | 20.135 | 24.708 | 84.089 | 1.00 | 26.94 | N |
| ATOM | 2992 | CA | ALA | B | 83 | 20.284 | 23.362 | 84.584 | 1.00 | 26.94 | C |
| ATOM | 2993 | CB | ALA | B | 83 | 19.811 | 22.385 | 83.536 | 1.00 | 21.59 | C |
| ATOM | 2994 | C | ALA | B | 83 | 21.741 | 23.097 | 84.940 | 1.00 | 26.94 | C |
| ATOM | 2995 | O | ALA | B | 83 | 22.650 | 23.451 | 84.188 | 1.00 | 26.94 | O |
| ATOM | 2996 | N | ILE | B | 84 | 21.953 | 22.468 | 86.091 | 1.00 | 26.70 | N |
| ATOM | 2997 | CA | ILE | B | 84 | 23.295 | 22.147 | 86.550 | 1.00 | 26.70 | C |
| ATOM | 2998 | CB | ILE | B | 84 | 23.552 | 22.727 | 87.964 | 1.00 | 23.72 | C |
| ATOM | 2999 | CG2 | ILE | B | 84 | 24.979 | 22.456 | 88.393 | 1.00 | 23.72 | C |
| ATOM | 3000 | CG1 | ILE | B | 84 | 23.301 | 24.230 | 87.971 | 1.00 | 23.72 | C |
| ATOM | 3001 | CD1 | ILE | B | 84 | 23.318 | 24.826 | 89.360 | 1.00 | 23.72 | C |
| ATOM | 3002 | C | ILE | B | 84 | 23.483 | 20.636 | 86.617 | 1.00 | 26.70 | C |
| ATOM | 3003 | O | ILE | B | 84 | 22.969 | 19.982 | 87.533 | 1.00 | 26.70 | O |
| ATOM | 3004 | N | LYS | B | 85 | 24.200 | 20.071 | 85.650 | 1.00 | 14.28 | N |
| ATOM | 3005 | CA | LYS | B | 85 | 24.448 | 18.634 | 85.681 | 1.00 | 14.28 | C |
| ATOM | 3006 | CB | LYS | B | 85 | 24.781 | 18.103 | 84.287 | 1.00 | 12.82 | C |
| ATOM | 3007 | CG | LYS | B | 85 | 25.163 | 16.611 | 84.269 | 1.00 | 12.82 | C |
| ATOM | 3008 | CD | LYS | B | 85 | 25.308 | 16.118 | 82.836 | 1.00 | 12.82 | C |
| ATOM | 3009 | CE | LYS | B | 85 | 25.941 | 14.747 | 82.756 | 1.00 | 12.82 | C |
| ATOM | 3010 | NZ | LYS | B | 85 | 26.016 | 14.221 | 81.350 | 1.00 | 12.82 | N |
| ATOM | 3011 | C | LYS | B | 85 | 25.633 | 18.414 | 86.629 | 1.00 | 14.28 | C |
| ATOM | 3012 | O | LYS | B | 85 | 26.714 | 18.972 | 86.425 | 1.00 | 14.28 | O |
| ATOM | 3013 | N | LYS | B | 86 | 25.429 | 17.623 | 87.679 | 1.00 | 22.41 | N |
| ATOM | 3014 | CA | LYS | B | 86 | 26.498 | 17.369 | 88.645 | 1.00 | 22.41 | C |
| ATOM | 3015 | CB | LYS | B | 86 | 25.974 | 17.602 | 90.068 | 1.00 | 44.09 | C |
| ATOM | 3016 | CG | LYS | B | 86 | 27.003 | 18.103 | 91.078 | 1.00 | 44.09 | C |
| ATOM | 3017 | CD | LYS | B | 86 | 26.444 | 18.014 | 92.501 | 1.00 | 44.09 | C |
| ATOM | 3018 | CE | LYS | B | 86 | 27.107 | 18.998 | 93.486 | 1.00 | 44.09 | C |
| ATOM | 3019 | NZ | LYS | B | 86 | 26.692 | 20.454 | 93.346 | 1.00 | 44.09 | N |

| | | | | | | | | | | | |
|---|---|---|---|---|---|---|---|---|---|---|---|
| ATOM | 3020 | C | LYS | B | 86 | 26.983 | 15.924 | 88.499 | 1.00 | 22.41 | C |
| ATOM | 3021 | O | LYS | B | 86 | 26.411 | 15.013 | 89.093 | 1.00 | 22.41 | O |
| ATOM | 3022 | N | VAL | B | 87 | 28.021 | 15.708 | 87.697 | 1.00 | 31.96 | N |
| ATOM | 3023 | CA | VAL | B | 87 | 28.540 | 14.350 | 87.518 | 1.00 | 31.96 | C |
| ATOM | 3024 | CB | VAL | B | 87 | 28.771 | 13.968 | 86.035 | 1.00 | 47.85 | C |
| ATOM | 3025 | CG1 | VAL | B | 87 | 27.458 | 13.865 | 85.313 | 1.00 | 47.85 | C |
| ATOM | 3026 | CG2 | VAL | B | 87 | 29.668 | 14.978 | 85.369 | 1.00 | 47.85 | C |
| ATOM | 3027 | C | VAL | B | 87 | 29.858 | 14.126 | 88.231 | 1.00 | 31.96 | C |
| ATOM | 3028 | O | VAL | B | 87 | 30.772 | 14.961 | 88.160 | 1.00 | 31.96 | O |
| ATOM | 3029 | N | LEU | B | 88 | 29.955 | 12.987 | 88.908 | 1.00 | 46.44 | N |
| ATOM | 3030 | CA | LEU | B | 88 | 31.160 | 12.631 | 89.642 | 1.00 | 46.44 | C |
| ATOM | 3031 | CB | LEU | B | 88 | 30.924 | 11.375 | 90.448 | 1.00 | 36.52 | C |
| ATOM | 3032 | CG | LEU | B | 88 | 32.145 | 10.785 | 91.120 | 1.00 | 36.52 | C |
| ATOM | 3033 | CD1 | LEU | B | 88 | 32.243 | 11.384 | 92.486 | 1.00 | 36.52 | C |
| ATOM | 3034 | CD2 | LEU | B | 88 | 32.009 | 9.292 | 91.205 | 1.00 | 36.52 | C |
| ATOM | 3035 | C | LEU | B | 88 | 32.302 | 12.365 | 88.693 | 1.00 | 46.44 | C |
| ATOM | 3036 | O | LEU | B | 88 | 32.077 | 11.987 | 87.545 | 1.00 | 46.44 | O |
| ATOM | 3037 | N | GLN | B | 89 | 33.525 | 12.554 | 89.174 | 1.00 | 54.38 | N |
| ATOM | 3038 | CA | GLN | B | 89 | 34.704 | 12.307 | 88.352 | 1.00 | 54.38 | C |
| ATOM | 3039 | CB | GLN | B | 89 | 35.708 | 13.430 | 88.515 | 1.00 | 54.83 | C |
| ATOM | 3040 | CG | GLN | B | 89 | 35.960 | 14.146 | 87.200 | 1.00 | 54.83 | C |
| ATOM | 3041 | CD | GLN | B | 89 | 34.696 | 14.769 | 86.609 | 1.00 | 54.83 | C |
| ATOM | 3042 | OE1 | GLN | B | 89 | 34.517 | 15.977 | 86.678 | 1.00 | 54.83 | O |
| ATOM | 3043 | NE2 | GLN | B | 89 | 33.828 | 13.952 | 86.018 | 1.00 | 54.83 | N |
| ATOM | 3044 | C | GLN | B | 89 | 35.357 | 10.992 | 88.728 | 1.00 | 54.38 | C |
| ATOM | 3045 | O | GLN | B | 89 | 35.556 | 10.726 | 89.909 | 1.00 | 54.38 | O |
| ATOM | 3046 | N | ASP | B | 90 | 35.706 | 10.171 | 87.746 | 1.00 | 32.91 | N |
| ATOM | 3047 | CA | ASP | B | 90 | 36.310 | 8.889 | 88.055 | 1.00 | 32.91 | C |
| ATOM | 3048 | CB | ASP | B | 90 | 35.359 | 7.760 | 87.664 | 1.00 | 43.97 | C |
| ATOM | 3049 | CG | ASP | B | 90 | 35.735 | 6.435 | 88.292 | 1.00 | 43.97 | C |
| ATOM | 3050 | OD1 | ASP | B | 90 | 34.871 | 5.535 | 88.314 | 1.00 | 43.97 | O |
| ATOM | 3051 | OD2 | ASP | B | 90 | 36.888 | 6.296 | 88.757 | 1.00 | 43.97 | O |
| ATOM | 3052 | C | ASP | B | 90 | 37.634 | 8.710 | 87.333 | 1.00 | 32.91 | C |
| ATOM | 3053 | O | ASP | B | 90 | 37.663 | 8.509 | 86.122 | 1.00 | 32.91 | O |
| ATOM | 3054 | N | GLY | B | 91 | 38.728 | 8.768 | 88.086 | 1.00 | 35.99 | N |
| ATOM | 3055 | CA | GLY | B | 91 | 40.045 | 8.599 | 87.501 | 1.00 | 35.99 | C |
| ATOM | 3056 | C | GLY | B | 91 | 40.157 | 7.275 | 86.775 | 1.00 | 35.99 | C |
| ATOM | 3057 | O | GLY | B | 91 | 41.046 | 7.101 | 85.938 | 1.00 | 35.99 | O |
| ATOM | 3058 | N | ALA | B | 92 | 39.301 | 6.317 | 87.110 | 1.00 | 53.18 | N |
| ATOM | 3059 | CA | ALA | B | 92 | 39.384 | 5.035 | 86.429 | 1.00 | 53.18 | C |
| ATOM | 3060 | CB | ALA | B | 92 | 38.983 | 3.905 | 87.366 | 1.00 | 7.11 | C |
| ATOM | 3061 | C | ALA | B | 92 | 38.561 | 4.955 | 85.150 | 1.00 | 53.18 | C |
| ATOM | 3062 | O | ALA | B | 92 | 38.445 | 3.877 | 84.552 | 1.00 | 53.18 | O |
| ATOM | 3063 | N | PHE | B | 93 | 38.017 | 6.078 | 84.700 | 1.00 | 60.78 | N |
| ATOM | 3064 | CA | PHE | B | 93 | 37.207 | 6.028 | 83.500 | 1.00 | 60.78 | C |
| ATOM | 3065 | CB | PHE | B | 93 | 35.729 | 6.025 | 83.880 | 1.00 | 66.95 | C |
| ATOM | 3066 | CG | PHE | B | 93 | 35.181 | 4.628 | 84.079 | 1.00 | 66.95 | C |
| ATOM | 3067 | CD1 | PHE | B | 93 | 35.196 | 3.735 | 83.003 | 1.00 | 66.95 | C |
| ATOM | 3068 | CD2 | PHE | B | 93 | 34.717 | 4.165 | 85.323 | 1.00 | 66.95 | C |
| ATOM | 3069 | CE1 | PHE | B | 93 | 34.767 | 2.400 | 83.132 | 1.00 | 66.95 | C |
| ATOM | 3070 | CE2 | PHE | B | 93 | 34.280 | 2.815 | 85.465 | 1.00 | 66.95 | C |
| ATOM | 3071 | CZ | PHE | B | 93 | 34.309 | 1.937 | 84.363 | 1.00 | 66.95 | C |
| ATOM | 3072 | C | PHE | B | 93 | 37.523 | 7.018 | 82.389 | 1.00 | 60.78 | C |
| ATOM | 3073 | O | PHE | B | 93 | 37.186 | 6.756 | 81.245 | 1.00 | 60.78 | O |
| ATOM | 3074 | N | LYS | B | 94 | 38.207 | 8.120 | 82.696 | 1.00 | 50.24 | N |
| ATOM | 3075 | CA | LYS | B | 94 | 38.605 | 9.088 | 81.658 | 1.00 | 50.24 | C |
| ATOM | 3076 | CB | LYS | B | 94 | 39.615 | 8.378 | 80.760 | 1.00 | 23.68 | C |
| ATOM | 3077 | CG | LYS | B | 94 | 39.579 | 8.815 | 79.303 | 1.00 | 23.68 | C |
| ATOM | 3078 | CD | LYS | B | 94 | 40.047 | 7.735 | 78.353 | 1.00 | 23.68 | C |
| ATOM | 3079 | CE | LYS | B | 94 | 41.355 | 8.205 | 77.772 | 1.00 | 23.68 | C |
| ATOM | 3080 | NZ | LYS | B | 94 | 42.134 | 7.017 | 77.310 | 1.00 | 23.68 | N |
| ATOM | 3081 | C | LYS | B | 94 | 37.547 | 9.807 | 80.777 | 1.00 | 50.24 | C |
| ATOM | 3082 | O | LYS | B | 94 | 37.468 | 9.607 | 79.568 | 1.00 | 50.24 | O |

| | | | | | | | | | | | |
|---|---|---|---|---|---|---|---|---|---|---|---|
| ATOM | 3083 | N | ASN | B | 95 | 36.775 | 10.704 | 81.354 | 1.00 | 44.11 | N |
| ATOM | 3084 | CA | ASN | B | 95 | 35.752 | 11.384 | 80.573 | 1.00 | 44.11 | C |
| ATOM | 3085 | CB | ASN | B | 95 | 34.560 | 11.529 | 81.516 | 1.00 | 78.25 | C |
| ATOM | 3086 | CG | ASN | B | 95 | 33.546 | 12.539 | 81.079 | 1.00 | 78.25 | C |
| ATOM | 3087 | OD1 | ASN | B | 95 | 33.769 | 13.326 | 80.178 | 1.00 | 78.25 | O |
| ATOM | 3088 | ND2 | ASN | B | 95 | 32.411 | 12.546 | 81.769 | 1.00 | 78.25 | N |
| ATOM | 3089 | C | ASN | B | 95 | 36.203 | 12.728 | 79.946 | 1.00 | 44.11 | C |
| ATOM | 3090 | O | ASN | B | 95 | 37.100 | 13.424 | 80.449 | 1.00 | 44.11 | O |
| ATOM | 3091 | N | ARG | B | 96 | 35.564 | 13.060 | 78.825 | 1.00 | 26.55 | N |
| ATOM | 3092 | CA | ARG | B | 96 | 35.813 | 14.298 | 78.078 | 1.00 | 26.55 | C |
| ATOM | 3093 | CB | ARG | B | 96 | 36.466 | 13.945 | 76.761 | 1.00 | 33.08 | C |
| ATOM | 3094 | CG | ARG | B | 96 | 37.100 | 12.620 | 76.832 | 1.00 | 33.08 | C |
| ATOM | 3095 | CD | ARG | B | 96 | 36.967 | 11.918 | 75.533 | 1.00 | 33.08 | C |
| ATOM | 3096 | NE | ARG | B | 96 | 37.892 | 10.785 | 75.467 | 1.00 | 33.08 | N |
| ATOM | 3097 | CZ | ARG | B | 96 | 37.566 | 9.488 | 75.571 | 1.00 | 33.08 | C |
| ATOM | 3098 | NH1 | ARG | B | 96 | 36.302 | 9.097 | 75.755 | 1.00 | 33.08 | N |
| ATOM | 3099 | NH2 | ARG | B | 96 | 38.513 | 8.554 | 75.458 | 1.00 | 33.08 | N |
| ATOM | 3100 | C | ARG | B | 96 | 34.525 | 15.074 | 77.814 | 1.00 | 26.55 | C |
| ATOM | 3101 | O | ARG | B | 96 | 34.411 | 15.803 | 76.839 | 1.00 | 26.55 | O |
| ATOM | 3102 | N | GLU | B | 97 | 33.575 | 14.921 | 78.719 | 1.00 | 32.59 | N |
| ATOM | 3103 | CA | GLU | B | 97 | 32.283 | 15.545 | 78.591 | 1.00 | 32.59 | C |
| ATOM | 3104 | CB | GLU | B | 97 | 31.373 | 15.095 | 79.736 | 1.00 | 28.80 | C |
| ATOM | 3105 | CG | GLU | B | 97 | 30.284 | 16.055 | 80.070 | 1.00 | 28.80 | C |
| ATOM | 3106 | CD | GLU | B | 97 | 29.060 | 15.362 | 80.588 | 1.00 | 28.80 | C |
| ATOM | 3107 | OE1 | GLU | B | 97 | 29.182 | 14.587 | 81.569 | 1.00 | 28.80 | O |
| ATOM | 3108 | OE2 | GLU | B | 97 | 27.980 | 15.608 | 80.001 | 1.00 | 28.80 | O |
| ATOM | 3109 | C | GLU | B | 97 | 32.472 | 17.032 | 78.598 | 1.00 | 32.59 | C |
| ATOM | 3110 | O | GLU | B | 97 | 31.763 | 17.760 | 77.915 | 1.00 | 32.59 | O |
| ATOM | 3111 | N | LEU | B | 98 | 33.459 | 17.483 | 79.361 | 1.00 | 38.44 | N |
| ATOM | 3112 | CA | LEU | B | 98 | 33.723 | 18.906 | 79.450 | 1.00 | 38.44 | C |
| ATOM | 3113 | CB | LEU | B | 98 | 34.512 | 19.228 | 80.717 | 1.00 | 17.39 | C |
| ATOM | 3114 | CG | LEU | B | 98 | 35.068 | 20.657 | 80.823 | 1.00 | 17.39 | C |
| ATOM | 3115 | CD1 | LEU | B | 98 | 33.998 | 21.713 | 80.602 | 1.00 | 17.39 | C |
| ATOM | 3116 | CD2 | LEU | B | 98 | 35.684 | 20.821 | 82.201 | 1.00 | 17.39 | C |
| ATOM | 3117 | C | LEU | B | 98 | 34.457 | 19.406 | 78.218 | 1.00 | 38.44 | C |
| ATOM | 3118 | O | LEU | B | 98 | 34.146 | 20.478 | 77.714 | 1.00 | 38.44 | O |
| ATOM | 3119 | N | GLN | B | 99 | 35.420 | 18.631 | 77.726 | 1.00 | 30.31 | N |
| ATOM | 3120 | CA | GLN | B | 99 | 36.166 | 19.022 | 76.540 | 1.00 | 30.31 | C |
| ATOM | 3121 | CB | GLN | B | 99 | 37.125 | 17.924 | 76.079 | 1.00 | 95.46 | C |
| ATOM | 3122 | CG | GLN | B | 99 | 38.147 | 17.428 | 77.070 | 1.00 | 95.46 | C |
| ATOM | 3123 | CD | GLN | B | 99 | 38.976 | 16.297 | 76.486 | 1.00 | 95.46 | C |
| ATOM | 3124 | OE1 | GLN | B | 99 | 39.723 | 15.622 | 77.195 | 1.00 | 95.46 | O |
| ATOM | 3125 | NE2 | GLN | B | 99 | 38.844 | 16.086 | 75.178 | 1.00 | 95.46 | N |
| ATOM | 3126 | C | GLN | B | 99 | 35.185 | 19.240 | 75.407 | 1.00 | 30.31 | C |
| ATOM | 3127 | O | GLN | B | 99 | 34.969 | 20.366 | 74.970 | 1.00 | 30.31 | O |
| ATOM | 3128 | N | ILE | B | 100 | 34.606 | 18.137 | 74.928 | 1.00 | 26.38 | N |
| ATOM | 3129 | CA | ILE | B | 100 | 33.667 | 18.168 | 73.808 | 1.00 | 26.38 | C |
| ATOM | 3130 | CB | ILE | B | 100 | 32.885 | 16.833 | 73.675 | 1.00 | 55.67 | C |
| ATOM | 3131 | CG2 | ILE | B | 100 | 31.710 | 17.007 | 72.713 | 1.00 | 55.67 | C |
| ATOM | 3132 | CG1 | ILE | B | 100 | 33.805 | 15.727 | 73.154 | 1.00 | 55.67 | C |
| ATOM | 3133 | CD1 | ILE | B | 100 | 34.936 | 15.388 | 74.067 | 1.00 | 55.67 | C |
| ATOM | 3134 | C | ILE | B | 100 | 32.666 | 19.293 | 73.963 | 1.00 | 26.38 | C |
| ATOM | 3135 | O | ILE | B | 100 | 32.635 | 20.235 | 73.169 | 1.00 | 26.38 | O |
| ATOM | 3136 | N | MET | B | 101 | 31.856 | 19.168 | 75.005 | 1.00 | 31.19 | N |
| ATOM | 3137 | CA | MET | B | 101 | 30.824 | 20.129 | 75.332 | 1.00 | 31.19 | C |
| ATOM | 3138 | CB | MET | B | 101 | 30.387 | 19.897 | 76.775 | 1.00 | 36.33 | C |
| ATOM | 3139 | CG | MET | B | 101 | 29.223 | 20.731 | 77.234 | 1.00 | 36.33 | C |
| ATOM | 3140 | SD | MET | B | 101 | 27.649 | 19.924 | 76.910 | 1.00 | 36.33 | S |
| ATOM | 3141 | CE | MET | B | 101 | 27.572 | 18.699 | 78.247 | 1.00 | 36.33 | C |
| ATOM | 3142 | C | MET | B | 101 | 31.343 | 21.562 | 75.165 | 1.00 | 31.19 | C |
| ATOM | 3143 | O | MET | B | 101 | 30.717 | 22.411 | 74.523 | 1.00 | 31.19 | O |
| ATOM | 3144 | N | ARG | B | 102 | 32.522 | 21.806 | 75.714 | 1.00 | 51.94 | N |
| ATOM | 3145 | CA | ARG | B | 102 | 33.113 | 23.128 | 75.696 | 1.00 | 51.94 | C |

| | | | | | | | | | | | |
|---|---|---|---|---|---|---|---|---|---|---|---|
| ATOM | 3146 | CB | ARG | B | 102 | 34.308 | 23.137 | 76.638 | 1.00 | 41.63 | C |
| ATOM | 3147 | CG | ARG | B | 102 | 34.577 | 24.479 | 77.221 | 1.00 | 41.63 | C |
| ATOM | 3148 | CD | ARG | B | 102 | 35.794 | 24.453 | 78.085 | 1.00 | 41.63 | C |
| ATOM | 3149 | NE | ARG | B | 102 | 36.151 | 25.812 | 78.447 | 1.00 | 41.63 | N |
| ATOM | 3150 | CZ | ARG | B | 102 | 37.225 | 26.141 | 79.152 | 1.00 | 41.63 | C |
| ATOM | 3151 | NH1 | ARG | B | 102 | 38.060 | 25.204 | 79.577 | 1.00 | 41.63 | N |
| ATOM | 3152 | NH2 | ARG | B | 102 | 37.461 | 27.416 | 79.437 | 1.00 | 41.63 | N |
| ATOM | 3153 | C | ARG | B | 102 | 33.507 | 23.733 | 74.346 | 1.00 | 51.94 | C |
| ATOM | 3154 | O | ARG | B | 102 | 34.016 | 24.851 | 74.304 | 1.00 | 51.94 | O |
| ATOM | 3155 | N | LYS | B | 103 | 33.281 | 23.027 | 73.245 | 1.00 | 26.57 | N |
| ATOM | 3156 | CA | LYS | B | 103 | 33.630 | 23.591 | 71.936 | 1.00 | 26.57 | C |
| ATOM | 3157 | CB | LYS | B | 103 | 34.864 | 22.878 | 71.344 | 1.00 | 56.85 | C |
| ATOM | 3158 | CG | LYS | B | 103 | 34.652 | 21.432 | 70.916 | 1.00 | 56.85 | C |
| ATOM | 3159 | CD | LYS | B | 103 | 35.806 | 20.876 | 70.028 | 1.00 | 56.85 | C |
| ATOM | 3160 | CE | LYS | B | 103 | 37.091 | 20.549 | 70.806 | 1.00 | 56.85 | C |
| ATOM | 3161 | NZ | LYS | B | 103 | 37.883 | 19.445 | 70.175 | 1.00 | 56.85 | N |
| ATOM | 3162 | C | LYS | B | 103 | 32.437 | 23.518 | 70.965 | 1.00 | 26.57 | C |
| ATOM | 3163 | O | LYS | B | 103 | 32.508 | 23.953 | 69.815 | 1.00 | 26.57 | O |
| ATOM | 3164 | N | LEU | B | 104 | 31.325 | 22.987 | 71.457 | 1.00 | 31.99 | N |
| ATOM | 3165 | CA | LEU | B | 104 | 30.123 | 22.834 | 70.654 | 1.00 | 31.99 | C |
| ATOM | 3166 | CB | LEU | B | 104 | 29.308 | 21.656 | 71.197 | 1.00 | 29.97 | C |
| ATOM | 3167 | CG | LEU | B | 104 | 29.487 | 20.266 | 70.573 | 1.00 | 29.97 | C |
| ATOM | 3168 | CD1 | LEU | B | 104 | 30.932 | 19.993 | 70.247 | 1.00 | 29.97 | C |
| ATOM | 3169 | CD2 | LEU | B | 104 | 28.944 | 19.230 | 71.529 | 1.00 | 29.97 | C |
| ATOM | 3170 | C | LEU | B | 104 | 29.260 | 24.089 | 70.617 | 1.00 | 31.99 | C |
| ATOM | 3171 | O | LEU | B | 104 | 29.121 | 24.795 | 71.608 | 1.00 | 31.99 | O |
| ATOM | 3172 | N | ASP | B | 105 | 28.684 | 24.369 | 69.459 | 1.00 | 41.97 | N |
| ATOM | 3173 | CA | ASP | B | 105 | 27.821 | 25.529 | 69.321 | 1.00 | 41.97 | C |
| ATOM | 3174 | CB | ASP | B | 105 | 28.638 | 26.777 | 69.021 | 1.00 | 60.41 | C |
| ATOM | 3175 | CG | ASP | B | 105 | 27.794 | 28.042 | 69.057 | 1.00 | 60.41 | C |
| ATOM | 3176 | OD1 | ASP | B | 105 | 26.645 | 28.018 | 68.566 | 1.00 | 60.41 | O |
| ATOM | 3177 | OD2 | ASP | B | 105 | 28.278 | 29.071 | 69.571 | 1.00 | 60.41 | O |
| ATOM | 3178 | C | ASP | B | 105 | 26.834 | 25.300 | 68.188 | 1.00 | 41.97 | C |
| ATOM | 3179 | O | ASP | B | 105 | 27.127 | 25.621 | 67.033 | 1.00 | 41.97 | O |
| ATOM | 3180 | N | HIS | B | 106 | 25.668 | 24.743 | 68.515 | 1.00 | 28.68 | N |
| ATOM | 3181 | CA | HIS | B | 106 | 24.645 | 24.470 | 67.511 | 1.00 | 28.68 | C |
| ATOM | 3182 | CB | HIS | B | 106 | 24.797 | 23.041 | 66.974 | 1.00 | 26.89 | C |
| ATOM | 3183 | CG | HIS | B | 106 | 24.005 | 22.777 | 65.733 | 1.00 | 26.89 | C |
| ATOM | 3184 | CD2 | HIS | B | 106 | 24.383 | 22.696 | 64.434 | 1.00 | 26.89 | C |
| ATOM | 3185 | ND1 | HIS | B | 106 | 22.641 | 22.590 | 65.747 | 1.00 | 26.89 | N |
| ATOM | 3186 | CE1 | HIS | B | 106 | 22.214 | 22.406 | 64.509 | 1.00 | 26.89 | C |
| ATOM | 3187 | NE2 | HIS | B | 106 | 23.251 | 22.467 | 63.694 | 1.00 | 26.89 | N |
| ATOM | 3188 | C | HIS | B | 106 | 23.266 | 24.676 | 68.128 | 1.00 | 28.68 | C |
| ATOM | 3189 | O | HIS | B | 106 | 23.070 | 24.455 | 69.328 | 1.00 | 28.68 | O |
| ATOM | 3190 | N | CYS | B | 107 | 22.317 | 25.116 | 67.307 | 1.00 | 37.82 | N |
| ATOM | 3191 | CA | CYS | B | 107 | 20.963 | 25.380 | 67.779 | 1.00 | 37.82 | C |
| ATOM | 3192 | CB | CYS | B | 107 | 20.161 | 26.072 | 66.690 | 1.00 | 38.39 | C |
| ATOM | 3193 | SG | CYS | B | 107 | 20.322 | 25.302 | 65.093 | 1.00 | 38.39 | S |
| ATOM | 3194 | C | CYS | B | 107 | 20.248 | 24.121 | 68.219 | 1.00 | 37.82 | C |
| ATOM | 3195 | O | CYS | B | 107 | 19.193 | 24.171 | 68.858 | 1.00 | 37.82 | O |
| ATOM | 3196 | N | ASN | B | 108 | 20.828 | 22.981 | 67.884 | 1.00 | 45.79 | N |
| ATOM | 3197 | CA | ASN | B | 108 | 20.227 | 21.723 | 68.262 | 1.00 | 45.79 | C |
| ATOM | 3198 | CB | ASN | B | 108 | 19.880 | 20.938 | 67.003 | 1.00 | 30.50 | C |
| ATOM | 3199 | CG | ASN | B | 108 | 18.728 | 21.567 | 66.240 | 1.00 | 30.50 | C |
| ATOM | 3200 | OD1 | ASN | B | 108 | 18.808 | 21.772 | 65.032 | 1.00 | 30.50 | O |
| ATOM | 3201 | ND2 | ASN | B | 108 | 17.641 | 21.873 | 66.952 | 1.00 | 30.50 | N |
| ATOM | 3202 | C | ASN | B | 108 | 21.149 | 20.950 | 69.188 | 1.00 | 45.79 | C |
| ATOM | 3203 | O | ASN | B | 108 | 21.339 | 19.738 | 69.061 | 1.00 | 45.79 | O |
| ATOM | 3204 | N | ILE | B | 109 | 21.718 | 21.689 | 70.130 | 1.00 | 27.52 | N |
| ATOM | 3205 | CA | ILE | B | 109 | 22.617 | 21.135 | 71.128 | 1.00 | 27.52 | C |
| ATOM | 3206 | CB | ILE | B | 109 | 24.080 | 21.016 | 70.565 | 1.00 | 27.17 | C |
| ATOM | 3207 | CG2 | ILE | B | 109 | 25.051 | 20.528 | 71.666 | 1.00 | 27.17 | C |
| ATOM | 3208 | CG1 | ILE | B | 109 | 24.097 | 20.041 | 69.382 | 1.00 | 27.17 | C |

| | | | | | | | | | | | |
|---|---|---|---|---|---|---|---|---|---|---|---|
| ATOM | 3209 | CD1 | ILE | B | 109 | 25.460 | 19.812 | 68.791 | 1.00 | 27.17 | C |
| ATOM | 3210 | C | ILE | B | 109 | 22.578 | 22.073 | 72.330 | 1.00 | 27.52 | C |
| ATOM | 3211 | O | ILE | B | 109 | 22.895 | 23.251 | 72.203 | 1.00 | 27.52 | O |
| ATOM | 3212 | N | VAL | B | 110 | 22.171 | 21.568 | 73.489 | 1.00 | 27.96 | N |
| ATOM | 3213 | CA | VAL | B | 110 | 22.118 | 22.417 | 74.674 | 1.00 | 27.96 | C |
| ATOM | 3214 | CB | VAL | B | 110 | 21.880 | 21.608 | 75.969 | 1.00 | 27.90 | C |
| ATOM | 3215 | CG1 | VAL | B | 110 | 20.396 | 21.328 | 76.133 | 1.00 | 27.90 | C |
| ATOM | 3216 | CG2 | VAL | B | 110 | 22.668 | 20.307 | 75.936 | 1.00 | 27.90 | C |
| ATOM | 3217 | C | VAL | B | 110 | 23.409 | 23.198 | 74.823 | 1.00 | 27.96 | C |
| ATOM | 3218 | O | VAL | B | 110 | 24.483 | 22.697 | 74.535 | 1.00 | 27.96 | O |
| ATOM | 3219 | N | ARG | B | 111 | 23.297 | 24.440 | 75.263 | 1.00 | 31.01 | N |
| ATOM | 3220 | CA | ARG | B | 111 | 24.470 | 25.269 | 75.426 | 1.00 | 31.01 | C |
| ATOM | 3221 | CB | ARG | B | 111 | 24.131 | 26.710 | 75.095 | 1.00 | 52.45 | C |
| ATOM | 3222 | CG | ARG | B | 111 | 25.329 | 27.616 | 75.005 | 1.00 | 52.45 | C |
| ATOM | 3223 | CD | ARG | B | 111 | 24.842 | 29.021 | 74.885 | 1.00 | 52.45 | C |
| ATOM | 3224 | NE | ARG | B | 111 | 23.787 | 29.247 | 75.862 | 1.00 | 52.45 | N |
| ATOM | 3225 | CZ | ARG | B | 111 | 23.136 | 30.395 | 75.998 | 1.00 | 52.45 | C |
| ATOM | 3226 | NH1 | ARG | B | 111 | 23.436 | 31.423 | 75.216 | 1.00 | 52.45 | N |
| ATOM | 3227 | NH2 | ARG | B | 111 | 22.182 | 30.517 | 76.910 | 1.00 | 52.45 | N |
| ATOM | 3228 | C | ARG | B | 111 | 25.034 | 25.201 | 76.833 | 1.00 | 31.01 | C |
| ATOM | 3229 | O | ARG | B | 111 | 24.289 | 25.116 | 77.812 | 1.00 | 31.01 | O |
| ATOM | 3230 | N | LEU | B | 112 | 26.362 | 25.232 | 76.911 | 1.00 | 34.91 | N |
| ATOM | 3231 | CA | LEU | B | 112 | 27.082 | 25.202 | 78.179 | 1.00 | 34.91 | C |
| ATOM | 3232 | CB | LEU | B | 112 | 28.418 | 24.457 | 78.027 | 1.00 | 33.62 | C |
| ATOM | 3233 | CG | LEU | B | 112 | 29.458 | 24.514 | 79.151 | 1.00 | 33.62 | C |
| ATOM | 3234 | CD1 | LEU | B | 112 | 28.892 | 23.921 | 80.439 | 1.00 | 33.62 | C |
| ATOM | 3235 | CD2 | LEU | B | 112 | 30.701 | 23.762 | 78.702 | 1.00 | 33.62 | C |
| ATOM | 3236 | C | LEU | B | 112 | 27.343 | 26.652 | 78.530 | 1.00 | 34.91 | C |
| ATOM | 3237 | O | LEU | B | 112 | 28.089 | 27.350 | 77.838 | 1.00 | 34.91 | O |
| ATOM | 3238 | N | ARG | B | 113 | 26.707 | 27.109 | 79.598 | 1.00 | 32.97 | N |
| ATOM | 3239 | CA | ARG | B | 113 | 26.870 | 28.478 | 80.036 | 1.00 | 32.97 | C |
| ATOM | 3240 | CB | ARG | B | 113 | 25.708 | 28.871 | 80.932 | 1.00 | 38.81 | C |
| ATOM | 3241 | CG | ARG | B | 113 | 24.378 | 28.840 | 80.238 | 1.00 | 38.81 | C |
| ATOM | 3242 | CD | ARG | B | 113 | 23.735 | 30.205 | 80.321 | 1.00 | 38.81 | C |
| ATOM | 3243 | NE | ARG | B | 113 | 22.294 | 30.162 | 80.099 | 1.00 | 38.81 | N |
| ATOM | 3244 | CZ | ARG | B | 113 | 21.518 | 31.242 | 80.078 | 1.00 | 38.81 | C |
| ATOM | 3245 | NH1 | ARG | B | 113 | 22.046 | 32.448 | 80.266 | 1.00 | 38.81 | N |
| ATOM | 3246 | NH2 | ARG | B | 113 | 20.215 | 31.115 | 79.866 | 1.00 | 38.81 | N |
| ATOM | 3247 | C | ARG | B | 113 | 28.179 | 28.591 | 80.795 | 1.00 | 32.97 | C |
| ATOM | 3248 | O | ARG | B | 113 | 29.114 | 29.259 | 80.351 | 1.00 | 32.97 | O |
| ATOM | 3249 | N | TYR | B | 114 | 28.235 | 27.914 | 81.935 | 1.00 | 35.36 | N |
| ATOM | 3250 | CA | TYR | B | 114 | 29.411 | 27.912 | 82.793 | 1.00 | 35.36 | C |
| ATOM | 3251 | CB | TYR | B | 114 | 29.144 | 28.762 | 84.036 | 1.00 | 46.62 | C |
| ATOM | 3252 | CG | TYR | B | 114 | 28.731 | 30.187 | 83.734 | 1.00 | 46.62 | C |
| ATOM | 3253 | CD1 | TYR | B | 114 | 29.671 | 31.137 | 83.296 | 1.00 | 46.62 | C |
| ATOM | 3254 | CE1 | TYR | B | 114 | 29.298 | 32.459 | 83.036 | 1.00 | 46.62 | C |
| ATOM | 3255 | CD2 | TYR | B | 114 | 27.402 | 30.596 | 83.899 | 1.00 | 46.62 | C |
| ATOM | 3256 | CE2 | TYR | B | 114 | 27.014 | 31.918 | 83.640 | 1.00 | 46.62 | C |
| ATOM | 3257 | CZ | TYR | B | 114 | 27.968 | 32.845 | 83.211 | 1.00 | 46.62 | C |
| ATOM | 3258 | OH | TYR | B | 114 | 27.593 | 34.156 | 82.973 | 1.00 | 46.62 | O |
| ATOM | 3259 | C | TYR | B | 114 | 29.723 | 26.483 | 83.222 | 1.00 | 35.36 | C |
| ATOM | 3260 | O | TYR | B | 114 | 29.025 | 25.541 | 82.852 | 1.00 | 35.36 | O |
| ATOM | 3261 | N | PHE | B | 115 | 30.784 | 26.329 | 83.998 | 1.00 | 31.05 | N |
| ATOM | 3262 | CA | PHE | B | 115 | 31.169 | 25.028 | 84.514 | 1.00 | 31.05 | C |
| ATOM | 3263 | CB | PHE | B | 115 | 31.819 | 24.187 | 83.419 | 1.00 | 39.53 | C |
| ATOM | 3264 | CG | PHE | B | 115 | 33.229 | 24.589 | 83.103 | 1.00 | 39.53 | C |
| ATOM | 3265 | CD1 | PHE | B | 115 | 34.288 | 24.111 | 83.865 | 1.00 | 39.53 | C |
| ATOM | 3266 | CD2 | PHE | B | 115 | 33.501 | 25.451 | 82.039 | 1.00 | 39.53 | C |
| ATOM | 3267 | CE1 | PHE | B | 115 | 35.596 | 24.481 | 83.570 | 1.00 | 39.53 | C |
| ATOM | 3268 | CE2 | PHE | B | 115 | 34.812 | 25.831 | 81.734 | 1.00 | 39.53 | C |
| ATOM | 3269 | CZ | PHE | B | 115 | 35.860 | 25.346 | 82.497 | 1.00 | 39.53 | C |
| ATOM | 3270 | C | PHE | B | 115 | 32.140 | 25.256 | 85.667 | 1.00 | 31.05 | C |
| ATOM | 3271 | O | PHE | B | 115 | 32.957 | 26.177 | 85.646 | 1.00 | 31.05 | O |

| | | | | | | | | | | |
|---|---|---|---|---|---|---|---|---|---|---|
| ATOM | 3272 | N | PHE | B | 116 | 32.031 | 24.425 | 86.690 | 1.00 20.29 | N |
| ATOM | 3273 | CA | PHE | B | 116 | 32.902 | 24.555 | 87.843 | 1.00 20.29 | C |
| ATOM | 3274 | CB | PHE | B | 116 | 32.341 | 25.596 | 88.826 | 1.00 35.64 | C |
| ATOM | 3275 | CG | PHE | B | 116 | 31.017 | 25.218 | 89.432 | 1.00 35.64 | C |
| ATOM | 3276 | CD1 | PHE | B | 116 | 30.955 | 24.467 | 90.601 | 1.00 35.64 | C |
| ATOM | 3277 | CD2 | PHE | B | 116 | 29.828 | 25.621 | 88.832 | 1.00 35.64 | C |
| ATOM | 3278 | CE1 | PHE | B | 116 | 29.730 | 24.128 | 91.164 | 1.00 35.64 | C |
| ATOM | 3279 | CE2 | PHE | B | 116 | 28.593 | 25.282 | 89.390 | 1.00 35.64 | C |
| ATOM | 3280 | CZ | PHE | B | 116 | 28.545 | 24.534 | 90.560 | 1.00 35.64 | C |
| ATOM | 3281 | C | PHE | B | 116 | 33.044 | 23.210 | 88.518 | 1.00 20.29 | C |
| ATOM | 3282 | O | PHE | B | 116 | 32.332 | 22.252 | 88.182 | 1.00 20.29 | O |
| ATOM | 3283 | N | TYR | B | 117 | 33.985 | 23.123 | 89.450 | 1.00 34.16 | N |
| ATOM | 3284 | CA | TYR | B | 117 | 34.185 | 21.867 | 90.159 | 1.00 34.16 | C |
| ATOM | 3285 | CB | TYR | B | 117 | 35.629 | 21.381 | 90.020 | 1.00 36.67 | C |
| ATOM | 3286 | CG | TYR | B | 117 | 35.959 | 20.855 | 88.645 | 1.00 36.67 | C |
| ATOM | 3287 | CD1 | TYR | B | 117 | 35.809 | 19.509 | 88.336 | 1.00 36.67 | C |
| ATOM | 3288 | CE1 | TYR | B | 117 | 36.084 | 19.035 | 87.052 | 1.00 36.67 | C |
| ATOM | 3289 | CD2 | TYR | B | 117 | 36.390 | 21.716 | 87.641 | 1.00 36.67 | C |
| ATOM | 3290 | CE2 | TYR | B | 117 | 36.661 | 21.254 | 86.359 | 1.00 36.67 | C |
| ATOM | 3291 | CZ | TYR | B | 117 | 36.507 | 19.923 | 86.069 | 1.00 36.67 | C |
| ATOM | 3292 | OH | TYR | B | 117 | 36.769 | 19.514 | 84.784 | 1.00 36.67 | O |
| ATOM | 3293 | C | TYR | B | 117 | 33.829 | 22.070 | 91.614 | 1.00 34.16 | C |
| ATOM | 3294 | O | TYR | B | 117 | 33.924 | 23.183 | 92.140 | 1.00 34.16 | O |
| ATOM | 3295 | N | SER | B | 118 | 33.400 | 20.991 | 92.255 | 1.00 27.15 | N |
| ATOM | 3296 | CA | SER | B | 118 | 33.005 | 21.062 | 93.654 | 1.00 27.15 | C |
| ATOM | 3297 | CB | SER | B | 118 | 31.557 | 21.555 | 93.799 | 1.00 33.36 | C |
| ATOM | 3298 | OG | SER | B | 118 | 30.617 | 20.594 | 93.371 | 1.00 33.36 | O |
| ATOM | 3299 | C | SER | B | 118 | 33.138 | 19.723 | 94.321 | 1.00 27.15 | C |
| ATOM | 3300 | O | SER | B | 118 | 33.285 | 18.698 | 93.652 | 1.00 27.15 | O |
| ATOM | 3301 | N | SER | B | 119 | 33.043 | 19.751 | 95.646 | 1.00 54.51 | N |
| ATOM | 3302 | CA | SER | B | 119 | 33.184 | 18.554 | 96.451 | 1.00 54.51 | C |
| ATOM | 3303 | CB | SER | B | 119 | 33.882 | 18.870 | 97.780 | 1.00 50.46 | C |
| ATOM | 3304 | OG | SER | B | 119 | 34.772 | 19.981 | 97.663 | 1.00 50.46 | O |
| ATOM | 3305 | C | SER | B | 119 | 31.783 | 18.085 | 96.699 | 1.00 54.51 | C |
| ATOM | 3306 | O | SER | B | 119 | 30.906 | 18.888 | 96.922 | 1.00 54.51 | O |
| ATOM | 3307 | N | GLY | B | 120 | 31.570 | 16.785 | 96.610 | 1.00 75.25 | N |
| ATOM | 3308 | CA | GLY | B | 120 | 30.240 | 16.240 | 96.828 | 1.00 75.25 | C |
| ATOM | 3309 | C | GLY | B | 120 | 30.412 | 14.987 | 97.657 | 1.00 75.25 | C |
| ATOM | 3310 | O | GLY | B | 120 | 31.389 | 14.950 | 98.395 | 1.00 75.25 | O |
| ATOM | 3311 | N | GLY | B | 121 | 29.530 | 13.981 | 97.527 | 1.00 82.88 | N |
| ATOM | 3312 | CA | GLY | B | 121 | 29.627 | 12.734 | 98.305 | 1.00 82.88 | C |
| ATOM | 3313 | C | GLY | B | 121 | 30.891 | 12.680 | 99.150 | 1.00 82.88 | C |
| ATOM | 3314 | O | GLY | B | 121 | 31.811 | 11.914 | 98.827 | 1.00 82.88 | O |
| ATOM | 3315 | N | ALA | B | 122 | 30.913 | 13.477 | 100.232 | 1.00 92.89 | N |
| ATOM | 3316 | CA | ALA | B | 122 | 32.080 | 13.624 | 101.098 | 1.00 92.89 | C |
| ATOM | 3317 | CB | ALA | B | 122 | 31.672 | 13.916 | 102.511 | 1.00 83.51 | C |
| ATOM | 3318 | C | ALA | B | 122 | 33.002 | 12.398 | 101.033 | 1.00 92.89 | C |
| ATOM | 3319 | O | ALA | B | 122 | 32.644 | 11.253 | 101.243 | 1.00 92.89 | O |
| ATOM | 3320 | N | GLY | B | 123 | 34.220 | 12.711 | 100.660 | 1.00 88.35 | N |
| ATOM | 3321 | CA | GLY | B | 123 | 35.271 | 11.741 | 100.494 | 1.00 88.35 | C |
| ATOM | 3322 | C | GLY | B | 123 | 36.279 | 12.431 | 99.606 | 1.00 88.35 | C |
| ATOM | 3323 | O | GLY | B | 123 | 36.562 | 13.589 | 99.879 | 1.00 88.35 | O |
| ATOM | 3324 | N | ASP | B | 124 | 36.832 | 11.758 | 98.593 | 1.00 73.41 | N |
| ATOM | 3325 | CA | ASP | B | 124 | 37.838 | 12.363 | 97.700 | 1.00 73.41 | C |
| ATOM | 3326 | CB | ASP | B | 124 | 38.848 | 11.318 | 97.224 | 1.00 26.37 | C |
| ATOM | 3327 | CG | ASP | B | 124 | 40.163 | 11.425 | 97.943 | 1.00 23.68 | C |
| ATOM | 3328 | OD1 | ASP | B | 124 | 40.460 | 12.567 | 98.409 | 1.00 23.68 | O |
| ATOM | 3329 | OD2 | ASP | B | 124 | 40.889 | 10.394 | 98.036 | 1.00 23.68 | O |
| ATOM | 3330 | C | ASP | B | 124 | 37.226 | 12.963 | 96.439 | 1.00 73.41 | C |
| ATOM | 3331 | O | ASP | B | 124 | 37.670 | 14.002 | 95.918 | 1.00 73.41 | O |
| ATOM | 3332 | N | ALA | B | 125 | 36.218 | 12.277 | 95.926 | 1.00 58.67 | N |
| ATOM | 3333 | CA | ALA | B | 125 | 35.577 | 12.661 | 94.683 | 1.00 58.67 | C |
| ATOM | 3334 | CB | ALA | B | 125 | 34.427 | 11.807 | 94.481 | 1.00 58.02 | C |

| | | | | | | | | | | | |
|---|---|---|---|---|---|---|---|---|---|---|---|
| ATOM | 3335 | C | ALA | B | 125 | 35.200 | 14.119 | 94.473 | 1.00 | 58.67 | C |
| ATOM | 3336 | O | ALA | B | 125 | 34.553 | 14.782 | 95.309 | 1.00 | 58.67 | O |
| ATOM | 3337 | N | VAL | B | 126 | 35.664 | 14.581 | 93.324 | 1.00 | 42.31 | N |
| ATOM | 3338 | CA | VAL | B | 126 | 35.474 | 15.912 | 92.837 | 1.00 | 42.31 | C |
| ATOM | 3339 | CB | VAL | B | 126 | 36.717 | 16.361 | 92.032 | 1.00 | 48.86 | C |
| ATOM | 3340 | CG1 | VAL | B | 126 | 37.054 | 17.737 | 92.369 | 1.00 | 48.86 | C |
| ATOM | 3341 | CG2 | VAL | B | 126 | 37.900 | 15.438 | 92.299 | 1.00 | 48.86 | C |
| ATOM | 3342 | C | VAL | B | 126 | 34.294 | 15.783 | 91.910 | 1.00 | 42.31 | C |
| ATOM | 3343 | O | VAL | B | 126 | 34.088 | 14.715 | 91.341 | 1.00 | 42.31 | O |
| ATOM | 3344 | N | TYR | B | 127 | 33.523 | 16.855 | 91.772 | 1.00 | 42.86 | N |
| ATOM | 3345 | CA | TYR | B | 127 | 32.378 | 16.821 | 90.890 | 1.00 | 42.86 | C |
| ATOM | 3346 | CB | TYR | B | 127 | 31.075 | 17.032 | 91.658 | 1.00 | 54.30 | C |
| ATOM | 3347 | CG | TYR | B | 127 | 30.576 | 15.820 | 92.412 | 1.00 | 54.30 | C |
| ATOM | 3348 | CD1 | TYR | B | 127 | 31.054 | 15.519 | 93.685 | 1.00 | 54.30 | C |
| ATOM | 3349 | CE1 | TYR | B | 127 | 30.570 | 14.424 | 94.395 | 1.00 | 54.30 | C |
| ATOM | 3350 | CD2 | TYR | B | 127 | 29.604 | 14.987 | 91.863 | 1.00 | 54.30 | C |
| ATOM | 3351 | CE2 | TYR | B | 127 | 29.117 | 13.889 | 92.564 | 1.00 | 54.30 | C |
| ATOM | 3352 | CZ | TYR | B | 127 | 29.598 | 13.615 | 93.830 | 1.00 | 54.30 | C |
| ATOM | 3353 | OH | TYR | B | 127 | 29.082 | 12.552 | 94.539 | 1.00 | 54.30 | O |
| ATOM | 3354 | C | TYR | B | 127 | 32.491 | 17.894 | 89.837 | 1.00 | 42.86 | C |
| ATOM | 3355 | O | TYR | B | 127 | 33.006 | 18.985 | 90.107 | 1.00 | 42.86 | O |
| ATOM | 3356 | N | LEU | B | 128 | 32.025 | 17.573 | 88.633 | 1.00 | 28.51 | N |
| ATOM | 3357 | CA | LEU | B | 128 | 32.027 | 18.537 | 87.545 | 1.00 | 28.51 | C |
| ATOM | 3358 | CB | LEU | B | 128 | 32.364 | 17.884 | 86.205 | 1.00 | 22.80 | C |
| ATOM | 3359 | CG | LEU | B | 128 | 32.181 | 18.793 | 84.982 | 1.00 | 22.80 | C |
| ATOM | 3360 | CD1 | LEU | B | 128 | 33.110 | 20.001 | 85.078 | 1.00 | 22.80 | C |
| ATOM | 3361 | CD2 | LEU | B | 128 | 32.447 | 17.996 | 83.711 | 1.00 | 22.80 | C |
| ATOM | 3362 | C | LEU | B | 128 | 30.609 | 19.061 | 87.516 | 1.00 | 28.51 | C |
| ATOM | 3363 | O | LEU | B | 128 | 29.665 | 18.282 | 87.637 | 1.00 | 28.51 | O |
| ATOM | 3364 | N | ASN | B | 129 | 30.466 | 20.377 | 87.389 | 1.00 | 35.31 | N |
| ATOM | 3365 | CA | ASN | B | 129 | 29.157 | 21.017 | 87.357 | 1.00 | 35.31 | C |
| ATOM | 3366 | CB | ASN | B | 129 | 29.030 | 22.019 | 88.496 | 1.00 | 33.40 | C |
| ATOM | 3367 | CG | ASN | B | 129 | 29.017 | 21.364 | 89.851 | 1.00 | 33.40 | C |
| ATOM | 3368 | OD1 | ASN | B | 129 | 27.995 | 20.845 | 90.289 | 1.00 | 33.40 | O |
| ATOM | 3369 | ND2 | ASN | B | 129 | 30.158 | 21.382 | 90.526 | 1.00 | 33.40 | N |
| ATOM | 3370 | C | ASN | B | 129 | 28.962 | 21.748 | 86.051 | 1.00 | 35.31 | C |
| ATOM | 3371 | O | ASN | B | 129 | 29.594 | 22.771 | 85.802 | 1.00 | 35.31 | O |
| ATOM | 3372 | N | LEU | B | 130 | 28.088 | 21.221 | 85.214 | 1.00 | 34.22 | N |
| ATOM | 3373 | CA | LEU | B | 130 | 27.806 | 21.857 | 83.942 | 1.00 | 34.22 | C |
| ATOM | 3374 | CB | LEU | B | 130 | 27.634 | 20.801 | 82.853 | 1.00 | 14.79 | C |
| ATOM | 3375 | CG | LEU | B | 130 | 28.854 | 19.896 | 82.725 | 1.00 | 14.79 | C |
| ATOM | 3376 | CD1 | LEU | B | 130 | 28.535 | 18.727 | 81.790 | 1.00 | 14.79 | C |
| ATOM | 3377 | CD2 | LEU | B | 130 | 30.055 | 20.722 | 82.238 | 1.00 | 14.79 | C |
| ATOM | 3378 | C | LEU | B | 130 | 26.541 | 22.705 | 84.055 | 1.00 | 34.22 | C |
| ATOM | 3379 | O | LEU | B | 130 | 25.448 | 22.193 | 84.329 | 1.00 | 34.22 | O |
| ATOM | 3380 | N | VAL | B | 131 | 26.705 | 24.010 | 83.869 | 1.00 | 35.73 | N |
| ATOM | 3381 | CA | VAL | B | 131 | 25.583 | 24.929 | 83.918 | 1.00 | 35.73 | C |
| ATOM | 3382 | CB | VAL | B | 131 | 26.006 | 26.275 | 84.483 | 1.00 | 31.72 | C |
| ATOM | 3383 | CG1 | VAL | B | 131 | 24.790 | 27.132 | 84.710 | 1.00 | 31.72 | C |
| ATOM | 3384 | CG2 | VAL | B | 131 | 26.772 | 26.066 | 85.768 | 1.00 | 31.72 | C |
| ATOM | 3385 | C | VAL | B | 131 | 25.136 | 25.088 | 82.472 | 1.00 | 35.73 | C |
| ATOM | 3386 | O | VAL | B | 131 | 25.834 | 25.696 | 81.652 | 1.00 | 35.73 | O |
| ATOM | 3387 | N | LEU | B | 132 | 23.979 | 24.513 | 82.165 | 1.00 | 38.34 | N |
| ATOM | 3388 | CA | LEU | B | 132 | 23.436 | 24.538 | 80.811 | 1.00 | 38.34 | C |
| ATOM | 3389 | CB | LEU | B | 132 | 23.156 | 23.105 | 80.329 | 1.00 | 19.98 | C |
| ATOM | 3390 | CG | LEU | B | 132 | 24.010 | 21.954 | 80.862 | 1.00 | 19.98 | C |
| ATOM | 3391 | CD1 | LEU | B | 132 | 23.292 | 20.658 | 80.574 | 1.00 | 19.98 | C |
| ATOM | 3392 | CD2 | LEU | B | 132 | 25.400 | 21.971 | 80.244 | 1.00 | 19.98 | C |
| ATOM | 3393 | C | LEU | B | 132 | 22.131 | 25.295 | 80.775 | 1.00 | 38.34 | C |
| ATOM | 3394 | O | LEU | B | 132 | 21.614 | 25.705 | 81.807 | 1.00 | 38.34 | O |
| ATOM | 3395 | N | ASP | B | 133 | 21.598 | 25.471 | 79.571 | 1.00 | 41.35 | N |
| ATOM | 3396 | CA | ASP | B | 133 | 20.309 | 26.125 | 79.413 | 1.00 | 41.35 | C |
| ATOM | 3397 | CB | ASP | B | 133 | 19.992 | 26.351 | 77.929 | 1.00 | 42.55 | C |

| | | | | | | | | | | | |
|---|---|---|---|---|---|---|---|---|---|---|---|
| ATOM | 3398 | CG | ASP | B | 133 | 20.573 | 27.655 | 77.393 | 1.00 | 42.55 | C |
| ATOM | 3399 | OD1 | ASP | B | 133 | 20.630 | 27.806 | 76.155 | 1.00 | 42.55 | O |
| ATOM | 3400 | OD2 | ASP | B | 133 | 20.959 | 28.533 | 78.199 | 1.00 | 42.55 | O |
| ATOM | 3401 | C | ASP | B | 133 | 19.293 | 25.162 | 80.016 | 1.00 | 41.35 | C |
| ATOM | 3402 | O | ASP | B | 133 | 19.589 | 23.982 | 80.241 | 1.00 | 41.35 | O |
| ATOM | 3403 | N | TYR | B | 134 | 18.101 | 25.658 | 80.305 | 1.00 | 29.27 | N |
| ATOM | 3404 | CA | TYR | B | 134 | 17.087 | 24.781 | 80.860 | 1.00 | 29.27 | C |
| ATOM | 3405 | CB | TYR | B | 134 | 16.374 | 25.463 | 82.019 | 1.00 | 28.48 | C |
| ATOM | 3406 | CG | TYR | B | 134 | 15.280 | 24.619 | 82.581 | 1.00 | 28.48 | C |
| ATOM | 3407 | CD1 | TYR | B | 134 | 15.570 | 23.419 | 83.224 | 1.00 | 28.48 | C |
| ATOM | 3408 | CE1 | TYR | B | 134 | 14.553 | 22.585 | 83.669 | 1.00 | 28.48 | C |
| ATOM | 3409 | CD2 | TYR | B | 134 | 13.942 | 24.970 | 82.402 | 1.00 | 28.48 | C |
| ATOM | 3410 | CE2 | TYR | B | 134 | 12.924 | 24.145 | 82.844 | 1.00 | 28.48 | C |
| ATOM | 3411 | CZ | TYR | B | 134 | 13.231 | 22.956 | 83.473 | 1.00 | 28.48 | C |
| ATOM | 3412 | OH | TYR | B | 134 | 12.220 | 22.129 | 83.905 | 1.00 | 28.48 | O |
| ATOM | 3413 | C | TYR | B | 134 | 16.082 | 24.376 | 79.776 | 1.00 | 29.27 | C |
| ATOM | 3414 | O | TYR | B | 134 | 15.660 | 25.198 | 78.968 | 1.00 | 29.27 | O |
| ATOM | 3415 | N | VAL | B | 135 | 15.721 | 23.099 | 79.752 | 1.00 | 34.03 | N |
| ATOM | 3416 | CA | VAL | B | 135 | 14.774 | 22.597 | 78.767 | 1.00 | 34.03 | C |
| ATOM | 3417 | CB | VAL | B | 135 | 15.502 | 21.836 | 77.631 | 1.00 | 31.23 | C |
| ATOM | 3418 | CG1 | VAL | B | 135 | 14.545 | 21.597 | 76.472 | 1.00 | 31.23 | C |
| ATOM | 3419 | CG2 | VAL | B | 135 | 16.707 | 22.631 | 77.152 | 1.00 | 31.23 | C |
| ATOM | 3420 | C | VAL | B | 135 | 13.771 | 21.675 | 79.466 | 1.00 | 34.03 | C |
| ATOM | 3421 | O | VAL | B | 135 | 14.117 | 20.586 | 79.916 | 1.00 | 34.03 | O |
| ATOM | 3422 | N | PRO | B | 136 | 12.497 | 22.102 | 79.531 | 1.00 | 35.64 | N |
| ATOM | 3423 | CD | PRO | B | 136 | 12.032 | 23.176 | 78.638 | 1.00 | 54.78 | C |
| ATOM | 3424 | CA | PRO | B | 136 | 11.342 | 21.438 | 80.152 | 1.00 | 35.64 | C |
| ATOM | 3425 | CB | PRO | B | 136 | 10.154 | 22.218 | 79.586 | 1.00 | 54.78 | C |
| ATOM | 3426 | CG | PRO | B | 136 | 10.727 | 23.532 | 79.257 | 1.00 | 54.78 | C |
| ATOM | 3427 | C | PRO | B | 136 | 11.182 | 19.929 | 79.940 | 1.00 | 35.64 | C |
| ATOM | 3428 | O | PRO | B | 136 | 11.312 | 19.139 | 80.883 | 1.00 | 35.64 | O |
| ATOM | 3429 | N | GLU | B | 137 | 10.899 | 19.532 | 78.701 | 1.00 | 30.25 | N |
| ATOM | 3430 | CA | GLU | B | 137 | 10.679 | 18.126 | 78.402 | 1.00 | 30.25 | C |
| ATOM | 3431 | CB | GLU | B | 137 | 9.303 | 17.953 | 77.738 | 1.00 | 48.55 | C |
| ATOM | 3432 | CG | GLU | B | 137 | 8.123 | 18.447 | 78.582 | 1.00 | 48.55 | C |
| ATOM | 3433 | CD | GLU | B | 137 | 7.879 | 17.610 | 79.824 | 1.00 | 48.55 | C |
| ATOM | 3434 | OE1 | GLU | B | 137 | 6.923 | 17.919 | 80.557 | 1.00 | 48.55 | O |
| ATOM | 3435 | OE2 | GLU | B | 137 | 8.636 | 16.646 | 80.074 | 1.00 | 48.55 | O |
| ATOM | 3436 | C | GLU | B | 137 | 11.756 | 17.437 | 77.567 | 1.00 | 30.25 | C |
| ATOM | 3437 | O | GLU | B | 137 | 12.781 | 18.026 | 77.206 | 1.00 | 30.25 | O |
| ATOM | 3438 | N | THR | B | 138 | 11.524 | 16.156 | 77.307 | 1.00 | 26.46 | N |
| ATOM | 3439 | CA | THR | B | 138 | 12.436 | 15.353 | 76.503 | 1.00 | 26.46 | C |
| ATOM | 3440 | CB | THR | B | 138 | 13.272 | 14.362 | 77.350 | 1.00 | 49.15 | C |
| ATOM | 3441 | OG1 | THR | B | 138 | 12.419 | 13.356 | 77.910 | 1.00 | 49.15 | O |
| ATOM | 3442 | CG2 | THR | B | 138 | 13.981 | 15.079 | 78.456 | 1.00 | 49.15 | C |
| ATOM | 3443 | C | THR | B | 138 | 11.626 | 14.521 | 75.525 | 1.00 | 26.46 | C |
| ATOM | 3444 | O | THR | B | 138 | 10.451 | 14.254 | 75.742 | 1.00 | 26.46 | O |
| ATOM | 3445 | N | VAL | B | 139 | 12.258 | 14.109 | 74.441 | 1.00 | 35.89 | N |
| ATOM | 3446 | CA | VAL | B | 139 | 11.581 | 13.283 | 73.462 | 1.00 | 35.89 | C |
| ATOM | 3447 | CB | VAL | B | 139 | 12.571 | 12.749 | 72.424 | 1.00 | 28.93 | C |
| ATOM | 3448 | CG1 | VAL | B | 139 | 11.918 | 11.657 | 71.595 | 1.00 | 28.93 | C |
| ATOM | 3449 | CG2 | VAL | B | 139 | 13.055 | 13.907 | 71.545 | 1.00 | 28.93 | C |
| ATOM | 3450 | C | VAL | B | 139 | 10.953 | 12.104 | 74.185 | 1.00 | 35.89 | C |
| ATOM | 3451 | O | VAL | B | 139 | 9.770 | 11.799 | 74.009 | 1.00 | 35.89 | O |
| ATOM | 3452 | N | TYR | B | 140 | 11.756 | 11.451 | 75.013 | 1.00 | 35.57 | N |
| ATOM | 3453 | CA | TYR | B | 140 | 11.289 | 10.298 | 75.759 | 1.00 | 35.57 | C |
| ATOM | 3454 | CB | TYR | B | 140 | 12.404 | 9.779 | 76.654 | 1.00 | 39.41 | C |
| ATOM | 3455 | CG | TYR | B | 140 | 11.935 | 8.723 | 77.602 | 1.00 | 39.41 | C |
| ATOM | 3456 | CD1 | TYR | B | 140 | 11.721 | 7.399 | 77.179 | 1.00 | 39.41 | C |
| ATOM | 3457 | CE1 | TYR | B | 140 | 11.172 | 6.449 | 78.047 | 1.00 | 39.41 | C |
| ATOM | 3458 | CD2 | TYR | B | 140 | 11.598 | 9.063 | 78.907 | 1.00 | 39.41 | C |
| ATOM | 3459 | CE2 | TYR | B | 140 | 11.055 | 8.136 | 79.768 | 1.00 | 39.41 | C |
| ATOM | 3460 | CZ | TYR | B | 140 | 10.843 | 6.836 | 79.340 | 1.00 | 39.41 | C |

| | | | | | | | | | | | |
|---|---|---|---|---|---|---|---|---|---|---|---|
| ATOM | 3461 | OH | TYR | B | 140 | 10.320 | 5.934 | 80.235 | 1.00 | 39.41 | O |
| ATOM | 3462 | C | TYR | B | 140 | 10.044 | 10.569 | 76.609 | 1.00 | 35.57 | C |
| ATOM | 3463 | O | TYR | B | 140 | 9.077 | 9.817 | 76.541 | 1.00 | 35.57 | O |
| ATOM | 3464 | N | ARG | B | 141 | 10.074 | 11.635 | 77.410 | 1.00 | 38.35 | N |
| ATOM | 3465 | CA | ARG | B | 141 | 8.952 | 11.974 | 78.279 | 1.00 | 38.35 | C |
| ATOM | 3466 | CB | ARG | B | 141 | 9.285 | 13.228 | 79.107 | 1.00 | 81.94 | C |
| ATOM | 3467 | CG | ARG | B | 141 | 8.522 | 13.334 | 80.428 | 1.00 | 81.94 | C |
| ATOM | 3468 | CD | ARG | B | 141 | 9.369 | 14.061 | 81.464 | 1.00 | 81.94 | C |
| ATOM | 3469 | NE | ARG | B | 141 | 10.718 | 13.491 | 81.540 | 1.00 | 81.94 | N |
| ATOM | 3470 | CZ | ARG | B | 141 | 11.032 | 12.347 | 82.151 | 1.00 | 81.94 | C |
| ATOM | 3471 | NH1 | ARG | B | 141 | 10.098 | 11.627 | 82.764 | 1.00 | 81.94 | N |
| ATOM | 3472 | NH2 | ARG | B | 141 | 12.285 | 11.908 | 82.132 | 1.00 | 81.94 | N |
| ATOM | 3473 | C | ARG | B | 141 | 7.685 | 12.182 | 77.447 | 1.00 | 38.35 | C |
| ATOM | 3474 | O | ARG | B | 141 | 6.599 | 11.747 | 77.829 | 1.00 | 38.35 | O |
| ATOM | 3475 | N | VAL | B | 142 | 7.834 | 12.827 | 76.298 | 1.00 | 31.20 | N |
| ATOM | 3476 | CA | VAL | B | 142 | 6.713 | 13.088 | 75.400 | 1.00 | 31.20 | C |
| ATOM | 3477 | CB | VAL | B | 142 | 7.111 | 14.098 | 74.300 | 1.00 | 36.06 | C |
| ATOM | 3478 | CG1 | VAL | B | 142 | 6.010 | 14.199 | 73.260 | 1.00 | 36.06 | C |
| ATOM | 3479 | CG2 | VAL | B | 142 | 7.384 | 15.468 | 74.923 | 1.00 | 36.06 | C |
| ATOM | 3480 | C | VAL | B | 142 | 6.236 | 11.814 | 74.716 | 1.00 | 31.20 | C |
| ATOM | 3481 | O | VAL | B | 142 | 5.044 | 11.608 | 74.526 | 1.00 | 31.20 | O |
| ATOM | 3482 | N | ALA | B | 143 | 7.180 | 10.964 | 74.338 | 1.00 | 43.38 | N |
| ATOM | 3483 | CA | ALA | B | 143 | 6.837 | 9.729 | 73.671 | 1.00 | 43.38 | C |
| ATOM | 3484 | CB | ALA | B | 143 | 8.089 | 9.050 | 73.159 | 1.00 | 18.36 | C |
| ATOM | 3485 | C | ALA | B | 143 | 6.088 | 8.824 | 74.641 | 1.00 | 43.38 | C |
| ATOM | 3486 | O | ALA | B | 143 | 5.077 | 8.223 | 74.272 | 1.00 | 43.38 | O |
| ATOM | 3487 | N | ARG | B | 144 | 6.567 | 8.728 | 75.881 | 1.00 | 36.24 | N |
| ATOM | 3488 | CA | ARG | B | 144 | 5.895 | 7.878 | 76.866 | 1.00 | 36.24 | C |
| ATOM | 3489 | CB | ARG | B | 144 | 6.693 | 7.774 | 78.159 | 1.00 | 77.18 | C |
| ATOM | 3490 | CG | ARG | B | 144 | 7.286 | 6.397 | 78.412 | 1.00 | 77.18 | C |
| ATOM | 3491 | CD | ARG | B | 144 | 6.776 | 5.851 | 79.727 | 1.00 | 77.18 | C |
| ATOM | 3492 | NE | ARG | B | 144 | 5.463 | 5.219 | 79.596 | 1.00 | 77.18 | N |
| ATOM | 3493 | CZ | ARG | B | 144 | 5.237 | 4.146 | 78.841 | 1.00 | 77.18 | C |
| ATOM | 3494 | NH1 | ARG | B | 144 | 6.244 | 3.591 | 78.156 | 1.00 | 77.18 | N |
| ATOM | 3495 | NH2 | ARG | B | 144 | 4.011 | 3.639 | 78.742 | 1.00 | 77.18 | N |
| ATOM | 3496 | C | ARG | B | 144 | 4.516 | 8.439 | 77.158 | 1.00 | 36.24 | C |
| ATOM | 3497 | O | ARG | B | 144 | 3.605 | 7.705 | 77.547 | 1.00 | 36.24 | O |
| ATOM | 3498 | N | HIS | B | 145 | 4.356 | 9.742 | 76.963 | 1.00 | 43.14 | N |
| ATOM | 3499 | CA | HIS | B | 145 | 3.062 | 10.339 | 77.193 | 1.00 | 43.14 | C |
| ATOM | 3500 | CB | HIS | B | 145 | 3.084 | 11.829 | 76.891 | 1.00 | 57.22 | C |
| ATOM | 3501 | CG | HIS | B | 145 | 1.731 | 12.450 | 76.953 | 1.00 | 57.22 | C |
| ATOM | 3502 | CD2 | HIS | B | 145 | 1.111 | 13.140 | 77.938 | 1.00 | 57.22 | C |
| ATOM | 3503 | ND1 | HIS | B | 145 | 0.797 | 12.285 | 75.954 | 1.00 | 57.22 | N |
| ATOM | 3504 | CE1 | HIS | B | 145 | -0.342 | 12.843 | 76.322 | 1.00 | 57.22 | C |
| ATOM | 3505 | NE2 | HIS | B | 145 | -0.177 | 13.369 | 77.523 | 1.00 | 57.22 | N |
| ATOM | 3506 | C | HIS | B | 145 | 2.047 | 9.638 | 76.290 | 1.00 | 43.14 | C |
| ATOM | 3507 | O | HIS | B | 145 | 1.079 | 9.052 | 76.768 | 1.00 | 43.14 | O |
| ATOM | 3508 | N | TYR | B | 146 | 2.282 | 9.693 | 74.984 | 1.00 | 44.84 | N |
| ATOM | 3509 | CA | TYR | B | 146 | 1.399 | 9.054 | 74.012 | 1.00 | 44.84 | C |
| ATOM | 3510 | CB | TYR | B | 146 | 1.893 | 9.328 | 72.588 | 1.00 | 40.49 | C |
| ATOM | 3511 | CG | TYR | B | 146 | 1.634 | 10.740 | 72.117 | 1.00 | 40.49 | C |
| ATOM | 3512 | CD1 | TYR | B | 146 | 2.305 | 11.819 | 72.679 | 1.00 | 40.49 | C |
| ATOM | 3513 | CE1 | TYR | B | 146 | 2.023 | 13.119 | 72.280 | 1.00 | 40.49 | C |
| ATOM | 3514 | CD2 | TYR | B | 146 | 0.674 | 11.001 | 71.137 | 1.00 | 40.49 | C |
| ATOM | 3515 | CE2 | TYR | B | 146 | 0.384 | 12.295 | 70.738 | 1.00 | 40.49 | C |
| ATOM | 3516 | CZ | TYR | B | 146 | 1.058 | 13.342 | 71.311 | 1.00 | 40.49 | C |
| ATOM | 3517 | OH | TYR | B | 146 | 0.740 | 14.615 | 70.922 | 1.00 | 40.49 | O |
| ATOM | 3518 | C | TYR | B | 146 | 1.206 | 7.545 | 74.202 | 1.00 | 44.84 | C |
| ATOM | 3519 | O | TYR | B | 146 | 0.081 | 7.082 | 74.320 | 1.00 | 44.84 | O |
| ATOM | 3520 | N | SER | B | 147 | 2.279 | 6.767 | 74.221 | 1.00 | 47.13 | N |
| ATOM | 3521 | CA | SER | B | 147 | 2.122 | 5.325 | 74.395 | 1.00 | 47.13 | C |
| ATOM | 3522 | CB | SER | B | 147 | 3.496 | 4.655 | 74.464 | 1.00 | 88.12 | C |
| ATOM | 3523 | OG | SER | B | 147 | 3.369 | 3.245 | 74.561 | 1.00 | 88.12 | O |

| | | | | | | | | | | | |
|---|---|---|---|---|---|---|---|---|---|---|---|
| ATOM | 3524 | C | SER | B | 147 | 1.309 | 4.967 | 75.653 | 1.00 | 47.13 | C |
| ATOM | 3525 | O | SER | B | 147 | 0.631 | 3.936 | 75.699 | 1.00 | 47.13 | O |
| ATOM | 3526 | N | ARG | B | 148 | 1.391 | 5.825 | 76.668 | 1.00 | 62.04 | N |
| ATOM | 3527 | CA | ARG | B | 148 | 0.681 | 5.618 | 77.934 | 1.00 | 62.04 | C |
| ATOM | 3528 | CB | ARG | B | 148 | 1.170 | 6.633 | 78.959 | 1.00 | 54.51 | C |
| ATOM | 3529 | CG | ARG | B | 148 | 1.290 | 6.057 | 80.357 | 1.00 | 54.51 | C |
| ATOM | 3530 | CD | ARG | B | 148 | 1.881 | 7.050 | 81.351 | 1.00 | 54.51 | C |
| ATOM | 3531 | NE | ARG | B | 148 | 3.345 | 7.071 | 81.339 | 1.00 | 54.51 | N |
| ATOM | 3532 | CZ | ARG | B | 148 | 4.075 | 8.187 | 81.353 | 1.00 | 54.51 | C |
| ATOM | 3533 | NH1 | ARG | B | 148 | 3.483 | 9.389 | 81.362 | 1.00 | 54.51 | N |
| ATOM | 3534 | NH2 | ARG | B | 148 | 5.402 | 8.111 | 81.403 | 1.00 | 54.51 | N |
| ATOM | 3535 | C | ARG | B | 148 | -0.775 | 5.886 | 77.586 | 1.00 | 62.04 | C |
| ATOM | 3536 | O | ARG | B | 148 | -1.721 | 5.230 | 78.044 | 1.00 | 62.04 | O |
| ATOM | 3537 | N | ALA | B | 149 | -0.891 | 6.858 | 76.695 | 1.00 | 61.50 | N |
| ATOM | 3538 | CA | ALA | B | 149 | -2.147 | 7.362 | 76.161 | 1.00 | 61.50 | C |
| ATOM | 3539 | CB | ALA | B | 149 | -1.927 | 8.744 | 75.504 | 1.00 | 42.34 | C |
| ATOM | 3540 | C | ALA | B | 149 | -2.704 | 6.426 | 75.144 | 1.00 | 61.50 | C |
| ATOM | 3541 | O | ALA | B | 149 | -3.685 | 6.746 | 74.478 | 1.00 | 61.50 | O |
| ATOM | 3542 | N | LYS | B | 150 | -2.061 | 5.274 | 75.023 | 1.00 | 43.70 | N |
| ATOM | 3543 | CA | LYS | B | 150 | -2.481 | 4.261 | 74.073 | 1.00 | 43.70 | C |
| ATOM | 3544 | CB | LYS | B | 150 | -3.828 | 3.677 | 74.523 | 1.00 | 58.15 | C |
| ATOM | 3545 | CG | LYS | B | 150 | -3.730 | 2.400 | 75.351 | 1.00 | 58.15 | C |
| ATOM | 3546 | CD | LYS | B | 150 | -2.328 | 2.156 | 75.876 | 1.00 | 58.15 | C |
| ATOM | 3547 | CE | LYS | B | 150 | -2.382 | 1.331 | 77.157 | 1.00 | 58.15 | C |
| ATOM | 3548 | NZ | LYS | B | 150 | -3.609 | 0.457 | 77.209 | 1.00 | 58.15 | N |
| ATOM | 3549 | C | LYS | B | 150 | -2.580 | 4.794 | 72.634 | 1.00 | 43.70 | C |
| ATOM | 3550 | O | LYS | B | 150 | -3.221 | 4.178 | 71.781 | 1.00 | 43.70 | O |
| ATOM | 3551 | N | GLN | B | 151 | -1.923 | 5.913 | 72.357 | 1.00 | 77.00 | N |
| ATOM | 3552 | CA | GLN | B | 151 | -1.980 | 6.522 | 71.038 | 1.00 | 77.00 | C |
| ATOM | 3553 | CB | GLN | B | 151 | -2.780 | 7.816 | 71.110 | 1.00 | 91.71 | C |
| ATOM | 3554 | CG | GLN | B | 151 | -2.175 | 8.820 | 72.049 | 1.00 | 91.71 | C |
| ATOM | 3555 | CD | GLN | B | 151 | -2.850 | 10.165 | 71.934 | 1.00 | 91.71 | C |
| ATOM | 3556 | OE1 | GLN | B | 151 | -2.671 | 11.043 | 72.781 | 1.00 | 91.71 | O |
| ATOM | 3557 | NE2 | GLN | B | 151 | -3.623 | 10.344 | 70.870 | 1.00 | 91.71 | N |
| ATOM | 3558 | C | GLN | B | 151 | -0.579 | 6.807 | 70.536 | 1.00 | 77.00 | C |
| ATOM | 3559 | O | GLN | B | 151 | 0.383 | 6.704 | 71.297 | 1.00 | 77.00 | O |
| ATOM | 3560 | N | THR | B | 152 | -0.465 | 7.191 | 69.268 | 1.00 | 46.21 | N |
| ATOM | 3561 | CA | THR | B | 152 | 0.856 | 7.445 | 68.706 | 1.00 | 46.21 | C |
| ATOM | 3562 | CB | THR | B | 152 | 1.114 | 6.596 | 67.430 | 1.00 | 59.19 | C |
| ATOM | 3563 | OG1 | THR | B | 152 | 2.525 | 6.539 | 67.171 | 1.00 | 59.19 | O |
| ATOM | 3564 | CG2 | THR | B | 152 | 0.403 | 7.201 | 66.227 | 1.00 | 59.19 | C |
| ATOM | 3565 | C | THR | B | 152 | 1.135 | 8.897 | 68.378 | 1.00 | 46.21 | C |
| ATOM | 3566 | O | THR | B | 152 | 0.226 | 9.642 | 68.005 | 1.00 | 46.21 | O |
| ATOM | 3567 | N | LEU | B | 153 | 2.403 | 9.287 | 68.510 | 1.00 | 58.02 | N |
| ATOM | 3568 | CA | LEU | B | 153 | 2.825 | 10.664 | 68.222 | 1.00 | 58.02 | C |
| ATOM | 3569 | CB | LEU | B | 153 | 4.285 | 10.880 | 68.614 | 1.00 | 26.27 | C |
| ATOM | 3570 | CG | LEU | B | 153 | 4.883 | 12.198 | 68.137 | 1.00 | 26.27 | C |
| ATOM | 3571 | CD1 | LEU | B | 153 | 4.617 | 13.281 | 69.169 | 1.00 | 26.27 | C |
| ATOM | 3572 | CD2 | LEU | B | 153 | 6.367 | 12.008 | 67.895 | 1.00 | 26.27 | C |
| ATOM | 3573 | C | LEU | B | 153 | 2.671 | 11.031 | 66.748 | 1.00 | 58.02 | C |
| ATOM | 3574 | O | LEU | B | 153 | 3.067 | 10.271 | 65.873 | 1.00 | 58.02 | O |
| ATOM | 3575 | N | PRO | B | 154 | 2.097 | 12.205 | 66.458 | 1.00 | 58.93 | N |
| ATOM | 3576 | CD | PRO | B | 154 | 1.518 | 13.121 | 67.455 | 1.00 | 39.19 | C |
| ATOM | 3577 | CA | PRO | B | 154 | 1.882 | 12.704 | 65.094 | 1.00 | 58.93 | C |
| ATOM | 3578 | CB | PRO | B | 154 | 1.347 | 14.109 | 65.314 | 1.00 | 39.19 | C |
| ATOM | 3579 | CG | PRO | B | 154 | 0.640 | 14.000 | 66.608 | 1.00 | 39.19 | C |
| ATOM | 3580 | C | PRO | B | 154 | 3.190 | 12.755 | 64.314 | 1.00 | 58.93 | C |
| ATOM | 3581 | O | PRO | B | 154 | 4.206 | 13.256 | 64.808 | 1.00 | 58.93 | O |
| ATOM | 3582 | N | VAL | B | 155 | 3.151 | 12.268 | 63.084 | 1.00 | 38.38 | N |
| ATOM | 3583 | CA | VAL | B | 155 | 4.321 | 12.266 | 62.221 | 1.00 | 38.38 | C |
| ATOM | 3584 | CB | VAL | B | 155 | 3.948 | 11.762 | 60.811 | 1.00 | 30.29 | C |
| ATOM | 3585 | CG1 | VAL | B | 155 | 3.291 | 10.394 | 60.916 | 1.00 | 30.29 | C |
| ATOM | 3586 | CG2 | VAL | B | 155 | 3.003 | 12.764 | 60.124 | 1.00 | 30.29 | C |

| | | | | | | | | | | | |
|---|---|---|---|---|---|---|---|---|---|---|---|
| ATOM | 3587 | C | VAL | B | 155 | 4.987 | 13.638 | 62.075 | 1.00 | 38.38 | C |
| ATOM | 3588 | O | VAL | B | 155 | 6.187 | 13.714 | 61.815 | 1.00 | 38.38 | O |
| ATOM | 3589 | N | ILE | B | 156 | 4.217 | 14.714 | 62.229 | 1.00 | 27.91 | N |
| ATOM | 3590 | CA | ILE | B | 156 | 4.780 | 16.055 | 62.085 | 1.00 | 27.91 | C |
| ATOM | 3591 | CB | ILE | B | 156 | 3.704 | 17.146 | 62.318 | 1.00 | 36.60 | C |
| ATOM | 3592 | CG2 | ILE | B | 156 | 3.196 | 17.086 | 63.736 | 1.00 | 36.60 | C |
| ATOM | 3593 | CG1 | ILE | B | 156 | 4.288 | 18.528 | 62.035 | 1.00 | 36.60 | C |
| ATOM | 3594 | CD1 | ILE | B | 156 | 4.758 | 18.698 | 60.606 | 1.00 | 36.60 | C |
| ATOM | 3595 | C | ILE | B | 156 | 5.902 | 16.204 | 63.109 | 1.00 | 27.91 | C |
| ATOM | 3596 | O | ILE | B | 156 | 6.959 | 16.771 | 62.824 | 1.00 | 27.91 | O |
| ATOM | 3597 | N | TYR | B | 157 | 5.662 | 15.667 | 64.300 | 1.00 | 33.67 | N |
| ATOM | 3598 | CA | TYR | B | 157 | 6.636 | 15.714 | 65.382 | 1.00 | 33.67 | C |
| ATOM | 3599 | CB | TYR | B | 157 | 5.942 | 15.400 | 66.703 | 1.00 | 36.45 | C |
| ATOM | 3600 | CG | TYR | B | 157 | 4.958 | 16.466 | 67.108 | 1.00 | 36.45 | C |
| ATOM | 3601 | CD1 | TYR | B | 157 | 3.626 | 16.146 | 67.397 | 1.00 | 36.45 | C |
| ATOM | 3602 | CE1 | TYR | B | 157 | 2.720 | 17.136 | 67.774 | 1.00 | 36.45 | C |
| ATOM | 3603 | CD2 | TYR | B | 157 | 5.355 | 17.798 | 67.204 | 1.00 | 36.45 | C |
| ATOM | 3604 | CE2 | TYR | B | 157 | 4.463 | 18.788 | 67.579 | 1.00 | 36.45 | C |
| ATOM | 3605 | CZ | TYR | B | 157 | 3.148 | 18.458 | 67.862 | 1.00 | 36.45 | C |
| ATOM | 3606 | OH | TYR | B | 157 | 2.271 | 19.446 | 68.244 | 1.00 | 36.45 | O |
| ATOM | 3607 | C | TYR | B | 157 | 7.765 | 14.713 | 65.135 | 1.00 | 33.67 | C |
| ATOM | 3608 | O | TYR | B | 157 | 8.944 | 14.996 | 65.382 | 1.00 | 33.67 | O |
| ATOM | 3609 | N | VAL | B | 158 | 7.394 | 13.533 | 64.653 | 1.00 | 28.34 | N |
| ATOM | 3610 | CA | VAL | B | 158 | 8.388 | 12.524 | 64.364 | 1.00 | 28.34 | C |
| ATOM | 3611 | CB | VAL | B | 158 | 7.749 | 11.248 | 63.783 | 1.00 | 25.63 | C |
| ATOM | 3612 | CG1 | VAL | B | 158 | 8.785 | 10.141 | 63.706 | 1.00 | 25.63 | C |
| ATOM | 3613 | CG2 | VAL | B | 158 | 6.586 | 10.809 | 64.653 | 1.00 | 25.63 | C |
| ATOM | 3614 | C | VAL | B | 158 | 9.364 | 13.112 | 63.355 | 1.00 | 28.34 | C |
| ATOM | 3615 | O | VAL | B | 158 | 10.561 | 12.873 | 63.446 | 1.00 | 28.34 | O |
| ATOM | 3616 | N | LYS | B | 159 | 8.848 | 13.884 | 62.399 | 1.00 | 42.04 | N |
| ATOM | 3617 | CA | LYS | B | 159 | 9.693 | 14.518 | 61.384 | 1.00 | 42.04 | C |
| ATOM | 3618 | CB | LYS | B | 159 | 8.843 | 15.100 | 60.243 | 1.00 | 49.36 | C |
| ATOM | 3619 | CG | LYS | B | 159 | 8.055 | 14.076 | 59.431 | 1.00 | 49.36 | C |
| ATOM | 3620 | CD | LYS | B | 159 | 7.154 | 14.748 | 58.391 | 1.00 | 49.36 | C |
| ATOM | 3621 | CE | LYS | B | 159 | 6.336 | 13.736 | 57.591 | 1.00 | 49.36 | C |
| ATOM | 3622 | NZ | LYS | B | 159 | 5.598 | 14.397 | 56.480 | 1.00 | 49.36 | N |
| ATOM | 3623 | C | LYS | B | 159 | 10.508 | 15.636 | 62.045 | 1.00 | 42.04 | C |
| ATOM | 3624 | O | LYS | B | 159 | 11.729 | 15.660 | 61.949 | 1.00 | 42.04 | O |
| ATOM | 3625 | N | LEU | B | 160 | 9.833 | 16.557 | 62.723 | 1.00 | 25.63 | N |
| ATOM | 3626 | CA | LEU | B | 160 | 10.520 | 17.658 | 63.402 | 1.00 | 25.63 | C |
| ATOM | 3627 | CB | LEU | B | 160 | 9.515 | 18.502 | 64.205 | 1.00 | 31.02 | C |
| ATOM | 3628 | CG | LEU | B | 160 | 8.700 | 19.556 | 63.456 | 1.00 | 31.02 | C |
| ATOM | 3629 | CD1 | LEU | B | 160 | 7.647 | 20.157 | 64.388 | 1.00 | 31.02 | C |
| ATOM | 3630 | CD2 | LEU | B | 160 | 9.636 | 20.626 | 62.916 | 1.00 | 31.02 | C |
| ATOM | 3631 | C | LEU | B | 160 | 11.639 | 17.202 | 64.347 | 1.00 | 25.63 | C |
| ATOM | 3632 | O | LEU | B | 160 | 12.725 | 17.791 | 64.366 | 1.00 | 25.63 | O |
| ATOM | 3633 | N | TYR | B | 161 | 11.372 | 16.159 | 65.135 | 1.00 | 19.56 | N |
| ATOM | 3634 | CA | TYR | B | 161 | 12.361 | 15.678 | 66.093 | 1.00 | 19.56 | C |
| ATOM | 3635 | CB | TYR | B | 161 | 11.725 | 14.768 | 67.141 | 1.00 | 28.88 | C |
| ATOM | 3636 | CG | TYR | B | 161 | 10.604 | 15.395 | 67.936 | 1.00 | 28.88 | C |
| ATOM | 3637 | CD1 | TYR | B | 161 | 10.533 | 16.778 | 68.130 | 1.00 | 28.88 | C |
| ATOM | 3638 | CE1 | TYR | B | 161 | 9.478 | 17.348 | 68.864 | 1.00 | 28.88 | C |
| ATOM | 3639 | CD2 | TYR | B | 161 | 9.605 | 14.596 | 68.499 | 1.00 | 28.88 | C |
| ATOM | 3640 | CE2 | TYR | B | 161 | 8.552 | 15.148 | 69.239 | 1.00 | 28.88 | C |
| ATOM | 3641 | CZ | TYR | B | 161 | 8.485 | 16.517 | 69.417 | 1.00 | 28.88 | C |
| ATOM | 3642 | OH | TYR | B | 161 | 7.412 | 17.037 | 70.119 | 1.00 | 28.88 | O |
| ATOM | 3643 | C | TYR | B | 161 | 13.508 | 14.945 | 65.437 | 1.00 | 19.56 | C |
| ATOM | 3644 | O | TYR | B | 161 | 14.677 | 15.141 | 65.792 | 1.00 | 19.56 | O |
| ATOM | 3645 | N | MET | B | 162 | 13.178 | 14.101 | 64.470 | 1.00 | 30.21 | N |
| ATOM | 3646 | CA | MET | B | 162 | 14.212 | 13.350 | 63.796 | 1.00 | 30.21 | C |
| ATOM | 3647 | CB | MET | B | 162 | 13.607 | 12.227 | 62.955 | 1.00 | 29.18 | C |
| ATOM | 3648 | CG | MET | B | 162 | 13.087 | 11.048 | 63.794 | 1.00 | 29.18 | C |
| ATOM | 3649 | SD | MET | B | 162 | 14.303 | 10.412 | 64.990 | 1.00 | 29.18 | S |

| | | | | | | | | | | | |
|---|---|---|---|---|---|---|---|---|---|---|---|
| ATOM | 3650 | CE | MET | B | 162 | 15.625 | 10.012 | 63.910 | 1.00 | 29.18 | C |
| ATOM | 3651 | C | MET | B | 162 | 15.078 | 14.246 | 62.947 | 1.00 | 30.21 | C |
| ATOM | 3652 | O | MET | B | 162 | 16.287 | 14.026 | 62.879 | 1.00 | 30.21 | O |
| ATOM | 3653 | N | TYR | B | 163 | 14.468 | 15.264 | 62.330 | 1.00 | 26.87 | N |
| ATOM | 3654 | CA | TYR | B | 163 | 15.192 | 16.213 | 61.474 | 1.00 | 26.87 | C |
| ATOM | 3655 | CB | TYR | B | 163 | 14.240 | 17.237 | 60.848 | 1.00 | 28.70 | C |
| ATOM | 3656 | CG | TYR | B | 163 | 14.932 | 18.200 | 59.909 | 1.00 | 28.70 | C |
| ATOM | 3657 | CD1 | TYR | B | 163 | 15.417 | 17.769 | 58.666 | 1.00 | 28.70 | C |
| ATOM | 3658 | CE1 | TYR | B | 163 | 16.063 | 18.646 | 57.792 | 1.00 | 28.70 | C |
| ATOM | 3659 | CD2 | TYR | B | 163 | 15.115 | 19.537 | 60.258 | 1.00 | 28.70 | C |
| ATOM | 3660 | CE2 | TYR | B | 163 | 15.758 | 20.428 | 59.394 | 1.00 | 28.70 | C |
| ATOM | 3661 | CZ | TYR | B | 163 | 16.229 | 19.982 | 58.162 | 1.00 | 28.70 | C |
| ATOM | 3662 | OH | TYR | B | 163 | 16.836 | 20.875 | 57.296 | 1.00 | 28.70 | O |
| ATOM | 3663 | C | TYR | B | 163 | 16.243 | 16.953 | 62.277 | 1.00 | 26.87 | C |
| ATOM | 3664 | O | TYR | B | 163 | 17.400 | 17.038 | 61.869 | 1.00 | 26.87 | O |
| ATOM | 3665 | N | GLN | B | 164 | 15.834 | 17.477 | 63.427 | 1.00 | 24.33 | N |
| ATOM | 3666 | CA | GLN | B | 164 | 16.743 | 18.219 | 64.293 | 1.00 | 24.33 | C |
| ATOM | 3667 | CB | GLN | B | 164 | 15.944 | 18.927 | 65.386 | 1.00 | 22.96 | C |
| ATOM | 3668 | CG | GLN | B | 164 | 14.852 | 19.802 | 64.829 | 1.00 | 22.96 | C |
| ATOM | 3669 | CD | GLN | B | 164 | 14.111 | 20.558 | 65.897 | 1.00 | 22.96 | C |
| ATOM | 3670 | OE1 | GLN | B | 164 | 14.617 | 21.533 | 66.450 | 1.00 | 22.96 | O |
| ATOM | 3671 | NE2 | GLN | B | 164 | 12.905 | 20.108 | 66.206 | 1.00 | 22.96 | N |
| ATOM | 3672 | C | GLN | B | 164 | 17.831 | 17.323 | 64.897 | 1.00 | 24.33 | C |
| ATOM | 3673 | O | GLN | B | 164 | 18.977 | 17.740 | 65.080 | 1.00 | 24.33 | O |
| ATOM | 3674 | N | LEU | B | 165 | 17.478 | 16.084 | 65.199 | 1.00 | 28.76 | N |
| ATOM | 3675 | CA | LEU | B | 165 | 18.451 | 15.157 | 65.736 | 1.00 | 28.76 | C |
| ATOM | 3676 | CB | LEU | B | 165 | 17.791 | 13.813 | 66.040 | 1.00 | 16.84 | C |
| ATOM | 3677 | CG | LEU | B | 165 | 18.736 | 12.627 | 66.293 | 1.00 | 16.84 | C |
| ATOM | 3678 | CD1 | LEU | B | 165 | 19.578 | 12.901 | 67.518 | 1.00 | 16.84 | C |
| ATOM | 3679 | CD2 | LEU | B | 165 | 17.941 | 11.322 | 66.466 | 1.00 | 16.84 | C |
| ATOM | 3680 | C | LEU | B | 165 | 19.556 | 14.952 | 64.708 | 1.00 | 28.76 | C |
| ATOM | 3681 | O | LEU | B | 165 | 20.736 | 15.013 | 65.043 | 1.00 | 28.76 | O |
| ATOM | 3682 | N | PHE | B | 166 | 19.170 | 14.707 | 63.455 | 1.00 | 17.54 | N |
| ATOM | 3683 | CA | PHE | B | 166 | 20.146 | 14.471 | 62.394 | 1.00 | 17.54 | C |
| ATOM | 3684 | CB | PHE | B | 166 | 19.452 | 14.052 | 61.095 | 1.00 | 25.20 | C |
| ATOM | 3685 | CG | PHE | B | 166 | 19.103 | 12.591 | 61.048 | 1.00 | 25.20 | C |
| ATOM | 3686 | CD1 | PHE | B | 166 | 20.087 | 11.628 | 61.245 | 1.00 | 25.20 | C |
| ATOM | 3687 | CD2 | PHE | B | 166 | 17.782 | 12.179 | 60.865 | 1.00 | 25.20 | C |
| ATOM | 3688 | CE1 | PHE | B | 166 | 19.769 | 10.276 | 61.270 | 1.00 | 25.20 | C |
| ATOM | 3689 | CE2 | PHE | B | 166 | 17.447 | 10.818 | 60.890 | 1.00 | 25.20 | C |
| ATOM | 3690 | CZ | PHE | B | 166 | 18.452 | 9.863 | 61.096 | 1.00 | 25.20 | C |
| ATOM | 3691 | C | PHE | B | 166 | 21.041 | 15.665 | 62.130 | 1.00 | 17.54 | C |
| ATOM | 3692 | O | PHE | B | 166 | 22.212 | 15.527 | 61.729 | 1.00 | 17.54 | O |
| ATOM | 3693 | N | ARG | B | 167 | 20.498 | 16.844 | 62.375 | 1.00 | 22.45 | N |
| ATOM | 3694 | CA | ARG | B | 167 | 21.273 | 18.035 | 62.143 | 1.00 | 22.45 | C |
| ATOM | 3695 | CB | ARG | B | 167 | 20.363 | 19.254 | 62.123 | 1.00 | 35.91 | C |
| ATOM | 3696 | CG | ARG | B | 167 | 21.053 | 20.446 | 61.520 | 1.00 | 35.91 | C |
| ATOM | 3697 | CD | ARG | B | 167 | 20.132 | 21.619 | 61.437 | 1.00 | 35.91 | C |
| ATOM | 3698 | NE | ARG | B | 167 | 20.882 | 22.846 | 61.239 | 1.00 | 35.91 | N |
| ATOM | 3699 | CZ | ARG | B | 167 | 20.351 | 24.051 | 61.360 | 1.00 | 35.91 | C |
| ATOM | 3700 | NH1 | ARG | B | 167 | 19.069 | 24.172 | 61.682 | 1.00 | 35.91 | N |
| ATOM | 3701 | NH2 | ARG | B | 167 | 21.096 | 25.126 | 61.157 | 1.00 | 35.91 | N |
| ATOM | 3702 | C | ARG | B | 167 | 22.371 | 18.181 | 63.196 | 1.00 | 22.45 | C |
| ATOM | 3703 | O | ARG | B | 167 | 23.529 | 18.483 | 62.876 | 1.00 | 22.45 | O |
| ATOM | 3704 | N | SER | B | 168 | 22.023 | 17.953 | 64.456 | 1.00 | 23.29 | N |
| ATOM | 3705 | CA | SER | B | 168 | 23.022 | 18.068 | 65.507 | 1.00 | 23.29 | C |
| ATOM | 3706 | CB | SER | B | 168 | 22.358 | 17.908 | 66.876 | 1.00 | 24.10 | C |
| ATOM | 3707 | OG | SER | B | 168 | 21.923 | 16.577 | 67.089 | 1.00 | 24.10 | O |
| ATOM | 3708 | C | SER | B | 168 | 24.084 | 16.983 | 65.299 | 1.00 | 23.29 | C |
| ATOM | 3709 | O | SER | B | 168 | 25.266 | 17.152 | 65.638 | 1.00 | 23.29 | O |
| ATOM | 3710 | N | LEU | B | 169 | 23.645 | 15.865 | 64.730 | 1.00 | 21.95 | N |
| ATOM | 3711 | CA | LEU | B | 169 | 24.527 | 14.740 | 64.466 | 1.00 | 21.95 | C |
| ATOM | 3712 | CB | LEU | B | 169 | 23.691 | 13.519 | 64.083 | 1.00 | 22.38 | C |

| | | | | | | | | | | | |
|---|---|---|---|---|---|---|---|---|---|---|---|
| ATOM | 3713 | CG | LEU | B | 169 | 23.839 | 12.290 | 64.969 | 1.00 | 22.38 | C |
| ATOM | 3714 | CD1 | LEU | B | 169 | 23.999 | 12.727 | 66.394 | 1.00 | 22.38 | C |
| ATOM | 3715 | CD2 | LEU | B | 169 | 22.636 | 11.398 | 64.789 | 1.00 | 22.38 | C |
| ATOM | 3716 | C | LEU | B | 169 | 25.510 | 15.114 | 63.357 | 1.00 | 21.95 | C |
| ATOM | 3717 | O | LEU | B | 169 | 26.701 | 14.853 | 63.451 | 1.00 | 21.95 | O |
| ATOM | 3718 | N | ALA | B | 170 | 25.011 | 15.768 | 62.320 | 1.00 | 36.28 | N |
| ATOM | 3719 | CA | ALA | B | 170 | 25.867 | 16.196 | 61.221 | 1.00 | 36.28 | C |
| ATOM | 3720 | CB | ALA | B | 170 | 25.009 | 16.791 | 60.119 | 1.00 | 30.60 | C |
| ATOM | 3721 | C | ALA | B | 170 | 26.902 | 17.225 | 61.693 | 1.00 | 36.28 | C |
| ATOM | 3722 | O | ALA | B | 170 | 28.028 | 17.260 | 61.198 | 1.00 | 36.28 | O |
| ATOM | 3723 | N | TYR | B | 171 | 26.503 | 18.066 | 62.645 | 1.00 | 28.14 | N |
| ATOM | 3724 | CA | TYR | B | 171 | 27.372 | 19.098 | 63.198 | 1.00 | 28.14 | C |
| ATOM | 3725 | CB | TYR | B | 171 | 26.595 | 19.981 | 64.172 | 1.00 | 31.16 | C |
| ATOM | 3726 | CG | TYR | B | 171 | 27.452 | 21.014 | 64.884 | 1.00 | 31.16 | C |
| ATOM | 3727 | CD1 | TYR | B | 171 | 27.808 | 22.209 | 64.263 | 1.00 | 31.16 | C |
| ATOM | 3728 | CE1 | TYR | B | 171 | 28.584 | 23.164 | 64.925 | 1.00 | 31.16 | C |
| ATOM | 3729 | CD2 | TYR | B | 171 | 27.901 | 20.800 | 66.188 | 1.00 | 31.16 | C |
| ATOM | 3730 | CE2 | TYR | B | 171 | 28.679 | 21.753 | 66.854 | 1.00 | 31.16 | C |
| ATOM | 3731 | CZ | TYR | B | 171 | 29.013 | 22.925 | 66.216 | 1.00 | 31.16 | C |
| ATOM | 3732 | OH | TYR | B | 171 | 29.790 | 23.847 | 66.867 | 1.00 | 31.16 | O |
| ATOM | 3733 | C | TYR | B | 171 | 28.565 | 18.493 | 63.919 | 1.00 | 28.14 | C |
| ATOM | 3734 | O | TYR | B | 171 | 29.708 | 18.699 | 63.510 | 1.00 | 28.14 | O |
| ATOM | 3735 | N | ILE | B | 172 | 28.305 | 17.740 | 64.987 | 1.00 | 28.53 | N |
| ATOM | 3736 | CA | ILE | B | 172 | 29.392 | 17.138 | 65.752 | 1.00 | 28.53 | C |
| ATOM | 3737 | CB | ILE | B | 172 | 28.887 | 16.364 | 66.994 | 1.00 | 21.79 | C |
| ATOM | 3738 | CG2 | ILE | B | 172 | 28.107 | 17.300 | 67.915 | 1.00 | 21.79 | C |
| ATOM | 3739 | CG1 | ILE | B | 172 | 28.023 | 15.181 | 66.565 | 1.00 | 21.79 | C |
| ATOM | 3740 | CD1 | ILE | B | 172 | 27.491 | 14.352 | 67.740 | 1.00 | 21.79 | C |
| ATOM | 3741 | C | ILE | B | 172 | 30.204 | 16.185 | 64.907 | 1.00 | 28.53 | C |
| ATOM | 3742 | O | ILE | B | 172 | 31.418 | 16.055 | 65.092 | 1.00 | 28.53 | O |
| ATOM | 3743 | N | HIS | B | 173 | 29.540 | 15.522 | 63.968 | 1.00 | 27.16 | N |
| ATOM | 3744 | CA | HIS | B | 173 | 30.251 | 14.578 | 63.138 | 1.00 | 27.16 | C |
| ATOM | 3745 | CB | HIS | B | 173 | 29.280 | 13.742 | 62.301 | 1.00 | 23.08 | C |
| ATOM | 3746 | CG | HIS | B | 173 | 28.602 | 12.657 | 63.079 | 1.00 | 23.08 | C |
| ATOM | 3747 | CD2 | HIS | B | 173 | 28.577 | 12.401 | 64.409 | 1.00 | 23.08 | C |
| ATOM | 3748 | ND1 | HIS | B | 173 | 27.787 | 11.713 | 62.490 | 1.00 | 23.08 | N |
| ATOM | 3749 | CE1 | HIS | B | 173 | 27.284 | 10.926 | 63.424 | 1.00 | 23.08 | C |
| ATOM | 3750 | NE2 | HIS | B | 173 | 27.746 | 11.320 | 64.597 | 1.00 | 23.08 | N |
| ATOM | 3751 | C | HIS | B | 173 | 31.275 | 15.265 | 62.258 | 1.00 | 27.16 | C |
| ATOM | 3752 | O | HIS | B | 173 | 32.321 | 14.689 | 61.951 | 1.00 | 27.16 | O |
| ATOM | 3753 | N | SER | B | 174 | 30.992 | 16.499 | 61.863 | 1.00 | 31.32 | N |
| ATOM | 3754 | CA | SER | B | 174 | 31.929 | 17.241 | 61.024 | 1.00 | 31.32 | C |
| ATOM | 3755 | CB | SER | B | 174 | 31.275 | 18.527 | 60.533 | 1.00 | 26.18 | C |
| ATOM | 3756 | OG | SER | B | 174 | 31.066 | 19.429 | 61.611 | 1.00 | 26.18 | O |
| ATOM | 3757 | C | SER | B | 174 | 33.206 | 17.568 | 61.822 | 1.00 | 31.32 | C |
| ATOM | 3758 | O | SER | B | 174 | 34.176 | 18.094 | 61.280 | 1.00 | 31.32 | O |
| ATOM | 3759 | N | PHE | B | 175 | 33.182 | 17.245 | 63.113 | 1.00 | 38.44 | N |
| ATOM | 3760 | CA | PHE | B | 175 | 34.308 | 17.479 | 64.008 | 1.00 | 38.44 | C |
| ATOM | 3761 | CB | PHE | B | 175 | 33.860 | 18.229 | 65.249 | 1.00 | 46.02 | C |
| ATOM | 3762 | CG | PHE | B | 175 | 33.414 | 19.616 | 64.977 | 1.00 | 46.02 | C |
| ATOM | 3763 | CD1 | PHE | B | 175 | 32.077 | 19.967 | 65.092 | 1.00 | 46.02 | C |
| ATOM | 3764 | CD2 | PHE | B | 175 | 34.336 | 20.585 | 64.610 | 1.00 | 46.02 | C |
| ATOM | 3765 | CE1 | PHE | B | 175 | 31.667 | 21.273 | 64.846 | 1.00 | 46.02 | C |
| ATOM | 3766 | CE2 | PHE | B | 175 | 33.938 | 21.887 | 64.364 | 1.00 | 46.02 | C |
| ATOM | 3767 | CZ | PHE | B | 175 | 32.604 | 22.236 | 64.481 | 1.00 | 46.02 | C |
| ATOM | 3768 | C | PHE | B | 175 | 34.908 | 16.166 | 64.450 | 1.00 | 38.44 | C |
| ATOM | 3769 | O | PHE | B | 175 | 35.843 | 16.149 | 65.251 | 1.00 | 38.44 | O |
| ATOM | 3770 | N | GLY | B | 176 | 34.351 | 15.072 | 63.932 | 1.00 | 23.23 | N |
| ATOM | 3771 | CA | GLY | B | 176 | 34.823 | 13.744 | 64.283 | 1.00 | 23.23 | C |
| ATOM | 3772 | C | GLY | B | 176 | 34.345 | 13.334 | 65.660 | 1.00 | 23.23 | C |
| ATOM | 3773 | O | GLY | B | 176 | 34.782 | 12.328 | 66.211 | 1.00 | 23.23 | O |
| ATOM | 3774 | N | ILE | B | 177 | 33.441 | 14.115 | 66.231 | 1.00 | 27.95 | N |
| ATOM | 3775 | CA | ILE | B | 177 | 32.941 | 13.790 | 67.553 | 1.00 | 27.95 | C |

| | | | | | | | | | | | |
|---|---|---|---|---|---|---|---|---|---|---|---|
| ATOM | 3776 | CB | ILE | B | 177 | 32.600 | 15.068 | 68.344 | 1.00 | 22.65 | C |
| ATOM | 3777 | CG2 | ILE | B | 177 | 32.023 | 14.692 | 69.715 | 1.00 | 22.65 | C |
| ATOM | 3778 | CG1 | ILE | B | 177 | 33.864 | 15.924 | 68.510 | 1.00 | 22.65 | C |
| ATOM | 3779 | CD1 | ILE | B | 177 | 33.610 | 17.305 | 69.150 | 1.00 | 22.65 | C |
| ATOM | 3780 | C | ILE | B | 177 | 31.710 | 12.884 | 67.454 | 1.00 | 27.95 | C |
| ATOM | 3781 | O | ILE | B | 177 | 30.700 | 13.244 | 66.845 | 1.00 | 27.95 | O |
| ATOM | 3782 | N | CYS | B | 178 | 31.810 | 11.698 | 68.049 | 1.00 | 21.35 | N |
| ATOM | 3783 | CA | CYS | B | 178 | 30.732 | 10.725 | 68.025 | 1.00 | 21.35 | C |
| ATOM | 3784 | CB | CYS | B | 178 | 31.290 | 9.330 | 67.738 | 1.00 | 32.94 | C |
| ATOM | 3785 | SG | CYS | B | 178 | 30.028 | 8.025 | 67.672 | 1.00 | 32.94 | S |
| ATOM | 3786 | C | CYS | B | 178 | 30.067 | 10.745 | 69.382 | 1.00 | 21.35 | C |
| ATOM | 3787 | O | CYS | B | 178 | 30.749 | 10.762 | 70.404 | 1.00 | 21.35 | O |
| ATOM | 3788 | N | HIS | B | 179 | 28.733 | 10.728 | 69.386 | 1.00 | 18.04 | N |
| ATOM | 3789 | CA | HIS | B | 179 | 27.954 | 10.765 | 70.621 | 1.00 | 18.04 | C |
| ATOM | 3790 | CB | HIS | B | 179 | 26.497 | 11.123 | 70.313 | 1.00 | 29.44 | C |
| ATOM | 3791 | CG | HIS | B | 179 | 25.696 | 11.488 | 71.528 | 1.00 | 29.44 | C |
| ATOM | 3792 | CD2 | HIS | B | 179 | 25.431 | 12.695 | 72.088 | 1.00 | 29.44 | C |
| ATOM | 3793 | ND1 | HIS | B | 179 | 25.092 | 10.549 | 72.338 | 1.00 | 29.44 | N |
| ATOM | 3794 | CE1 | HIS | B | 179 | 24.491 | 11.159 | 73.344 | 1.00 | 29.44 | C |
| ATOM | 3795 | NE2 | HIS | B | 179 | 24.683 | 12.461 | 73.215 | 1.00 | 29.44 | N |
| ATOM | 3796 | C | HIS | B | 179 | 28.003 | 9.480 | 71.429 | 1.00 | 18.04 | C |
| ATOM | 3797 | O | HIS | B | 179 | 28.121 | 9.526 | 72.641 | 1.00 | 18.04 | O |
| ATOM | 3798 | N | ARG | B | 180 | 27.887 | 8.339 | 70.757 | 1.00 | 40.69 | N |
| ATOM | 3799 | CA | ARG | B | 180 | 27.929 | 7.026 | 71.407 | 1.00 | 40.69 | C |
| ATOM | 3800 | CB | ARG | B | 180 | 29.291 | 6.816 | 72.073 | 1.00 | 26.75 | C |
| ATOM | 3801 | CG | ARG | B | 180 | 30.439 | 6.894 | 71.092 | 1.00 | 26.75 | C |
| ATOM | 3802 | CD | ARG | B | 180 | 31.716 | 7.278 | 71.783 | 1.00 | 26.75 | C |
| ATOM | 3803 | NE | ARG | B | 180 | 32.427 | 6.151 | 72.364 | 1.00 | 26.75 | N |
| ATOM | 3804 | CZ | ARG | B | 180 | 33.193 | 6.232 | 73.444 | 1.00 | 26.75 | C |
| ATOM | 3805 | NH1 | ARG | B | 180 | 33.341 | 7.386 | 74.074 | 1.00 | 26.75 | N |
| ATOM | 3806 | NH2 | ARG | B | 180 | 33.828 | 5.159 | 73.885 | 1.00 | 26.75 | N |
| ATOM | 3807 | C | ARG | B | 180 | 26.819 | 6.733 | 72.415 | 1.00 | 40.69 | C |
| ATOM | 3808 | O | ARG | B | 180 | 26.889 | 5.752 | 73.140 | 1.00 | 40.69 | O |
| ATOM | 3809 | N | ASP | B | 181 | 25.794 | 7.571 | 72.471 | 1.00 | 34.91 | N |
| ATOM | 3810 | CA | ASP | B | 181 | 24.703 | 7.310 | 73.389 | 1.00 | 34.91 | C |
| ATOM | 3811 | CB | ASP | B | 181 | 25.118 | 7.661 | 74.816 | 1.00 | 31.20 | C |
| ATOM | 3812 | CG | ASP | B | 181 | 24.181 | 7.058 | 75.865 | 1.00 | 31.20 | C |
| ATOM | 3813 | OD1 | ASP | B | 181 | 23.894 | 5.839 | 75.829 | 1.00 | 31.20 | O |
| ATOM | 3814 | OD2 | ASP | B | 181 | 23.735 | 7.812 | 76.747 | 1.00 | 31.20 | O |
| ATOM | 3815 | C | ASP | B | 181 | 23.418 | 8.039 | 72.997 | 1.00 | 34.91 | C |
| ATOM | 3816 | O | ASP | B | 181 | 22.739 | 8.638 | 73.832 | 1.00 | 34.91 | O |
| ATOM | 3817 | N | ILE | B | 182 | 23.088 | 7.971 | 71.712 | 1.00 | 24.87 | N |
| ATOM | 3818 | CA | ILE | B | 182 | 21.883 | 8.594 | 71.198 | 1.00 | 24.87 | C |
| ATOM | 3819 | CB | ILE | B | 182 | 21.944 | 8.741 | 69.645 | 1.00 | 25.01 | C |
| ATOM | 3820 | CG2 | ILE | B | 182 | 20.601 | 9.201 | 69.117 | 1.00 | 25.01 | C |
| ATOM | 3821 | CG1 | ILE | B | 182 | 23.029 | 9.752 | 69.240 | 1.00 | 25.01 | C |
| ATOM | 3822 | CD1 | ILE | B | 182 | 22.804 | 11.188 | 69.764 | 1.00 | 25.01 | C |
| ATOM | 3823 | C | ILE | B | 182 | 20.672 | 7.744 | 71.598 | 1.00 | 24.87 | C |
| ATOM | 3824 | O | ILE | B | 182 | 20.566 | 6.562 | 71.249 | 1.00 | 24.87 | O |
| ATOM | 3825 | N | LYS | B | 183 | 19.765 | 8.363 | 72.345 | 1.00 | 33.76 | N |
| ATOM | 3826 | CA | LYS | B | 183 | 18.560 | 7.704 | 72.829 | 1.00 | 33.76 | C |
| ATOM | 3827 | CB | LYS | B | 183 | 18.868 | 6.903 | 74.102 | 1.00 | 37.09 | C |
| ATOM | 3828 | CG | LYS | B | 183 | 19.187 | 7.797 | 75.308 | 1.00 | 37.09 | C |
| ATOM | 3829 | CD | LYS | B | 183 | 20.190 | 7.189 | 76.300 | 1.00 | 37.09 | C |
| ATOM | 3830 | CE | LYS | B | 183 | 19.590 | 6.077 | 77.127 | 1.00 | 37.09 | C |
| ATOM | 3831 | NZ | LYS | B | 183 | 20.551 | 5.621 | 78.170 | 1.00 | 37.09 | N |
| ATOM | 3832 | C | LYS | B | 183 | 17.600 | 8.835 | 73.162 | 1.00 | 33.76 | C |
| ATOM | 3833 | O | LYS | B | 183 | 18.021 | 9.977 | 73.358 | 1.00 | 33.76 | O |
| ATOM | 3834 | N | PRO | B | 184 | 16.299 | 8.526 | 73.259 | 1.00 | 36.14 | N |
| ATOM | 3835 | CD | PRO | B | 184 | 15.746 | 7.165 | 73.140 | 1.00 | 18.13 | C |
| ATOM | 3836 | CA | PRO | B | 184 | 15.235 | 9.491 | 73.570 | 1.00 | 36.14 | C |
| ATOM | 3837 | CB | PRO | B | 184 | 14.035 | 8.593 | 73.808 | 1.00 | 18.13 | C |
| ATOM | 3838 | CG | PRO | B | 184 | 14.299 | 7.445 | 72.905 | 1.00 | 18.13 | C |

| | | | | | | | | | | | |
|---|---|---|---|---|---|---|---|---|---|---|---|
| ATOM | 3839 | C | PRO | B | 184 | 15.508 | 10.392 | 74.777 | 1.00 | 36.14 | C |
| ATOM | 3840 | O | PRO | B | 184 | 15.295 | 11.604 | 74.735 | 1.00 | 36.14 | O |
| ATOM | 3841 | N | GLN | B | 185 | 15.964 | 9.775 | 75.857 | 1.00 | 32.24 | N |
| ATOM | 3842 | CA | GLN | B | 185 | 16.248 | 10.480 | 77.092 | 1.00 | 32.24 | C |
| ATOM | 3843 | CB | GLN | B | 185 | 16.658 | 9.487 | 78.181 | 1.00 | 31.31 | C |
| ATOM | 3844 | CG | GLN | B | 185 | 15.570 | 8.509 | 78.566 | 1.00 | 31.31 | C |
| ATOM | 3845 | CD | GLN | B | 185 | 15.448 | 7.316 | 77.605 | 1.00 | 31.31 | C |
| ATOM | 3846 | OE1 | GLN | B | 185 | 15.791 | 7.412 | 76.417 | 1.00 | 31.31 | O |
| ATOM | 3847 | NE2 | GLN | B | 185 | 14.939 | 6.186 | 78.120 | 1.00 | 31.31 | N |
| ATOM | 3848 | C | GLN | B | 185 | 17.305 | 11.571 | 76.984 | 1.00 | 32.24 | C |
| ATOM | 3849 | O | GLN | B | 185 | 17.364 | 12.436 | 77.866 | 1.00 | 32.24 | O |
| ATOM | 3850 | N | ASN | B | 186 | 18.138 | 11.531 | 75.932 | 1.00 | 31.92 | N |
| ATOM | 3851 | CA | ASN | B | 186 | 19.202 | 12.542 | 75.731 | 1.00 | 31.92 | C |
| ATOM | 3852 | CB | ASN | B | 186 | 20.527 | 11.882 | 75.358 | 1.00 | 32.49 | C |
| ATOM | 3853 | CG | ASN | B | 186 | 21.083 | 11.066 | 76.479 | 1.00 | 32.49 | C |
| ATOM | 3854 | OD1 | ASN | B | 186 | 20.946 | 11.449 | 77.626 | 1.00 | 32.49 | O |
| ATOM | 3855 | ND2 | ASN | B | 186 | 21.717 | 9.944 | 76.163 | 1.00 | 32.49 | N |
| ATOM | 3856 | C | ASN | B | 186 | 18.866 | 13.596 | 74.683 | 1.00 | 31.92 | C |
| ATOM | 3857 | O | ASN | B | 186 | 19.731 | 14.344 | 74.215 | 1.00 | 31.92 | O |
| ATOM | 3858 | N | LEU | B | 187 | 17.591 | 13.640 | 74.332 | 1.00 | 32.37 | N |
| ATOM | 3859 | CA | LEU | B | 187 | 17.082 | 14.578 | 73.357 | 1.00 | 32.37 | C |
| ATOM | 3860 | CB | LEU | B | 187 | 16.390 | 13.820 | 72.227 | 1.00 | 17.23 | C |
| ATOM | 3861 | CG | LEU | B | 187 | 17.233 | 12.763 | 71.486 | 1.00 | 17.23 | C |
| ATOM | 3862 | CD1 | LEU | B | 187 | 16.389 | 12.125 | 70.386 | 1.00 | 17.23 | C |
| ATOM | 3863 | CD2 | LEU | B | 187 | 18.481 | 13.395 | 70.886 | 1.00 | 17.23 | C |
| ATOM | 3864 | C | LEU | B | 187 | 16.091 | 15.457 | 74.091 | 1.00 | 32.37 | C |
| ATOM | 3865 | O | LEU | B | 187 | 14.967 | 15.046 | 74.366 | 1.00 | 32.37 | O |
| ATOM | 3866 | N | LEU | B | 188 | 16.528 | 16.663 | 74.425 | 1.00 | 30.81 | N |
| ATOM | 3867 | CA | LEU | B | 188 | 15.688 | 17.606 | 75.141 | 1.00 | 30.81 | C |
| ATOM | 3868 | CB | LEU | B | 188 | 16.546 | 18.583 | 75.942 | 1.00 | 28.21 | C |
| ATOM | 3869 | CG | LEU | B | 188 | 17.683 | 18.015 | 76.794 | 1.00 | 28.21 | C |
| ATOM | 3870 | CD1 | LEU | B | 188 | 18.389 | 19.157 | 77.470 | 1.00 | 28.21 | C |
| ATOM | 3871 | CD2 | LEU | B | 188 | 17.162 | 17.041 | 77.833 | 1.00 | 28.21 | C |
| ATOM | 3872 | C | LEU | B | 188 | 14.885 | 18.385 | 74.137 | 1.00 | 30.81 | C |
| ATOM | 3873 | O | LEU | B | 188 | 15.319 | 18.558 | 73.000 | 1.00 | 30.81 | O |
| ATOM | 3874 | N | LEU | B | 189 | 13.711 | 18.852 | 74.551 | 1.00 | 34.53 | N |
| ATOM | 3875 | CA | LEU | B | 189 | 12.886 | 19.652 | 73.658 | 1.00 | 34.53 | C |
| ATOM | 3876 | CB | LEU | B | 189 | 12.145 | 18.752 | 72.668 | 1.00 | 40.73 | C |
| ATOM | 3877 | CG | LEU | B | 189 | 10.878 | 18.052 | 73.128 | 1.00 | 40.73 | C |
| ATOM | 3878 | CD1 | LEU | B | 189 | 9.675 | 18.668 | 72.417 | 1.00 | 40.73 | C |
| ATOM | 3879 | CD2 | LEU | B | 189 | 10.982 | 16.573 | 72.808 | 1.00 | 40.73 | C |
| ATOM | 3880 | C | LEU | B | 189 | 11.899 | 20.595 | 74.343 | 1.00 | 34.53 | C |
| ATOM | 3881 | O | LEU | B | 189 | 11.466 | 20.371 | 75.477 | 1.00 | 34.53 | O |
| ATOM | 3882 | N | ASP | B | 190 | 11.588 | 21.676 | 73.636 | 1.00 | 37.98 | N |
| ATOM | 3883 | CA | ASP | B | 190 | 10.640 | 22.675 | 74.092 | 1.00 | 37.98 | C |
| ATOM | 3884 | CB | ASP | B | 190 | 11.060 | 24.057 | 73.599 | 1.00 | 52.35 | C |
| ATOM | 3885 | CG | ASP | B | 190 | 10.221 | 25.155 | 74.185 | 1.00 | 52.35 | C |
| ATOM | 3886 | OD1 | ASP | B | 190 | 8.992 | 25.153 | 73.957 | 1.00 | 52.35 | O |
| ATOM | 3887 | OD2 | ASP | B | 190 | 10.791 | 26.020 | 74.880 | 1.00 | 52.35 | O |
| ATOM | 3888 | C | ASP | B | 190 | 9.310 | 22.252 | 73.454 | 1.00 | 37.98 | C |
| ATOM | 3889 | O | ASP | B | 190 | 9.146 | 22.281 | 72.223 | 1.00 | 37.98 | O |
| ATOM | 3890 | N | PRO | B | 191 | 8.342 | 21.853 | 74.289 | 1.00 | 58.59 | N |
| ATOM | 3891 | CD | PRO | B | 191 | 8.339 | 22.047 | 75.750 | 1.00 | 53.31 | C |
| ATOM | 3892 | CA | PRO | B | 191 | 7.025 | 21.406 | 73.832 | 1.00 | 58.59 | C |
| ATOM | 3893 | CB | PRO | B | 191 | 6.336 | 21.018 | 75.129 | 1.00 | 53.31 | C |
| ATOM | 3894 | CG | PRO | B | 191 | 6.861 | 22.055 | 76.068 | 1.00 | 53.31 | C |
| ATOM | 3895 | C | PRO | B | 191 | 6.208 | 22.411 | 73.035 | 1.00 | 58.59 | C |
| ATOM | 3896 | O | PRO | B | 191 | 5.291 | 22.017 | 72.322 | 1.00 | 58.59 | O |
| ATOM | 3897 | N | ASP | B | 192 | 6.536 | 23.696 | 73.148 | 1.00 | 31.52 | N |
| ATOM | 3898 | CA | ASP | B | 192 | 5.786 | 24.730 | 72.433 | 1.00 | 31.52 | C |
| ATOM | 3899 | CB | ASP | B | 192 | 5.608 | 25.962 | 73.306 | 1.00 | 52.70 | C |
| ATOM | 3900 | CG | ASP | B | 192 | 4.877 | 25.658 | 74.580 | 1.00 | 52.70 | C |
| ATOM | 3901 | OD1 | ASP | B | 192 | 3.756 | 25.104 | 74.511 | 1.00 | 52.70 | O |

| | | | | | | | | | | | |
|---|---|---|---|---|---|---|---|---|---|---|---|
| ATOM | 3902 | OD2 | ASP | B | 192 | 5.428 | 25.973 | 75.651 | 1.00 | 52.70 | O |
| ATOM | 3903 | C | ASP | B | 192 | 6.401 | 25.159 | 71.122 | 1.00 | 31.52 | C |
| ATOM | 3904 | O | ASP | B | 192 | 5.682 | 25.419 | 70.157 | 1.00 | 31.52 | O |
| ATOM | 3905 | N | THR | B | 193 | 7.728 | 25.258 | 71.090 | 1.00 | 32.76 | N |
| ATOM | 3906 | CA | THR | B | 193 | 8.420 | 25.657 | 69.870 | 1.00 | 32.76 | C |
| ATOM | 3907 | CB | THR | B | 193 | 9.691 | 26.463 | 70.187 | 1.00 | 42.96 | C |
| ATOM | 3908 | OG1 | THR | B | 193 | 10.507 | 25.751 | 71.127 | 1.00 | 42.96 | O |
| ATOM | 3909 | CG2 | THR | B | 193 | 9.316 | 27.796 | 70.773 | 1.00 | 42.96 | C |
| ATOM | 3910 | C | THR | B | 193 | 8.792 | 24.443 | 69.039 | 1.00 | 32.76 | C |
| ATOM | 3911 | O | THR | B | 193 | 9.079 | 24.555 | 67.846 | 1.00 | 32.76 | O |
| ATOM | 3912 | N | ALA | B | 194 | 8.758 | 23.282 | 69.688 | 1.00 | 32.55 | N |
| ATOM | 3913 | CA | ALA | B | 194 | 9.087 | 22.011 | 69.058 | 1.00 | 32.55 | C |
| ATOM | 3914 | CB | ALA | B | 194 | 8.245 | 21.794 | 67.803 | 1.00 | 24.57 | C |
| ATOM | 3915 | C | ALA | B | 194 | 10.559 | 21.962 | 68.706 | 1.00 | 32.55 | C |
| ATOM | 3916 | O | ALA | B | 194 | 10.960 | 21.252 | 67.793 | 1.00 | 32.55 | O |
| ATOM | 3917 | N | VAL | B | 195 | 11.363 | 22.721 | 69.441 | 1.00 | 37.11 | N |
| ATOM | 3918 | CA | VAL | B | 195 | 12.803 | 22.762 | 69.213 | 1.00 | 37.11 | C |
| ATOM | 3919 | CB | VAL | B | 195 | 13.384 | 24.147 | 69.602 | 1.00 | 17.51 | C |
| ATOM | 3920 | CG1 | VAL | B | 195 | 14.891 | 24.182 | 69.388 | 1.00 | 17.51 | C |
| ATOM | 3921 | CG2 | VAL | B | 195 | 12.715 | 25.223 | 68.771 | 1.00 | 17.51 | C |
| ATOM | 3922 | C | VAL | B | 195 | 13.499 | 21.671 | 70.019 | 1.00 | 37.11 | C |
| ATOM | 3923 | O | VAL | B | 195 | 13.238 | 21.497 | 71.212 | 1.00 | 37.11 | O |
| ATOM | 3924 | N | LEU | B | 196 | 14.385 | 20.939 | 69.354 | 1.00 | 30.07 | N |
| ATOM | 3925 | CA | LEU | B | 196 | 15.115 | 19.856 | 69.995 | 1.00 | 30.07 | C |
| ATOM | 3926 | CB | LEU | B | 196 | 15.055 | 18.599 | 69.116 | 1.00 | 24.26 | C |
| ATOM | 3927 | CG | LEU | B | 196 | 15.759 | 17.351 | 69.652 | 1.00 | 24.26 | C |
| ATOM | 3928 | CD1 | LEU | B | 196 | 15.056 | 16.104 | 69.116 | 1.00 | 24.26 | C |
| ATOM | 3929 | CD2 | LEU | B | 196 | 17.248 | 17.384 | 69.282 | 1.00 | 24.26 | C |
| ATOM | 3930 | C | LEU | B | 196 | 16.563 | 20.246 | 70.276 | 1.00 | 30.07 | C |
| ATOM | 3931 | O | LEU | B | 196 | 17.159 | 21.036 | 69.536 | 1.00 | 30.07 | O |
| ATOM | 3932 | N | LYS | B | 197 | 17.108 | 19.692 | 71.359 | 1.00 | 26.26 | N |
| ATOM | 3933 | CA | LYS | B | 197 | 18.478 | 19.961 | 71.767 | 1.00 | 26.26 | C |
| ATOM | 3934 | CB | LYS | B | 197 | 18.499 | 21.106 | 72.778 | 1.00 | 45.24 | C |
| ATOM | 3935 | CG | LYS | B | 197 | 18.366 | 22.468 | 72.127 | 1.00 | 45.24 | C |
| ATOM | 3936 | CD | LYS | B | 197 | 18.368 | 23.573 | 73.150 | 1.00 | 45.24 | C |
| ATOM | 3937 | CE | LYS | B | 197 | 18.598 | 24.921 | 72.489 | 1.00 | 45.24 | C |
| ATOM | 3938 | NZ | LYS | B | 197 | 20.023 | 25.059 | 72.052 | 1.00 | 45.24 | N |
| ATOM | 3939 | C | LYS | B | 197 | 19.210 | 18.749 | 72.338 | 1.00 | 26.26 | C |
| ATOM | 3940 | O | LYS | B | 197 | 18.839 | 18.226 | 73.397 | 1.00 | 26.26 | O |
| ATOM | 3941 | N | LEU | B | 198 | 20.261 | 18.323 | 71.638 | 1.00 | 29.86 | N |
| ATOM | 3942 | CA | LEU | B | 198 | 21.056 | 17.174 | 72.056 | 1.00 | 29.86 | C |
| ATOM | 3943 | CB | LEU | B | 198 | 22.107 | 16.840 | 71.008 | 1.00 | 18.44 | C |
| ATOM | 3944 | CG | LEU | B | 198 | 22.337 | 15.374 | 70.707 | 1.00 | 18.44 | C |
| ATOM | 3945 | CD1 | LEU | B | 198 | 23.693 | 15.195 | 70.064 | 1.00 | 18.44 | C |
| ATOM | 3946 | CD2 | LEU | B | 198 | 22.246 | 14.628 | 71.976 | 1.00 | 18.44 | C |
| ATOM | 3947 | C | LEU | B | 198 | 21.776 | 17.509 | 73.345 | 1.00 | 29.86 | C |
| ATOM | 3948 | O | LEU | B | 198 | 22.267 | 18.622 | 73.506 | 1.00 | 29.86 | O |
| ATOM | 3949 | N | CYS | B | 199 | 21.853 | 16.548 | 74.259 | 1.00 | 32.10 | N |
| ATOM | 3950 | CA | CYS | B | 199 | 22.534 | 16.791 | 75.520 | 1.00 | 32.10 | C |
| ATOM | 3951 | CB | CYS | B | 199 | 21.543 | 17.229 | 76.601 | 1.00 | 32.64 | C |
| ATOM | 3952 | SG | CYS | B | 199 | 20.582 | 15.881 | 77.312 | 1.00 | 32.64 | S |
| ATOM | 3953 | C | CYS | B | 199 | 23.280 | 15.556 | 75.990 | 1.00 | 32.10 | C |
| ATOM | 3954 | O | CYS | B | 199 | 23.239 | 14.508 | 75.343 | 1.00 | 32.10 | O |
| ATOM | 3955 | N | ASP | B | 200 | 23.979 | 15.699 | 77.111 | 1.00 | 29.54 | N |
| ATOM | 3956 | CA | ASP | B | 200 | 24.730 | 14.596 | 77.665 | 1.00 | 29.54 | C |
| ATOM | 3957 | CB | ASP | B | 200 | 23.795 | 13.440 | 77.942 | 1.00 | 50.97 | C |
| ATOM | 3958 | CG | ASP | B | 200 | 24.502 | 12.233 | 78.544 | 1.00 | 50.97 | C |
| ATOM | 3959 | OD1 | ASP | B | 200 | 25.354 | 12.394 | 79.447 | 1.00 | 50.97 | O |
| ATOM | 3960 | OD2 | ASP | B | 200 | 24.173 | 11.109 | 78.123 | 1.00 | 50.97 | O |
| ATOM | 3961 | C | ASP | B | 200 | 25.817 | 14.103 | 76.744 | 1.00 | 29.54 | C |
| ATOM | 3962 | O | ASP | B | 200 | 25.536 | 13.319 | 75.852 | 1.00 | 29.54 | O |
| ATOM | 3963 | N | PHE | B | 201 | 27.054 | 14.530 | 76.970 | 1.00 | 33.51 | N |
| ATOM | 3964 | CA | PHE | B | 201 | 28.151 | 14.087 | 76.130 | 1.00 | 33.51 | C |

| | | | | | | | | | | | |
|---|---|---|---|---|---|---|---|---|---|---|---|
| ATOM | 3965 | CB | PHE | B | 201 | 28.768 | 15.275 | 75.377 | 1.00 | 19.60 | C |
| ATOM | 3966 | CG | PHE | B | 201 | 27.810 | 15.923 | 74.403 | 1.00 | 19.60 | C |
| ATOM | 3967 | CD1 | PHE | B | 201 | 26.670 | 16.594 | 74.871 | 1.00 | 19.60 | C |
| ATOM | 3968 | CD2 | PHE | B | 201 | 27.972 | 15.747 | 73.022 | 1.00 | 19.60 | C |
| ATOM | 3969 | CE1 | PHE | B | 201 | 25.714 | 17.062 | 73.979 | 1.00 | 19.60 | C |
| ATOM | 3970 | CE2 | PHE | B | 201 | 27.031 | 16.207 | 72.129 | 1.00 | 19.60 | C |
| ATOM | 3971 | CZ | PHE | B | 201 | 25.897 | 16.863 | 72.595 | 1.00 | 19.60 | C |
| ATOM | 3972 | C | PHE | B | 201 | 29.165 | 13.382 | 76.977 | 1.00 | 33.51 | C |
| ATOM | 3973 | O | PHE | B | 201 | 30.368 | 13.399 | 76.671 | 1.00 | 33.51 | O |
| ATOM | 3974 | N | GLY | B | 202 | 28.650 | 12.771 | 78.049 | 1.00 | 26.43 | N |
| ATOM | 3975 | CA | GLY | B | 202 | 29.458 | 12.010 | 78.996 | 1.00 | 26.43 | C |
| ATOM | 3976 | C | GLY | B | 202 | 30.028 | 10.738 | 78.363 | 1.00 | 26.43 | C |
| ATOM | 3977 | O | GLY | B | 202 | 30.961 | 10.131 | 78.874 | 1.00 | 26.43 | O |
| ATOM | 3978 | N | SER | B | 203 | 29.501 | 10.352 | 77.210 | 1.00 | 49.15 | N |
| ATOM | 3979 | CA | SER | B | 203 | 29.981 | 9.174 | 76.489 | 1.00 | 49.15 | C |
| ATOM | 3980 | CB | SER | B | 203 | 28.967 | 8.006 | 76.460 | 1.00 | 34.69 | C |
| ATOM | 3981 | OG | SER | B | 203 | 27.875 | 8.104 | 77.391 | 1.00 | 34.69 | O |
| ATOM | 3982 | C | SER | B | 203 | 30.018 | 9.810 | 75.164 | 1.00 | 49.15 | C |
| ATOM | 3983 | O | SER | B | 203 | 28.979 | 10.023 | 74.566 | 1.00 | 49.15 | O |
| ATOM | 3984 | N | ALA | B | 204 | 31.208 | 10.205 | 74.763 | 1.00 | 22.47 | N |
| ATOM | 3985 | CA | ALA | B | 204 | 31.389 | 10.858 | 73.491 | 1.00 | 22.47 | C |
| ATOM | 3986 | CB | ALA | B | 204 | 30.698 | 12.211 | 73.461 | 1.00 | 30.84 | C |
| ATOM | 3987 | C | ALA | B | 204 | 32.871 | 11.031 | 73.306 | 1.00 | 22.47 | C |
| ATOM | 3988 | O | ALA | B | 204 | 33.576 | 11.516 | 74.195 | 1.00 | 22.47 | O |
| ATOM | 3989 | N | LYS | B | 205 | 33.337 | 10.629 | 72.136 | 1.00 | 34.03 | N |
| ATOM | 3990 | CA | LYS | B | 205 | 34.739 | 10.692 | 71.812 | 1.00 | 34.03 | C |
| ATOM | 3991 | CB | LYS | B | 205 | 35.389 | 9.323 | 72.083 | 1.00 | 39.35 | C |
| ATOM | 3992 | CG | LYS | B | 205 | 36.906 | 9.379 | 72.139 | 1.00 | 39.35 | C |
| ATOM | 3993 | CD | LYS | B | 205 | 37.428 | 8.030 | 71.742 | 1.00 | 39.35 | C |
| ATOM | 3994 | CE | LYS | B | 205 | 38.696 | 7.690 | 72.484 | 1.00 | 39.35 | C |
| ATOM | 3995 | NZ | LYS | B | 205 | 39.319 | 6.455 | 71.913 | 1.00 | 39.35 | N |
| ATOM | 3996 | C | LYS | B | 205 | 34.925 | 11.097 | 70.378 | 1.00 | 34.03 | C |
| ATOM | 3997 | O | LYS | B | 205 | 34.090 | 10.835 | 69.522 | 1.00 | 34.03 | O |
| ATOM | 3998 | N | GLN | B | 206 | 36.023 | 11.788 | 70.146 | 1.00 | 41.77 | N |
| ATOM | 3999 | CA | GLN | B | 206 | 36.343 | 12.194 | 68.817 | 1.00 | 41.77 | C |
| ATOM | 4000 | CB | GLN | B | 206 | 37.257 | 13.388 | 68.865 | 1.00 | 59.16 | C |
| ATOM | 4001 | CG | GLN | B | 206 | 37.466 | 14.020 | 67.542 | 1.00 | 59.16 | C |
| ATOM | 4002 | CD | GLN | B | 206 | 38.357 | 15.212 | 67.640 | 1.00 | 59.16 | C |
| ATOM | 4003 | OE1 | GLN | B | 206 | 38.102 | 16.136 | 68.423 | 1.00 | 59.16 | O |
| ATOM | 4004 | NE2 | GLN | B | 206 | 39.419 | 15.213 | 66.842 | 1.00 | 59.16 | N |
| ATOM | 4005 | C | GLN | B | 206 | 37.068 | 10.997 | 68.283 | 1.00 | 41.77 | C |
| ATOM | 4006 | O | GLN | B | 206 | 38.240 | 10.806 | 68.582 | 1.00 | 41.77 | O |
| ATOM | 4007 | N | LEU | B | 207 | 36.357 | 10.177 | 67.520 | 1.00 | 34.07 | N |
| ATOM | 4008 | CA | LEU | B | 207 | 36.934 | 8.960 | 66.985 | 1.00 | 34.07 | C |
| ATOM | 4009 | CB | LEU | B | 207 | 35.848 | 8.122 | 66.324 | 1.00 | 31.55 | C |
| ATOM | 4010 | CG | LEU | B | 207 | 34.497 | 8.065 | 67.048 | 1.00 | 31.55 | C |
| ATOM | 4011 | CD1 | LEU | B | 207 | 33.499 | 7.278 | 66.226 | 1.00 | 31.55 | C |
| ATOM | 4012 | CD2 | LEU | B | 207 | 34.677 | 7.447 | 68.405 | 1.00 | 31.55 | C |
| ATOM | 4013 | C | LEU | B | 207 | 38.042 | 9.213 | 65.988 | 1.00 | 34.07 | C |
| ATOM | 4014 | O | LEU | B | 207 | 37.907 | 10.054 | 65.102 | 1.00 | 34.07 | O |
| ATOM | 4015 | N | VAL | B | 208 | 39.137 | 8.474 | 66.165 | 1.00 | 50.99 | N |
| ATOM | 4016 | CA | VAL | B | 208 | 40.306 | 8.516 | 65.293 | 1.00 | 50.99 | C |
| ATOM | 4017 | CB | VAL | B | 208 | 41.564 | 8.994 | 66.042 | 1.00 | 38.31 | C |
| ATOM | 4018 | CG1 | VAL | B | 208 | 42.782 | 8.897 | 65.126 | 1.00 | 38.31 | C |
| ATOM | 4019 | CG2 | VAL | B | 208 | 41.361 | 10.427 | 66.547 | 1.00 | 38.31 | C |
| ATOM | 4020 | C | VAL | B | 208 | 40.498 | 7.061 | 64.884 | 1.00 | 50.99 | C |
| ATOM | 4021 | O | VAL | B | 208 | 40.202 | 6.148 | 65.666 | 1.00 | 50.99 | O |
| ATOM | 4022 | N | ARG | B | 209 | 40.968 | 6.837 | 63.663 | 1.00 | 50.78 | N |
| ATOM | 4023 | CA | ARG | B | 209 | 41.166 | 5.475 | 63.189 | 1.00 | 50.78 | C |
| ATOM | 4024 | CB | ARG | B | 209 | 40.874 | 5.391 | 61.700 | 1.00 | 99.98 | C |
| ATOM | 4025 | CG | ARG | B | 209 | 41.186 | 6.664 | 60.974 | 1.00 | 99.98 | C |
| ATOM | 4026 | CD | ARG | B | 209 | 41.109 | 6.465 | 59.489 | 1.00 | 99.98 | C |
| ATOM | 4027 | NE | ARG | B | 209 | 41.160 | 7.744 | 58.796 | 1.00 | 99.98 | N |

| | | | | | | | | | | | |
|---|---|---|---|---|---|---|---|---|---|---|---|
| ATOM | 4028 | CZ | ARG | B | 209 | 41.478 | 7.879 | 57.514 | 1.00 | 99.98 | C |
| ATOM | 4029 | NH1 | ARG | B | 209 | 41.779 | 6.808 | 56.789 | 1.00 | 99.98 | N |
| ATOM | 4030 | NH2 | ARG | B | 209 | 41.488 | 9.080 | 56.955 | 1.00 | 99.98 | N |
| ATOM | 4031 | C | ARG | B | 209 | 42.574 | 4.993 | 63.473 | 1.00 | 50.78 | C |
| ATOM | 4032 | O | ARG | B | 209 | 43.554 | 5.651 | 63.112 | 1.00 | 50.78 | O |
| ATOM | 4033 | N | GLY | B | 210 | 42.654 | 3.827 | 64.109 | 1.00 | 70.49 | N |
| ATOM | 4034 | CA | GLY | B | 210 | 43.929 | 3.248 | 64.486 | 1.00 | 70.49 | C |
| ATOM | 4035 | C | GLY | B | 210 | 43.919 | 3.246 | 65.999 | 1.00 | 70.49 | C |
| ATOM | 4036 | O | GLY | B | 210 | 44.499 | 2.382 | 66.661 | 1.00 | 70.49 | O |
| ATOM | 4037 | N | GLU | B | 211 | 43.244 | 4.249 | 66.544 | 1.00 | 57.60 | N |
| ATOM | 4038 | CA | GLU | B | 211 | 43.097 | 4.380 | 67.975 | 1.00 | 57.60 | C |
| ATOM | 4039 | CB | GLU | B | 211 | 43.053 | 5.858 | 68.338 | 1.00 | 61.71 | C |
| ATOM | 4040 | CG | GLU | B | 211 | 44.114 | 6.667 | 67.594 | 1.00 | 61.71 | C |
| ATOM | 4041 | CD | GLU | B | 211 | 44.370 | 8.034 | 68.209 | 1.00 | 61.71 | C |
| ATOM | 4042 | OE1 | GLU | B | 211 | 45.228 | 8.776 | 67.676 | 1.00 | 61.71 | O |
| ATOM | 4043 | OE2 | GLU | B | 211 | 43.716 | 8.363 | 69.226 | 1.00 | 61.71 | O |
| ATOM | 4044 | C | GLU | B | 211 | 41.769 | 3.681 | 68.264 | 1.00 | 57.60 | C |
| ATOM | 4045 | O | GLU | B | 211 | 40.716 | 4.095 | 67.767 | 1.00 | 57.60 | O |
| ATOM | 4046 | N | PRO | B | 212 | 41.811 | 2.591 | 69.048 | 1.00 | 34.38 | N |
| ATOM | 4047 | CD | PRO | B | 212 | 43.047 | 2.027 | 69.611 | 1.00 | 43.35 | C |
| ATOM | 4048 | CA | PRO | B | 212 | 40.657 | 1.772 | 69.438 | 1.00 | 34.38 | C |
| ATOM | 4049 | CB | PRO | B | 212 | 41.299 | 0.533 | 70.070 | 1.00 | 43.35 | C |
| ATOM | 4050 | CG | PRO | B | 212 | 42.737 | 0.569 | 69.609 | 1.00 | 43.35 | C |
| ATOM | 4051 | C | PRO | B | 212 | 39.727 | 2.462 | 70.418 | 1.00 | 34.38 | C |
| ATOM | 4052 | O | PRO | B | 212 | 40.154 | 3.300 | 71.210 | 1.00 | 34.38 | O |
| ATOM | 4053 | N | ASN | B | 213 | 38.452 | 2.094 | 70.360 | 1.00 | 37.24 | N |
| ATOM | 4054 | CA | ASN | B | 213 | 37.449 | 2.650 | 71.258 | 1.00 | 37.24 | C |
| ATOM | 4055 | CB | ASN | B | 213 | 36.440 | 3.495 | 70.487 | 1.00 | 34.37 | C |
| ATOM | 4056 | CG | ASN | B | 213 | 37.074 | 4.251 | 69.356 | 1.00 | 34.37 | C |
| ATOM | 4057 | OD1 | ASN | B | 213 | 37.798 | 5.225 | 69.563 | 1.00 | 34.37 | O |
| ATOM | 4058 | ND2 | ASN | B | 213 | 36.825 | 3.792 | 68.141 | 1.00 | 34.37 | N |
| ATOM | 4059 | C | ASN | B | 213 | 36.744 | 1.462 | 71.883 | 1.00 | 37.24 | C |
| ATOM | 4060 | O | ASN | B | 213 | 36.724 | 0.376 | 71.300 | 1.00 | 37.24 | O |
| ATOM | 4061 | N | VAL | B | 214 | 36.178 | 1.673 | 73.067 | 1.00 | 22.64 | N |
| ATOM | 4062 | CA | VAL | B | 214 | 35.468 | 0.627 | 73.793 | 1.00 | 22.64 | C |
| ATOM | 4063 | CB | VAL | B | 214 | 34.936 | 1.155 | 75.125 | 1.00 | 28.92 | C |
| ATOM | 4064 | CG1 | VAL | B | 214 | 33.924 | 2.266 | 74.862 | 1.00 | 28.92 | C |
| ATOM | 4065 | CG2 | VAL | B | 214 | 34.312 | 0.017 | 75.934 | 1.00 | 28.92 | C |
| ATOM | 4066 | C | VAL | B | 214 | 34.295 | 0.064 | 72.985 | 1.00 | 22.64 | C |
| ATOM | 4067 | O | VAL | B | 214 | 33.646 | 0.785 | 72.221 | 1.00 | 22.64 | O |
| ATOM | 4068 | N | SER | B | 215 | 34.022 | -1.223 | 73.165 | 1.00 | 38.91 | N |
| ATOM | 4069 | CA | SER | B | 215 | 32.955 | -1.864 | 72.422 | 1.00 | 38.91 | C |
| ATOM | 4070 | CB | SER | B | 215 | 33.348 | -3.301 | 72.080 | 1.00 | 43.77 | C |
| ATOM | 4071 | OG | SER | B | 215 | 34.570 | -3.324 | 71.354 | 1.00 | 43.77 | O |
| ATOM | 4072 | C | SER | B | 215 | 31.644 | -1.837 | 73.182 | 1.00 | 38.91 | C |
| ATOM | 4073 | O | SER | B | 215 | 30.600 | -1.573 | 72.599 | 1.00 | 38.91 | O |
| ATOM | 4074 | N | PTY | B | 216 | 31.686 | -2.107 | 74.481 | 1.00 | 37.64 | N |
| ATOM | 4075 | CA | PTY | B | 216 | 30.460 | -2.077 | 75.260 | 1.00 | 37.64 | C |
| ATOM | 4076 | C | PTY | B | 216 | 30.177 | -0.597 | 75.496 | 1.00 | 37.64 | C |
| ATOM | 4077 | O | PTY | B | 216 | 30.525 | -0.015 | 76.518 | 1.00 | 37.64 | O |
| ATOM | 4078 | CB | PTY | B | 216 | 30.625 | -2.844 | 76.580 | 1.00 | 49.59 | C |
| ATOM | 4079 | CG | PTY | B | 216 | 29.332 | -3.212 | 77.227 | 1.00 | 49.59 | C |
| ATOM | 4080 | CD1 | PTY | B | 216 | 28.337 | -4.210 | 76.759 | 1.00 | 49.59 | C |
| ATOM | 4081 | CD2 | PTY | B | 216 | 29.073 | -2.450 | 78.434 | 1.00 | 49.59 | C |
| ATOM | 4082 | CE1 | PTY | B | 216 | 27.097 | -4.414 | 77.531 | 1.00 | 49.59 | C |
| ATOM | 4083 | CE2 | PTY | B | 216 | 27.840 | -2.651 | 79.191 | 1.00 | 49.59 | C |
| ATOM | 4084 | CZ | PTY | B | 216 | 26.858 | -3.623 | 78.751 | 1.00 | 49.59 | C |
| ATOM | 4085 | OH | PTY | B | 216 | 25.853 | -3.750 | 79.600 | 1.00 | 49.59 | O |
| ATOM | 4086 | P | PTY | B | 216 | 24.445 | -4.080 | 79.047 | 1.00 | 49.59 | P |
| ATOM | 4087 | OP1 | PTY | B | 216 | 24.013 | -3.215 | 78.007 | 1.00 | 49.59 | O |
| ATOM | 4088 | OP2 | PTY | B | 216 | 24.335 | -5.510 | 78.412 | 1.00 | 49.59 | O |
| ATOM | 4089 | OP3 | PTY | B | 216 | 23.631 | -3.871 | 80.199 | 1.00 | 49.59 | O |
| ATOM | 4090 | N | ILE | B | 217 | 29.541 | 0.004 | 74.502 | 1.00 | 35.27 | N |

| | | | | | | | | | | | |
|---|---|---|---|---|---|---|---|---|---|---|---|
| ATOM | 4091 | CA | ILE | B | 217 | 29.199 | 1.410 | 74.542 | 1.00 | 35.27 | C |
| ATOM | 4092 | CB | ILE | B | 217 | 30.201 | 2.214 | 73.686 | 1.00 | 15.85 | C |
| ATOM | 4093 | CG2 | ILE | B | 217 | 29.913 | 1.998 | 72.190 | 1.00 | 15.85 | C |
| ATOM | 4094 | CG1 | ILE | B | 217 | 30.144 | 3.698 | 74.061 | 1.00 | 15.85 | C |
| ATOM | 4095 | CD1 | ILE | B | 217 | 30.579 | 3.961 | 75.454 | 1.00 | 15.85 | C |
| ATOM | 4096 | C | ILE | B | 217 | 27.793 | 1.611 | 73.991 | 1.00 | 35.27 | C |
| ATOM | 4097 | O | ILE | B | 217 | 27.382 | 0.881 | 73.103 | 1.00 | 35.27 | O |
| ATOM | 4098 | N | CYS | B | 218 | 27.072 | 2.600 | 74.516 | 1.00 | 29.69 | N |
| ATOM | 4099 | CA | CYS | B | 218 | 25.713 | 2.913 | 74.068 | 1.00 | 29.69 | C |
| ATOM | 4100 | CB | CYS | B | 218 | 25.609 | 2.724 | 72.553 | 1.00 | 43.14 | C |
| ATOM | 4101 | SG | CYS | B | 218 | 24.235 | 3.538 | 71.749 | 1.00 | 43.14 | S |
| ATOM | 4102 | C | CYS | B | 218 | 24.724 | 2.005 | 74.779 | 1.00 | 29.69 | C |
| ATOM | 4103 | O | CYS | B | 218 | 25.003 | 0.817 | 74.967 | 1.00 | 29.69 | O |
| ATOM | 4104 | N | SER | B | 219 | 23.578 | 2.566 | 75.173 | 1.00 | 28.41 | N |
| ATOM | 4105 | CA | SER | B | 219 | 22.553 | 1.799 | 75.886 | 1.00 | 28.41 | C |
| ATOM | 4106 | CB | SER | B | 219 | 21.399 | 2.700 | 76.331 | 1.00 | 35.10 | C |
| ATOM | 4107 | OG | SER | B | 219 | 21.815 | 4.051 | 76.325 | 1.00 | 35.10 | O |
| ATOM | 4108 | C | SER | B | 219 | 22.024 | 0.659 | 75.033 | 1.00 | 28.41 | C |
| ATOM | 4109 | O | SER | B | 219 | 21.578 | 0.870 | 73.901 | 1.00 | 28.41 | O |
| ATOM | 4110 | N | ARG | B | 220 | 22.118 | -0.543 | 75.594 | 1.00 | 30.04 | N |
| ATOM | 4111 | CA | ARG | B | 220 | 21.697 | -1.797 | 74.978 | 1.00 | 30.04 | C |
| ATOM | 4112 | CB | ARG | B | 220 | 21.011 | -2.625 | 76.069 | 1.00 | 50.55 | C |
| ATOM | 4113 | CG | ARG | B | 220 | 21.225 | -4.122 | 76.017 | 1.00 | 50.55 | C |
| ATOM | 4114 | CD | ARG | B | 220 | 21.095 | -4.643 | 77.436 | 1.00 | 50.55 | C |
| ATOM | 4115 | NE | ARG | B | 220 | 19.913 | -4.091 | 78.093 | 1.00 | 50.55 | N |
| ATOM | 4116 | CZ | ARG | B | 220 | 19.669 | -4.256 | 79.386 | 1.00 | 50.55 | C |
| ATOM | 4117 | NH1 | ARG | B | 220 | 20.530 | -4.968 | 80.112 | 1.00 | 50.55 | N |
| ATOM | 4118 | NH2 | ARG | B | 220 | 18.623 | -3.676 | 79.975 | 1.00 | 50.55 | N |
| ATOM | 4119 | C | ARG | B | 220 | 20.755 | -1.619 | 73.764 | 1.00 | 30.04 | C |
| ATOM | 4120 | O | ARG | B | 220 | 21.087 | -1.971 | 72.620 | 1.00 | 30.04 | O |
| ATOM | 4121 | N | TYR | B | 221 | 19.582 | -1.067 | 74.056 | 1.00 | 18.10 | N |
| ATOM | 4122 | CA | TYR | B | 221 | 18.512 | -0.835 | 73.111 | 1.00 | 18.10 | C |
| ATOM | 4123 | CB | TYR | B | 221 | 17.440 | -0.019 | 73.801 | 1.00 | 35.14 | C |
| ATOM | 4124 | CG | TYR | B | 221 | 16.538 | -0.856 | 74.662 | 1.00 | 35.14 | C |
| ATOM | 4125 | CD1 | TYR | B | 221 | 17.039 | -1.908 | 75.450 | 1.00 | 35.14 | C |
| ATOM | 4126 | CE1 | TYR | B | 221 | 16.192 | -2.672 | 76.255 | 1.00 | 35.14 | C |
| ATOM | 4127 | CD2 | TYR | B | 221 | 15.175 | -0.598 | 74.709 | 1.00 | 35.14 | C |
| ATOM | 4128 | CE2 | TYR | B | 221 | 14.328 | -1.356 | 75.512 | 1.00 | 35.14 | C |
| ATOM | 4129 | CZ | TYR | B | 221 | 14.839 | -2.384 | 76.275 | 1.00 | 35.14 | C |
| ATOM | 4130 | OH | TYR | B | 221 | 13.970 | -3.137 | 77.024 | 1.00 | 35.14 | O |
| ATOM | 4131 | C | TYR | B | 221 | 18.898 | -0.144 | 71.825 | 1.00 | 18.10 | C |
| ATOM | 4132 | O | TYR | B | 221 | 18.393 | -0.496 | 70.751 | 1.00 | 18.10 | O |
| ATOM | 4133 | N | TYR | B | 222 | 19.766 | 0.857 | 71.921 | 1.00 | 22.19 | N |
| ATOM | 4134 | CA | TYR | B | 222 | 20.145 | 1.603 | 70.737 | 1.00 | 22.19 | C |
| ATOM | 4135 | CB | TYR | B | 222 | 19.882 | 3.085 | 70.980 | 1.00 | 27.11 | C |
| ATOM | 4136 | CG | TYR | B | 222 | 18.570 | 3.341 | 71.703 | 1.00 | 27.11 | C |
| ATOM | 4137 | CD1 | TYR | B | 222 | 18.466 | 3.136 | 73.086 | 1.00 | 27.11 | C |
| ATOM | 4138 | CE1 | TYR | B | 222 | 17.274 | 3.329 | 73.746 | 1.00 | 27.11 | C |
| ATOM | 4139 | CD2 | TYR | B | 222 | 17.421 | 3.748 | 71.001 | 1.00 | 27.11 | C |
| ATOM | 4140 | CE2 | TYR | B | 222 | 16.219 | 3.940 | 71.664 | 1.00 | 27.11 | C |
| ATOM | 4141 | CZ | TYR | B | 222 | 16.161 | 3.729 | 73.035 | 1.00 | 27.11 | C |
| ATOM | 4142 | OH | TYR | B | 222 | 14.990 | 3.943 | 73.706 | 1.00 | 27.11 | O |
| ATOM | 4143 | C | TYR | B | 222 | 21.592 | 1.369 | 70.363 | 1.00 | 22.19 | C |
| ATOM | 4144 | O | TYR | B | 222 | 22.187 | 2.157 | 69.630 | 1.00 | 22.19 | O |
| ATOM | 4145 | N | ARG | B | 223 | 22.148 | 0.280 | 70.879 | 1.00 | 26.61 | N |
| ATOM | 4146 | CA | ARG | B | 223 | 23.522 | -0.081 | 70.592 | 1.00 | 26.61 | C |
| ATOM | 4147 | CB | ARG | B | 223 | 24.056 | -1.052 | 71.661 | 1.00 | 42.92 | C |
| ATOM | 4148 | CG | ARG | B | 223 | 25.440 | -1.659 | 71.371 | 1.00 | 42.92 | C |
| ATOM | 4149 | CD | ARG | B | 223 | 26.250 | -1.783 | 72.660 | 1.00 | 42.92 | C |
| ATOM | 4150 | NE | ARG | B | 223 | 25.431 | -2.282 | 73.762 | 1.00 | 42.92 | N |
| ATOM | 4151 | CZ | ARG | B | 223 | 25.616 | -1.982 | 75.045 | 1.00 | 42.92 | C |
| ATOM | 4152 | NH1 | ARG | B | 223 | 26.606 | -1.175 | 75.416 | 1.00 | 42.92 | N |
| ATOM | 4153 | NH2 | ARG | B | 223 | 24.790 | -2.468 | 75.958 | 1.00 | 42.92 | N |

| | | | | | | | | | | | |
|---|---|---|---|---|---|---|---|---|---|---|---|
| ATOM | 4154 | C | ARG | B | 223 | 23.566 | -0.714 | 69.206 | 1.00 | 26.61 | C |
| ATOM | 4155 | O | ARG | B | 223 | 22.857 | -1.684 | 68.918 | 1.00 | 26.61 | O |
| ATOM | 4156 | N | ALA | B | 224 | 24.391 | -0.134 | 68.340 | 1.00 | 28.20 | N |
| ATOM | 4157 | CA | ALA | B | 224 | 24.554 | -0.633 | 66.975 | 1.00 | 28.20 | C |
| ATOM | 4158 | CB | ALA | B | 224 | 25.569 | 0.222 | 66.215 | 1.00 | 13.65 | C |
| ATOM | 4159 | C | ALA | B | 224 | 25.017 | -2.088 | 67.019 | 1.00 | 28.20 | C |
| ATOM | 4160 | O | ALA | B | 224 | 25.612 | -2.530 | 68.002 | 1.00 | 28.20 | O |
| ATOM | 4161 | N | PRO | B | 225 | 24.759 | -2.852 | 65.949 | 1.00 | 42.94 | N |
| ATOM | 4162 | CD | PRO | B | 225 | 24.164 | -2.444 | 64.662 | 1.00 | 40.19 | C |
| ATOM | 4163 | CA | PRO | B | 225 | 25.169 | -4.260 | 65.920 | 1.00 | 42.94 | C |
| ATOM | 4164 | CB | PRO | B | 225 | 24.631 | -4.742 | 64.574 | 1.00 | 40.19 | C |
| ATOM | 4165 | CG | PRO | B | 225 | 24.674 | -3.498 | 63.723 | 1.00 | 40.19 | C |
| ATOM | 4166 | C | PRO | B | 225 | 26.678 | -4.493 | 66.086 | 1.00 | 42.94 | C |
| ATOM | 4167 | O | PRO | B | 225 | 27.090 | -5.323 | 66.901 | 1.00 | 42.94 | O |
| ATOM | 4168 | N | GLU | B | 226 | 27.490 | -3.755 | 65.327 | 1.00 | 47.96 | N |
| ATOM | 4169 | CA | GLU | B | 226 | 28.948 | -3.881 | 65.384 | 1.00 | 47.96 | C |
| ATOM | 4170 | CB | GLU | B | 226 | 29.624 | -2.733 | 64.638 | 1.00 | 31.25 | C |
| ATOM | 4171 | CG | GLU | B | 226 | 28.873 | -2.259 | 63.429 | 1.00 | 31.25 | C |
| ATOM | 4172 | CD | GLU | B | 226 | 28.116 | -0.972 | 63.696 | 1.00 | 31.25 | C |
| ATOM | 4173 | OE1 | GLU | B | 226 | 28.765 | 0.078 | 63.888 | 1.00 | 31.25 | O |
| ATOM | 4174 | OE2 | GLU | B | 226 | 26.871 | -1.010 | 63.722 | 1.00 | 31.25 | O |
| ATOM | 4175 | C | GLU | B | 226 | 29.471 | -3.883 | 66.812 | 1.00 | 47.96 | C |
| ATOM | 4176 | O | GLU | B | 226 | 30.311 | -4.704 | 67.172 | 1.00 | 47.96 | O |
| ATOM | 4177 | N | LEU | B | 227 | 28.990 | -2.947 | 67.620 | 1.00 | 43.36 | N |
| ATOM | 4178 | CA | LEU | B | 227 | 29.428 | -2.871 | 69.000 | 1.00 | 43.36 | C |
| ATOM | 4179 | CB | LEU | B | 227 | 28.677 | -1.740 | 69.718 | 1.00 | 26.27 | C |
| ATOM | 4180 | CG | LEU | B | 227 | 28.762 | -0.352 | 69.048 | 1.00 | 26.27 | C |
| ATOM | 4181 | CD1 | LEU | B | 227 | 27.926 | 0.633 | 69.835 | 1.00 | 26.27 | C |
| ATOM | 4182 | CD2 | LEU | B | 227 | 30.214 | 0.140 | 68.951 | 1.00 | 26.27 | C |
| ATOM | 4183 | C | LEU | B | 227 | 29.200 | -4.220 | 69.694 | 1.00 | 43.36 | C |
| ATOM | 4184 | O | LEU | B | 227 | 30.099 | -4.748 | 70.348 | 1.00 | 43.36 | O |
| ATOM | 4185 | N | ILE | B | 228 | 28.003 | -4.778 | 69.541 | 1.00 | 54.79 | N |
| ATOM | 4186 | CA | ILE | B | 228 | 27.667 | -6.071 | 70.136 | 1.00 | 54.79 | C |
| ATOM | 4187 | CB | ILE | B | 228 | 26.220 | -6.461 | 69.760 | 1.00 | 48.10 | C |
| ATOM | 4188 | CG2 | ILE | B | 228 | 25.846 | -7.802 | 70.392 | 1.00 | 48.10 | C |
| ATOM | 4189 | CG1 | ILE | B | 228 | 25.271 | -5.338 | 70.201 | 1.00 | 48.10 | C |
| ATOM | 4190 | CD1 | ILE | B | 228 | 23.840 | -5.526 | 69.793 | 1.00 | 48.10 | C |
| ATOM | 4191 | C | ILE | B | 228 | 28.647 | -7.149 | 69.635 | 1.00 | 54.79 | C |
| ATOM | 4192 | O | ILE | B | 228 | 28.967 | -8.106 | 70.346 | 1.00 | 54.79 | O |
| ATOM | 4193 | N | PHE | B | 229 | 29.116 | -6.973 | 68.402 | 1.00 | 32.87 | N |
| ATOM | 4194 | CA | PHE | B | 229 | 30.067 | -7.886 | 67.781 | 1.00 | 32.87 | C |
| ATOM | 4195 | CB | PHE | B | 229 | 29.907 | -7.846 | 66.263 | 1.00 | 66.75 | C |
| ATOM | 4196 | CG | PHE | B | 229 | 28.689 | -8.561 | 65.774 | 1.00 | 66.75 | C |
| ATOM | 4197 | CD1 | PHE | B | 229 | 28.799 | -9.566 | 64.824 | 1.00 | 66.75 | C |
| ATOM | 4198 | CD2 | PHE | B | 229 | 27.436 | -8.260 | 66.290 | 1.00 | 66.75 | C |
| ATOM | 4199 | CE1 | PHE | B | 229 | 27.678 | -10.266 | 64.399 | 1.00 | 66.75 | C |
| ATOM | 4200 | CE2 | PHE | B | 229 | 26.309 | -8.955 | 65.869 | 1.00 | 66.75 | C |
| ATOM | 4201 | CZ | PHE | B | 229 | 26.431 | -9.958 | 64.925 | 1.00 | 66.75 | C |
| ATOM | 4202 | C | PHE | B | 229 | 31.511 | -7.562 | 68.167 | 1.00 | 32.87 | C |
| ATOM | 4203 | O | PHE | B | 229 | 32.455 | -8.021 | 67.528 | 1.00 | 32.87 | O |
| ATOM | 4204 | N | GLY | B | 230 | 31.672 | -6.747 | 69.203 | 1.00 | 46.55 | N |
| ATOM | 4205 | CA | GLY | B | 230 | 33.000 | -6.409 | 69.680 | 1.00 | 46.55 | C |
| ATOM | 4206 | C | GLY | B | 230 | 33.875 | -5.487 | 68.852 | 1.00 | 46.55 | C |
| ATOM | 4207 | O | GLY | B | 230 | 35.070 | -5.356 | 69.126 | 1.00 | 46.55 | O |
| ATOM | 4208 | N | ALA | B | 231 | 33.308 | -4.851 | 67.838 | 1.00 | 48.81 | N |
| ATOM | 4209 | CA | ALA | B | 231 | 34.097 | -3.935 | 67.029 | 1.00 | 48.81 | C |
| ATOM | 4210 | CB | ALA | B | 231 | 33.205 | -3.266 | 65.990 | 1.00 | 20.78 | C |
| ATOM | 4211 | C | ALA | B | 231 | 34.708 | -2.883 | 67.958 | 1.00 | 48.81 | C |
| ATOM | 4212 | O | ALA | B | 231 | 34.078 | -2.455 | 68.923 | 1.00 | 48.81 | O |
| ATOM | 4213 | N | THR | B | 232 | 35.935 | -2.472 | 67.684 | 1.00 | 46.21 | N |
| ATOM | 4214 | CA | THR | B | 232 | 36.575 | -1.466 | 68.520 | 1.00 | 46.21 | C |
| ATOM | 4215 | CB | THR | B | 232 | 37.795 | -2.061 | 69.294 | 1.00 | 29.55 | C |
| ATOM | 4216 | OG1 | THR | B | 232 | 38.786 | -2.516 | 68.358 | 1.00 | 29.55 | O |

| | | | | | | | | | | | |
|---|---|---|---|---|---|---|---|---|---|---|---|
| ATOM | 4217 | CG2 | THR | B | 232 | 37.359 | -3.230 | 70.178 | 1.00 | 29.55 | C |
| ATOM | 4218 | C | THR | B | 232 | 37.049 | -0.367 | 67.579 | 1.00 | 46.21 | C |
| ATOM | 4219 | O | THR | B | 232 | 37.444 | 0.729 | 67.998 | 1.00 | 46.21 | O |
| ATOM | 4220 | N | ASP | B | 233 | 36.981 | -0.691 | 66.293 | 1.00 | 41.91 | N |
| ATOM | 4221 | CA | ASP | B | 233 | 37.414 | 0.192 | 65.223 | 1.00 | 41.91 | C |
| ATOM | 4222 | CB | ASP | B | 233 | 38.358 | -0.580 | 64.294 | 1.00 | 94.75 | C |
| ATOM | 4223 | CG | ASP | B | 233 | 37.842 | -1.975 | 63.955 | 1.00 | 94.75 | C |
| ATOM | 4224 | OD1 | ASP | B | 233 | 37.465 | -2.739 | 64.875 | 1.00 | 94.75 | O |
| ATOM | 4225 | OD2 | ASP | B | 233 | 37.824 | -2.319 | 62.756 | 1.00 | 94.75 | O |
| ATOM | 4226 | C | ASP | B | 233 | 36.243 | 0.786 | 64.436 | 1.00 | 41.91 | C |
| ATOM | 4227 | O | ASP | B | 233 | 36.374 | 1.097 | 63.249 | 1.00 | 41.91 | O |
| ATOM | 4228 | N | TYR | B | 234 | 35.109 | 0.967 | 65.116 | 1.00 | 27.87 | N |
| ATOM | 4229 | CA | TYR | B | 234 | 33.901 | 1.521 | 64.508 | 1.00 | 27.87 | C |
| ATOM | 4230 | CB | TYR | B | 234 | 32.702 | 1.262 | 65.420 | 1.00 | 29.14 | C |
| ATOM | 4231 | CG | TYR | B | 234 | 32.902 | 1.733 | 66.845 | 1.00 | 29.14 | C |
| ATOM | 4232 | CD1 | TYR | B | 234 | 32.712 | 3.074 | 67.203 | 1.00 | 29.14 | C |
| ATOM | 4233 | CE1 | TYR | B | 234 | 32.892 | 3.490 | 68.524 | 1.00 | 29.14 | C |
| ATOM | 4234 | CD2 | TYR | B | 234 | 33.278 | 0.831 | 67.840 | 1.00 | 29.14 | C |
| ATOM | 4235 | CE2 | TYR | B | 234 | 33.457 | 1.233 | 69.147 | 1.00 | 29.14 | C |
| ATOM | 4236 | CZ | TYR | B | 234 | 33.262 | 2.553 | 69.487 | 1.00 | 29.14 | C |
| ATOM | 4237 | OH | TYR | B | 234 | 33.419 | 2.926 | 70.800 | 1.00 | 29.14 | O |
| ATOM | 4238 | C | TYR | B | 234 | 33.995 | 3.012 | 64.236 | 1.00 | 27.87 | C |
| ATOM | 4239 | O | TYR | B | 234 | 34.943 | 3.680 | 64.657 | 1.00 | 27.87 | O |
| ATOM | 4240 | N | THR | B | 235 | 32.996 | 3.540 | 63.539 | 1.00 | 37.97 | N |
| ATOM | 4241 | CA | THR | B | 235 | 32.984 | 4.967 | 63.233 | 1.00 | 37.97 | C |
| ATOM | 4242 | CB | THR | B | 235 | 32.951 | 5.226 | 61.719 | 1.00 | 36.63 | C |
| ATOM | 4243 | OG1 | THR | B | 235 | 31.641 | 4.930 | 61.212 | 1.00 | 36.63 | O |
| ATOM | 4244 | CG2 | THR | B | 235 | 33.988 | 4.359 | 61.014 | 1.00 | 36.63 | C |
| ATOM | 4245 | C | THR | B | 235 | 31.793 | 5.692 | 63.845 | 1.00 | 37.97 | C |
| ATOM | 4246 | O | THR | B | 235 | 30.957 | 5.105 | 64.541 | 1.00 | 37.97 | O |
| ATOM | 4247 | N | SER | B | 236 | 31.725 | 6.986 | 63.575 | 1.00 | 34.82 | N |
| ATOM | 4248 | CA | SER | B | 236 | 30.636 | 7.785 | 64.093 | 1.00 | 34.82 | C |
| ATOM | 4249 | CB | SER | B | 236 | 30.962 | 9.287 | 64.023 | 1.00 | 41.56 | C |
| ATOM | 4250 | OG | SER | B | 236 | 31.075 | 9.745 | 62.686 | 1.00 | 41.56 | O |
| ATOM | 4251 | C | SER | B | 236 | 29.380 | 7.477 | 63.300 | 1.00 | 34.82 | C |
| ATOM | 4252 | O | SER | B | 236 | 28.451 | 8.273 | 63.268 | 1.00 | 34.82 | O |
| ATOM | 4253 | N | SER | B | 237 | 29.359 | 6.317 | 62.651 | 1.00 | 39.31 | N |
| ATOM | 4254 | CA | SER | B | 237 | 28.180 | 5.904 | 61.893 | 1.00 | 39.31 | C |
| ATOM | 4255 | CB | SER | B | 237 | 28.558 | 5.056 | 60.669 | 1.00 | 55.67 | C |
| ATOM | 4256 | OG | SER | B | 237 | 29.110 | 3.805 | 61.041 | 1.00 | 55.67 | O |
| ATOM | 4257 | C | SER | B | 237 | 27.298 | 5.087 | 62.822 | 1.00 | 39.31 | C |
| ATOM | 4258 | O | SER | B | 237 | 26.237 | 4.620 | 62.433 | 1.00 | 39.31 | O |
| ATOM | 4259 | N | ILE | B | 238 | 27.744 | 4.921 | 64.061 | 1.00 | 29.82 | N |
| ATOM | 4260 | CA | ILE | B | 238 | 26.977 | 4.162 | 65.039 | 1.00 | 29.82 | C |
| ATOM | 4261 | CB | ILE | B | 238 | 27.889 | 3.605 | 66.148 | 1.00 | 25.88 | C |
| ATOM | 4262 | CG2 | ILE | B | 238 | 28.997 | 2.801 | 65.532 | 1.00 | 25.88 | C |
| ATOM | 4263 | CG1 | ILE | B | 238 | 28.501 | 4.749 | 66.946 | 1.00 | 25.88 | C |
| ATOM | 4264 | CD1 | ILE | B | 238 | 29.011 | 4.338 | 68.317 | 1.00 | 25.88 | C |
| ATOM | 4265 | C | ILE | B | 238 | 25.859 | 5.010 | 65.665 | 1.00 | 29.82 | C |
| ATOM | 4266 | O | ILE | B | 238 | 24.930 | 4.474 | 66.271 | 1.00 | 29.82 | O |
| ATOM | 4267 | N | ASP | B | 239 | 25.967 | 6.333 | 65.524 | 1.00 | 20.72 | N |
| ATOM | 4268 | CA | ASP | B | 239 | 24.956 | 7.255 | 66.030 | 1.00 | 20.72 | C |
| ATOM | 4269 | CB | ASP | B | 239 | 25.481 | 8.702 | 66.072 | 1.00 | 20.04 | C |
| ATOM | 4270 | CG | ASP | B | 239 | 26.350 | 8.997 | 67.303 | 1.00 | 20.04 | C |
| ATOM | 4271 | OD1 | ASP | B | 239 | 26.064 | 8.445 | 68.386 | 1.00 | 20.04 | O |
| ATOM | 4272 | OD2 | ASP | B | 239 | 27.304 | 9.801 | 67.205 | 1.00 | 20.04 | O |
| ATOM | 4273 | C | ASP | B | 239 | 23.752 | 7.197 | 65.092 | 1.00 | 20.72 | C |
| ATOM | 4274 | O | ASP | B | 239 | 22.608 | 7.393 | 65.513 | 1.00 | 20.72 | O |
| ATOM | 4275 | N | VAL | B | 240 | 24.019 | 6.934 | 63.817 | 1.00 | 23.28 | N |
| ATOM | 4276 | CA | VAL | B | 240 | 22.962 | 6.855 | 62.826 | 1.00 | 23.28 | C |
| ATOM | 4277 | CB | VAL | B | 240 | 23.549 | 6.719 | 61.421 | 1.00 | 24.25 | C |
| ATOM | 4278 | CG1 | VAL | B | 240 | 22.430 | 6.606 | 60.411 | 1.00 | 24.25 | C |
| ATOM | 4279 | CG2 | VAL | B | 240 | 24.434 | 7.916 | 61.113 | 1.00 | 24.25 | C |

| | | | | | | | | | | | |
|---|---|---|---|---|---|---|---|---|---|---|---|
| ATOM | 4280 | C | VAL | B | 240 | 22.056 | 5.661 | 63.120 | 1.00 | 23.28 | C |
| ATOM | 4281 | O | VAL | B | 240 | 20.839 | 5.721 | 62.915 | 1.00 | 23.28 | O |
| ATOM | 4282 | N | TRP | B | 241 | 22.657 | 4.573 | 63.595 | 1.00 | 24.82 | N |
| ATOM | 4283 | CA | TRP | B | 241 | 21.898 | 3.379 | 63.948 | 1.00 | 24.82 | C |
| ATOM | 4284 | CB | TRP | B | 241 | 22.841 | 2.295 | 64.467 | 1.00 | 25.44 | C |
| ATOM | 4285 | CG | TRP | B | 241 | 22.132 | 1.088 | 64.981 | 1.00 | 25.44 | C |
| ATOM | 4286 | CD2 | TRP | B | 241 | 21.711 | -0.033 | 64.210 | 1.00 | 25.44 | C |
| ATOM | 4287 | CE2 | TRP | B | 241 | 21.032 | -0.915 | 65.087 | 1.00 | 25.44 | C |
| ATOM | 4288 | CE3 | TRP | B | 241 | 21.870 | -0.401 | 62.867 | 1.00 | 25.44 | C |
| ATOM | 4289 | CD1 | TRP | B | 241 | 21.688 | 0.869 | 66.256 | 1.00 | 25.44 | C |
| ATOM | 4290 | NE1 | TRP | B | 241 | 21.021 | -0.330 | 66.325 | 1.00 | 25.44 | N |
| ATOM | 4291 | CZ2 | TRP | B | 241 | 20.467 | -2.126 | 64.651 | 1.00 | 25.44 | C |
| ATOM | 4292 | CZ3 | TRP | B | 241 | 21.307 | -1.615 | 62.430 | 1.00 | 25.44 | C |
| ATOM | 4293 | CH2 | TRP | B | 241 | 20.630 | -2.466 | 63.329 | 1.00 | 25.44 | C |
| ATOM | 4294 | C | TRP | B | 241 | 20.914 | 3.767 | 65.042 | 1.00 | 24.82 | C |
| ATOM | 4295 | O | TRP | B | 241 | 19.710 | 3.553 | 64.919 | 1.00 | 24.82 | O |
| ATOM | 4296 | N | SER | B | 242 | 21.459 | 4.330 | 66.116 | 1.00 | 24.85 | N |
| ATOM | 4297 | CA | SER | B | 242 | 20.678 | 4.793 | 67.249 | 1.00 | 24.85 | C |
| ATOM | 4298 | CB | SER | B | 242 | 21.590 | 5.534 | 68.240 | 1.00 | 26.97 | C |
| ATOM | 4299 | OG | SER | B | 242 | 22.735 | 4.767 | 68.584 | 1.00 | 26.97 | O |
| ATOM | 4300 | C | SER | B | 242 | 19.598 | 5.745 | 66.721 | 1.00 | 24.85 | C |
| ATOM | 4301 | O | SER | B | 242 | 18.446 | 5.715 | 67.159 | 1.00 | 24.85 | O |
| ATOM | 4302 | N | ALA | B | 243 | 19.976 | 6.598 | 65.774 | 1.00 | 25.24 | N |
| ATOM | 4303 | CA | ALA | B | 243 | 19.023 | 7.535 | 65.189 | 1.00 | 25.24 | C |
| ATOM | 4304 | CB | ALA | B | 243 | 19.702 | 8.348 | 64.101 | 1.00 | 27.33 | C |
| ATOM | 4305 | C | ALA | B | 243 | 17.841 | 6.735 | 64.618 | 1.00 | 25.24 | C |
| ATOM | 4306 | O | ALA | B | 243 | 16.677 | 7.052 | 64.874 | 1.00 | 25.24 | O |
| ATOM | 4307 | N | GLY | B | 244 | 18.152 | 5.694 | 63.852 | 1.00 | 26.88 | N |
| ATOM | 4308 | CA | GLY | B | 244 | 17.114 | 4.846 | 63.298 | 1.00 | 26.88 | C |
| ATOM | 4309 | C | GLY | B | 244 | 16.310 | 4.152 | 64.396 | 1.00 | 26.88 | C |
| ATOM | 4310 | O | GLY | B | 244 | 15.100 | 3.949 | 64.251 | 1.00 | 26.88 | O |
| ATOM | 4311 | N | CYS | B | 245 | 16.969 | 3.800 | 65.502 | 1.00 | 36.99 | N |
| ATOM | 4312 | CA | CYS | B | 245 | 16.294 | 3.125 | 66.612 | 1.00 | 36.99 | C |
| ATOM | 4313 | CB | CYS | B | 245 | 17.301 | 2.610 | 67.650 | 1.00 | 29.55 | C |
| ATOM | 4314 | SG | CYS | B | 245 | 18.170 | 1.086 | 67.175 | 1.00 | 29.55 | S |
| ATOM | 4315 | C | CYS | B | 245 | 15.310 | 4.045 | 67.293 | 1.00 | 36.99 | C |
| ATOM | 4316 | O | CYS | B | 245 | 14.285 | 3.601 | 67.815 | 1.00 | 36.99 | O |
| ATOM | 4317 | N | VAL | B | 246 | 15.618 | 5.334 | 67.294 | 1.00 | 19.27 | N |
| ATOM | 4318 | CA | VAL | B | 246 | 14.730 | 6.288 | 67.933 | 1.00 | 19.27 | C |
| ATOM | 4319 | CB | VAL | B | 246 | 15.467 | 7.632 | 68.237 | 1.00 | 11.03 | C |
| ATOM | 4320 | CG1 | VAL | B | 246 | 14.482 | 8.687 | 68.766 | 1.00 | 11.03 | C |
| ATOM | 4321 | CG2 | VAL | B | 246 | 16.560 | 7.392 | 69.261 | 1.00 | 11.03 | C |
| ATOM | 4322 | C | VAL | B | 246 | 13.536 | 6.523 | 67.016 | 1.00 | 19.27 | C |
| ATOM | 4323 | O | VAL | B | 246 | 12.390 | 6.562 | 67.471 | 1.00 | 19.27 | O |
| ATOM | 4324 | N | LEU | B | 247 | 13.821 | 6.656 | 65.722 | 1.00 | 27.56 | N |
| ATOM | 4325 | CA | LEU | B | 247 | 12.794 | 6.885 | 64.720 | 1.00 | 27.56 | C |
| ATOM | 4326 | CB | LEU | B | 247 | 13.420 | 6.983 | 63.331 | 1.00 | 23.05 | C |
| ATOM | 4327 | CG | LEU | B | 247 | 12.453 | 7.041 | 62.144 | 1.00 | 23.05 | C |
| ATOM | 4328 | CD1 | LEU | B | 247 | 11.548 | 8.273 | 62.237 | 1.00 | 23.05 | C |
| ATOM | 4329 | CD2 | LEU | B | 247 | 13.264 | 7.050 | 60.858 | 1.00 | 23.05 | C |
| ATOM | 4330 | C | LEU | B | 247 | 11.810 | 5.735 | 64.742 | 1.00 | 27.56 | C |
| ATOM | 4331 | O | LEU | B | 247 | 10.590 | 5.934 | 64.845 | 1.00 | 27.56 | O |
| ATOM | 4332 | N | ALA | B | 248 | 12.350 | 4.526 | 64.644 | 1.00 | 30.09 | N |
| ATOM | 4333 | CA | ALA | B | 248 | 11.515 | 3.339 | 64.640 | 1.00 | 30.09 | C |
| ATOM | 4334 | CB | ALA | B | 248 | 12.367 | 2.114 | 64.629 | 1.00 | 4.68 | C |
| ATOM | 4335 | C | ALA | B | 248 | 10.634 | 3.338 | 65.867 | 1.00 | 30.09 | C |
| ATOM | 4336 | O | ALA | B | 248 | 9.434 | 3.051 | 65.795 | 1.00 | 30.09 | O |
| ATOM | 4337 | N | GLU | B | 249 | 11.245 | 3.680 | 66.994 | 1.00 | 31.18 | N |
| ATOM | 4338 | CA | GLU | B | 249 | 10.547 | 3.723 | 68.265 | 1.00 | 31.18 | C |
| ATOM | 4339 | CB | GLU | B | 249 | 11.538 | 4.039 | 69.376 | 1.00 | 22.39 | C |
| ATOM | 4340 | CG | GLU | B | 249 | 10.980 | 3.869 | 70.782 | 1.00 | 22.39 | C |
| ATOM | 4341 | CD | GLU | B | 249 | 12.048 | 4.034 | 71.837 | 1.00 | 22.39 | C |
| ATOM | 4342 | OE1 | GLU | B | 249 | 13.173 | 3.564 | 71.603 | 1.00 | 22.39 | O |

| | | | | | | | | | | | |
|---|---|---|---|---|---|---|---|---|---|---|---|
| ATOM | 4343 | OE2 | GLU | B | 249 | 11.774 | 4.616 | 72.899 | 1.00 | 22.39 | O |
| ATOM | 4344 | C | GLU | B | 249 | 9.415 | 4.736 | 68.296 | 1.00 | 31.18 | C |
| ATOM | 4345 | O | GLU | B | 249 | 8.385 | 4.503 | 68.931 | 1.00 | 31.18 | O |
| ATOM | 4346 | N | LEU | B | 250 | 9.617 | 5.869 | 67.627 | 1.00 | 29.29 | N |
| ATOM | 4347 | CA | LEU | B | 250 | 8.602 | 6.909 | 67.585 | 1.00 | 29.29 | C |
| ATOM | 4348 | CB | LEU | B | 250 | 9.205 | 8.206 | 67.050 | 1.00 | 14.11 | C |
| ATOM | 4349 | CG | LEU | B | 250 | 10.156 | 8.927 | 68.018 | 1.00 | 14.11 | C |
| ATOM | 4350 | CD1 | LEU | B | 250 | 10.557 | 10.286 | 67.409 | 1.00 | 14.11 | C |
| ATOM | 4351 | CD2 | LEU | B | 250 | 9.493 | 9.132 | 69.392 | 1.00 | 14.11 | C |
| ATOM | 4352 | C | LEU | B | 250 | 7.447 | 6.450 | 66.712 | 1.00 | 29.29 | C |
| ATOM | 4353 | O | LEU | B | 250 | 6.292 | 6.760 | 66.968 | 1.00 | 29.29 | O |
| ATOM | 4354 | N | LEU | B | 251 | 7.768 | 5.682 | 65.684 | 1.00 | 35.46 | N |
| ATOM | 4355 | CA | LEU | B | 251 | 6.745 | 5.193 | 64.795 | 1.00 | 35.46 | C |
| ATOM | 4356 | CB | LEU | B | 251 | 7.375 | 4.710 | 63.488 | 1.00 | 20.66 | C |
| ATOM | 4357 | CG | LEU | B | 251 | 7.983 | 5.807 | 62.621 | 1.00 | 20.66 | C |
| ATOM | 4358 | CD1 | LEU | B | 251 | 8.573 | 5.157 | 61.361 | 1.00 | 20.66 | C |
| ATOM | 4359 | CD2 | LEU | B | 251 | 6.910 | 6.850 | 62.295 | 1.00 | 20.66 | C |
| ATOM | 4360 | C | LEU | B | 251 | 5.994 | 4.064 | 65.475 | 1.00 | 35.46 | C |
| ATOM | 4361 | O | LEU | B | 251 | 4.767 | 3.989 | 65.377 | 1.00 | 35.46 | O |
| ATOM | 4362 | N | LEU | B | 252 | 6.729 | 3.218 | 66.202 | 1.00 | 37.91 | N |
| ATOM | 4363 | CA | LEU | B | 252 | 6.131 | 2.070 | 66.869 | 1.00 | 37.91 | C |
| ATOM | 4364 | CB | LEU | B | 252 | 7.195 | 1.020 | 67.189 | 1.00 | 41.94 | C |
| ATOM | 4365 | CG | LEU | B | 252 | 6.899 | -0.394 | 66.711 | 1.00 | 41.94 | C |
| ATOM | 4366 | CD1 | LEU | B | 252 | 6.724 | -0.361 | 65.211 | 1.00 | 41.94 | C |
| ATOM | 4367 | CD2 | LEU | B | 252 | 8.034 | -1.344 | 67.129 | 1.00 | 41.94 | C |
| ATOM | 4368 | C | LEU | B | 252 | 5.378 | 2.473 | 68.130 | 1.00 | 37.91 | C |
| ATOM | 4369 | O | LEU | B | 252 | 4.210 | 2.185 | 68.243 | 1.00 | 37.91 | O |
| ATOM | 4370 | N | GLY | B | 253 | 6.010 | 3.169 | 69.063 | 1.00 | 40.49 | N |
| ATOM | 4371 | CA | GLY | B | 253 | 5.314 | 3.544 | 70.279 | 1.00 | 40.49 | C |
| ATOM | 4372 | C | GLY | B | 253 | 5.983 | 2.771 | 71.403 | 1.00 | 40.49 | C |
| ATOM | 4373 | O | GLY | B | 253 | 5.494 | 2.792 | 72.523 | 1.00 | 40.49 | O |
| ATOM | 4374 | N | GLN | B | 254 | 7.091 | 2.078 | 71.098 | 1.00 | 32.48 | N |
| ATOM | 4375 | CA | GLN | B | 254 | 7.858 | 1.286 | 72.064 | 1.00 | 32.48 | C |
| ATOM | 4376 | CB | GLN | B | 254 | 7.127 | -0.046 | 72.370 | 1.00 | 40.68 | C |
| ATOM | 4377 | CG | GLN | B | 254 | 7.155 | -1.136 | 71.293 | 1.00 | 40.68 | C |
| ATOM | 4378 | CD | GLN | B | 254 | 6.170 | -2.249 | 71.593 | 1.00 | 40.68 | C |
| ATOM | 4379 | OE1 | GLN | B | 254 | 6.408 | -3.104 | 72.458 | 1.00 | 40.68 | O |
| ATOM | 4380 | NE2 | GLN | B | 254 | 5.042 | -2.235 | 70.889 | 1.00 | 40.68 | N |
| ATOM | 4381 | C | GLN | B | 254 | 9.196 | 1.025 | 71.400 | 1.00 | 32.48 | C |
| ATOM | 4382 | O | GLN | B | 254 | 9.337 | 1.086 | 70.197 | 1.00 | 32.48 | O |
| ATOM | 4383 | N | PRO | B | 255 | 10.199 | 0.751 | 72.181 | 1.00 | 83.88 | N |
| ATOM | 4384 | CD | PRO | B | 255 | 10.084 | 0.679 | 73.632 | 1.00 | 28.67 | C |
| ATOM | 4385 | CA | PRO | B | 255 | 11.551 | 0.467 | 71.719 | 1.00 | 83.88 | C |
| ATOM | 4386 | CB | PRO | B | 255 | 12.264 | 0.113 | 72.988 | 1.00 | 28.67 | C |
| ATOM | 4387 | CG | PRO | B | 255 | 11.452 | 0.816 | 74.011 | 1.00 | 28.67 | C |
| ATOM | 4388 | C | PRO | B | 255 | 11.534 | -0.699 | 70.786 | 1.00 | 83.88 | C |
| ATOM | 4389 | O | PRO | B | 255 | 11.146 | -1.785 | 71.177 | 1.00 | 83.88 | O |
| ATOM | 4390 | N | ILE | B | 256 | 11.935 | -0.464 | 69.550 | 1.00 | 29.28 | N |
| ATOM | 4391 | CA | ILE | B | 256 | 11.968 | -1.504 | 68.517 | 1.00 | 29.28 | C |
| ATOM | 4392 | CB | ILE | B | 256 | 12.508 | -0.894 | 67.200 | 1.00 | 16.86 | C |
| ATOM | 4393 | CG2 | ILE | B | 256 | 13.916 | -0.344 | 67.400 | 1.00 | 16.86 | C |
| ATOM | 4394 | CG1 | ILE | B | 256 | 12.388 | -1.903 | 66.073 | 1.00 | 16.86 | C |
| ATOM | 4395 | CD1 | ILE | B | 256 | 13.393 | -1.715 | 65.044 | 1.00 | 16.86 | C |
| ATOM | 4396 | C | ILE | B | 256 | 12.777 | -2.770 | 68.877 | 1.00 | 29.28 | C |
| ATOM | 4397 | O | ILE | B | 256 | 12.357 | -3.904 | 68.587 | 1.00 | 29.28 | O |
| ATOM | 4398 | N | PHE | B | 257 | 13.935 | -2.568 | 69.500 | 1.00 | 50.32 | N |
| ATOM | 4399 | CA | PHE | B | 257 | 14.819 | -3.673 | 69.905 | 1.00 | 50.32 | C |
| ATOM | 4400 | CB | PHE | B | 257 | 16.123 | -3.597 | 69.132 | 1.00 | 32.91 | C |
| ATOM | 4401 | CG | PHE | B | 257 | 15.935 | -3.668 | 67.652 | 1.00 | 32.91 | C |
| ATOM | 4402 | CD1 | PHE | B | 257 | 15.204 | -4.714 | 67.115 | 1.00 | 32.91 | C |
| ATOM | 4403 | CD2 | PHE | B | 257 | 16.480 | -2.705 | 66.784 | 1.00 | 32.91 | C |
| ATOM | 4404 | CE1 | PHE | B | 257 | 15.013 | -4.822 | 65.759 | 1.00 | 32.91 | C |
| ATOM | 4405 | CE2 | PHE | B | 257 | 16.289 | -2.814 | 65.398 | 1.00 | 32.91 | C |

| | | | | | | | | | | | |
|---|---|---|---|---|---|---|---|---|---|---|---|
| ATOM | 4406 | CZ | PHE | B | 257 | 15.549 | -3.885 | 64.892 | 1.00 | 32.91 | C |
| ATOM | 4407 | C | PHE | B | 257 | 15.092 | -3.616 | 71.399 | 1.00 | 50.32 | C |
| ATOM | 4408 | O | PHE | B | 257 | 16.113 | -3.080 | 71.851 | 1.00 | 50.32 | O |
| ATOM | 4409 | N | PRO | B | 258 | 14.173 | -4.199 | 72.180 | 1.00 | 44.88 | N |
| ATOM | 4410 | CD | PRO | B | 258 | 12.969 | -4.889 | 71.673 | 1.00 | 30.26 | C |
| ATOM | 4411 | CA | PRO | B | 258 | 14.231 | -4.251 | 73.640 | 1.00 | 44.88 | C |
| ATOM | 4412 | CB | PRO | B | 258 | 12.760 | -4.294 | 74.027 | 1.00 | 30.26 | C |
| ATOM | 4413 | CG | PRO | B | 258 | 12.140 | -5.147 | 72.940 | 1.00 | 30.26 | C |
| ATOM | 4414 | C | PRO | B | 258 | 15.032 | -5.359 | 74.289 | 1.00 | 44.88 | C |
| ATOM | 4415 | O | PRO | B | 258 | 14.605 | -5.939 | 75.282 | 1.00 | 44.88 | O |
| ATOM | 4416 | N | GLY | B | 259 | 16.205 | -5.643 | 73.739 | 1.00 | 61.44 | N |
| ATOM | 4417 | CA | GLY | B | 259 | 17.036 | -6.688 | 74.305 | 1.00 | 61.44 | C |
| ATOM | 4418 | C | GLY | B | 259 | 17.416 | -6.403 | 75.754 | 1.00 | 61.44 | C |
| ATOM | 4419 | O | GLY | B | 259 | 17.471 | -5.248 | 76.178 | 1.00 | 61.44 | O |
| ATOM | 4420 | N | ASP | B | 260 | 17.684 | -7.459 | 76.516 | 1.00 | 42.39 | N |
| ATOM | 4421 | CA | ASP | B | 260 | 18.060 | -7.333 | 77.919 | 1.00 | 42.39 | C |
| ATOM | 4422 | CB | ASP | B | 260 | 17.234 | -8.296 | 78.769 | 1.00 | 29.22 | C |
| ATOM | 4423 | CG | ASP | B | 260 | 17.324 | -8.006 | 80.265 | 1.00 | 29.22 | C |
| ATOM | 4424 | OD1 | ASP | B | 260 | 17.640 | -8.940 | 81.038 | 1.00 | 29.22 | O |
| ATOM | 4425 | OD2 | ASP | B | 260 | 17.053 | -6.857 | 80.668 | 1.00 | 29.22 | O |
| ATOM | 4426 | C | ASP | B | 260 | 19.538 | -7.658 | 78.082 | 1.00 | 42.39 | C |
| ATOM | 4427 | O | ASP | B | 260 | 20.090 | -7.586 | 79.185 | 1.00 | 42.39 | O |
| ATOM | 4428 | N | SER | B | 261 | 20.183 | -8.044 | 76.992 | 1.00 | 33.88 | N |
| ATOM | 4429 | CA | SER | B | 261 | 21.593 | -8.355 | 77.075 | 1.00 | 33.88 | C |
| ATOM | 4430 | CB | SER | B | 261 | 21.782 | -9.691 | 77.799 | 1.00 | 32.74 | C |
| ATOM | 4431 | OG | SER | B | 261 | 22.226 | -10.698 | 76.896 | 1.00 | 32.74 | O |
| ATOM | 4432 | C | SER | B | 261 | 22.199 | -8.426 | 75.671 | 1.00 | 33.88 | C |
| ATOM | 4433 | O | SER | B | 261 | 21.471 | -8.520 | 74.695 | 1.00 | 33.88 | O |
| ATOM | 4434 | N | GLY | B | 262 | 23.526 | -8.378 | 75.577 | 1.00 | 43.12 | N |
| ATOM | 4435 | CA | GLY | B | 262 | 24.184 | -8.459 | 74.288 | 1.00 | 43.12 | C |
| ATOM | 4436 | C | GLY | B | 262 | 23.527 | -9.518 | 73.422 | 1.00 | 43.12 | C |
| ATOM | 4437 | O | GLY | B | 262 | 23.551 | -9.440 | 72.198 | 1.00 | 43.12 | O |
| ATOM | 4438 | N | VAL | B | 263 | 22.966 | -10.541 | 74.057 | 1.00 | 37.23 | N |
| ATOM | 4439 | CA | VAL | B | 263 | 22.309 | -11.651 | 73.351 | 1.00 | 37.23 | C |
| ATOM | 4440 | CB | VAL | B | 263 | 22.074 | -12.797 | 74.292 | 1.00 | 39.36 | C |
| ATOM | 4441 | CG1 | VAL | B | 263 | 21.391 | -13.975 | 73.680 | 1.00 | 39.36 | C |
| ATOM | 4442 | CG2 | VAL | B | 263 | 23.311 | -13.310 | 74.560 | 1.00 | 39.36 | C |
| ATOM | 4443 | C | VAL | B | 263 | 20.987 | -11.327 | 72.700 | 1.00 | 37.23 | C |
| ATOM | 4444 | O | VAL | B | 263 | 20.883 | -11.333 | 71.477 | 1.00 | 37.23 | O |
| ATOM | 4445 | N | ASP | B | 264 | 19.972 | -11.063 | 73.510 | 1.00 | 65.81 | N |
| ATOM | 4446 | CA | ASP | B | 264 | 18.639 | -10.779 | 73.012 | 1.00 | 65.81 | C |
| ATOM | 4447 | CB | ASP | B | 264 | 17.706 | -10.576 | 74.192 | 1.00 | 50.02 | C |
| ATOM | 4448 | CG | ASP | B | 264 | 18.163 | -11.340 | 75.419 | 1.00 | 50.02 | C |
| ATOM | 4449 | OD1 | ASP | B | 264 | 19.129 | -12.138 | 75.320 | 1.00 | 50.02 | O |
| ATOM | 4450 | OD2 | ASP | B | 264 | 17.540 | -11.133 | 76.476 | 1.00 | 50.02 | O |
| ATOM | 4451 | C | ASP | B | 264 | 18.722 | -9.543 | 72.170 | 1.00 | 65.81 | C |
| ATOM | 4452 | O | ASP | B | 264 | 18.026 | -9.423 | 71.169 | 1.00 | 65.81 | O |
| ATOM | 4453 | N | GLN | B | 265 | 19.591 | -8.626 | 72.579 | 1.00 | 43.16 | N |
| ATOM | 4454 | CA | GLN | B | 265 | 19.780 | -7.382 | 71.850 | 1.00 | 43.16 | C |
| ATOM | 4455 | CB | GLN | B | 265 | 20.905 | -6.558 | 72.478 | 1.00 | 31.93 | C |
| ATOM | 4456 | CG | GLN | B | 265 | 20.895 | -5.113 | 72.050 | 1.00 | 31.93 | C |
| ATOM | 4457 | CD | GLN | B | 265 | 19.476 | -4.587 | 71.867 | 1.00 | 31.93 | C |
| ATOM | 4458 | OE1 | GLN | B | 265 | 18.630 | -4.688 | 72.772 | 1.00 | 31.93 | O |
| ATOM | 4459 | NE2 | GLN | B | 265 | 19.205 | -4.025 | 70.697 | 1.00 | 31.93 | N |
| ATOM | 4460 | C | GLN | B | 265 | 20.121 | -7.716 | 70.412 | 1.00 | 43.16 | C |
| ATOM | 4461 | O | GLN | B | 265 | 19.821 | -6.969 | 69.491 | 1.00 | 43.16 | O |
| ATOM | 4462 | N | LEU | B | 266 | 20.745 | -8.864 | 70.222 | 1.00 | 36.95 | N |
| ATOM | 4463 | CA | LEU | B | 266 | 21.116 | -9.288 | 68.894 | 1.00 | 36.95 | C |
| ATOM | 4464 | CB | LEU | B | 266 | 22.410 | -10.091 | 68.960 | 1.00 | 43.56 | C |
| ATOM | 4465 | CG | LEU | B | 266 | 22.900 | -10.720 | 67.656 | 1.00 | 43.56 | C |
| ATOM | 4466 | CD1 | LEU | B | 266 | 22.578 | -9.828 | 66.452 | 1.00 | 43.56 | C |
| ATOM | 4467 | CD2 | LEU | B | 266 | 24.387 | -10.966 | 67.775 | 1.00 | 43.56 | C |
| ATOM | 4468 | C | LEU | B | 266 | 19.995 | -10.095 | 68.231 | 1.00 | 36.95 | C |

| | | | | | | | | | | | |
|---|---|---|---|---|---|---|---|---|---|---|---|
| ATOM | 4469 | O | LEU | B | 266 | 19.872 | -10.087 | 67.001 | 1.00 | 36.95 | O |
| ATOM | 4470 | N | ALA | B | 267 | 19.169 | -10.778 | 69.024 | 1.00 | 52.70 | N |
| ATOM | 4471 | CA | ALA | B | 267 | 18.067 | -11.550 | 68.460 | 1.00 | 52.70 | C |
| ATOM | 4472 | CB | ALA | B | 267 | 17.459 | -12.499 | 69.520 | 1.00 | 47.57 | C |
| ATOM | 4473 | C | ALA | B | 267 | 17.053 | -10.529 | 67.999 | 1.00 | 52.70 | C |
| ATOM | 4474 | O | ALA | B | 267 | 16.617 | -10.554 | 66.852 | 1.00 | 52.70 | O |
| ATOM | 4475 | N | GLU | B | 268 | 16.740 | -9.599 | 68.894 | 1.00 | 39.75 | N |
| ATOM | 4476 | CA | GLU | B | 268 | 15.793 | -8.543 | 68.626 | 1.00 | 39.75 | C |
| ATOM | 4477 | CB | GLU | B | 268 | 15.969 | -7.421 | 69.639 | 1.00 | 43.11 | C |
| ATOM | 4478 | CG | GLU | B | 268 | 14.637 | -6.967 | 70.183 | 1.00 | 43.11 | C |
| ATOM | 4479 | CD | GLU | B | 268 | 13.760 | -8.125 | 70.591 | 1.00 | 43.11 | C |
| ATOM | 4480 | OE1 | GLU | B | 268 | 14.160 | -8.828 | 71.544 | 1.00 | 43.11 | O |
| ATOM | 4481 | OE2 | GLU | B | 268 | 12.693 | -8.310 | 69.957 | 1.00 | 43.11 | O |
| ATOM | 4482 | C | GLU | B | 268 | 16.004 | -8.022 | 67.222 | 1.00 | 39.75 | C |
| ATOM | 4483 | O | GLU | B | 268 | 15.053 | -7.949 | 66.437 | 1.00 | 39.75 | O |
| ATOM | 4484 | N | ILE | B | 269 | 17.265 | -7.739 | 66.899 | 1.00 | 34.19 | N |
| ATOM | 4485 | CA | ILE | B | 269 | 17.633 | -7.199 | 65.598 | 1.00 | 34.19 | C |
| ATOM | 4486 | CB | ILE | B | 269 | 19.080 | -6.639 | 65.606 | 1.00 | 25.87 | C |
| ATOM | 4487 | CG2 | ILE | B | 269 | 19.428 | -6.054 | 64.251 | 1.00 | 25.87 | C |
| ATOM | 4488 | CG1 | ILE | B | 269 | 19.191 | -5.535 | 66.664 | 1.00 | 25.87 | C |
| ATOM | 4489 | CD1 | ILE | B | 269 | 20.529 | -4.834 | 66.703 | 1.00 | 25.87 | C |
| ATOM | 4490 | C | ILE | B | 269 | 17.491 | -8.253 | 64.513 | 1.00 | 34.19 | C |
| ATOM | 4491 | O | ILE | B | 269 | 16.948 | -7.980 | 63.440 | 1.00 | 34.19 | O |
| ATOM | 4492 | N | ILE | B | 270 | 17.977 | -9.455 | 64.801 | 1.00 | 48.74 | N |
| ATOM | 4493 | CA | ILE | B | 270 | 17.902 | -10.576 | 63.866 | 1.00 | 48.74 | C |
| ATOM | 4494 | CB | ILE | B | 270 | 18.480 | -11.844 | 64.502 | 1.00 | 40.74 | C |
| ATOM | 4495 | CG2 | ILE | B | 270 | 17.622 | -13.030 | 64.140 | 1.00 | 40.74 | C |
| ATOM | 4496 | CG1 | ILE | B | 270 | 19.934 | -12.029 | 64.069 | 1.00 | 40.74 | C |
| ATOM | 4497 | CD1 | ILE | B | 270 | 20.761 | -12.736 | 65.115 | 1.00 | 40.74 | C |
| ATOM | 4498 | C | ILE | B | 270 | 16.462 | -10.884 | 63.433 | 1.00 | 48.74 | C |
| ATOM | 4499 | O | ILE | B | 270 | 16.170 | -11.014 | 62.242 | 1.00 | 48.74 | O |
| ATOM | 4500 | N | LYS | B | 271 | 15.572 | -11.009 | 64.413 | 1.00 | 47.88 | N |
| ATOM | 4501 | CA | LYS | B | 271 | 14.178 | -11.346 | 64.161 | 1.00 | 47.88 | C |
| ATOM | 4502 | CB | LYS | B | 271 | 13.450 | -11.469 | 65.490 | 1.00 | 53.58 | C |
| ATOM | 4503 | CG | LYS | B | 271 | 13.782 | -12.792 | 66.187 | 1.00 | 53.58 | C |
| ATOM | 4504 | CD | LYS | B | 271 | 12.592 | -13.333 | 66.966 | 1.00 | 53.58 | C |
| ATOM | 4505 | CE | LYS | B | 271 | 11.784 | -14.381 | 66.184 | 1.00 | 53.58 | C |
| ATOM | 4506 | NZ | LYS | B | 271 | 10.379 | -14.541 | 66.716 | 1.00 | 53.58 | N |
| ATOM | 4507 | C | LYS | B | 271 | 13.415 | -10.418 | 63.215 | 1.00 | 47.88 | C |
| ATOM | 4508 | O | LYS | B | 271 | 12.280 | -10.724 | 62.803 | 1.00 | 47.88 | O |
| ATOM | 4509 | N | VAL | B | 272 | 14.062 | -9.318 | 62.843 | 1.00 | 51.66 | N |
| ATOM | 4510 | CA | VAL | B | 272 | 13.485 | -8.309 | 61.964 | 1.00 | 51.66 | C |
| ATOM | 4511 | CB | VAL | B | 272 | 13.346 | -6.942 | 62.663 | 1.00 | 56.86 | C |
| ATOM | 4512 | CG1 | VAL | B | 272 | 14.462 | -6.760 | 63.653 | 1.00 | 56.86 | C |
| ATOM | 4513 | CG2 | VAL | B | 272 | 13.397 | -5.812 | 61.613 | 1.00 | 56.86 | C |
| ATOM | 4514 | C | VAL | B | 272 | 14.414 | -8.124 | 60.806 | 1.00 | 51.66 | C |
| ATOM | 4515 | O | VAL | B | 272 | 14.030 | -8.344 | 59.671 | 1.00 | 51.66 | O |
| ATOM | 4516 | N | LEU | B | 273 | 15.637 | -7.703 | 61.108 | 1.00 | 38.93 | N |
| ATOM | 4517 | CA | LEU | B | 273 | 16.629 | -7.480 | 60.076 | 1.00 | 38.93 | C |
| ATOM | 4518 | CB | LEU | B | 273 | 17.814 | -6.666 | 60.610 | 1.00 | 37.69 | C |
| ATOM | 4519 | CG | LEU | B | 273 | 17.609 | -5.169 | 60.484 | 1.00 | 37.69 | C |
| ATOM | 4520 | CD1 | LEU | B | 273 | 18.897 | -4.457 | 60.768 | 1.00 | 37.69 | C |
| ATOM | 4521 | CD2 | LEU | B | 273 | 17.140 | -4.880 | 59.063 | 1.00 | 37.69 | C |
| ATOM | 4522 | C | LEU | B | 273 | 17.129 | -8.754 | 59.423 | 1.00 | 38.93 | C |
| ATOM | 4523 | O | LEU | B | 273 | 17.693 | -8.691 | 58.332 | 1.00 | 38.93 | O |
| ATOM | 4524 | N | GLY | B | 274 | 16.890 | -9.904 | 60.056 | 1.00 | 49.33 | N |
| ATOM | 4525 | CA | GLY | B | 274 | 17.325 | -11.168 | 59.494 | 1.00 | 49.33 | C |
| ATOM | 4526 | C | GLY | B | 274 | 18.703 | -11.525 | 59.992 | 1.00 | 49.33 | C |
| ATOM | 4527 | O | GLY | B | 274 | 19.216 | -10.926 | 60.933 | 1.00 | 49.33 | O |
| ATOM | 4528 | N | THR | B | 275 | 19.306 | -12.503 | 59.338 | 1.00 | 45.74 | N |
| ATOM | 4529 | CA | THR | B | 275 | 20.630 | -12.979 | 59.694 | 1.00 | 45.74 | C |
| ATOM | 4530 | CB | THR | B | 275 | 20.693 | -14.500 | 59.526 | 1.00 | 55.99 | C |
| ATOM | 4531 | OG1 | THR | B | 275 | 20.102 | -15.102 | 60.681 | 1.00 | 55.99 | O |

| | | | | | | | | | | | |
|---|---|---|---|---|---|---|---|---|---|---|---|
| ATOM | 4532 | CG2 | THR | B | 275 | 22.131 | -14.990 | 59.346 | 1.00 | 55.99 | C |
| ATOM | 4533 | C | THR | B | 275 | 21.813 | -12.336 | 58.961 | 1.00 | 45.74 | C |
| ATOM | 4534 | O | THR | B | 275 | 21.940 | -12.416 | 57.732 | 1.00 | 45.74 | O |
| ATOM | 4535 | N | PRO | B | 276 | 22.712 | -11.708 | 59.733 | 1.00 | 49.46 | N |
| ATOM | 4536 | CD | PRO | B | 276 | 22.662 | -11.710 | 61.208 | 1.00 | 49.54 | C |
| ATOM | 4537 | CA | PRO | B | 276 | 23.917 | -11.031 | 59.249 | 1.00 | 49.46 | C |
| ATOM | 4538 | CB | PRO | B | 276 | 24.696 | -10.761 | 60.534 | 1.00 | 49.54 | C |
| ATOM | 4539 | CG | PRO | B | 276 | 23.621 | -10.614 | 61.566 | 1.00 | 49.54 | C |
| ATOM | 4540 | C | PRO | B | 276 | 24.693 | -11.945 | 58.305 | 1.00 | 49.46 | C |
| ATOM | 4541 | O | PRO | B | 276 | 24.932 | -13.105 | 58.625 | 1.00 | 49.46 | O |
| ATOM | 4542 | N | THR | B | 277 | 25.089 | -11.432 | 57.148 | 1.00 | 54.80 | N |
| ATOM | 4543 | CA | THR | B | 277 | 25.840 | -12.246 | 56.198 | 1.00 | 54.80 | C |
| ATOM | 4544 | CB | THR | B | 277 | 25.739 | -11.692 | 54.781 | 1.00 | 47.56 | C |
| ATOM | 4545 | OG1 | THR | B | 277 | 26.717 | -10.658 | 54.613 | 1.00 | 47.56 | O |
| ATOM | 4546 | CG2 | THR | B | 277 | 24.343 | -11.124 | 54.524 | 1.00 | 47.56 | C |
| ATOM | 4547 | C | THR | B | 277 | 27.317 | -12.243 | 56.582 | 1.00 | 54.80 | C |
| ATOM | 4548 | O | THR | B | 277 | 27.747 | -11.418 | 57.396 | 1.00 | 54.80 | O |
| ATOM | 4549 | N | ARG | B | 278 | 28.092 | -13.156 | 55.997 | 1.00 | 75.77 | N |
| ATOM | 4550 | CA | ARG | B | 278 | 29.522 | -13.231 | 56.290 | 1.00 | 75.77 | C |
| ATOM | 4551 | CB | ARG | B | 278 | 30.231 | -14.235 | 55.366 | 1.00 | 92.31 | C |
| ATOM | 4552 | CG | ARG | B | 278 | 31.579 | -14.724 | 55.921 | 1.00 | 92.31 | C |
| ATOM | 4553 | CD | ARG | B | 278 | 32.709 | -14.846 | 54.887 | 1.00 | 92.31 | C |
| ATOM | 4554 | NE | ARG | B | 278 | 32.523 | -15.929 | 53.922 | 1.00 | 92.31 | N |
| ATOM | 4555 | CZ | ARG | B | 278 | 33.523 | -16.639 | 53.396 | 1.00 | 92.31 | C |
| ATOM | 4556 | NH1 | ARG | B | 278 | 34.783 | -16.389 | 53.747 | 1.00 | 92.31 | N |
| ATOM | 4557 | NH2 | ARG | B | 278 | 33.269 | -17.590 | 52.505 | 1.00 | 92.31 | N |
| ATOM | 4558 | C | ARG | B | 278 | 30.113 | -11.841 | 56.066 | 1.00 | 75.77 | C |
| ATOM | 4559 | O | ARG | B | 278 | 30.599 | -11.200 | 57.002 | 1.00 | 75.77 | O |
| ATOM | 4560 | N | GLU | B | 279 | 30.054 | -11.387 | 54.816 | 1.00 | 73.27 | N |
| ATOM | 4561 | CA | GLU | B | 279 | 30.560 | -10.077 | 54.434 | 1.00 | 73.27 | C |
| ATOM | 4562 | CB | GLU | B | 279 | 30.123 | -9.750 | 53.000 | 1.00101.71 | | C |
| ATOM | 4563 | CG | GLU | B | 279 | 30.504 | -8.354 | 52.508 | 1.00101.71 | | C |
| ATOM | 4564 | CD | GLU | B | 279 | 30.276 | -8.170 | 51.011 | 1.00101.71 | | C |
| ATOM | 4565 | OE1 | GLU | B | 279 | 30.193 | -7.006 | 50.555 | 1.00101.71 | | O |
| ATOM | 4566 | OE2 | GLU | B | 279 | 30.192 | -9.187 | 50.287 | 1.00101.71 | | O |
| ATOM | 4567 | C | GLU | B | 279 | 30.050 | -9.013 | 55.403 | 1.00 | 73.27 | C |
| ATOM | 4568 | O | GLU | B | 279 | 30.816 | -8.159 | 55.857 | 1.00 | 73.27 | O |
| ATOM | 4569 | N | GLN | B | 280 | 28.762 | -9.065 | 55.730 | 1.00 | 71.30 | N |
| ATOM | 4570 | CA | GLN | B | 280 | 28.201 | -8.090 | 56.649 | 1.00 | 71.30 | C |
| ATOM | 4571 | CB | GLN | B | 280 | 26.717 | -8.350 | 56.883 | 1.00 | 41.61 | C |
| ATOM | 4572 | CG | GLN | B | 280 | 25.858 | -7.930 | 55.699 | 1.00 | 41.61 | C |
| ATOM | 4573 | CD | GLN | B | 280 | 24.360 | -7.982 | 55.977 | 1.00 | 41.61 | C |
| ATOM | 4574 | OE1 | GLN | B | 280 | 23.896 | -8.762 | 56.815 | 1.00 | 41.61 | O |
| ATOM | 4575 | NE2 | GLN | B | 280 | 23.592 | -7.160 | 55.254 | 1.00 | 41.61 | N |
| ATOM | 4576 | C | GLN | B | 280 | 28.961 | -8.153 | 57.950 | 1.00 | 71.30 | C |
| ATOM | 4577 | O | GLN | B | 280 | 29.280 | -7.122 | 58.528 | 1.00 | 71.30 | O |
| ATOM | 4578 | N | ILE | B | 281 | 29.261 | -9.359 | 58.412 | 1.00 | 51.52 | N |
| ATOM | 4579 | CA | ILE | B | 281 | 30.019 | -9.489 | 59.643 | 1.00 | 51.52 | C |
| ATOM | 4580 | CB | ILE | B | 281 | 29.964 | -10.937 | 60.171 | 1.00 | 47.30 | C |
| ATOM | 4581 | CG2 | ILE | B | 281 | 30.971 | -11.134 | 61.317 | 1.00 | 47.30 | C |
| ATOM | 4582 | CG1 | ILE | B | 281 | 28.533 | -11.236 | 60.626 | 1.00 | 47.30 | C |
| ATOM | 4583 | CD1 | ILE | B | 281 | 28.380 | -12.510 | 61.426 | 1.00 | 47.30 | C |
| ATOM | 4584 | C | ILE | B | 281 | 31.477 | -9.040 | 59.437 | 1.00 | 51.52 | C |
| ATOM | 4585 | O | ILE | B | 281 | 32.139 | -8.597 | 60.374 | 1.00 | 51.52 | O |
| ATOM | 4586 | N | ALA | B | 282 | 31.974 | -9.139 | 58.211 | 1.00 | 56.64 | N |
| ATOM | 4587 | CA | ALA | B | 282 | 33.339 | -8.711 | 57.941 | 1.00 | 56.64 | C |
| ATOM | 4588 | CB | ALA | B | 282 | 33.793 | -9.216 | 56.569 | 1.00 | 49.86 | C |
| ATOM | 4589 | C | ALA | B | 282 | 33.416 | -7.180 | 57.994 | 1.00 | 56.64 | C |
| ATOM | 4590 | O | ALA | B | 282 | 34.488 | -6.606 | 58.228 | 1.00 | 56.64 | O |
| ATOM | 4591 | N | GLU | B | 283 | 32.280 | -6.518 | 57.788 | 1.00 | 48.02 | N |
| ATOM | 4592 | CA | GLU | B | 283 | 32.254 | -5.059 | 57.801 | 1.00 | 48.02 | C |
| ATOM | 4593 | CB | GLU | B | 283 | 31.364 | -4.549 | 56.674 | 1.00 | 63.40 | C |
| ATOM | 4594 | CG | GLU | B | 283 | 31.933 | -4.870 | 55.310 | 1.00 | 63.40 | C |

| | | | | | | | | | | | |
|---|---|---|---|---|---|---|---|---|---|---|---|
| ATOM | 4595 | CD | GLU | B | 283 | 31.112 | -4.301 | 54.176 | 1.00 | 63.40 | C |
| ATOM | 4596 | OE1 | GLU | B | 283 | 29.914 | -4.649 | 54.081 | 1.00 | 63.40 | O |
| ATOM | 4597 | OE2 | GLU | B | 283 | 31.670 | -3.510 | 53.380 | 1.00 | 63.40 | O |
| ATOM | 4598 | C | GLU | B | 283 | 31.838 | -4.426 | 59.125 | 1.00 | 48.02 | C |
| ATOM | 4599 | O | GLU | B | 283 | 32.027 | -3.226 | 59.326 | 1.00 | 48.02 | O |
| ATOM | 4600 | N | MET | B | 284 | 31.257 | -5.222 | 60.016 | 1.00 | 52.12 | N |
| ATOM | 4601 | CA | MET | B | 284 | 30.863 | -4.732 | 61.333 | 1.00 | 52.12 | C |
| ATOM | 4602 | CB | MET | B | 284 | 29.731 | -5.592 | 61.905 | 1.00 | 50.31 | C |
| ATOM | 4603 | CG | MET | B | 284 | 28.364 | -5.318 | 61.314 | 1.00 | 50.31 | C |
| ATOM | 4604 | SD | MET | B | 284 | 27.179 | -6.584 | 61.740 | 1.00 | 50.31 | S |
| ATOM | 4605 | CE | MET | B | 284 | 27.188 | -6.471 | 63.513 | 1.00 | 50.31 | C |
| ATOM | 4606 | C | MET | B | 284 | 32.118 | -4.875 | 62.202 | 1.00 | 52.12 | C |
| ATOM | 4607 | O | MET | B | 284 | 32.552 | -3.929 | 62.867 | 1.00 | 52.12 | O |
| ATOM | 4608 | N | ASN | B | 285 | 32.685 | -6.079 | 62.179 | 1.00 | 67.72 | N |
| ATOM | 4609 | CA | ASN | B | 285 | 33.902 | -6.413 | 62.909 | 1.00 | 67.72 | C |
| ATOM | 4610 | CB | ASN | B | 285 | 33.566 | -7.021 | 64.272 | 1.00 | 50.00 | C |
| ATOM | 4611 | CG | ASN | B | 285 | 34.804 | -7.419 | 65.043 | 1.00 | 50.00 | C |
| ATOM | 4612 | OD1 | ASN | B | 285 | 35.909 | -7.023 | 64.689 | 1.00 | 50.00 | O |
| ATOM | 4613 | ND2 | ASN | B | 285 | 34.629 | -8.195 | 66.104 | 1.00 | 50.00 | N |
| ATOM | 4614 | C | ASN | B | 285 | 34.676 | -7.420 | 62.054 | 1.00 | 67.72 | C |
| ATOM | 4615 | O | ASN | B | 285 | 34.346 | -8.605 | 62.022 | 1.00 | 67.72 | O |
| ATOM | 4616 | N | PRO | B | 286 | 35.718 | -6.953 | 61.345 | 1.00 | 87.67 | N |
| ATOM | 4617 | CD | PRO | B | 286 | 36.261 | -5.583 | 61.399 | 1.00 | 57.11 | C |
| ATOM | 4618 | CA | PRO | B | 286 | 36.542 | -7.805 | 60.479 | 1.00 | 87.67 | C |
| ATOM | 4619 | CB | PRO | B | 286 | 37.607 | -6.836 | 59.959 | 1.00 | 57.11 | C |
| ATOM | 4620 | CG | PRO | B | 286 | 37.703 | -5.806 | 61.052 | 1.00 | 57.11 | C |
| ATOM | 4621 | C | PRO | B | 286 | 37.147 | -9.042 | 61.140 | 1.00 | 87.67 | C |
| ATOM | 4622 | O | PRO | B | 286 | 37.824 | -9.832 | 60.476 | 1.00 | 87.67 | O |
| ATOM | 4623 | N | ASN | B | 287 | 36.872 | -9.221 | 62.432 | 1.00 | 63.45 | N |
| ATOM | 4624 | CA | ASN | B | 287 | 37.414 | -10.341 | 63.201 | 1.00 | 63.45 | C |
| ATOM | 4625 | CB | ASN | B | 287 | 38.603 | -9.851 | 64.030 | 1.00 | 65.66 | C |
| ATOM | 4626 | CG | ASN | B | 287 | 38.604 | -8.335 | 64.212 | 1.00 | 65.66 | C |
| ATOM | 4627 | OD1 | ASN | B | 287 | 38.698 | -7.582 | 63.241 | 1.00 | 65.66 | O |
| ATOM | 4628 | ND2 | ASN | B | 287 | 38.500 | -7.885 | 65.454 | 1.00 | 65.66 | N |
| ATOM | 4629 | C | ASN | B | 287 | 36.377 | -10.972 | 64.114 | 1.00 | 63.45 | C |
| ATOM | 4630 | O | ASN | B | 287 | 35.430 | -11.591 | 63.648 | 1.00 | 63.45 | O |
| ATOM | 4631 | N | ALA | B | 294 | 20.909 | -19.334 | 63.293 | 1.00 | 71.46 | N |
| ATOM | 4632 | CA | ALA | B | 294 | 21.429 | -19.855 | 62.029 | 1.00 | 71.46 | C |
| ATOM | 4633 | CB | ALA | B | 294 | 21.115 | -21.350 | 61.898 | 1.00 | 40.27 | C |
| ATOM | 4634 | C | ALA | B | 294 | 20.871 | -19.108 | 60.824 | 1.00 | 71.46 | C |
| ATOM | 4635 | O | ALA | B | 294 | 21.330 | -18.020 | 60.511 | 1.00 | 71.46 | O |
| ATOM | 4636 | N | ALA | B | 295 | 19.894 | -19.697 | 60.141 | 1.00 | 74.97 | N |
| ATOM | 4637 | CA | ALA | B | 295 | 19.302 | -19.064 | 58.966 | 1.00 | 74.97 | C |
| ATOM | 4638 | CB | ALA | B | 295 | 19.022 | -20.103 | 57.893 | 1.00 | 56.78 | C |
| ATOM | 4639 | C | ALA | B | 295 | 18.019 | -18.346 | 59.343 | 1.00 | 74.97 | C |
| ATOM | 4640 | O | ALA | B | 295 | 17.062 | -18.967 | 59.805 | 1.00 | 74.97 | O |
| ATOM | 4641 | N | ALA | B | 296 | 17.995 | -17.037 | 59.137 | 1.00 | 58.20 | N |
| ATOM | 4642 | CA | ALA | B | 296 | 16.818 | -16.236 | 59.475 | 1.00 | 58.20 | C |
| ATOM | 4643 | CB | ALA | B | 296 | 17.071 | -15.434 | 60.726 | 1.00 | 29.15 | C |
| ATOM | 4644 | C | ALA | B | 296 | 16.505 | -15.282 | 58.334 | 1.00 | 58.20 | C |
| ATOM | 4645 | O | ALA | B | 296 | 17.365 | -14.458 | 57.987 | 1.00 | 58.20 | O |
| ATOM | 4646 | N | ALA | B | 297 | 15.302 | -15.370 | 57.756 | 1.00 | 55.77 | N |
| ATOM | 4647 | CA | ALA | B | 297 | 14.970 | -14.474 | 56.654 | 1.00 | 55.77 | C |
| ATOM | 4648 | CB | ALA | B | 297 | 13.815 | -15.033 | 55.813 | 1.00 | 47.98 | C |
| ATOM | 4649 | C | ALA | B | 297 | 14.620 | -13.155 | 57.297 | 1.00 | 55.77 | C |
| ATOM | 4650 | O | ALA | B | 297 | 14.169 | -13.127 | 58.435 | 1.00 | 55.77 | O |
| ATOM | 4651 | N | ALA | B | 298 | 14.934 | -12.069 | 56.598 | 1.00 | 59.68 | N |
| ATOM | 4652 | CA | ALA | B | 298 | 14.670 | -10.731 | 57.110 | 1.00 | 59.68 | C |
| ATOM | 4653 | CB | ALA | B | 298 | 15.223 | -9.692 | 56.154 | 1.00 | 30.06 | C |
| ATOM | 4654 | C | ALA | B | 298 | 13.164 | -10.575 | 57.235 | 1.00 | 59.68 | C |
| ATOM | 4655 | O | ALA | B | 298 | 12.413 | -11.341 | 56.650 | 1.00 | 59.68 | O |
| ATOM | 4656 | N | HIS | B | 299 | 12.712 | -9.613 | 58.020 | 1.00 | 60.83 | N |
| ATOM | 4657 | CA | HIS | B | 299 | 11.283 | -9.395 | 58.155 | 1.00 | 60.83 | C |

| | | | | | | | | | | | |
|---|---|---|---|---|---|---|---|---|---|---|---|
| ATOM | 4658 | CB | HIS | B | 299 | 10.868 | -9.371 | 59.620 | 1.00 | 44.31 | C |
| ATOM | 4659 | CG | HIS | B | 299 | 9.392 | -9.482 | 59.825 | 1.00 | 44.31 | C |
| ATOM | 4660 | CD2 | HIS | B | 299 | 8.436 | -8.532 | 59.964 | 1.00 | 44.31 | C |
| ATOM | 4661 | ND1 | HIS | B | 299 | 8.741 | -10.696 | 59.901 | 1.00 | 44.31 | N |
| ATOM | 4662 | CE1 | HIS | B | 299 | 7.450 | -10.489 | 60.084 | 1.00 | 44.31 | C |
| ATOM | 4663 | NE2 | HIS | B | 299 | 7.238 | -9.184 | 60.126 | 1.00 | 44.31 | N |
| ATOM | 4664 | C | HIS | B | 299 | 10.962 | -8.049 | 57.515 | 1.00 | 60.83 | C |
| ATOM | 4665 | O | HIS | B | 299 | 11.469 | -7.013 | 57.940 | 1.00 | 60.83 | O |
| ATOM | 4666 | N | PRO | B | 300 | 10.108 | -8.049 | 56.484 | 1.00 | 59.29 | N |
| ATOM | 4667 | CD | PRO | B | 300 | 9.324 | -9.214 | 56.042 | 1.00 | 65.44 | C |
| ATOM | 4668 | CA | PRO | B | 300 | 9.700 | -6.839 | 55.759 | 1.00 | 59.29 | C |
| ATOM | 4669 | CB | PRO | B | 300 | 8.463 | -7.299 | 54.997 | 1.00 | 65.44 | C |
| ATOM | 4670 | CG | PRO | B | 300 | 8.772 | -8.739 | 54.726 | 1.00 | 65.44 | C |
| ATOM | 4671 | C | PRO | B | 300 | 9.419 | -5.640 | 56.667 | 1.00 | 59.29 | C |
| ATOM | 4672 | O | PRO | B | 300 | 8.617 | -5.716 | 57.598 | 1.00 | 59.29 | O |
| ATOM | 4673 | N | TRP | B | 301 | 10.085 | -4.530 | 56.377 | 1.00 | 41.97 | N |
| ATOM | 4674 | CA | TRP | B | 301 | 9.929 | -3.318 | 57.163 | 1.00 | 41.97 | C |
| ATOM | 4675 | CB | TRP | B | 301 | 10.804 | -2.200 | 56.568 | 1.00 | 30.44 | C |
| ATOM | 4676 | CG | TRP | B | 301 | 12.245 | -2.274 | 57.022 | 1.00 | 30.44 | C |
| ATOM | 4677 | CD2 | TRP | B | 301 | 12.713 | -2.200 | 58.379 | 1.00 | 30.44 | C |
| ATOM | 4678 | CE2 | TRP | B | 301 | 14.131 | -2.309 | 58.330 | 1.00 | 30.44 | C |
| ATOM | 4679 | CE3 | TRP | B | 301 | 12.086 | -1.962 | 59.610 | 1.00 | 30.44 | C |
| ATOM | 4680 | CD1 | TRP | B | 301 | 13.352 | -2.482 | 56.243 | 1.00 | 30.44 | C |
| ATOM | 4681 | NE1 | TRP | B | 301 | 14.487 | -2.517 | 57.031 | 1.00 | 30.44 | N |
| ATOM | 4682 | CZ2 | TRP | B | 301 | 14.914 | -2.303 | 59.494 | 1.00 | 30.44 | C |
| ATOM | 4683 | CZ3 | TRP | B | 301 | 12.874 | -1.947 | 60.770 | 1.00 | 30.44 | C |
| ATOM | 4684 | CH2 | TRP | B | 301 | 14.278 | -2.074 | 60.694 | 1.00 | 30.44 | C |
| ATOM | 4685 | C | TRP | B | 301 | 8.484 | -2.859 | 57.278 | 1.00 | 41.97 | C |
| ATOM | 4686 | O | TRP | B | 301 | 8.061 | -2.341 | 58.321 | 1.00 | 41.97 | O |
| ATOM | 4687 | N | THR | B | 302 | 7.728 | -3.058 | 56.209 | 1.00 | 39.00 | N |
| ATOM | 4688 | CA | THR | B | 302 | 6.340 | -2.638 | 56.185 | 1.00 | 39.00 | C |
| ATOM | 4689 | CB | THR | B | 302 | 5.739 | -2.826 | 54.796 | 1.00 | 43.08 | C |
| ATOM | 4690 | OG1 | THR | B | 302 | 6.788 | -2.847 | 53.818 | 1.00 | 43.08 | O |
| ATOM | 4691 | CG2 | THR | B | 302 | 4.822 | -1.675 | 54.482 | 1.00 | 43.08 | C |
| ATOM | 4692 | C | THR | B | 302 | 5.495 | -3.396 | 57.200 | 1.00 | 39.00 | C |
| ATOM | 4693 | O | THR | B | 302 | 4.589 | -2.823 | 57.815 | 1.00 | 39.00 | O |
| ATOM | 4694 | N | ALA | B | 303 | 5.812 | -4.674 | 57.386 | 1.00 | 39.92 | N |
| ATOM | 4695 | CA | ALA | B | 303 | 5.083 | -5.524 | 58.318 | 1.00 | 39.92 | C |
| ATOM | 4696 | CB | ALA | B | 303 | 5.305 | -6.993 | 57.973 | 1.00 | 61.07 | C |
| ATOM | 4697 | C | ALA | B | 303 | 5.518 | -5.254 | 59.749 | 1.00 | 39.92 | C |
| ATOM | 4698 | O | ALA | B | 303 | 4.850 | -5.666 | 60.702 | 1.00 | 39.92 | O |
| ATOM | 4699 | N | VAL | B | 304 | 6.639 | -4.556 | 59.898 | 1.00 | 37.90 | N |
| ATOM | 4700 | CA | VAL | B | 304 | 7.157 | -4.228 | 61.221 | 1.00 | 37.90 | C |
| ATOM | 4701 | CB | VAL | B | 304 | 8.624 | -3.793 | 61.157 | 1.00 | 43.52 | C |
| ATOM | 4702 | CG1 | VAL | B | 304 | 9.154 | -3.573 | 62.562 | 1.00 | 43.52 | C |
| ATOM | 4703 | CG2 | VAL | B | 304 | 9.447 | -4.837 | 60.422 | 1.00 | 43.52 | C |
| ATOM | 4704 | C | VAL | B | 304 | 6.367 | -3.103 | 61.861 | 1.00 | 37.90 | C |
| ATOM | 4705 | O | VAL | B | 304 | 6.155 | -3.101 | 63.068 | 1.00 | 37.90 | O |
| ATOM | 4706 | N | PHE | B | 305 | 5.921 | -2.151 | 61.048 | 1.00 | 39.18 | N |
| ATOM | 4707 | CA | PHE | B | 305 | 5.171 | -1.009 | 61.567 | 1.00 | 39.18 | C |
| ATOM | 4708 | CB | PHE | B | 305 | 5.679 | 0.284 | 60.932 | 1.00 | 28.88 | C |
| ATOM | 4709 | CG | PHE | B | 305 | 7.115 | 0.565 | 61.223 | 1.00 | 28.88 | C |
| ATOM | 4710 | CD1 | PHE | B | 305 | 7.492 | 1.110 | 62.436 | 1.00 | 28.88 | C |
| ATOM | 4711 | CD2 | PHE | B | 305 | 8.099 | 0.264 | 60.287 | 1.00 | 28.88 | C |
| ATOM | 4712 | CE1 | PHE | B | 305 | 8.833 | 1.354 | 62.720 | 1.00 | 28.88 | C |
| ATOM | 4713 | CE2 | PHE | B | 305 | 9.450 | 0.504 | 60.565 | 1.00 | 28.88 | C |
| ATOM | 4714 | CZ | PHE | B | 305 | 9.814 | 1.047 | 61.778 | 1.00 | 28.88 | C |
| ATOM | 4715 | C | PHE | B | 305 | 3.680 | -1.118 | 61.347 | 1.00 | 39.18 | C |
| ATOM | 4716 | O | PHE | B | 305 | 3.213 | -1.982 | 60.613 | 1.00 | 39.18 | O |
| ATOM | 4717 | N | ARG | B | 306 | 2.939 | -0.218 | 61.980 | 1.00 | 58.92 | N |
| ATOM | 4718 | CA | ARG | B | 306 | 1.494 | -0.228 | 61.866 | 1.00 | 58.92 | C |
| ATOM | 4719 | CB | ARG | B | 306 | 0.898 | 0.738 | 62.893 | 1.00 | 95.92 | C |
| ATOM | 4720 | CG | ARG | B | 306 | 1.400 | 0.406 | 64.308 | 1.00 | 95.92 | C |

| | | | | | | | | | | | |
|---|---|---|---|---|---|---|---|---|---|---|---|
| ATOM | 4721 | CD | ARG | B | 306 | 0.650 | 1.099 | 65.439 | 1.00 | 95.92 | C |
| ATOM | 4722 | NE | ARG | B | 306 | 0.912 | 2.532 | 65.519 | 1.00 | 95.92 | N |
| ATOM | 4723 | CZ | ARG | B | 306 | 0.410 | 3.436 | 64.681 | 1.00 | 95.92 | C |
| ATOM | 4724 | NH1 | ARG | B | 306 | -0.390 | 3.068 | 63.685 | 1.00 | 95.92 | N |
| ATOM | 4725 | NH2 | ARG | B | 306 | 0.703 | 4.717 | 64.845 | 1.00 | 95.92 | N |
| ATOM | 4726 | C | ARG | B | 306 | 1.088 | 0.100 | 60.432 | 1.00 | 58.92 | C |
| ATOM | 4727 | O | ARG | B | 306 | 1.700 | 0.946 | 59.784 | 1.00 | 58.92 | O |
| ATOM | 4728 | N | PRO | B | 307 | 0.054 | -0.591 | 59.920 | 1.00 | 61.29 | N |
| ATOM | 4729 | CD | PRO | B | 307 | -0.888 | -1.272 | 60.821 | 1.00 | 63.64 | C |
| ATOM | 4730 | CA | PRO | B | 307 | -0.529 | -0.489 | 58.575 | 1.00 | 61.29 | C |
| ATOM | 4731 | CB | PRO | B | 307 | -1.871 | -1.203 | 58.729 | 1.00 | 63.64 | C |
| ATOM | 4732 | CG | PRO | B | 307 | -2.203 | -0.974 | 60.160 | 1.00 | 63.64 | C |
| ATOM | 4733 | C | PRO | B | 307 | -0.675 | 0.910 | 57.981 | 1.00 | 61.29 | C |
| ATOM | 4734 | O | PRO | B | 307 | -0.634 | 1.080 | 56.756 | 1.00 | 61.29 | O |
| ATOM | 4735 | N | ALA | B | 308 | -0.840 | 1.911 | 58.838 | 1.00 | 54.69 | N |
| ATOM | 4736 | CA | ALA | B | 308 | -0.999 | 3.277 | 58.360 | 1.00 | 54.69 | C |
| ATOM | 4737 | CB | ALA | B | 308 | -1.804 | 4.082 | 59.369 | 1.00 | 42.92 | C |
| ATOM | 4738 | C | ALA | B | 308 | 0.347 | 3.960 | 58.085 | 1.00 | 54.69 | C |
| ATOM | 4739 | O | ALA | B | 308 | 0.450 | 4.782 | 57.174 | 1.00 | 54.69 | O |
| ATOM | 4740 | N | THR | B | 309 | 1.364 | 3.612 | 58.877 | 1.00 | 50.38 | N |
| ATOM | 4741 | CA | THR | B | 309 | 2.721 | 4.168 | 58.775 | 1.00 | 50.38 | C |
| ATOM | 4742 | CB | THR | B | 309 | 3.768 | 3.134 | 59.245 | 1.00 | 62.72 | C |
| ATOM | 4743 | OG1 | THR | B | 309 | 3.401 | 2.640 | 60.540 | 1.00 | 62.72 | O |
| ATOM | 4744 | CG2 | THR | B | 309 | 5.149 | 3.766 | 59.330 | 1.00 | 62.72 | C |
| ATOM | 4745 | C | THR | B | 309 | 3.128 | 4.662 | 57.381 | 1.00 | 50.38 | C |
| ATOM | 4746 | O | THR | B | 309 | 3.129 | 3.897 | 56.407 | 1.00 | 50.38 | O |
| ATOM | 4747 | N | PRO | B | 310 | 3.505 | 5.951 | 57.281 | 1.00 | 27.06 | N |
| ATOM | 4748 | CD | PRO | B | 310 | 3.597 | 6.926 | 58.380 | 1.00 | 25.15 | C |
| ATOM | 4749 | CA | PRO | B | 310 | 3.915 | 6.570 | 56.020 | 1.00 | 27.06 | C |
| ATOM | 4750 | CB | PRO | B | 310 | 4.264 | 8.007 | 56.426 | 1.00 | 25.15 | C |
| ATOM | 4751 | CG | PRO | B | 310 | 3.435 | 8.245 | 57.641 | 1.00 | 25.15 | C |
| ATOM | 4752 | C | PRO | B | 310 | 5.092 | 5.850 | 55.376 | 1.00 | 27.06 | C |
| ATOM | 4753 | O | PRO | B | 310 | 6.120 | 5.598 | 56.012 | 1.00 | 27.06 | O |
| ATOM | 4754 | N | PRO | B | 311 | 4.947 | 5.499 | 54.098 | 1.00 | 32.68 | N |
| ATOM | 4755 | CD | PRO | B | 311 | 3.720 | 5.653 | 53.300 | 1.00 | 20.54 | C |
| ATOM | 4756 | CA | PRO | B | 311 | 5.977 | 4.805 | 53.329 | 1.00 | 32.68 | C |
| ATOM | 4757 | CB | PRO | B | 311 | 5.418 | 4.856 | 51.916 | 1.00 | 20.54 | C |
| ATOM | 4758 | CG | PRO | B | 311 | 3.962 | 4.690 | 52.170 | 1.00 | 20.54 | C |
| ATOM | 4759 | C | PRO | B | 311 | 7.363 | 5.445 | 53.434 | 1.00 | 32.68 | C |
| ATOM | 4760 | O | PRO | B | 311 | 8.367 | 4.747 | 53.595 | 1.00 | 32.68 | O |
| ATOM | 4761 | N | GLU | B | 312 | 7.413 | 6.771 | 53.347 | 1.00 | 32.51 | N |
| ATOM | 4762 | CA | GLU | B | 312 | 8.686 | 7.490 | 53.426 | 1.00 | 32.51 | C |
| ATOM | 4763 | CB | GLU | B | 312 | 8.472 | 8.993 | 53.192 | 1.00 | 61.45 | C |
| ATOM | 4764 | CG | GLU | B | 312 | 7.635 | 9.354 | 51.968 | 1.00 | 61.45 | C |
| ATOM | 4765 | CD | GLU | B | 312 | 6.148 | 9.322 | 52.253 | 1.00 | 61.45 | C |
| ATOM | 4766 | OE1 | GLU | B | 312 | 5.380 | 9.817 | 51.400 | 1.00 | 61.45 | O |
| ATOM | 4767 | OE2 | GLU | B | 312 | 5.748 | 8.807 | 53.322 | 1.00 | 61.45 | O |
| ATOM | 4768 | C | GLU | B | 312 | 9.385 | 7.273 | 54.777 | 1.00 | 32.51 | C |
| ATOM | 4769 | O | GLU | B | 312 | 10.615 | 7.203 | 54.863 | 1.00 | 32.51 | O |
| ATOM | 4770 | N | ALA | B | 313 | 8.588 | 7.169 | 55.833 | 1.00 | 40.51 | N |
| ATOM | 4771 | CA | ALA | B | 313 | 9.119 | 6.944 | 57.168 | 1.00 | 40.51 | C |
| ATOM | 4772 | CB | ALA | B | 313 | 7.995 | 6.962 | 58.159 | 1.00 | 19.43 | C |
| ATOM | 4773 | C | ALA | B | 313 | 9.818 | 5.591 | 57.182 | 1.00 | 40.51 | C |
| ATOM | 4774 | O | ALA | B | 313 | 10.972 | 5.474 | 57.587 | 1.00 | 40.51 | O |
| ATOM | 4775 | N | ILE | B | 314 | 9.102 | 4.571 | 56.729 | 1.00 | 38.19 | N |
| ATOM | 4776 | CA | ILE | B | 314 | 9.638 | 3.219 | 56.652 | 1.00 | 38.19 | C |
| ATOM | 4777 | CB | ILE | B | 314 | 8.606 | 2.281 | 56.024 | 1.00 | 39.09 | C |
| ATOM | 4778 | CG2 | ILE | B | 314 | 9.062 | 0.845 | 56.154 | 1.00 | 39.09 | C |
| ATOM | 4779 | CG1 | ILE | B | 314 | 7.264 | 2.463 | 56.734 | 1.00 | 39.09 | C |
| ATOM | 4780 | CD1 | ILE | B | 314 | 6.112 | 1.785 | 56.049 | 1.00 | 39.09 | C |
| ATOM | 4781 | C | ILE | B | 314 | 10.909 | 3.205 | 55.801 | 1.00 | 38.19 | C |
| ATOM | 4782 | O | ILE | B | 314 | 11.924 | 2.626 | 56.178 | 1.00 | 38.19 | O |
| ATOM | 4783 | N | ALA | B | 315 | 10.845 | 3.864 | 54.650 | 1.00 | 27.52 | N |

| | | | | | | | | | | | |
|---|---|---|---|---|---|---|---|---|---|---|---|
| ATOM | 4784 | CA | ALA | B | 315 | 11.983 | 3.931 | 53.747 | 1.00 | 27.52 | C |
| ATOM | 4785 | CB | ALA | B | 315 | 11.623 | 4.758 | 52.535 | 1.00 | 39.25 | C |
| ATOM | 4786 | C | ALA | B | 315 | 13.228 | 4.503 | 54.418 | 1.00 | 27.52 | C |
| ATOM | 4787 | O | ALA | B | 315 | 14.290 | 3.893 | 54.363 | 1.00 | 27.52 | O |
| ATOM | 4788 | N | LEU | B | 316 | 13.100 | 5.670 | 55.049 | 1.00 | 26.07 | N |
| ATOM | 4789 | CA | LEU | B | 316 | 14.243 | 6.299 | 55.702 | 1.00 | 26.07 | C |
| ATOM | 4790 | CB | LEU | B | 316 | 13.820 | 7.562 | 56.453 | 1.00 | 23.58 | C |
| ATOM | 4791 | CG | LEU | B | 316 | 14.885 | 8.157 | 57.377 | 1.00 | 23.58 | C |
| ATOM | 4792 | CD1 | LEU | B | 316 | 16.100 | 8.596 | 56.586 | 1.00 | 23.58 | C |
| ATOM | 4793 | CD2 | LEU | B | 316 | 14.281 | 9.325 | 58.143 | 1.00 | 23.58 | C |
| ATOM | 4794 | C | LEU | B | 316 | 14.822 | 5.310 | 56.672 | 1.00 | 26.07 | C |
| ATOM | 4795 | O | LEU | B | 316 | 16.008 | 5.001 | 56.642 | 1.00 | 26.07 | O |
| ATOM | 4796 | N | CYS | B | 317 | 13.952 | 4.800 | 57.527 | 1.00 | 34.50 | N |
| ATOM | 4797 | CA | CYS | B | 317 | 14.334 | 3.827 | 58.531 | 1.00 | 34.50 | C |
| ATOM | 4798 | CB | CYS | B | 317 | 13.067 | 3.270 | 59.161 | 1.00 | 45.62 | C |
| ATOM | 4799 | SG | CYS | B | 317 | 13.346 | 2.371 | 60.641 | 1.00 | 45.62 | S |
| ATOM | 4800 | C | CYS | B | 317 | 15.189 | 2.690 | 57.949 | 1.00 | 34.50 | C |
| ATOM | 4801 | O | CYS | B | 317 | 16.157 | 2.255 | 58.565 | 1.00 | 34.50 | O |
| ATOM | 4802 | N | SER | B | 318 | 14.825 | 2.222 | 56.758 | 1.00 | 28.95 | N |
| ATOM | 4803 | CA | SER | B | 318 | 15.540 | 1.144 | 56.099 | 1.00 | 28.95 | C |
| ATOM | 4804 | CB | SER | B | 318 | 14.854 | 0.801 | 54.782 | 1.00 | 29.18 | C |
| ATOM | 4805 | OG | SER | B | 318 | 15.387 | 1.588 | 53.733 | 1.00 | 29.18 | O |
| ATOM | 4806 | C | SER | B | 318 | 17.001 | 1.531 | 55.819 | 1.00 | 28.95 | C |
| ATOM | 4807 | O | SER | B | 318 | 17.900 | 0.681 | 55.850 | 1.00 | 28.95 | O |
| ATOM | 4808 | N | ARG | B | 319 | 17.238 | 2.814 | 55.549 | 1.00 | 32.46 | N |
| ATOM | 4809 | CA | ARG | B | 319 | 18.588 | 3.297 | 55.239 | 1.00 | 32.46 | C |
| ATOM | 4810 | CB | ARG | B | 319 | 18.522 | 4.529 | 54.341 | 1.00 | 47.50 | C |
| ATOM | 4811 | CG | ARG | B | 319 | 17.559 | 4.421 | 53.162 | 1.00 | 47.50 | C |
| ATOM | 4812 | CD | ARG | B | 319 | 17.981 | 3.389 | 52.114 | 1.00 | 47.50 | C |
| ATOM | 4813 | NE | ARG | B | 319 | 19.270 | 3.673 | 51.478 | 1.00 | 47.50 | N |
| ATOM | 4814 | CZ | ARG | B | 319 | 20.427 | 3.186 | 51.911 | 1.00 | 47.50 | C |
| ATOM | 4815 | NH1 | ARG | B | 319 | 20.446 | 2.397 | 52.974 | 1.00 | 47.50 | N |
| ATOM | 4816 | NH2 | ARG | B | 319 | 21.561 | 3.473 | 51.281 | 1.00 | 47.50 | N |
| ATOM | 4817 | C | ARG | B | 319 | 19.396 | 3.646 | 56.487 | 1.00 | 32.46 | C |
| ATOM | 4818 | O | ARG | B | 319 | 20.549 | 4.081 | 56.392 | 1.00 | 32.46 | O |
| ATOM | 4819 | N | LEU | B | 320 | 18.782 | 3.457 | 57.653 | 1.00 | 30.92 | N |
| ATOM | 4820 | CA | LEU | B | 320 | 19.429 | 3.740 | 58.930 | 1.00 | 30.92 | C |
| ATOM | 4821 | CB | LEU | B | 320 | 18.514 | 4.630 | 59.772 | 1.00 | 25.28 | C |
| ATOM | 4822 | CG | LEU | B | 320 | 18.288 | 6.016 | 59.165 | 1.00 | 25.28 | C |
| ATOM | 4823 | CD1 | LEU | B | 320 | 17.181 | 6.719 | 59.901 | 1.00 | 25.28 | C |
| ATOM | 4824 | CD2 | LEU | B | 320 | 19.554 | 6.838 | 59.247 | 1.00 | 25.28 | C |
| ATOM | 4825 | C | LEU | B | 320 | 19.765 | 2.448 | 59.689 | 1.00 | 30.92 | C |
| ATOM | 4826 | O | LEU | B | 320 | 20.856 | 2.280 | 60.233 | 1.00 | 30.92 | O |
| ATOM | 4827 | N | LEU | B | 321 | 18.818 | 1.528 | 59.718 | 1.00 | 39.82 | N |
| ATOM | 4828 | CA | LEU | B | 321 | 19.046 | 0.283 | 60.412 | 1.00 | 39.82 | C |
| ATOM | 4829 | CB | LEU | B | 321 | 17.742 | -0.207 | 61.027 | 1.00 | 29.49 | C |
| ATOM | 4830 | CG | LEU | B | 321 | 17.120 | 0.772 | 62.022 | 1.00 | 29.49 | C |
| ATOM | 4831 | CD1 | LEU | B | 321 | 15.921 | 0.112 | 62.673 | 1.00 | 29.49 | C |
| ATOM | 4832 | CD2 | LEU | B | 321 | 18.150 | 1.193 | 63.075 | 1.00 | 29.49 | C |
| ATOM | 4833 | C | LEU | B | 321 | 19.612 | -0.751 | 59.454 | 1.00 | 39.82 | C |
| ATOM | 4834 | O | LEU | B | 321 | 18.872 | -1.493 | 58.808 | 1.00 | 39.82 | O |
| ATOM | 4835 | N | GLU | B | 322 | 20.935 | -0.786 | 59.360 | 1.00 | 34.98 | N |
| ATOM | 4836 | CA | GLU | B | 322 | 21.607 | -1.723 | 58.481 | 1.00 | 34.98 | C |
| ATOM | 4837 | CB | GLU | B | 322 | 22.047 | -1.020 | 57.202 | 1.00 | 42.89 | C |
| ATOM | 4838 | CG | GLU | B | 322 | 20.965 | -0.222 | 56.529 | 1.00 | 42.89 | C |
| ATOM | 4839 | CD | GLU | B | 322 | 21.267 | 0.003 | 55.065 | 1.00 | 42.89 | C |
| ATOM | 4840 | OE1 | GLU | B | 322 | 21.043 | -0.920 | 54.261 | 1.00 | 42.89 | O |
| ATOM | 4841 | OE2 | GLU | B | 322 | 21.749 | 1.093 | 54.709 | 1.00 | 42.89 | O |
| ATOM | 4842 | C | GLU | B | 322 | 22.824 | -2.303 | 59.180 | 1.00 | 34.98 | C |
| ATOM | 4843 | O | GLU | B | 322 | 23.411 | -1.659 | 60.049 | 1.00 | 34.98 | O |
| ATOM | 4844 | N | TYR | B | 323 | 23.199 | -3.518 | 58.793 | 1.00 | 23.84 | N |
| ATOM | 4845 | CA | TYR | B | 323 | 24.358 | -4.166 | 59.385 | 1.00 | 23.84 | C |
| ATOM | 4846 | CB | TYR | B | 323 | 24.390 | -5.655 | 59.040 | 1.00 | 52.21 | C |

| | | | | | | | | | | | |
|---|---|---|---|---|---|---|---|---|---|---|---|
| ATOM | 4847 | CG | TYR | B | 323 | 23.405 | -6.473 | 59.830 | 1.00 | 52.21 | C |
| ATOM | 4848 | CD1 | TYR | B | 323 | 23.432 | -6.465 | 61.215 | 1.00 | 52.21 | C |
| ATOM | 4849 | CE1 | TYR | B | 323 | 22.524 | -7.193 | 61.944 | 1.00 | 52.21 | C |
| ATOM | 4850 | CD2 | TYR | B | 323 | 22.432 | -7.241 | 59.190 | 1.00 | 52.21 | C |
| ATOM | 4851 | CE2 | TYR | B | 323 | 21.517 | -7.974 | 59.910 | 1.00 | 52.21 | C |
| ATOM | 4852 | CZ | TYR | B | 323 | 21.567 | -7.946 | 61.286 | 1.00 | 52.21 | C |
| ATOM | 4853 | OH | TYR | B | 323 | 20.657 | -8.683 | 61.999 | 1.00 | 52.21 | O |
| ATOM | 4854 | C | TYR | B | 323 | 25.641 | -3.501 | 58.925 | 1.00 | 23.84 | C |
| ATOM | 4855 | O | TYR | B | 323 | 26.473 | -3.143 | 59.749 | 1.00 | 23.84 | O |
| ATOM | 4856 | N | THR | B | 324 | 25.820 | -3.339 | 57.619 | 1.00 | 43.22 | N |
| ATOM | 4857 | CA | THR | B | 324 | 27.028 | -2.679 | 57.130 | 1.00 | 43.22 | C |
| ATOM | 4858 | CB | THR | B | 324 | 27.122 | -2.724 | 55.578 | 1.00 | 41.65 | C |
| ATOM | 4859 | OG1 | THR | B | 324 | 27.051 | -4.089 | 55.136 | 1.00 | 41.65 | O |
| ATOM | 4860 | CG2 | THR | B | 324 | 28.443 | -2.113 | 55.103 | 1.00 | 41.65 | C |
| ATOM | 4861 | C | THR | B | 324 | 26.935 | -1.228 | 57.620 | 1.00 | 43.22 | C |
| ATOM | 4862 | O | THR | B | 324 | 26.017 | -0.486 | 57.258 | 1.00 | 43.22 | O |
| ATOM | 4863 | N | PRO | B | 325 | 27.884 | -0.806 | 58.464 | 1.00 | 39.69 | N |
| ATOM | 4864 | CD | PRO | B | 325 | 29.012 | -1.548 | 59.045 | 1.00 | 19.04 | C |
| ATOM | 4865 | CA | PRO | B | 325 | 27.840 | 0.565 | 58.977 | 1.00 | 39.69 | C |
| ATOM | 4866 | CB | PRO | B | 325 | 29.048 | 0.623 | 59.904 | 1.00 | 19.04 | C |
| ATOM | 4867 | CG | PRO | B | 325 | 29.201 | -0.805 | 60.345 | 1.00 | 19.04 | C |
| ATOM | 4868 | C | PRO | B | 325 | 27.890 | 1.633 | 57.896 | 1.00 | 39.69 | C |
| ATOM | 4869 | O | PRO | B | 325 | 27.153 | 2.622 | 57.951 | 1.00 | 39.69 | O |
| ATOM | 4870 | N | THR | B | 326 | 28.762 | 1.424 | 56.913 | 1.00 | 32.90 | N |
| ATOM | 4871 | CA | THR | B | 326 | 28.943 | 2.364 | 55.809 | 1.00 | 32.90 | C |
| ATOM | 4872 | CB | THR | B | 326 | 30.147 | 1.951 | 54.967 | 1.00 | 35.11 | C |
| ATOM | 4873 | OG1 | THR | B | 326 | 29.886 | 0.677 | 54.357 | 1.00 | 35.11 | O |
| ATOM | 4874 | CG2 | THR | B | 326 | 31.386 | 1.849 | 55.853 | 1.00 | 35.11 | C |
| ATOM | 4875 | C | THR | B | 326 | 27.724 | 2.484 | 54.889 | 1.00 | 32.90 | C |
| ATOM | 4876 | O | THR | B | 326 | 27.561 | 3.466 | 54.163 | 1.00 | 32.90 | O |
| ATOM | 4877 | N | ALA | B | 327 | 26.869 | 1.472 | 54.924 | 1.00 | 31.75 | N |
| ATOM | 4878 | CA | ALA | B | 327 | 25.670 | 1.451 | 54.093 | 1.00 | 31.75 | C |
| ATOM | 4879 | CB | ALA | B | 327 | 25.066 | 0.051 | 54.103 | 1.00 | 22.22 | C |
| ATOM | 4880 | C | ALA | B | 327 | 24.640 | 2.474 | 54.559 | 1.00 | 31.75 | C |
| ATOM | 4881 | O | ALA | B | 327 | 23.814 | 2.940 | 53.775 | 1.00 | 31.75 | O |
| ATOM | 4882 | N | ARG | B | 328 | 24.713 | 2.815 | 55.841 | 1.00 | 18.46 | N |
| ATOM | 4883 | CA | ARG | B | 328 | 23.825 | 3.779 | 56.473 | 1.00 | 18.46 | C |
| ATOM | 4884 | CB | ARG | B | 328 | 24.119 | 3.868 | 57.968 | 1.00 | 38.50 | C |
| ATOM | 4885 | CG | ARG | B | 328 | 23.739 | 2.645 | 58.740 | 1.00 | 38.50 | C |
| ATOM | 4886 | CD | ARG | B | 328 | 24.496 | 2.547 | 60.052 | 1.00 | 38.50 | C |
| ATOM | 4887 | NE | ARG | B | 328 | 24.409 | 1.189 | 60.583 | 1.00 | 38.50 | N |
| ATOM | 4888 | CZ | ARG | B | 328 | 25.180 | 0.693 | 61.540 | 1.00 | 38.50 | C |
| ATOM | 4889 | NH1 | ARG | B | 328 | 26.126 | 1.420 | 62.115 | 1.00 | 38.50 | N |
| ATOM | 4890 | NH2 | ARG | B | 328 | 25.004 | -0.555 | 61.910 | 1.00 | 38.50 | N |
| ATOM | 4891 | C | ARG | B | 328 | 23.955 | 5.171 | 55.892 | 1.00 | 18.46 | C |
| ATOM | 4892 | O | ARG | B | 328 | 24.978 | 5.554 | 55.324 | 1.00 | 18.46 | O |
| ATOM | 4893 | N | LEU | B | 329 | 22.901 | 5.947 | 56.050 | 1.00 | 28.73 | N |
| ATOM | 4894 | CA | LEU | B | 329 | 22.936 | 7.297 | 55.556 | 1.00 | 28.73 | C |
| ATOM | 4895 | CB | LEU | B | 329 | 21.532 | 7.901 | 55.566 | 1.00 | 28.39 | C |
| ATOM | 4896 | CG | LEU | B | 329 | 20.542 | 7.178 | 54.656 | 1.00 | 28.39 | C |
| ATOM | 4897 | CD1 | LEU | B | 329 | 19.164 | 7.845 | 54.731 | 1.00 | 28.39 | C |
| ATOM | 4898 | CD2 | LEU | B | 329 | 21.083 | 7.194 | 53.242 | 1.00 | 28.39 | C |
| ATOM | 4899 | C | LEU | B | 329 | 23.844 | 8.088 | 56.480 | 1.00 | 28.73 | C |
| ATOM | 4900 | O | LEU | B | 329 | 24.314 | 7.580 | 57.505 | 1.00 | 28.73 | O |
| ATOM | 4901 | N | THR | B | 330 | 24.109 | 9.327 | 56.086 | 1.00 | 27.92 | N |
| ATOM | 4902 | CA | THR | B | 330 | 24.910 | 10.233 | 56.887 | 1.00 | 27.92 | C |
| ATOM | 4903 | CB | THR | B | 330 | 25.967 | 10.985 | 56.048 | 1.00 | 35.01 | C |
| ATOM | 4904 | OG1 | THR | B | 330 | 25.320 | 11.961 | 55.213 | 1.00 | 35.01 | O |
| ATOM | 4905 | CG2 | THR | B | 330 | 26.745 | 10.020 | 55.192 | 1.00 | 35.01 | C |
| ATOM | 4906 | C | THR | B | 330 | 23.902 | 11.252 | 57.413 | 1.00 | 27.92 | C |
| ATOM | 4907 | O | THR | B | 330 | 22.889 | 11.537 | 56.759 | 1.00 | 27.92 | O |
| ATOM | 4908 | N | PRO | B | 331 | 24.161 | 11.808 | 58.603 | 1.00 | 32.26 | N |
| ATOM | 4909 | CD | PRO | B | 331 | 25.326 | 11.606 | 59.488 | 1.00 | 48.20 | C |

| | | | | | | | | | | | |
|---|---|---|---|---|---|---|---|---|---|---|---|
| ATOM | 4910 | CA | PRO | B | 331 | 23.240 | 12.795 | 59.168 | 1.00 | 32.26 | C |
| ATOM | 4911 | CB | PRO | B | 331 | 24.102 | 13.492 | 60.219 | 1.00 | 48.20 | C |
| ATOM | 4912 | CG | PRO | B | 331 | 24.913 | 12.350 | 60.760 | 1.00 | 48.20 | C |
| ATOM | 4913 | C | PRO | B | 331 | 22.680 | 13.764 | 58.122 | 1.00 | 32.26 | C |
| ATOM | 4914 | O | PRO | B | 331 | 21.467 | 13.989 | 58.063 | 1.00 | 32.26 | O |
| ATOM | 4915 | N | LEU | B | 332 | 23.566 | 14.332 | 57.299 | 1.00 | 31.38 | N |
| ATOM | 4916 | CA | LEU | B | 332 | 23.152 | 15.281 | 56.264 | 1.00 | 31.38 | C |
| ATOM | 4917 | CB | LEU | B | 332 | 24.372 | 15.849 | 55.534 | 1.00 | 44.06 | C |
| ATOM | 4918 | CG | LEU | B | 332 | 24.845 | 17.232 | 56.003 | 1.00 | 44.06 | C |
| ATOM | 4919 | CD1 | LEU | B | 332 | 26.107 | 17.621 | 55.240 | 1.00 | 44.06 | C |
| ATOM | 4920 | CD2 | LEU | B | 332 | 23.741 | 18.273 | 55.797 | 1.00 | 44.06 | C |
| ATOM | 4921 | C | LEU | B | 332 | 22.195 | 14.669 | 55.253 | 1.00 | 31.38 | C |
| ATOM | 4922 | O | LEU | B | 332 | 21.186 | 15.284 | 54.900 | 1.00 | 31.38 | O |
| ATOM | 4923 | N | GLU | B | 333 | 22.522 | 13.461 | 54.792 | 1.00 | 22.08 | N |
| ATOM | 4924 | CA | GLU | B | 333 | 21.701 | 12.746 | 53.821 | 1.00 | 22.08 | C |
| ATOM | 4925 | CB | GLU | B | 333 | 22.407 | 11.449 | 53.432 | 1.00 | 42.09 | C |
| ATOM | 4926 | CG | GLU | B | 333 | 23.713 | 11.673 | 52.686 | 1.00 | 42.09 | C |
| ATOM | 4927 | CD | GLU | B | 333 | 24.553 | 10.402 | 52.568 | 1.00 | 42.09 | C |
| ATOM | 4928 | OE1 | GLU | B | 333 | 23.984 | 9.302 | 52.723 | 1.00 | 42.09 | O |
| ATOM | 4929 | OE2 | GLU | B | 333 | 25.779 | 10.495 | 52.313 | 1.00 | 42.09 | O |
| ATOM | 4930 | C | GLU | B | 333 | 20.303 | 12.458 | 54.388 | 1.00 | 22.08 | C |
| ATOM | 4931 | O | GLU | B | 333 | 19.282 | 12.599 | 53.697 | 1.00 | 22.08 | O |
| ATOM | 4932 | N | ALA | B | 334 | 20.271 | 12.065 | 55.657 | 1.00 | 27.55 | N |
| ATOM | 4933 | CA | ALA | B | 334 | 19.024 | 11.775 | 56.339 | 1.00 | 27.55 | C |
| ATOM | 4934 | CB | ALA | B | 334 | 19.304 | 11.333 | 57.758 | 1.00 | 33.37 | C |
| ATOM | 4935 | C | ALA | B | 334 | 18.178 | 13.036 | 56.345 | 1.00 | 27.55 | C |
| ATOM | 4936 | O | ALA | B | 334 | 16.965 | 12.990 | 56.162 | 1.00 | 27.55 | O |
| ATOM | 4937 | N | CYS | B | 335 | 18.824 | 14.172 | 56.553 | 1.00 | 35.72 | N |
| ATOM | 4938 | CA | CYS | B | 335 | 18.113 | 15.442 | 56.576 | 1.00 | 35.72 | C |
| ATOM | 4939 | CB | CYS | B | 335 | 19.058 | 16.569 | 56.992 | 1.00 | 27.72 | C |
| ATOM | 4940 | SG | CYS | B | 335 | 19.451 | 16.624 | 58.730 | 1.00 | 27.72 | S |
| ATOM | 4941 | C | CYS | B | 335 | 17.495 | 15.802 | 55.229 | 1.00 | 35.72 | C |
| ATOM | 4942 | O | CYS | B | 335 | 16.531 | 16.556 | 55.164 | 1.00 | 35.72 | O |
| ATOM | 4943 | N | ALA | B | 336 | 18.062 | 15.280 | 54.153 | 1.00 | 43.40 | N |
| ATOM | 4944 | CA | ALA | B | 336 | 17.552 | 15.586 | 52.827 | 1.00 | 43.40 | C |
| ATOM | 4945 | CB | ALA | B | 336 | 18.718 | 15.734 | 51.848 | 1.00 | 17.69 | C |
| ATOM | 4946 | C | ALA | B | 336 | 16.575 | 14.519 | 52.332 | 1.00 | 43.40 | C |
| ATOM | 4947 | O | ALA | B | 336 | 16.103 | 14.577 | 51.194 | 1.00 | 43.40 | O |
| ATOM | 4948 | N | HIS | B | 337 | 16.266 | 13.552 | 53.190 | 1.00 | 32.55 | N |
| ATOM | 4949 | CA | HIS | B | 337 | 15.360 | 12.479 | 52.815 | 1.00 | 32.55 | C |
| ATOM | 4950 | CB | HIS | B | 337 | 15.342 | 11.395 | 53.887 | 1.00 | 33.66 | C |
| ATOM | 4951 | CG | HIS | B | 337 | 14.514 | 10.204 | 53.516 | 1.00 | 33.66 | C |
| ATOM | 4952 | CD2 | HIS | B | 337 | 13.179 | 9.991 | 53.592 | 1.00 | 33.66 | C |
| ATOM | 4953 | ND1 | HIS | B | 337 | 15.051 | 9.077 | 52.932 | 1.00 | 33.66 | N |
| ATOM | 4954 | CE1 | HIS | B | 337 | 14.081 | 8.223 | 52.663 | 1.00 | 33.66 | C |
| ATOM | 4955 | NE2 | HIS | B | 337 | 12.934 | 8.753 | 53.051 | 1.00 | 33.66 | N |
| ATOM | 4956 | C | HIS | B | 337 | 13.951 | 12.993 | 52.608 | 1.00 | 32.55 | C |
| ATOM | 4957 | O | HIS | B | 337 | 13.513 | 13.926 | 53.281 | 1.00 | 32.55 | O |
| ATOM | 4958 | N | SER | B | 338 | 13.234 | 12.362 | 51.688 | 1.00 | 31.72 | N |
| ATOM | 4959 | CA | SER | B | 338 | 11.863 | 12.755 | 51.390 | 1.00 | 31.72 | C |
| ATOM | 4960 | CB | SER | B | 338 | 11.346 | 11.948 | 50.216 | 1.00 | 31.21 | C |
| ATOM | 4961 | OG | SER | B | 338 | 11.343 | 10.574 | 50.557 | 1.00 | 31.21 | O |
| ATOM | 4962 | C | SER | B | 338 | 10.879 | 12.616 | 52.556 | 1.00 | 31.72 | C |
| ATOM | 4963 | O | SER | B | 338 | 9.699 | 12.915 | 52.397 | 1.00 | 31.72 | O |
| ATOM | 4964 | N | PHE | B | 339 | 11.333 | 12.150 | 53.717 | 1.00 | 27.70 | N |
| ATOM | 4965 | CA | PHE | B | 339 | 10.420 | 12.032 | 54.858 | 1.00 | 27.70 | C |
| ATOM | 4966 | CB | PHE | B | 339 | 10.962 | 11.062 | 55.910 | 1.00 | 25.70 | C |
| ATOM | 4967 | CG | PHE | B | 339 | 10.144 | 11.023 | 57.178 | 1.00 | 25.70 | C |
| ATOM | 4968 | CD1 | PHE | B | 339 | 8.783 | 10.742 | 57.137 | 1.00 | 25.70 | C |
| ATOM | 4969 | CD2 | PHE | B | 339 | 10.746 | 11.241 | 58.419 | 1.00 | 25.70 | C |
| ATOM | 4970 | CE1 | PHE | B | 339 | 8.034 | 10.673 | 58.316 | 1.00 | 25.70 | C |
| ATOM | 4971 | CE2 | PHE | B | 339 | 10.007 | 11.175 | 59.604 | 1.00 | 25.70 | C |
| ATOM | 4972 | CZ | PHE | B | 339 | 8.650 | 10.889 | 59.552 | 1.00 | 25.70 | C |

| | | | | | | | | | | | |
|---|---|---|---|---|---|---|---|---|---|---|---|
| ATOM | 4973 | C | PHE | B | 339 | 10.277 | 13.410 | 55.473 | 1.00 | 27.70 | C |
| ATOM | 4974 | O | PHE | B | 339 | 9.272 | 13.742 | 56.106 | 1.00 | 27.70 | O |
| ATOM | 4975 | N | PHE | B | 340 | 11.311 | 14.211 | 55.261 | 1.00 | 52.41 | N |
| ATOM | 4976 | CA | PHE | B | 340 | 11.346 | 15.562 | 55.770 | 1.00 | 52.41 | C |
| ATOM | 4977 | CB | PHE | B | 340 | 12.727 | 15.851 | 56.353 | 1.00 | 37.23 | C |
| ATOM | 4978 | CG | PHE | B | 340 | 13.122 | 14.918 | 57.466 | 1.00 | 37.23 | C |
| ATOM | 4979 | CD1 | PHE | B | 340 | 12.358 | 14.825 | 58.627 | 1.00 | 37.23 | C |
| ATOM | 4980 | CD2 | PHE | B | 340 | 14.252 | 14.117 | 57.343 | 1.00 | 37.23 | C |
| ATOM | 4981 | CE1 | PHE | B | 340 | 12.719 | 13.947 | 59.641 | 1.00 | 37.23 | C |
| ATOM | 4982 | CE2 | PHE | B | 340 | 14.619 | 13.237 | 58.351 | 1.00 | 37.23 | C |
| ATOM | 4983 | CZ | PHE | B | 340 | 13.853 | 13.151 | 59.499 | 1.00 | 37.23 | C |
| ATOM | 4984 | C | PHE | B | 340 | 11.011 | 16.582 | 54.684 | 1.00 | 52.41 | C |
| ATOM | 4985 | O | PHE | B | 340 | 11.458 | 17.721 | 54.741 | 1.00 | 52.41 | O |
| ATOM | 4986 | N | ASP | B | 341 | 10.236 | 16.178 | 53.686 | 1.00 | 52.29 | N |
| ATOM | 4987 | CA | ASP | B | 341 | 9.863 | 17.112 | 52.637 | 1.00 | 52.29 | C |
| ATOM | 4988 | CB | ASP | B | 341 | 9.333 | 16.356 | 51.409 | 1.00 | 37.13 | C |
| ATOM | 4989 | CG | ASP | B | 341 | 10.451 | 15.730 | 50.569 | 1.00 | 37.13 | C |
| ATOM | 4990 | OD1 | ASP | B | 341 | 11.645 | 15.953 | 50.880 | 1.00 | 37.13 | O |
| ATOM | 4991 | OD2 | ASP | B | 341 | 10.133 | 15.018 | 49.587 | 1.00 | 37.13 | O |
| ATOM | 4992 | C | ASP | B | 341 | 8.794 | 18.067 | 53.190 | 1.00 | 52.29 | C |
| ATOM | 4993 | O | ASP | B | 341 | 8.778 | 19.258 | 52.857 | 1.00 | 52.29 | O |
| ATOM | 4994 | N | GLU | B | 342 | 7.915 | 17.544 | 54.046 | 1.00 | 34.93 | N |
| ATOM | 4995 | CA | GLU | B | 342 | 6.859 | 18.357 | 54.630 | 1.00 | 34.93 | C |
| ATOM | 4996 | CB | GLU | B | 342 | 5.962 | 17.535 | 55.555 | 1.00 | 34.72 | C |
| ATOM | 4997 | CG | GLU | B | 342 | 4.874 | 18.372 | 56.188 | 1.00 | 34.72 | C |
| ATOM | 4998 | CD | GLU | B | 342 | 4.087 | 17.649 | 57.262 | 1.00 | 34.72 | C |
| ATOM | 4999 | OE1 | GLU | B | 342 | 4.667 | 16.832 | 58.013 | 1.00 | 34.72 | O |
| ATOM | 5000 | OE2 | GLU | B | 342 | 2.877 | 17.920 | 57.378 | 1.00 | 34.72 | O |
| ATOM | 5001 | C | GLU | B | 342 | 7.448 | 19.515 | 55.413 | 1.00 | 34.93 | C |
| ATOM | 5002 | O | GLU | B | 342 | 6.789 | 20.533 | 55.584 | 1.00 | 34.93 | O |
| ATOM | 5003 | N | LEU | B | 343 | 8.677 | 19.369 | 55.903 | 1.00 | 29.89 | N |
| ATOM | 5004 | CA | LEU | B | 343 | 9.297 | 20.458 | 56.648 | 1.00 | 29.89 | C |
| ATOM | 5005 | CB | LEU | B | 343 | 10.491 | 19.962 | 57.462 | 1.00 | 22.88 | C |
| ATOM | 5006 | CG | LEU | B | 343 | 10.252 | 18.948 | 58.580 | 1.00 | 22.88 | C |
| ATOM | 5007 | CD1 | LEU | B | 343 | 11.553 | 18.733 | 59.359 | 1.00 | 22.88 | C |
| ATOM | 5008 | CD2 | LEU | B | 343 | 9.141 | 19.452 | 59.484 | 1.00 | 22.88 | C |
| ATOM | 5009 | C | LEU | B | 343 | 9.769 | 21.529 | 55.676 | 1.00 | 29.89 | C |
| ATOM | 5010 | O | LEU | B | 343 | 9.658 | 22.724 | 55.947 | 1.00 | 29.89 | O |
| ATOM | 5011 | N | ARG | B | 344 | 10.302 | 21.096 | 54.541 | 1.00 | 41.81 | N |
| ATOM | 5012 | CA | ARG | B | 344 | 10.801 | 22.031 | 53.528 | 1.00 | 41.81 | C |
| ATOM | 5013 | CB | ARG | B | 344 | 11.663 | 21.293 | 52.492 | 1.00 | 26.01 | C |
| ATOM | 5014 | CG | ARG | B | 344 | 13.135 | 21.155 | 52.863 | 1.00 | 26.01 | C |
| ATOM | 5015 | CD | ARG | B | 344 | 13.861 | 20.289 | 51.837 | 1.00 | 26.01 | C |
| ATOM | 5016 | NE | ARG | B | 344 | 13.392 | 18.908 | 51.881 | 1.00 | 26.01 | N |
| ATOM | 5017 | CZ | ARG | B | 344 | 13.844 | 17.992 | 52.727 | 1.00 | 26.01 | C |
| ATOM | 5018 | NH1 | ARG | B | 344 | 14.789 | 18.308 | 53.588 | 1.00 | 26.01 | N |
| ATOM | 5019 | NH2 | ARG | B | 344 | 13.318 | 16.775 | 52.736 | 1.00 | 26.01 | N |
| ATOM | 5020 | C | ARG | B | 344 | 9.699 | 22.826 | 52.811 | 1.00 | 41.81 | C |
| ATOM | 5021 | O | ARG | B | 344 | 9.967 | 23.570 | 51.855 | 1.00 | 41.81 | O |
| ATOM | 5022 | N | ASP | B | 345 | 8.468 | 22.675 | 53.288 | 1.00 | 47.48 | N |
| ATOM | 5023 | CA | ASP | B | 345 | 7.306 | 23.370 | 52.728 | 1.00 | 47.48 | C |
| ATOM | 5024 | CB | ASP | B | 345 | 6.054 | 22.587 | 53.117 | 1.00 | 54.74 | C |
| ATOM | 5025 | CG | ASP | B | 345 | 4.810 | 23.072 | 52.407 | 1.00 | 54.74 | C |
| ATOM | 5026 | OD1 | ASP | B | 345 | 4.137 | 22.243 | 51.752 | 1.00 | 54.74 | O |
| ATOM | 5027 | OD2 | ASP | B | 345 | 4.496 | 24.275 | 52.515 | 1.00 | 54.74 | O |
| ATOM | 5028 | C | ASP | B | 345 | 7.256 | 24.818 | 53.283 | 1.00 | 47.48 | C |
| ATOM | 5029 | O | ASP | B | 345 | 7.425 | 25.035 | 54.470 | 1.00 | 47.48 | O |
| ATOM | 5030 | N | PRO | B | 346 | 7.038 | 25.827 | 52.422 | 1.00 | 53.82 | N |
| ATOM | 5031 | CD | PRO | B | 346 | 7.127 | 25.608 | 50.975 | 1.00 | 40.08 | C |
| ATOM | 5032 | CA | PRO | B | 346 | 6.959 | 27.265 | 52.765 | 1.00 | 53.82 | C |
| ATOM | 5033 | CB | PRO | B | 346 | 6.625 | 27.932 | 51.446 | 1.00 | 40.08 | C |
| ATOM | 5034 | CG | PRO | B | 346 | 6.965 | 26.953 | 50.426 | 1.00 | 40.08 | C |
| ATOM | 5035 | C | PRO | B | 346 | 5.900 | 27.650 | 53.786 | 1.00 | 53.82 | C |

| | | | | | | | | | | | |
|---|---|---|---|---|---|---|---|---|---|---|---|
| ATOM | 5036 | O | PRO | B | 346 | 5.973 | 28.707 | 54.433 | 1.00 | 53.82 | O |
| ATOM | 5037 | N | ASN | B | 347 | 4.893 | 26.798 | 53.906 | 1.00 | 63.12 | N |
| ATOM | 5038 | CA | ASN | B | 347 | 3.780 | 27.060 | 54.803 | 1.00 | 63.12 | C |
| ATOM | 5039 | CB | ASN | B | 347 | 2.630 | 27.518 | 53.933 | 1.00 | 77.79 | C |
| ATOM | 5040 | CG | ASN | B | 347 | 2.625 | 26.805 | 52.598 | 1.00 | 77.79 | C |
| ATOM | 5041 | OD1 | ASN | B | 347 | 1.734 | 26.011 | 52.329 | 1.00 | 77.79 | O |
| ATOM | 5042 | ND2 | ASN | B | 347 | 3.630 | 27.076 | 51.757 | 1.00 | 77.79 | N |
| ATOM | 5043 | C | ASN | B | 347 | 3.405 | 25.824 | 55.627 | 1.00 | 63.12 | C |
| ATOM | 5044 | O | ASN | B | 347 | 2.301 | 25.285 | 55.516 | 1.00 | 63.12 | O |
| ATOM | 5045 | N | VAL | B | 348 | 4.339 | 25.413 | 56.469 | 1.00 | 74.40 | N |
| ATOM | 5046 | CA | VAL | B | 348 | 4.190 | 24.255 | 57.329 | 1.00 | 74.40 | C |
| ATOM | 5047 | CB | VAL | B | 348 | 5.352 | 23.253 | 57.166 | 1.00 | 67.21 | C |
| ATOM | 5048 | CG1 | VAL | B | 348 | 4.915 | 22.055 | 56.390 | 1.00 | 67.21 | C |
| ATOM | 5049 | CG2 | VAL | B | 348 | 6.521 | 23.912 | 56.496 | 1.00 | 67.21 | C |
| ATOM | 5050 | C | VAL | B | 348 | 4.259 | 24.760 | 58.735 | 1.00 | 74.40 | C |
| ATOM | 5051 | O | VAL | B | 348 | 5.309 | 25.181 | 59.191 | 1.00 | 74.40 | O |
| ATOM | 5052 | N | LYS | B | 349 | 3.145 | 24.727 | 59.433 | 1.00 | 70.15 | N |
| ATOM | 5053 | CA | LYS | B | 349 | 3.151 | 25.213 | 60.793 | 1.00 | 70.15 | C |
| ATOM | 5054 | CB | LYS | B | 349 | 2.140 | 26.335 | 60.889 | 1.00 | 62.03 | C |
| ATOM | 5055 | CG | LYS | B | 349 | 2.627 | 27.552 | 60.150 | 1.00 | 62.03 | C |
| ATOM | 5056 | CD | LYS | B | 349 | 1.523 | 28.436 | 59.655 | 1.00 | 62.03 | C |
| ATOM | 5057 | CE | LYS | B | 349 | 2.103 | 29.817 | 59.404 | 1.00 | 62.03 | C |
| ATOM | 5058 | NZ | LYS | B | 349 | 3.601 | 29.774 | 59.237 | 1.00 | 62.03 | N |
| ATOM | 5059 | C | LYS | B | 349 | 2.853 | 24.060 | 61.710 | 1.00 | 70.15 | C |
| ATOM | 5060 | O | LYS | B | 349 | 2.433 | 23.002 | 61.213 | 1.00 | 70.15 | O |
| ATOM | 5061 | N | LEU | B | 350 | 3.071 | 24.219 | 63.015 | 1.00 | 48.99 | N |
| ATOM | 5062 | CA | LEU | B | 350 | 2.827 | 23.110 | 63.913 | 1.00 | 48.99 | C |
| ATOM | 5063 | CB | LEU | B | 350 | 3.493 | 23.389 | 65.266 | 1.00 | 33.83 | C |
| ATOM | 5064 | CG | LEU | B | 350 | 4.854 | 24.067 | 65.247 | 1.00 | 33.83 | C |
| ATOM | 5065 | CD1 | LEU | B | 350 | 5.215 | 24.406 | 66.660 | 1.00 | 33.83 | C |
| ATOM | 5066 | CD2 | LEU | B | 350 | 5.855 | 23.149 | 64.647 | 1.00 | 33.83 | C |
| ATOM | 5067 | C | LEU | B | 350 | 1.317 | 22.971 | 64.085 | 1.00 | 48.99 | C |
| ATOM | 5068 | O | LEU | B | 350 | 0.556 | 23.760 | 63.579 | 1.00 | 48.99 | O |
| ATOM | 5069 | N | PRO | B | 351 | 0.872 | 21.953 | 64.802 | 1.00 | 42.22 | N |
| ATOM | 5070 | CD | PRO | B | 351 | 1.524 | 20.660 | 64.938 | 1.00 | 27.80 | C |
| ATOM | 5071 | CA | PRO | B | 351 | -0.586 | 21.820 | 64.952 | 1.00 | 42.22 | C |
| ATOM | 5072 | CB | PRO | B | 351 | -0.732 | 20.651 | 65.950 | 1.00 | 27.80 | C |
| ATOM | 5073 | CG | PRO | B | 351 | 0.719 | 20.050 | 66.019 | 1.00 | 27.80 | C |
| ATOM | 5074 | C | PRO | B | 351 | -1.199 | 23.069 | 65.405 | 1.00 | 42.22 | C |
| ATOM | 5075 | O | PRO | B | 351 | -2.324 | 23.382 | 65.024 | 1.00 | 42.22 | O |
| ATOM | 5076 | N | ASN | B | 352 | -0.407 | 23.807 | 66.153 | 1.00 | 56.68 | N |
| ATOM | 5077 | CA | ASN | B | 352 | -0.862 | 25.073 | 66.671 | 1.00 | 56.68 | C |
| ATOM | 5078 | CB | ASN | B | 352 | -0.187 | 25.339 | 68.001 | 1.00 | 64.57 | C |
| ATOM | 5079 | CG | ASN | B | 352 | 1.281 | 25.752 | 67.854 | 1.00 | 64.57 | C |
| ATOM | 5080 | OD1 | ASN | B | 352 | 2.024 | 25.723 | 68.827 | 1.00 | 64.57 | O |
| ATOM | 5081 | ND2 | ASN | B | 352 | 1.691 | 26.162 | 66.653 | 1.00 | 64.57 | N |
| ATOM | 5082 | C | ASN | B | 352 | -0.754 | 26.358 | 65.835 | 1.00 | 56.68 | C |
| ATOM | 5083 | O | ASN | B | 352 | -0.731 | 27.446 | 66.411 | 1.00 | 56.68 | O |
| ATOM | 5084 | N | GLY | B | 353 | -0.731 | 26.260 | 64.510 | 1.00 | 46.93 | N |
| ATOM | 5085 | CA | GLY | B | 353 | -0.615 | 27.459 | 63.690 | 1.00 | 46.93 | C |
| ATOM | 5086 | C | GLY | B | 353 | 0.645 | 28.294 | 63.856 | 1.00 | 46.93 | C |
| ATOM | 5087 | O | GLY | B | 353 | 0.973 | 29.044 | 62.933 | 1.00 | 46.93 | O |
| ATOM | 5088 | N | ARG | B | 354 | 1.403 | 28.091 | 64.938 | 1.00 | 54.48 | N |
| ATOM | 5089 | CA | ARG | B | 354 | 2.606 | 28.865 | 65.131 | 1.00 | 54.48 | C |
| ATOM | 5090 | CB | ARG | B | 354 | 3.106 | 28.779 | 66.591 | 1.00 | 99.39 | C |
| ATOM | 5091 | CG | ARG | B | 354 | 2.400 | 29.706 | 67.609 | 1.00 | 99.39 | C |
| ATOM | 5092 | CD | ARG | B | 354 | 3.011 | 29.547 | 69.025 | 1.00 | 99.39 | C |
| ATOM | 5093 | NE | ARG | B | 354 | 2.262 | 30.313 | 70.025 | 1.00 | 99.39 | N |
| ATOM | 5094 | CZ | ARG | B | 354 | 2.598 | 30.391 | 71.314 | 1.00 | 99.39 | C |
| ATOM | 5095 | NH1 | ARG | B | 354 | 1.863 | 31.107 | 72.166 | 1.00 | 99.39 | N |
| ATOM | 5096 | NH2 | ARG | B | 354 | 3.661 | 29.721 | 71.768 | 1.00 | 99.39 | N |
| ATOM | 5097 | C | ARG | B | 354 | 3.602 | 28.325 | 64.166 | 1.00 | 54.48 | C |
| ATOM | 5098 | O | ARG | B | 354 | 3.437 | 27.204 | 63.699 | 1.00 | 54.48 | O |

| | | | | | | | | | | | |
|---|---|---|---|---|---|---|---|---|---|---|---|
| ATOM | 5099 | N | ASP | B | 355 | 4.605 | 29.138 | 63.853 | 1.00 | 38.07 | N |
| ATOM | 5100 | CA | ASP | B | 355 | 5.652 | 28.745 | 62.911 | 1.00 | 38.07 | C |
| ATOM | 5101 | CB | ASP | B | 355 | 6.574 | 29.936 | 62.616 | 1.00 | 57.46 | C |
| ATOM | 5102 | CG | ASP | B | 355 | 6.308 | 30.576 | 61.266 | 1.00 | 57.46 | C |
| ATOM | 5103 | OD1 | ASP | B | 355 | 6.109 | 29.840 | 60.257 | 1.00 | 57.46 | O |
| ATOM | 5104 | OD2 | ASP | B | 355 | 6.350 | 31.824 | 61.226 | 1.00 | 57.46 | O |
| ATOM | 5105 | C | ASP | B | 355 | 6.523 | 27.581 | 63.393 | 1.00 | 38.07 | C |
| ATOM | 5106 | O | ASP | B | 355 | 6.606 | 27.326 | 64.584 | 1.00 | 38.07 | O |
| ATOM | 5107 | N | THR | B | 356 | 7.207 | 26.905 | 62.479 | 1.00 | 43.54 | N |
| ATOM | 5108 | CA | THR | B | 356 | 8.086 | 25.819 | 62.871 | 1.00 | 43.54 | C |
| ATOM | 5109 | CB | THR | B | 356 | 8.305 | 24.852 | 61.688 | 1.00 | 38.93 | C |
| ATOM | 5110 | OG1 | THR | B | 356 | 8.678 | 25.590 | 60.506 | 1.00 | 38.93 | O |
| ATOM | 5111 | CG2 | THR | B | 356 | 7.011 | 24.081 | 61.409 | 1.00 | 38.93 | C |
| ATOM | 5112 | C | THR | B | 356 | 9.407 | 26.481 | 63.265 | 1.00 | 43.54 | C |
| ATOM | 5113 | O | THR | B | 356 | 9.613 | 27.655 | 62.972 | 1.00 | 43.54 | O |
| ATOM | 5114 | N | PRO | B | 357 | 10.290 | 25.750 | 63.971 | 1.00 | 33.58 | N |
| ATOM | 5115 | CD | PRO | B | 357 | 10.008 | 24.510 | 64.717 | 1.00 | 42.70 | C |
| ATOM | 5116 | CA | PRO | B | 357 | 11.580 | 26.311 | 64.389 | 1.00 | 33.58 | C |
| ATOM | 5117 | CB | PRO | B | 357 | 12.038 | 25.338 | 65.474 | 1.00 | 42.70 | C |
| ATOM | 5118 | CG | PRO | B | 357 | 11.375 | 24.038 | 65.101 | 1.00 | 42.70 | C |
| ATOM | 5119 | C | PRO | B | 357 | 12.576 | 26.448 | 63.270 | 1.00 | 33.58 | C |
| ATOM | 5120 | O | PRO | B | 357 | 12.281 | 26.079 | 62.144 | 1.00 | 33.58 | O |
| ATOM | 5121 | N | ALA | B | 358 | 13.740 | 27.010 | 63.569 | 1.00 | 40.19 | N |
| ATOM | 5122 | CA | ALA | B | 358 | 14.763 | 27.168 | 62.545 | 1.00 | 40.19 | C |
| ATOM | 5123 | CB | ALA | B | 358 | 15.989 | 27.863 | 63.118 | 1.00 | 29.36 | C |
| ATOM | 5124 | C | ALA | B | 358 | 15.155 | 25.793 | 62.016 | 1.00 | 40.19 | C |
| ATOM | 5125 | O | ALA | B | 358 | 15.444 | 24.881 | 62.797 | 1.00 | 40.19 | O |
| ATOM | 5126 | N | LEU | B | 359 | 15.180 | 25.632 | 60.697 | 1.00 | 31.70 | N |
| ATOM | 5127 | CA | LEU | B | 359 | 15.555 | 24.338 | 60.143 | 1.00 | 31.70 | C |
| ATOM | 5128 | CB | LEU | B | 359 | 14.303 | 23.568 | 59.710 | 1.00 | 28.28 | C |
| ATOM | 5129 | CG | LEU | B | 359 | 13.255 | 23.276 | 60.790 | 1.00 | 28.28 | C |
| ATOM | 5130 | CD1 | LEU | B | 359 | 12.022 | 22.650 | 60.142 | 1.00 | 28.28 | C |
| ATOM | 5131 | CD2 | LEU | B | 359 | 13.826 | 22.356 | 61.849 | 1.00 | 28.28 | C |
| ATOM | 5132 | C | LEU | B | 359 | 16.505 | 24.431 | 58.965 | 1.00 | 31.70 | C |
| ATOM | 5133 | O | LEU | B | 359 | 17.156 | 23.446 | 58.609 | 1.00 | 31.70 | O |
| ATOM | 5134 | N | PHE | B | 360 | 16.616 | 25.617 | 58.380 | 1.00 | 48.61 | N |
| ATOM | 5135 | CA | PHE | B | 360 | 17.442 | 25.762 | 57.192 | 1.00 | 48.61 | C |
| ATOM | 5136 | CB | PHE | B | 360 | 16.560 | 26.245 | 56.035 | 1.00 | 44.87 | C |
| ATOM | 5137 | CG | PHE | B | 360 | 15.230 | 25.553 | 55.964 | 1.00 | 44.87 | C |
| ATOM | 5138 | CD1 | PHE | B | 360 | 15.143 | 24.218 | 55.587 | 1.00 | 44.87 | C |
| ATOM | 5139 | CD2 | PHE | B | 360 | 14.073 | 26.220 | 56.330 | 1.00 | 44.87 | C |
| ATOM | 5140 | CE1 | PHE | B | 360 | 13.919 | 23.558 | 55.581 | 1.00 | 44.87 | C |
| ATOM | 5141 | CE2 | PHE | B | 360 | 12.849 | 25.571 | 56.327 | 1.00 | 44.87 | C |
| ATOM | 5142 | CZ | PHE | B | 360 | 12.770 | 24.236 | 55.953 | 1.00 | 44.87 | C |
| ATOM | 5143 | C | PHE | B | 360 | 18.657 | 26.658 | 57.294 | 1.00 | 48.61 | C |
| ATOM | 5144 | O | PHE | B | 360 | 19.332 | 26.893 | 56.294 | 1.00 | 48.61 | O |
| ATOM | 5145 | N | ASN | B | 361 | 18.955 | 27.152 | 58.486 | 1.00 | 32.42 | N |
| ATOM | 5146 | CA | ASN | B | 361 | 20.102 | 28.034 | 58.632 | 1.00 | 32.42 | C |
| ATOM | 5147 | CB | ASN | B | 361 | 19.927 | 28.911 | 59.866 | 1.00 | 38.75 | C |
| ATOM | 5148 | CG | ASN | B | 361 | 19.831 | 28.105 | 61.141 | 1.00 | 38.75 | C |
| ATOM | 5149 | OD1 | ASN | B | 361 | 19.077 | 27.143 | 61.226 | 1.00 | 38.75 | O |
| ATOM | 5150 | ND2 | ASN | B | 361 | 20.595 | 28.501 | 62.147 | 1.00 | 38.75 | N |
| ATOM | 5151 | C | ASN | B | 361 | 21.419 | 27.276 | 58.707 | 1.00 | 32.42 | C |
| ATOM | 5152 | O | ASN | B | 361 | 22.247 | 27.537 | 59.578 | 1.00 | 32.42 | O |
| ATOM | 5153 | N | PHE | B | 362 | 21.619 | 26.333 | 57.796 | 1.00 | 34.23 | N |
| ATOM | 5154 | CA | PHE | B | 362 | 22.859 | 25.568 | 57.797 | 1.00 | 34.23 | C |
| ATOM | 5155 | CB | PHE | B | 362 | 22.852 | 24.551 | 56.659 | 1.00 | 40.30 | C |
| ATOM | 5156 | CG | PHE | B | 362 | 21.829 | 23.489 | 56.832 | 1.00 | 40.30 | C |
| ATOM | 5157 | CD1 | PHE | B | 362 | 22.001 | 22.502 | 57.790 | 1.00 | 40.30 | C |
| ATOM | 5158 | CD2 | PHE | B | 362 | 20.668 | 23.499 | 56.078 | 1.00 | 40.30 | C |
| ATOM | 5159 | CE1 | PHE | B | 362 | 21.032 | 21.537 | 58.004 | 1.00 | 40.30 | C |
| ATOM | 5160 | CE2 | PHE | B | 362 | 19.684 | 22.535 | 56.282 | 1.00 | 40.30 | C |
| ATOM | 5161 | CZ | PHE | B | 362 | 19.868 | 21.548 | 57.253 | 1.00 | 40.30 | C |

| | | | | | | | | | | | |
|---|---|---|---|---|---|---|---|---|---|---|---|
| ATOM | 5162 | C | PHE | B | 362 | 24.078 | 26.475 | 57.665 | 1.00 | 34.23 | C |
| ATOM | 5163 | O | PHE | B | 362 | 23.972 | 27.628 | 57.236 | 1.00 | 34.23 | O |
| ATOM | 5164 | N | THR | B | 363 | 25.237 | 25.955 | 58.044 | 1.00 | 48.58 | N |
| ATOM | 5165 | CA | THR | B | 363 | 26.468 | 26.722 | 57.943 | 1.00 | 48.58 | C |
| ATOM | 5166 | CB | THR | B | 363 | 27.166 | 26.926 | 59.321 | 1.00 | 49.03 | C |
| ATOM | 5167 | OG1 | THR | B | 363 | 27.619 | 25.667 | 59.843 | 1.00 | 49.03 | O |
| ATOM | 5168 | CG2 | THR | B | 363 | 26.208 | 27.562 | 60.302 | 1.00 | 49.03 | C |
| ATOM | 5169 | C | THR | B | 363 | 27.389 | 25.923 | 57.059 | 1.00 | 48.58 | C |
| ATOM | 5170 | O | THR | B | 363 | 27.289 | 24.702 | 56.996 | 1.00 | 48.58 | O |
| ATOM | 5171 | N | THR | B | 364 | 28.286 | 26.612 | 56.374 | 1.00 | 66.27 | N |
| ATOM | 5172 | CA | THR | B | 364 | 29.234 | 25.949 | 55.494 | 1.00 | 66.27 | C |
| ATOM | 5173 | CB | THR | B | 364 | 30.287 | 26.961 | 55.019 | 1.00 | 43.80 | C |
| ATOM | 5174 | OG1 | THR | B | 364 | 30.983 | 27.489 | 56.153 | 1.00 | 43.80 | O |
| ATOM | 5175 | CG2 | THR | B | 364 | 29.611 | 28.115 | 54.287 | 1.00 | 43.80 | C |
| ATOM | 5176 | C | THR | B | 364 | 29.920 | 24.753 | 56.182 | 1.00 | 66.27 | C |
| ATOM | 5177 | O | THR | B | 364 | 30.371 | 23.811 | 55.520 | 1.00 | 66.27 | O |
| ATOM | 5178 | N | GLN | B | 365 | 29.993 | 24.804 | 57.510 | 1.00 | 46.92 | N |
| ATOM | 5179 | CA | GLN | B | 365 | 30.600 | 23.747 | 58.317 | 1.00 | 46.92 | C |
| ATOM | 5180 | CB | GLN | B | 365 | 30.816 | 24.256 | 59.752 | 1.00 | 60.59 | C |
| ATOM | 5181 | CG | GLN | B | 365 | 31.018 | 23.180 | 60.812 | 1.00 | 60.59 | C |
| ATOM | 5182 | CD | GLN | B | 365 | 32.451 | 22.705 | 60.918 | 1.00 | 60.59 | C |
| ATOM | 5183 | OE1 | GLN | B | 365 | 32.735 | 21.709 | 61.583 | 1.00 | 60.59 | O |
| ATOM | 5184 | NE2 | GLN | B | 365 | 33.365 | 23.419 | 60.273 | 1.00 | 60.59 | N |
| ATOM | 5185 | C | GLN | B | 365 | 29.656 | 22.547 | 58.327 | 1.00 | 46.92 | C |
| ATOM | 5186 | O | GLN | B | 365 | 30.075 | 21.405 | 58.098 | 1.00 | 46.92 | O |
| ATOM | 5187 | N | GLU | B | 366 | 28.379 | 22.818 | 58.597 | 1.00 | 44.48 | N |
| ATOM | 5188 | CA | GLU | B | 366 | 27.369 | 21.766 | 58.637 | 1.00 | 44.48 | C |
| ATOM | 5189 | CB | GLU | B | 366 | 25.974 | 22.354 | 58.876 | 1.00 | 50.96 | C |
| ATOM | 5190 | CG | GLU | B | 366 | 25.822 | 23.136 | 60.163 | 1.00 | 50.96 | C |
| ATOM | 5191 | CD | GLU | B | 366 | 24.370 | 23.381 | 60.508 | 1.00 | 50.96 | C |
| ATOM | 5192 | OE1 | GLU | B | 366 | 23.644 | 22.379 | 60.701 | 1.00 | 50.96 | O |
| ATOM | 5193 | OE2 | GLU | B | 366 | 23.955 | 24.564 | 60.583 | 1.00 | 50.96 | O |
| ATOM | 5194 | C | GLU | B | 366 | 27.358 | 20.989 | 57.329 | 1.00 | 44.48 | C |
| ATOM | 5195 | O | GLU | B | 366 | 27.330 | 19.760 | 57.335 | 1.00 | 44.48 | O |
| ATOM | 5196 | N | LEU | B | 367 | 27.397 | 21.713 | 56.212 | 1.00 | 46.28 | N |
| ATOM | 5197 | CA | LEU | B | 367 | 27.368 | 21.113 | 54.879 | 1.00 | 46.28 | C |
| ATOM | 5198 | CB | LEU | B | 367 | 26.834 | 22.140 | 53.887 | 1.00 | 29.99 | C |
| ATOM | 5199 | CG | LEU | B | 367 | 25.538 | 22.835 | 54.315 | 1.00 | 29.99 | C |
| ATOM | 5200 | CD1 | LEU | B | 367 | 25.195 | 23.957 | 53.333 | 1.00 | 29.99 | C |
| ATOM | 5201 | CD2 | LEU | B | 367 | 24.398 | 21.817 | 54.374 | 1.00 | 29.99 | C |
| ATOM | 5202 | C | LEU | B | 367 | 28.717 | 20.578 | 54.377 | 1.00 | 46.28 | C |
| ATOM | 5203 | O | LEU | B | 367 | 28.823 | 20.107 | 53.242 | 1.00 | 46.28 | O |
| ATOM | 5204 | N | SER | B | 368 | 29.739 | 20.644 | 55.226 | 1.00 | 42.12 | N |
| ATOM | 5205 | CA | SER | B | 368 | 31.076 | 20.188 | 54.862 | 1.00 | 42.12 | C |
| ATOM | 5206 | CB | SER | B | 368 | 32.006 | 20.191 | 56.085 | 1.00 | 41.88 | C |
| ATOM | 5207 | OG | SER | B | 368 | 31.713 | 19.138 | 56.991 | 1.00 | 41.88 | O |
| ATOM | 5208 | C | SER | B | 368 | 31.126 | 18.812 | 54.220 | 1.00 | 42.12 | C |
| ATOM | 5209 | O | SER | B | 368 | 31.753 | 18.647 | 53.177 | 1.00 | 42.12 | O |
| ATOM | 5210 | N | SER | B | 369 | 30.474 | 17.827 | 54.837 | 1.00 | 75.62 | N |
| ATOM | 5211 | CA | SER | B | 369 | 30.499 | 16.453 | 54.328 | 1.00 | 75.62 | C |
| ATOM | 5212 | CB | SER | B | 369 | 29.748 | 15.518 | 55.276 | 1.00 | 30.91 | C |
| ATOM | 5213 | OG | SER | B | 369 | 28.350 | 15.656 | 55.121 | 1.00 | 30.91 | O |
| ATOM | 5214 | C | SER | B | 369 | 29.939 | 16.290 | 52.920 | 1.00 | 75.62 | C |
| ATOM | 5215 | O | SER | B | 369 | 30.273 | 15.328 | 52.224 | 1.00 | 75.62 | O |
| ATOM | 5216 | N | ASN | B | 370 | 29.076 | 17.217 | 52.511 | 1.00 | 44.71 | N |
| ATOM | 5217 | CA | ASN | B | 370 | 28.487 | 17.181 | 51.175 | 1.00 | 44.71 | C |
| ATOM | 5218 | CB | ASN | B | 370 | 27.547 | 15.988 | 51.015 | 1.00 | 46.21 | C |
| ATOM | 5219 | CG | ASN | B | 370 | 26.973 | 15.894 | 49.622 | 1.00 | 46.21 | C |
| ATOM | 5220 | OD1 | ASN | B | 370 | 25.945 | 16.497 | 49.330 | 1.00 | 46.21 | O |
| ATOM | 5221 | ND2 | ASN | B | 370 | 27.646 | 15.154 | 48.744 | 1.00 | 46.21 | N |
| ATOM | 5222 | C | ASN | B | 370 | 27.743 | 18.463 | 50.841 | 1.00 | 44.71 | C |
| ATOM | 5223 | O | ASN | B | 370 | 26.520 | 18.532 | 50.944 | 1.00 | 44.71 | O |
| ATOM | 5224 | N | PRO | B | 371 | 28.488 | 19.497 | 50.417 | 1.00 | 44.39 | N |

| | | | | | | | | | | | |
|---|---|---|---|---|---|---|---|---|---|---|---|
| ATOM | 5225 | CD | PRO | B | 371 | 29.954 | 19.445 | 50.247 | 1.00 | 14.29 | C |
| ATOM | 5226 | CA | PRO | B | 371 | 27.977 | 20.818 | 50.045 | 1.00 | 44.39 | C |
| ATOM | 5227 | CB | PRO | B | 371 | 29.200 | 21.491 | 49.446 | 1.00 | 14.29 | C |
| ATOM | 5228 | CG | PRO | B | 371 | 30.321 | 20.901 | 50.237 | 1.00 | 14.29 | C |
| ATOM | 5229 | C | PRO | B | 371 | 26.787 | 20.811 | 49.077 | 1.00 | 44.39 | C |
| ATOM | 5230 | O | PRO | B | 371 | 25.773 | 21.461 | 49.331 | 1.00 | 44.39 | O |
| ATOM | 5231 | N | PRO | B | 372 | 26.896 | 20.084 | 47.948 | 1.00 | 50.52 | N |
| ATOM | 5232 | CD | PRO | B | 372 | 28.013 | 19.248 | 47.477 | 1.00 | 51.18 | C |
| ATOM | 5233 | CA | PRO | B | 372 | 25.788 | 20.043 | 46.987 | 1.00 | 50.52 | C |
| ATOM | 5234 | CB | PRO | B | 372 | 26.281 | 19.052 | 45.933 | 1.00 | 51.18 | C |
| ATOM | 5235 | CG | PRO | B | 372 | 27.290 | 18.220 | 46.664 | 1.00 | 51.18 | C |
| ATOM | 5236 | C | PRO | B | 372 | 24.411 | 19.679 | 47.538 | 1.00 | 50.52 | C |
| ATOM | 5237 | O | PRO | B | 372 | 23.400 | 19.947 | 46.892 | 1.00 | 50.52 | O |
| ATOM | 5238 | N | LEU | B | 373 | 24.362 | 19.067 | 48.720 | 1.00 | 33.41 | N |
| ATOM | 5239 | CA | LEU | B | 373 | 23.077 | 18.704 | 49.319 | 1.00 | 33.41 | C |
| ATOM | 5240 | CB | LEU | B | 373 | 23.280 | 17.817 | 50.548 | 1.00 | 27.82 | C |
| ATOM | 5241 | CG | LEU | B | 373 | 23.145 | 16.312 | 50.317 | 1.00 | 27.82 | C |
| ATOM | 5242 | CD1 | LEU | B | 373 | 23.175 | 15.608 | 51.659 | 1.00 | 27.82 | C |
| ATOM | 5243 | CD2 | LEU | B | 373 | 21.847 | 16.012 | 49.578 | 1.00 | 27.82 | C |
| ATOM | 5244 | C | LEU | B | 373 | 22.293 | 19.934 | 49.733 | 1.00 | 33.41 | C |
| ATOM | 5245 | O | LEU | B | 373 | 21.097 | 19.857 | 50.019 | 1.00 | 33.41 | O |
| ATOM | 5246 | N | ALA | B | 374 | 22.988 | 21.065 | 49.766 | 1.00 | 33.79 | N |
| ATOM | 5247 | CA | ALA | B | 374 | 22.401 | 22.341 | 50.163 | 1.00 | 33.79 | C |
| ATOM | 5248 | CB | ALA | B | 374 | 23.480 | 23.447 | 50.131 | 1.00 | 16.28 | C |
| ATOM | 5249 | C | ALA | B | 374 | 21.221 | 22.728 | 49.282 | 1.00 | 33.79 | C |
| ATOM | 5250 | O | ALA | B | 374 | 20.243 | 23.313 | 49.756 | 1.00 | 33.79 | O |
| ATOM | 5251 | N | THR | B | 375 | 21.325 | 22.386 | 48.001 | 1.00 | 49.11 | N |
| ATOM | 5252 | CA | THR | B | 375 | 20.293 | 22.686 | 47.015 | 1.00 | 49.11 | C |
| ATOM | 5253 | CB | THR | B | 375 | 20.658 | 22.088 | 45.638 | 1.00 | 41.67 | C |
| ATOM | 5254 | OG1 | THR | B | 375 | 21.788 | 22.790 | 45.100 | 1.00 | 41.67 | O |
| ATOM | 5255 | CG2 | THR | B | 375 | 19.478 | 22.190 | 44.669 | 1.00 | 41.67 | C |
| ATOM | 5256 | C | THR | B | 375 | 18.942 | 22.146 | 47.439 | 1.00 | 49.11 | C |
| ATOM | 5257 | O | THR | B | 375 | 17.902 | 22.715 | 47.102 | 1.00 | 49.11 | O |
| ATOM | 5258 | N | ILE | B | 376 | 18.971 | 21.047 | 48.182 | 1.00 | 45.63 | N |
| ATOM | 5259 | CA | ILE | B | 376 | 17.759 | 20.416 | 48.667 | 1.00 | 45.63 | C |
| ATOM | 5260 | CB | ILE | B | 376 | 17.883 | 18.908 | 48.589 | 1.00 | 37.88 | C |
| ATOM | 5261 | CG2 | ILE | B | 376 | 16.627 | 18.243 | 49.151 | 1.00 | 37.88 | C |
| ATOM | 5262 | CG1 | ILE | B | 376 | 18.118 | 18.522 | 47.133 | 1.00 | 37.88 | C |
| ATOM | 5263 | CD1 | ILE | B | 376 | 18.544 | 17.121 | 46.950 | 1.00 | 37.88 | C |
| ATOM | 5264 | C | ILE | B | 376 | 17.451 | 20.807 | 50.101 | 1.00 | 45.63 | C |
| ATOM | 5265 | O | ILE | B | 376 | 16.297 | 21.064 | 50.445 | 1.00 | 45.63 | O |
| ATOM | 5266 | N | LEU | B | 377 | 18.484 | 20.847 | 50.936 | 1.00 | 42.98 | N |
| ATOM | 5267 | CA | LEU | B | 377 | 18.311 | 21.208 | 52.337 | 1.00 | 42.98 | C |
| ATOM | 5268 | CB | LEU | B | 377 | 19.656 | 21.116 | 53.064 | 1.00 | 27.06 | C |
| ATOM | 5269 | CG | LEU | B | 377 | 20.041 | 19.674 | 53.380 | 1.00 | 27.06 | C |
| ATOM | 5270 | CD1 | LEU | B | 377 | 21.505 | 19.588 | 53.802 | 1.00 | 27.06 | C |
| ATOM | 5271 | CD2 | LEU | B | 377 | 19.115 | 19.157 | 54.470 | 1.00 | 27.06 | C |
| ATOM | 5272 | C | LEU | B | 377 | 17.729 | 22.610 | 52.496 | 1.00 | 42.98 | C |
| ATOM | 5273 | O | LEU | B | 377 | 16.833 | 22.845 | 53.320 | 1.00 | 42.98 | O |
| ATOM | 5274 | N | ILE | B | 378 | 18.239 | 23.532 | 51.688 | 1.00 | 50.15 | N |
| ATOM | 5275 | CA | ILE | B | 378 | 17.802 | 24.919 | 51.738 | 1.00 | 50.15 | C |
| ATOM | 5276 | CB | ILE | B | 378 | 19.009 | 25.842 | 51.599 | 1.00 | 49.14 | C |
| ATOM | 5277 | CG2 | ILE | B | 378 | 18.576 | 27.281 | 51.778 | 1.00 | 49.14 | C |
| ATOM | 5278 | CG1 | ILE | B | 378 | 20.060 | 25.461 | 52.650 | 1.00 | 49.14 | C |
| ATOM | 5279 | CD1 | ILE | B | 378 | 21.418 | 26.082 | 52.433 | 1.00 | 49.14 | C |
| ATOM | 5280 | C | ILE | B | 378 | 16.793 | 25.213 | 50.628 | 1.00 | 50.15 | C |
| ATOM | 5281 | O | ILE | B | 378 | 17.175 | 25.413 | 49.475 | 1.00 | 50.15 | O |
| ATOM | 5282 | N | PRO | B | 379 | 15.487 | 25.256 | 50.976 | 1.00 | 39.64 | N |
| ATOM | 5283 | CD | PRO | B | 379 | 14.928 | 25.198 | 52.334 | 1.00 | 33.73 | C |
| ATOM | 5284 | CA | PRO | B | 379 | 14.414 | 25.517 | 50.019 | 1.00 | 39.64 | C |
| ATOM | 5285 | CB | PRO | B | 379 | 13.140 | 25.287 | 50.844 | 1.00 | 33.73 | C |
| ATOM | 5286 | CG | PRO | B | 379 | 13.596 | 24.581 | 52.062 | 1.00 | 33.73 | C |
| ATOM | 5287 | C | PRO | B | 379 | 14.524 | 26.949 | 49.546 | 1.00 | 39.64 | C |

| | | | | | | | | | | | |
|---|---|---|---|---|---|---|---|---|---|---|---|
| ATOM | 5288 | O | PRO | B | 379 | 15.001 | 27.804 | 50.280 | 1.00 | 39.64 | O |
| ATOM | 5289 | N | PRO | B | 380 | 14.082 | 27.230 | 48.314 | 1.00 | 67.03 | N |
| ATOM | 5290 | CD | PRO | B | 380 | 13.597 | 26.271 | 47.305 | 1.00 | 77.46 | C |
| ATOM | 5291 | CA | PRO | B | 380 | 14.139 | 28.582 | 47.757 | 1.00 | 67.03 | C |
| ATOM | 5292 | CB | PRO | B | 380 | 13.356 | 28.441 | 46.461 | 1.00 | 77.46 | C |
| ATOM | 5293 | CG | PRO | B | 380 | 13.729 | 27.057 | 46.026 | 1.00 | 77.46 | C |
| ATOM | 5294 | C | PRO | B | 380 | 13.583 | 29.685 | 48.662 | 1.00 | 67.03 | C |
| ATOM | 5295 | O | PRO | B | 380 | 14.223 | 30.725 | 48.820 | 1.00 | 67.03 | O |
| ATOM | 5296 | N | HIS | B | 381 | 12.415 | 29.459 | 49.265 | 1.00 | 39.48 | N |
| ATOM | 5297 | CA | HIS | B | 381 | 11.794 | 30.476 | 50.123 | 1.00 | 39.48 | C |
| ATOM | 5298 | CB | HIS | B | 381 | 10.388 | 30.043 | 50.546 | 1.00 | 66.37 | C |
| ATOM | 5299 | CG | HIS | B | 381 | 10.367 | 28.962 | 51.579 | 1.00 | 66.37 | C |
| ATOM | 5300 | CD2 | HIS | B | 381 | 10.142 | 27.633 | 51.470 | 1.00 | 66.37 | C |
| ATOM | 5301 | ND1 | HIS | B | 381 | 10.602 | 29.202 | 52.915 | 1.00 | 66.37 | N |
| ATOM | 5302 | CE1 | HIS | B | 381 | 10.520 | 28.066 | 53.585 | 1.00 | 66.37 | C |
| ATOM | 5303 | NE2 | HIS | B | 381 | 10.243 | 27.098 | 52.731 | 1.00 | 66.37 | N |
| ATOM | 5304 | C | HIS | B | 381 | 12.595 | 30.841 | 51.362 | 1.00 | 39.48 | C |
| ATOM | 5305 | O | HIS | B | 381 | 12.614 | 31.997 | 51.770 | 1.00 | 39.48 | O |
| ATOM | 5306 | N | ALA | B | 382 | 13.257 | 29.859 | 51.959 | 1.00 | 68.78 | N |
| ATOM | 5307 | CA | ALA | B | 382 | 14.057 | 30.113 | 53.149 | 1.00 | 68.78 | C |
| ATOM | 5308 | CB | ALA | B | 382 | 14.384 | 28.800 | 53.843 | 1.00 | 53.94 | C |
| ATOM | 5309 | C | ALA | B | 382 | 15.343 | 30.851 | 52.785 | 1.00 | 68.78 | C |
| ATOM | 5310 | O | ALA | B | 382 | 16.155 | 31.170 | 53.656 | 1.00 | 68.78 | O |
| ATOM | 5311 | N | ARG | B | 383 | 15.519 | 31.128 | 51.496 | 1.00 | 62.64 | N |
| ATOM | 5312 | CA | ARG | B | 383 | 16.712 | 31.816 | 51.028 | 1.00 | 62.64 | C |
| ATOM | 5313 | CB | ARG | B | 383 | 16.851 | 31.623 | 49.520 | 1.00 | 61.02 | C |
| ATOM | 5314 | CG | ARG | B | 383 | 18.264 | 31.315 | 49.067 | 1.00 | 61.02 | C |
| ATOM | 5315 | CD | ARG | B | 383 | 18.539 | 29.825 | 49.040 | 1.00 | 61.02 | C |
| ATOM | 5316 | NE | ARG | B | 383 | 17.722 | 29.164 | 48.028 | 1.00 | 61.02 | N |
| ATOM | 5317 | CZ | ARG | B | 383 | 17.908 | 27.913 | 47.615 | 1.00 | 61.02 | C |
| ATOM | 5318 | NH1 | ARG | B | 383 | 18.892 | 27.180 | 48.132 | 1.00 | 61.02 | N |
| ATOM | 5319 | NH2 | ARG | B | 383 | 17.112 | 27.397 | 46.684 | 1.00 | 61.02 | N |
| ATOM | 5320 | C | ARG | B | 383 | 16.676 | 33.315 | 51.362 | 1.00 | 62.64 | C |
| ATOM | 5321 | O | ARG | B | 383 | 17.233 | 34.110 | 50.576 | 1.00 | 62.64 | O |
| ATOM | 5322 | OXT | ARG | B | 383 | 16.114 | 33.683 | 52.419 | 1.00 | 61.02 | O |
| TER | 5323 | | ARG | B | 383 | | | | | | |
| ATOM | 5324 | CB | SER | C | 3 | 24.199 | 4.995 | 78.942 | 1.00 | 61.09 | C |
| ATOM | 5325 | OG | SER | C | 3 | 24.607 | 5.817 | 80.023 | 1.00 | 61.09 | O |
| ATOM | 5326 | C | SER | C | 3 | 25.505 | 3.073 | 79.952 | 1.00 | 34.71 | C |
| ATOM | 5327 | O | SER | C | 3 | 25.741 | 3.303 | 81.140 | 1.00 | 34.71 | O |
| ATOM | 5328 | N | SER | C | 3 | 23.017 | 3.199 | 80.239 | 1.00 | 34.71 | N |
| ATOM | 5329 | CA | SER | C | 3 | 24.166 | 3.493 | 79.318 | 1.00 | 34.71 | C |
| ATOM | 5330 | N | PRO | C | 4 | 26.400 | 2.460 | 79.151 | 1.00 | 35.32 | N |
| ATOM | 5331 | CD | PRO | C | 4 | 26.178 | 2.096 | 77.740 | 1.00 | 65.93 | C |
| ATOM | 5332 | CA | PRO | C | 4 | 27.710 | 1.988 | 79.587 | 1.00 | 35.32 | C |
| ATOM | 5333 | CB | PRO | C | 4 | 28.130 | 1.058 | 78.458 | 1.00 | 65.93 | C |
| ATOM | 5334 | CG | PRO | C | 4 | 27.573 | 1.714 | 77.284 | 1.00 | 65.93 | C |
| ATOM | 5335 | C | PRO | C | 4 | 28.788 | 2.999 | 79.904 | 1.00 | 35.32 | C |
| ATOM | 5336 | O | PRO | C | 4 | 28.616 | 4.213 | 79.787 | 1.00 | 35.32 | O |
| ATOM | 5337 | N | HIS | C | 5 | 29.928 | 2.454 | 80.294 | 1.00 | 45.56 | N |
| ATOM | 5338 | CA | HIS | C | 5 | 31.064 | 3.250 | 80.664 | 1.00 | 45.56 | C |
| ATOM | 5339 | CB | HIS | C | 5 | 31.763 | 2.620 | 81.840 | 1.00 | 69.60 | C |
| ATOM | 5340 | CG | HIS | C | 5 | 31.283 | 3.138 | 83.151 | 1.00 | 69.60 | C |
| ATOM | 5341 | CD2 | HIS | C | 5 | 31.152 | 4.407 | 83.604 | 1.00 | 69.60 | C |
| ATOM | 5342 | ND1 | HIS | C | 5 | 30.866 | 2.317 | 84.176 | 1.00 | 69.60 | N |
| ATOM | 5343 | CE1 | HIS | C | 5 | 30.498 | 3.057 | 85.206 | 1.00 | 69.60 | C |
| ATOM | 5344 | NE2 | HIS | C | 5 | 30.662 | 4.328 | 84.887 | 1.00 | 69.60 | N |
| ATOM | 5345 | C | HIS | C | 5 | 32.074 | 3.497 | 79.601 | 1.00 | 45.56 | C |
| ATOM | 5346 | O | HIS | C | 5 | 32.213 | 2.742 | 78.639 | 1.00 | 45.56 | O |
| ATOM | 5347 | N | GLN | C | 6 | 32.789 | 4.585 | 79.817 | 1.00 | 37.64 | N |
| ATOM | 5348 | CA | GLN | C | 6 | 33.855 | 5.012 | 78.949 | 1.00 | 37.64 | C |
| ATOM | 5349 | CB | GLN | C | 6 | 34.413 | 6.305 | 79.473 | 1.00 | 78.39 | C |
| ATOM | 5350 | CG | GLN | C | 6 | 33.530 | 6.908 | 80.496 | 1.00 | 78.39 | C |

| | | | | | | | | | | | |
|---|---|---|---|---|---|---|---|---|---|---|---|
| ATOM | 5351 | CD | GLN | C | 6 | 33.570 | 8.374 | 80.383 | 1.00 | 78.39 | C |
| ATOM | 5352 | OE1 | GLN | C | 6 | 33.864 | 9.067 | 81.342 | 1.00 | 78.39 | O |
| ATOM | 5353 | NE2 | GLN | C | 6 | 33.288 | 8.875 | 79.187 | 1.00 | 78.39 | N |
| ATOM | 5354 | C | GLN | C | 6 | 34.971 | 3.987 | 78.994 | 1.00 | 37.64 | C |
| ATOM | 5355 | O | GLN | C | 6 | 34.877 | 2.935 | 79.651 | 1.00 | 37.64 | O |
| ATOM | 5356 | N | PSE | C | 7 | 36.044 | 4.337 | 78.304 | 1.00 | 49.29 | N |
| ATOM | 5357 | CA | PSE | C | 7 | 37.235 | 3.525 | 78.245 | 1.00 | 49.29 | C |
| ATOM | 5358 | C | PSE | C | 7 | 38.138 | 4.075 | 79.328 | 1.00 | 49.29 | C |
| ATOM | 5359 | O | PSE | C | 7 | 38.642 | 5.191 | 79.213 | 1.00 | 49.29 | O |
| ATOM | 5360 | CB | PSE | C | 7 | 37.926 | 3.784 | 76.961 | 1.00 | 49.58 | C |
| ATOM | 5361 | OG | PSE | C | 7 | 37.318 | 4.887 | 76.430 | 1.00 | 49.58 | O |
| ATOM | 5362 | P | PSE | C | 7 | 37.305 | 4.980 | 74.959 | 1.00 | 49.58 | P |
| ATOM | 5363 | OP1 | PSE | C | 7 | 38.669 | 5.108 | 74.578 | 1.00 | 49.58 | O |
| ATOM | 5364 | OP2 | PSE | C | 7 | 36.541 | 6.280 | 74.575 | 1.00 | 49.58 | O |
| ATOM | 5365 | OP3 | PSE | C | 7 | 36.666 | 3.833 | 74.421 | 1.00 | 49.58 | O |
| ATOM | 5366 | N | GLU | C | 8 | 38.303 | 3.303 | 80.393 | 1.00 | 65.98 | N |
| ATOM | 5367 | CA | GLU | C | 8 | 39.148 | 3.698 | 81.510 | 1.00 | 65.98 | C |
| ATOM | 5368 | CB | GLU | C | 8 | 39.369 | 2.518 | 82.439 | 1.00 | 71.72 | C |
| ATOM | 5369 | CG | GLU | C | 8 | 38.530 | 1.321 | 82.109 | 1.00 | 71.72 | C |
| ATOM | 5370 | CD | GLU | C | 8 | 38.811 | 0.185 | 83.056 | 1.00 | 71.72 | C |
| ATOM | 5371 | OE1 | GLU | C | 8 | 39.102 | 0.458 | 84.241 | 1.00 | 71.72 | O |
| ATOM | 5372 | OE2 | GLU | C | 8 | 38.736 | -0.984 | 82.633 | 1.00 | 71.72 | O |
| ATOM | 5373 | C | GLU | C | 8 | 40.498 | 4.143 | 80.996 | 1.00 | 65.98 | C |
| ATOM | 5374 | O | GLU | C | 8 | 41.032 | 3.565 | 80.050 | 1.00 | 65.98 | O |
| ATOM | 5375 | N | ASP | C | 9 | 41.059 | 5.164 | 81.620 | 1.00 | 60.49 | N |
| ATOM | 5376 | CA | ASP | C | 9 | 42.355 | 5.618 | 81.190 | 1.00 | 60.49 | C |
| ATOM | 5377 | CB | ASP | C | 9 | 42.432 | 7.144 | 81.327 | 1.00 | 71.12 | C |
| ATOM | 5378 | CG | ASP | C | 9 | 43.300 | 7.596 | 82.463 | 1.00 | 71.12 | C |
| ATOM | 5379 | OD1 | ASP | C | 9 | 44.534 | 7.511 | 82.314 | 1.00 | 71.12 | O |
| ATOM | 5380 | OD2 | ASP | C | 9 | 42.755 | 8.040 | 83.498 | 1.00 | 71.12 | O |
| ATOM | 5381 | C | ASP | C | 9 | 43.340 | 4.885 | 82.084 | 1.00 | 60.49 | C |
| ATOM | 5382 | O | ASP | C | 9 | 43.012 | 4.534 | 83.217 | 1.00 | 60.49 | O |
| ATOM | 5383 | N | GLU | C | 10 | 44.516 | 4.583 | 81.554 | 1.00 | 98.32 | N |
| ATOM | 5384 | CA | GLU | C | 10 | 45.541 | 3.900 | 82.334 | 1.00 | 98.32 | C |
| ATOM | 5385 | CB | GLU | C | 10 | 45.231 | 2.393 | 82.485 | 1.00 | 89.01 | C |
| ATOM | 5386 | CG | GLU | C | 10 | 44.029 | 1.891 | 81.695 | 1.00 | 89.01 | C |
| ATOM | 5387 | CD | GLU | C | 10 | 44.419 | 1.155 | 80.433 | 1.00 | 89.01 | C |
| ATOM | 5388 | OE1 | GLU | C | 10 | 44.536 | -0.088 | 80.477 | 1.00 | 89.01 | O |
| ATOM | 5389 | OE2 | GLU | C | 10 | 44.617 | 1.819 | 79.397 | 1.00 | 89.01 | O |
| ATOM | 5390 | C | GLU | C | 10 | 46.877 | 4.093 | 81.639 | 1.00 | 98.32 | C |
| ATOM | 5391 | O | GLU | C | 10 | 46.877 | 4.216 | 80.397 | 1.00 | 98.32 | O |
| ATOM | 5392 | OXT | GLU | C | 10 | 47.907 | 4.116 | 82.342 | 1.00 | 89.01 | O |
| TER | 5393 | | GLU | C | 10 | | | | | | |
| ATOM | 5394 | CB | HIS | D | 1 | 12.411 | 6.182 | 23.767 | 1.00 | 81.17 | C |
| ATOM | 5395 | CG | HIS | D | 1 | 11.804 | 5.342 | 24.857 | 1.00 | 81.17 | C |
| ATOM | 5396 | CD2 | HIS | D | 1 | 10.558 | 4.855 | 25.011 | 1.00 | 81.17 | C |
| ATOM | 5397 | ND1 | HIS | D | 1 | 12.550 | 4.815 | 25.897 | 1.00 | 81.17 | N |
| ATOM | 5398 | CE1 | HIS | D | 1 | 11.783 | 4.030 | 26.633 | 1.00 | 81.17 | C |
| ATOM | 5399 | NE2 | HIS | D | 1 | 10.572 | 4.032 | 26.120 | 1.00 | 81.17 | N |
| ATOM | 5400 | C | HIS | D | 1 | 14.322 | 4.890 | 22.695 | 1.00 | 72.25 | C |
| ATOM | 5401 | O | HIS | D | 1 | 14.566 | 3.714 | 22.994 | 1.00 | 72.25 | O |
| ATOM | 5402 | N | HIS | D | 1 | 11.969 | 4.220 | 22.307 | 1.00 | 72.25 | N |
| ATOM | 5403 | CA | HIS | D | 1 | 12.880 | 5.376 | 22.534 | 1.00 | 72.25 | C |
| ATOM | 5404 | N | SER | D | 2 | 15.274 | 5.796 | 22.477 | 1.00 | 66.23 | N |
| ATOM | 5405 | CA | SER | D | 2 | 16.700 | 5.465 | 22.584 | 1.00 | 66.23 | C |
| ATOM | 5406 | CB | SER | D | 2 | 17.535 | 6.446 | 21.781 | 1.00 | 41.95 | C |
| ATOM | 5407 | OG | SER | D | 2 | 18.915 | 6.098 | 21.894 | 1.00 | 41.95 | O |
| ATOM | 5408 | C | SER | D | 2 | 17.297 | 5.381 | 24.001 | 1.00 | 66.23 | C |
| ATOM | 5409 | O | SER | D | 2 | 18.155 | 4.548 | 24.259 | 1.00 | 66.23 | O |
| ATOM | 5410 | N | SER | D | 3 | 16.887 | 6.252 | 24.907 | 1.00 | 45.54 | N |
| ATOM | 5411 | CA | SER | D | 3 | 17.396 | 6.171 | 26.256 | 1.00 | 45.54 | C |
| ATOM | 5412 | CB | SER | D | 3 | 17.983 | 7.514 | 26.665 | 1.00 | 91.16 | C |
| ATOM | 5413 | OG | SER | D | 3 | 19.302 | 7.651 | 26.131 | 1.00 | 91.16 | O |

| | | | | | | | | | | | |
|---|---|---|---|---|---|---|---|---|---|---|---|
| ATOM | 5414 | C | SER | D | 3 | 16.209 | 5.719 | 27.093 | 1.00 | 45.54 | C |
| ATOM | 5415 | O | SER | D | 3 | 15.067 | 6.034 | 26.793 | 1.00 | 45.54 | O |
| ATOM | 5416 | N | PRO | D | 4 | 16.473 | 4.961 | 28.153 | 1.00 | 38.13 | N |
| ATOM | 5417 | CD | PRO | D | 4 | 17.825 | 4.789 | 28.695 | 1.00 | 63.08 | C |
| ATOM | 5418 | CA | PRO | D | 4 | 15.463 | 4.423 | 29.069 | 1.00 | 38.13 | C |
| ATOM | 5419 | CB | PRO | D | 4 | 16.280 | 3.566 | 30.020 | 1.00 | 63.08 | C |
| ATOM | 5420 | CG | PRO | D | 4 | 17.675 | 3.564 | 29.433 | 1.00 | 63.08 | C |
| ATOM | 5421 | C | PRO | D | 4 | 14.669 | 5.443 | 29.848 | 1.00 | 38.13 | C |
| ATOM | 5422 | O | PRO | D | 4 | 15.120 | 6.564 | 30.081 | 1.00 | 38.13 | O |
| ATOM | 5423 | N | HIS | D | 5 | 13.494 | 5.031 | 30.303 | 1.00 | 76.32 | N |
| ATOM | 5424 | CA | HIS | D | 5 | 12.645 | 5.904 | 31.083 | 1.00 | 76.32 | C |
| ATOM | 5425 | CB | HIS | D | 5 | 11.357 | 5.179 | 31.464 | 1.00 | 83.02 | C |
| ATOM | 5426 | CG | HIS | D | 5 | 10.219 | 5.510 | 30.561 | 1.00 | 83.02 | C |
| ATOM | 5427 | CD2 | HIS | D | 5 | 9.628 | 6.687 | 30.278 | 1.00 | 83.02 | C |
| ATOM | 5428 | ND1 | HIS | D | 5 | 9.598 | 4.565 | 29.769 | 1.00 | 83.02 | N |
| ATOM | 5429 | CE1 | HIS | D | 5 | 8.672 | 5.153 | 29.036 | 1.00 | 83.02 | C |
| ATOM | 5430 | NE2 | HIS | D | 5 | 8.668 | 6.438 | 29.321 | 1.00 | 83.02 | N |
| ATOM | 5431 | C | HIS | D | 5 | 13.353 | 6.371 | 32.310 | 1.00 | 76.32 | C |
| ATOM | 5432 | O | HIS | D | 5 | 14.538 | 6.074 | 32.558 | 1.00 | 76.32 | O |
| ATOM | 5433 | N | GLN | D | 6 | 12.620 | 7.161 | 33.066 | 1.00 | 69.29 | N |
| ATOM | 5434 | CA | GLN | D | 6 | 13.162 | 7.659 | 34.313 | 1.00 | 69.29 | C |
| ATOM | 5435 | CB | GLN | D | 6 | 12.814 | 9.108 | 34.522 | 1.00 | 73.76 | C |
| ATOM | 5436 | CG | GLN | D | 6 | 11.517 | 9.429 | 33.954 | 1.00 | 73.76 | C |
| ATOM | 5437 | CD | GLN | D | 6 | 11.712 | 9.889 | 32.567 | 1.00 | 73.76 | C |
| ATOM | 5438 | OE1 | GLN | D | 6 | 12.578 | 9.382 | 31.856 | 1.00 | 73.76 | O |
| ATOM | 5439 | NE2 | GLN | D | 6 | 10.932 | 10.863 | 32.157 | 1.00 | 73.76 | N |
| ATOM | 5440 | C | GLN | D | 6 | 12.461 | 6.844 | 35.348 | 1.00 | 69.29 | C |
| ATOM | 5441 | O | GLN | D | 6 | 11.784 | 5.790 | 34.919 | 1.00 | 69.29 | O |
| ATOM | 5442 | N | PSE | D | 7 | 12.609 | 7.261 | 36.621 | 1.00 | 46.32 | N |
| ATOM | 5443 | CA | PSE | D | 7 | 11.899 | 6.601 | 37.713 | 1.00 | 46.32 | C |
| ATOM | 5444 | C | PSE | D | 7 | 10.385 | 6.773 | 37.534 | 1.00 | 46.32 | C |
| ATOM | 5445 | O | PSE | D | 7 | 9.901 | 7.751 | 36.949 | 1.00 | 46.32 | O |
| ATOM | 5446 | CB | PSE | D | 7 | 12.013 | 7.328 | 38.898 | 1.00 | 43.95 | C |
| ATOM | 5447 | OG | PSE | D | 7 | 13.381 | 7.076 | 39.045 | 1.00 | 43.95 | O |
| ATOM | 5448 | P | PSE | D | 7 | 14.269 | 8.284 | 39.193 | 1.00 | 43.95 | P |
| ATOM | 5449 | OP1 | PSE | D | 7 | 13.744 | 9.078 | 40.261 | 1.00 | 43.95 | O |
| ATOM | 5450 | OP2 | PSE | D | 7 | 15.649 | 7.569 | 39.563 | 1.00 | 43.95 | O |
| ATOM | 5451 | OP3 | PSE | D | 7 | 14.245 | 9.057 | 37.980 | 1.00 | 43.95 | O |
| ATOM | 5452 | N | GLU | D | 8 | 9.654 | 5.739 | 37.944 | 1.00 | 59.99 | N |
| ATOM | 5453 | CA | GLU | D | 8 | 8.192 | 5.706 | 37.799 | 1.00 | 59.99 | C |
| ATOM | 5454 | CB | GLU | D | 8 | 7.655 | 4.274 | 37.982 | 1.00 | 84.97 | C |
| ATOM | 5455 | CG | GLU | D | 8 | 7.950 | 3.315 | 36.869 | 1.00 | 84.97 | C |
| ATOM | 5456 | CD | GLU | D | 8 | 7.395 | 1.936 | 37.120 | 1.00 | 84.97 | C |
| ATOM | 5457 | OE1 | GLU | D | 8 | 6.997 | 1.653 | 38.270 | 1.00 | 84.97 | O |
| ATOM | 5458 | OE2 | GLU | D | 8 | 7.368 | 1.132 | 36.162 | 1.00 | 84.97 | O |
| ATOM | 5459 | C | GLU | D | 8 | 7.460 | 6.627 | 38.784 | 1.00 | 59.99 | C |
| ATOM | 5460 | O | GLU | D | 8 | 8.057 | 7.063 | 39.767 | 1.00 | 59.99 | O |
| ATOM | 5461 | N | ASP | D | 9 | 6.172 | 6.894 | 38.576 | 1.00 | 98.45 | N |
| ATOM | 5462 | CA | ASP | D | 9 | 5.428 | 7.783 | 39.478 | 1.00 | 98.45 | C |
| ATOM | 5463 | CB | ASP | D | 9 | 4.670 | 8.821 | 38.657 | 1.00103.61 | | C |
| ATOM | 5464 | CG | ASP | D | 9 | 3.978 | 9.863 | 39.512 | 1.00103.61 | | C |
| ATOM | 5465 | OD1 | ASP | D | 9 | 4.208 | 9.846 | 40.730 | 1.00103.61 | | O |
| ATOM | 5466 | OD2 | ASP | D | 9 | 3.215 | 10.712 | 38.984 | 1.00103.61 | | O |
| ATOM | 5467 | C | ASP | D | 9 | 4.439 | 7.016 | 40.348 | 1.00 | 98.45 | C |
| ATOM | 5468 | O | ASP | D | 9 | 4.031 | 7.587 | 41.372 | 1.00 | 98.45 | O |
| ATOM | 5469 | OXT | ASP | D | 9 | 4.039 | 5.885 | 39.990 | 1.00103.61 | | O |
| TER | 5470 | | ASP | D | 9 | | | | | | |
| ATOM | 5471 | C1 | AD2 | J | 1 | 18.557 | 22.858 | 17.579 | 1.00 | 38.95 | C |
| ATOM | 5472 | N2 | AD2 | J | 1 | 18.364 | 21.507 | 17.761 | 1.00 | 45.03 | N |
| ATOM | 5473 | C3 | AD2 | J | 1 | 18.181 | 21.023 | 19.059 | 1.00 | 38.95 | C |
| ATOM | 5474 | C4 | AD2 | J | 1 | 18.182 | 21.918 | 20.283 | 1.00 | 38.95 | C |
| ATOM | 5475 | C5 | AD2 | J | 1 | 18.405 | 23.300 | 19.974 | 1.00 | 38.95 | C |
| ATOM | 5476 | N6 | AD2 | J | 1 | 18.583 | 23.742 | 18.661 | 1.00 | 45.03 | N |

| | | | | | | | | | | | |
|---|---|---|---|---|---|---|---|---|---|---|---|
| ATOM | 5477 | N8 | AD2 | J | 1 | 17.981 | 19.766 | 19.512 | 1.00 | 45.03 | N |
| ATOM | 5478 | C9 | AD2 | J | 1 | 17.849 | 19.863 | 20.864 | 1.00 | 45.03 | C |
| ATOM | 5479 | N10 | AD2 | J | 1 | 17.961 | 21.106 | 21.361 | 1.00 | 45.03 | N |
| ATOM | 5480 | N12 | AD2 | J | 1 | 18.528 | 24.266 | 20.955 | 1.00 | 45.03 | N |
| ATOM | 5481 | C13 | AD2 | J | 1 | 18.253 | 18.582 | 18.644 | 1.00 | 38.95 | C |
| ATOM | 5482 | O14 | AD2 | J | 1 | 17.133 | 17.729 | 18.587 | 1.00 | 45.03 | O |
| ATOM | 5483 | C15 | AD2 | J | 1 | 17.295 | 16.527 | 19.301 | 1.00 | 38.95 | C |
| ATOM | 5484 | C16 | AD2 | J | 1 | 18.733 | 16.547 | 19.788 | 1.00 | 38.95 | C |
| ATOM | 5485 | C17 | AD2 | J | 1 | 19.404 | 17.645 | 19.018 | 1.00 | 38.95 | C |
| ATOM | 5486 | O21 | AD2 | J | 1 | 19.360 | 15.299 | 19.766 | 1.00 | 38.95 | O |
| ATOM | 5487 | C22 | AD2 | J | 1 | 16.217 | 16.464 | 20.361 | 1.00 | 38.95 | C |
| ATOM | 5488 | O1 | AD2 | J | 1 | 19.880 | 17.128 | 17.815 | 1.00 | 45.03 | O |
| ATOM | 5489 | O29 | AD2 | J | 1 | 16.886 | 16.429 | 21.598 | 1.00 | 38.95 | O |
| ATOM | 5490 | P1 | AD2 | J | 1 | 16.190 | 16.367 | 22.988 | 1.00 | 45.03 | P |
| ATOM | 5491 | O33 | AD2 | J | 1 | 14.896 | 15.376 | 22.967 | 1.00 | 38.95 | O |
| ATOM | 5492 | O34 | AD2 | J | 1 | 15.858 | 17.715 | 23.376 | 1.00 | 38.95 | O |
| ATOM | 5493 | O35 | AD2 | J | 1 | 17.399 | 15.765 | 23.760 | 1.00 | 38.95 | O |
| ATOM | 5494 | P2 | AD2 | J | 1 | 14.975 | 13.936 | 23.586 | 1.00 | 45.03 | P |
| ATOM | 5495 | O38 | AD2 | J | 1 | 14.187 | 12.974 | 22.932 | 1.00 | 38.95 | O |
| ATOM | 5496 | O39 | AD2 | J | 1 | 14.804 | 13.710 | 25.130 | 1.00 | 38.95 | O |
| ATOM | 5497 | O40 | AD2 | J | 1 | 16.378 | 13.611 | 23.198 | 1.00 | 38.95 | O |
| TER | 5498 | | AD2 | J | 1 | | | | | | |
| ATOM | 5499 | C1 | AD1 | I | 1 | 16.122 | 19.968 | 81.651 | 1.00 | 60.87 | C |
| ATOM | 5500 | N2 | AD1 | I | 1 | 16.505 | 18.693 | 81.948 | 1.00 | 63.71 | N |
| ATOM | 5501 | C3 | AD1 | I | 1 | 17.842 | 18.304 | 81.666 | 1.00 | 60.87 | C |
| ATOM | 5502 | C4 | AD1 | I | 1 | 18.895 | 19.233 | 81.083 | 1.00 | 60.87 | C |
| ATOM | 5503 | C5 | AD1 | I | 1 | 18.370 | 20.518 | 80.806 | 1.00 | 60.87 | C |
| ATOM | 5504 | N6 | AD1 | I | 1 | 17.039 | 20.874 | 81.097 | 1.00 | 63.71 | N |
| ATOM | 5505 | N8 | AD1 | I | 1 | 18.509 | 17.143 | 81.857 | 1.00 | 63.71 | N |
| ATOM | 5506 | C9 | AD1 | I | 1 | 19.800 | 17.322 | 81.438 | 1.00 | 63.71 | C |
| ATOM | 5507 | N10 | AD1 | I | 1 | 20.072 | 18.540 | 80.964 | 1.00 | 63.71 | N |
| ATOM | 5508 | N12 | AD1 | I | 1 | 19.191 | 21.487 | 80.237 | 1.00 | 63.71 | N |
| ATOM | 5509 | C13 | AD1 | I | 1 | 17.749 | 15.952 | 82.304 | 1.00 | 60.87 | C |
| ATOM | 5510 | O14 | AD1 | I | 1 | 18.415 | 15.153 | 83.257 | 1.00 | 63.71 | O |
| ATOM | 5511 | C15 | AD1 | I | 1 | 18.804 | 13.900 | 82.697 | 1.00 | 60.87 | C |
| ATOM | 5512 | C16 | AD1 | I | 1 | 18.077 | 13.729 | 81.344 | 1.00 | 60.87 | C |
| ATOM | 5513 | C17 | AD1 | I | 1 | 17.272 | 15.027 | 81.184 | 1.00 | 60.87 | C |
| ATOM | 5514 | O21 | AD1 | I | 1 | 17.276 | 12.578 | 81.446 | 1.00 | 60.87 | O |
| ATOM | 5515 | C22 | AD1 | I | 1 | 20.340 | 13.759 | 82.719 | 1.00 | 60.87 | C |
| ATOM | 5516 | O1 | AD1 | I | 1 | 15.881 | 14.855 | 81.395 | 1.00 | 63.71 | O |
| ATOM | 5517 | O29 | AD1 | I | 1 | 20.953 | 14.416 | 81.621 | 1.00 | 60.87 | O |
| ATOM | 5518 | P1 | AD1 | I | 1 | 22.462 | 14.183 | 81.300 | 1.00 | 63.71 | P |
| ATOM | 5519 | O33 | AD1 | I | 1 | 23.225 | 13.164 | 82.317 | 1.00 | 60.87 | O |
| ATOM | 5520 | O34 | AD1 | I | 1 | 23.134 | 15.451 | 81.034 | 1.00 | 60.87 | O |
| ATOM | 5521 | O35 | AD1 | I | 1 | 22.272 | 13.380 | 80.016 | 1.00 | 60.87 | O |
| ATOM | 5522 | P2 | AD1 | I | 1 | 23.018 | 11.592 | 82.047 | 1.00 | 63.71 | P |
| ATOM | 5523 | O38 | AD1 | I | 1 | 21.605 | 11.562 | 81.833 | 1.00 | 60.87 | O |
| ATOM | 5524 | O39 | AD1 | I | 1 | 23.271 | 10.718 | 83.281 | 1.00 | 60.87 | O |
| ATOM | 5525 | O40 | AD1 | I | 1 | 23.914 | 10.791 | 80.900 | 1.00 | 60.87 | O |
| TER | 5526 | | AD1 | I | 1 | | | | | | |
| ATOM | 5527 | MG | MG | E | 2 | 18.398 | 13.452 | 23.509 | 1.00 | 28.08 | |
| MG | | | | | | | | | | | |
| TER | 5528 | | MG | E | 2 | | | | | | |
| ATOM | 5529 | MG | MG | F | 3 | 21.459 | 11.040 | 79.674 | 1.00 | 46.01 | |
| MG | | | | | | | | | | | |
| TER | 5530 | | MG | F | 3 | | | | | | |
| ATOM | 5531 | O | HOH | W | 1 | 19.003 | 14.328 | 31.052 | 1.00 | 20.17 | O |
| ATOM | 5532 | O | HOH | W | 2 | 33.835 | 9.074 | 76.724 | 1.00 | 19.08 | O |
| ATOM | 5533 | O | HOH | W | 3 | 29.271 | 31.315 | 41.929 | 1.00 | 23.44 | O |
| ATOM | 5534 | O | HOH | W | 4 | 11.735 | 28.180 | 93.014 | 1.00 | 35.46 | O |
| ATOM | 5535 | O | HOH | W | 5 | 15.794 | -9.663 | 76.193 | 1.00 | 29.59 | O |
| ATOM | 5536 | O | HOH | W | 7 | 15.029 | -10.473 | 73.182 | 1.00 | 22.74 | O |
| ATOM | 5537 | O | HOH | W | 8 | 37.399 | 17.234 | 79.714 | 1.00 | 37.43 | O |

| | | | | | | | | | | | |
|---|---|---|---|---|---|---|---|---|---|---|---|
| ATOM | 5538 | O | HOH | W | 9 | 16.652 | 20.334 | 27.140 | 1.00 | 28.12 | O |
| ATOM | 5539 | O | HOH | W | 10 | 36.304 | 11.294 | 84.101 | 1.00 | 48.22 | O |
| ATOM | 5540 | O | HOH | W | 11 | 27.123 | 4.860 | 56.771 | 1.00 | 13.91 | O |
| ATOM | 5541 | O | HOH | W | 12 | 15.199 | 0.336 | 70.441 | 1.00 | 29.67 | O |
| ATOM | 5542 | O | HOH | W | 13 | 28.430 | 6.028 | 40.727 | 1.00 | 39.66 | O |
| ATOM | 5543 | O | HOH | W | 14 | 4.947 | 23.786 | 18.050 | 1.00 | 26.26 | O |
| ATOM | 5544 | O | HOH | W | 15 | 32.647 | 27.989 | 25.689 | 1.00 | 18.11 | O |
| ATOM | 5545 | O | HOH | W | 16 | 33.710 | 26.403 | 28.185 | 1.00 | 41.53 | O |
| ATOM | 5546 | O | HOH | W | 17 | 36.872 | 18.366 | 41.289 | 1.00 | 39.01 | O |
| ATOM | 5547 | O | HOH | W | 18 | 13.886 | 13.318 | 35.235 | 1.00 | 48.82 | O |
| ATOM | 5548 | O | HOH | W | 19 | 30.998 | 15.417 | 40.405 | 1.00 | 28.44 | O |
| ATOM | 5549 | O | HOH | W | 20 | 23.541 | -3.518 | 55.672 | 1.00 | 34.51 | O |
| ATOM | 5550 | O | HOH | W | 21 | 36.719 | 35.310 | 16.361 | 1.00 | 33.78 | O |
| ATOM | 5551 | O | HOH | W | 22 | 16.917 | 12.168 | 28.199 | 1.00 | 31.20 | O |
| ATOM | 5552 | O | HOH | W | 23 | 19.678 | -1.951 | 69.208 | 1.00 | 30.61 | O |
| ATOM | 5553 | O | HOH | W | 24 | 27.122 | 5.213 | 76.529 | 1.00 | 52.25 | O |
| ATOM | 5554 | O | HOH | W | 25 | 26.095 | 12.525 | 43.440 | 1.00 | 22.76 | O |
| ATOM | 5555 | O | HOH | W | 26 | 24.594 | 15.119 | 45.793 | 1.00 | 29.48 | O |
| ATOM | 5556 | O | HOH | W | 27 | 24.331 | 22.522 | 93.476 | 1.00 | 22.70 | O |
| ATOM | 5557 | O | HOH | W | 28 | 29.790 | 30.372 | 32.816 | 1.00 | 36.83 | O |
| ATOM | 5558 | O | HOH | W | 29 | 9.880 | -12.937 | 58.476 | 1.00 | 32.42 | O |
| ATOM | 5559 | O | HOH | W | 30 | 30.701 | 0.664 | 17.143 | 1.00 | 46.78 | O |
| ATOM | 5560 | O | HOH | W | 31 | 24.073 | 29.316 | 17.706 | 1.00 | 40.34 | O |
| ATOM | 5561 | O | HOH | W | 32 | 9.814 | 18.393 | 33.742 | 1.00 | 35.42 | O |
| ATOM | 5562 | O | HOH | W | 33 | 37.162 | 19.106 | 67.629 | 1.00 | 30.46 | O |
| ATOM | 5563 | O | HOH | W | 34 | 14.054 | 33.888 | 93.035 | 1.00 | 28.61 | O |
| ATOM | 5564 | O | HOH | W | 35 | 25.683 | 24.262 | 71.753 | 1.00 | 40.38 | O |
| ATOM | 5565 | O | HOH | W | 36 | 16.414 | 19.968 | 54.544 | 1.00 | 57.48 | O |
| ATOM | 5566 | O | HOH | W | 37 | 11.487 | 14.595 | 32.470 | 1.00 | 42.52 | O |
| ATOM | 5567 | O | HOH | W | 38 | 33.214 | 11.246 | 38.966 | 1.00 | 46.27 | O |
| ATOM | 5568 | O | HOH | W | 39 | 25.358 | 10.138 | 31.388 | 1.00 | 24.33 | O |
| ATOM | 5569 | O | HOH | W | 40 | 26.113 | 33.092 | 30.825 | 1.00 | 42.45 | O |
| ATOM | 5570 | O | HOH | W | 41 | 14.596 | 27.086 | 89.788 | 1.00 | 36.96 | O |
| ATOM | 5571 | O | HOH | W | 42 | 14.892 | 19.885 | 95.551 | 1.00 | 37.47 | O |
| ATOM | 5572 | O | HOH | W | 43 | 31.900 | 6.761 | 84.936 | 1.00 | 35.38 | O |
| ATOM | 5573 | O | HOH | W | 44 | 18.771 | 27.551 | 30.942 | 1.00 | 22.54 | O |
| ATOM | 5574 | O | HOH | W | 45 | 11.917 | 18.894 | 84.248 | 1.00 | 40.30 | O |
| ATOM | 5575 | O | HOH | W | 46 | 6.986 | 20.428 | 70.209 | 1.00 | 57.28 | O |
| ATOM | 5576 | O | HOH | W | 47 | 24.987 | 2.314 | 68.413 | 1.00 | 28.36 | O |
| ATOM | 5577 | O | HOH | W | 48 | 32.977 | 18.855 | 39.603 | 1.00 | 63.81 | O |
| ATOM | 5578 | O | HOH | W | 49 | 27.160 | 11.510 | 82.090 | 1.00 | 56.37 | O |
| ATOM | 5579 | O | HOH | W | 50 | 28.650 | 5.338 | 7.158 | 1.00 | 43.22 | O |
| ATOM | 5580 | O | HOH | W | 51 | 27.767 | 3.212 | 46.392 | 1.00 | 13.38 | O |
| TER | 5581 | | HOH | W | 51 | | | | | | |

E
N
D

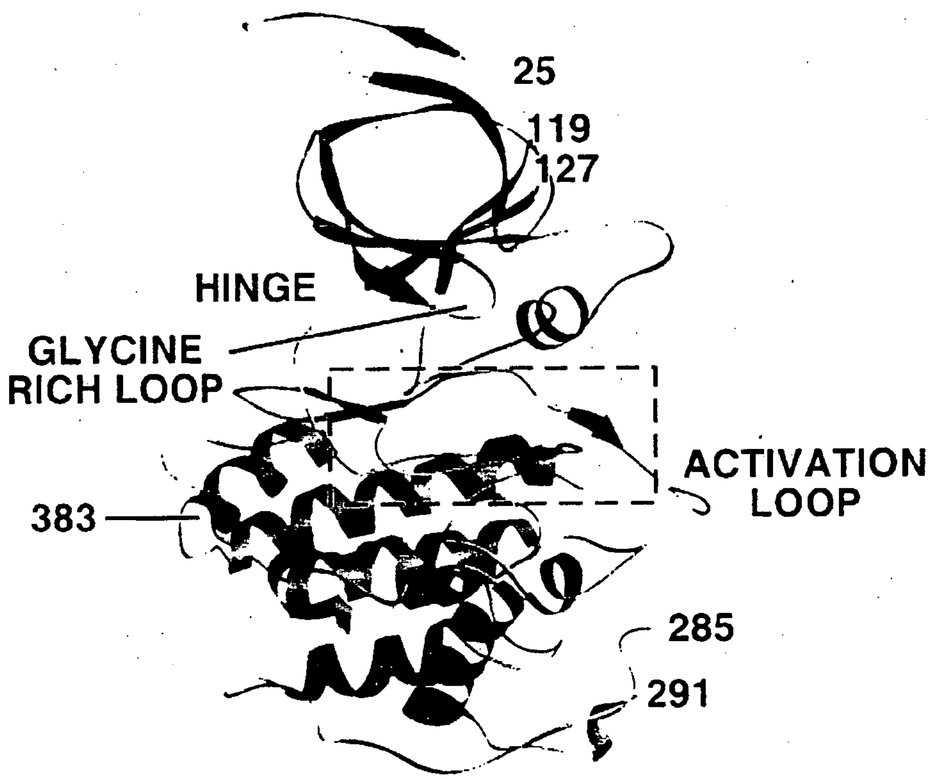
25
119
127
HINGE
GLYCINE
RICH LOOP
ACTIVATION
LOOP
383
285
291

FIG. 8

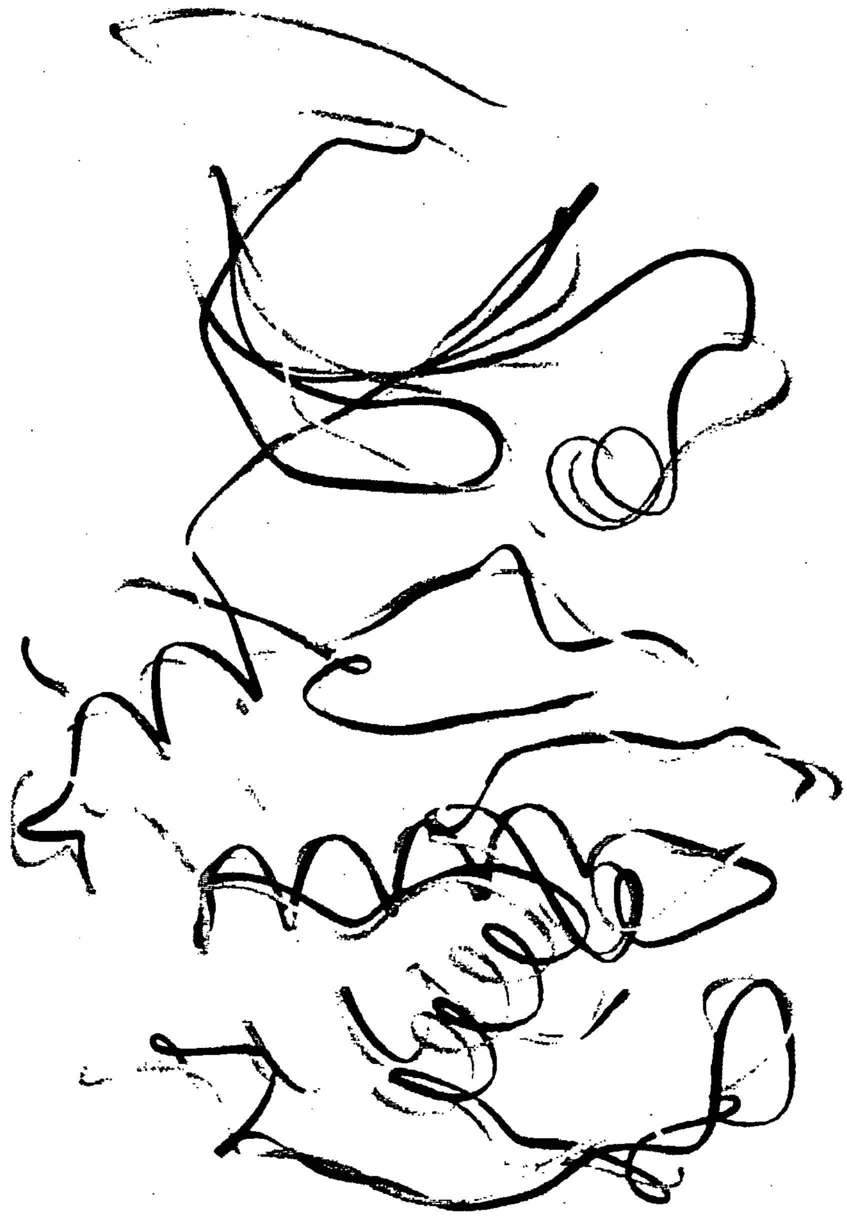

FIG. 9

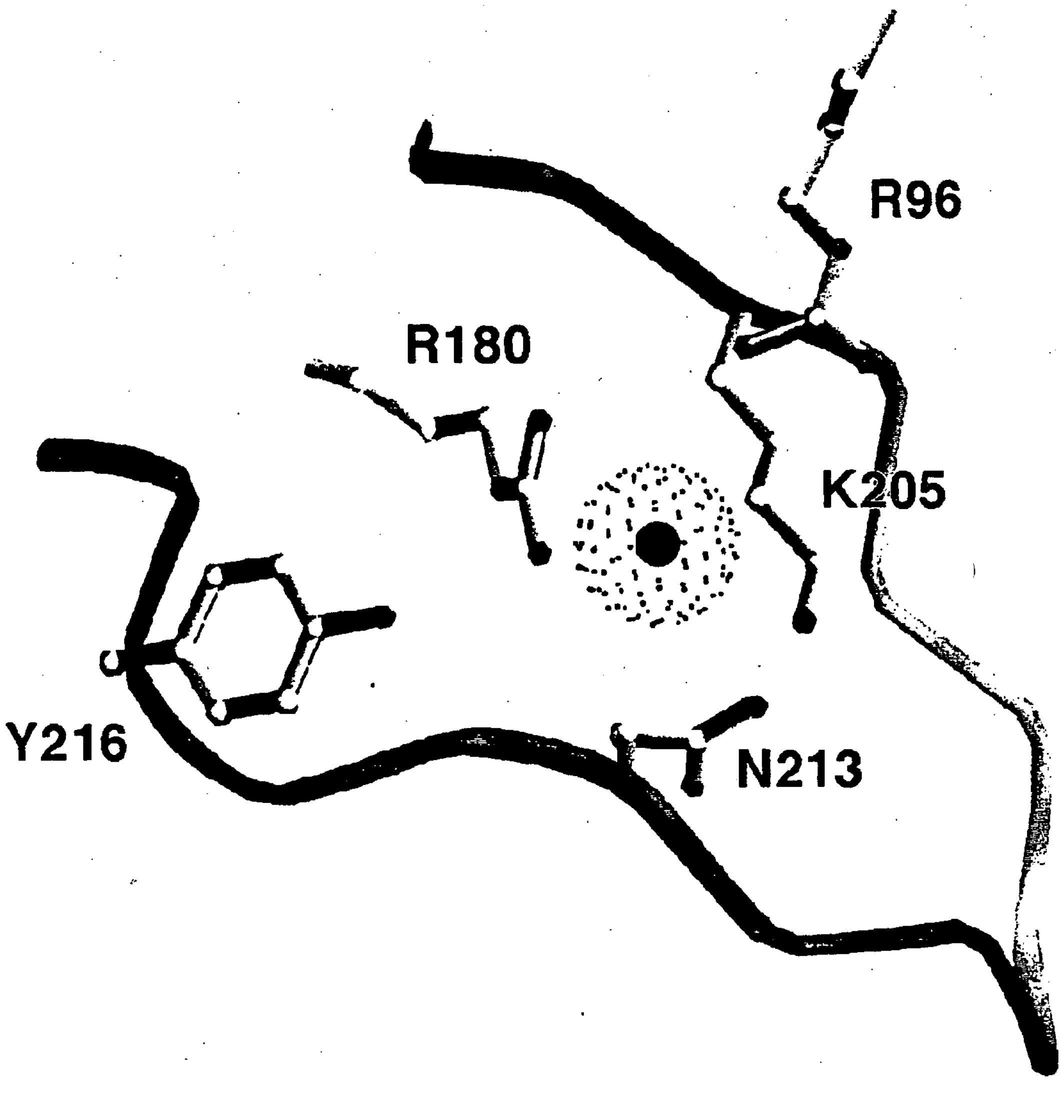


FIG. 10A

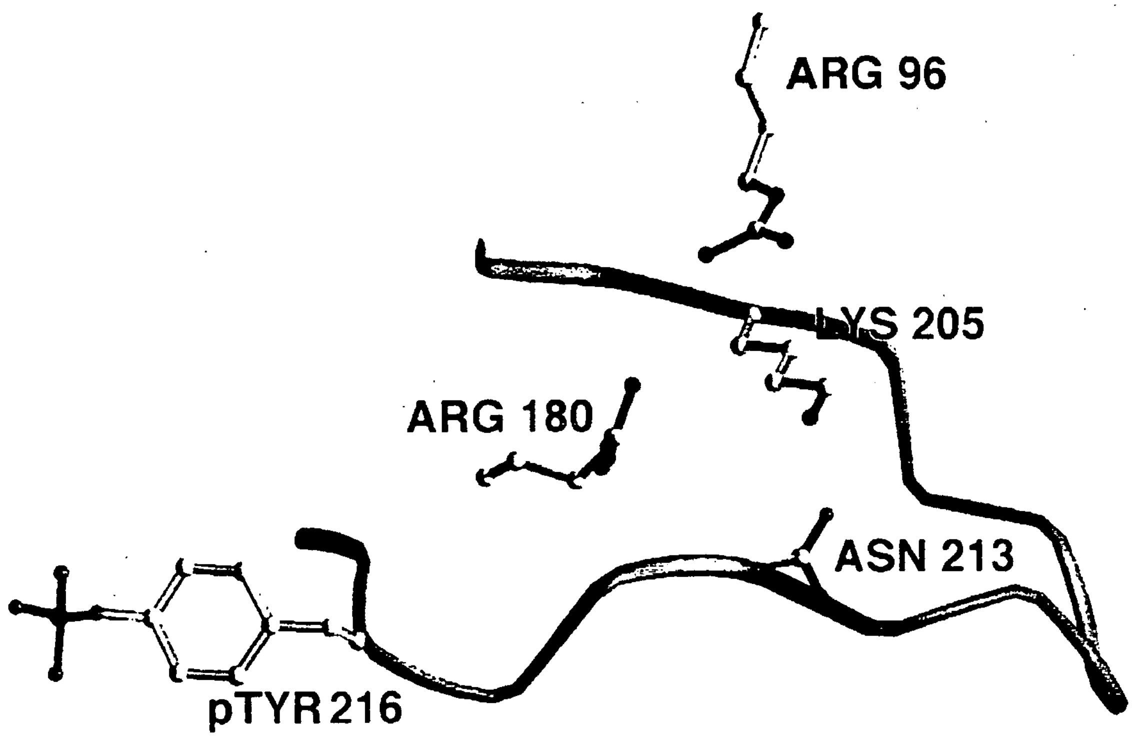


FIG. 10B

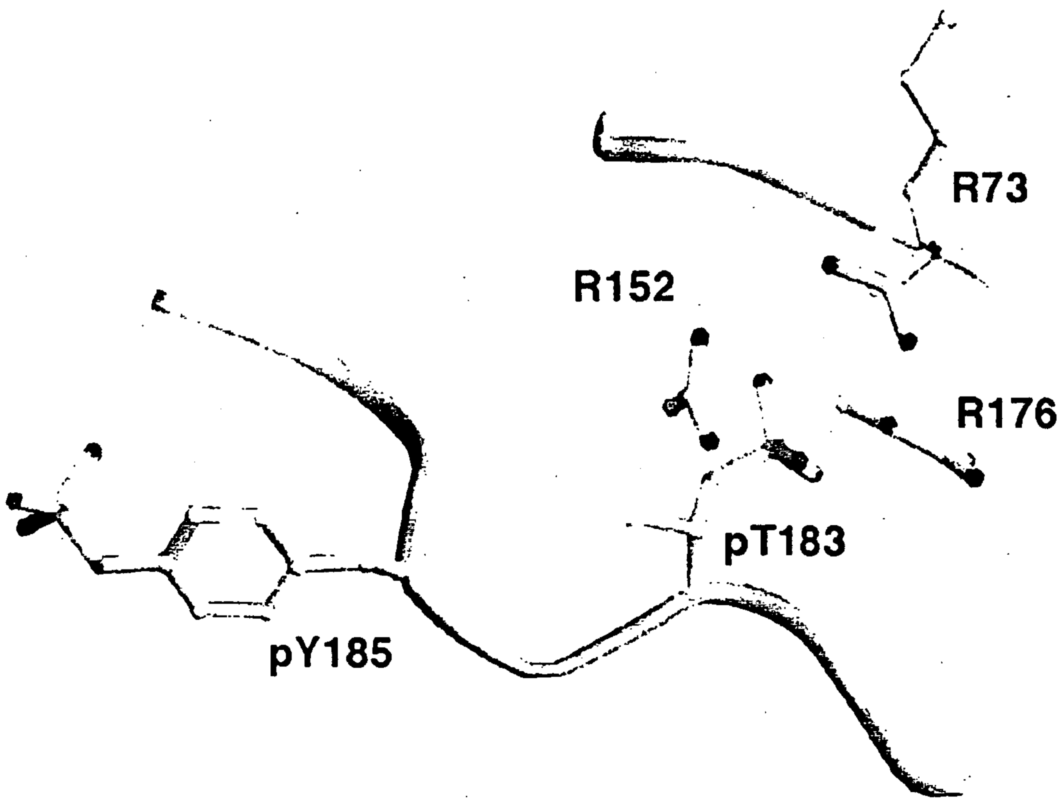


FIG. 10C

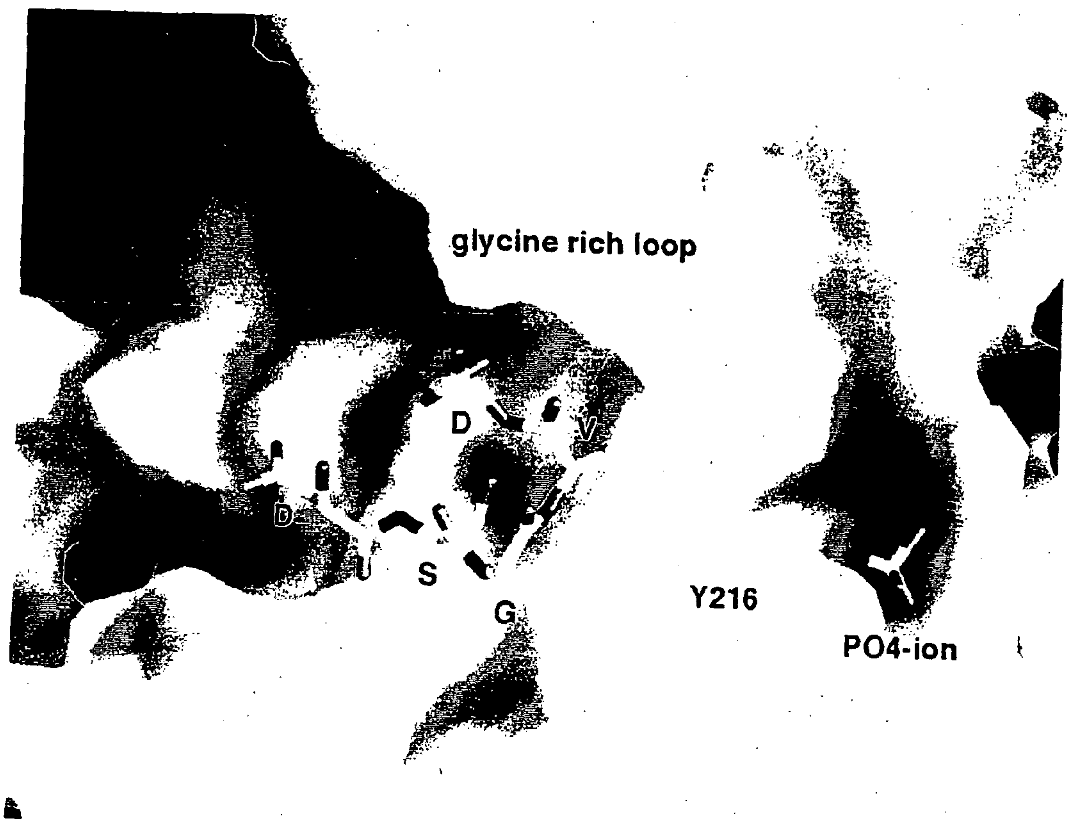


FIG. 11A

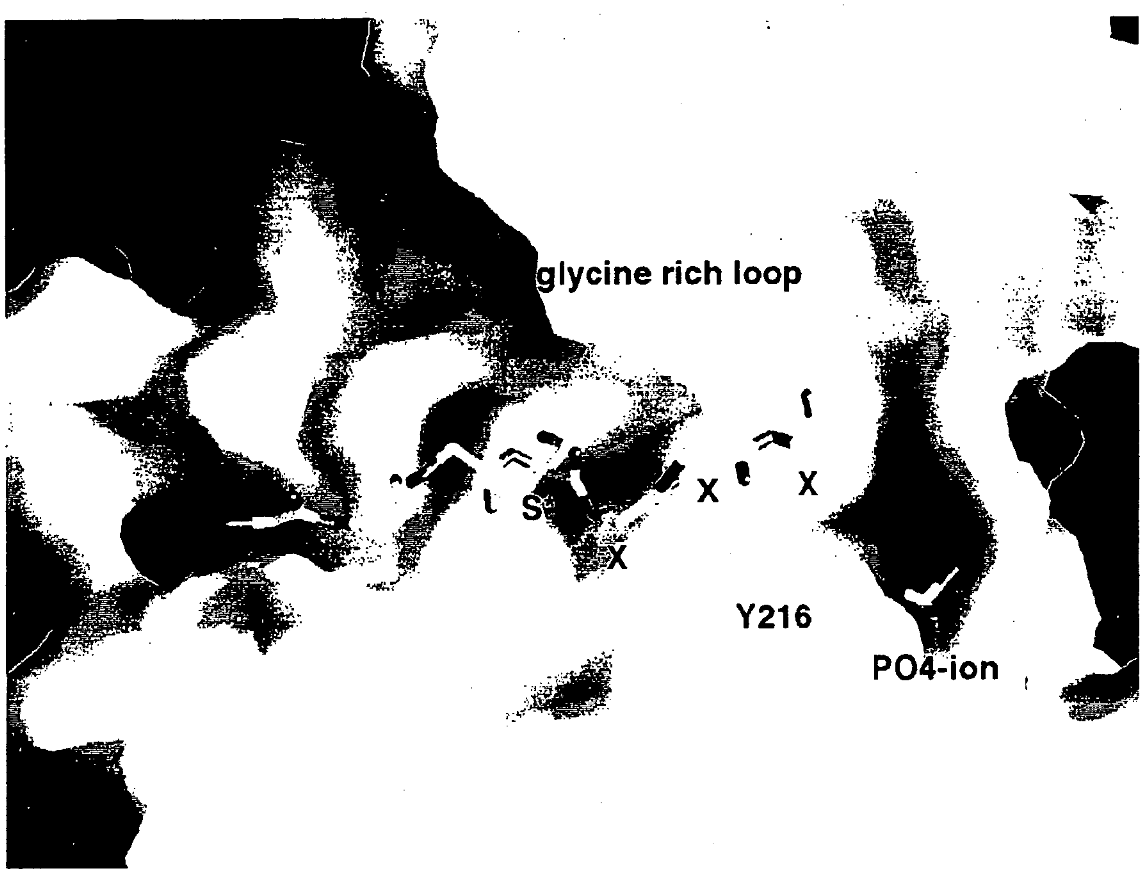


FIG. 11B

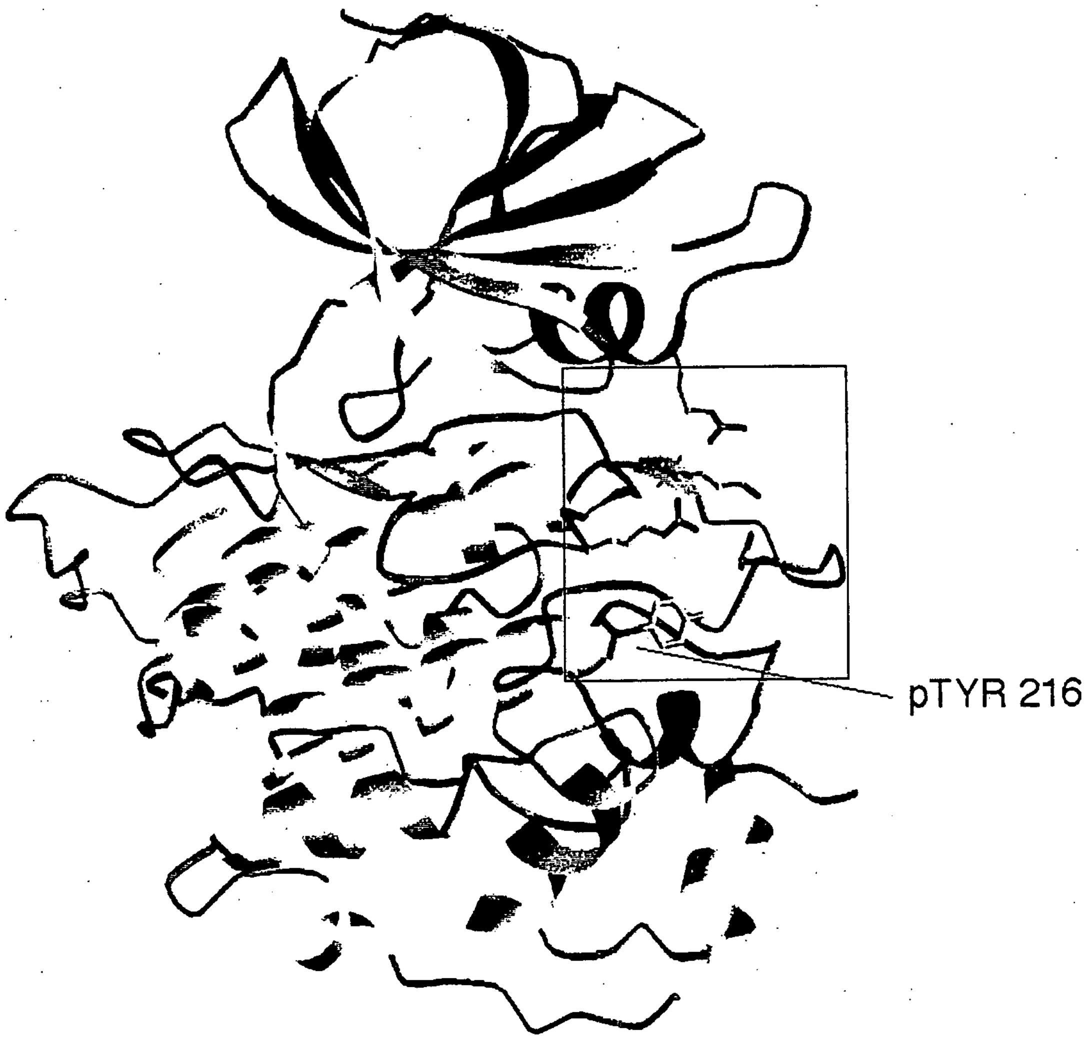


FIG. 12

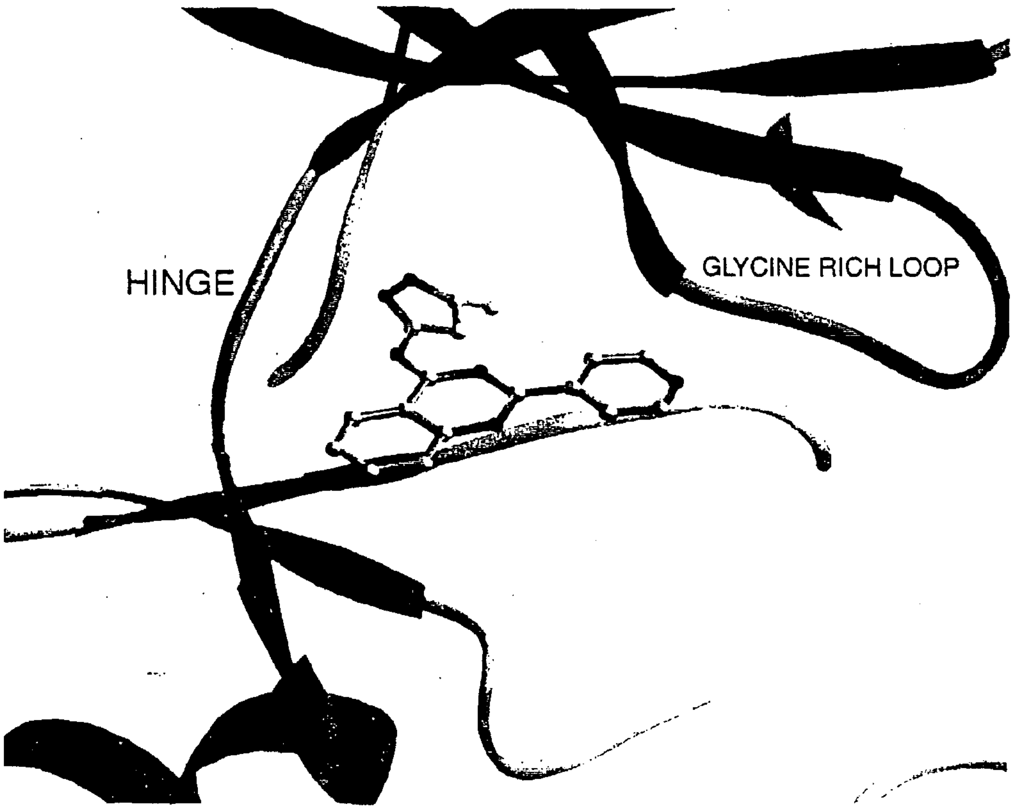


FIG. 13

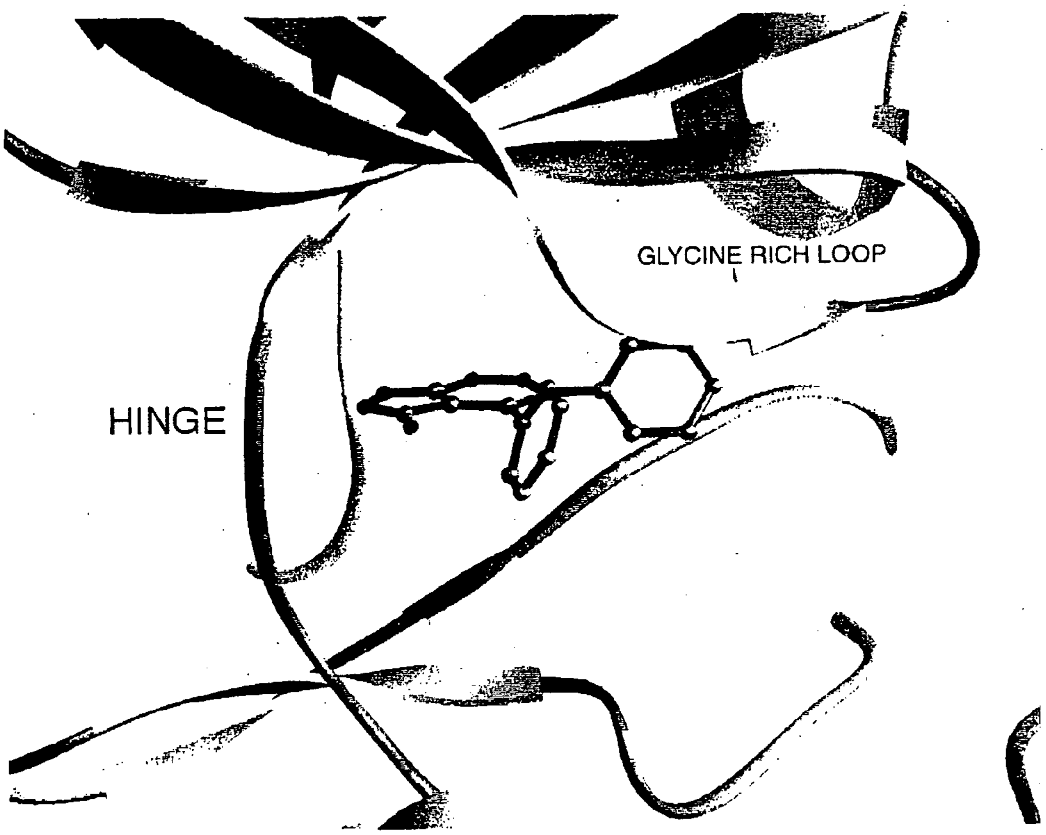


FIG. 14

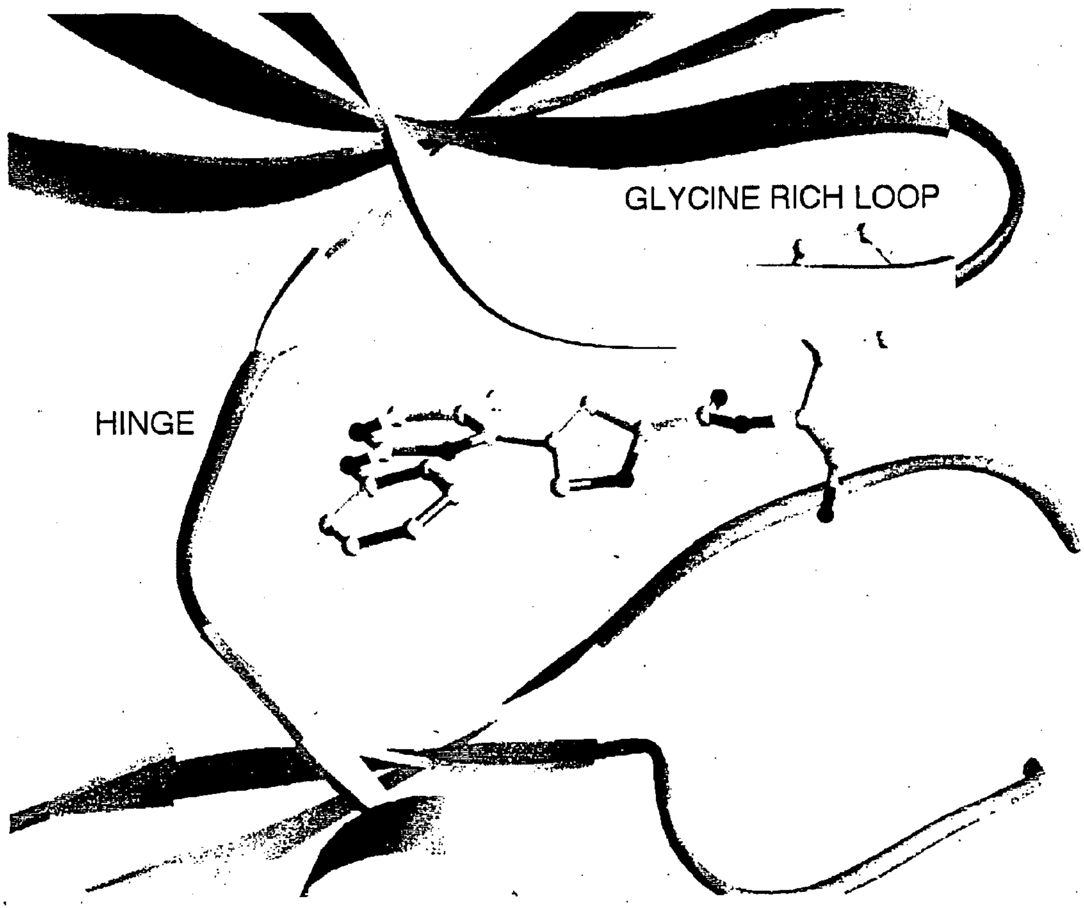


FIG. 15

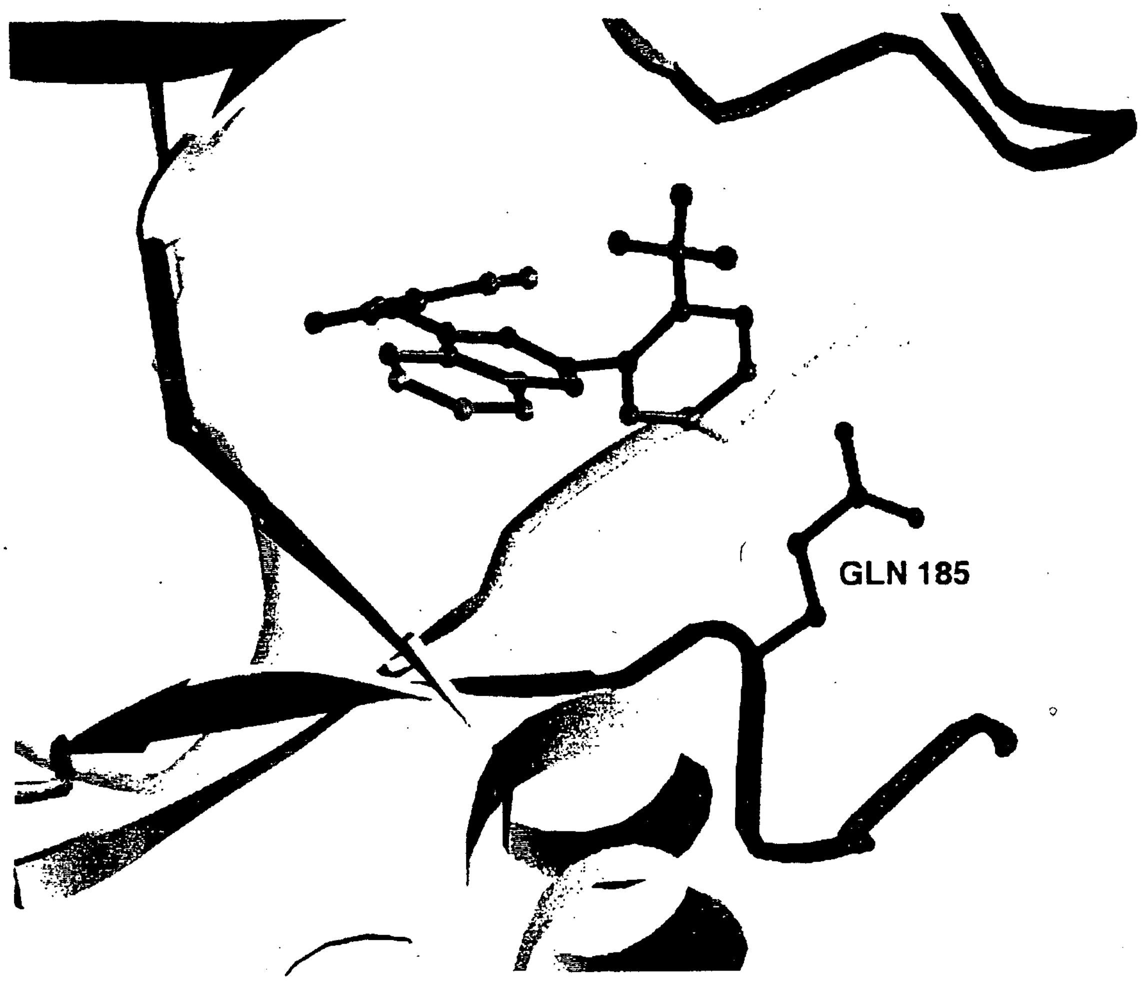


FIG. 16

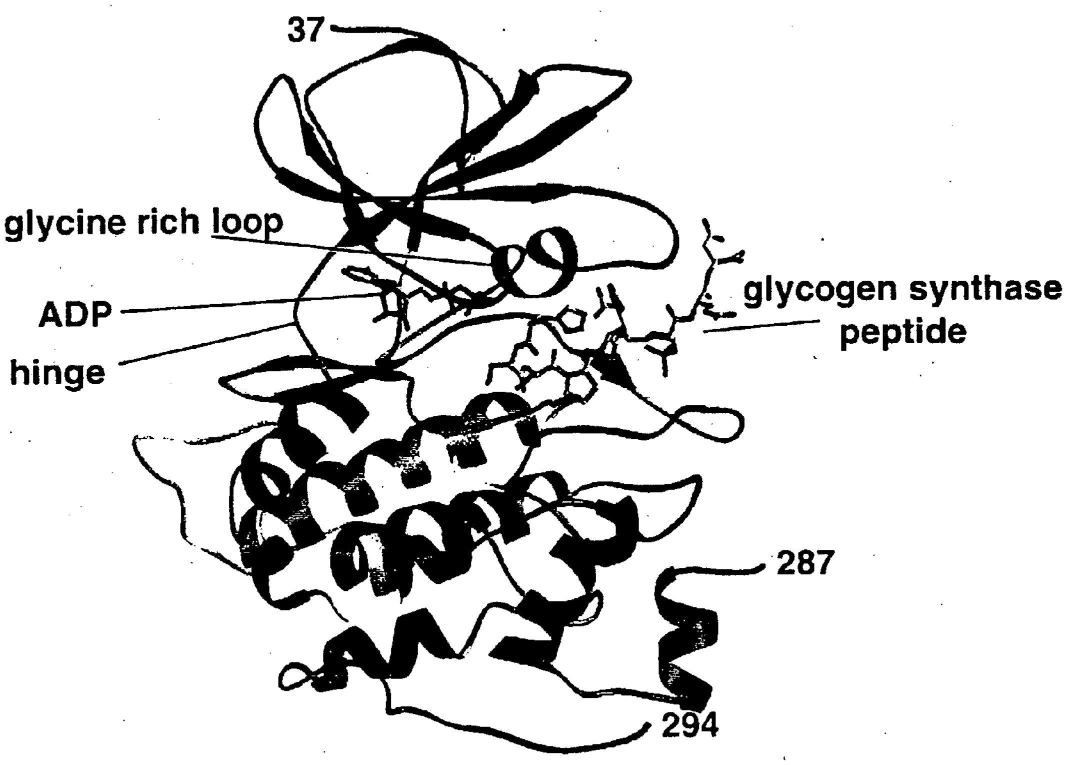
37
glycine rich loop
ADP
hinge
glycogen synthase peptide
287
294

FIG. 17

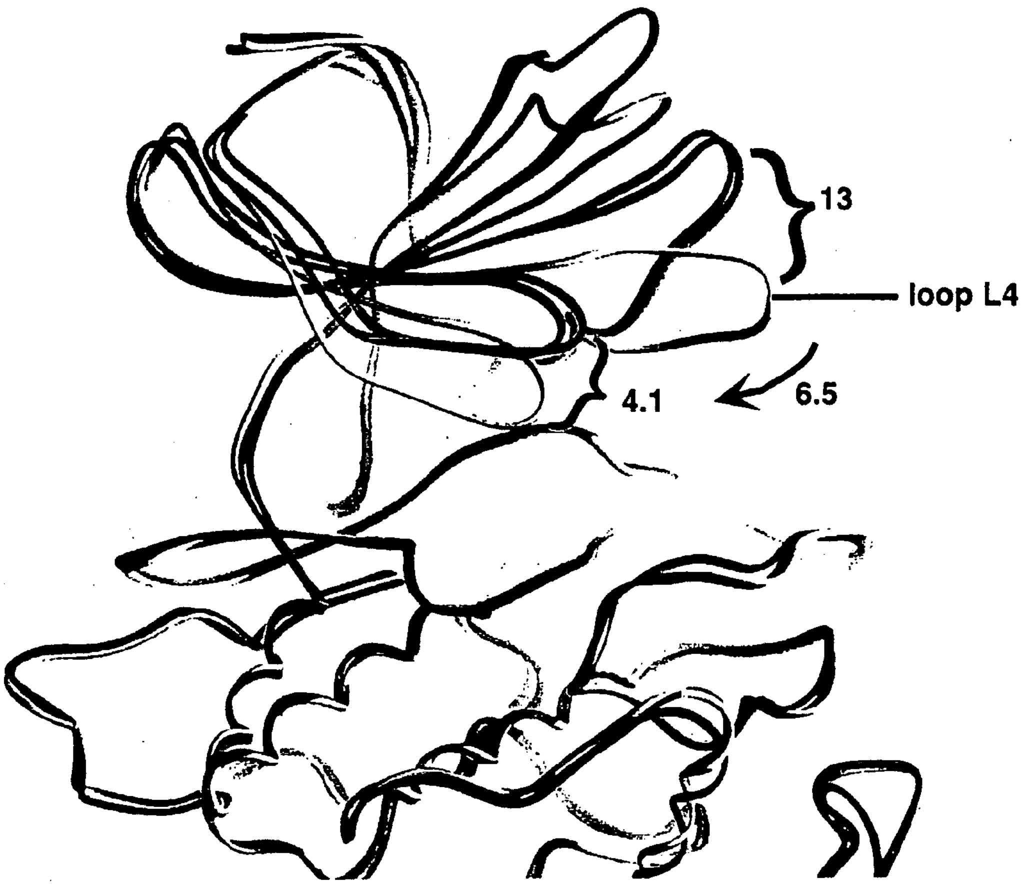


FIG. 18

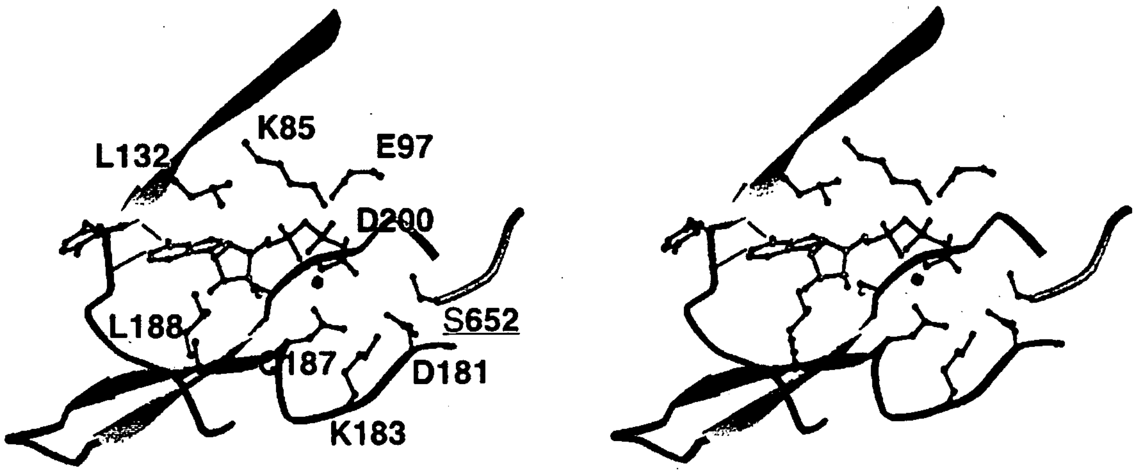


FIG. 19A

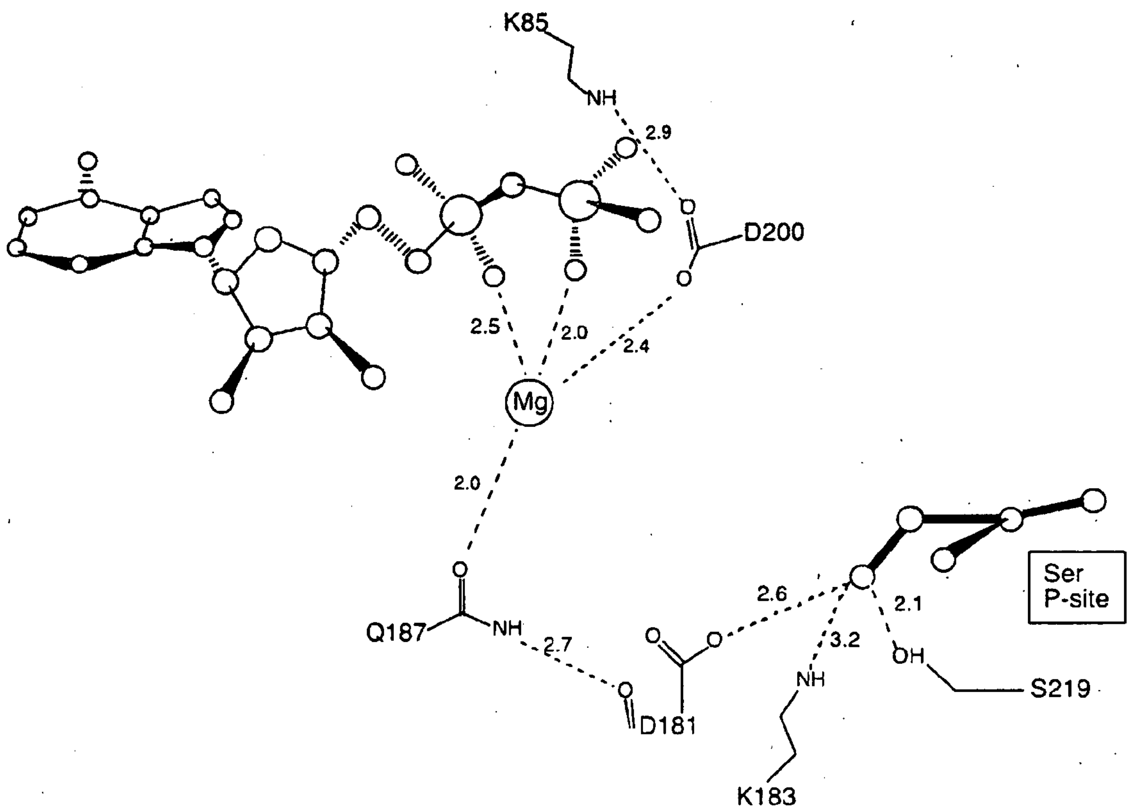

FIG. 19B

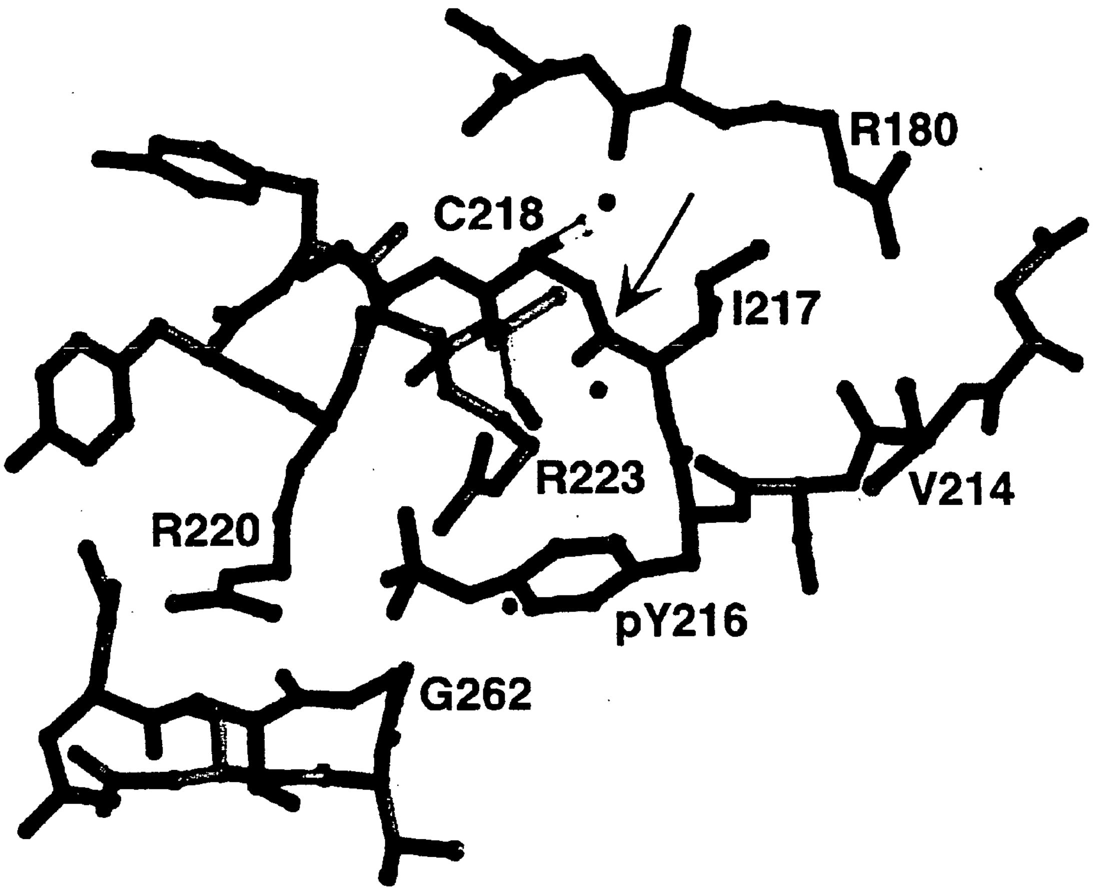


FIG. 20

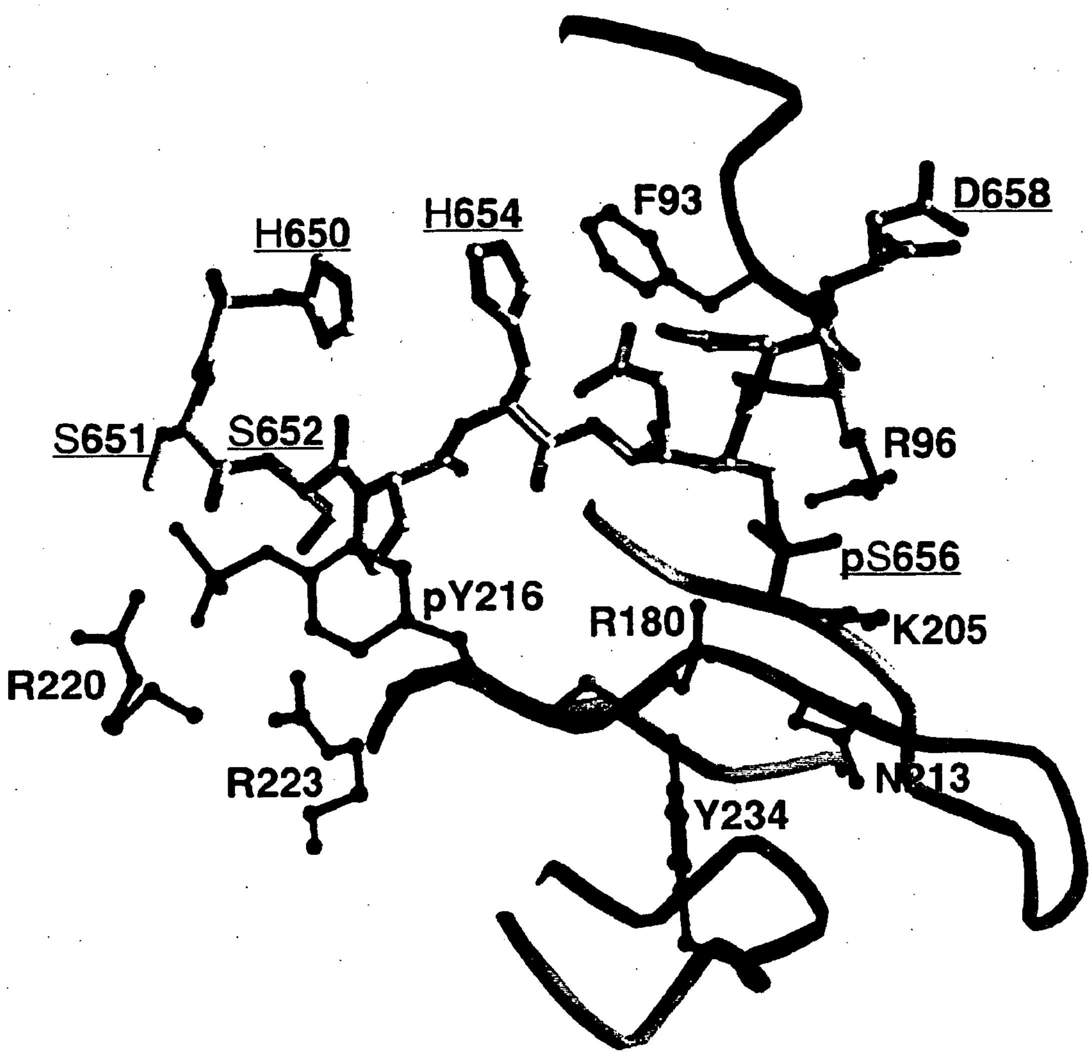
H650
H654
F93
D658
S651
S652
R96
pS656
pY216
R180
K205
R220
R223
N213
Y234

FIG. 21

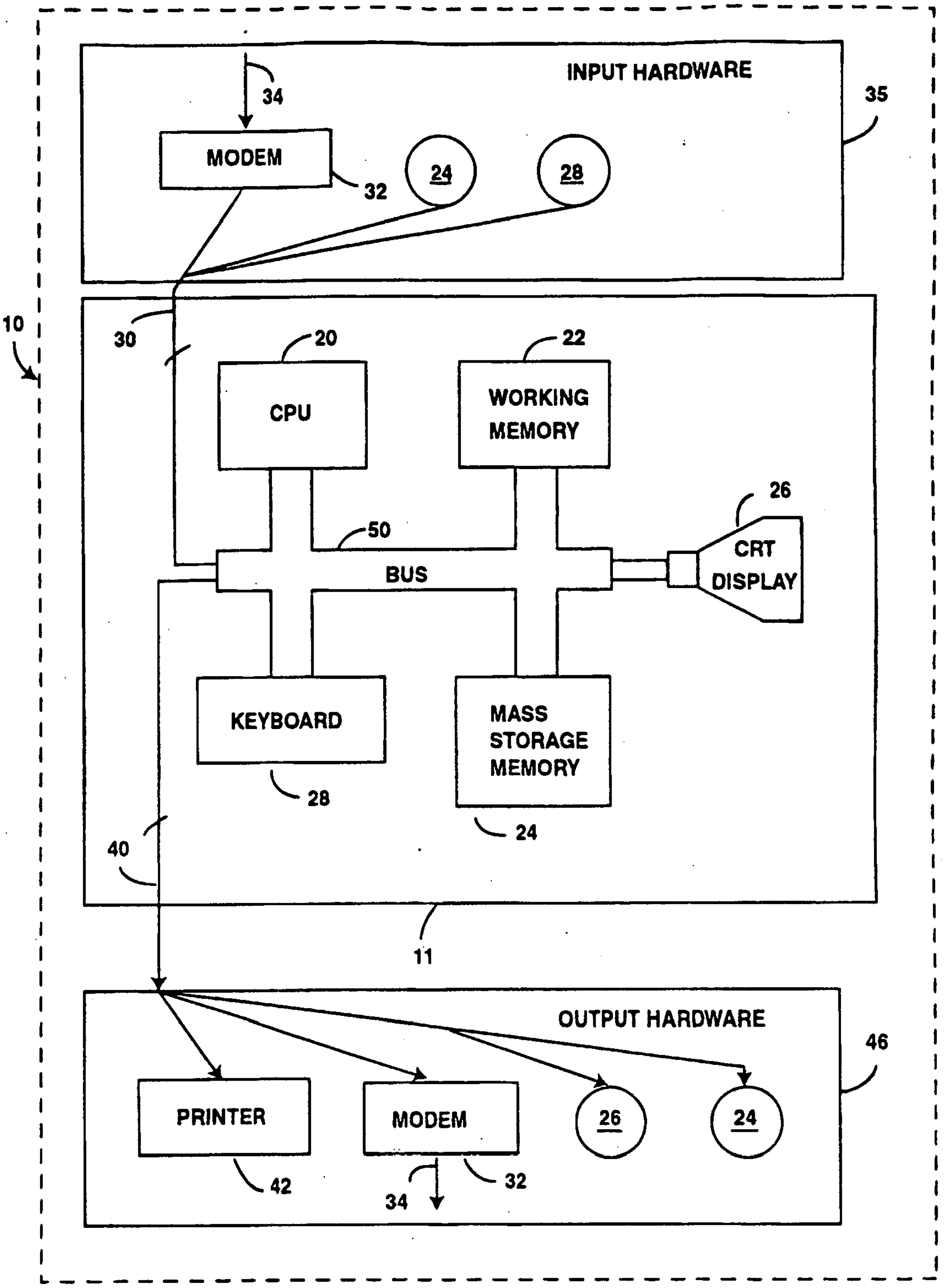
INPUT HARDWARE
34
MODEM
32
24
28
35
10
30
20
CPU
22
WORKING MEMORY
26
50
CRT DISPLAY
BUS
KEYBOARD
28
MASS STORAGE MEMORY
24
40
11
OUTPUT HARDWARE
46
PRINTER
42
MODEM
34
32
26
24

FIG. 22

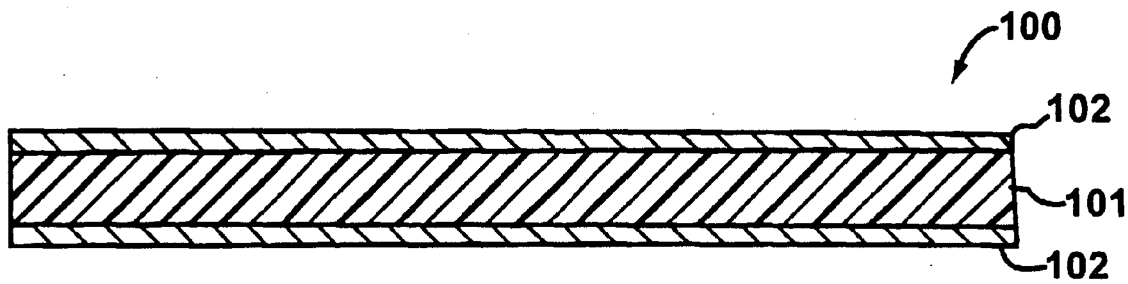


FIG. 23

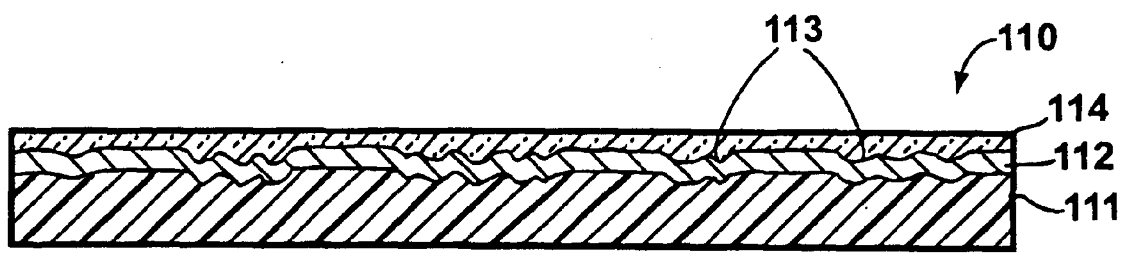


FIG. 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9965897 A
- WO 0038675 A
- WO 0012497 A
- US 4009022 A
- US 4886646 A
- US 5096676 A
- US 5130105 A
- US 5221410 A
- US 5400741 A
- US 60287366 B
- US 60361899 B
- US 60297094 B


### Non-patent literature cited in the description


- **Coghlan et al.** *Chemistry & Biology,* 2000, vol. 7, 793-803
- **Kim ; Kimmel.** *Curr. Opinion Genetics Dev.,* 2000, vol. 10, 508-514
- **Woodgett, J.R.** *EMBO J.,* 1990, vol. 9, 2431-8
- **Hoeflich, K.P. et al.** *Nature,* 2000, vol. 406, 86-90
- **Thomas, G.M. et al.** *FEBS Lett.,* 1999, vol. 458, 247-51
- **Woodgett, J.R. ; P. Cohen.** *Biochim. Biophys. Acta,* 1984, vol. 788, 339-47
- **Kuret, J. et al.** *Eur. J. Biochem.,* 1985, vol. 151, 39-48
- **Haq et al.** *J. Cell Biol.,* 2000, vol. 151, 117
- **Summers, S.A. et al.** *J. Biol. Chem.,* 1999, vol. 274, 17934-40
- **Ross, S.E. et al.** *Mol. Cell. Biol.,* 1999, vol. 19, 8433-41
- **Klein et al.** *PNAS,* 1996, vol. 93, 8455-9
- **Cross et al.** *Biochem. J.,* 1994, vol. 303, 21-26
- **Cohen.** *Biochem. Soc. Trans.,* 1993, vol. 21, 555-567
- **Massillon et al.** *Biochem. J,* 1994, vol. 299, 123-128
- **Lovestone et al.** *Current Biology,* 1994, vol. 4, 1077-86
- **Brownlees et al.** *Neuroreport,* 1997, vol. 8, 3251-55
- **Zhong et al.** *Nature,* 1998, vol. 395, 698-702
- **Takashima et al.** *PNAS,* 1993, vol. 90, 7789-93
- **Pei et al.** *J. Neuropathol. Exp.,* 1997, vol. 56, 70-78
- **Yost, C. et al.** *Cell,* 1998, vol. 93, 1031-41
- **Ding, V.W. et al.** *J. Biol. Chem.,* 2000, vol. 275, 32475-81
- **Waltzer, L. ; M. Bienz.** *Cancer Metastasis Rev.,* 1999, vol. 18, 231-46
- **Ikeda, S. et al.** *EMBO J,* 1998, vol. 17, 1371-84
- **Thomas, G.M. et al.** *FEBS Lett,* 1999, vol. 458, 247-51
- **Salic, A. et al.** *Mol. Cell.,* 2000, vol. 5, 523-32
- **Fang, X. et al.** *Proc. Natl. Acad. Sci. U S A,* 2000, vol. 97, 11960-5
- **Xie et al.** *Structure,* 1998, vol. 6, 983-991
- **Smith ; Waterman.** *Advances in Applied Mathematics,* 1981, vol. 2, 482
- **Dayhoff et al.** *Atlas of Protein Sequence and Structure,* 1998, vol. 5, 345-352
- **El-Gendy, A.M. et al.** *Asian J. Chem.,* 1989, vol. 1, 376
- **Deeb, A. ; Said, S.A.** *Collect. Czech. Chem. Comm.,* 1990, vol. 50, 2795
- **Shalaby, A.A.** *J. Prakt. Chemie,* 1990, vol. 332, 104
- **Hill, D.T. et al.** *J. Het. Chem.,* 1991, vol. 28, 1181
- **Rieke, R.D. et al.** *J. Org. Chem.,* 1991, vol. 56, 1445
- **Fujisowa, T. et al.** *Chem. Lett.,* 1981, 1135
- **Iyoda, M. et al.** *Tet. Lett.,* 1985, vol. 26, 4777
- **Pav et al.** *Proteins: Structure, Function, and Genetics,* 1994, vol. 20, 98-102
- **S.C. Lovell et al.** *Proteins: Structure, Function, and Genetics,* 2000, vol. 40, 389-408
- **Bax, B. et al.** *Structure,* 2001, vol. 9, 1143-1152
- **Davis et al.** *Science,* 2001, vol. 291, 134
- **Cyclin A. Russo et al.** *Nat. Struct. Biol.,* 1996, vol. 3, 696
- **Jones et al.** *Acta Crystallogr.,* 1991, vol. A47, 110-119

- **Carson.** *J. Appl. Crystallogr.,* 1991, vol. 24, 9589-961
- **Gerstein et al.** *J. Mol. Biol.,* 1995, vol. 251, 161-175
- *Acta Crystallogr.,* 1994, vol. D50, 760-763
- **P. J. Goodford.** A Computational Procedure for Determining Energetically Favorable Binding Sites on Biologically Important Macromolecules. *J. Med. Chem.,* 1985, vol. 28, 849-857
- **A. Miranker et al.** Functionality Maps of Binding Sites: A Multiple Copy Simultaneous Search Method. *Proteins: Structure, Function and Genetics,* 1991, vol. 11, 29-34
- **D. S. Goodsell et al.** Automated Docking of Substrates to Proteins by Simulated Annealing. *Proteins: Structure, Function, and Genetics,* 1990, vol. 8, 195-202
- **I. D. Kuntz et al.** A Geometric Approach to Macromolecule-Ligand Interactions. *J. Mol. Biol.,* 1982, vol. 161, 269-288
- CAVEAT: A Program to Facilitate the Structure-Derived Design of Biologically Active Molecules. **P. A. Bartlett et al.** Molecular Recognition in Chemical and Biological Problems. Royal Chem. Soc, vol. 78, 182-196
- **G. Lauri ; P. A. Bartlett.** CAVEAT: a Program to Facilitate the Design of Organic Molecules. *J. Comput. Aided Mol. Des.,* 1994, vol. 8, 51-66
- **Y. C. Martin.** 3D Database Searching in Drug Design. *J. Med. Chem.,* 1992, vol. 35, 2145-2154
- **M. B. Eisen et al.** HOOK: A Program for Finding Novel Molecular Architectures that Satisfy the Chemical and Steric Requirements of a Macromolecule Binding Site. *Proteins: Struct., Funct., Genet.,* 1994, vol. 19, 199-221
- **H.-J. Bohm.** The Computer Program LUDI: A New Method for the De Novo Design of Enzyme Inhibitors. *J. Comp. Aid. Molec. Design,* 1992, vol. 6, 61-78
- **Y. Nishibata et al.** *Tetrahedron,* 1991, vol. 47, 8985
- **V. Gillet et al.** SPROUT: A Program for Structure Generation. *J. Comput. Aided Mol. Design,* 1993, vol. 7, 127-153
- **N. C. Cohen et al.** Molecular Modeling Software and Methods for Medicinal Chemistry. *J. Med. Chem.,* 1990, vol. 33, 883-894
- **M. A. Navia ; M. A. Murcko.** The Use of Structural Information in Drug Design. *Current Opinions in Structural Biology,* 1992, vol. 2, 202-210
- A Perspective of Modern Methods in Computer-Aided Drug Design. **L. M. Balbes et al.** Reviews in Computational Chemistry. VCH, 1994, vol. 5, 337-380
- **W. C. Guida.** Software For Structure-Based Drug Design. *Curr. Opin. Struct. Biology,* 1994, vol. 4, 777-781
- **E. C. Meng et al.** *J. Comp. Chem.,* 1992, vol. 13, 505-524
- **E. Lattman.** Use of the Rotation and Translation Functions. *Meth. Enzymol.,* 1985, vol. 115, 55-77
- The Molecular Replacement Method. Int. Sci. Rev. Ser. Gordon & Breach, 1972
- Meth. Enzymol. Academic Press, 1985, vol. 114, 115
- **Otwinowski et al.** *Methods Enzymol,* 1989, vol. 180, 51-62
- Collaborative Computational Project, N. *Acta Cryst.,* 1994, vol. D50, 760-763
- **Lawrie, A.M. et al.** *Nat. Struct. Biol.,* 1997, vol. 4, 796-801
- **Navaza.** *J. Acta. Cryst.,* 1994, vol. 50, 157-163
- **Hanks, S.K. et al.** *Science,* 1988, vol. 241, 42-52
- **Hanks, S.K. ; A.M. Quinn.** *Methods Enzymol.,* 1991, vol. 200, 38-62
- **Schulze-Gahmen, U. et al.** *Proteins,* 1995, vol. 22, 378-91
- **Bellon, S. et al.** *Structure Fold Des,* 1999, vol. 7, 1057-65
- **Brown, N.R. et al.** *Nat. Cell. Biol.,* 1999, vol. 1, 438-443
- **Canagarajah, B.J. et al.** *Cell,* 1997, vol. 90, 859-69
- **Hanks, S.K. ; Quinn, A.M.** *Methods Enzymol.,* 1991, vol. 200, 38-62
- **Hanks, S.K. ; Hunter, T.** *FASEB J.,* 1995, vol. 9, 576-96
- **Bax, B. et al.** *Structure(Camb),* 2001, vol. 9, 1143-52
- **Madhusudan ; Trafny, E.A. et al.** *Protein Sci,* 1994, vol. 3, 176-87
- **Bellon, S. et al.** *Structure Fold Des.,* 1999, vol. 7, 1057-65
- **Xie, X. et al.** *Structure,* 1998, vol. 6, 983-91
- **Bossemeyer, D. et al.** *EMBO J,* 1993, vol. 12, 849-59
- **Adams, J.A.** *Chem. Rev.,* 2001, vol. 101, 2271-2290
- **Zhang, F. et al.** *Nature,* 1994, vol. 367, 704-11

SEQUENCE LISTING

<110> VERTEX PHARMACEUTICALS, INC.

<120> INHIBITORS OF GSK-3 AND CRYSTAL STRUCTURES OF GSK-3B
PROTEIN AND PROTEIN COMPLEXES

<130> H3033 EP/1 S3

<140>
<141>

<150> 60/361,899
<151> 2002-02-27

<150> 60/297,094
<151> 2001-06-08

<150> 60/287,366
<151> 2001-04-30

<160> 1

<170> PatentIn Ver. 2.1

<210> 1
<211> 420
<212> PRT
<213> Homo sapiens

<400> 1
```
Met Ser Gly Arg Pro Arg Thr Thr Ser Phe Ala Glu Ser Cys Lys Pro
  1               5                  10                  15

Val Gln Gln Pro Ser Ala Phe Gly Ser Met Lys Val Ser Arg Asp Lys
             20                  25                  30

Asp Gly Ser Lys Val Thr Thr Val Val Ala Thr Pro Gly Gln Gly Pro
         35                  40                  45

Asp Arg Pro Gln Glu Val Ser Tyr Thr Asp Thr Lys Val Ile Gly Asn
     50                  55                  60

Gly Ser Phe Gly Val Val Tyr Gln Ala Lys Leu Cys Asp Ser Gly Glu
 65                  70                  75                  80

Leu Val Ala Ile Lys Lys Val Leu Gln Asp Lys Arg Phe Lys Asn Arg
                 85                  90                  95

Glu Leu Gln Ile Met Arg Lys Leu Asp His Cys Asn Ile Val Arg Leu
            100                 105                 110

Arg Tyr Phe Phe Tyr Ser Ser Gly Glu Lys Lys Asp Glu Val Tyr Leu
        115                 120                 125

Asn Leu Val Leu Asp Tyr Val Pro Glu Thr Val Tyr Arg Val Ala Arg
    130                 135                 140
```

His Tyr Ser Arg Ala Lys Gln Thr Leu Pro Val Ile Tyr Val Lys Leu
145 150 155 160

Tyr Met Tyr Gln Leu Phe Arg Ser Leu Ala Tyr Ile His Ser Phe Gly
165 170 175

Ile Cys His Arg Asp Ile Lys Pro Gln Asn Leu Leu Leu Asp Pro Asp
180 185 190

Thr Ala Val Leu Lys Leu Cys Asp Phe Gly Ser Ala Lys Gln Leu Val
195 200 205

Arg Gly Glu Pro Asn Val Ser Tyr Ile Cys Ser Arg Tyr Tyr Arg Ala
210 215 220

Pro Glu Leu Ile Phe Gly Ala Thr Asp Tyr Thr Ser Ser Ile Asp Val
225 230 235 240

Trp Ser Ala Gly Cys Val Leu Ala Glu Leu Leu Leu Gly Gln Pro Ile
245 250 255

Phe Pro Gly Asp Ser Gly Val Asp Gln Leu Val Glu Ile Ile Lys Val
260 265 270

Leu Gly Thr Pro Thr Arg Glu Gln Ile Arg Glu Met Asn Pro Asn Tyr
275 280 285

Thr Glu Phe Lys Phe Pro Gln Ile Lys Ala His Pro Trp Thr Lys Val
290 295 300

Phe Arg Pro Arg Thr Pro Pro Glu Ala Ile Ala Leu Cys Ser Arg Leu
305 310 315 320

Leu Glu Tyr Thr Pro Thr Ala Arg Leu Thr Pro Leu Glu Ala Cys Ala
325 330 335

His Ser Phe Phe Asp Glu Leu Arg Asp Pro Asn Val Lys Leu Pro Asn
340 345 350

Gly Arg Asp Thr Pro Ala Leu Phe Asn Phe Thr Thr Gln Glu Leu Ser
355 360 365

Ser Asn Pro Pro Leu Ala Thr Ile Leu Ile Pro Pro His Ala Arg Ile
370 375 380

Gln Ala Ala Ala Ser Thr Pro Thr Asn Ala Thr Ala Ala Ser Asp Ala
385 390 395 400

Asn Thr Gly Asp Arg Gly Gln Thr Asn Asn Ala Ala Ser Ala Ser Ala
405 410 415

Ser Asn Ser Thr
420